(19)

Europäisches
Patentamt
European
Patent Office
Office européen
des brevets

(11) **EP 1 752 536 A1**

(12) **EUROPEAN PATENT APPLICATION**
published in accordance with Art. 158(3) EPC

(43) Date of publication:
**14.02.2007  Bulletin 2007/07**

(21) Application number: **05744759.1**

(22) Date of filing: **11.05.2005**

(51) Int Cl.:
*C12N 15/09* (2006.01)     *A61K 31/713* (2006.01)
*A61K 48/00* (2006.01)     *A61P 35/00* (2006.01)
*G06F 17/30* (2006.01)     *G06F 17/40* (2006.01)
*G06F 17/50* (2006.01)

(86) International application number:
**PCT/IB2005/001647**

(87) International publication number:
**WO 2005/116204 (08.12.2005 Gazette 2005/49)**

(84) Designated Contracting States:
**AT BE BG CH CY CZ DE DK EE ES FI FR GB GR
HU IE IS IT LI LT LU MC NL PL PT RO SE SI SK TR**

(30) Priority:  **11.05.2004  JP 2004232811**

(71) Applicant: RNAi Co., Ltd.
**Chiyoda-ku
Tokyo
101-0047 (JP)**

(72) Inventors:
• **NAITO, Yuki
Tokyo 102-0082 (JP)**

• **FUJINO, Masato
Tokyo 113-0024 (JP)**
• **OGUCHI, Shinobu
Tokyo 165-0033 (JP)**
• **NATORI, Yukikazu
Kanagawa 220-0012 (JP)**

(74) Representative: **HOFFMANN EITLE
Patent- und Rechtsanwälte
Arabellastrasse 4
81925 München (DE)**

(54) **POLYNUCLEOTIDE CAUSING RNA INTERFERE AND METHOD OF REGULATING GENE EXPRESSION WITH THE USE OF THE SAME**

(57)    The present invention provides a polynucleotide that not only has a high RNA interference effect on its target gene, but also has a very small risk of causing RNA interference against a gene unrelated to the target gene. A sequence segment conforming to the following rules (a) to (d) is searched from the base sequences of a target gene for RNA interference and, based on the search results, a polynucleotide capable of causing RNAi is designed, synthesized, etc.:

(a) The 3' end base is adenine, thymine, or uracil,
(b) The 5' end base is guanine or cytosine,
(c) A 7-base sequence from the 3' end is rich in one or more types of bases selected from the group consisting of adenine, thymine, and uracil, and
(d) The number of bases is within a range that allows RNA interference to occur without causing cytotoxicity.

EP 1 752 536 A1

**Description**

<u>TECHNICAL FIELD</u>

The present invention relates to polynucleotides for causing RNA interference. Hereinafter, RNA interference may also be referred to as "RNAi."

<u>BACKGROUND ART</u>

RNA interference is a phenomenon of gene destruction wherein double-stranded RNA comprising sense RNA and anti-sense RNA (hereinafter also referred to as "dsRNA") homologous to a specific region of a gene to be functionally inhibited, destructs the target gene by causing interference in the homologous portion of mRNA which is a transcript of the target gene. RNA interference was first proposed in 1998 following an experiment using nematodes. However, in mammals, when long dsRNA with about 30 or more base pairs is introduced into cells, an interferon response is induced, and cell death occurs due to apoptosis. Therefore, it was difficult to apply the RNAi method to mammals.
On the other hand, it was demonstrated that RNA interference could occur in early stage mouse embryos and cultured mammalian cells, and it was found that the induction mechanism of RNA interference also existed in the mammalian cells. At present, it has been demonstrated that short double-stranded RNA with about 21 to 23 base pairs (short interfering RNA, siRNA) can induce RNA interference without exhibiting cytotoxicity even in the mammalian cell system, and it has become possible to apply the RNAi method to mammals.

<u>DISCLOSURE OF THE INVENTION</u>

The RNAi method is a technique which is expected to have various applications. However, while dsRNA or siRNA that is homologous to a specific region of a gene, exhibits an RNA interference effect in most of the sequences in drosophila and nematodes, 70% to 80% of randomly selected (21 base) siRNA do not exhibit an RNA interference effect in mammals. This poses a great problem when gene functional analysis is carried out using the RNAi method in mammals.
Conventional designing of siRNA has greatly depended on the experiences and sensory perceptions of the researcher or the like, and it has been difficult to design siRNA actually exhibiting an RNA interference effect with high probability. Other factors that prevent further research being conducted on RNA interference and its various applications are high costs and time consuming procedures required for carrying out an RNA synthesis resulting in part from the unwanted synthesis of siRNA.
In order to solve the above problems, the present invention aims to provide a polynucleotide capable of effectively acting as siRNA, a method for designing the same, a method for inhibiting gene expression using such a polynucleotide, a pharmaceutical composition comprising such a polynucleotide, and a composition for inhibiting gene expression.

<u>BRIEF DESCRIPTION OF THE DRAWINGS</u>

Figure 1 is a diagram which shows the designing of siRNA corresponding to sequences common to human and mice.
Figure 2 is a diagram which shows the regularity of siRNA exhibiting an RNAi effect.
Figure 3 is a diagram which shows common segments (shown in bold letters) having prescribed sequences in the base sequences of human FBP1 and mouse Fbp1.
Figure 4 is a diagram listing prescribed sequences common to human FBP1 and mouse Fbp1.
Figure 5 is a diagram in which the prescribed sequences common to human FBP1 and mouse Fbp1 are scored.
Figure 6 is a diagram showing the results of BLAST searches on one of the prescribed sequences performed so that genes other than the target are not knocked out.
Figure 7 is a diagram showing the results of BLAST searches on one of the prescribed sequences performed so that genes other than the target are not knocked out.
Figure 8 is a diagram showing an output result of a program.
Figure 9 is a diagram which shows the designing of RNA fragments (a to p).
Figure 10 is a diagram showing the results of testing whether siRNA a to p exhibited an RNAi effect, in which "B" shows the results in drosophila cultured cells, and "C" shows the results in human cultured cells.
Figure 11 is a diagram showing the analysis results concerning the characteristics of sequences of siRNA a to p.
Figure 12 is a principle diagram showing the basic principle of the present invention.
Figure 13 is a block diagram which shows an example of the configuration of a base sequence processing apparatus 100 of the system to which the present invention is applied.

Figure 14 is a diagram which shows an example of information stored in a target gene base sequence file 106a.

Figure 15 is a diagram which shows an example of information stored in a partial base sequence file 106b.

Figure 16 is a diagram which shows an example of information stored in a determination result file 106c.

Figure 17 is a diagram which shows an example of information stored in a prescribed sequence file 106d.

Figure 18 is a diagram which shows an example of information stored in a reference sequence database 106e.

Figure 19 is a diagram which shows an example of information stored in a degree of identity or similarity file 106f.

Figure 20 is a diagram which shows an example of information stored in an evaluation result file 106g.

Figure 21 is a block diagram which shows an example of the structure of a partial base sequence creation part 102a of the system to which the present invention is applied.

Figure 22 is a block diagram which shows an example of the structure of an unrelated gene target evaluation part 102h of the system to which the present invention is applied.

Figure 23 is a flowchart which shows an example of the main processing of the system in the embodiment.

Figure 24 is a flowchart which shows an example of the unrelated gene evaluation process of the system in the embodiment.

Figure 25 is a diagram which shows the structure of a target expression vector pTREC.

Figure 26 is a diagram which shows the results of PCR in which one of the primers in Example 2, 2.(2) is designed such that no intron is inserted.

Figure 27 is a diagram which shows the results of PCR in which one of the primers in Example 2, 2.(2) is designed such that an intron is inserted.

Figure 28 is a diagram which shows the sequence and structure of siRNA; siVIM35.

Figure 29 is a diagram which shows the sequence and structure of siRNA; siVIM812.

Figure 30 is a diagram which shows the sequence and structure of siRNA; siControl.

Figure 31 is a diagram which shows the results of assay of RNAi activity of siVIM812 and siVIM35.

Figure 32 is a diagram which shows RNAi activity of siControl, siVIM812 and siVIM35 against vimentin.

Figure 33 is a diagram which shows the results of antibody staining.

Figure 34 is a diagram which shows the assay results of RNAi activity of siRNA designed by the program against the luciferase gene.

Figure 35 is a diagram which shows the assay results of RNAi activity of siRNA designed by the program against the sequences of SARS virus.

Figure 36 is a diagram which shows the assay results of RNAi activity of siRNA designed by the program against other genes containing sequences with a small number of mismatches to the siRNA.

Figure 37 is a diagram which shows the relationship between apoptosis-related genes and GO_ID in Gene Ontology.

Figure 38 is a diagram which shows the relationship between phosphatase or phosphatase activity-related genes and GO_ID in Gene Ontology.

Figure 39 is a diagram which shows the relationship between cell cycle-related genes and GO_ID in Gene Ontology.

Figure 40 is a diagram which shows the relationship between receptor-related genes and GO_ID in Gene Ontology.

Figure 41 is a diagram which shows the relationship between ion channel-encoding genes and GO_ID in Gene Ontology.

Figure 42 is a diagram which shows the relationship between signal transduction system-related genes and GO_ID in Gene Ontology.

Figure 43 is a diagram which shows the relationship between kinase or kinase activity-related genes and GO_ID in Gene Ontology.

Figure 44 is a diagram which shows the relationship between transcription regulation-related genes and GO_ID in Gene Ontology.

Figure 45 is a diagram which shows the relationship between G protein-coupled receptor (GPCR) or GPCR-related genes and GO_ID in Gene Ontology.

Figure 46 is a list of target sequences to be targeted by the polynucleotides of the present invention, along with their genes, biological function categories, reported biological functions and related diseases.

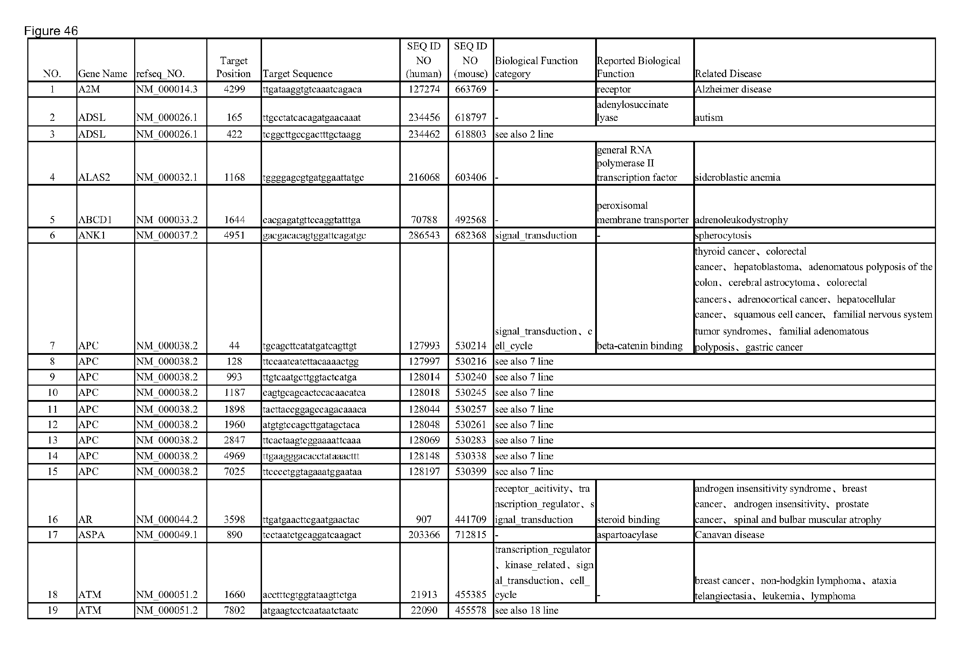

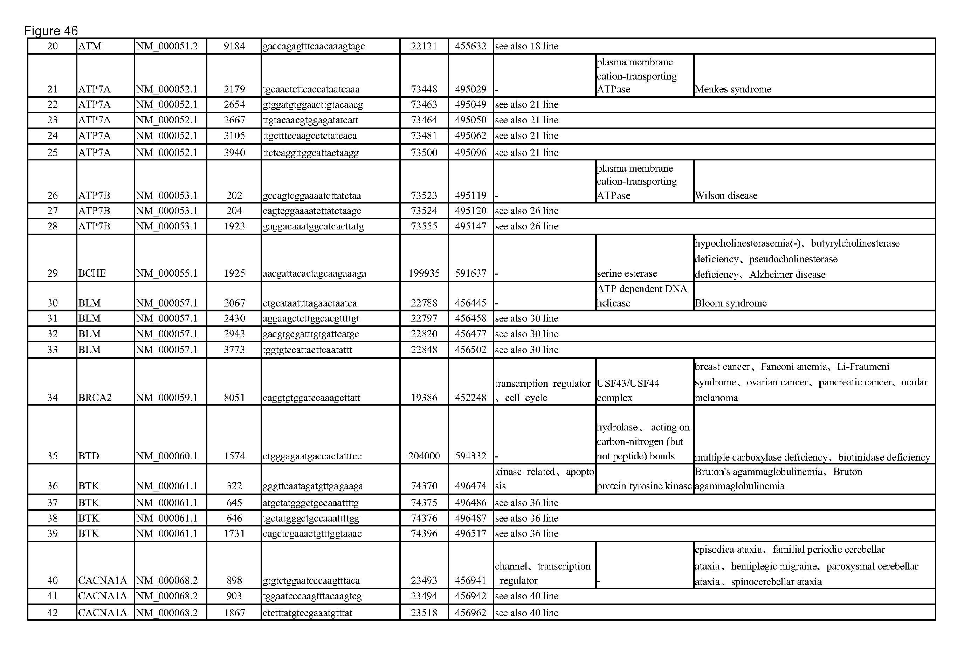

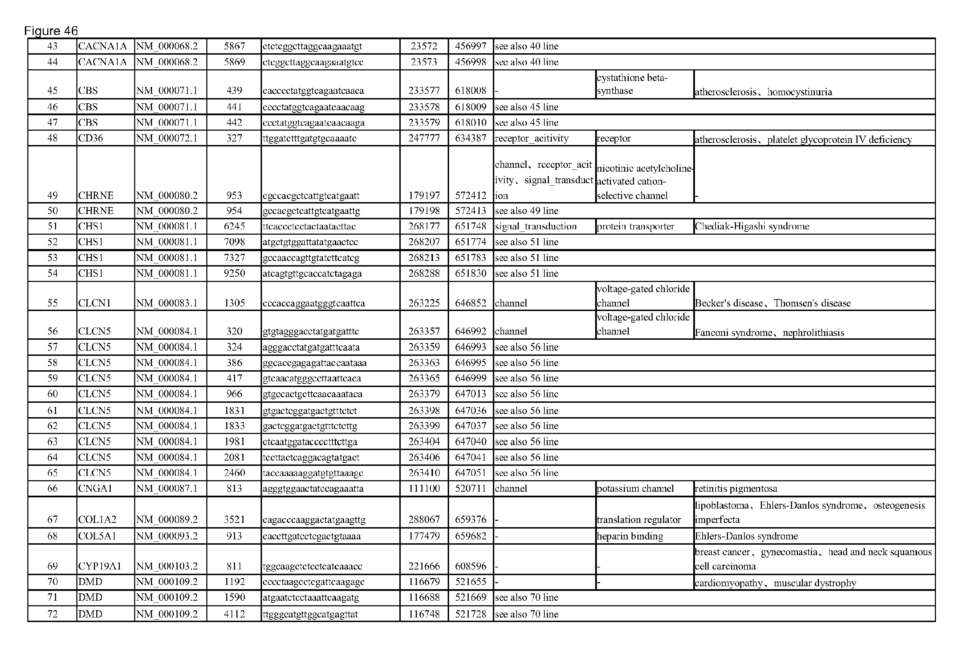

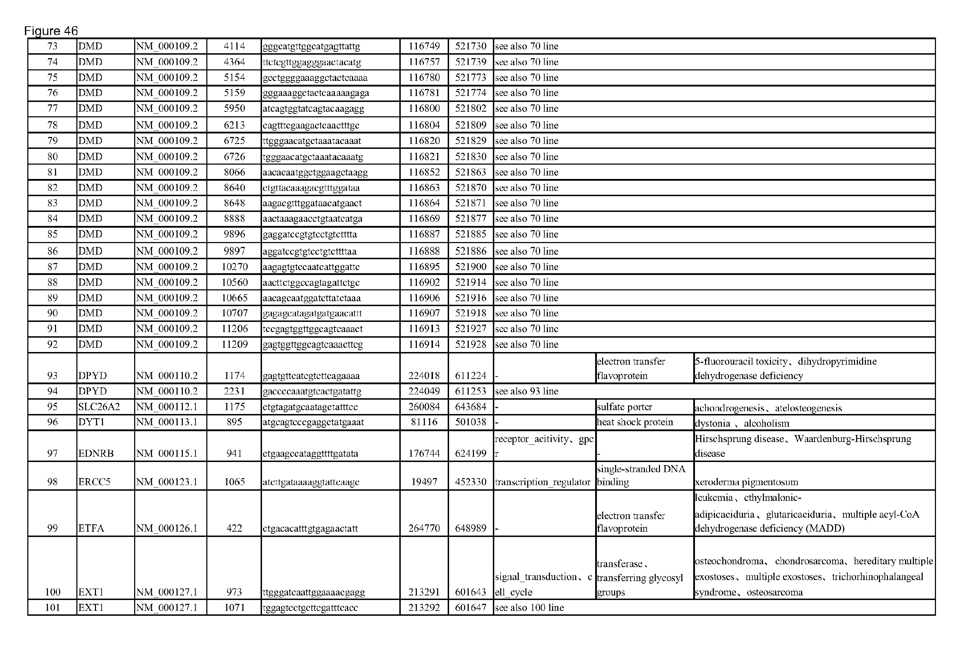

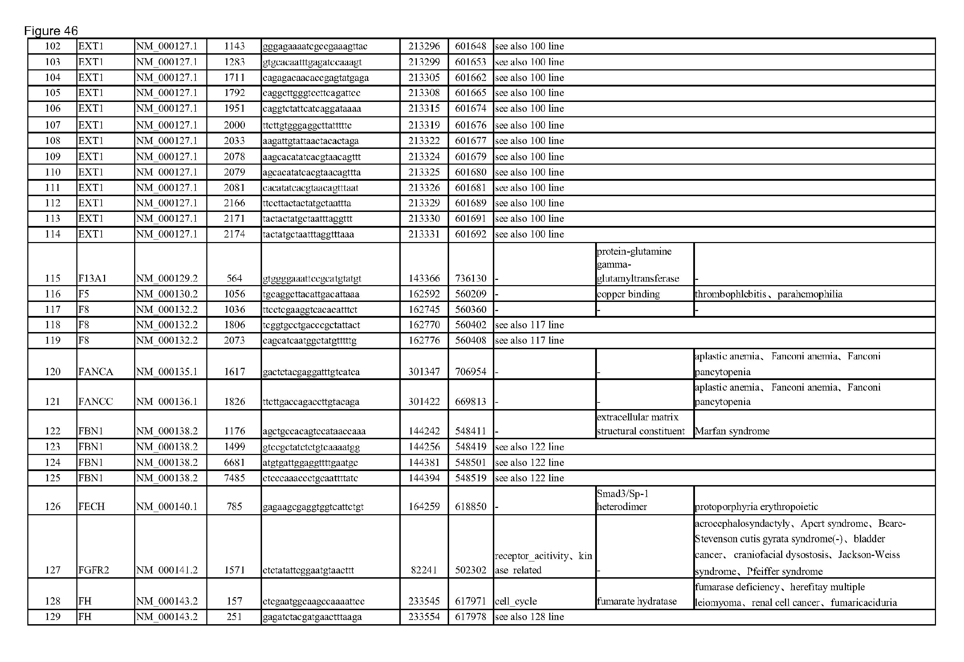

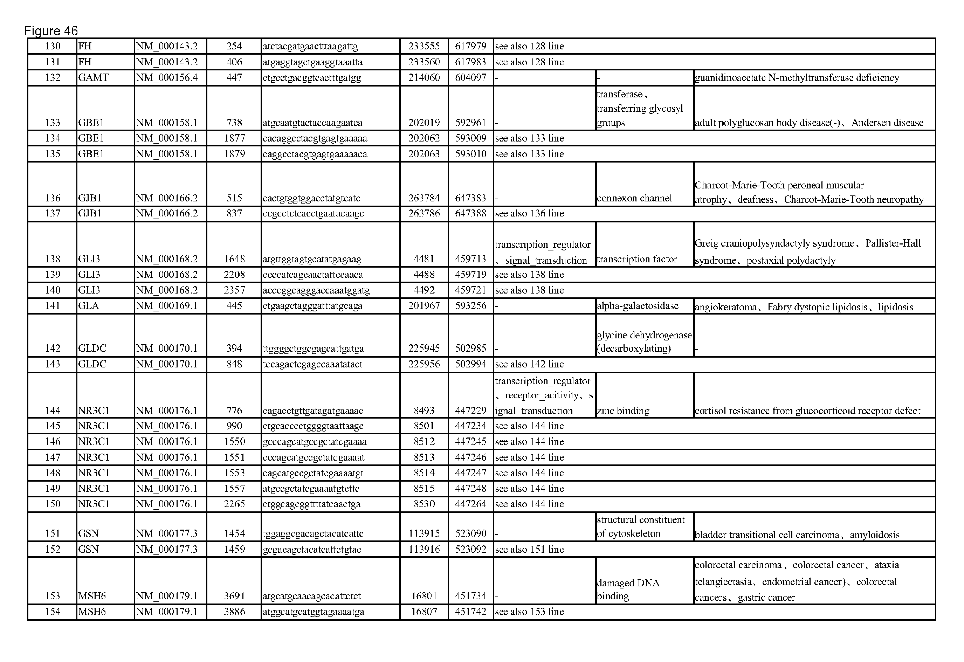

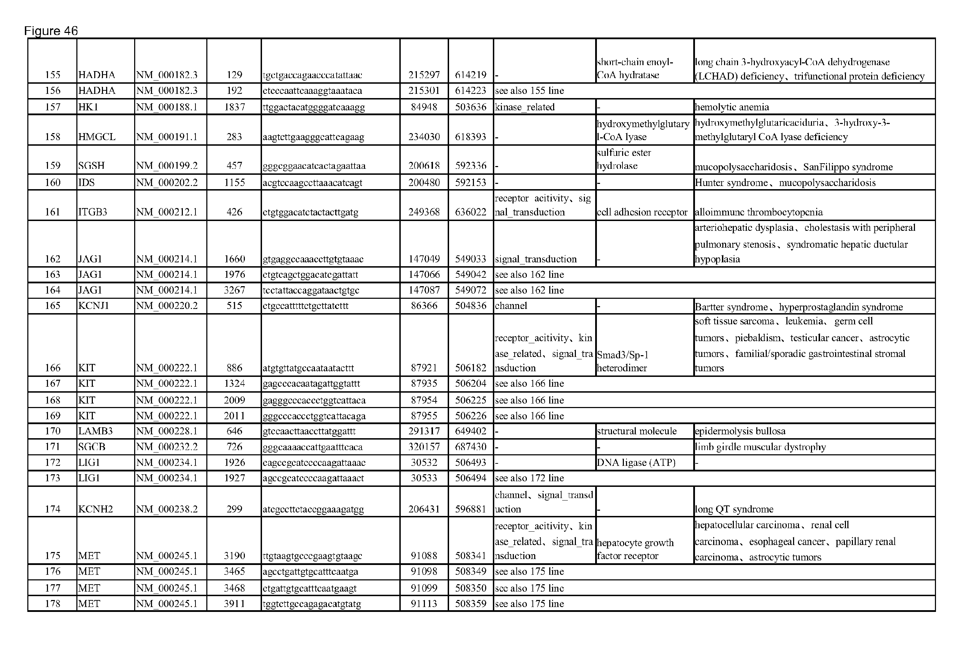

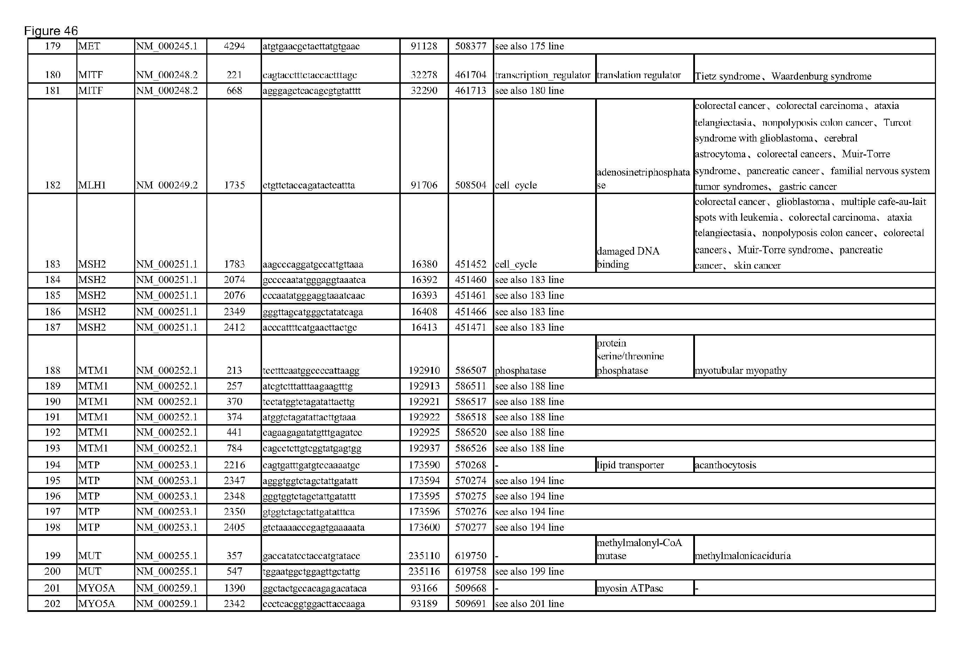

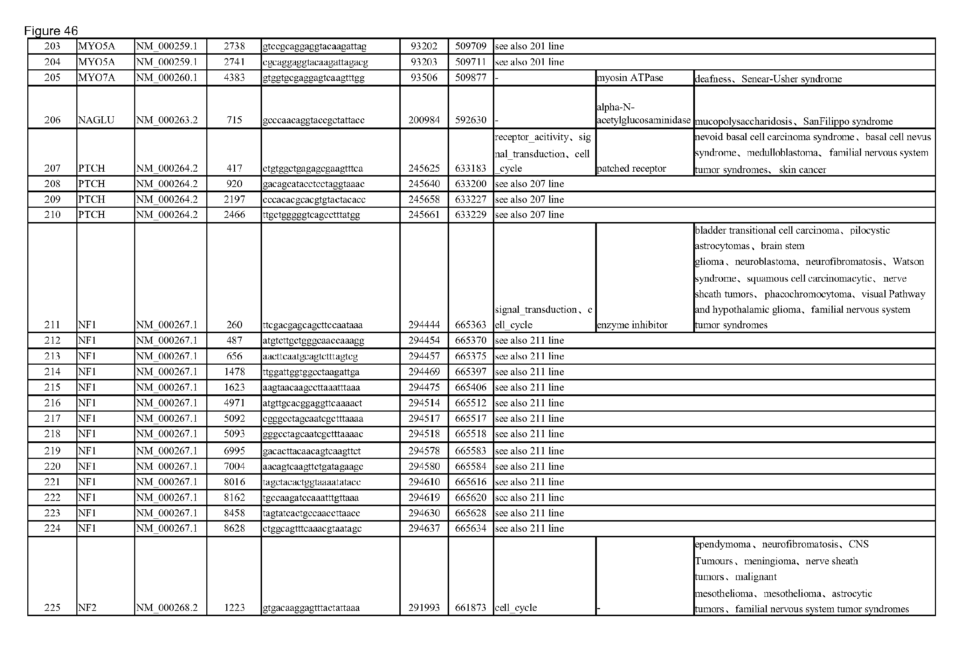

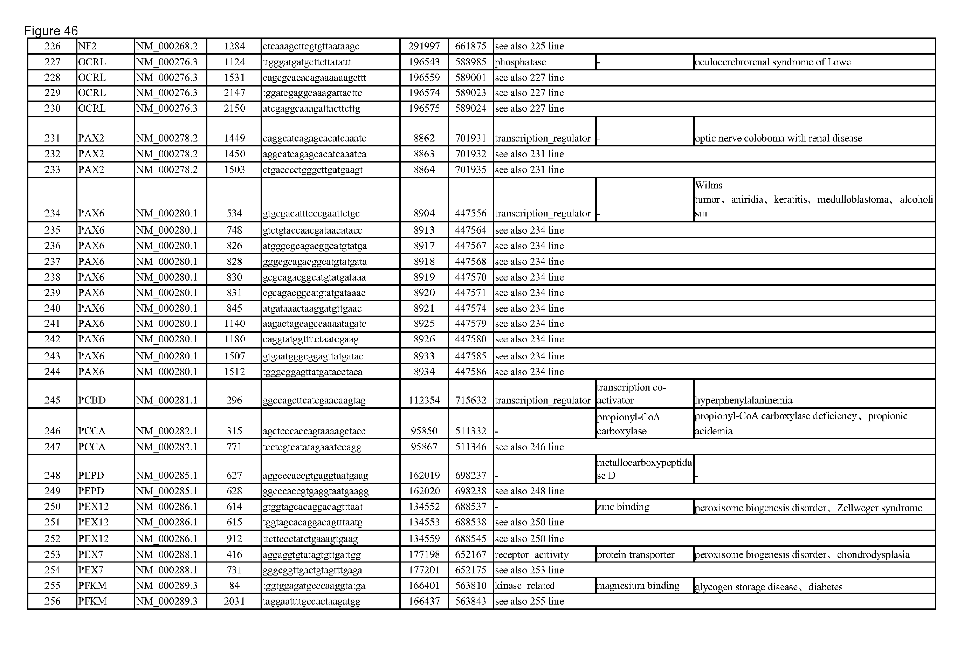

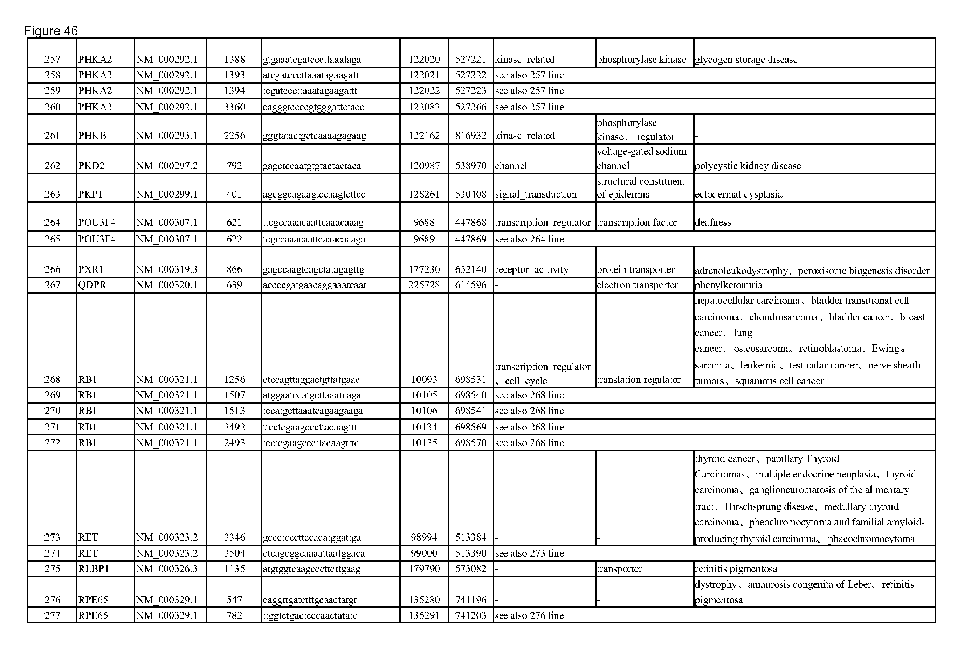

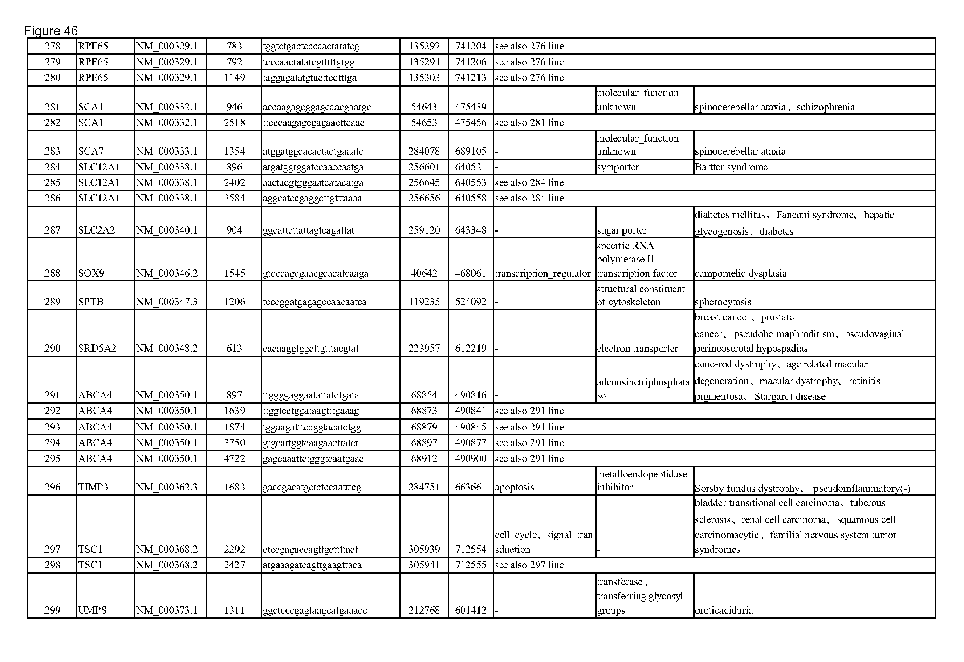

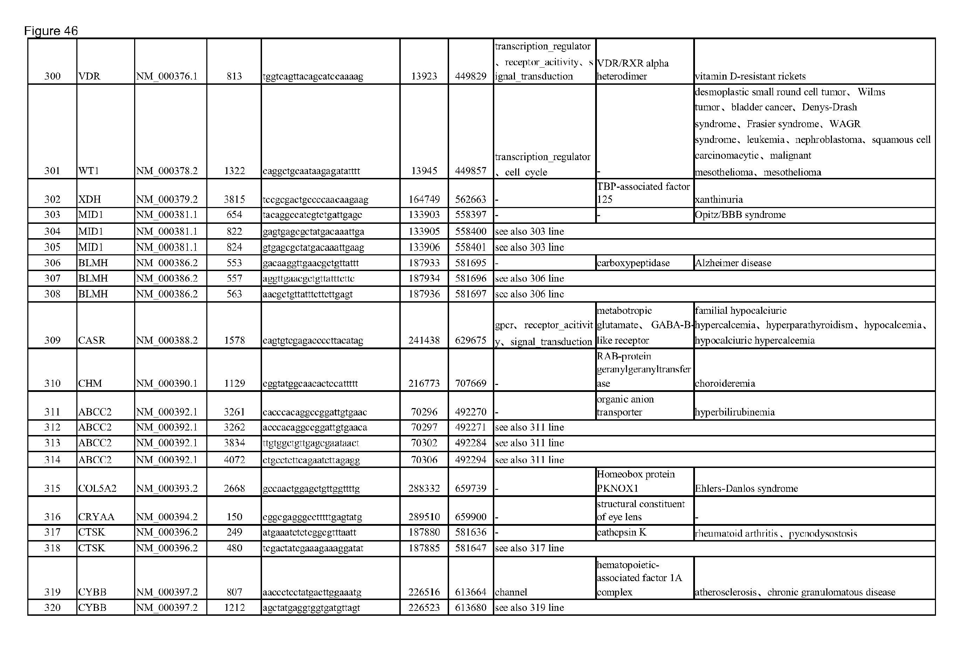

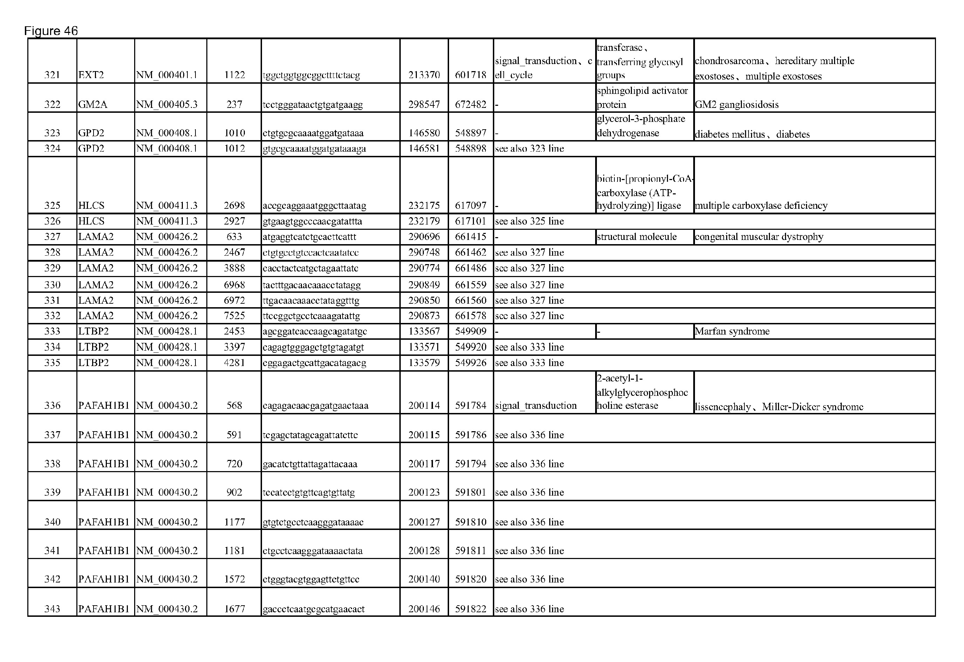

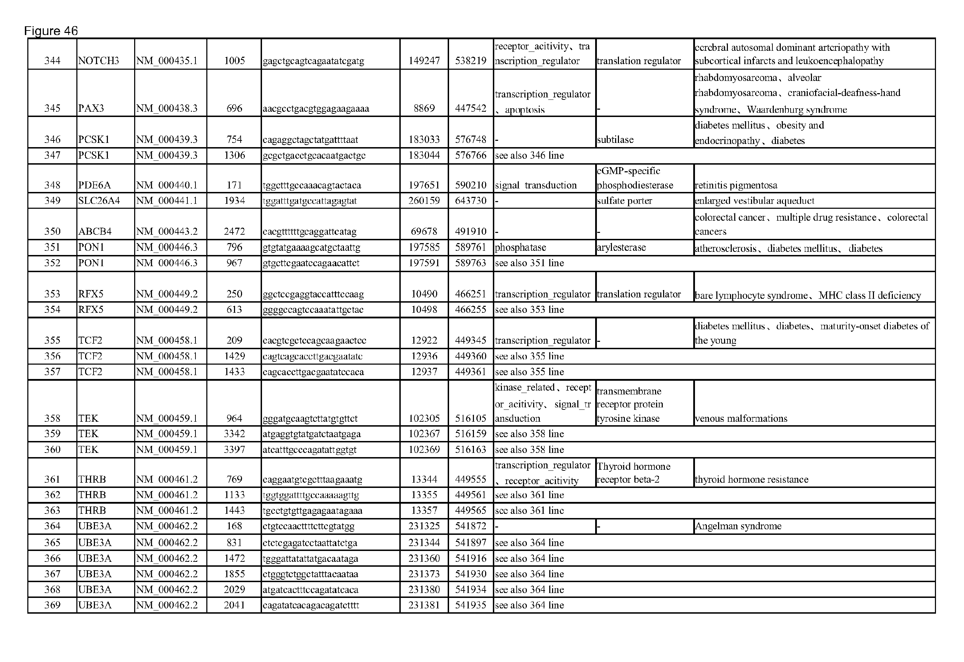

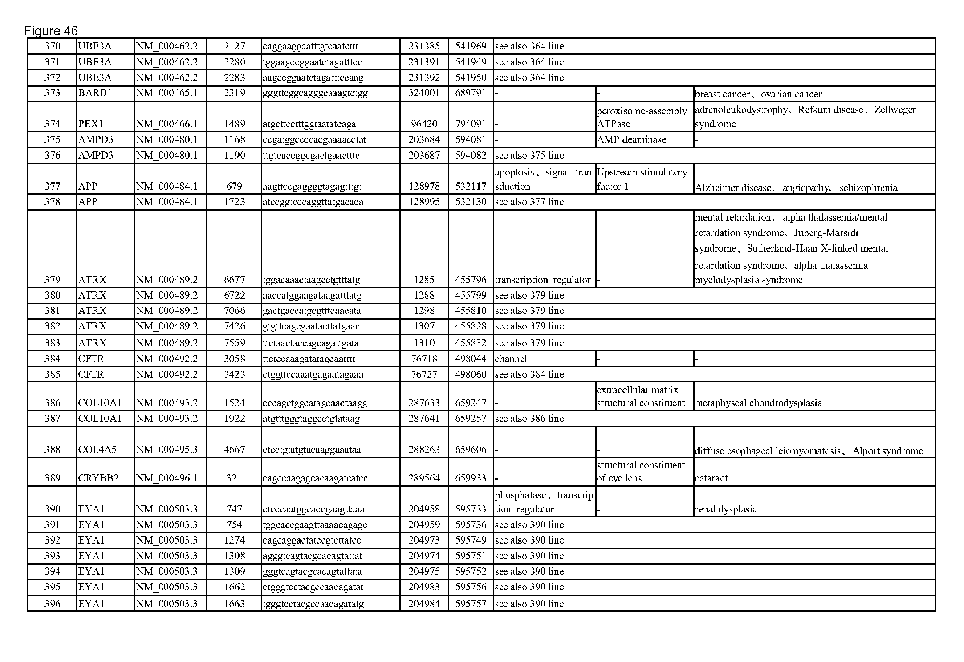

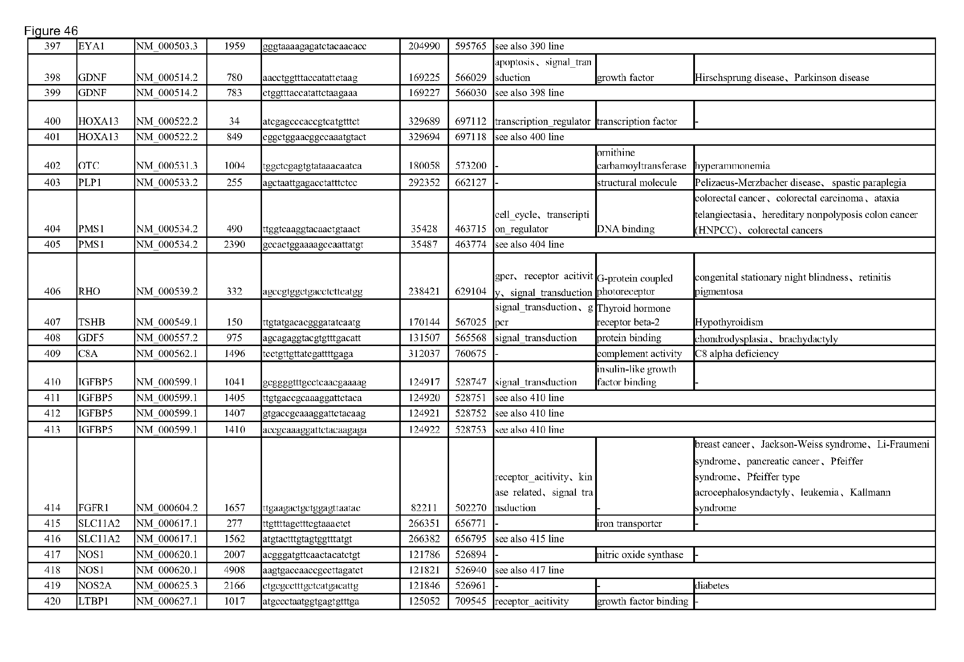

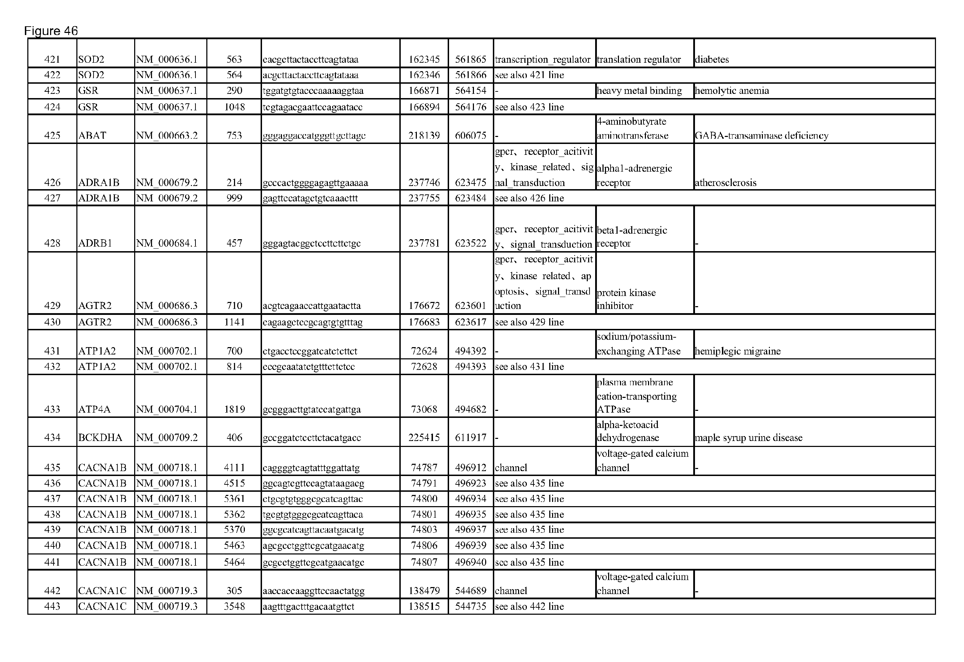

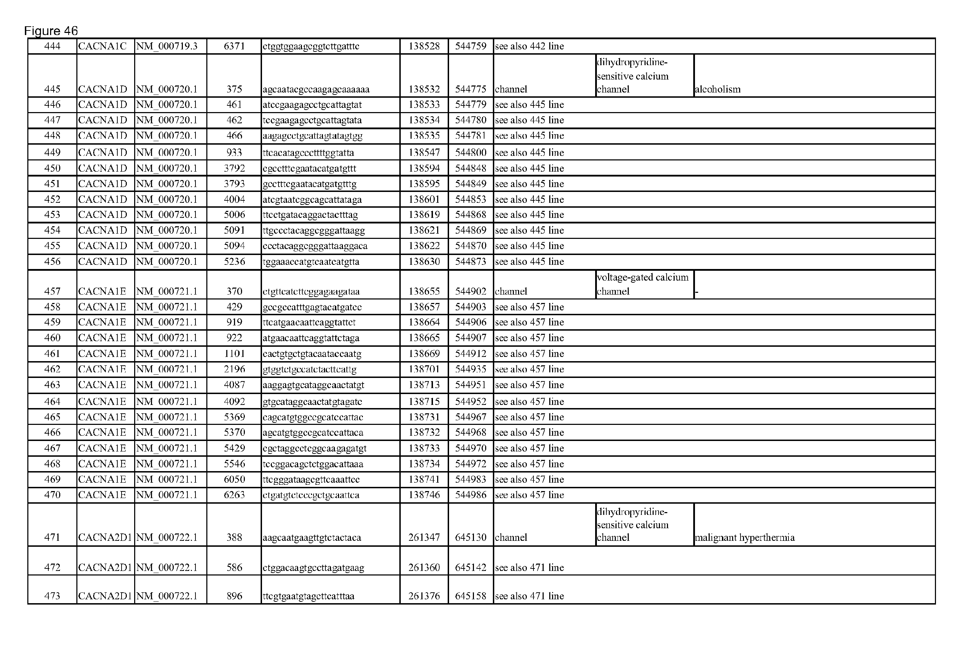

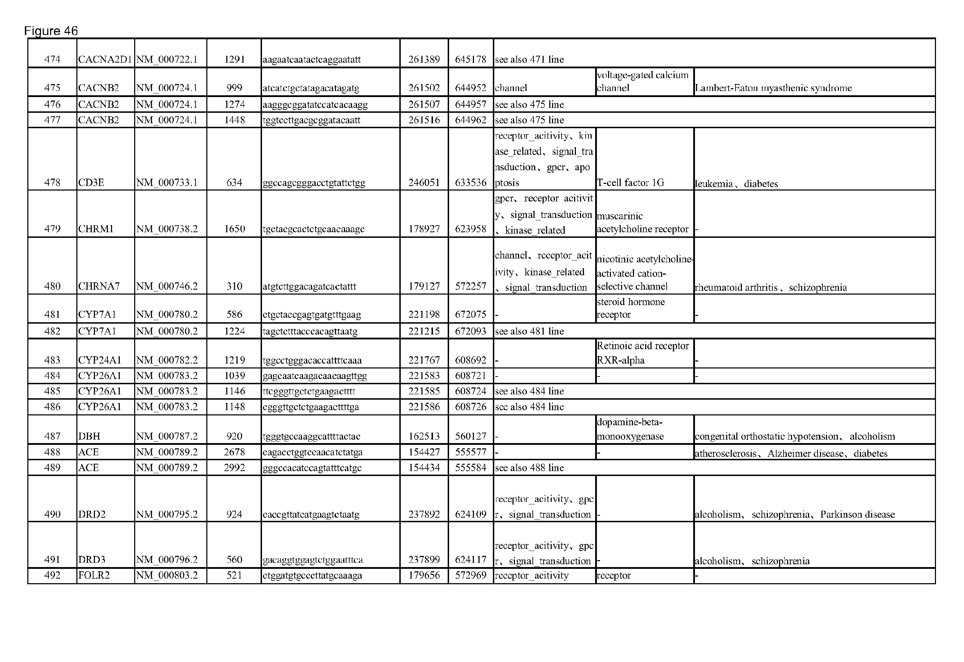

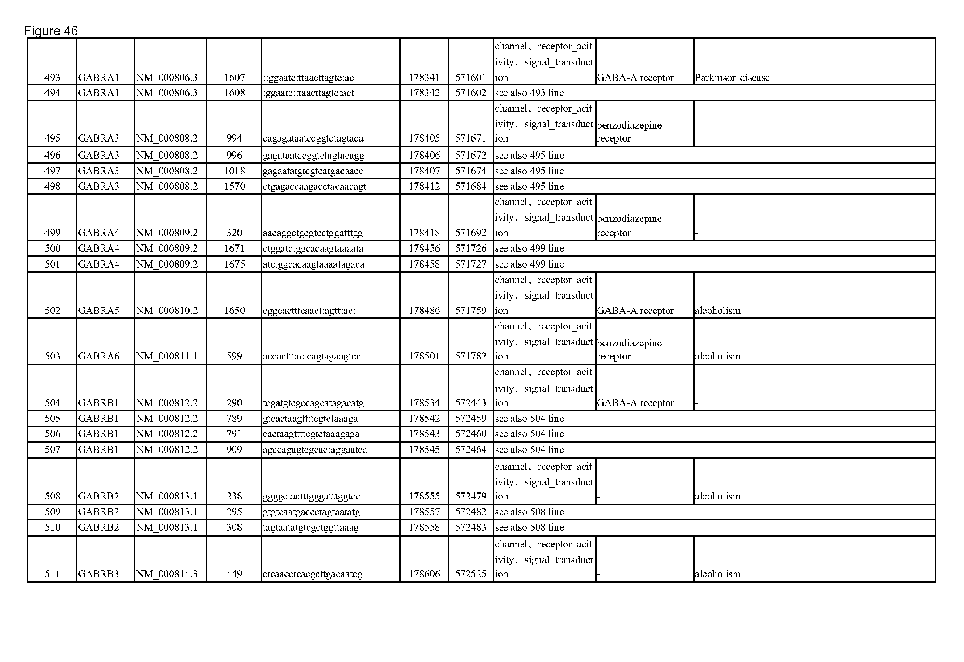

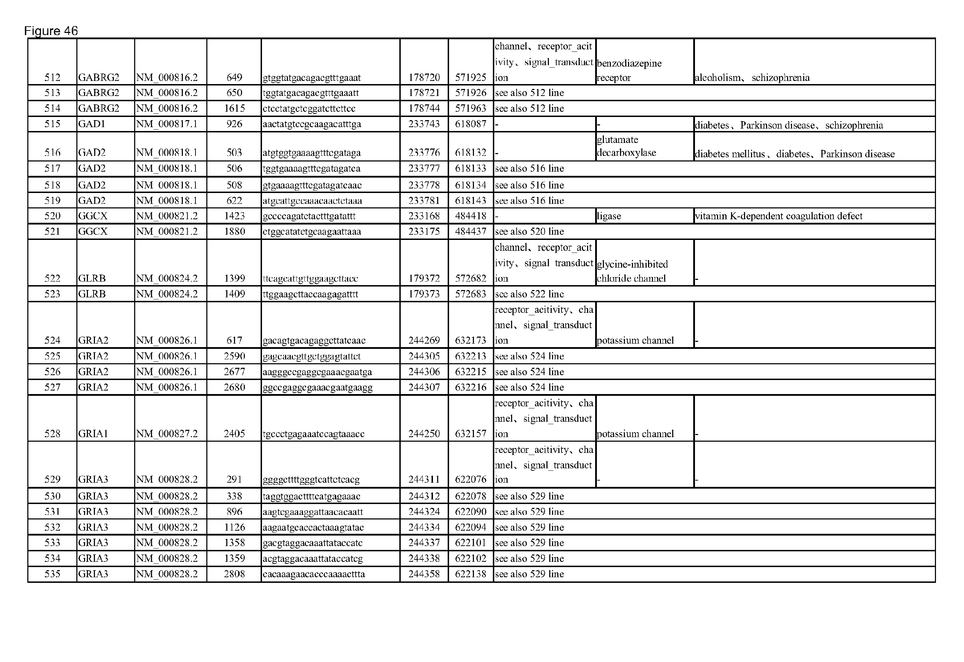

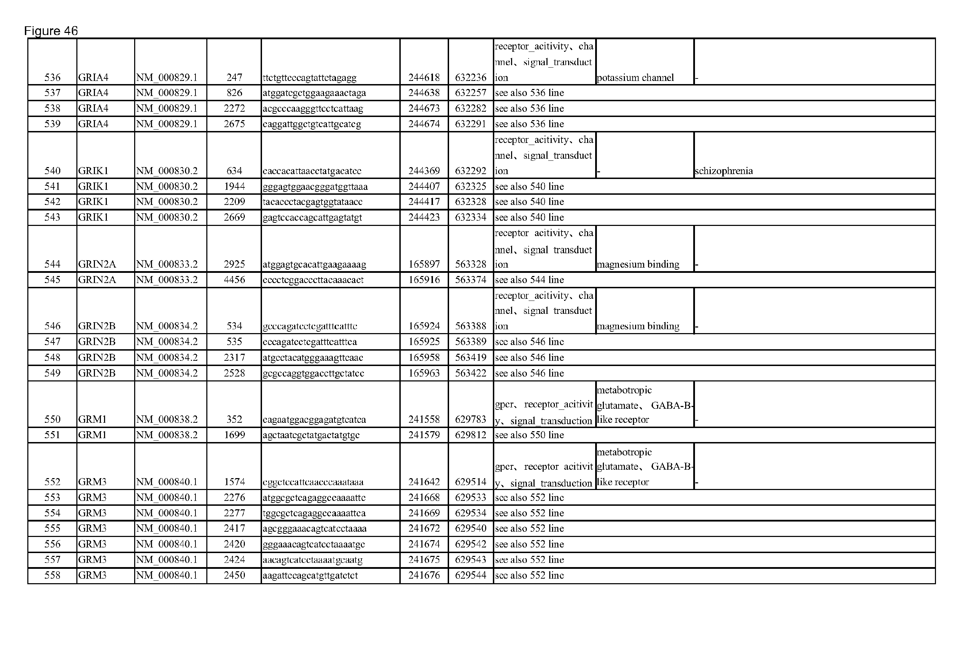

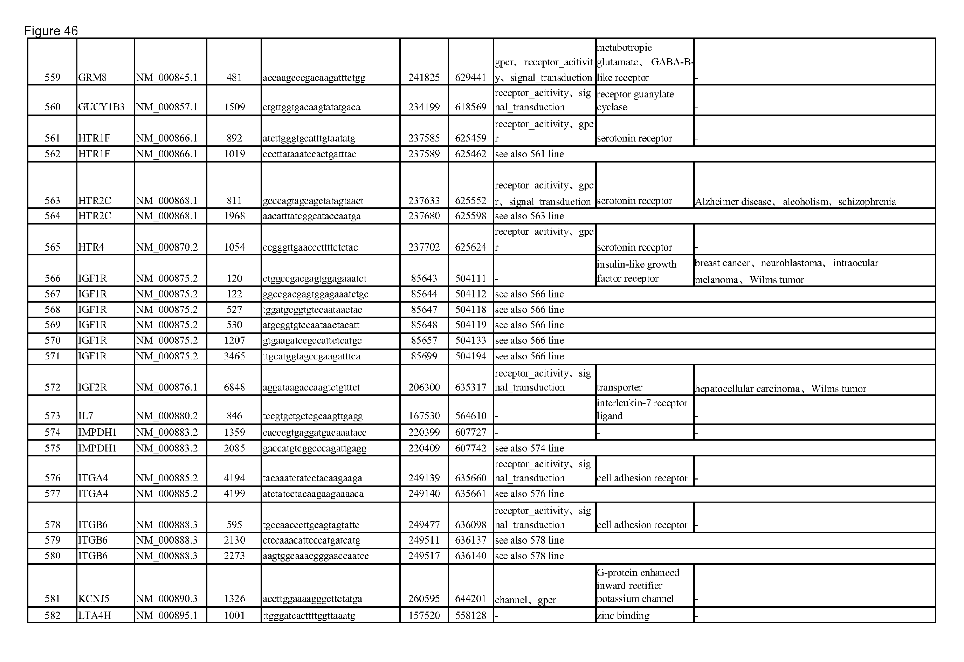

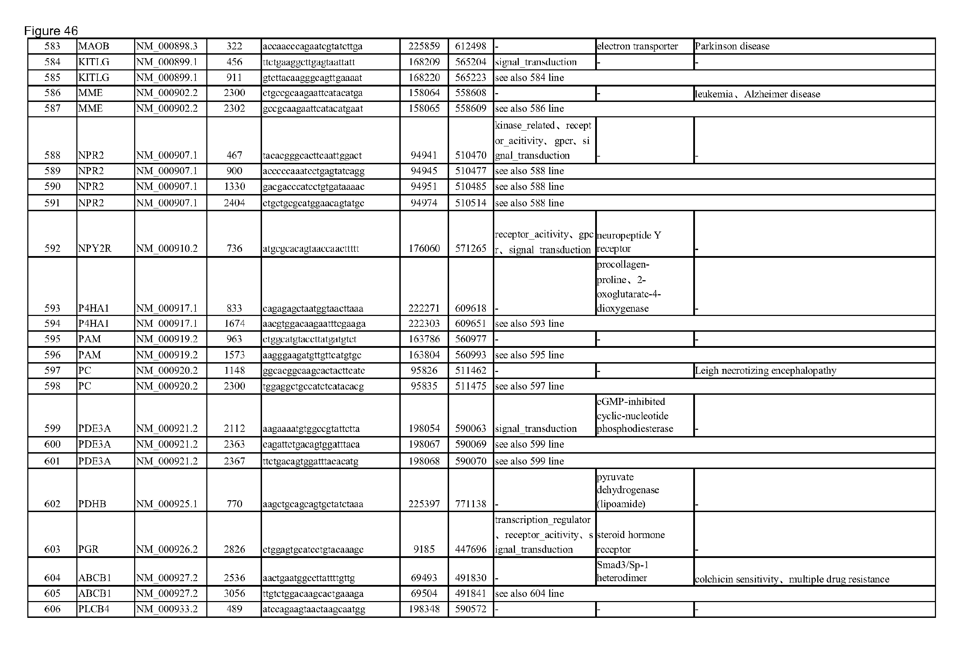

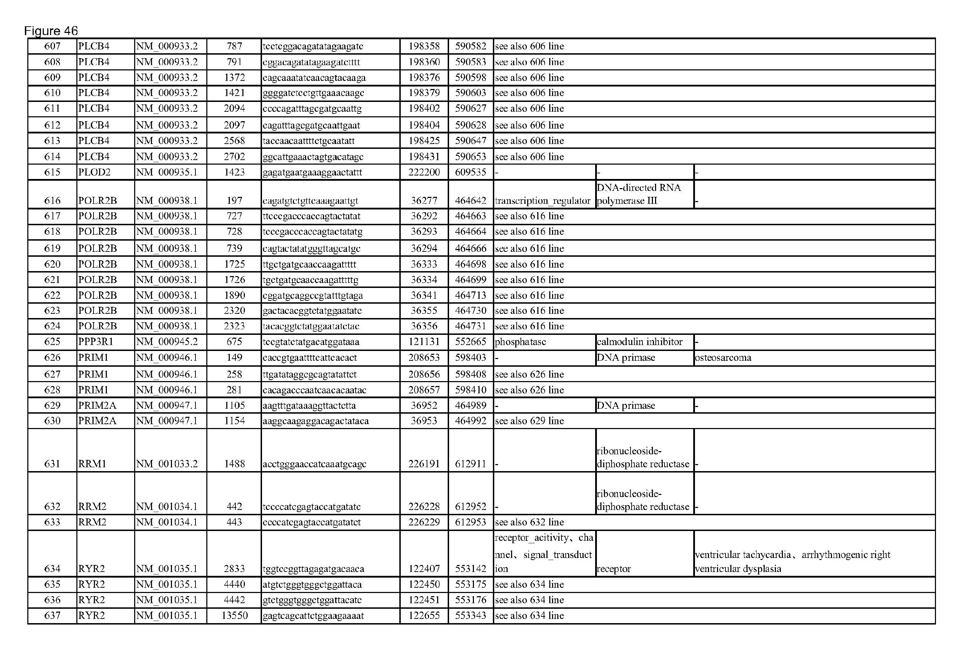

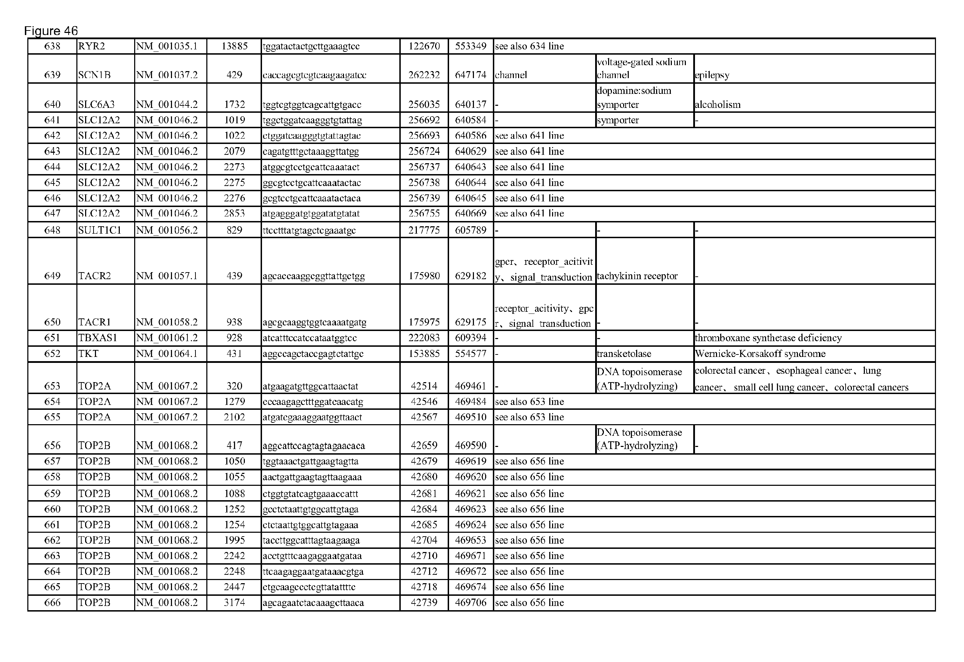

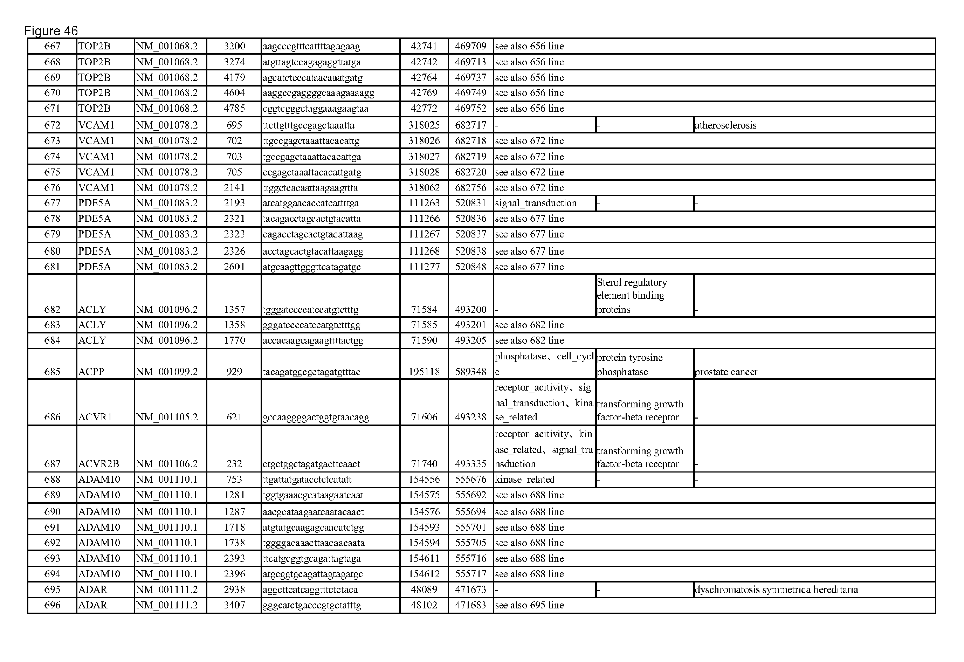

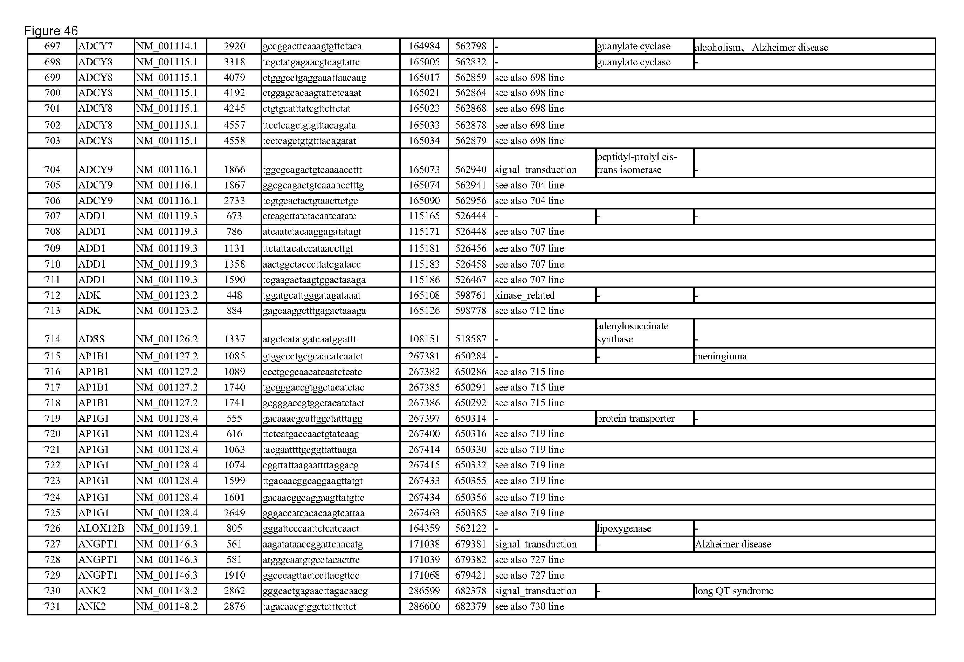

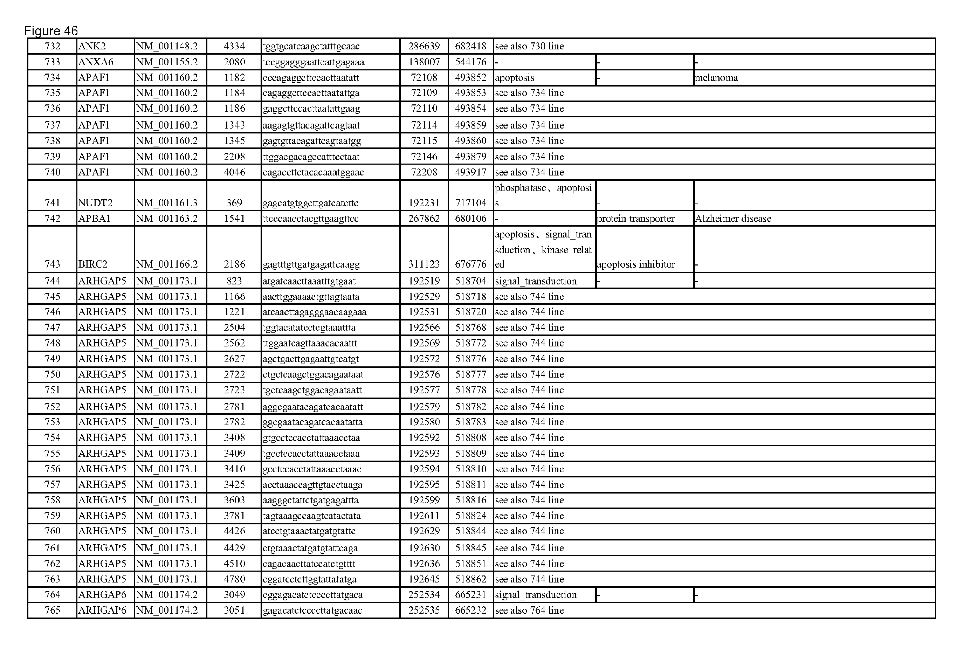

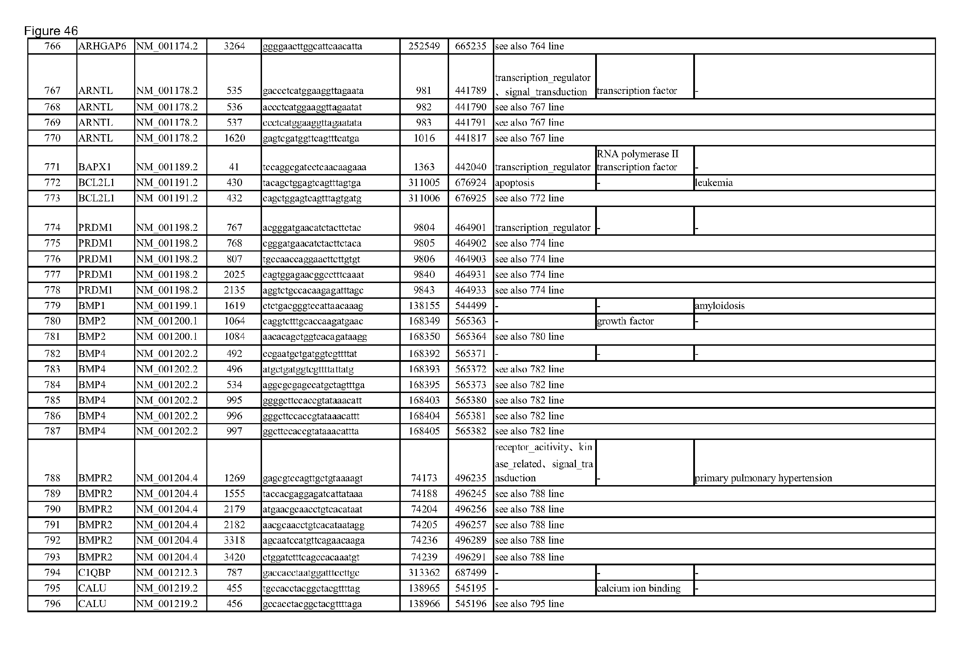

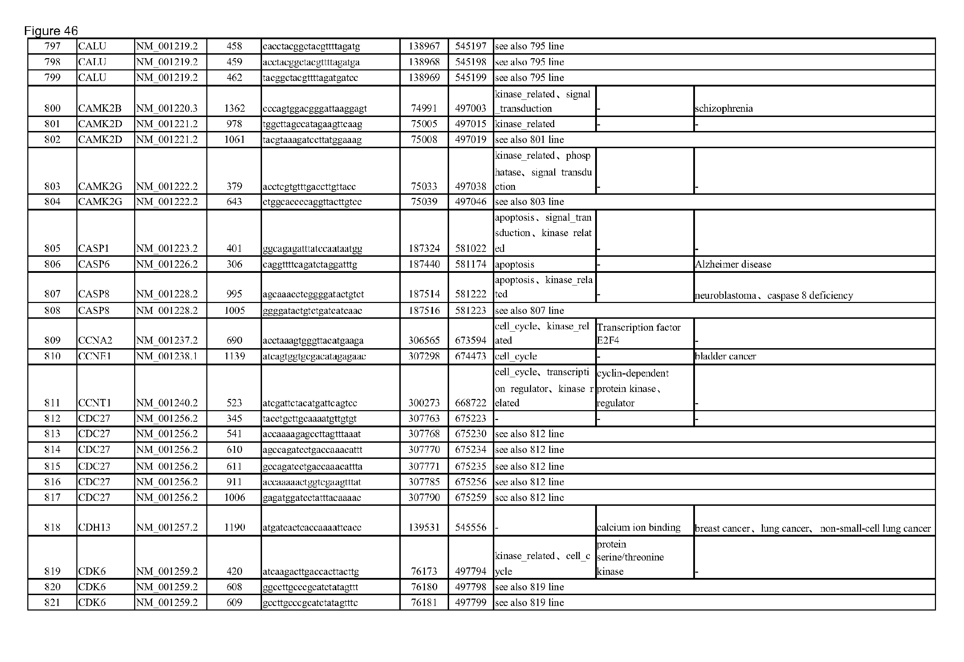

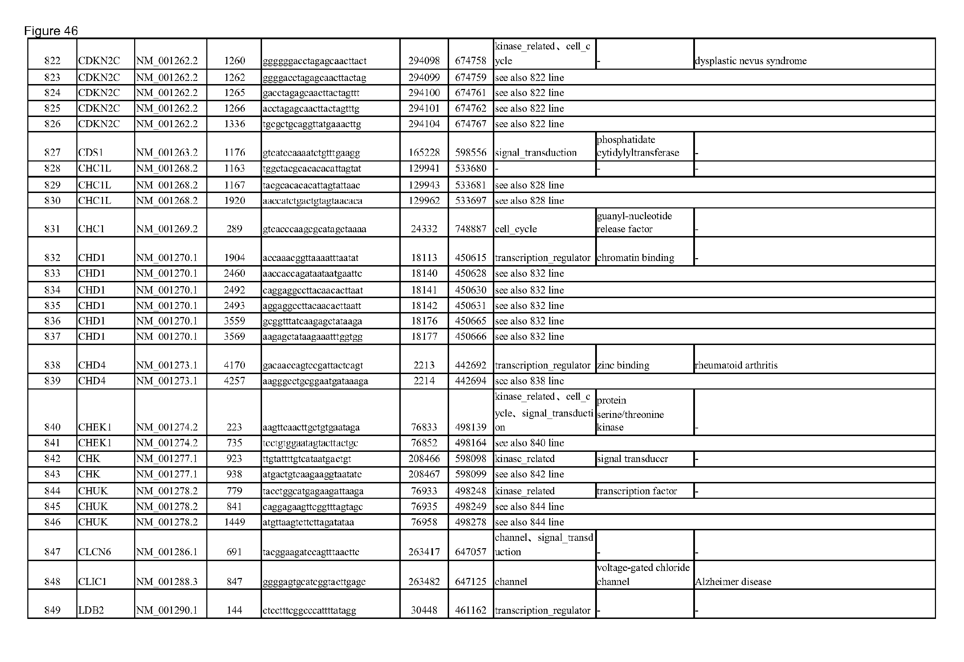

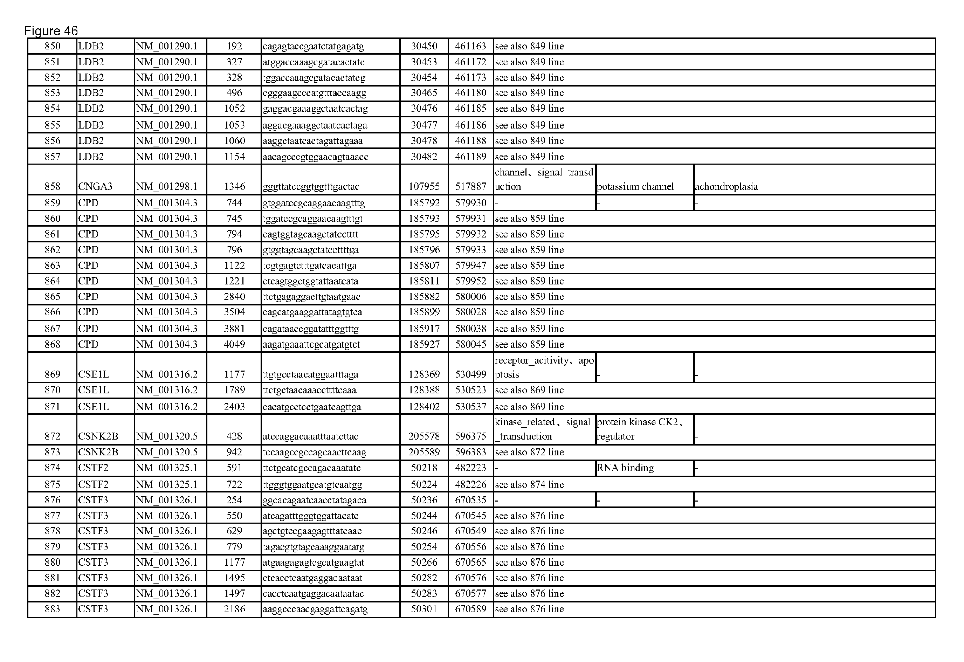

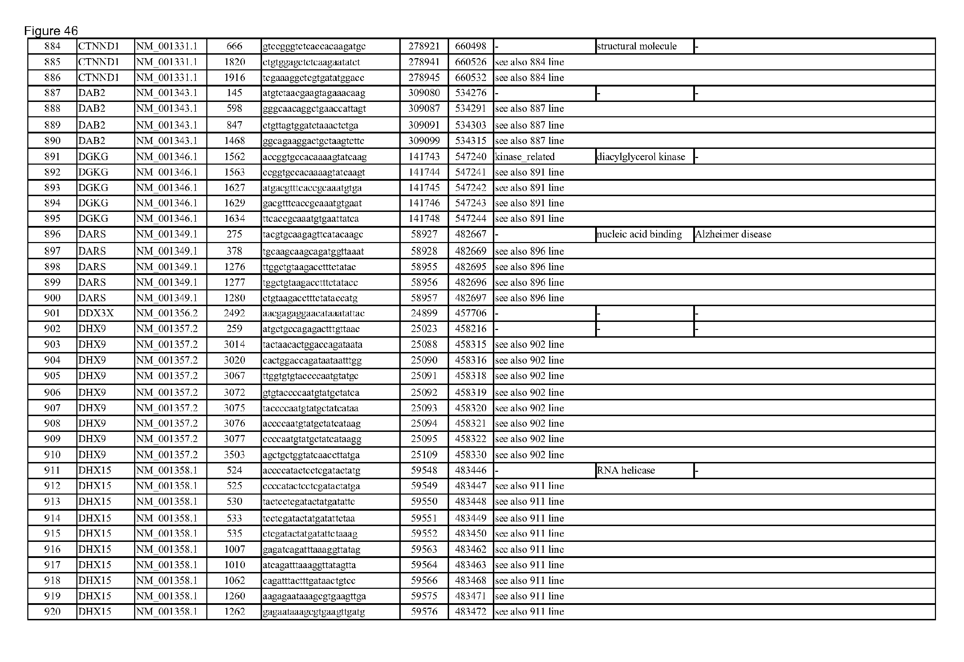

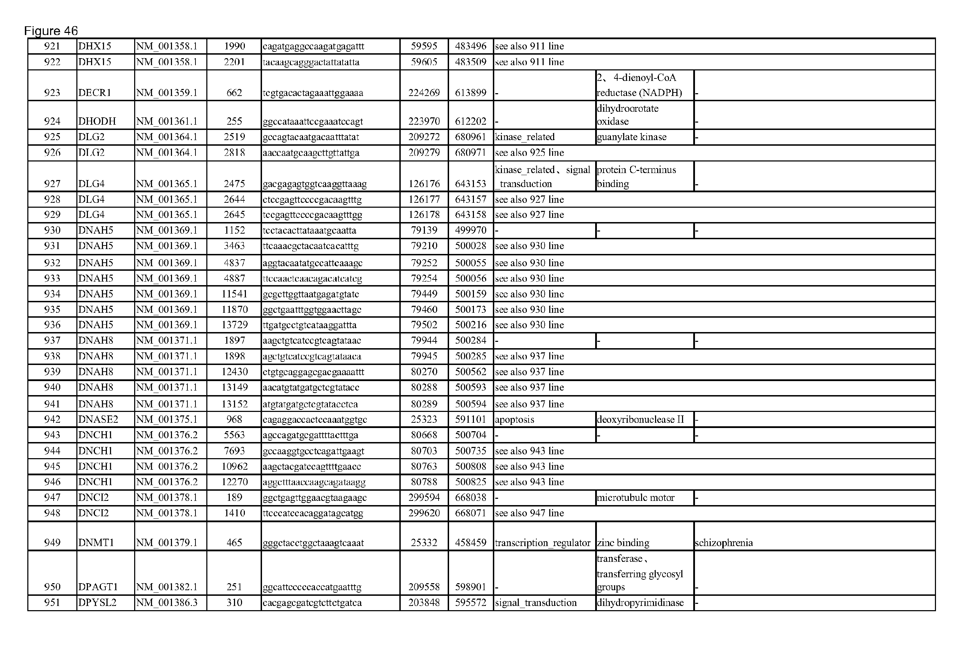

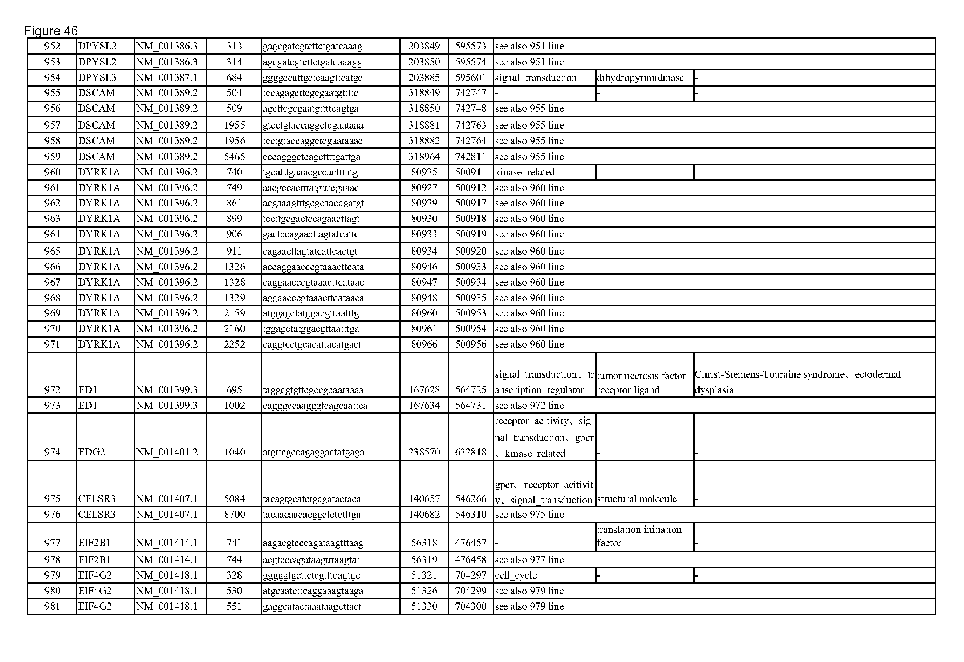

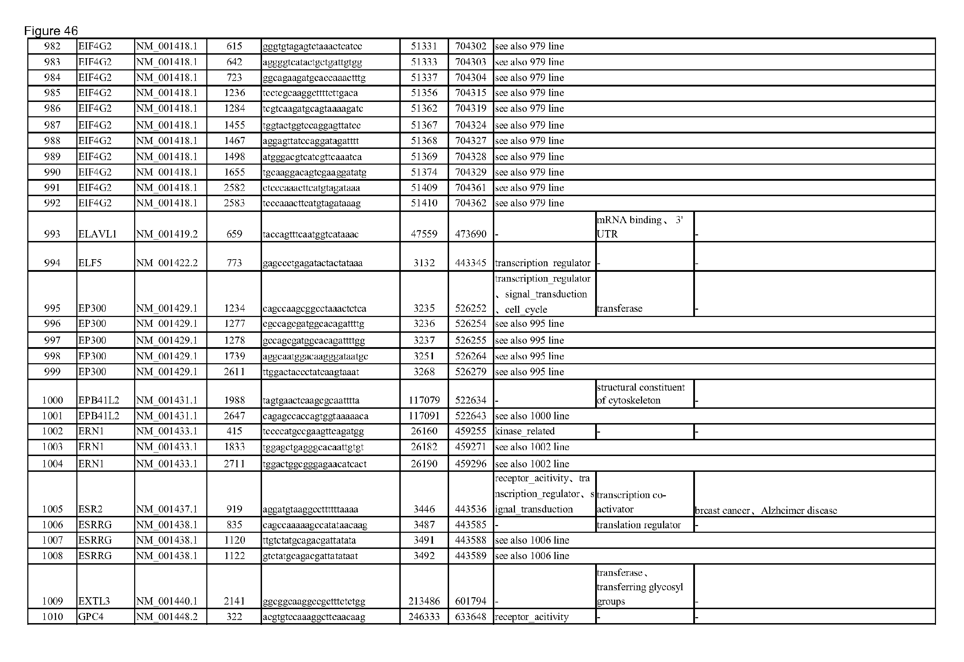

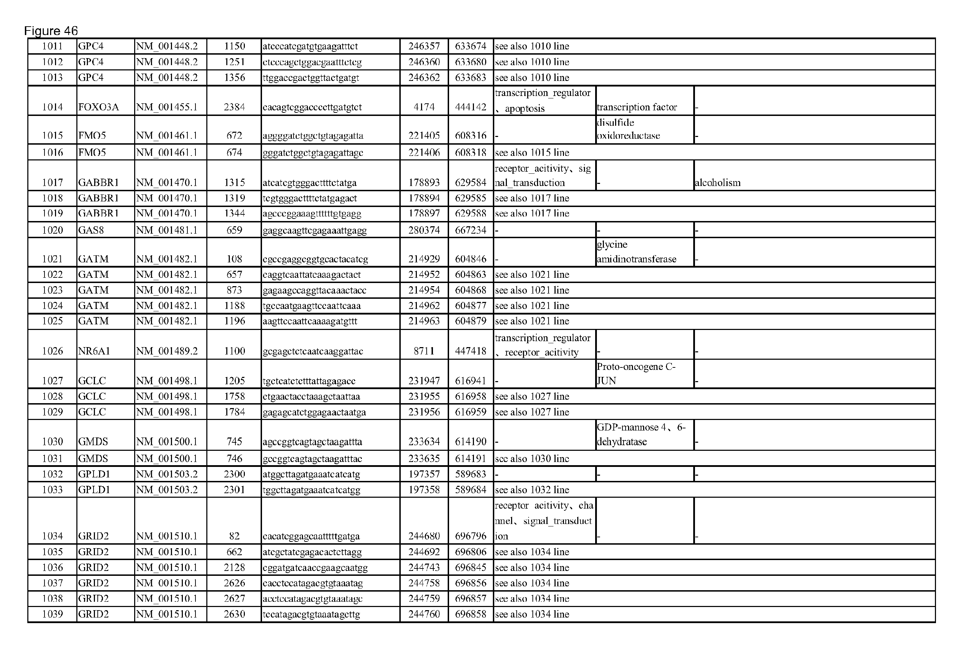

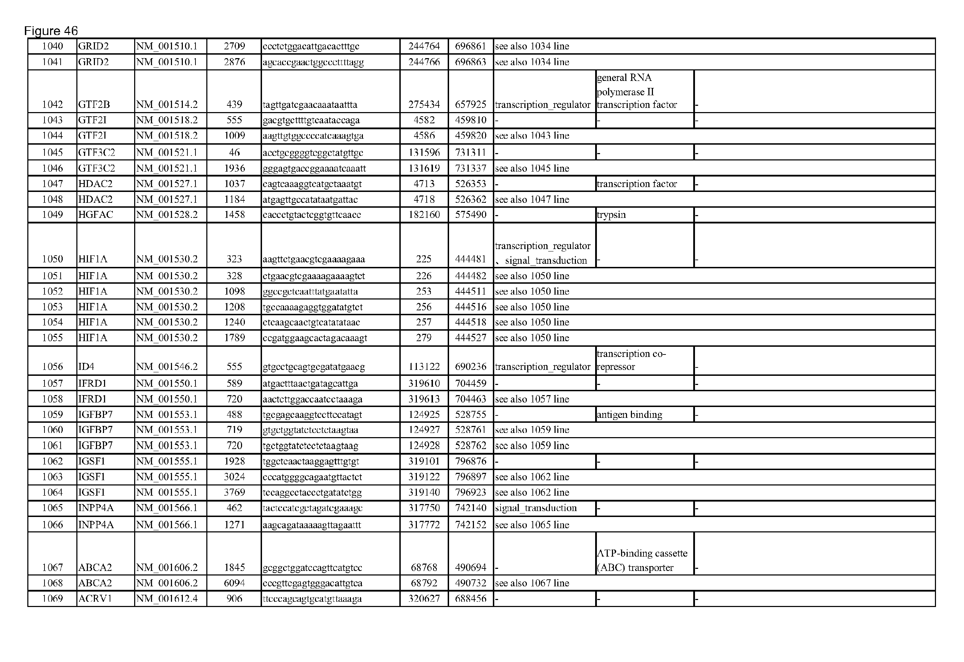

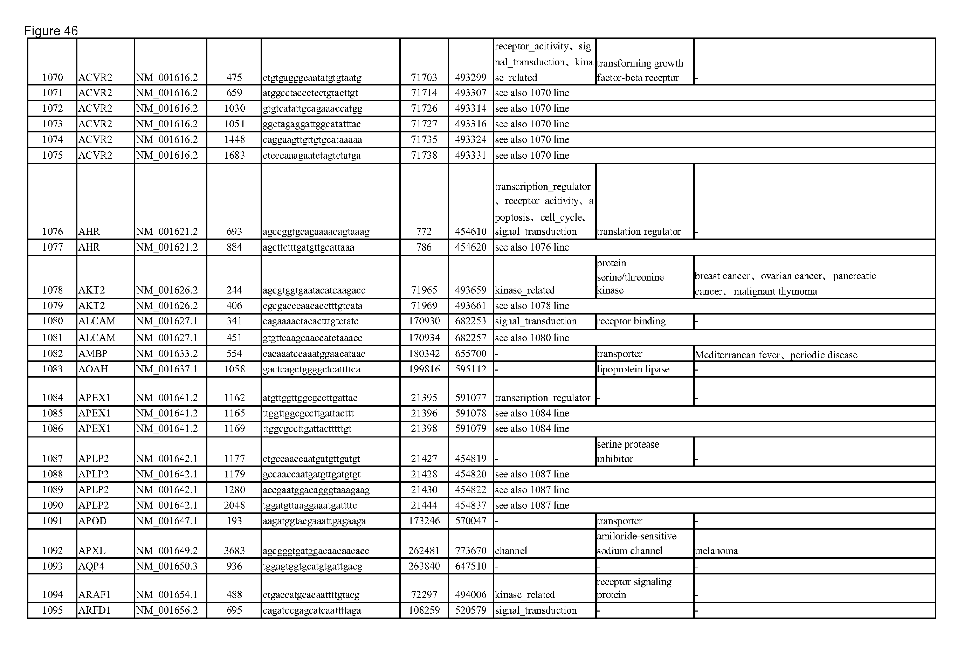

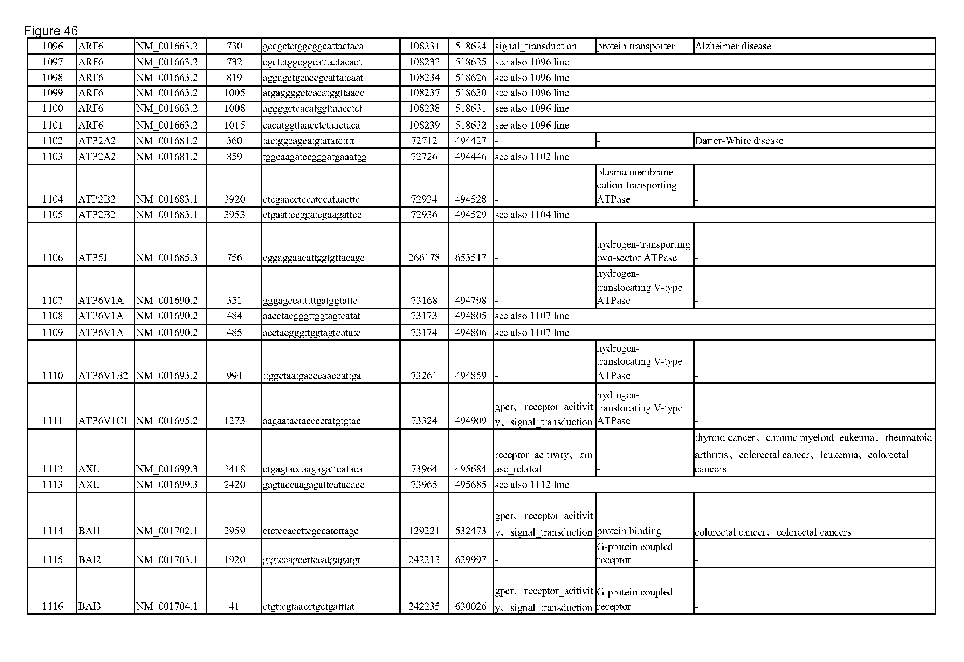

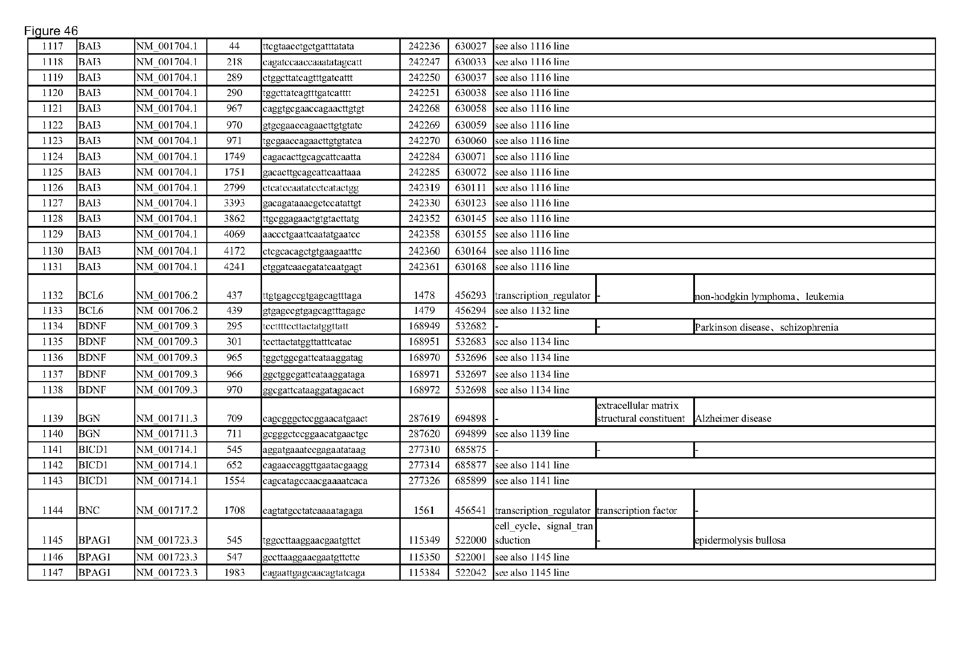

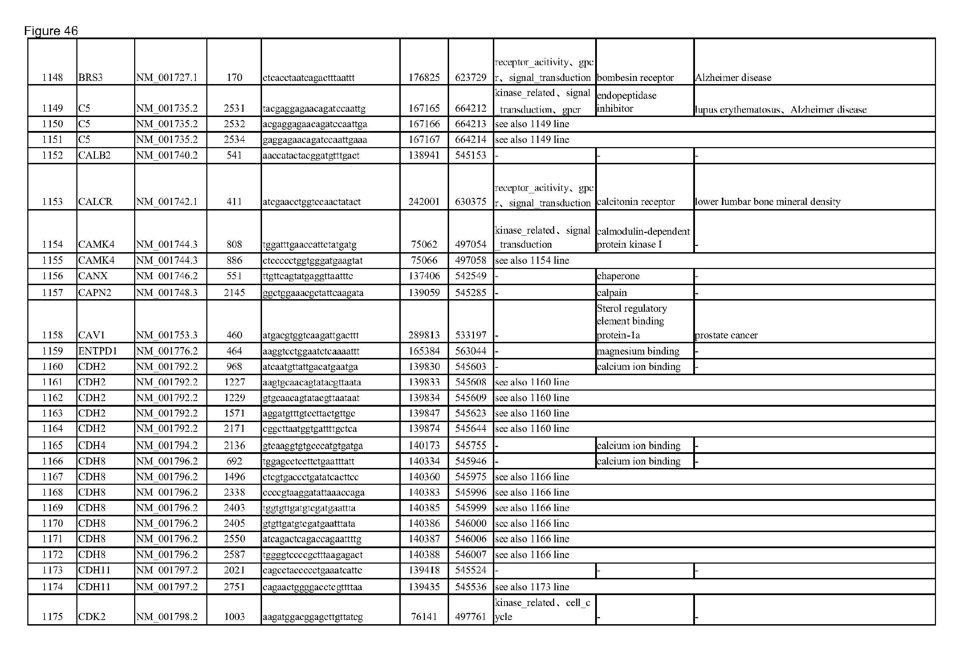

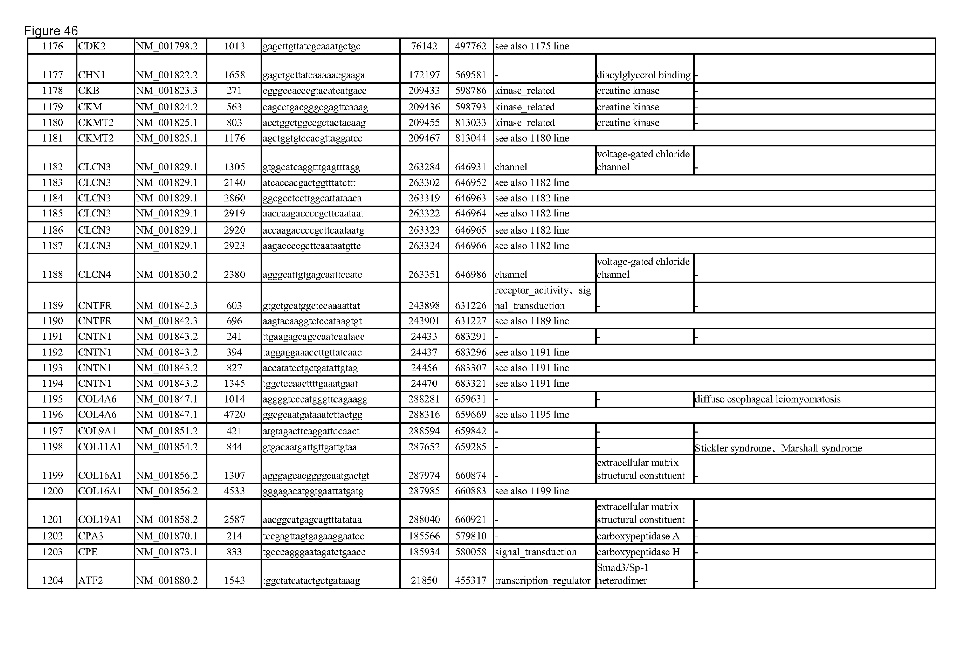

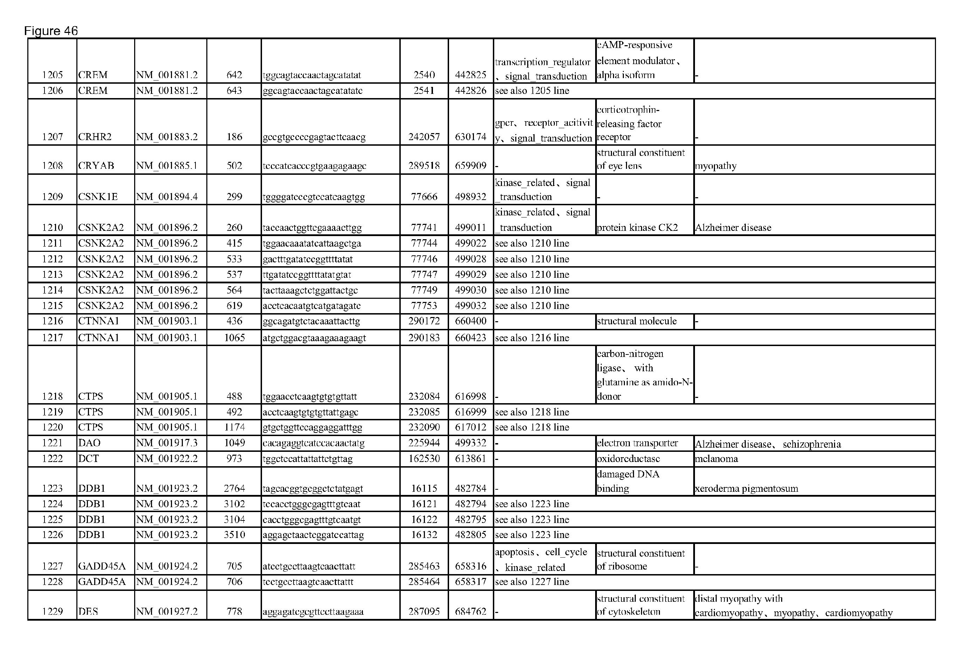

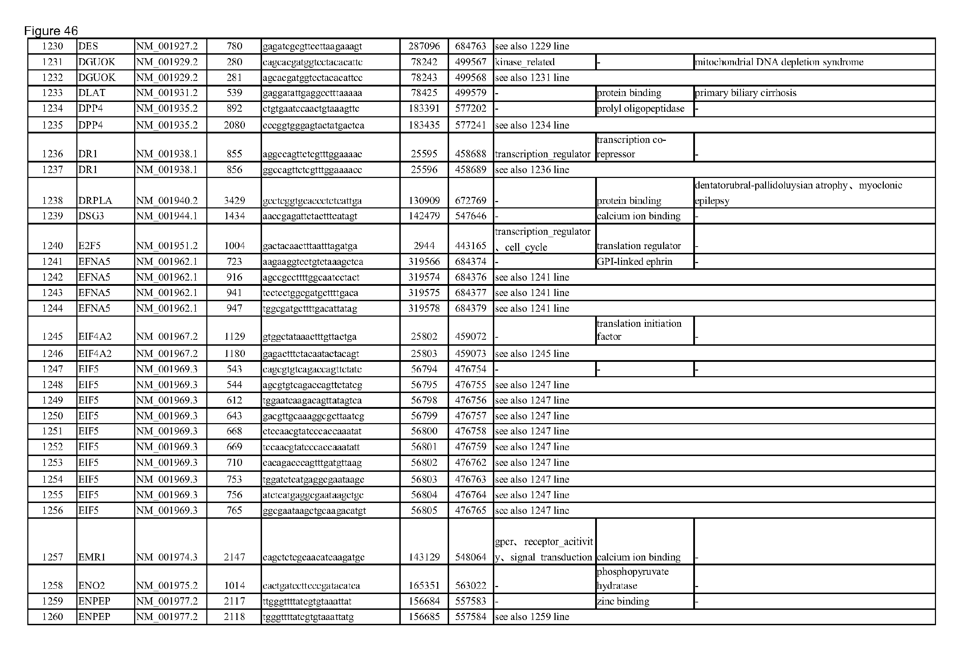

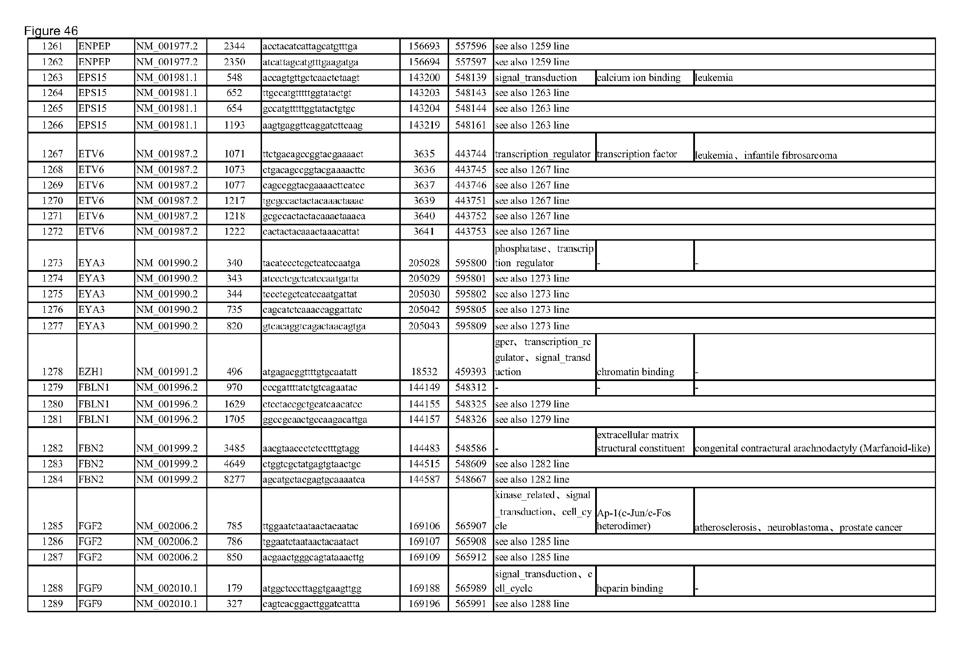

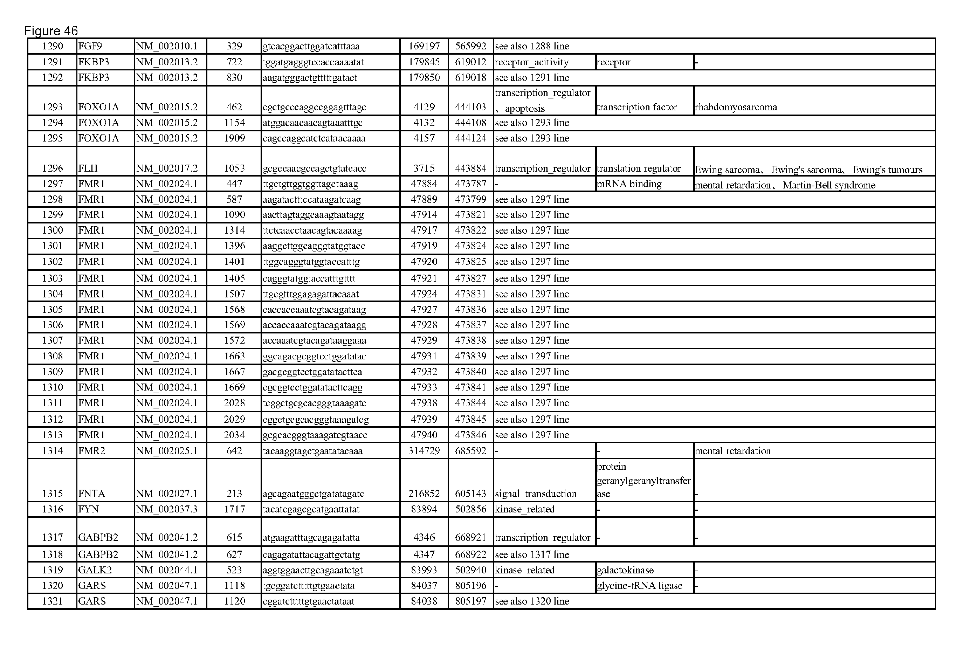

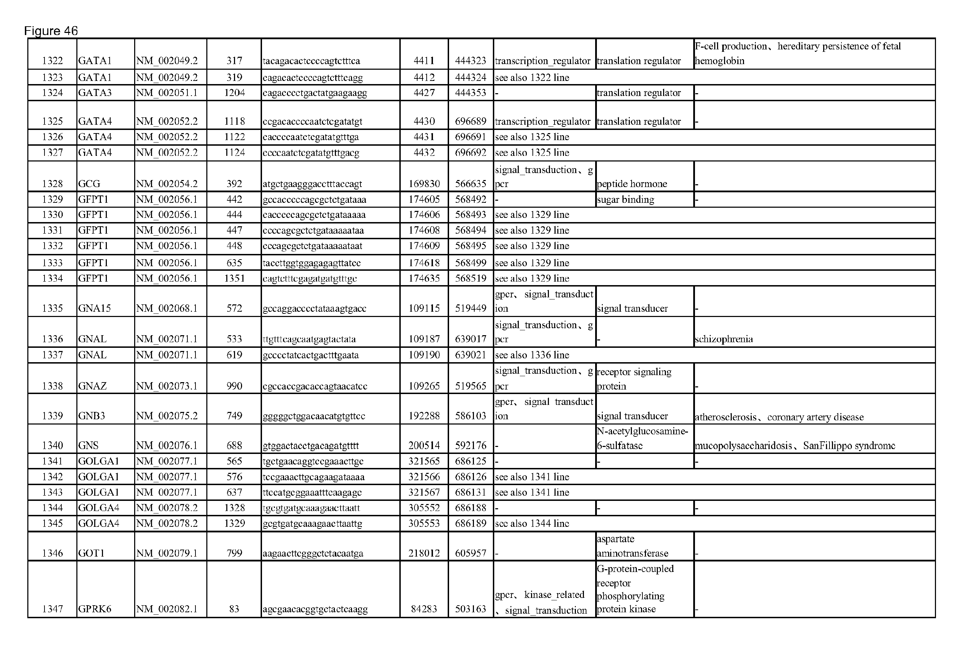

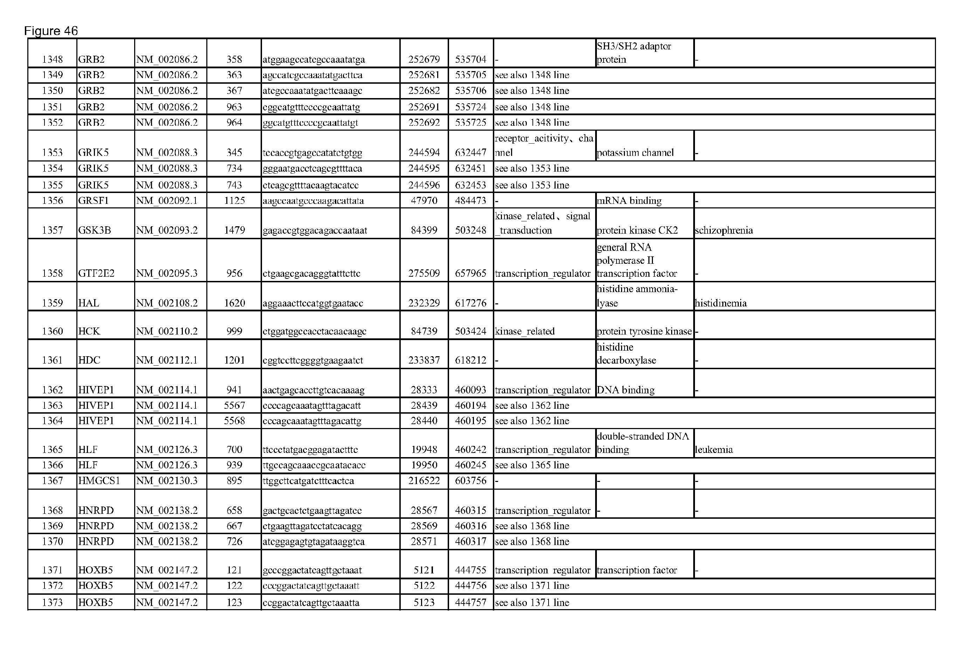

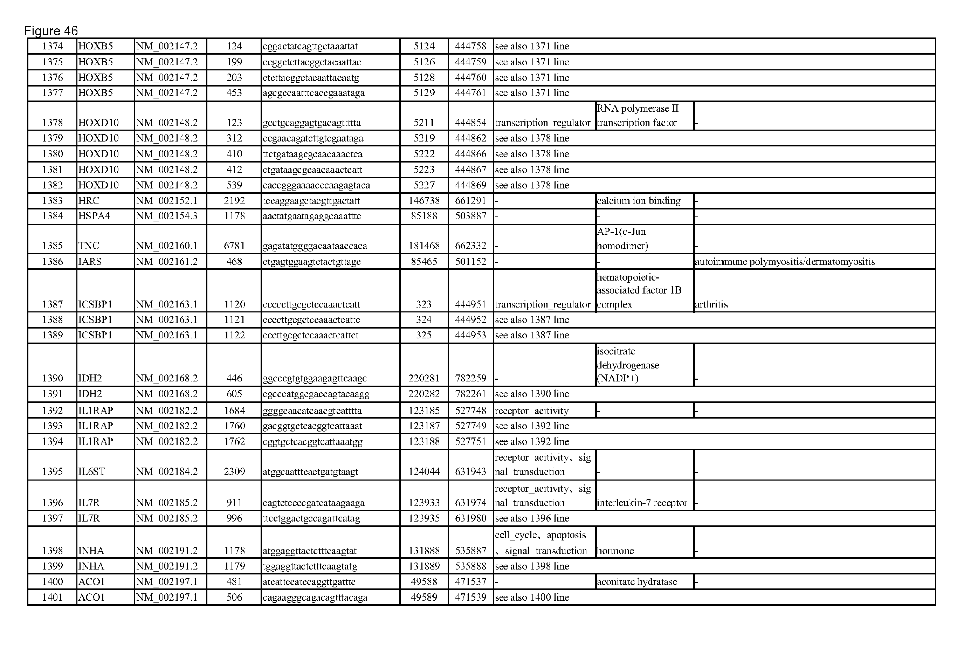

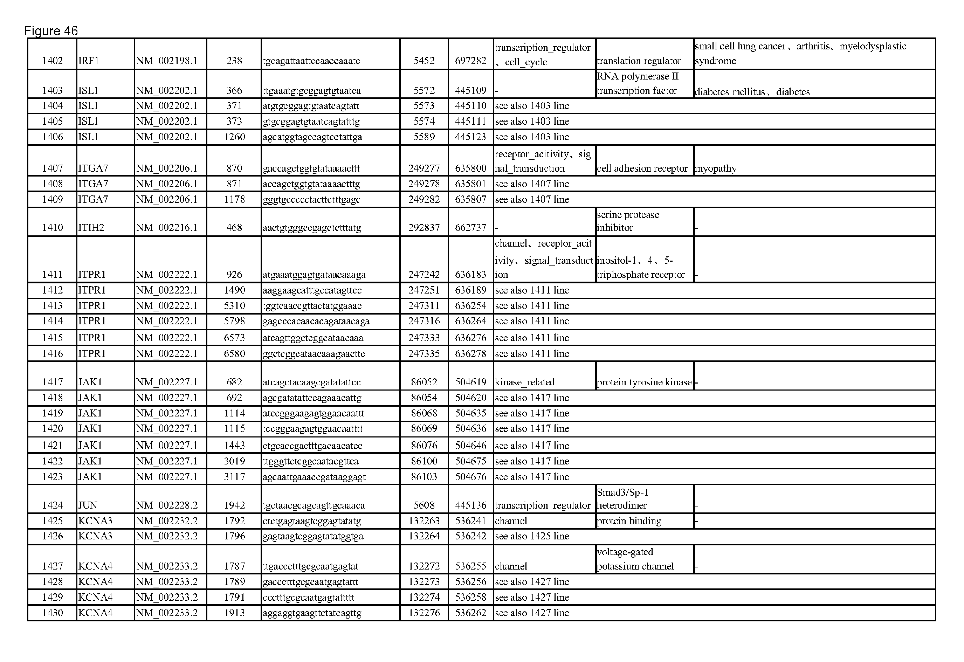

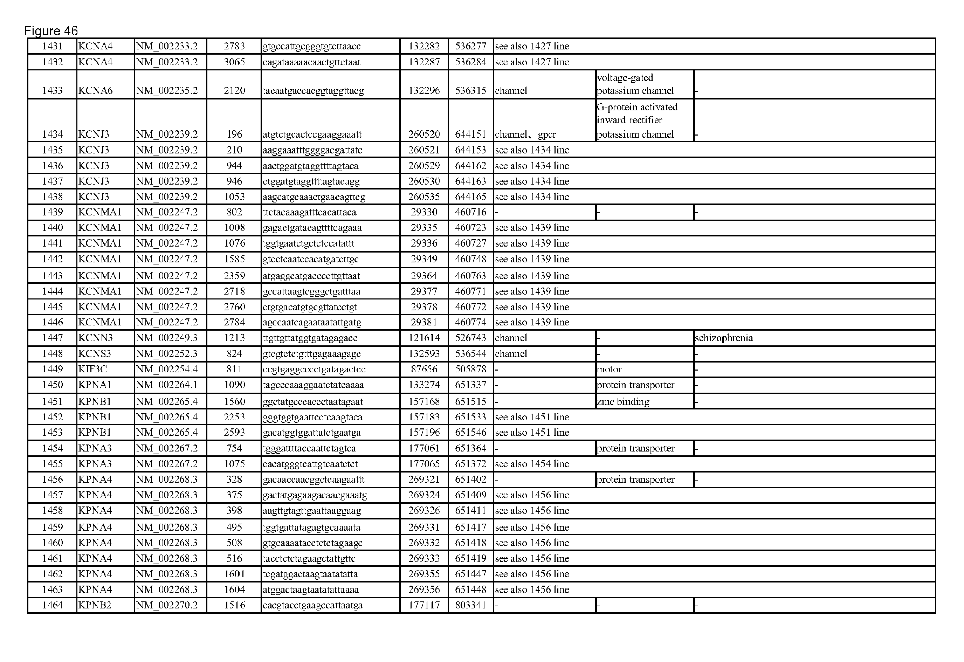

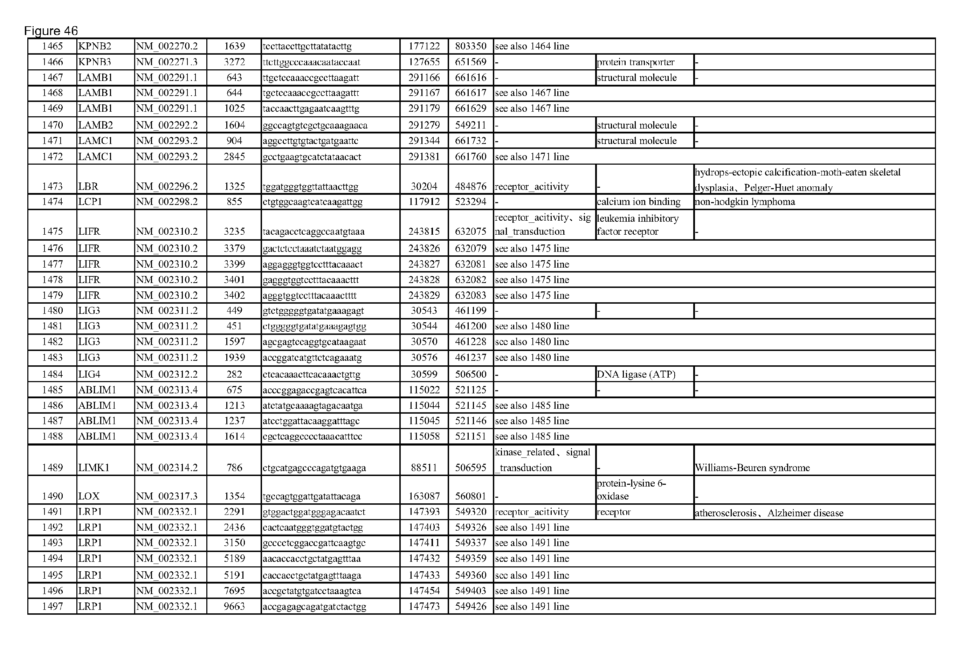

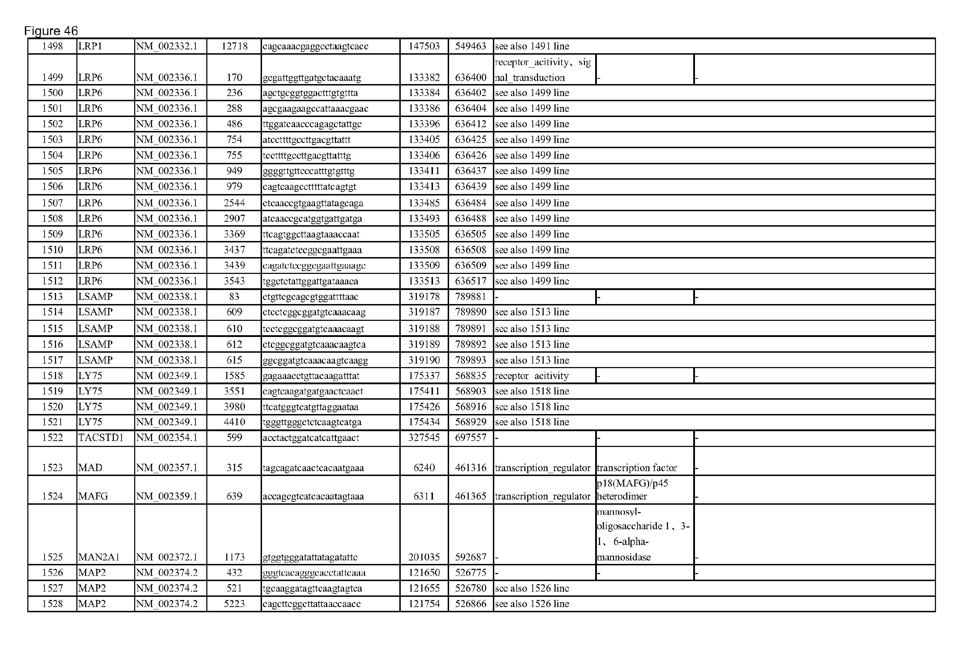

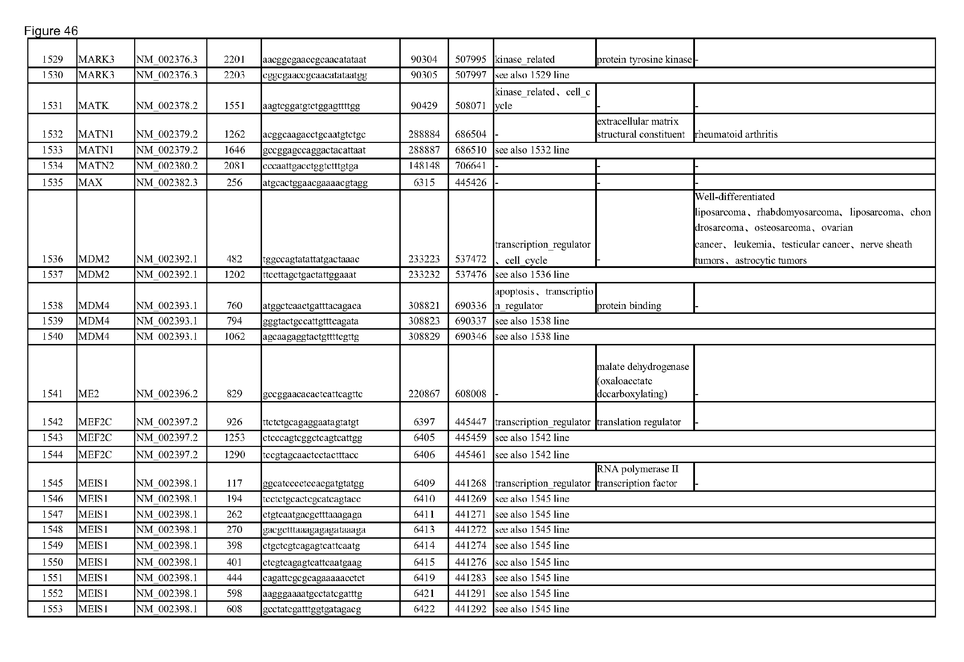

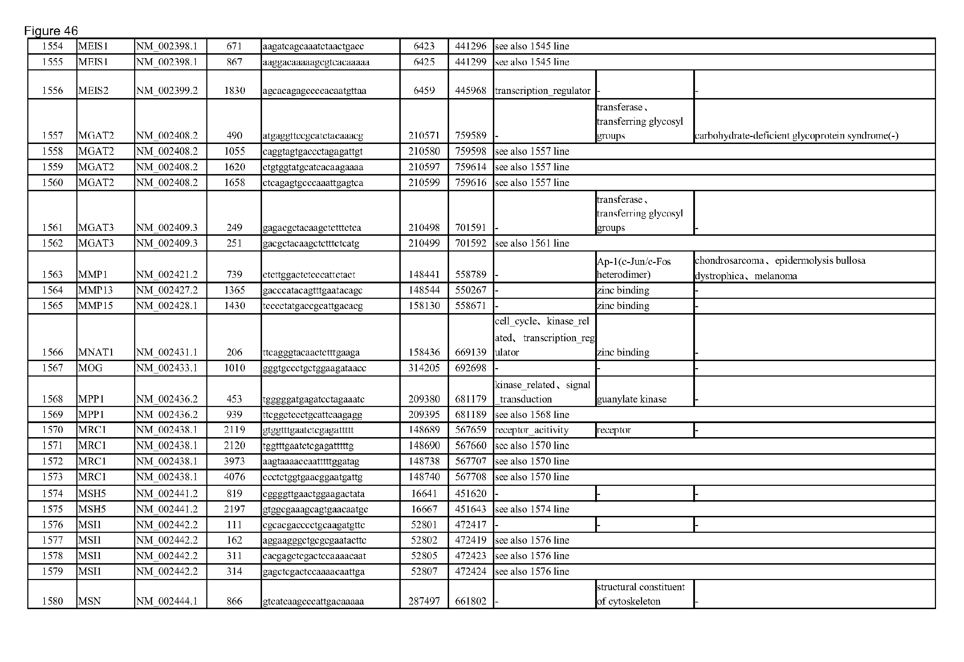

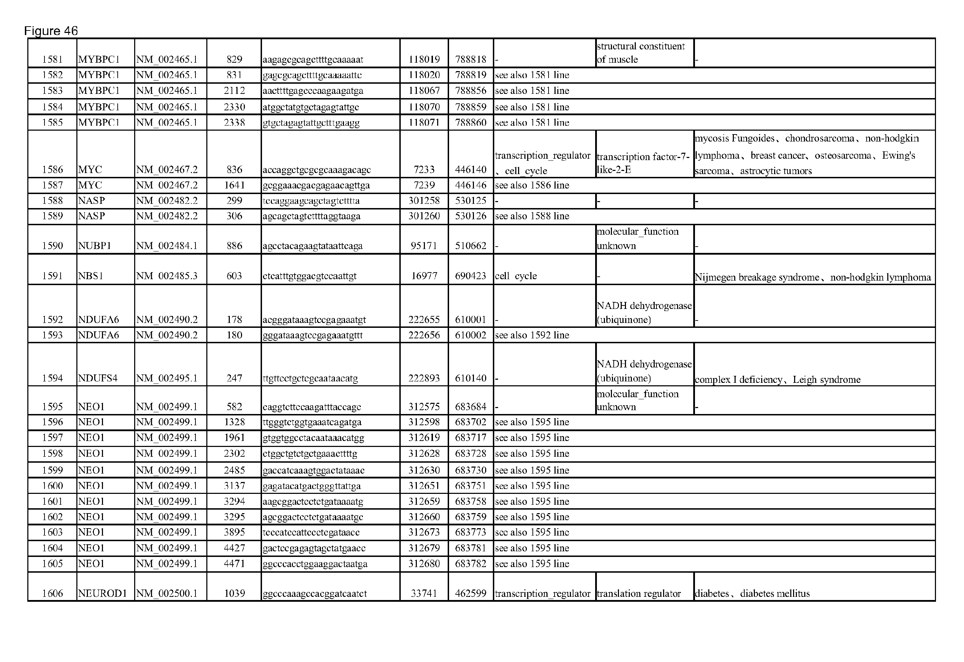

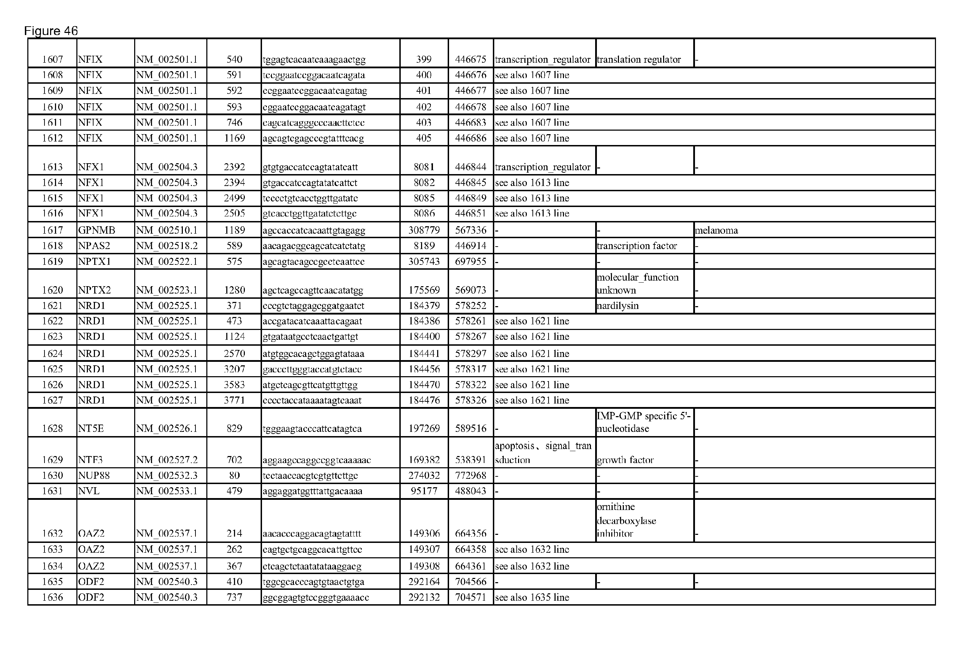

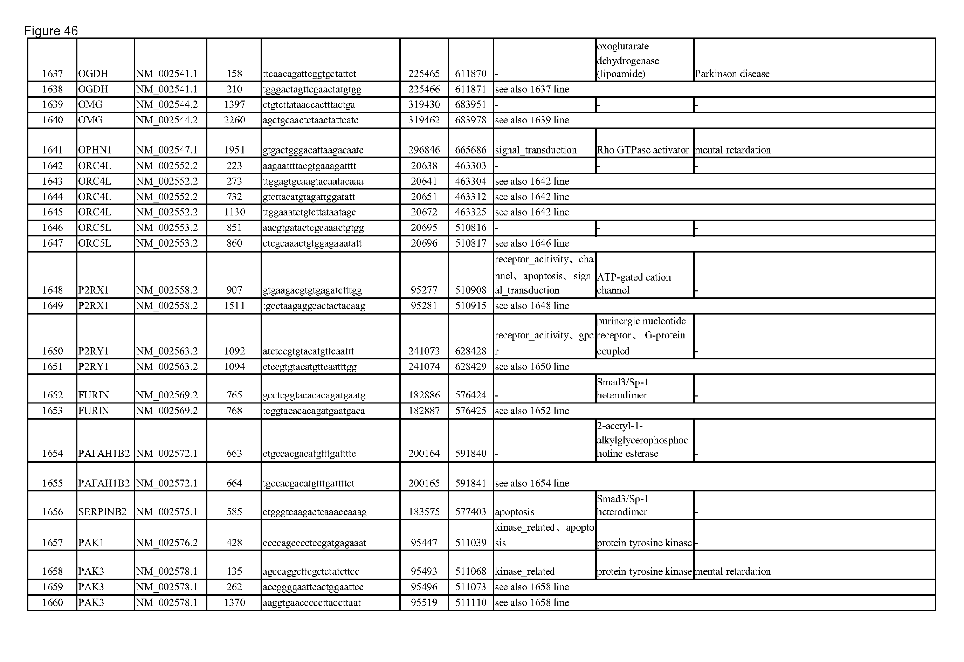

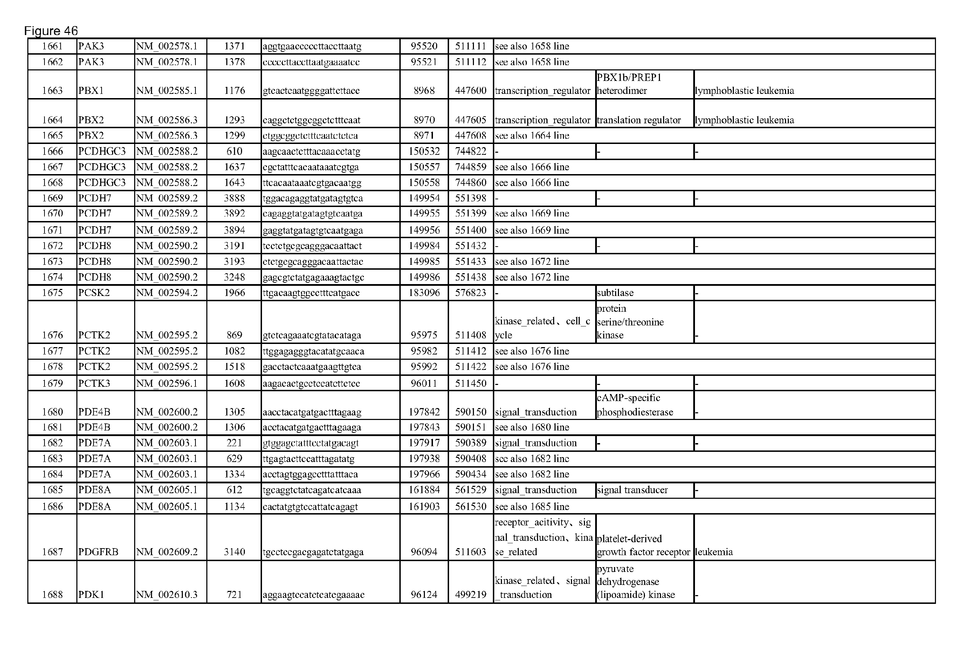

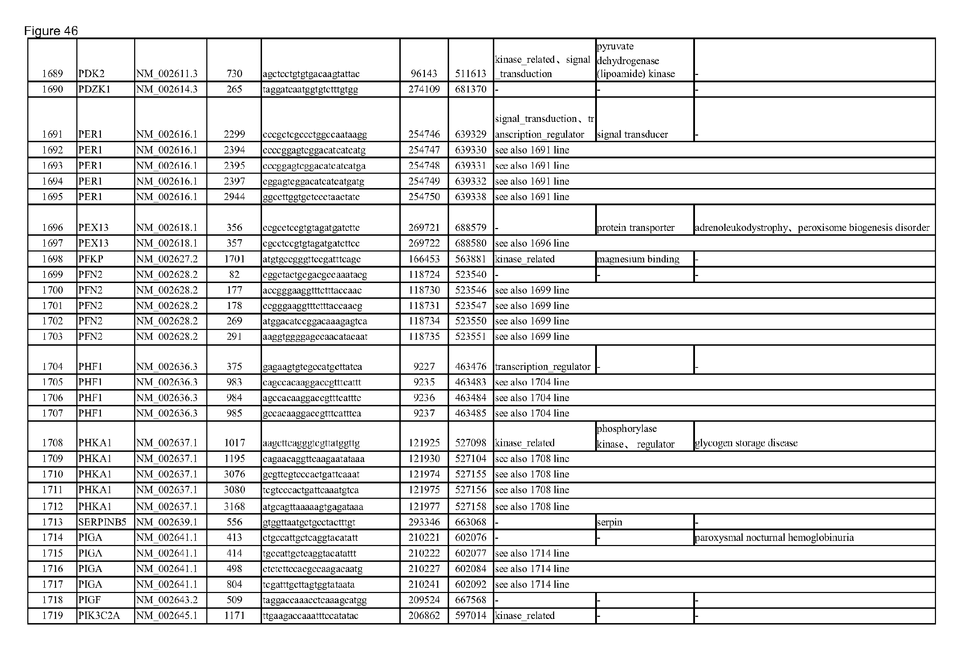

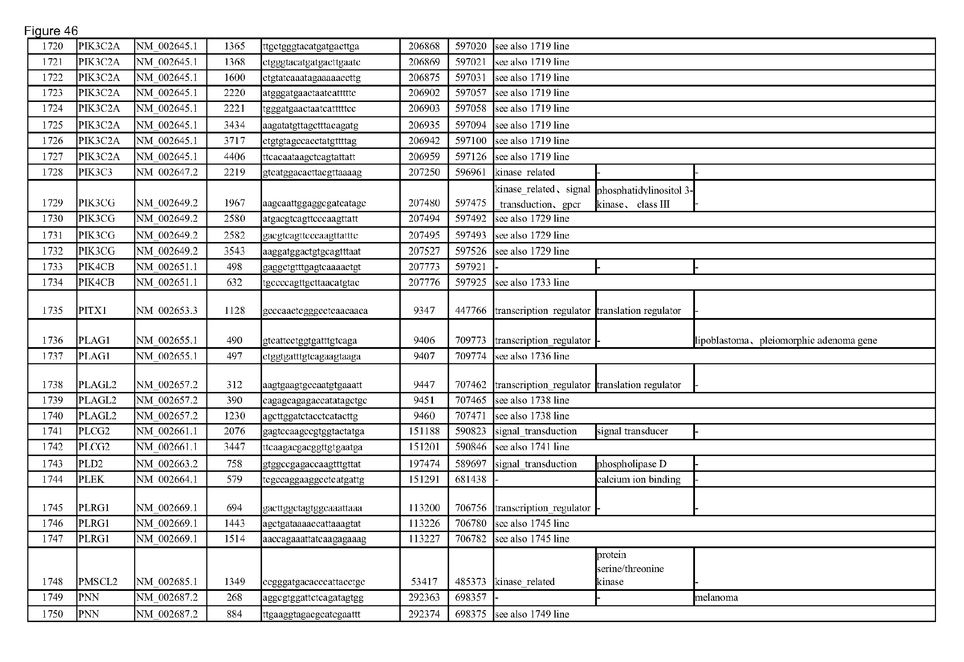

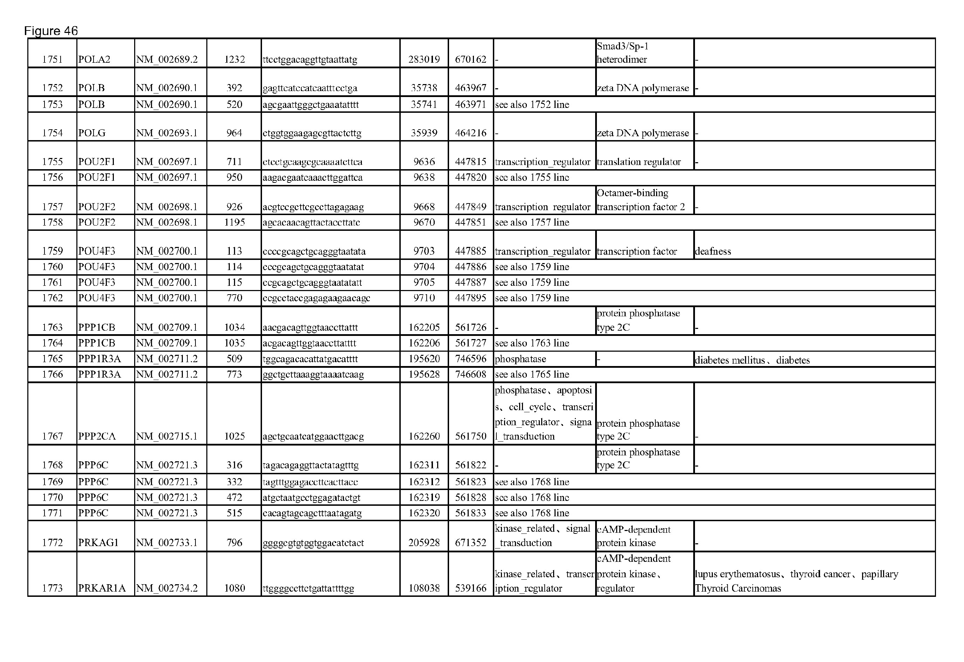

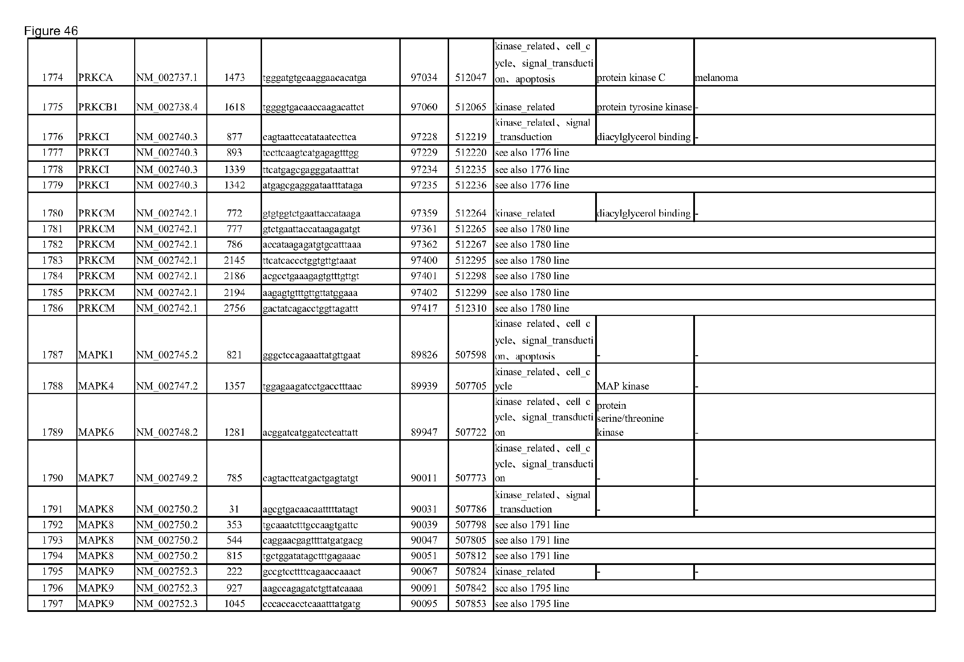

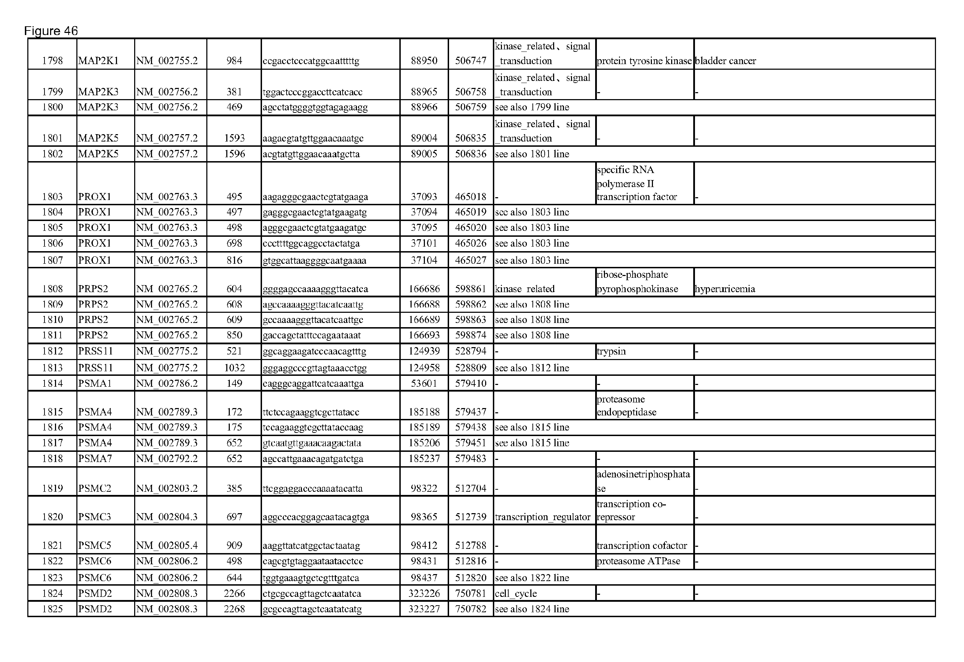

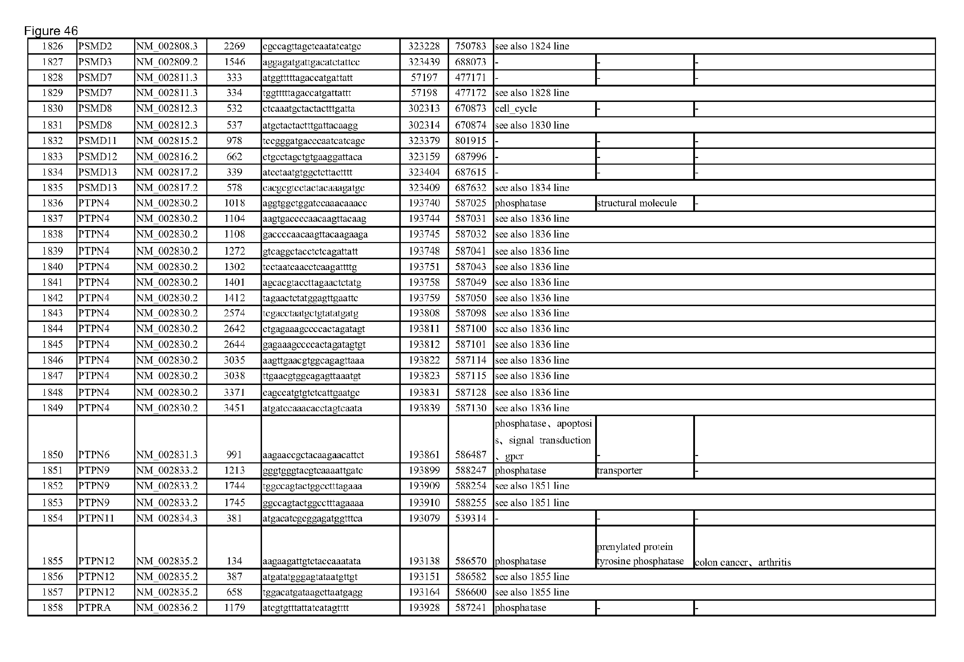

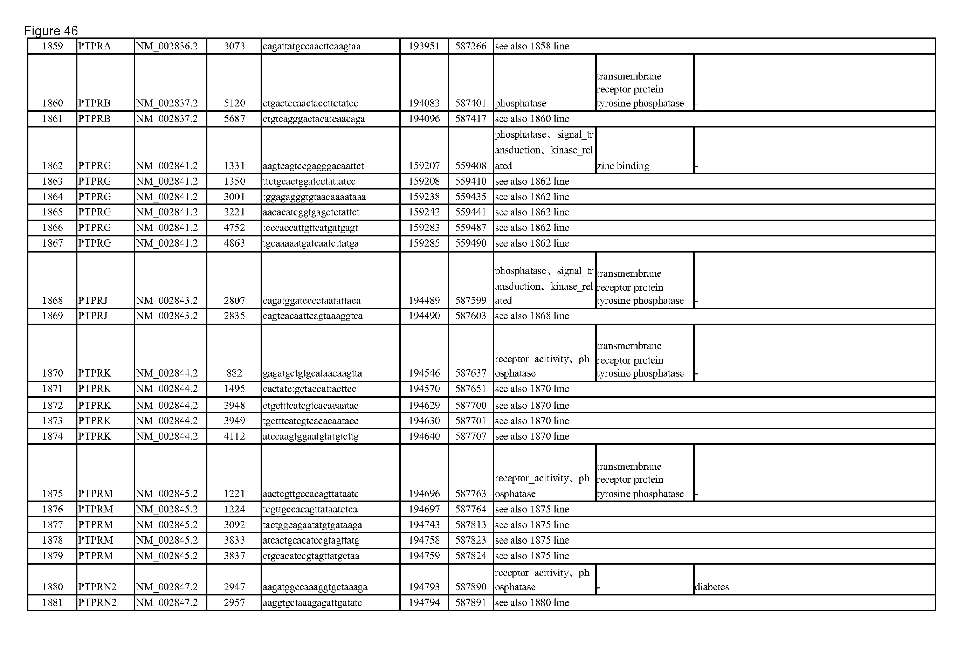

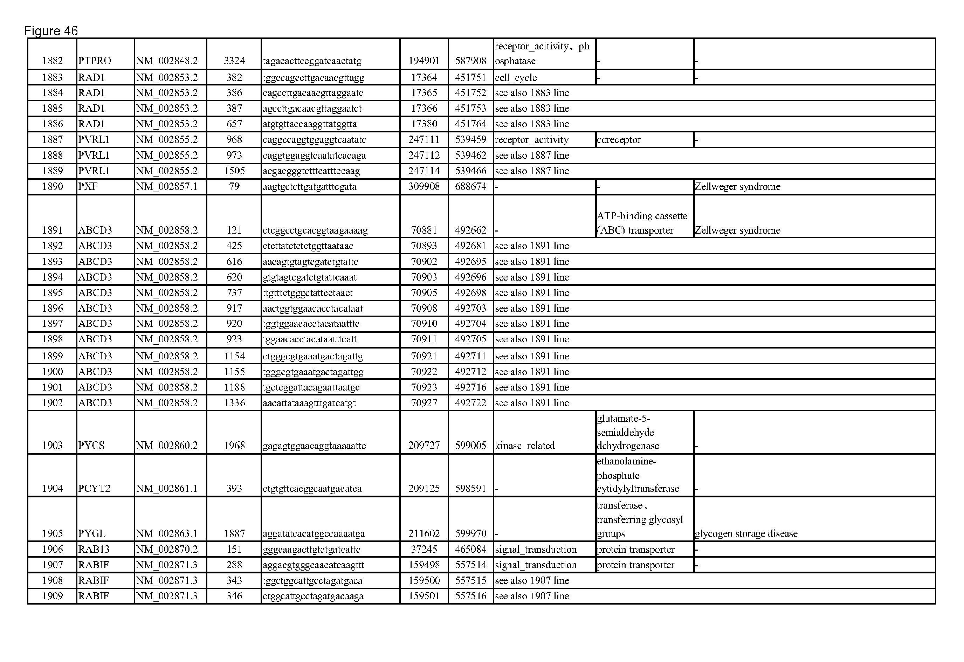

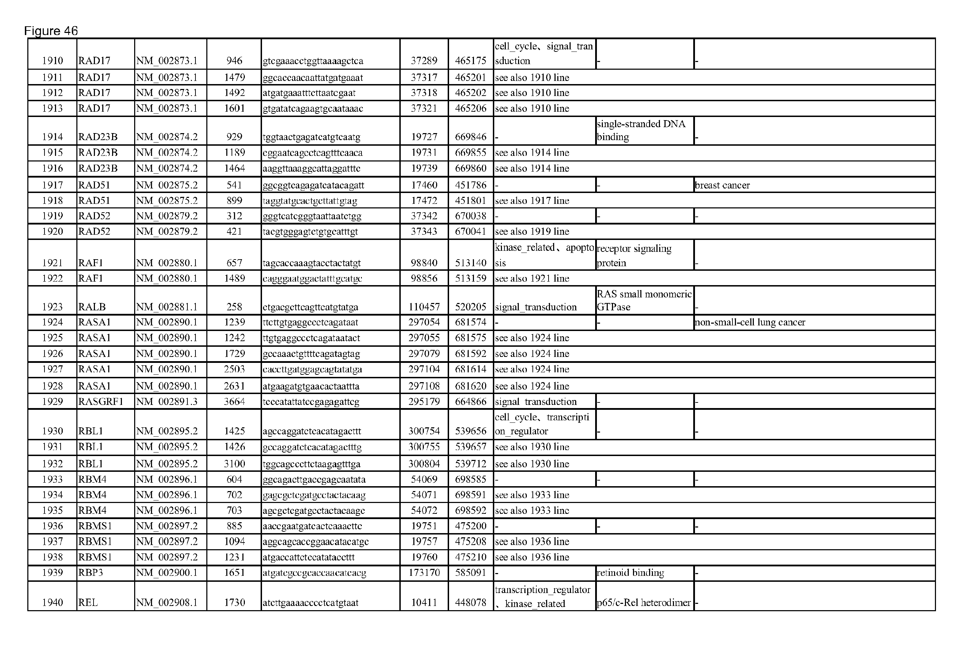

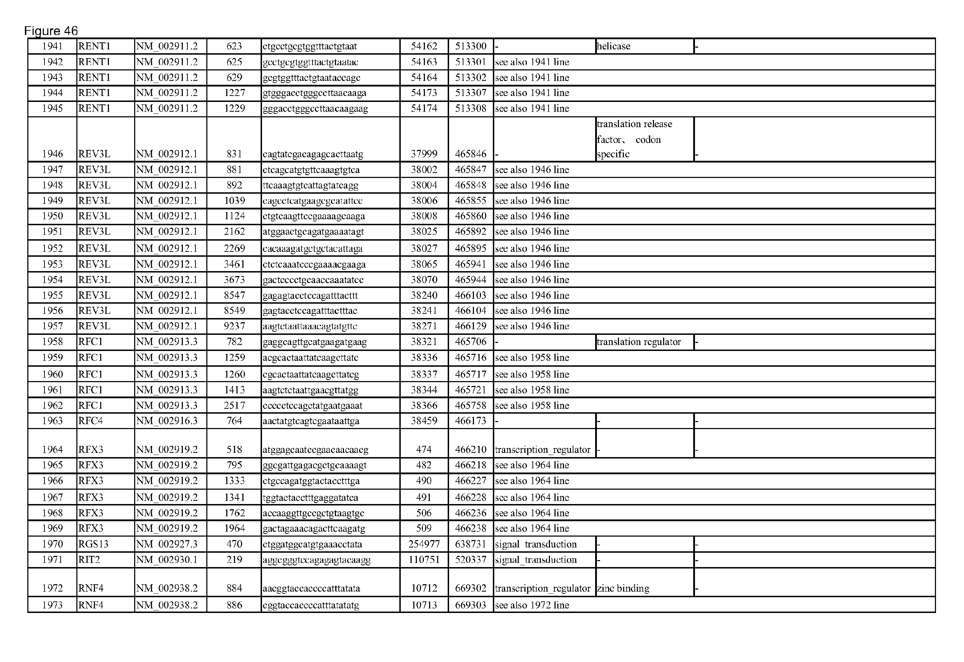

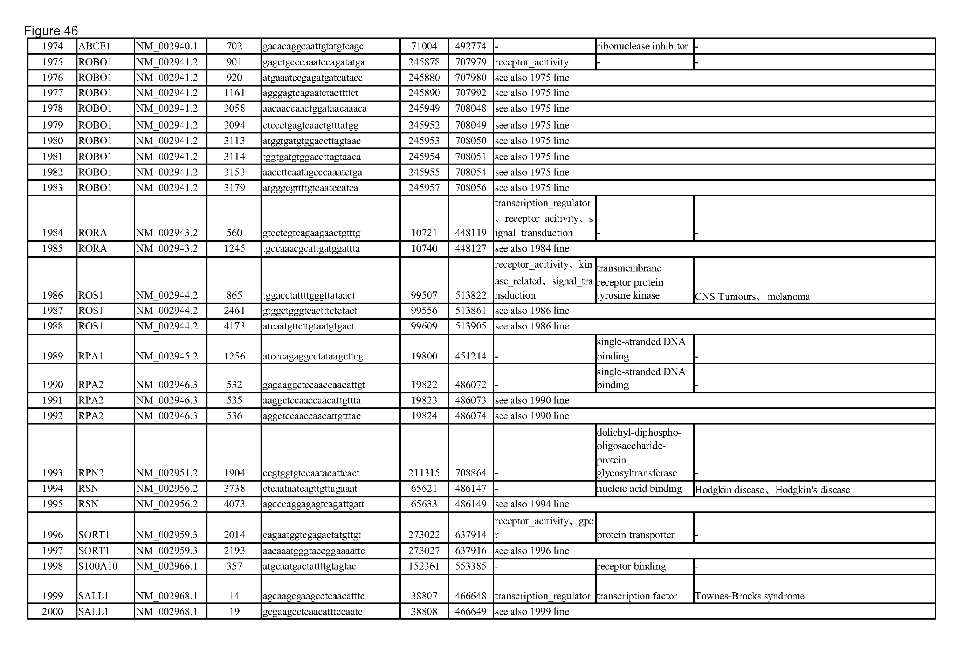

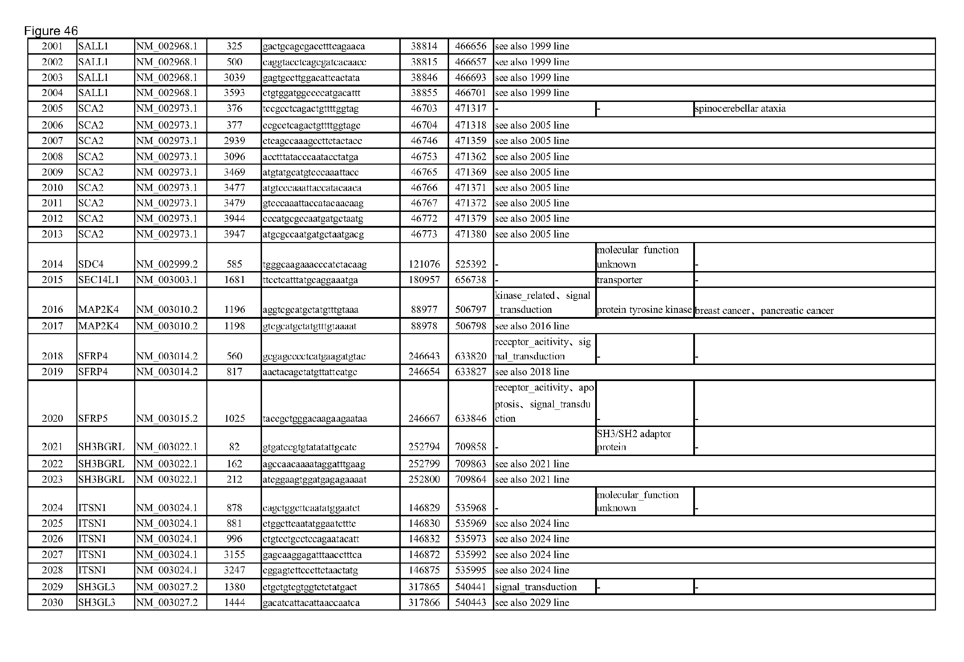

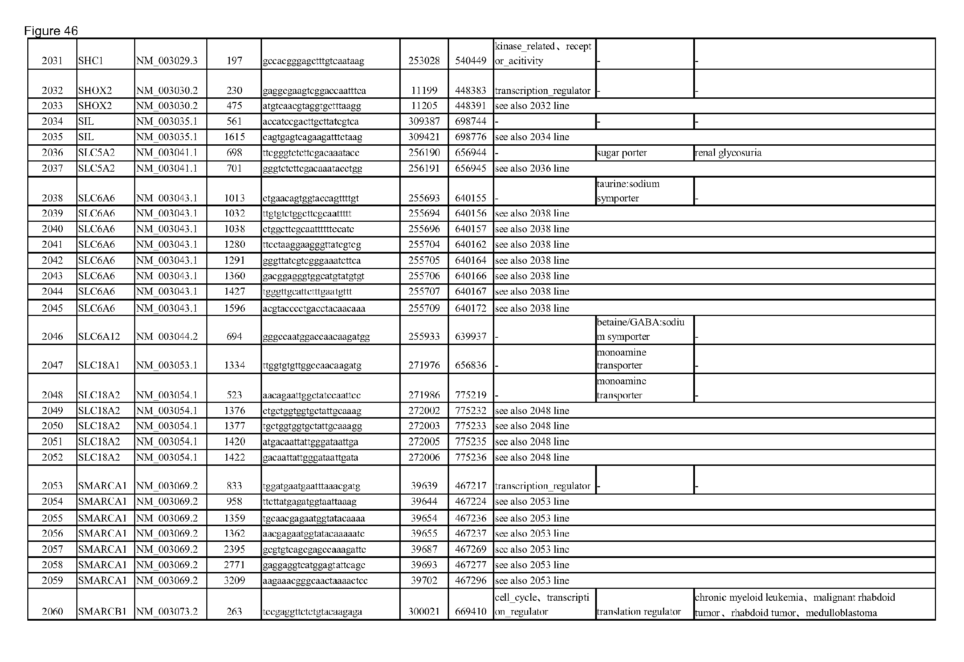

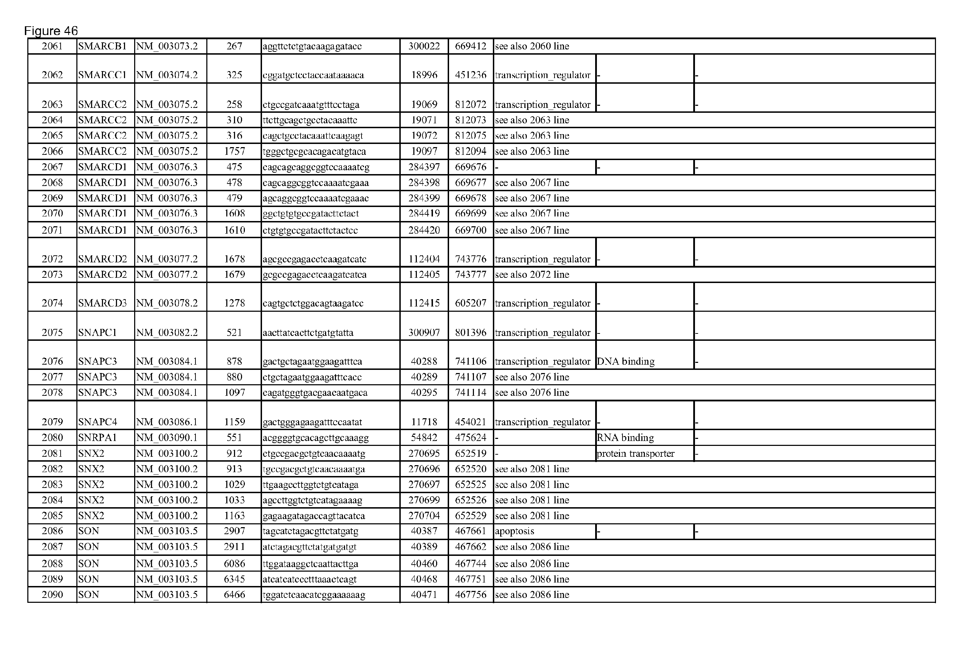

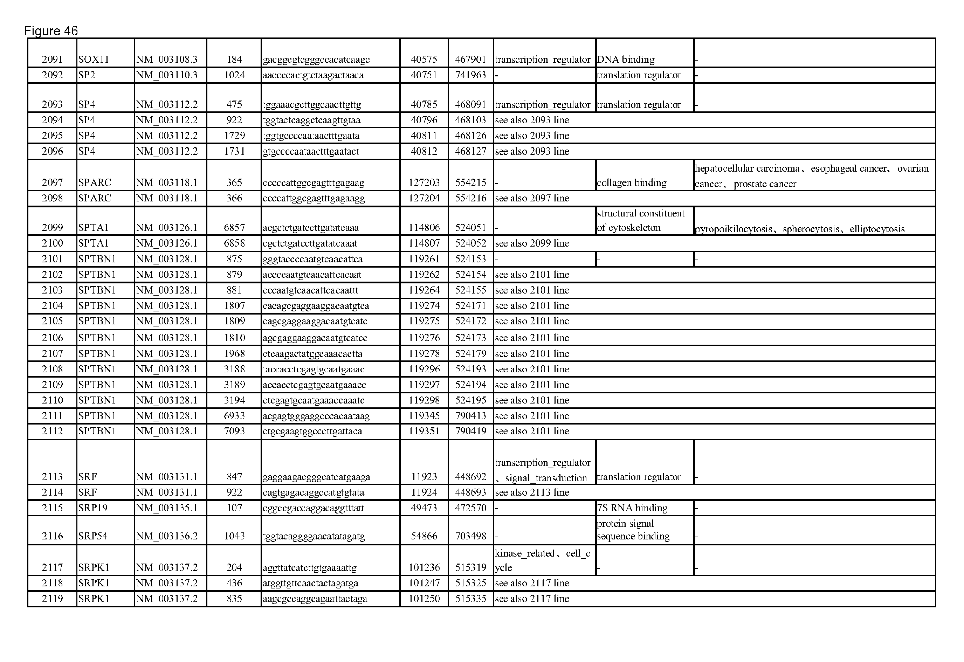

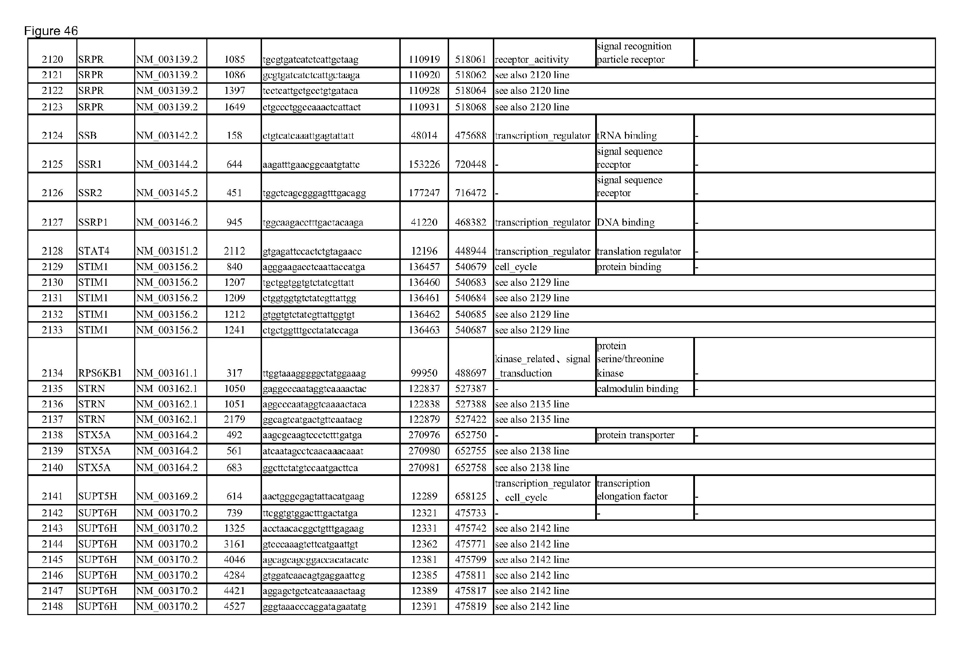

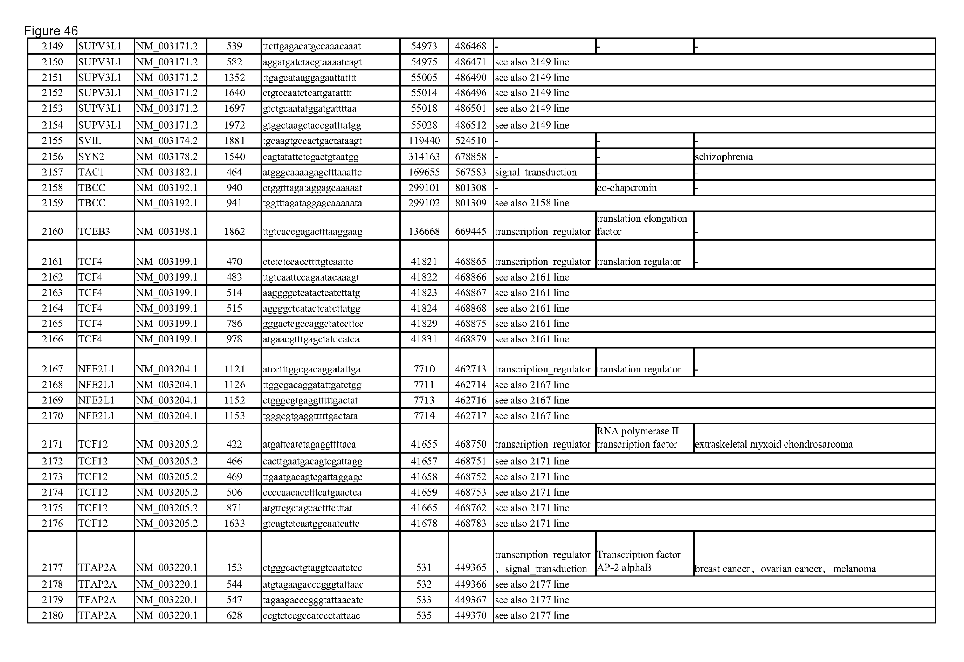

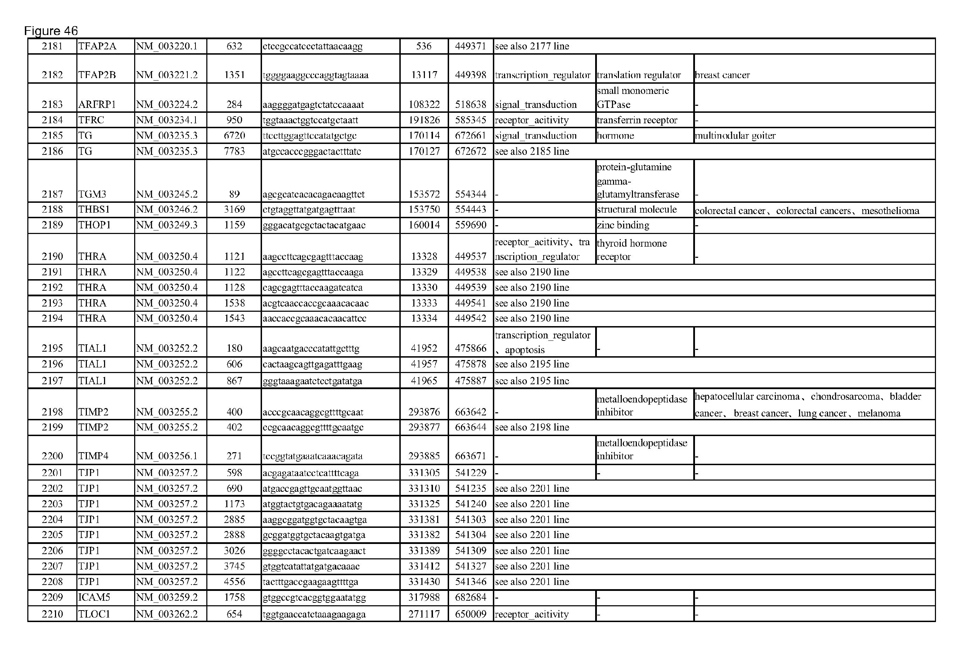

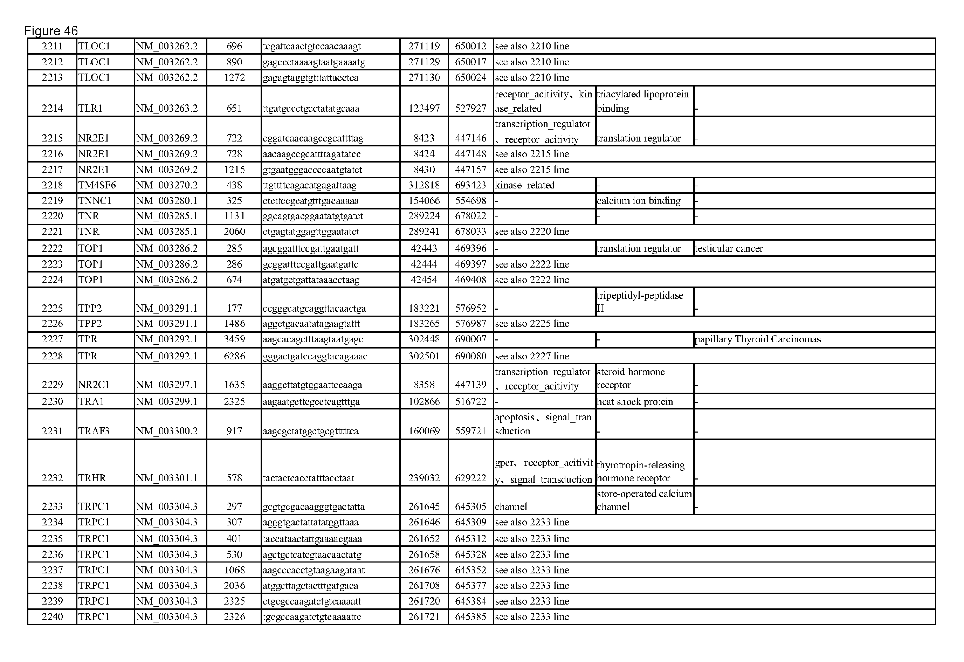

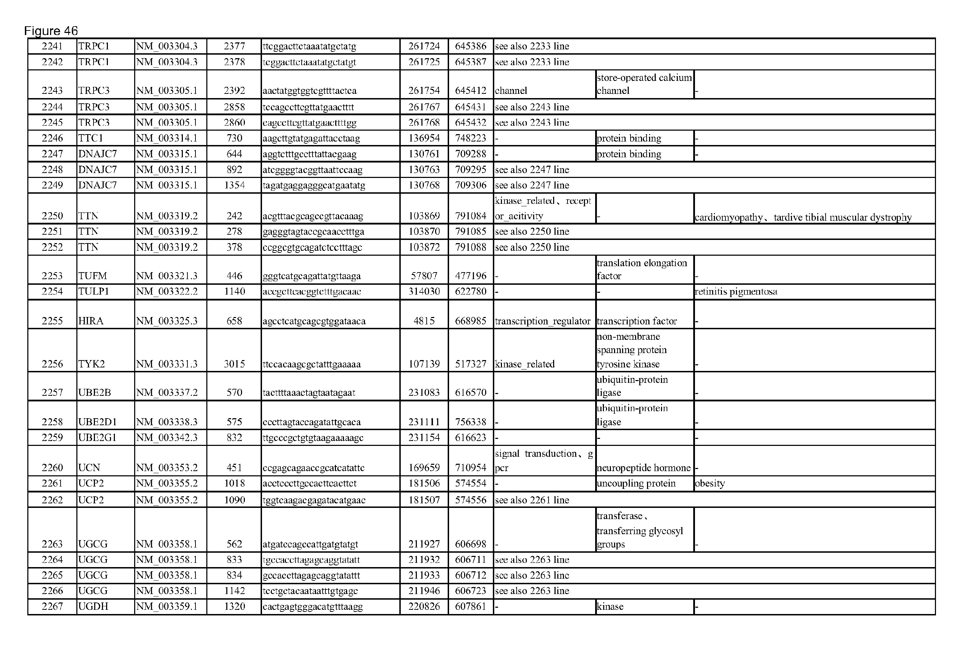

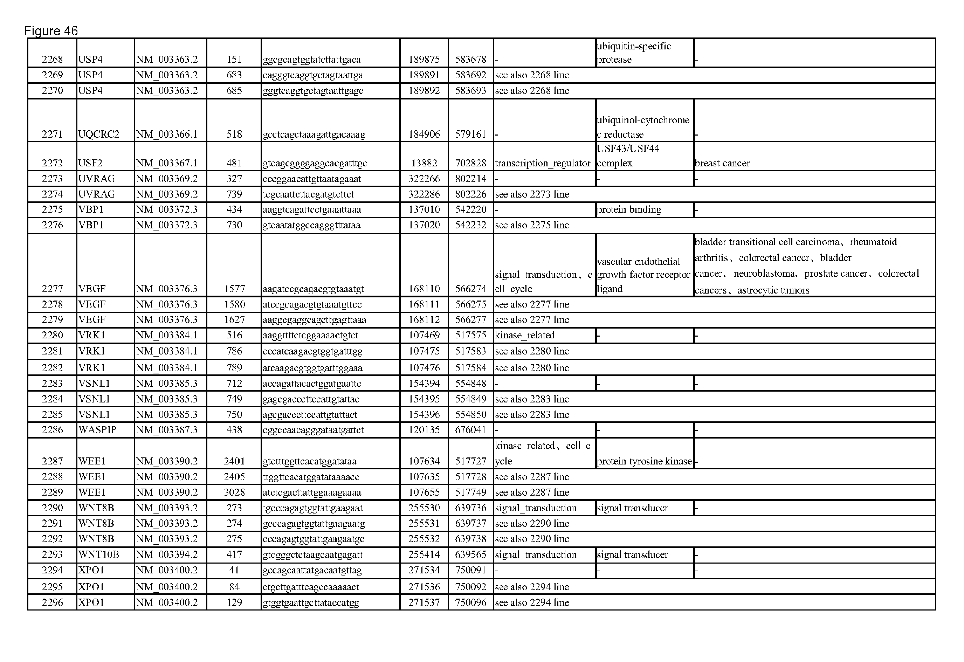

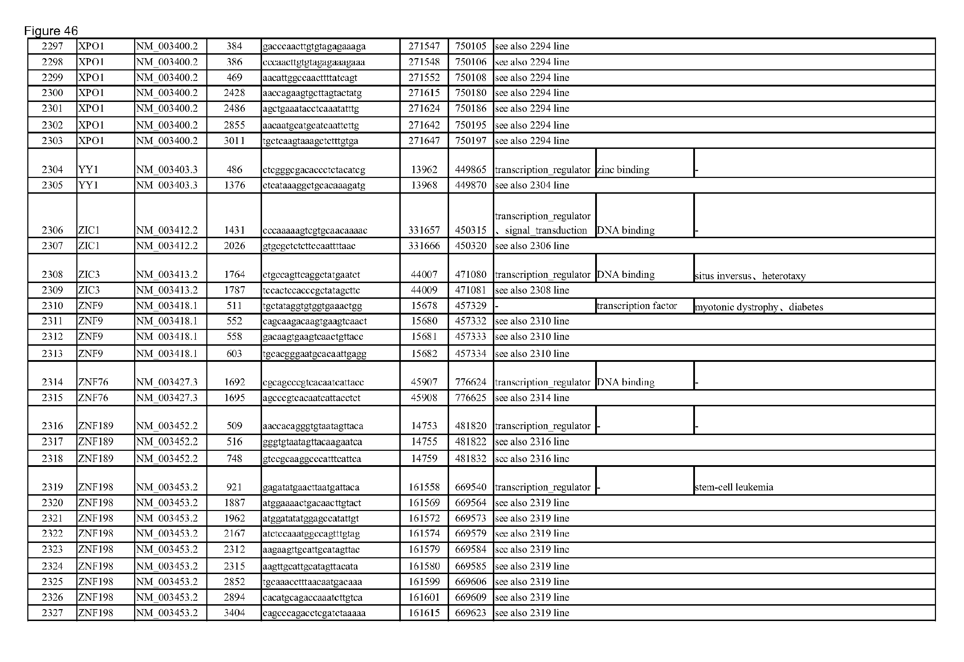

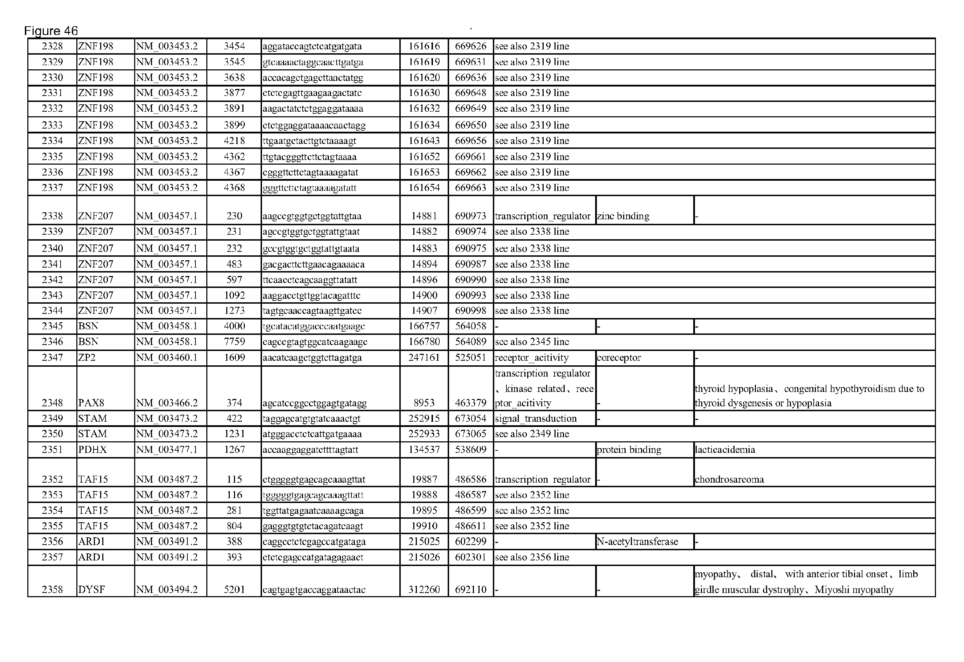

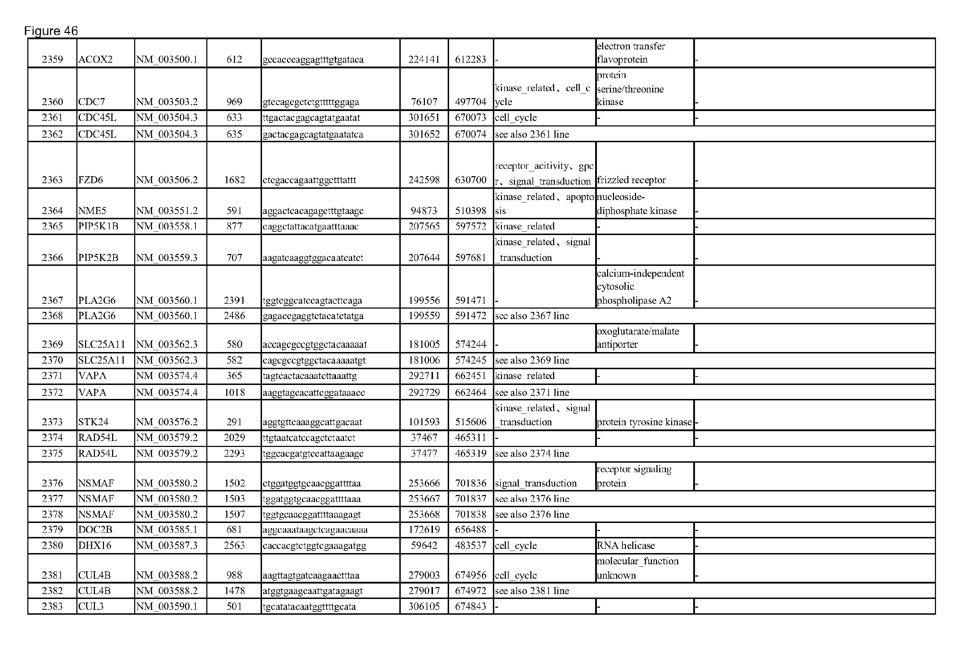

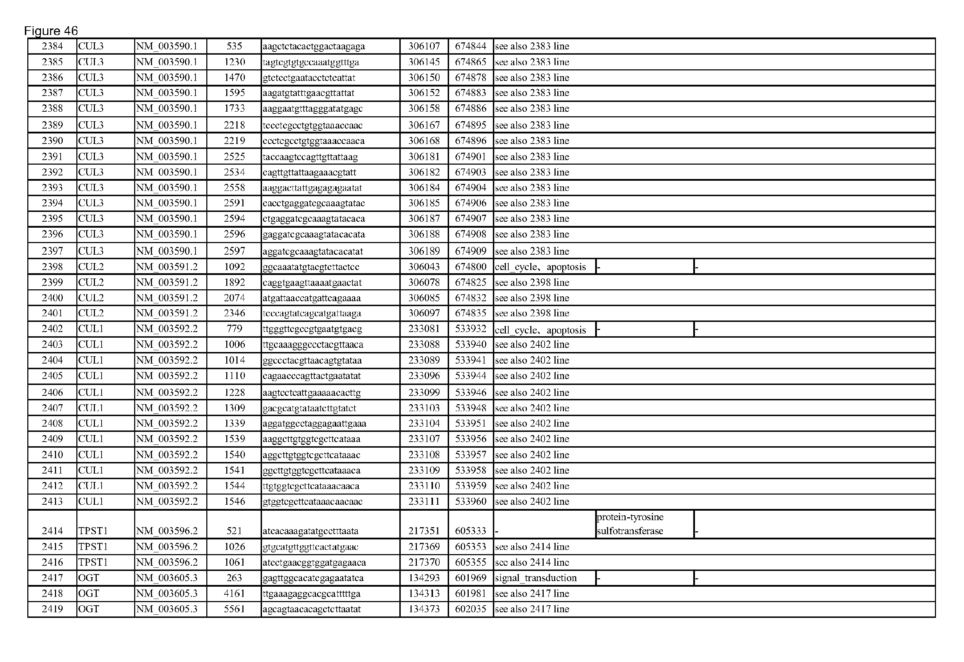

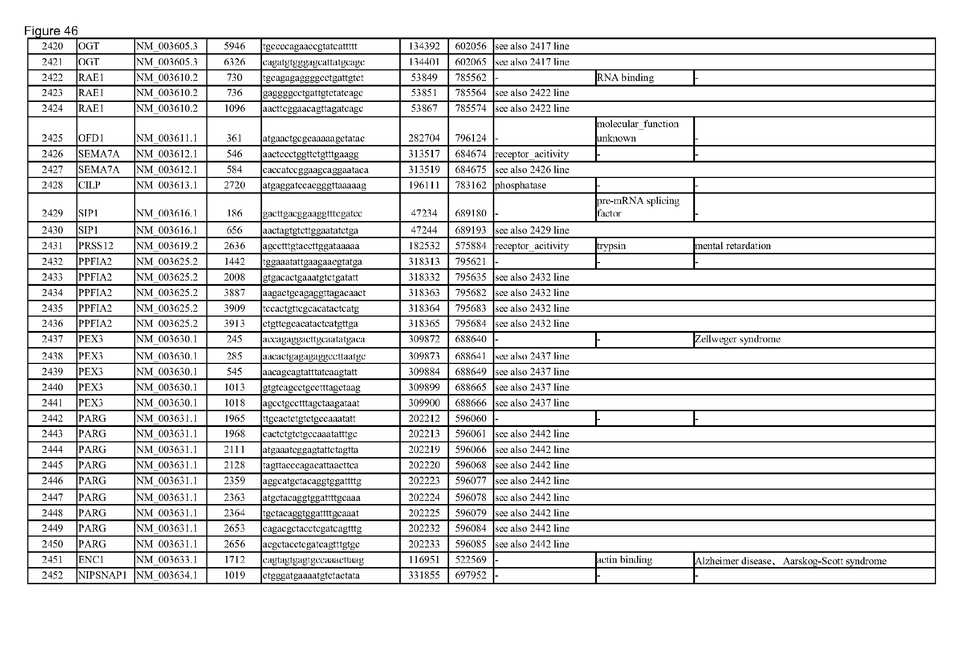

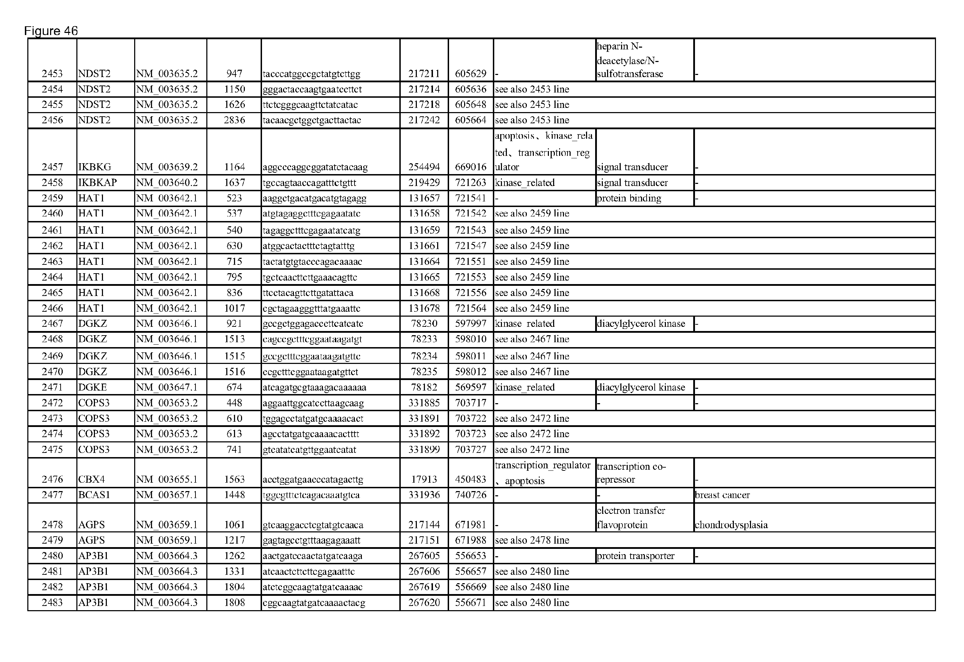

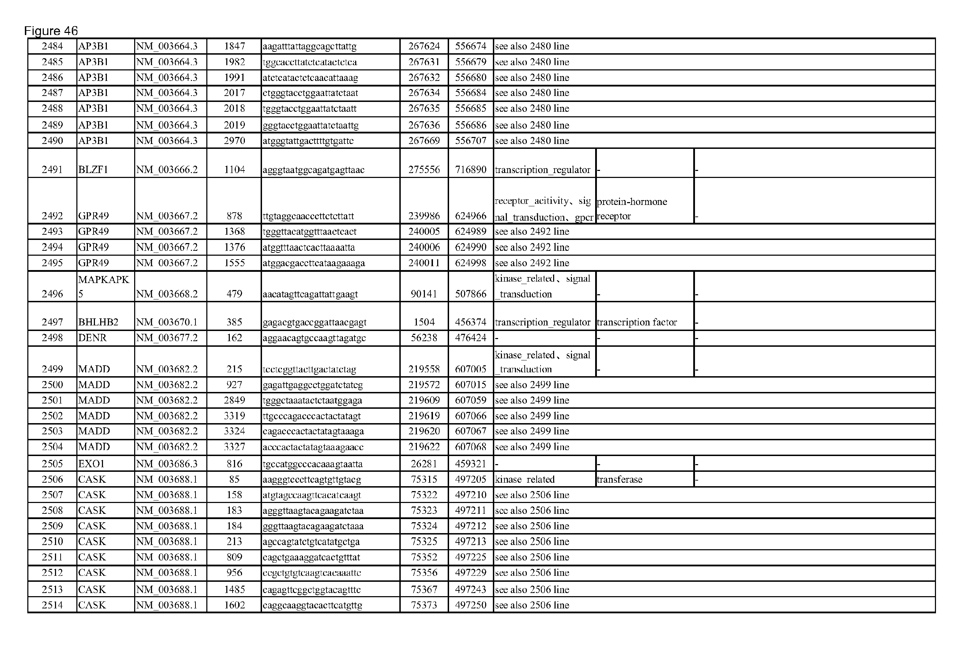

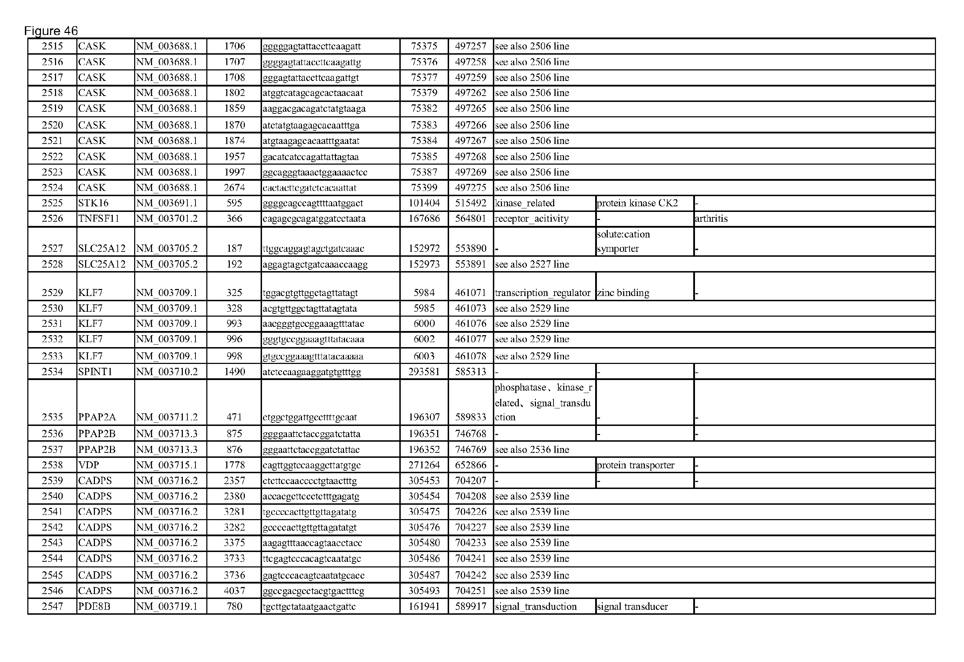

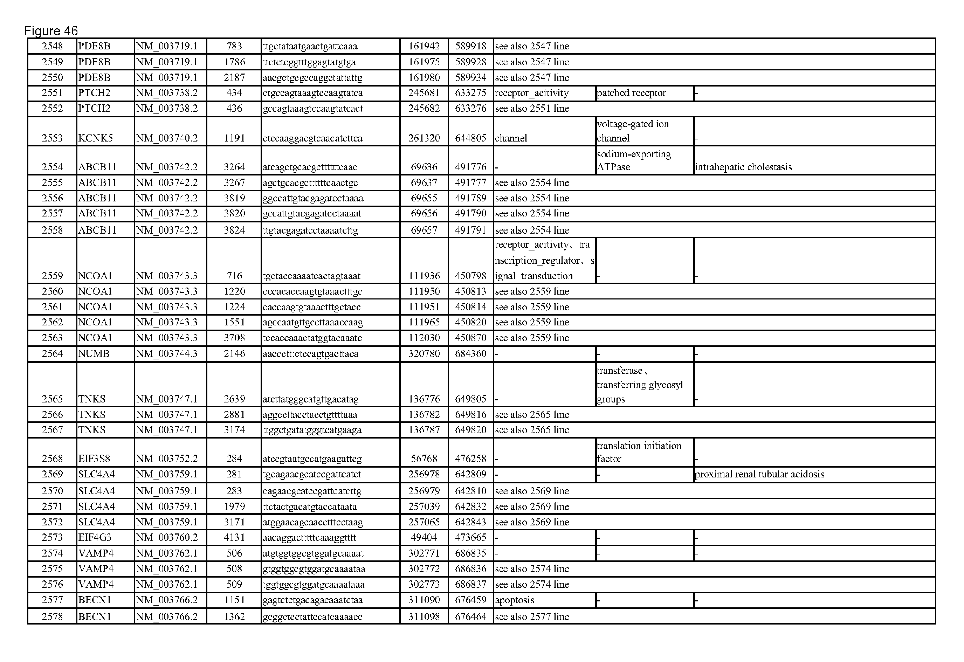

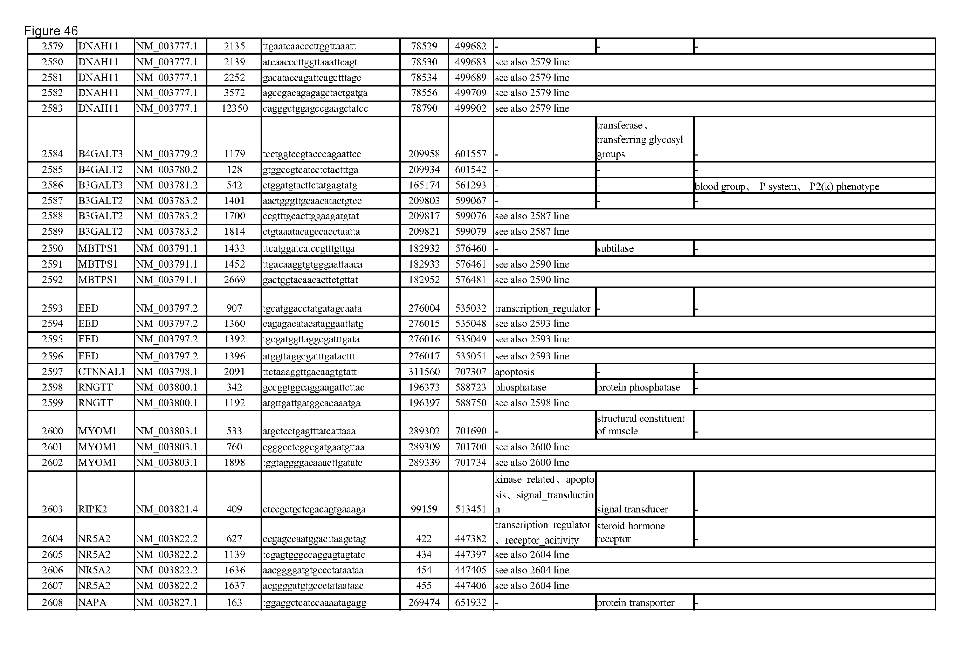

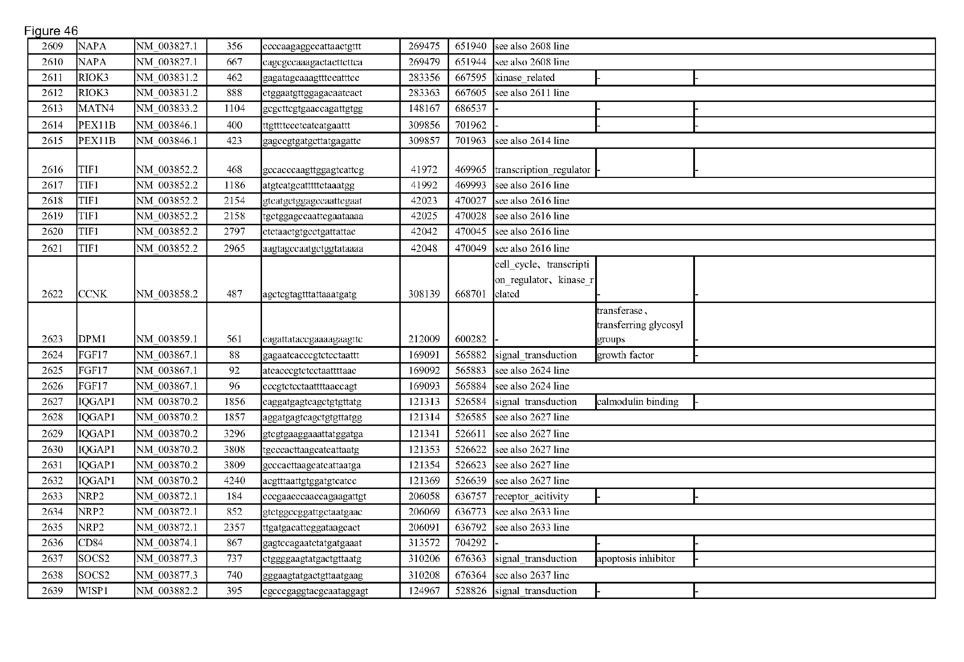

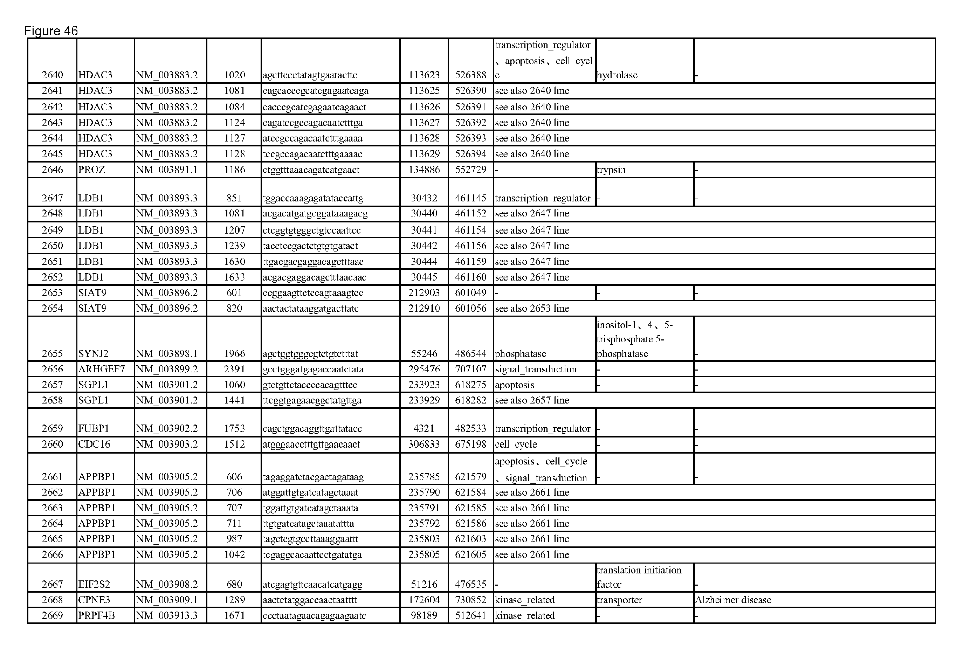

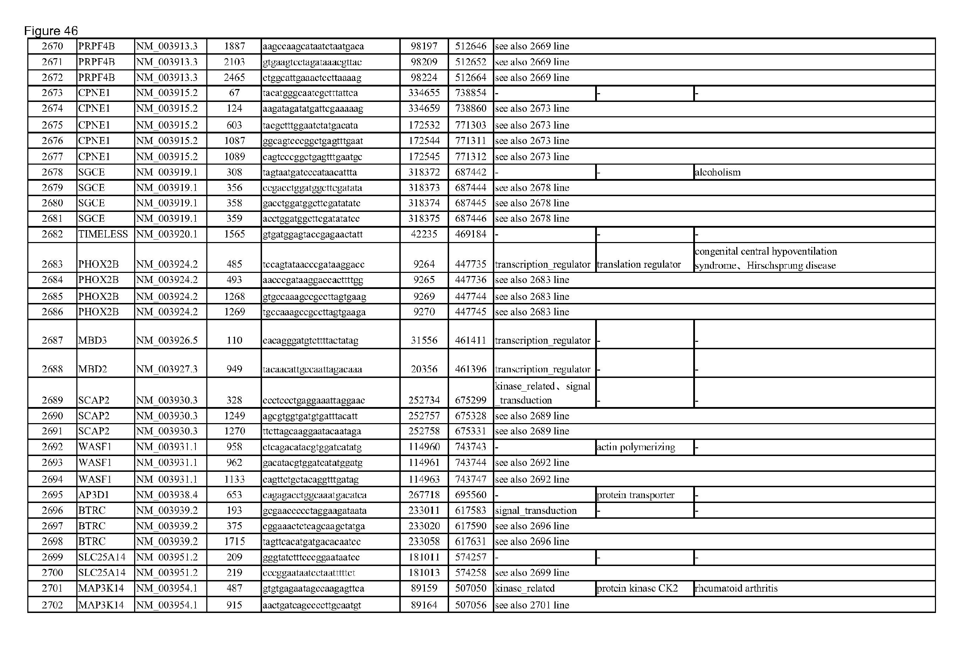

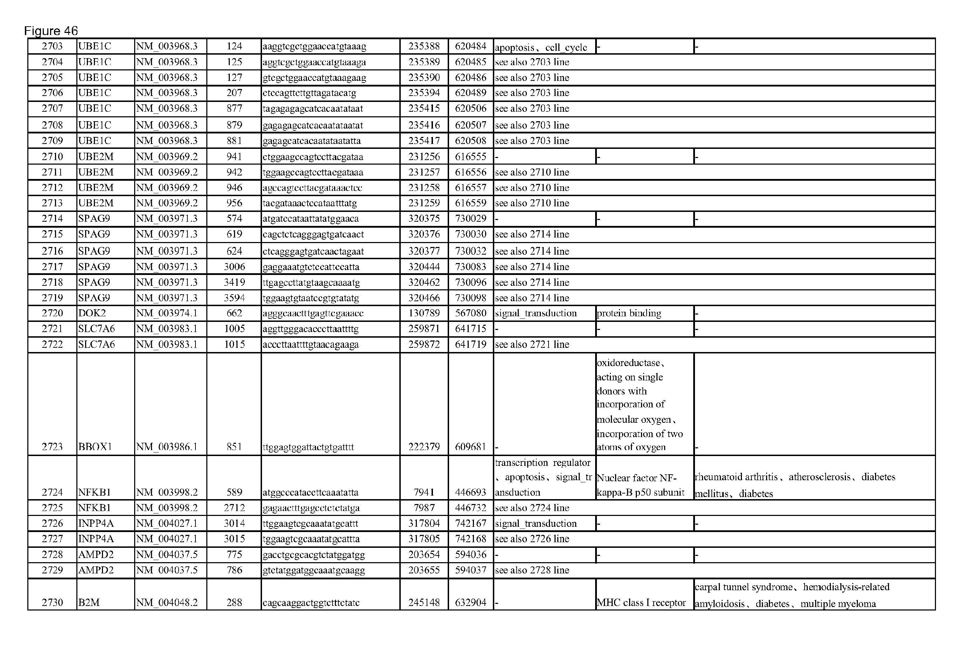

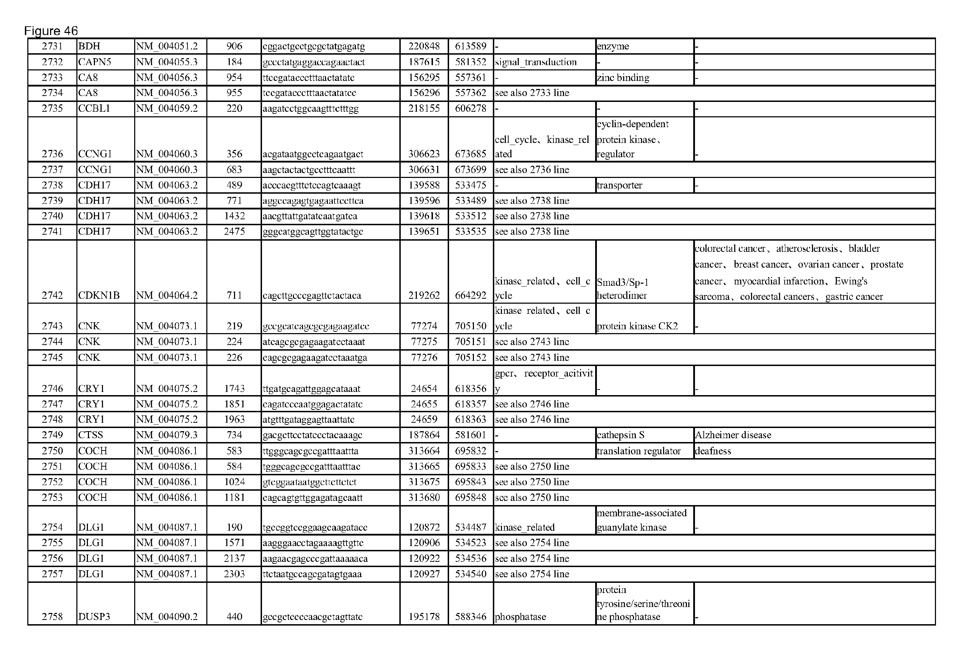

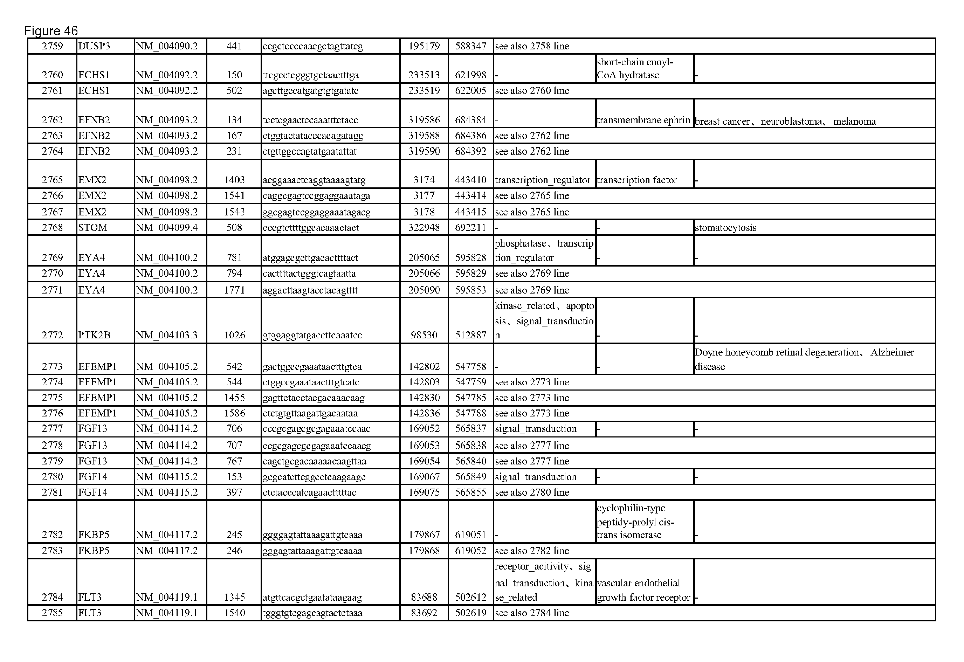

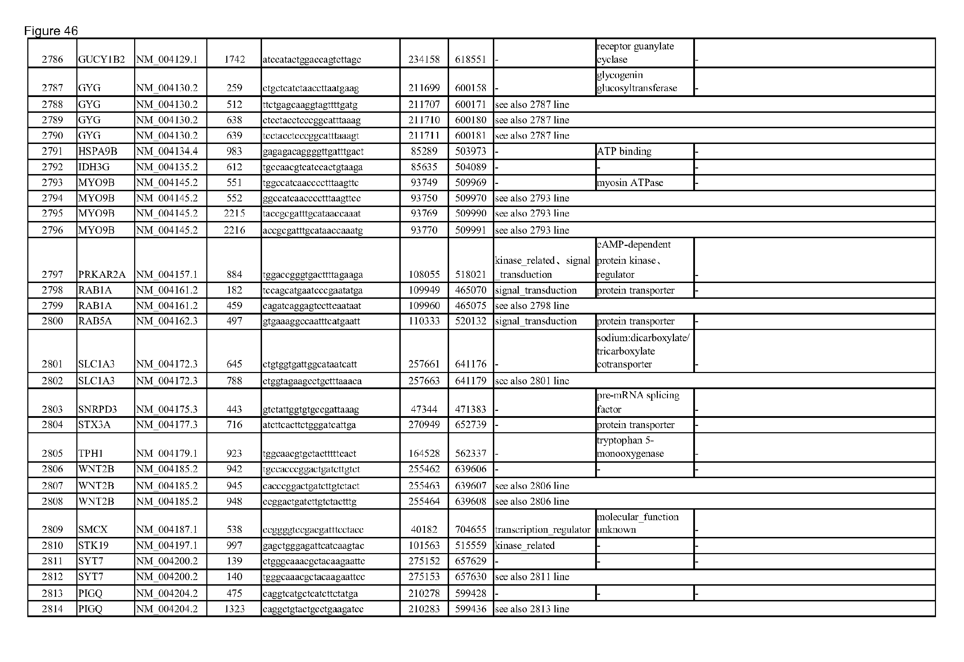

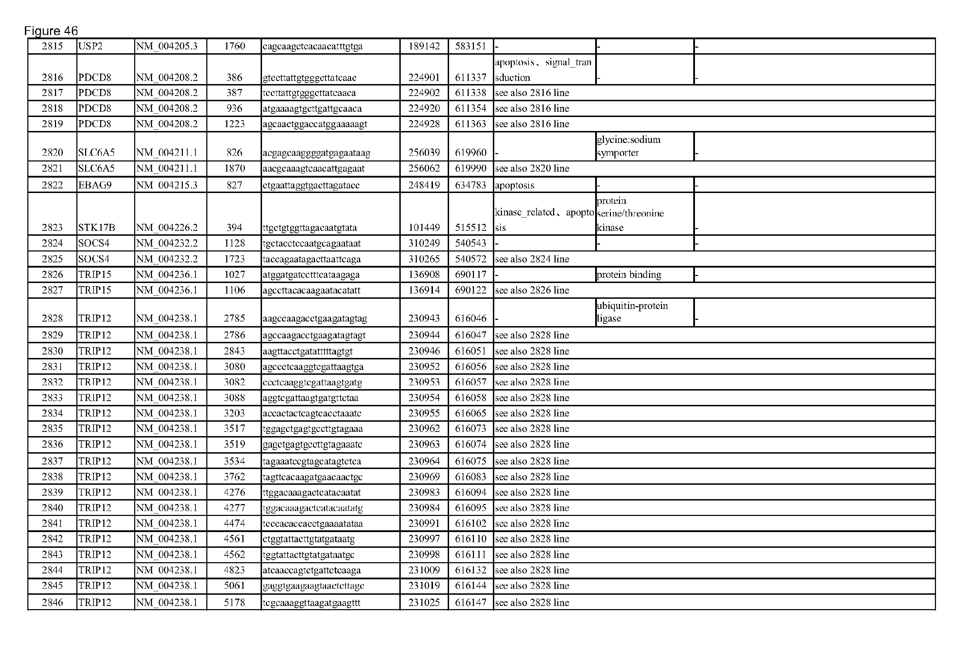

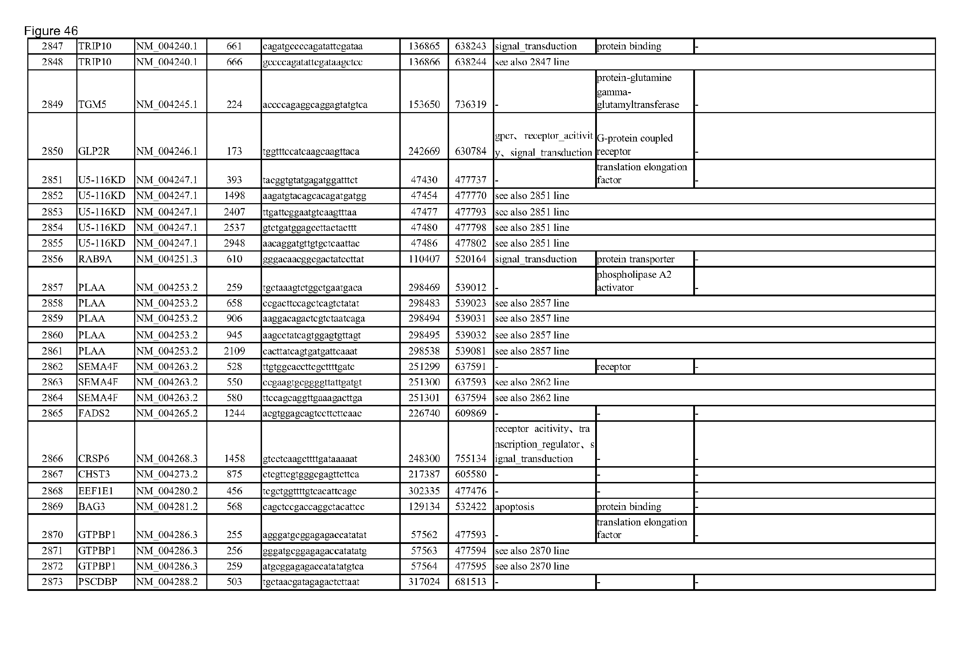

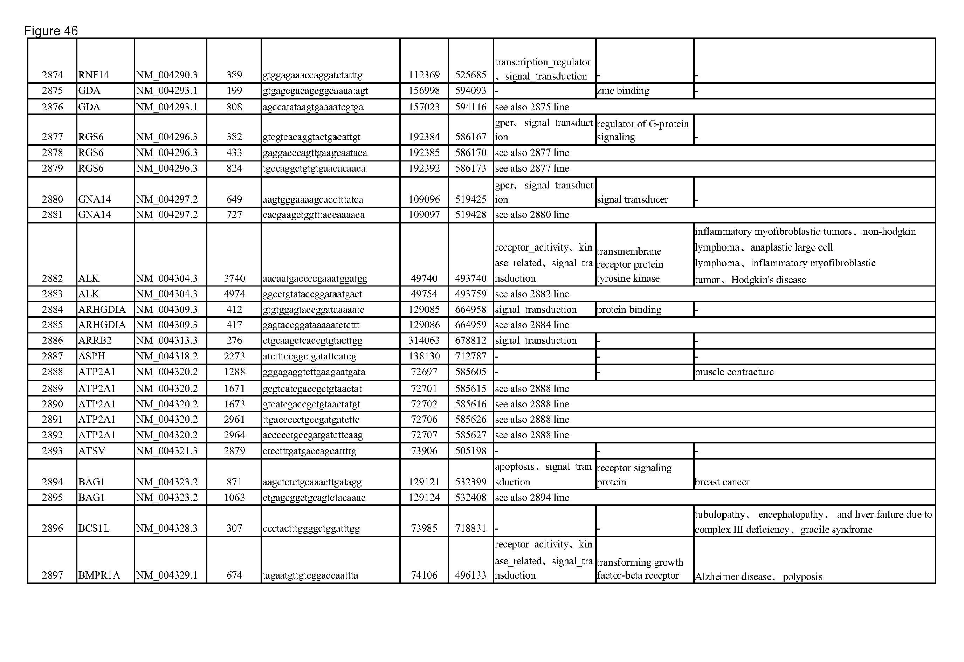

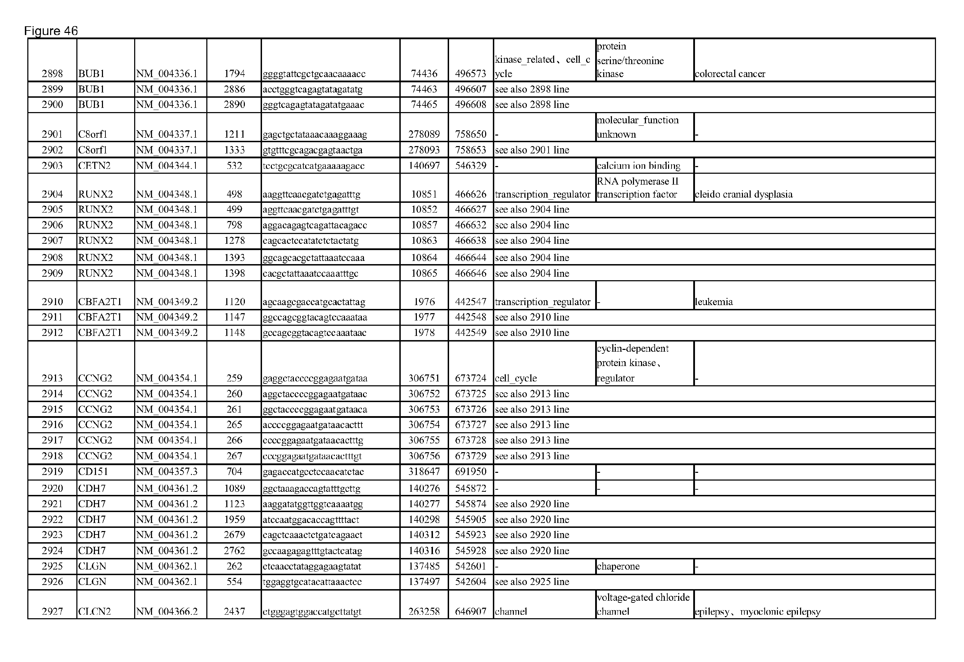

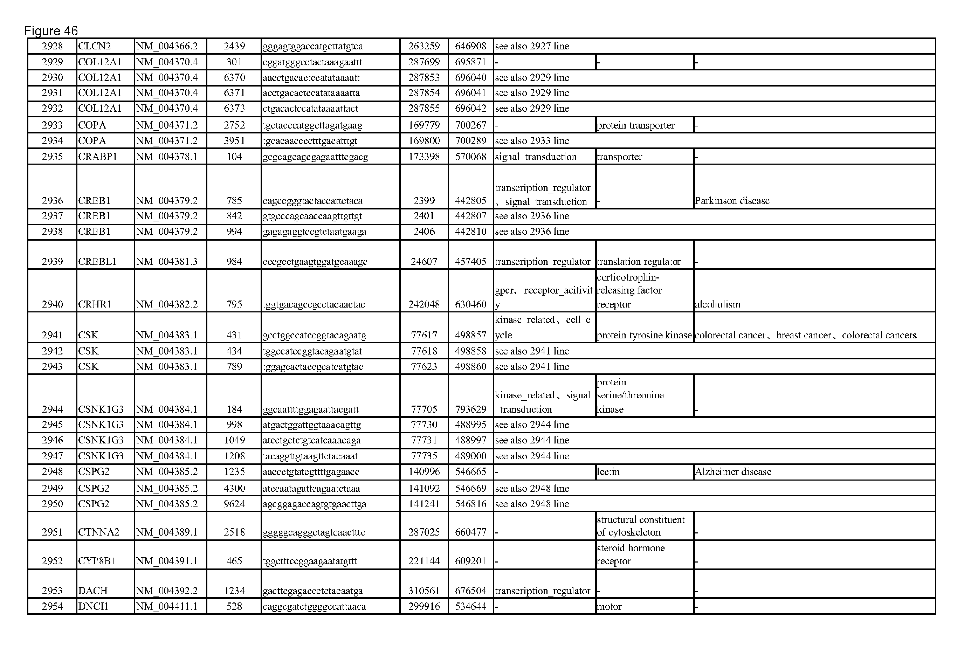

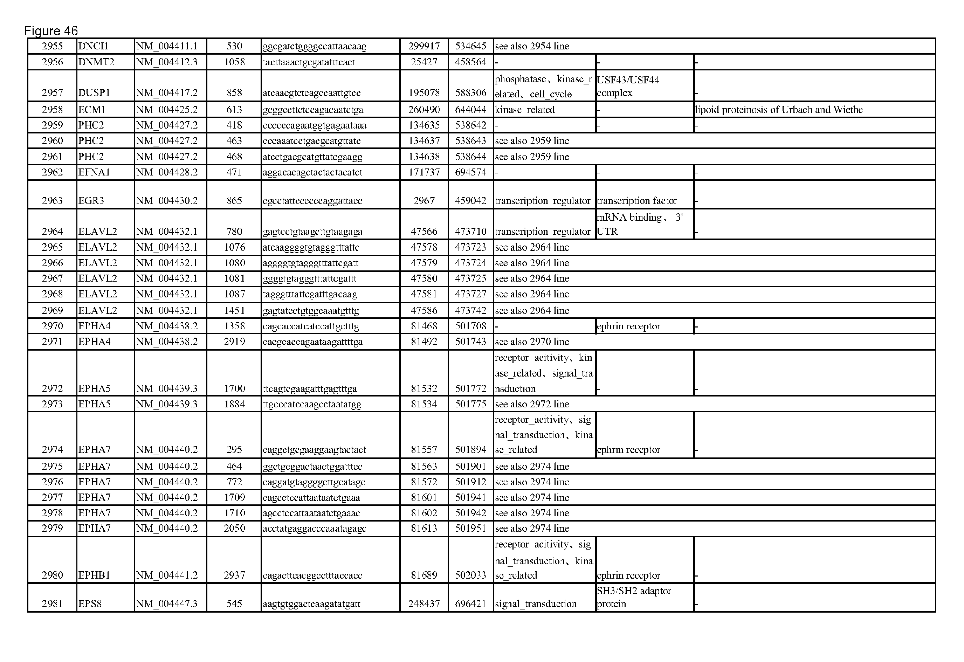

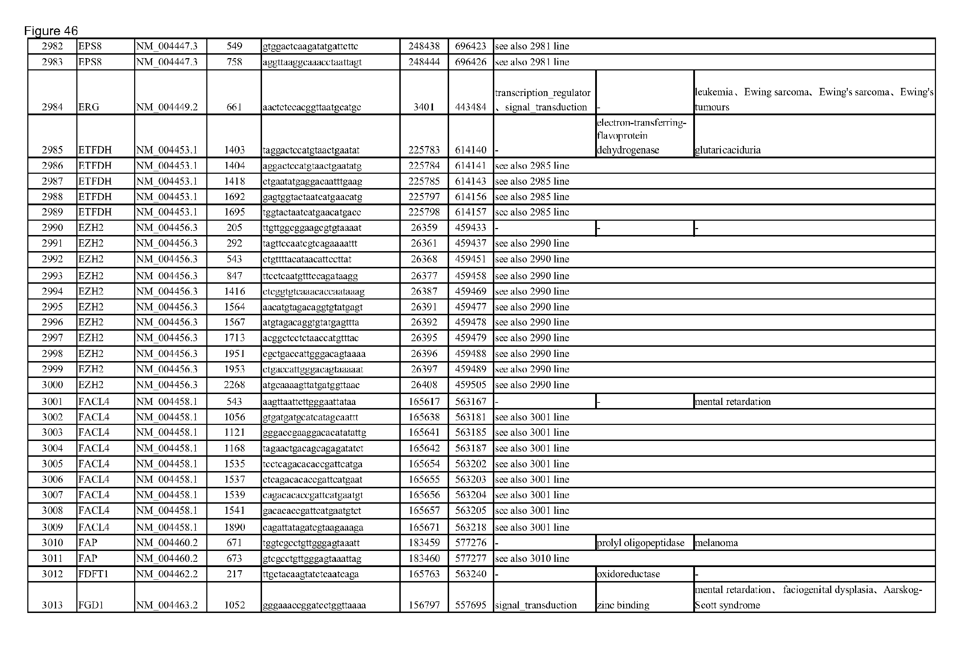

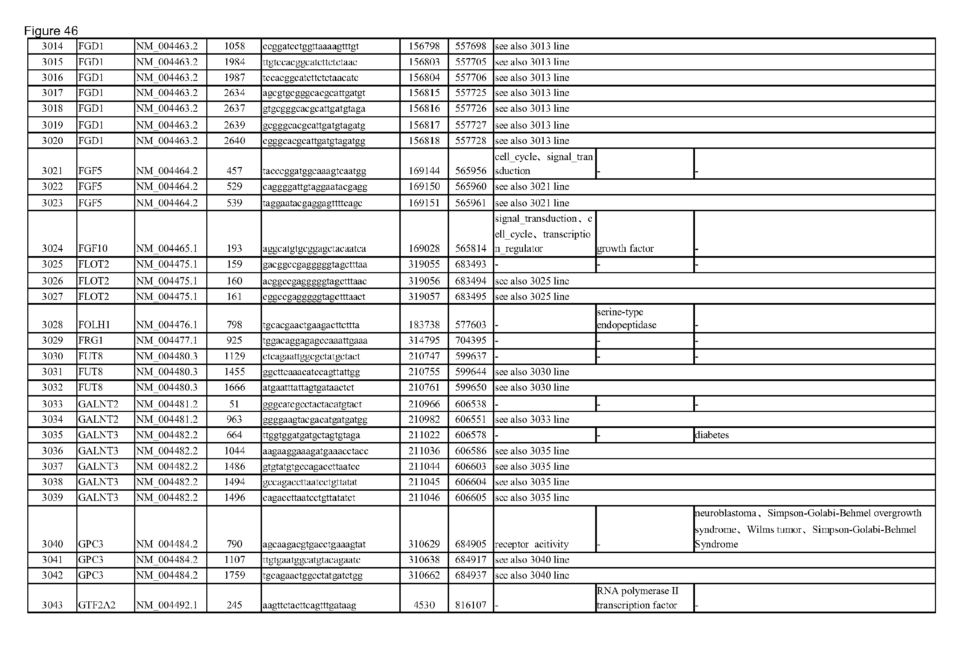

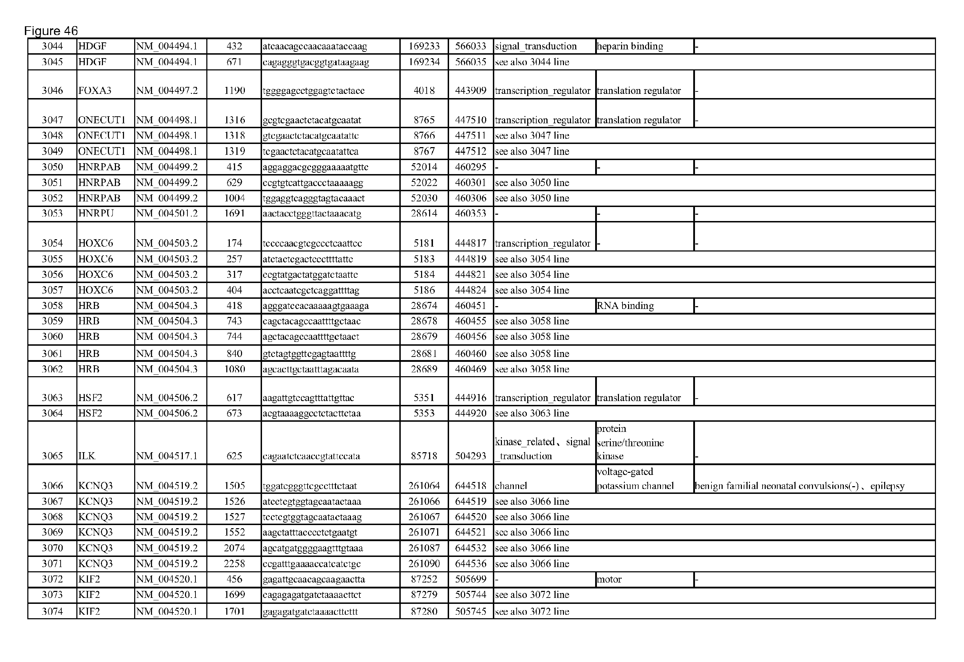

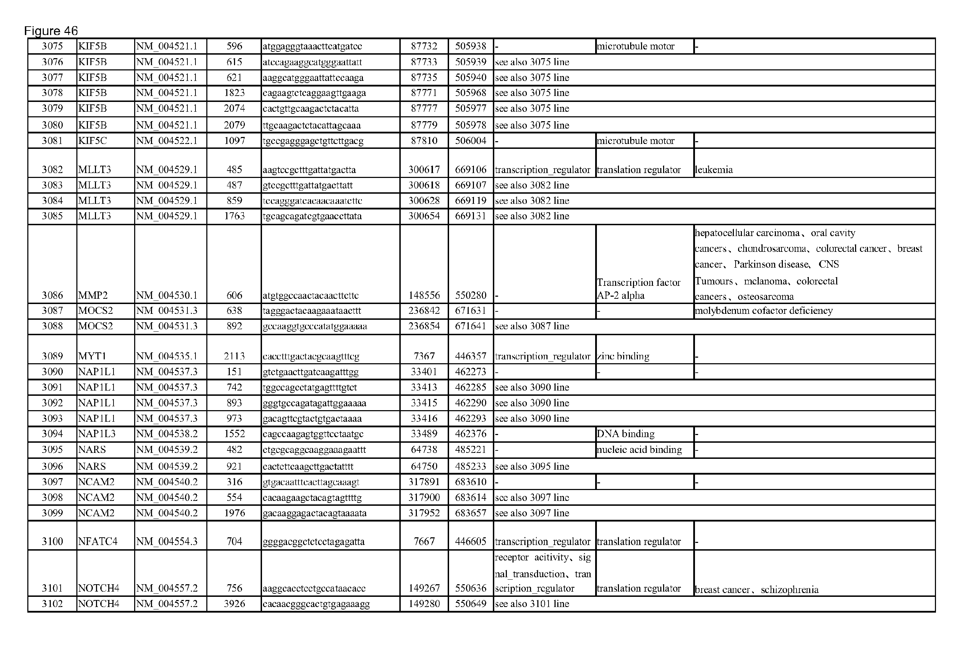

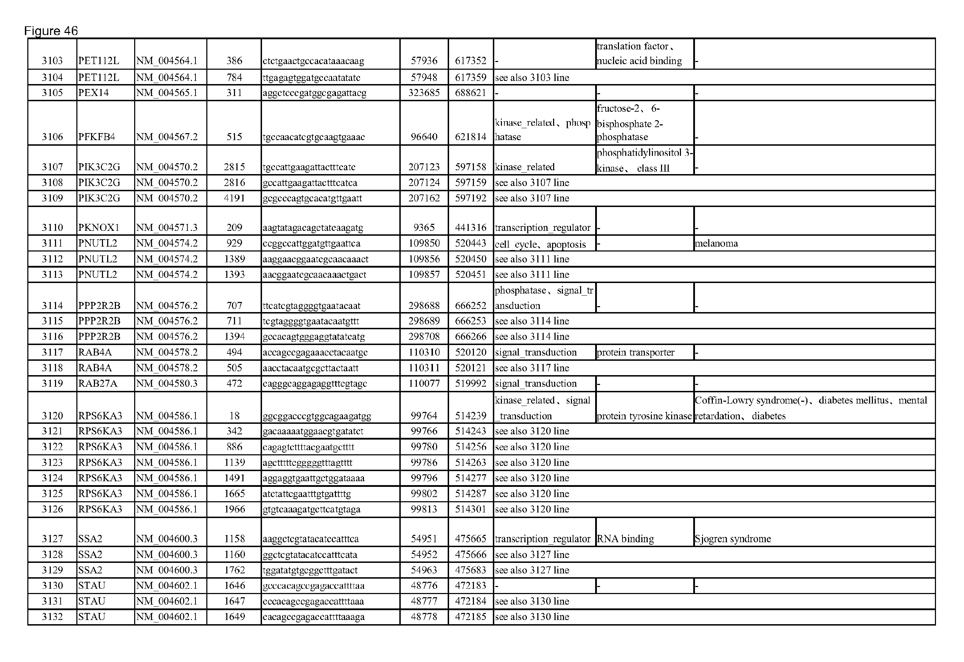

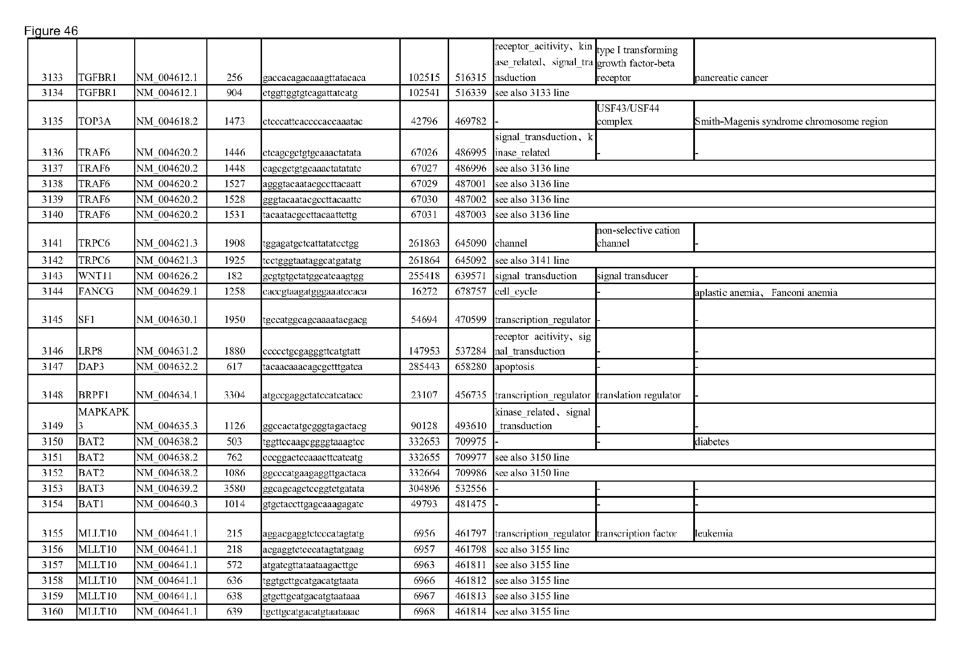

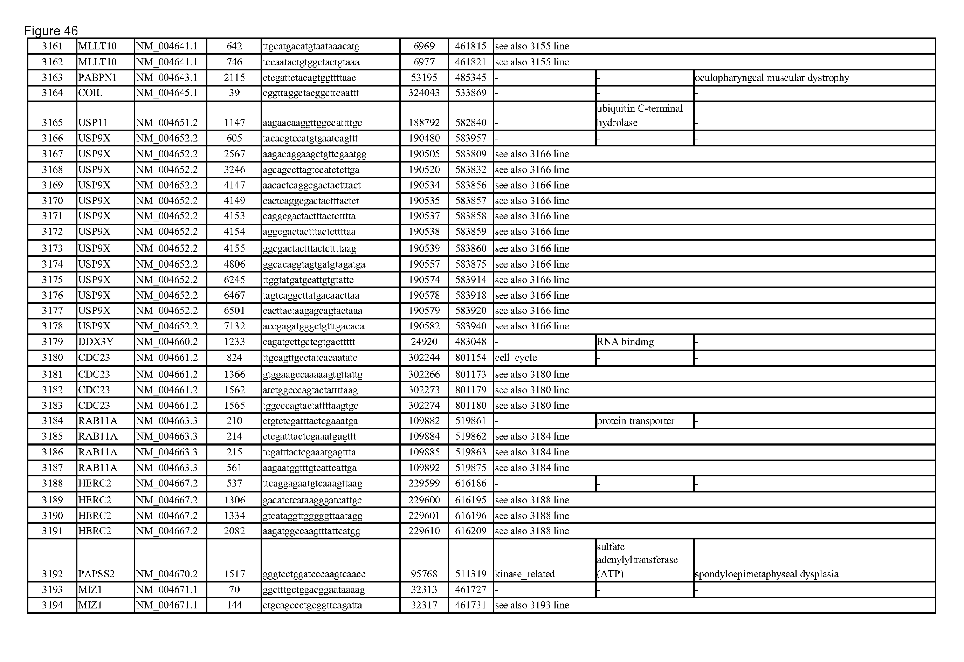

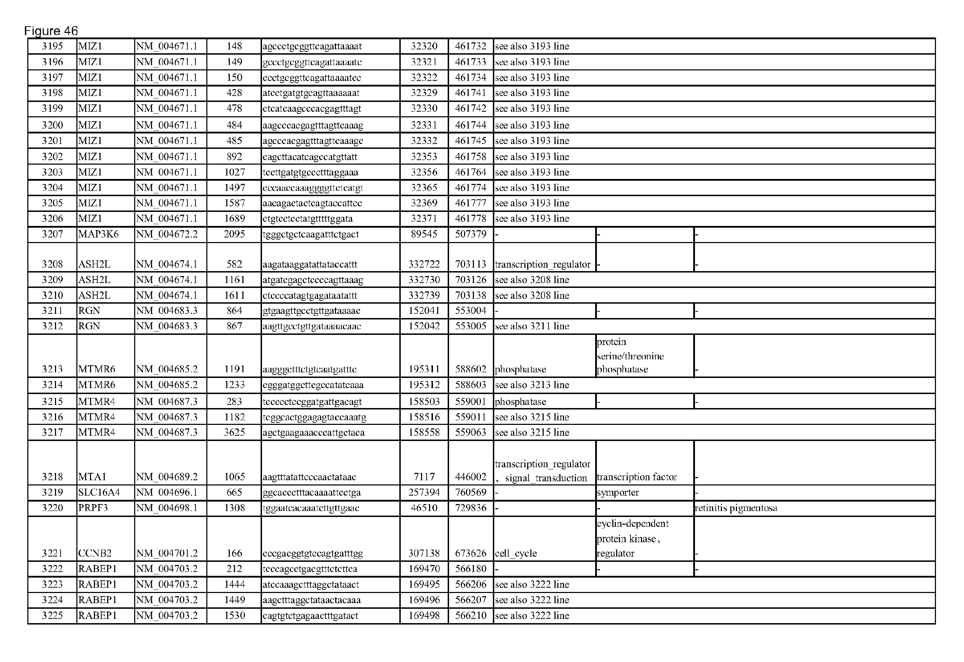

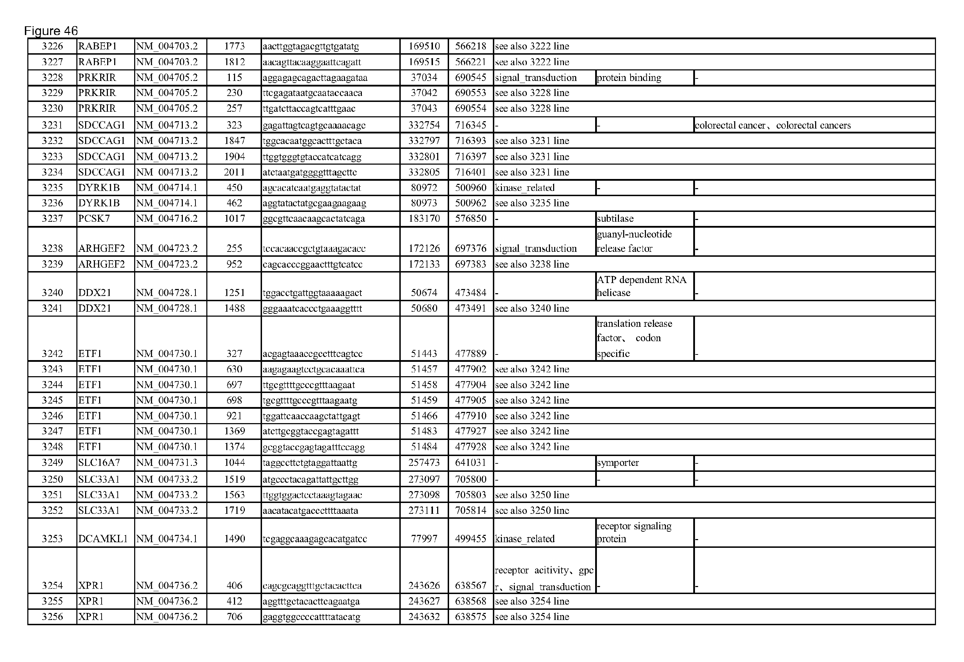

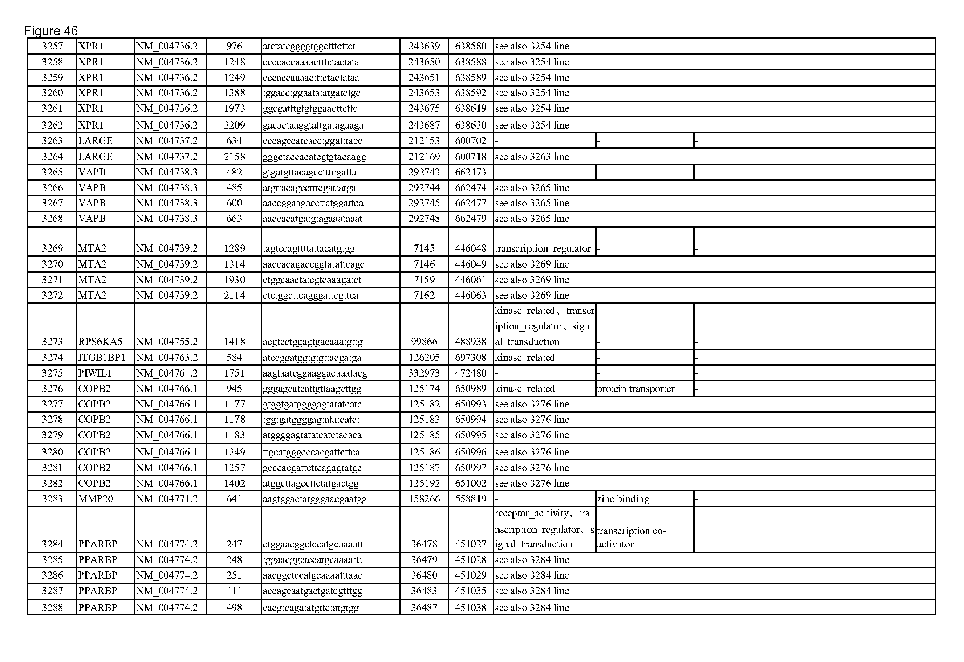

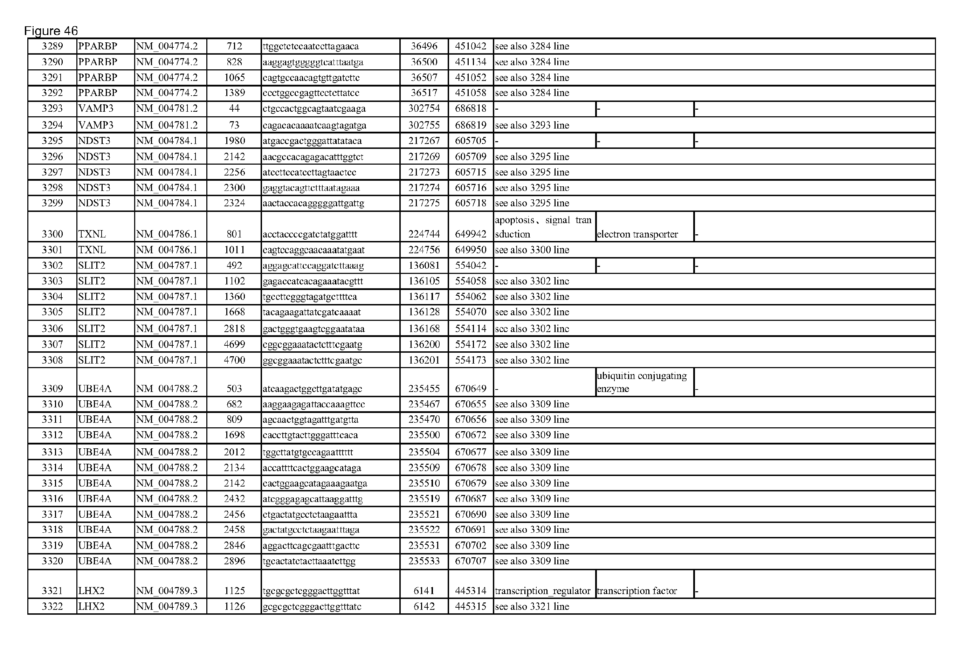

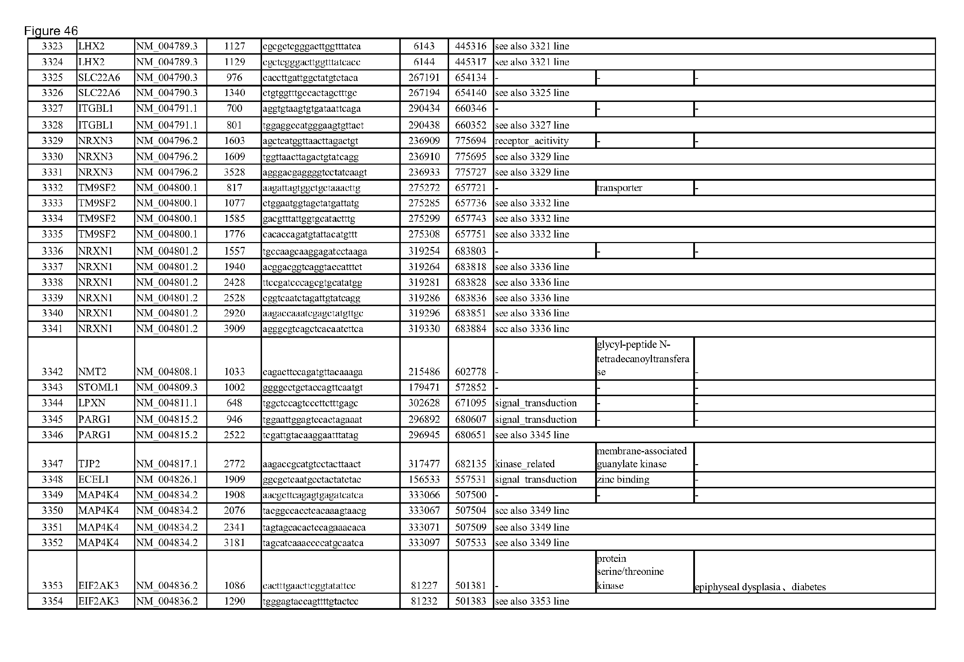

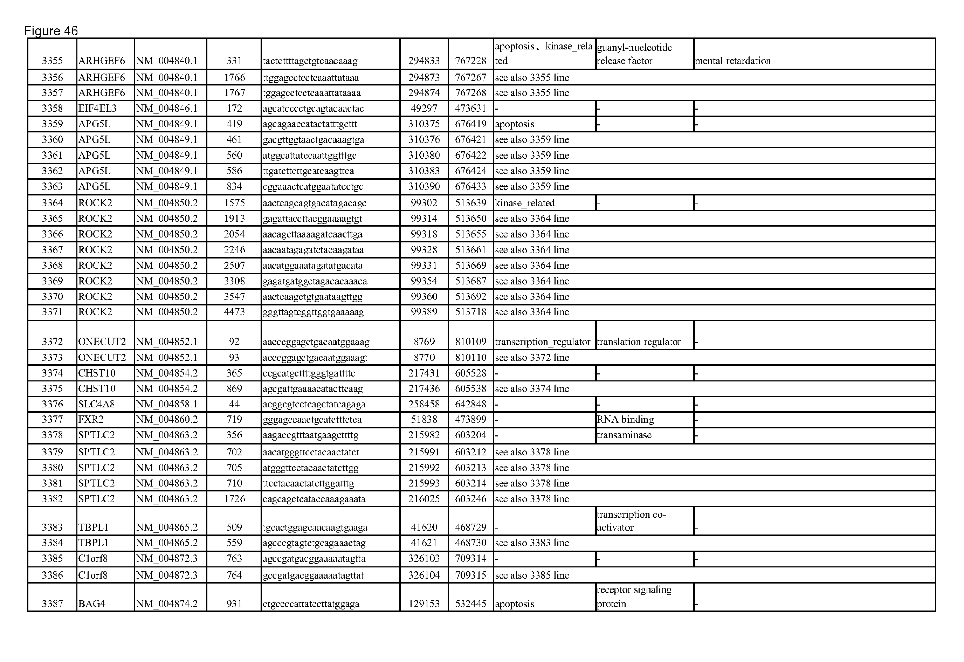

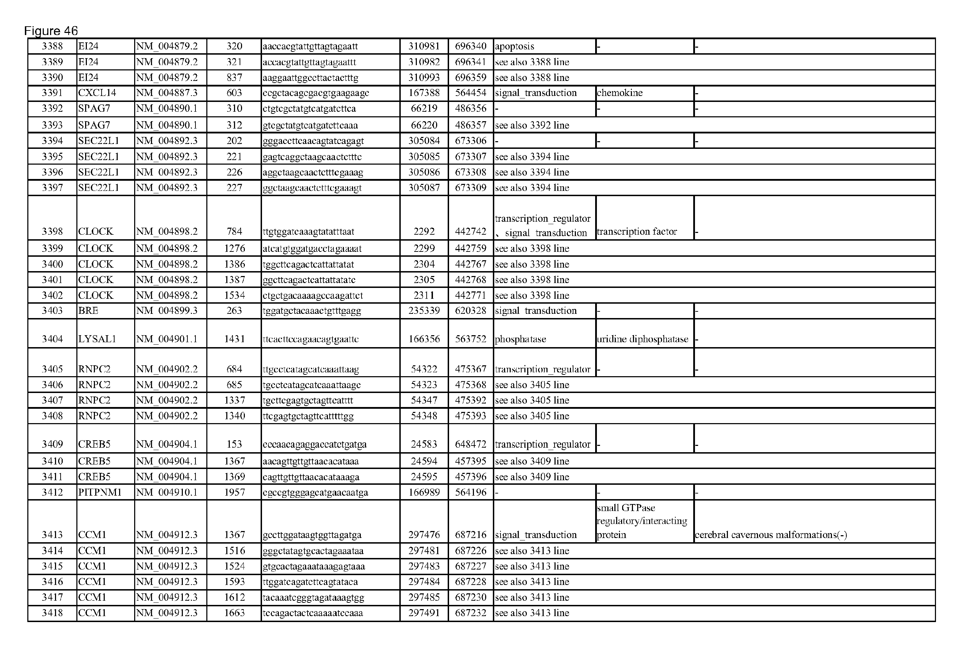

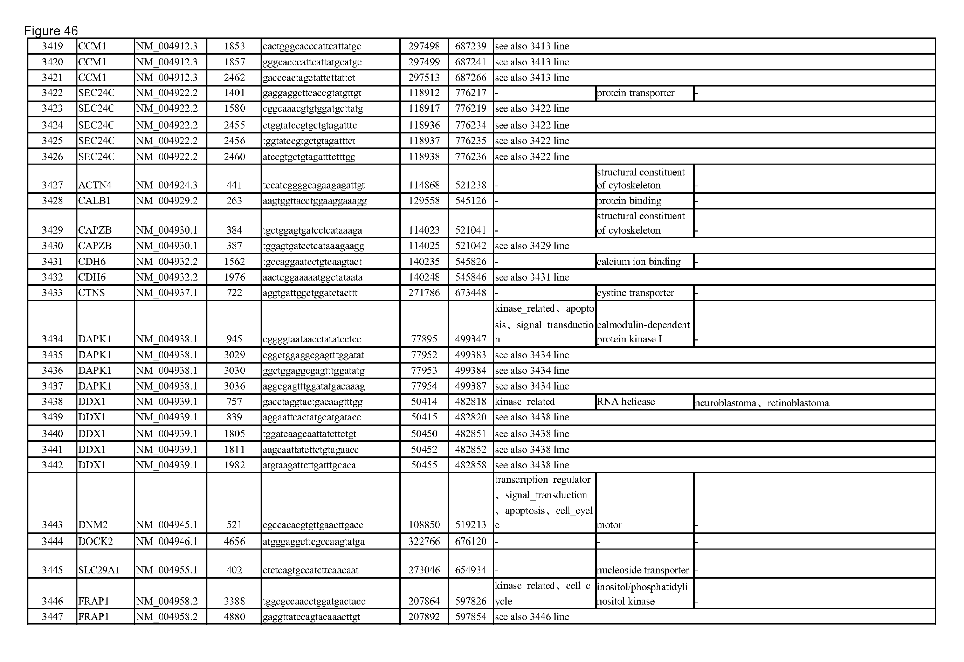

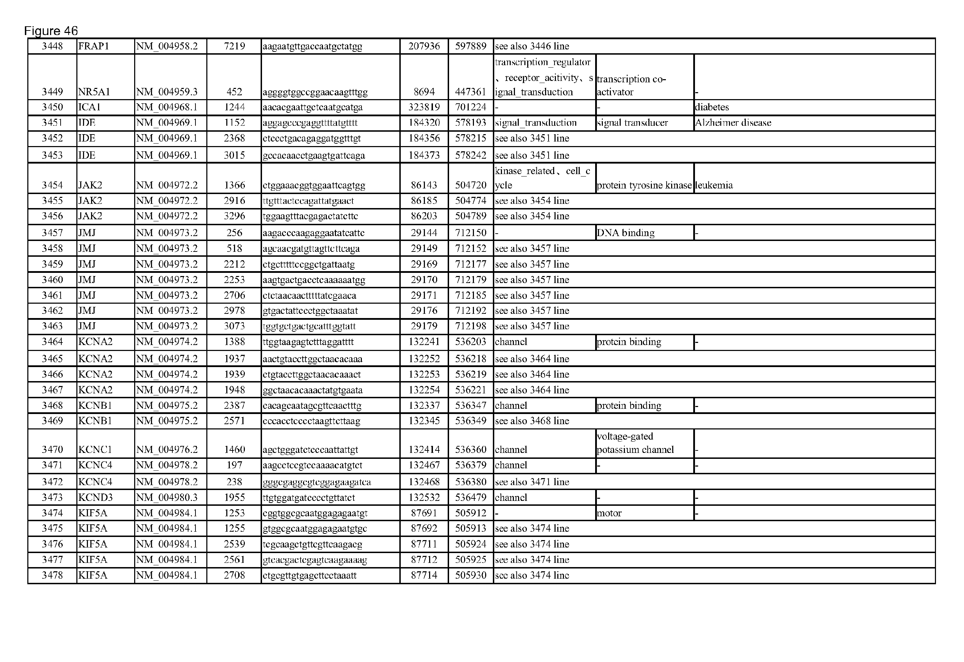

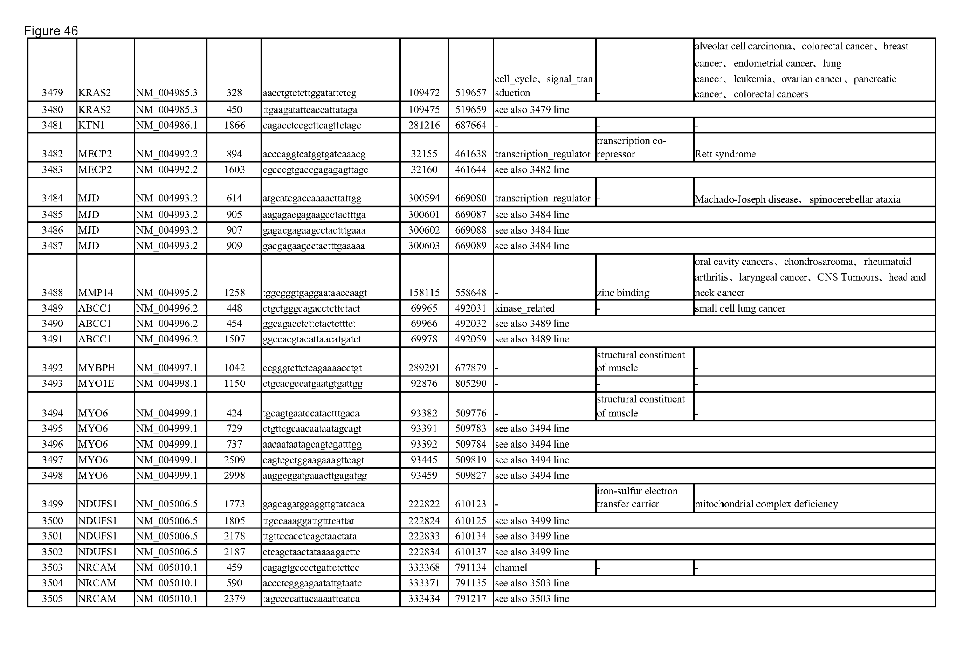

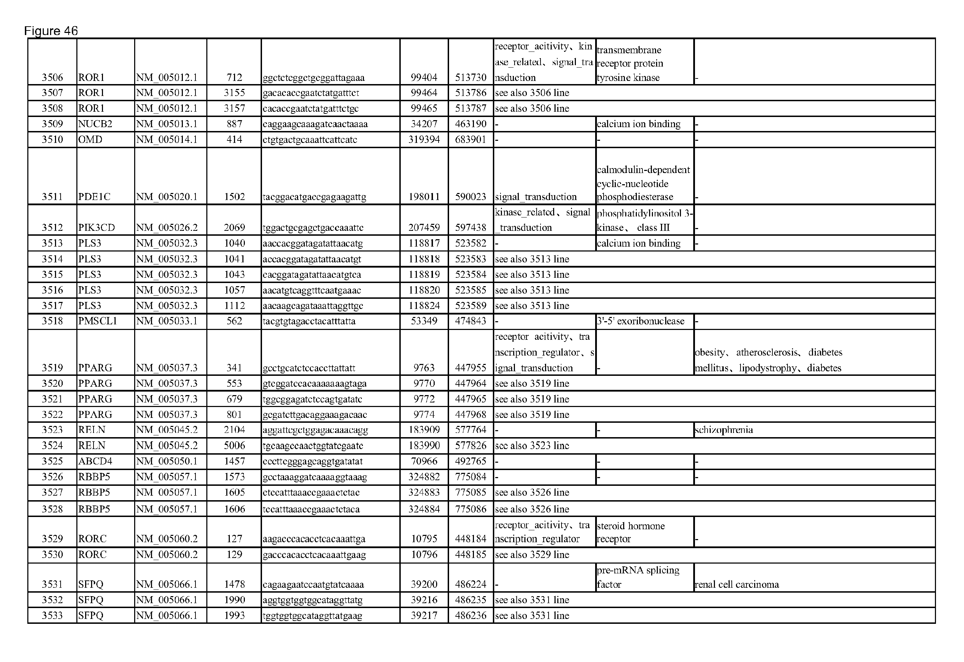

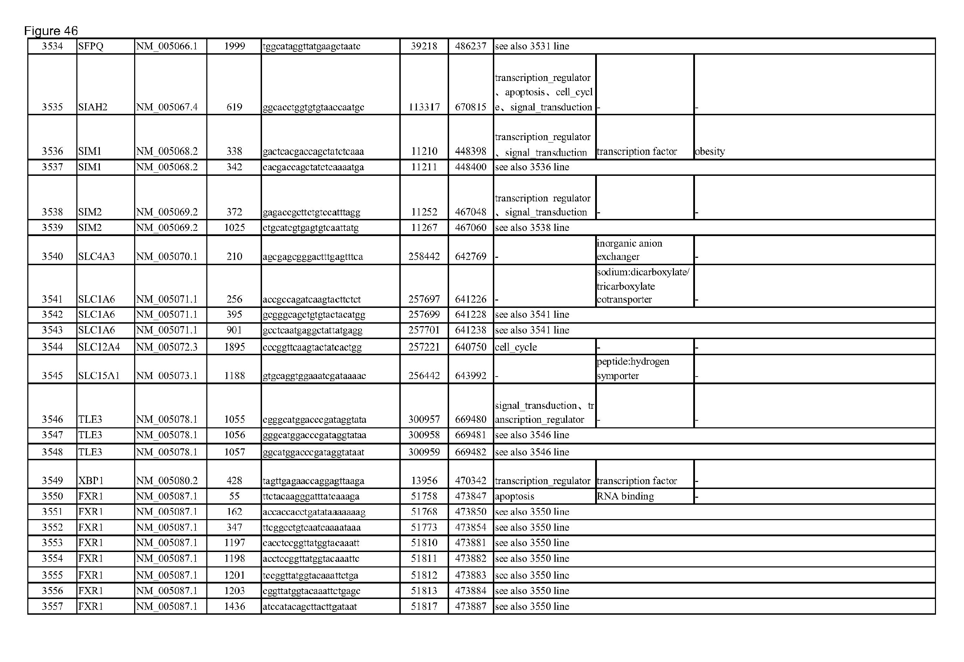

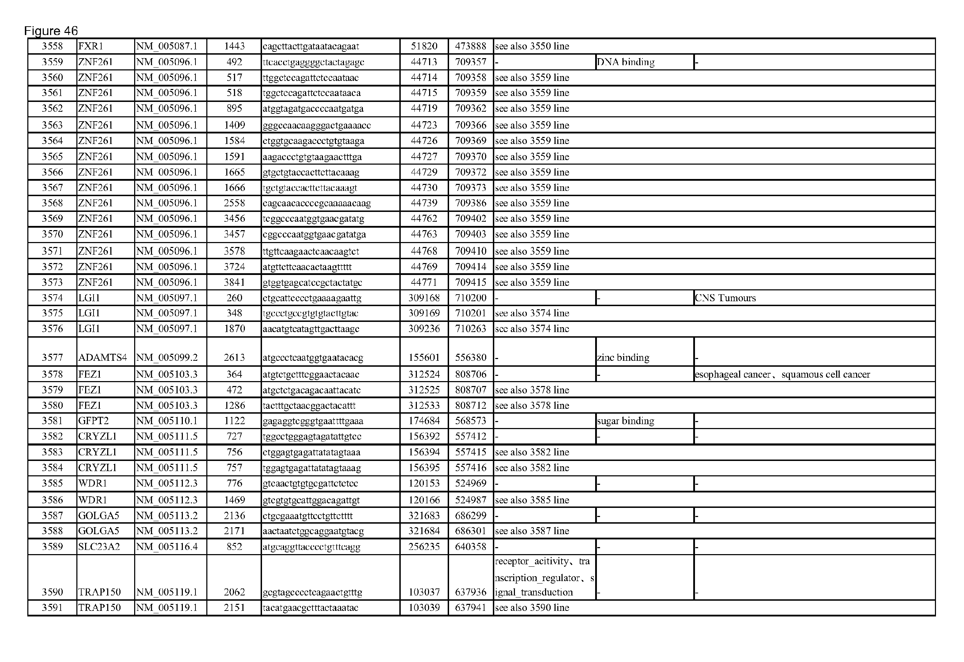

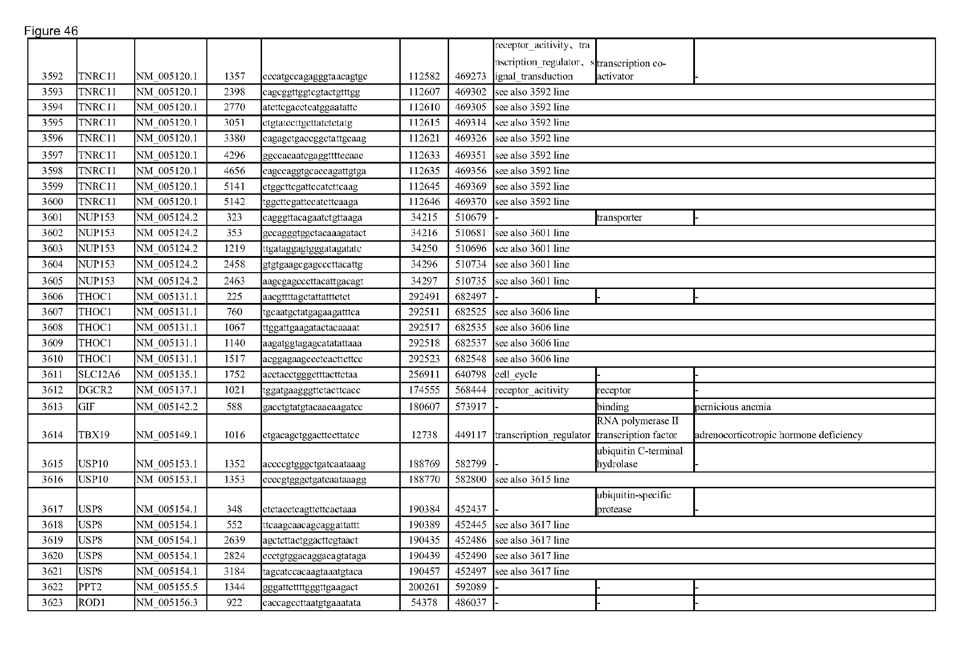

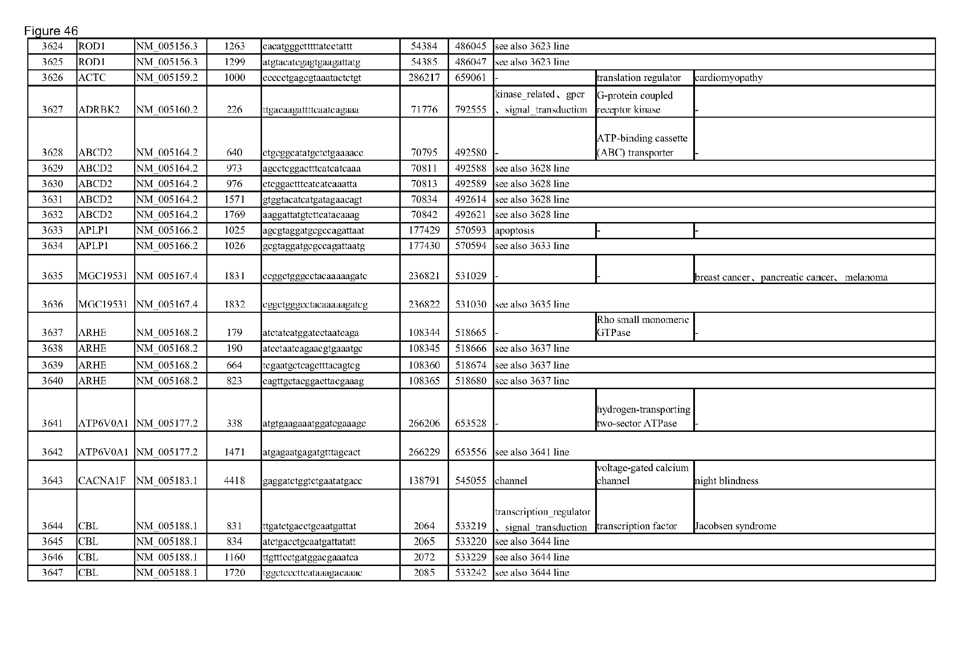

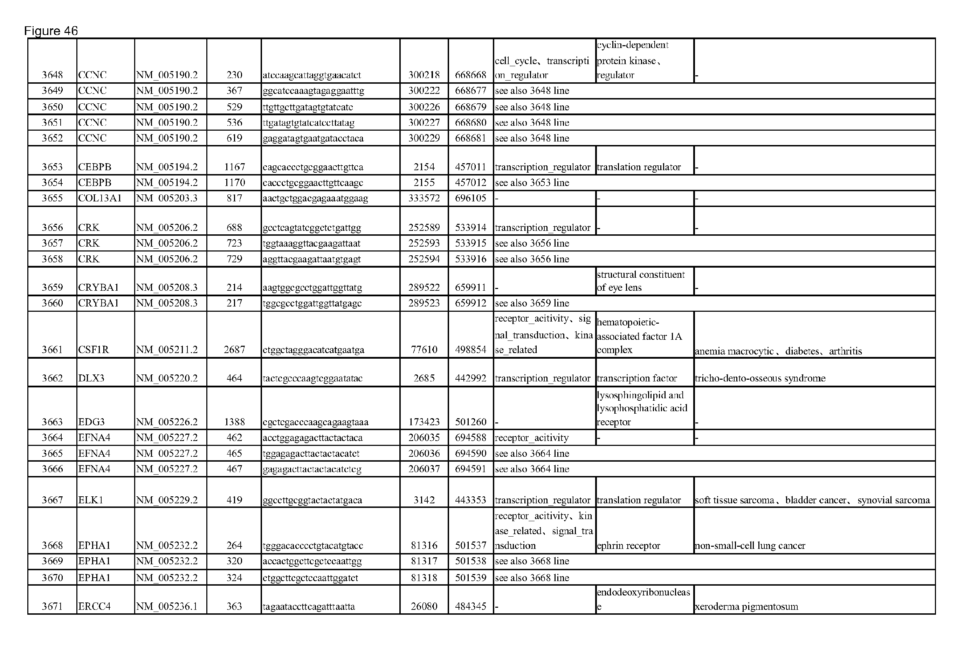

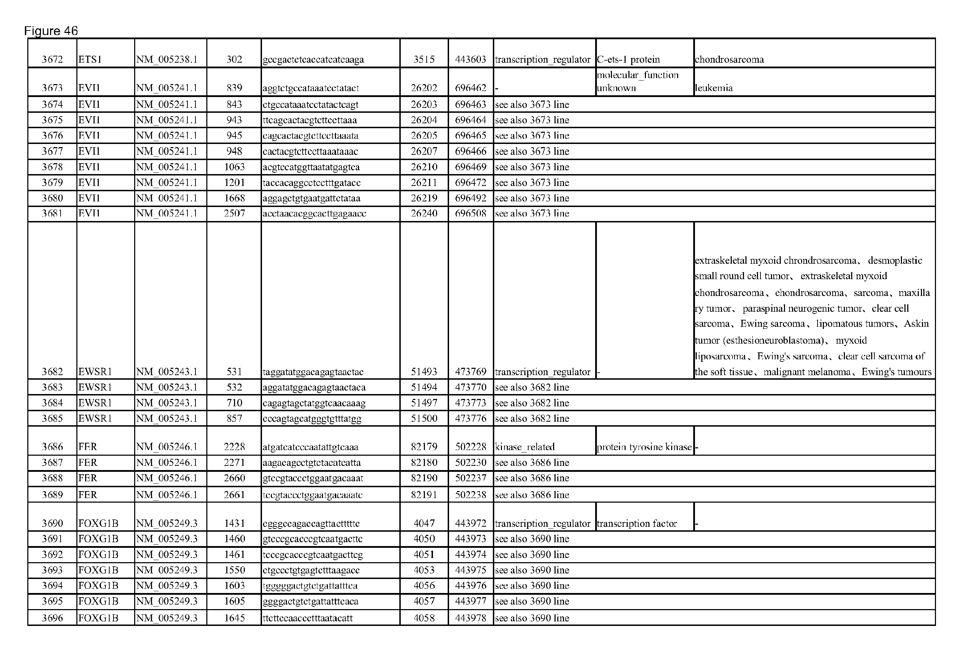

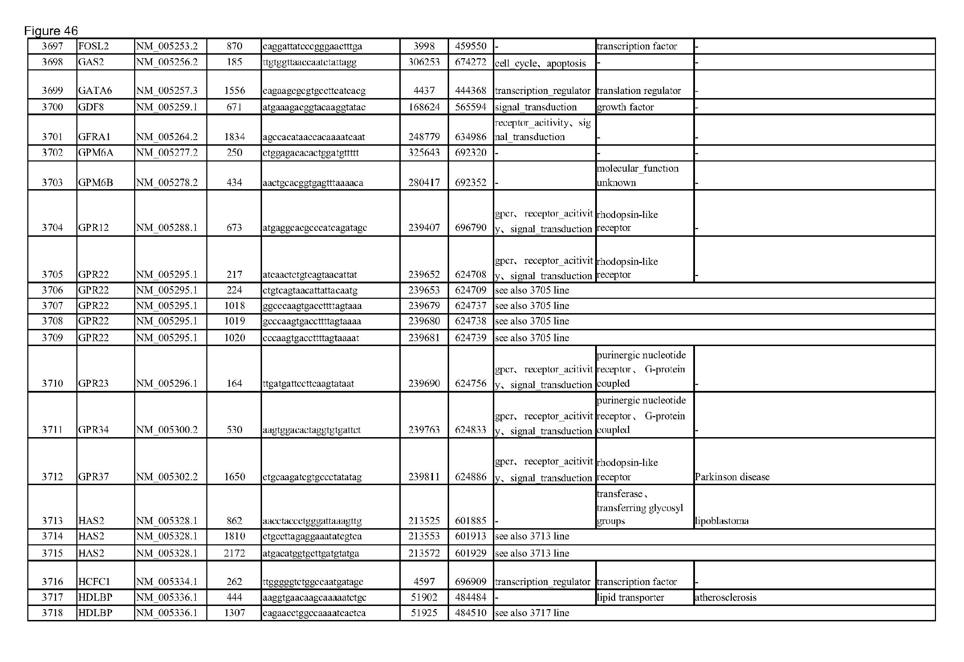

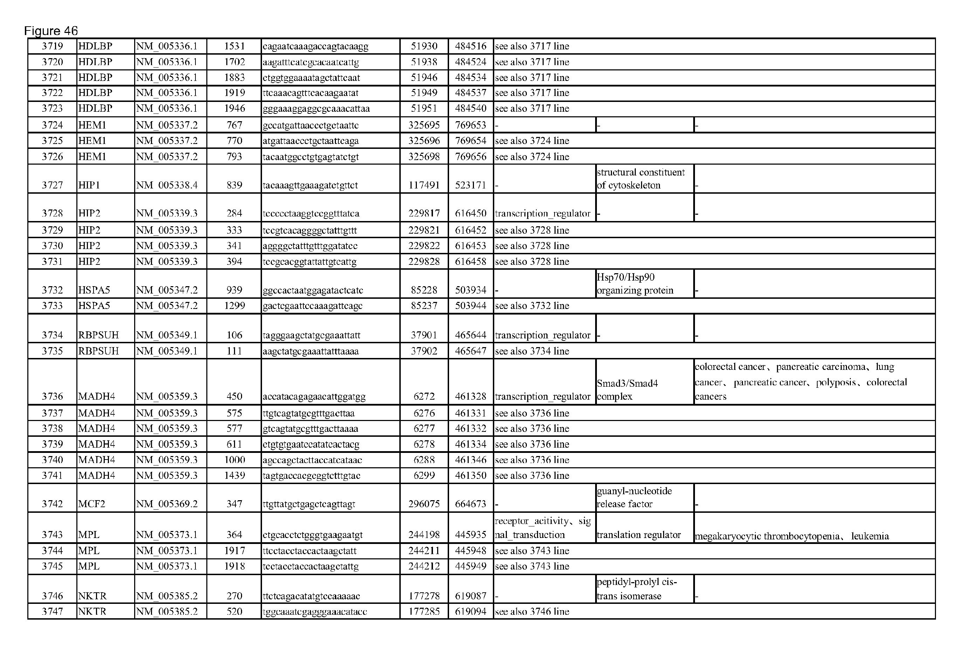

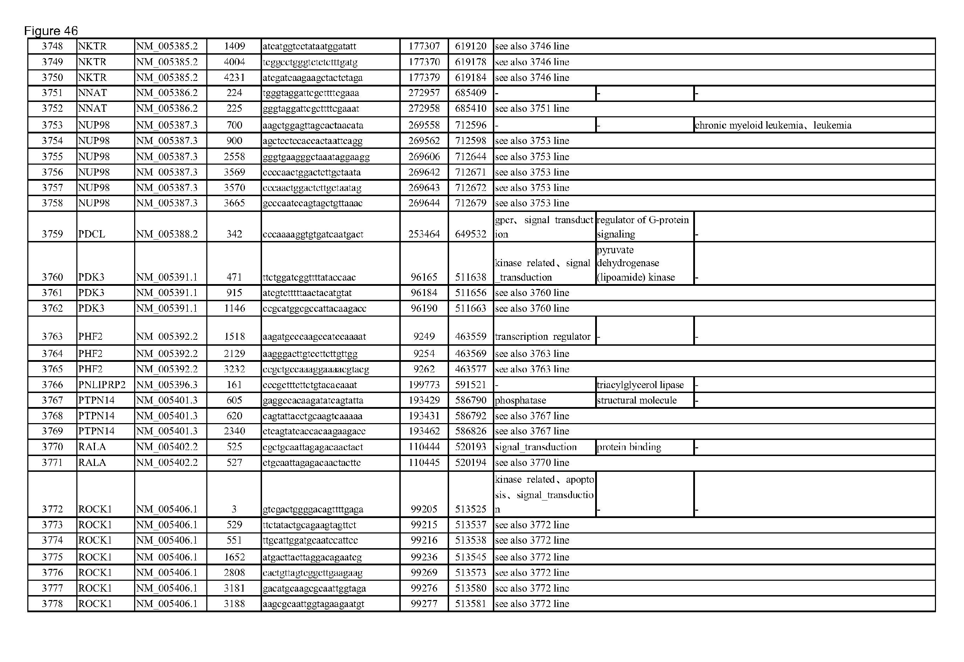

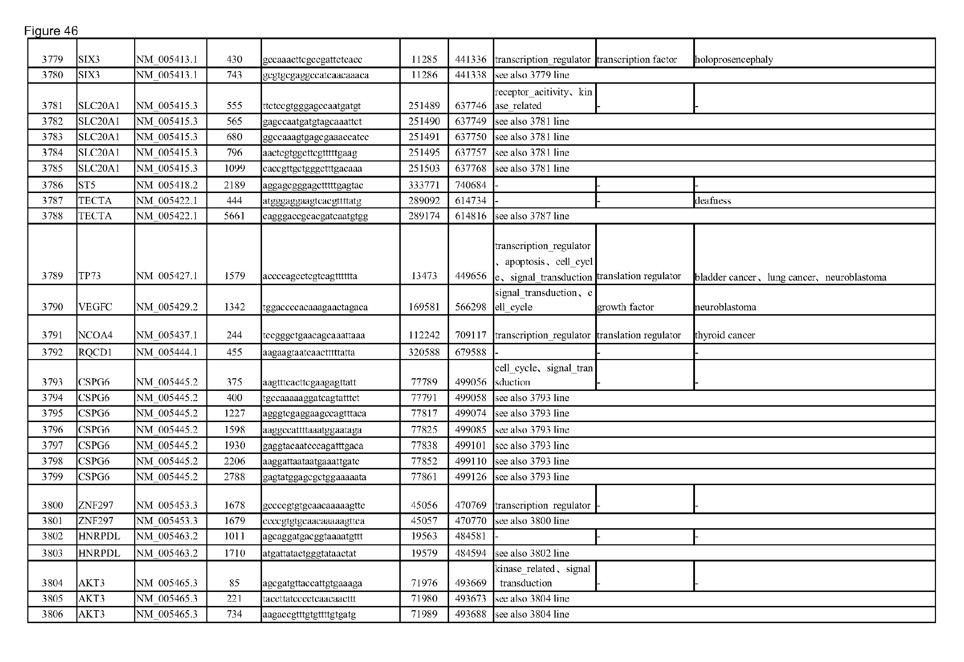

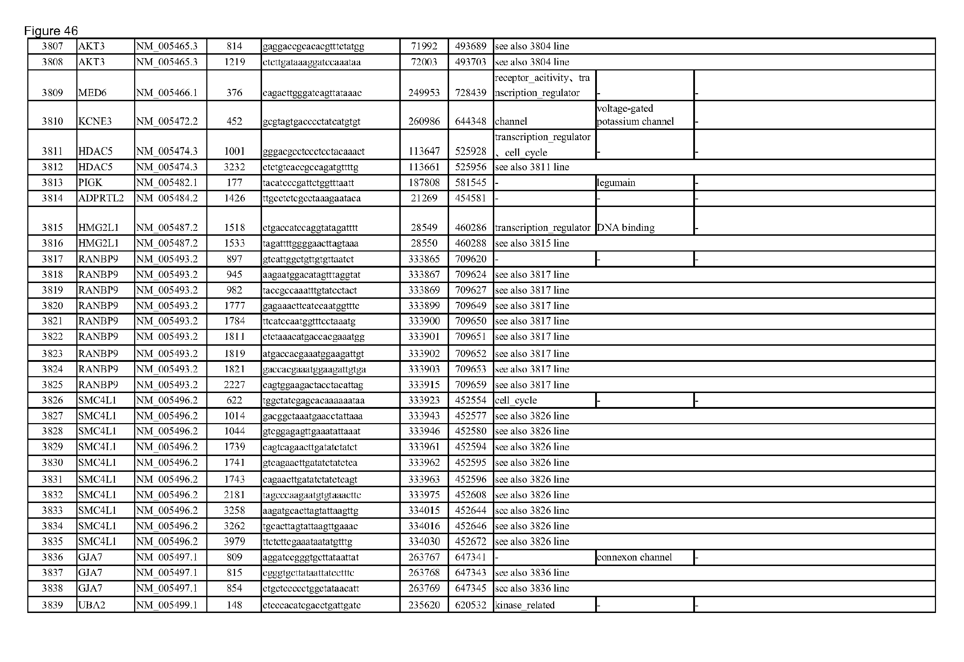

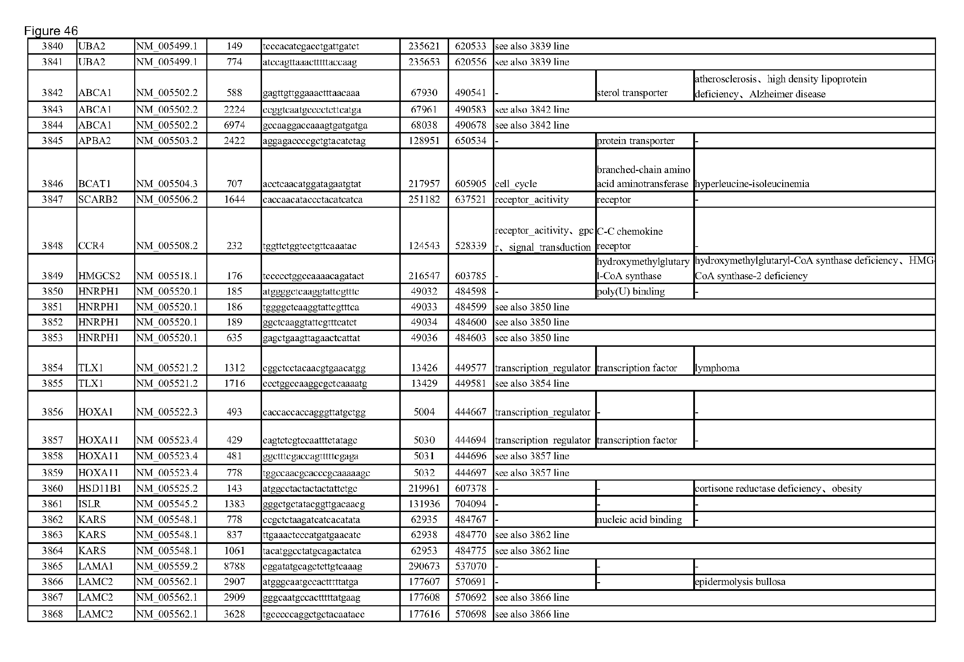

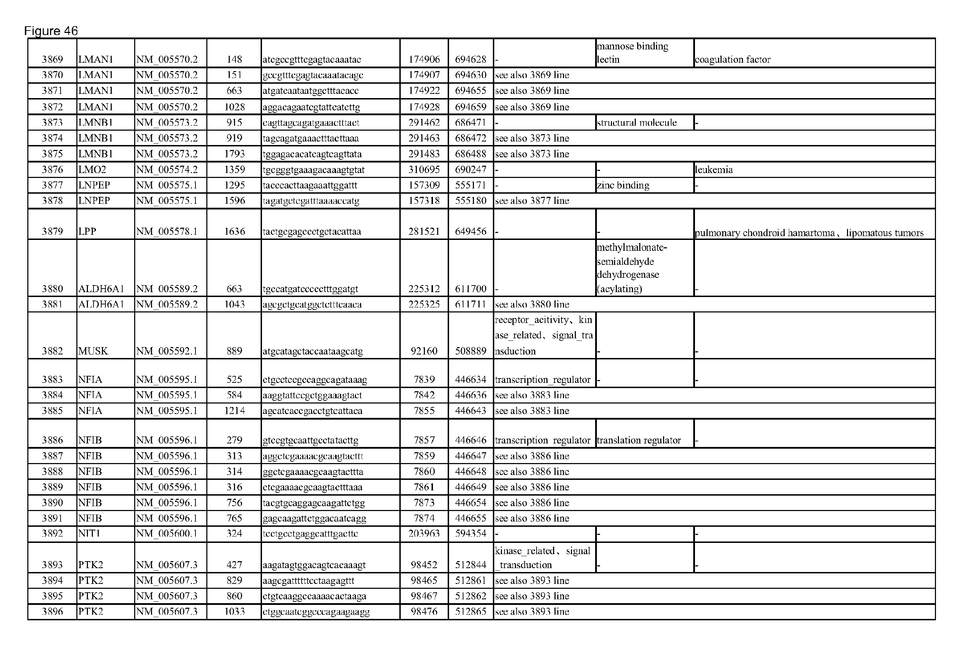

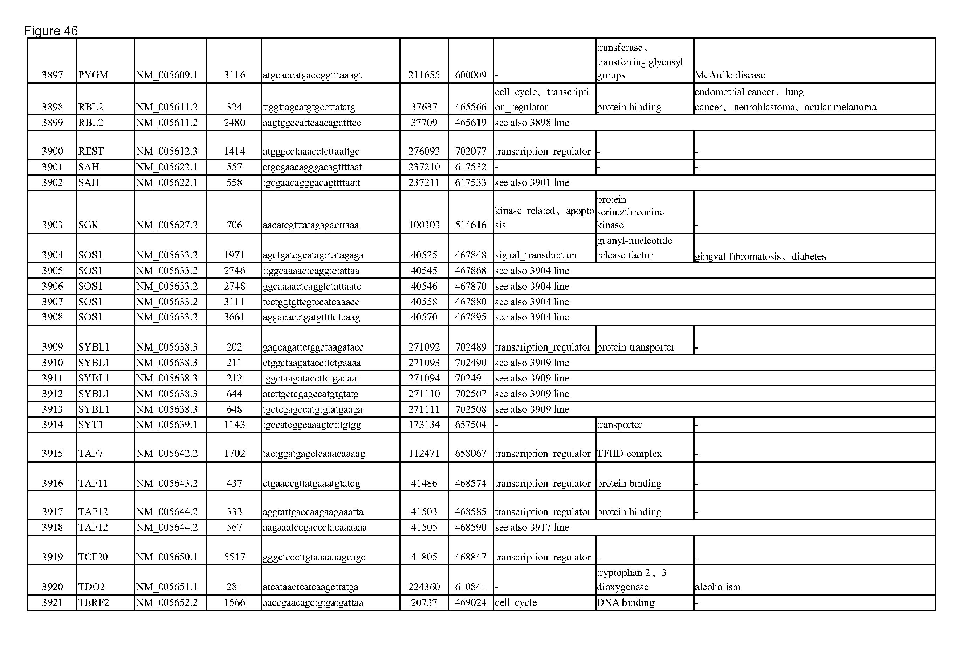

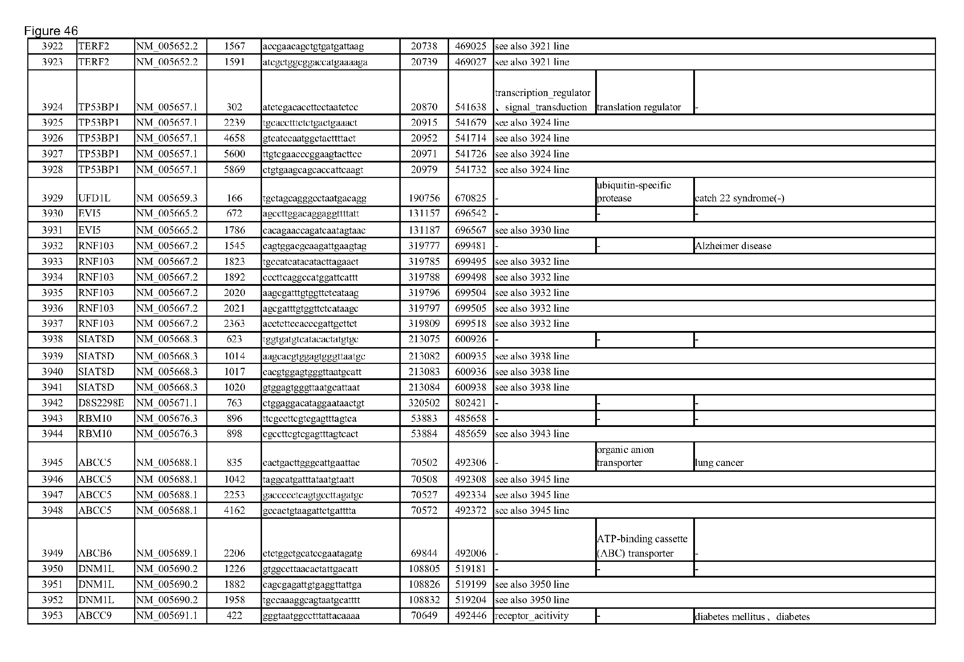

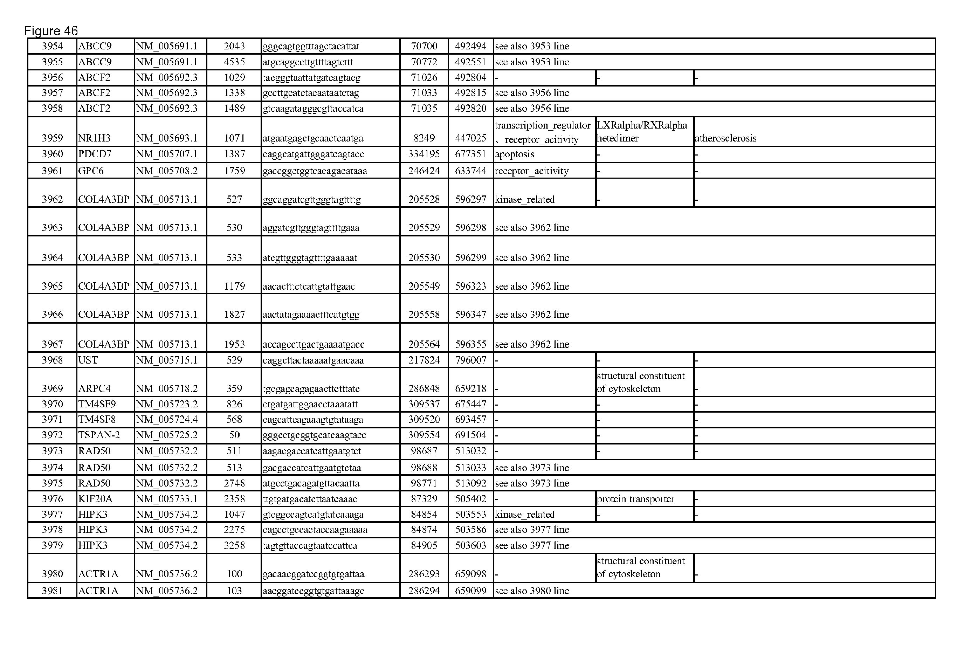

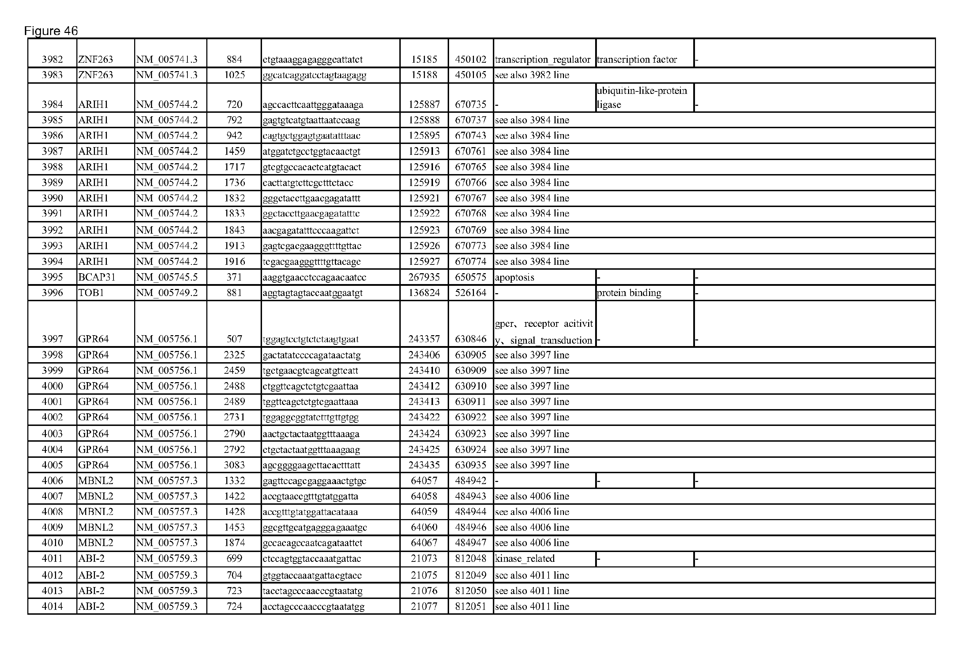

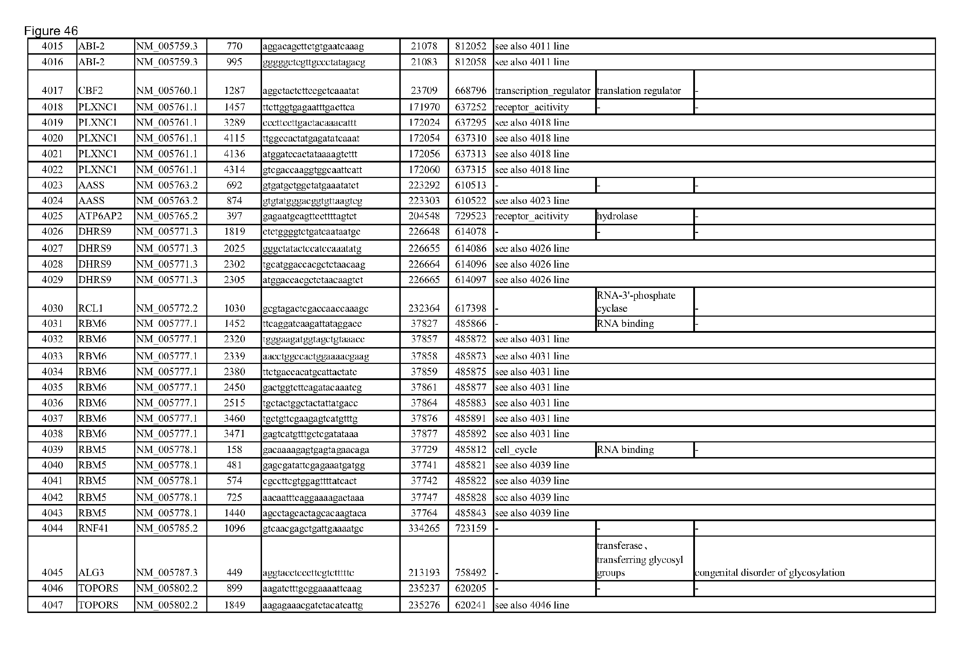

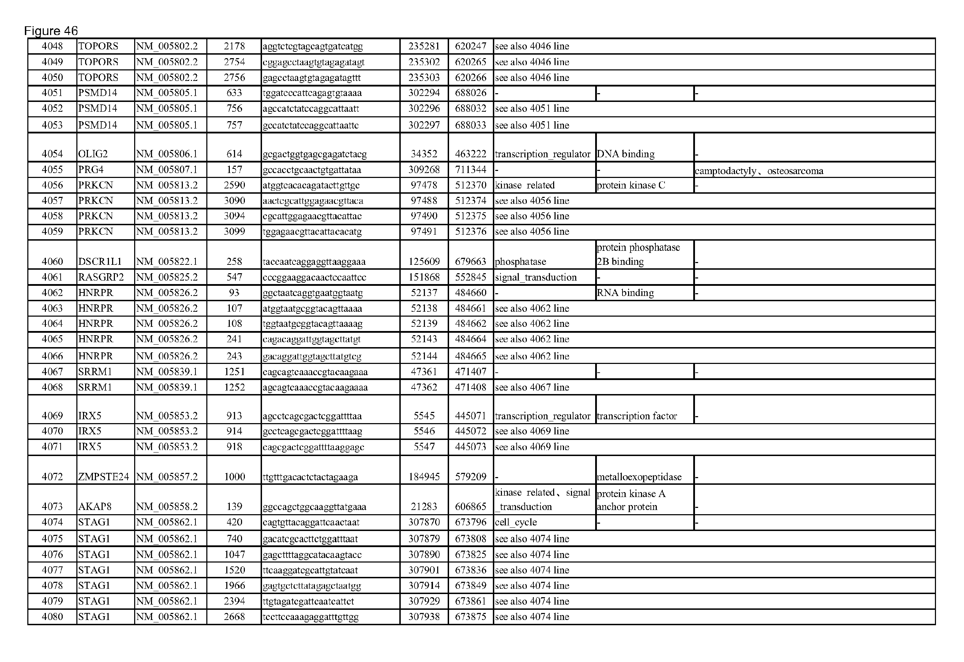

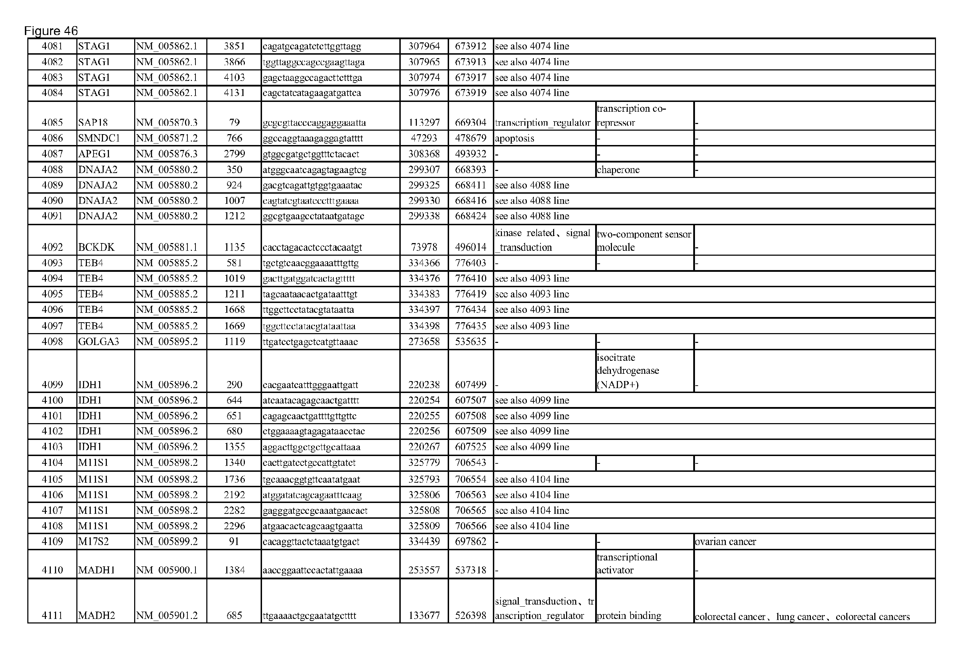

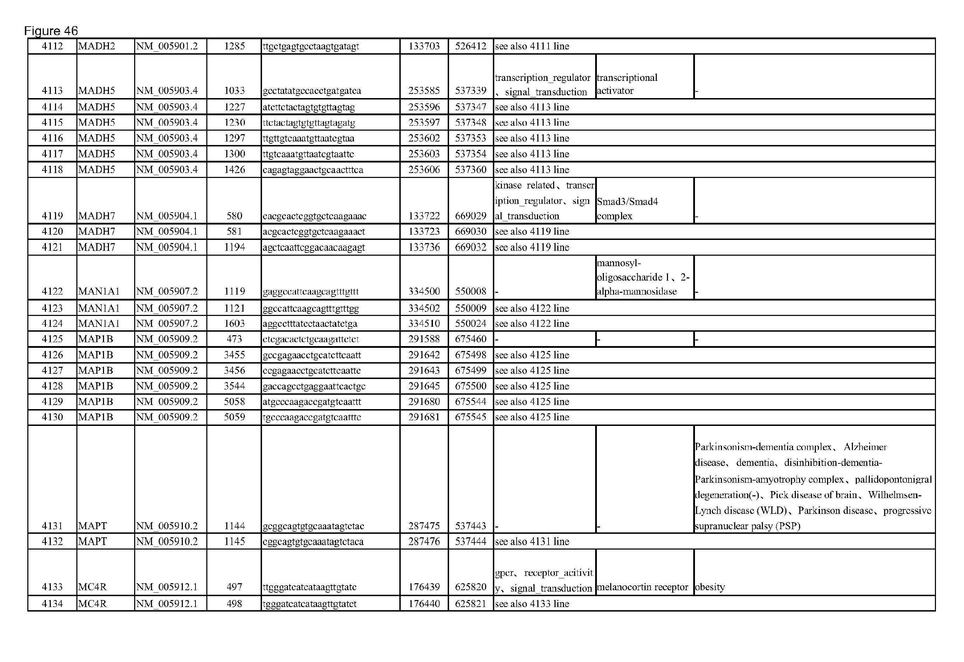

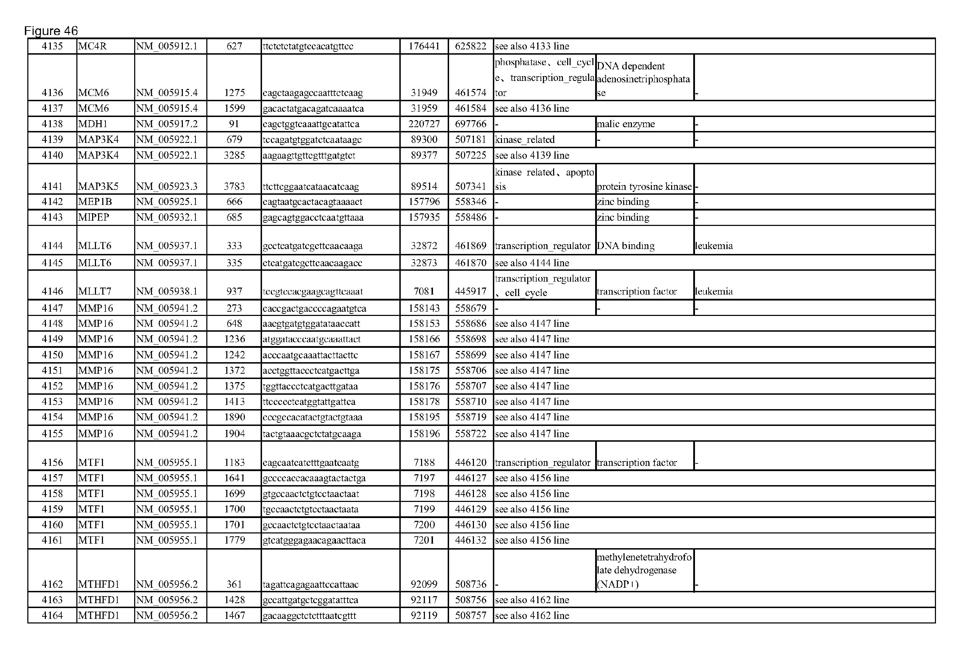

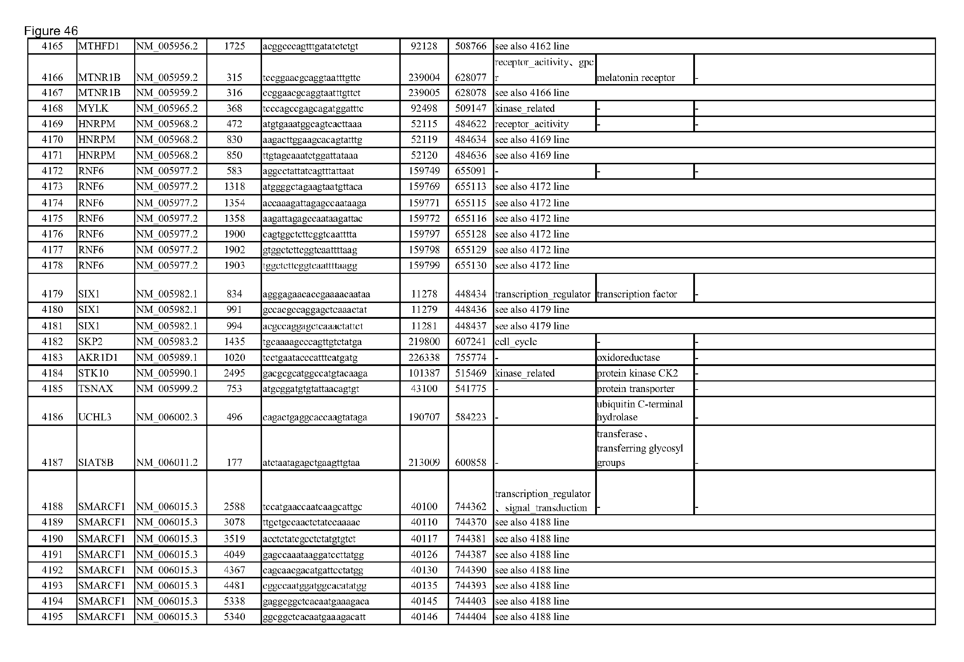

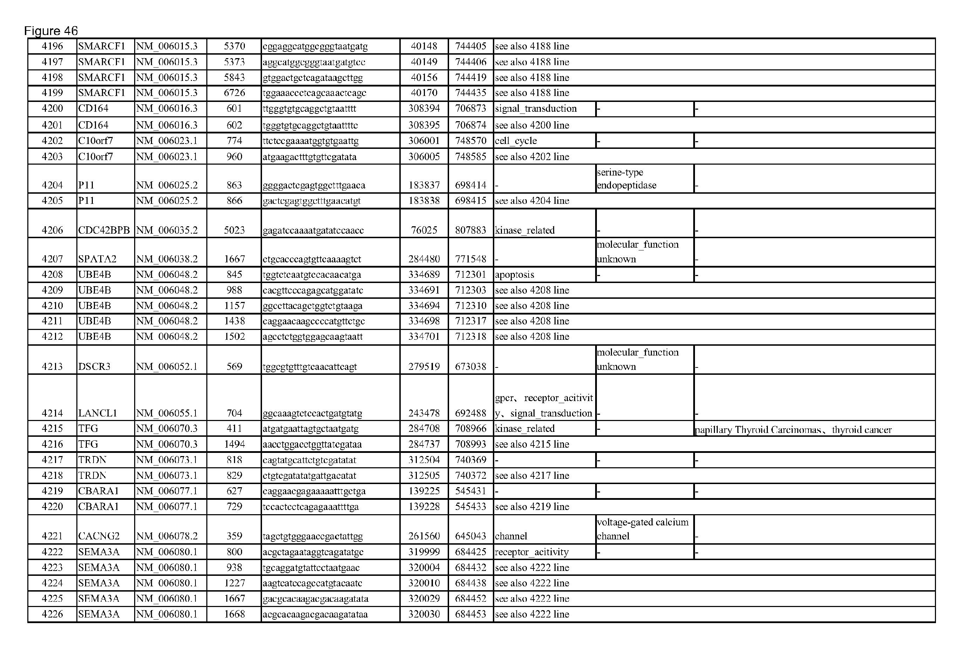

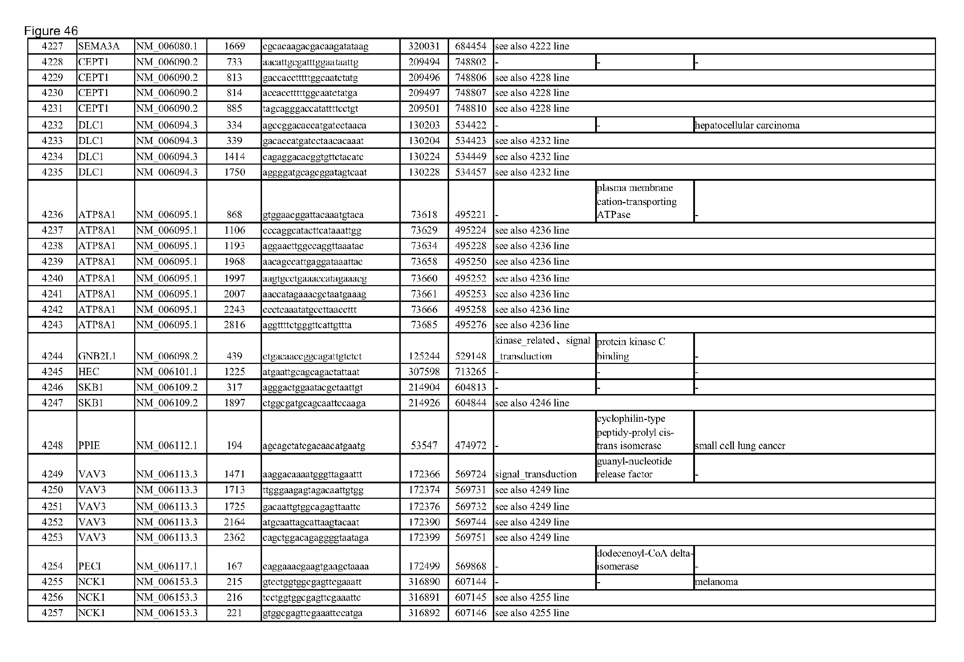

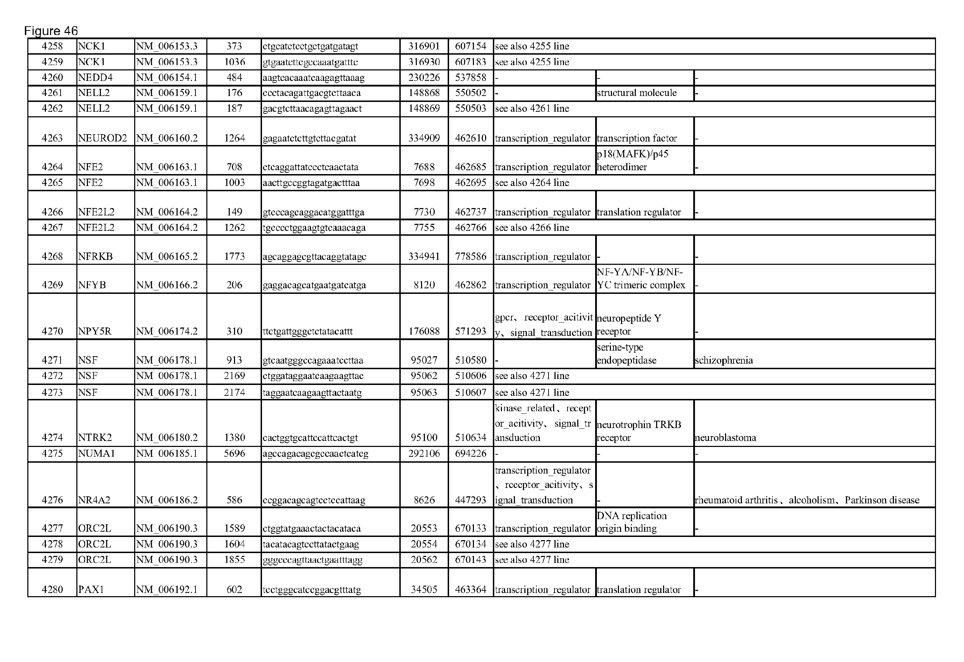

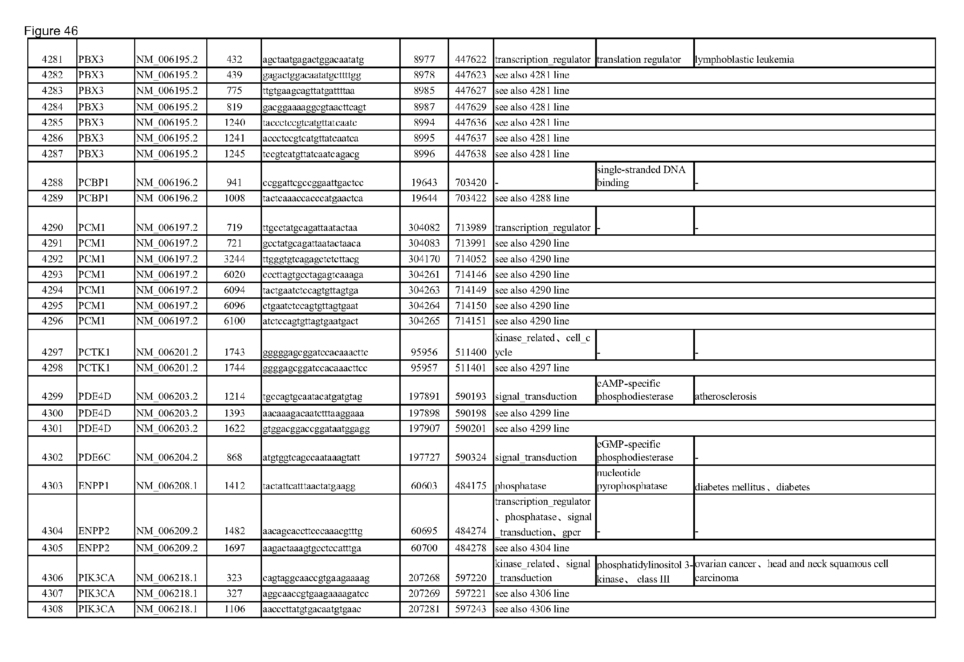

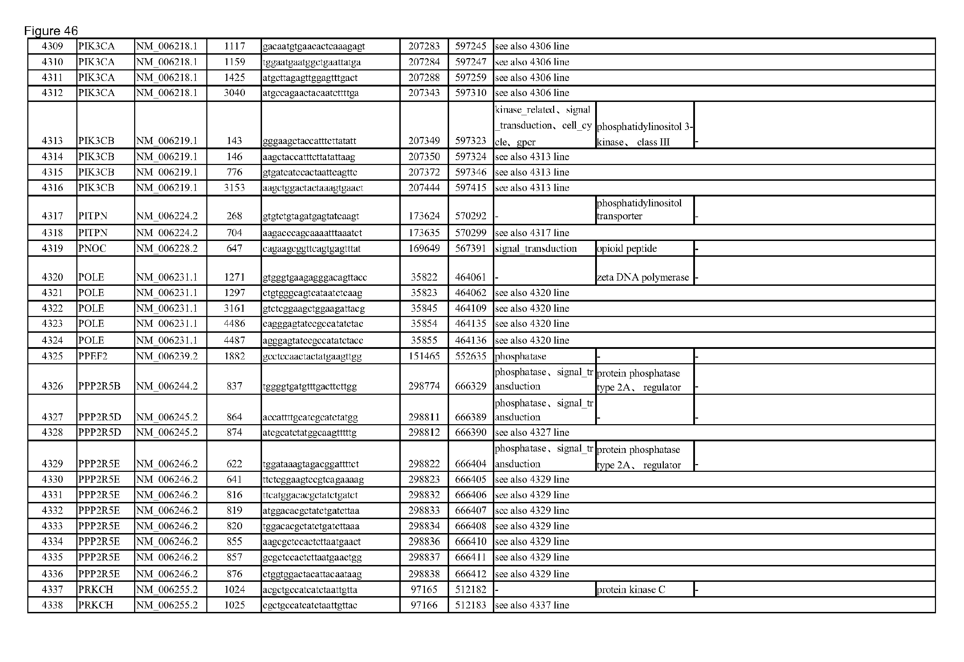

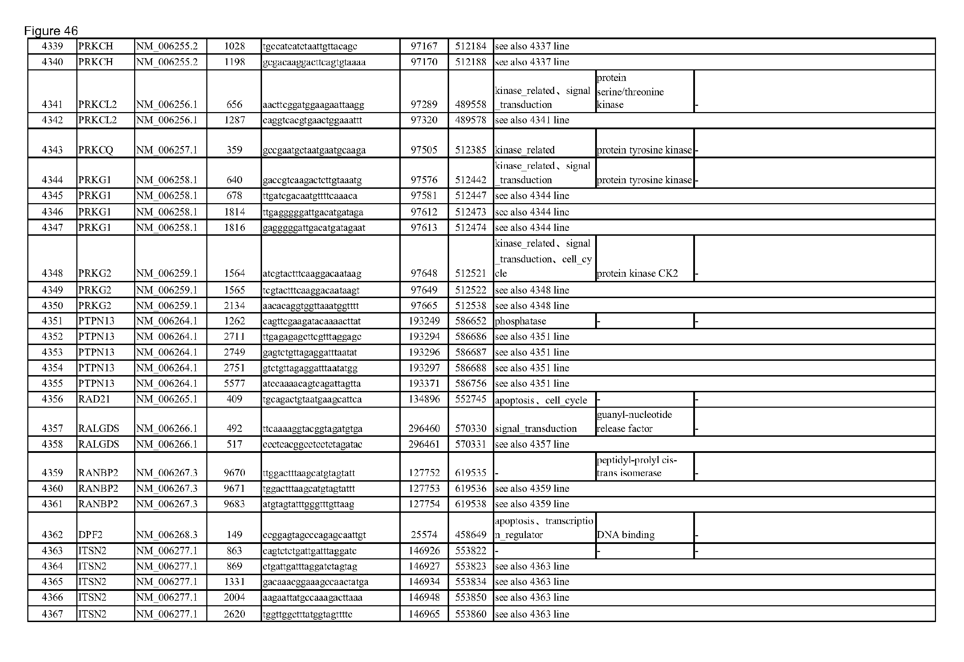

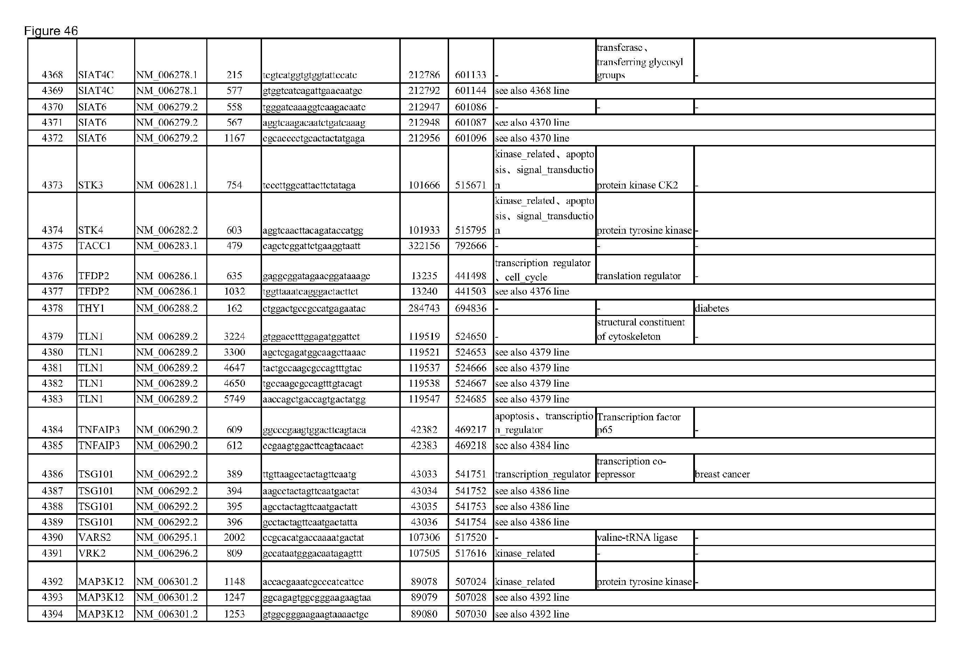

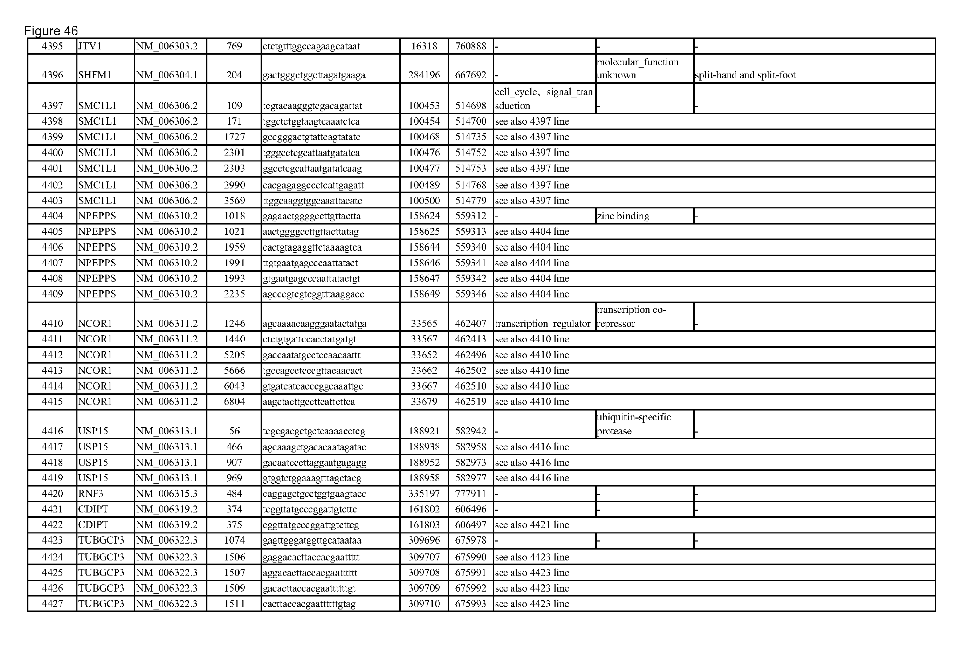

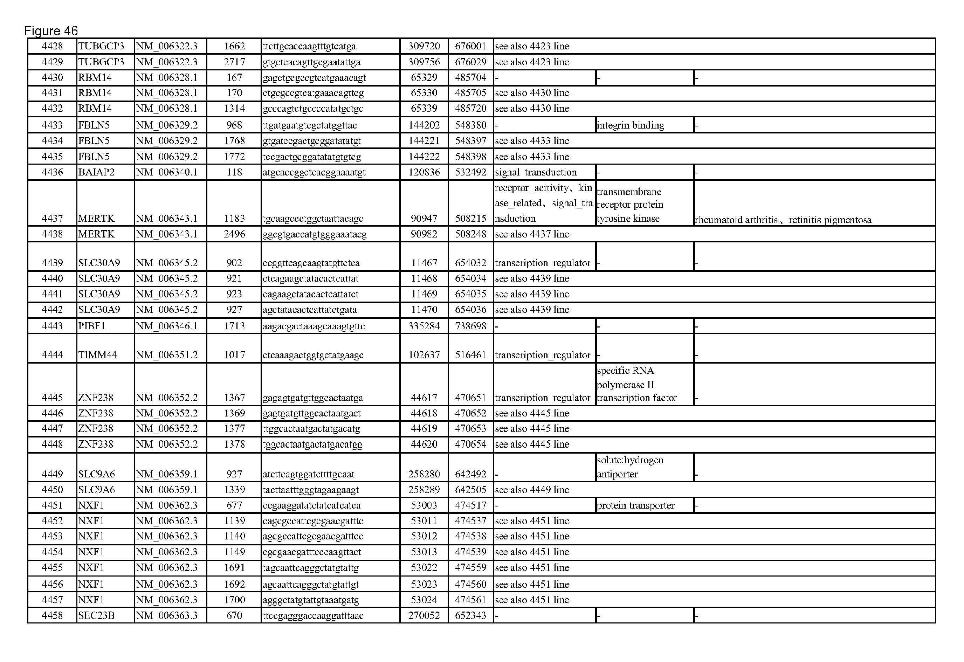

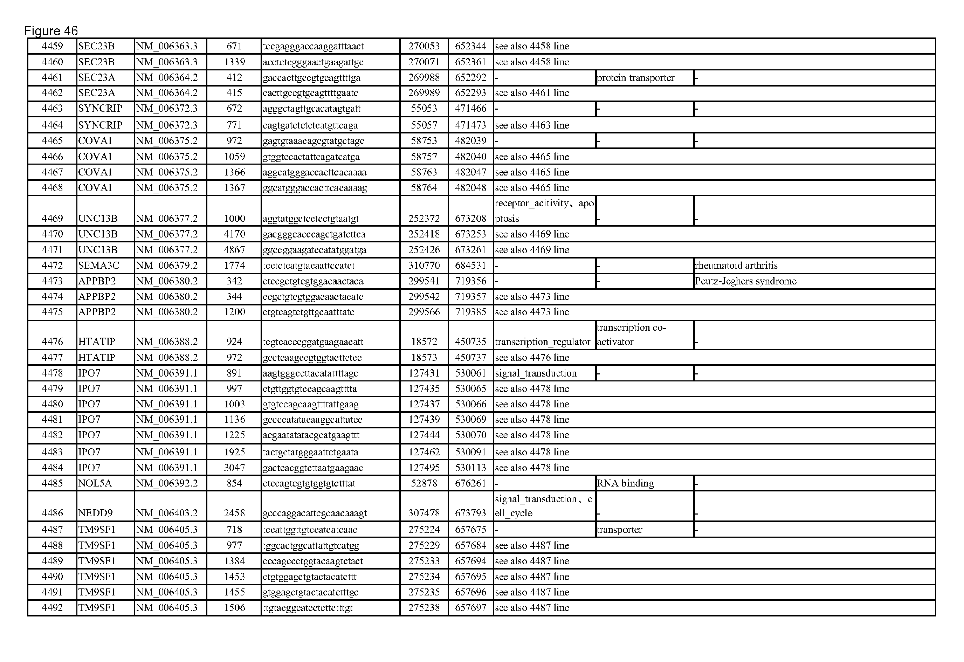

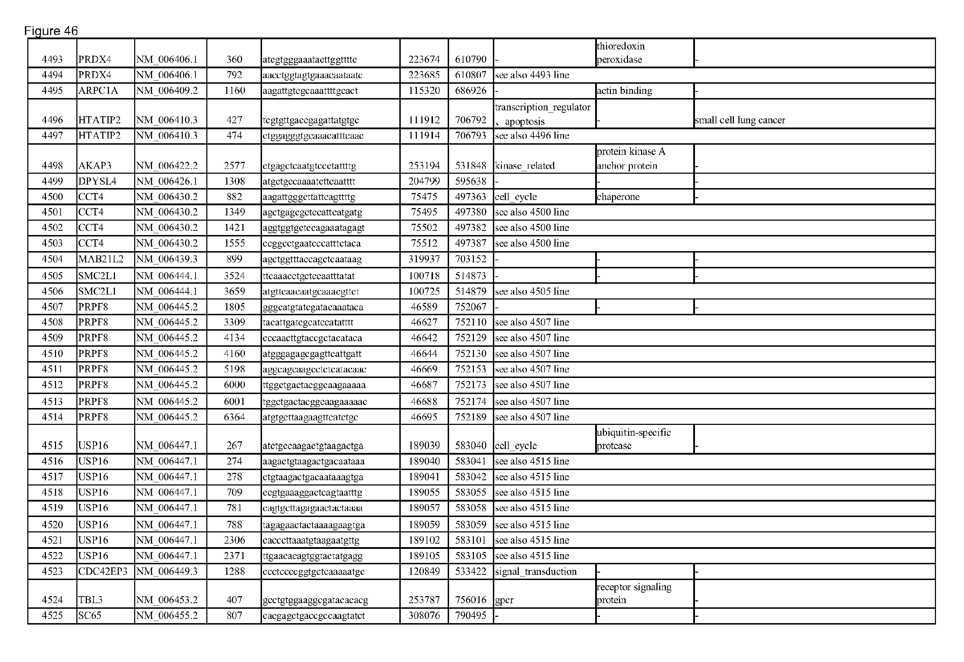

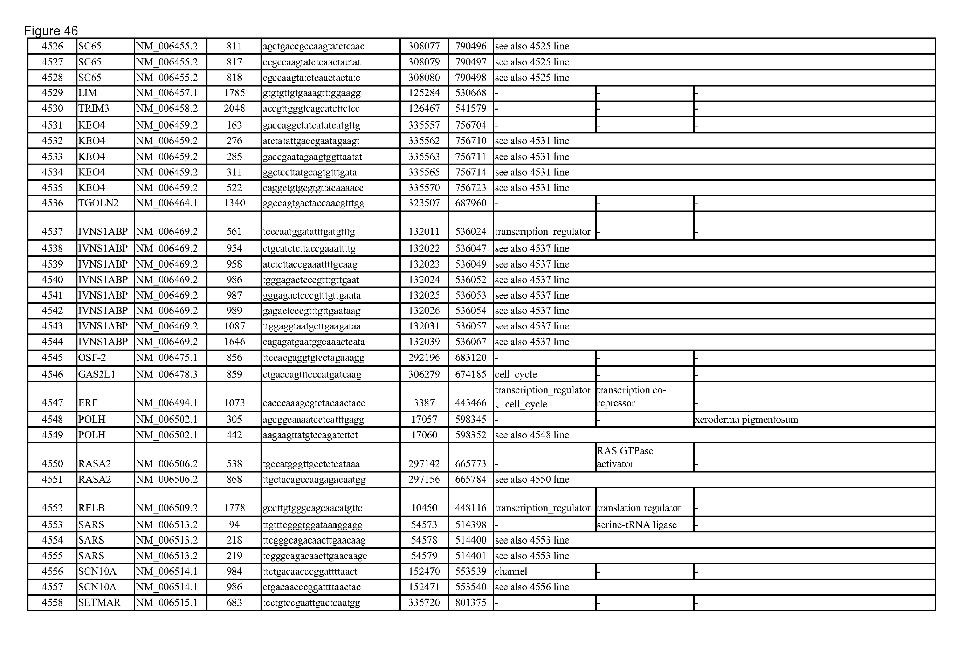

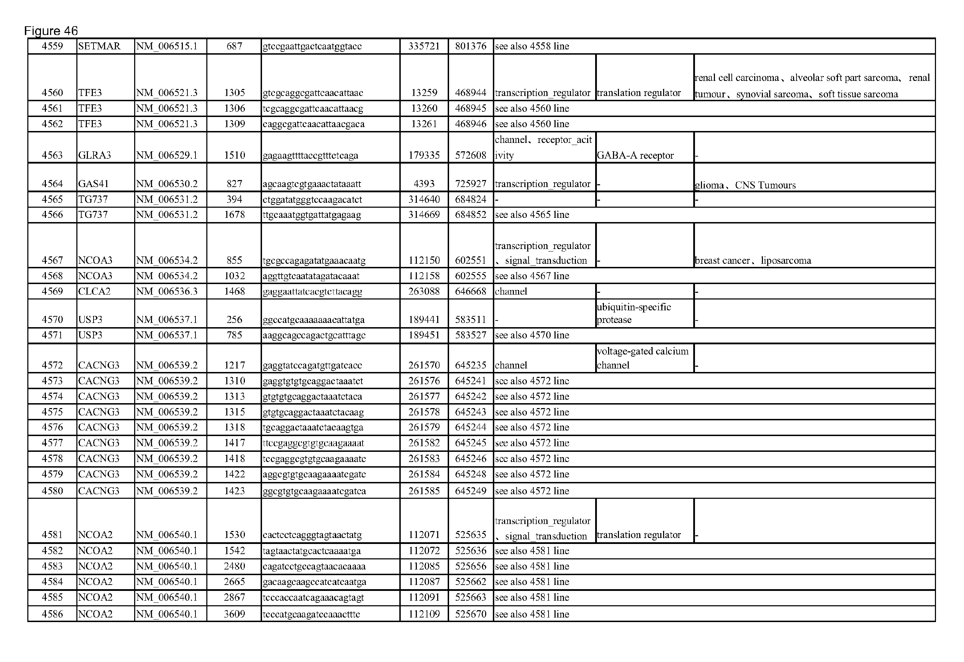

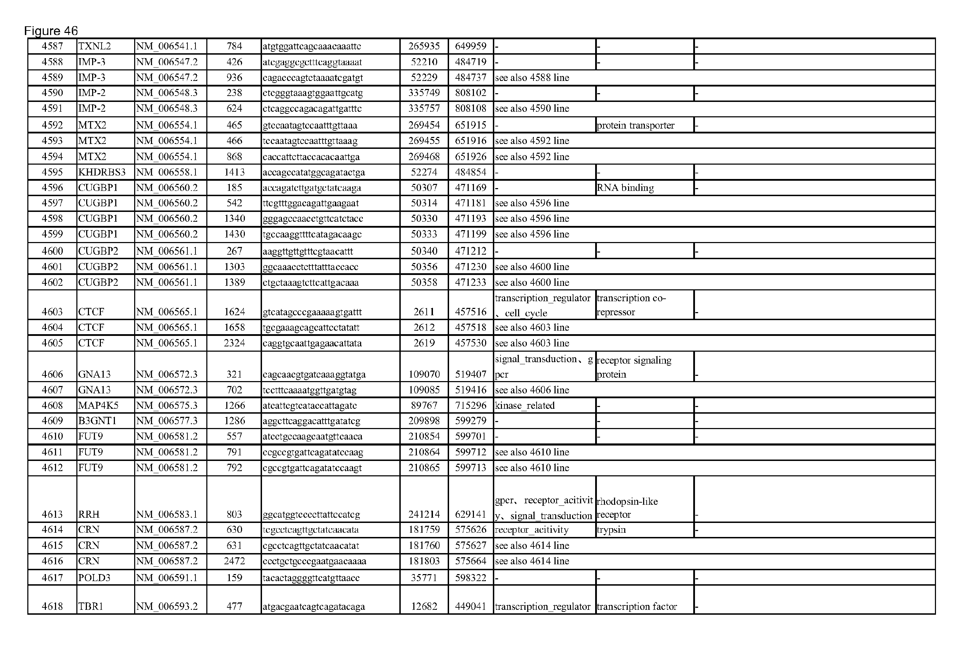

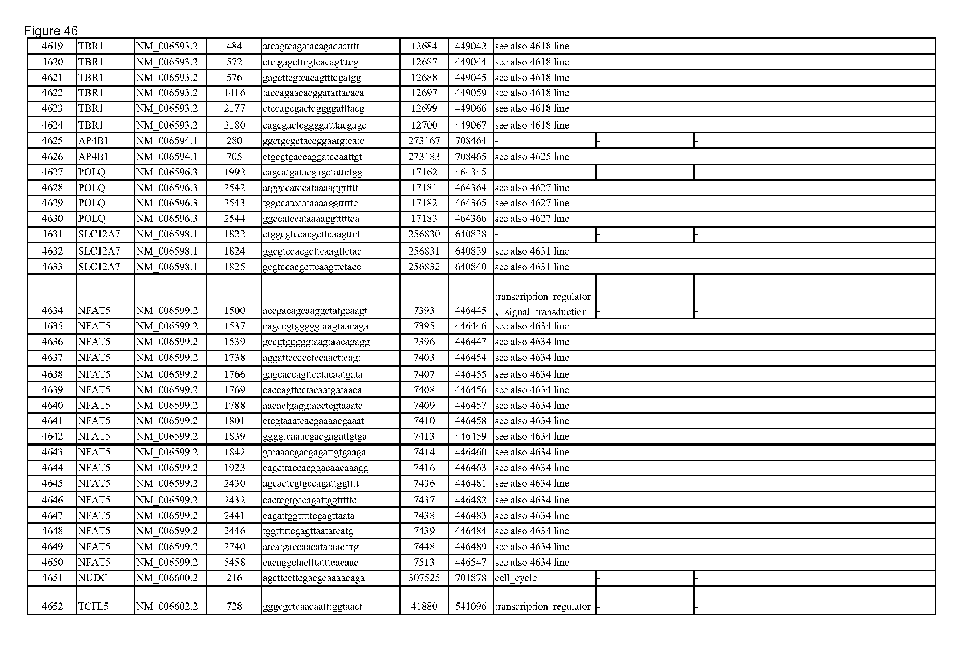

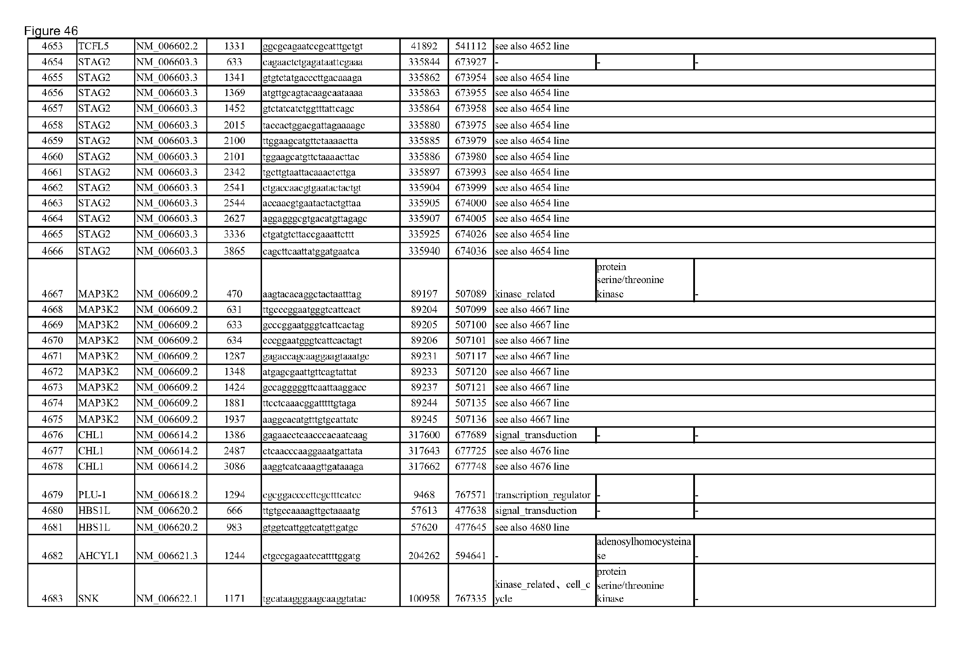

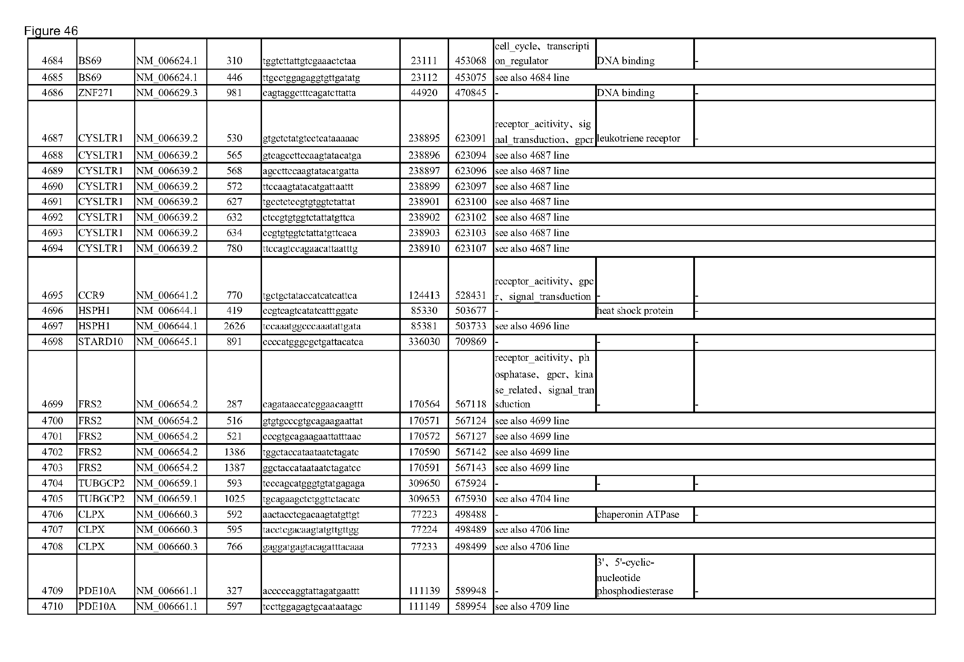

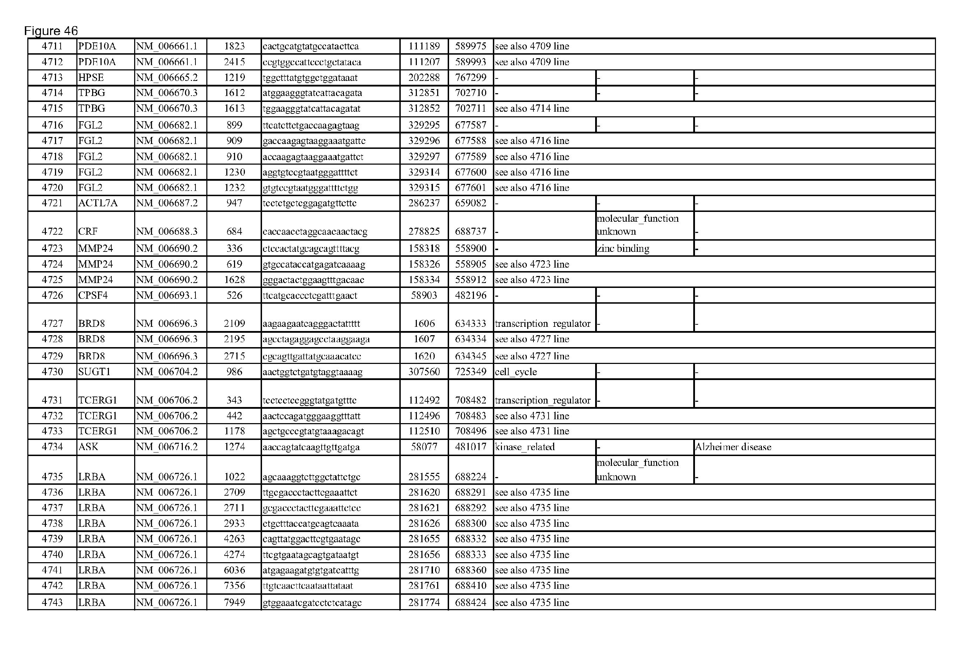

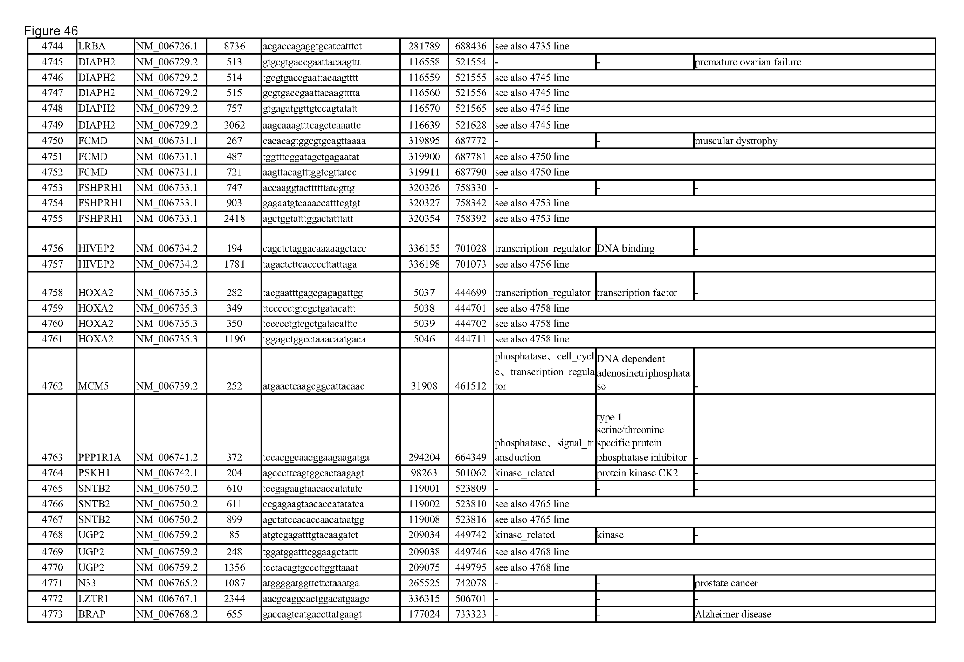

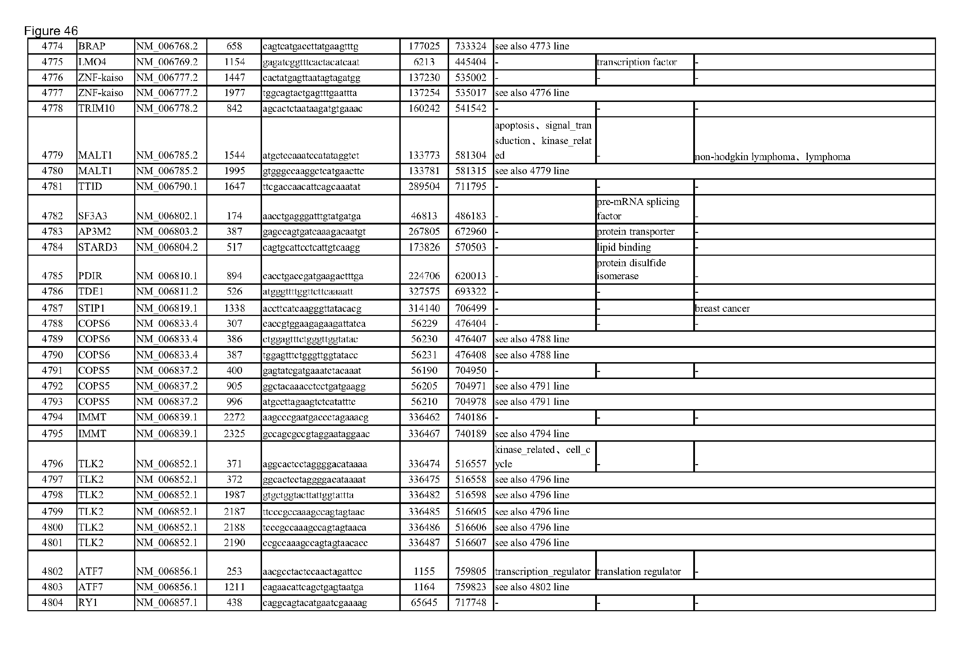

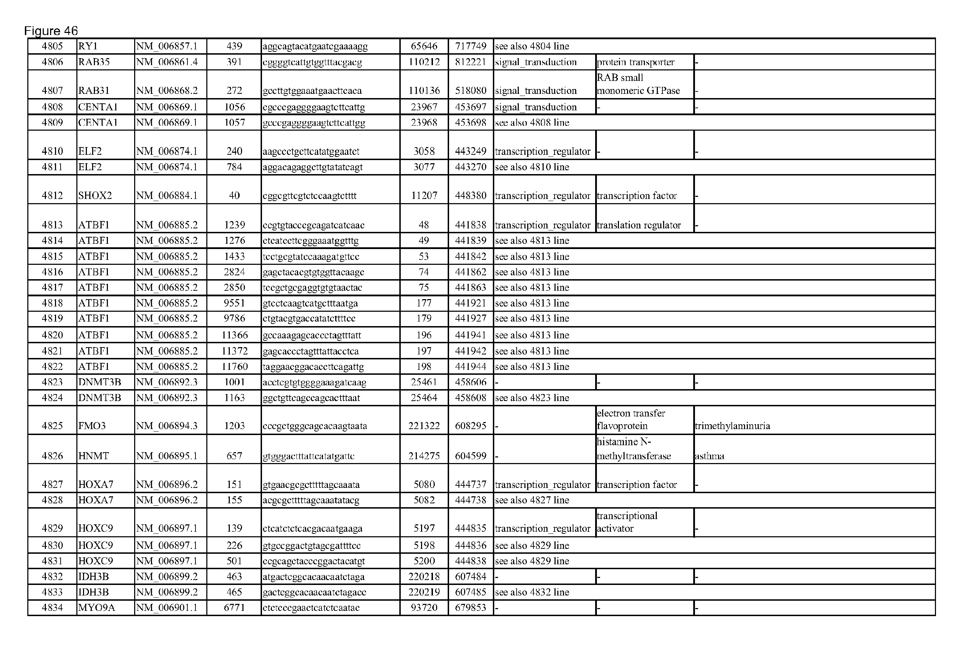

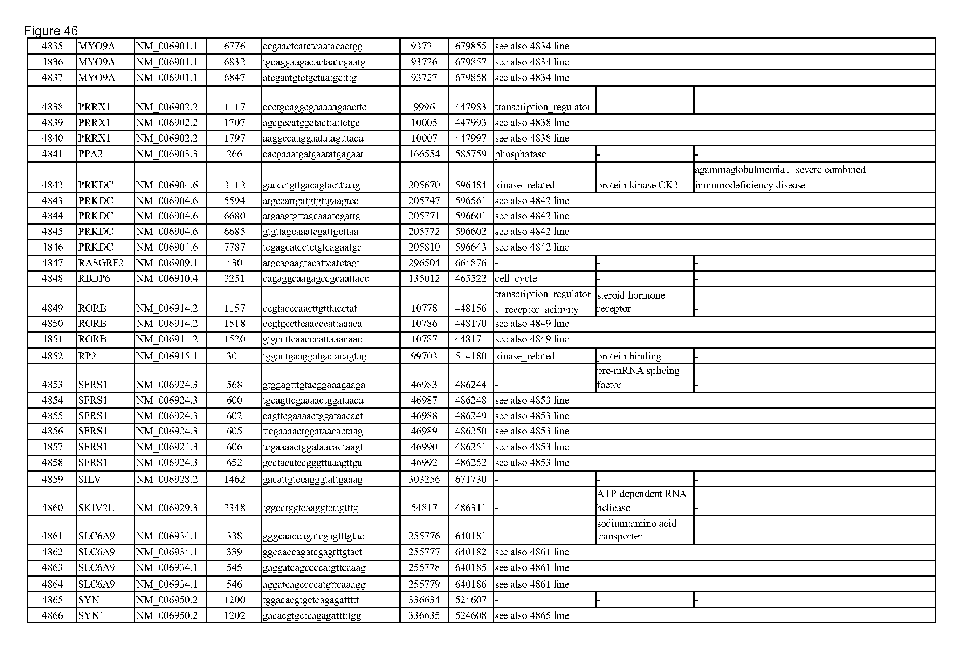

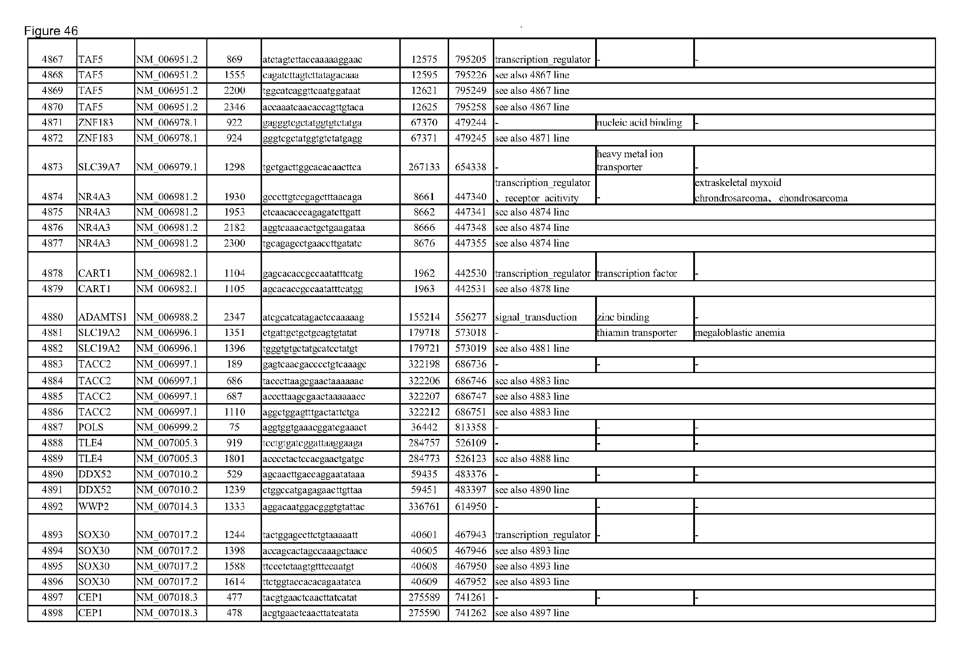

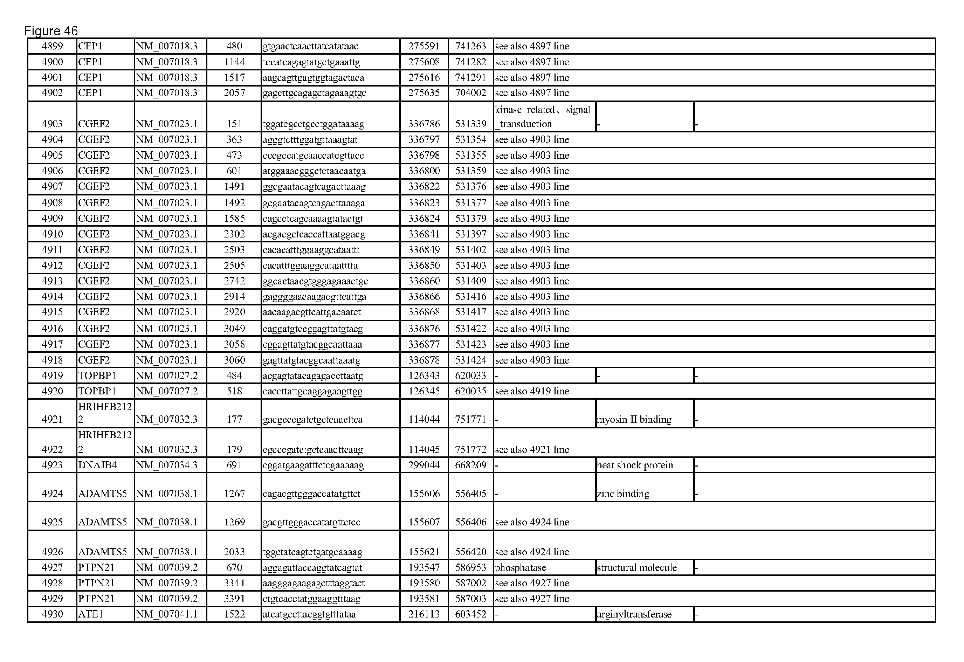

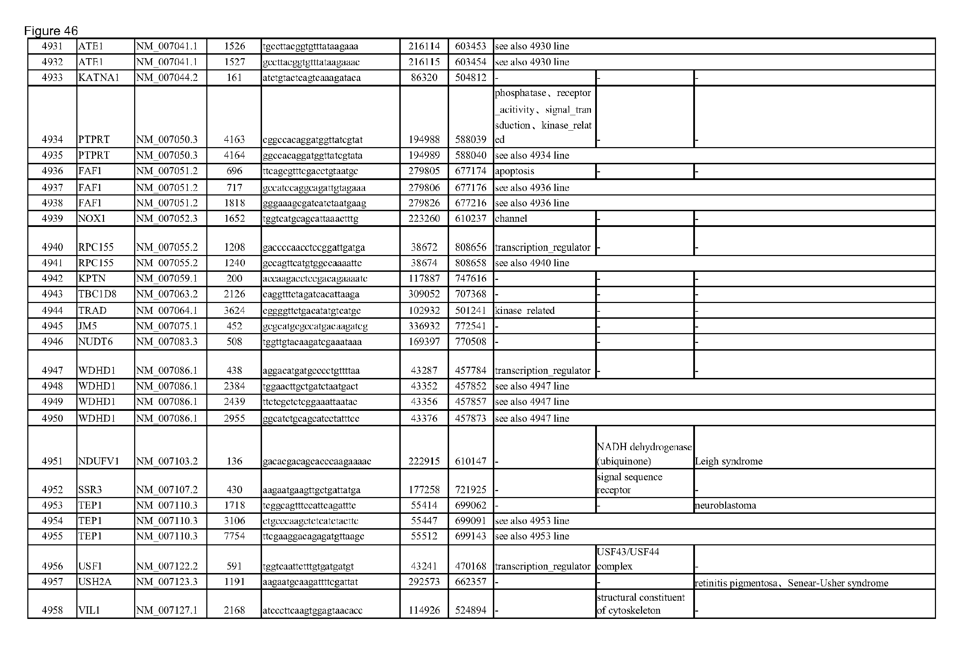

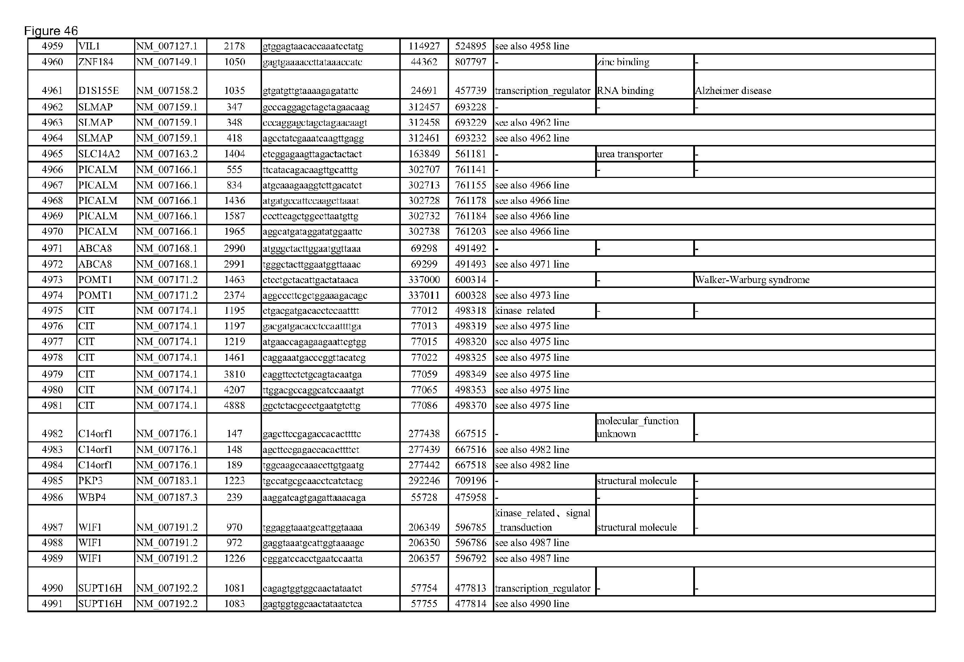

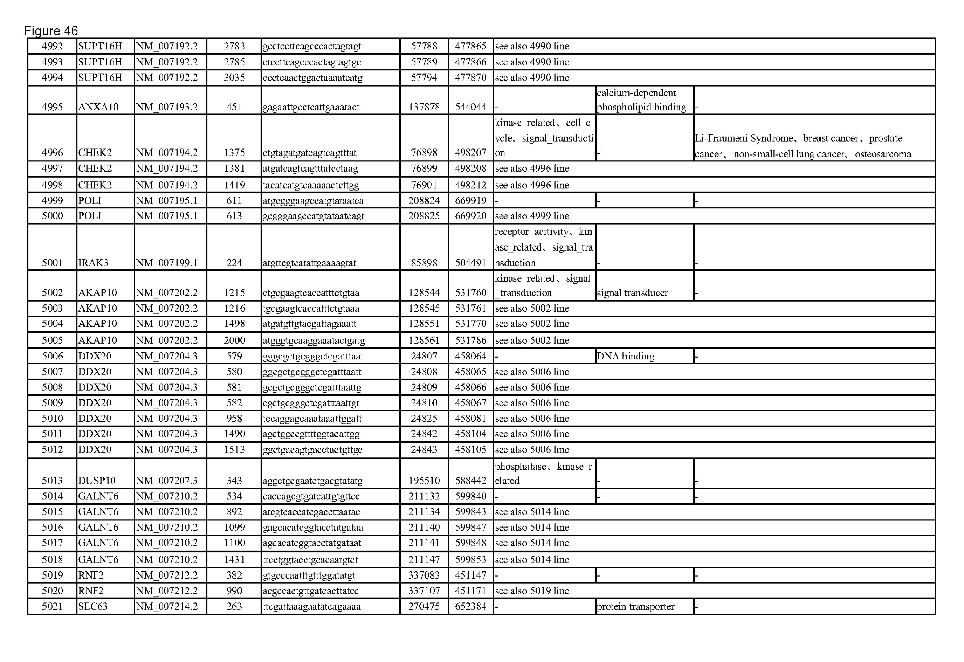

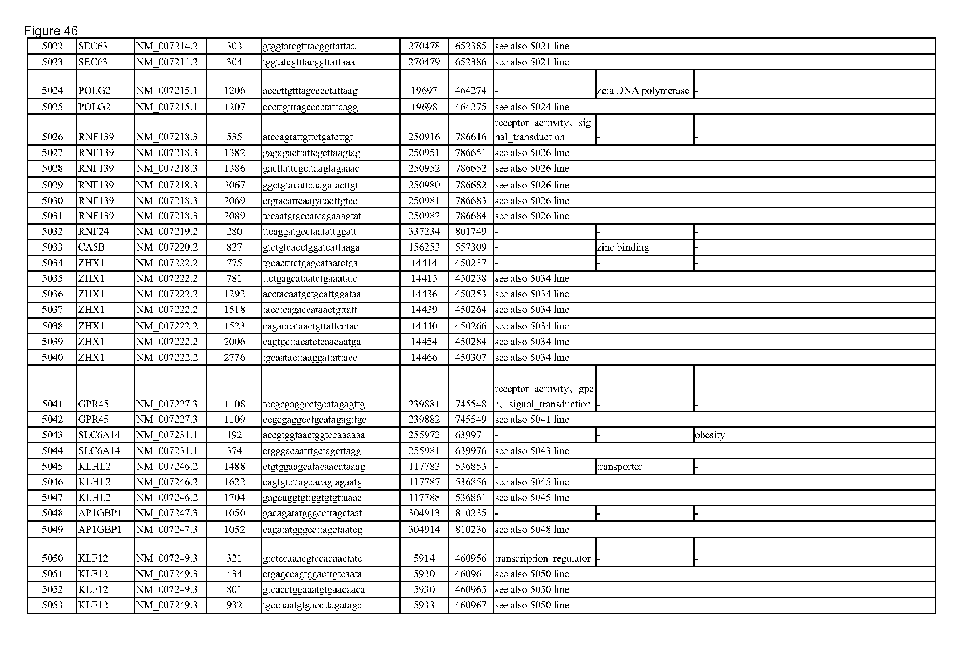

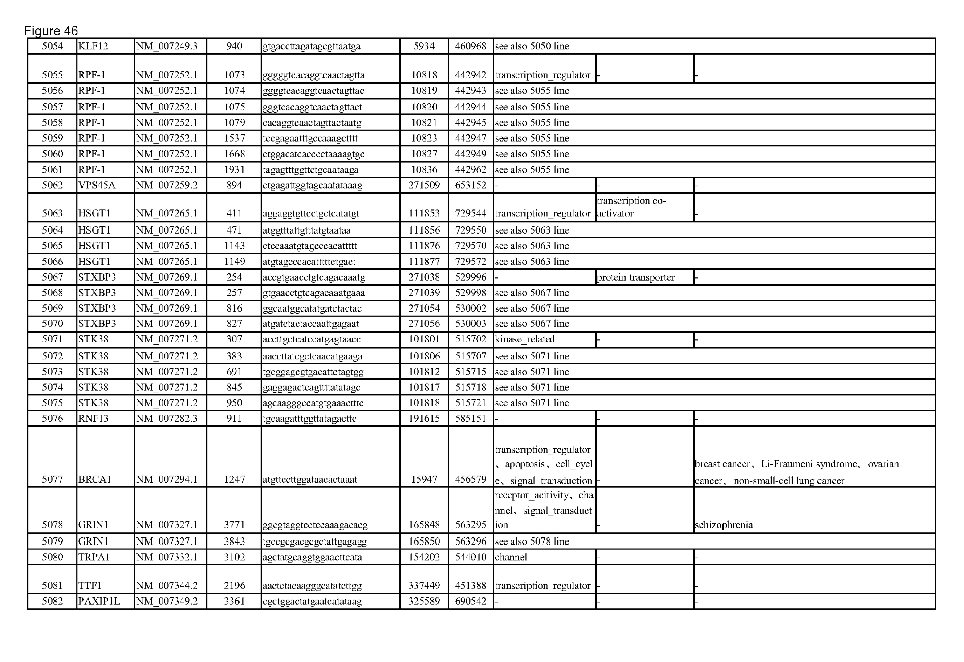

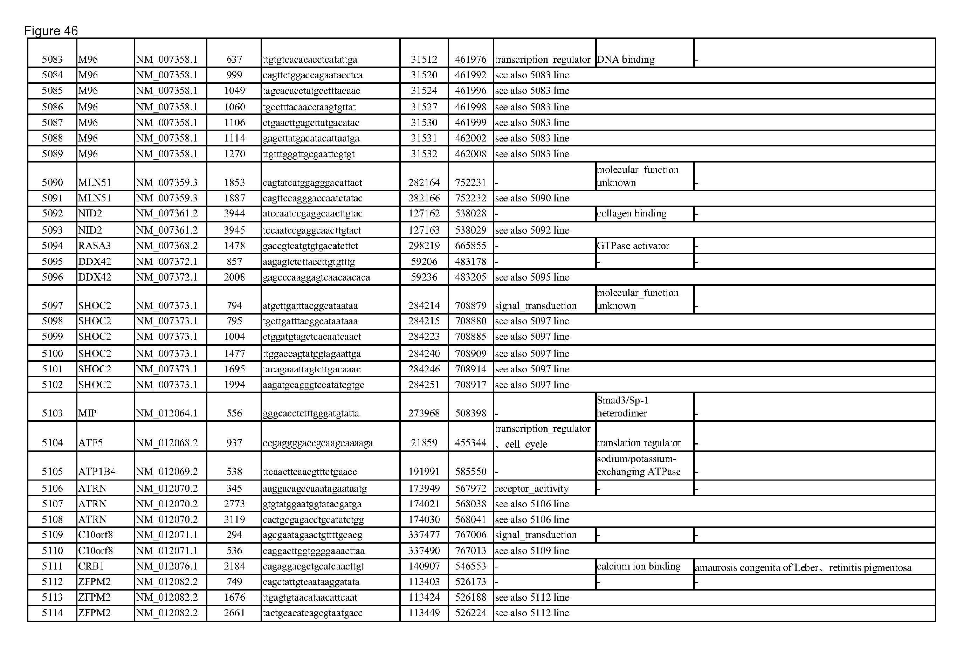

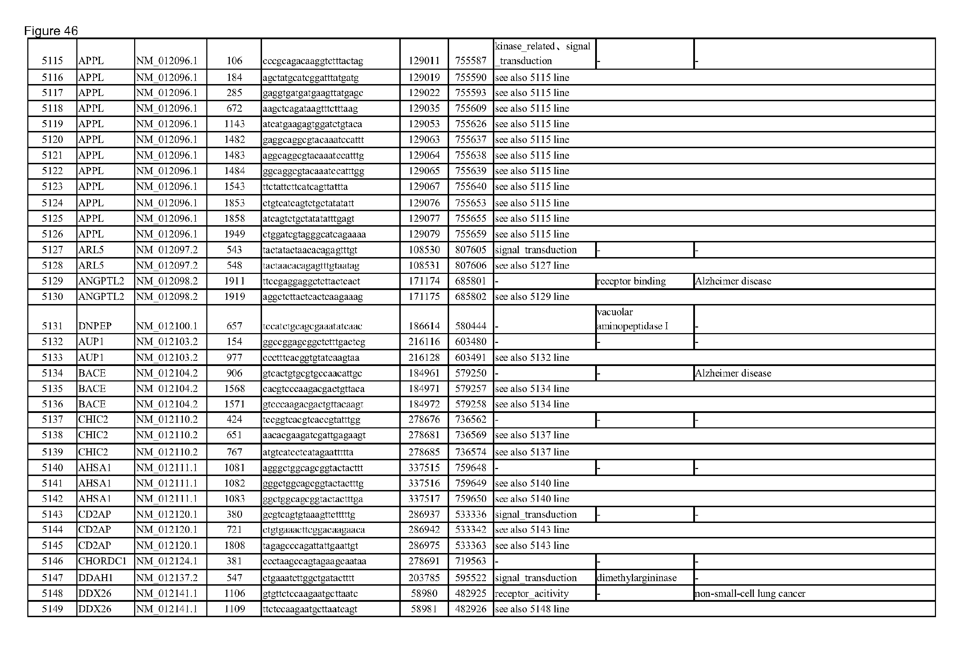

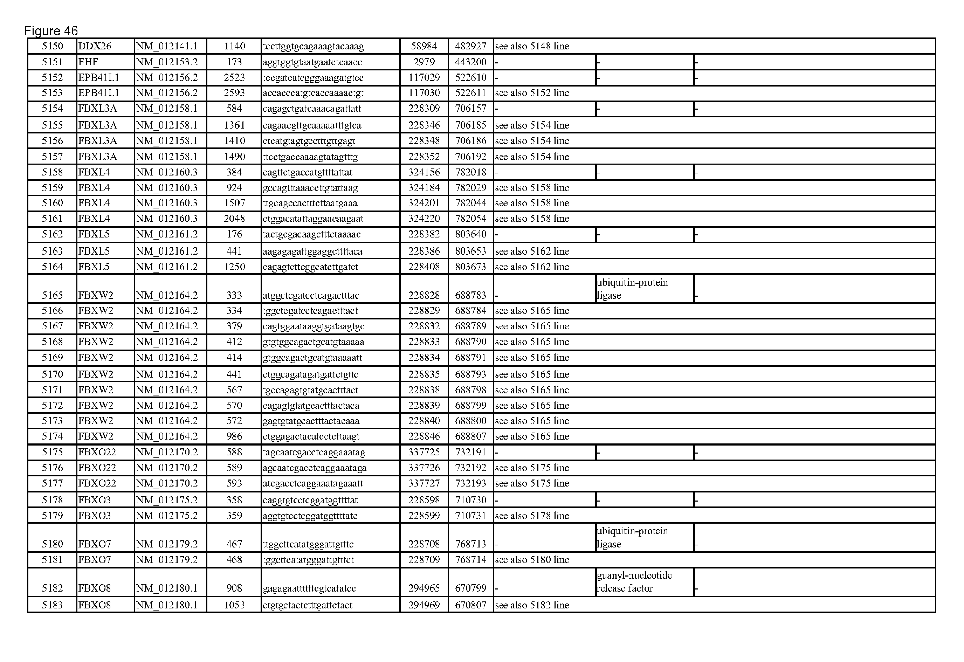

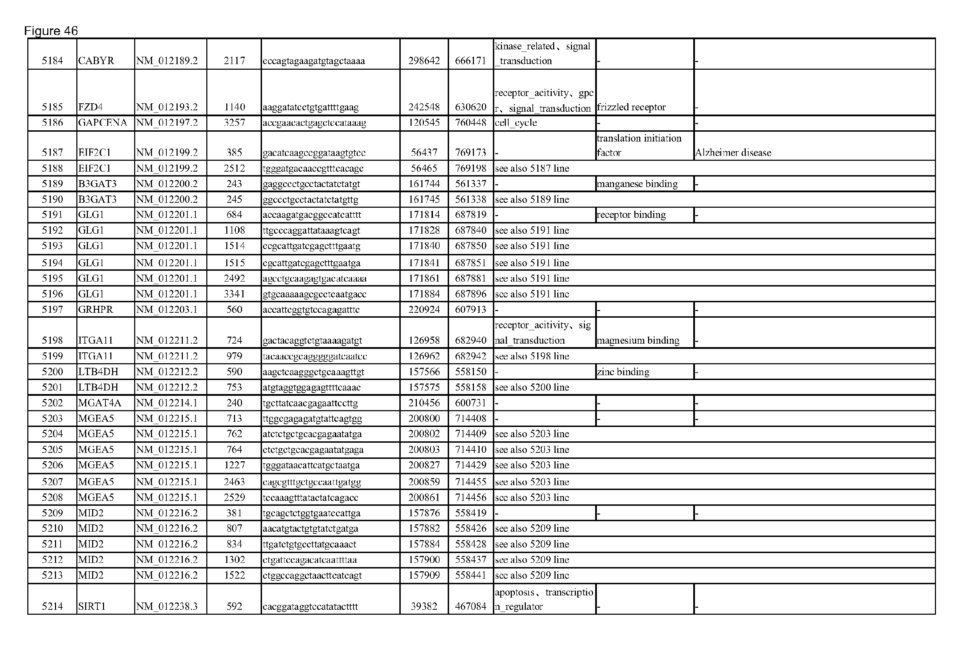

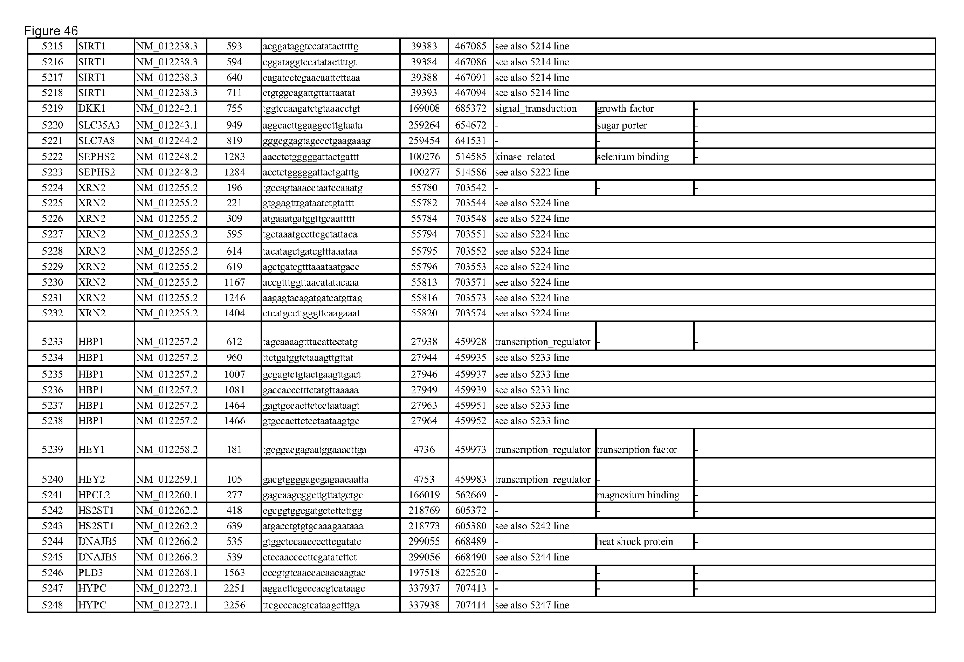

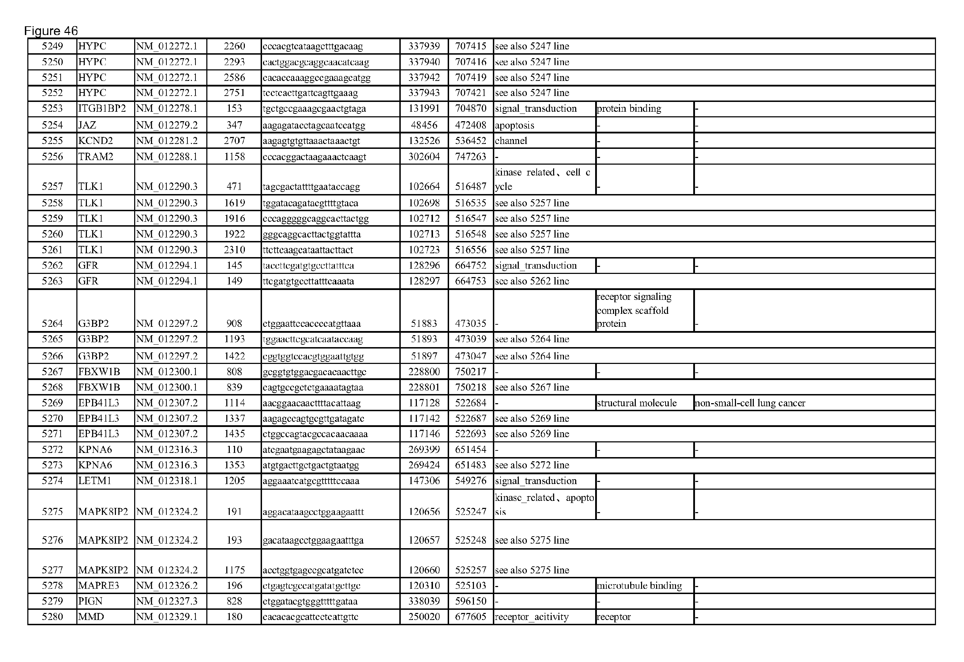

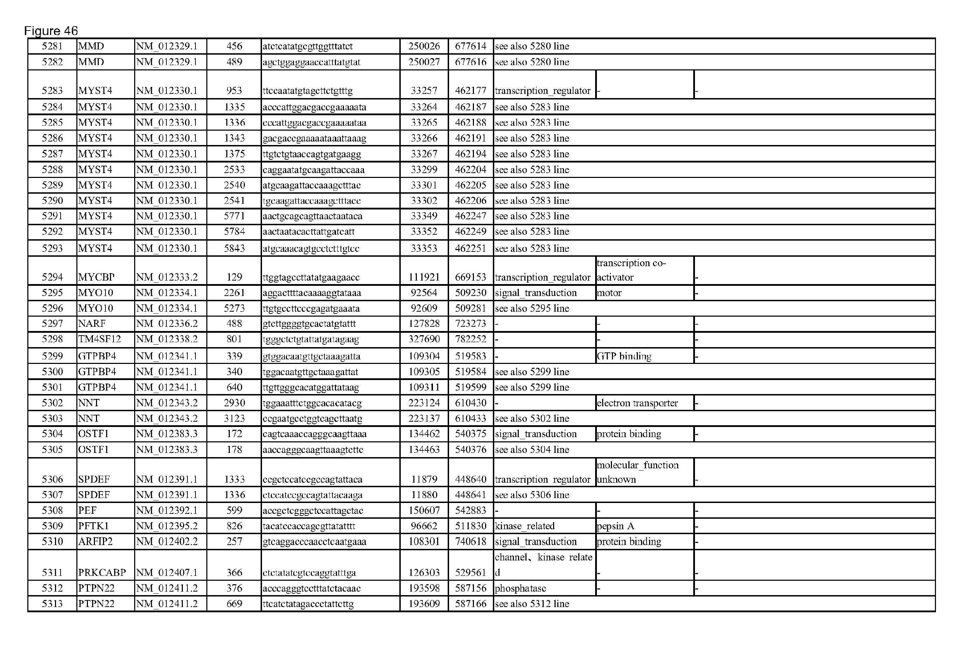

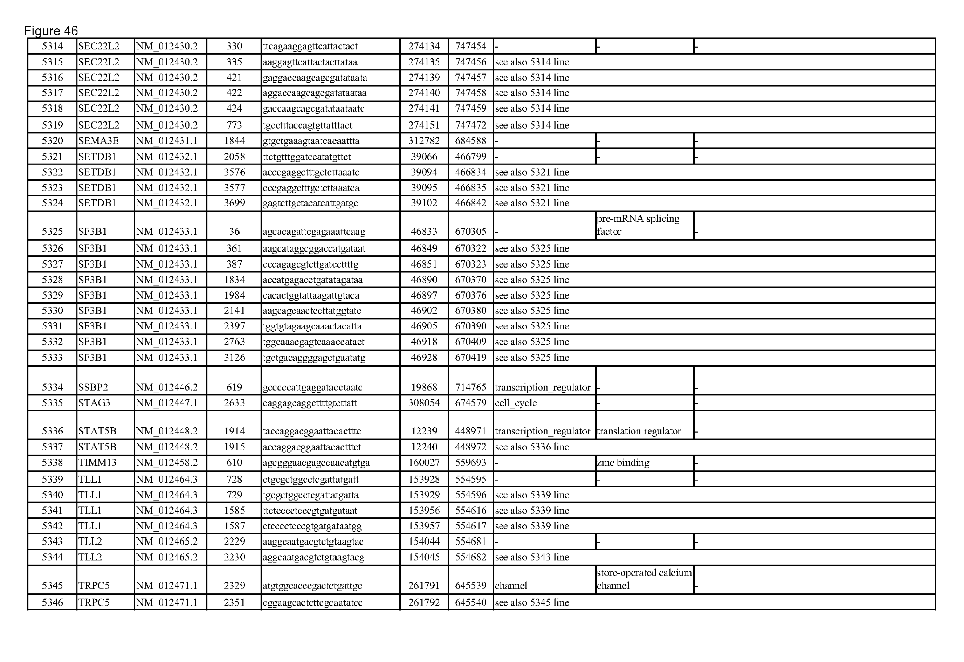

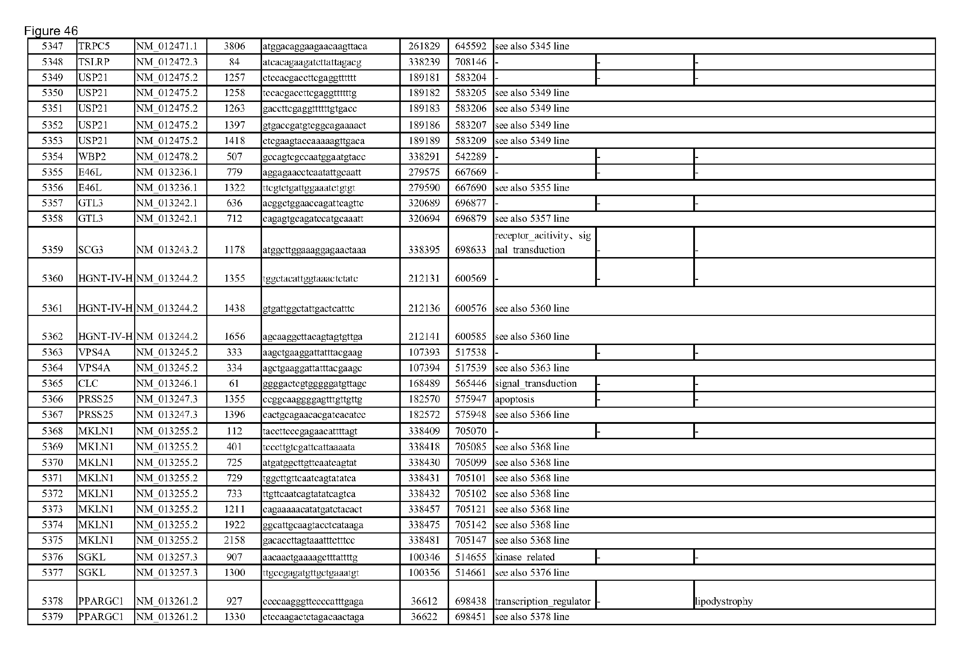

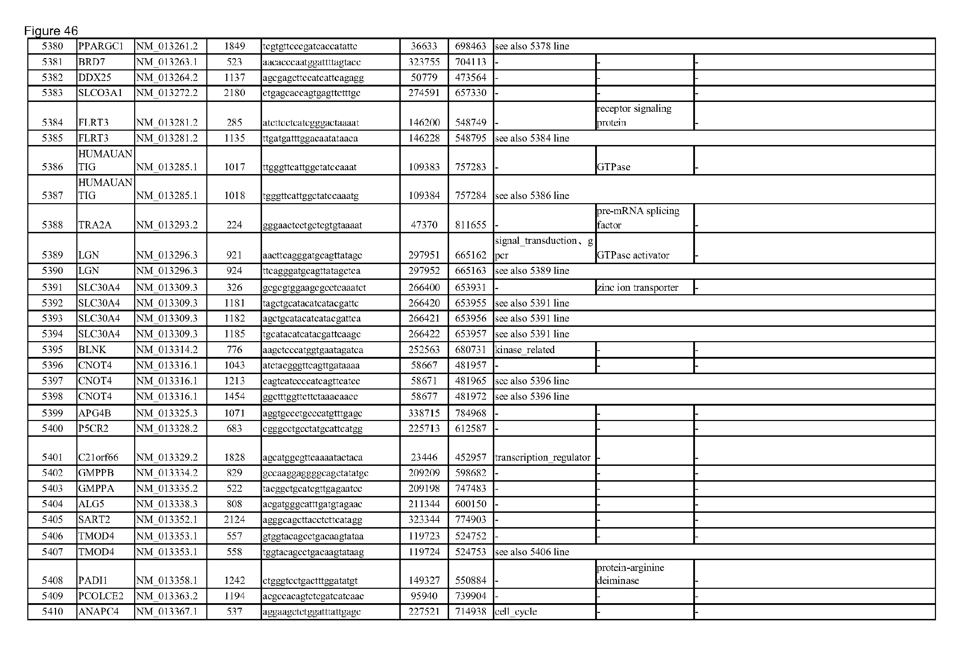

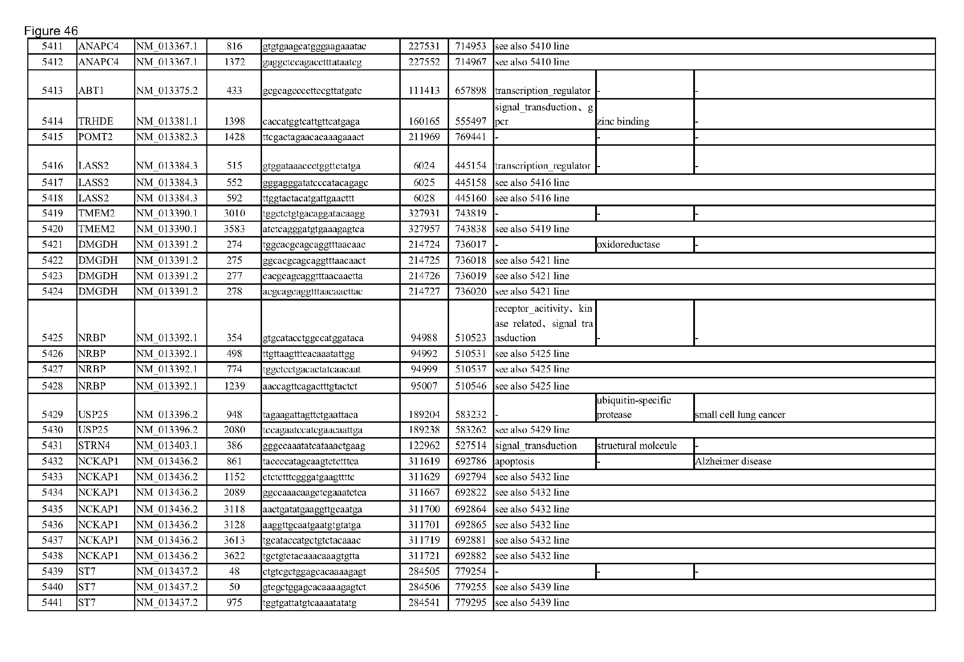

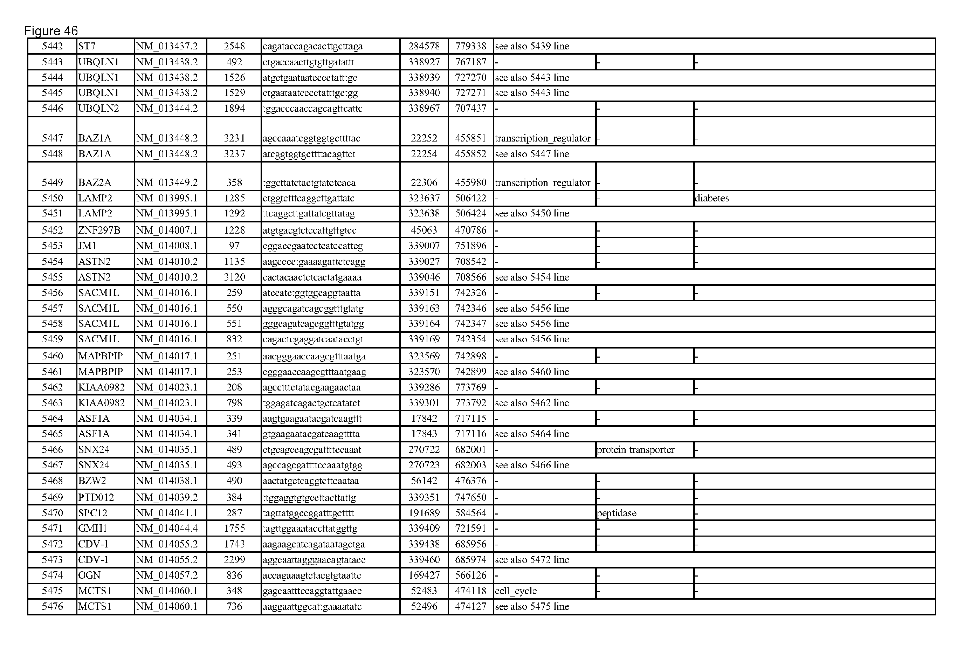

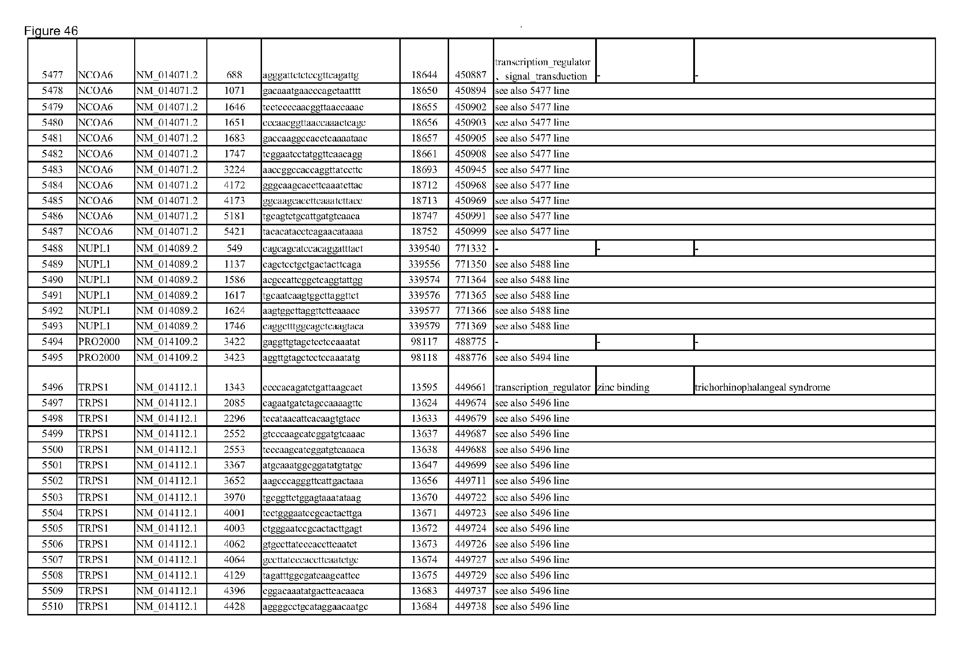

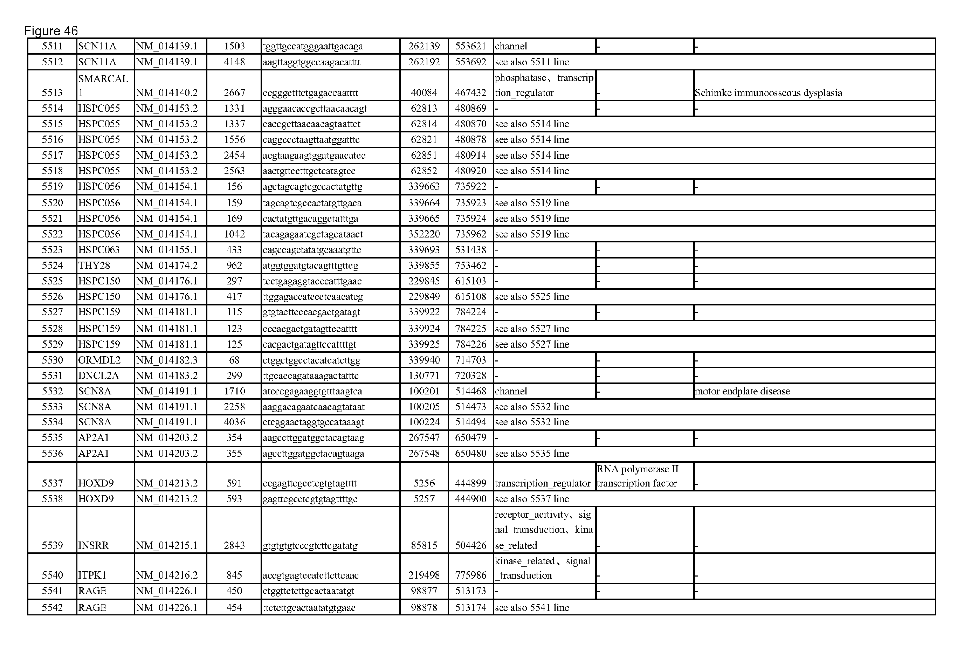

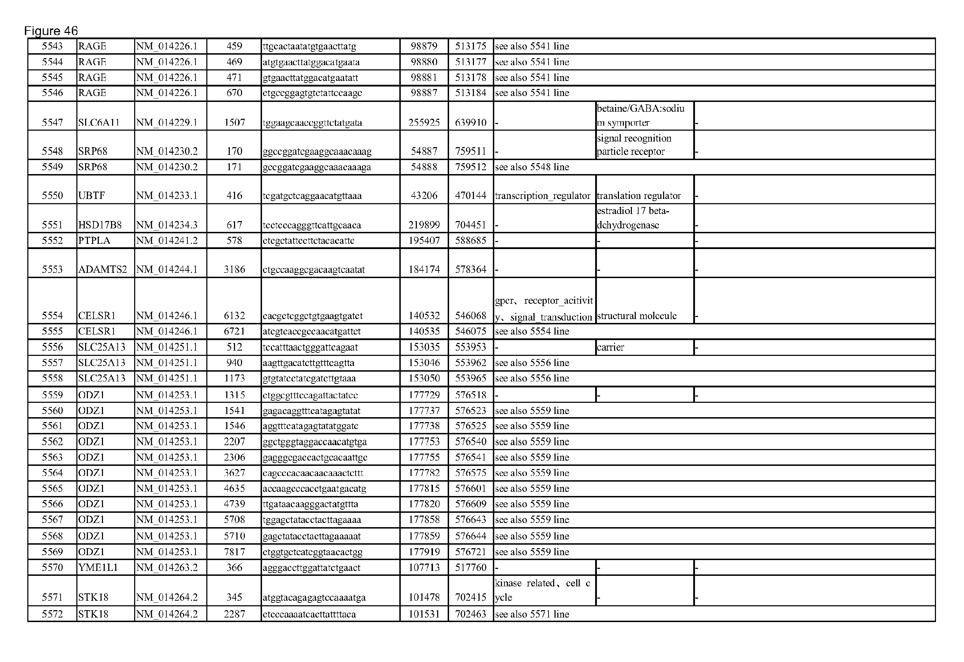

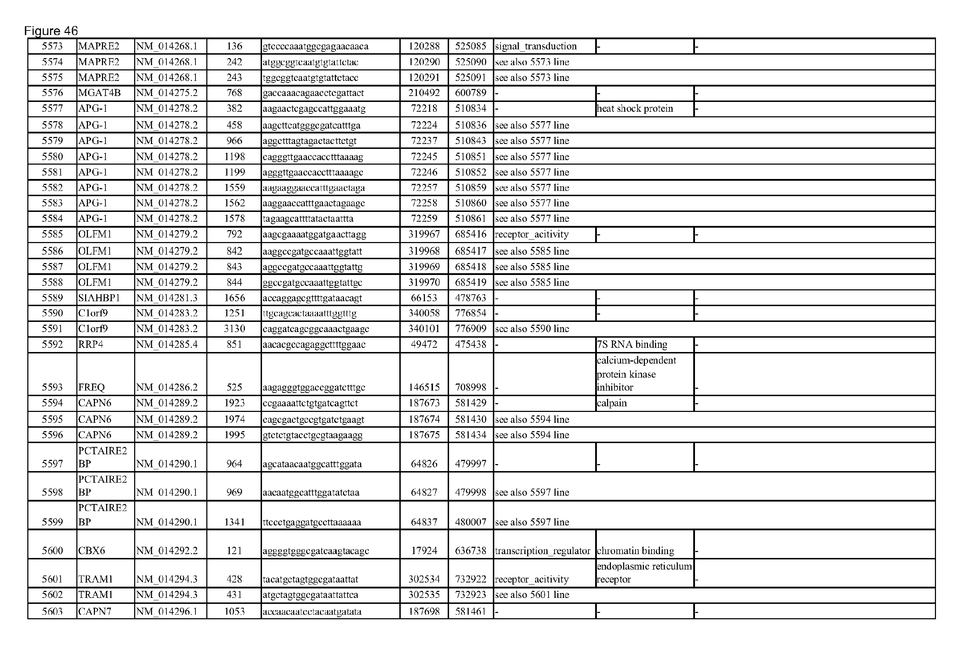

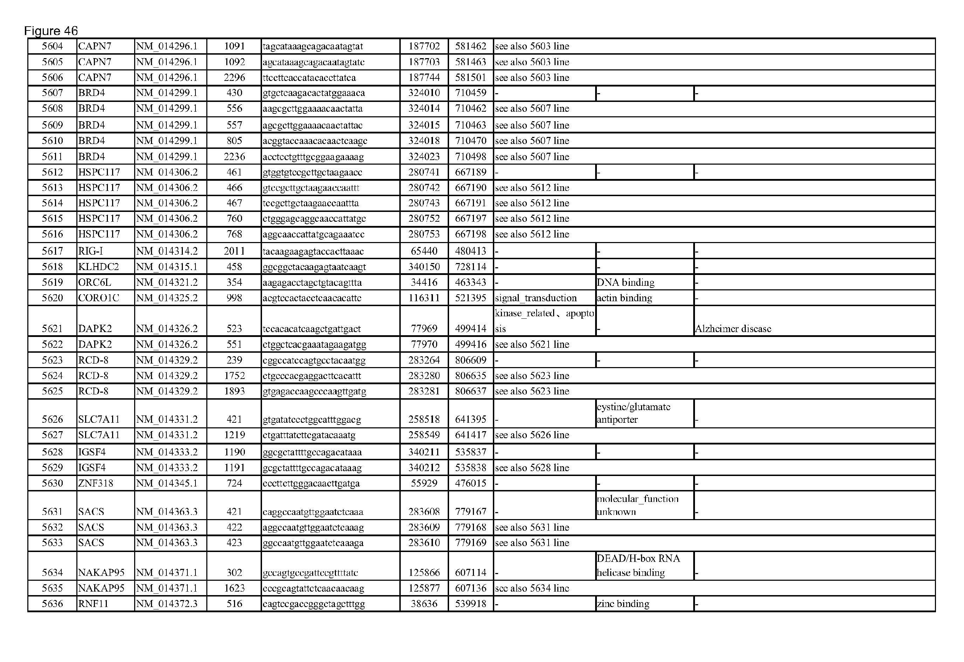

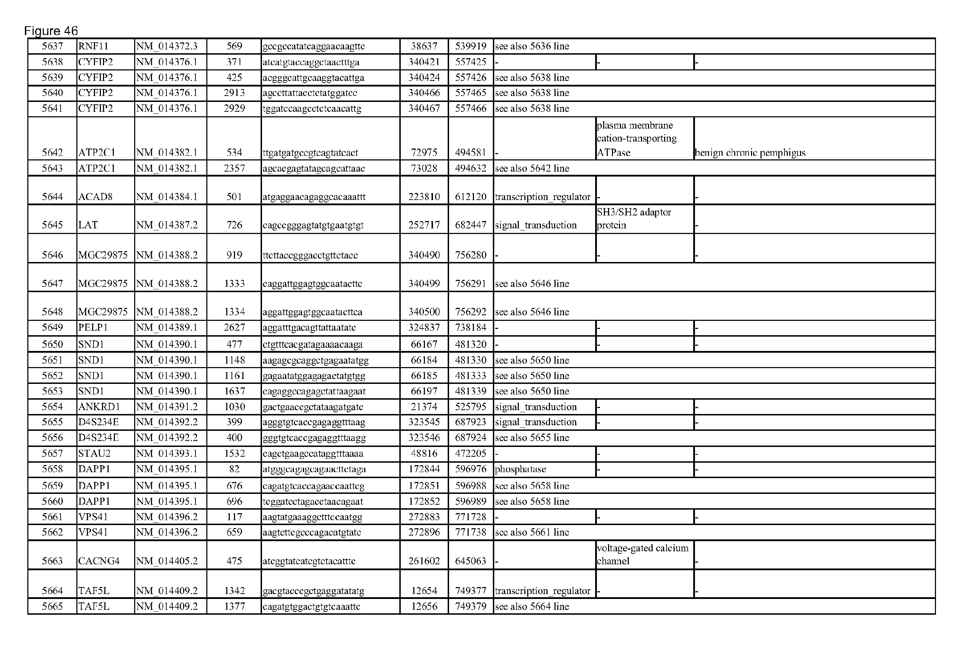

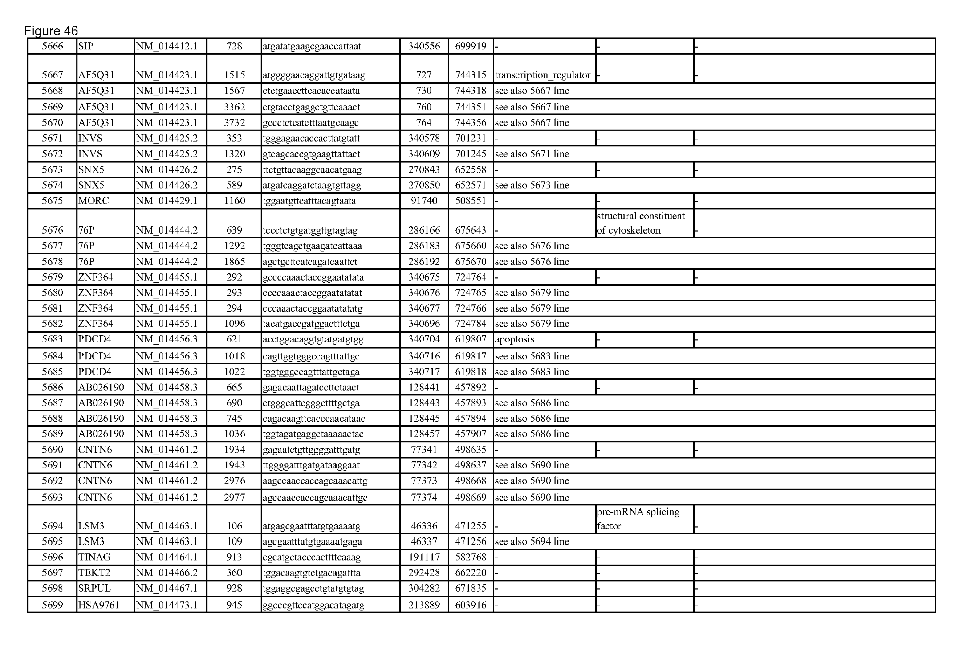

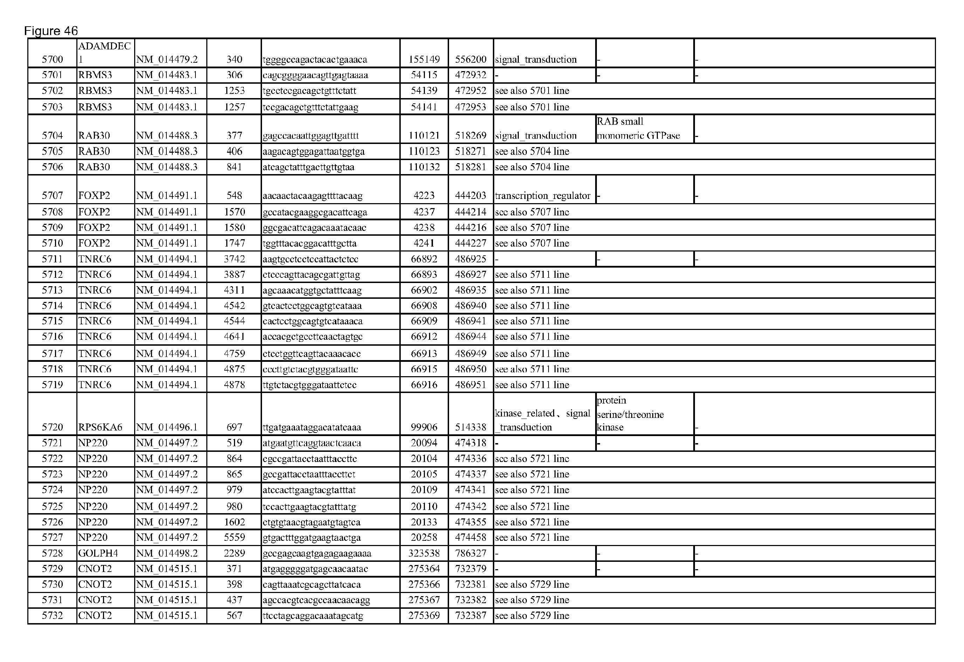

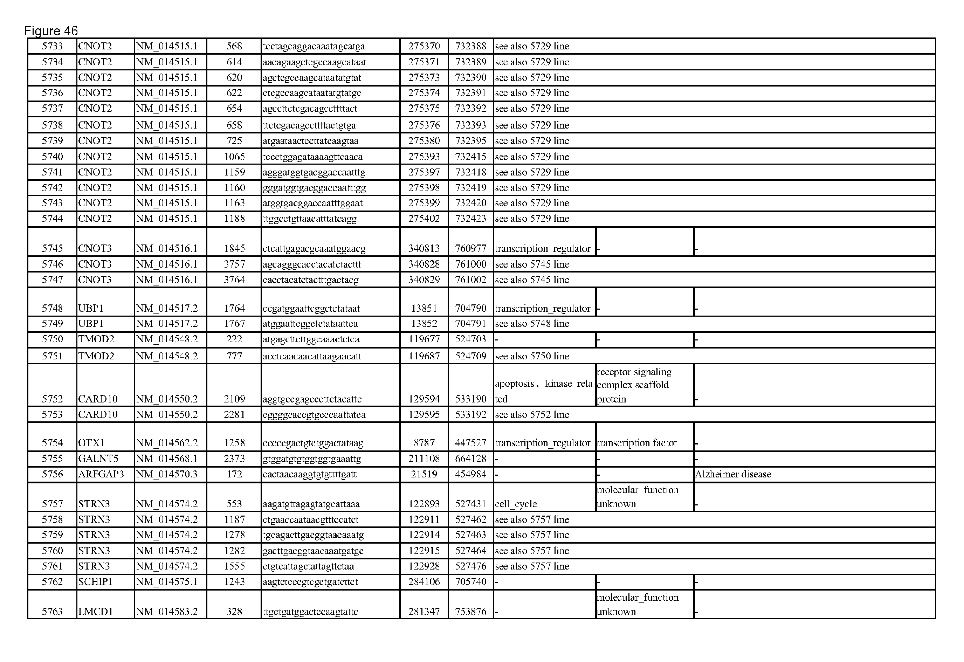

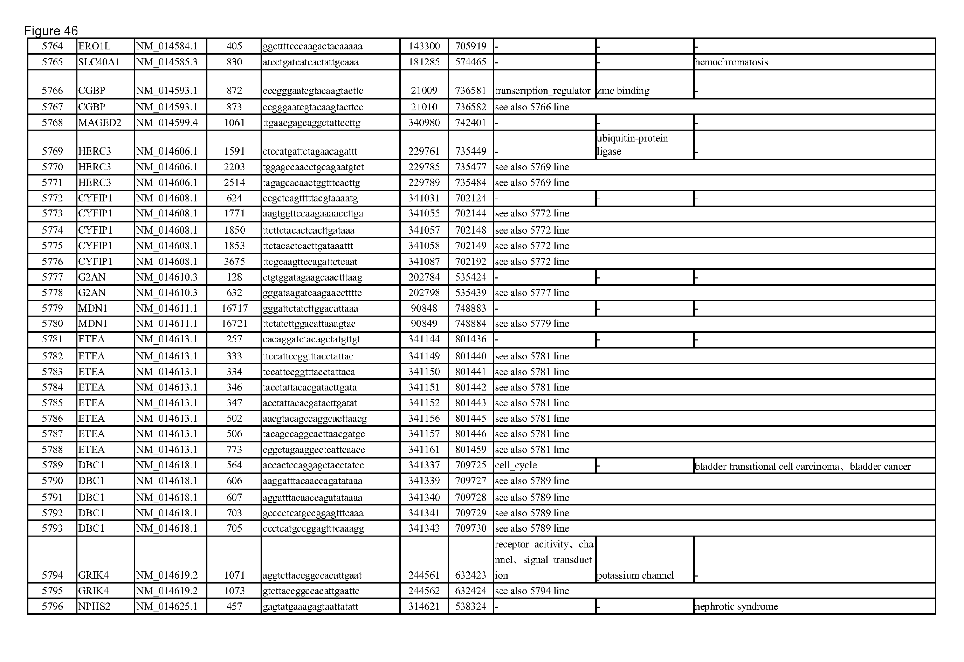

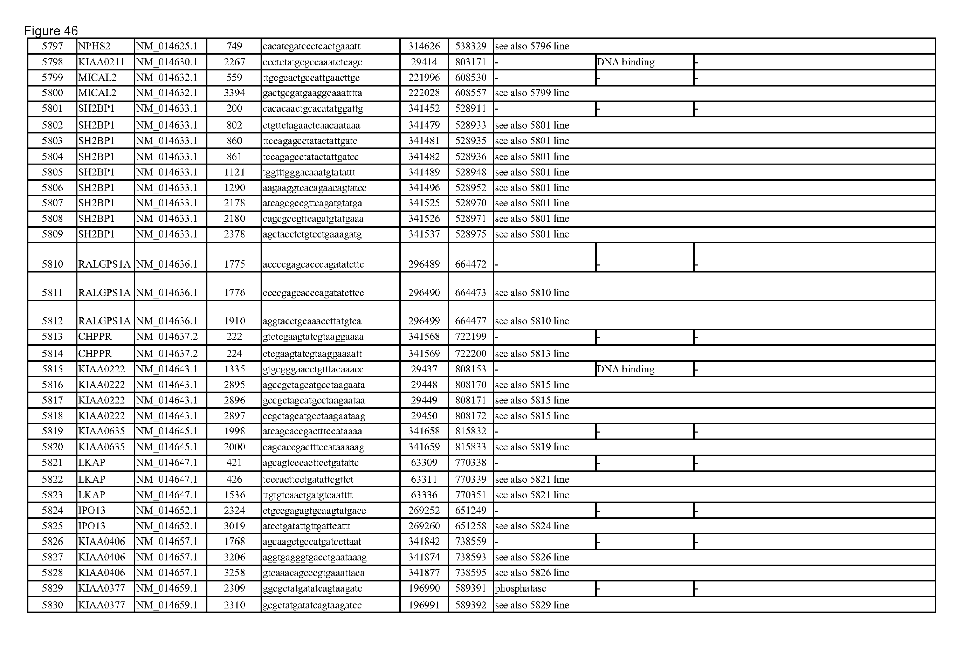

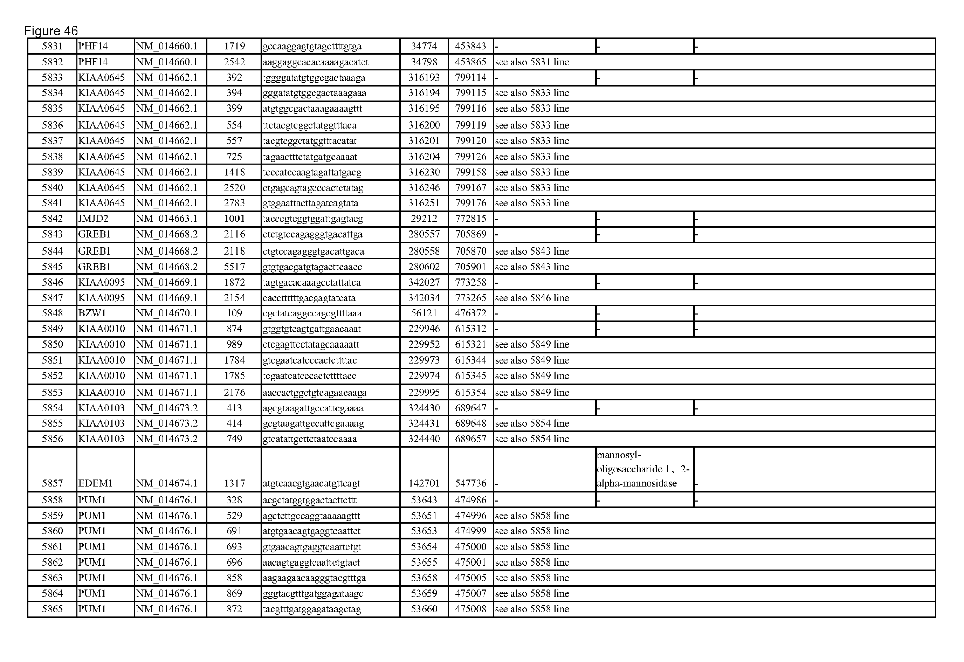

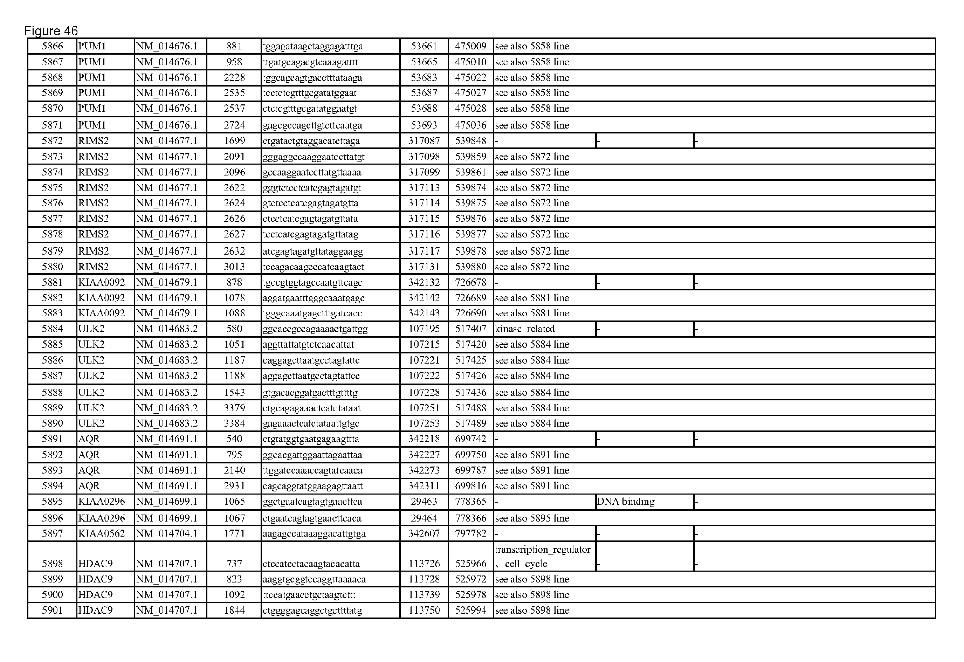

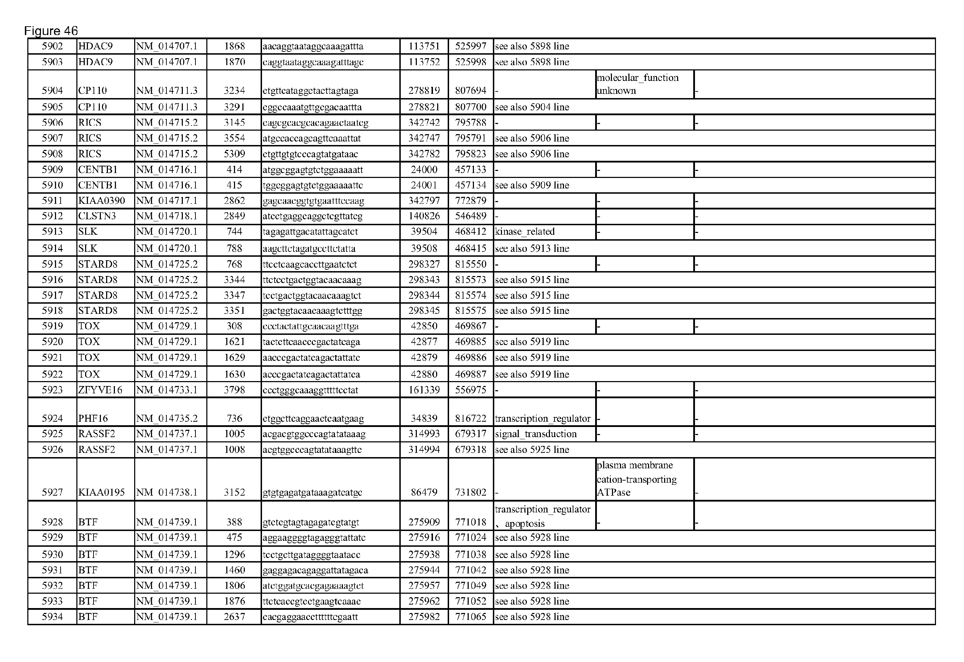

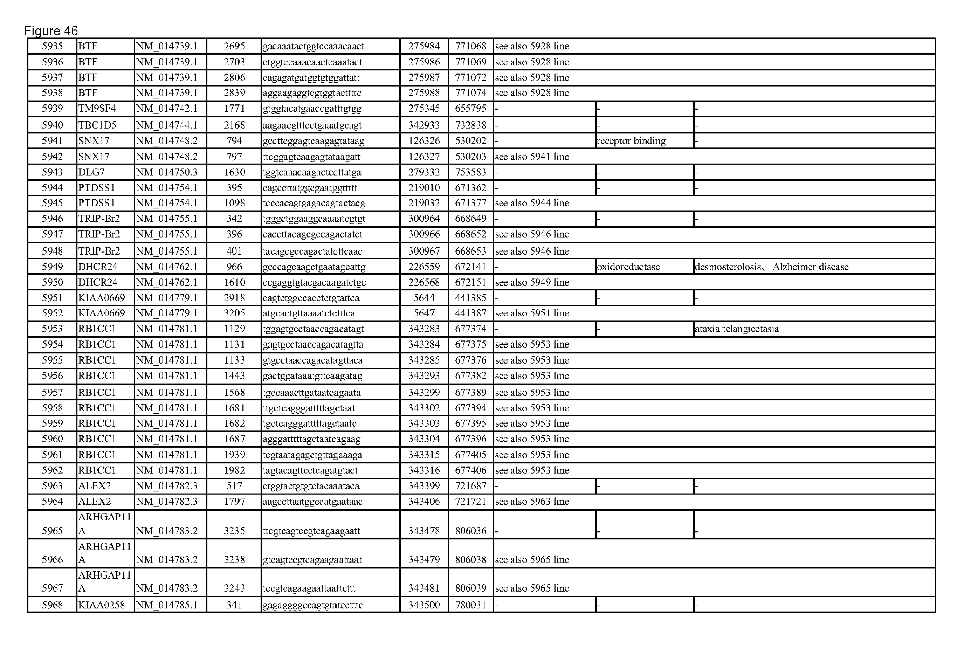

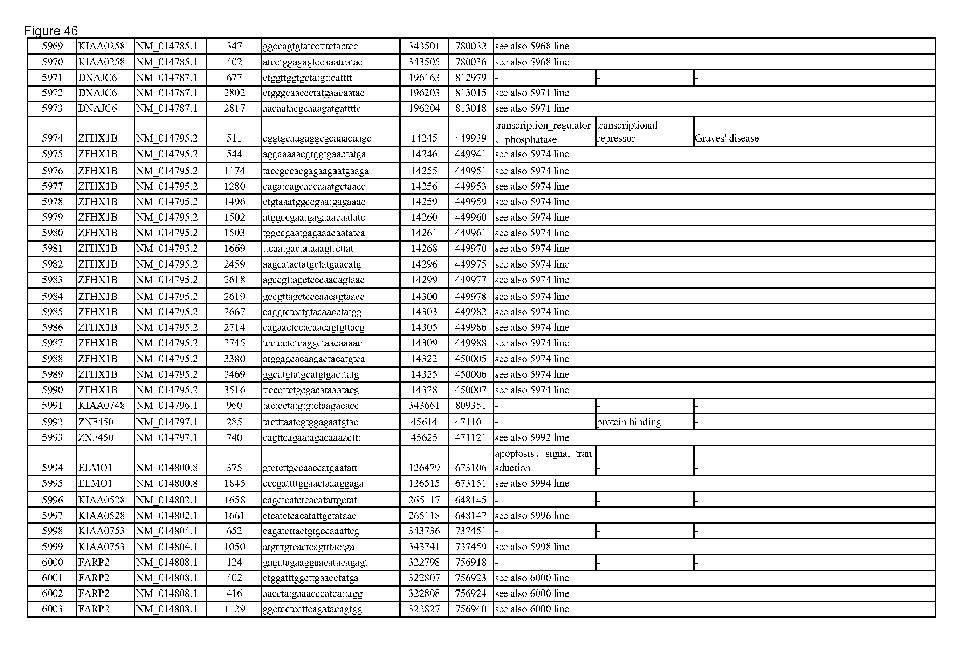

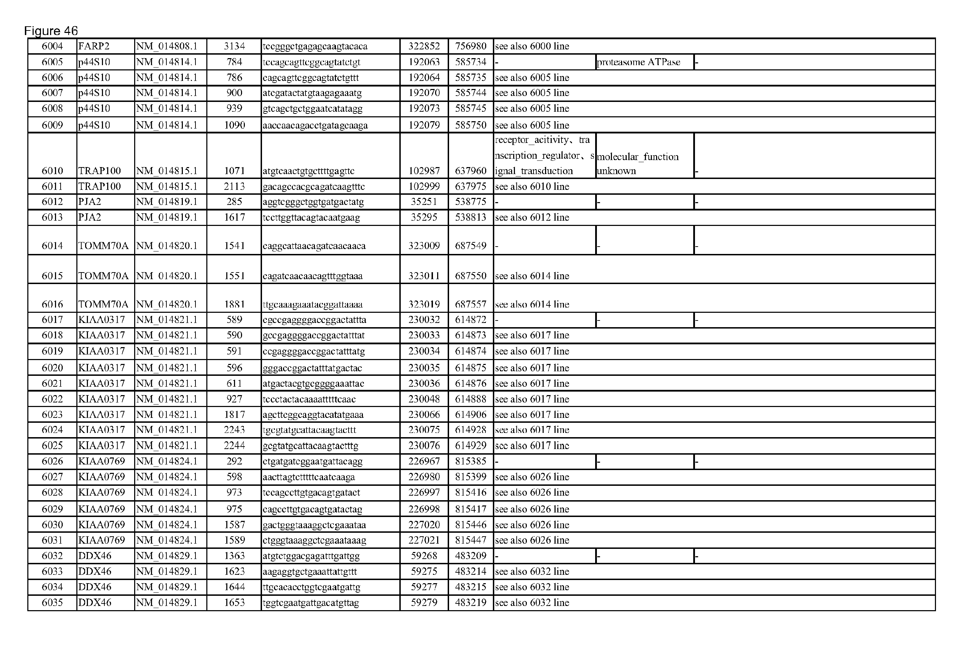

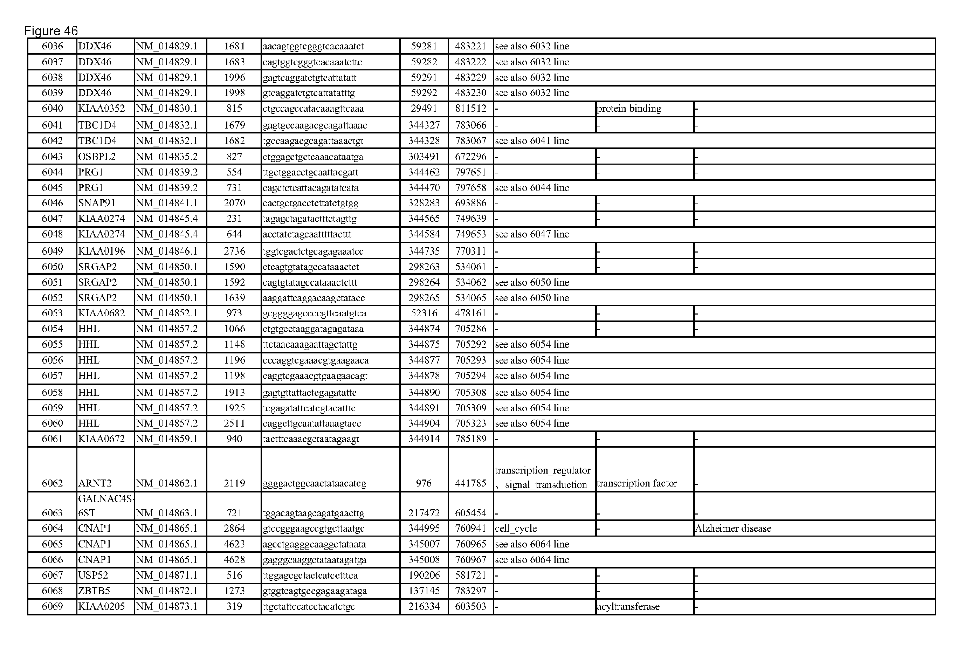

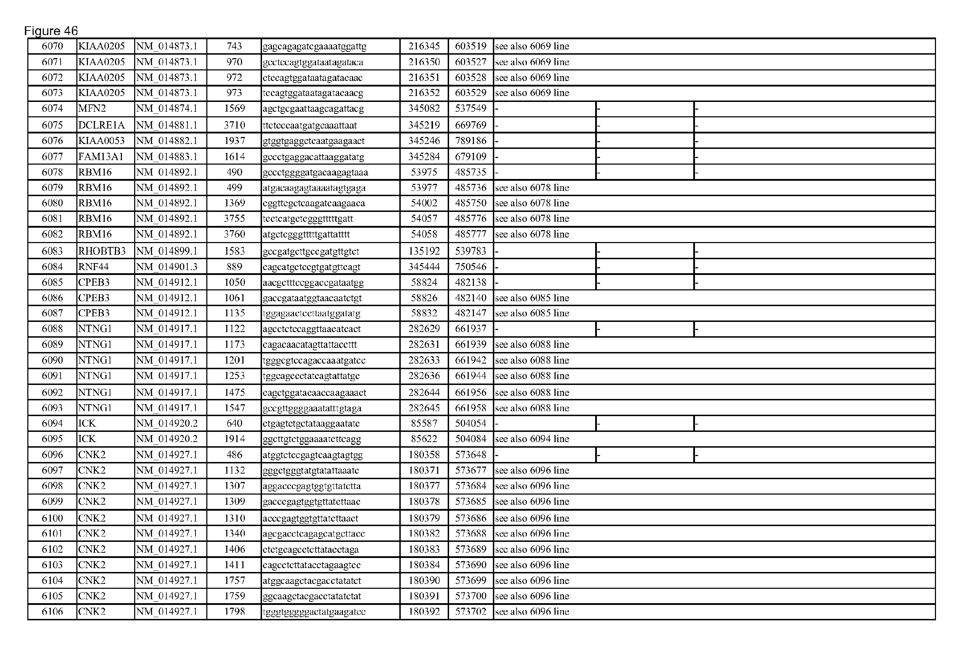

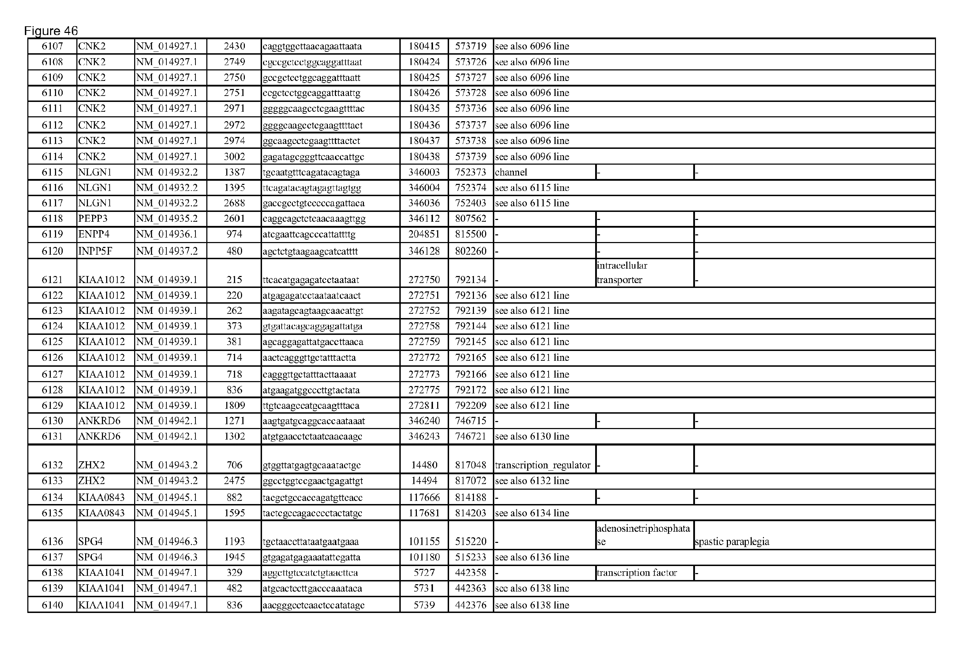

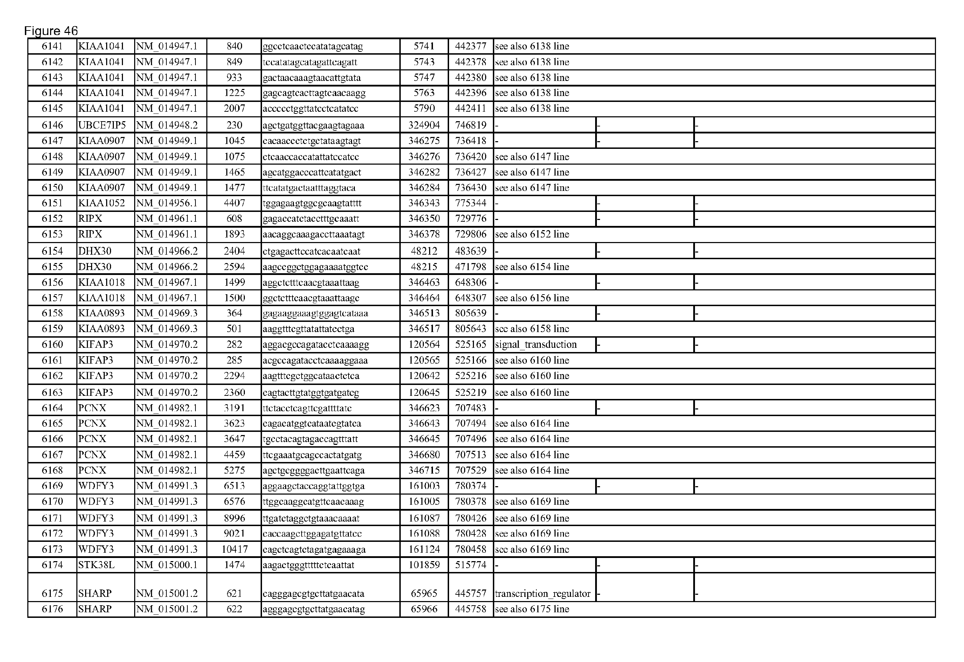

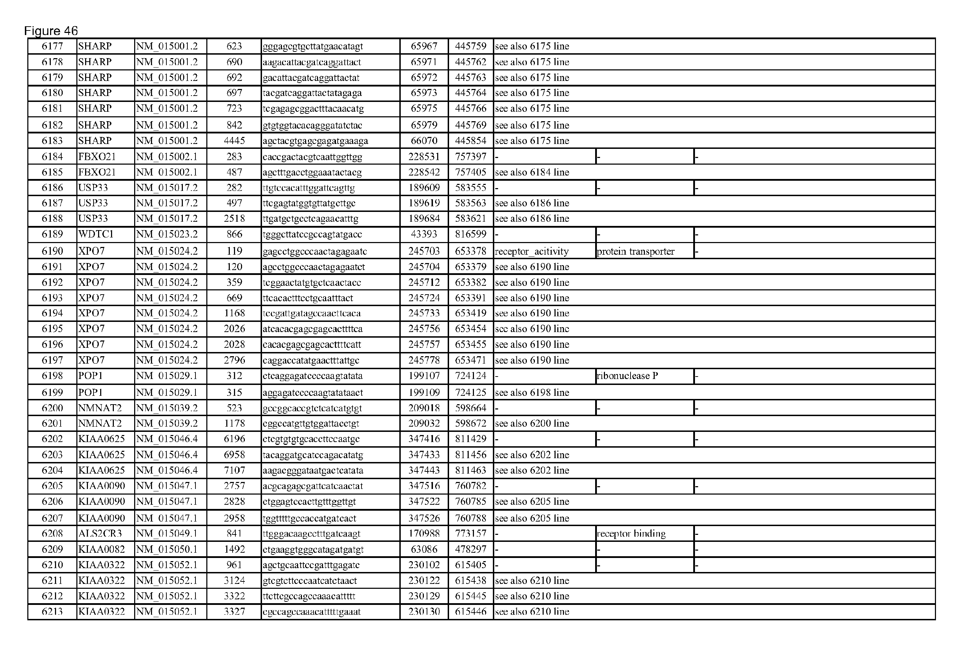

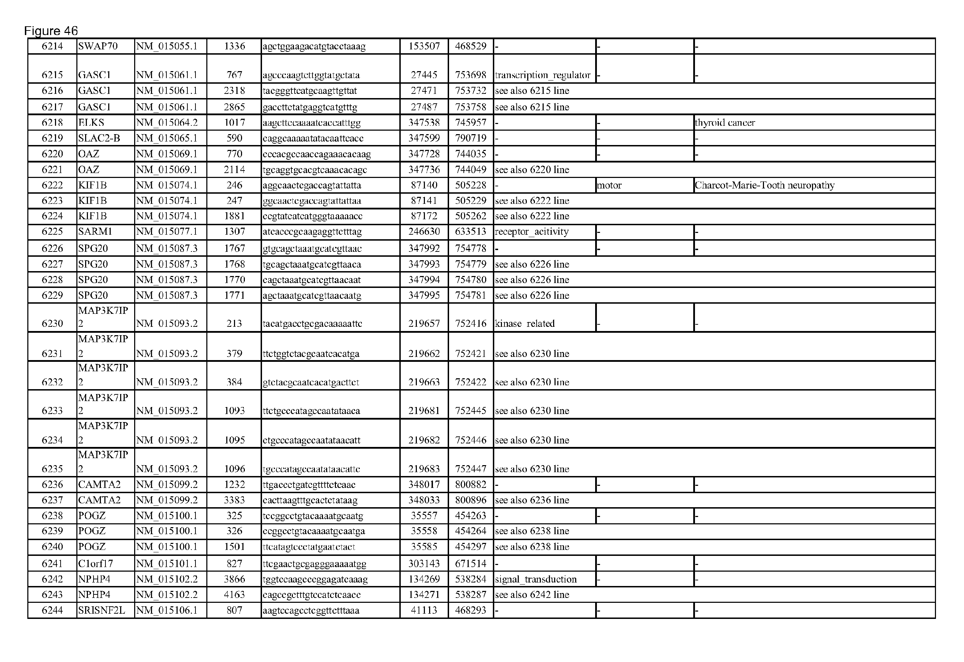

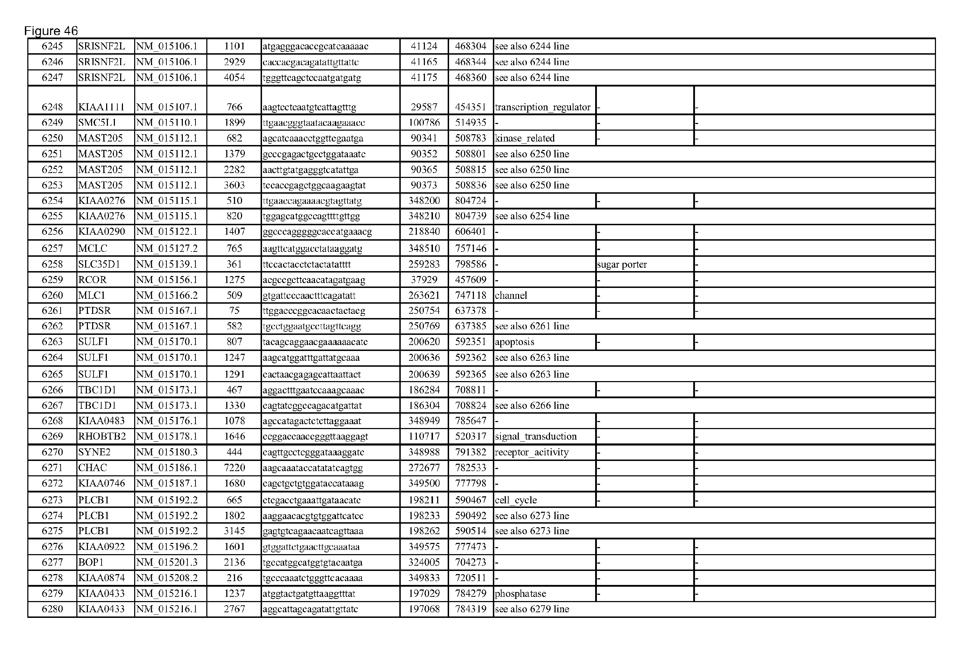

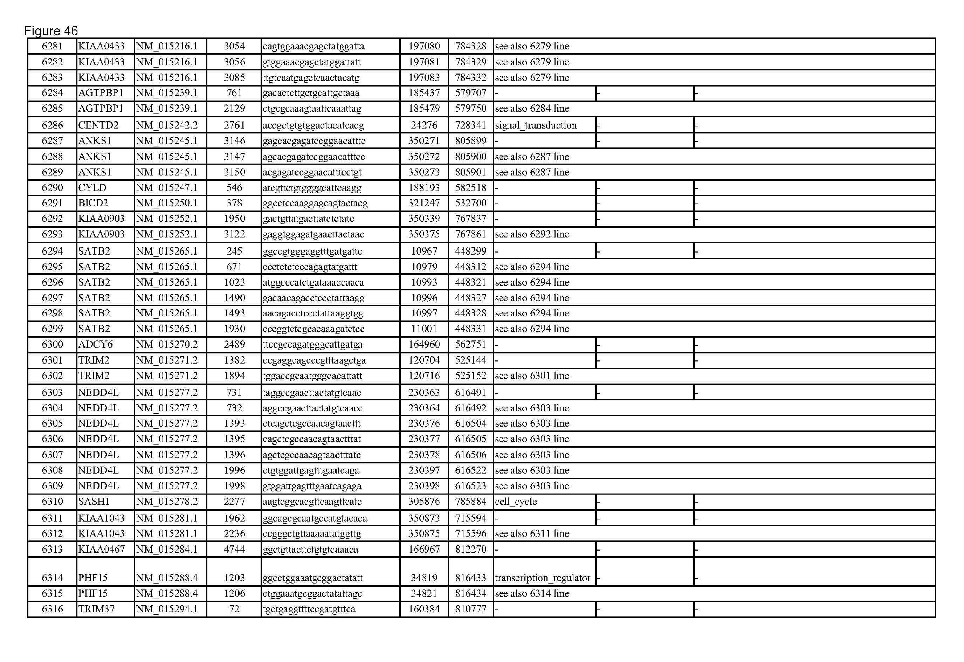

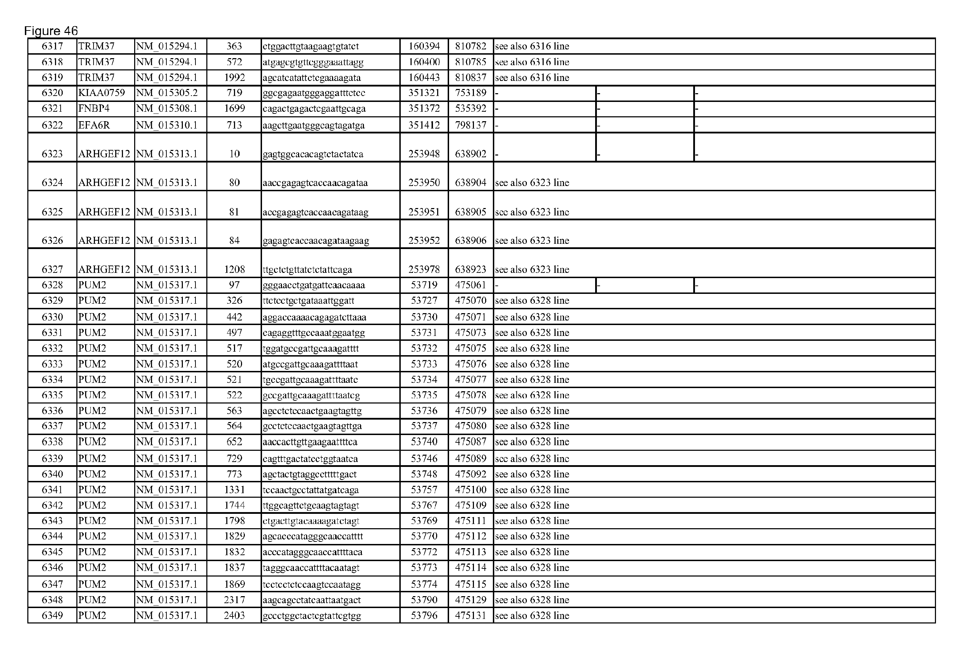

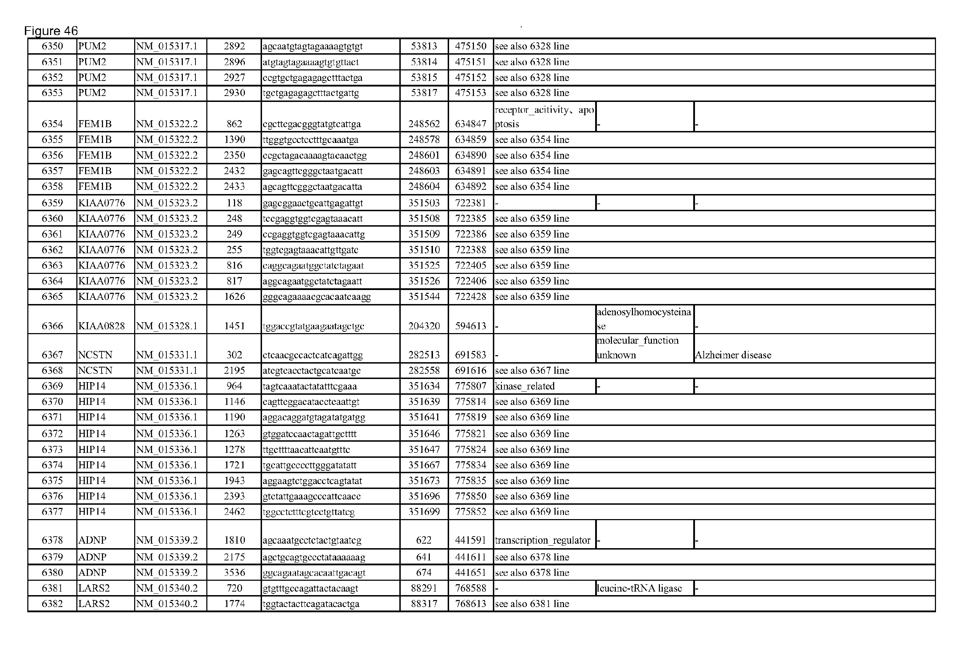

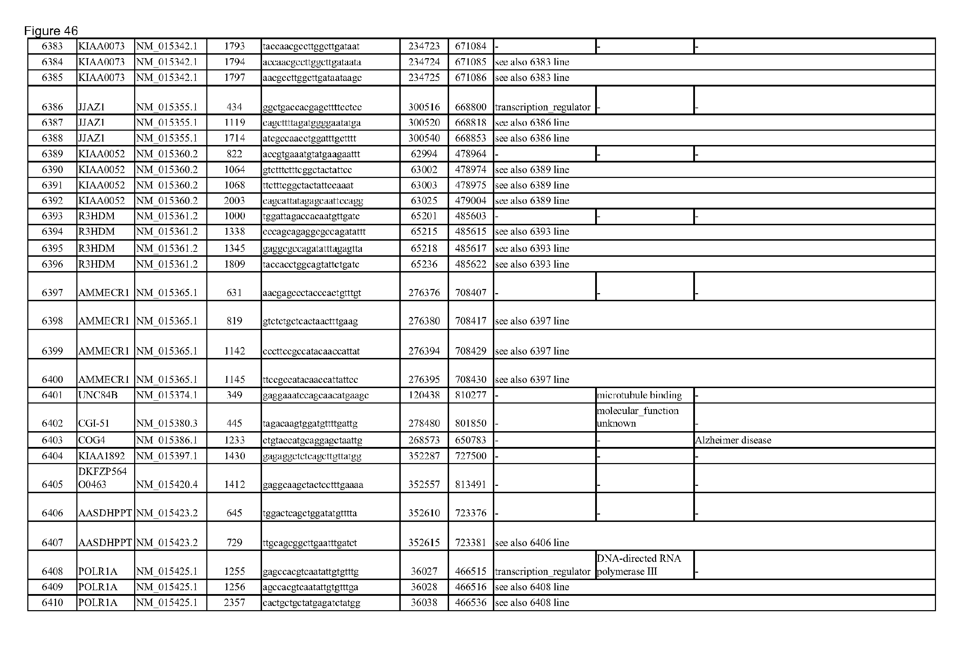

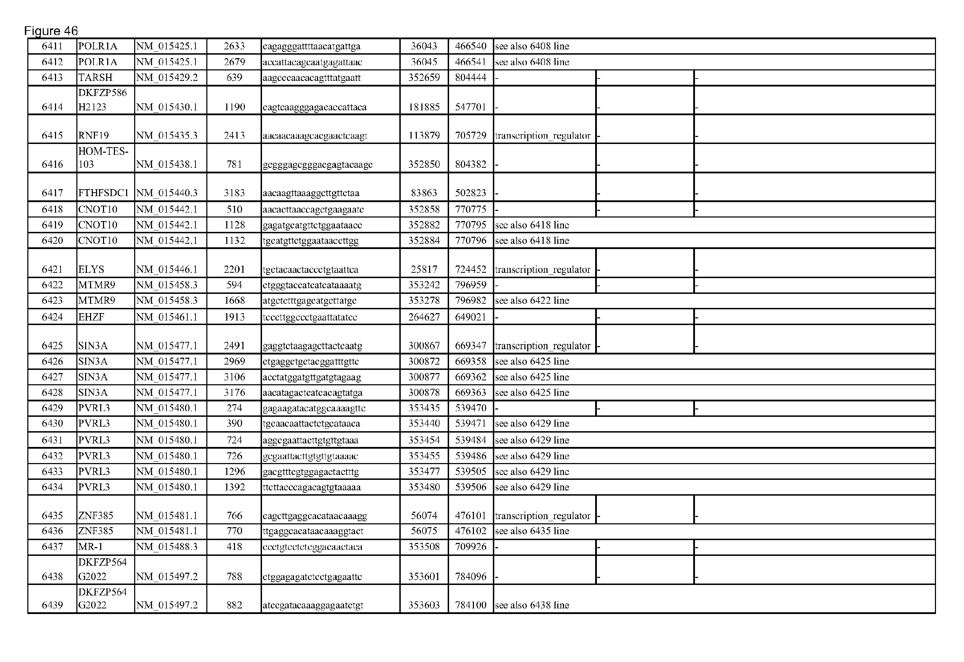

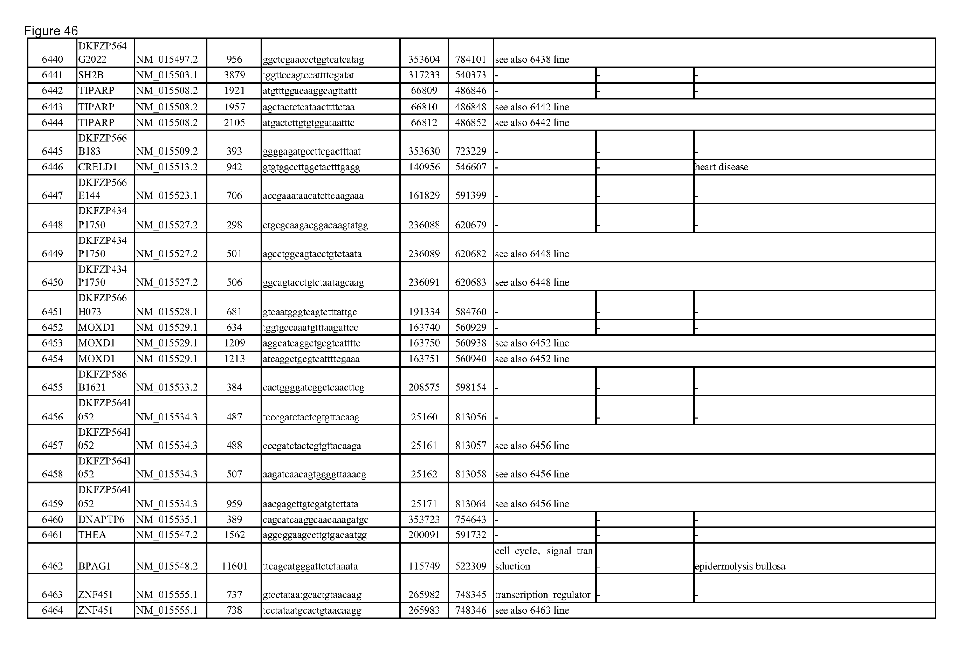

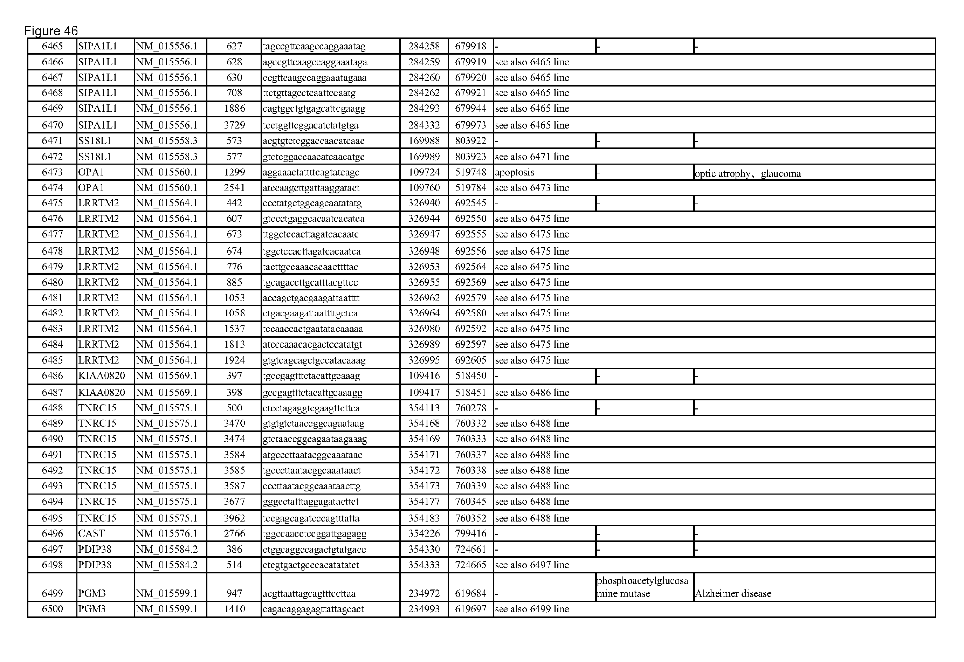

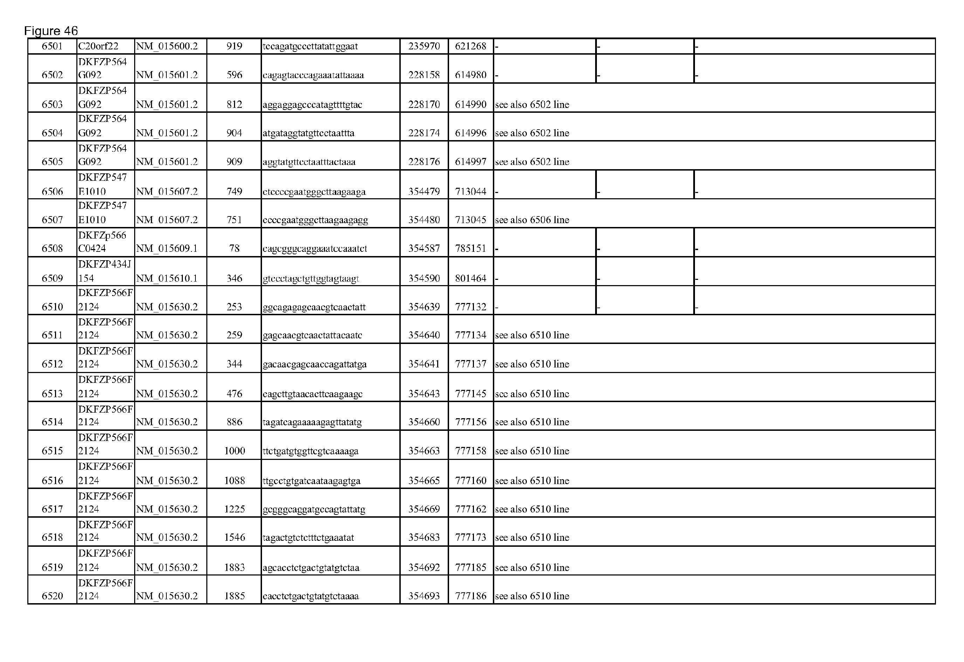

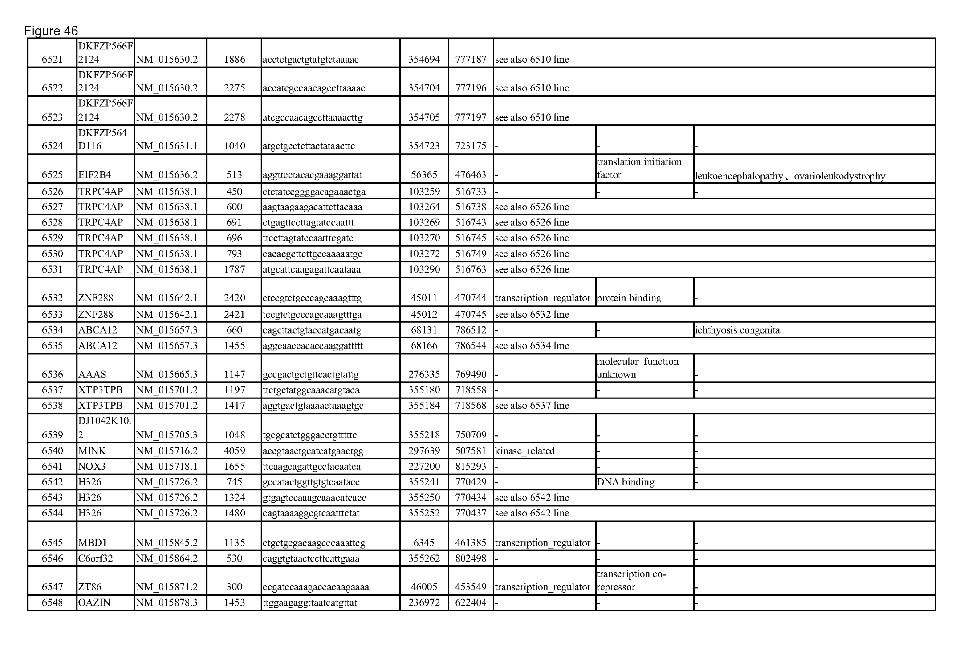

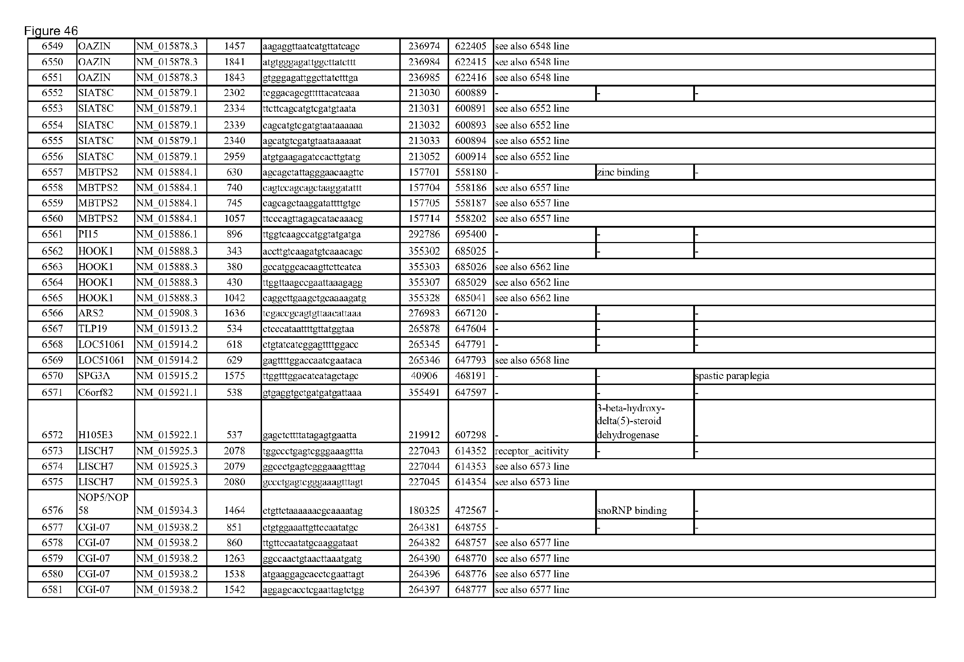

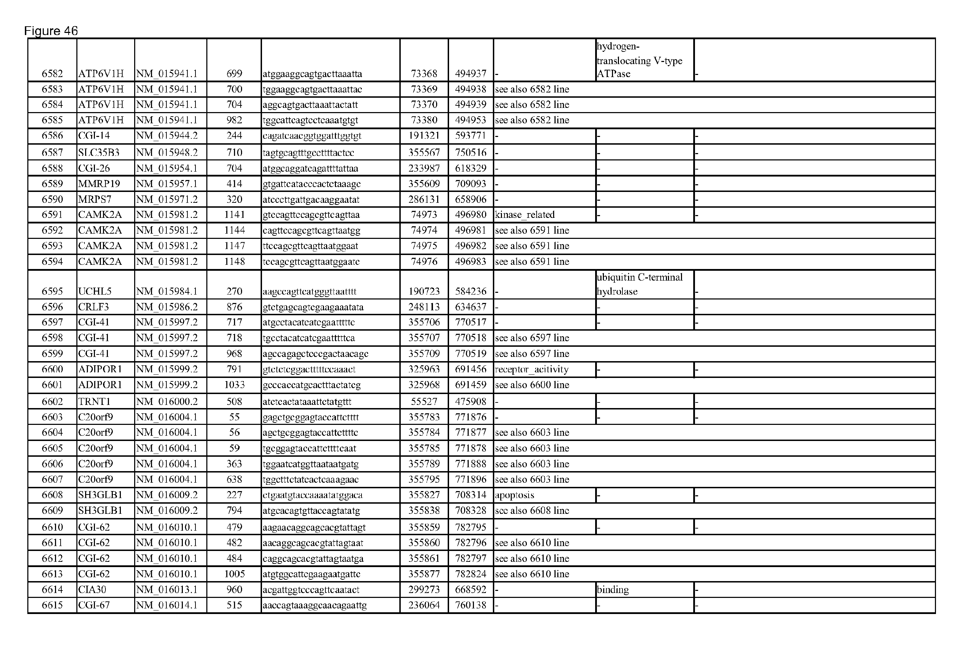

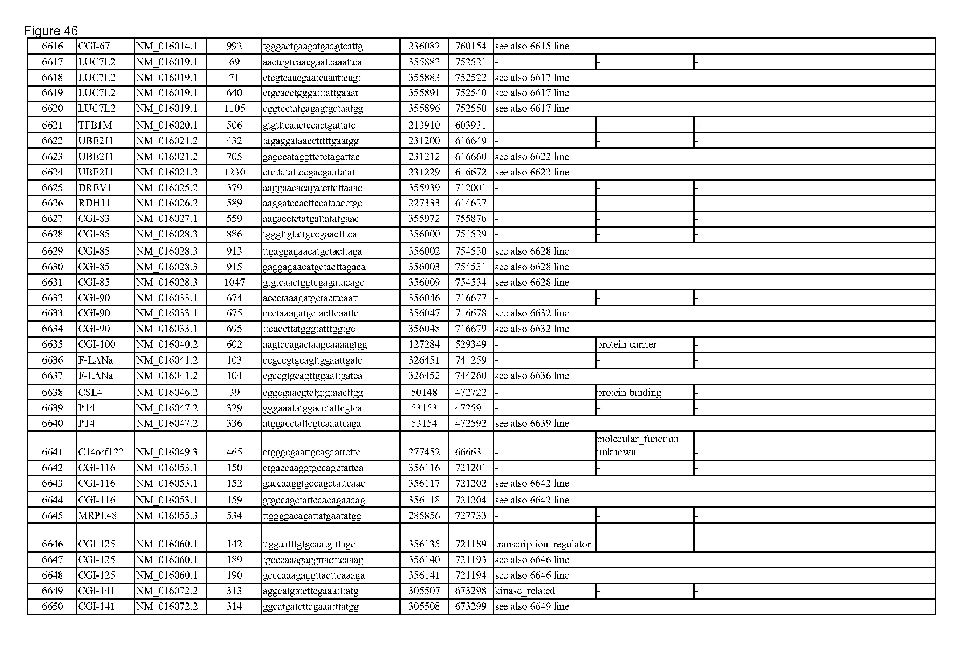

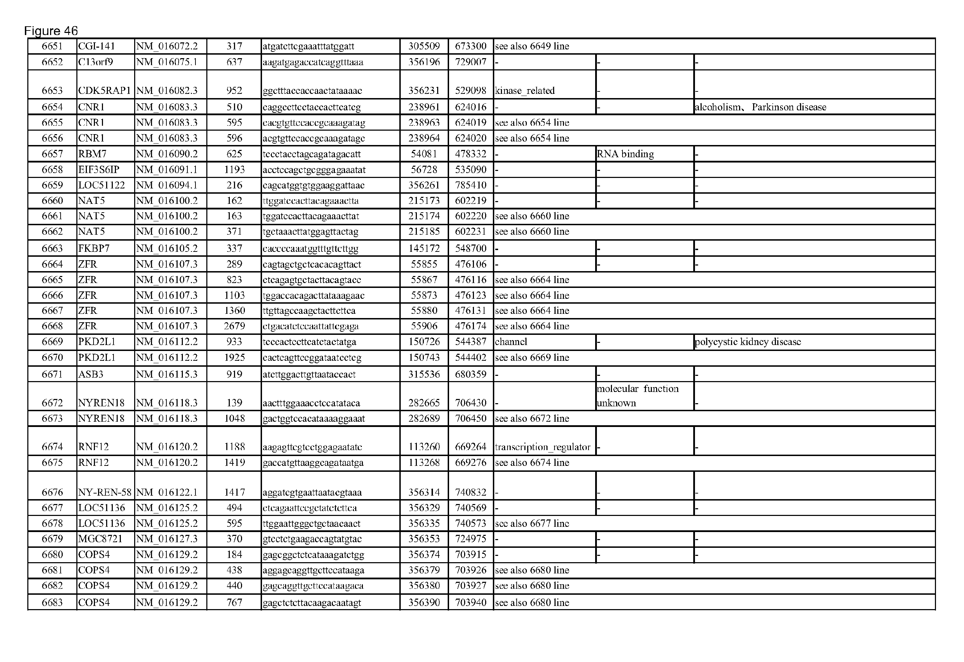

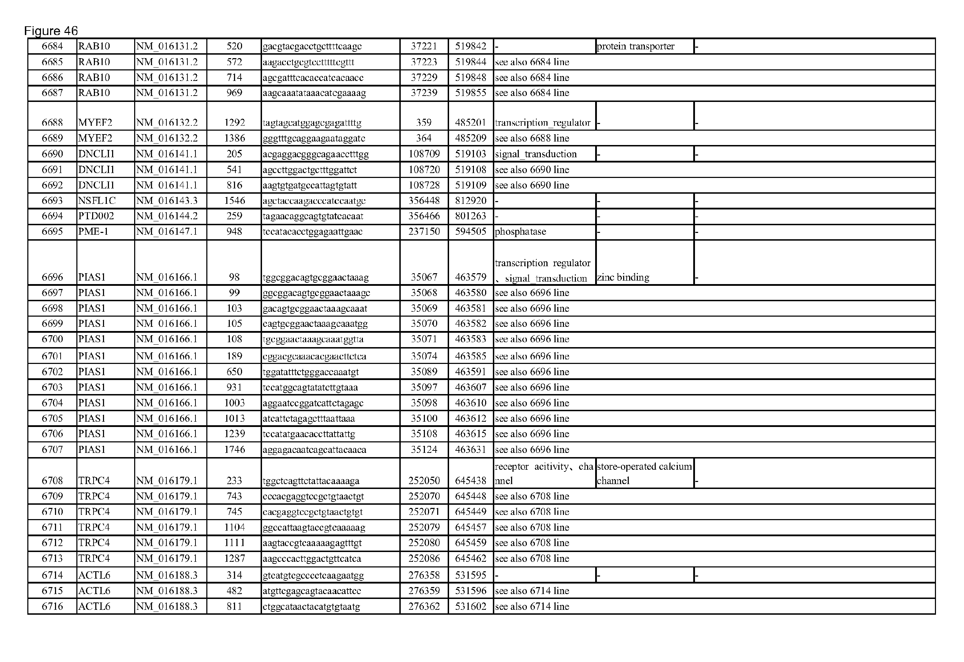

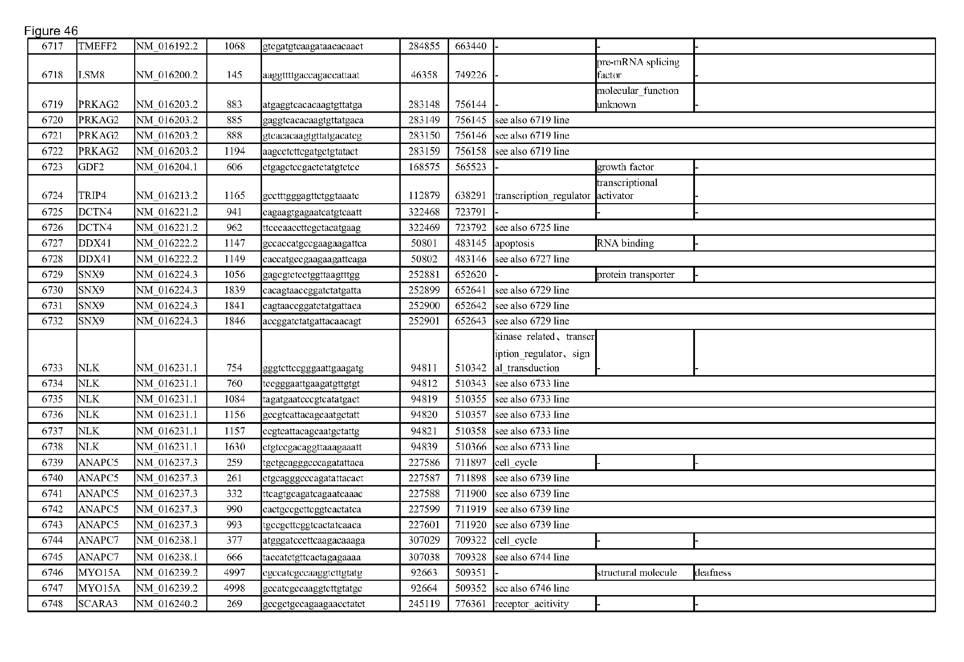

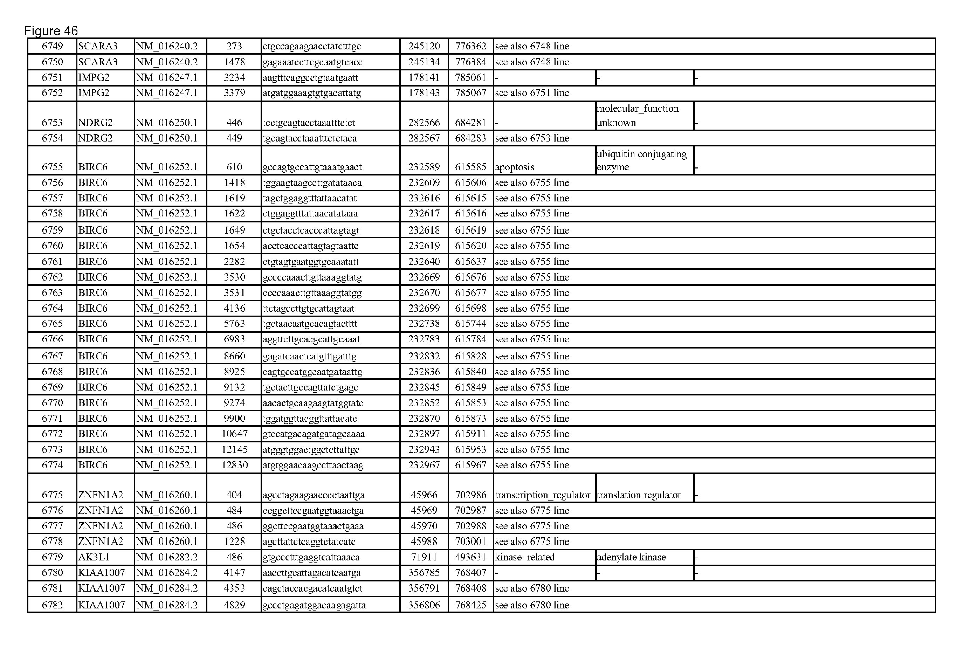

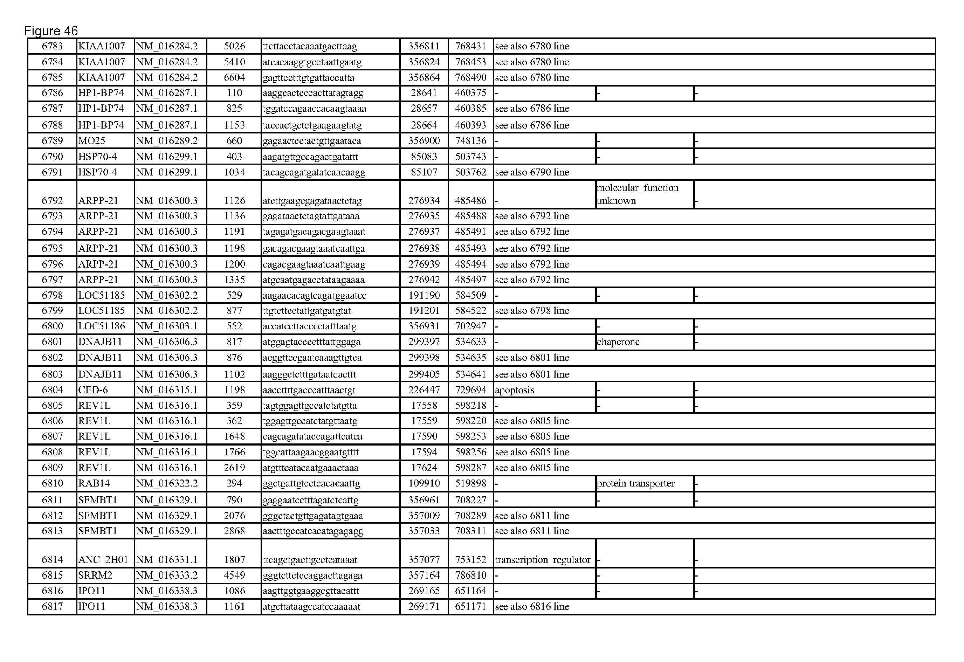

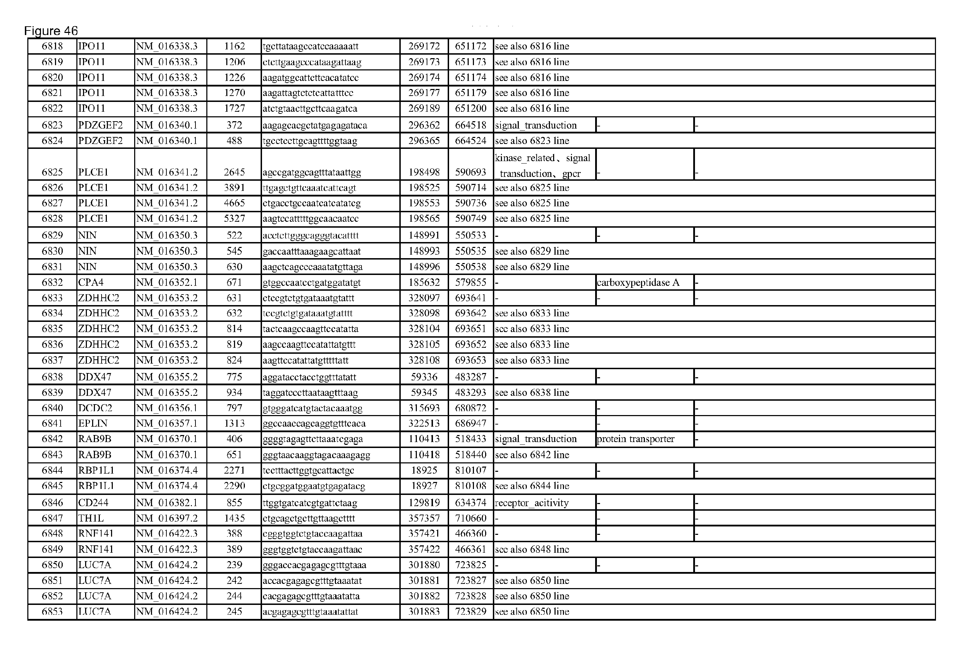

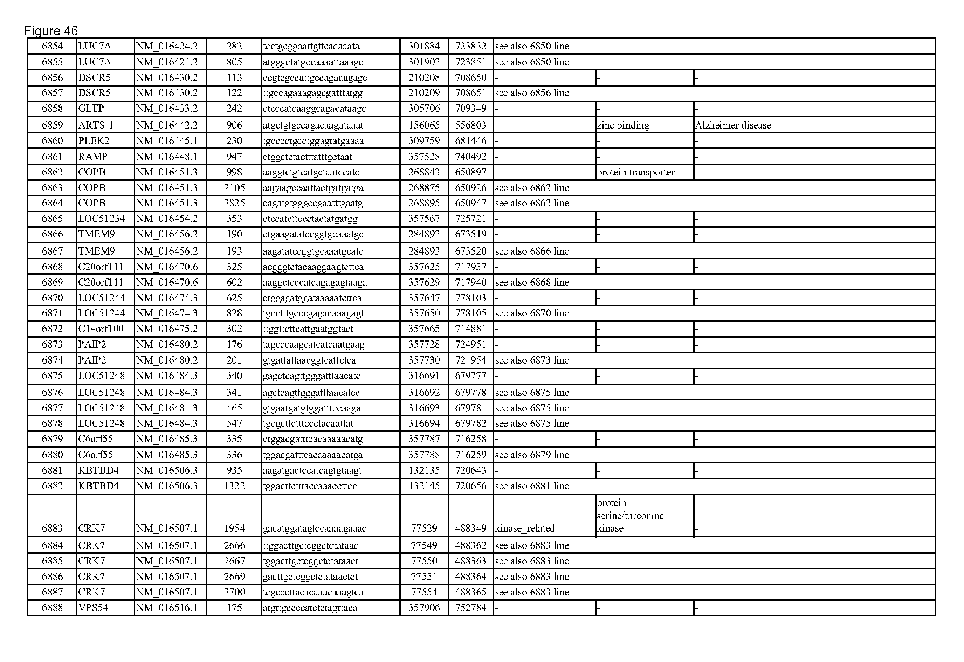

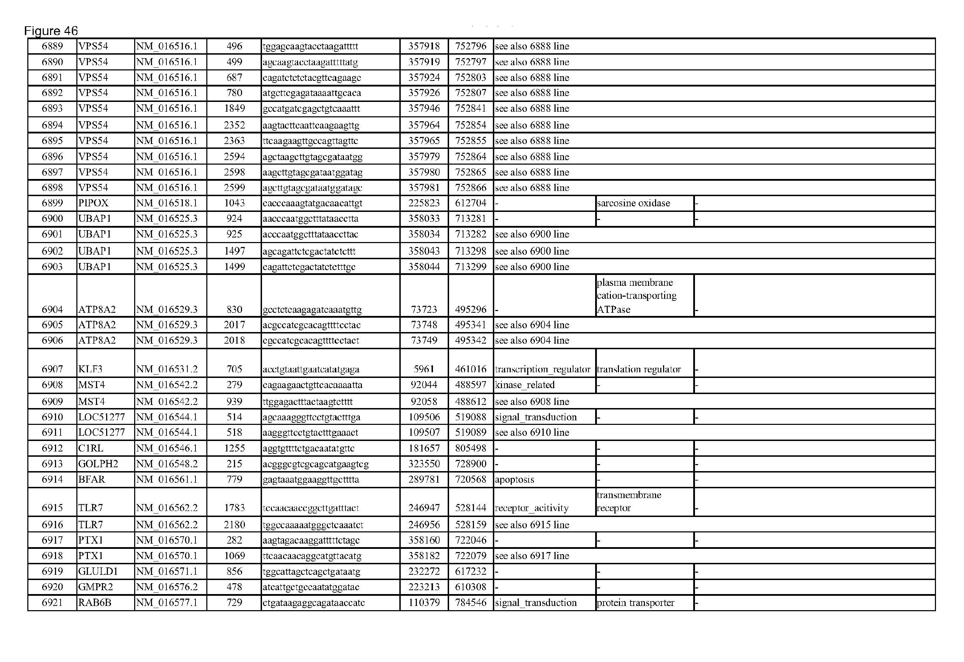

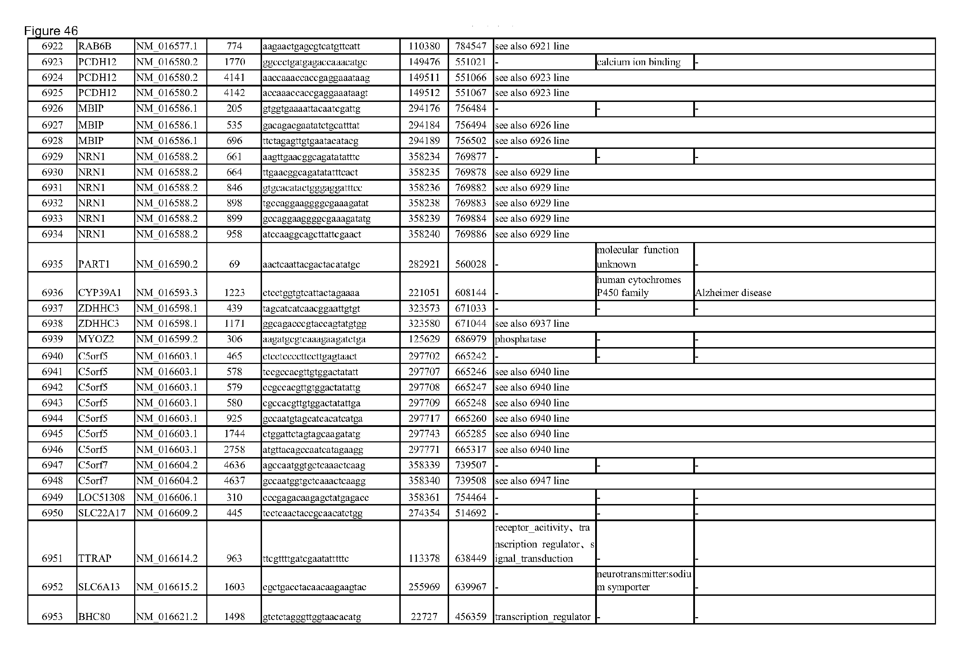

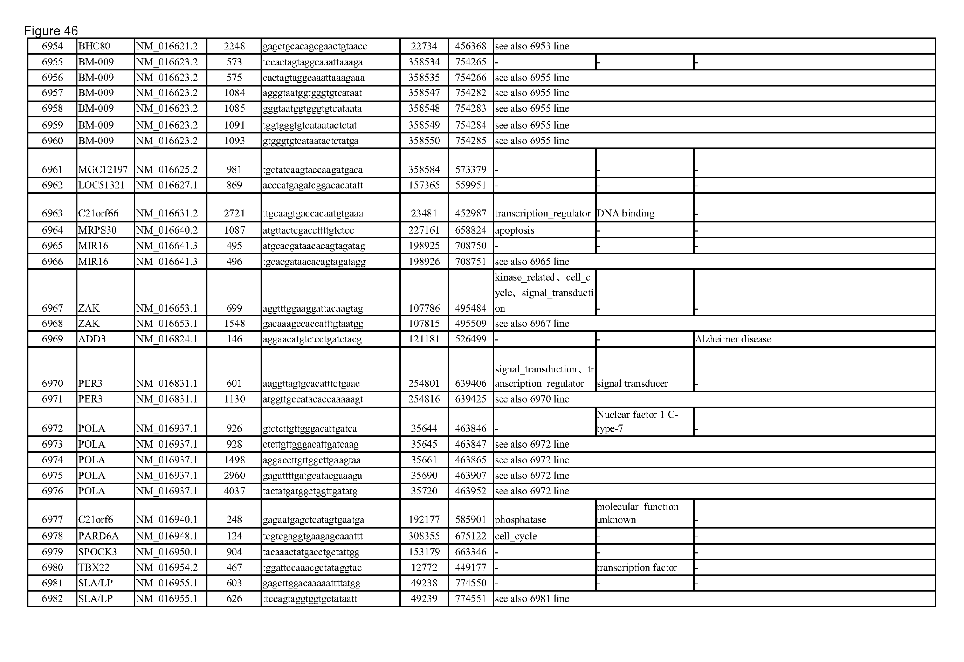

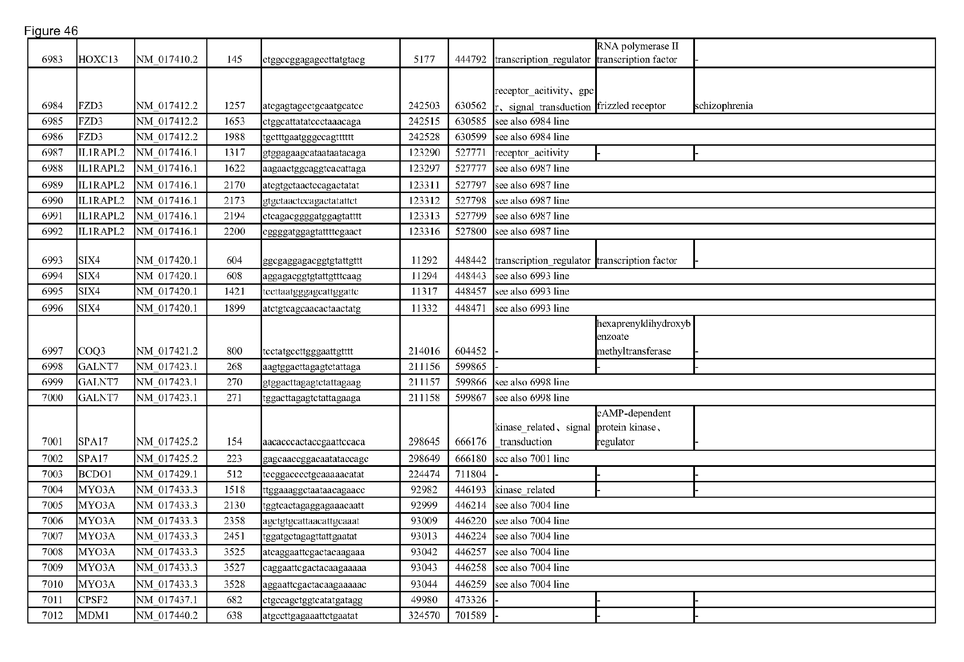

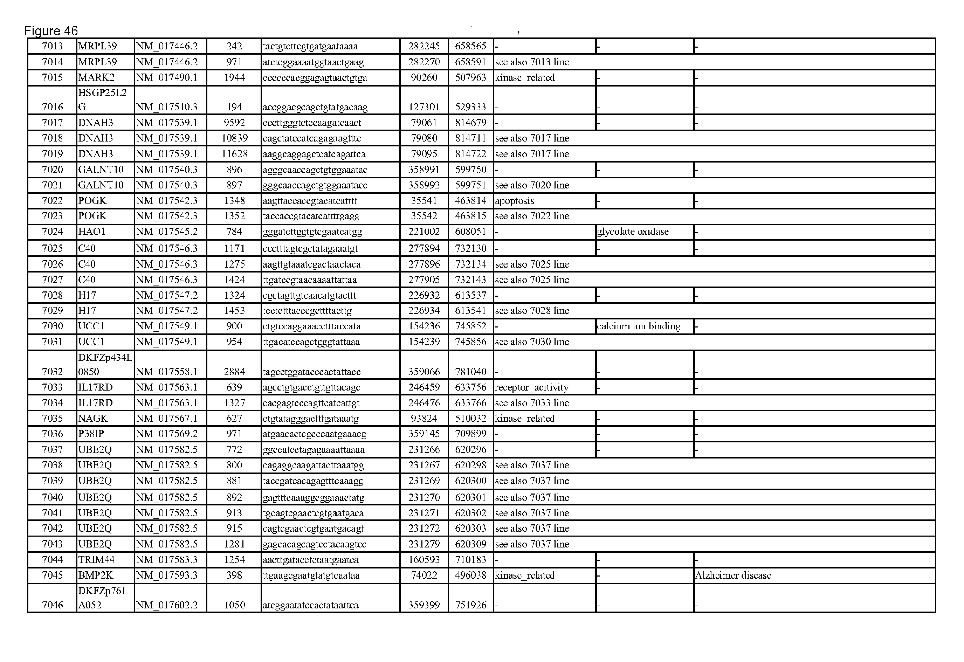

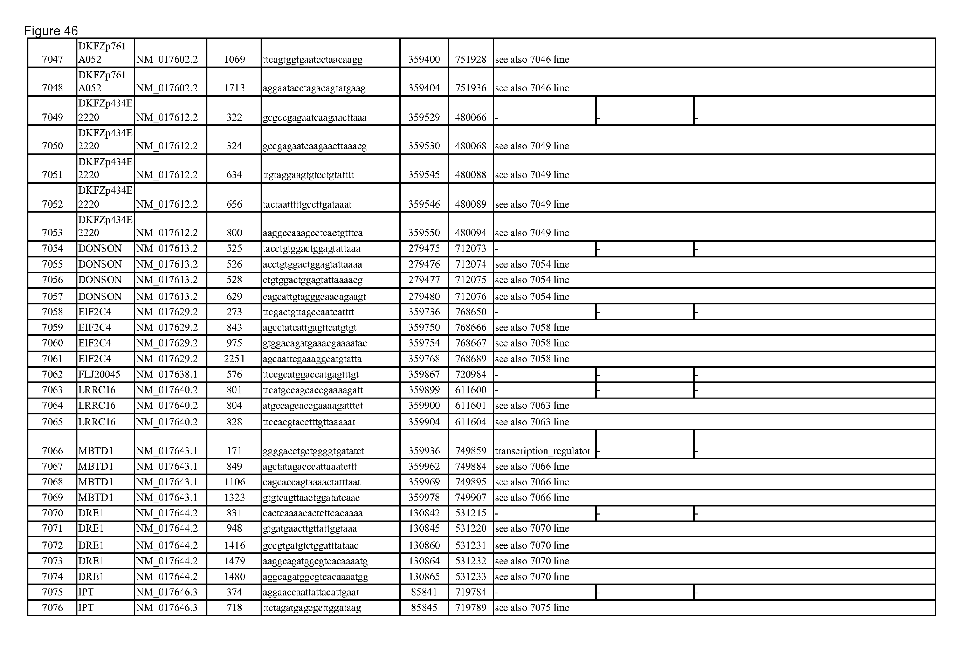

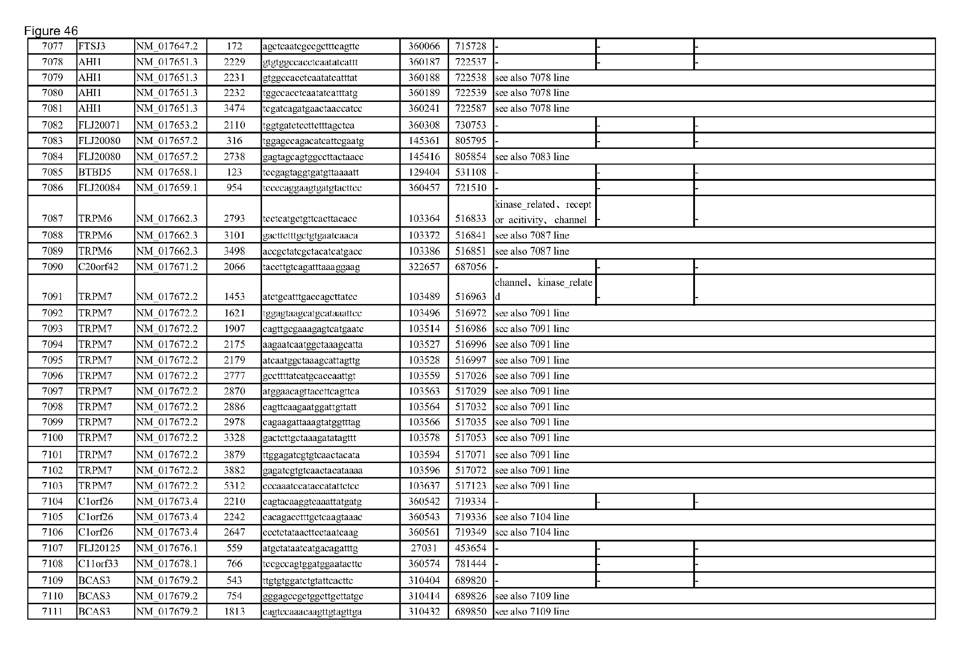

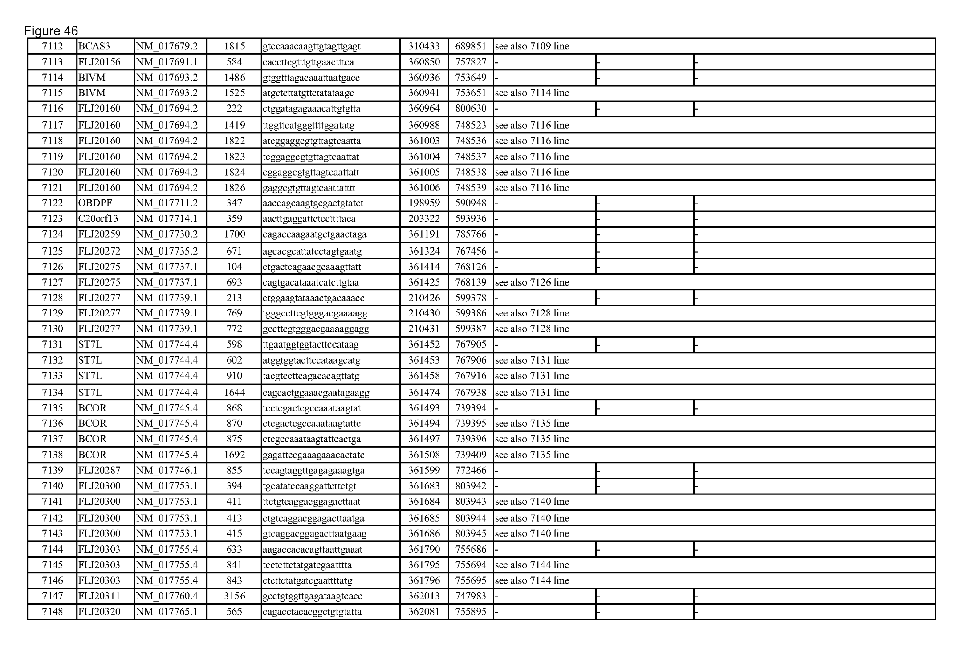

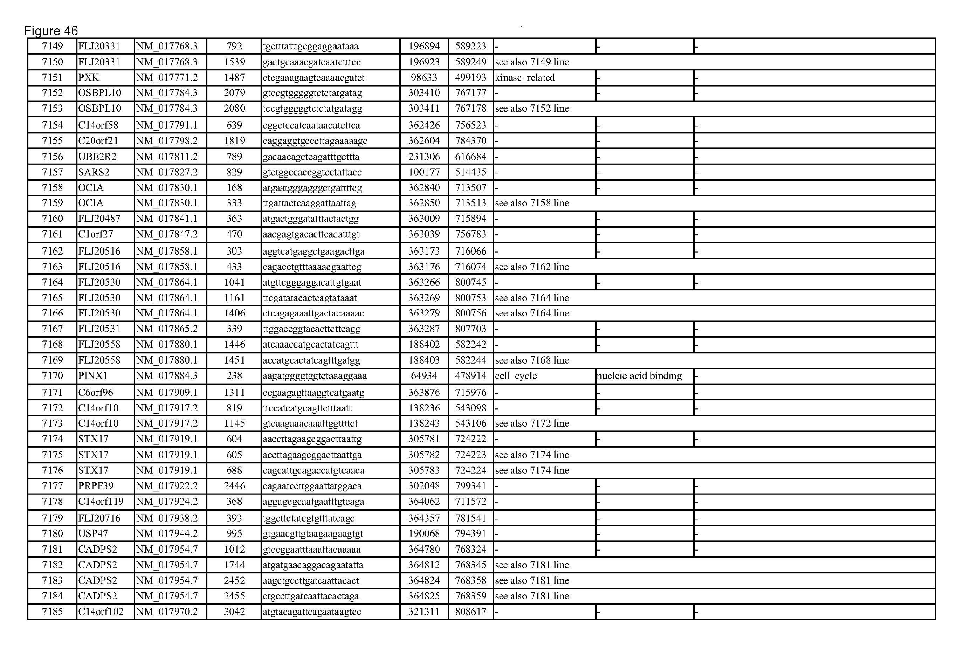

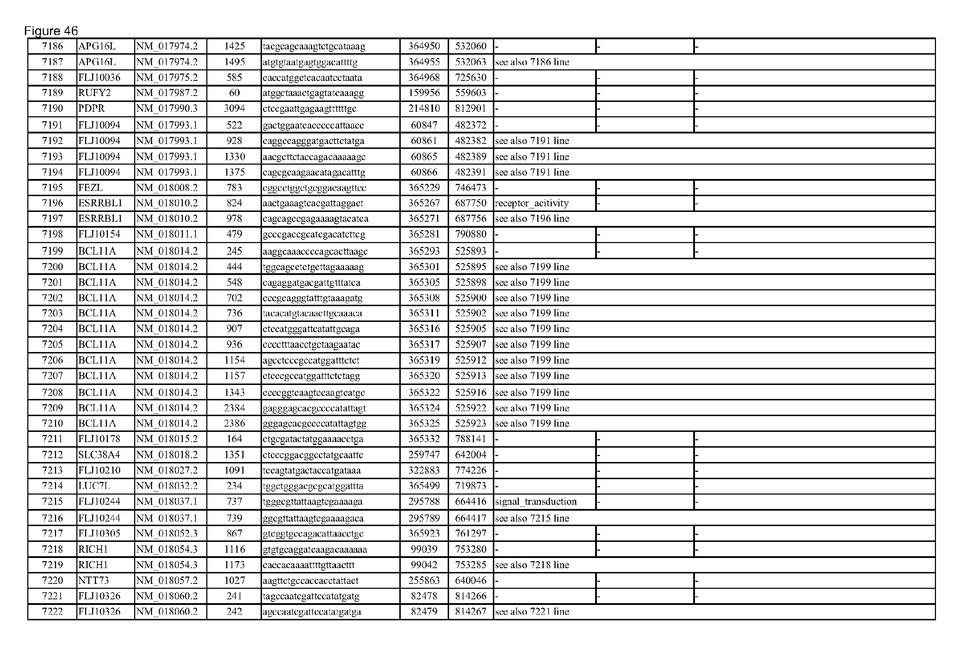

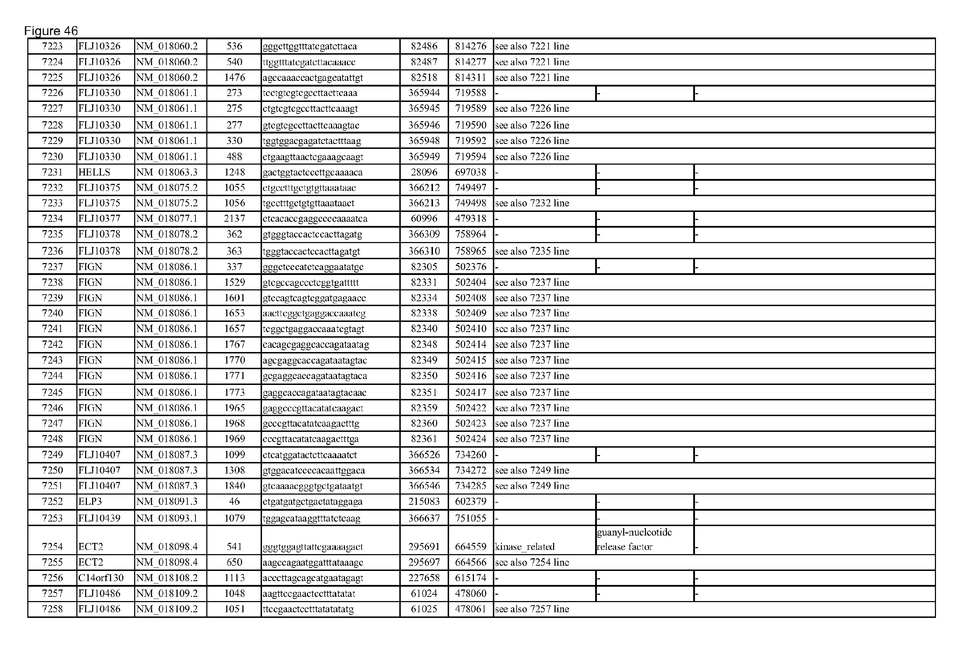

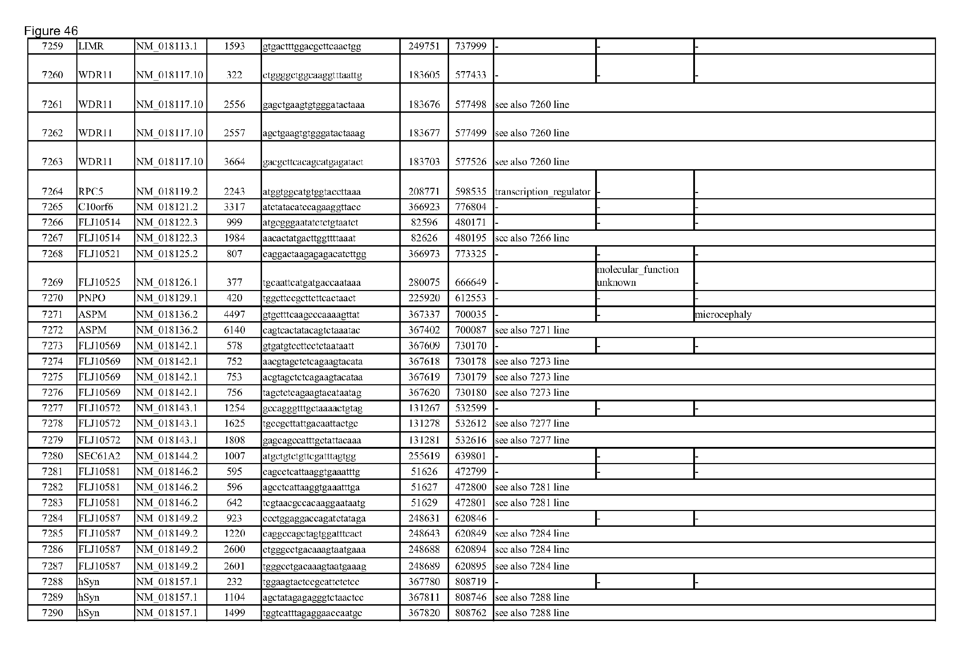

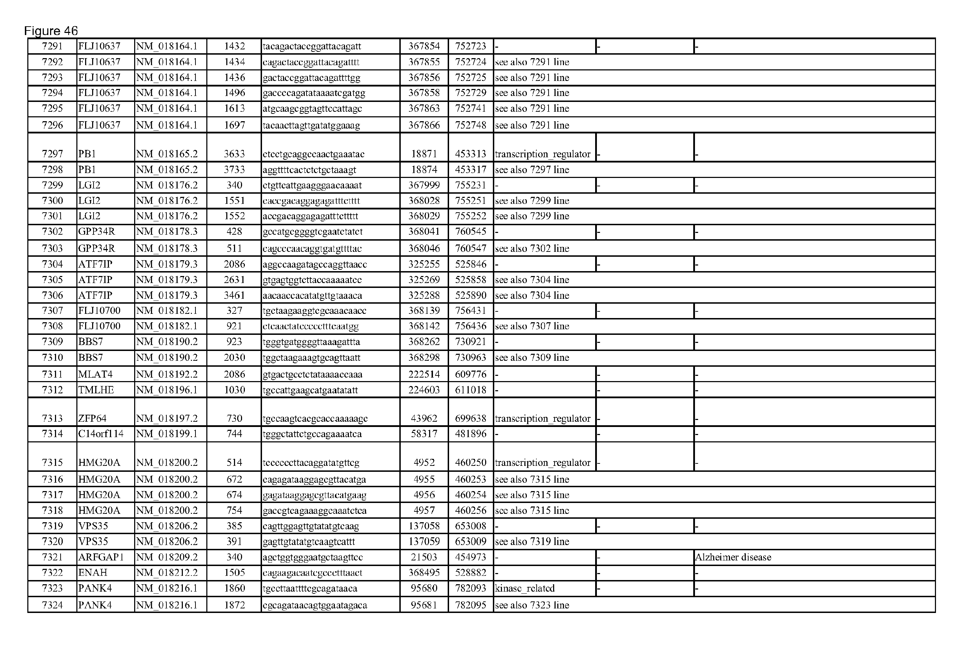

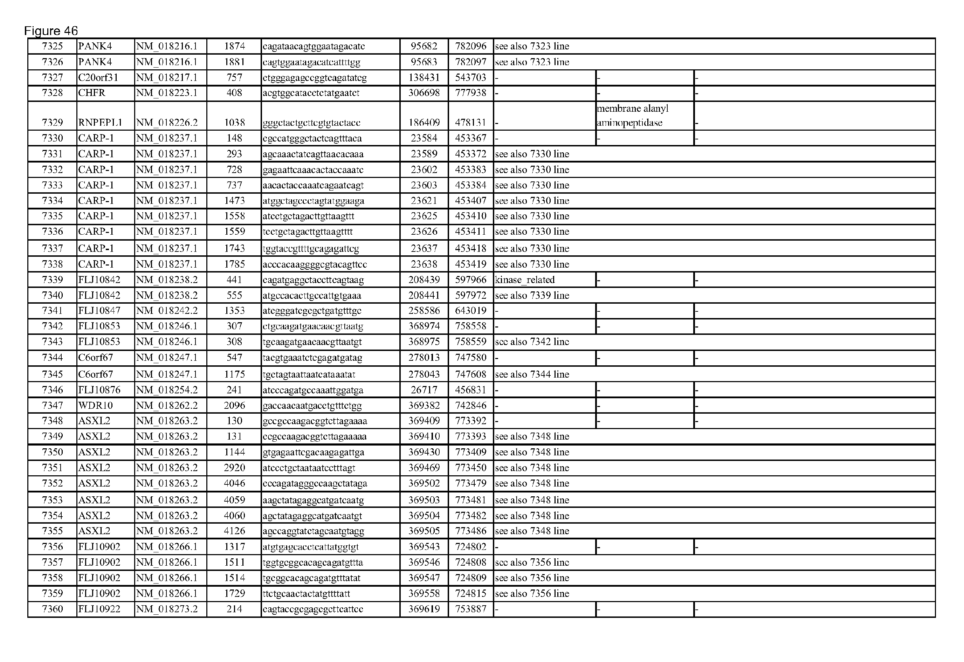

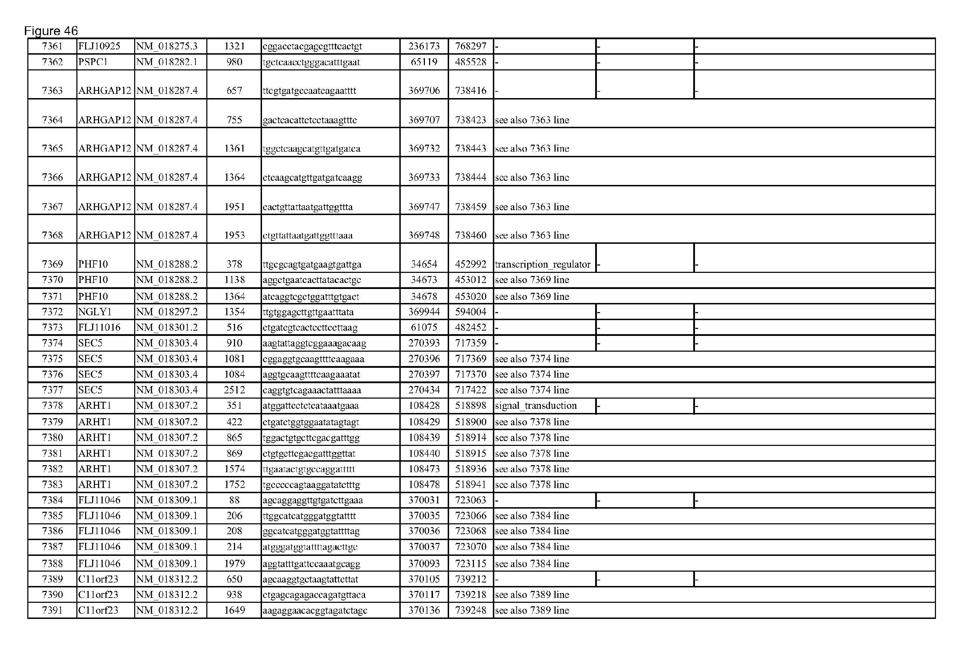

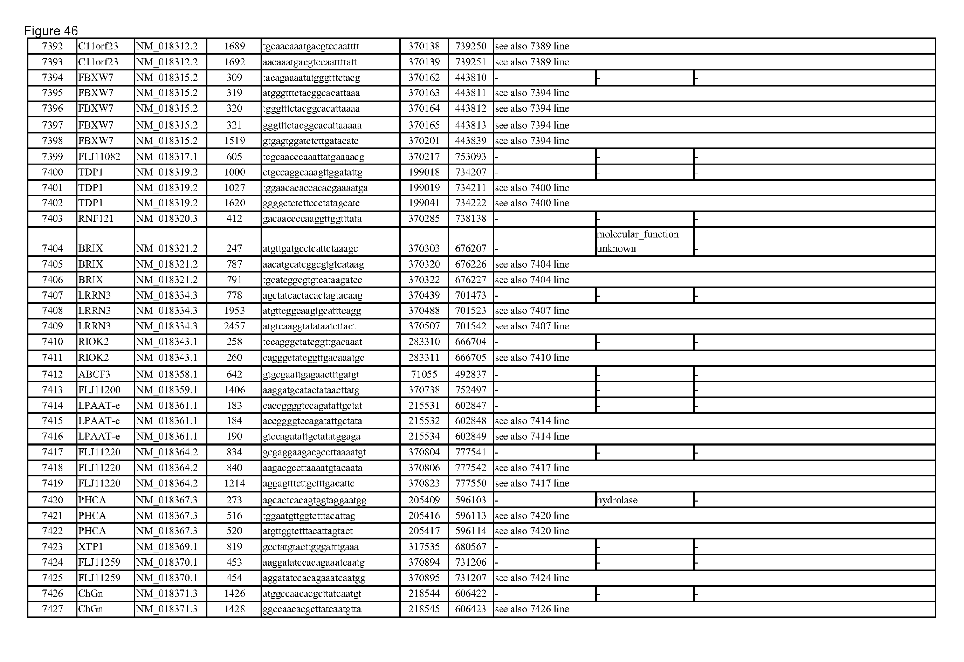

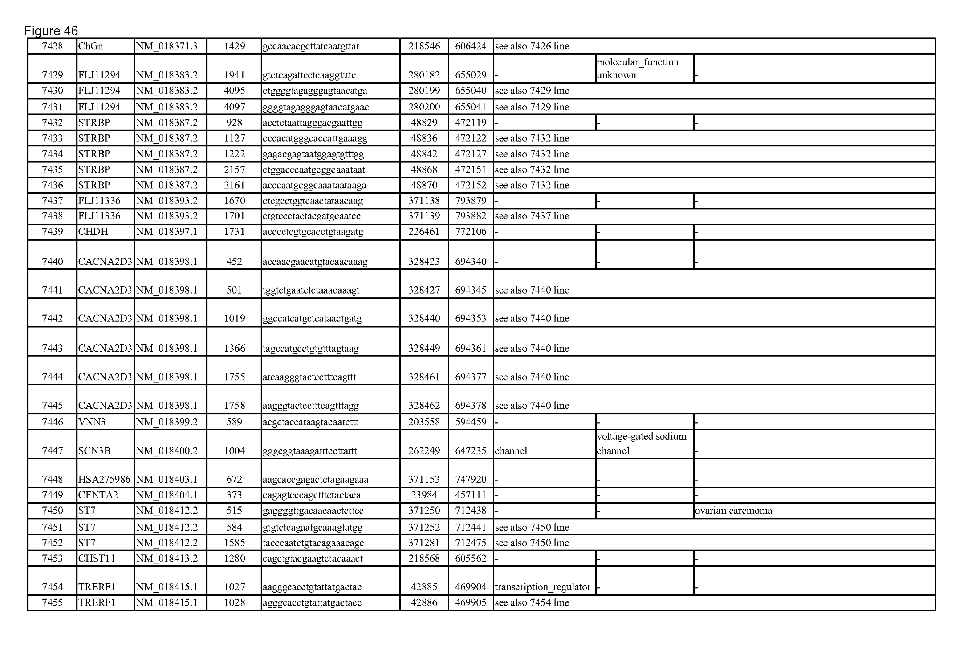

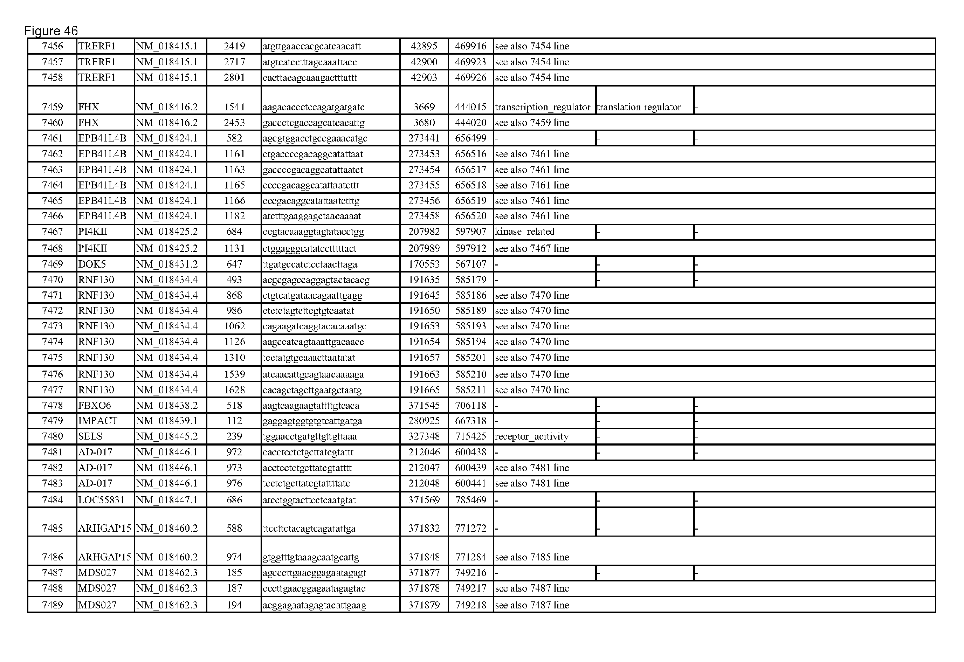

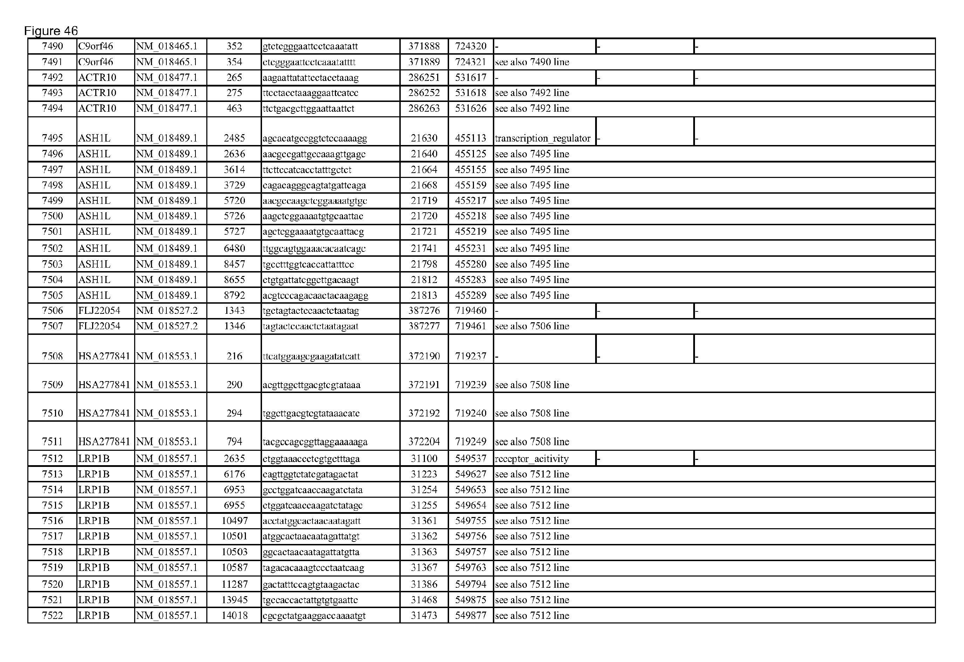

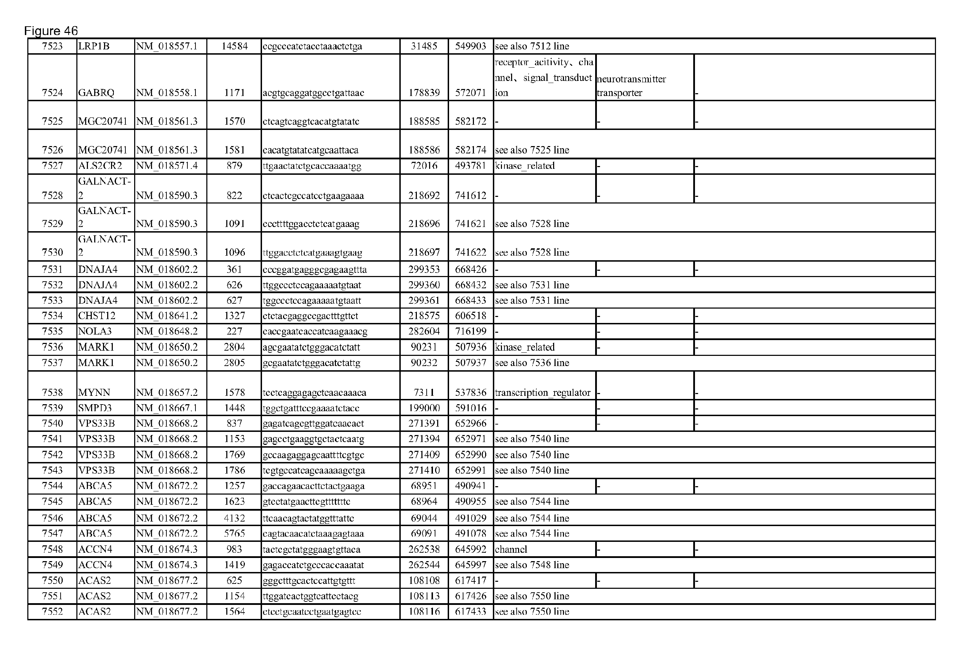

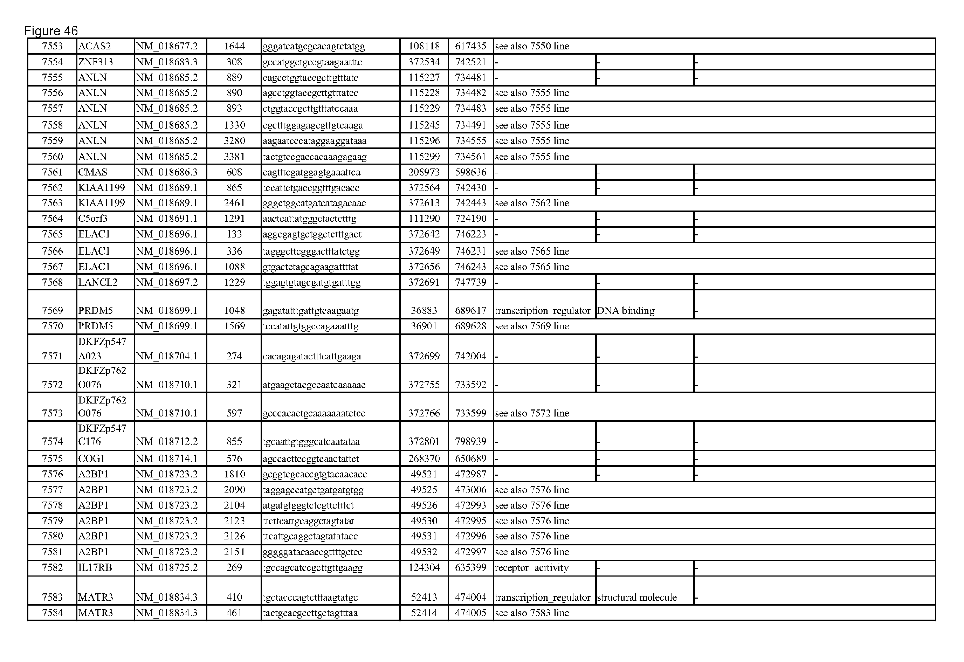

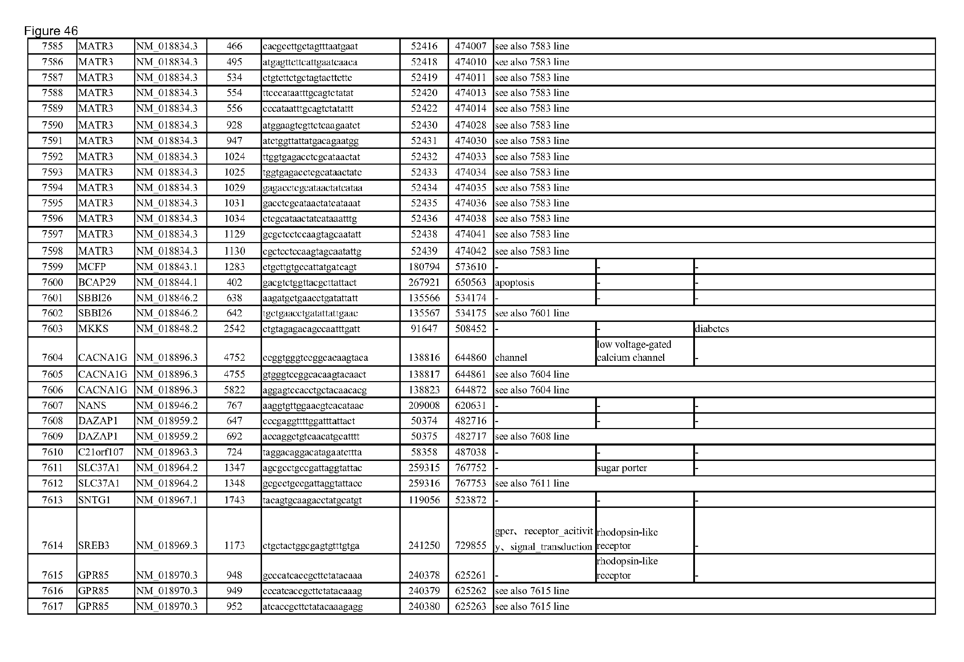

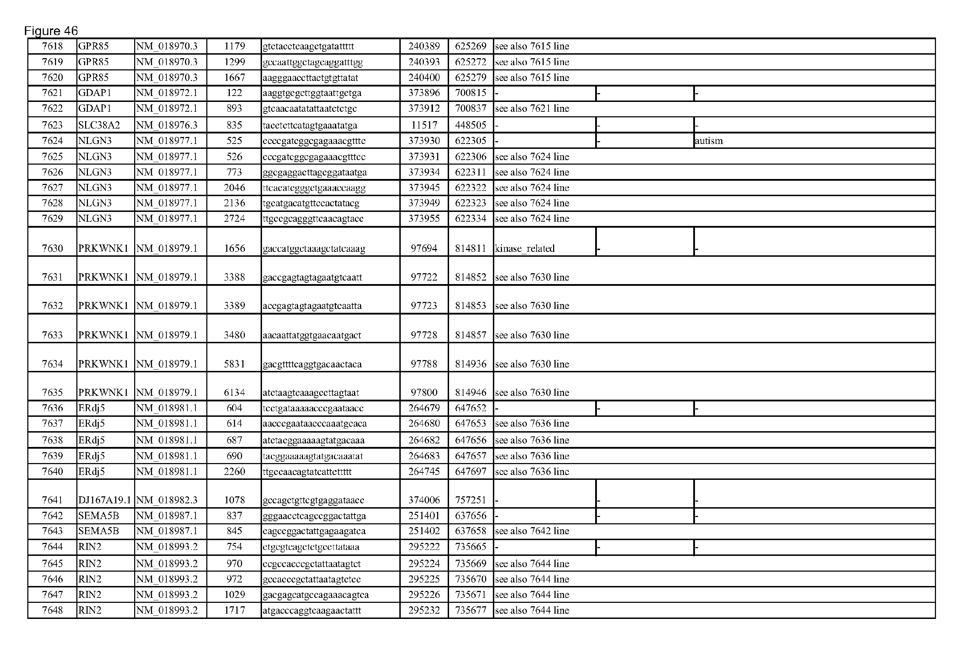

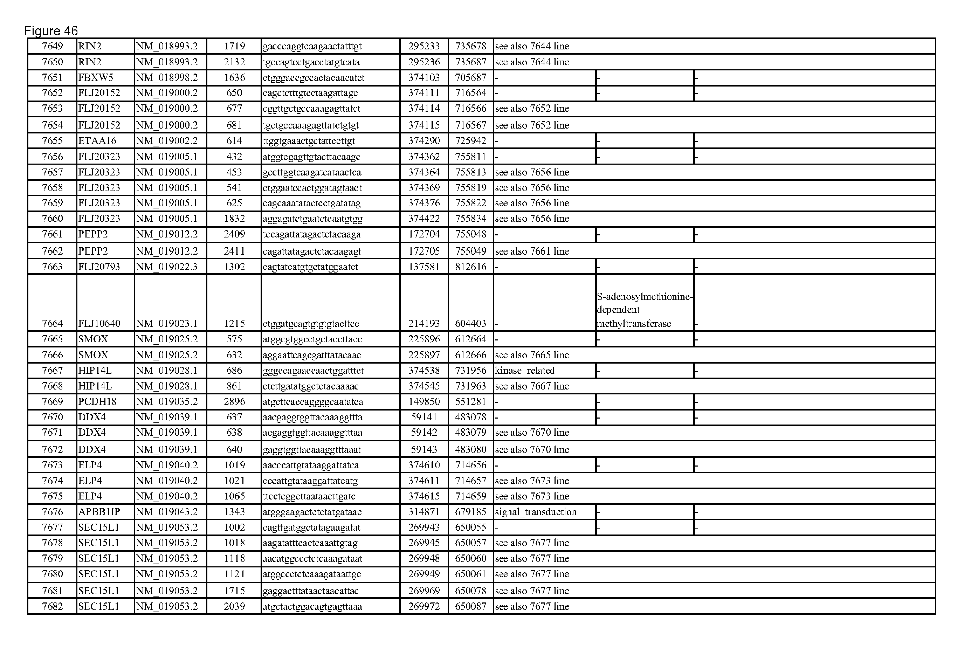

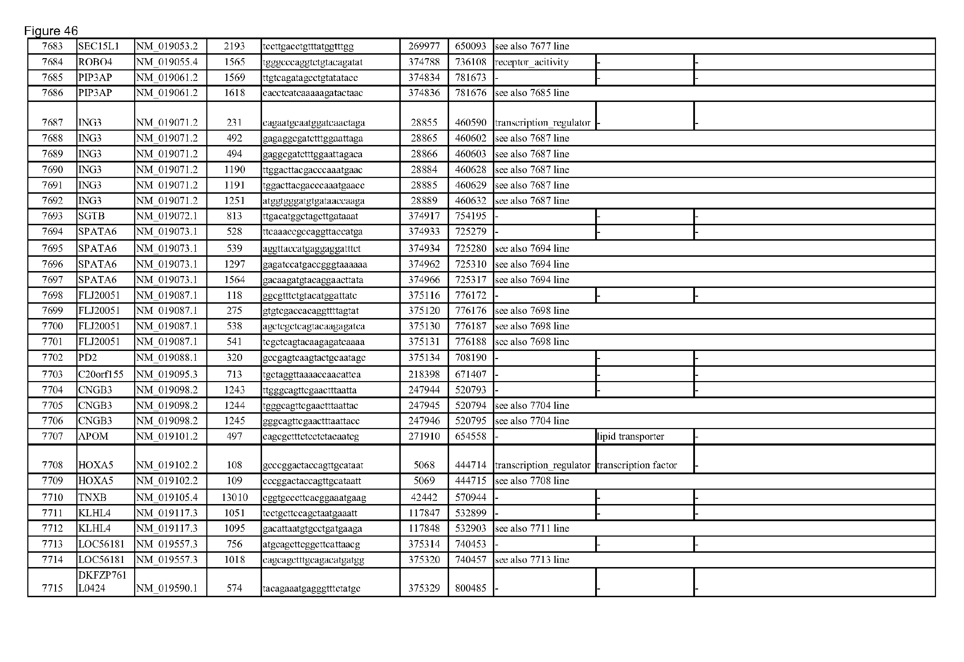

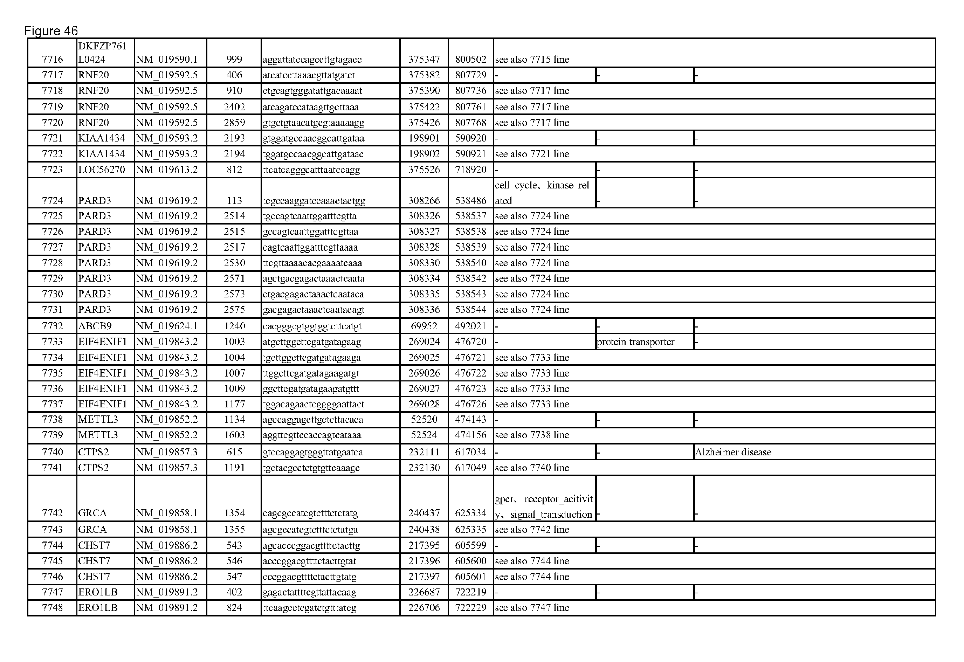

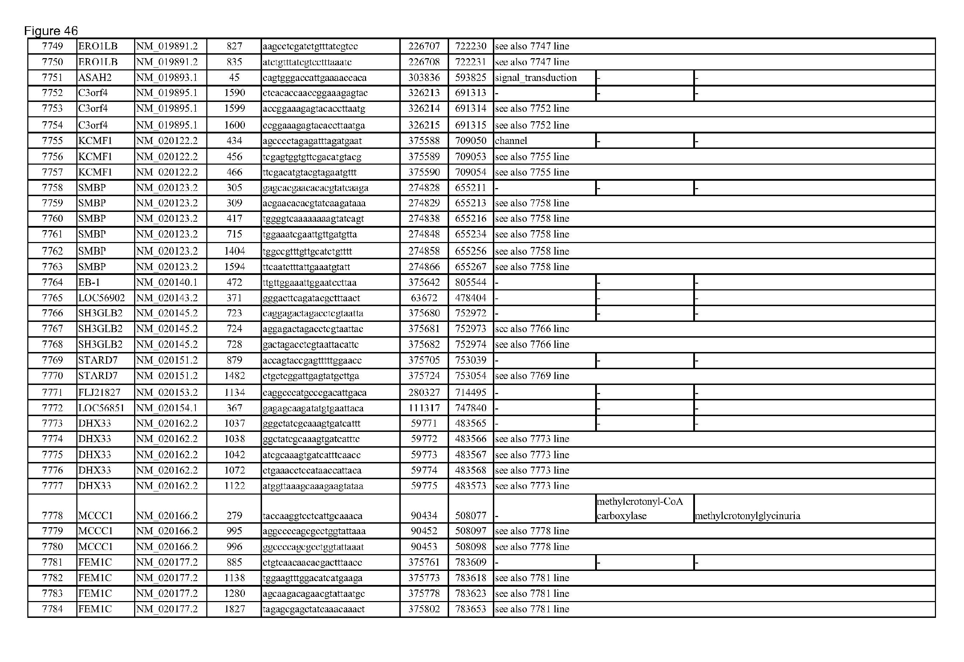

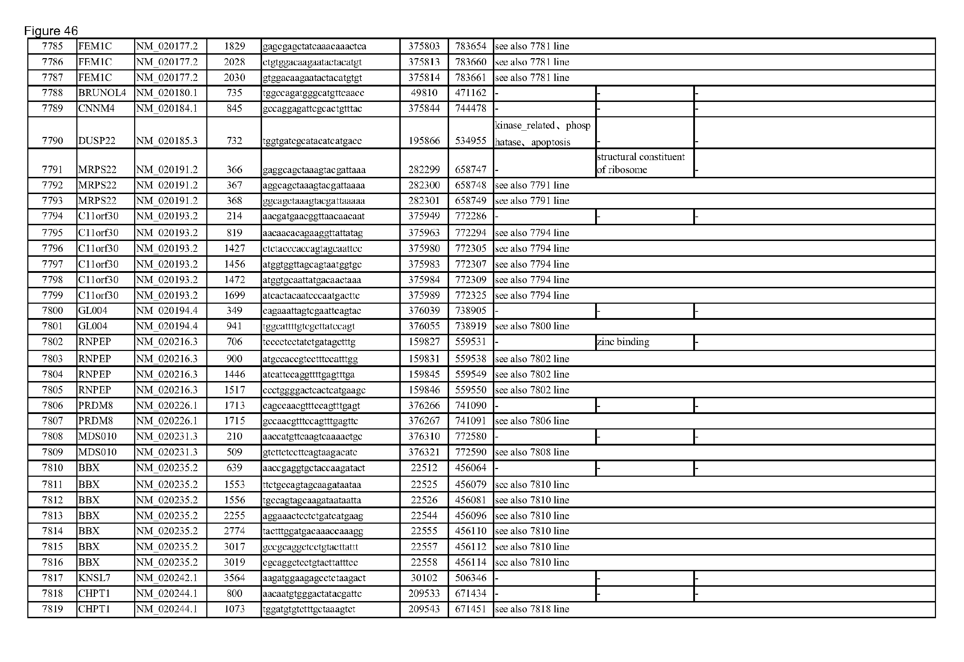

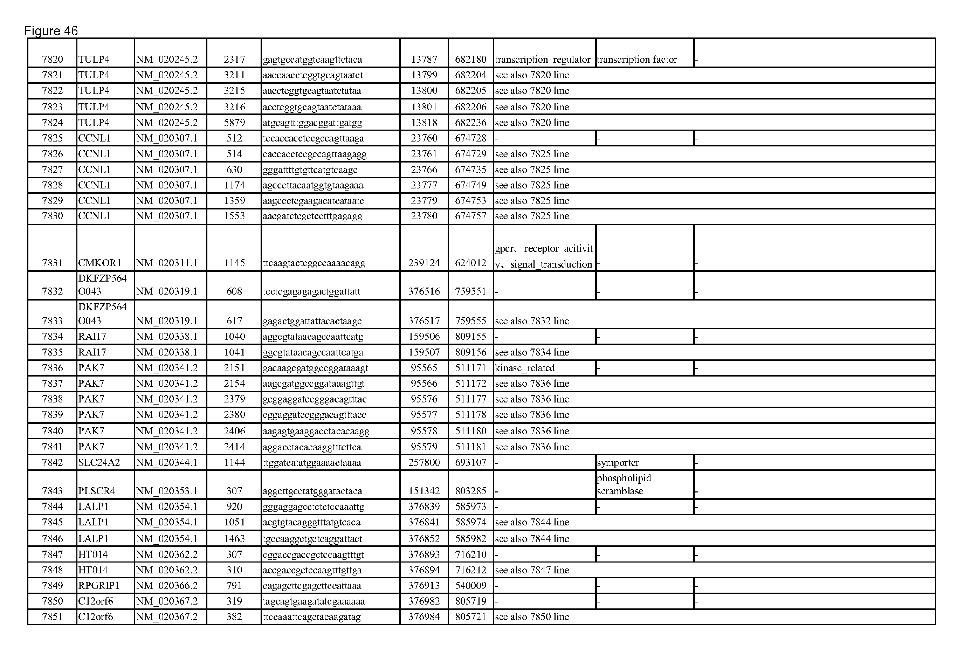

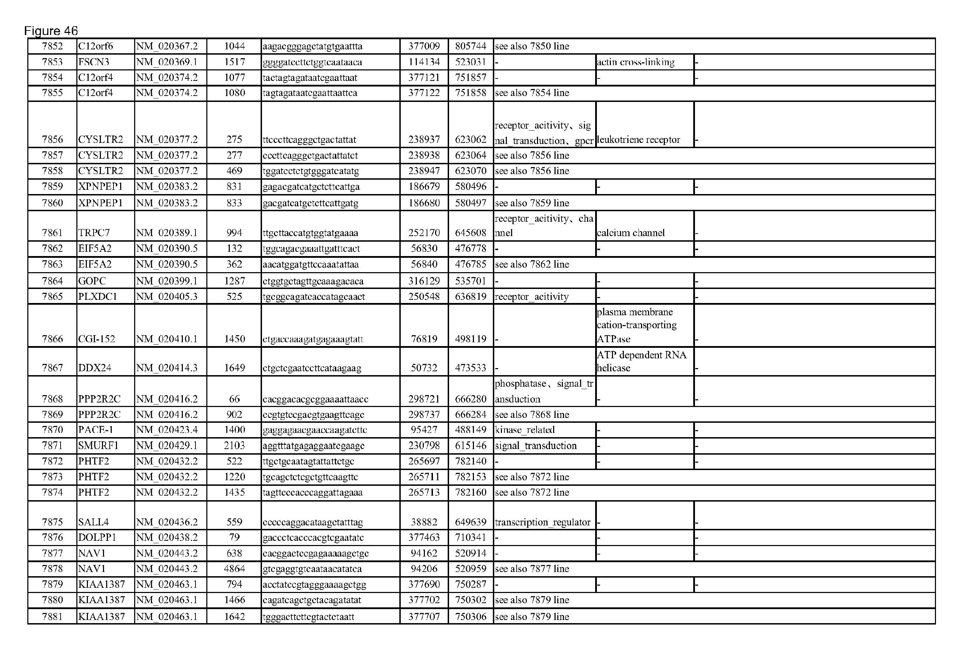

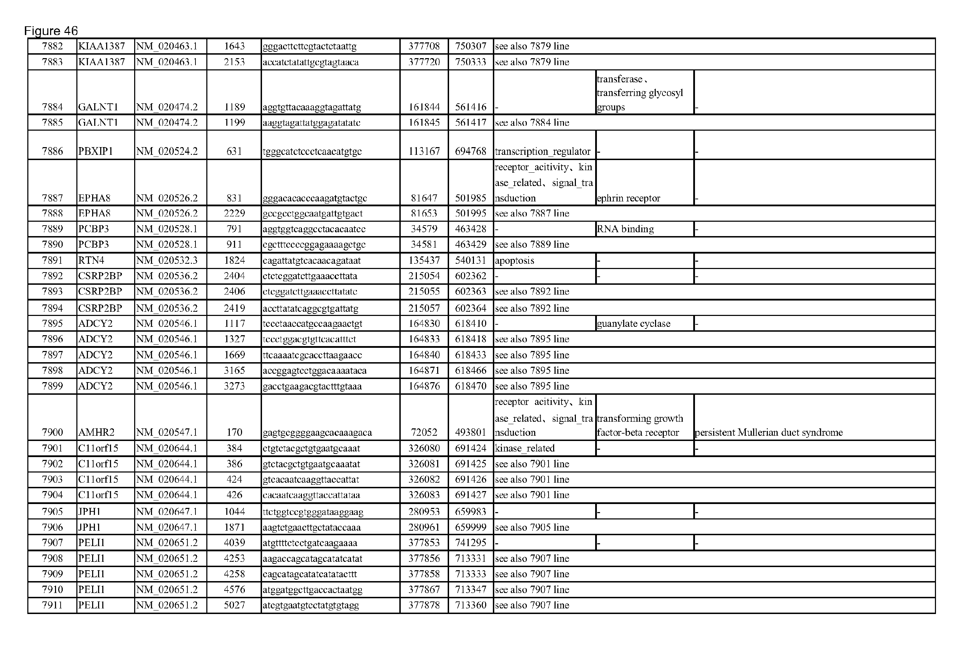

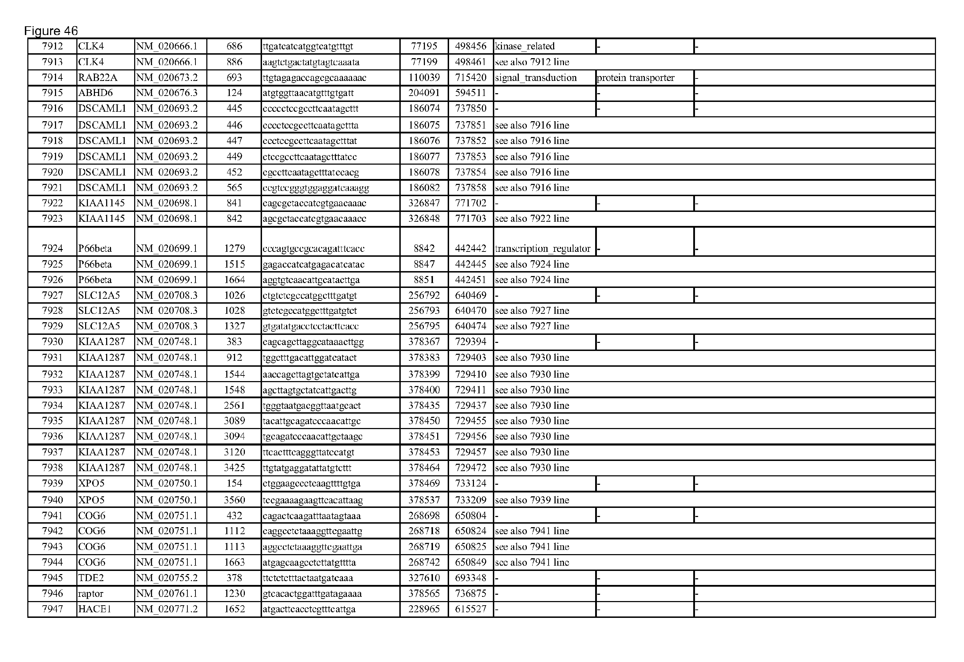

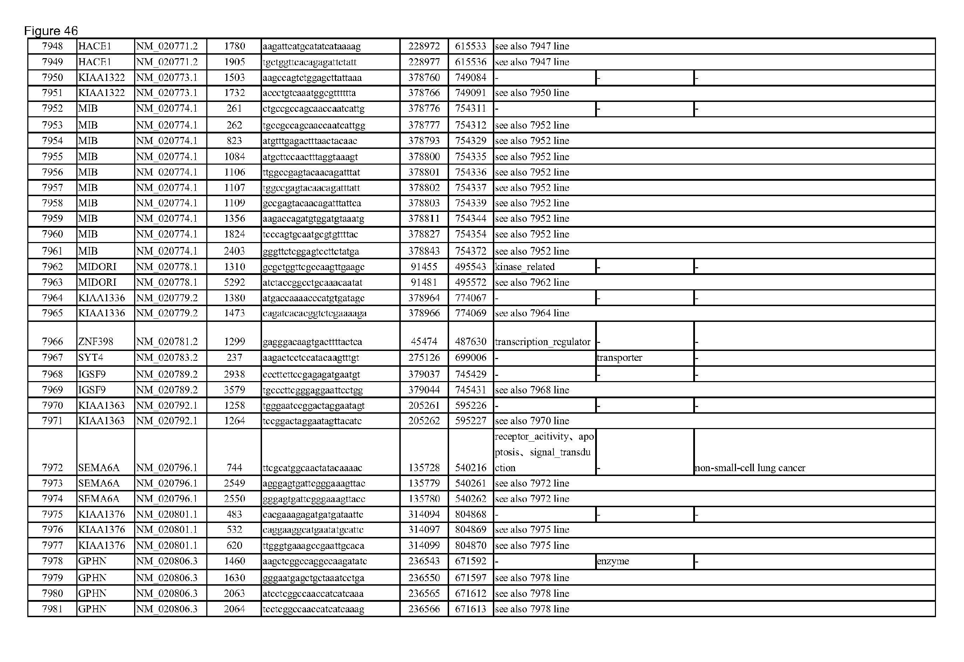

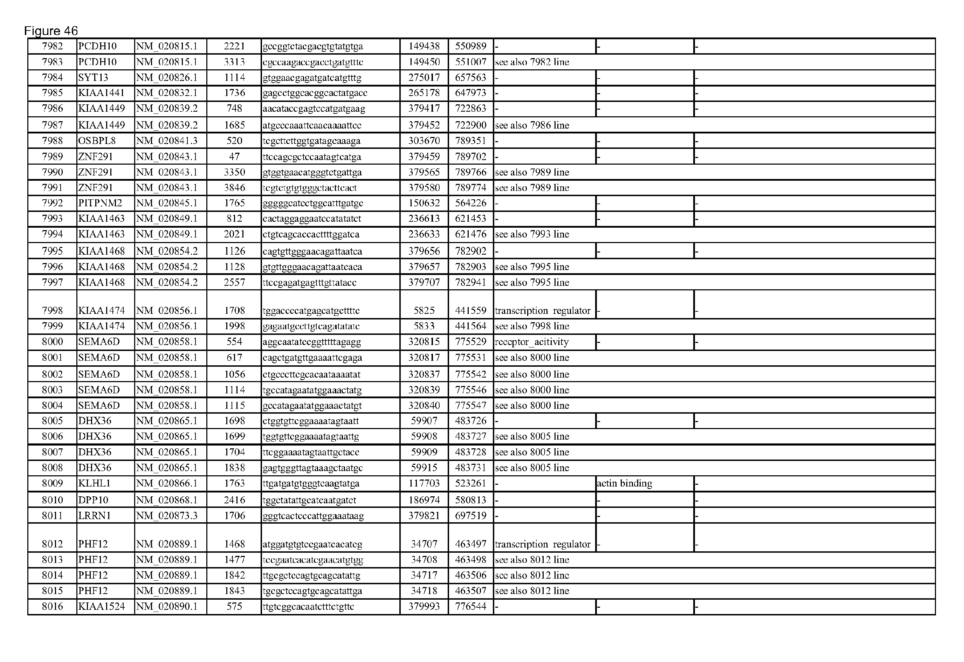

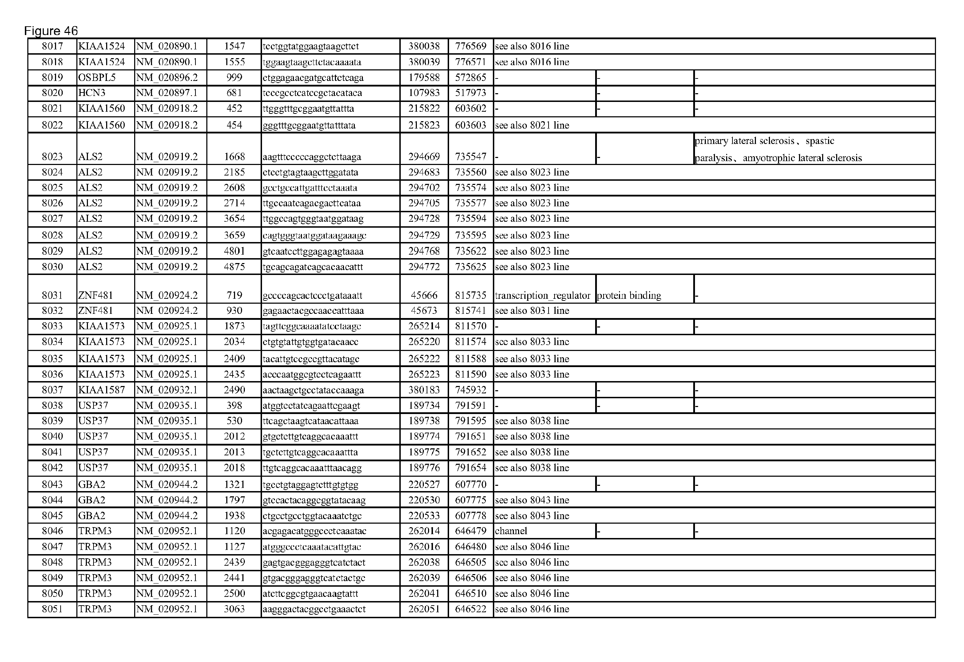

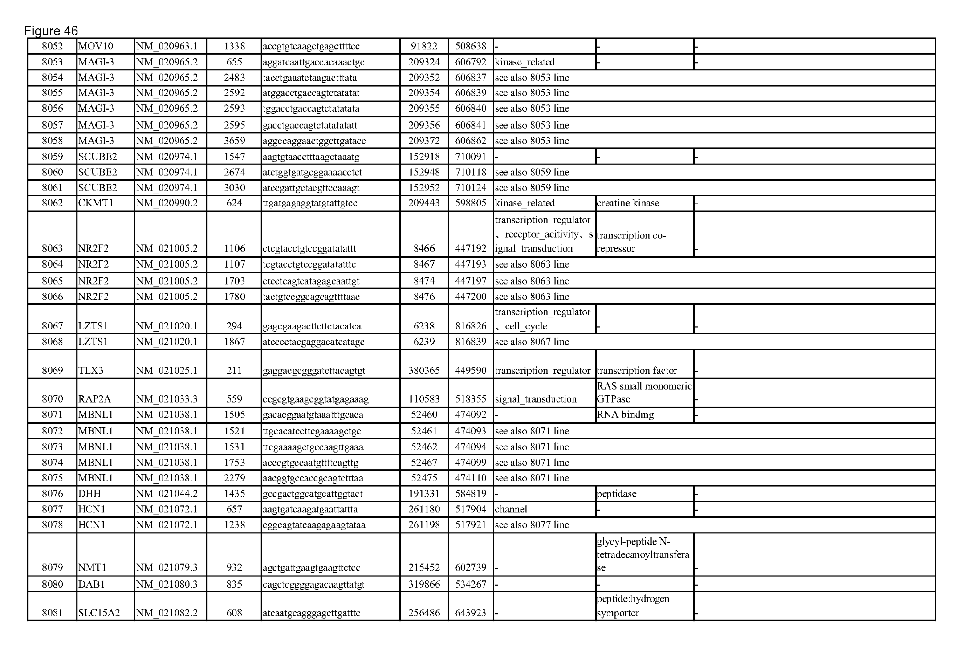

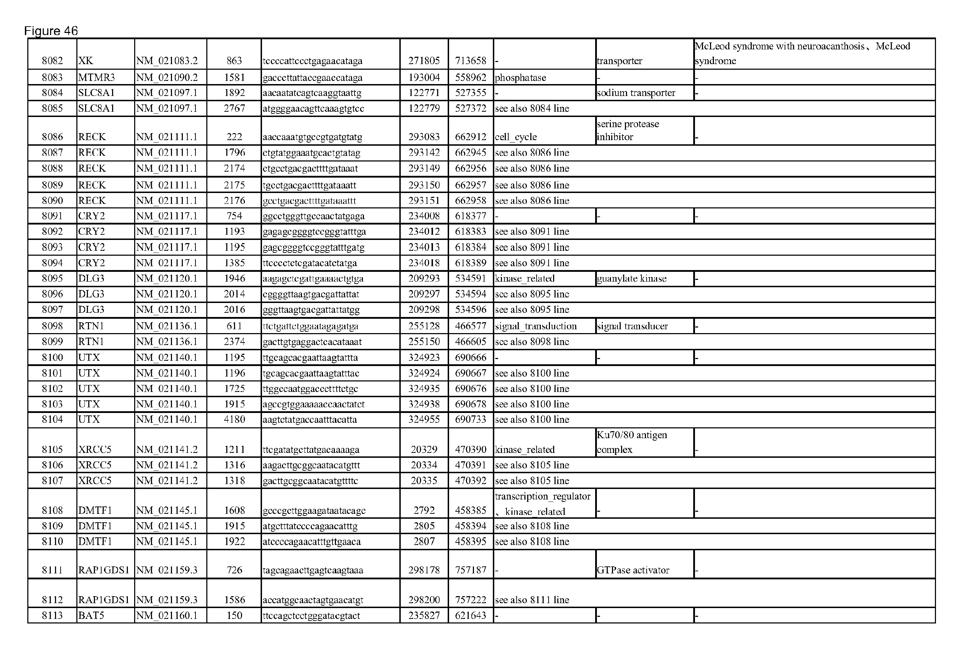

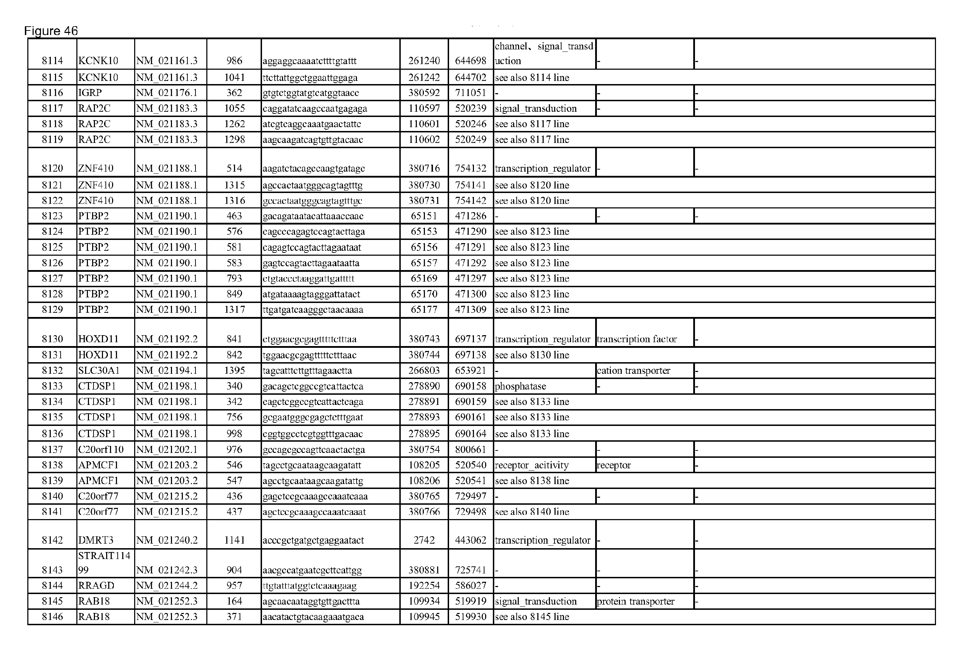

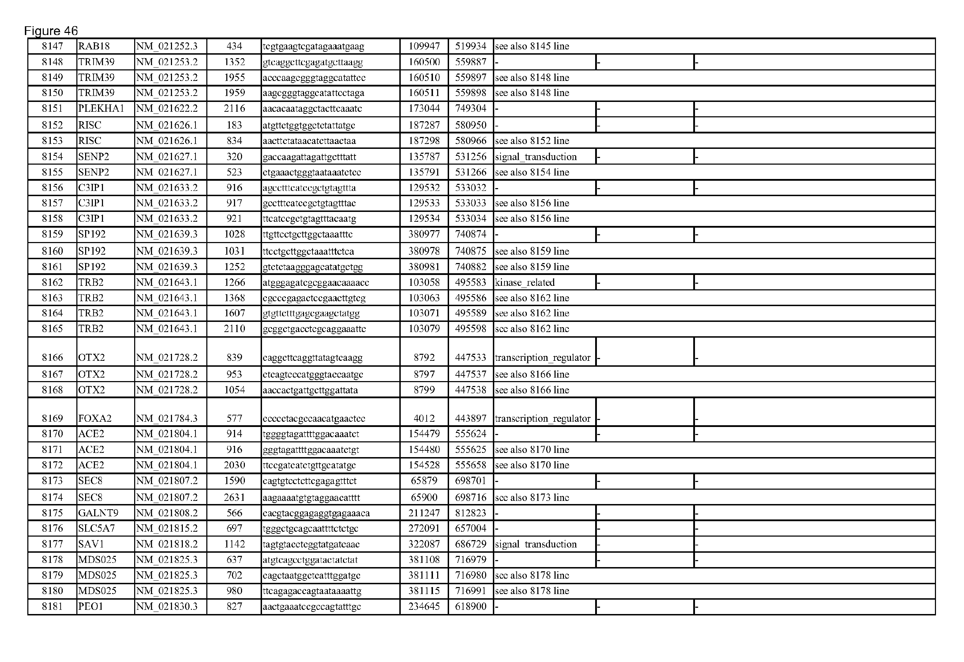

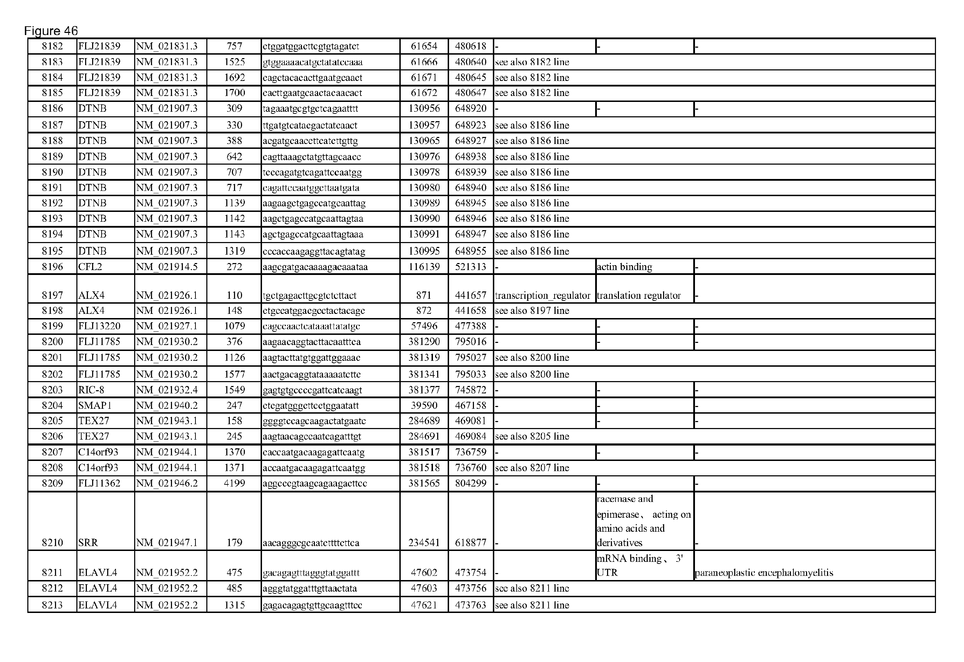

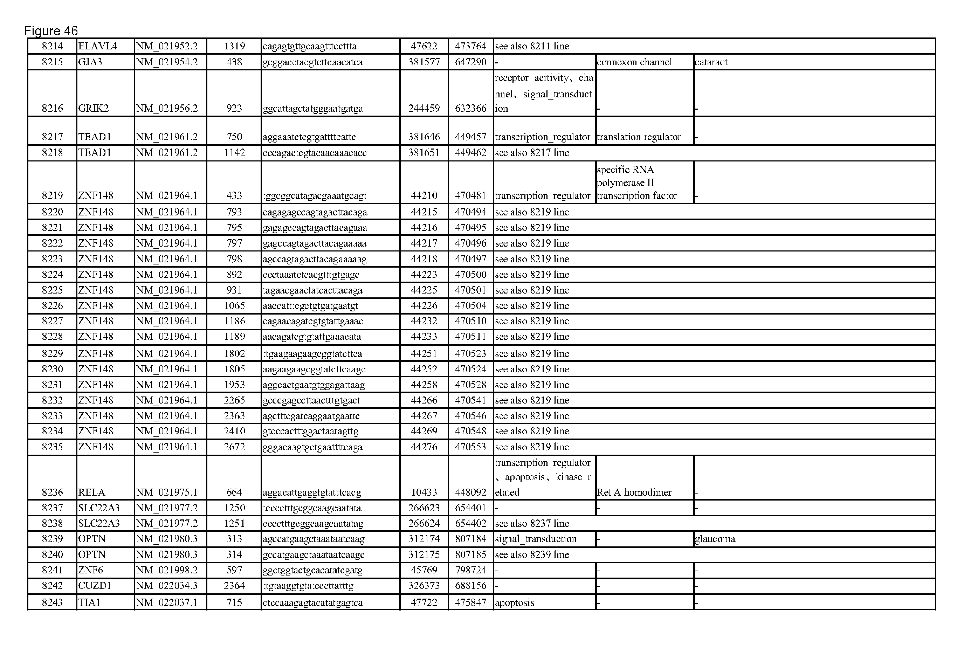

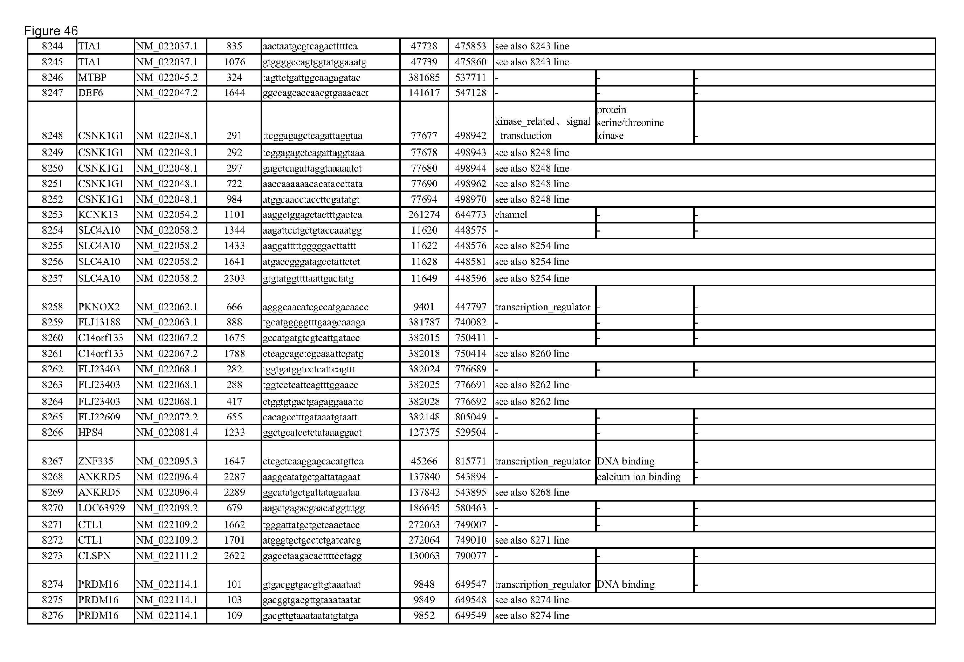

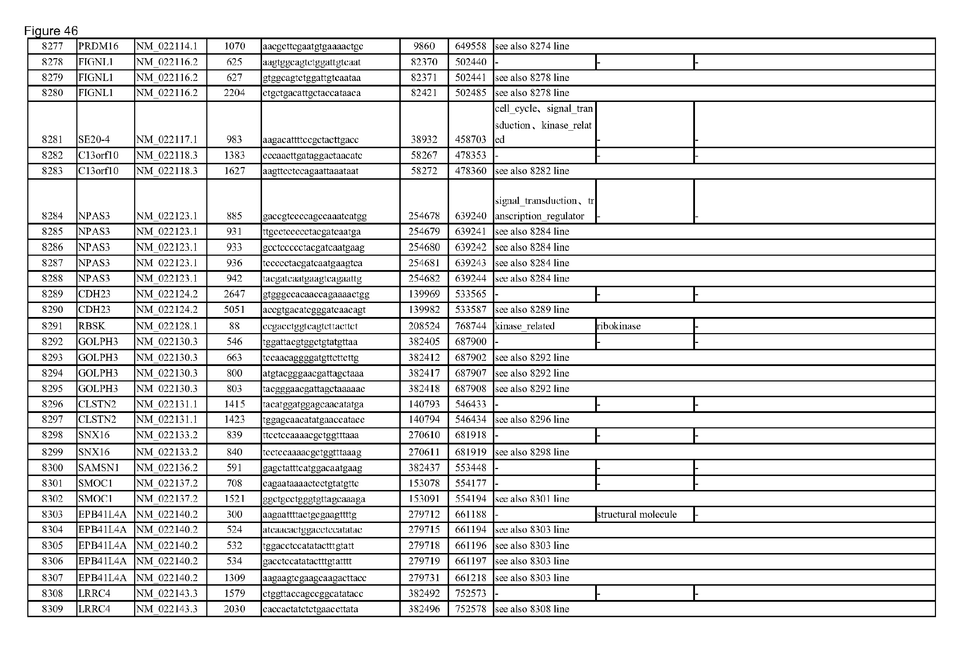

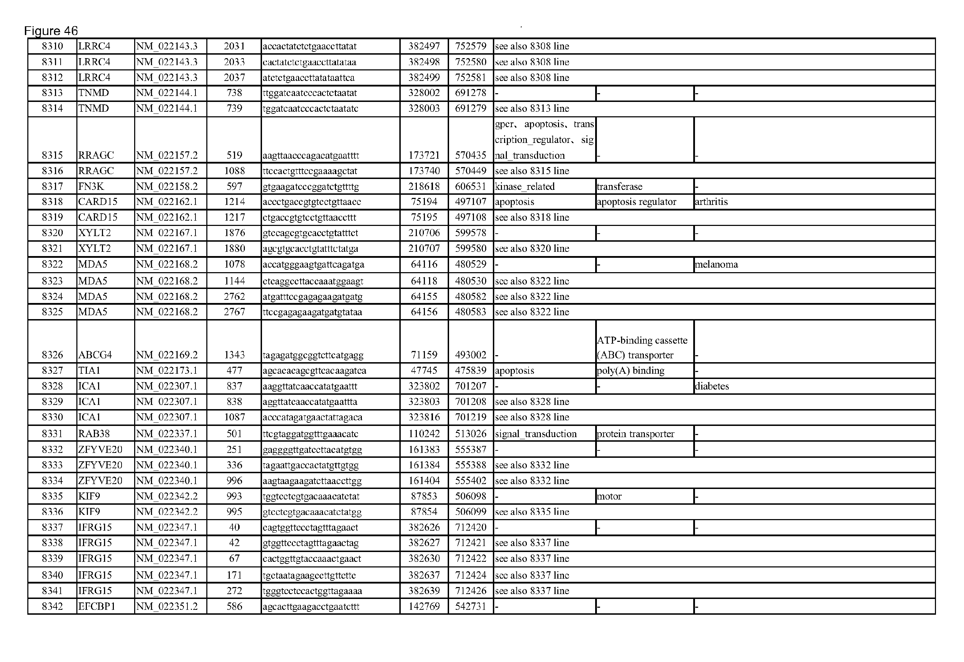

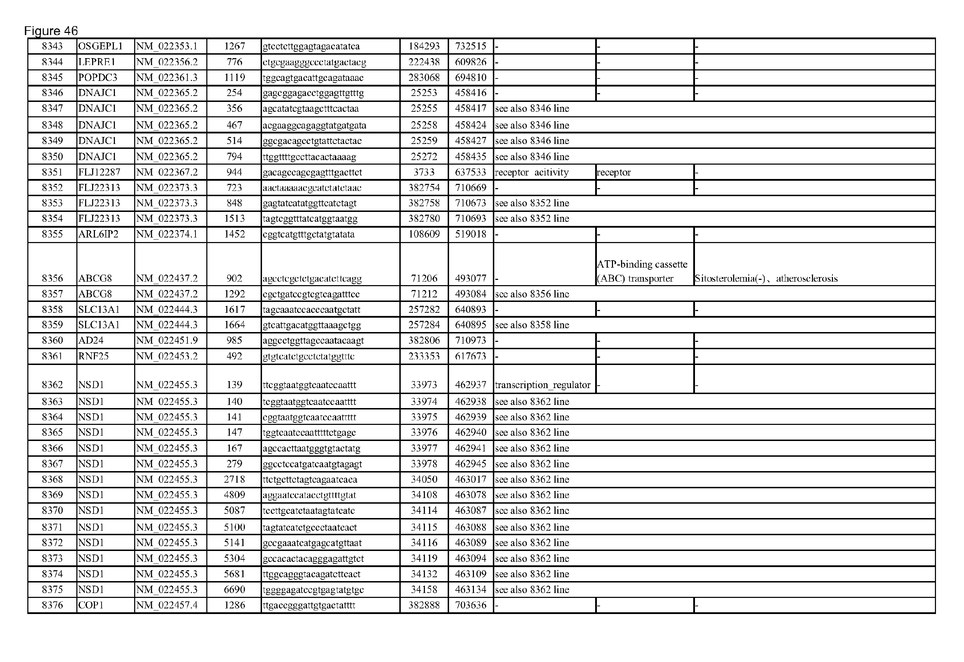

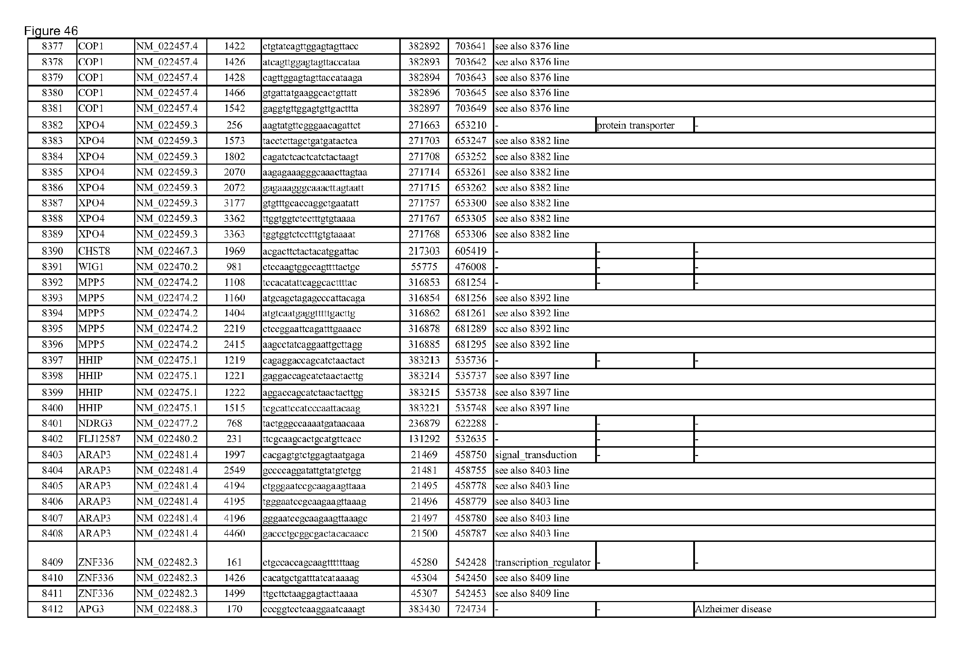

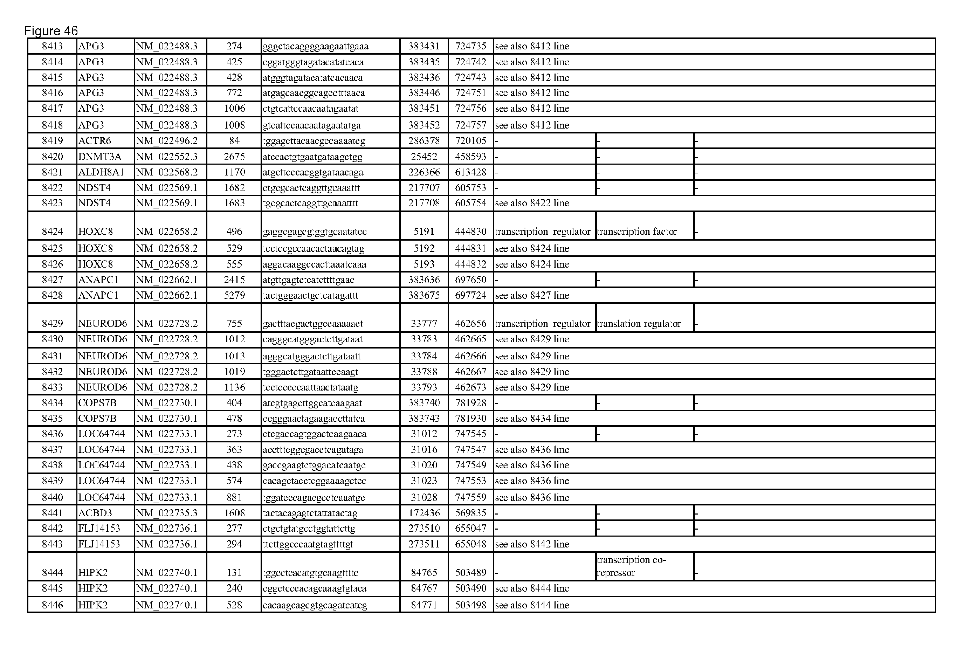

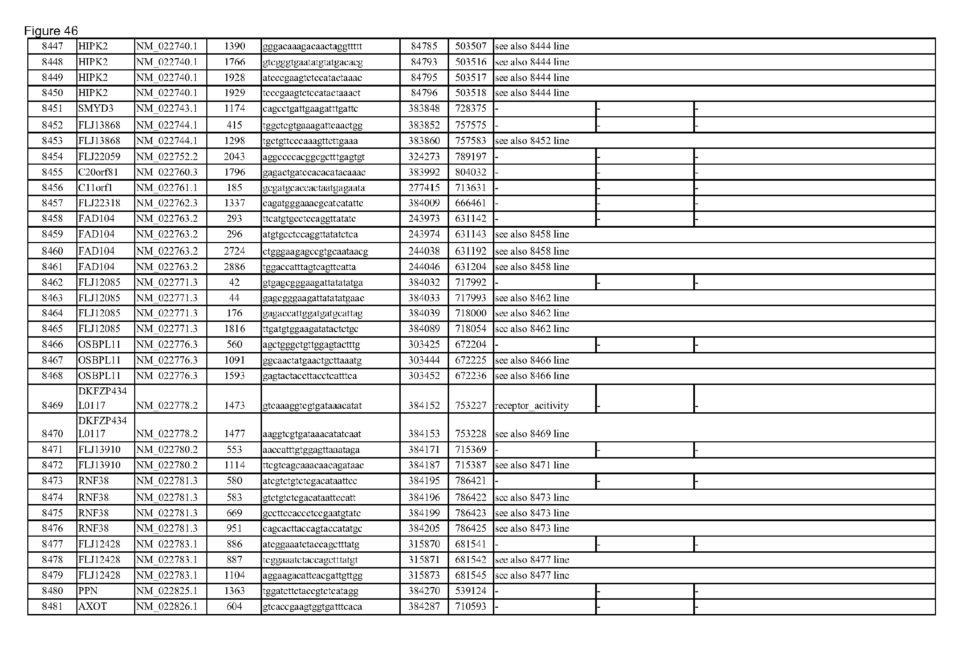

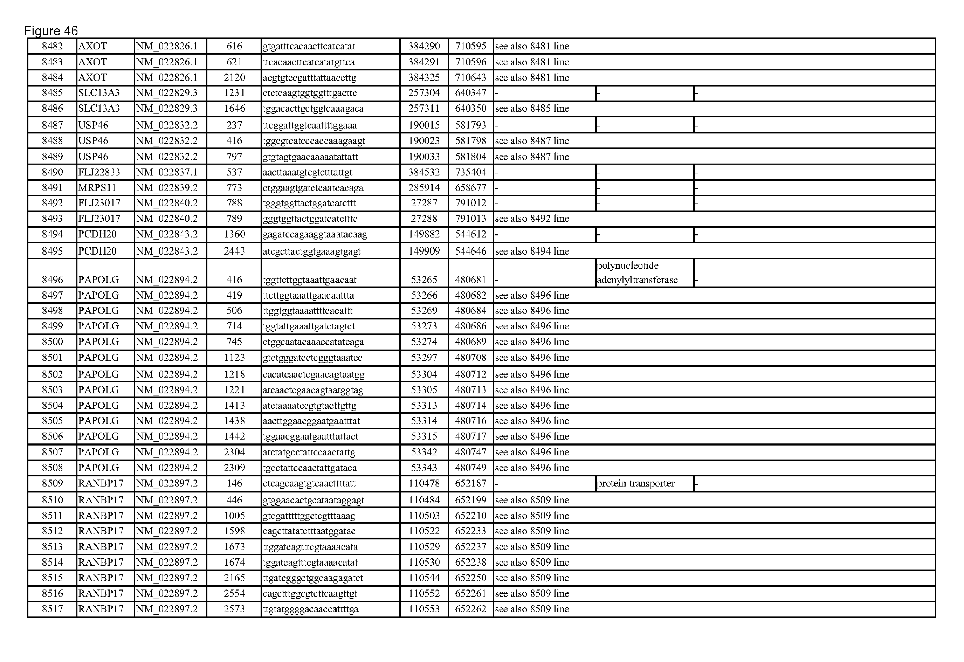

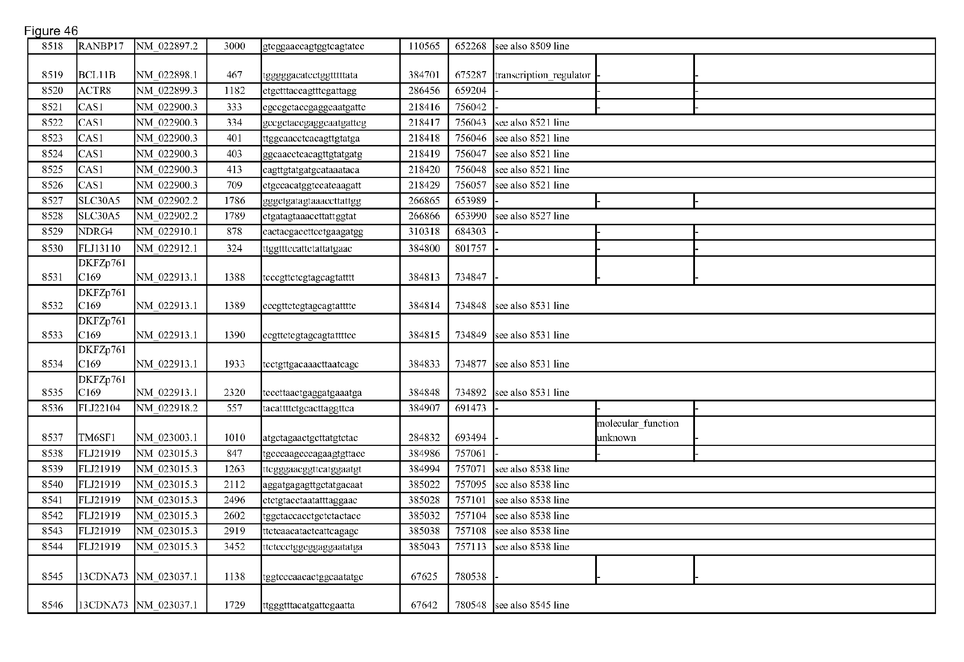

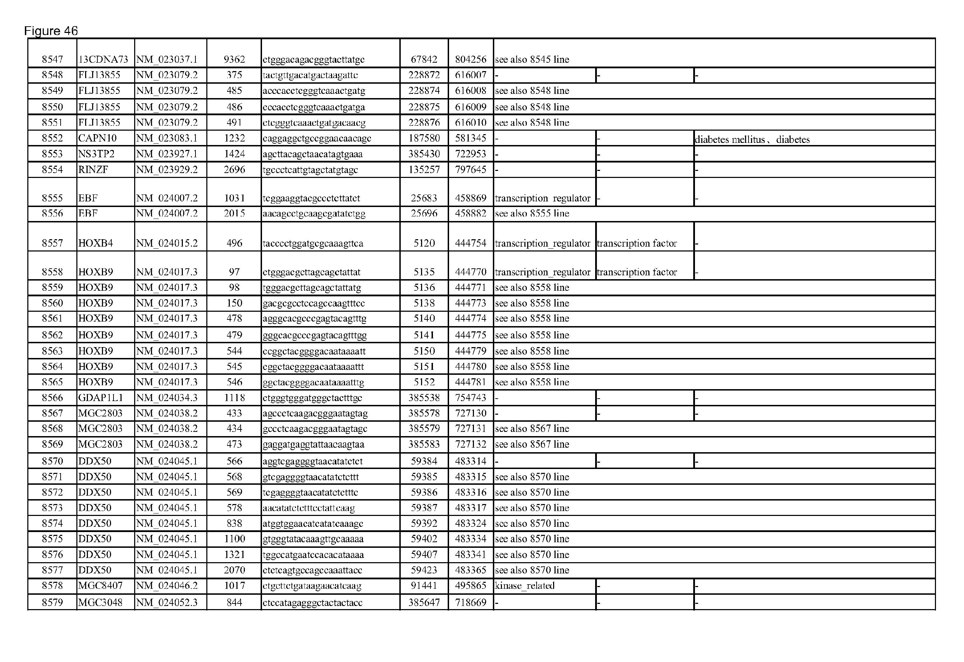

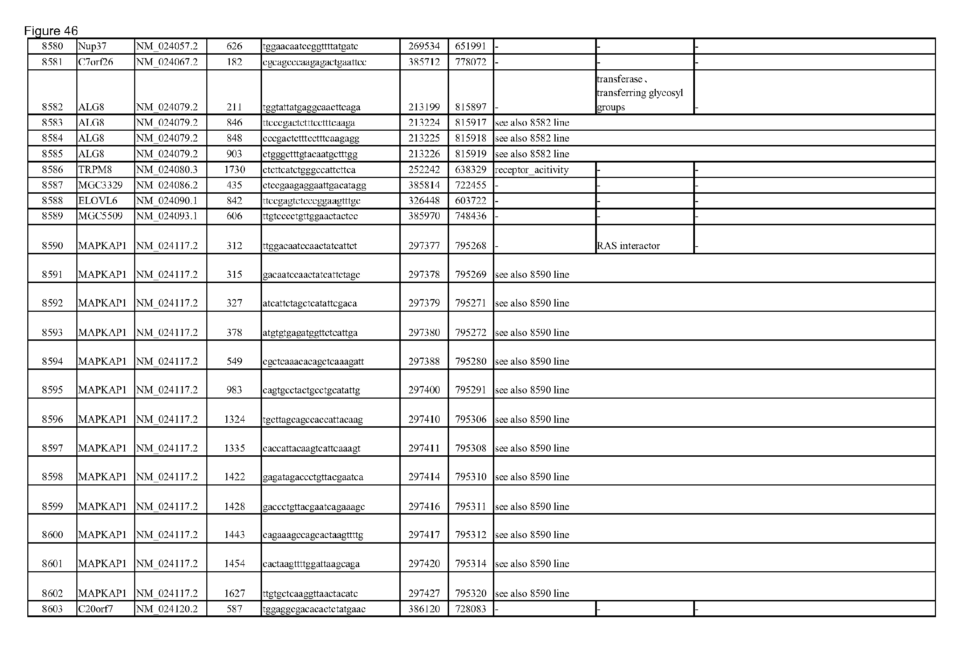

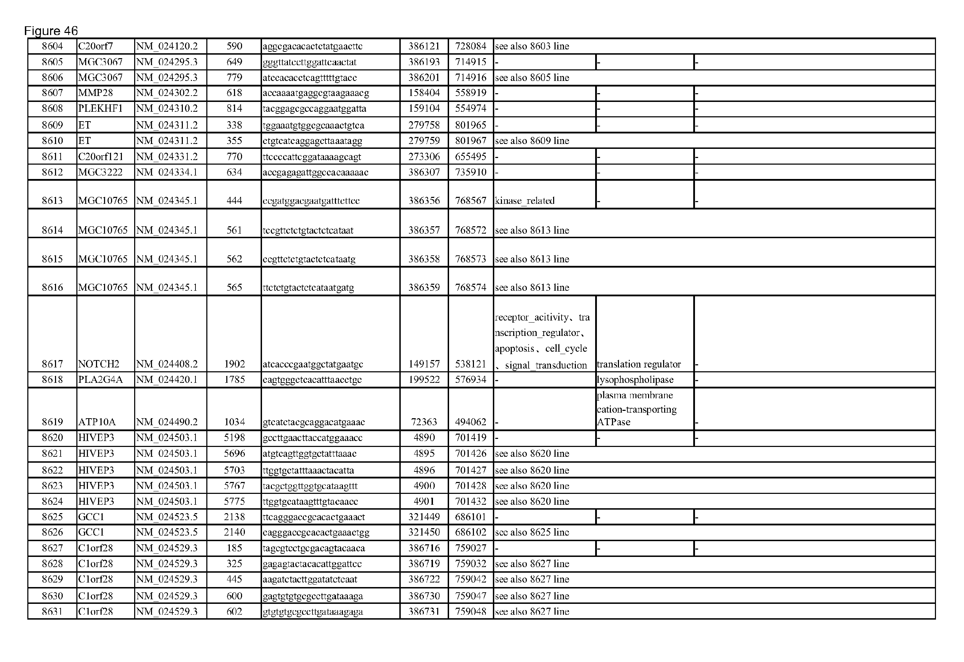

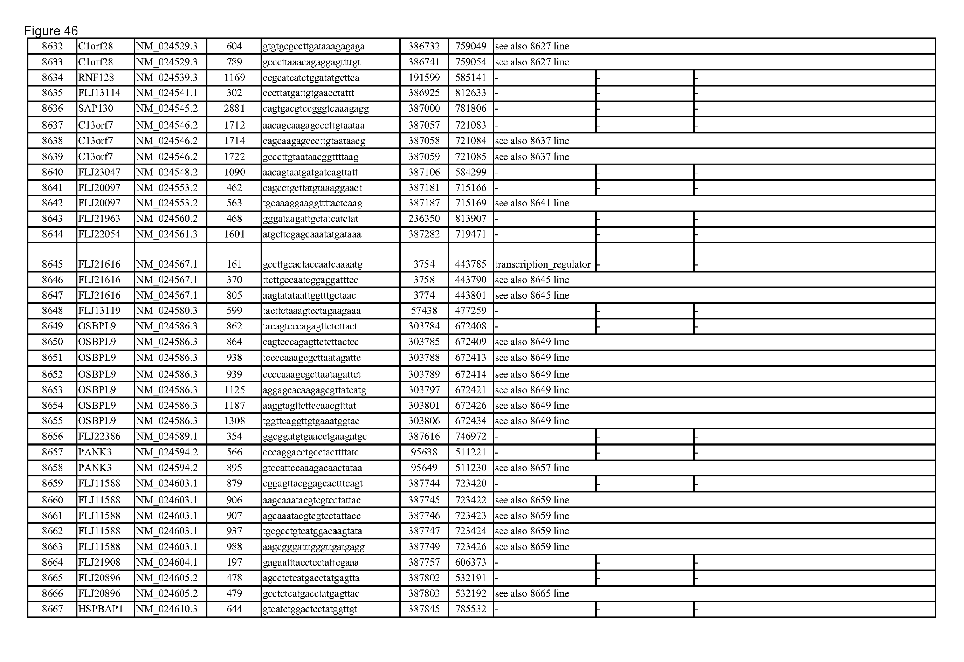

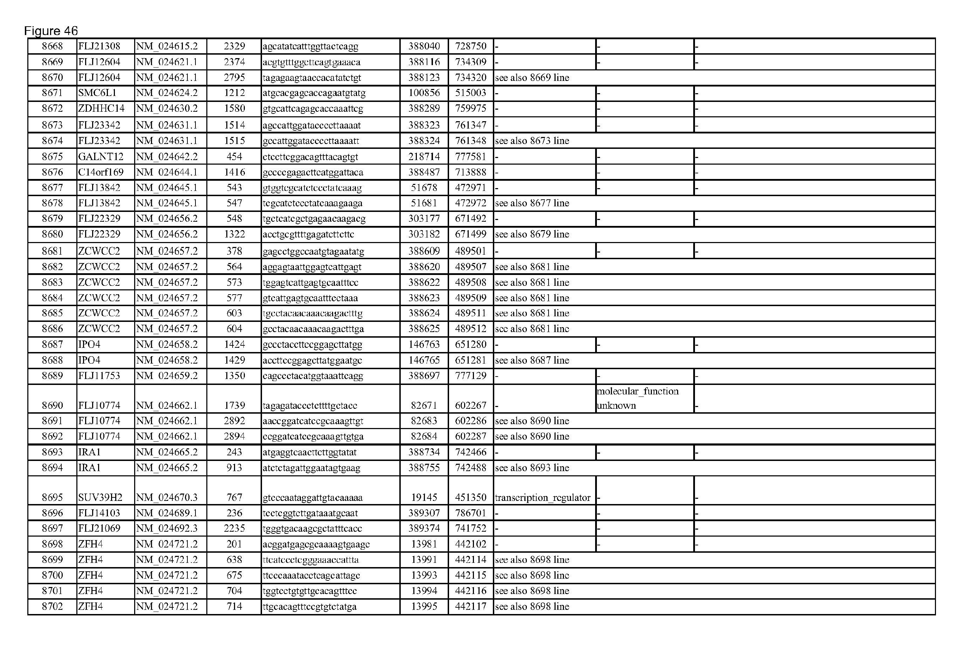

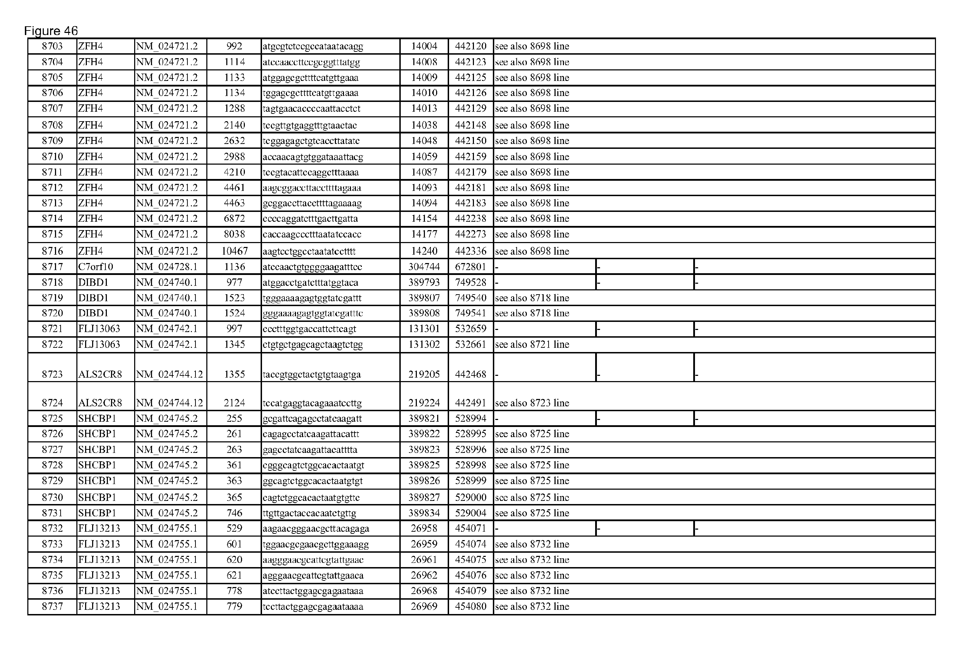

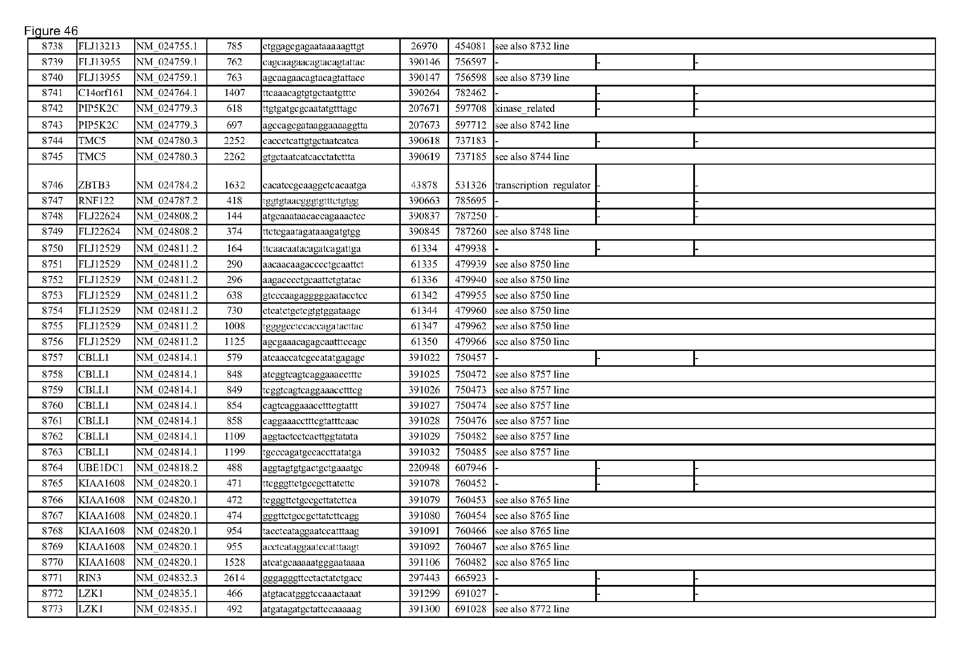

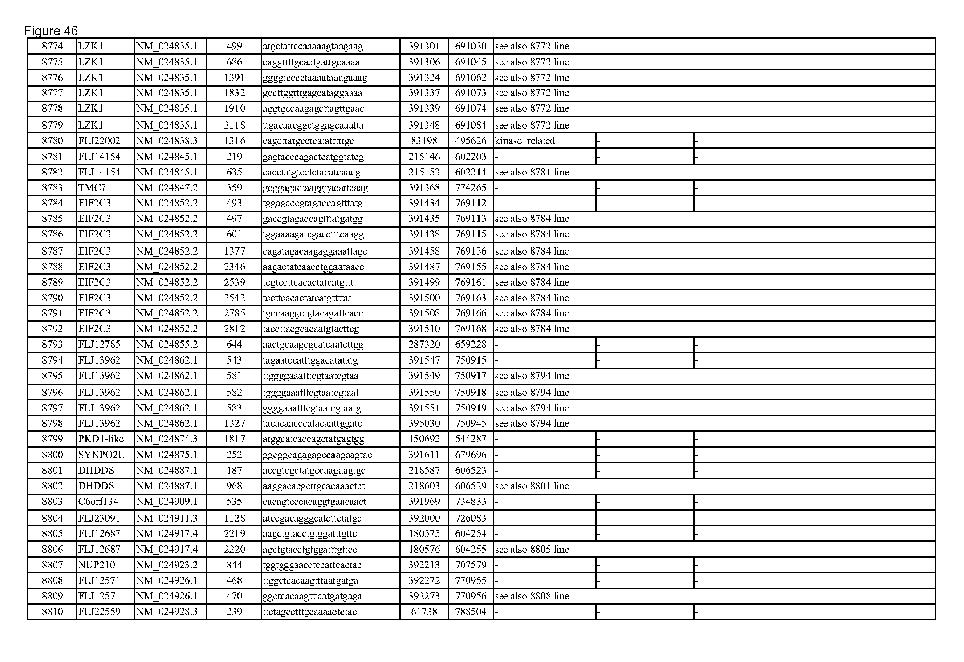

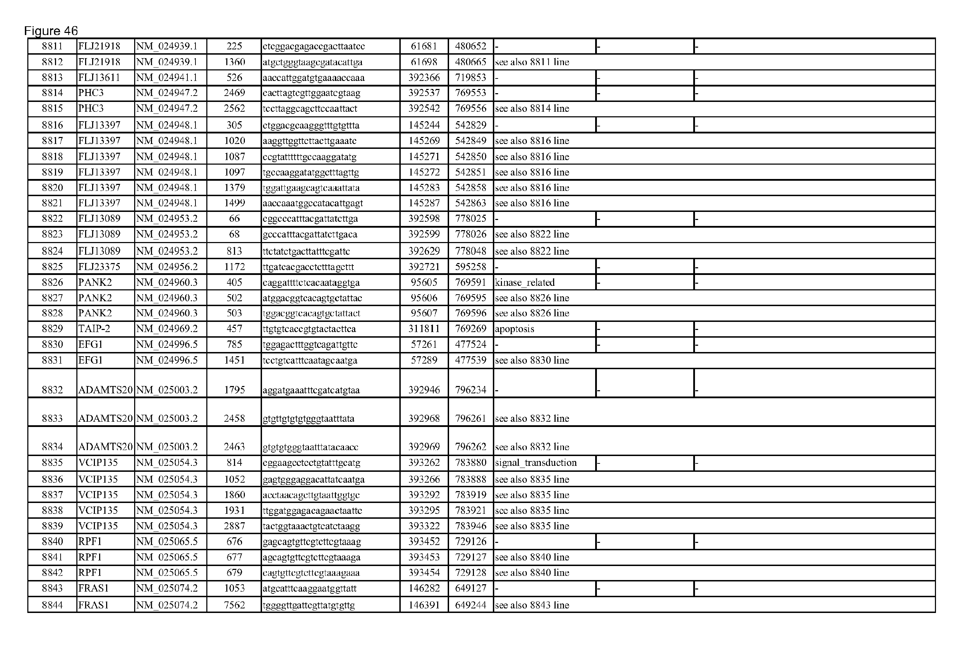

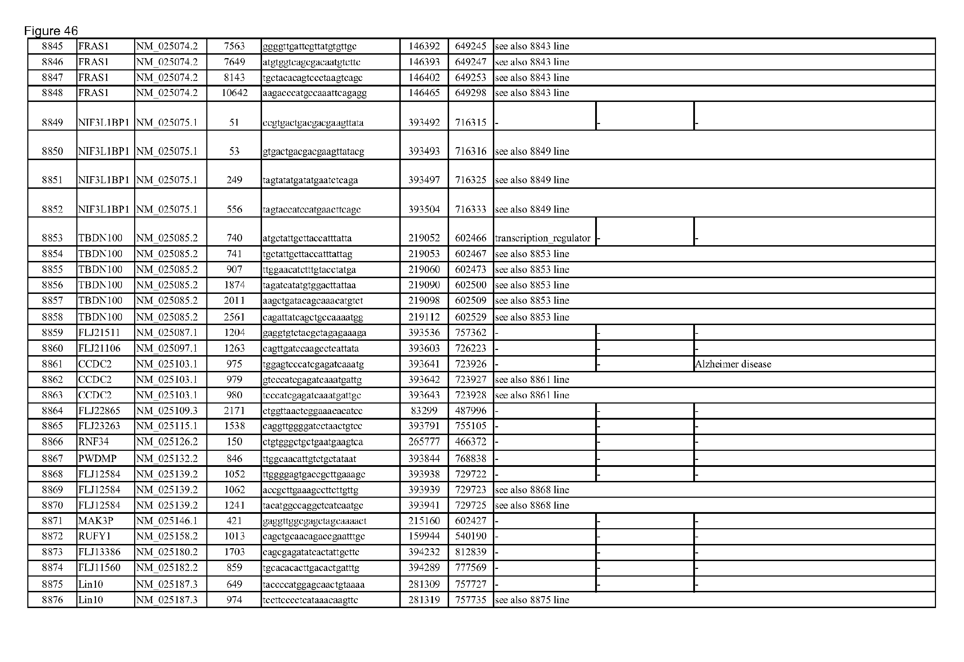

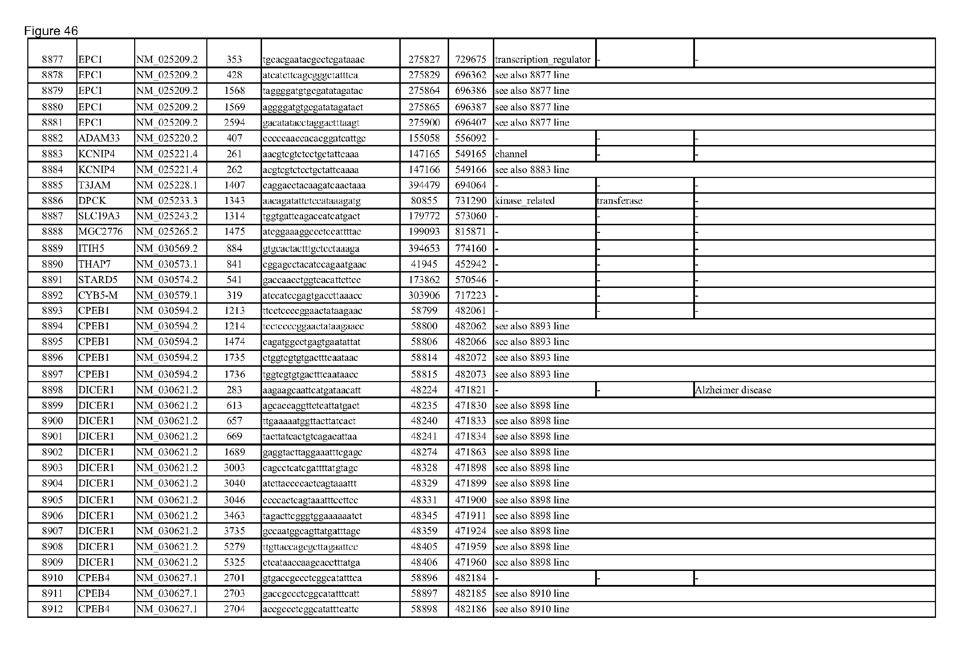

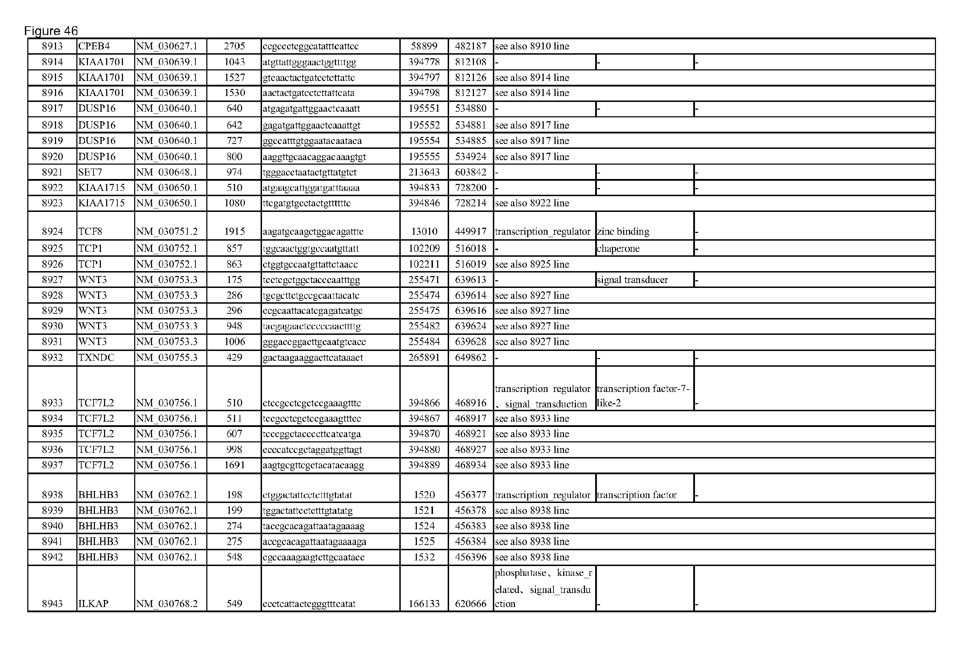

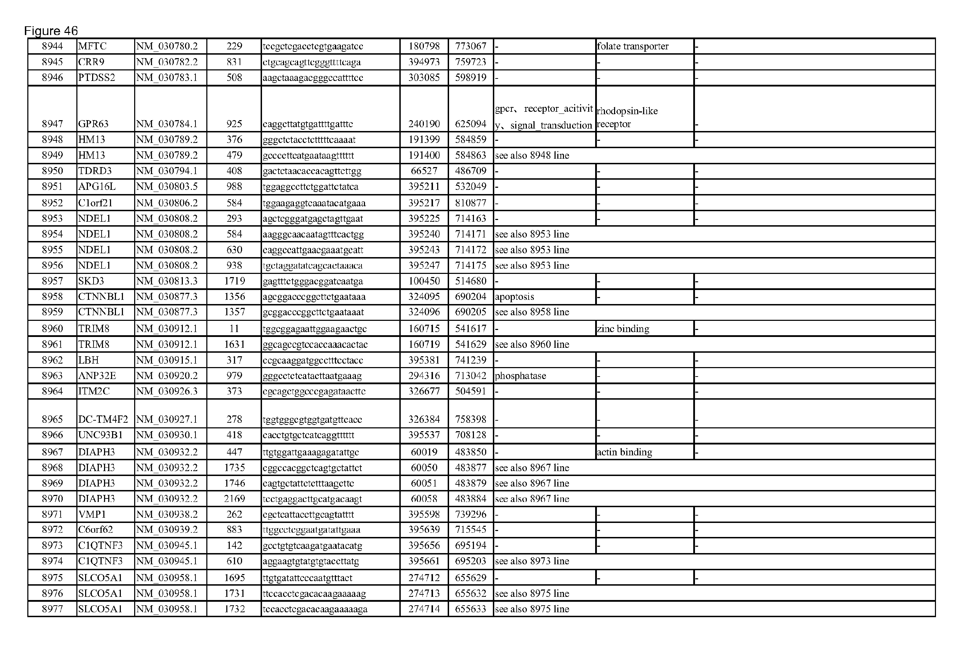

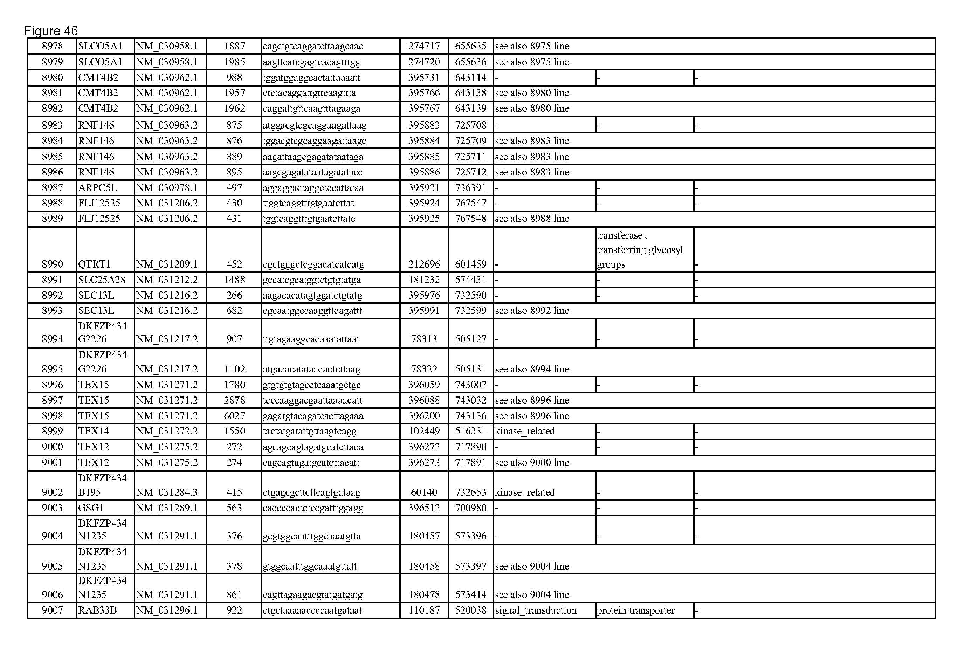

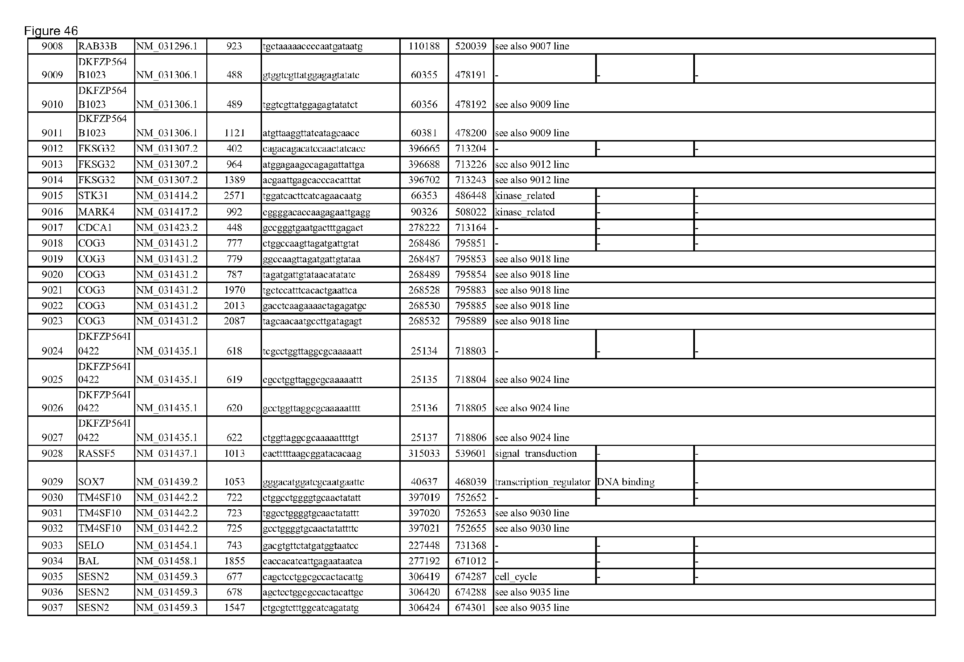

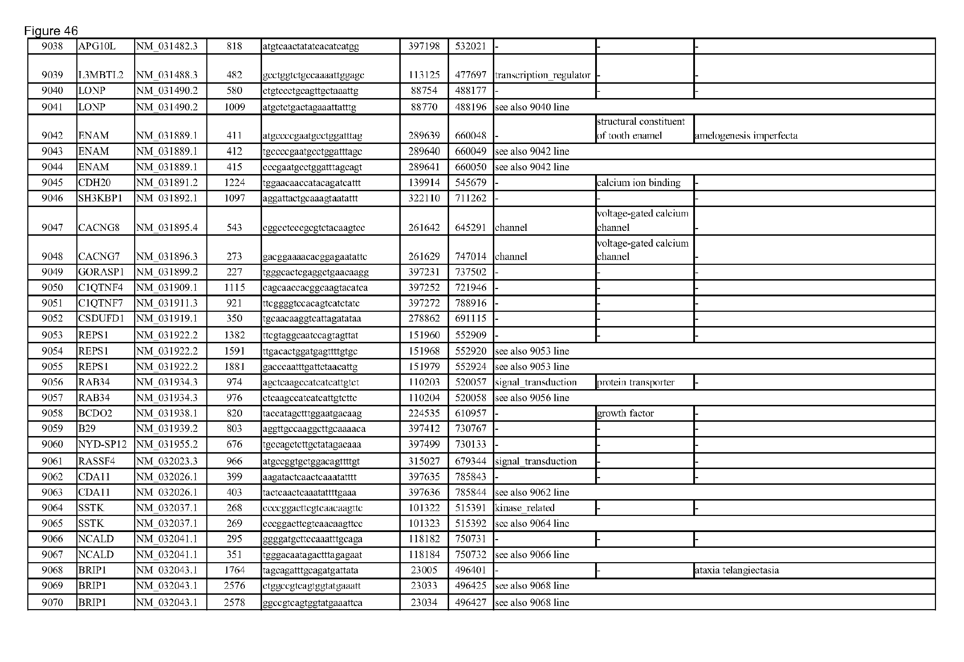

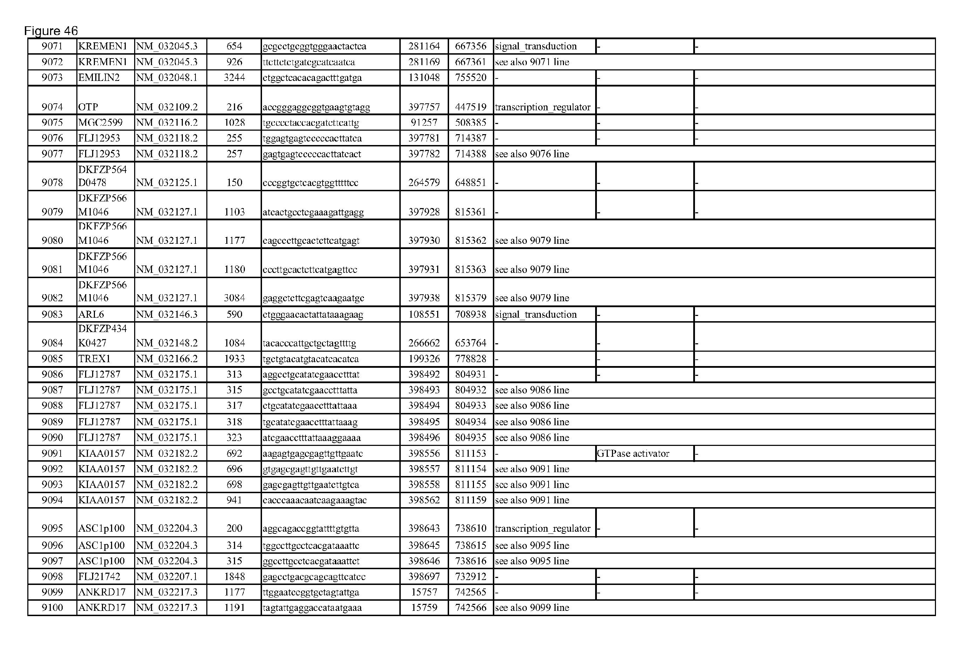

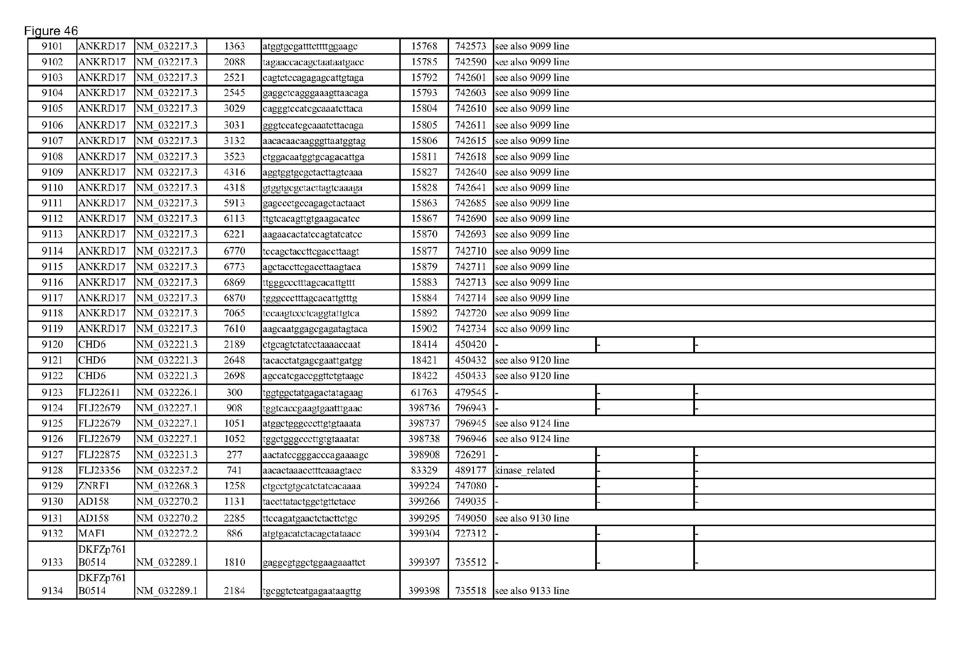

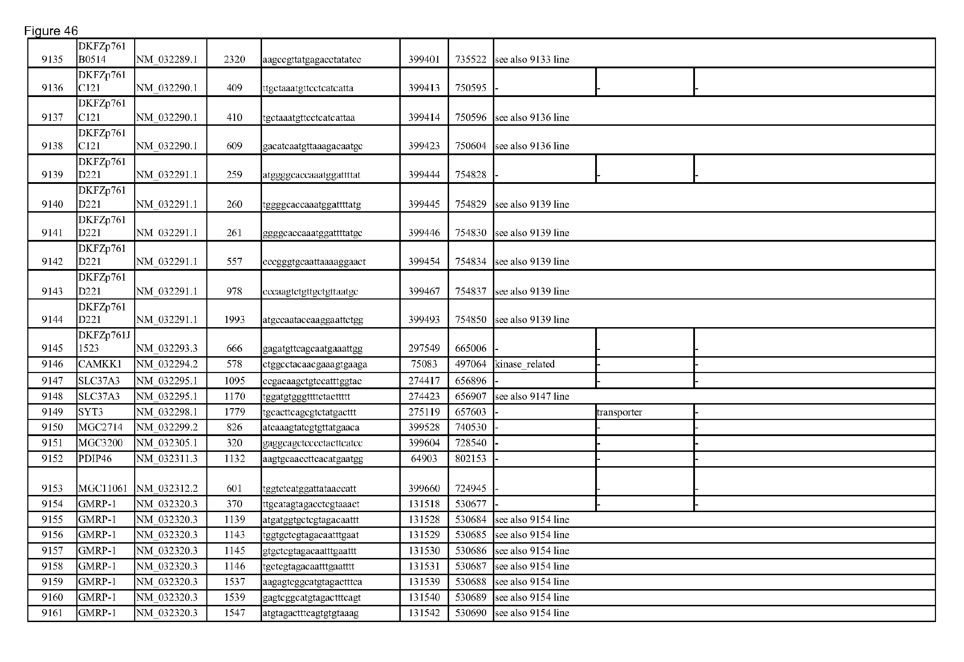

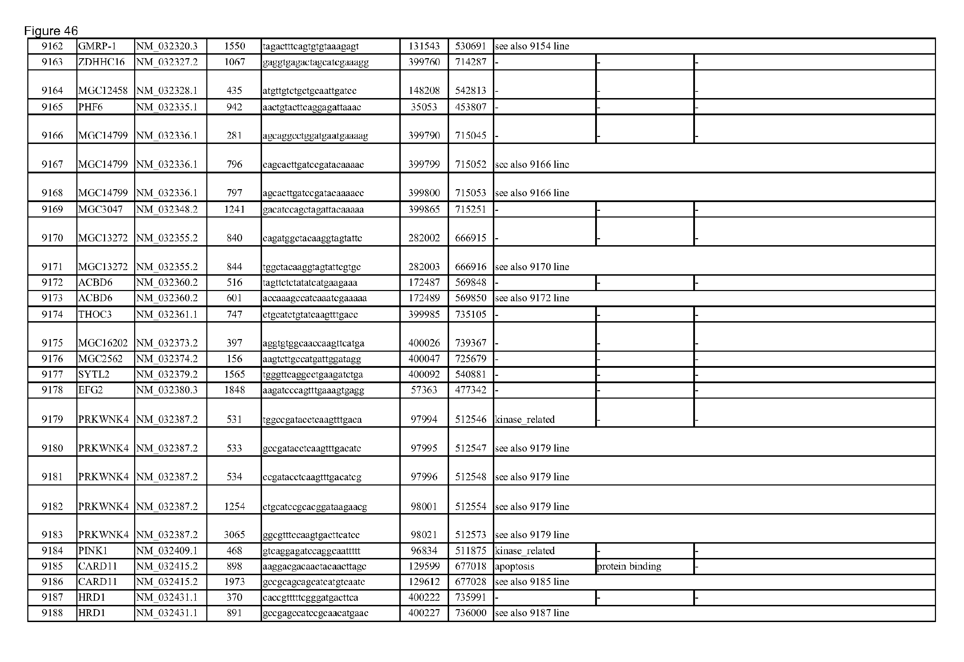

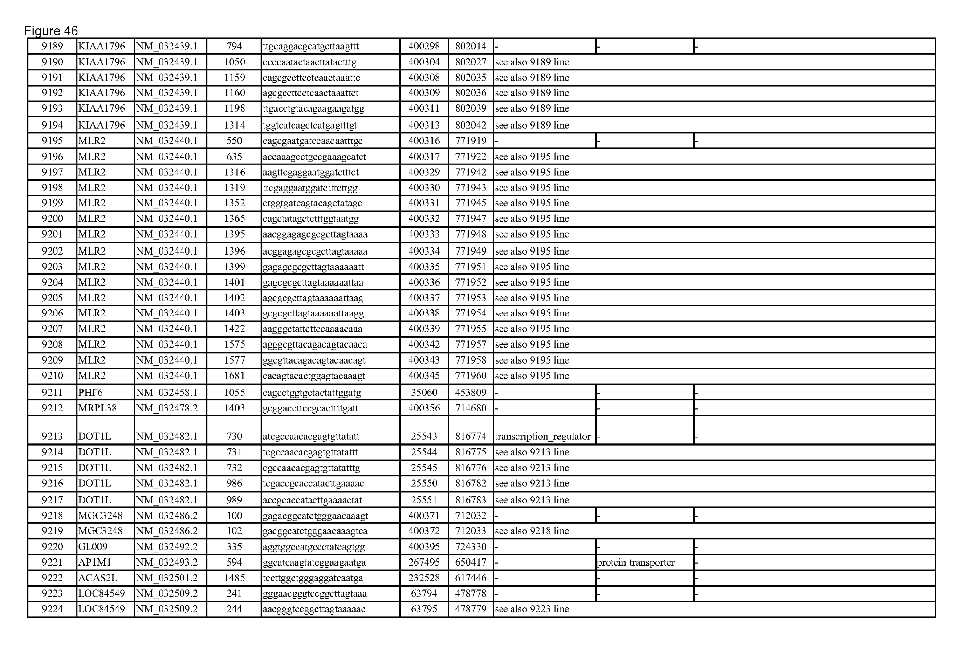

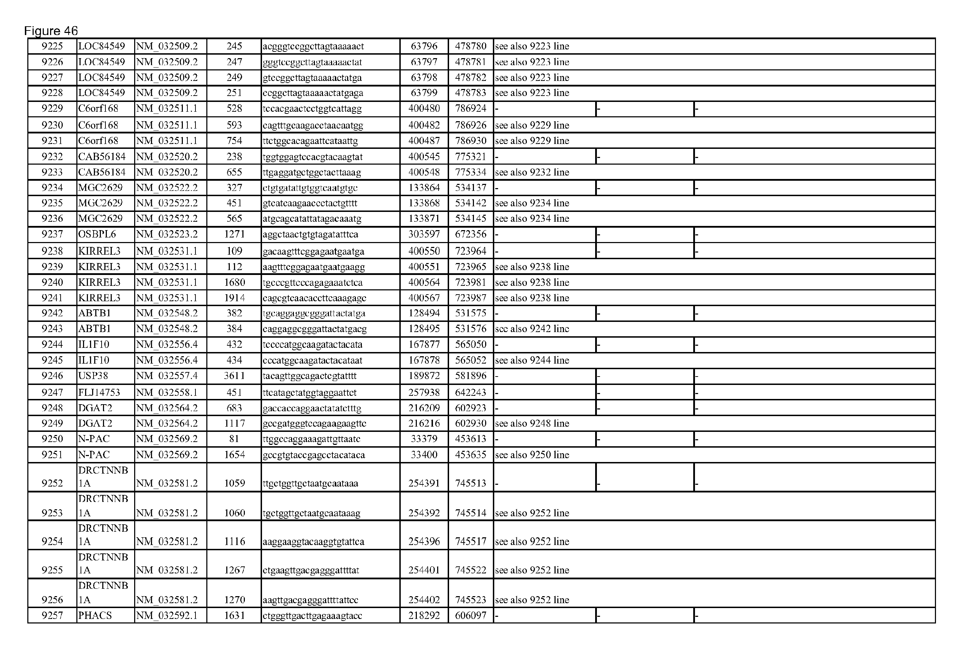

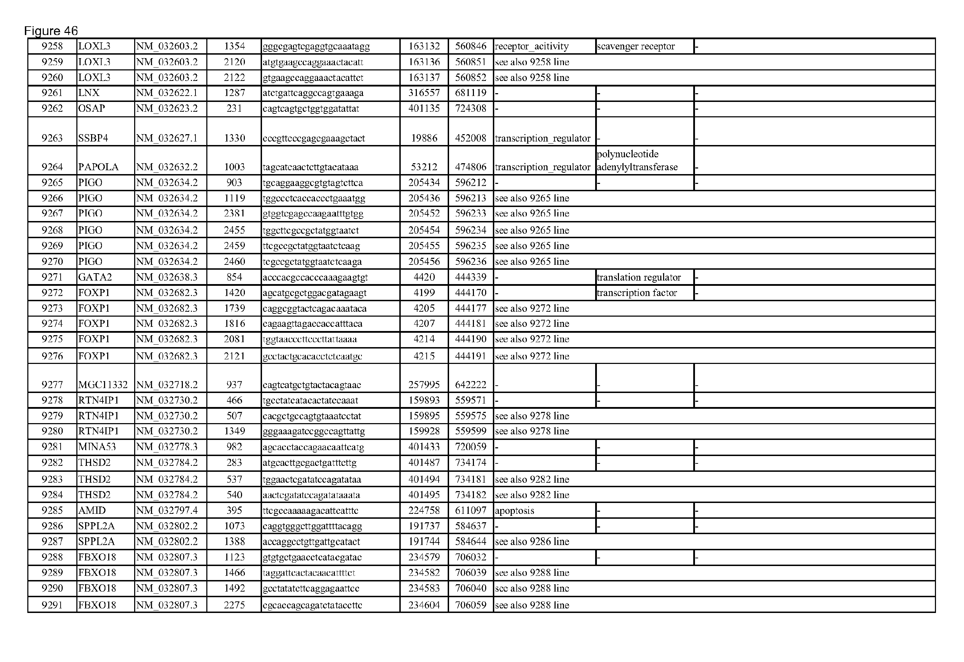

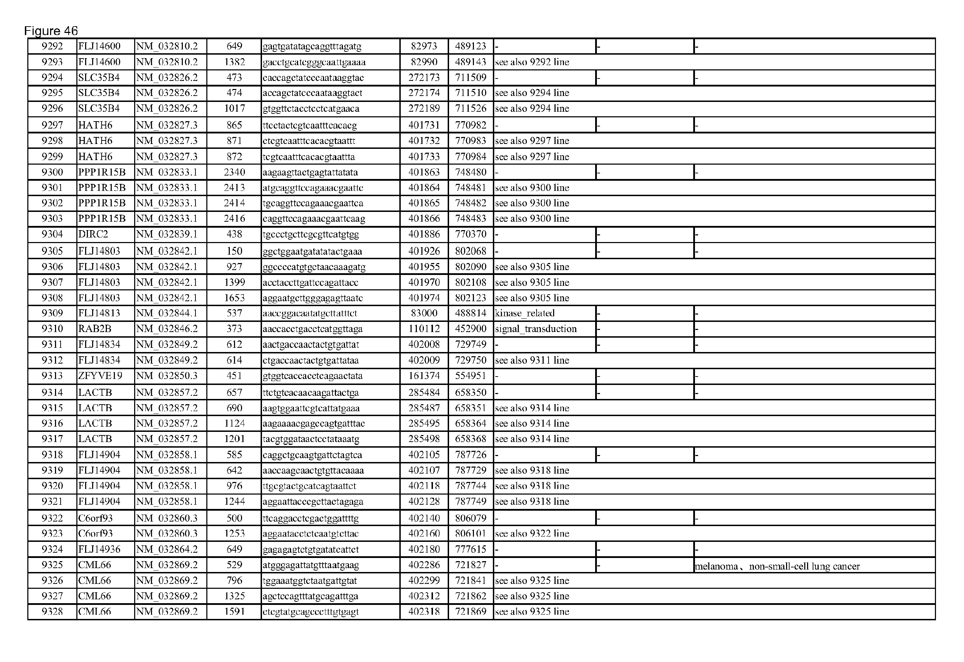

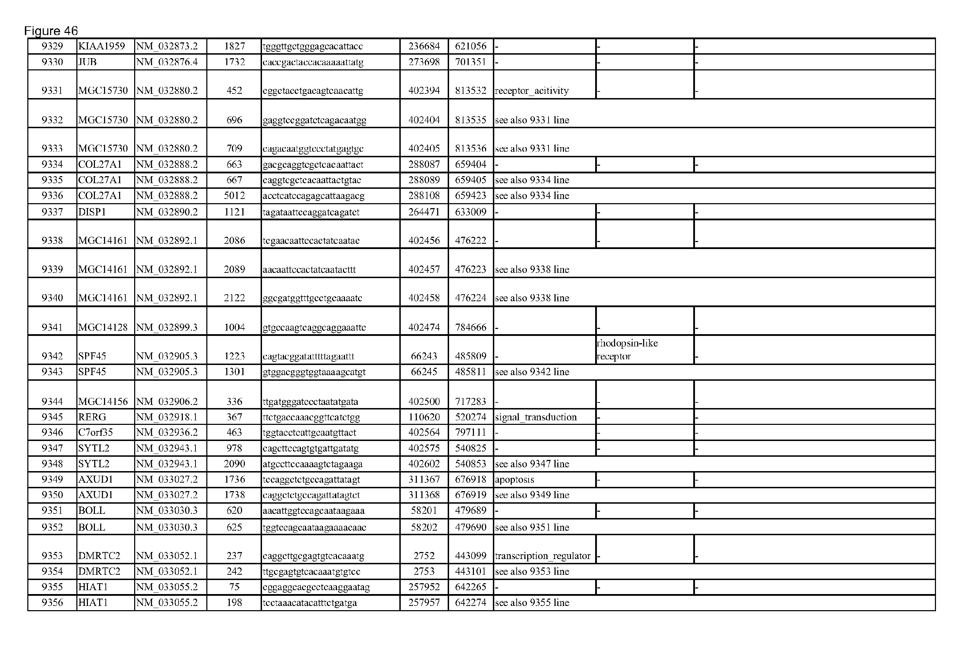

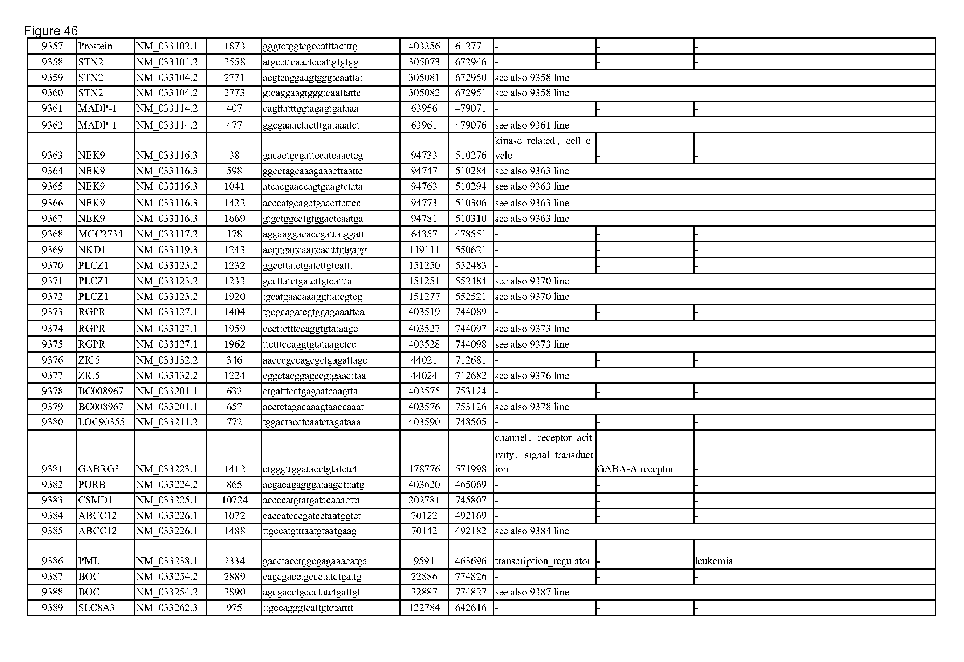

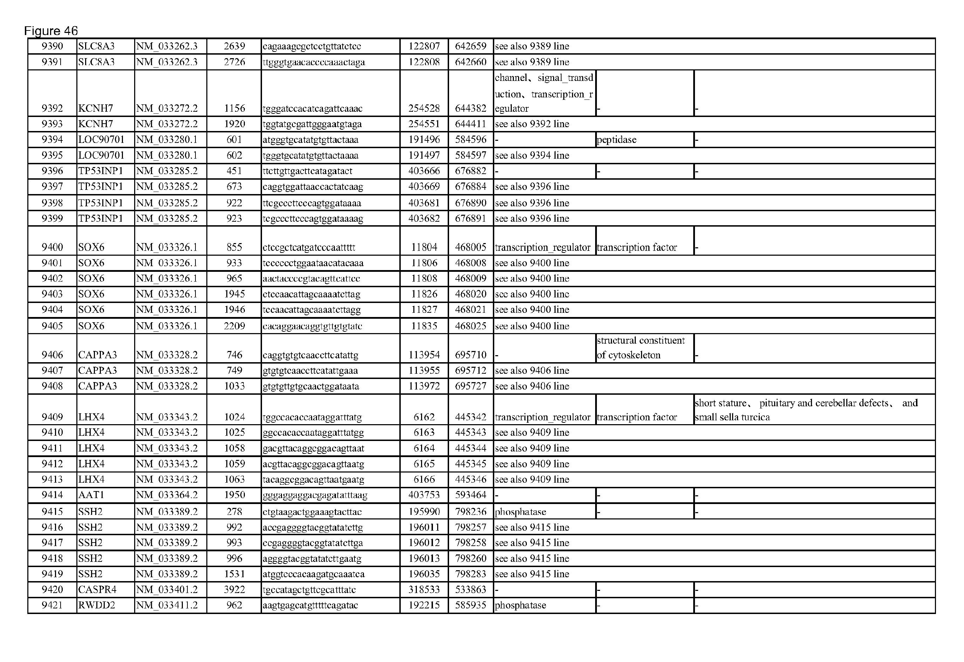

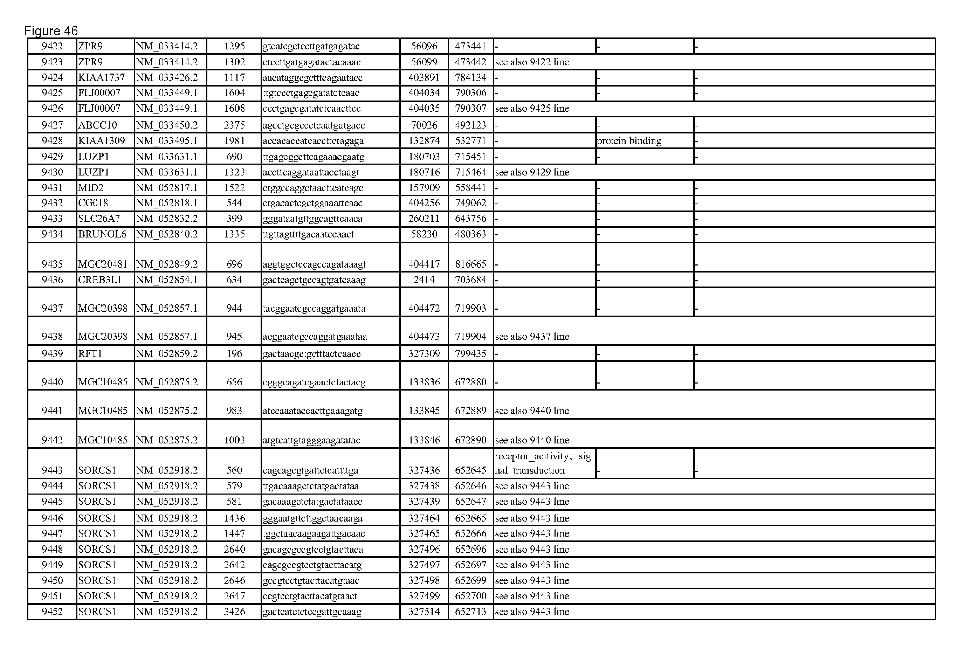

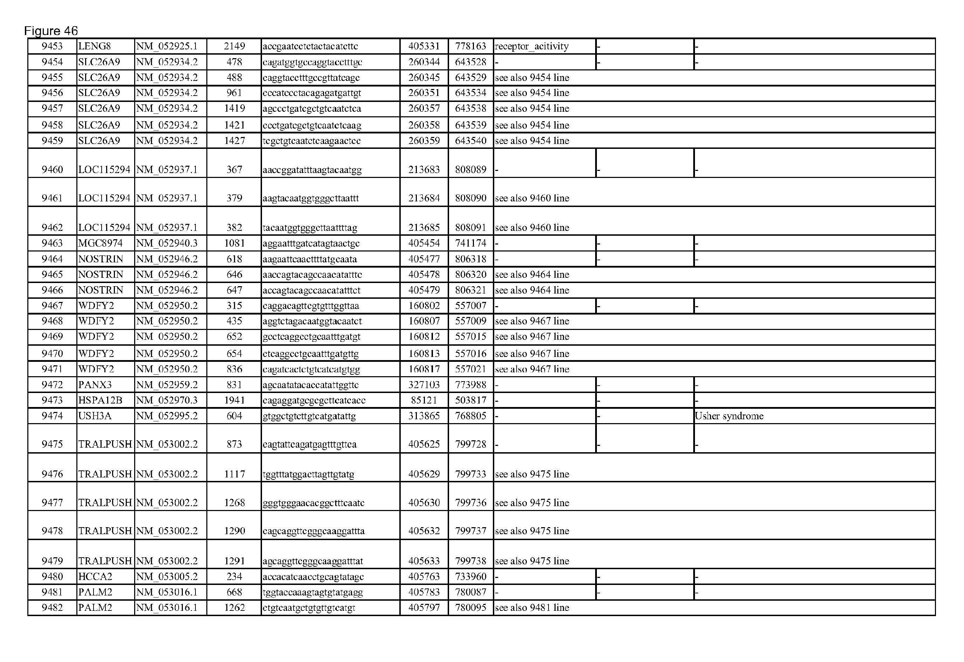

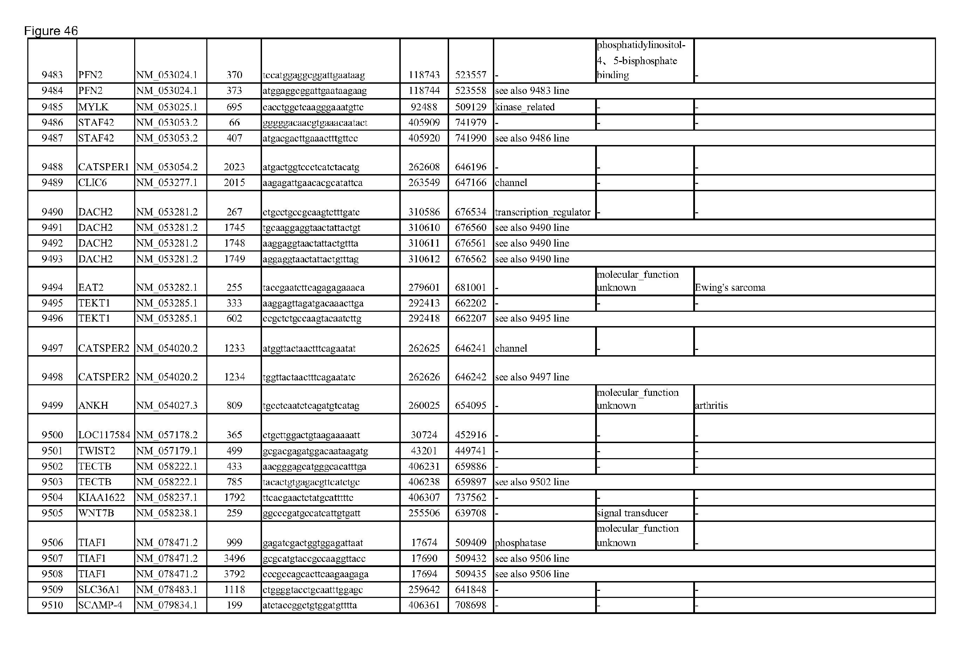

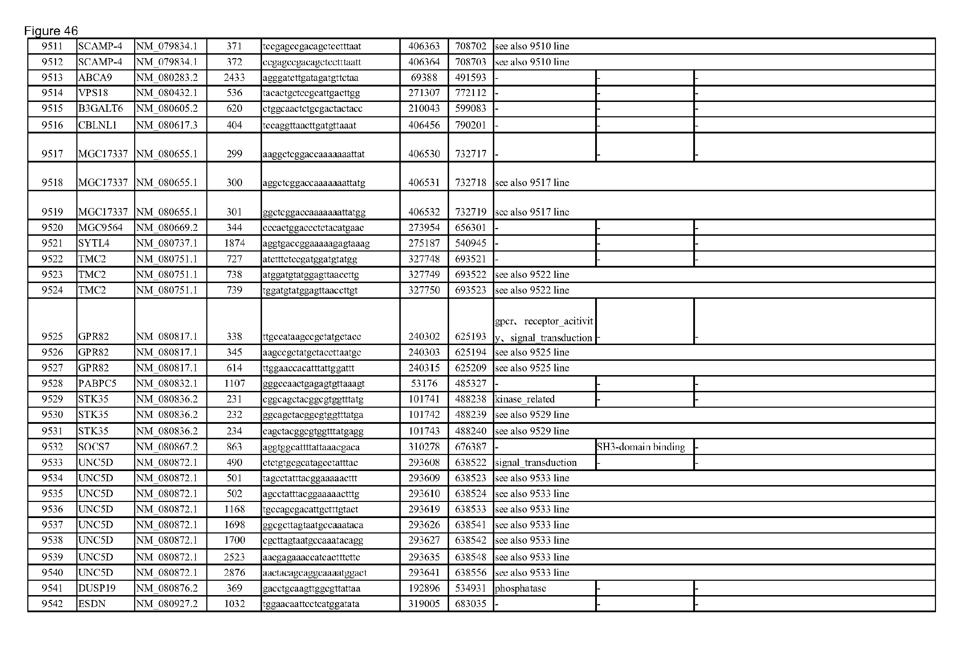

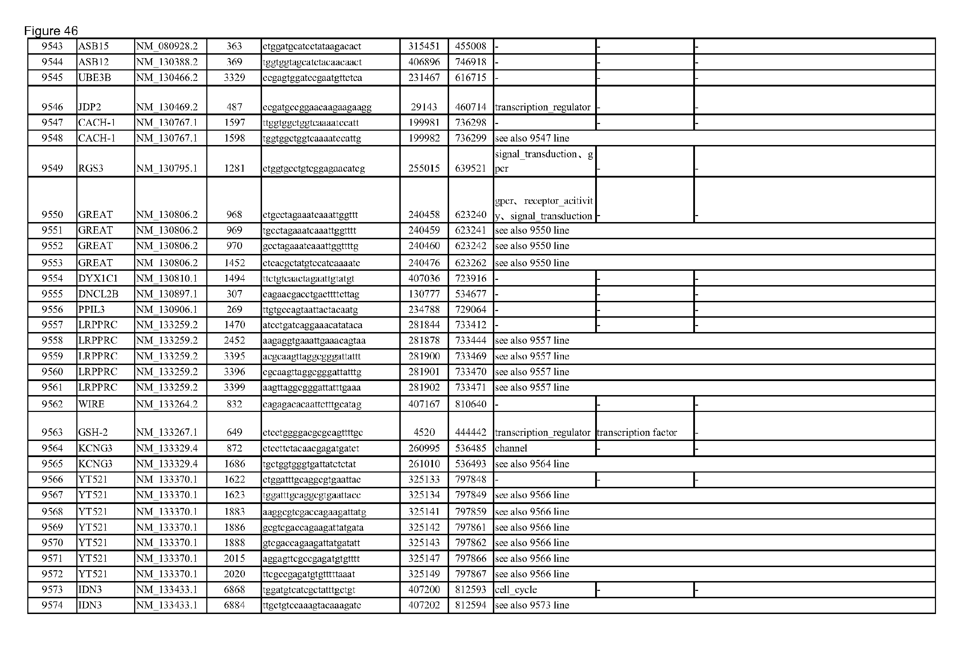

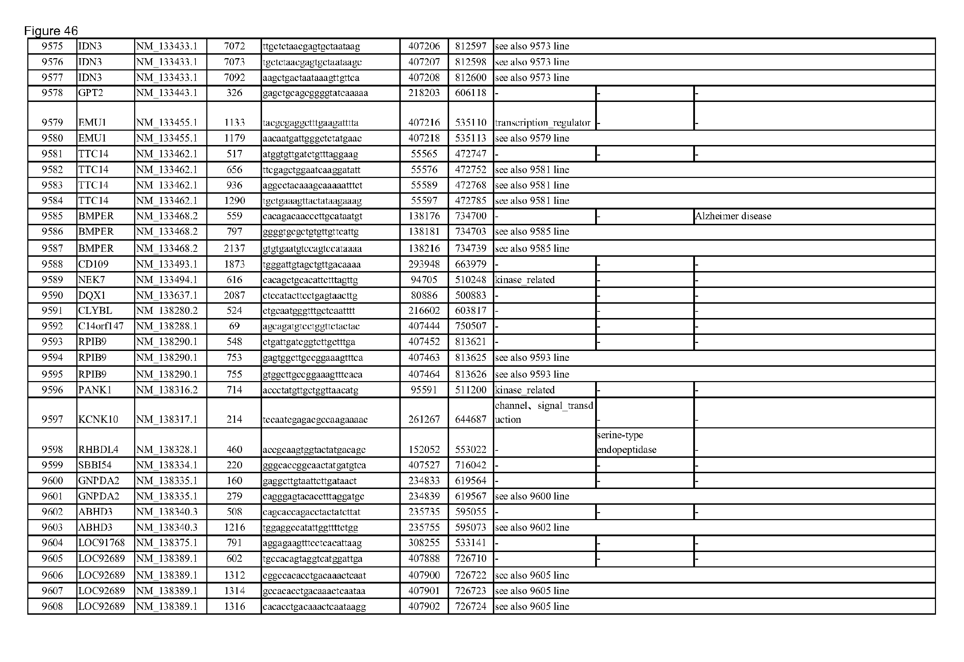

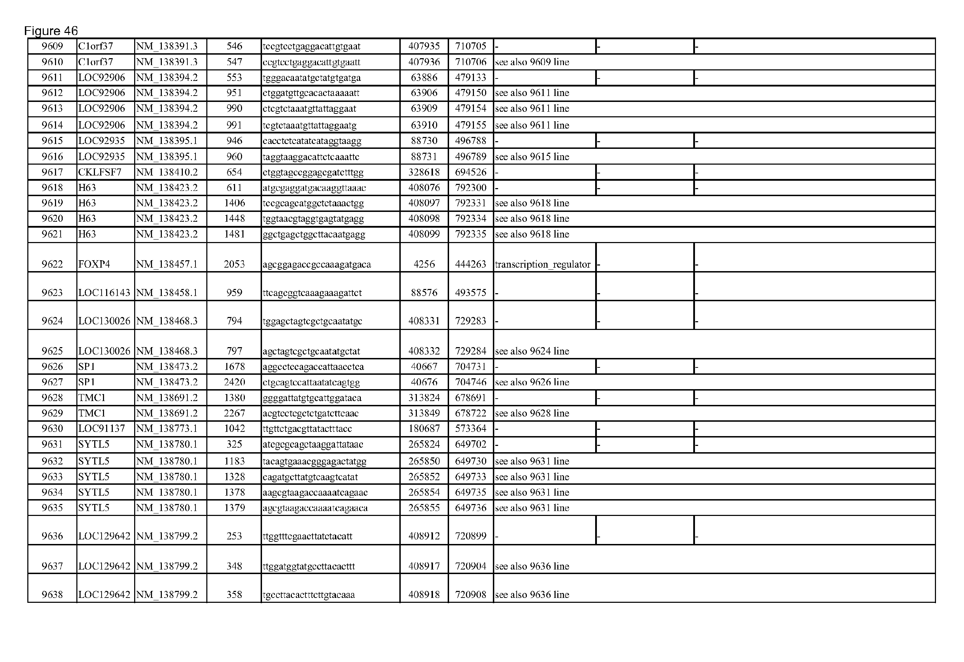

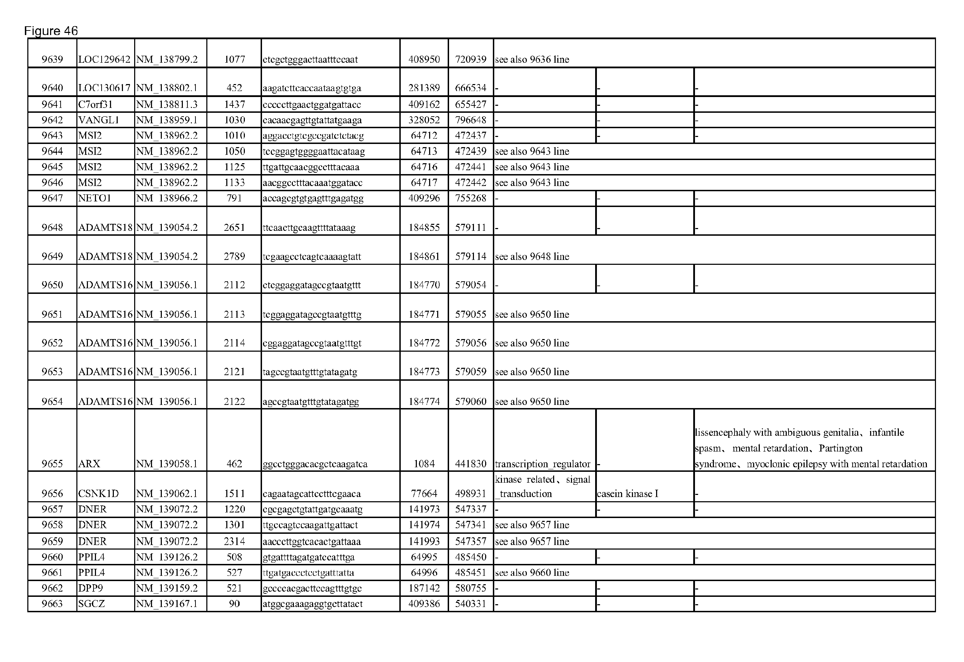

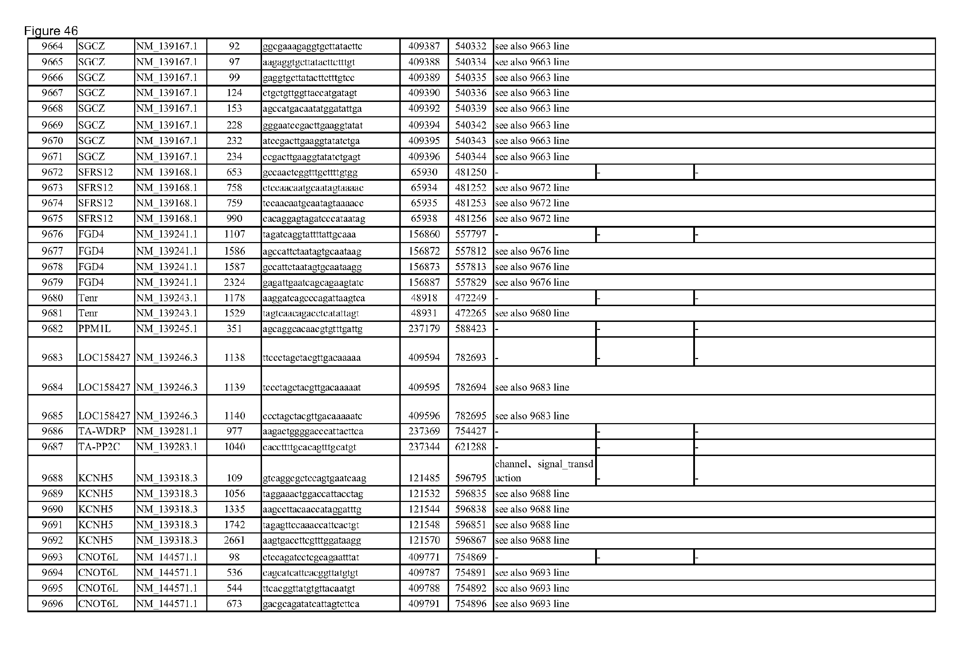

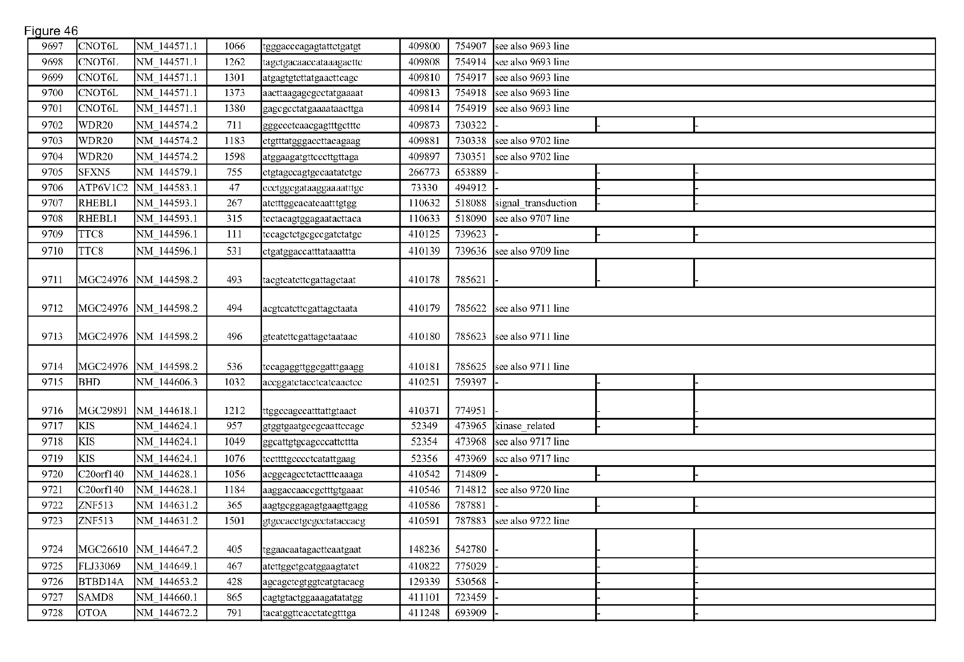

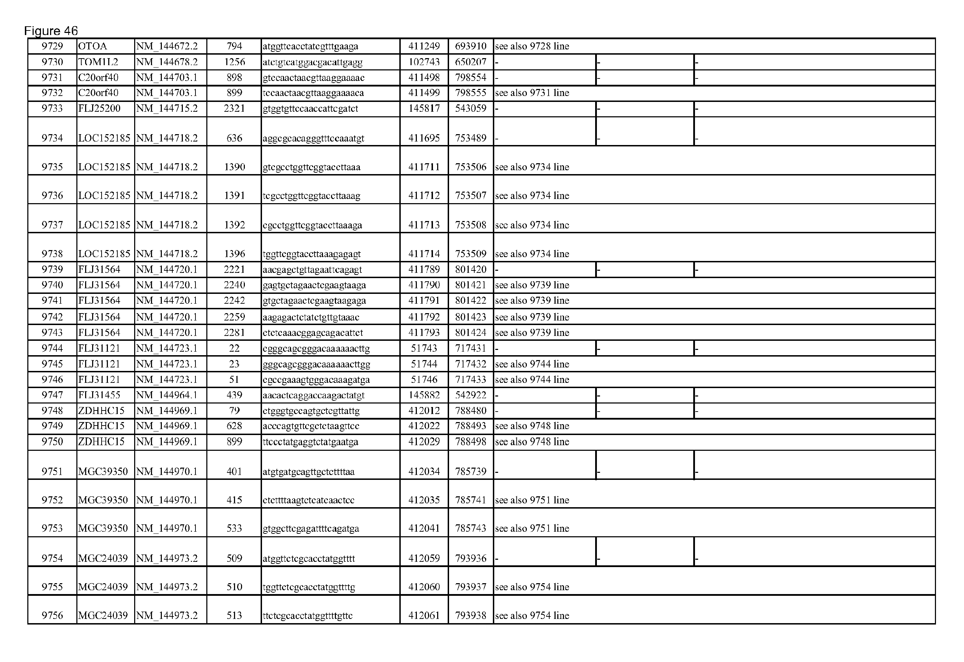

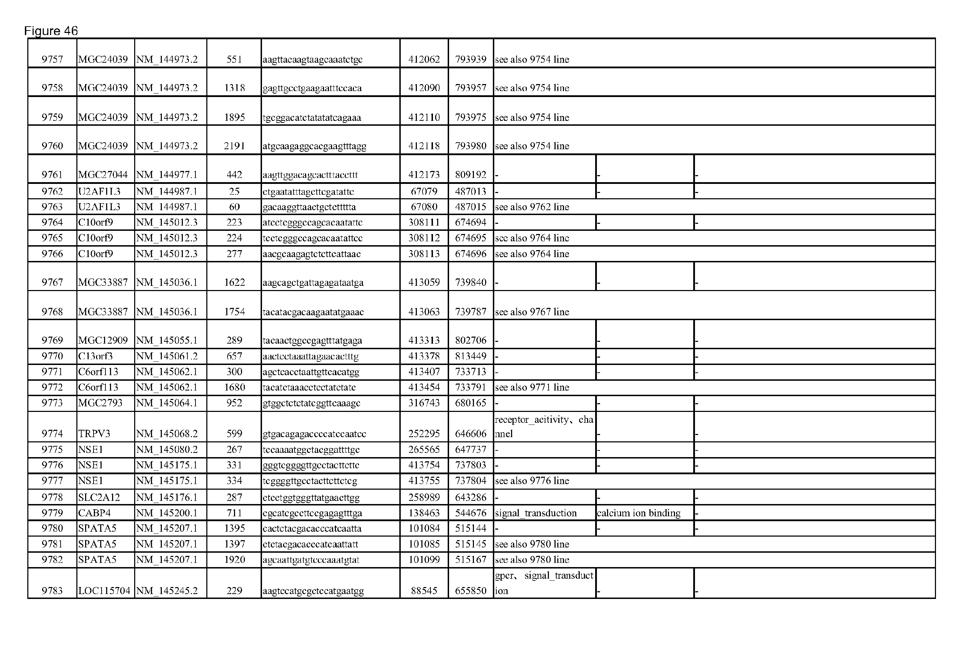

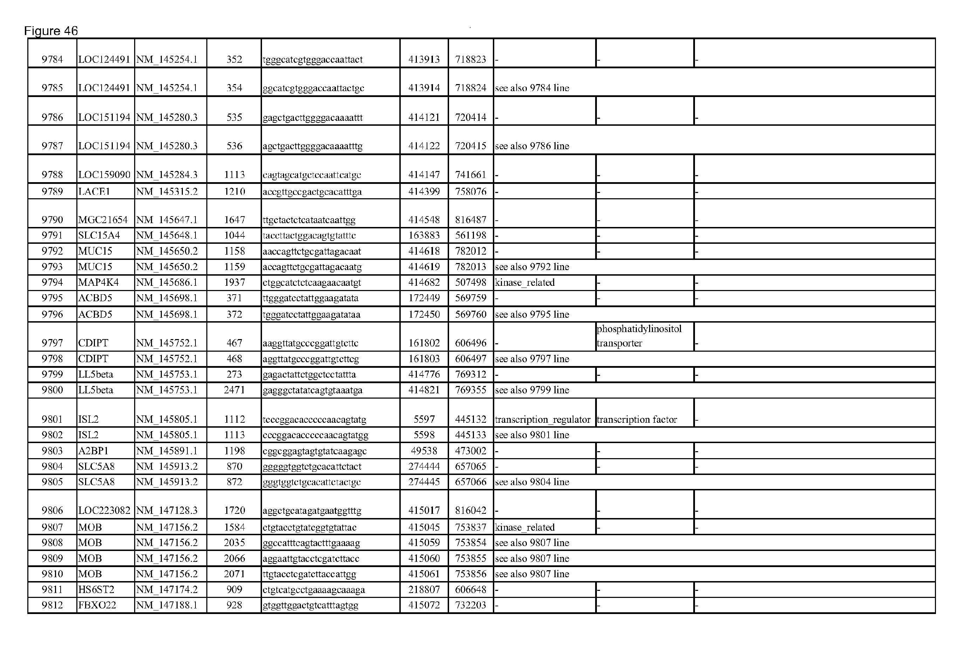

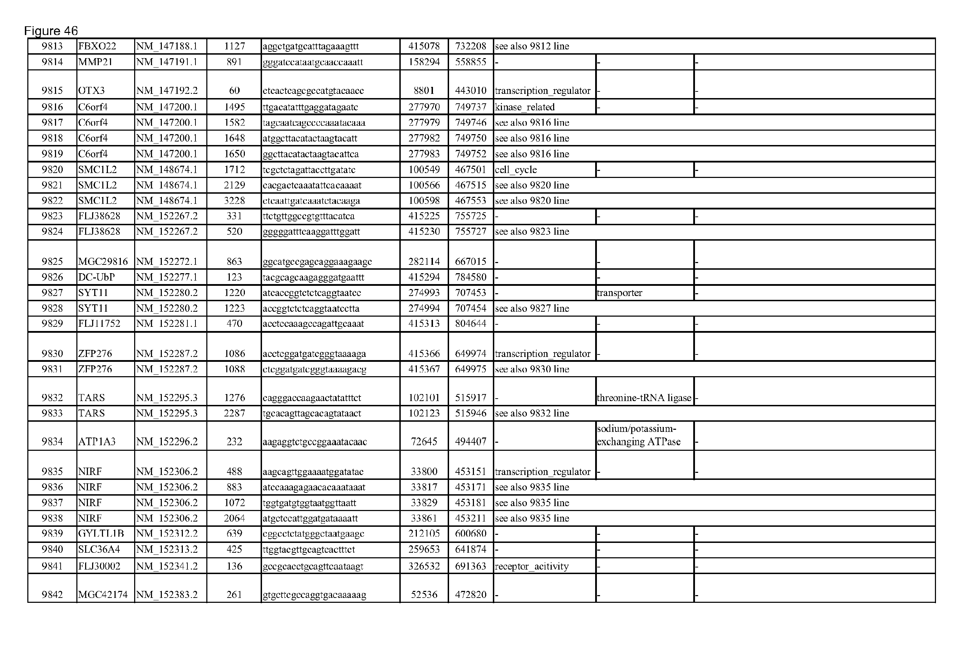

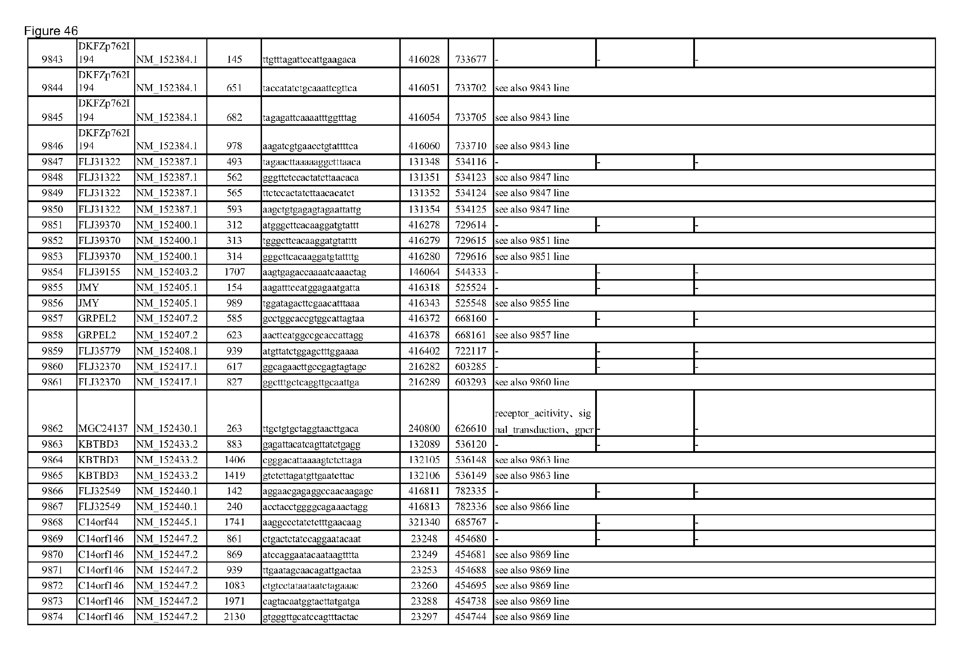

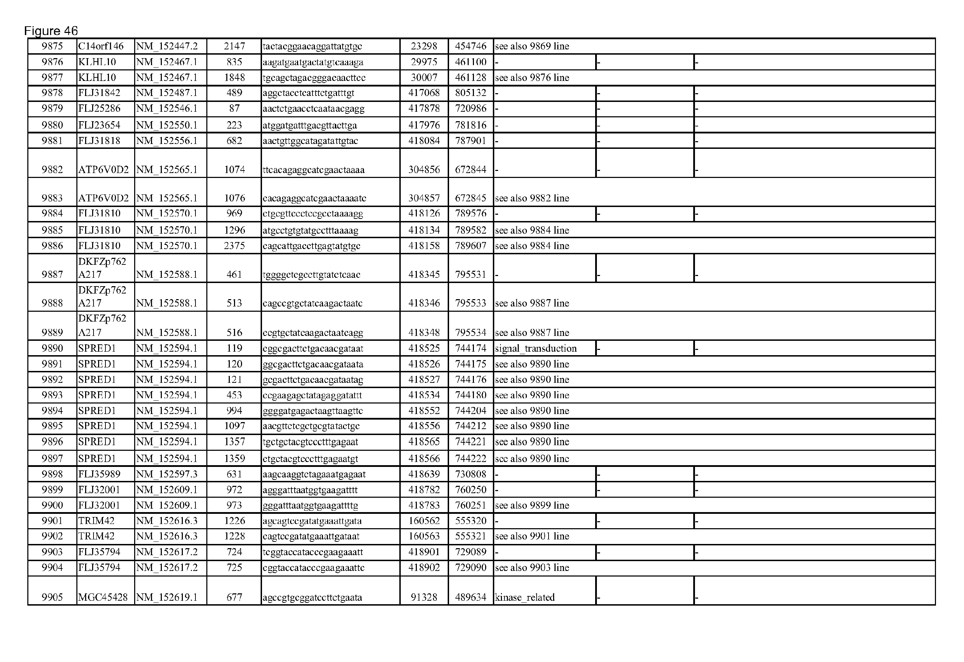

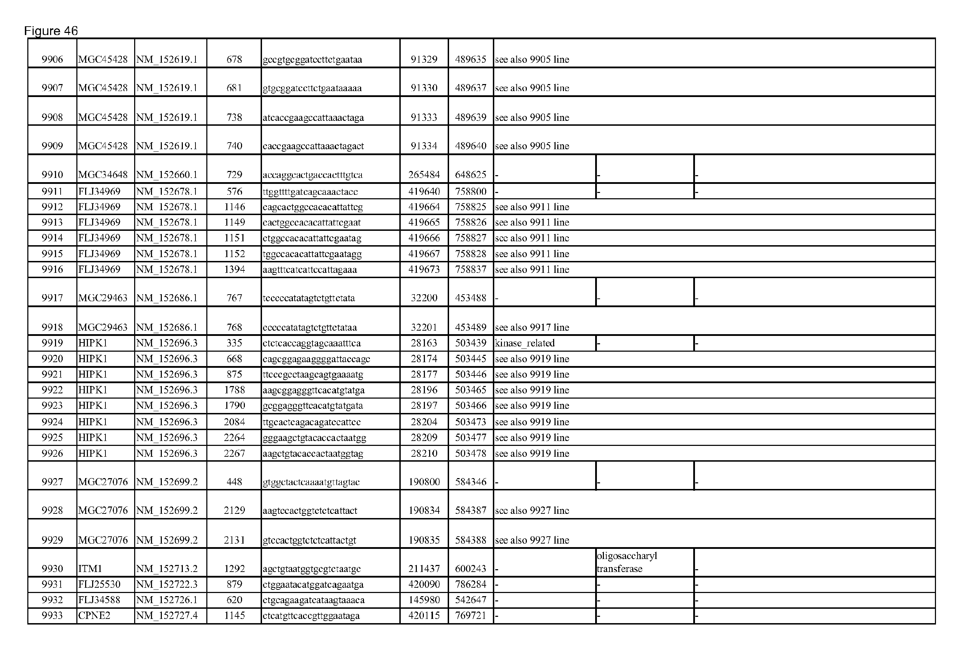

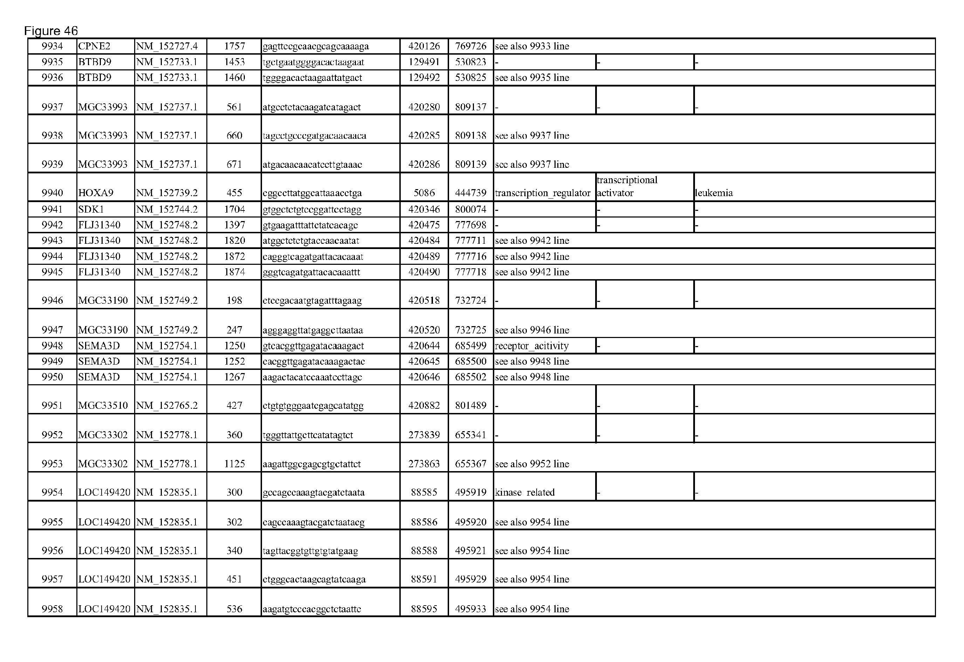

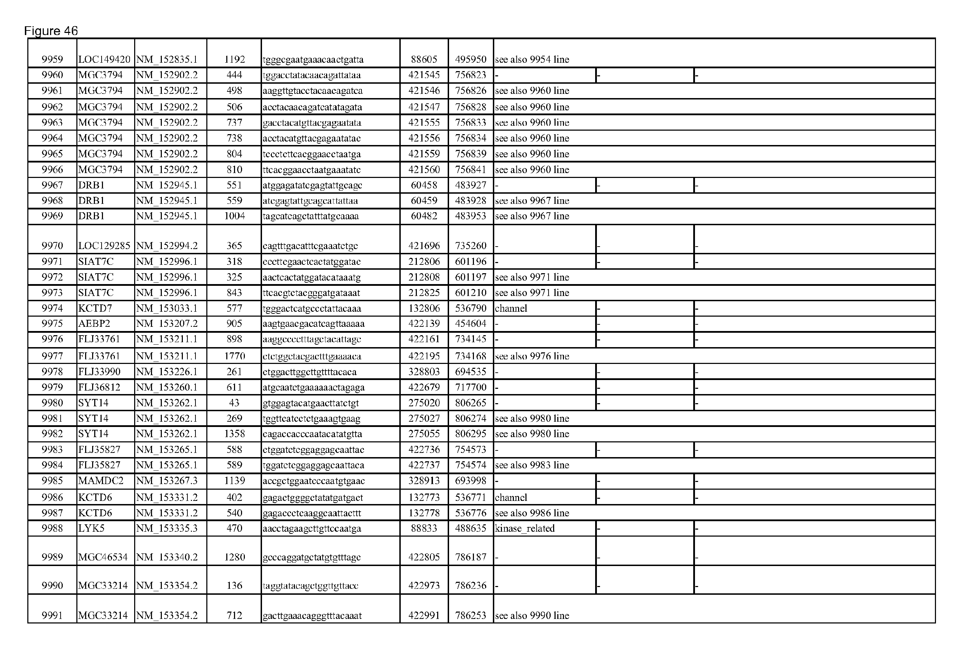

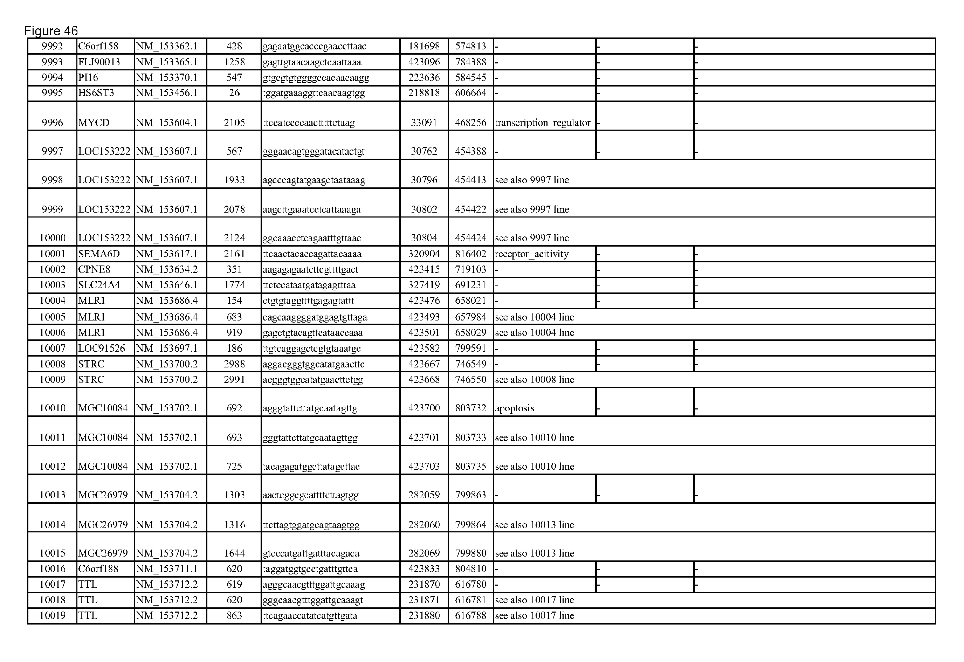

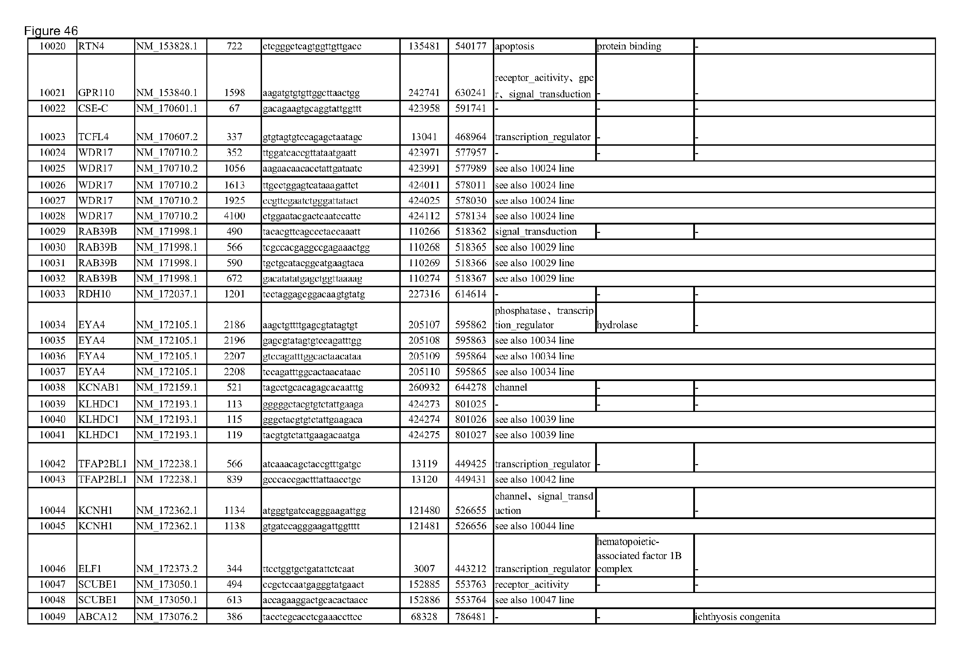

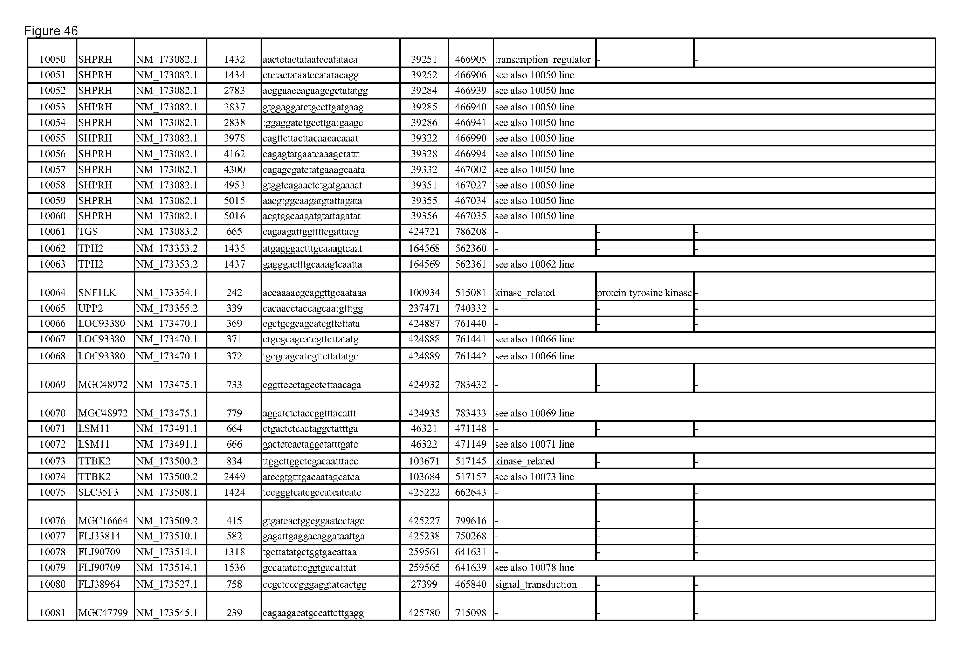

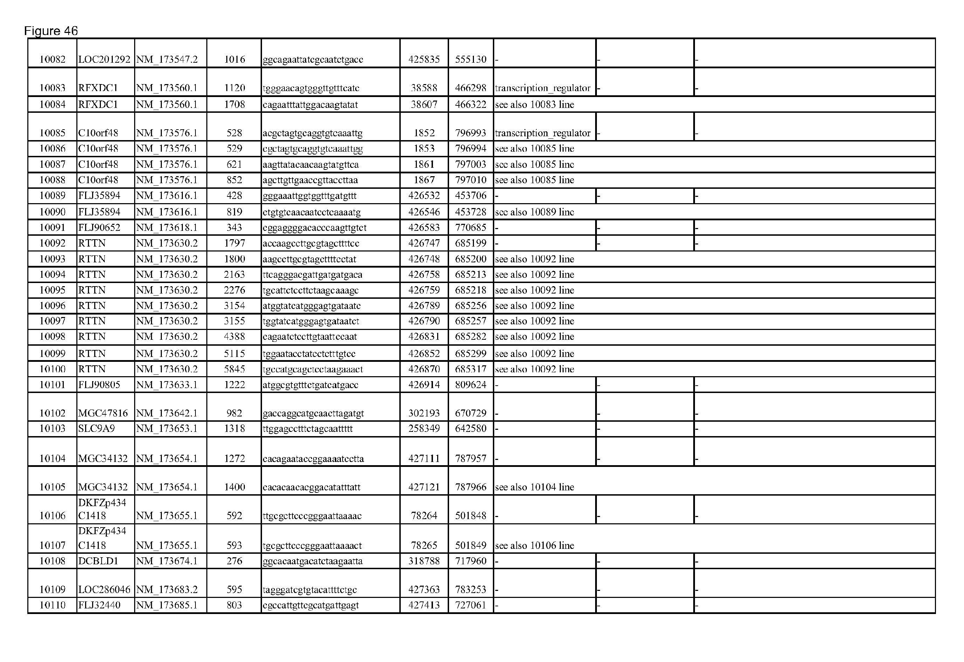

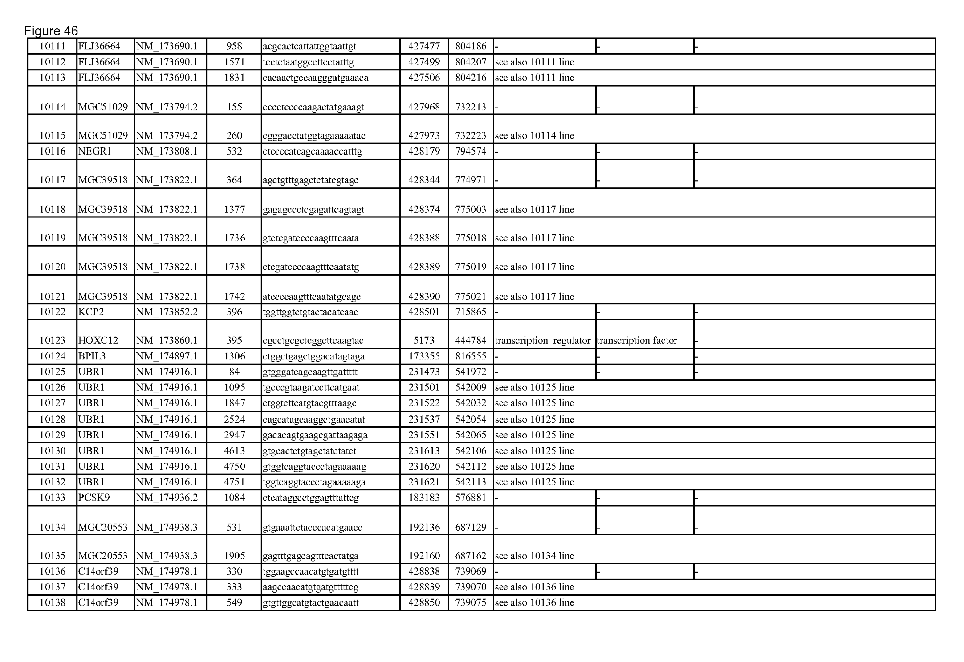

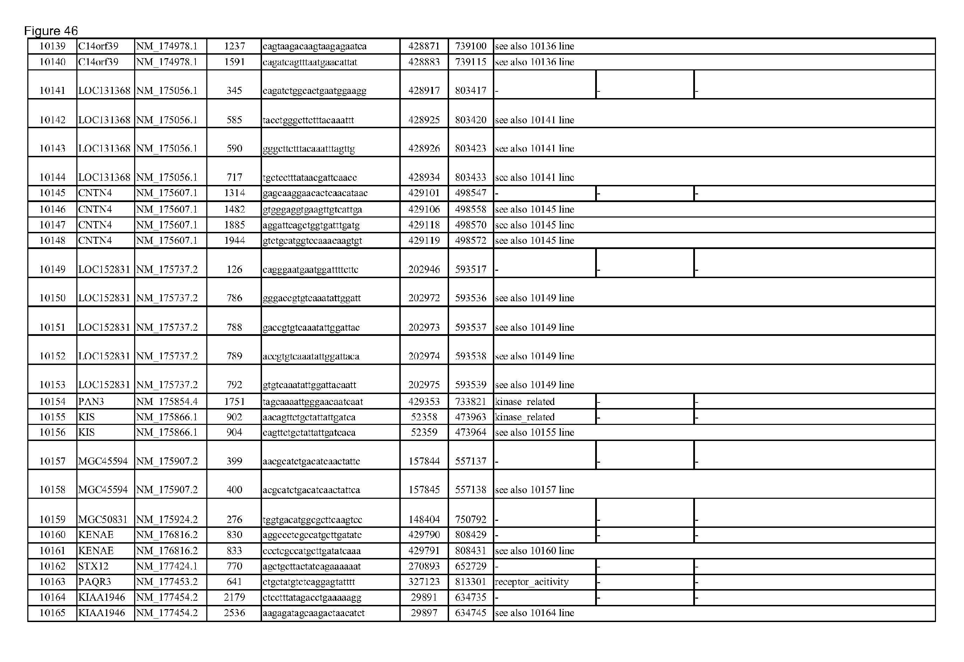

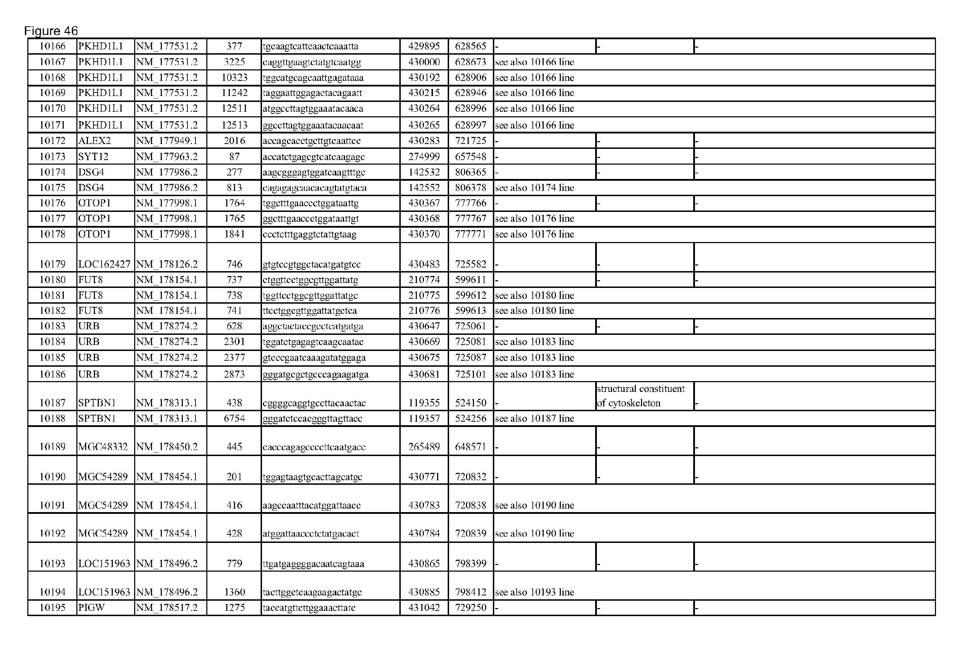

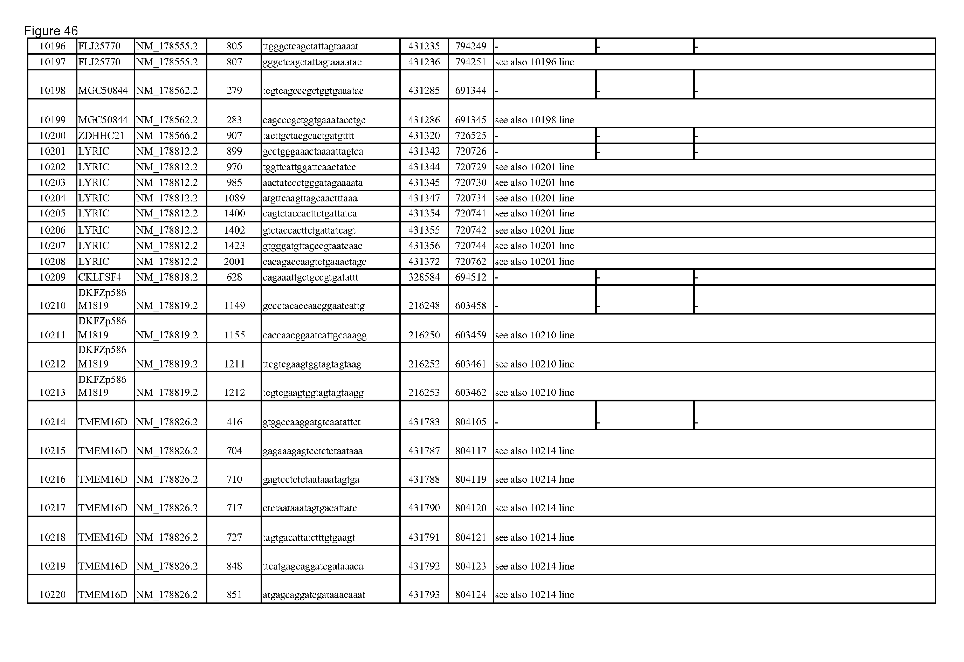

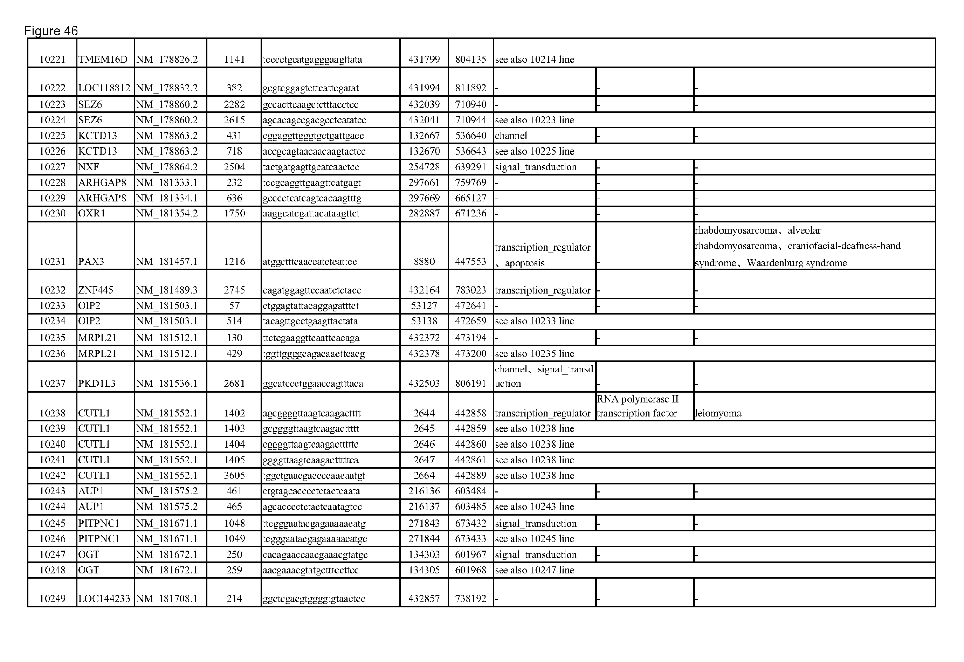

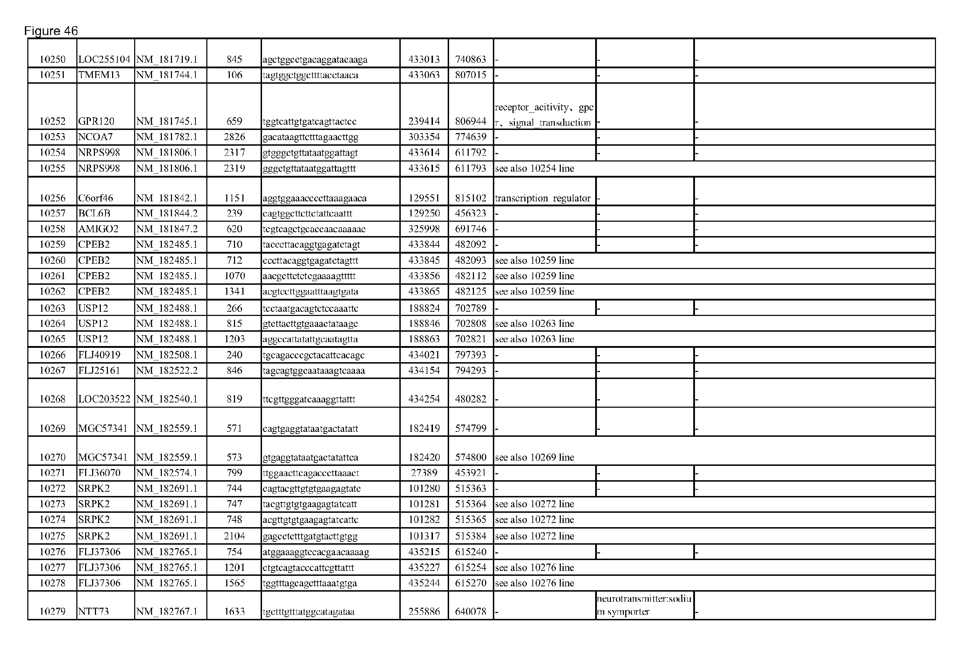

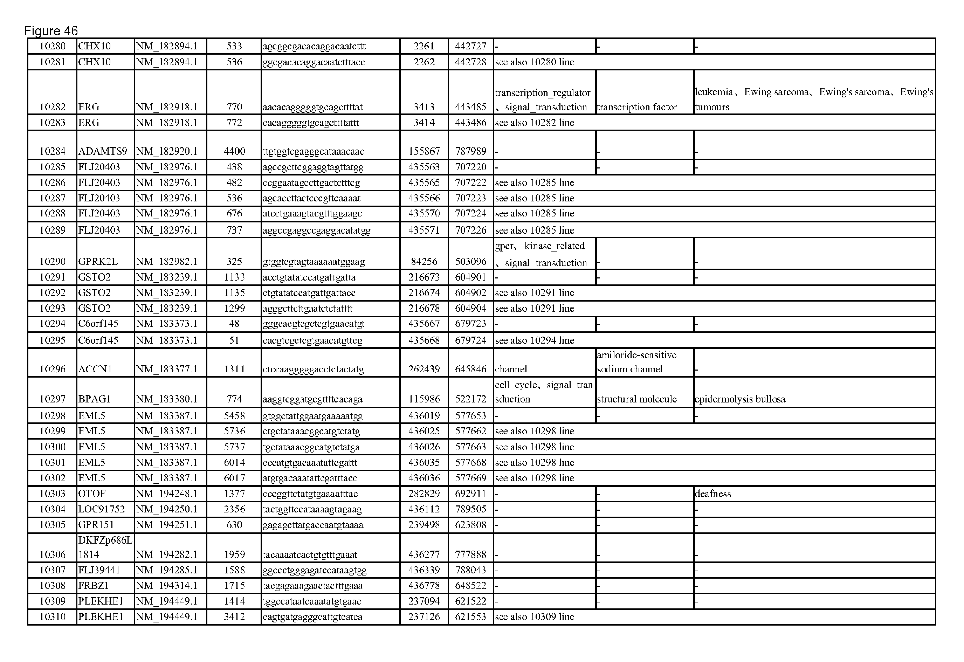

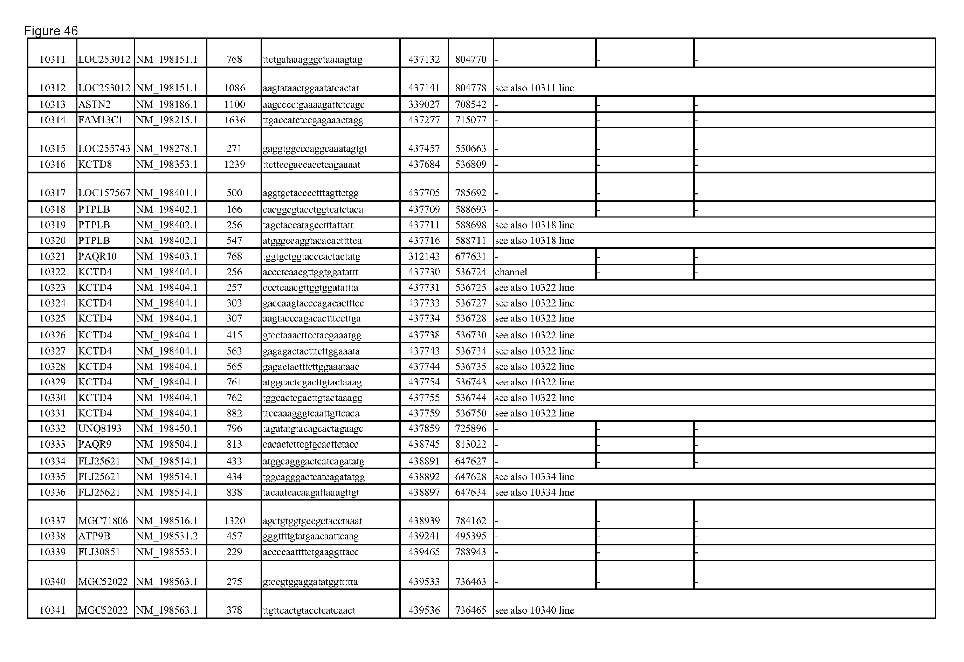

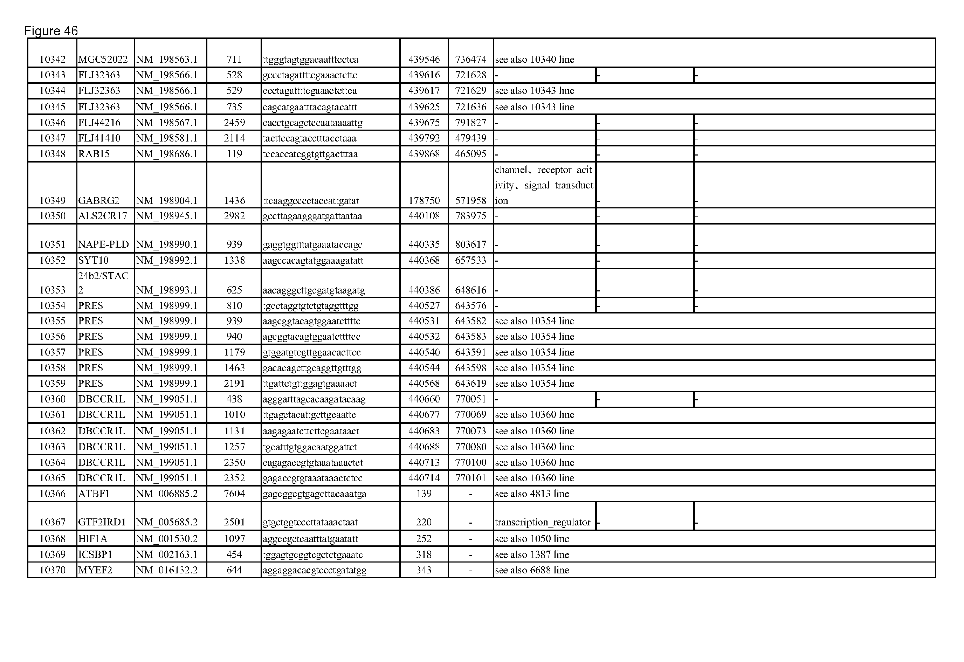

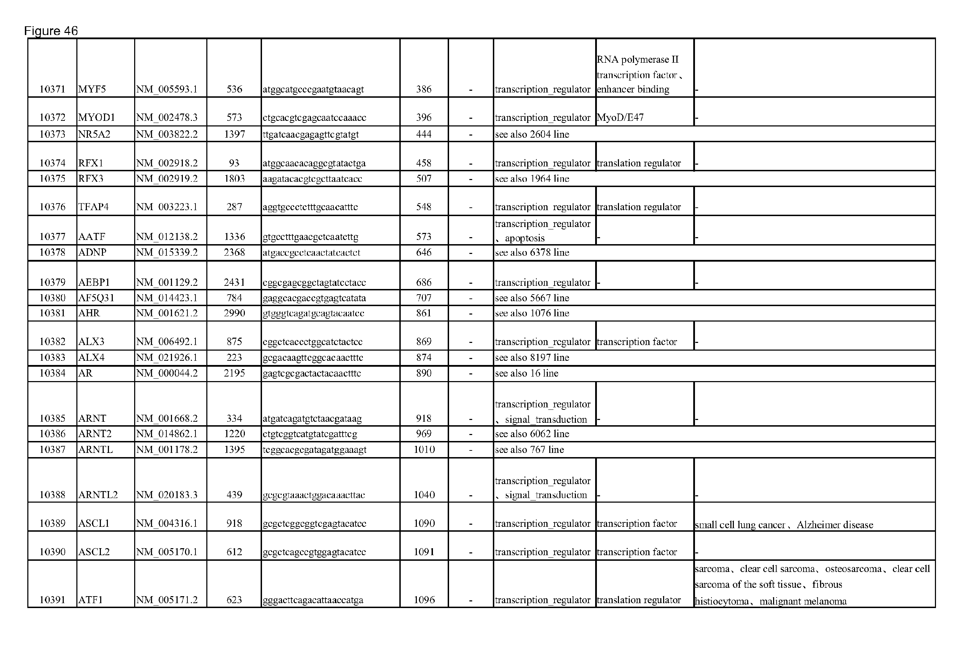

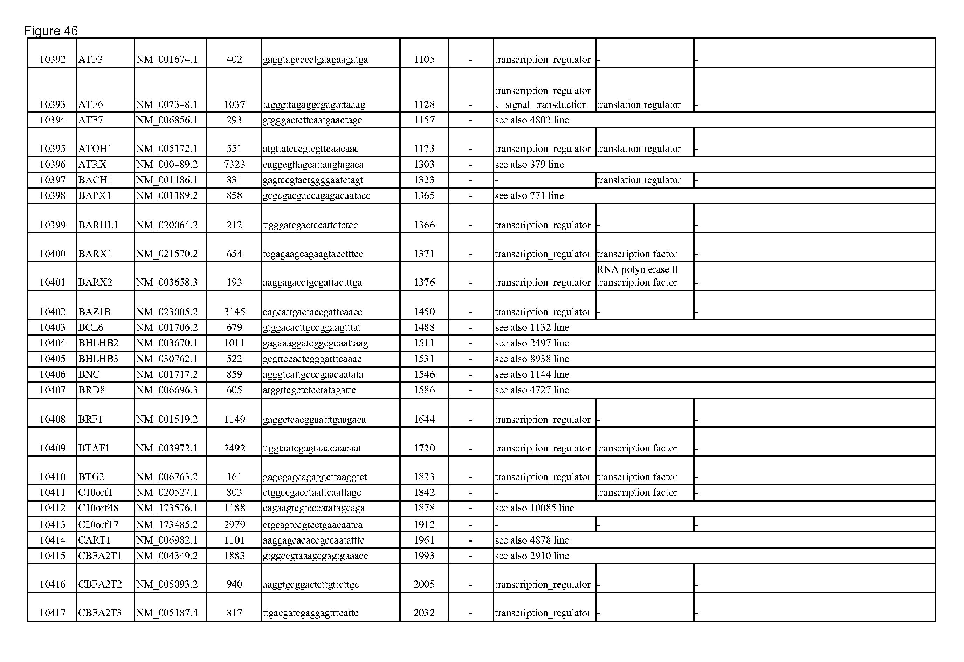

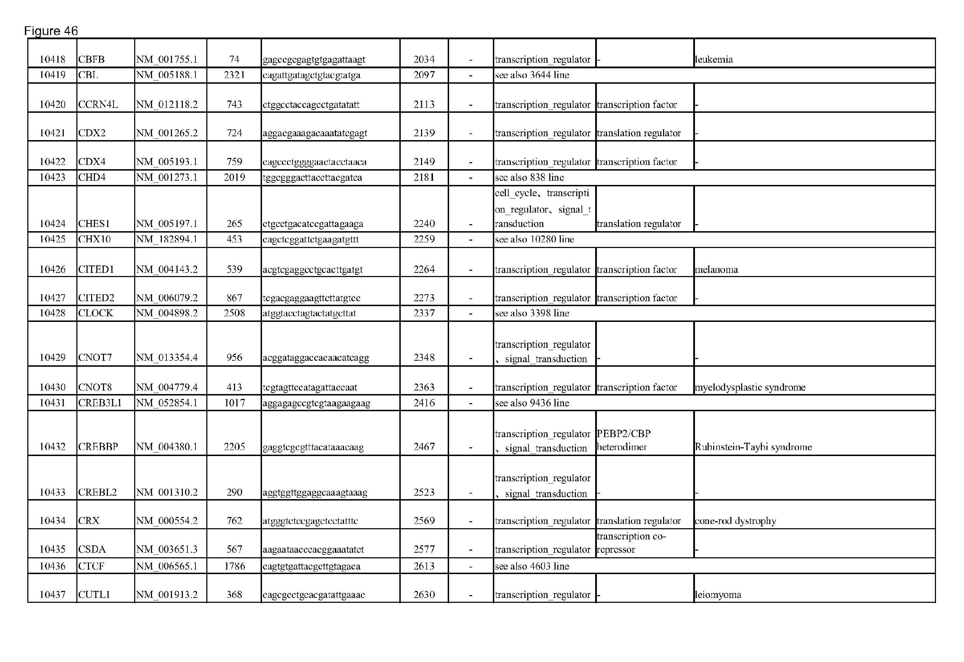

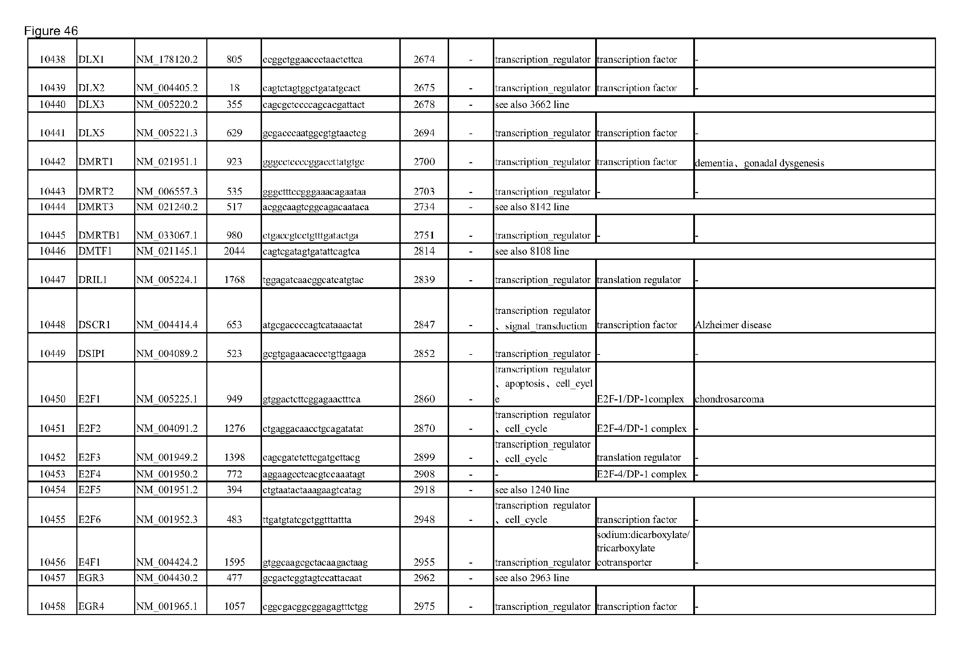

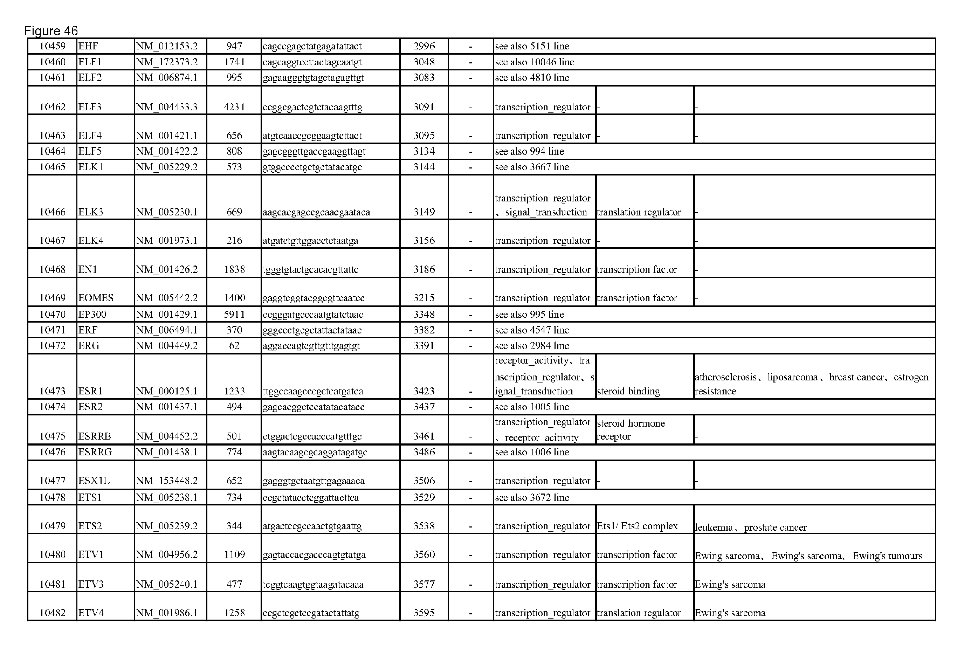

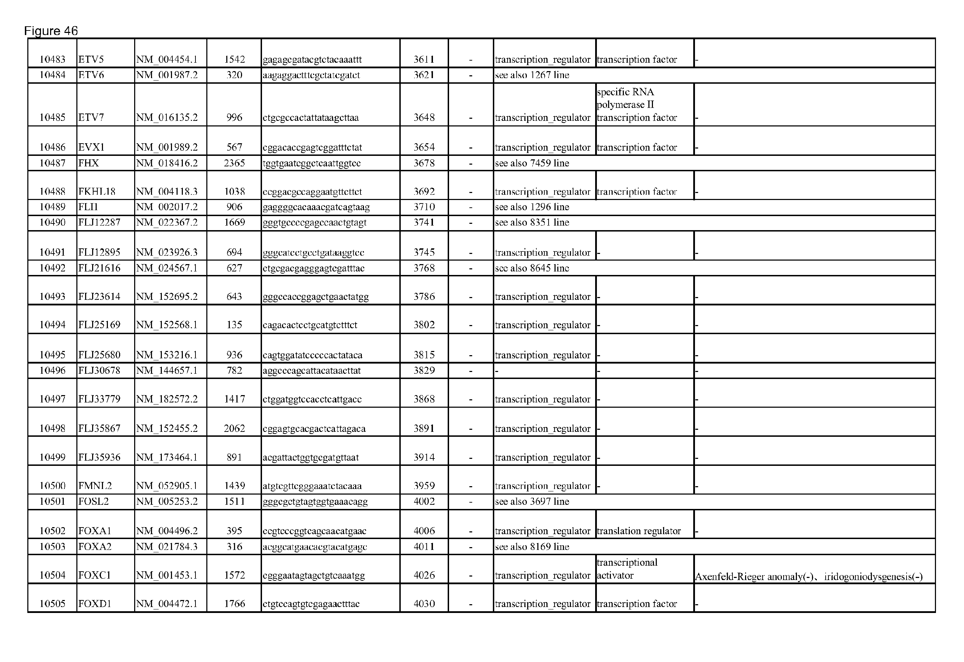

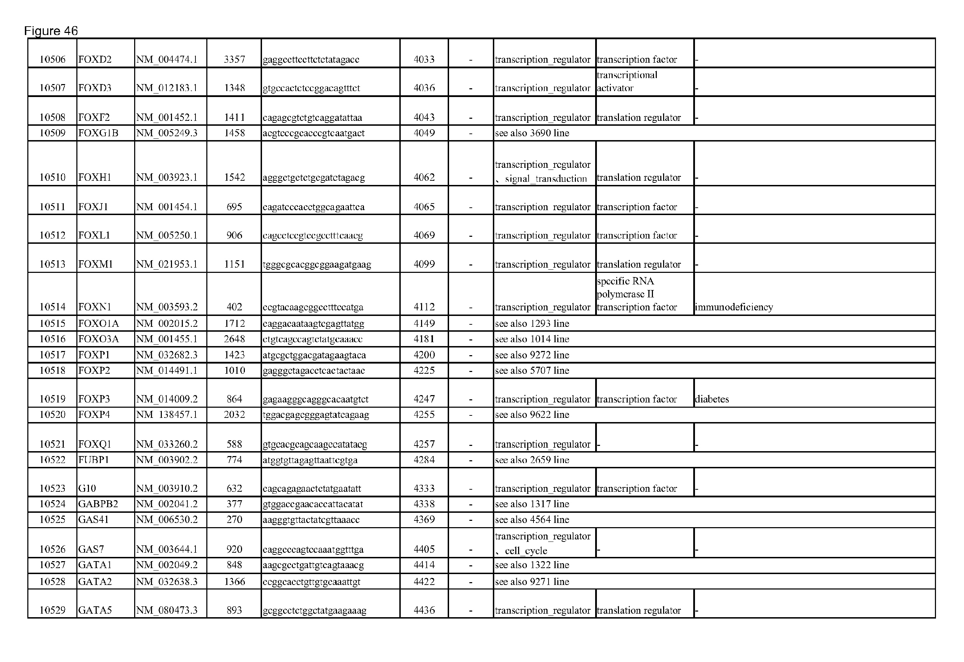

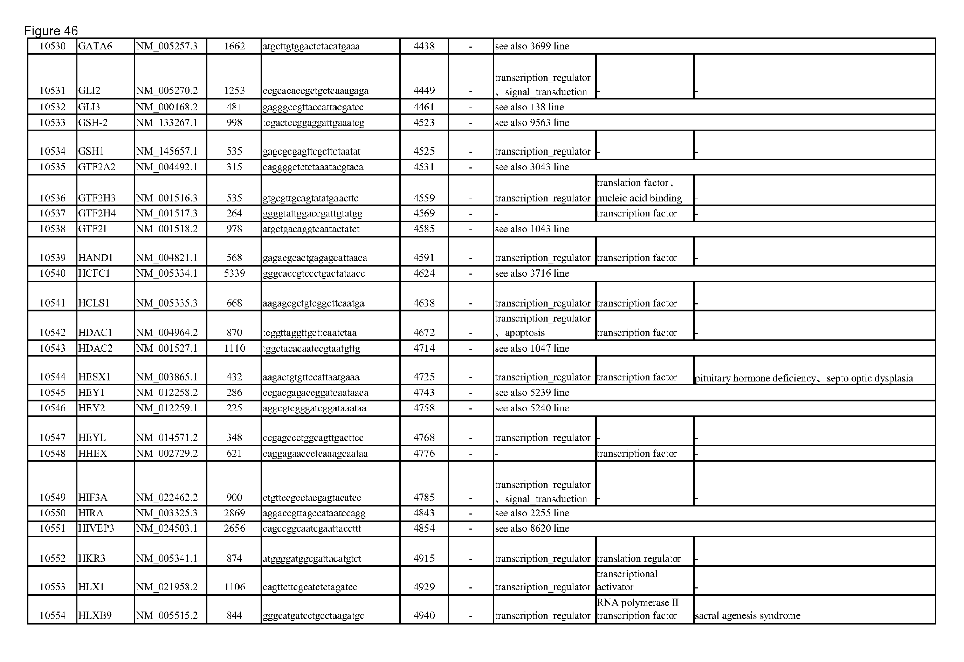

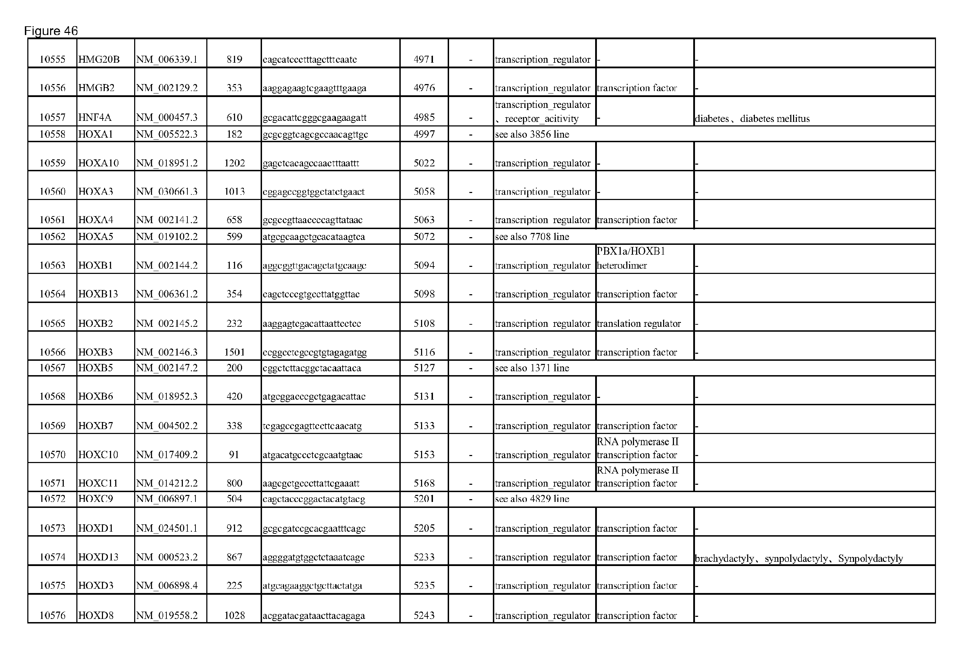

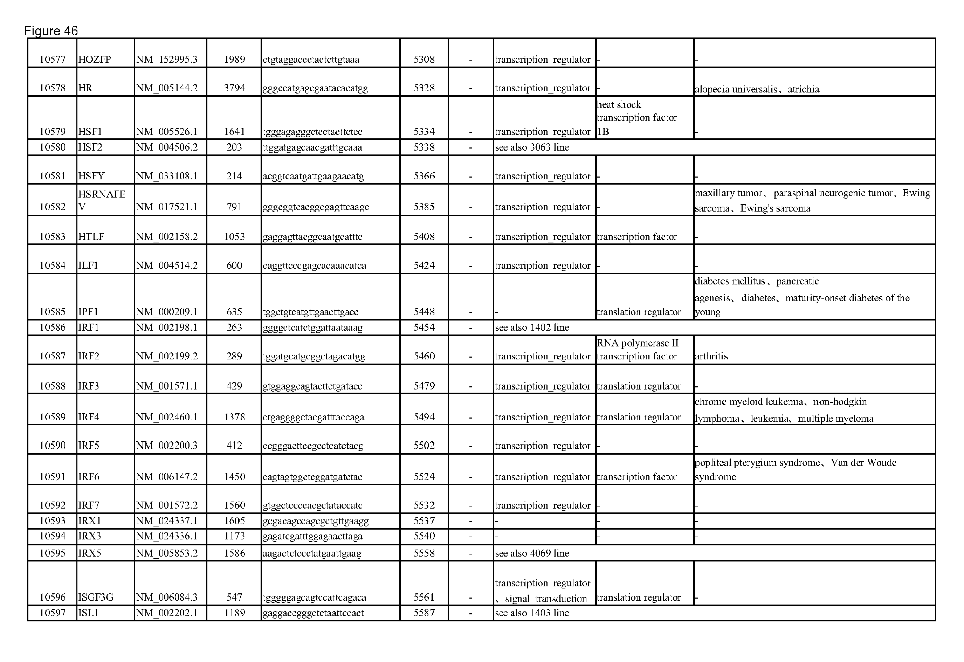

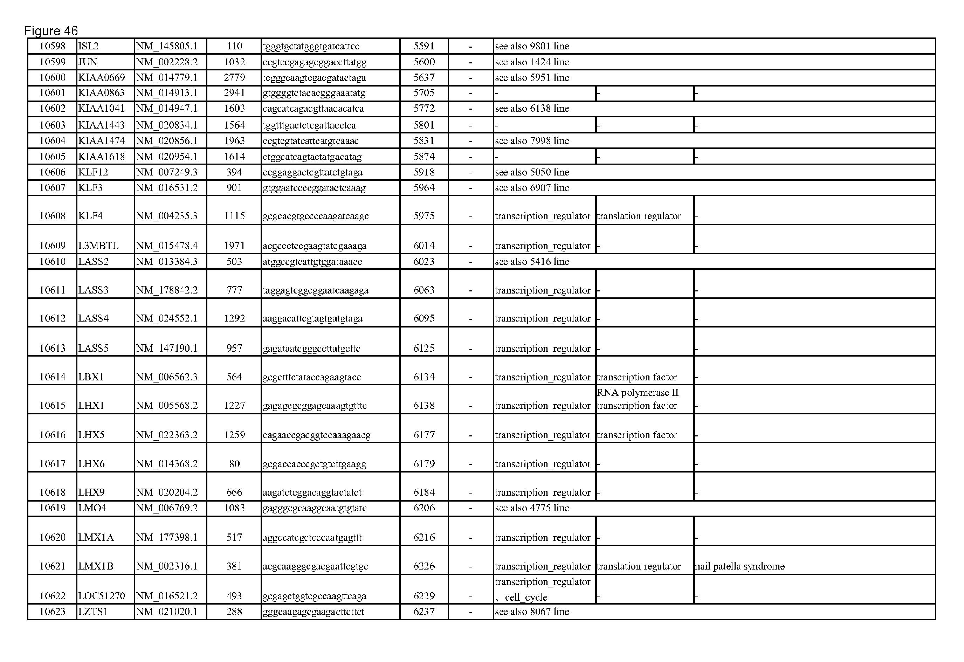

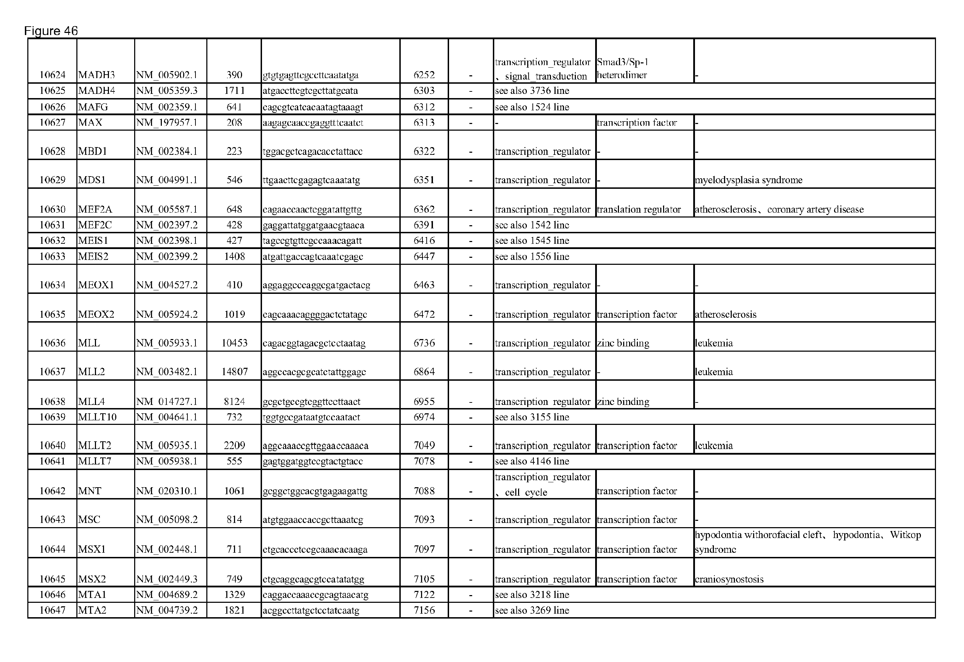

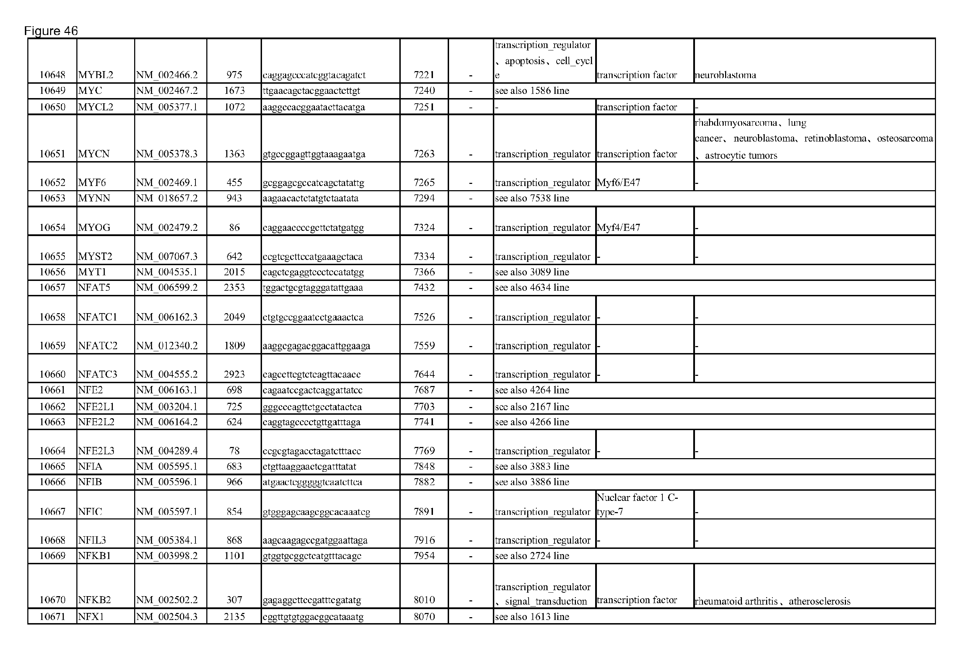

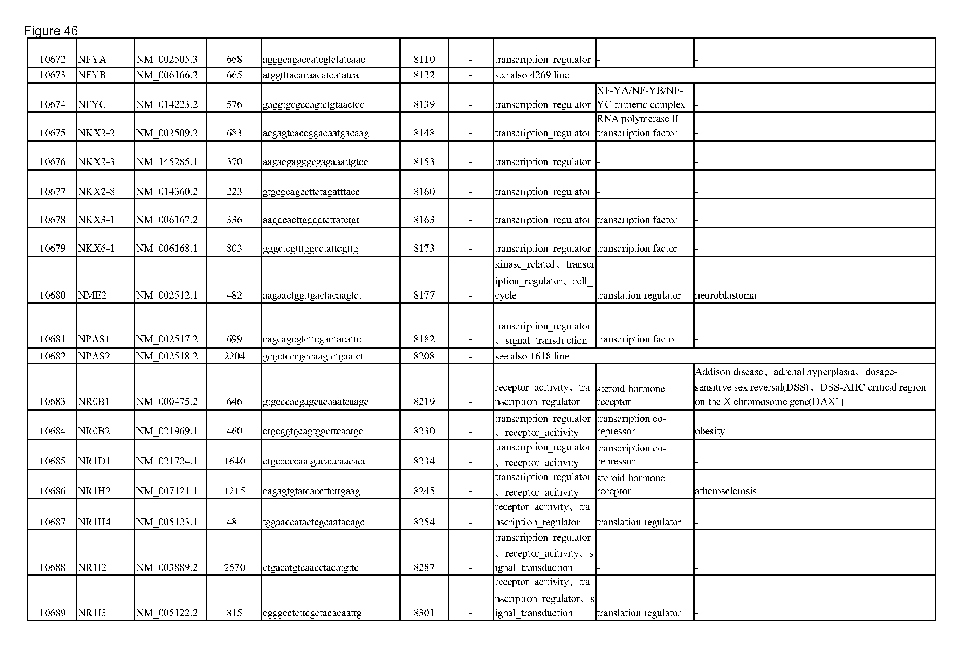

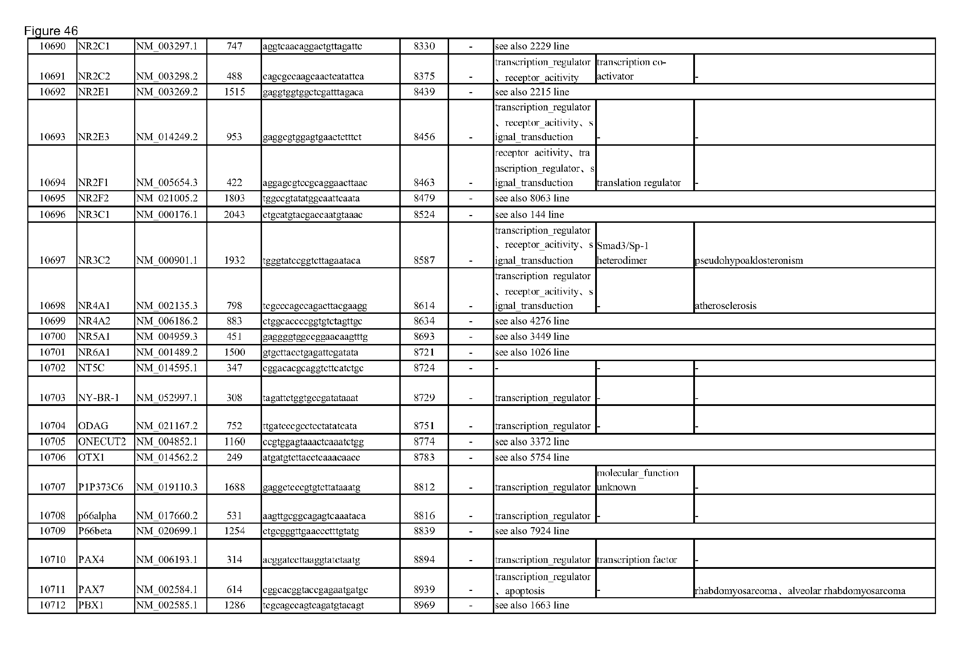

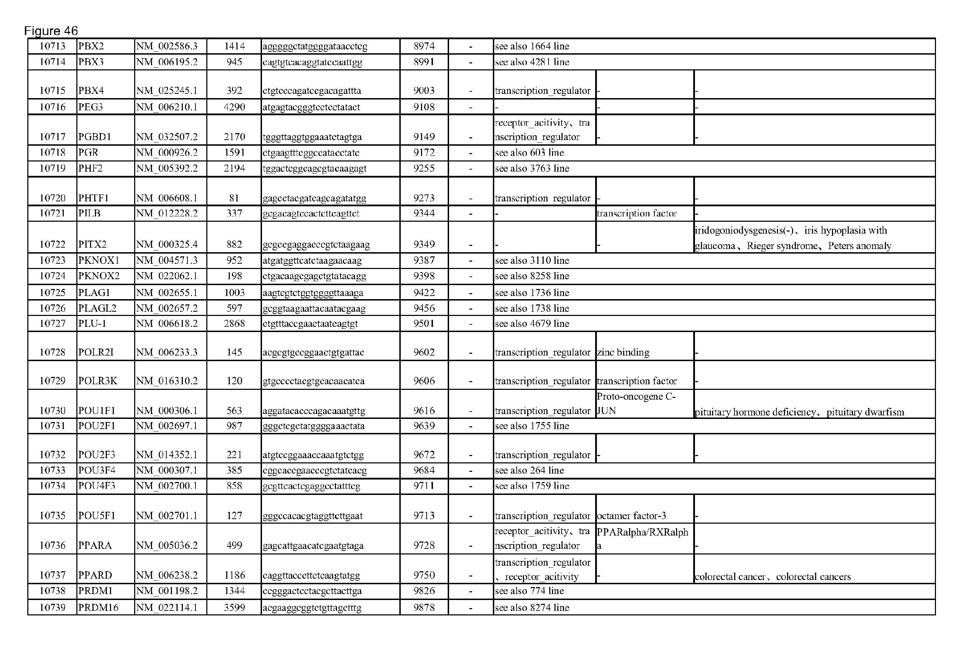

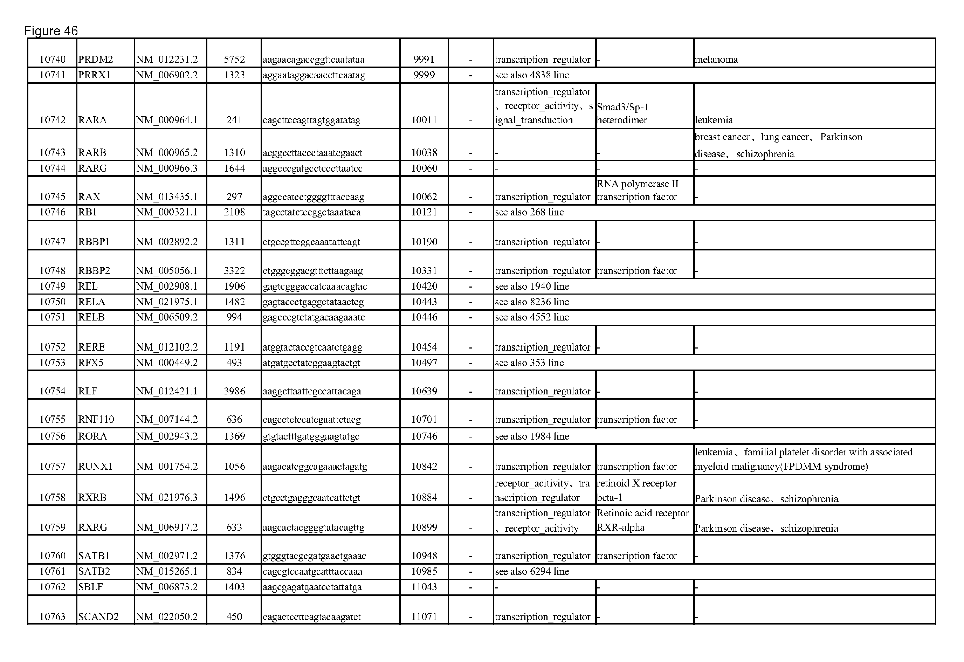

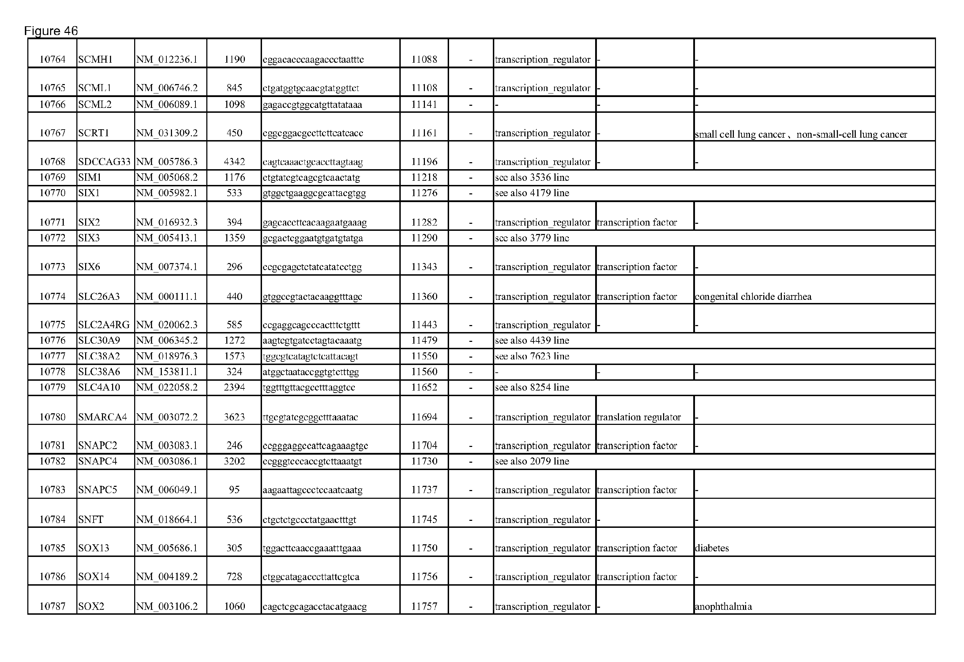

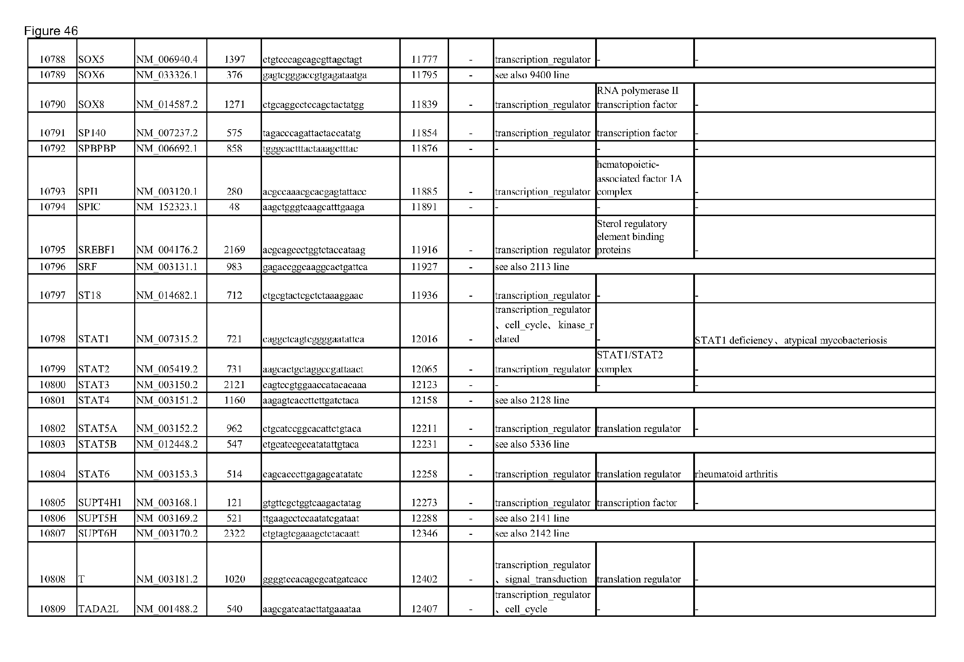

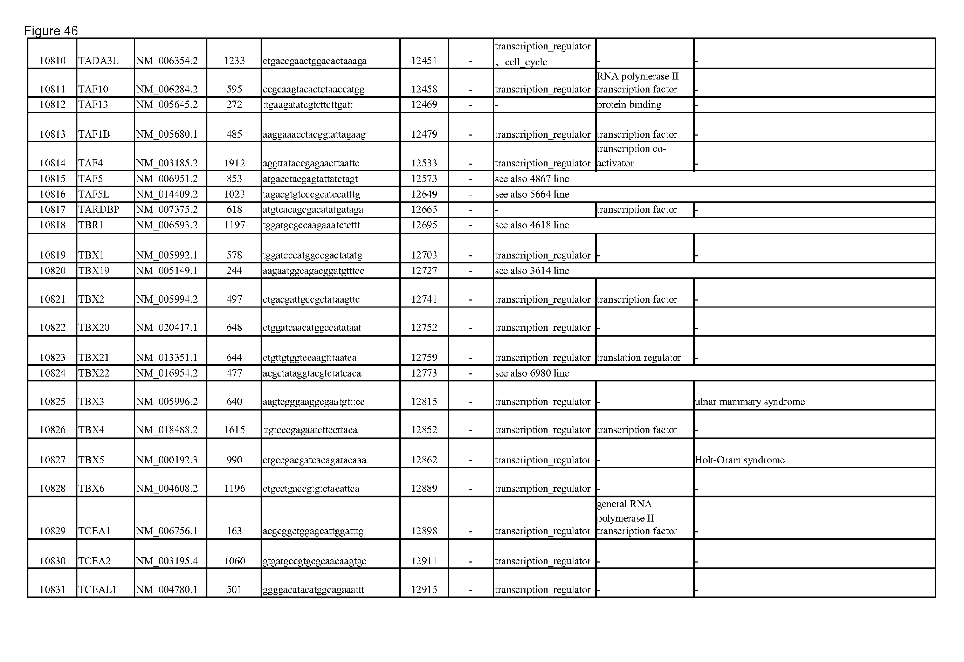

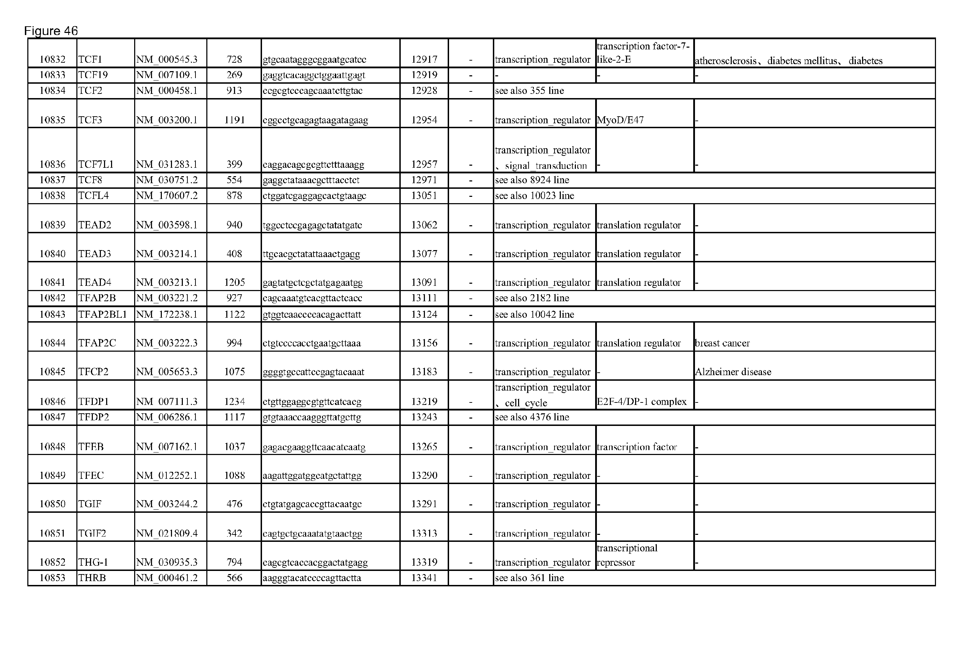

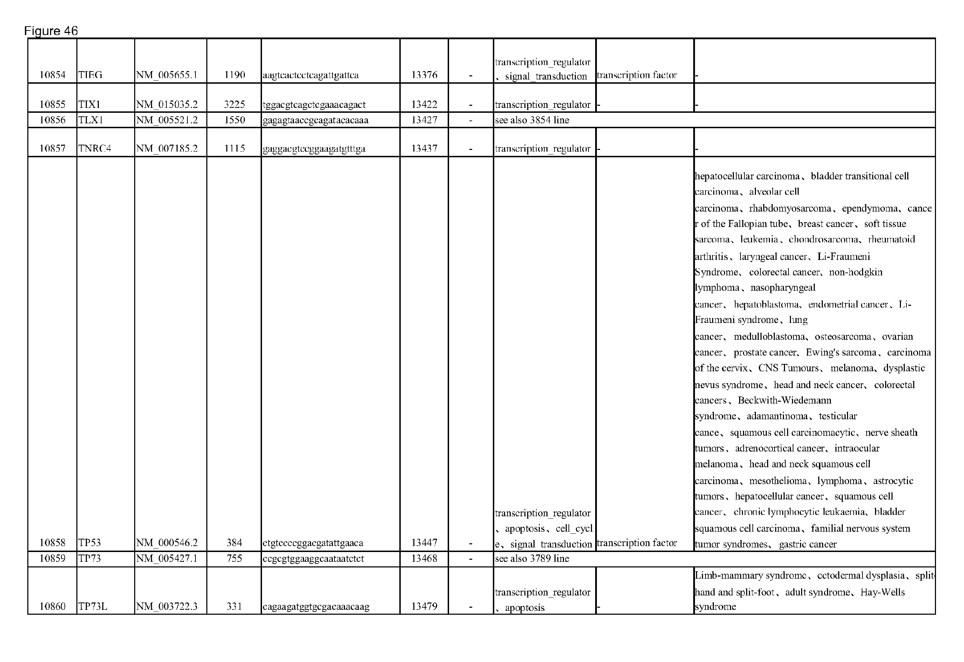

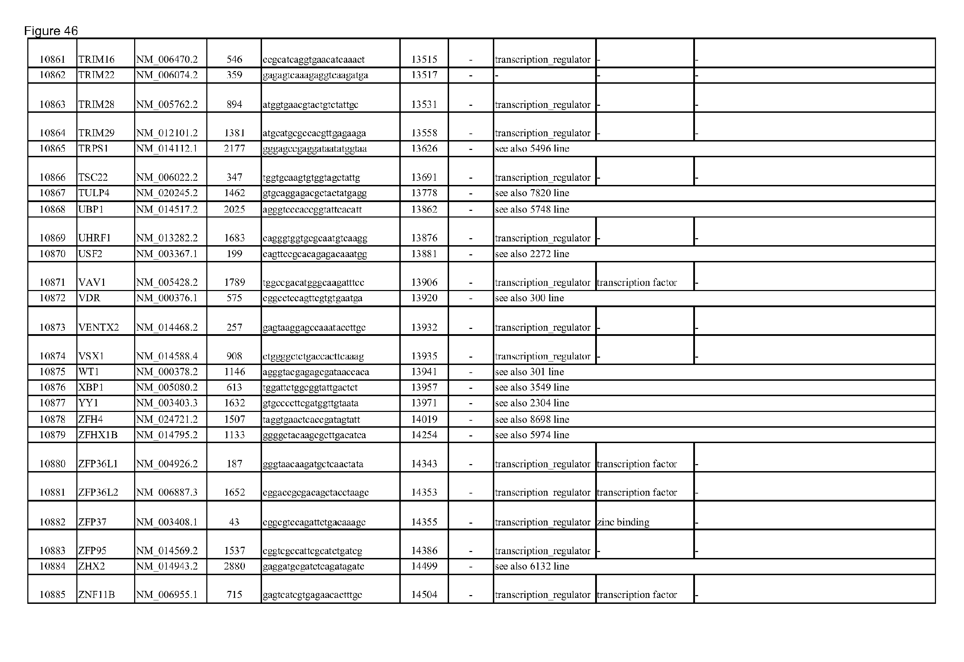

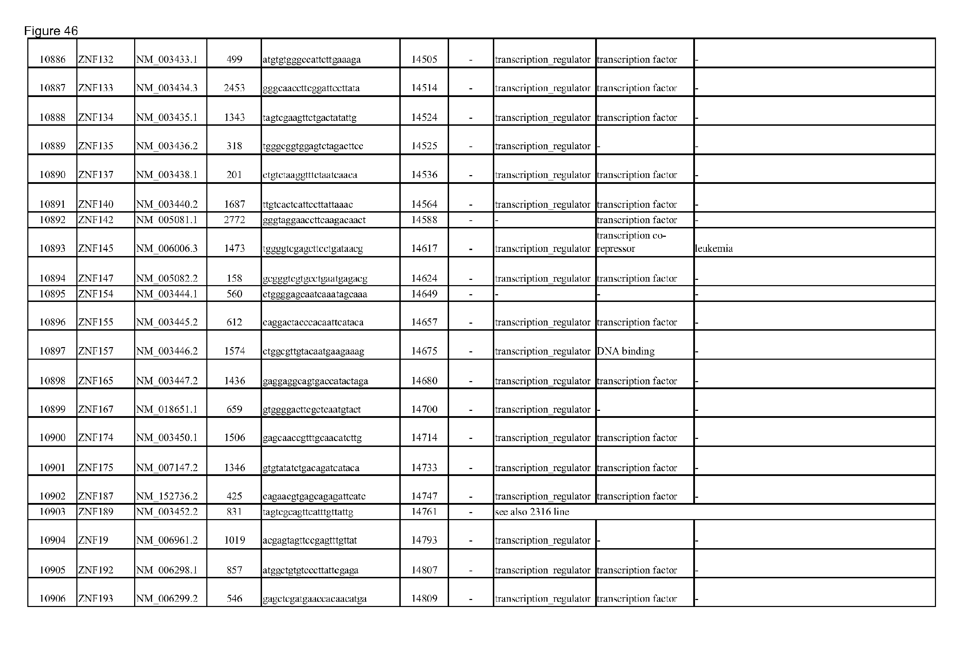

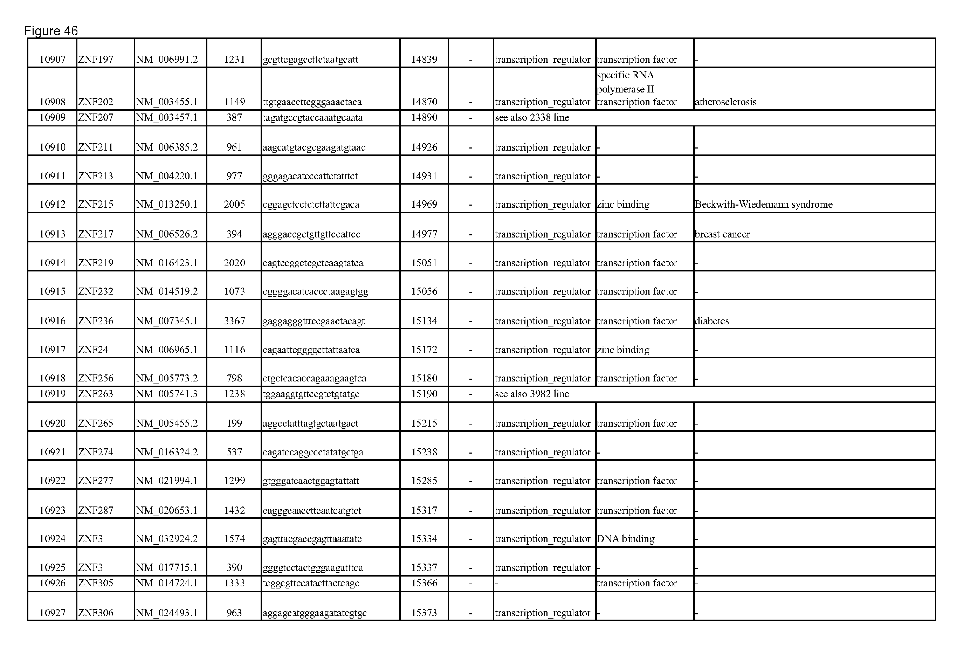

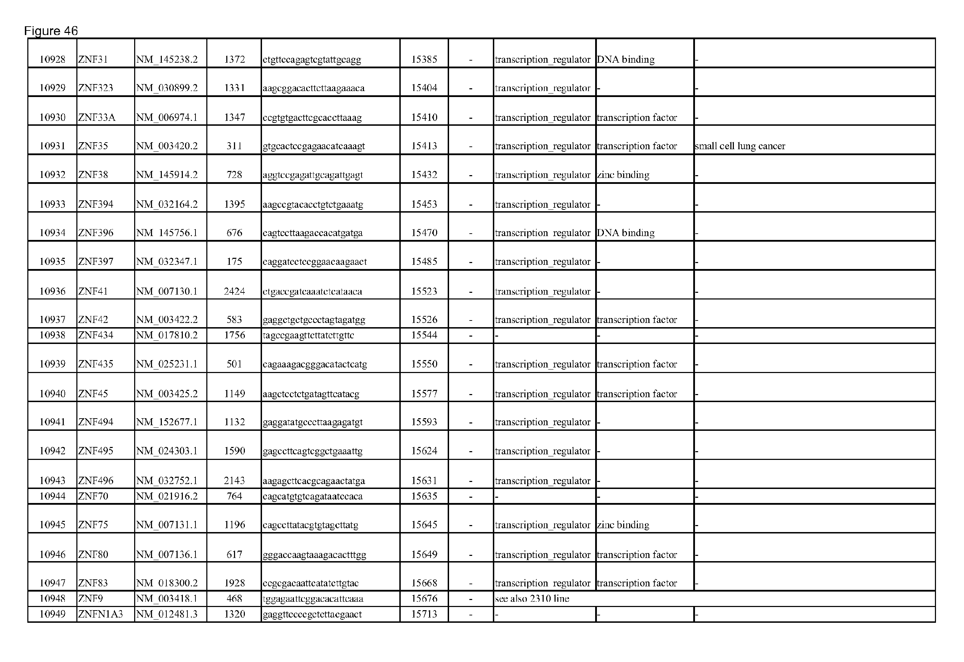

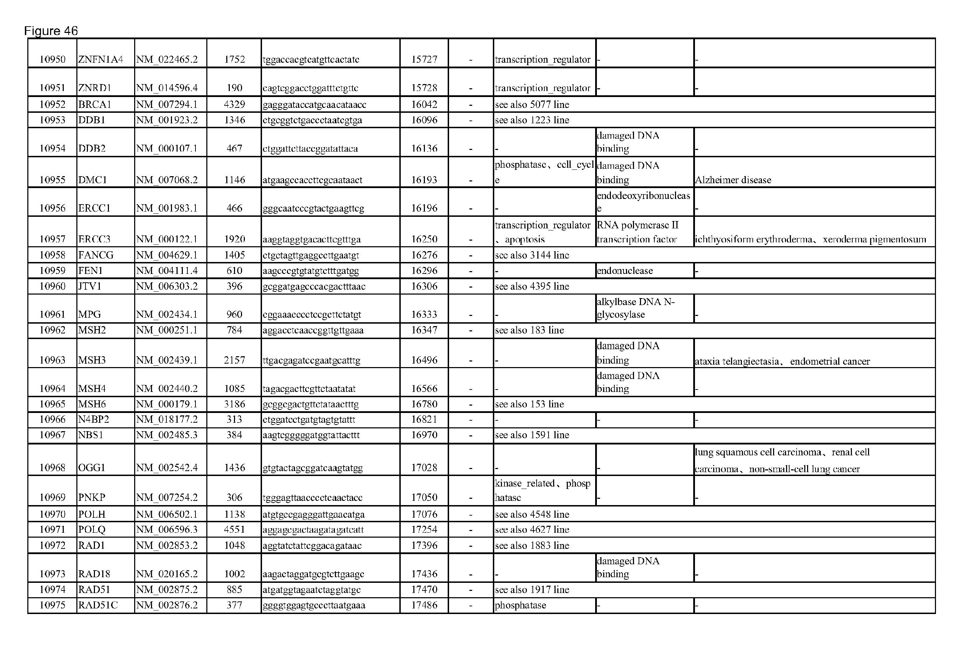

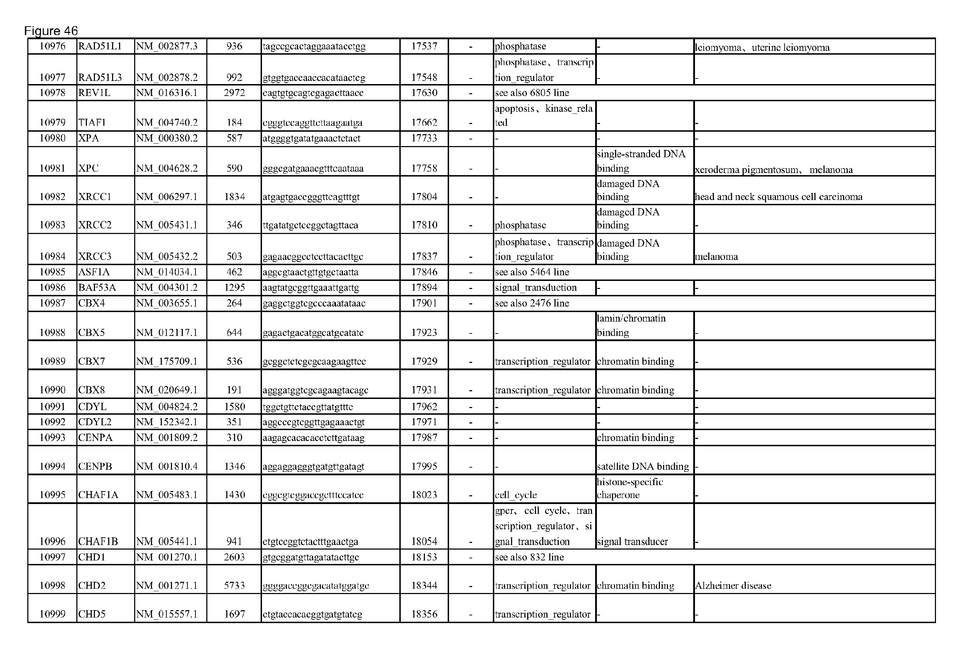

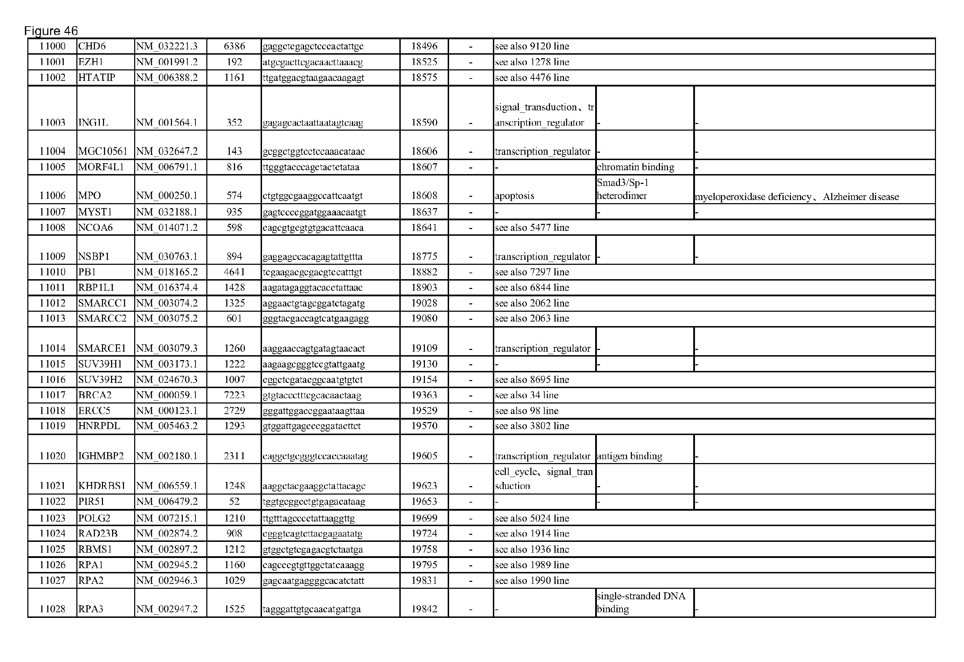

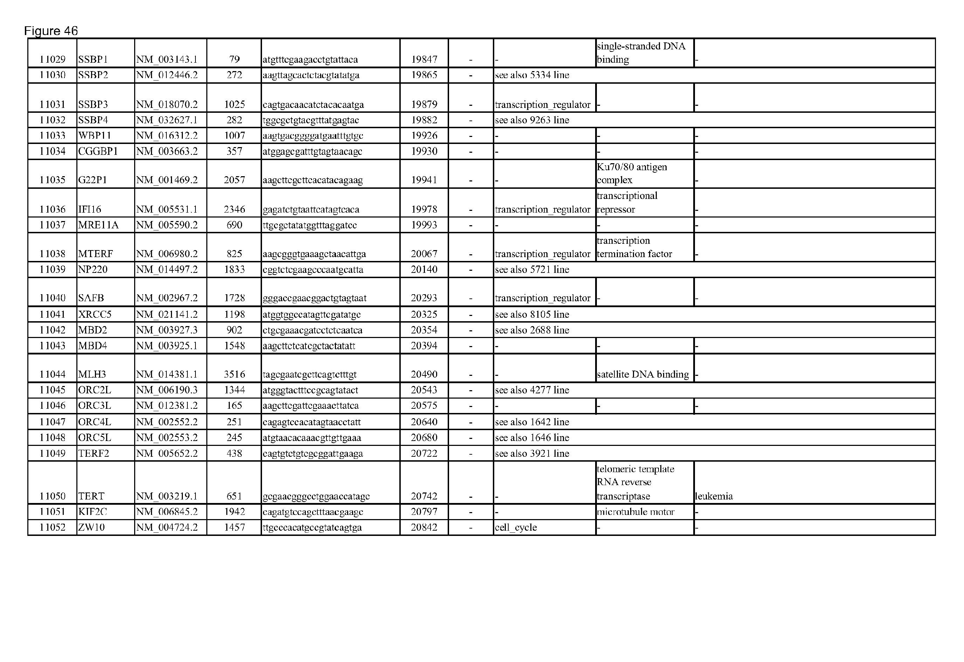

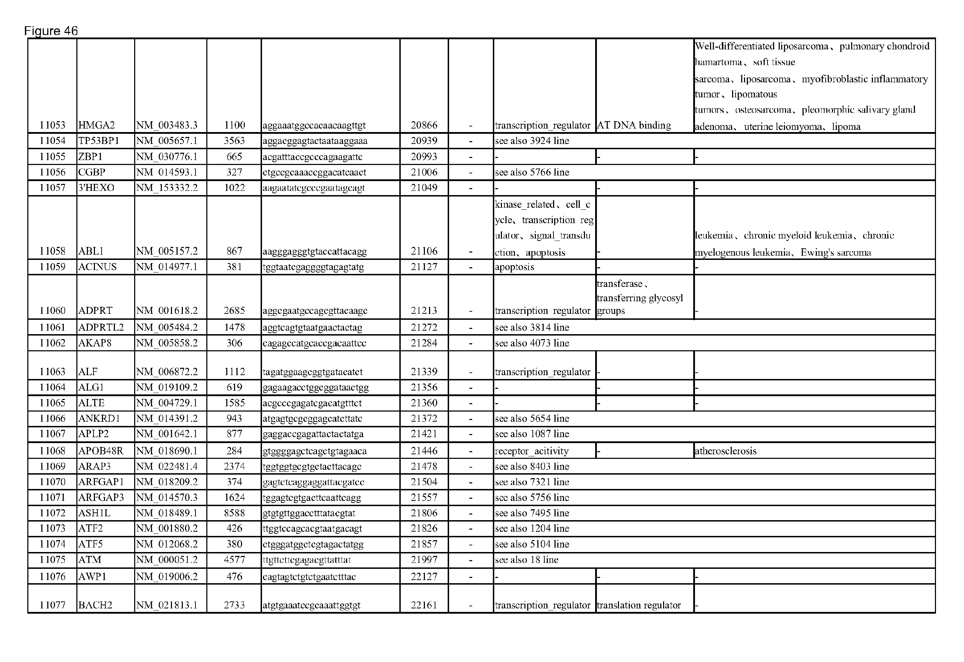

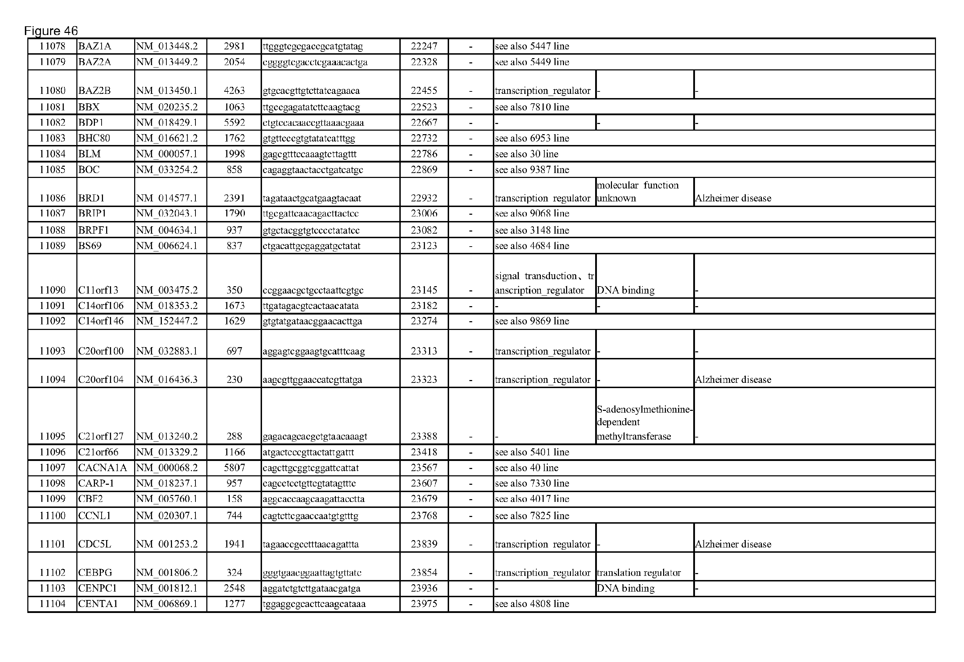

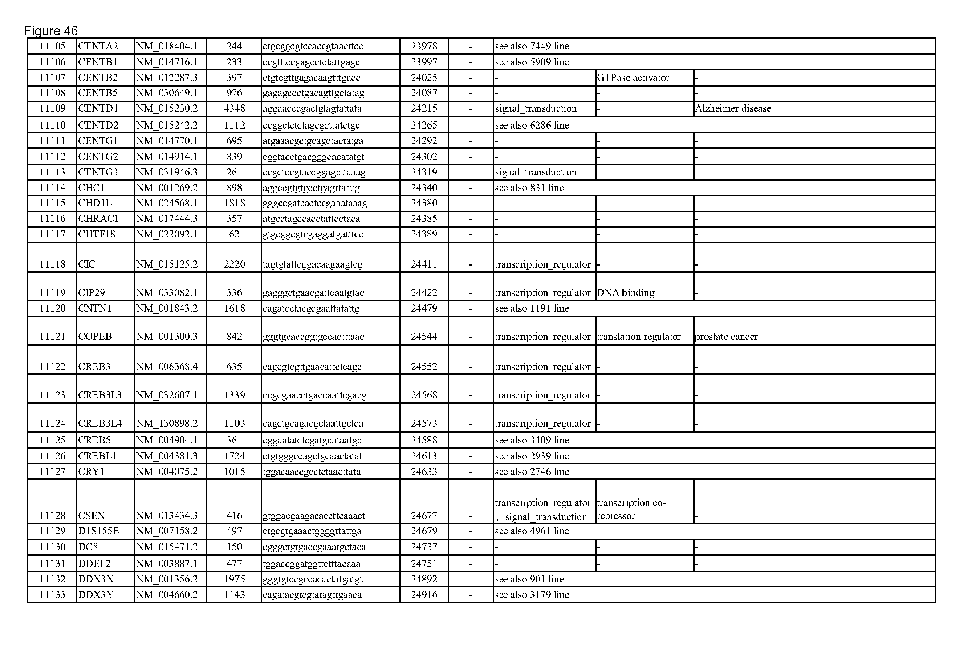

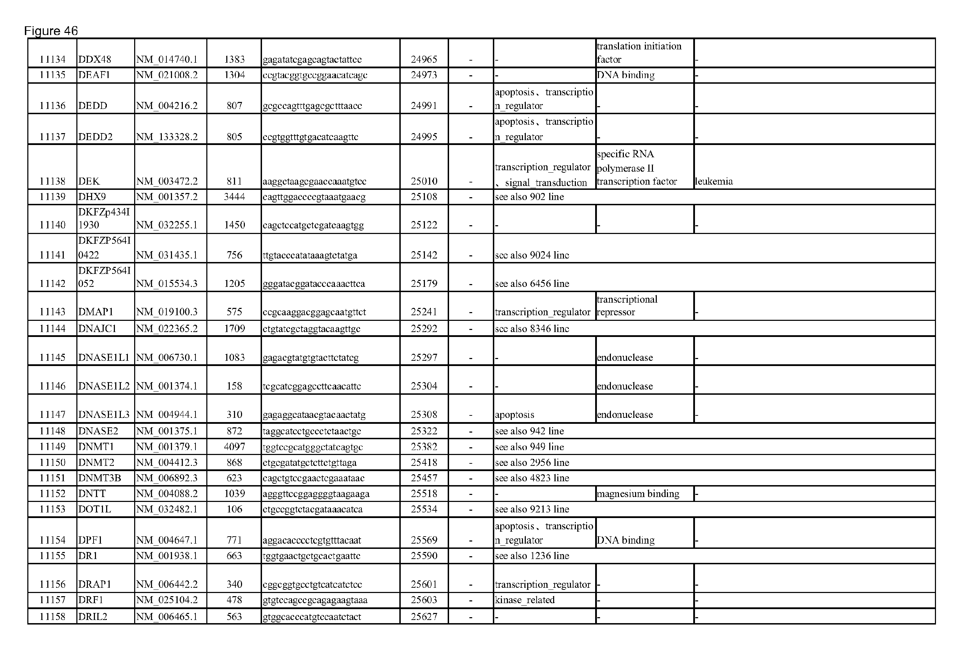

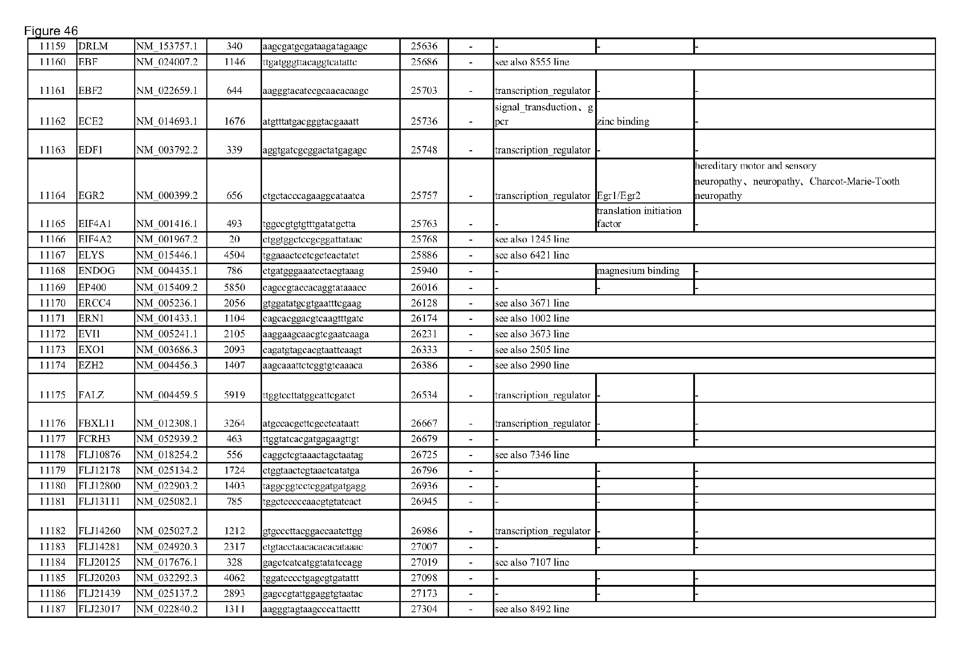

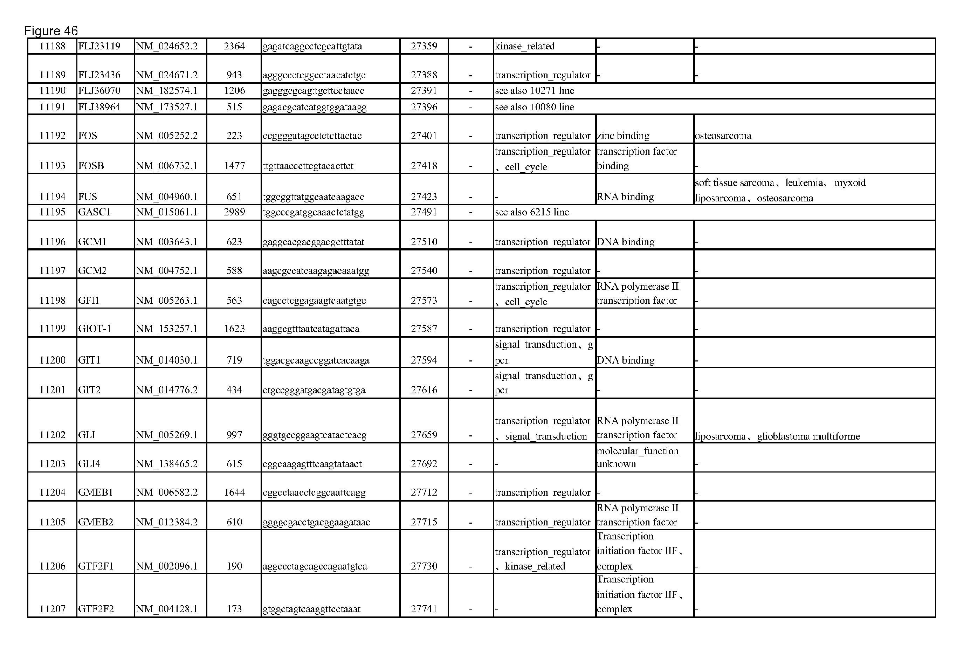

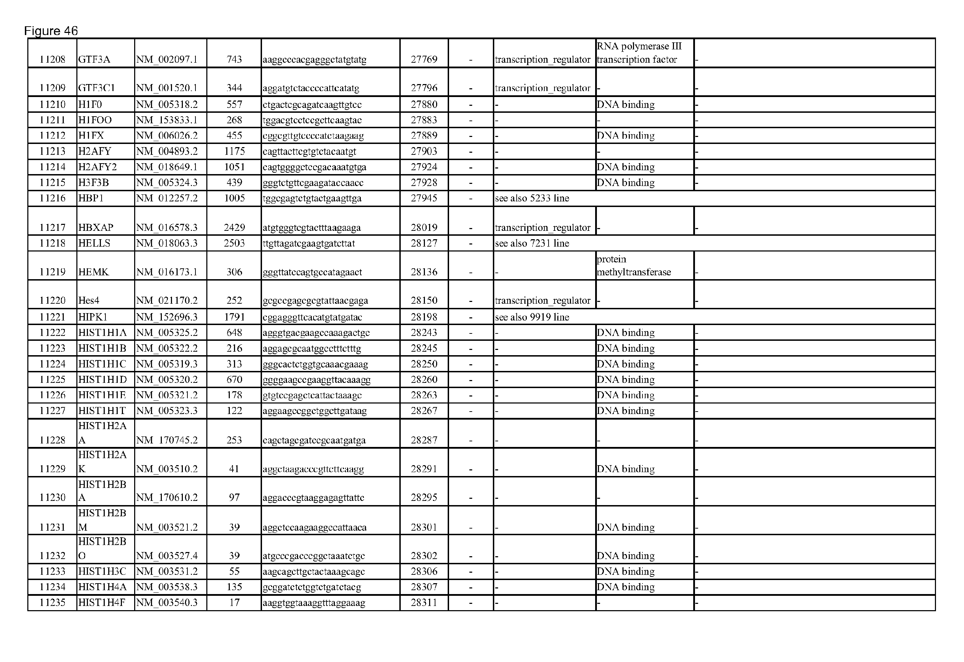

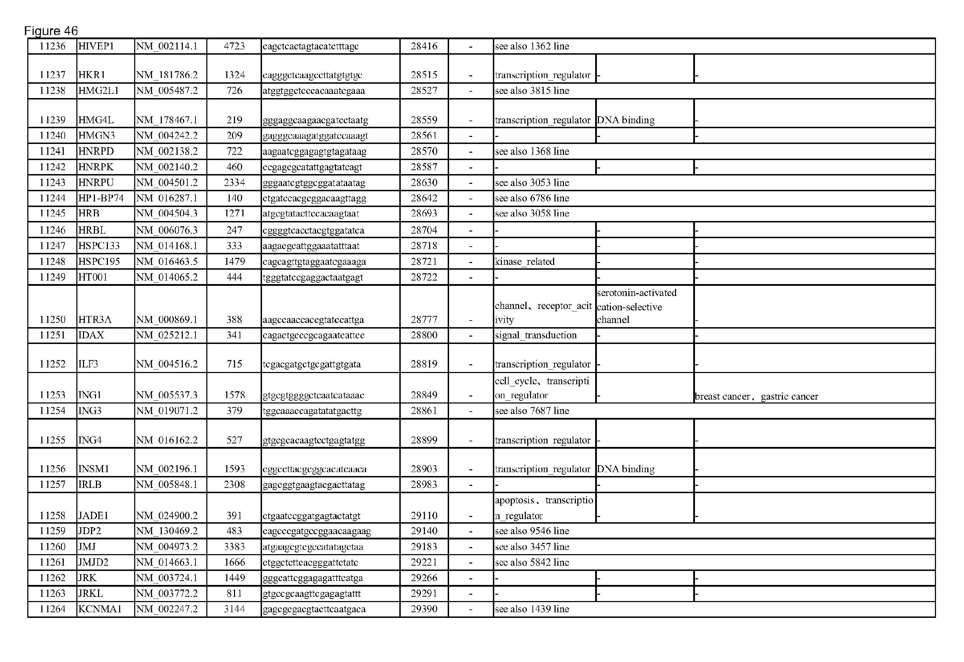

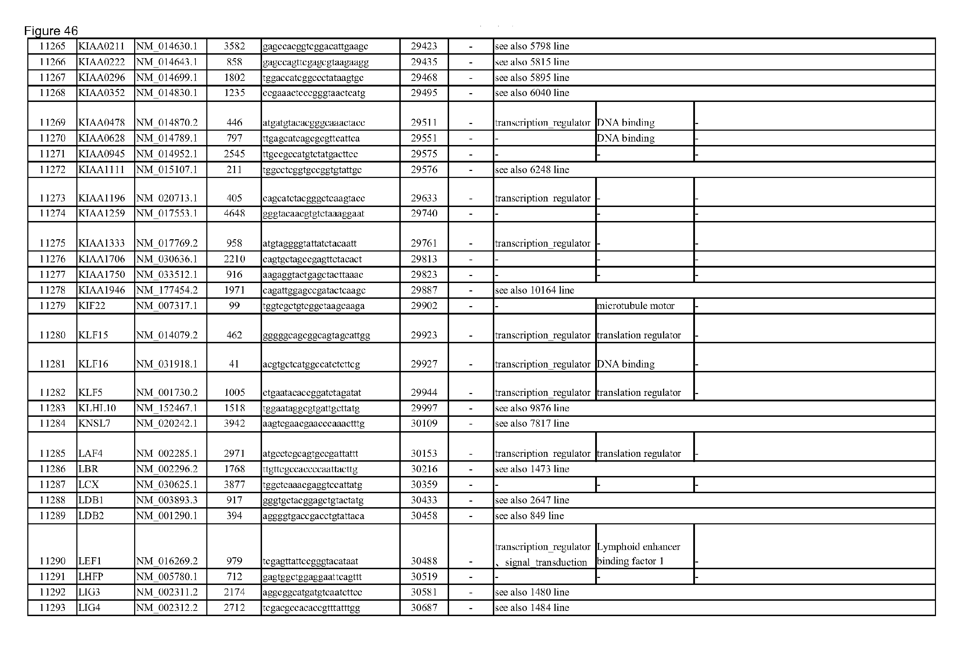

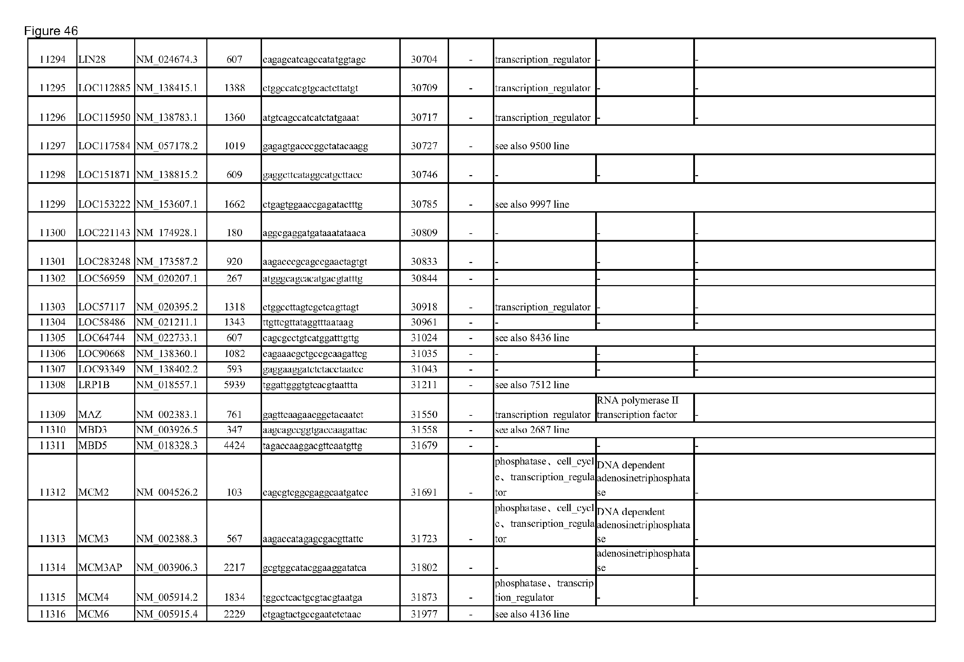

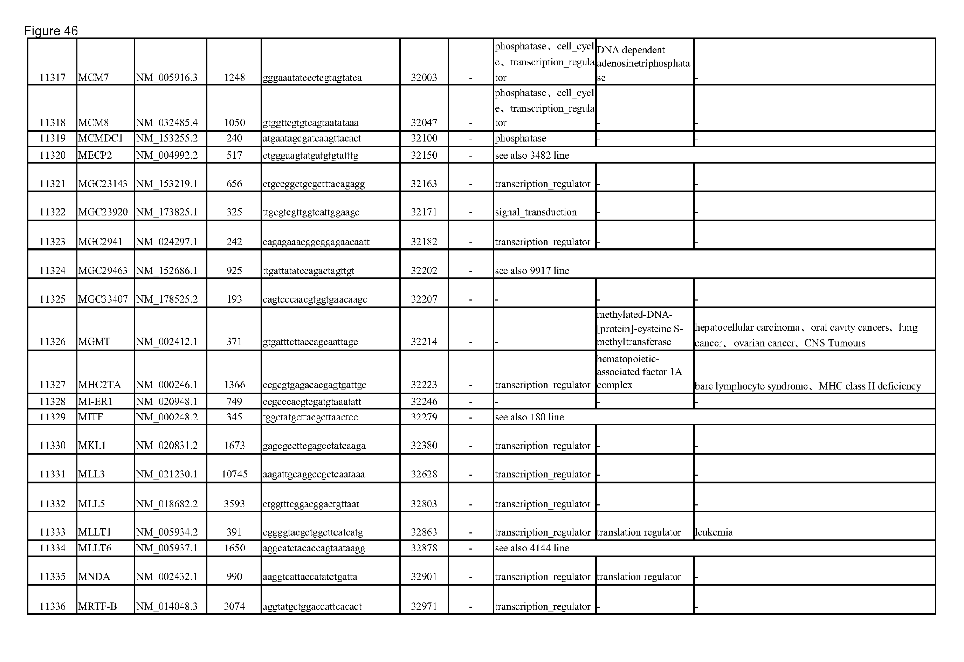

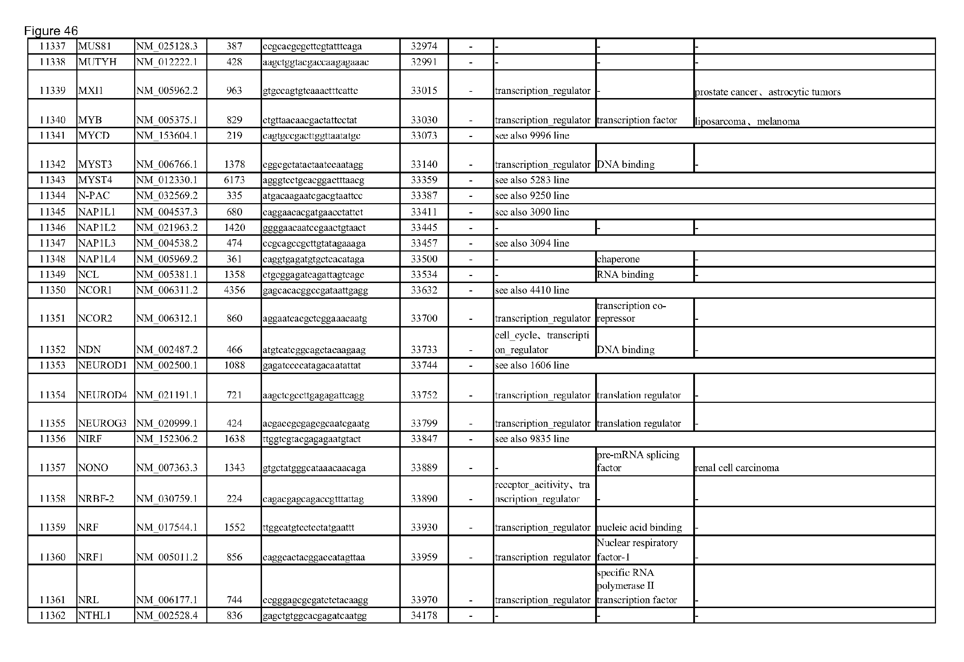

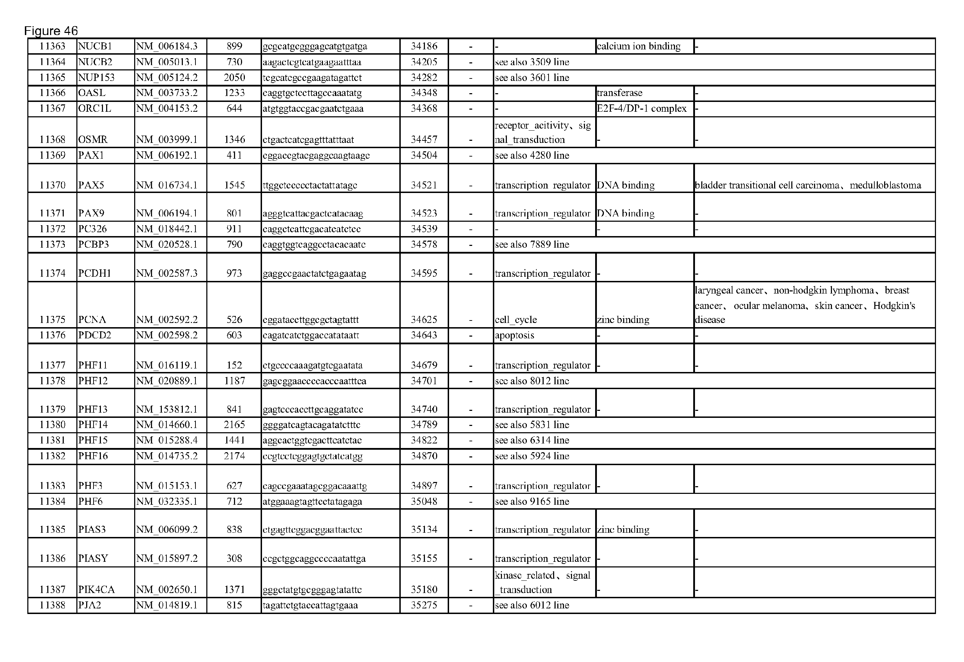

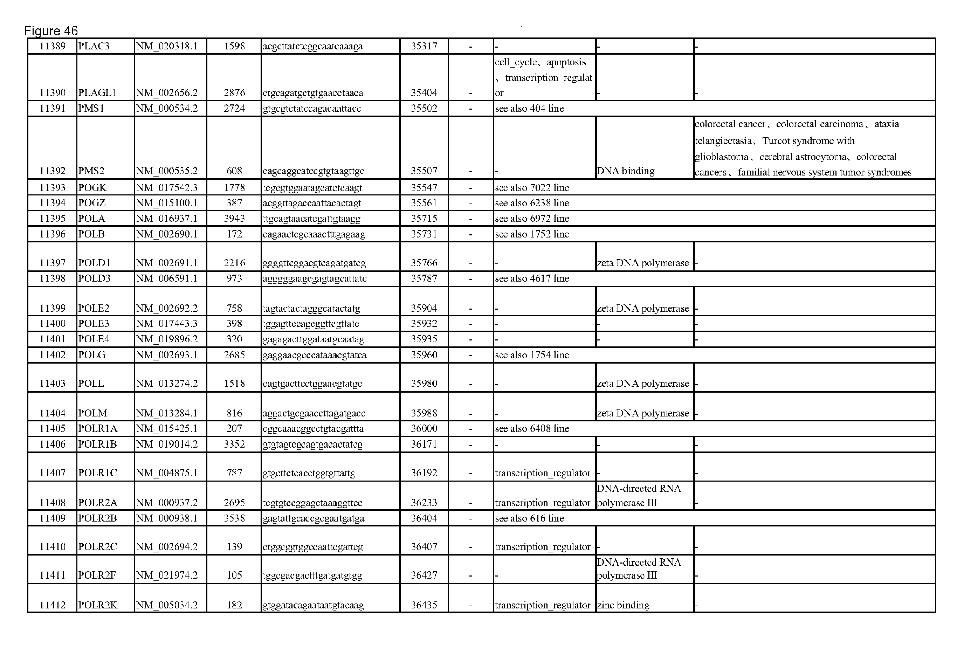

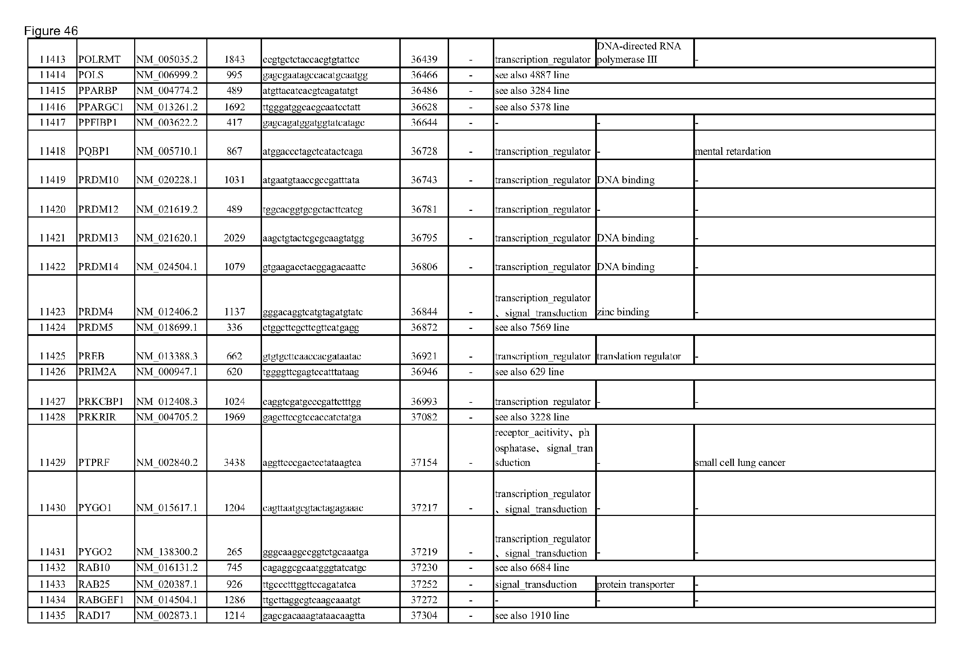

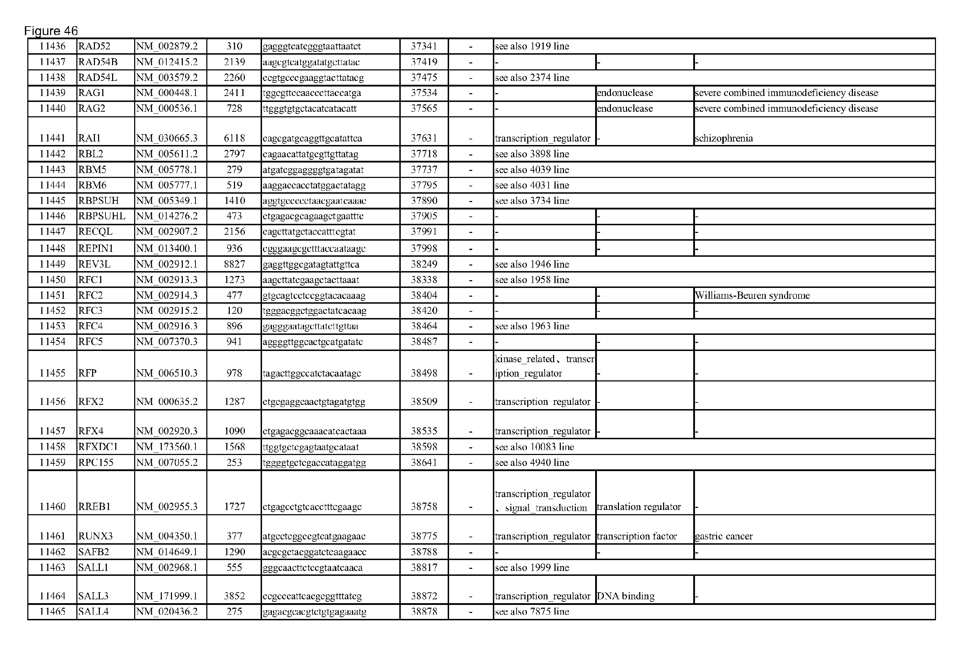

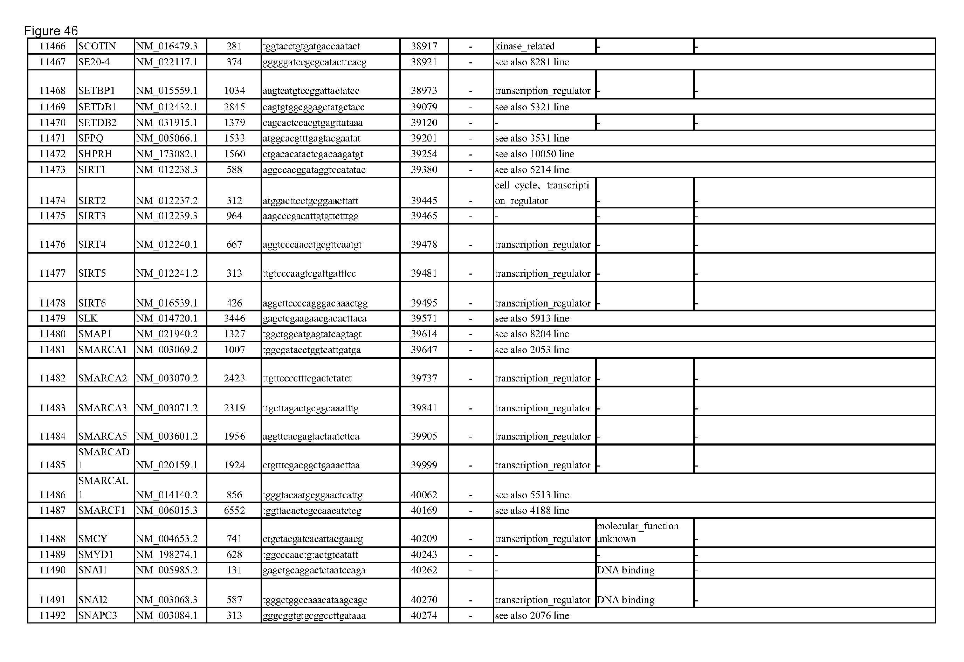

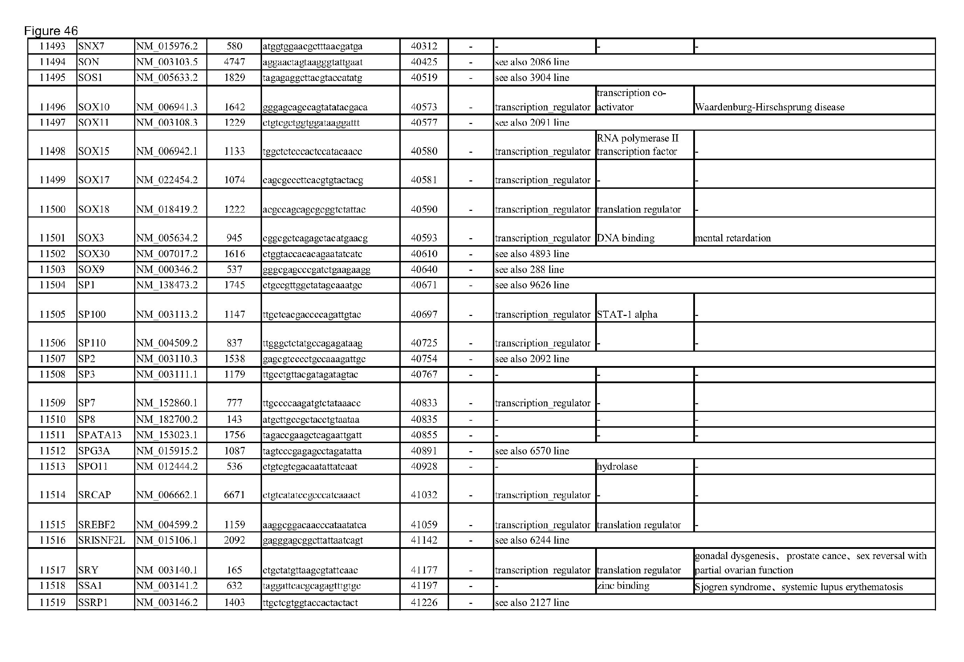

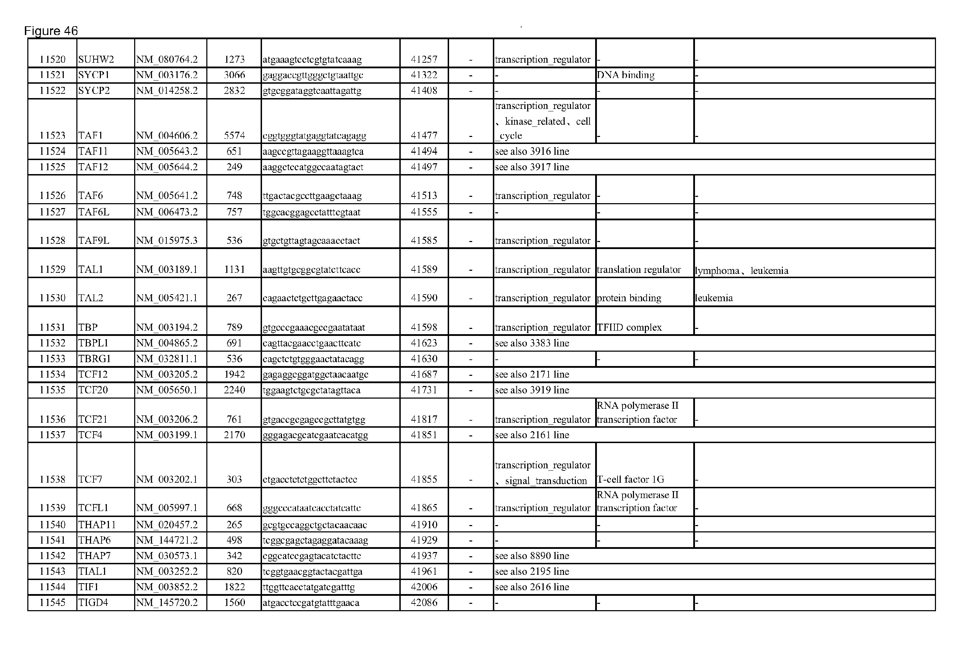

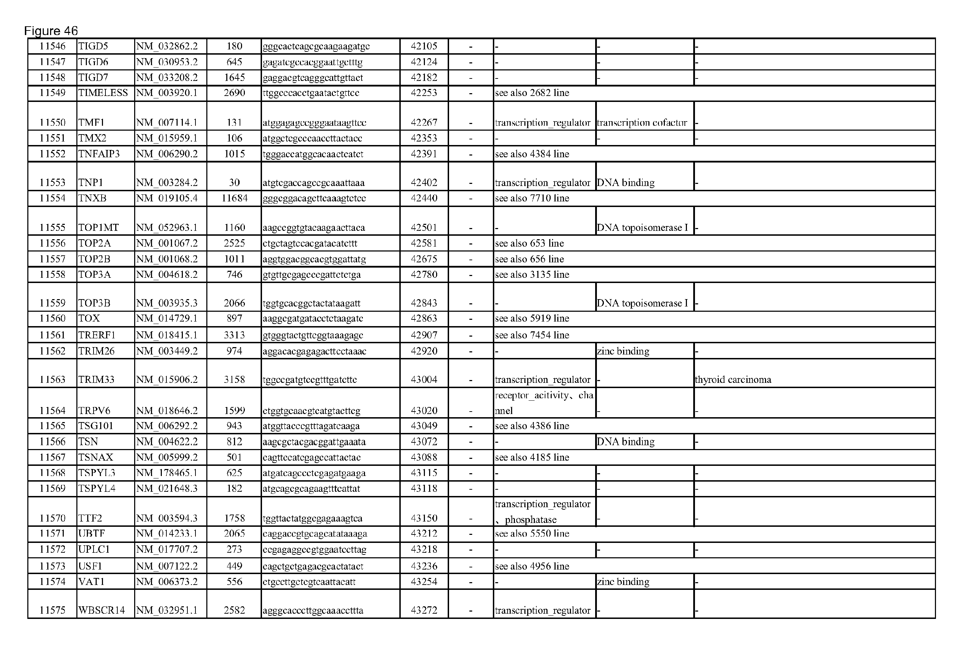

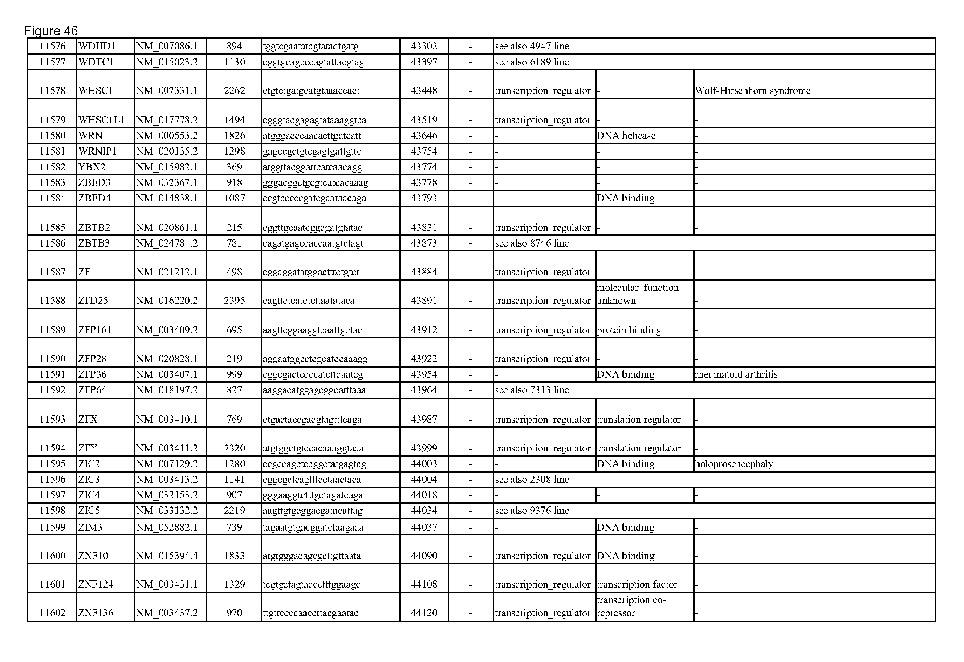

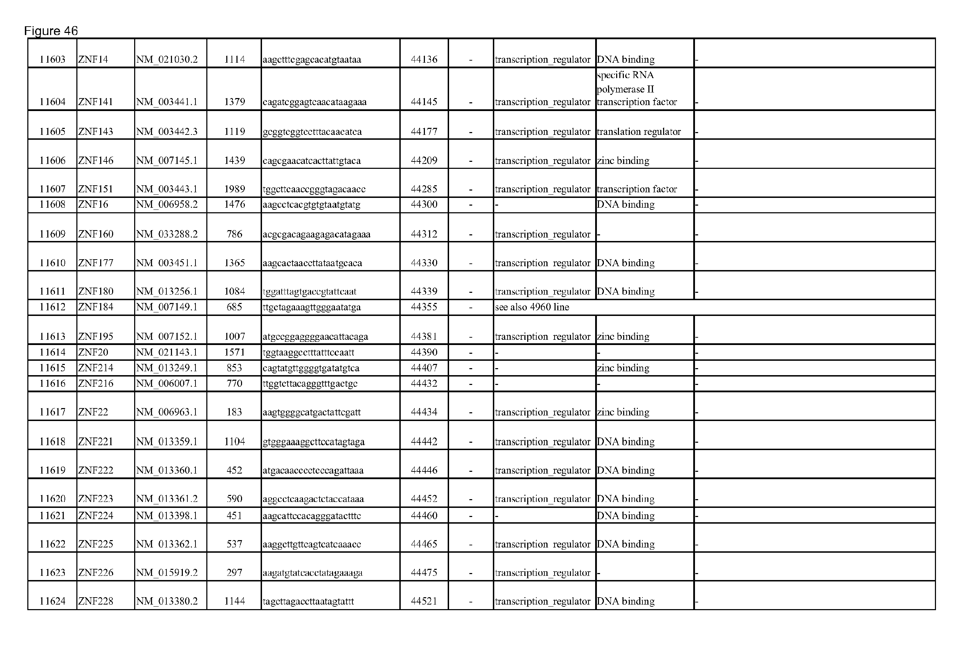

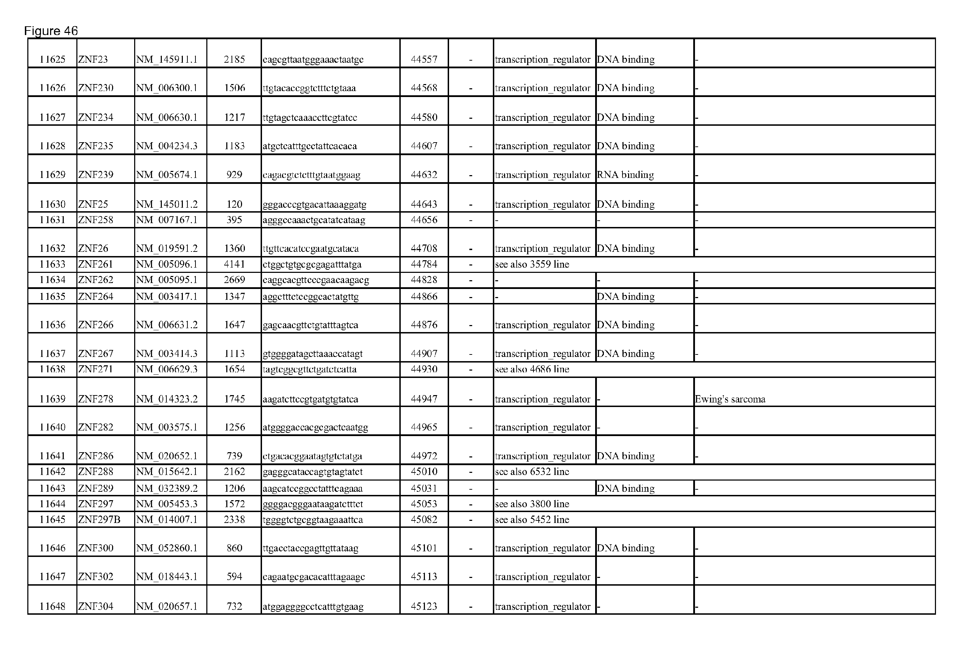

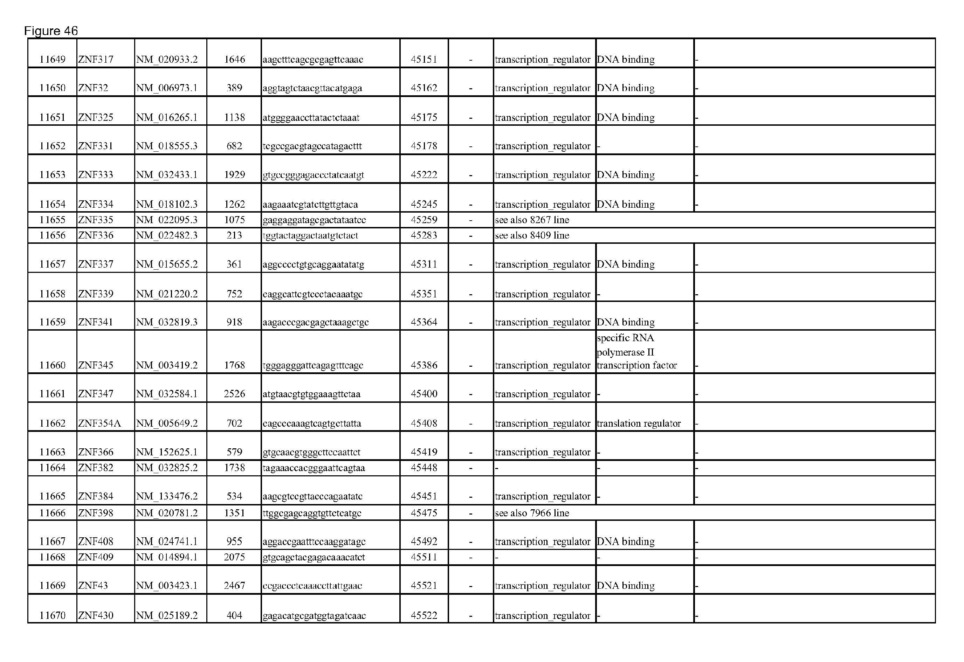

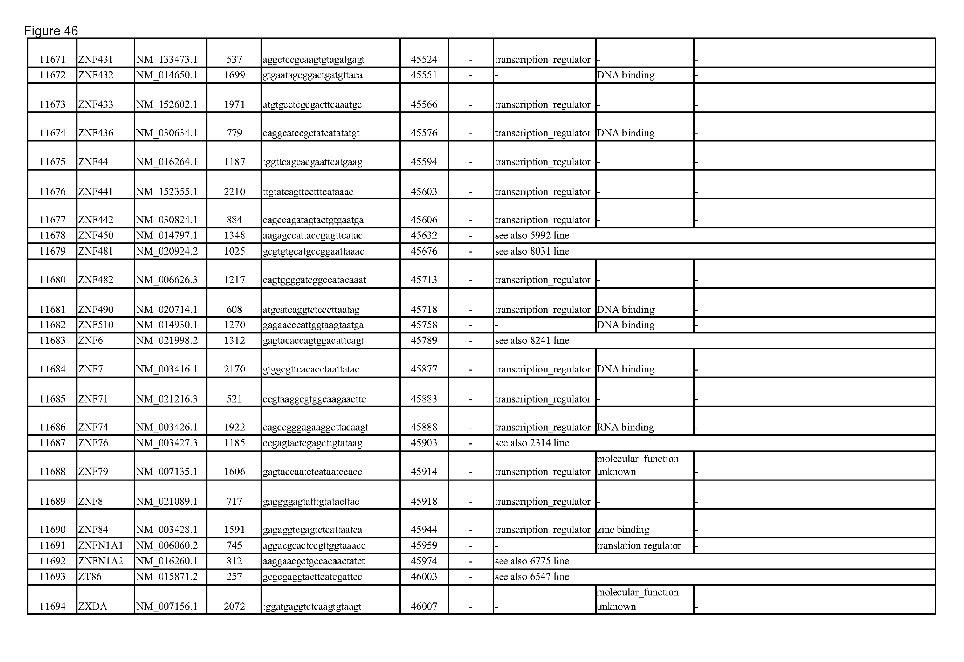

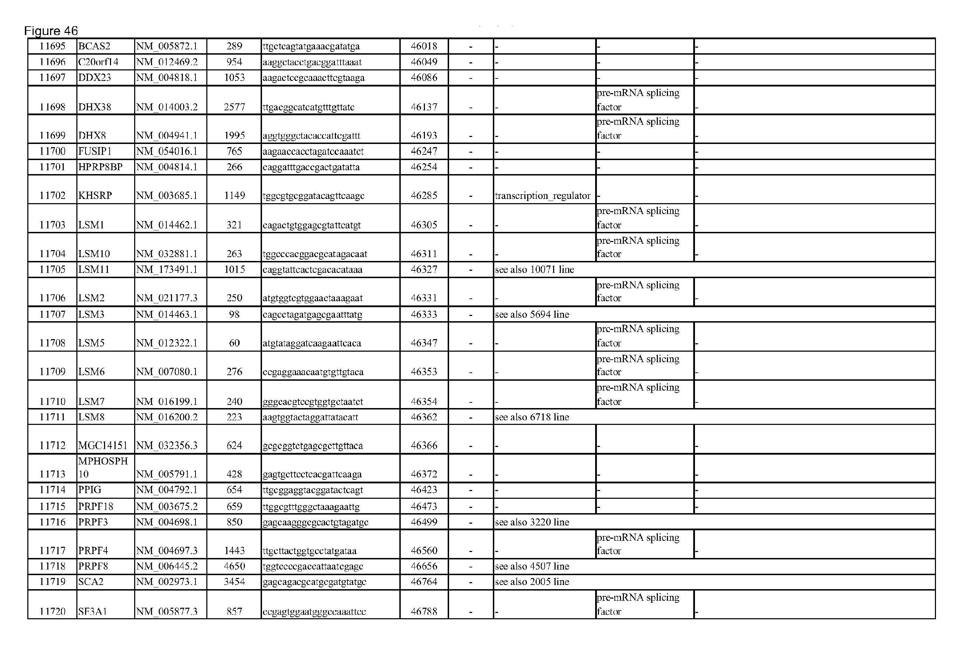

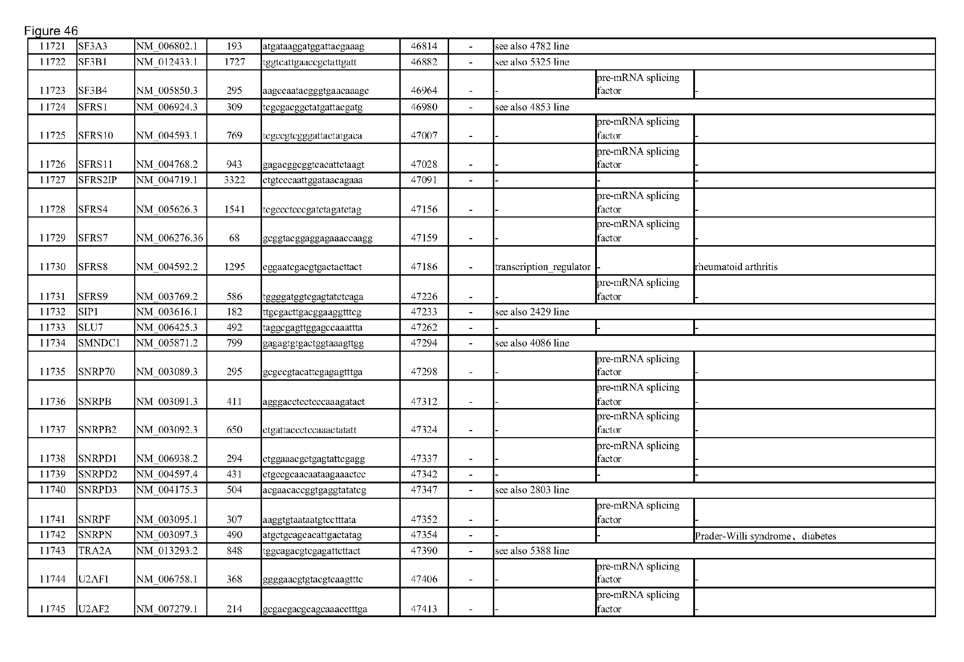

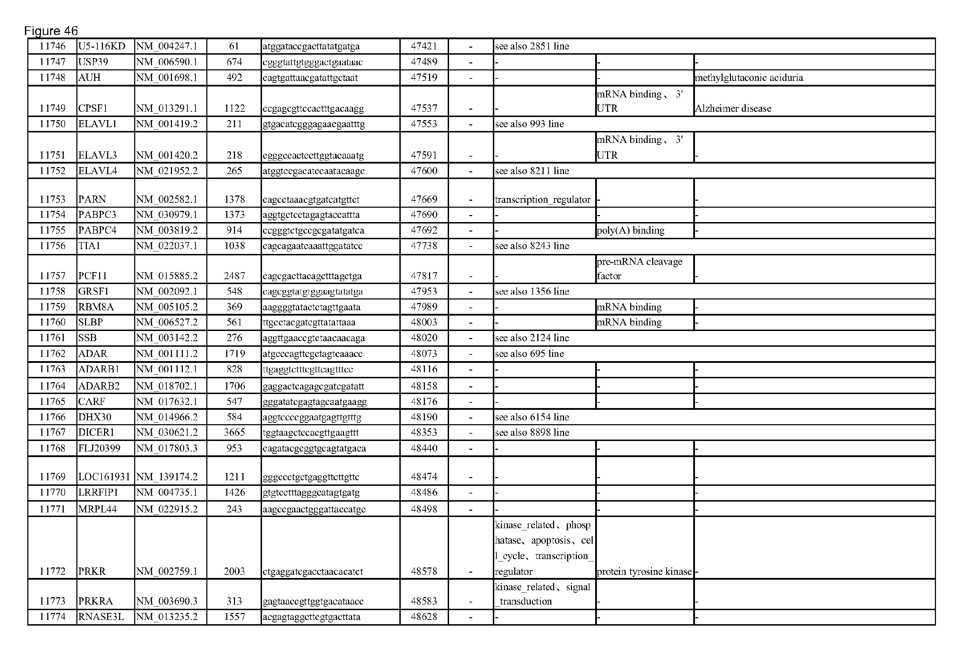

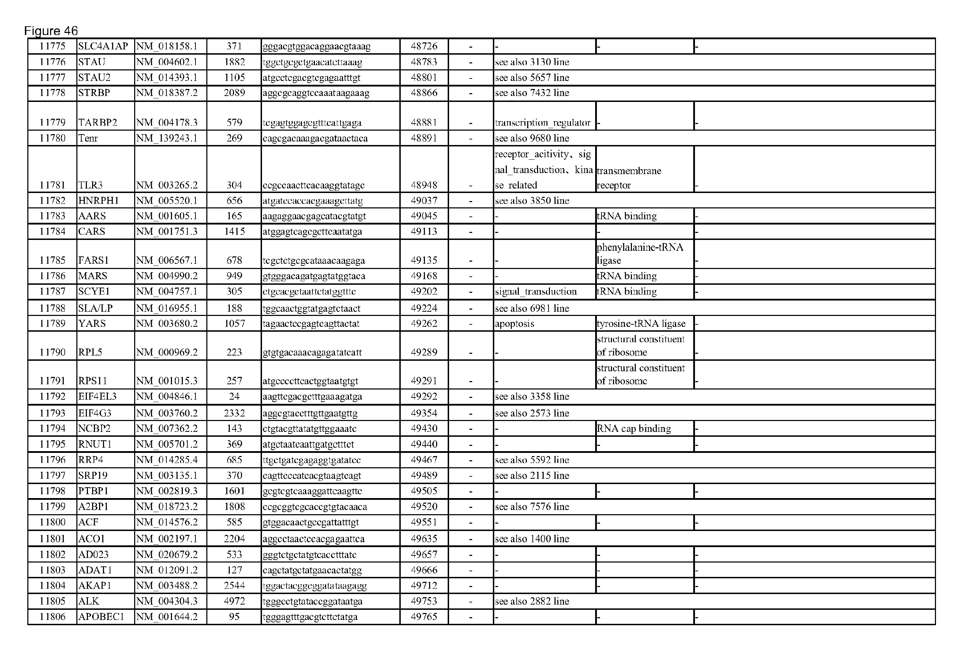

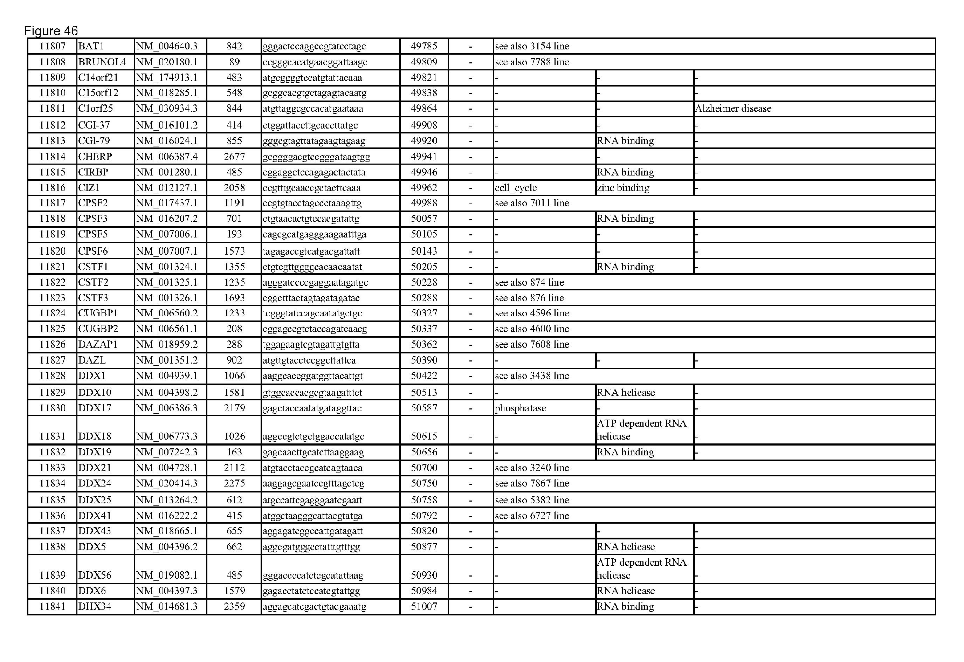

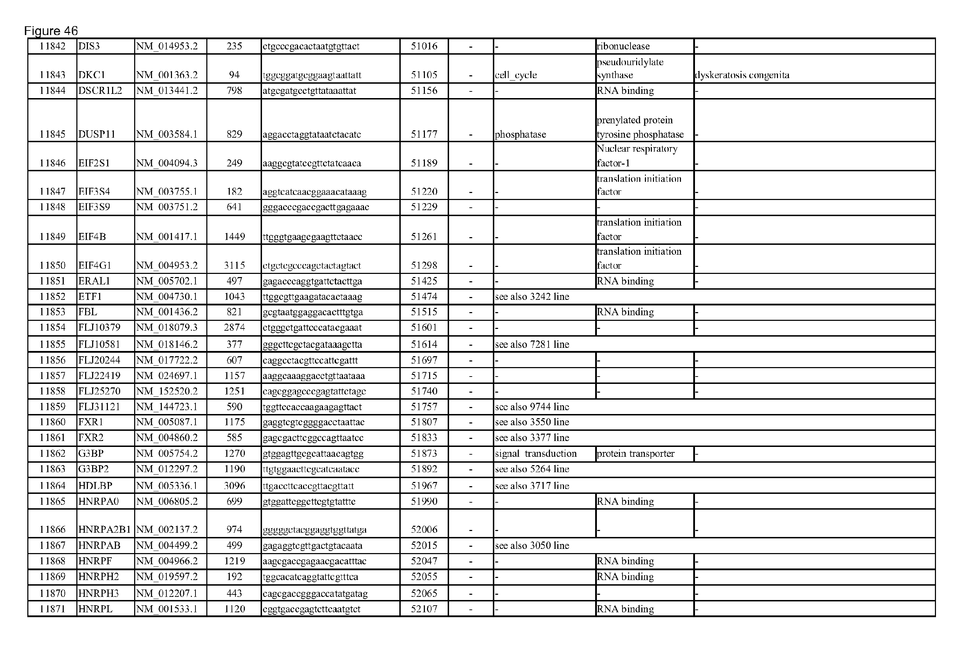

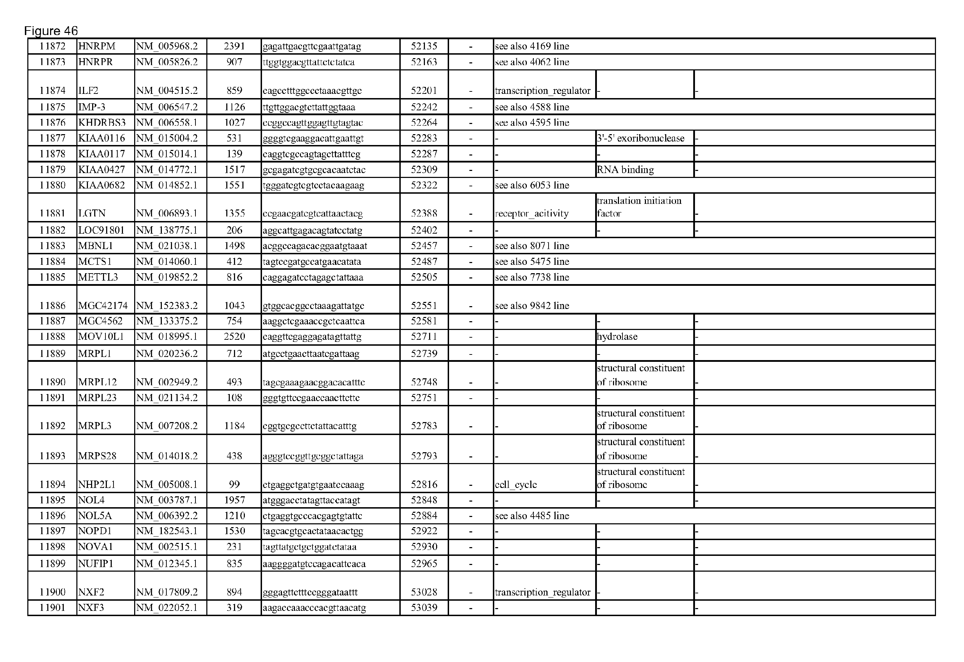

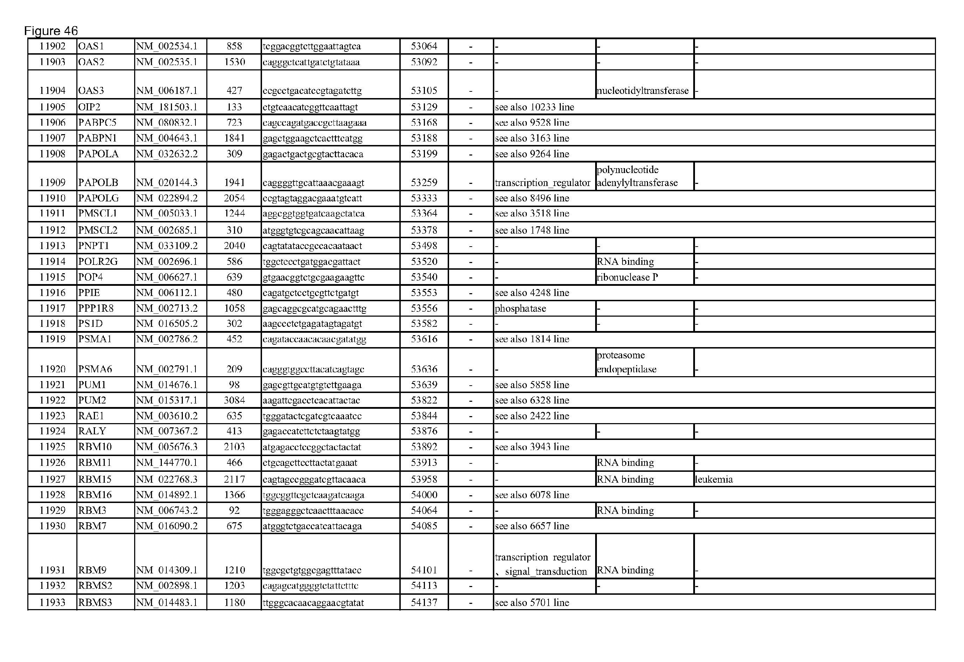

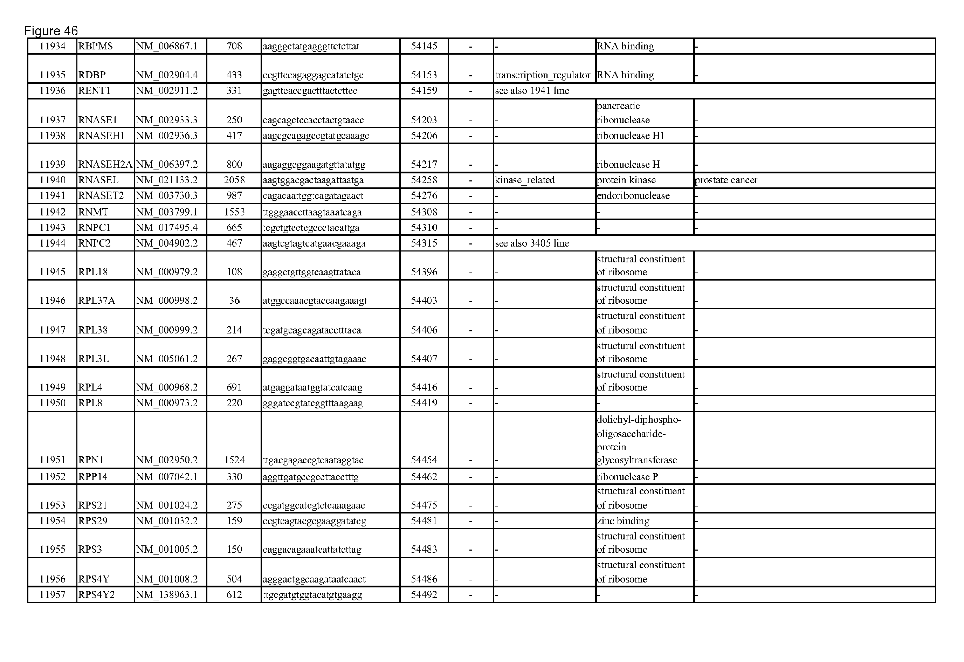

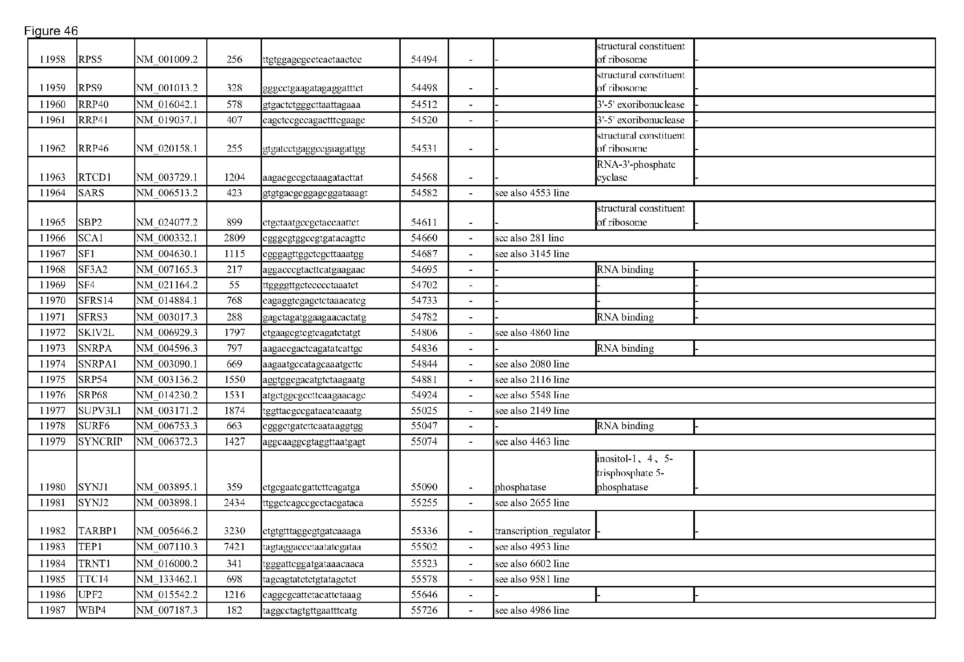

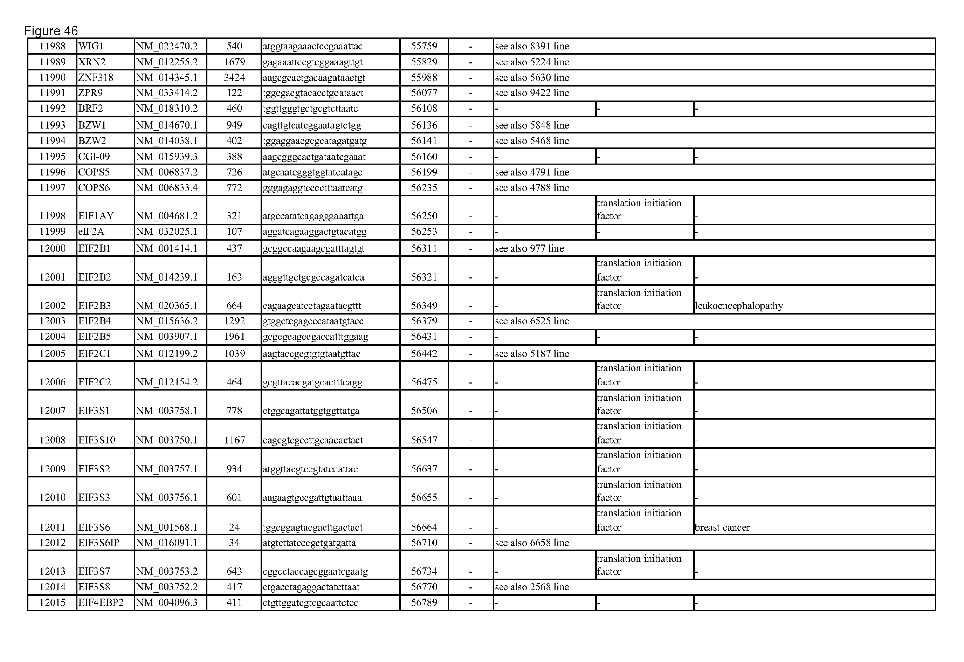

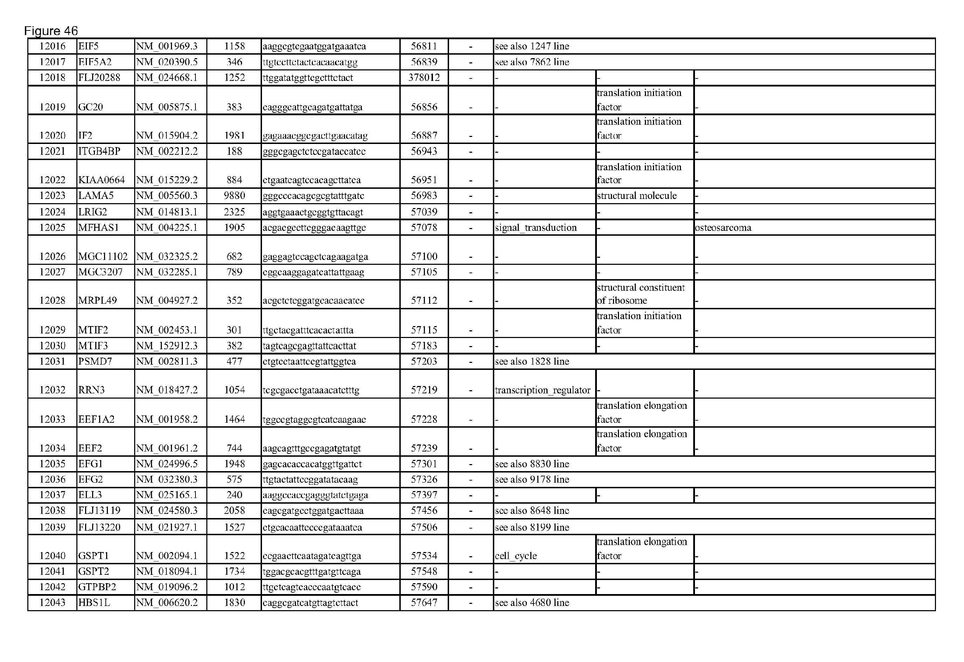

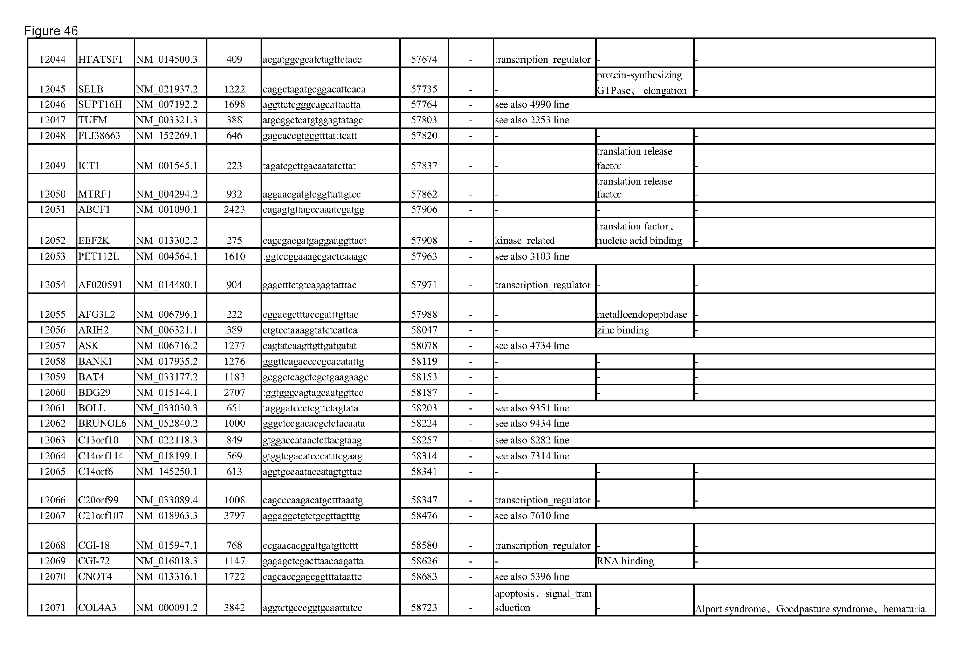

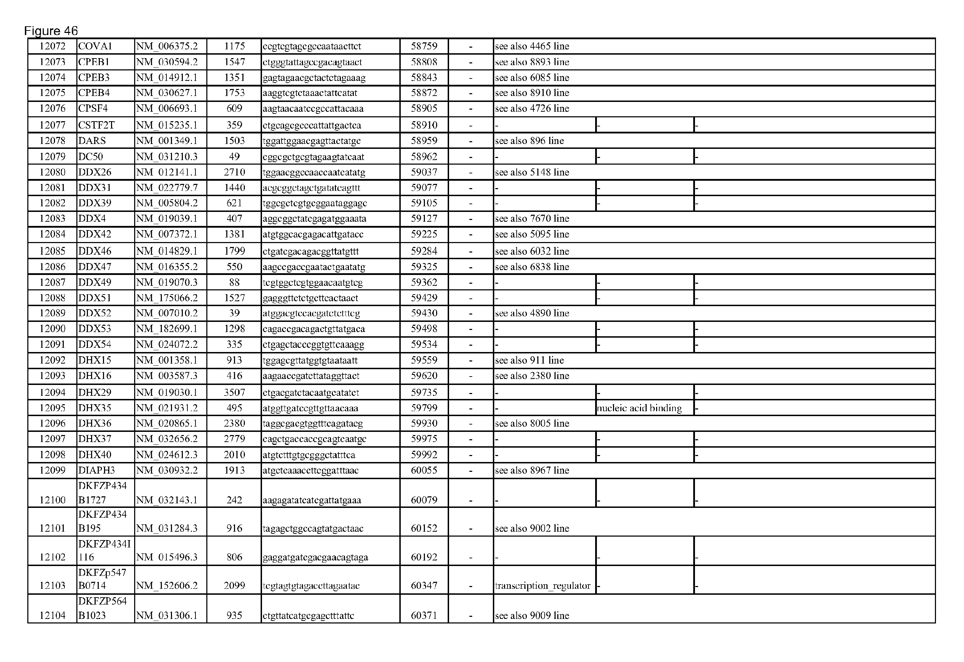

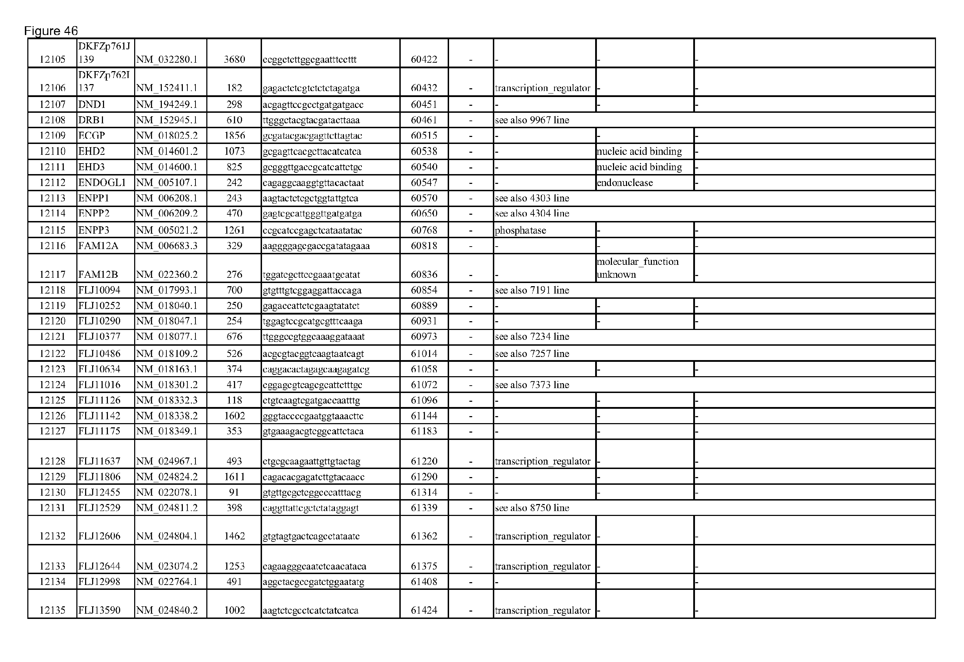

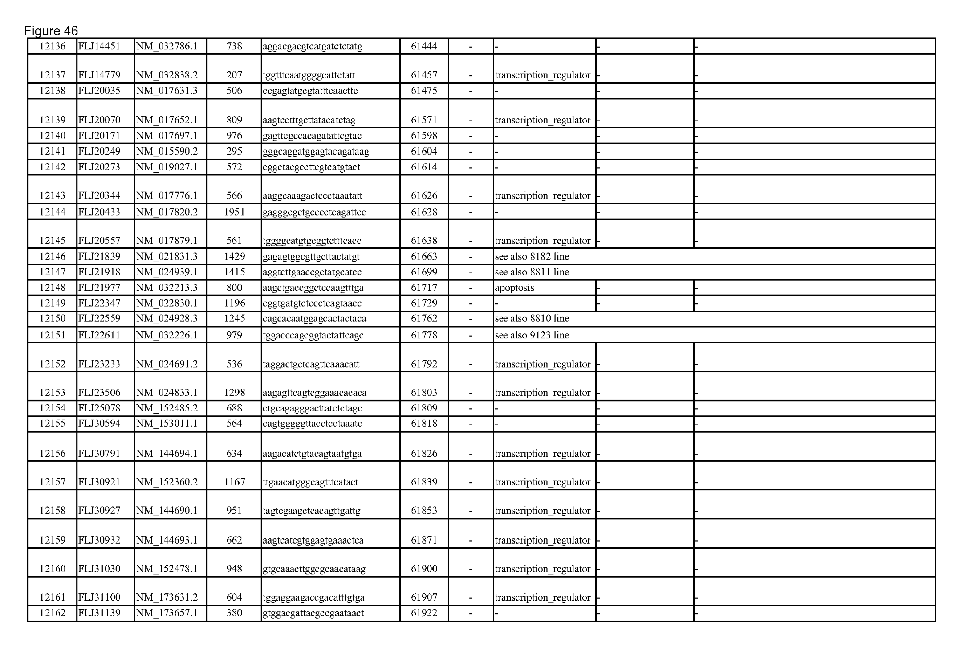

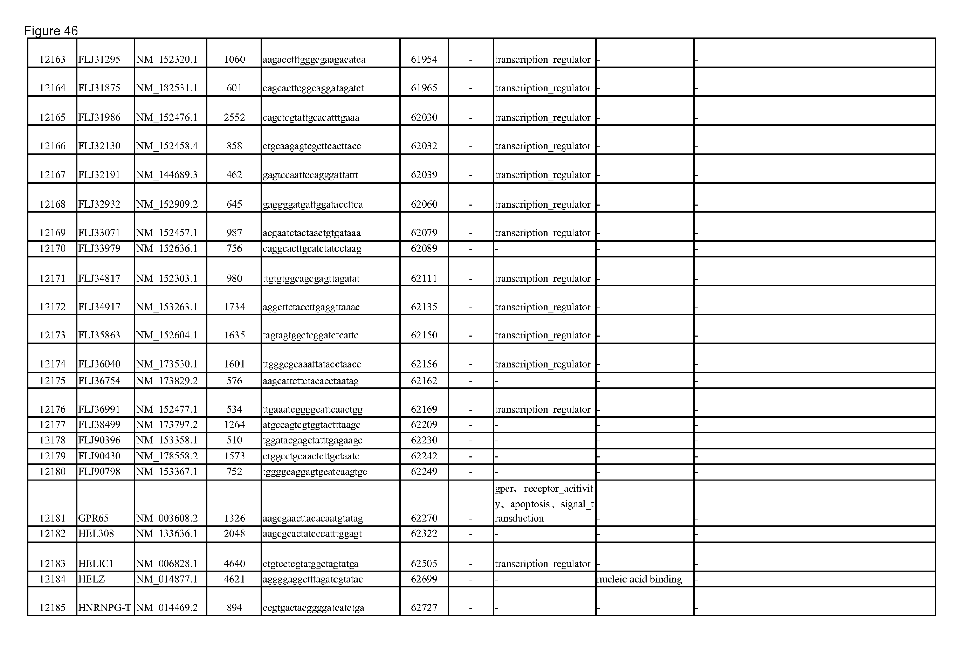

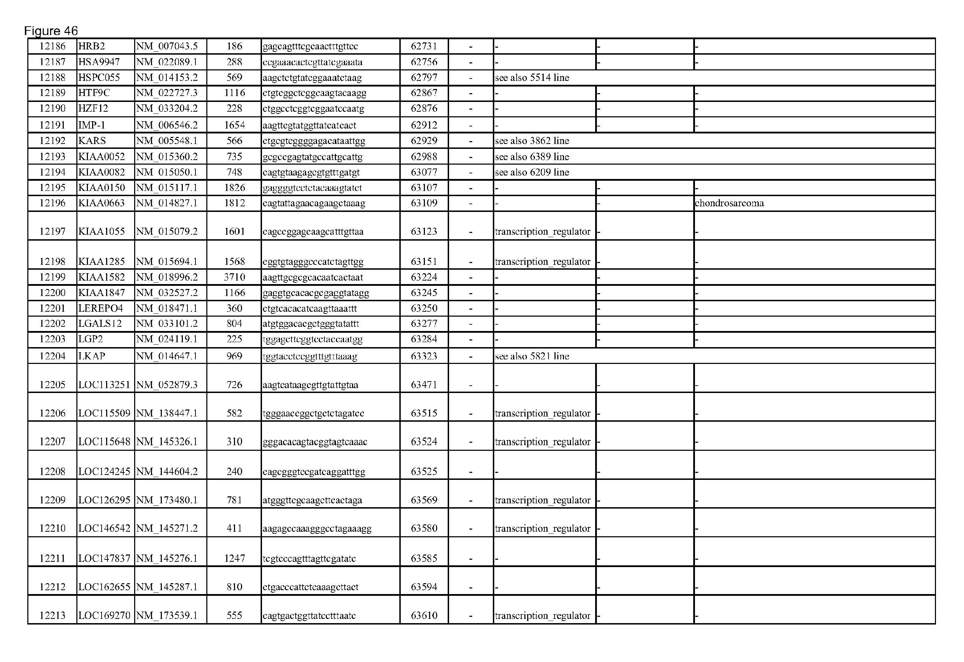

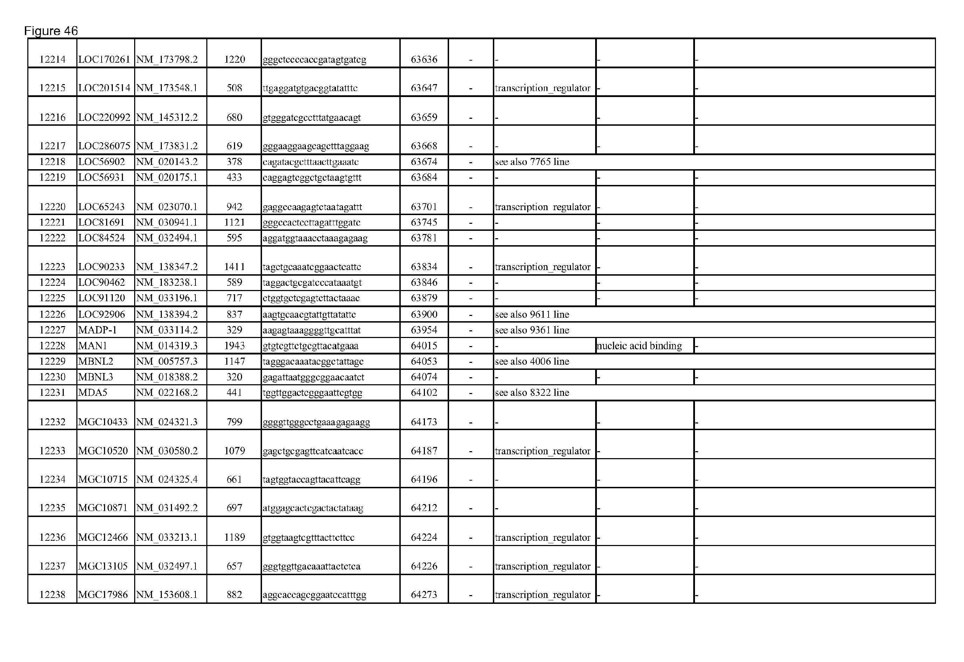

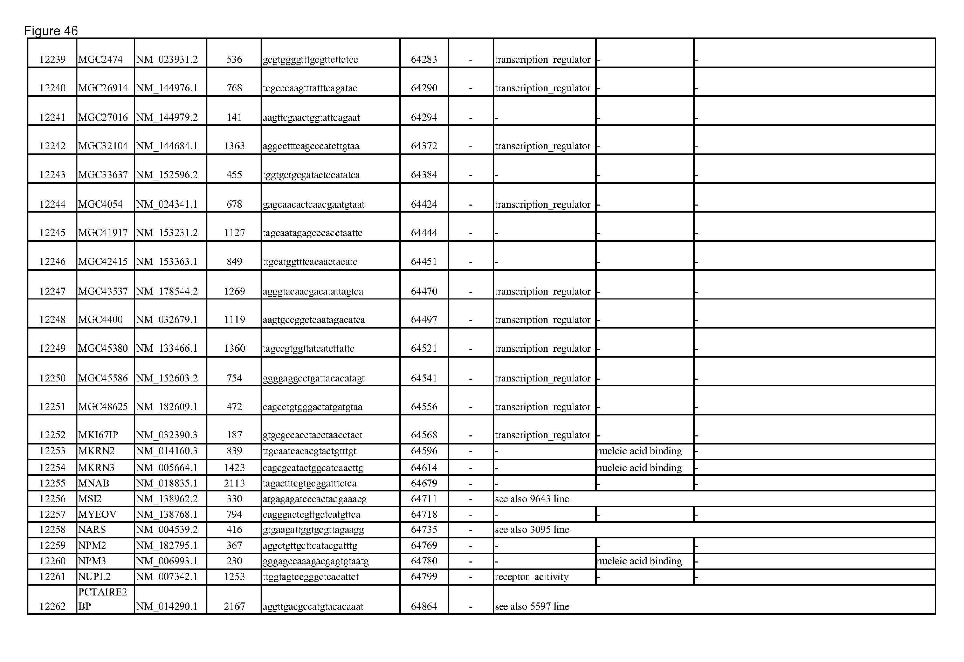

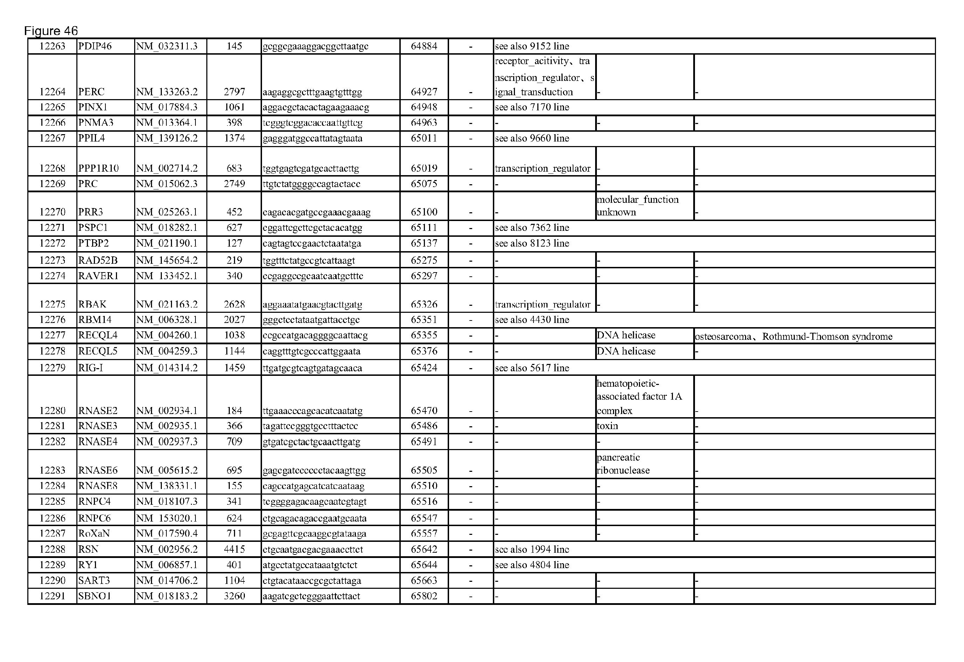

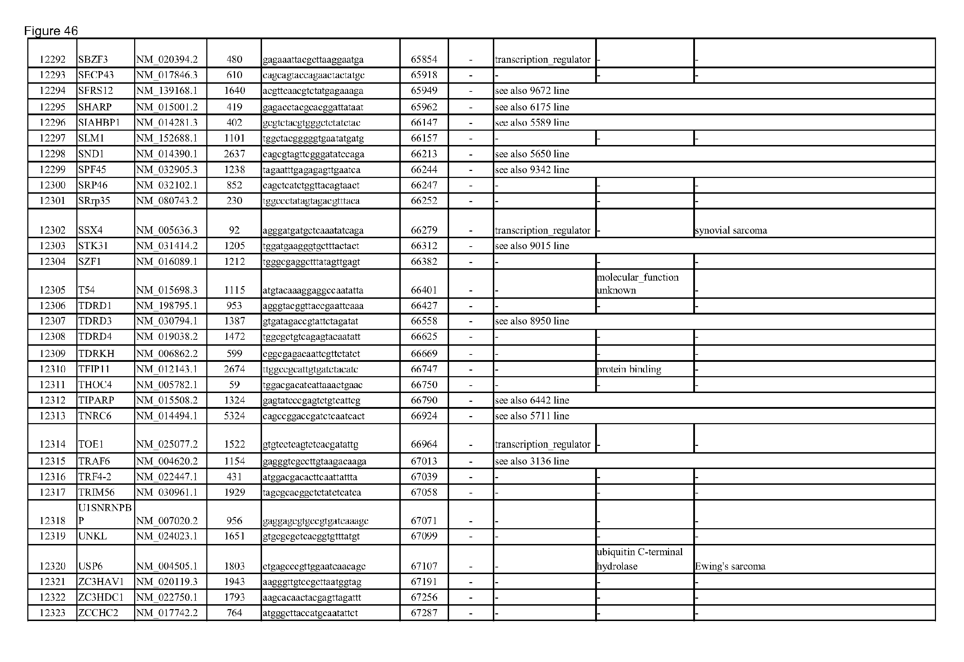

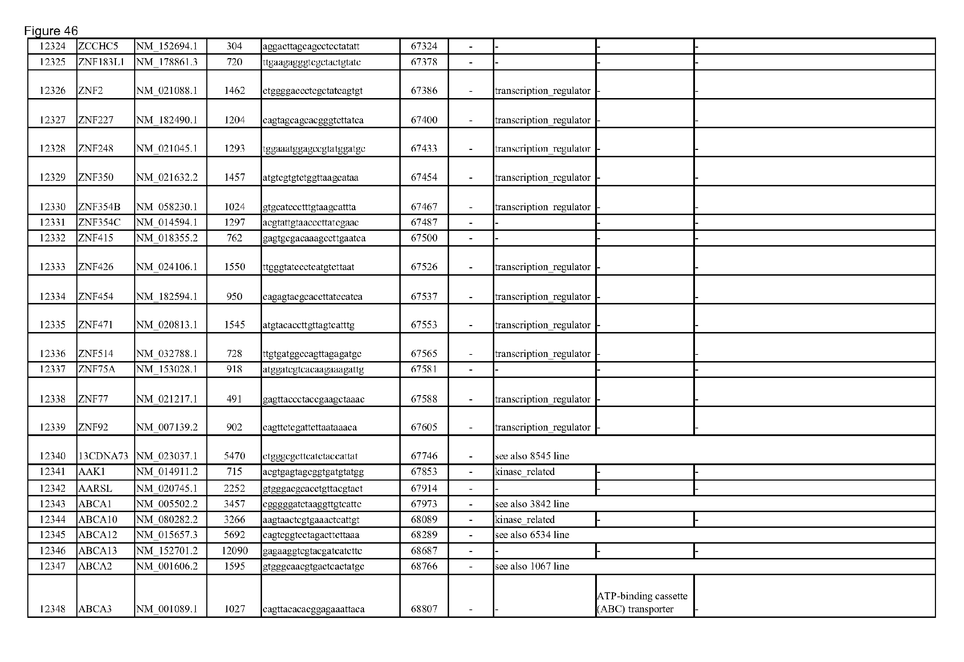

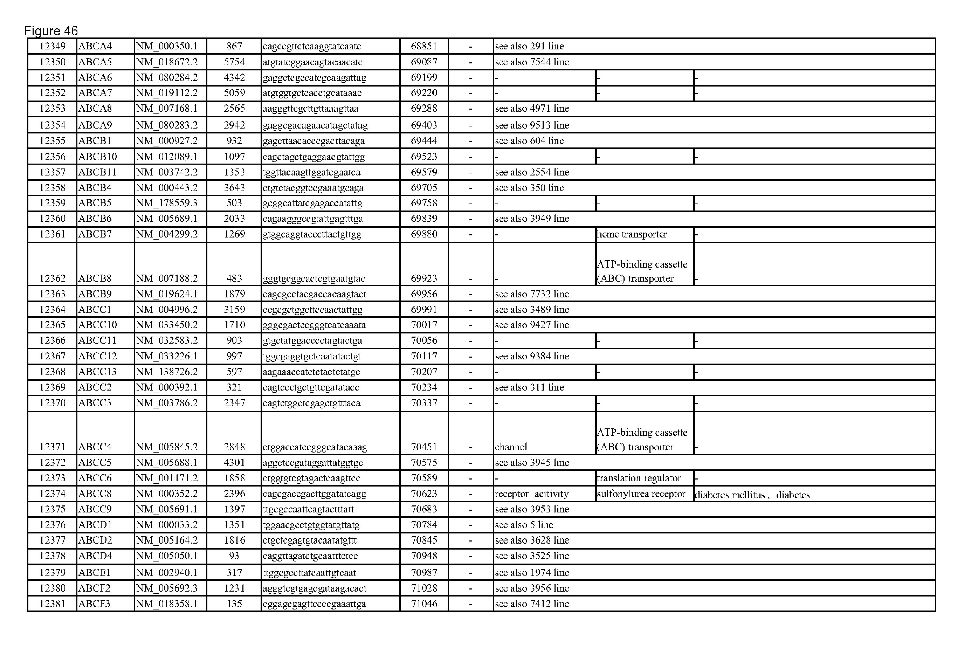

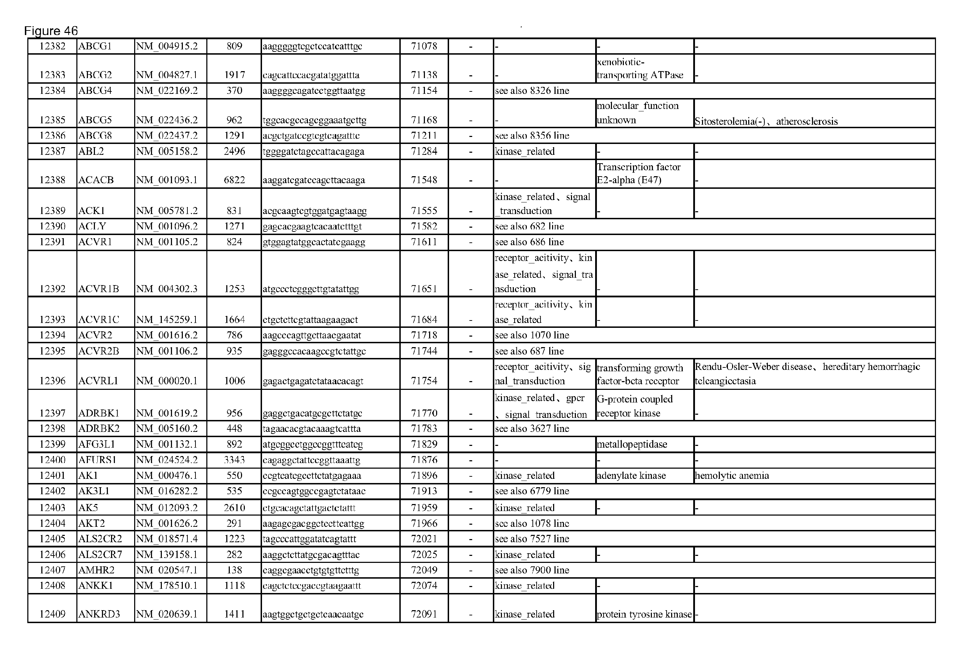

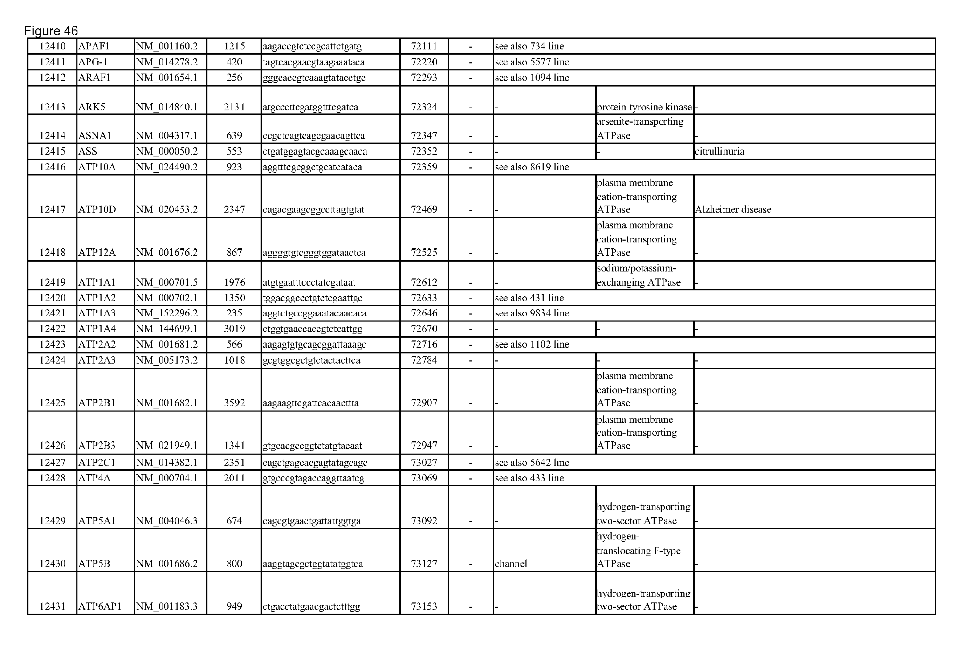

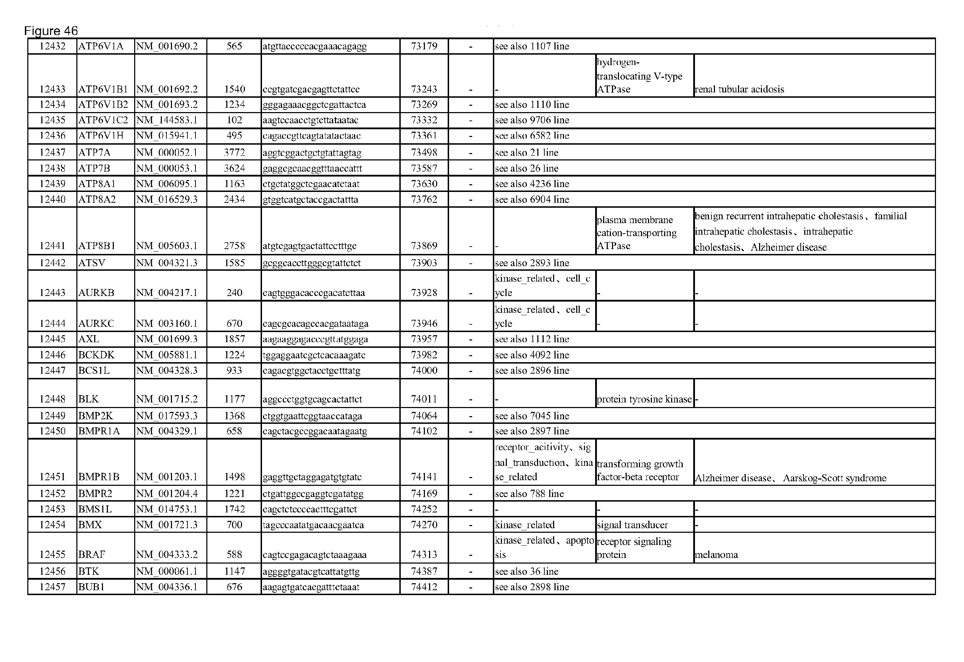

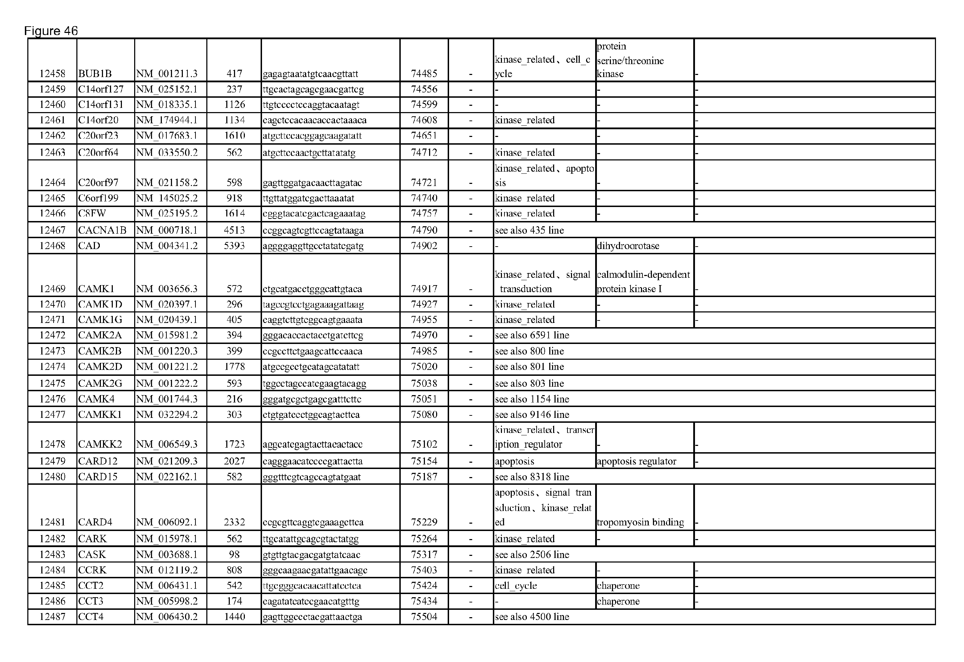

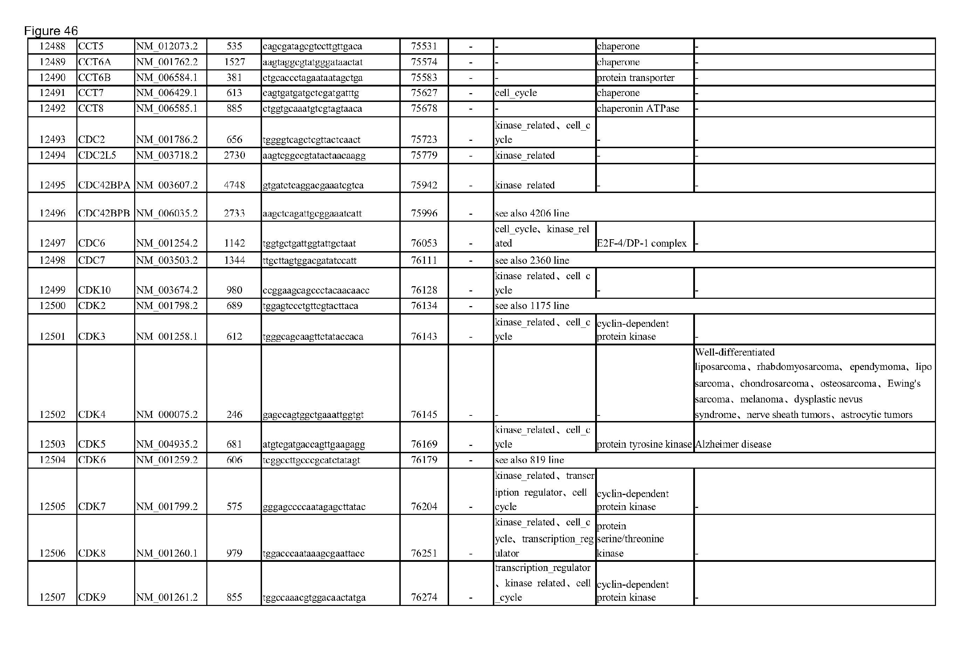

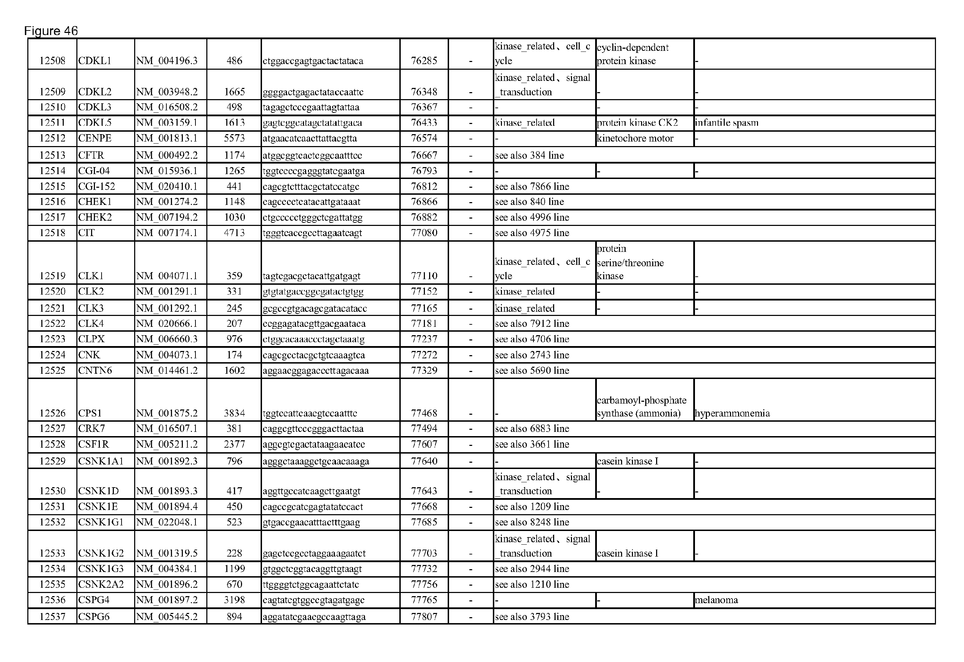

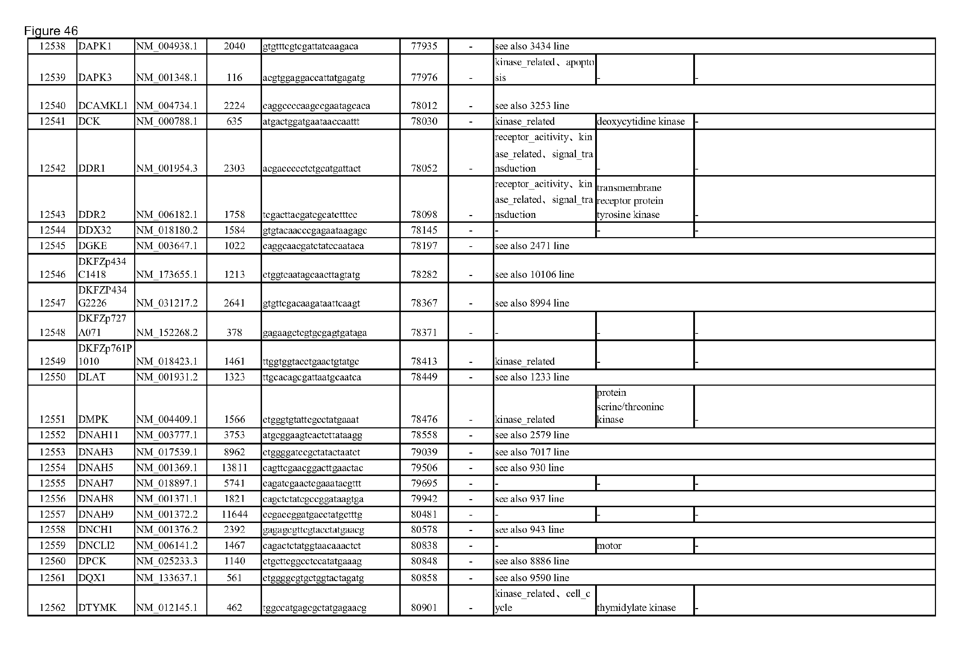

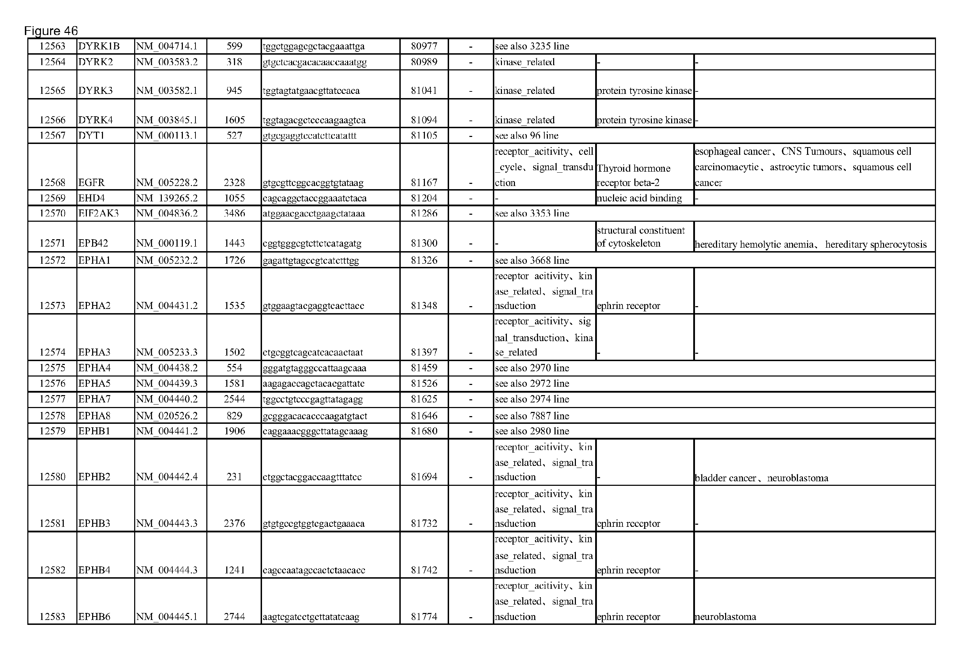

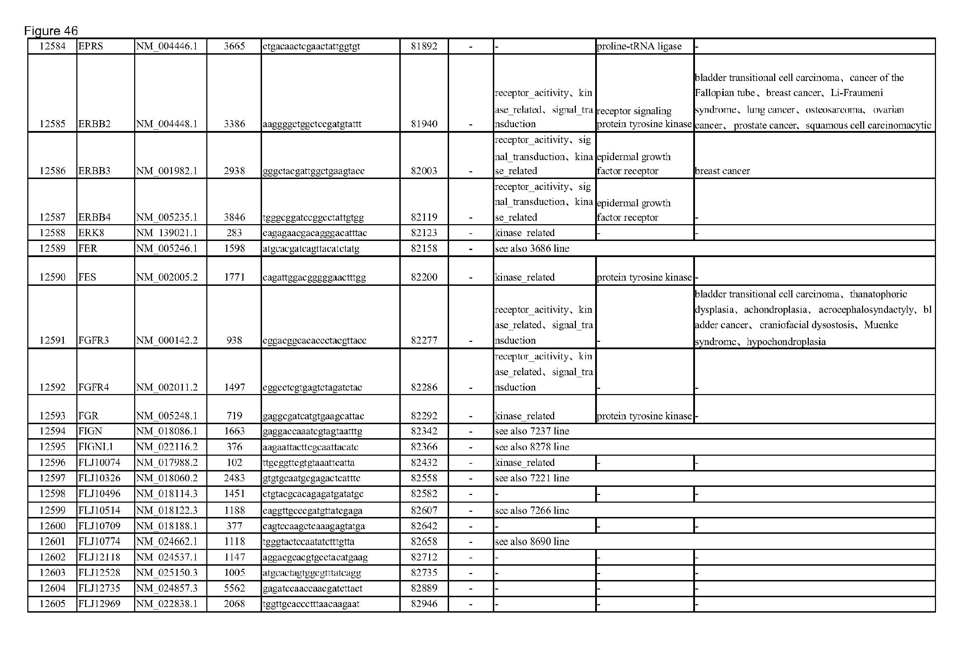

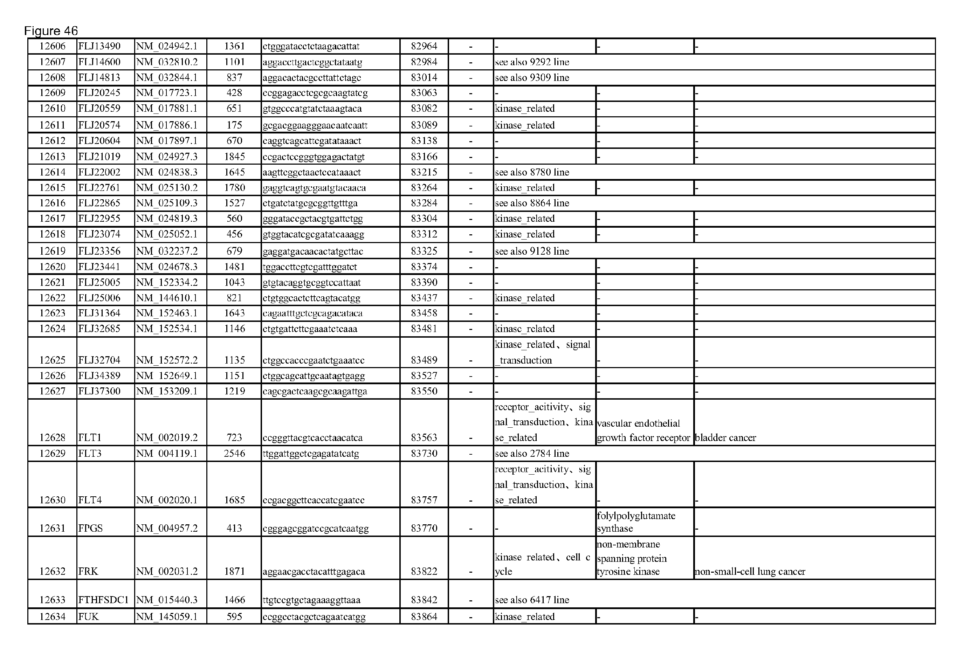

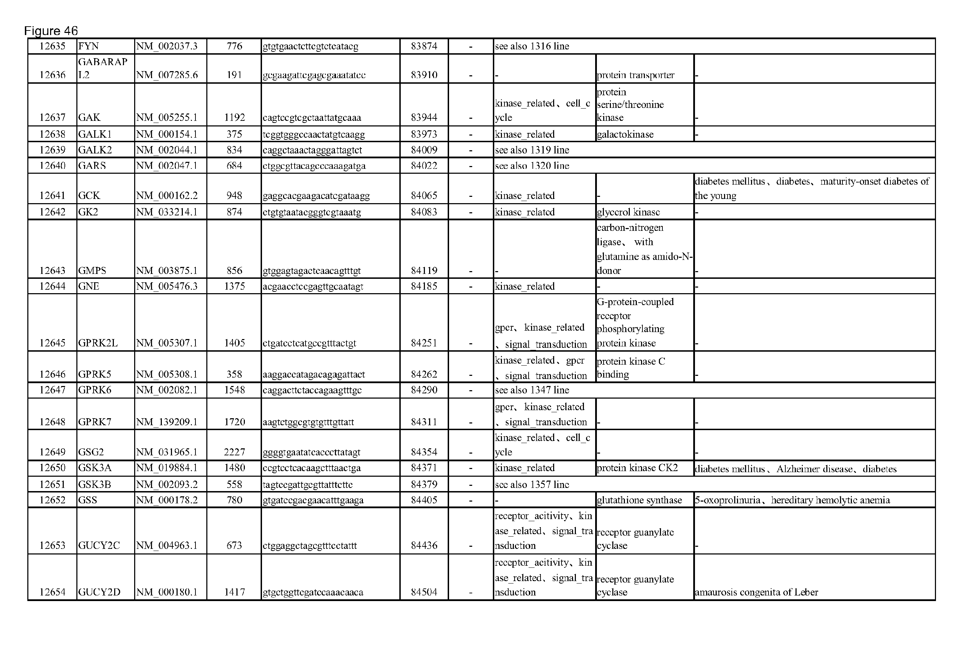

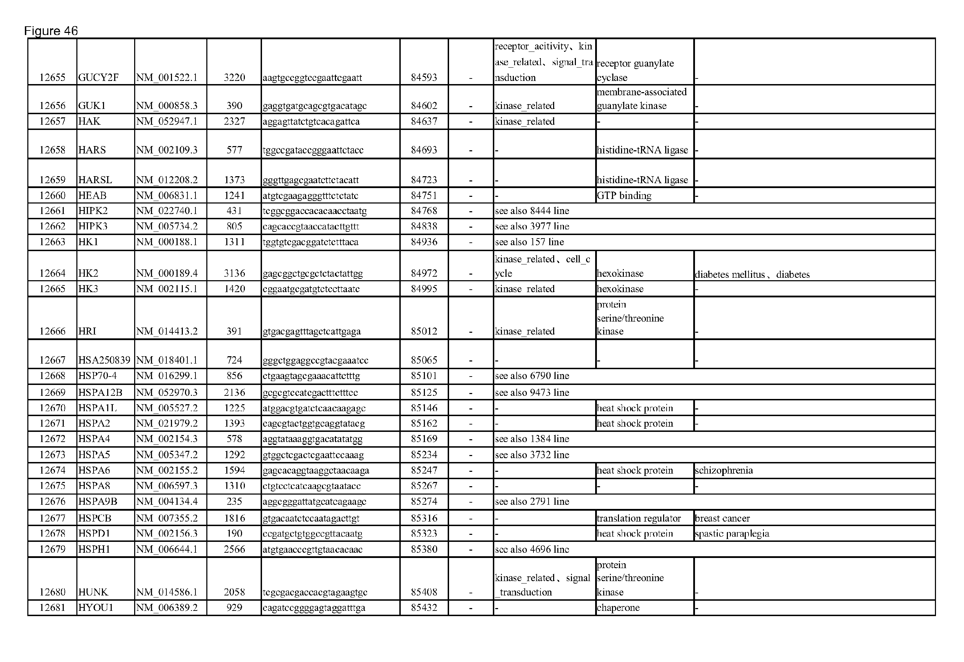

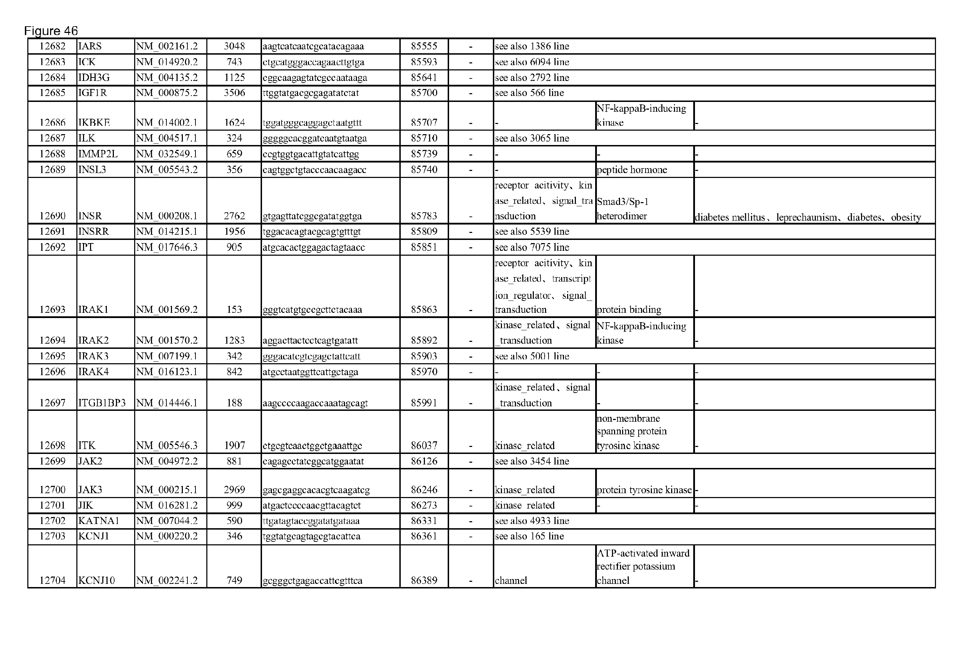

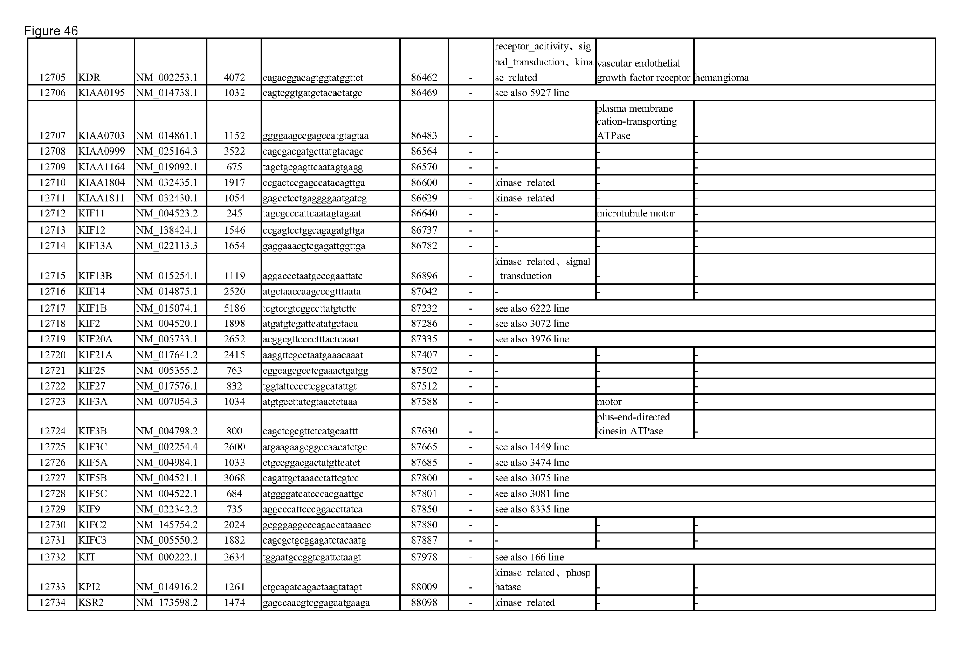

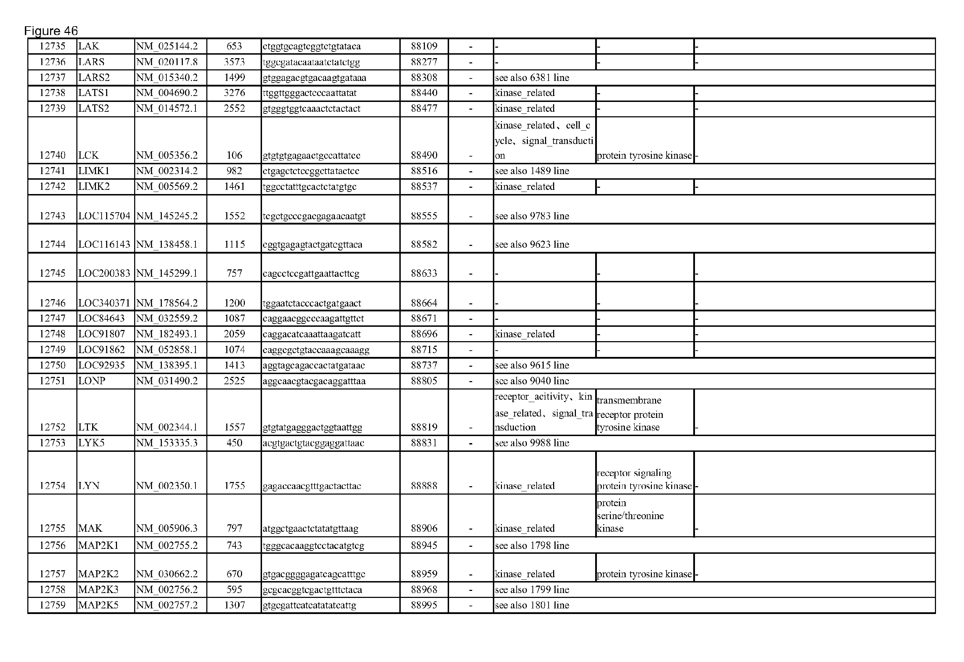

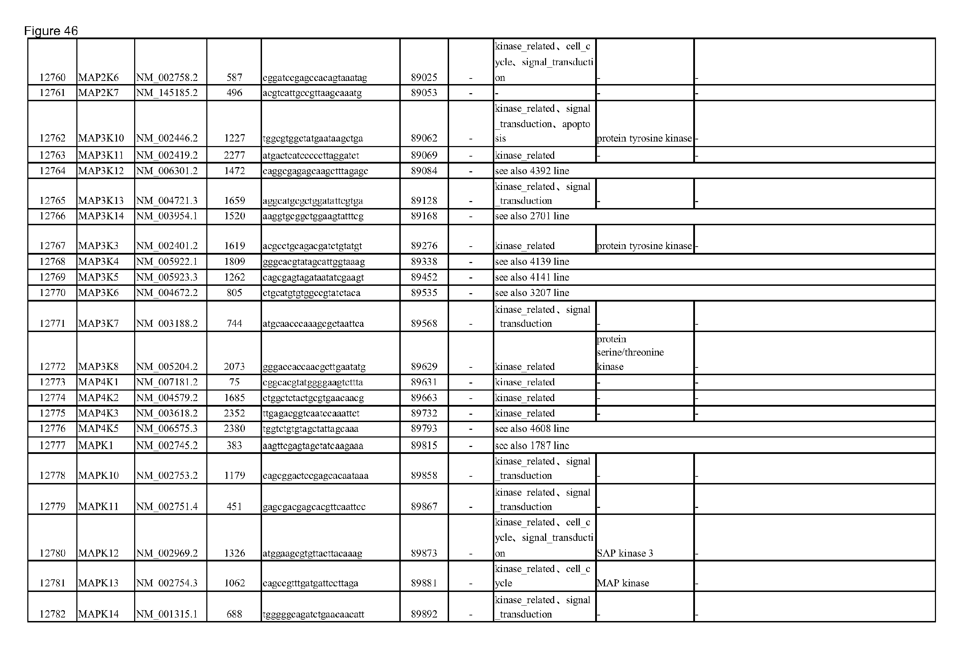

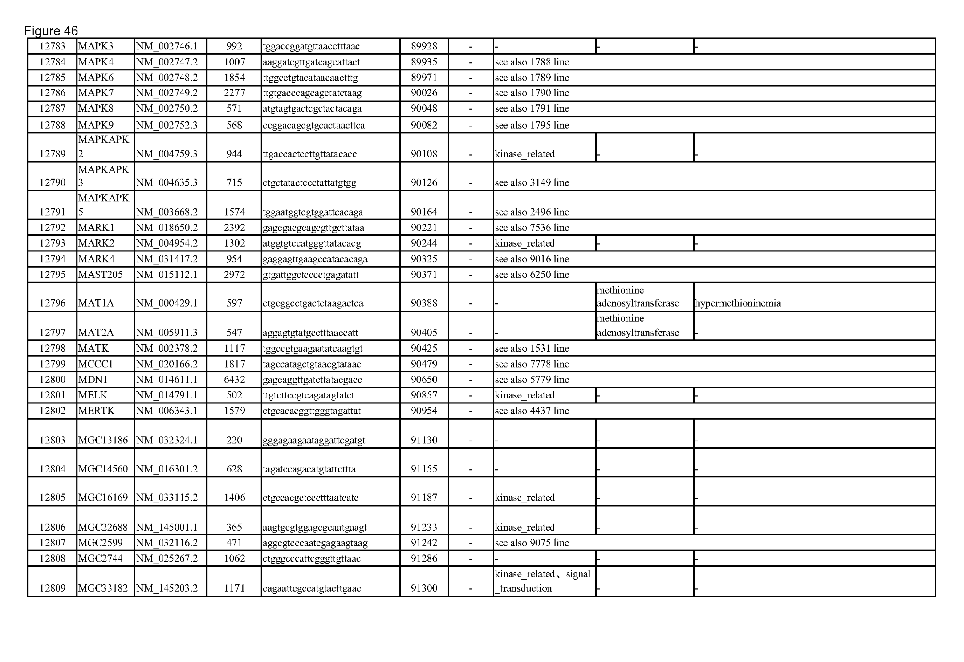

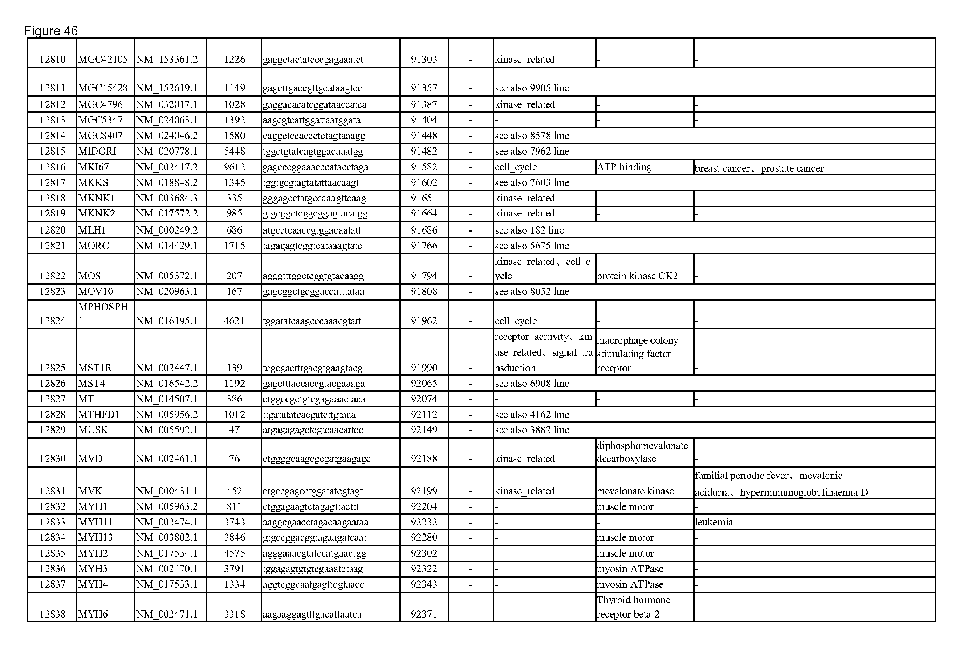

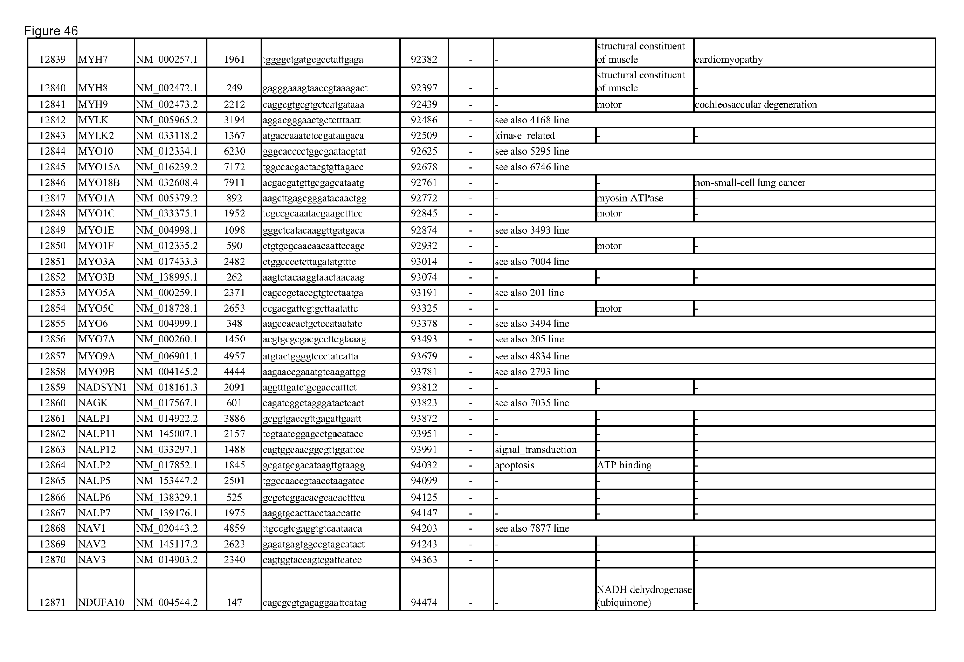

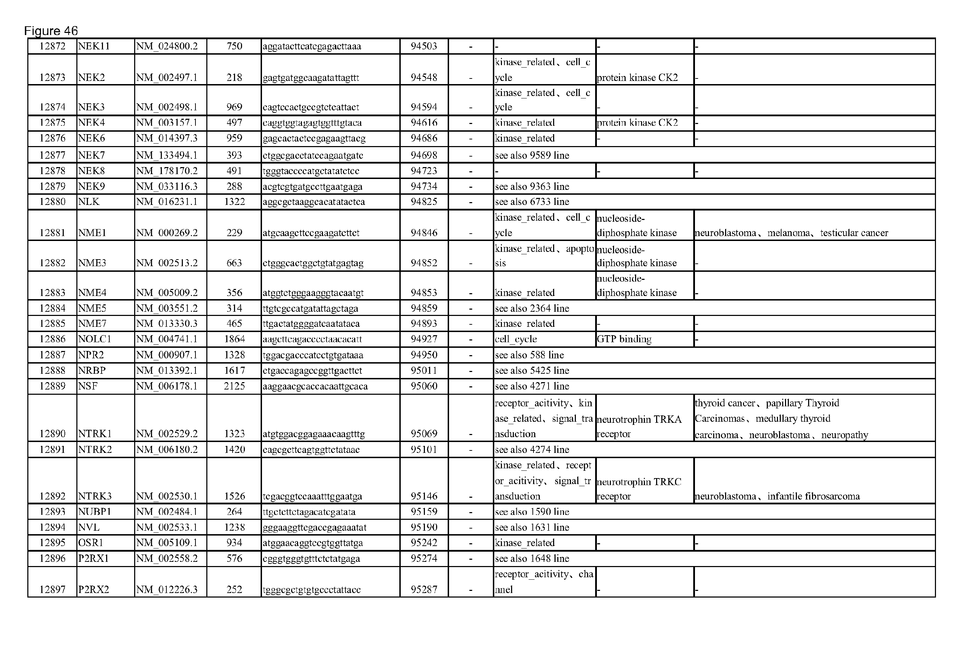

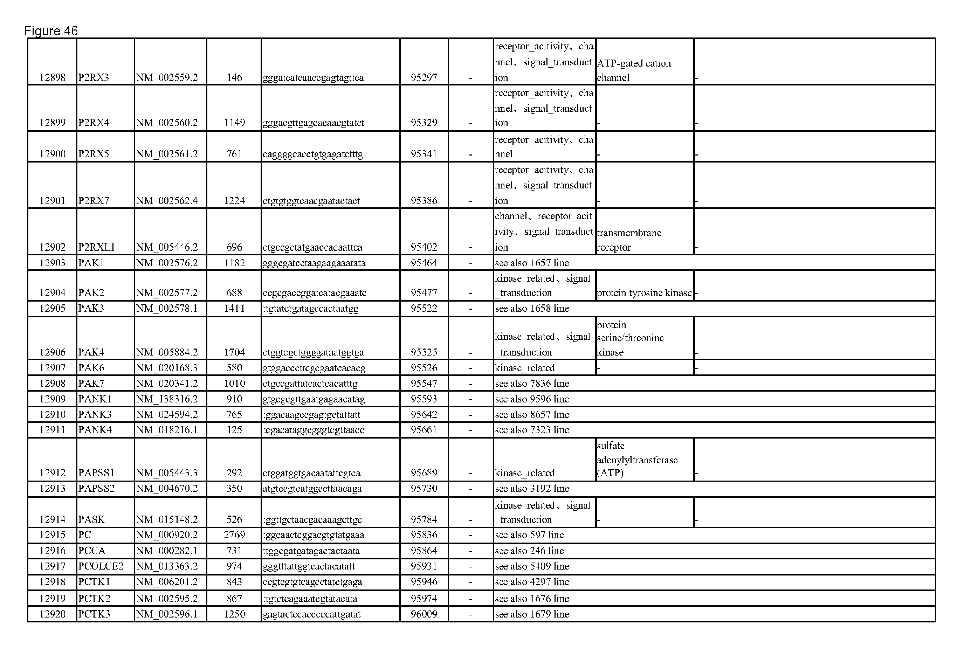

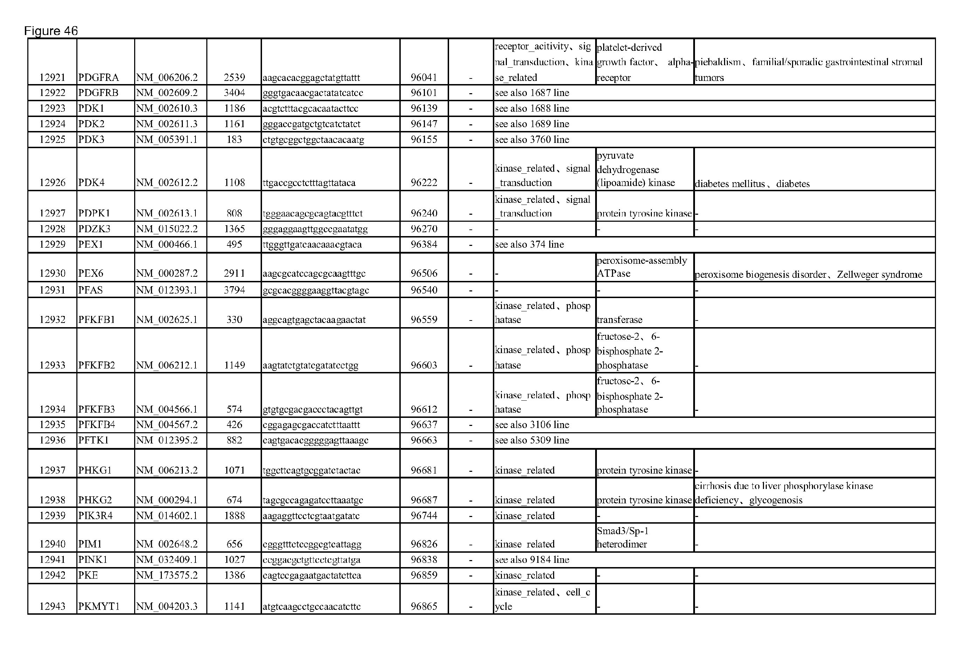

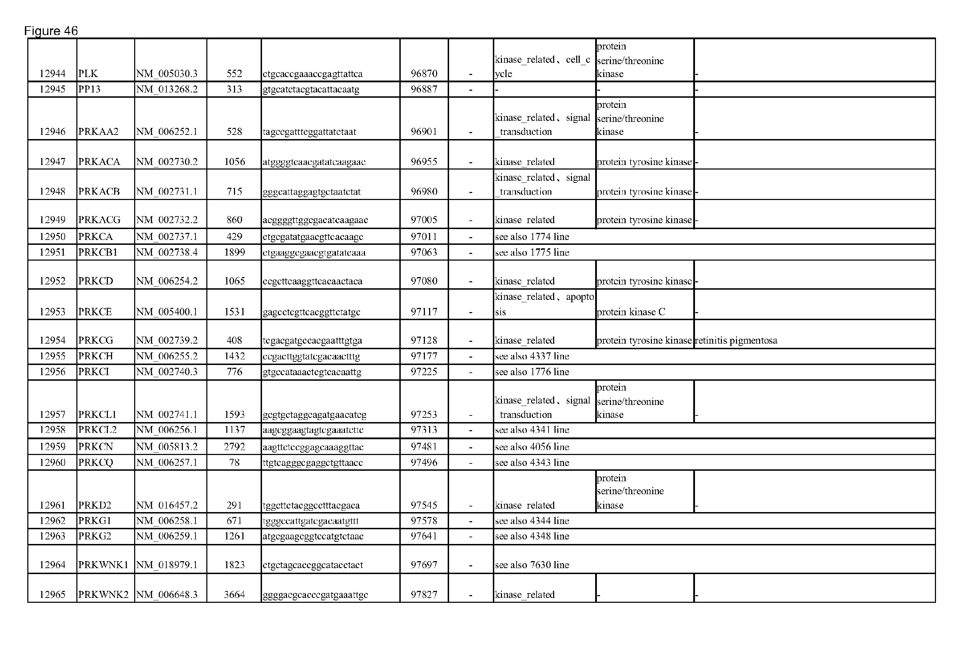

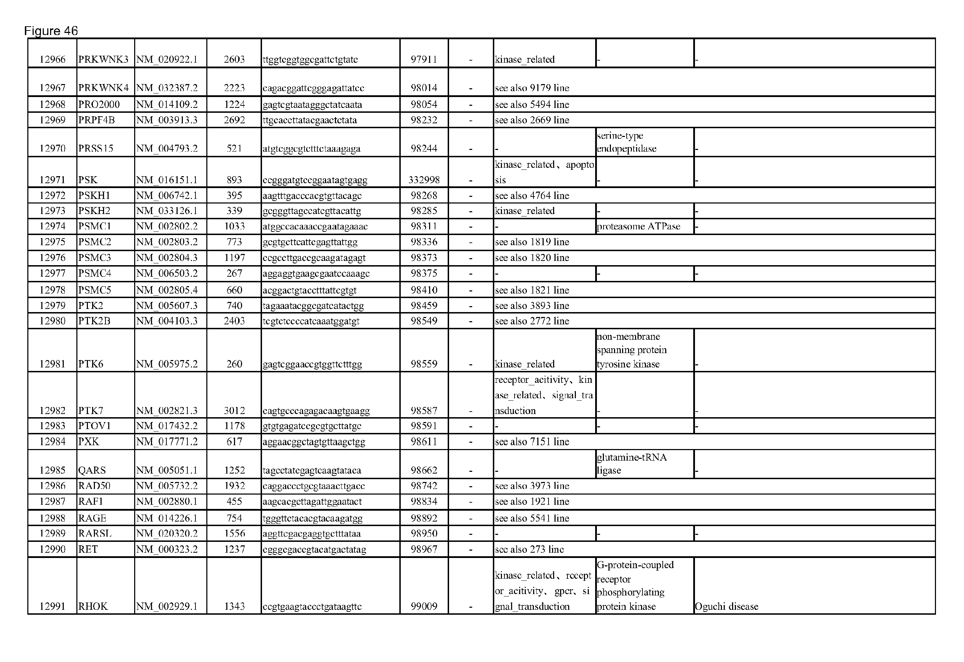

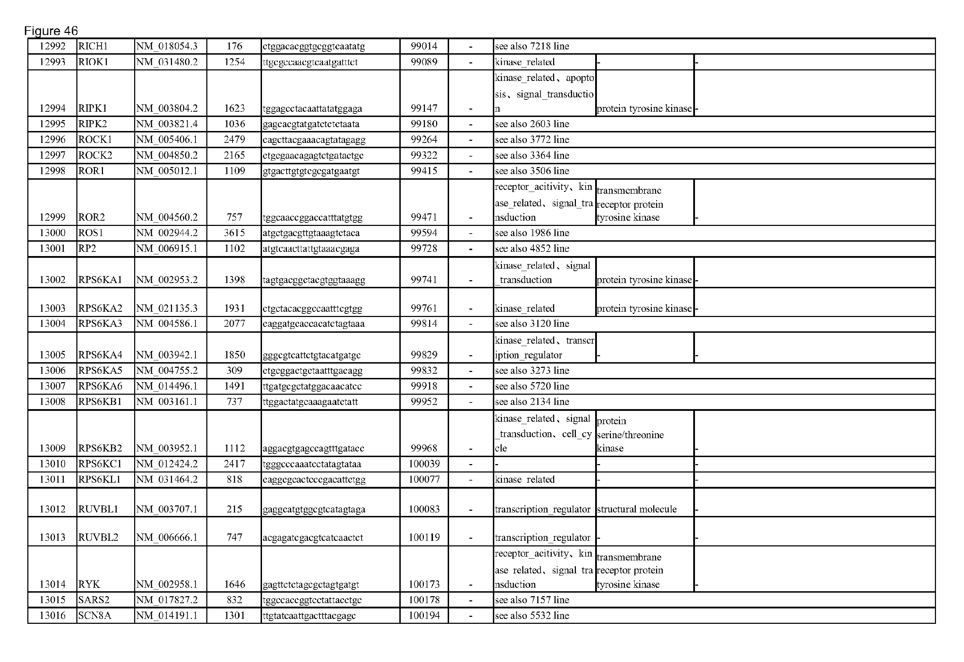

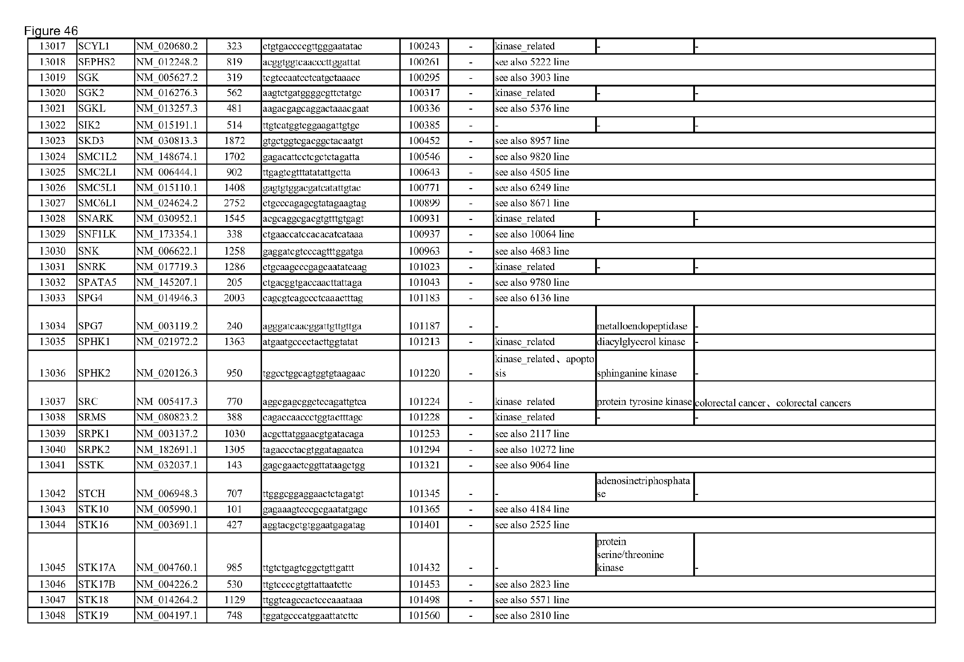

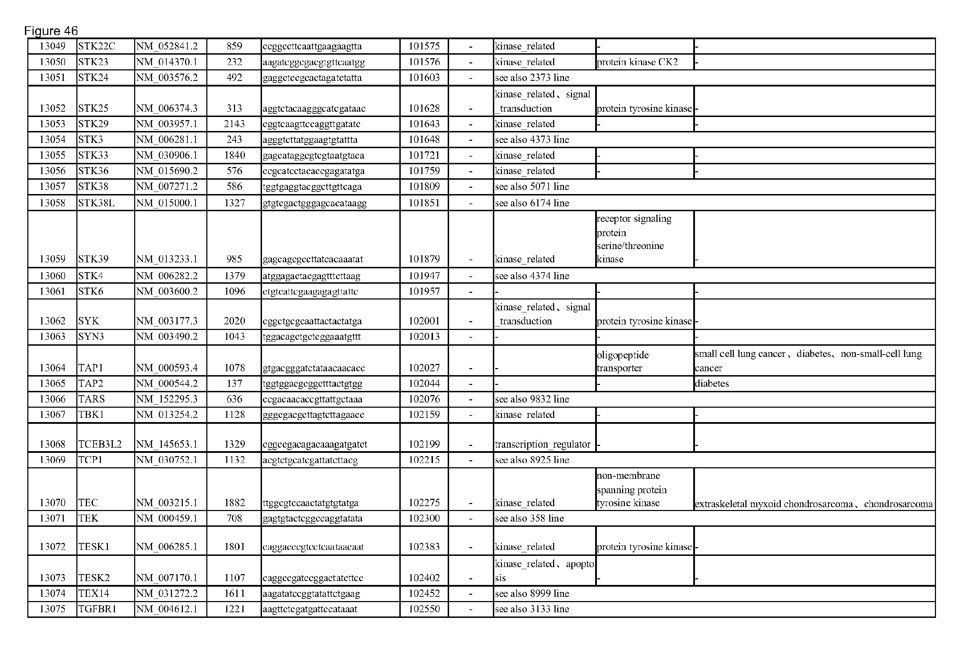

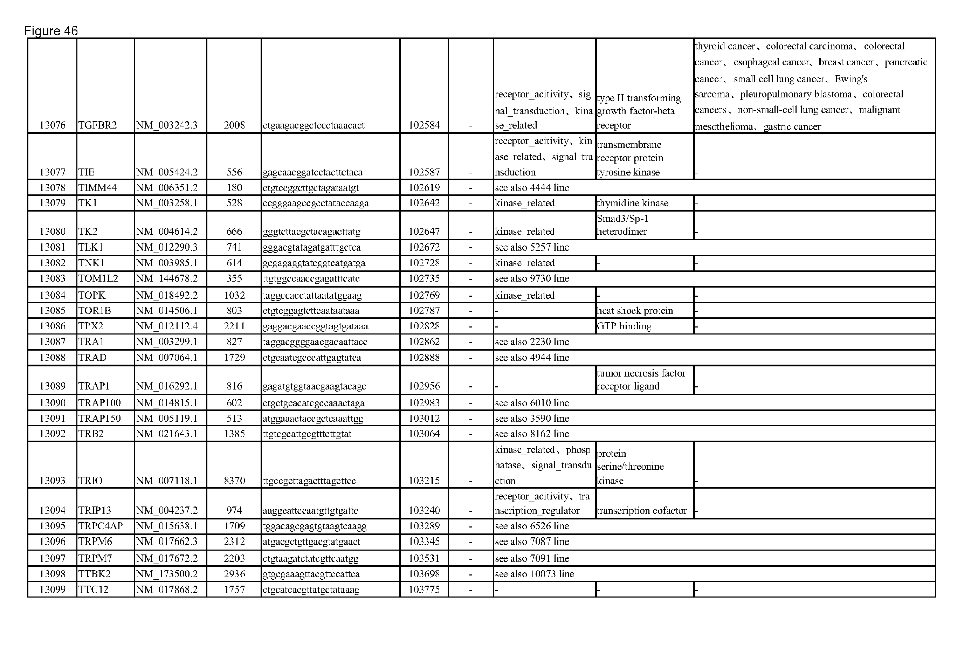

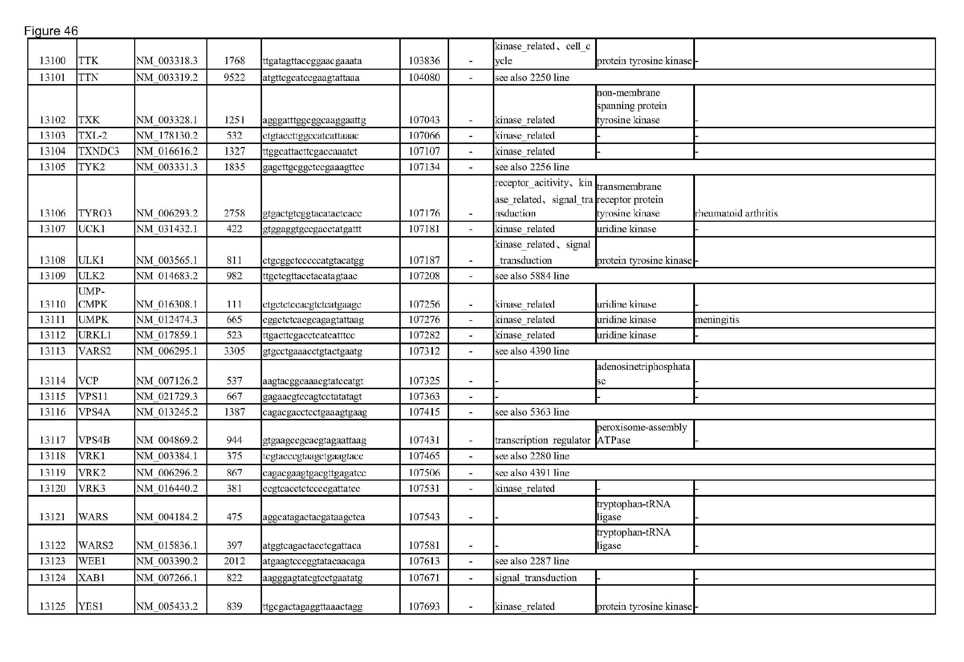

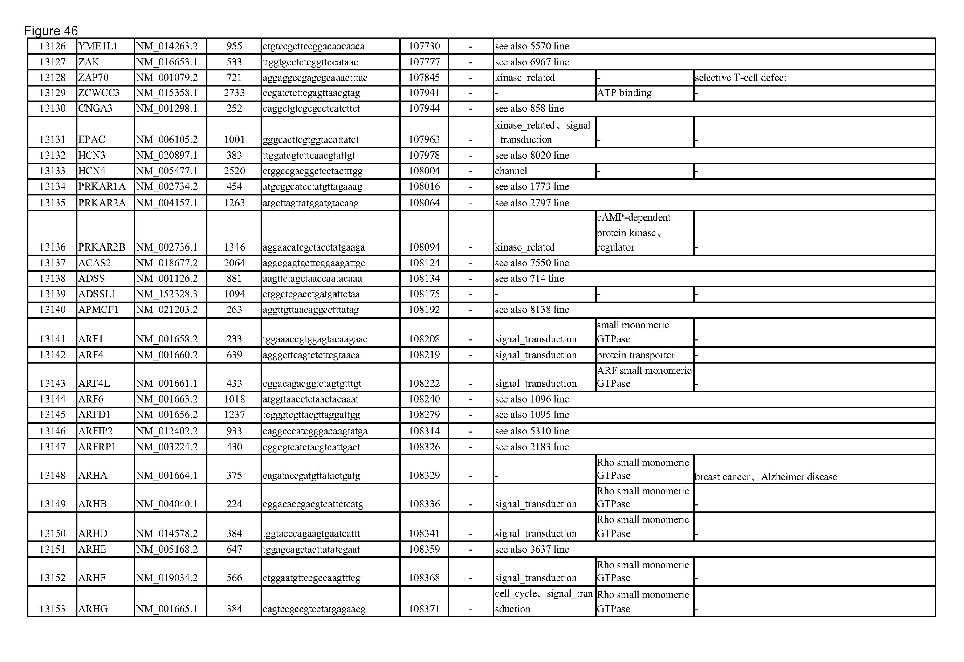

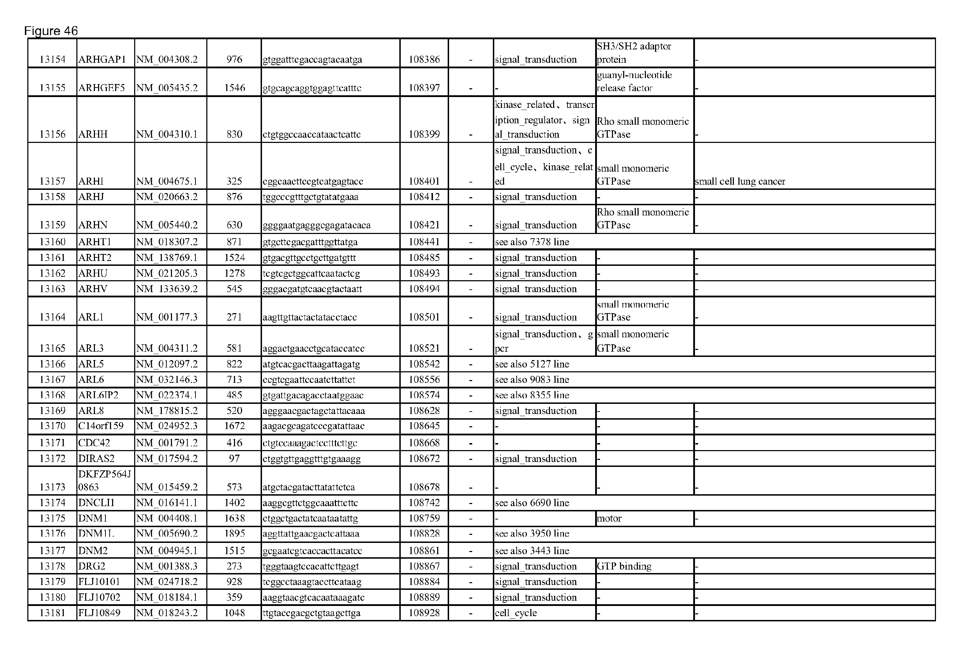

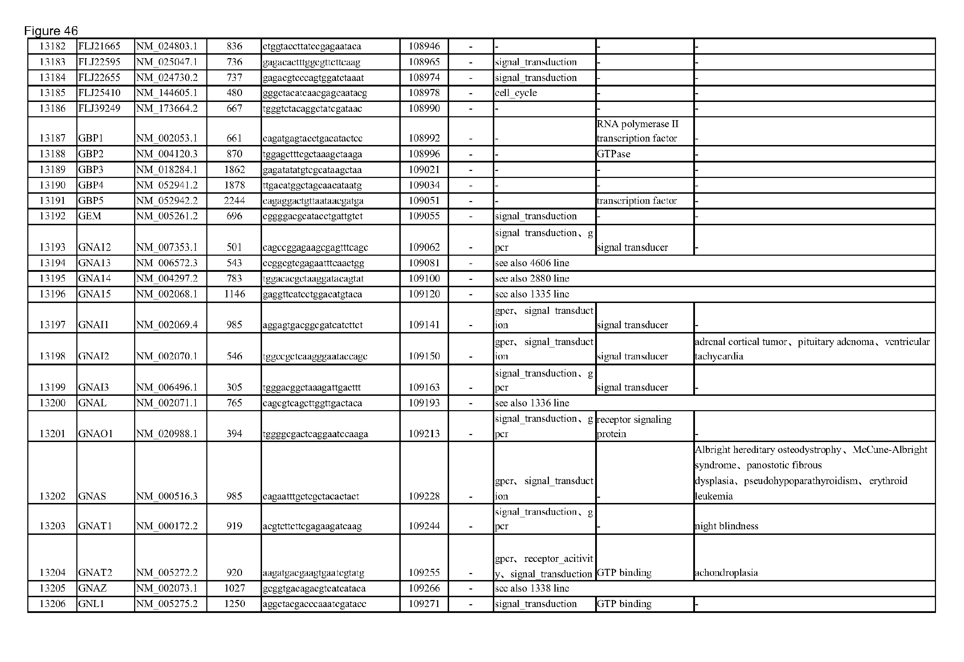

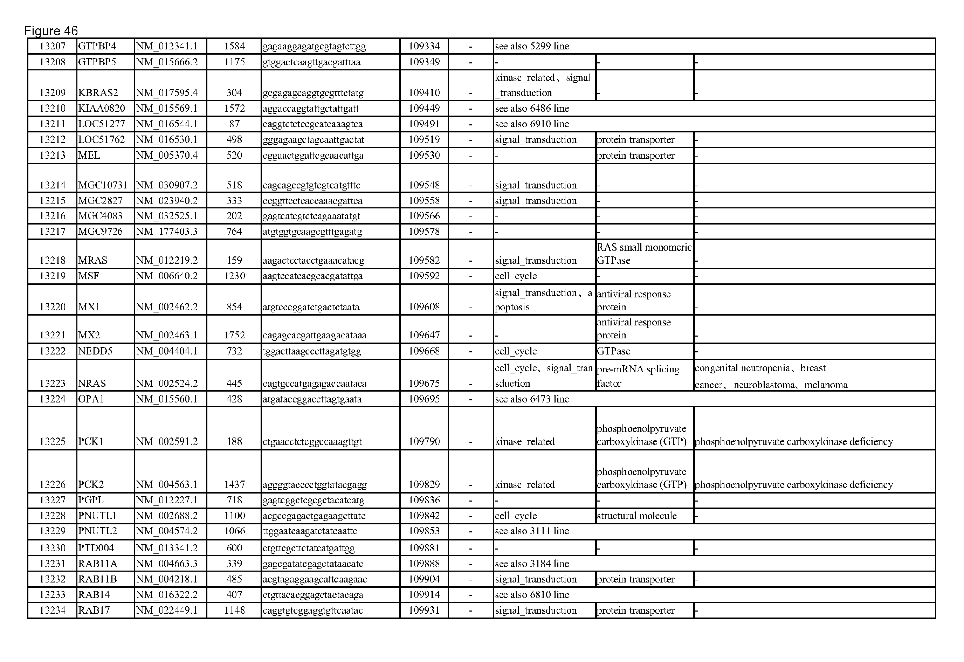

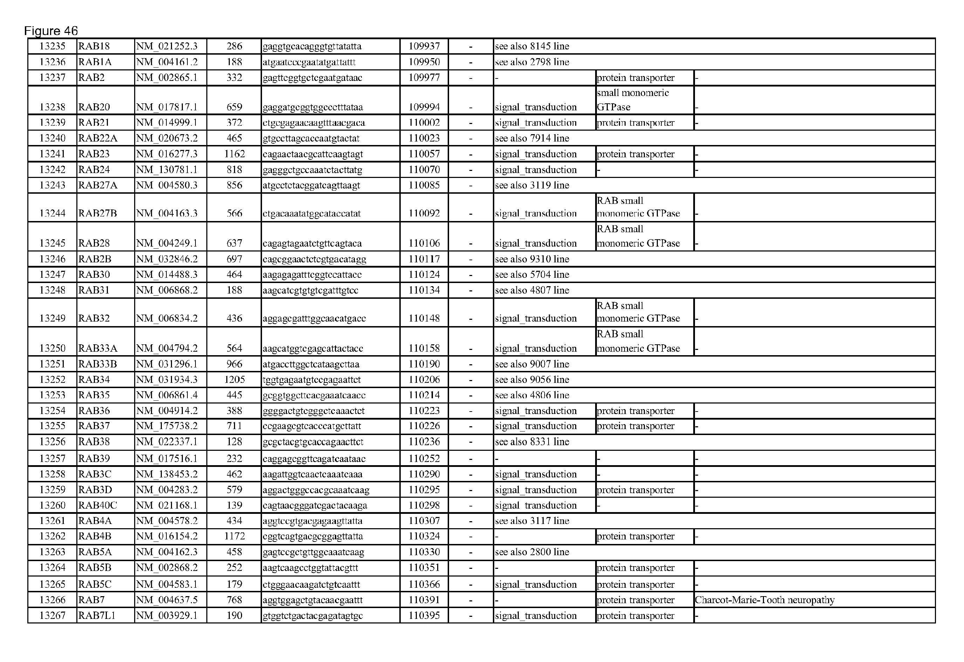

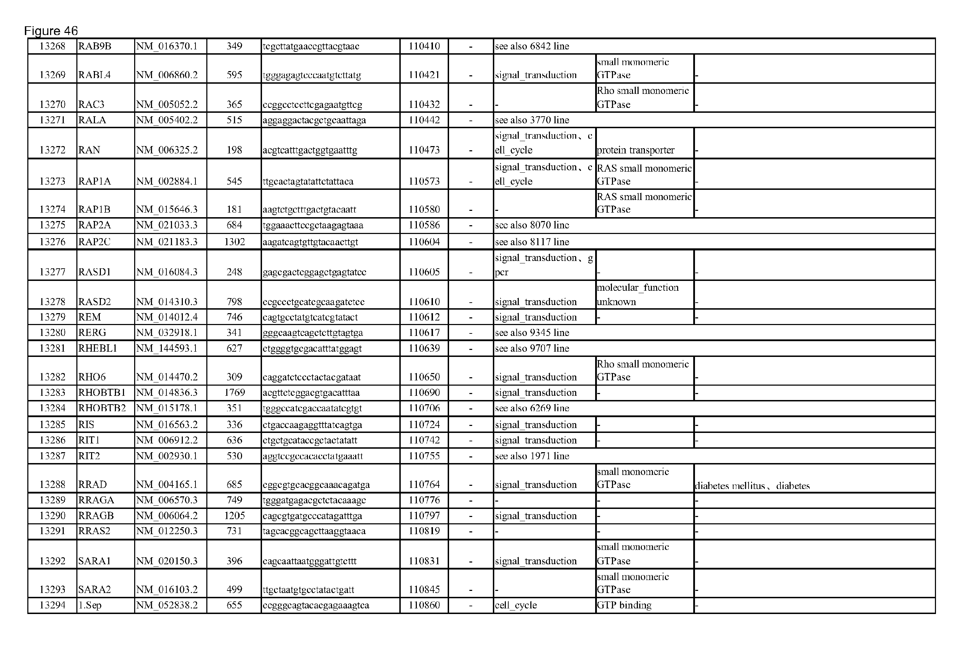

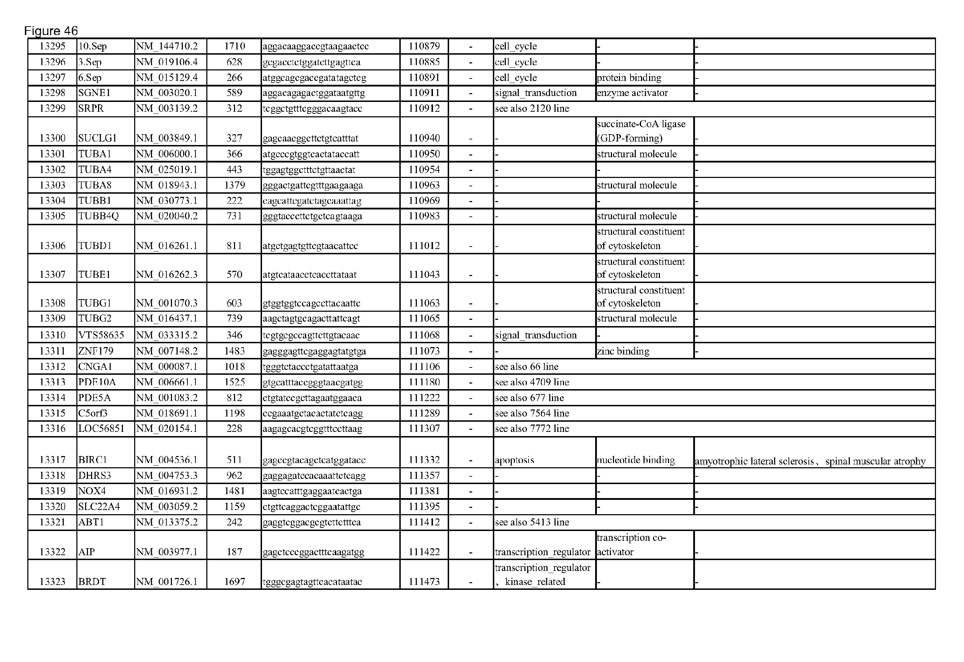

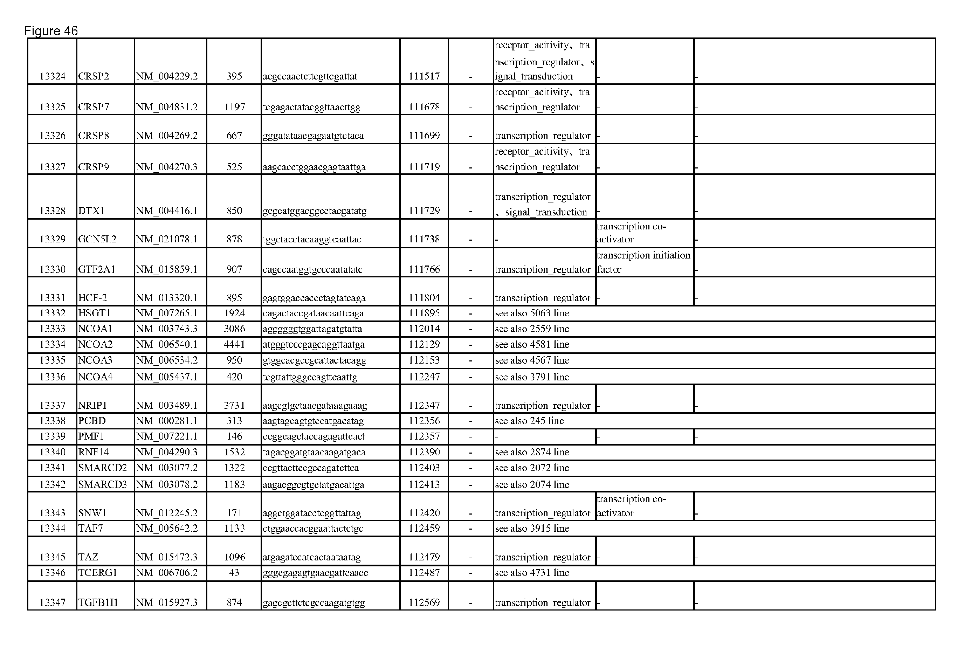

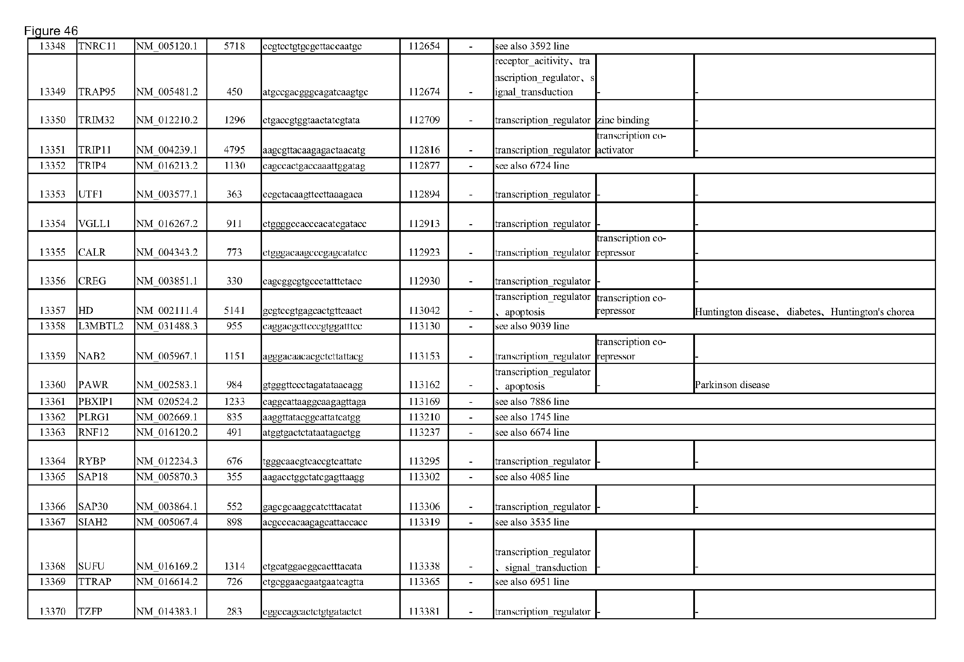

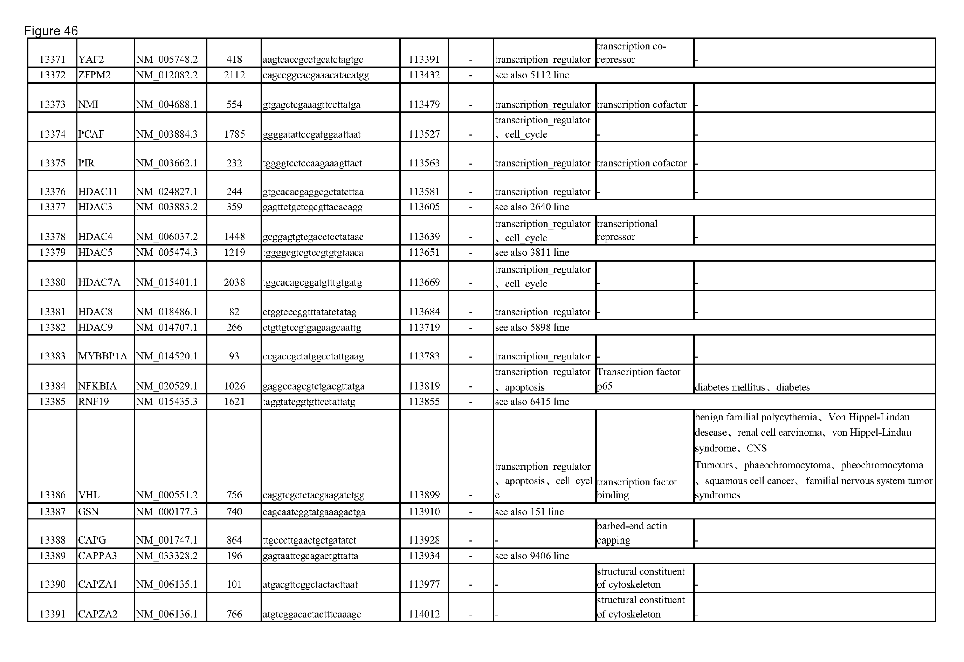

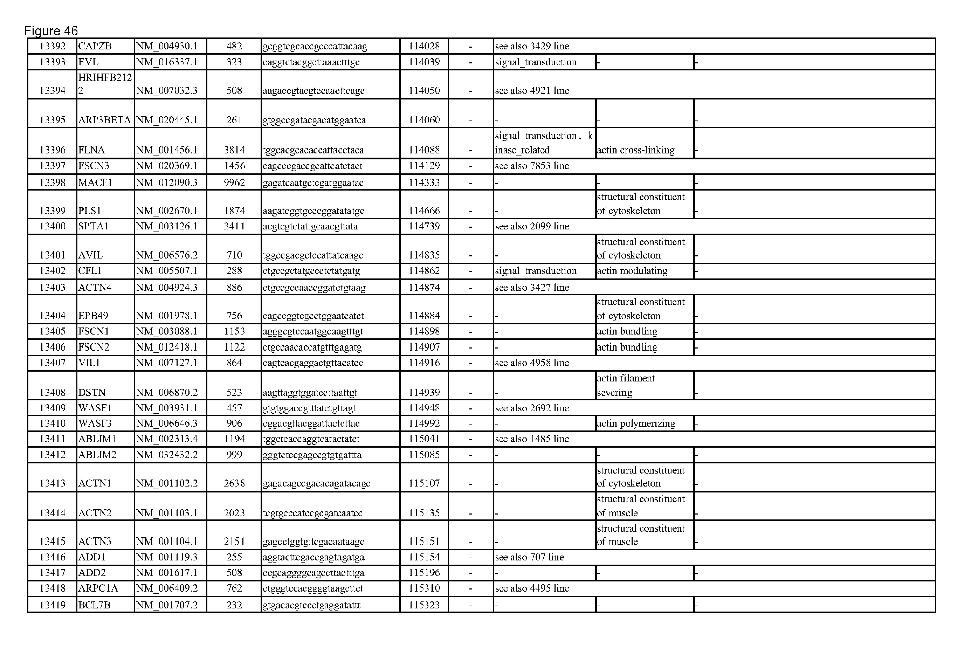

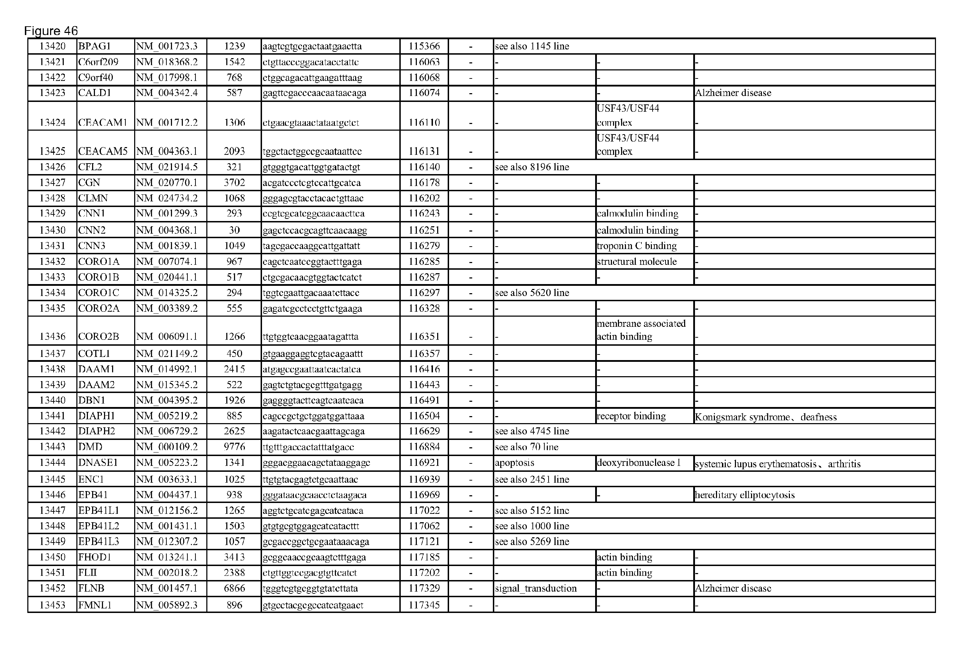

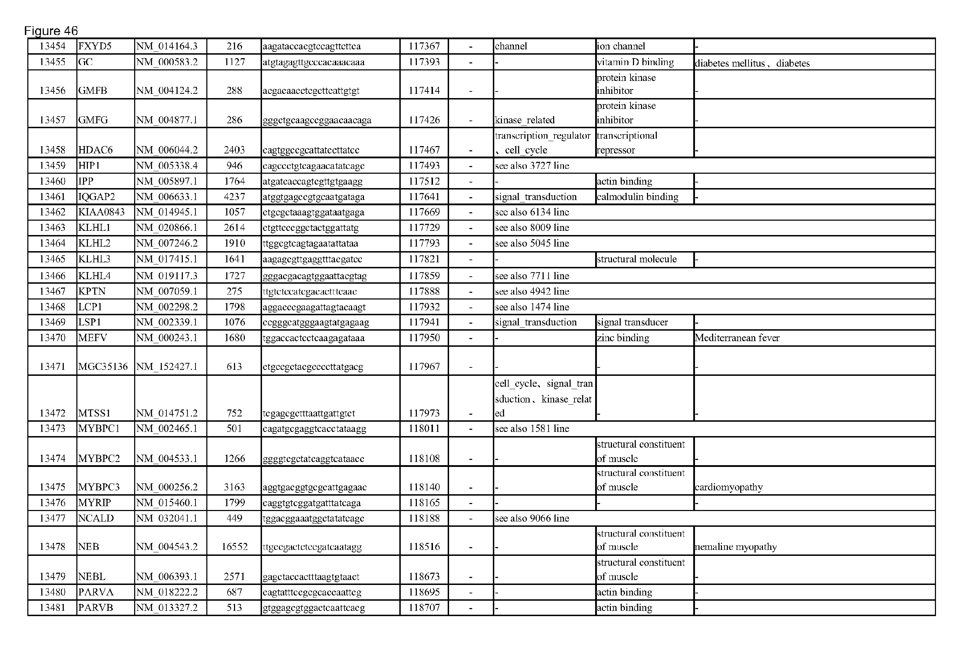

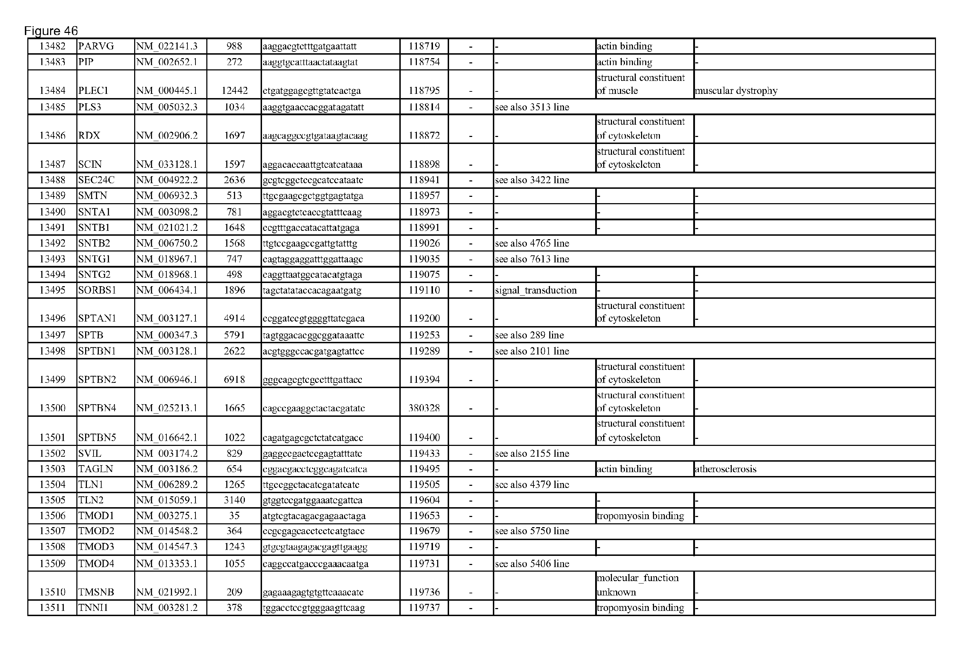

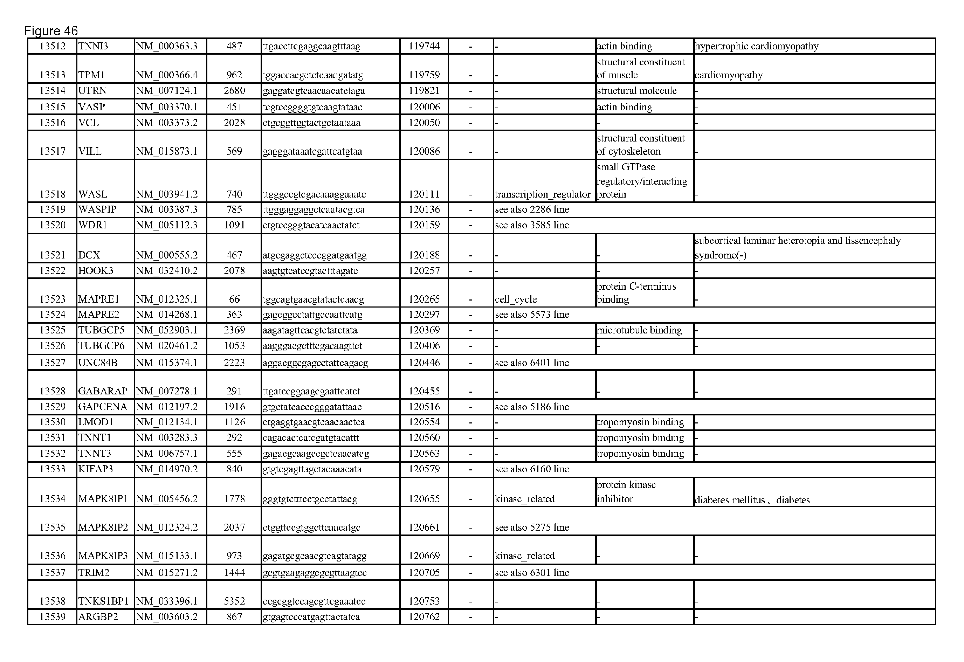

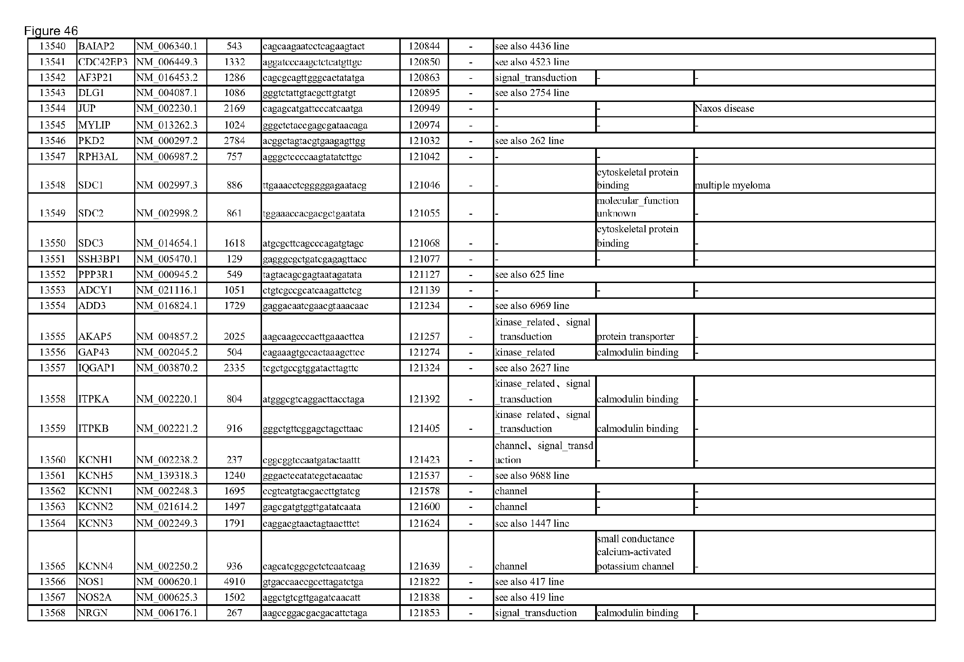

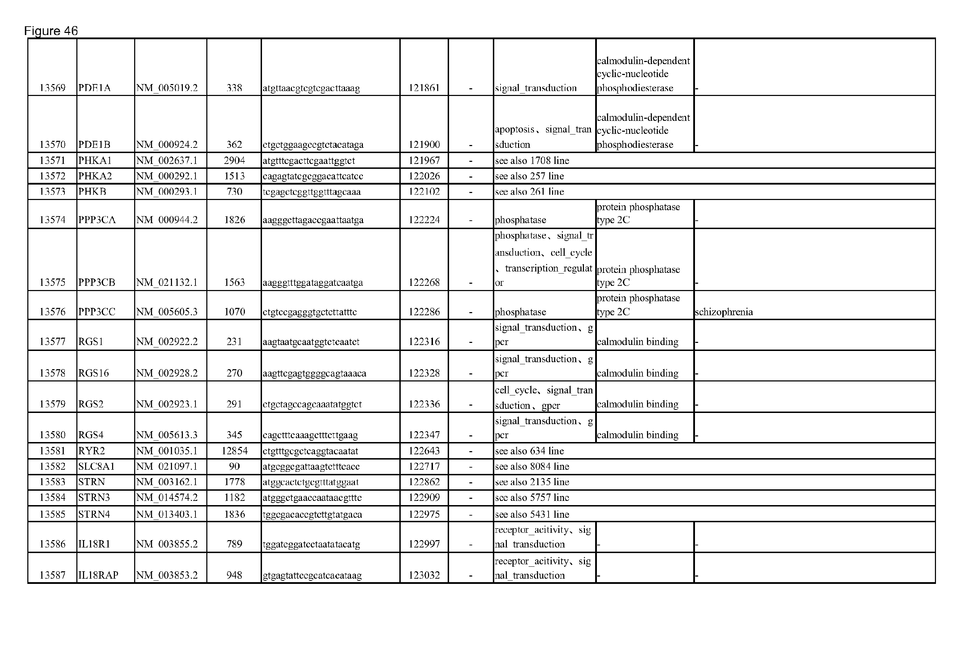

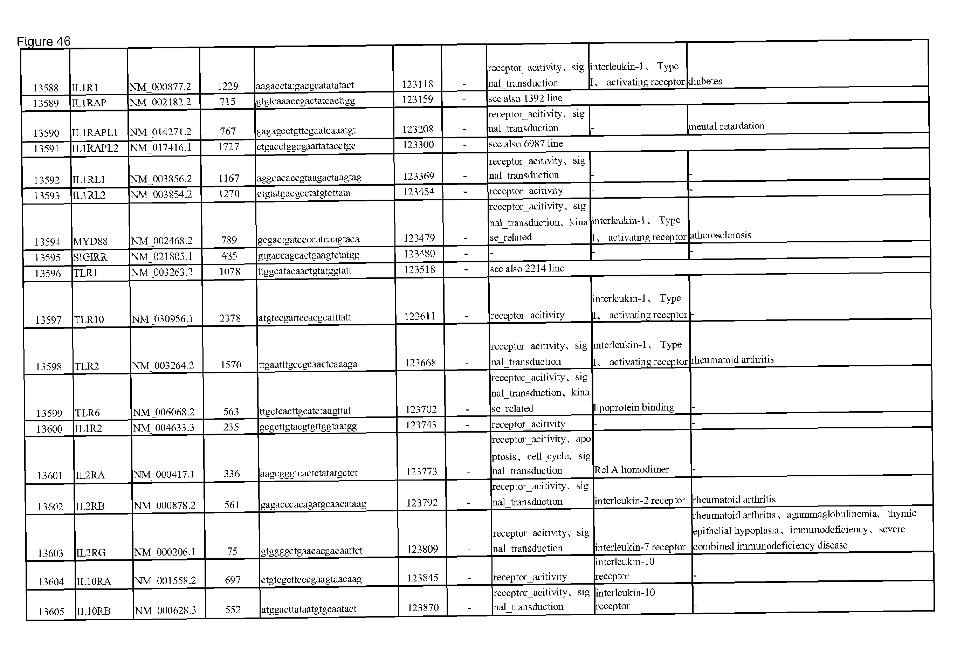

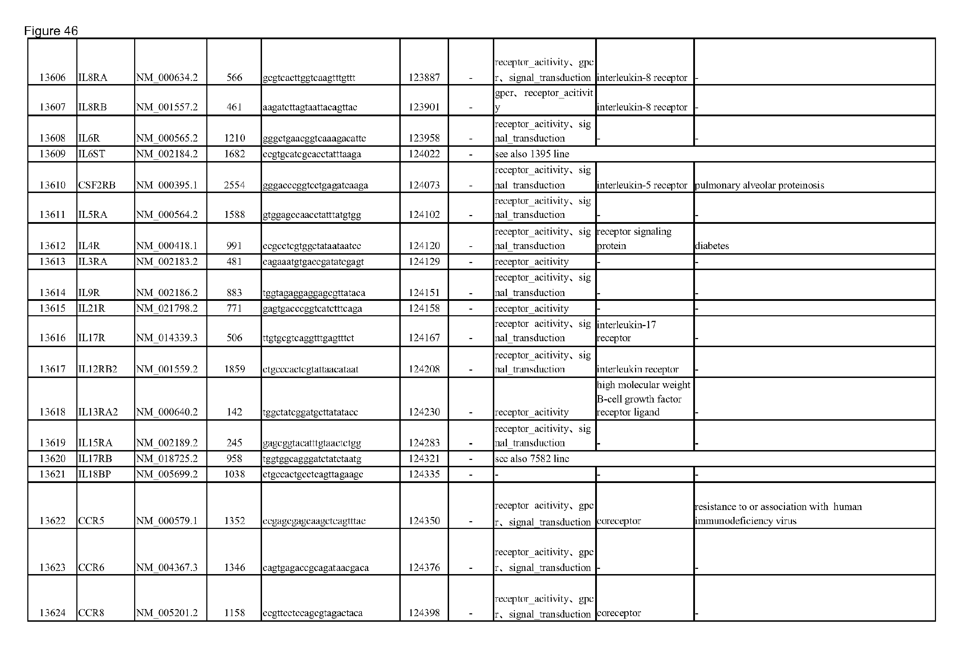

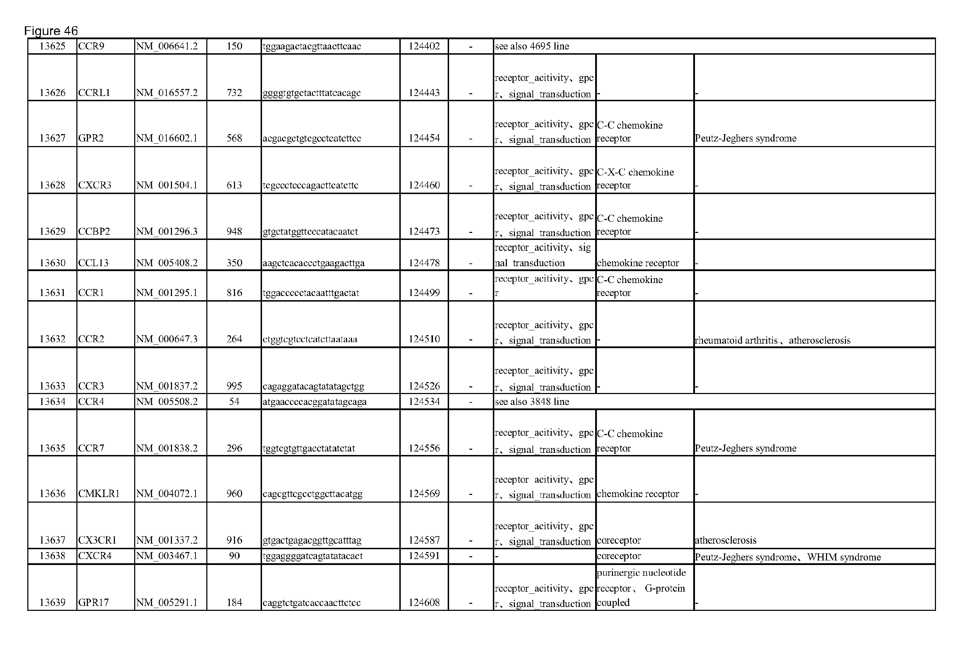

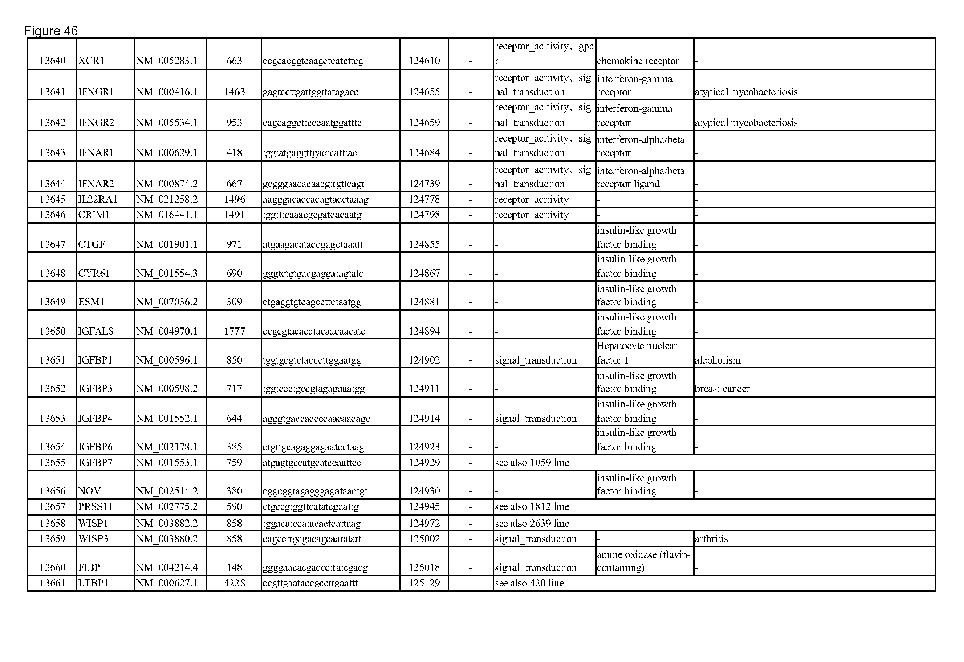

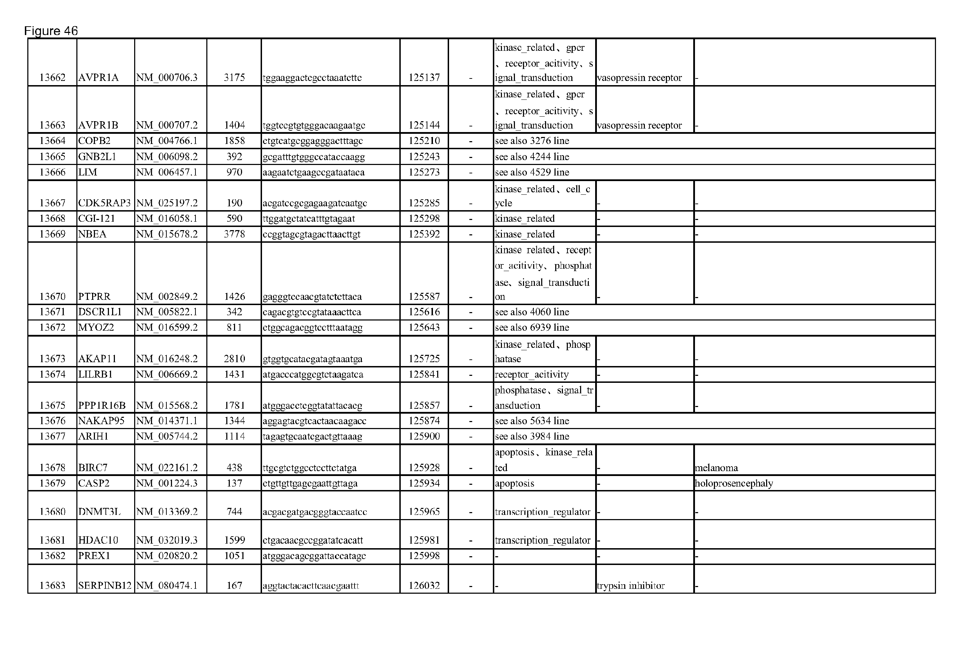

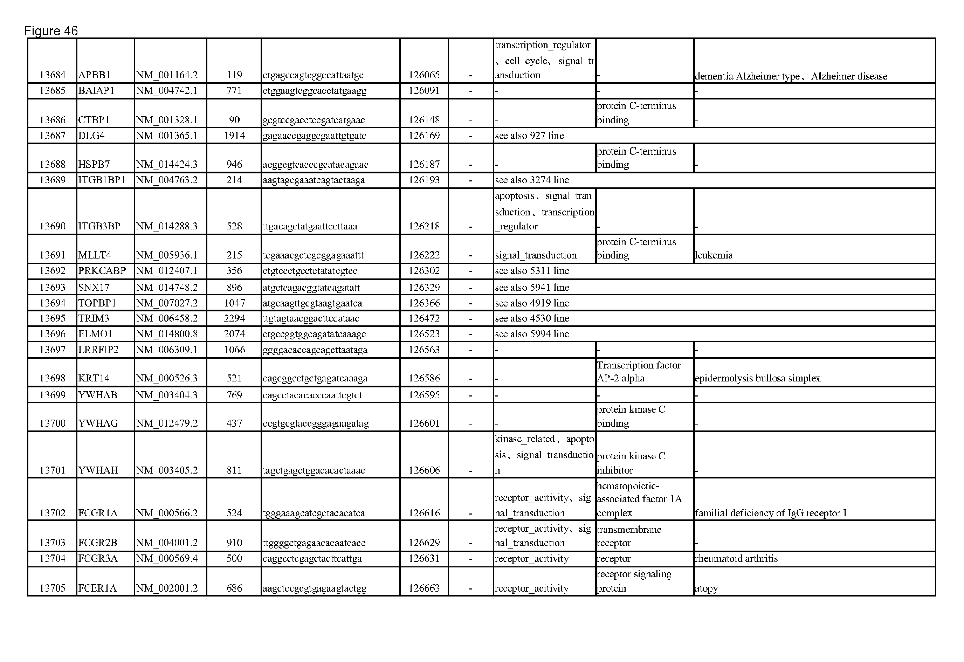

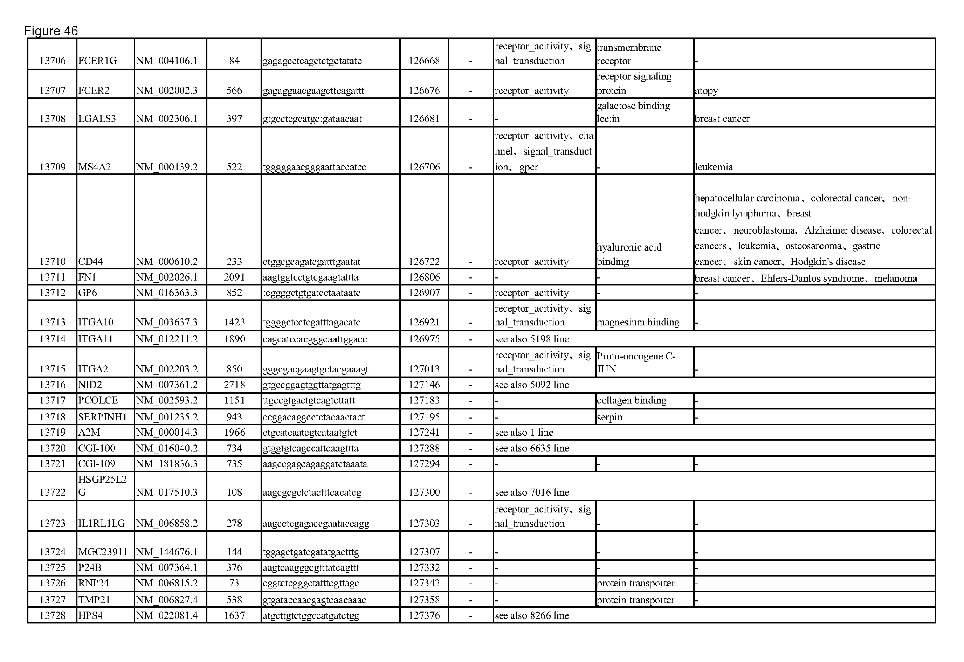

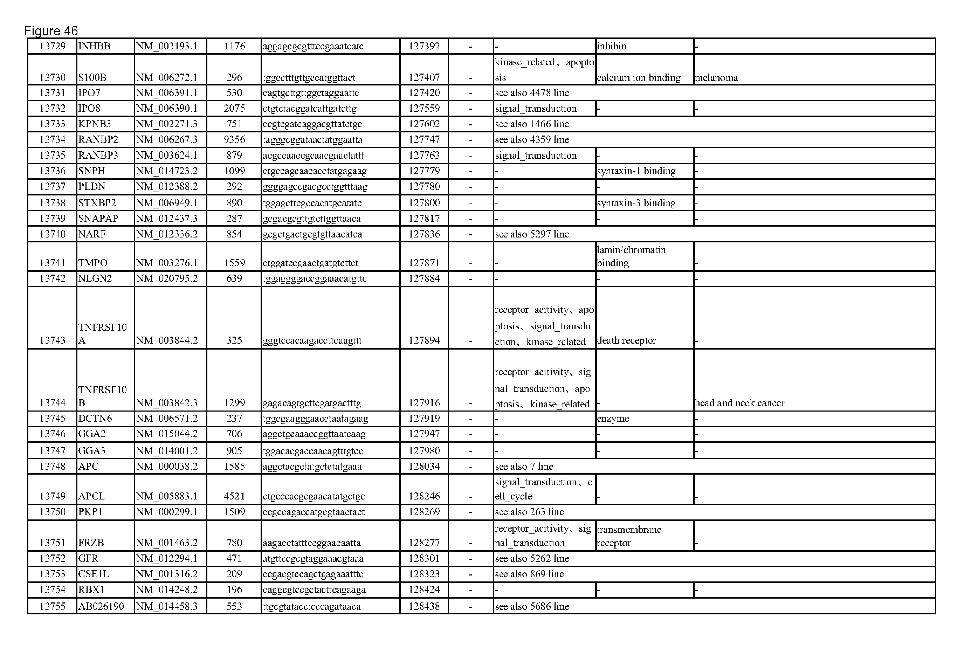

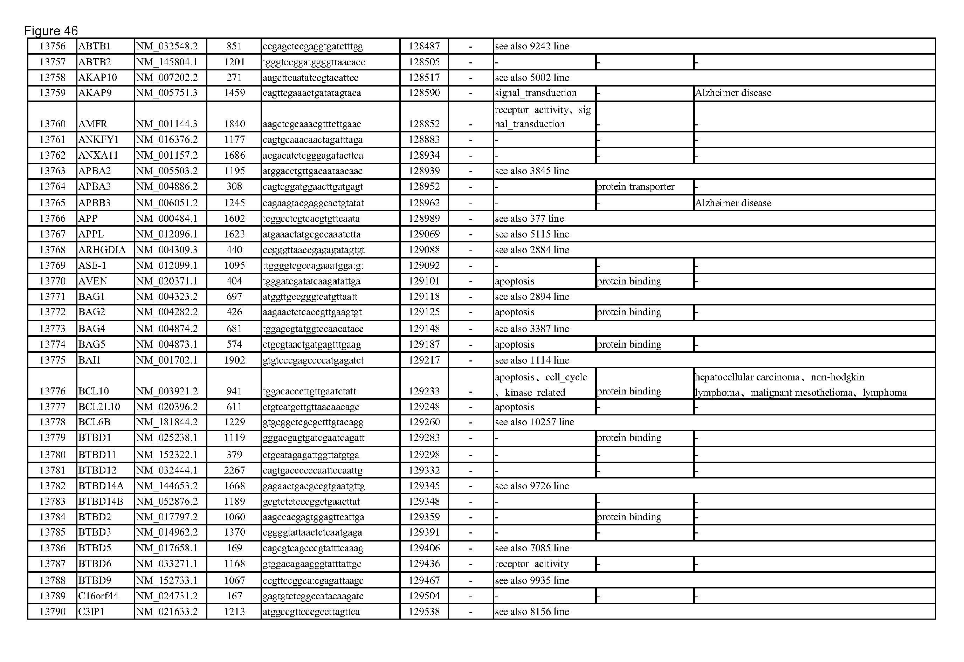

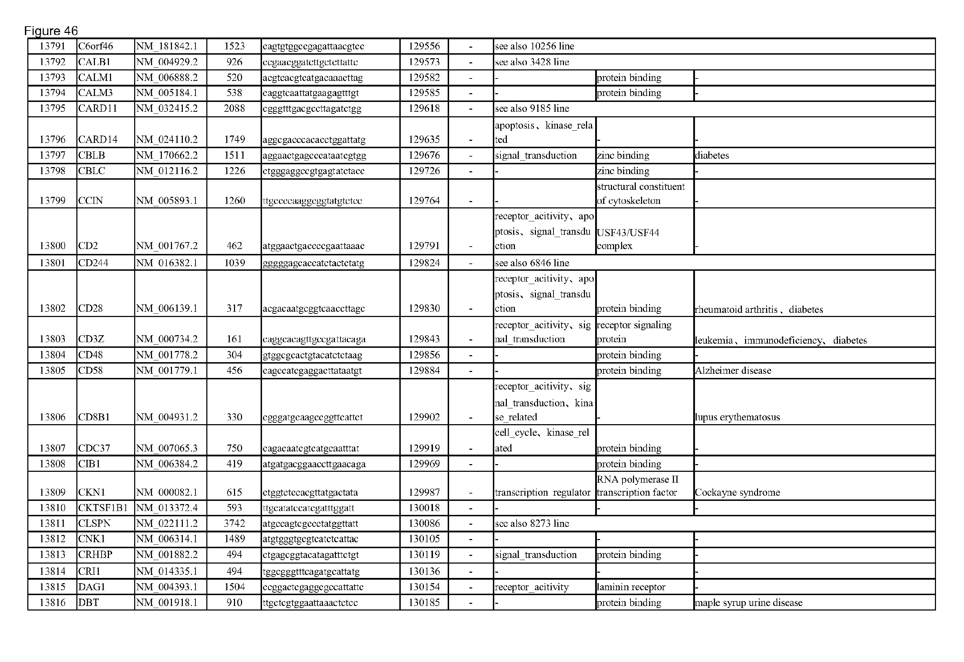

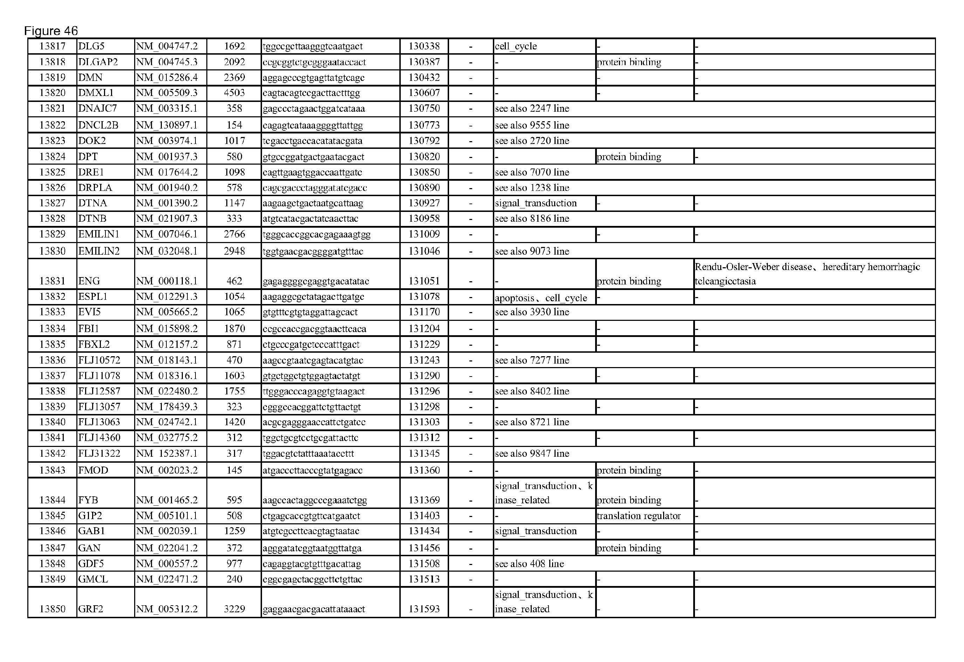

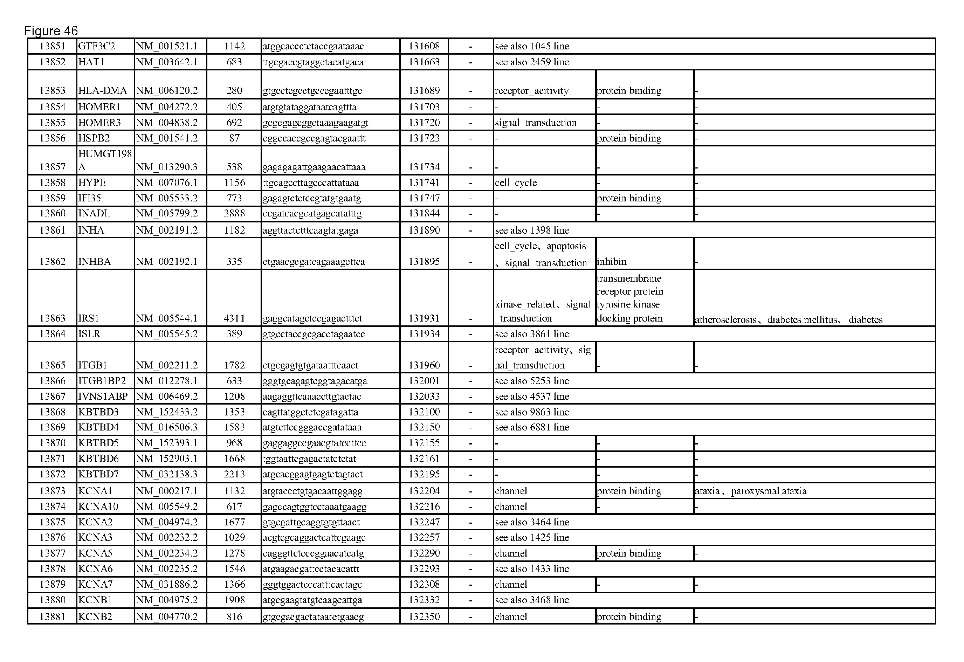

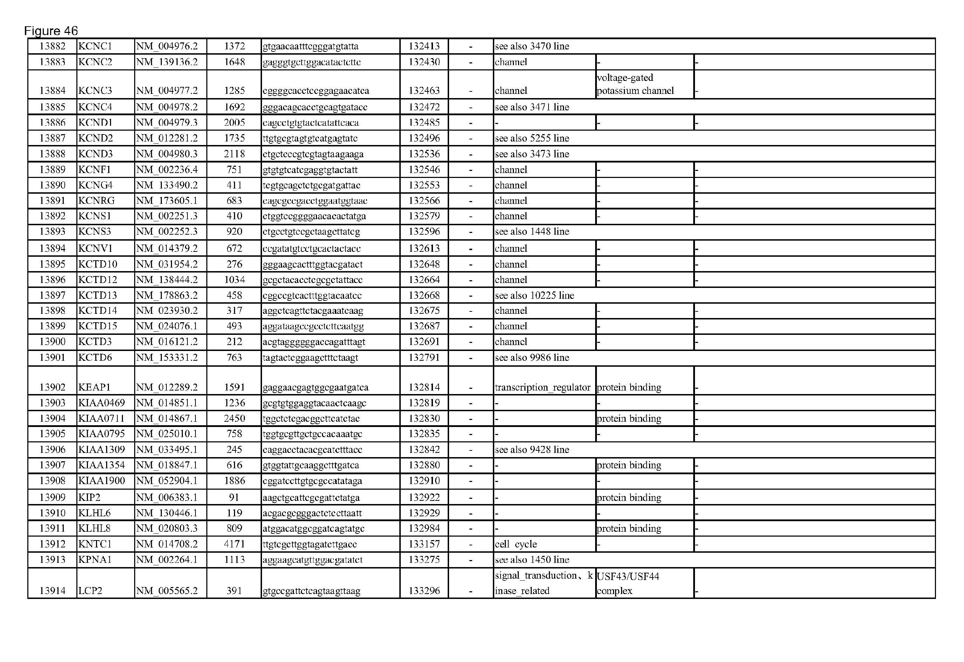

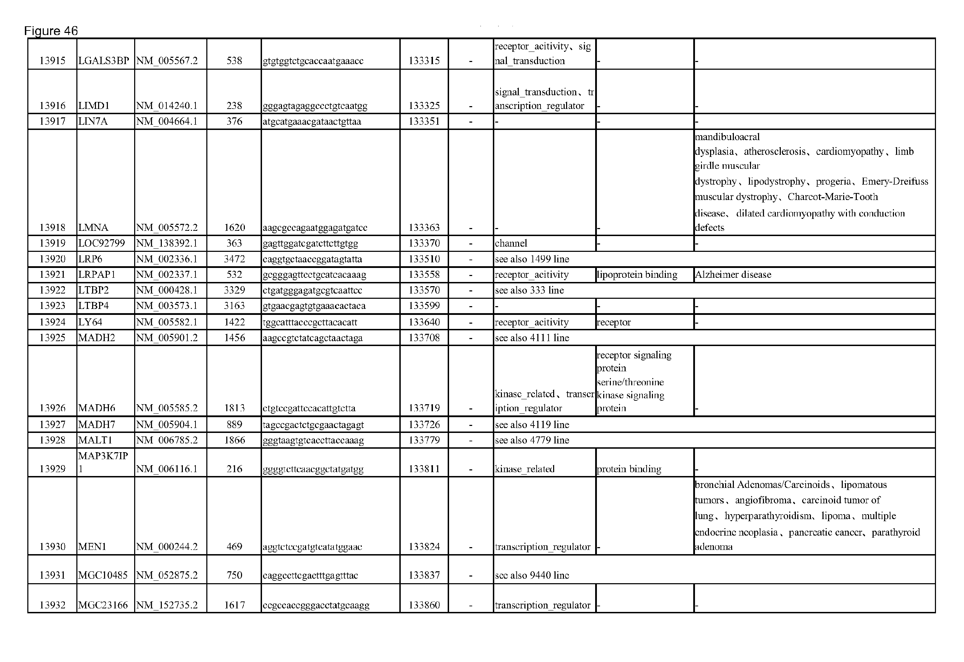

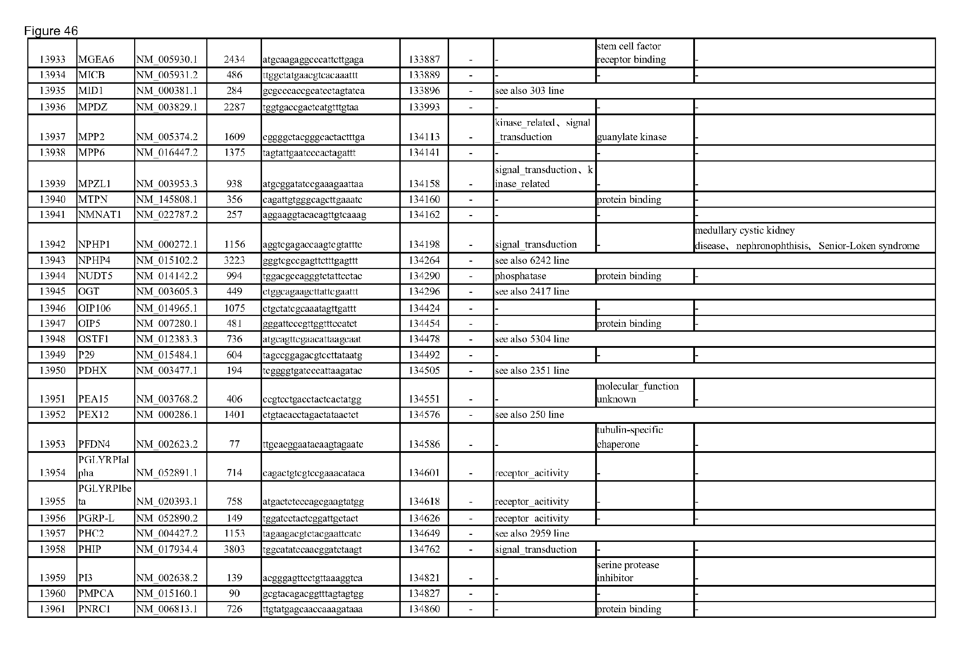

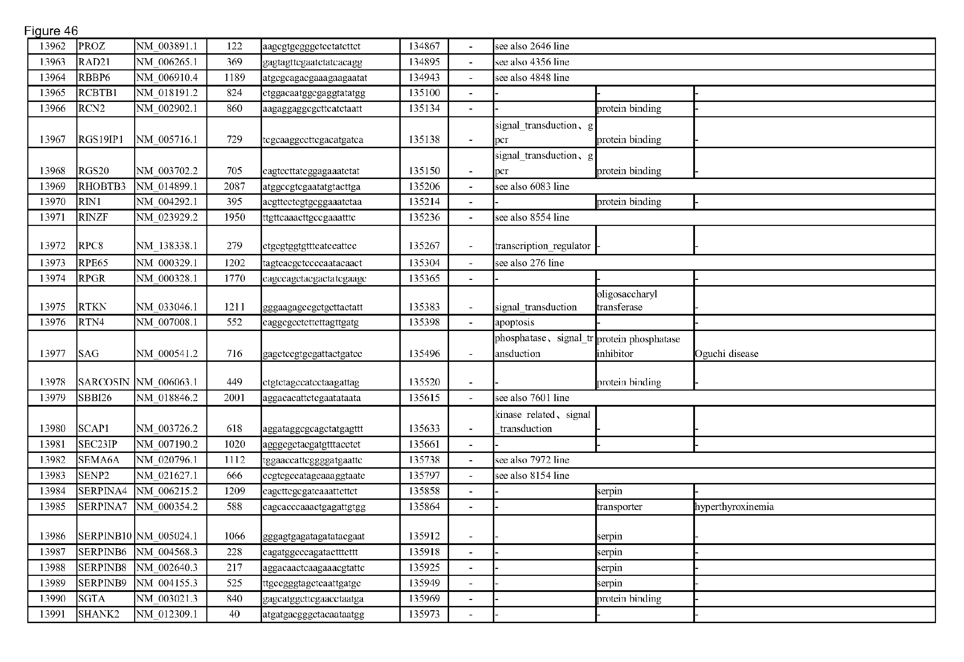

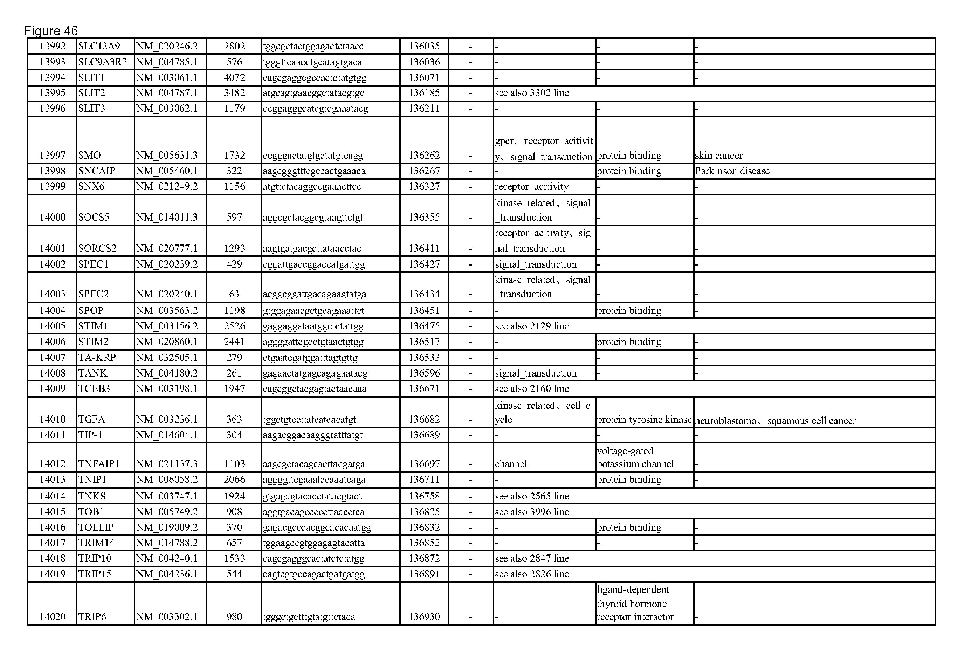

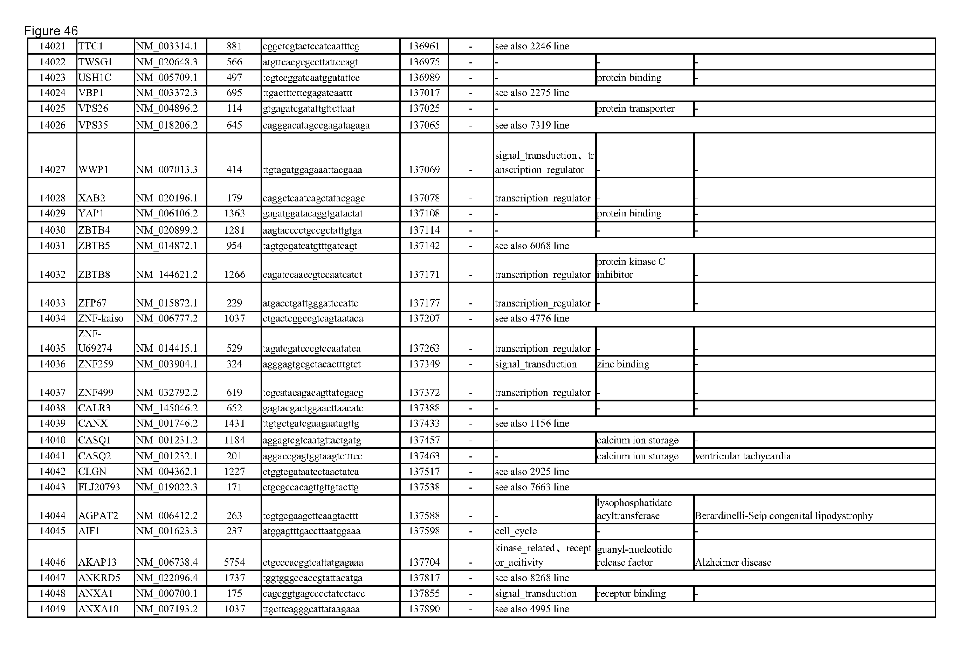

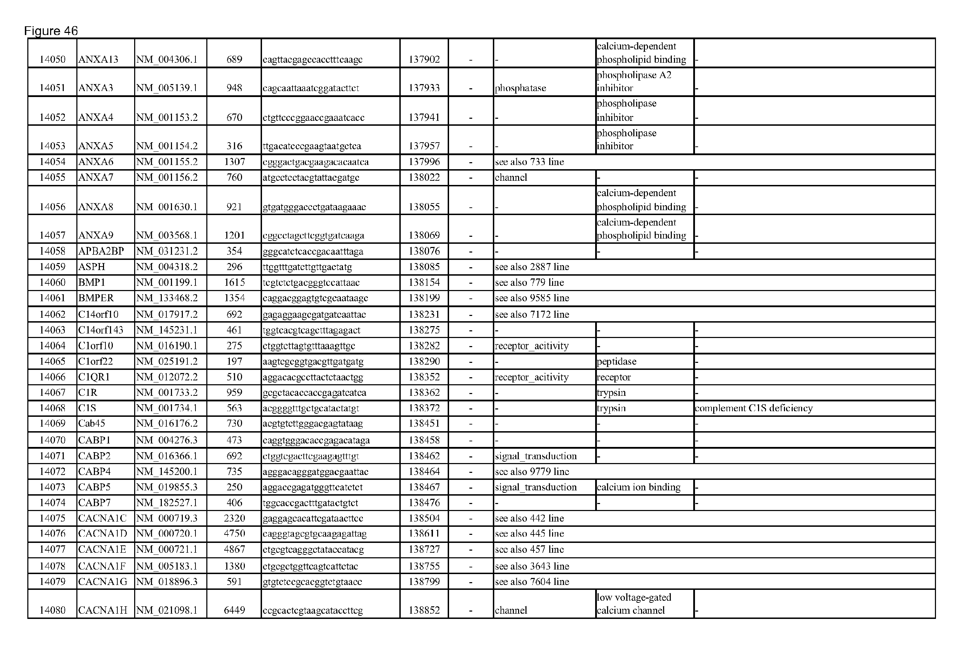

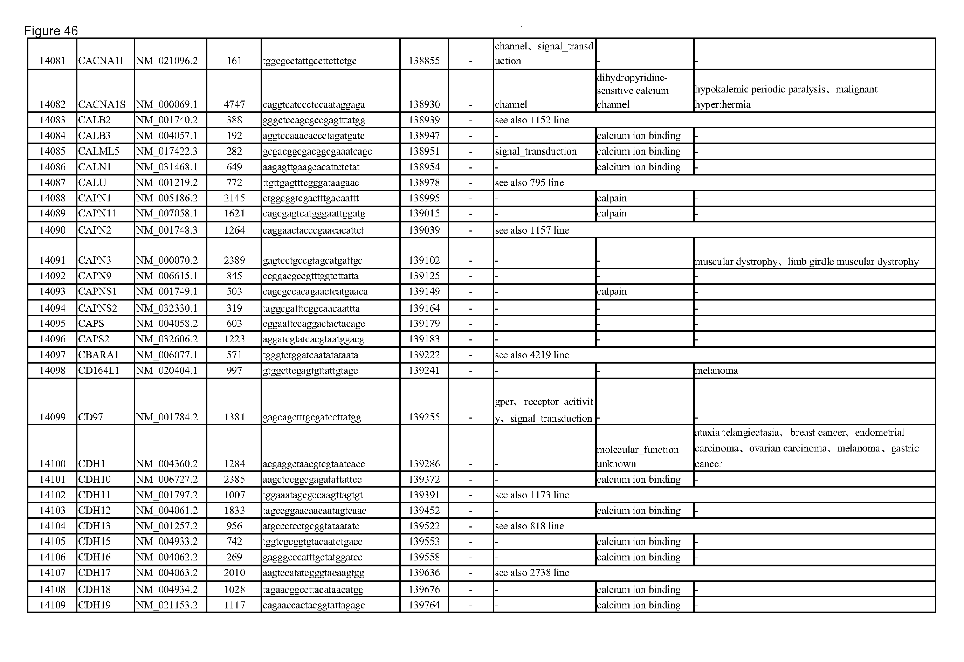

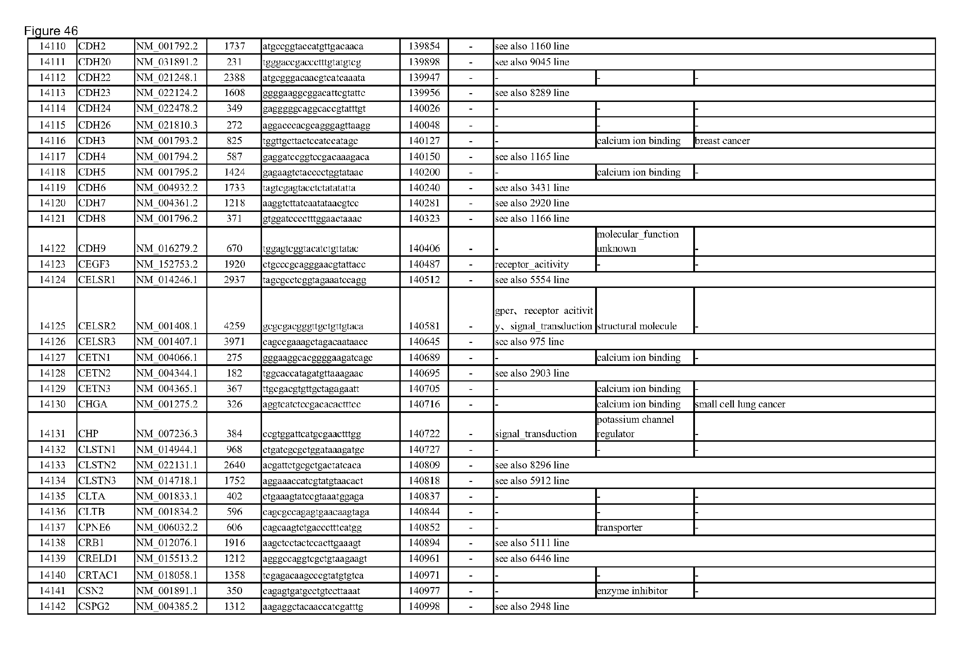

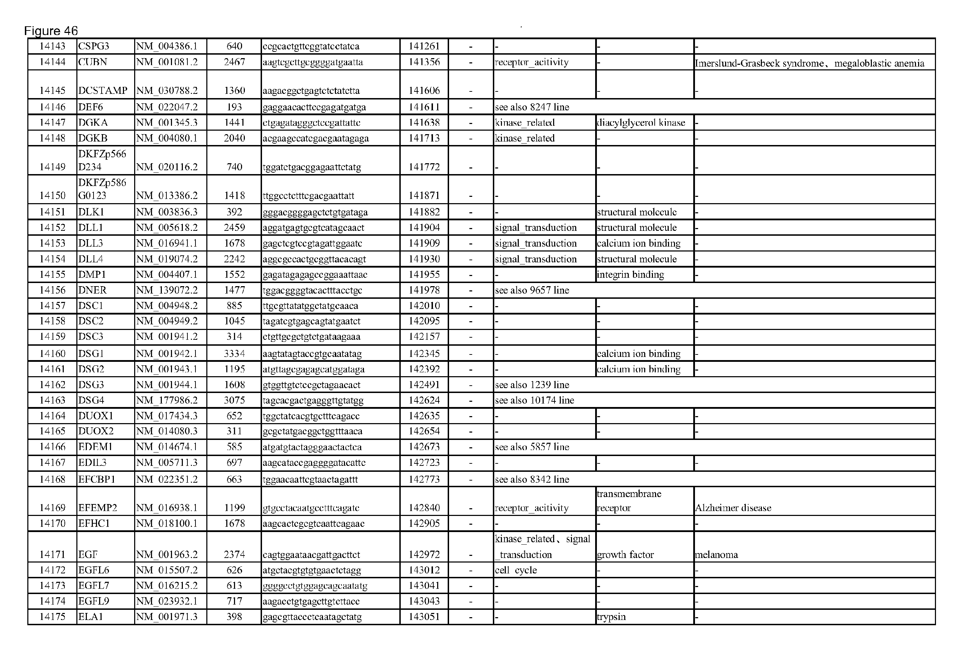

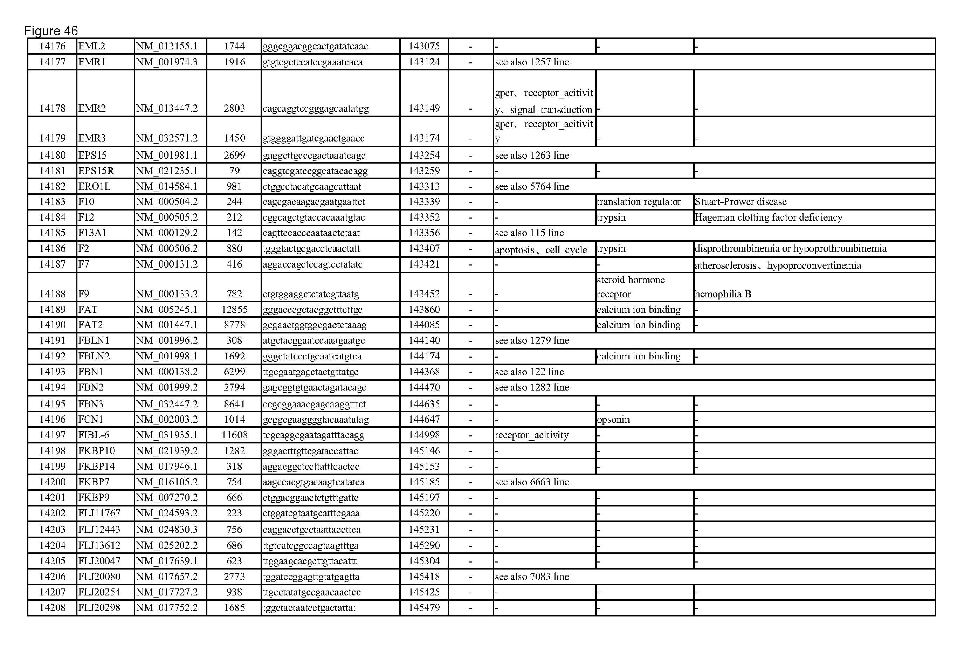

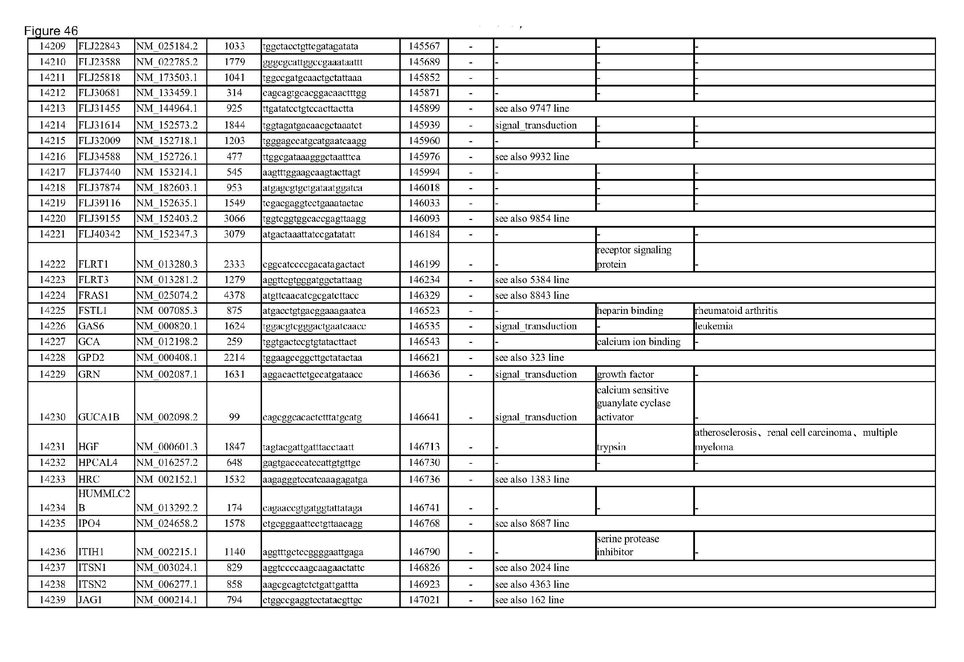

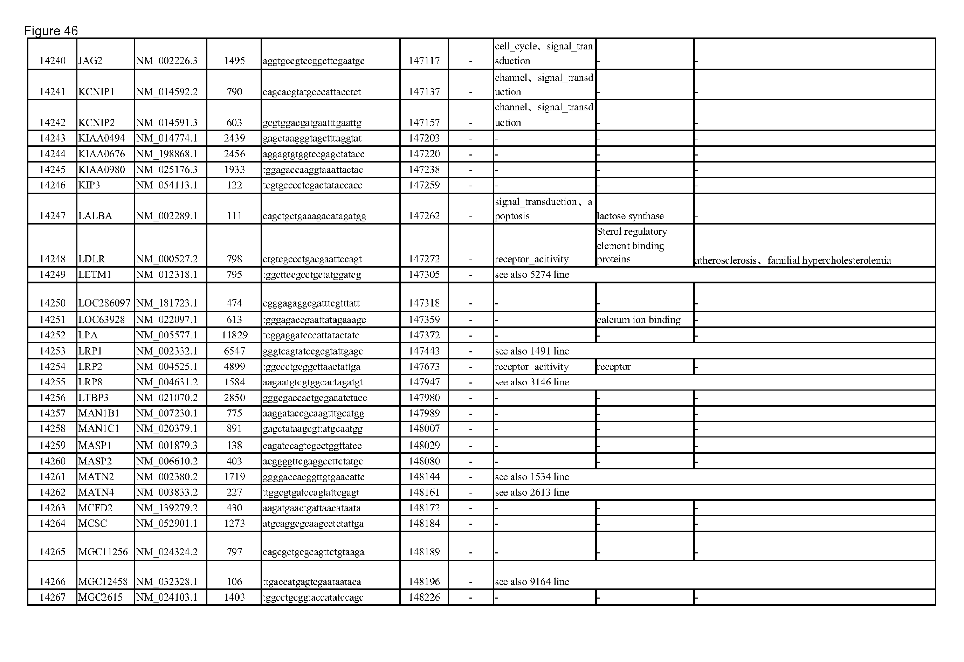

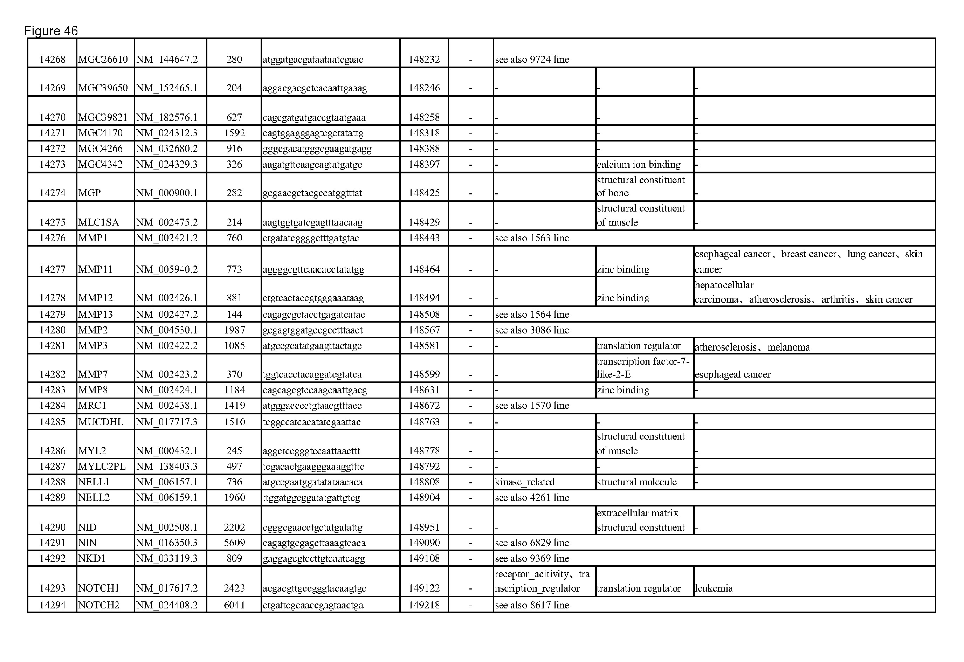

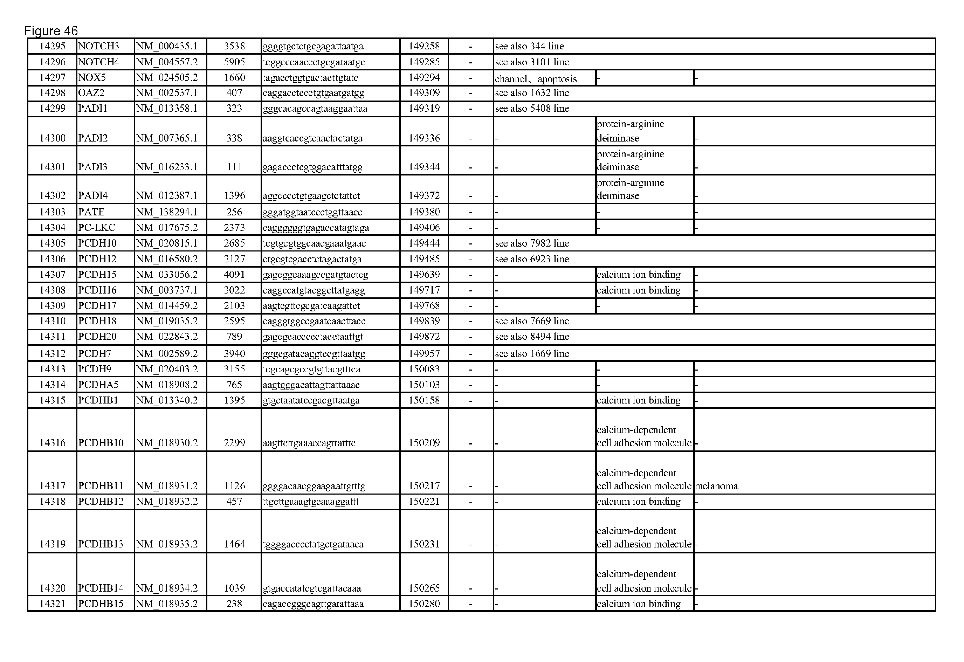

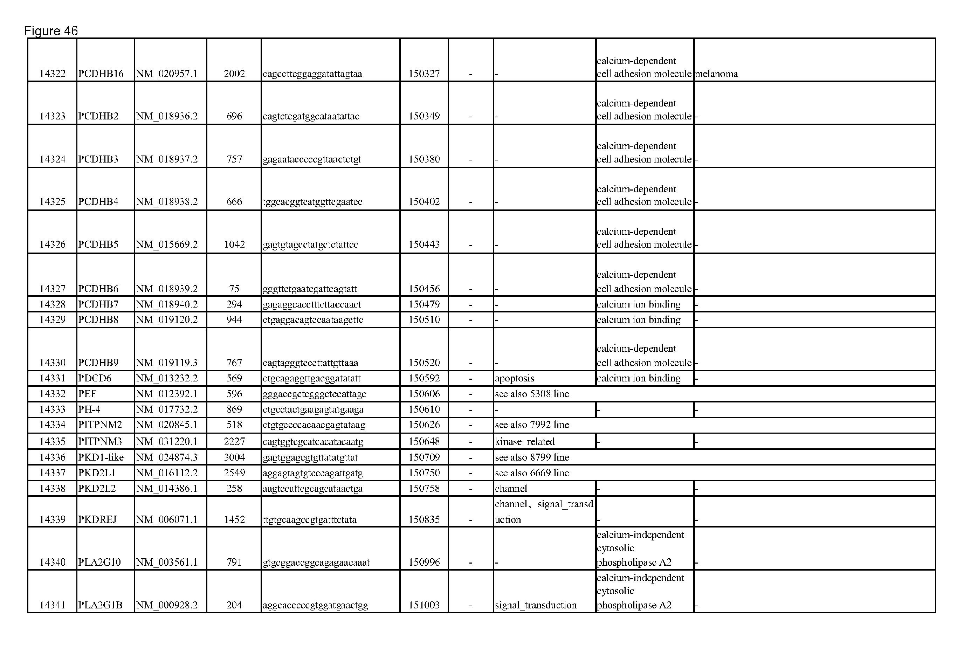

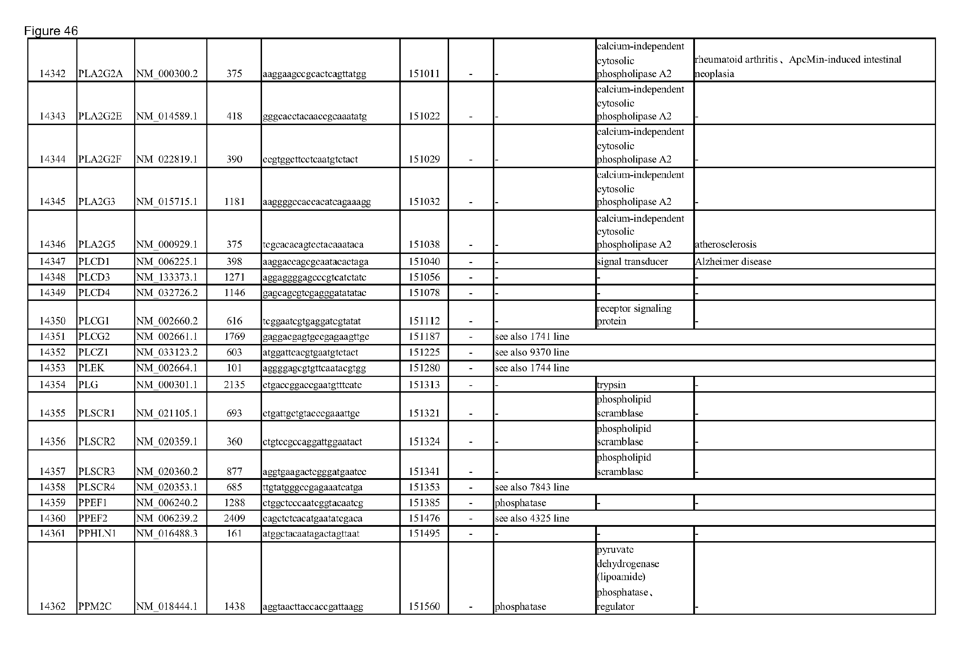

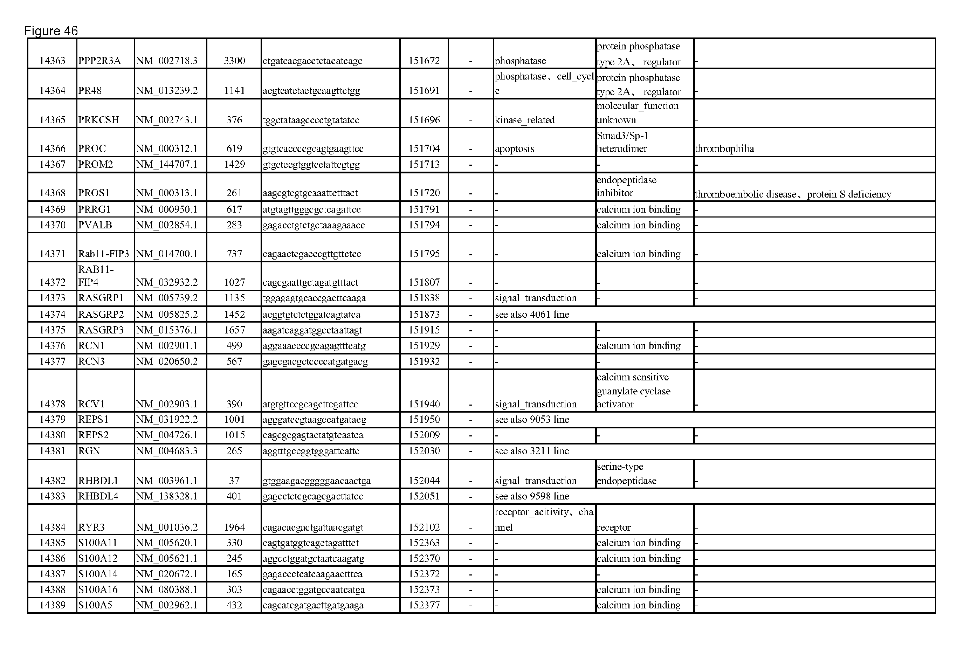

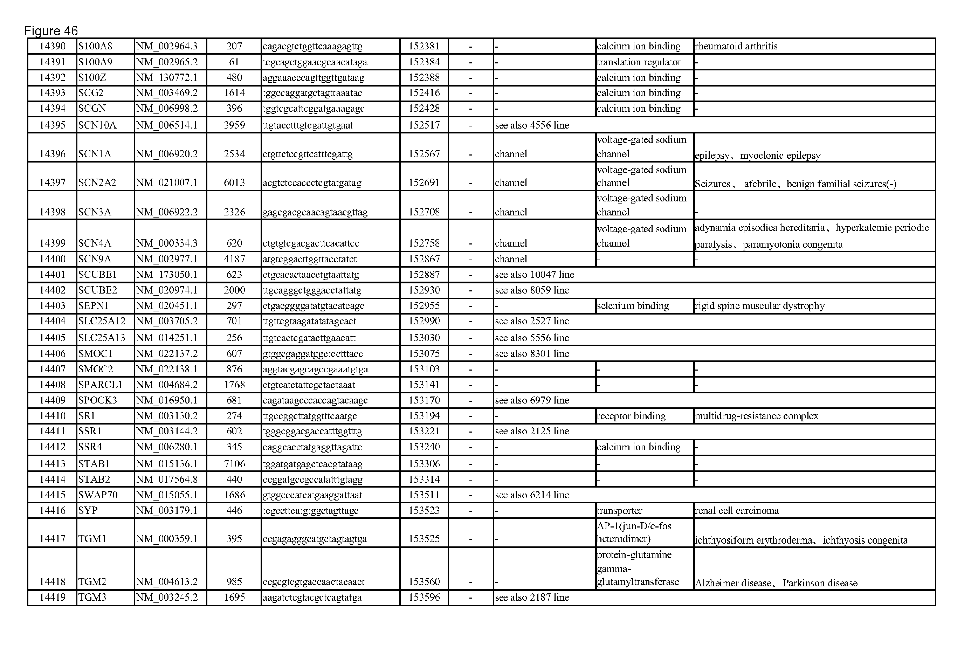

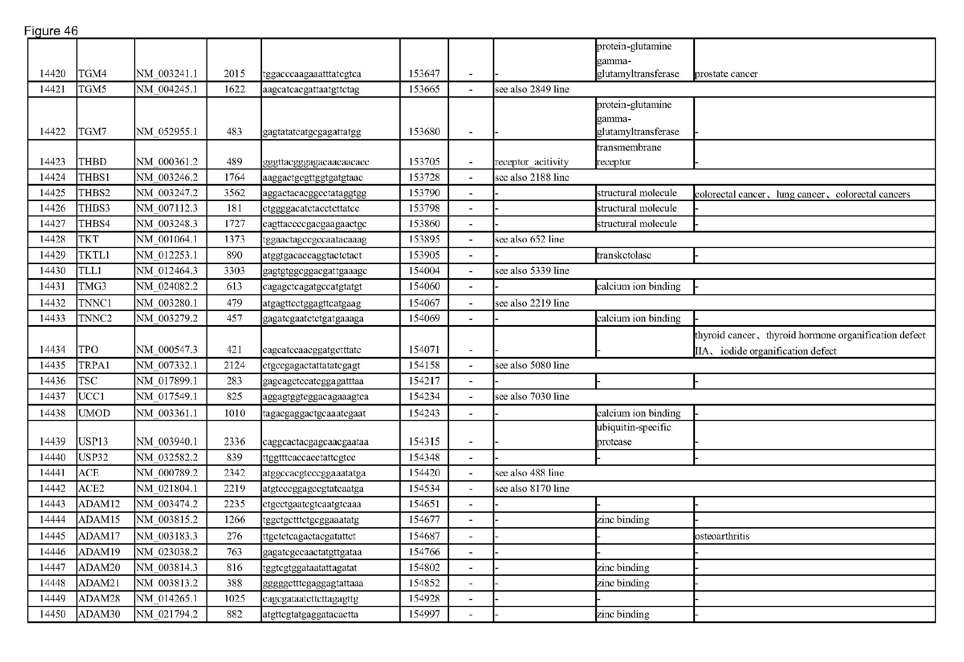

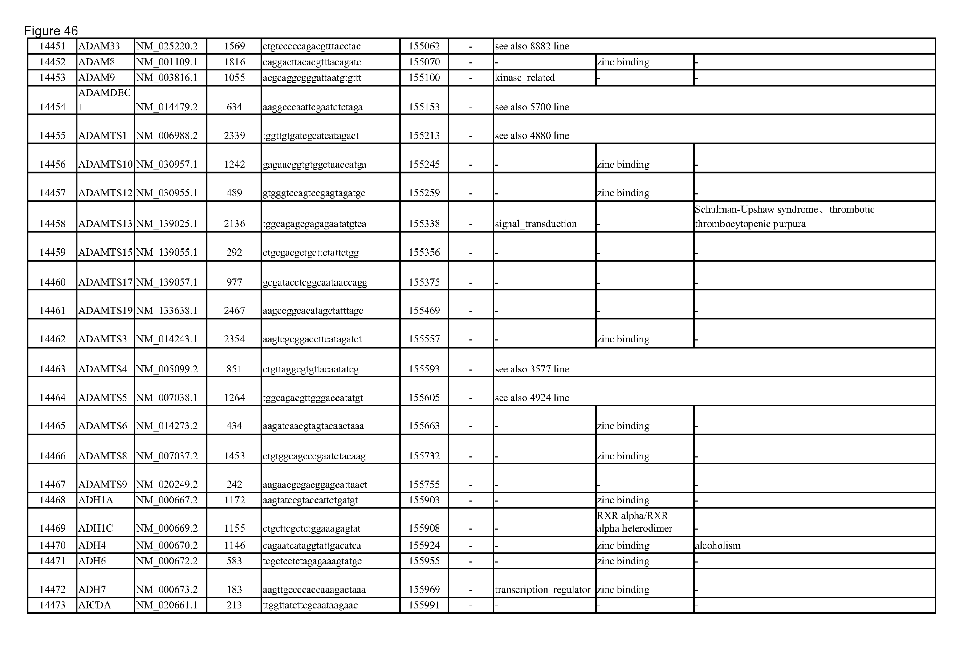

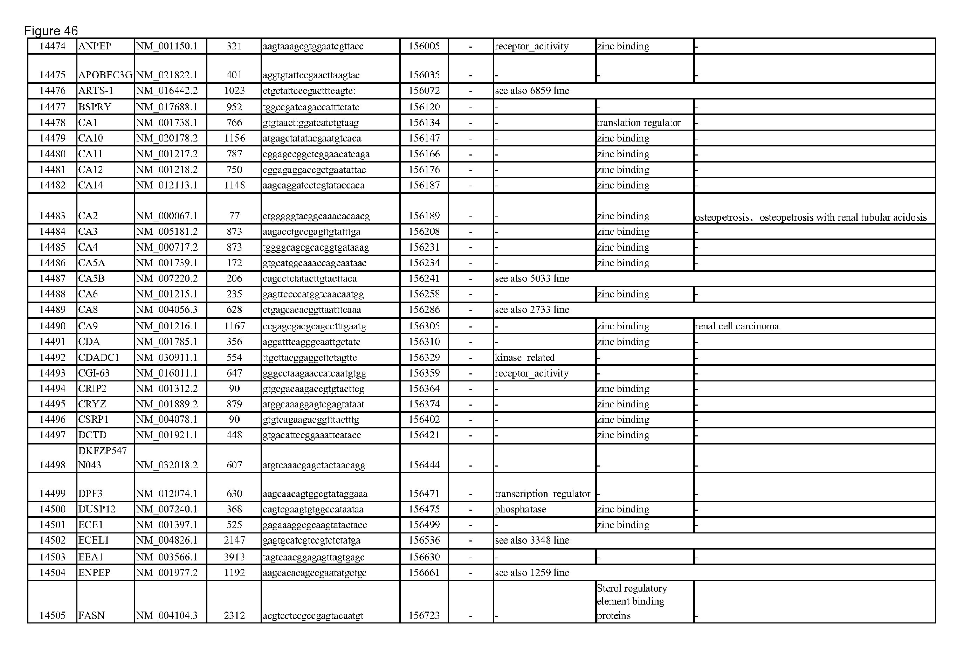

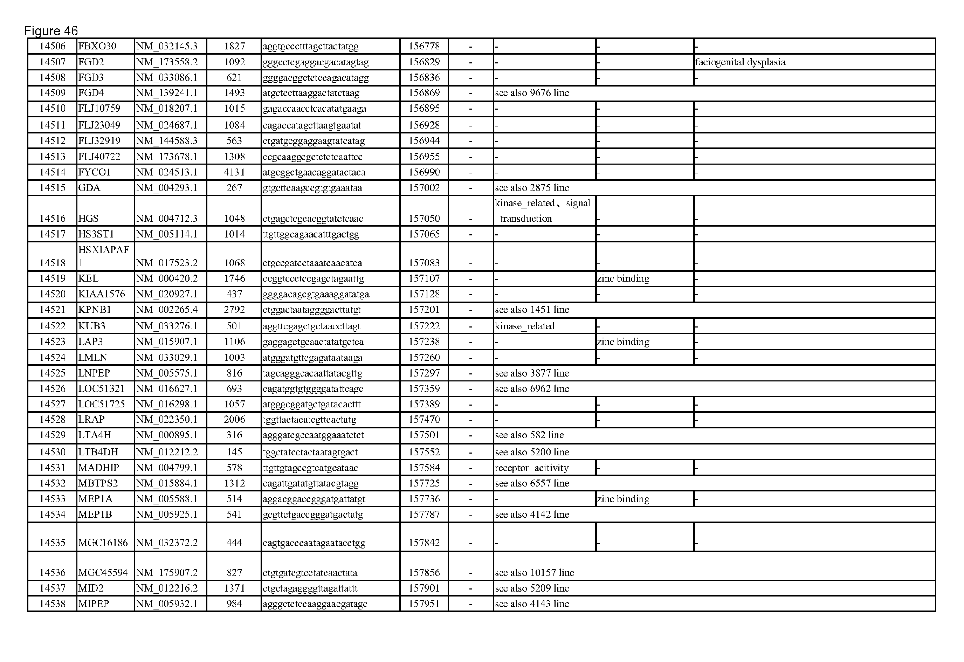

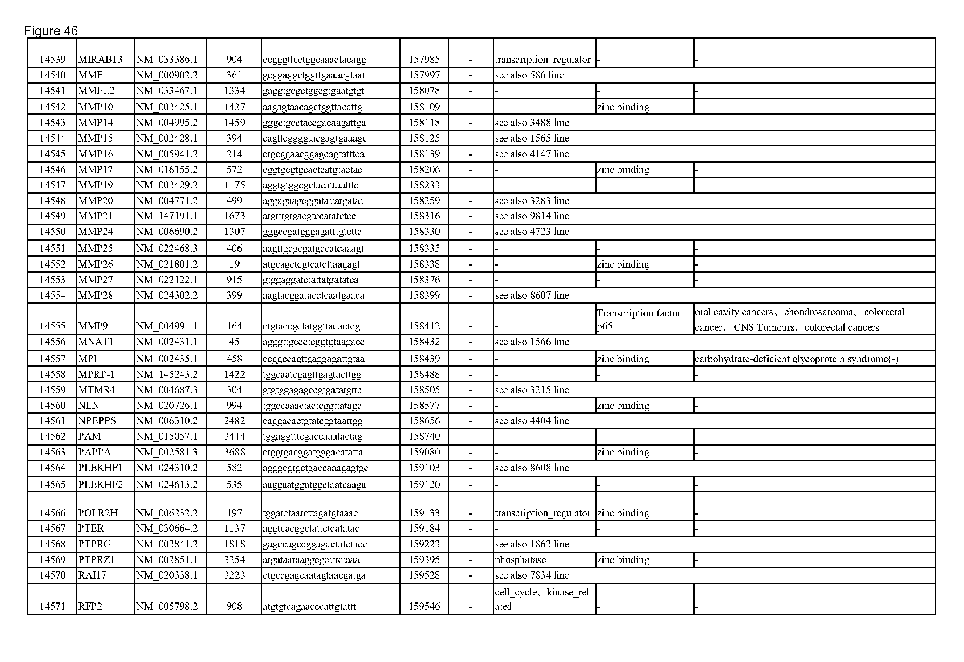

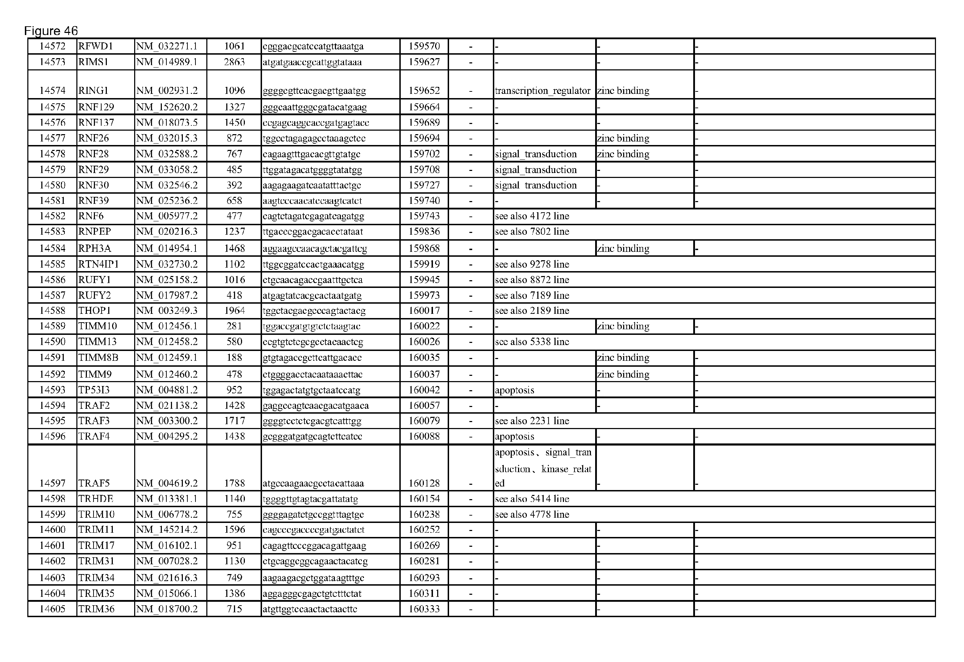

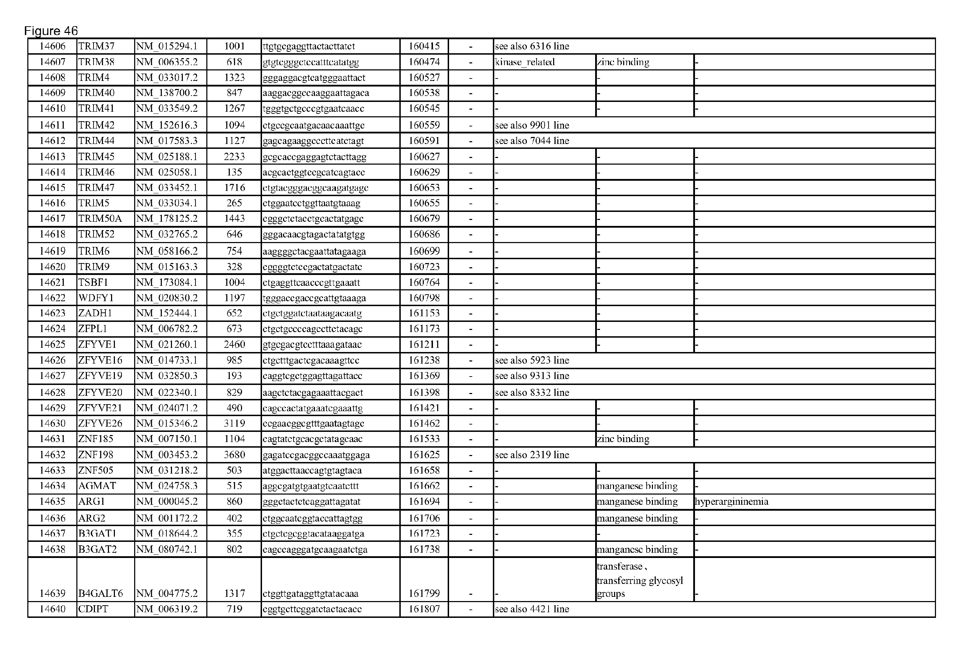

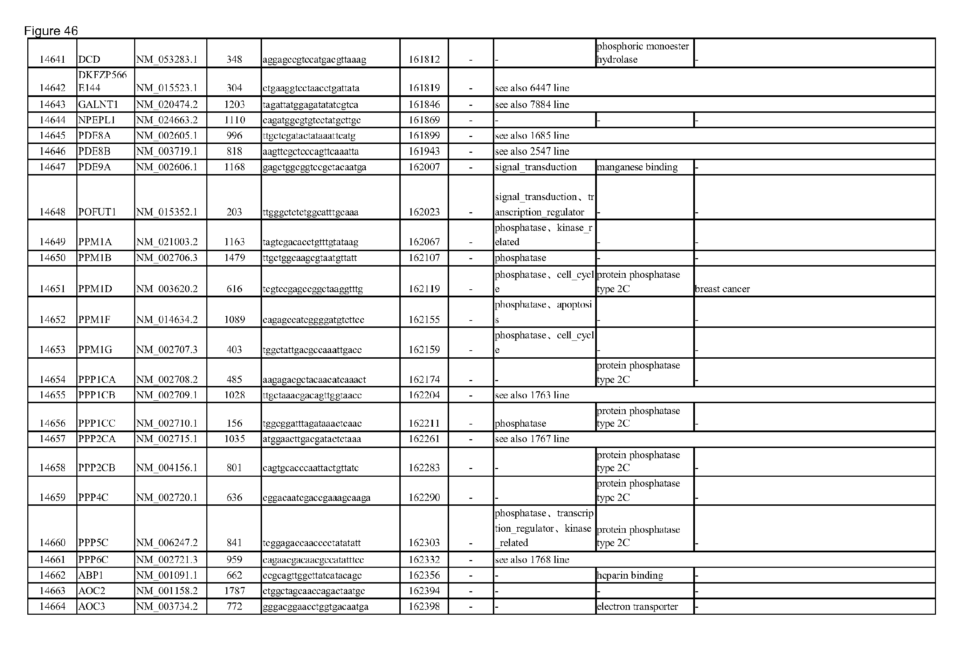

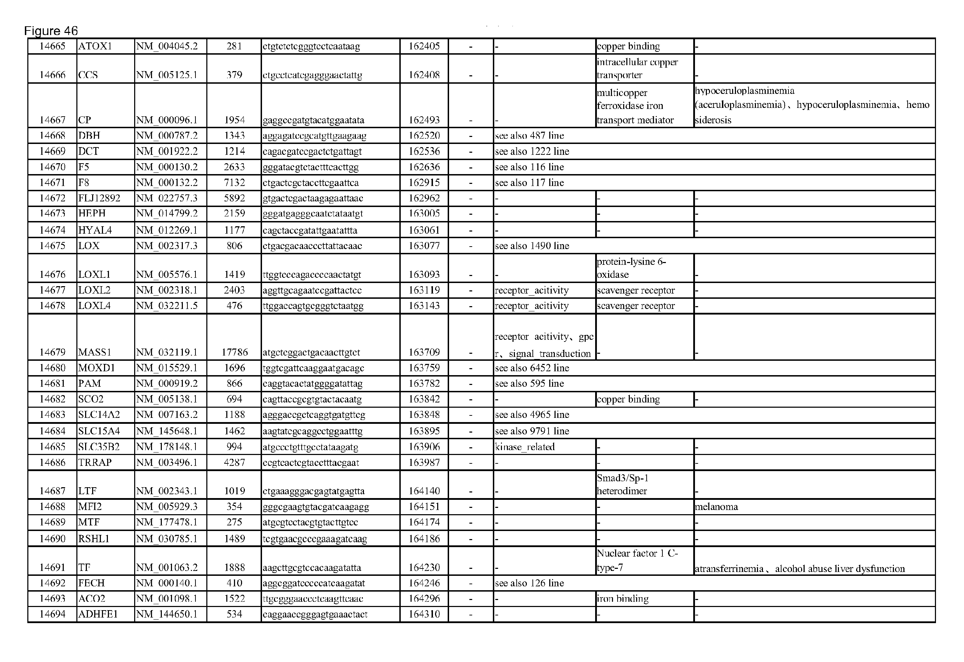

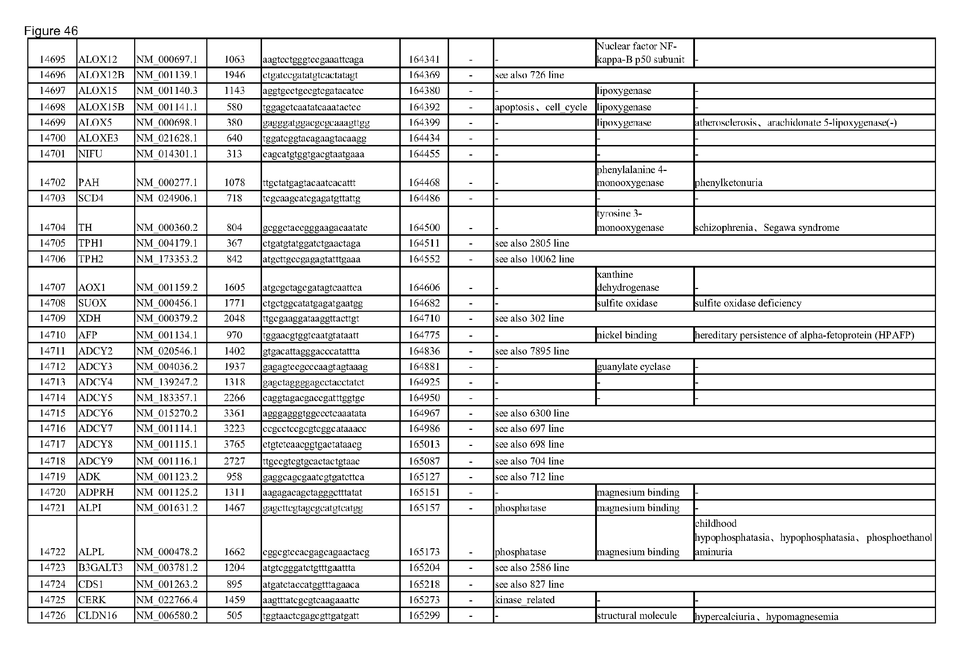

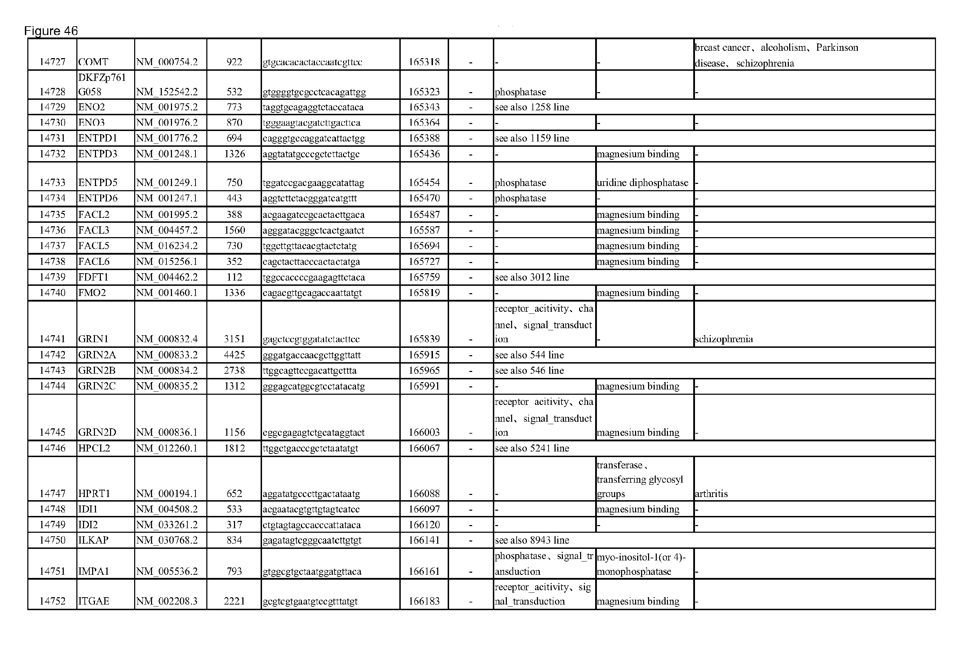

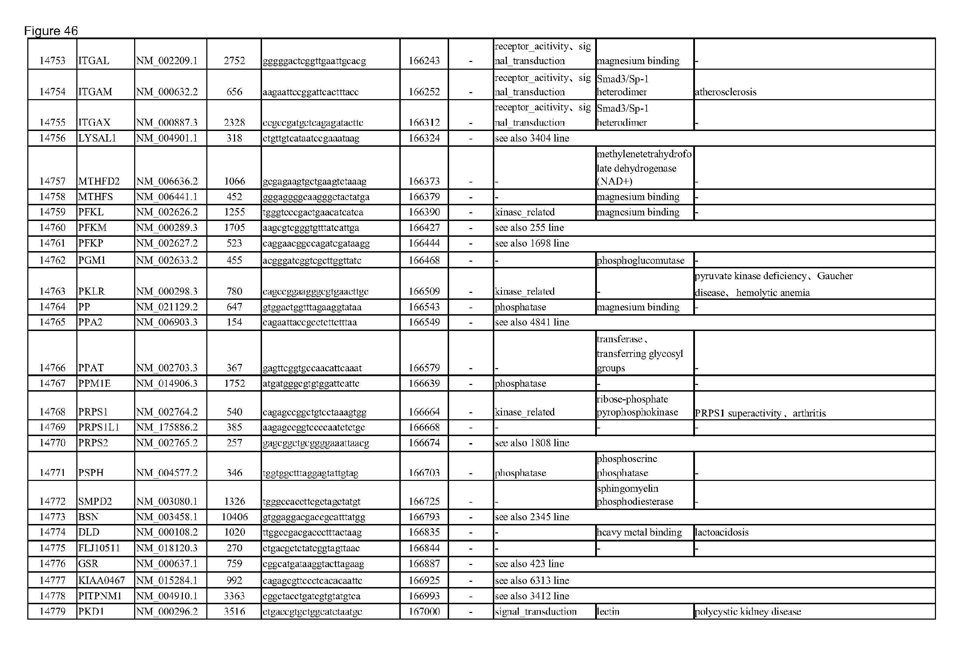

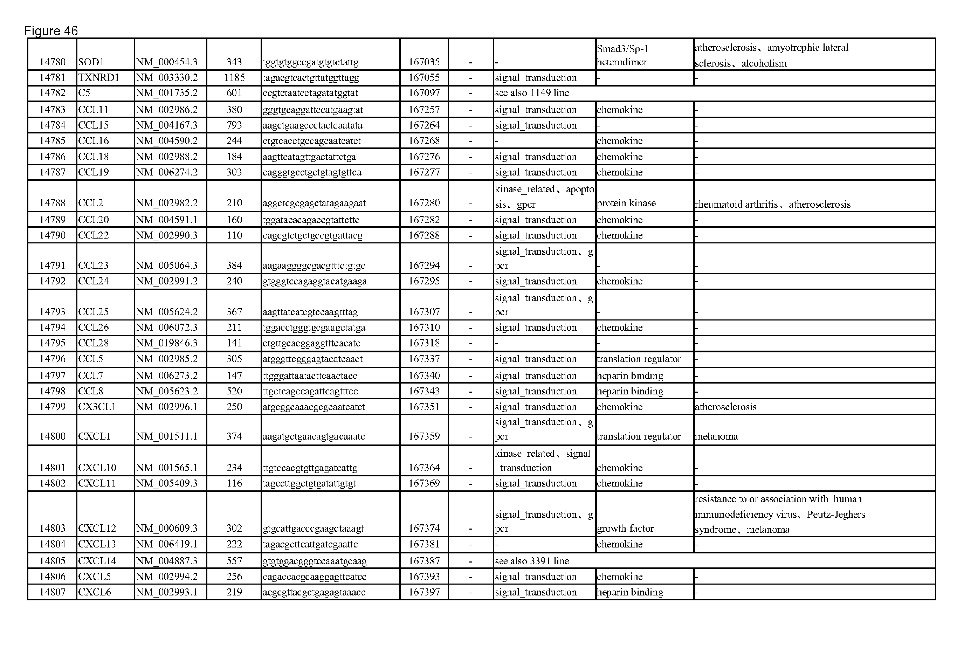

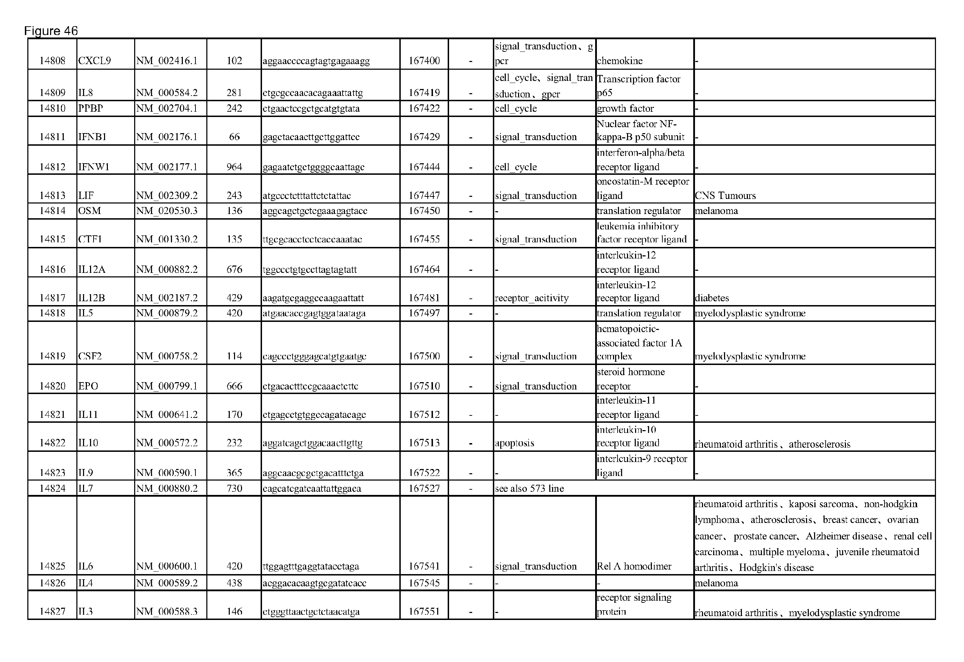

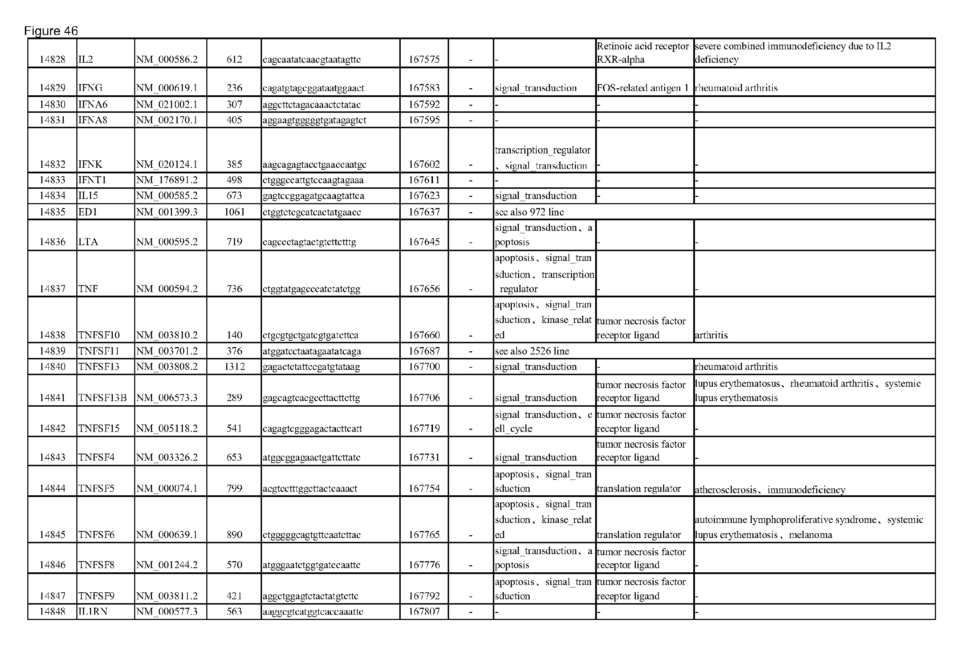

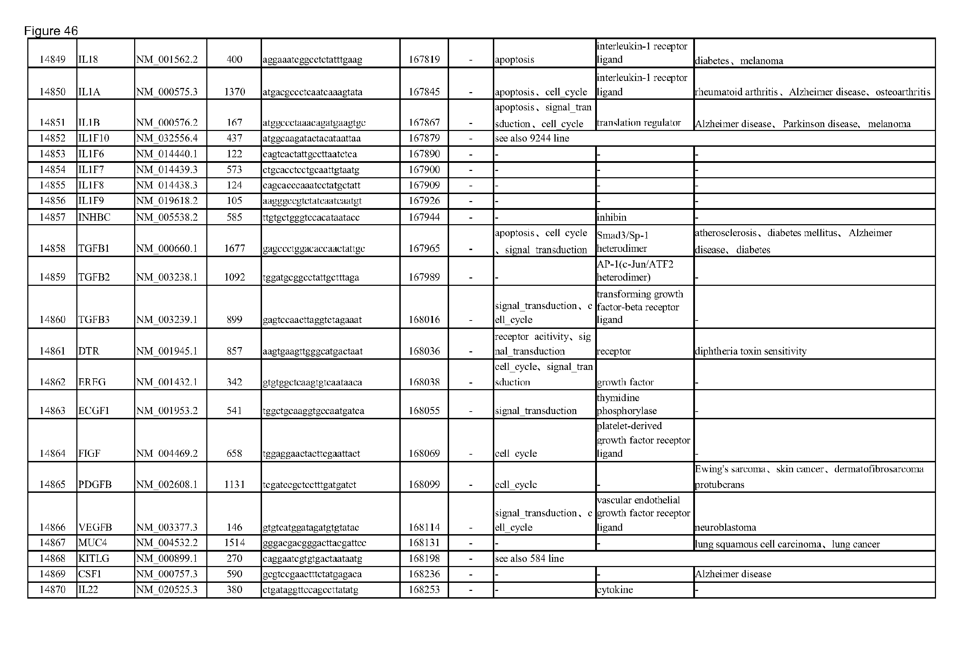

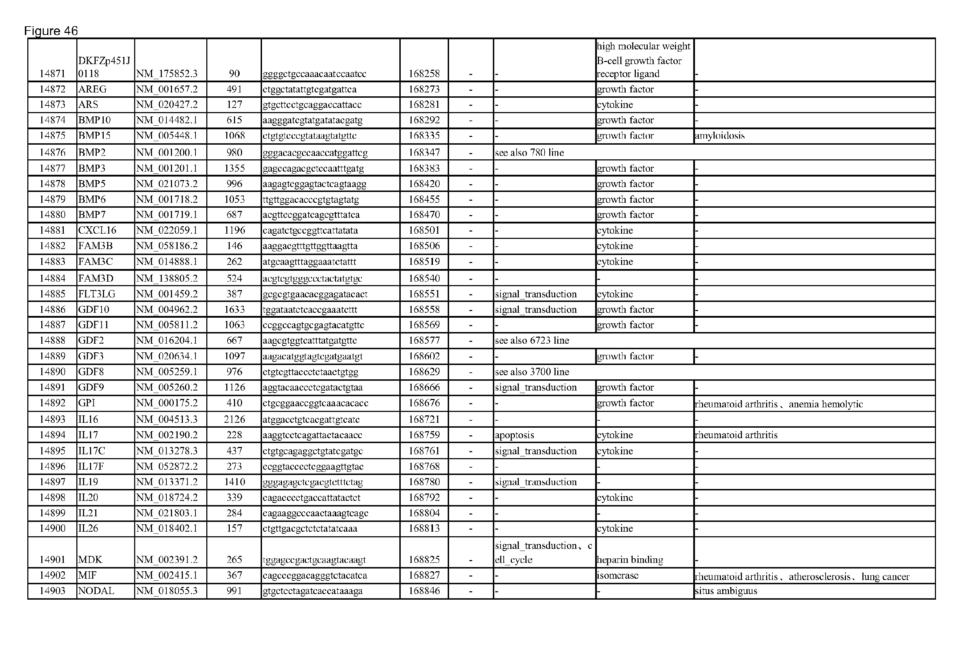

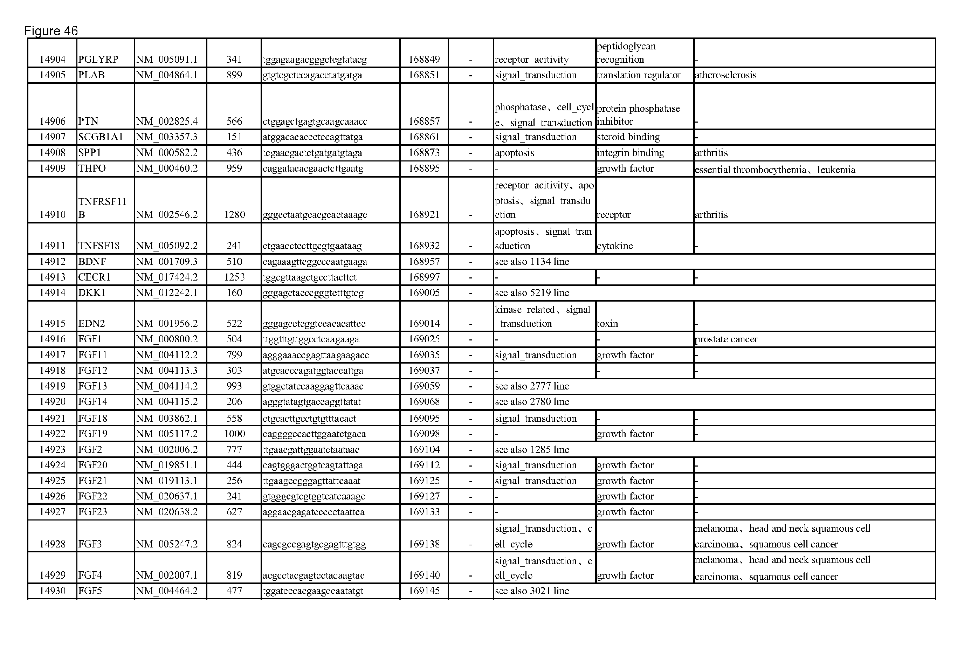

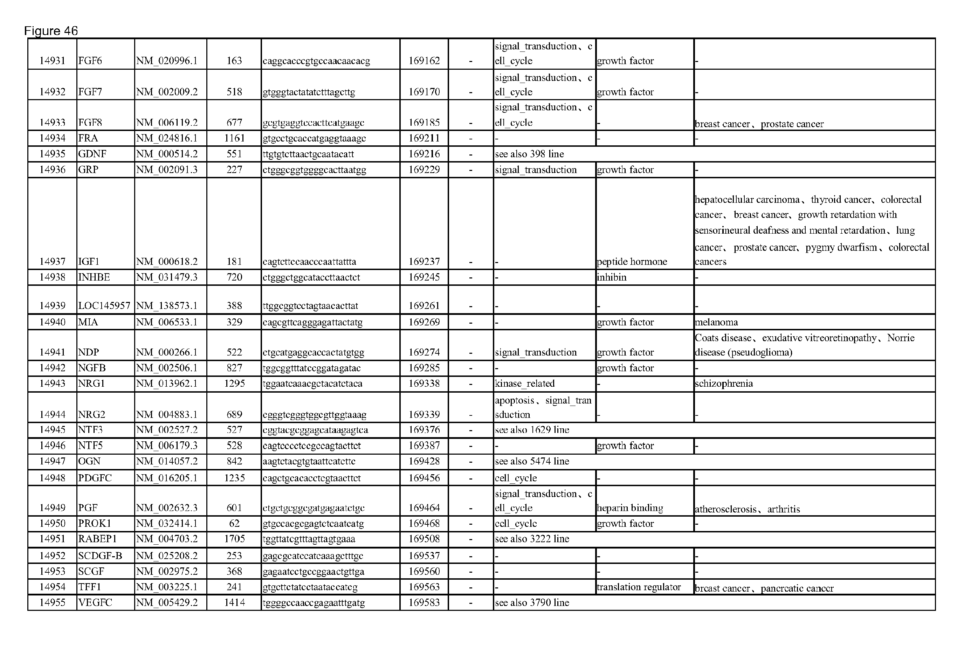

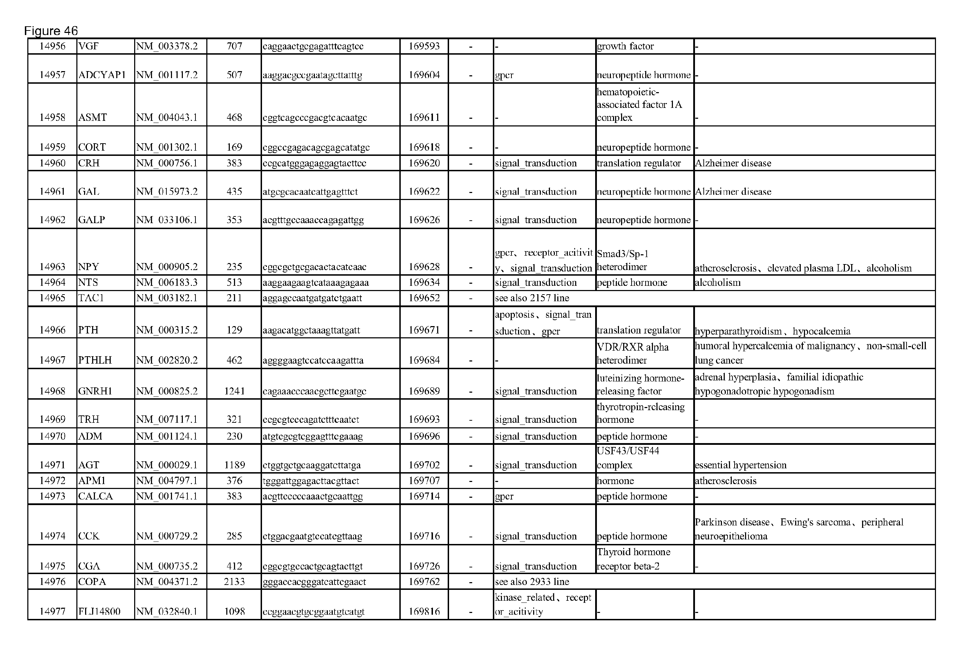

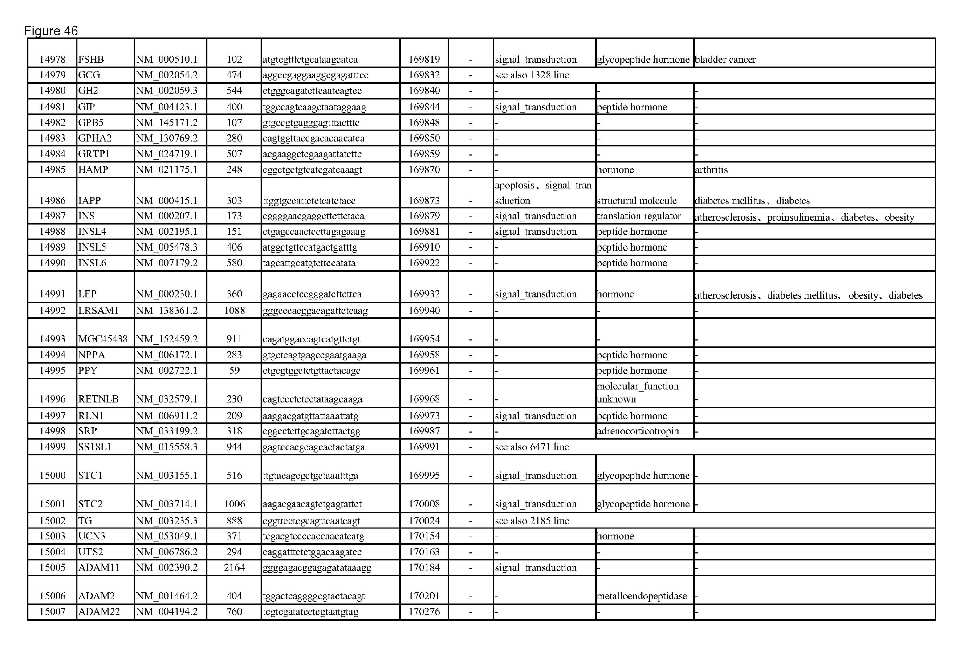

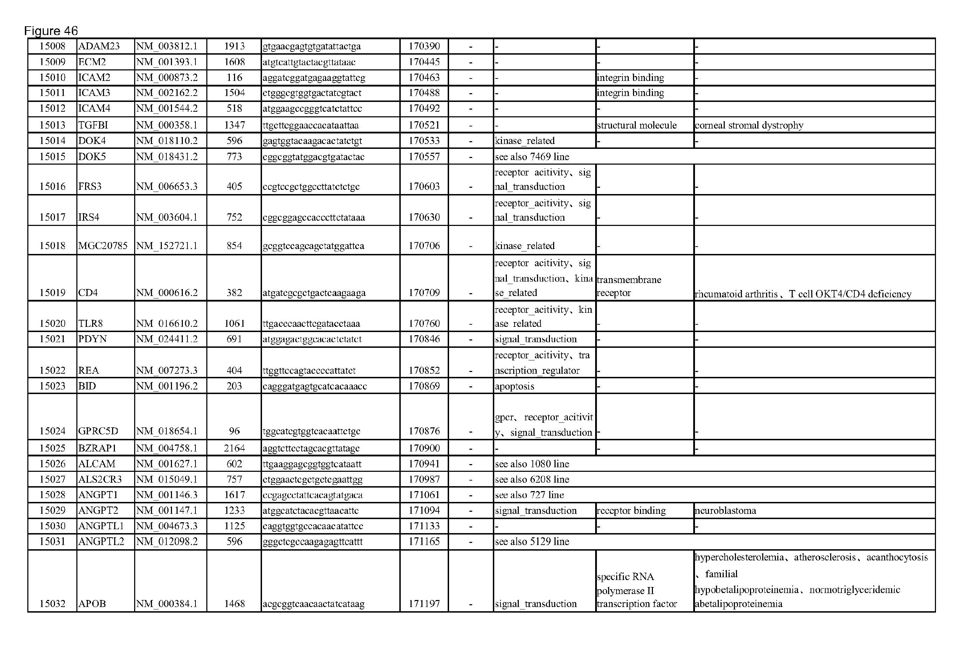

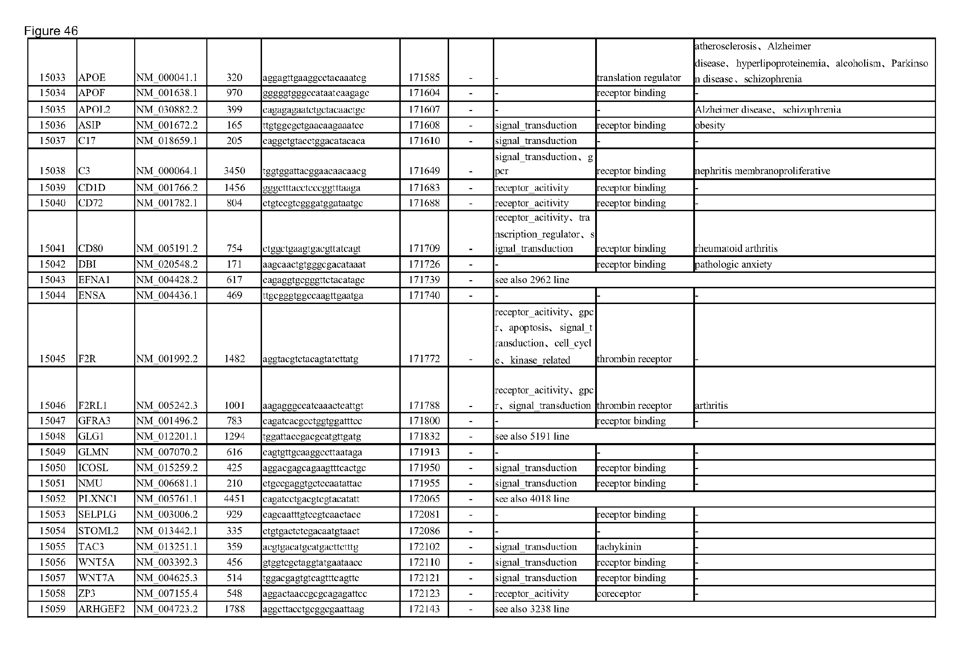

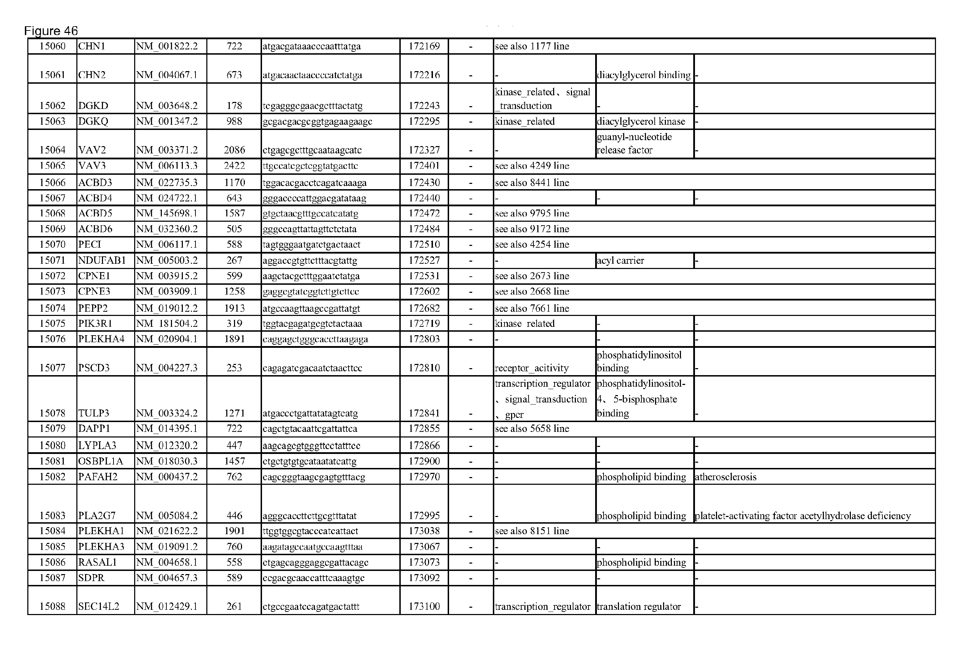

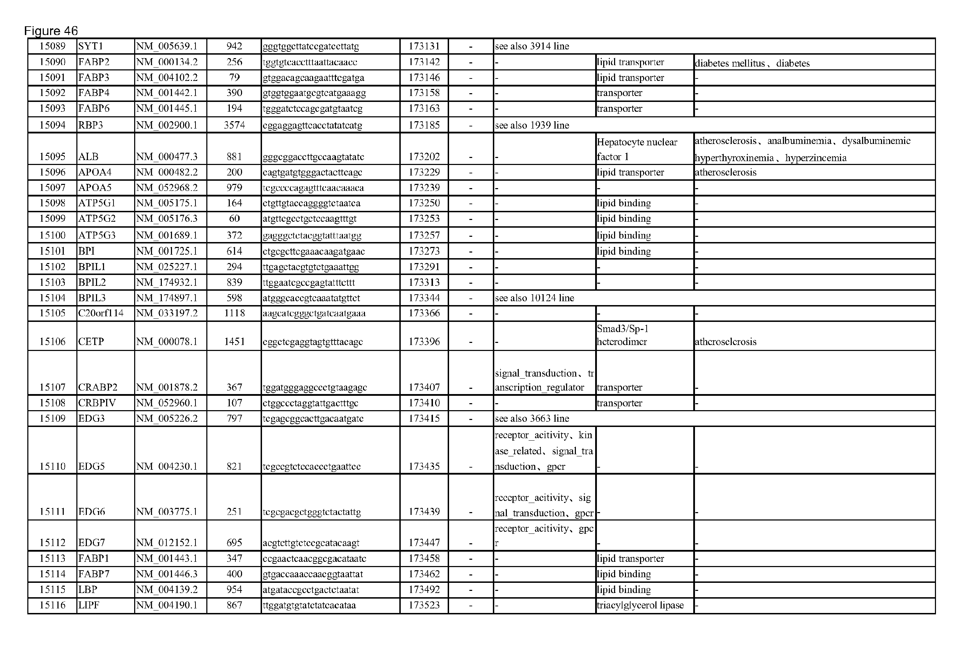

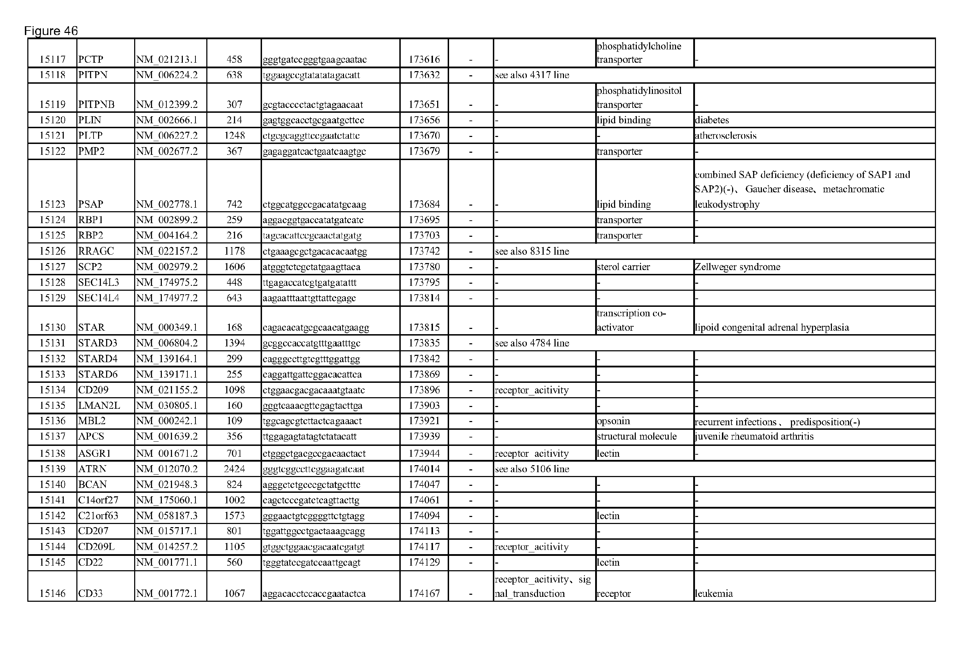

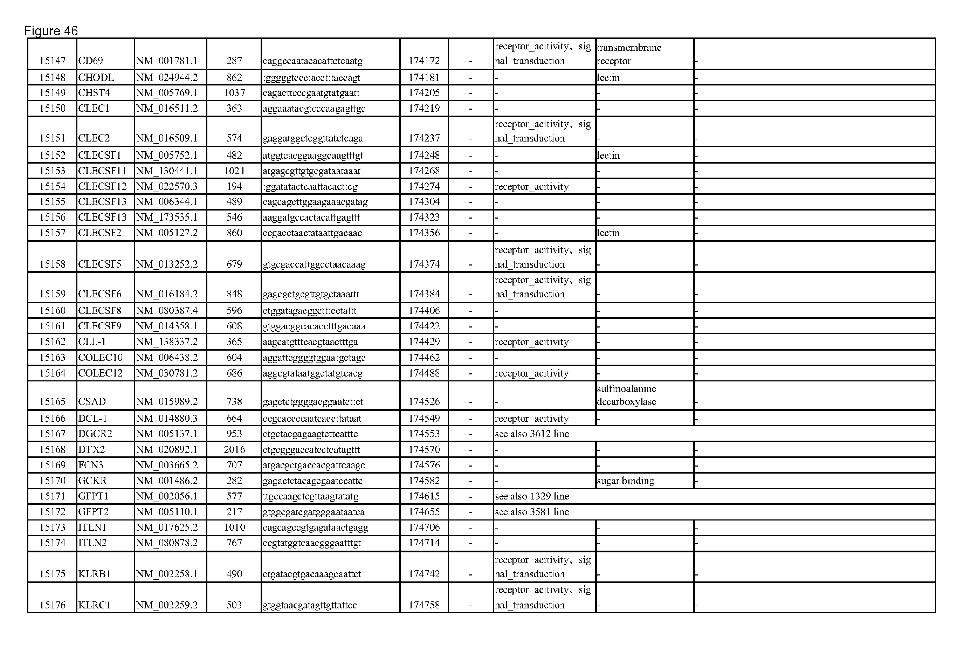

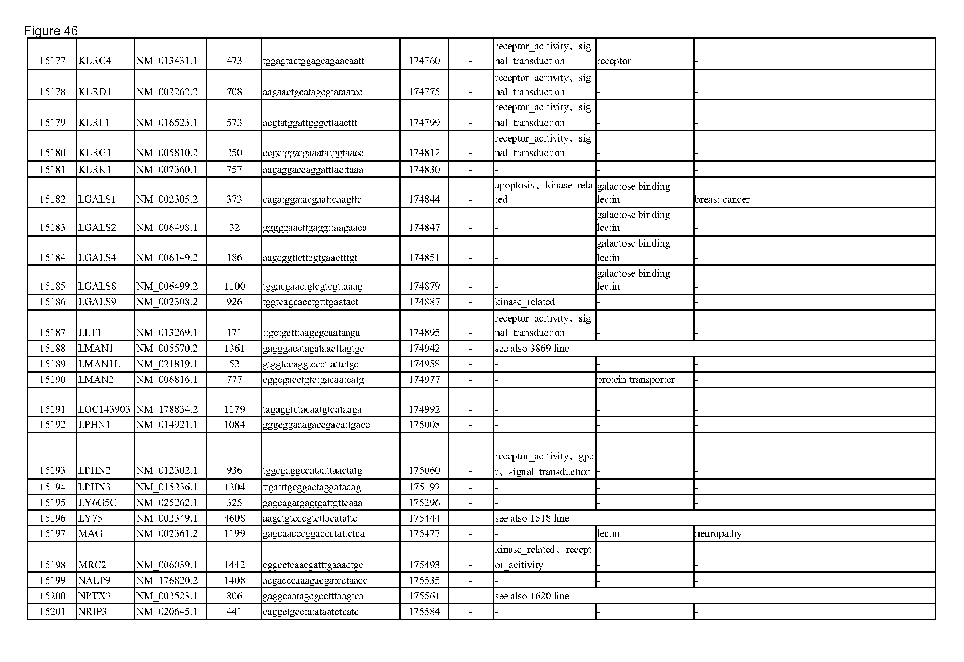

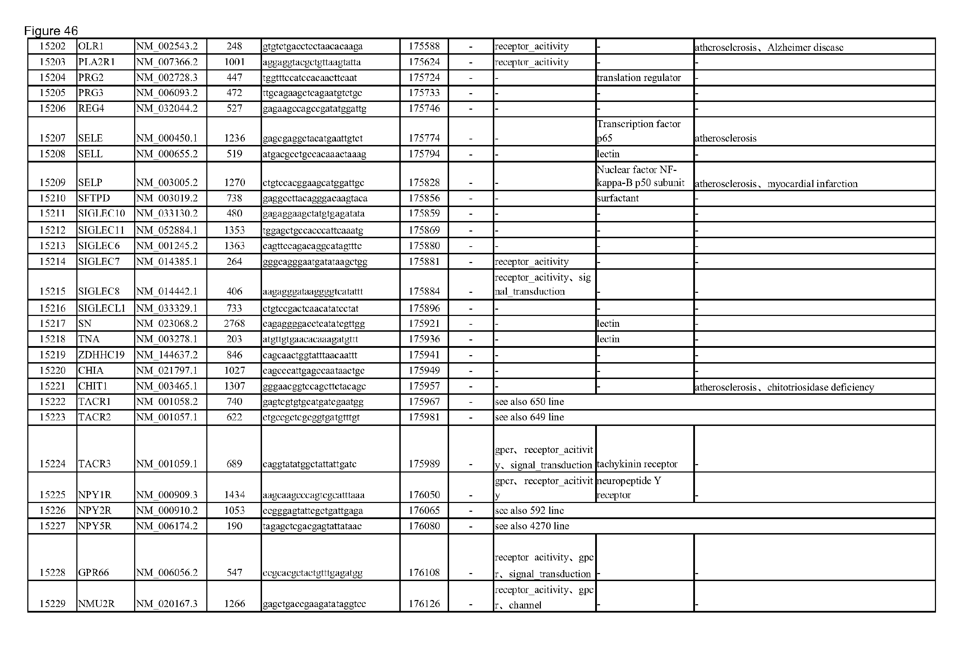

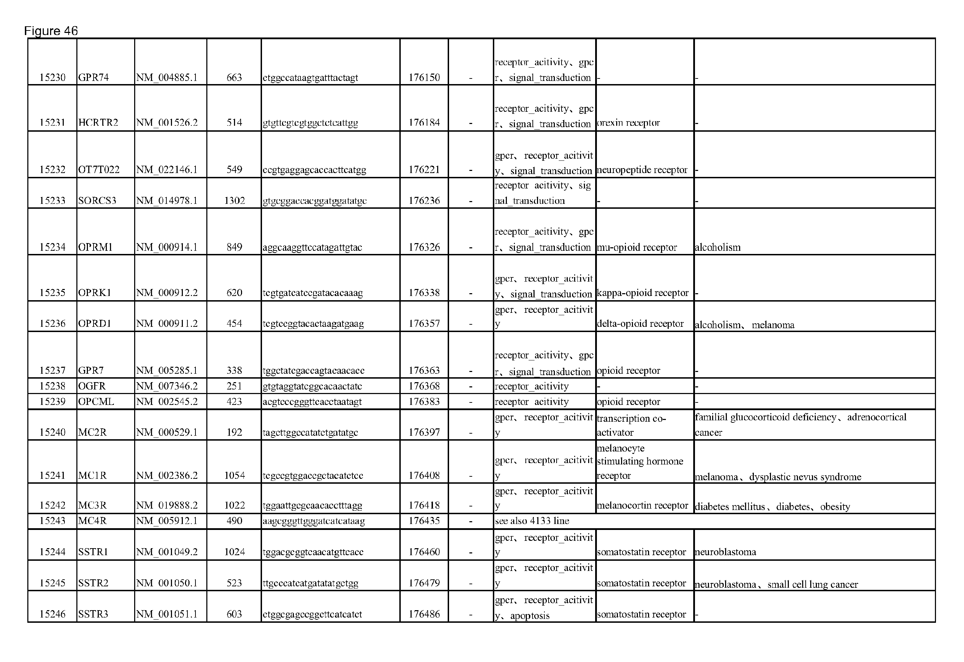

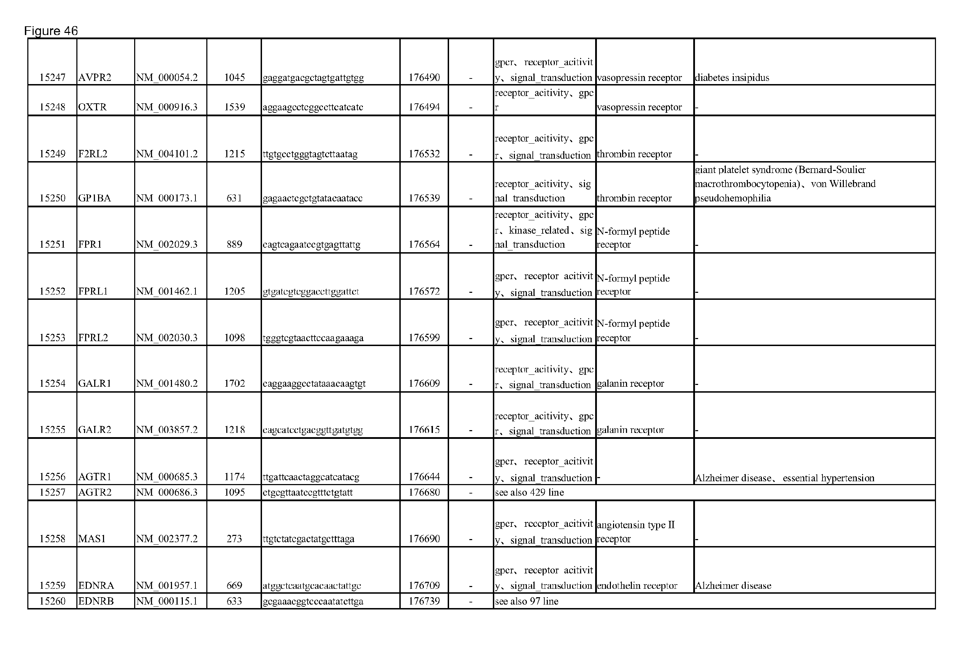

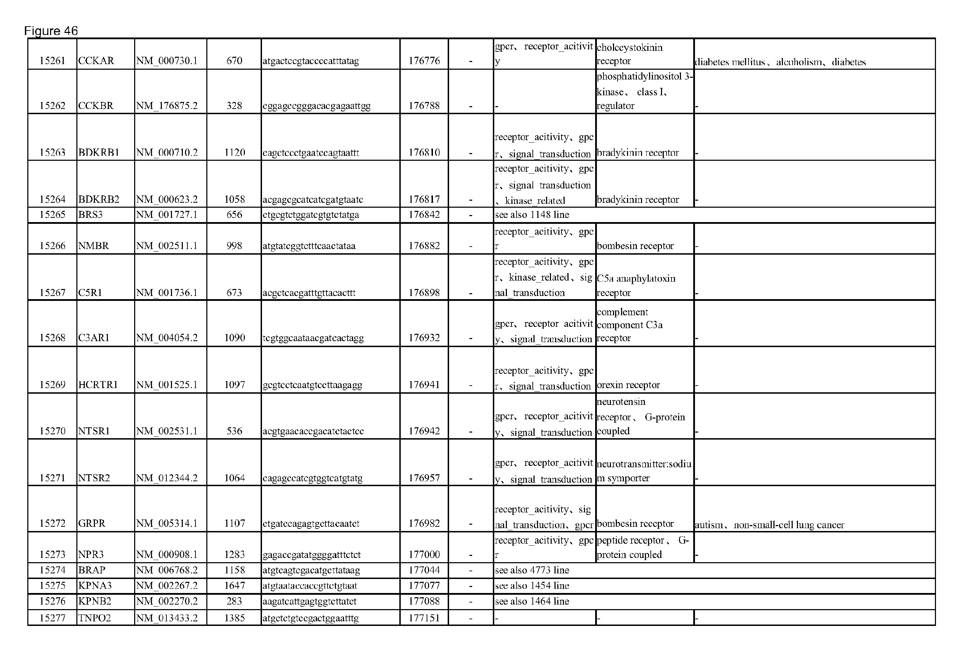

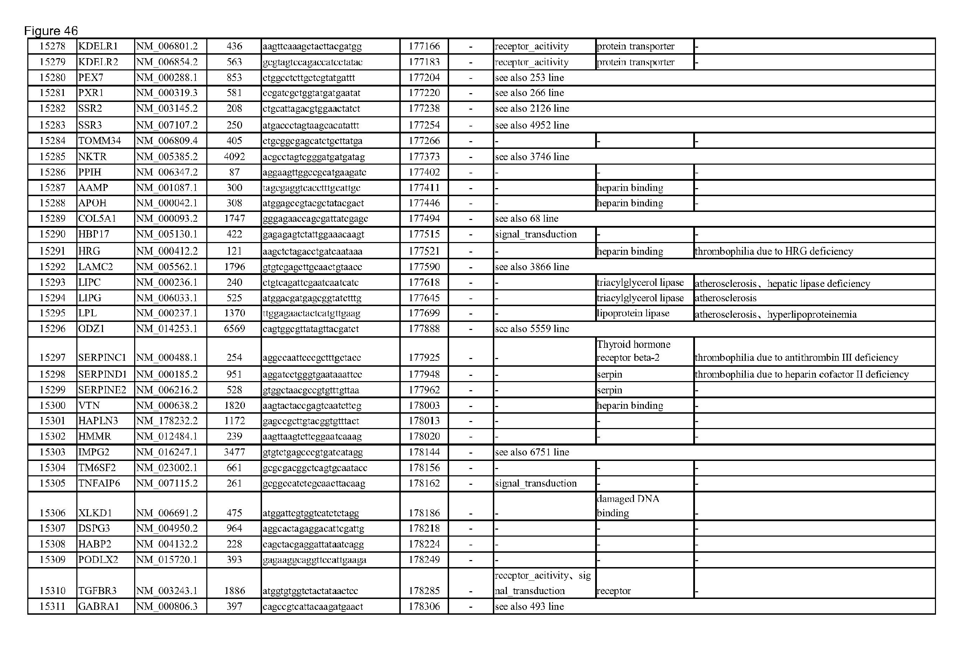

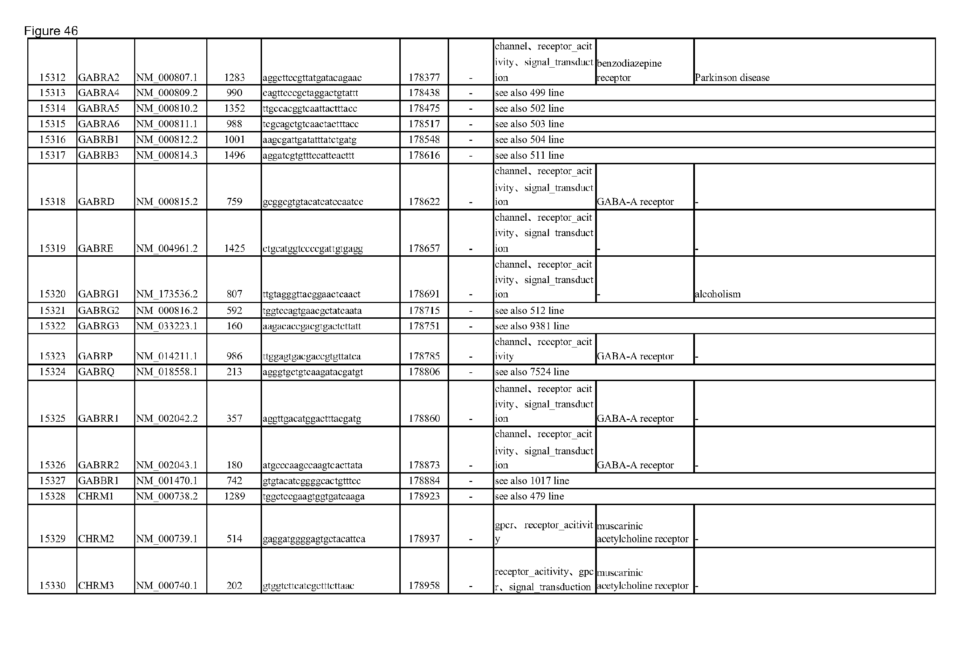

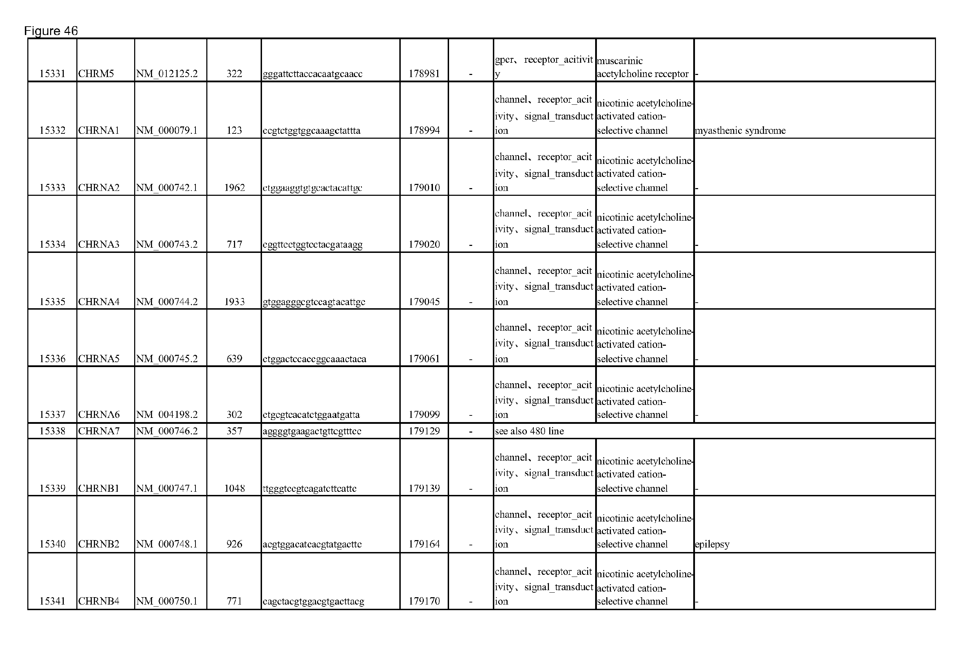

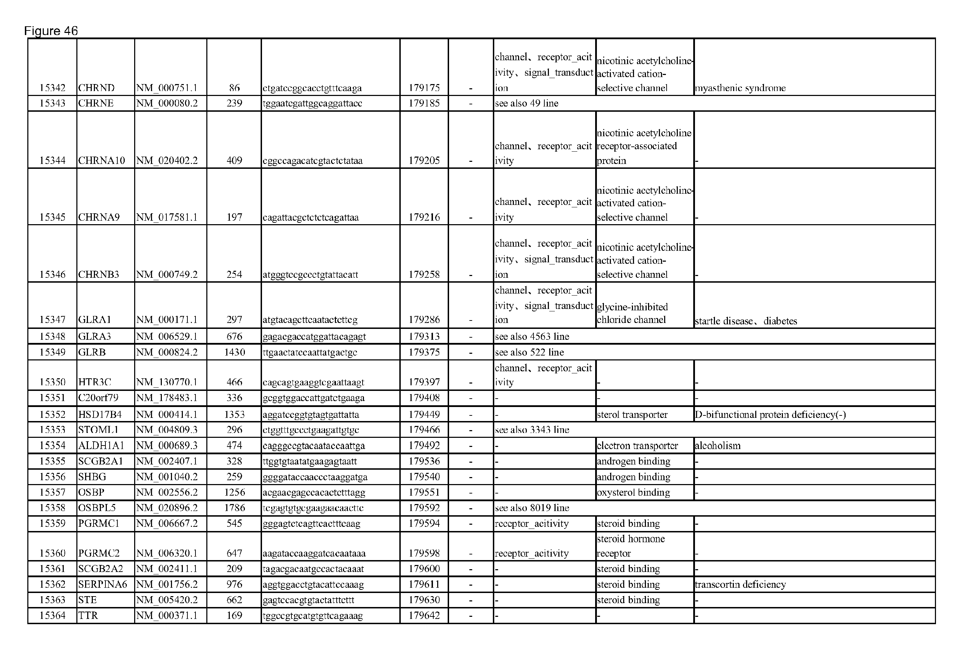

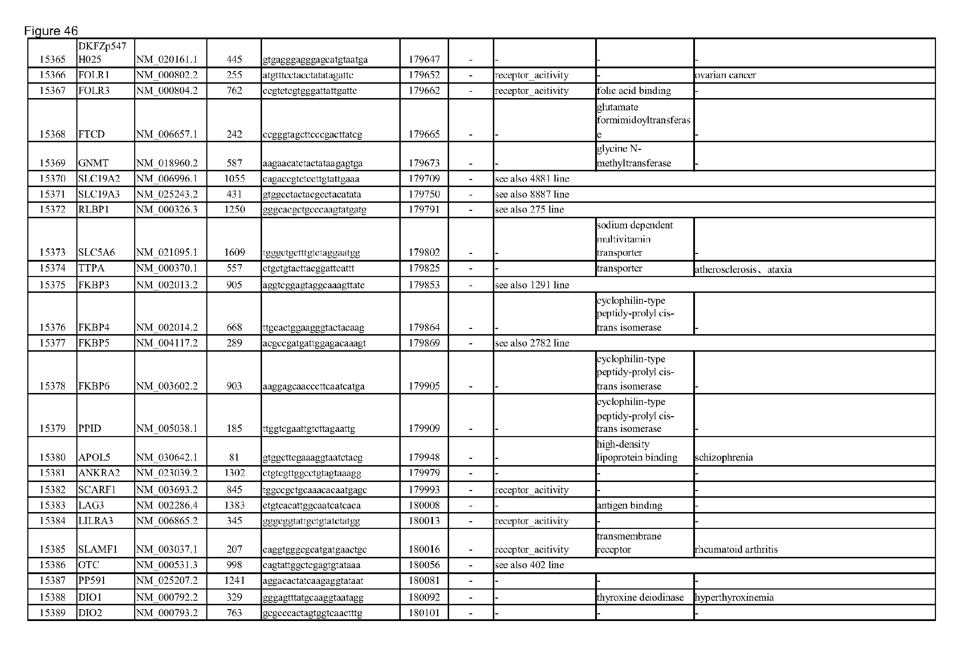

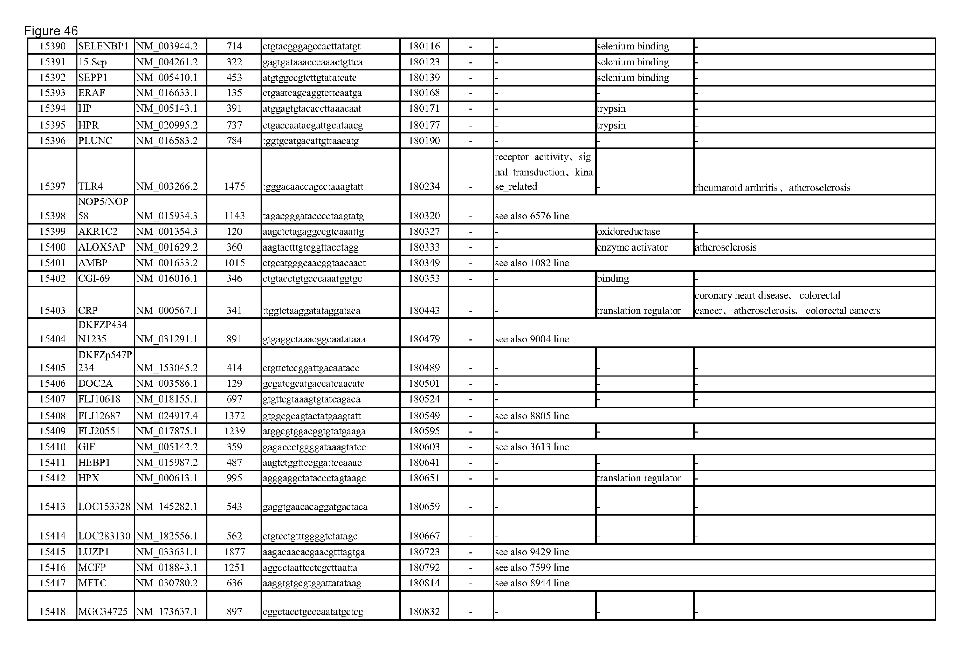

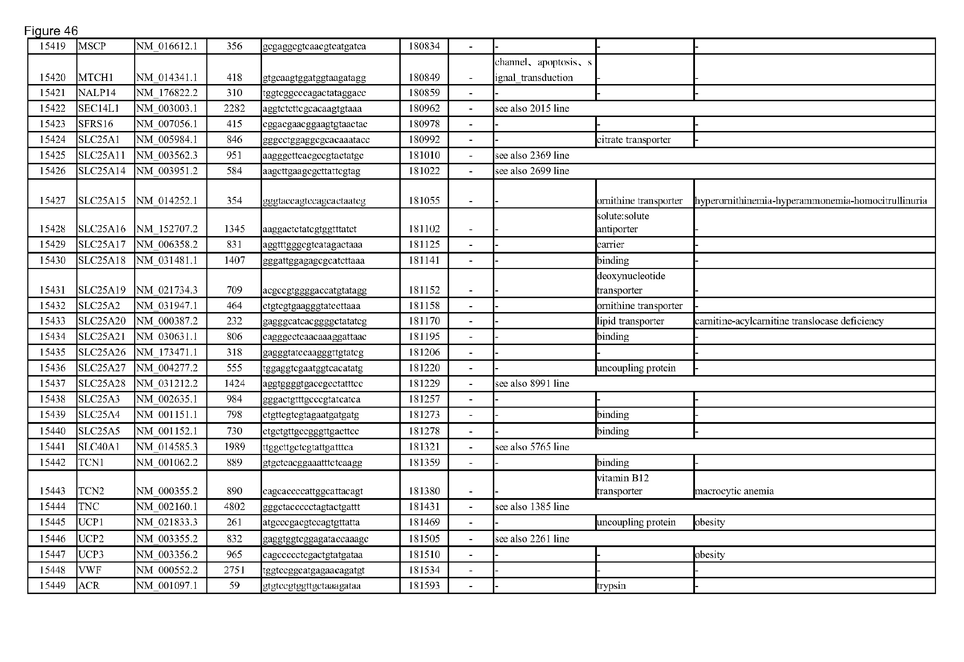

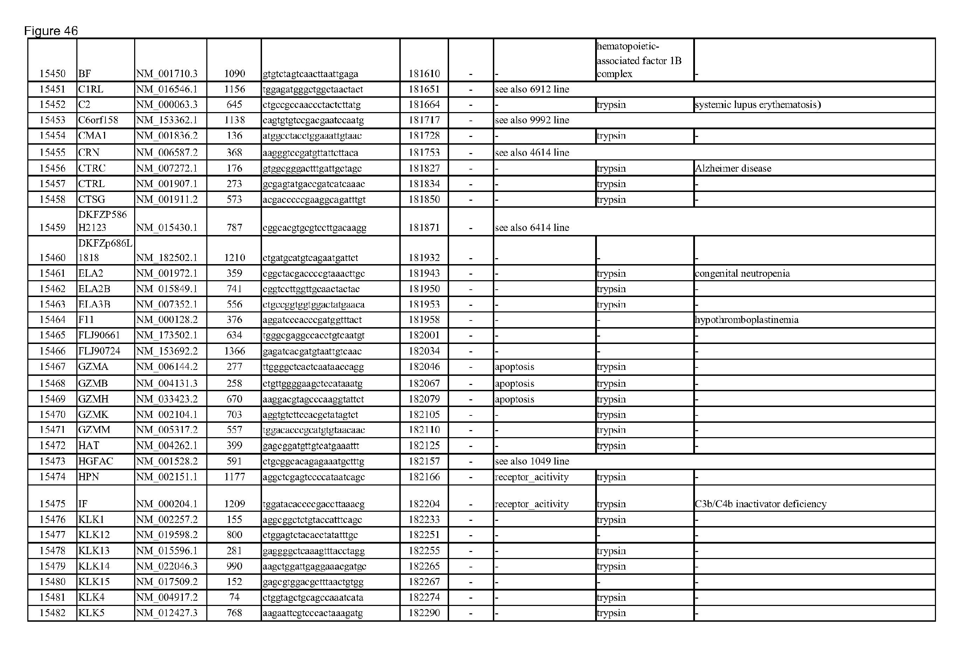

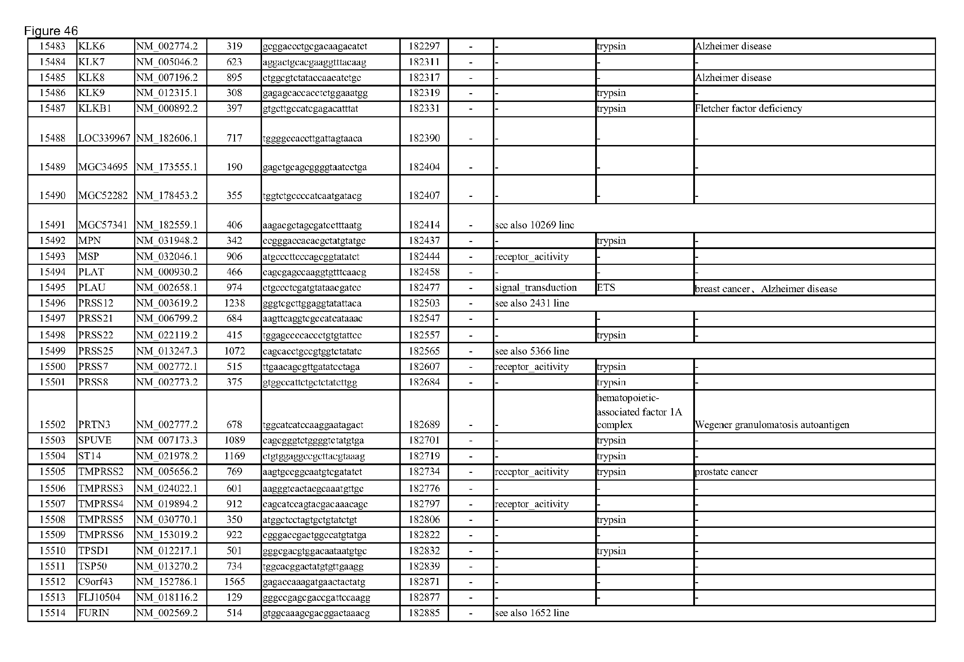

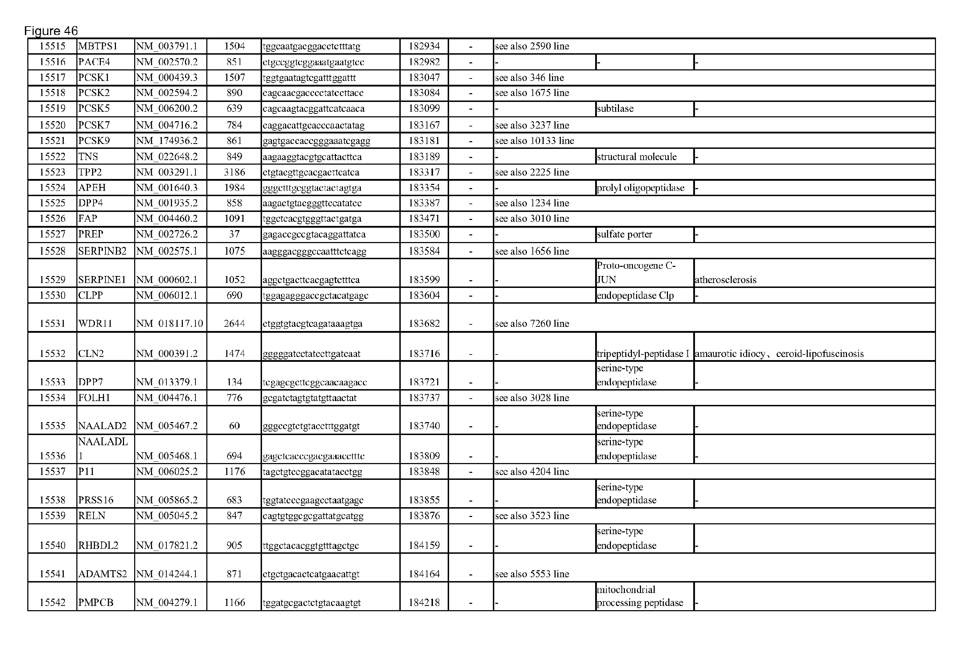

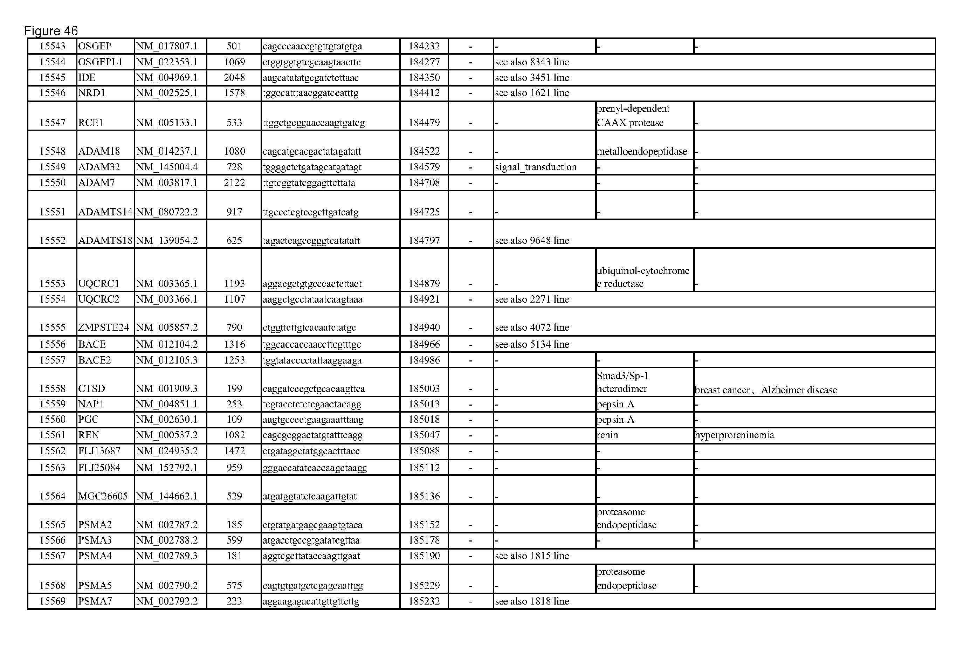

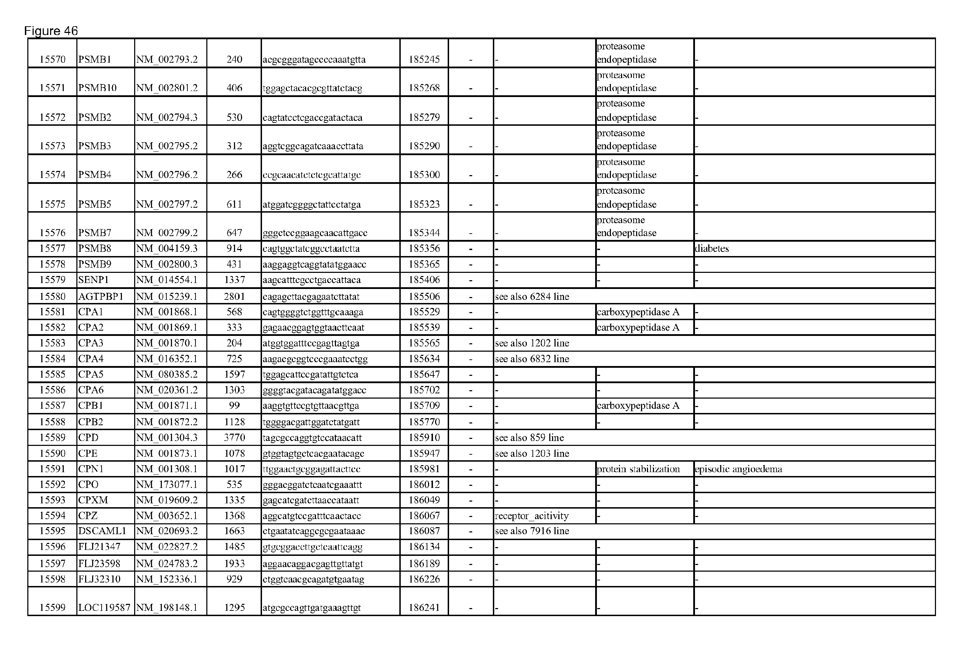

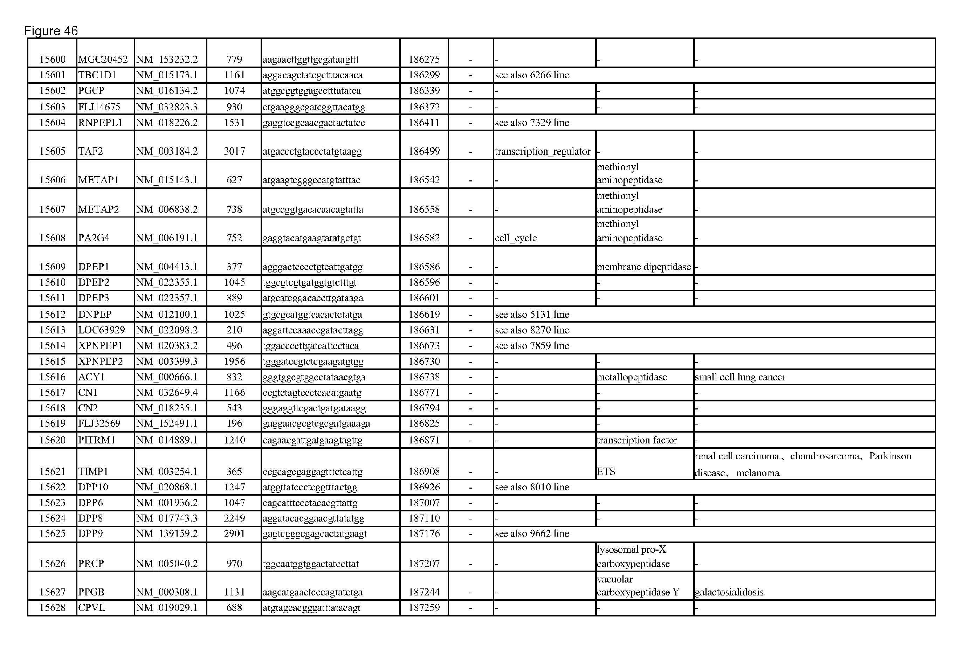

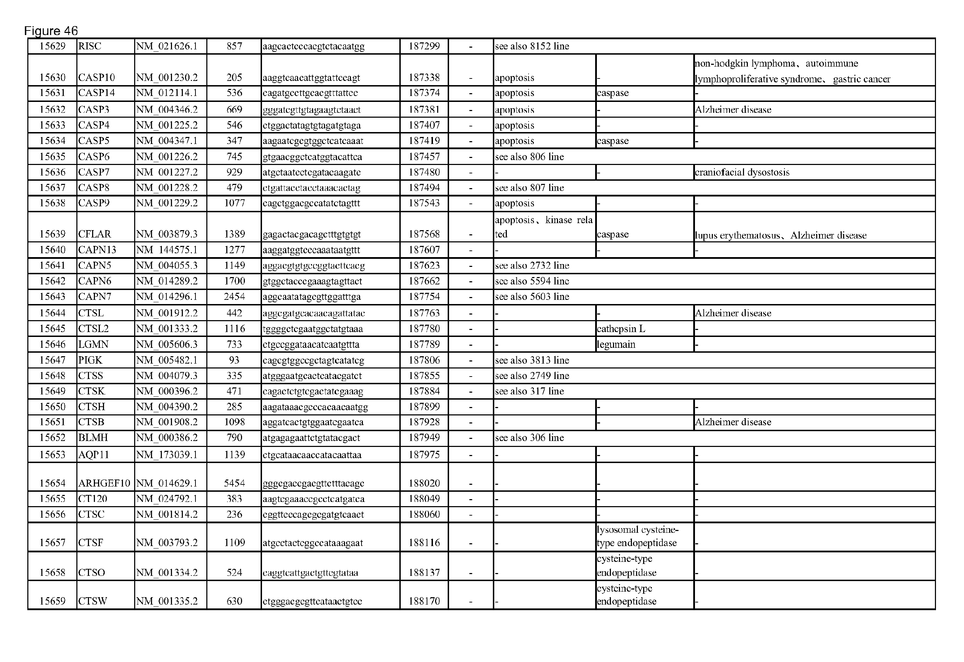

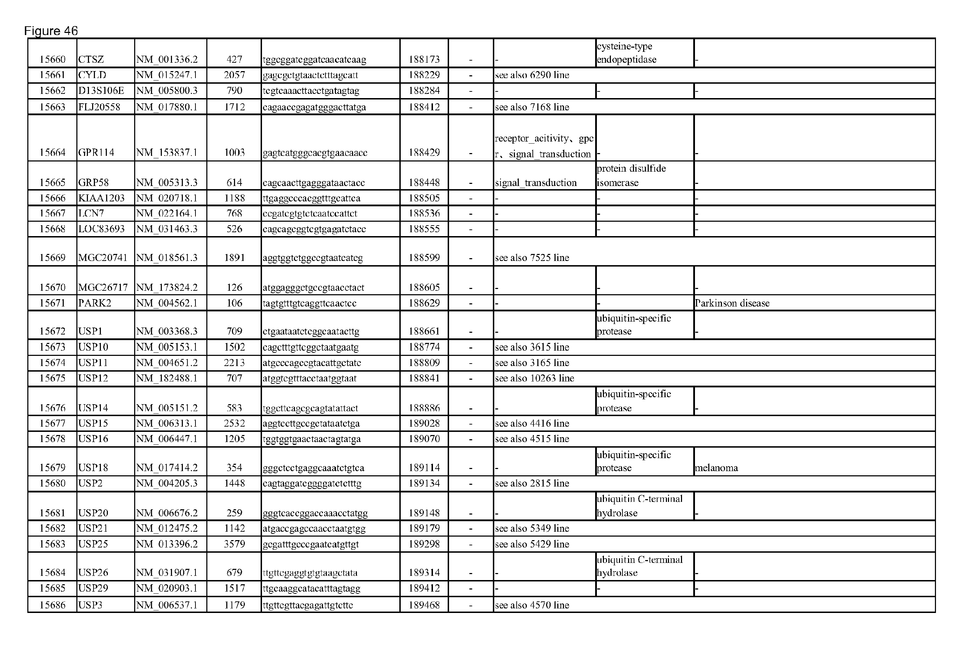

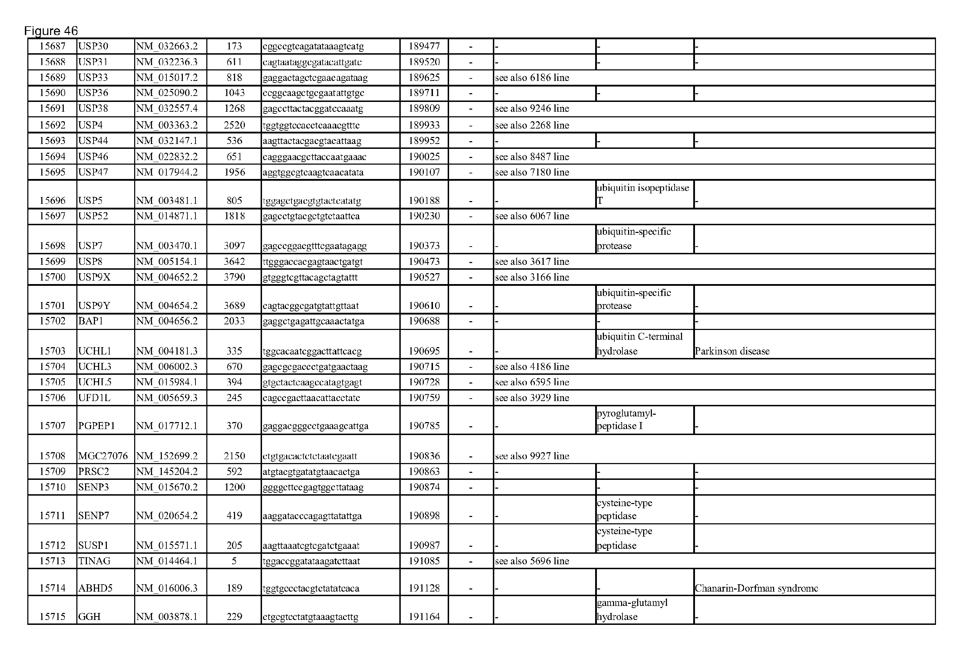

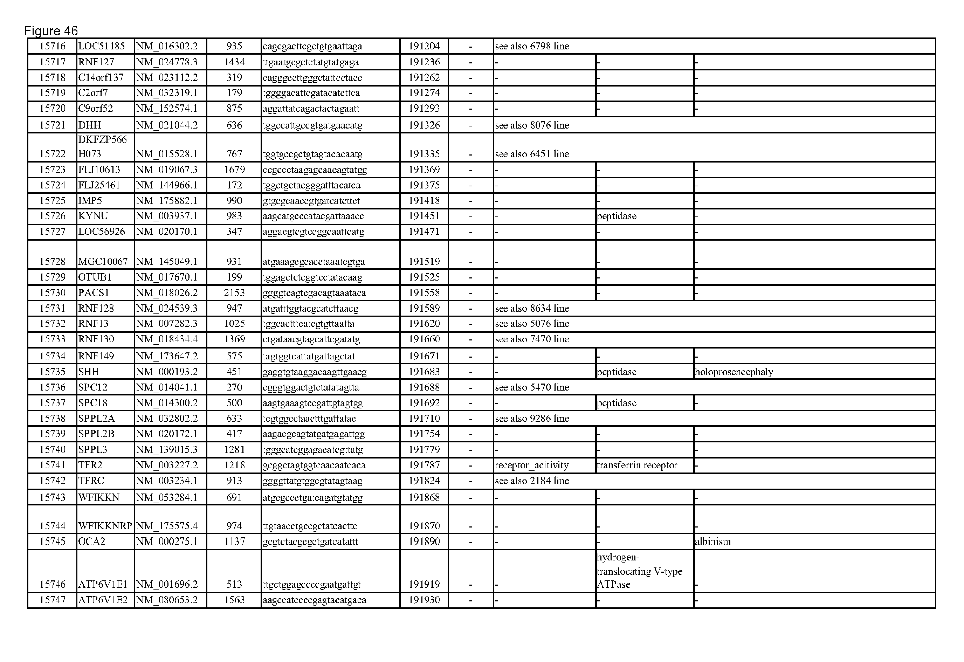

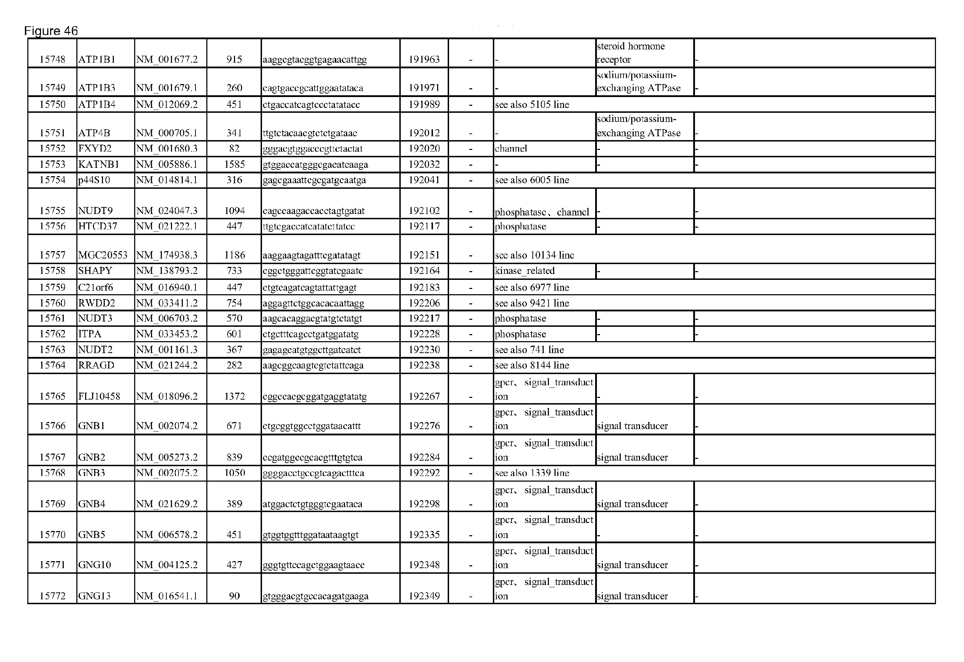

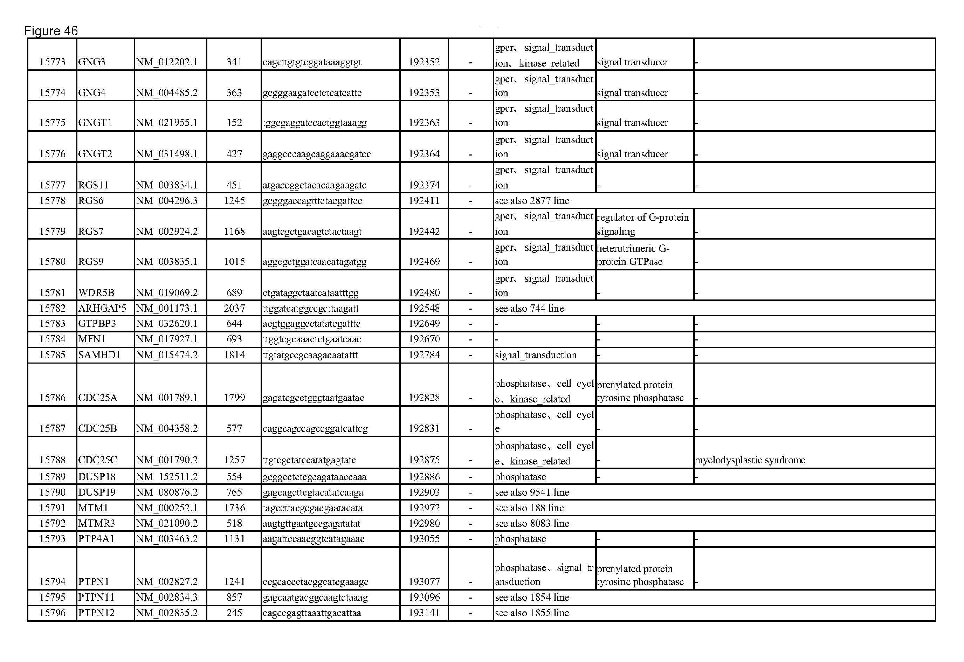

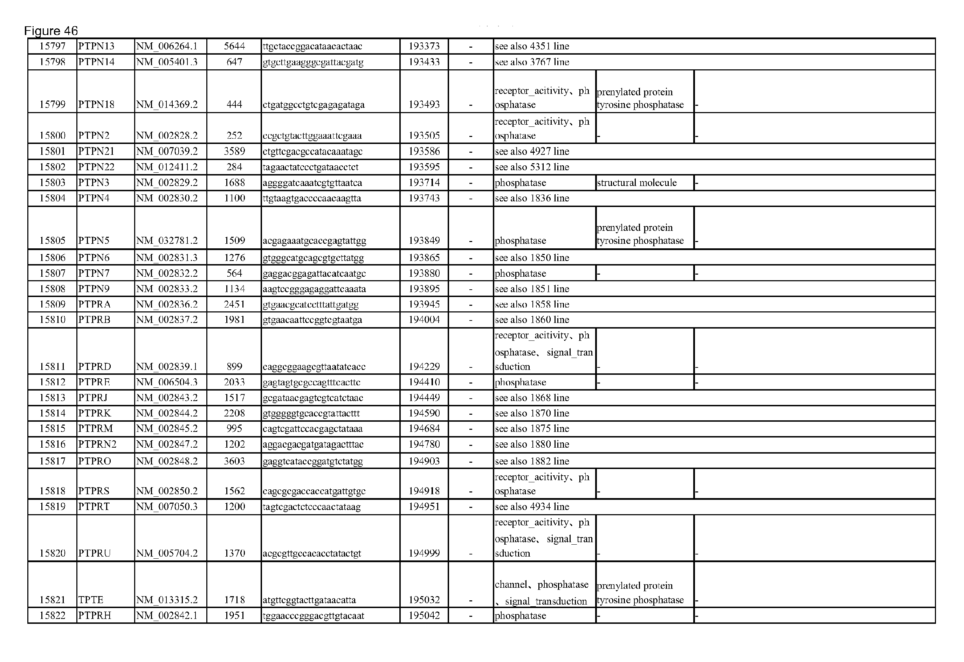

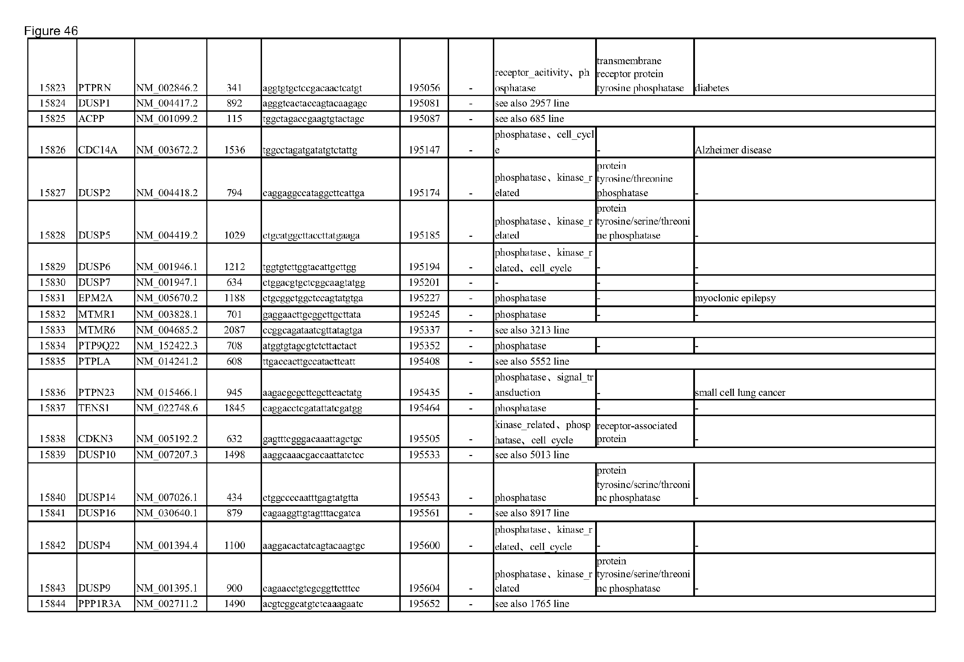

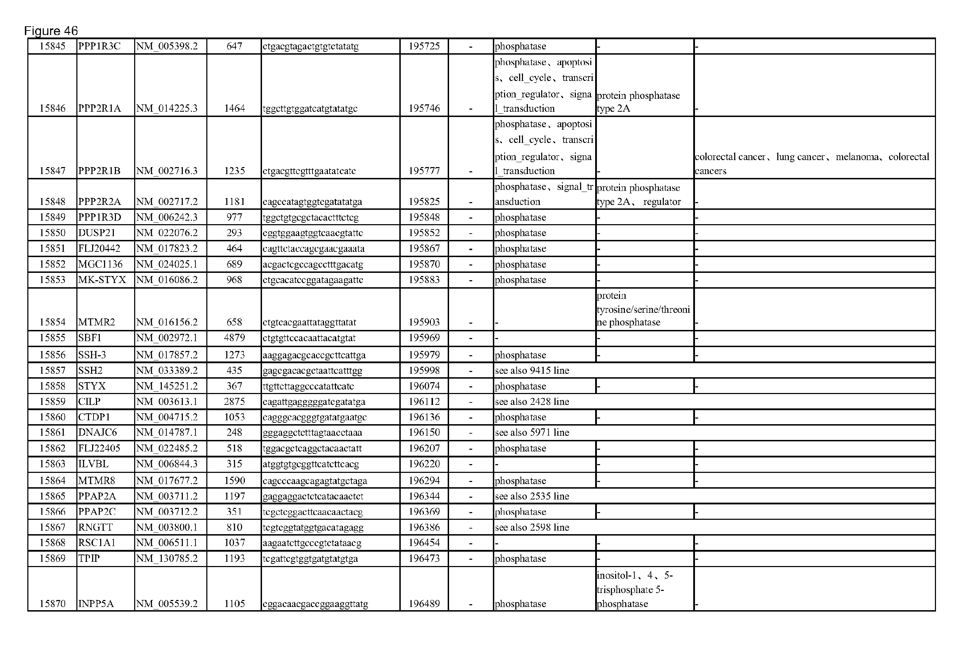

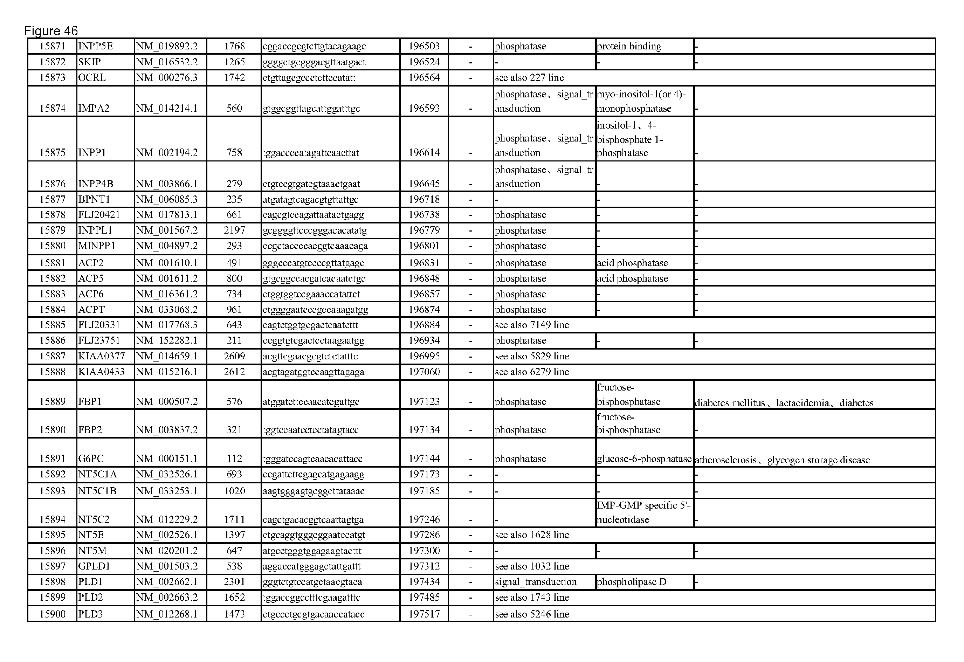

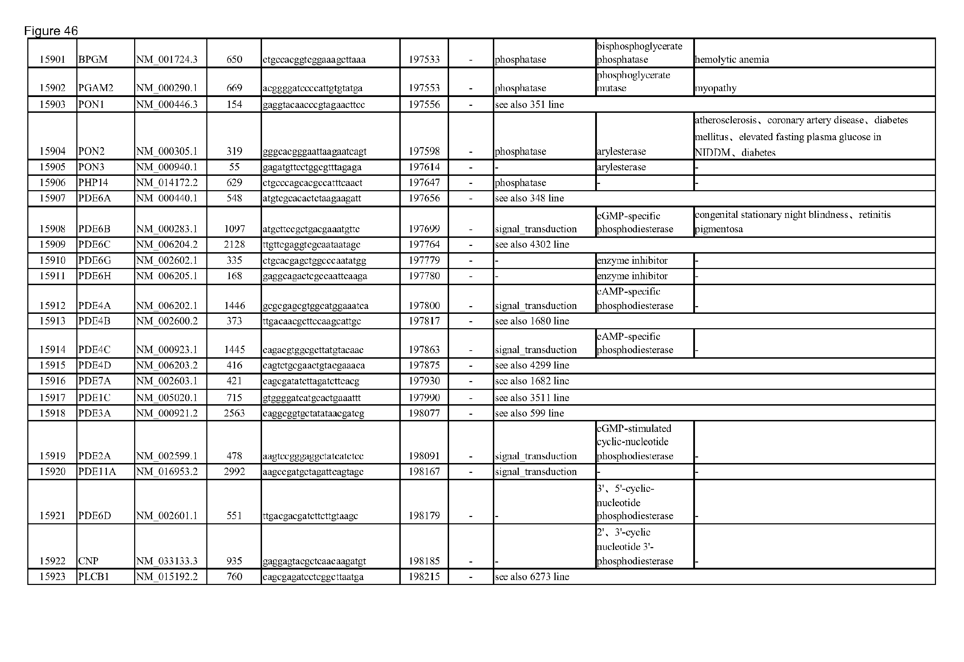

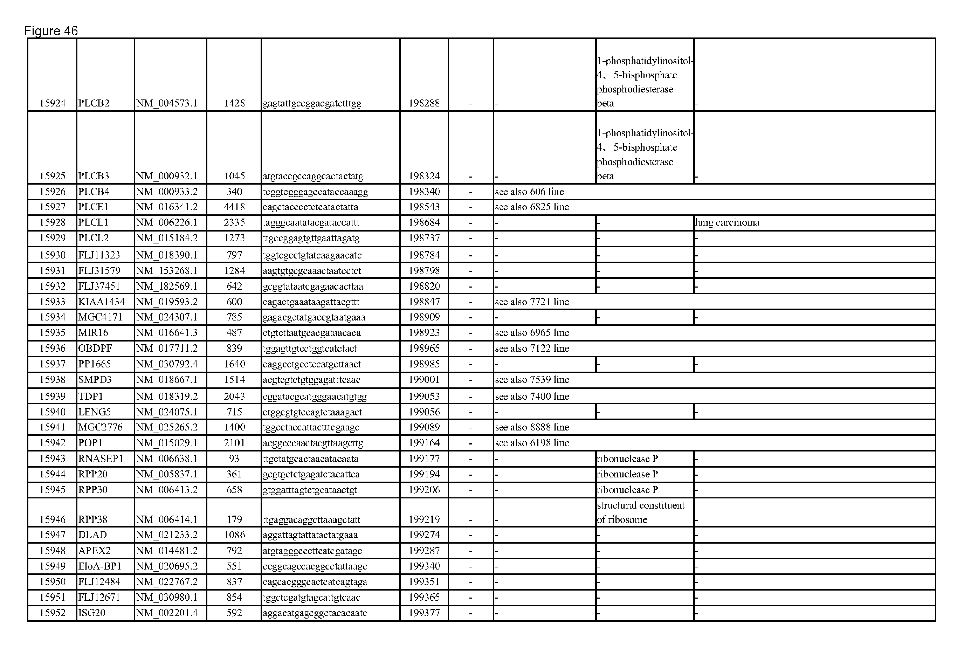

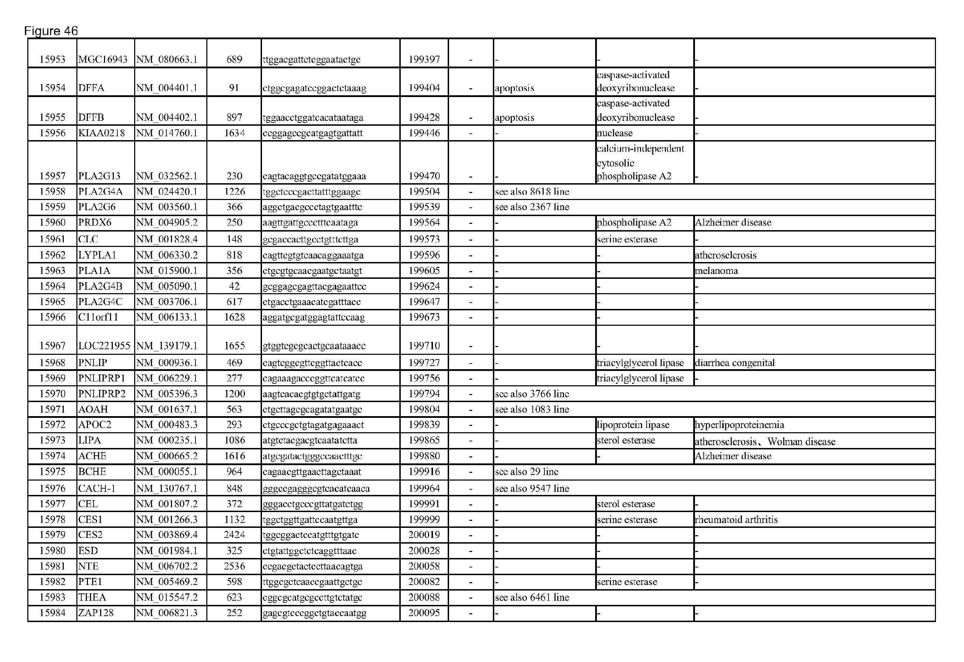

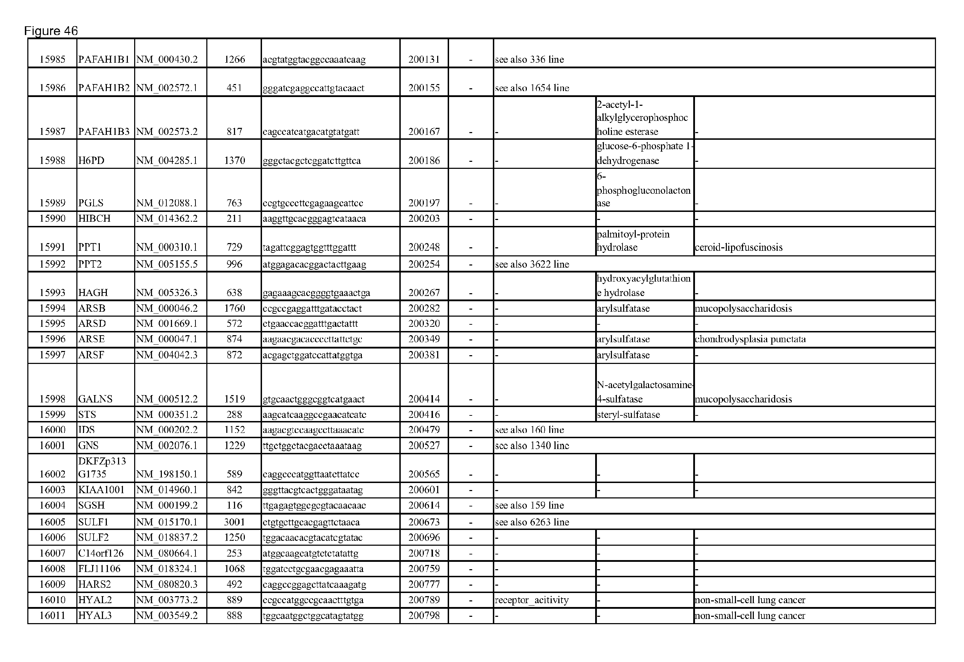

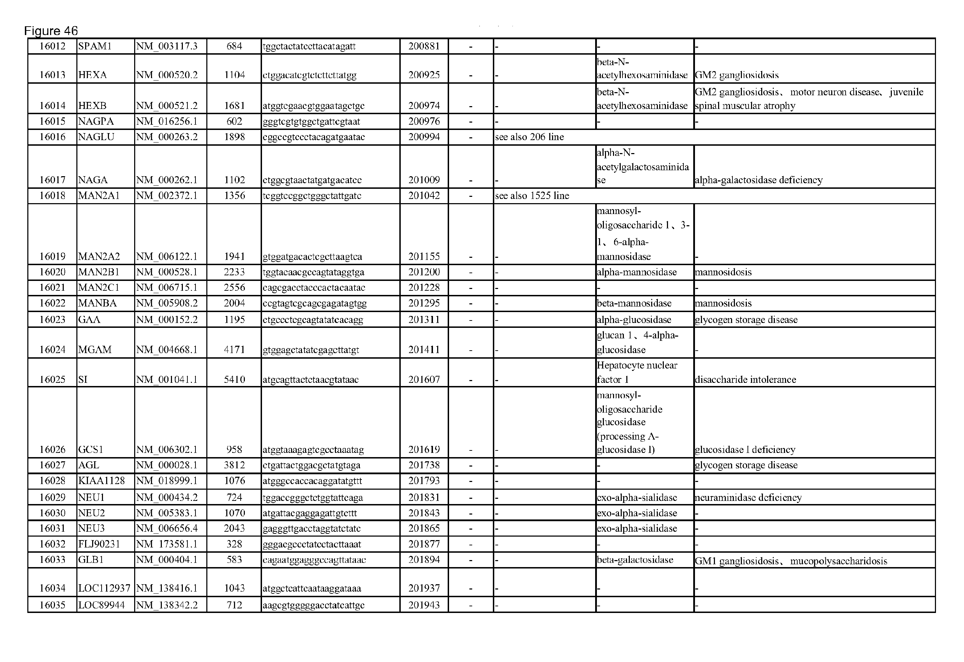

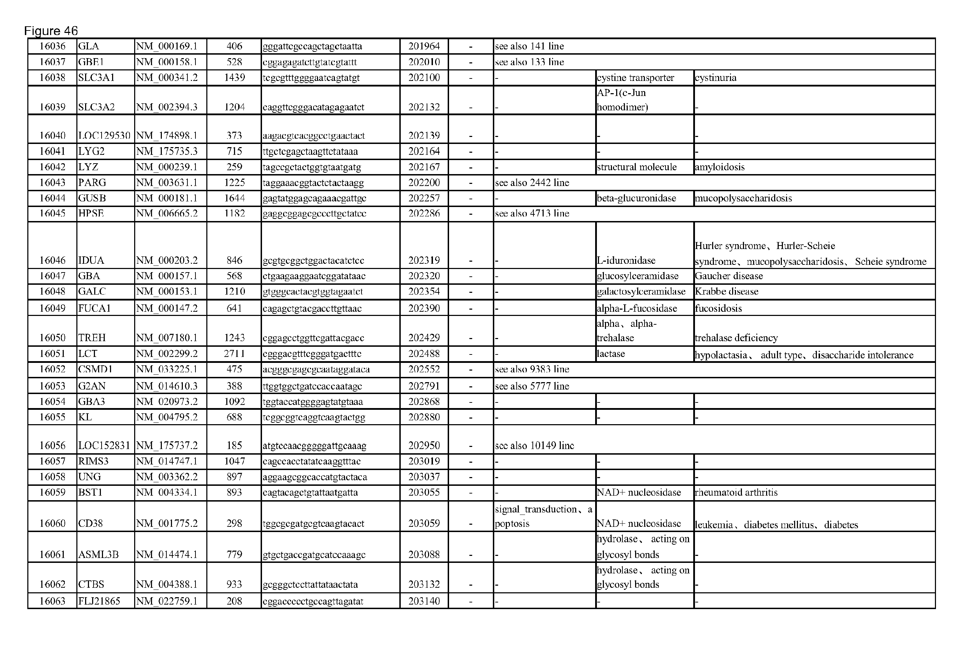

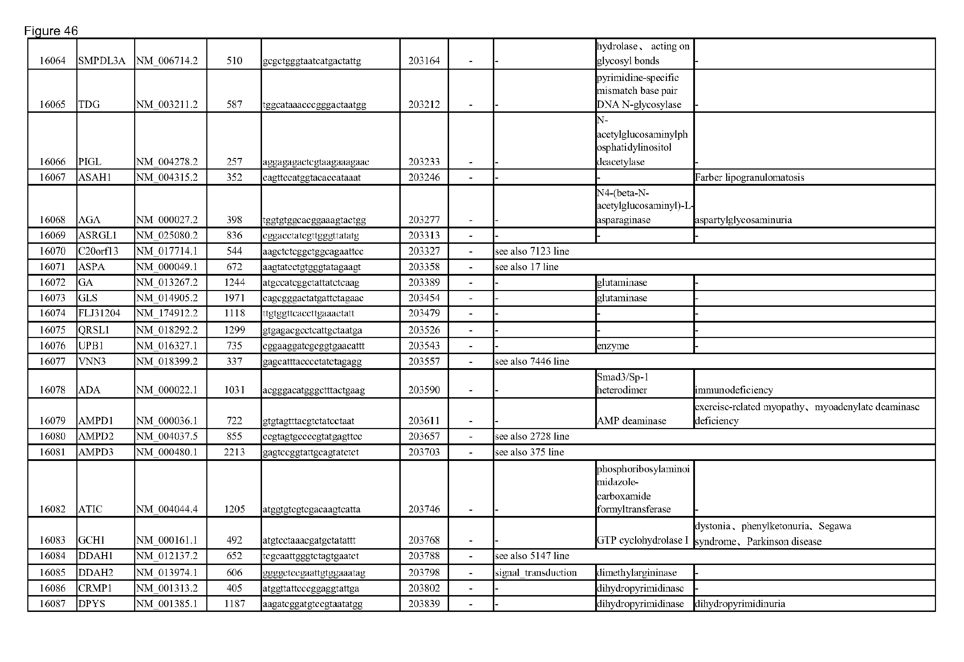

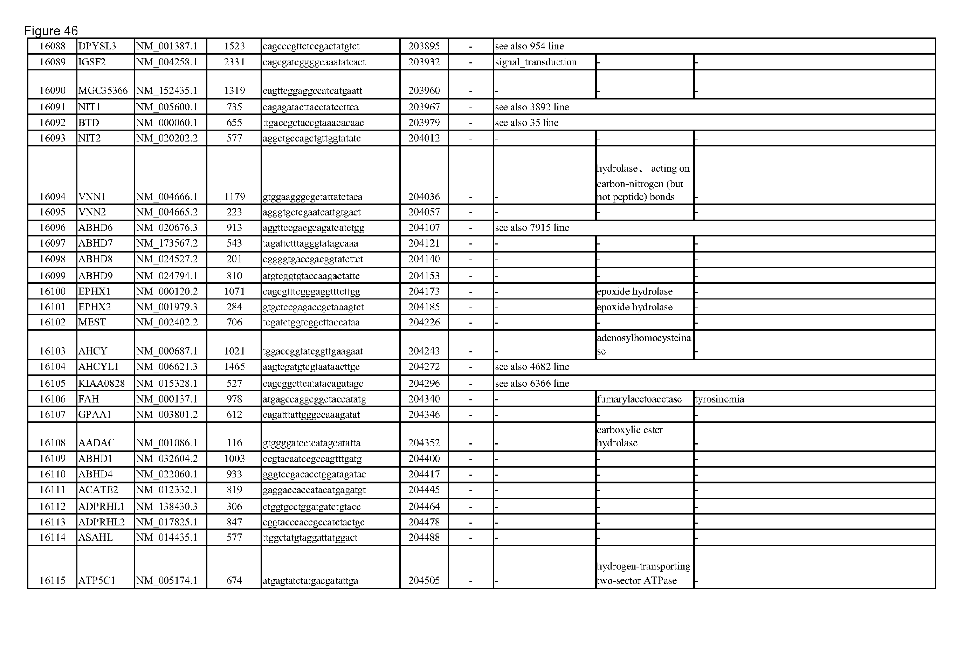

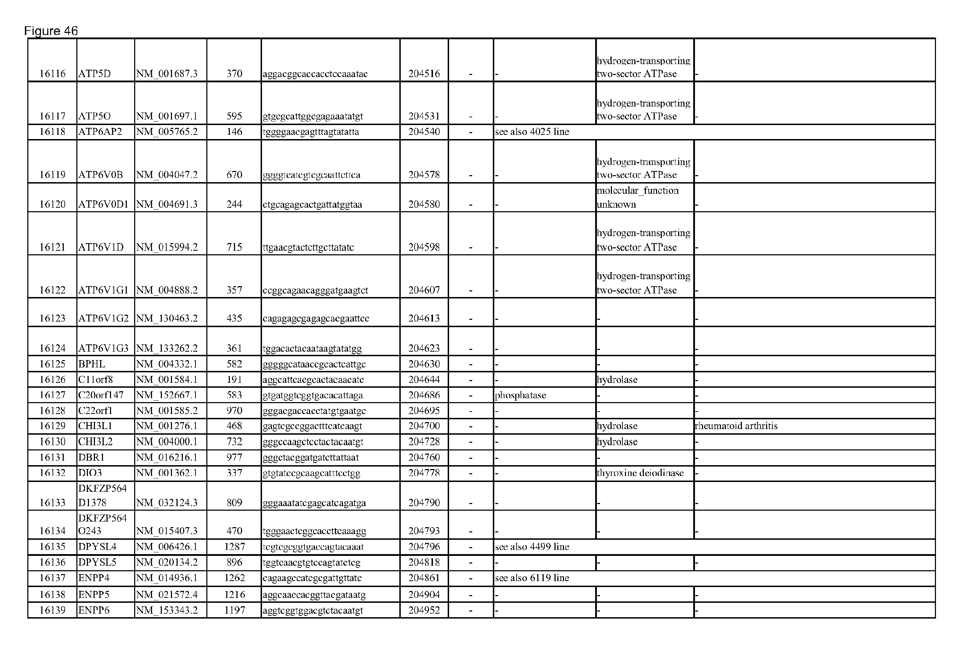

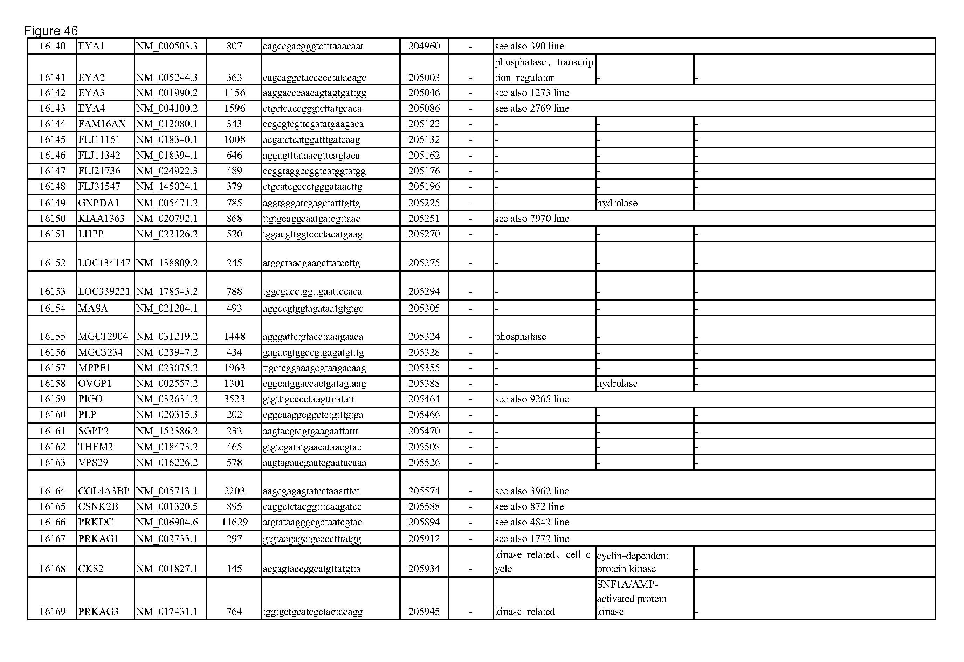

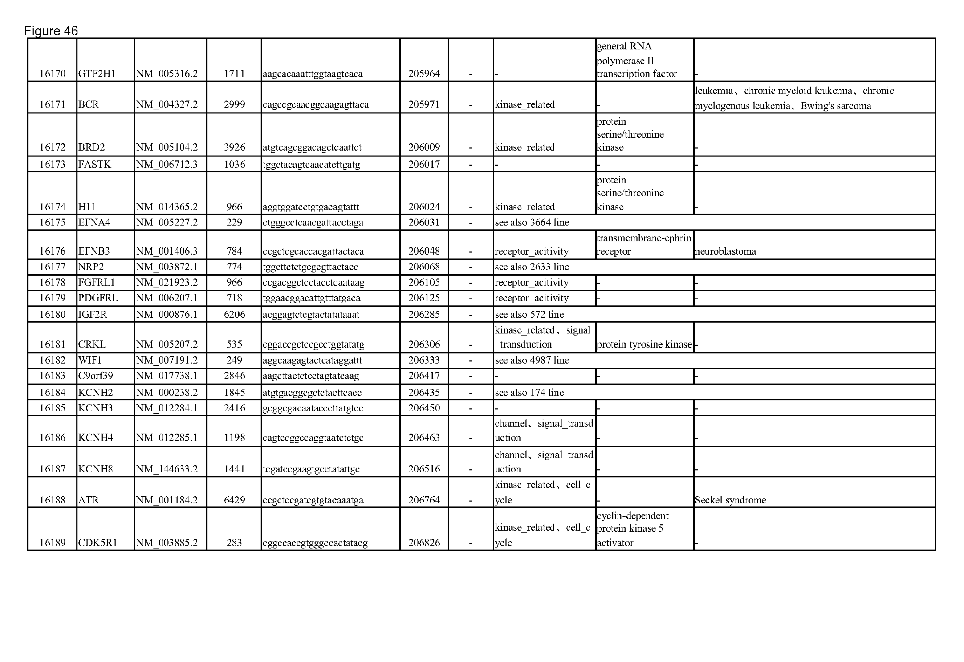

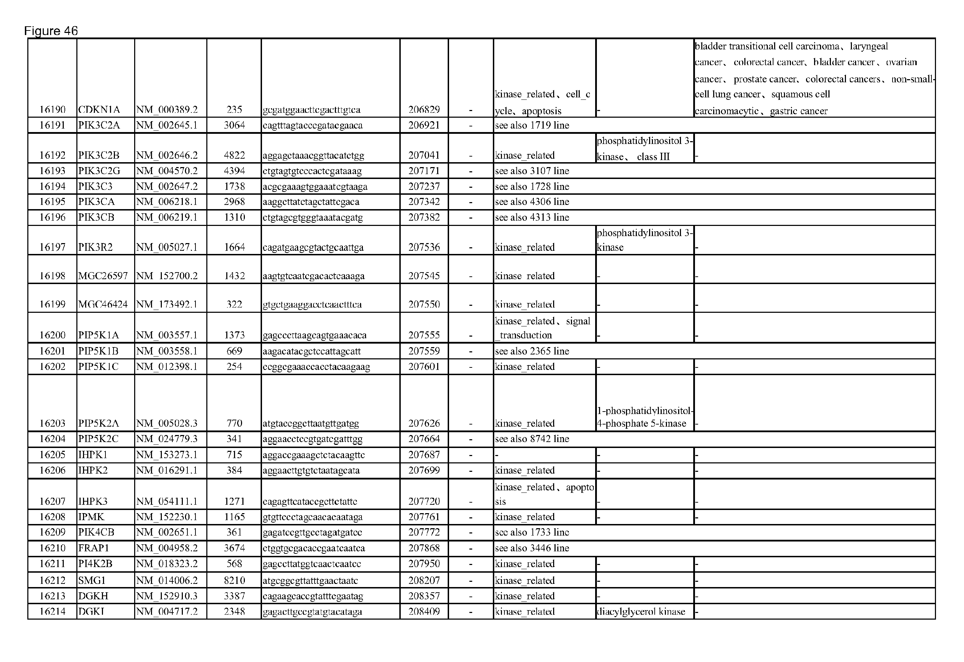

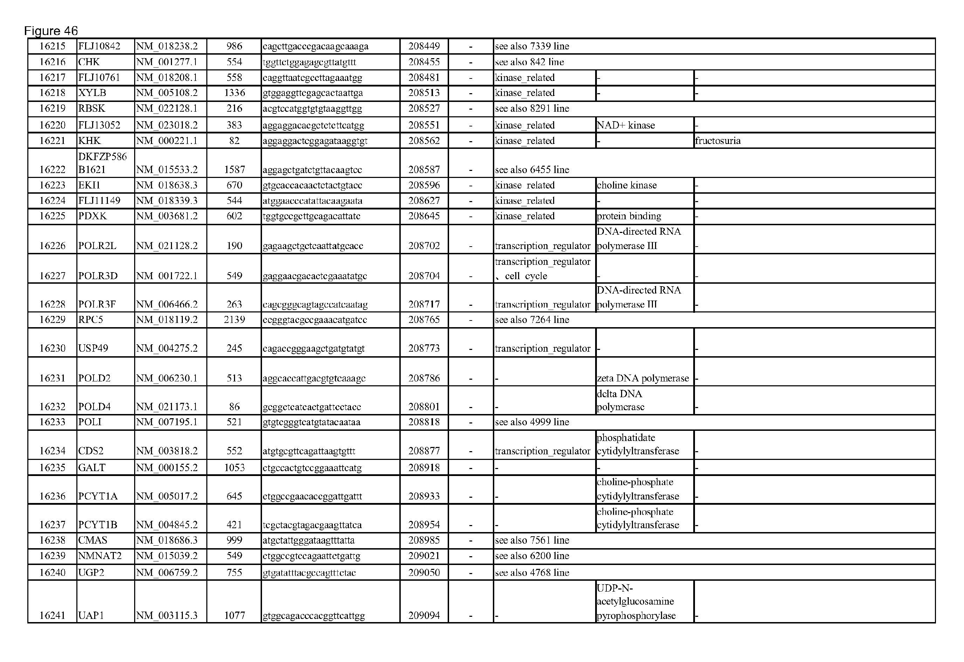

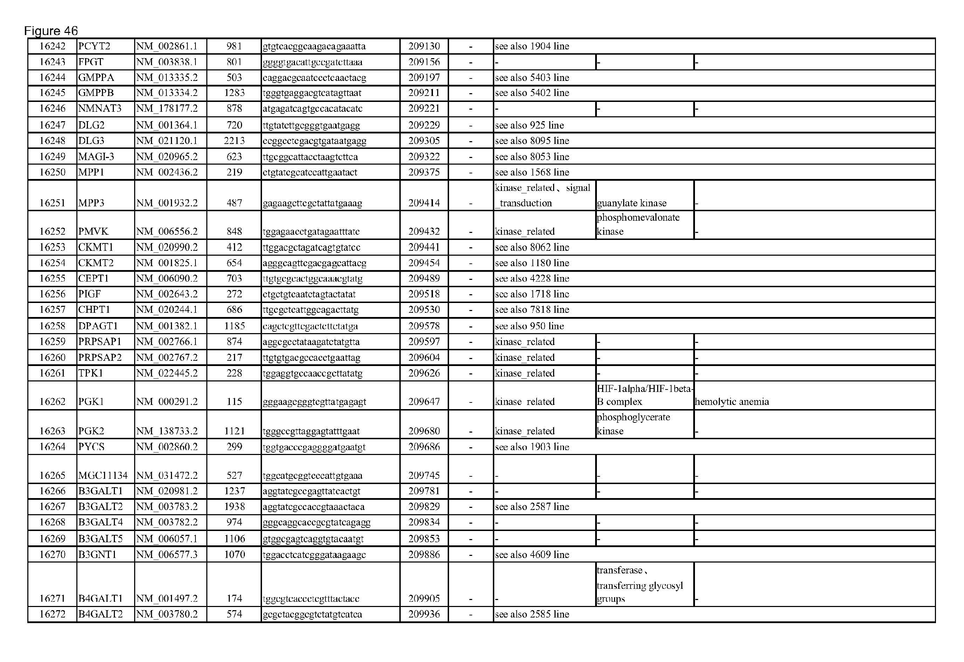

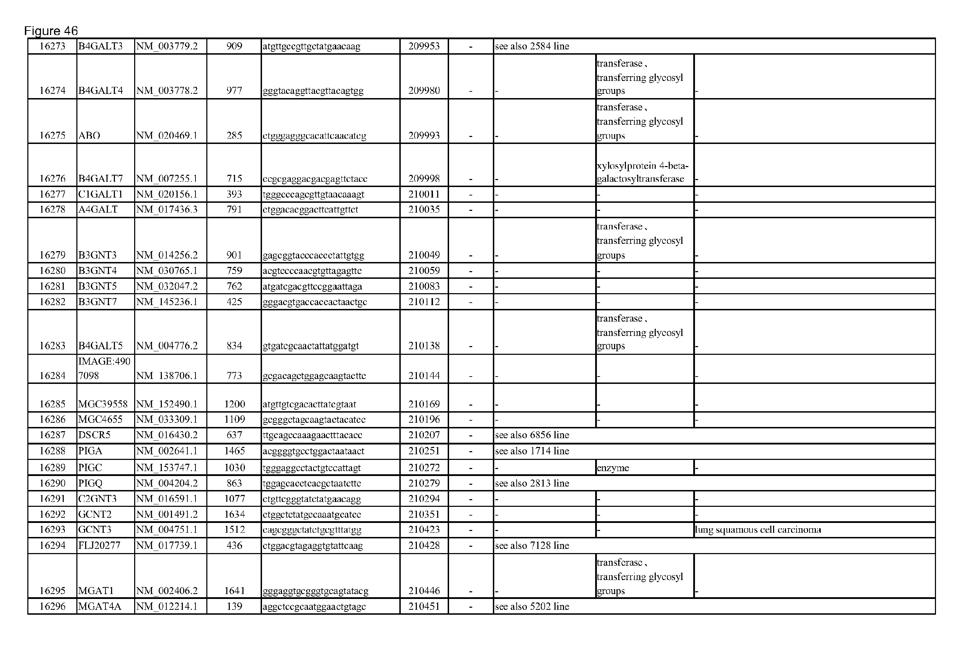

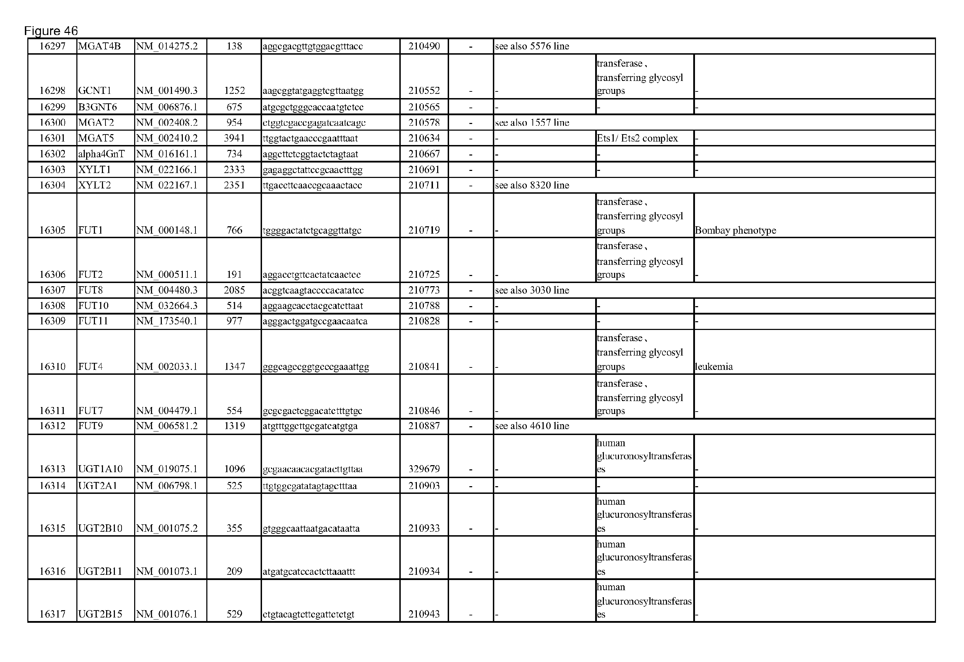

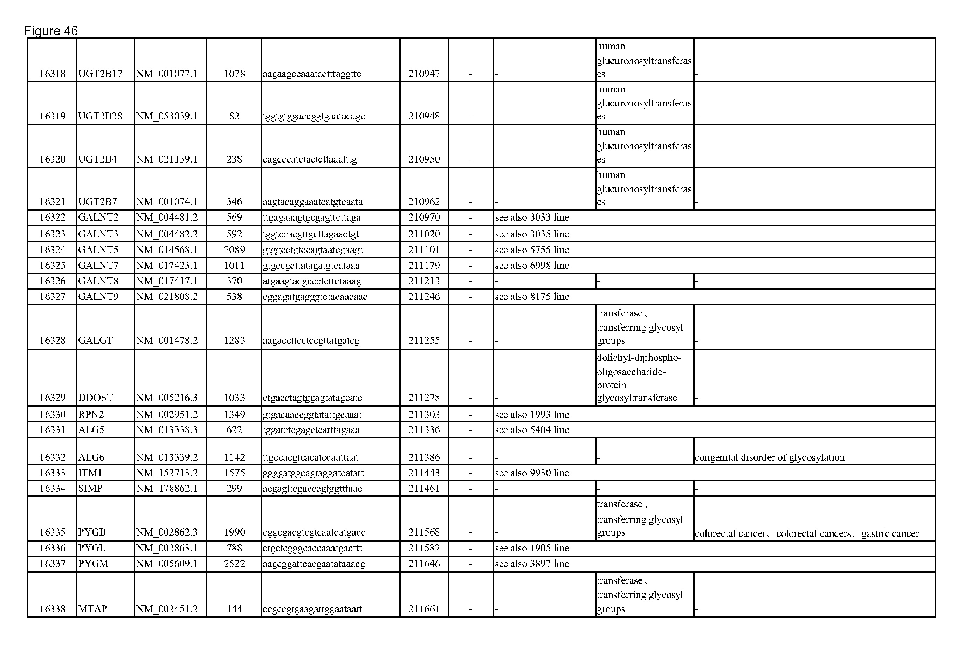

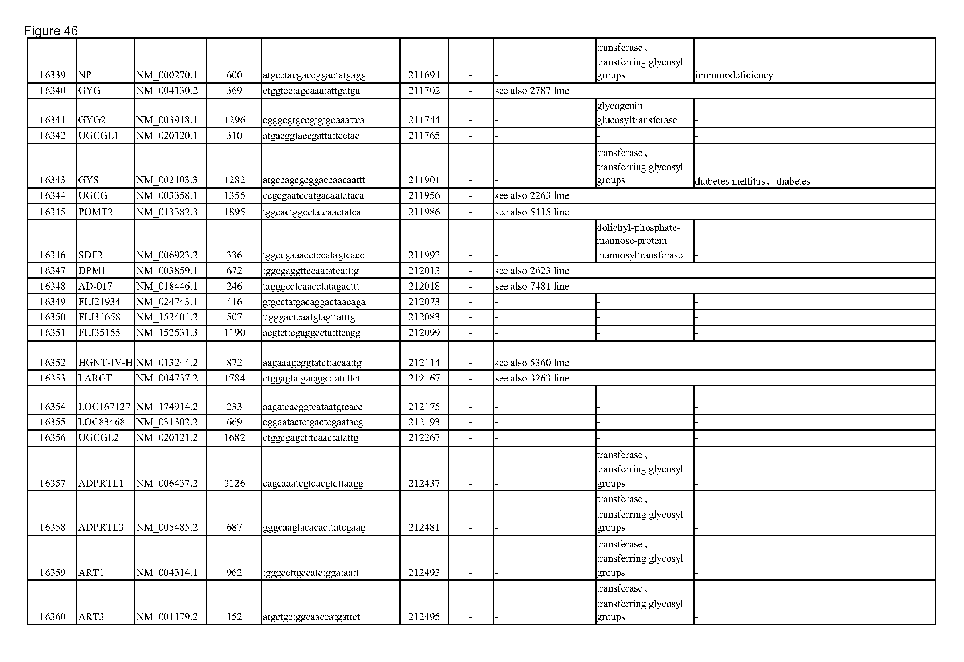

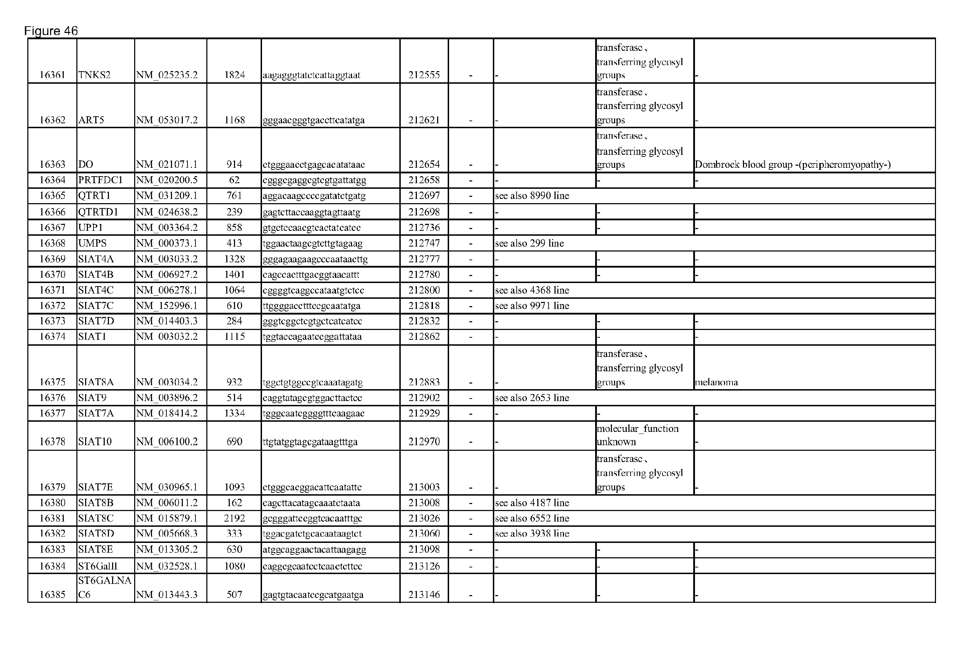

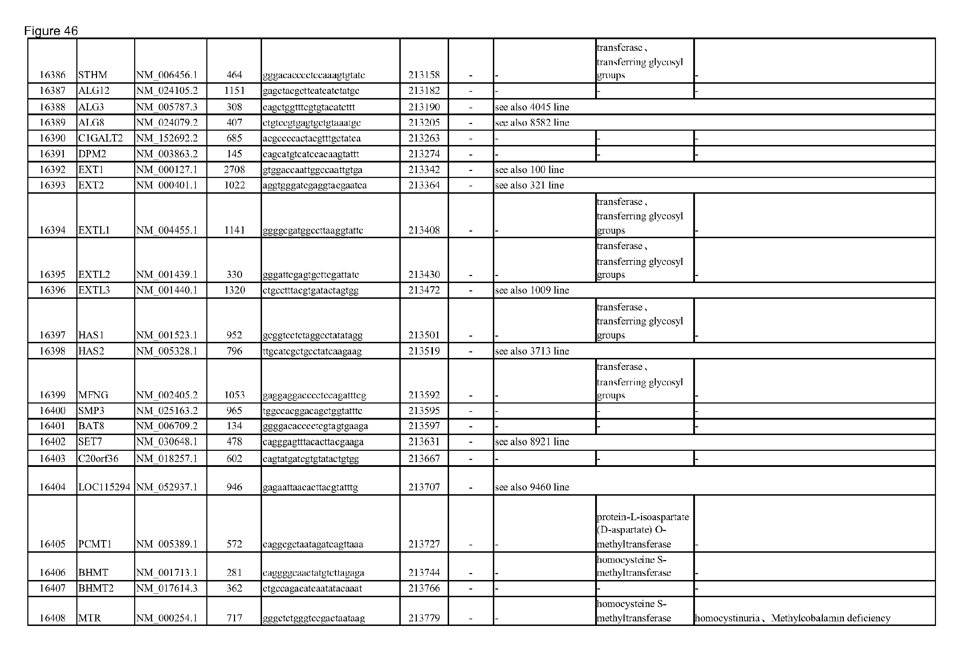

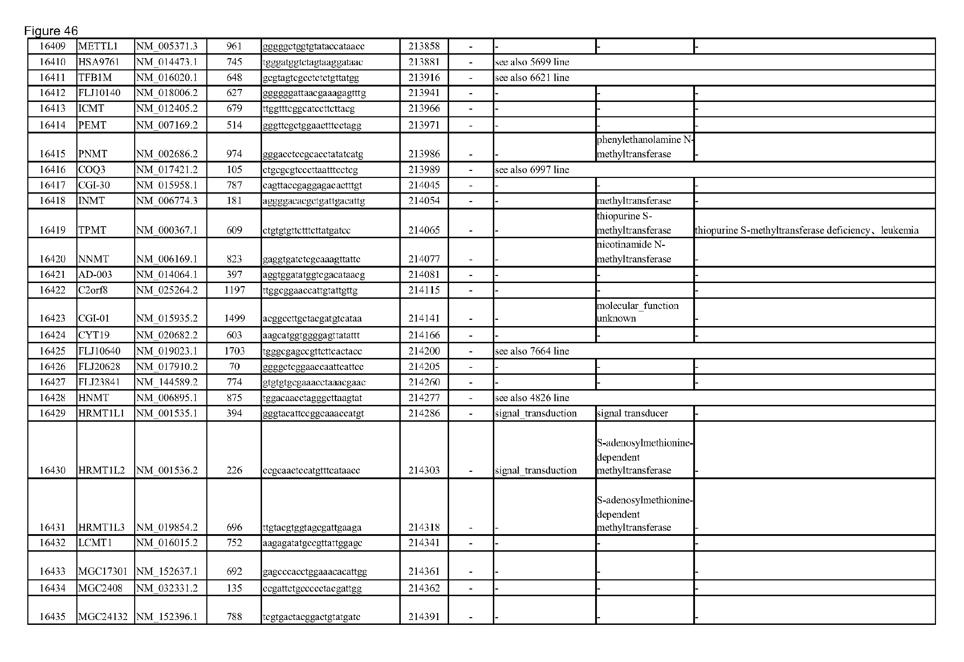

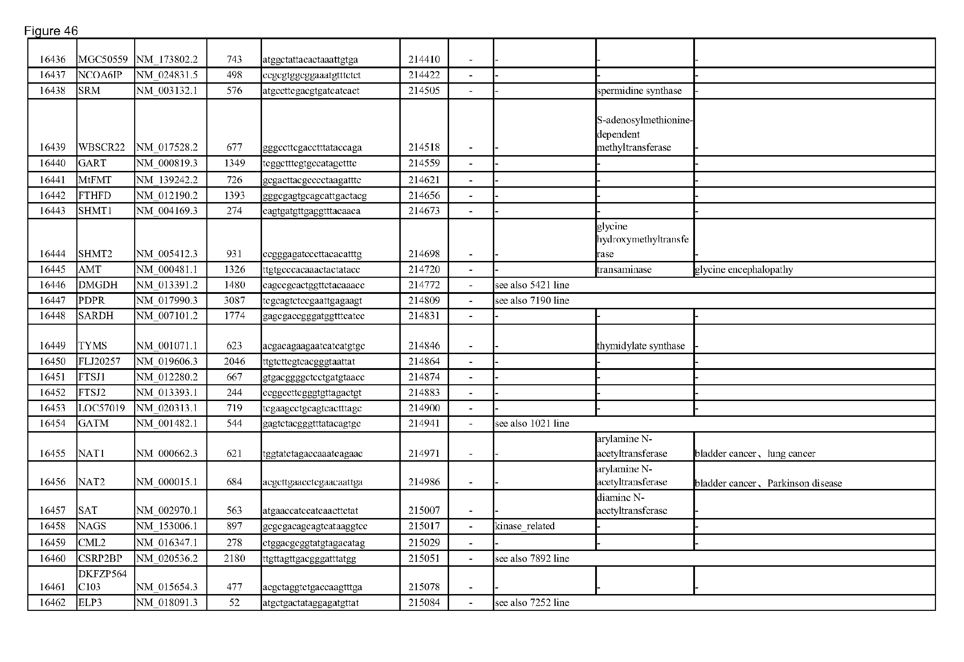

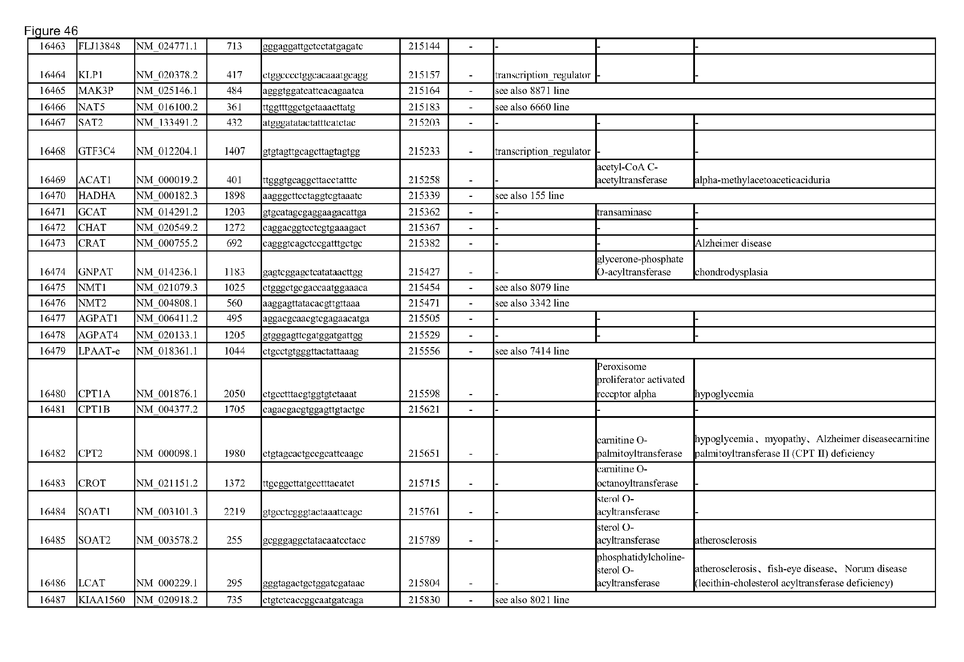

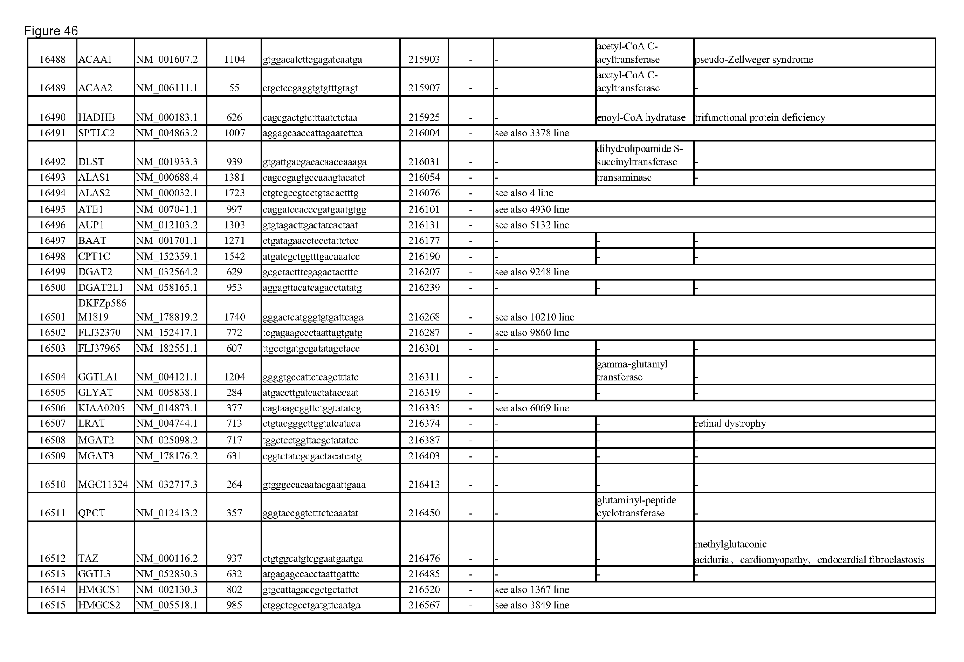

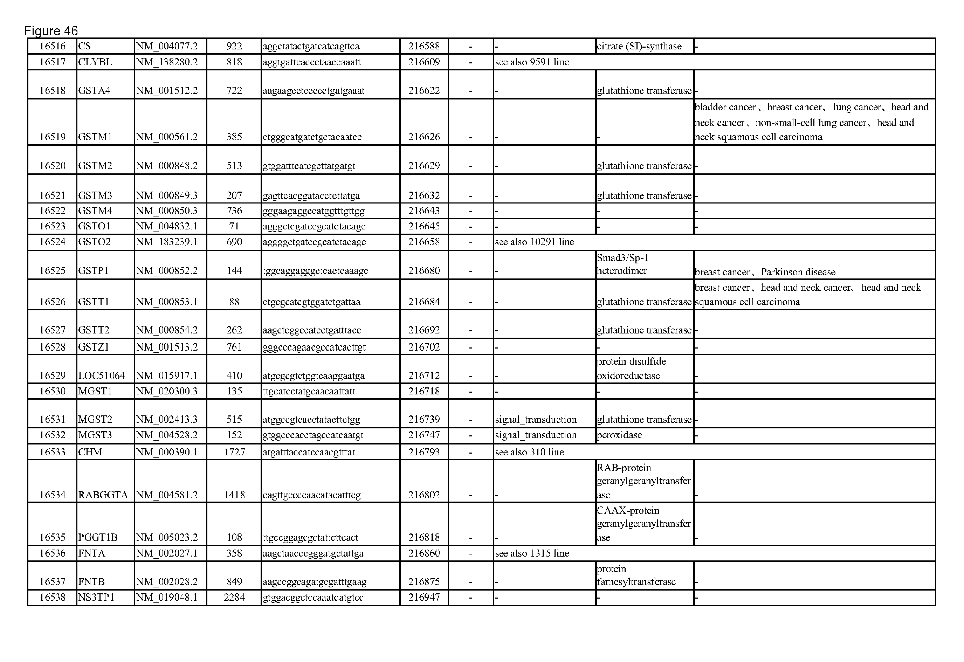

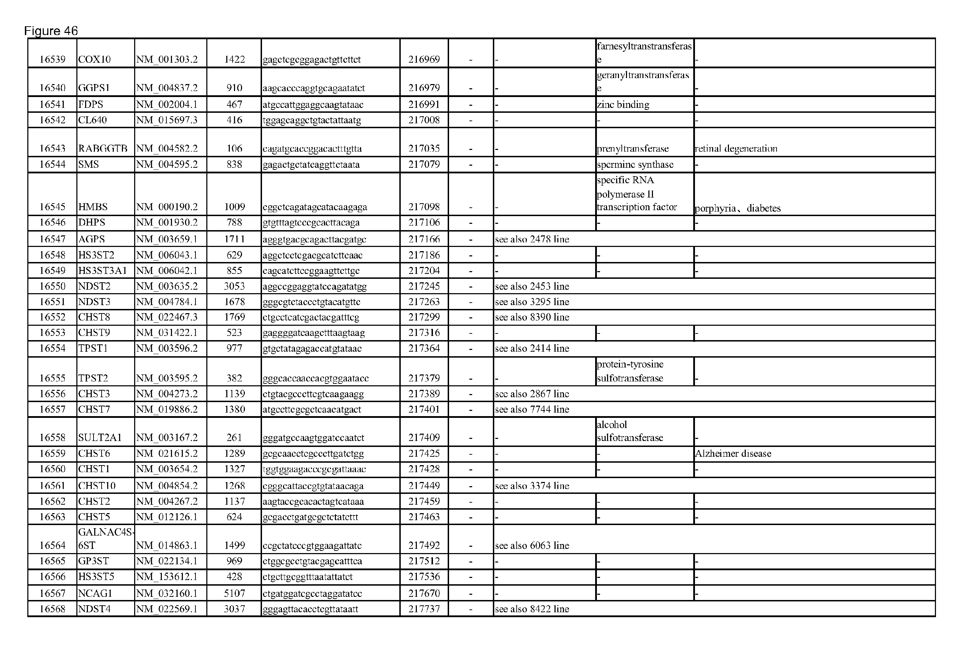

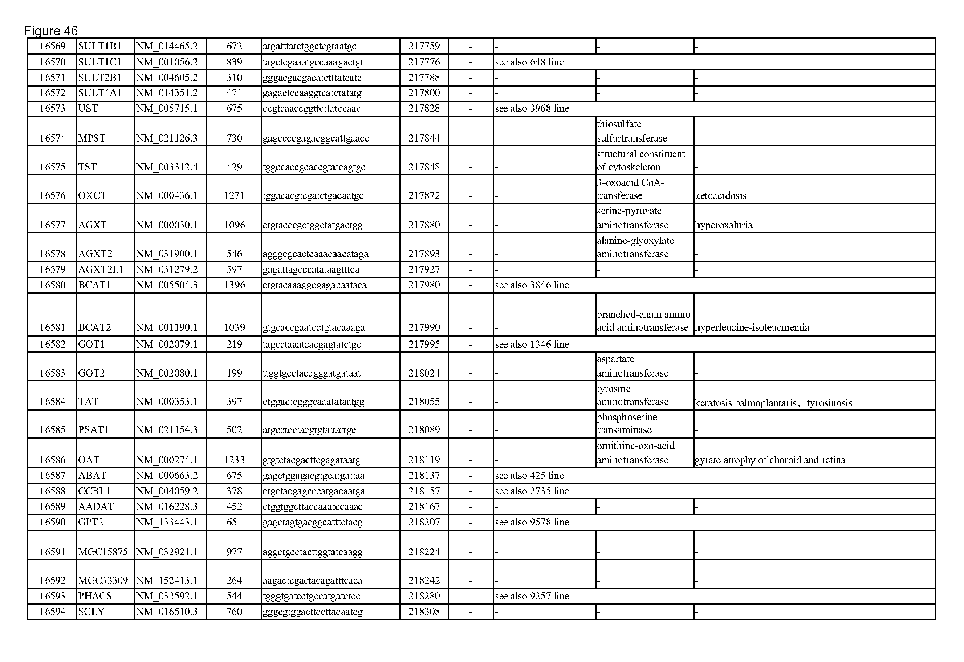

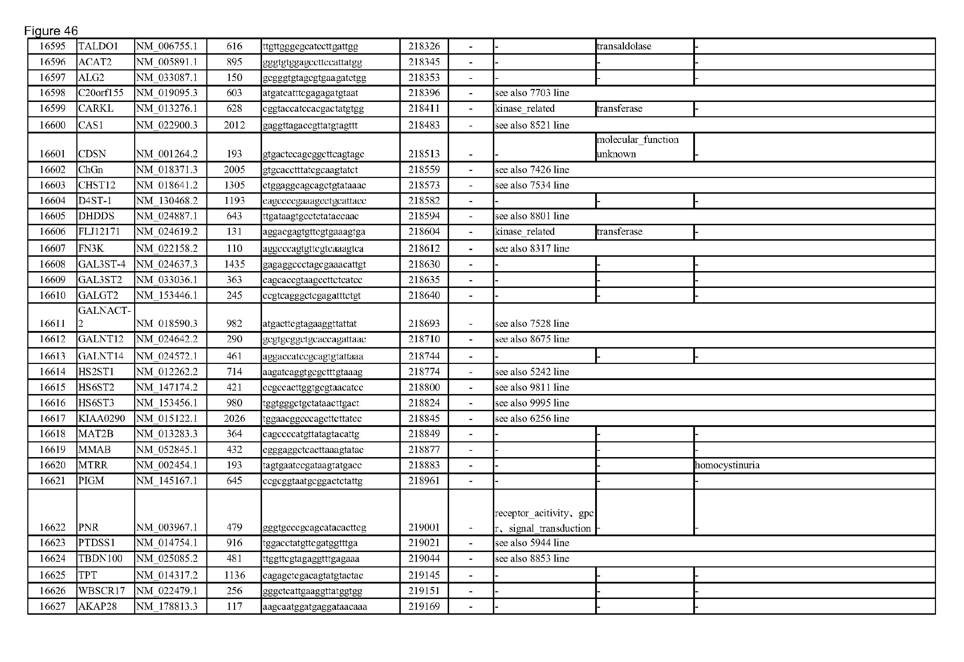

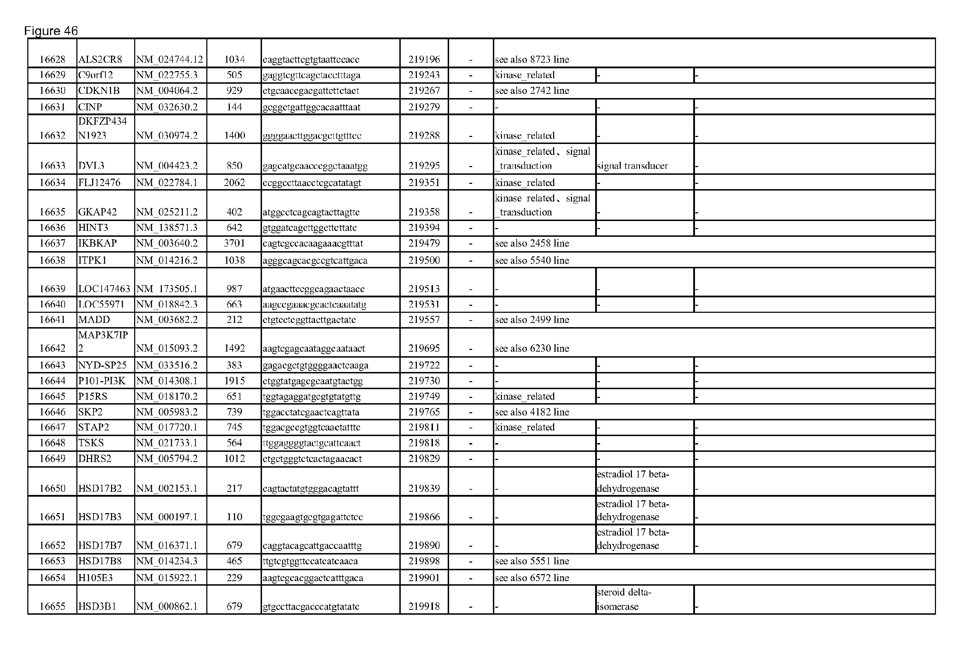

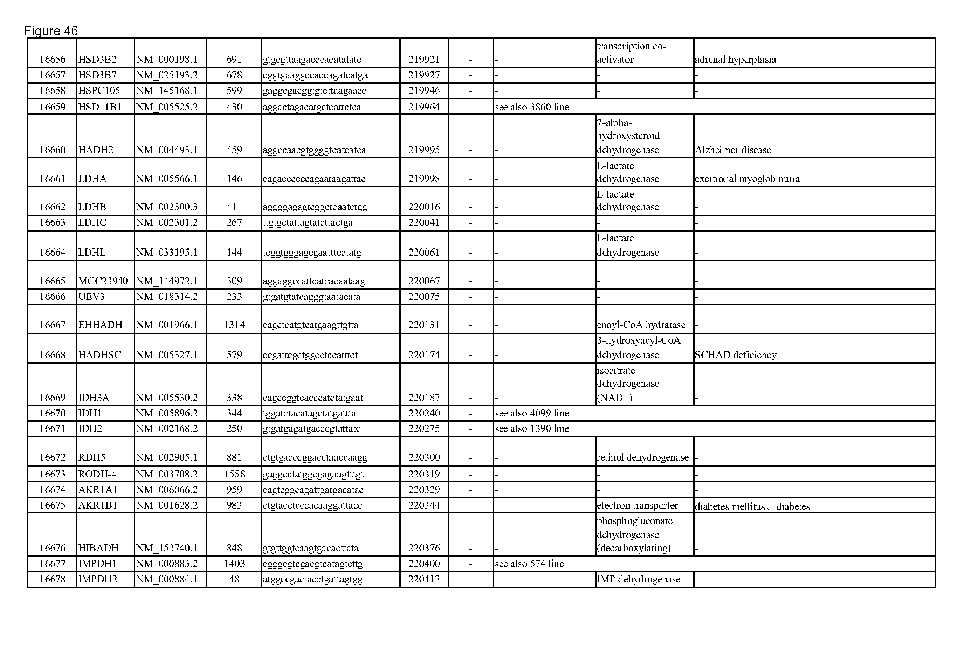

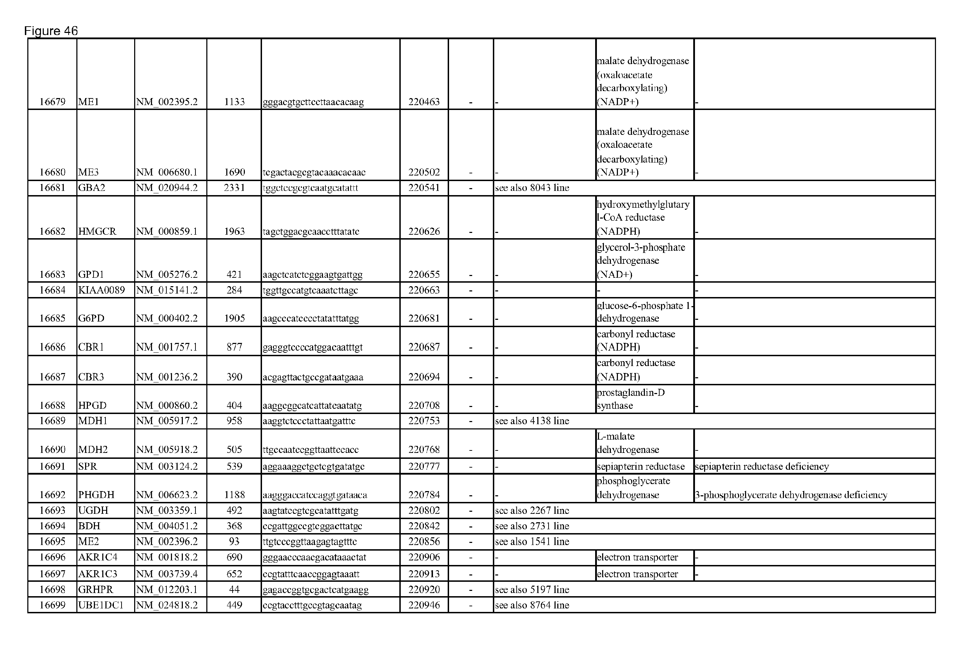

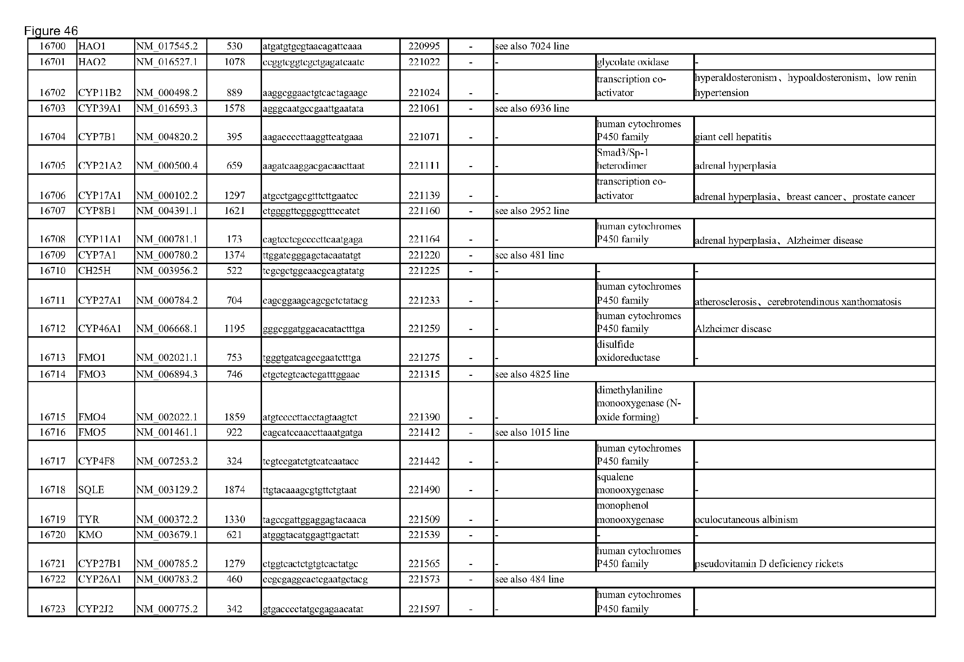

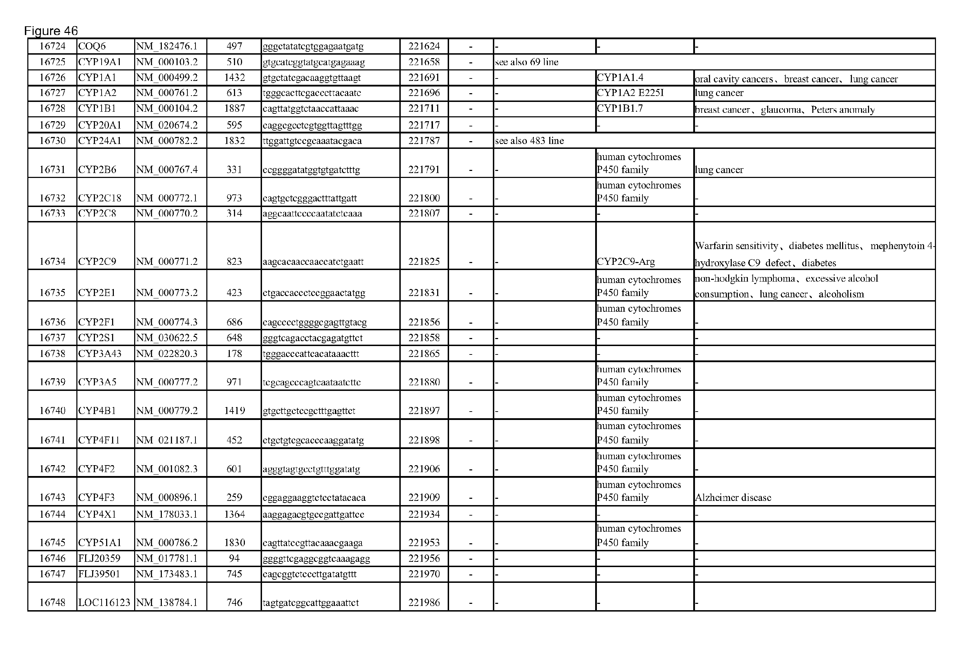

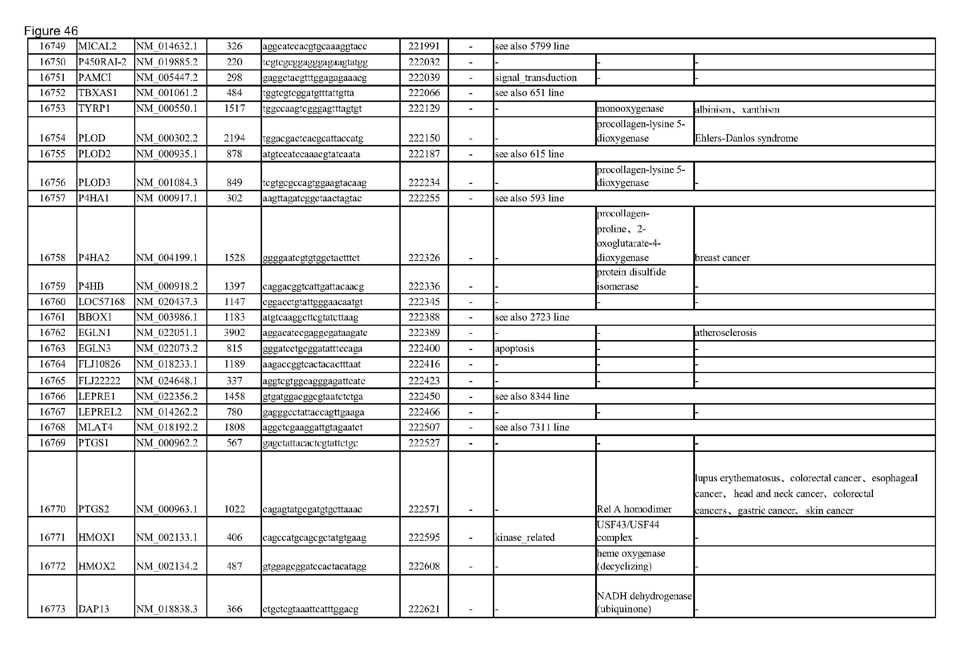

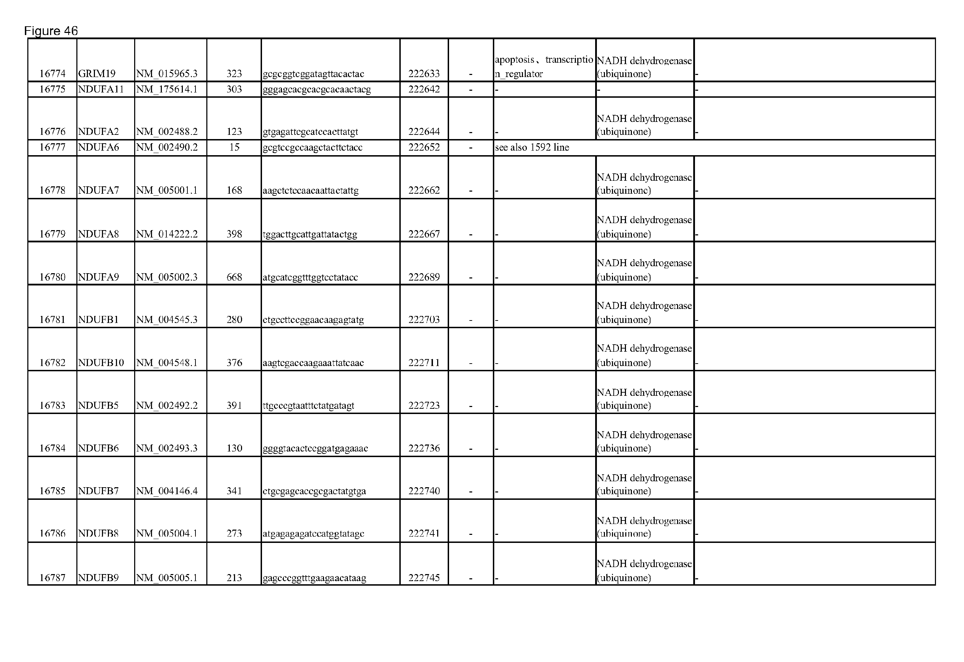

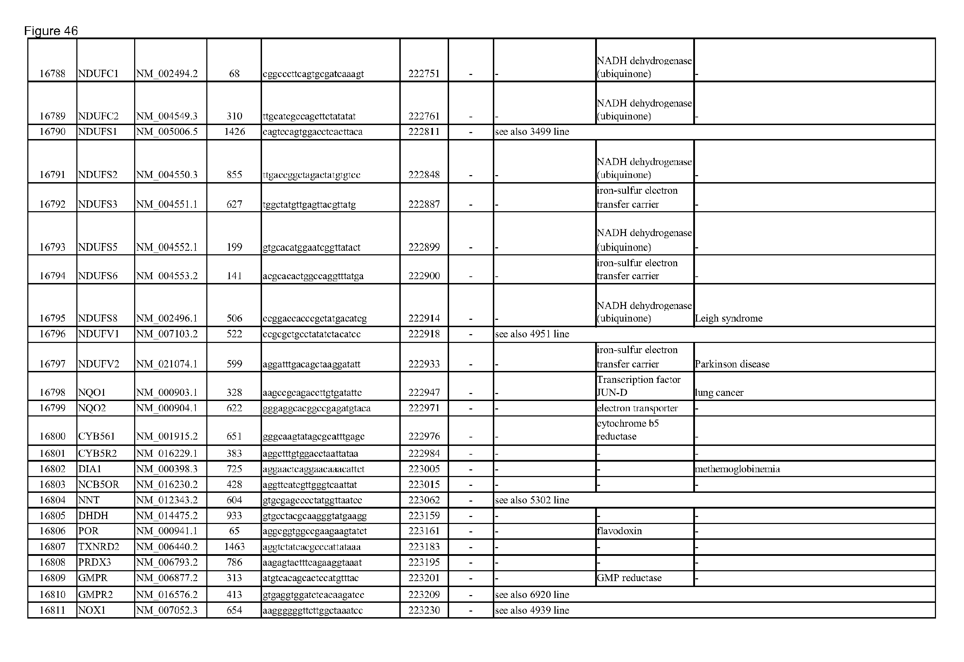

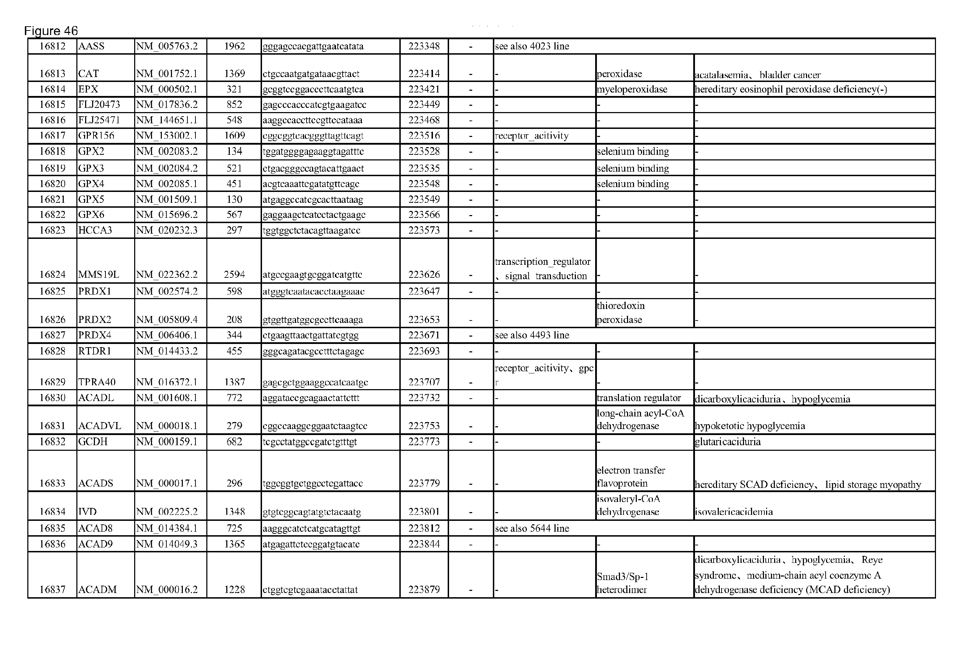

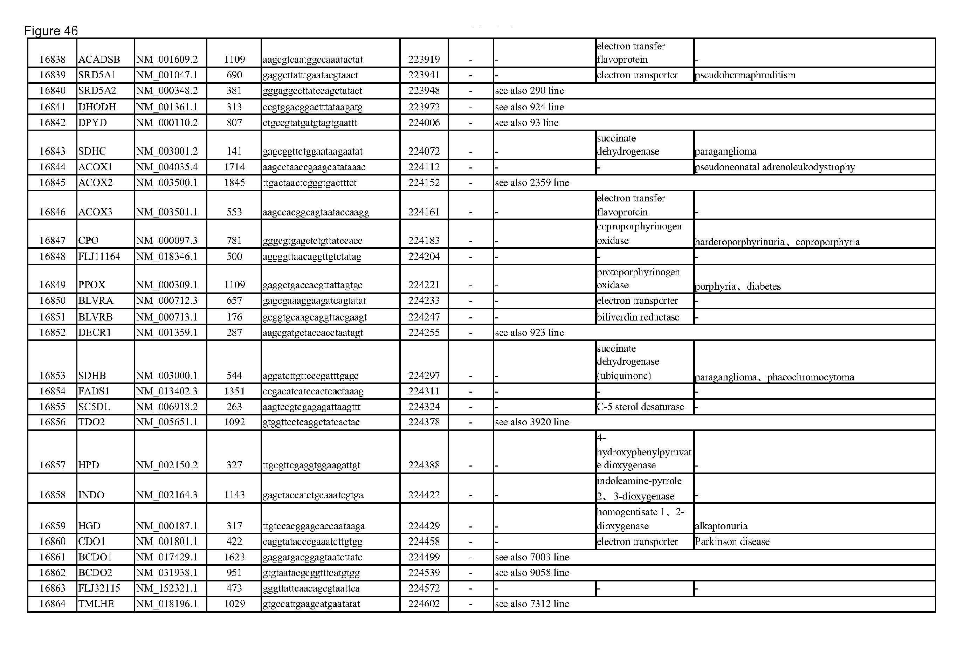

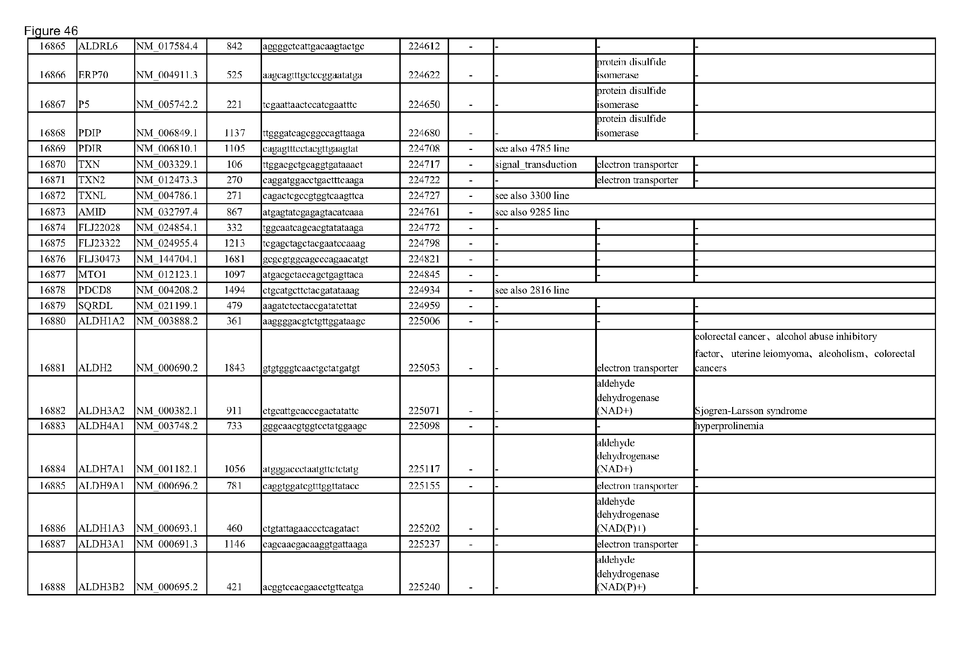

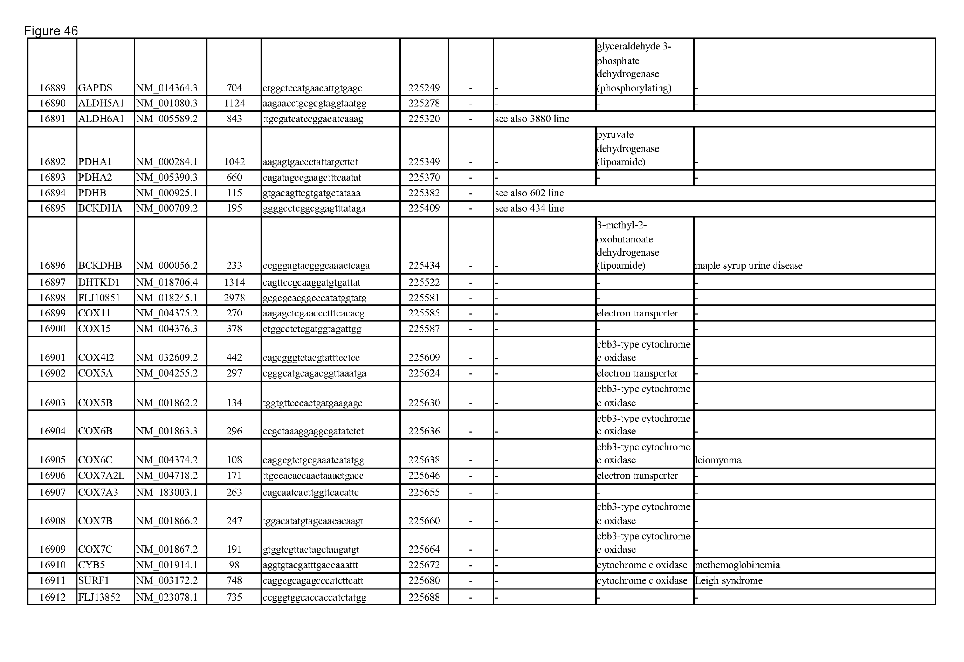

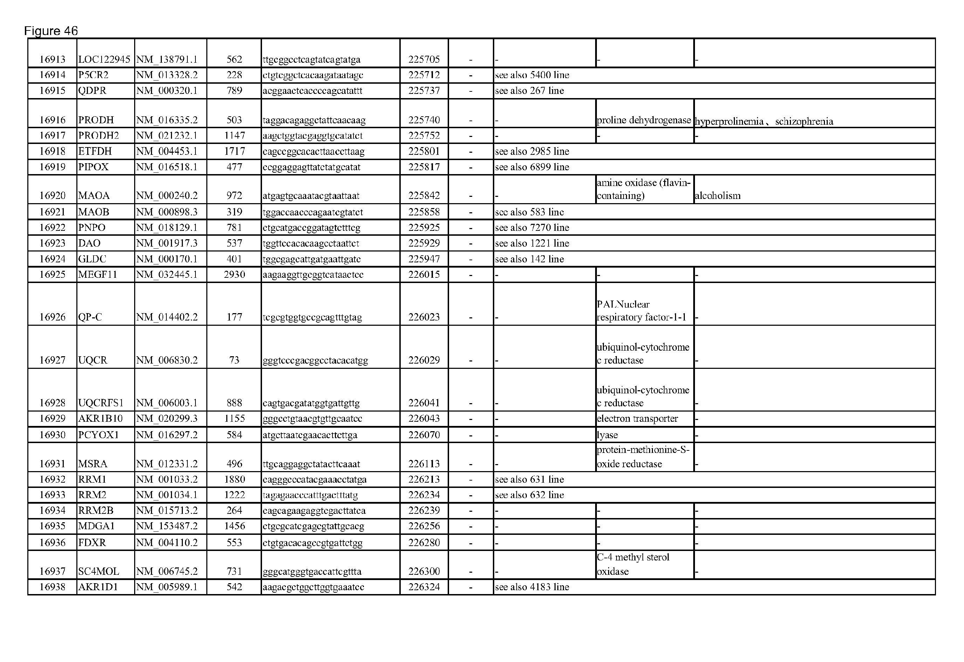

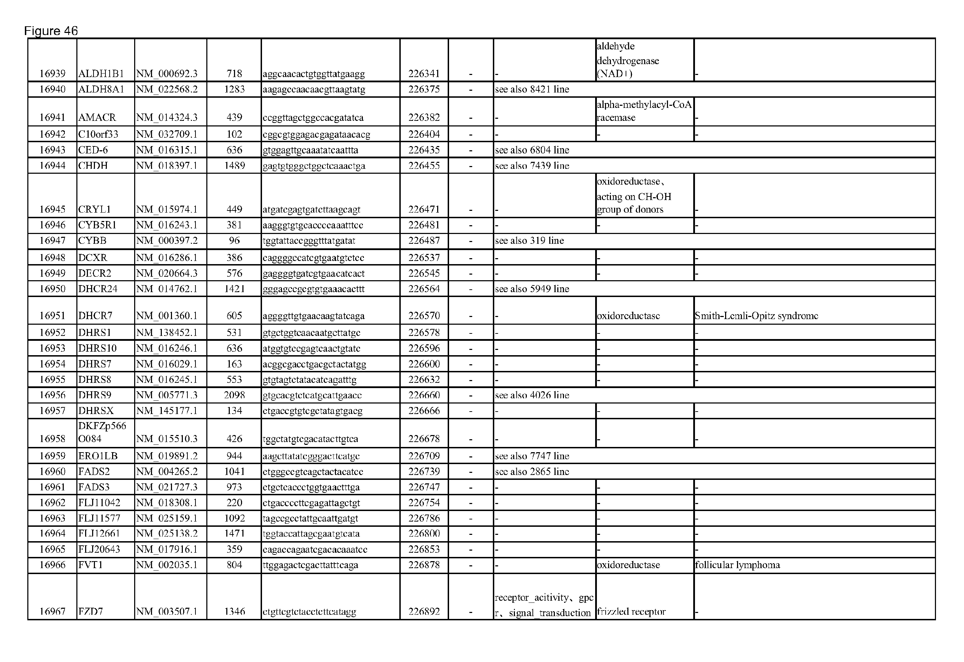

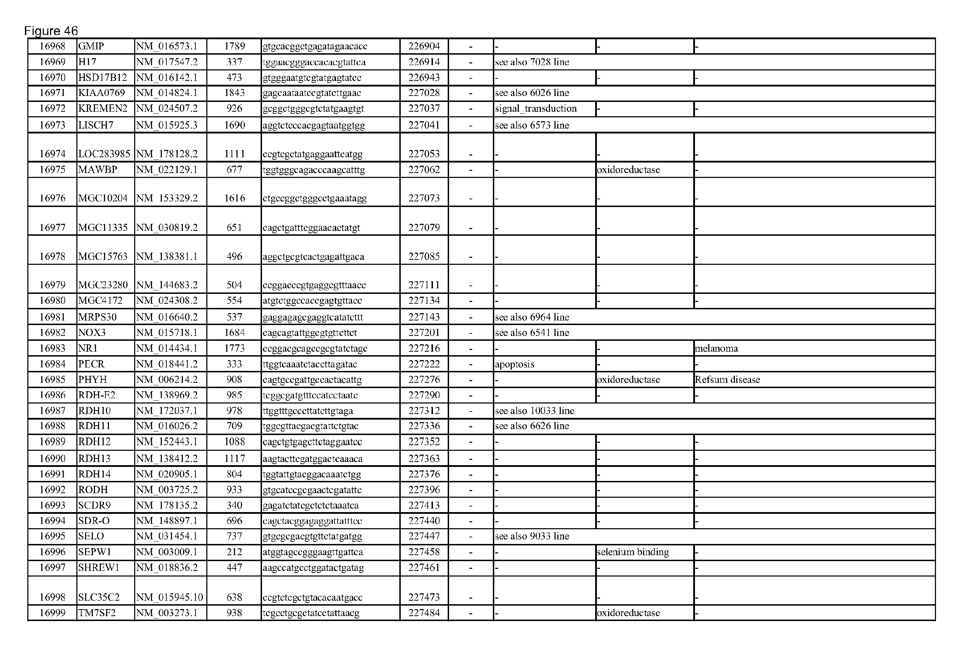

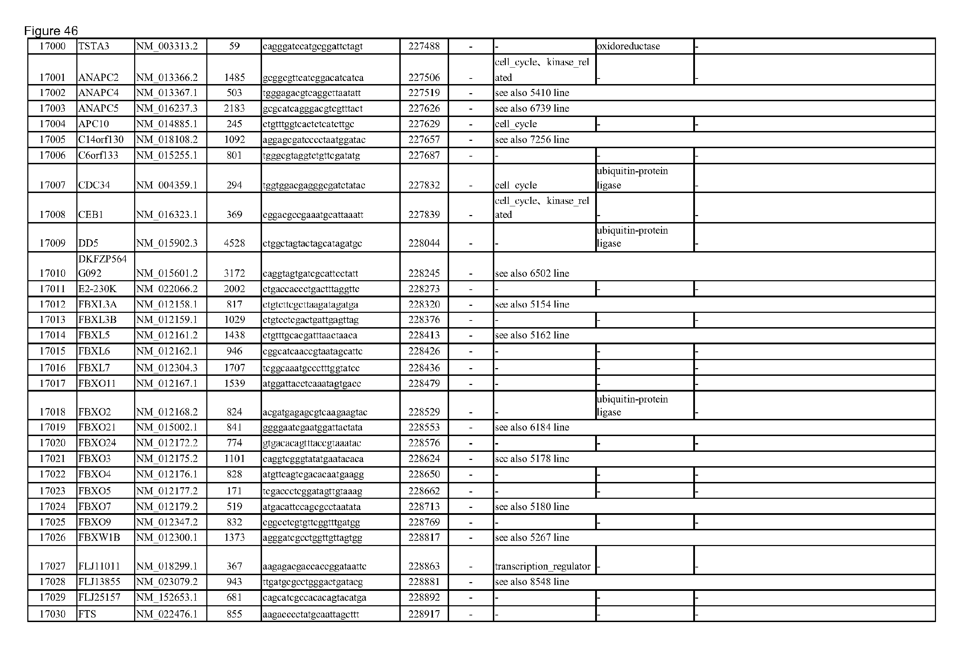

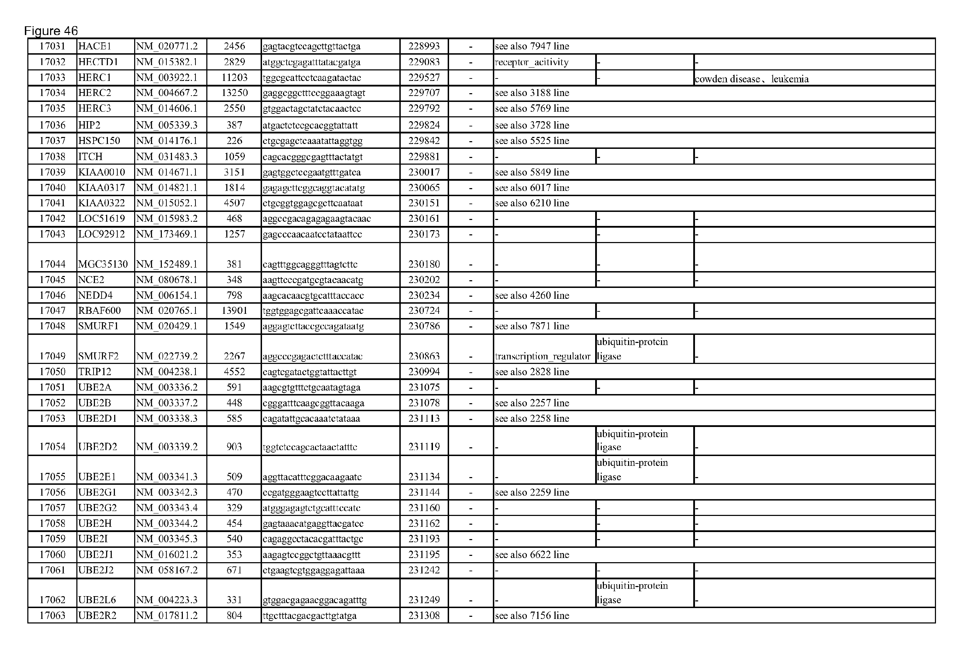

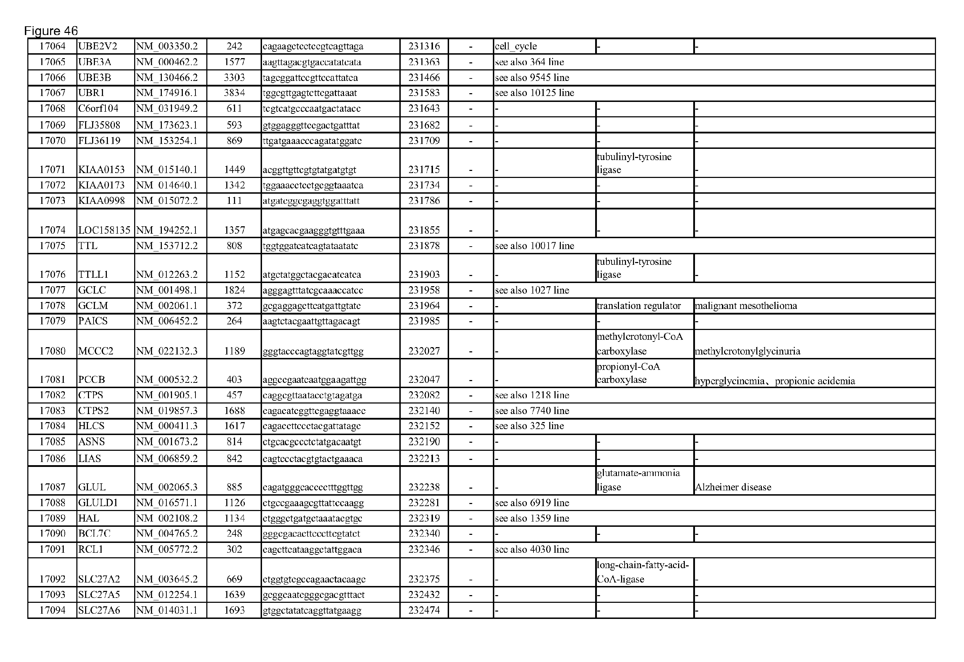

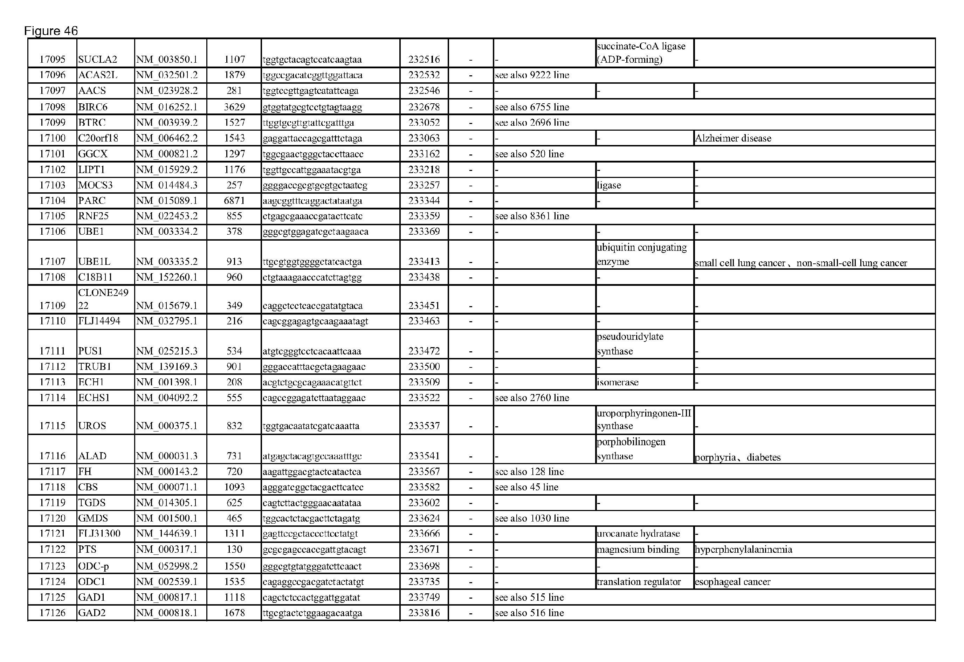

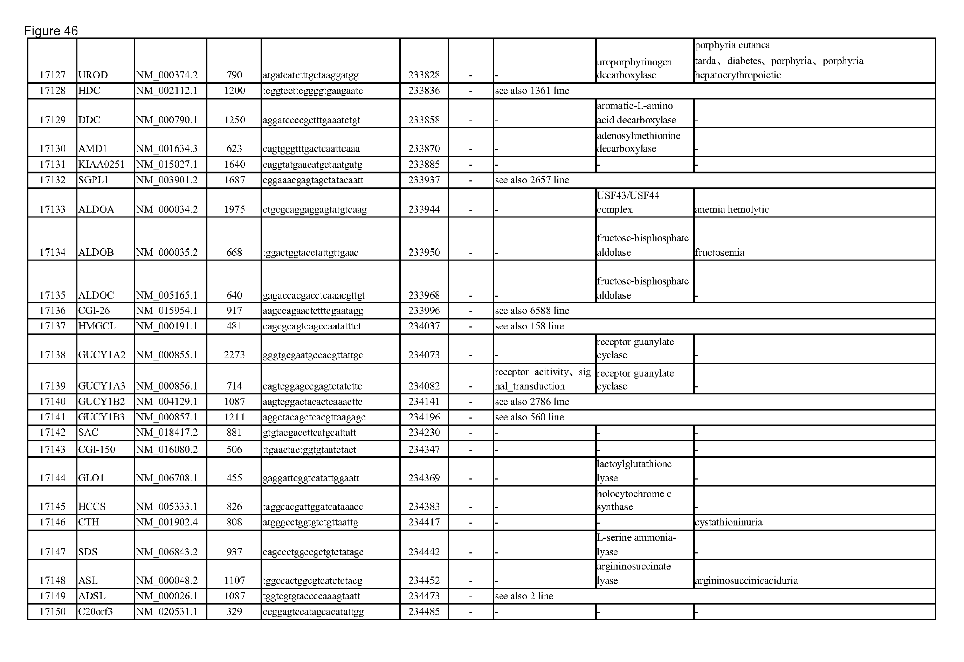

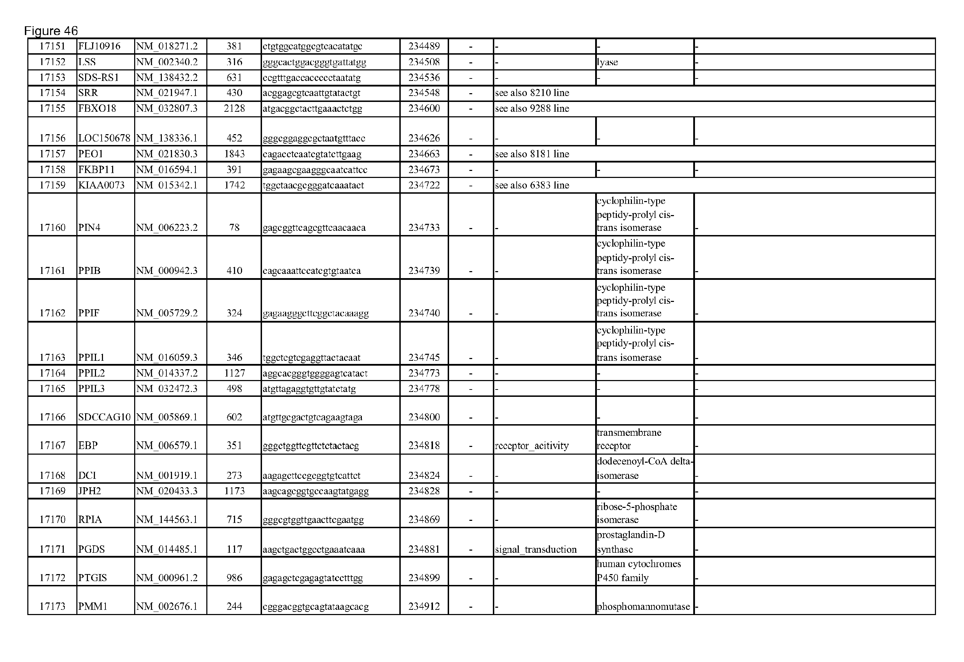

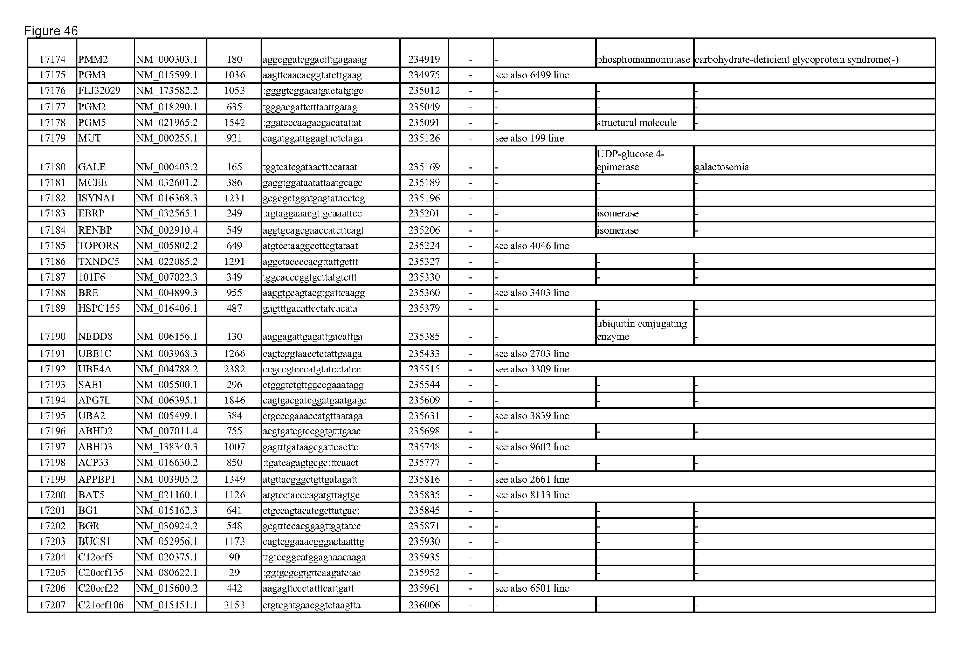

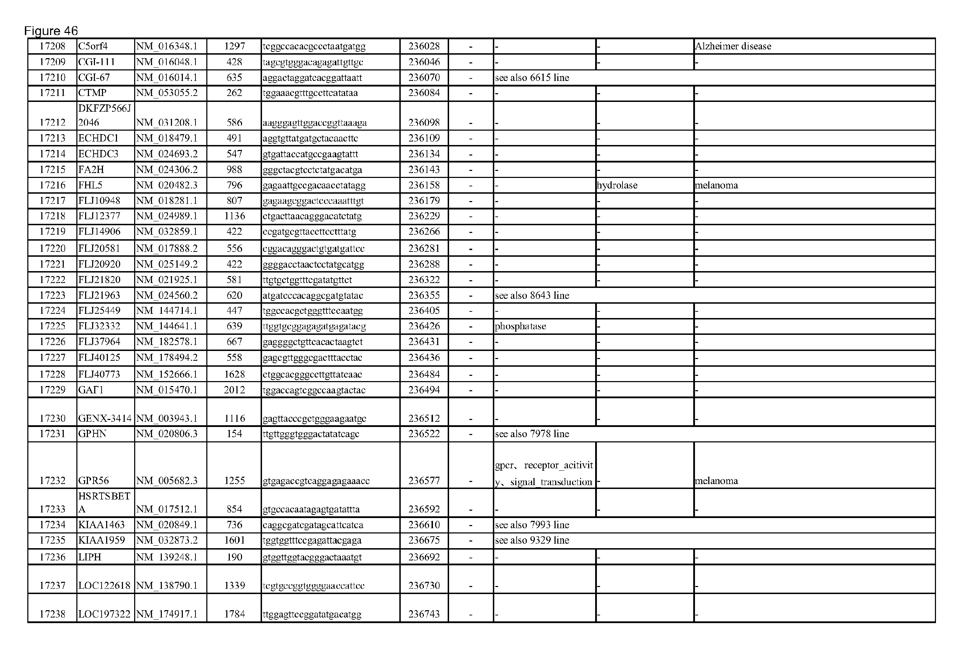

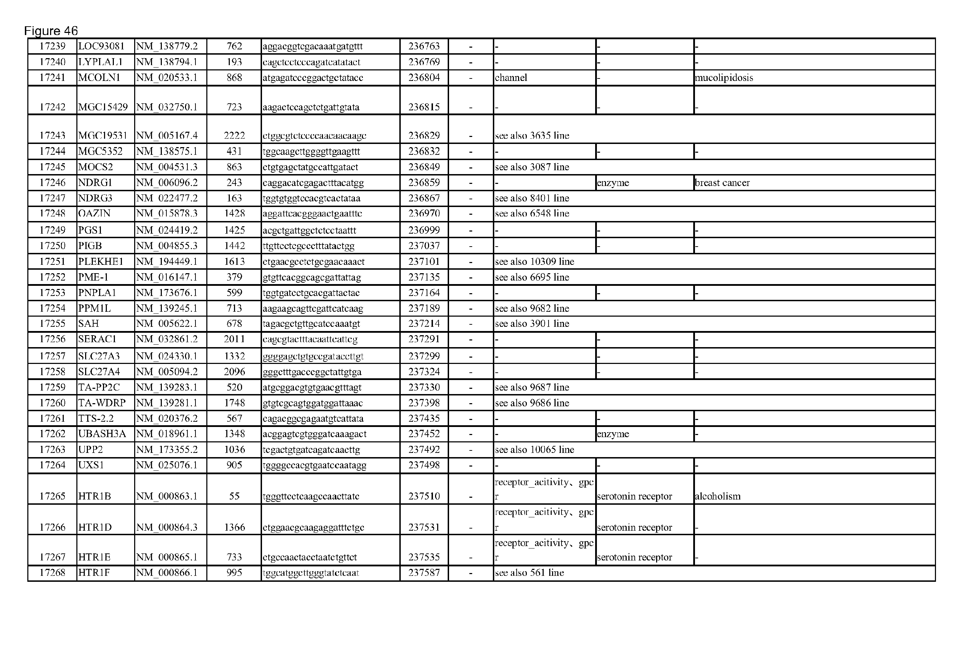

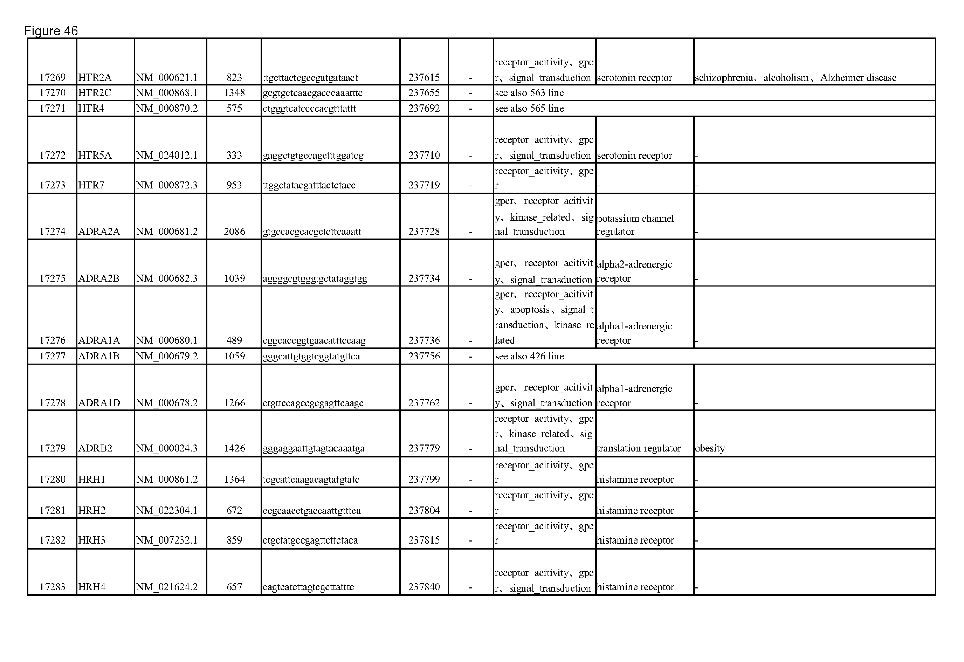

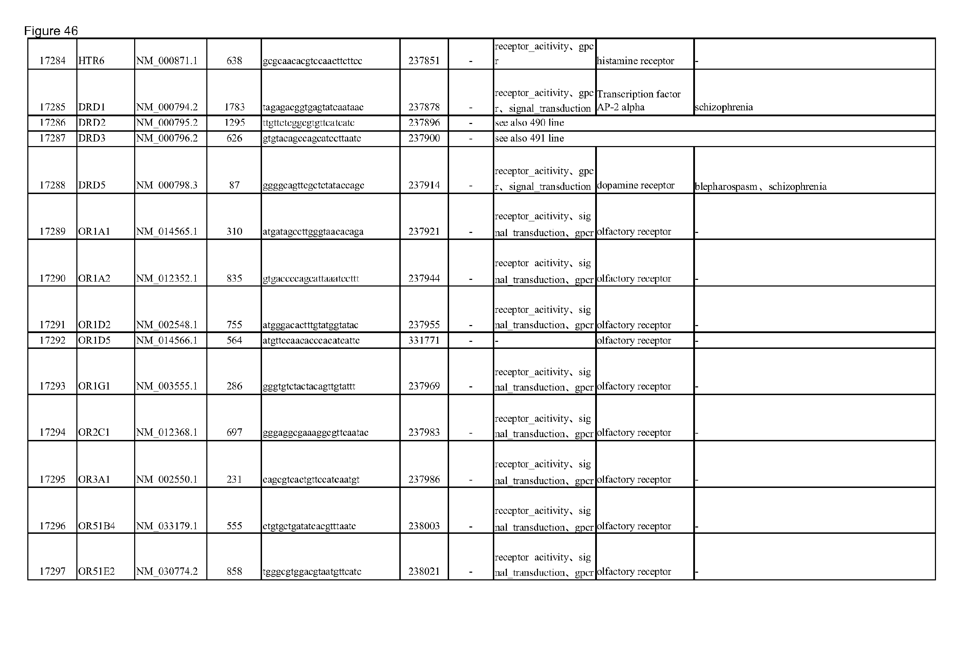

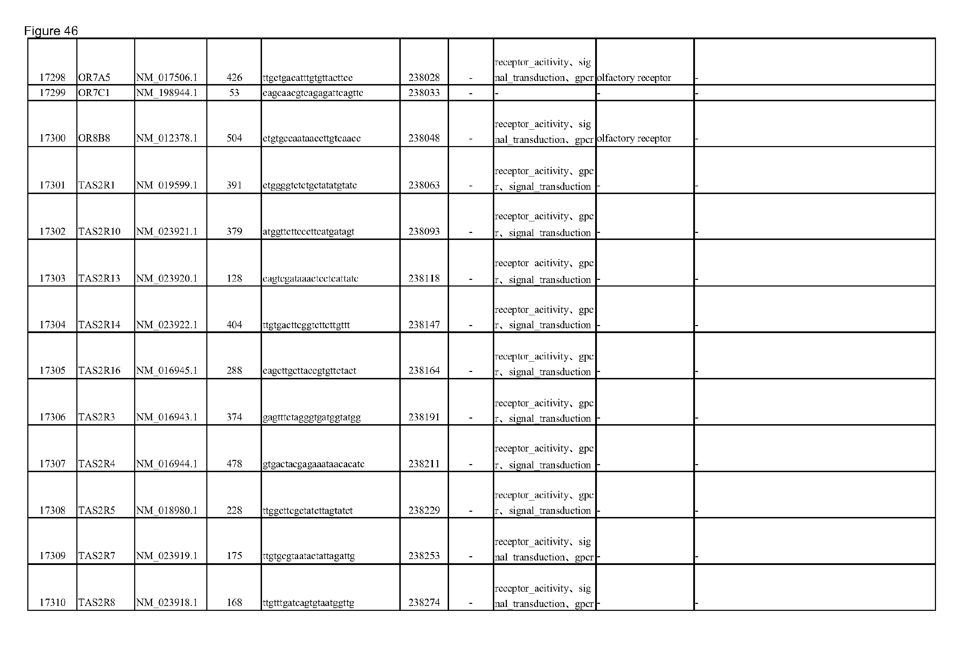

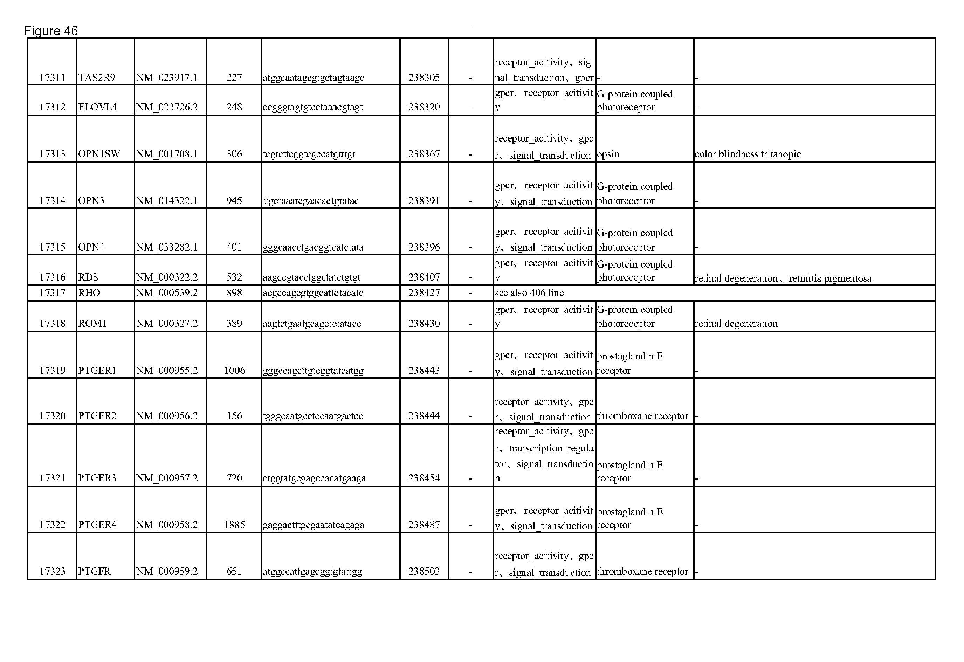

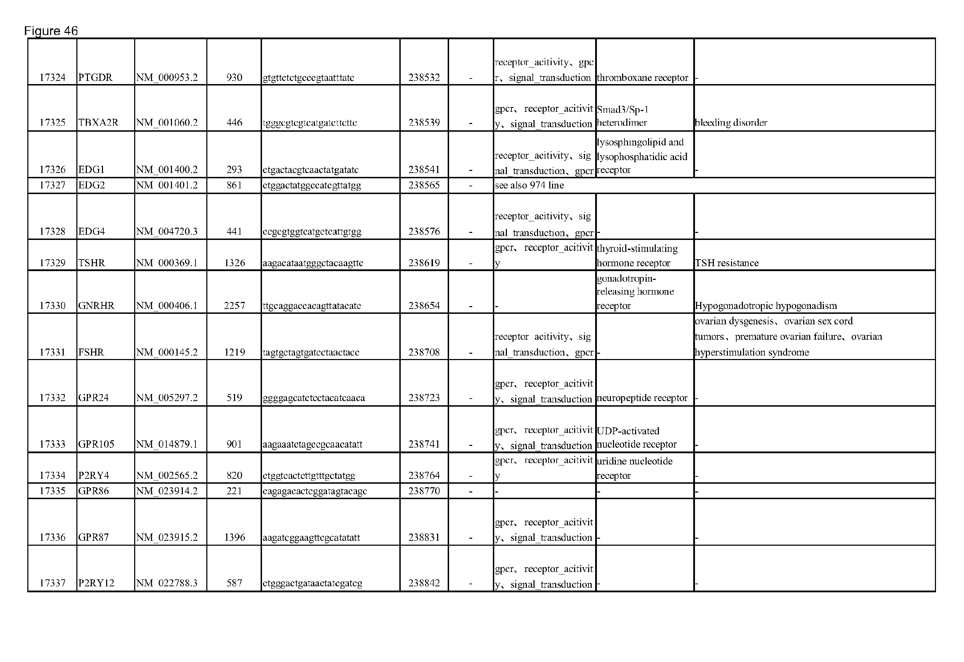

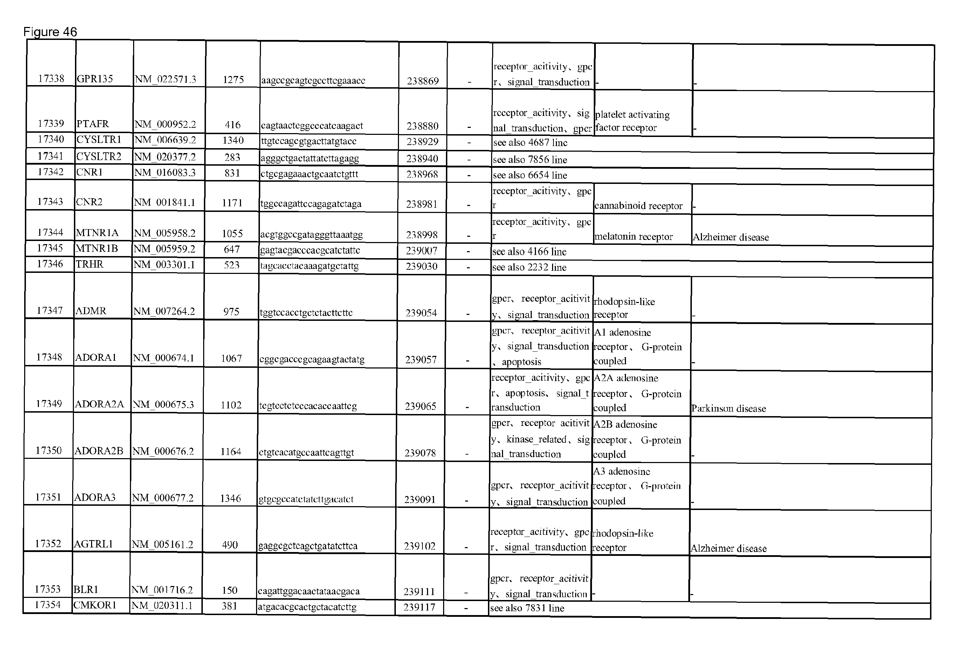

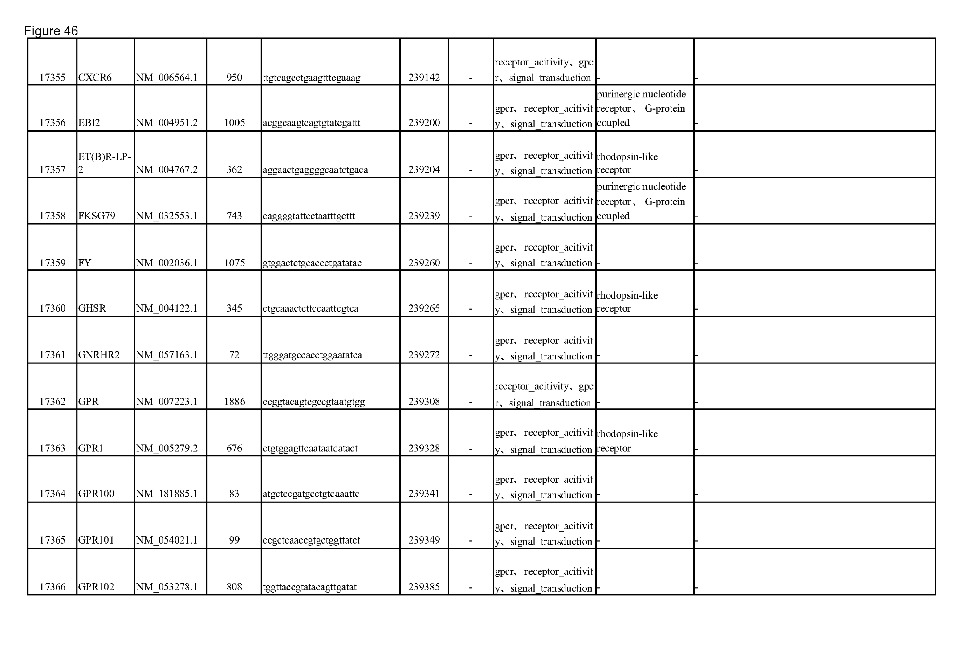

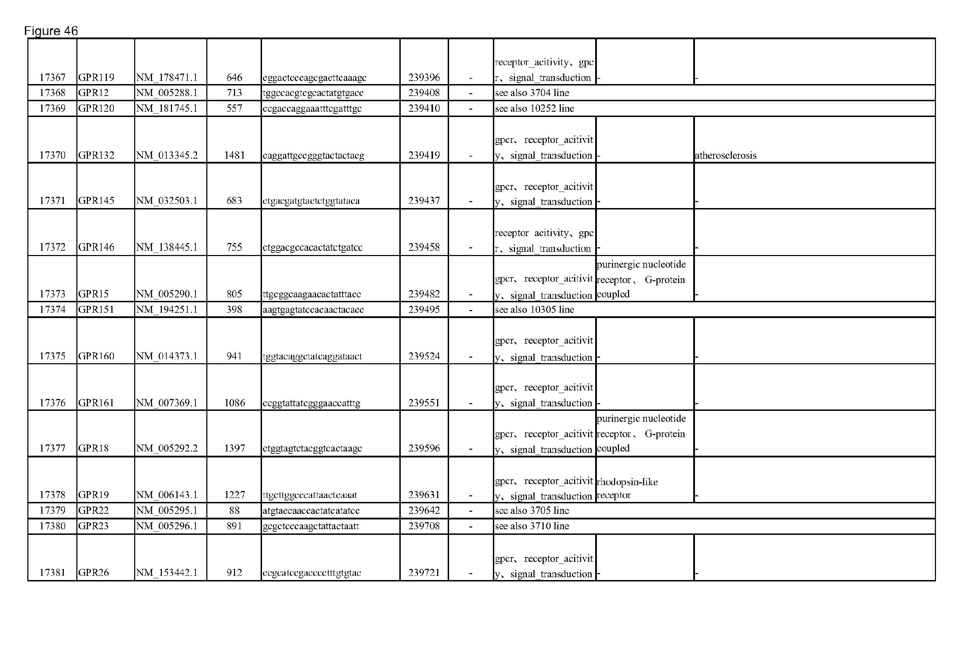

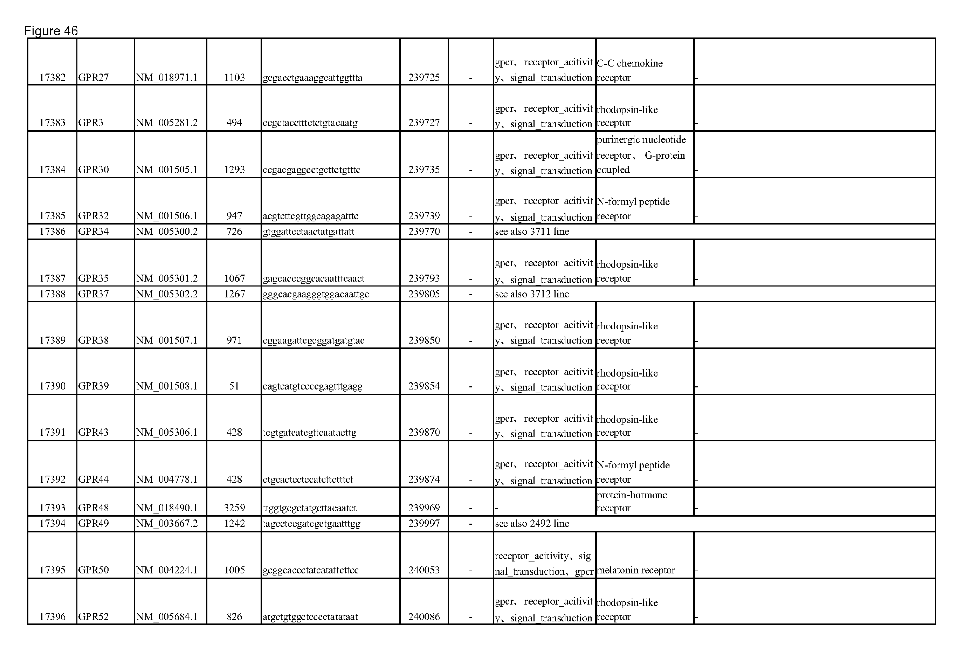

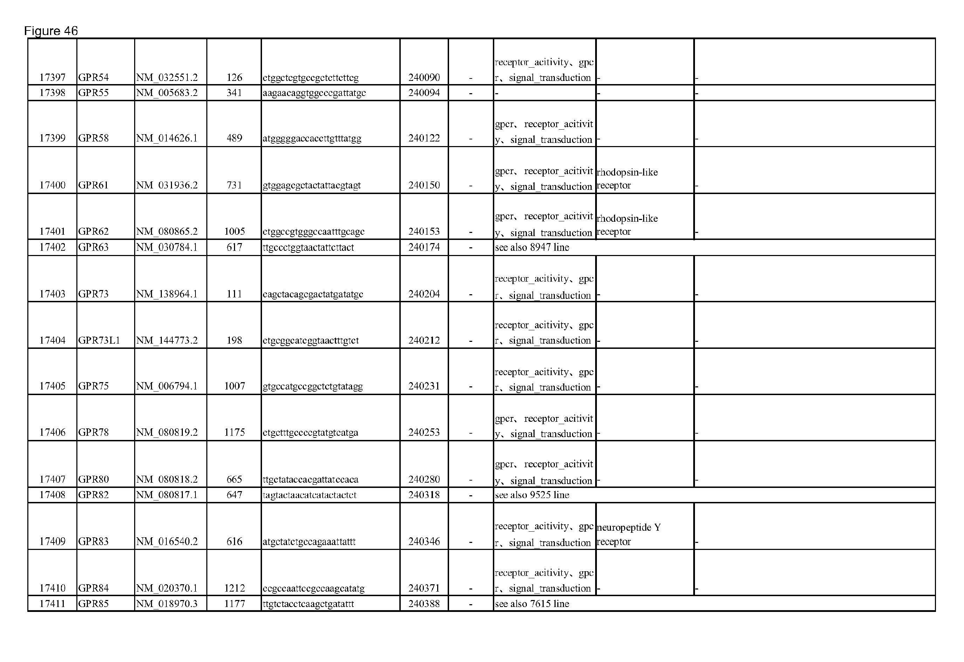

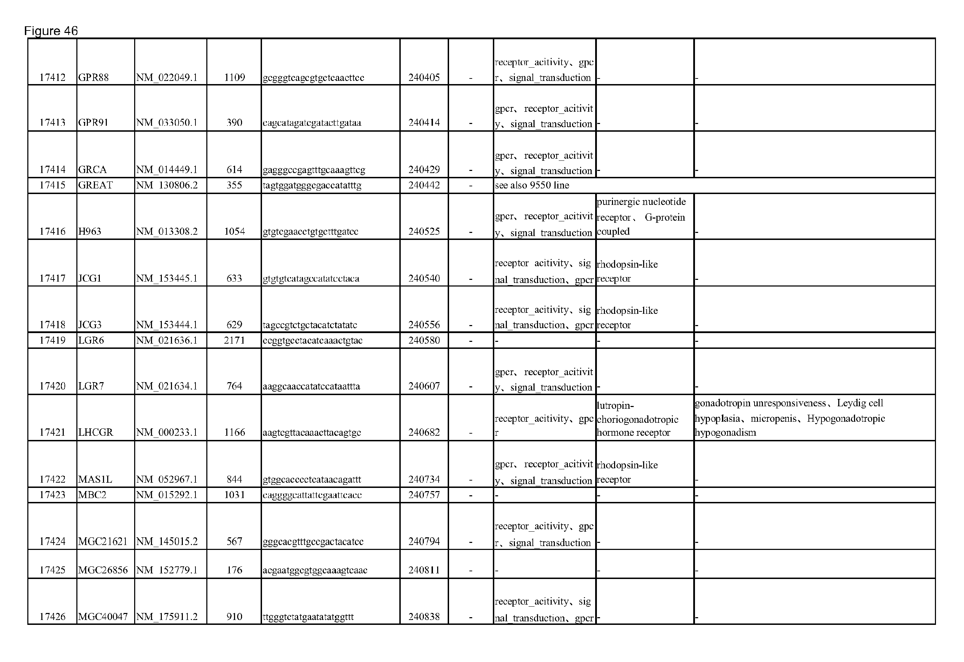

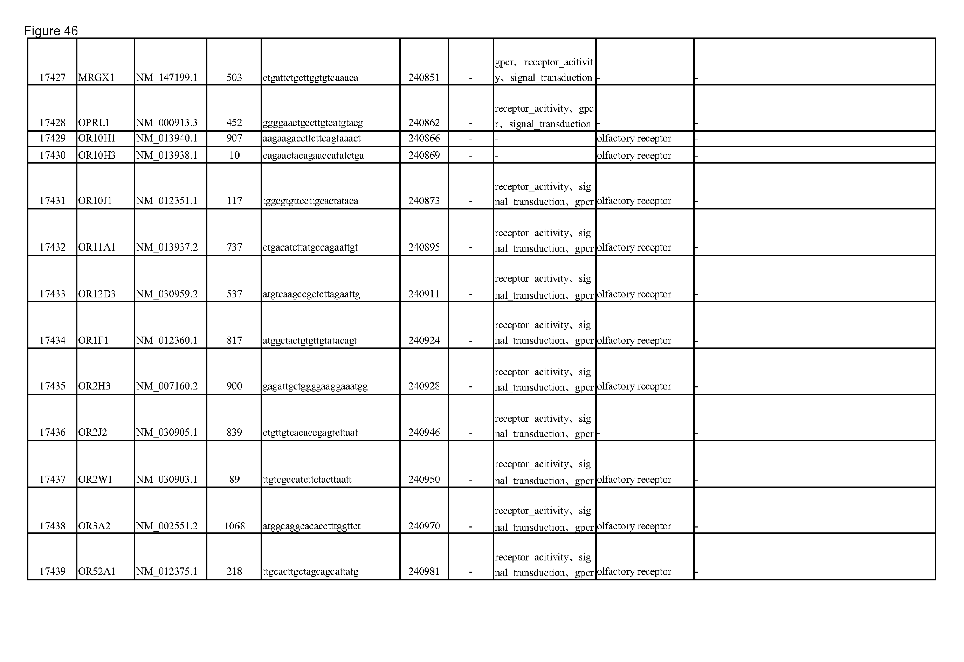

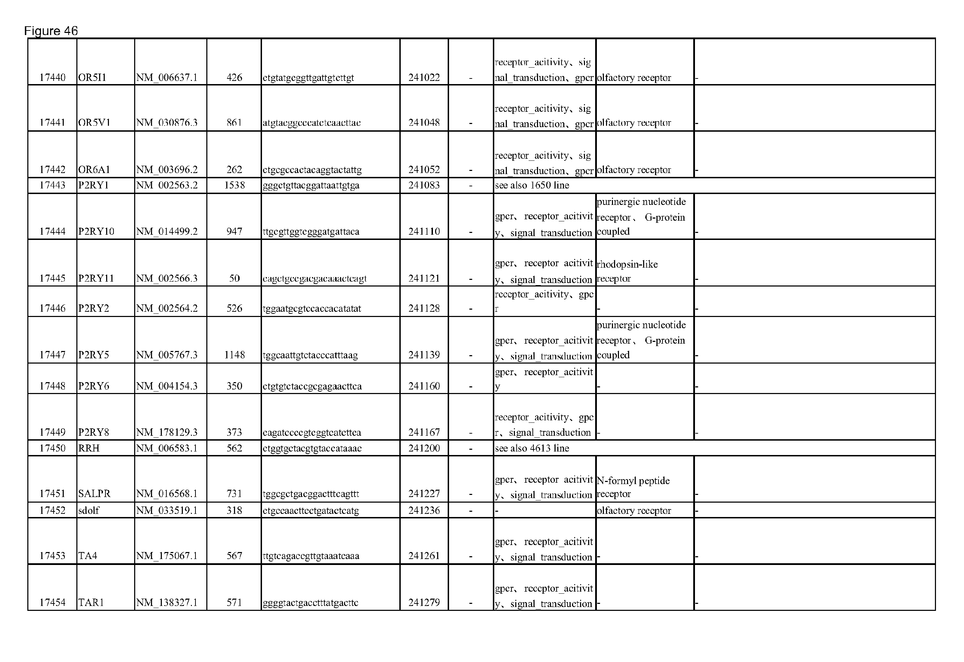

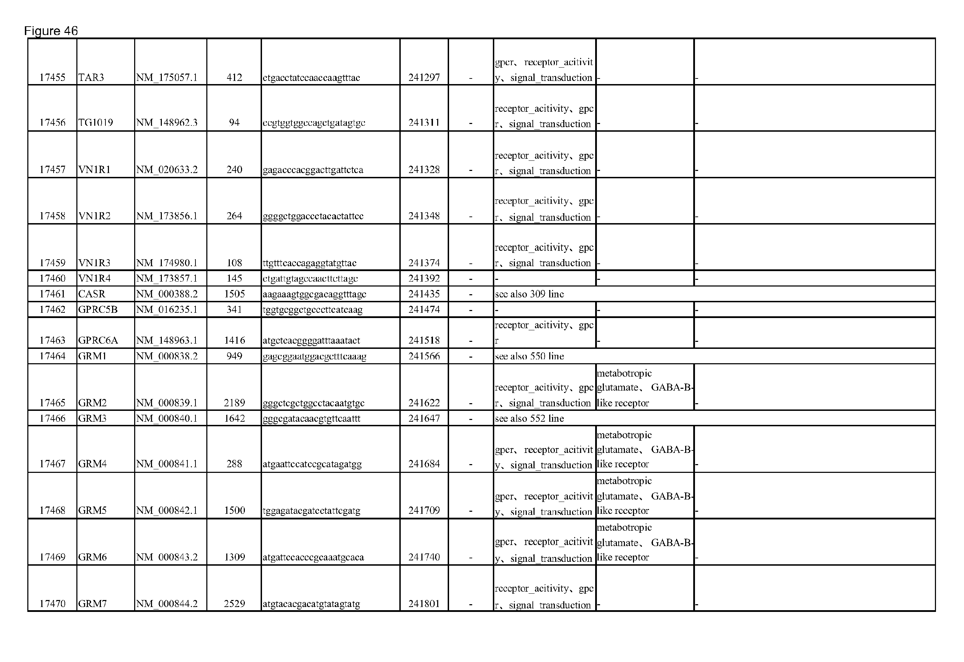

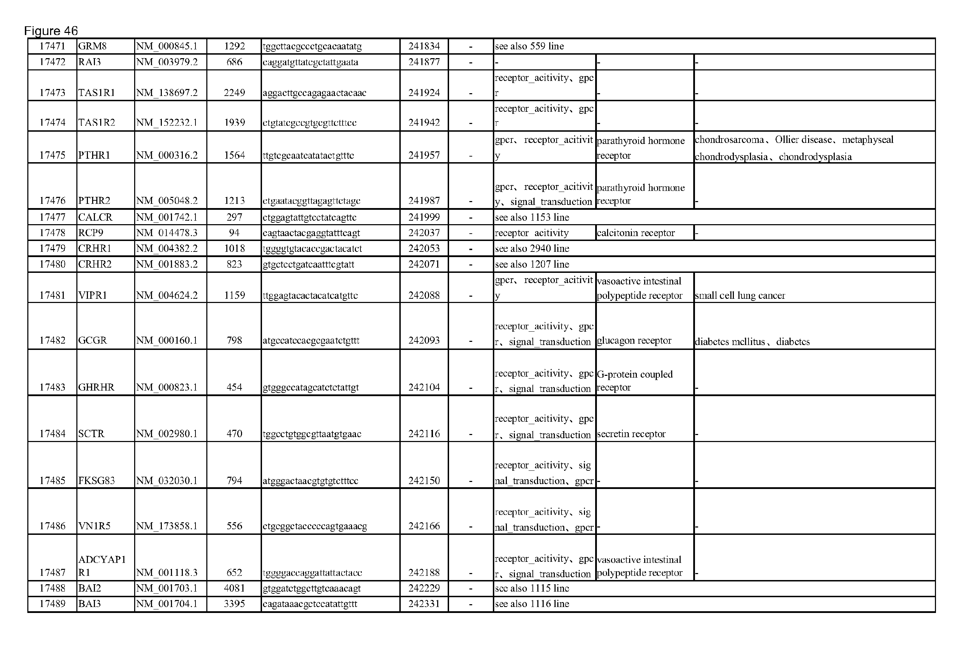

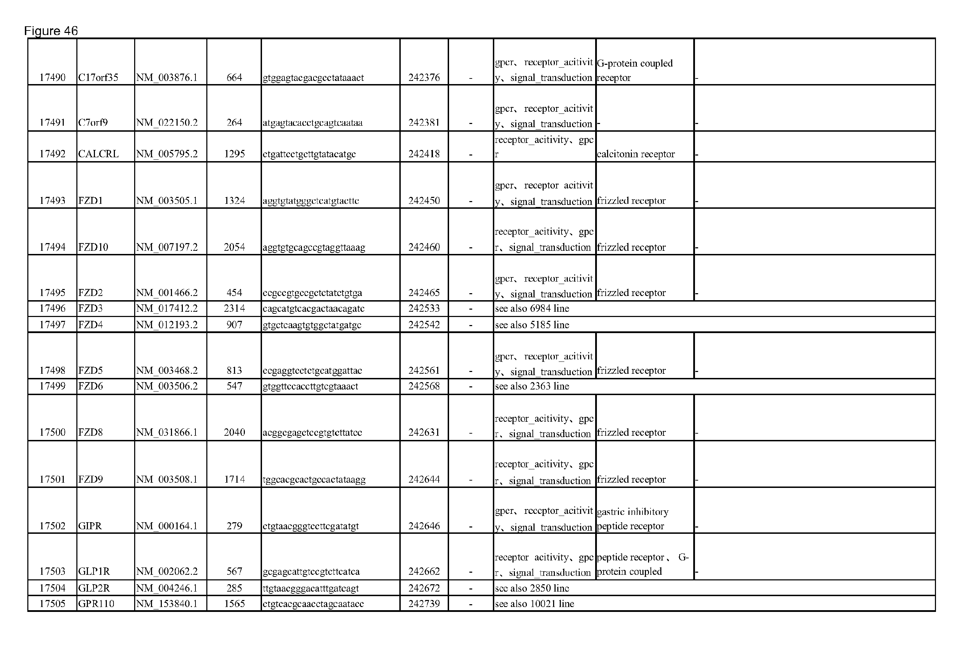

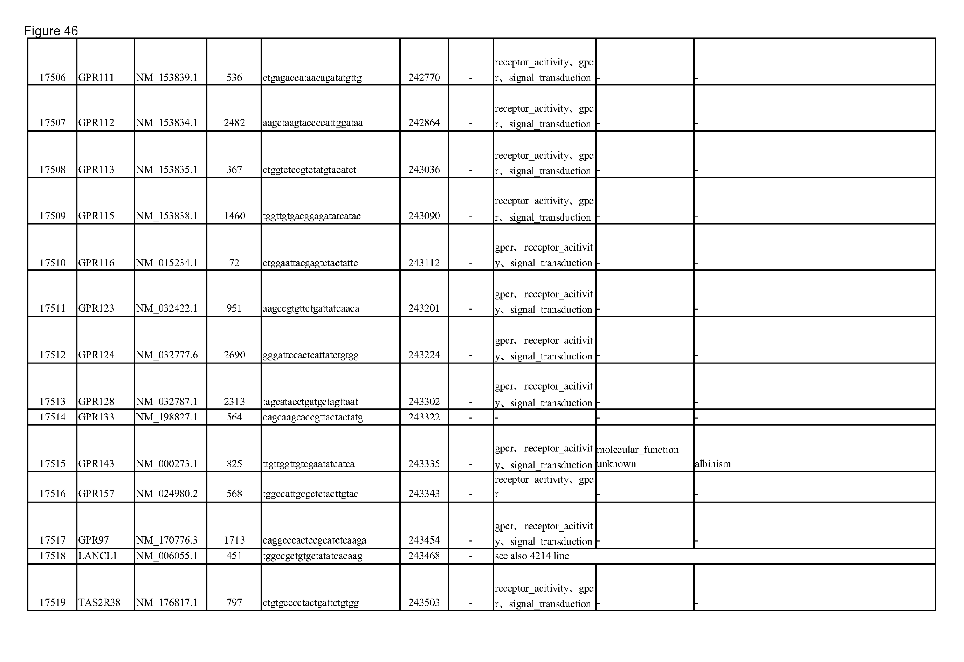

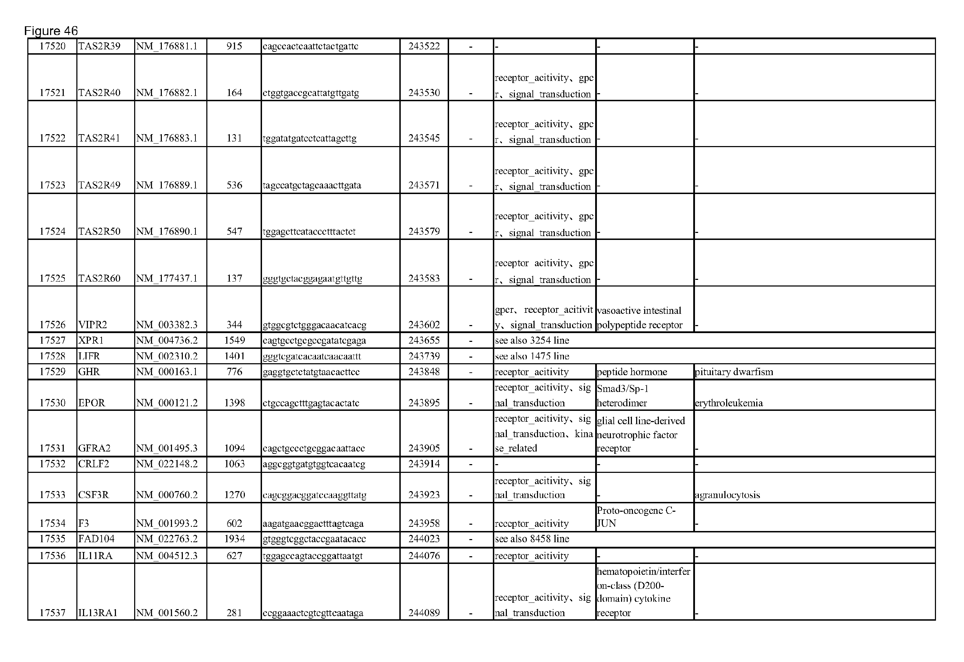

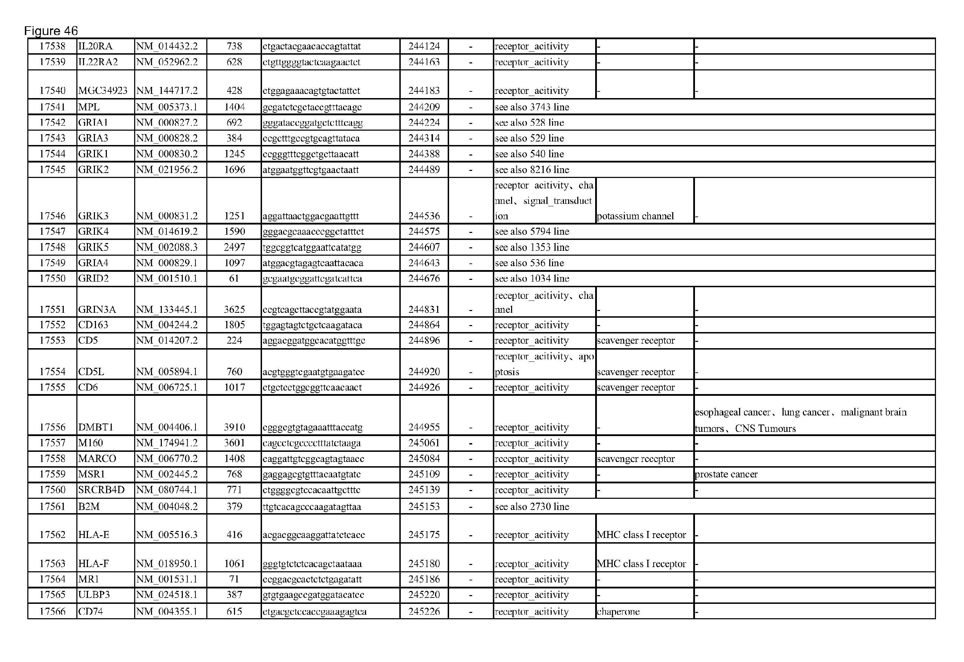

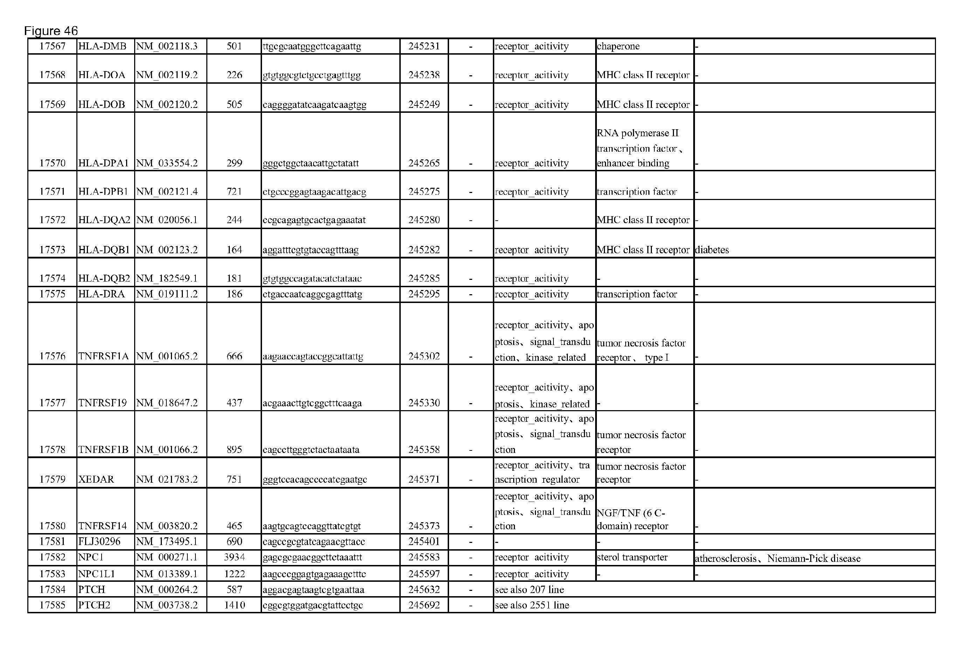

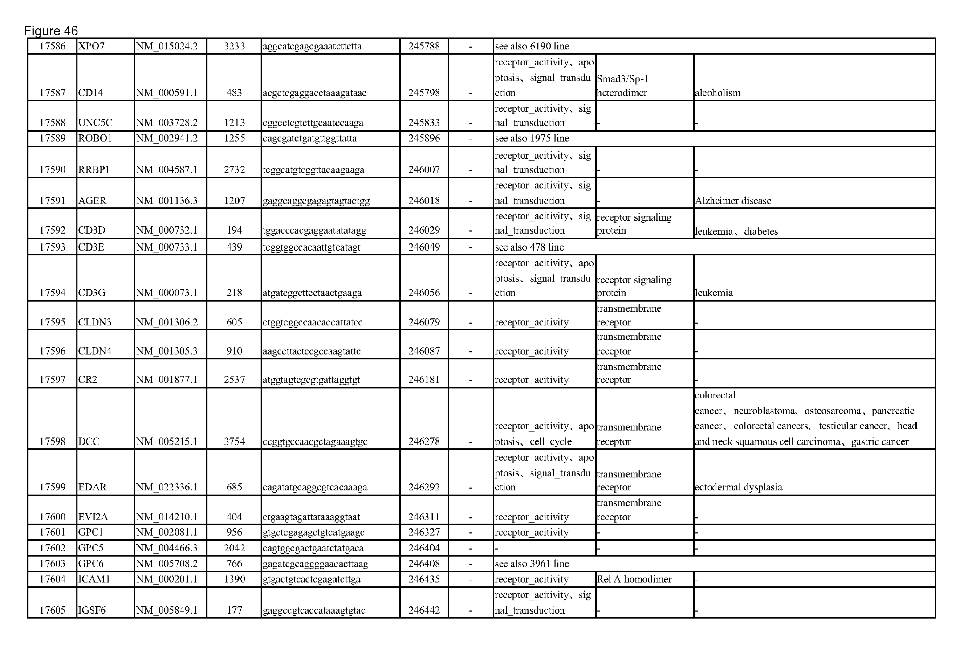

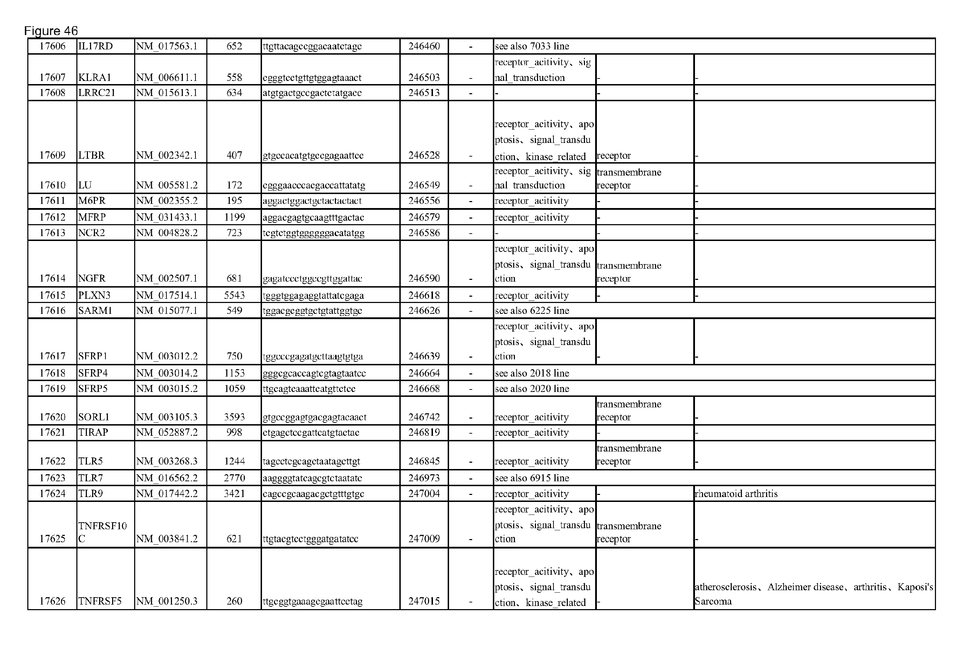

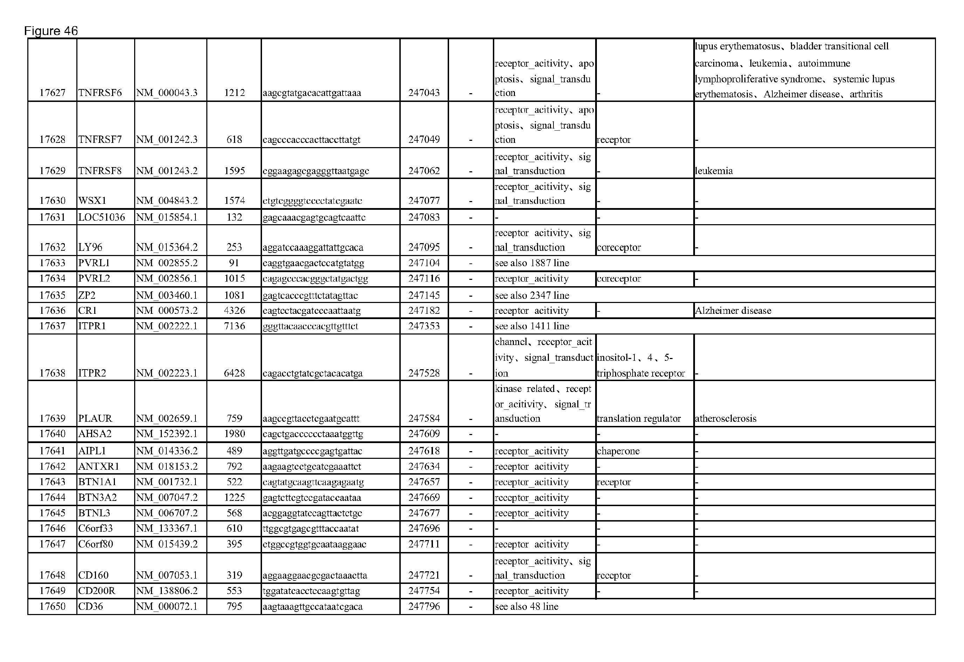

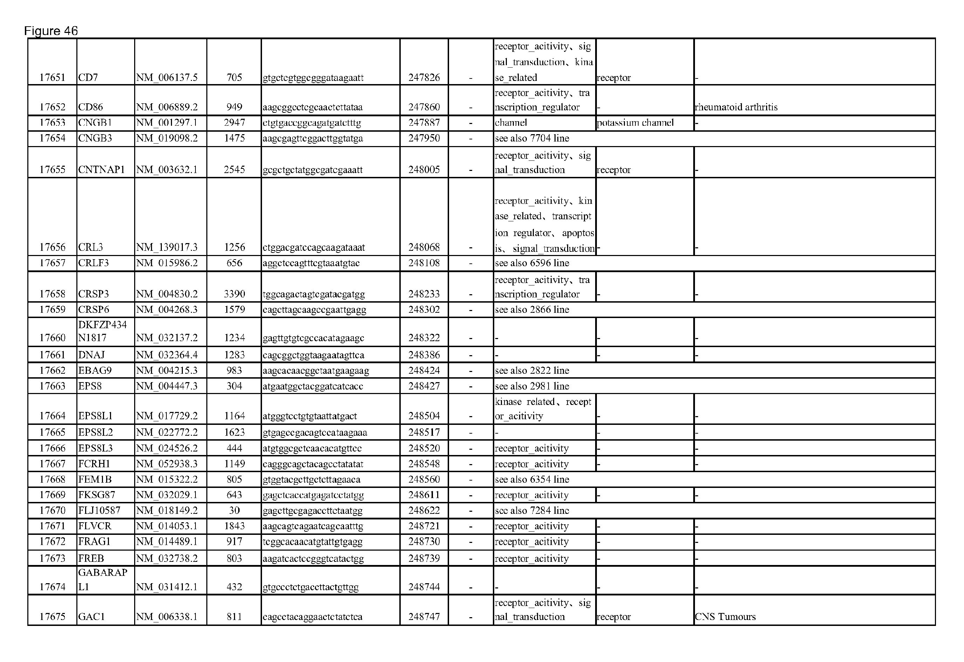

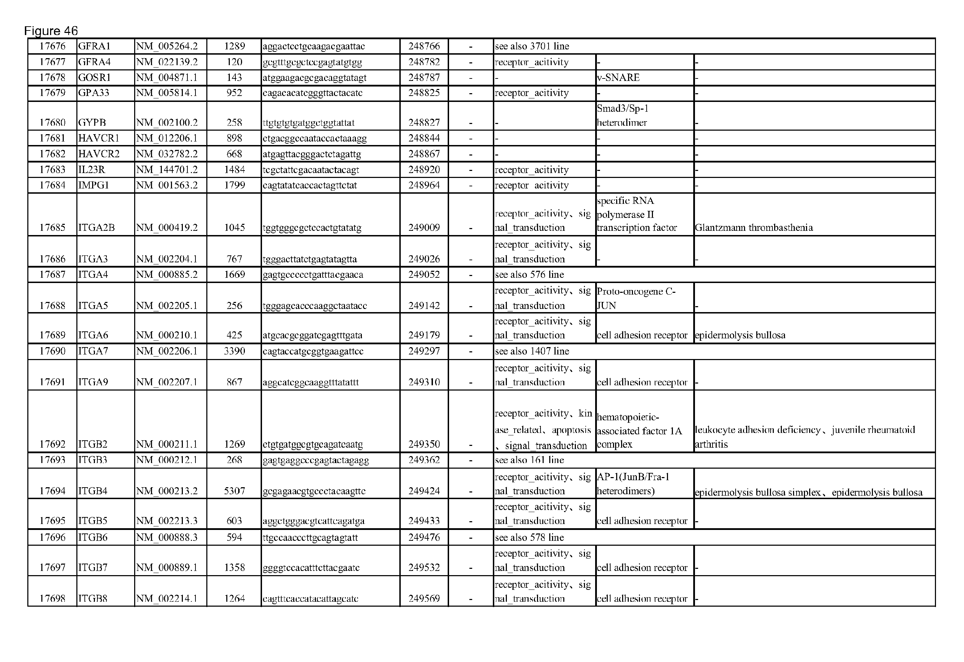

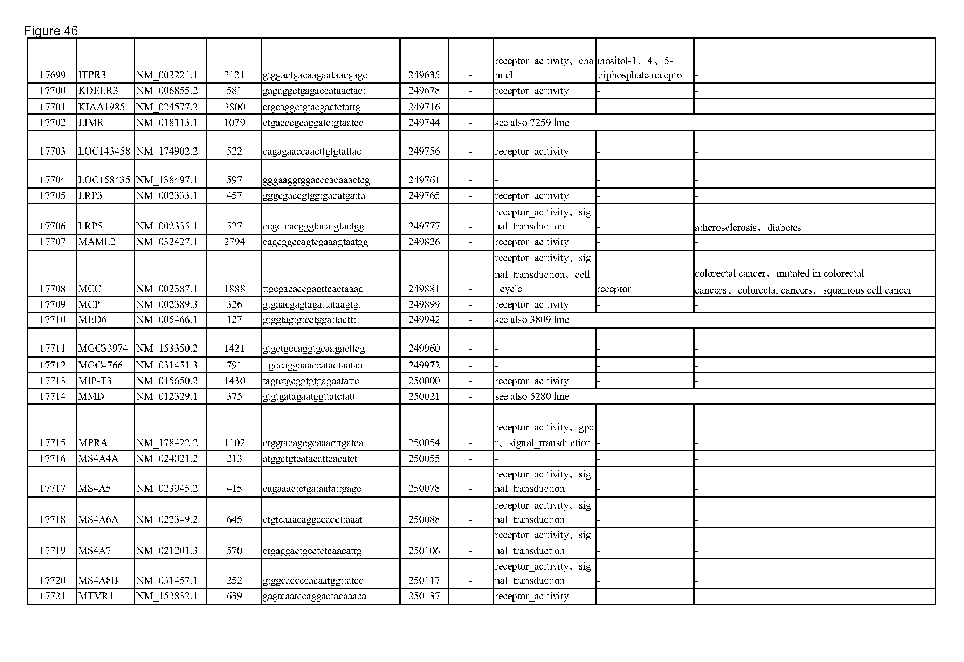

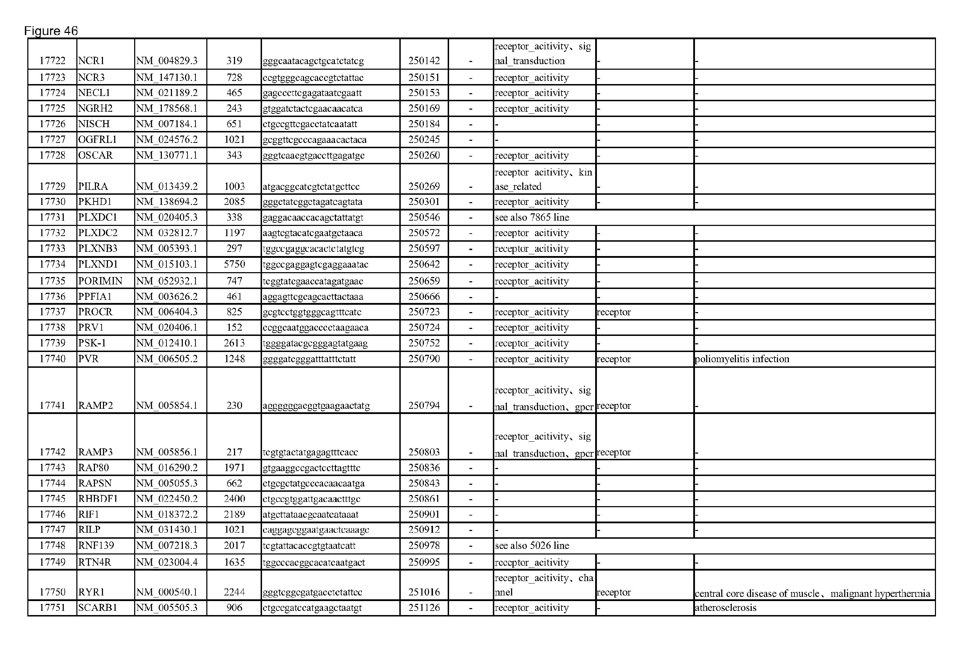

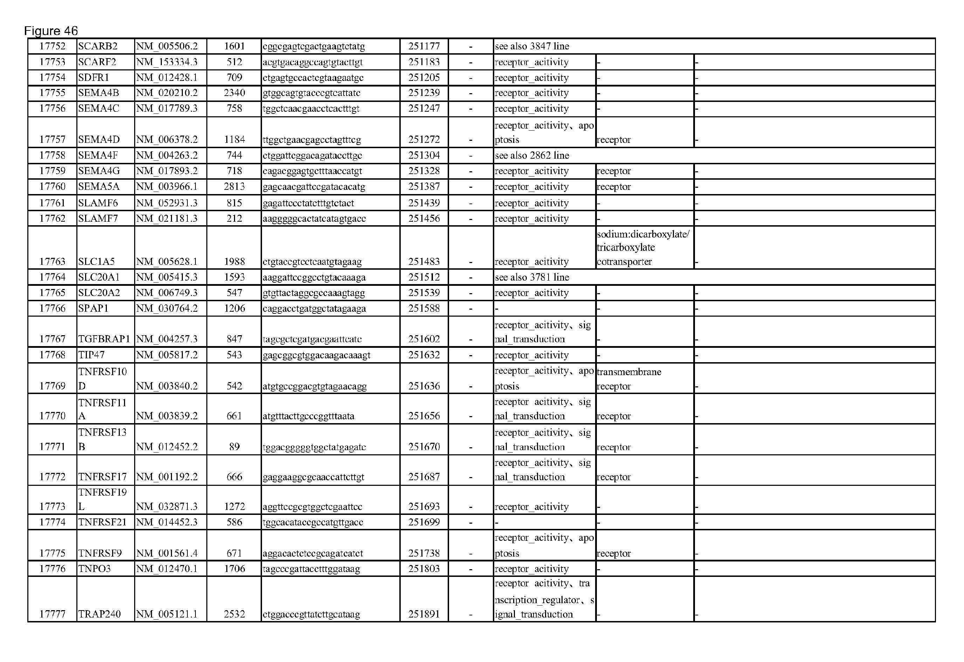

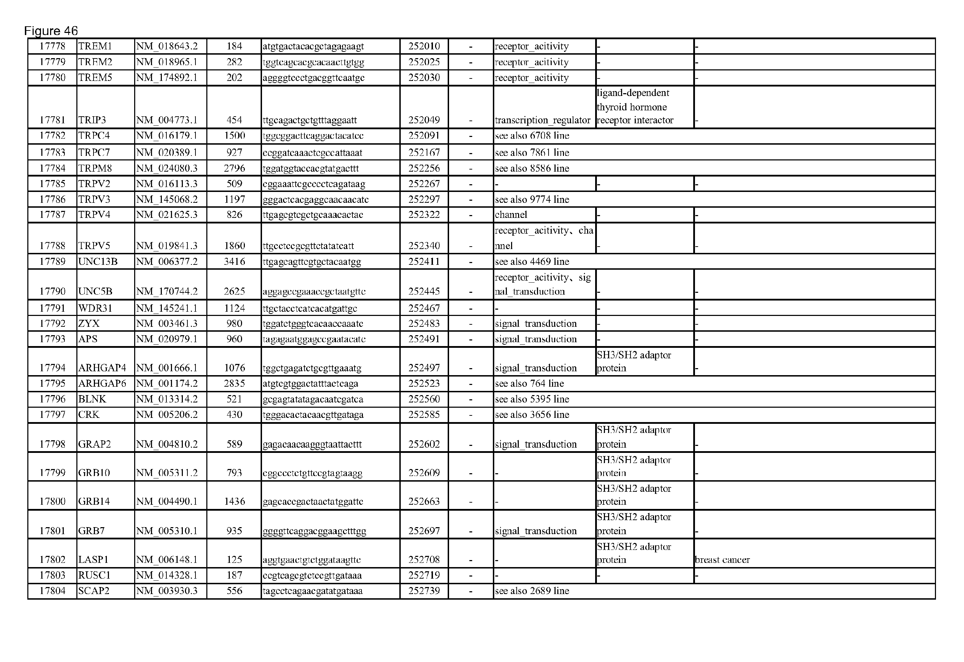

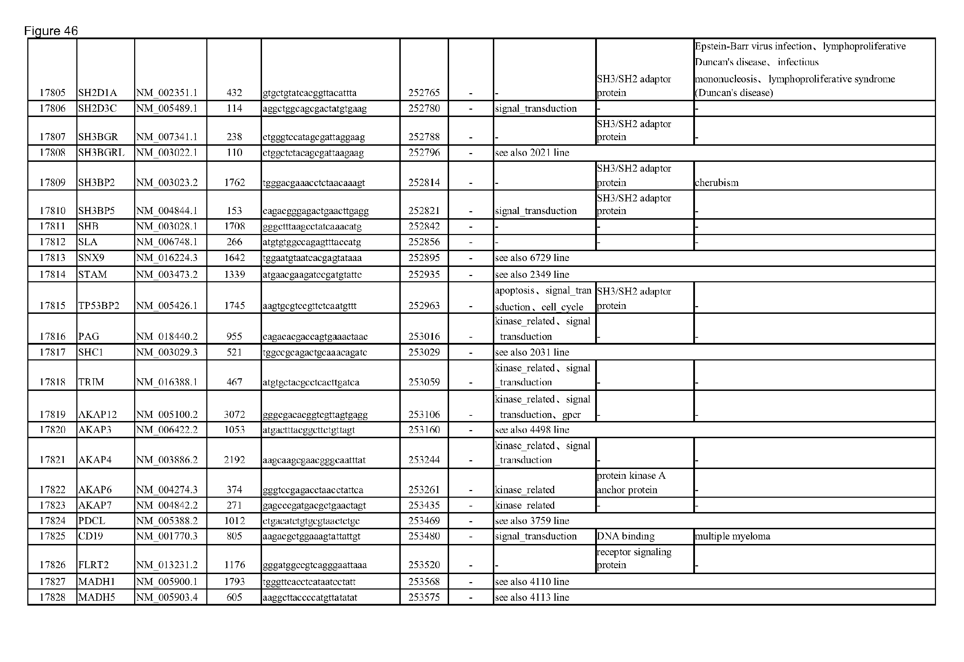

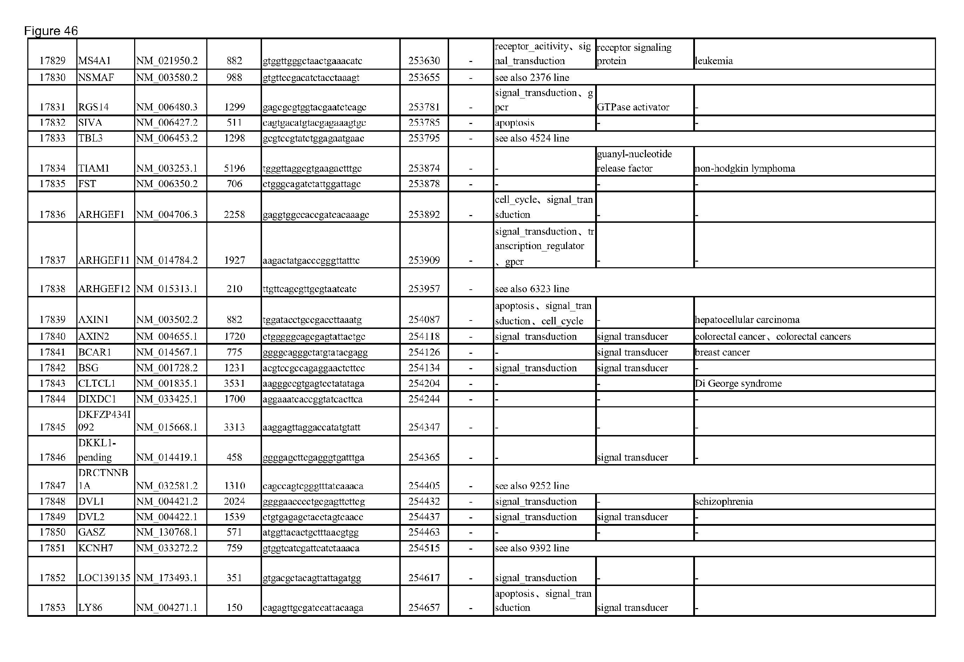

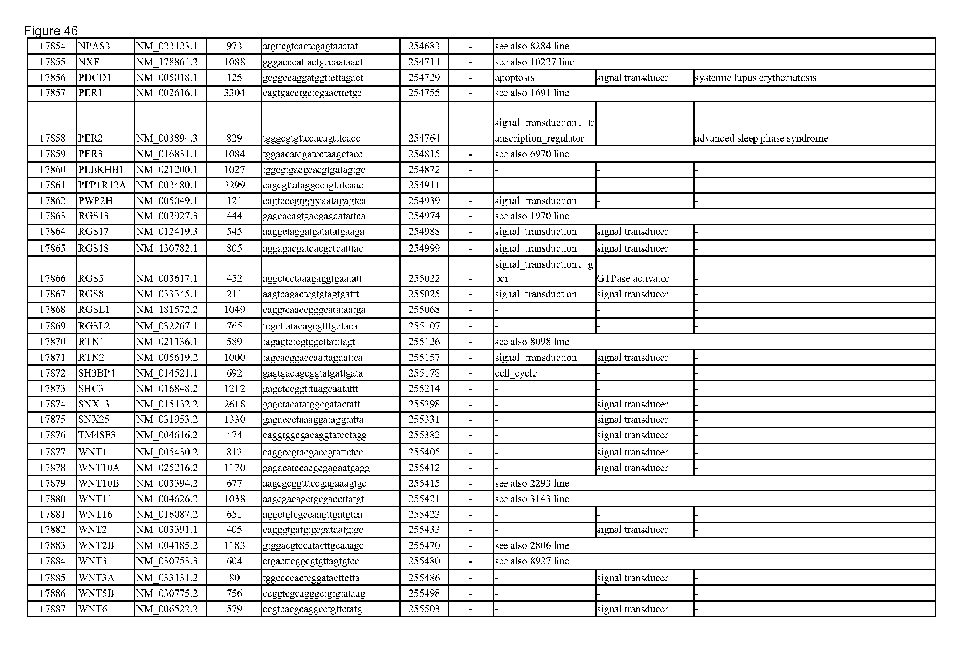

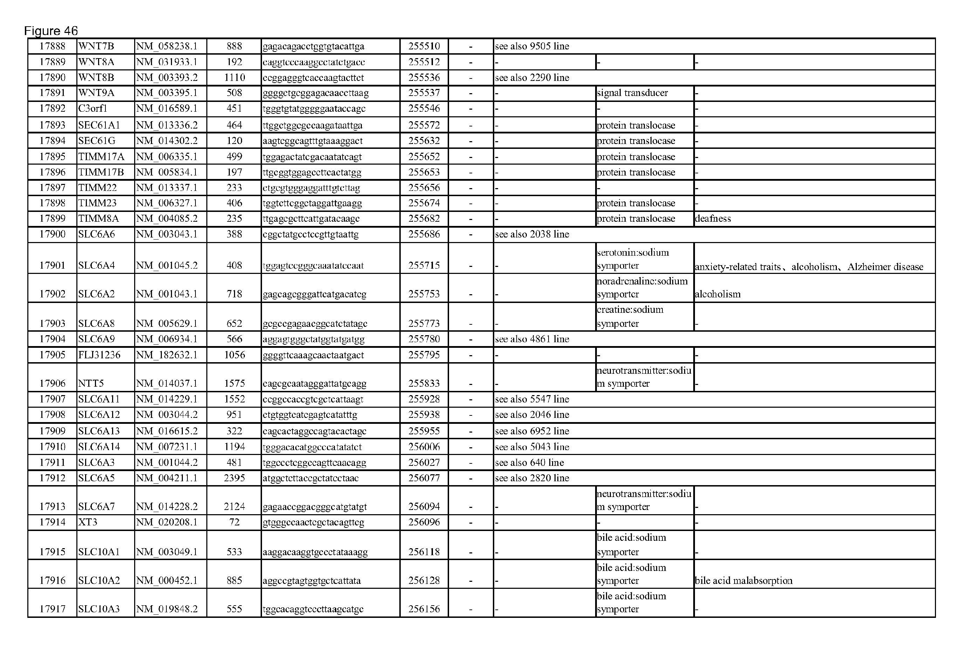

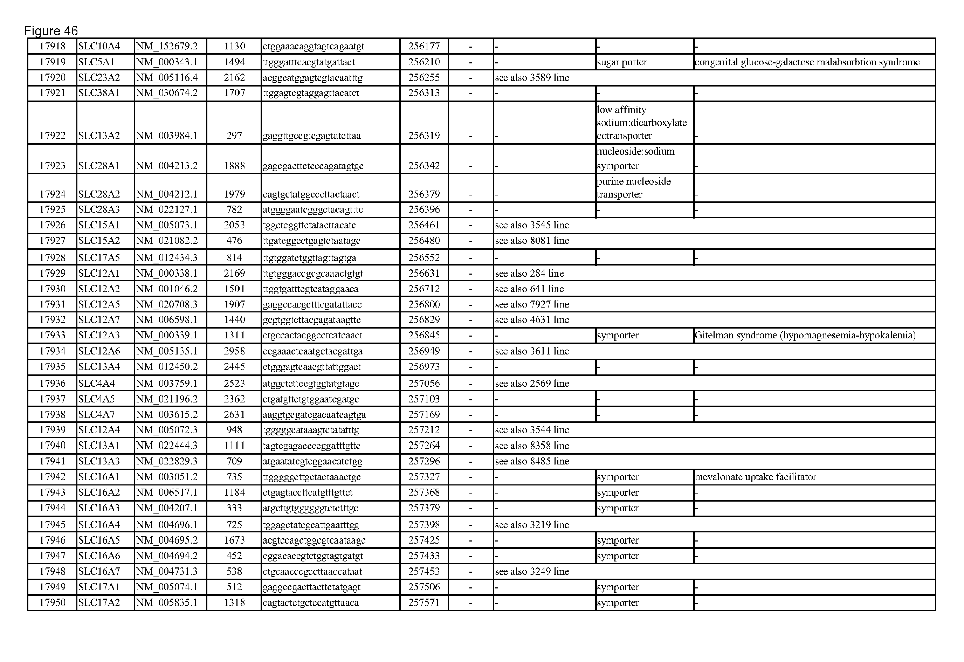

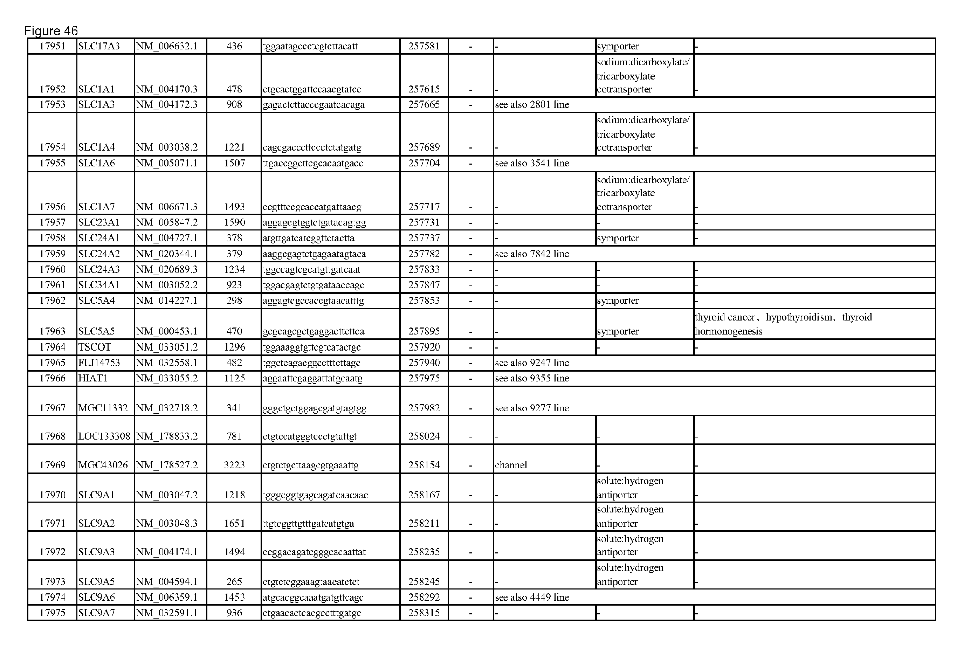

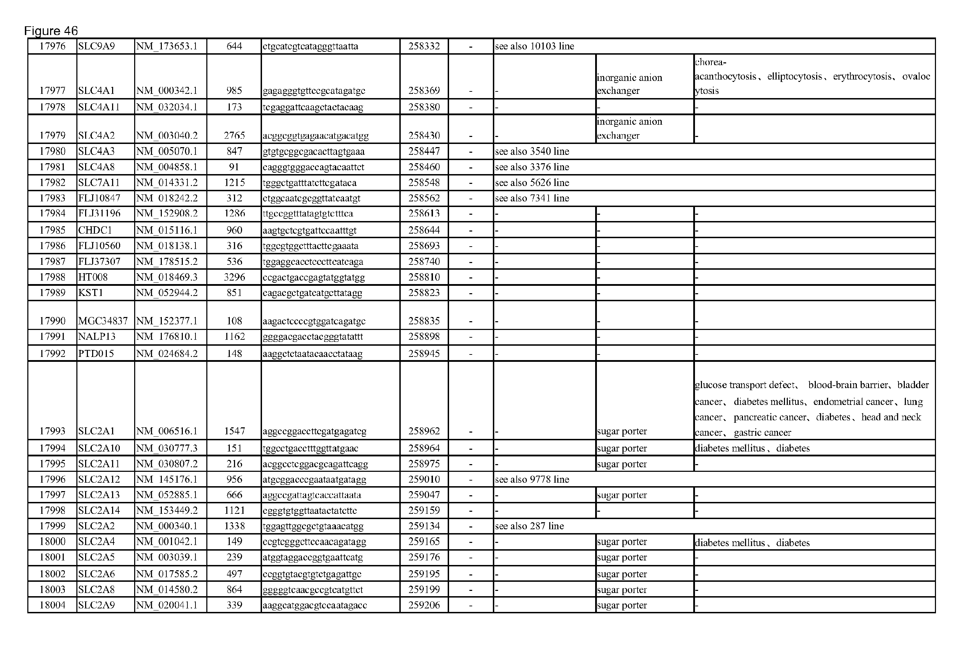

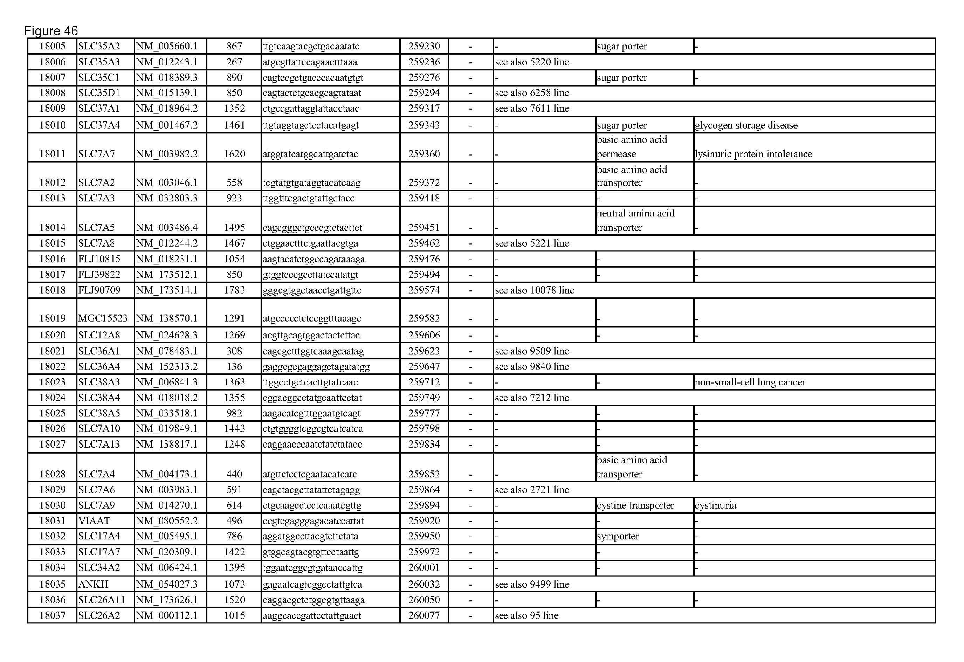

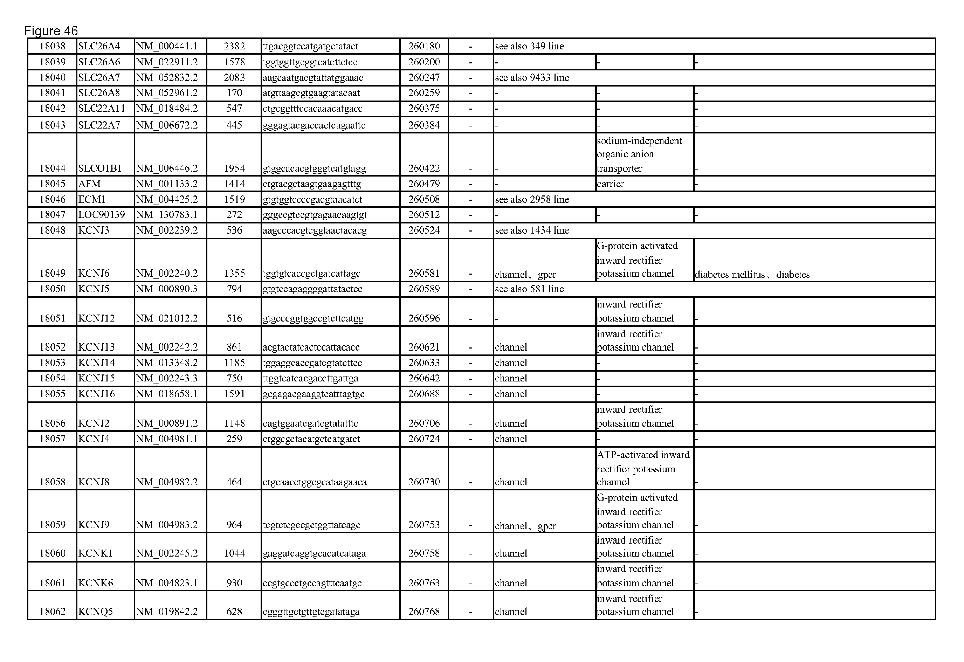

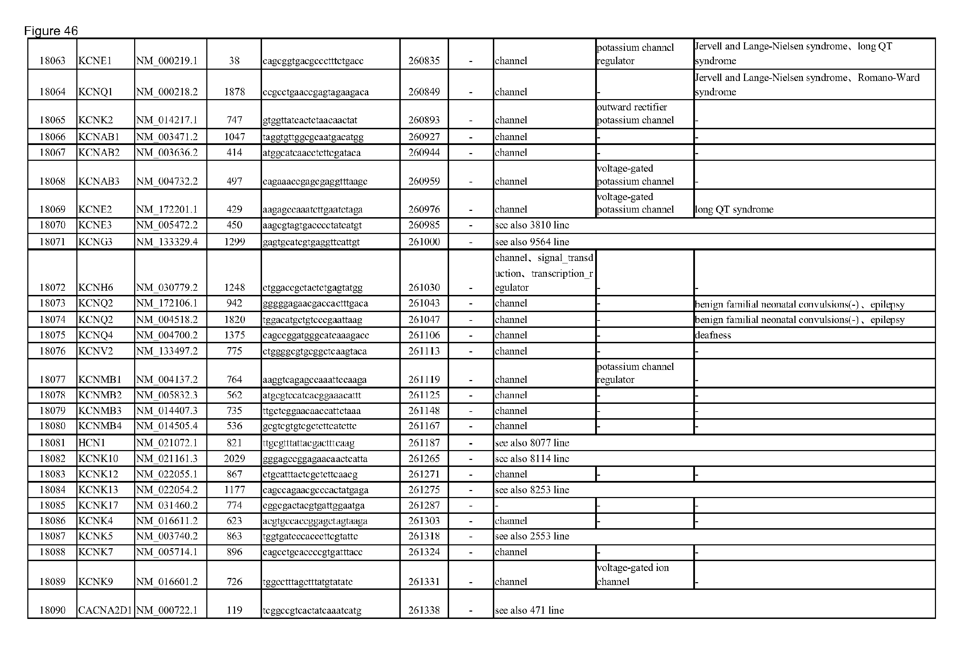

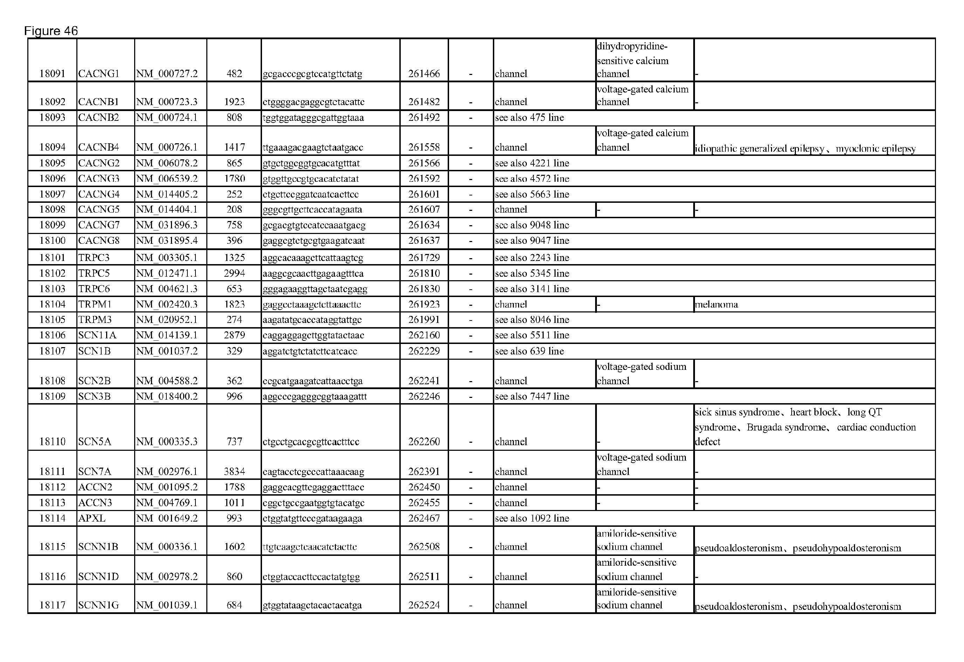

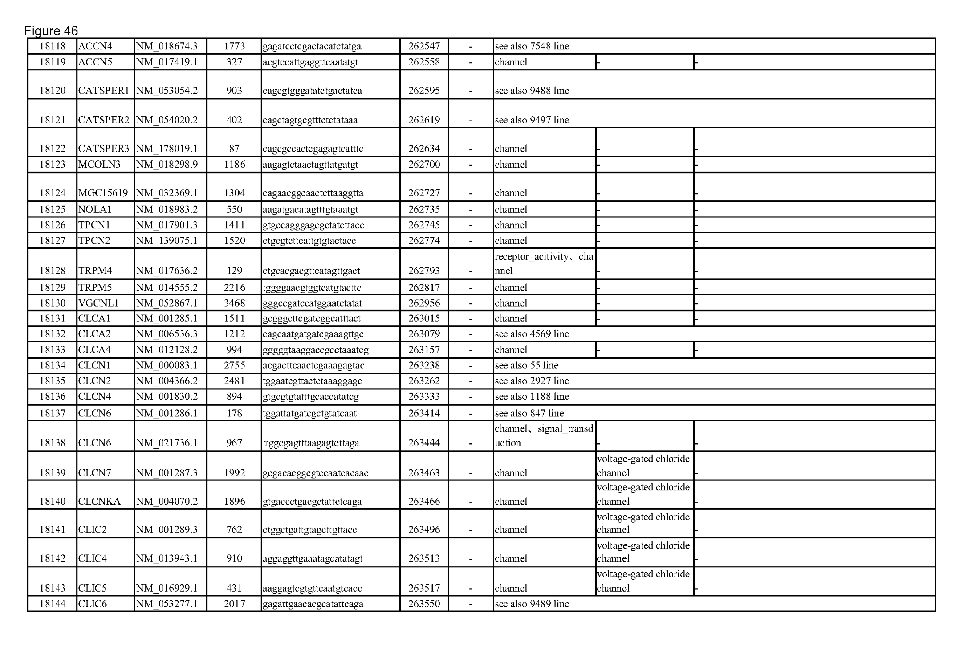

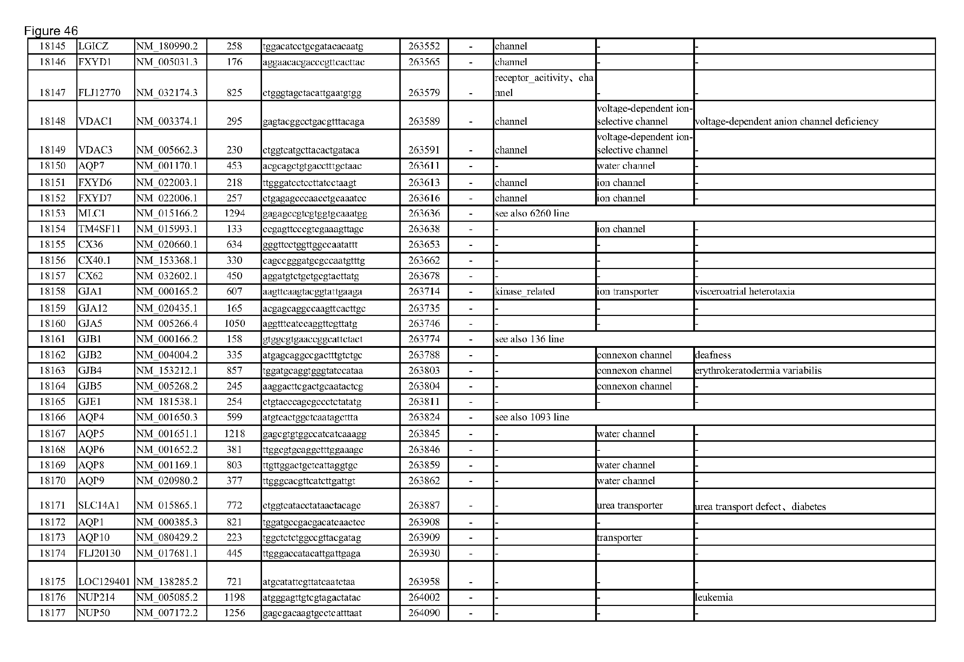

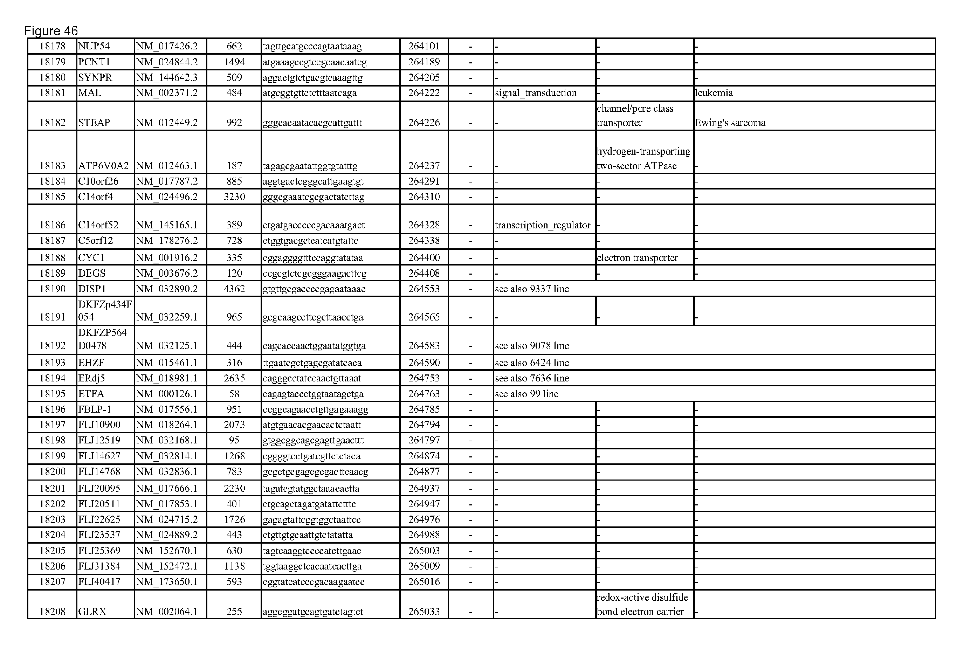

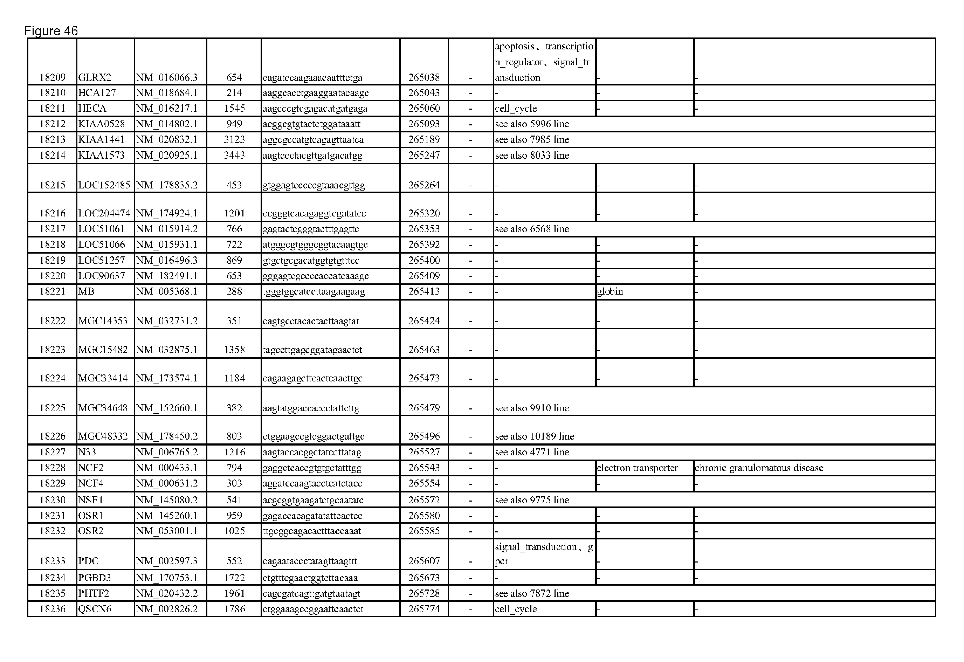

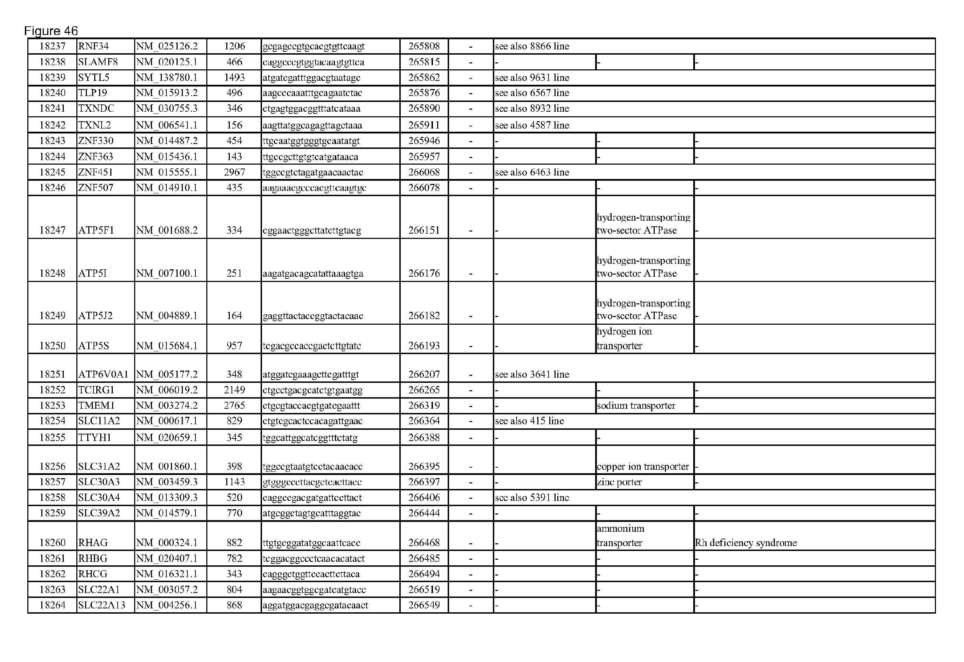

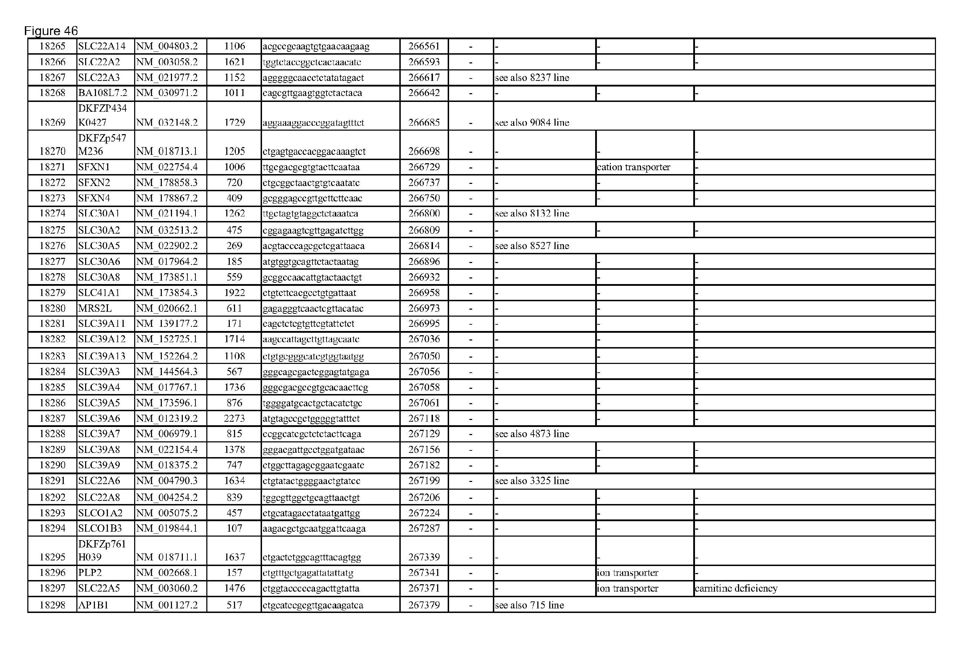

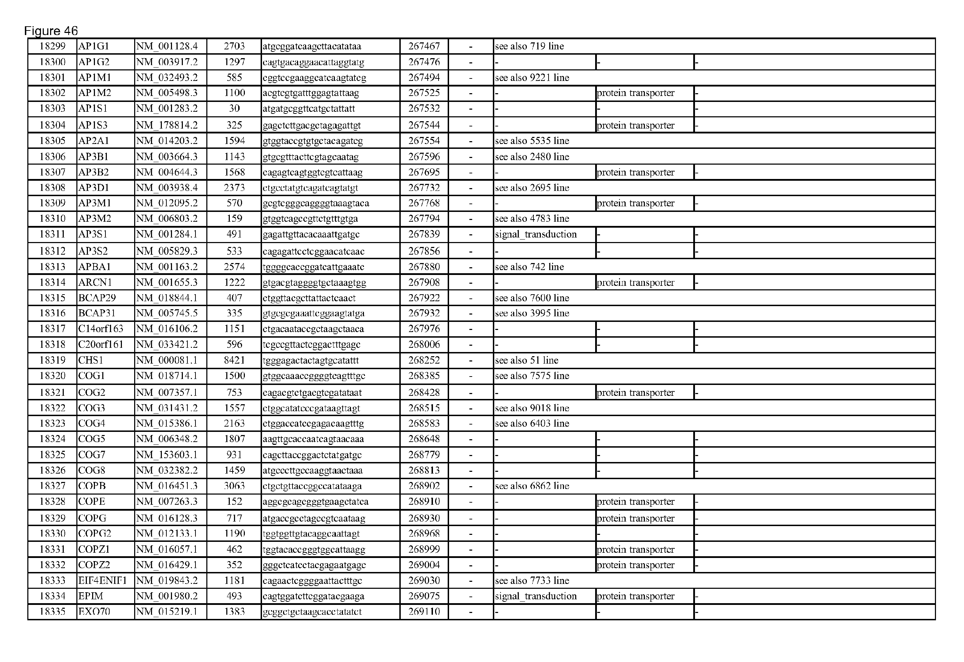

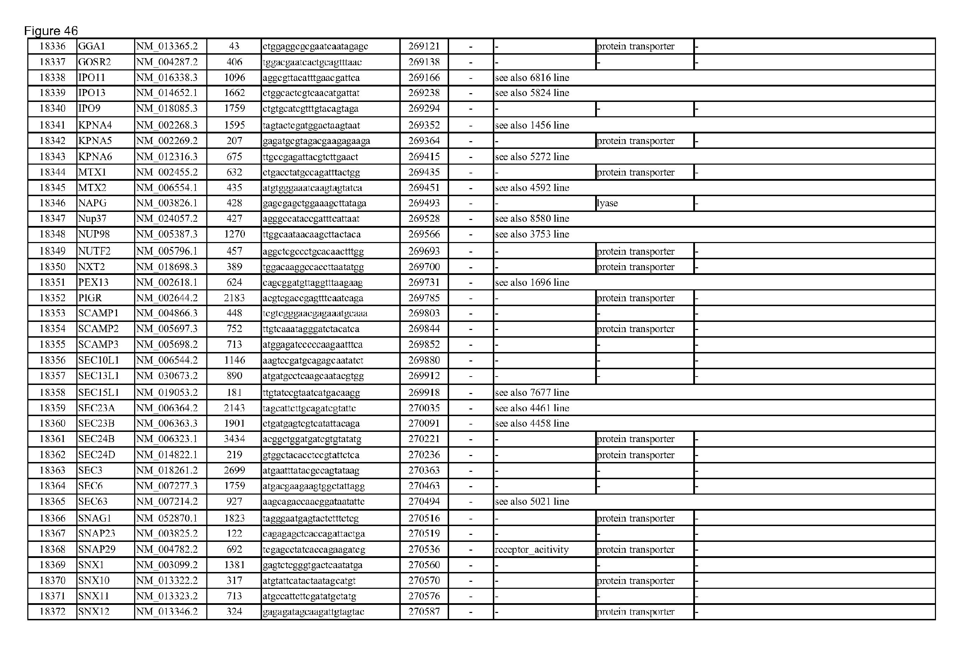

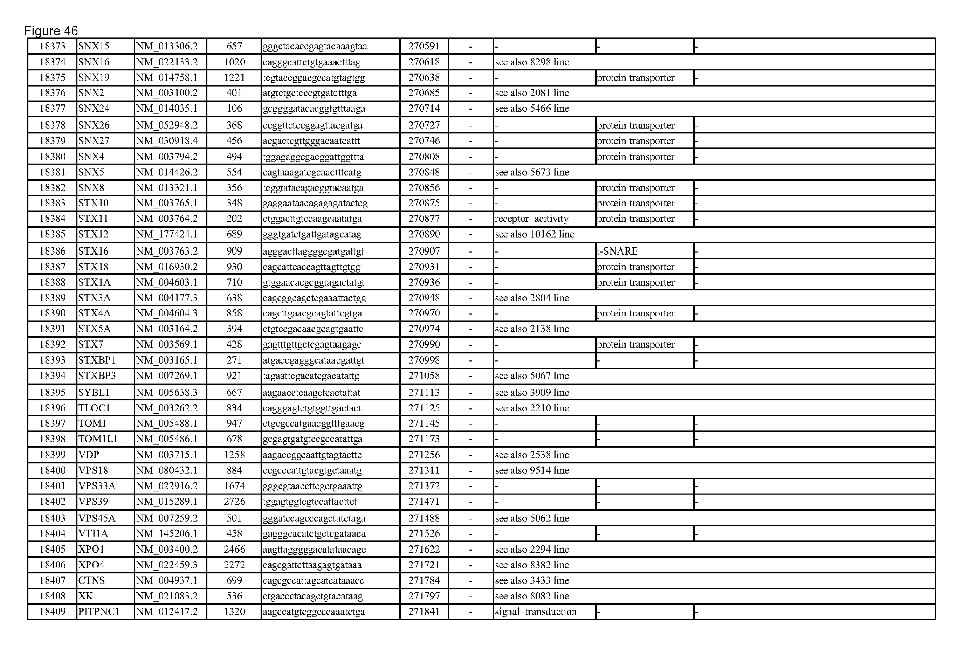

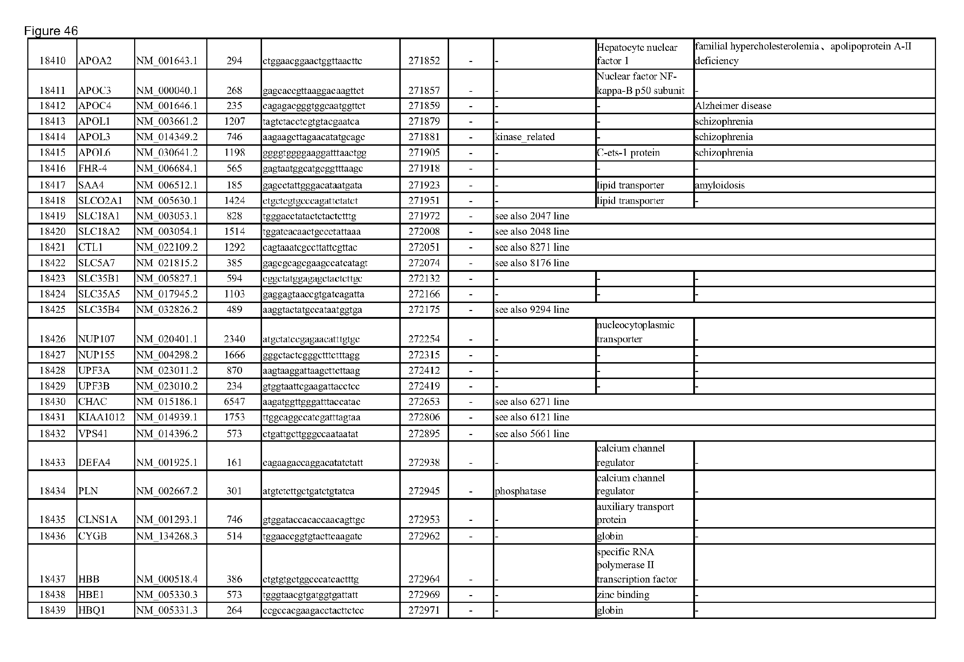

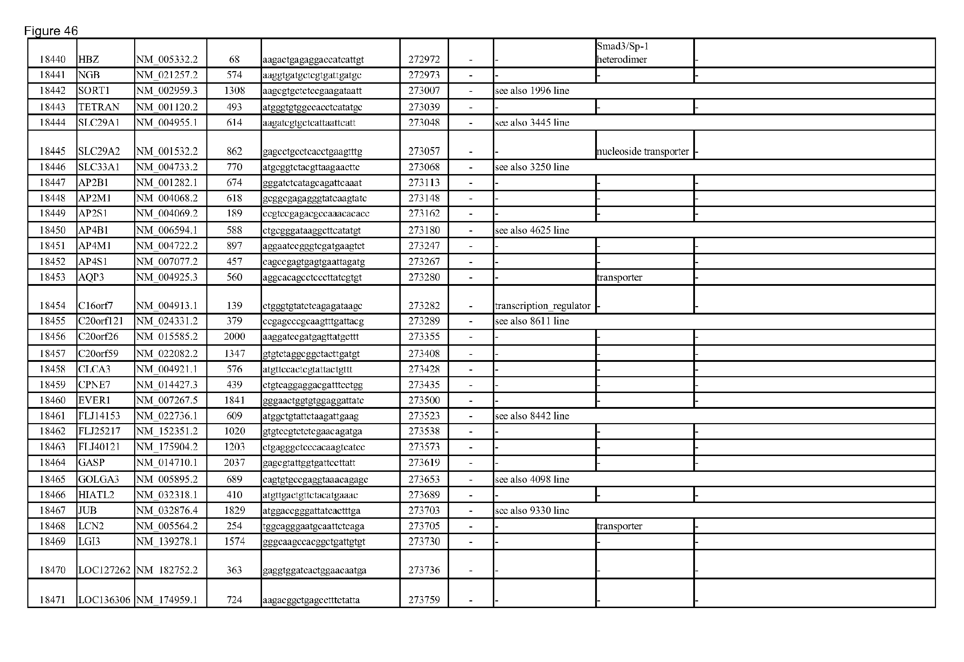

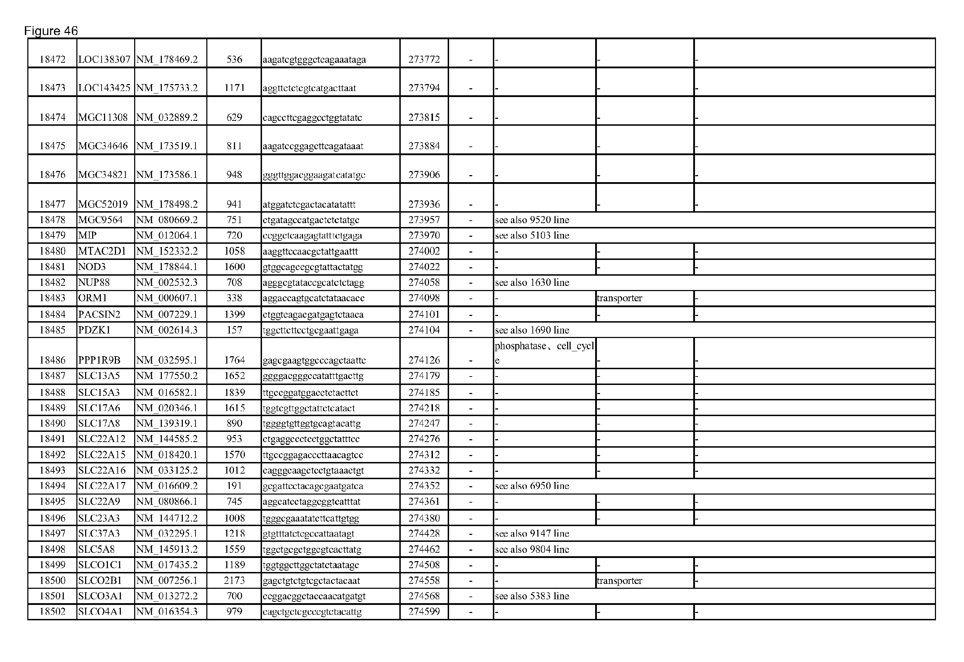

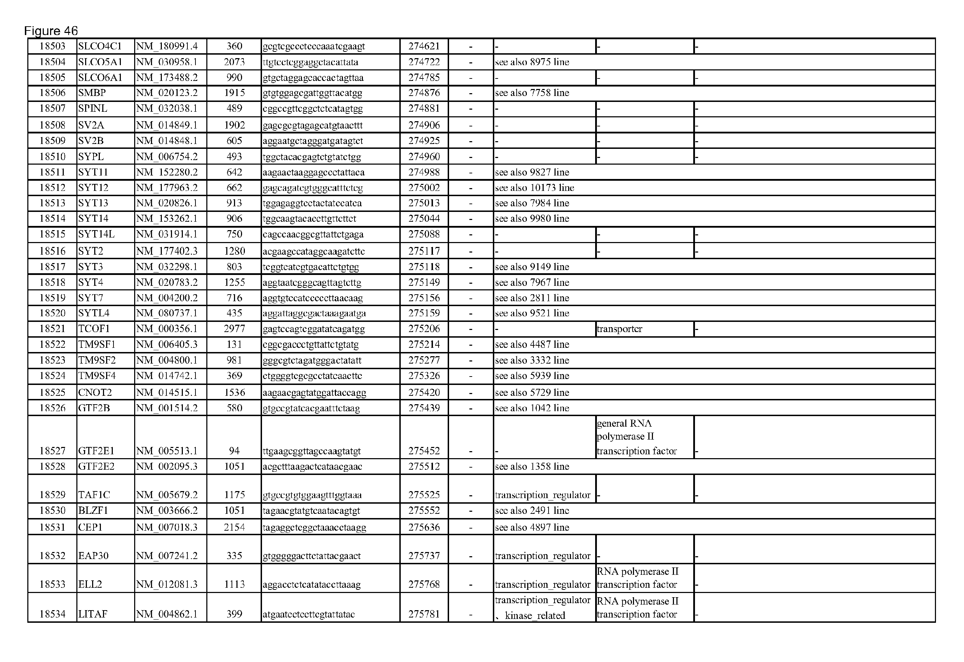

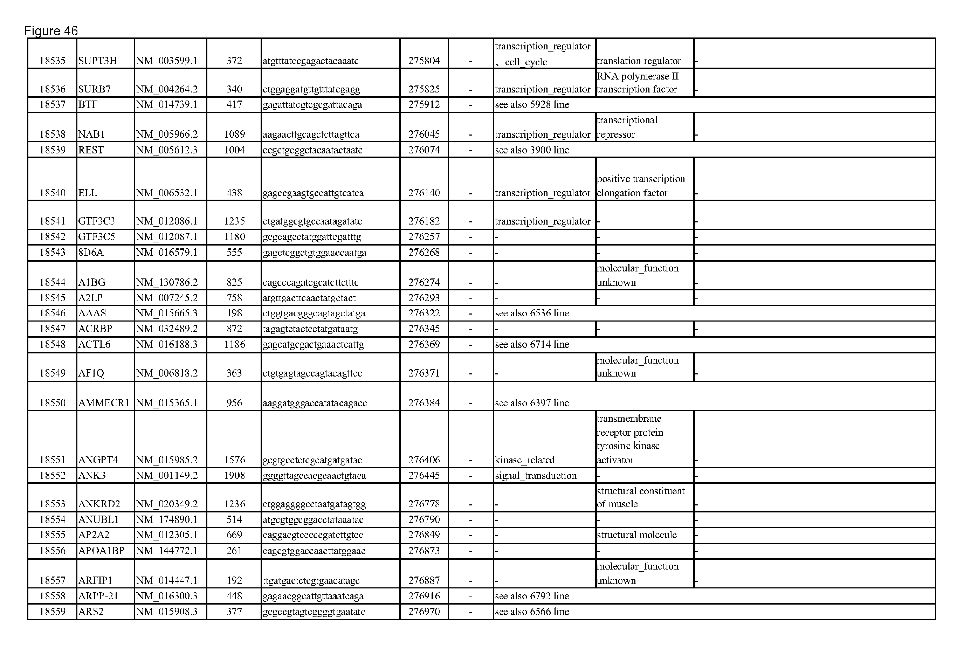

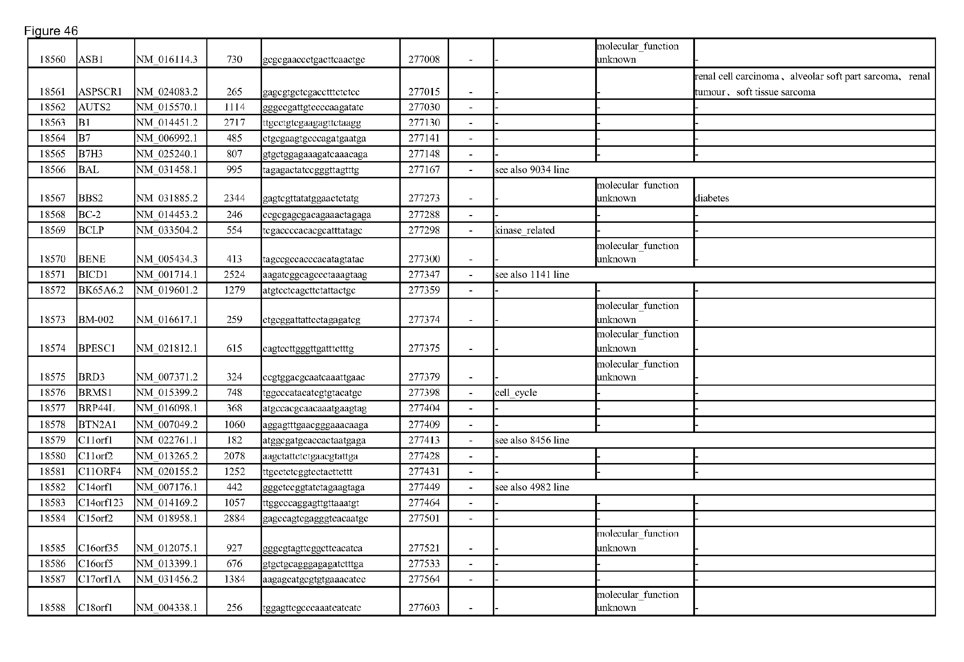

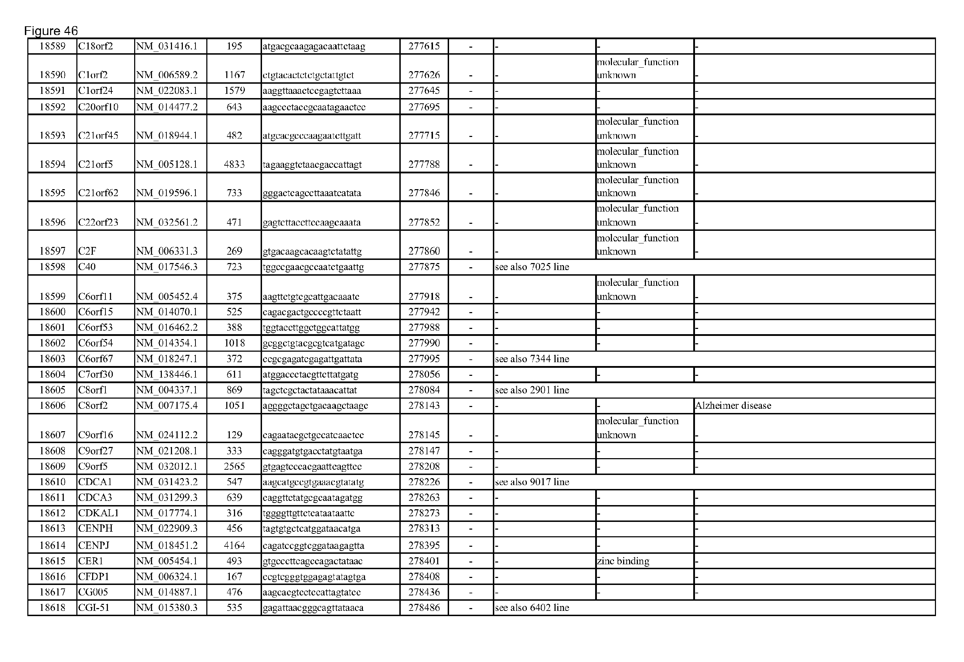

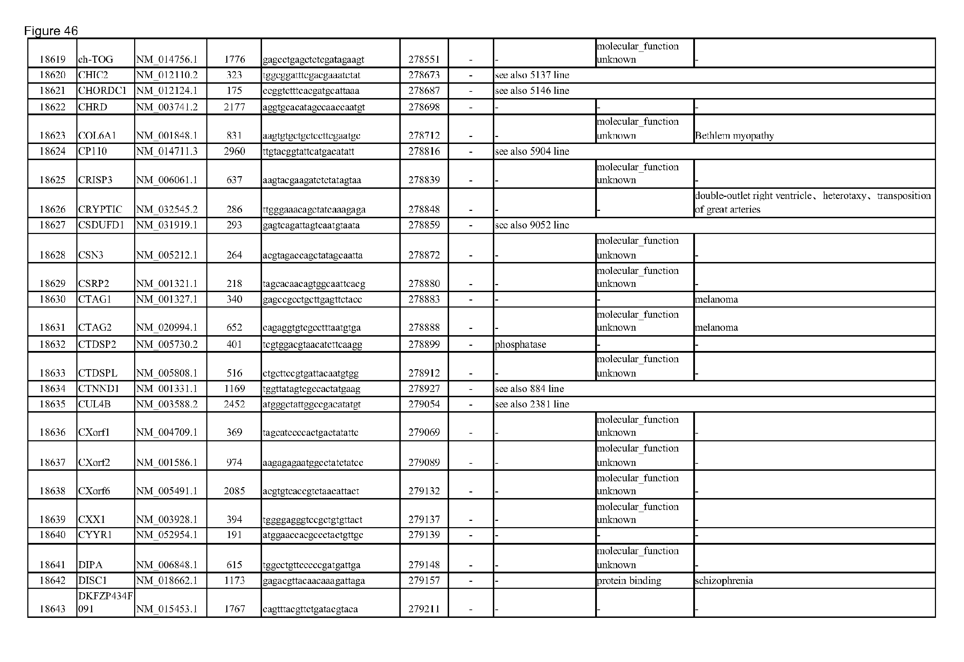

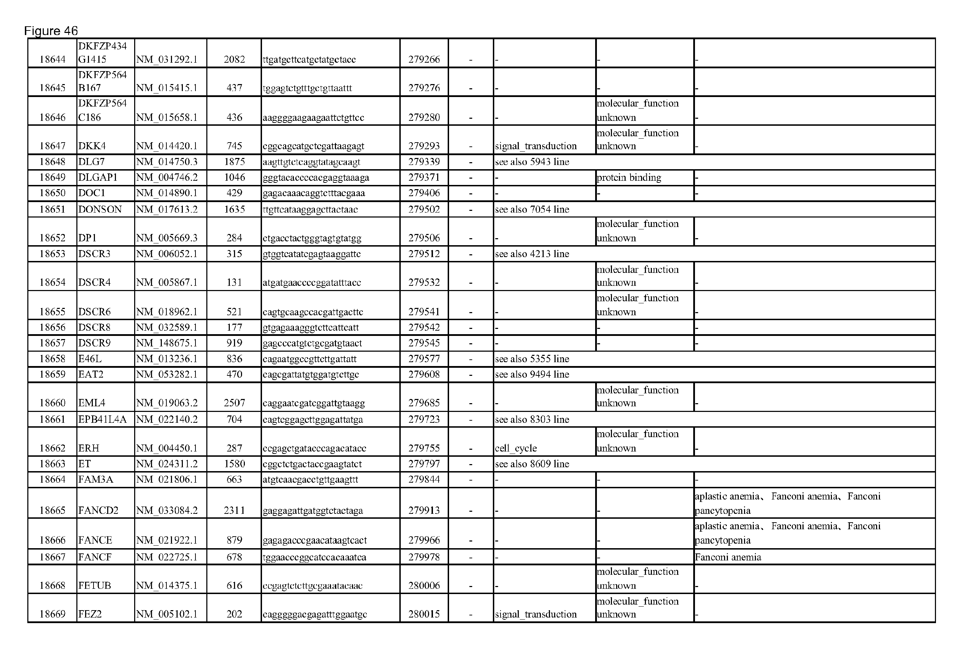

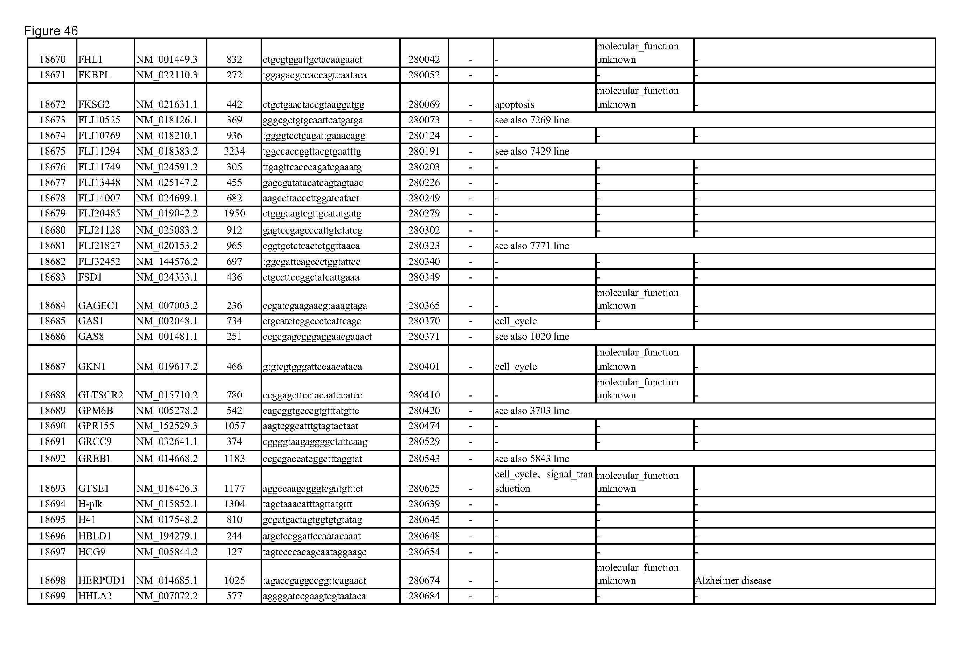

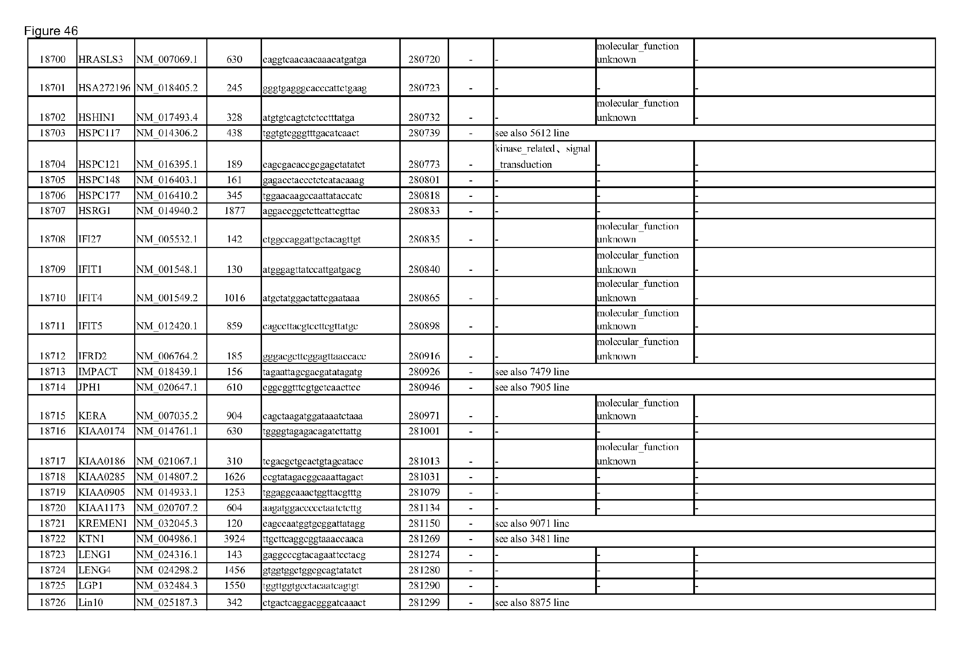

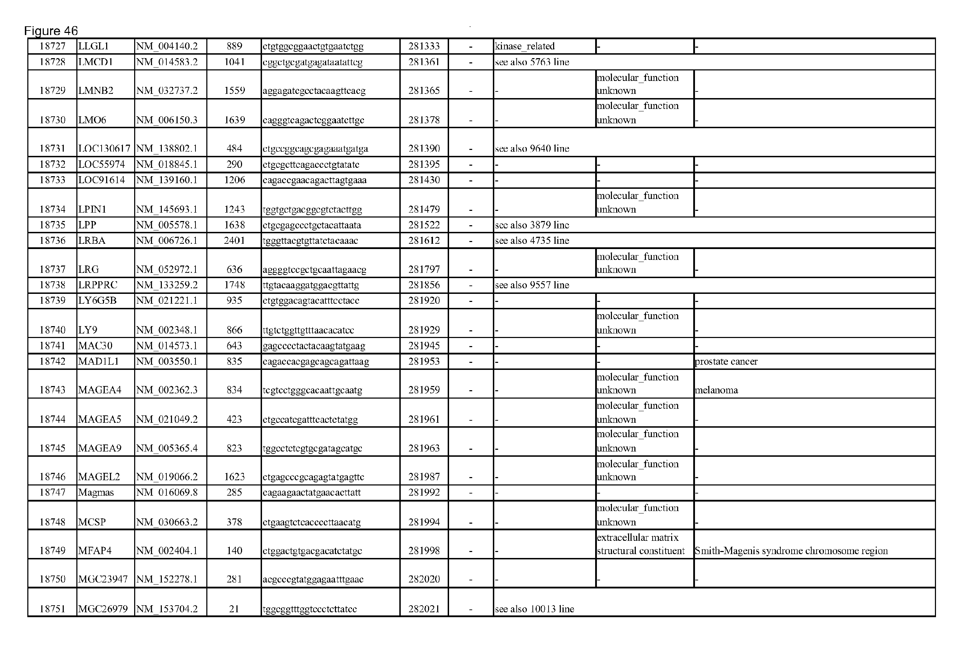

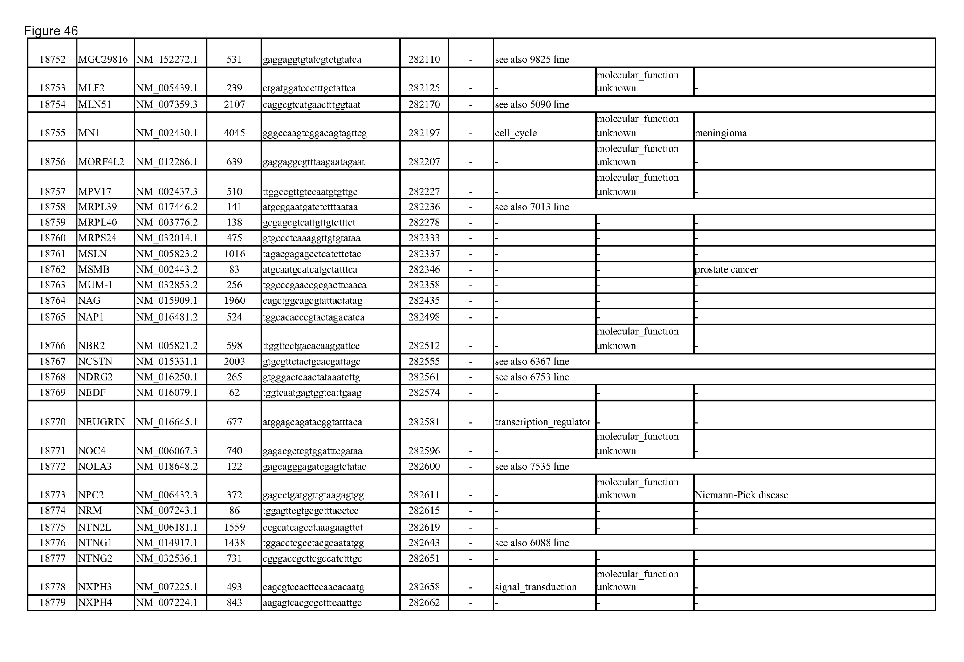

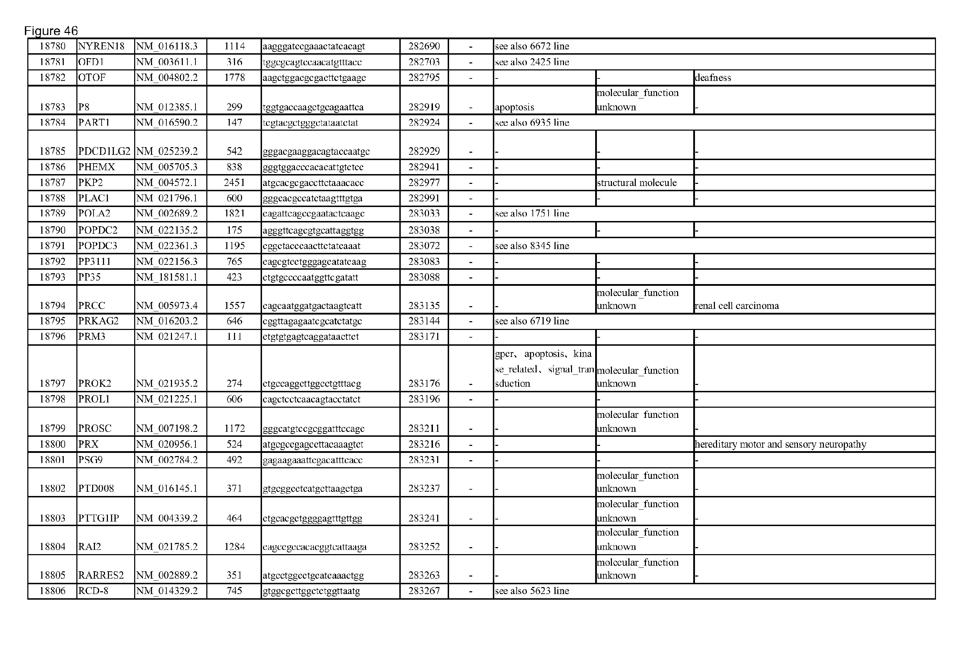

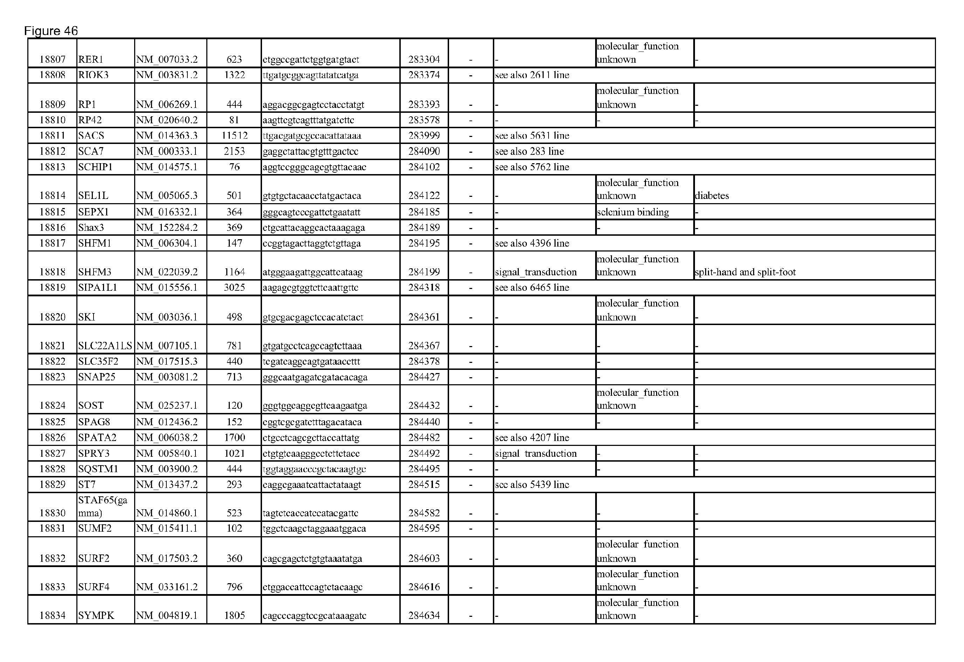

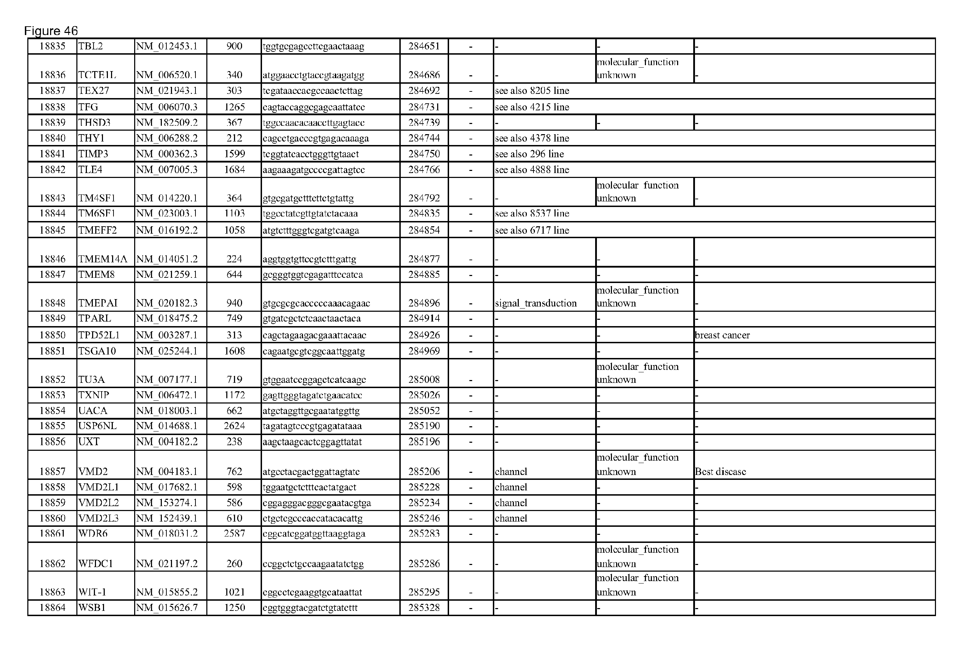

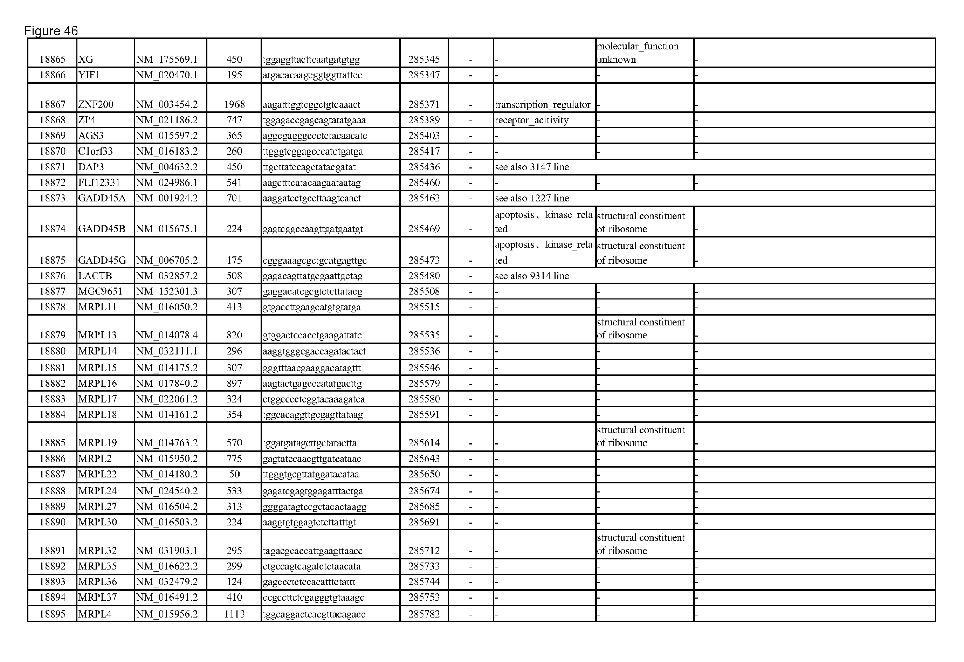

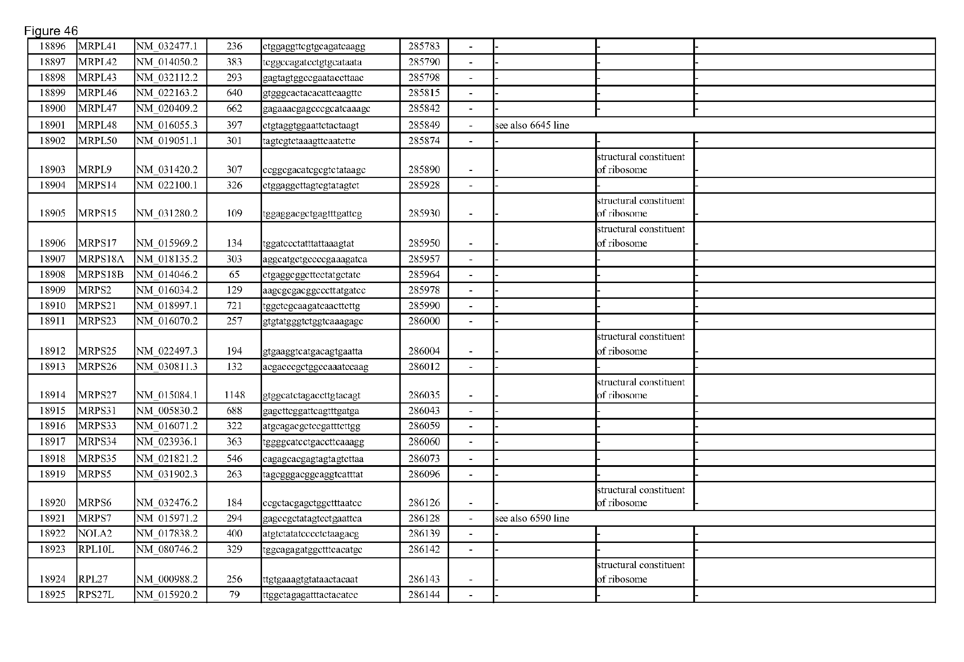

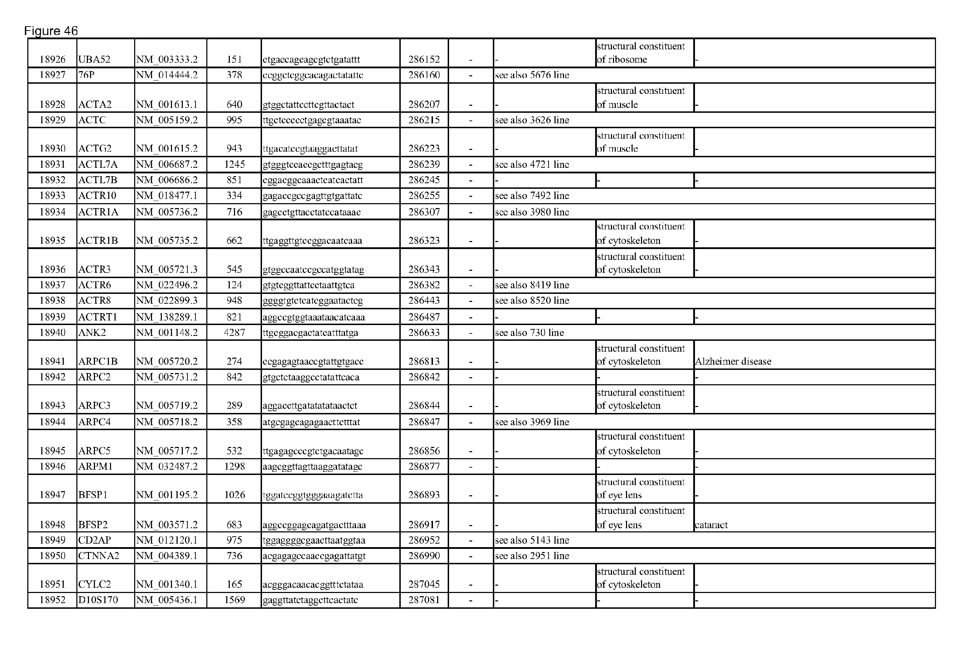

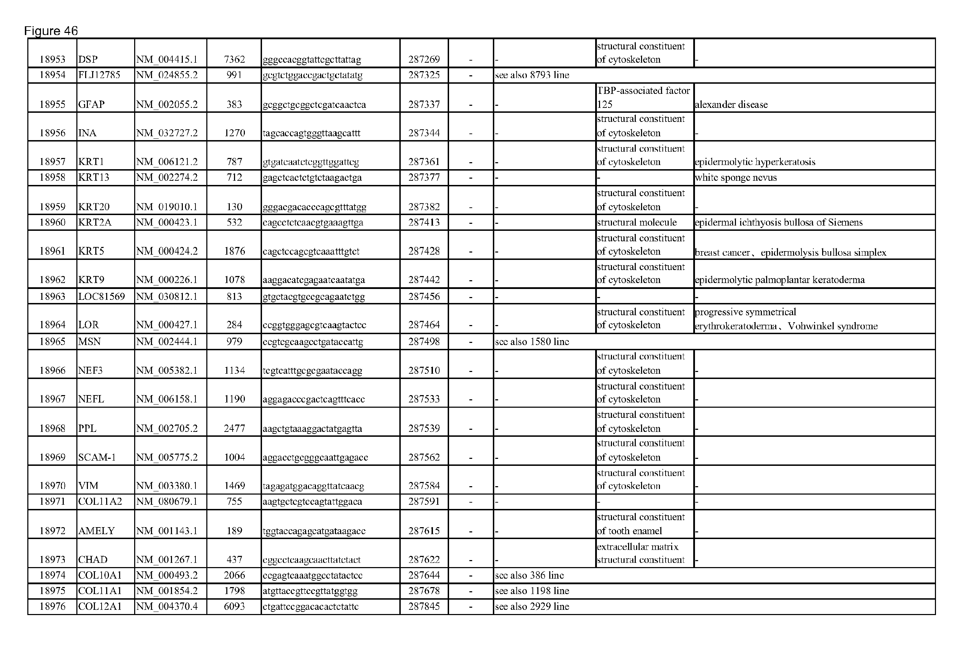

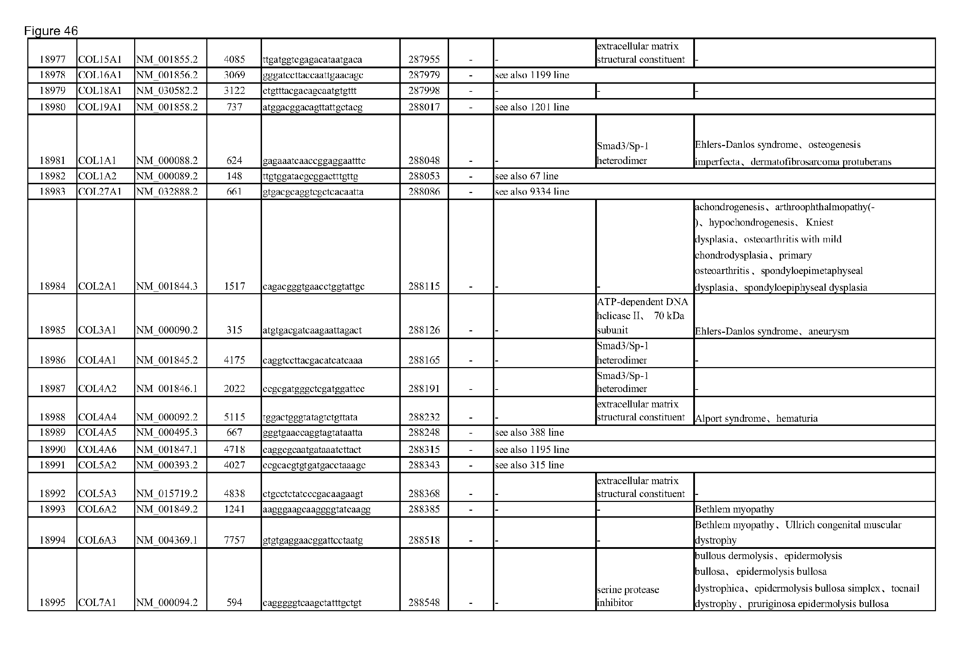

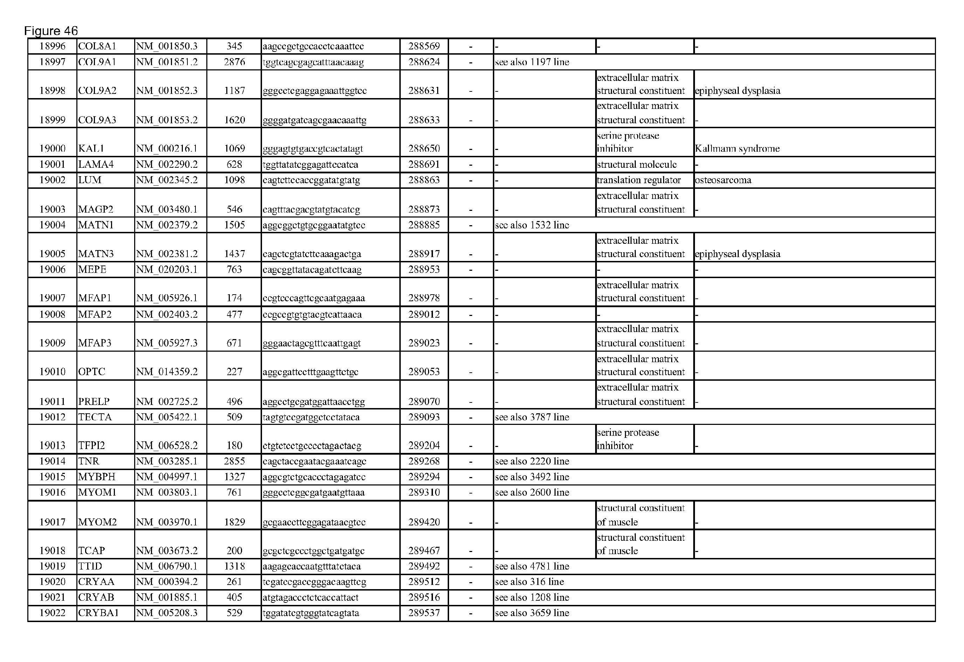

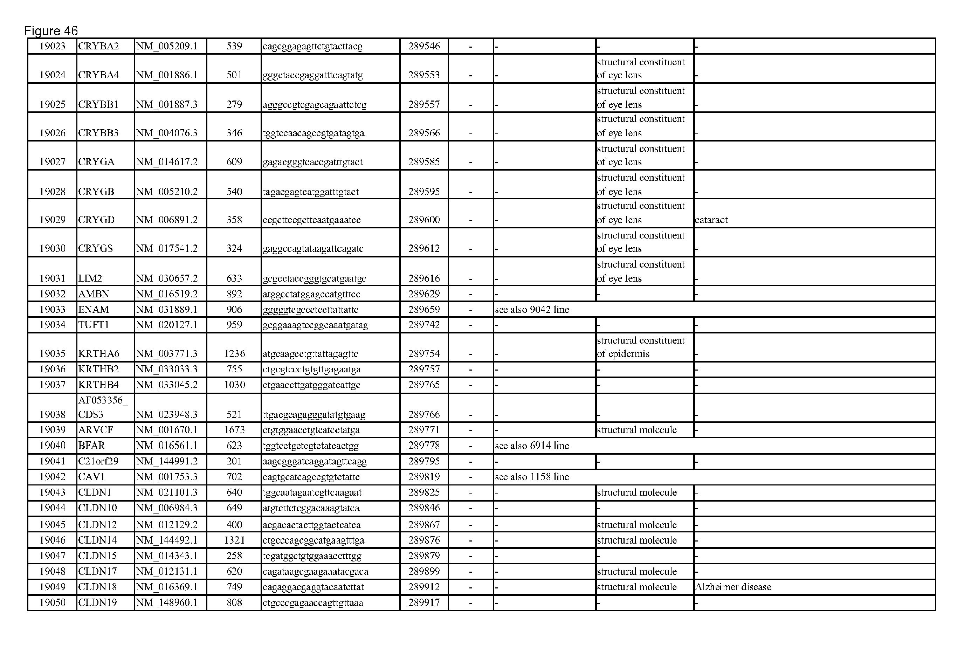

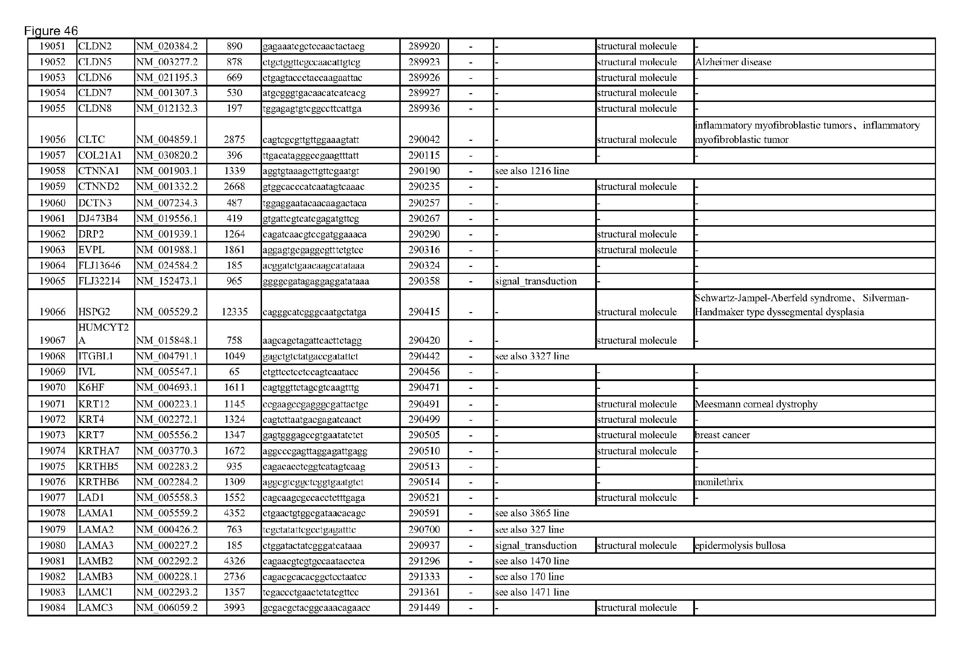

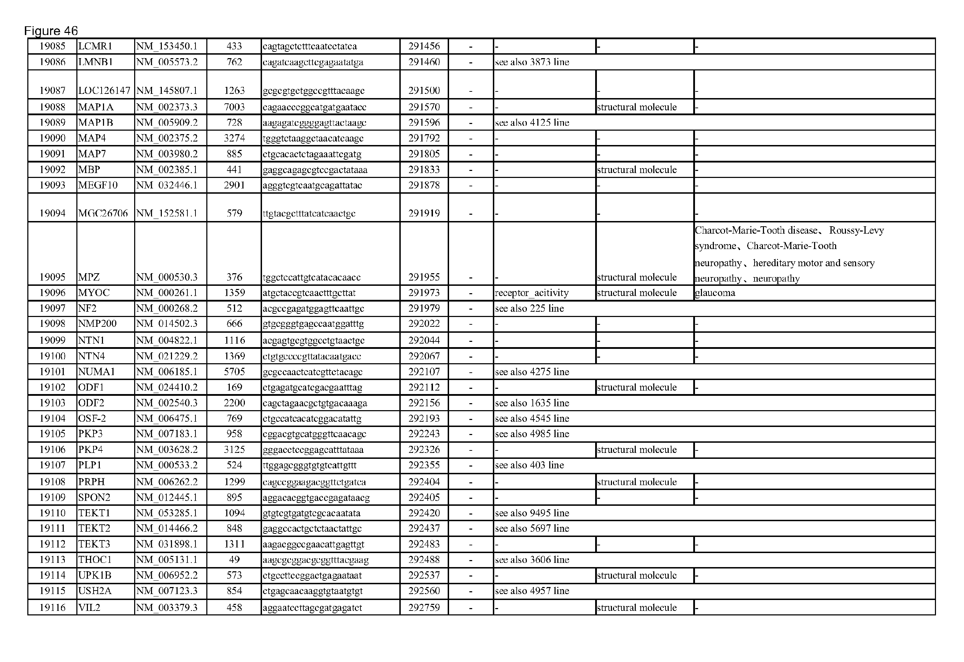

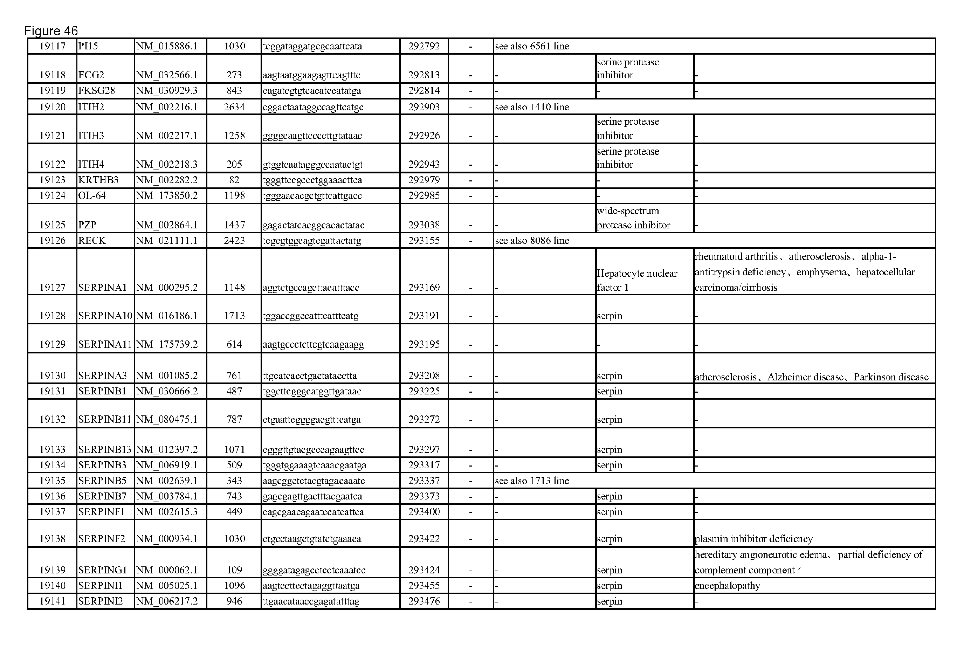

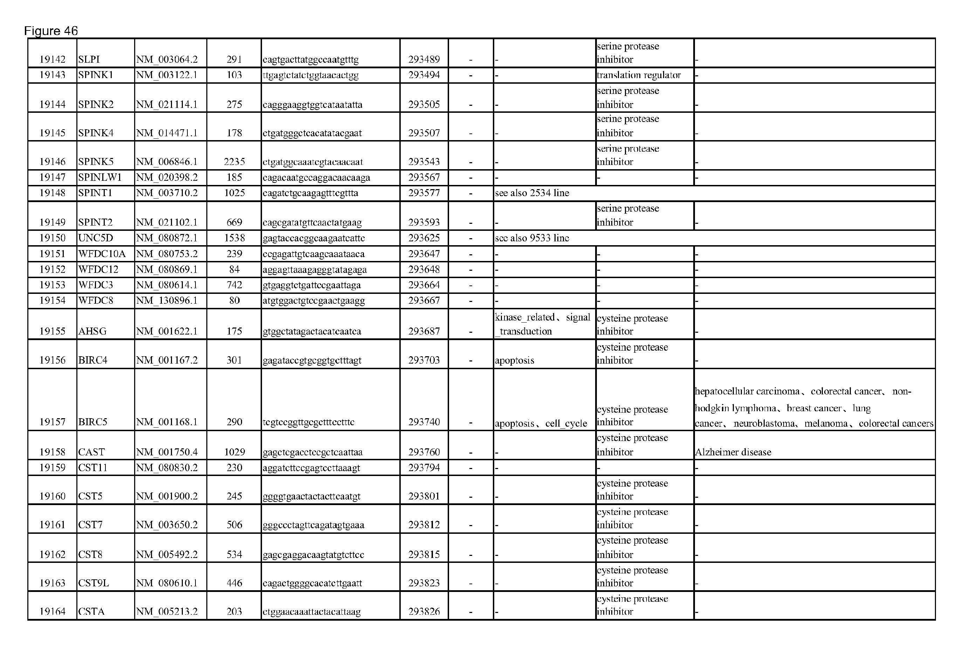

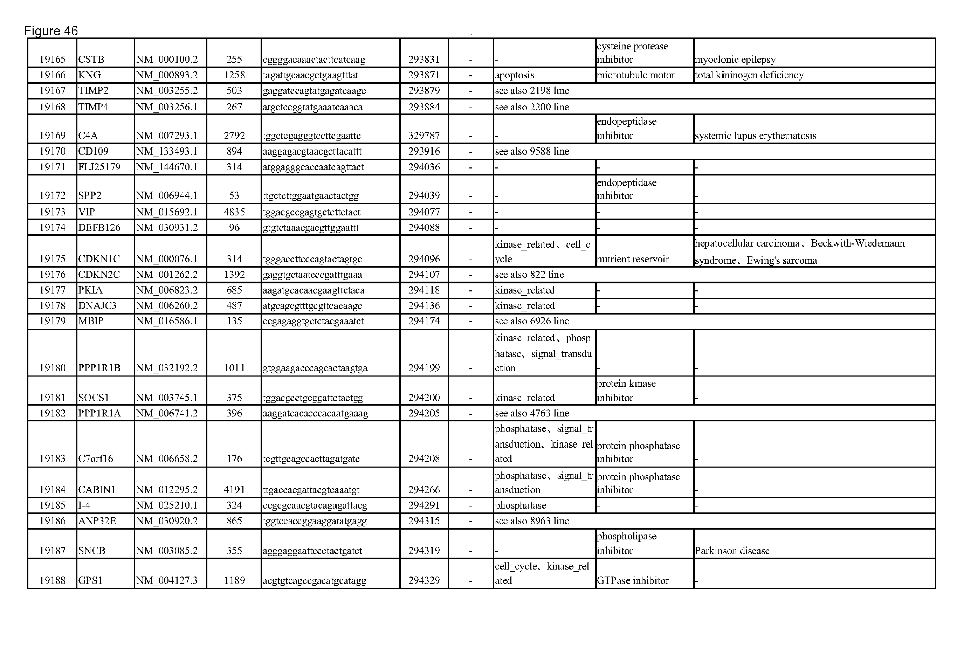

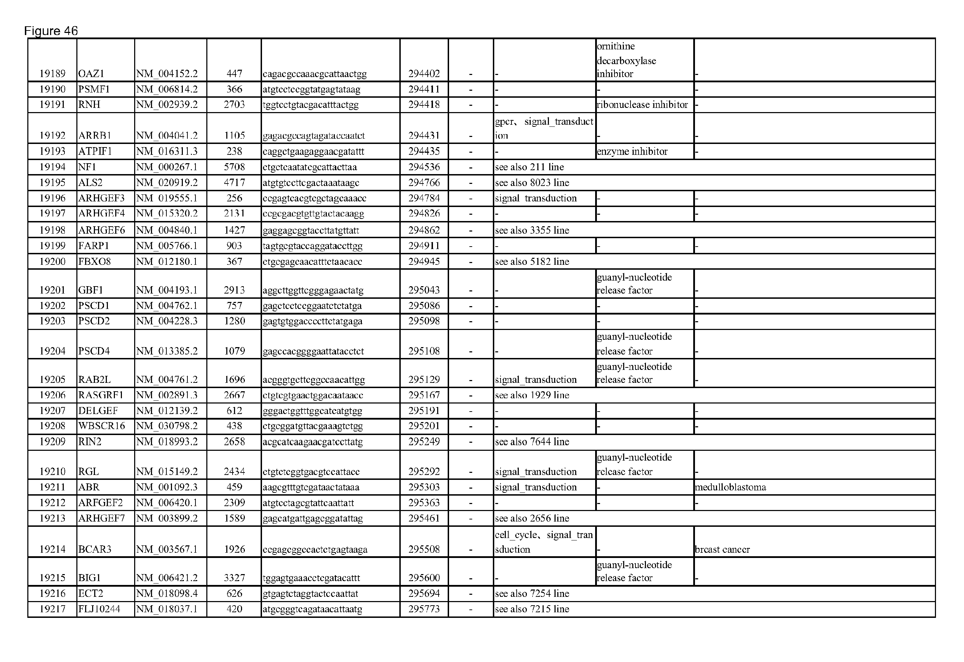

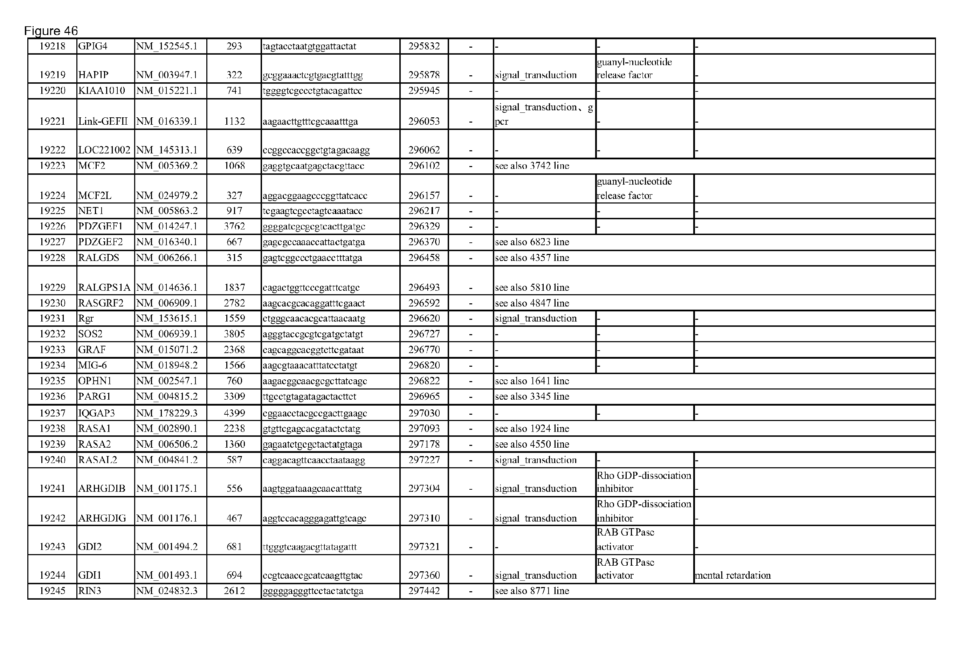

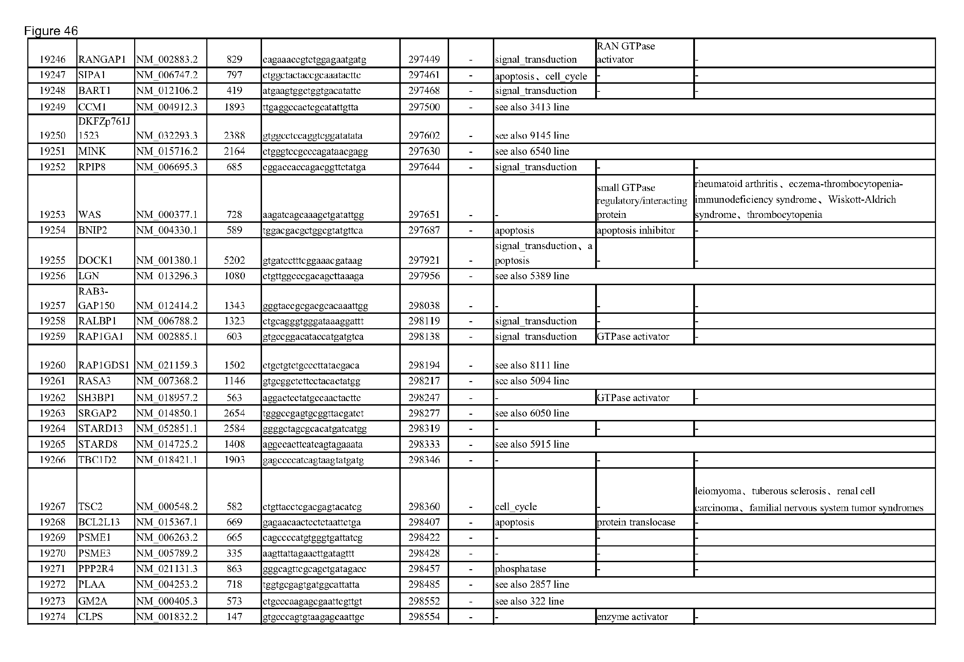

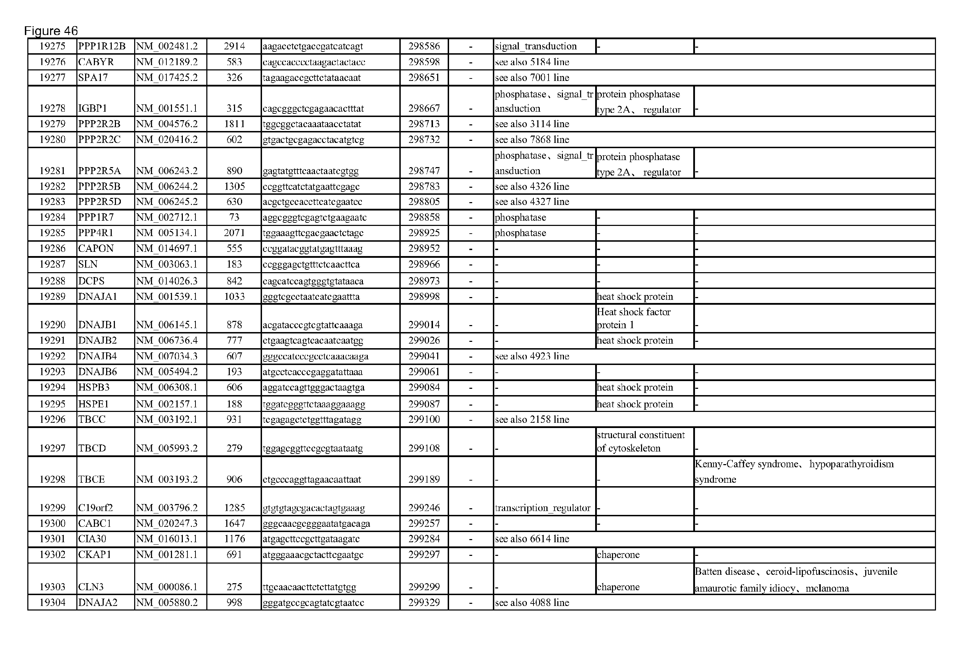

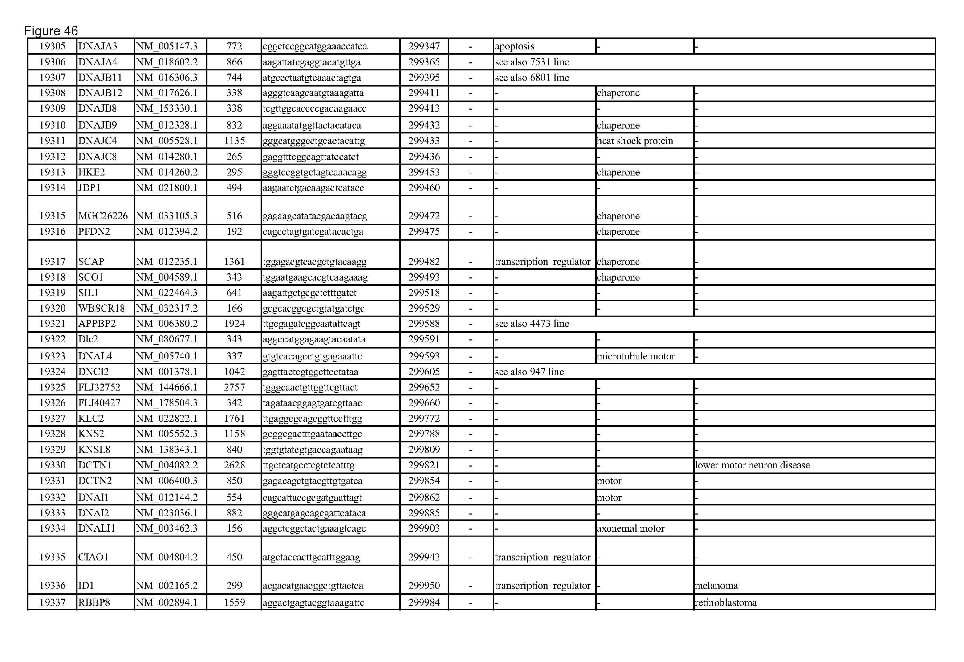

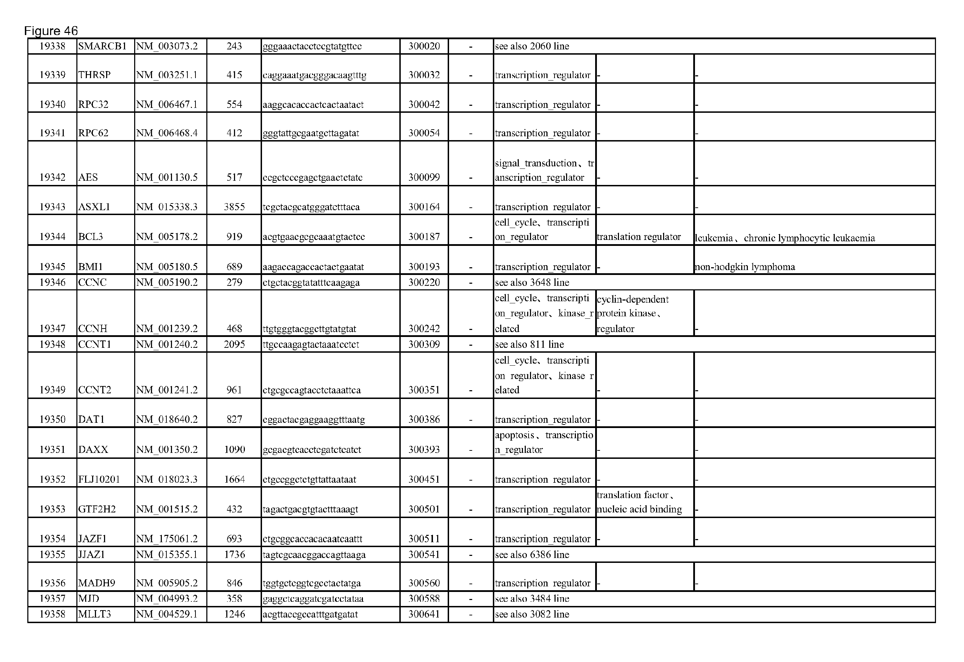

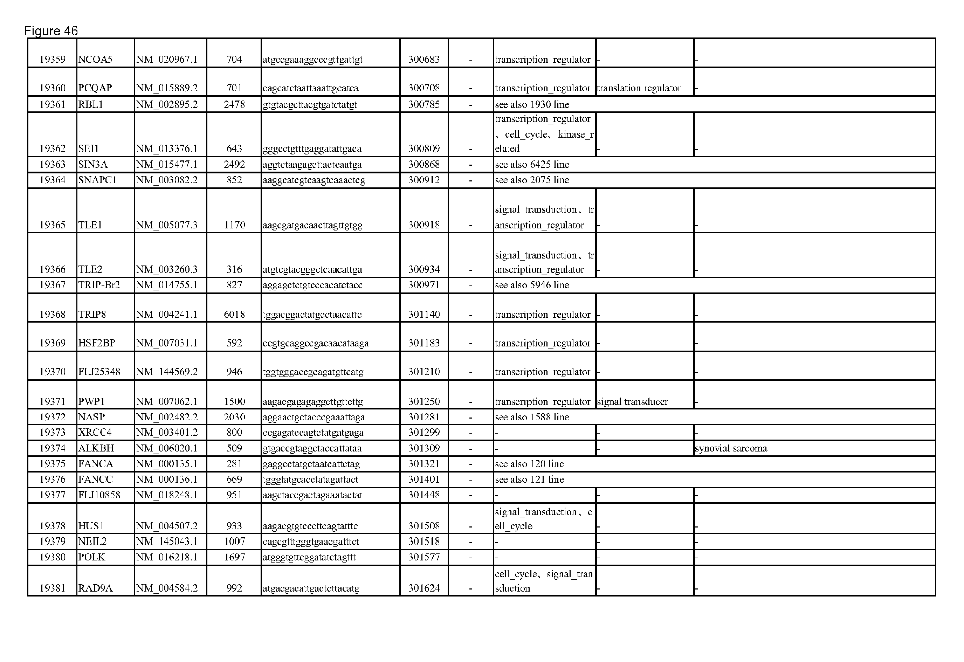

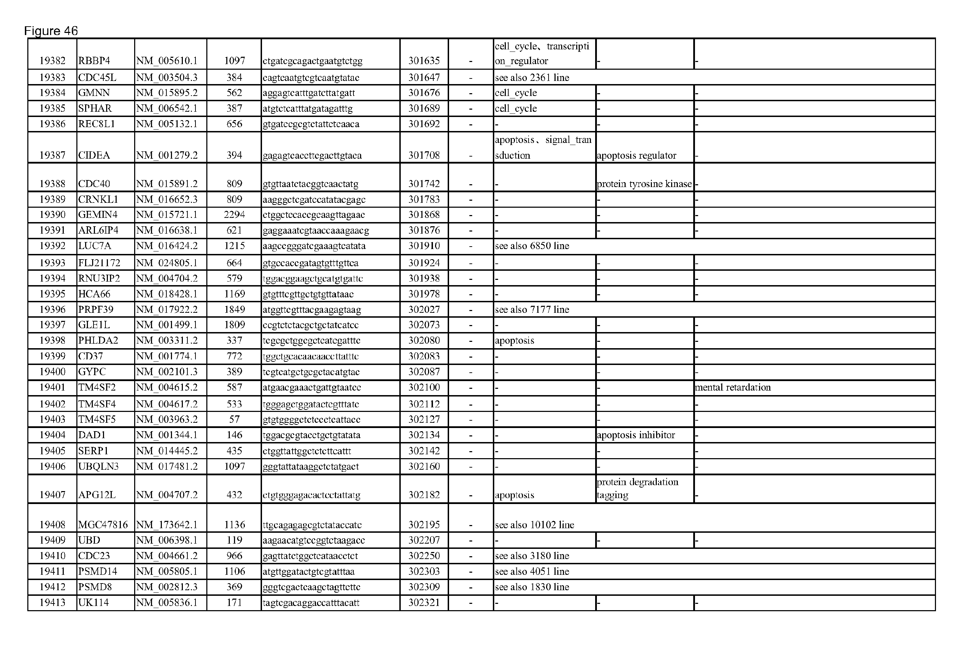

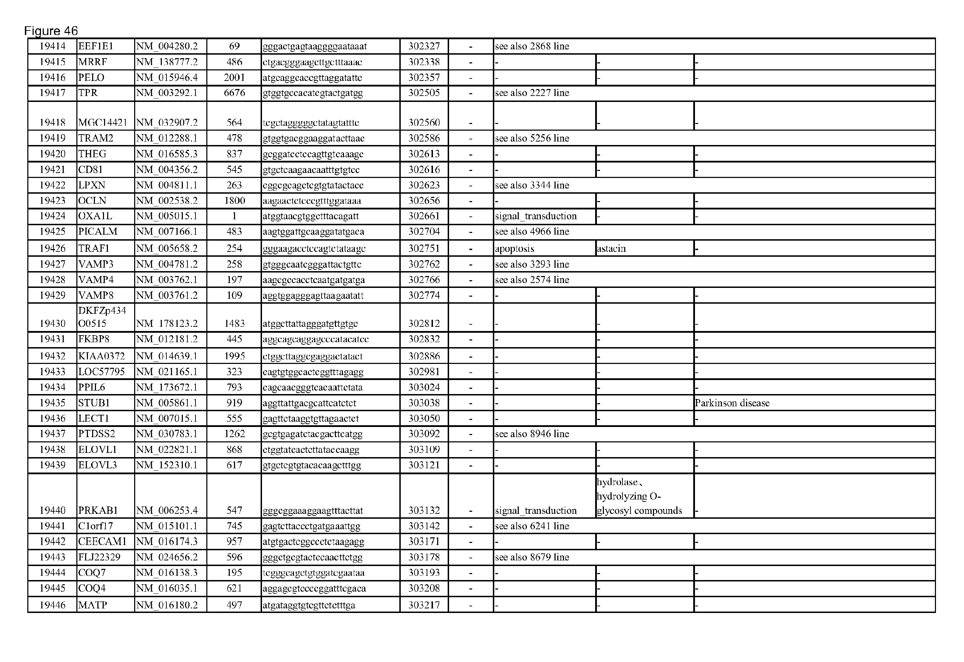

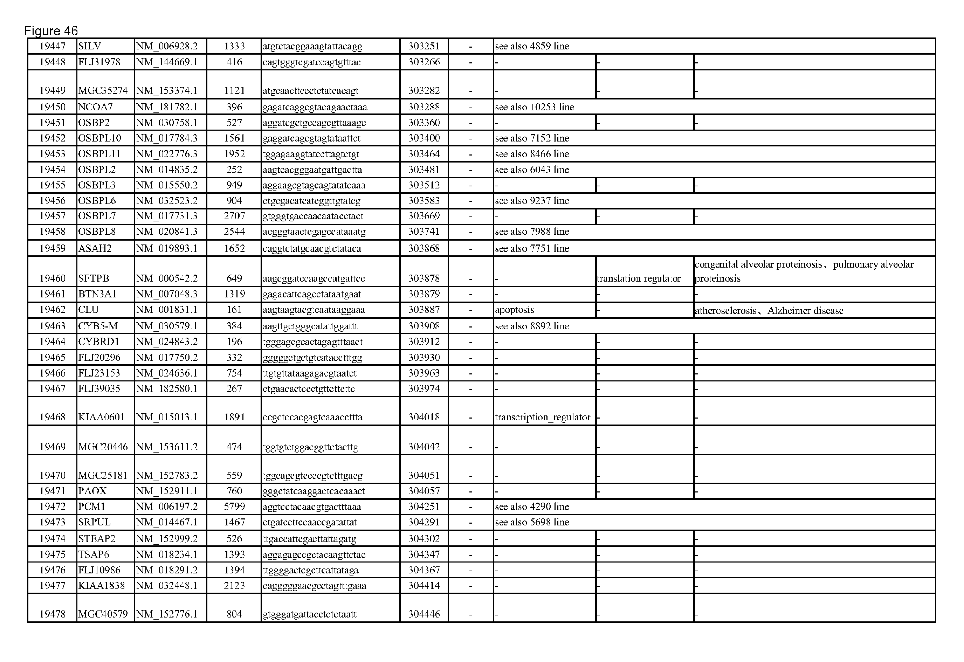

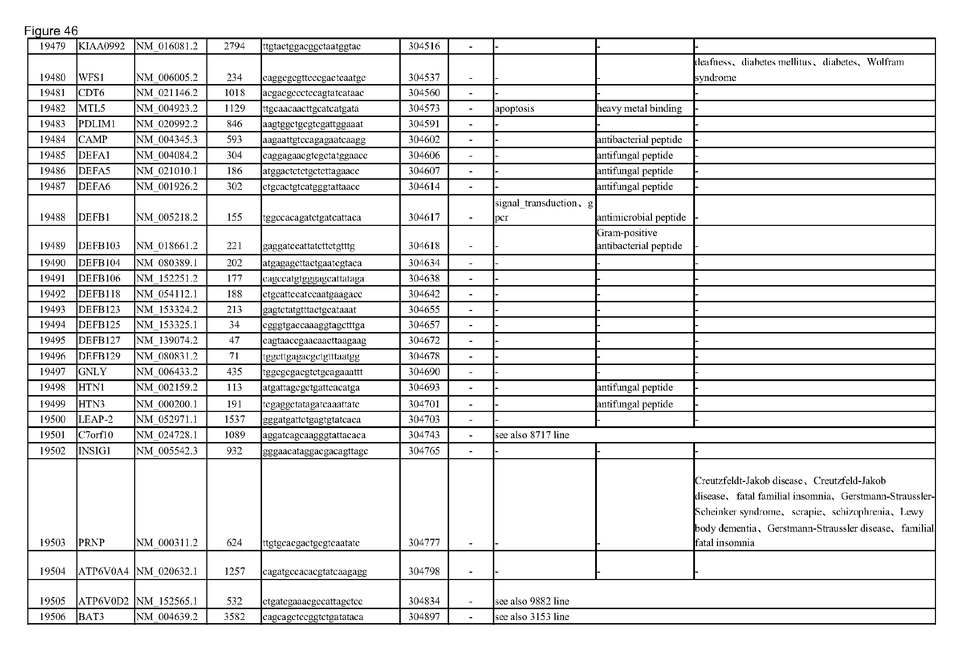

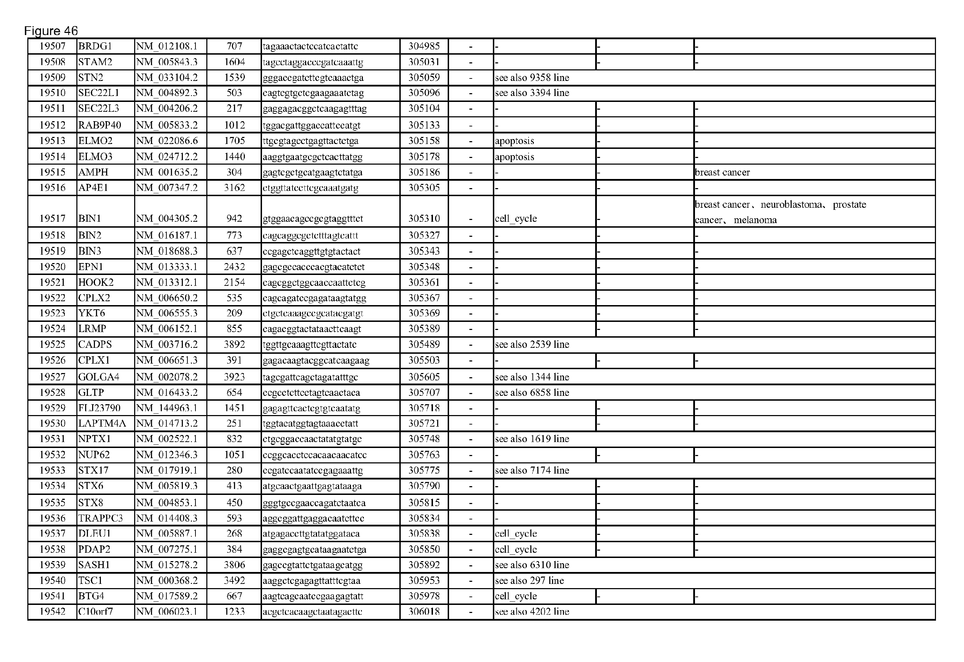

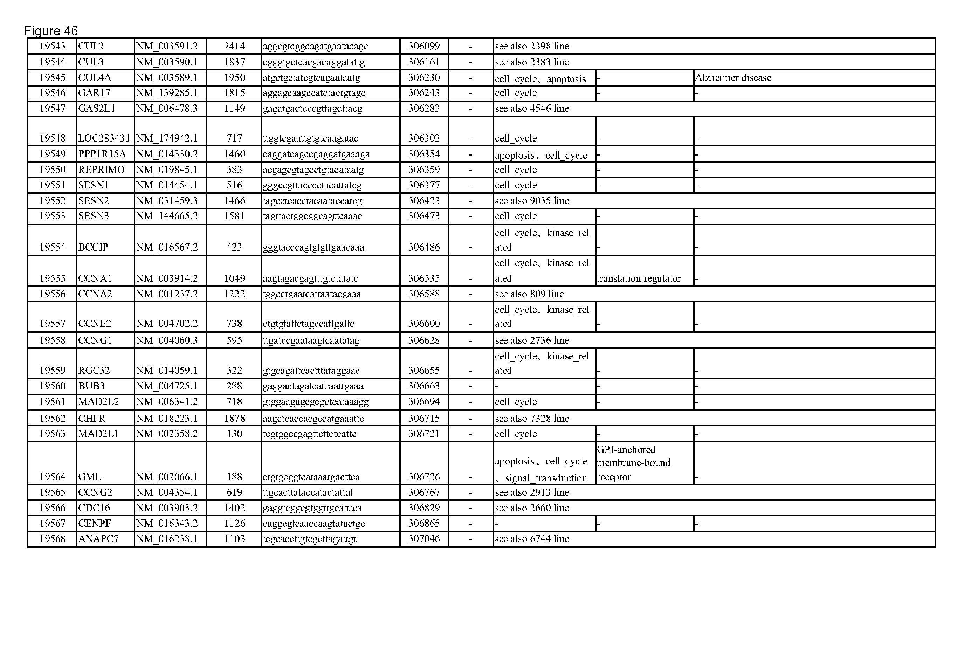

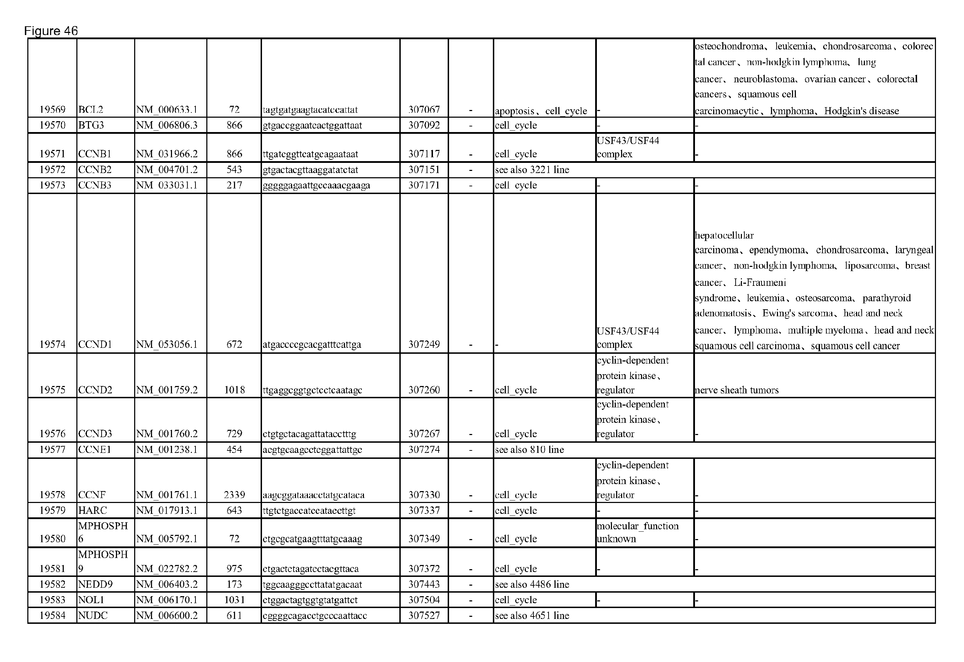

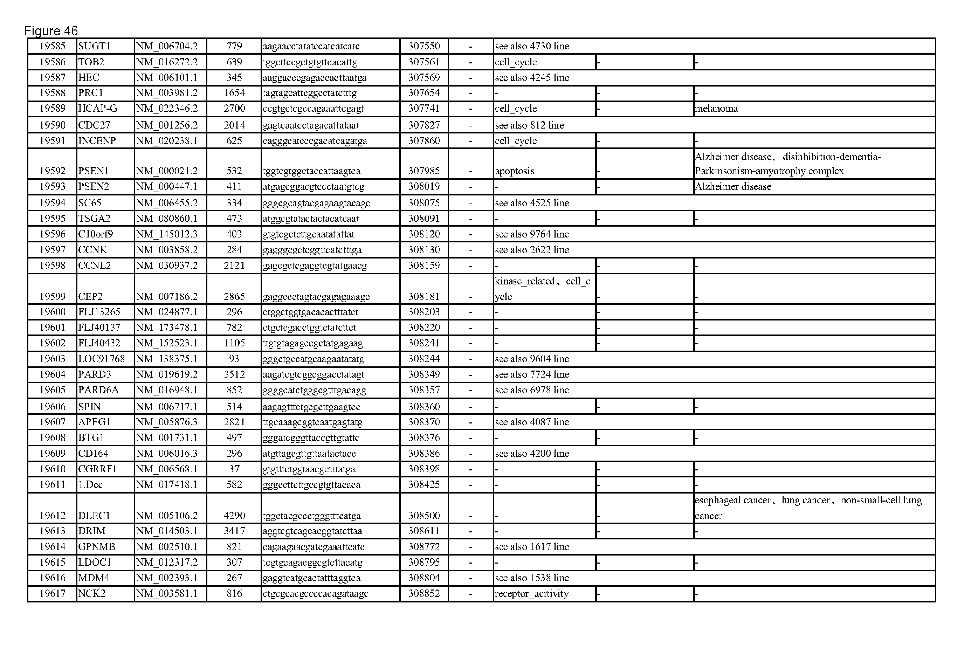

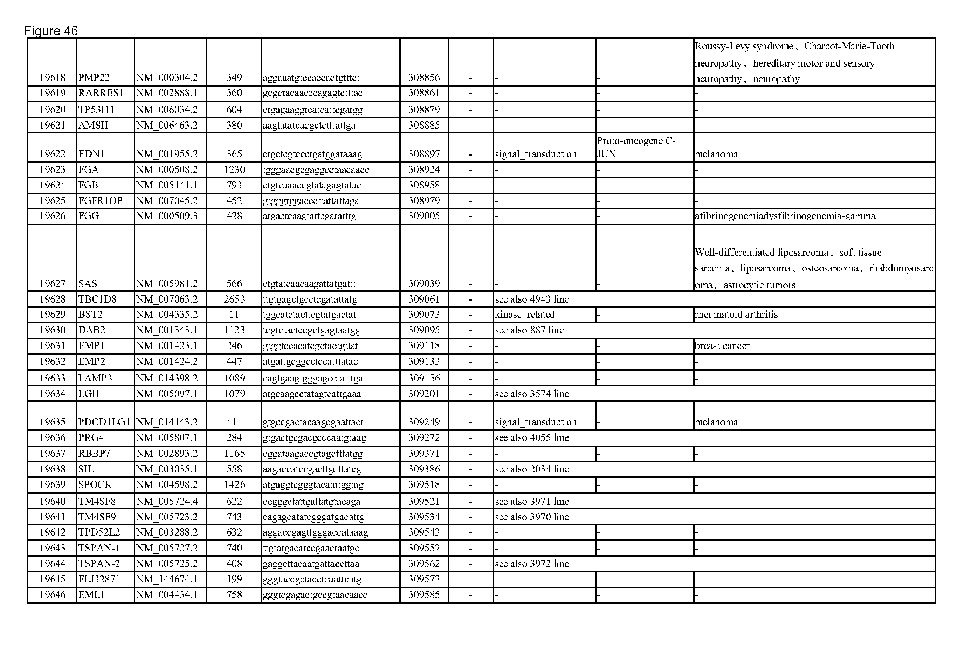

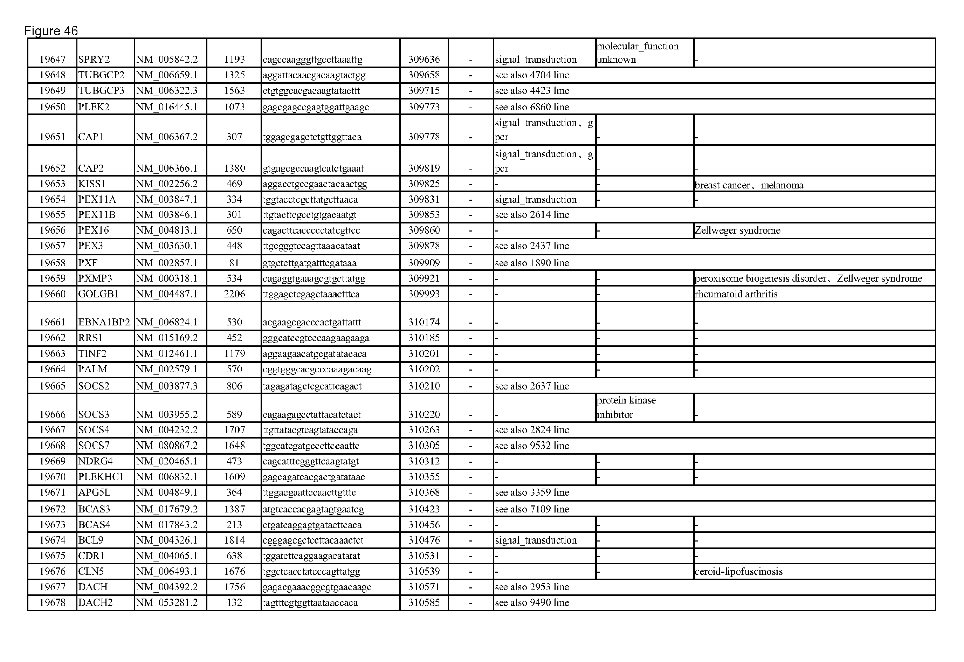

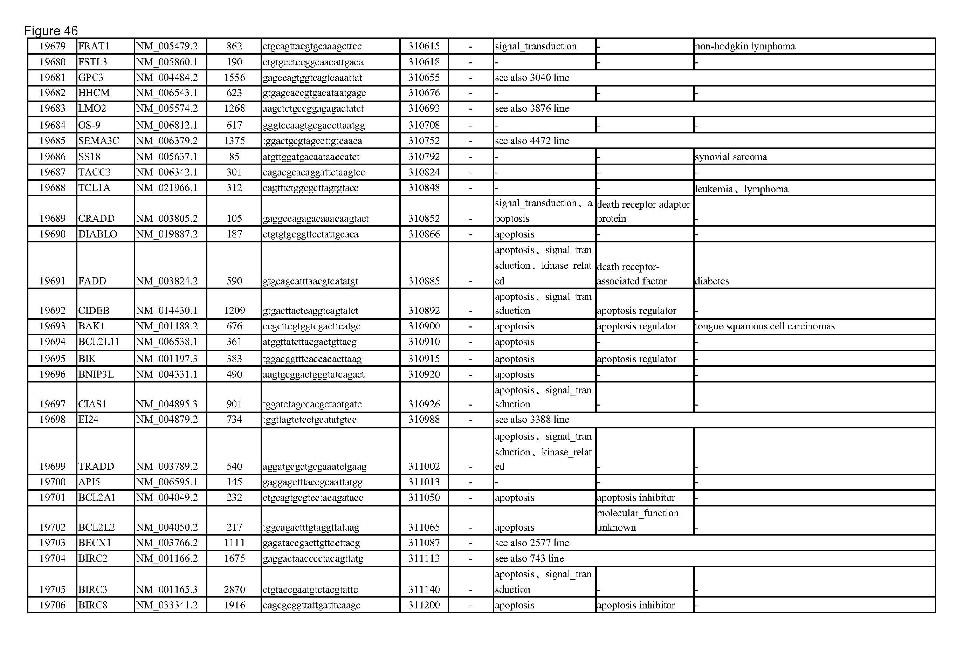

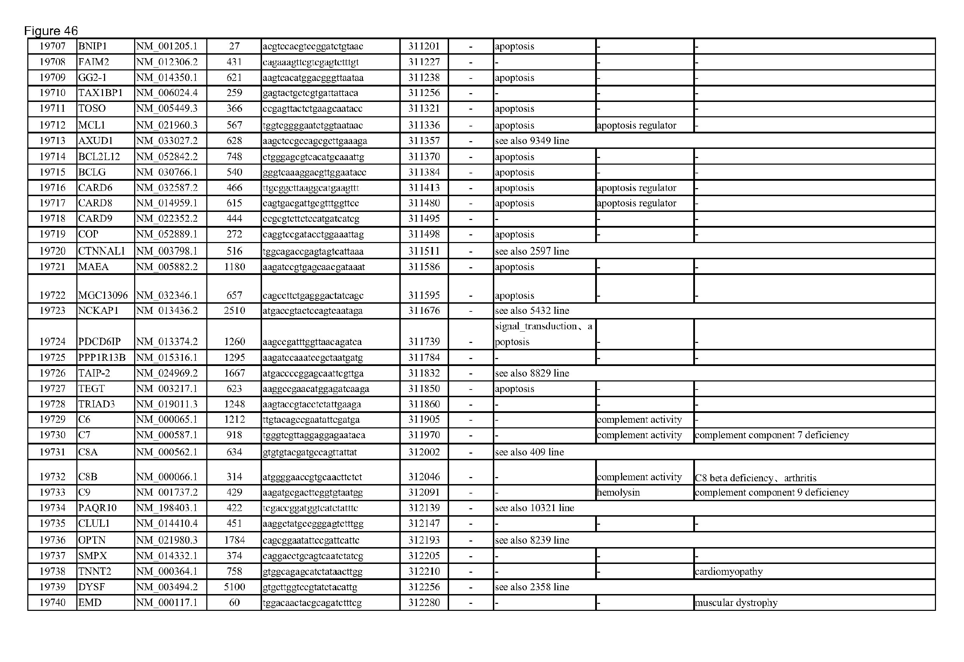

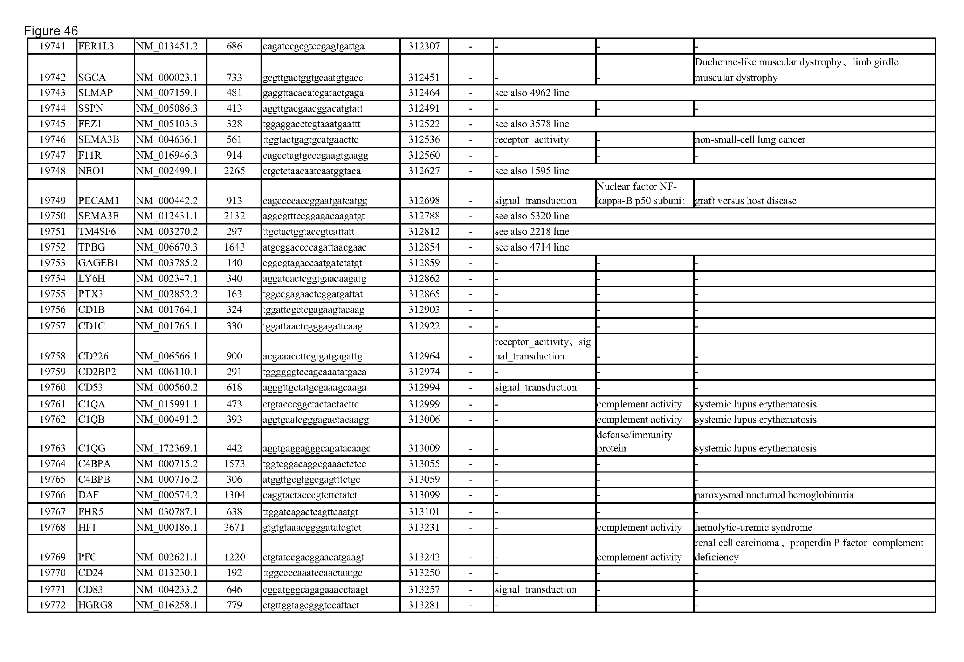

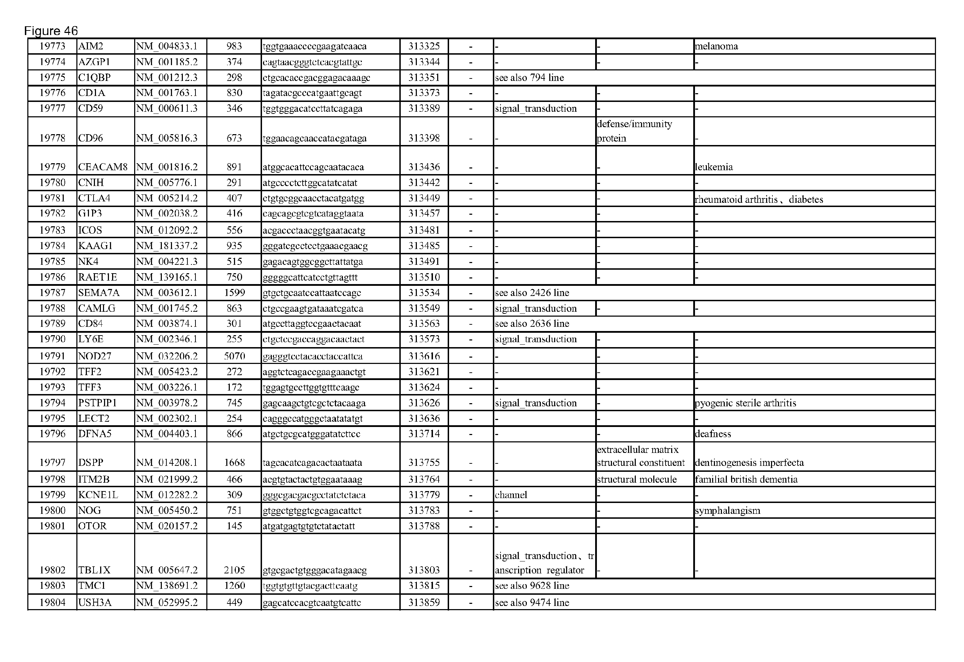

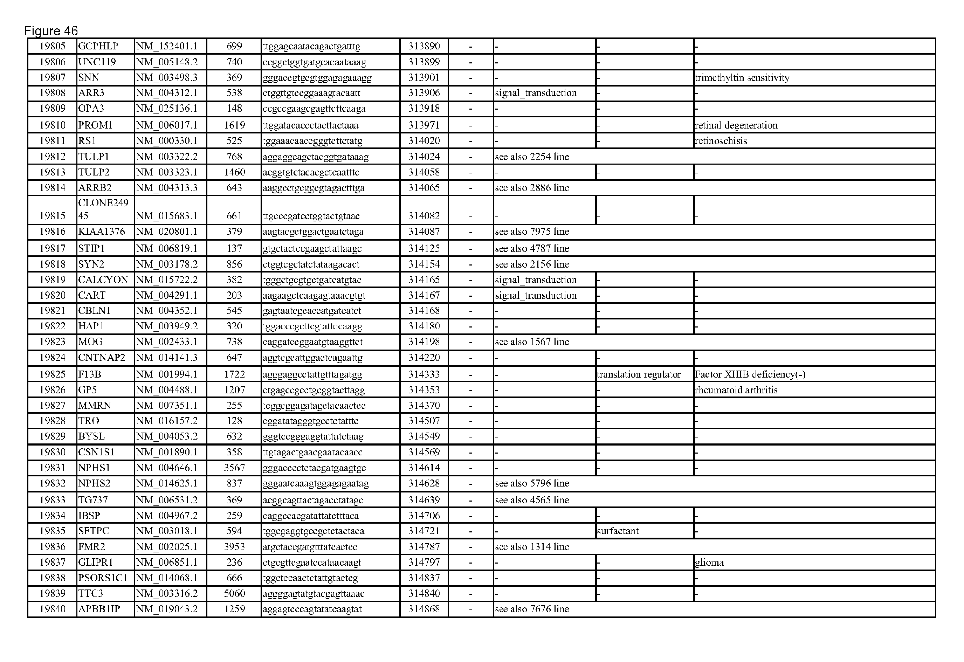

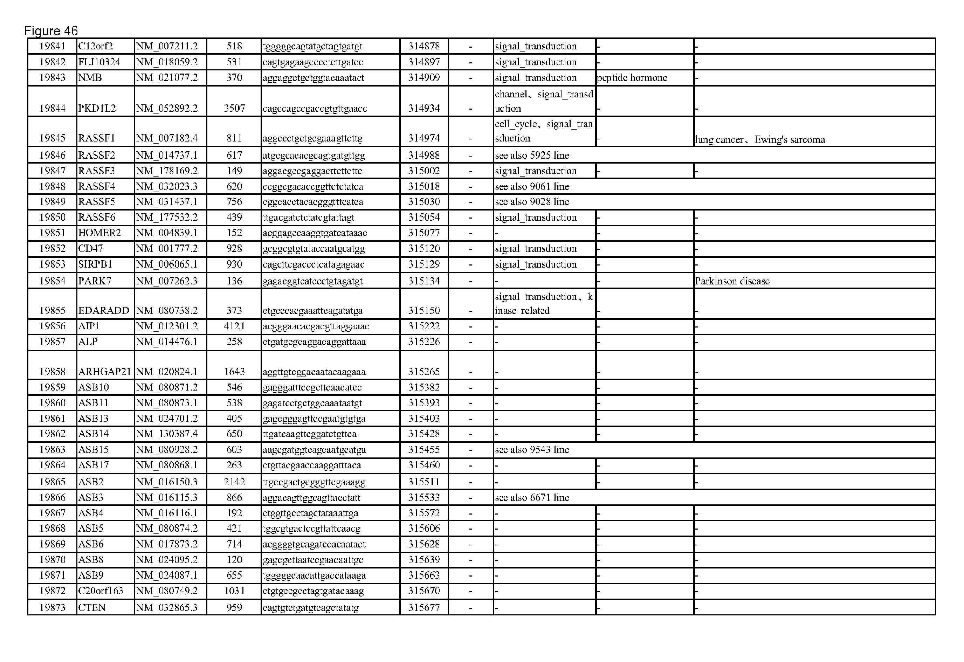

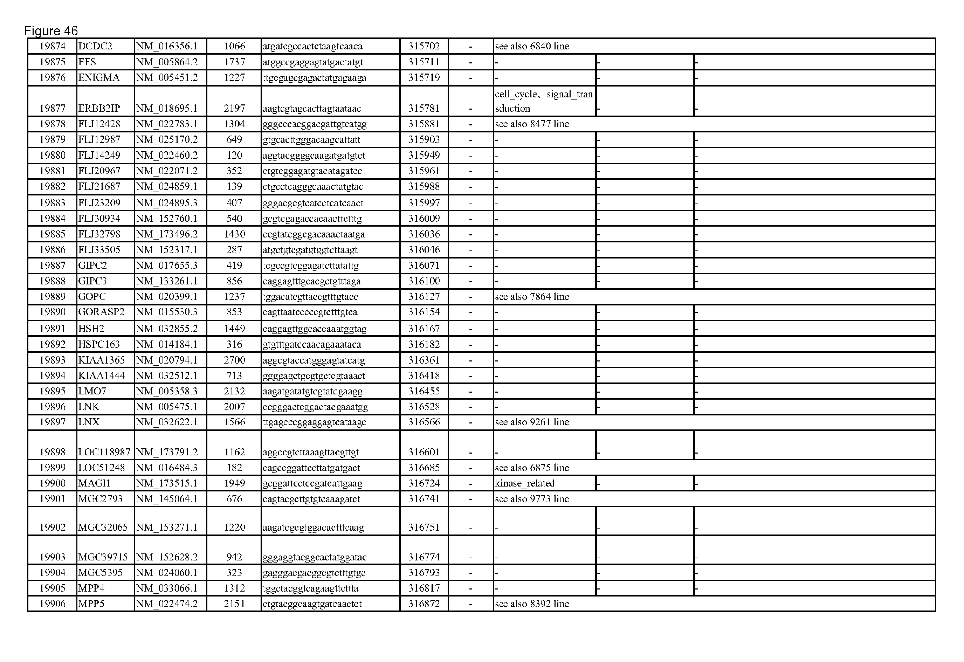

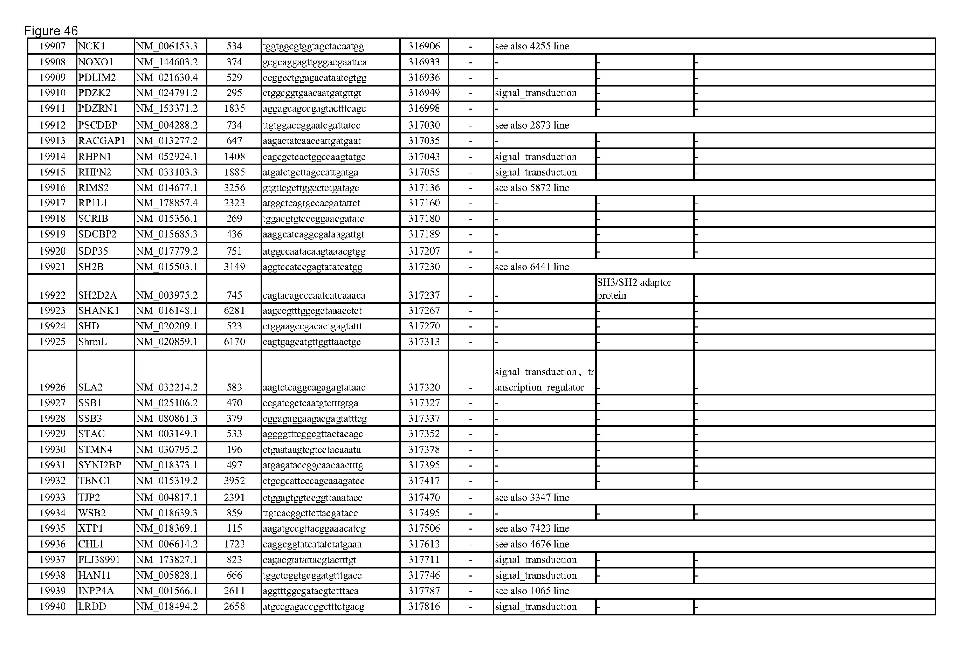

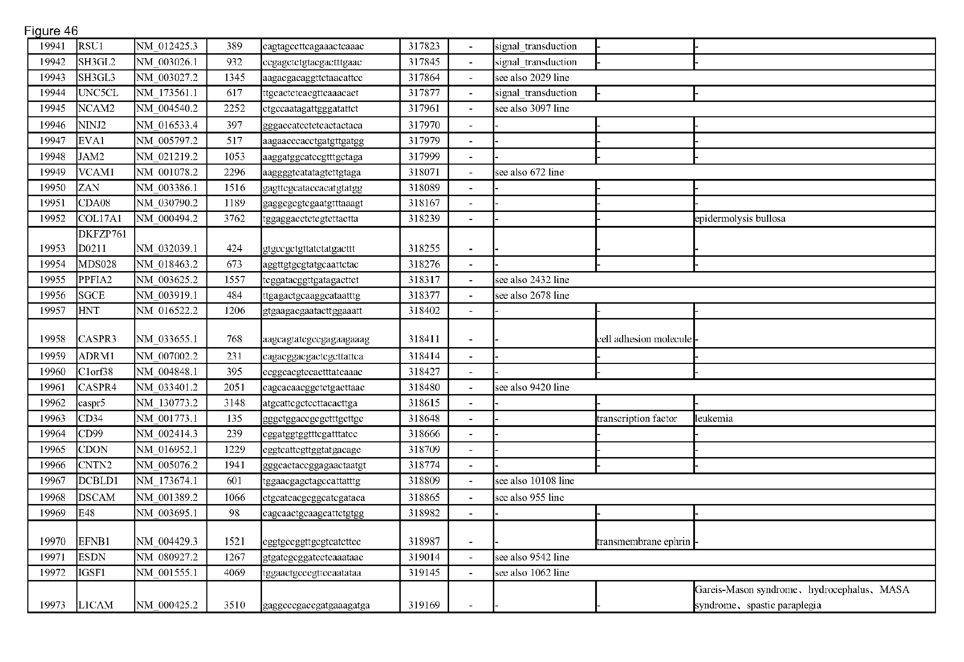

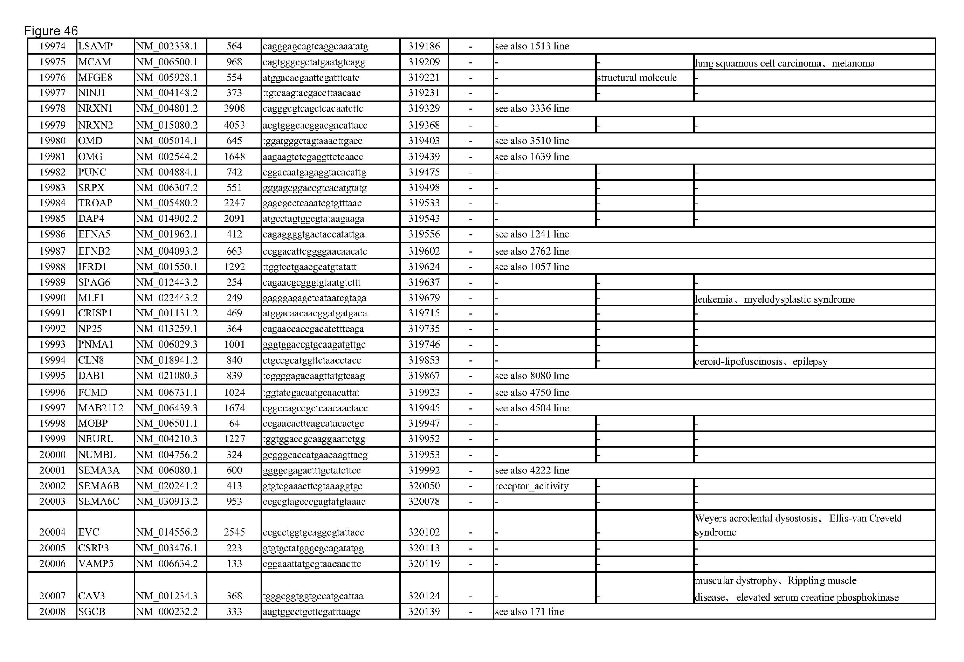

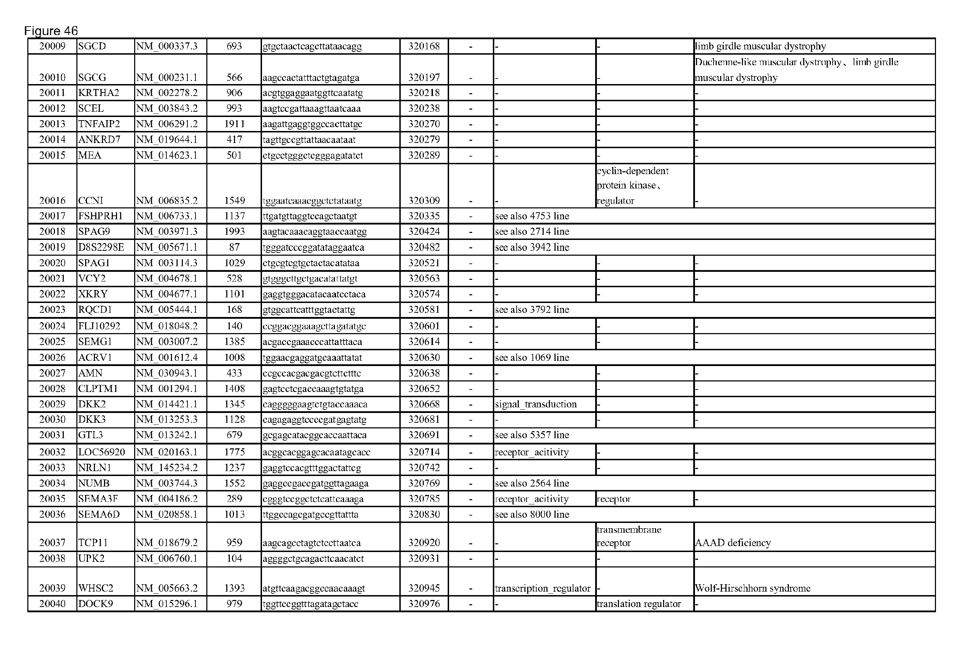

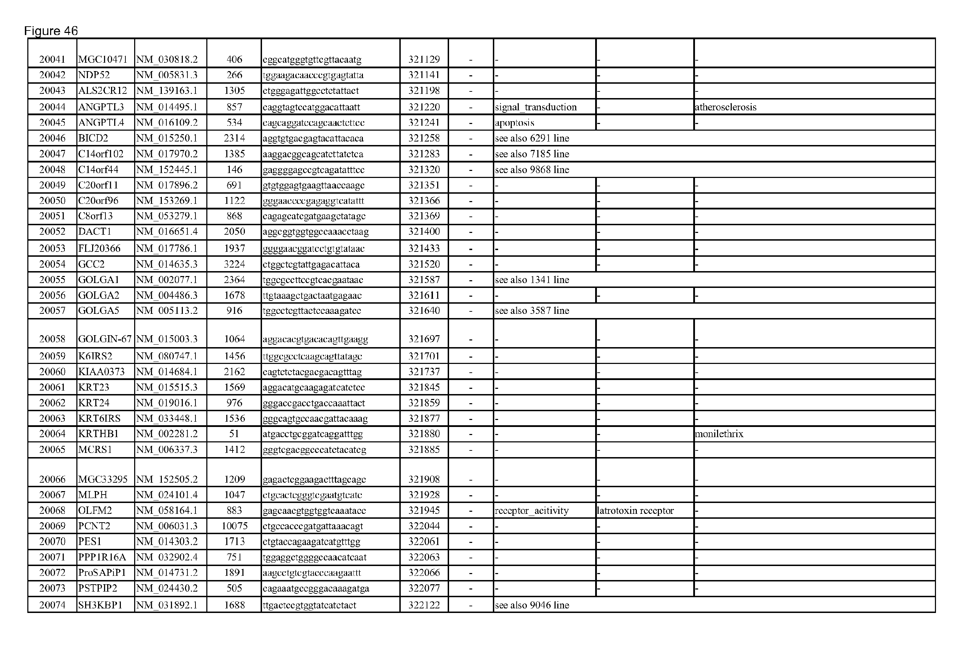

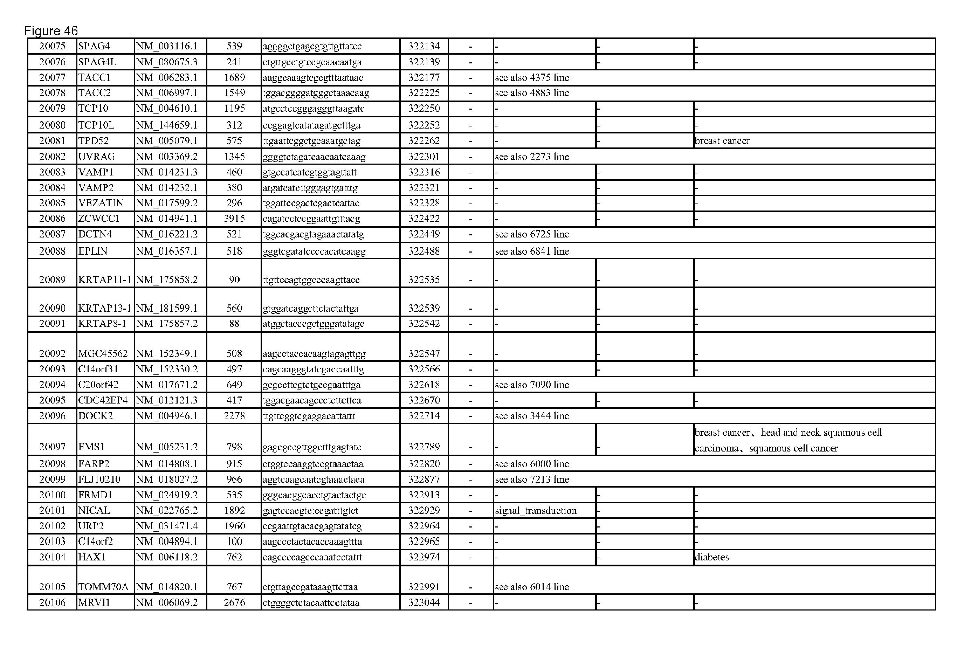

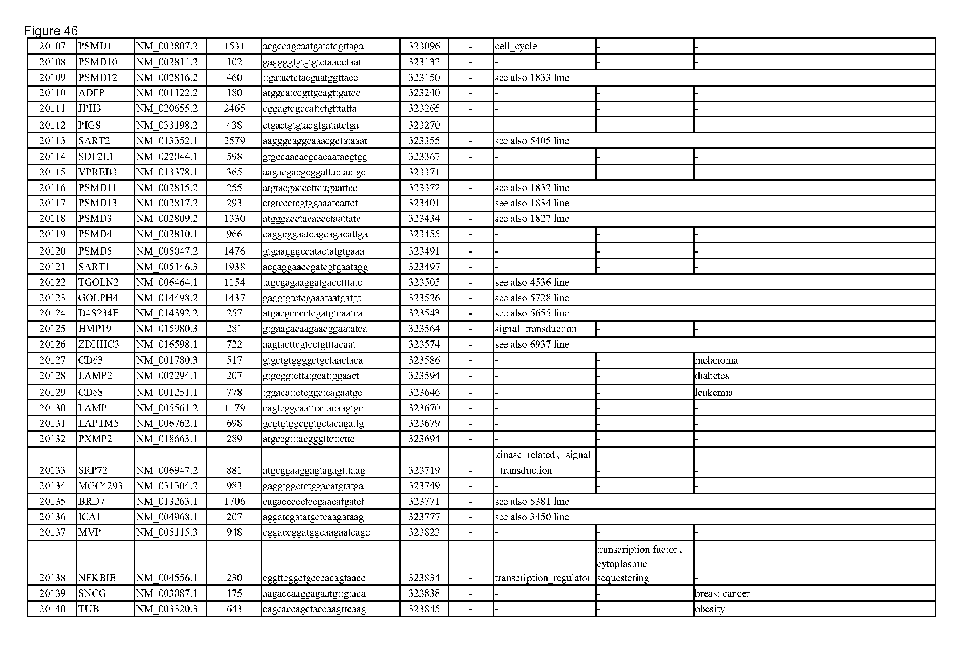

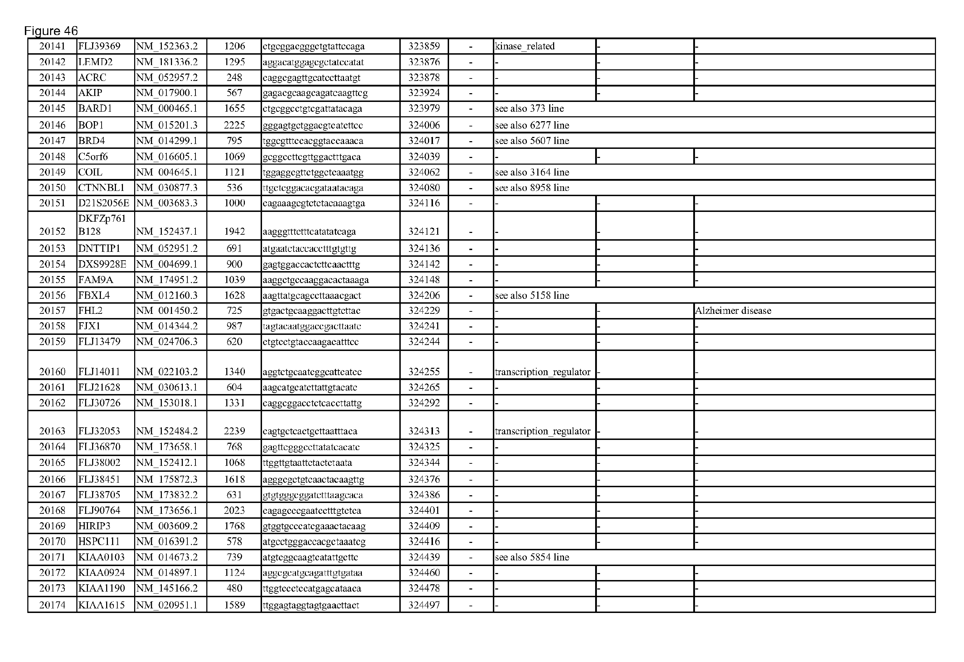

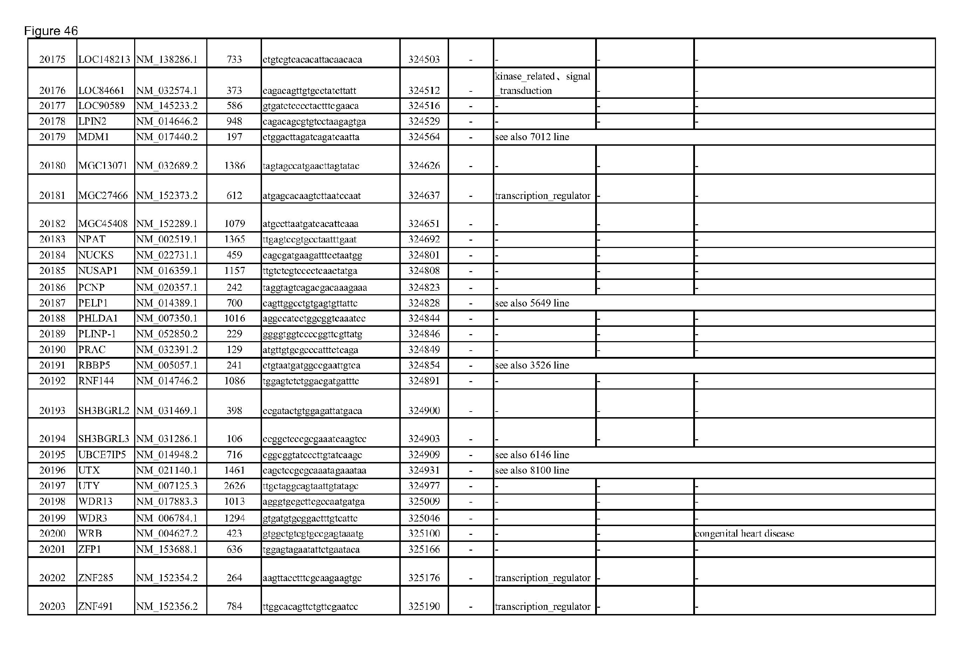

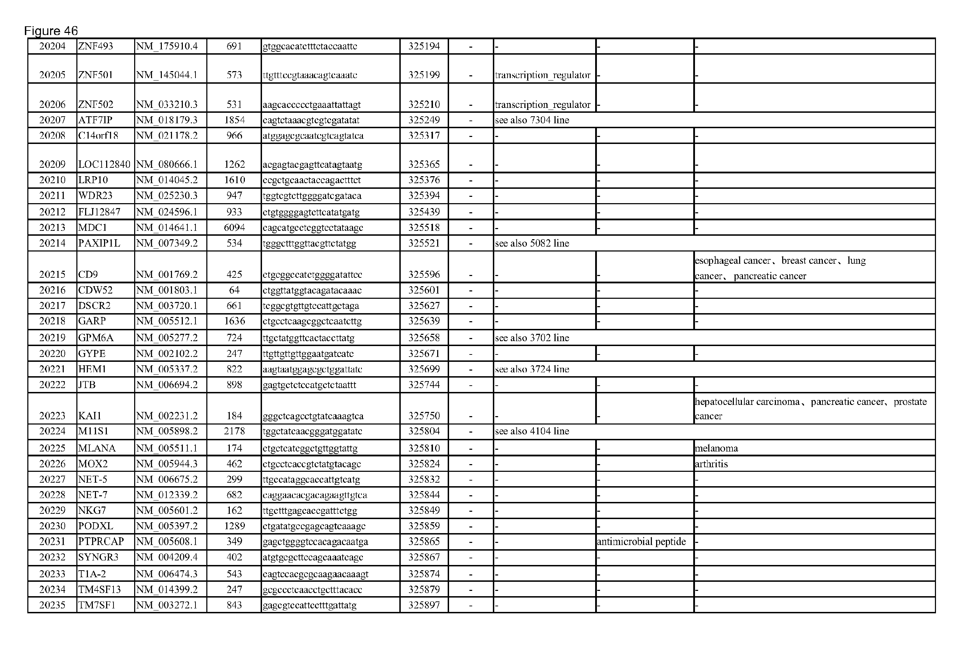

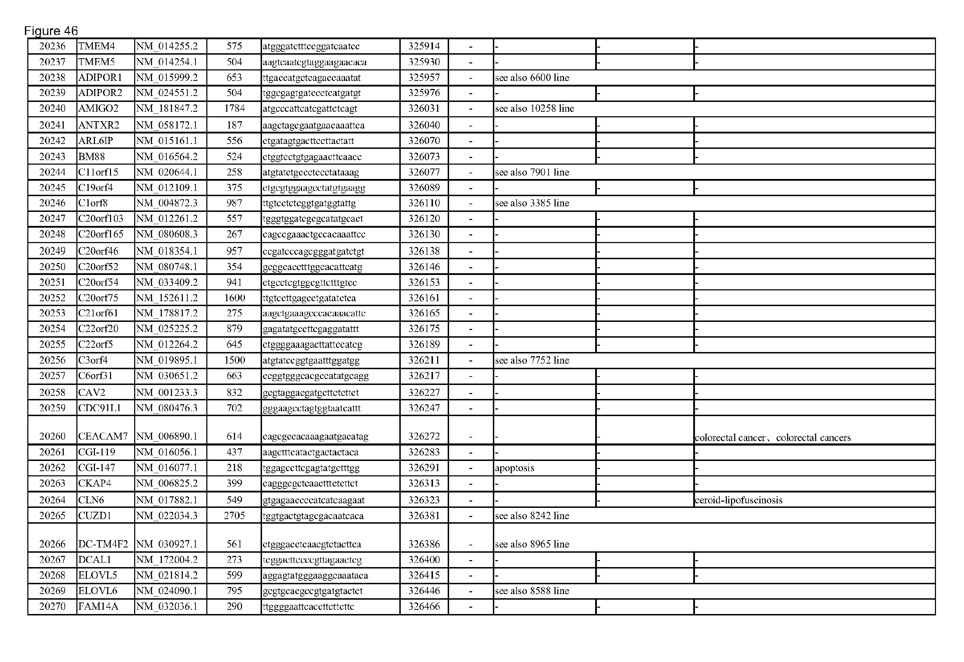

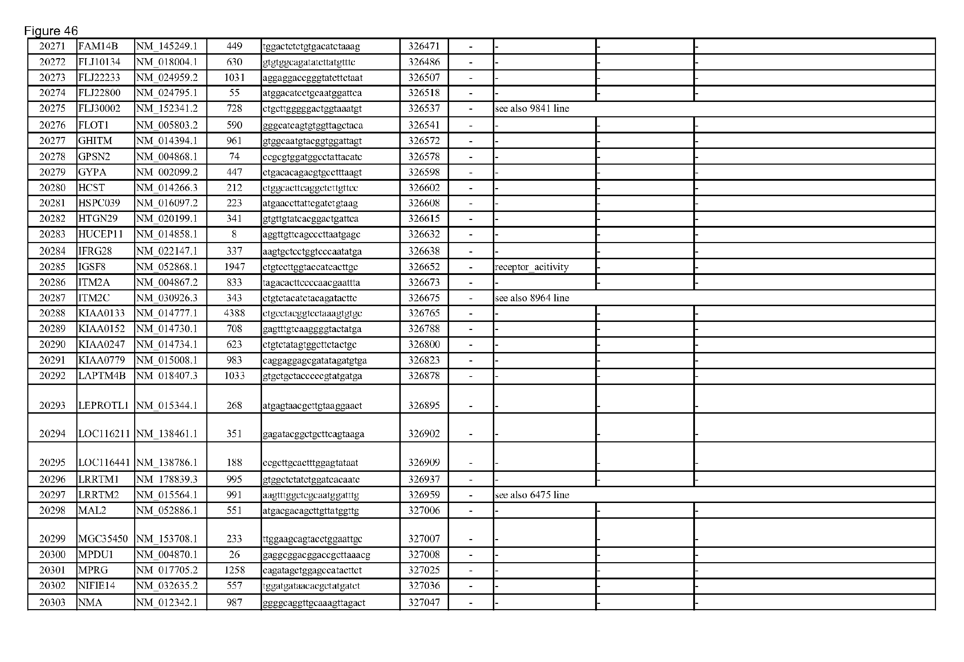

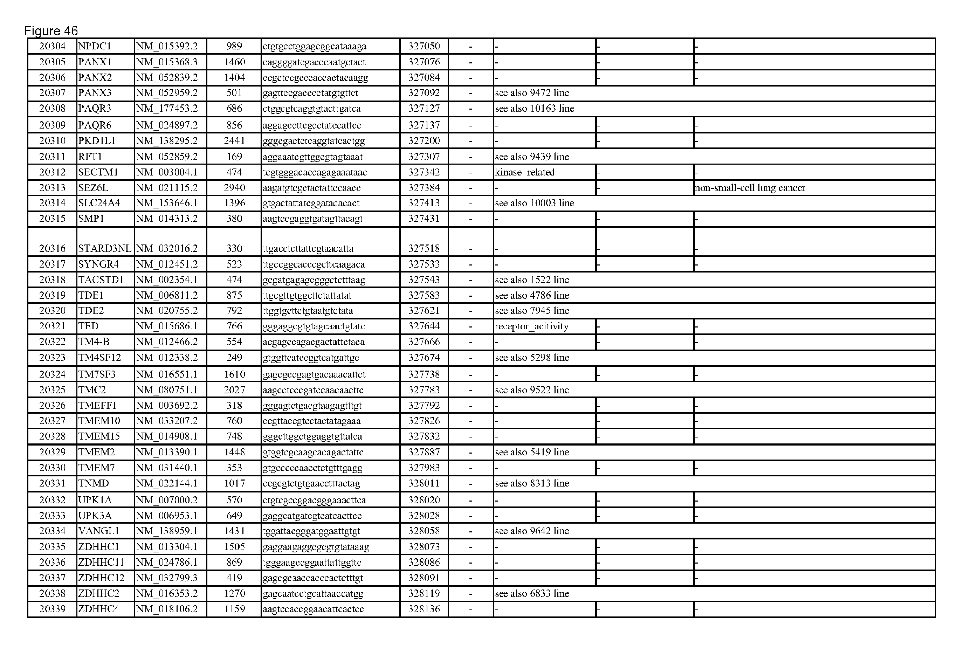

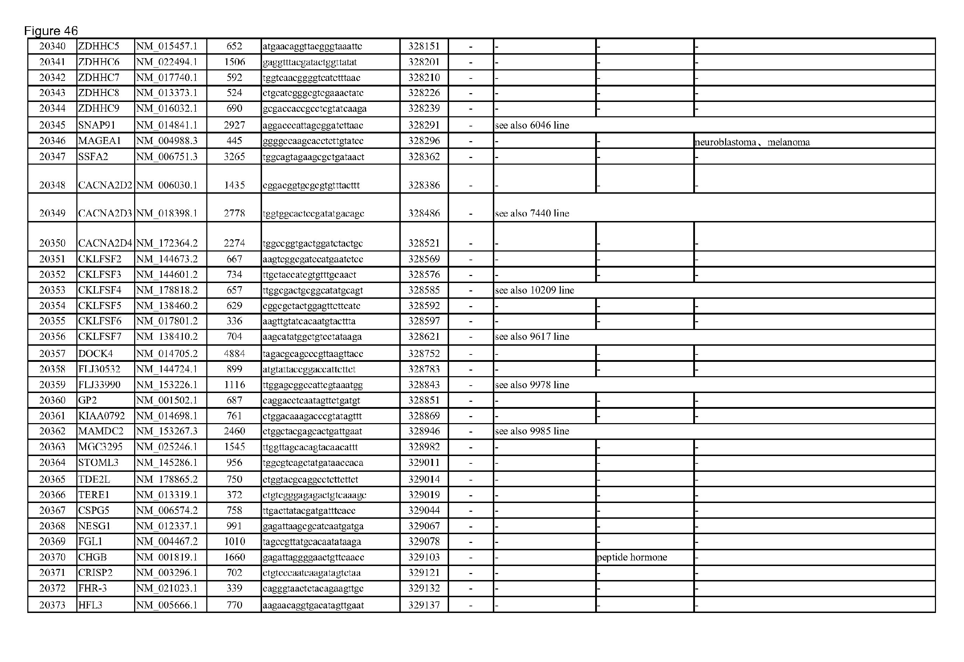

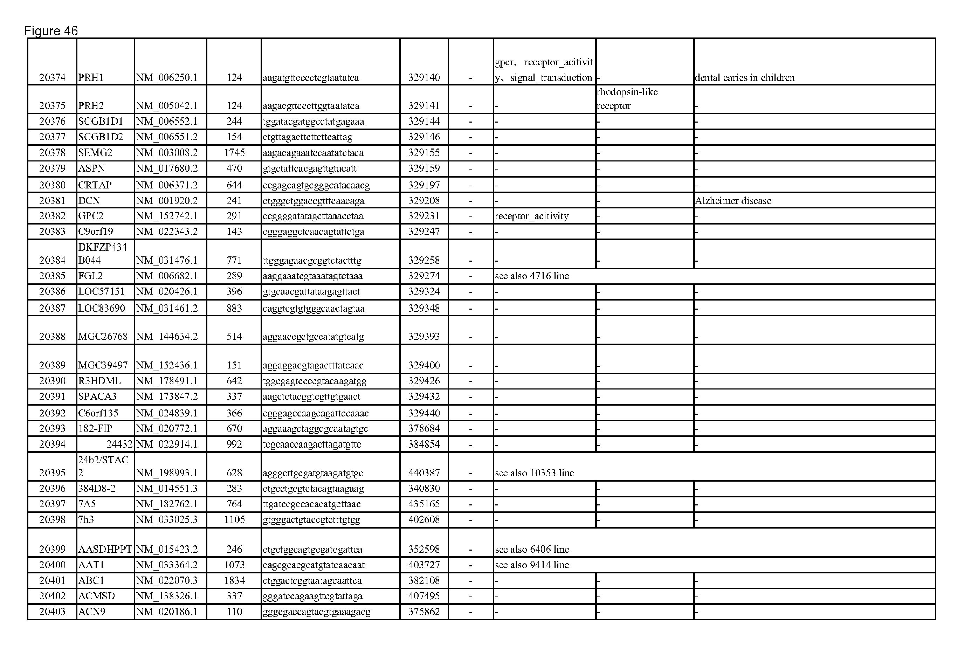

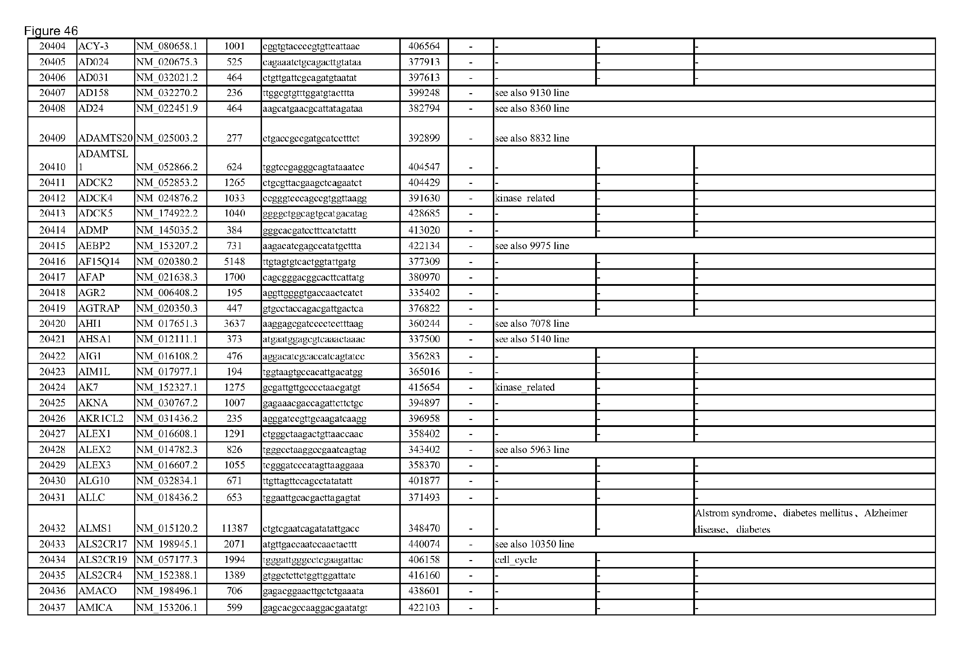

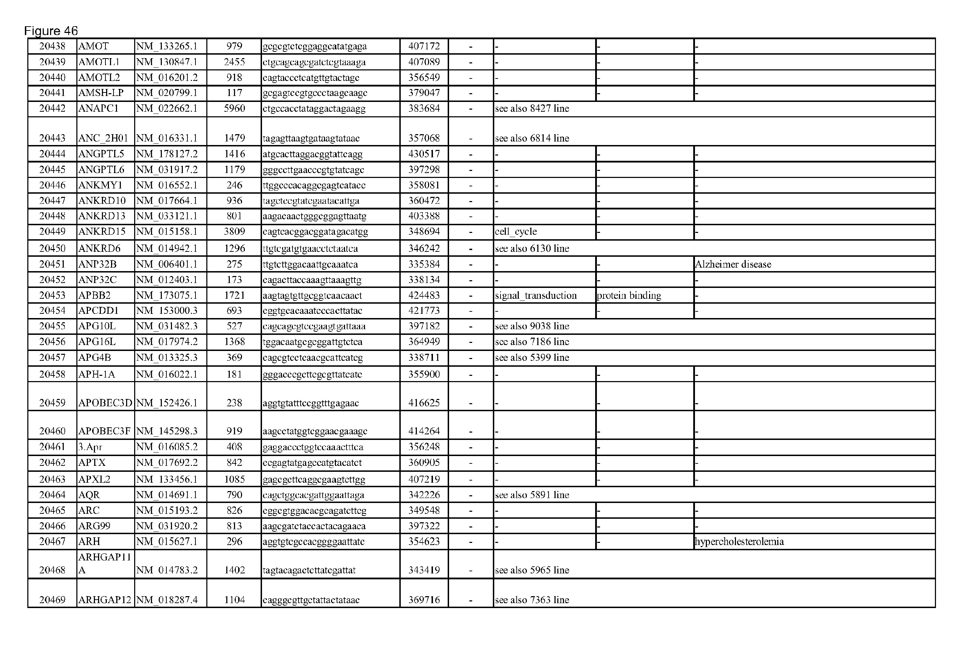

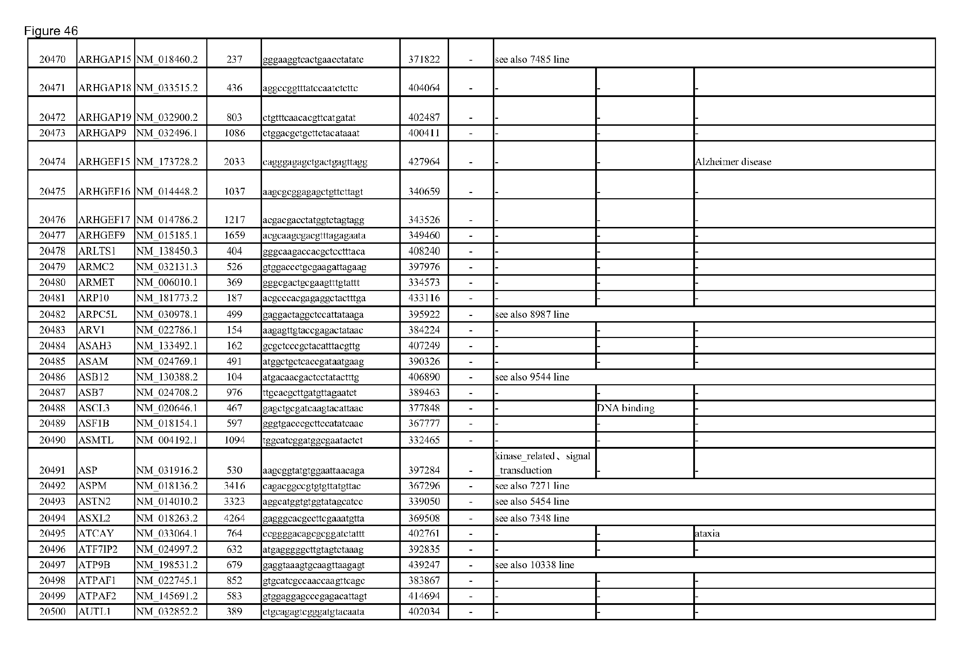

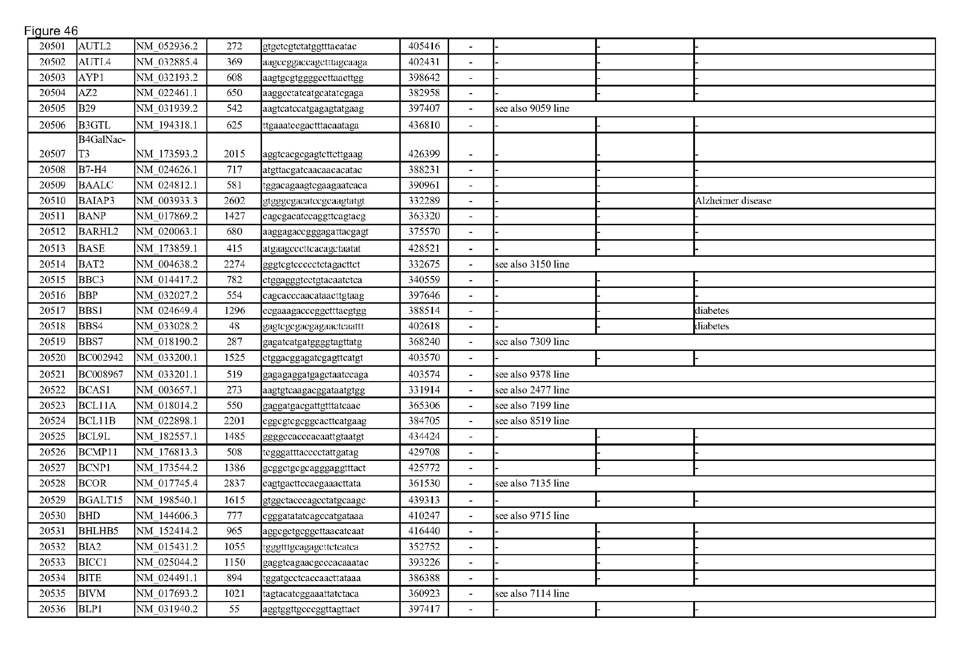

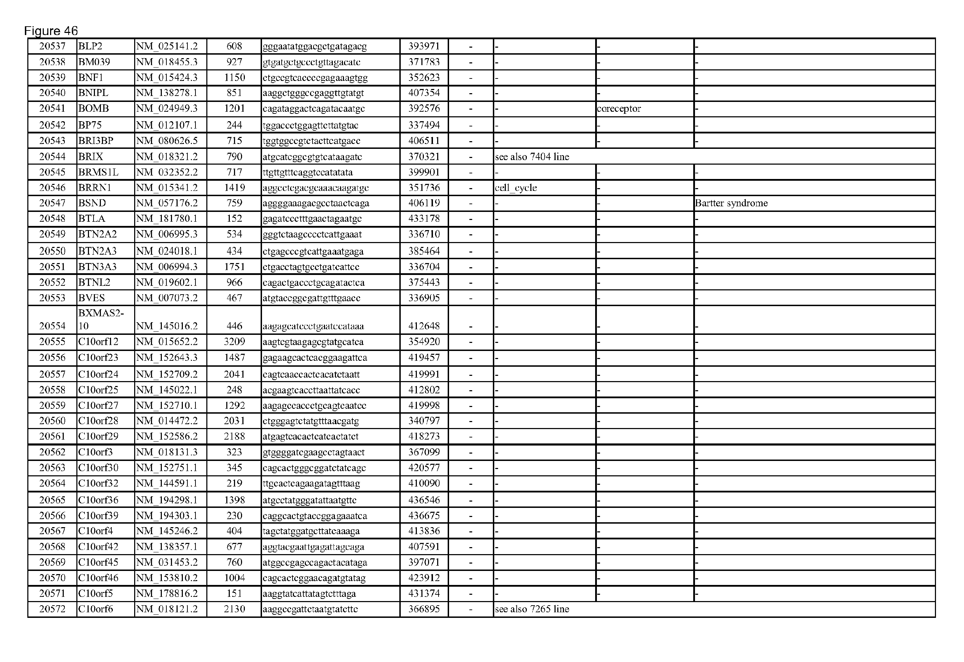

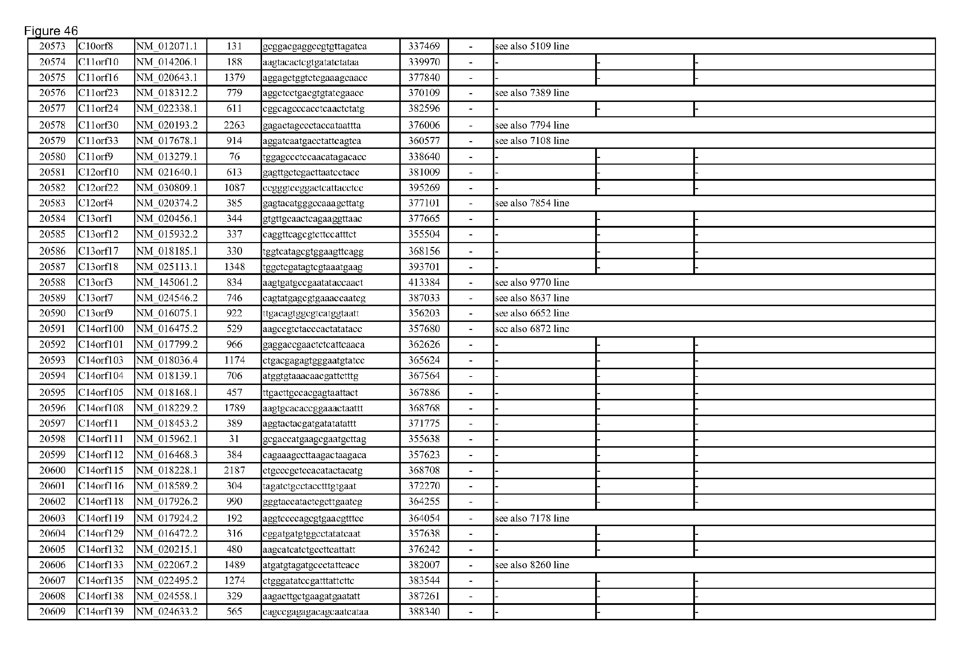

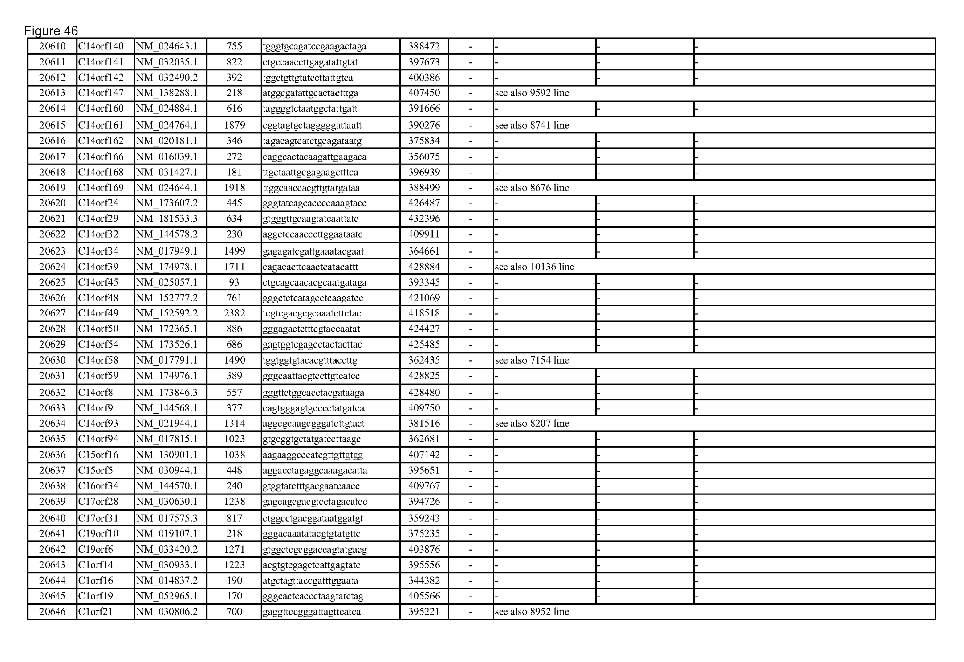

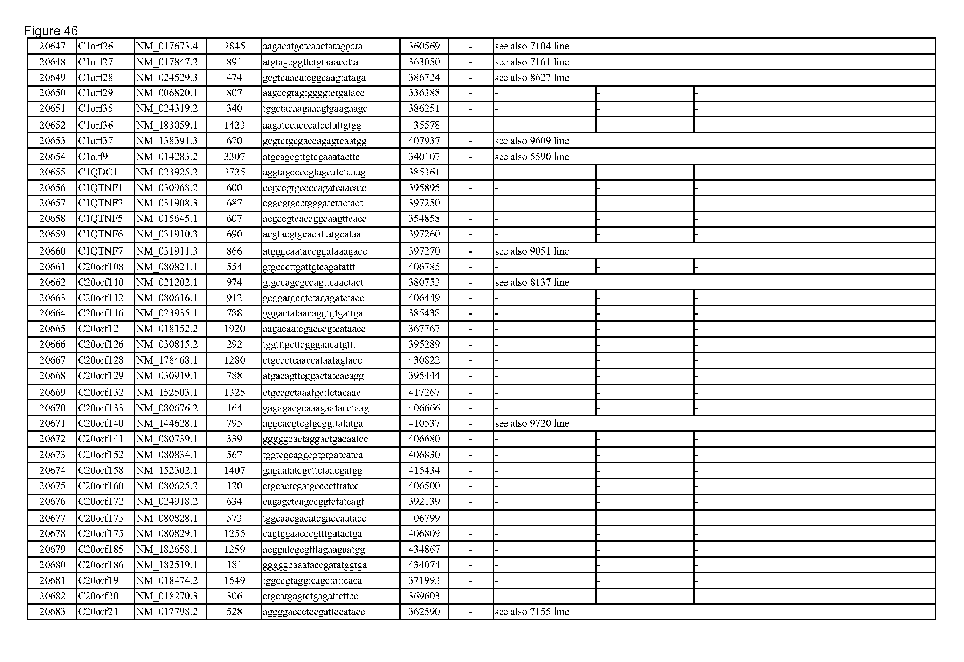

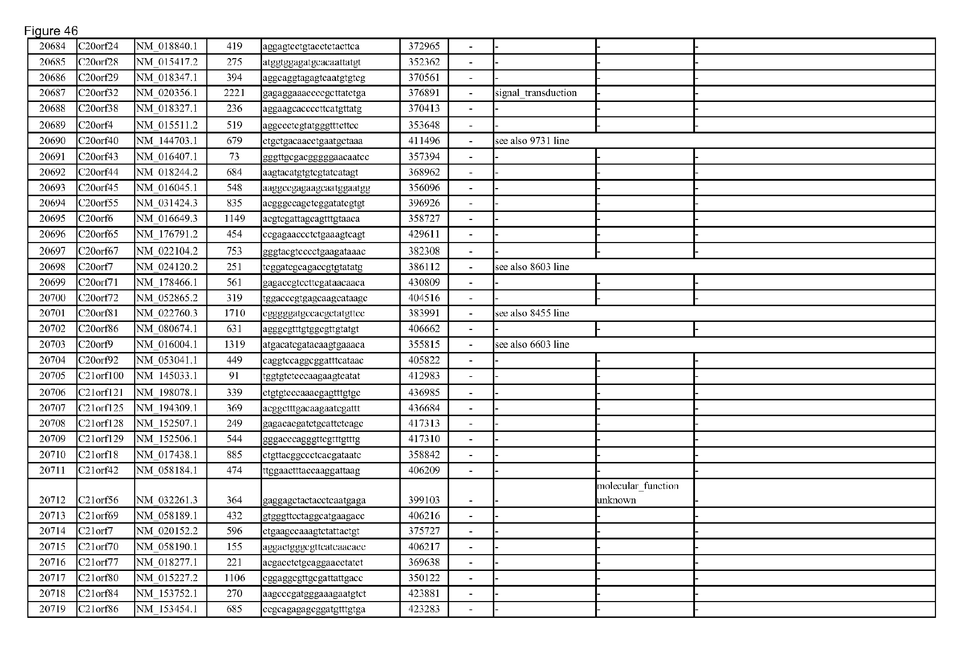

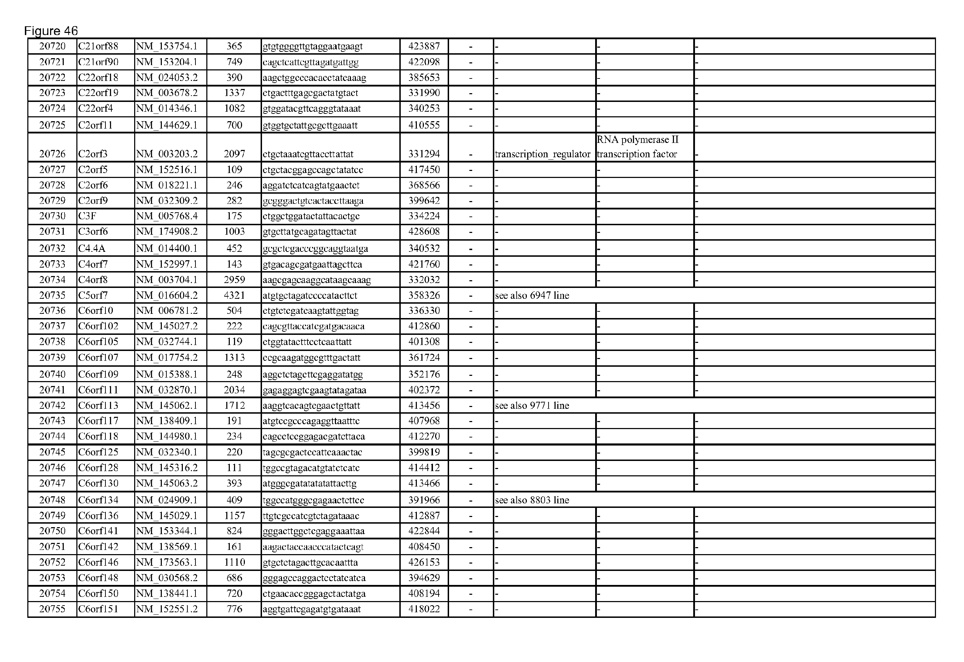

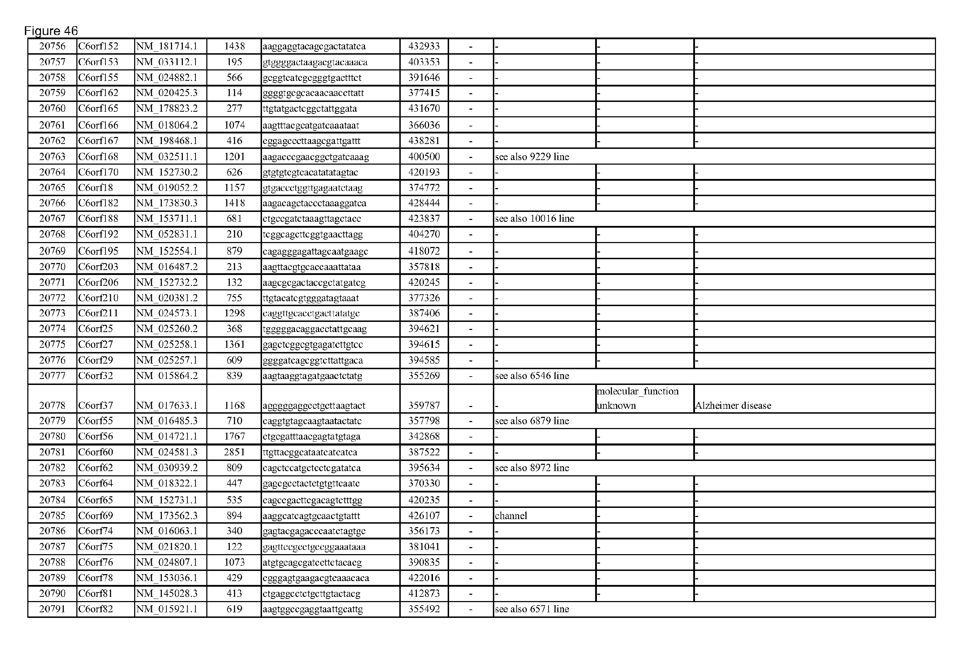

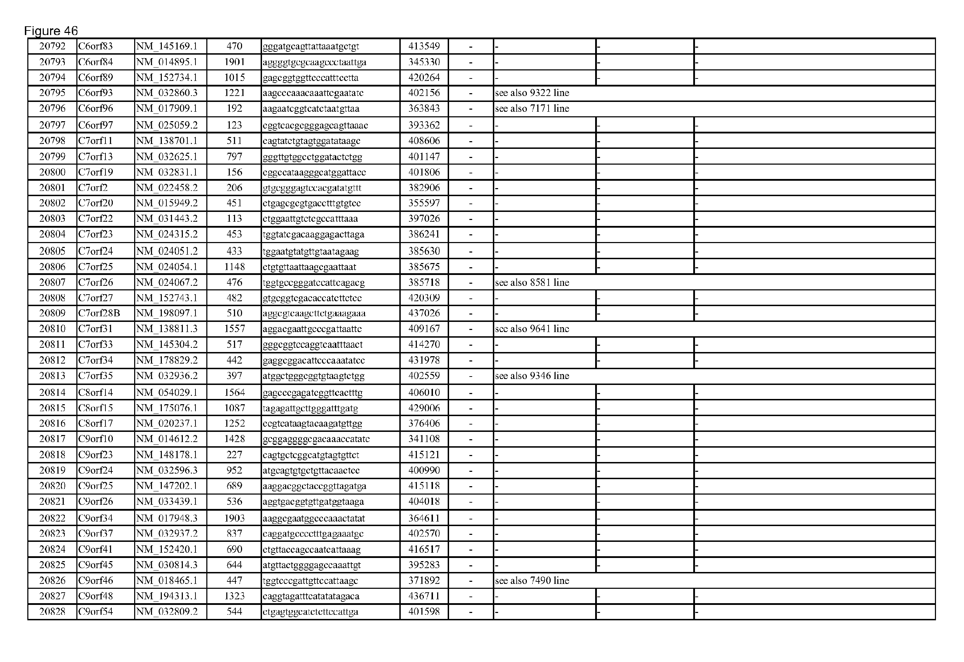

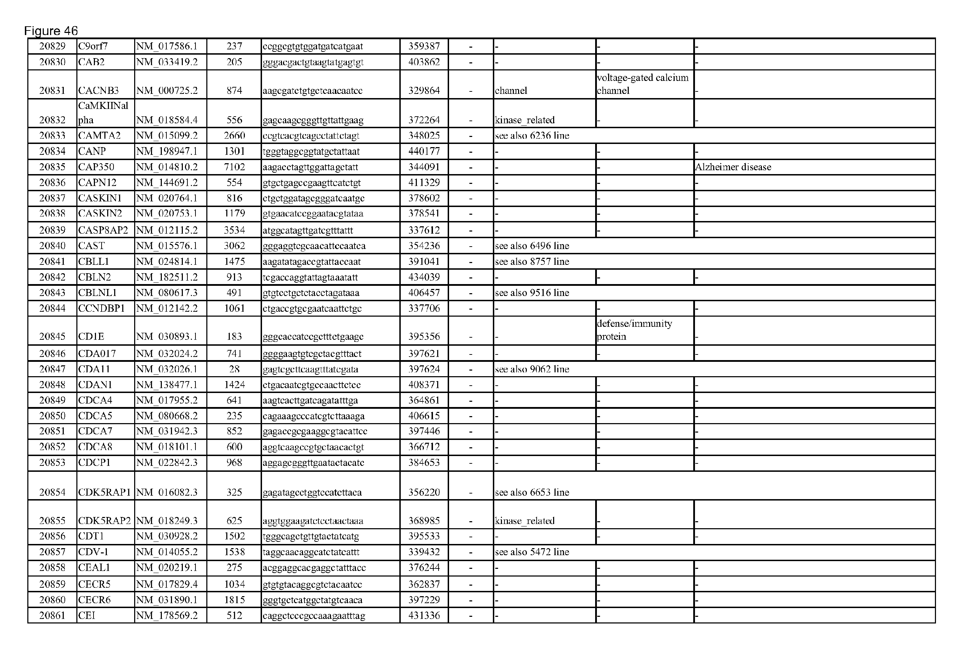

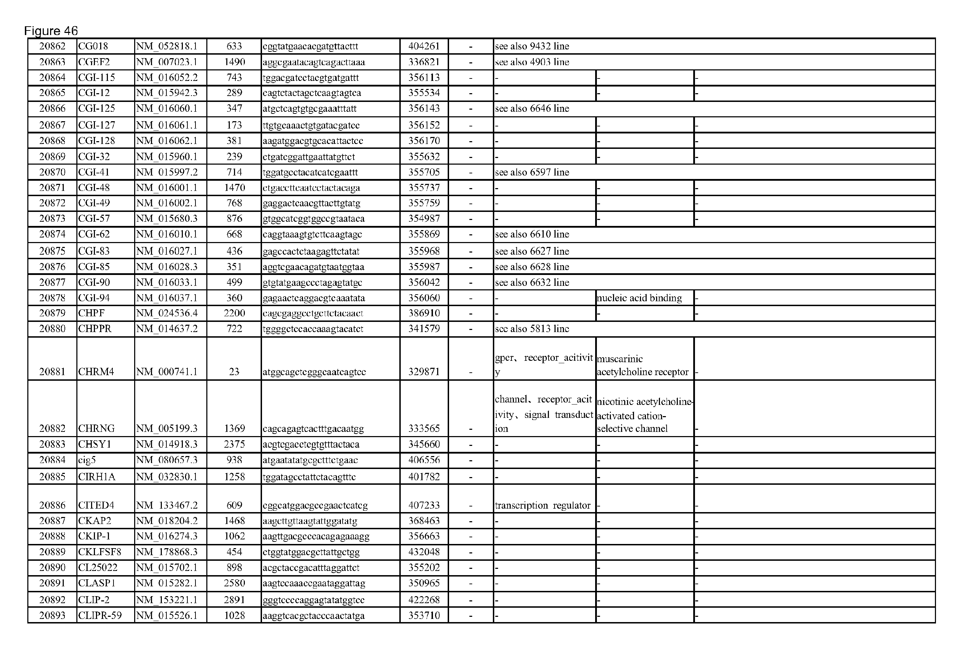

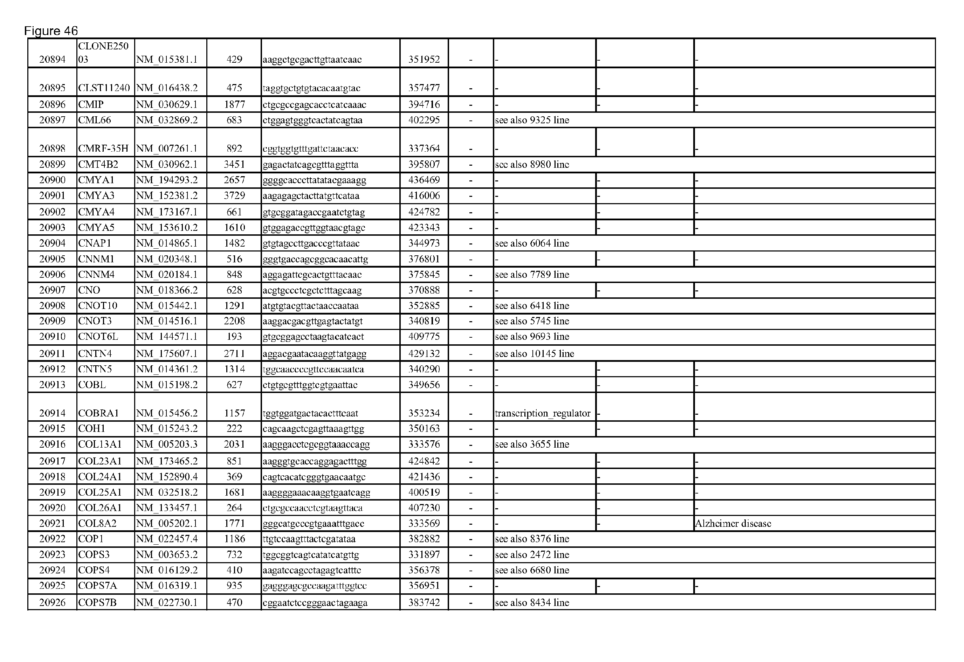

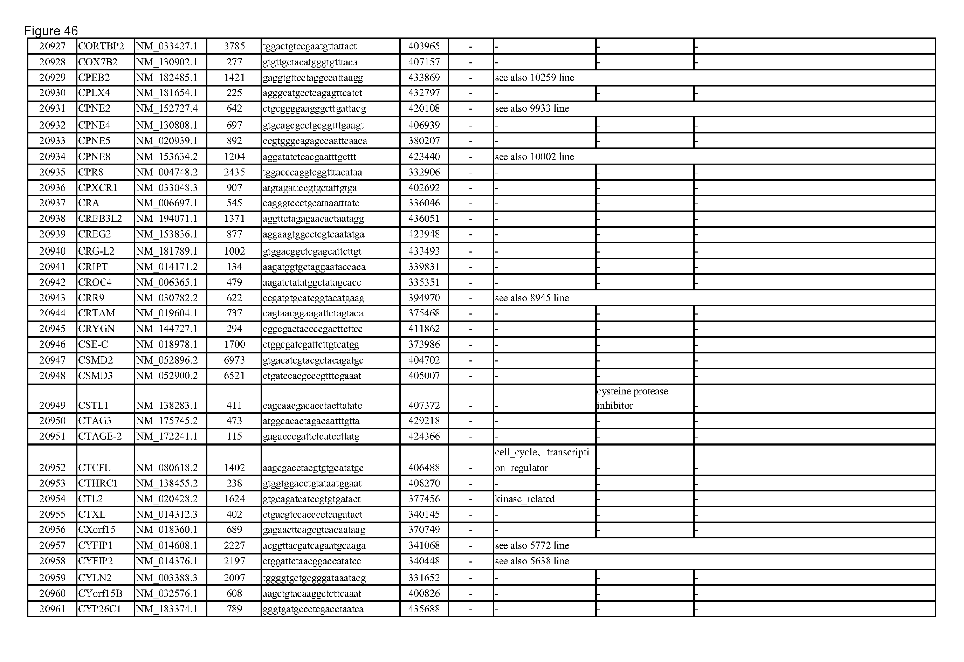

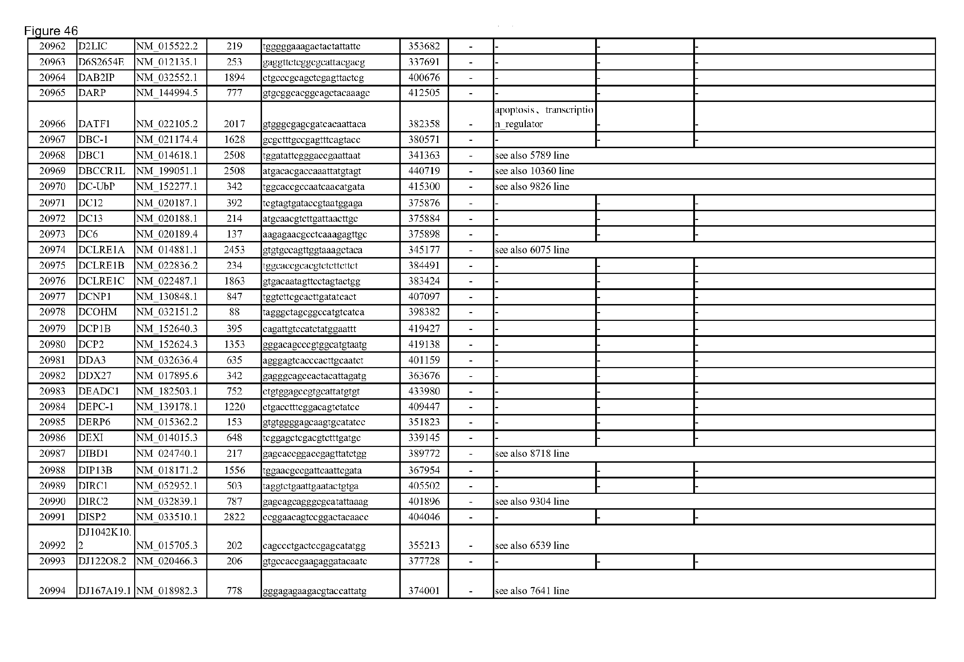

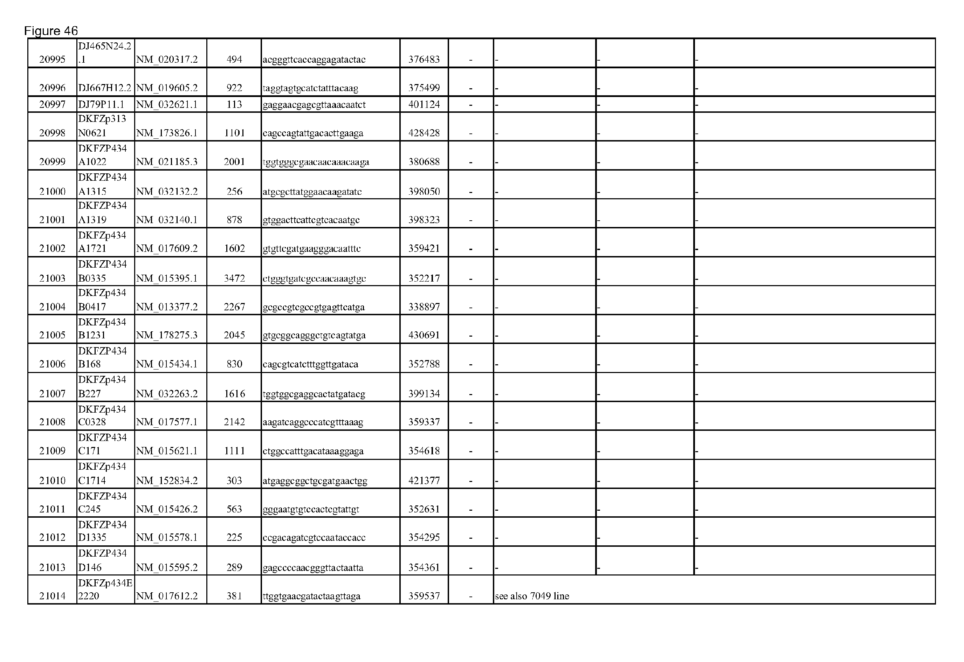

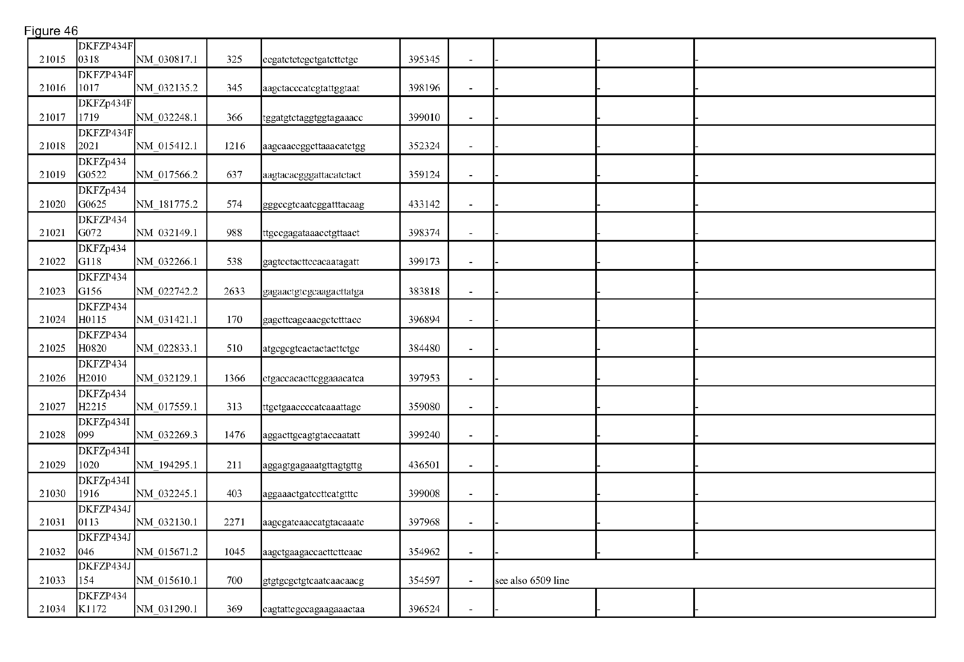

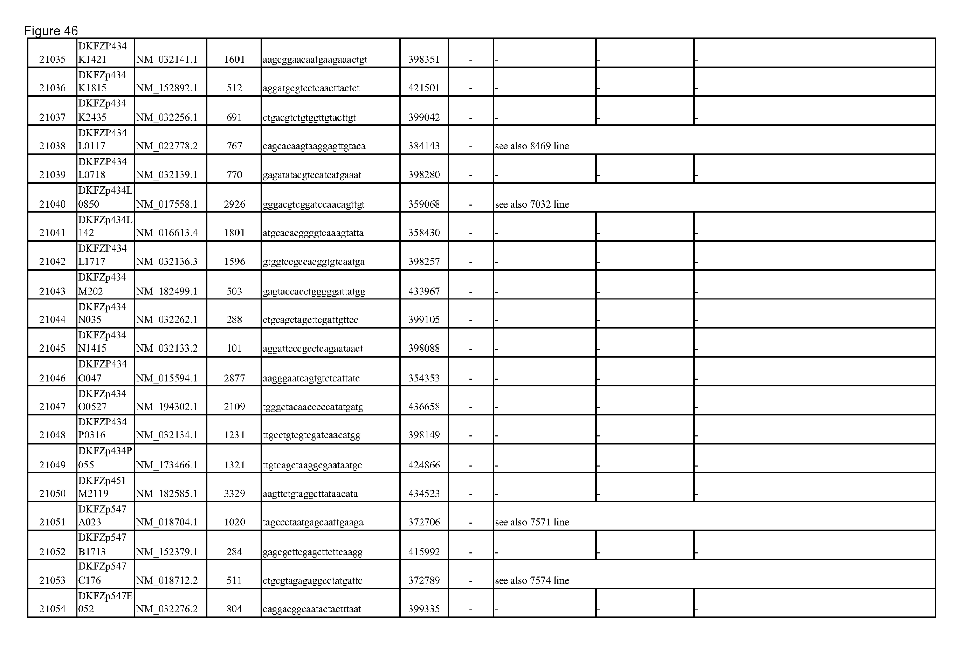

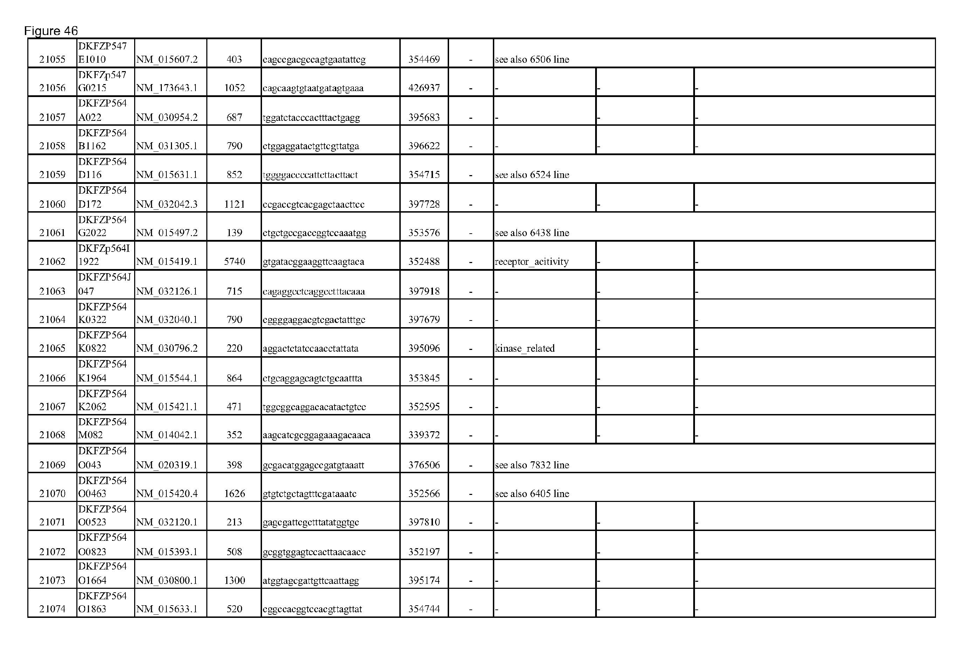

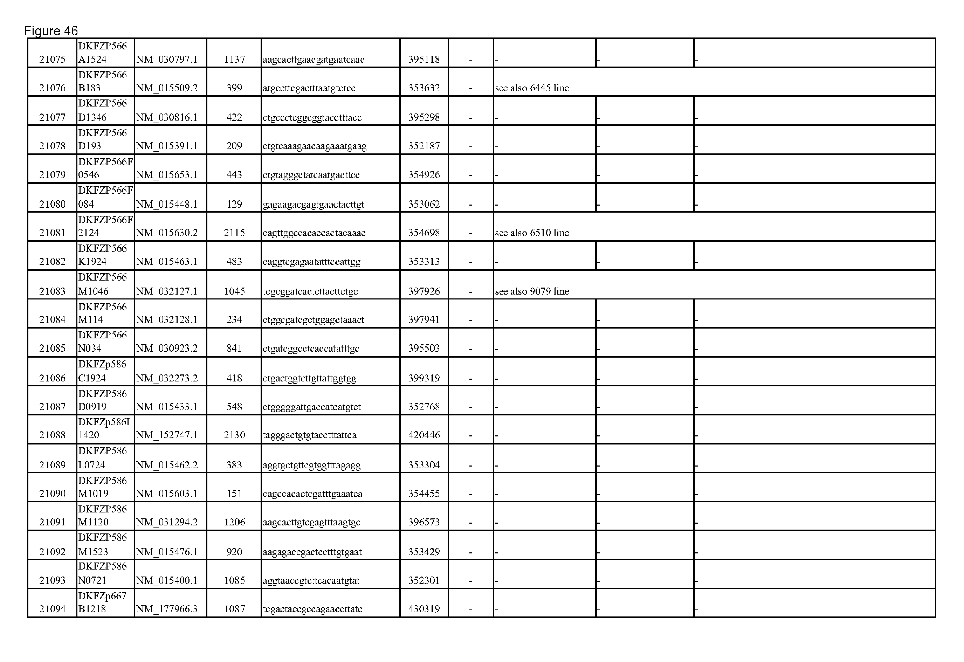

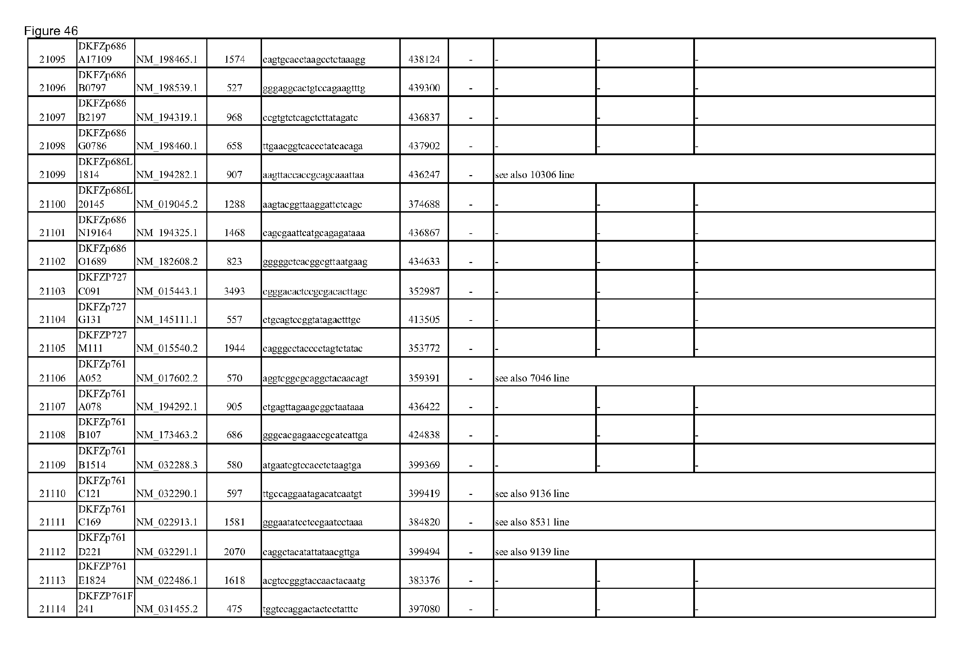

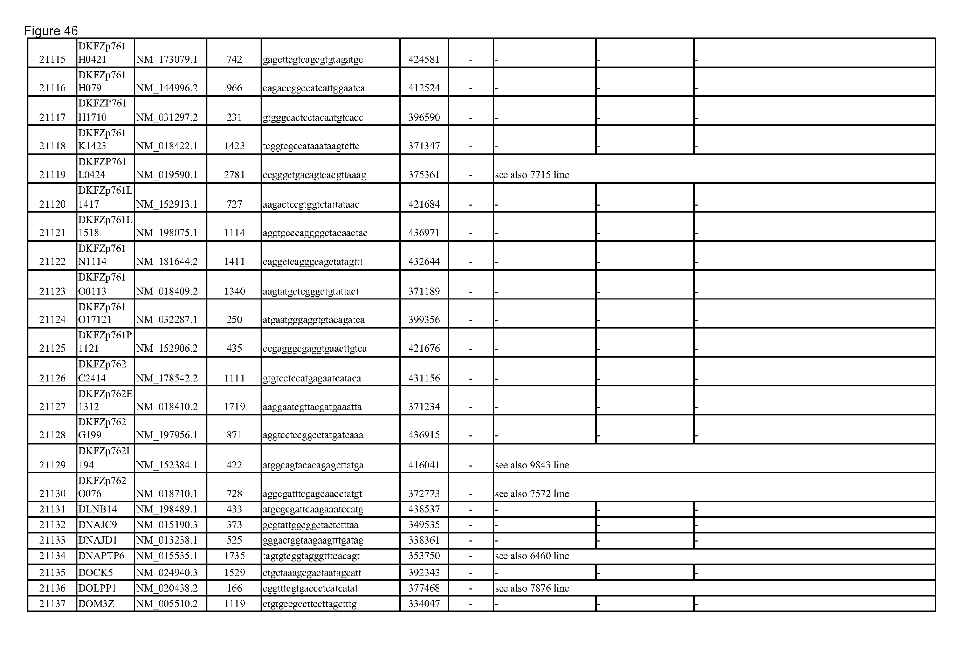

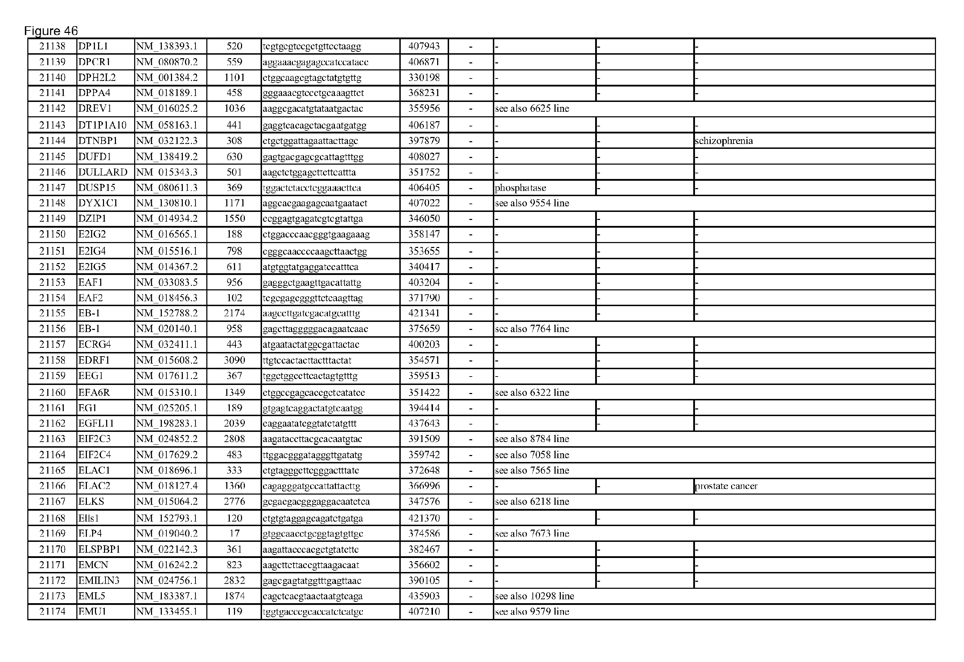

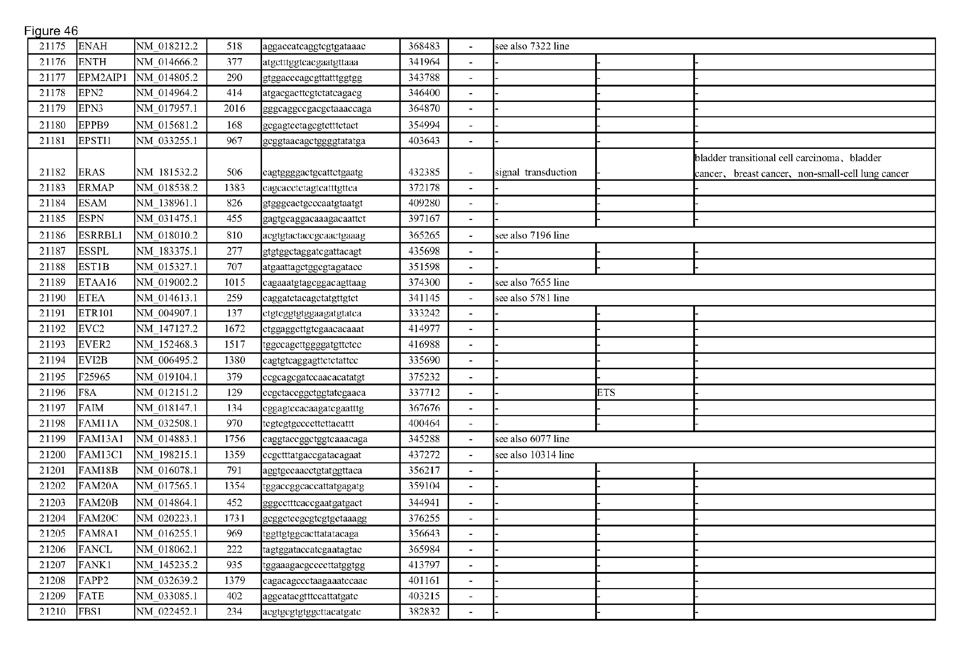

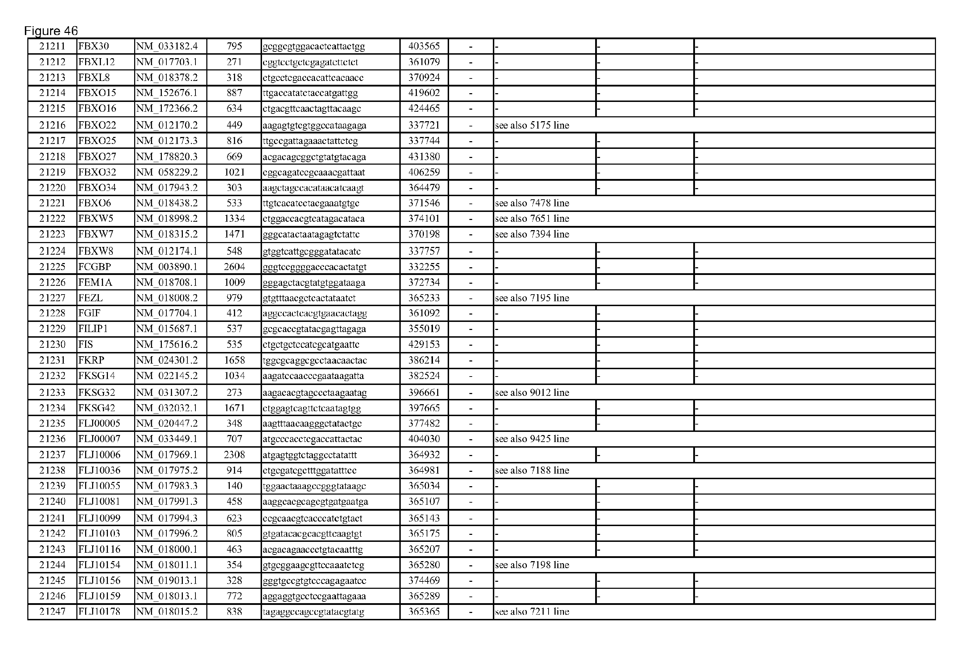

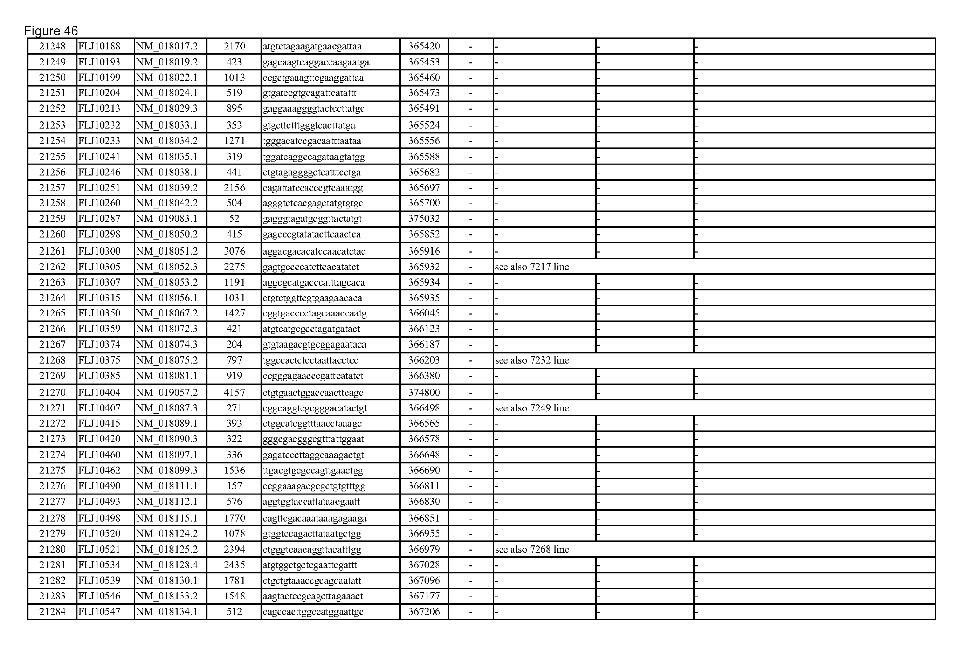

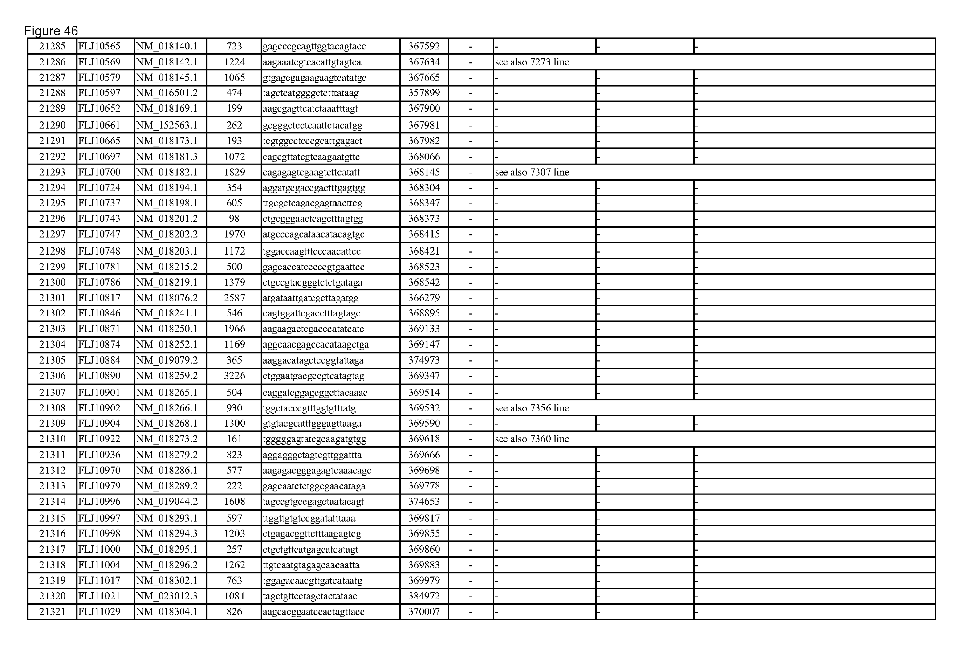

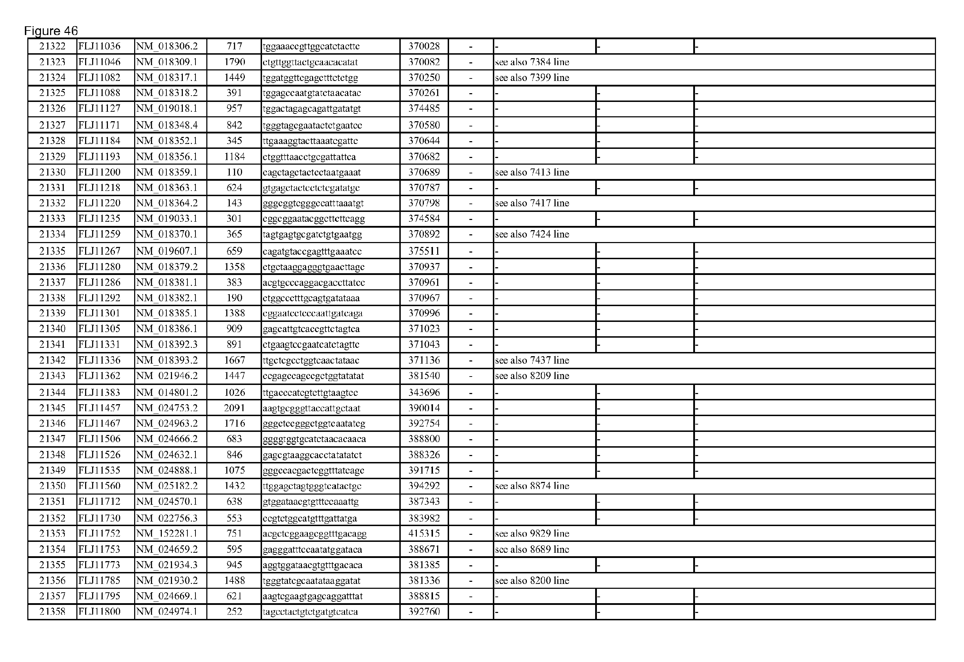

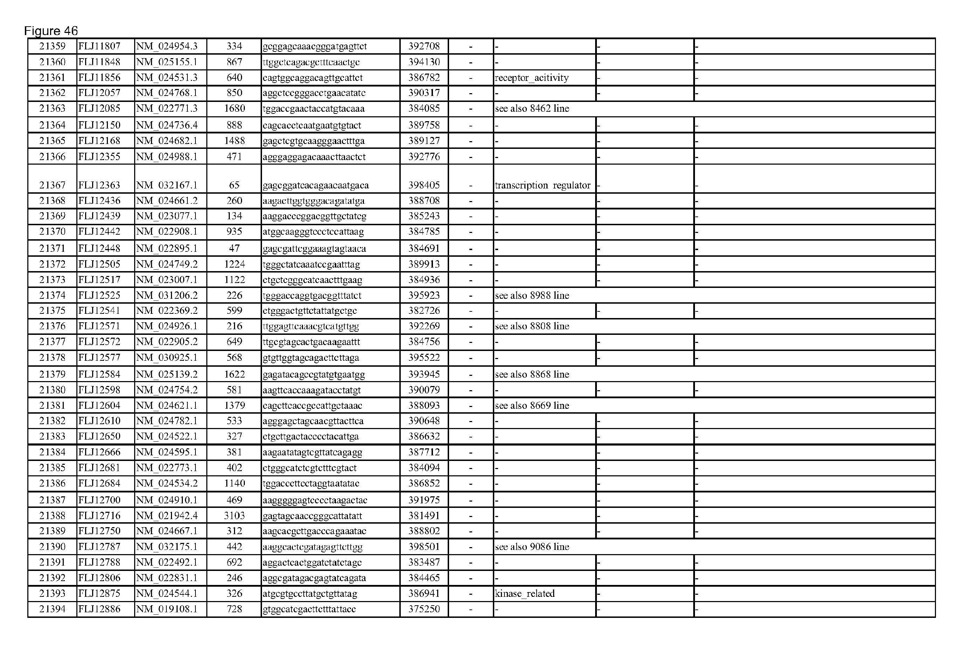

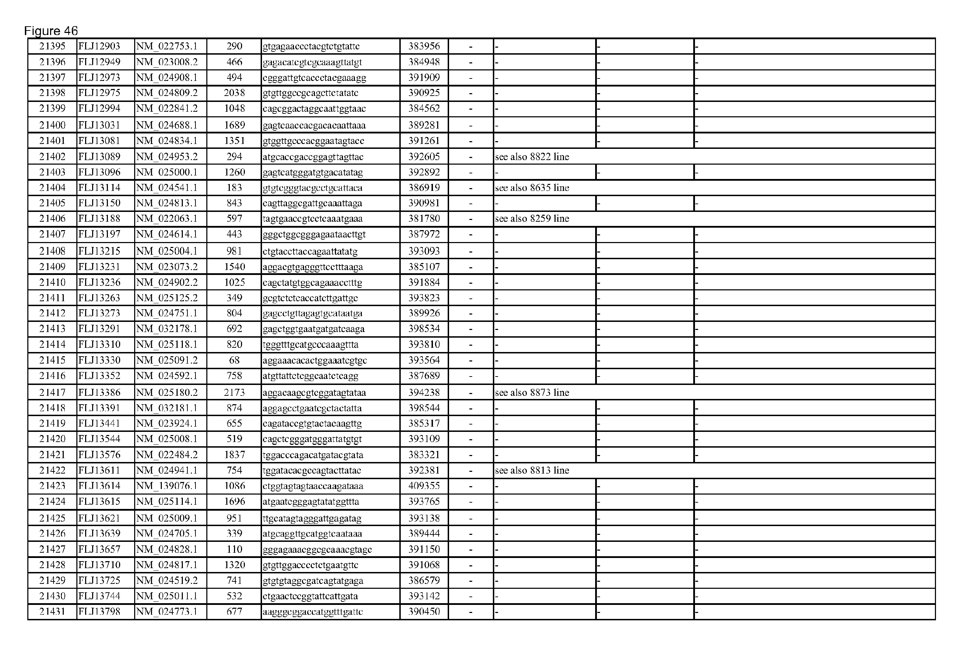

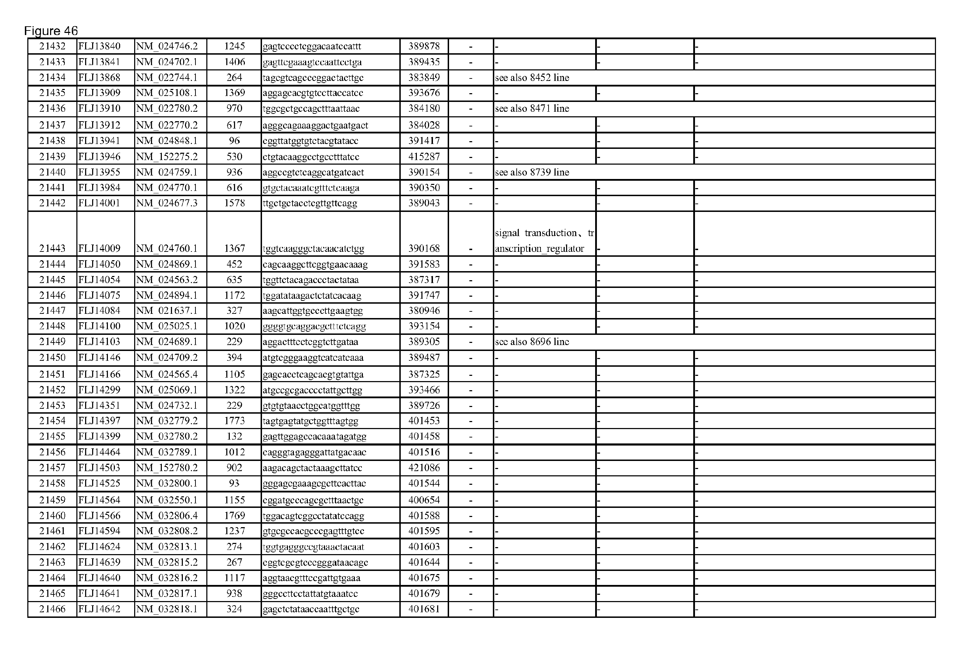

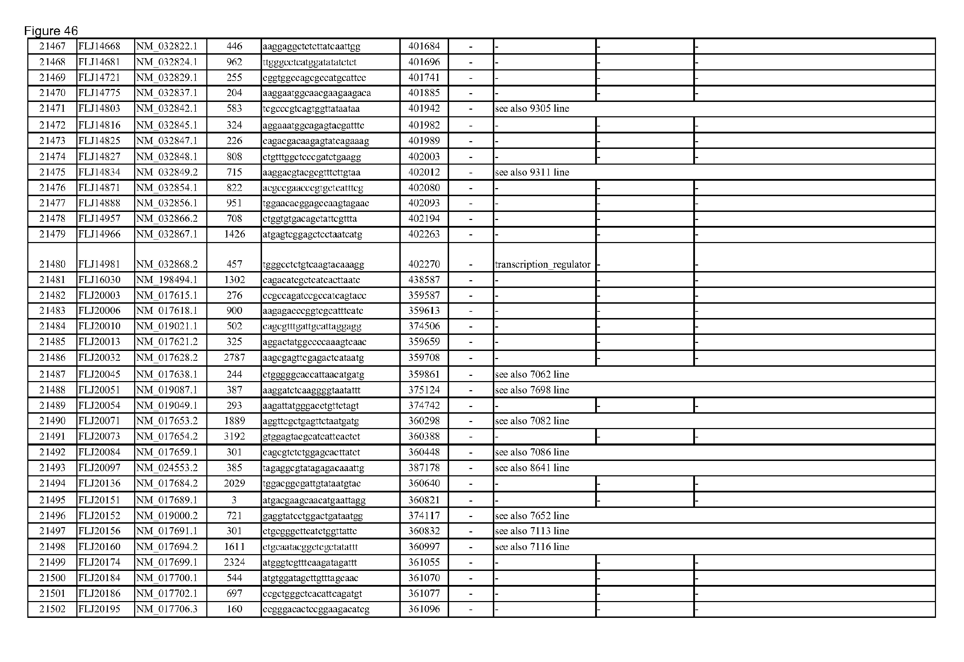

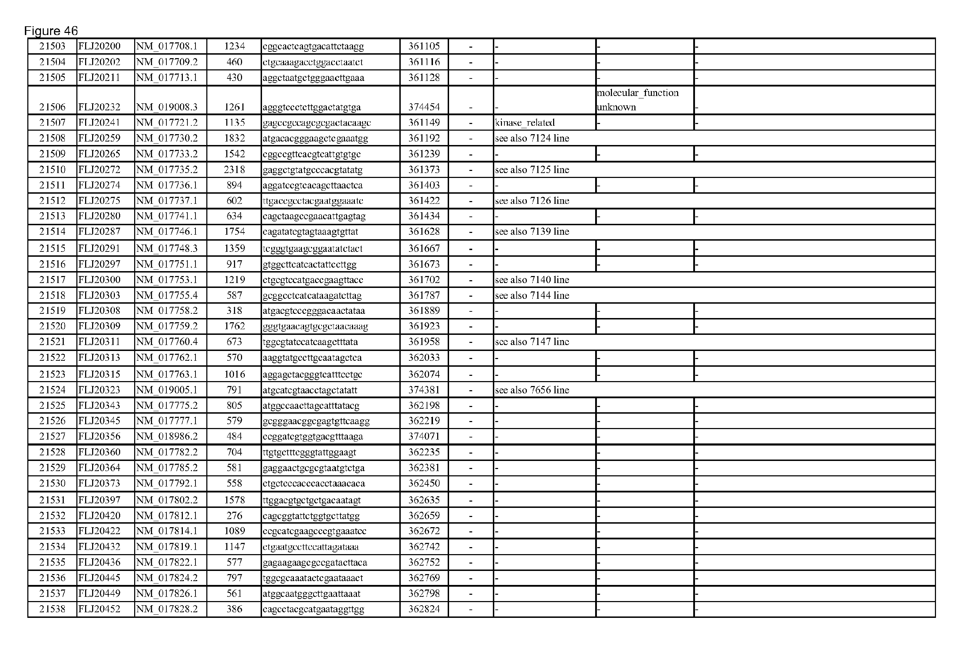

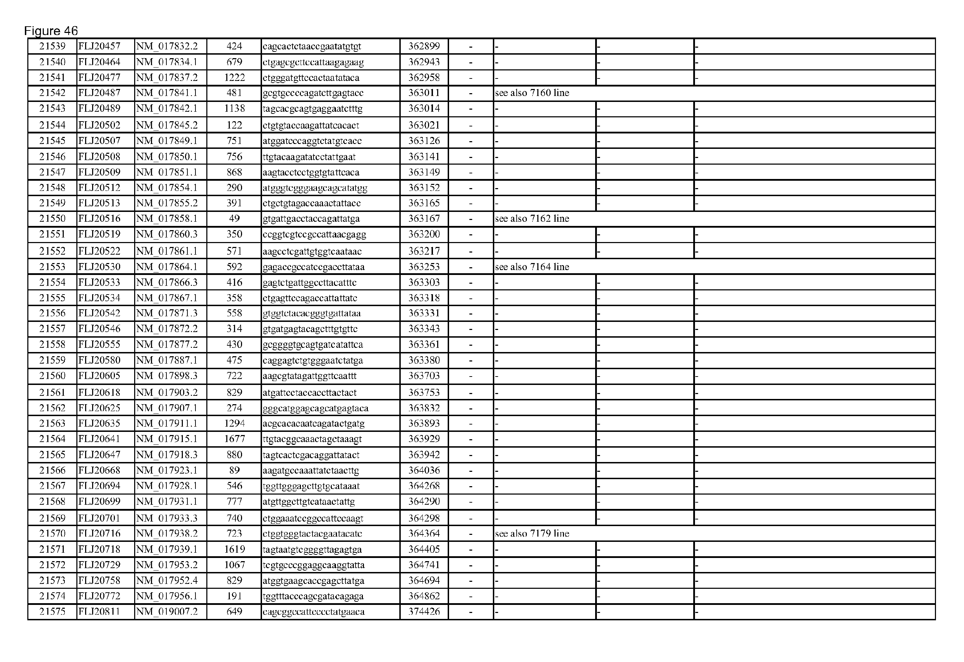

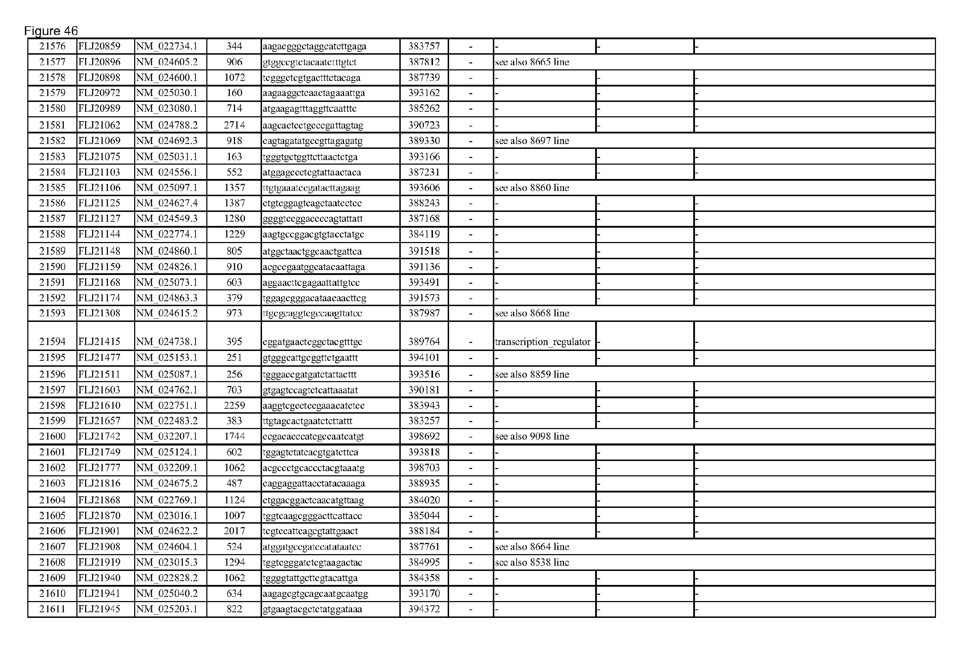

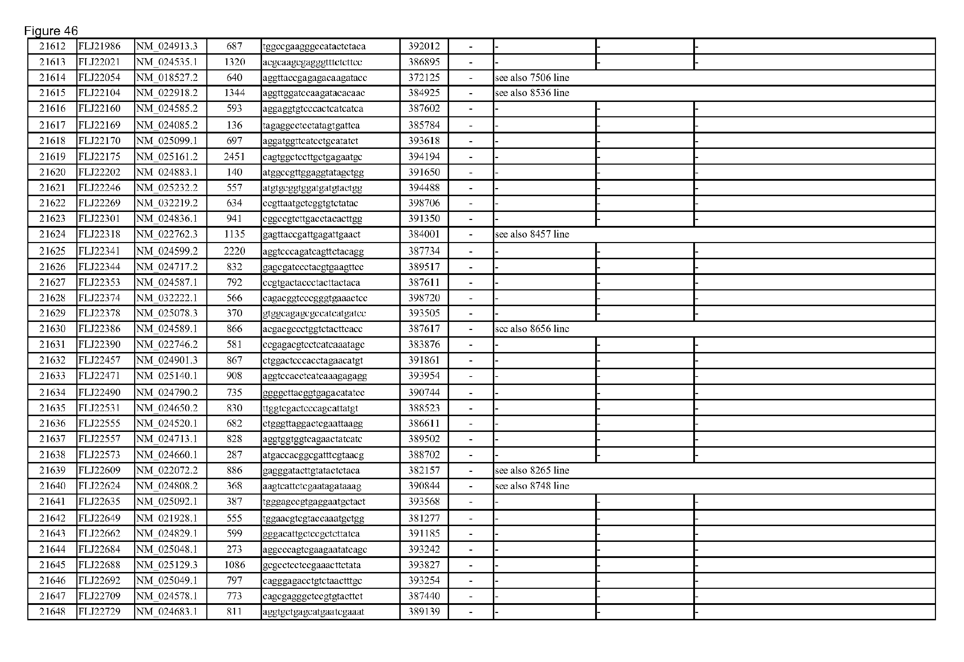

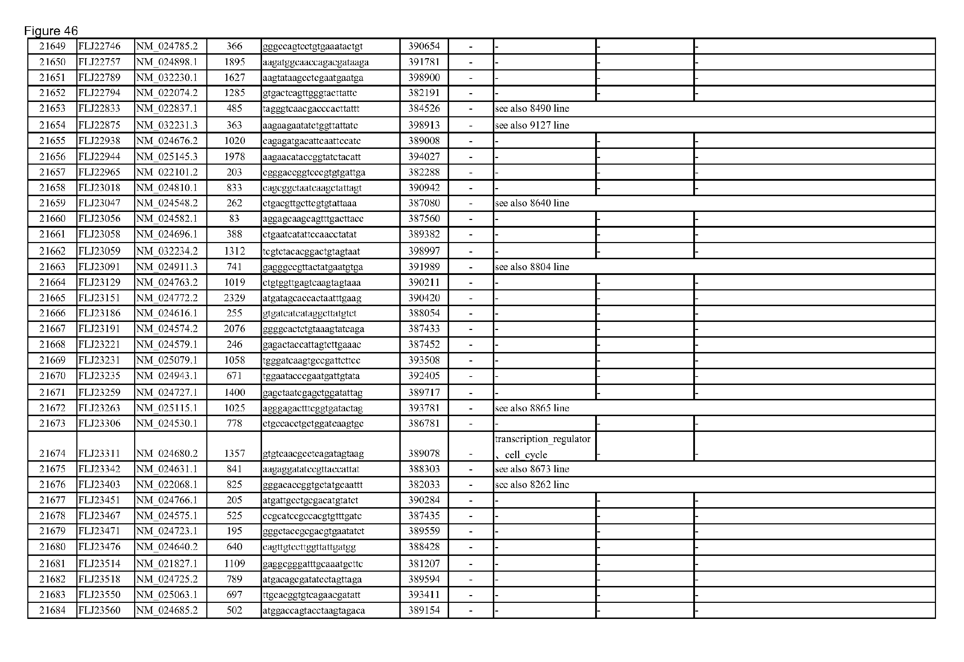

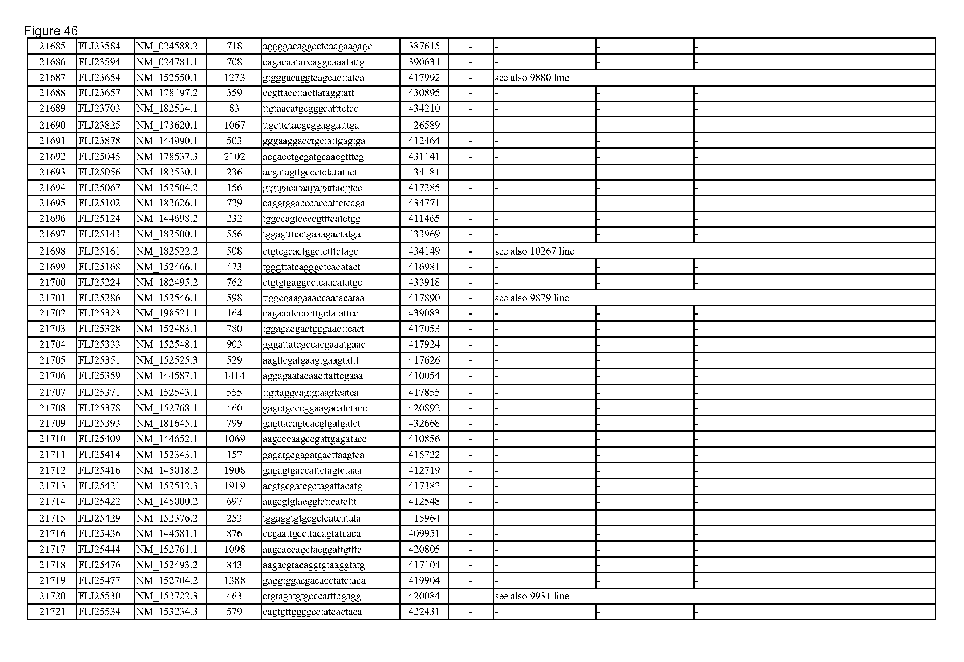

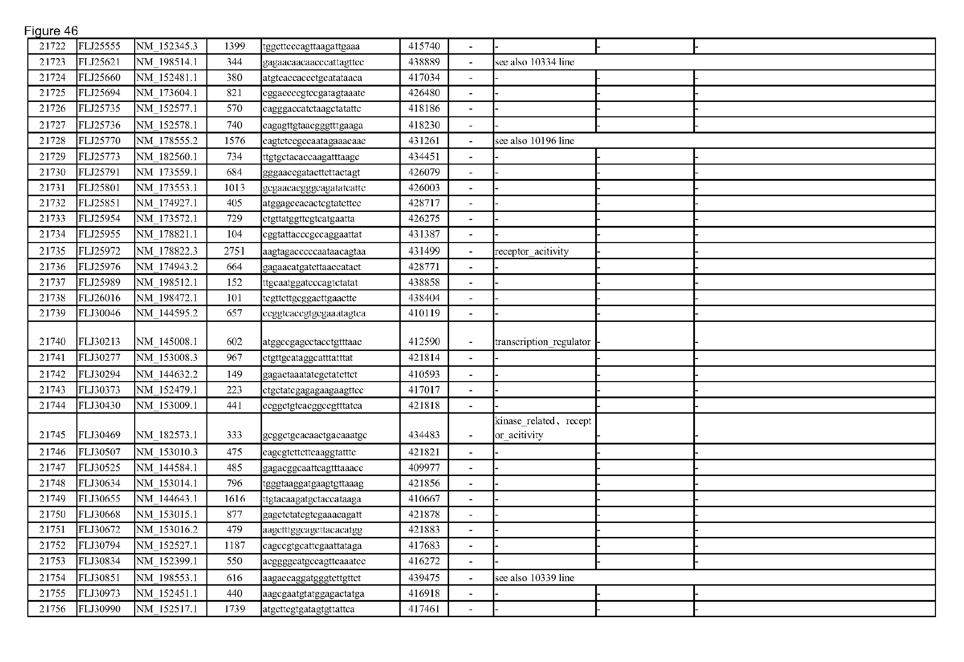

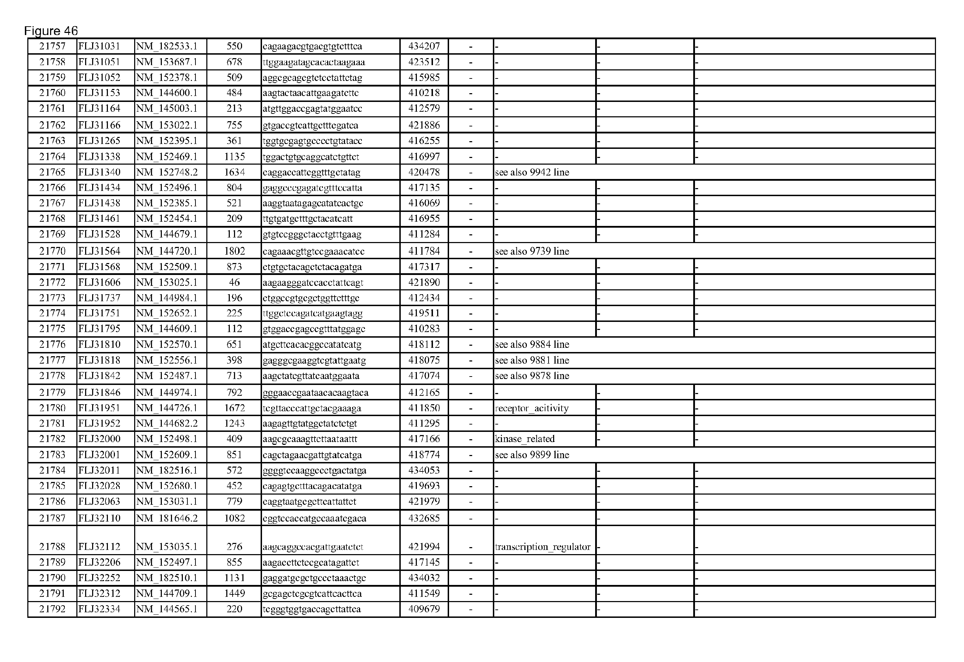

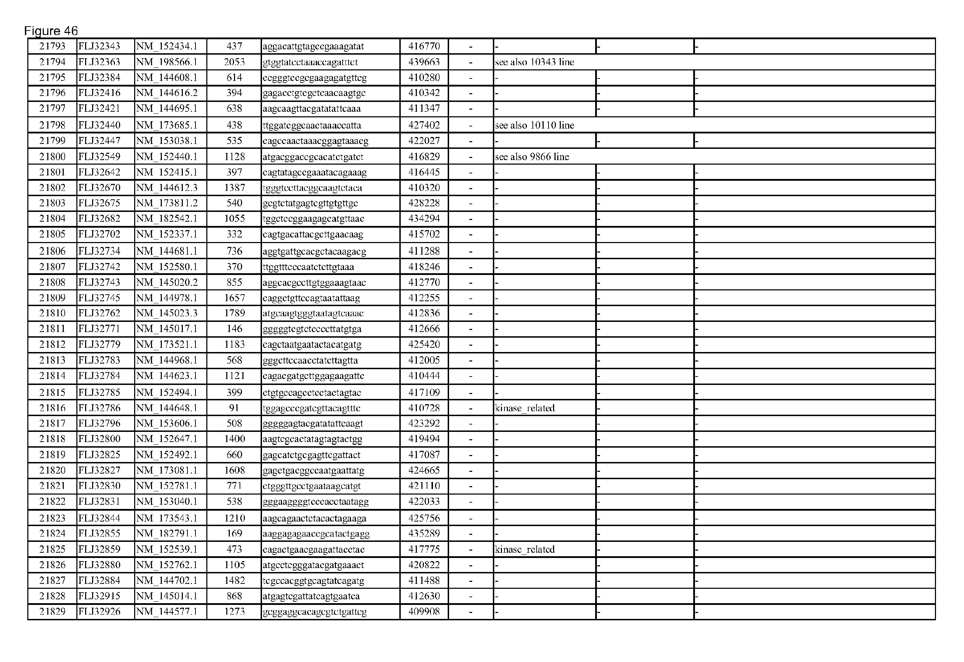

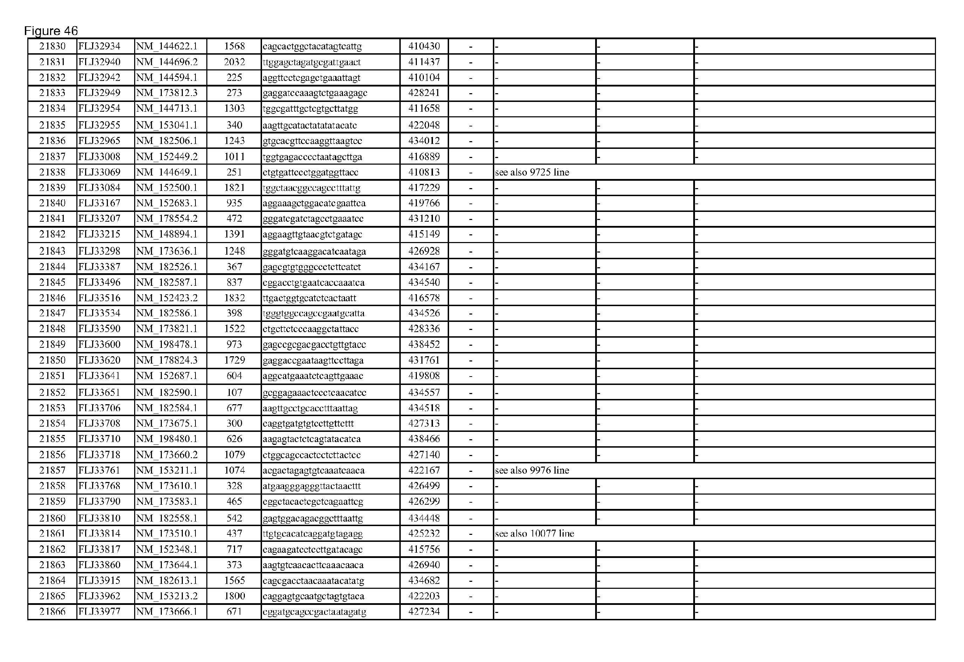

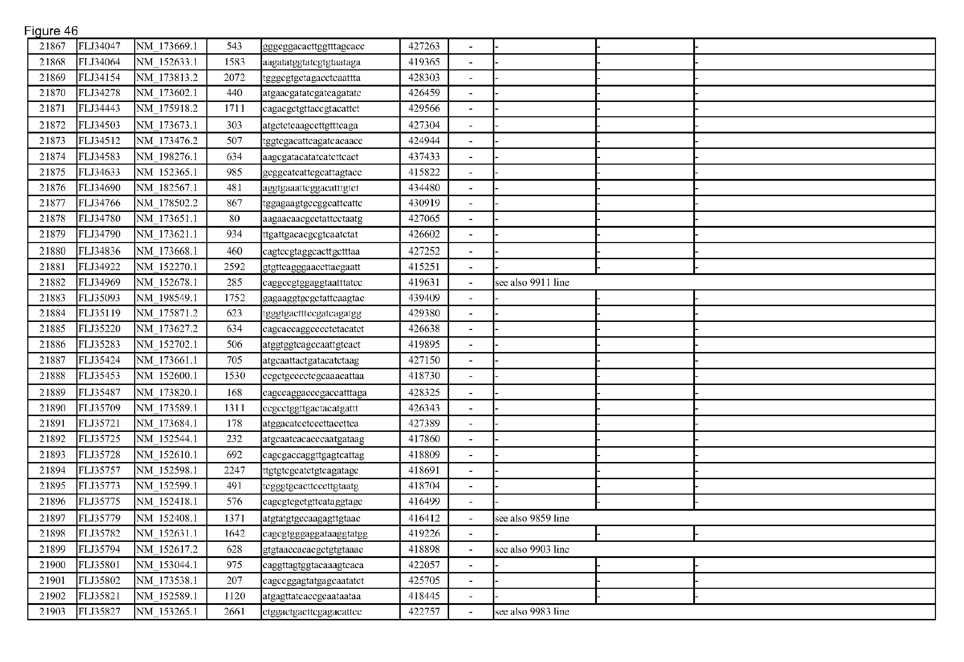

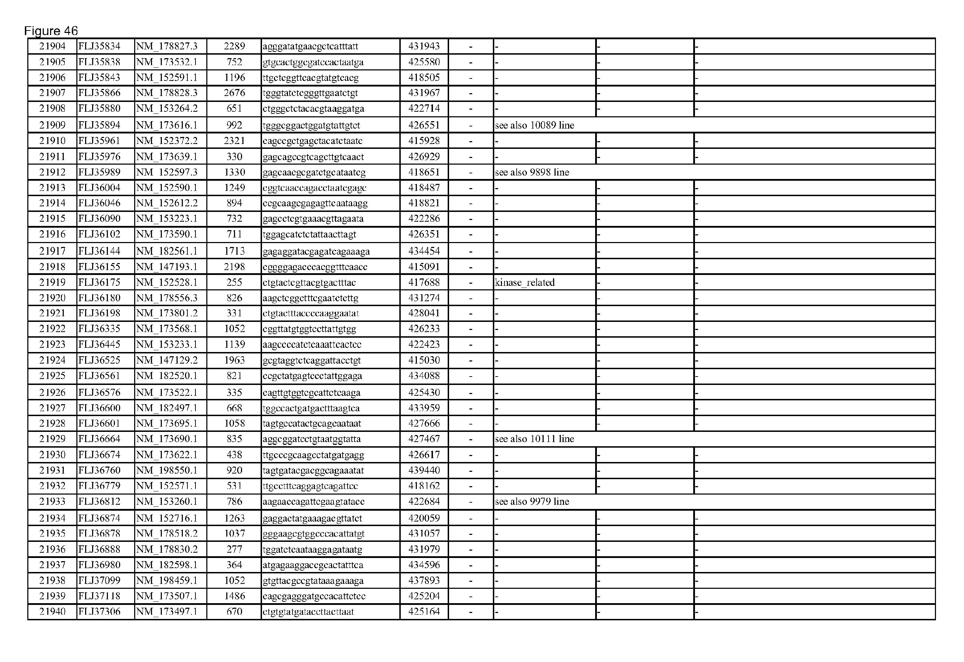

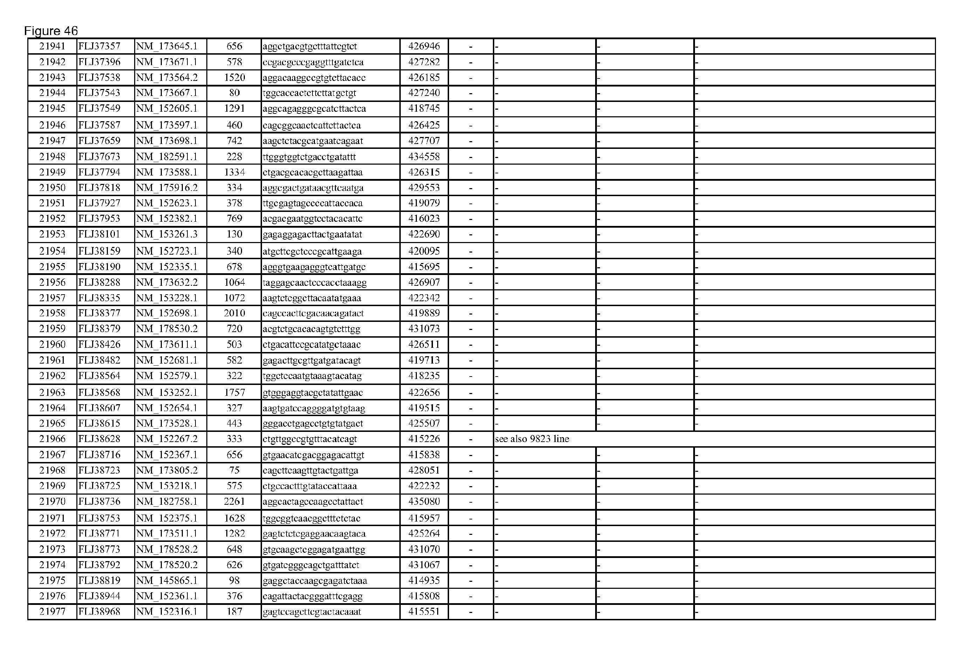

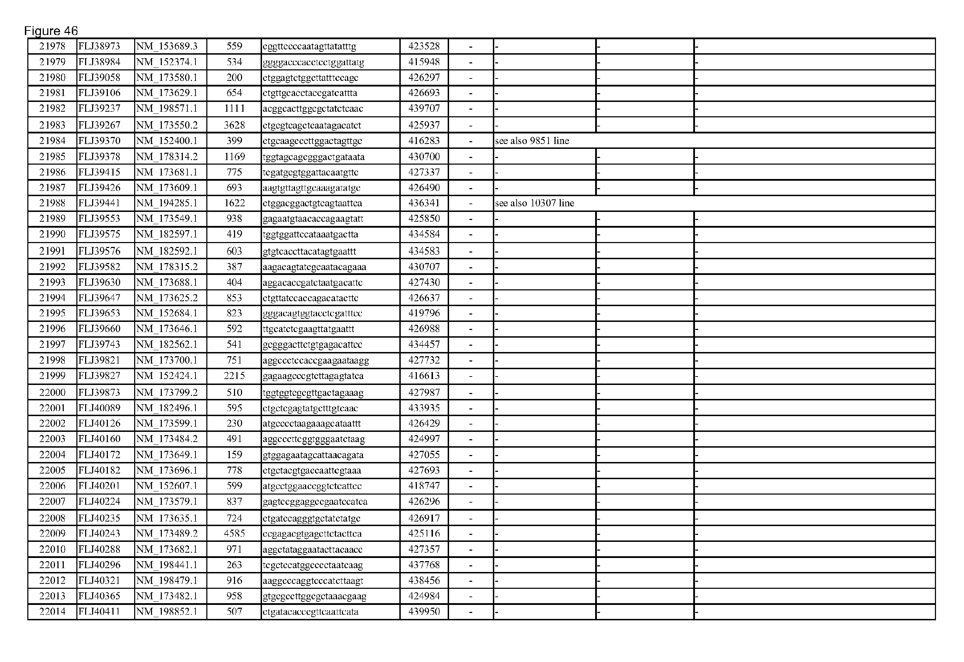

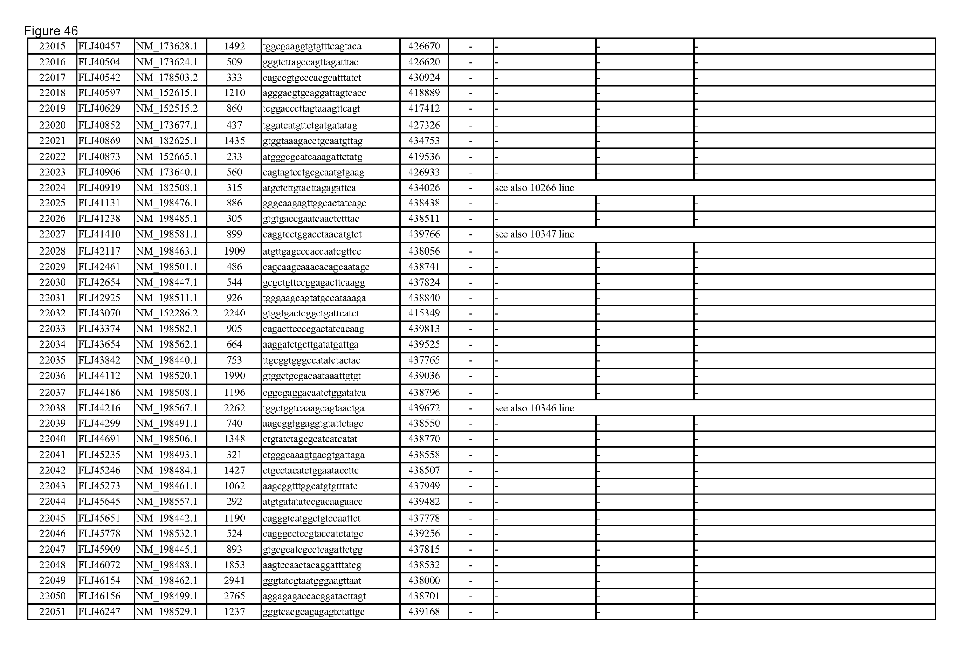

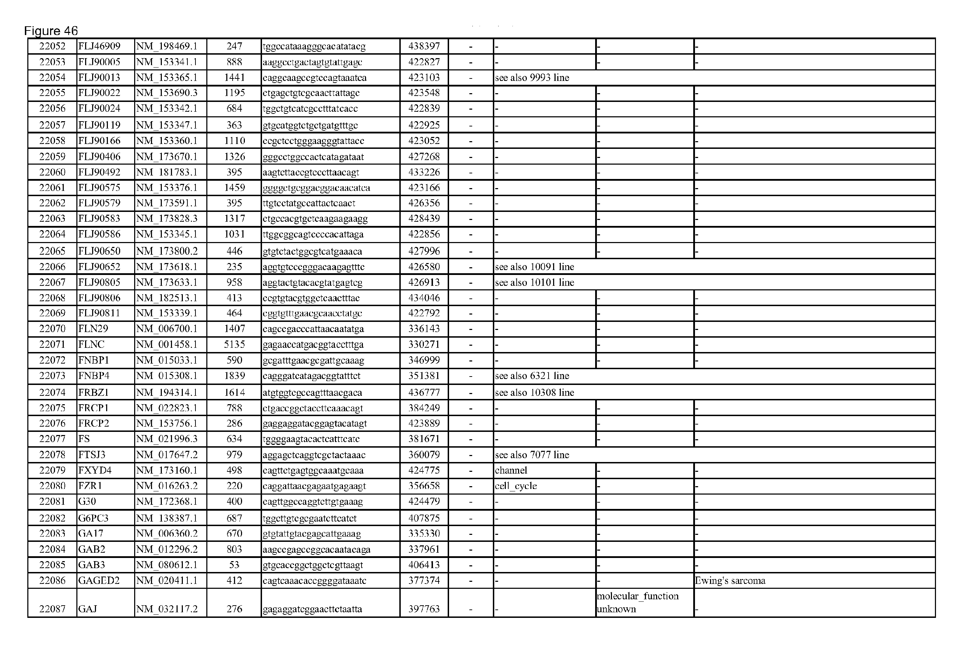

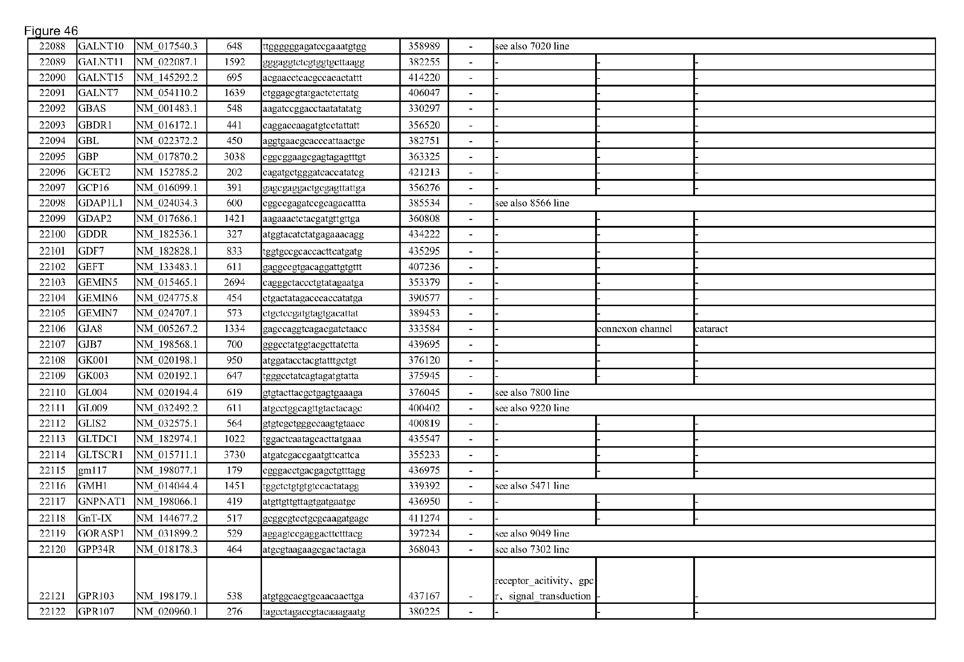

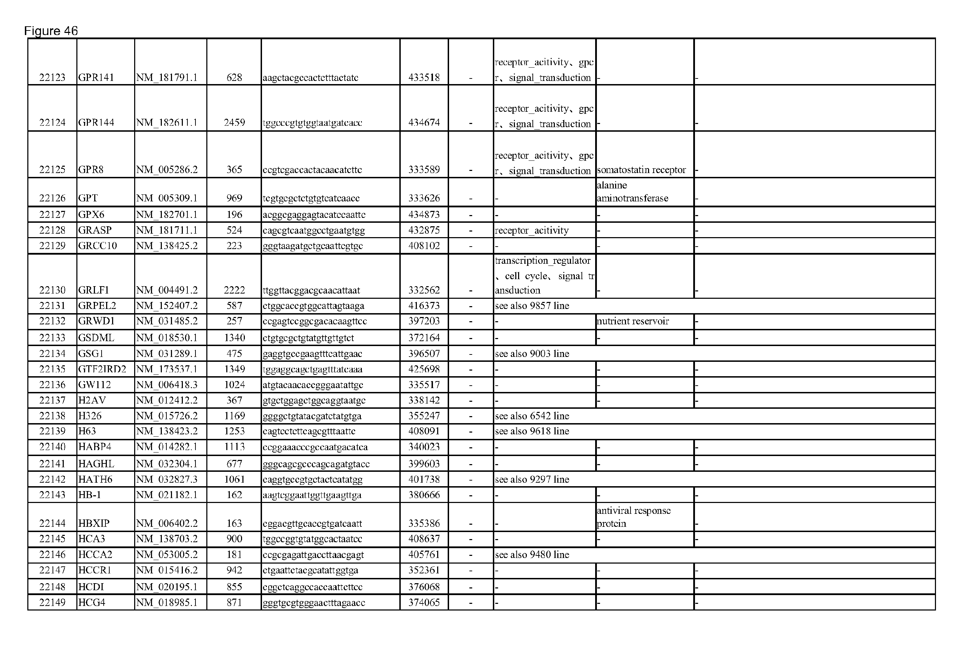

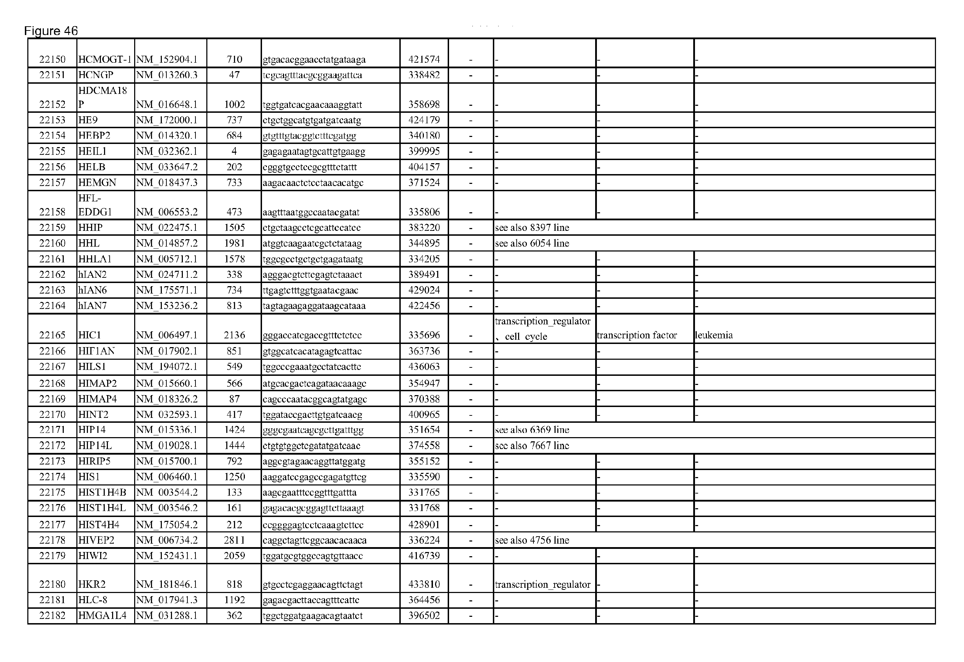

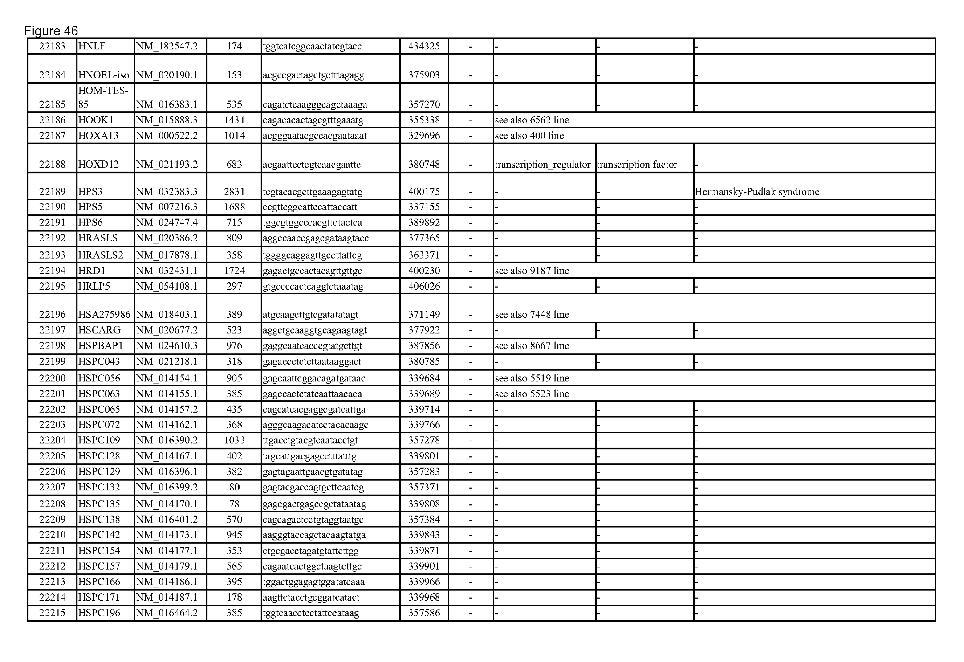

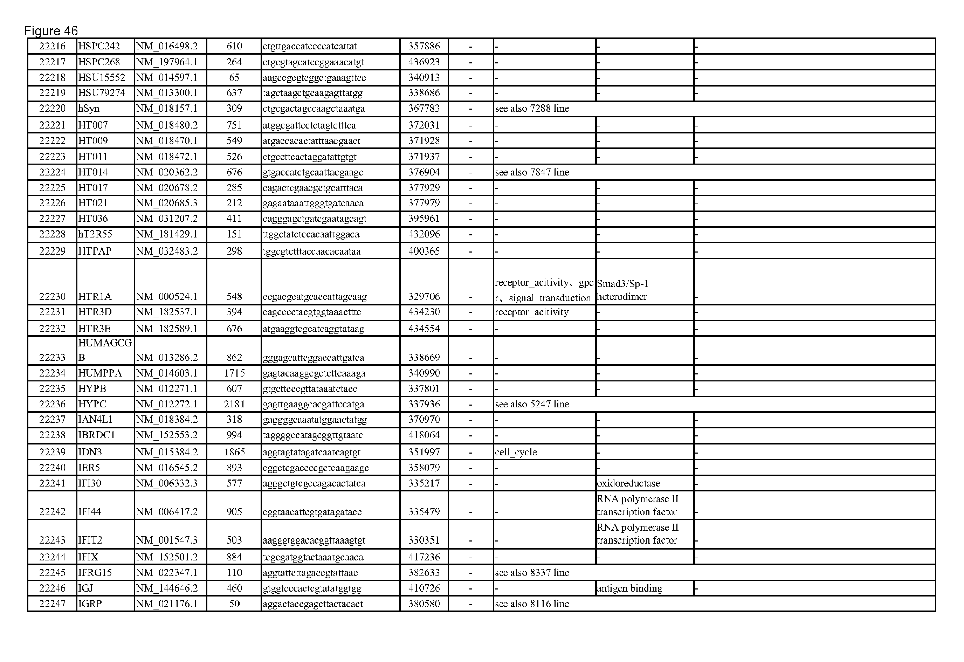

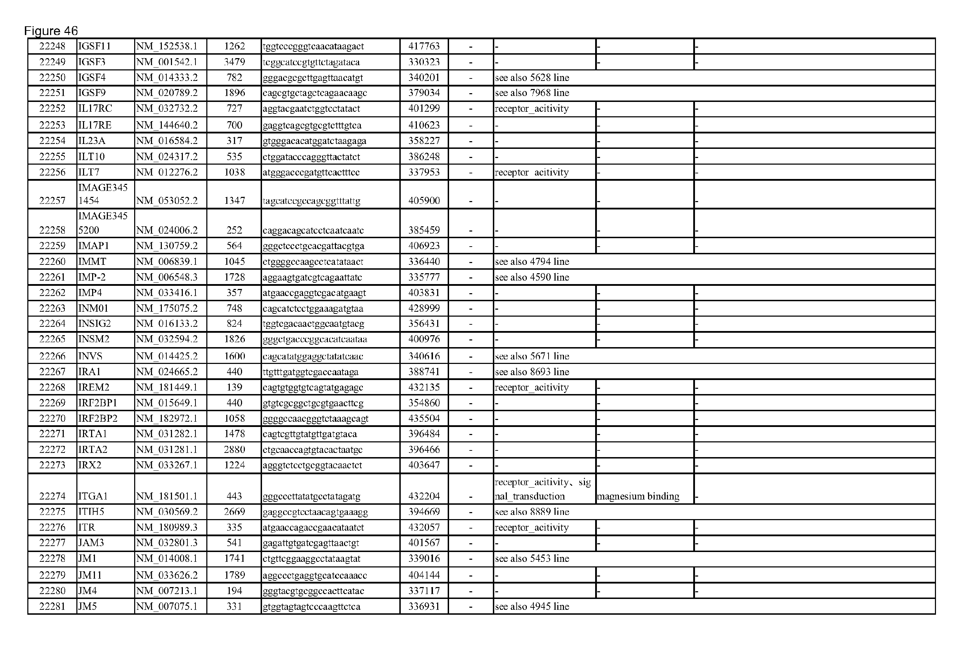

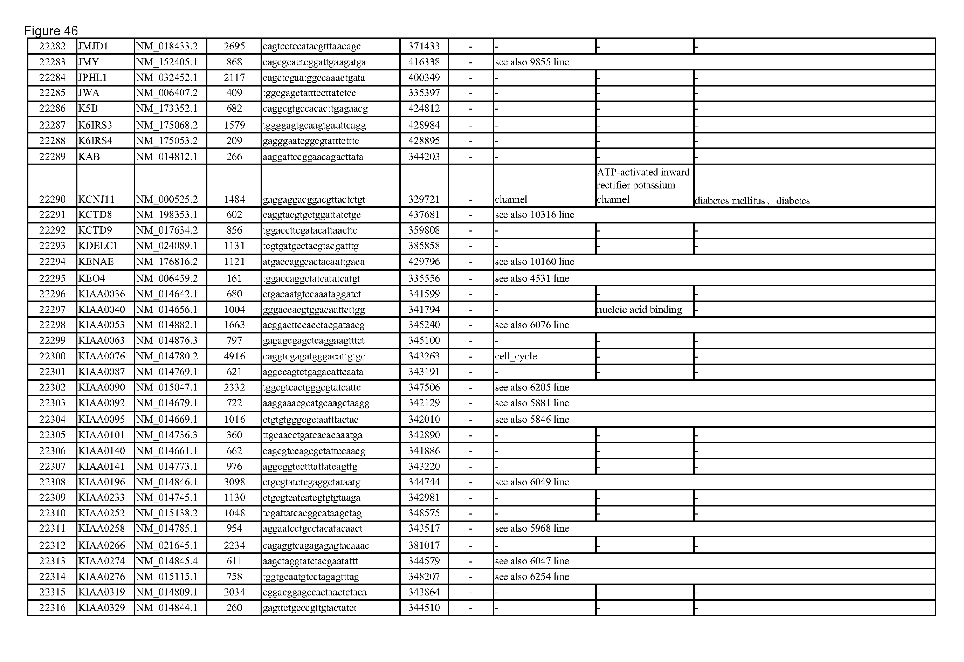

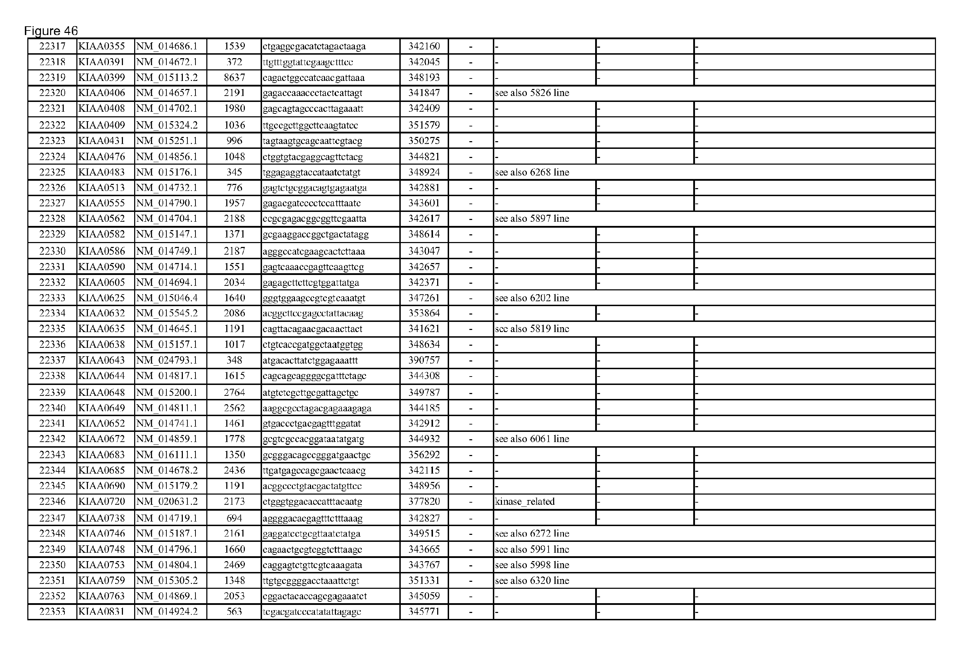

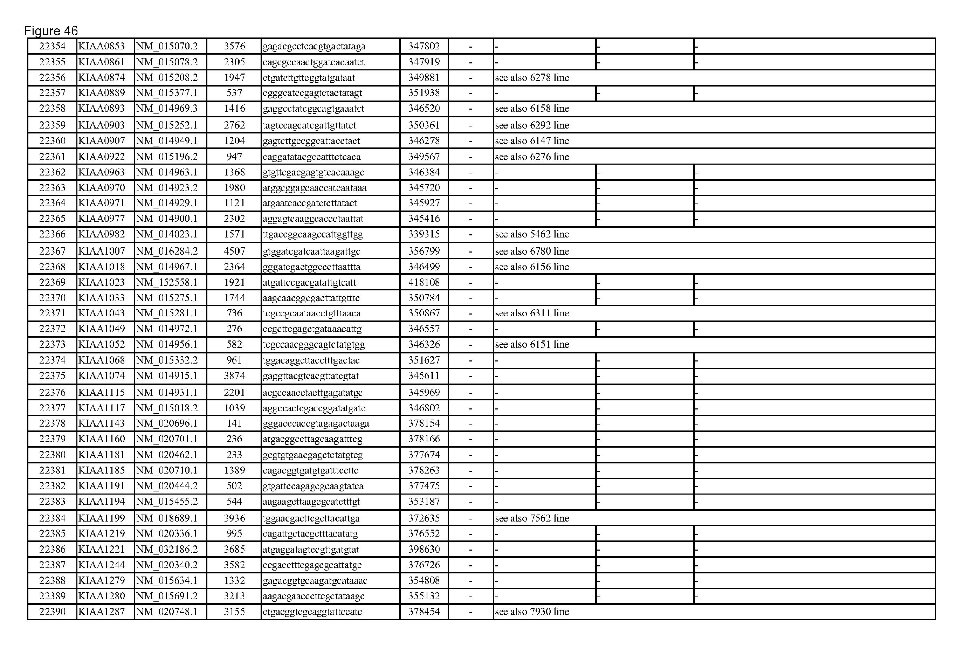

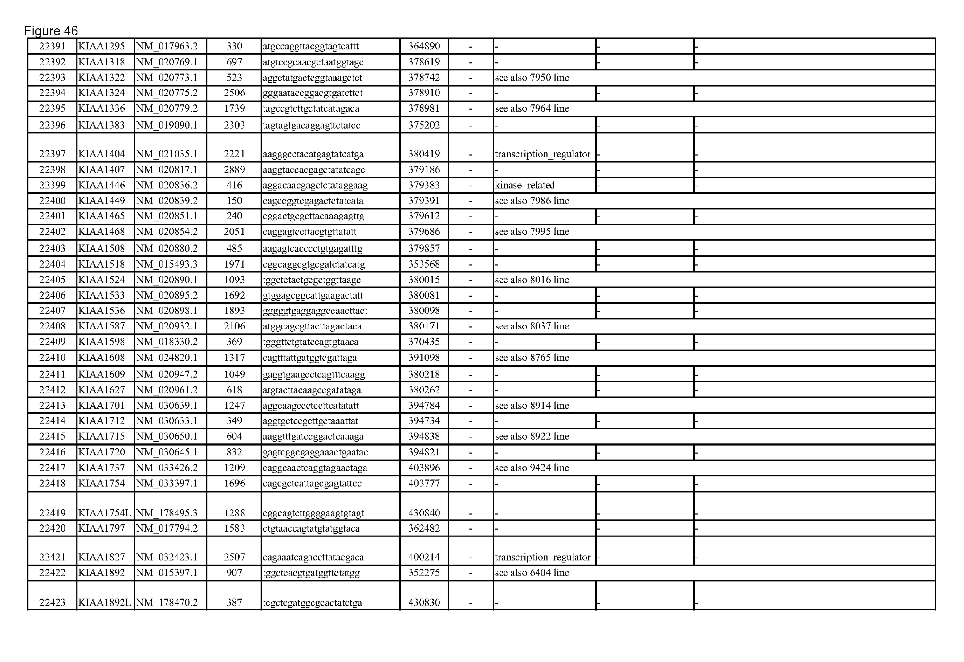

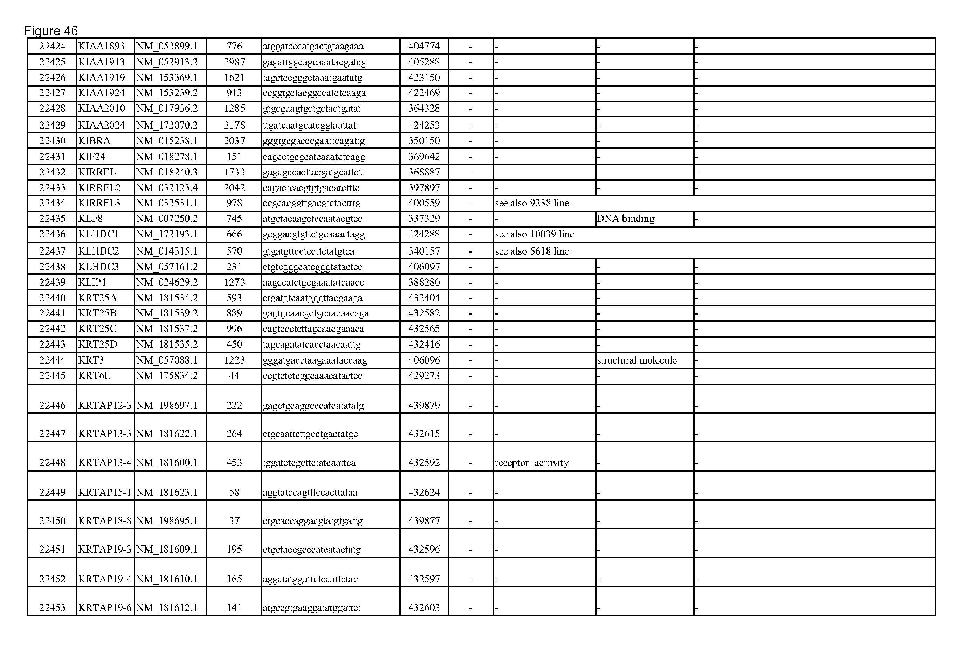

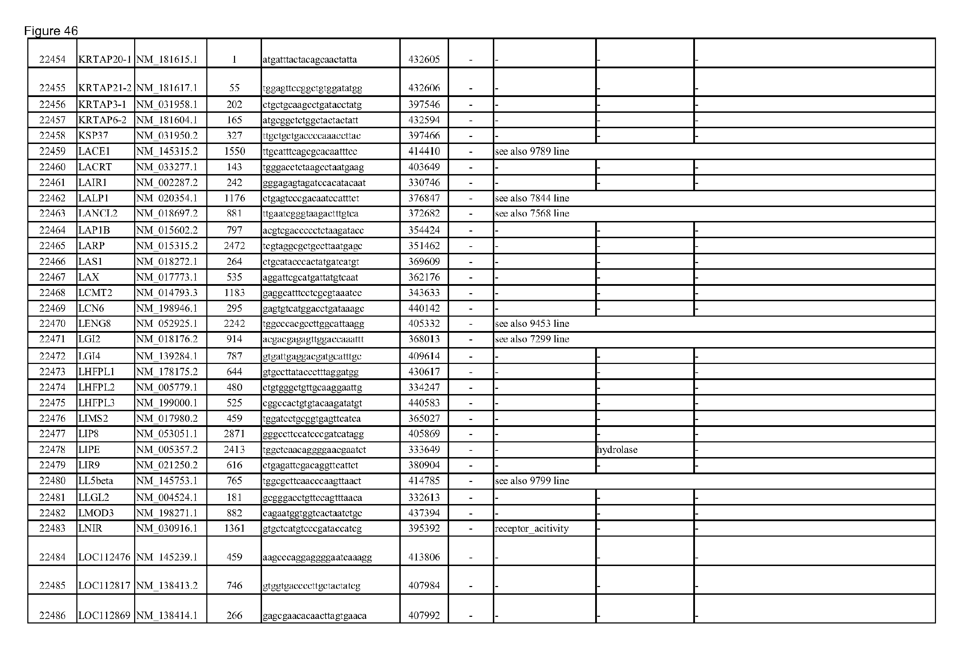

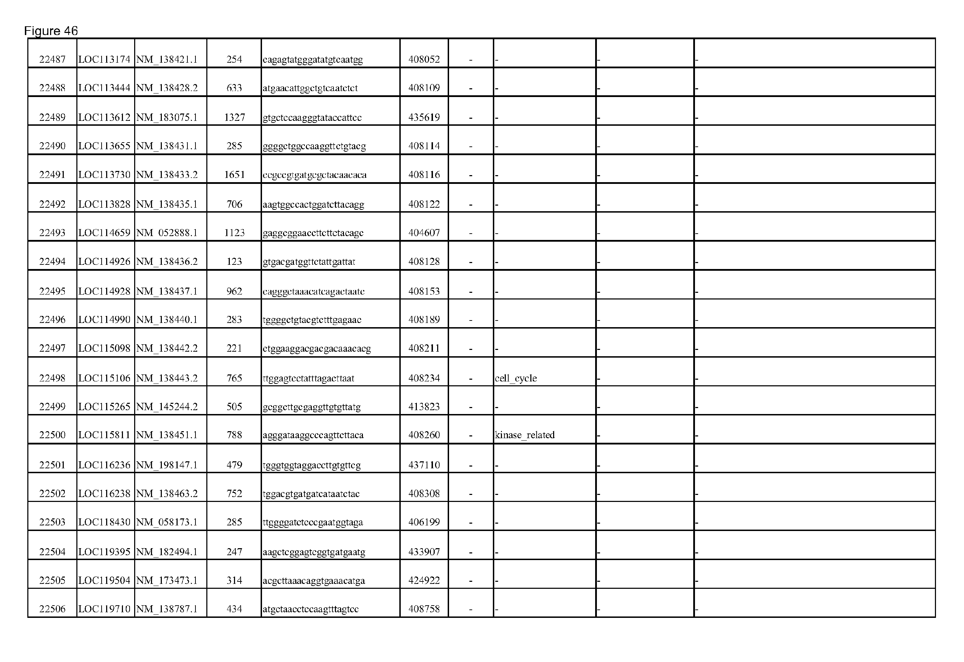

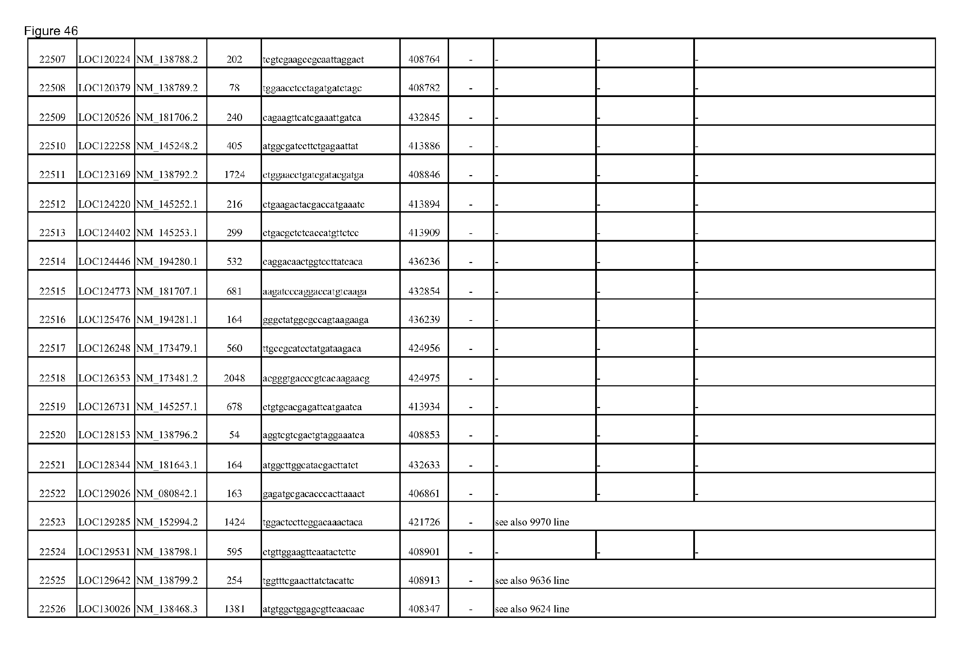

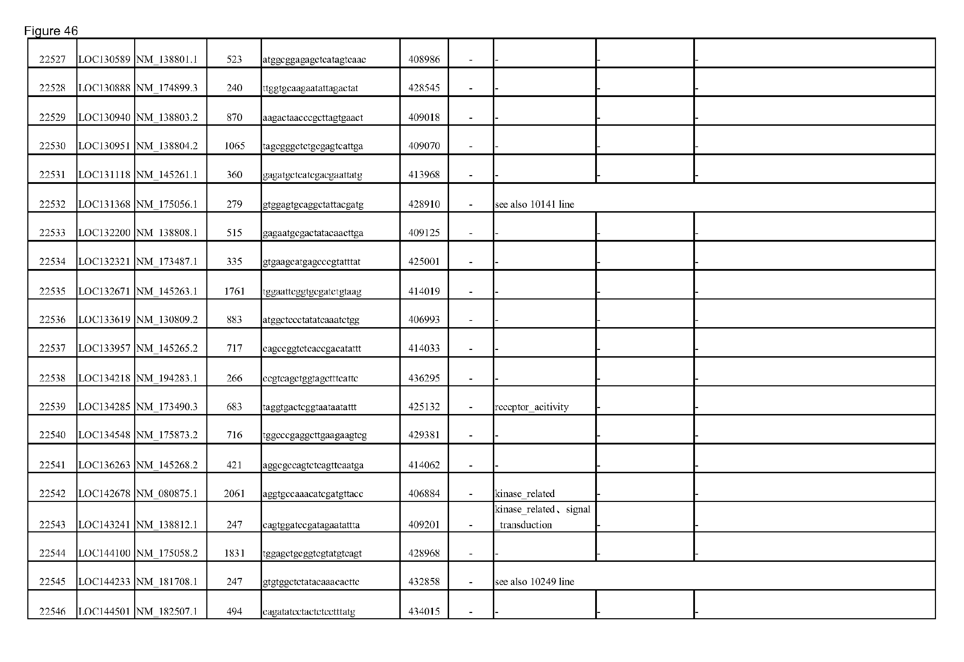

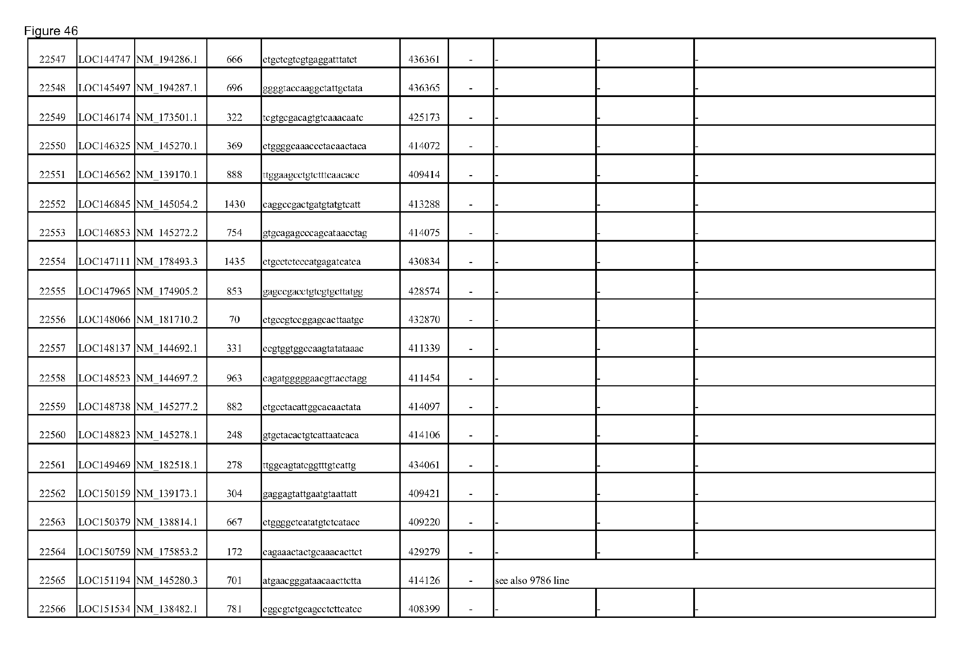

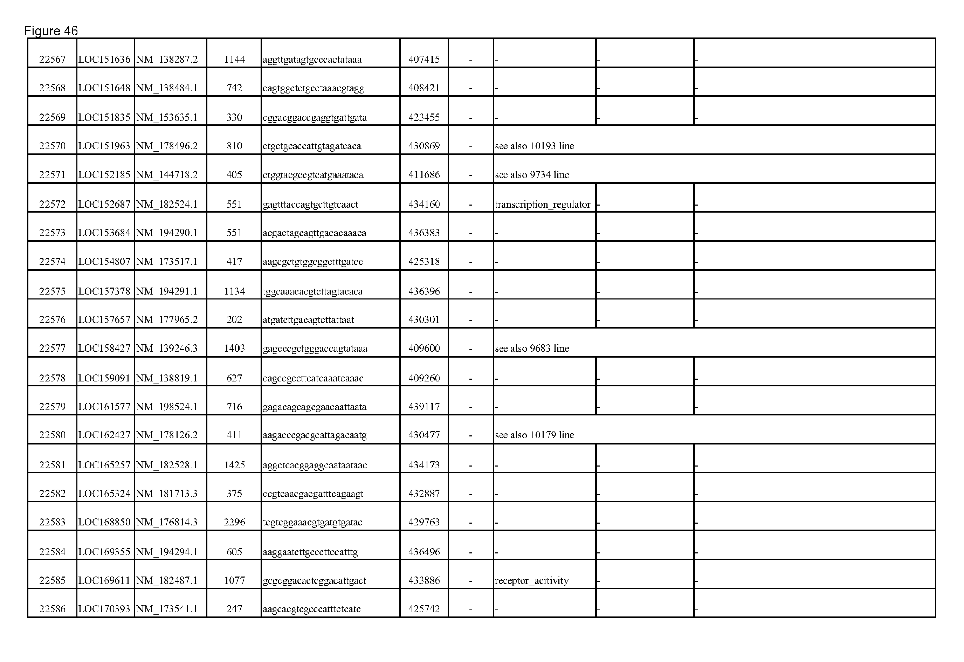

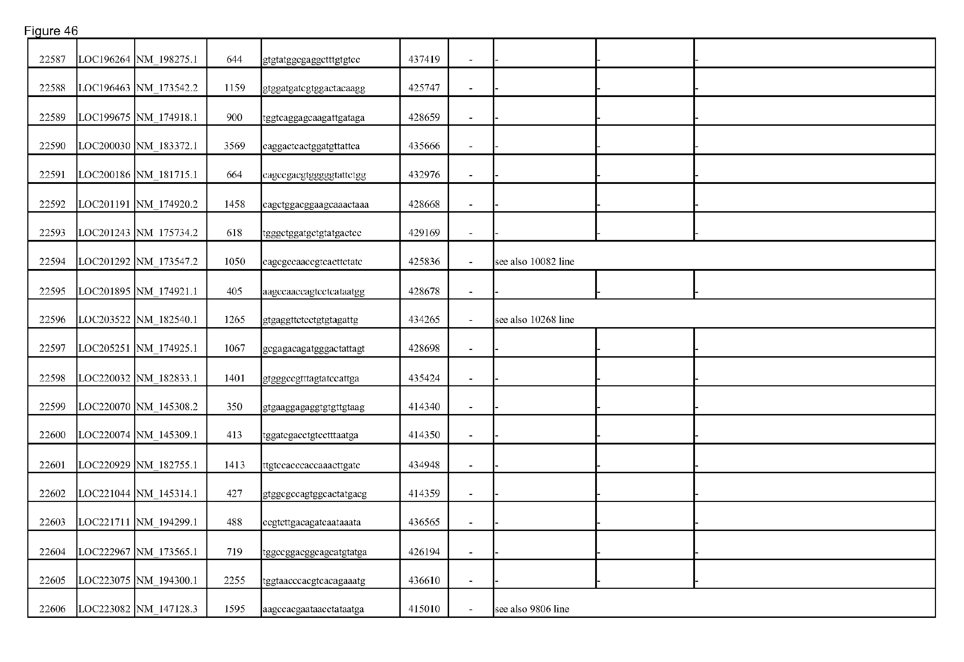

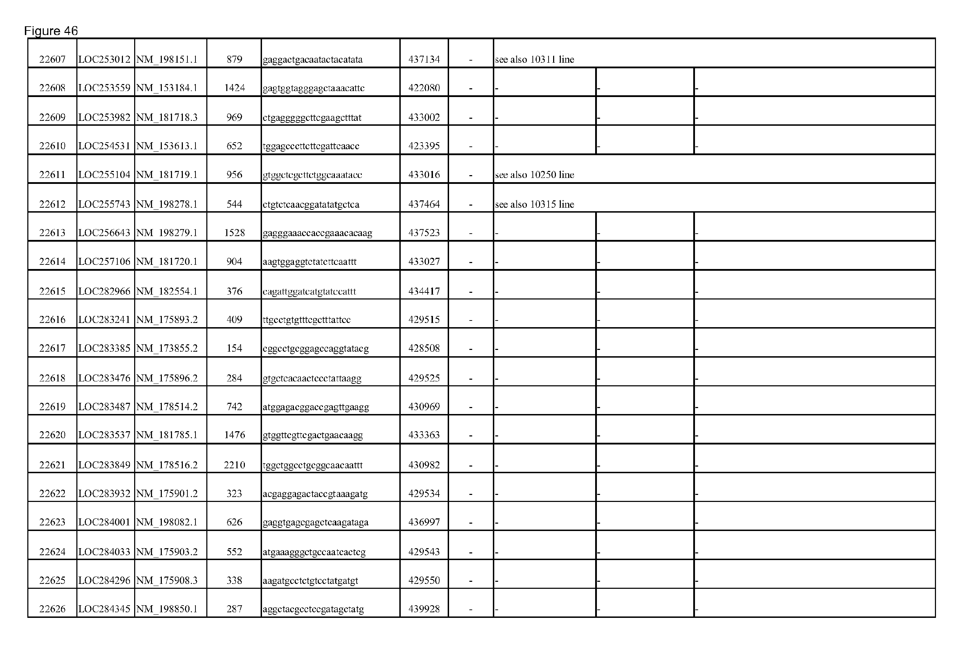

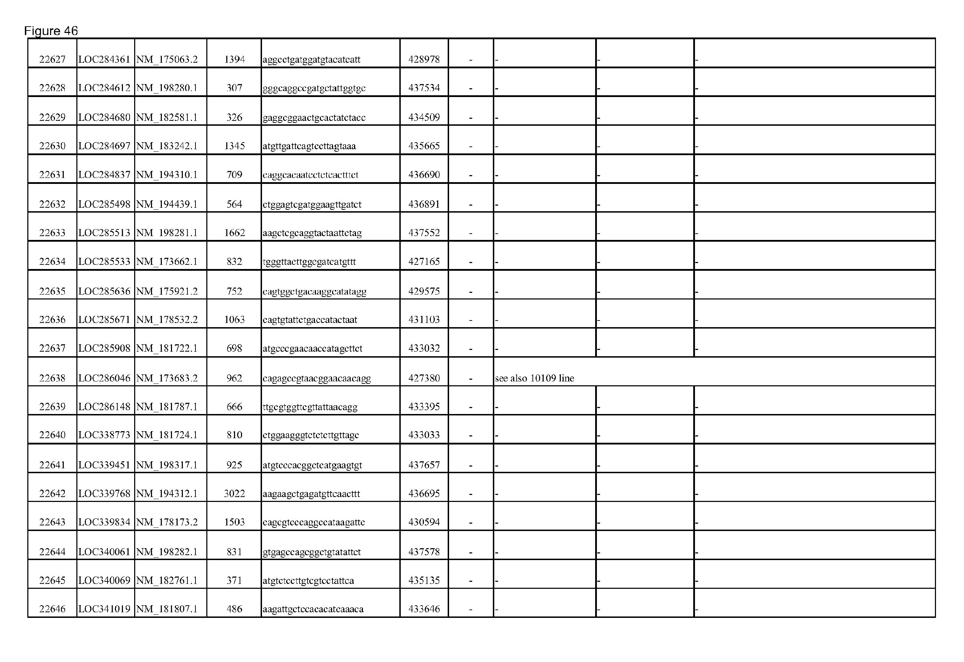

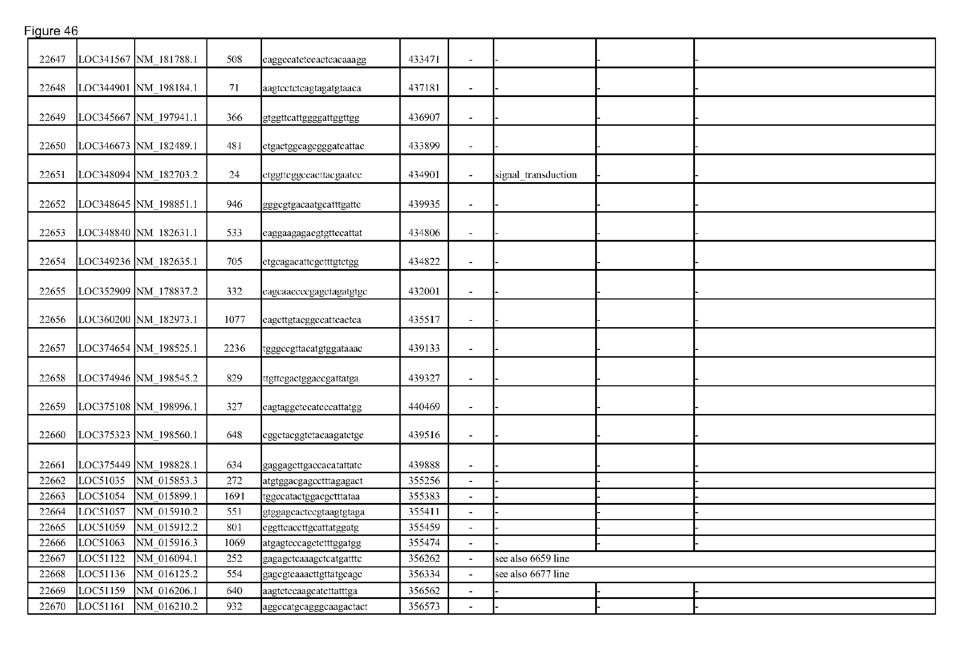

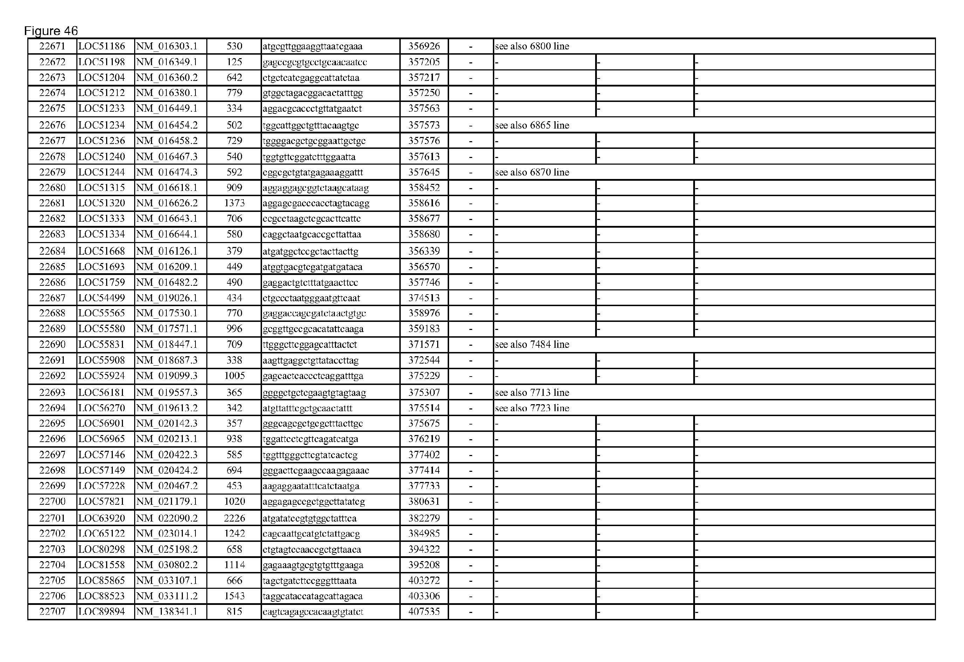

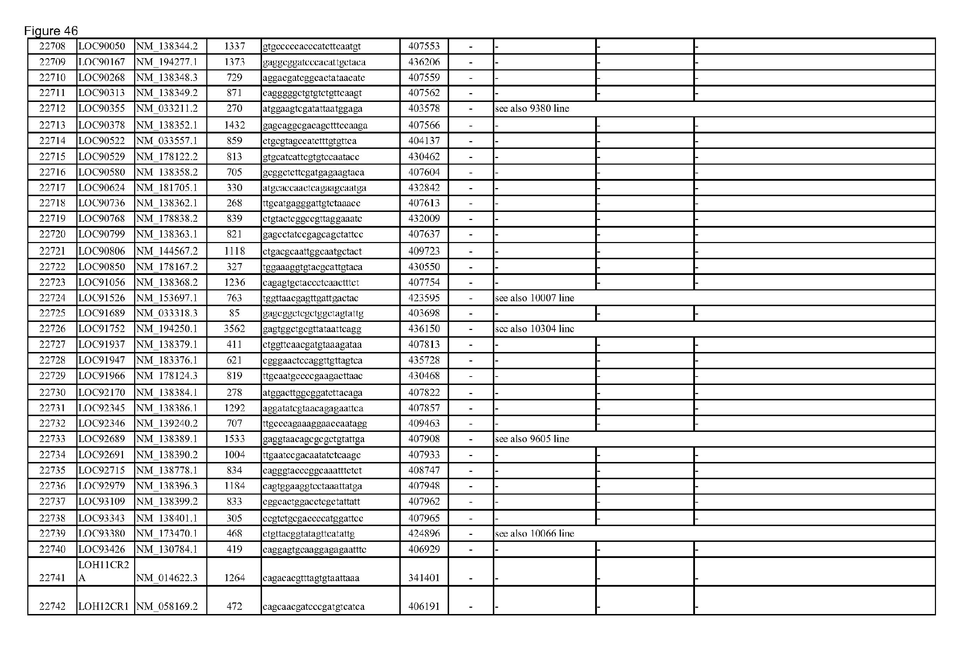

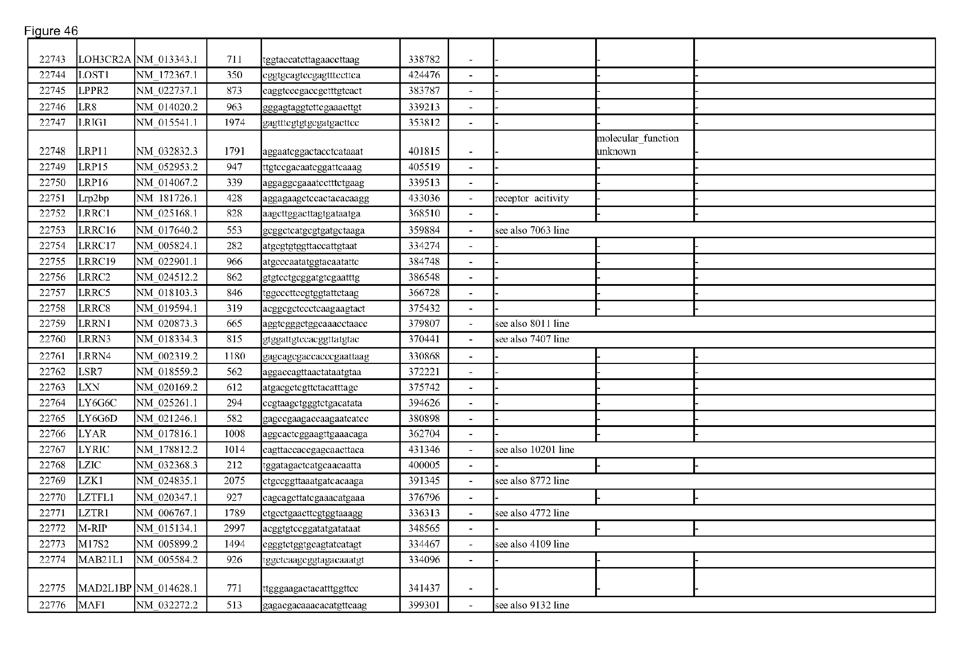

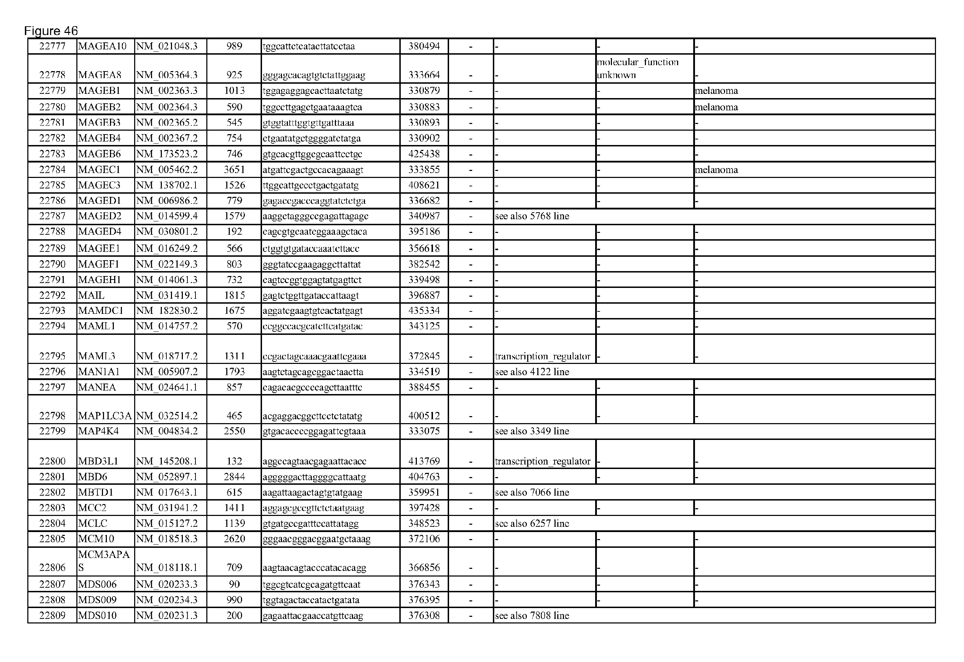

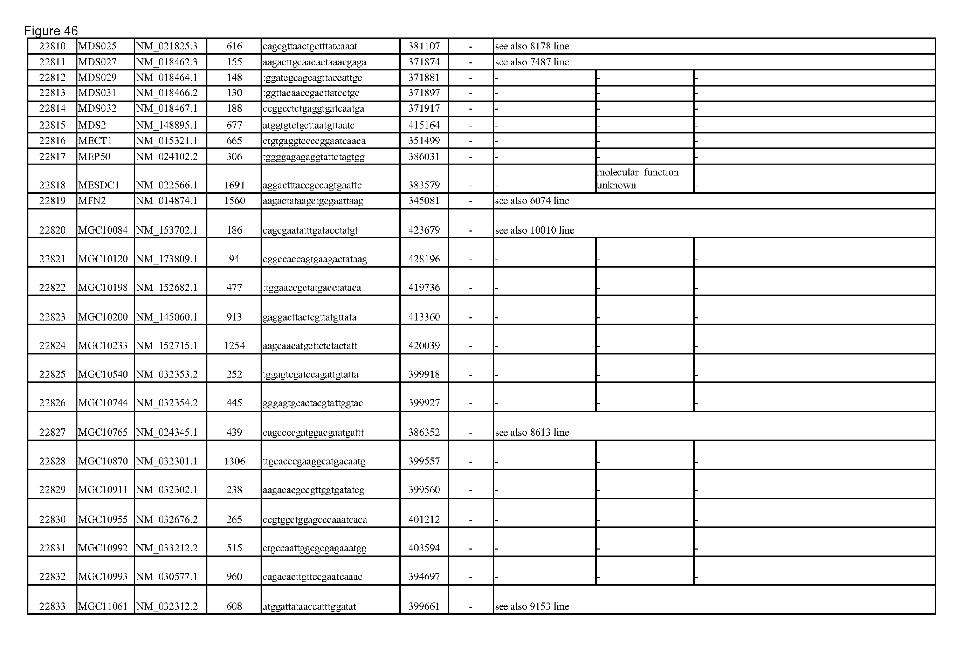

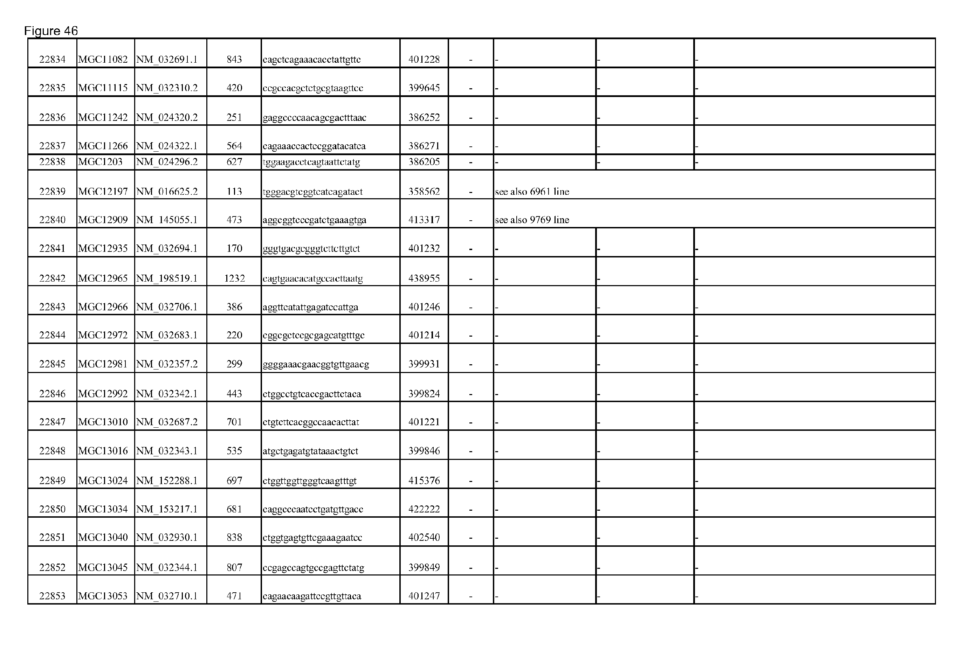

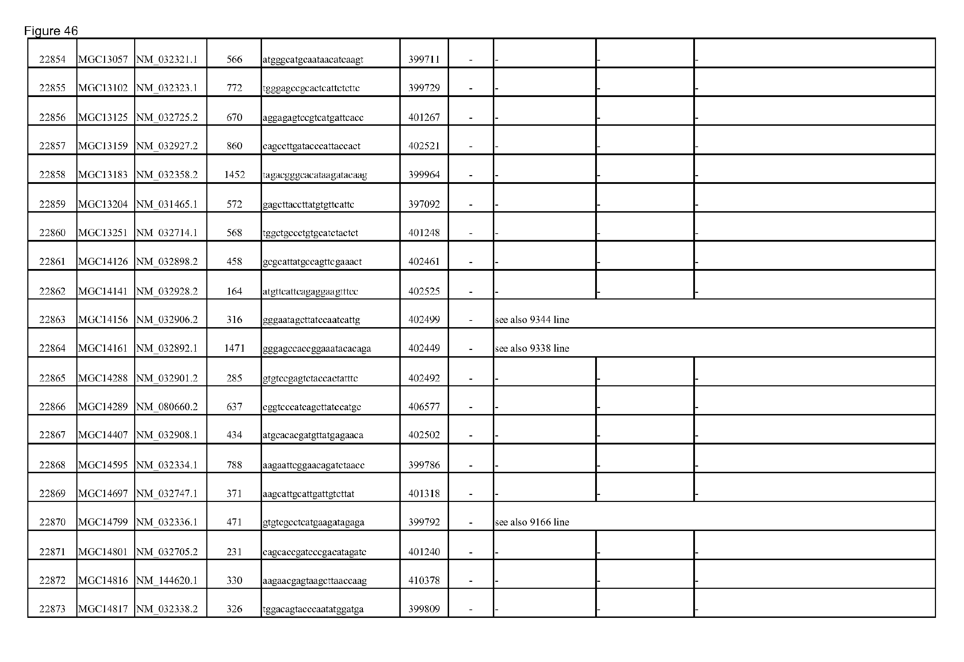

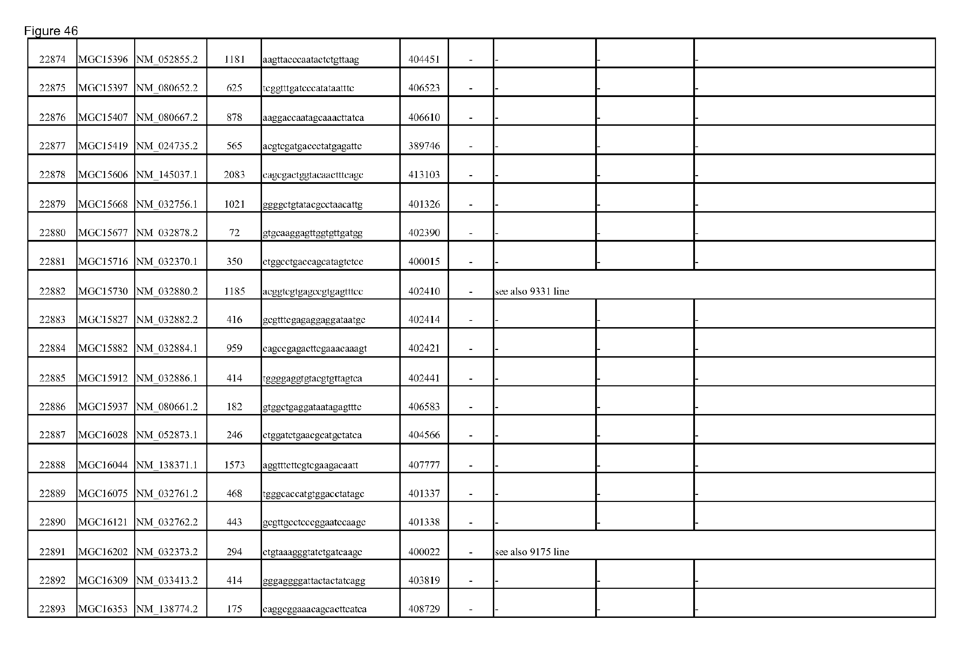

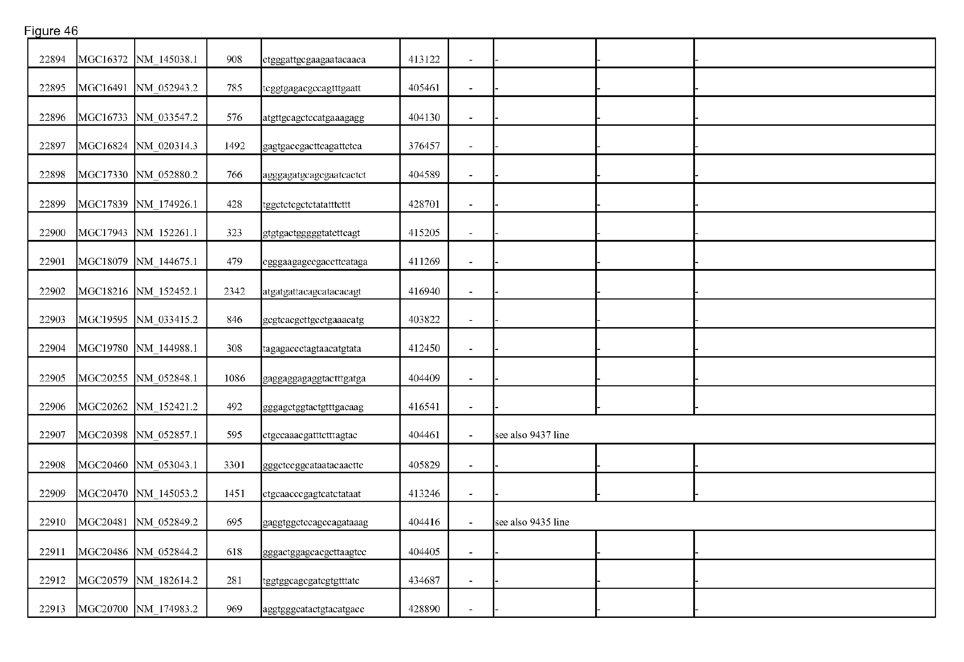

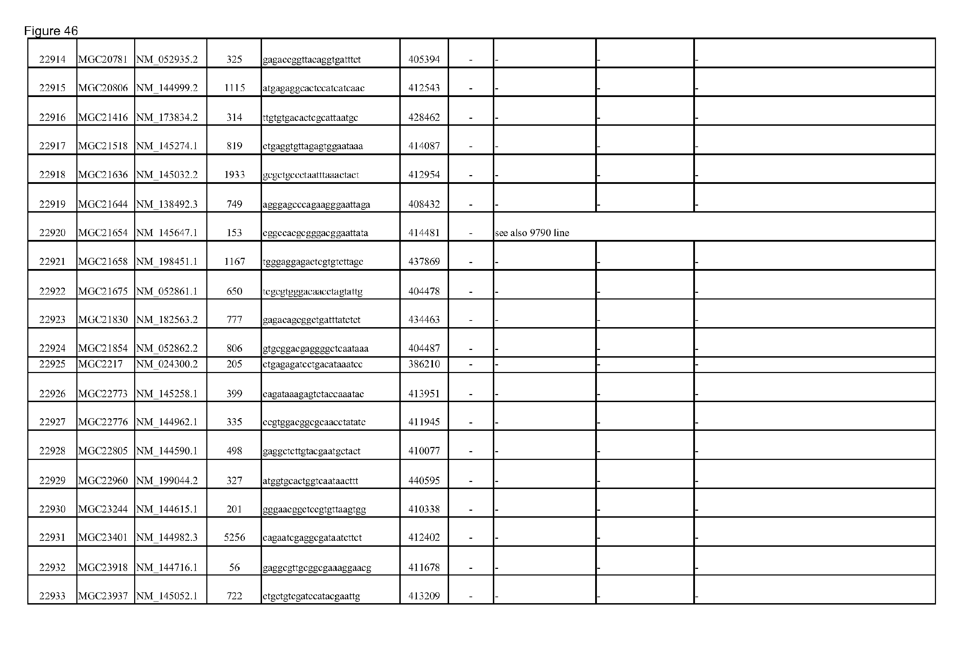

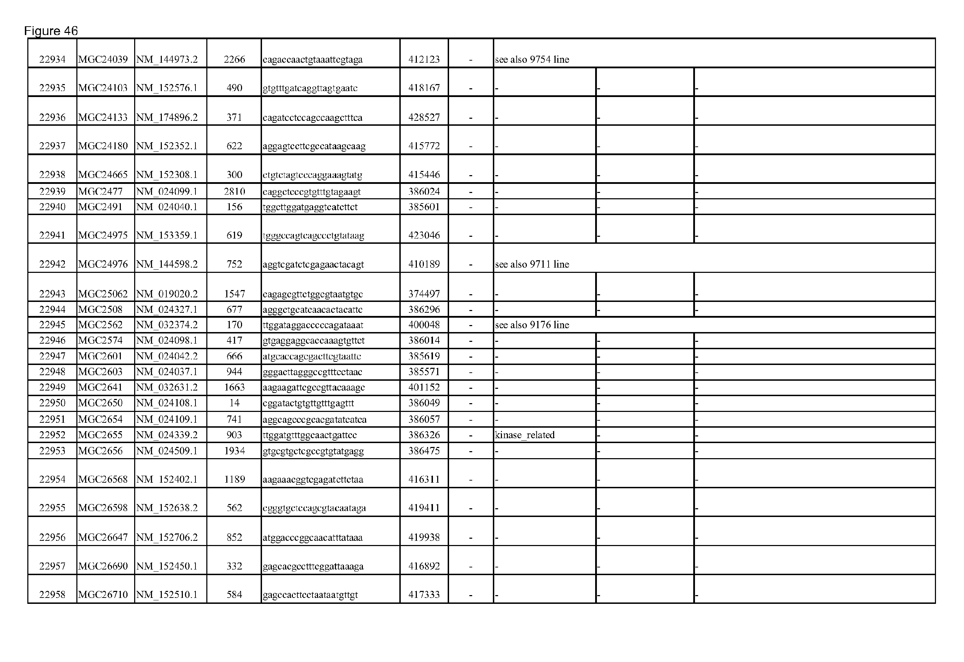

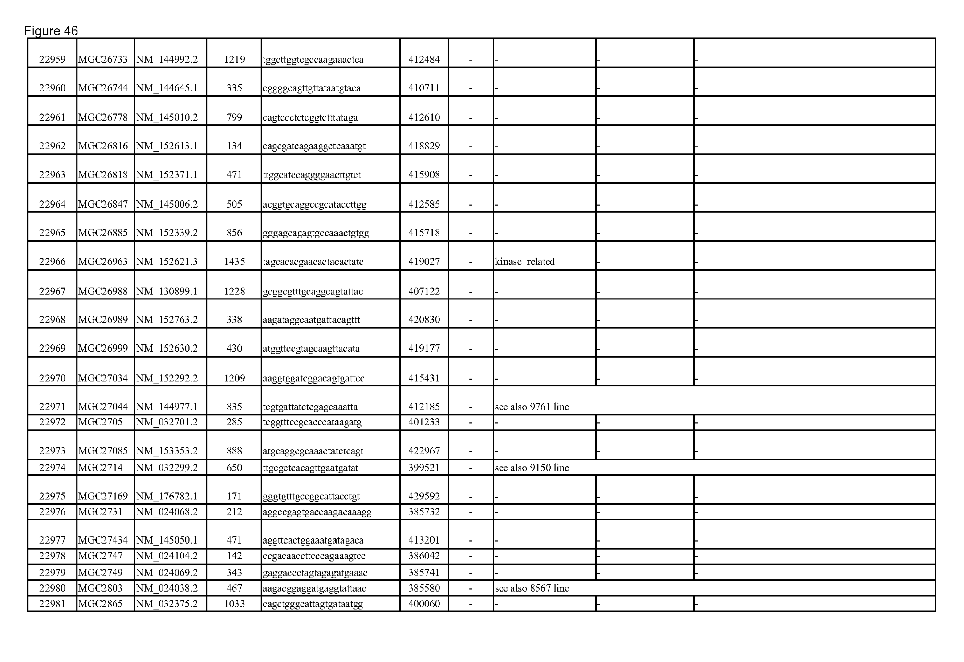

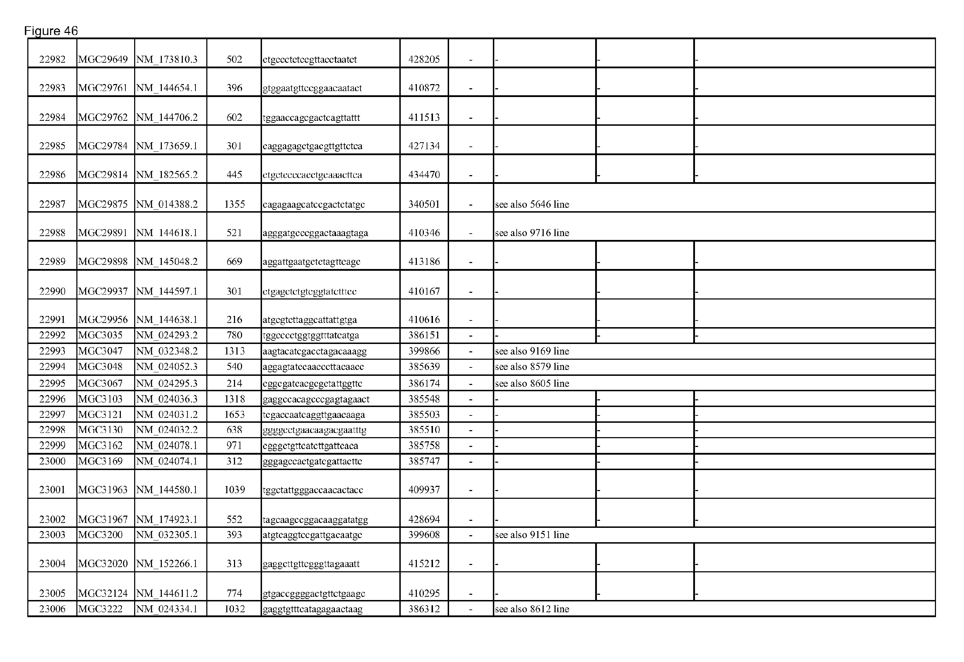

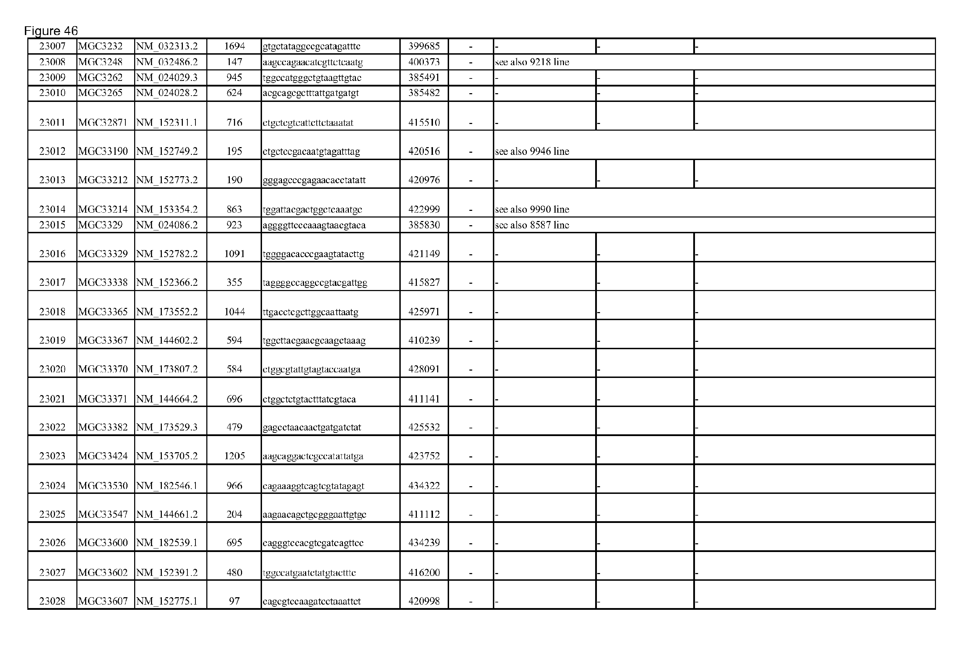

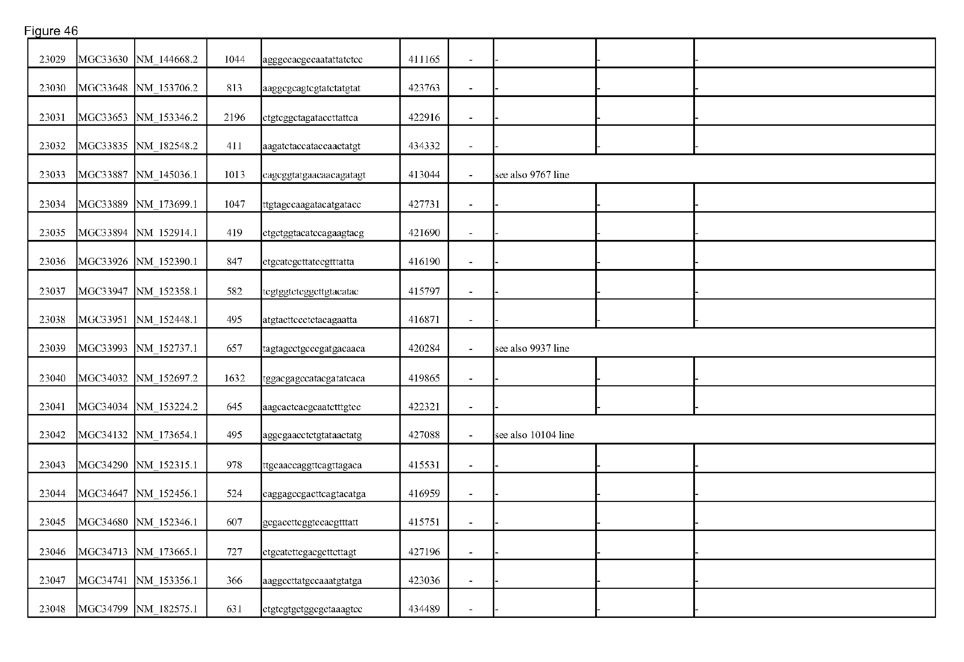

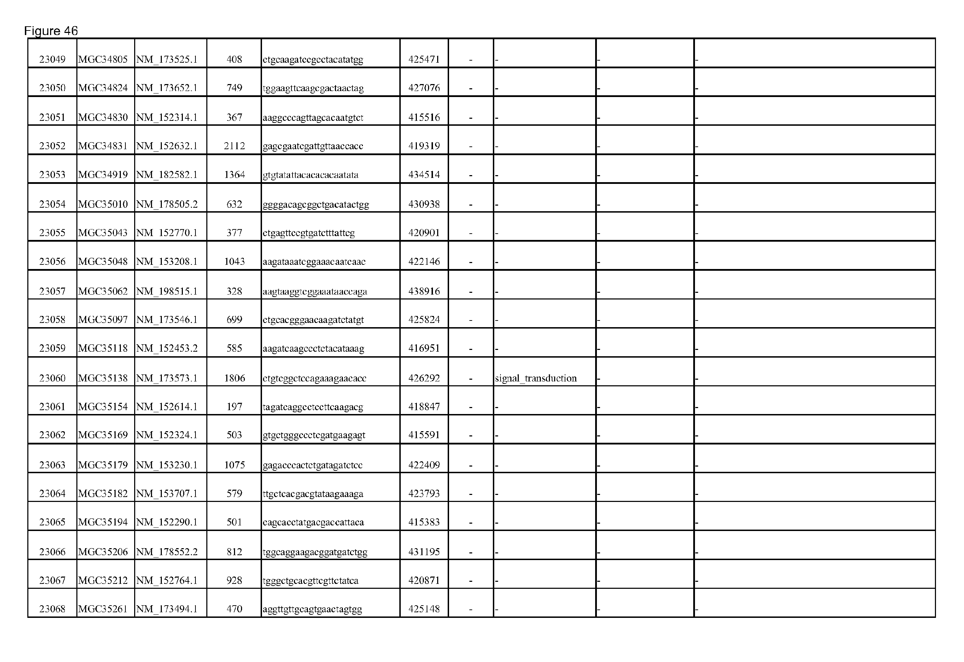

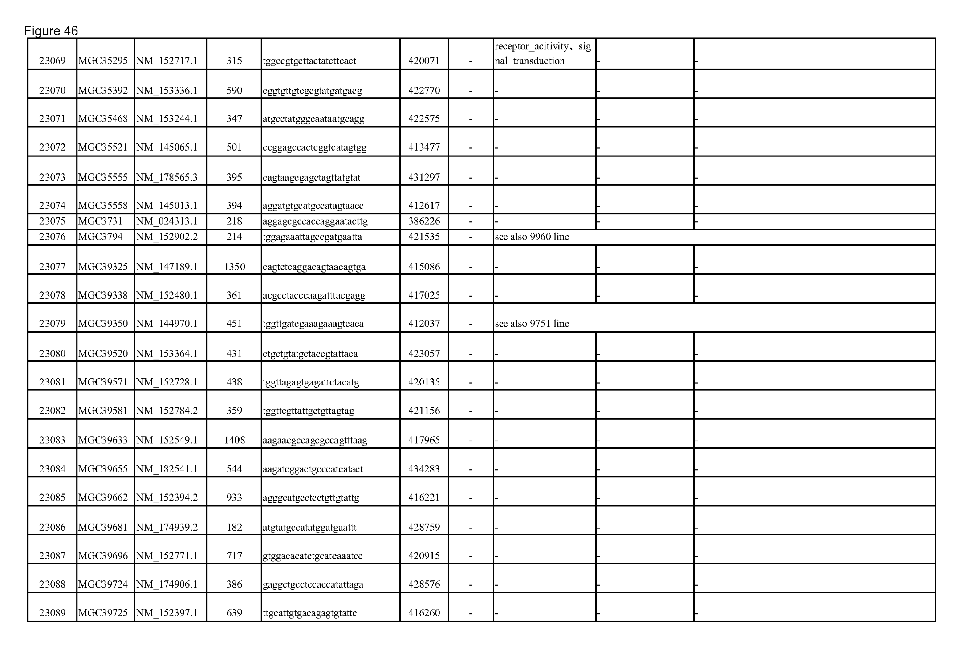

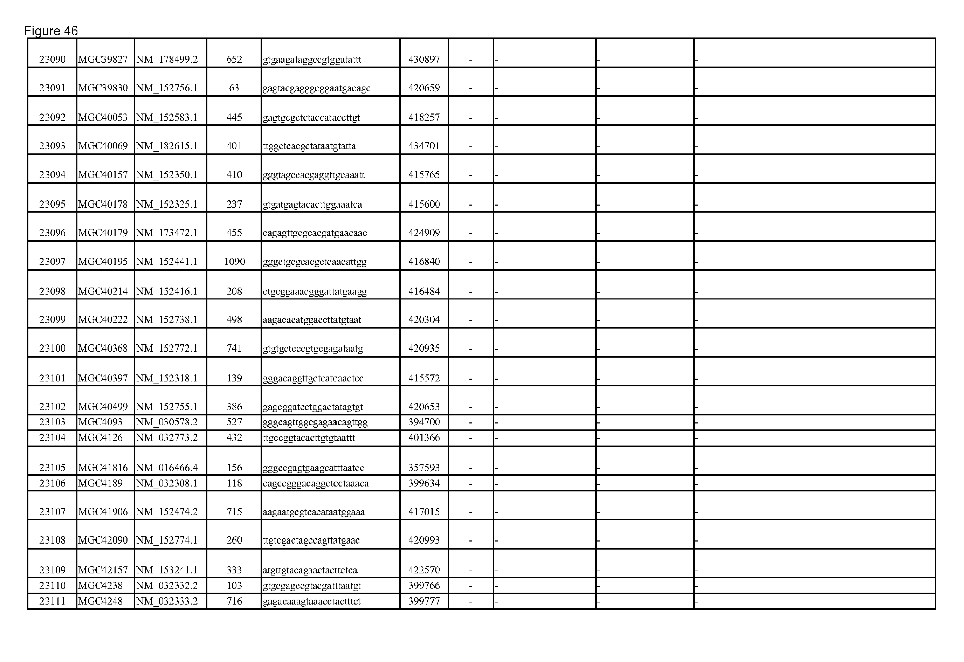

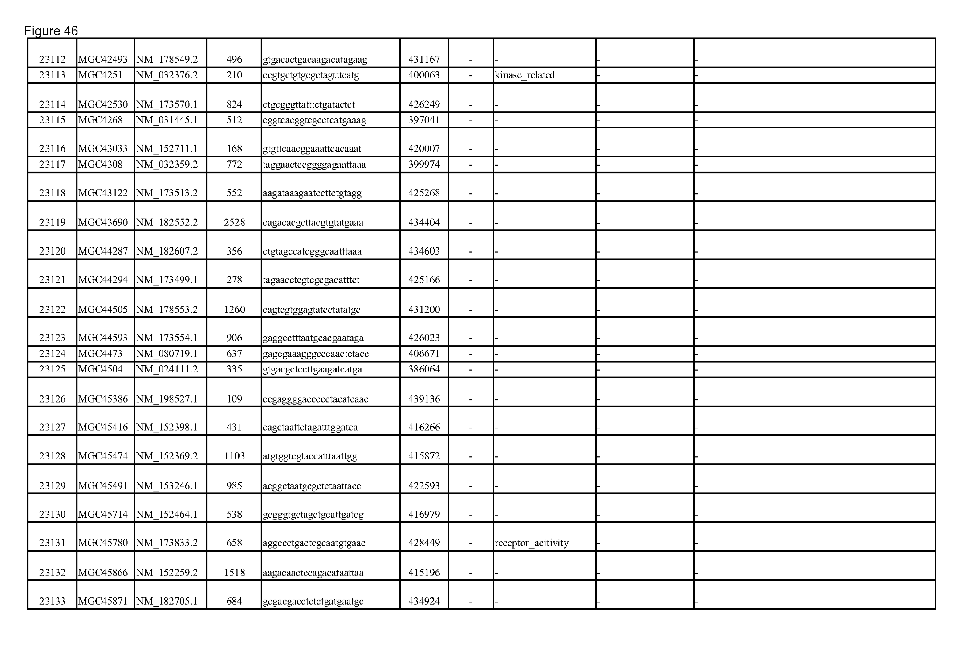

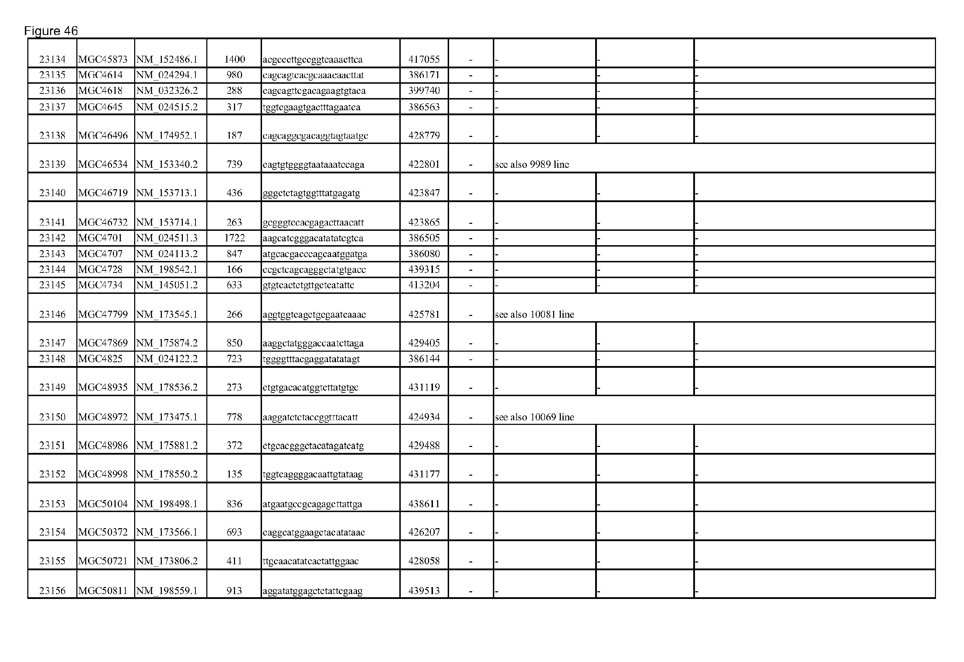

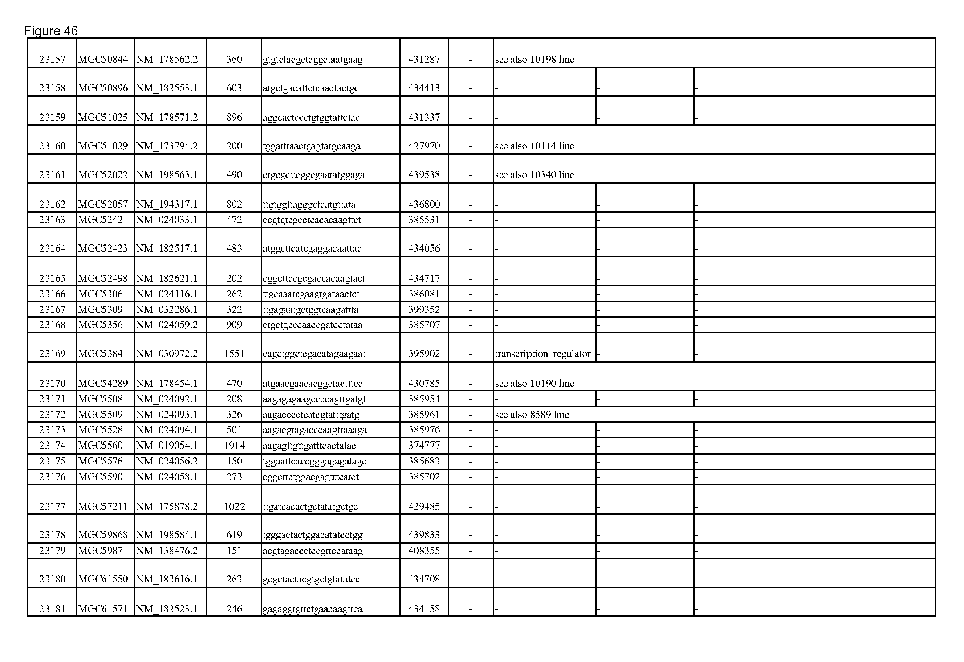

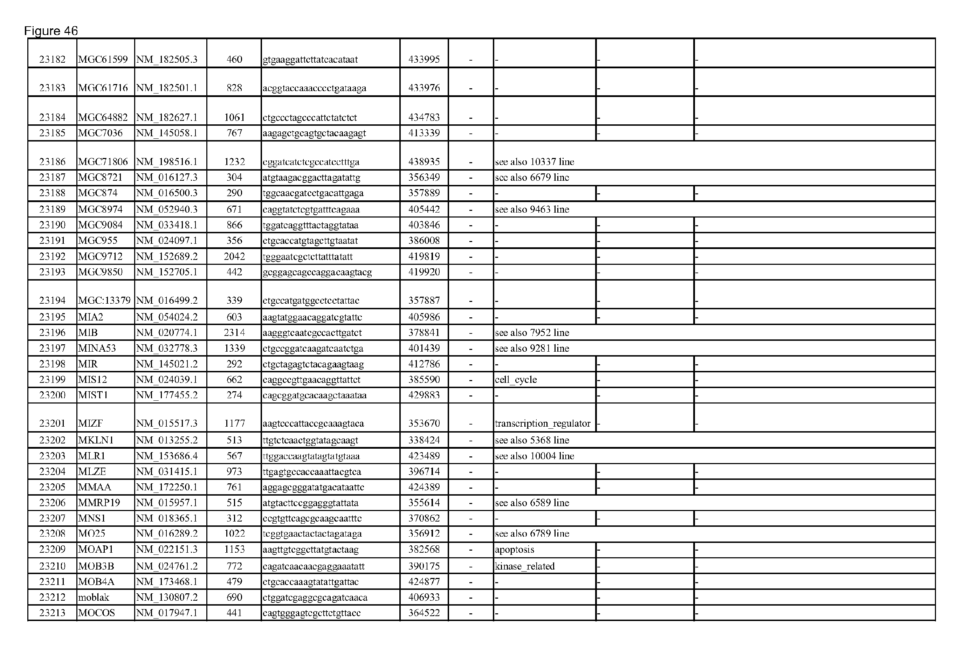

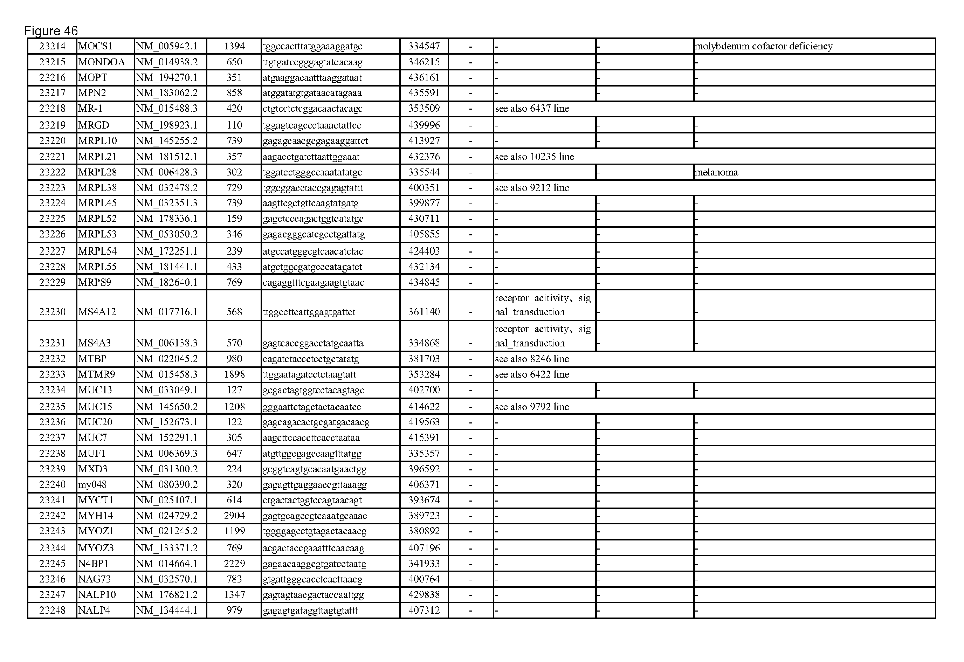

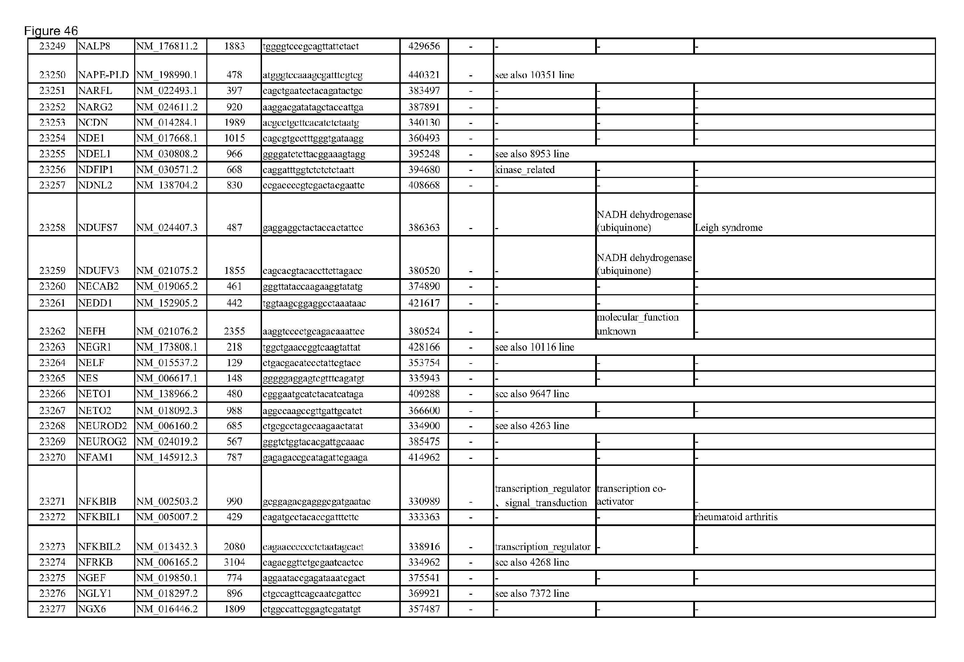

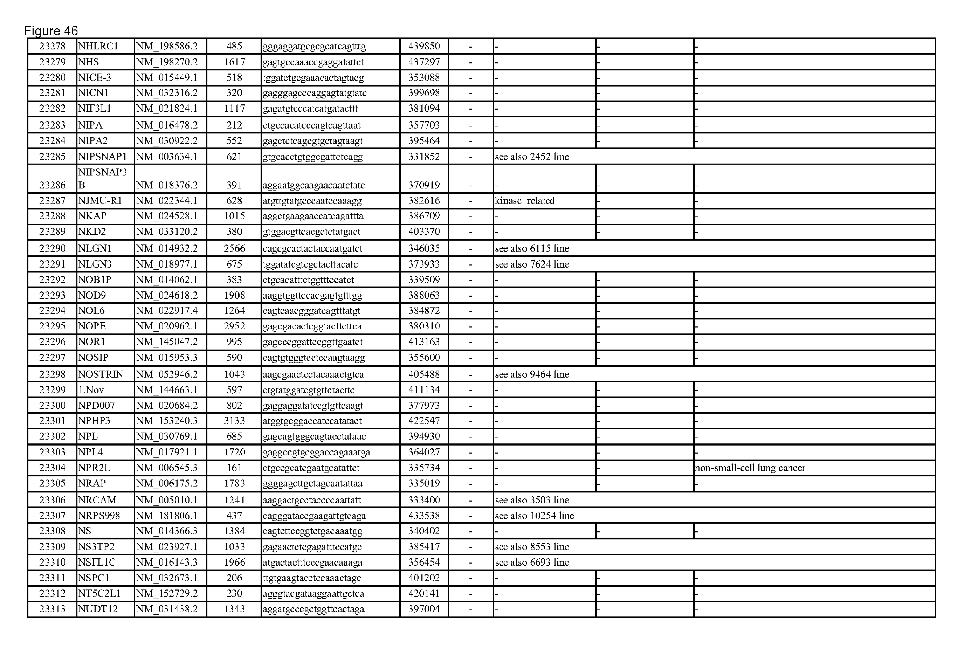

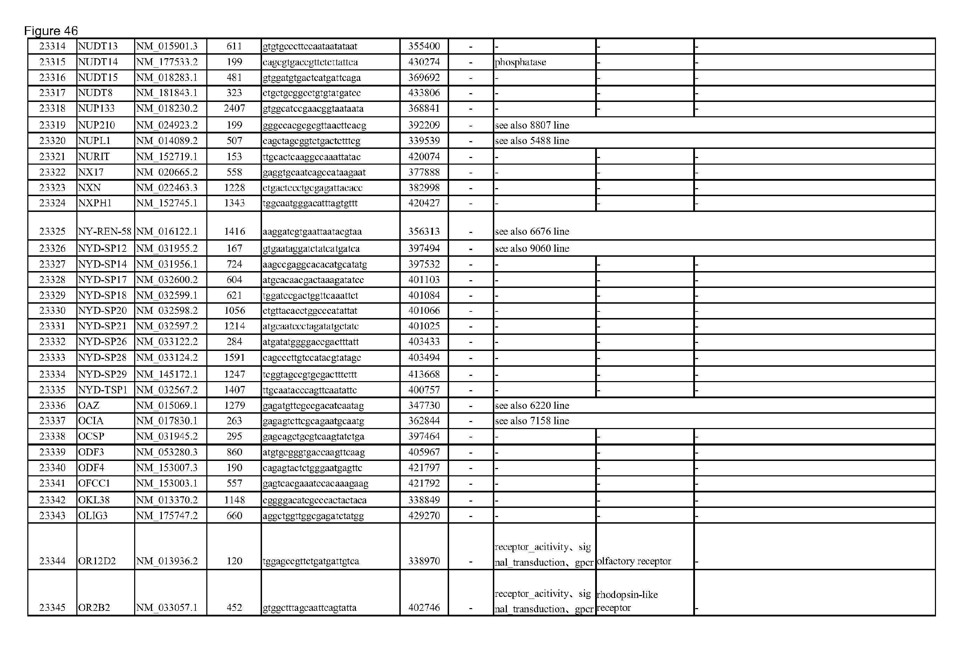

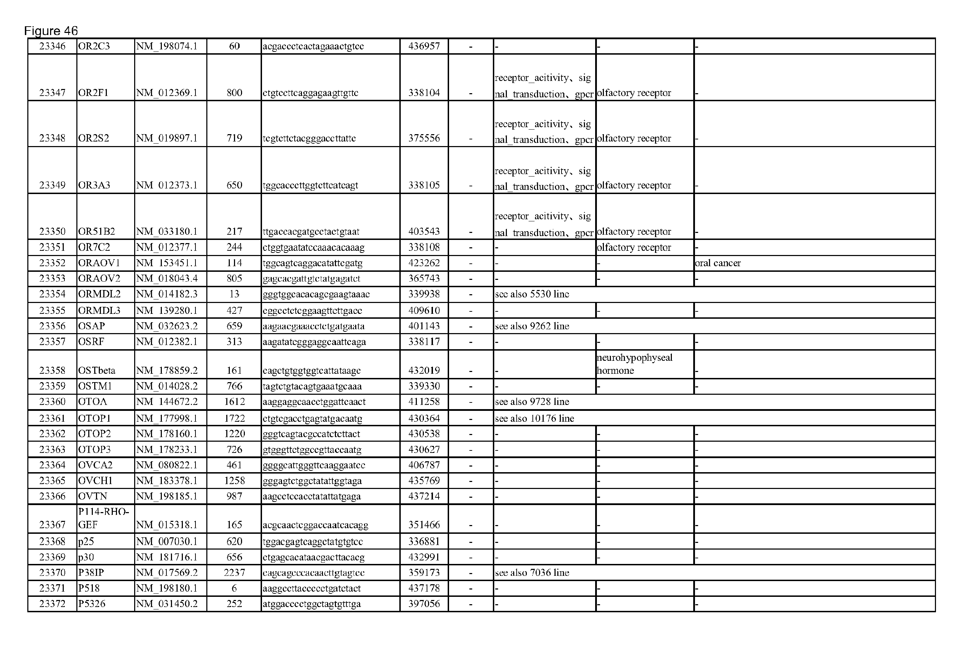

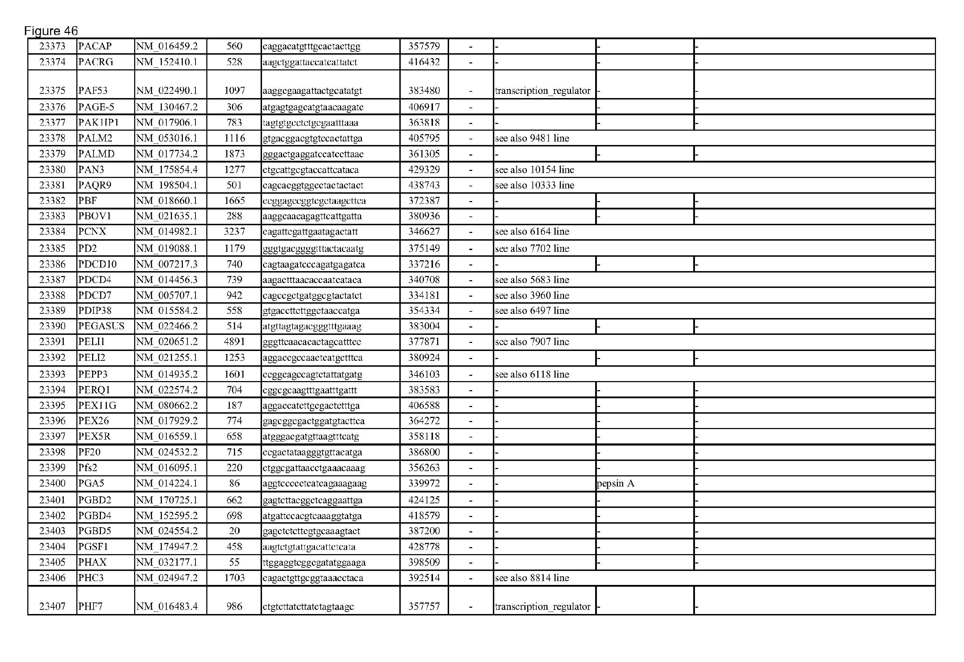

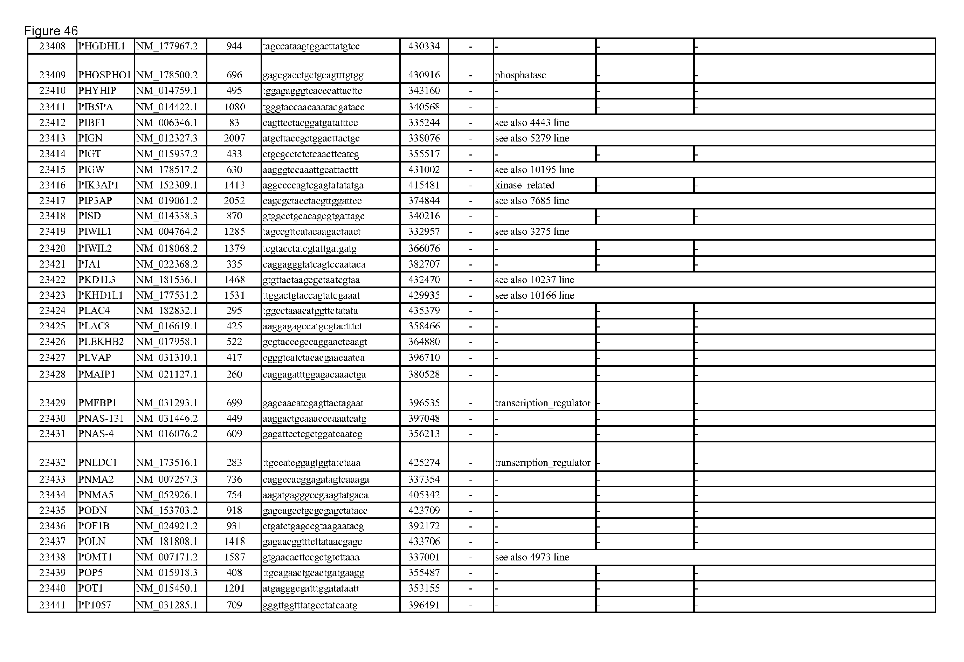

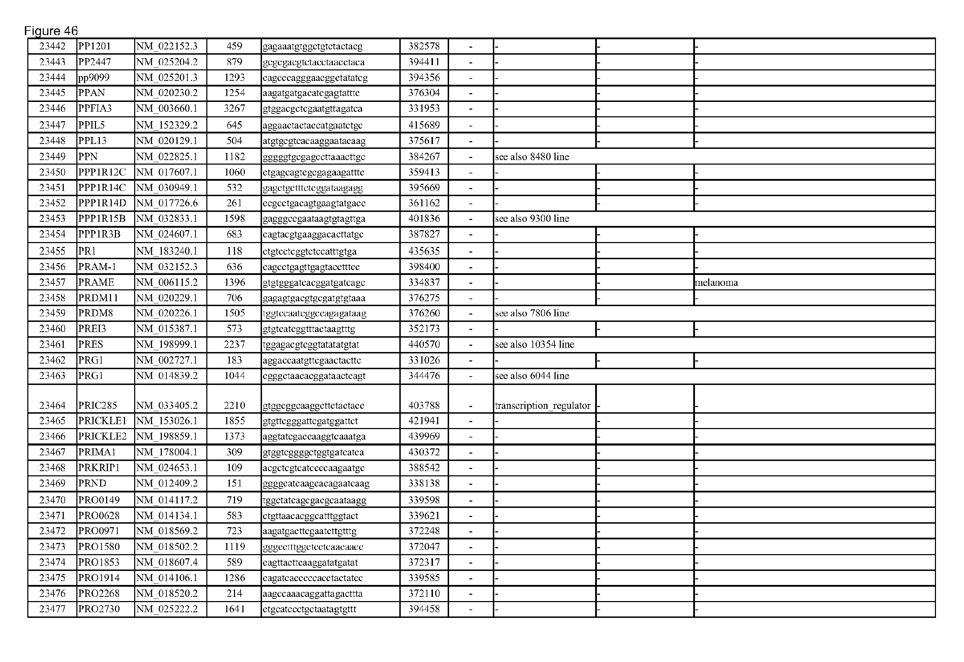

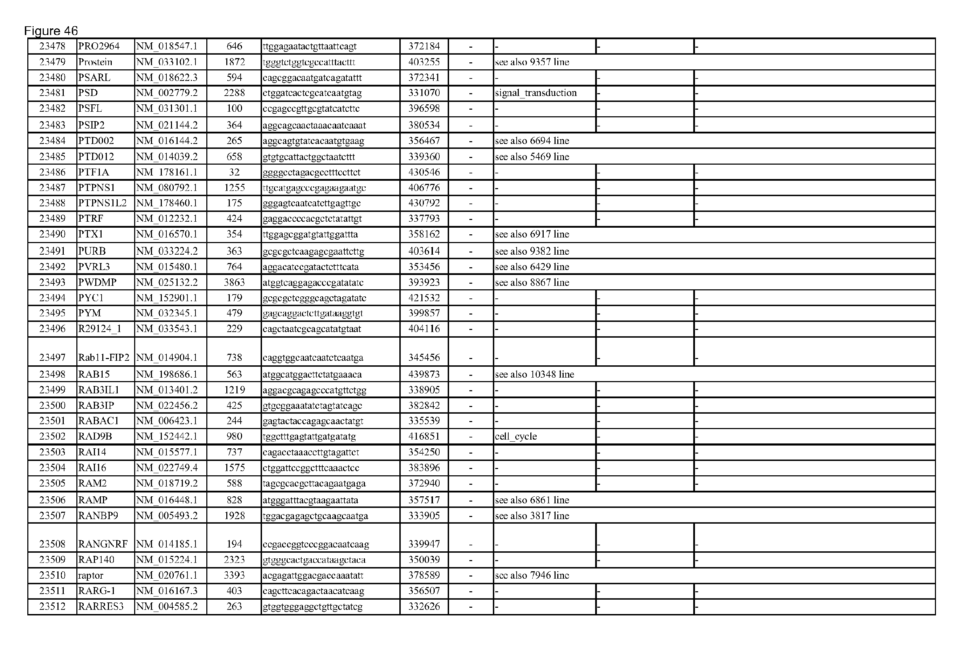

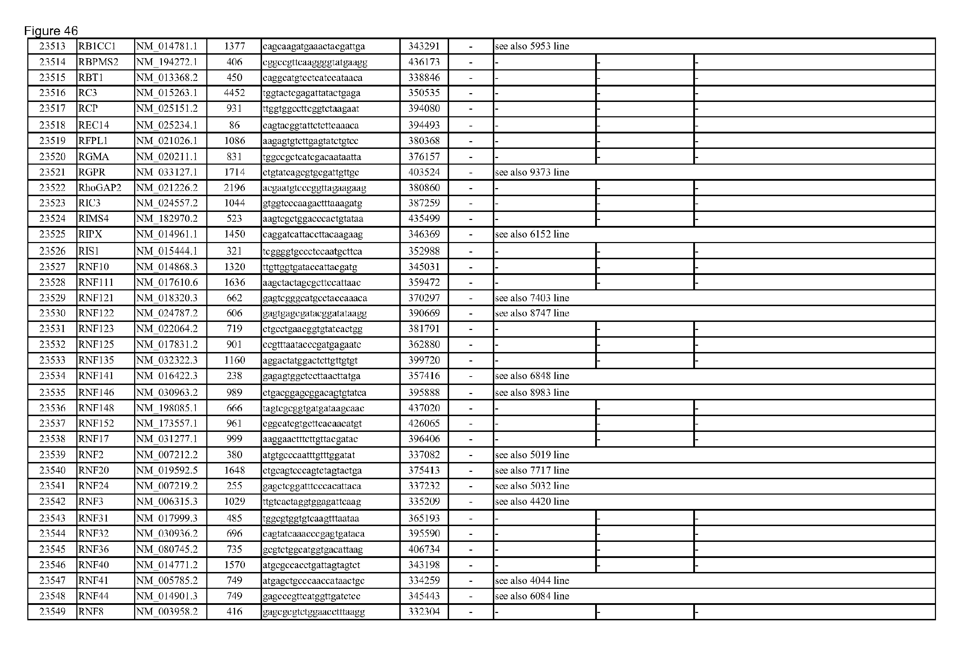

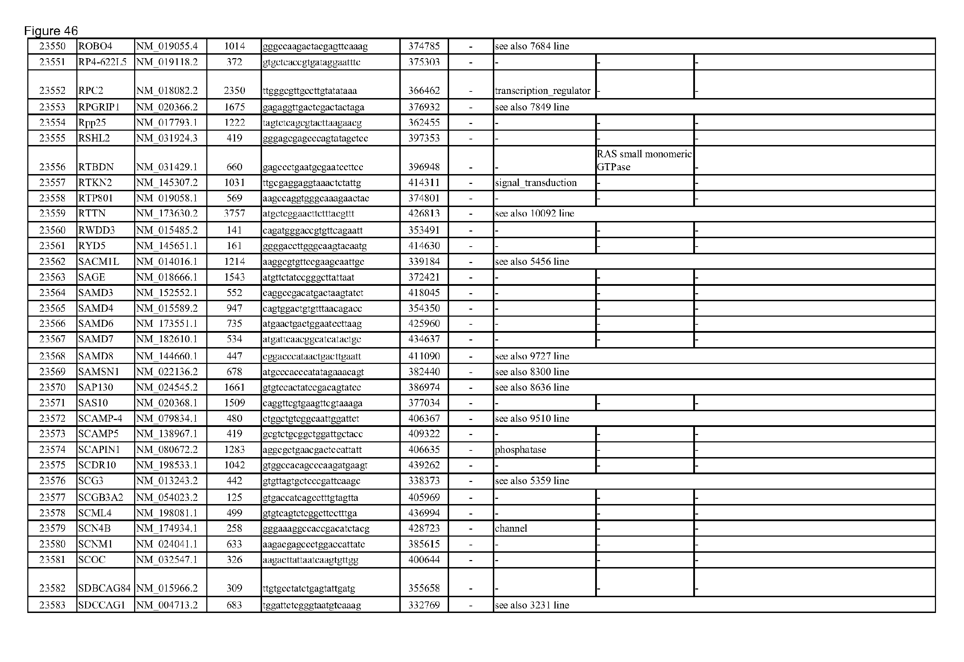

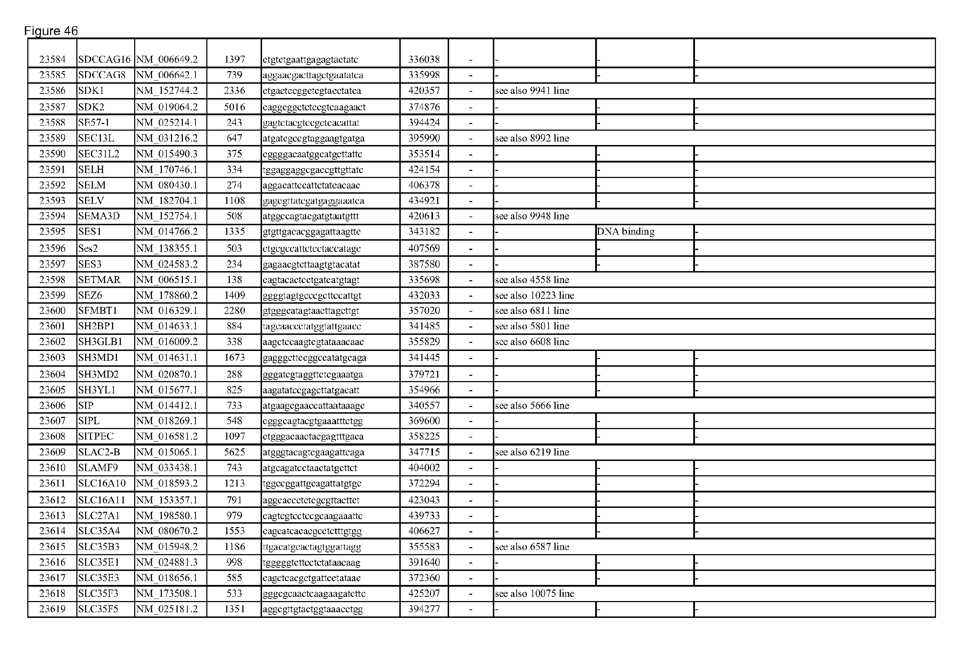

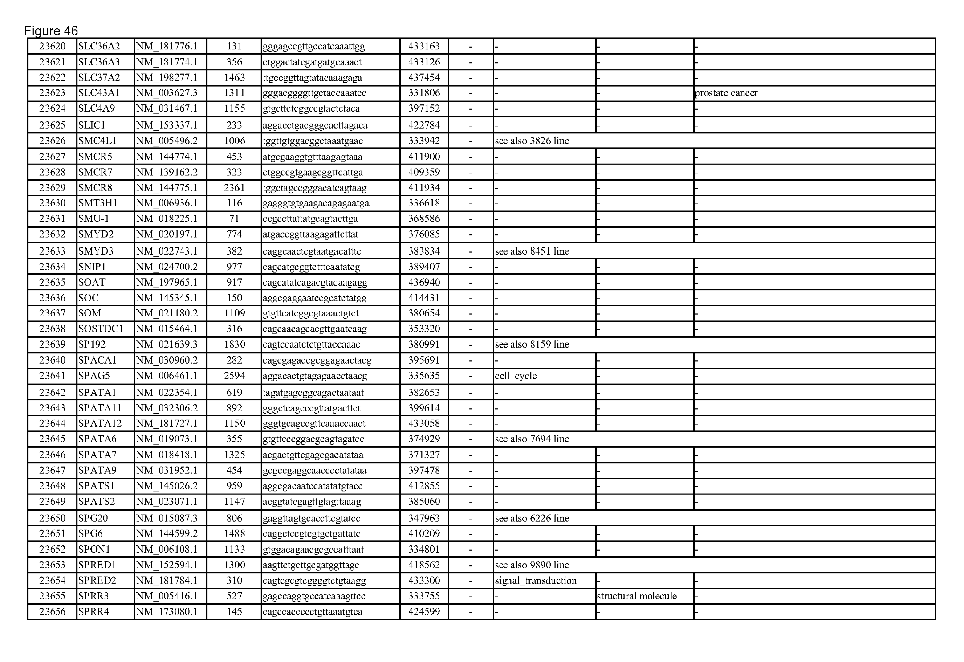

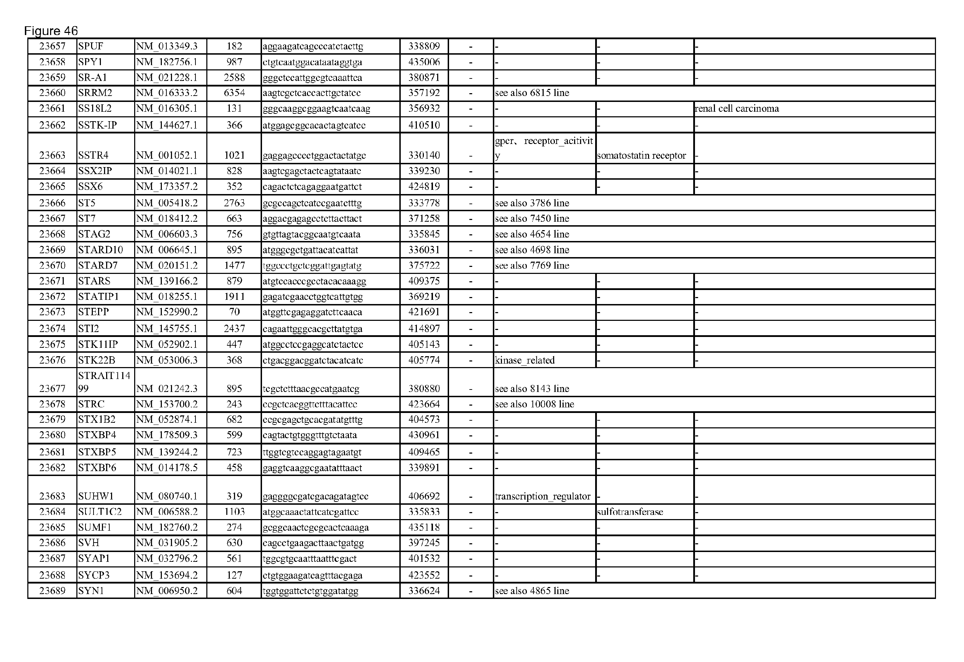

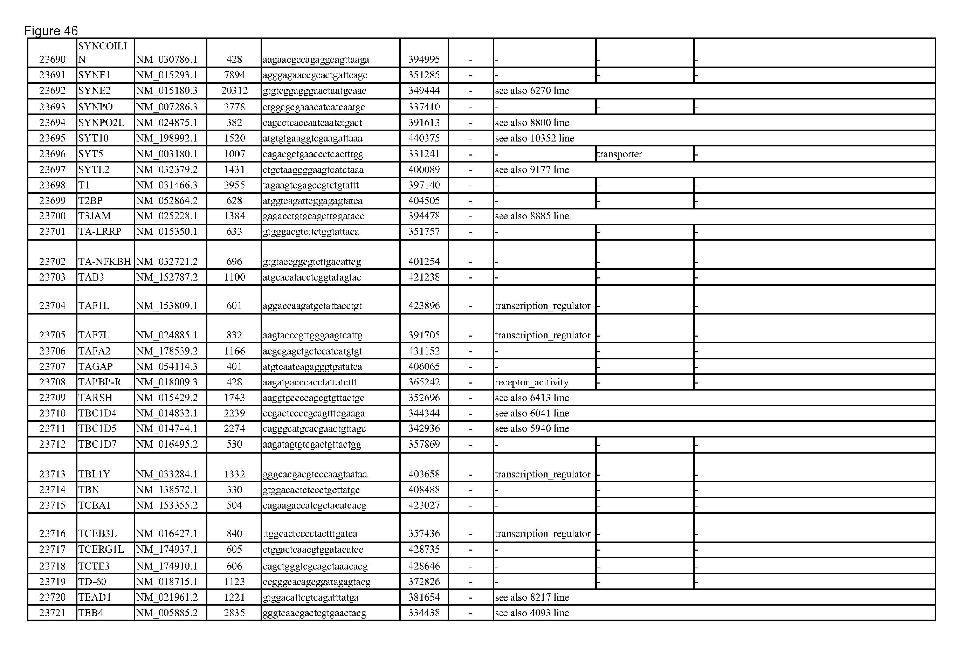

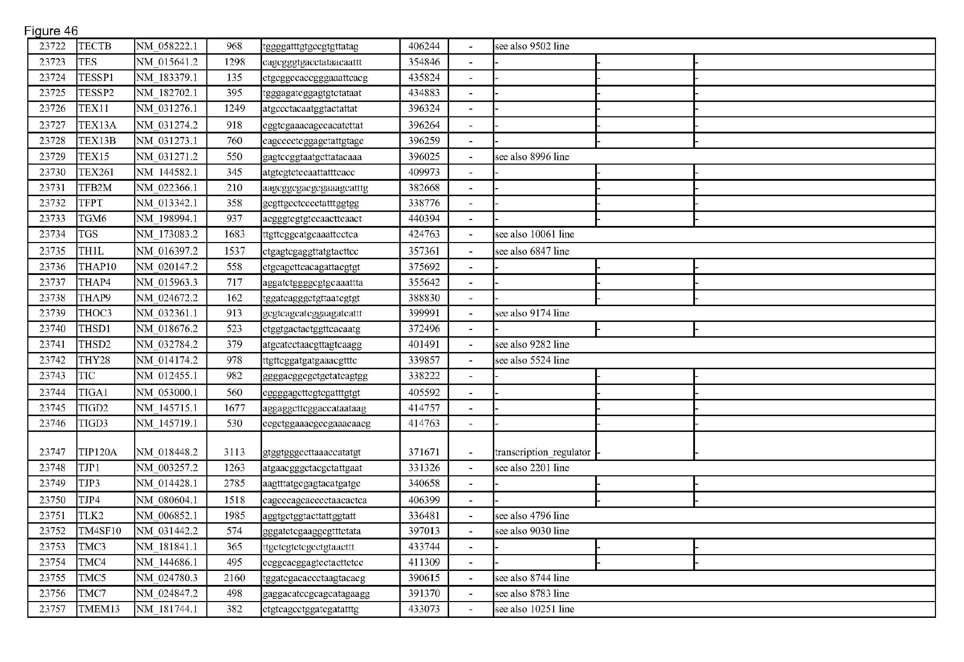

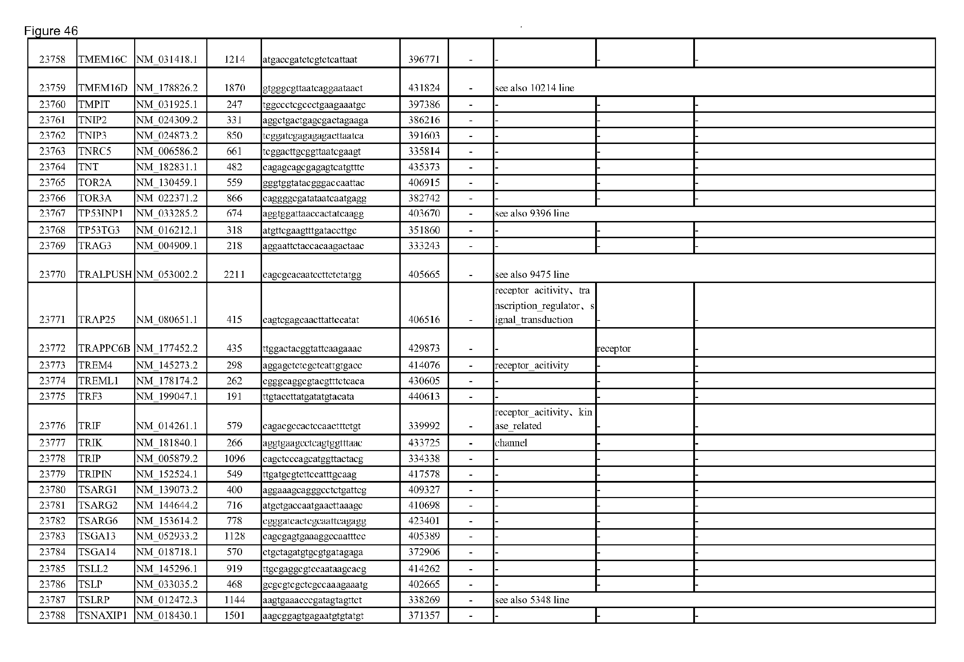

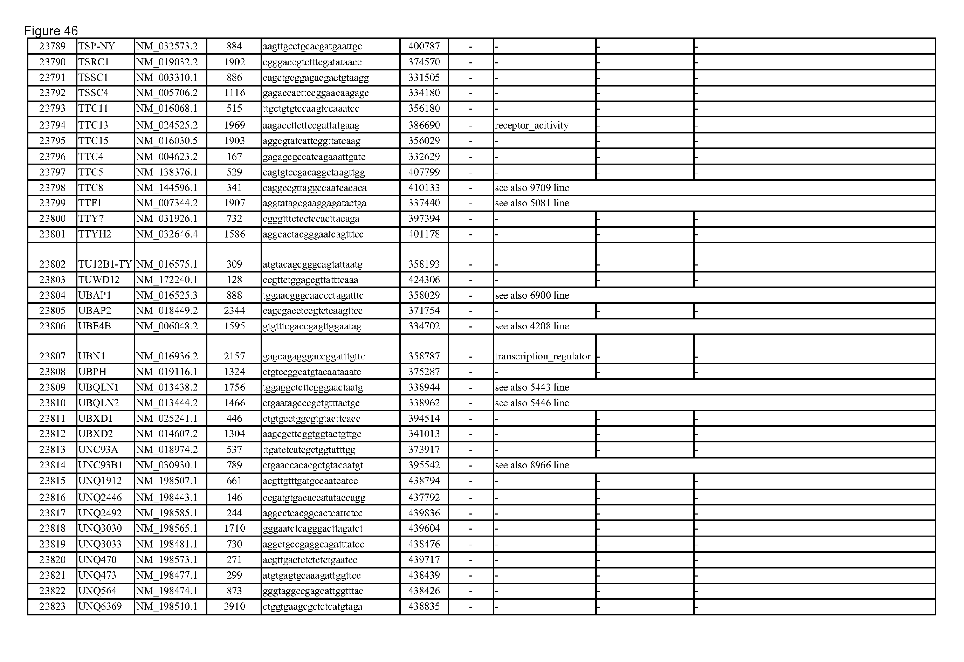

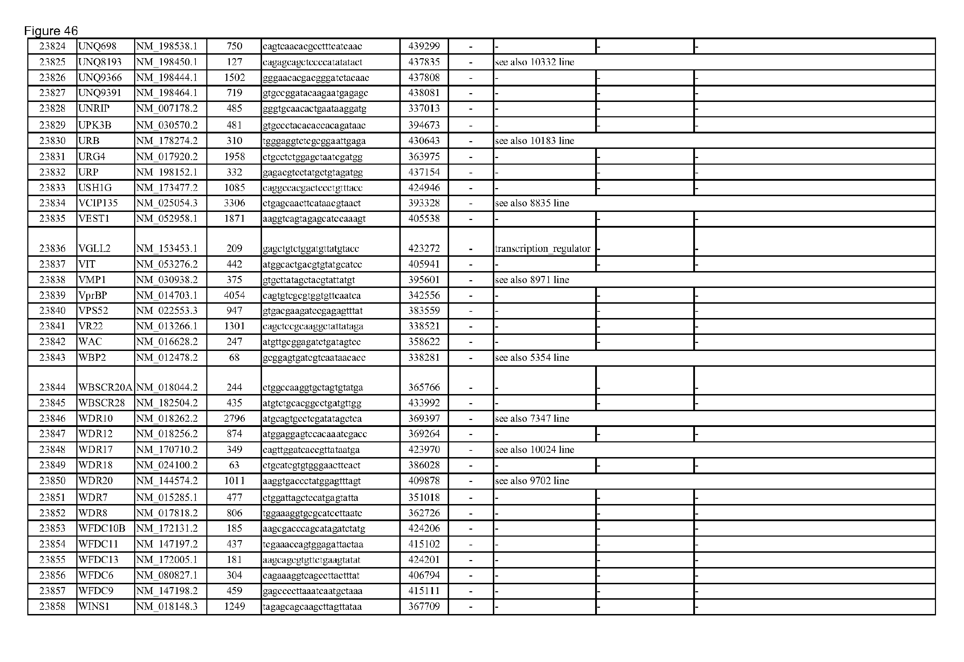

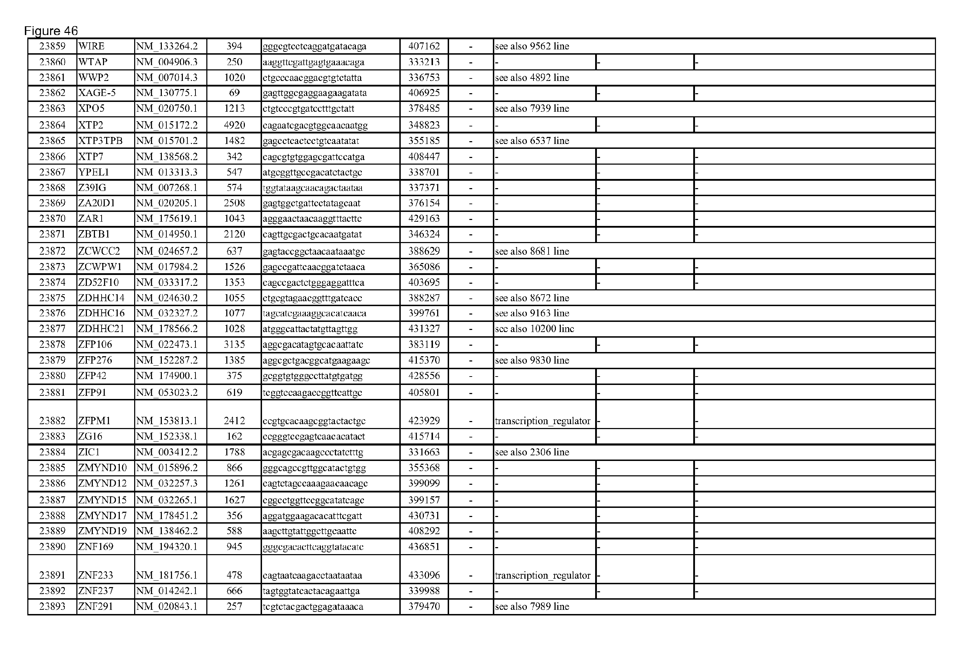

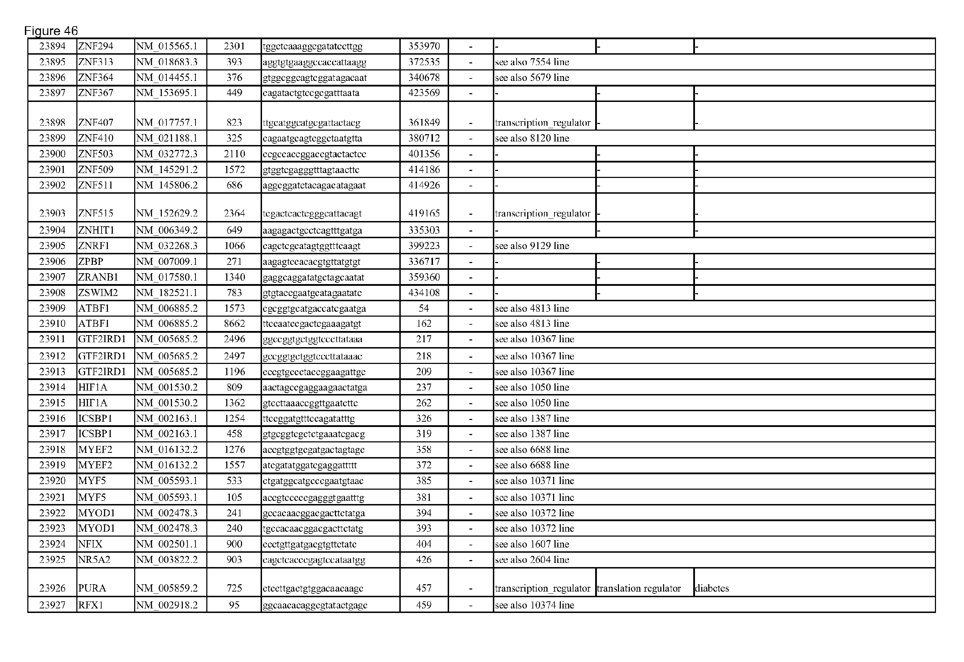

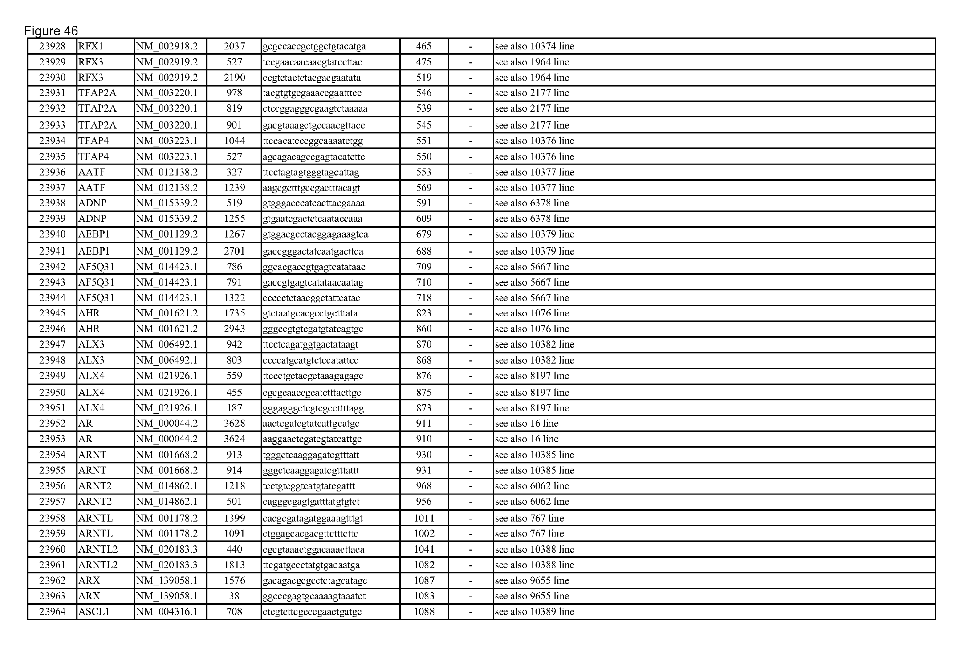

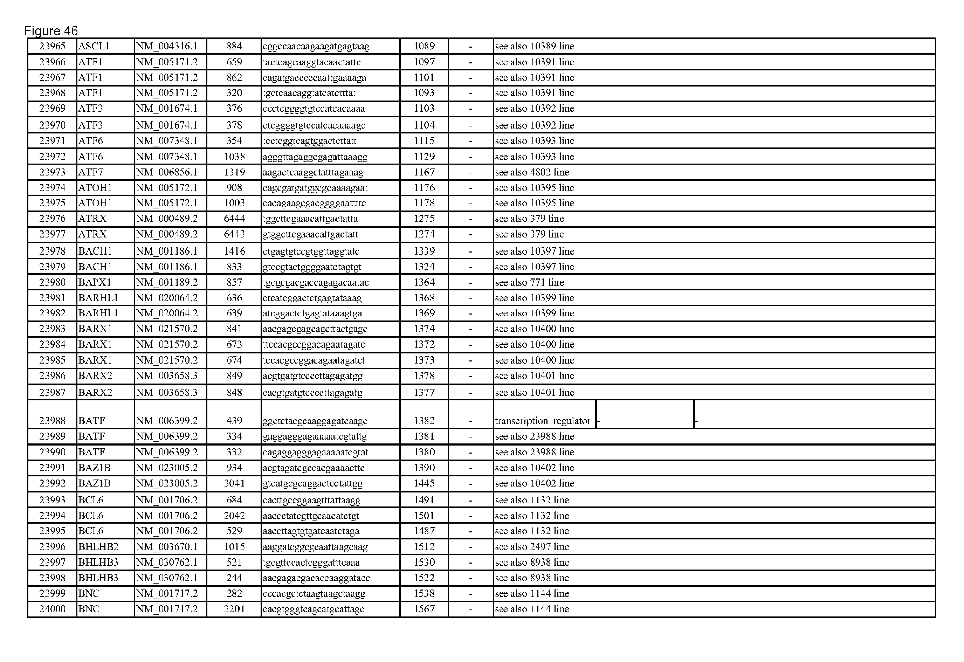

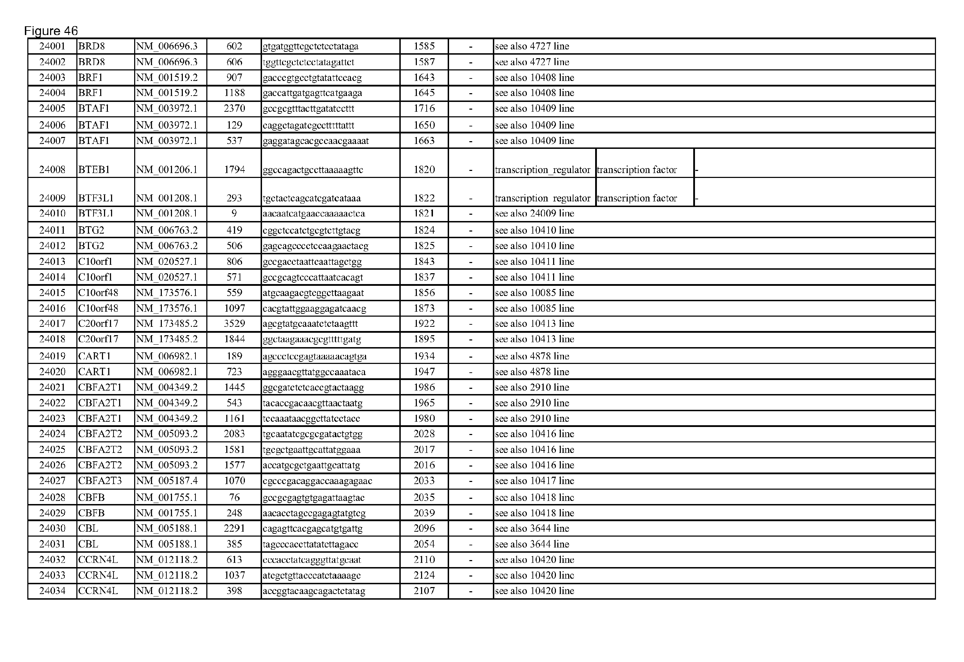

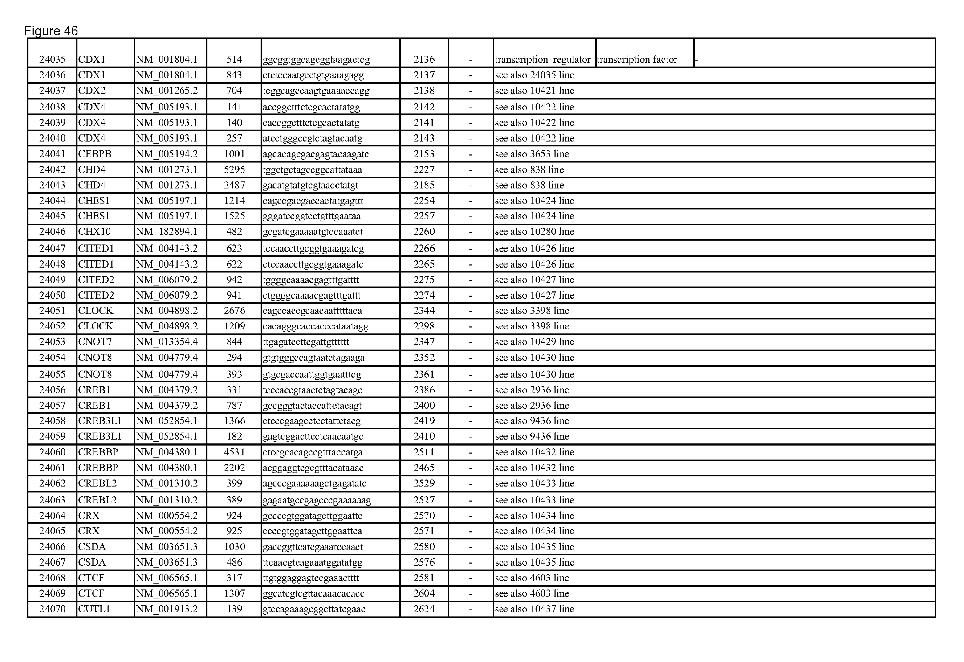

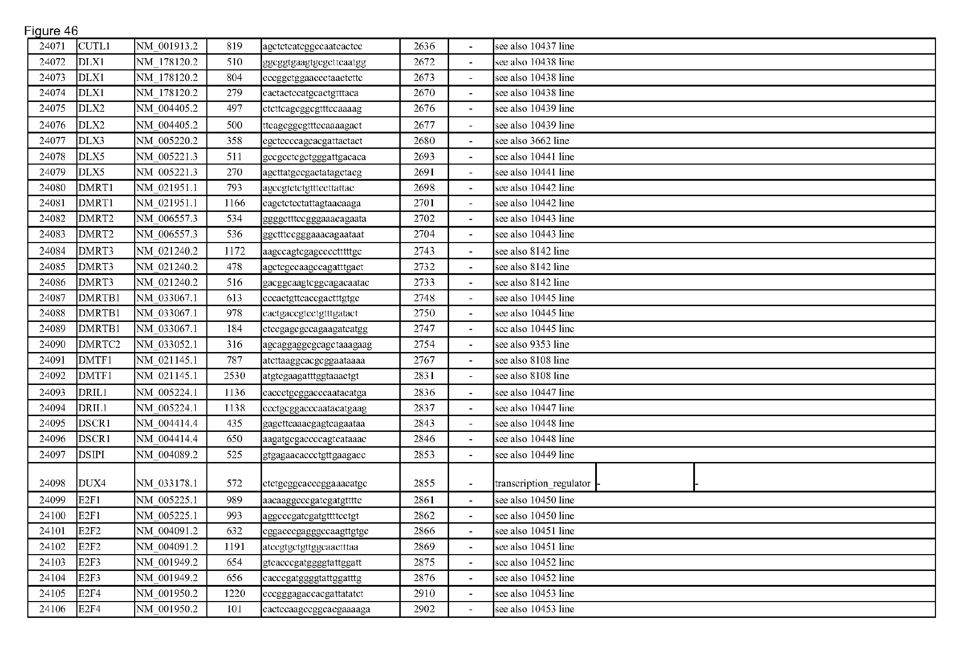

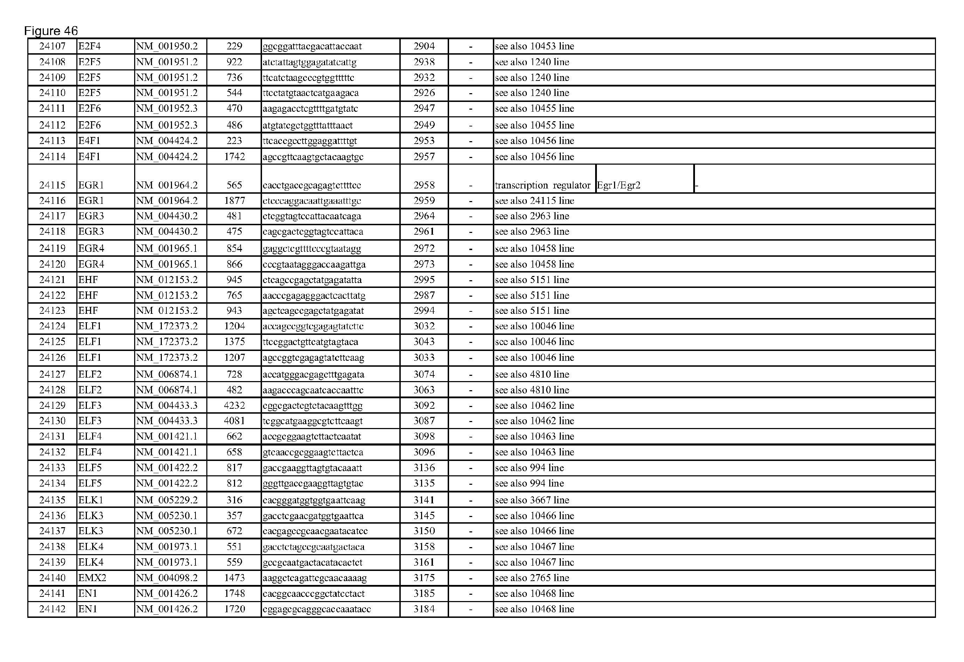

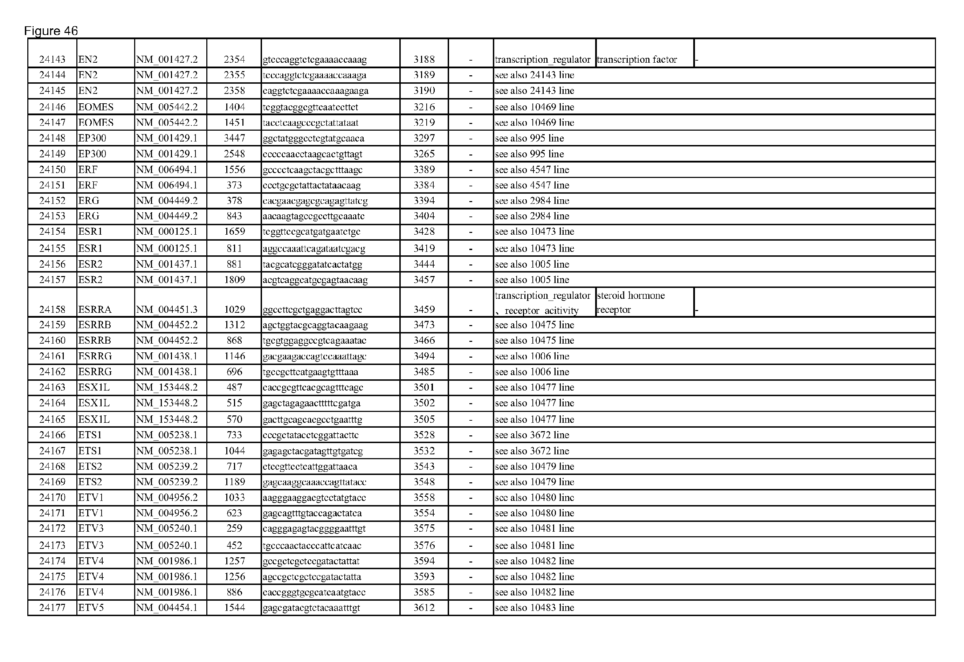

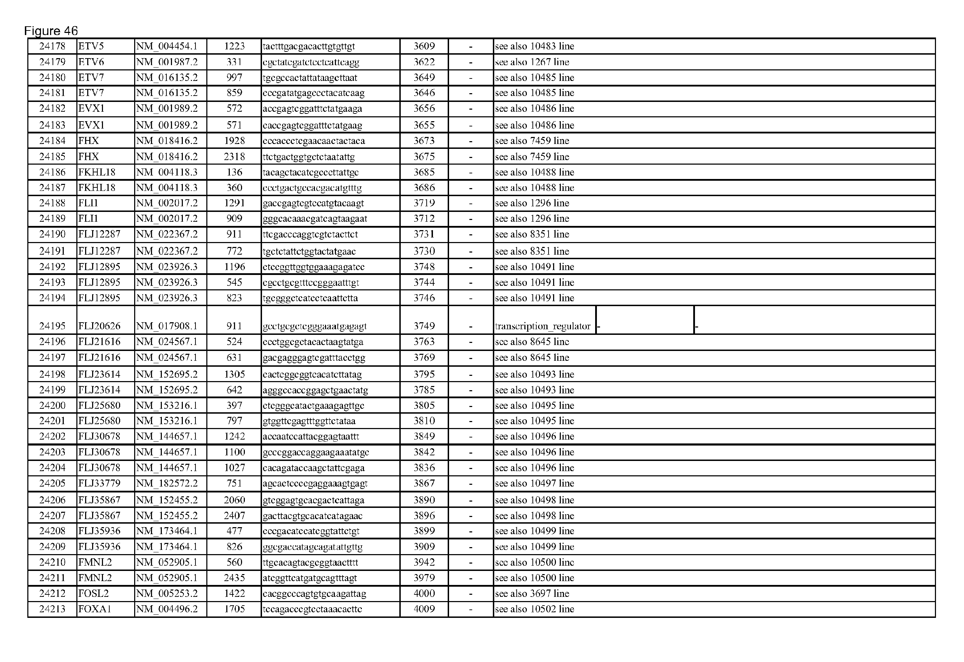

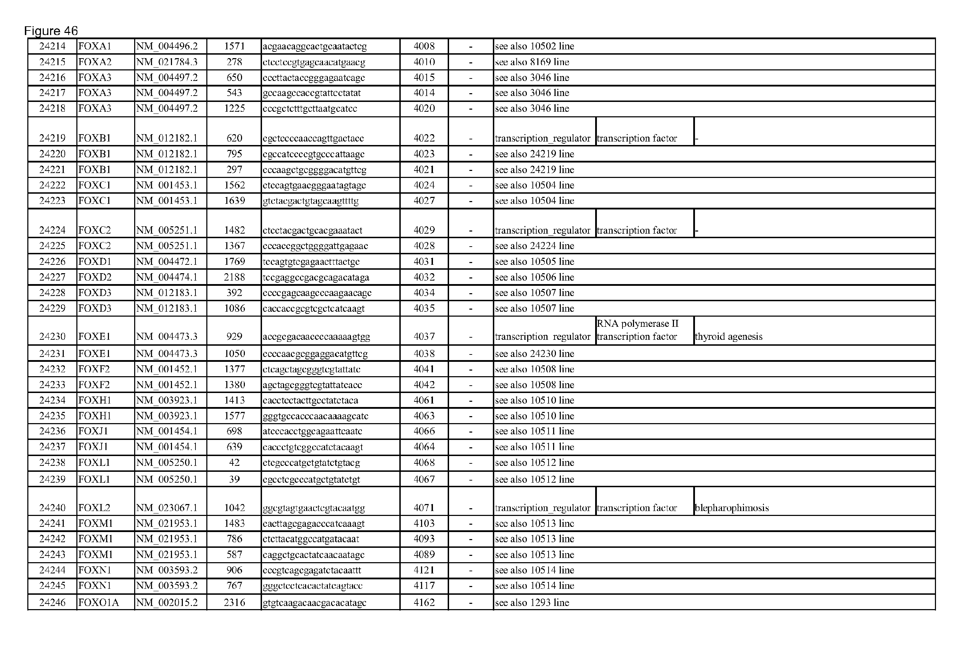

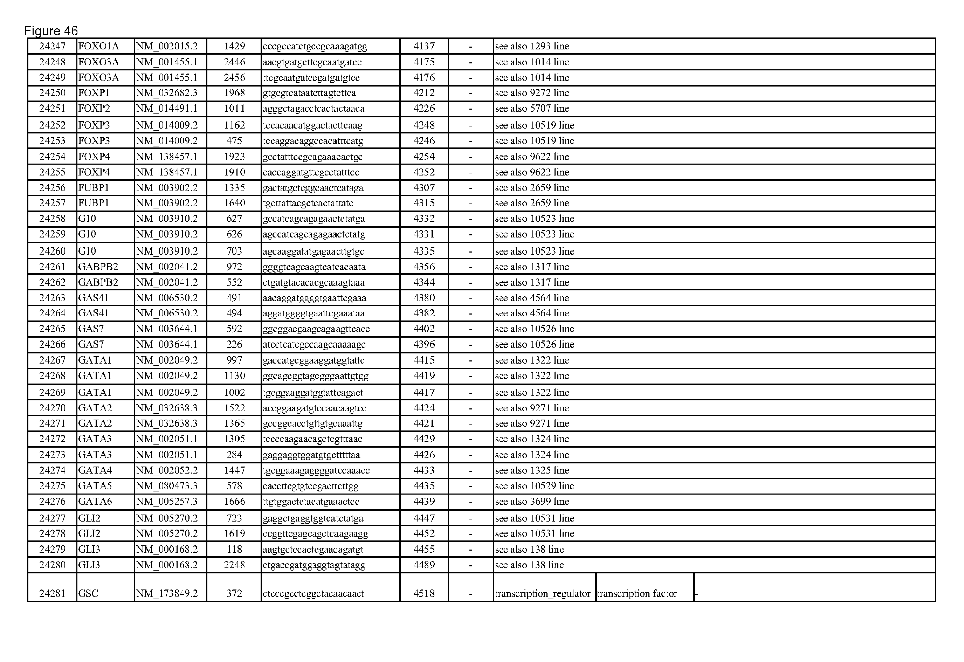

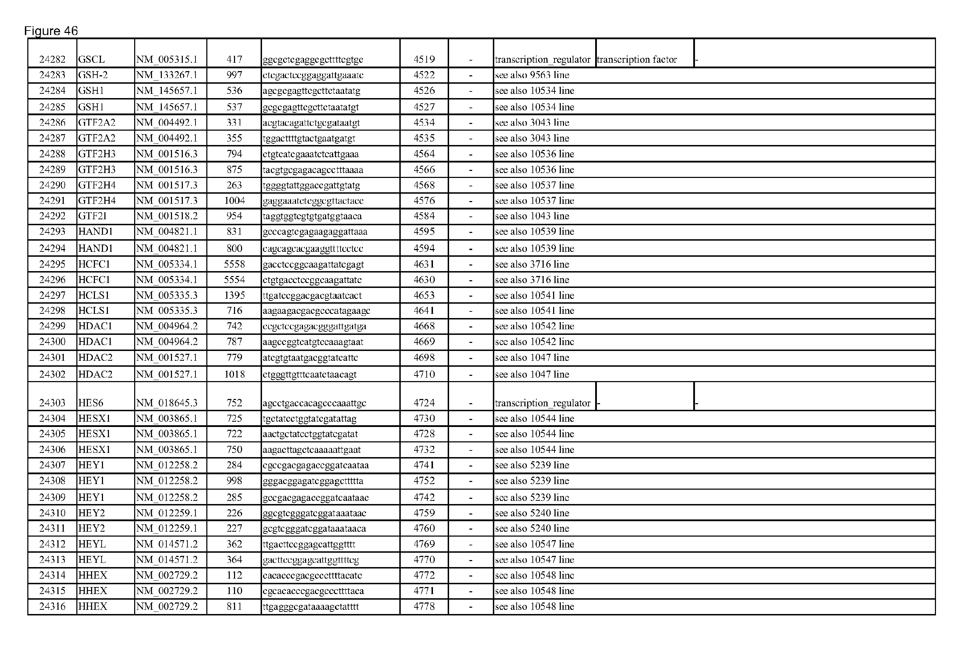

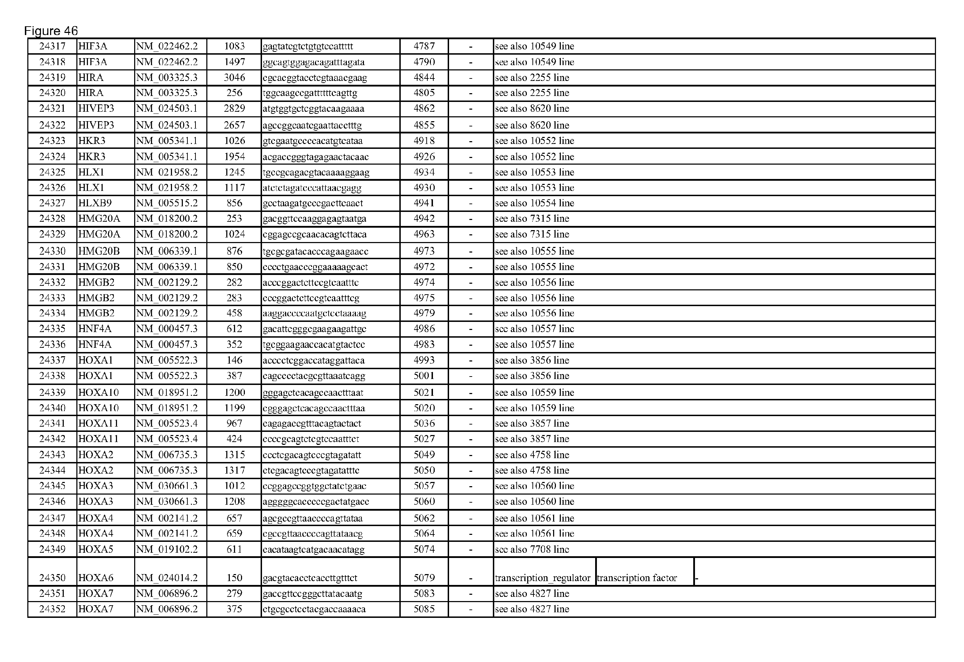

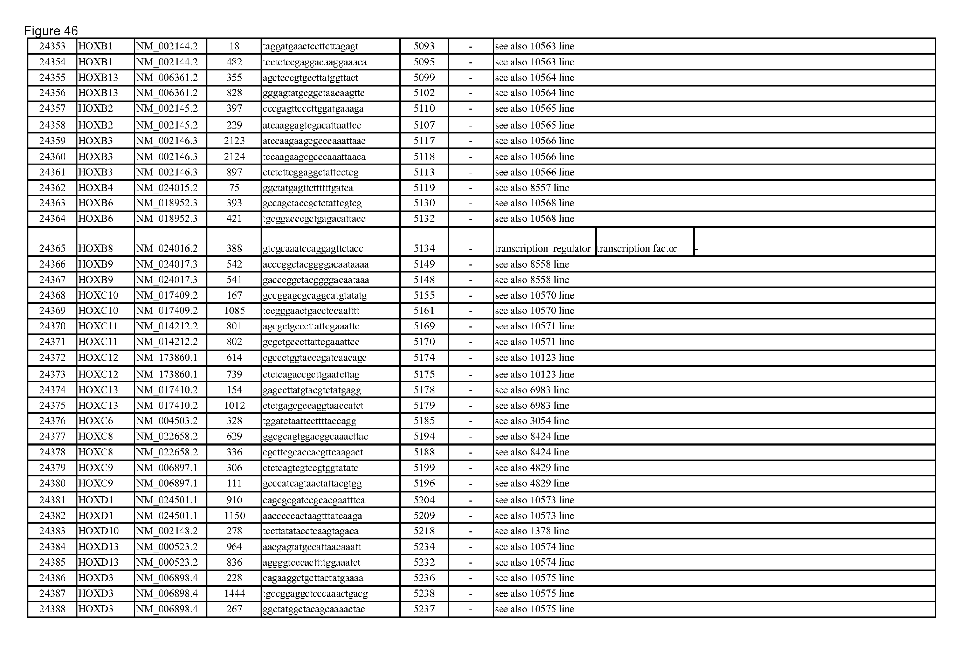

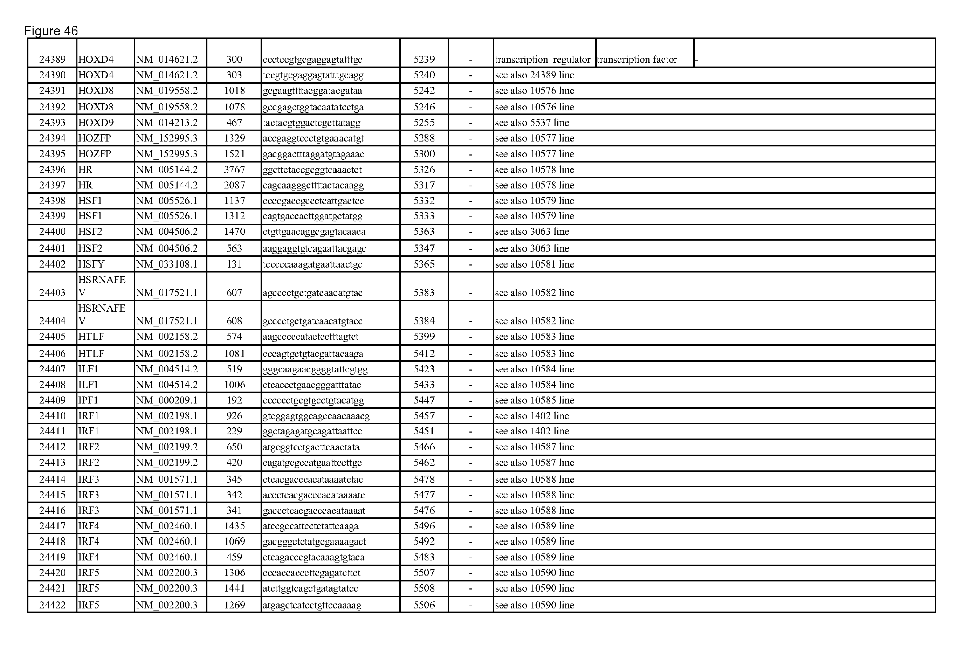

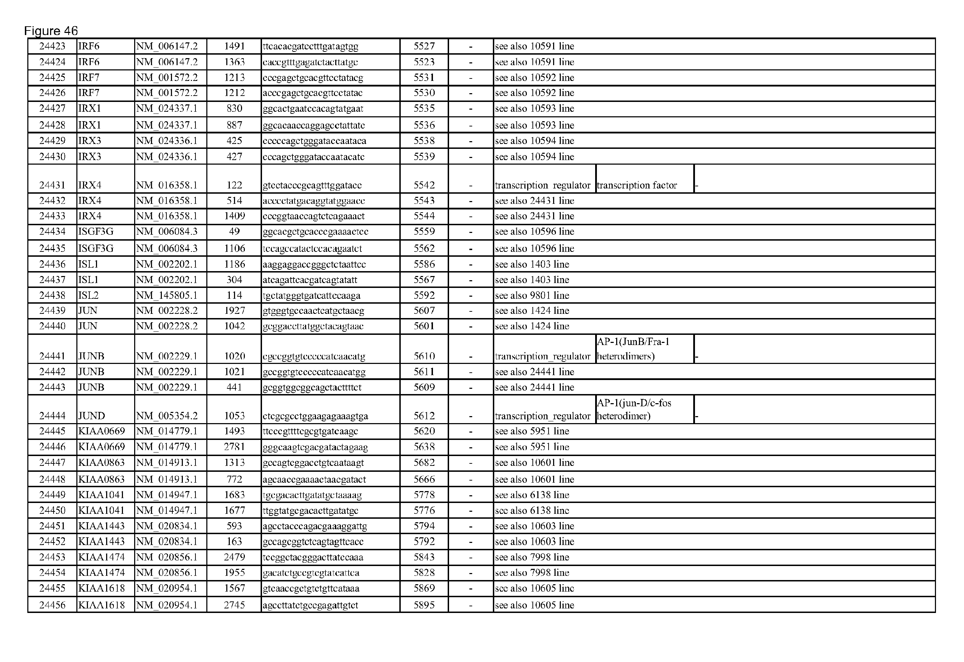

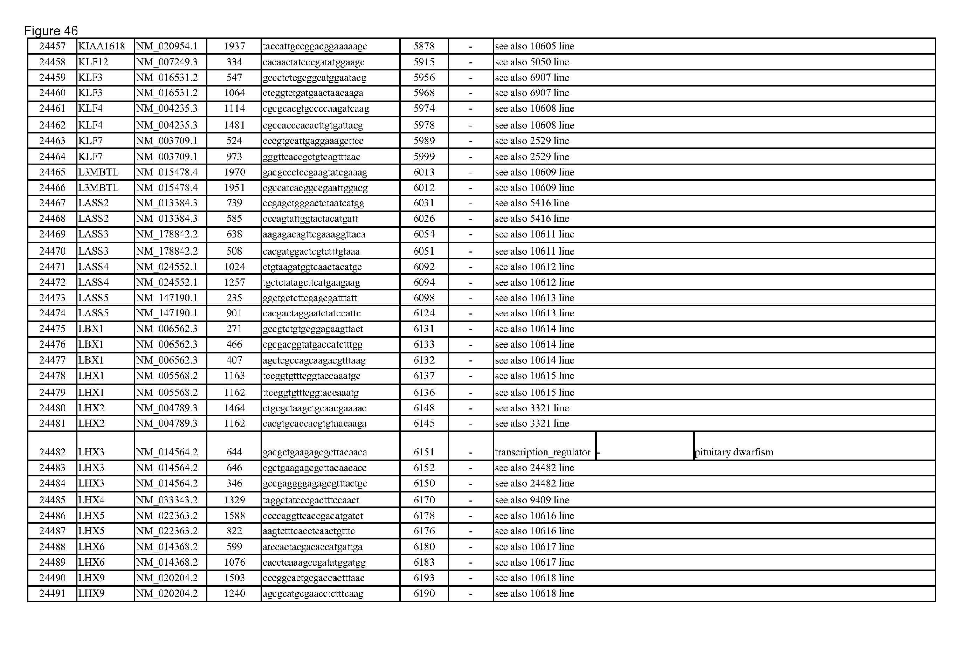

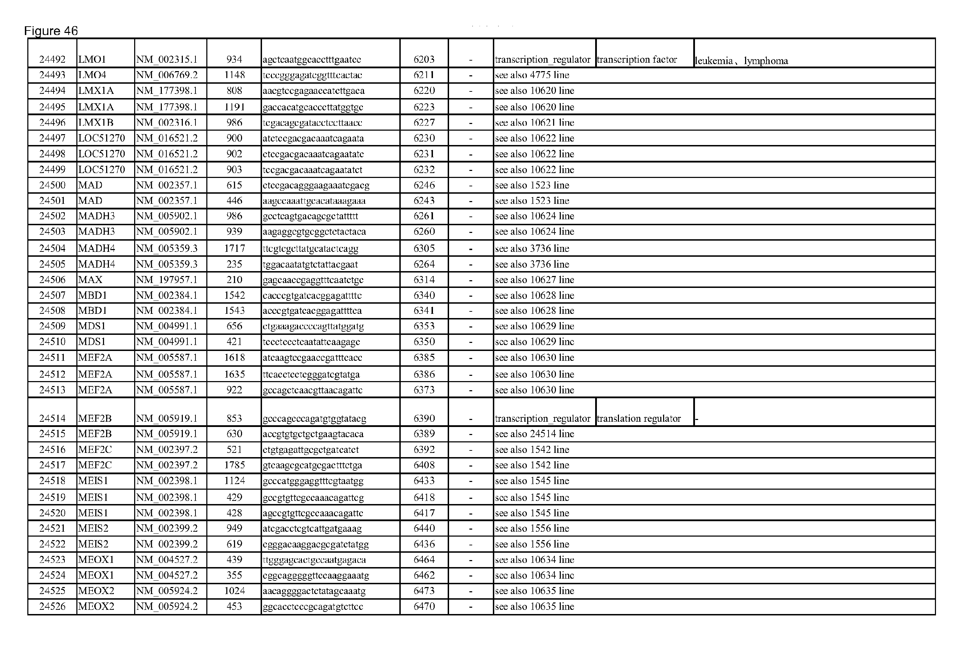

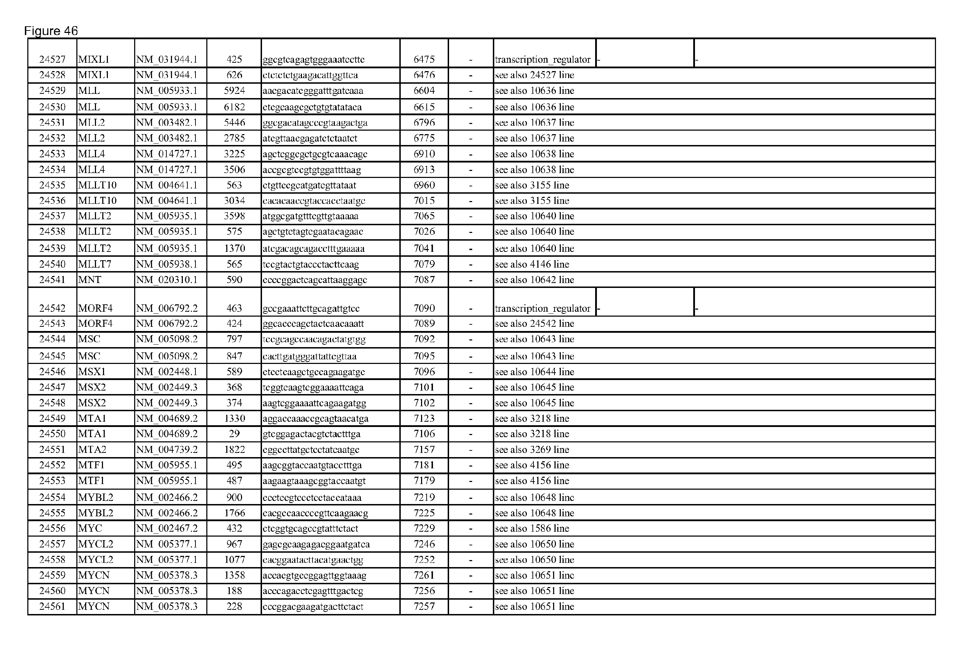

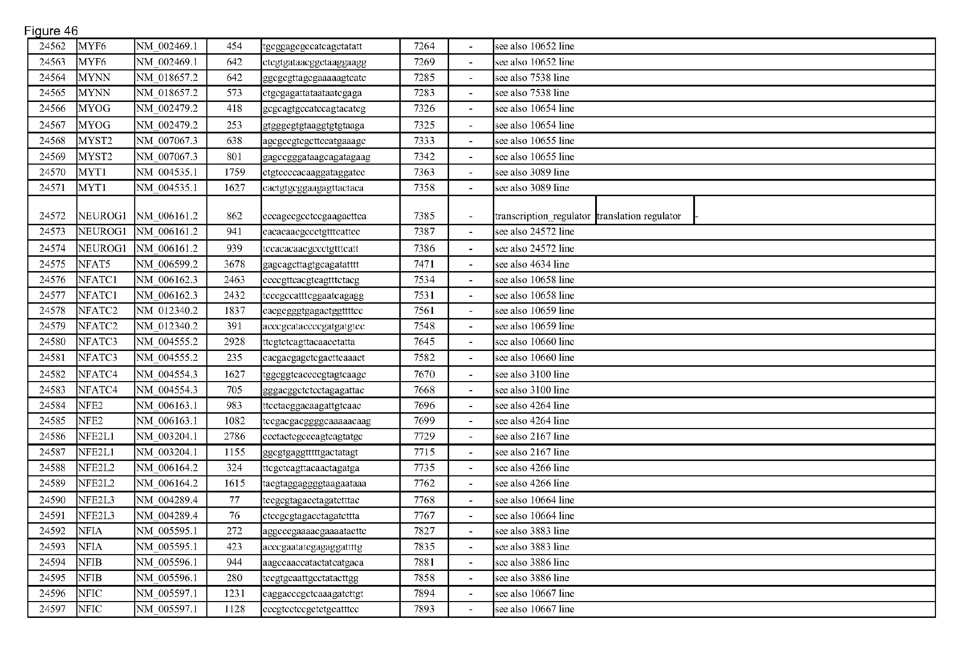

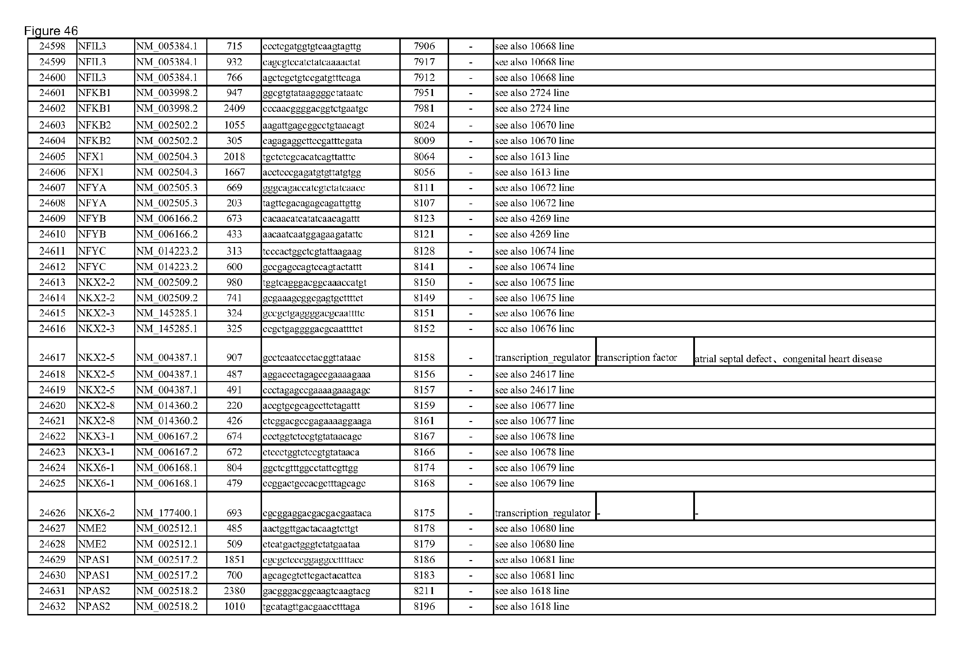

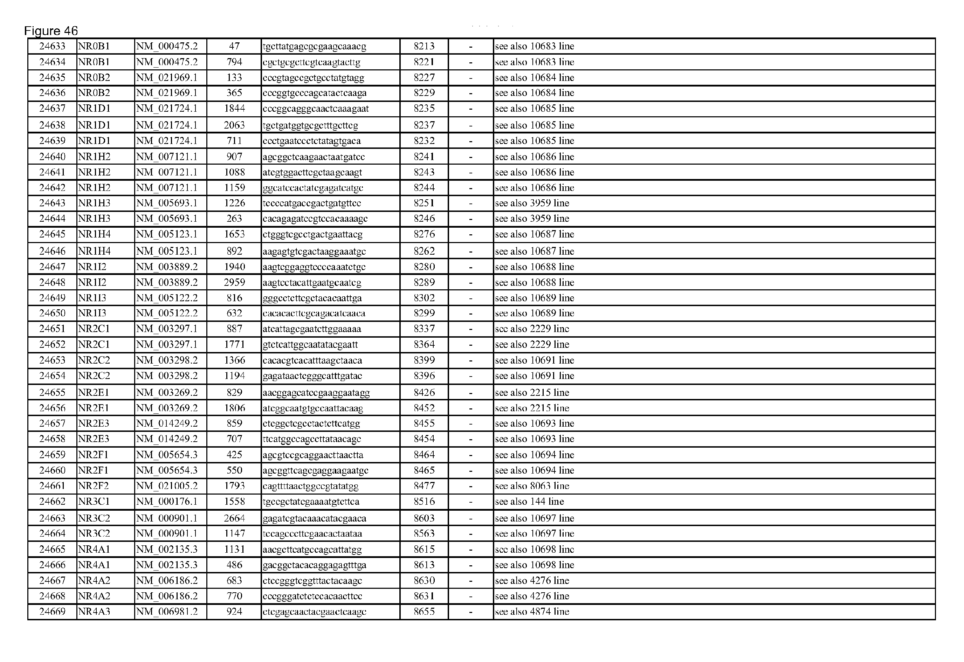

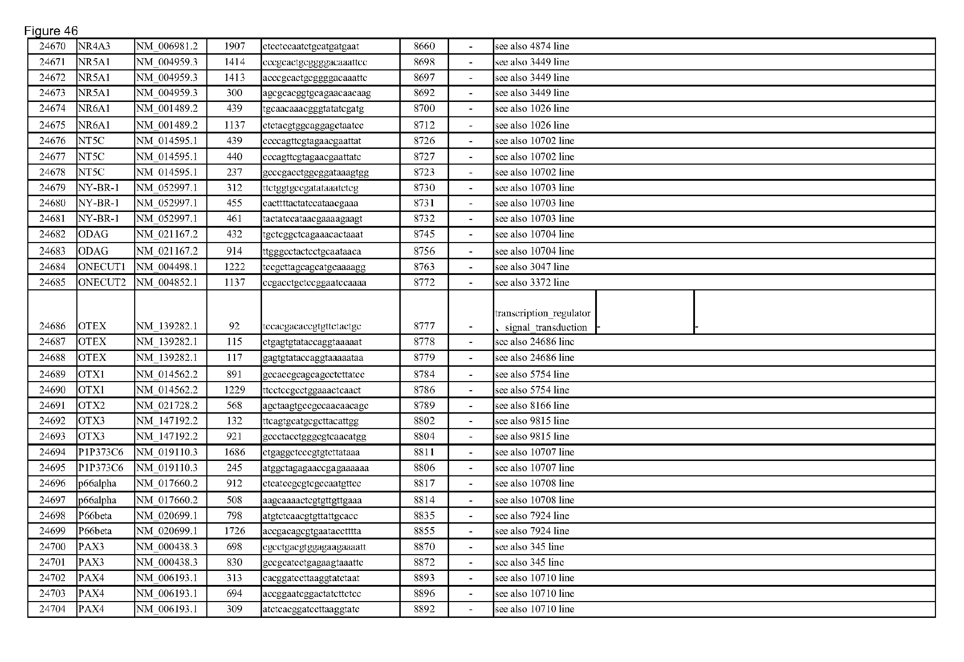

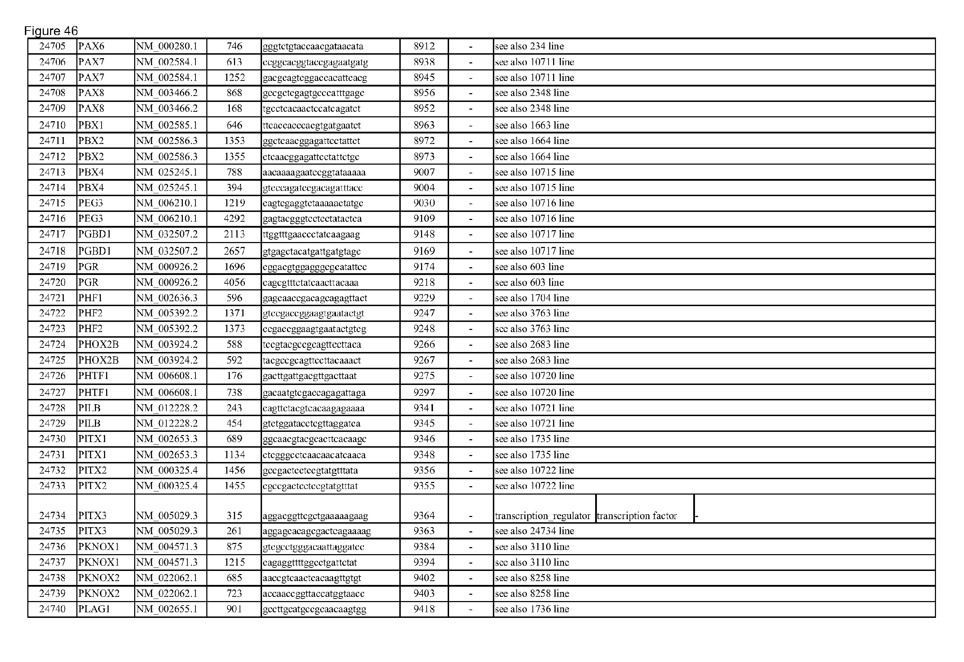

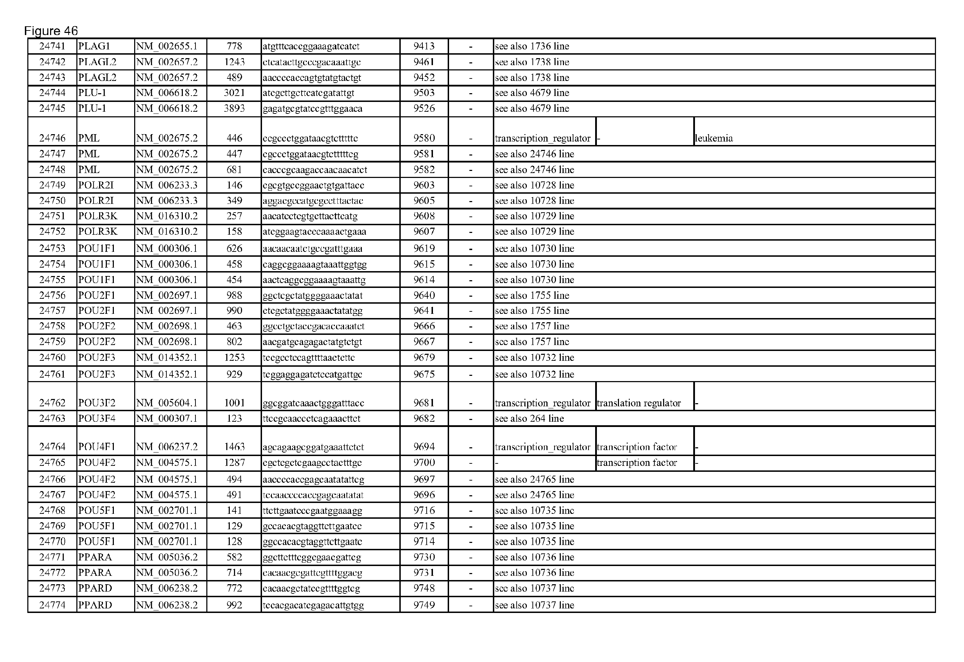

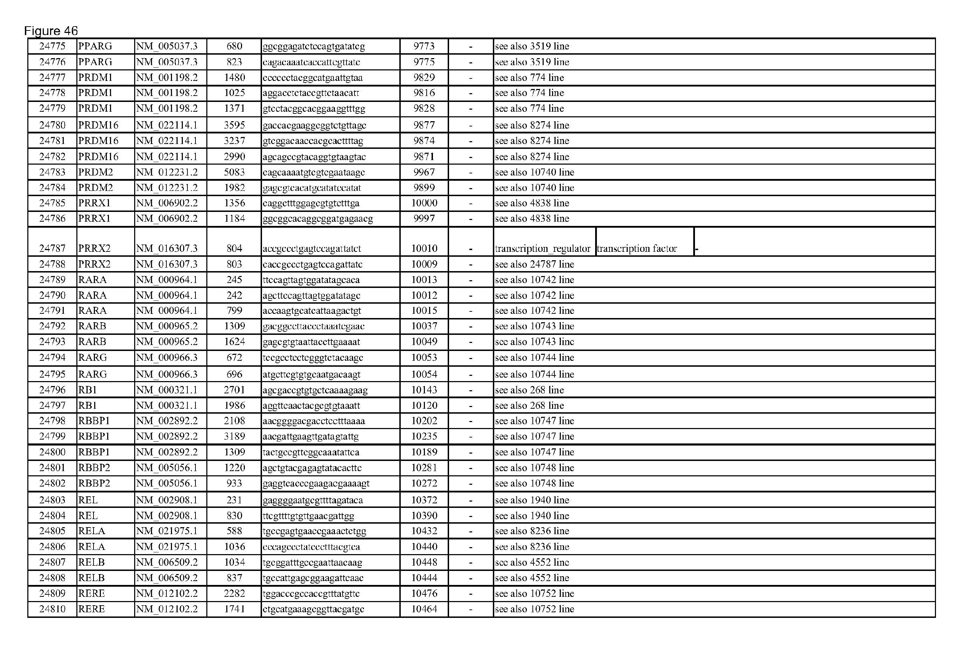

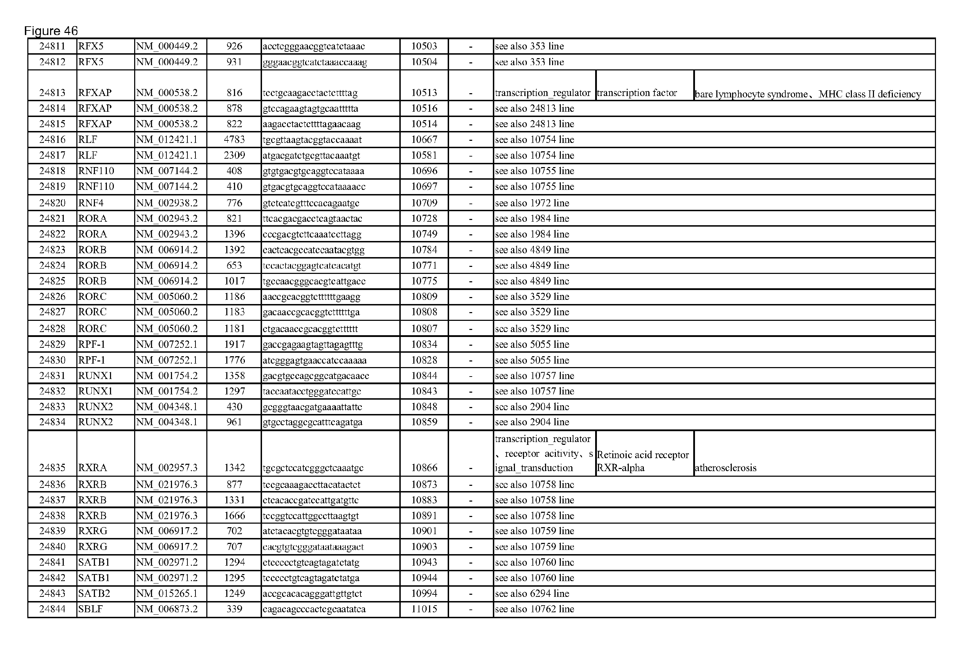

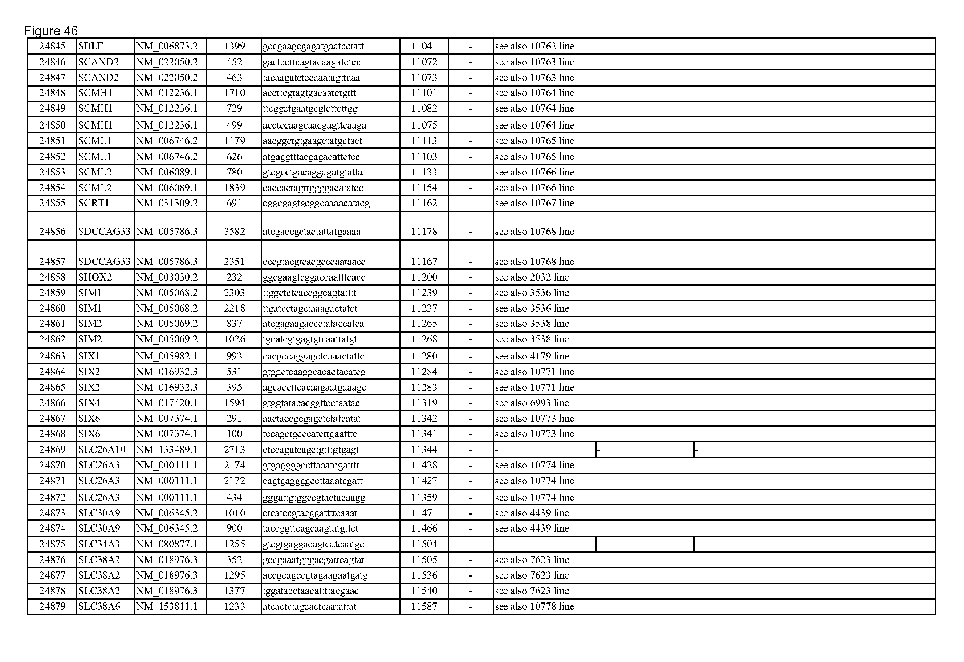

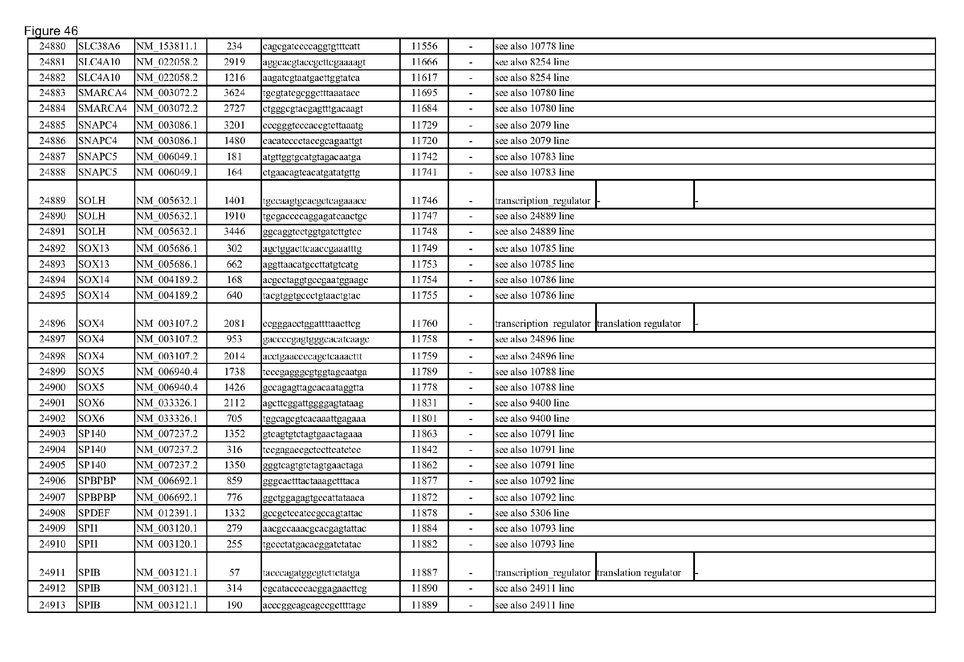

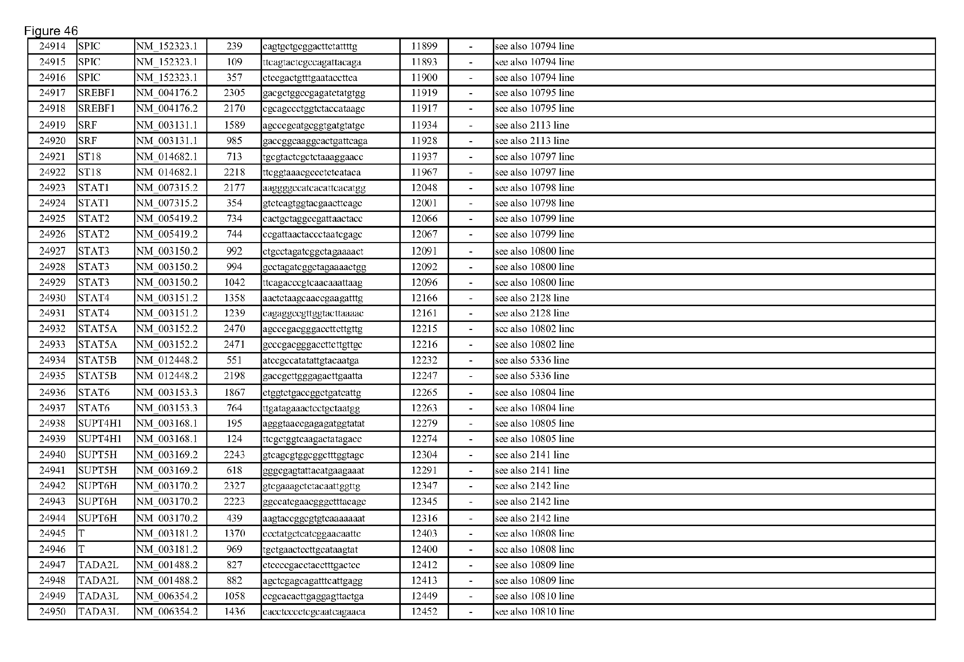

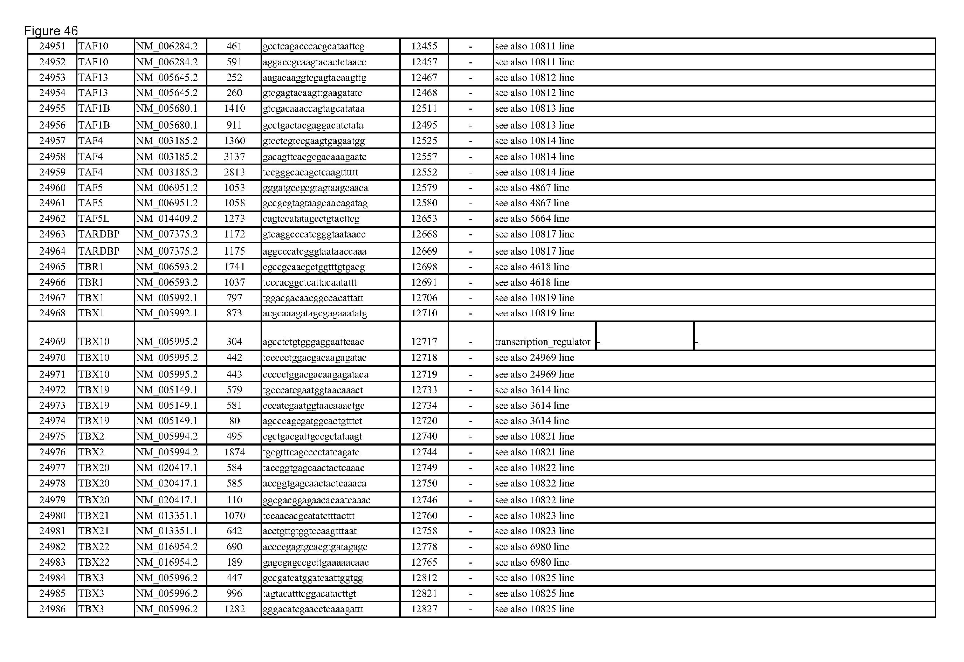

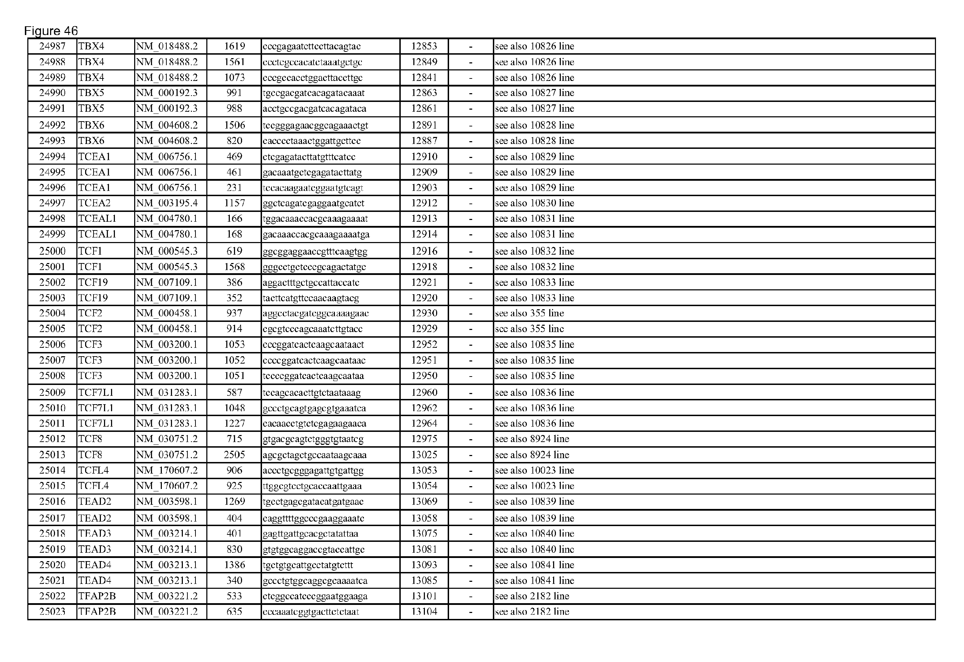

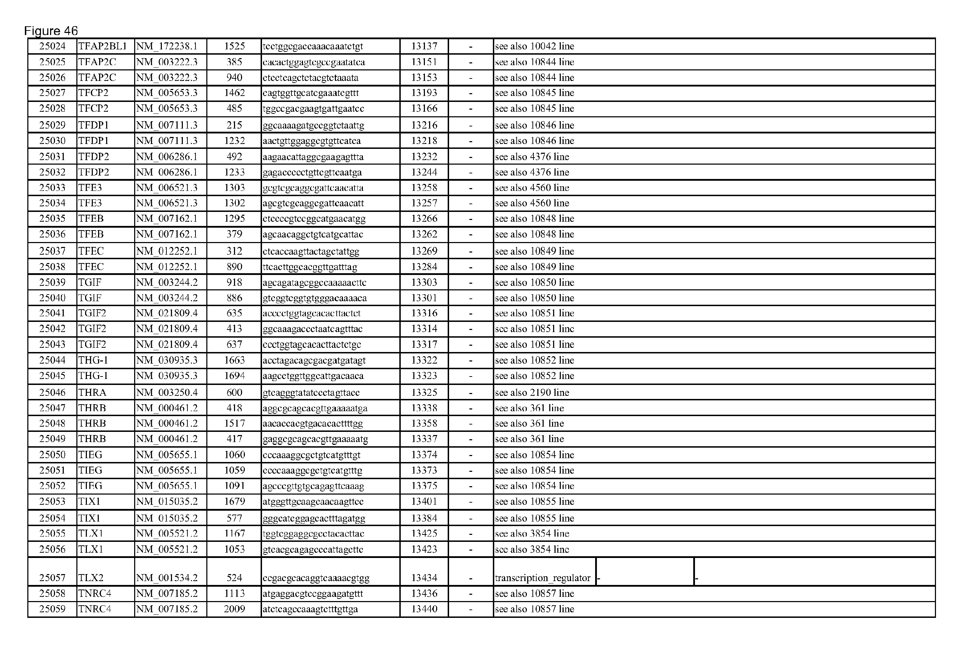

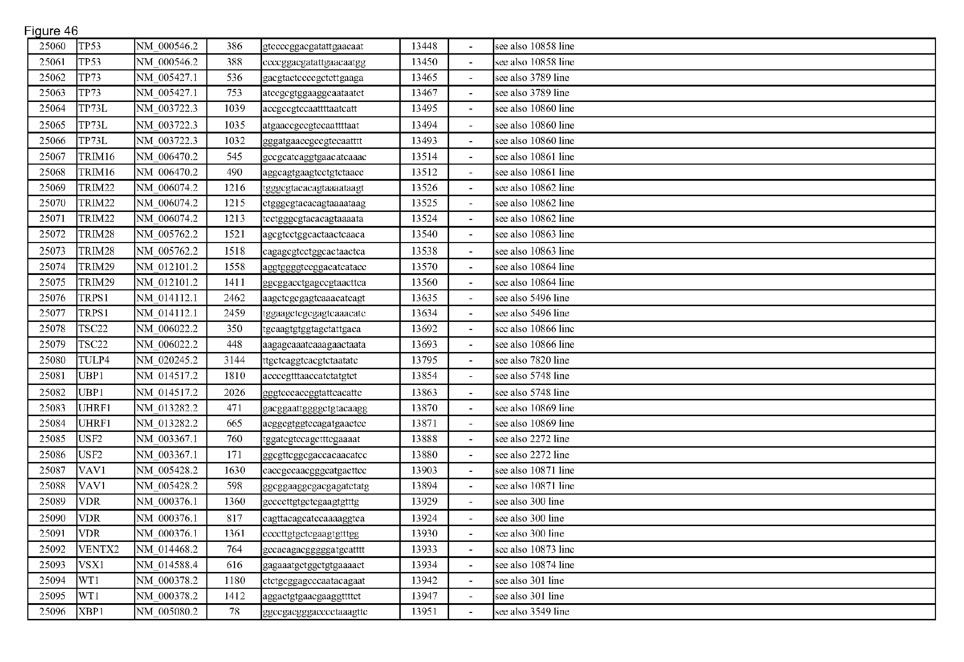

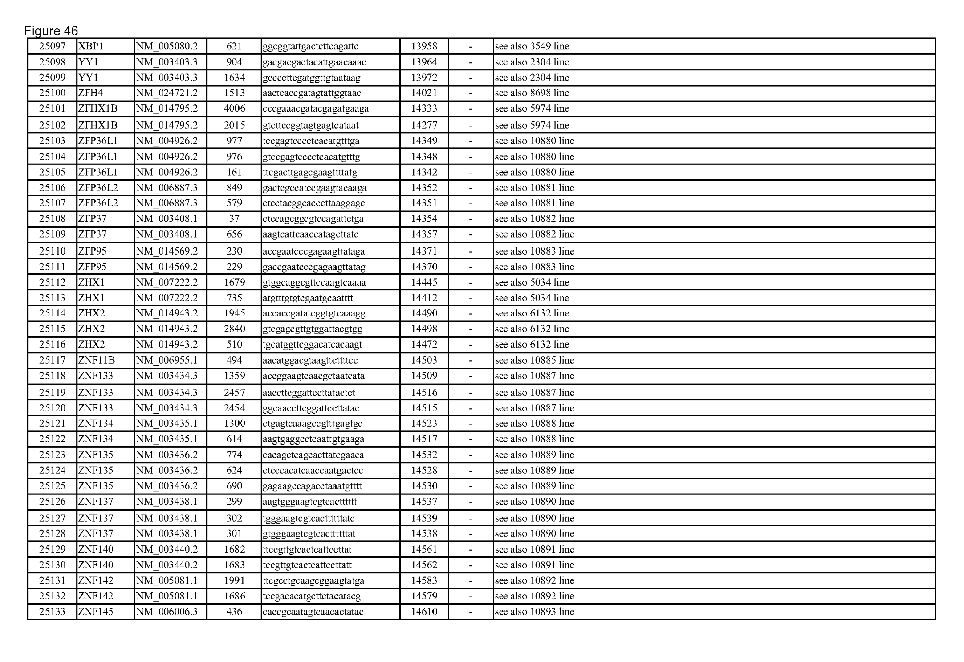

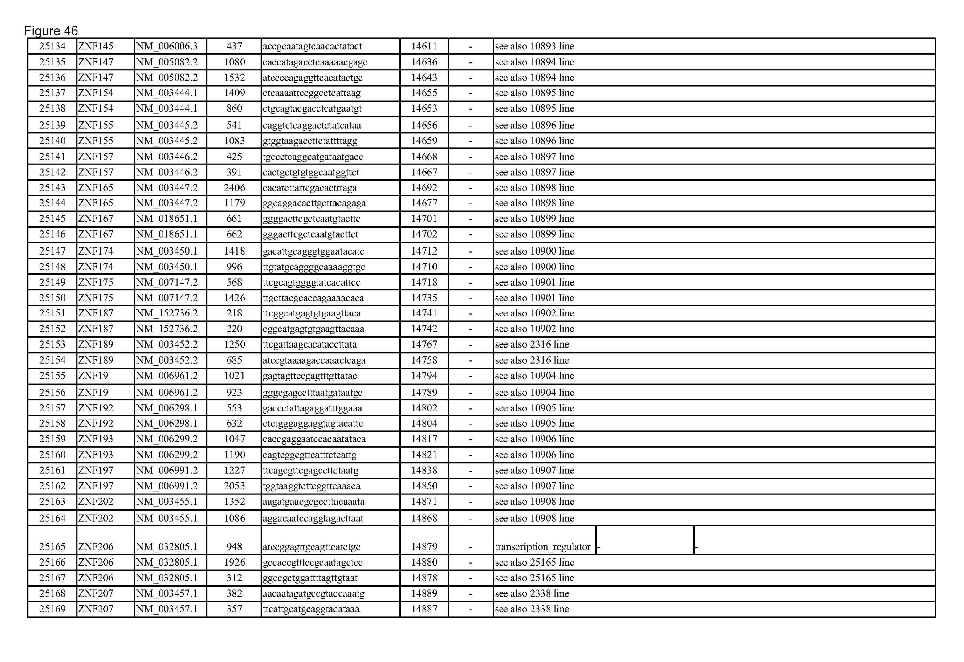

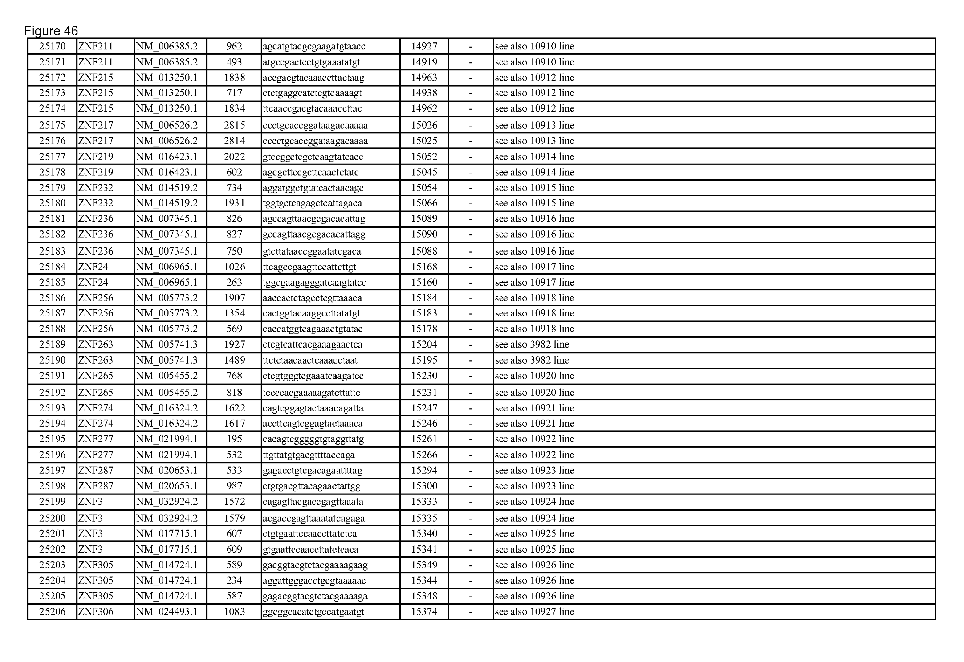

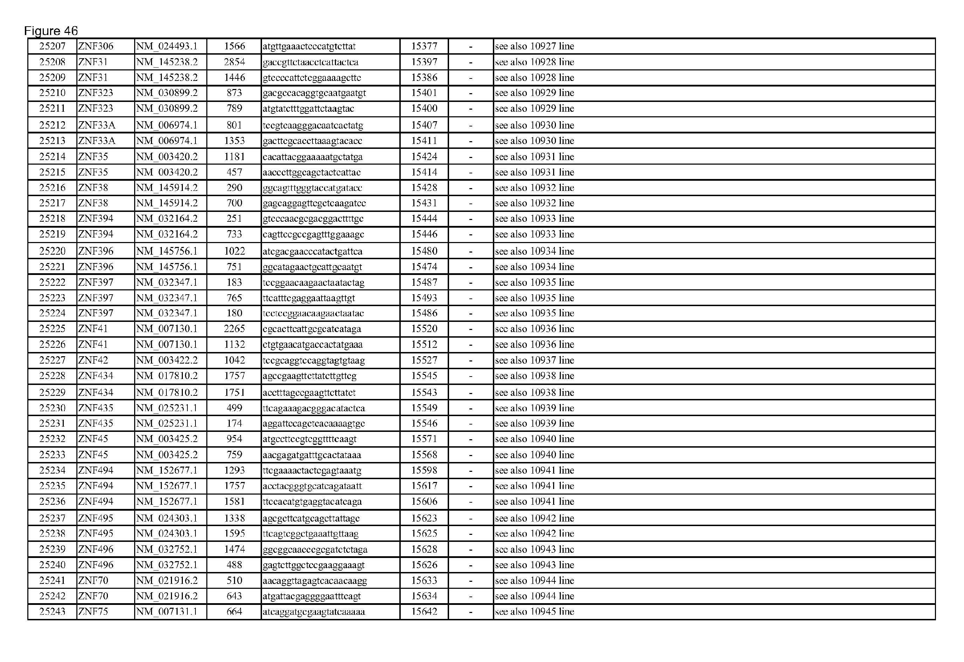

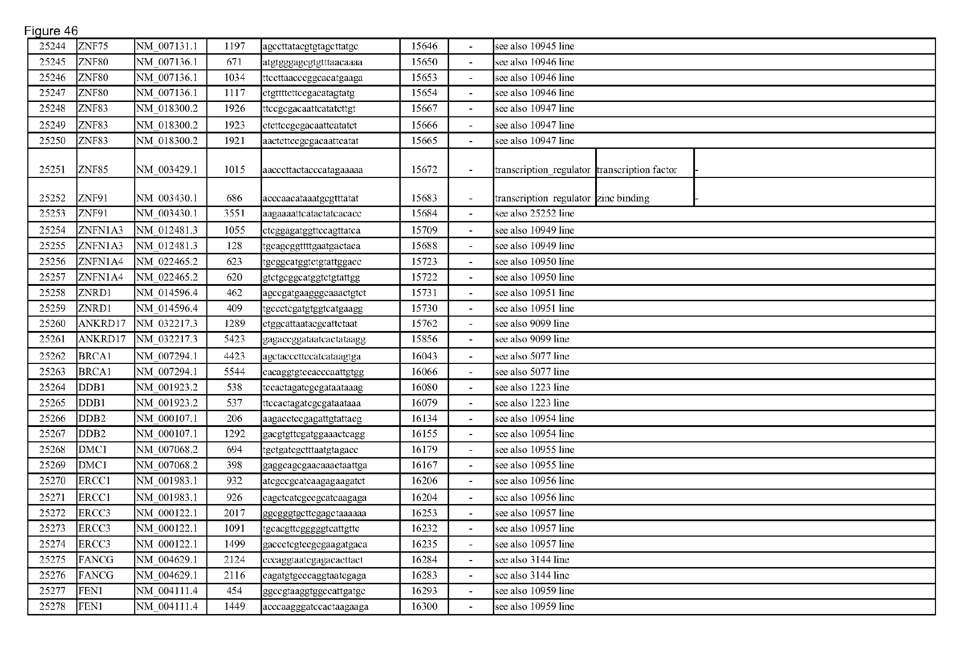

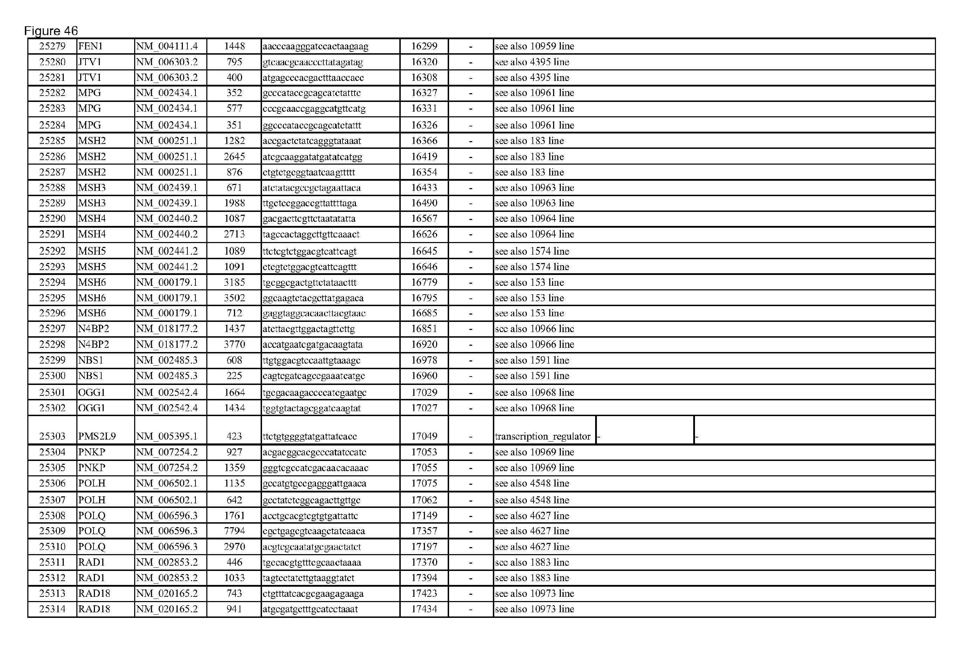

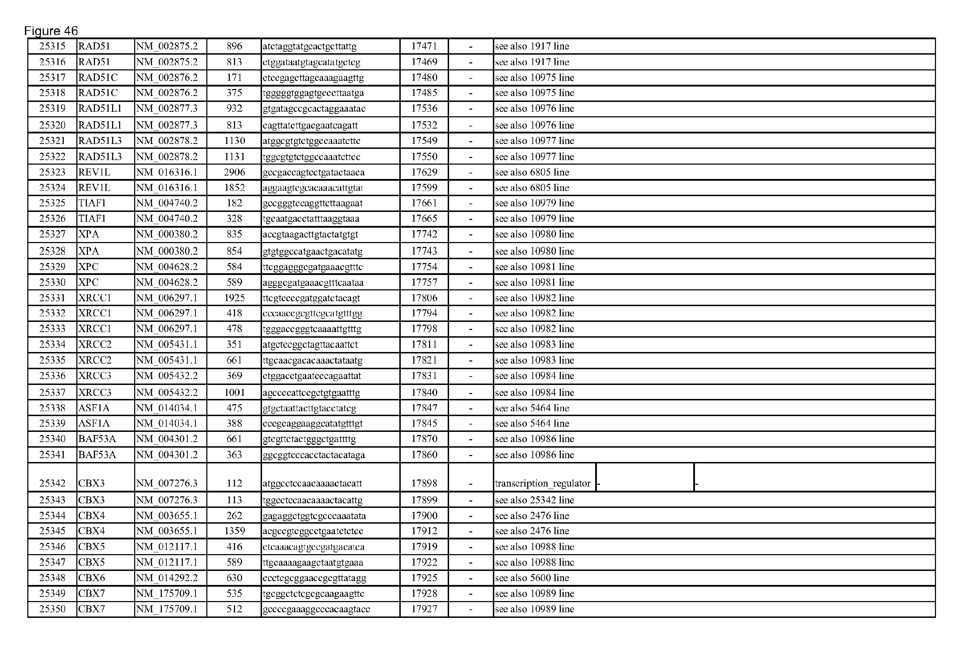

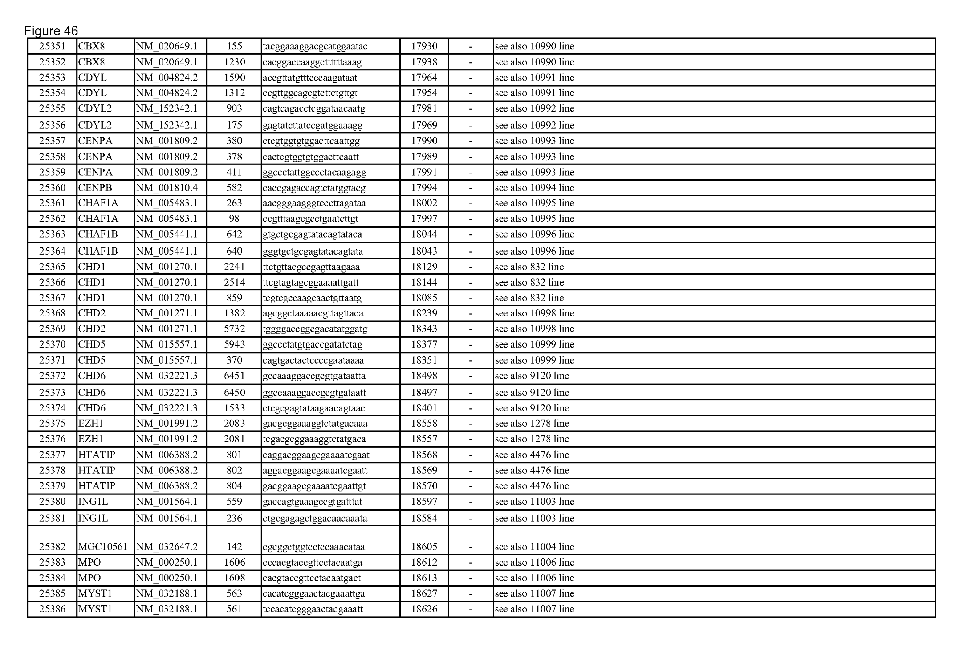

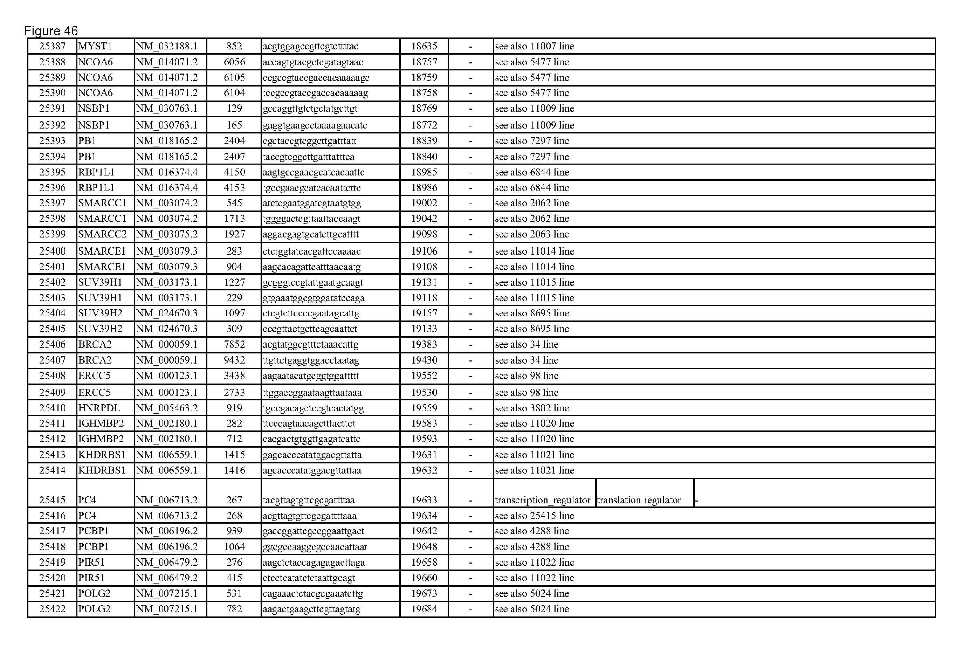

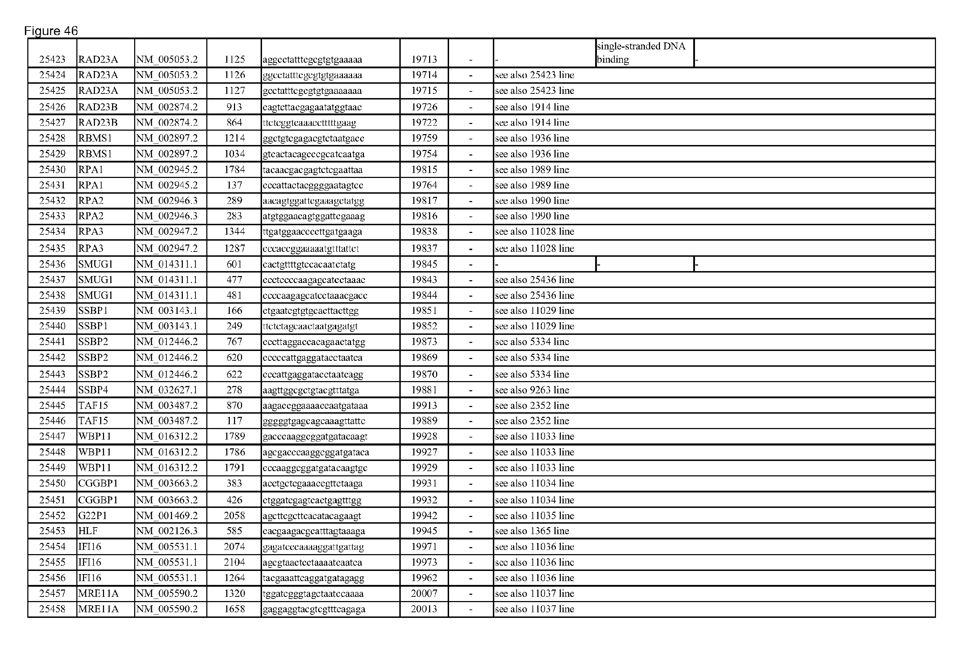

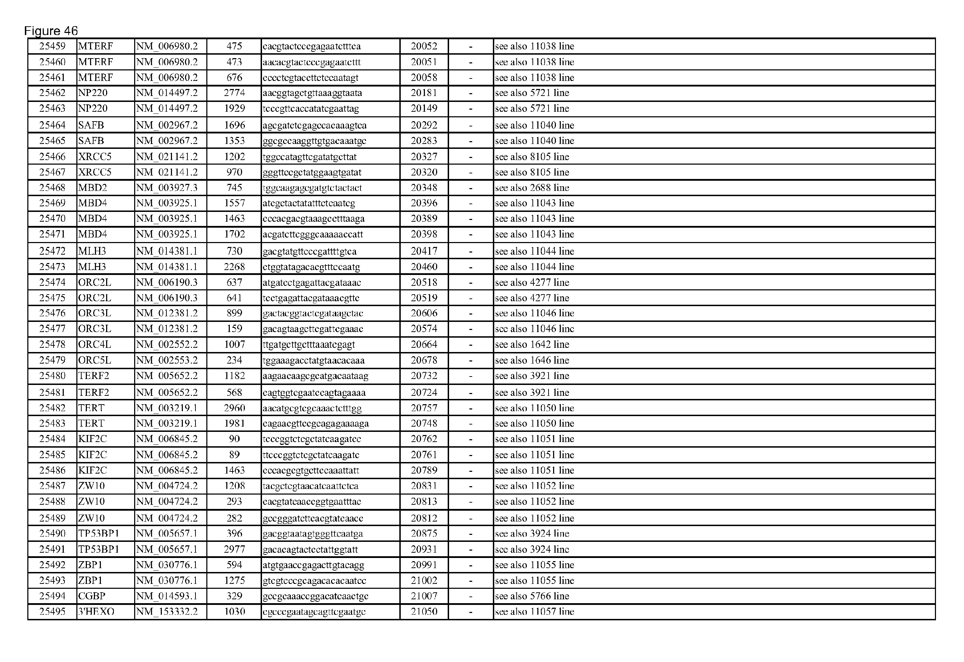

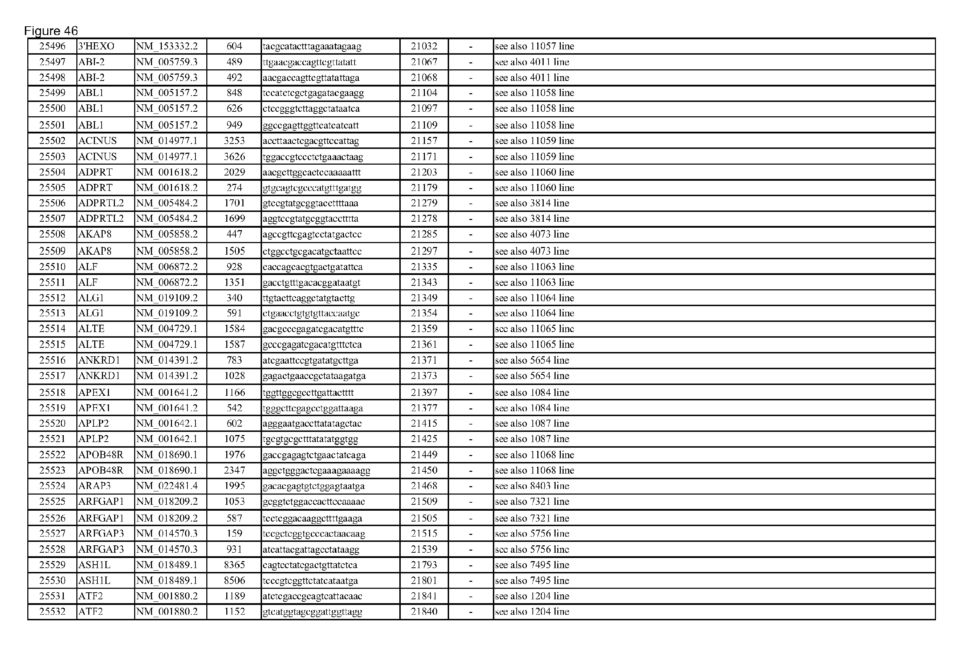

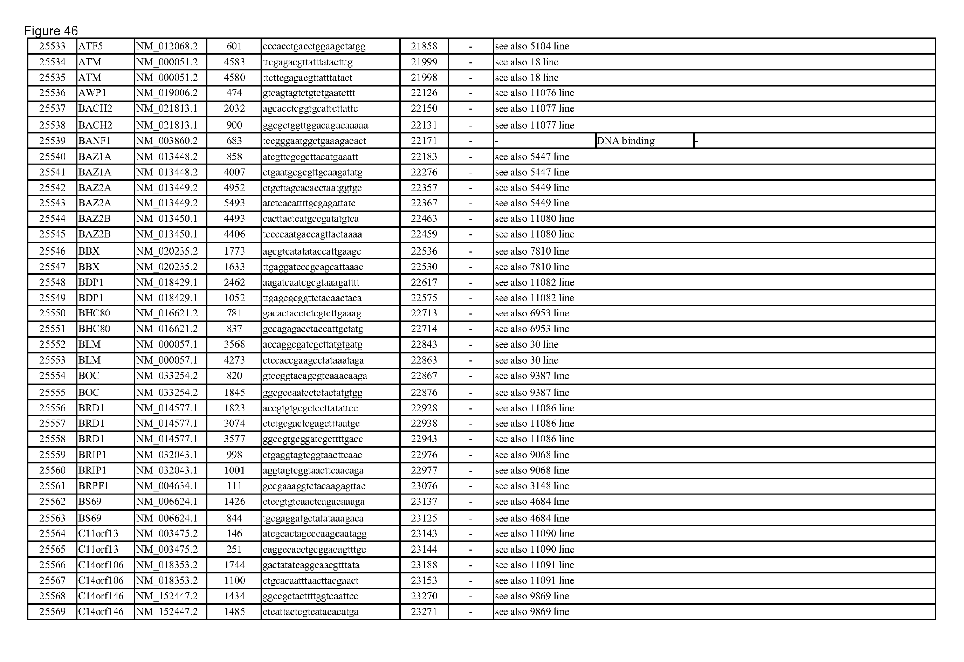

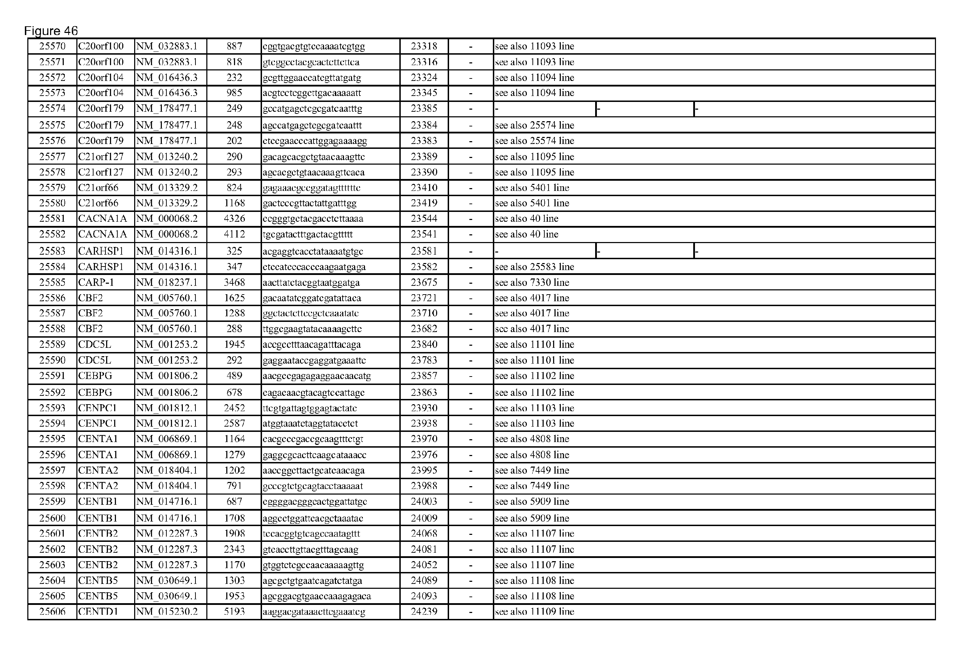

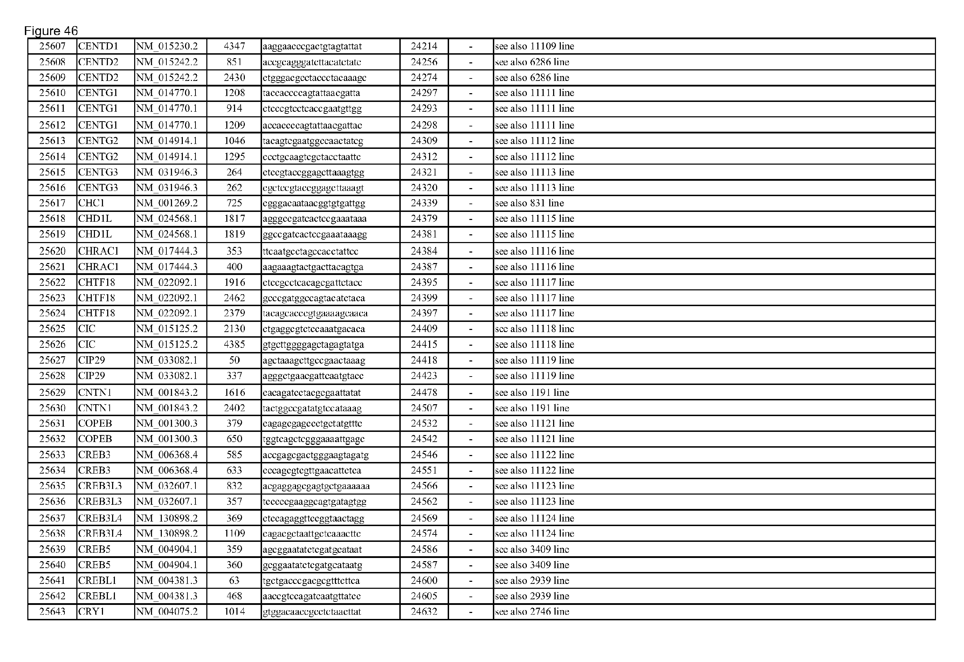

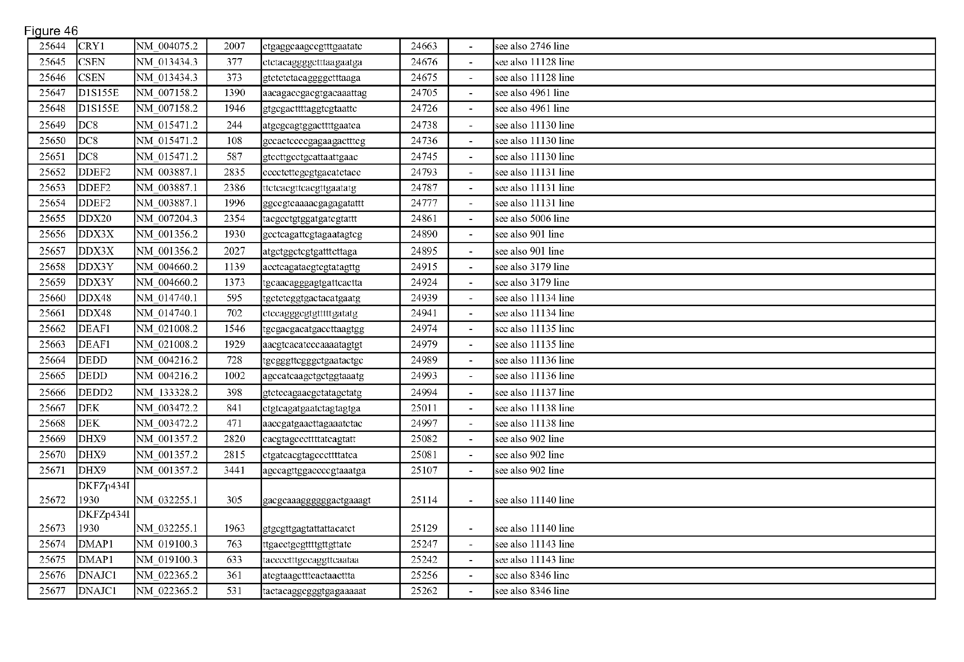

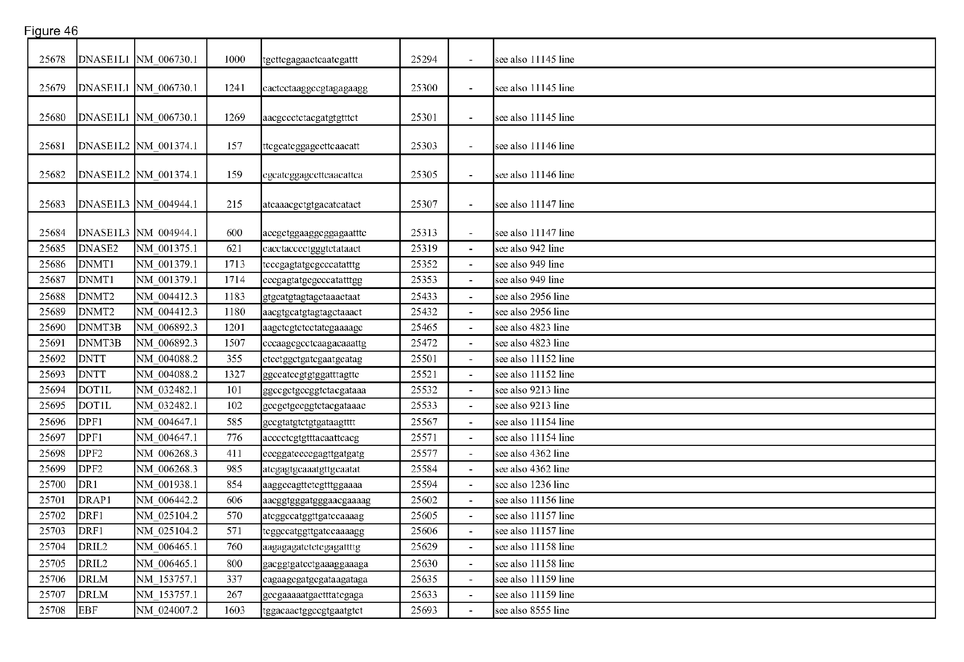

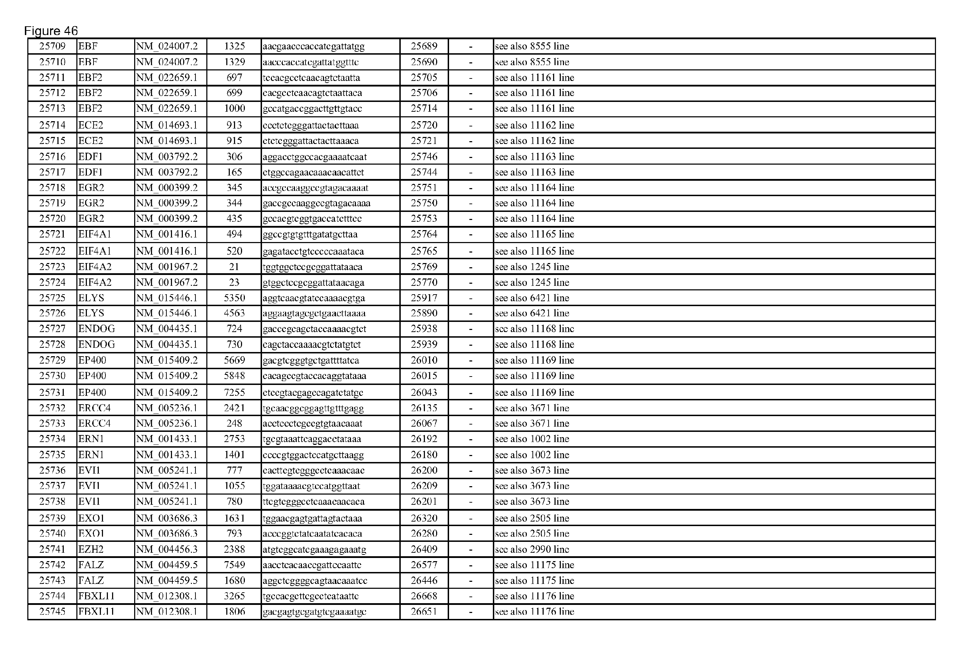

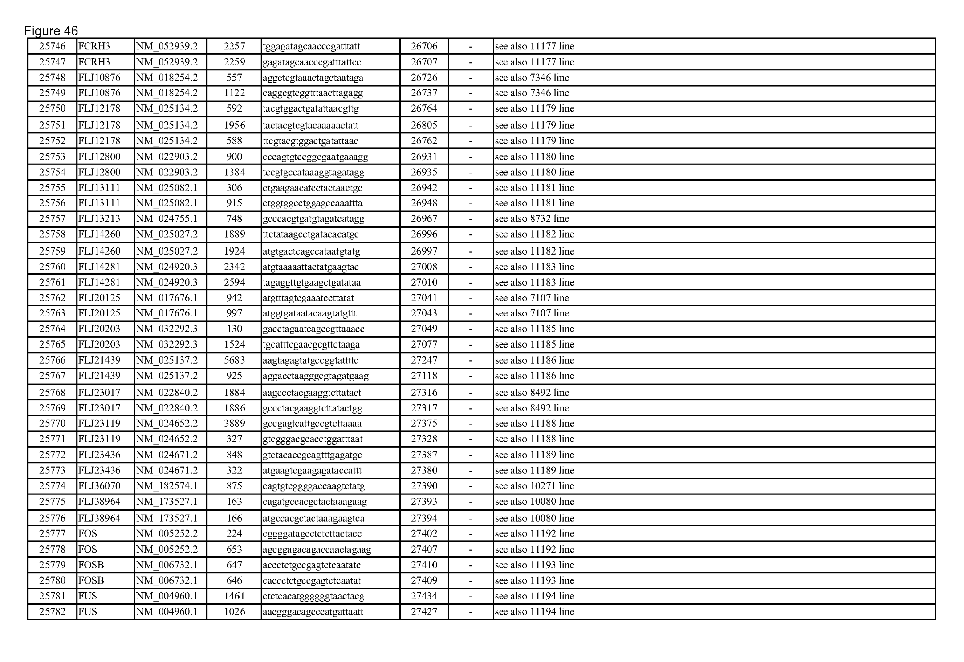

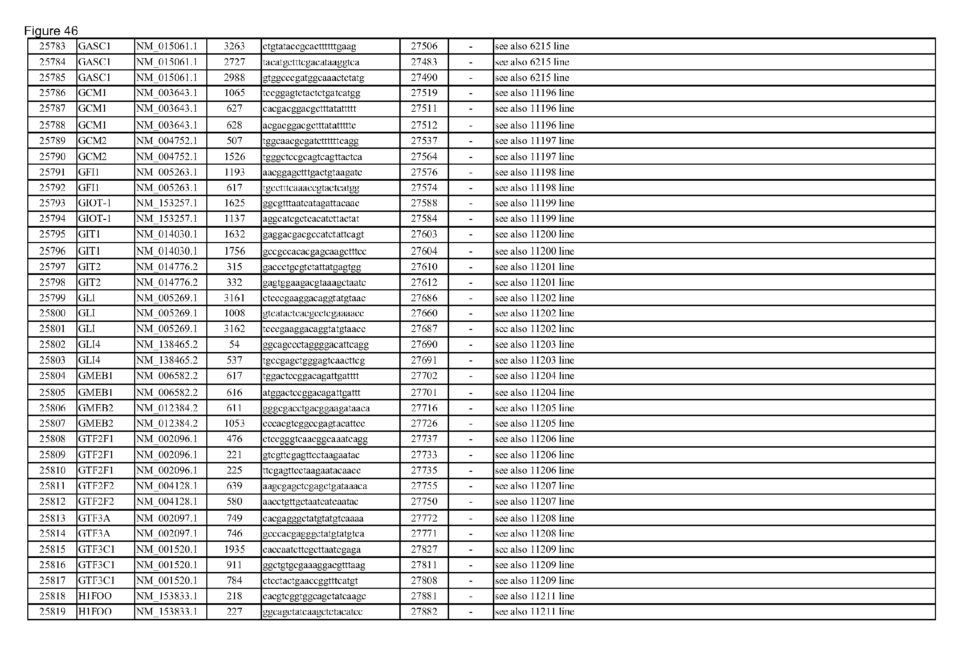

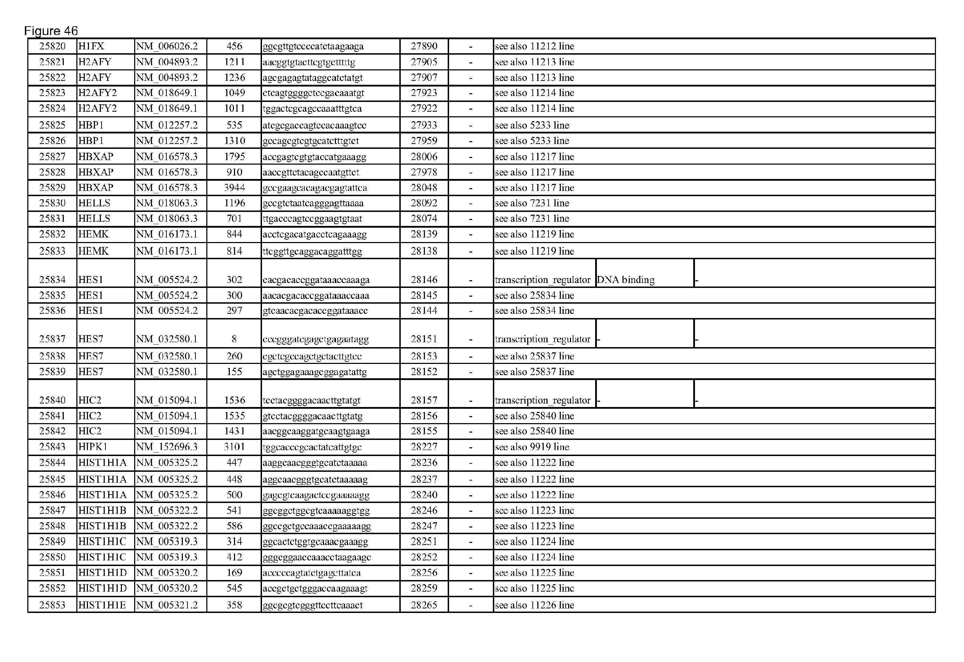

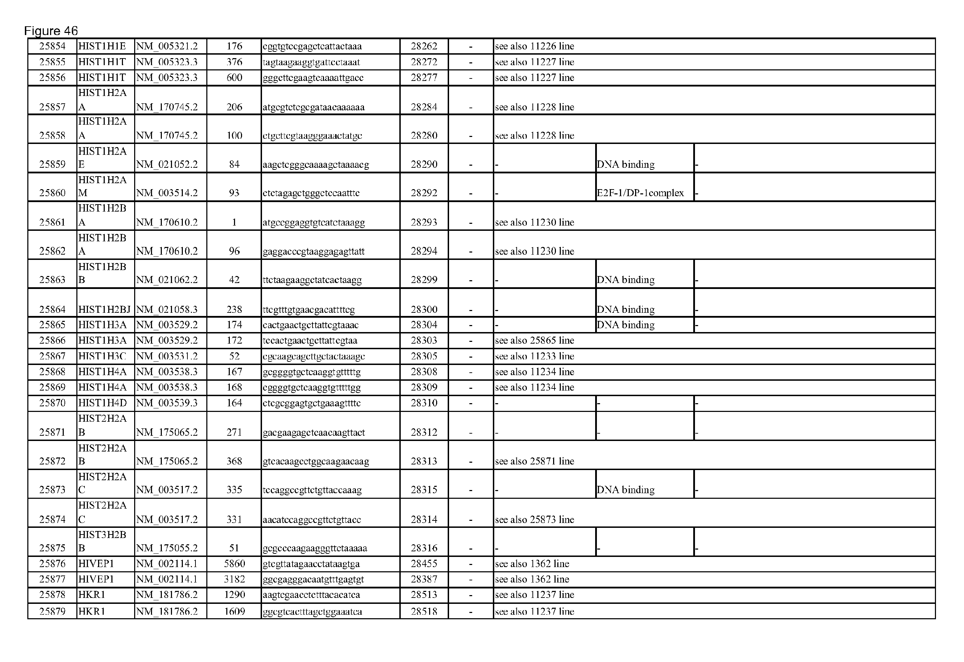

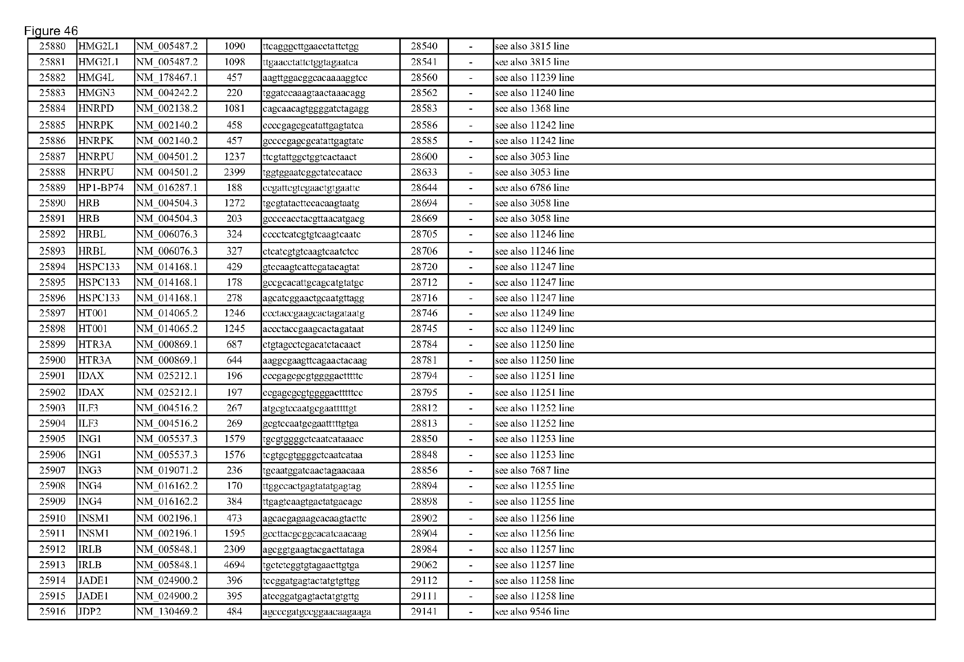

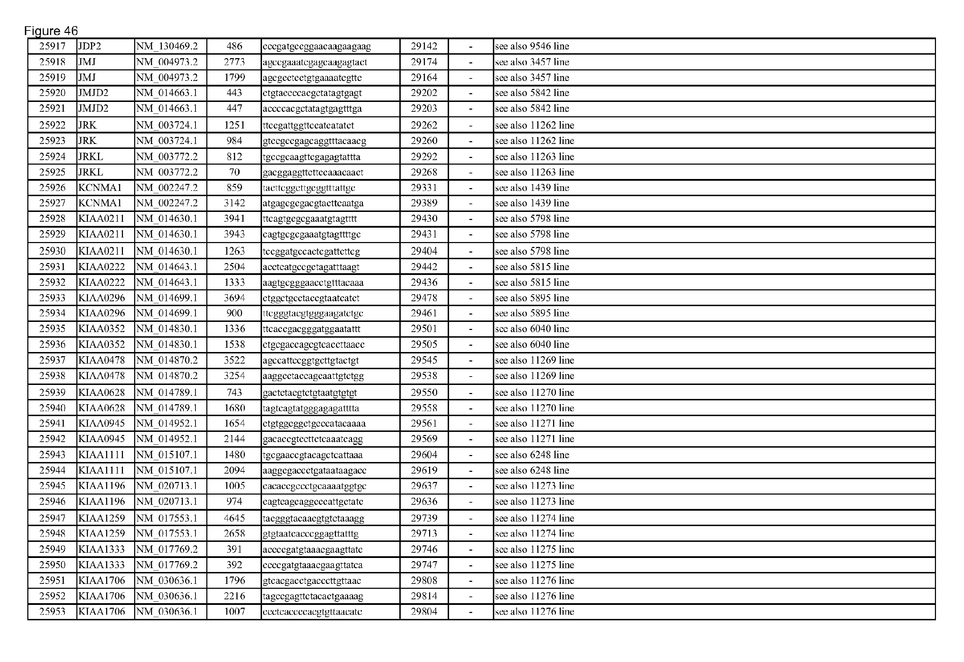

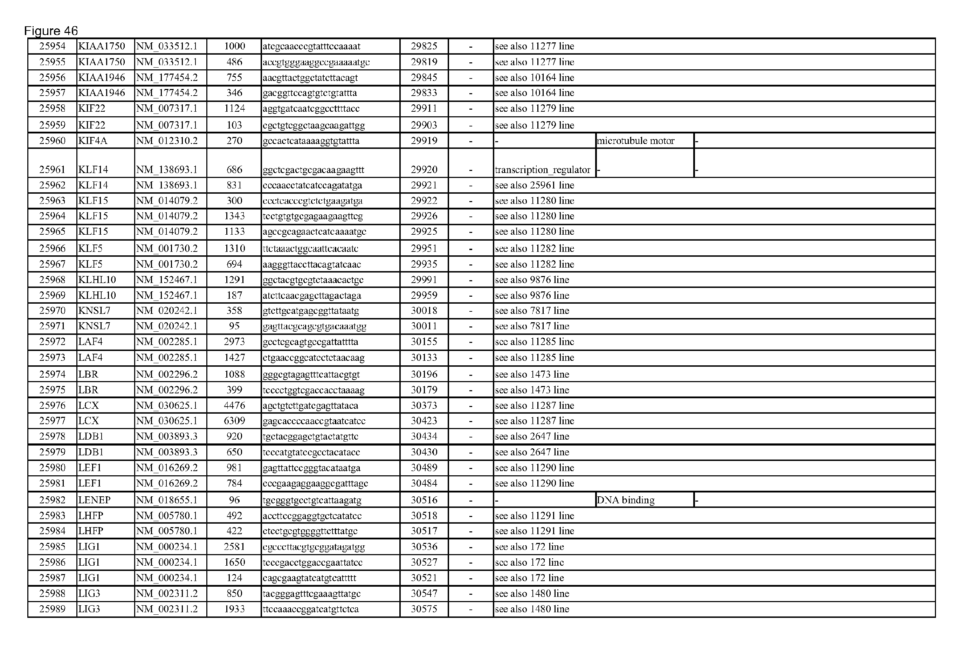

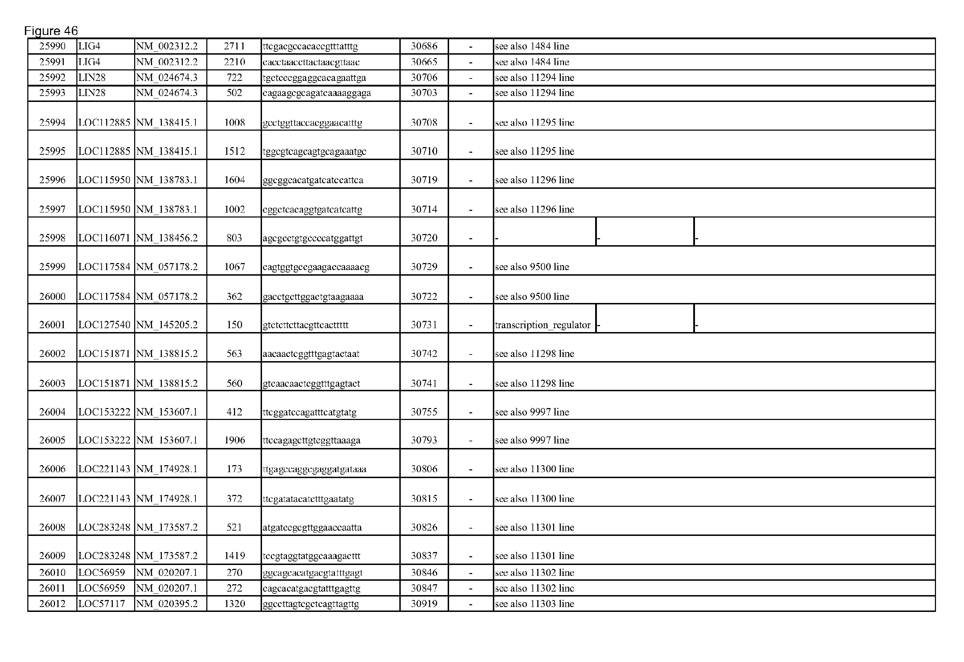

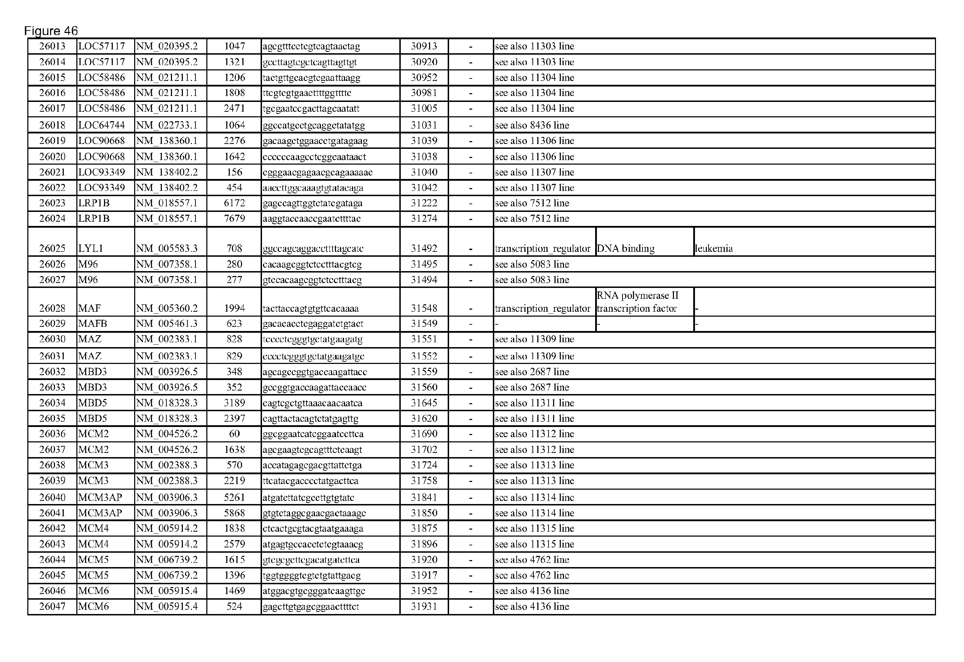

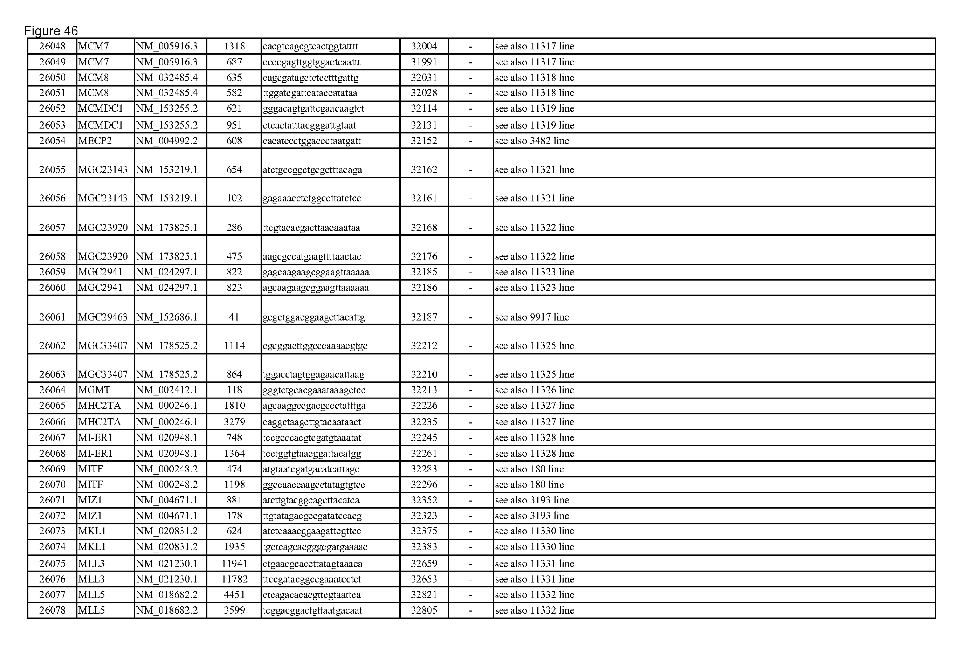

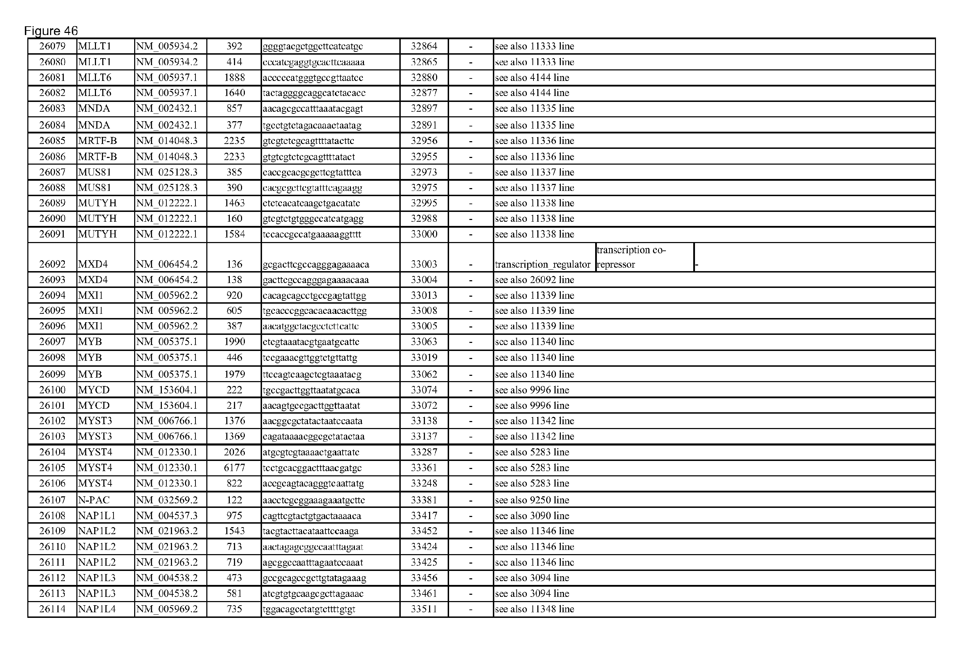

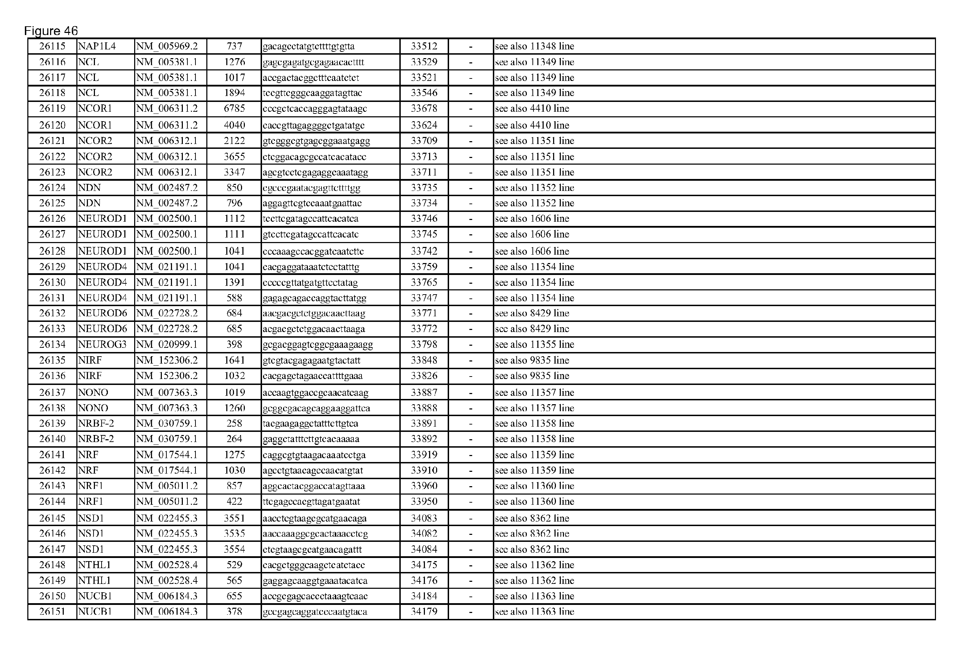

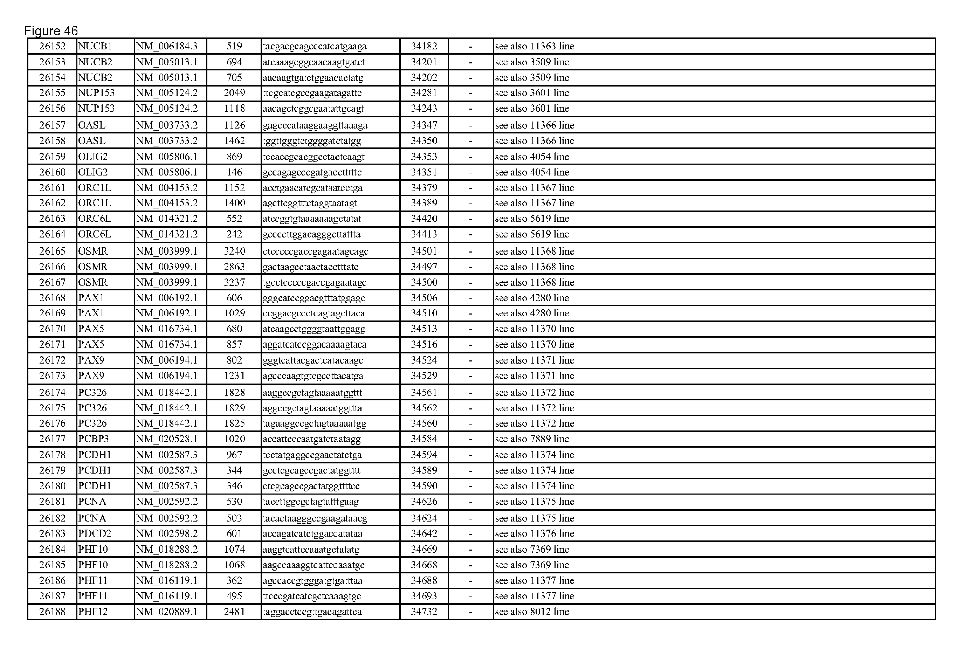

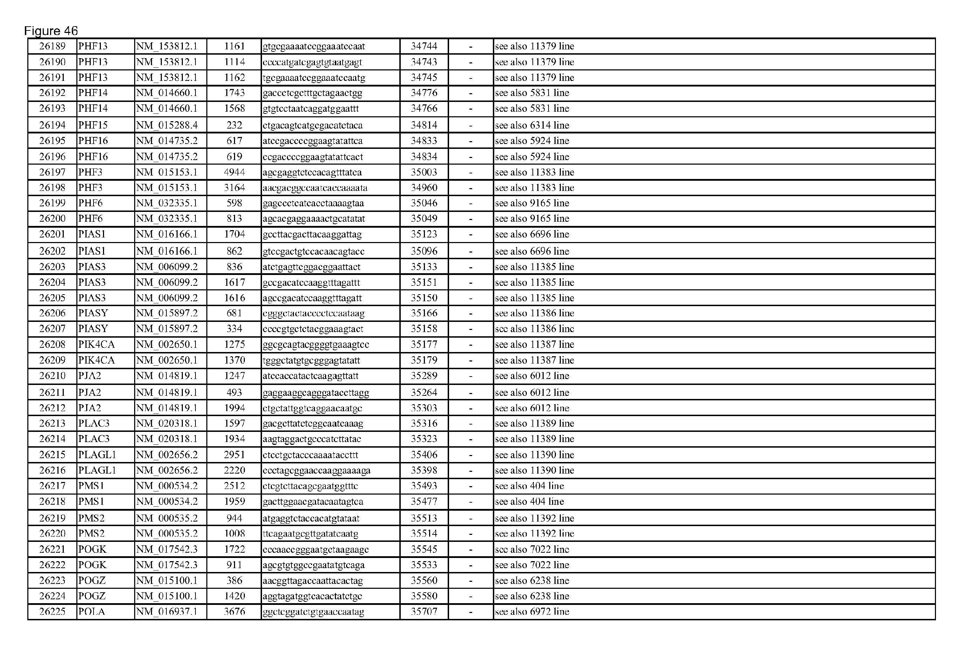

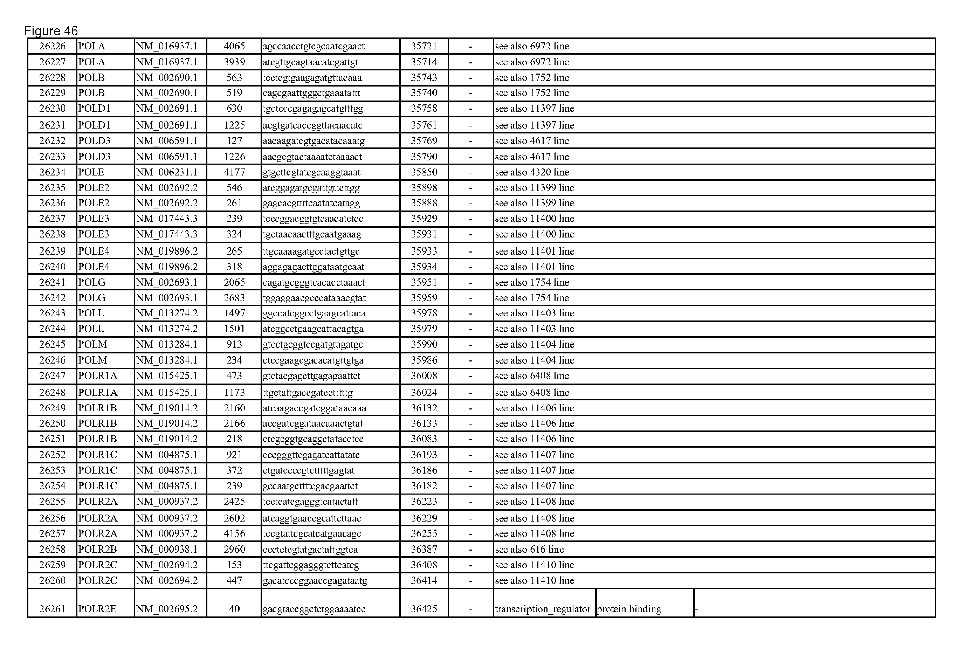

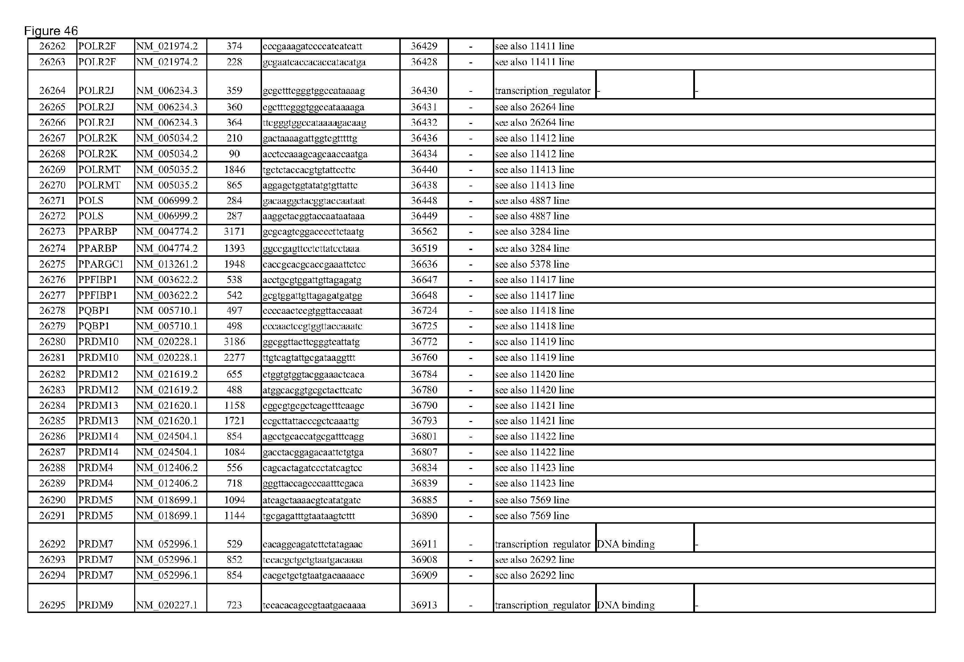

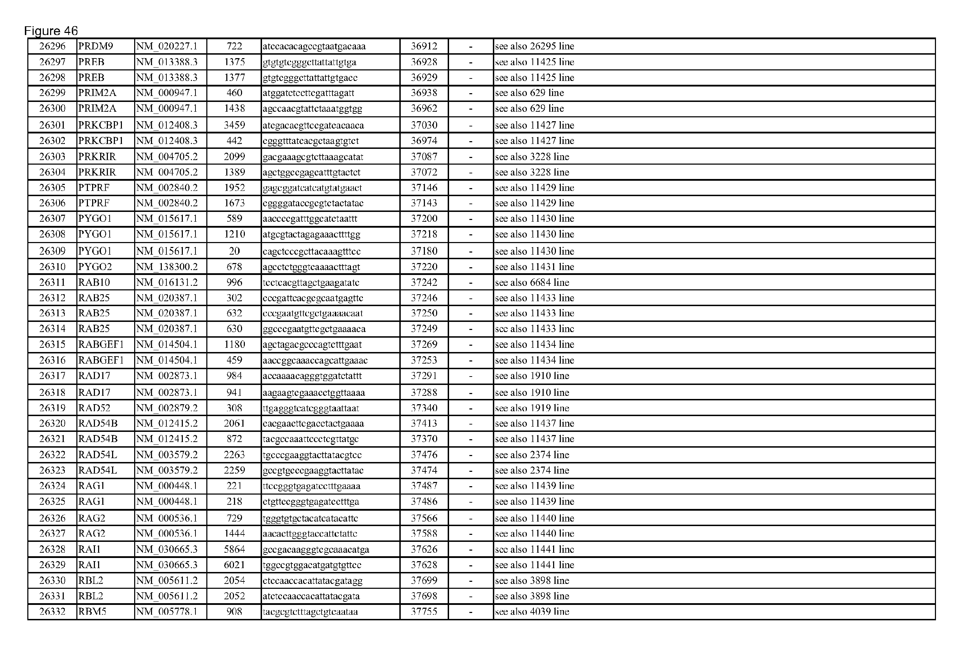

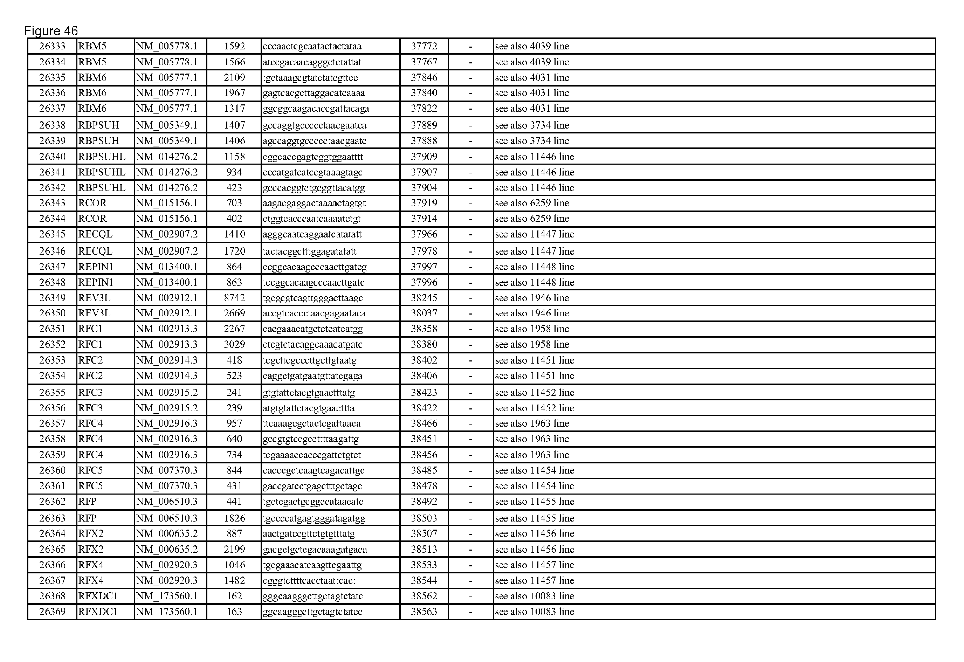

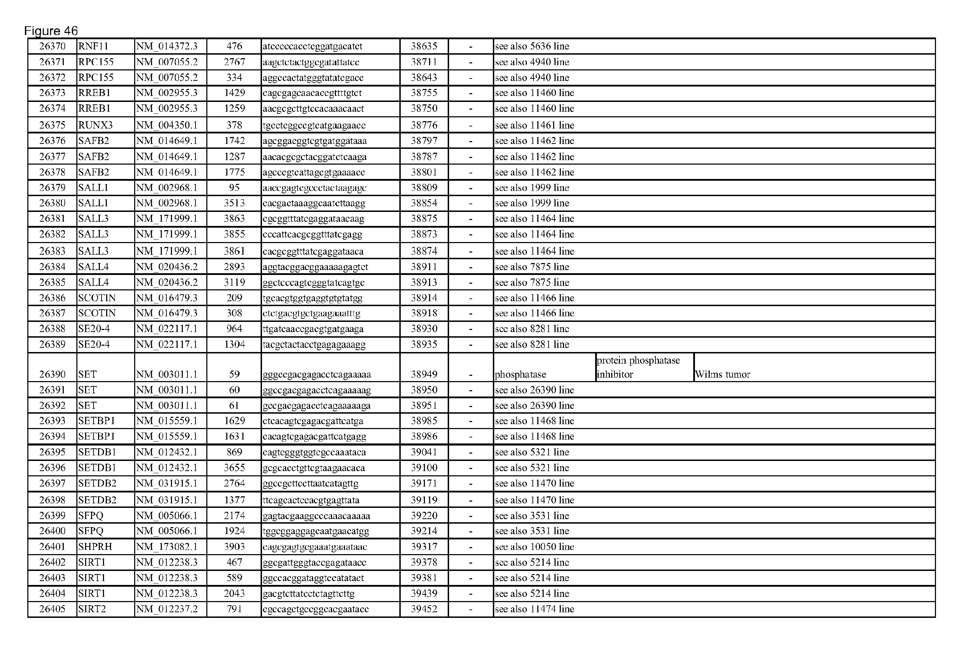

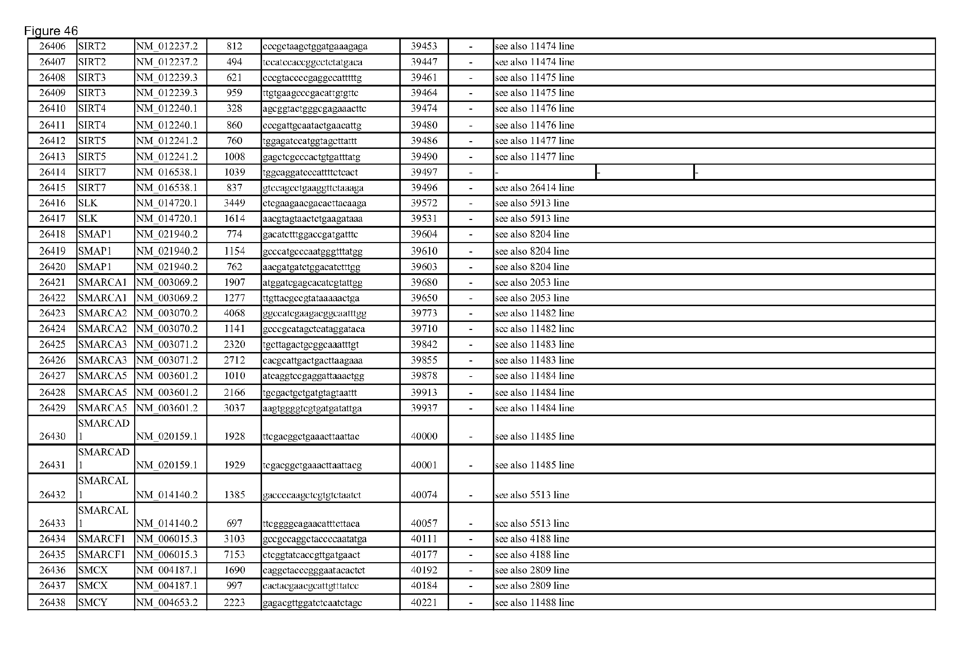

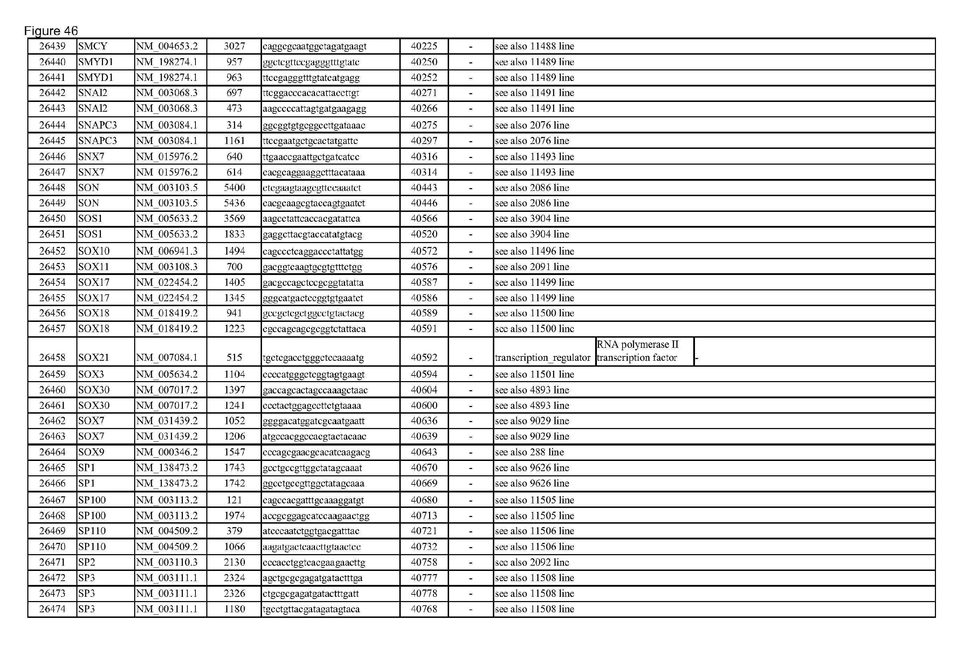

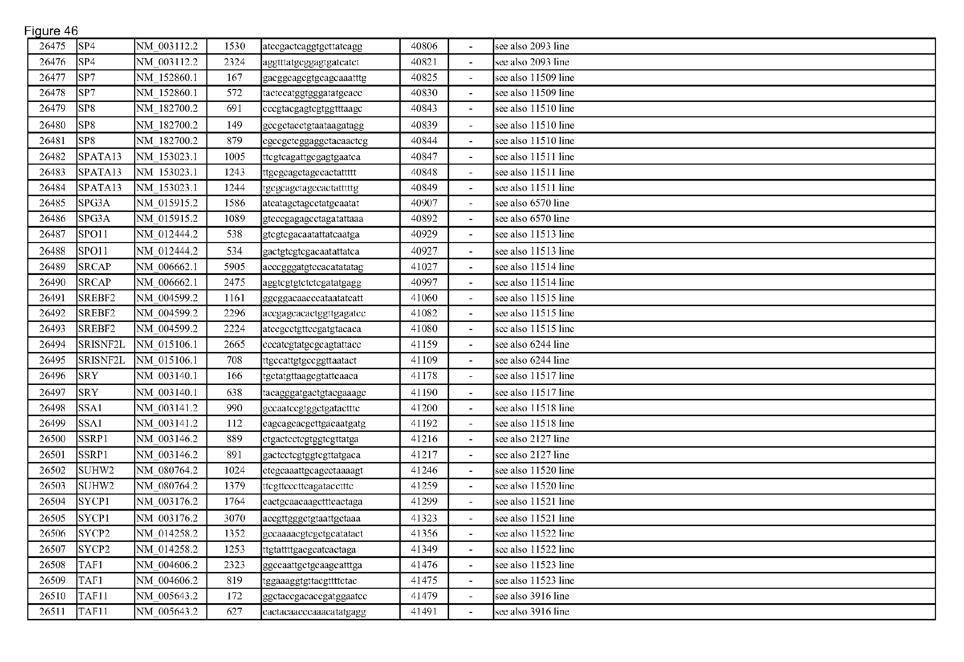

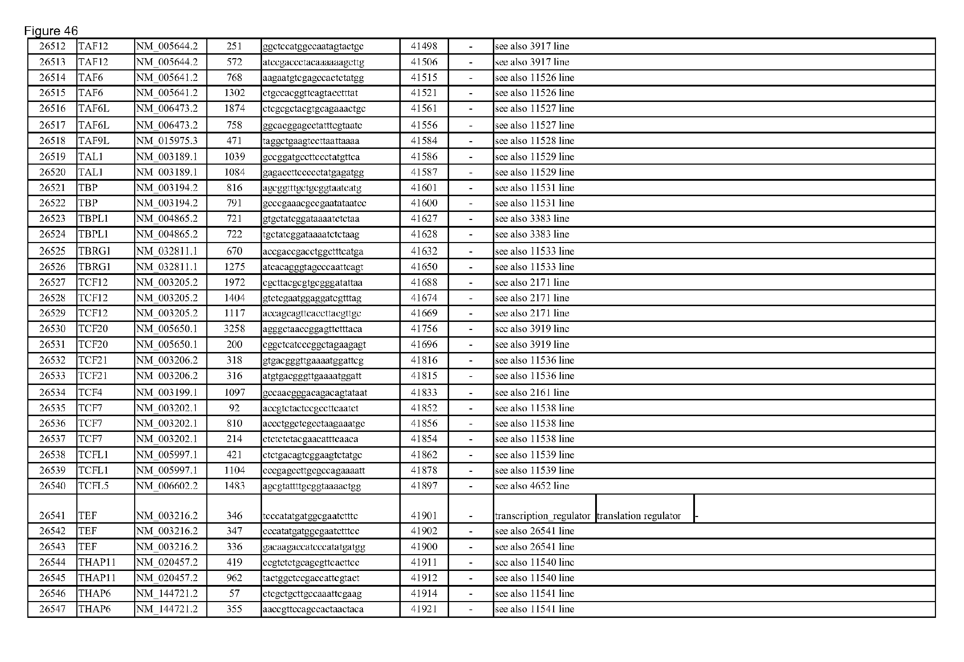

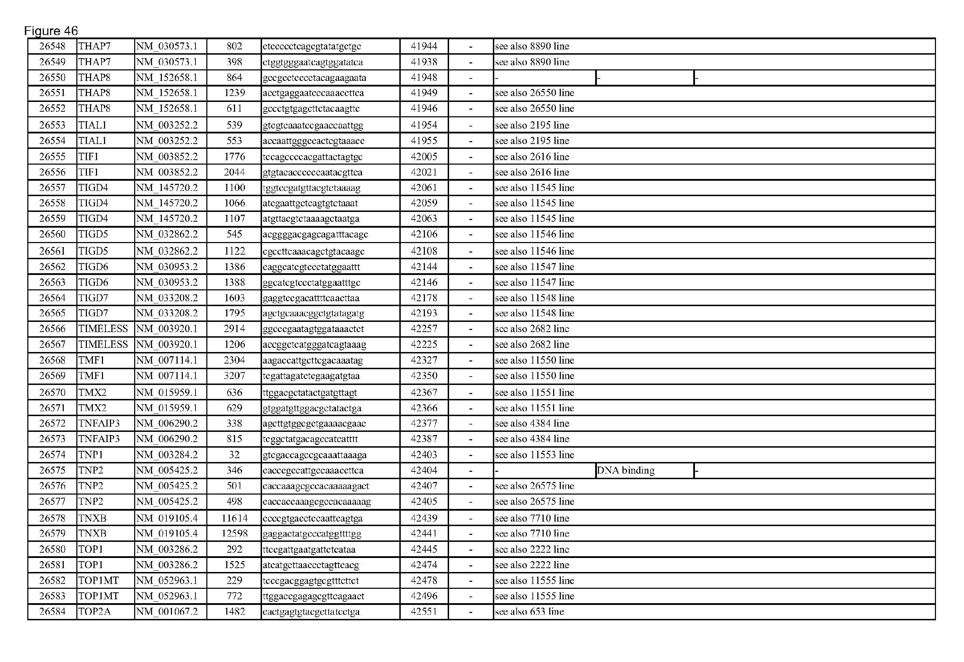

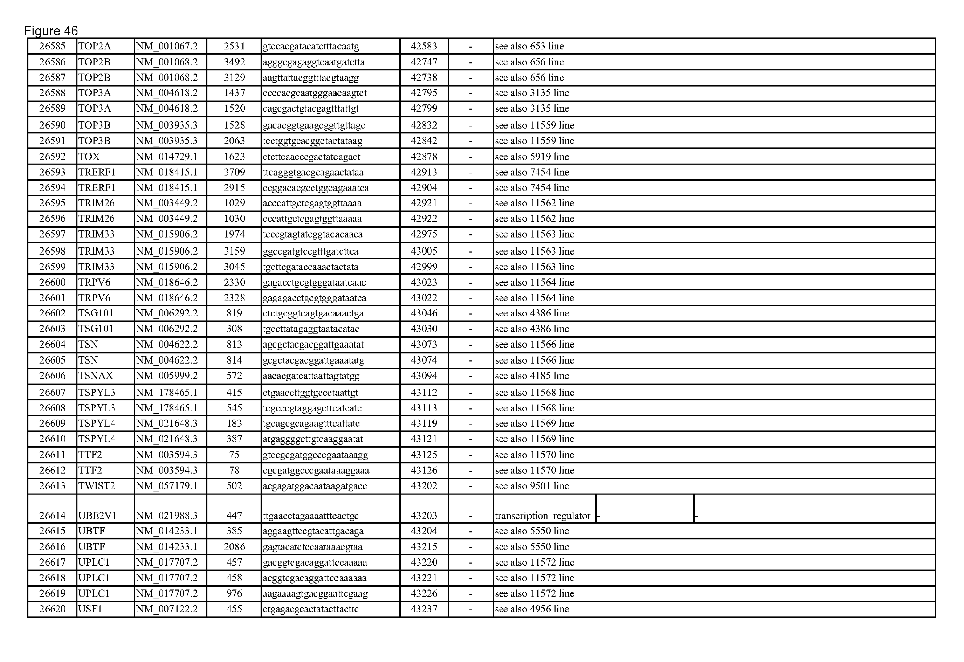

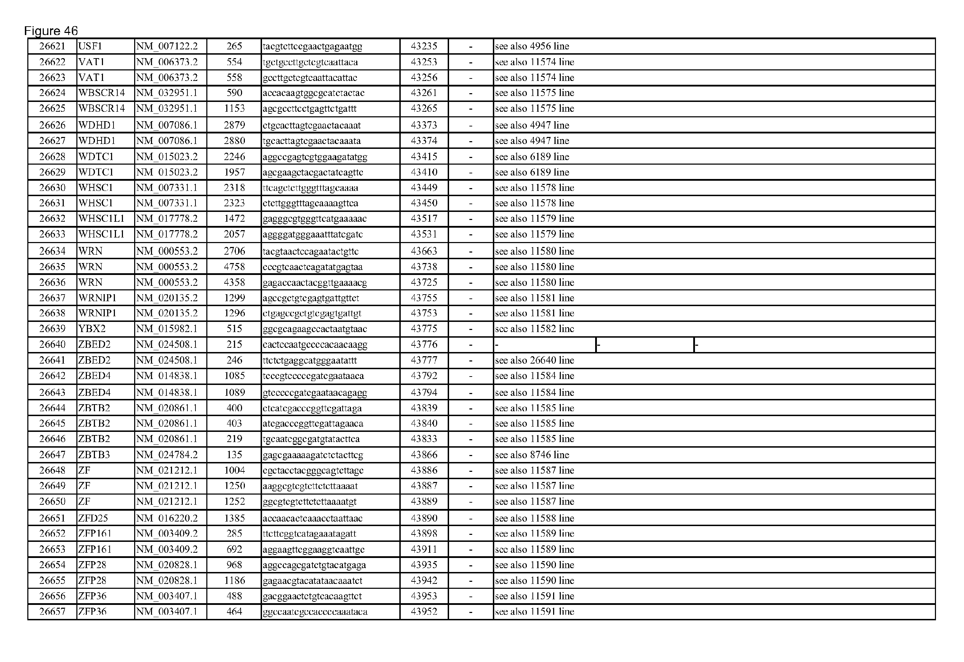

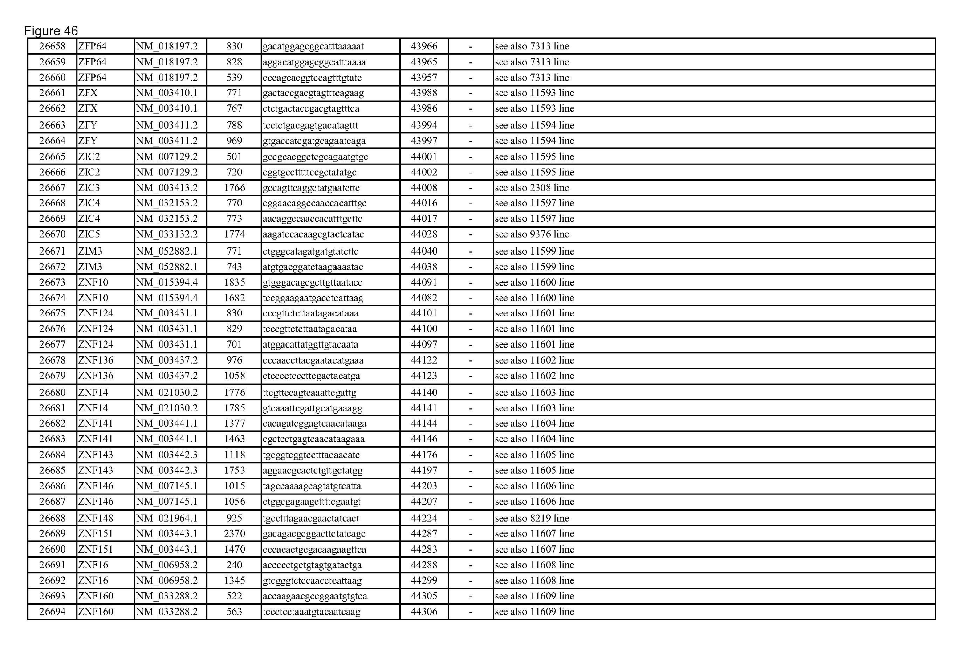

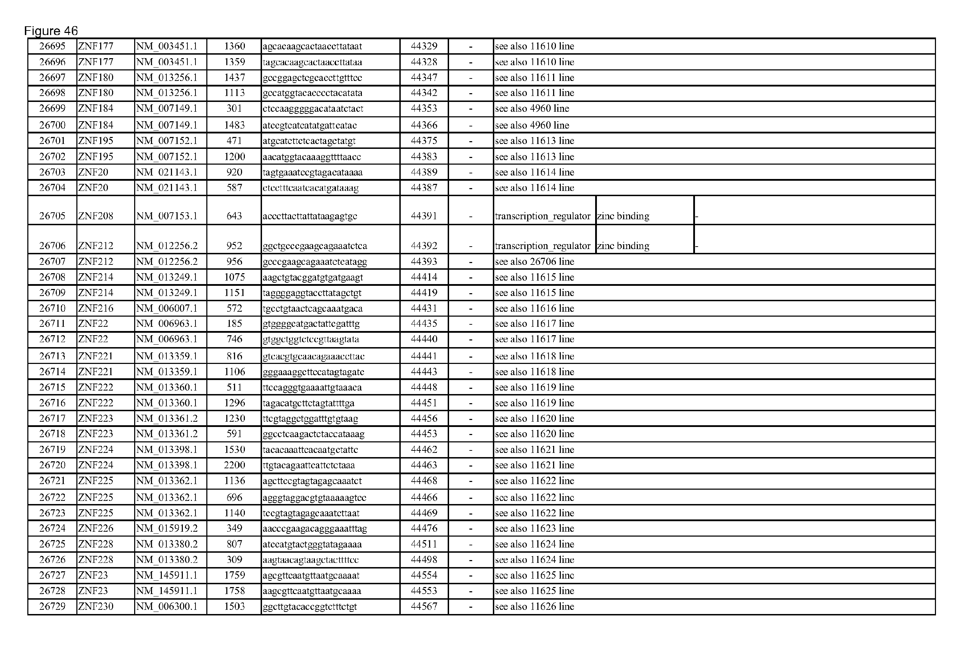

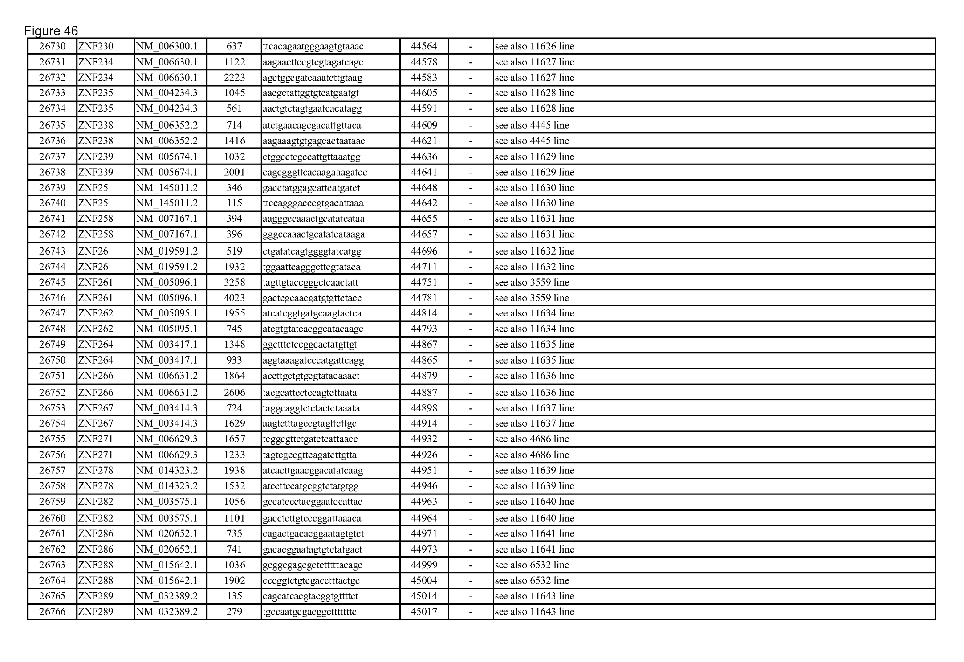

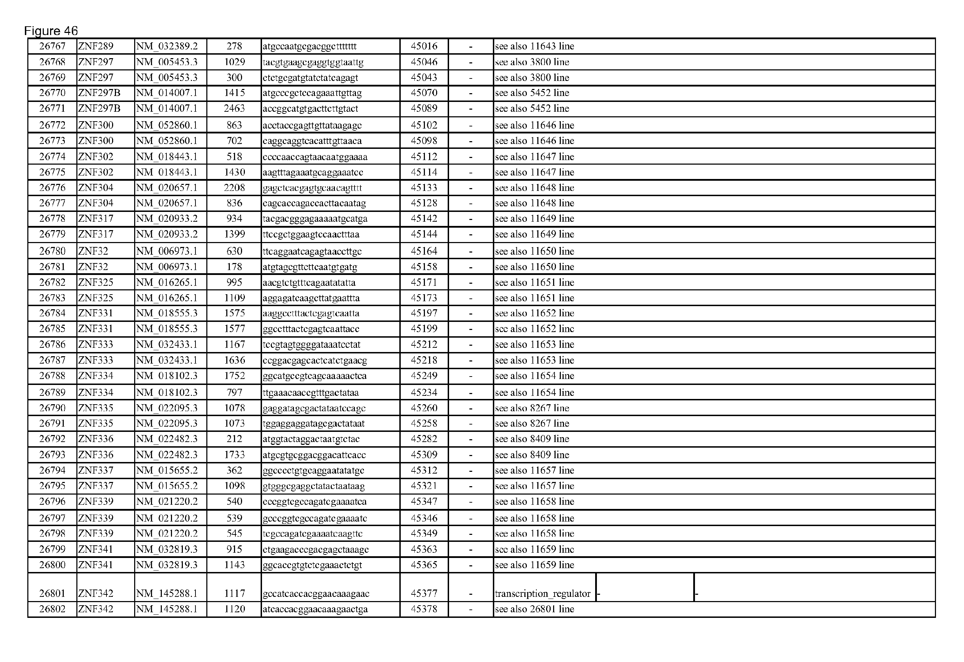

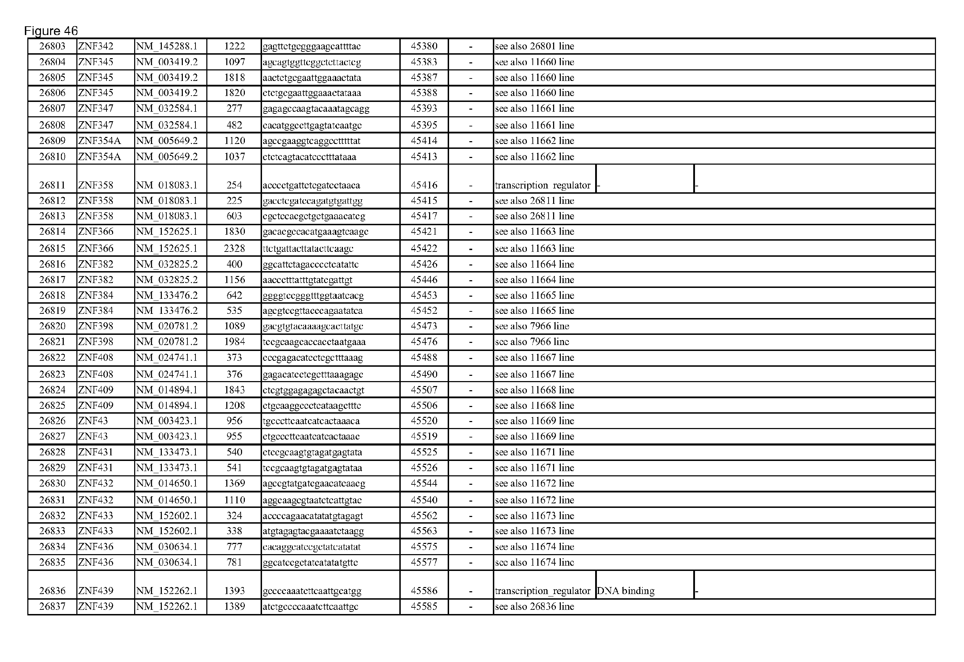

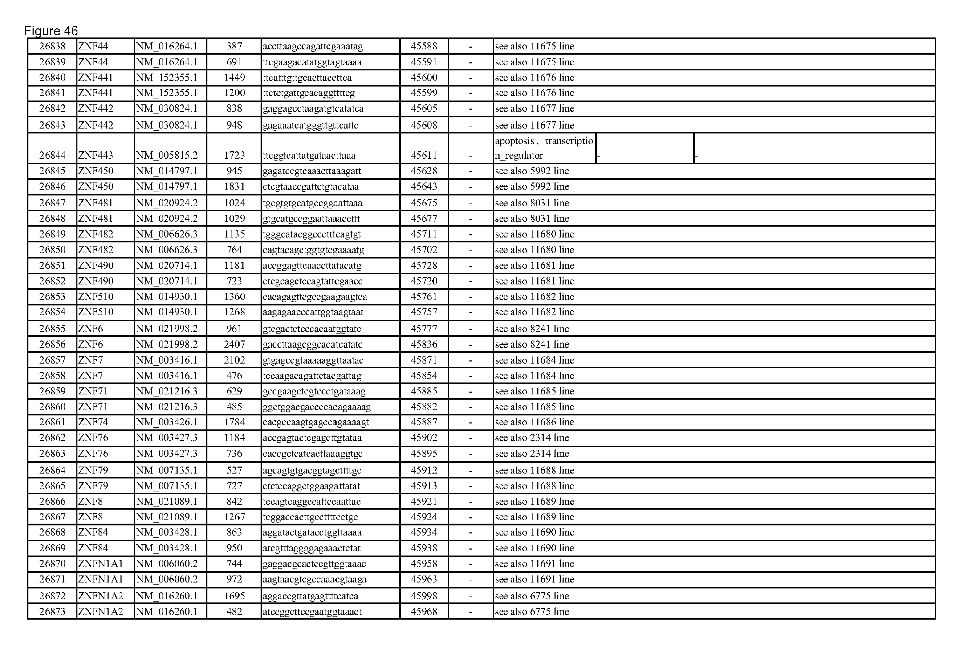

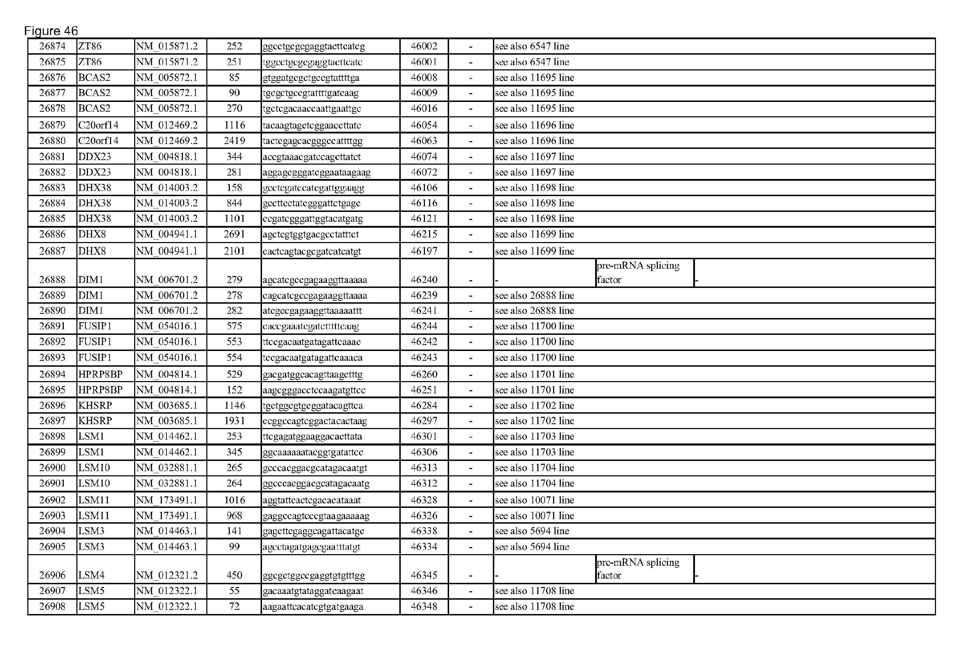

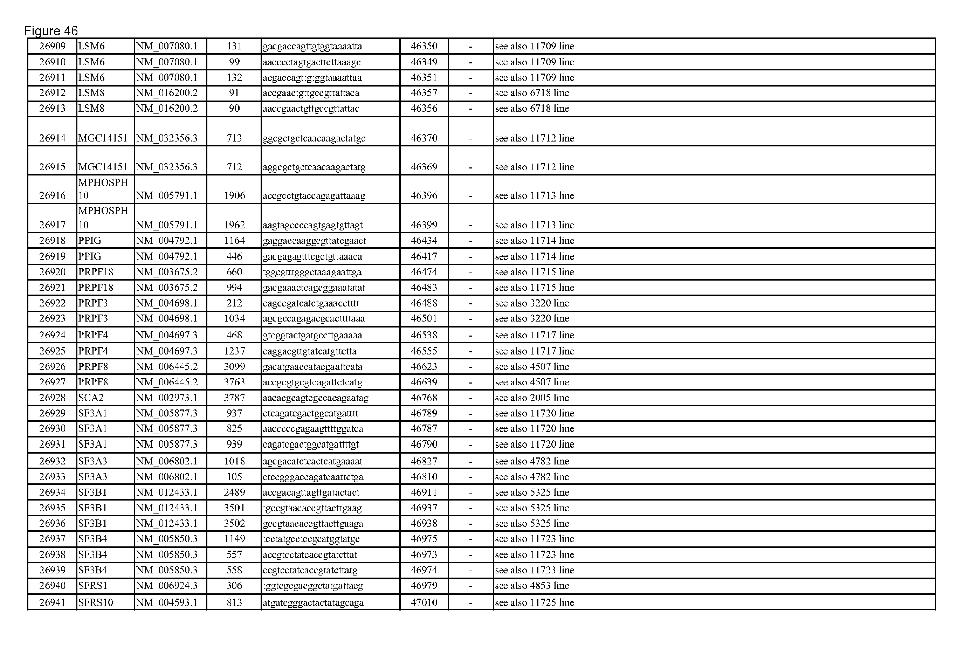

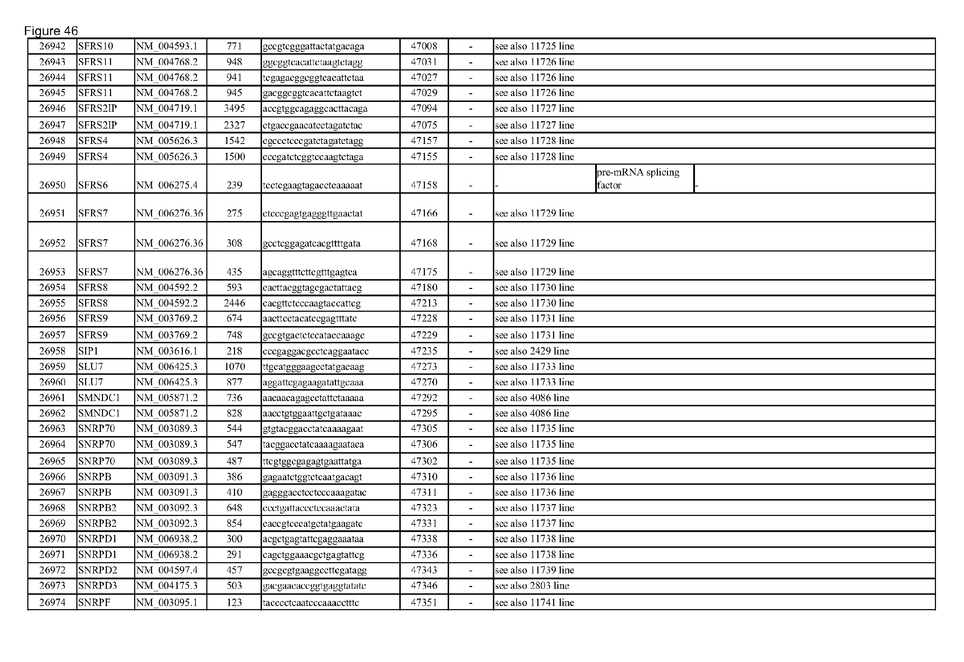

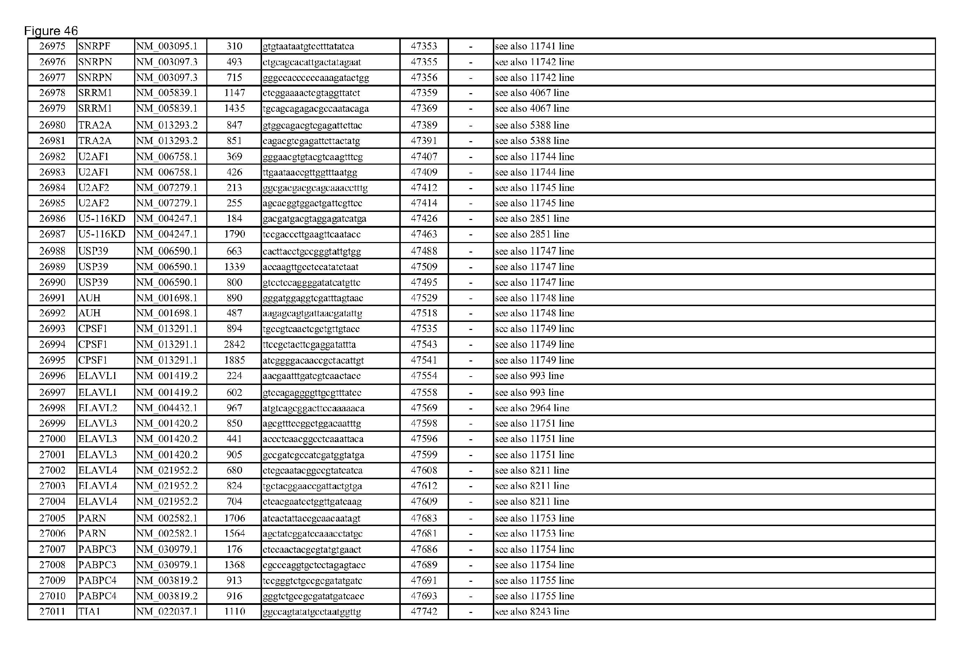

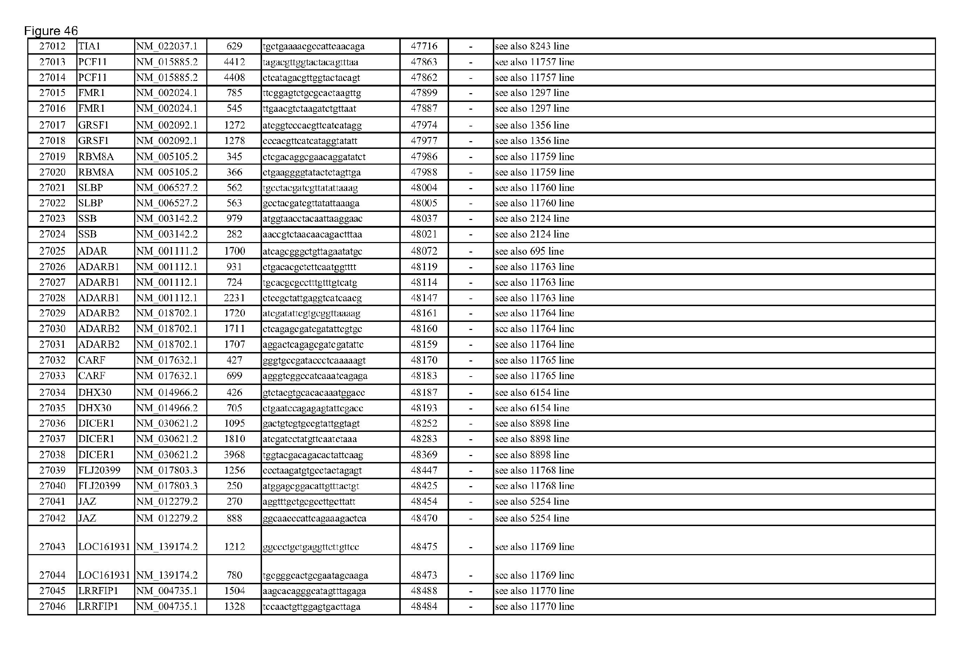

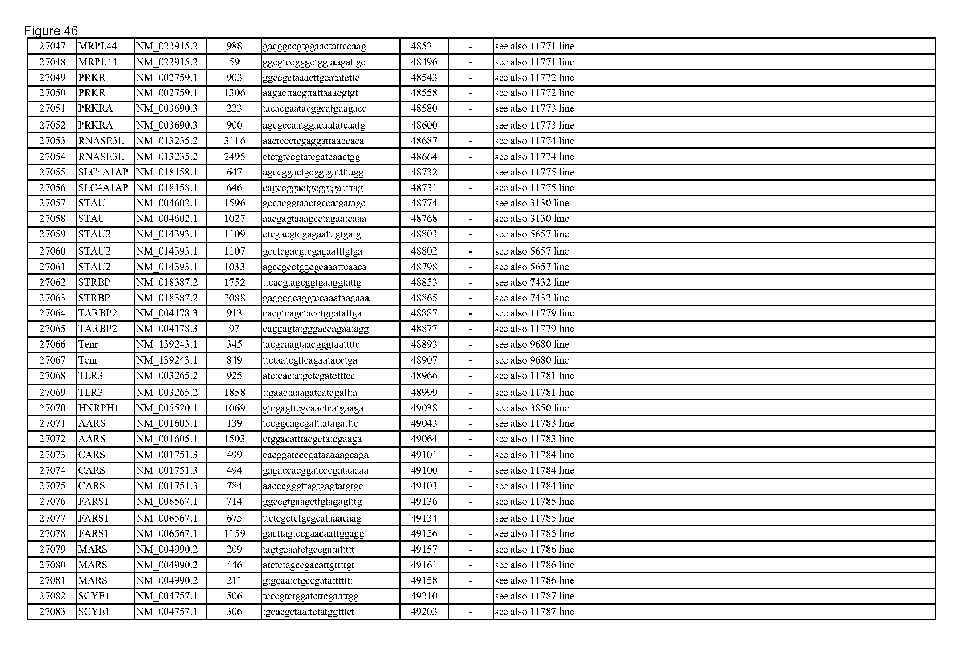

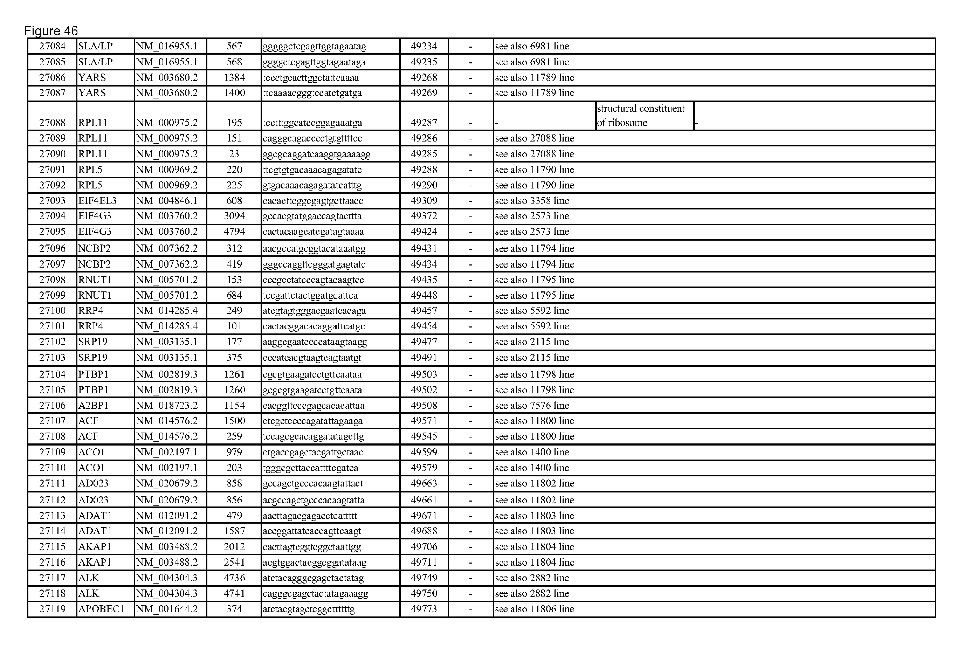

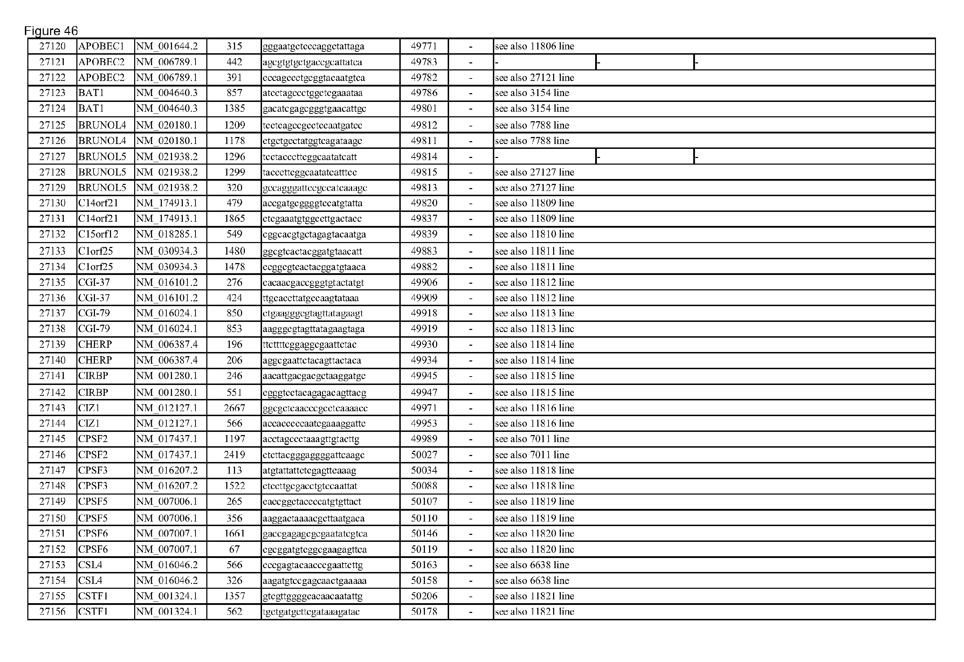

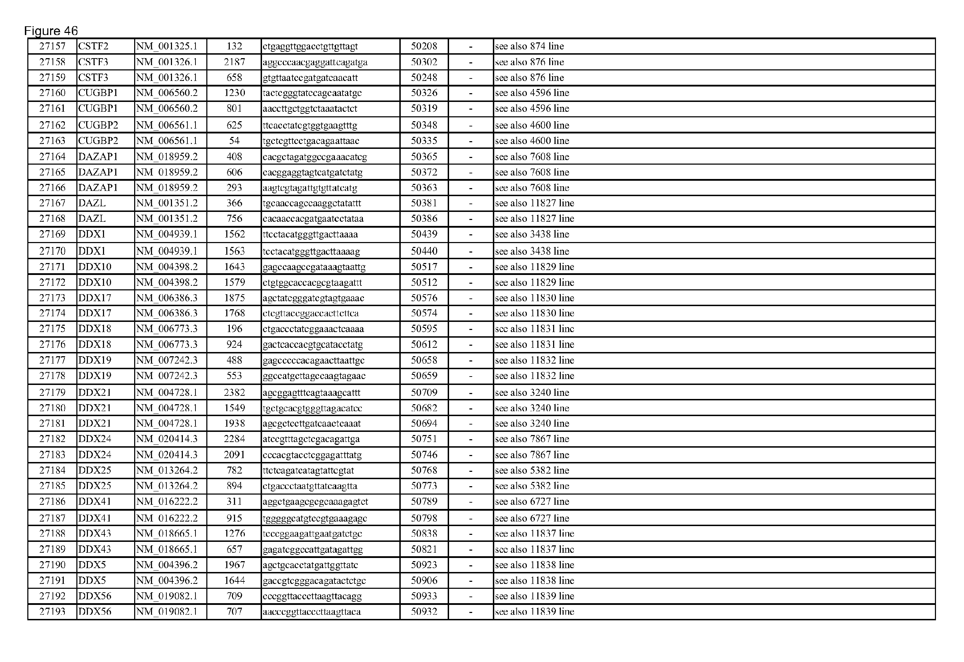

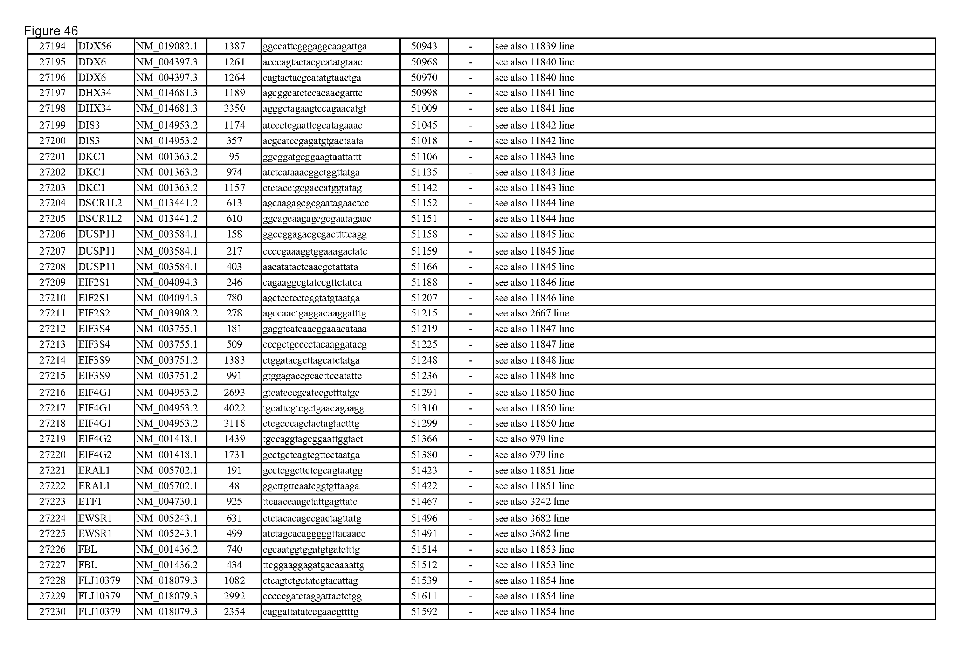

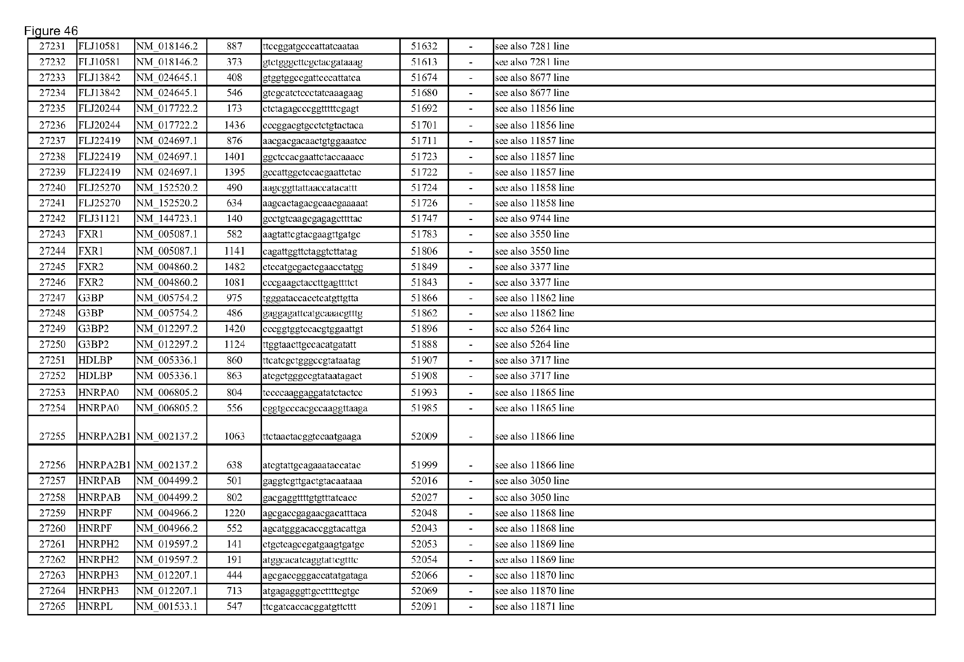

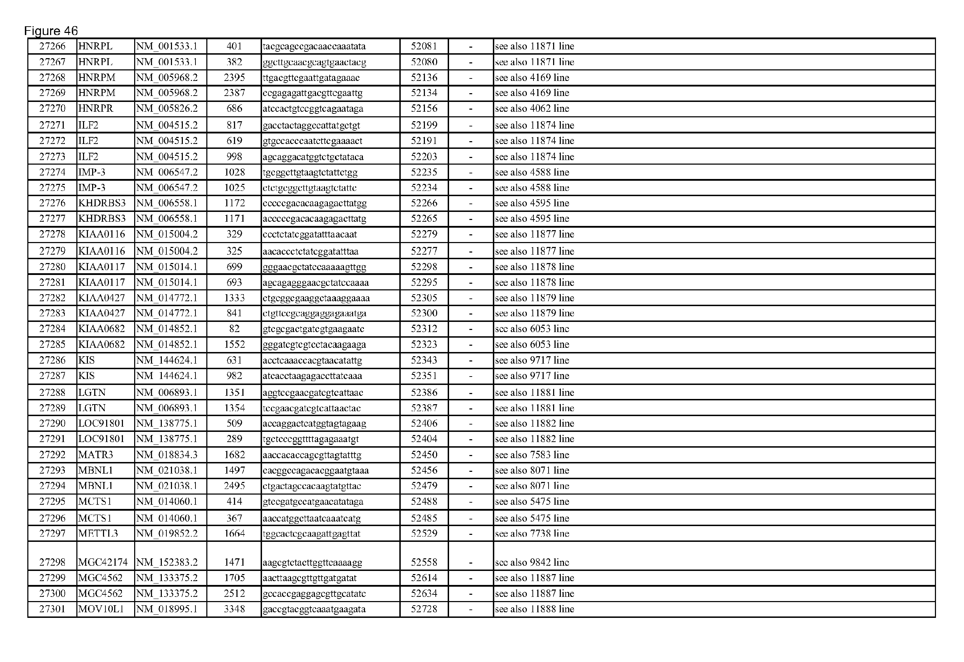

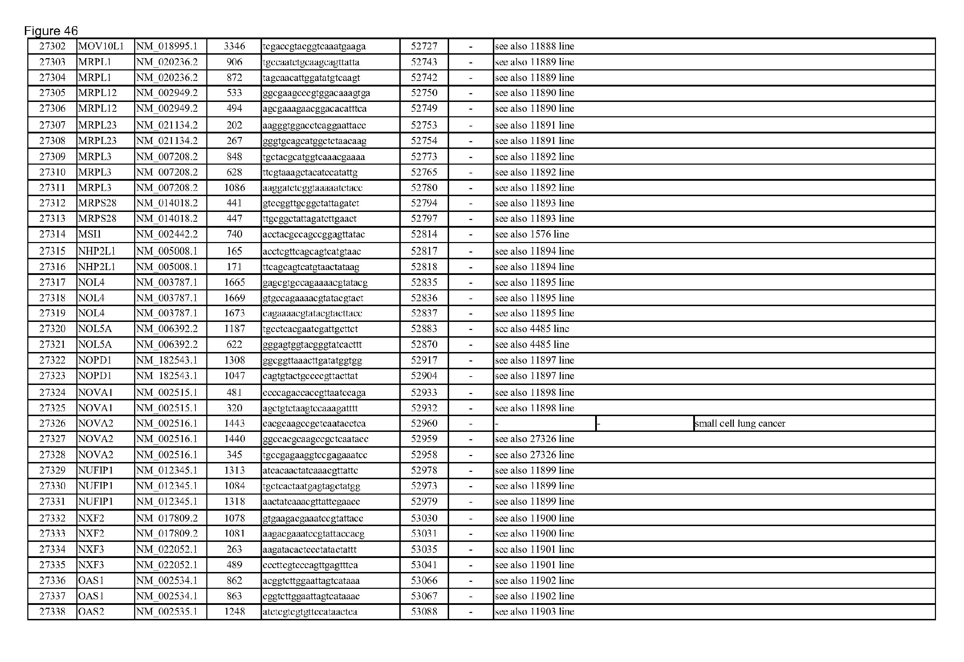

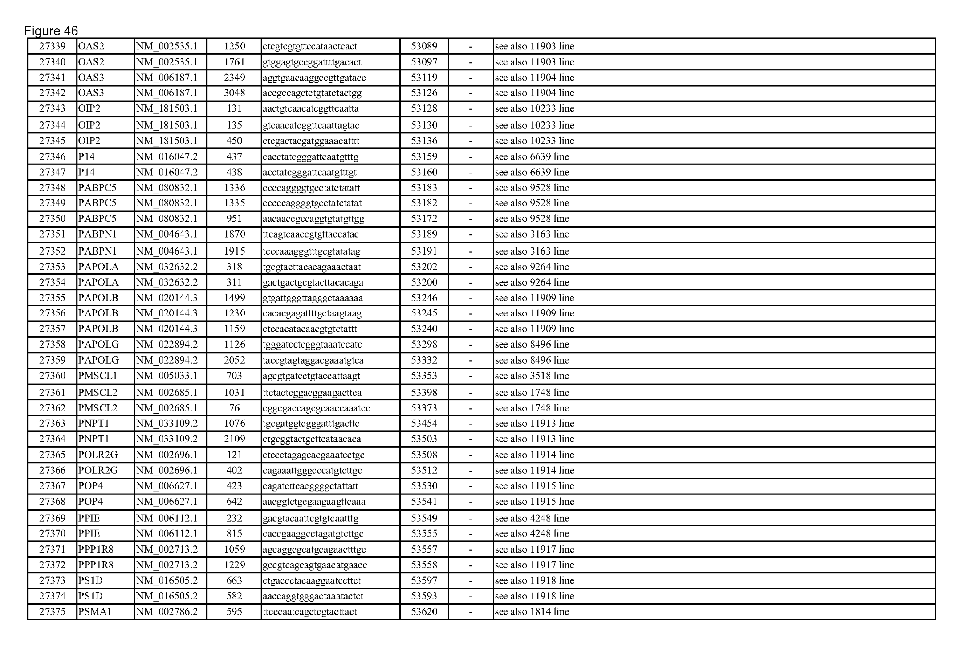

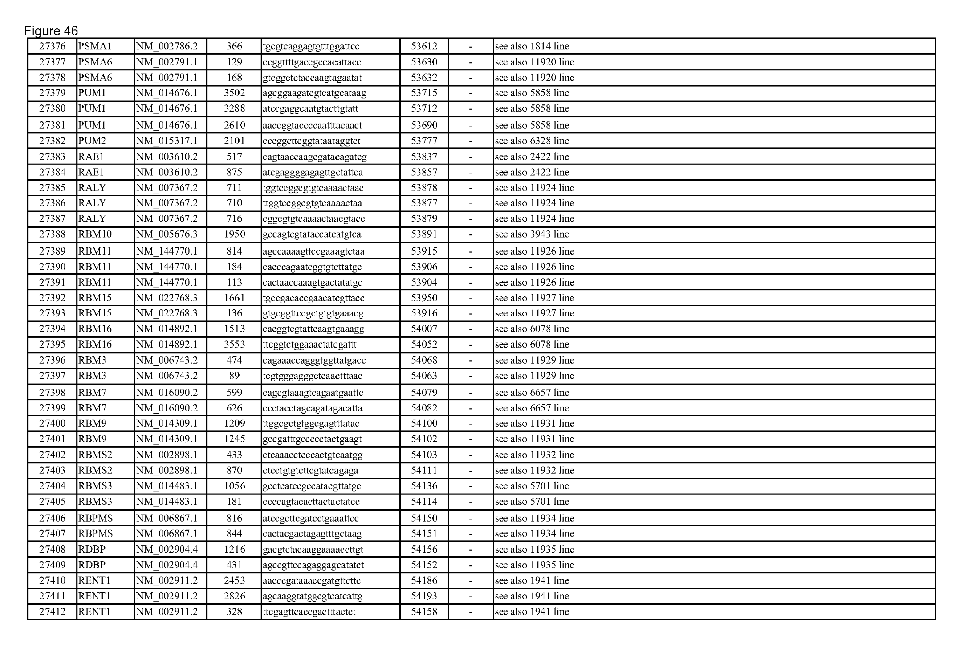

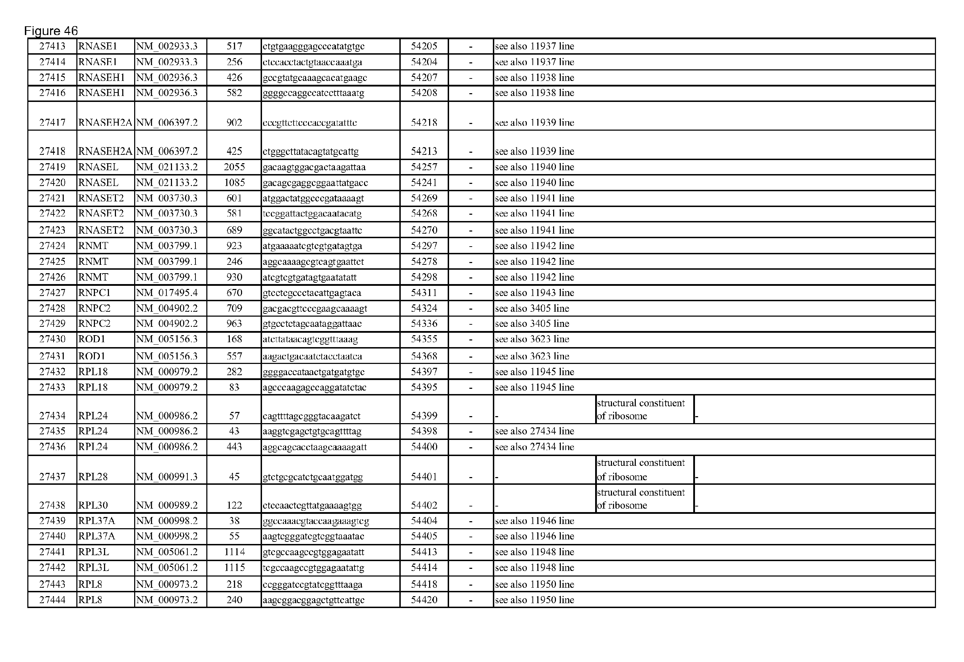

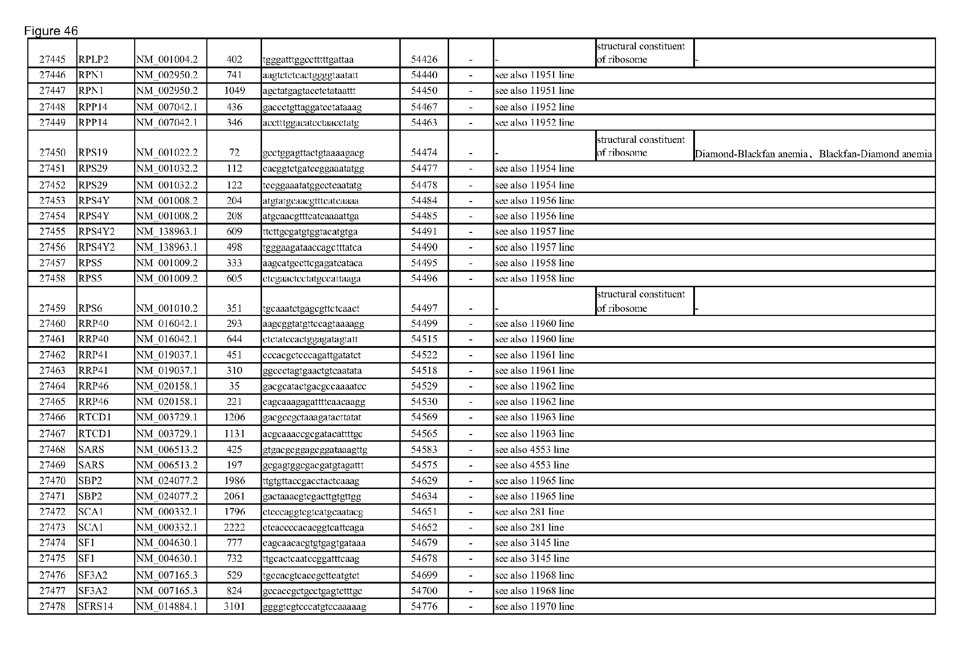

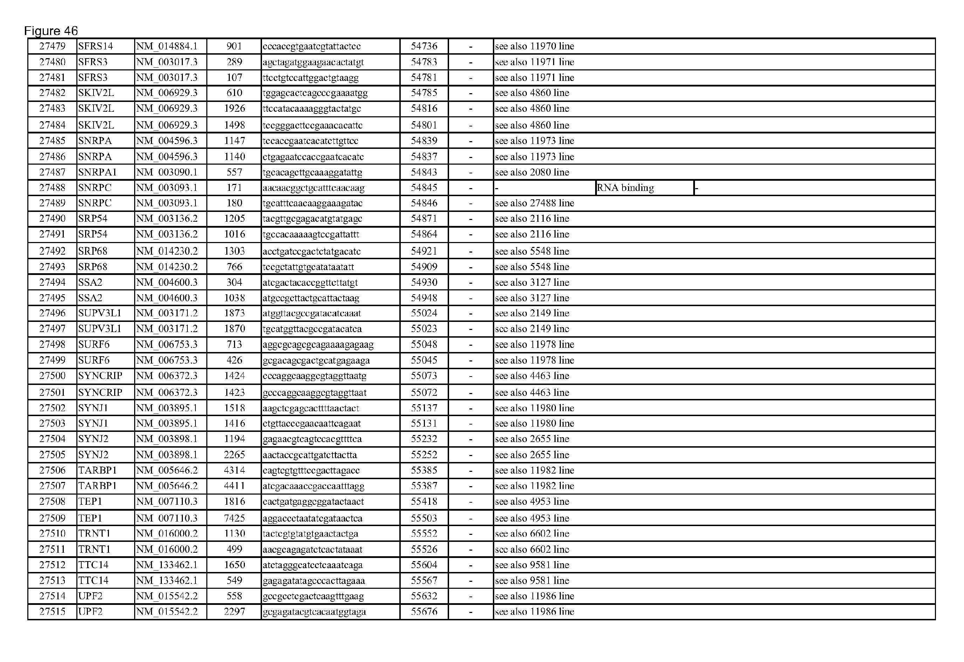

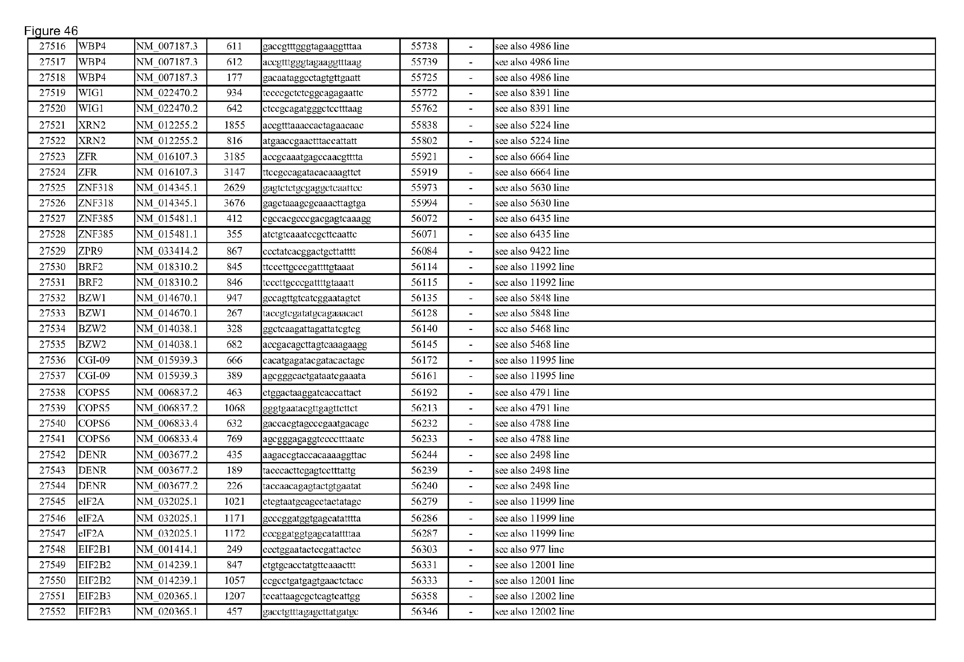

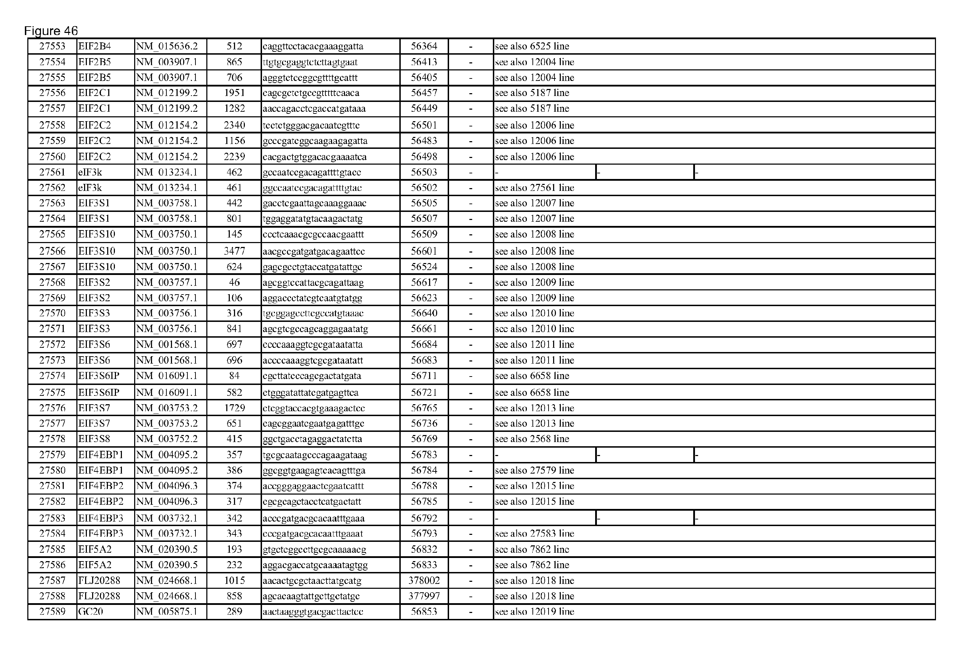

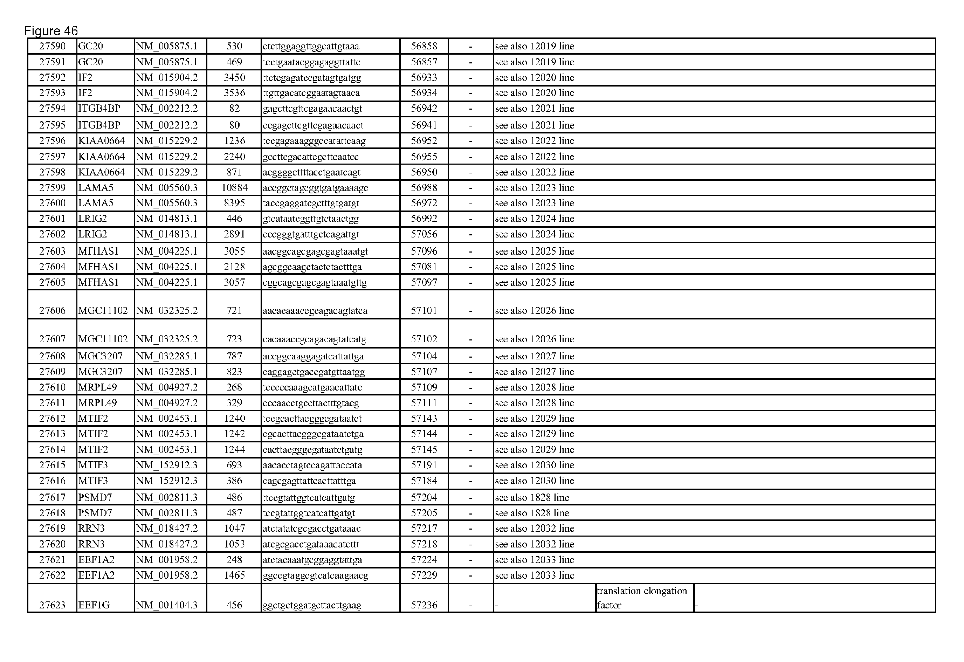

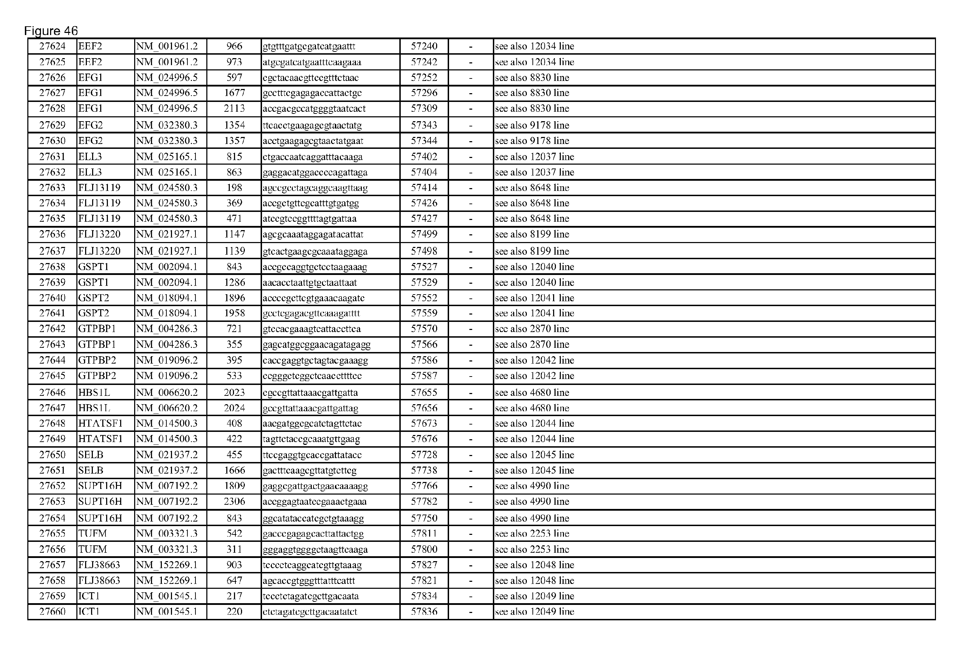

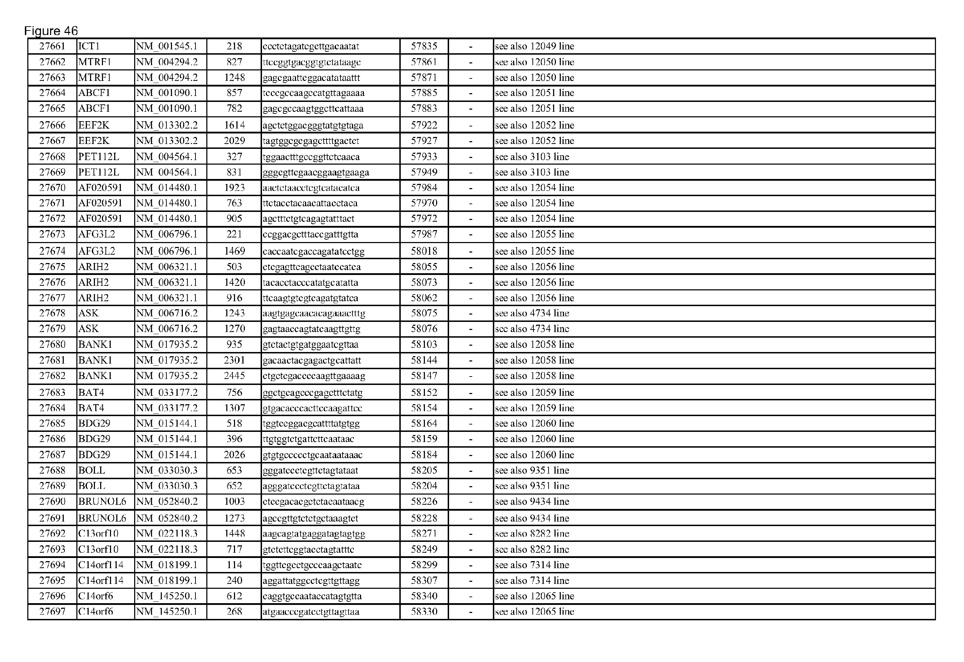

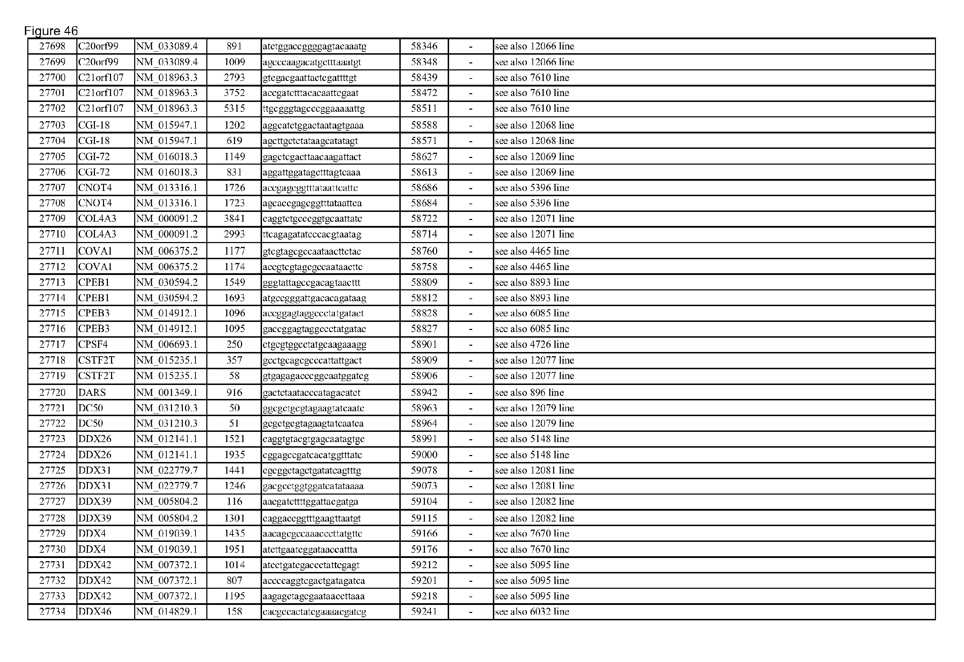

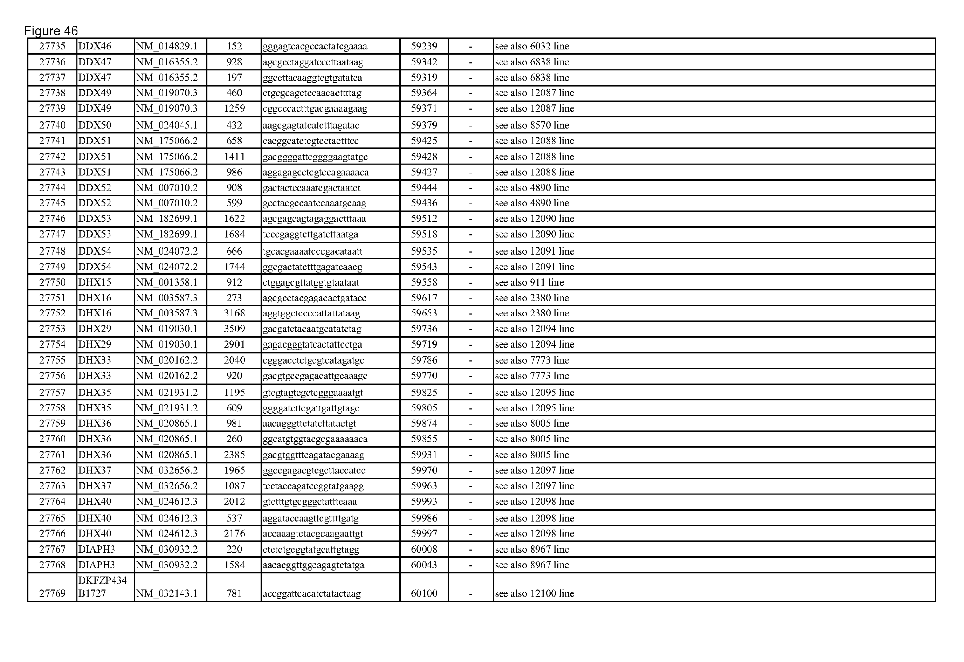

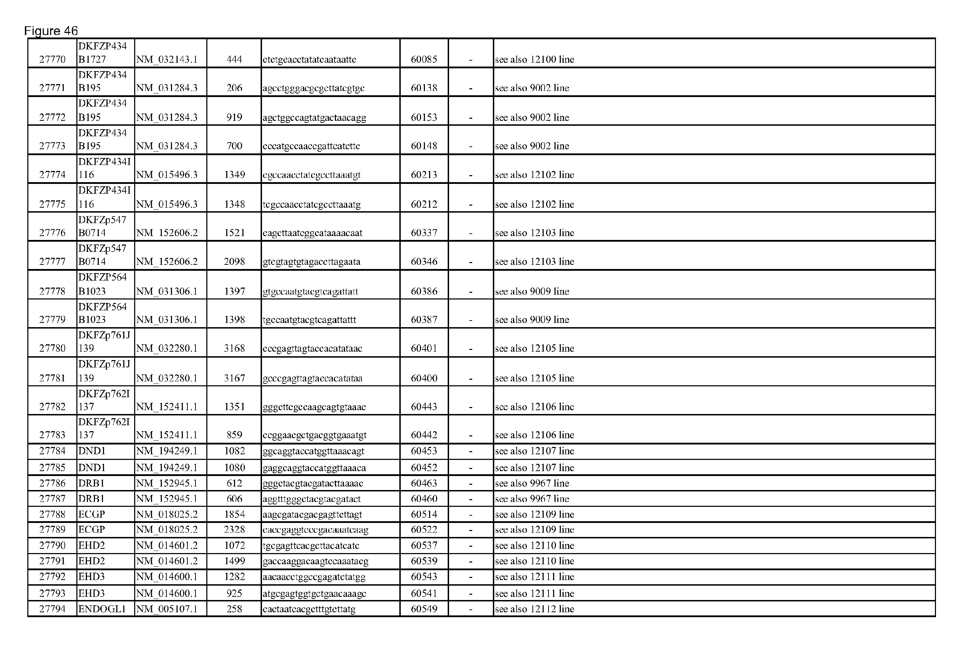

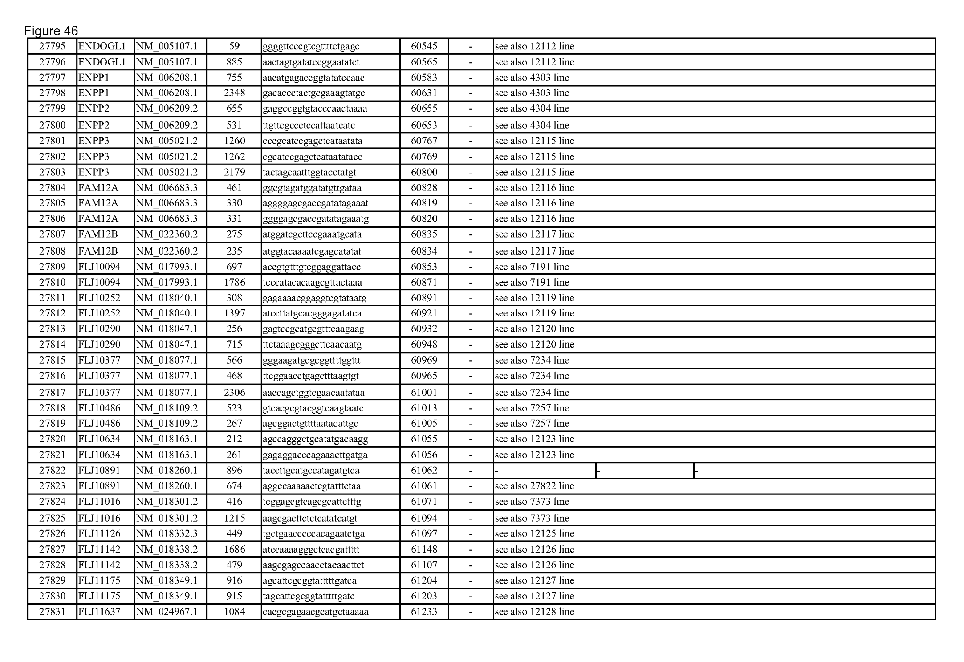

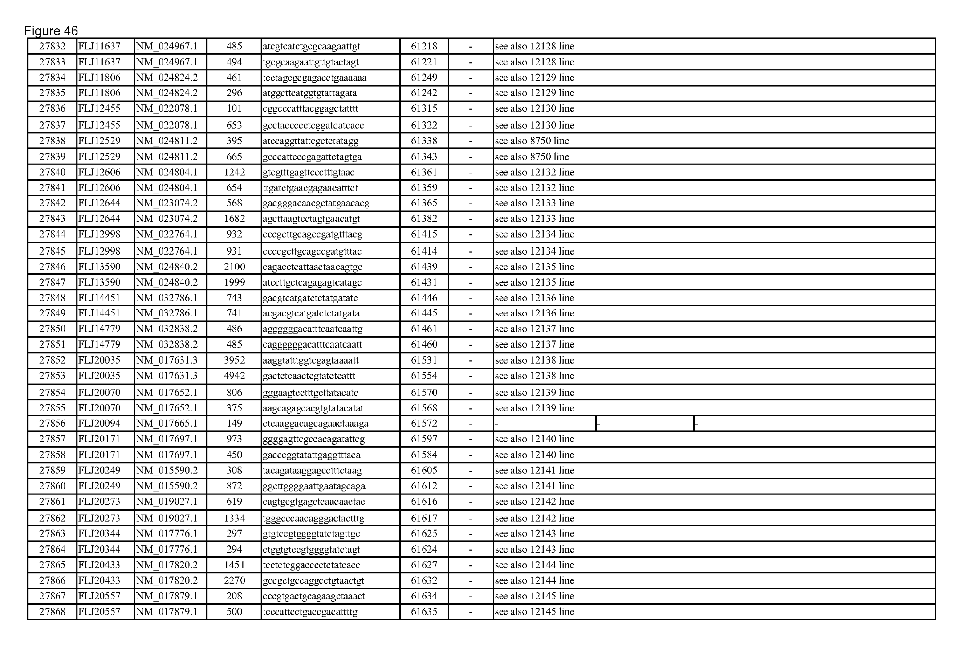

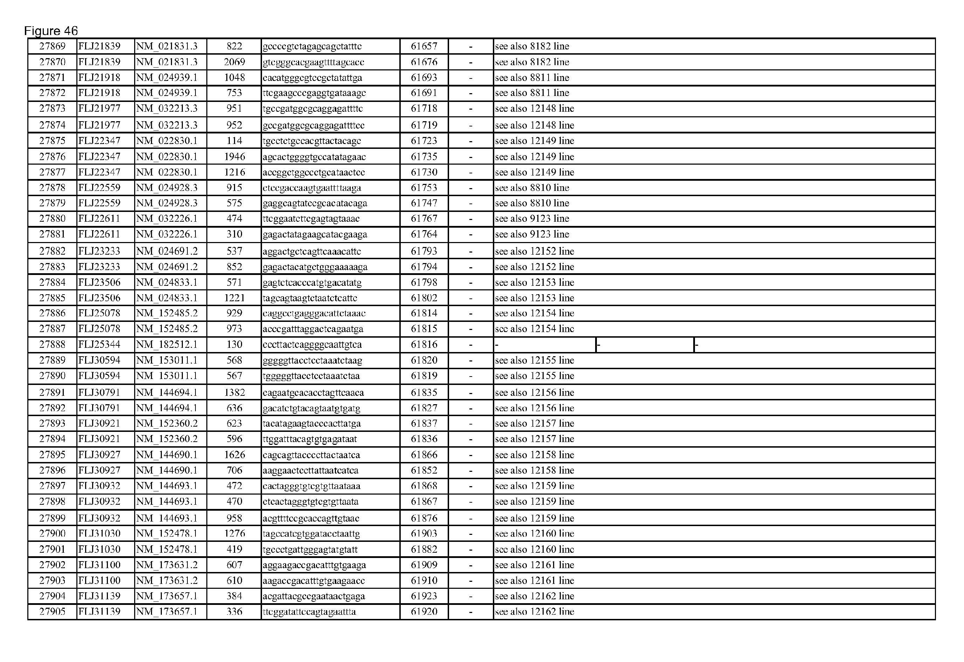

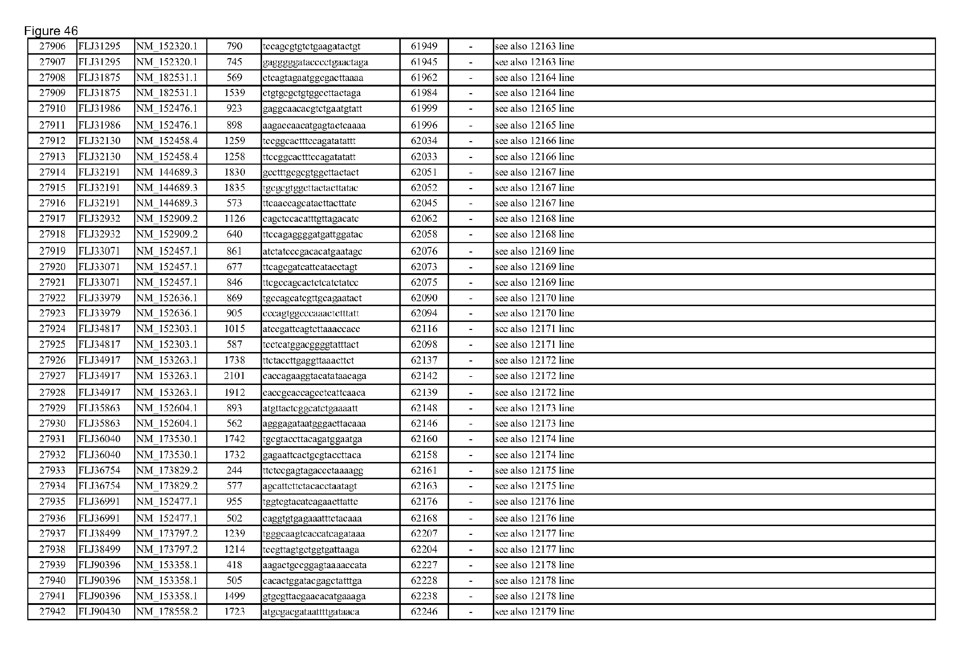

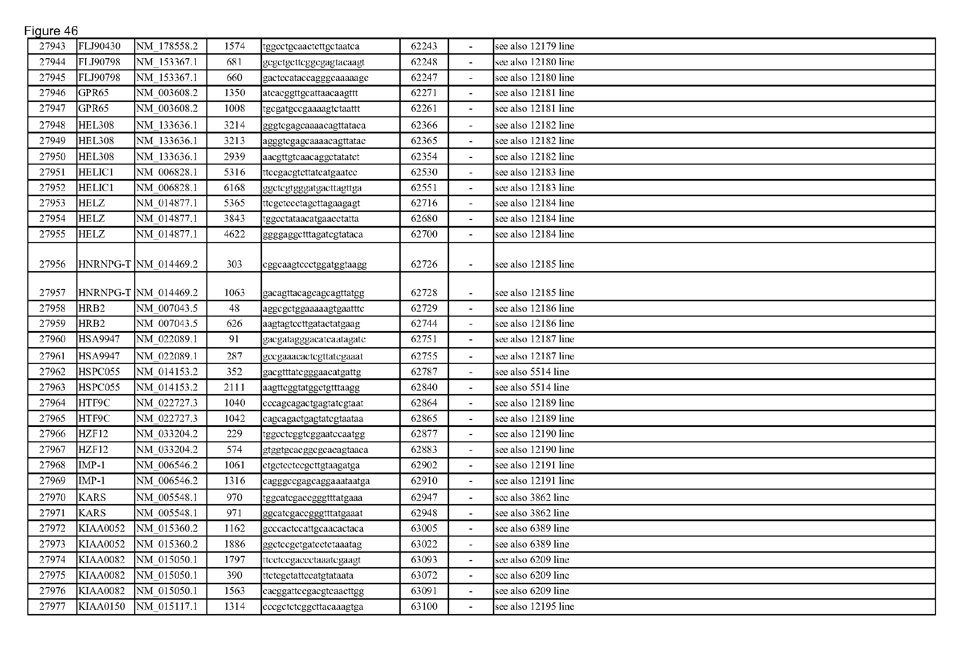

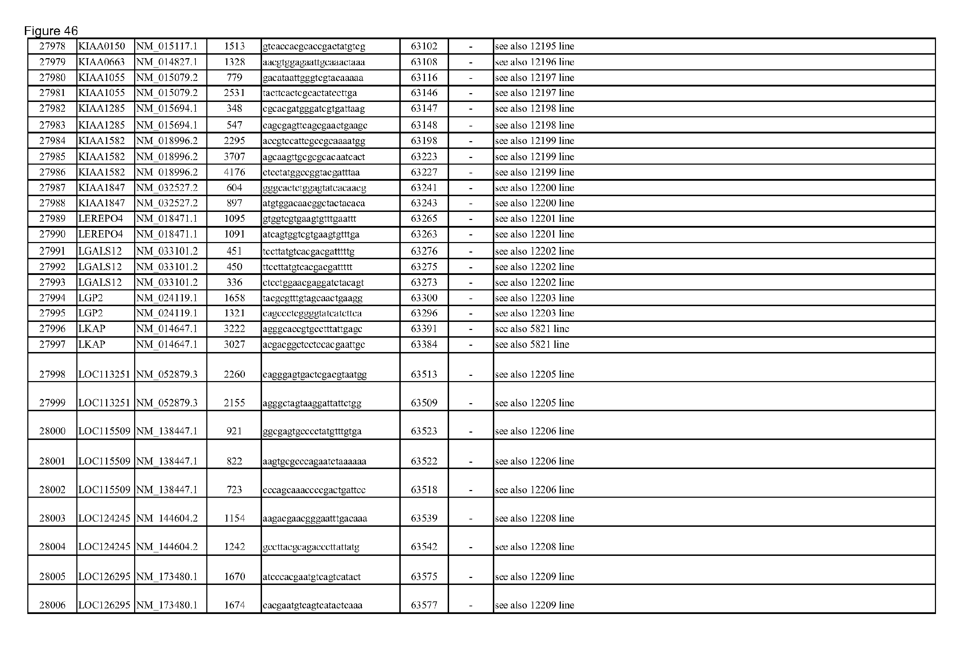

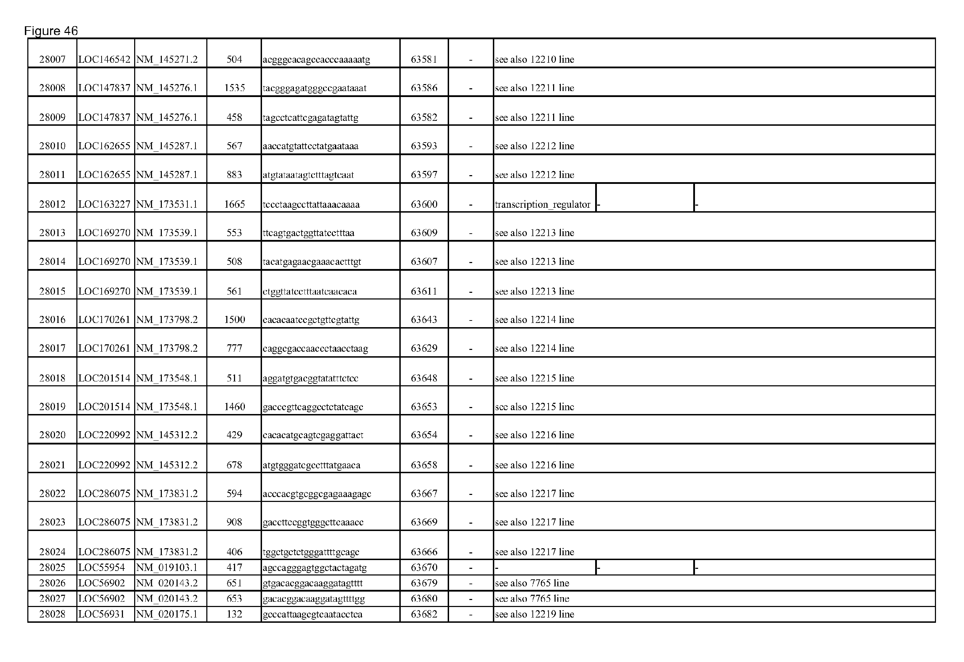

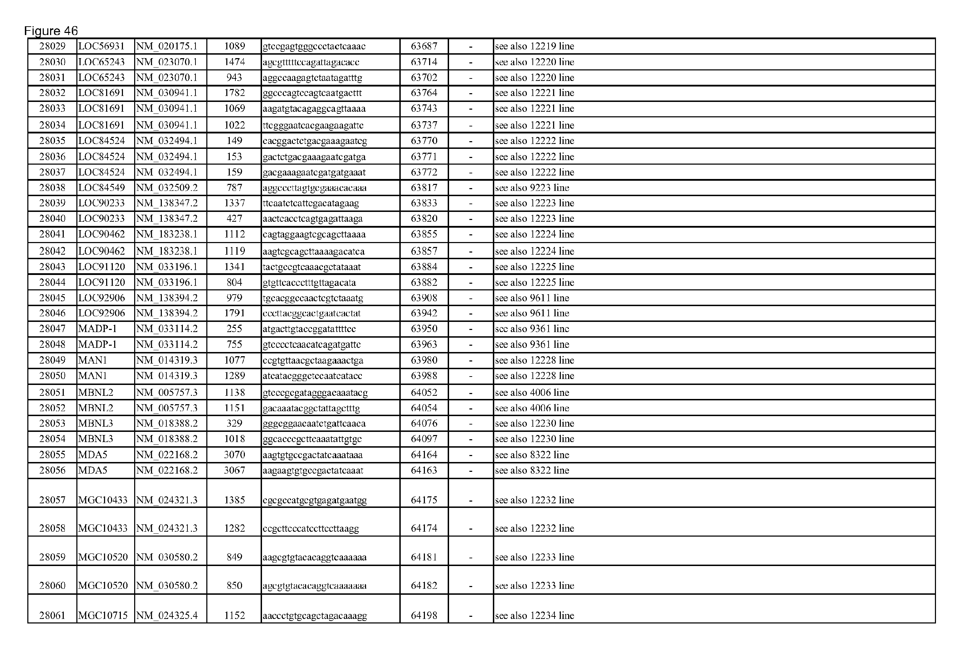

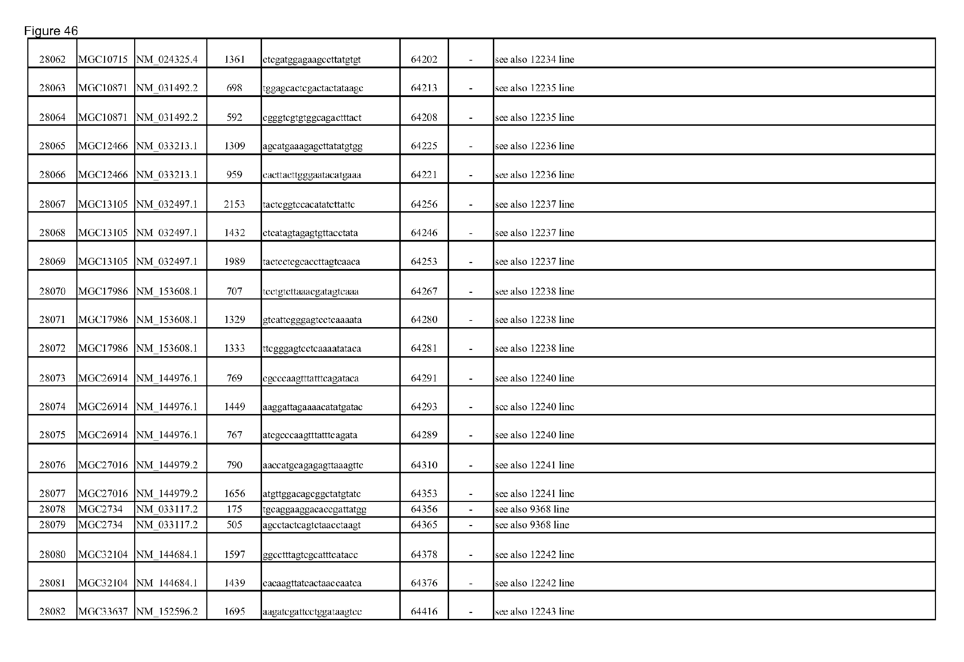

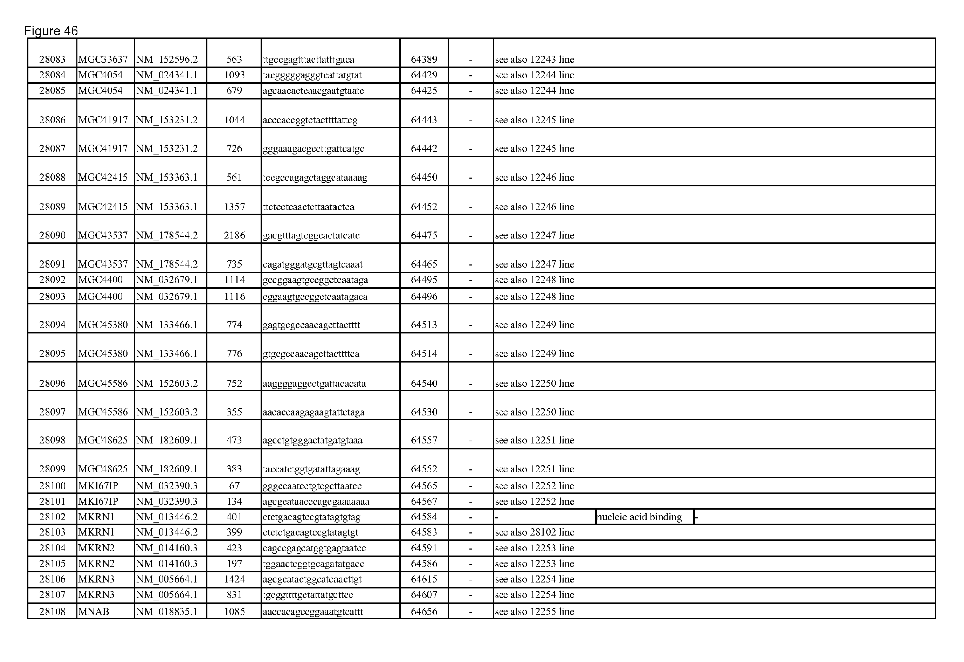

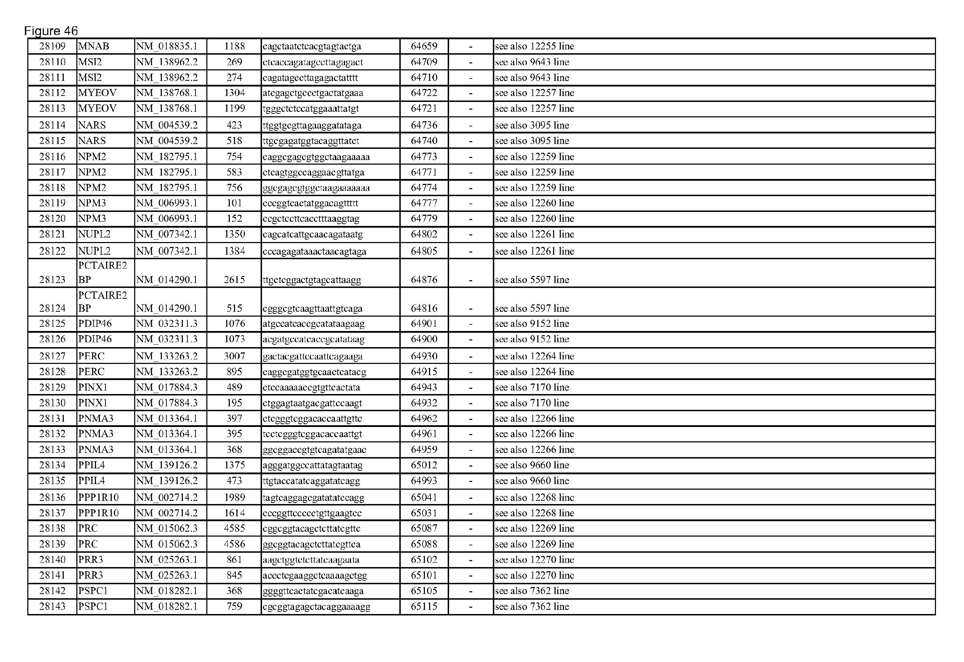

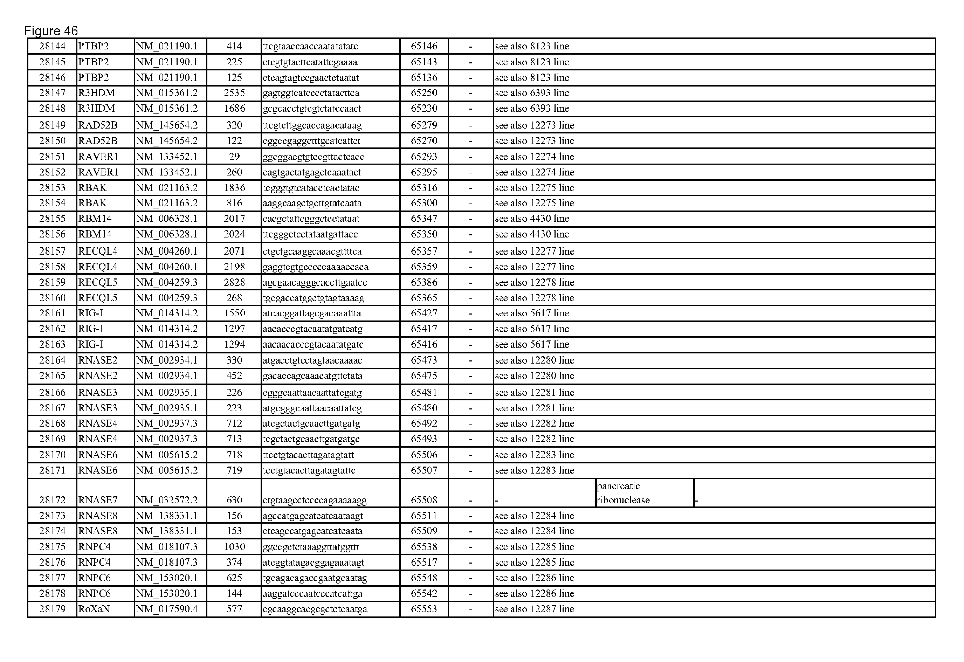

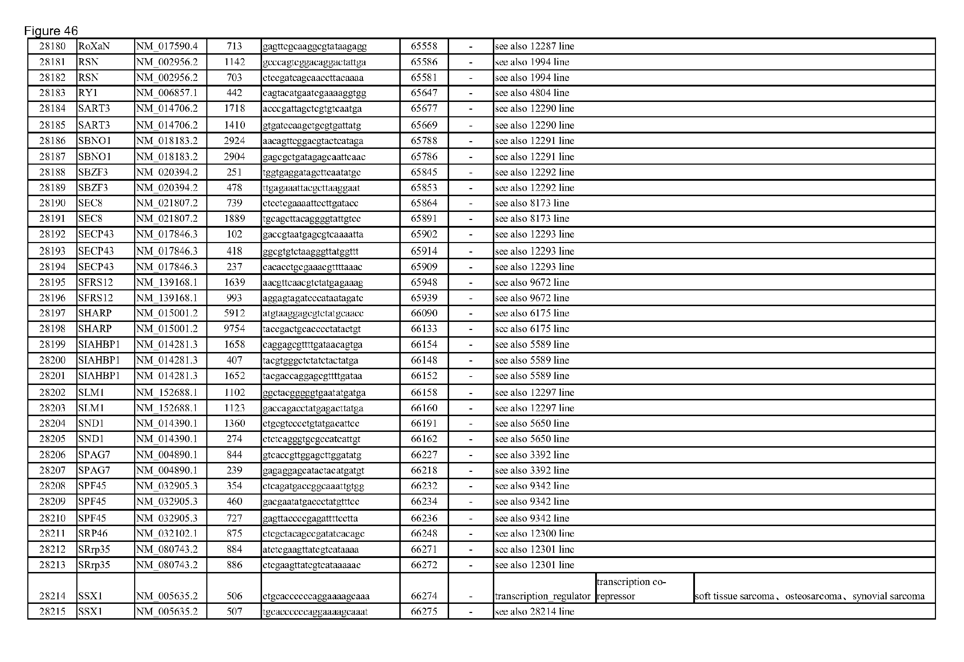

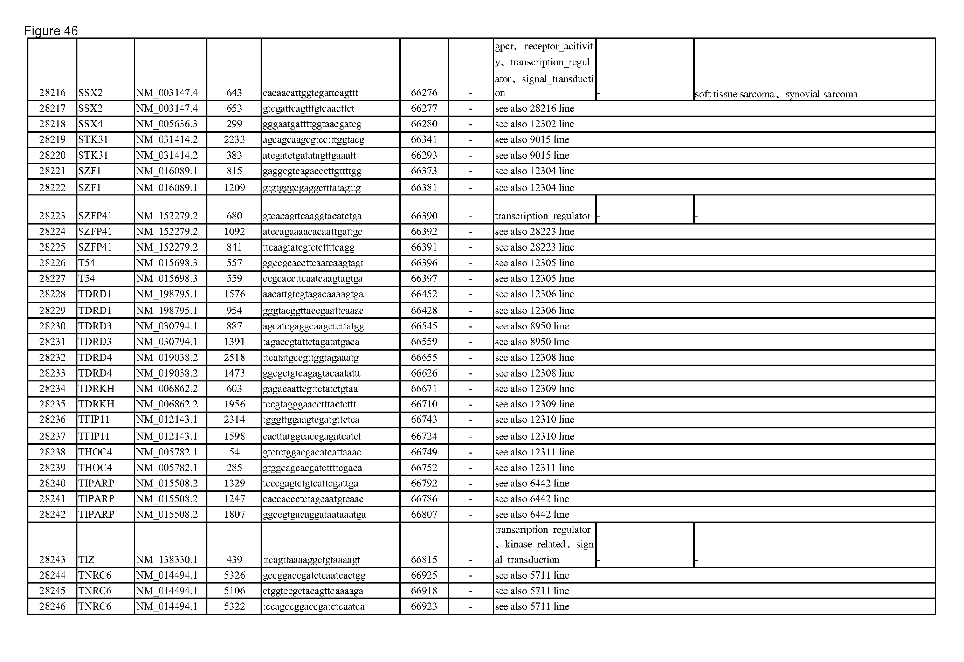

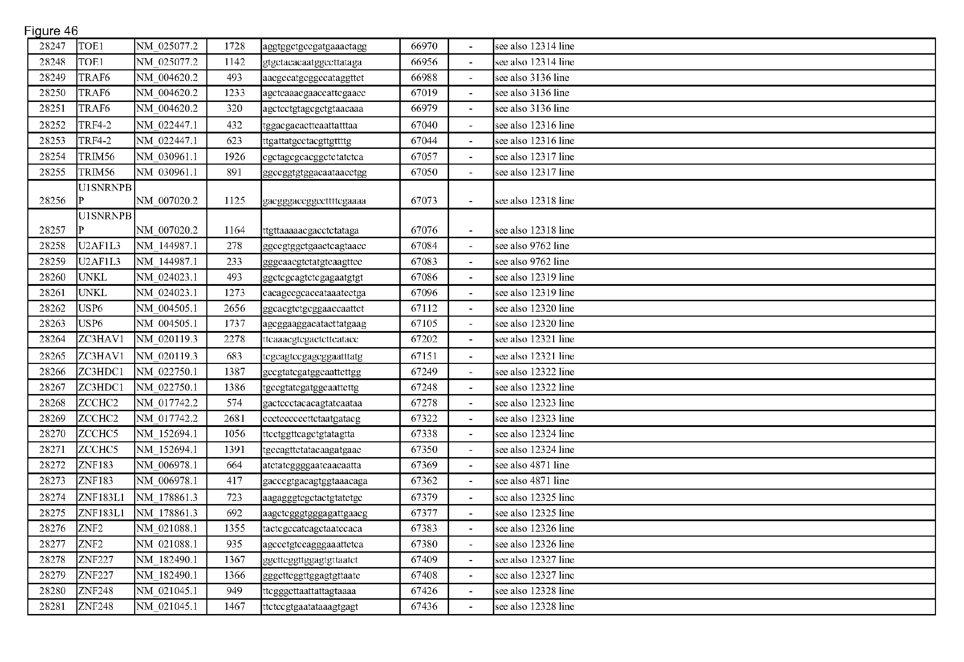

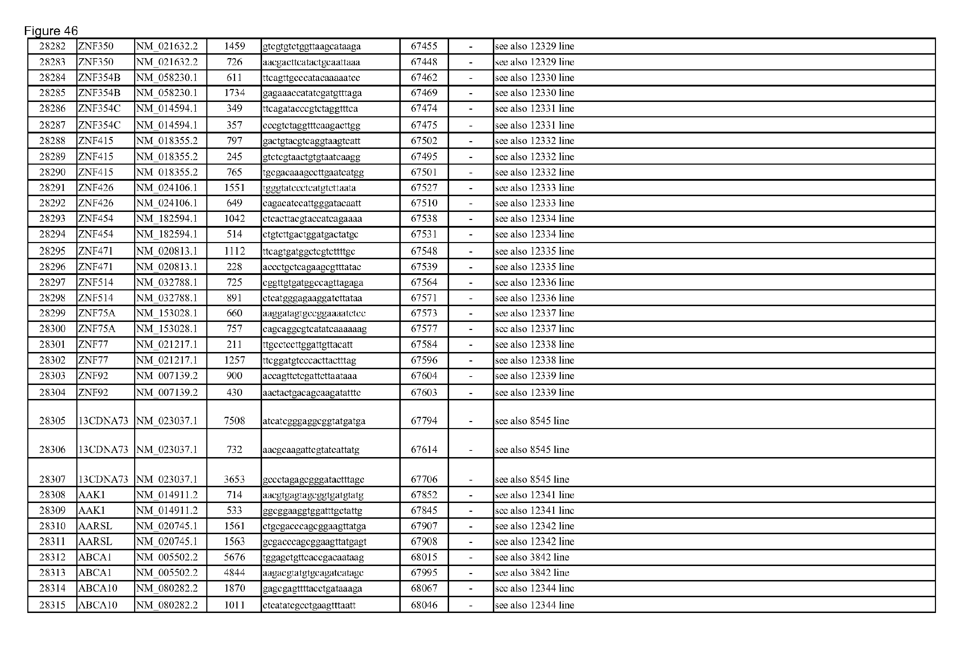

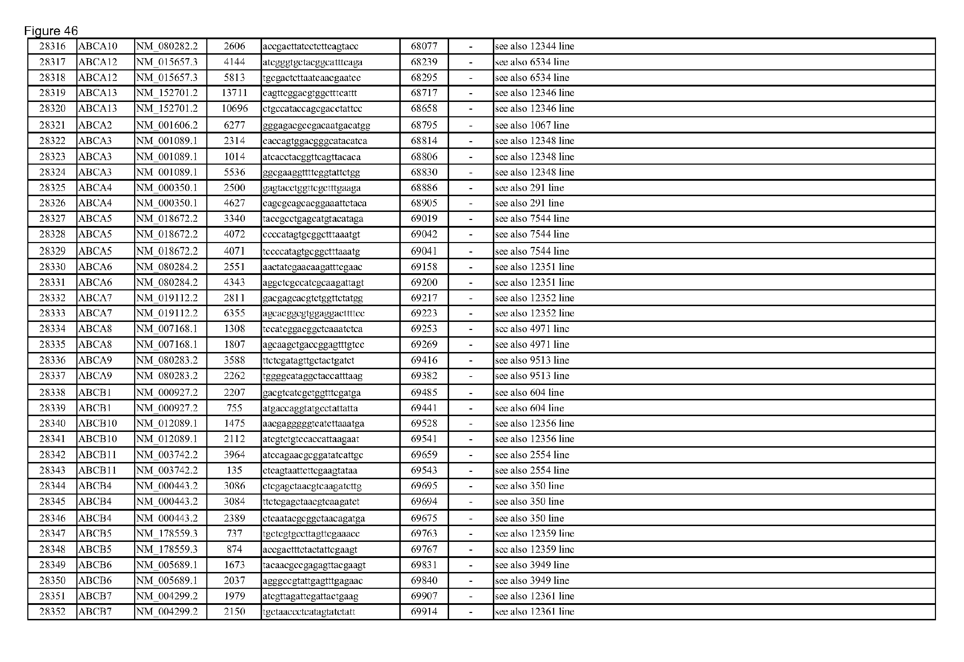

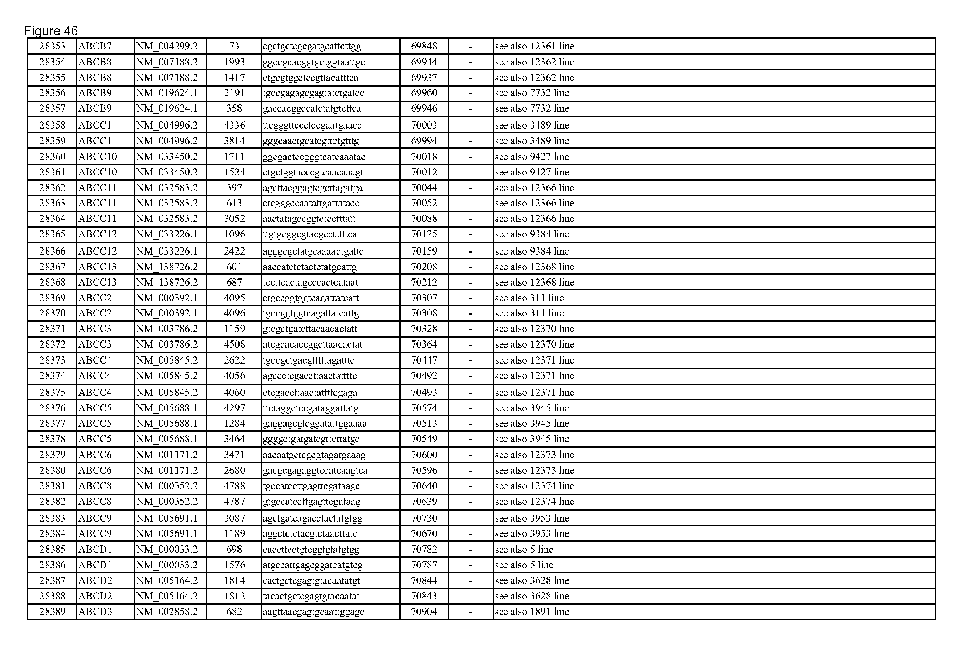

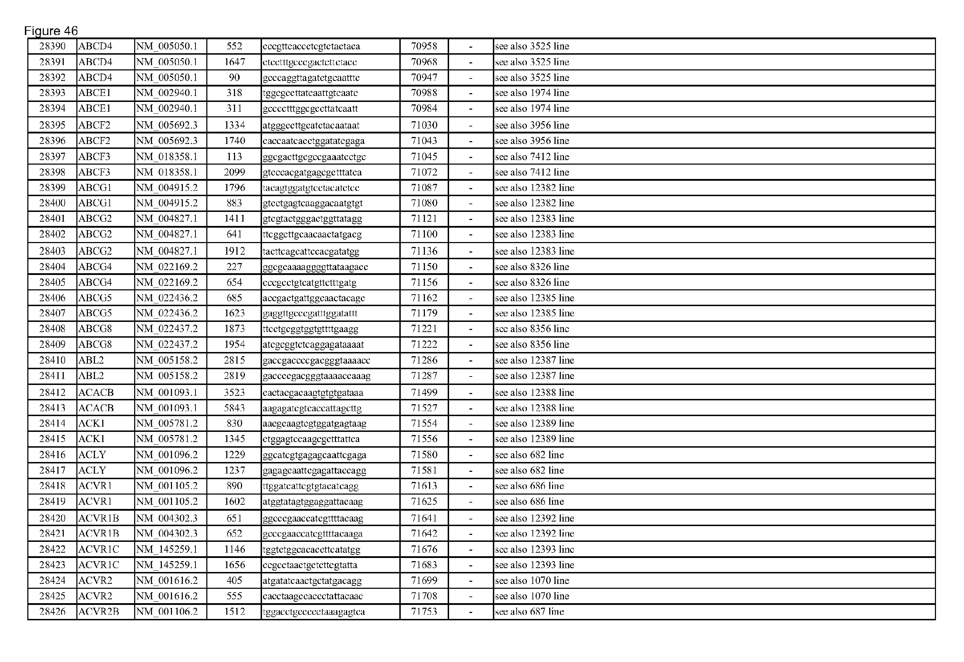

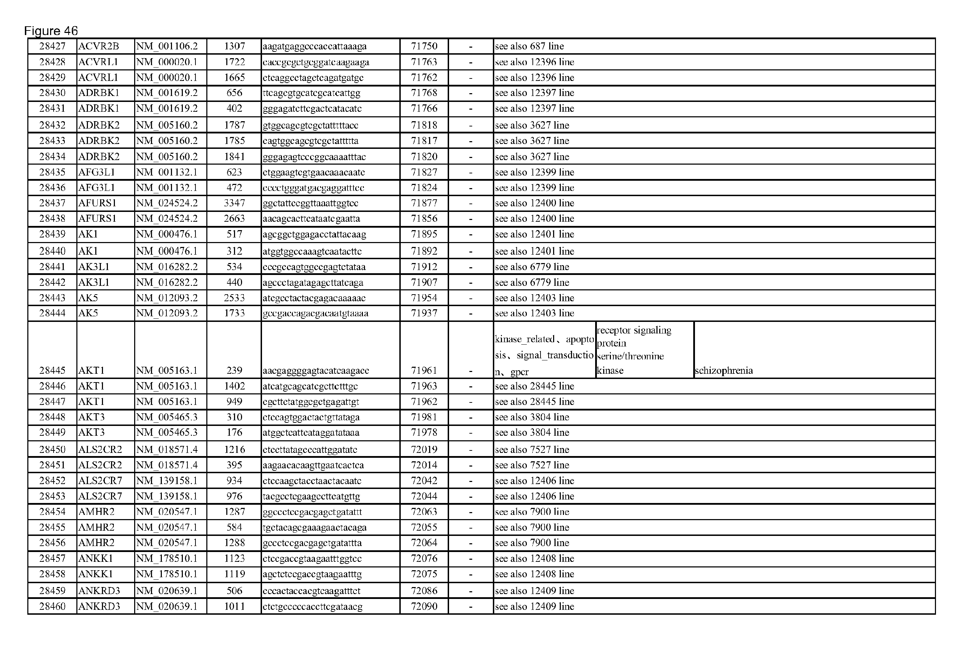

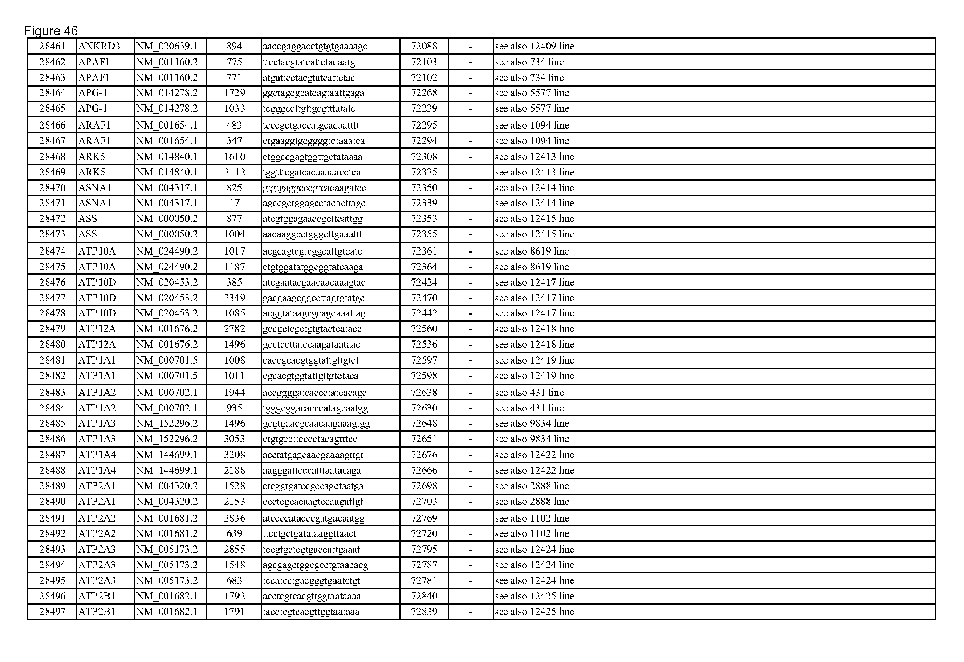

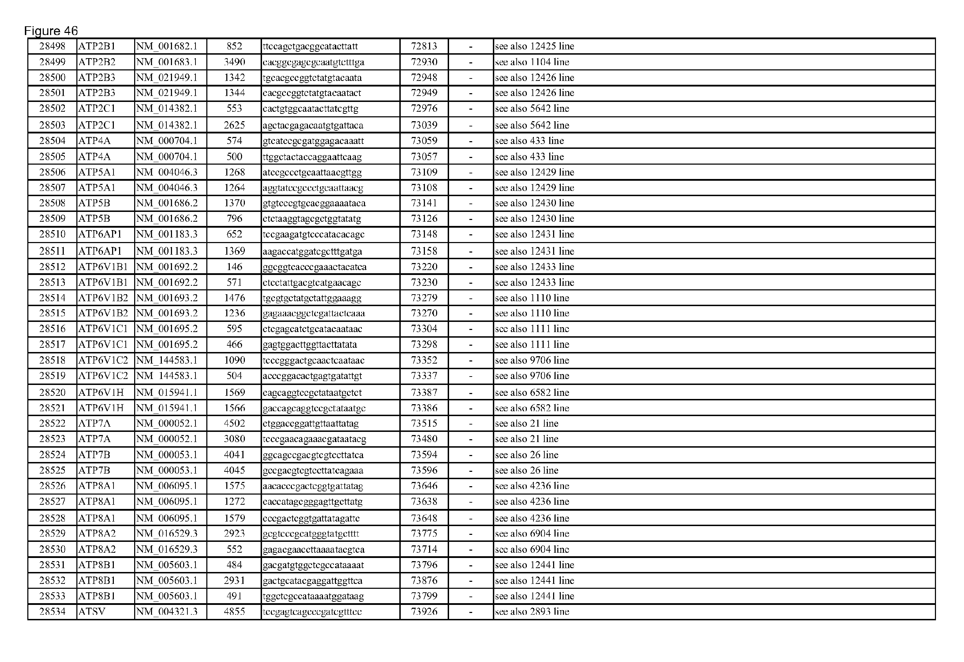

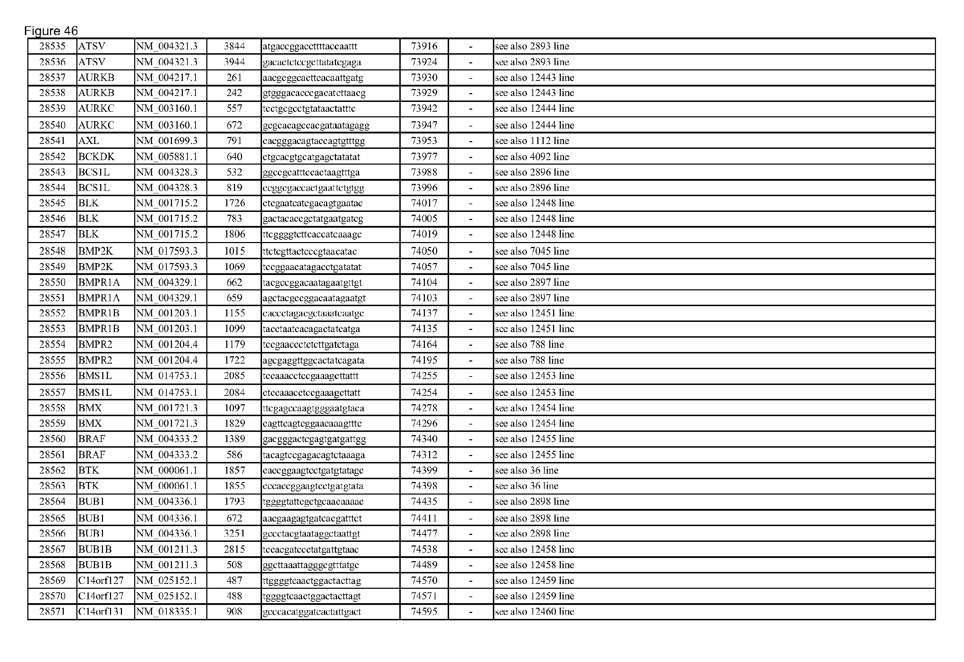

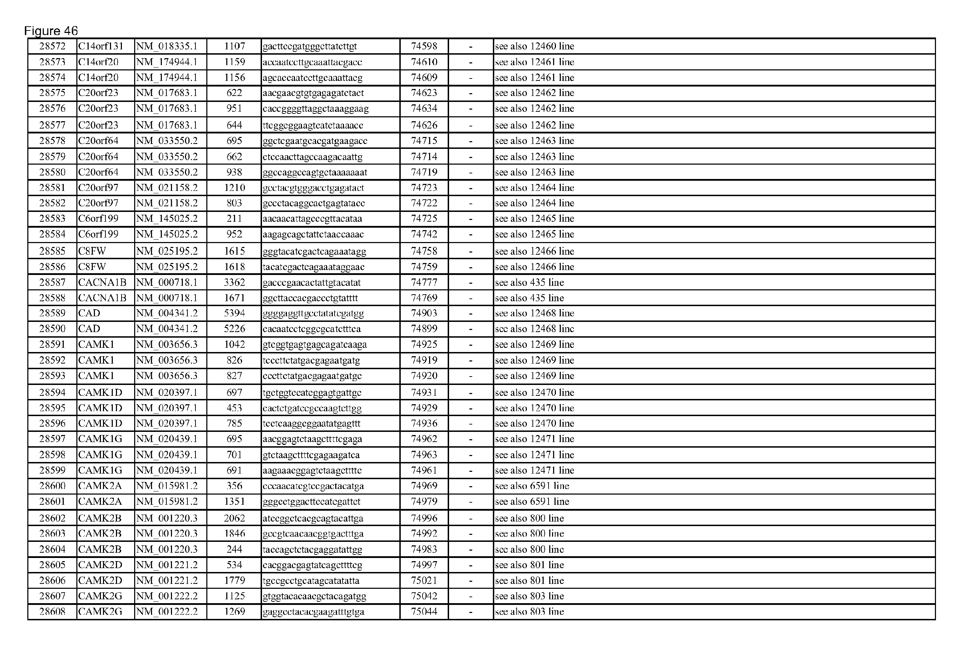

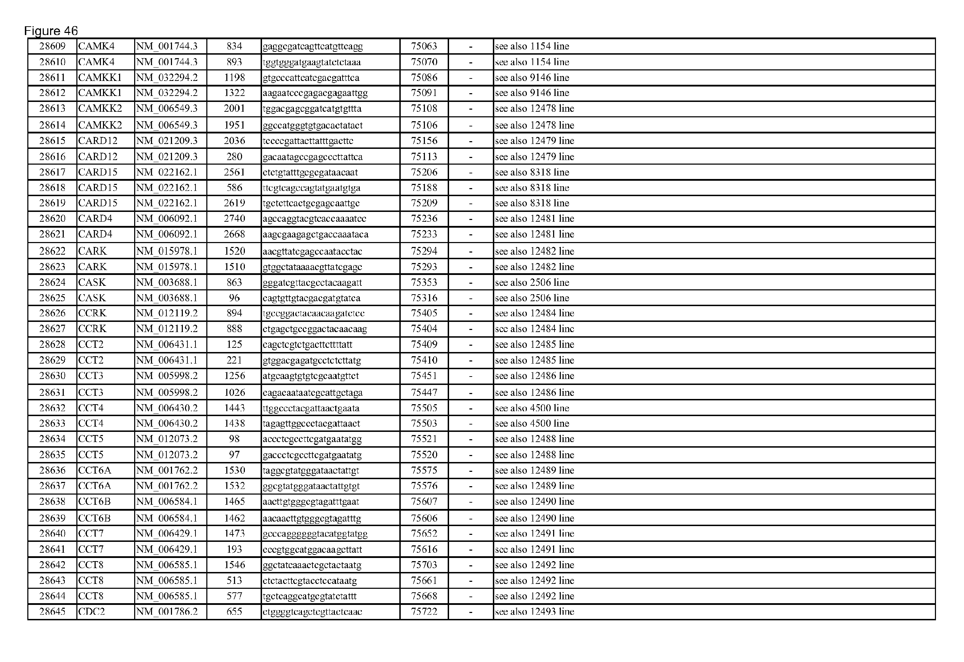

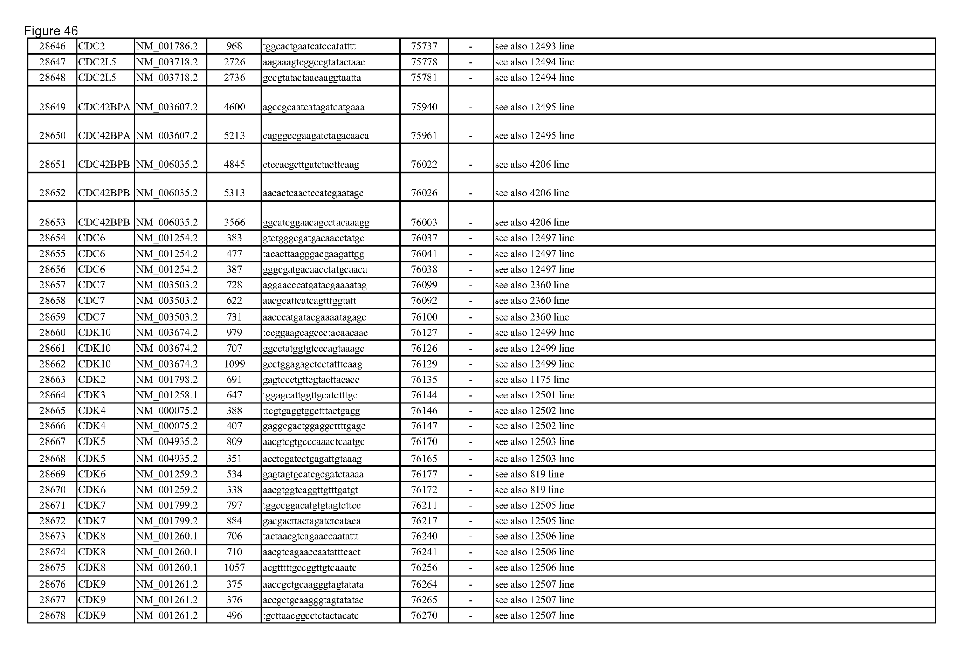

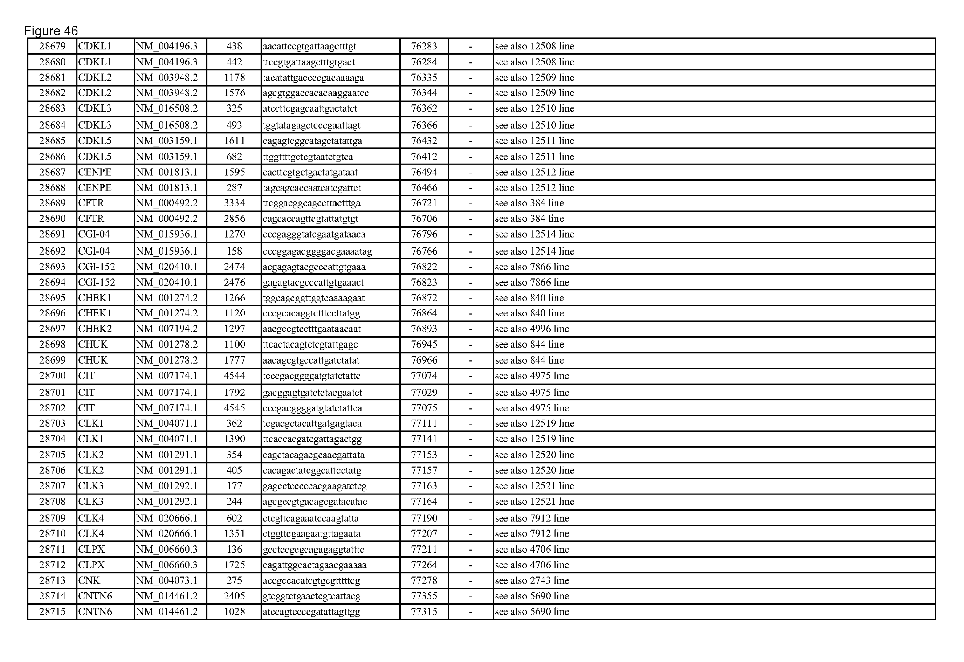

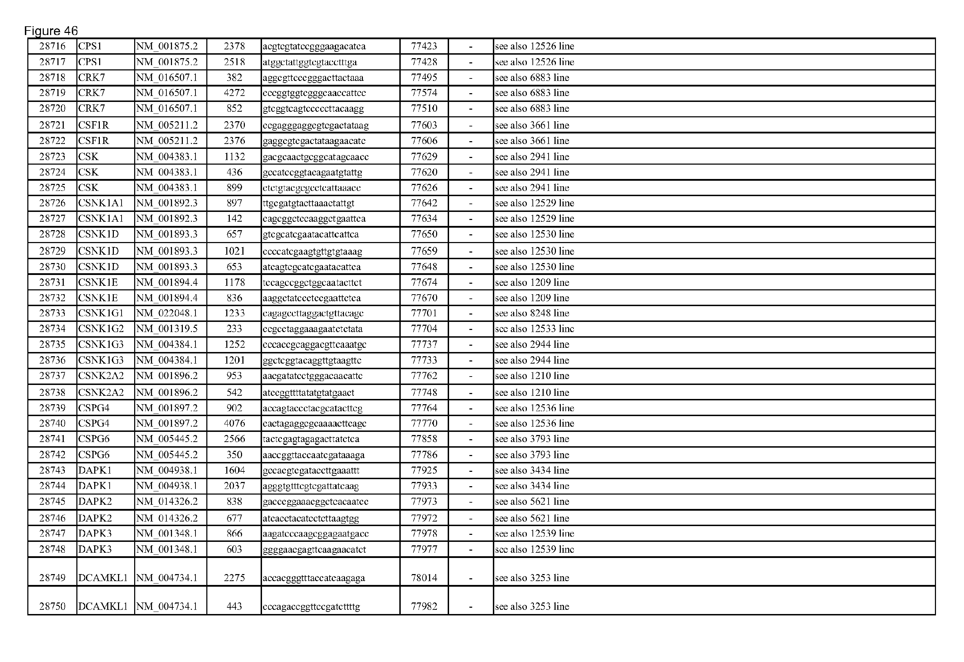

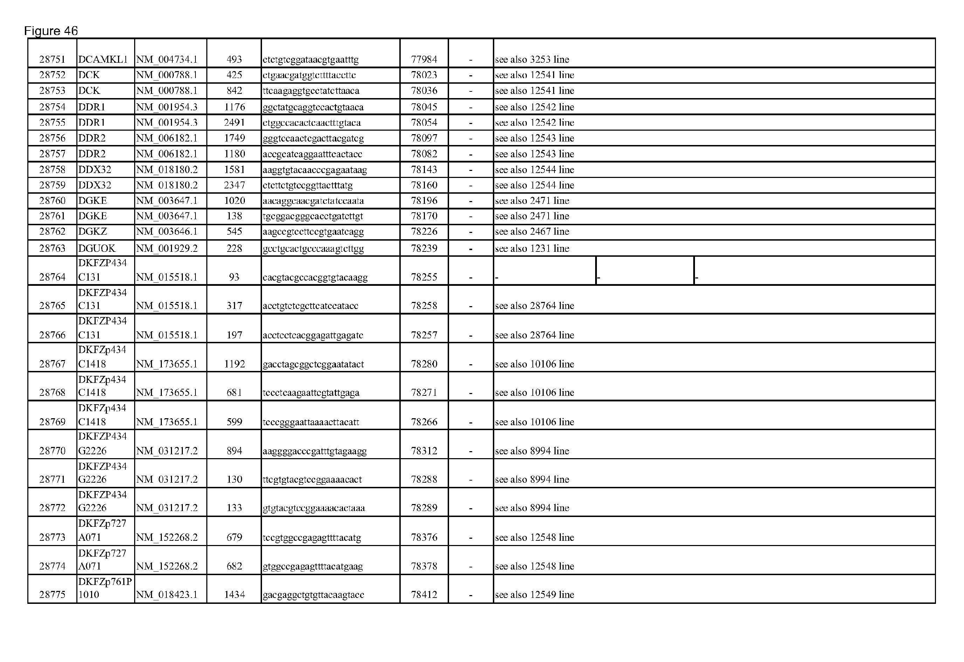

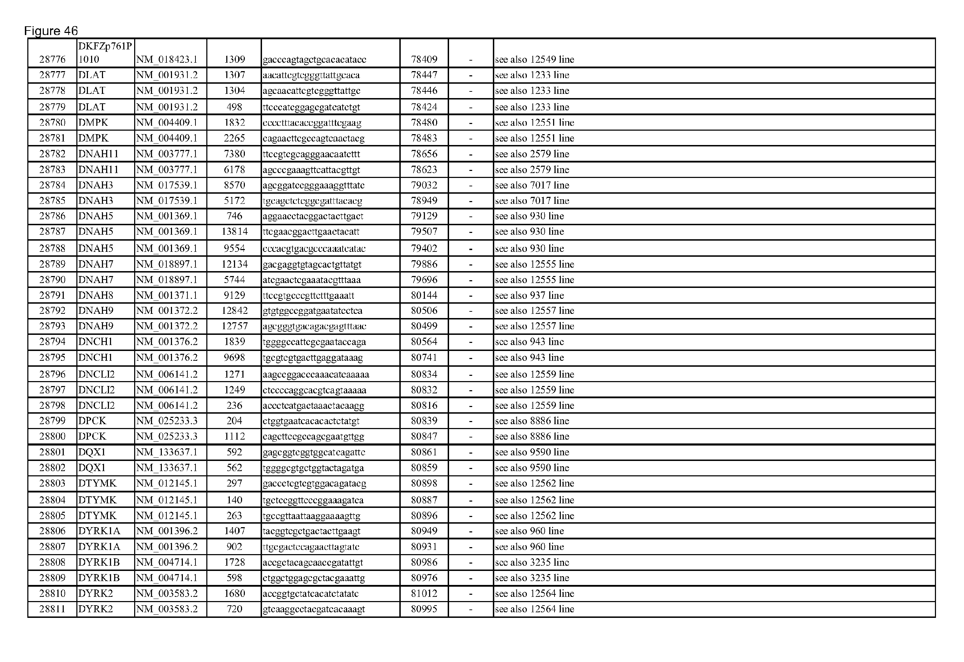

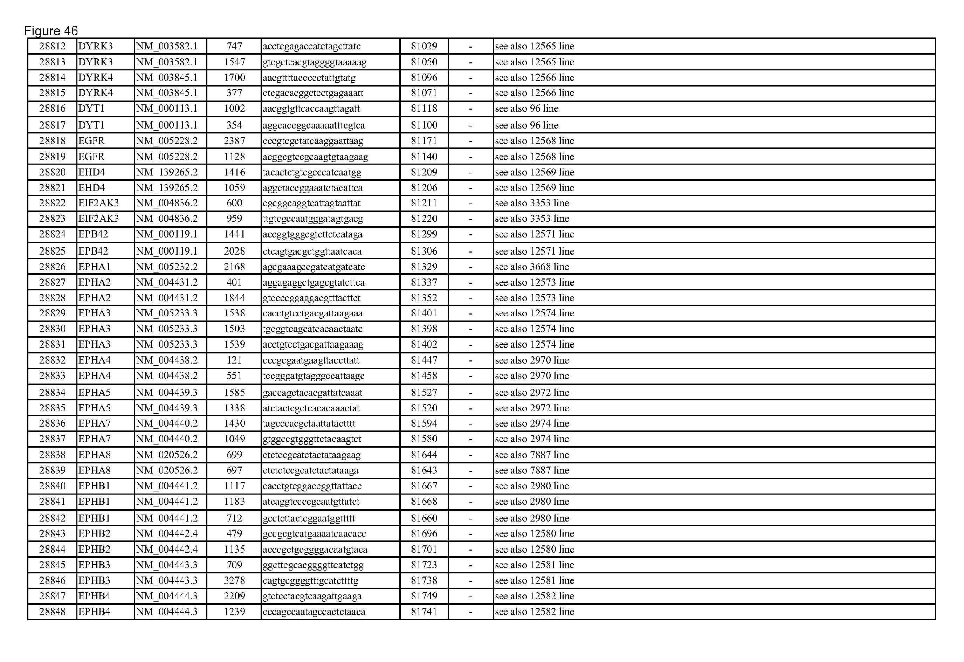

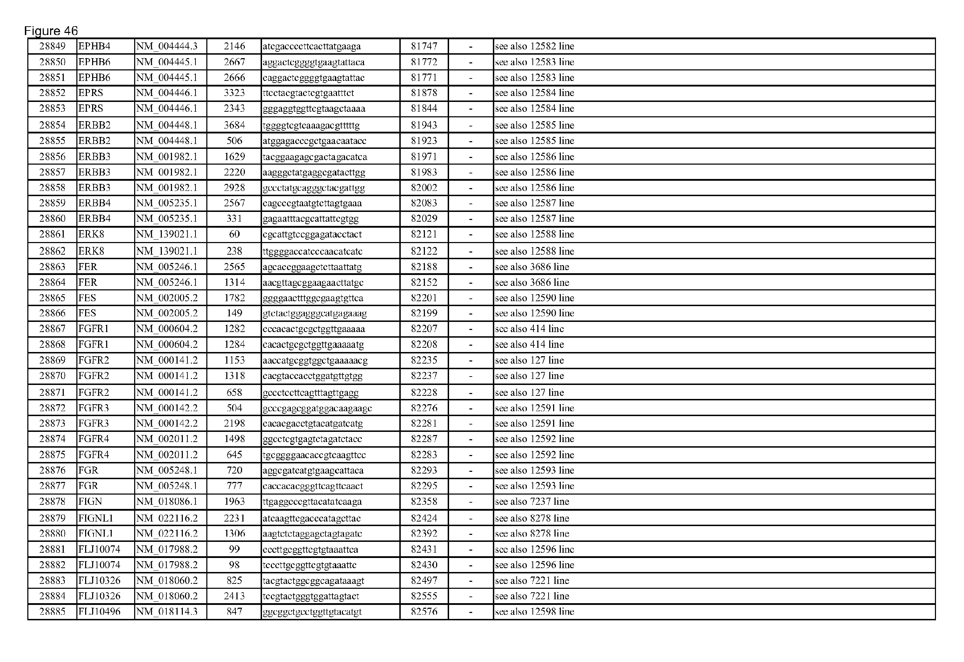

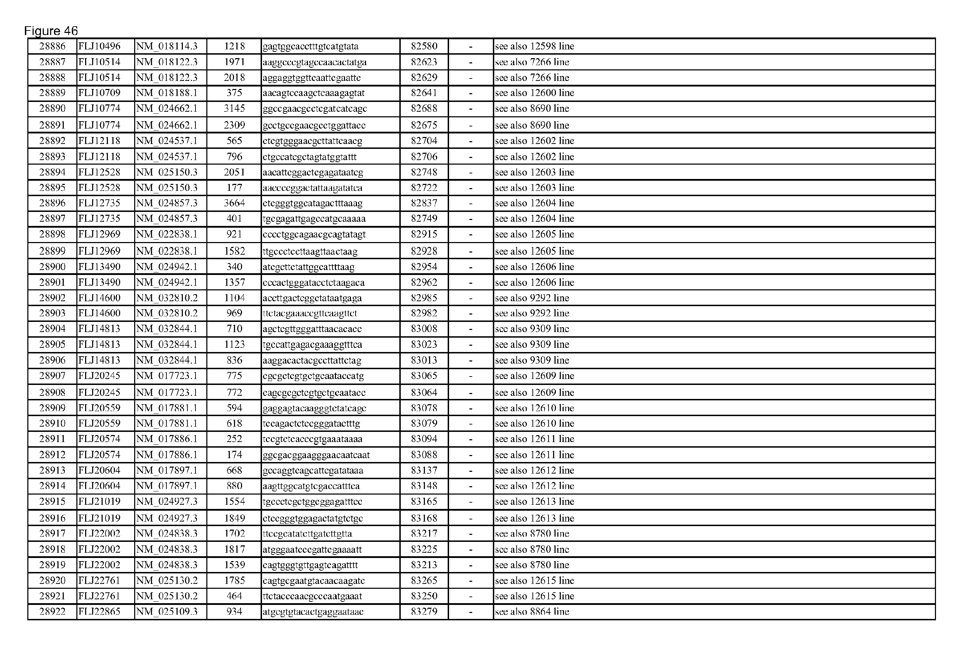

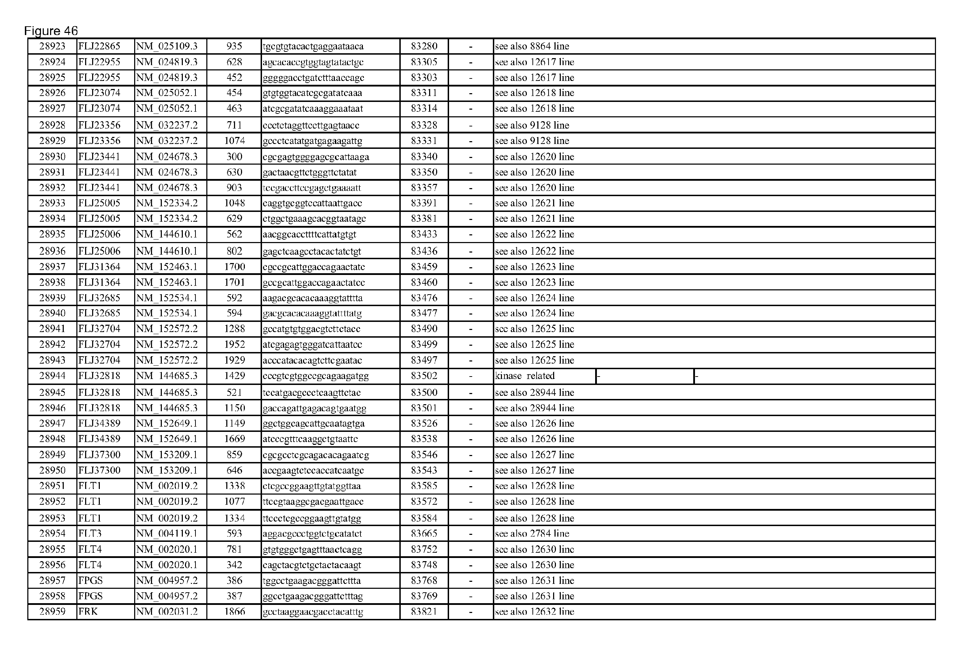

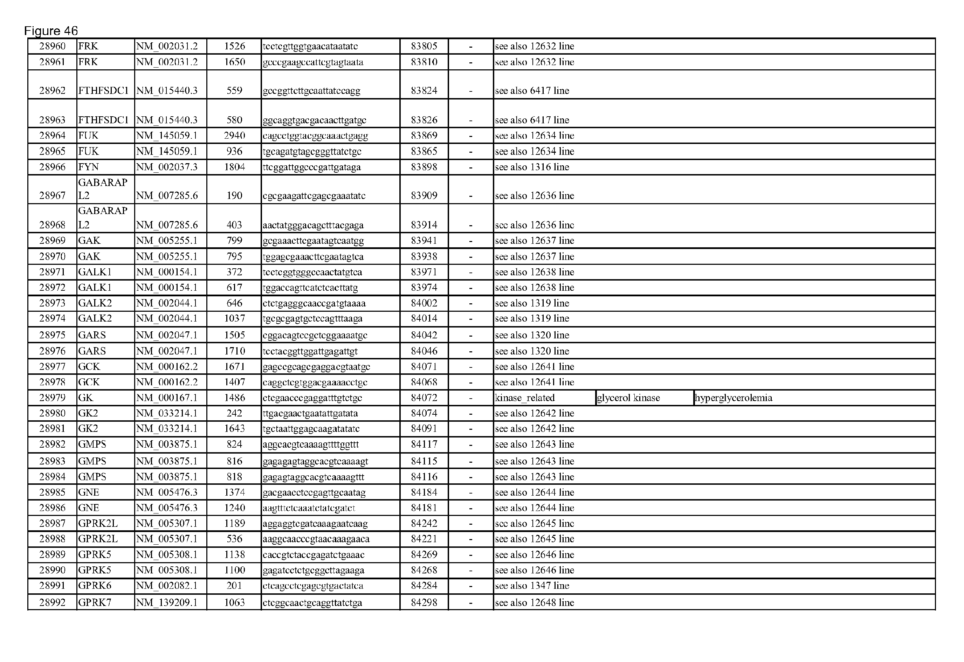

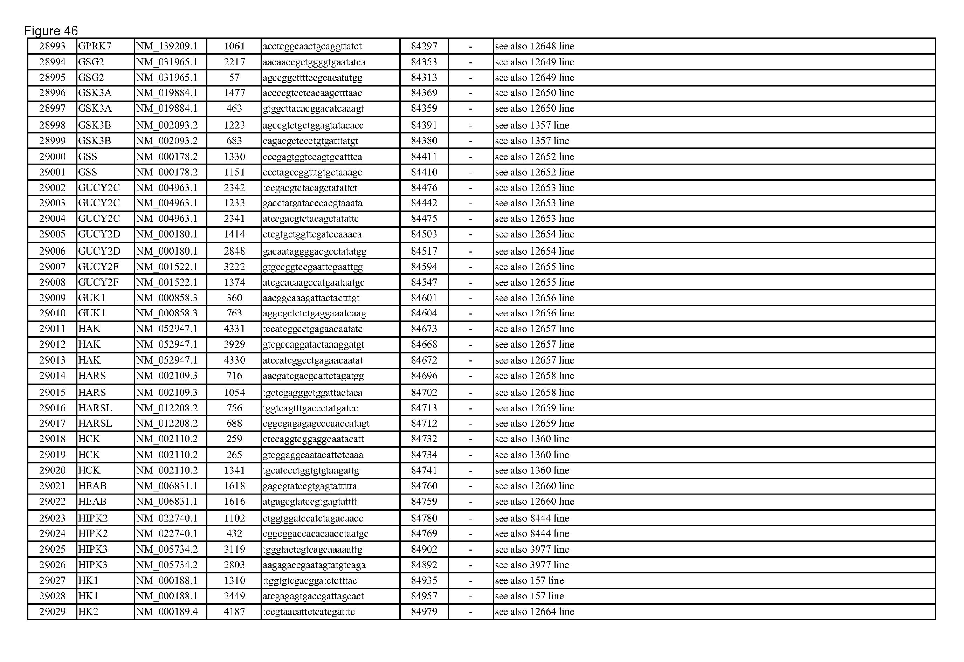

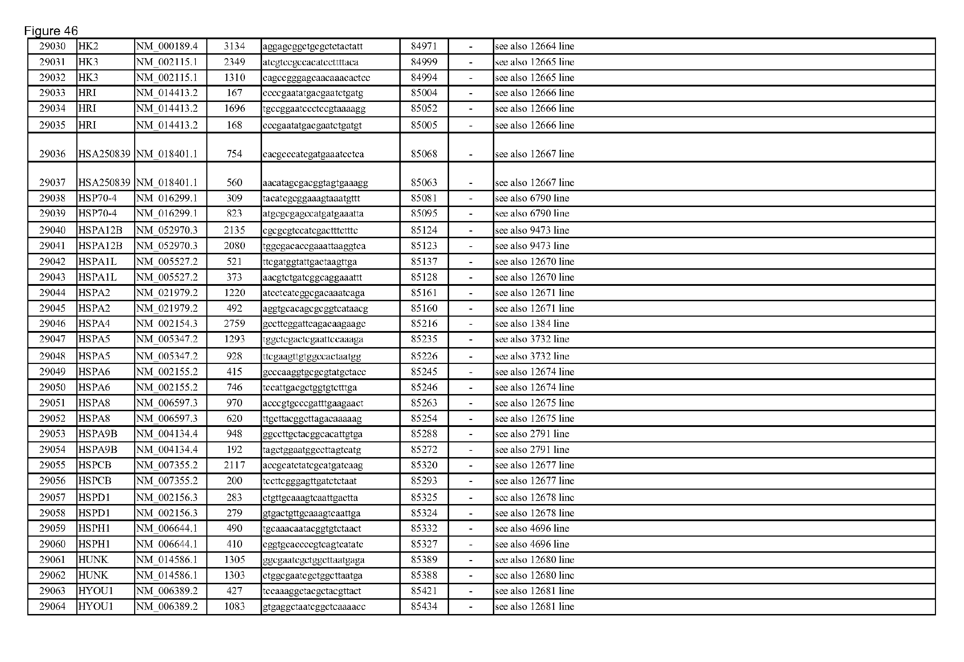

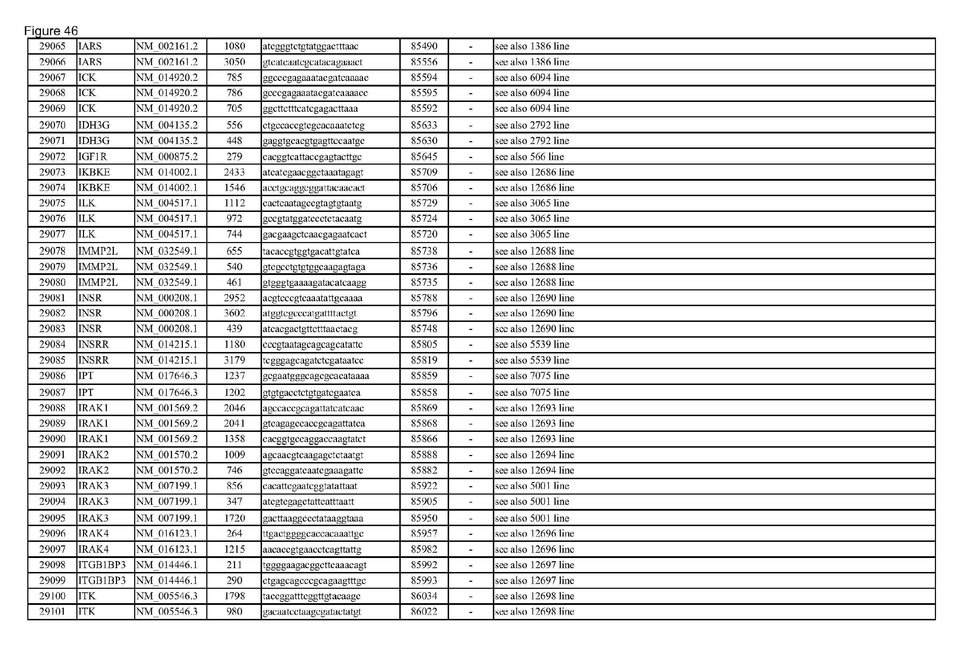

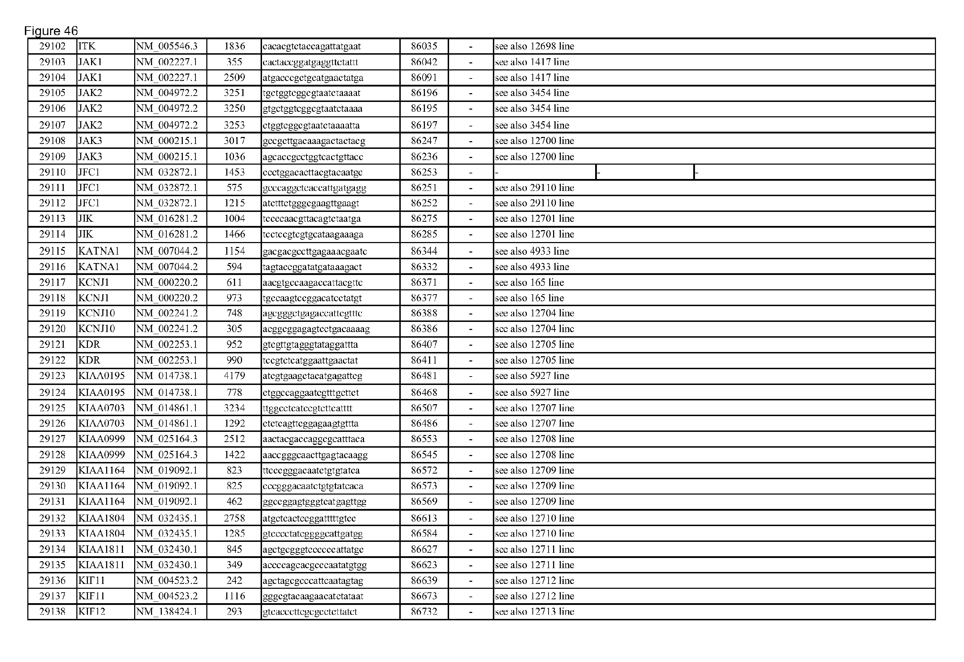

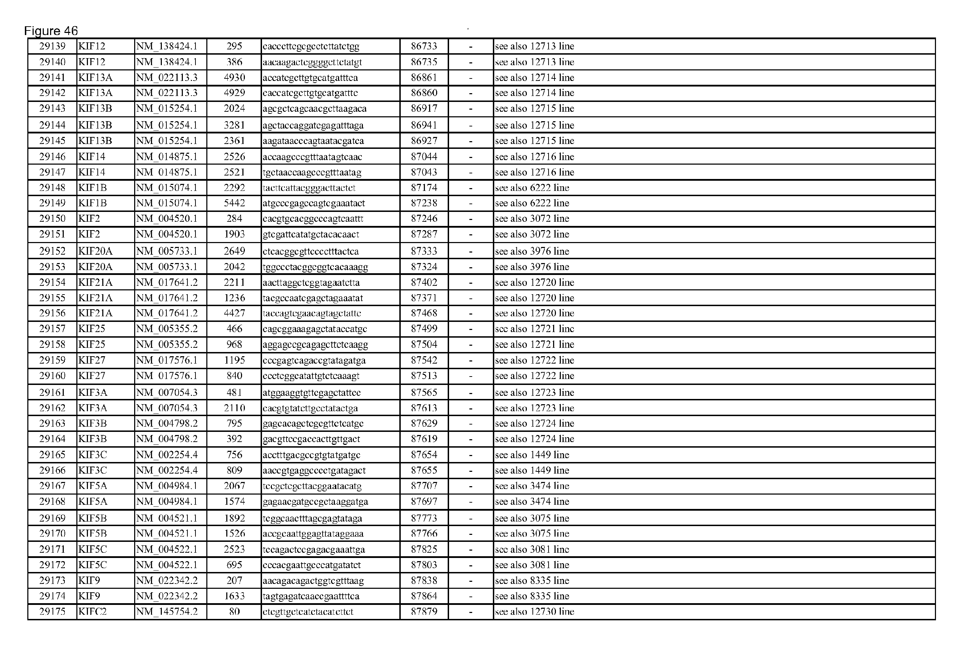

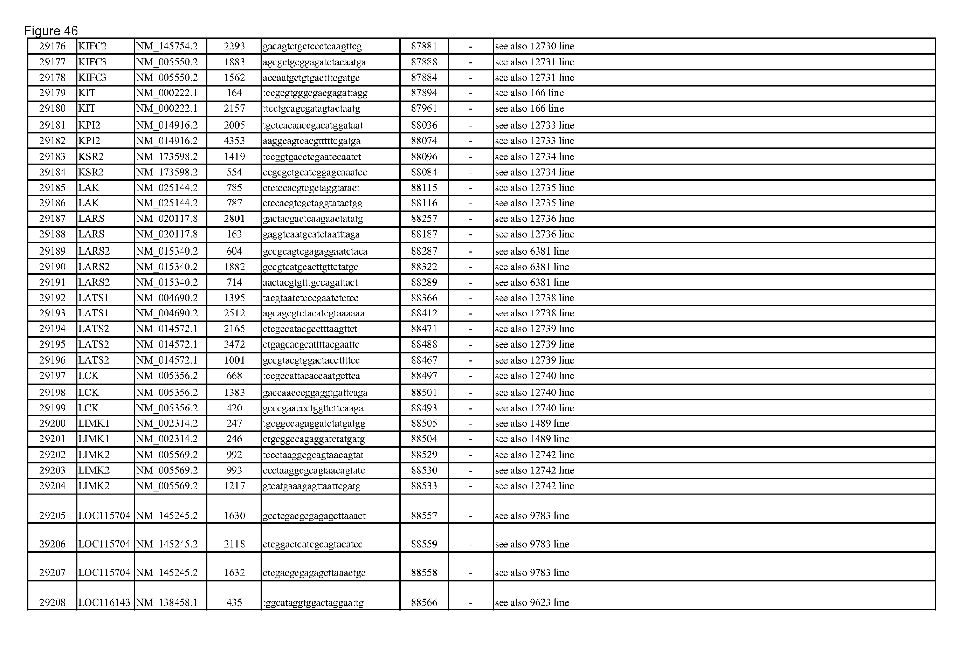

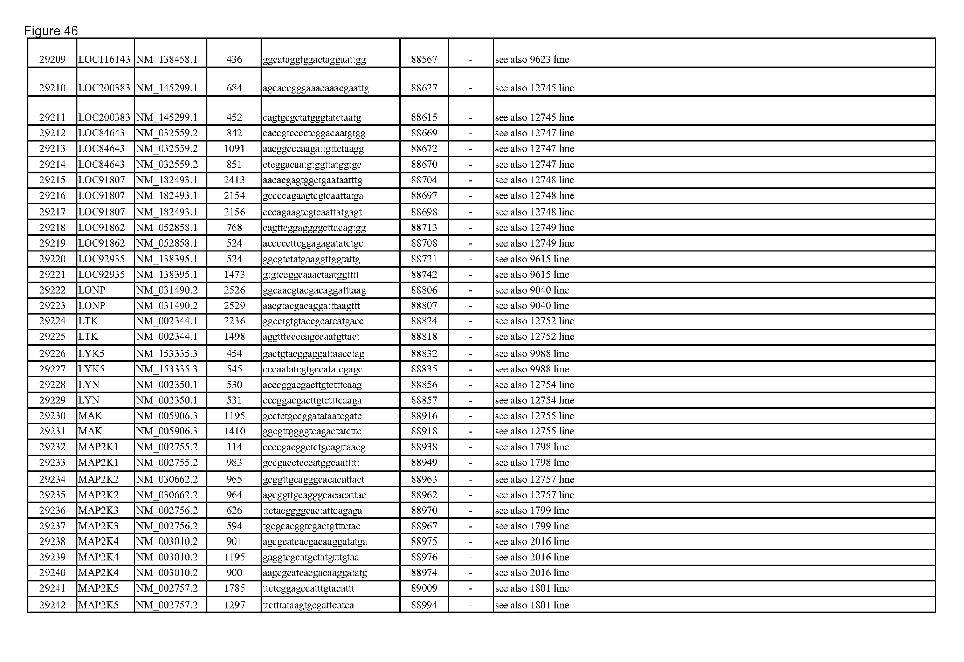

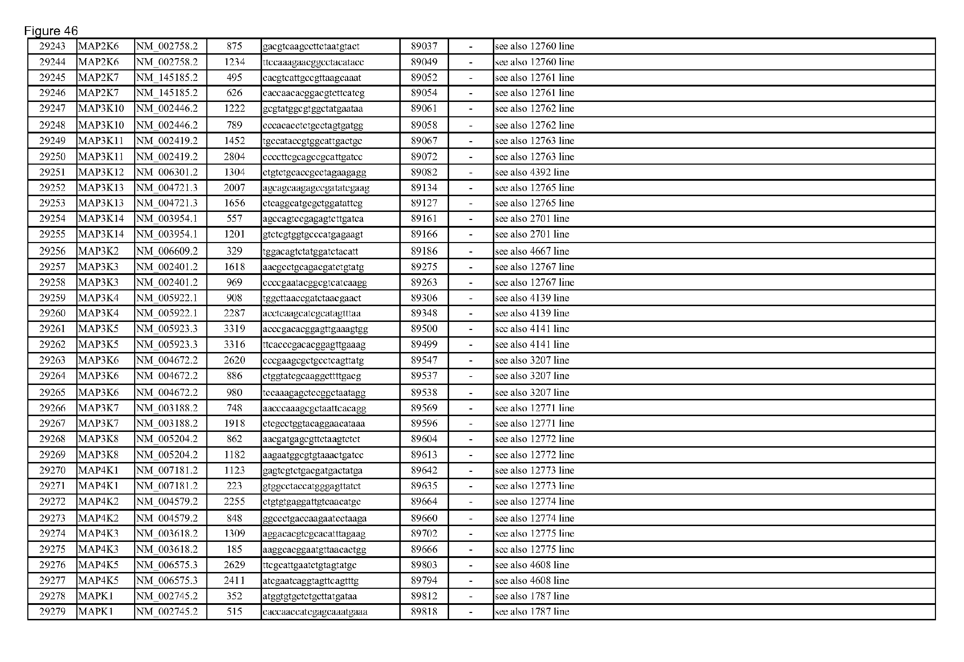

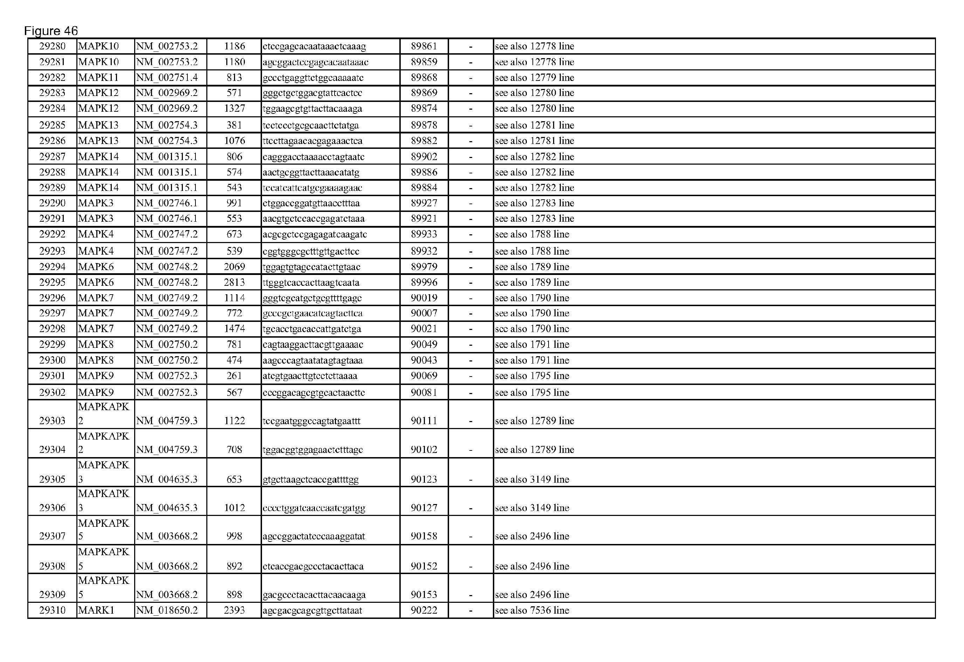

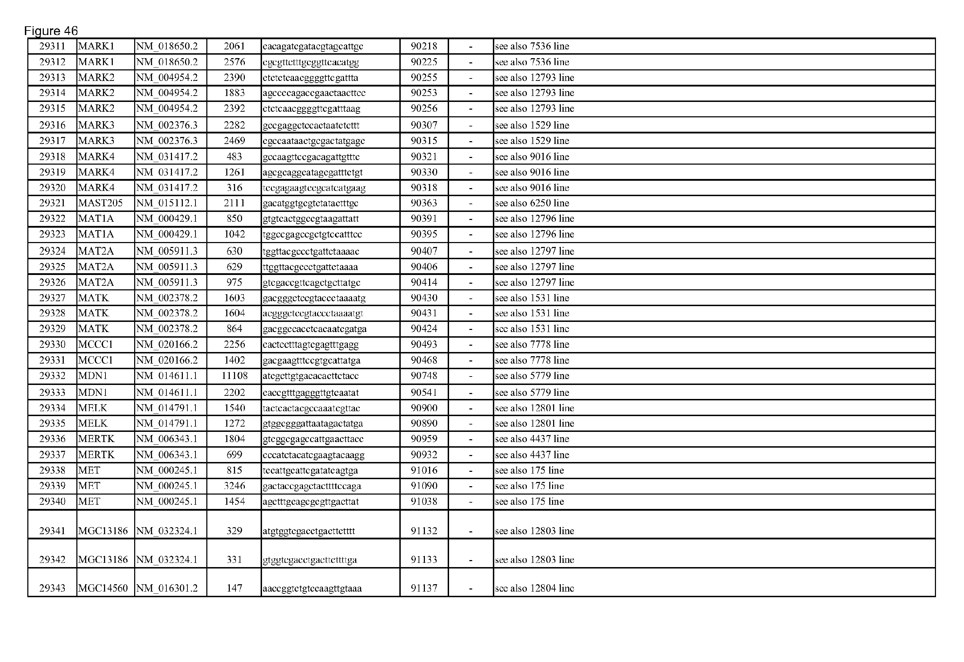

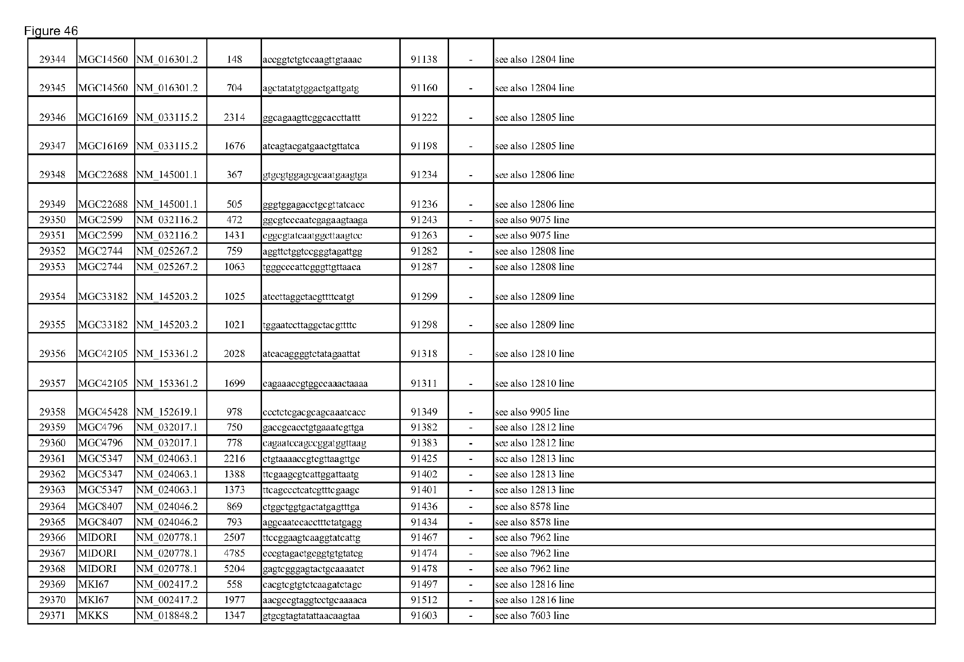

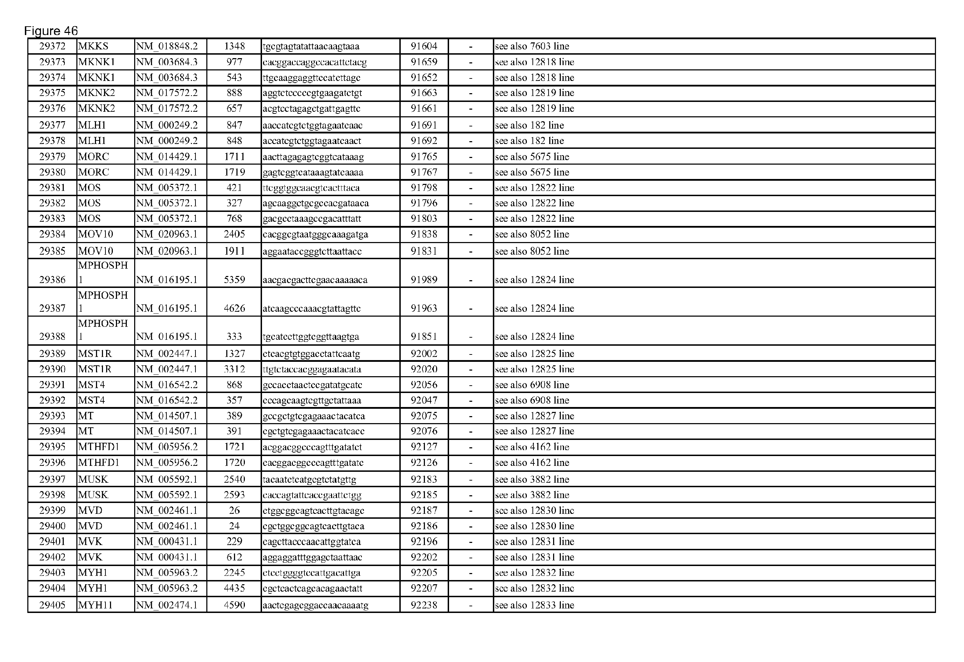

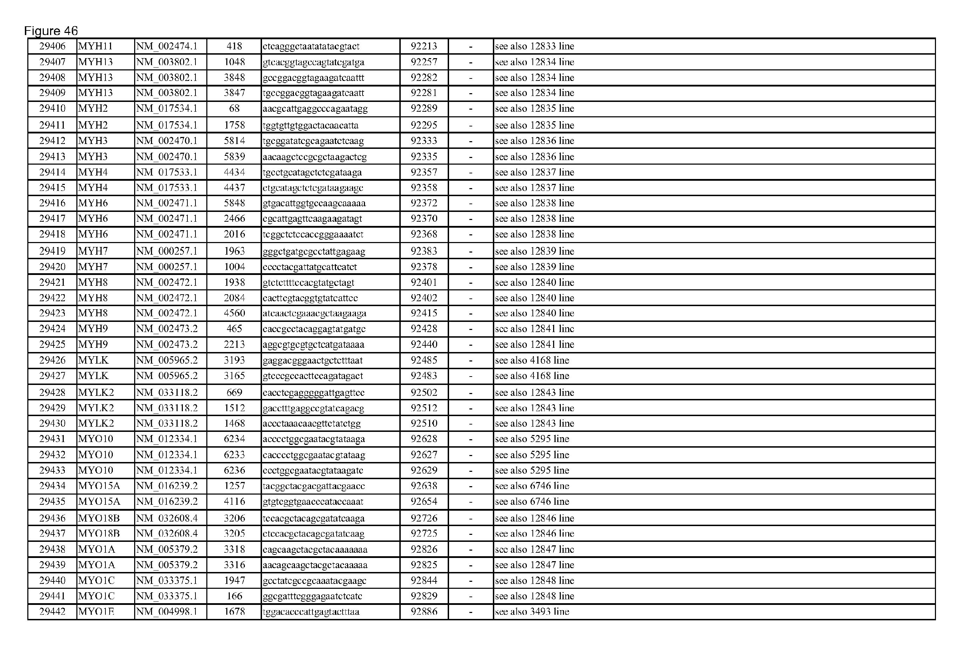

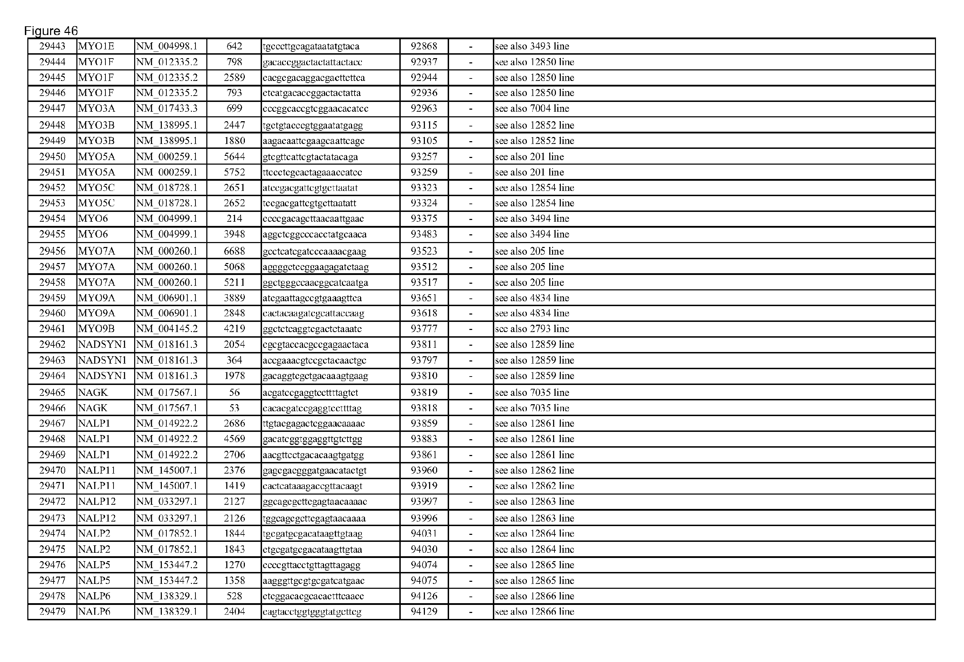

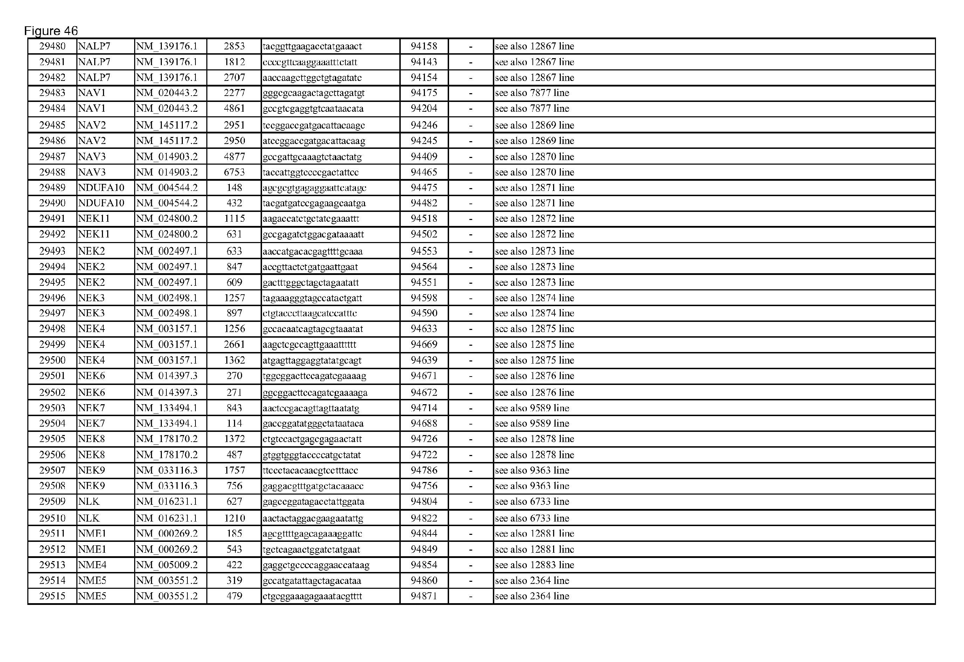

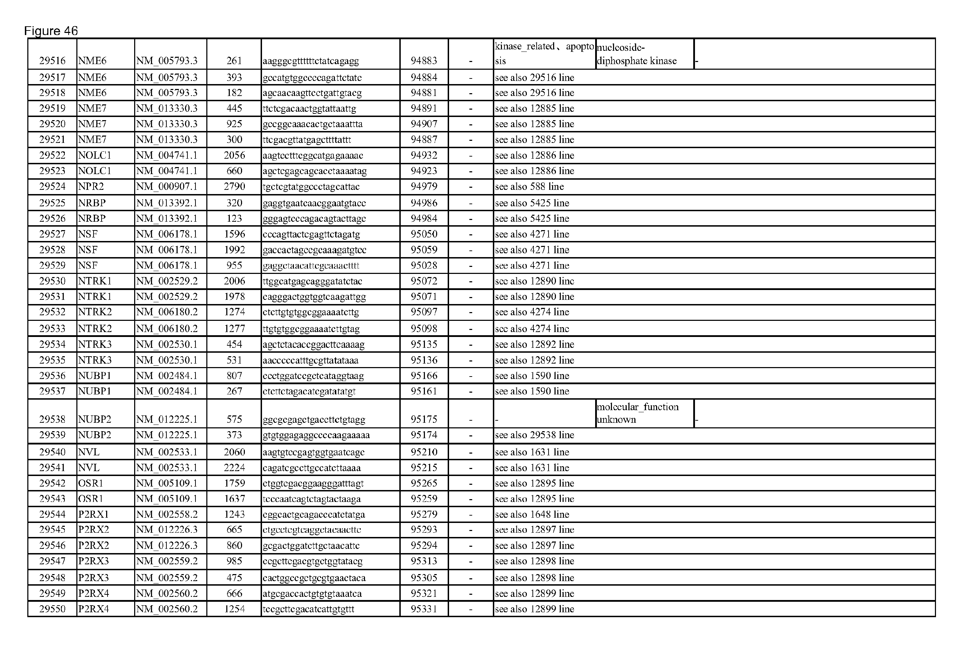

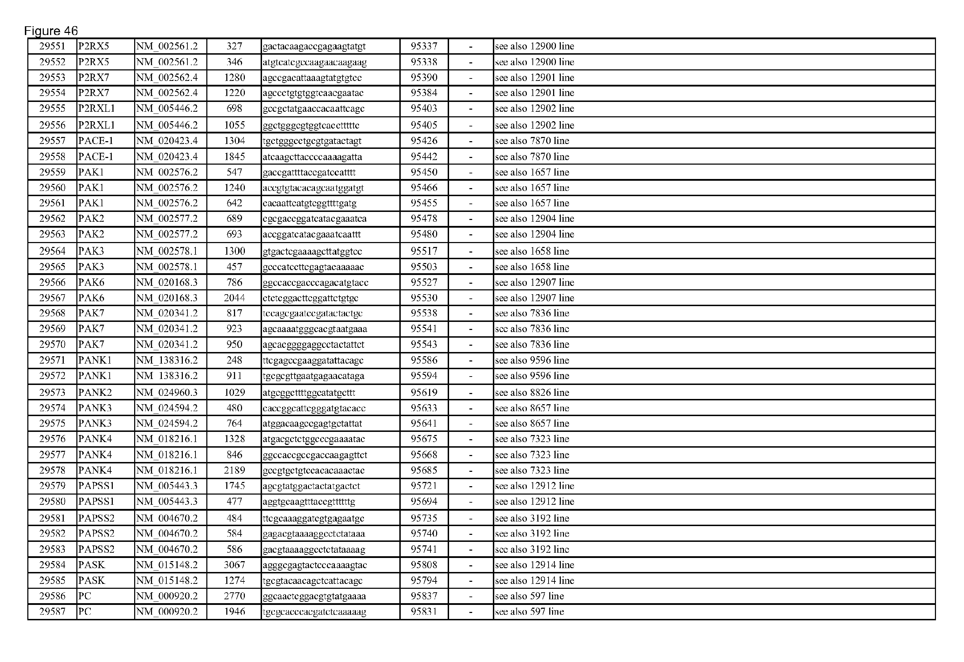

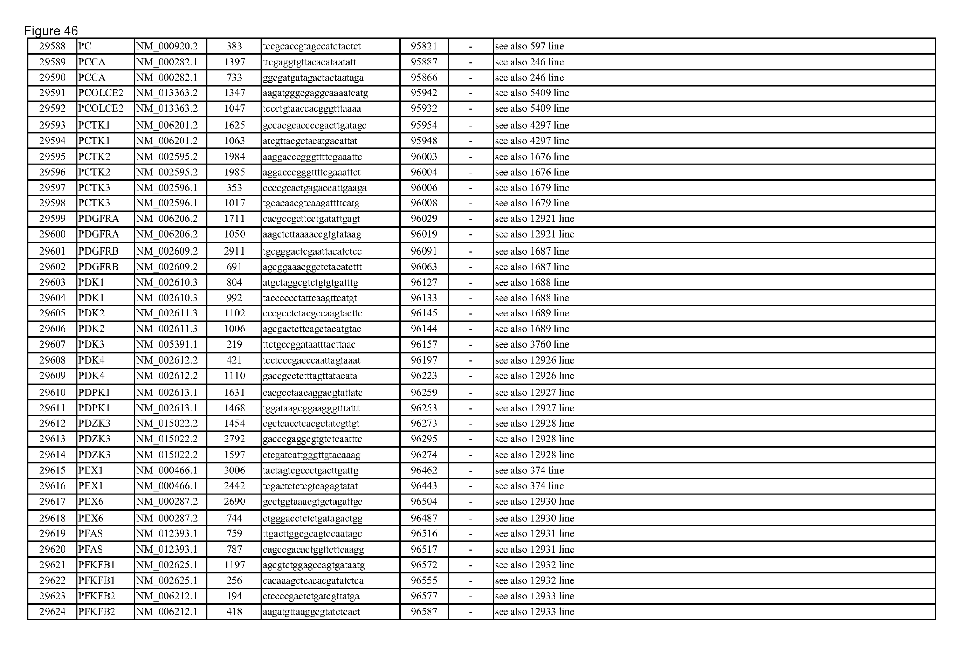

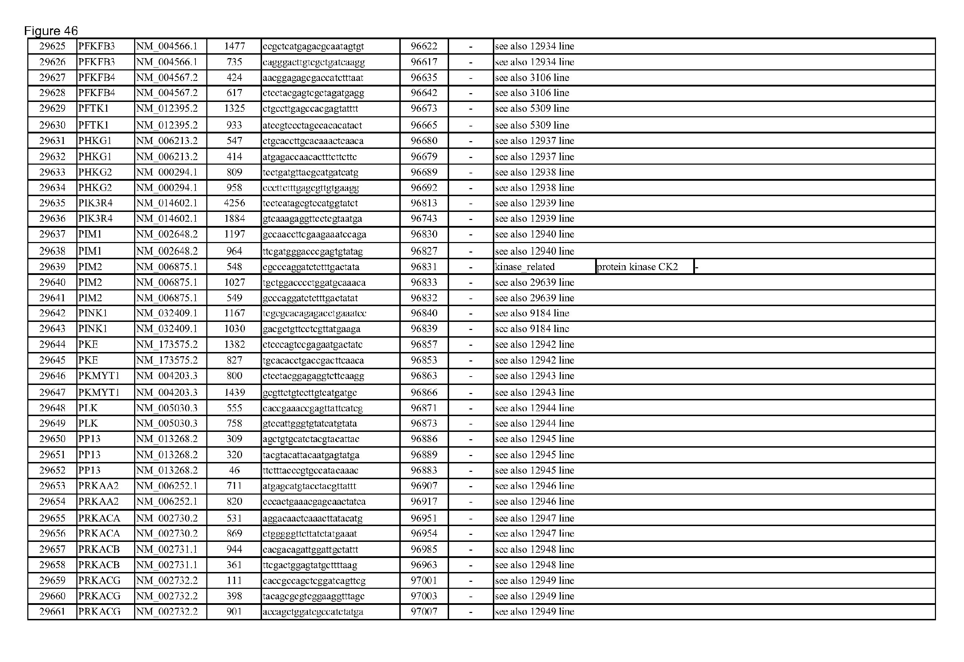

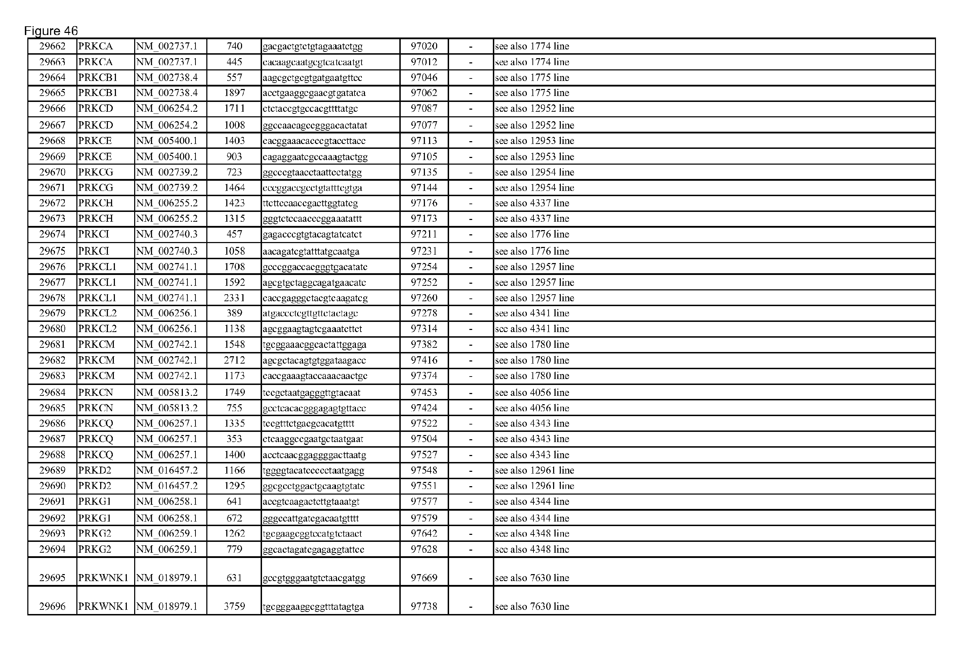

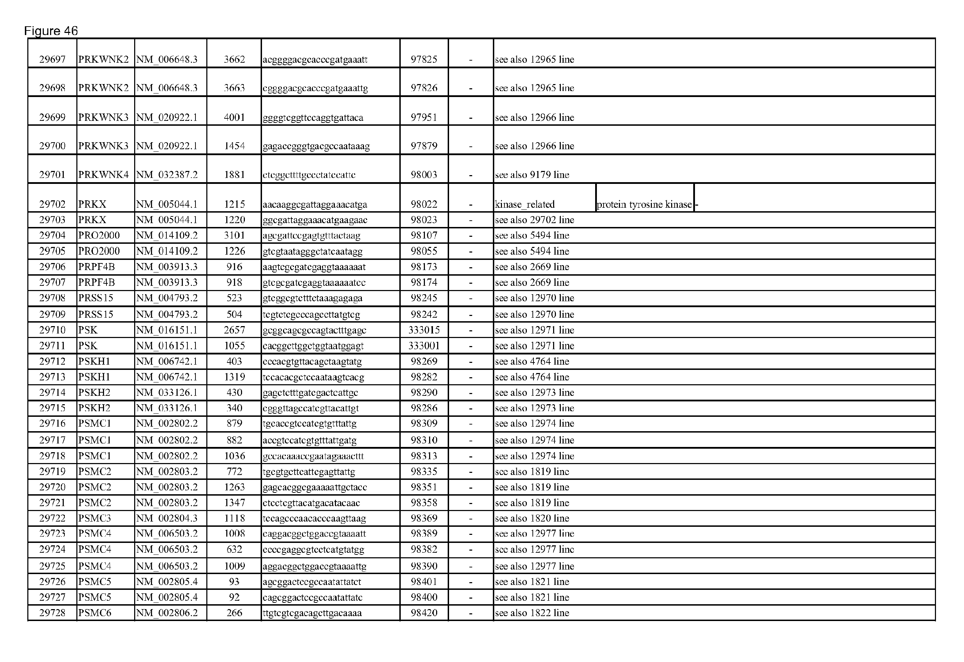

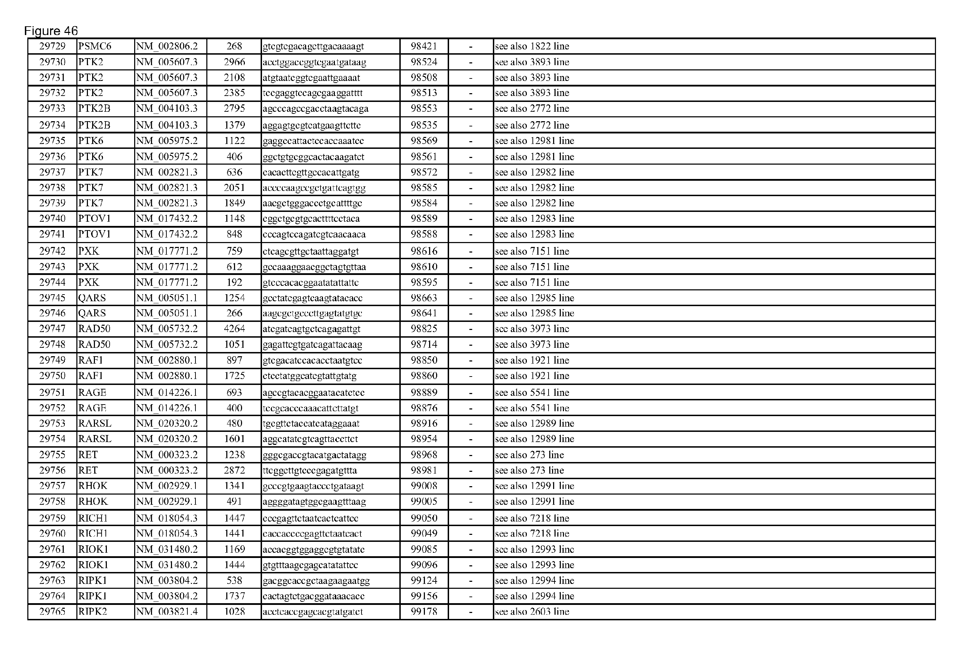

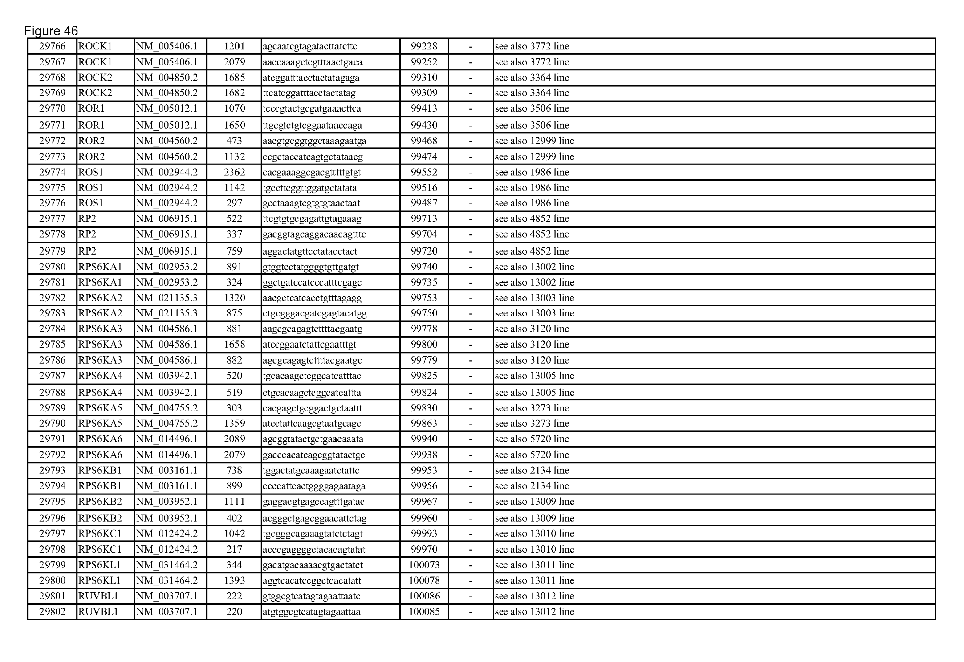

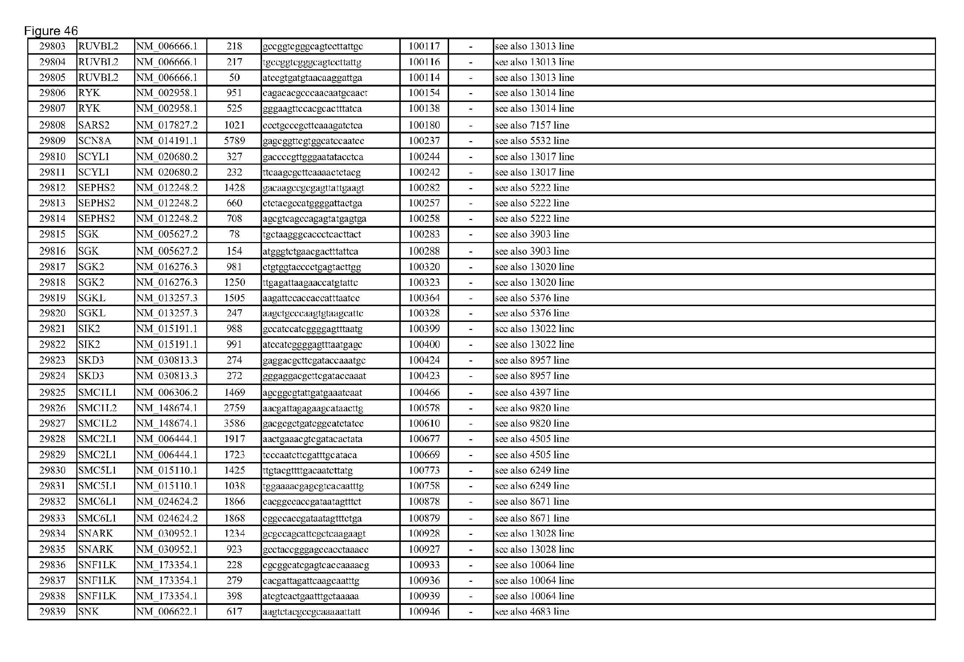

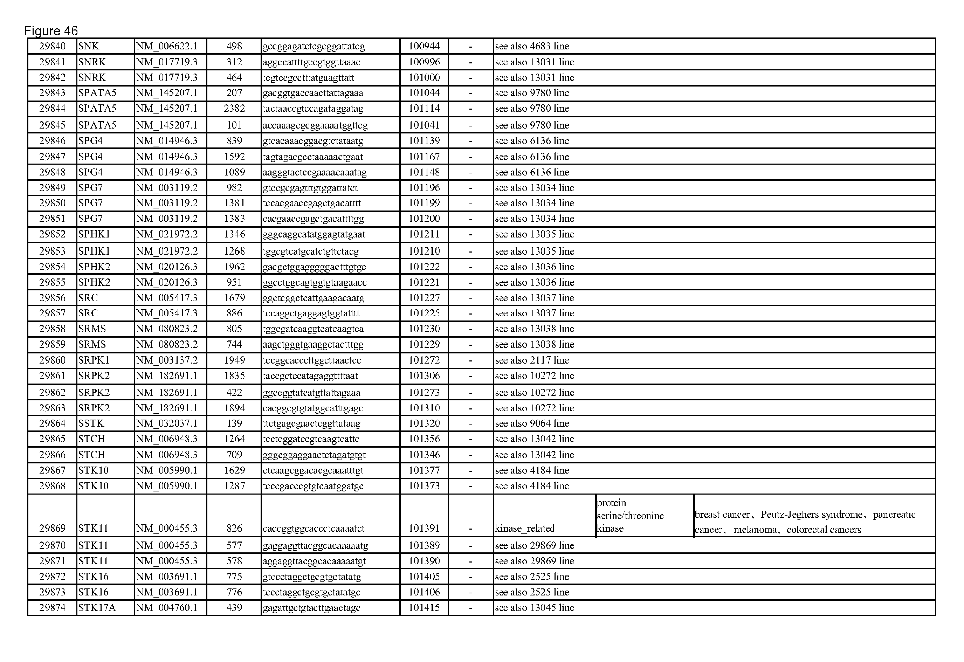

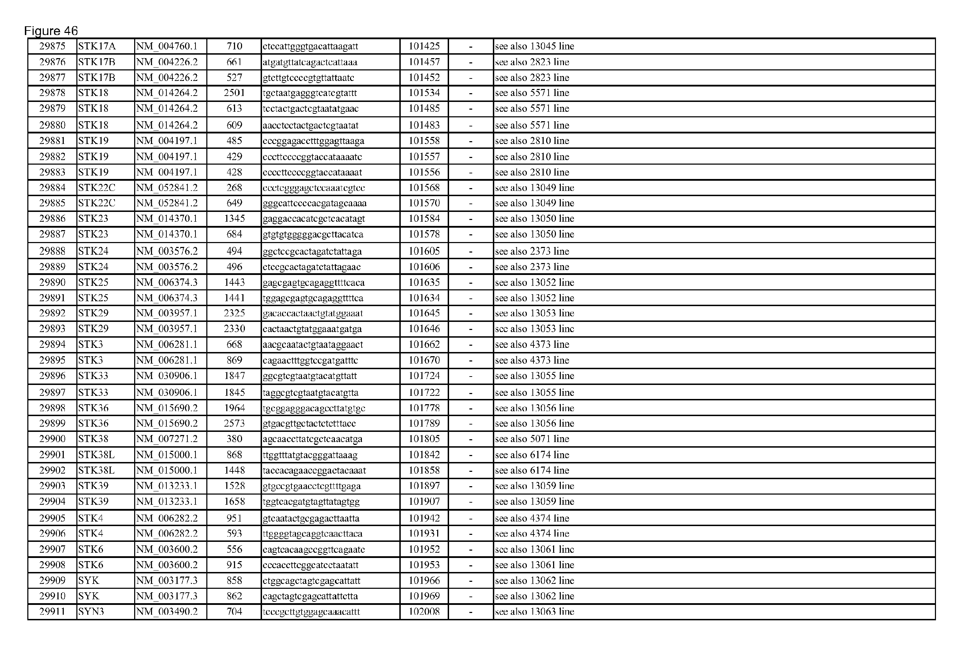

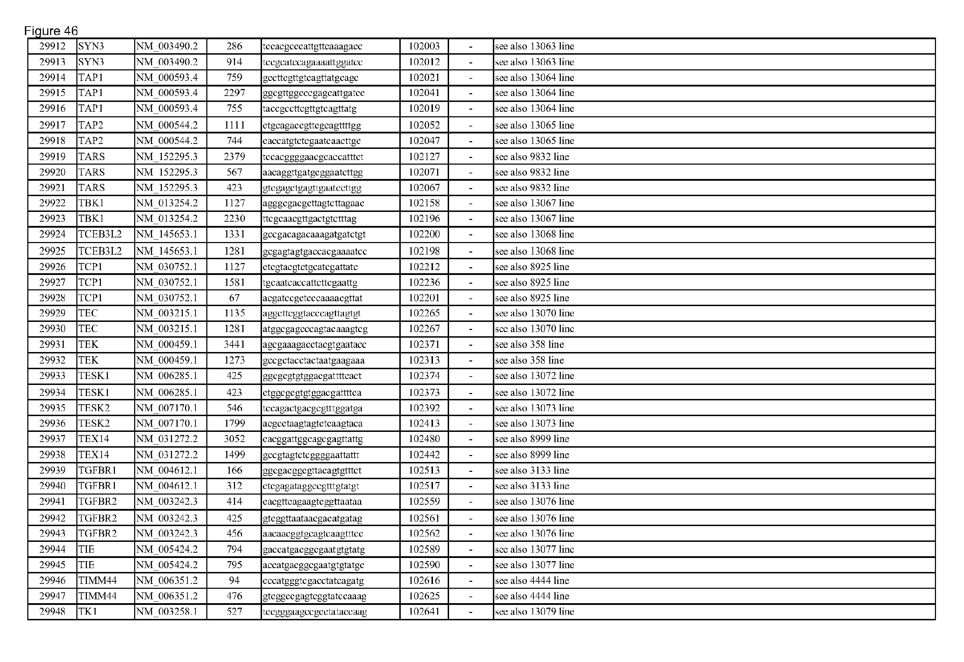

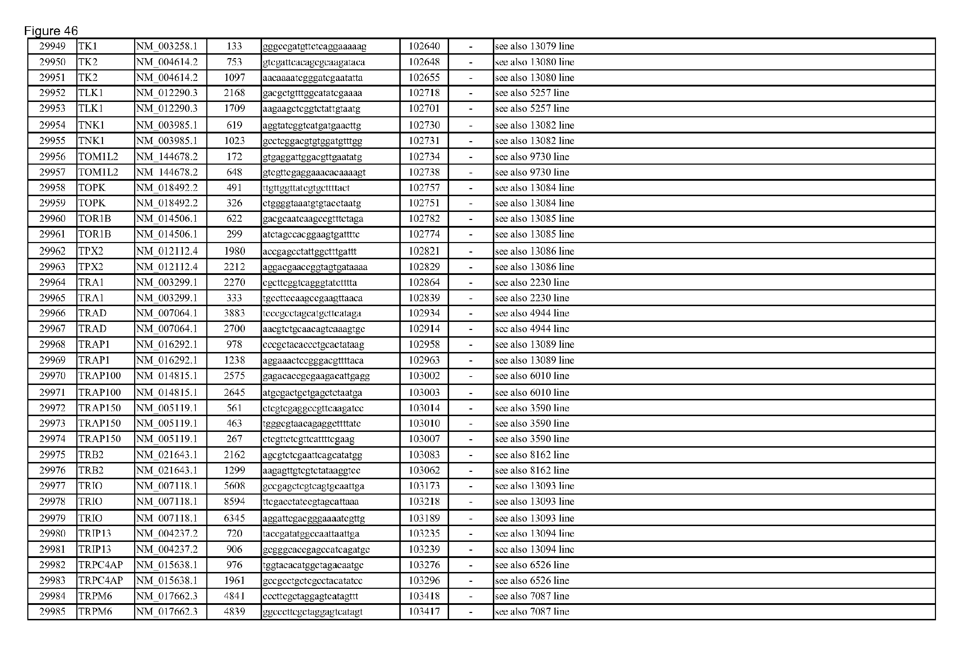

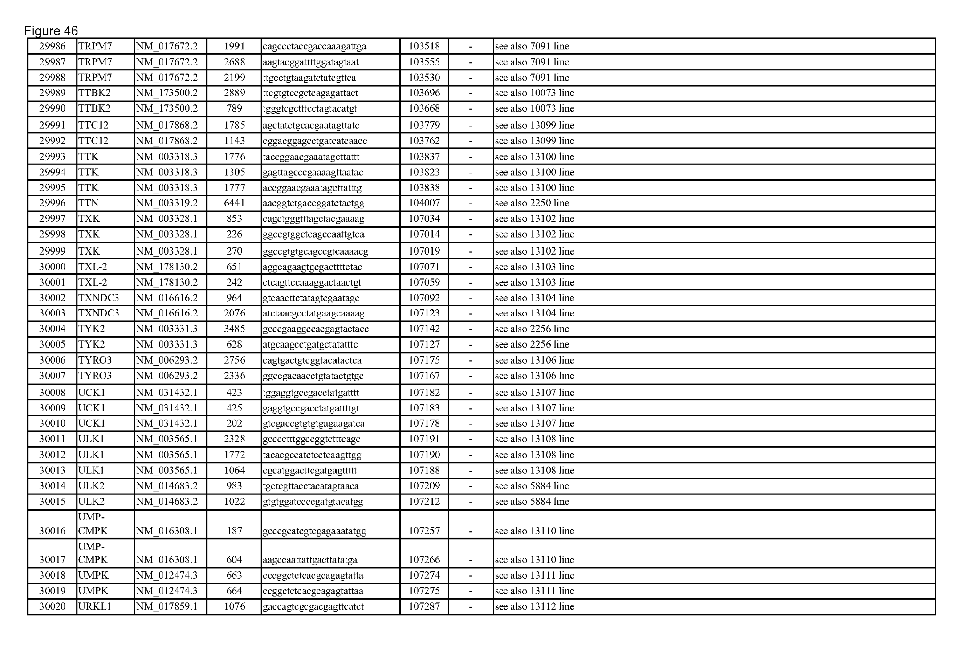

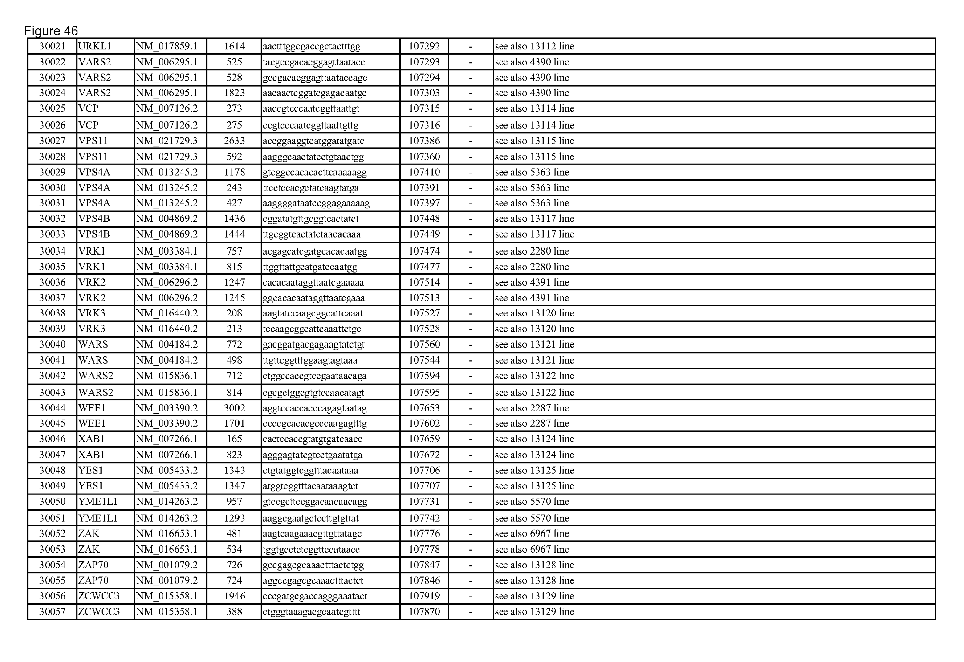

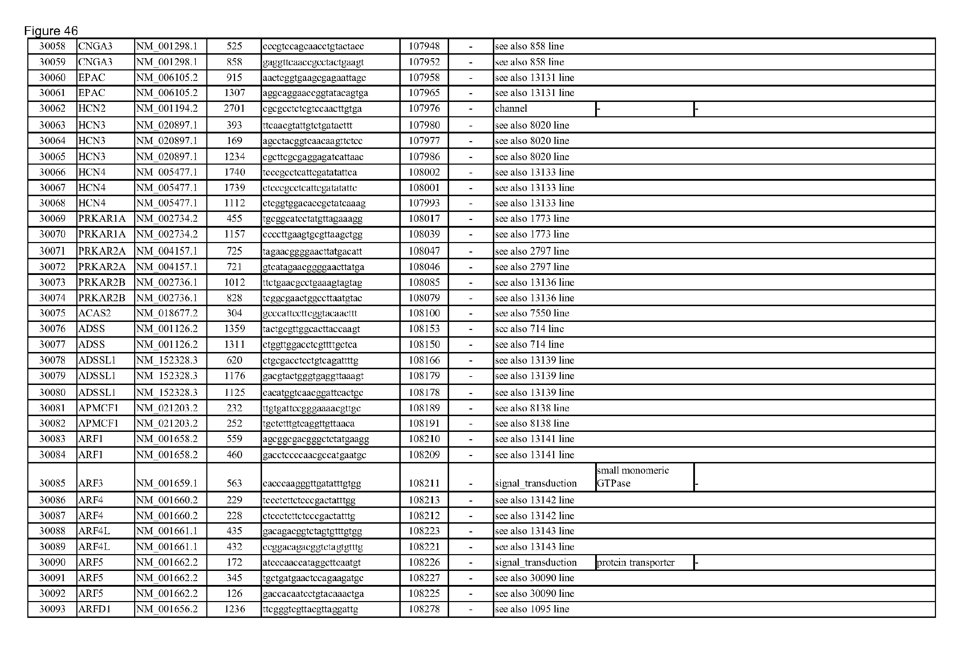

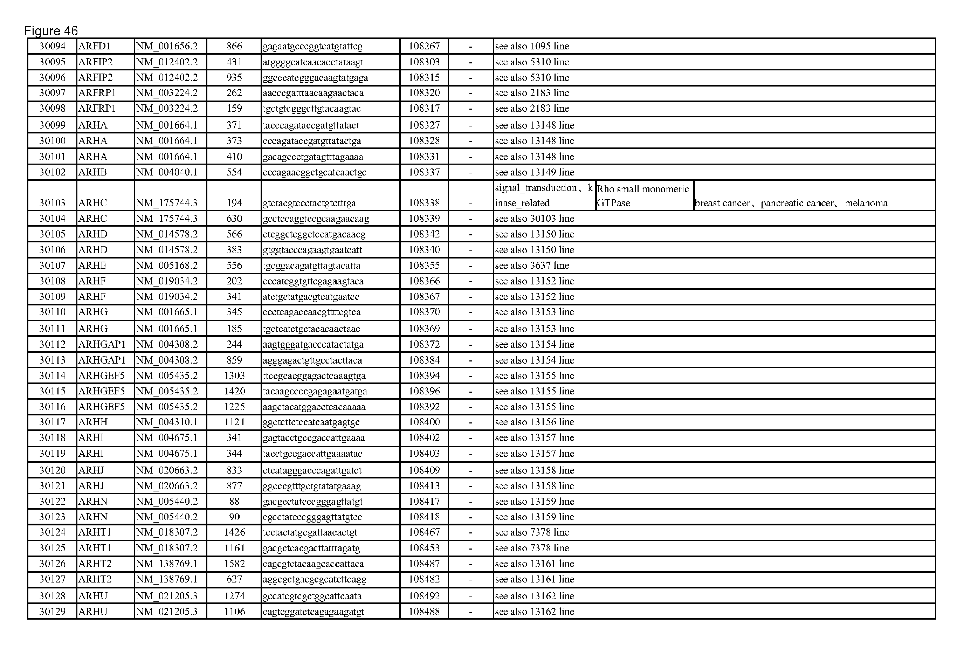

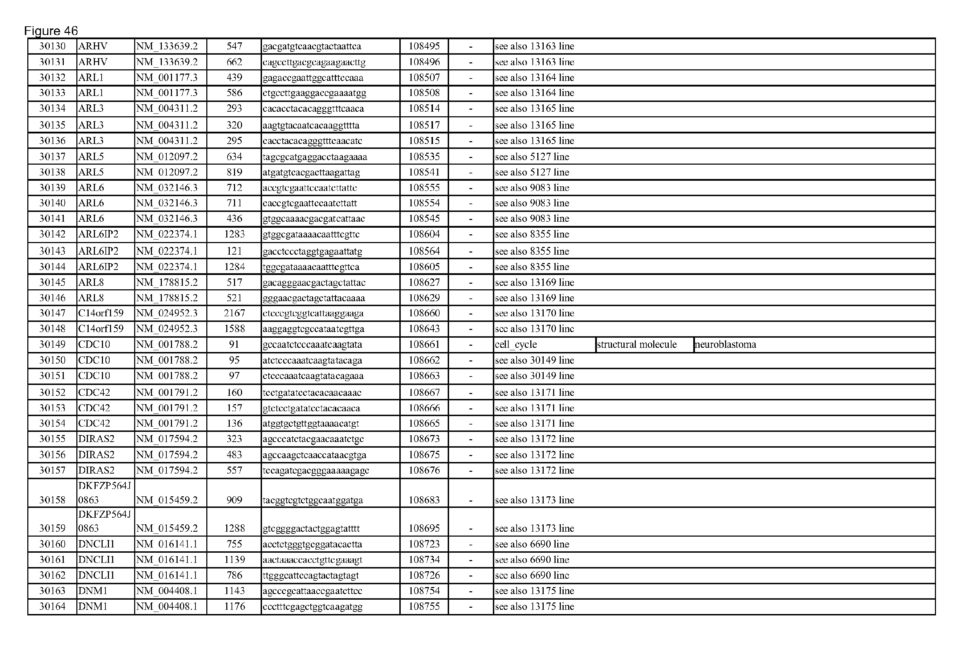

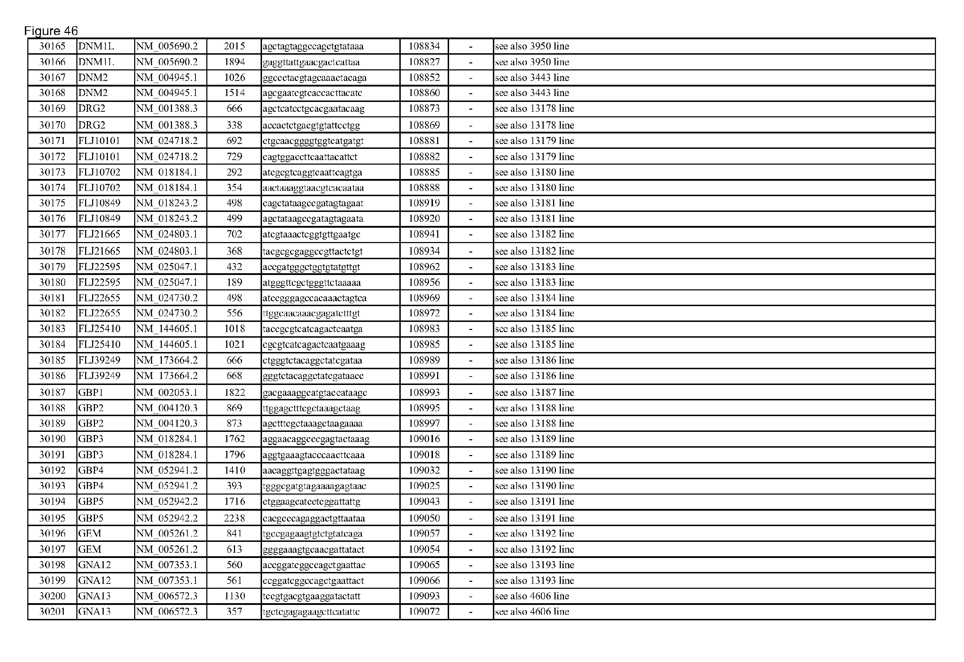

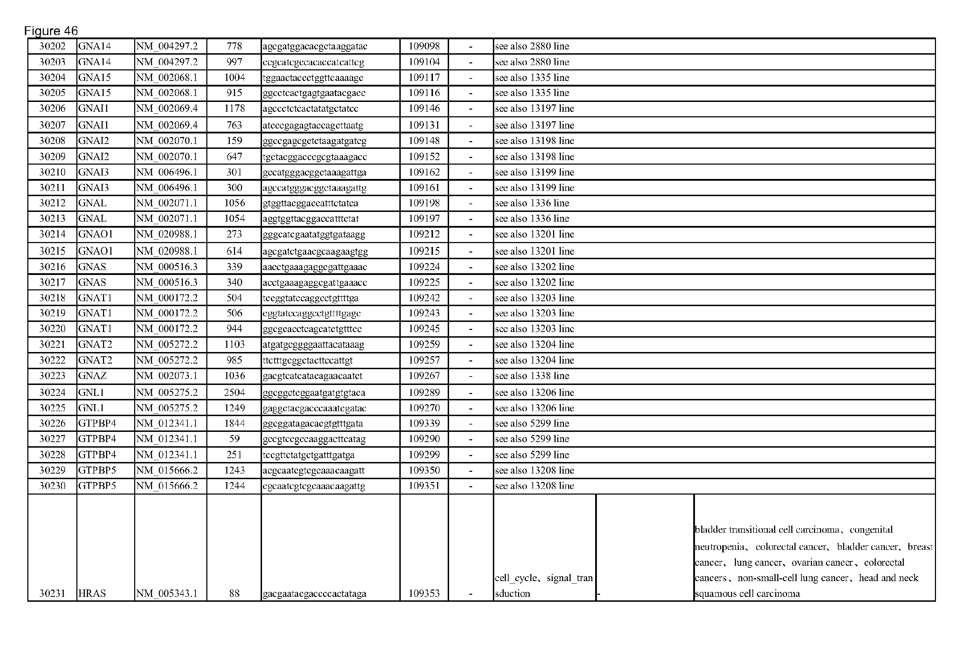

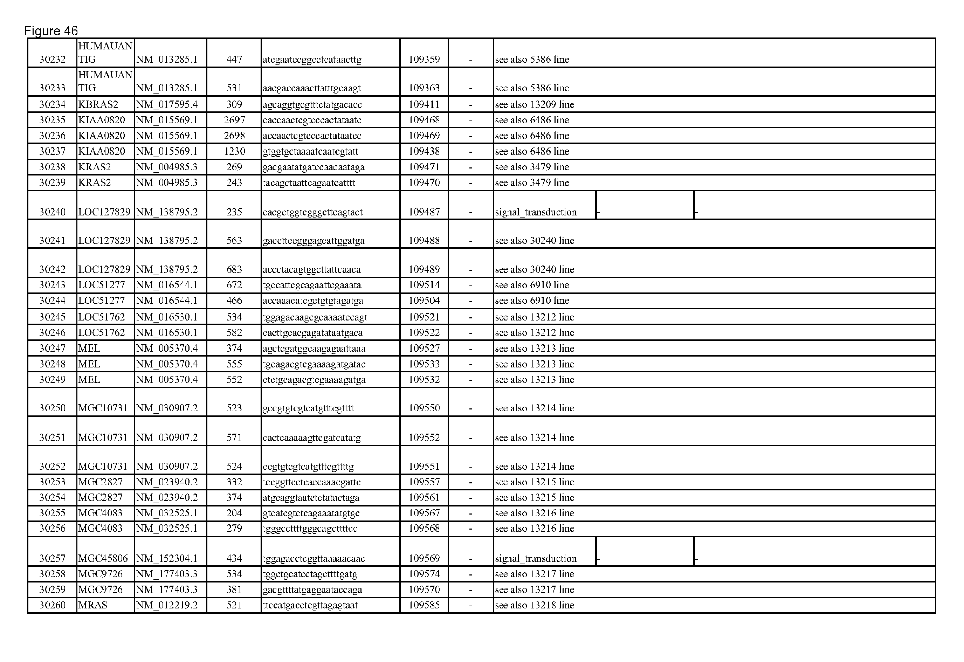

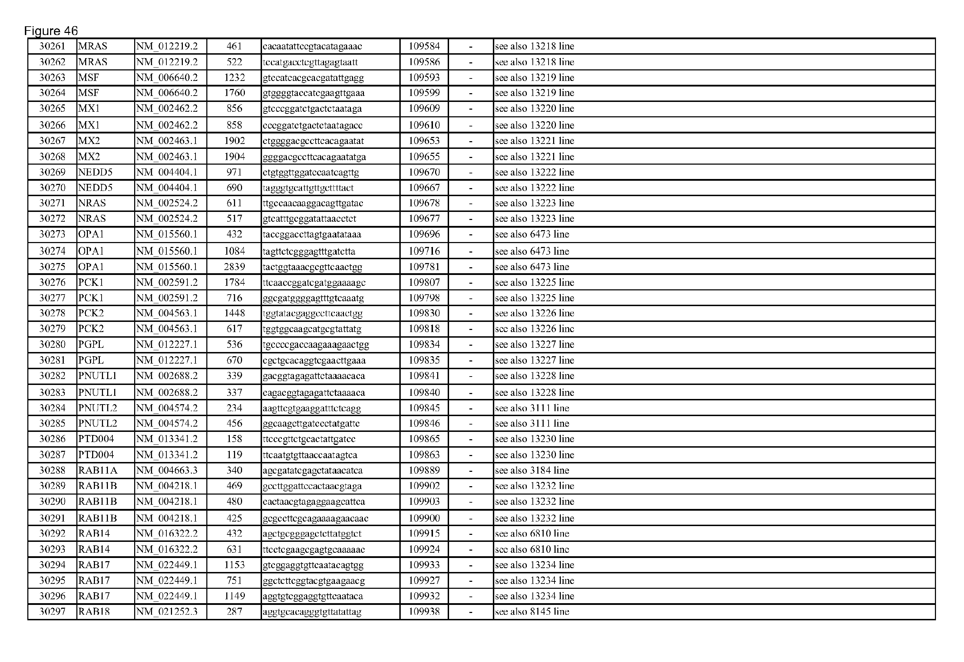

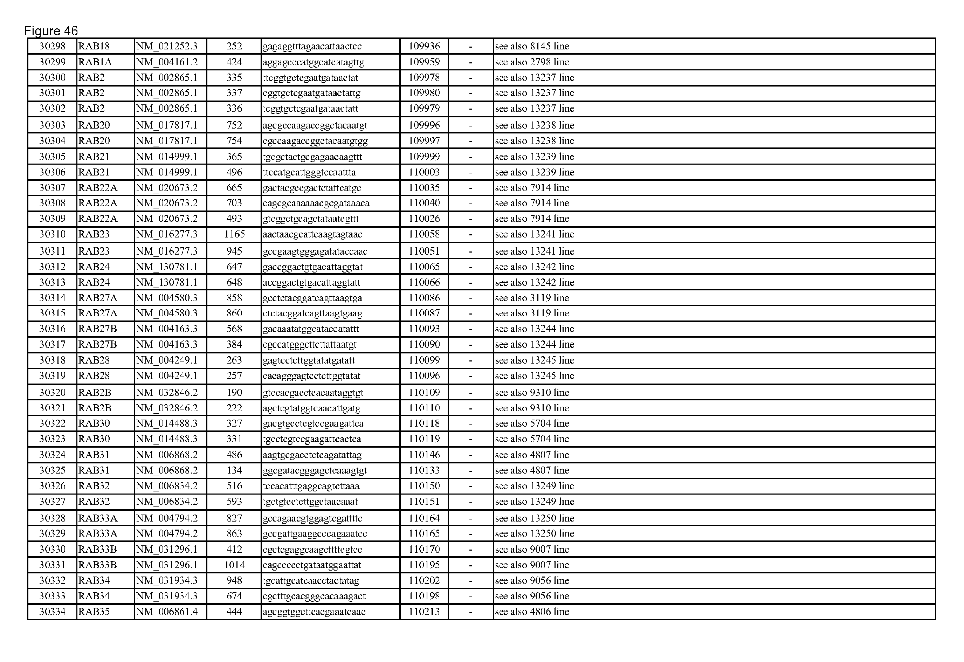

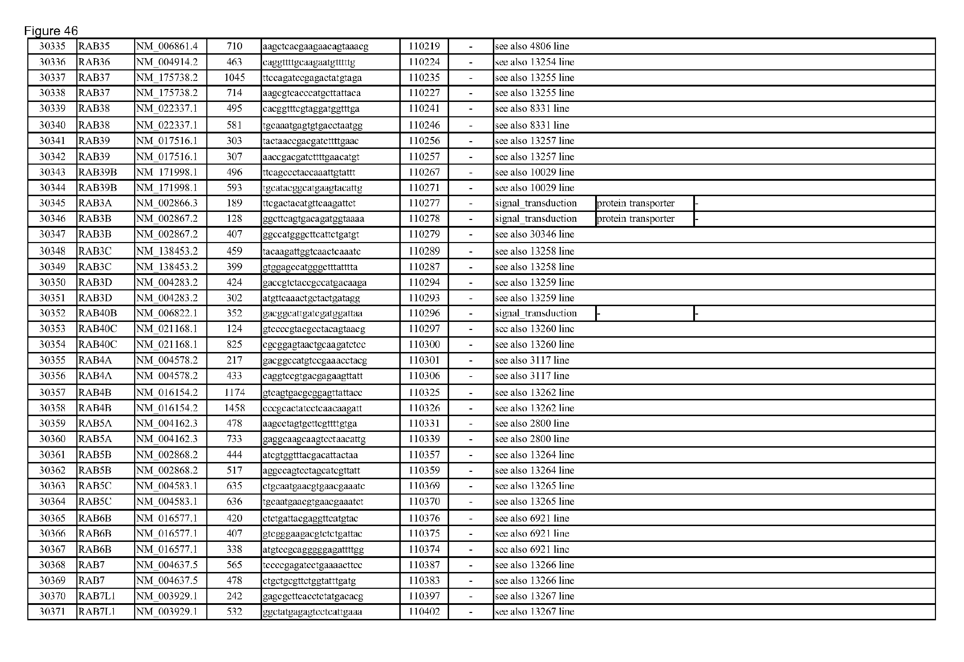

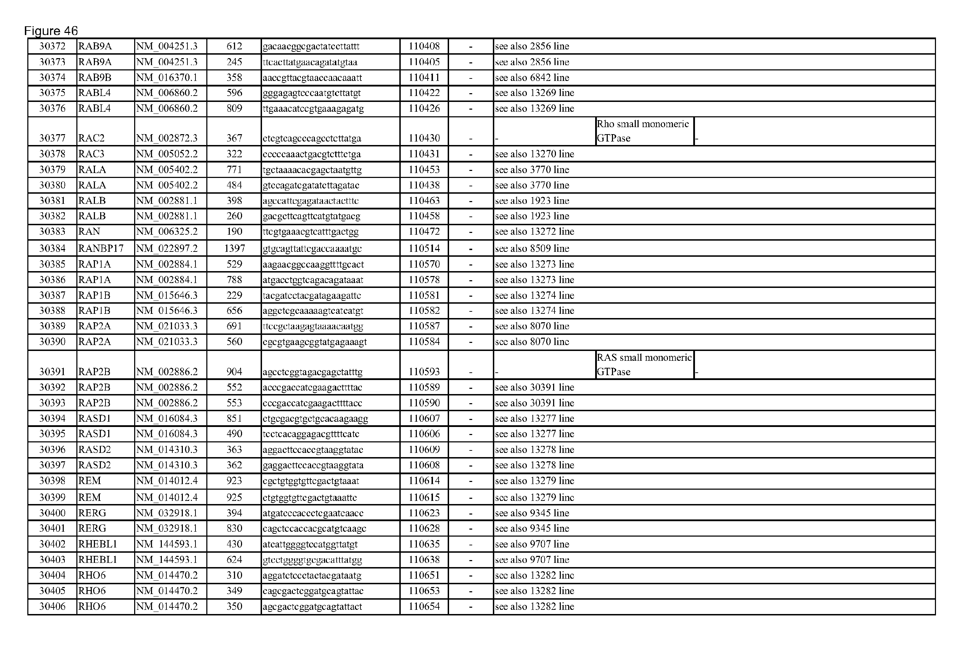

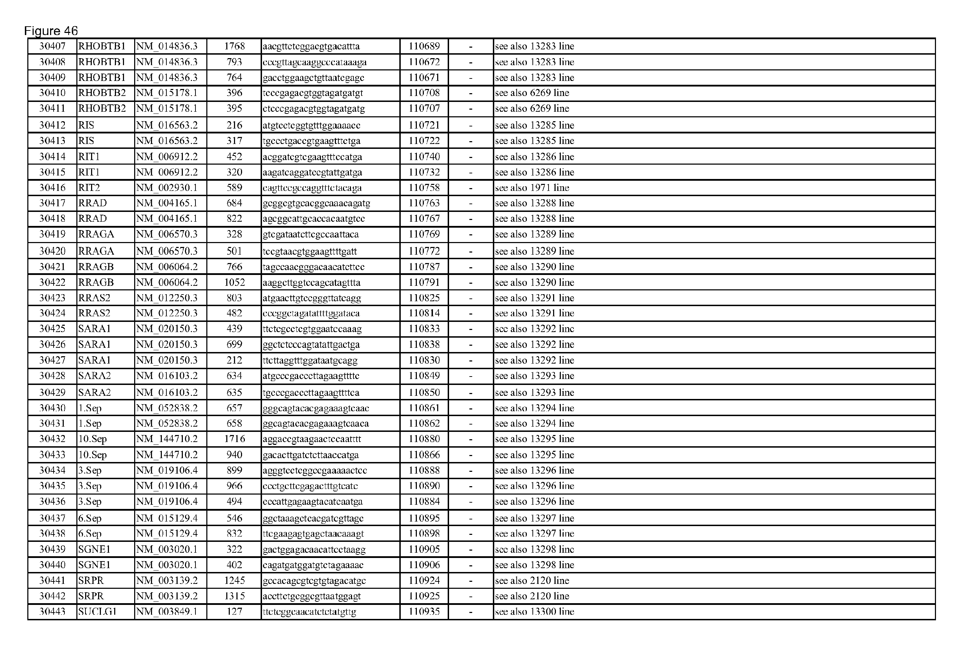

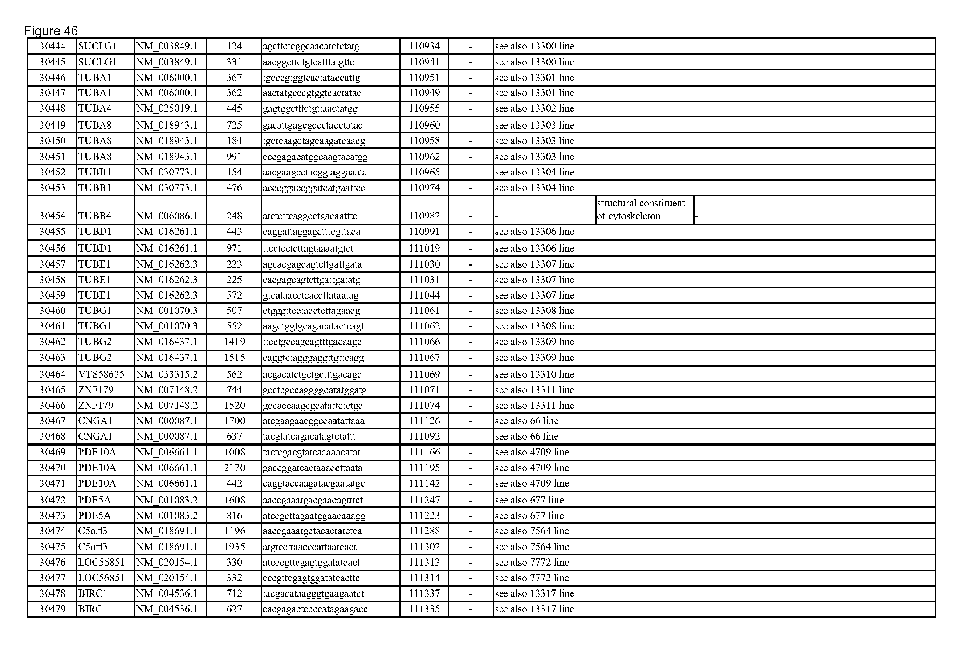

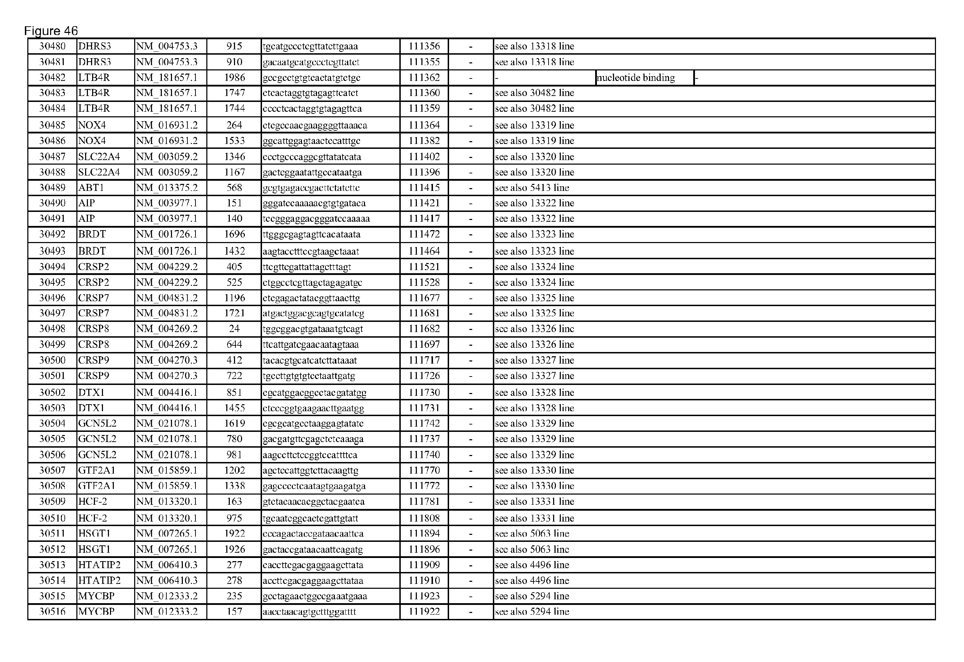

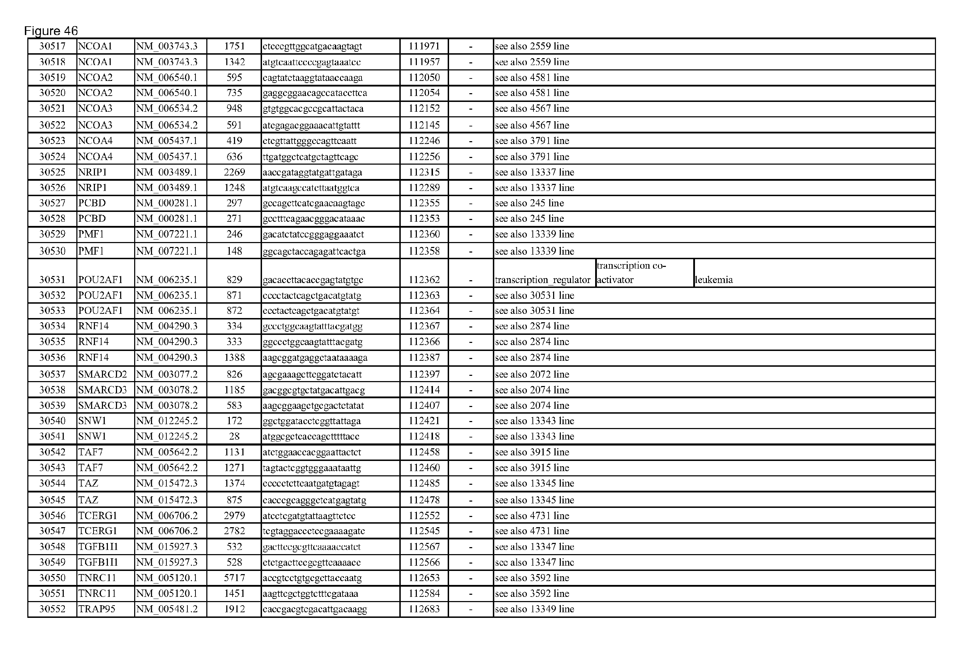

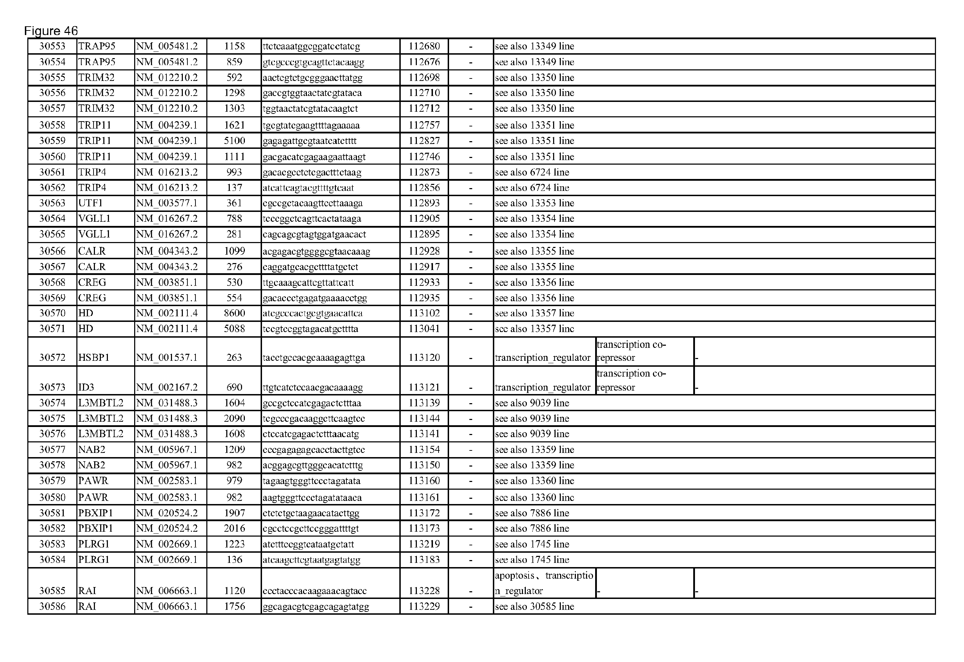

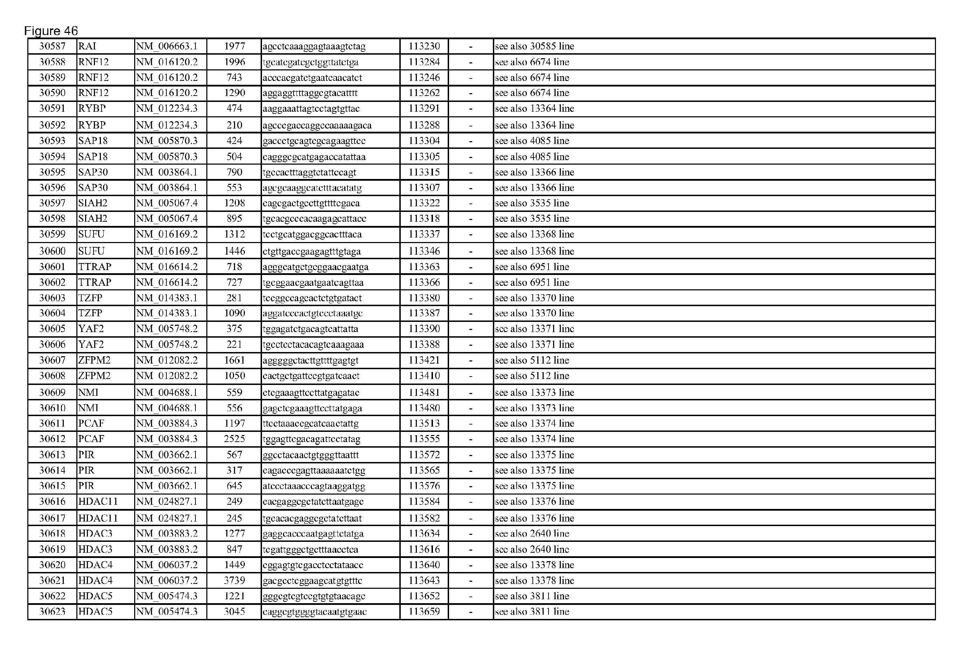

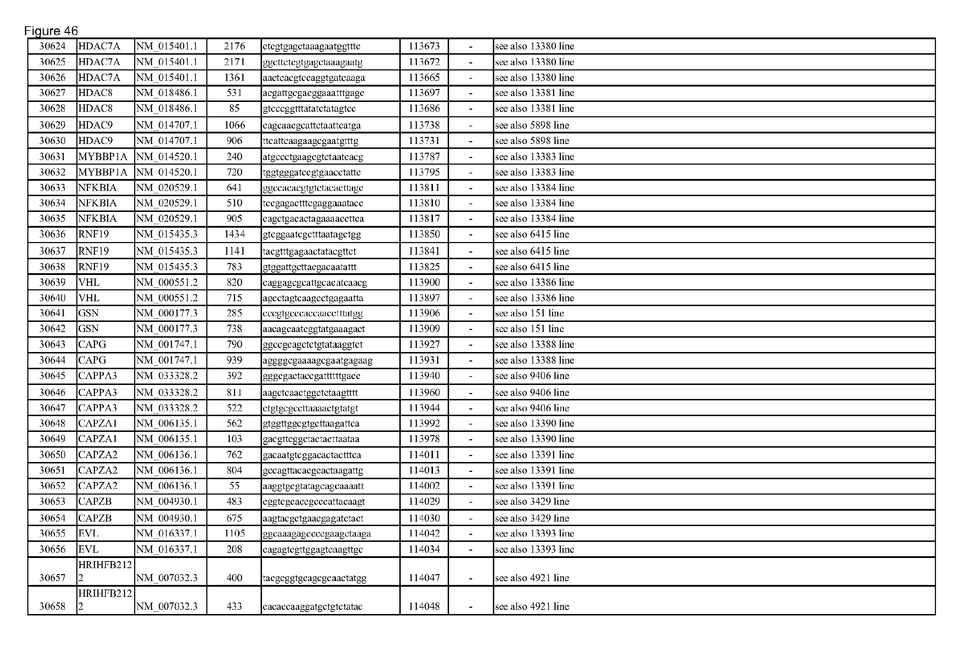

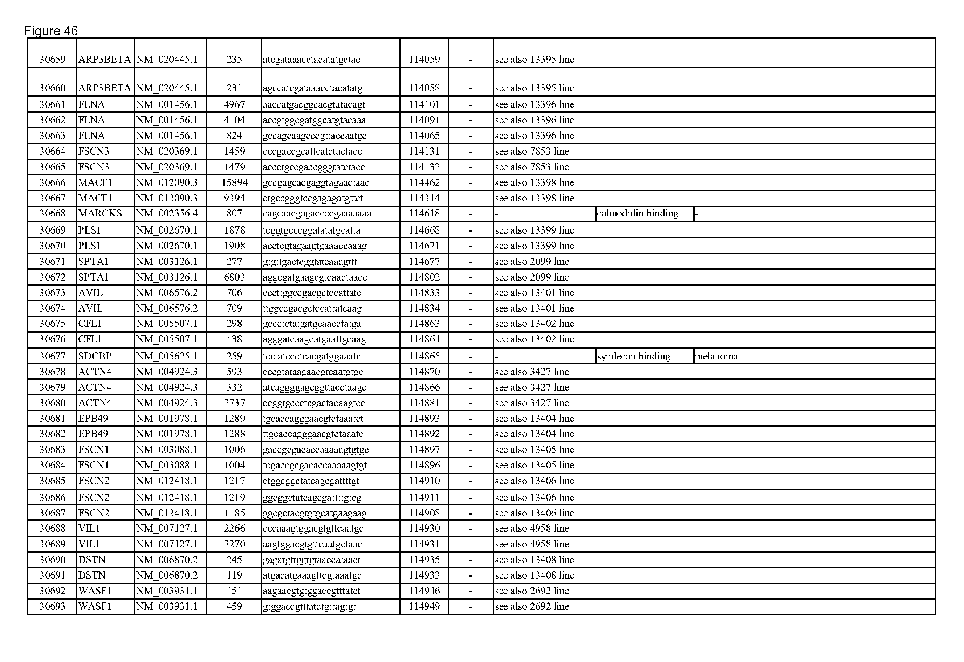

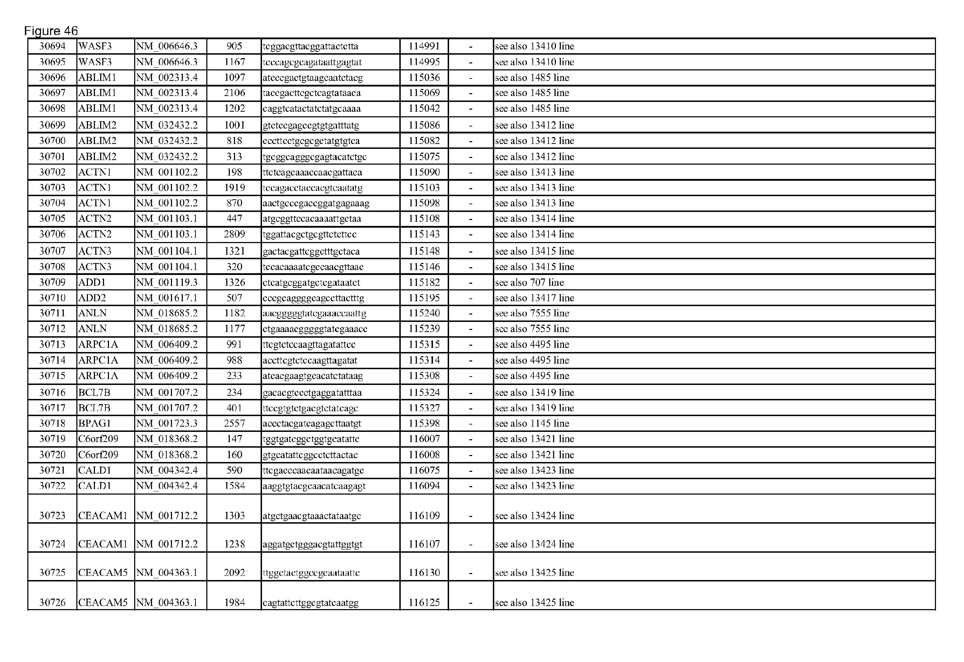

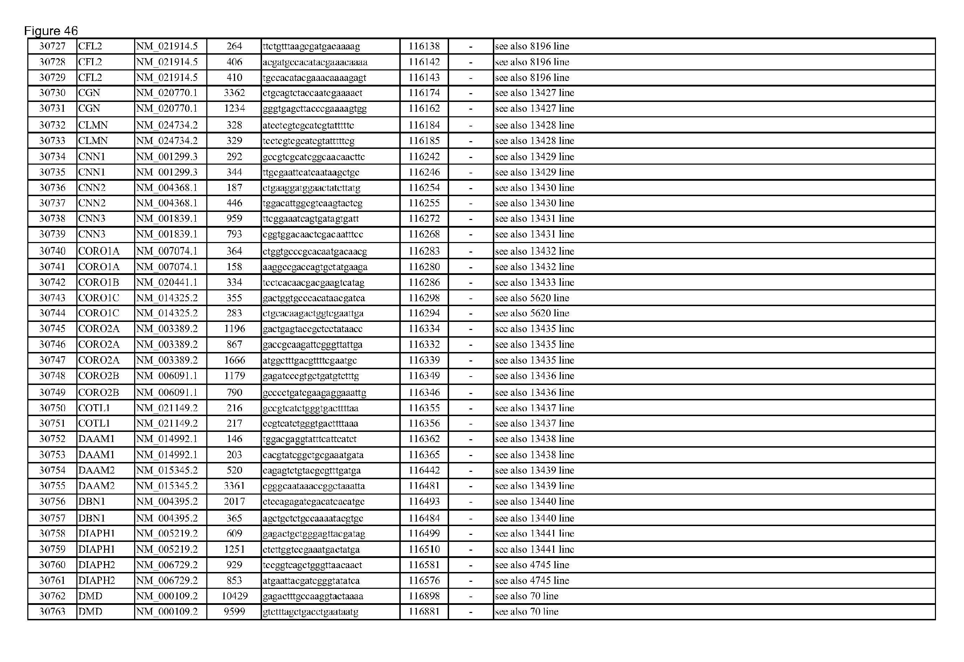

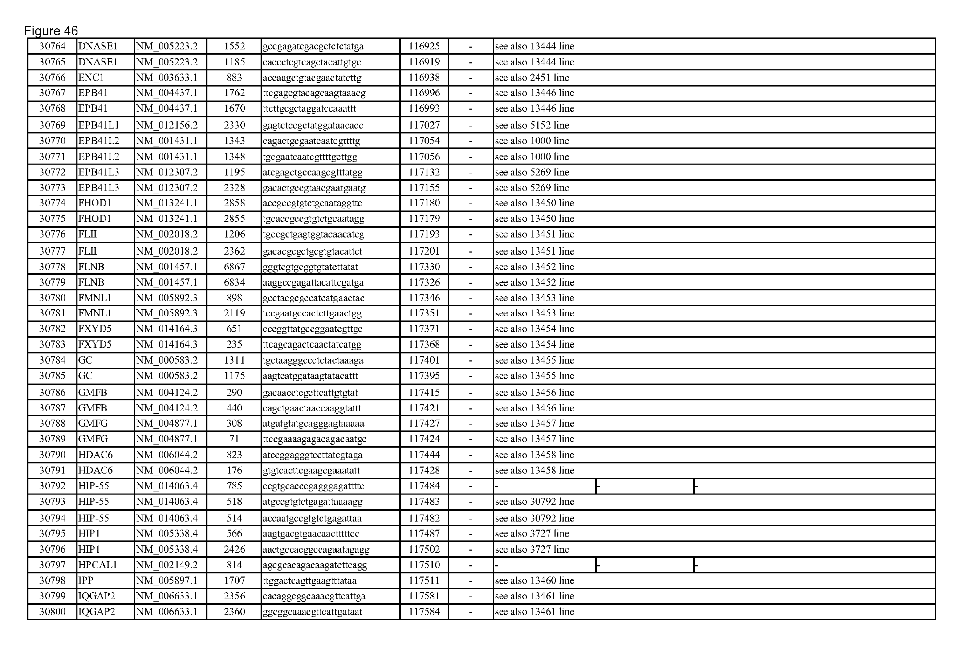

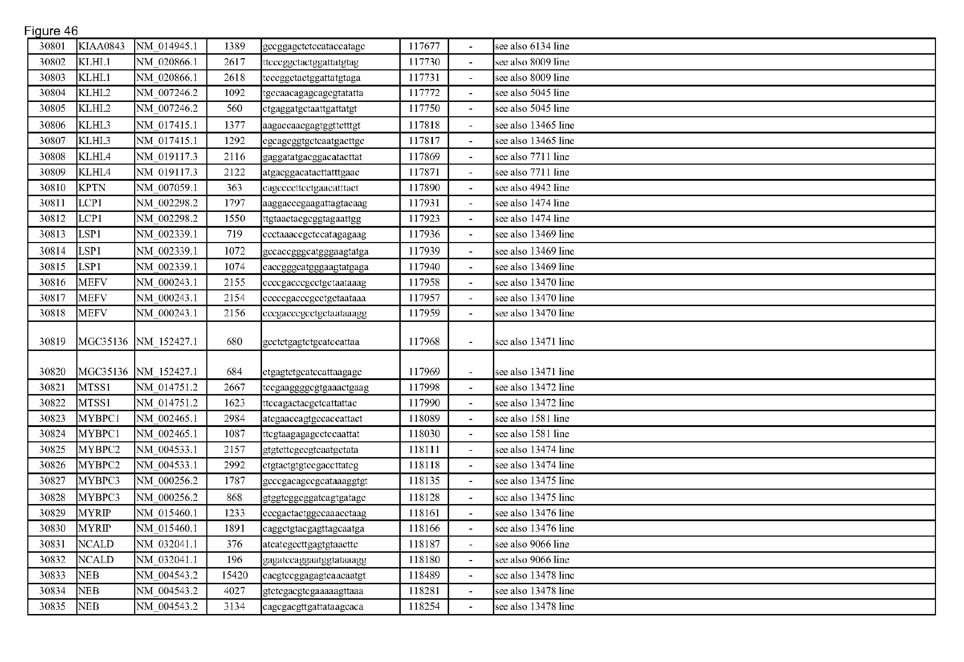

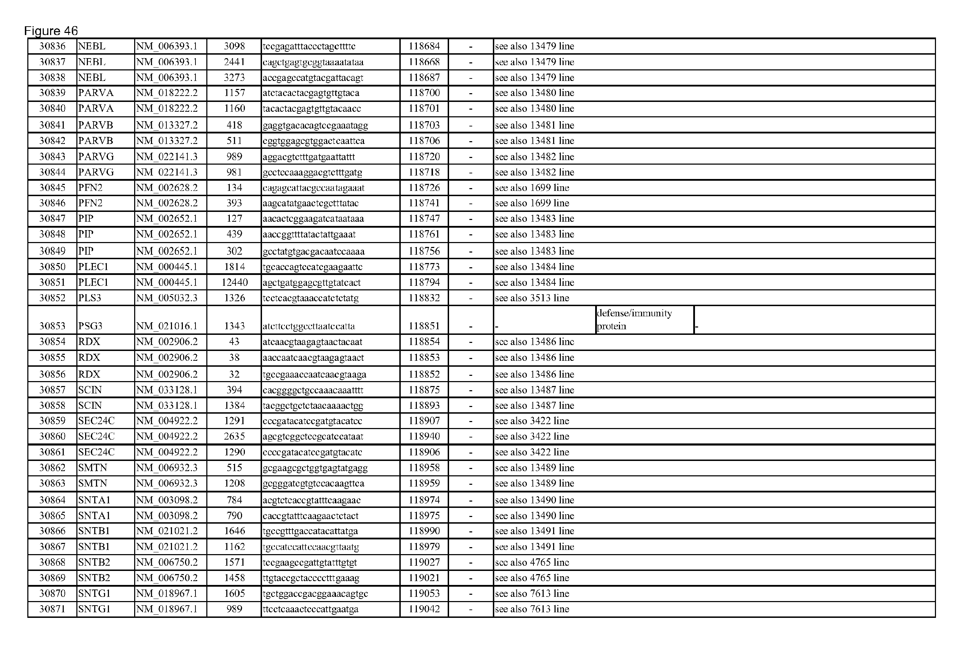

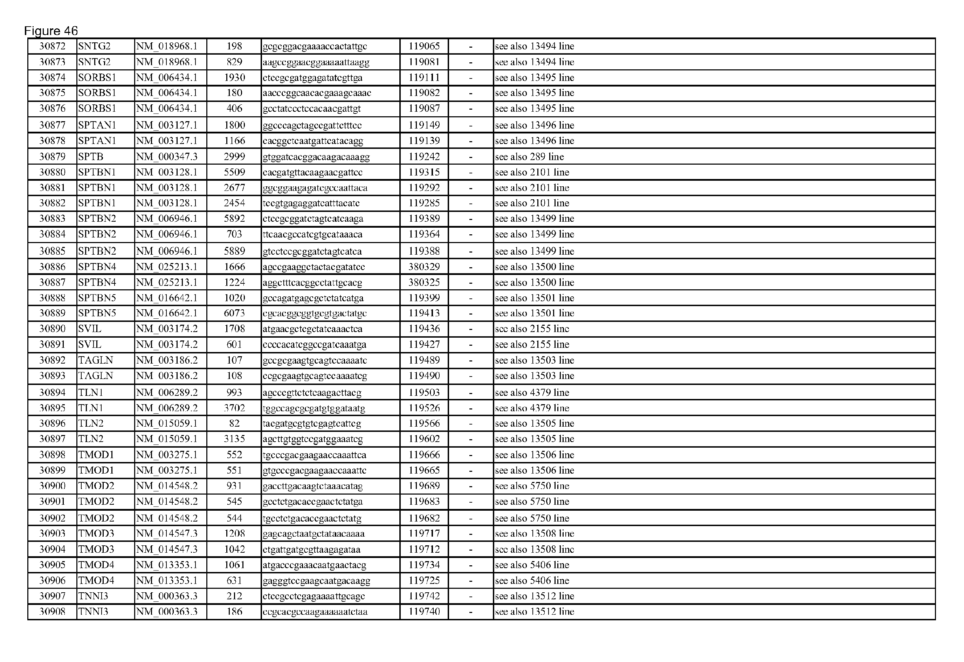

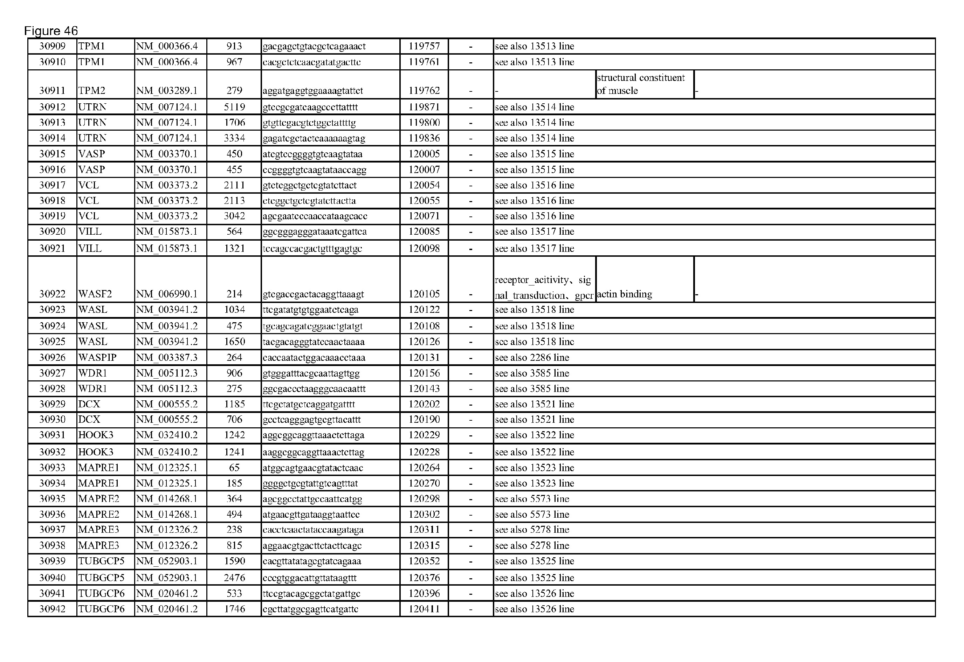

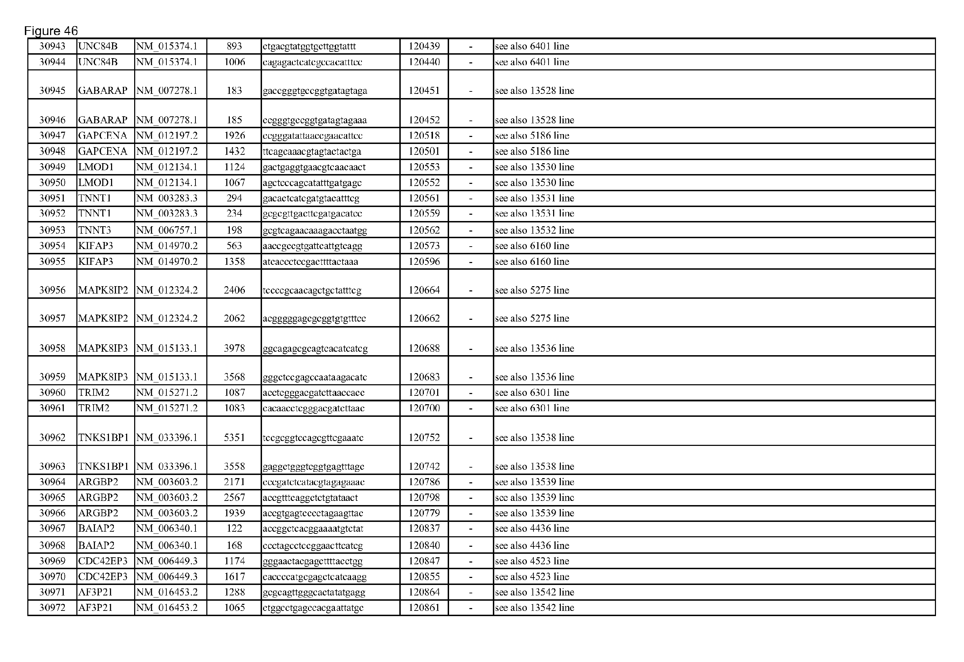

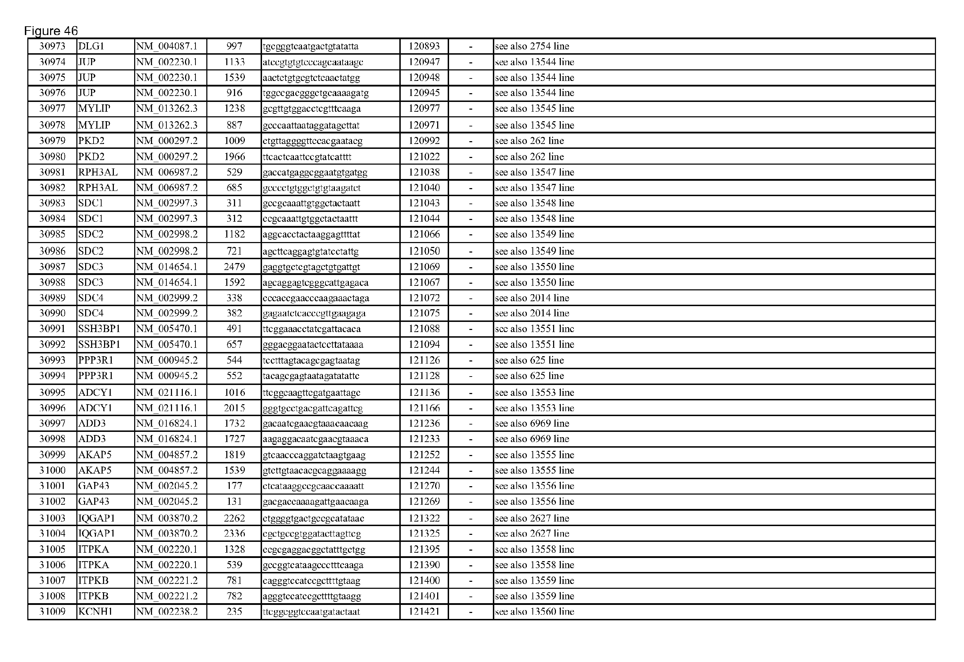

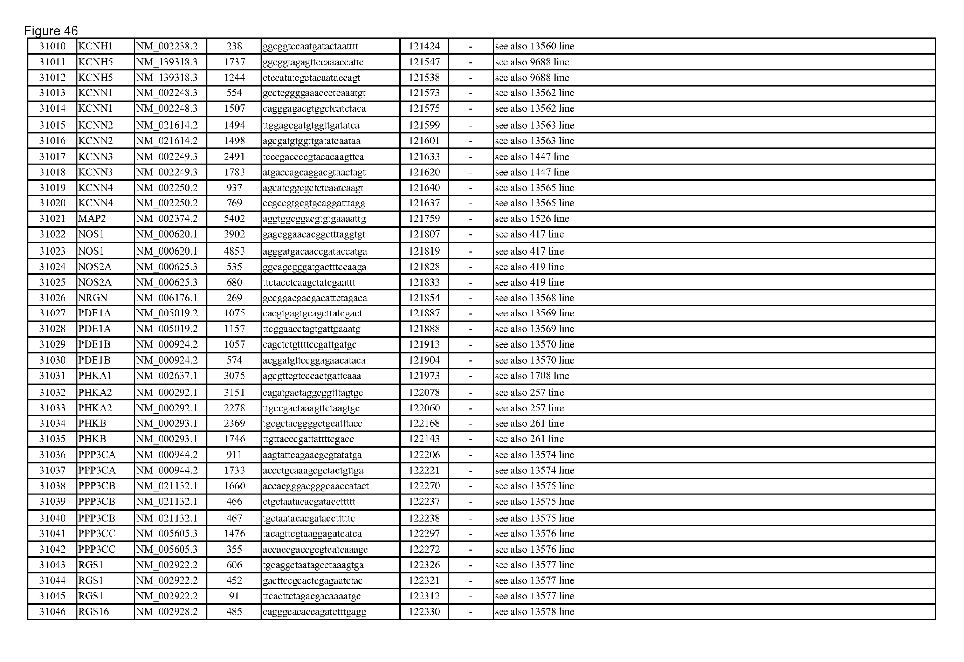

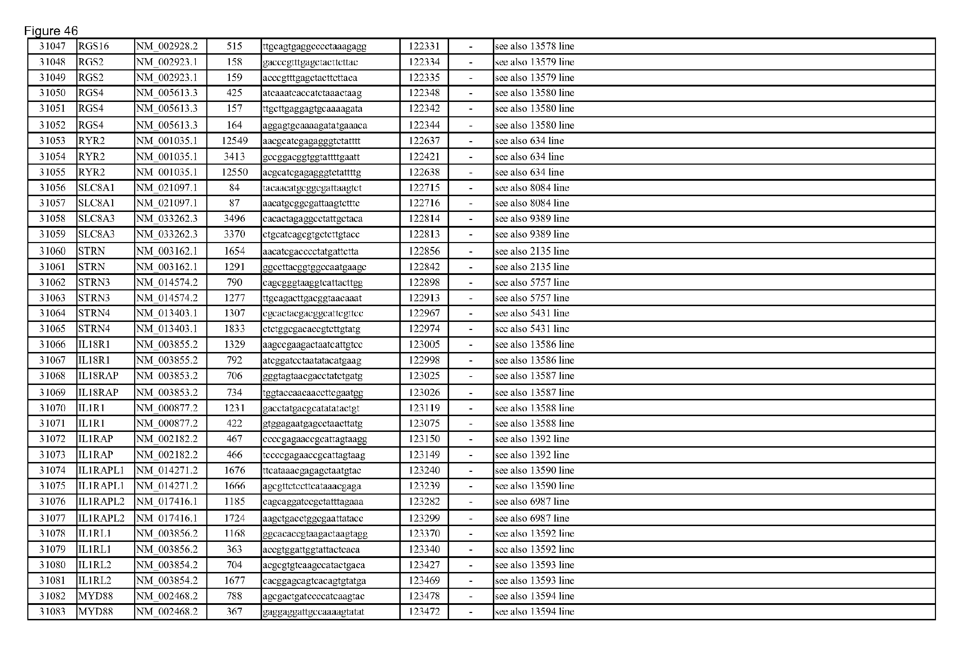

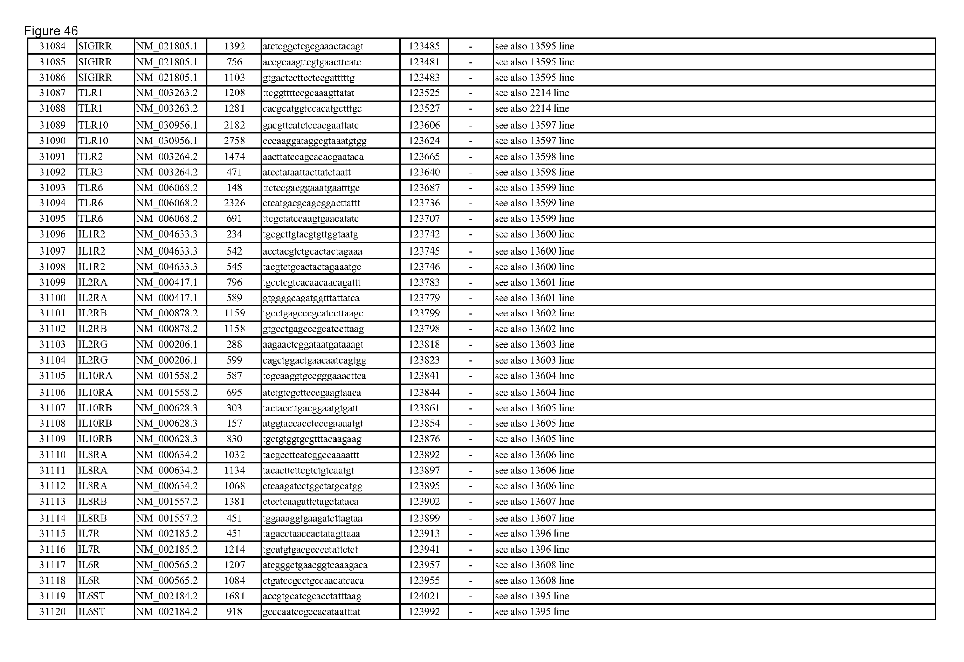

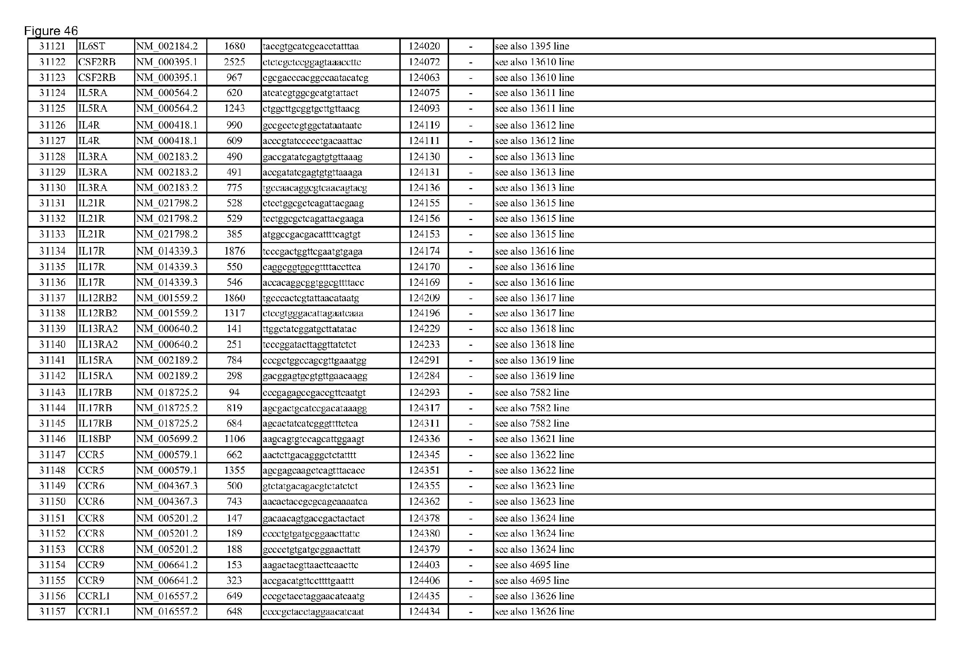

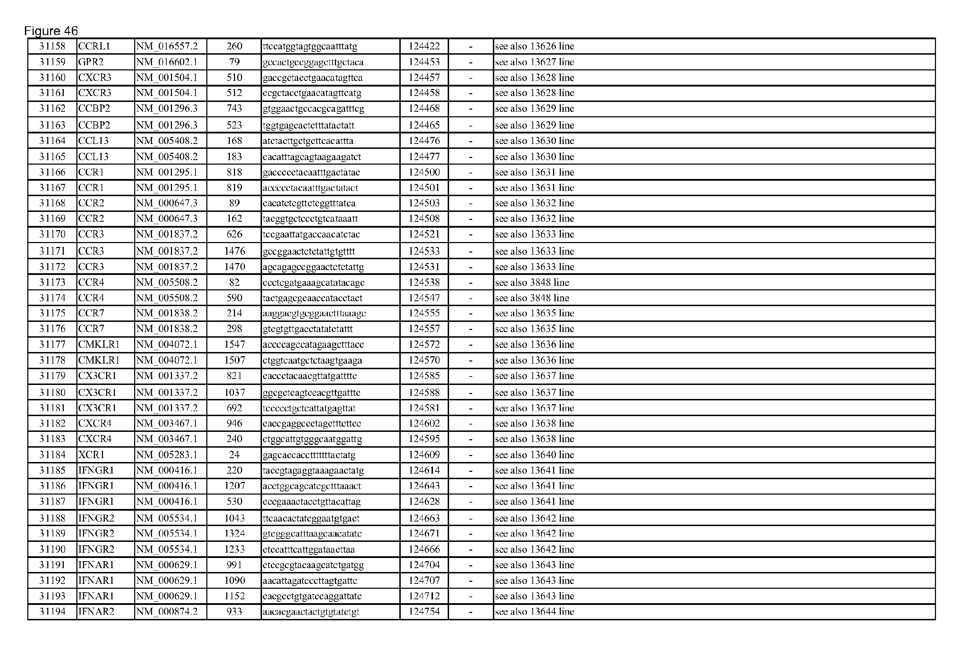

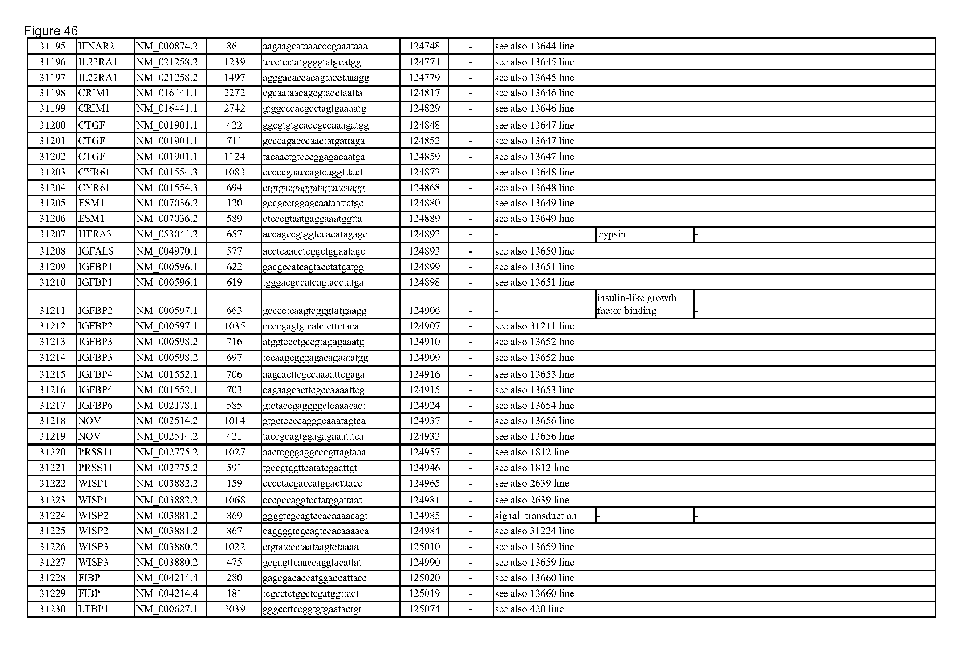

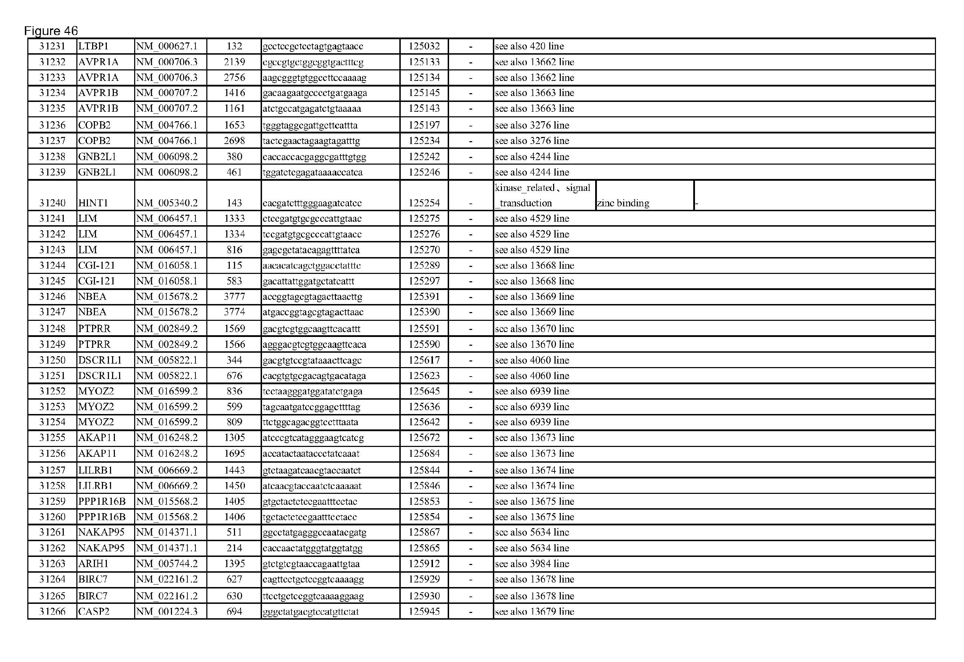

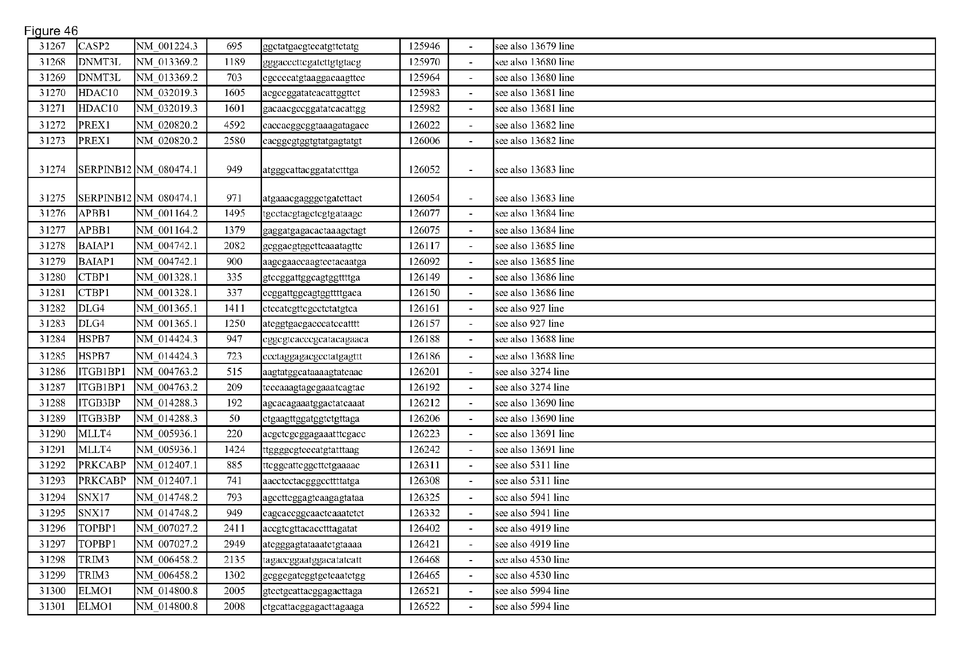

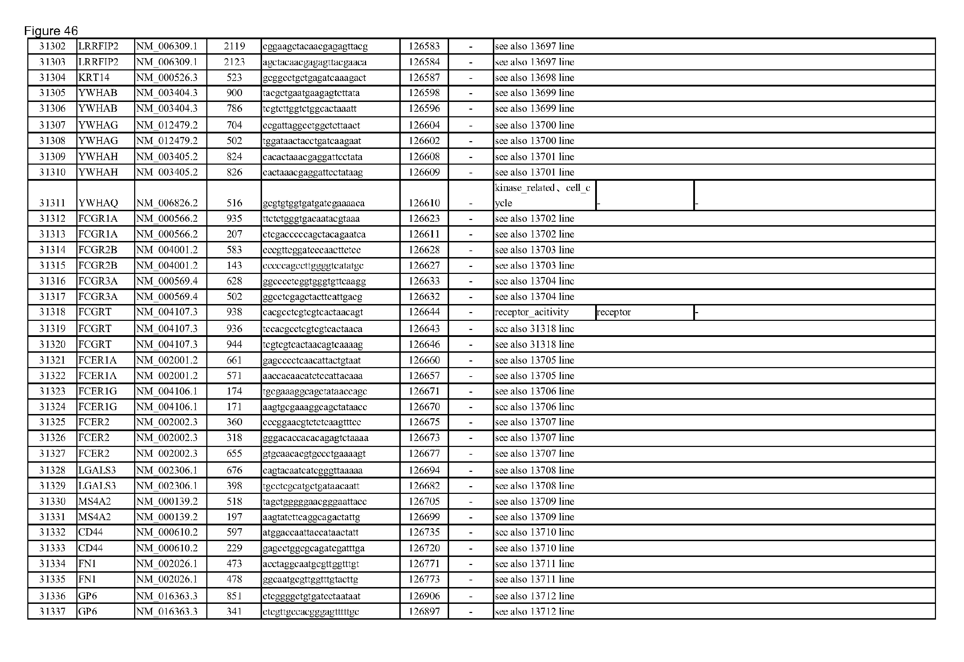

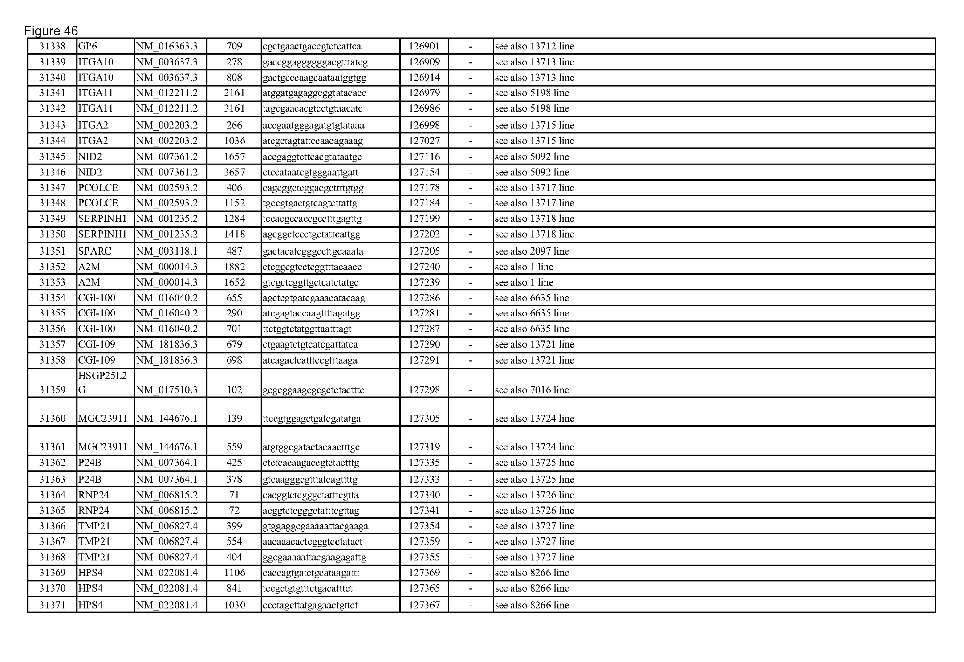

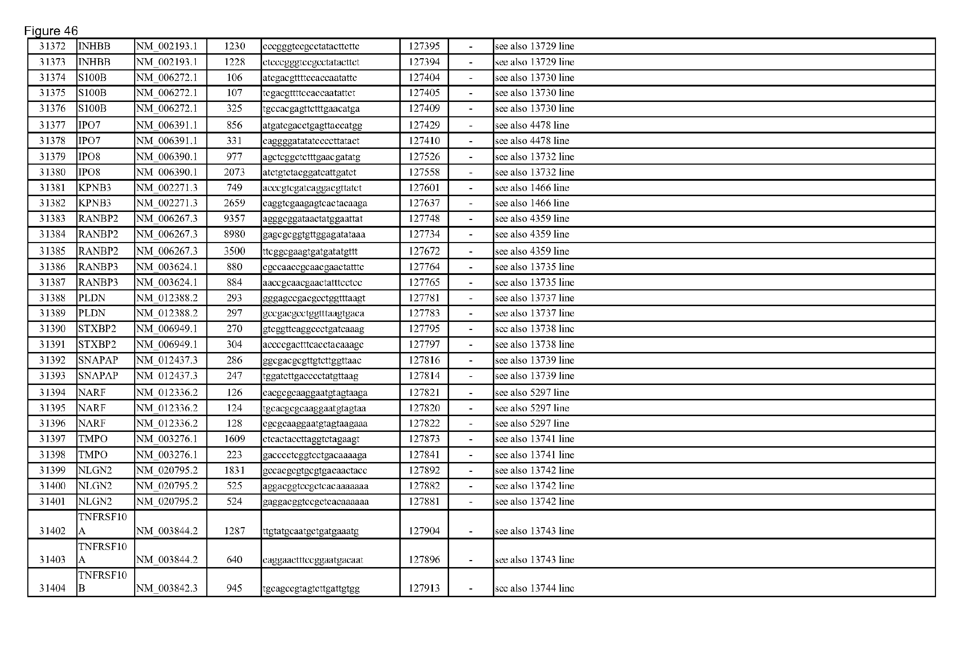

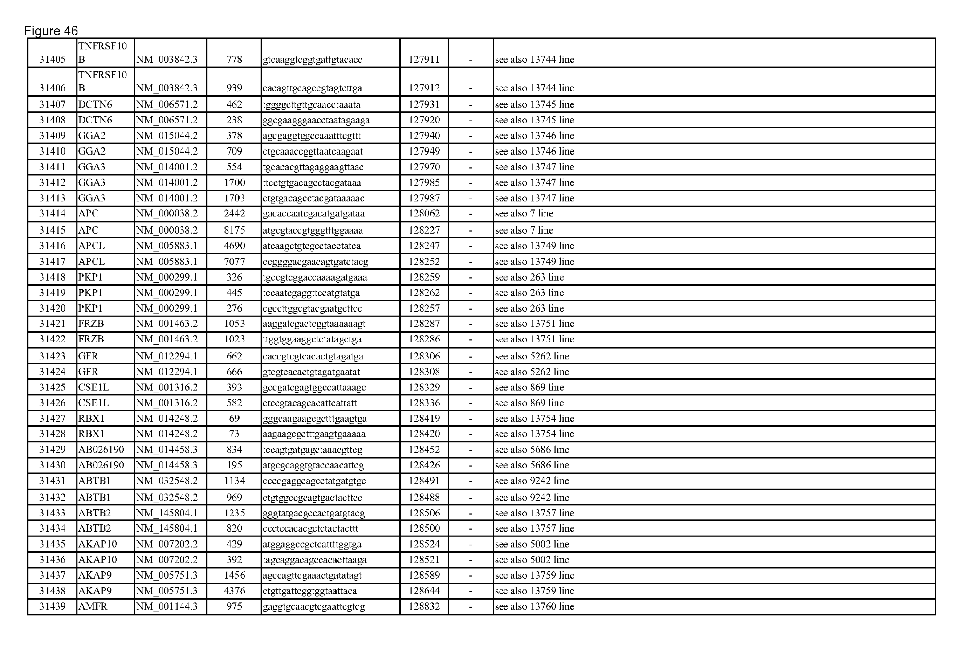

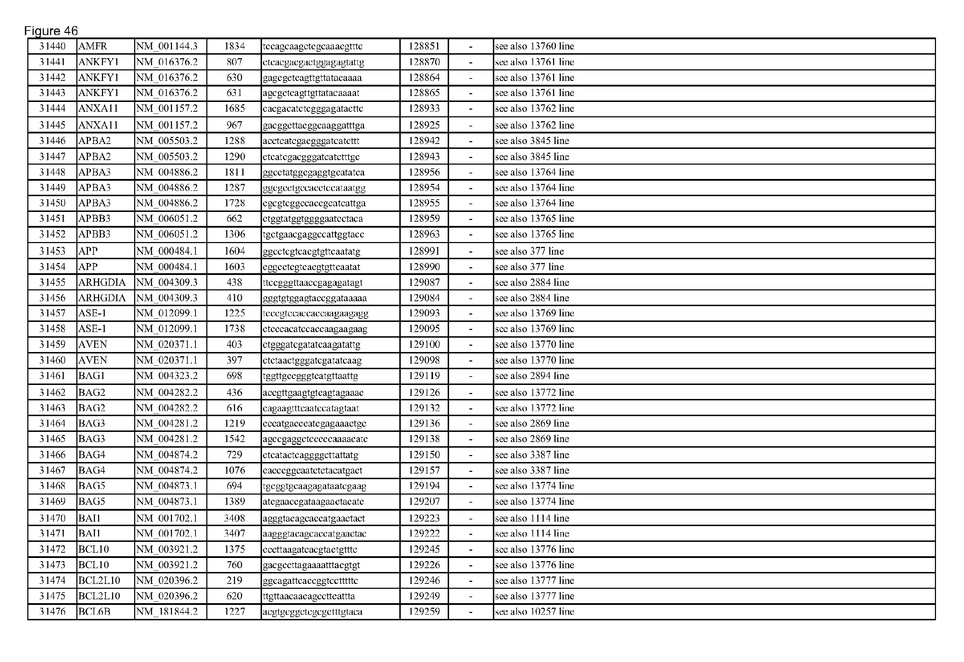

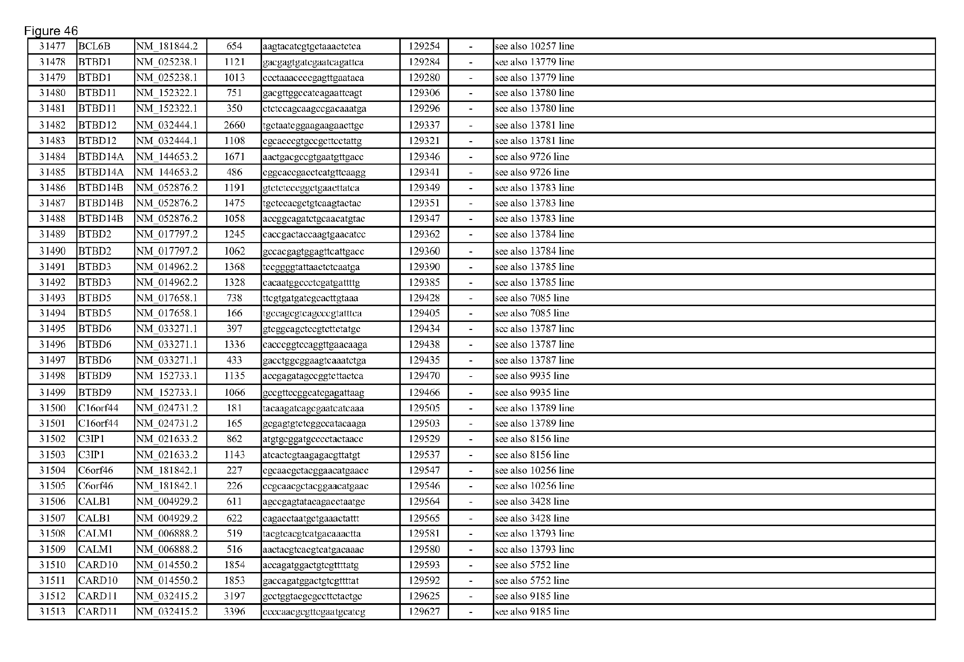

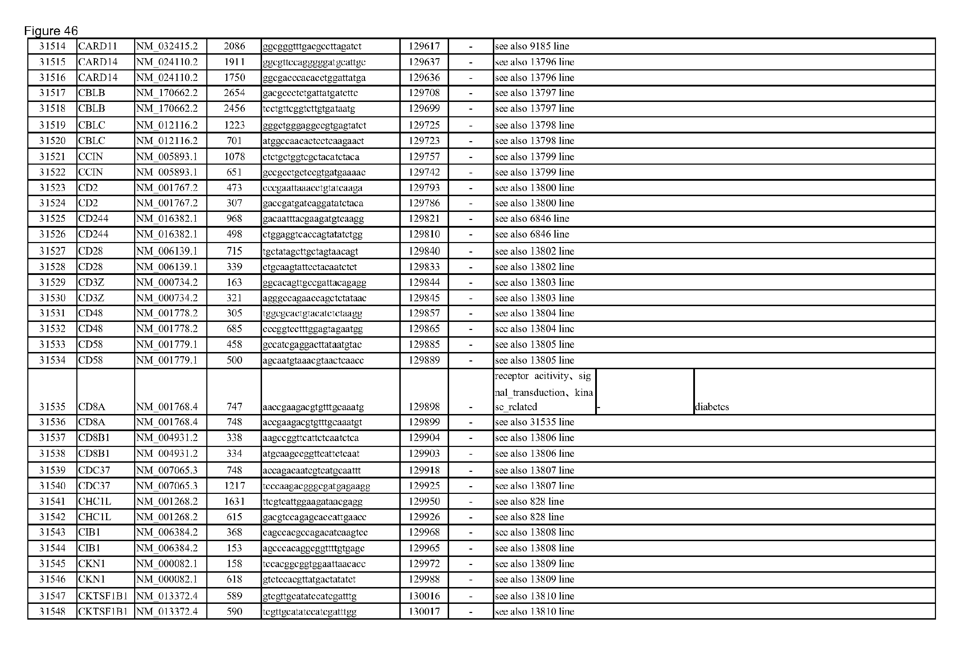

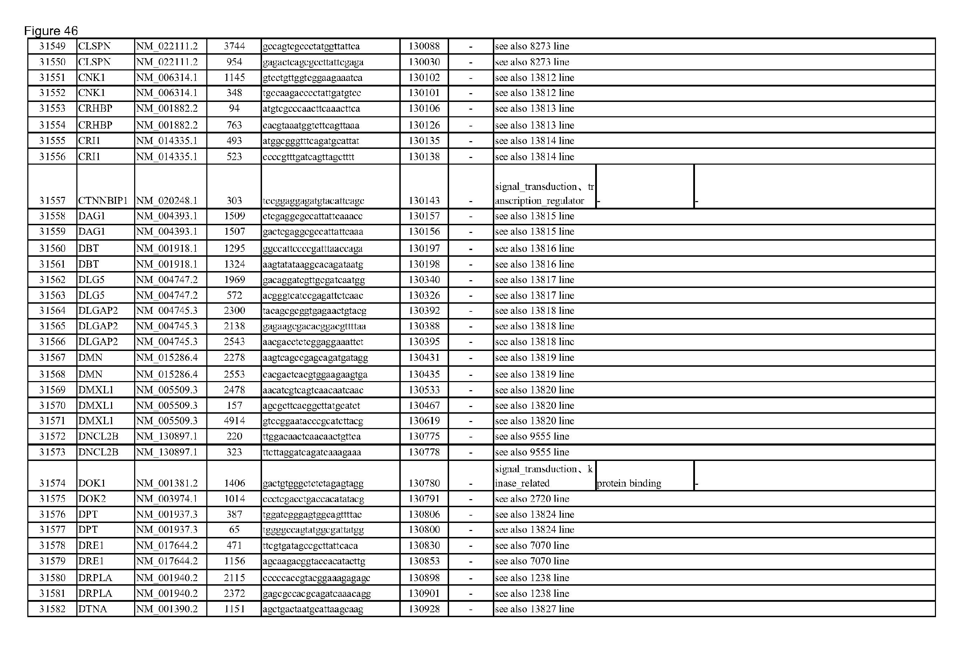

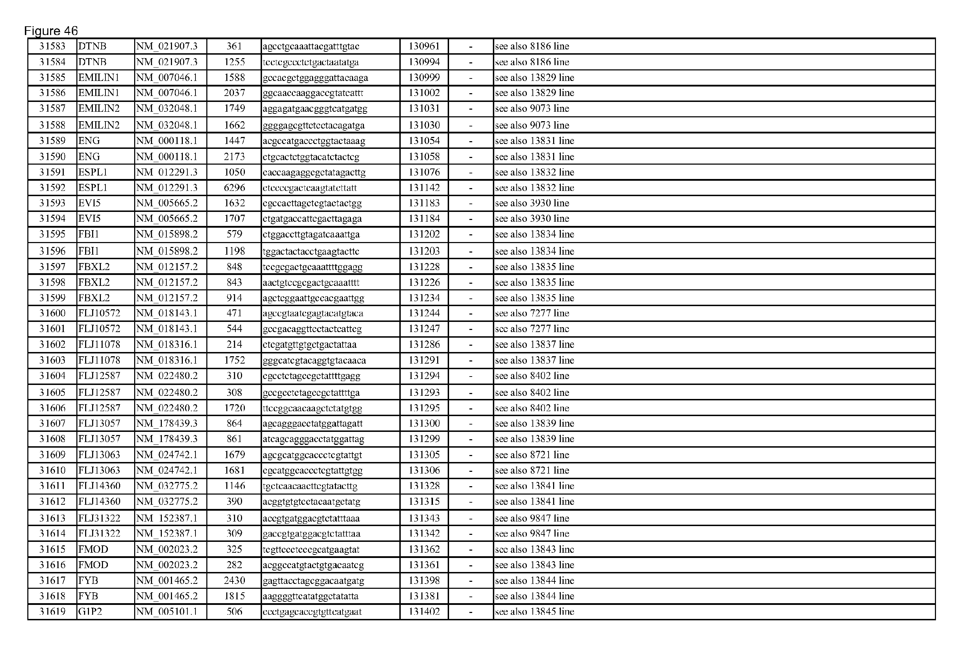

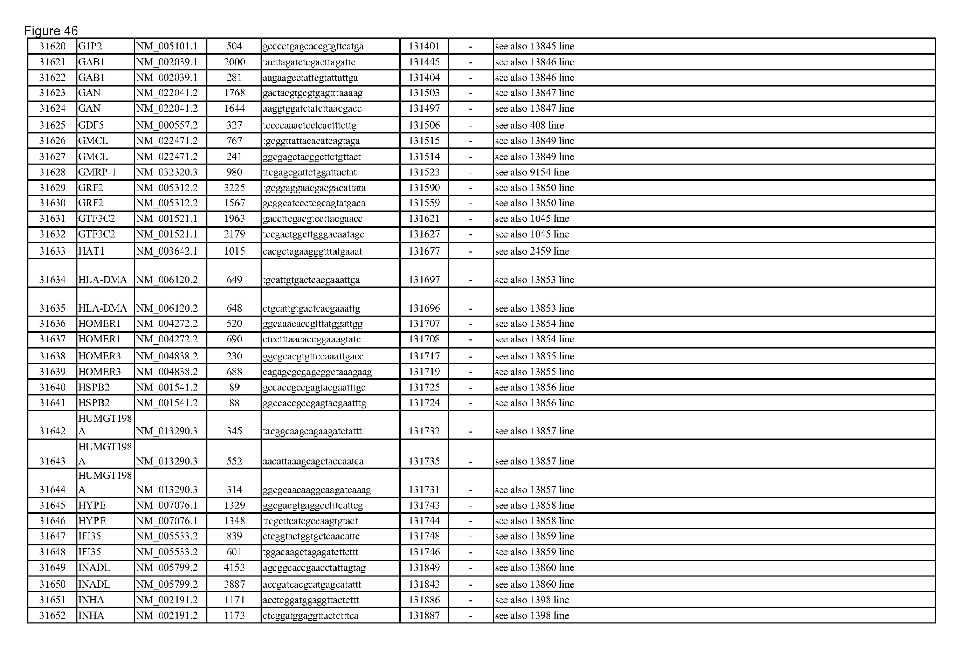

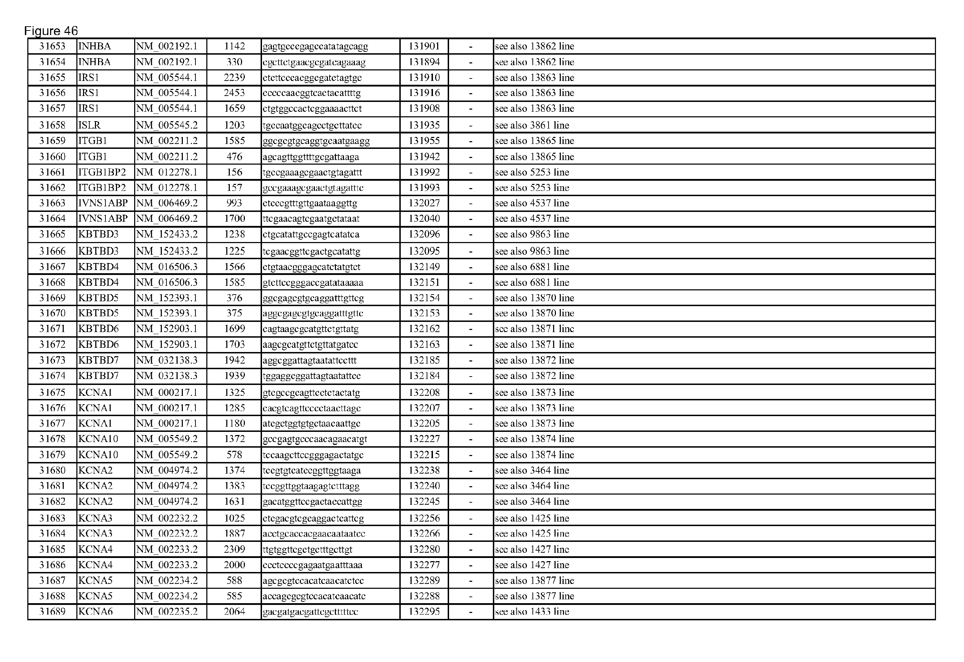

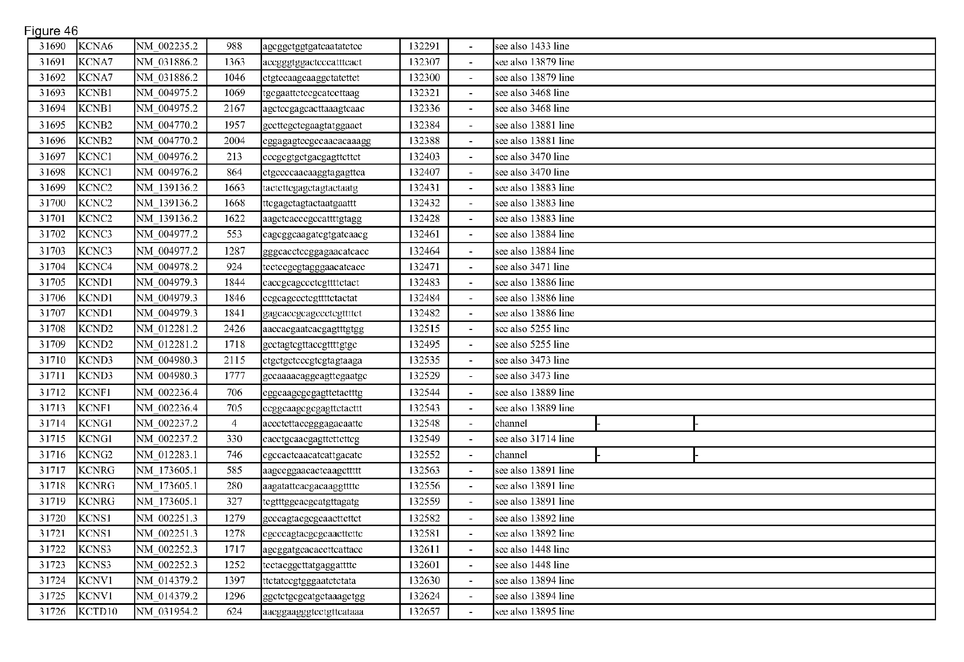

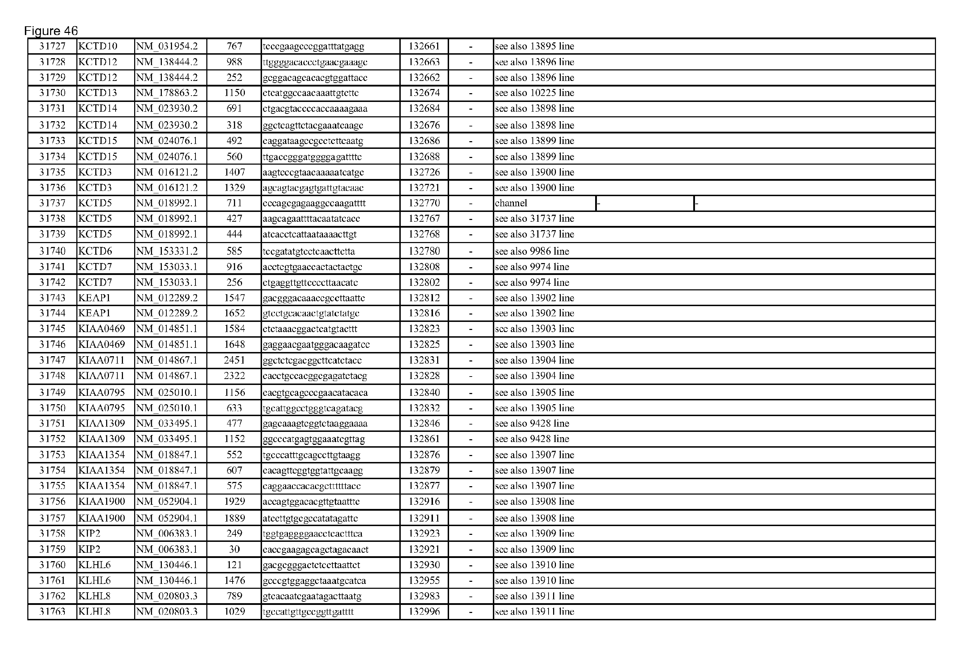

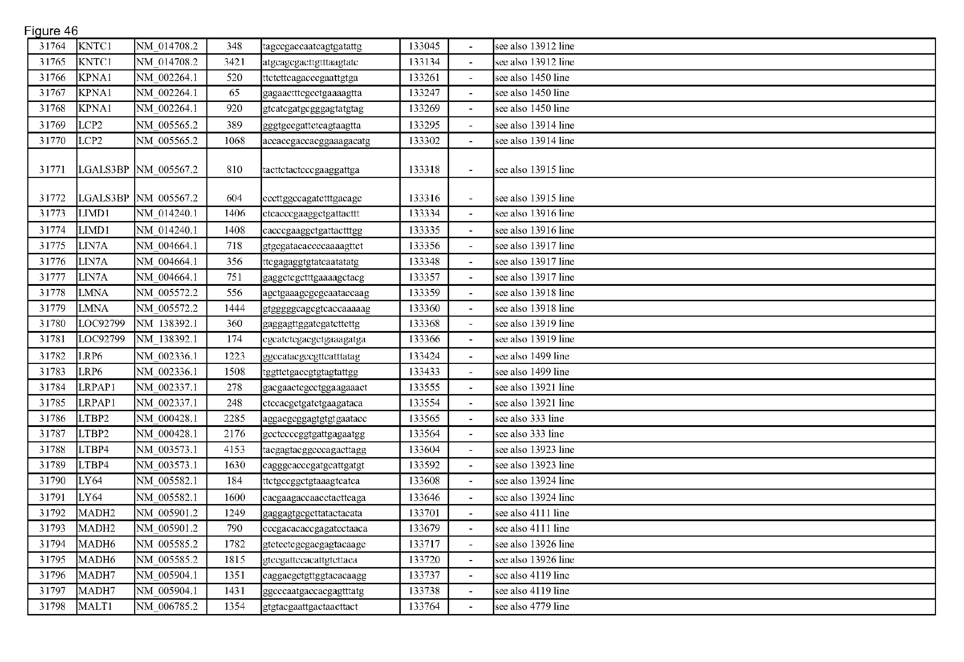

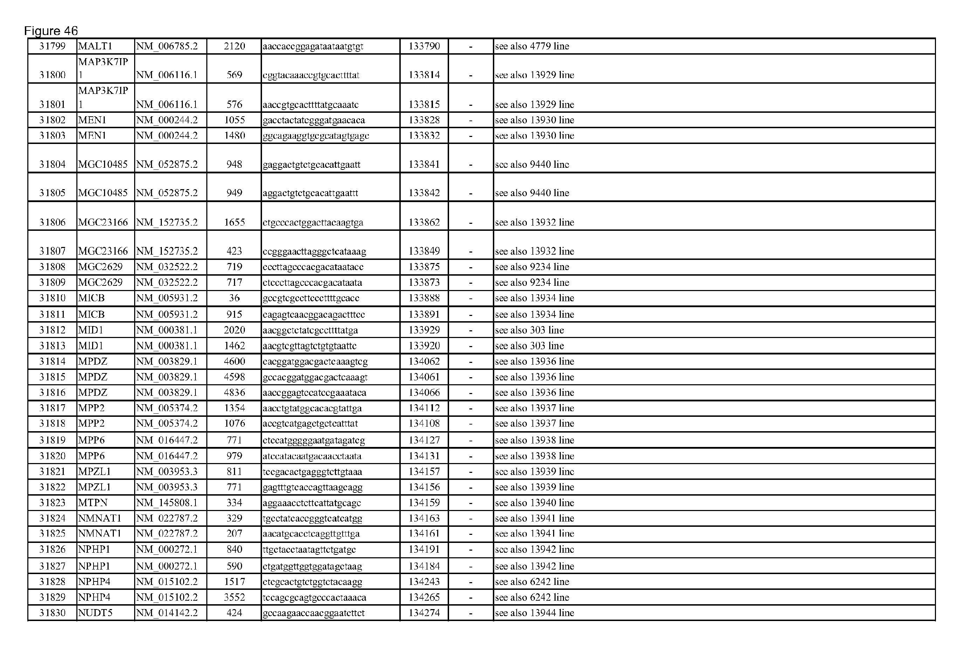

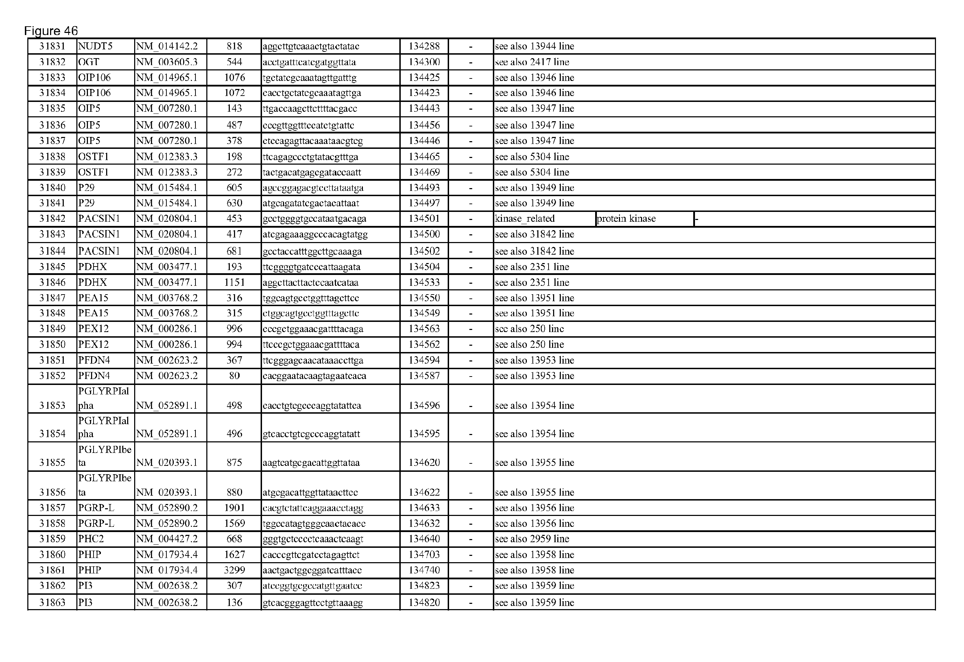

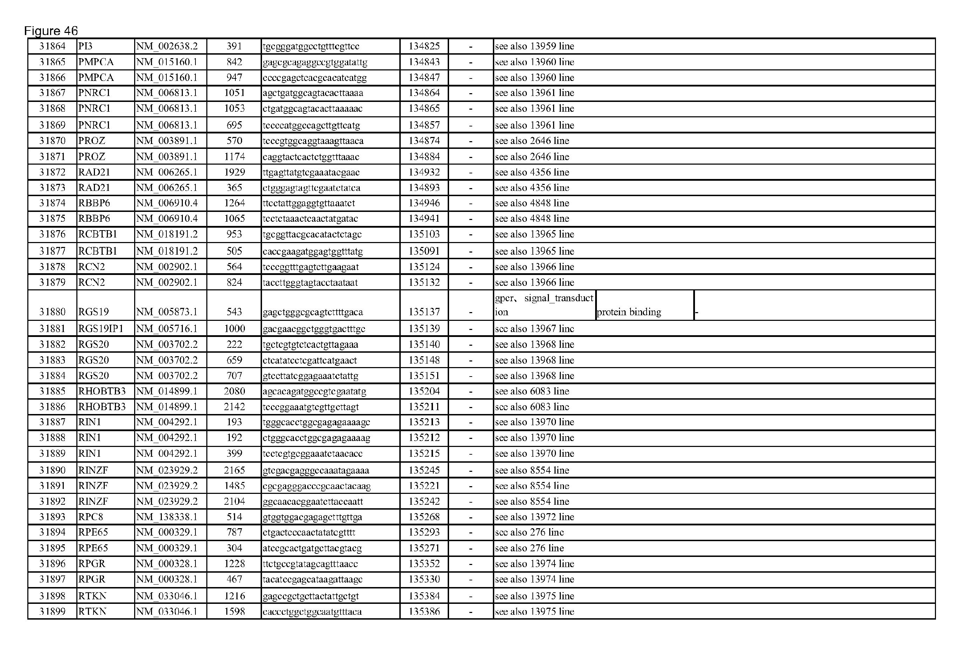

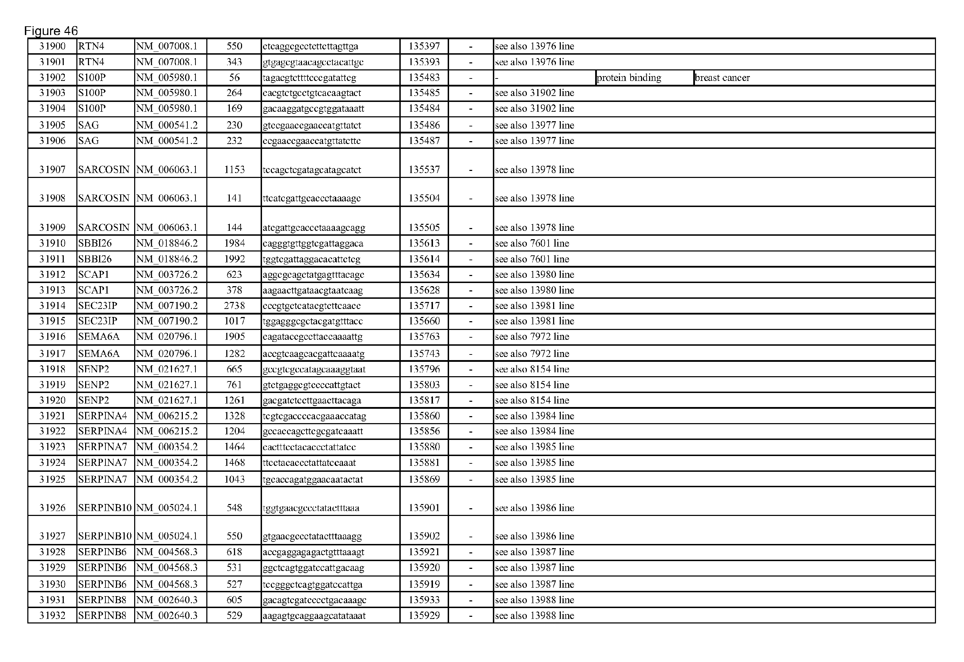

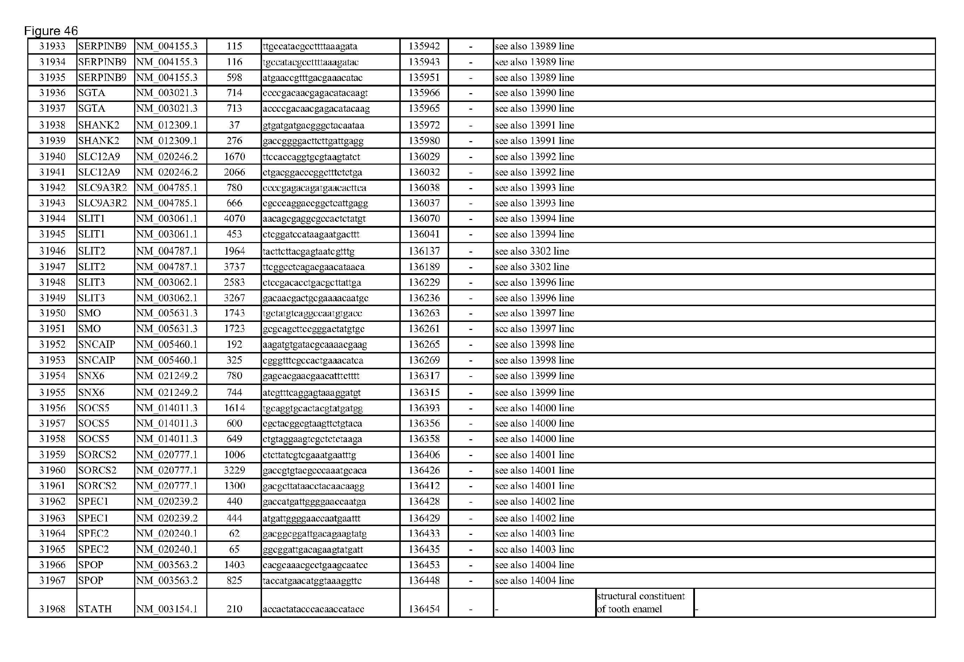

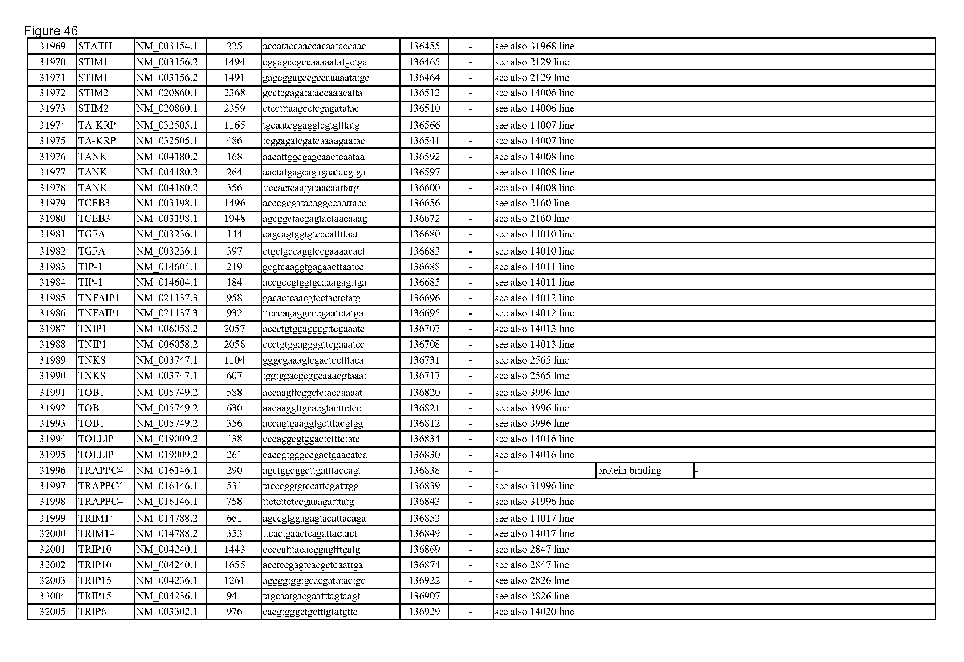

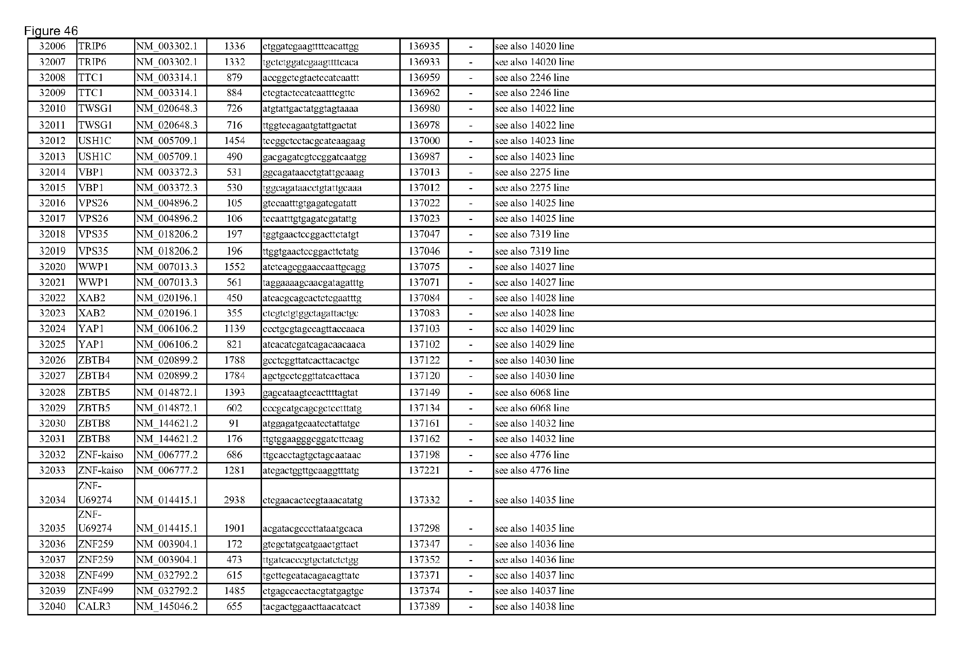

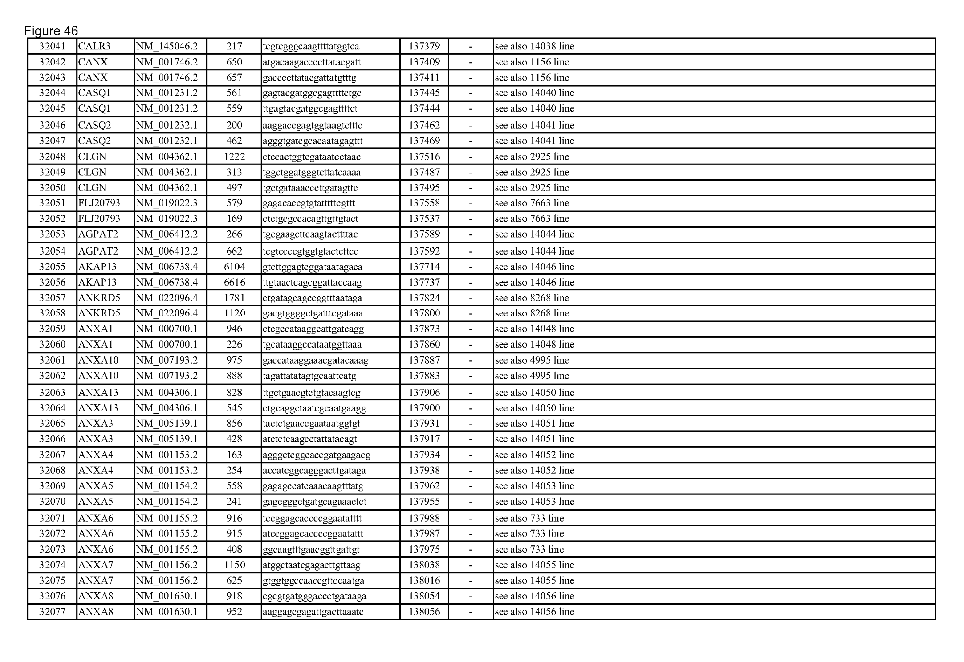

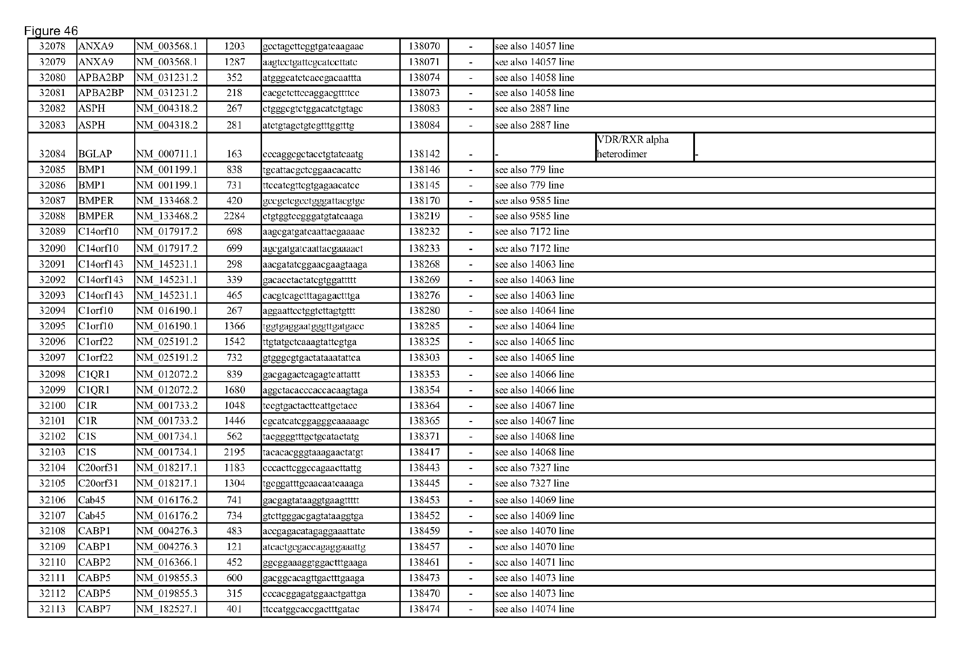

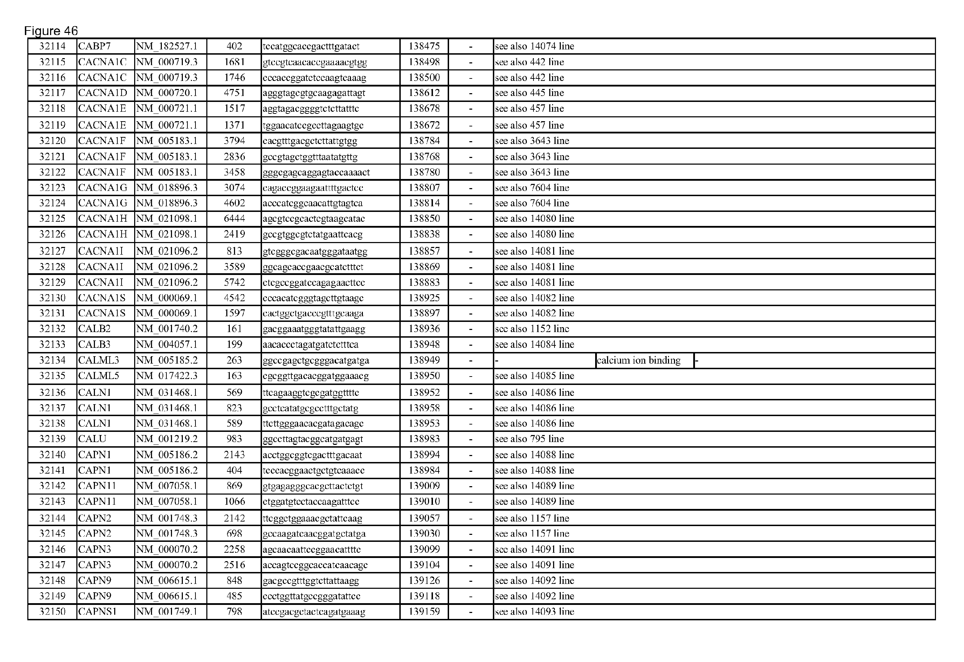

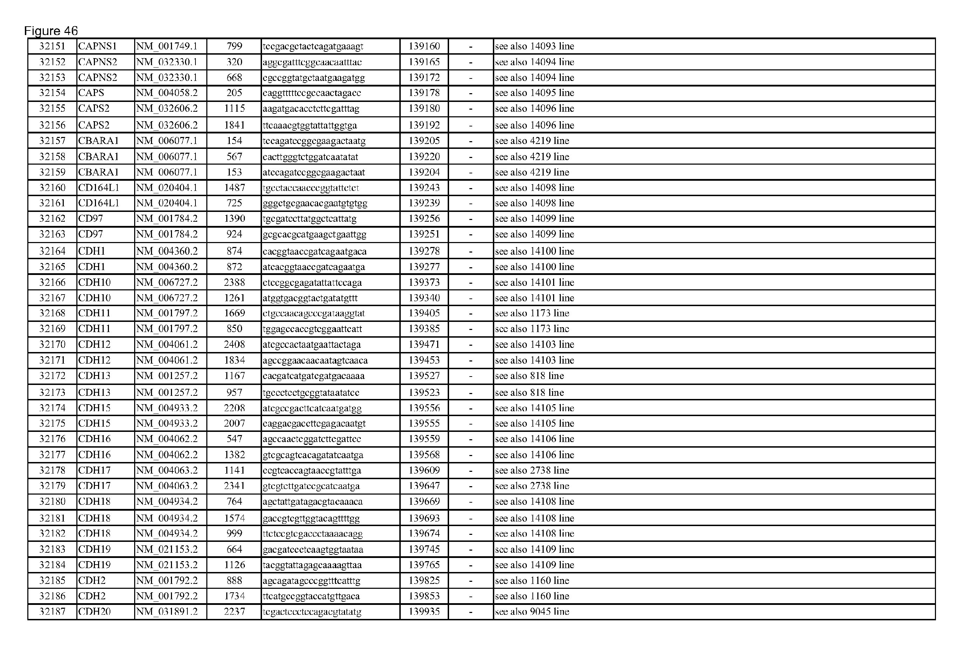

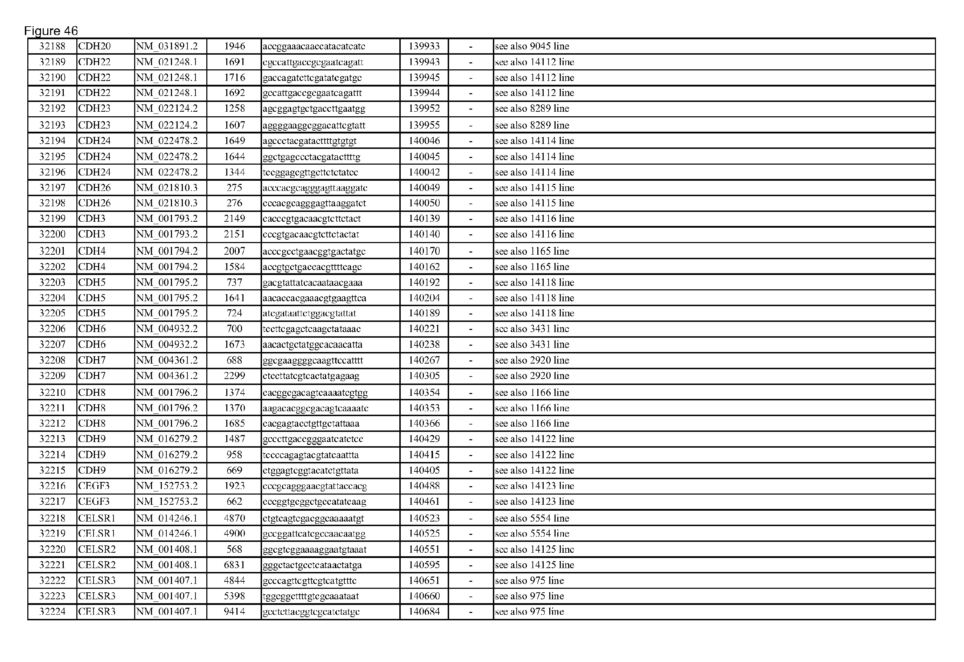

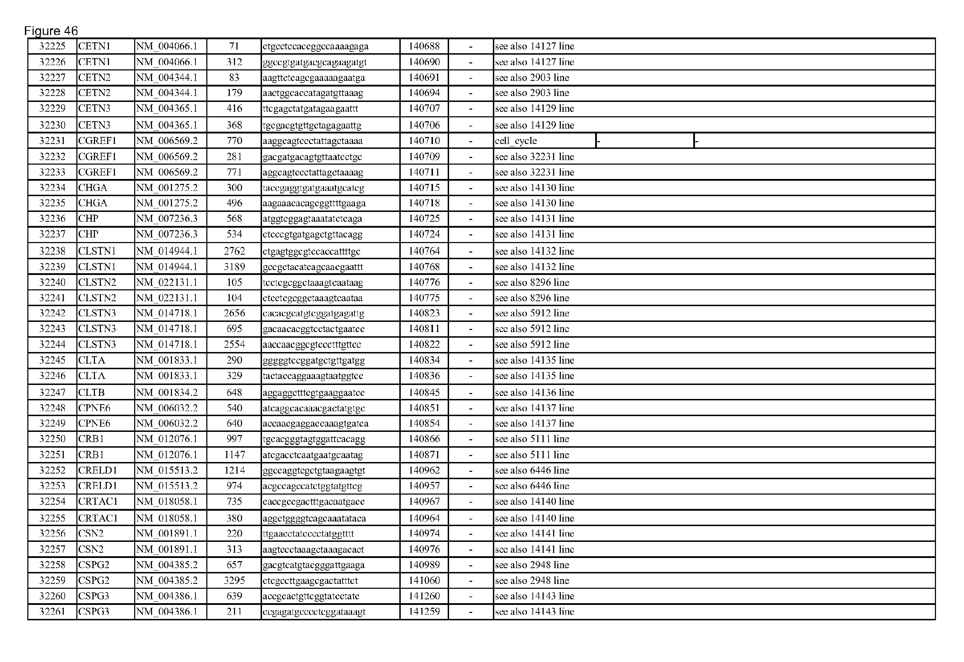

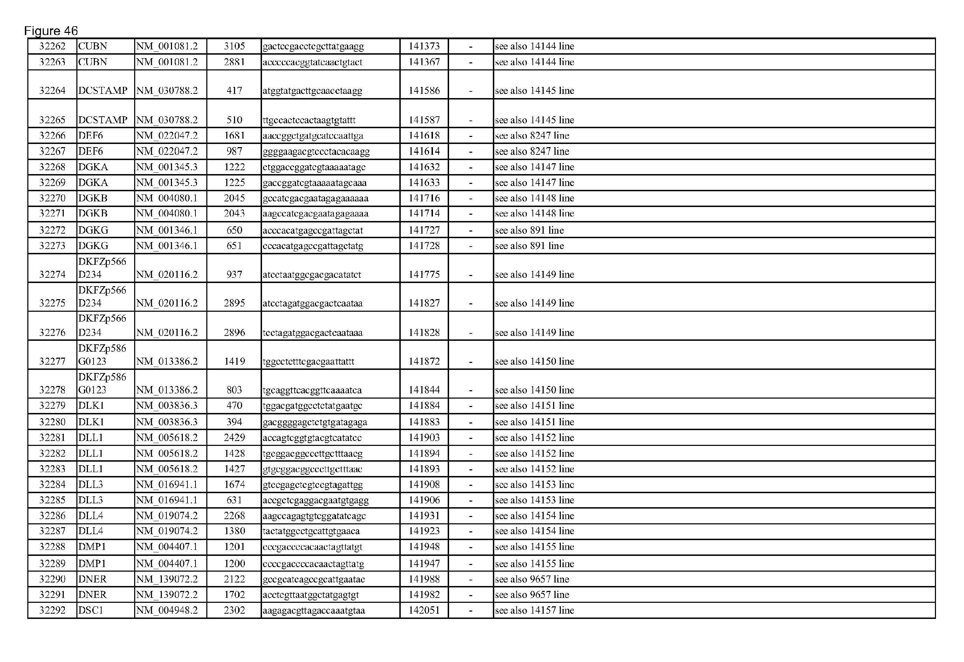

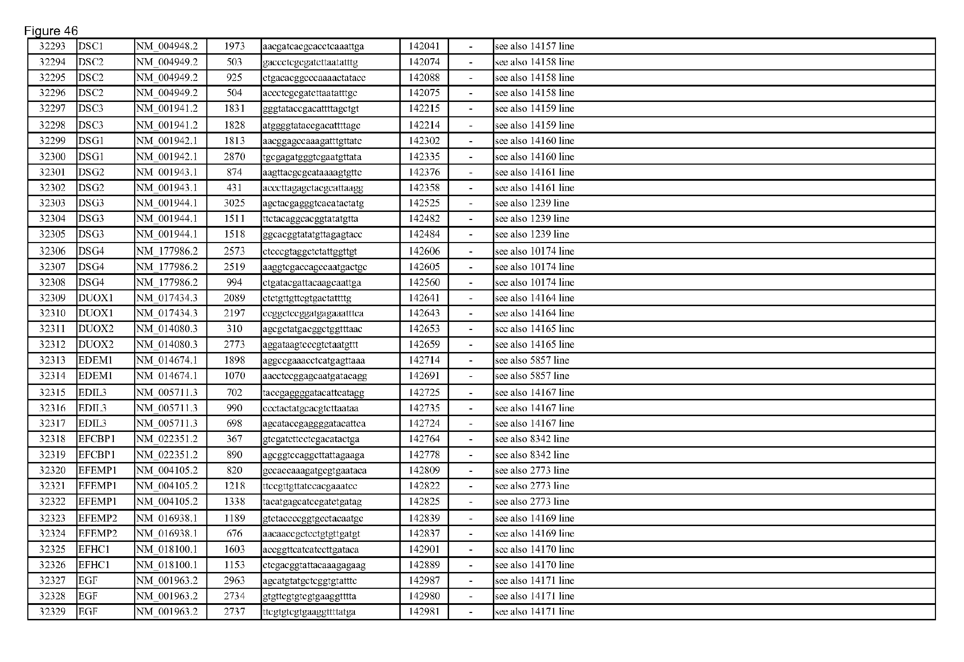

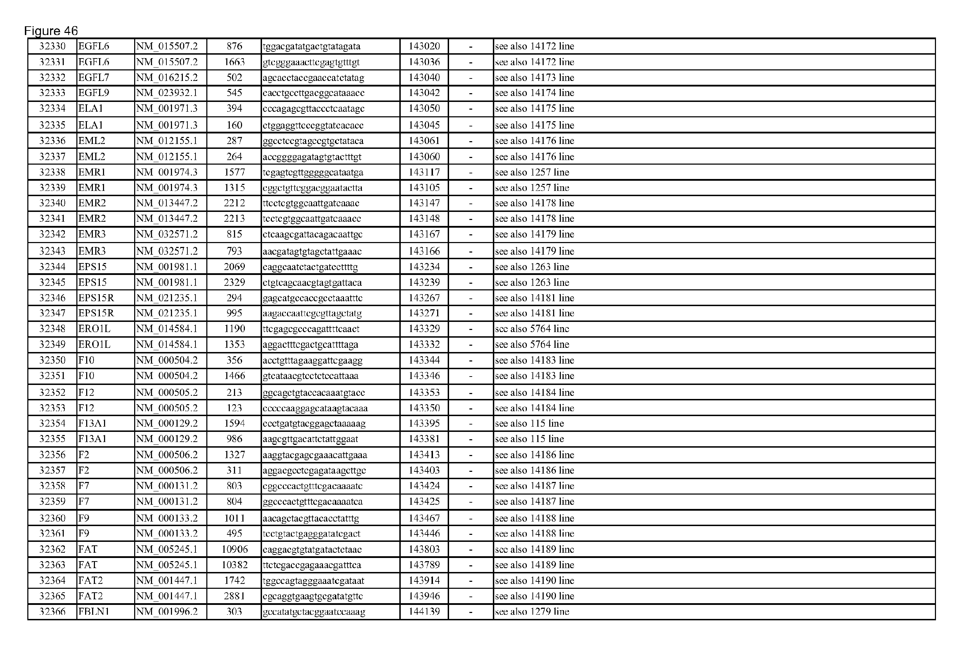

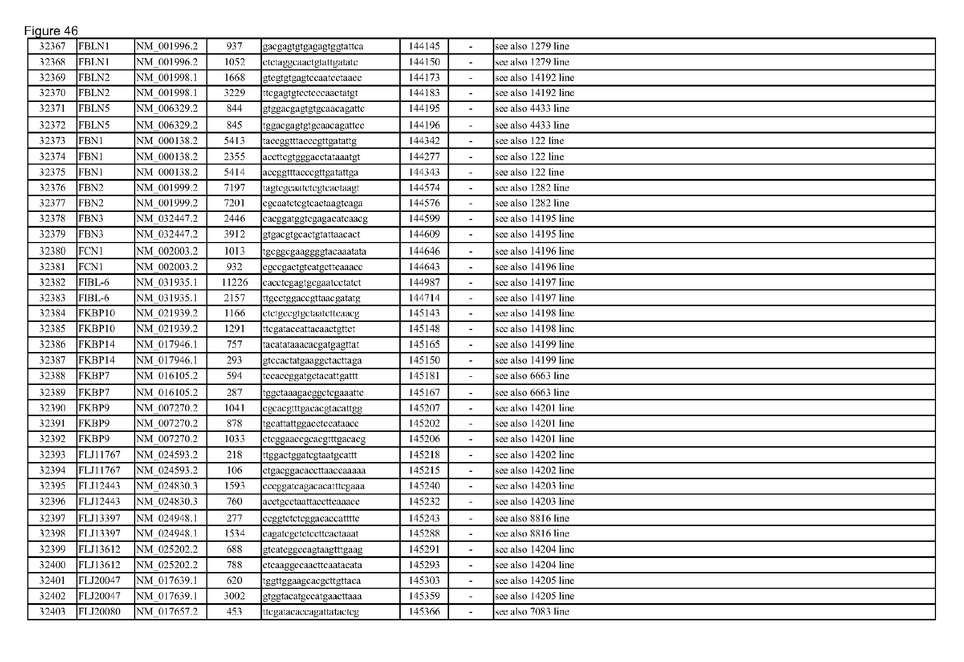

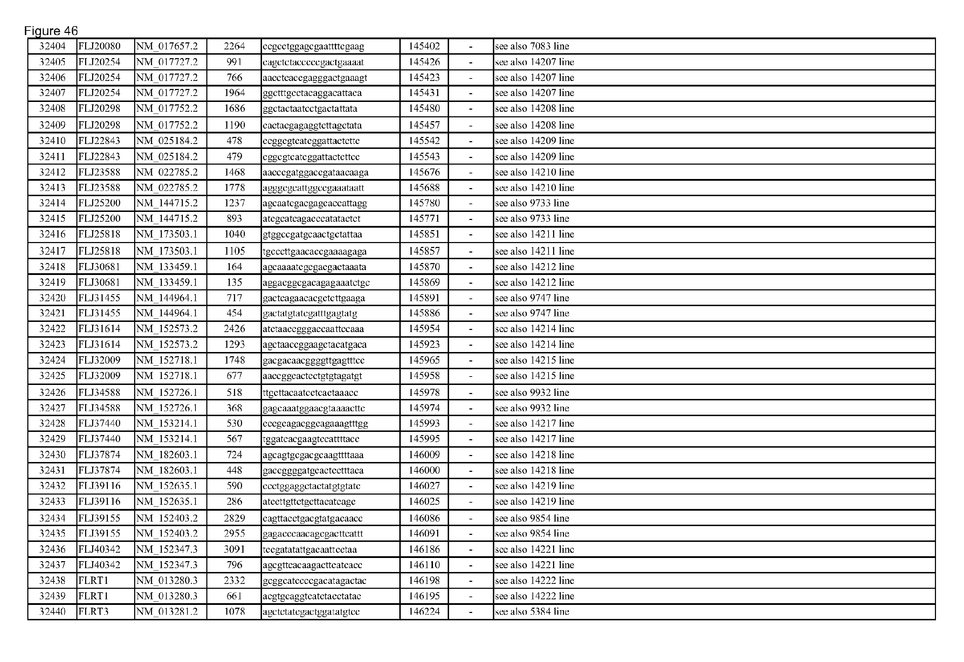

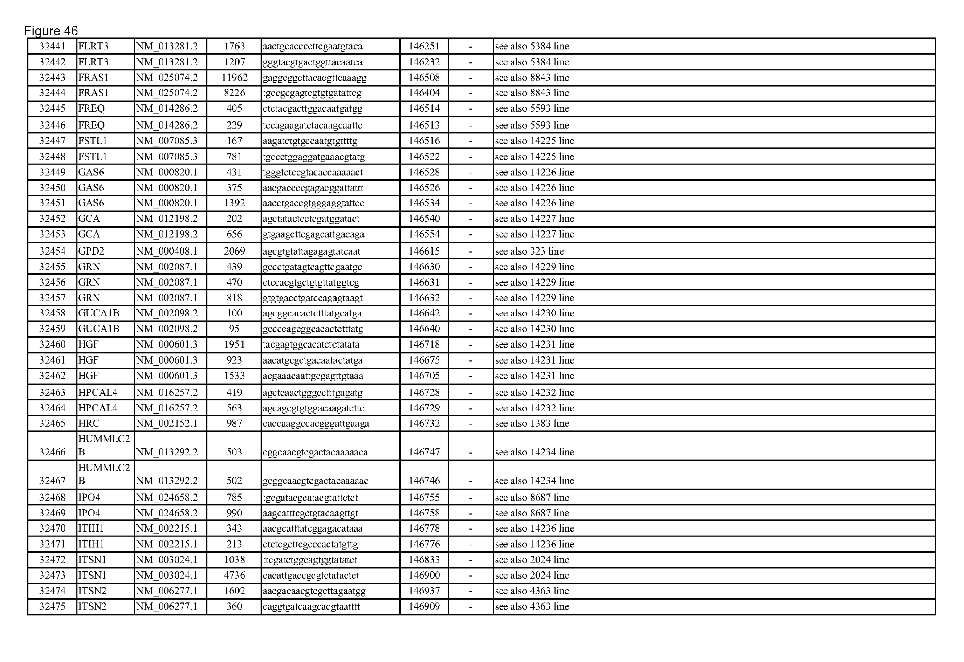

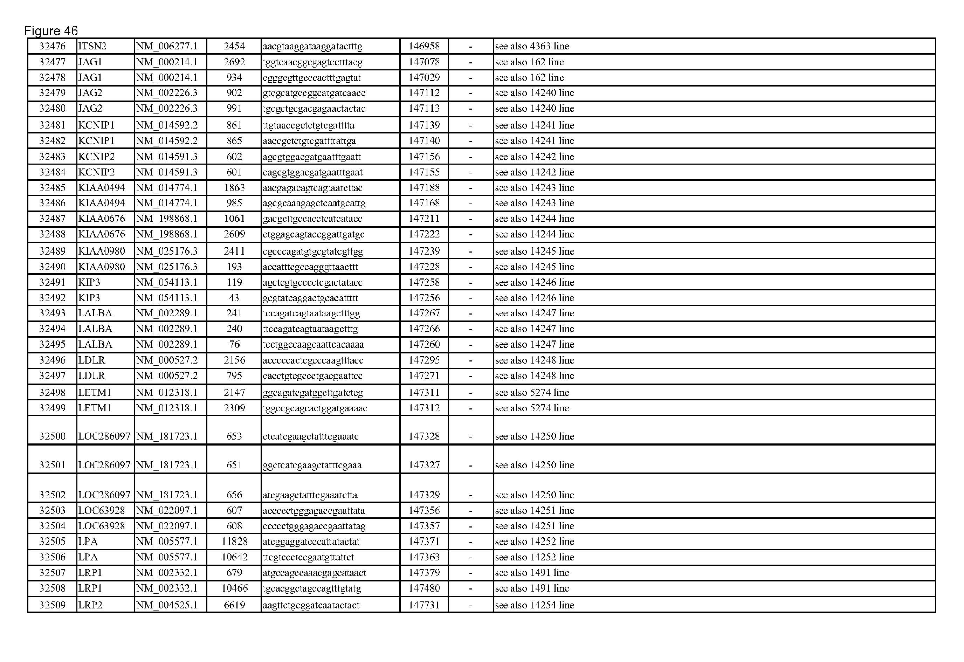

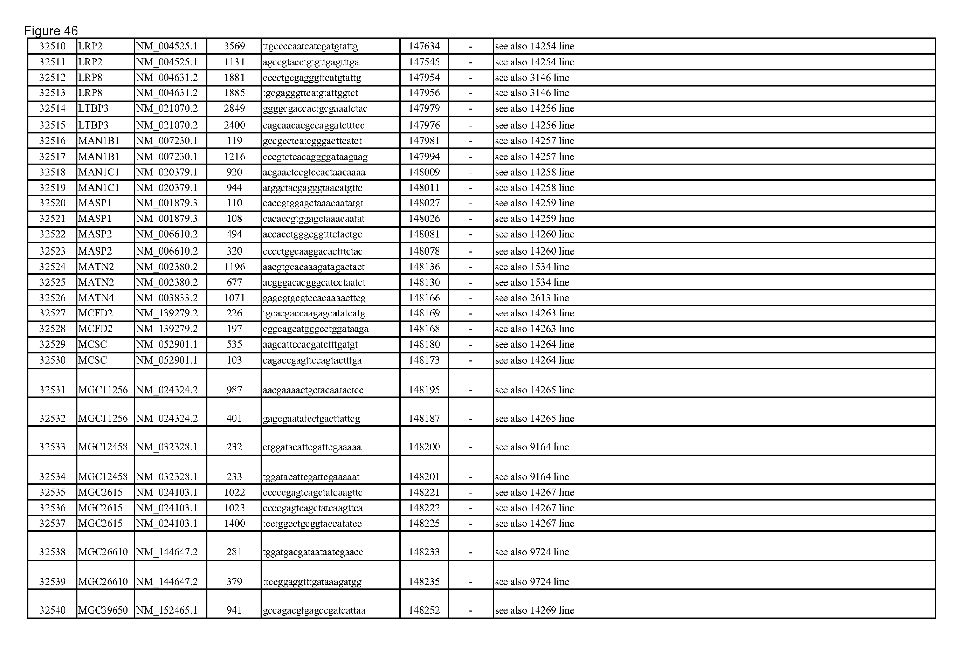

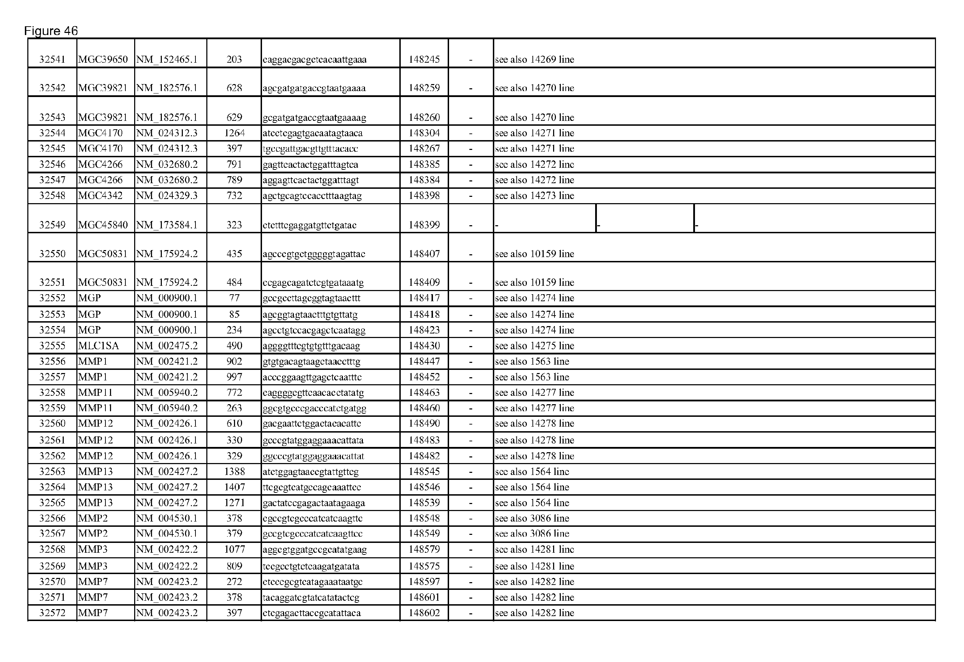

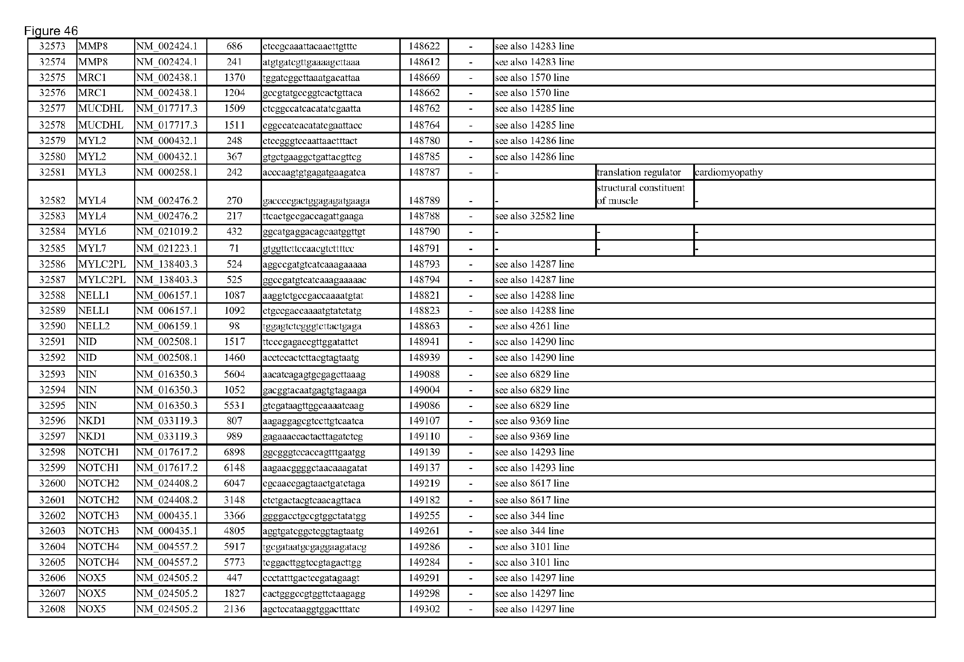

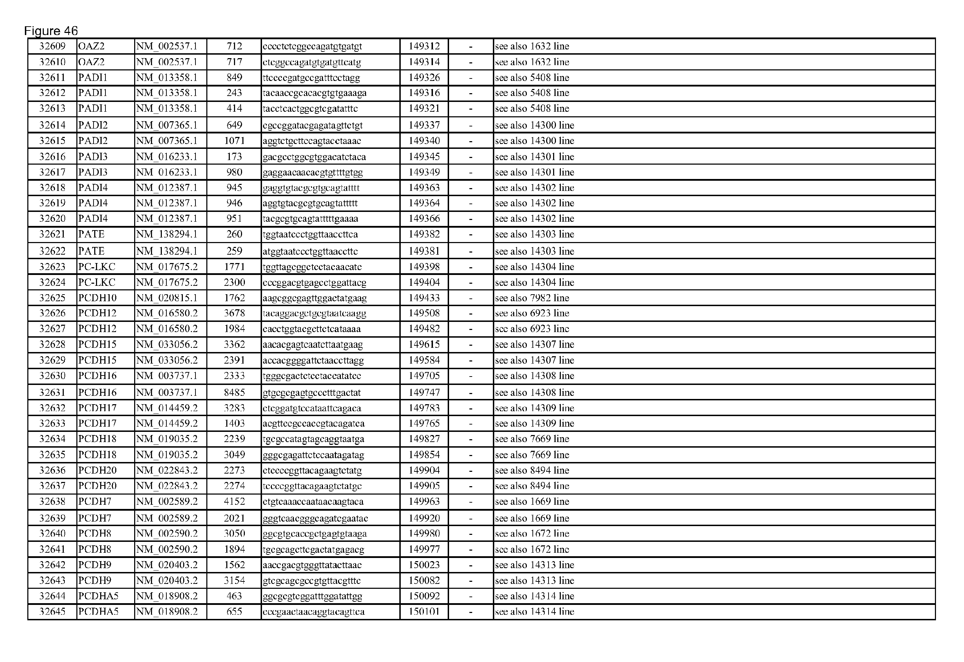

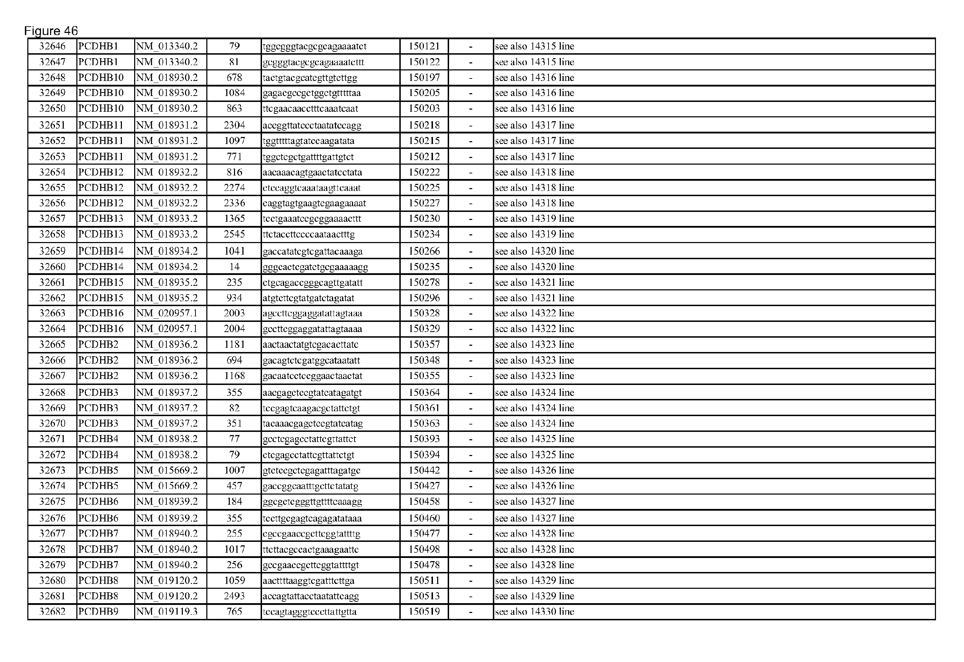

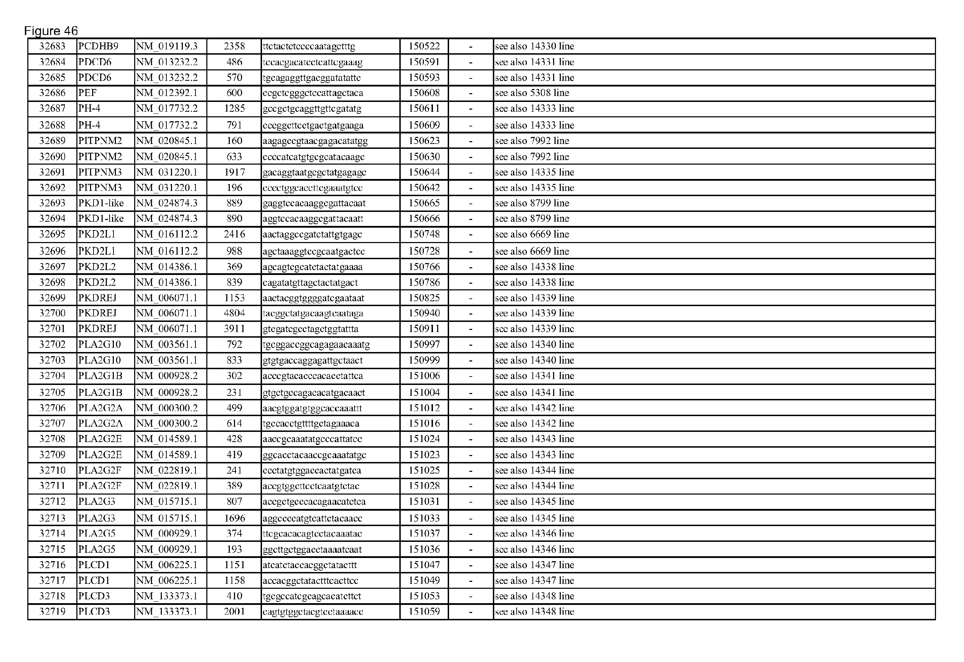

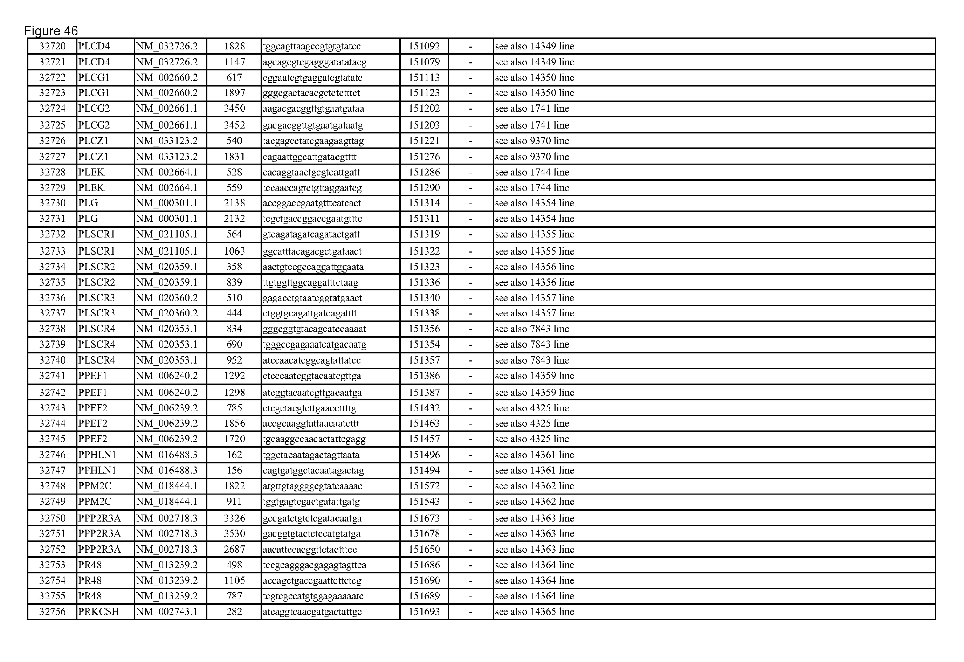

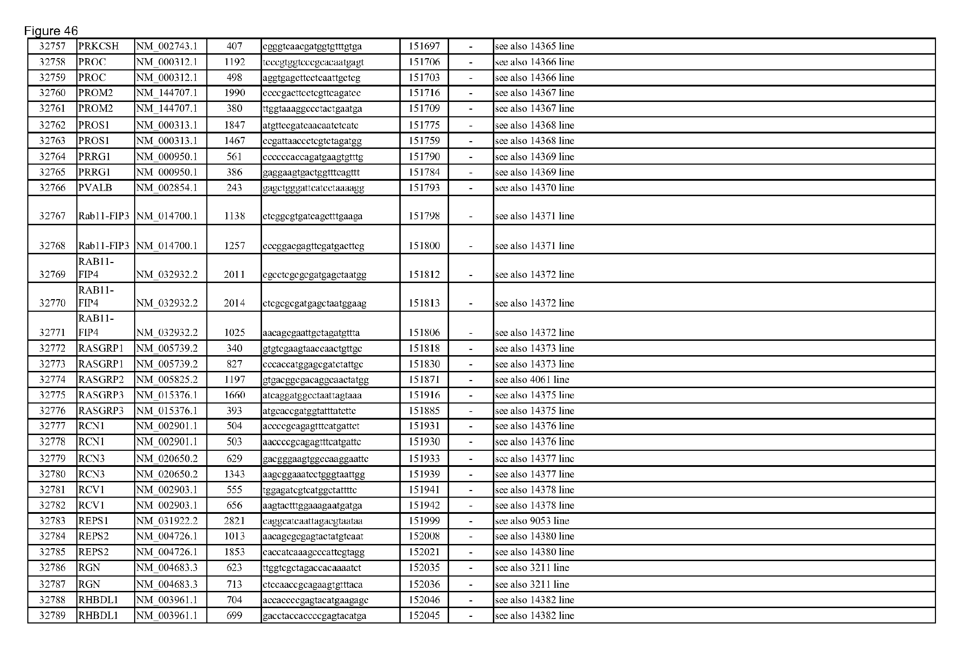

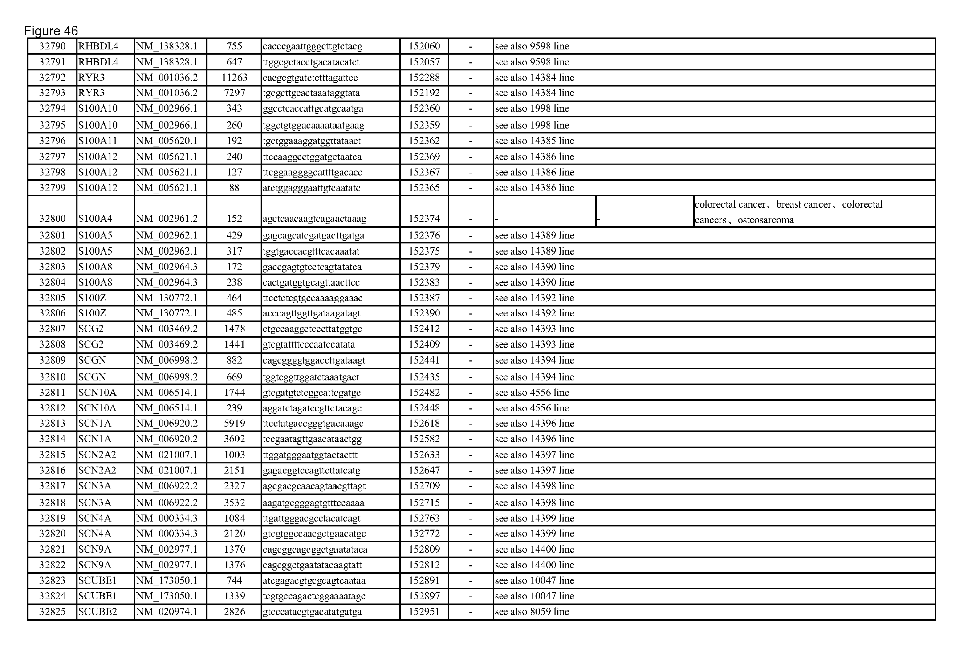

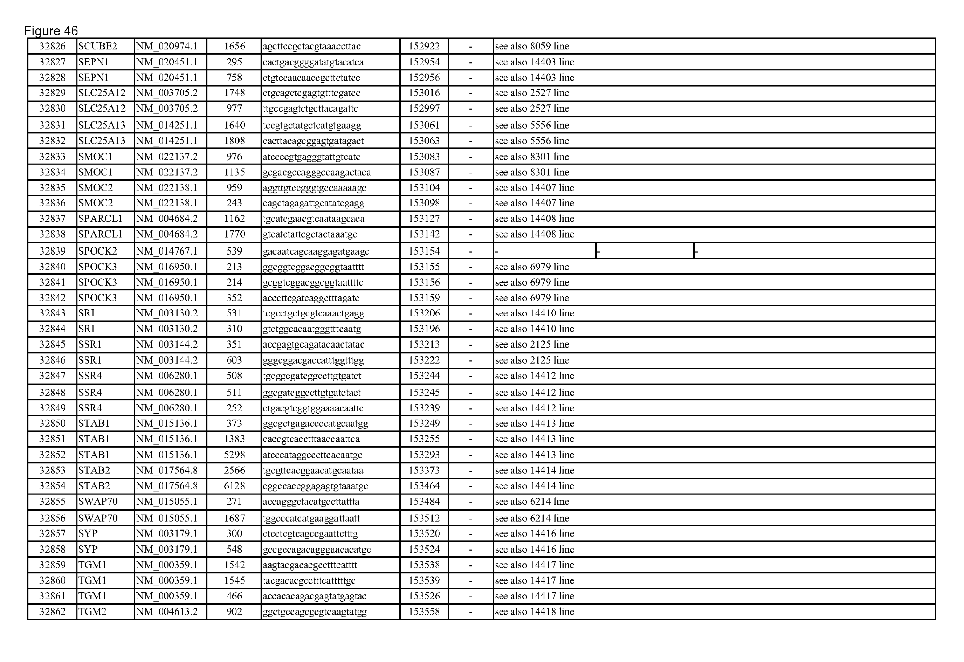

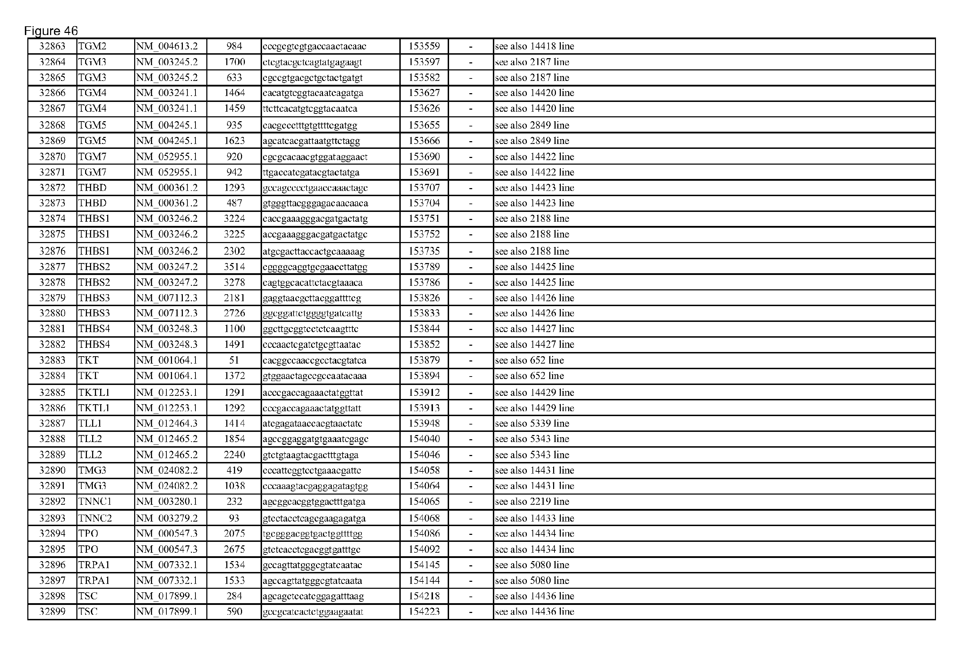

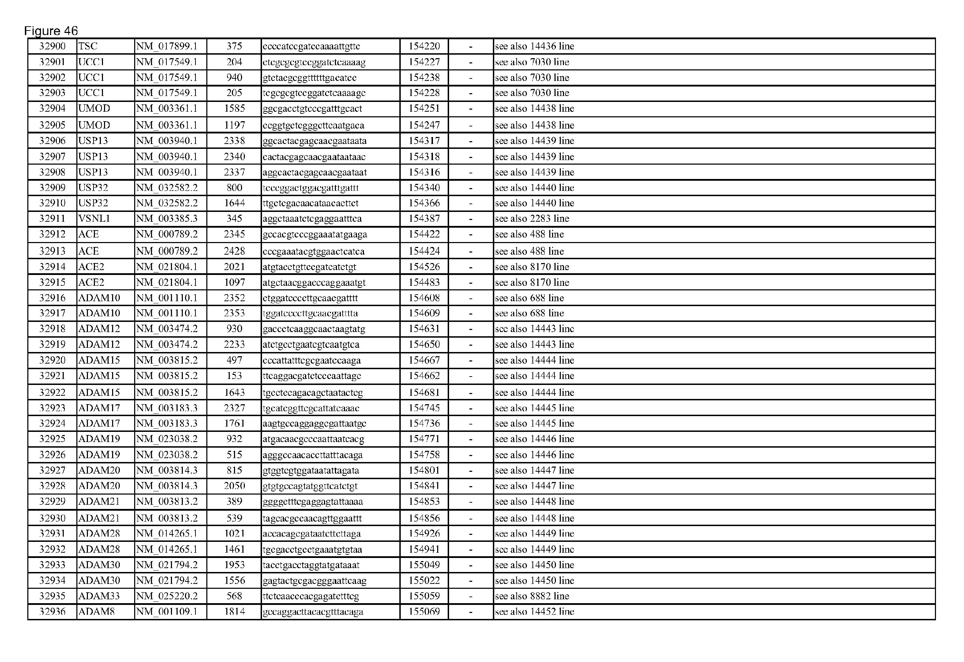

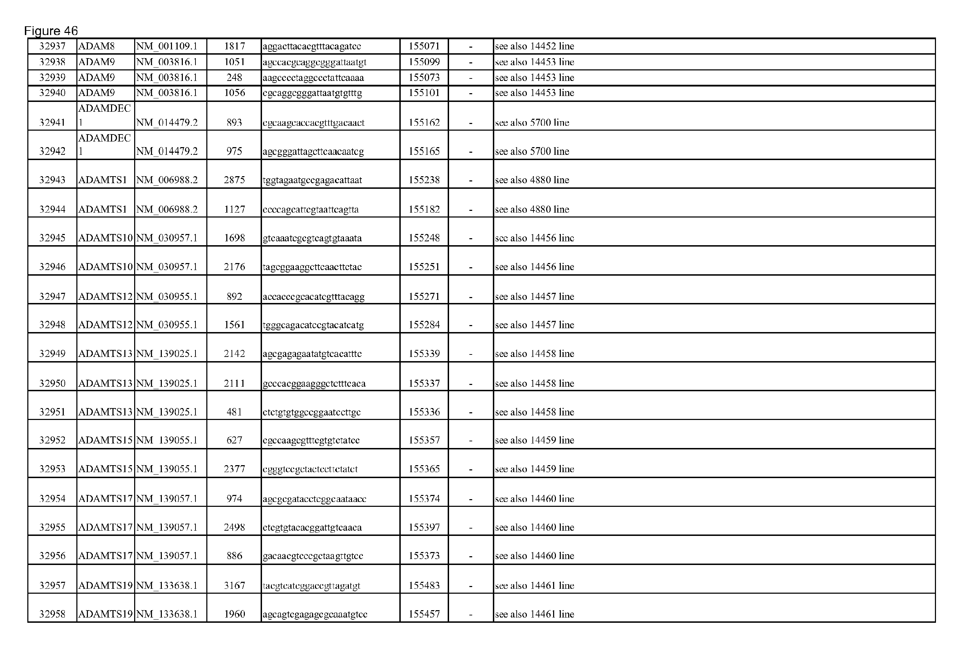

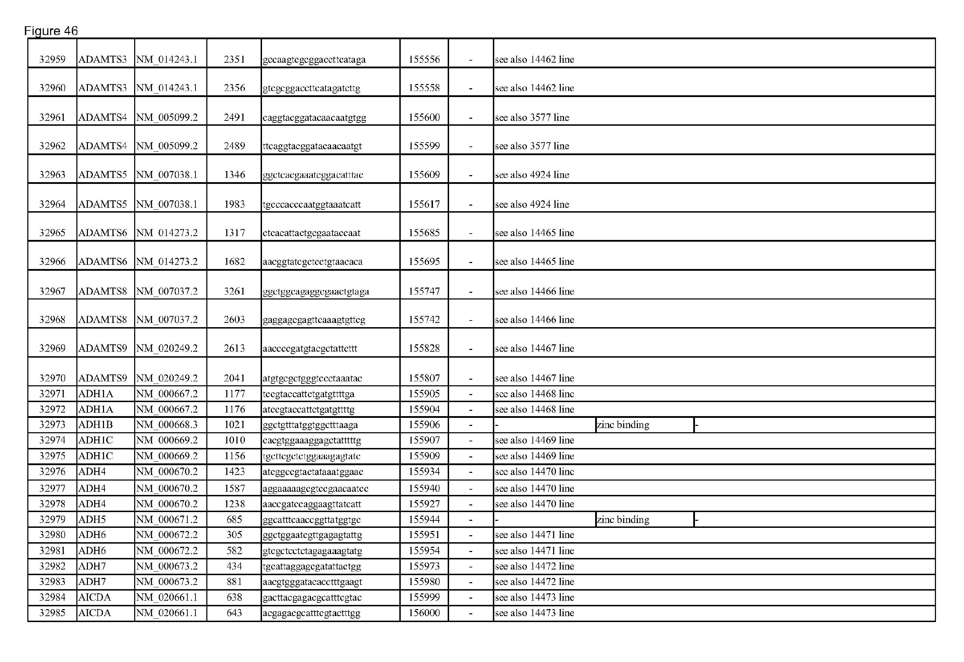

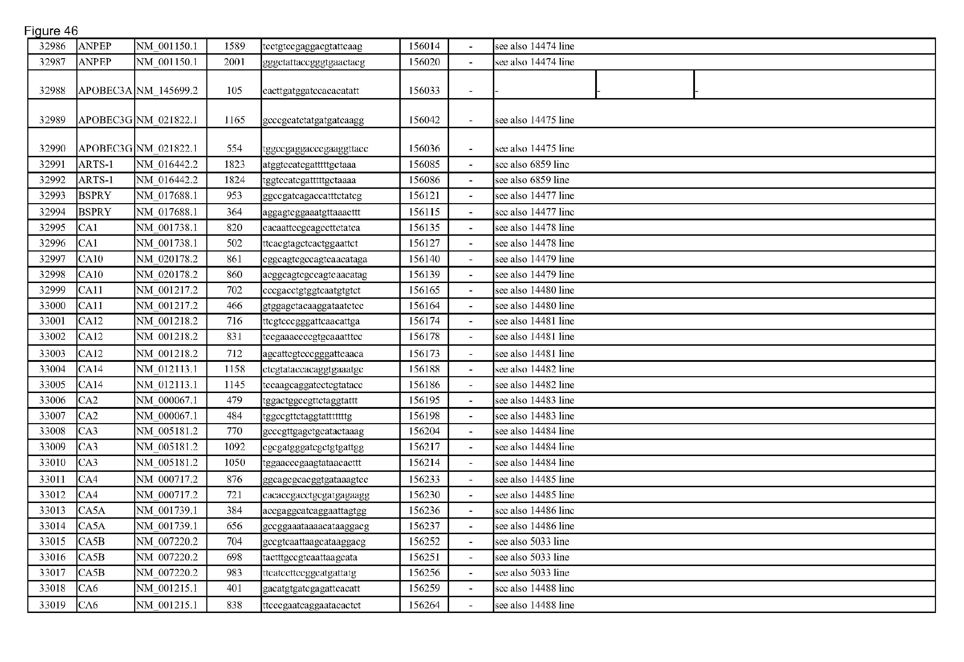

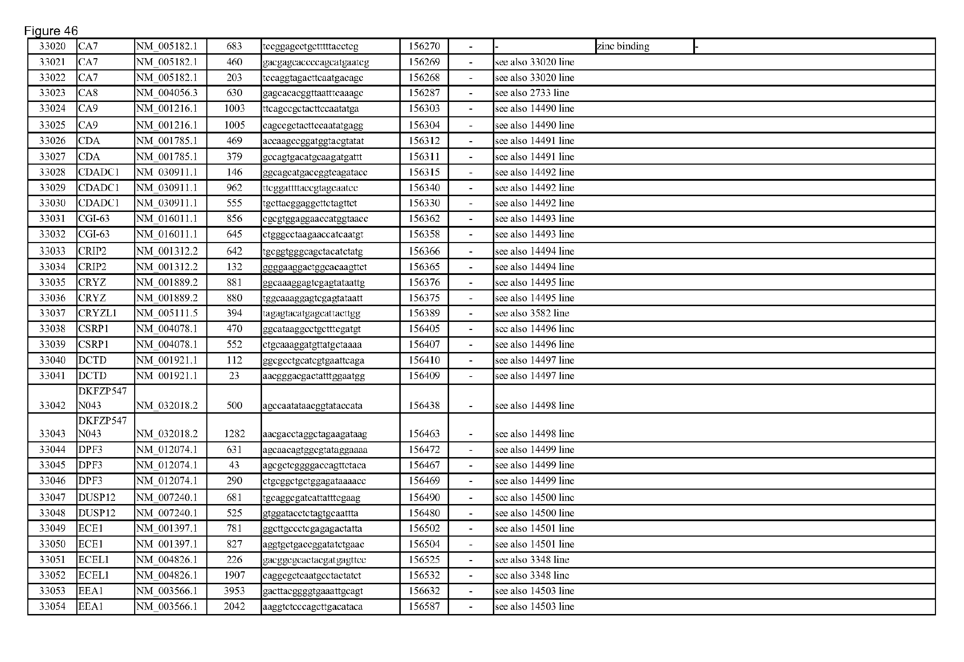

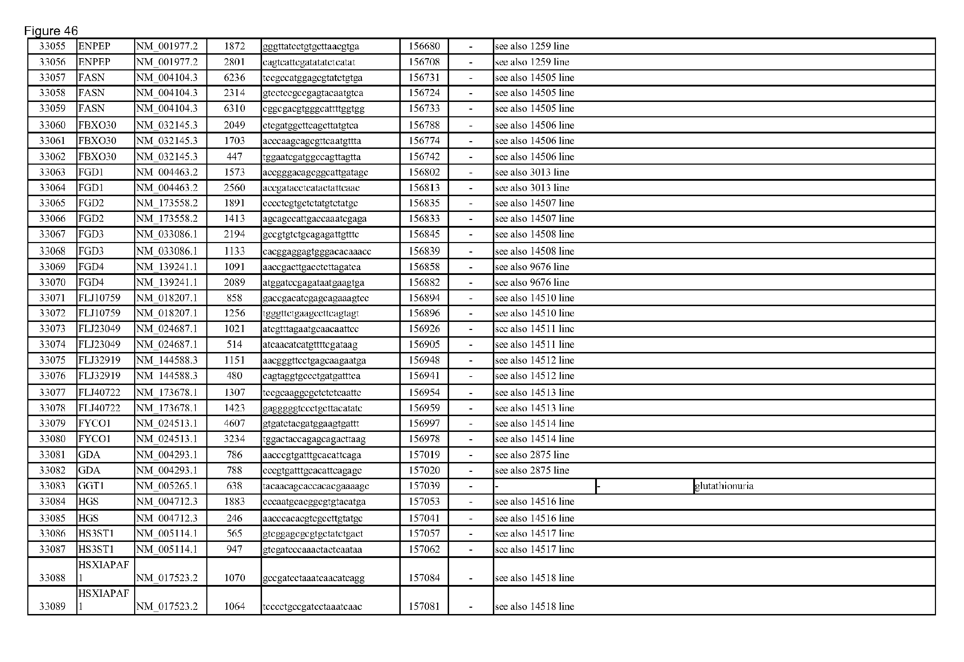

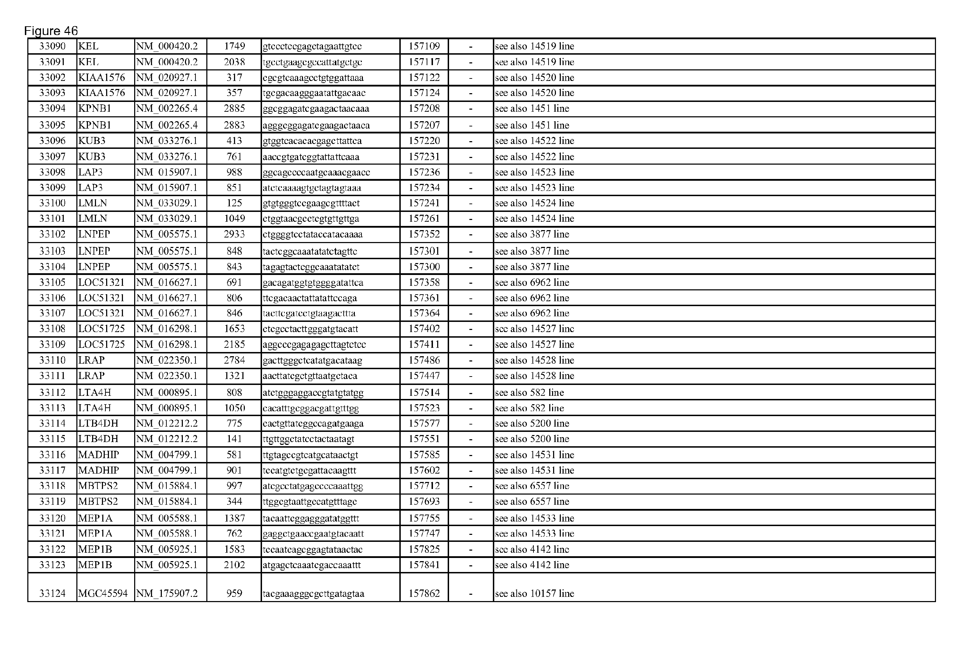

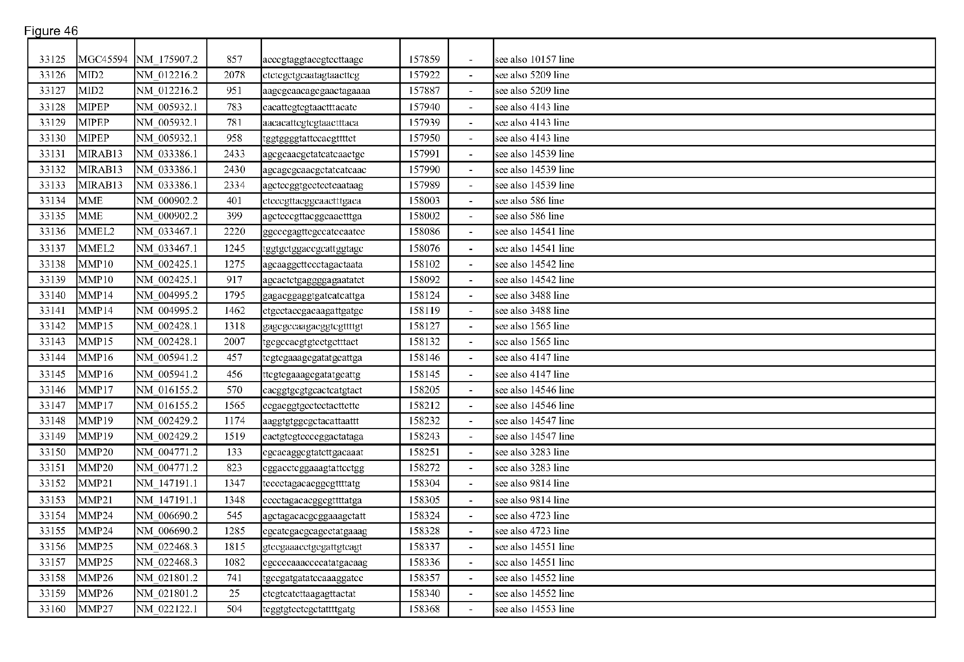

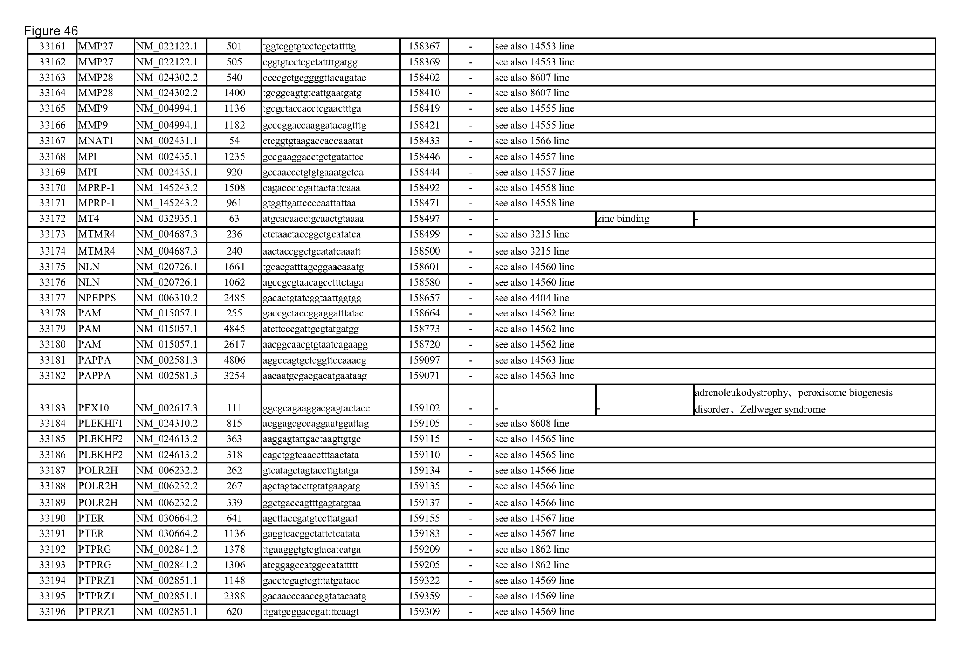

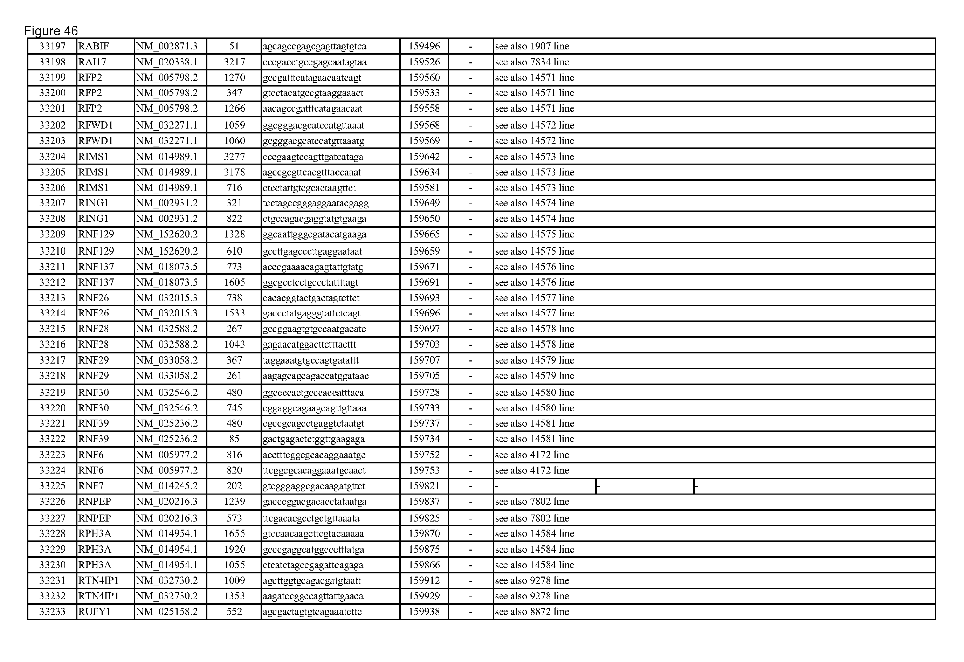

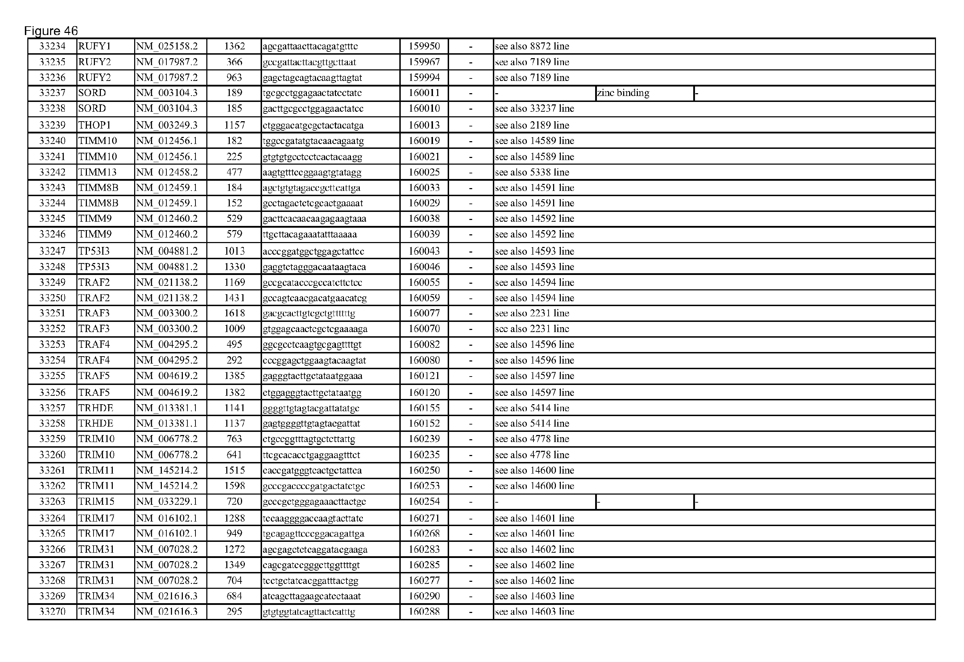

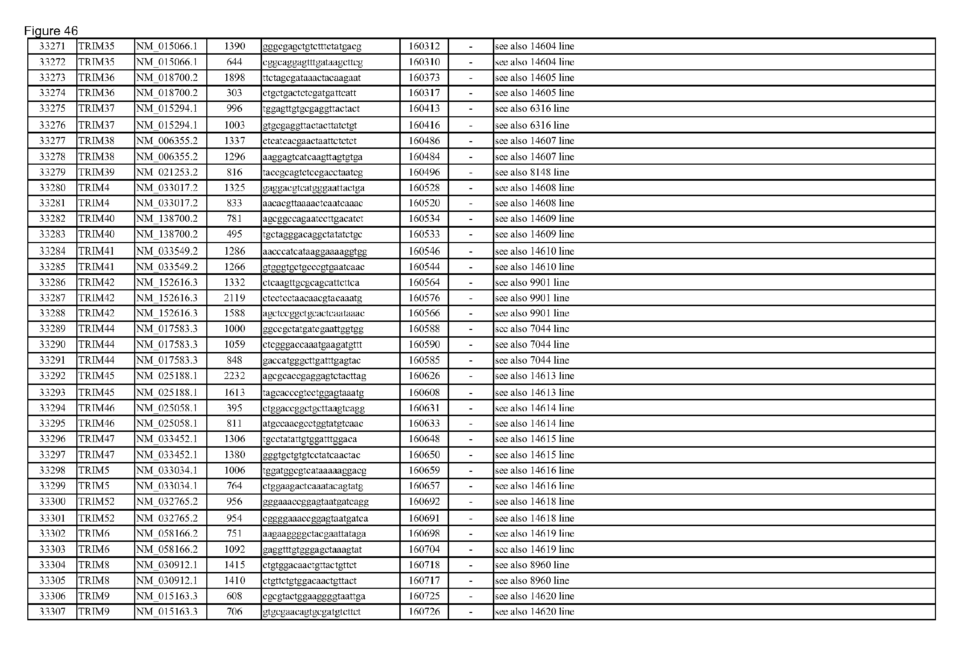

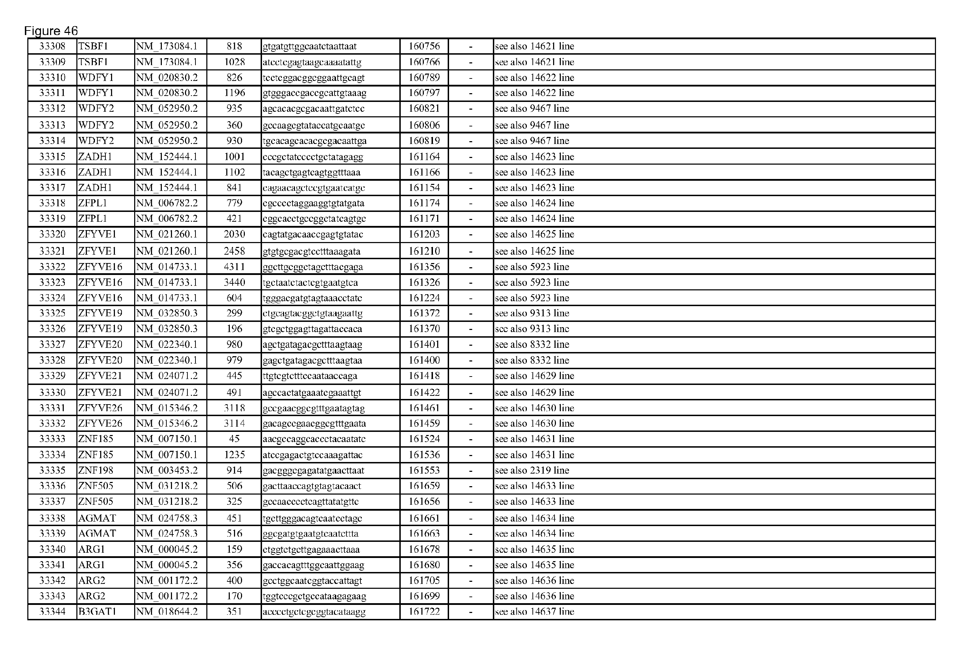

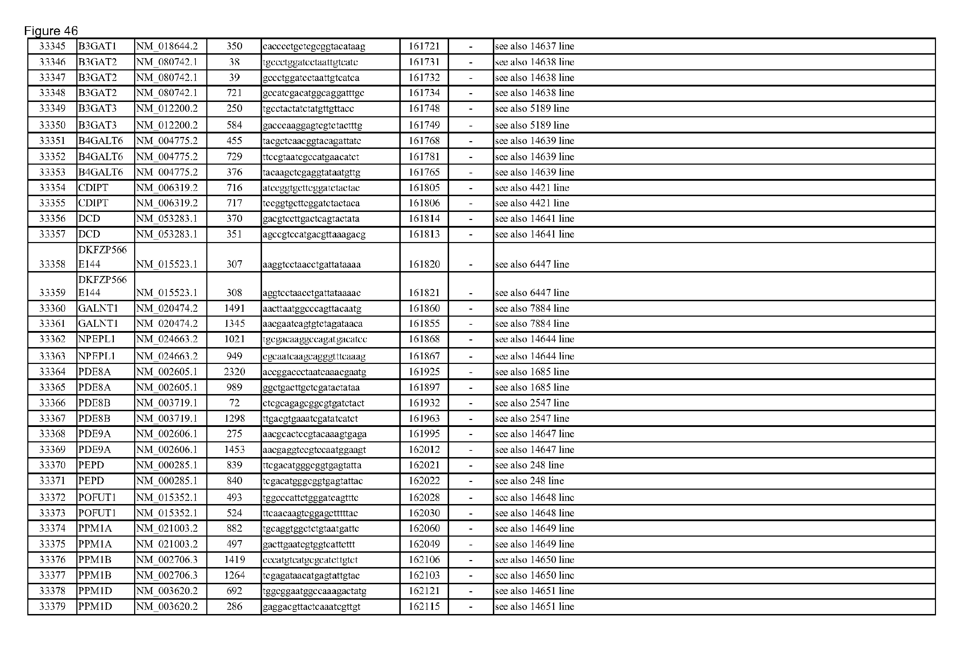

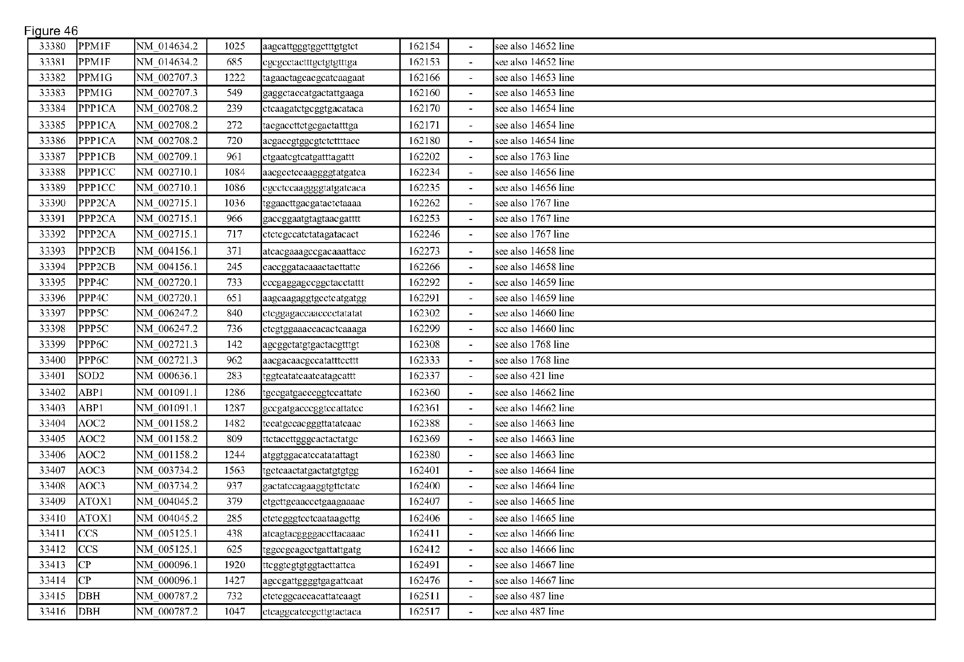

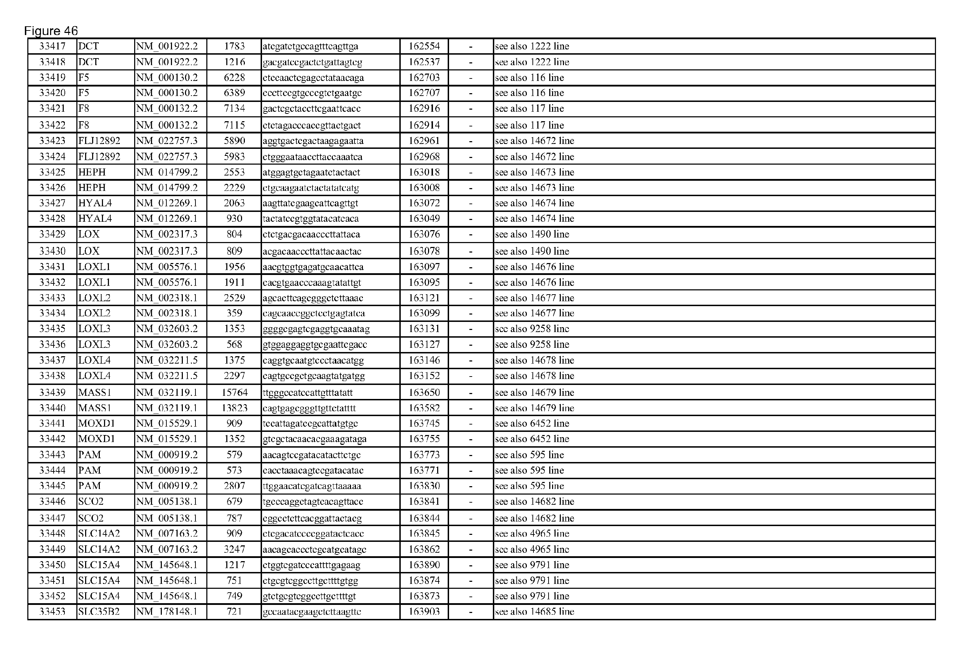

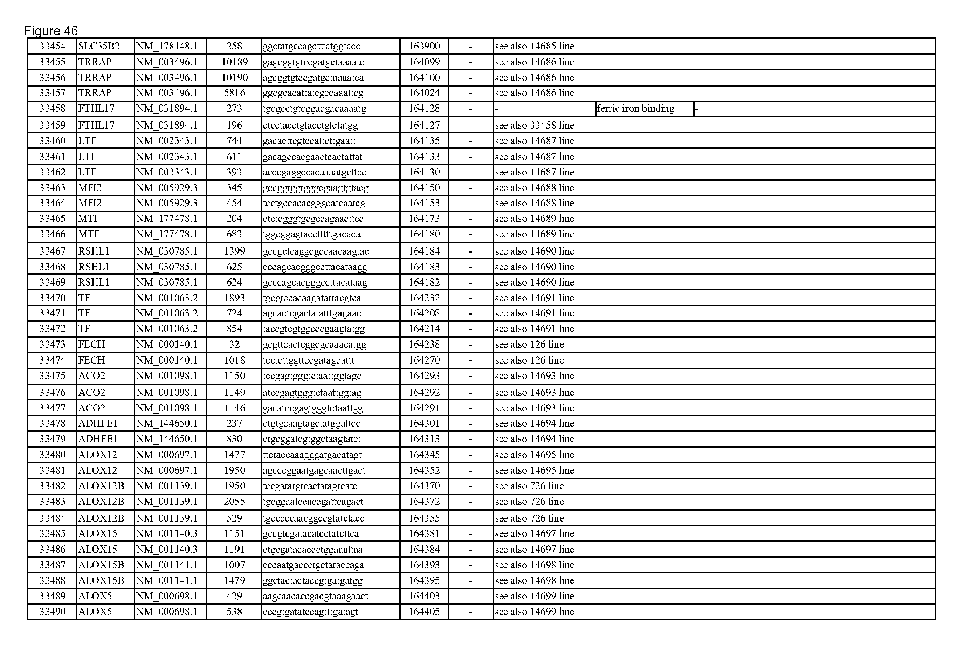

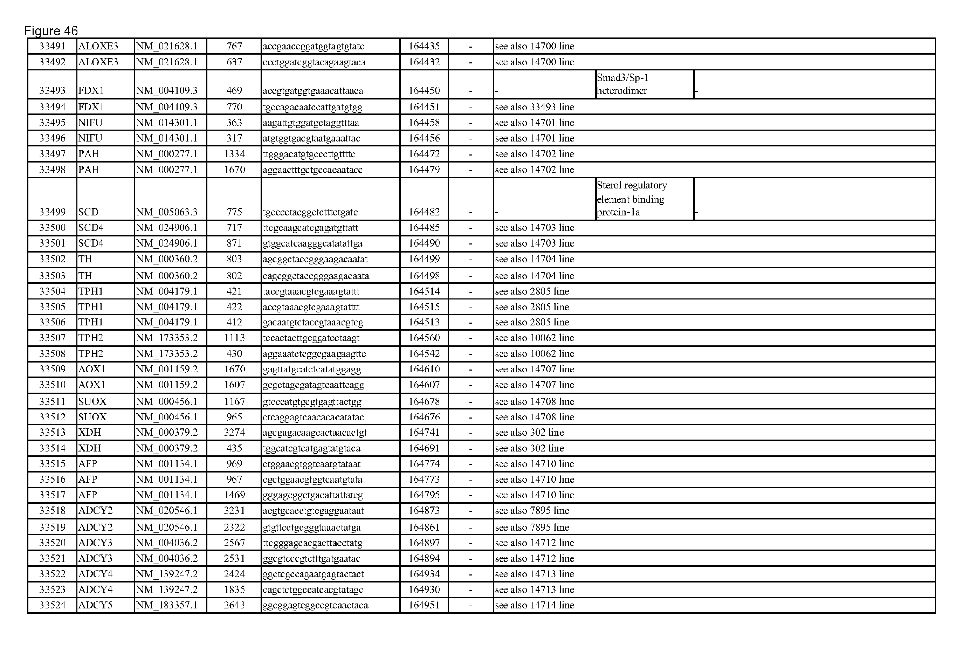

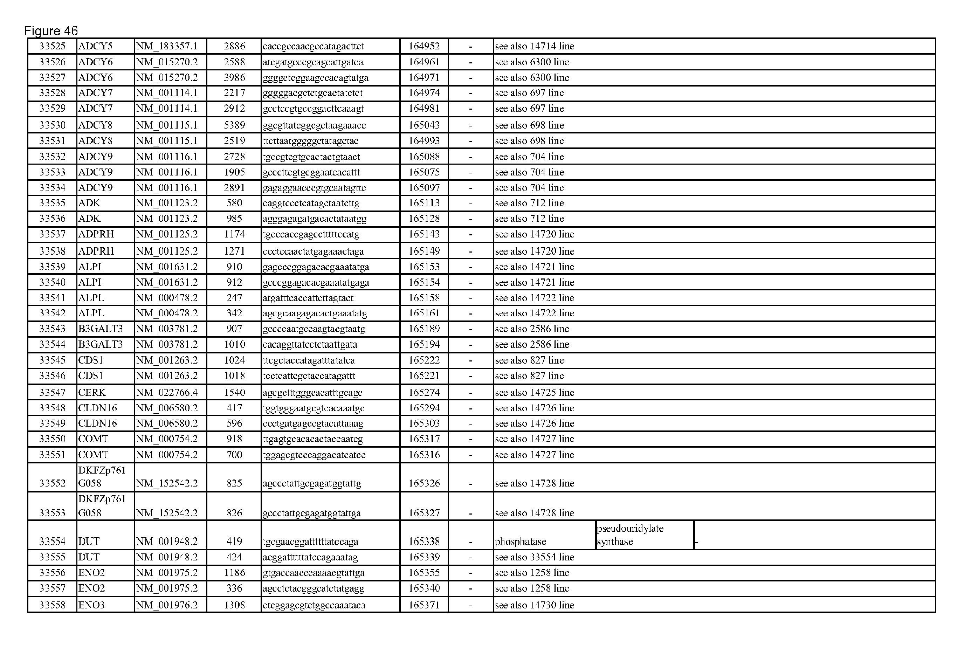

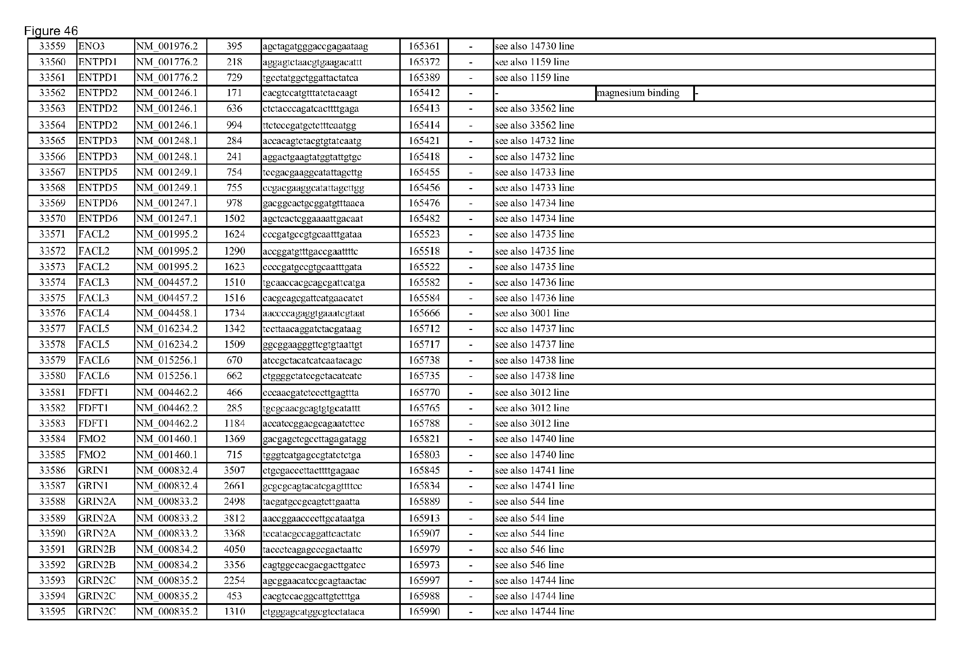

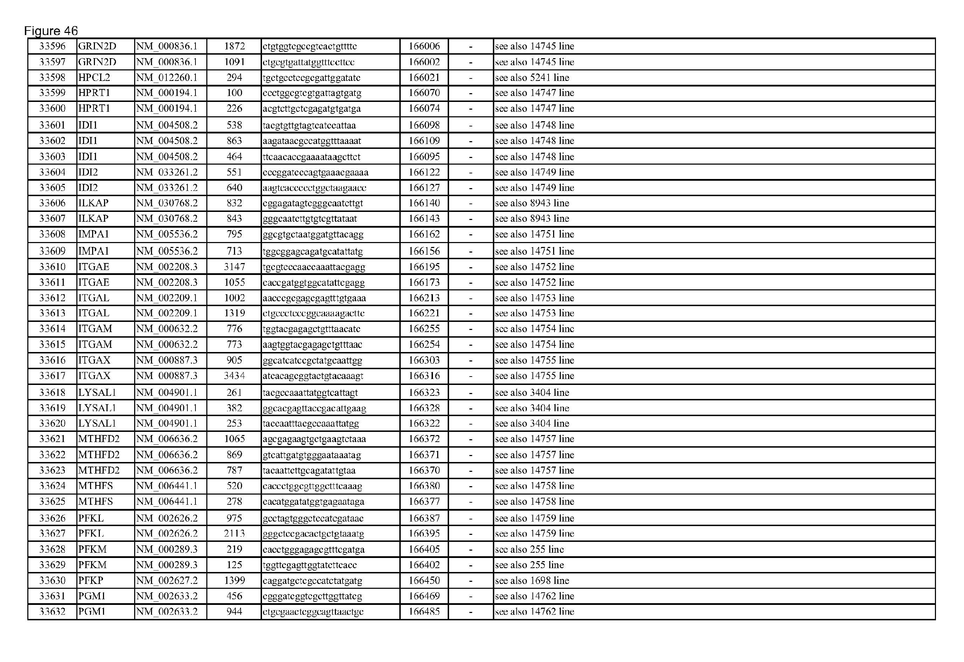

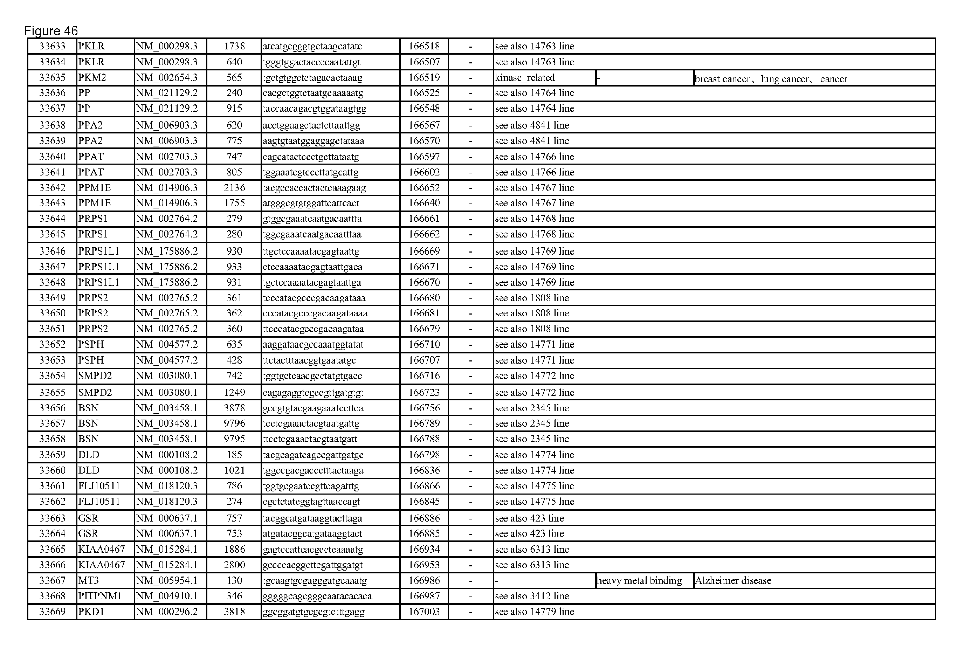

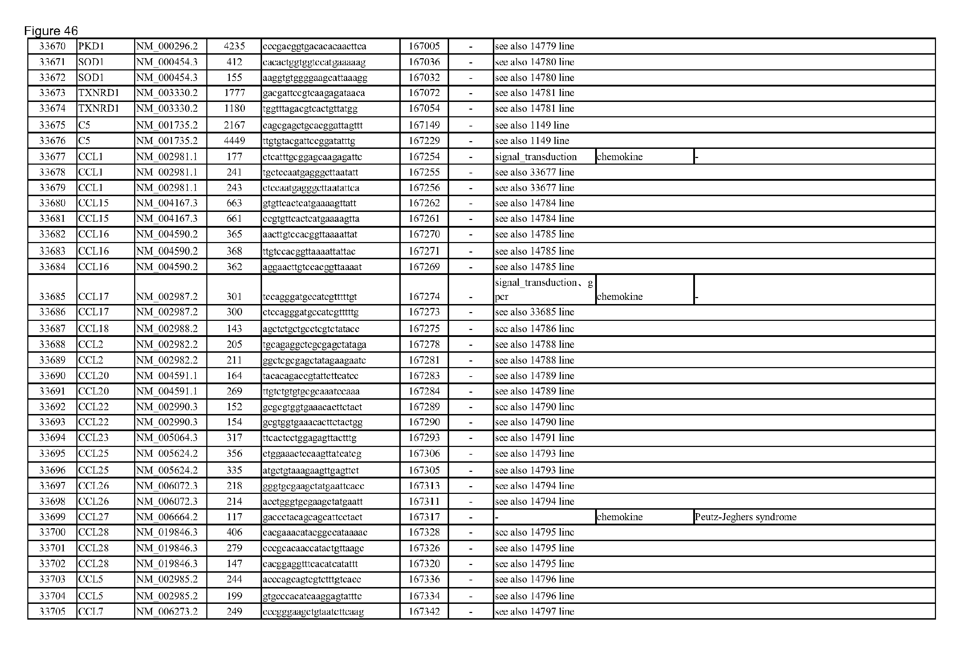

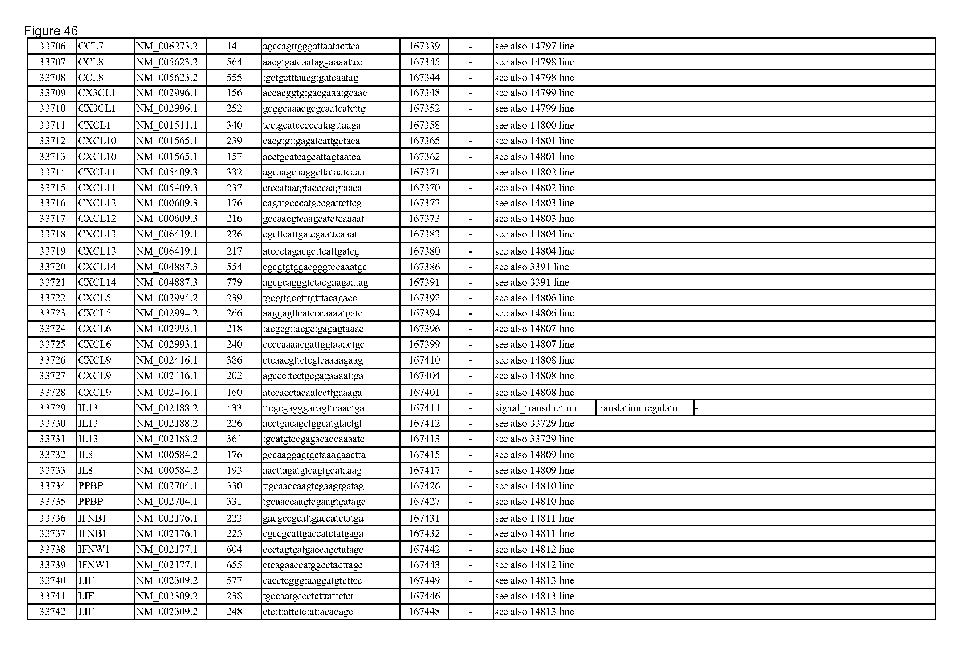

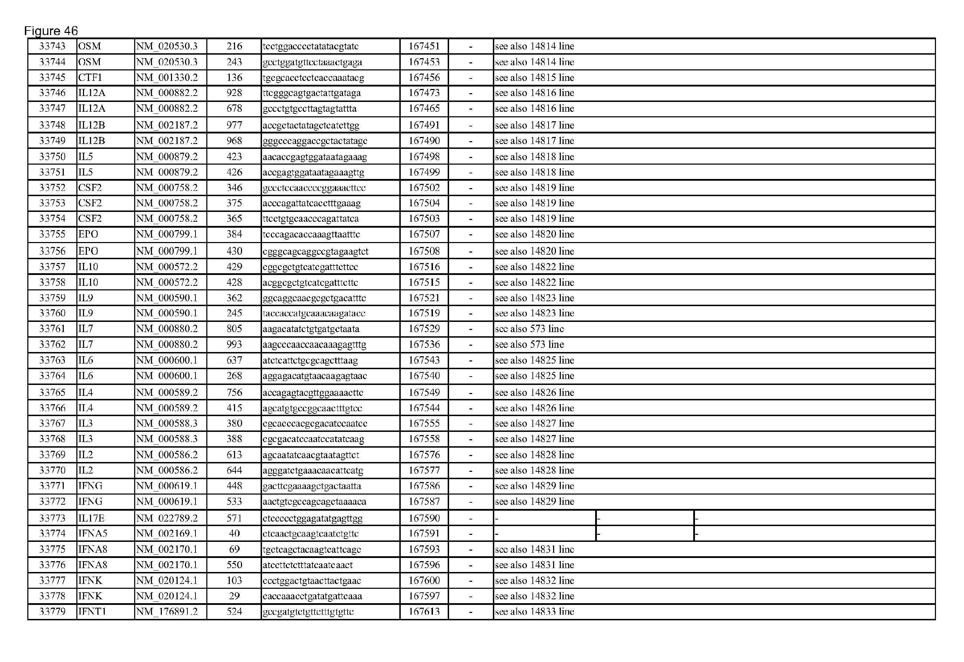

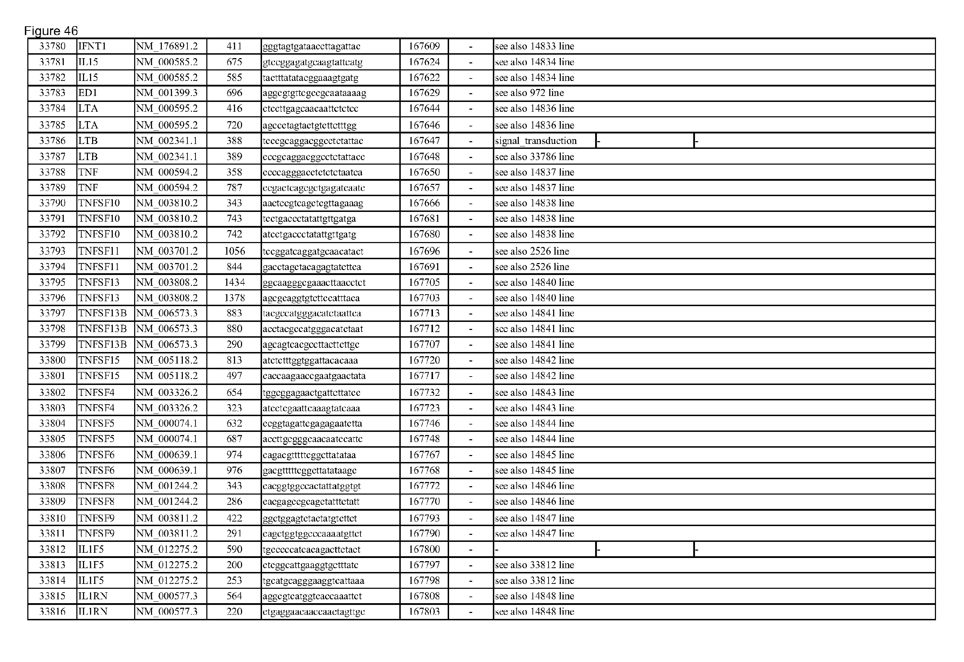

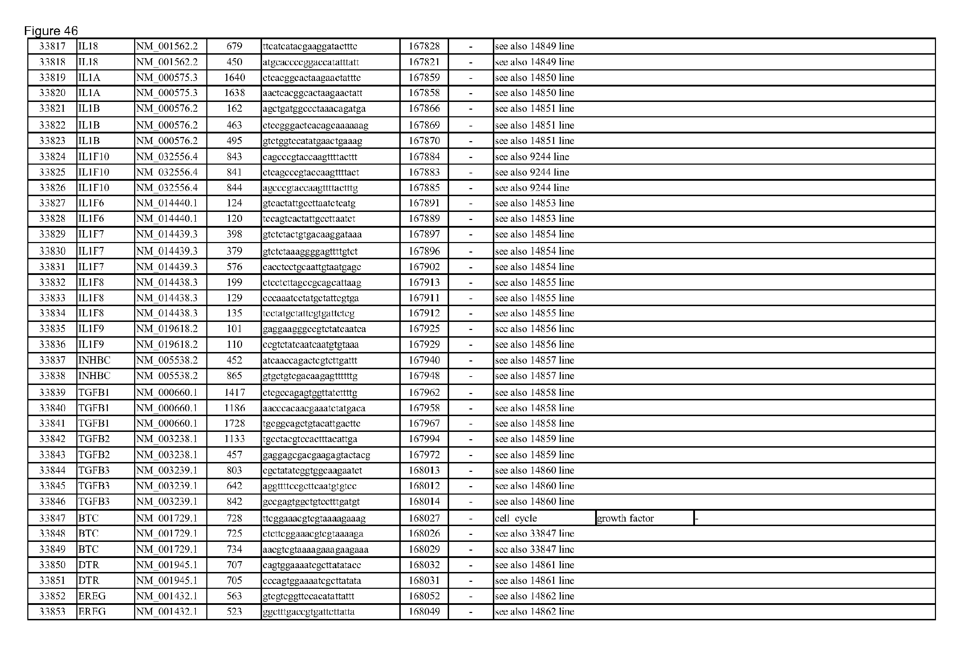

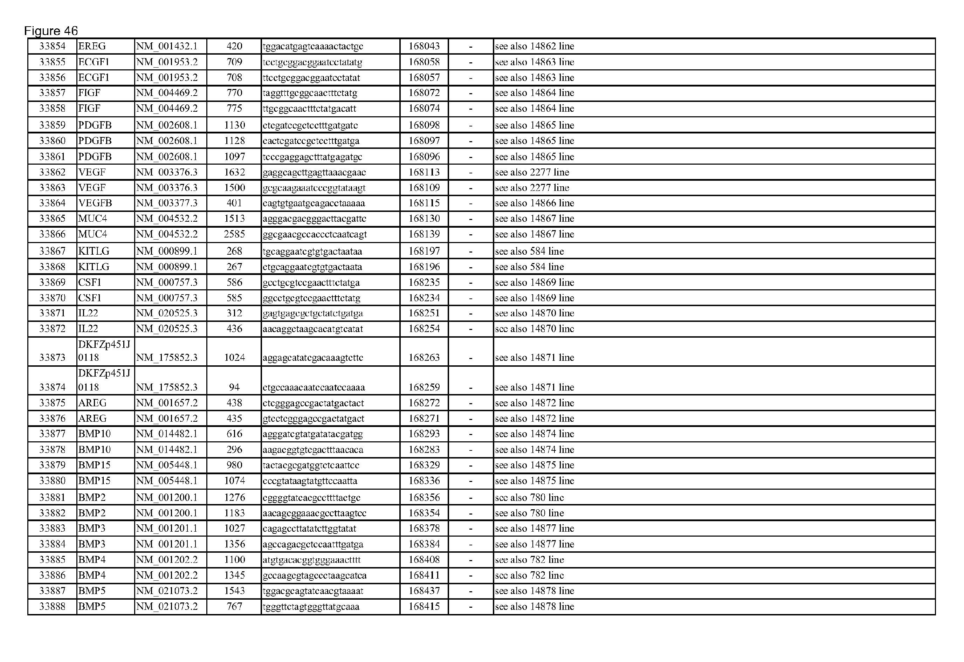

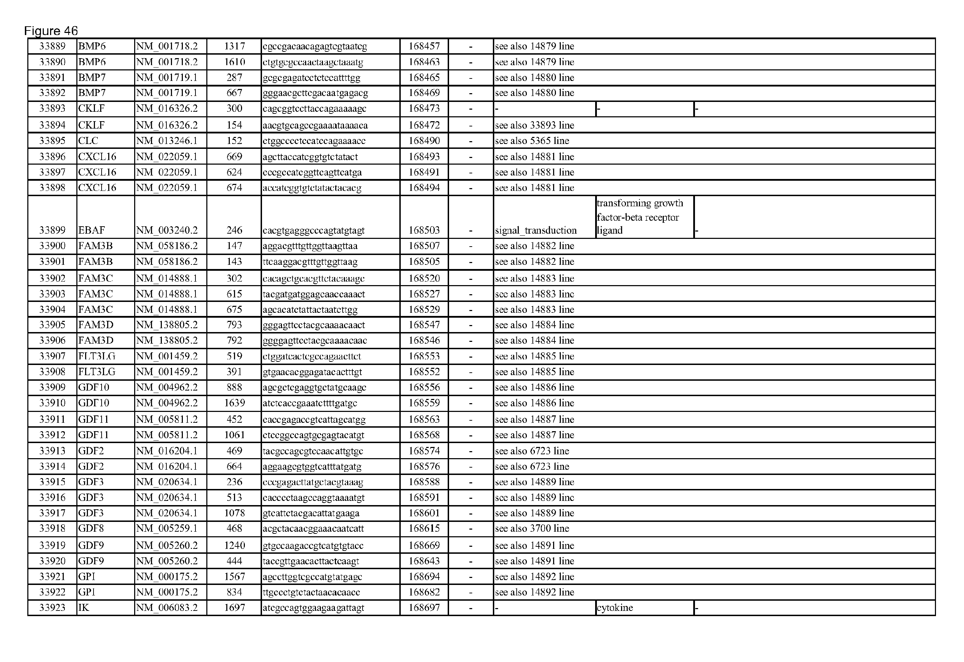

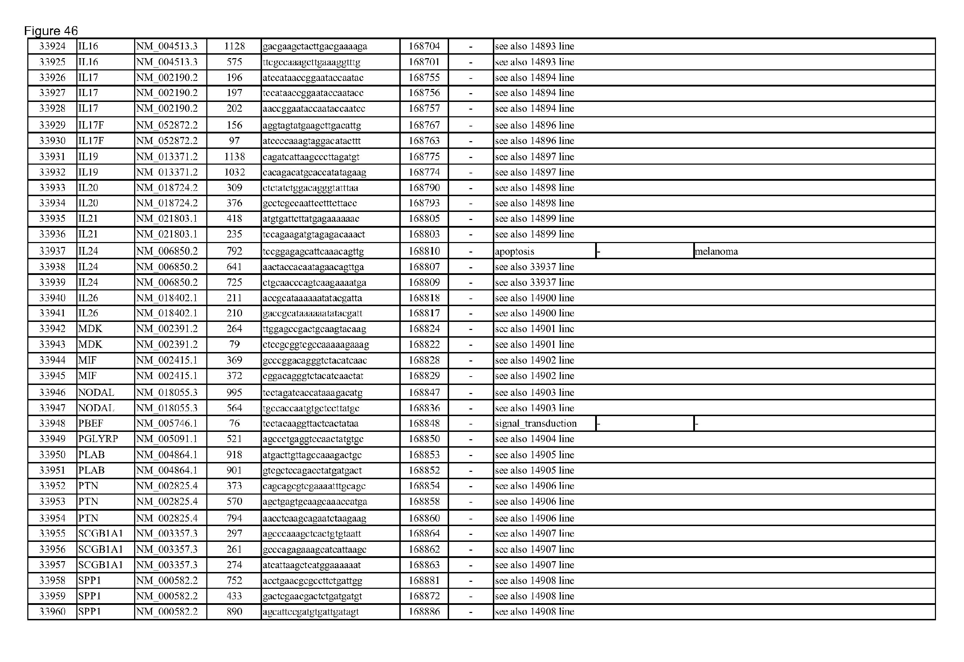

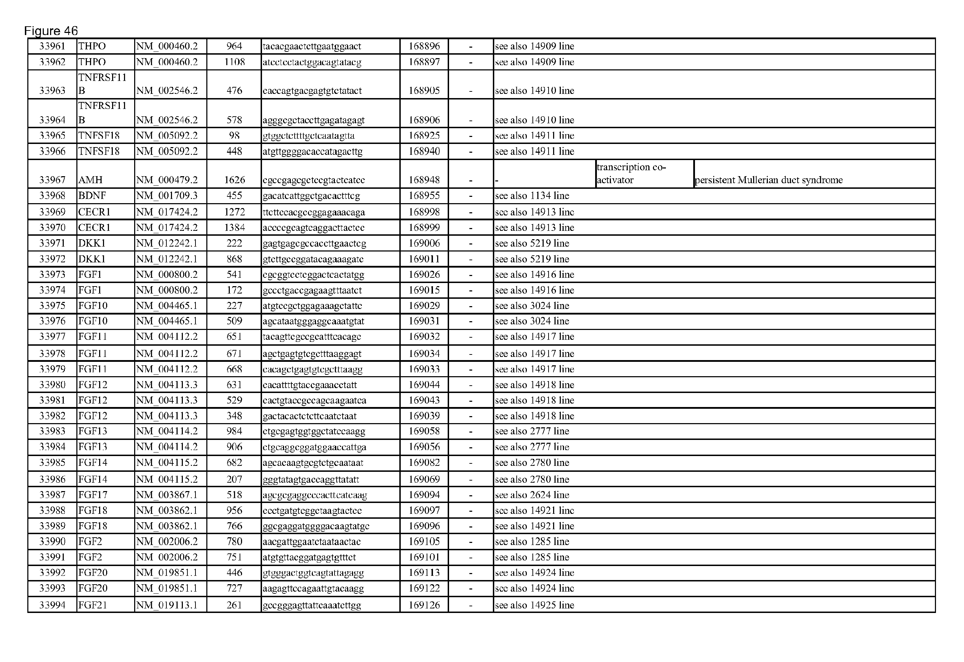

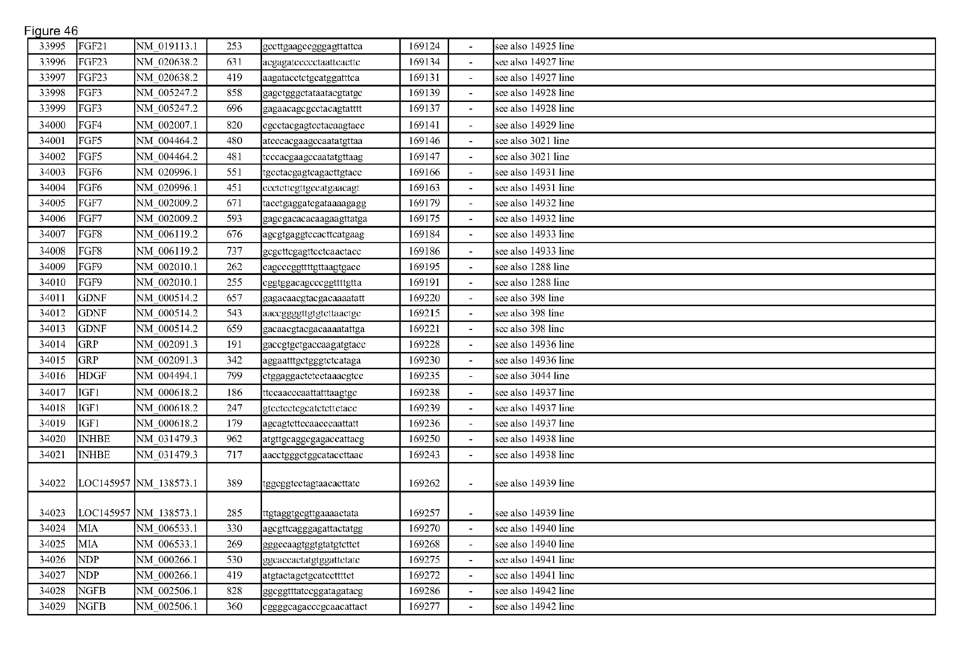

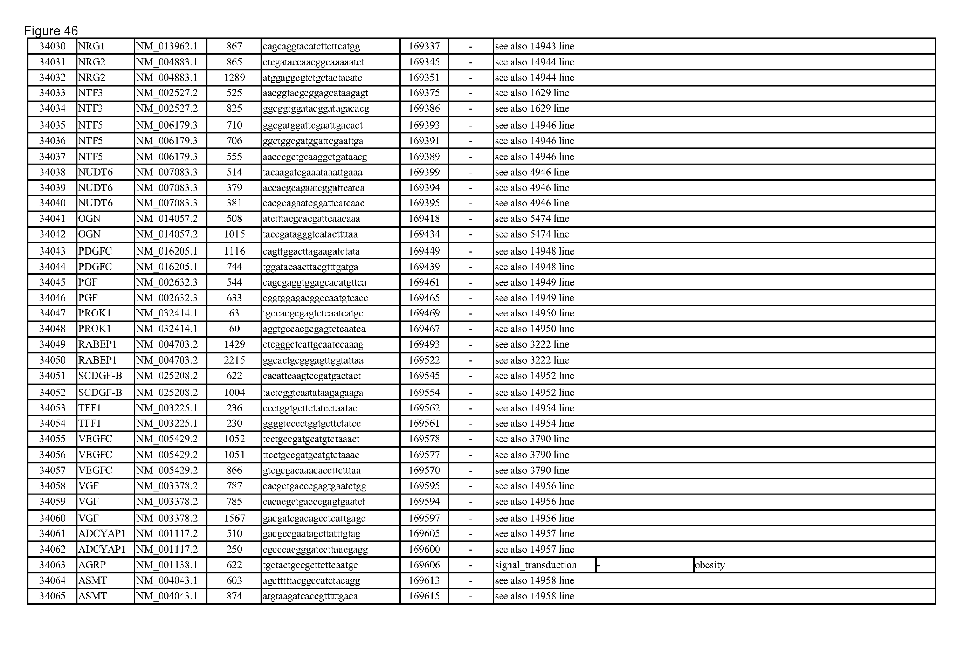

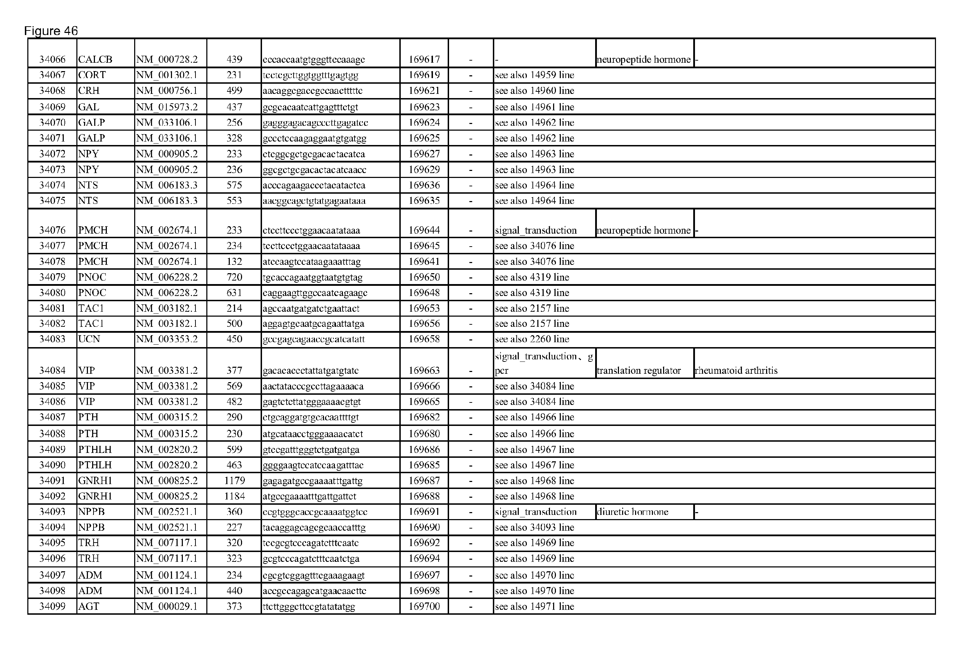

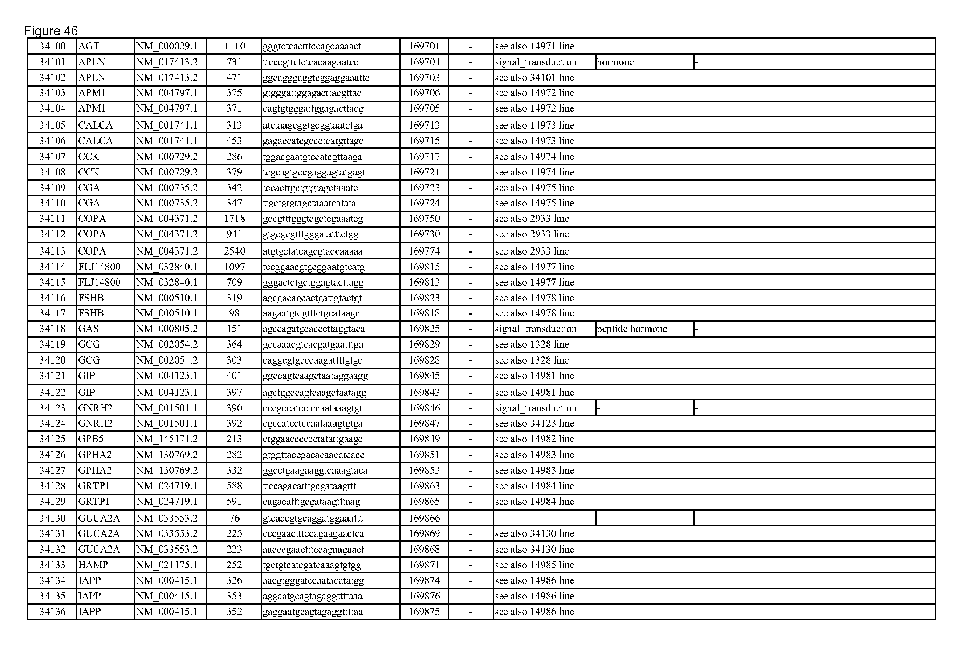

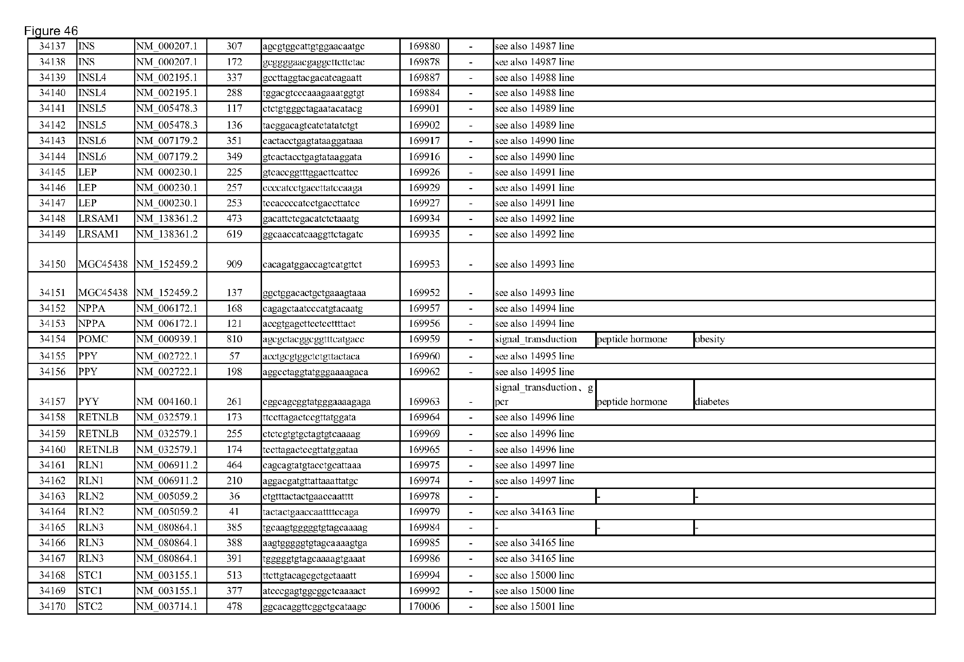

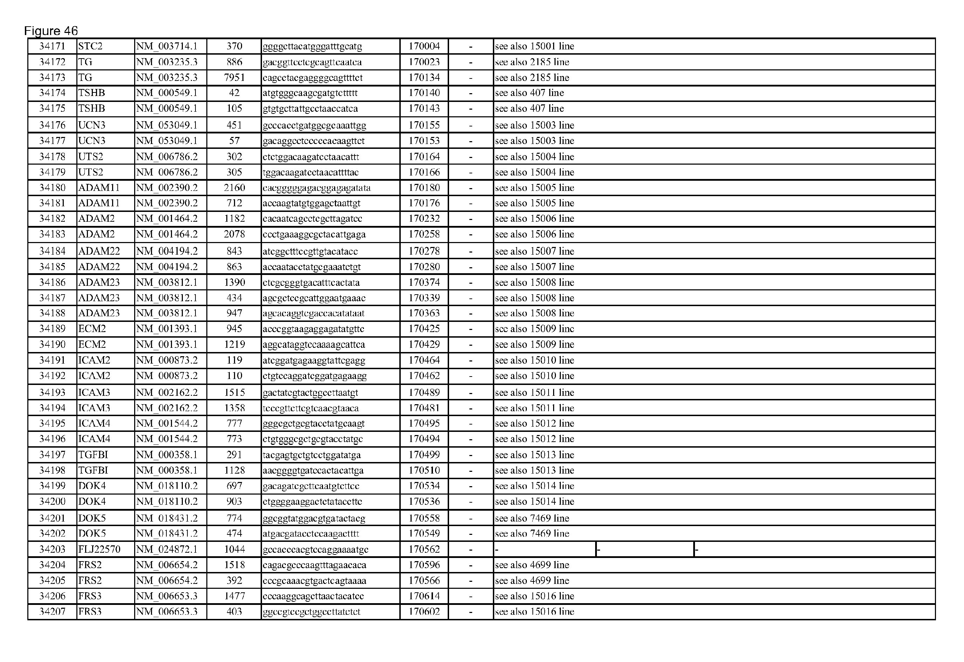

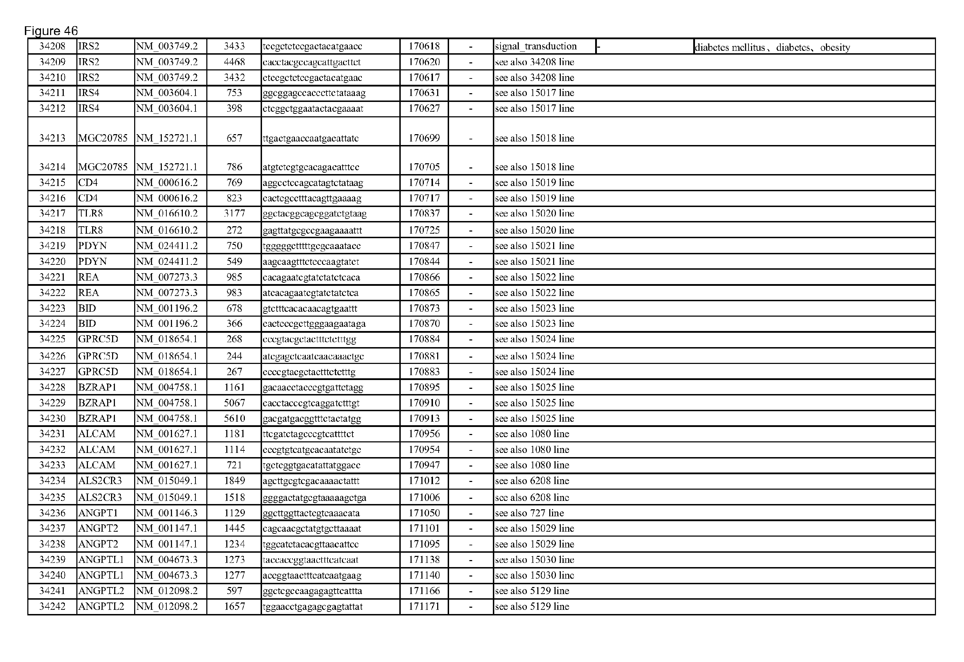

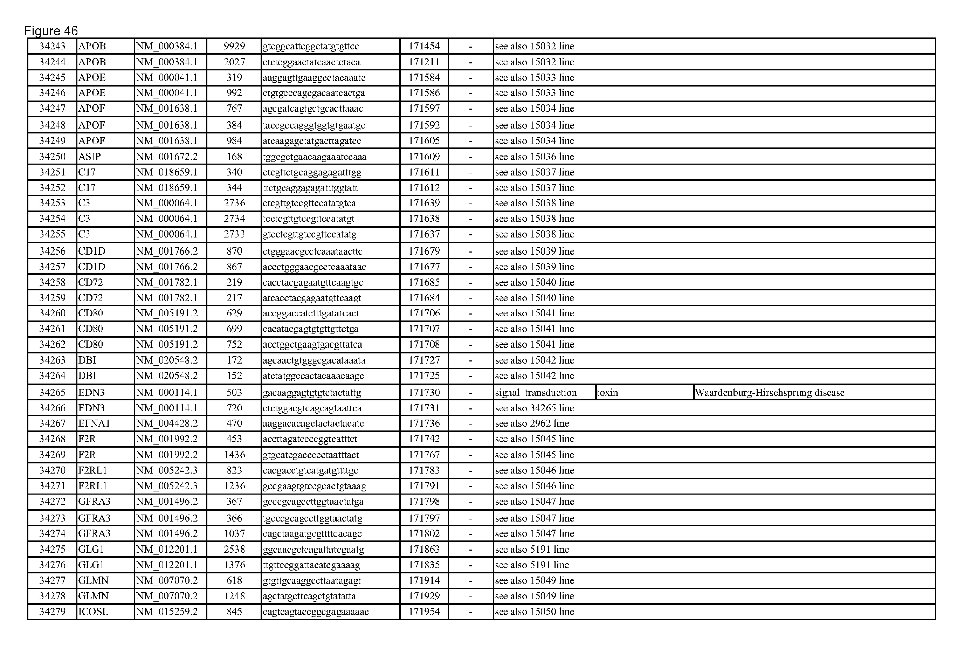

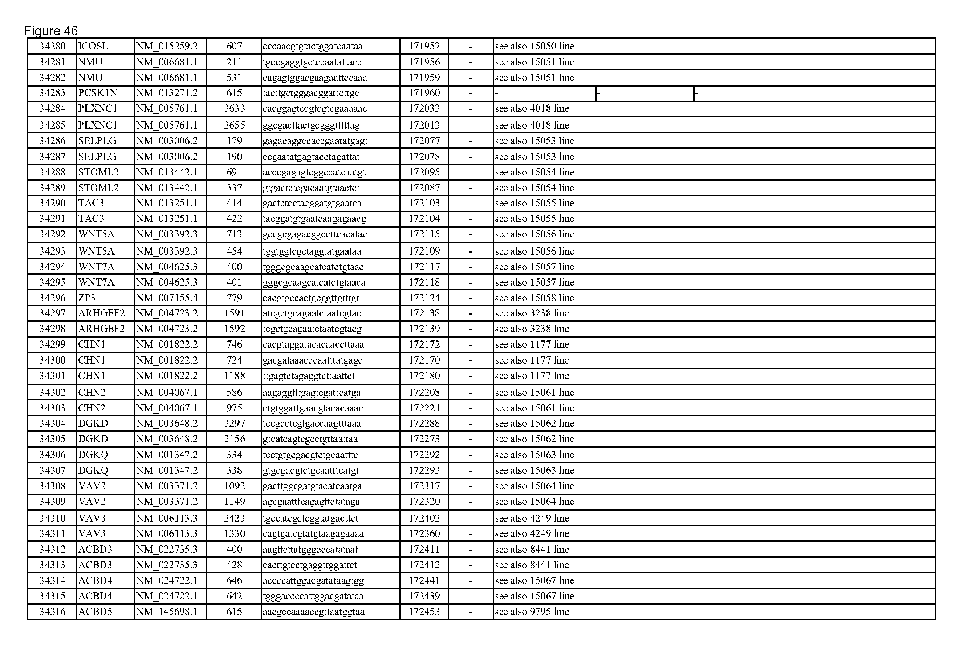

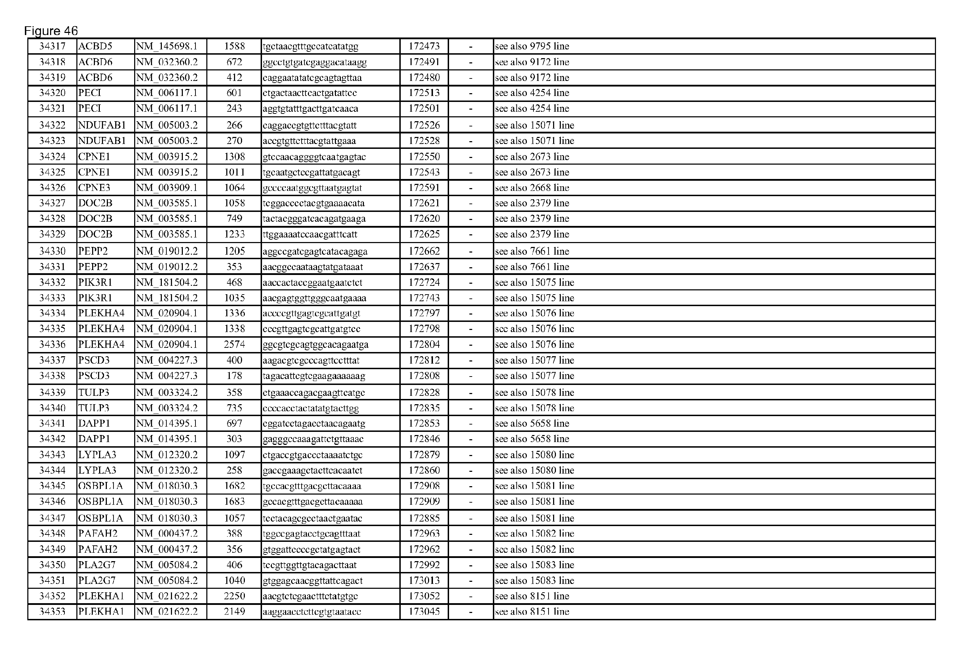

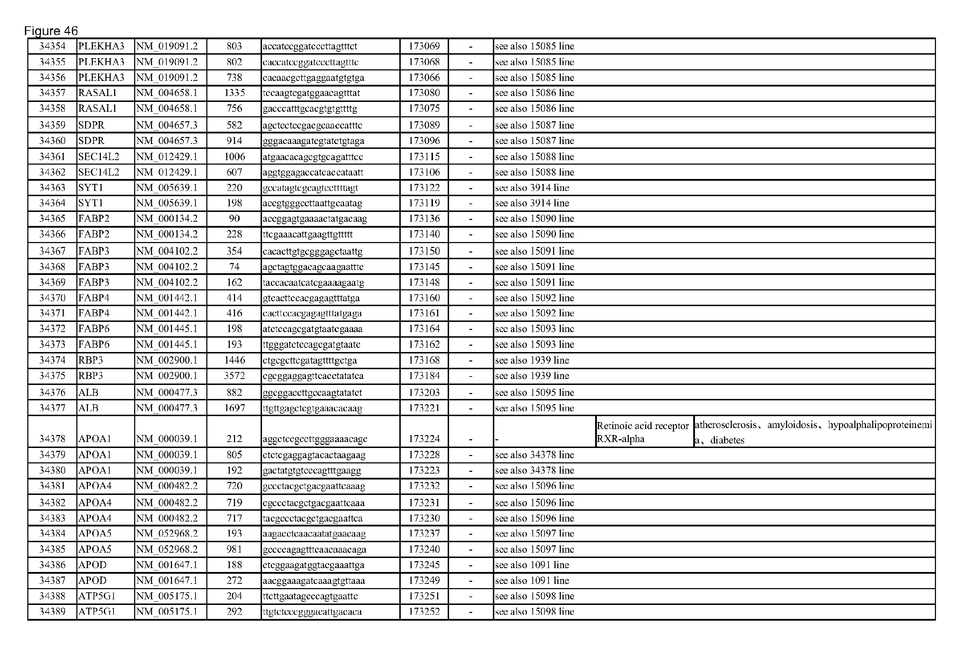

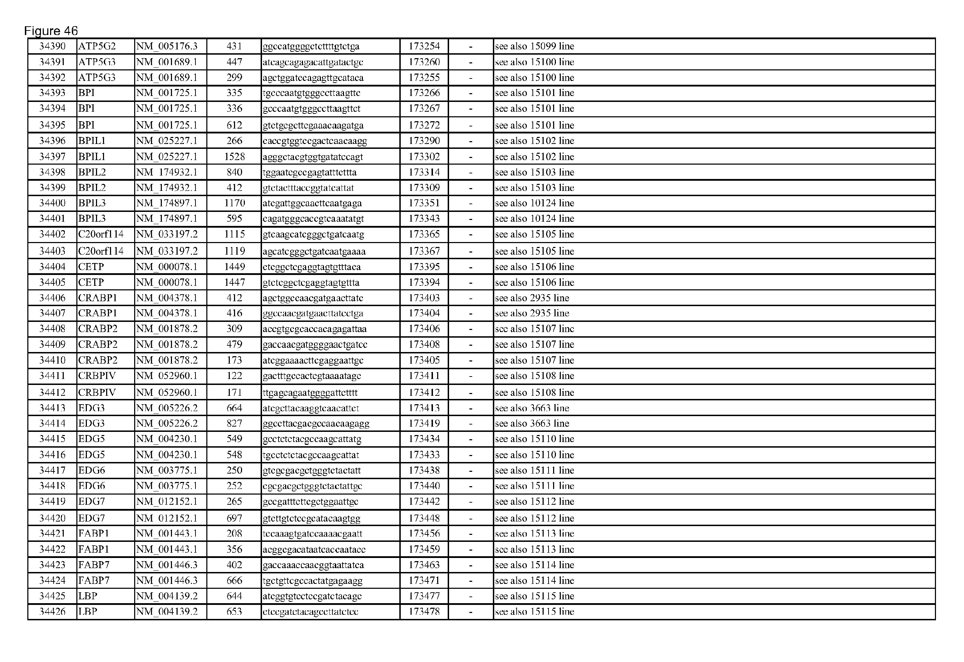

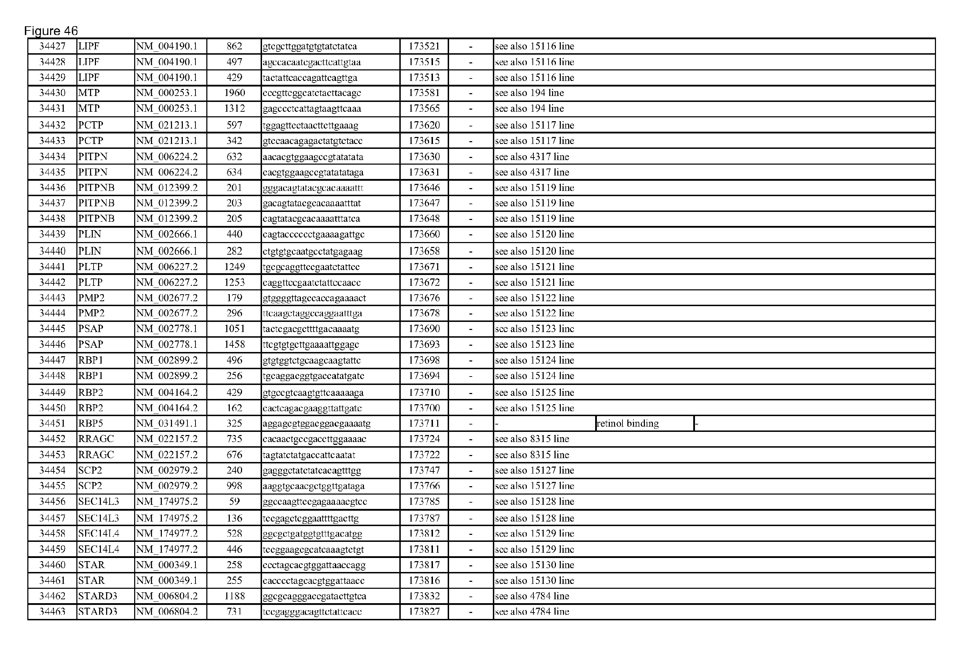

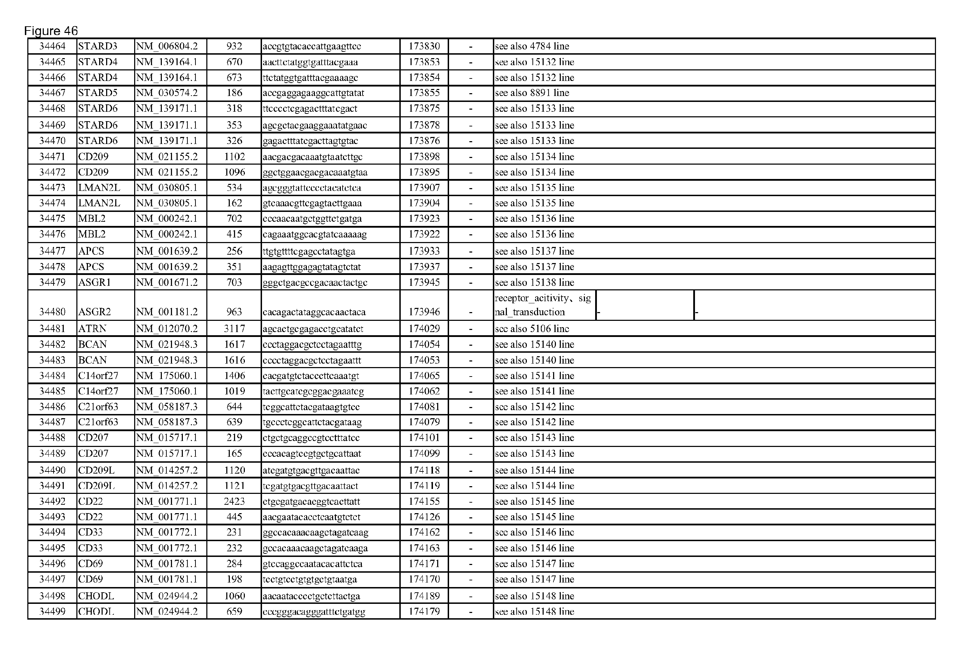

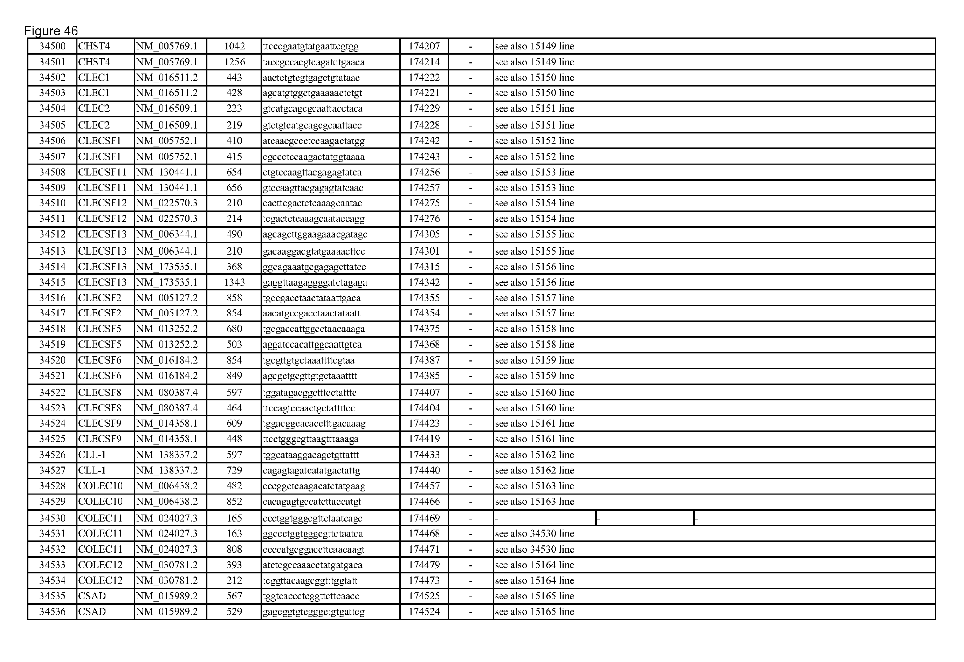

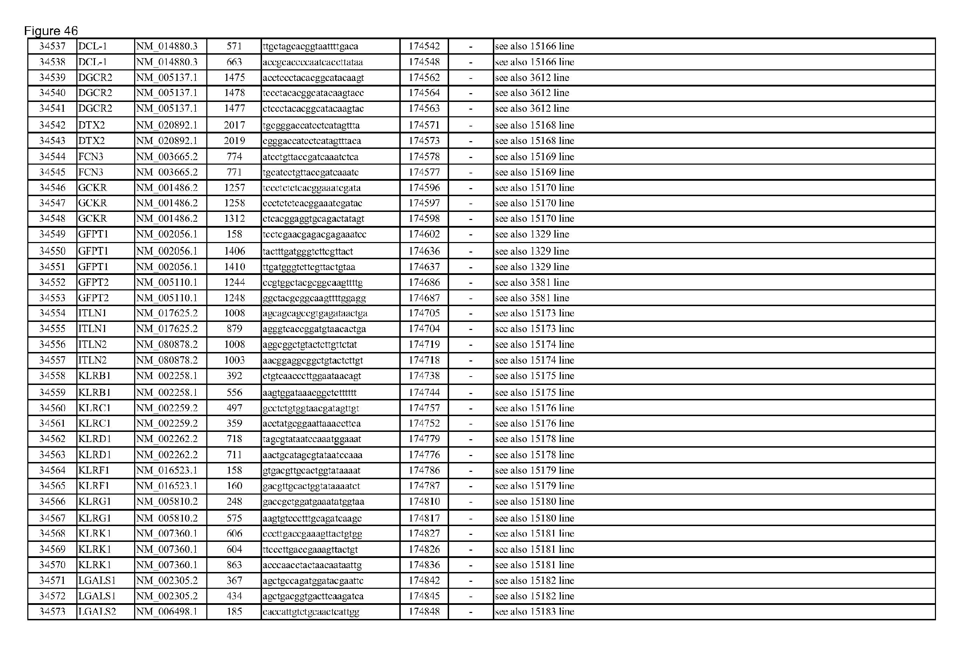

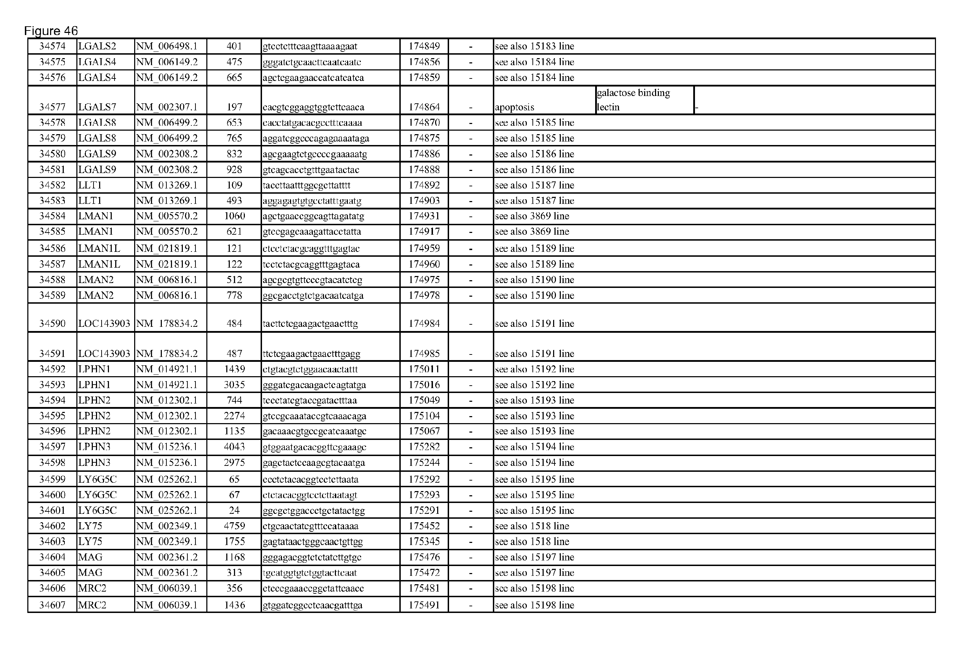

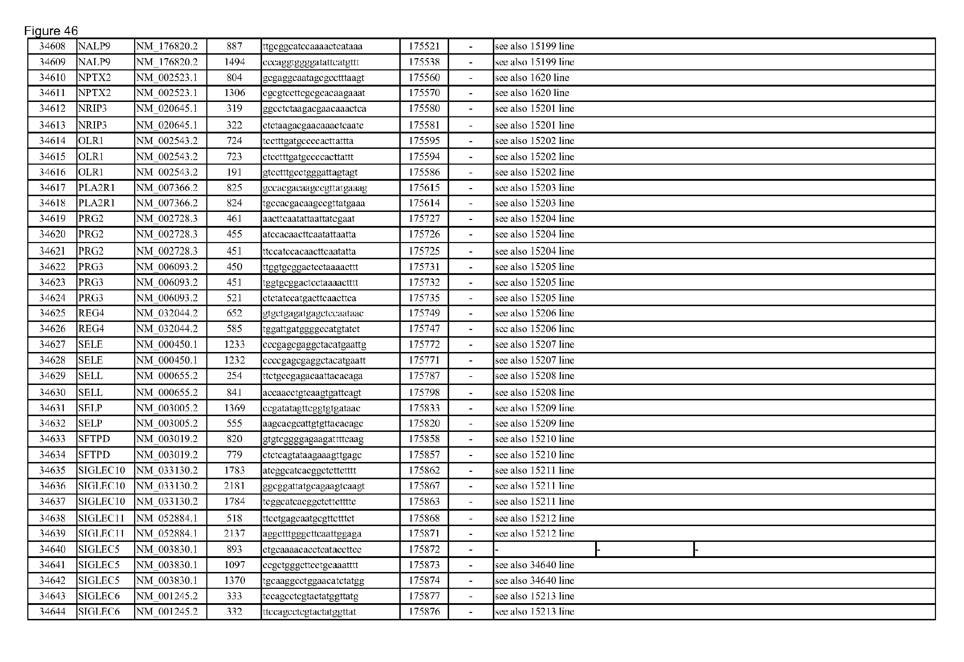

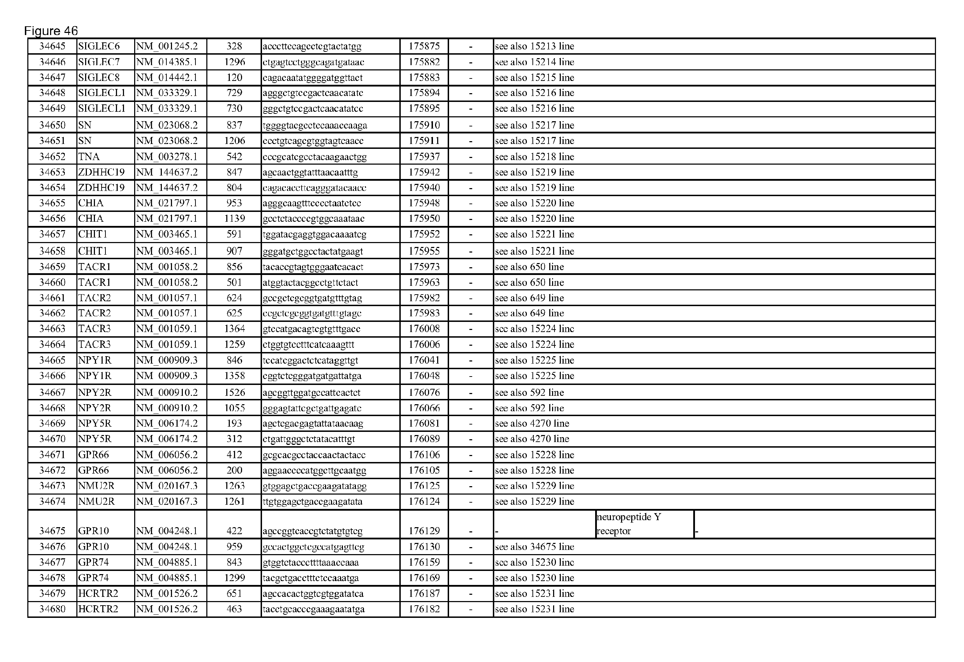

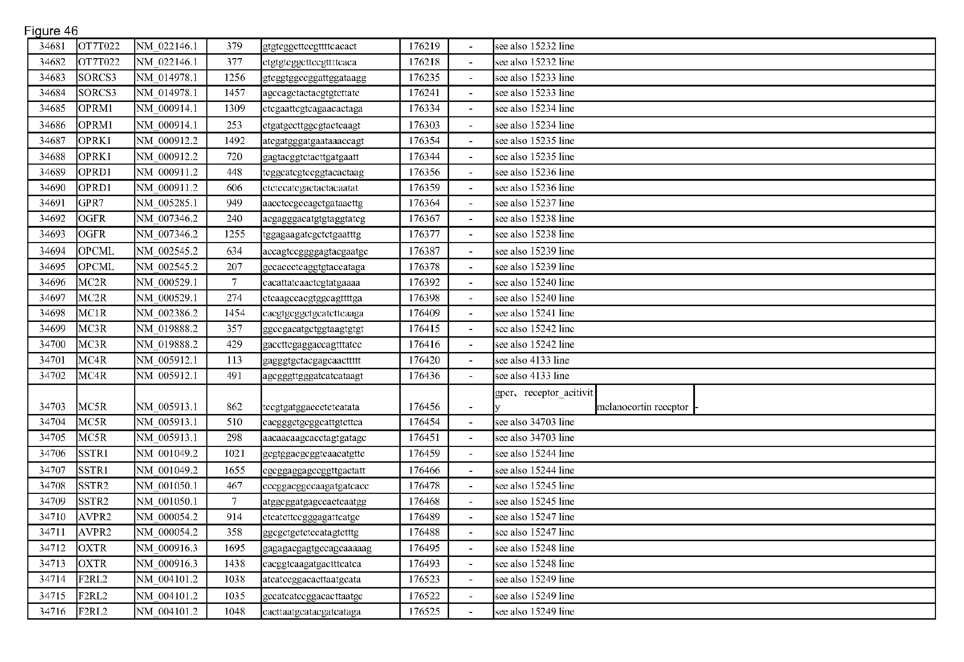

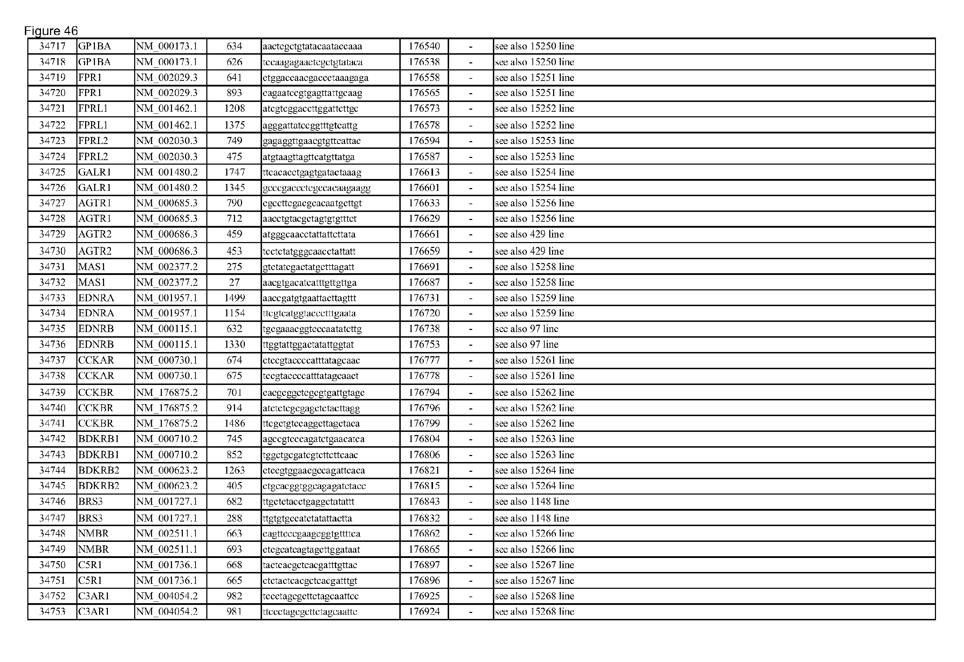

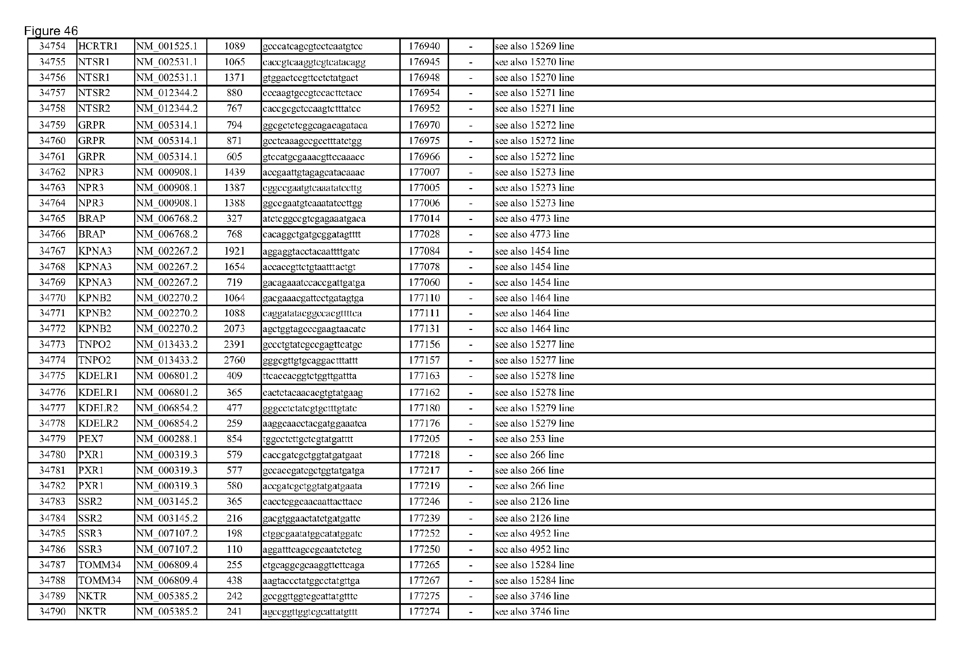

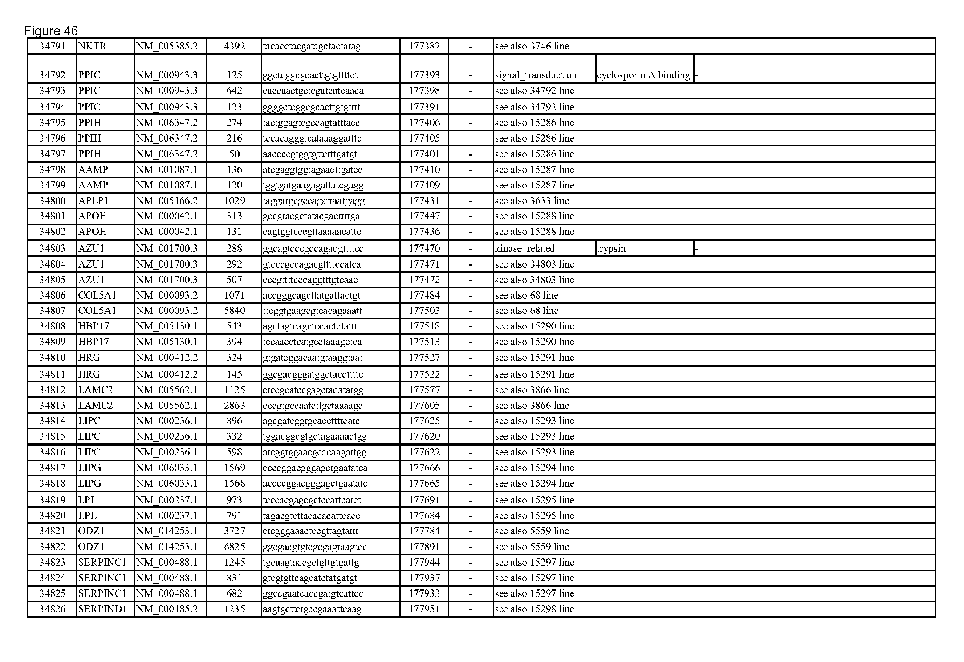

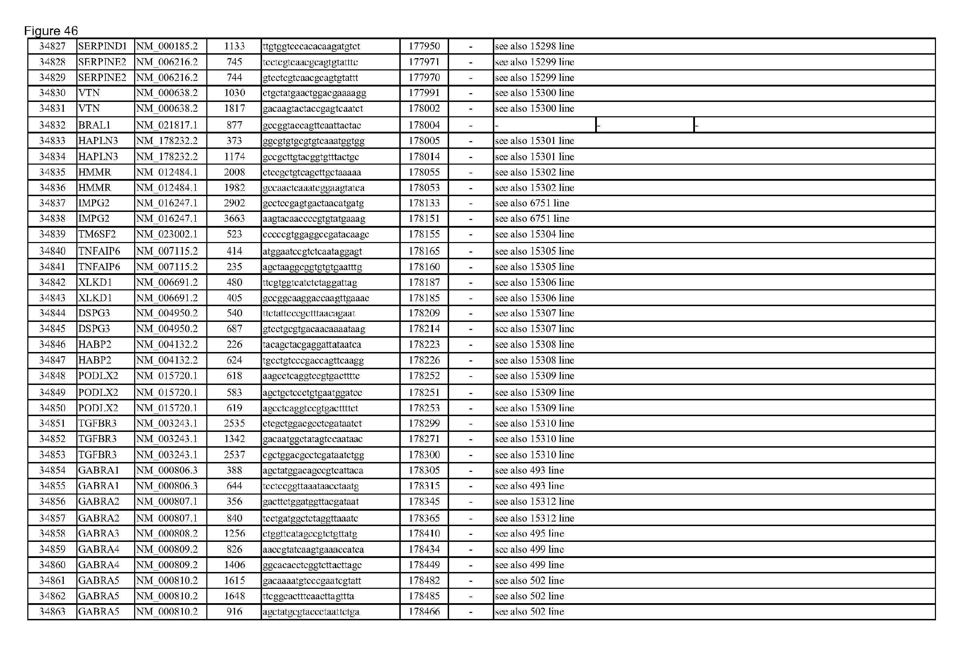

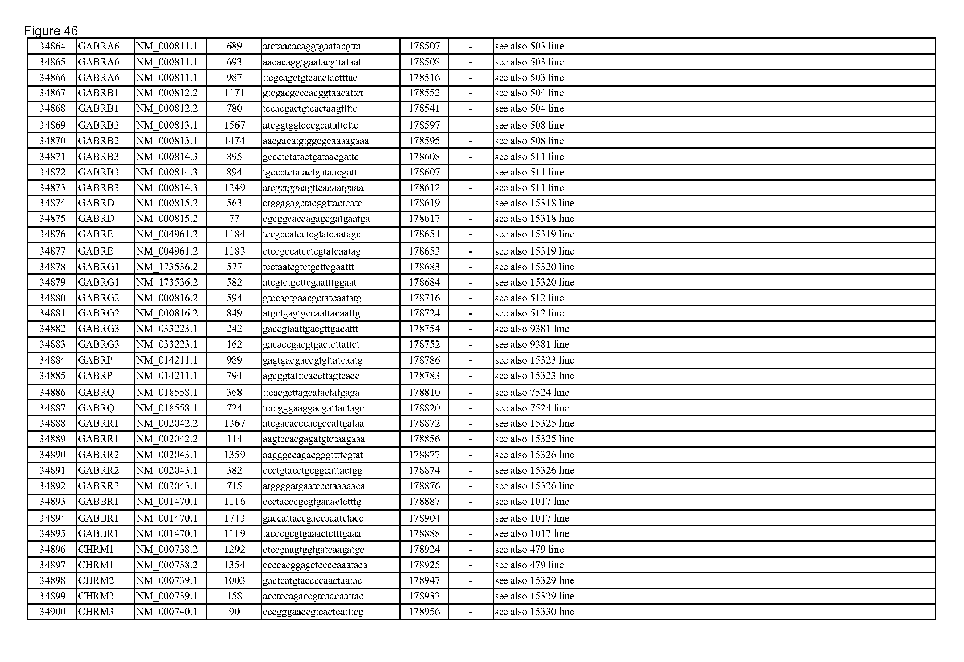

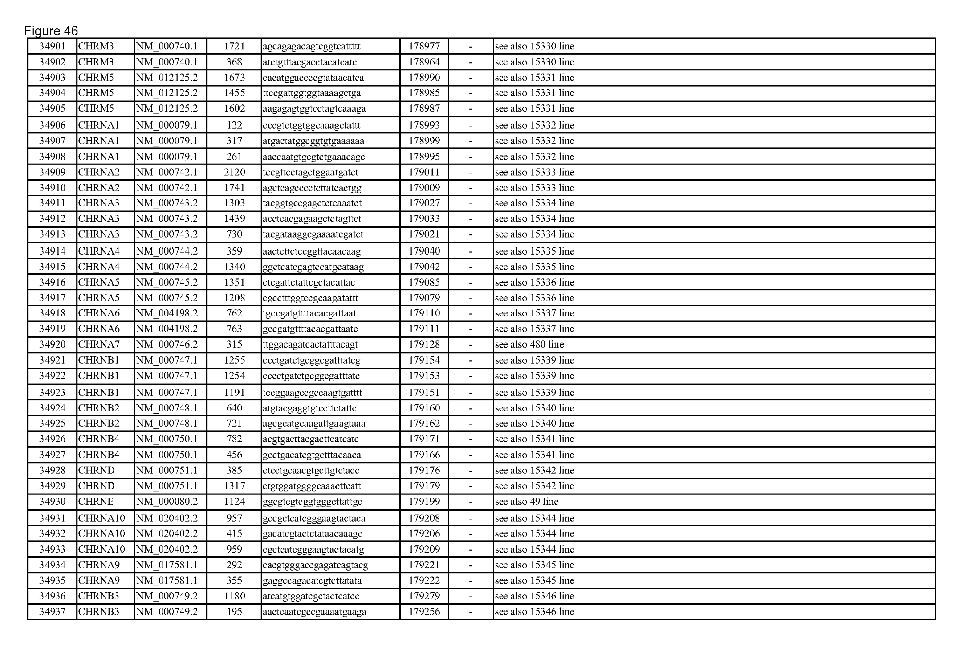

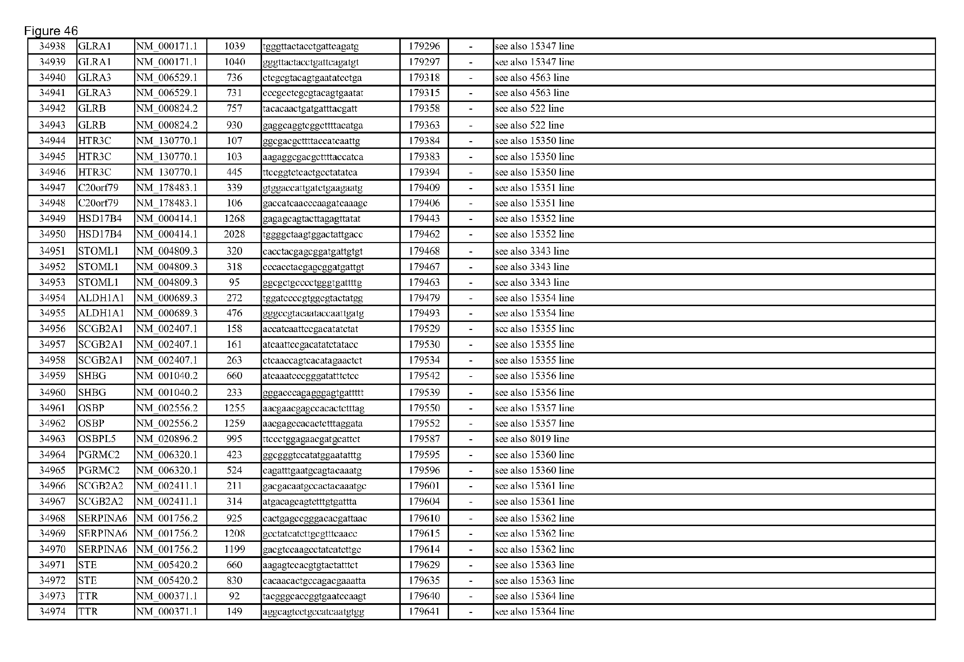

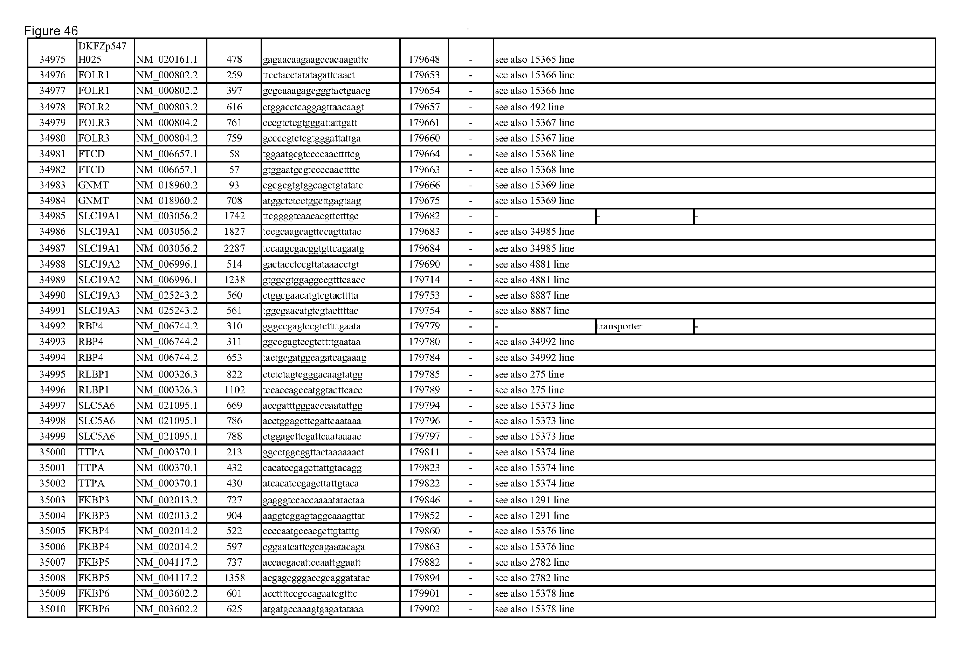

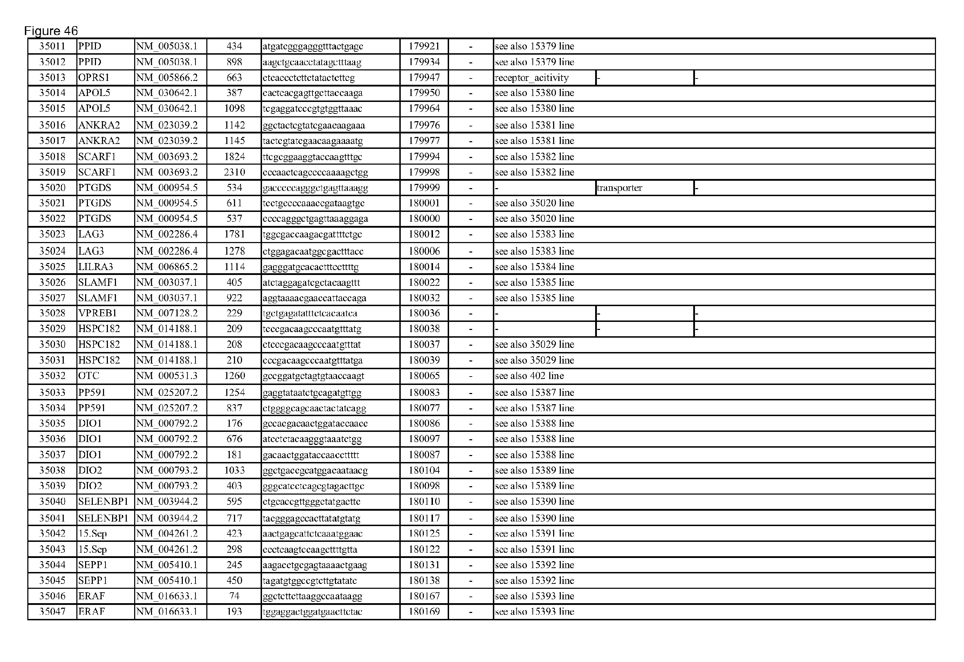

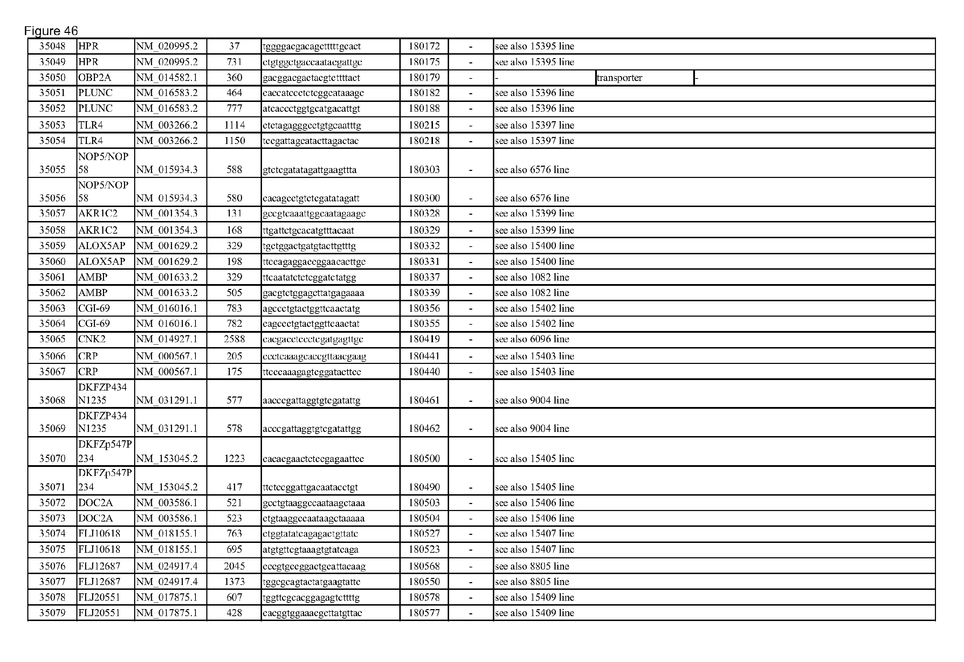

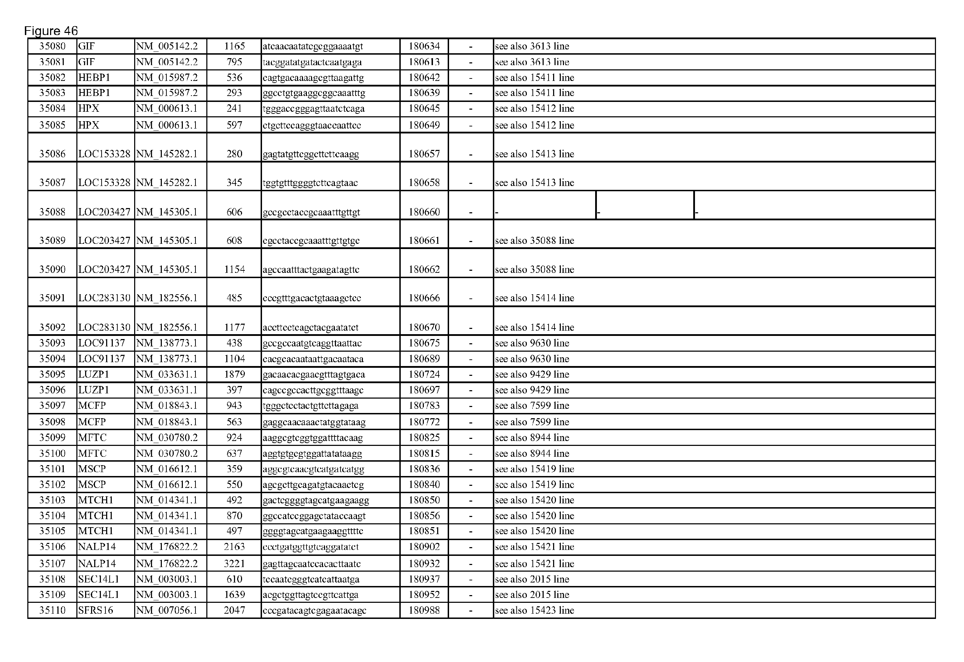

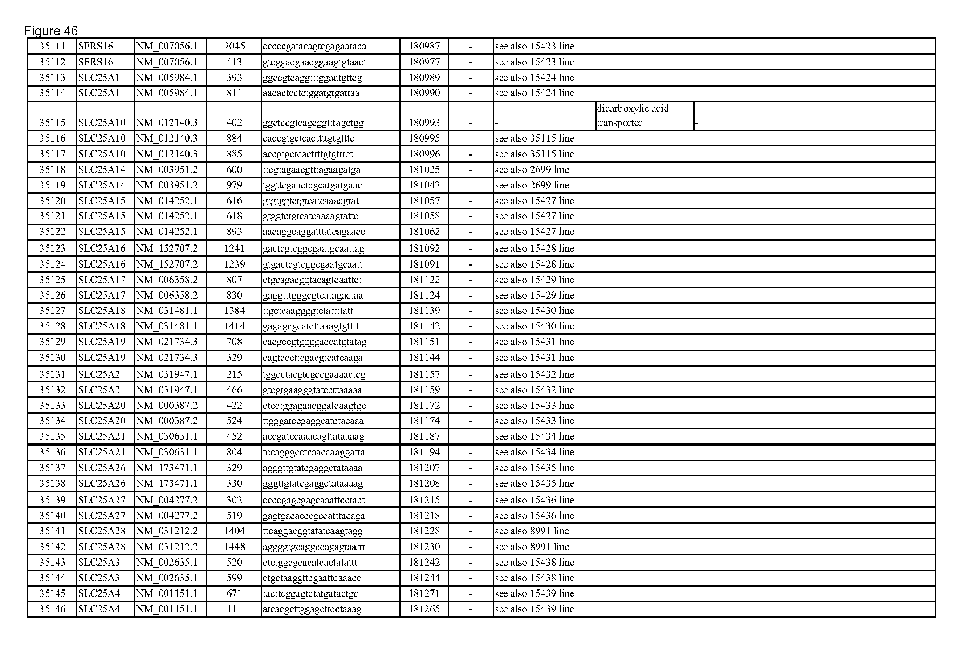

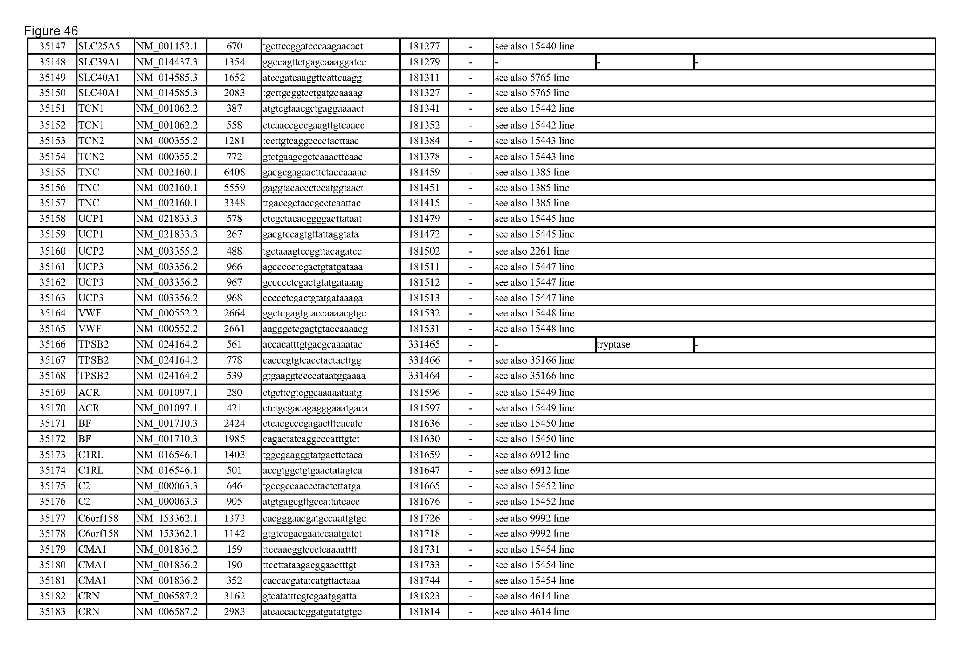

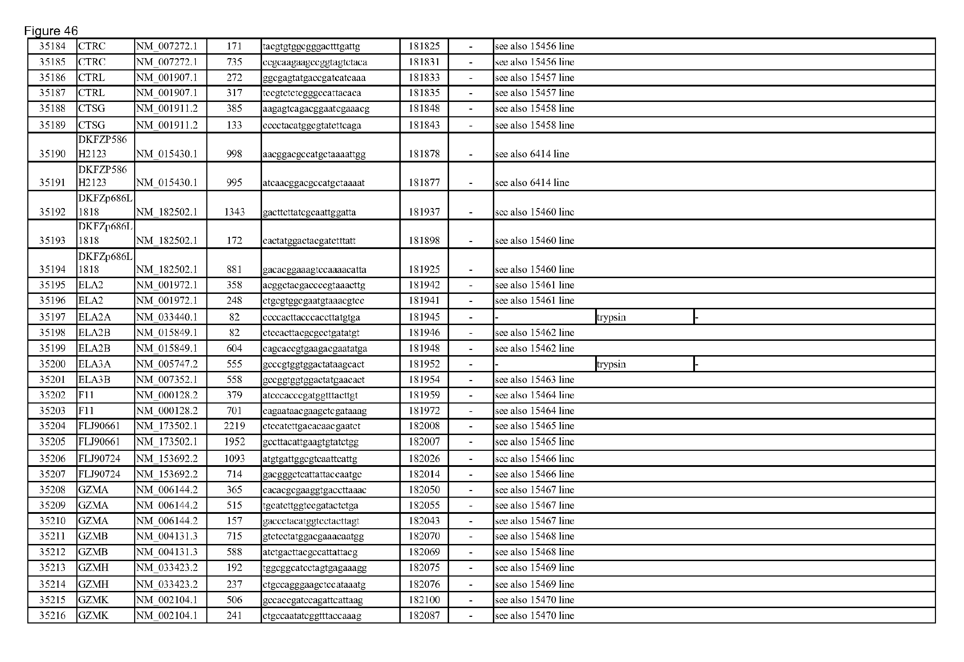

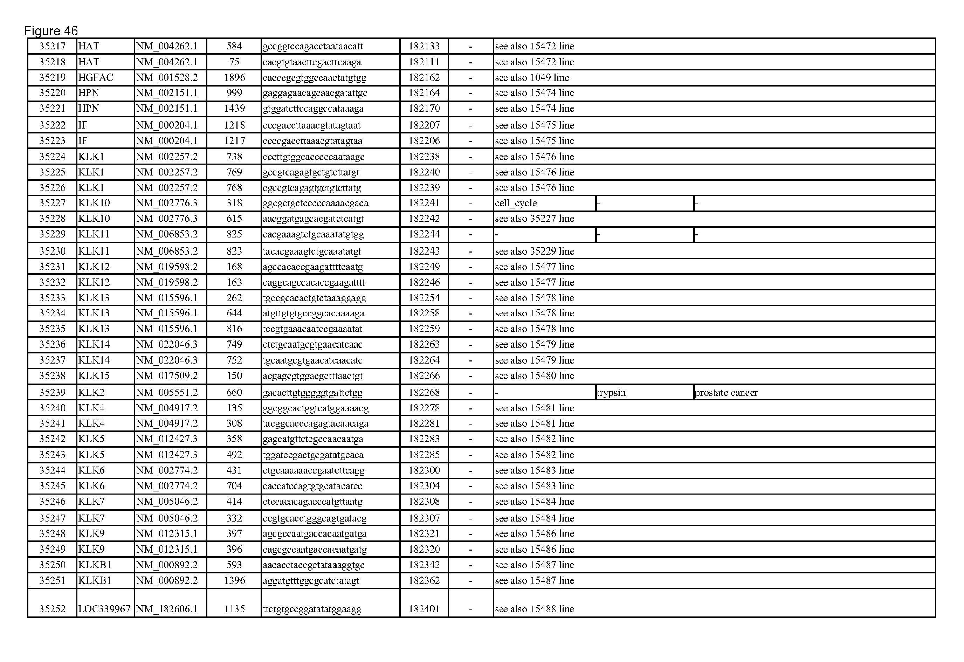

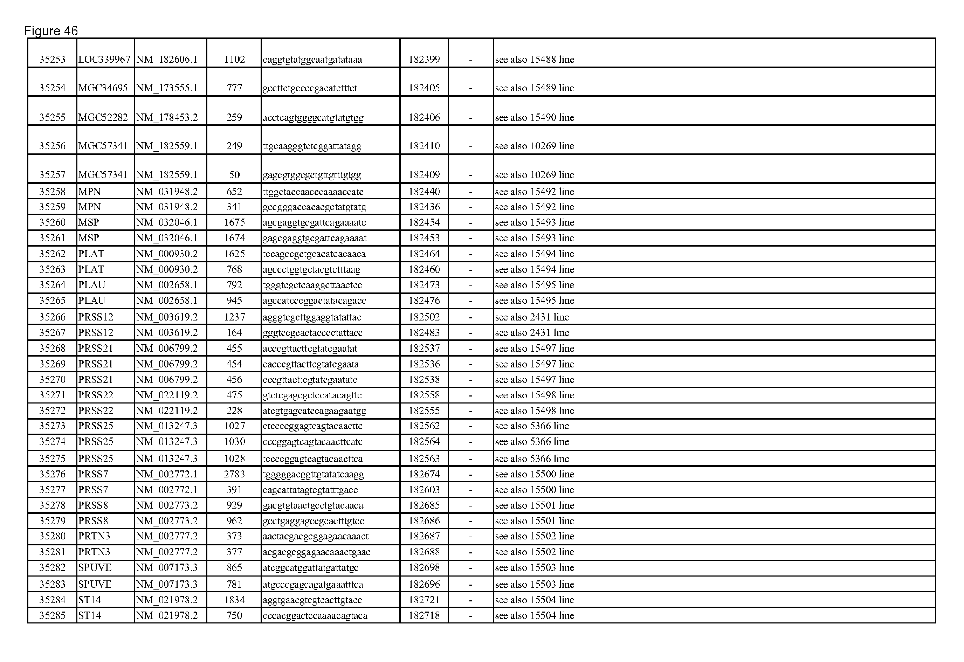

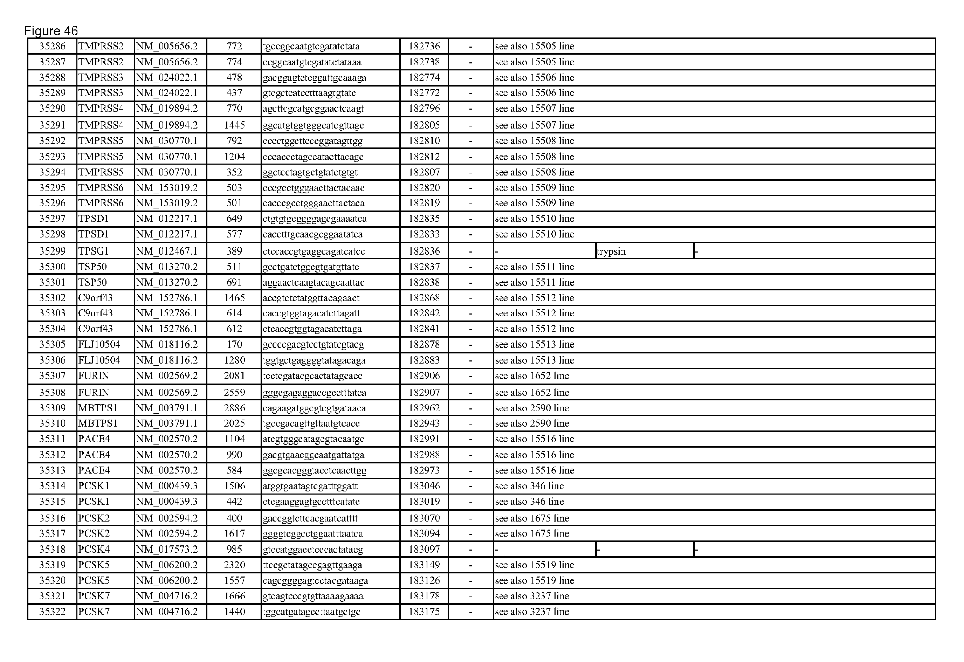

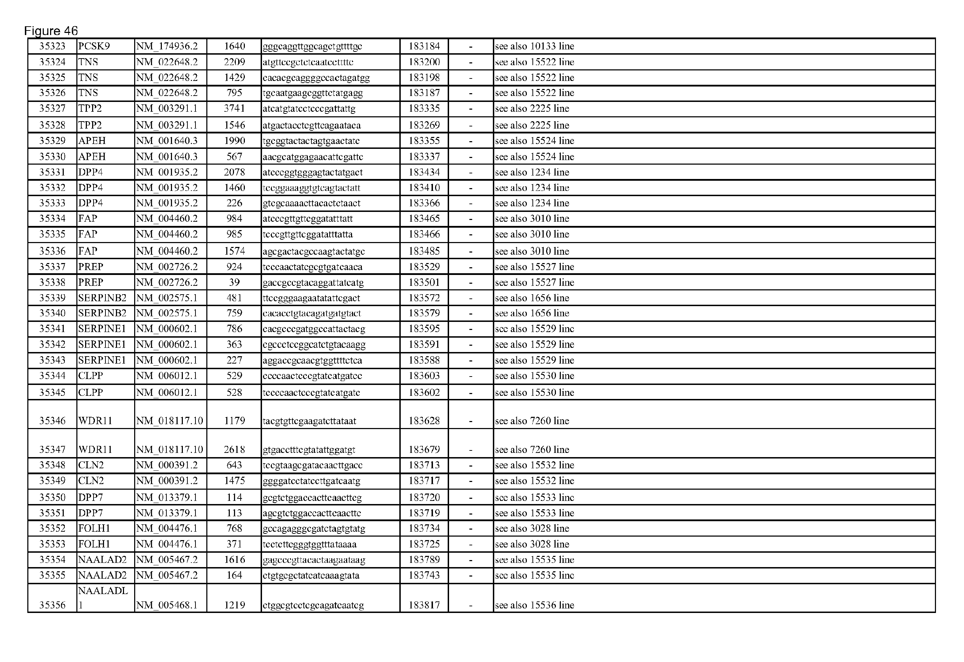

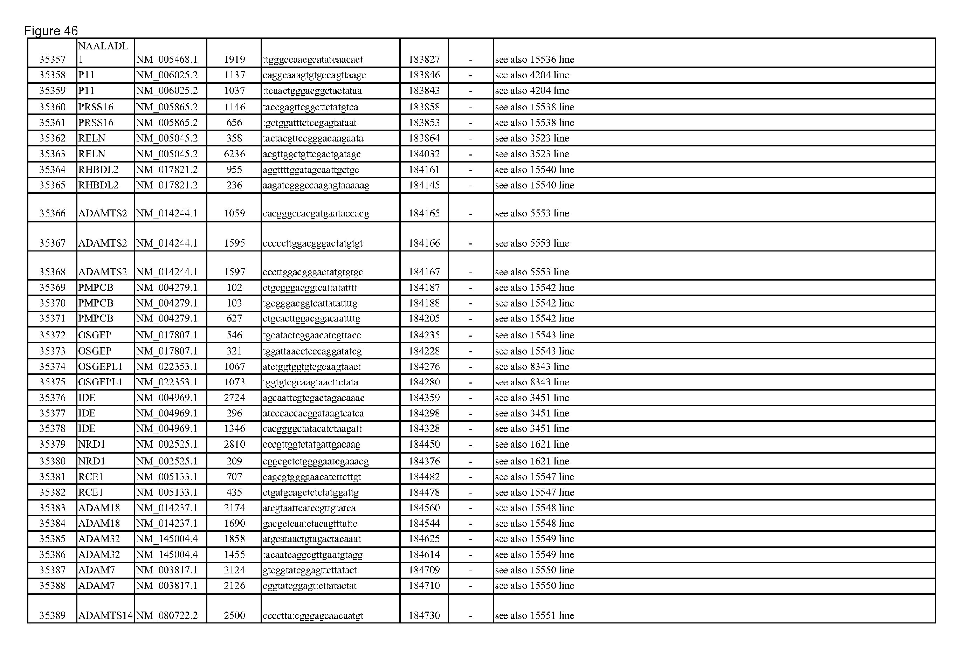

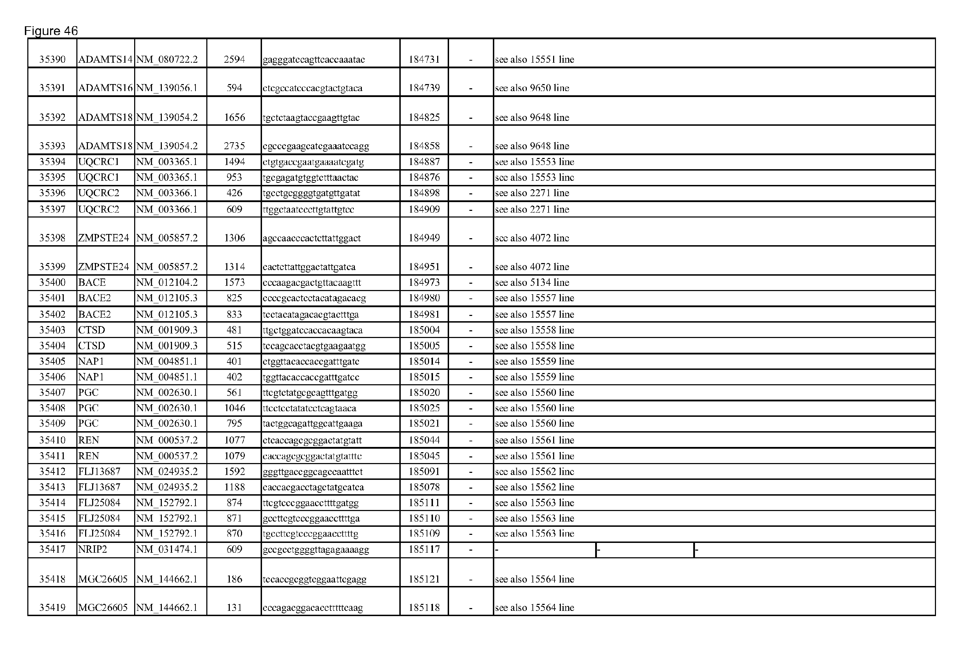

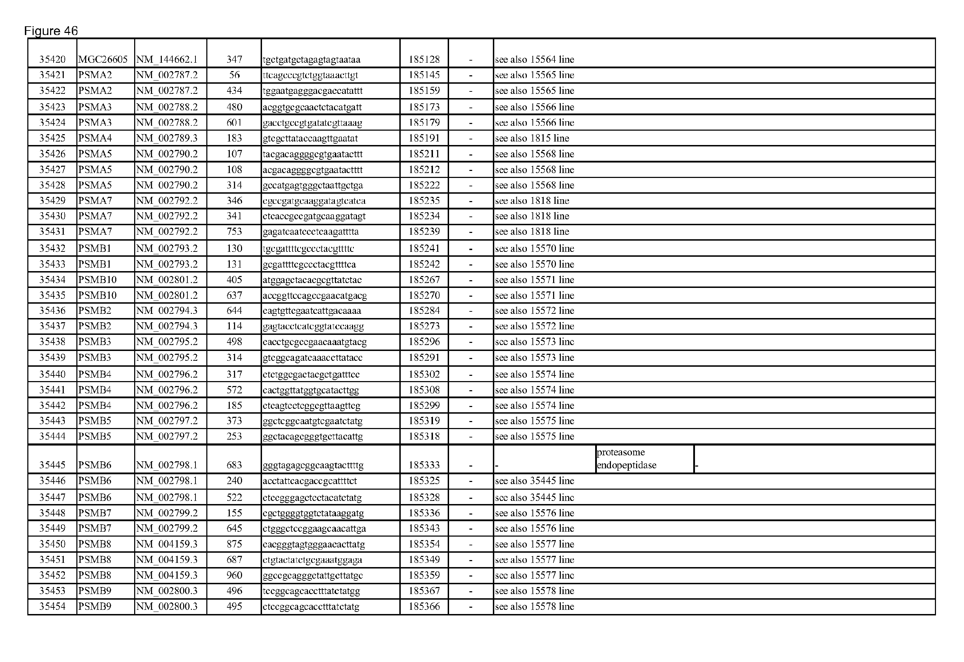

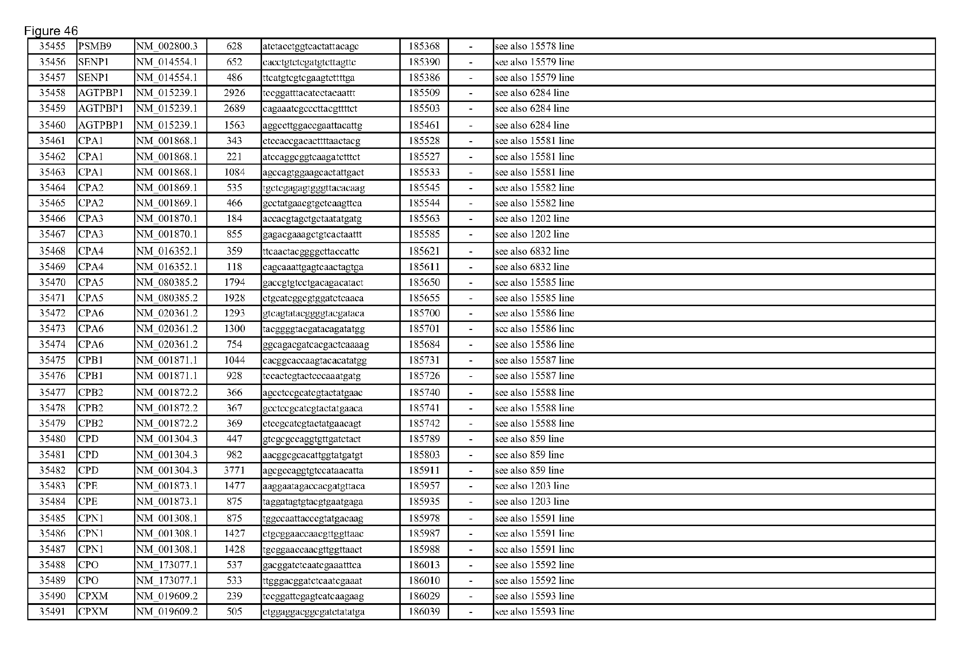

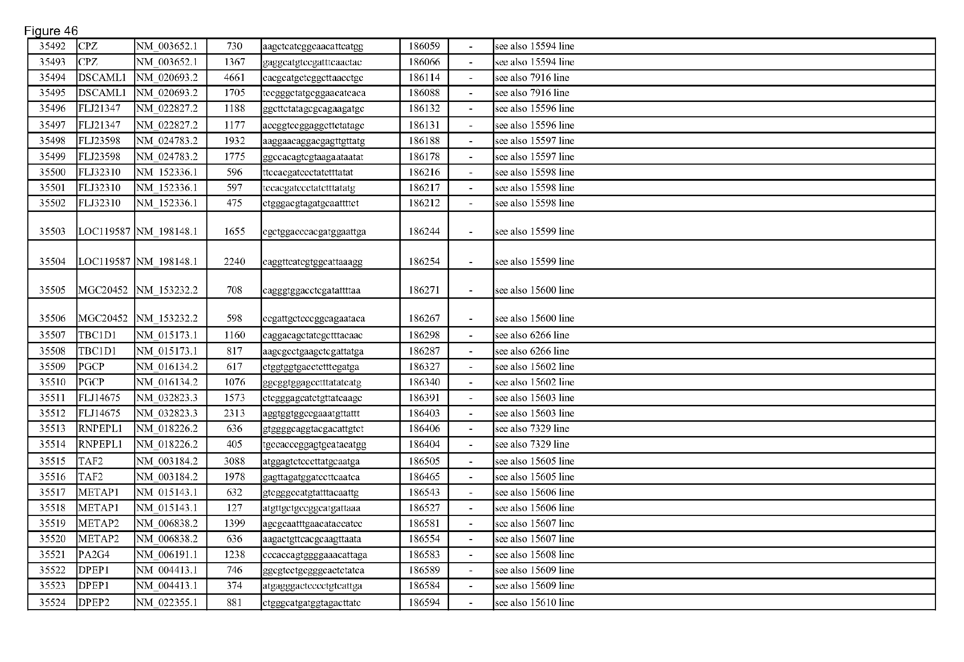

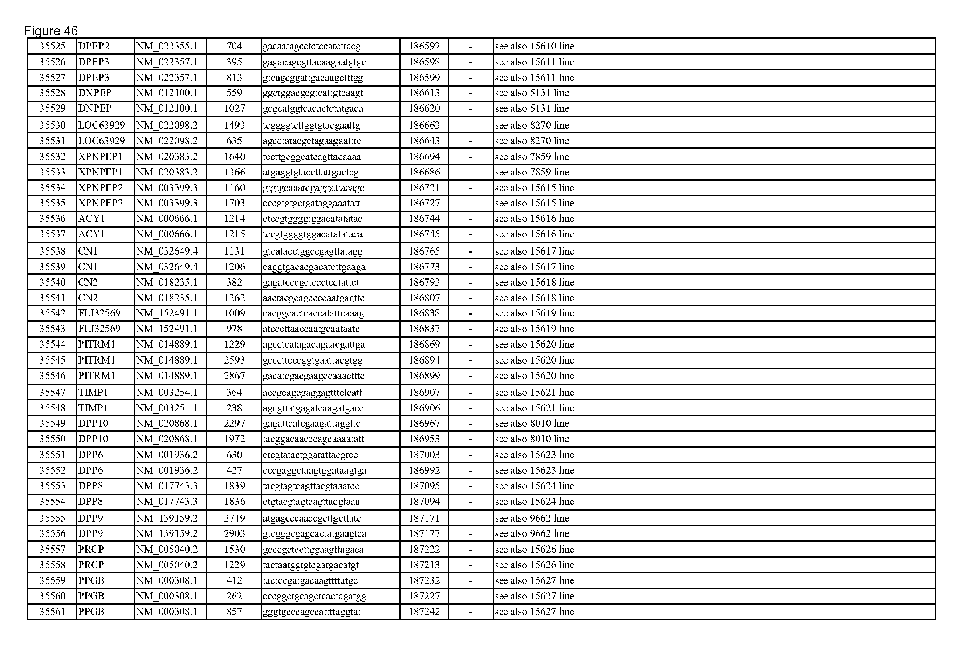

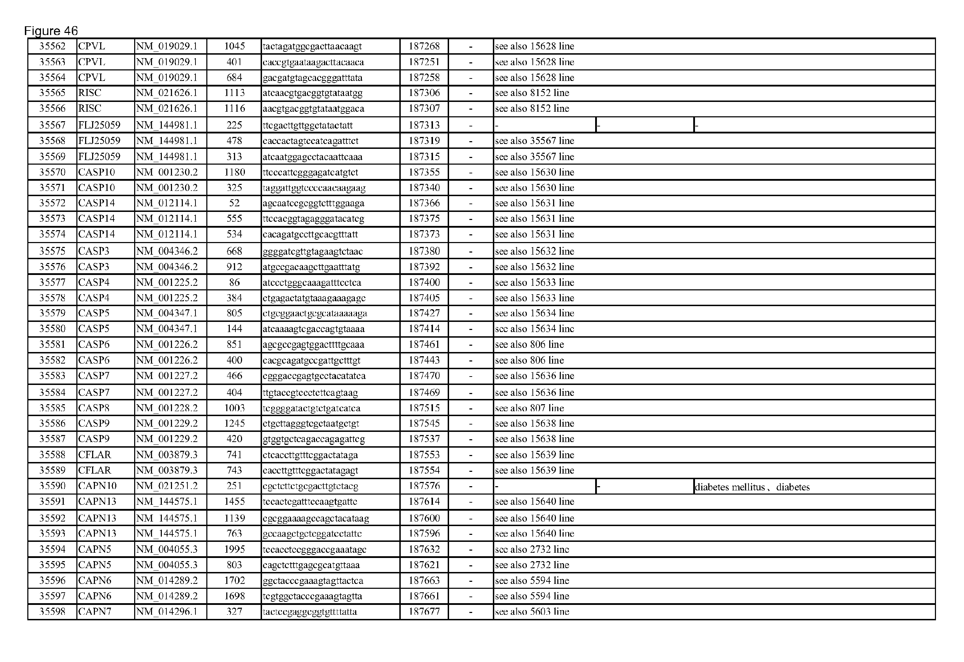

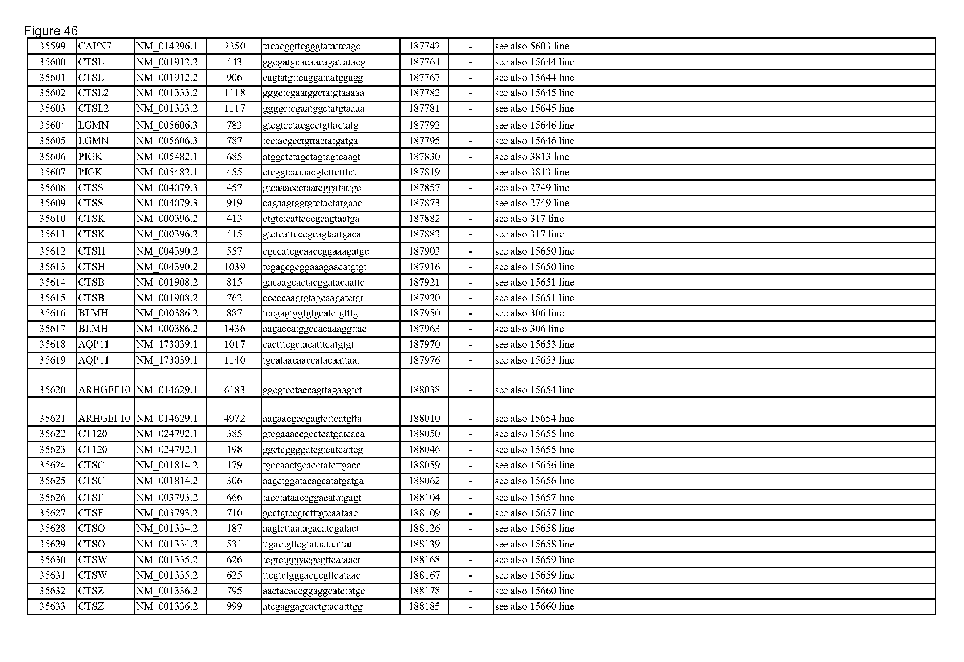

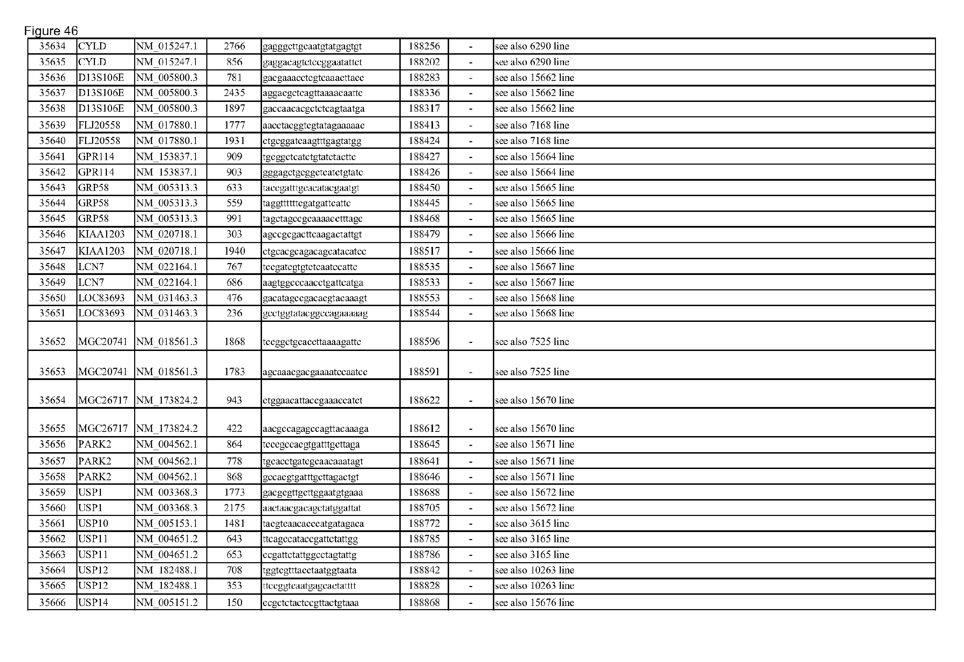

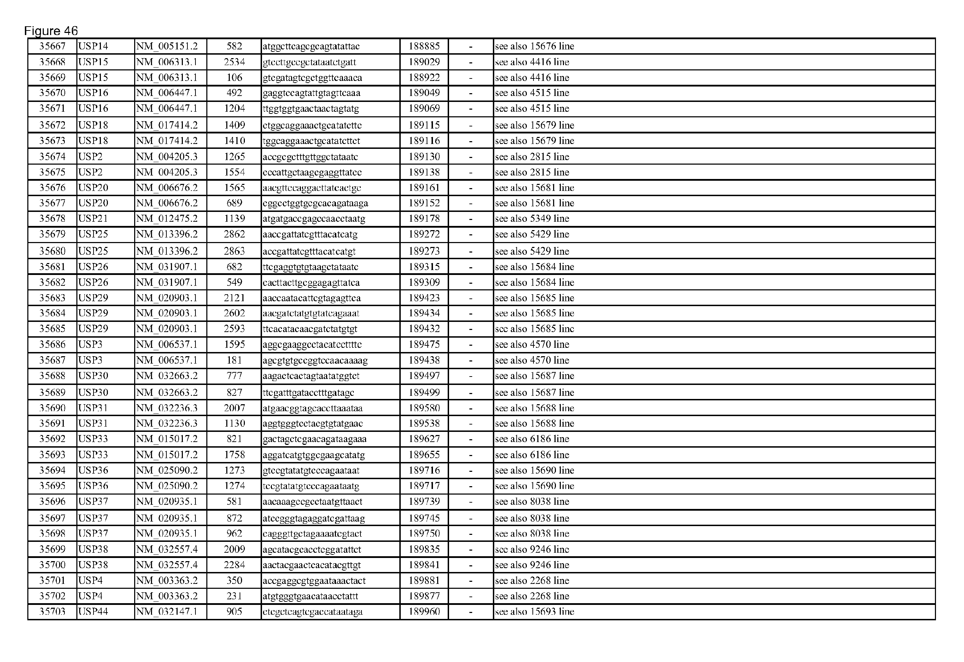

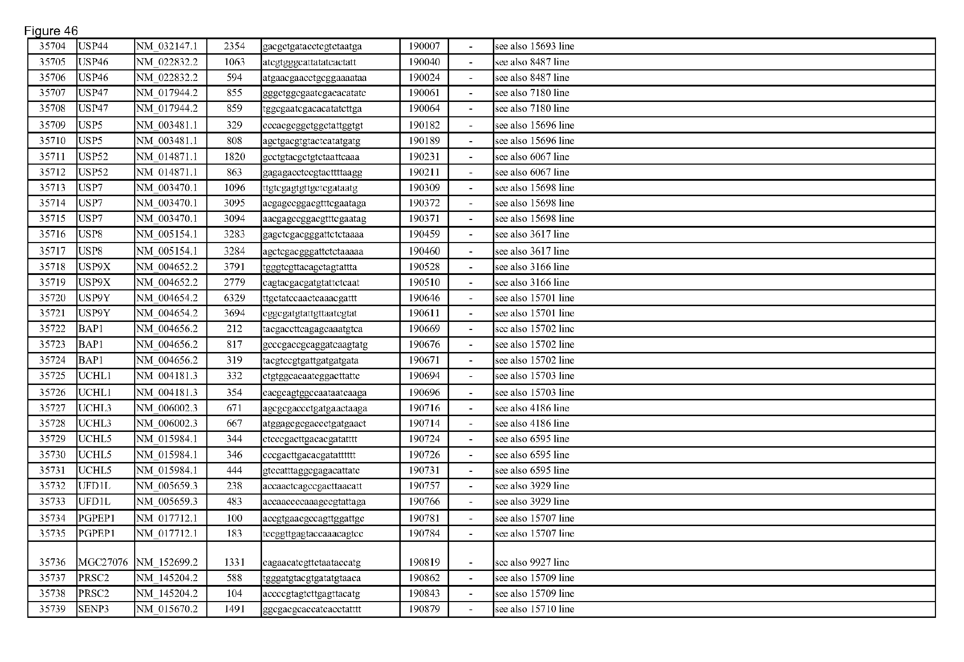

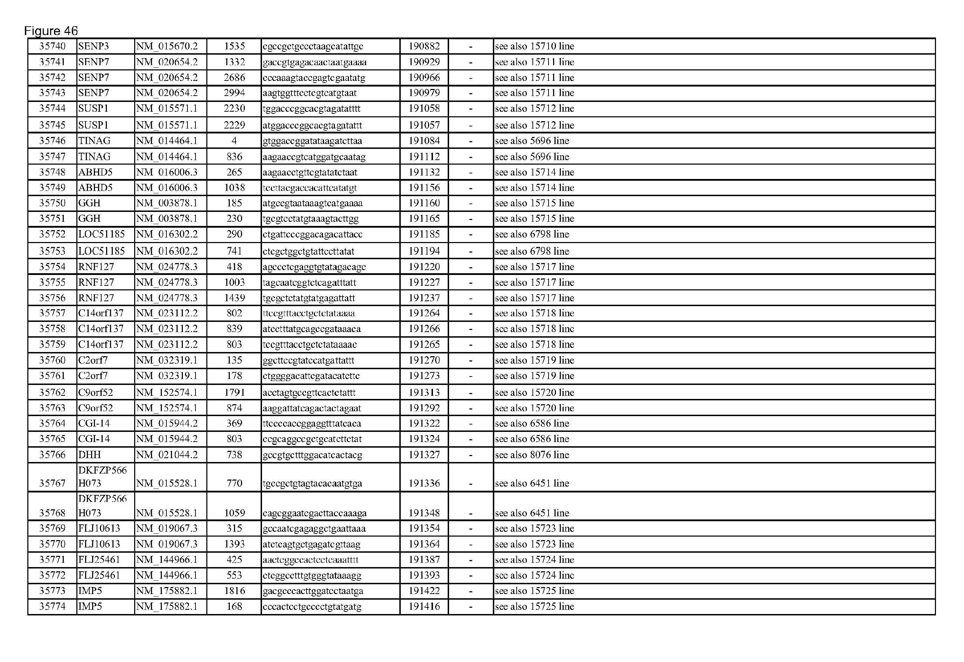

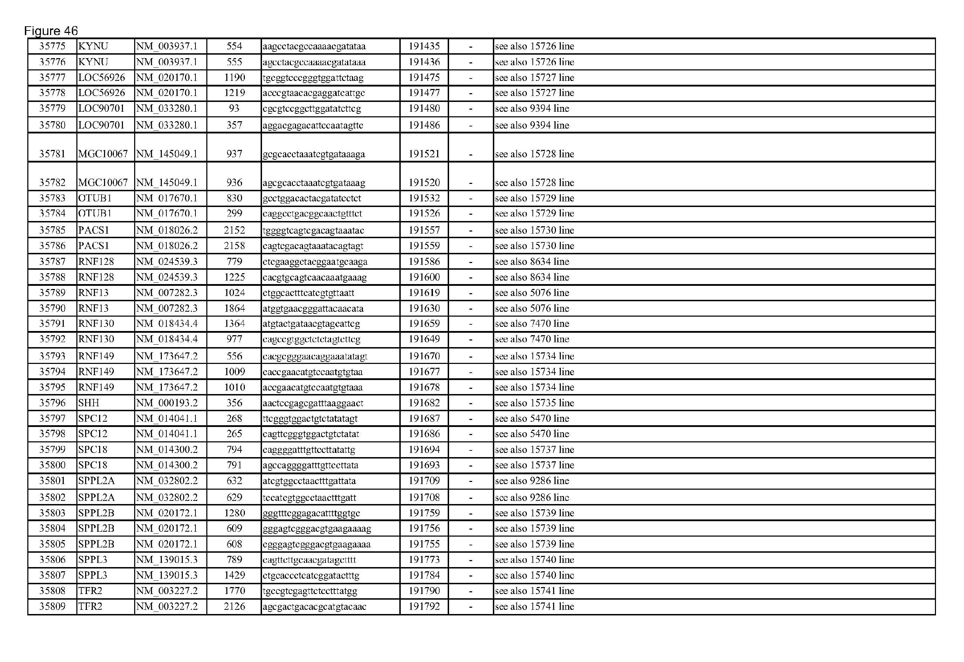

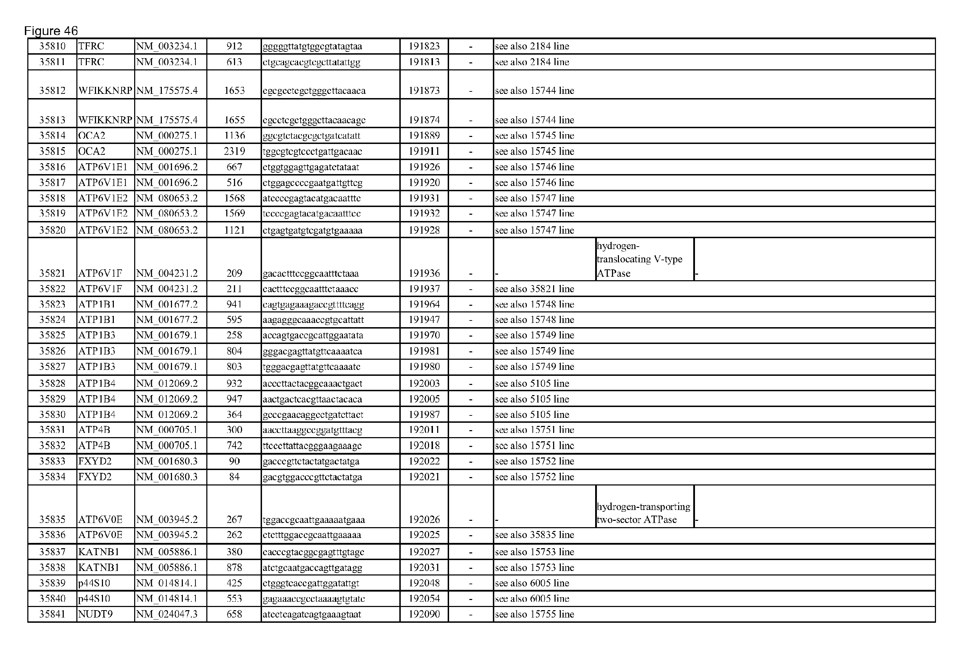

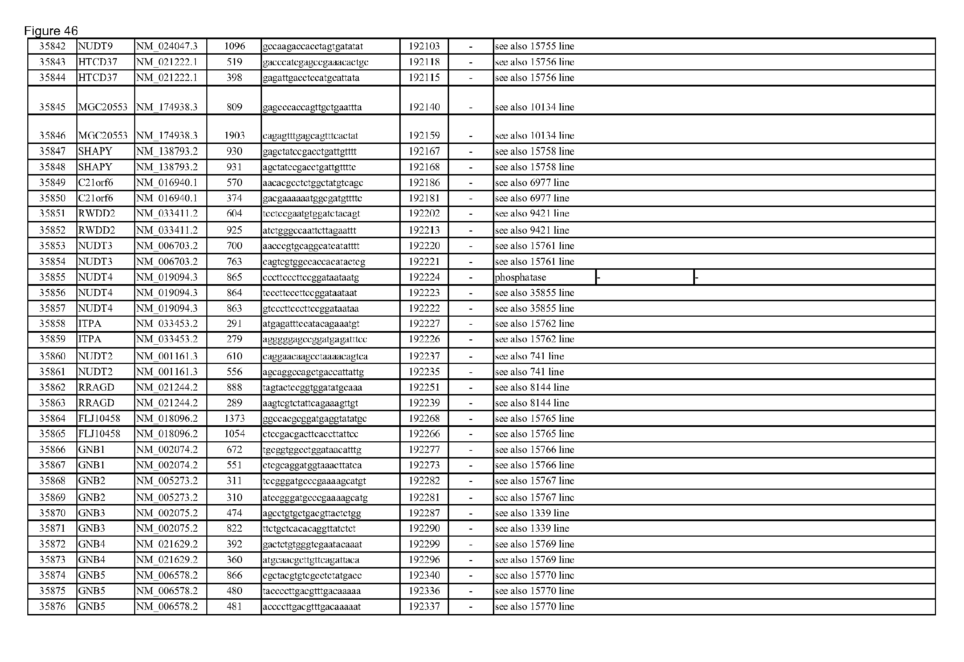

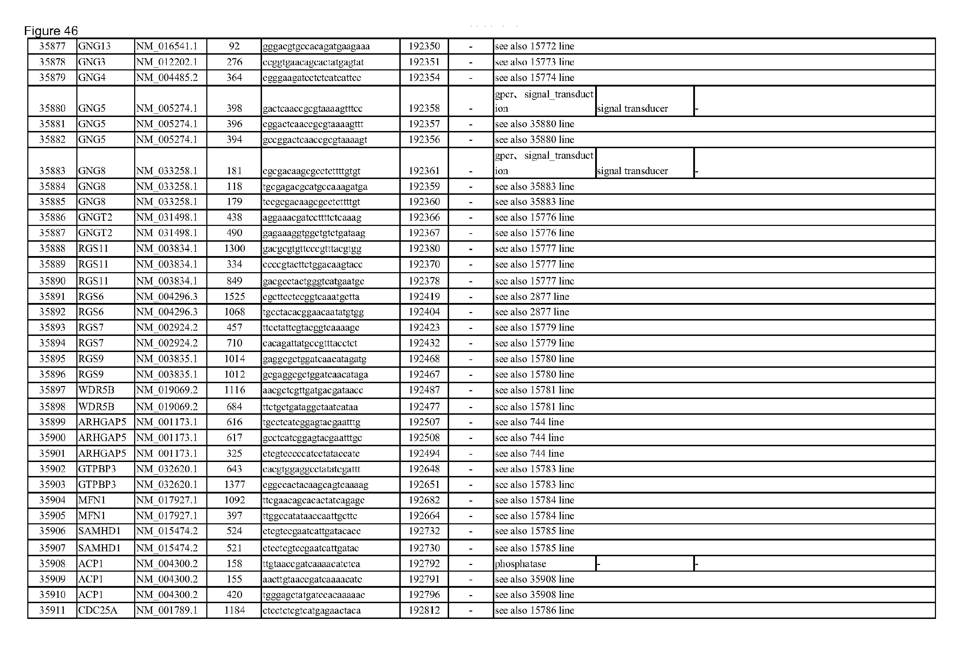

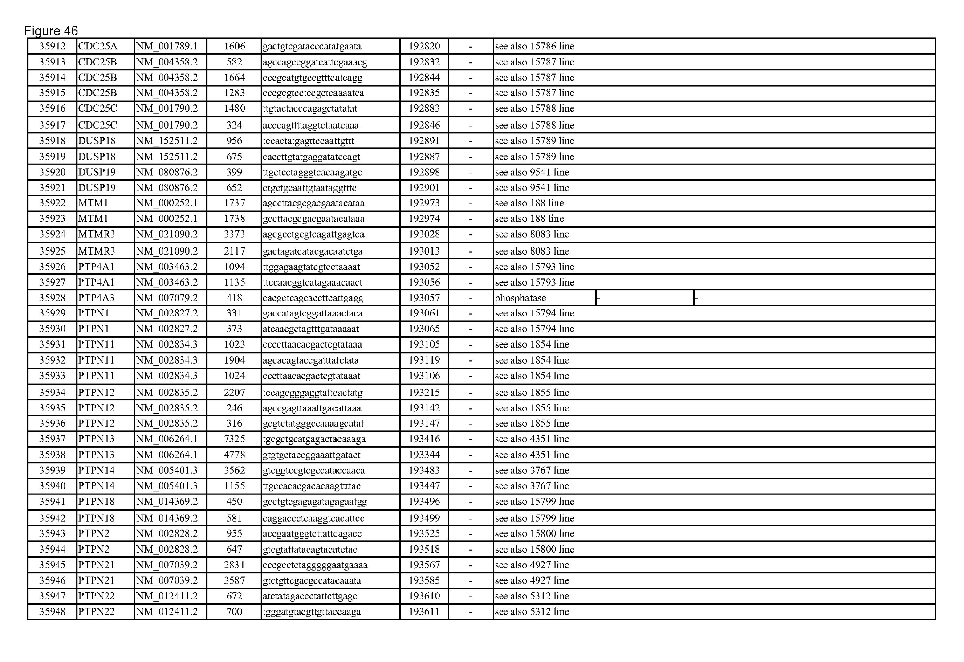

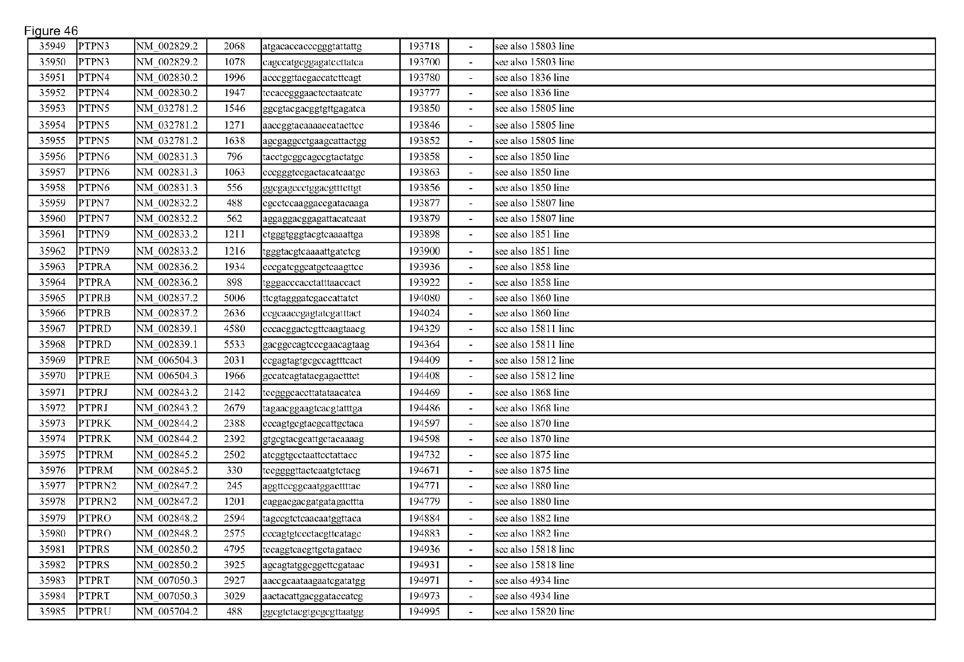

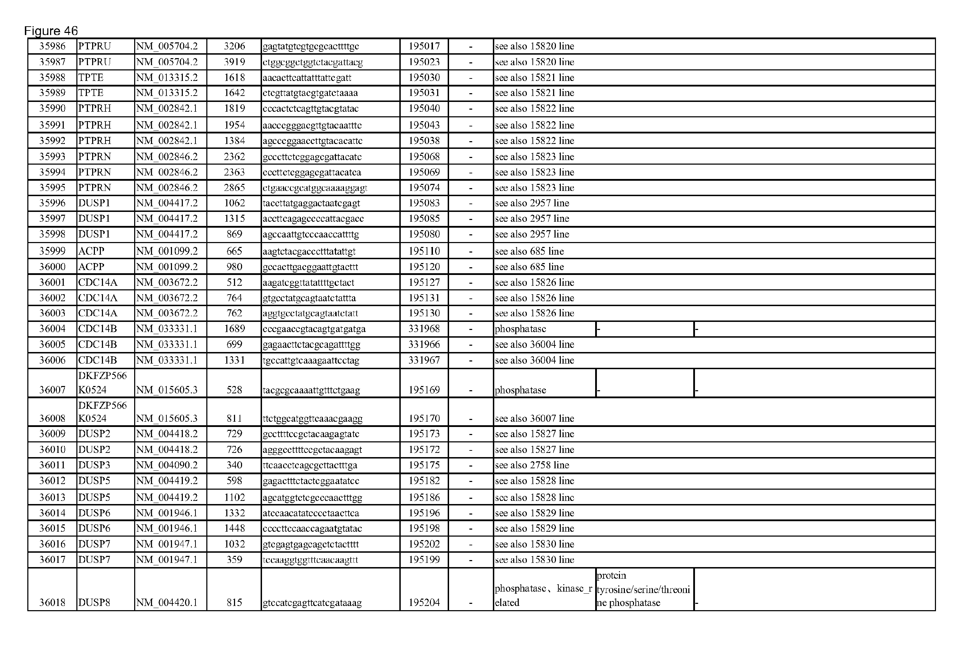

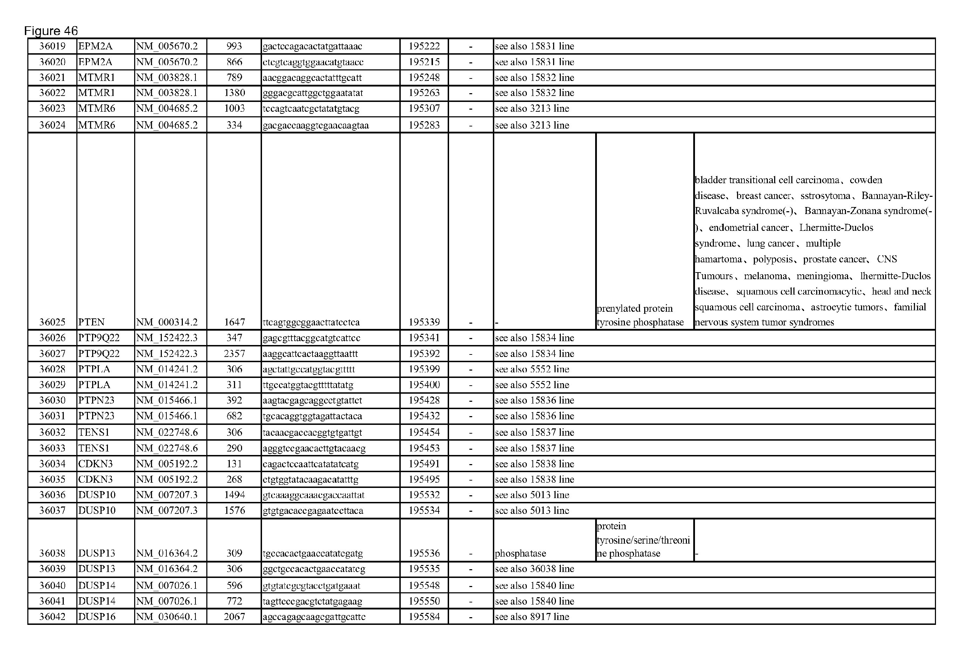

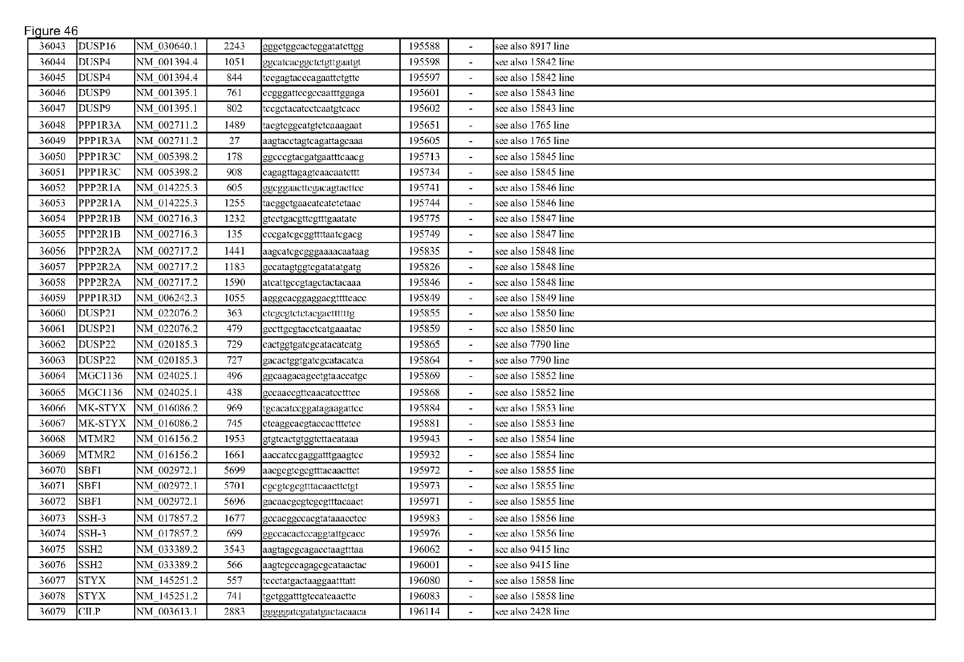

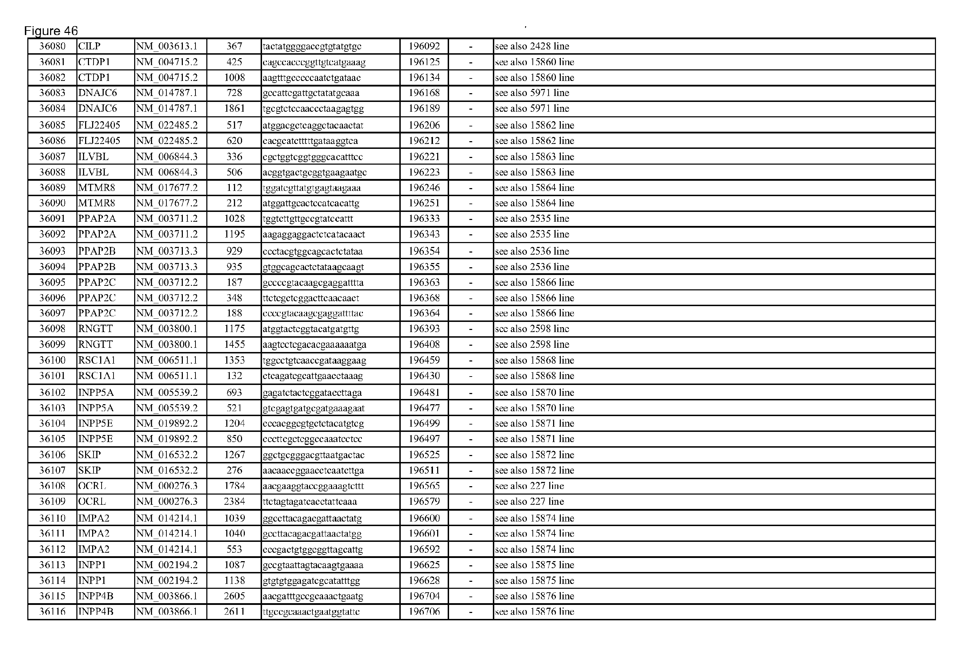

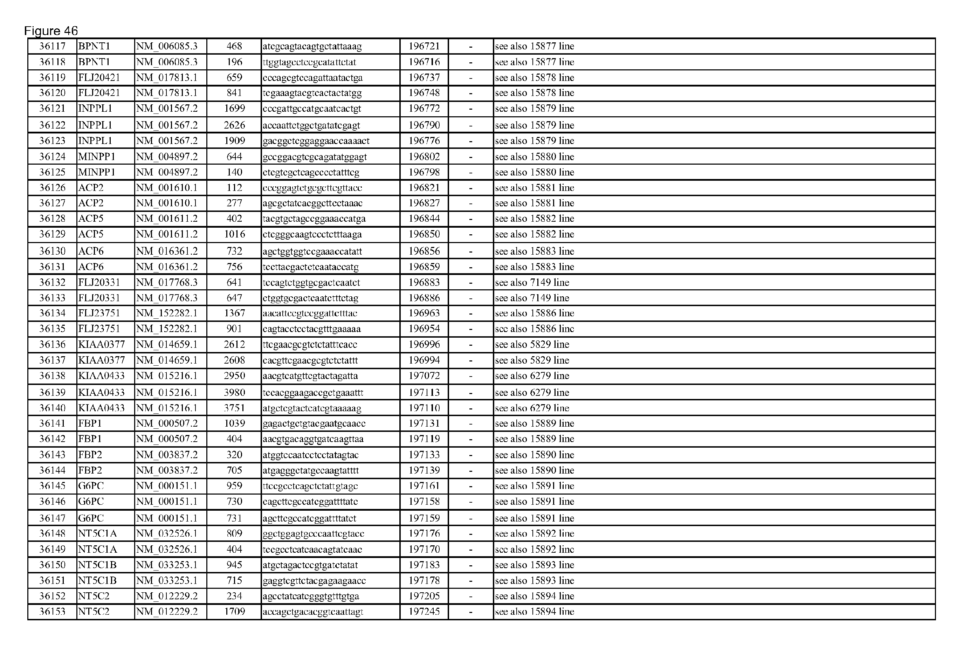

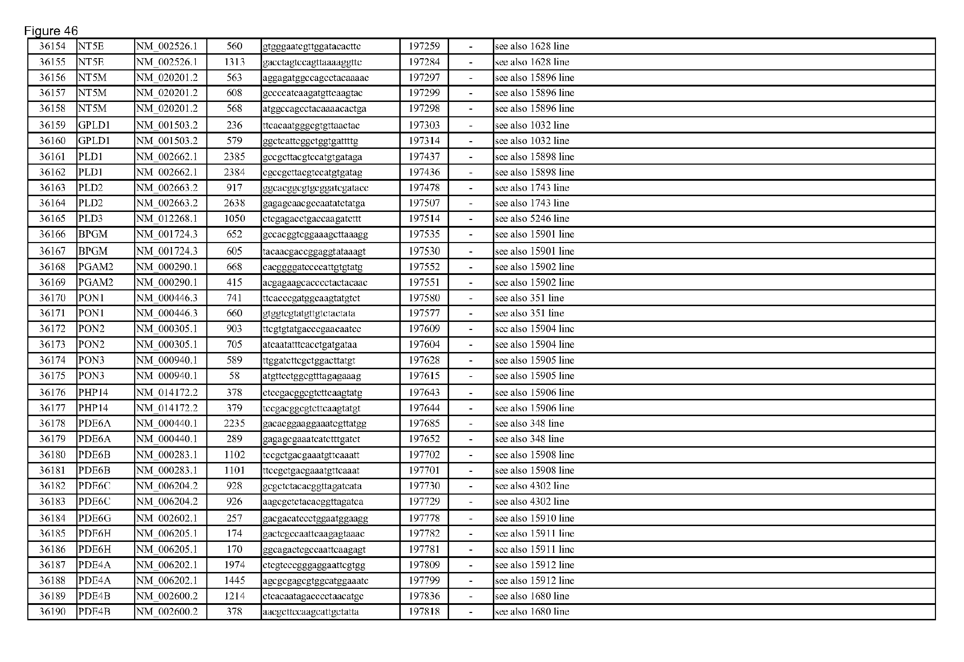

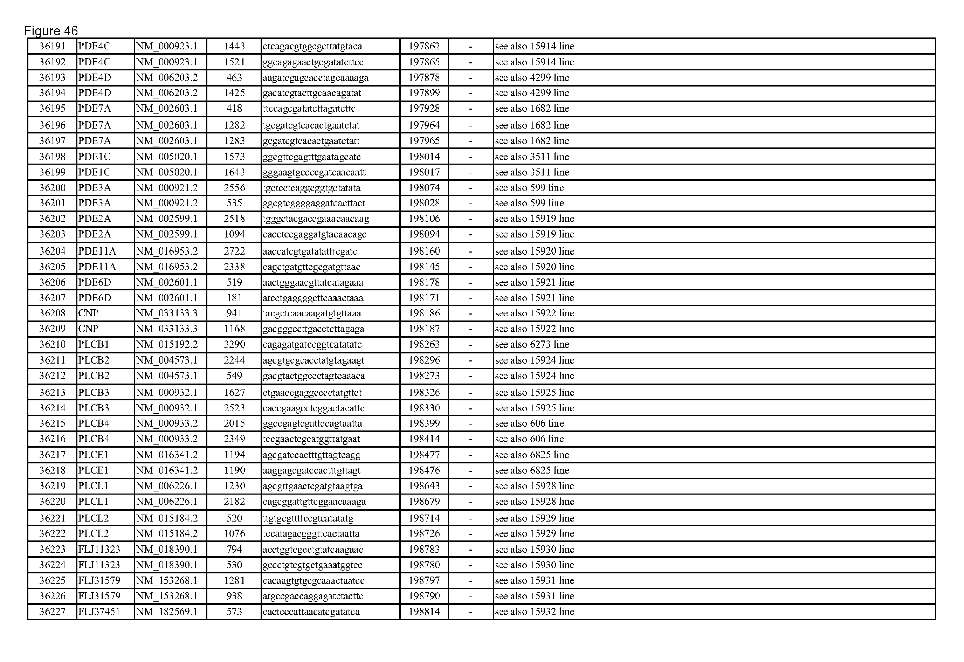

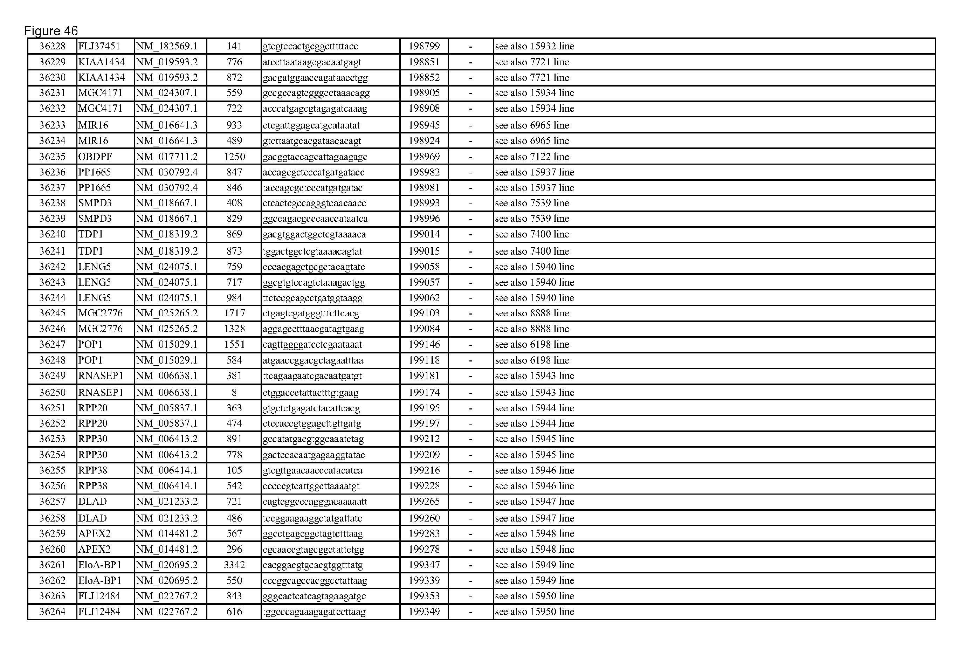

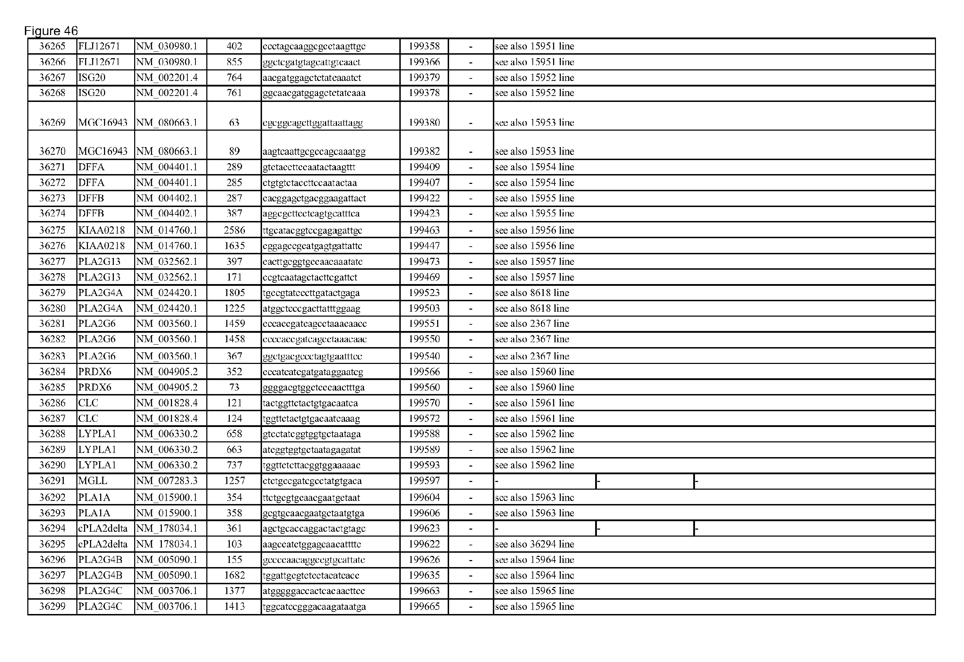

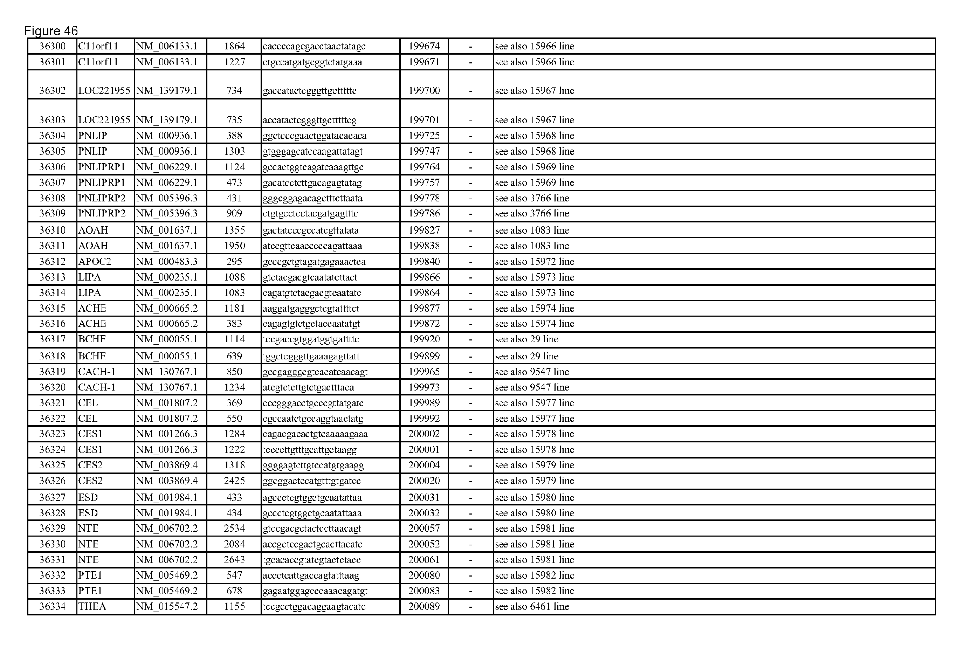

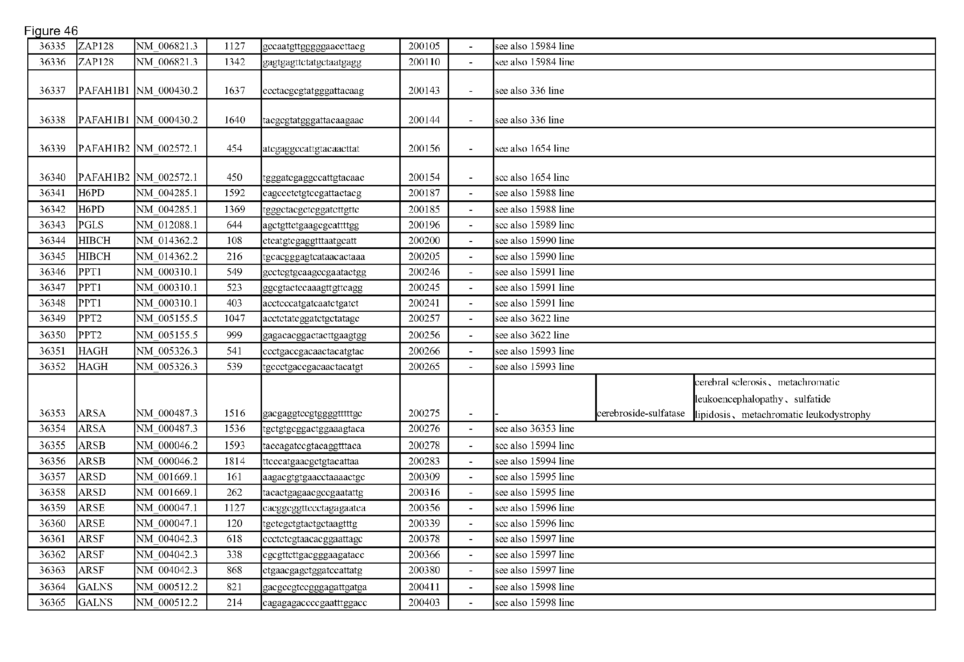

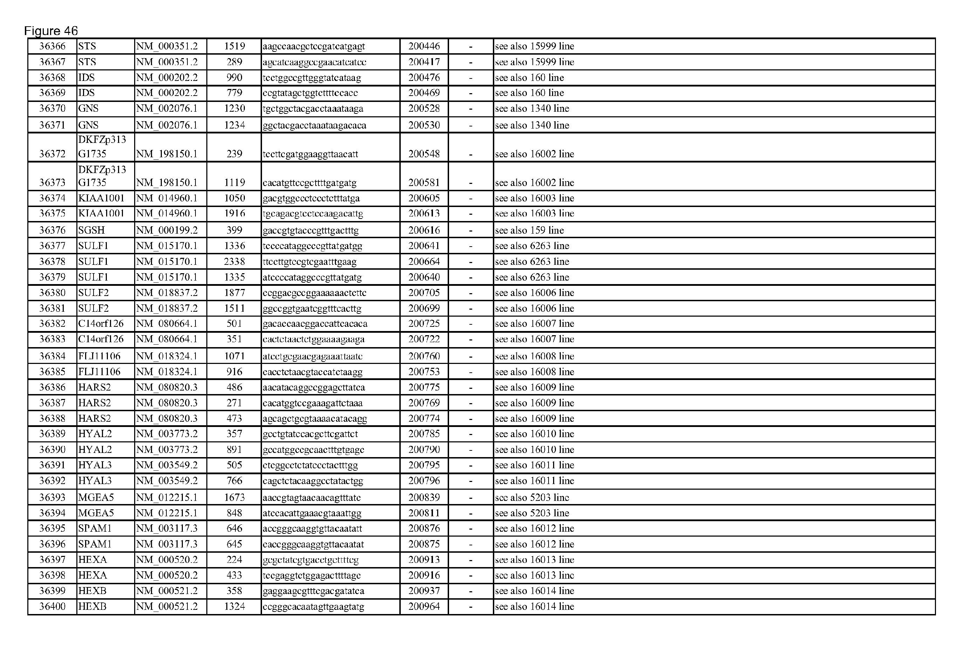

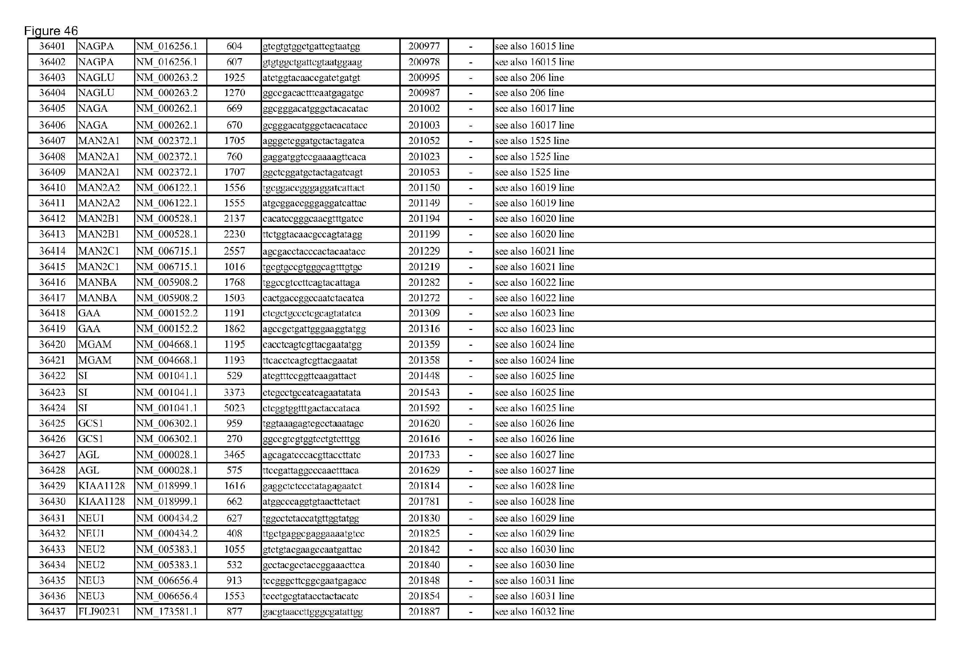

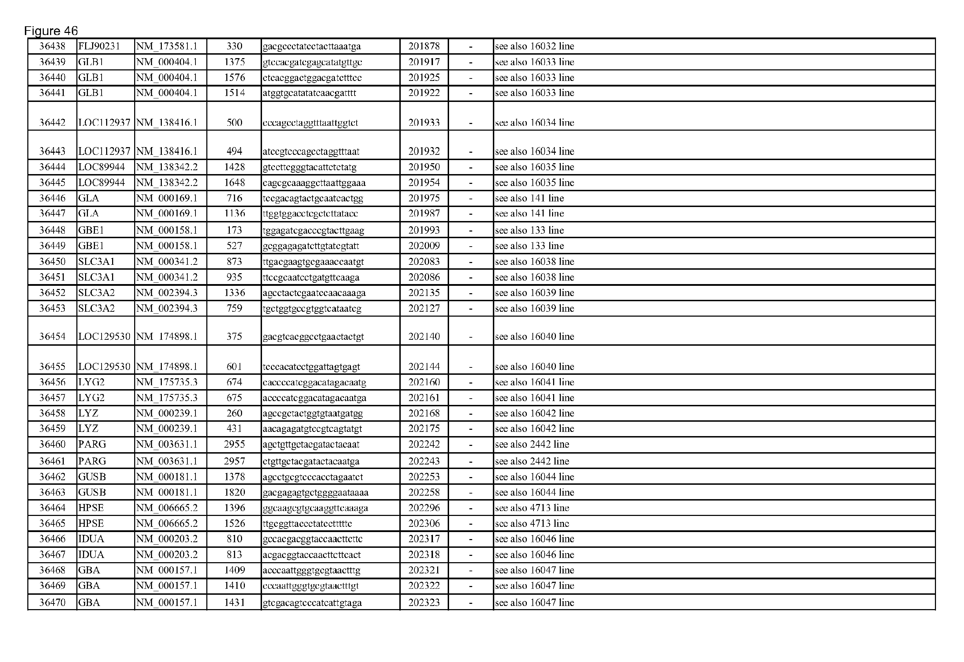

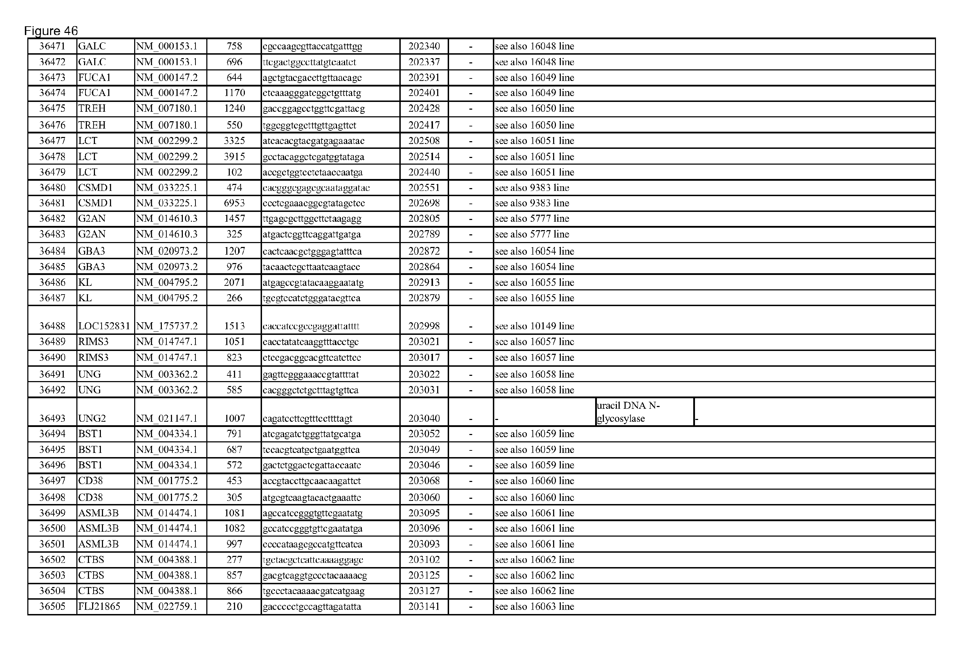

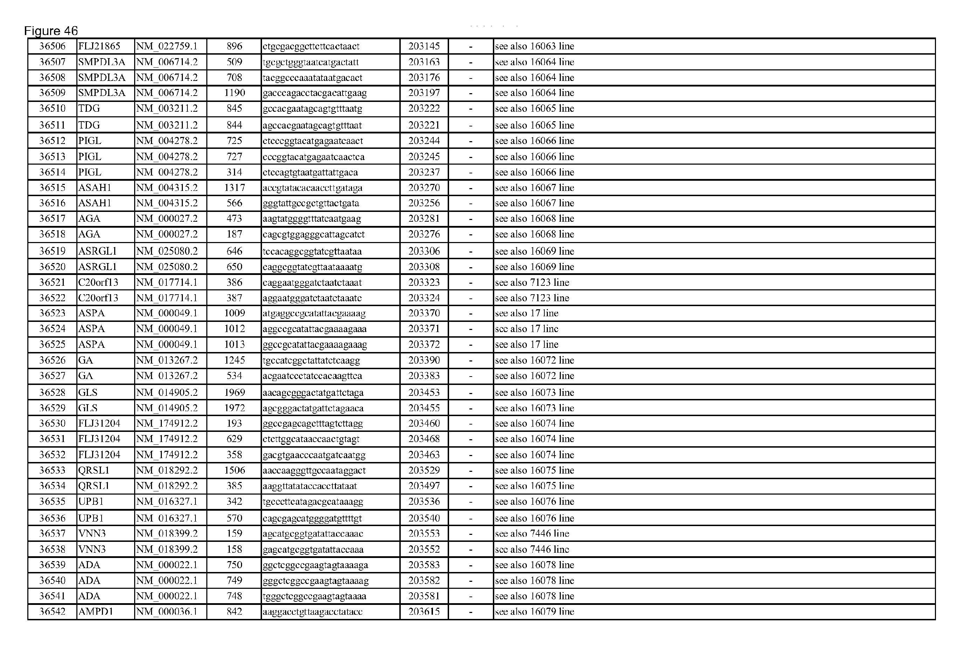

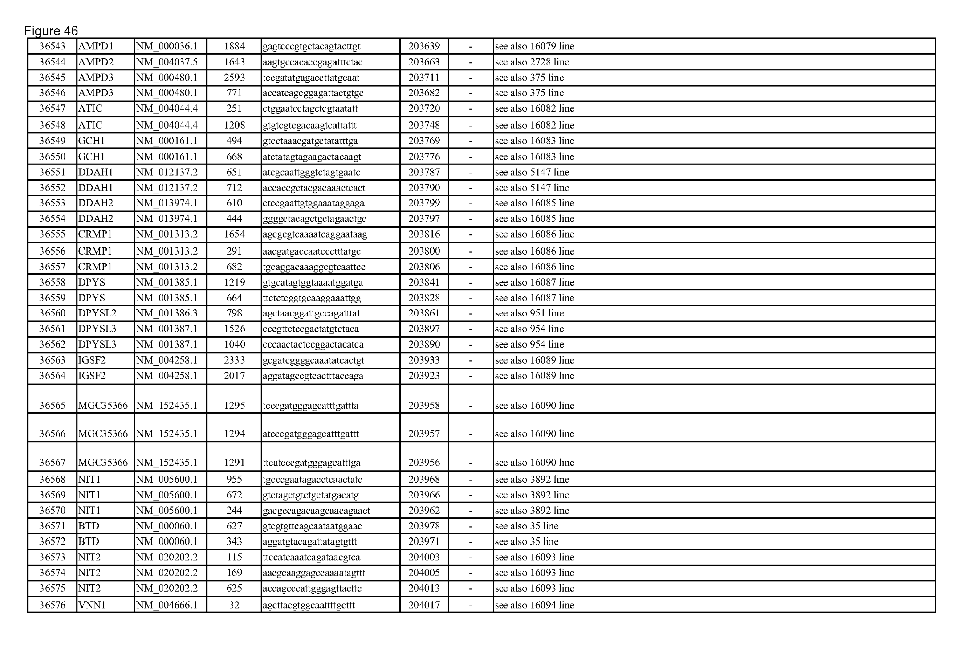

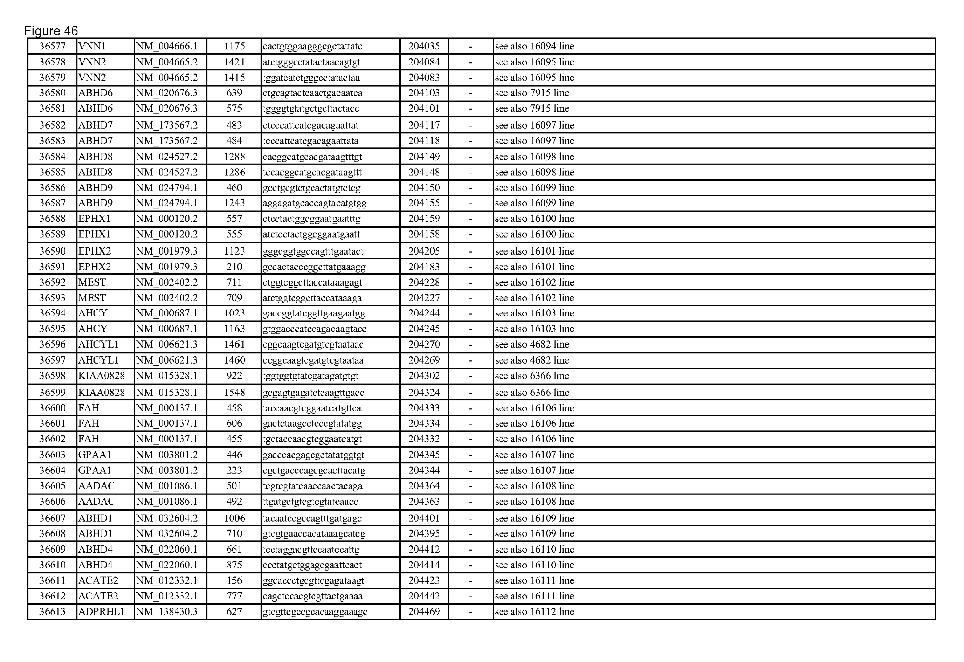

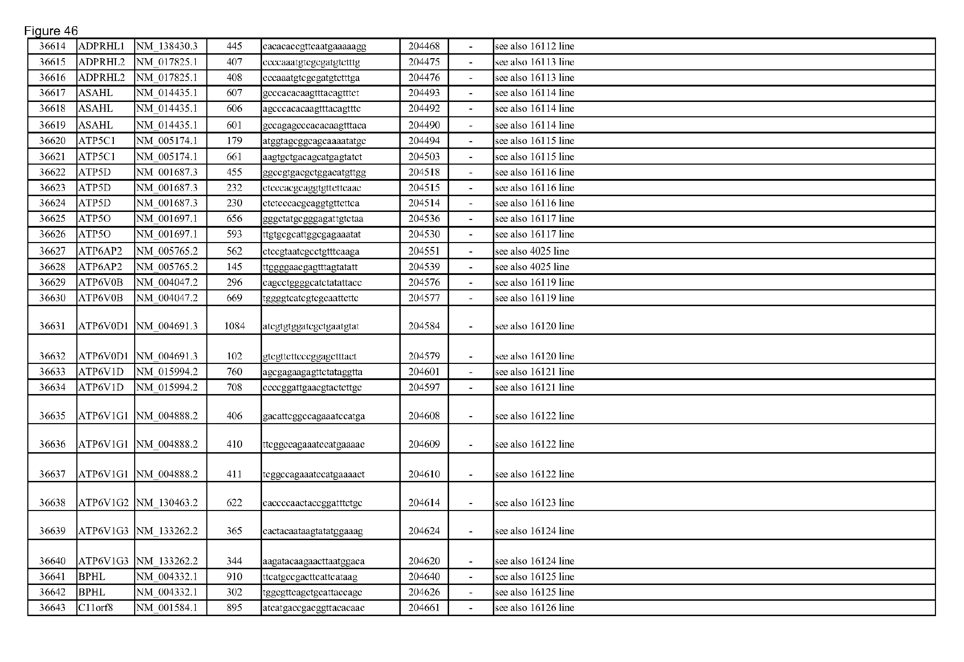

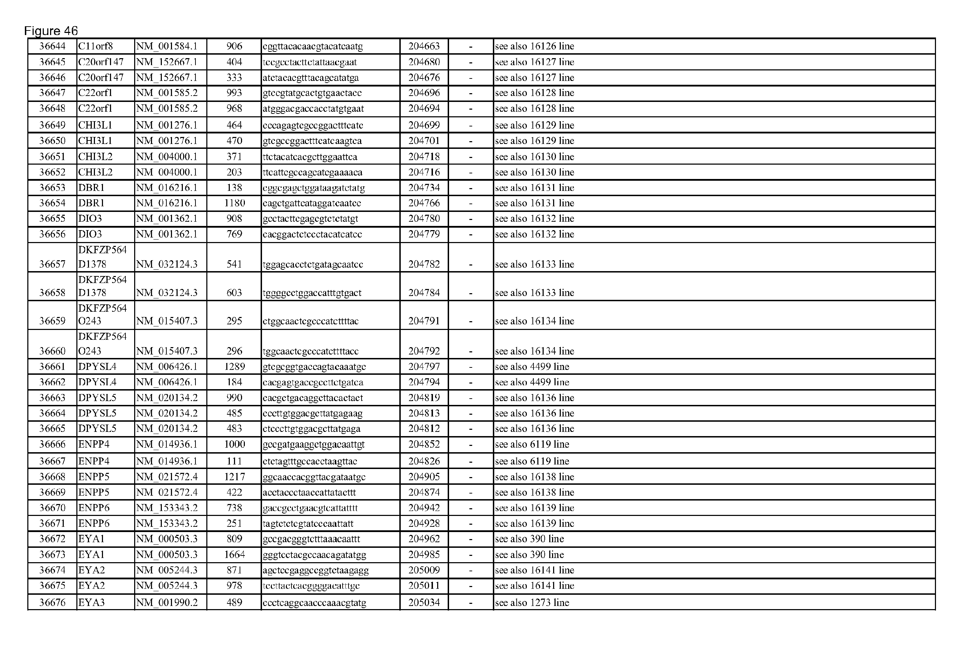

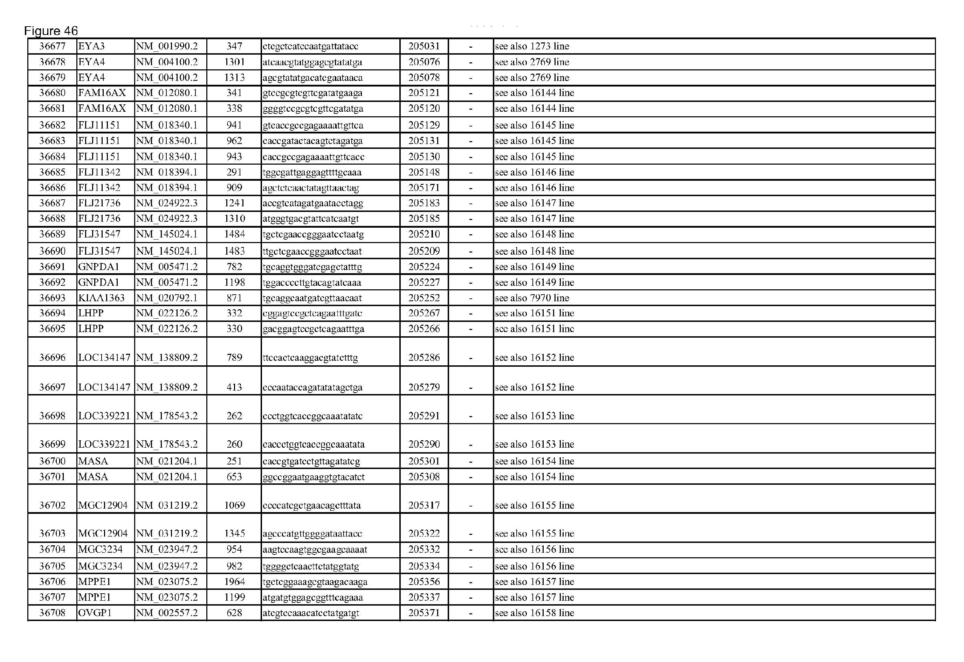

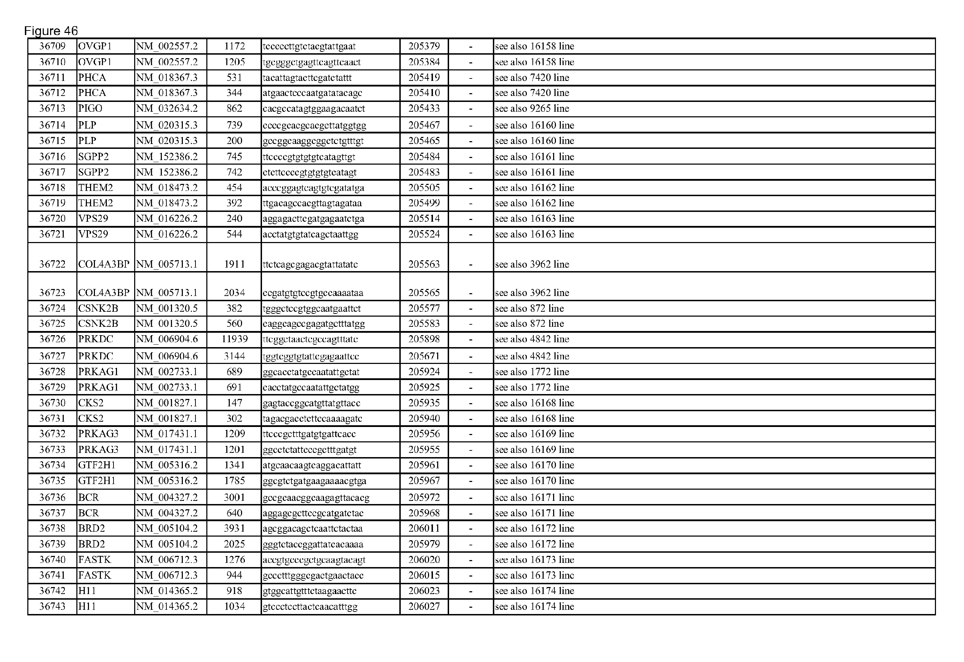

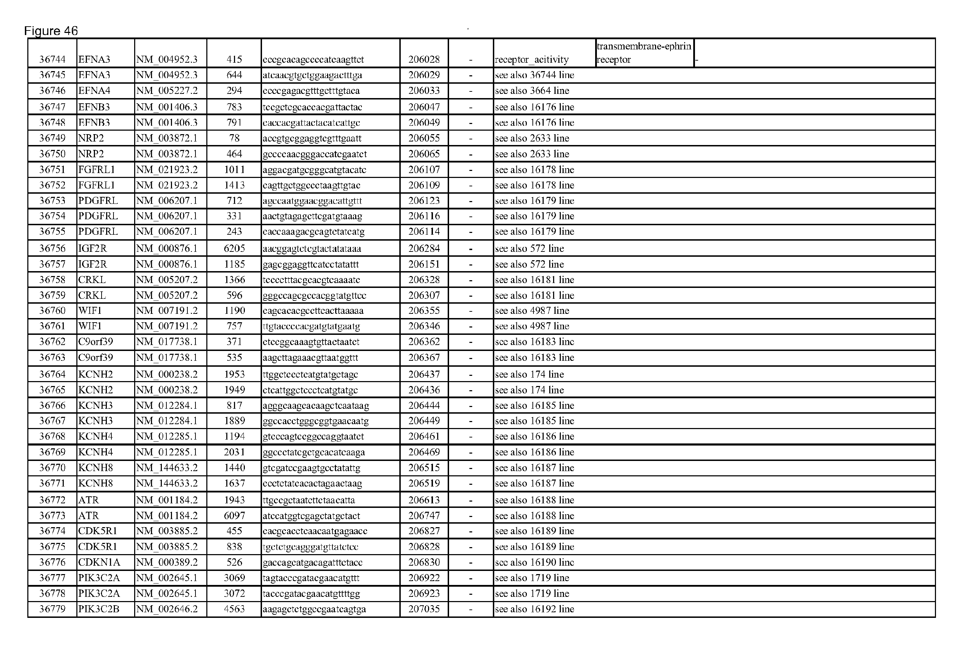

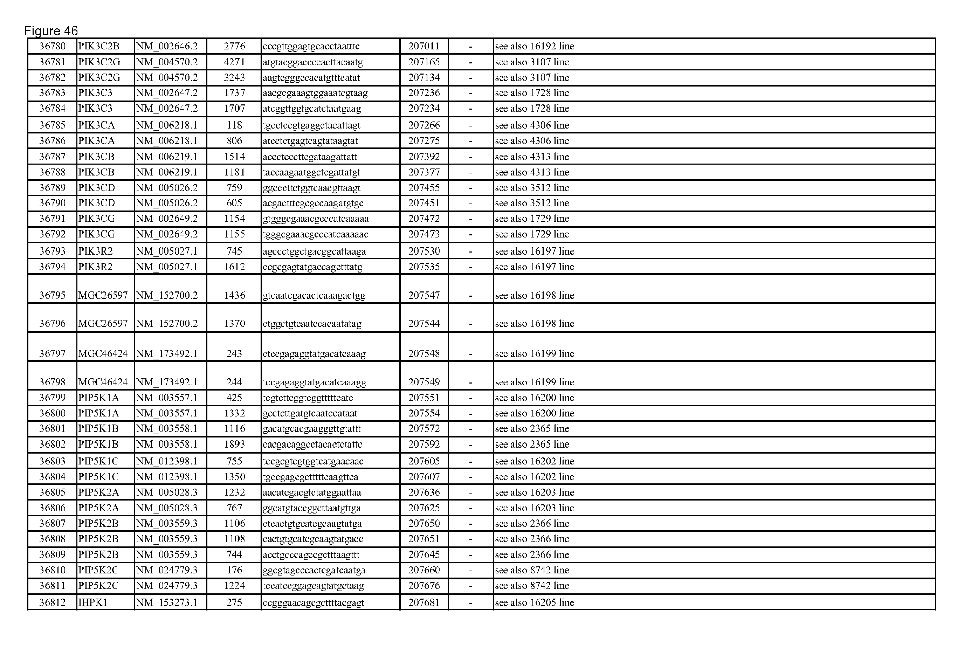

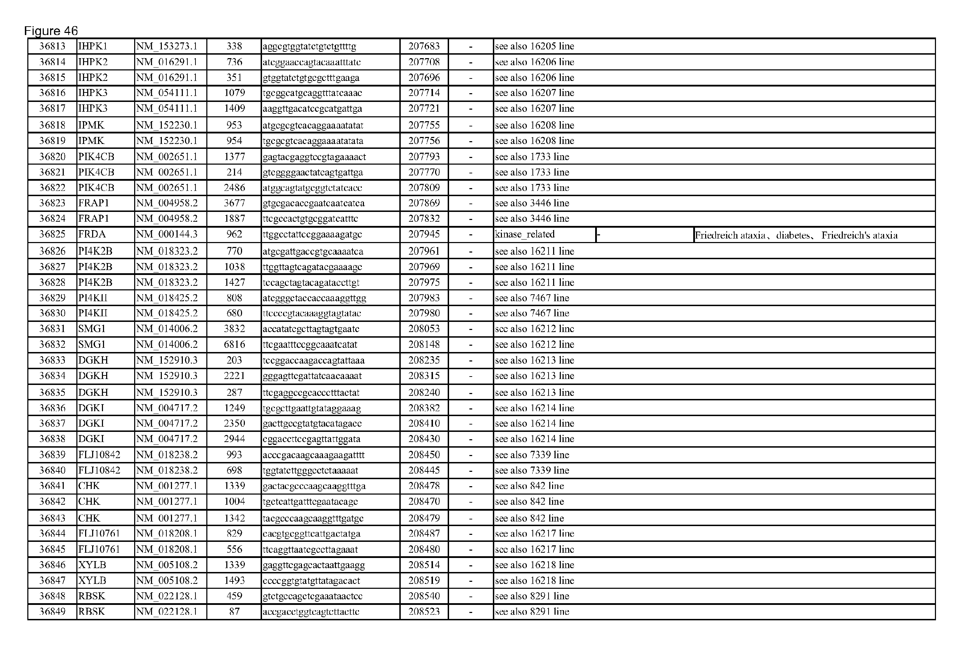

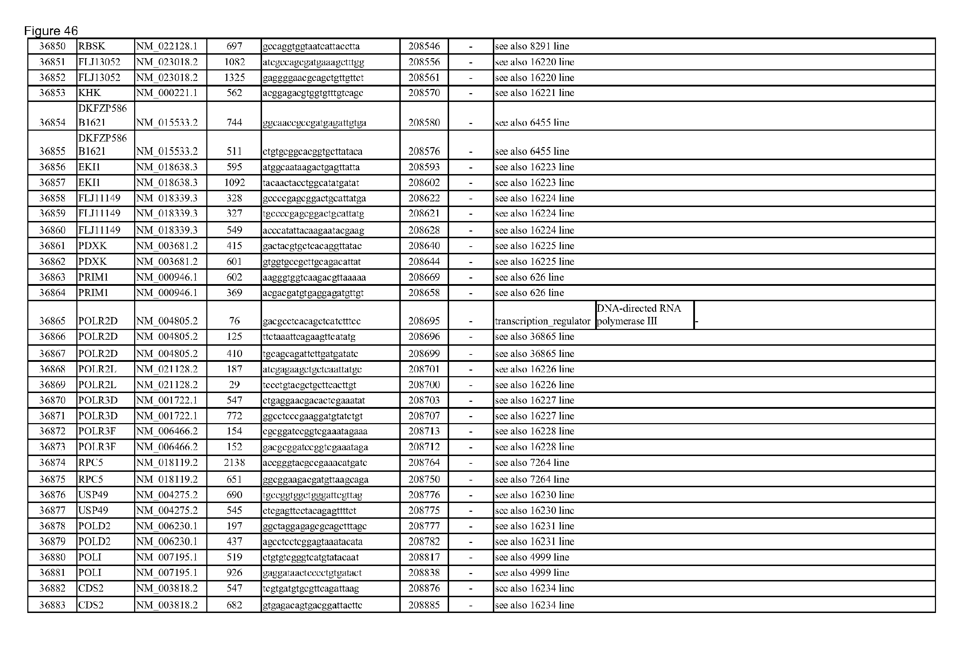

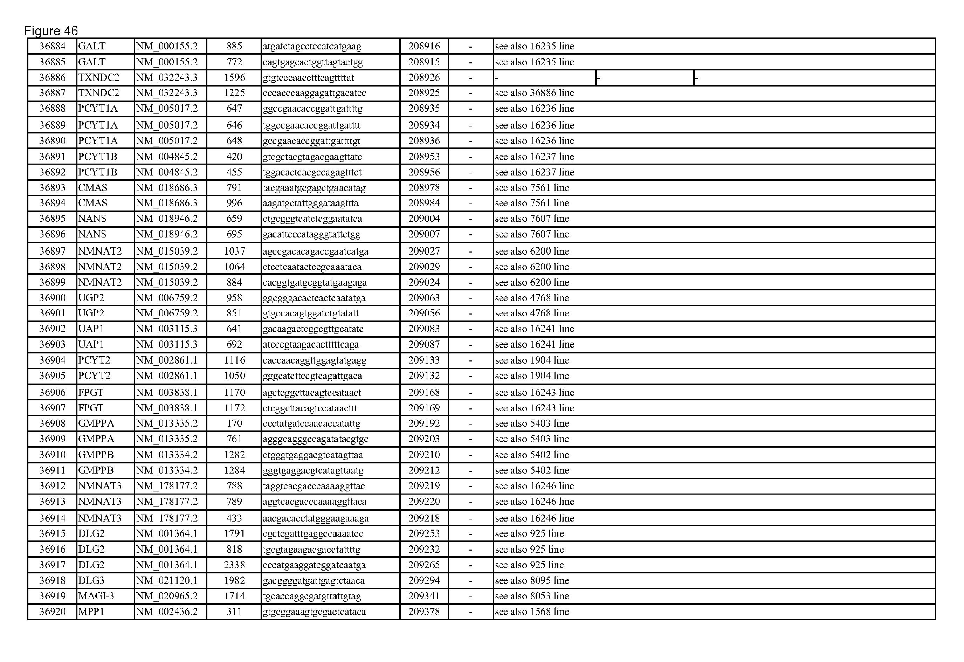

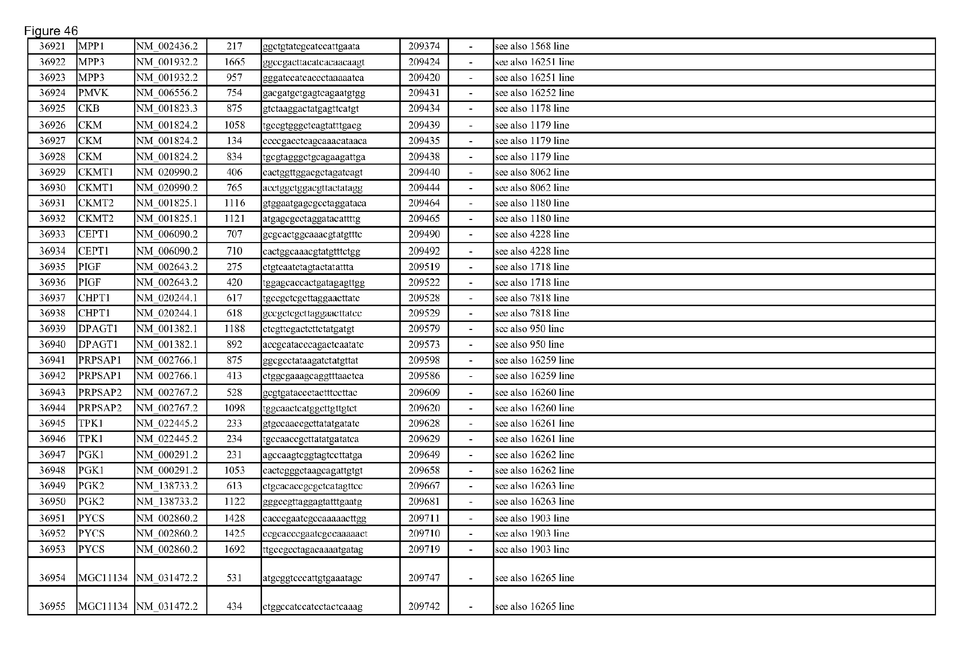

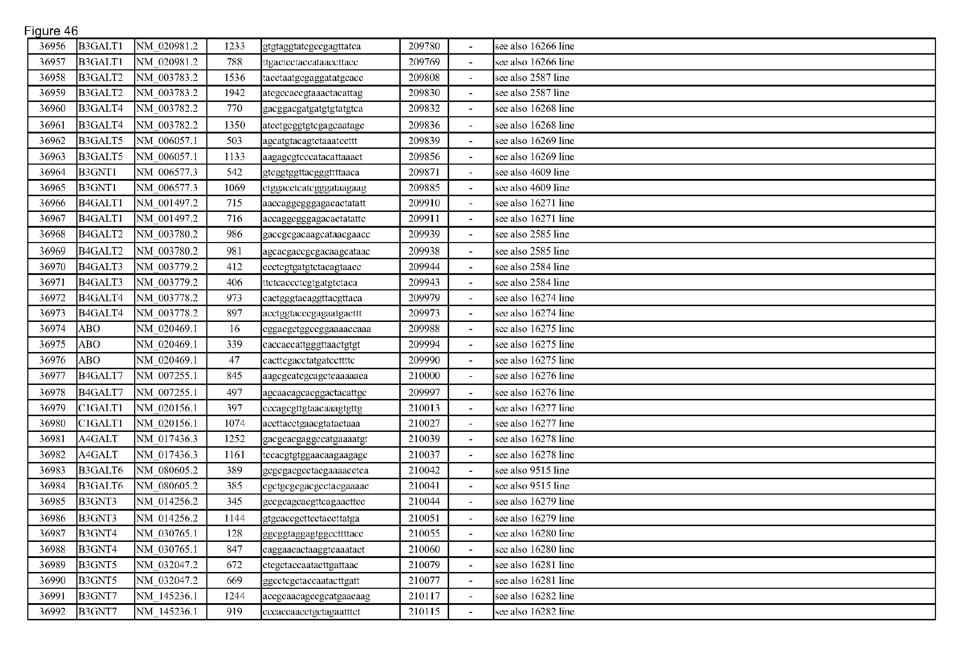

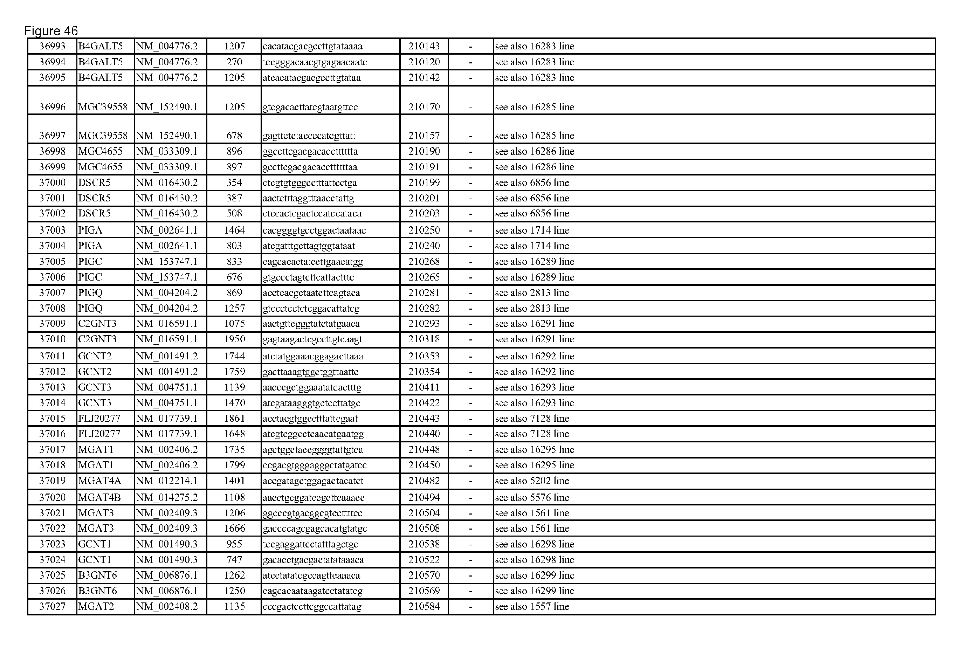

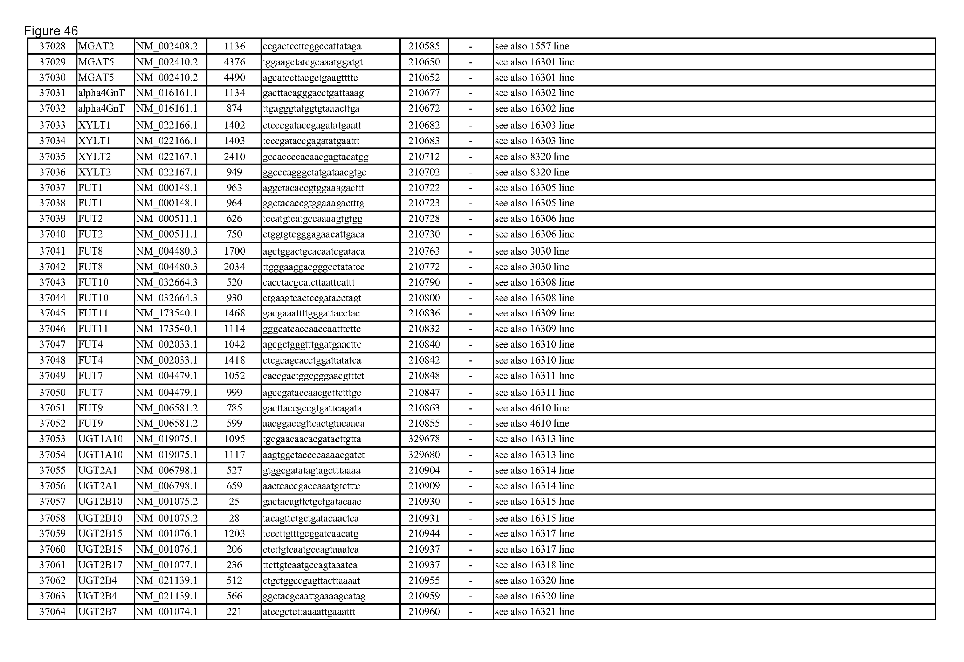

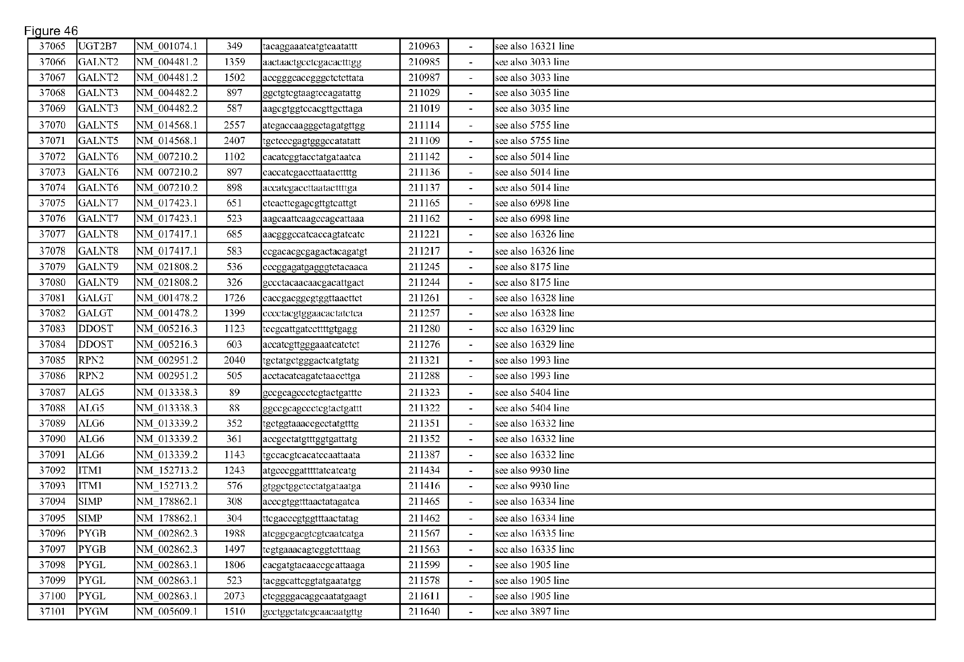

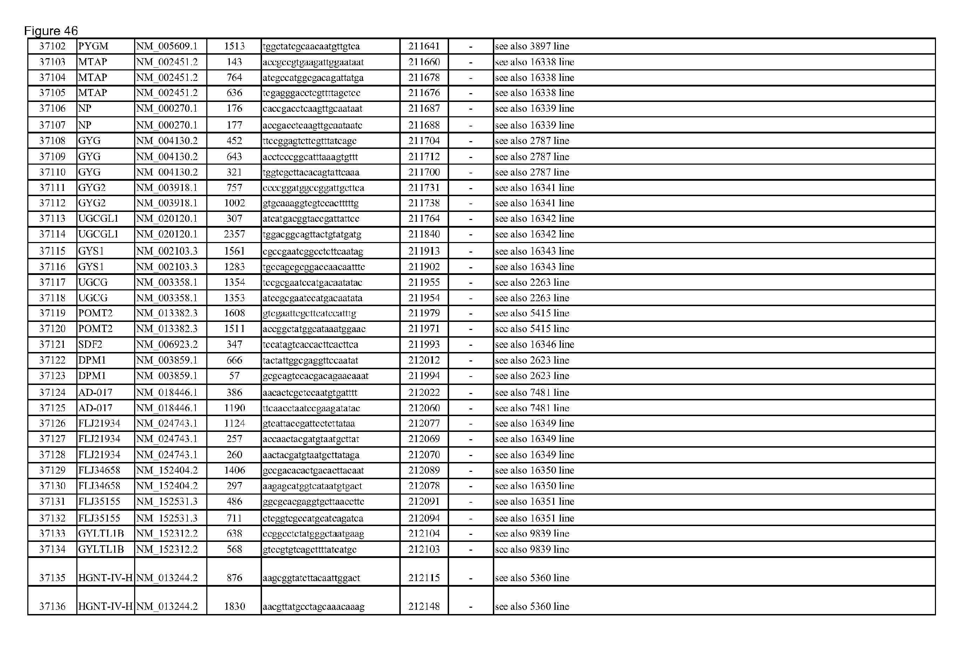

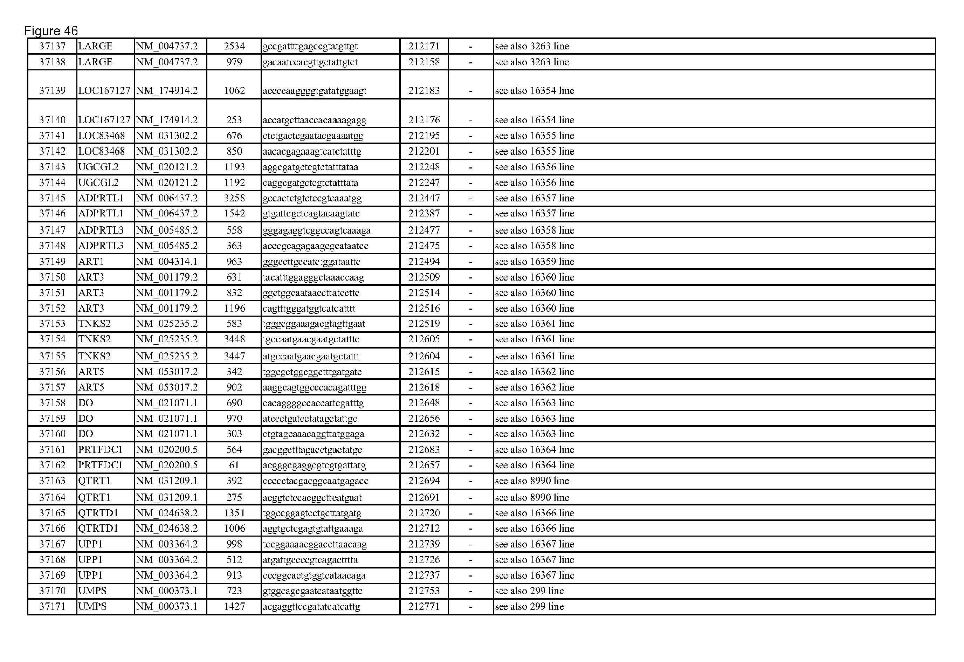

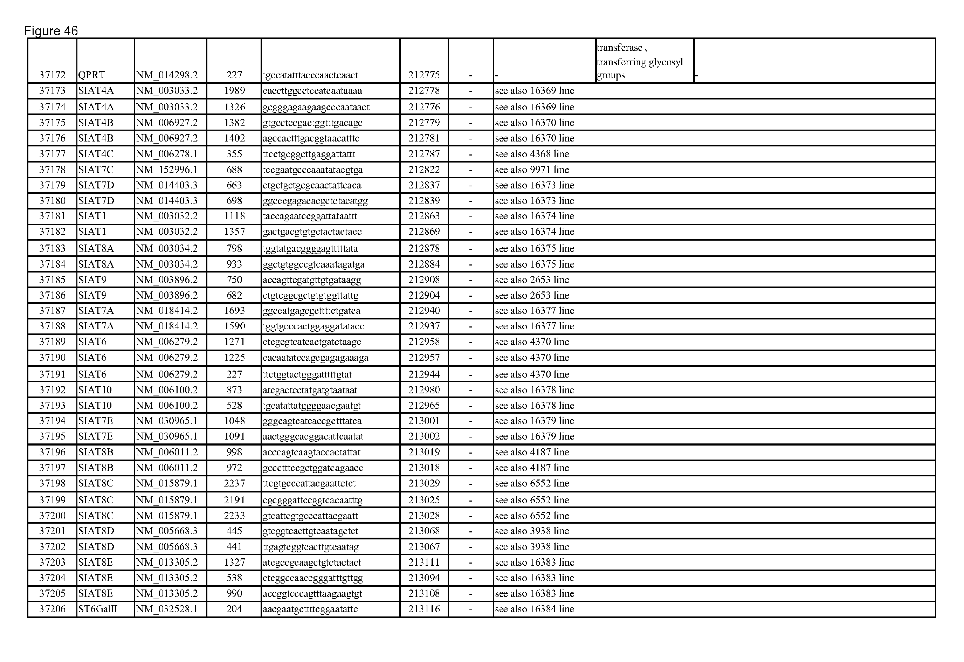

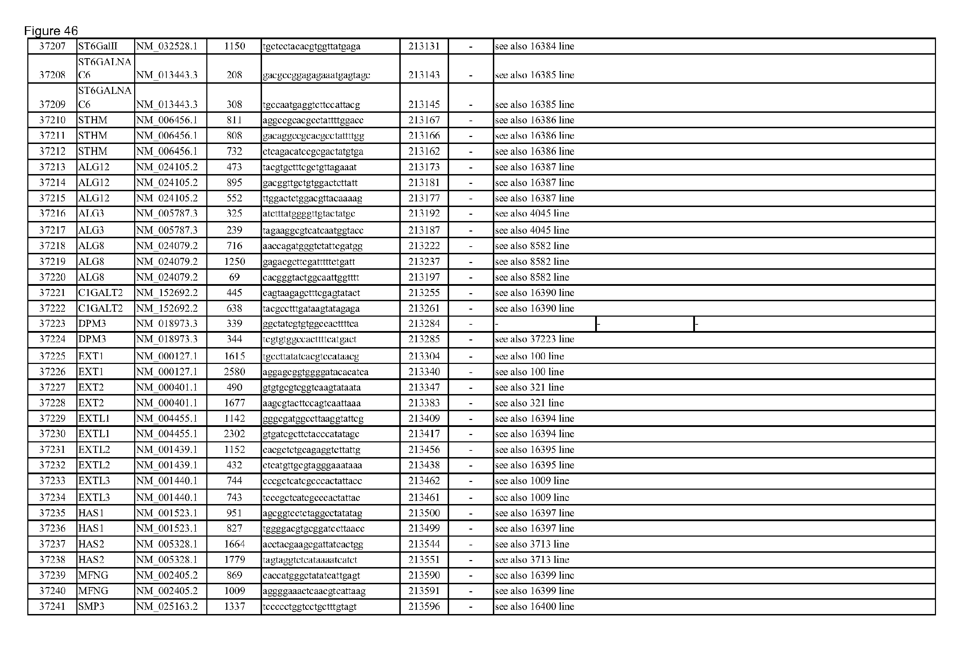

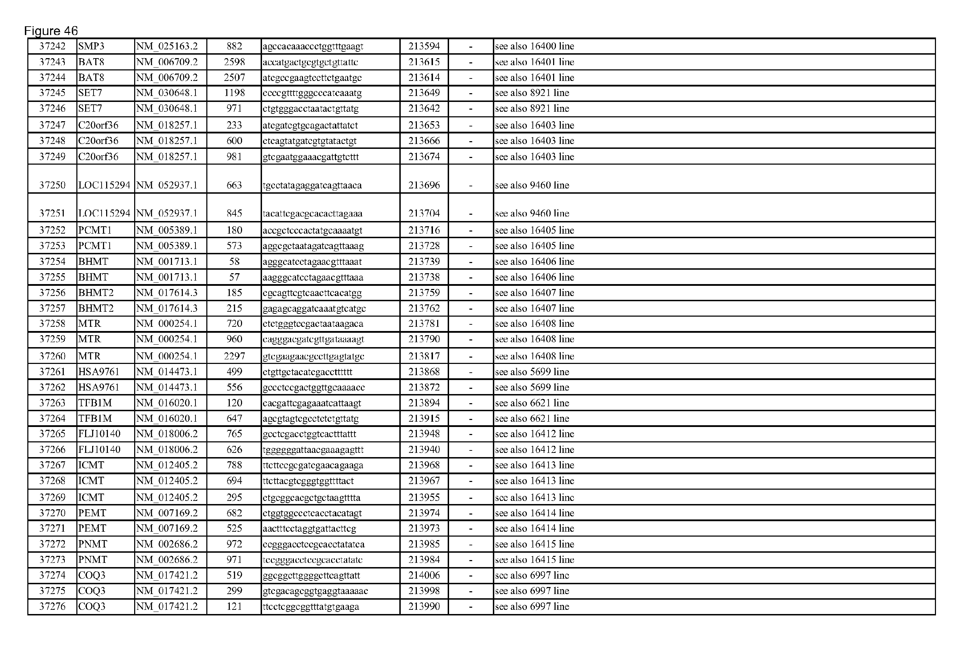

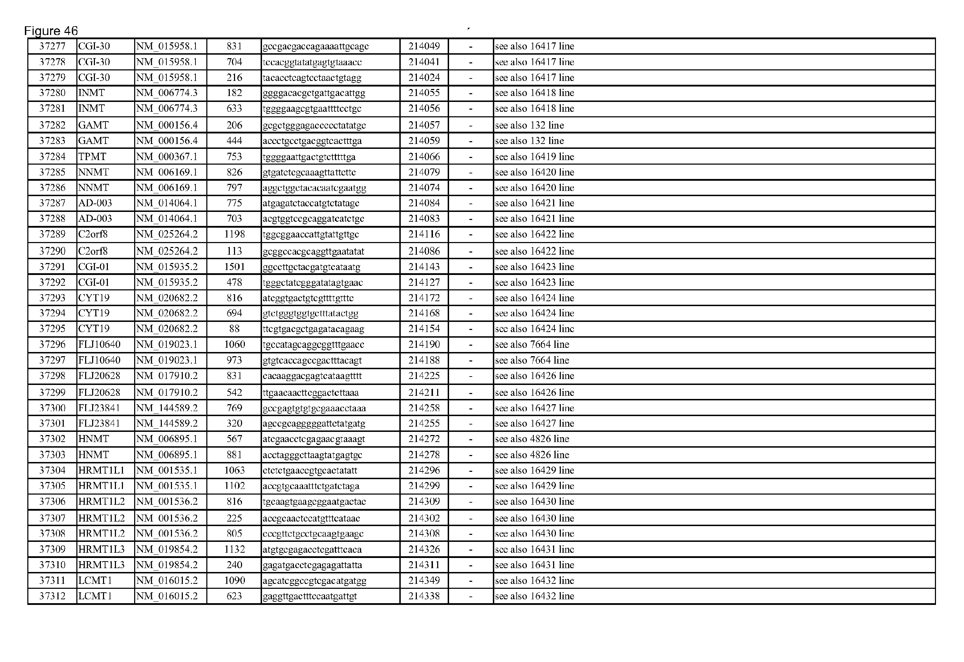

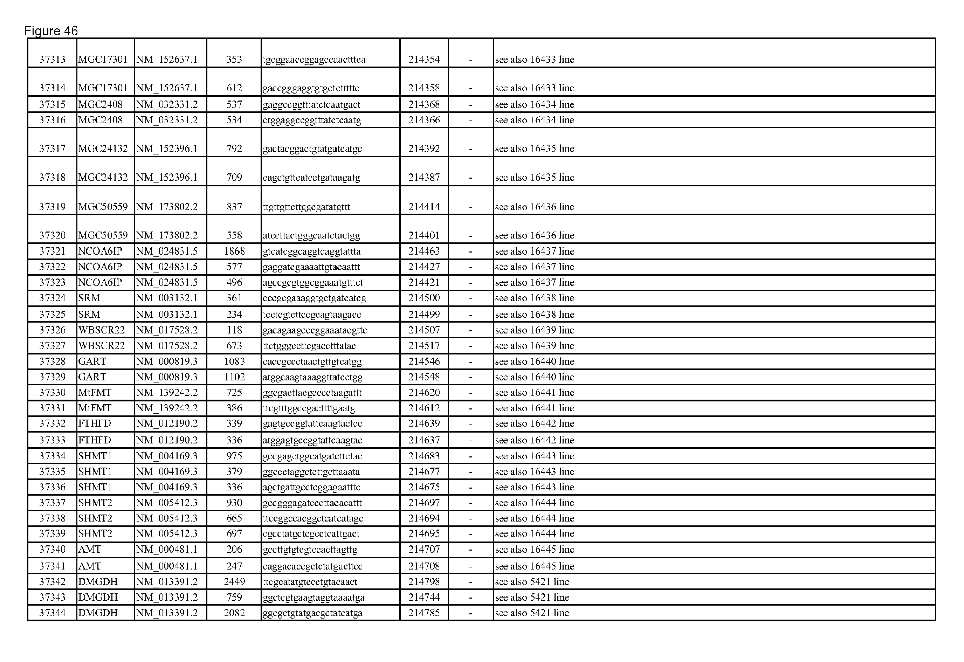

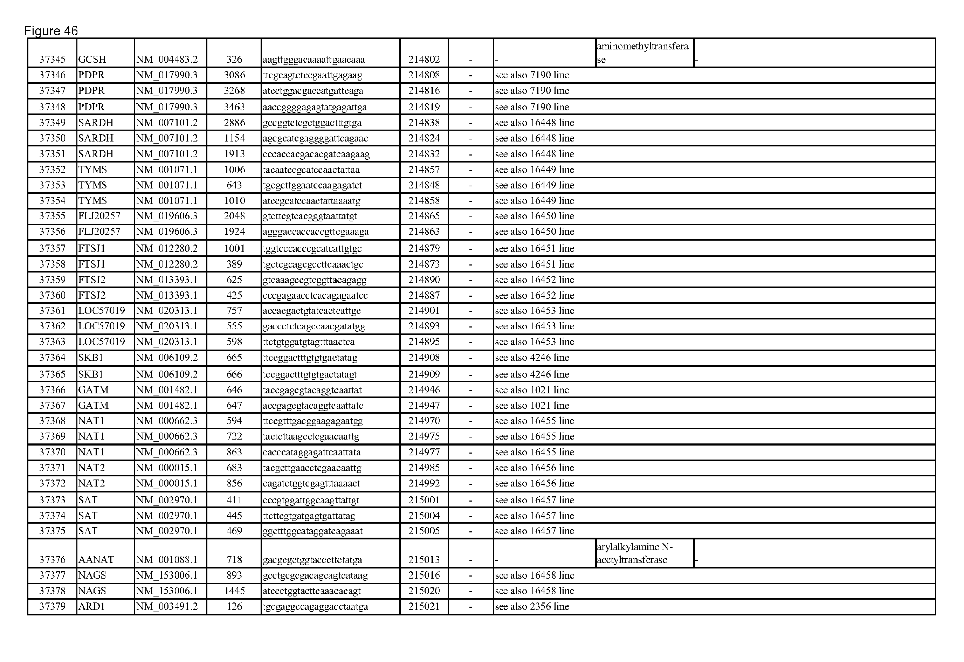

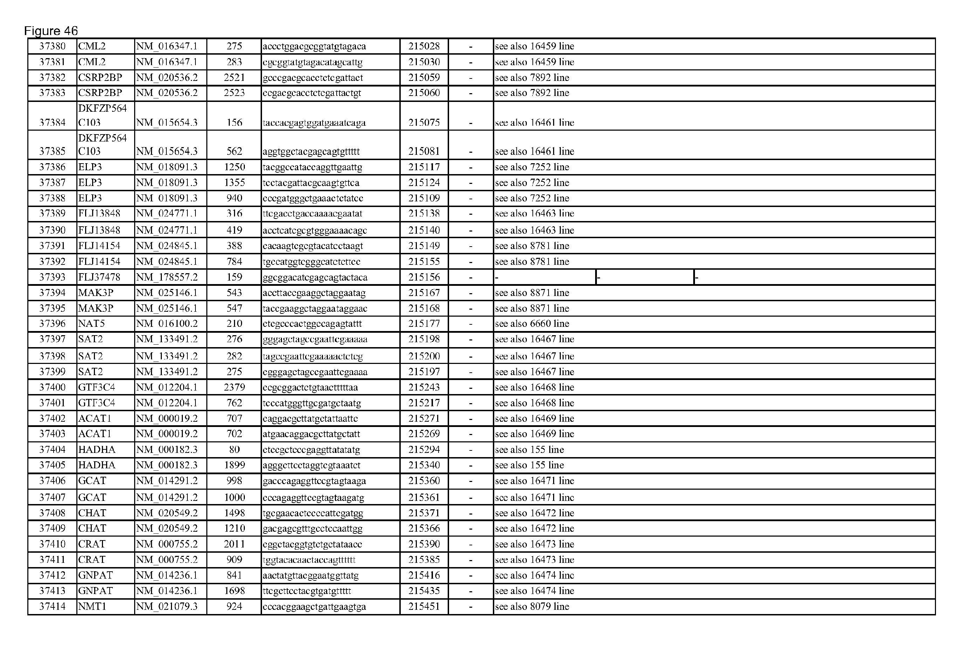

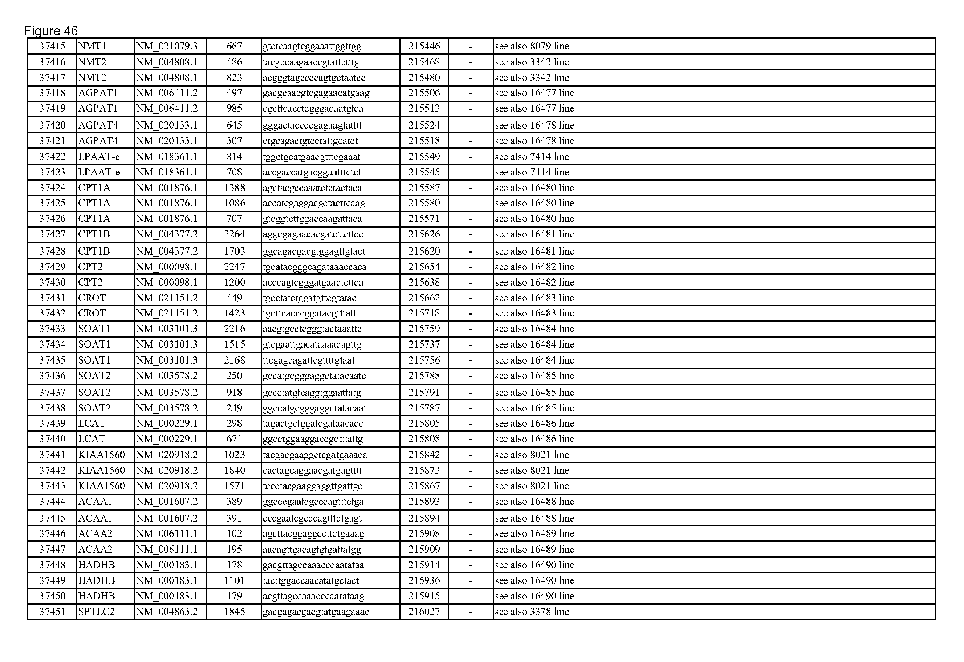

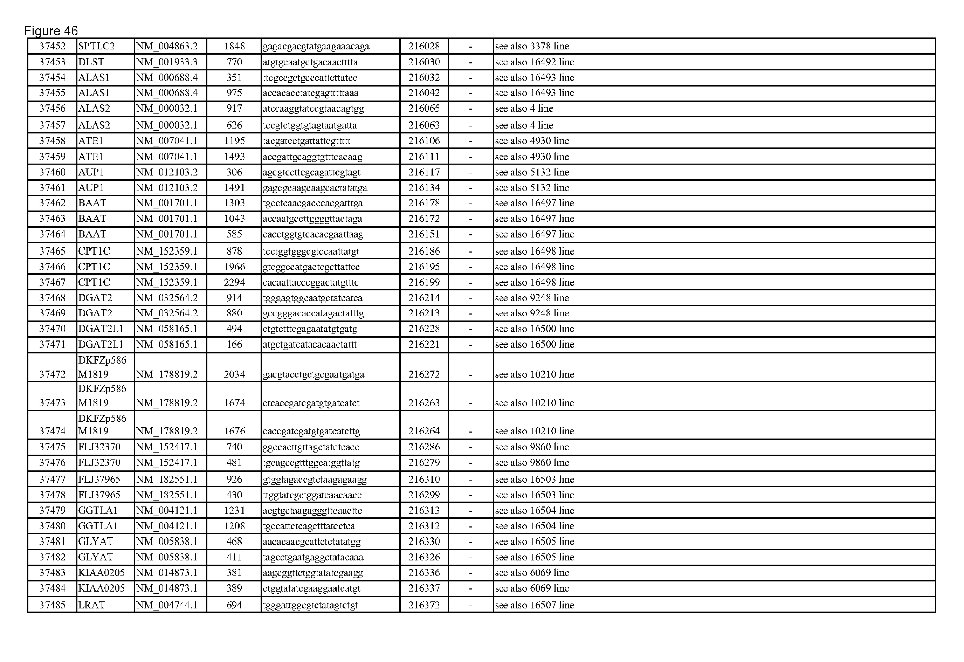

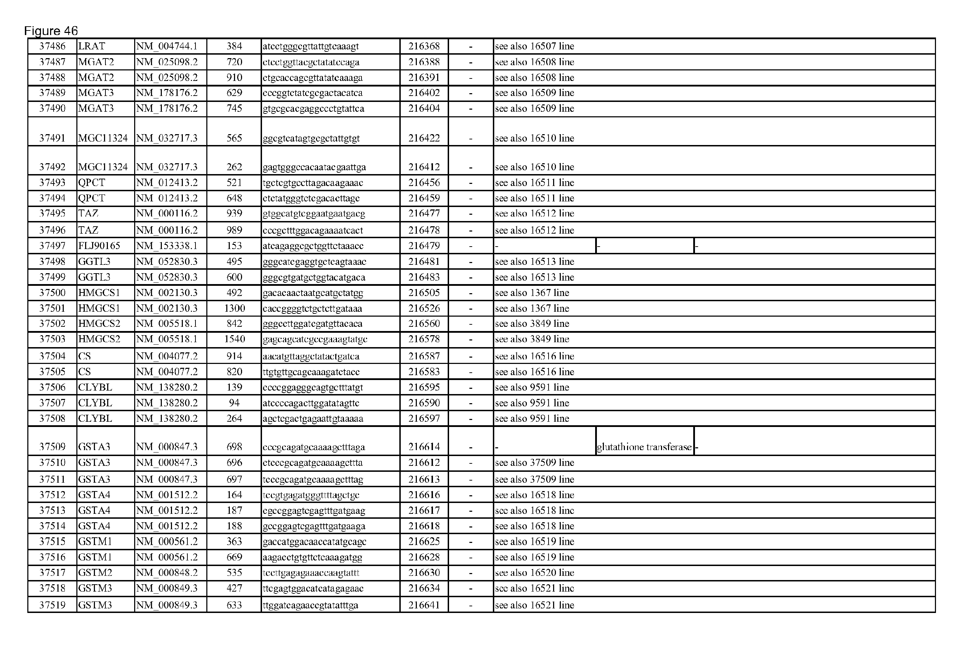

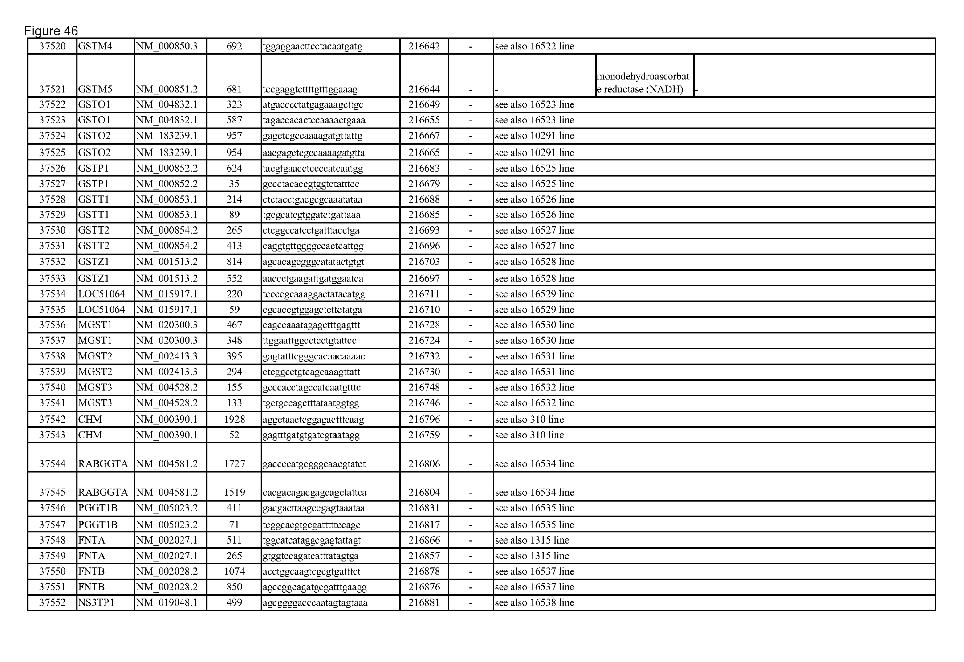

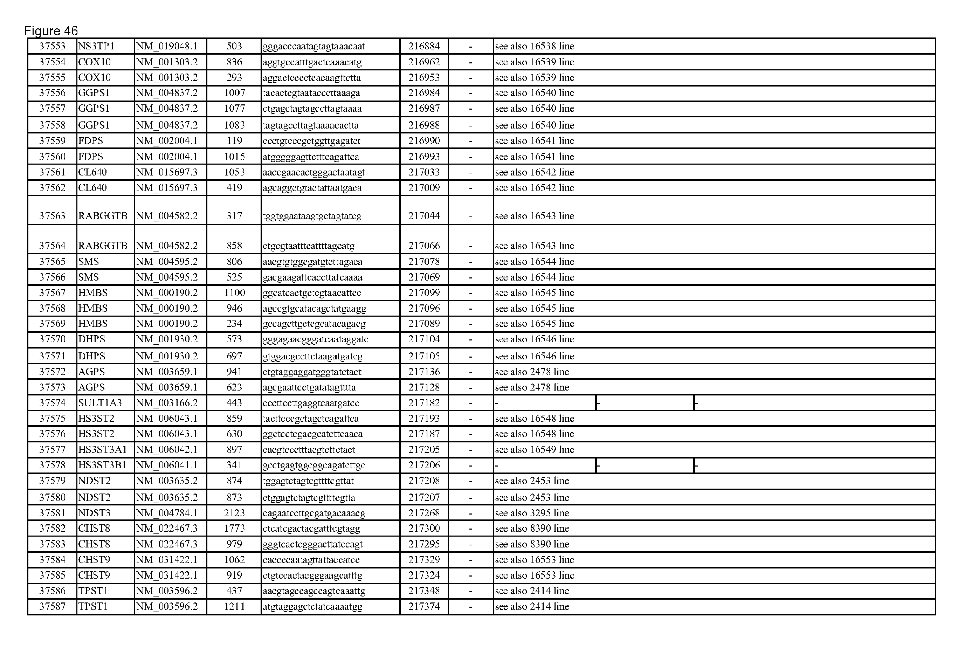

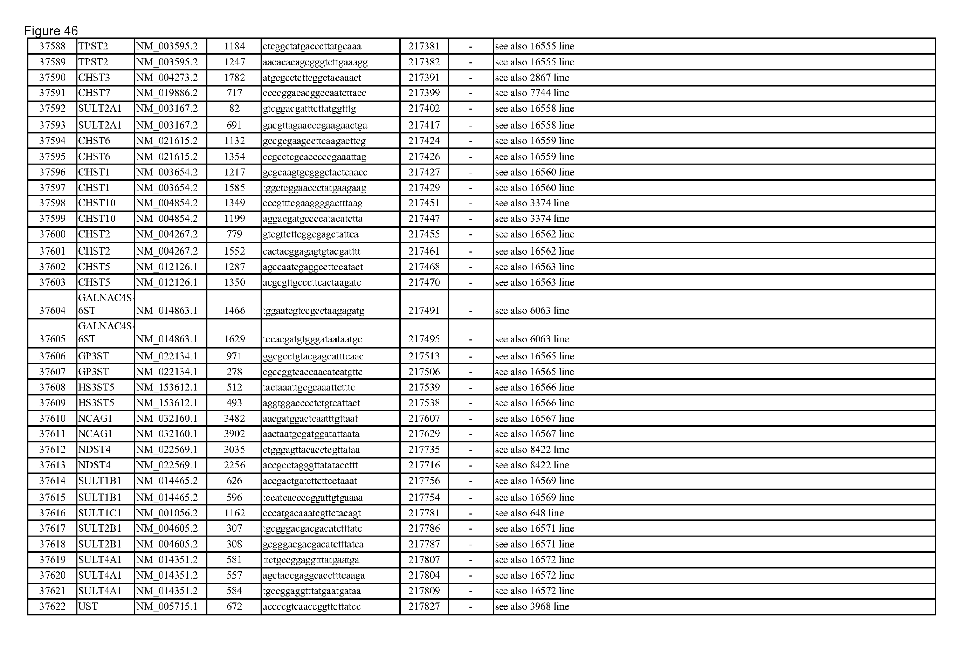

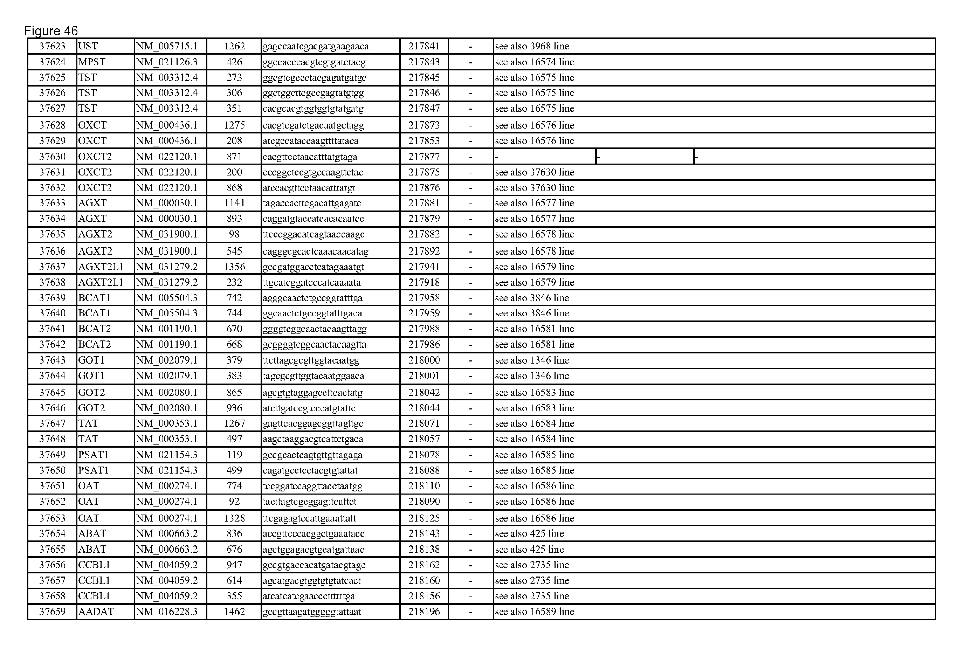

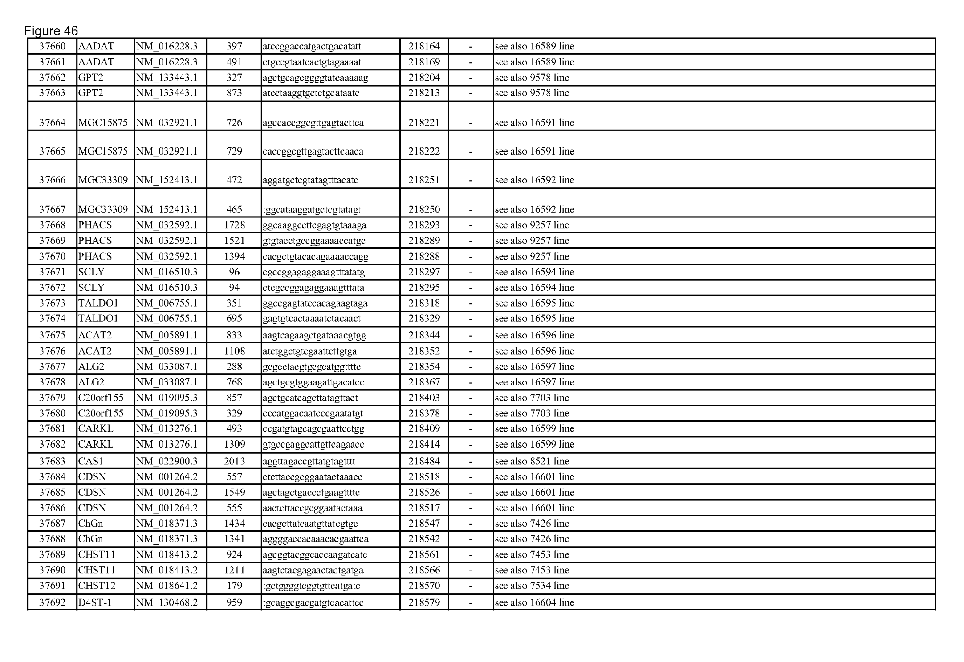

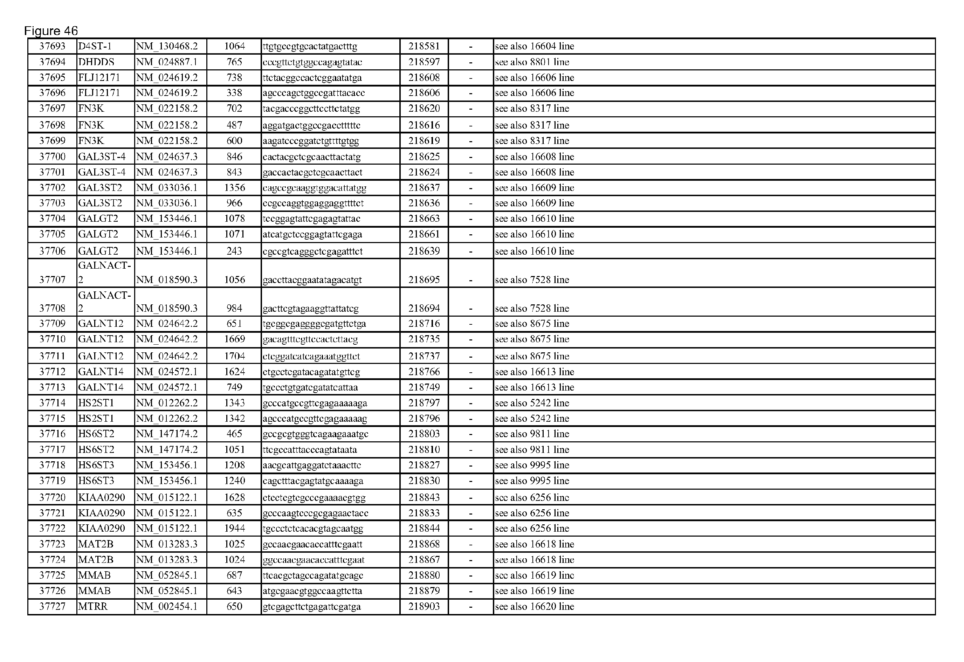

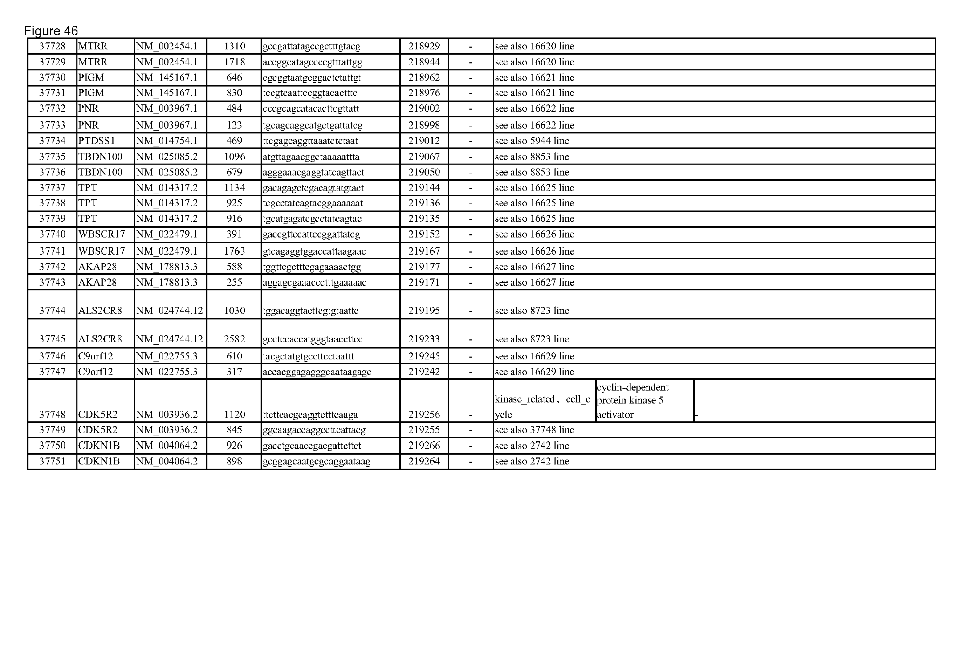

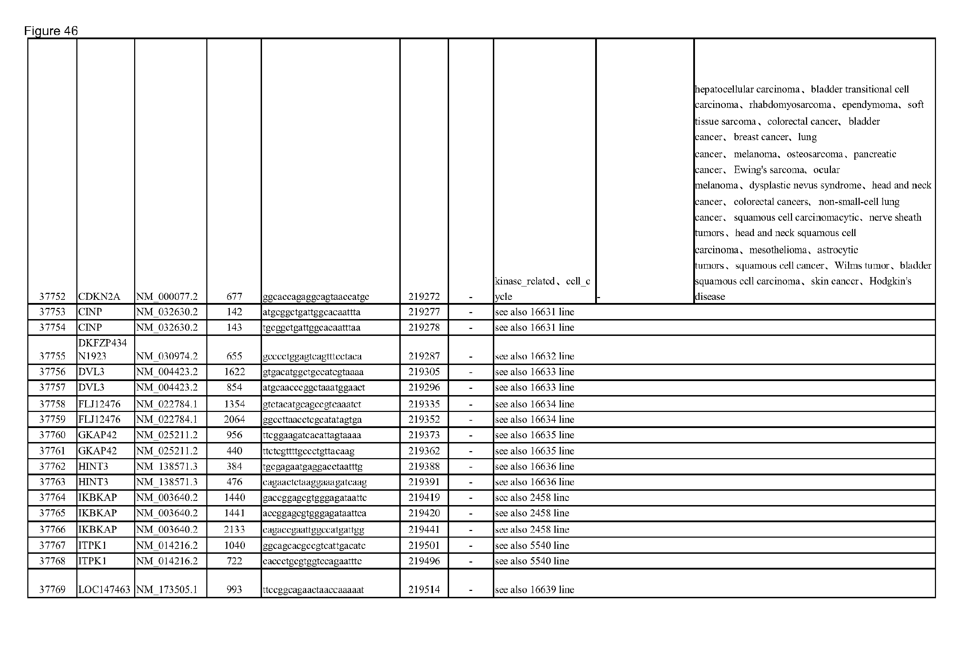

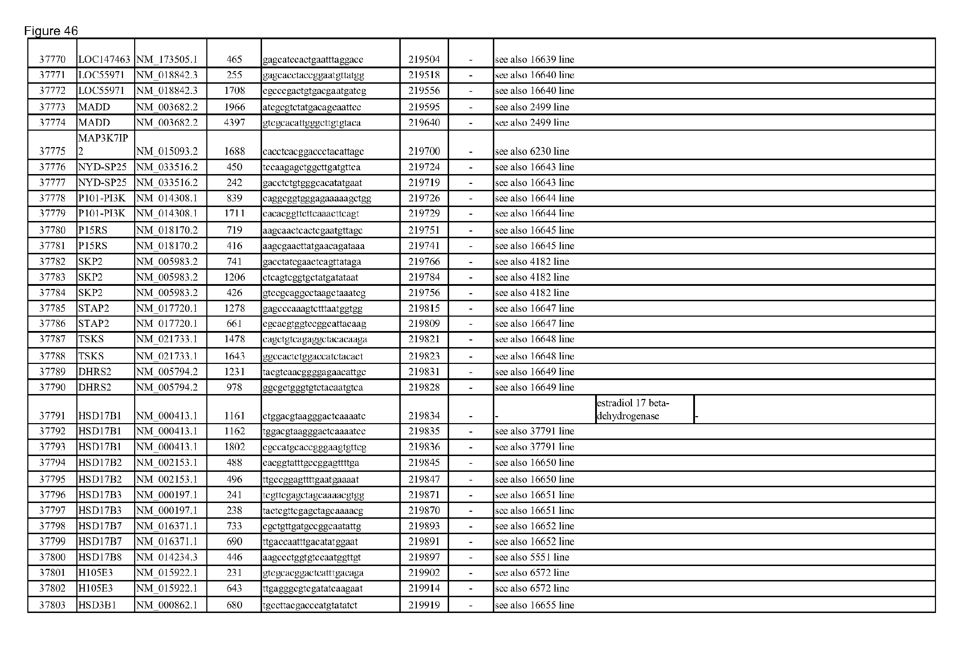

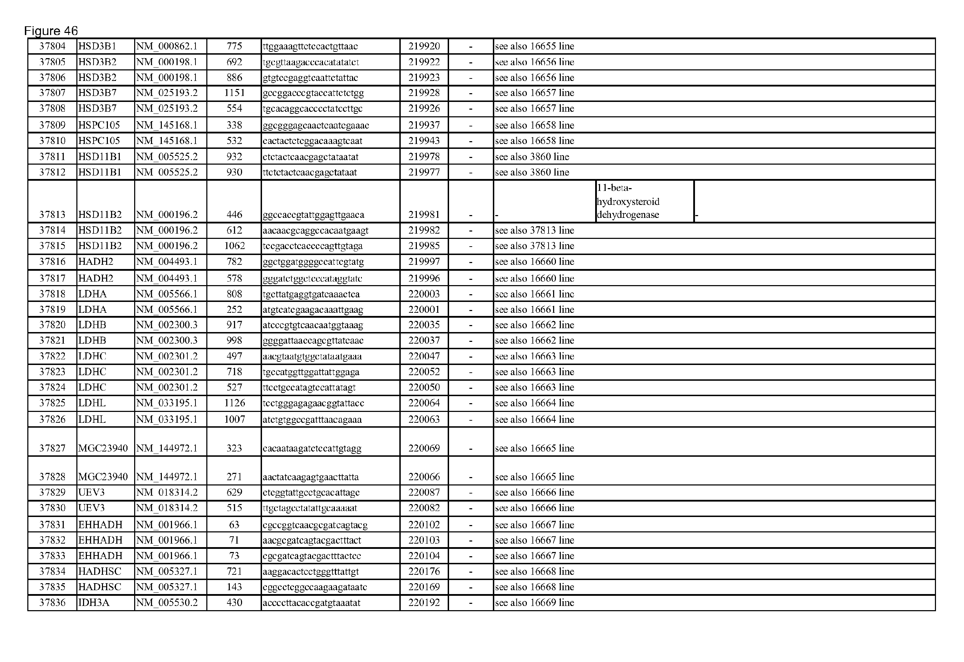

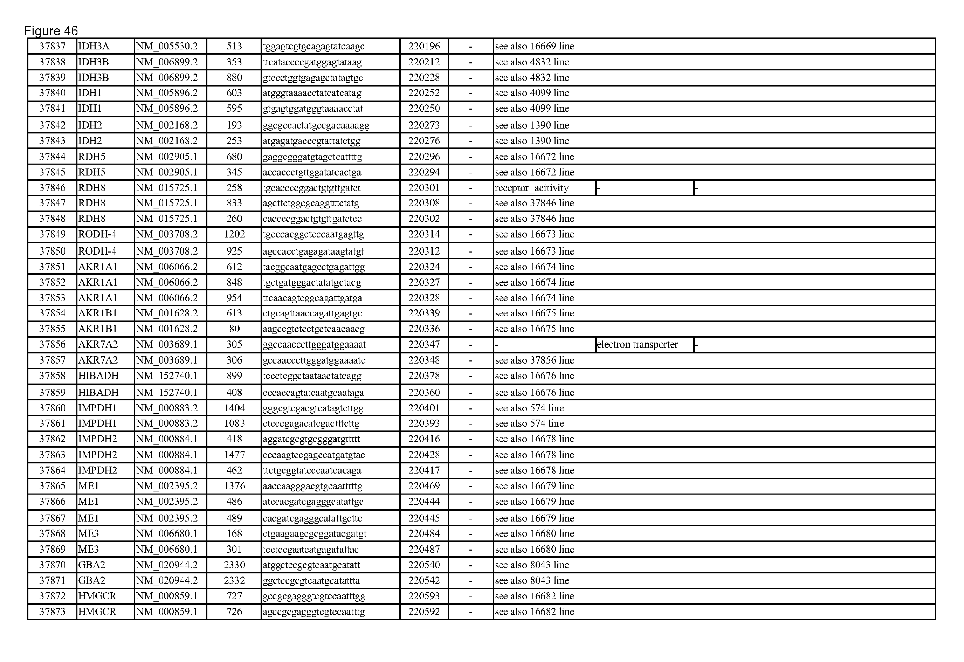

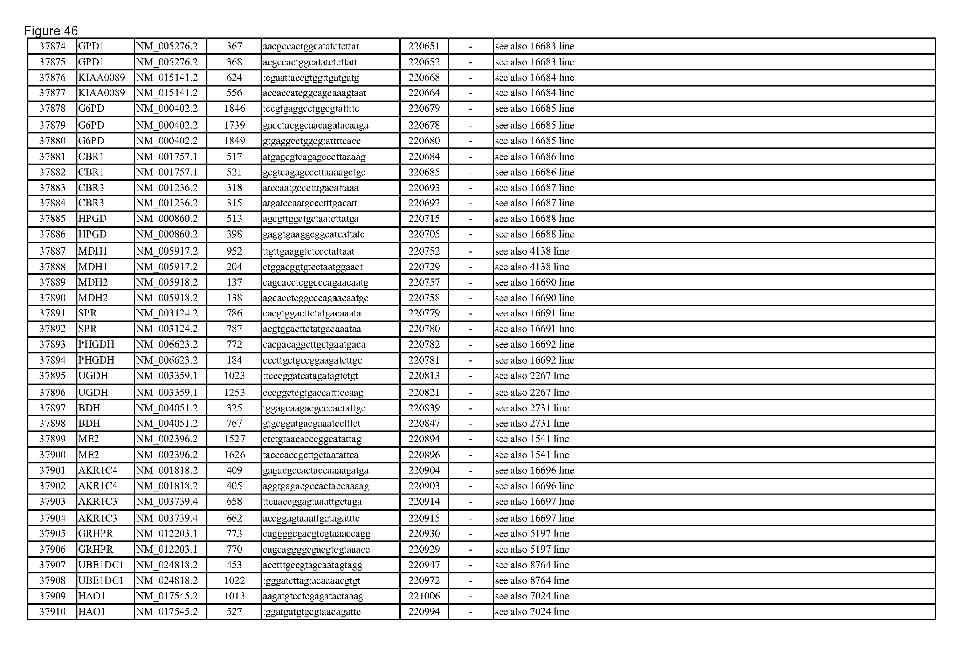

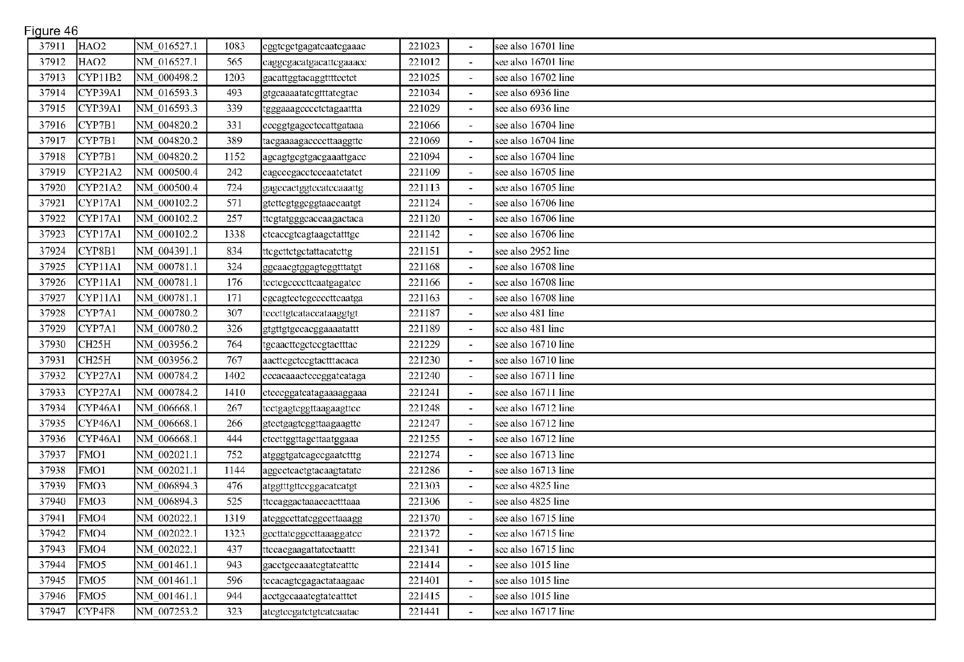

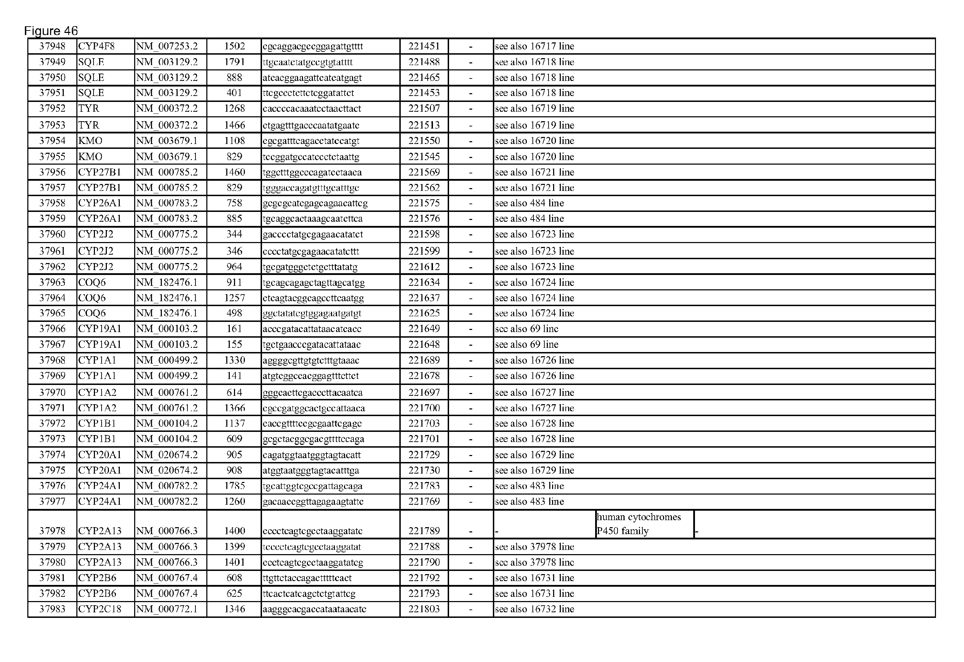

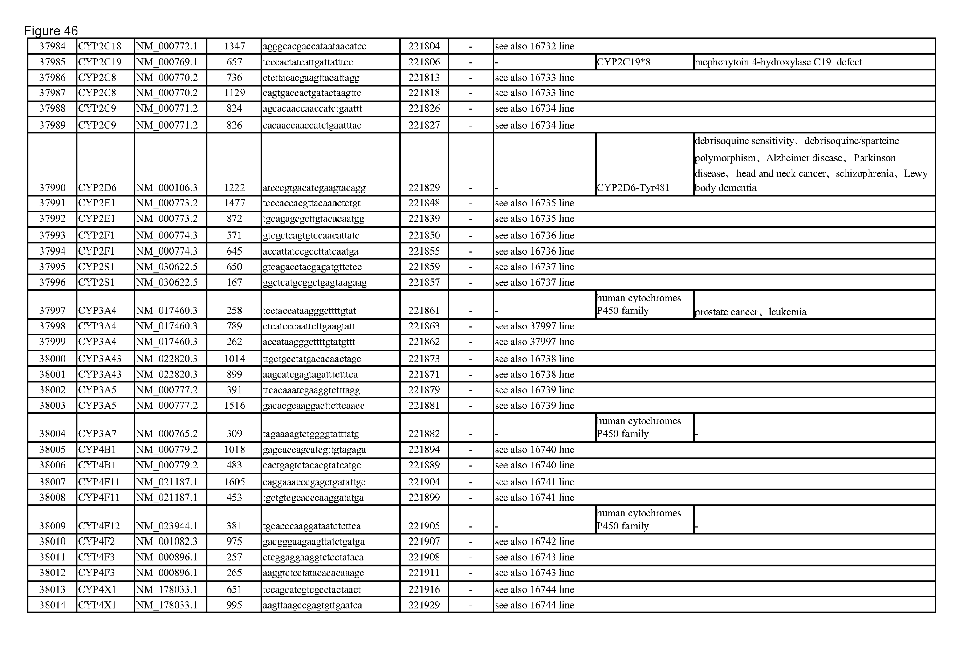

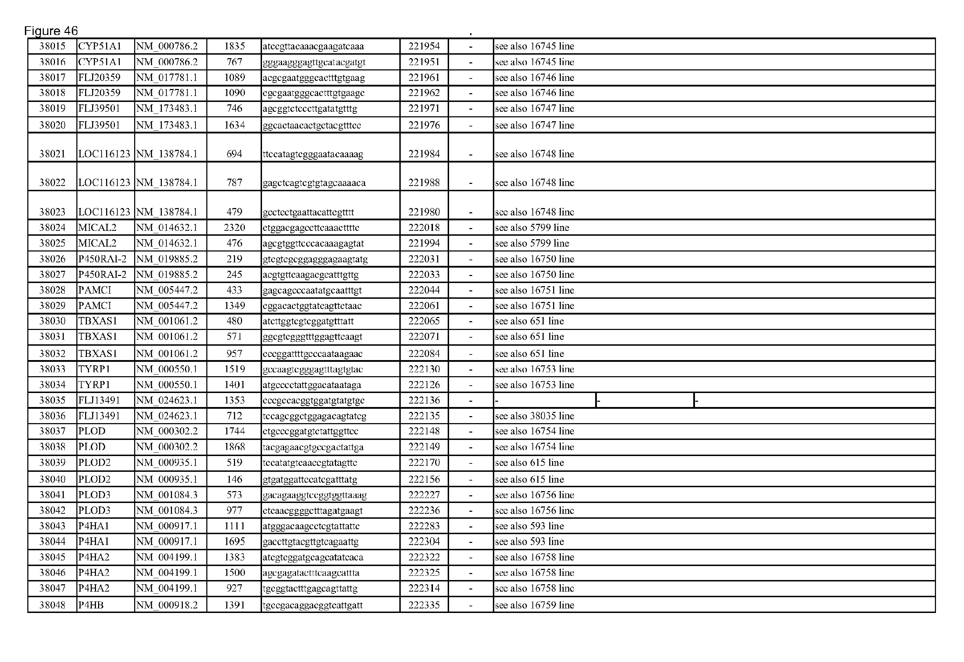

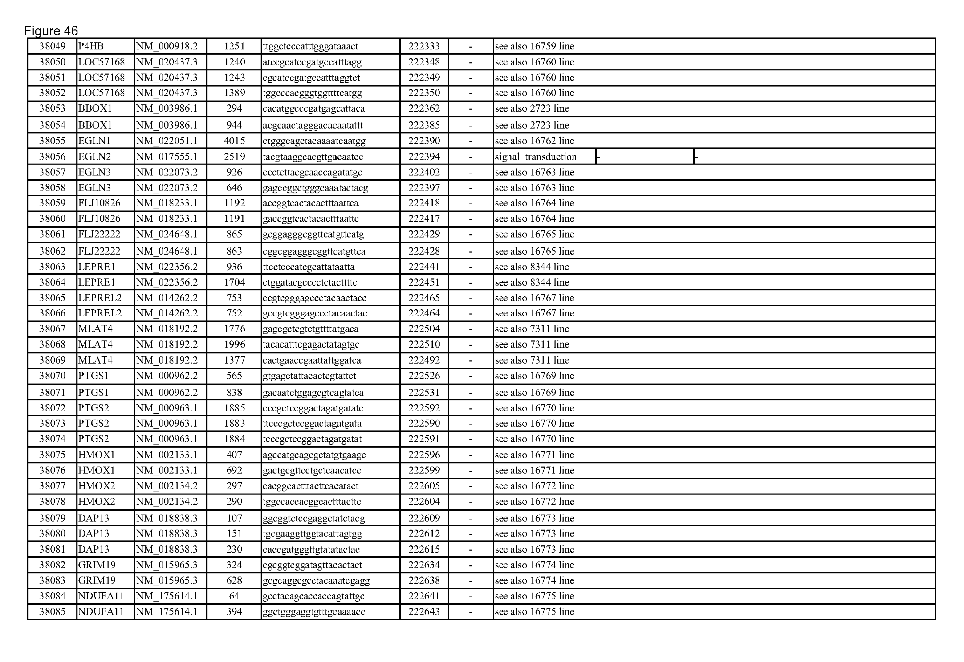

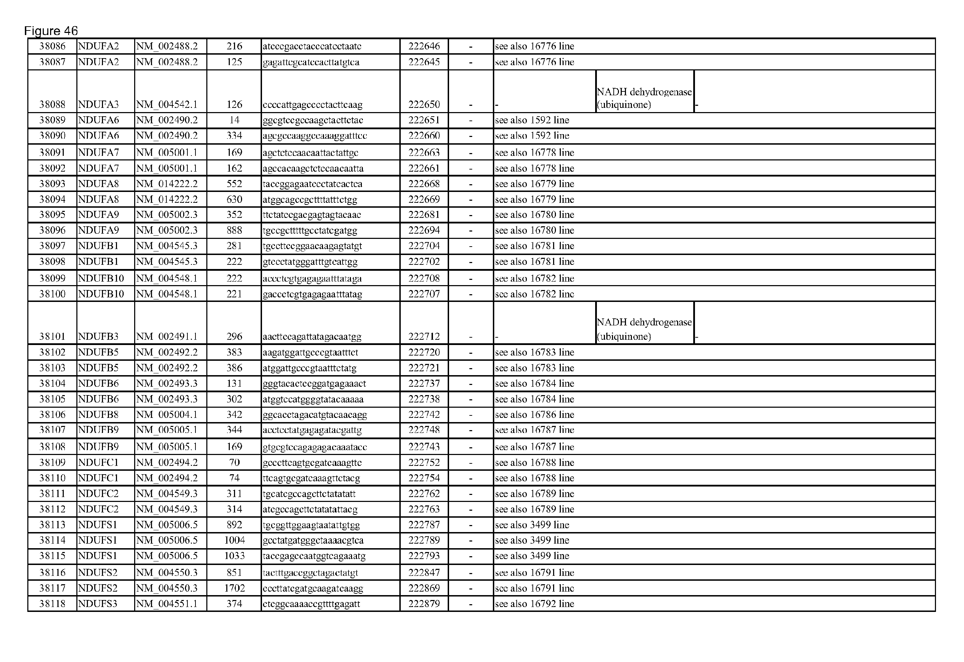

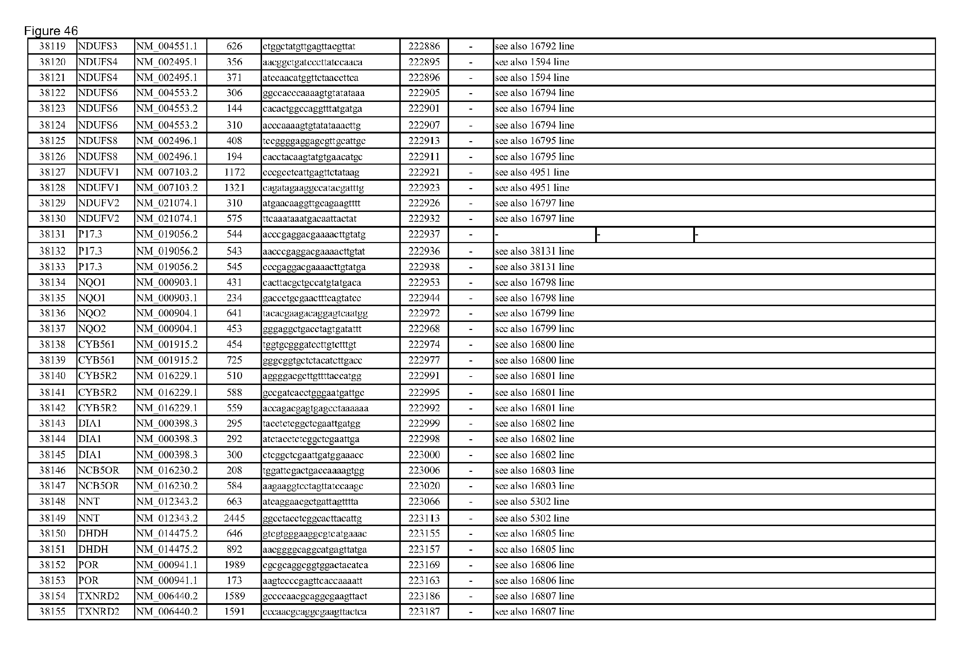

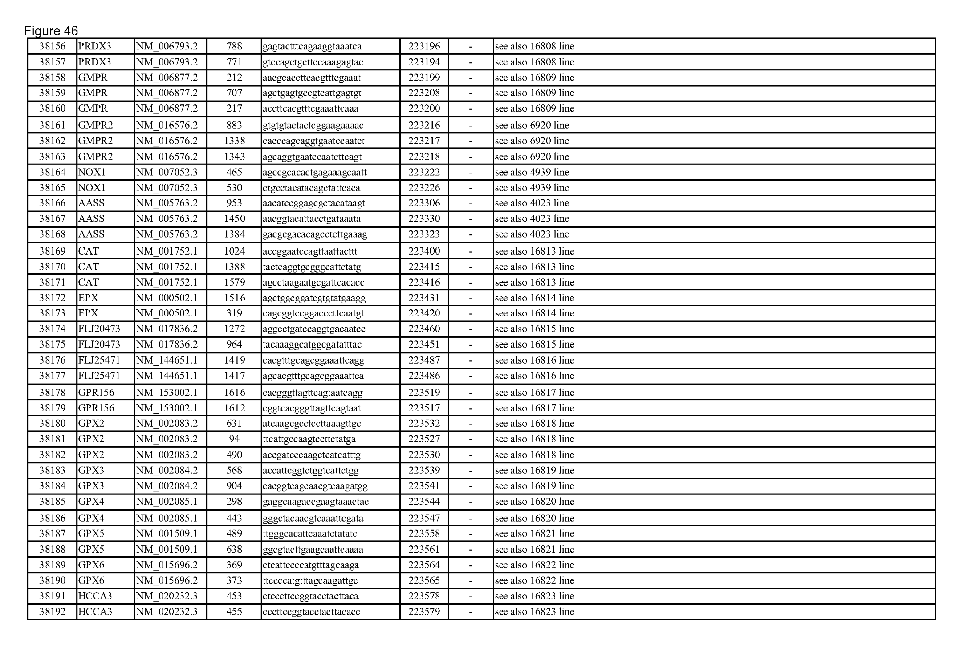

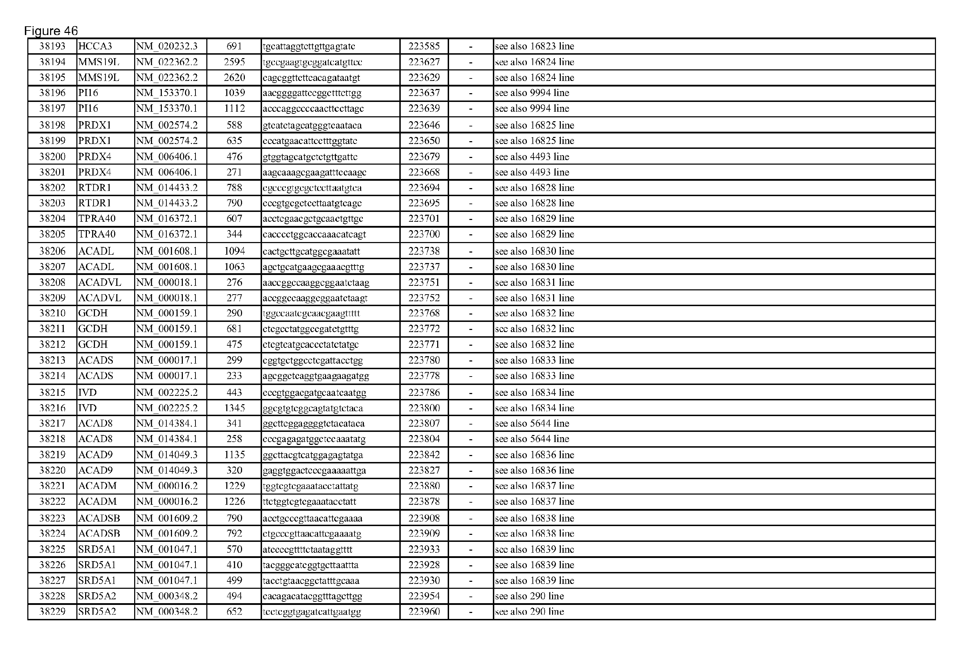

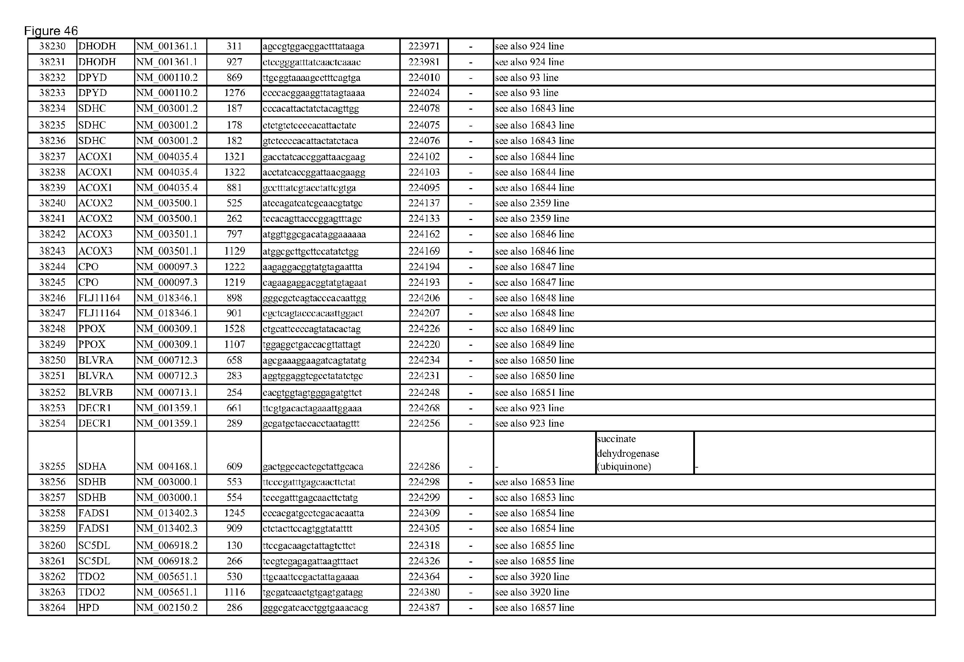

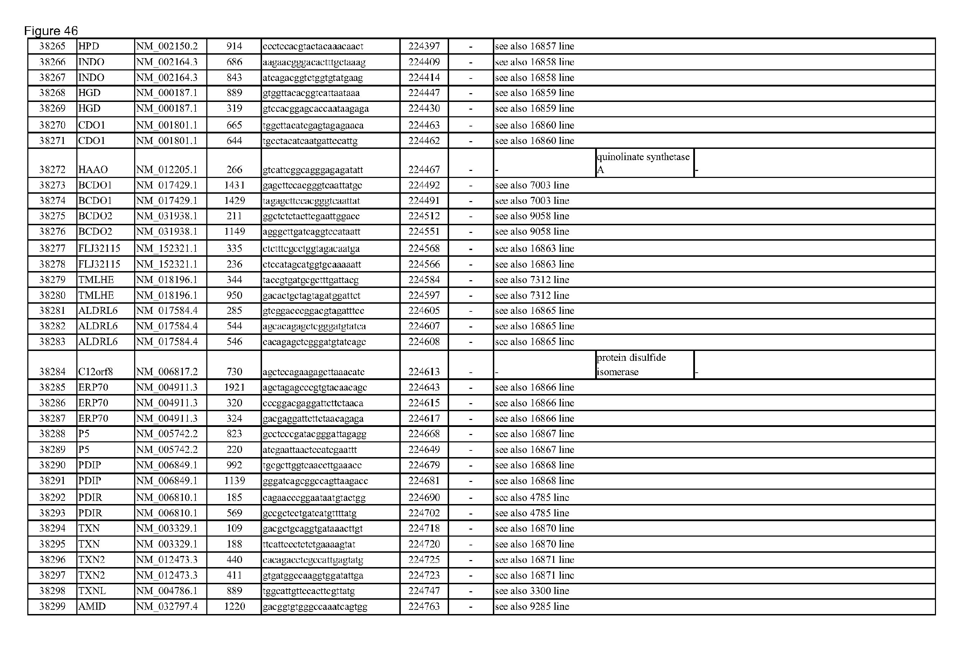

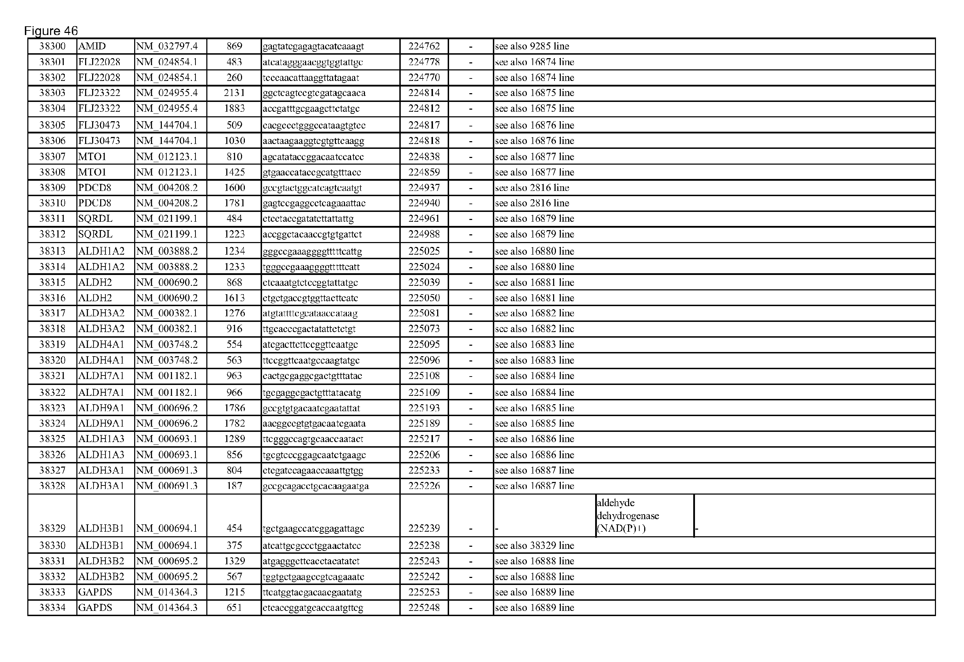

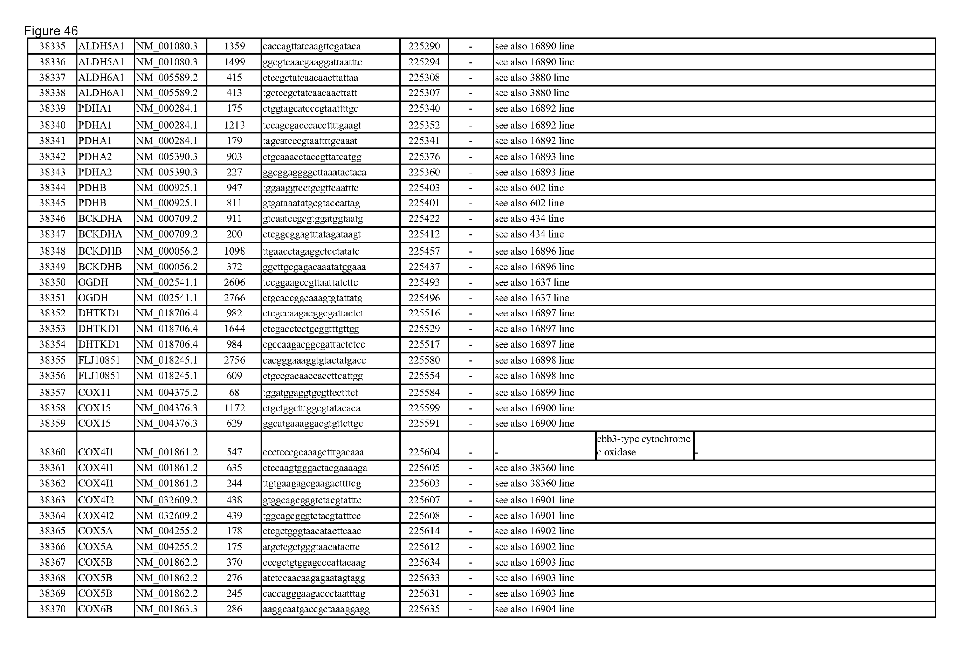

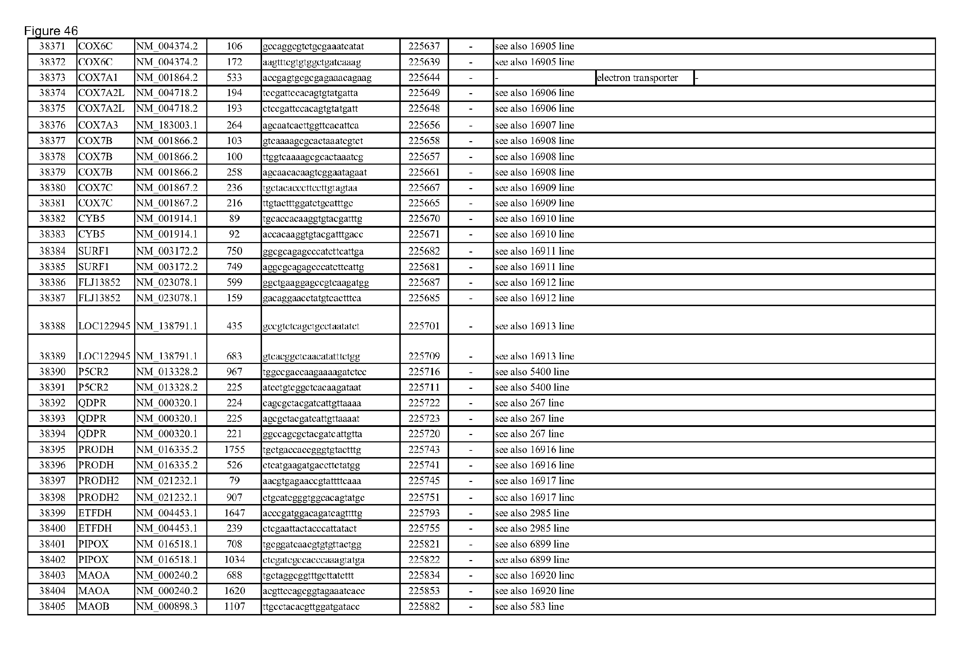

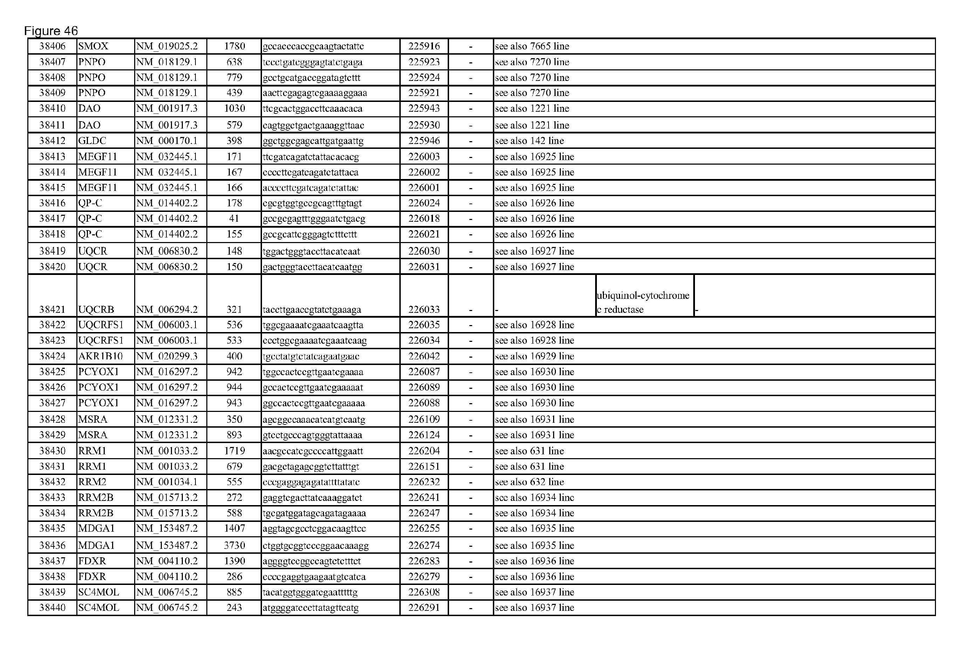

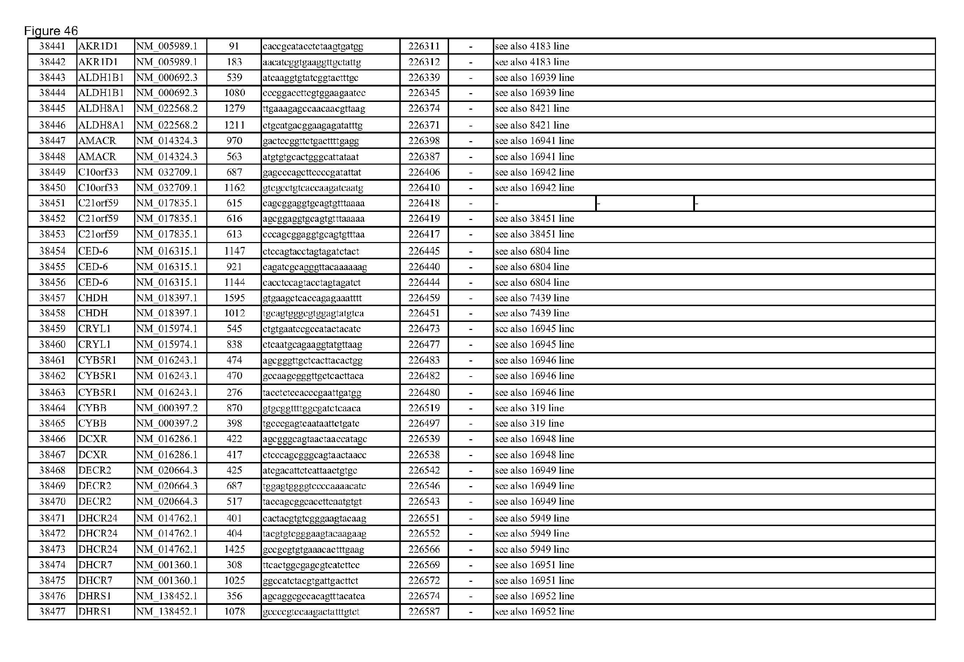

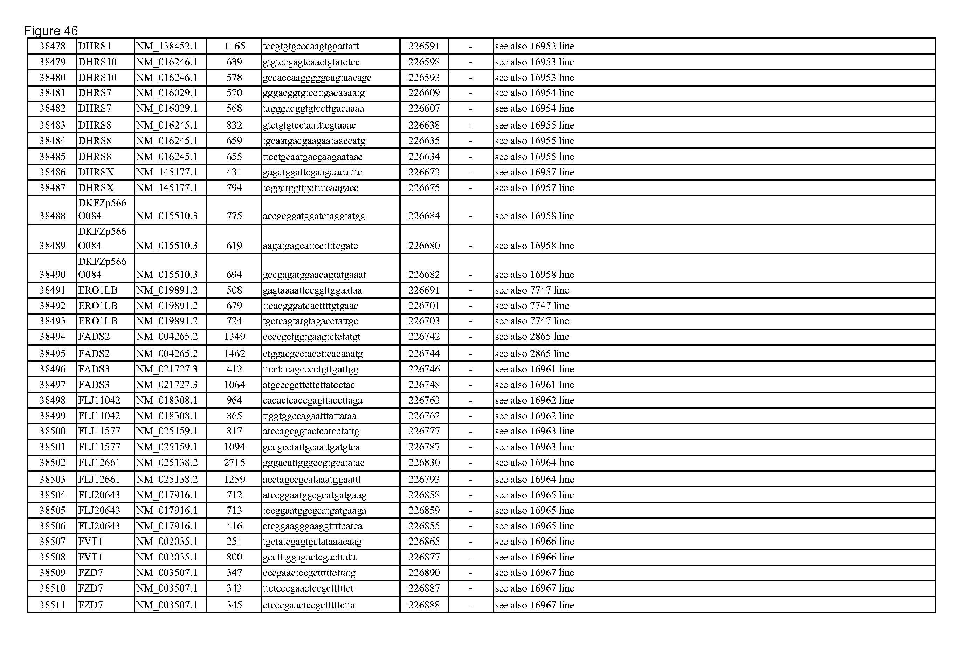

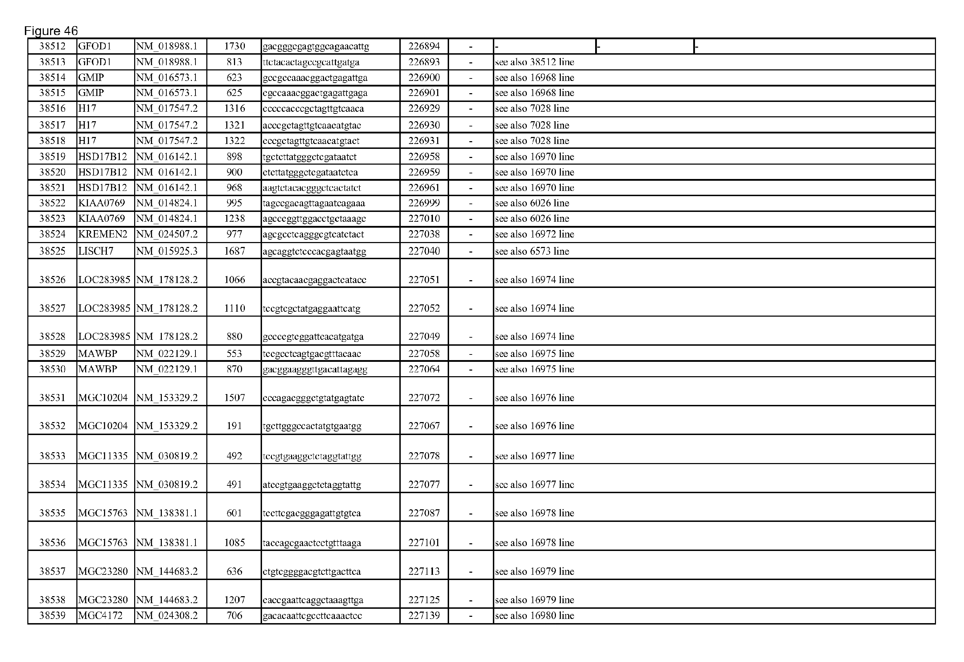

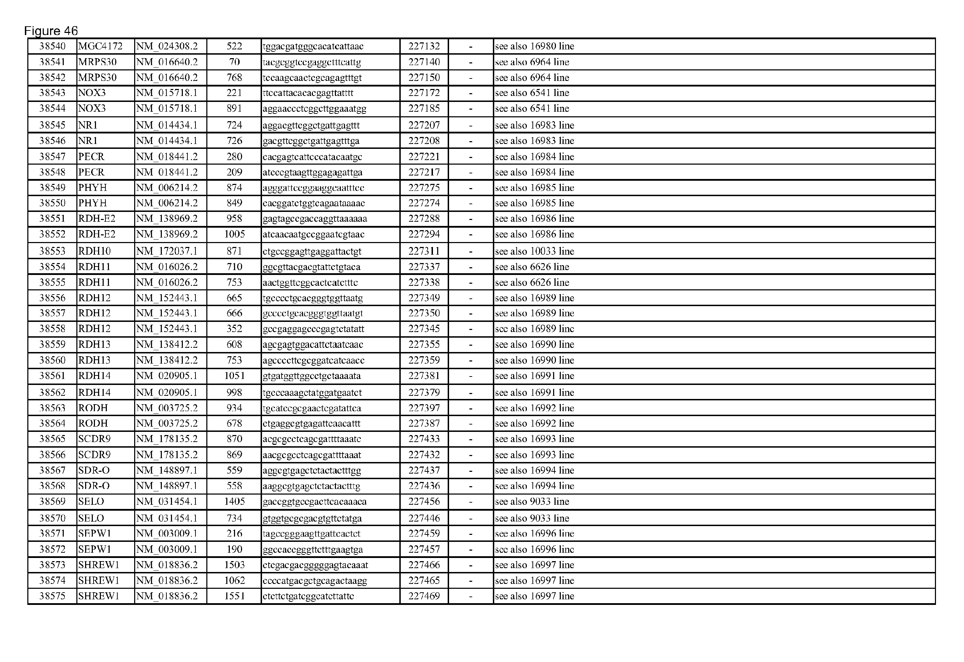

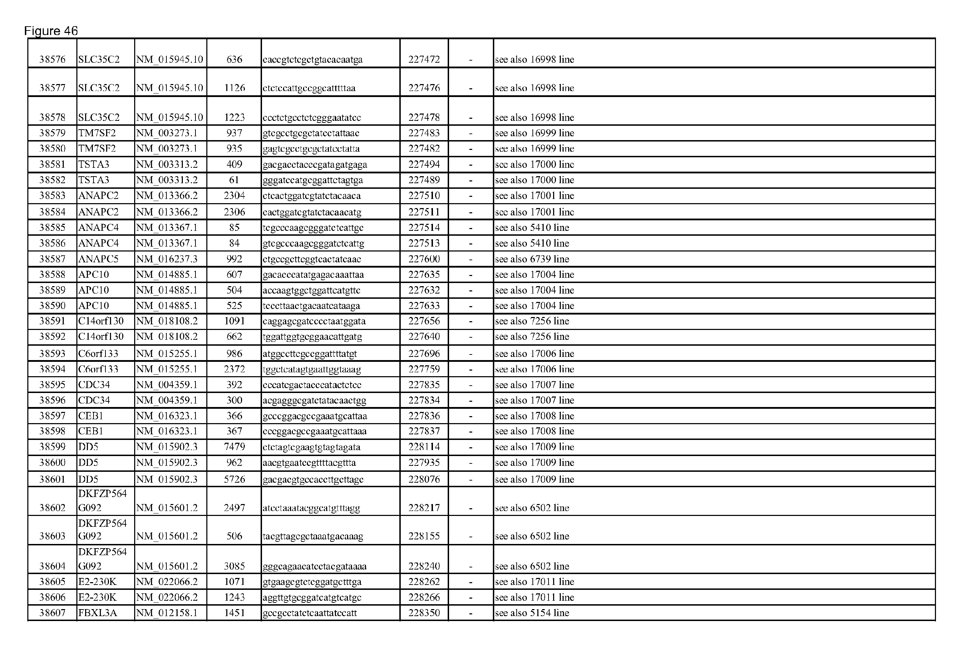

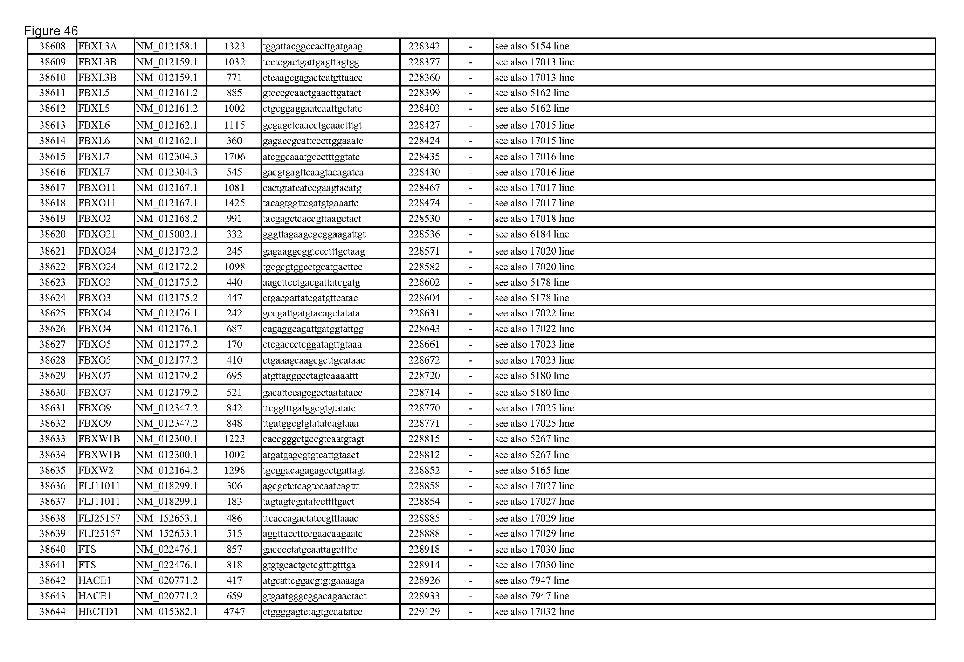

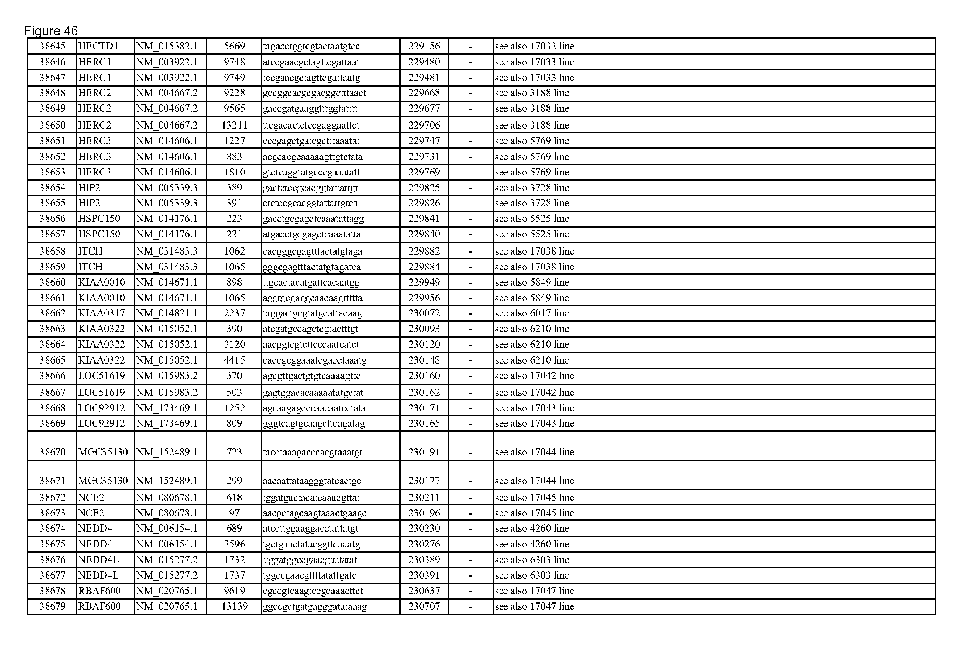

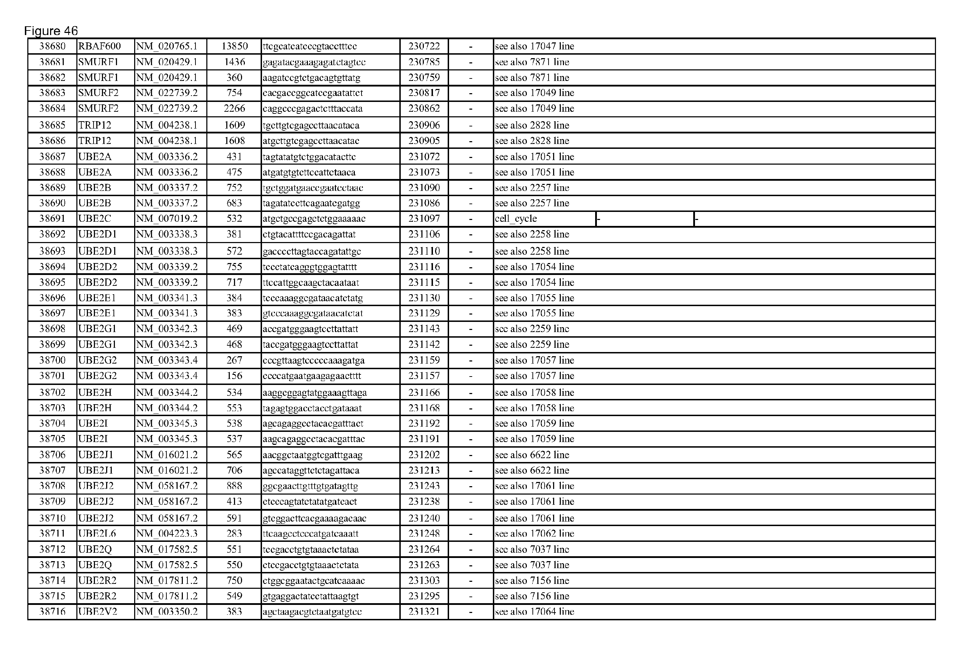

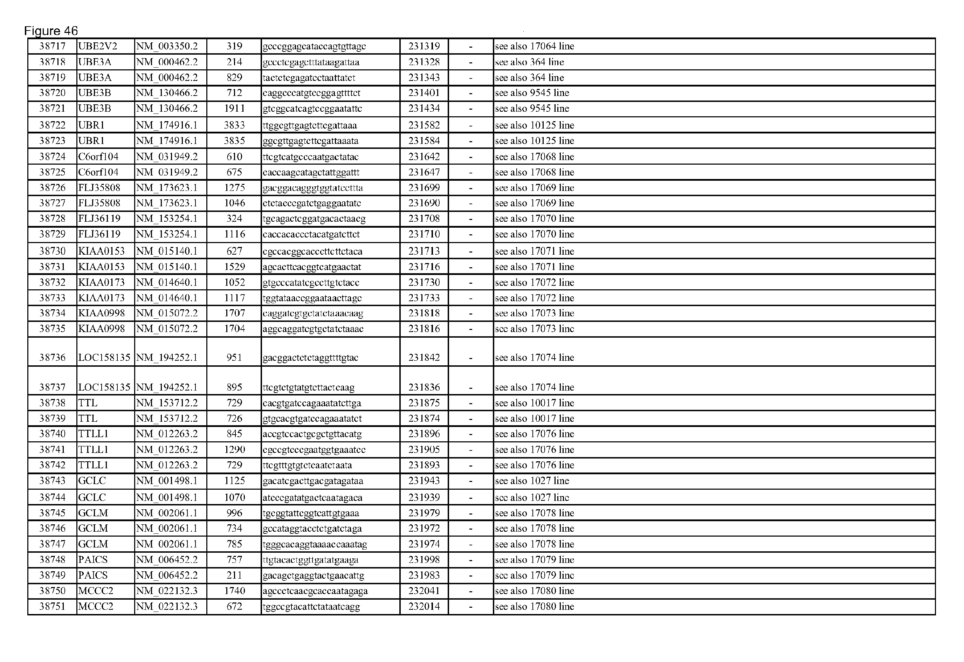

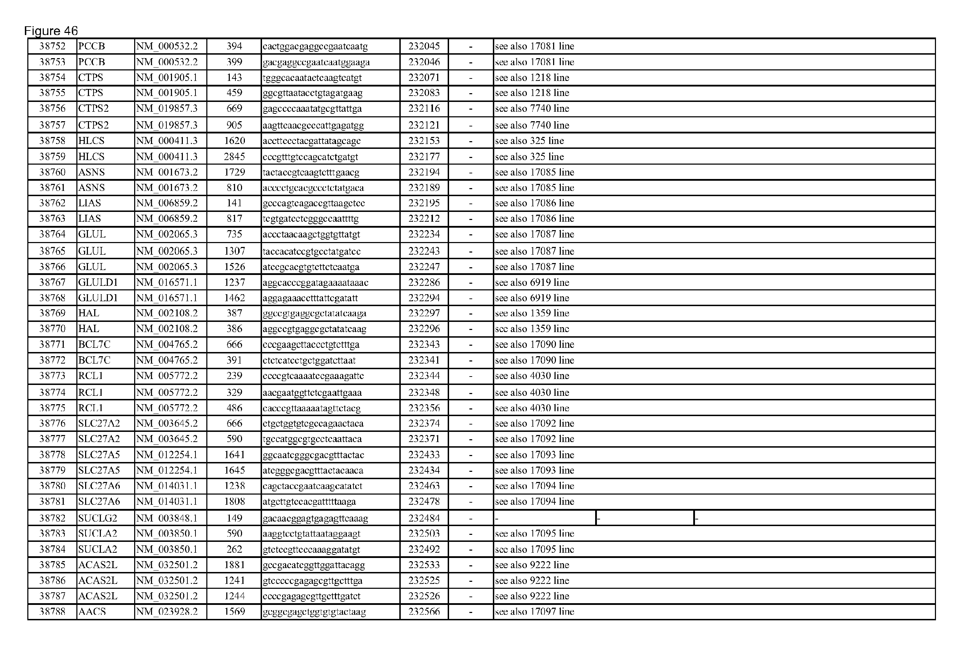

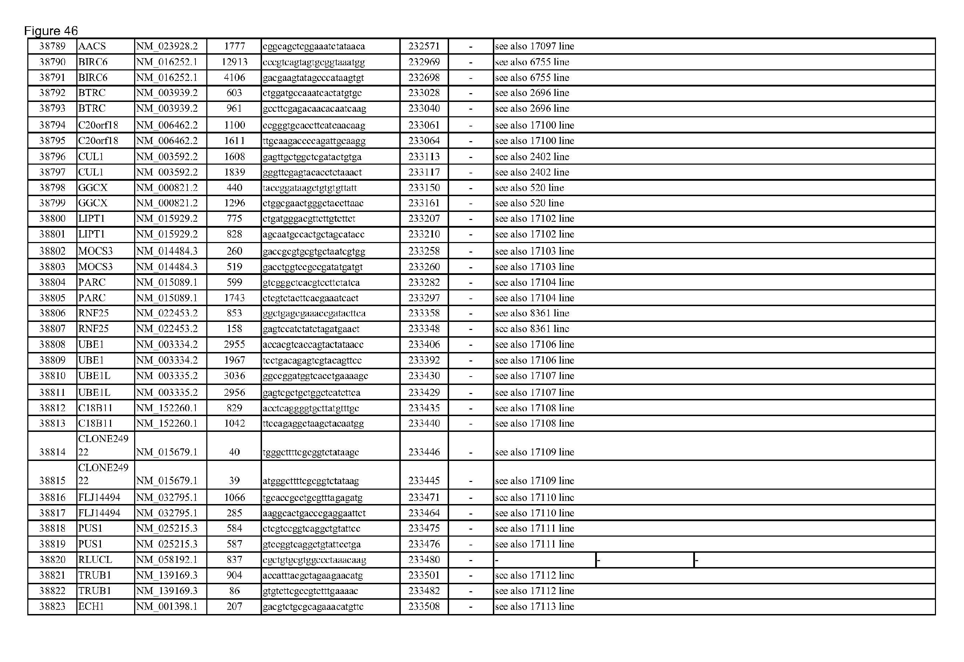

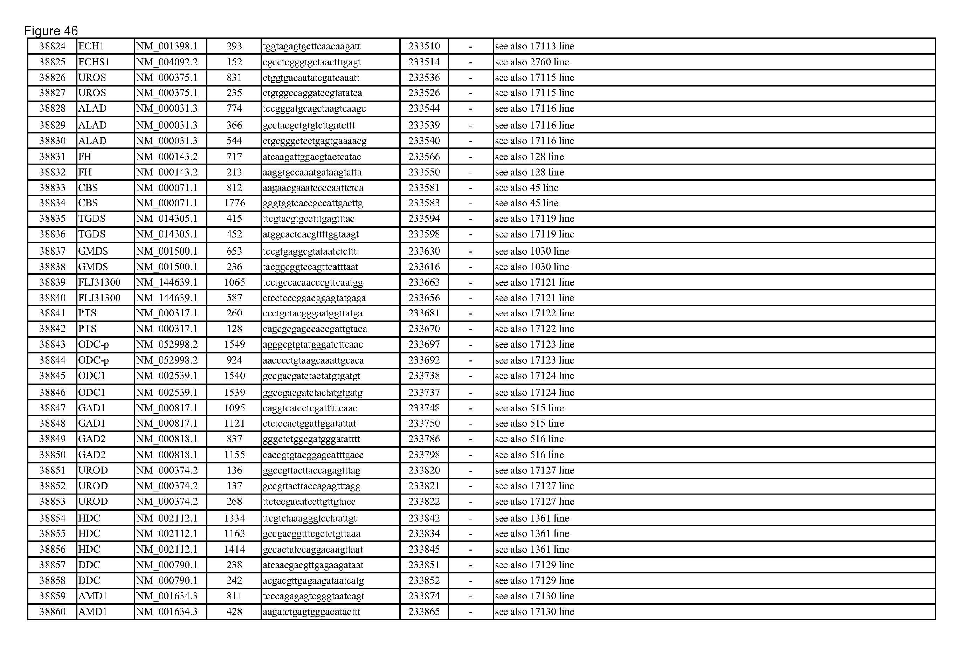

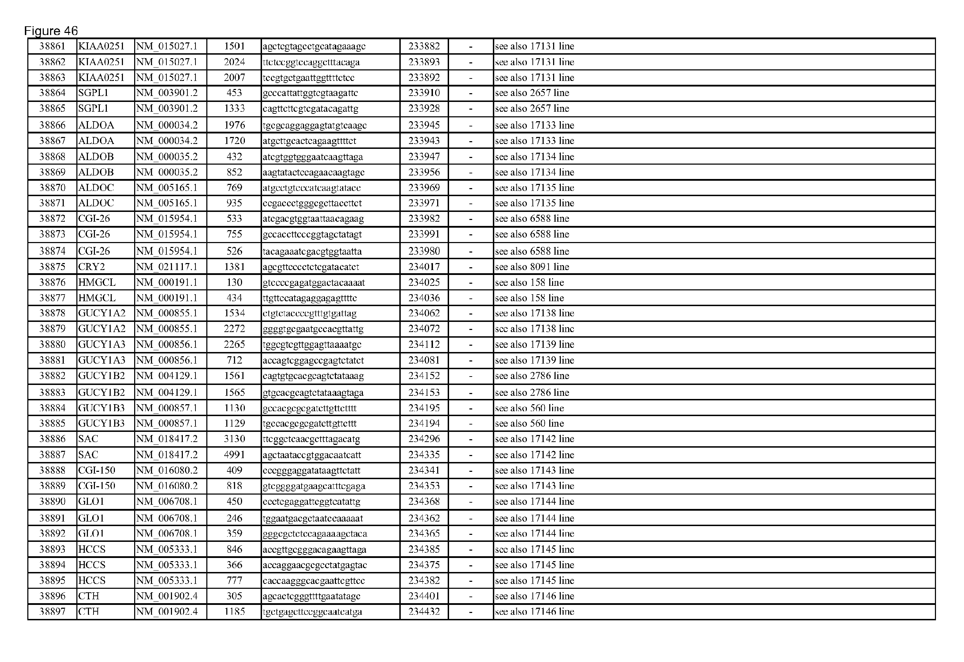

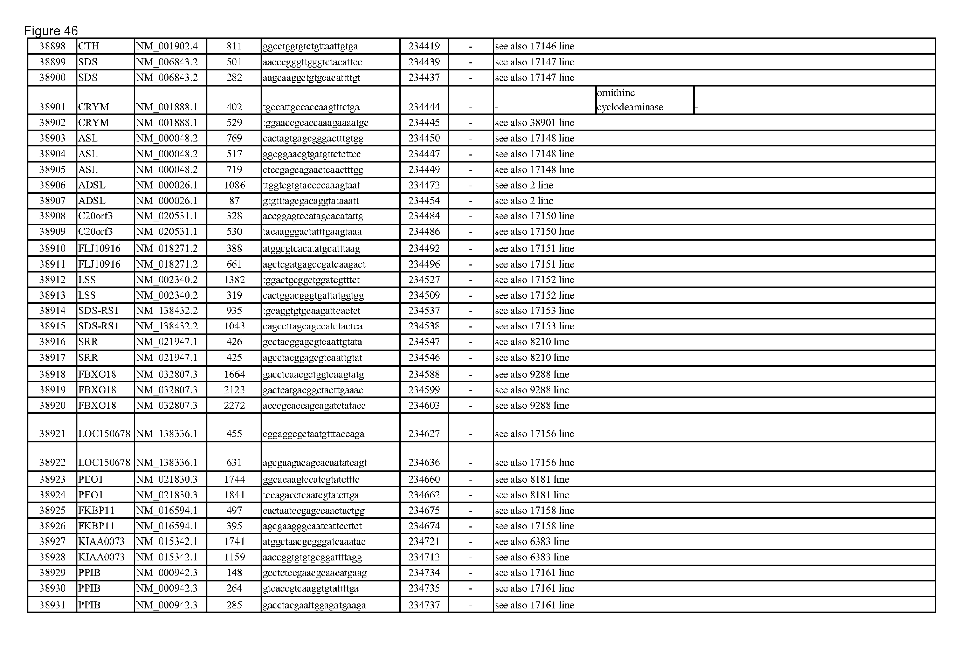

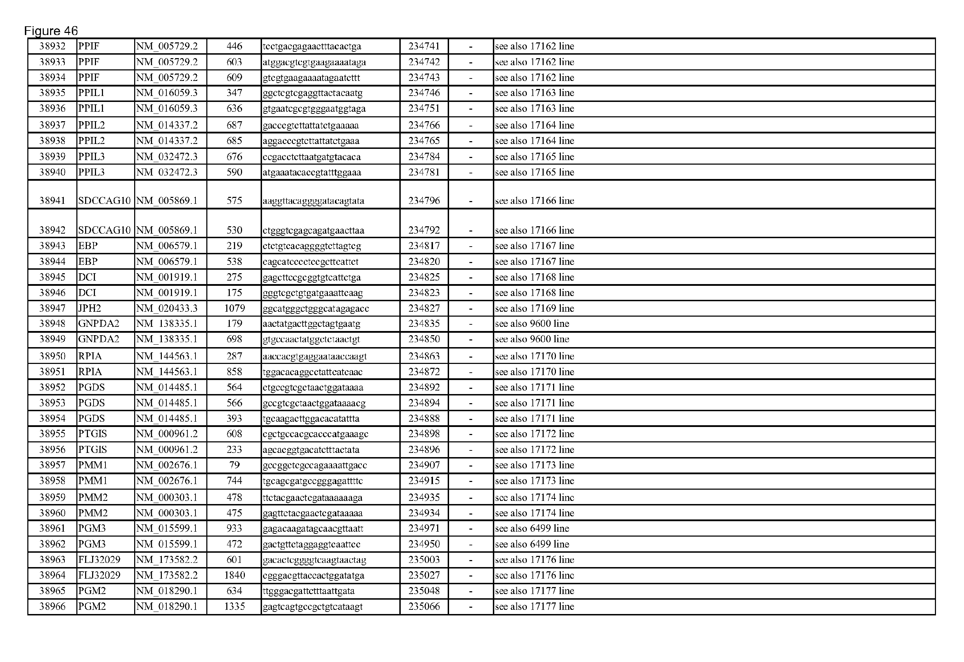

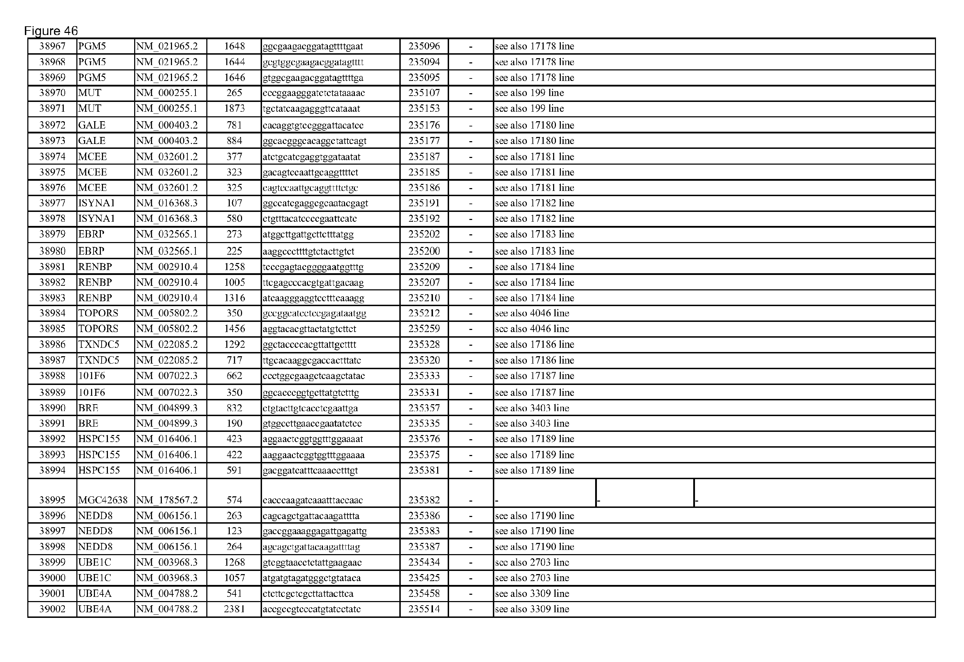

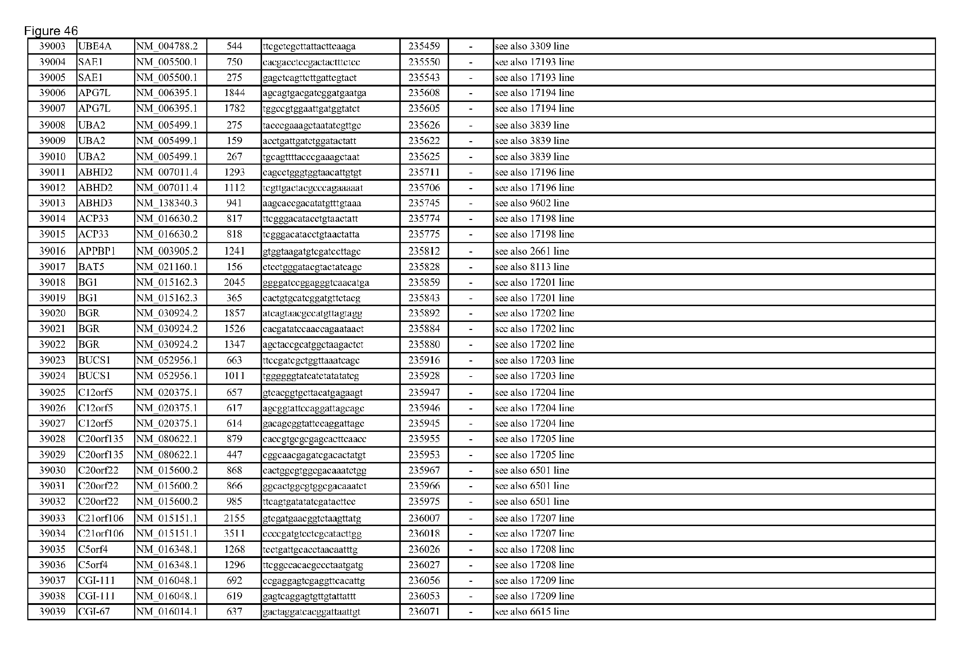

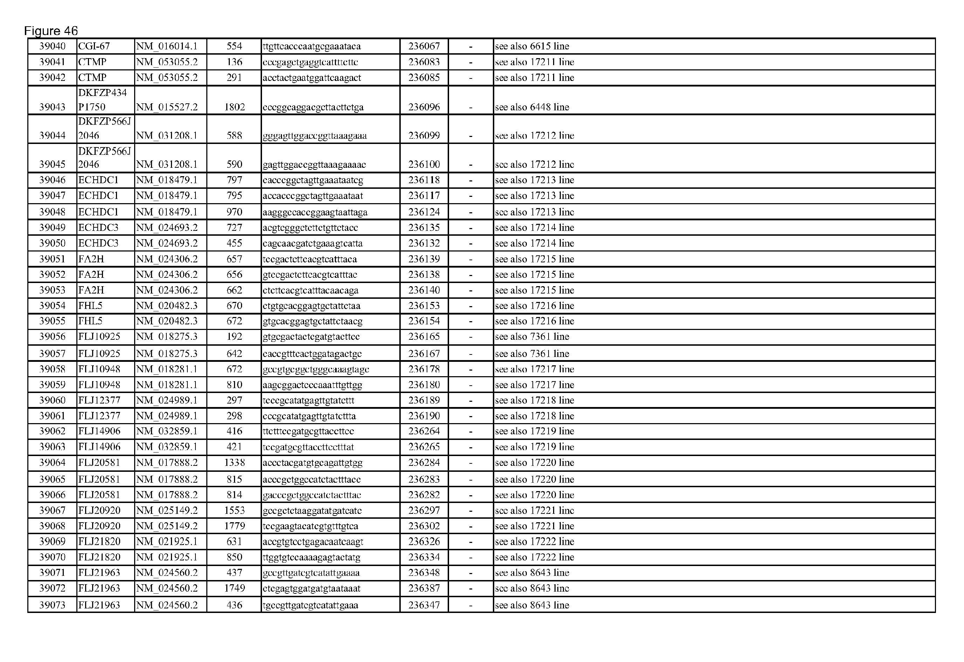

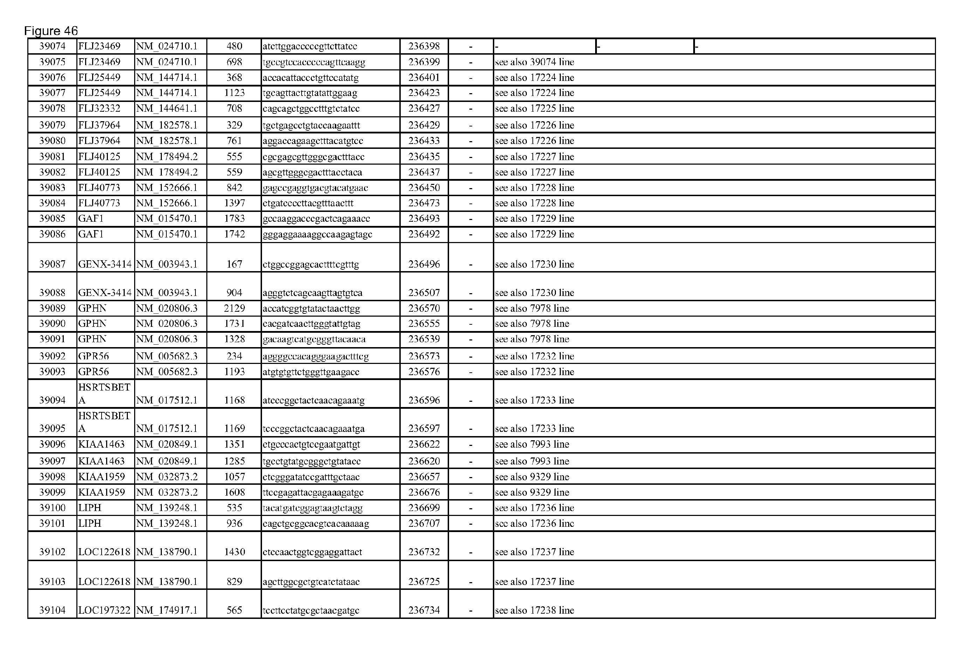

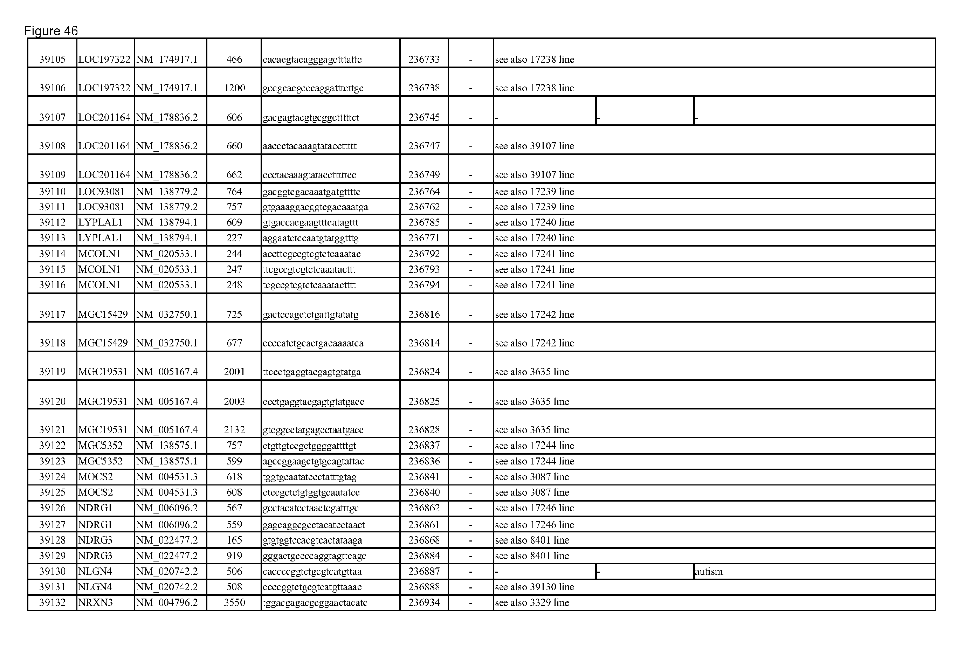

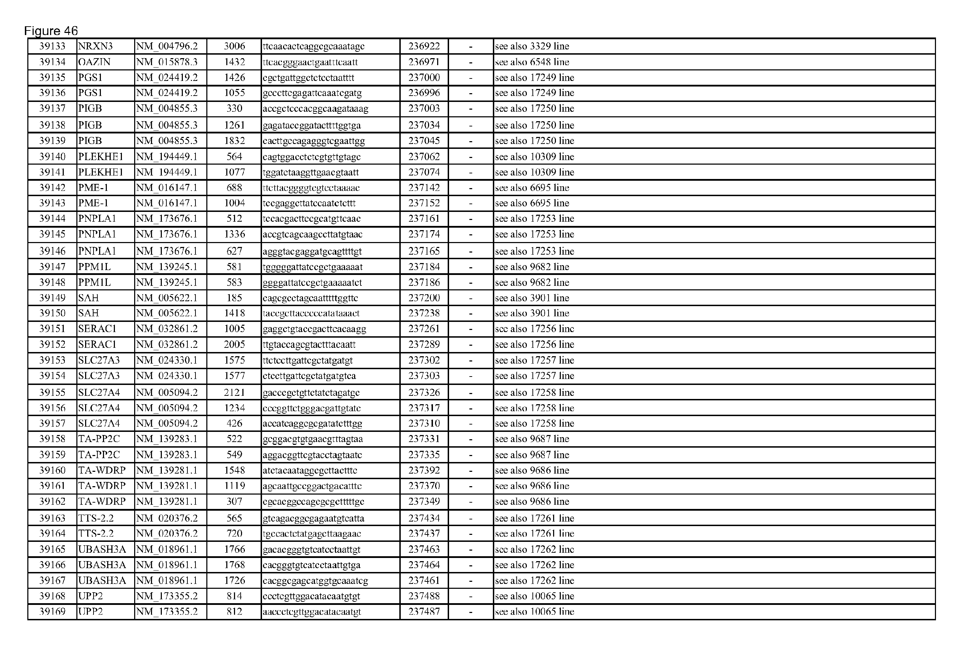

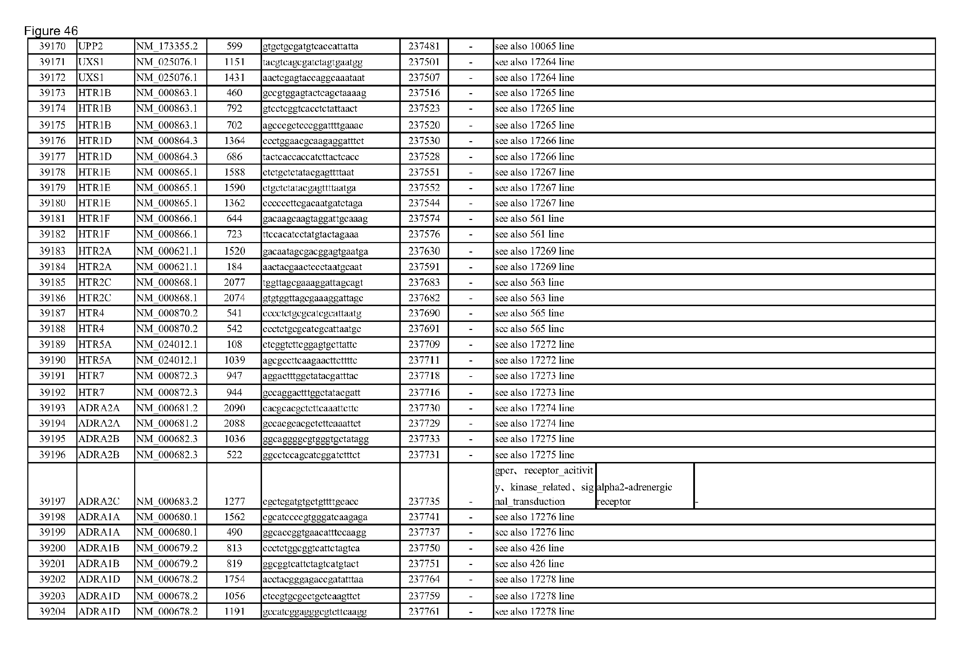

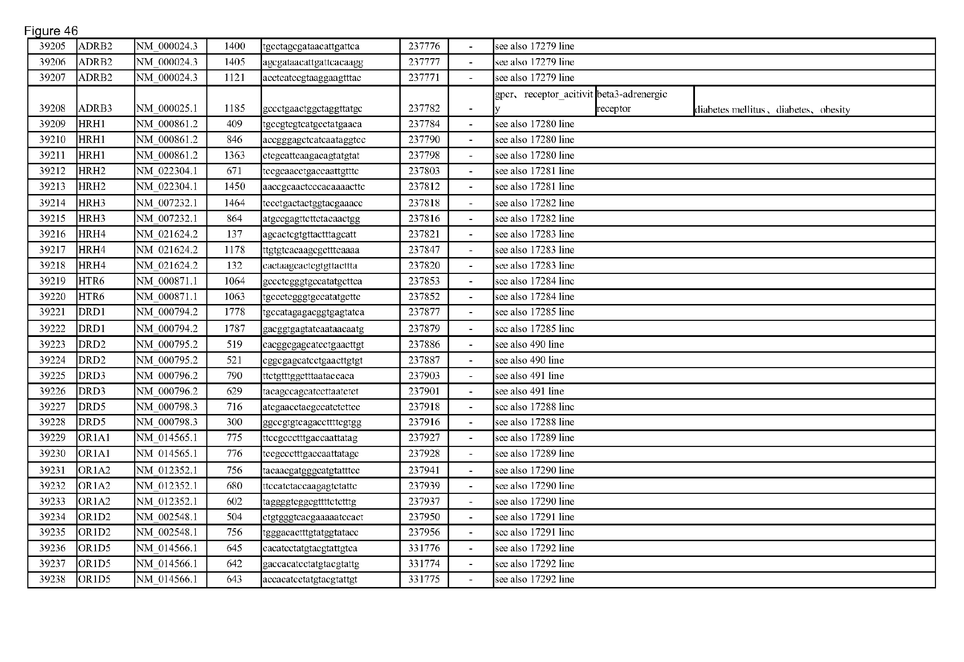

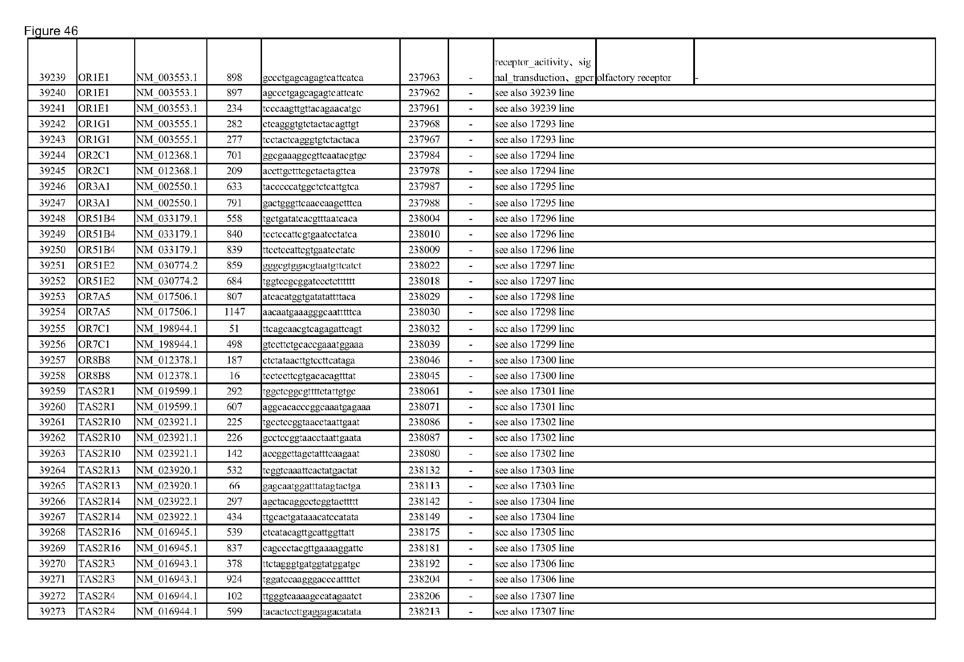

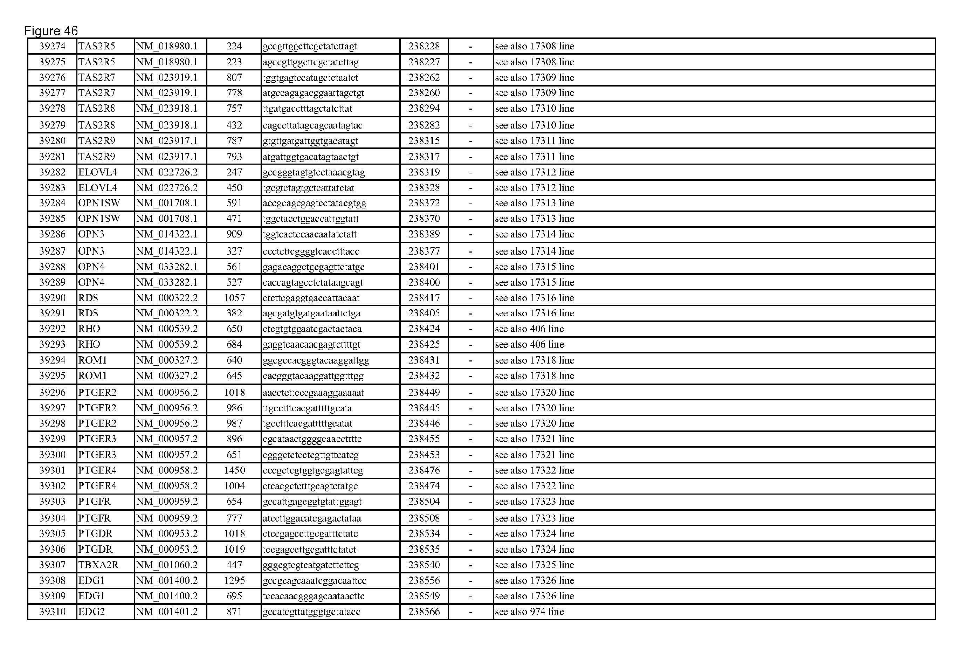

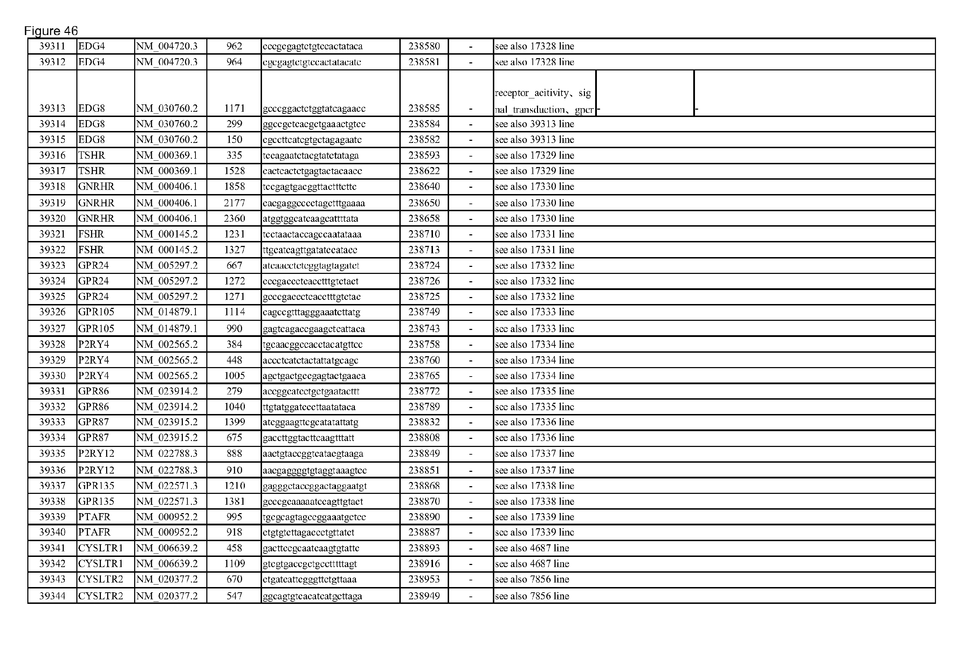

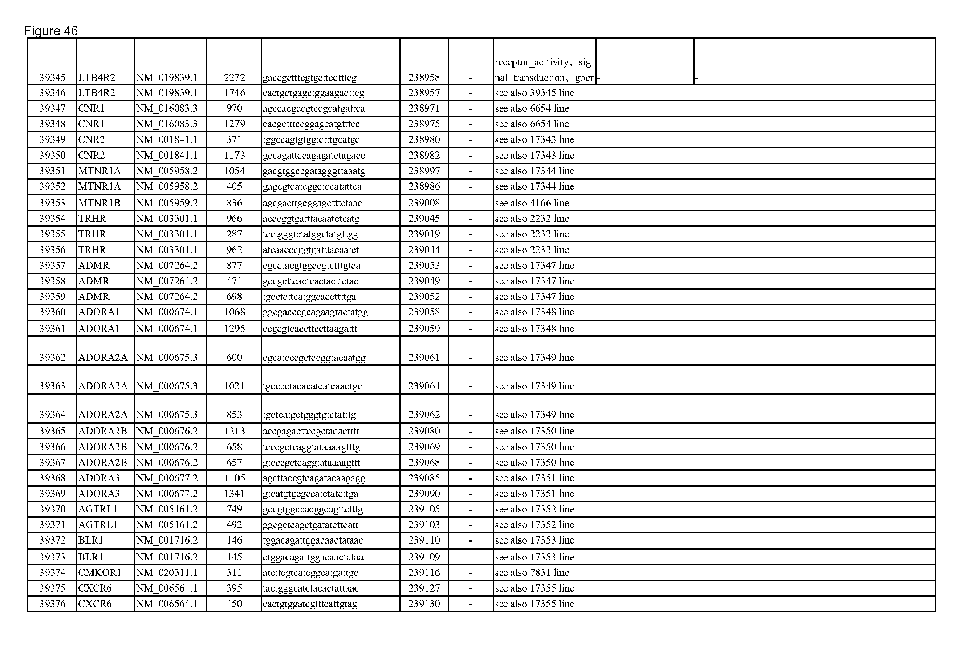

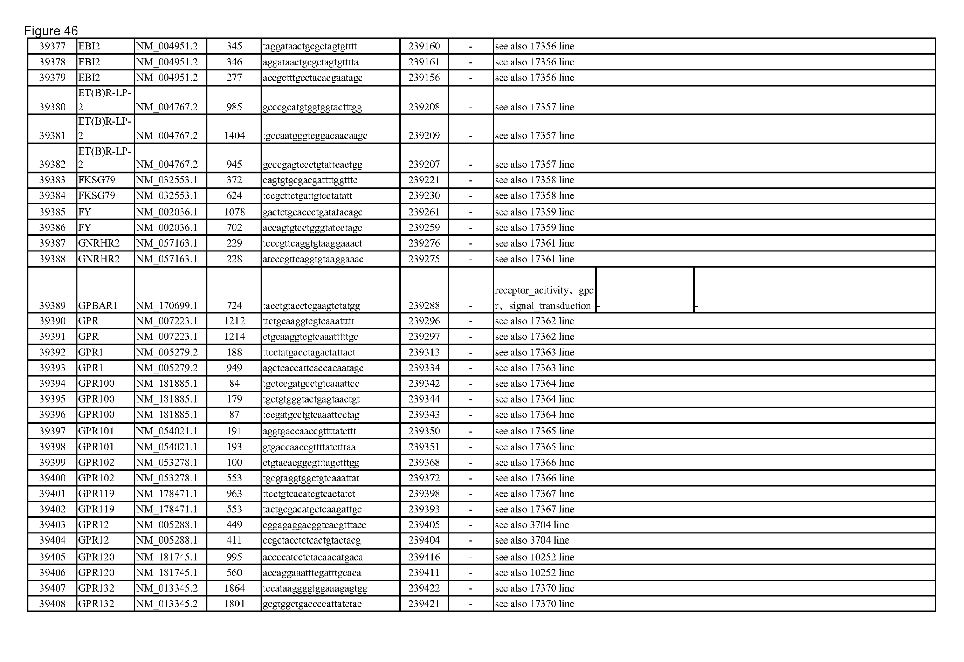

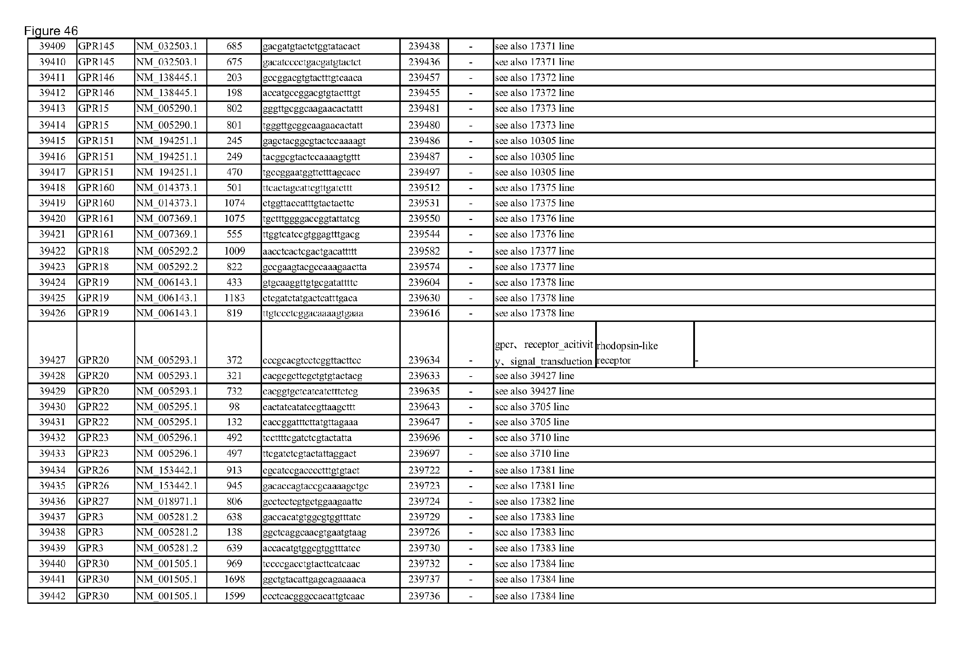

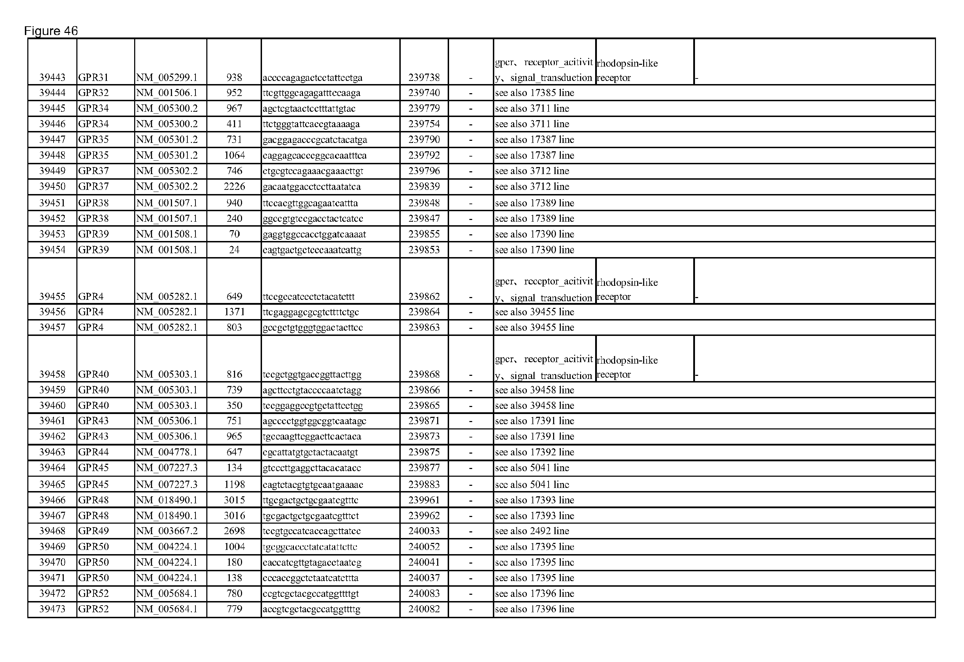

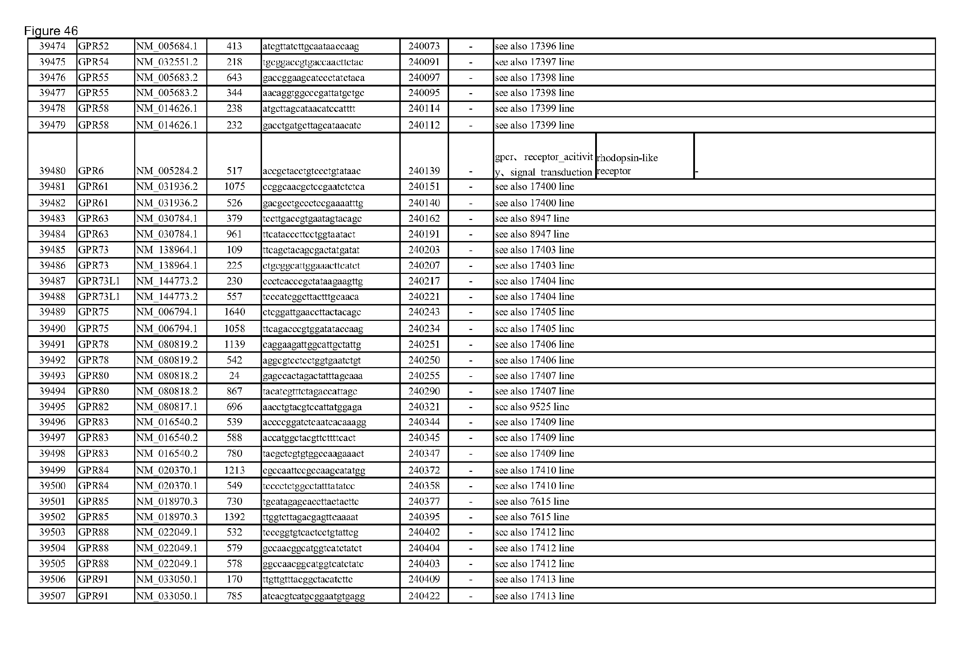

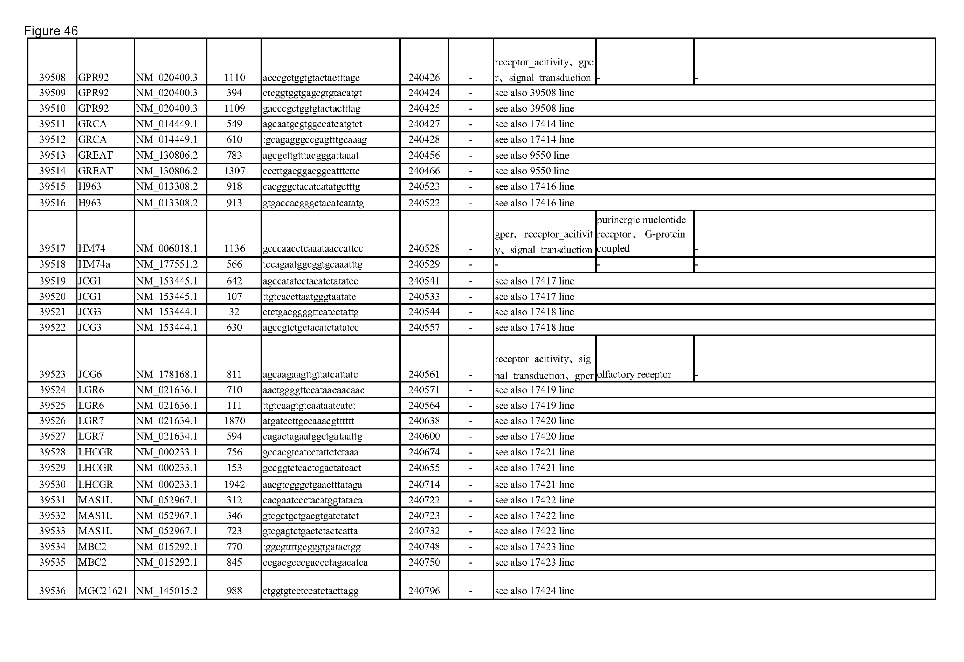

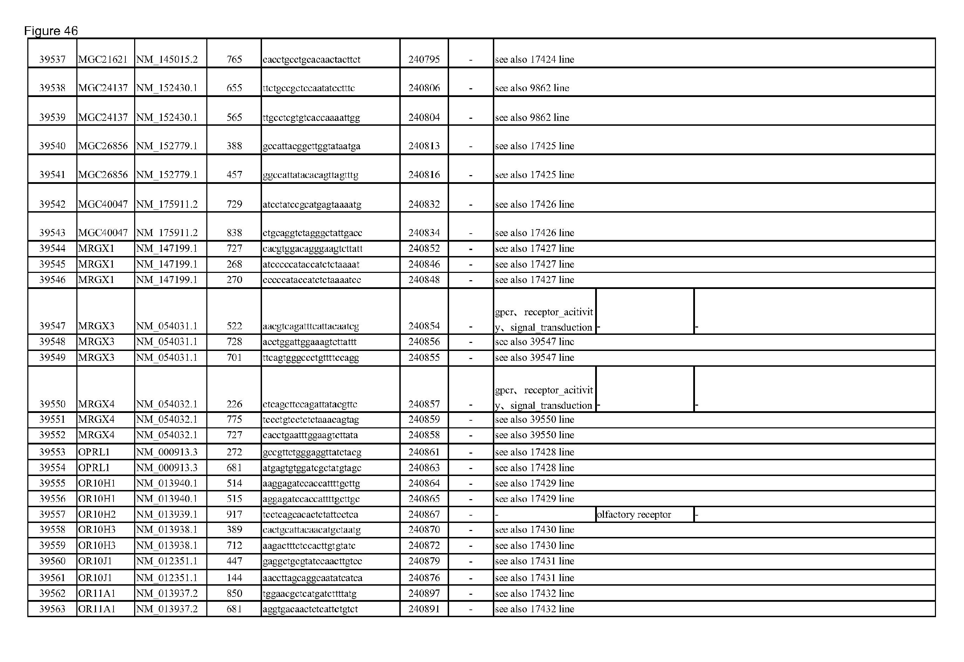

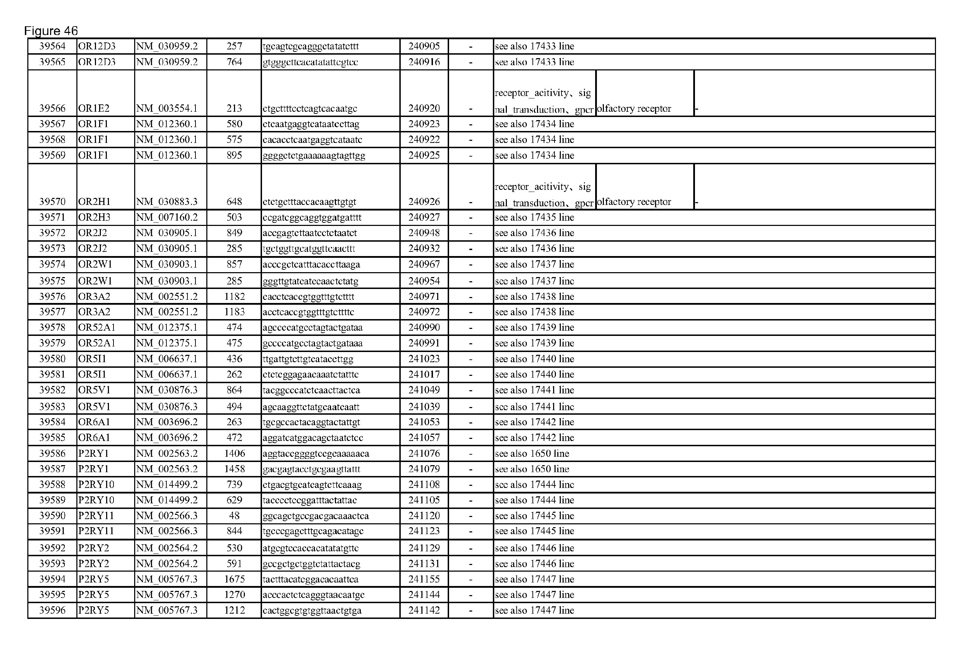

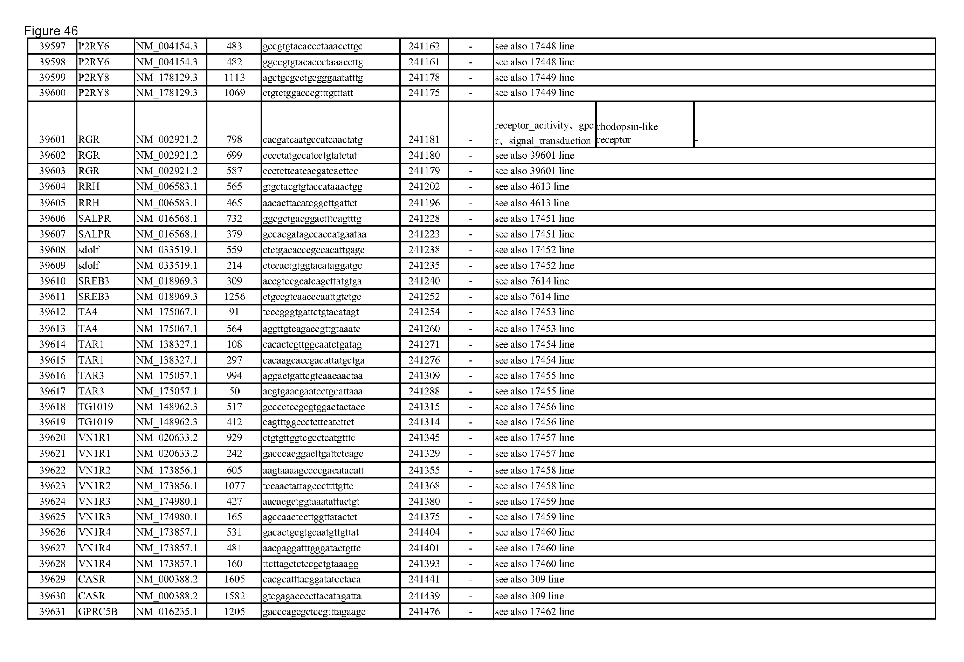

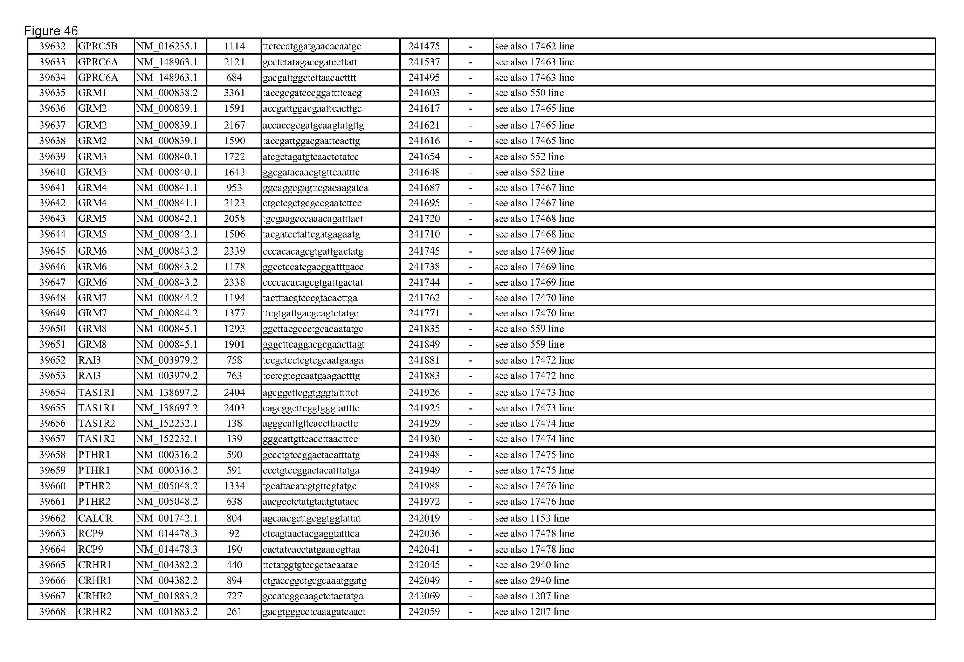

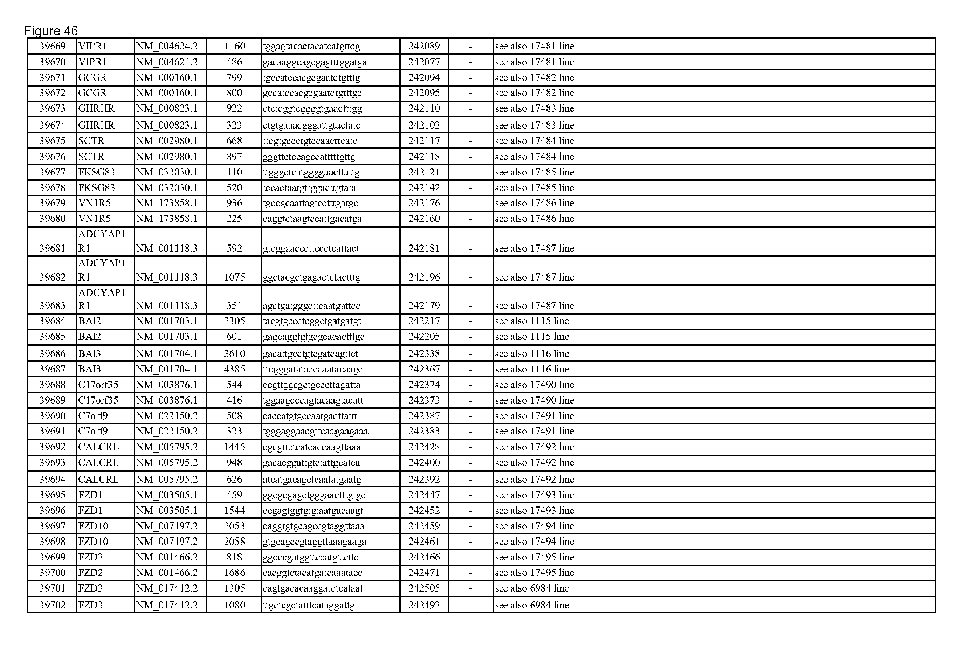

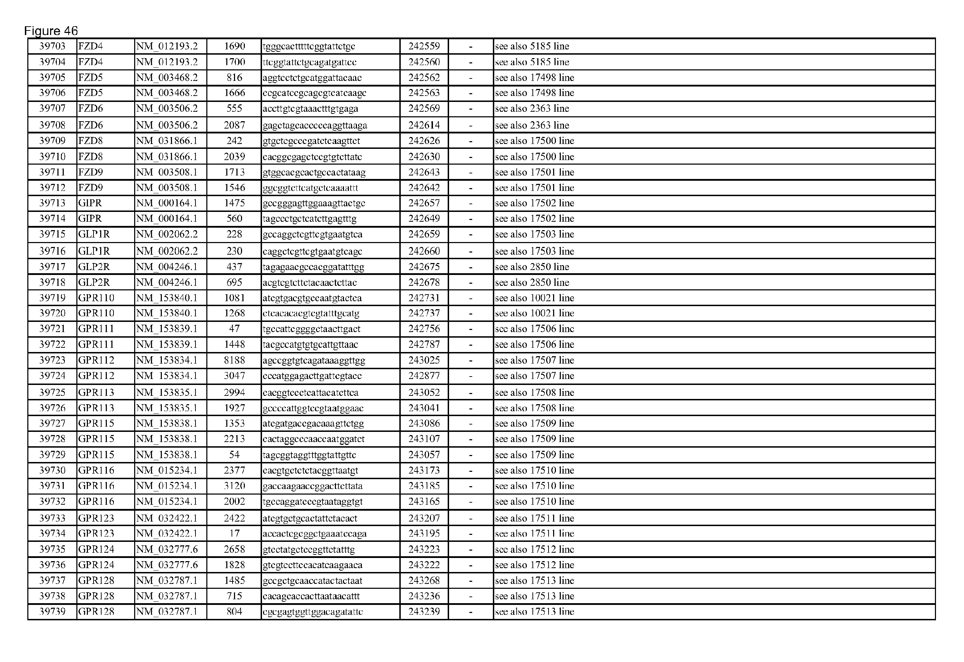

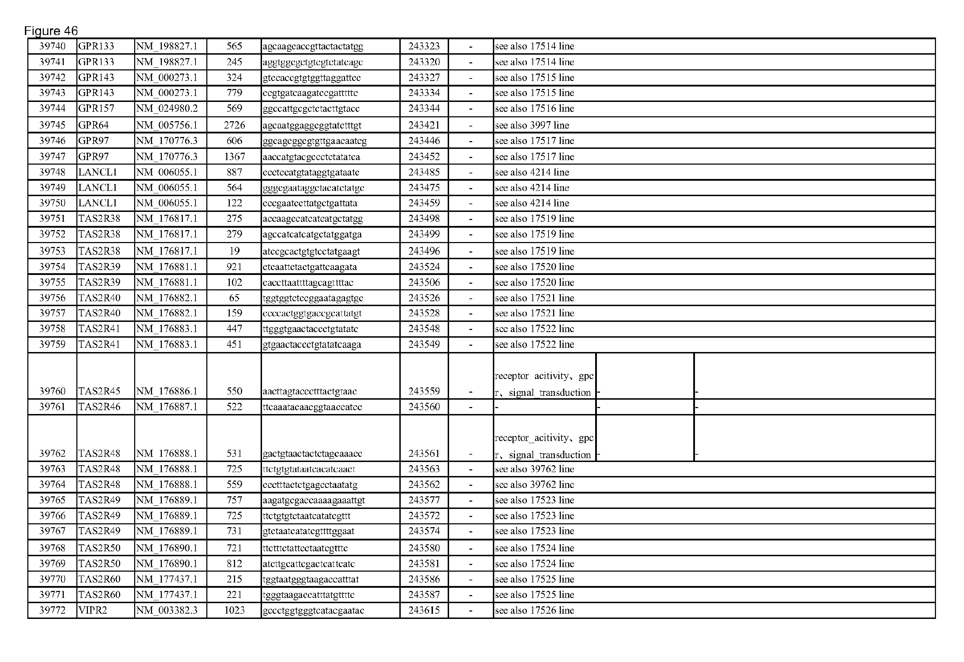

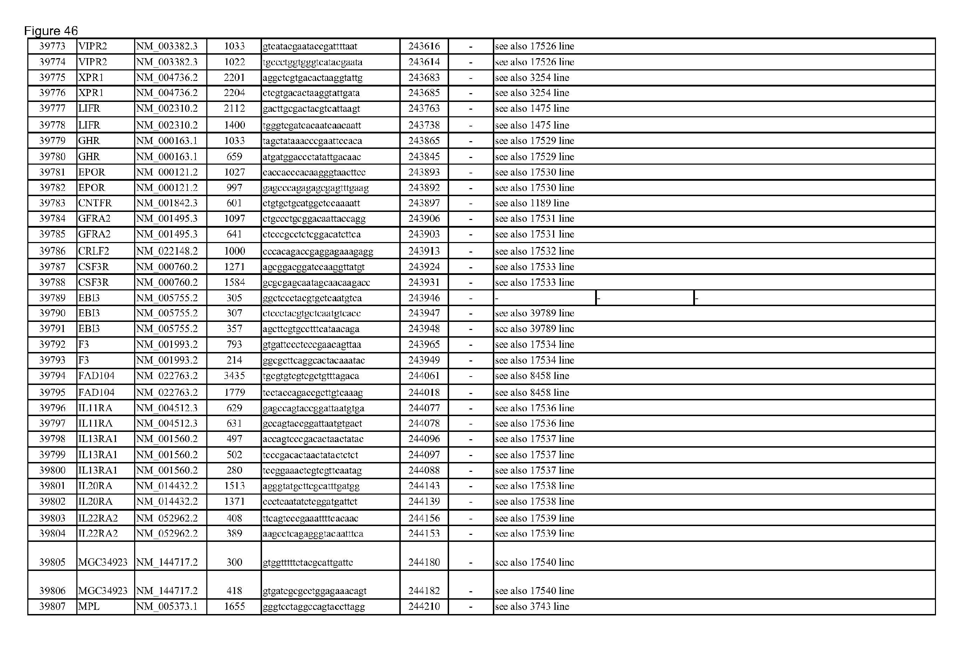

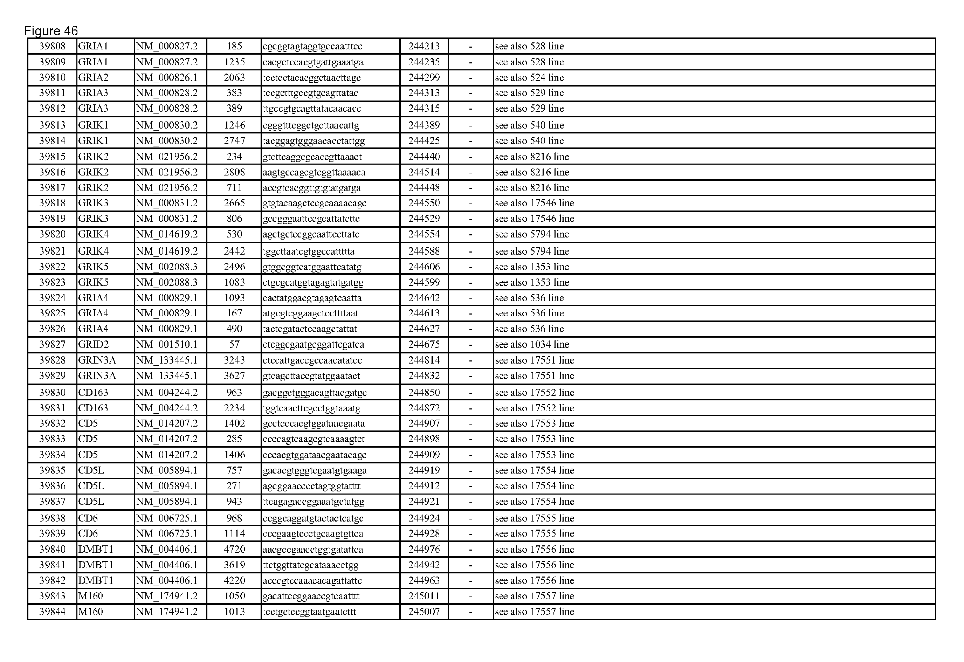

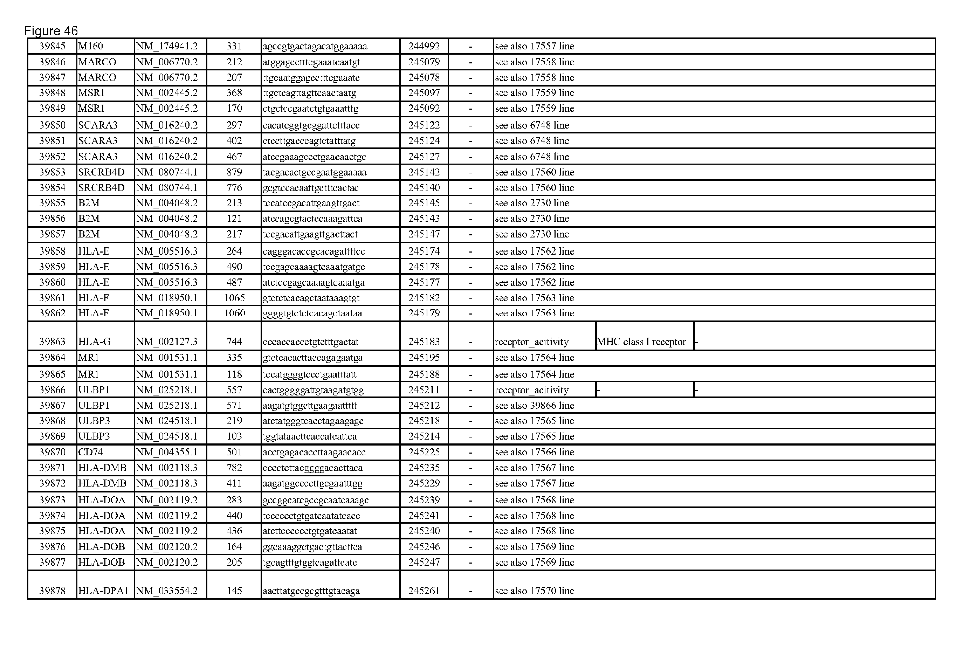

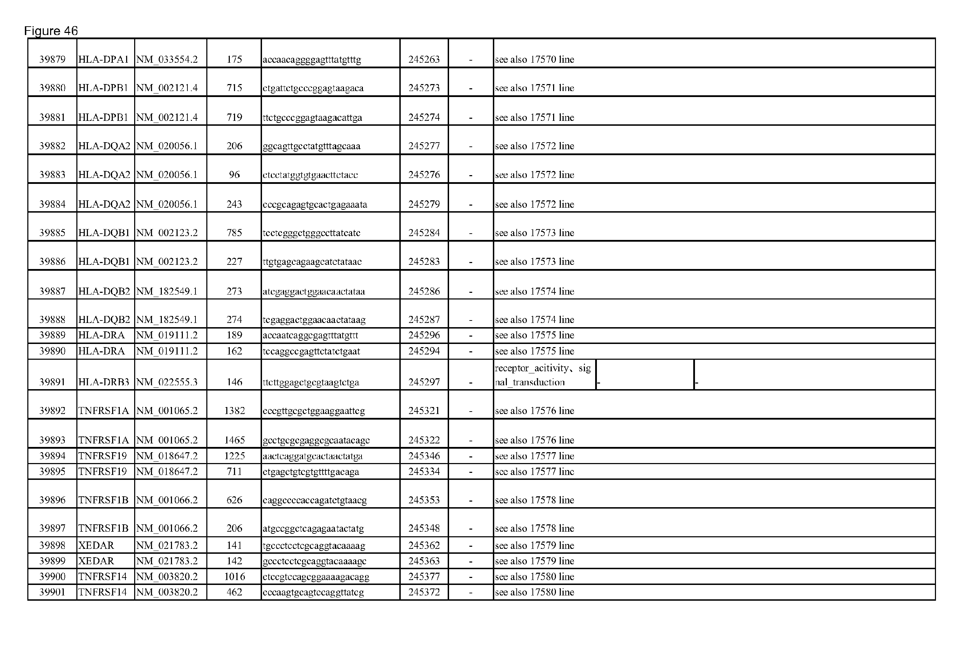

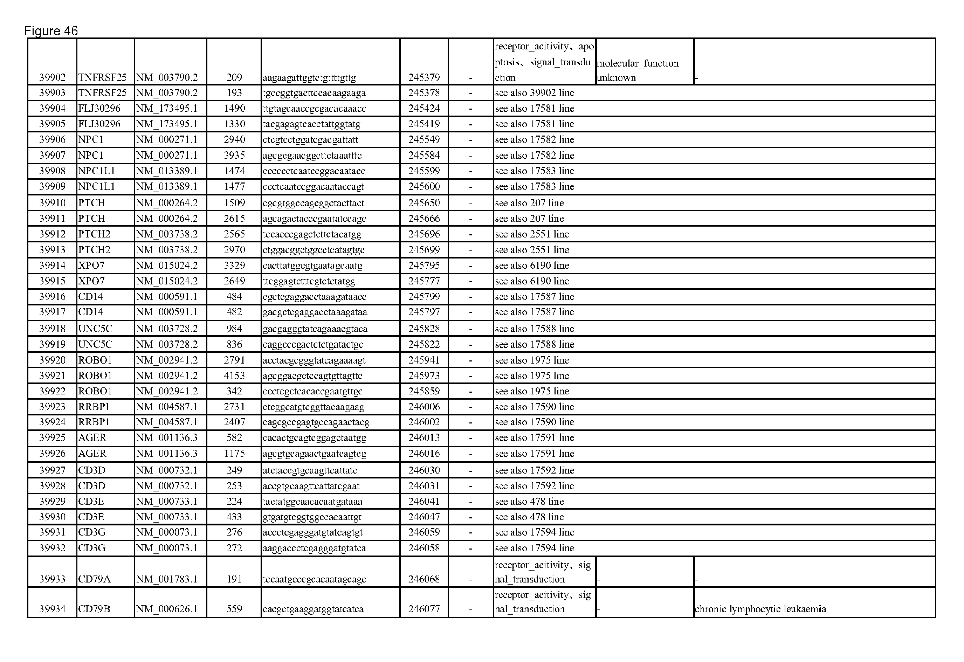

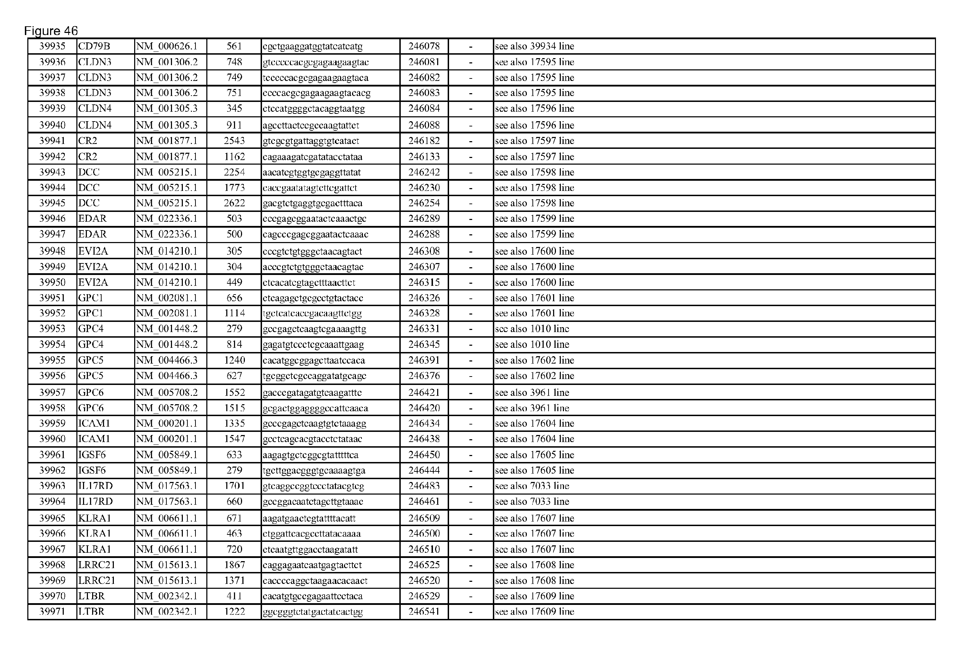

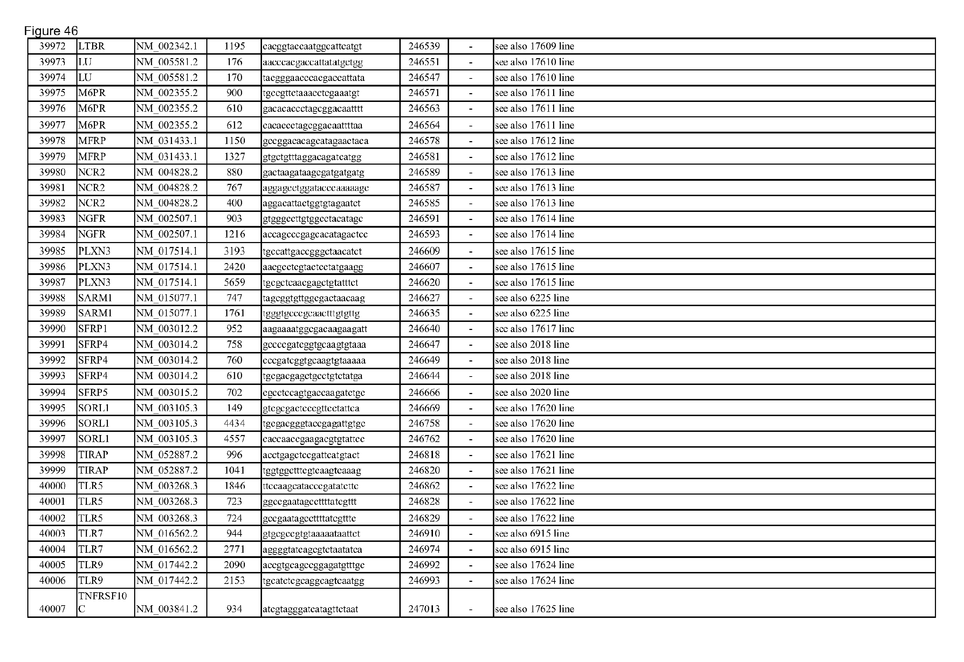

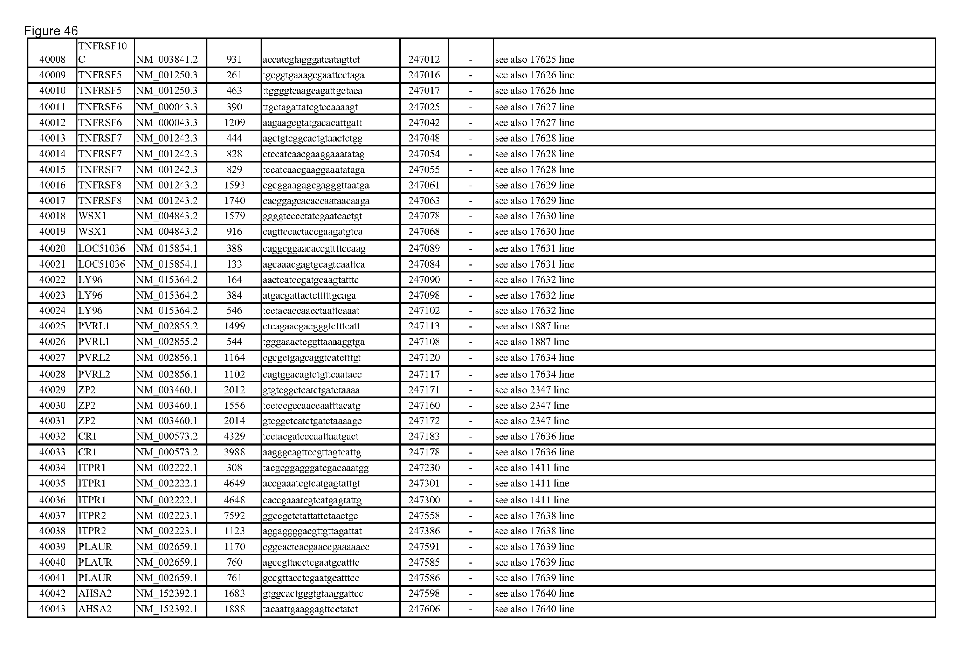

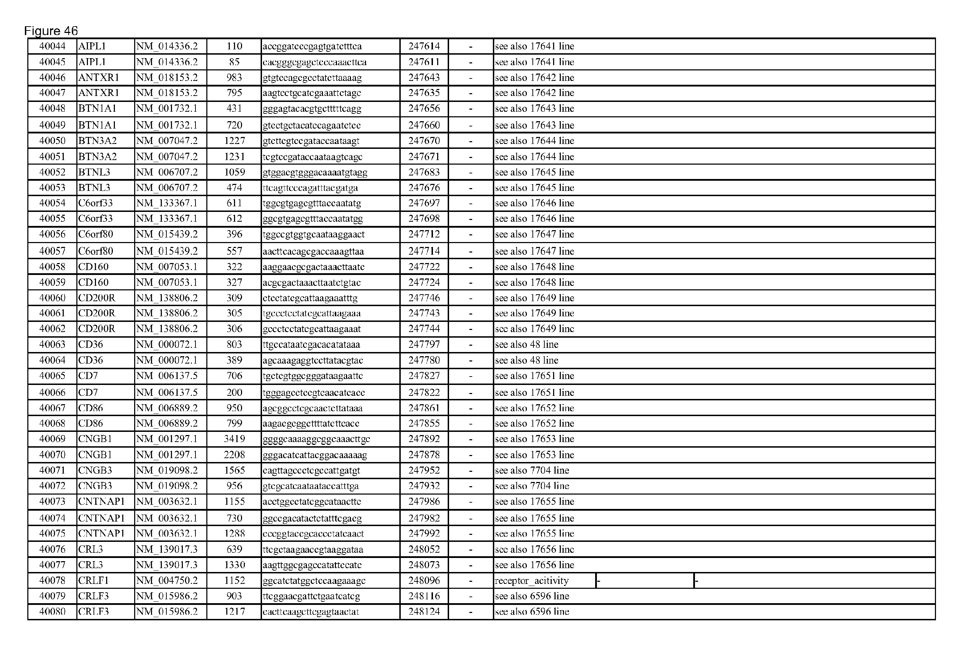

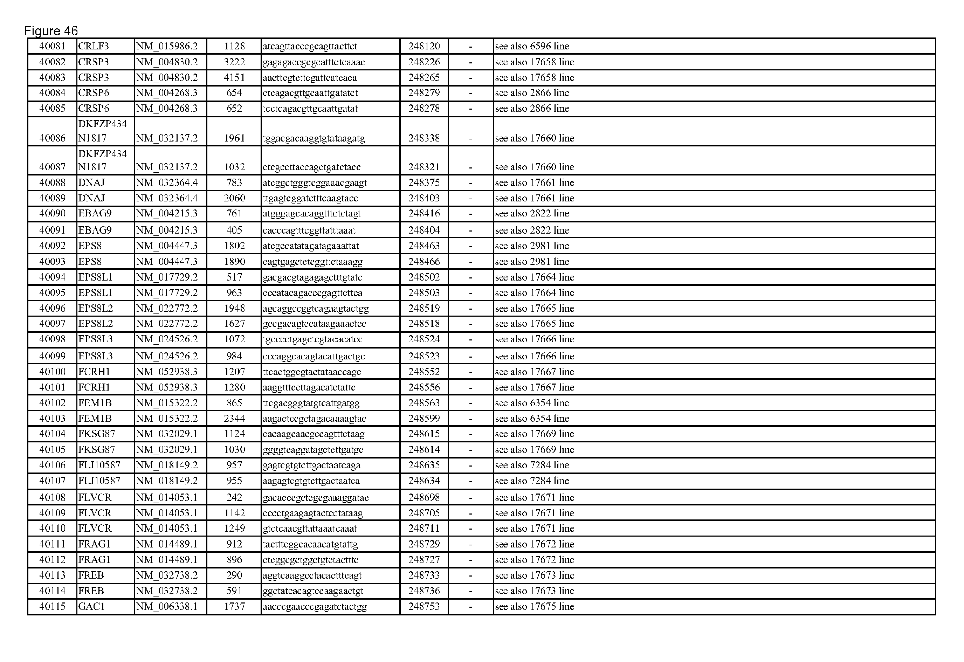

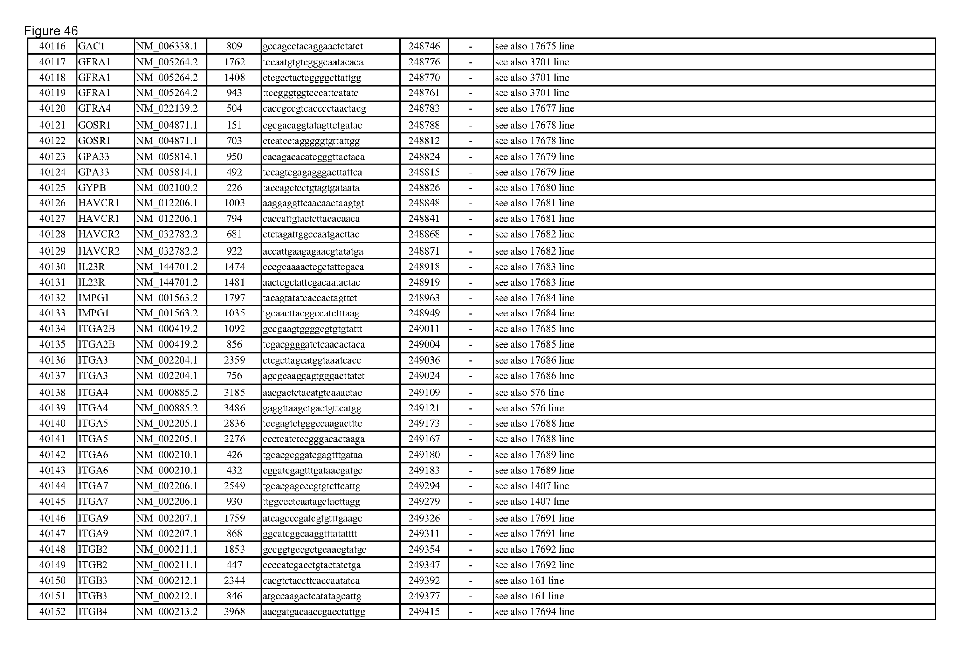

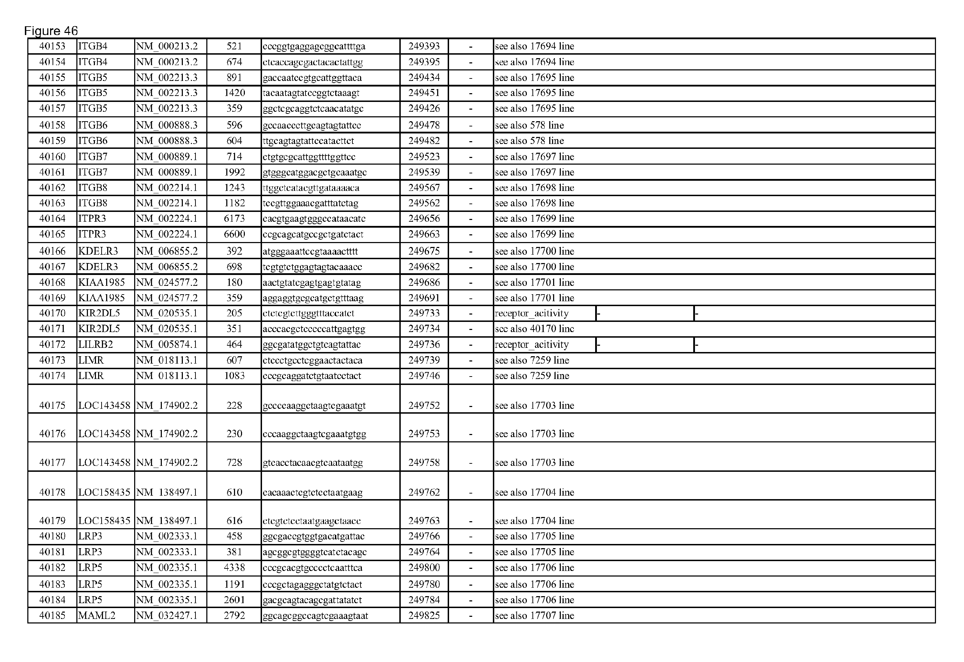

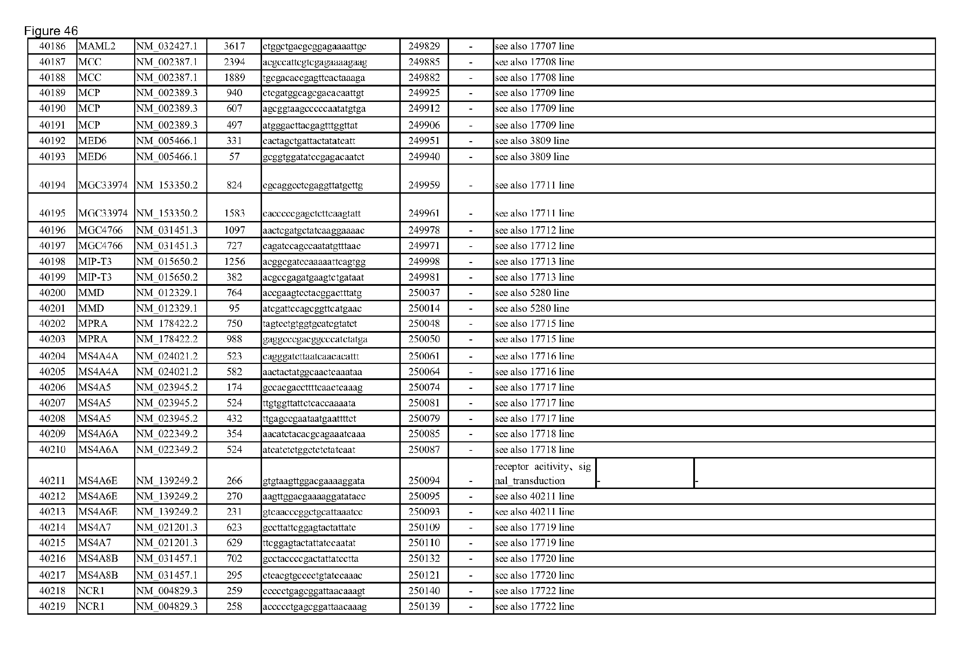

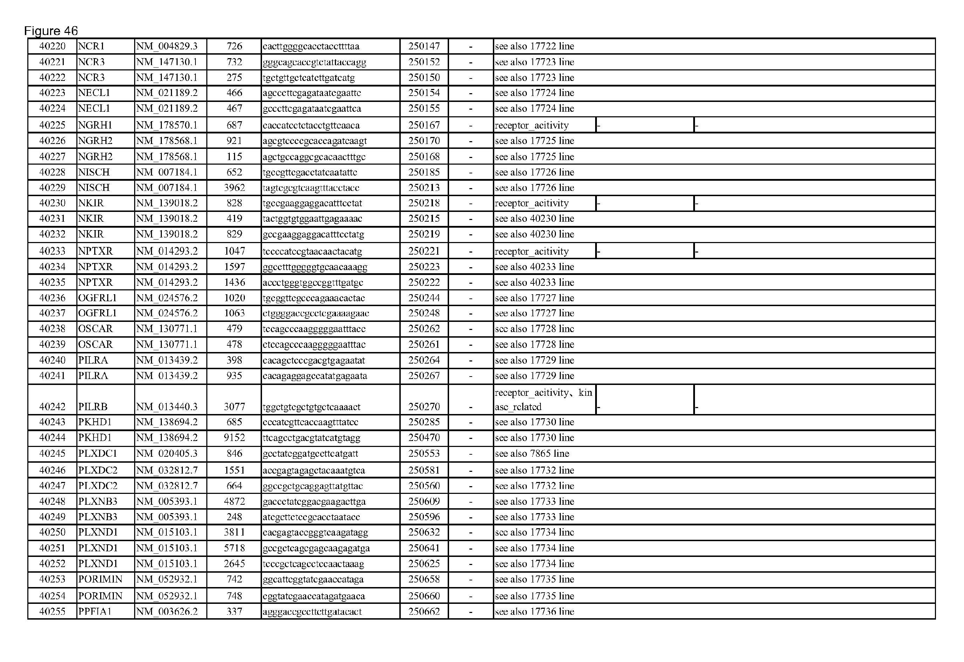

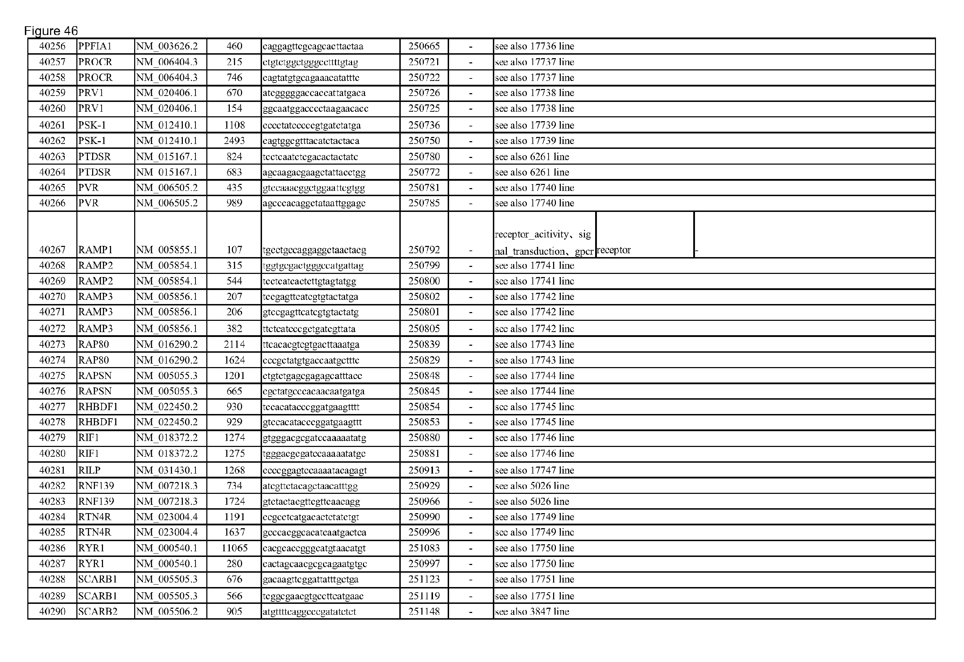

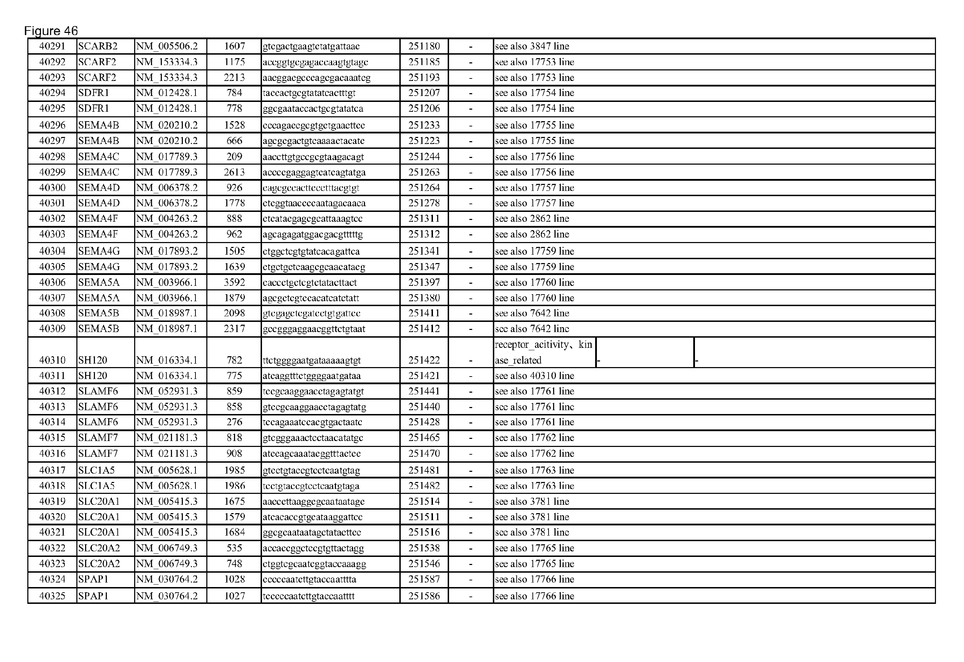

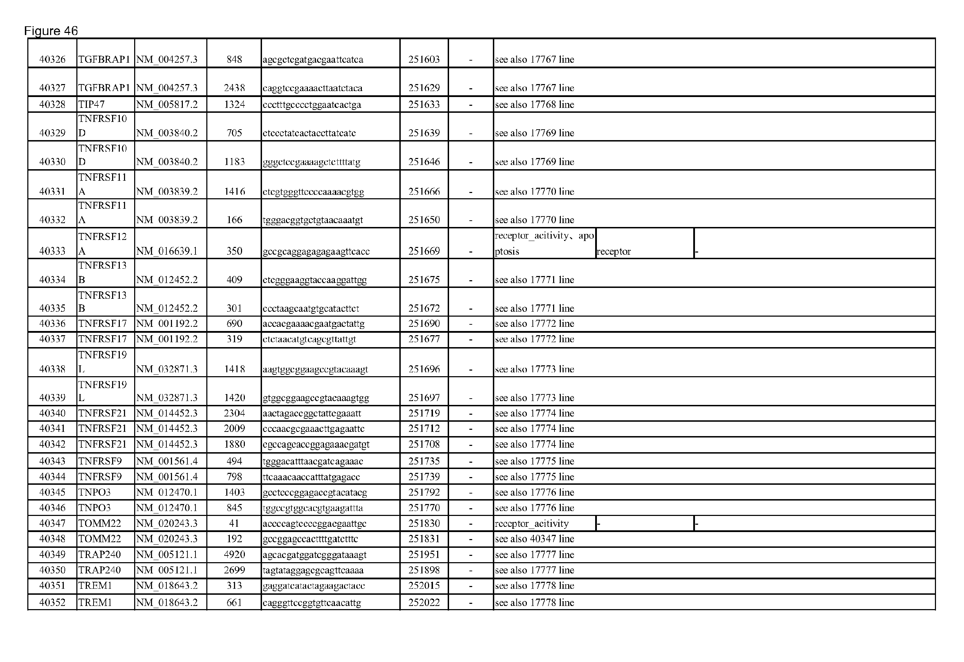

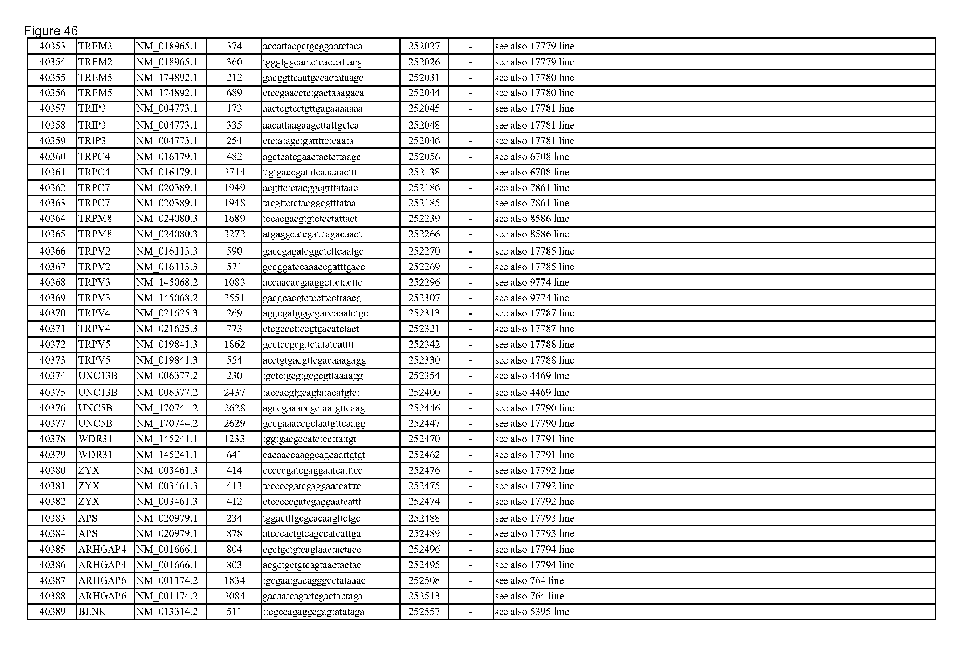

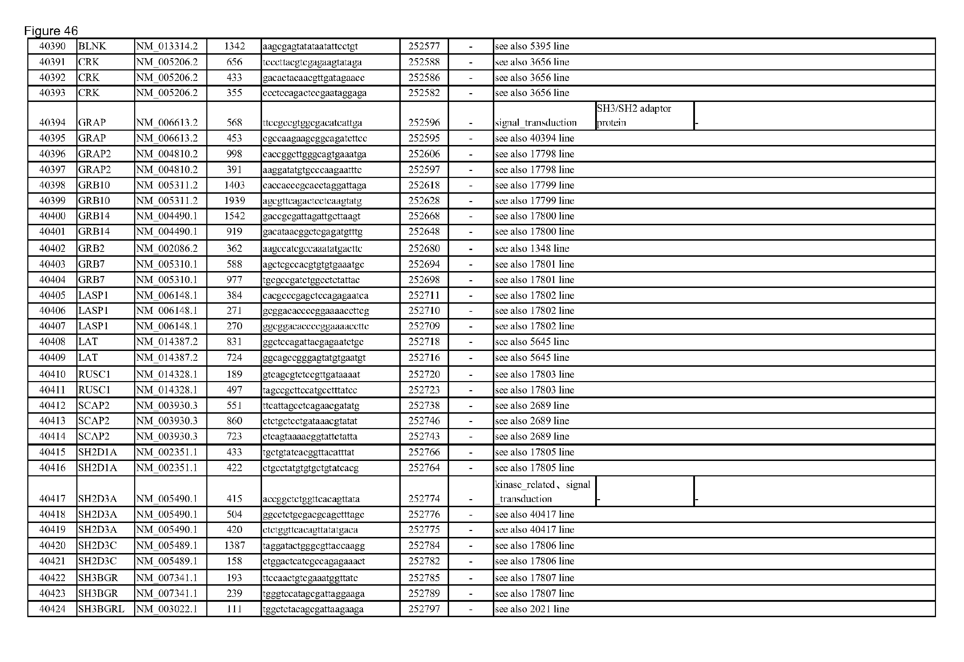

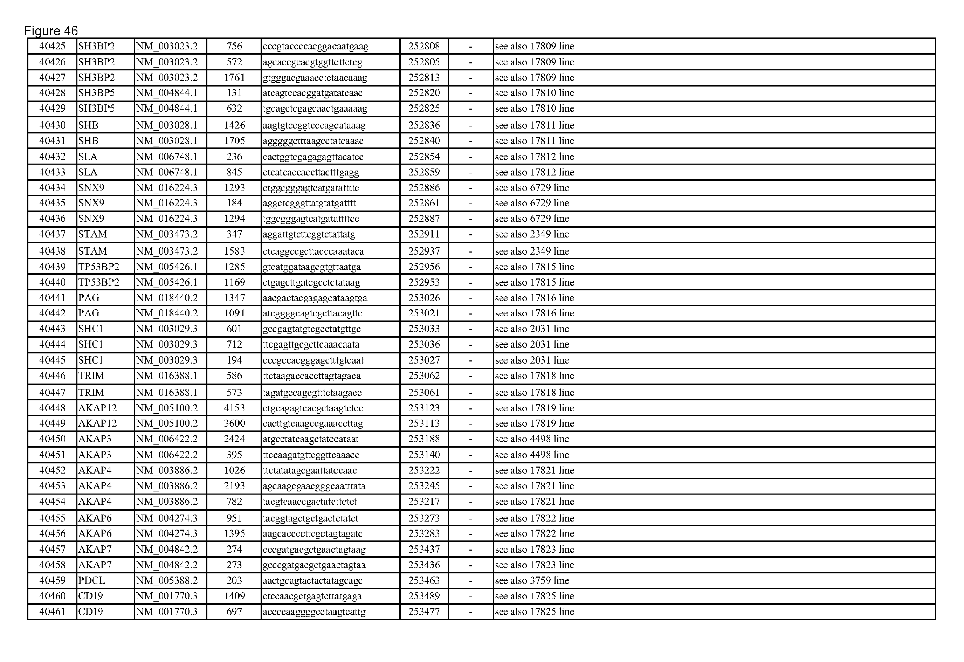

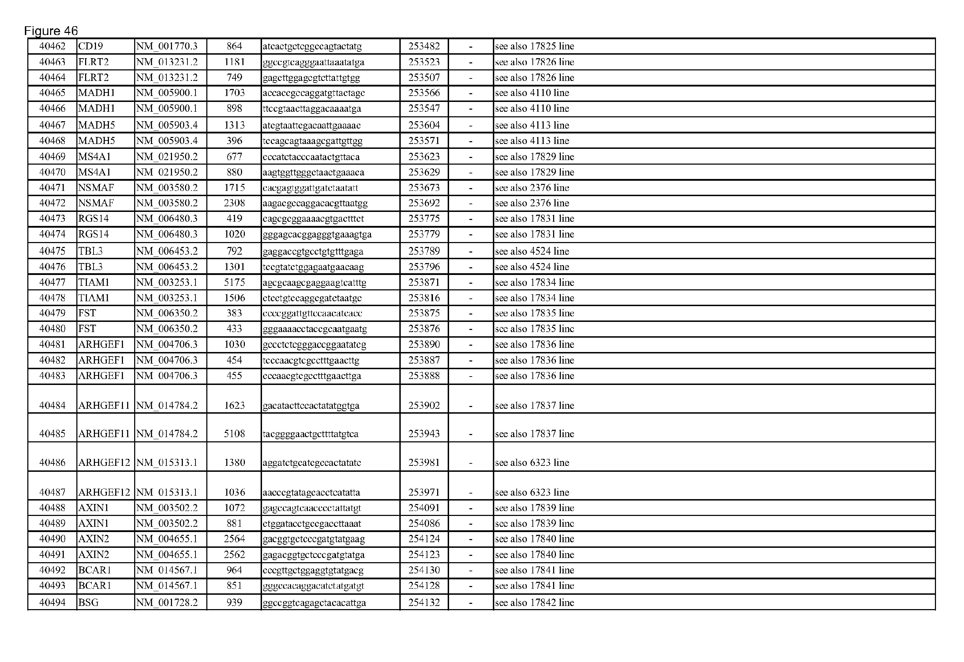

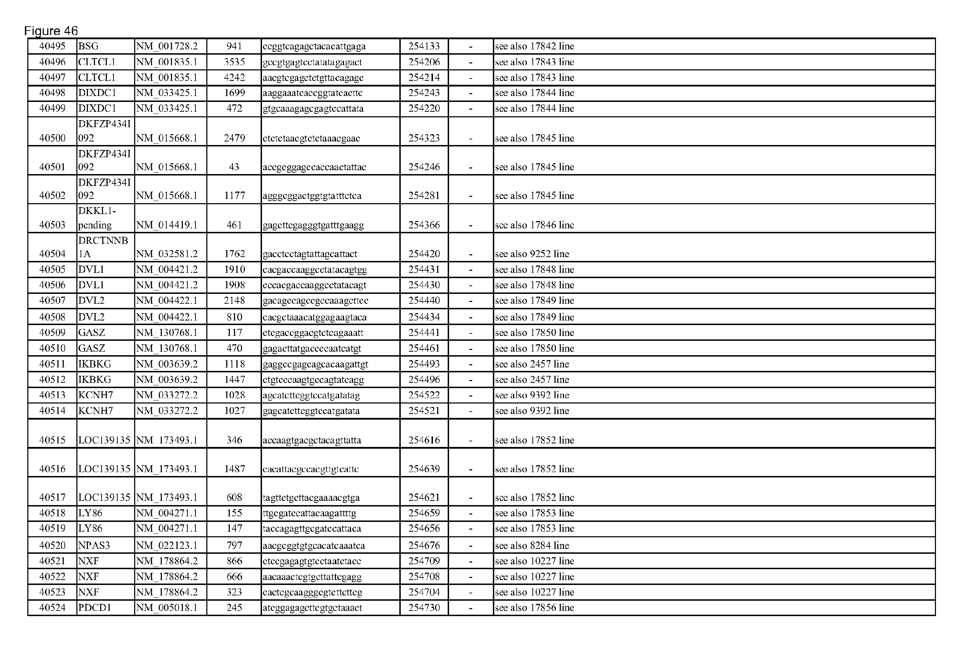

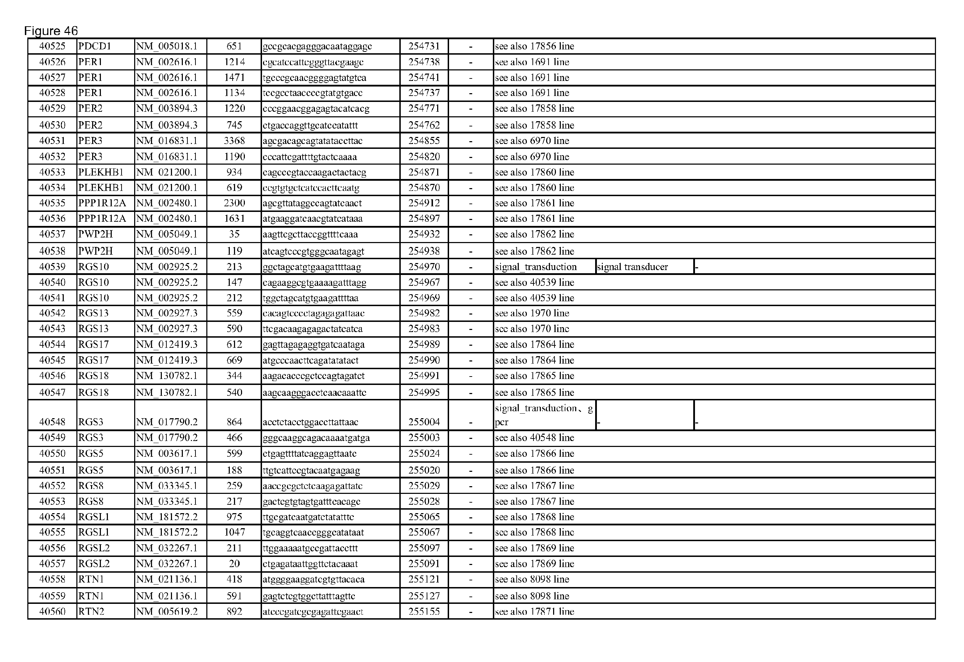

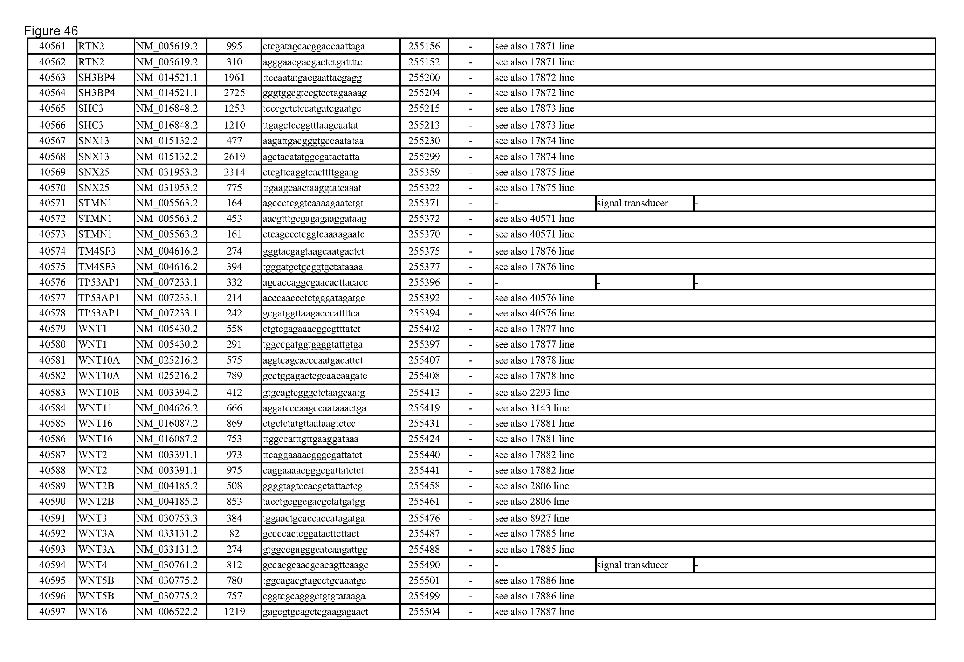

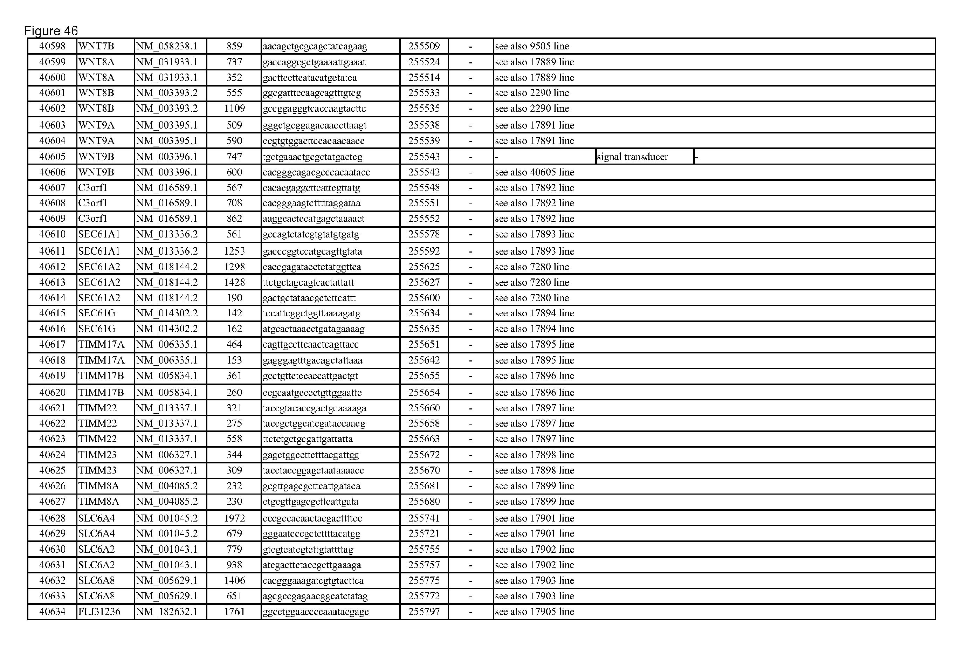

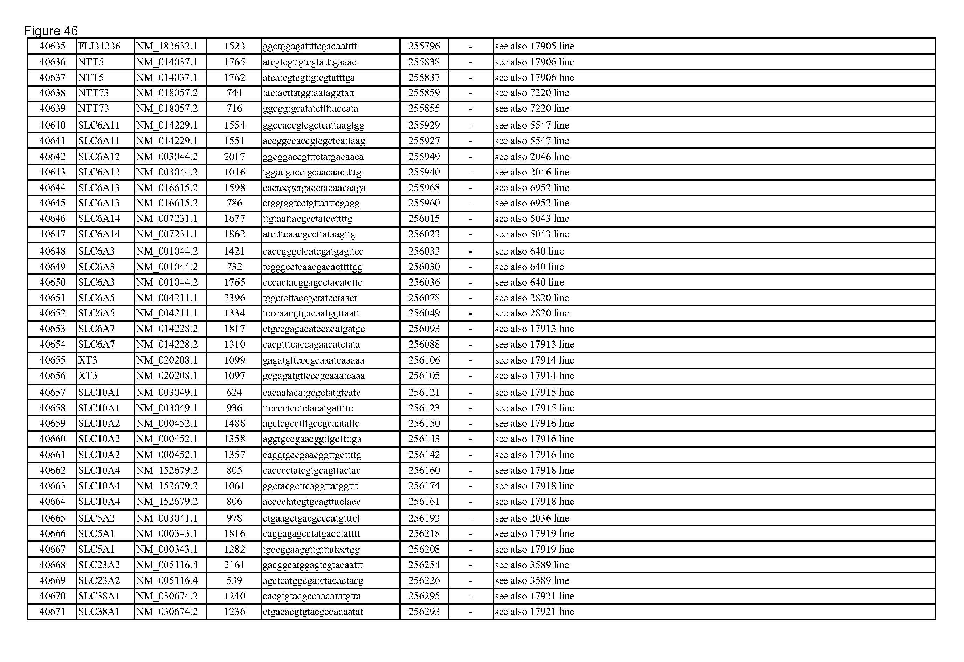

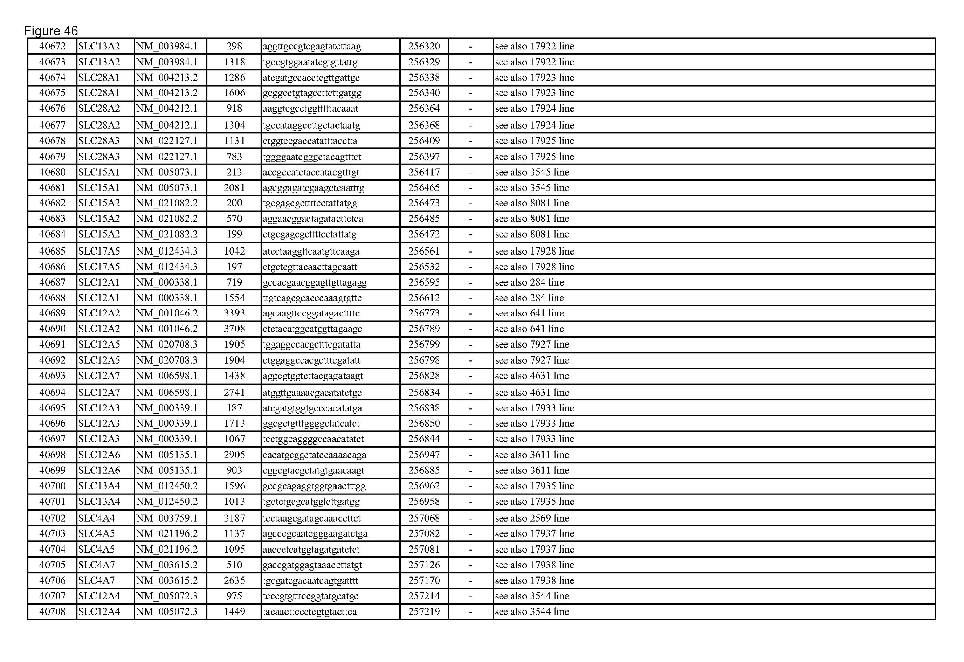

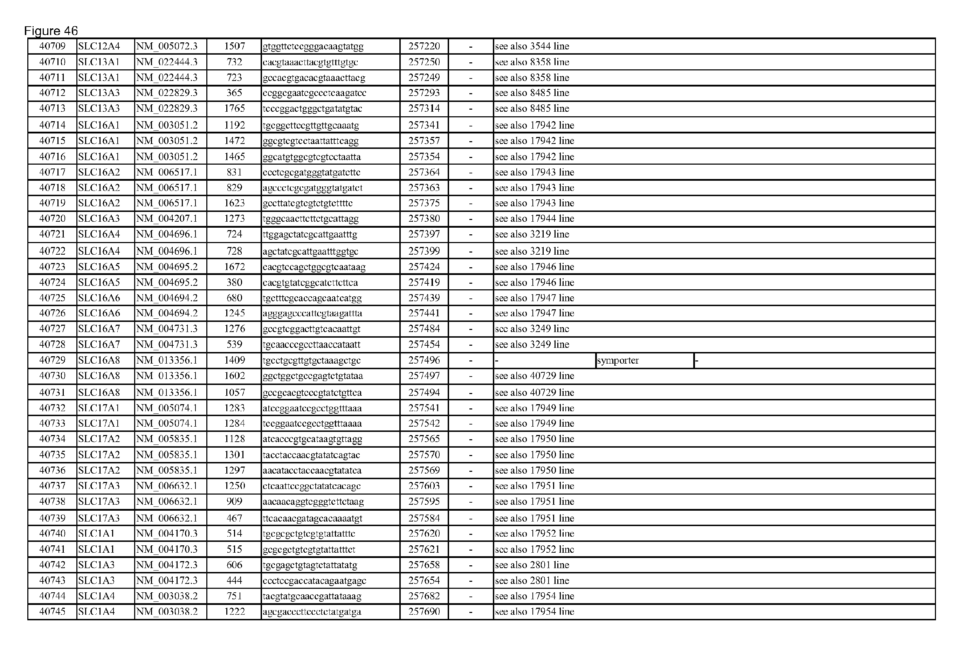

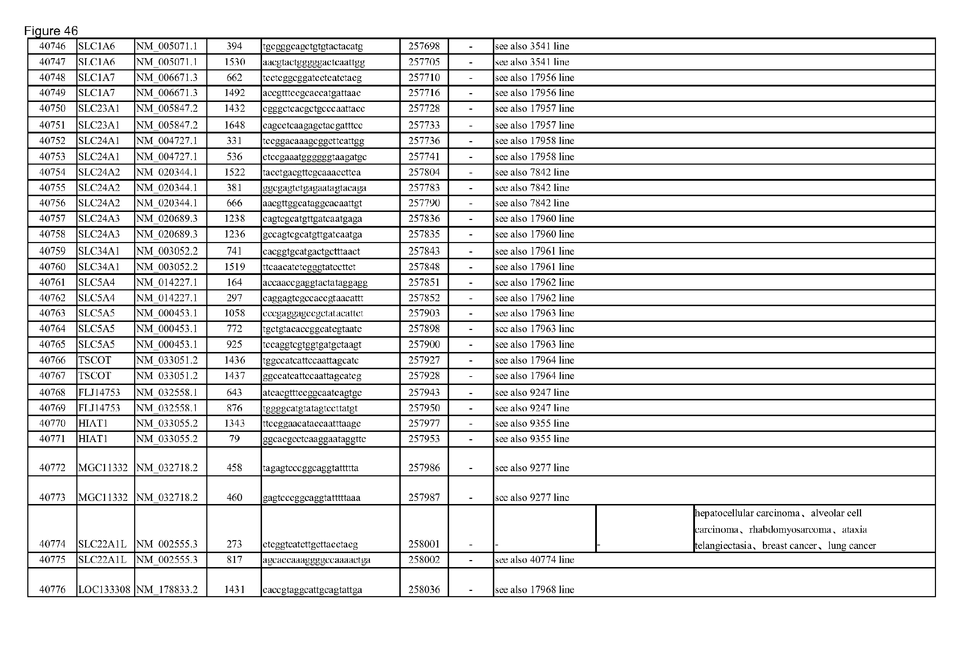

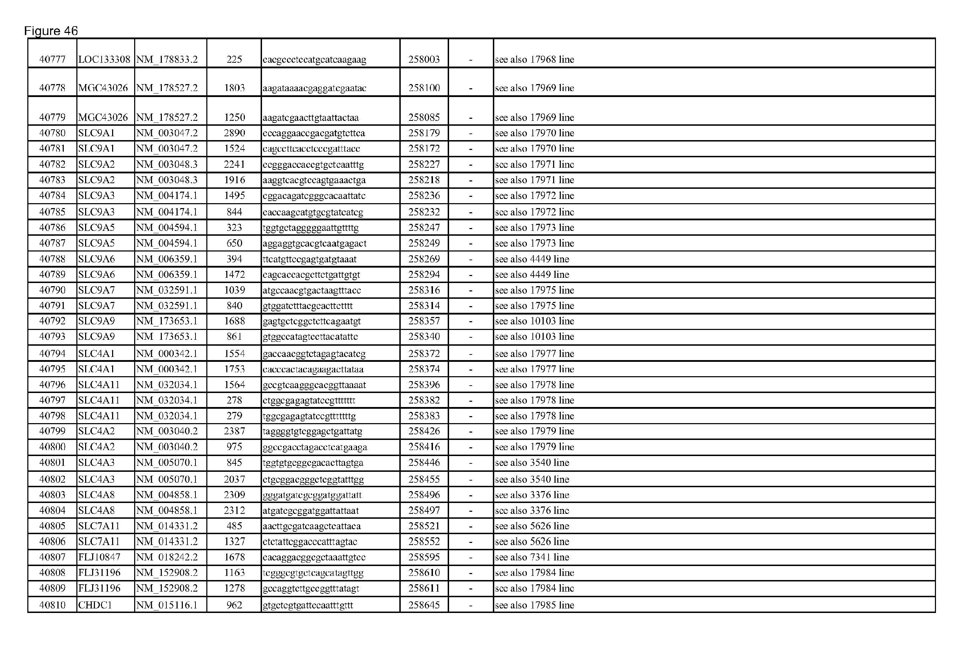

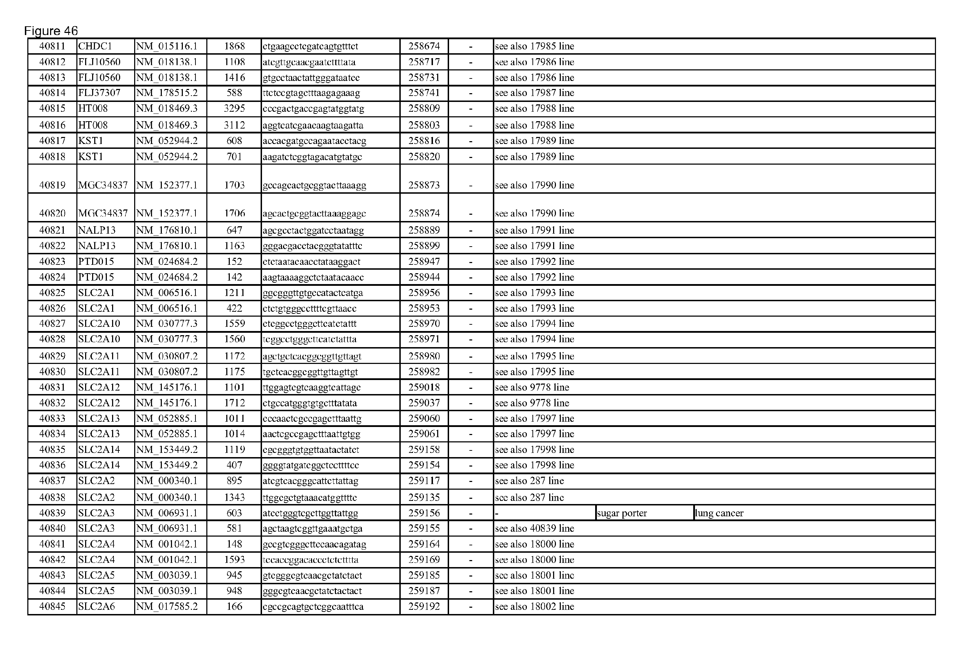

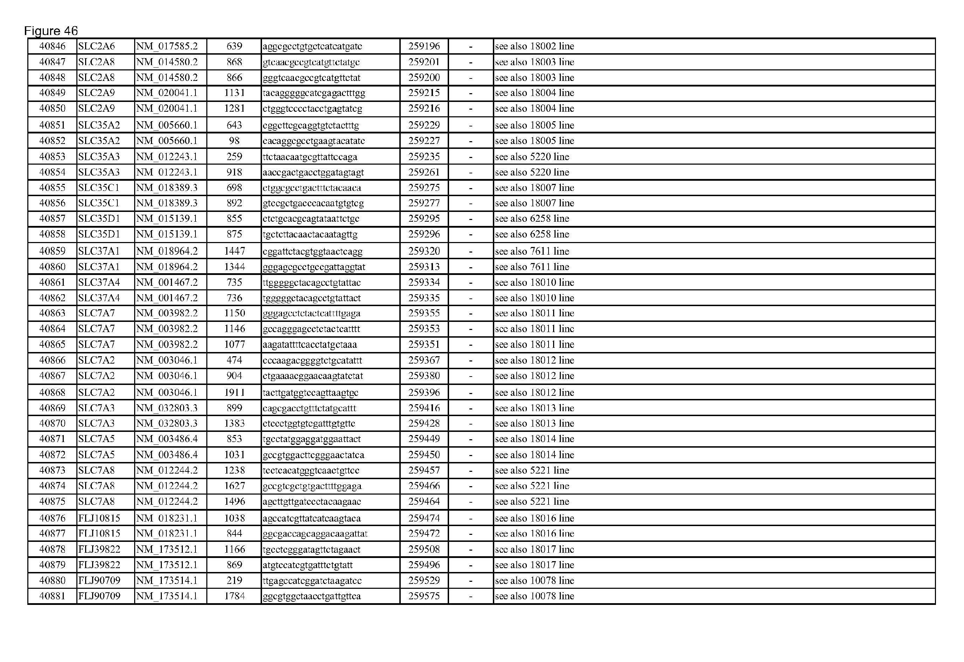

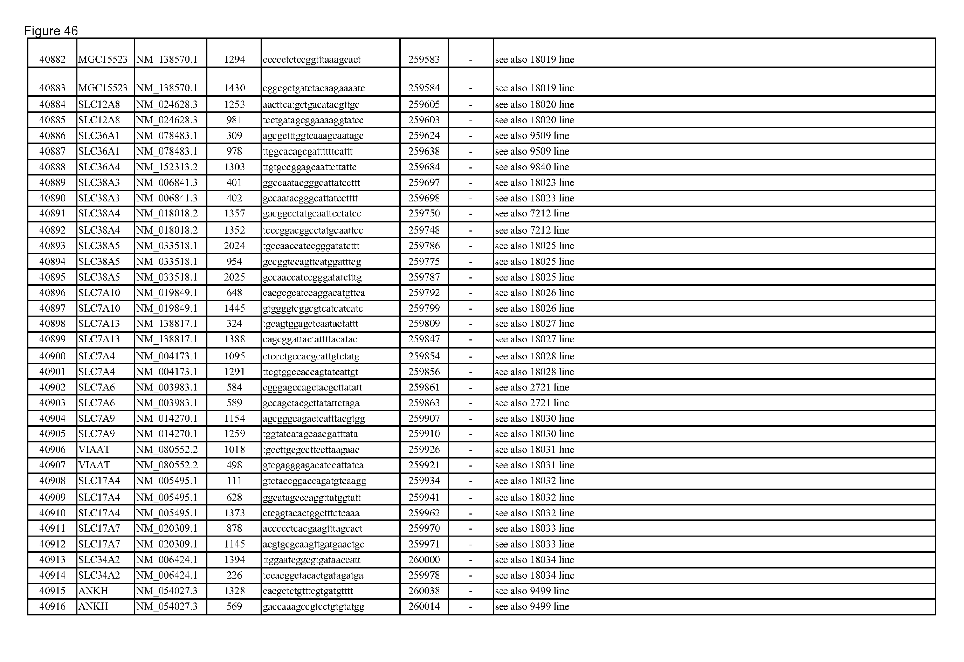

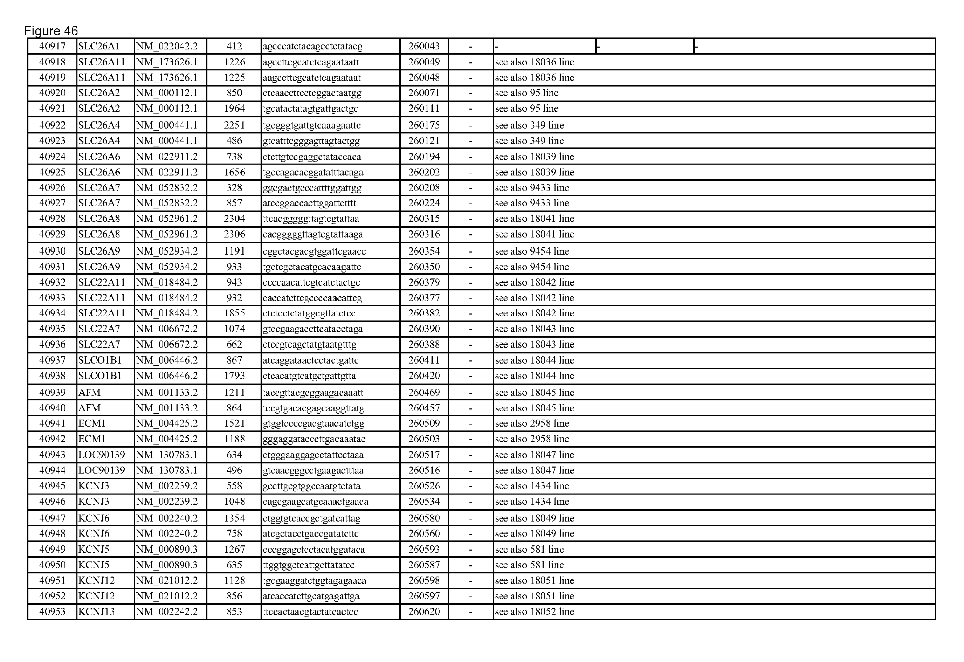

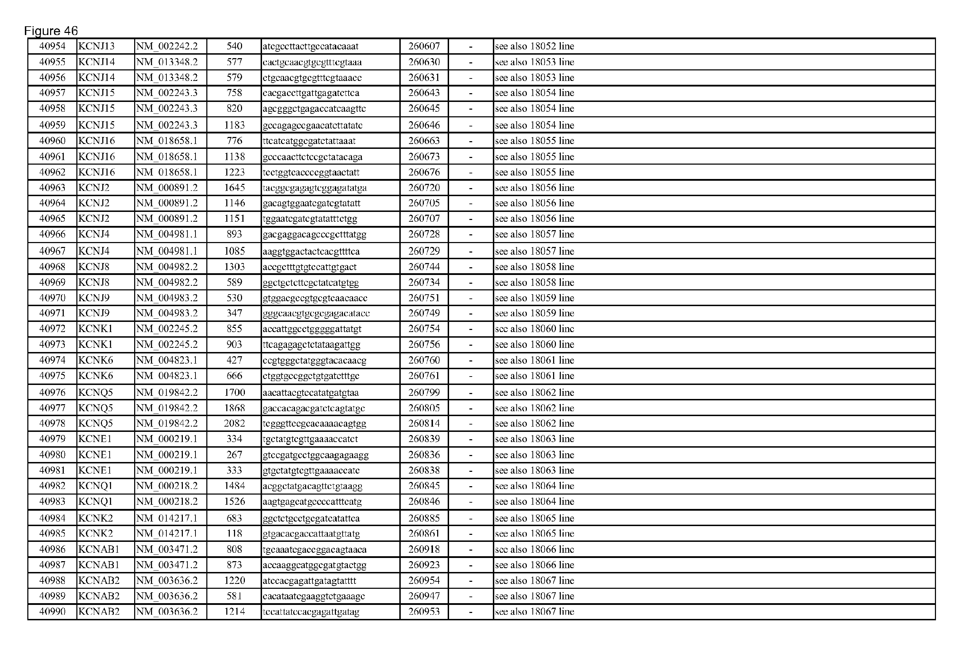

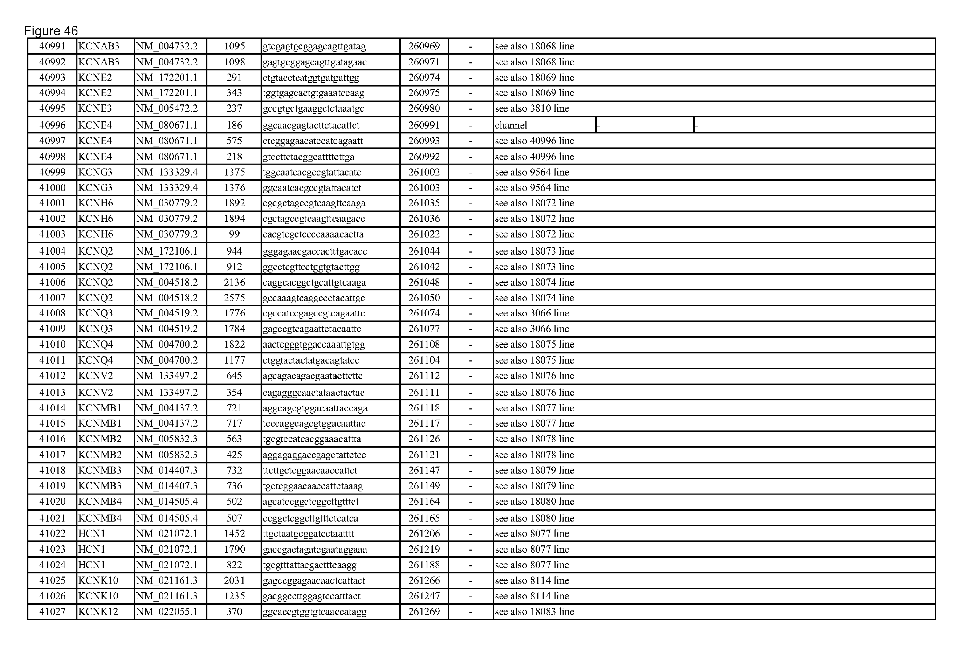

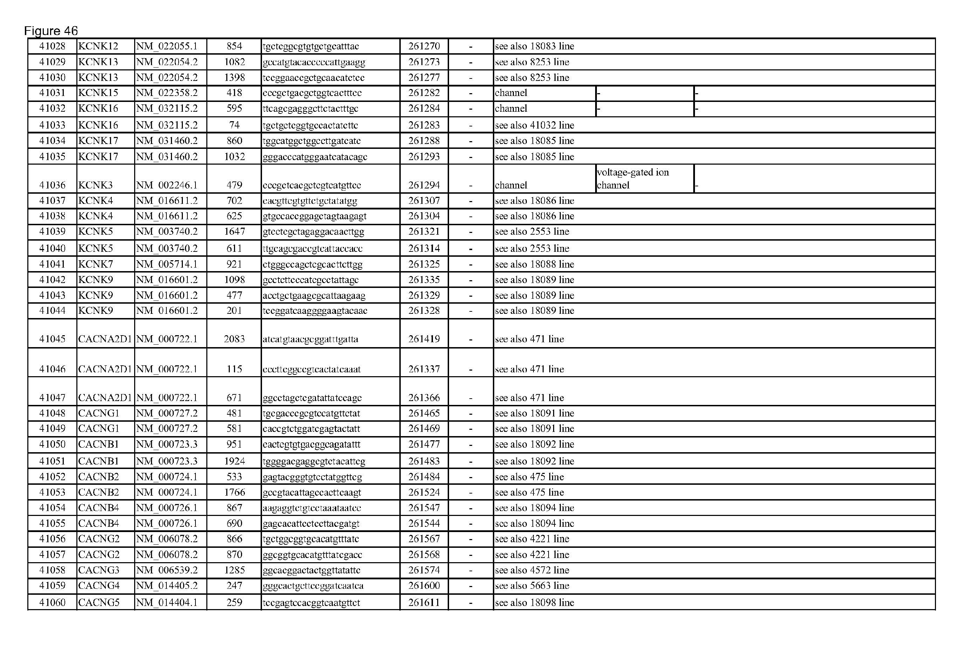

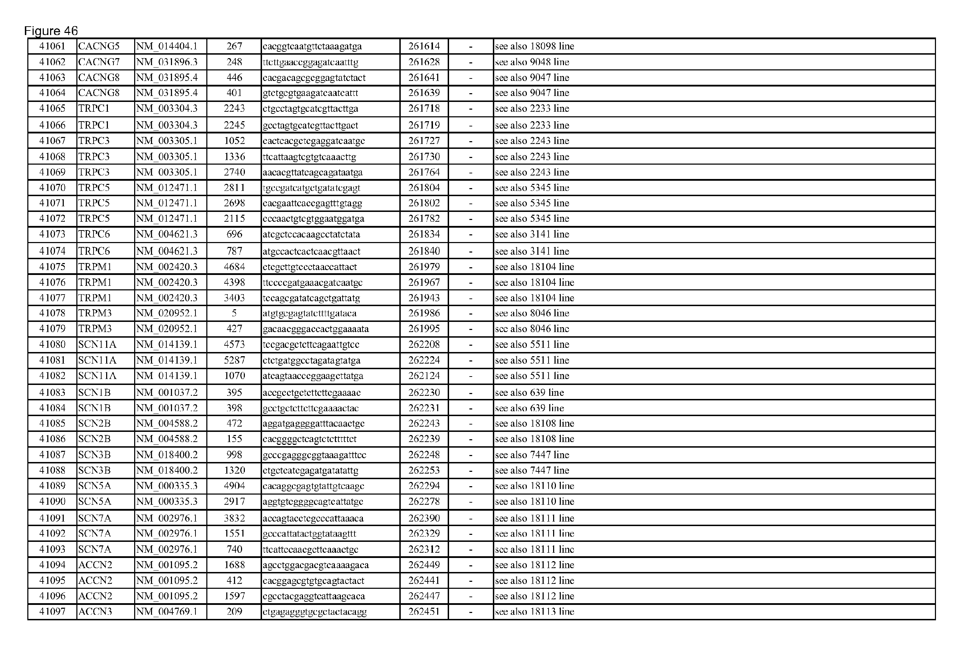

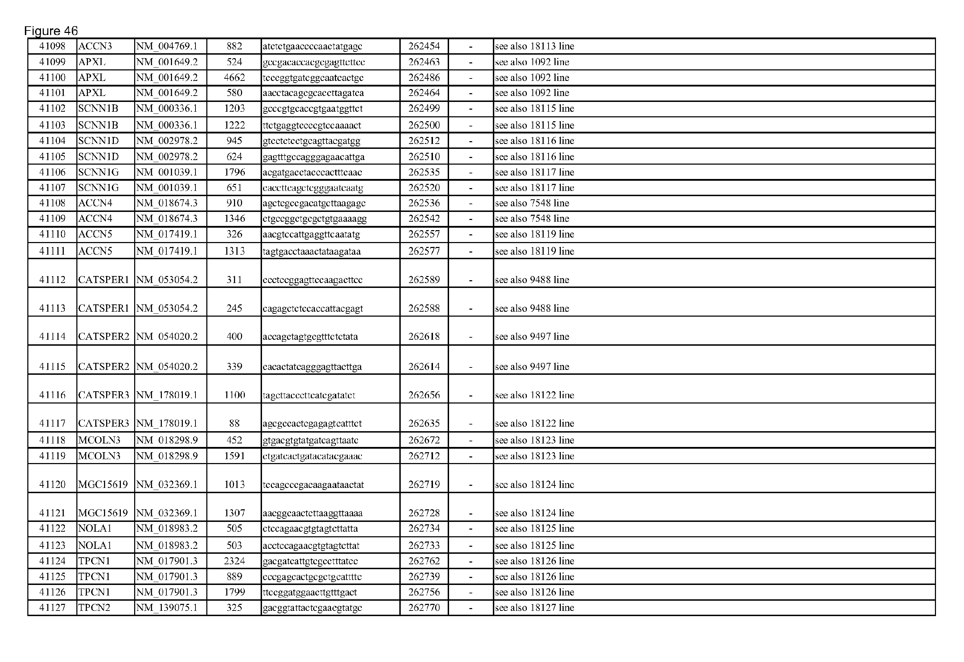

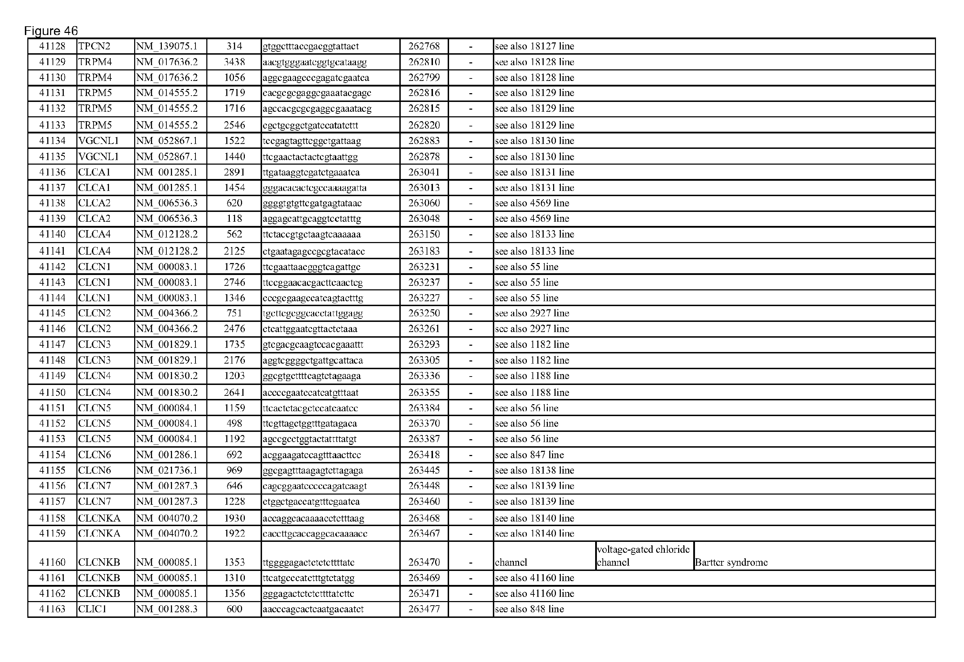

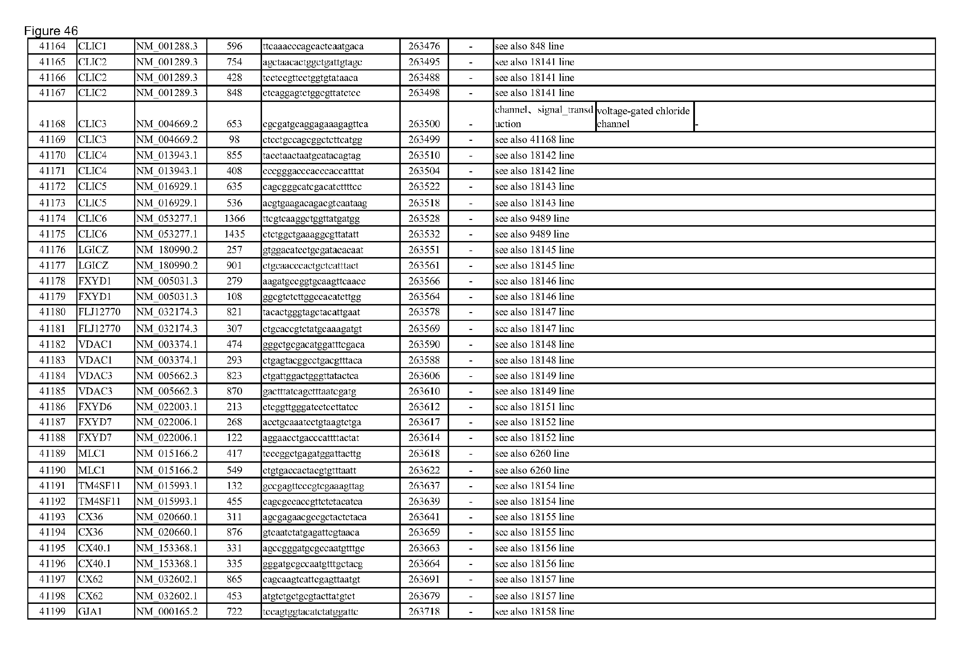

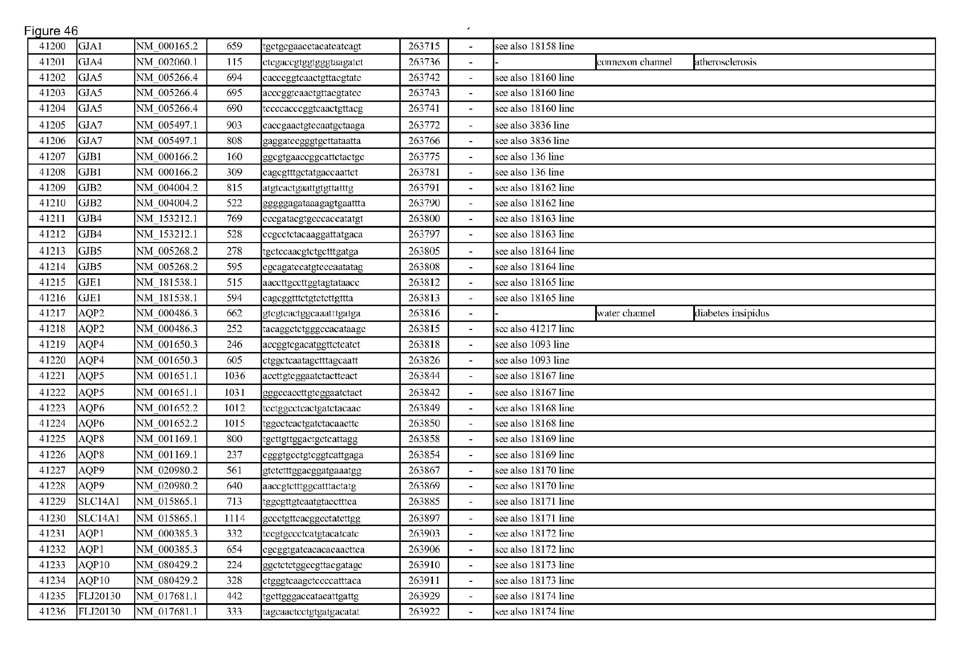

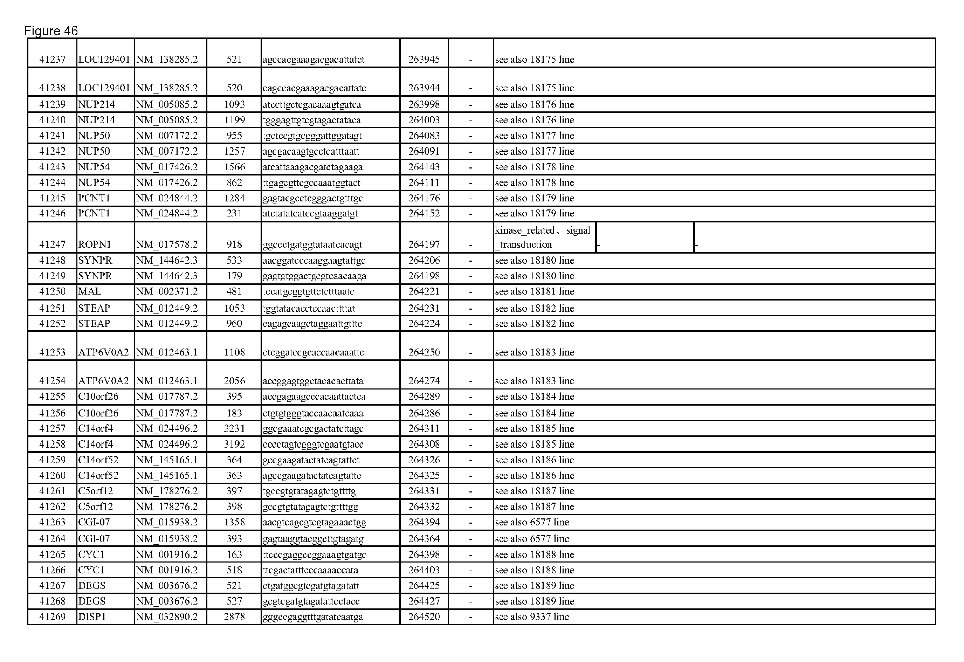

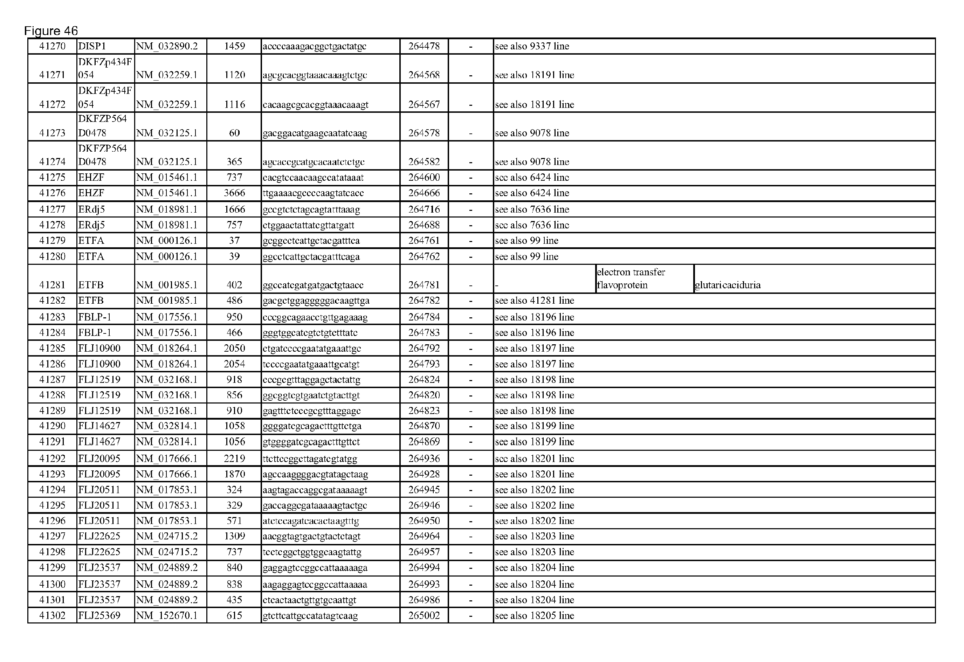

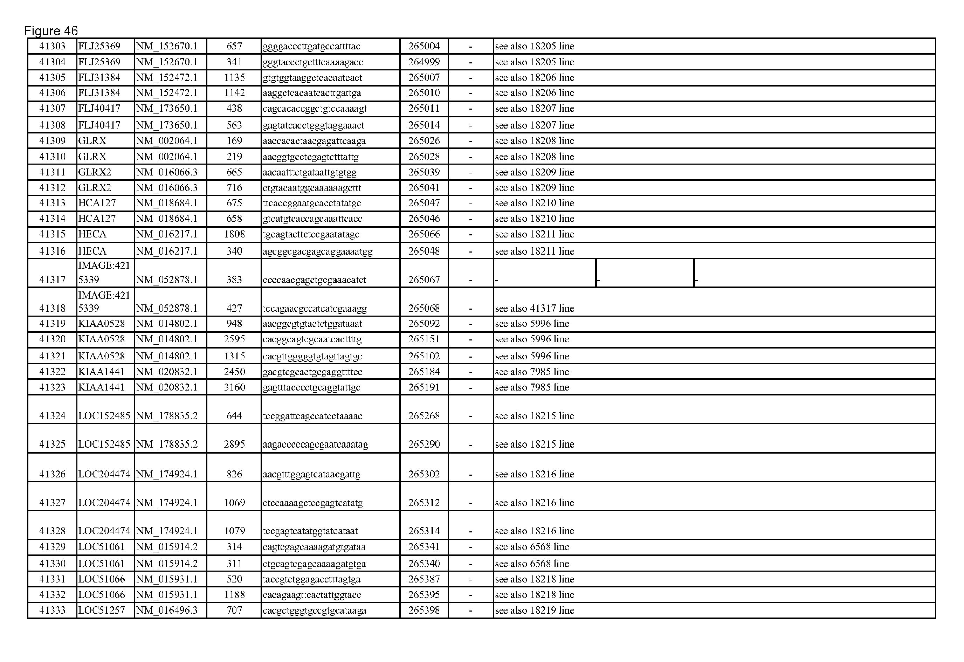

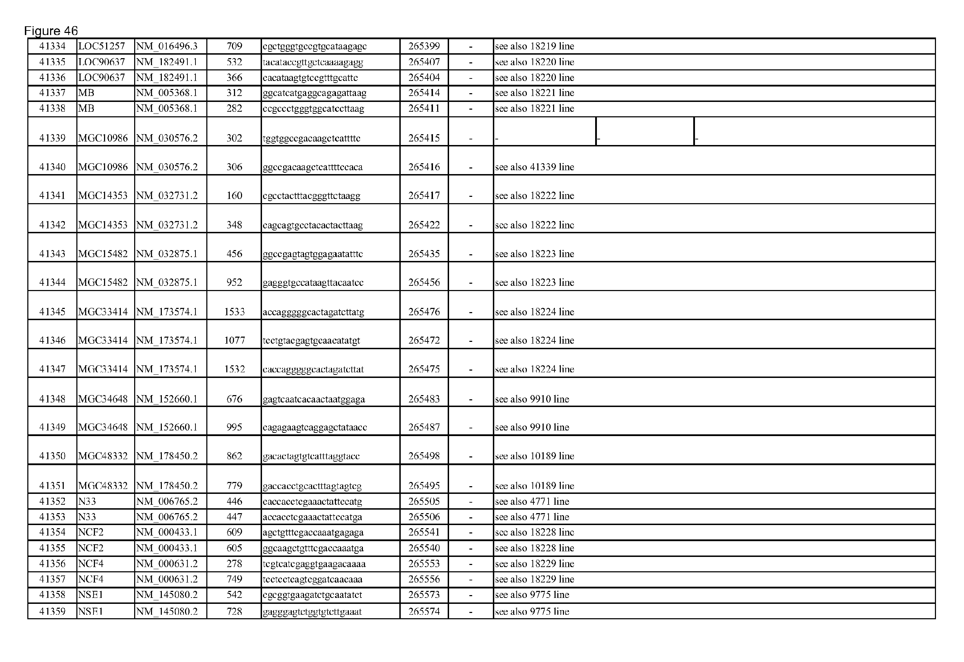

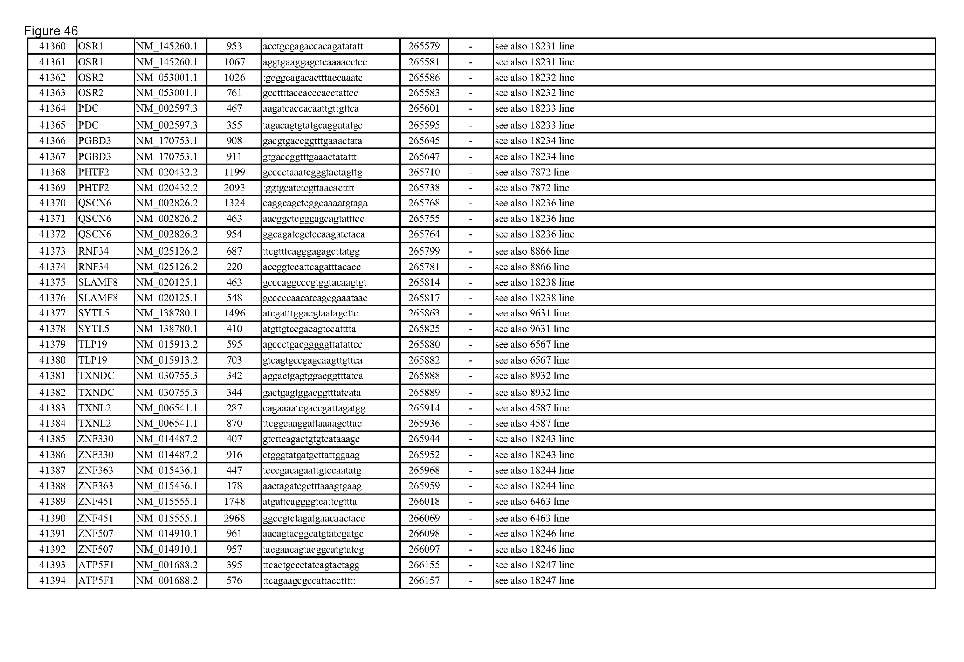

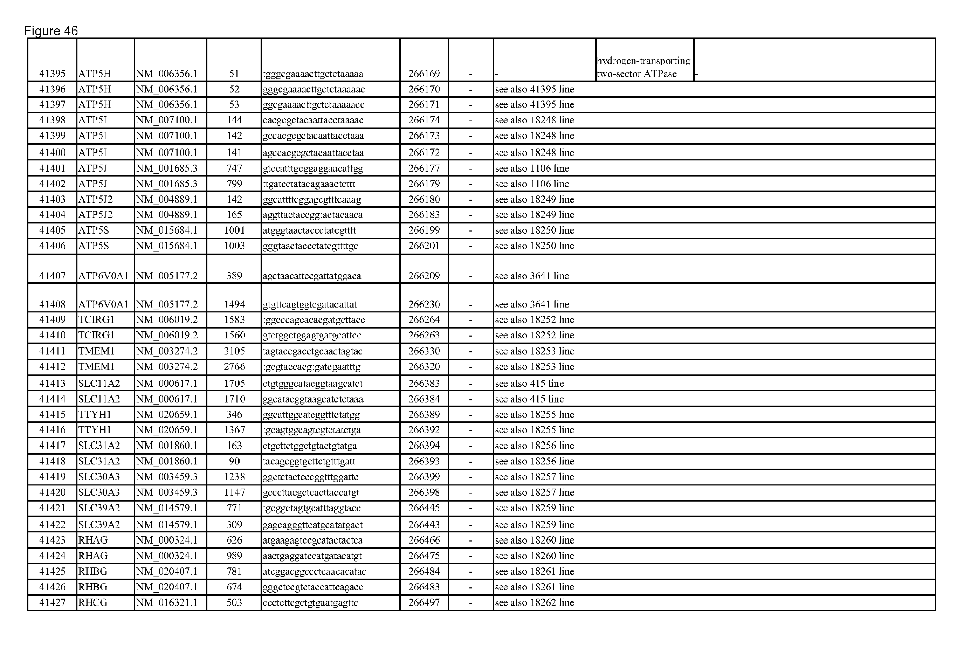

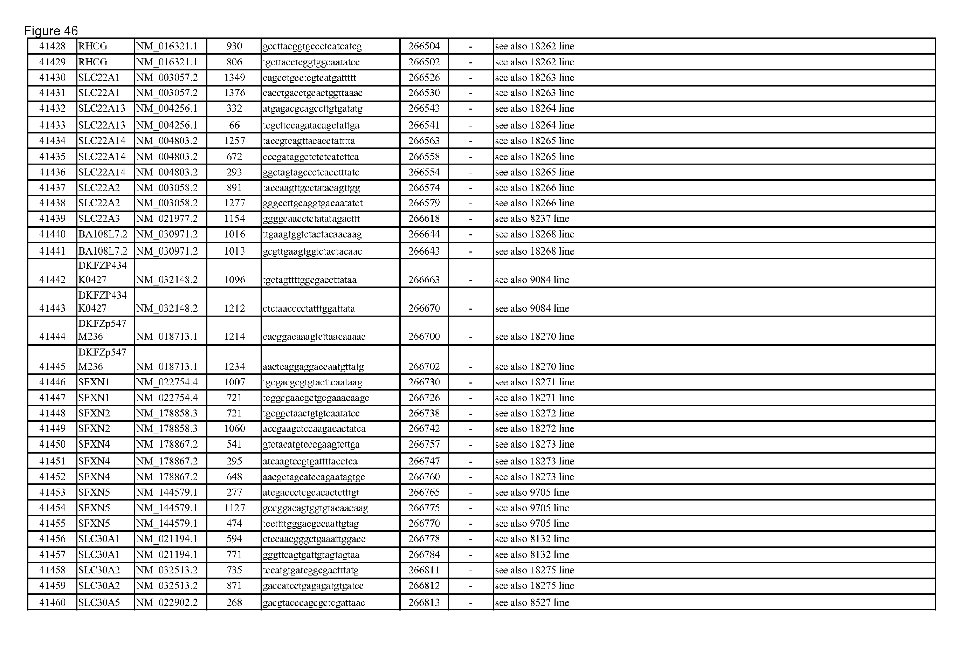

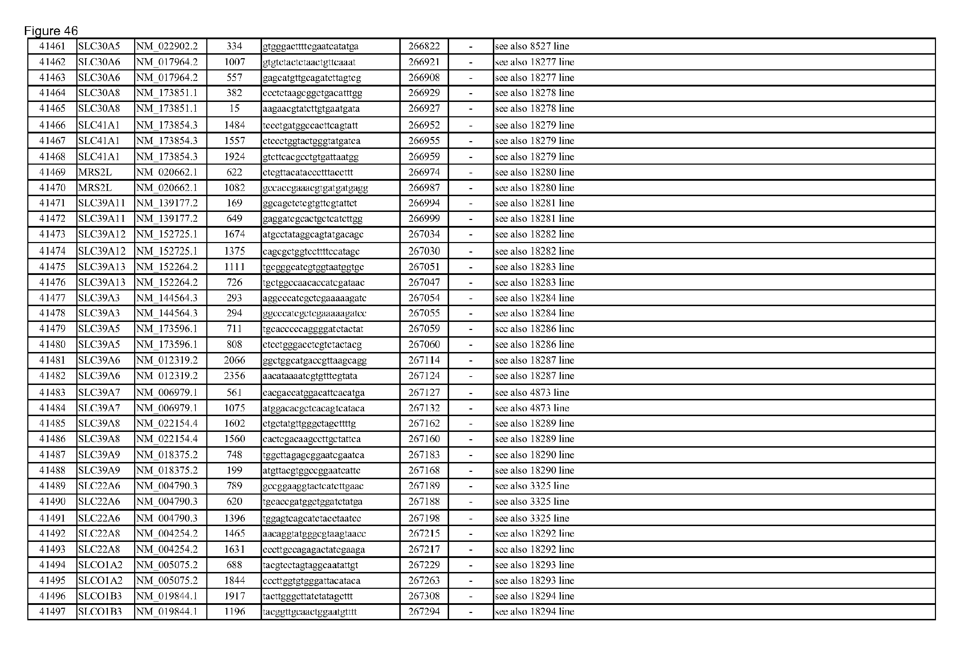

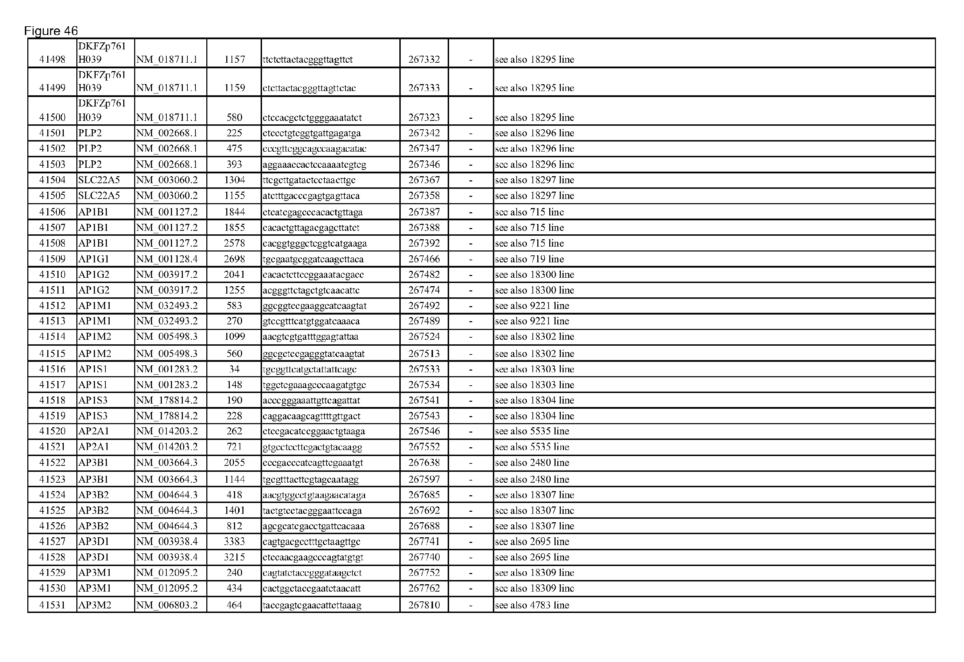

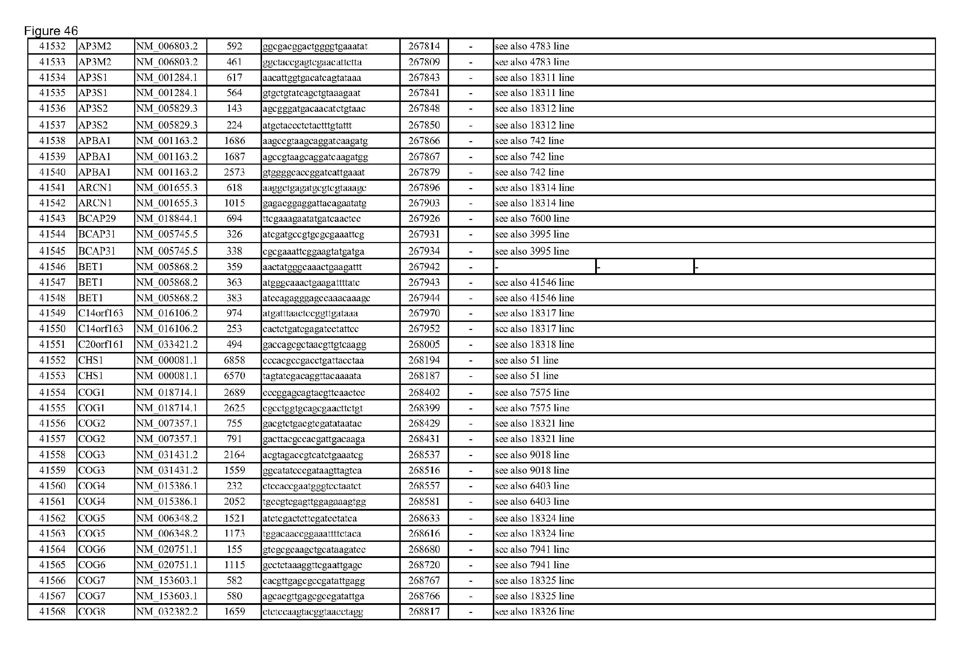

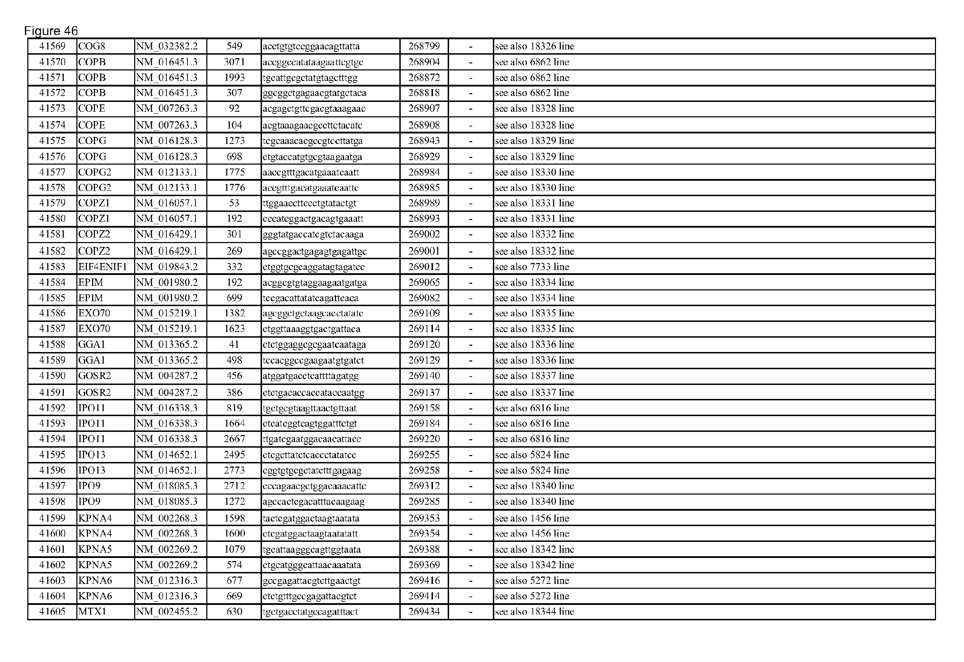

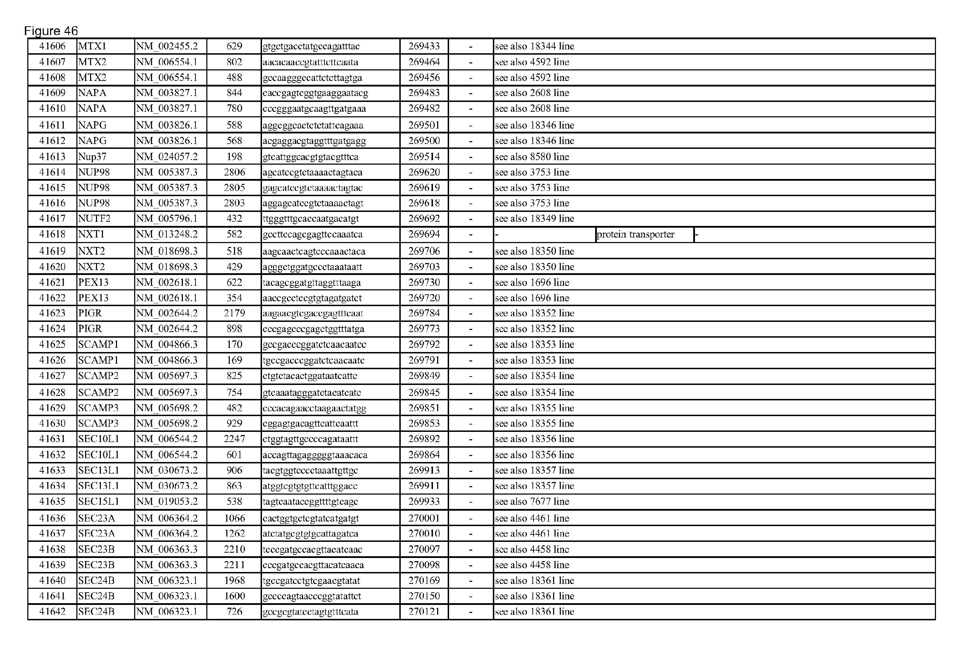

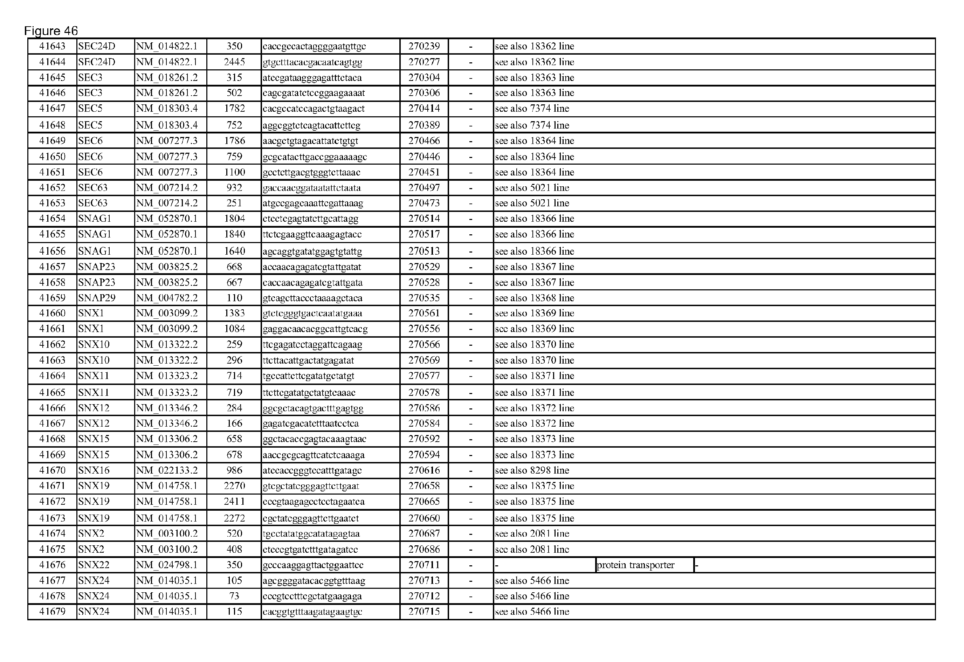

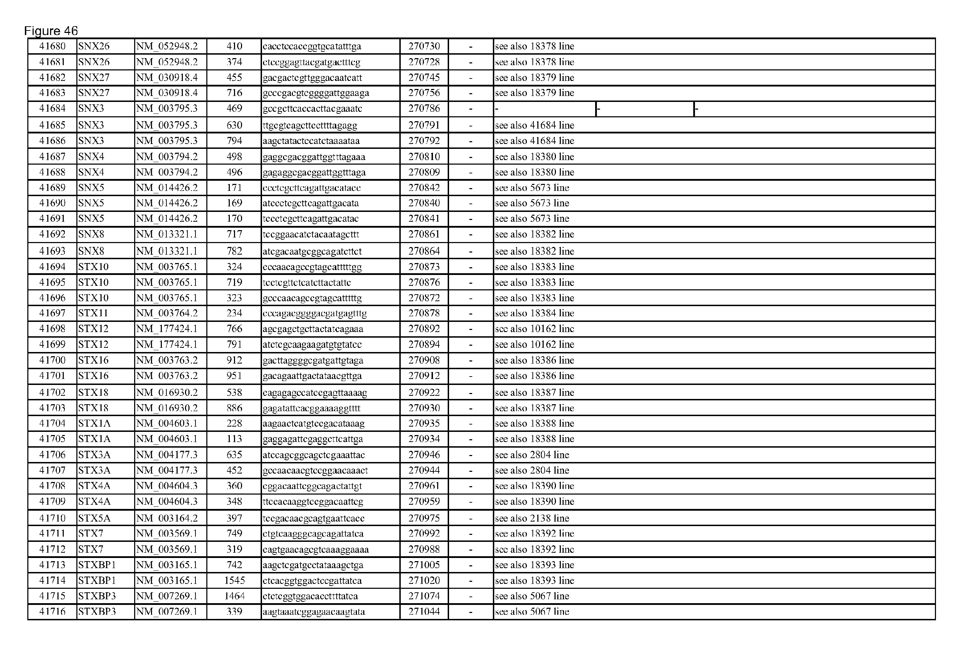

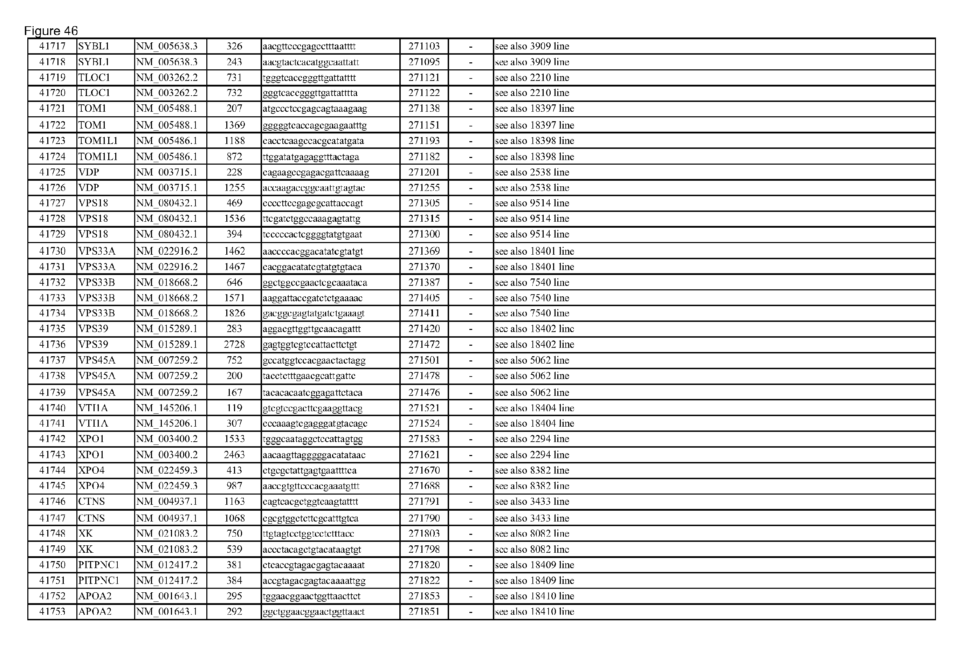

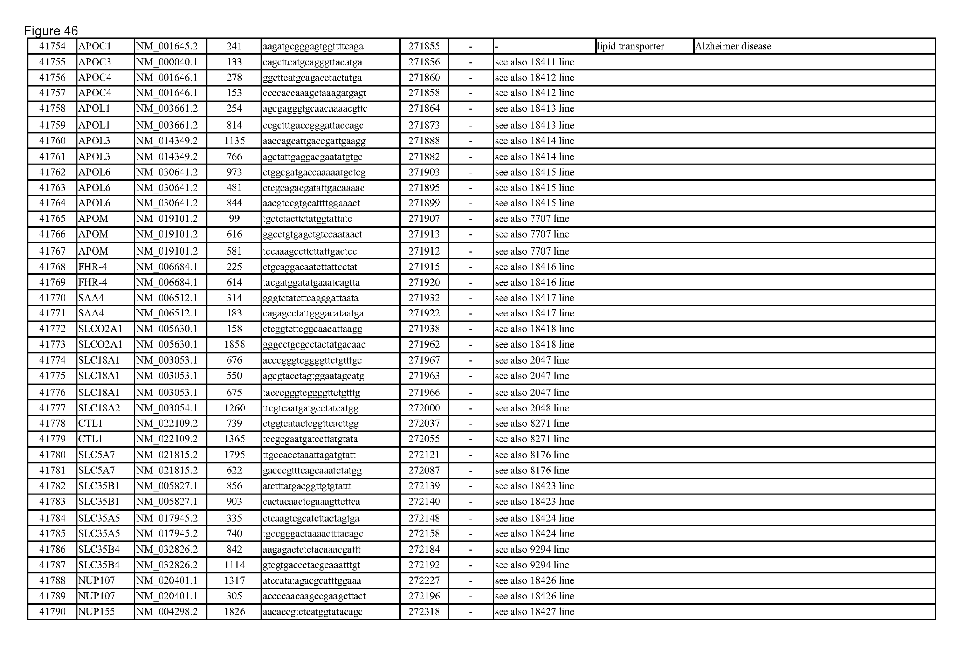

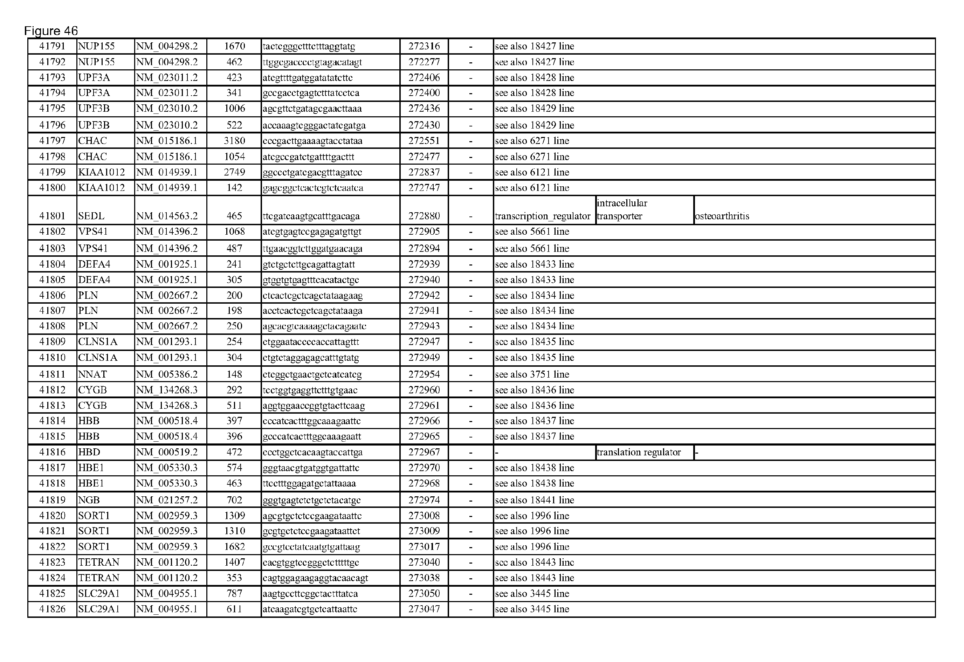

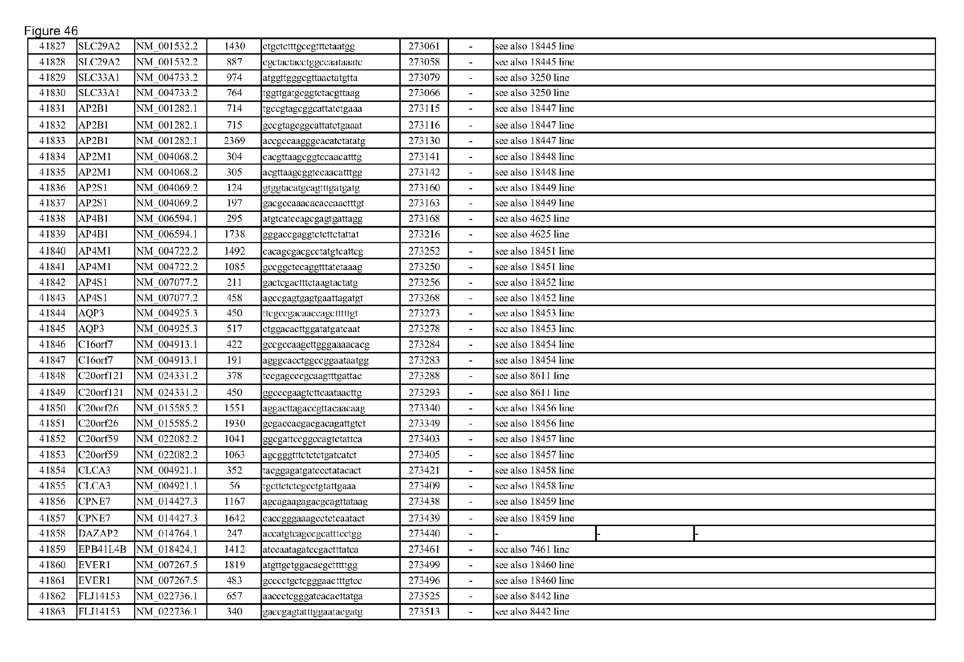

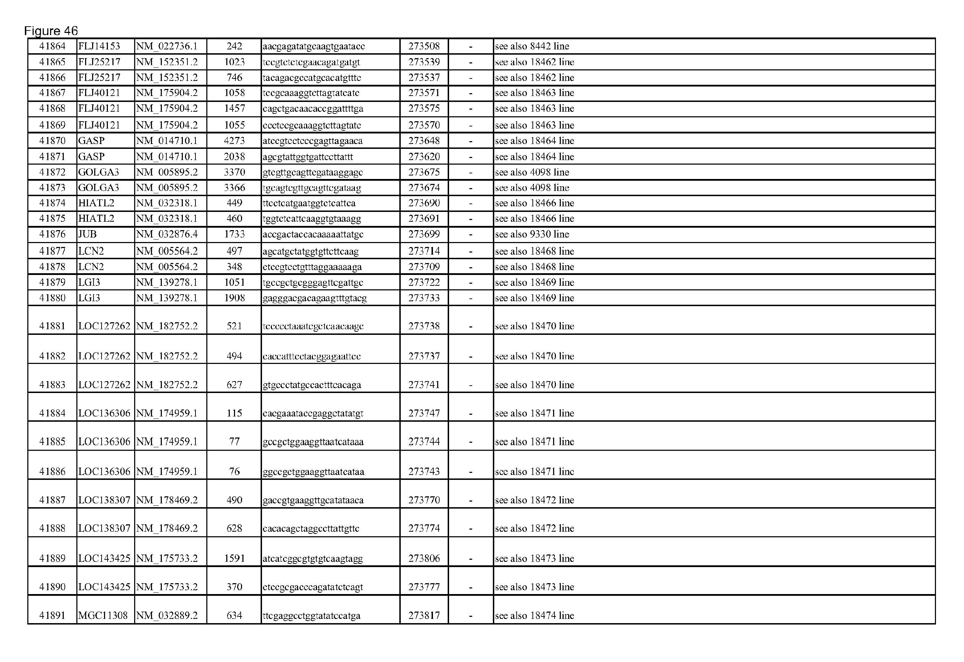

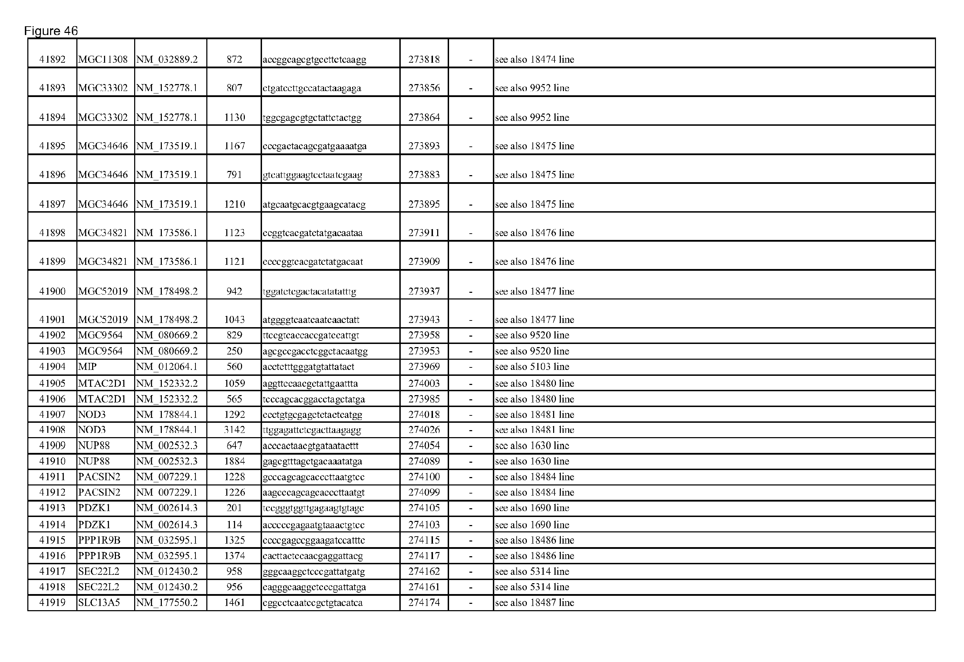

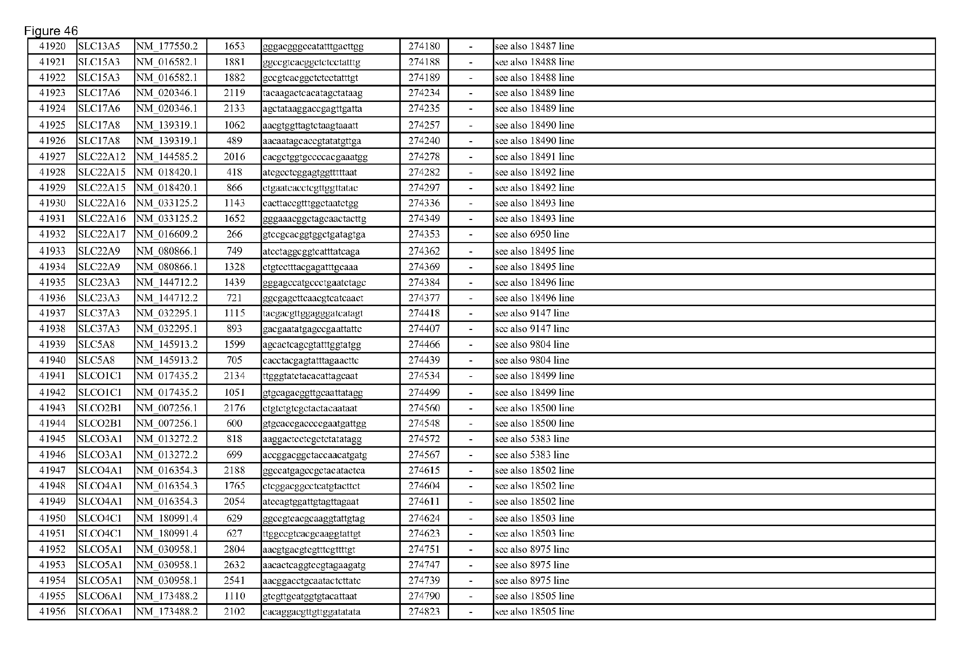

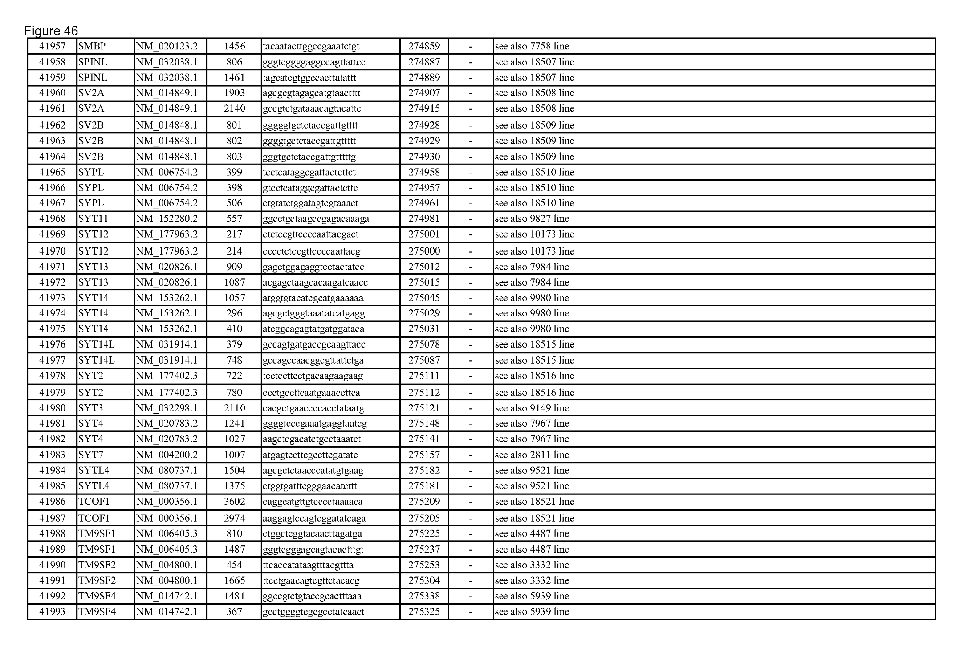

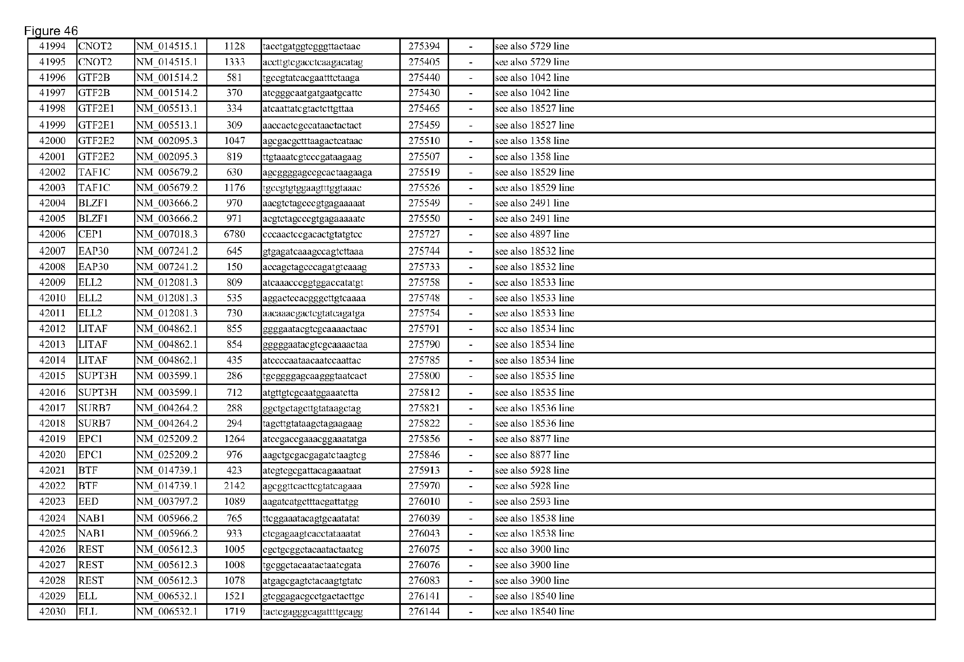

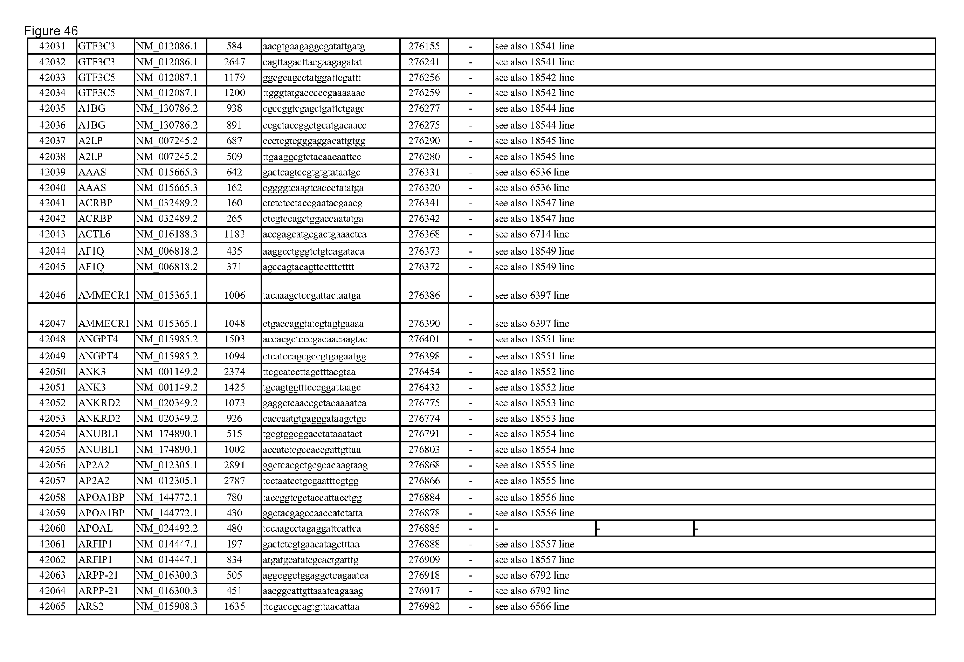

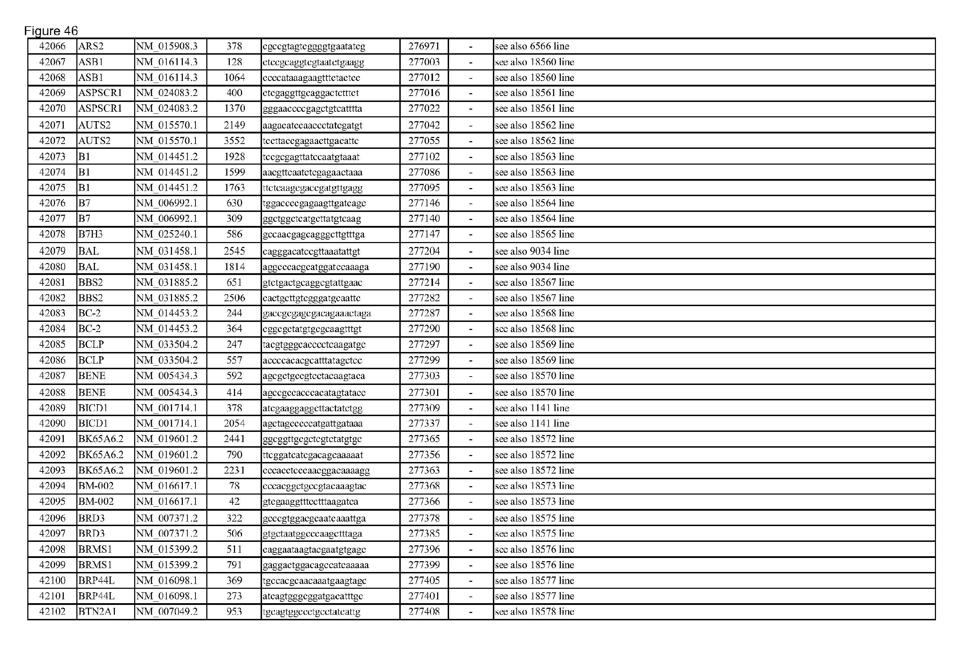

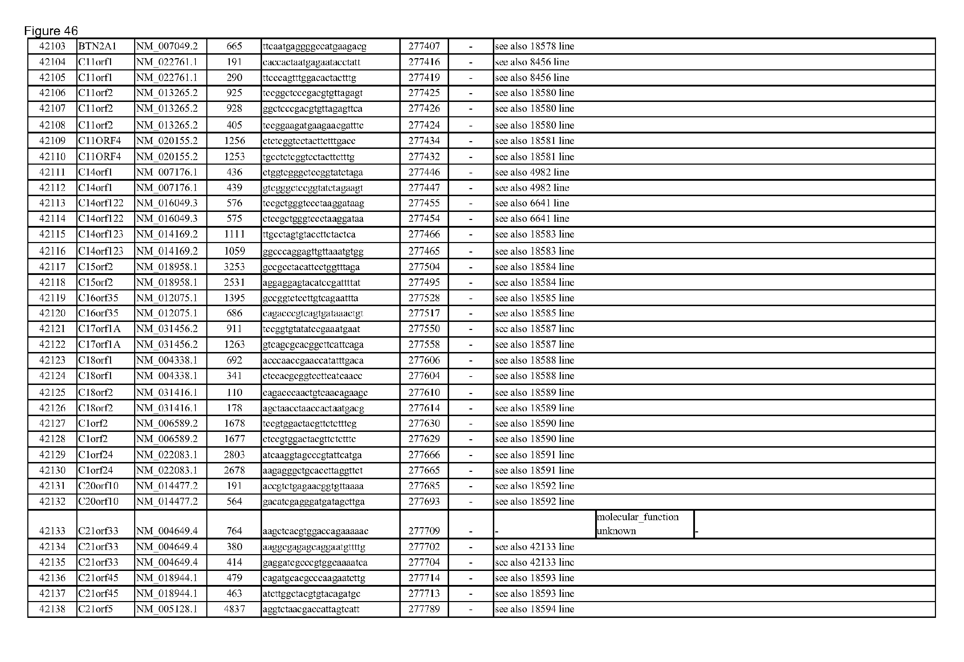

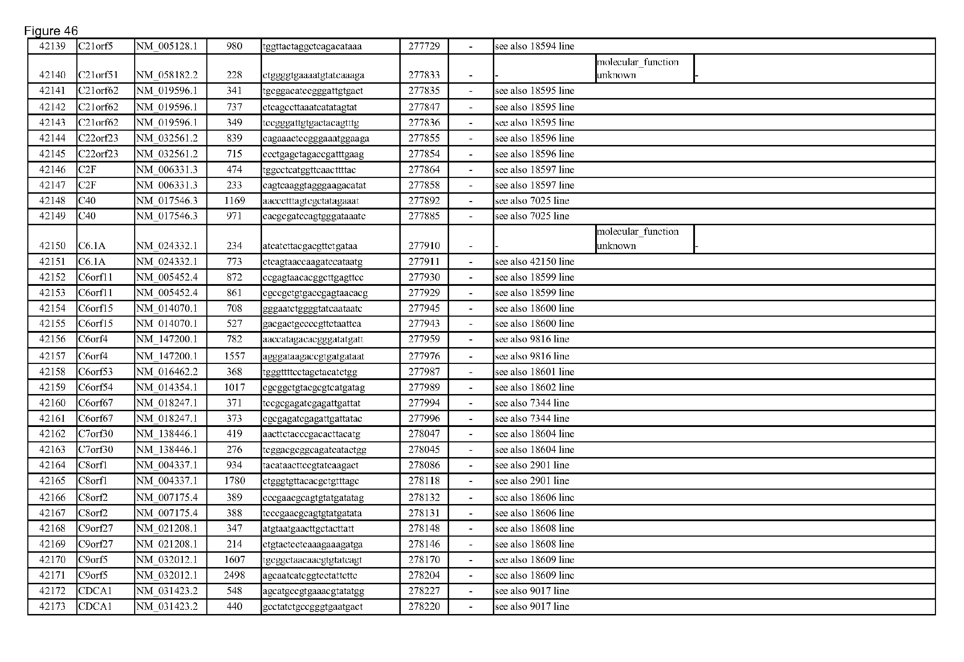

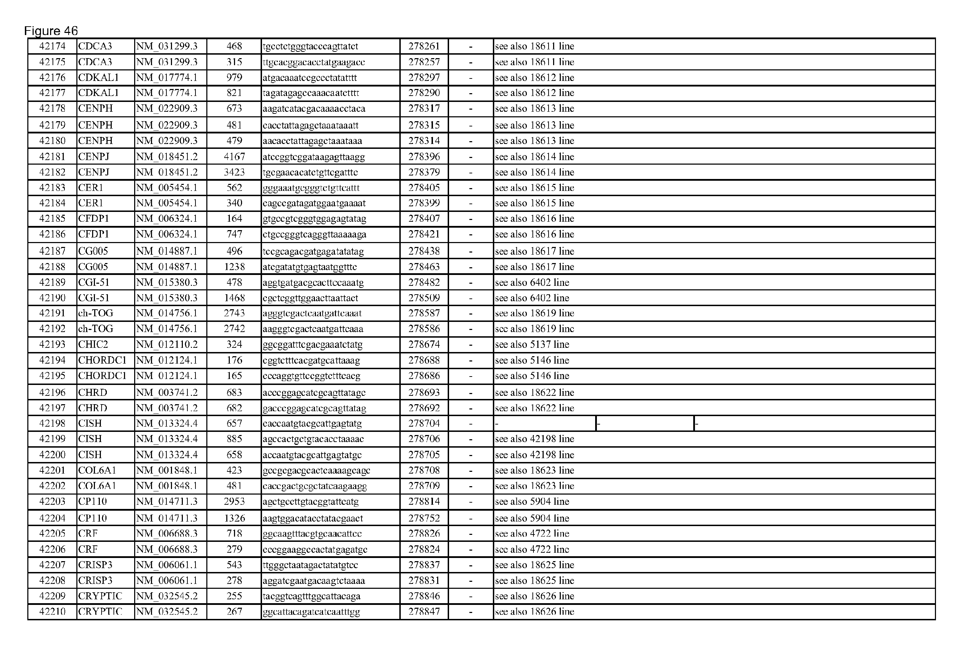

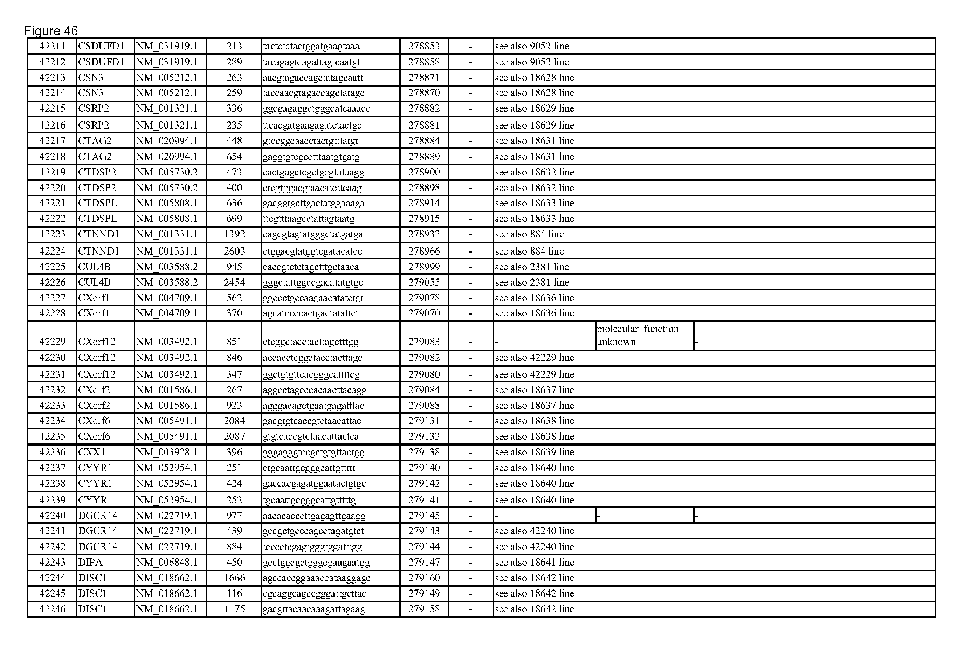

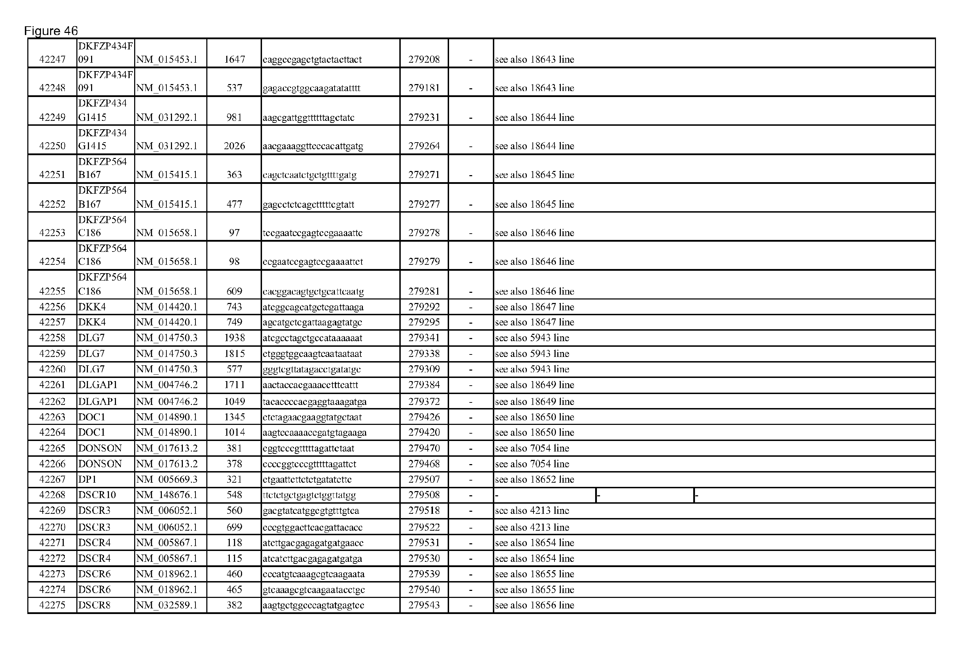

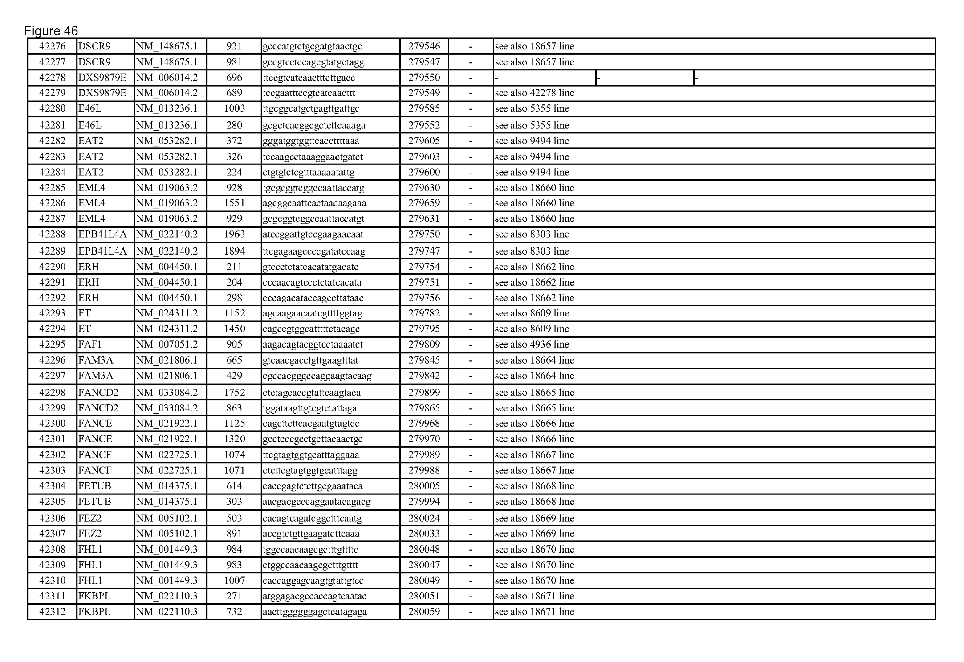

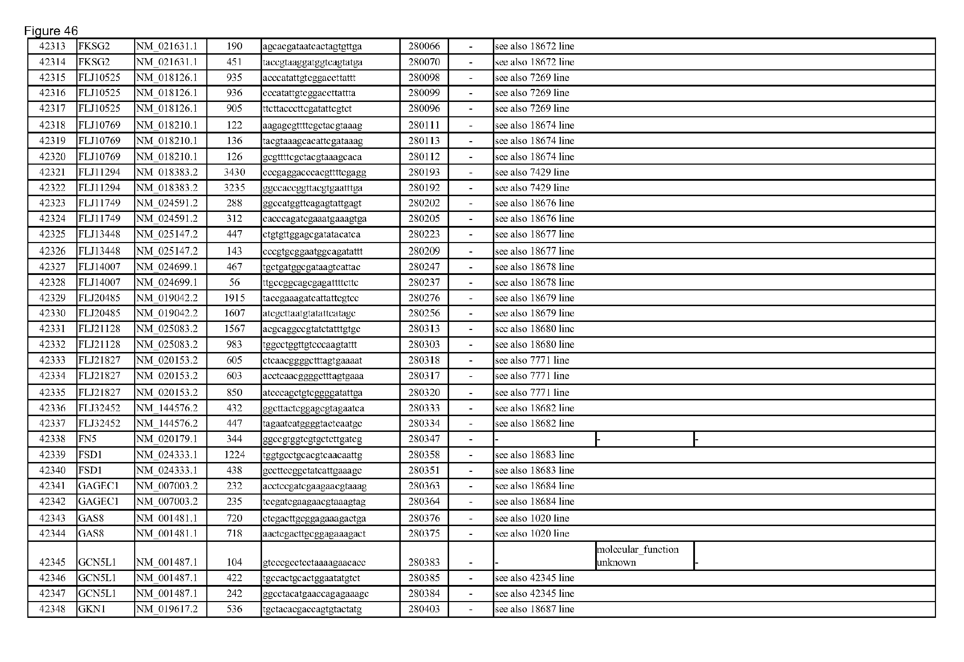

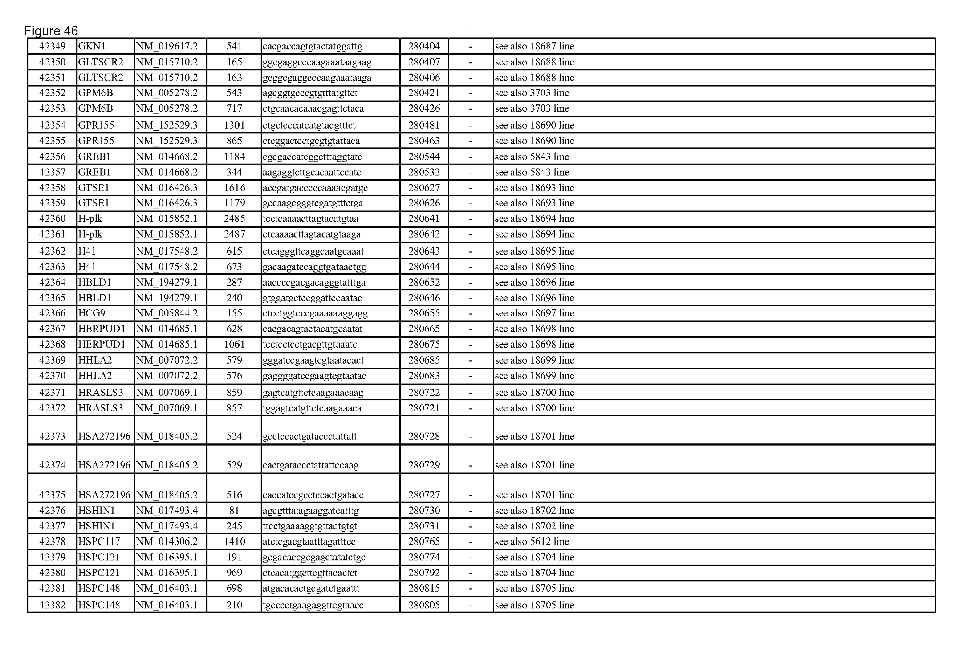

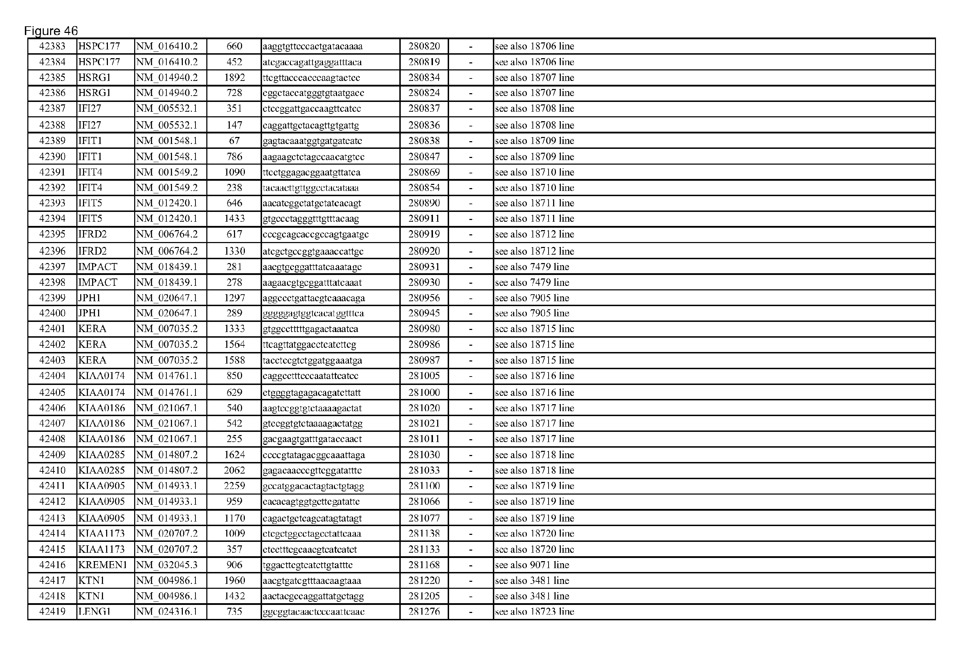

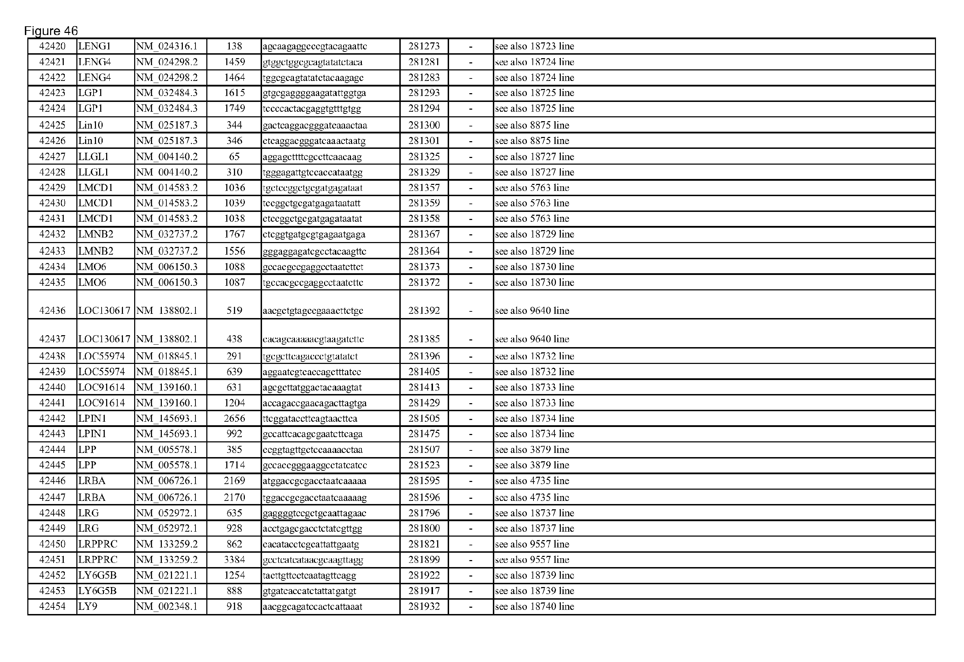

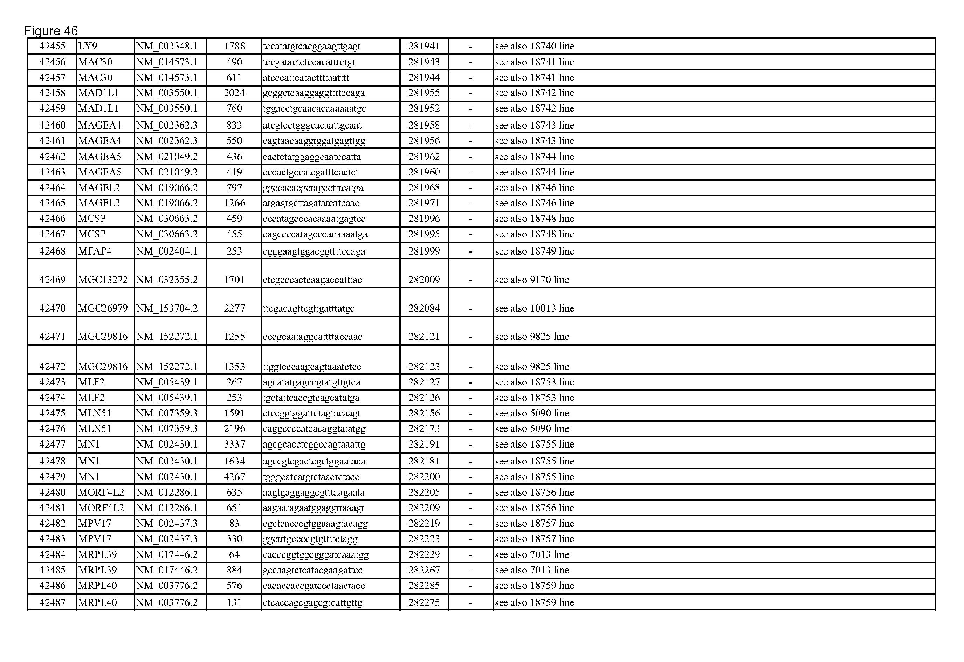

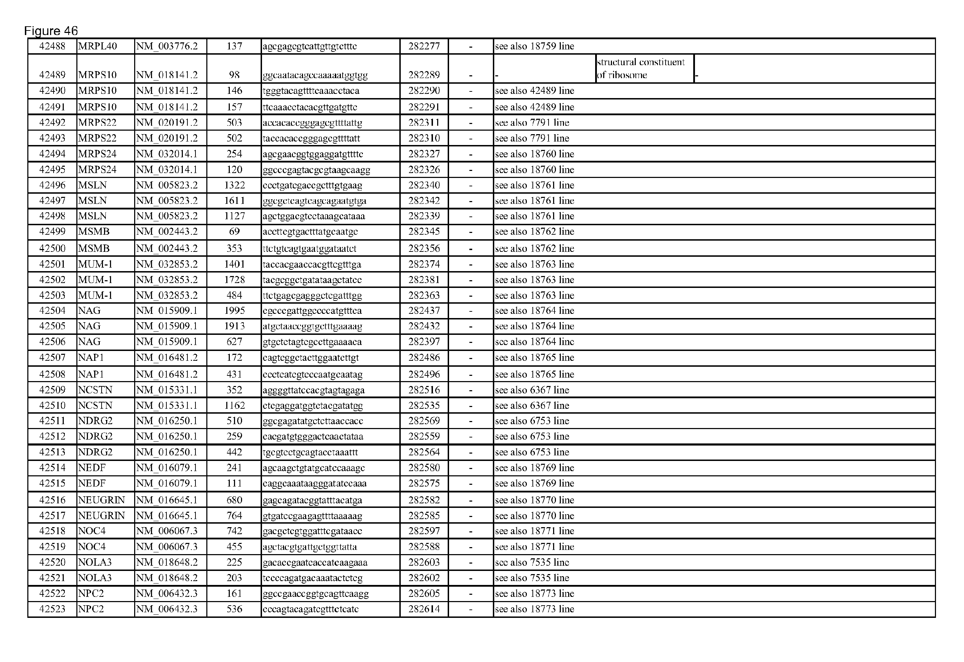

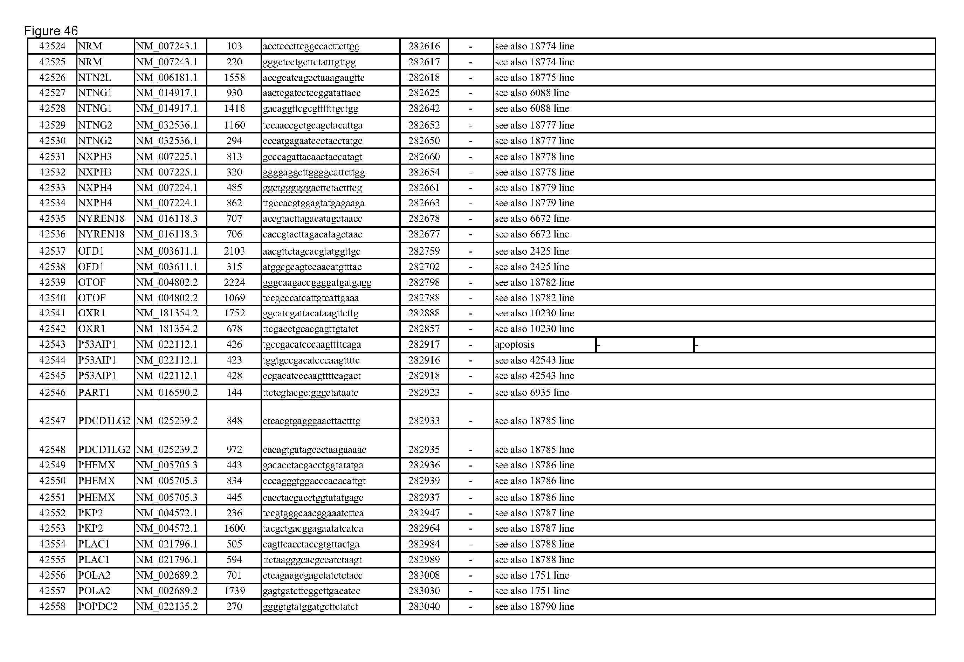

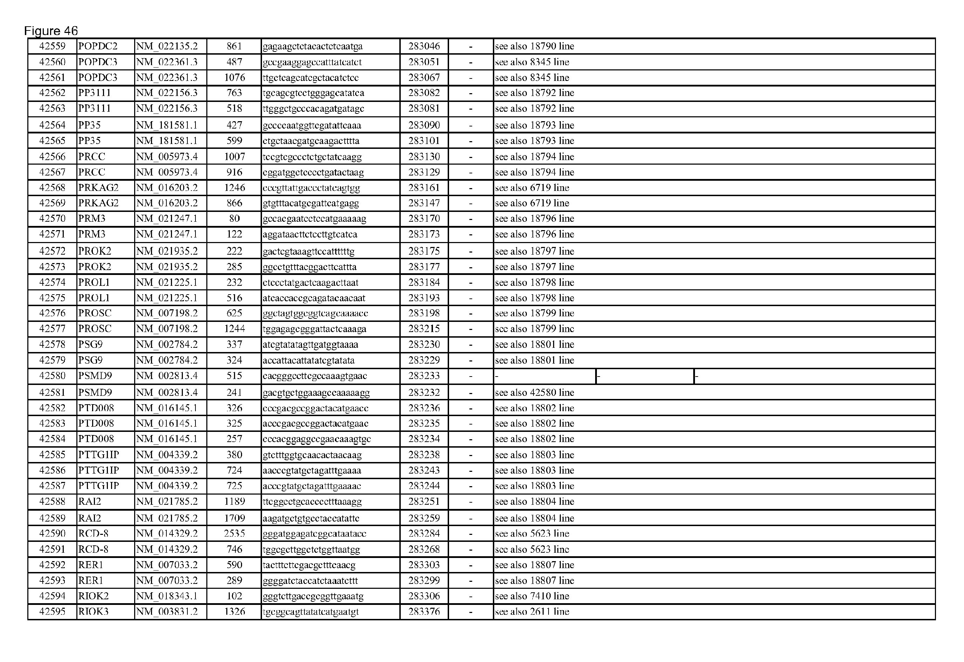

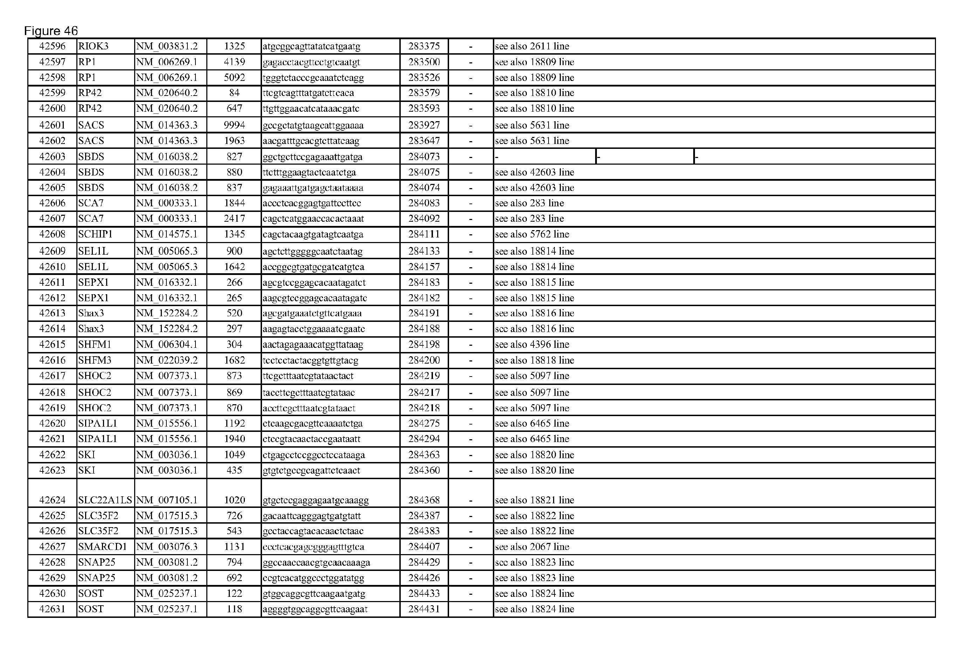

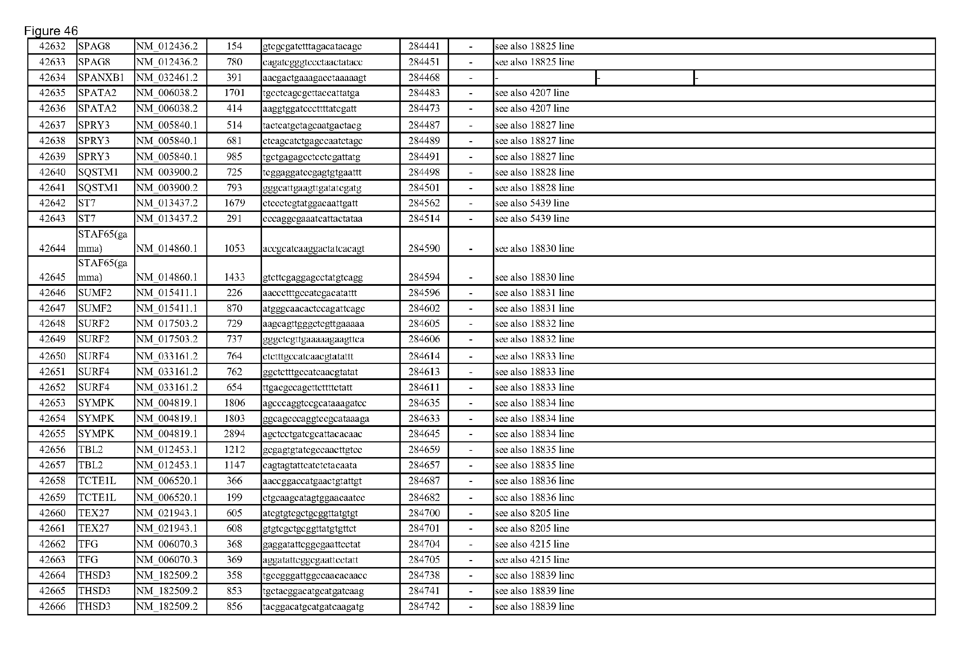

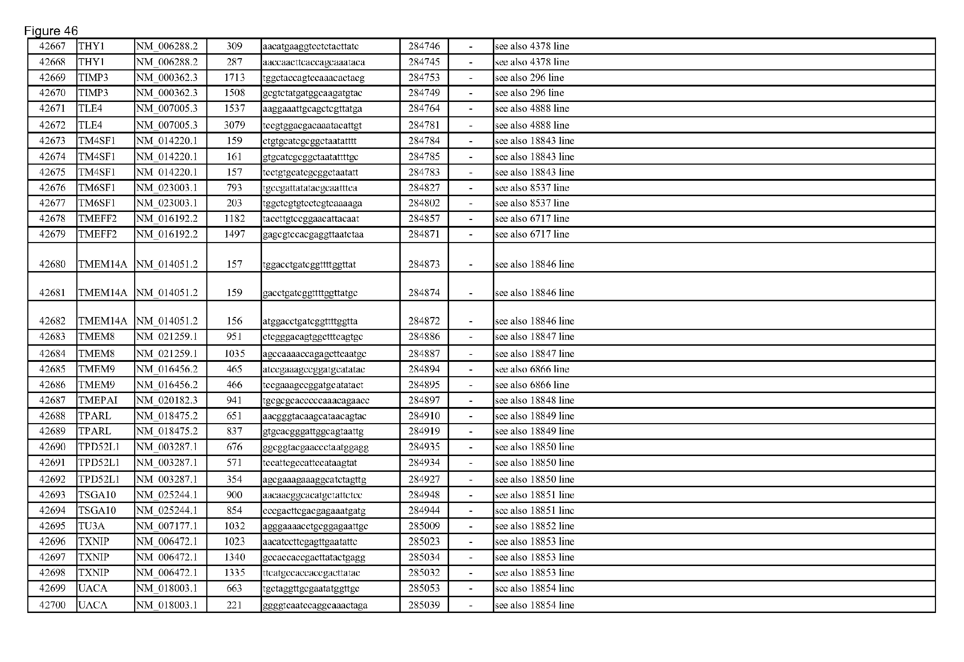

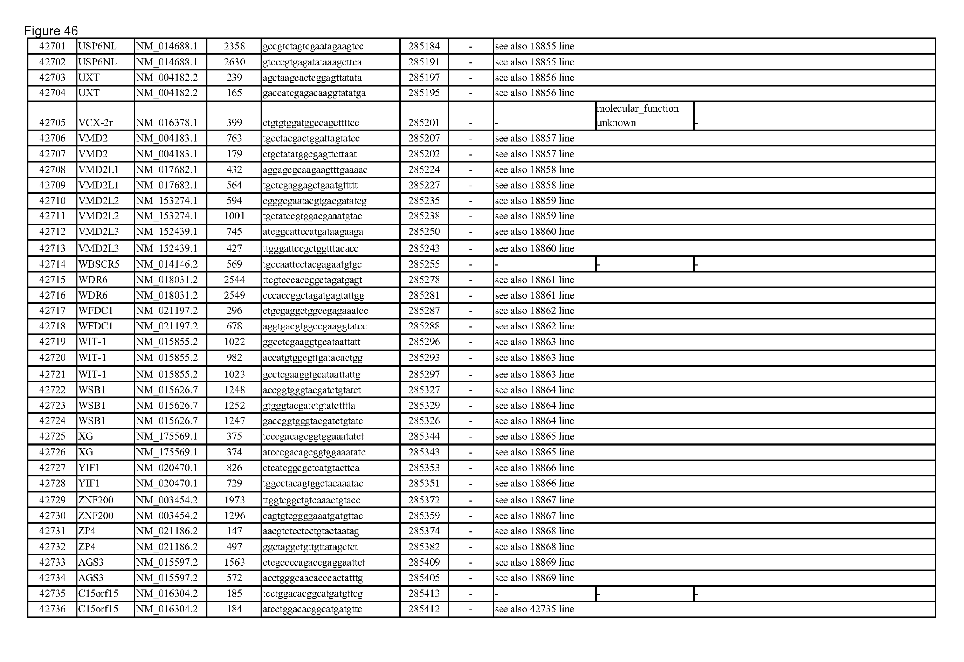

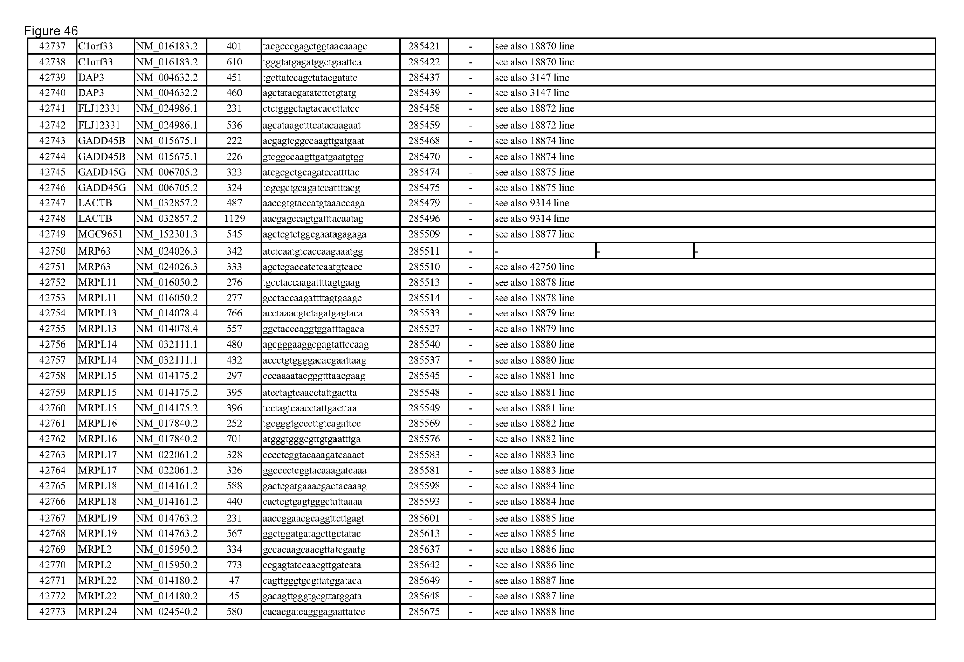

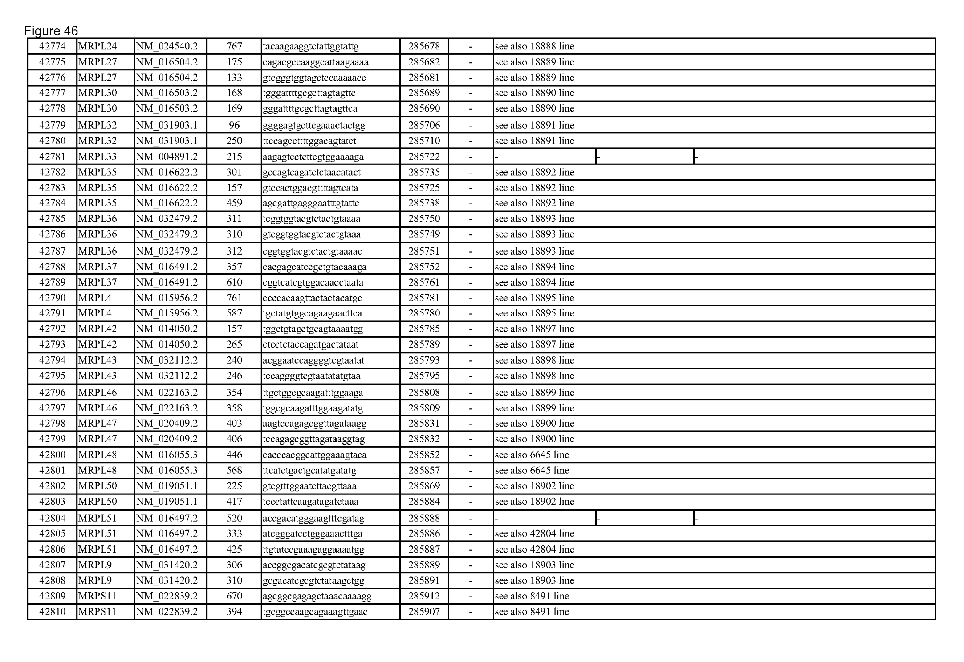

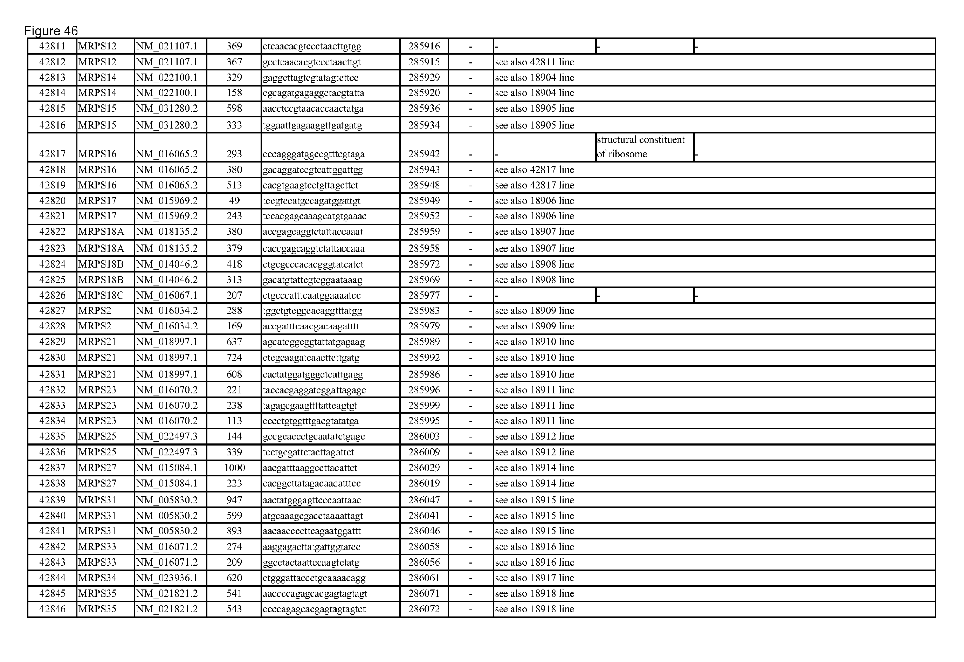

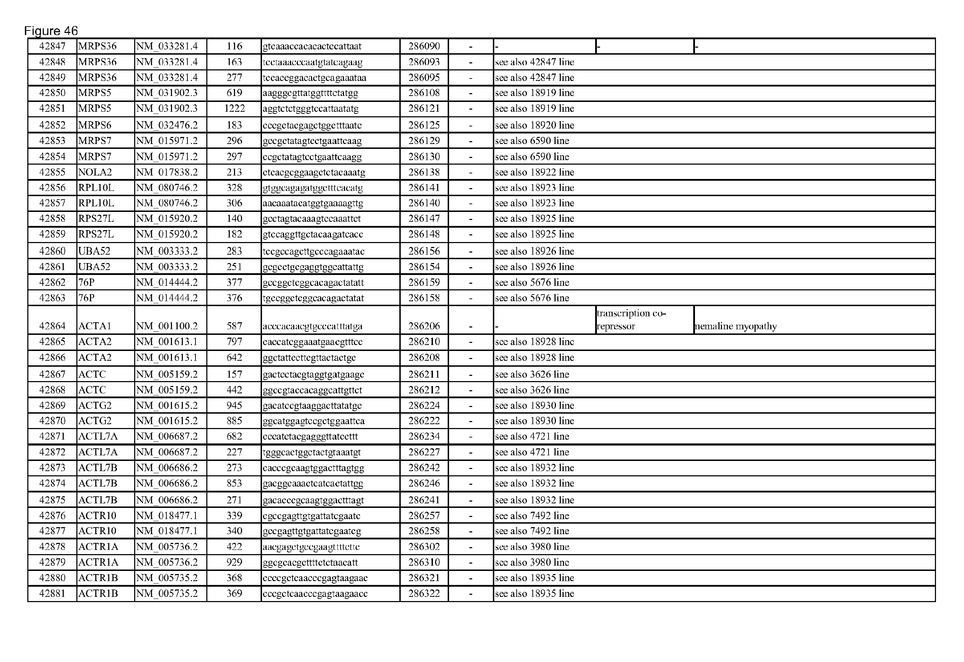

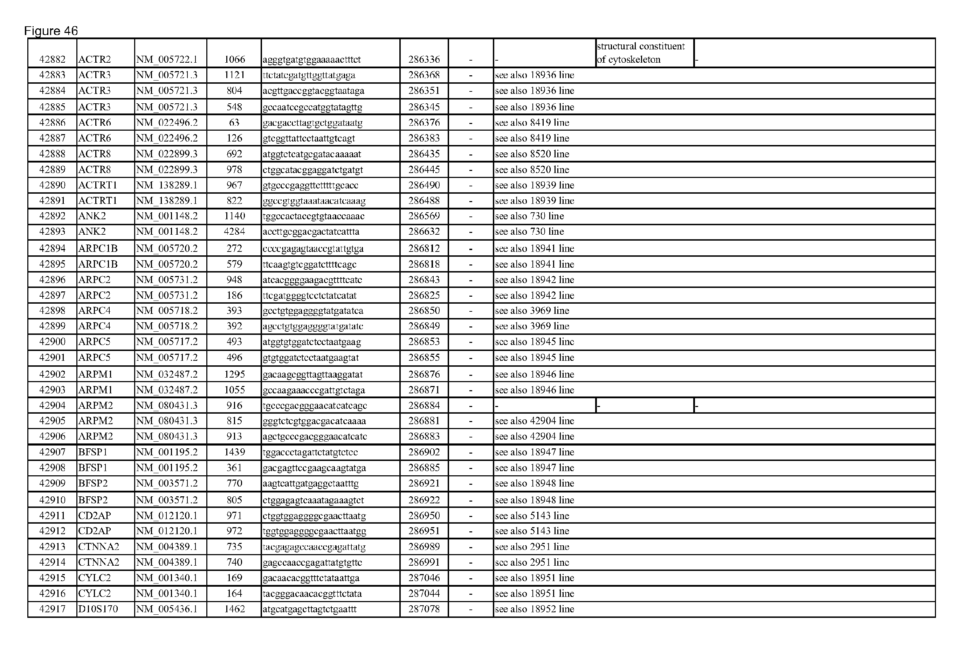

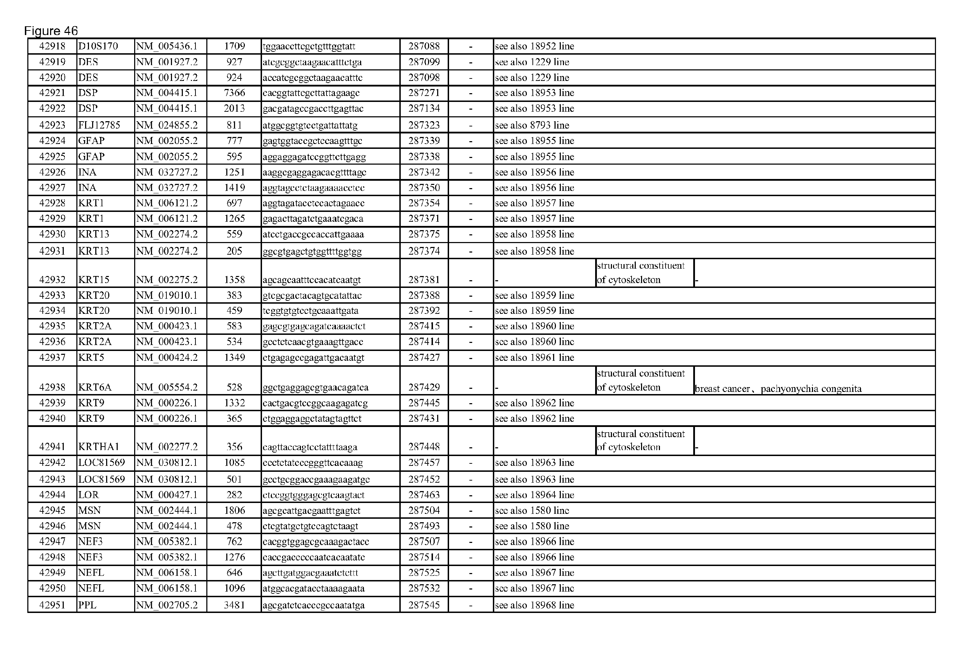

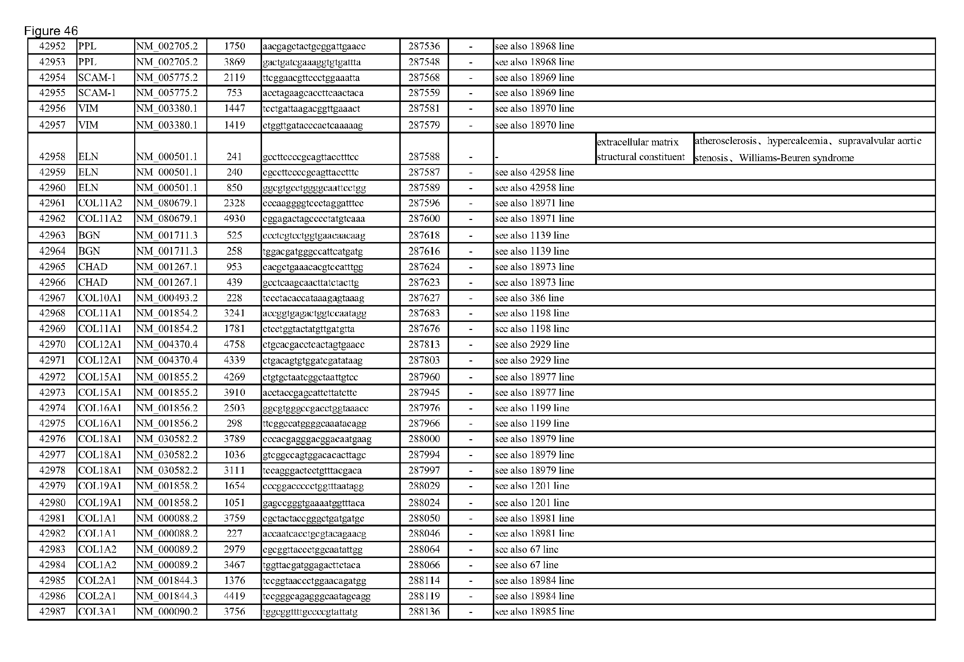

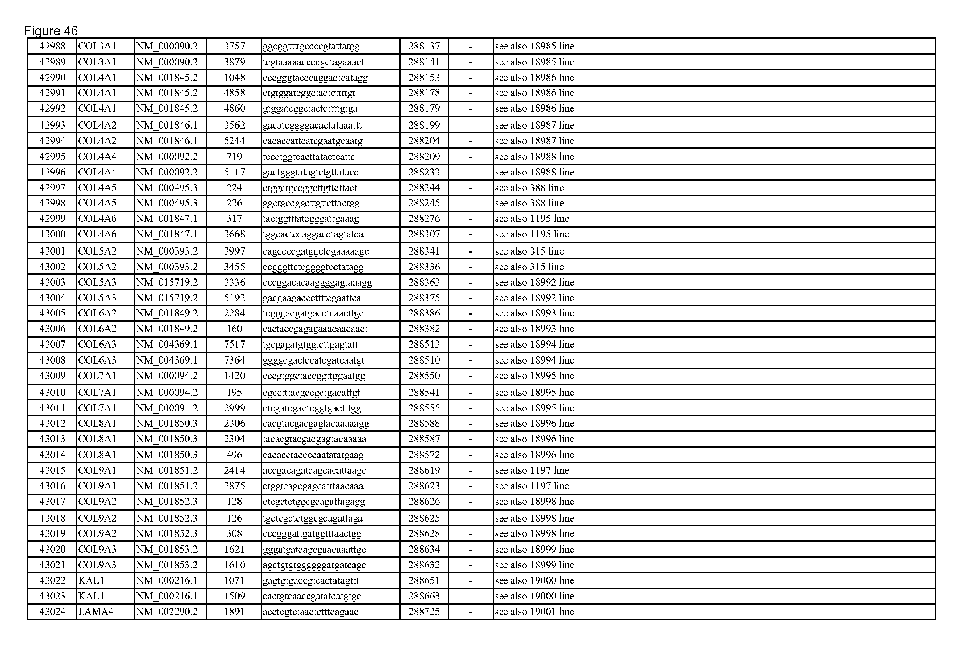

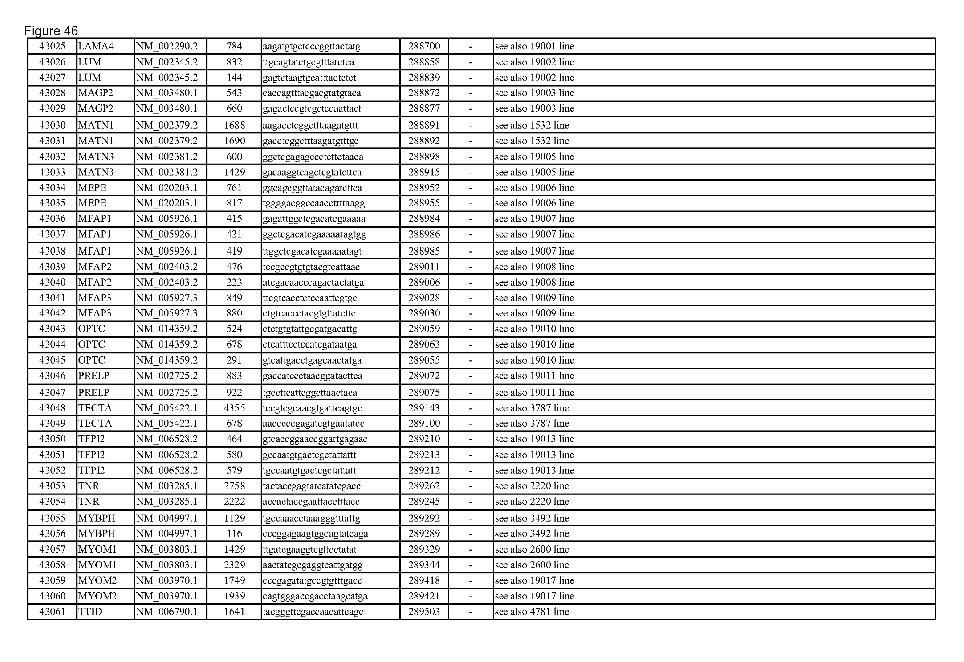

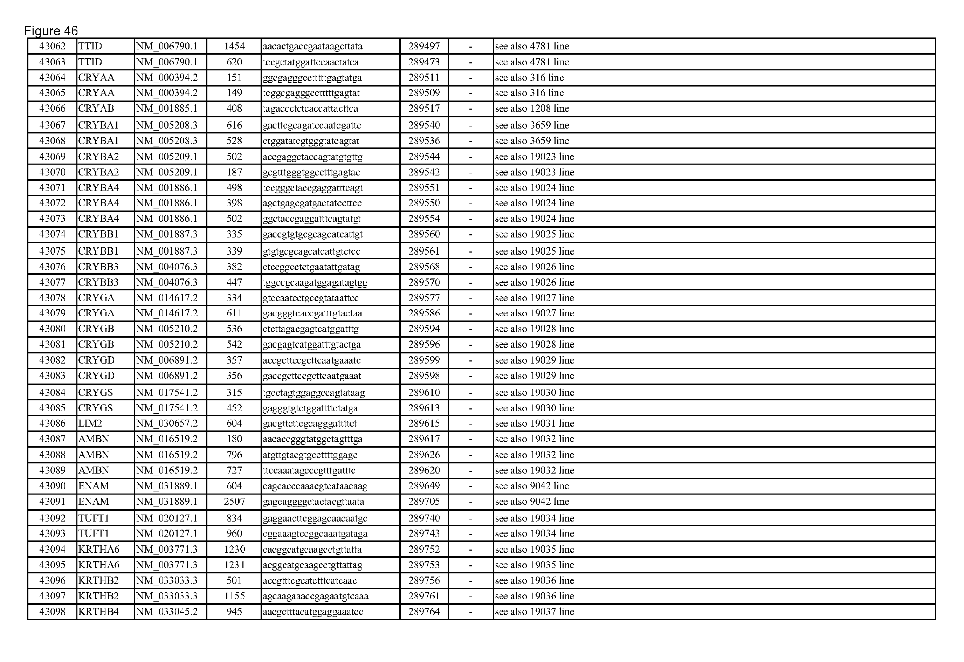

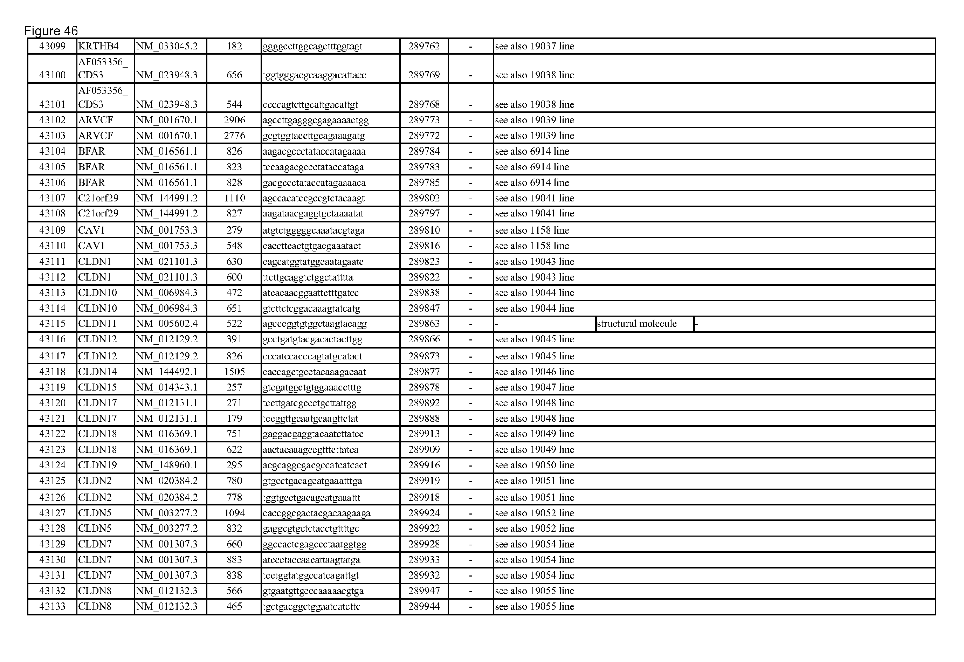

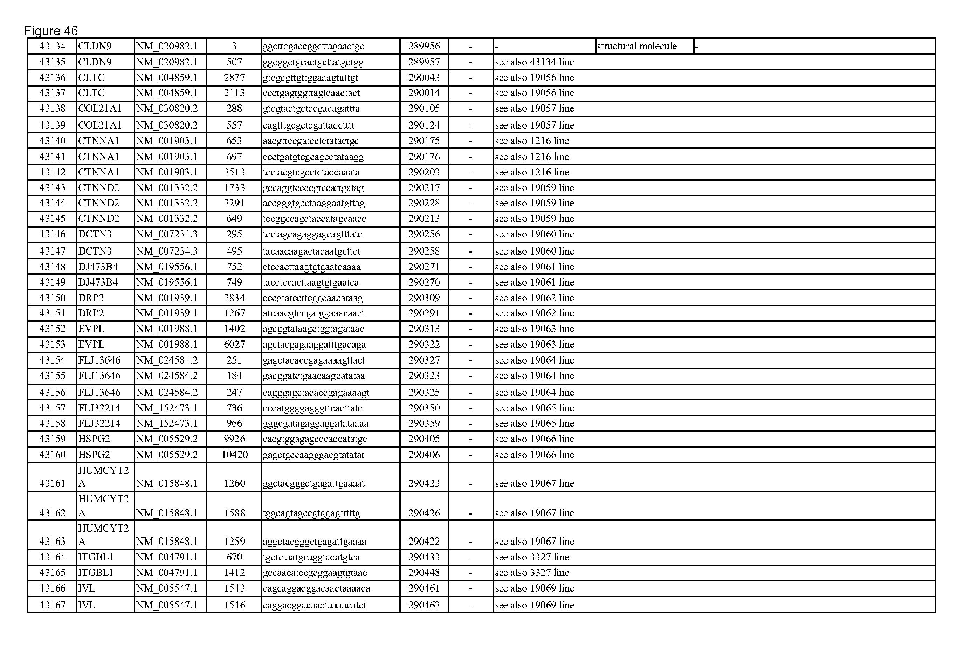

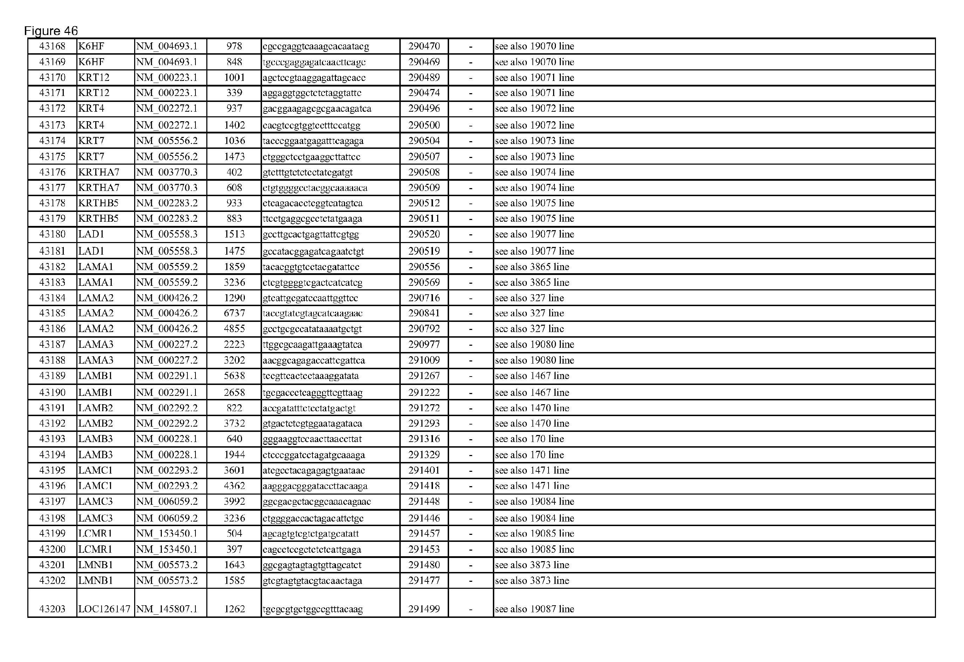

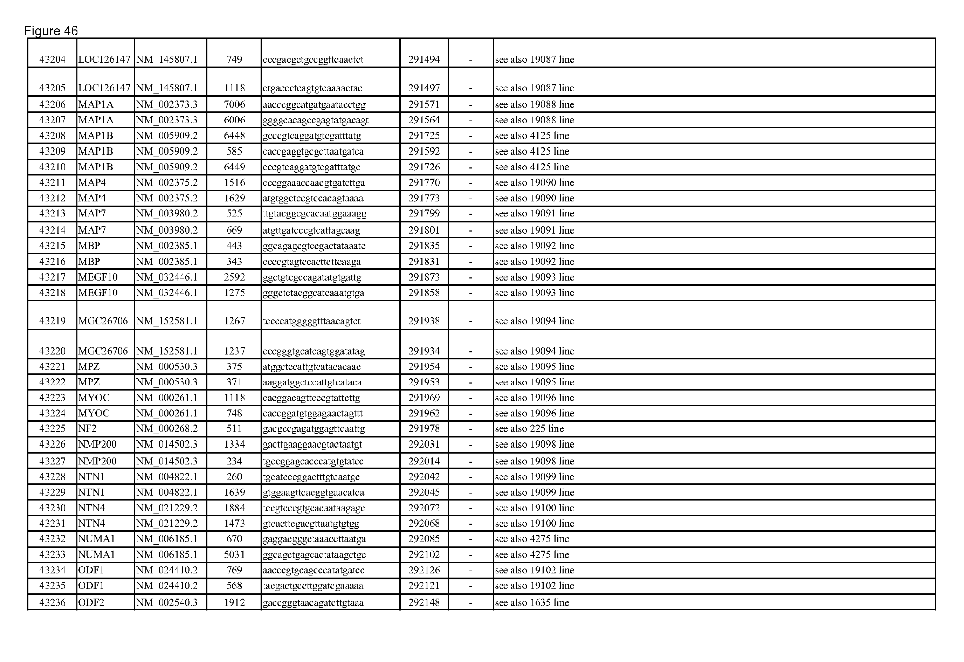

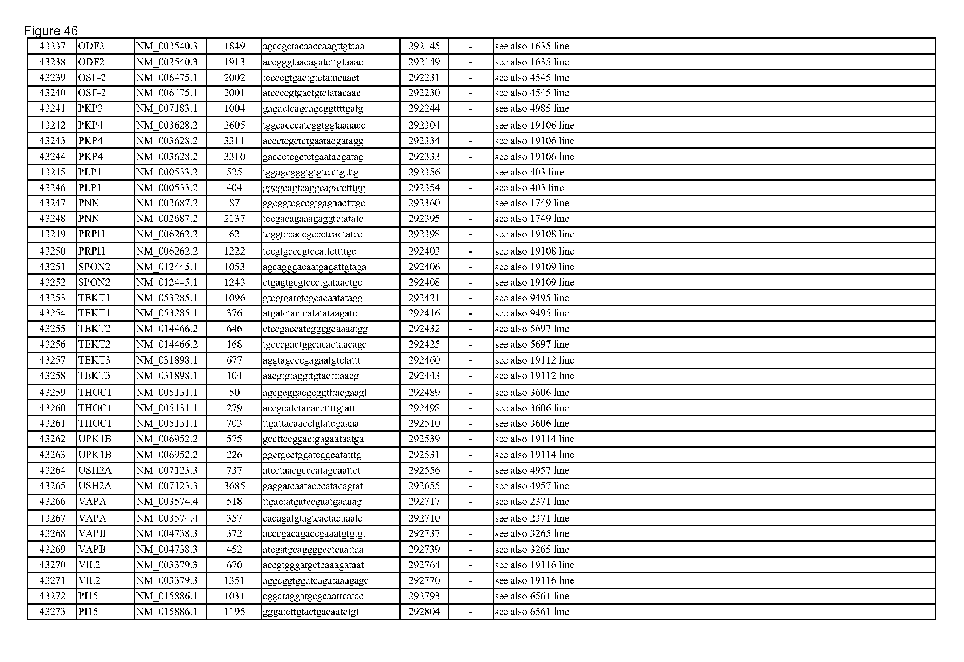

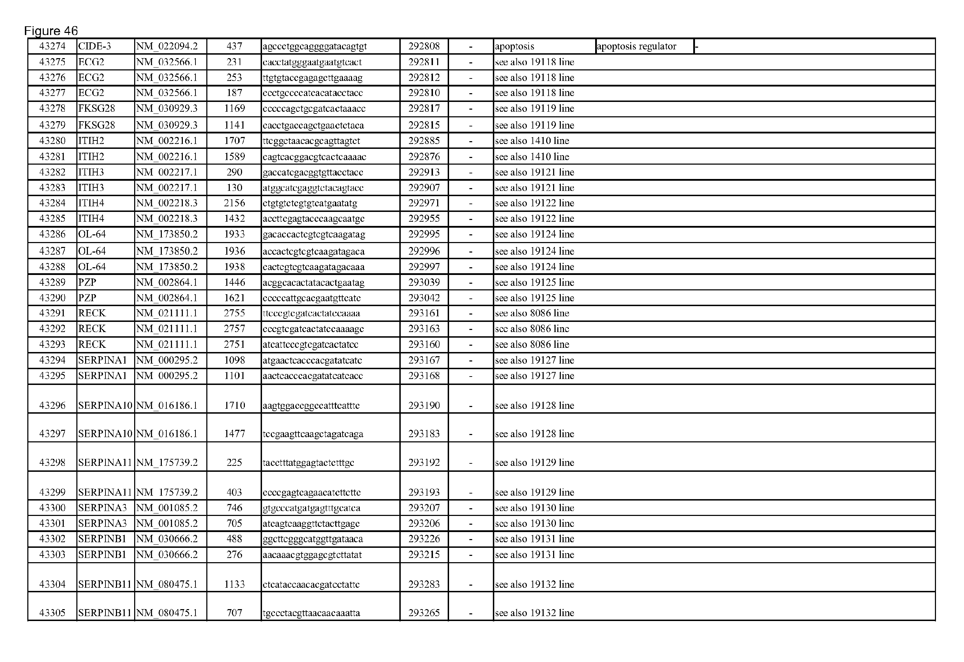

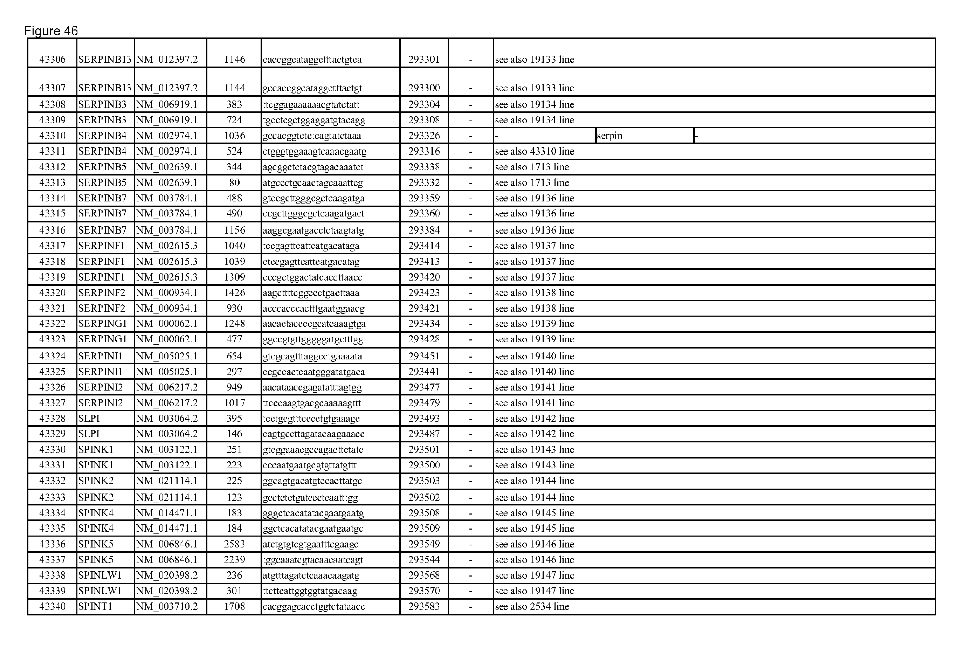

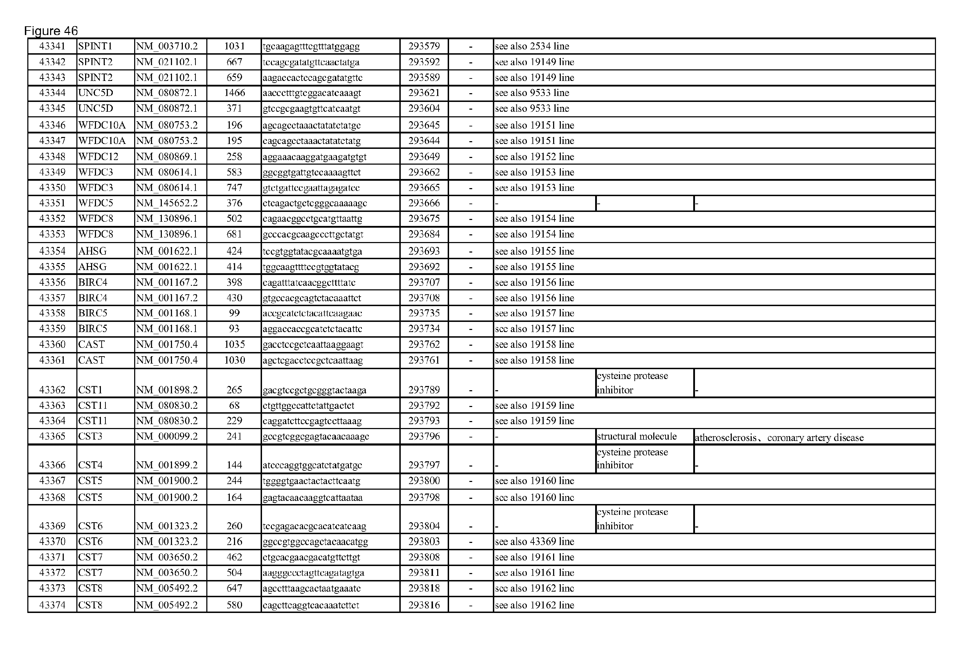

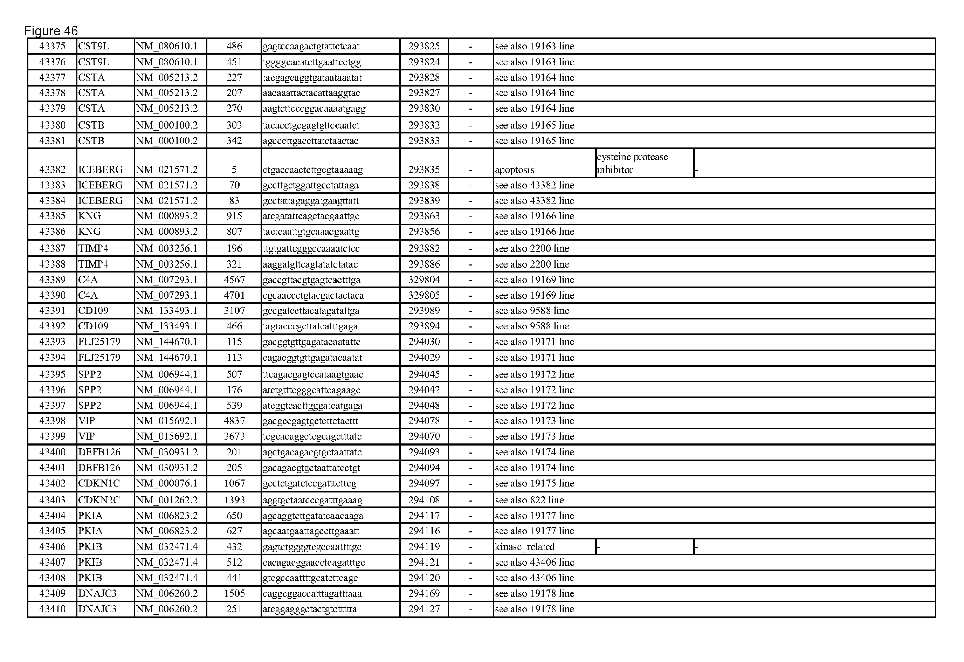

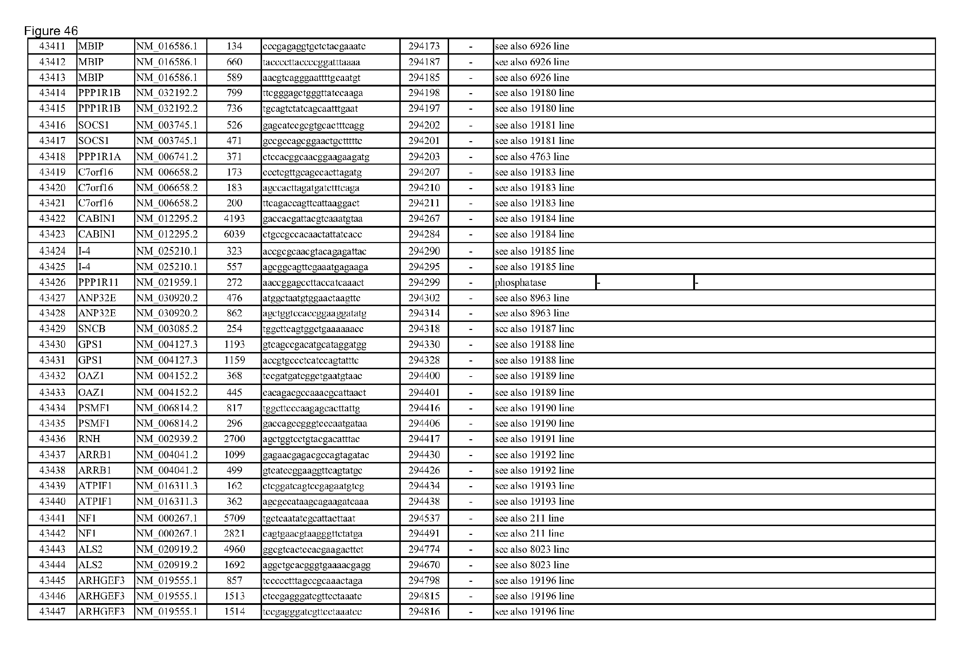

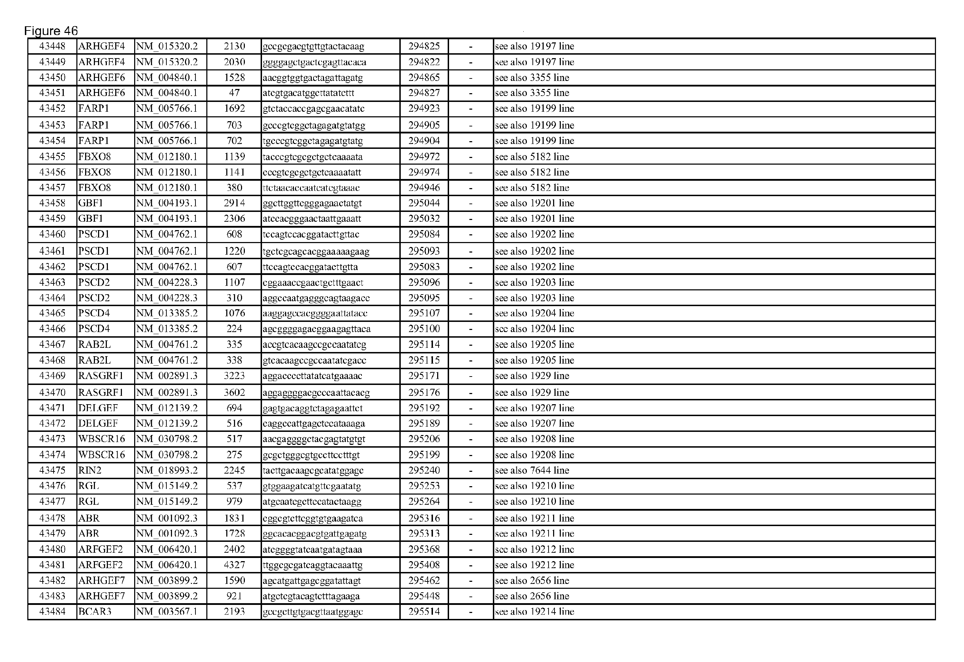

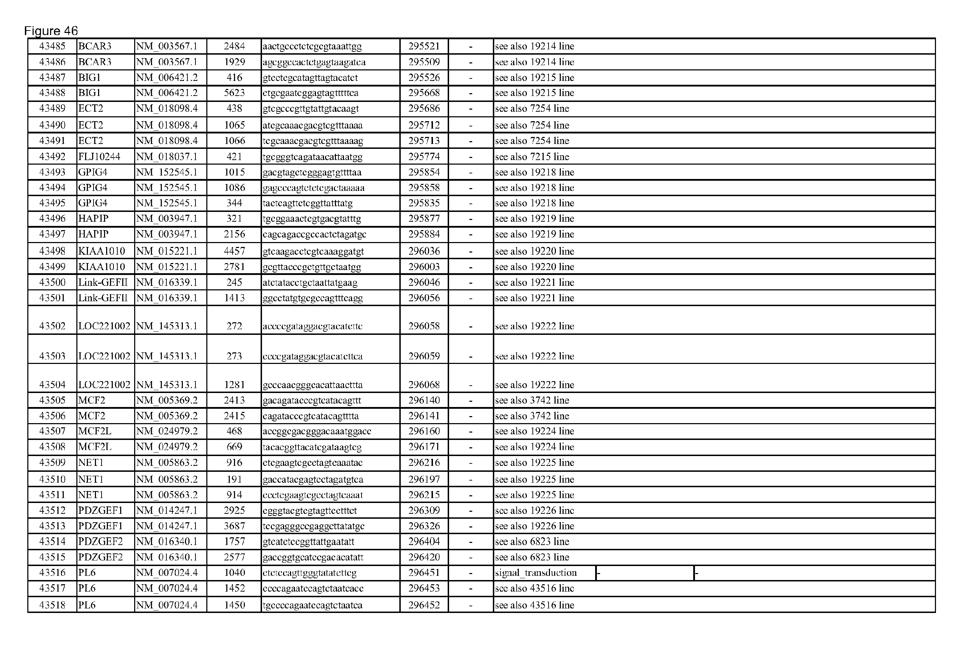

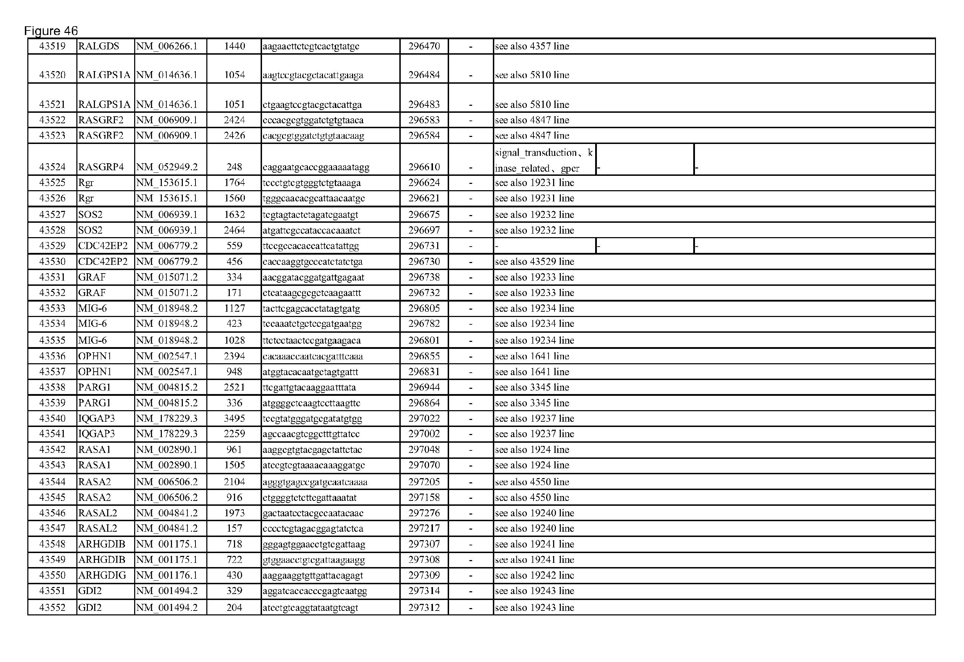

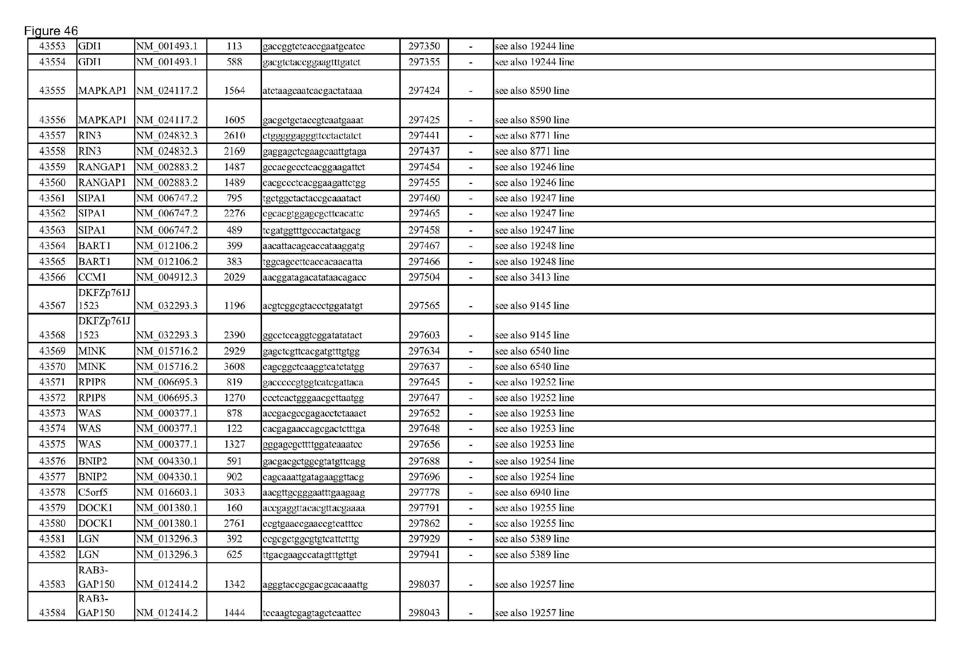

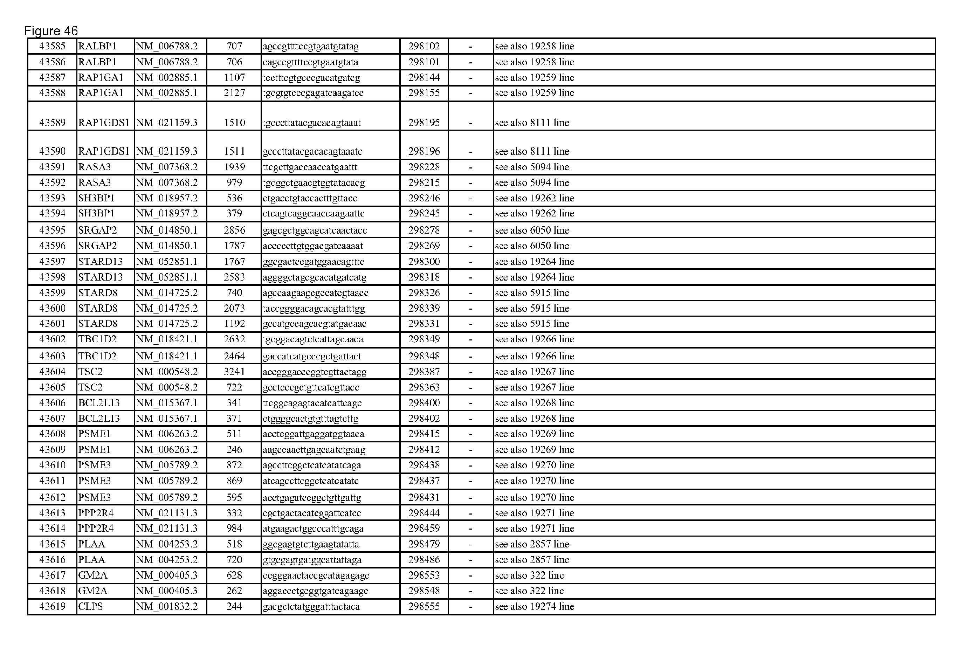

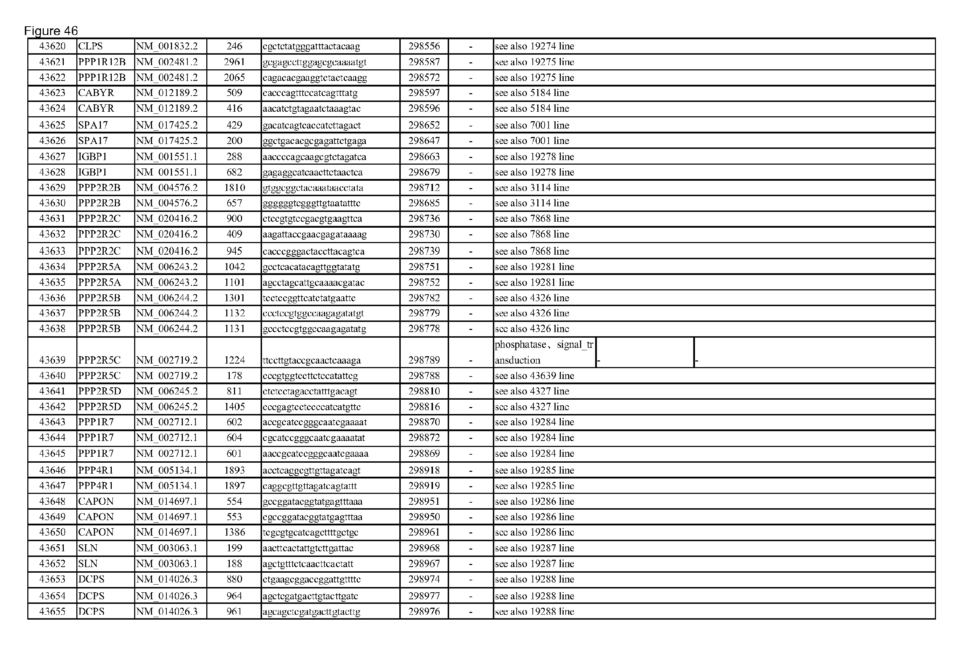

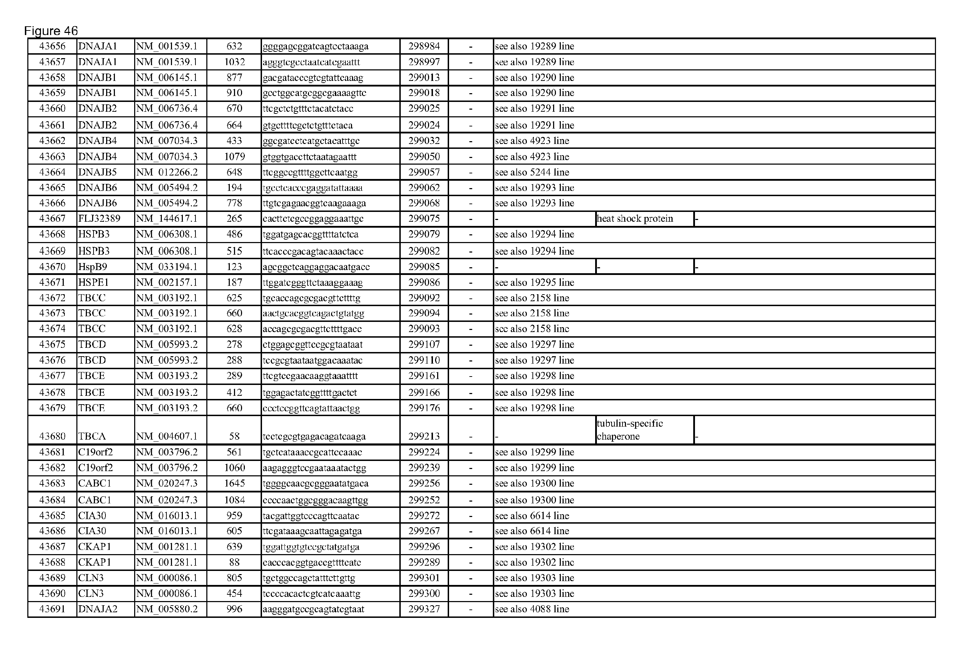

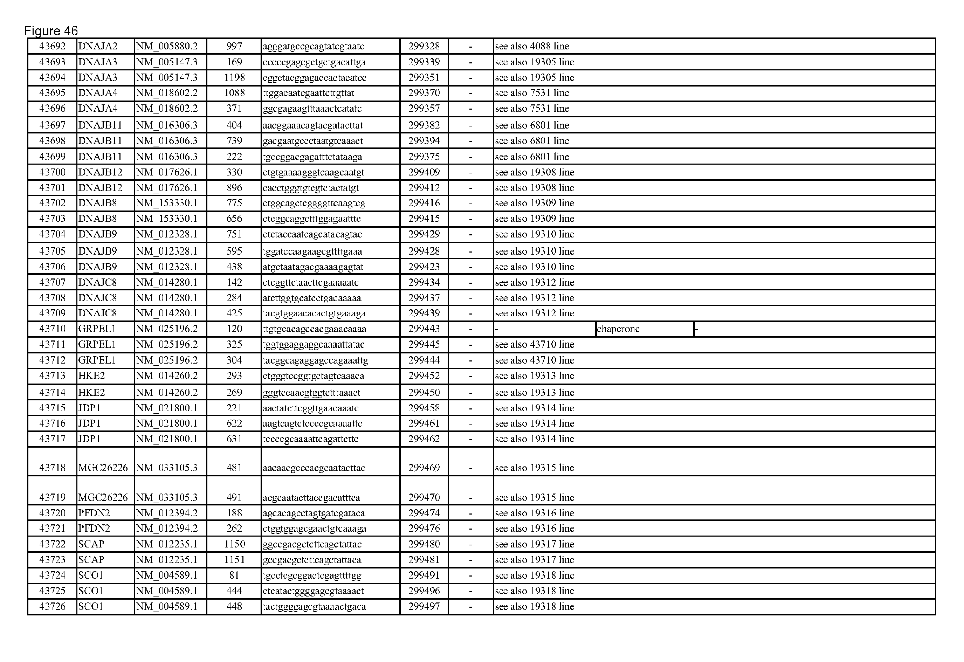

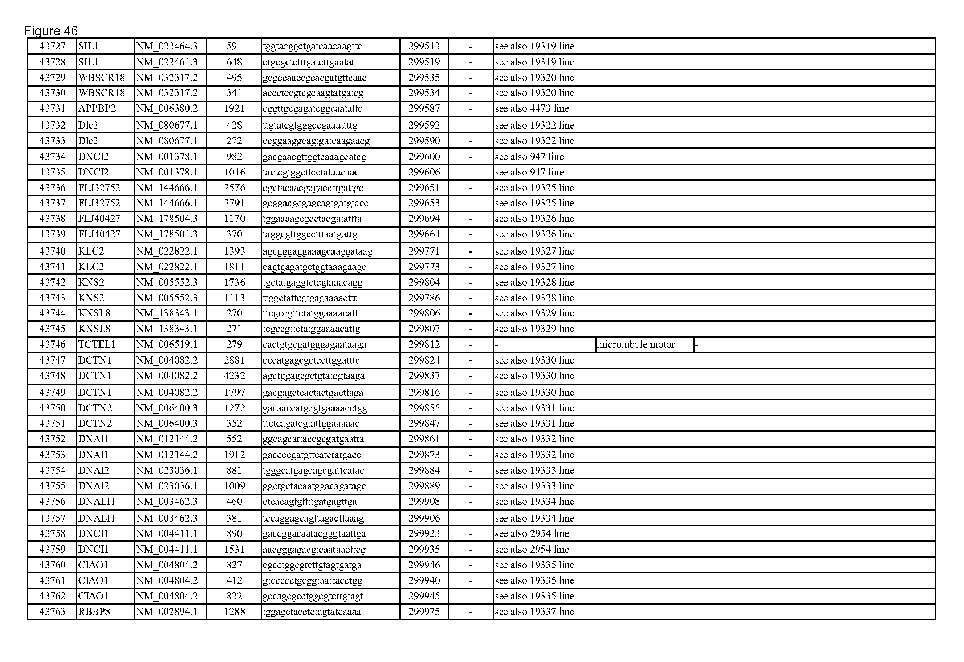

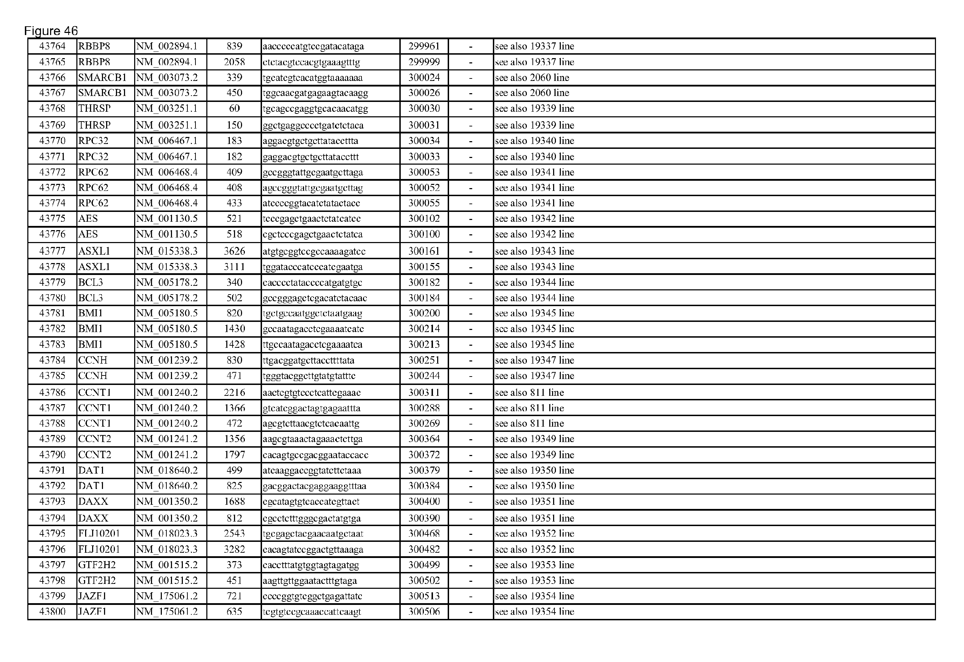

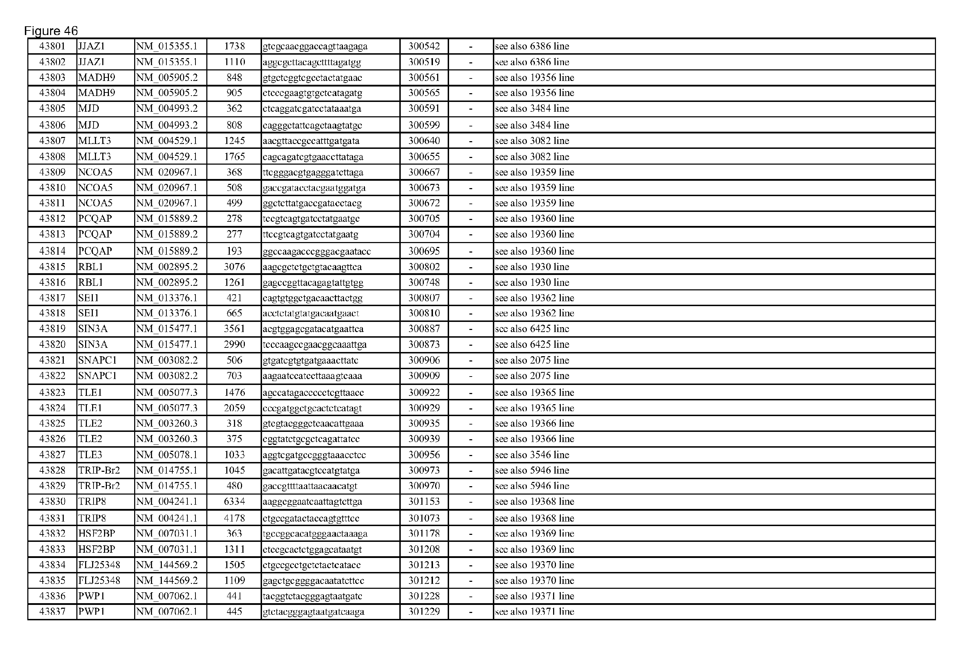

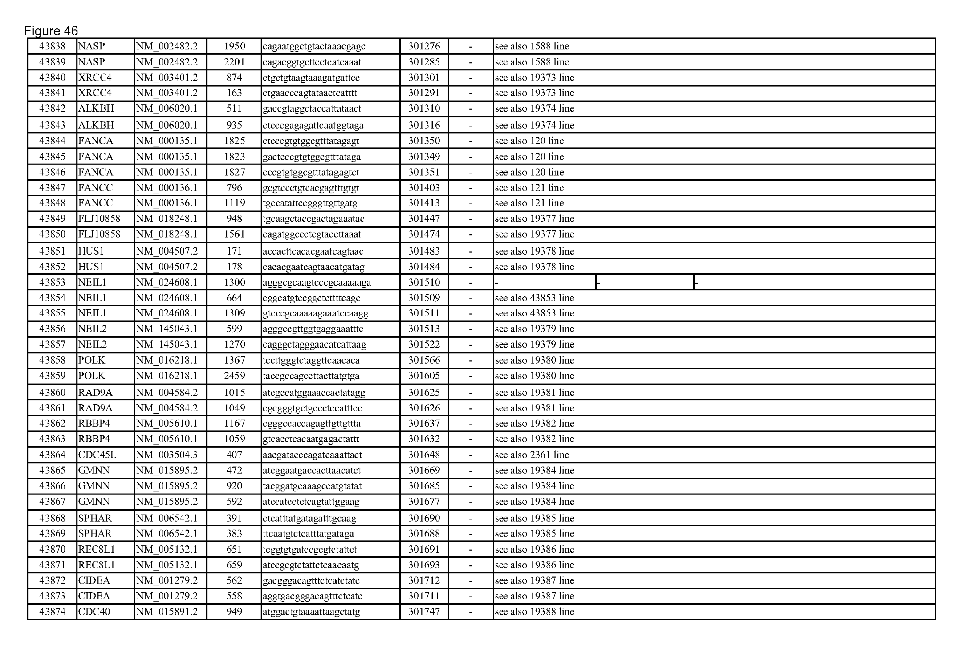

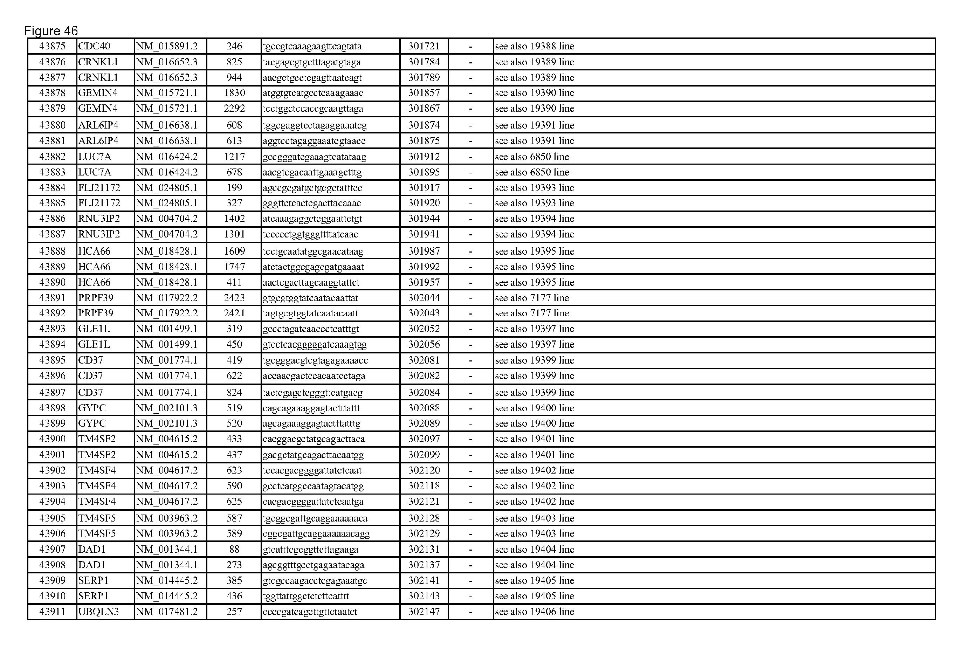

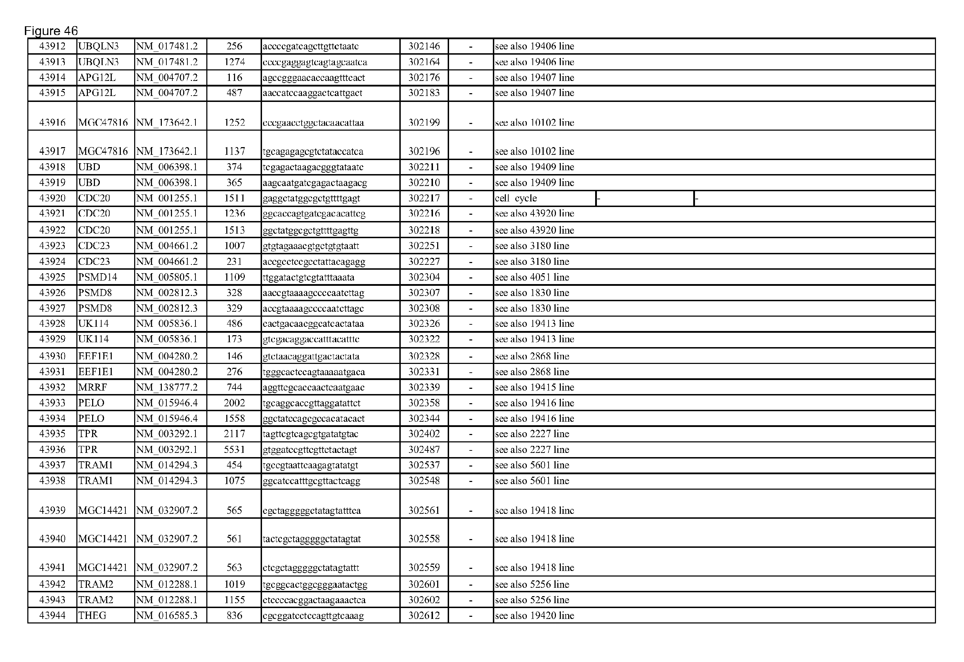

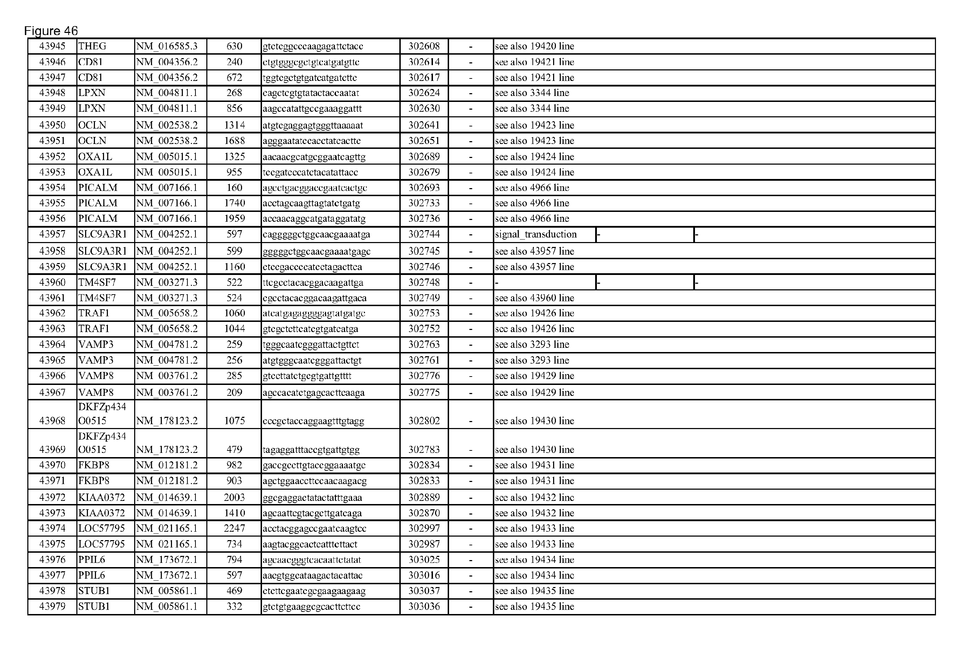

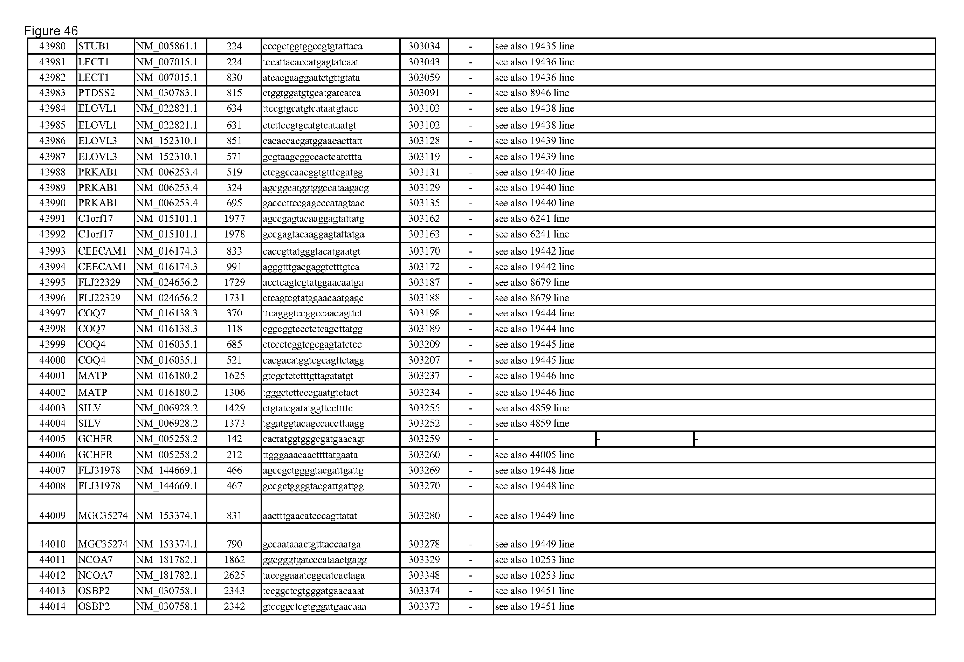

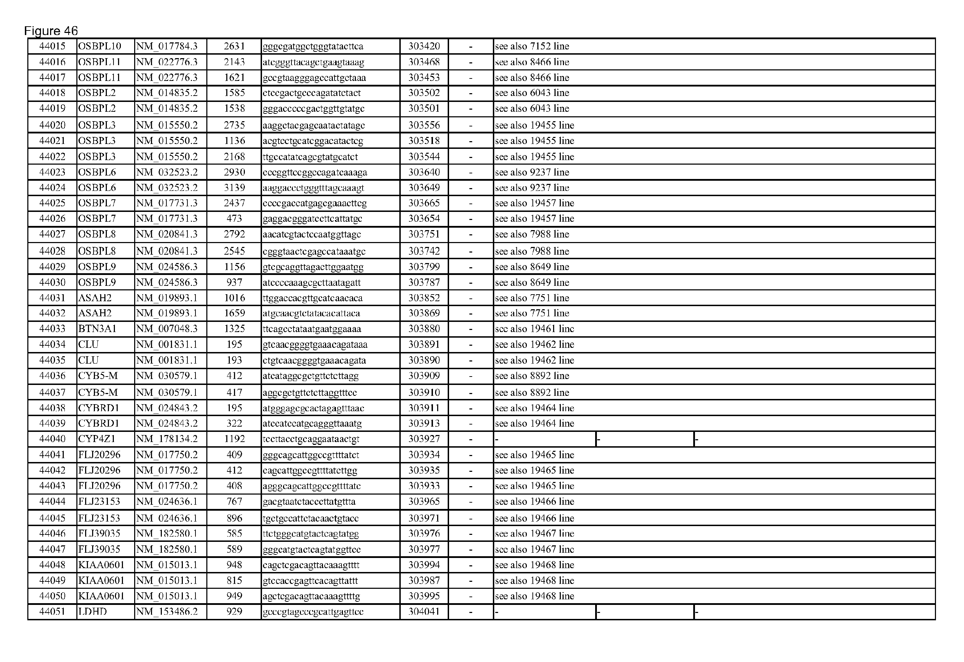

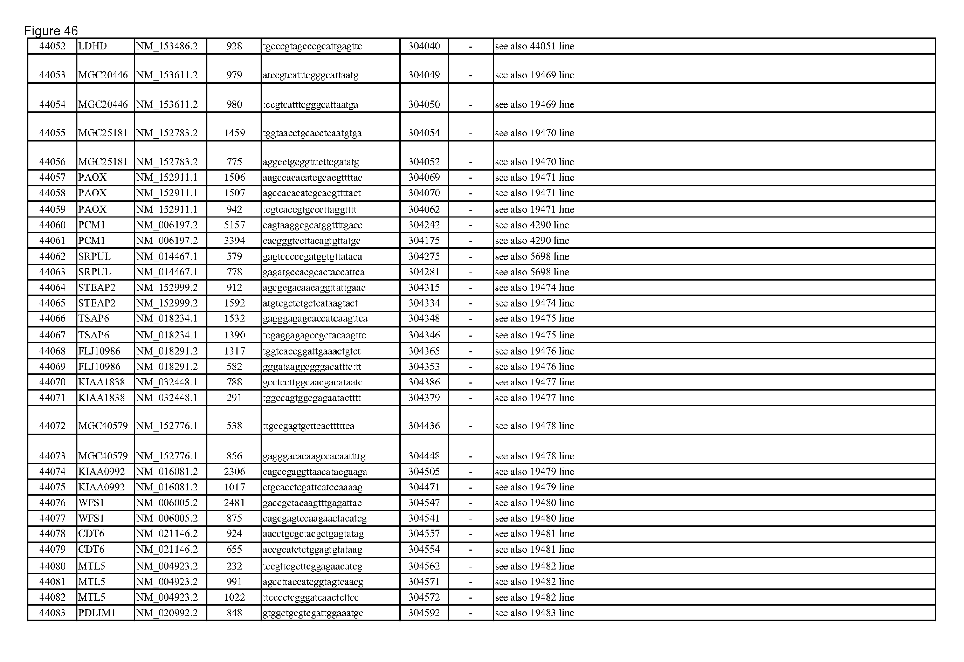

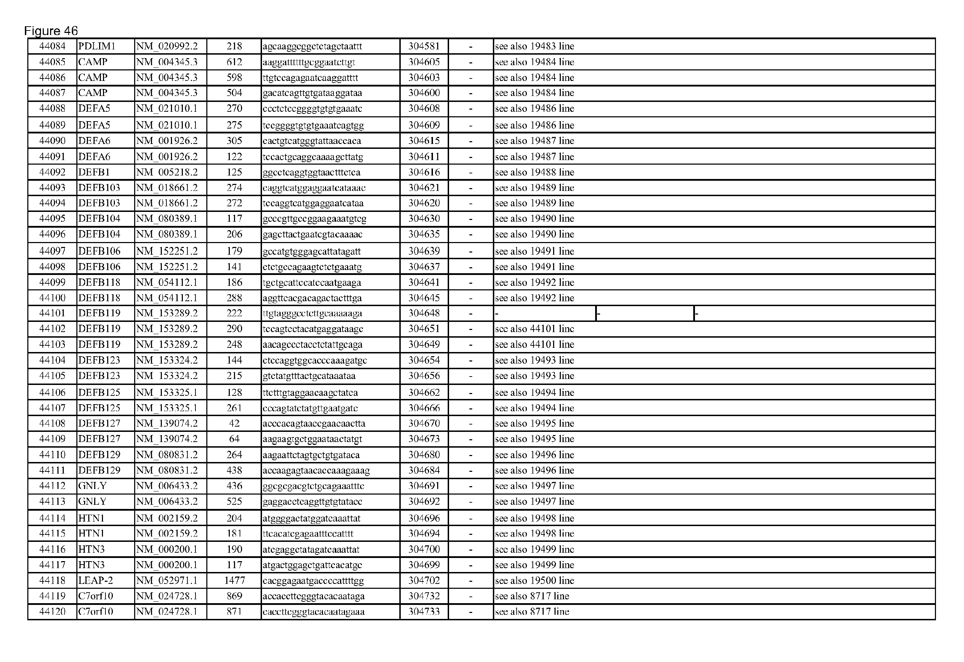

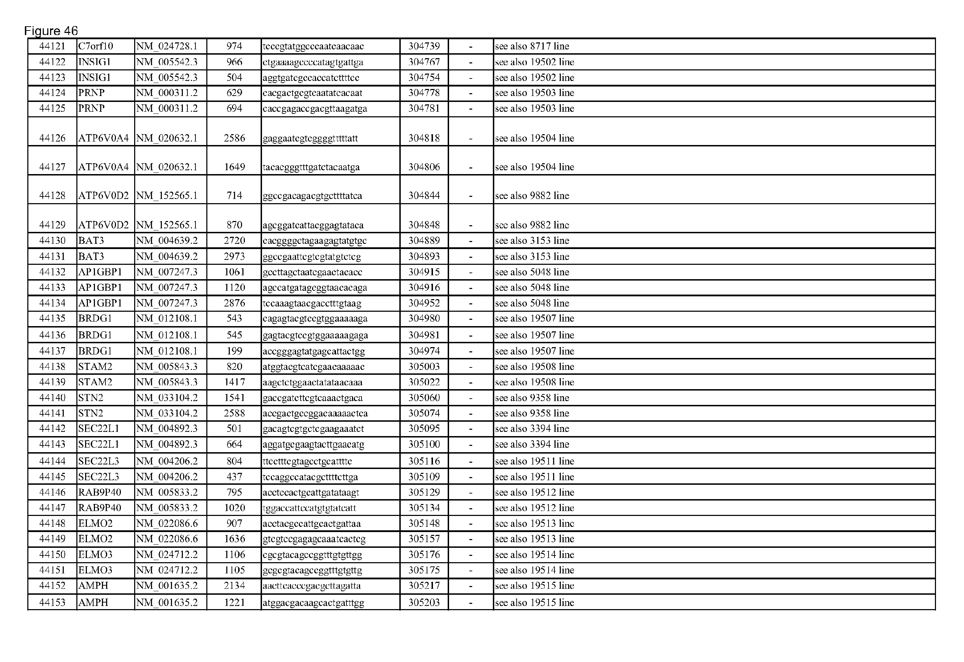

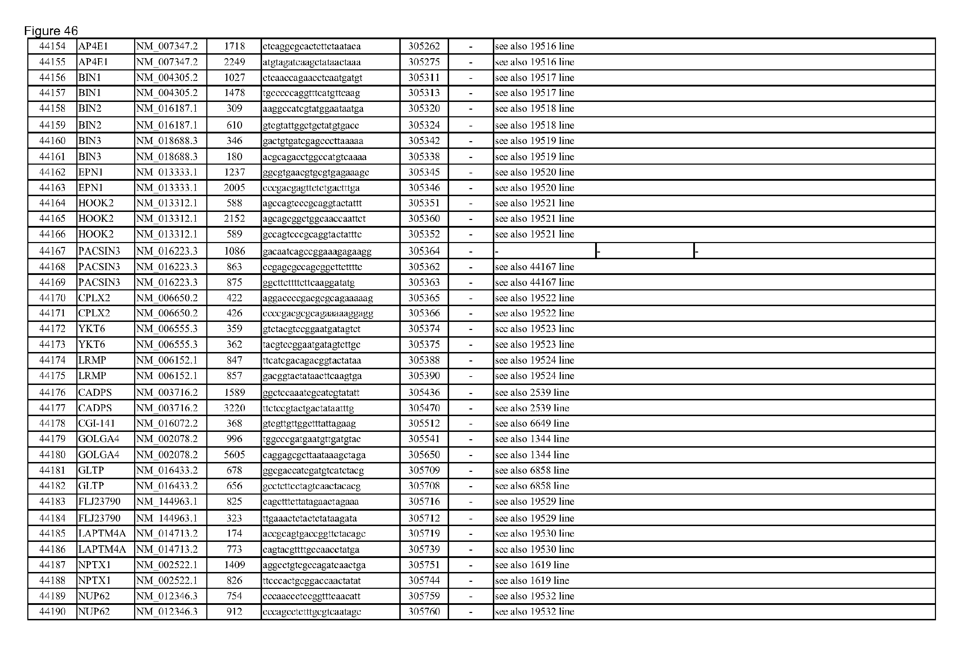

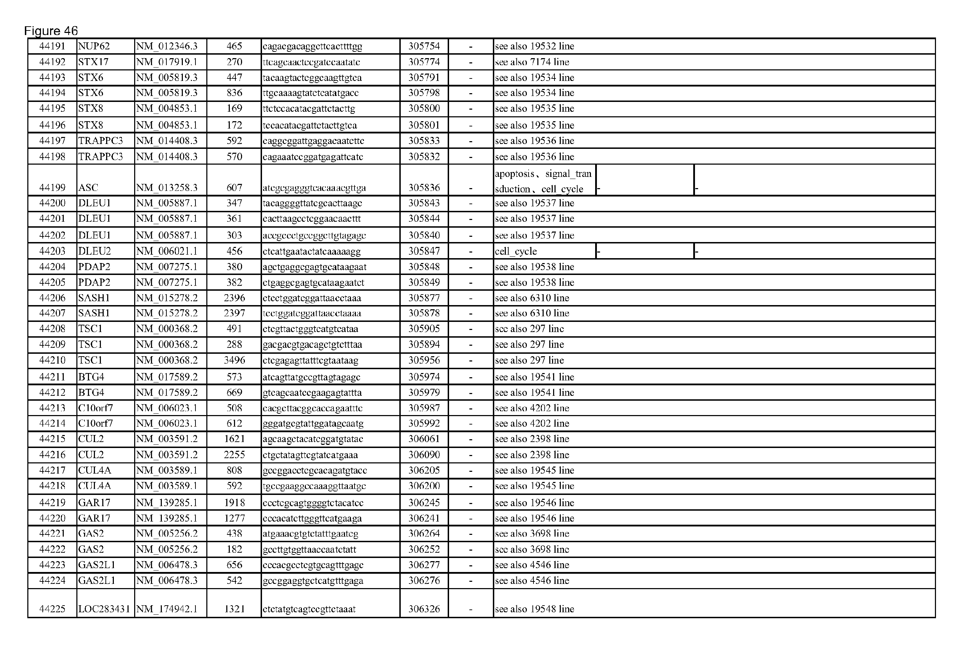

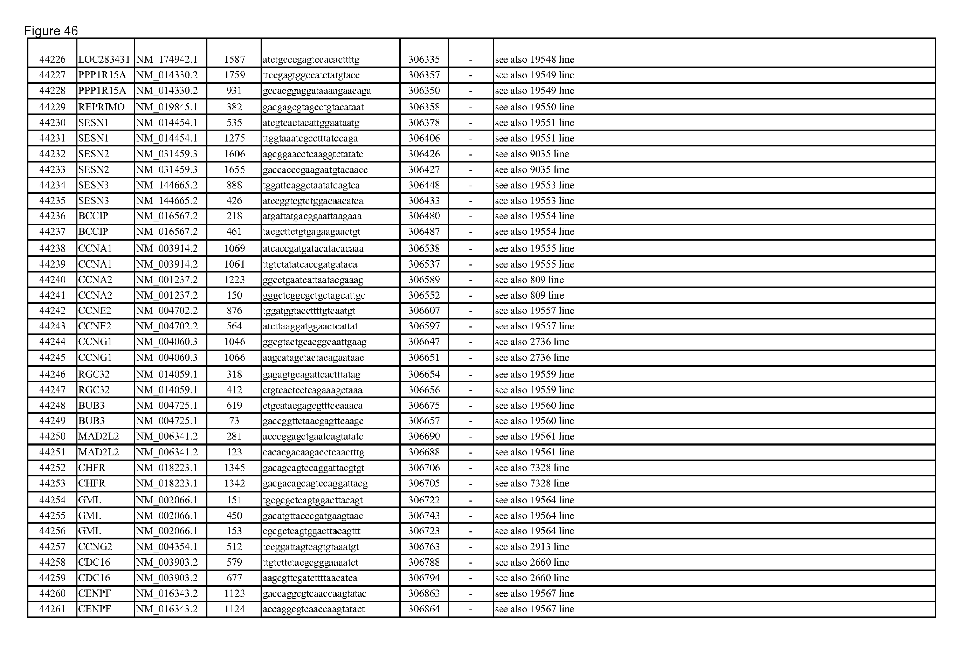

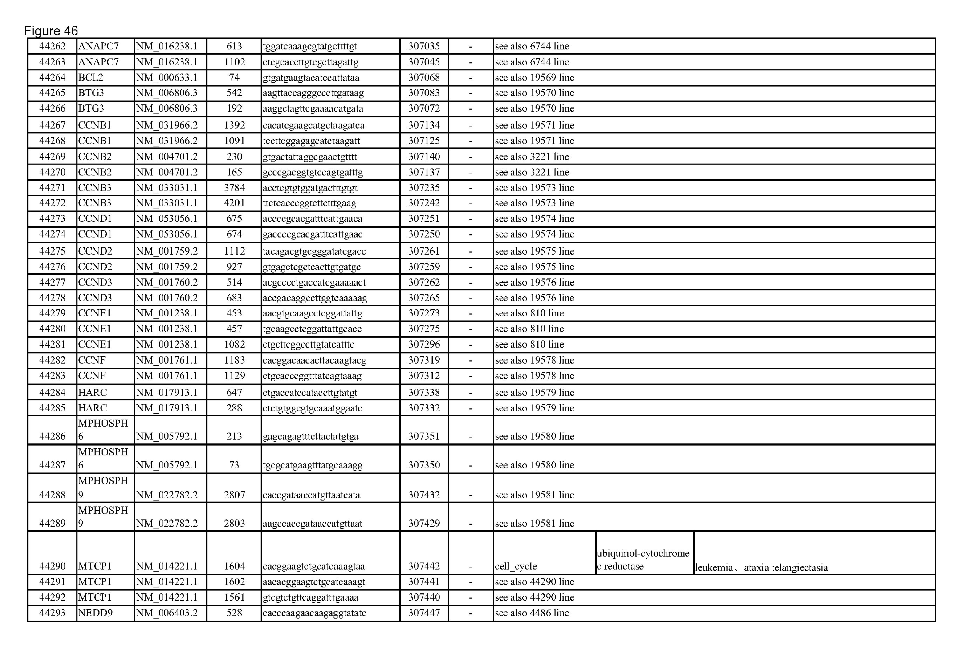

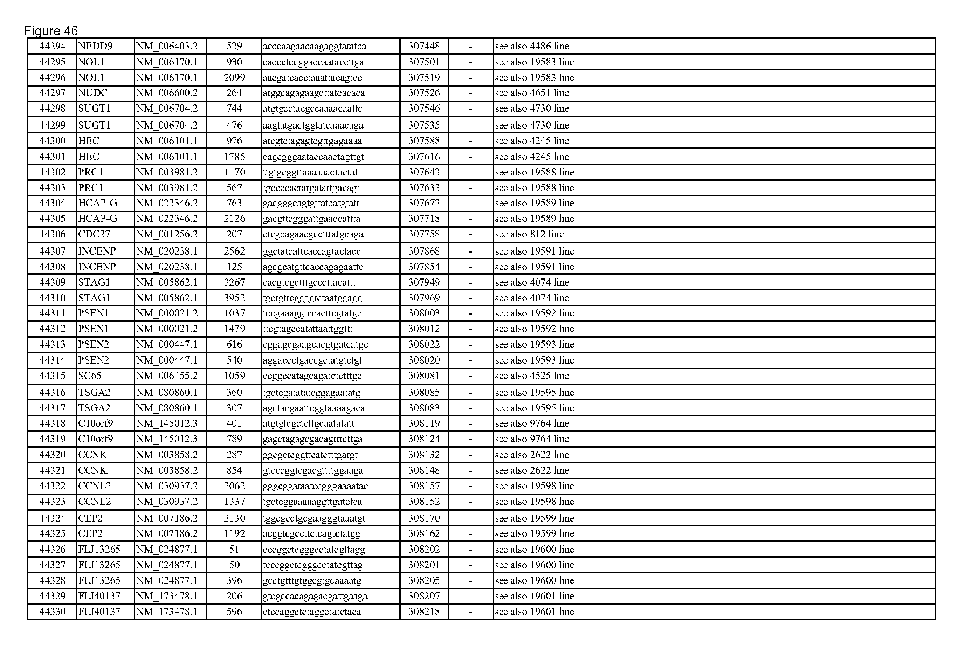

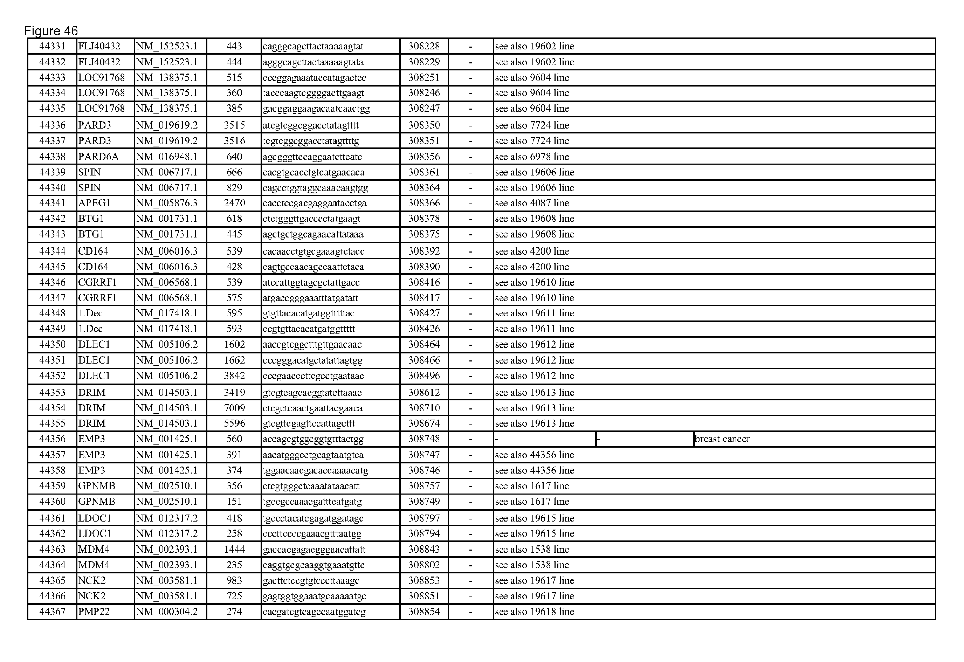

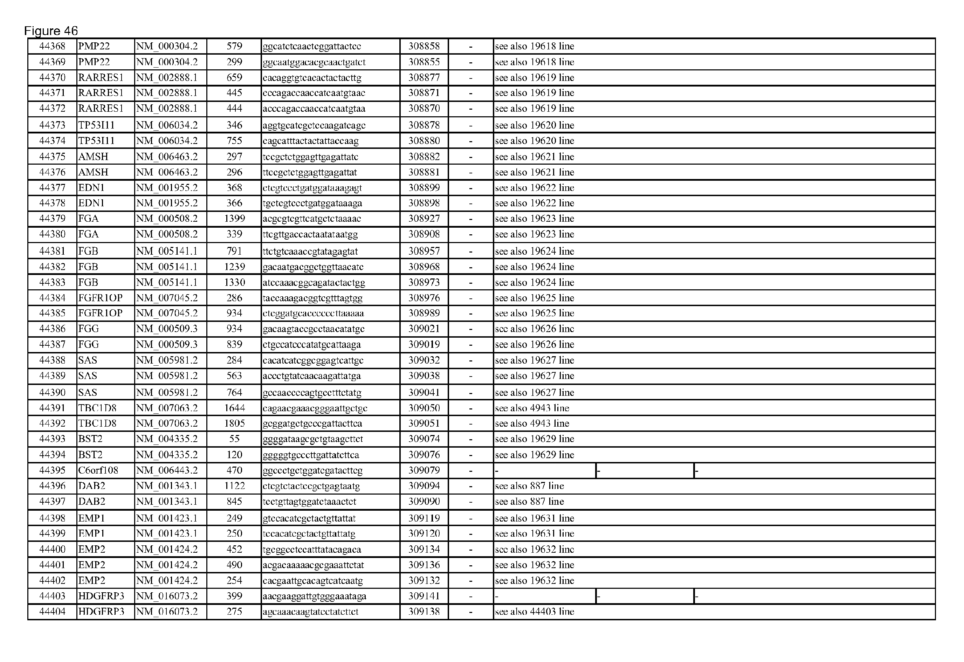

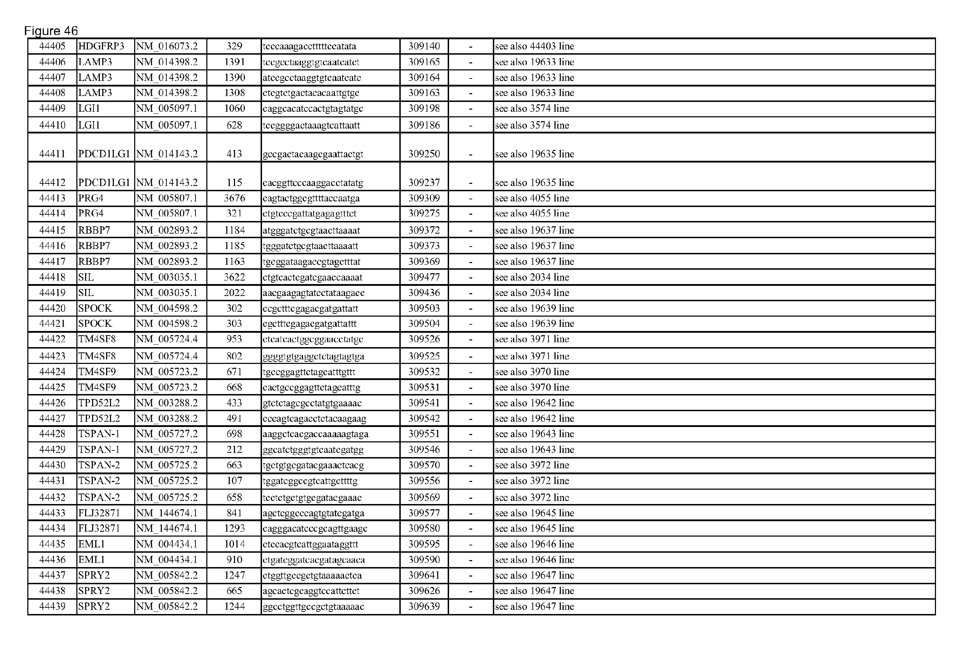

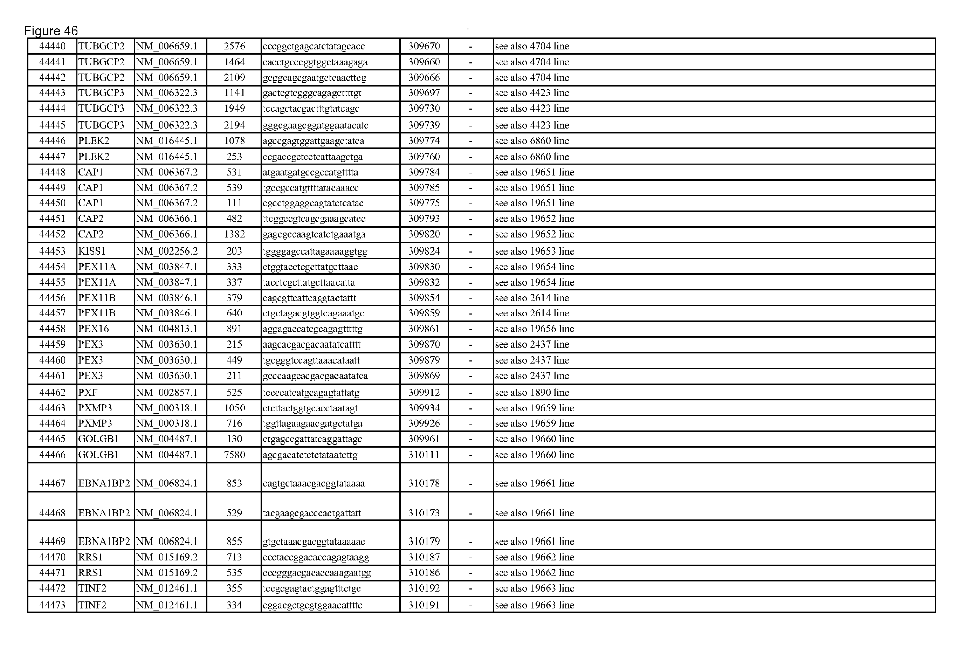

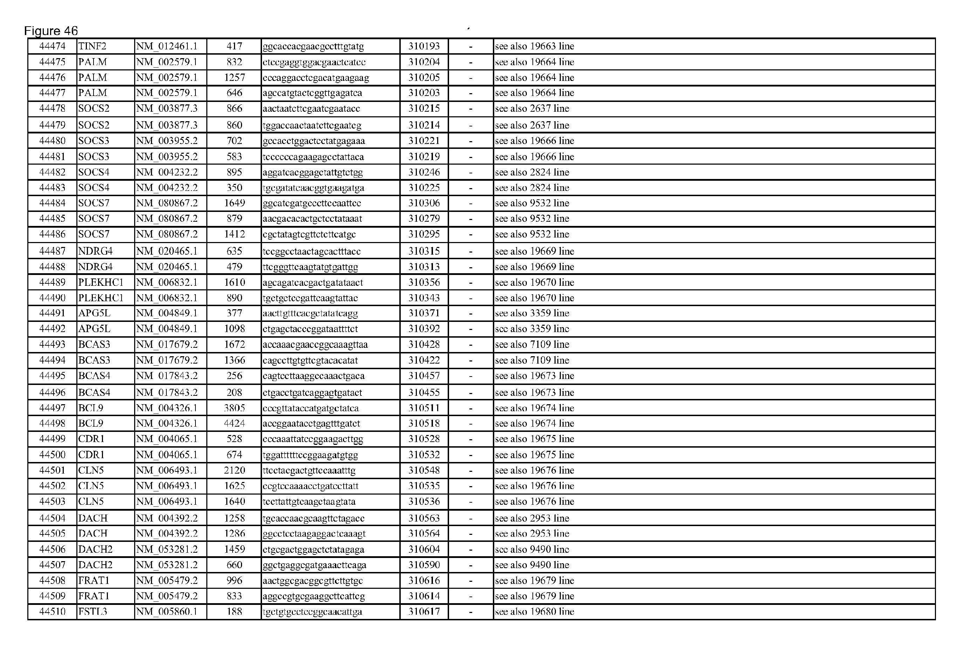

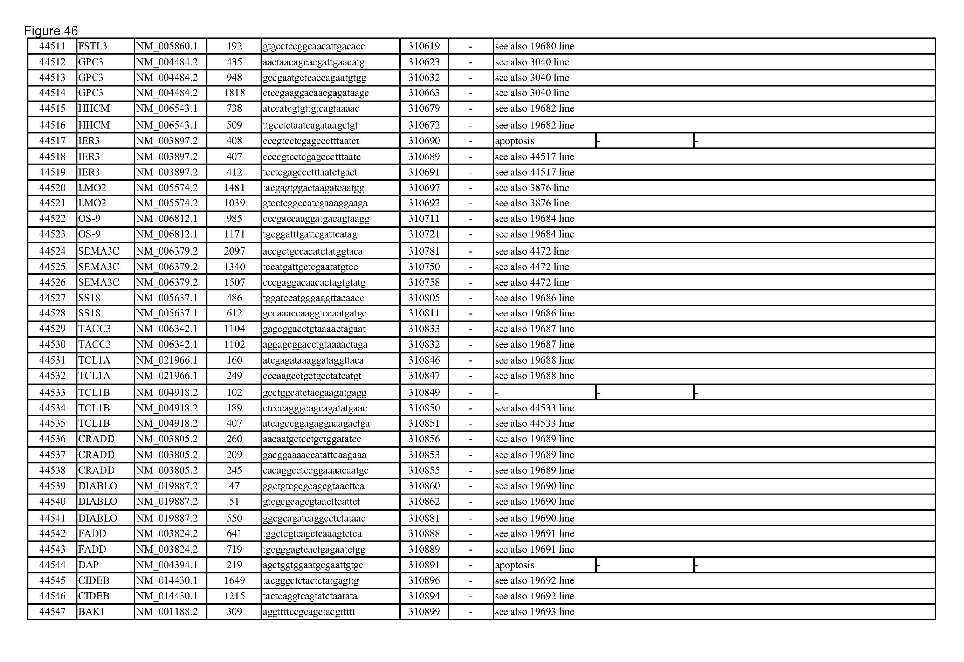

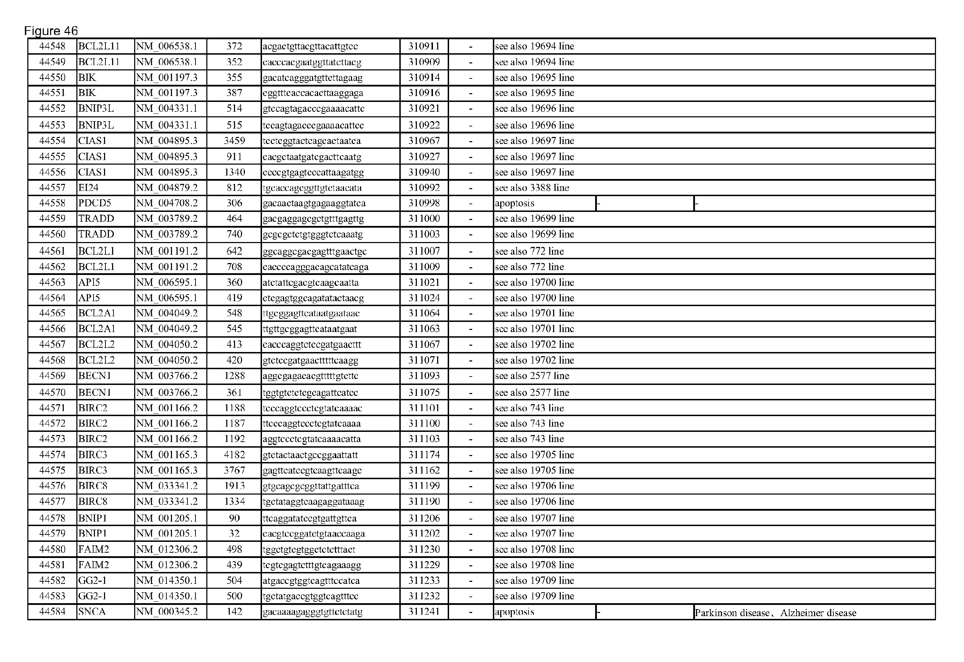

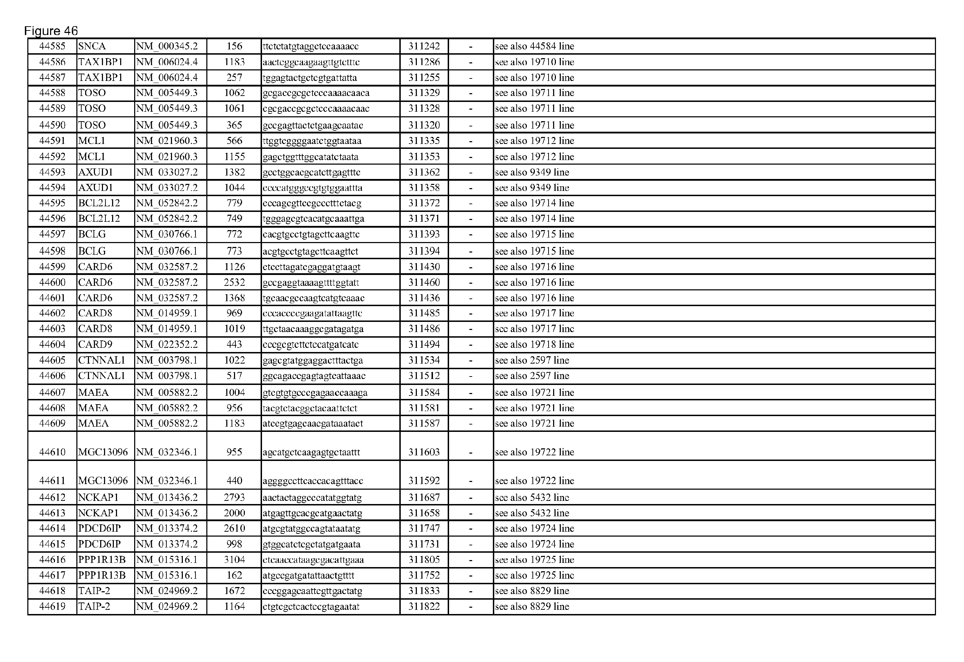

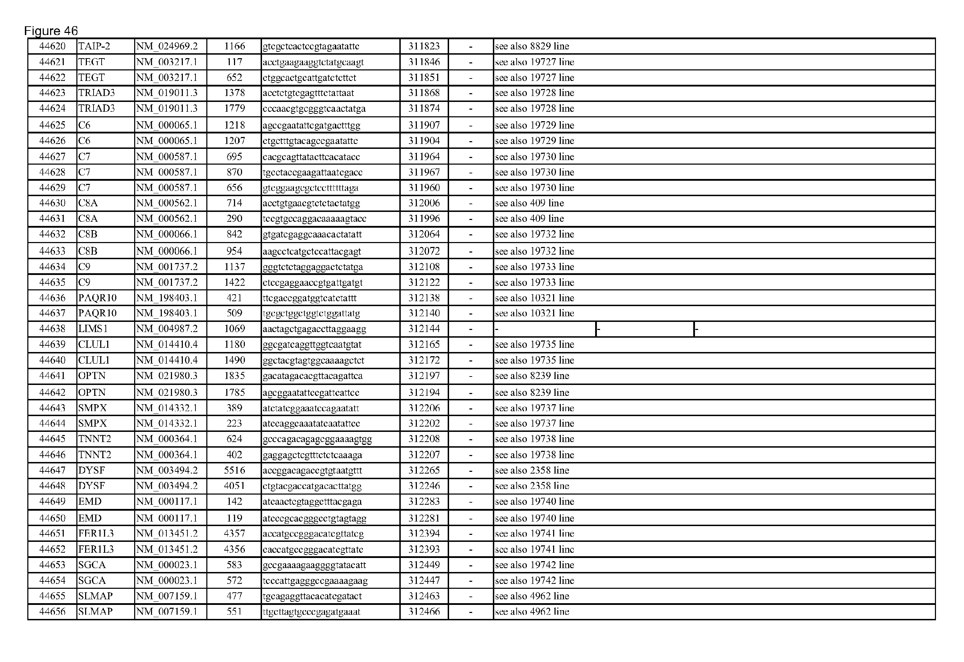

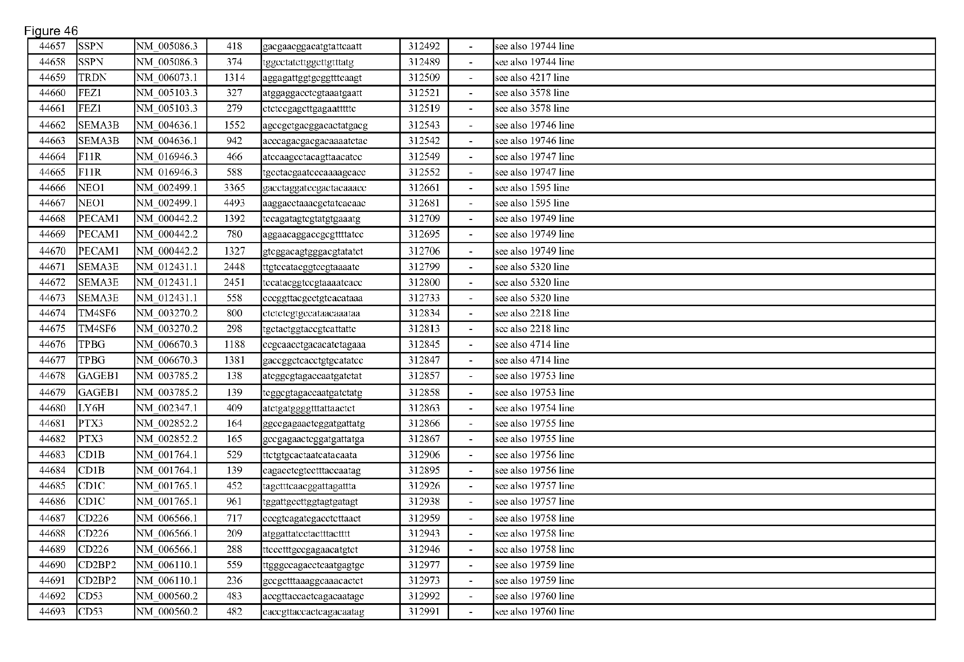

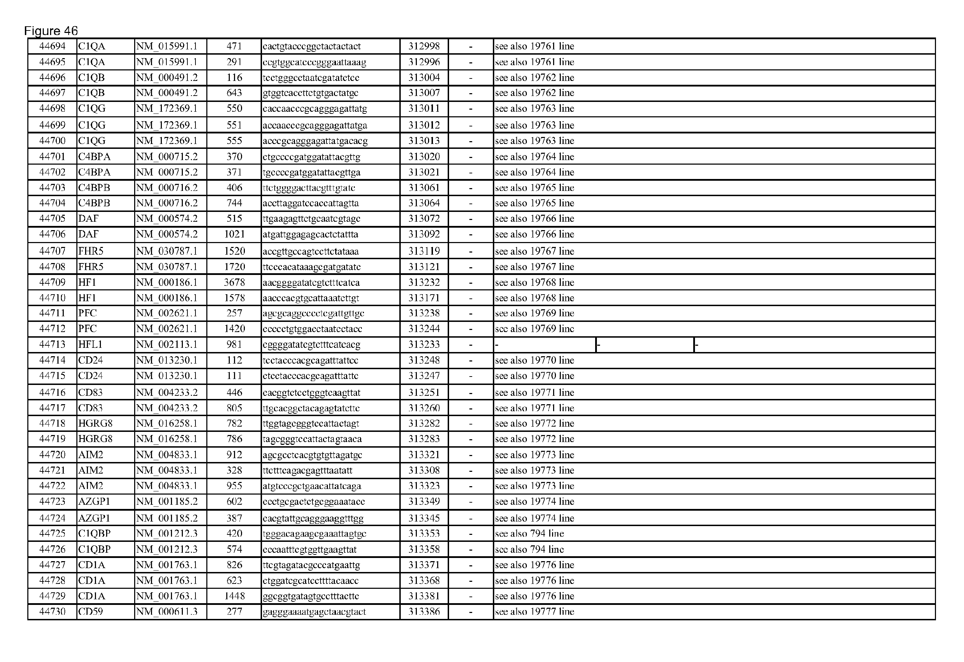

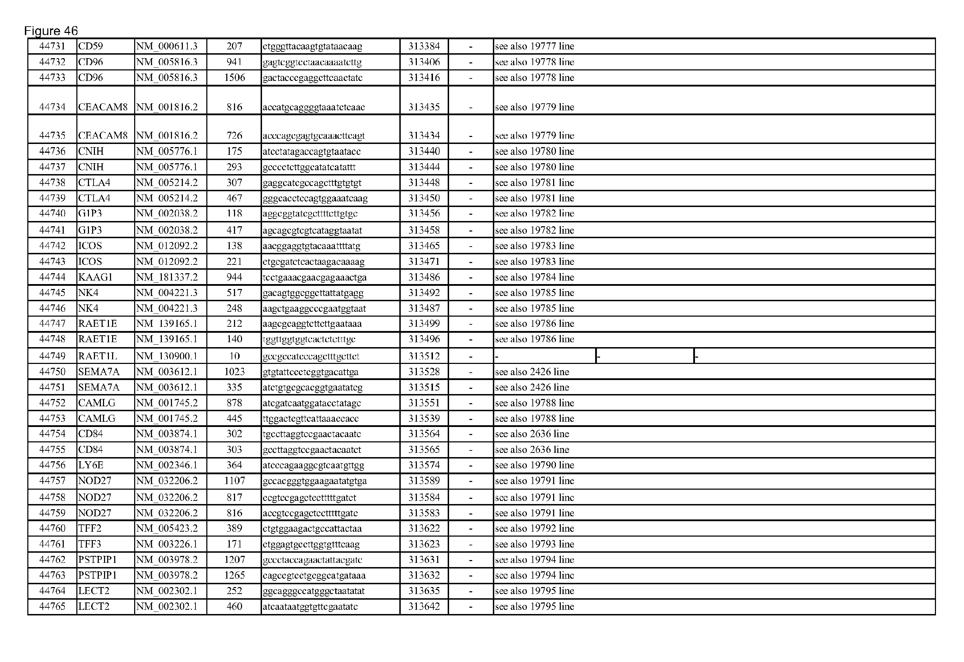

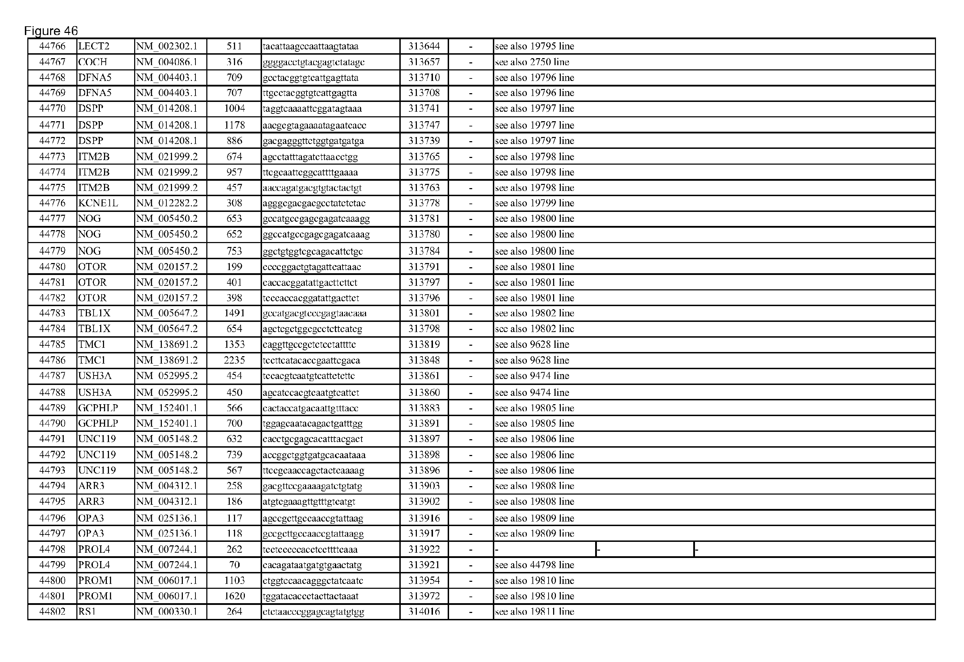

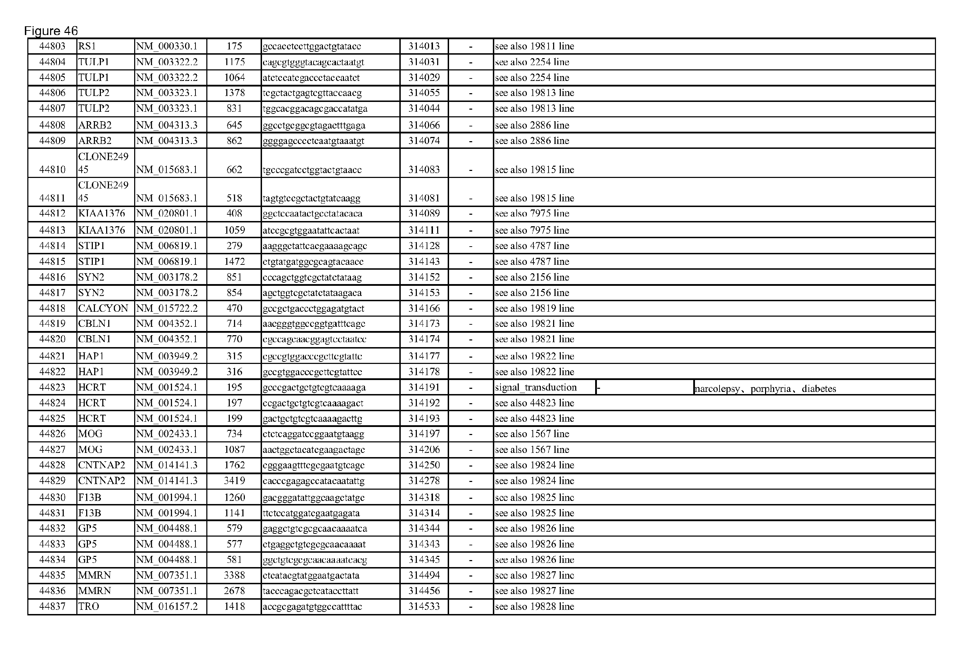

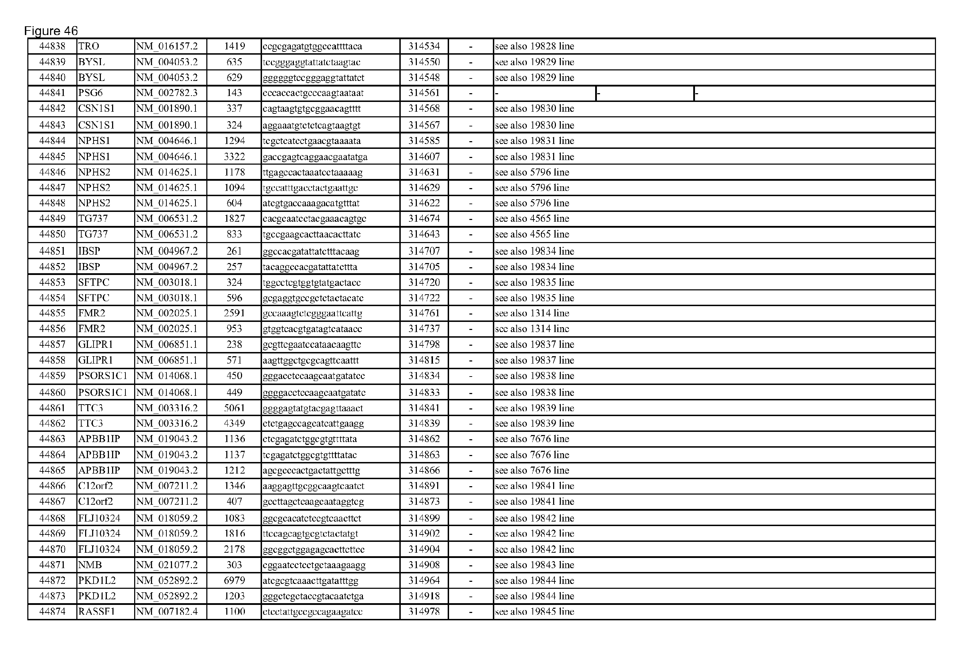

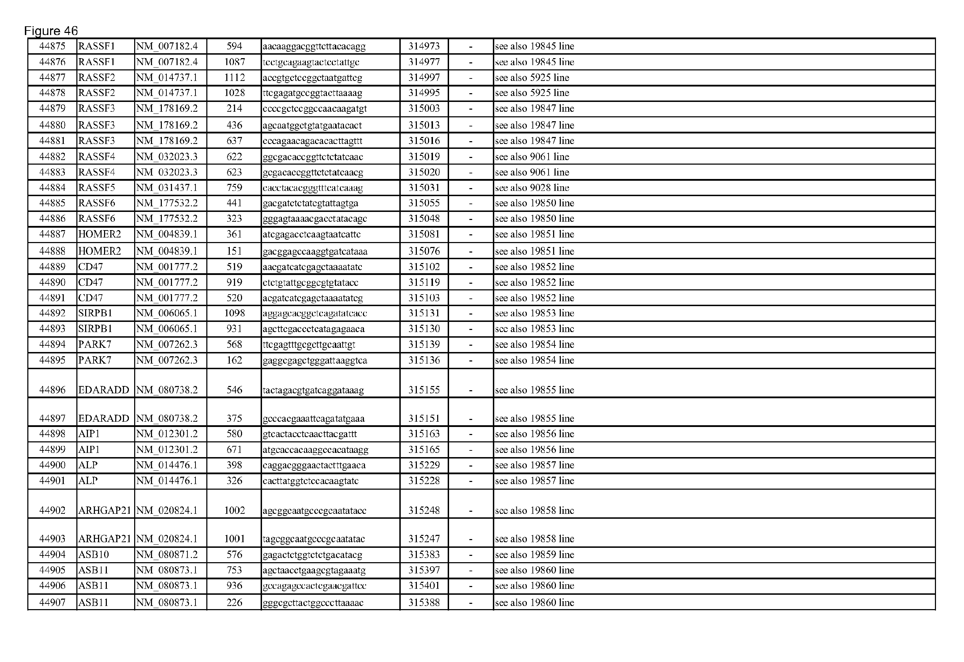

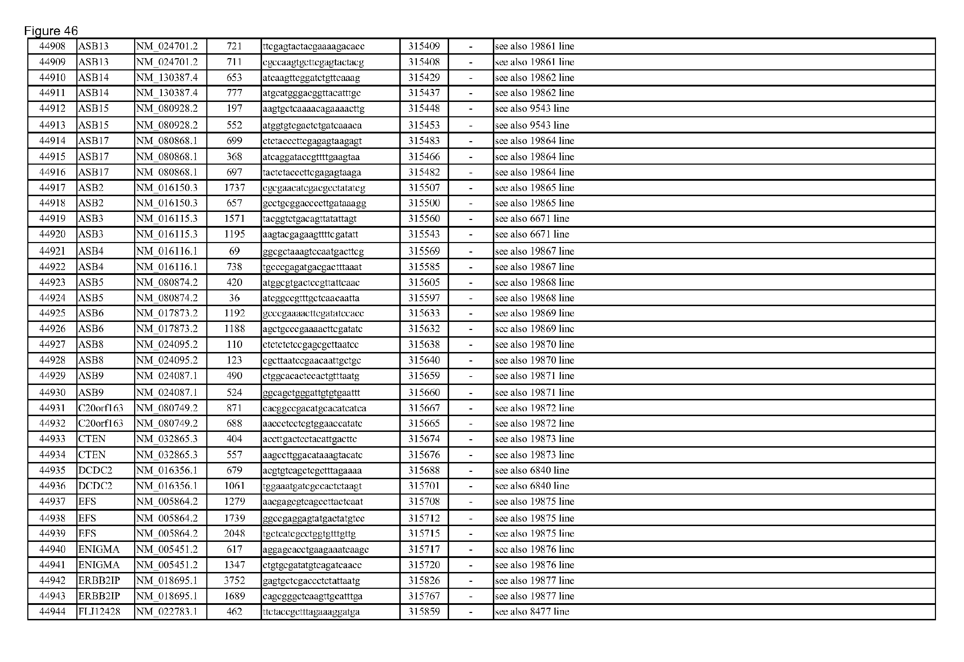

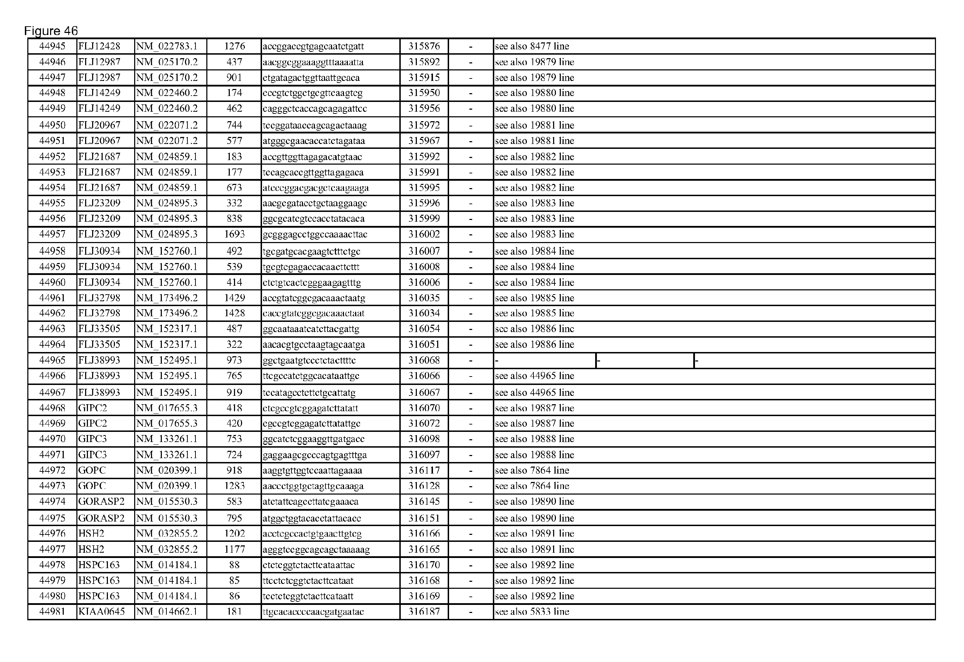

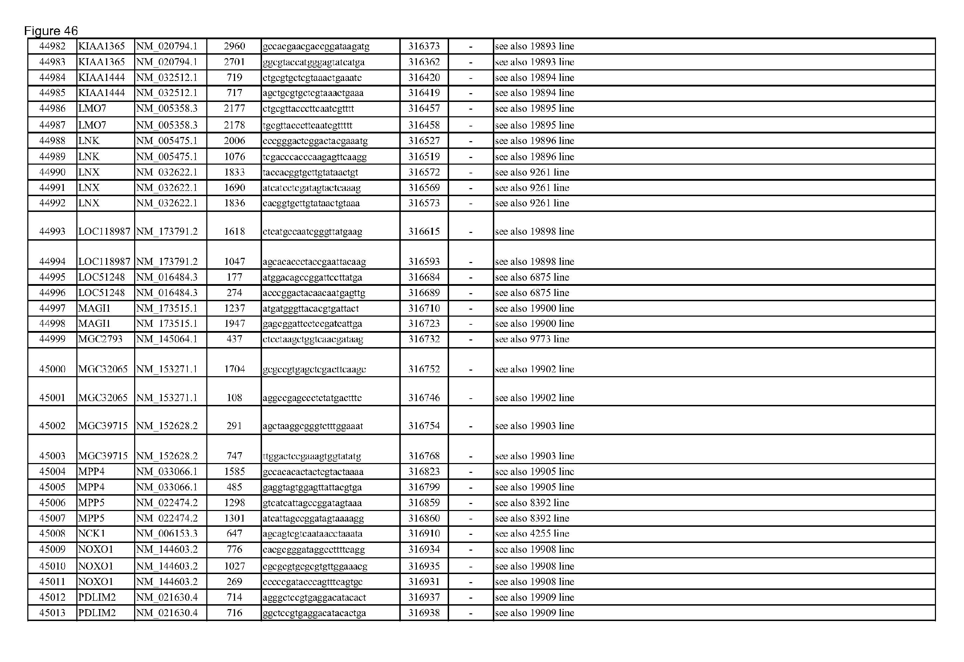

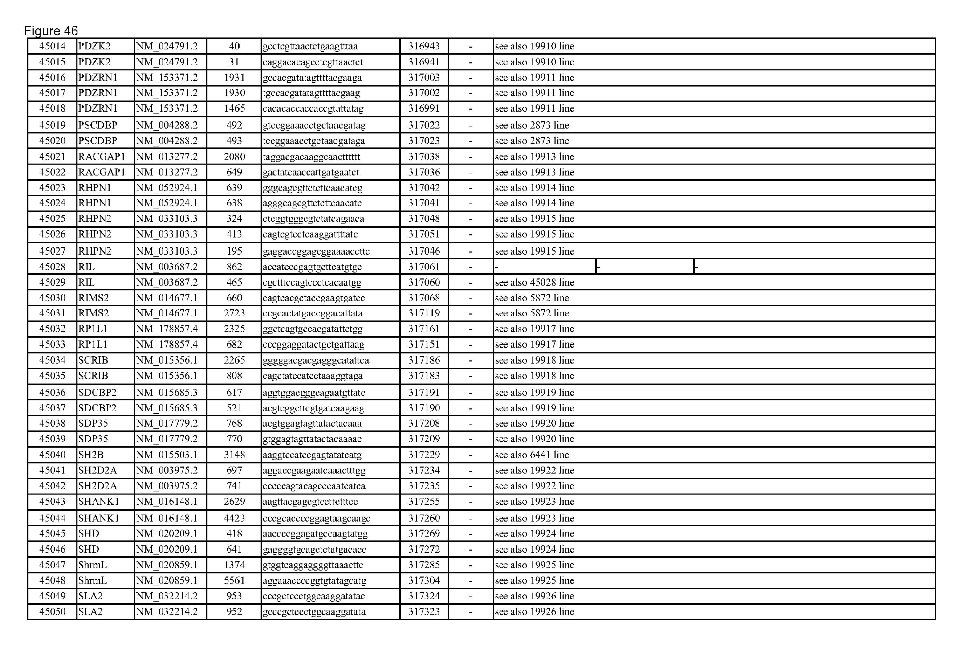

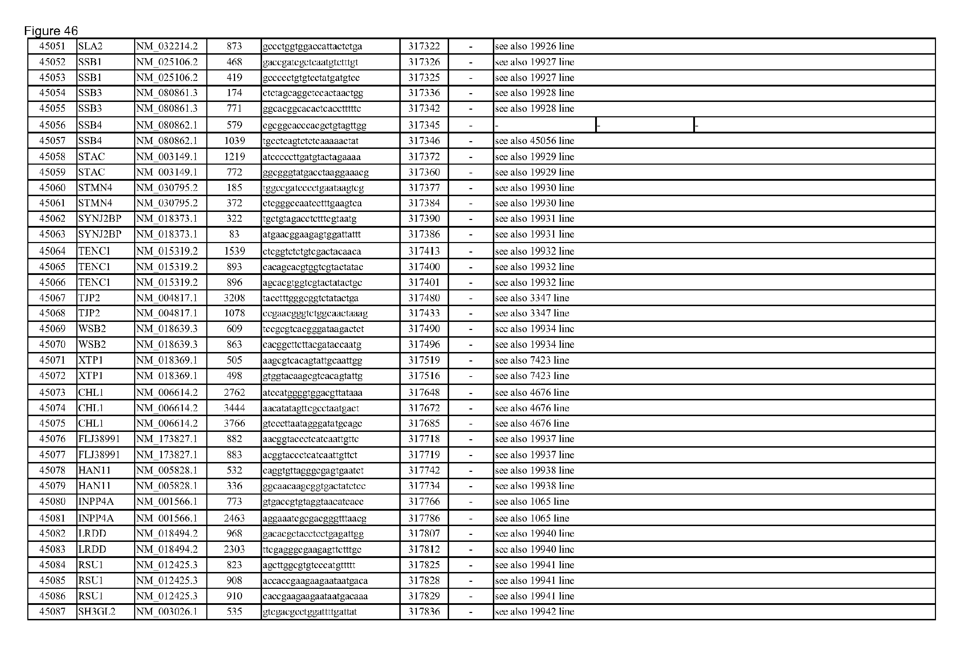

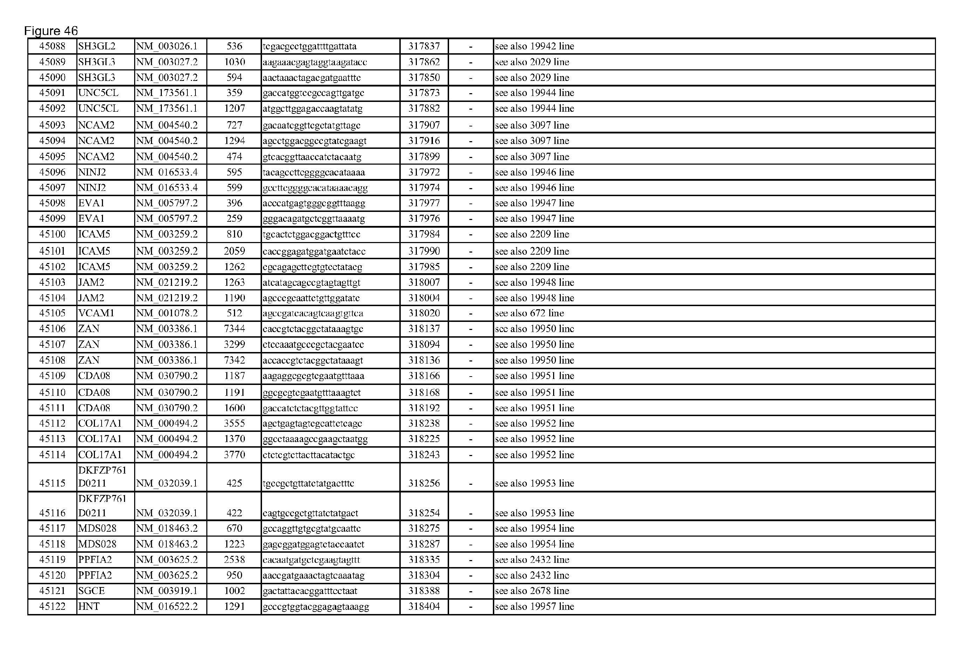

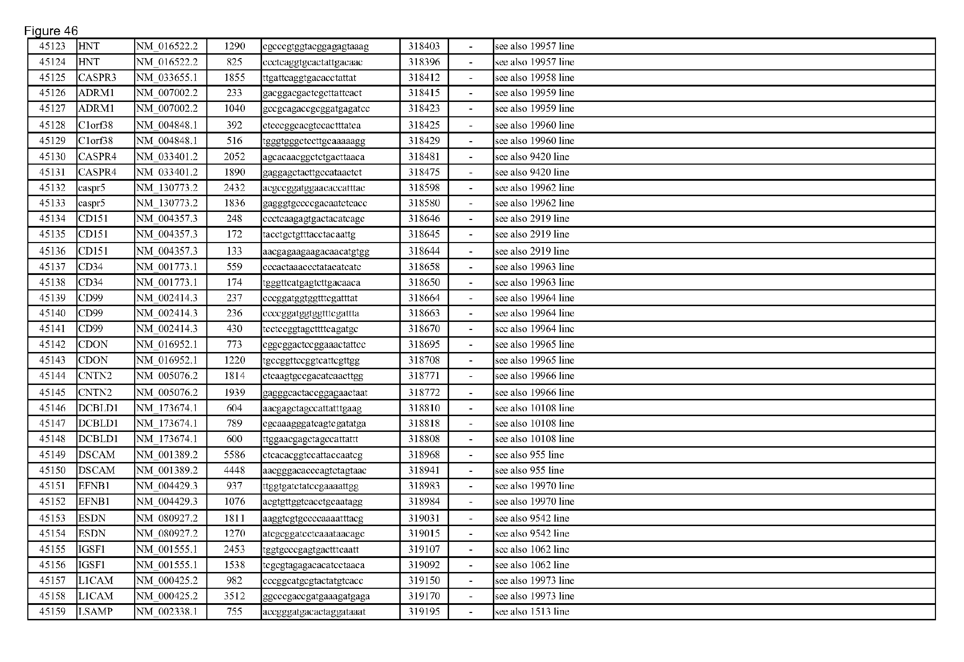

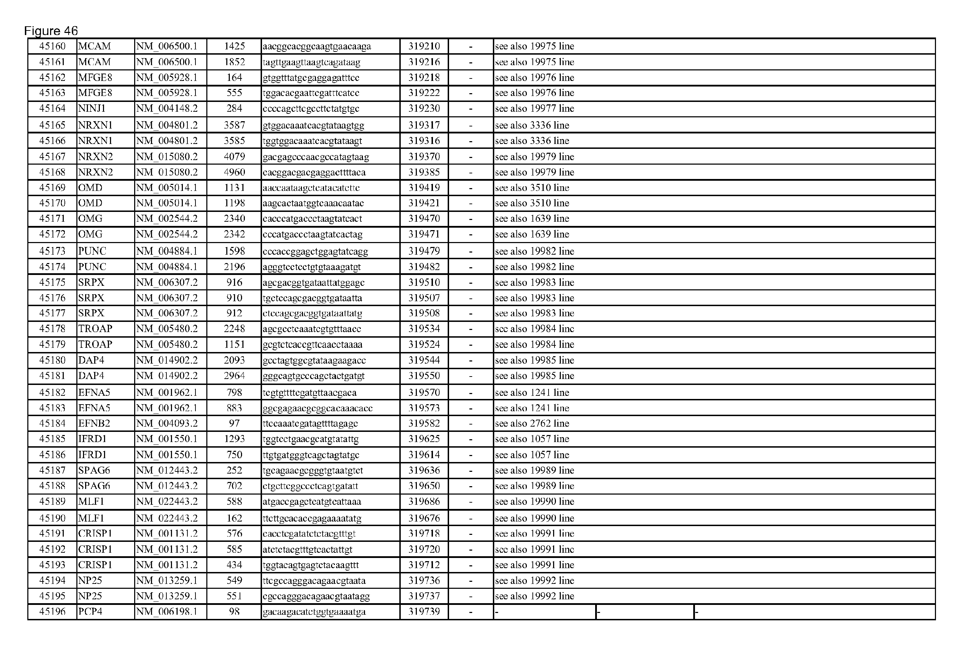

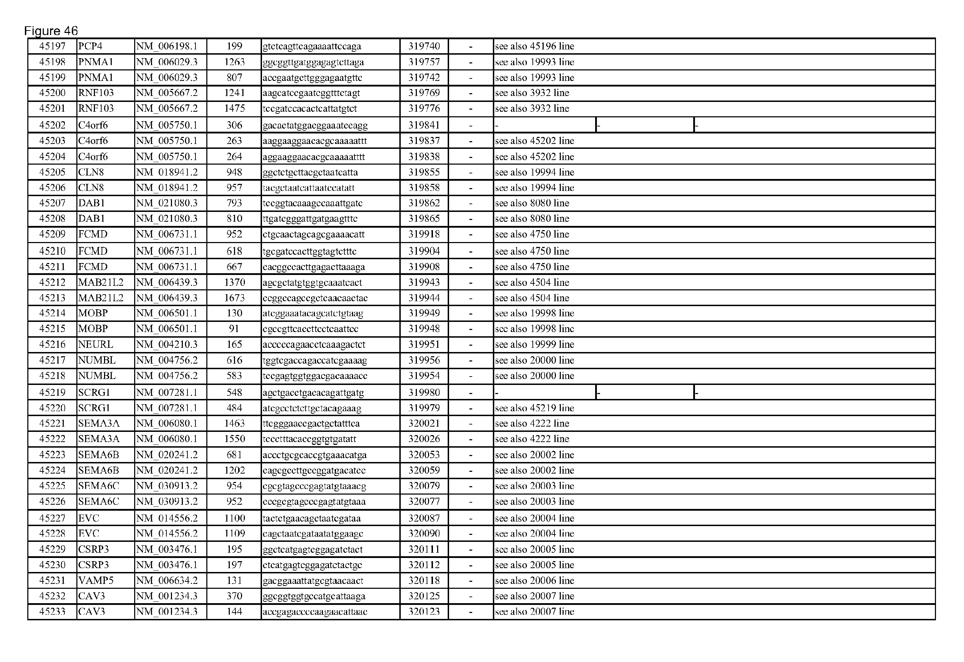

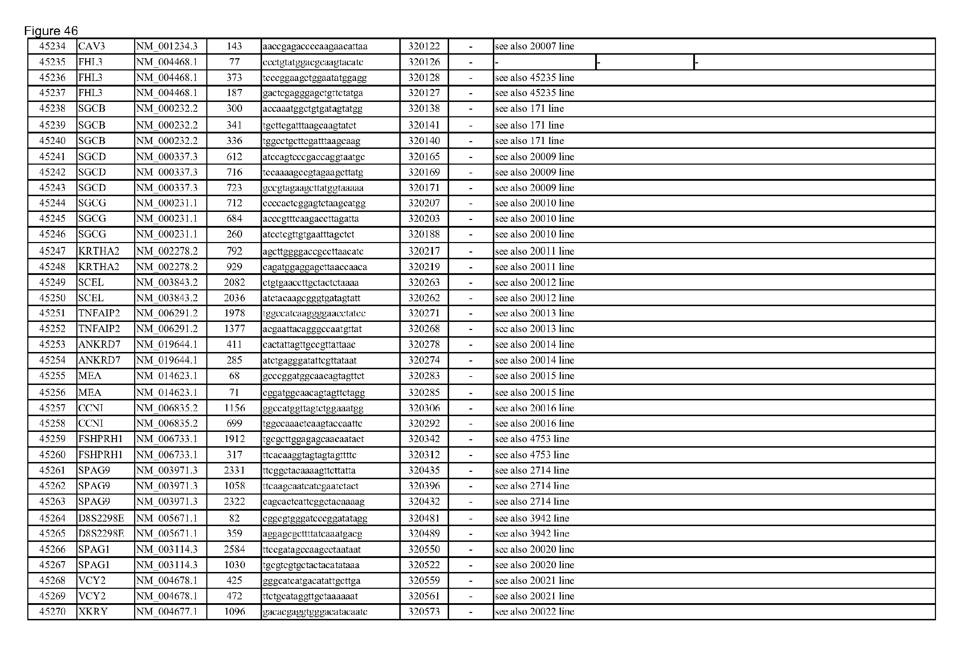

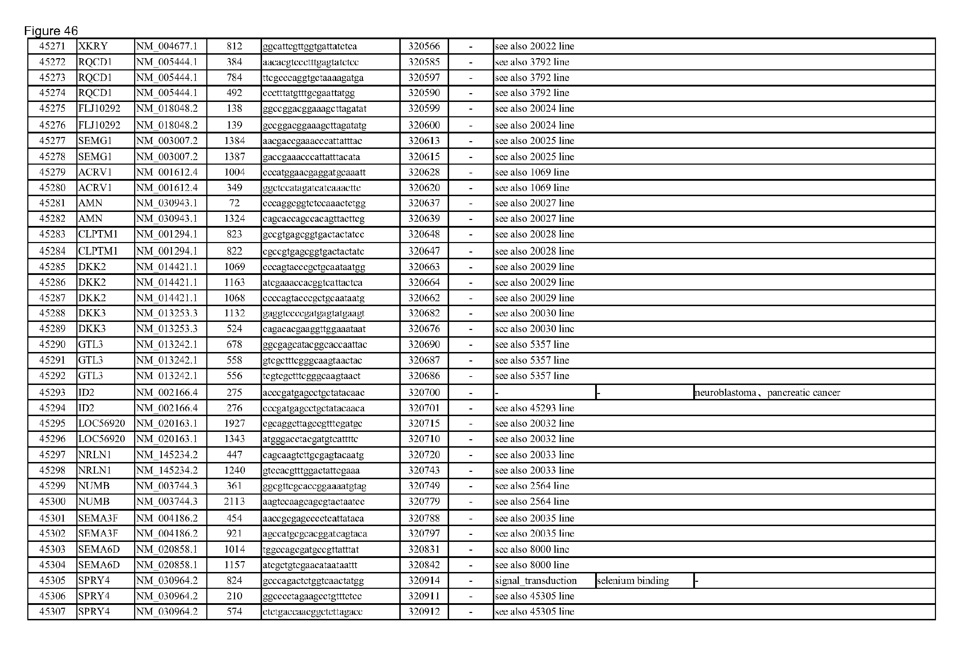

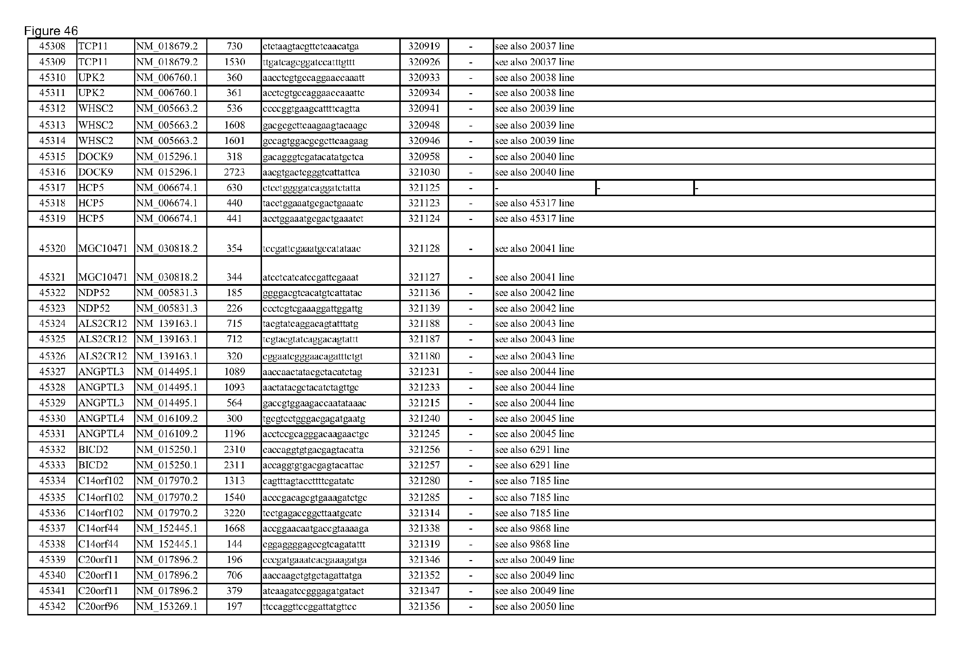

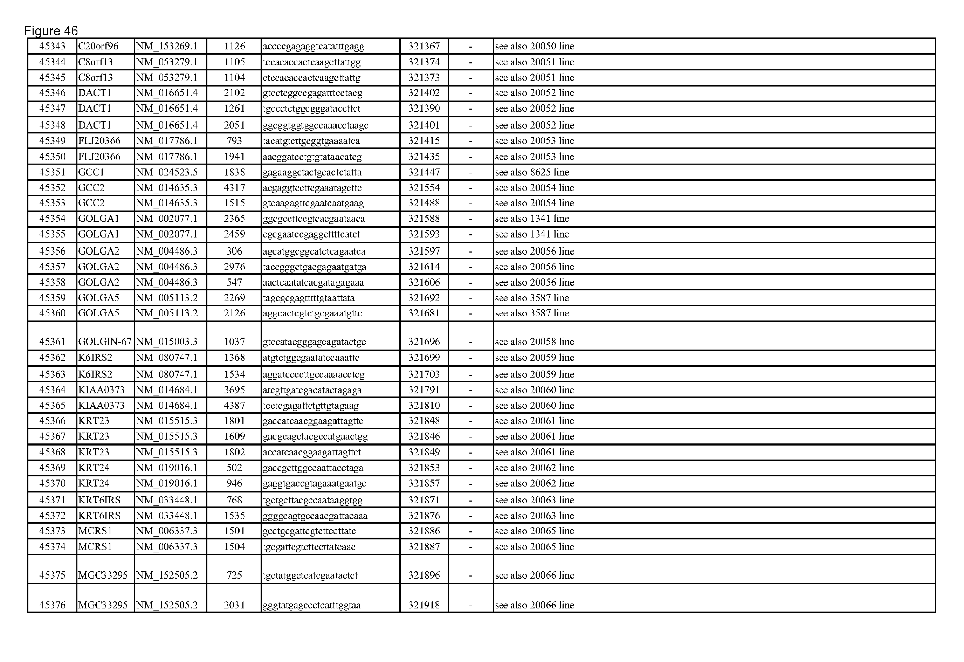

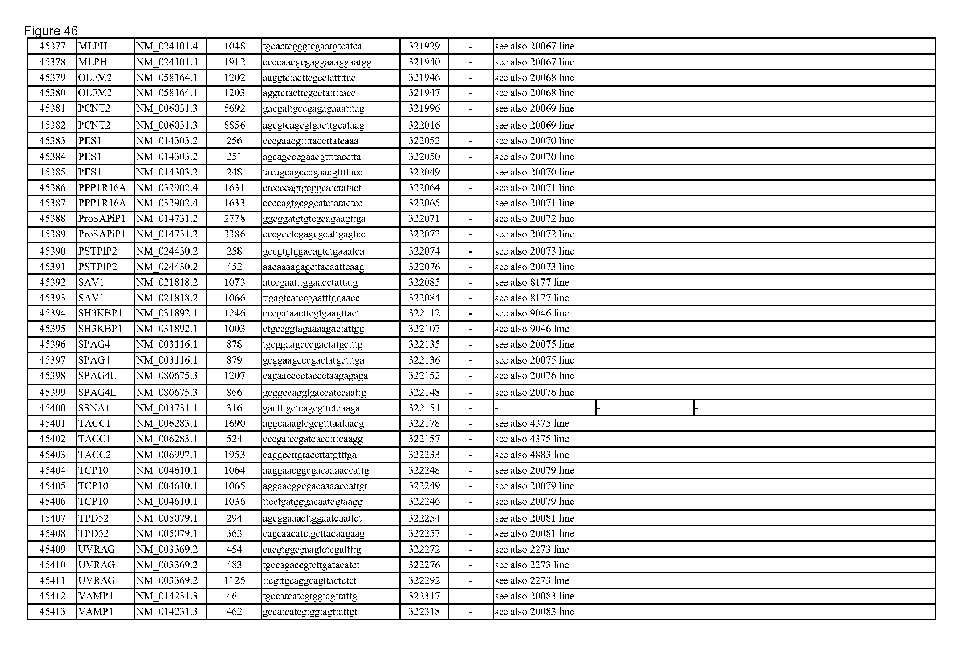

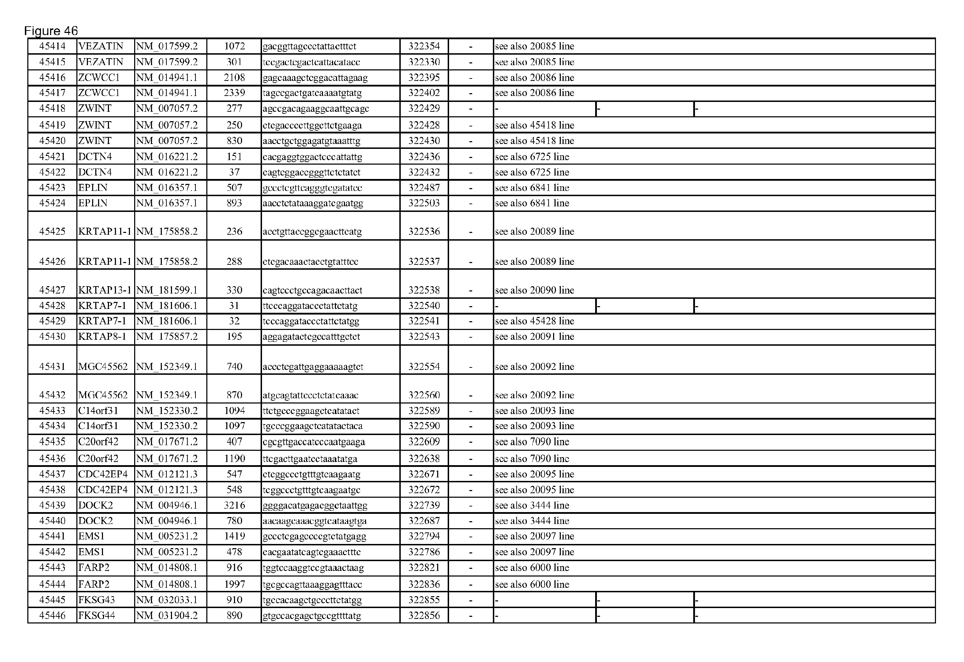

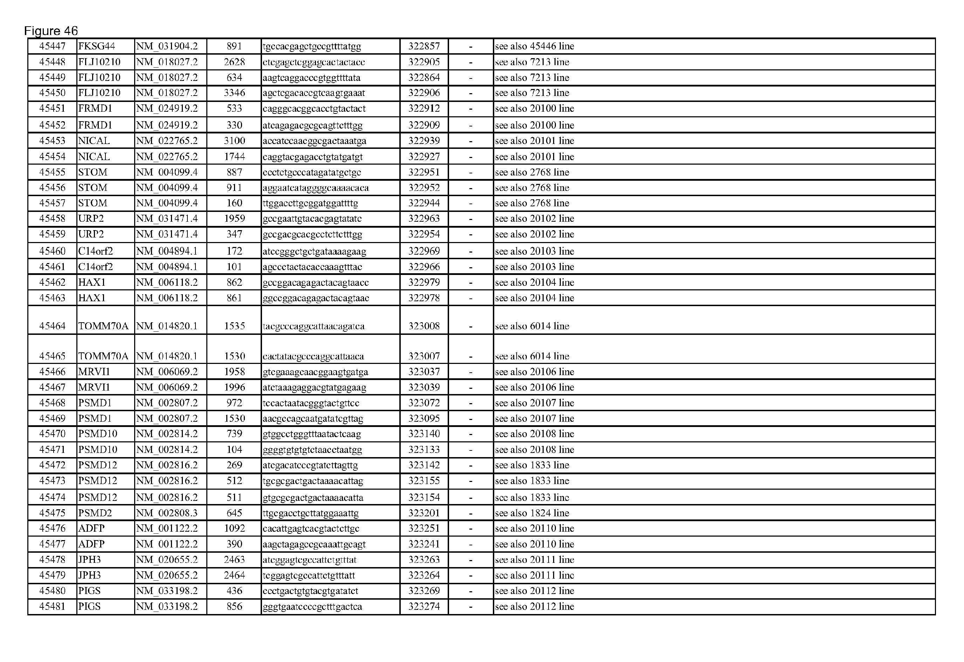

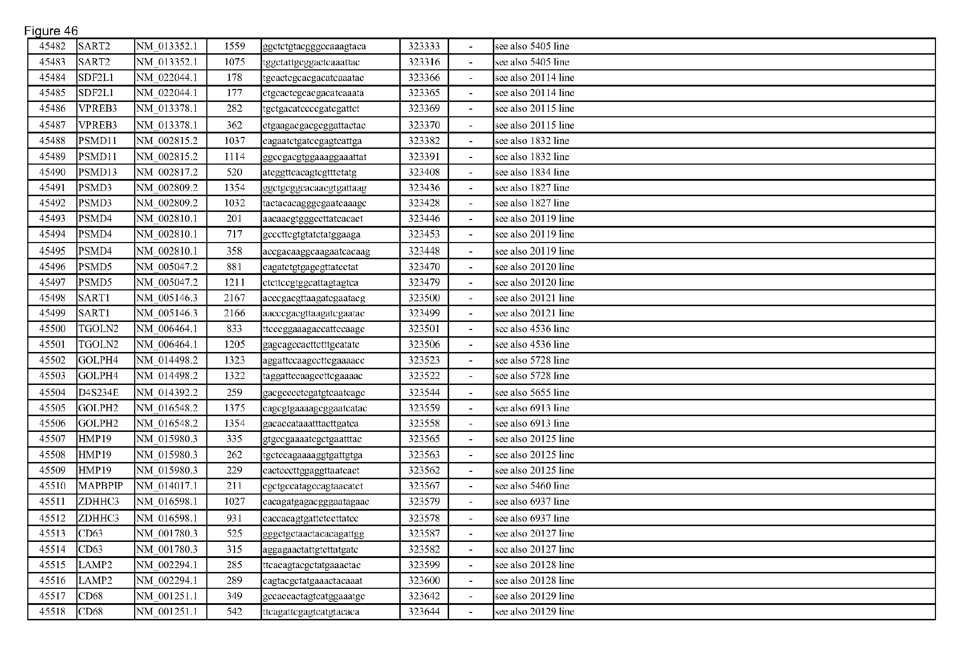

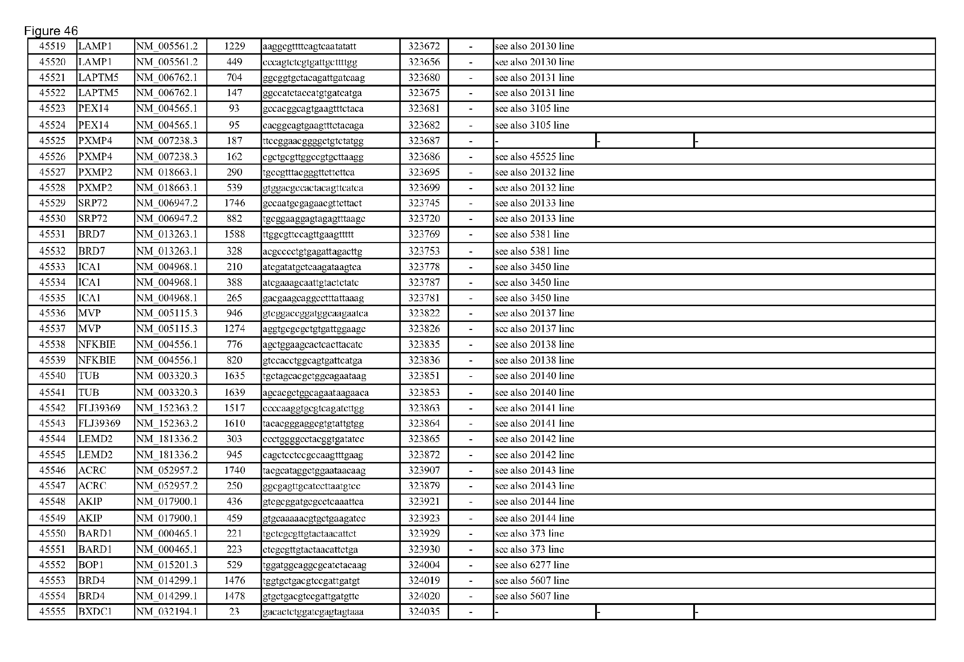

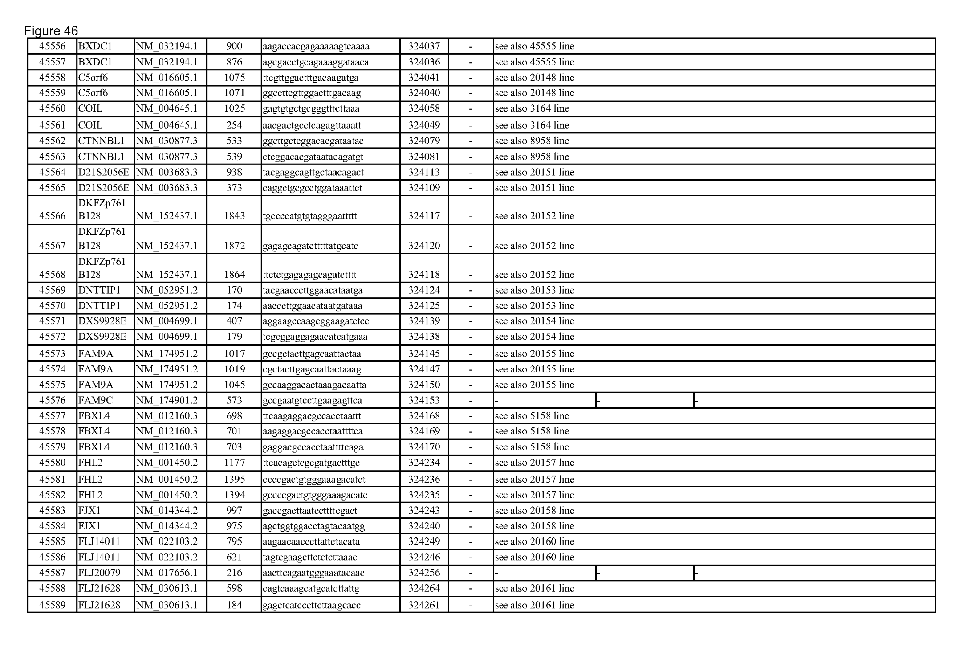

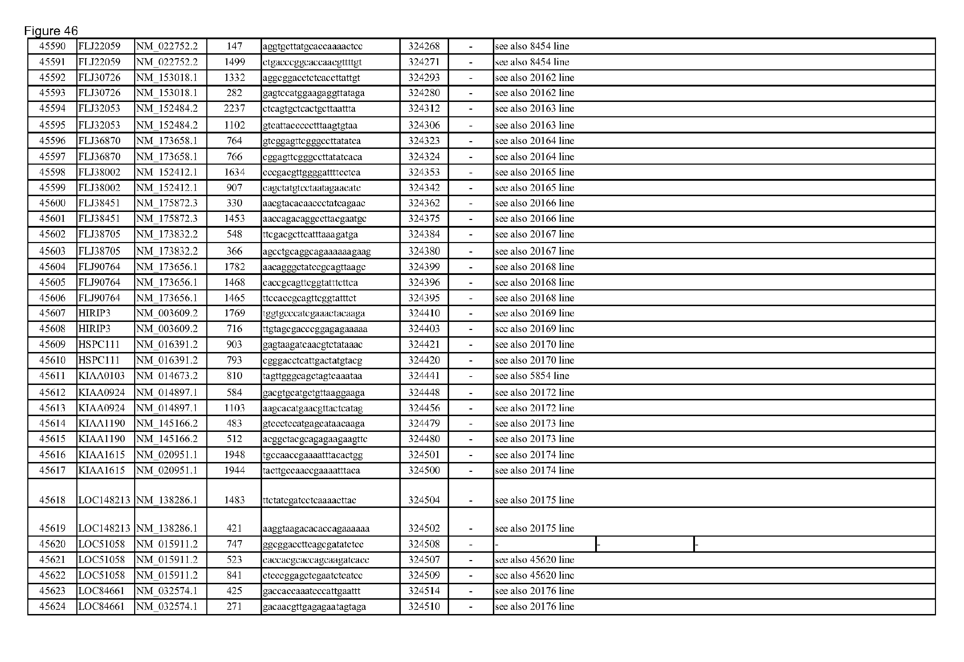

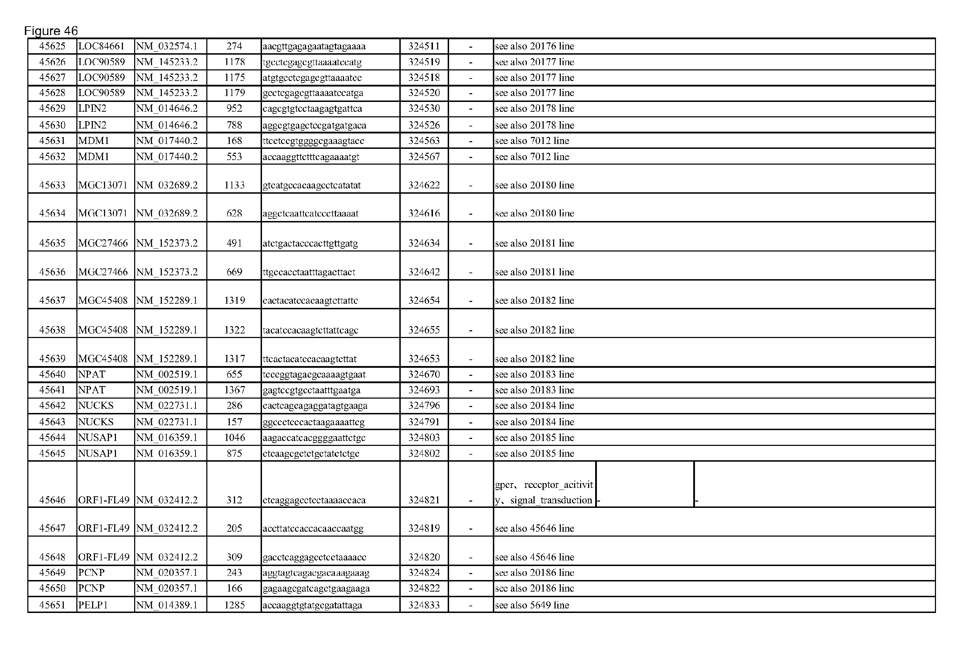

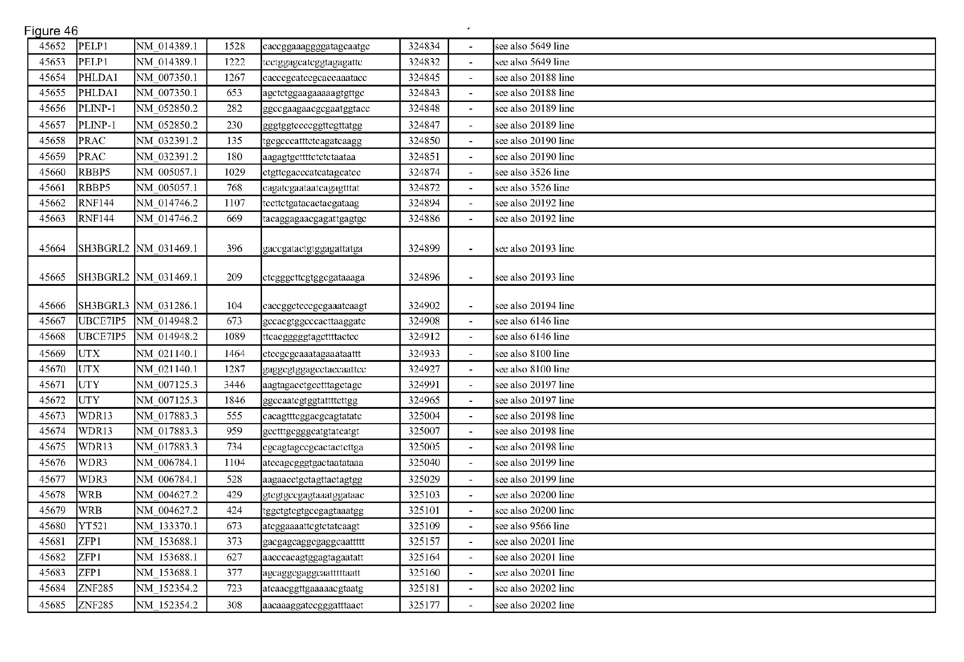

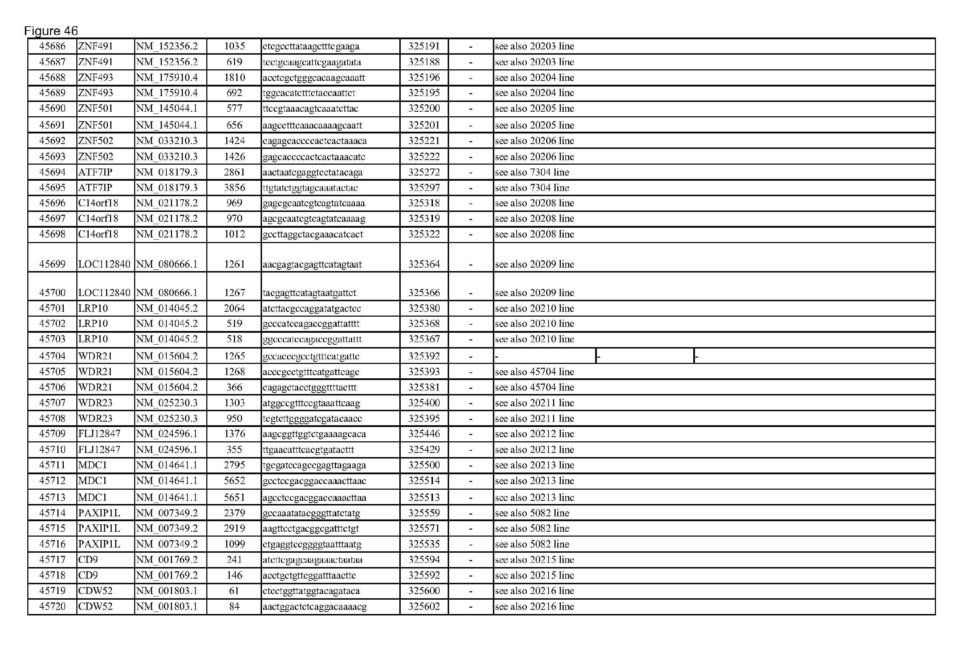

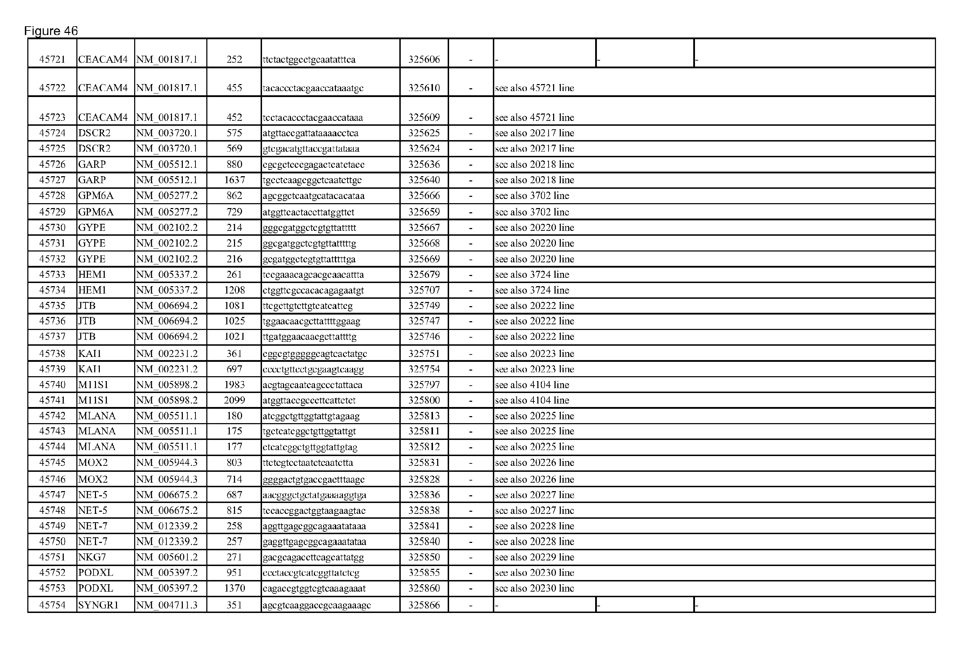

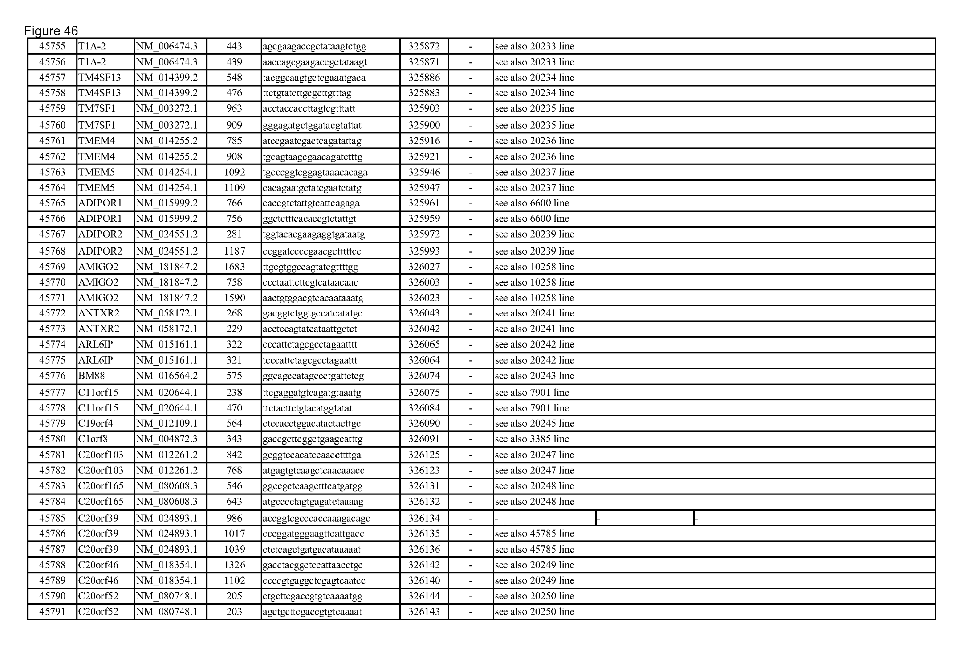

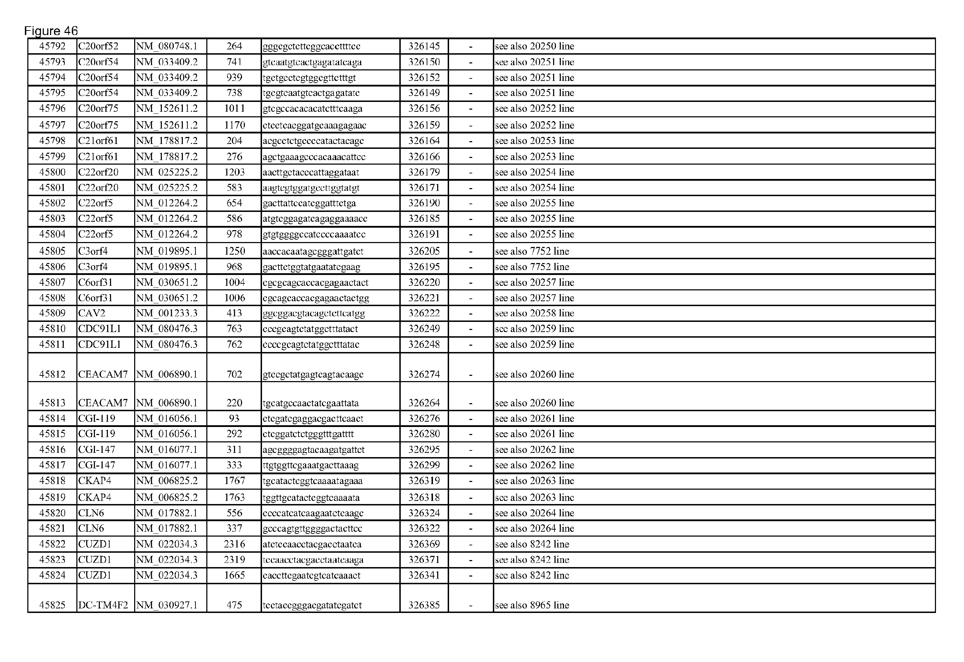

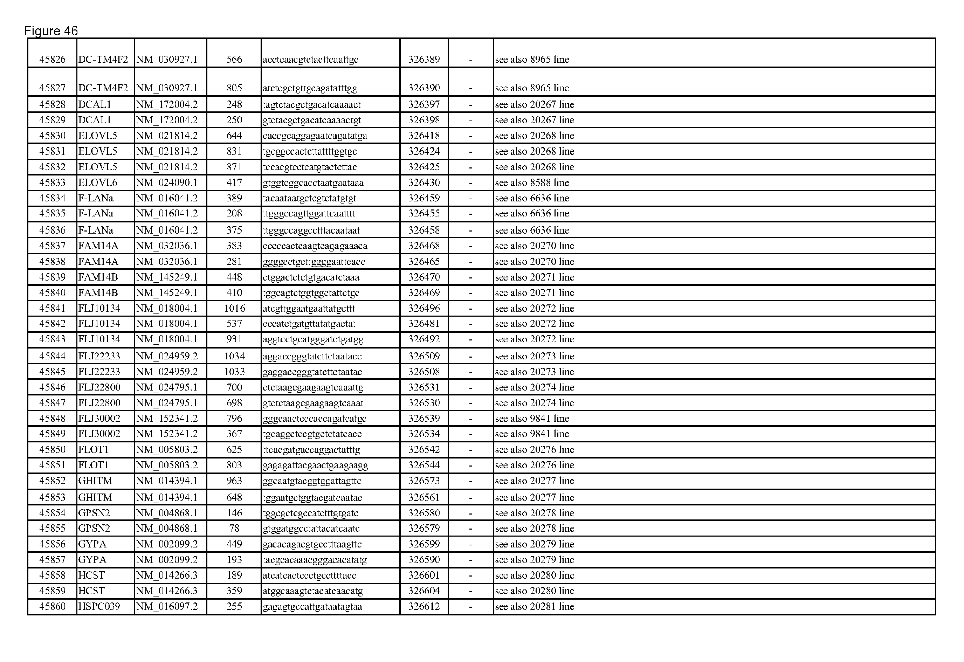

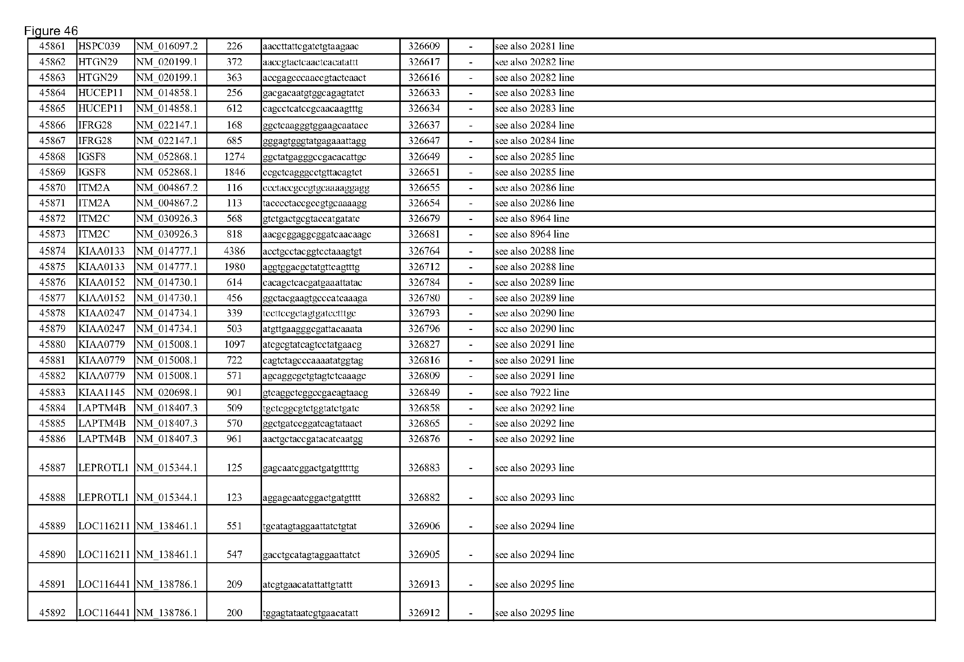

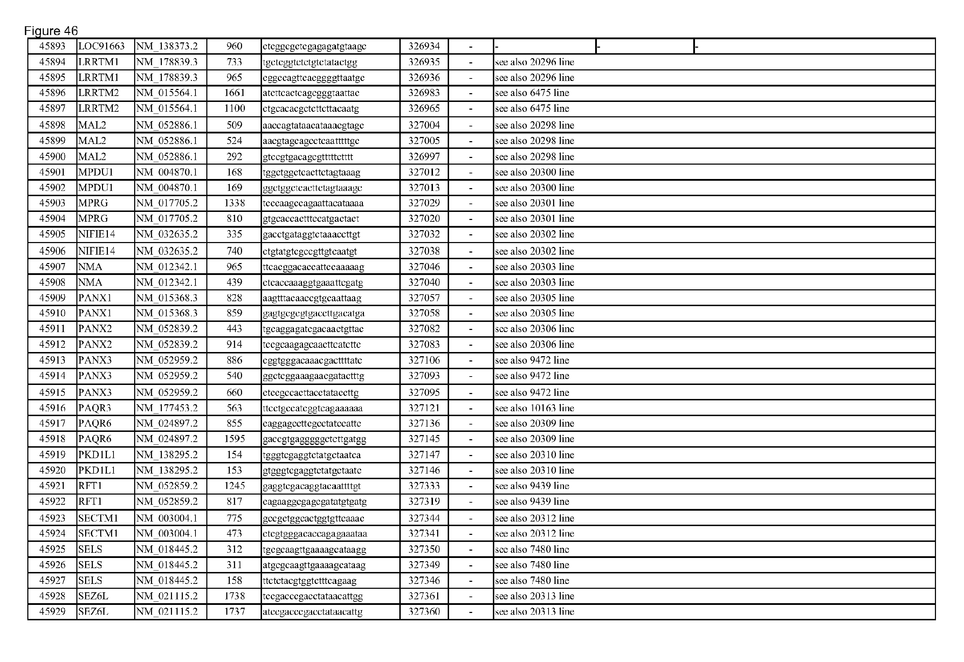

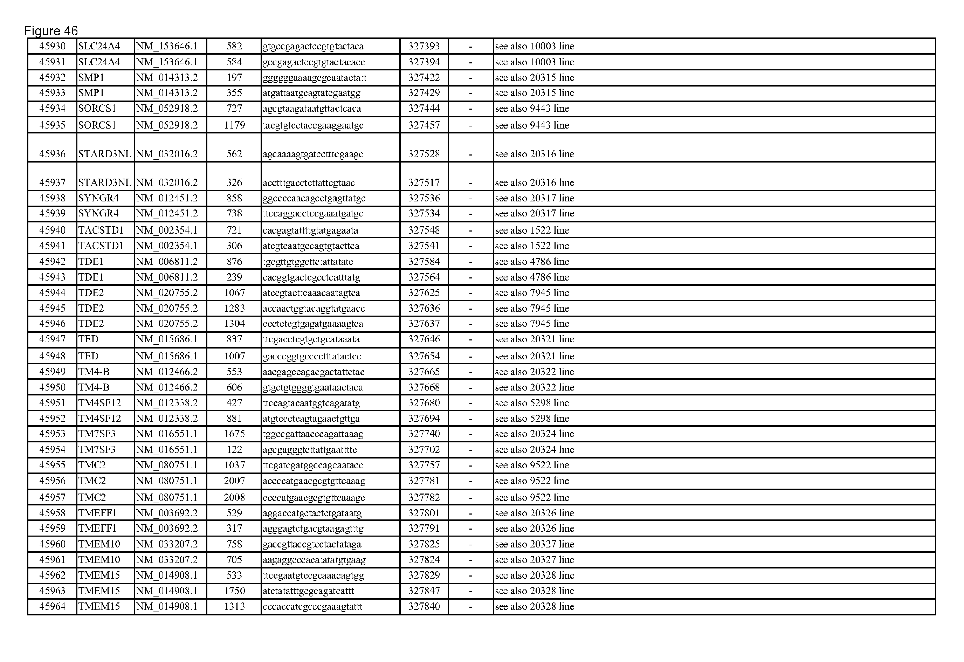

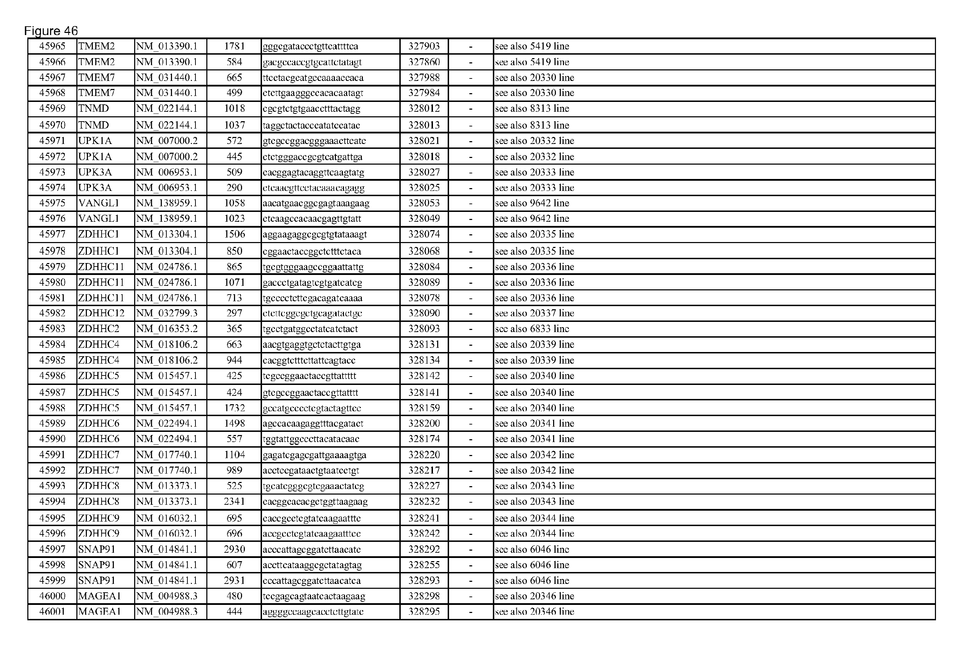

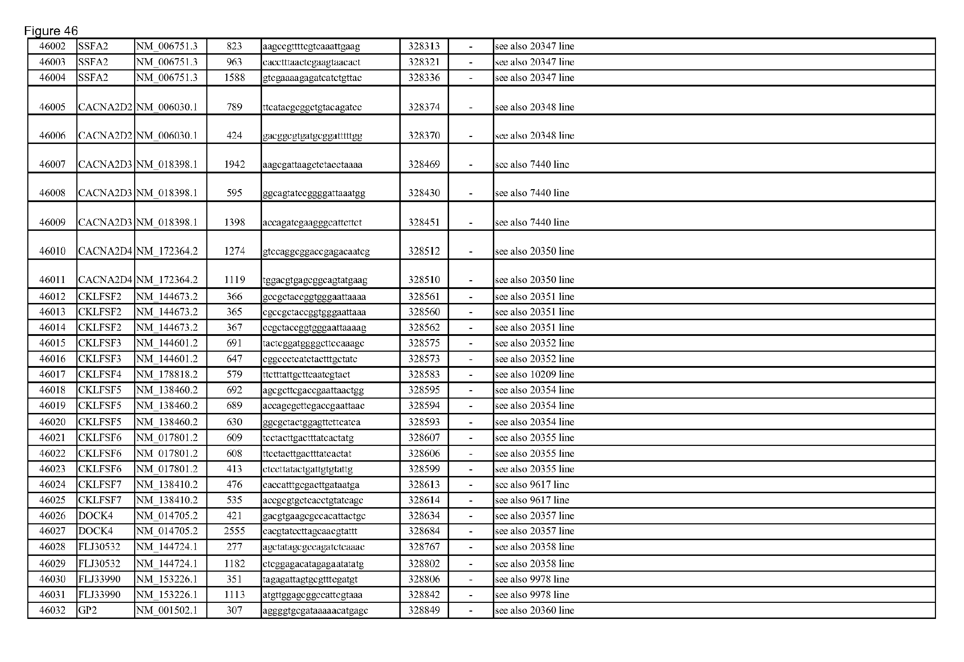

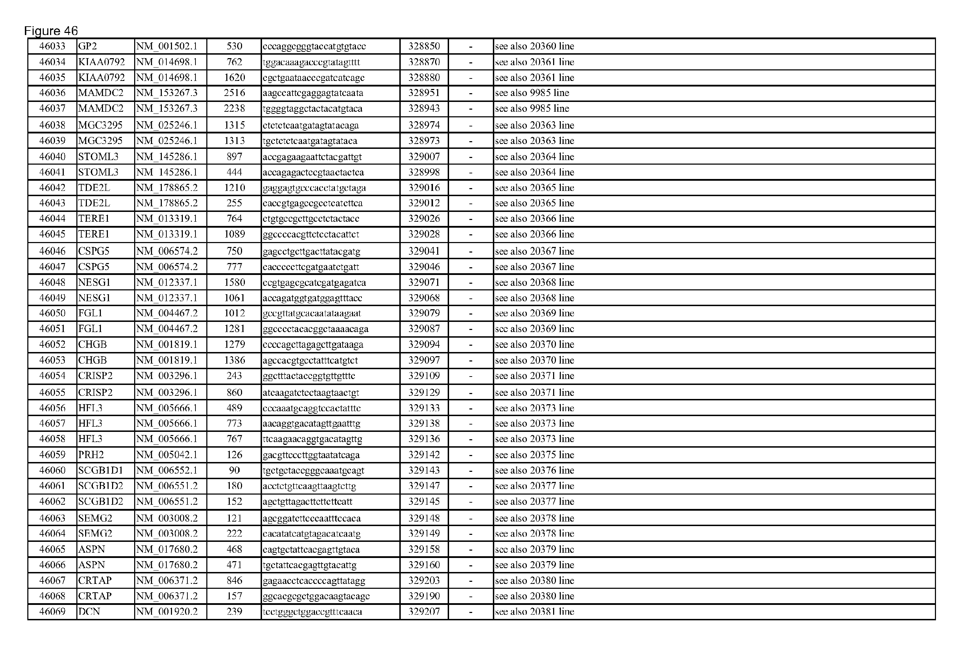

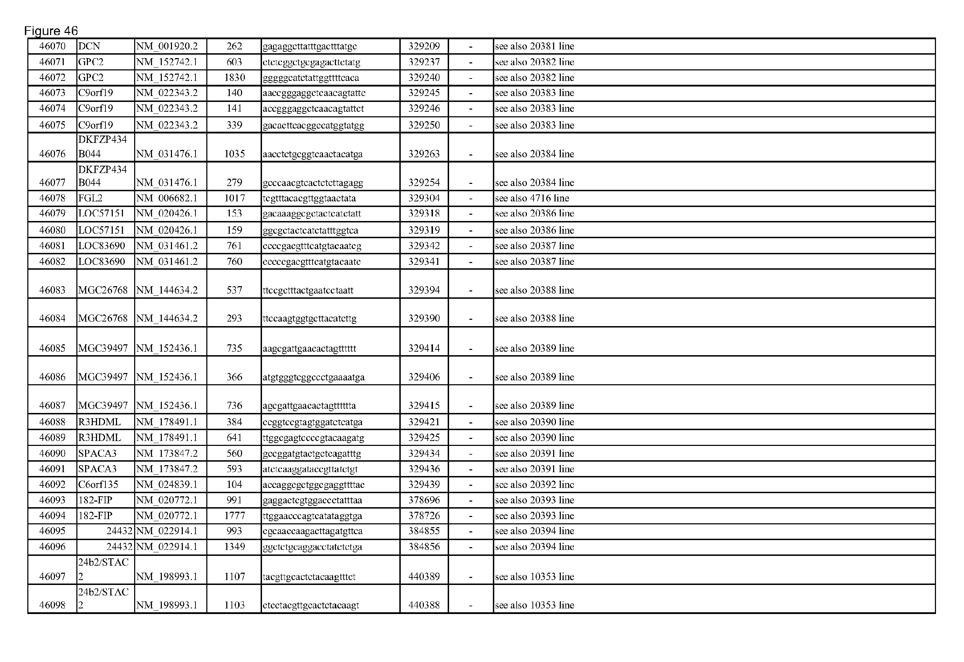

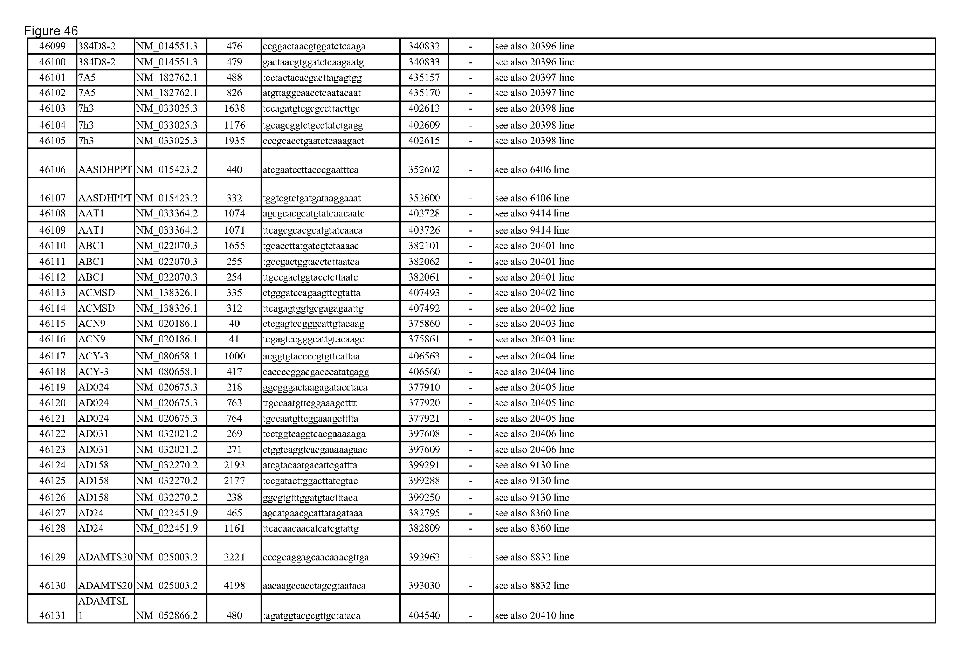

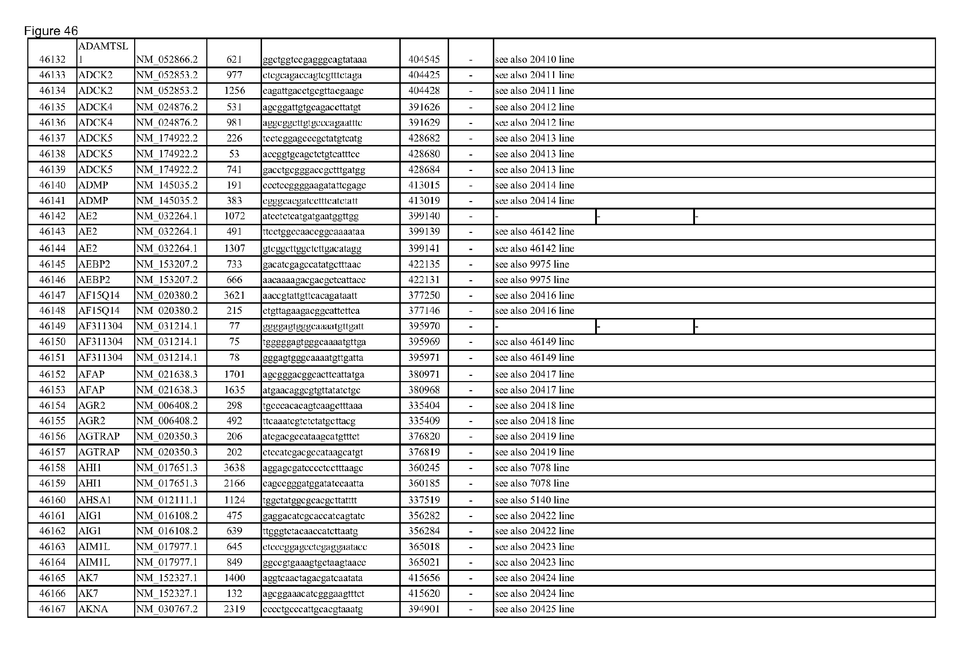

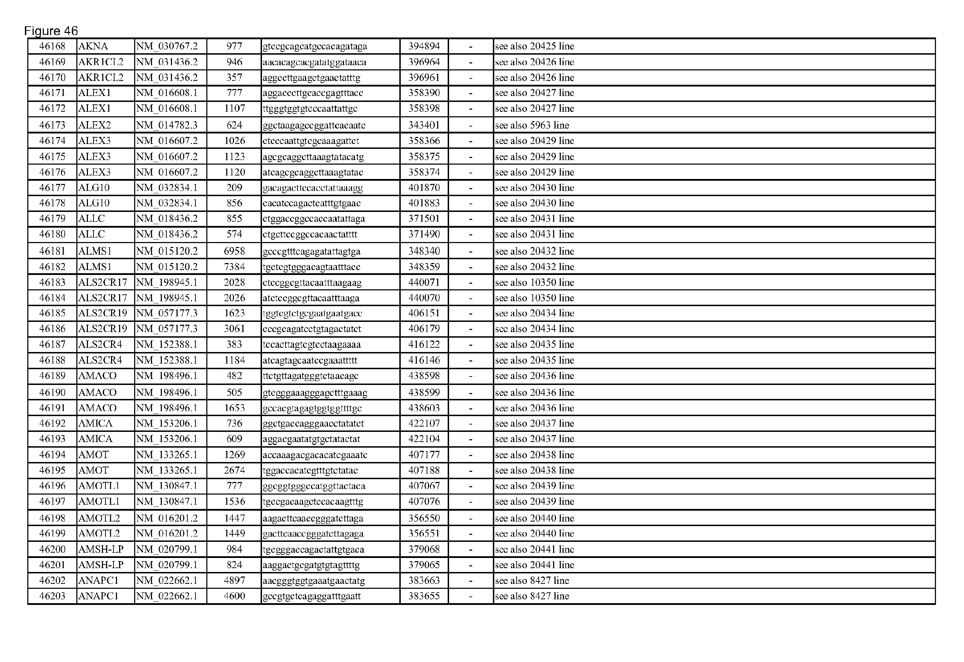

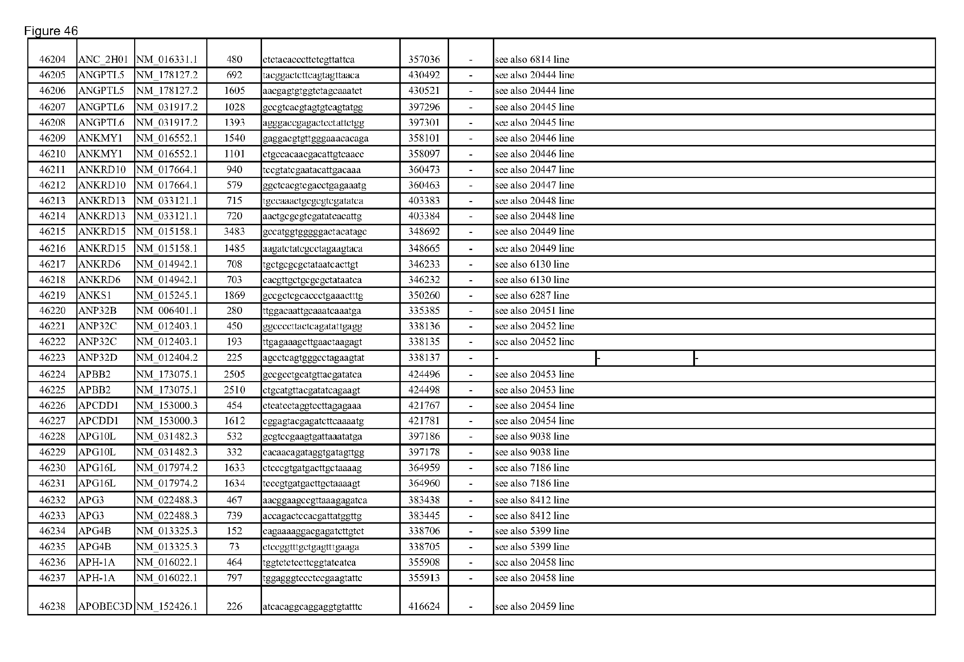

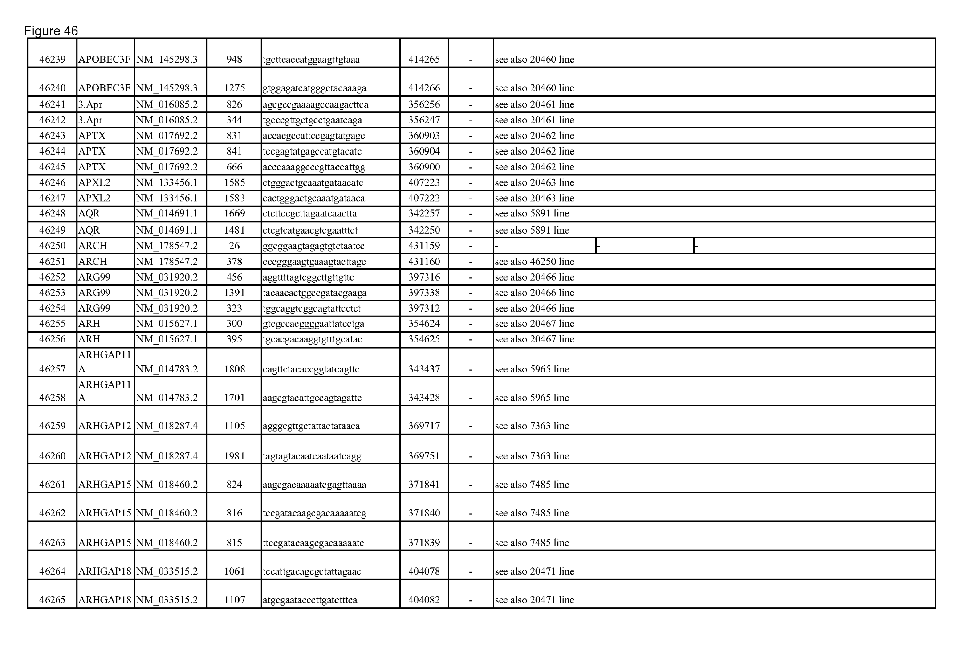

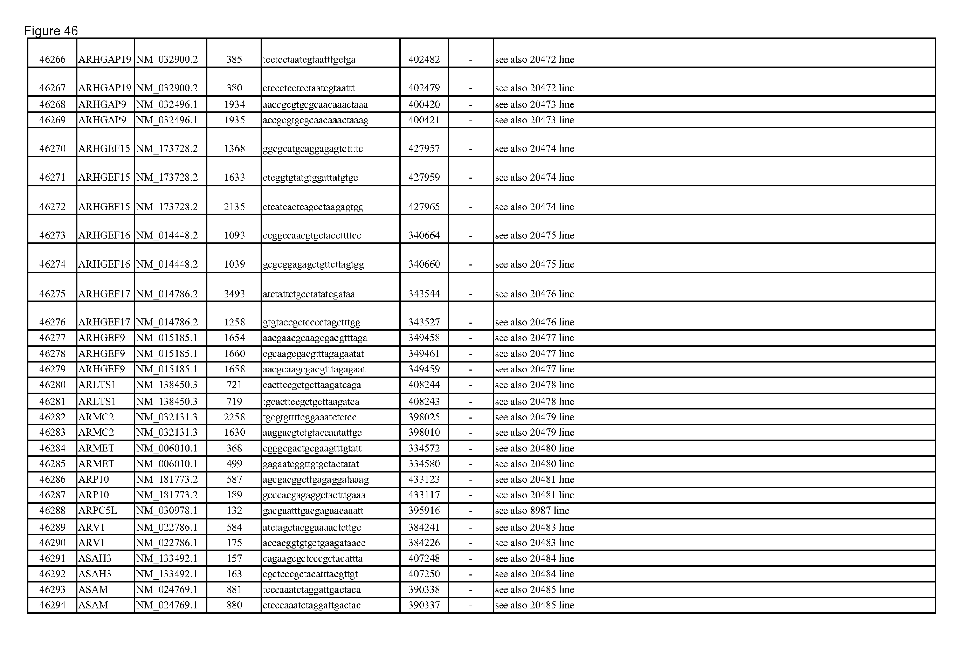

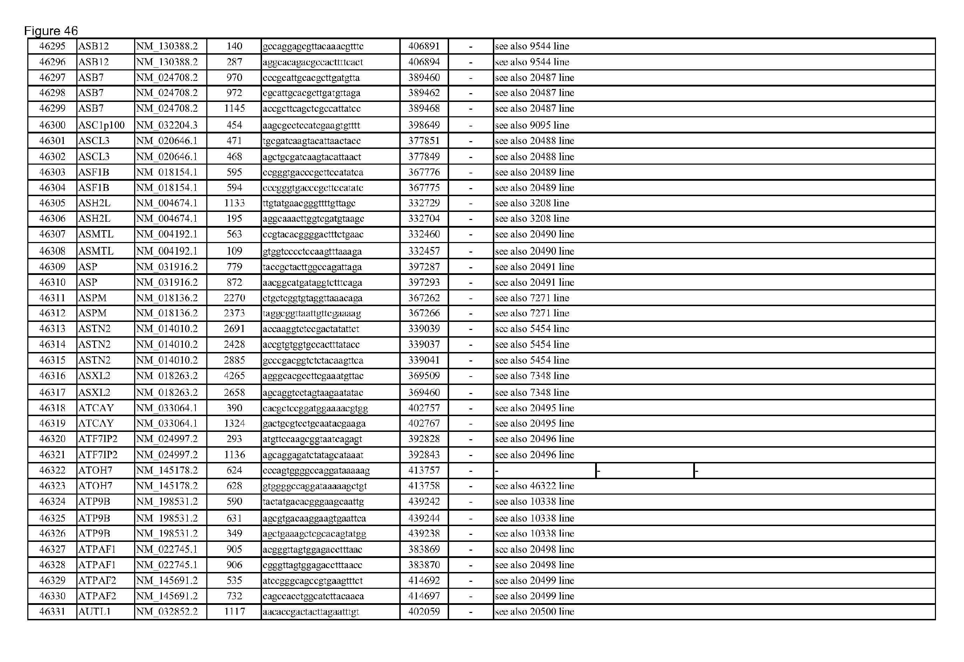

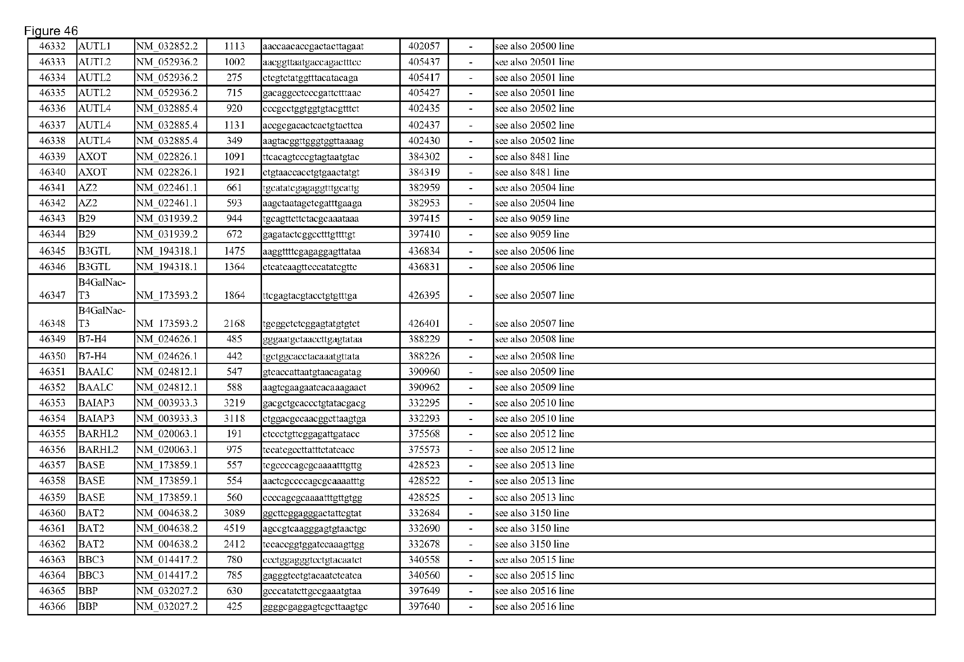

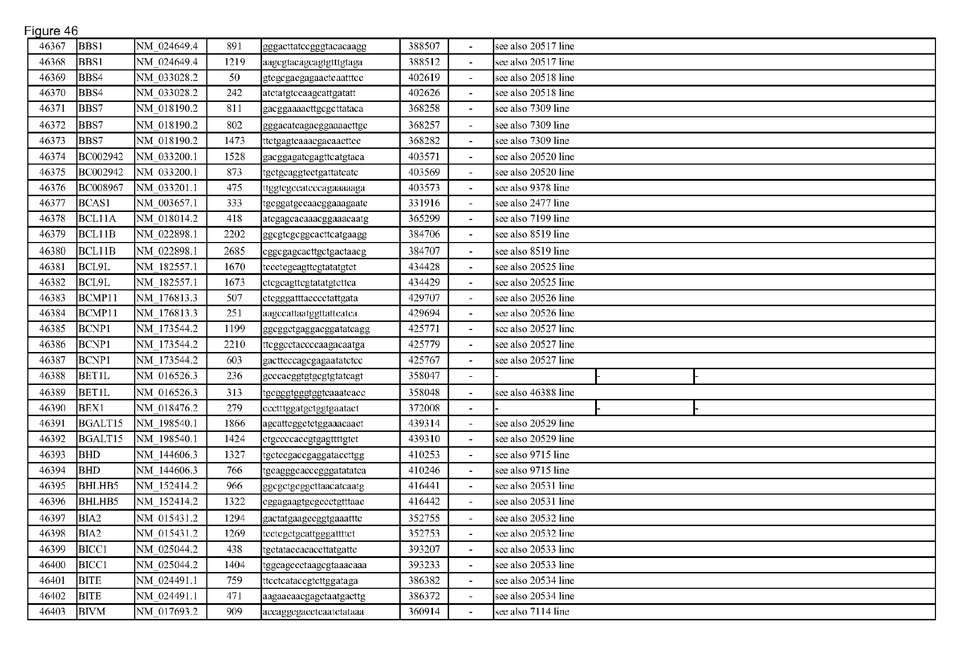

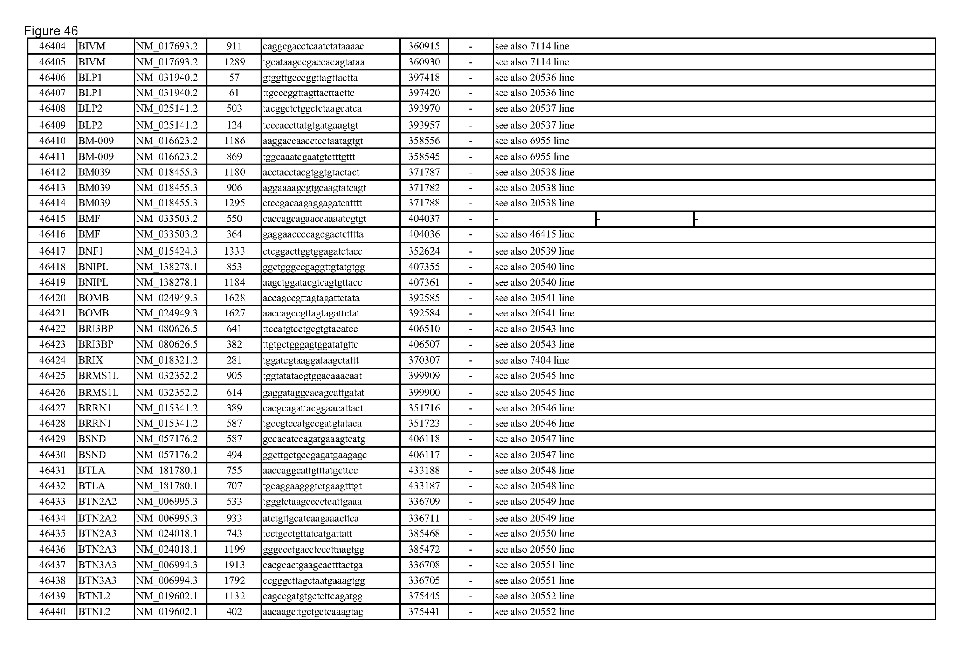

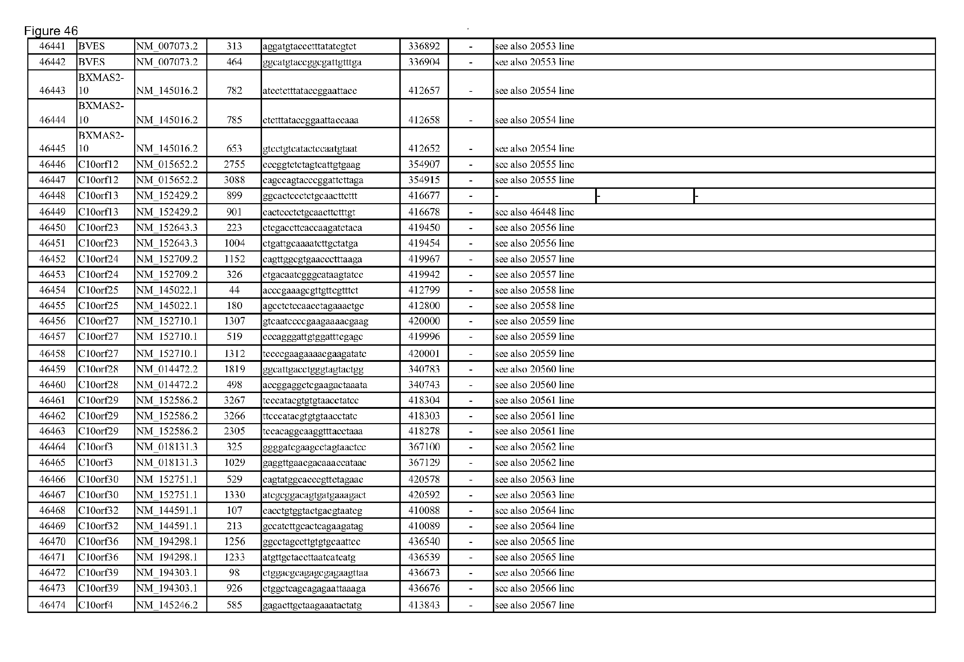

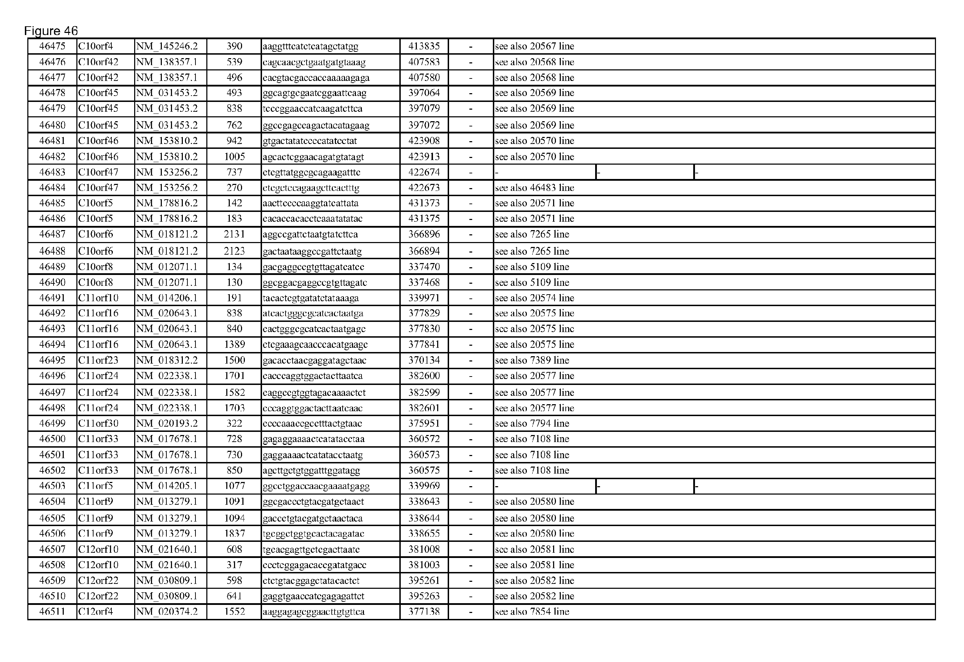

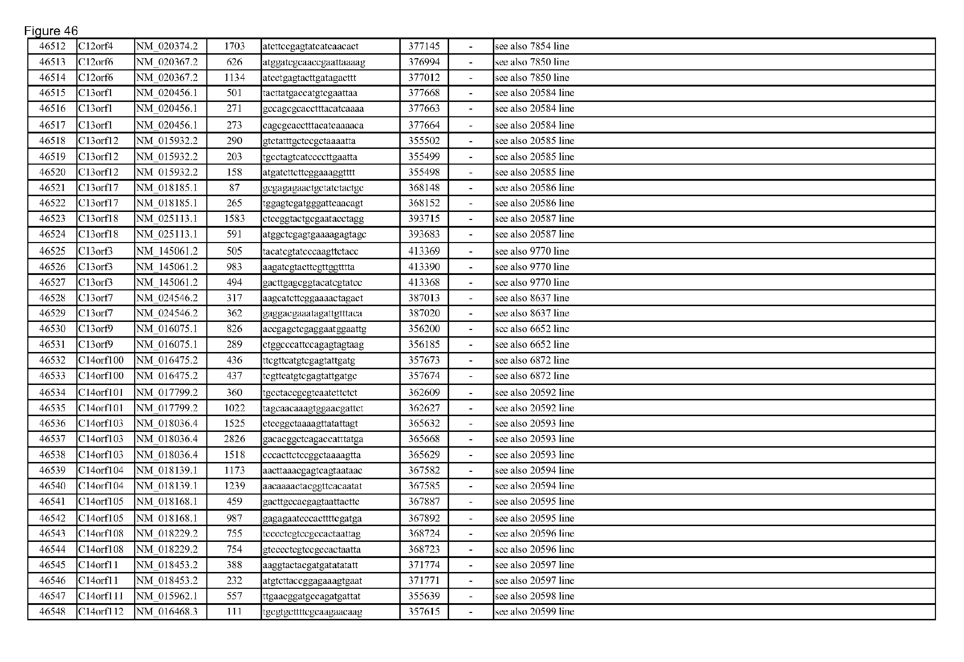

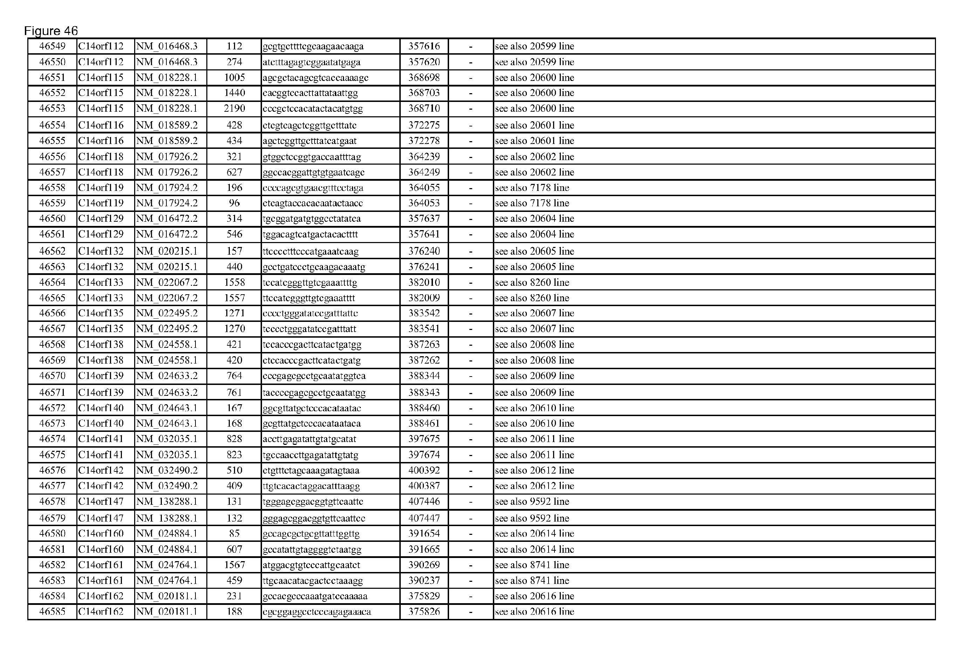

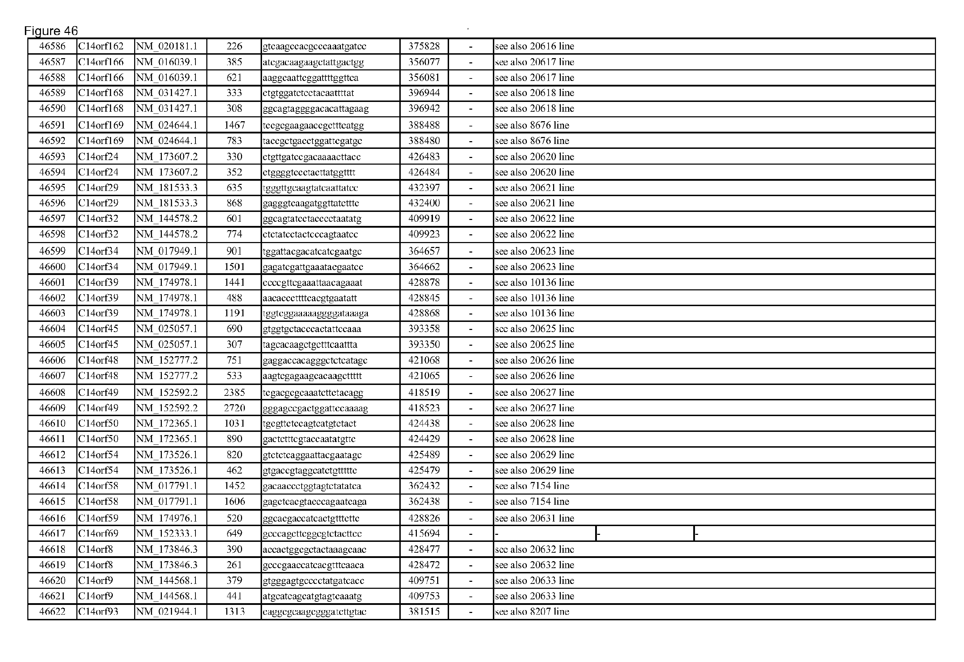

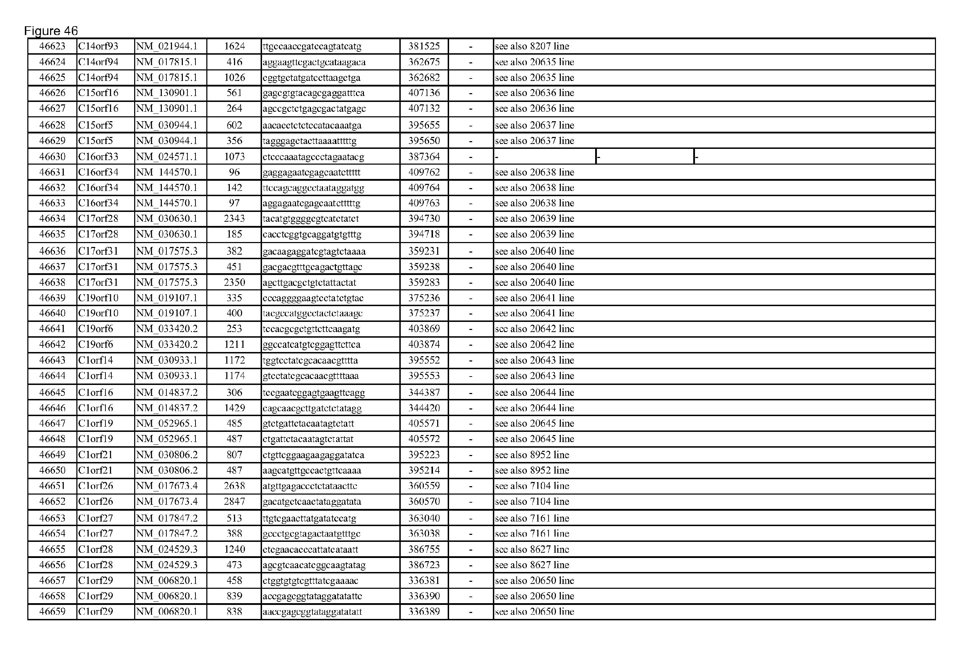

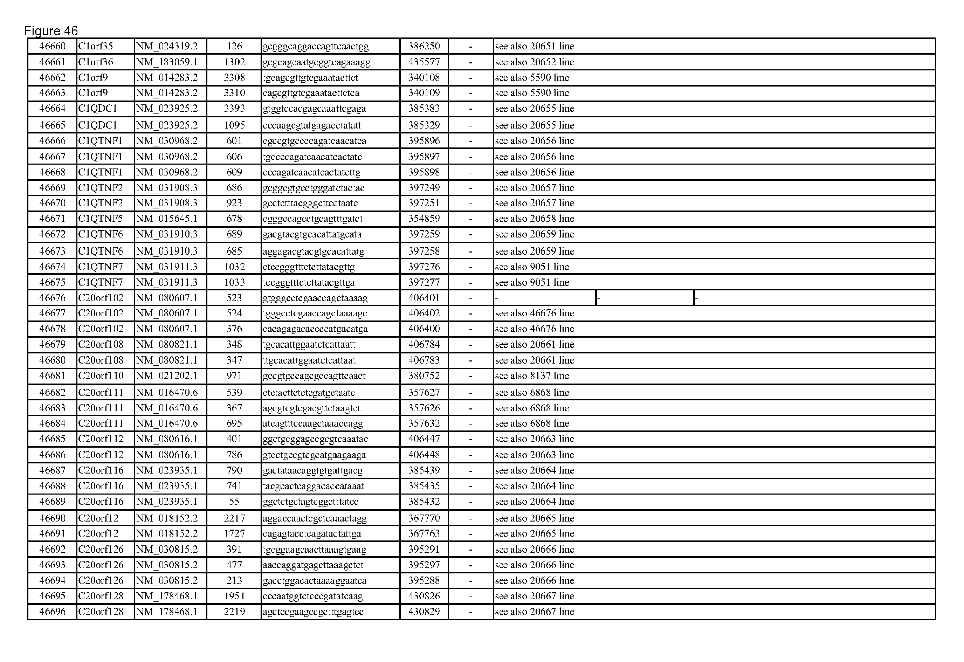

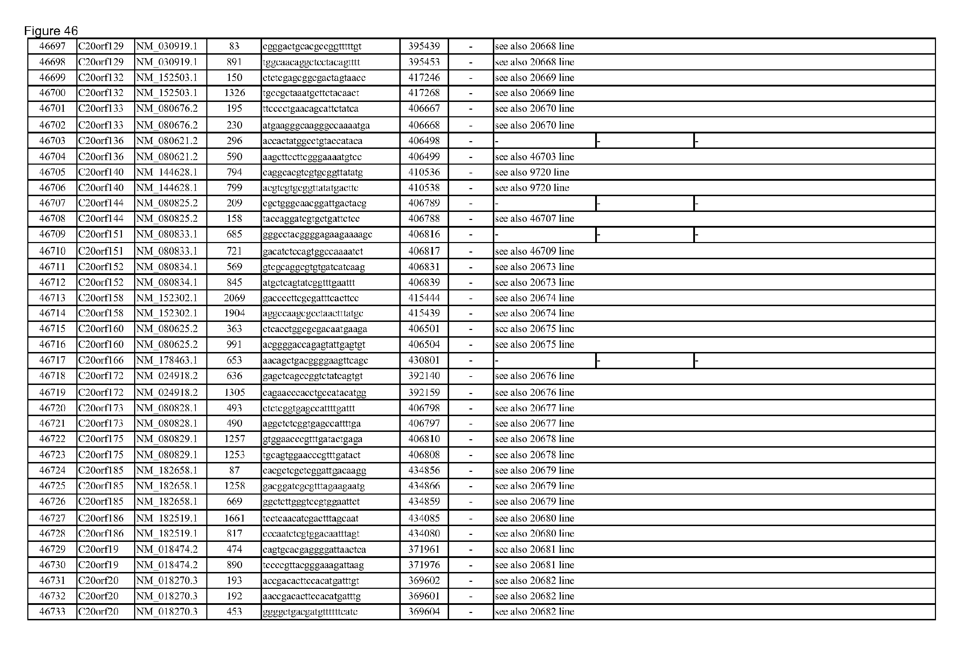

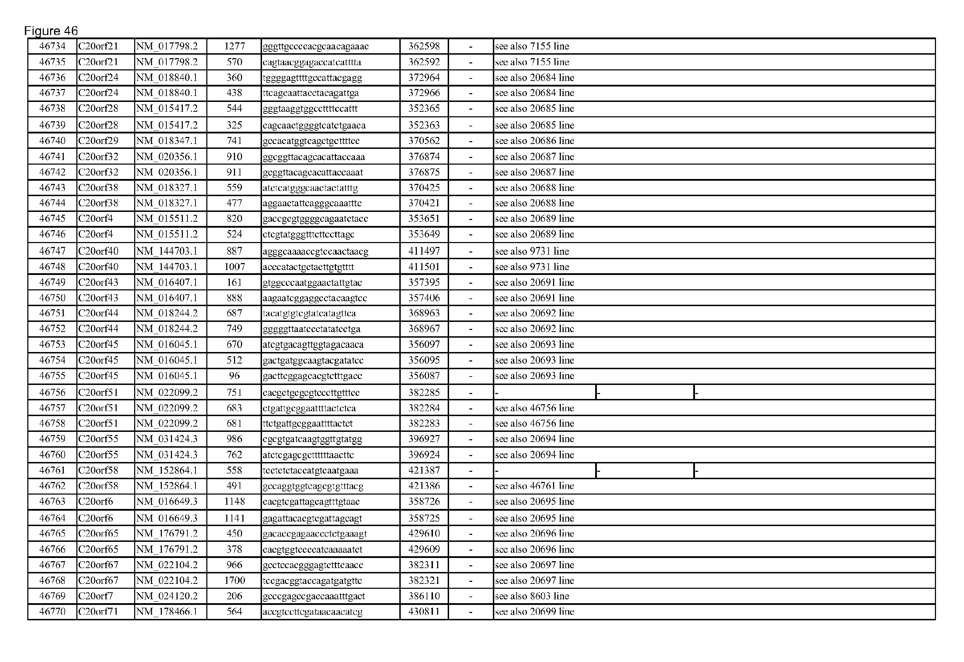

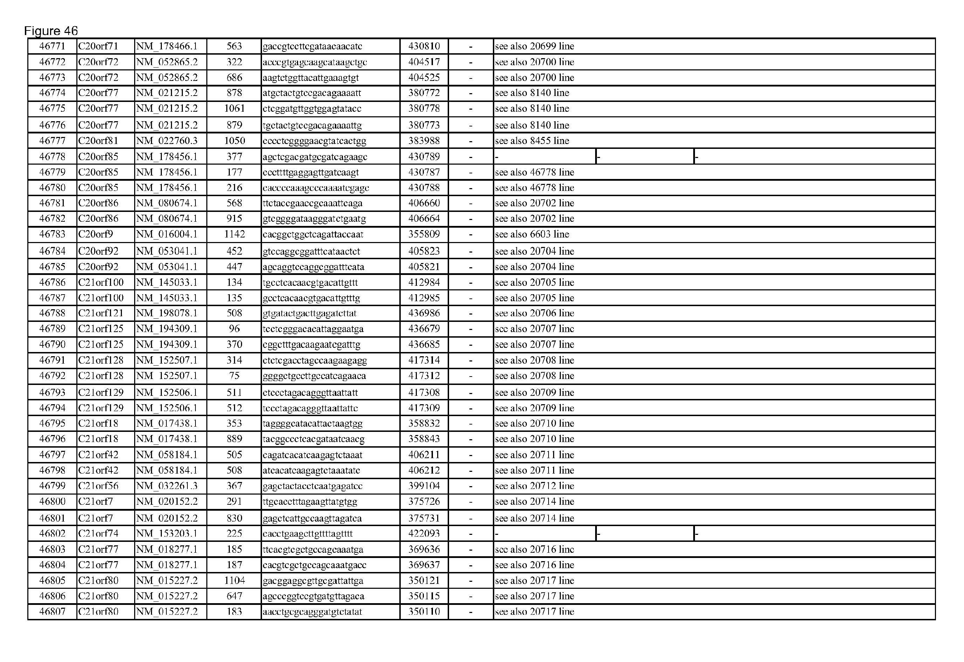

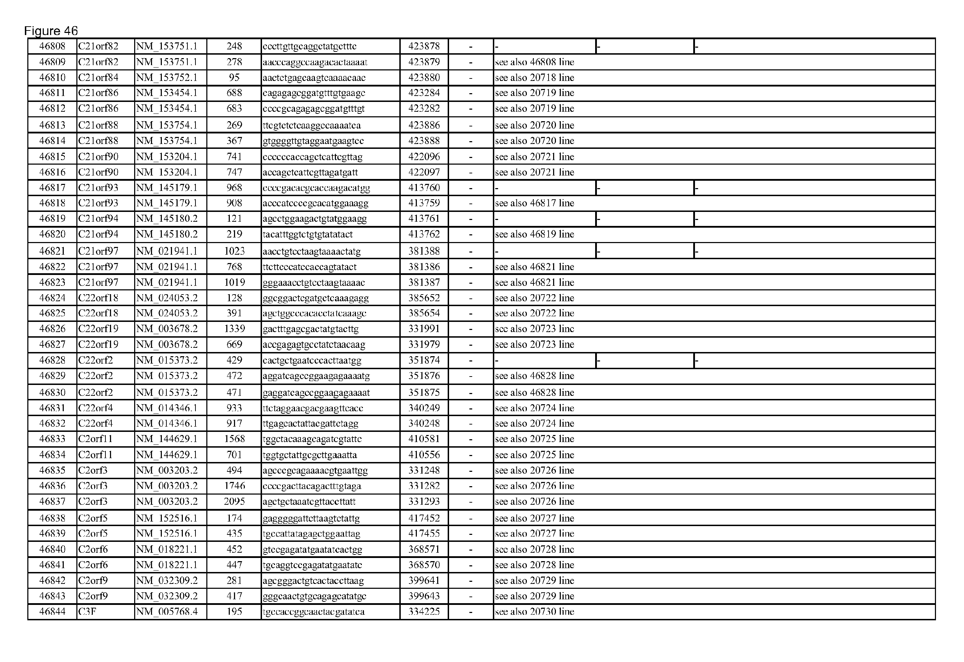

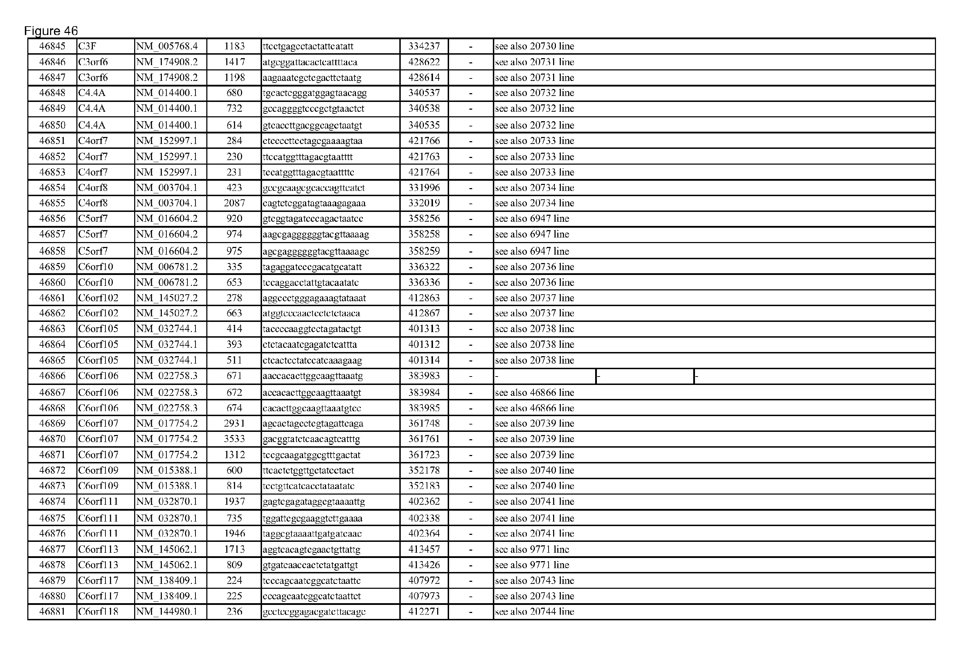

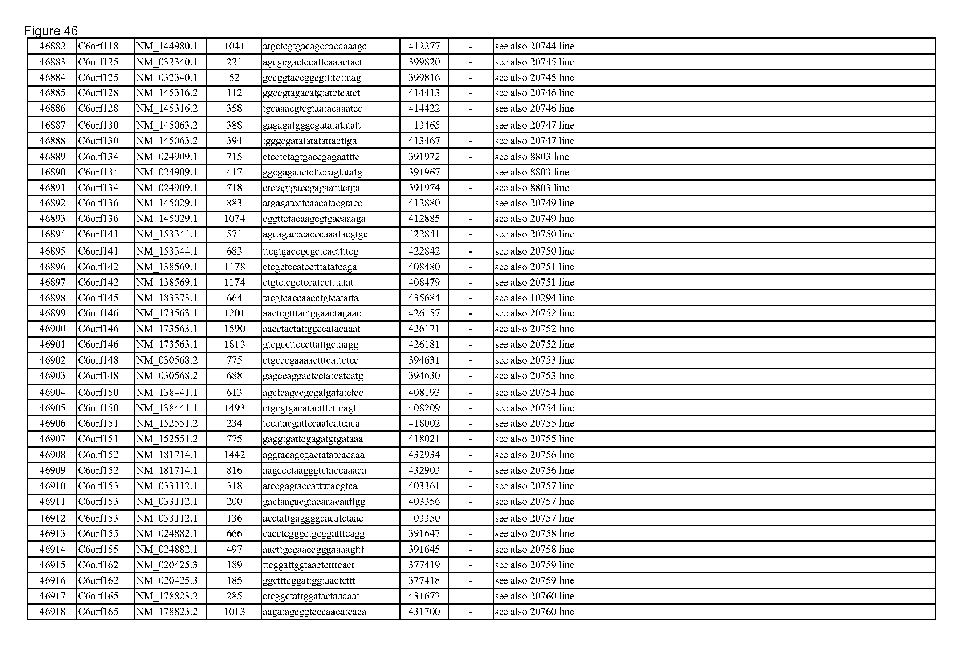

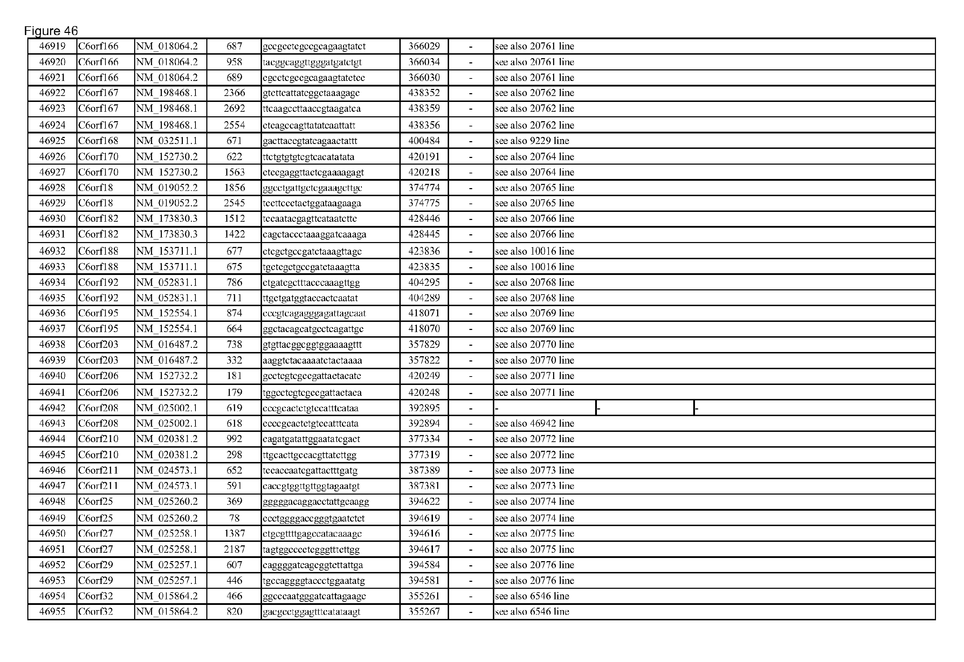

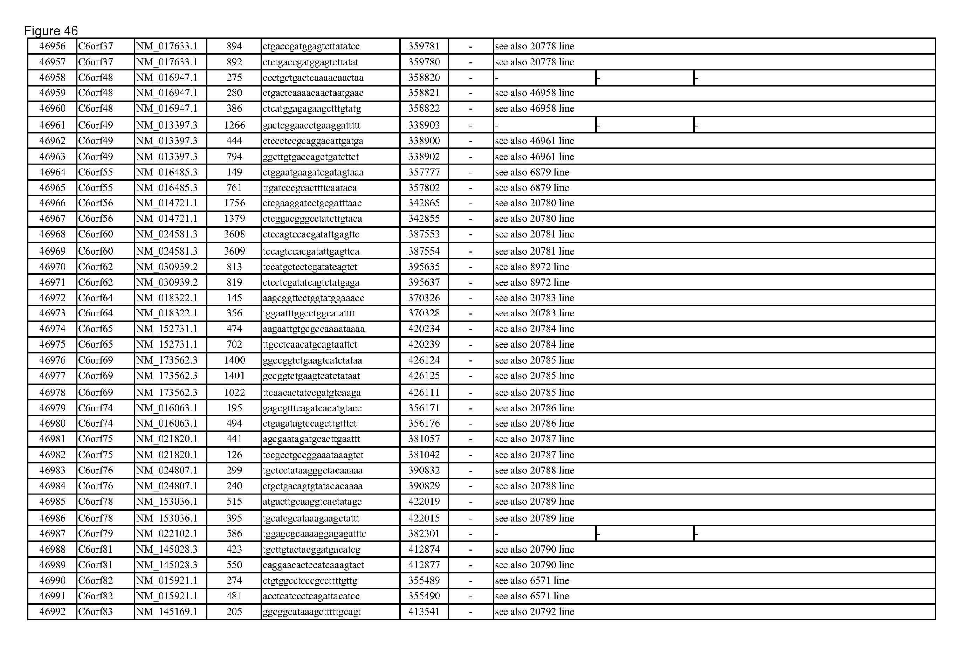

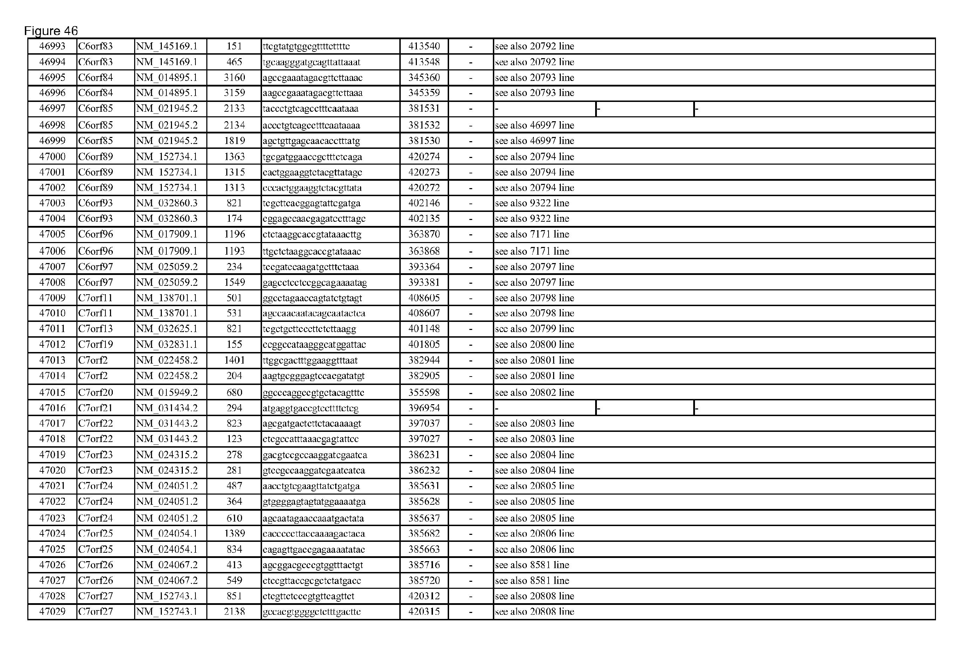

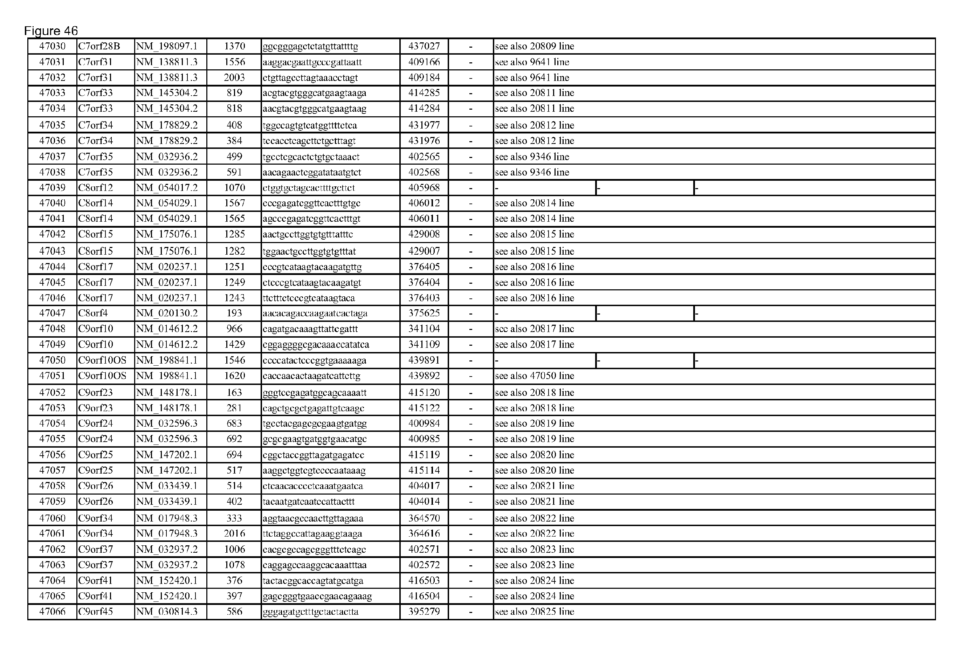

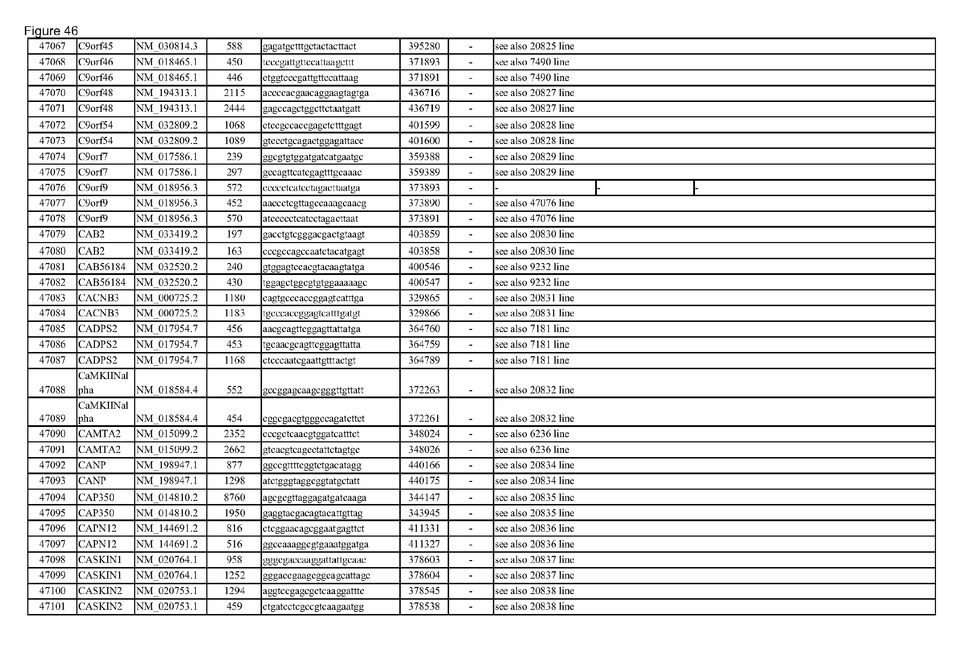

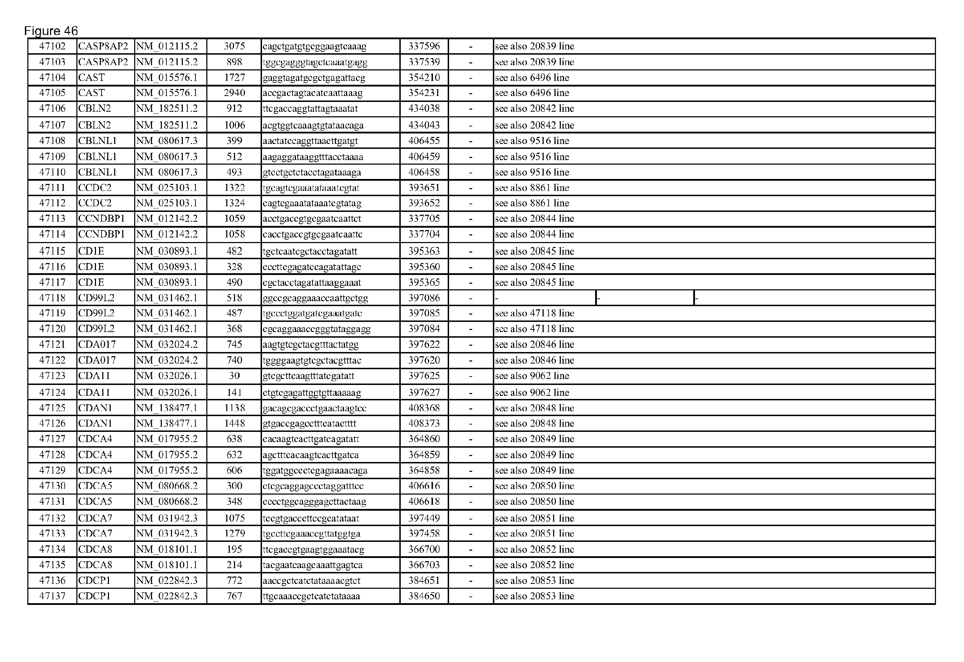

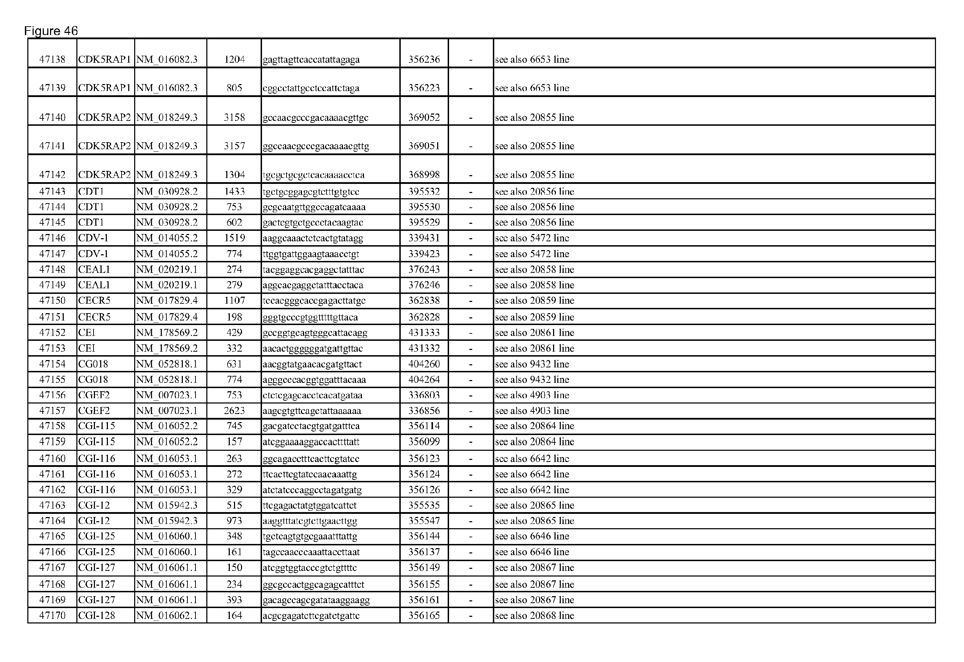

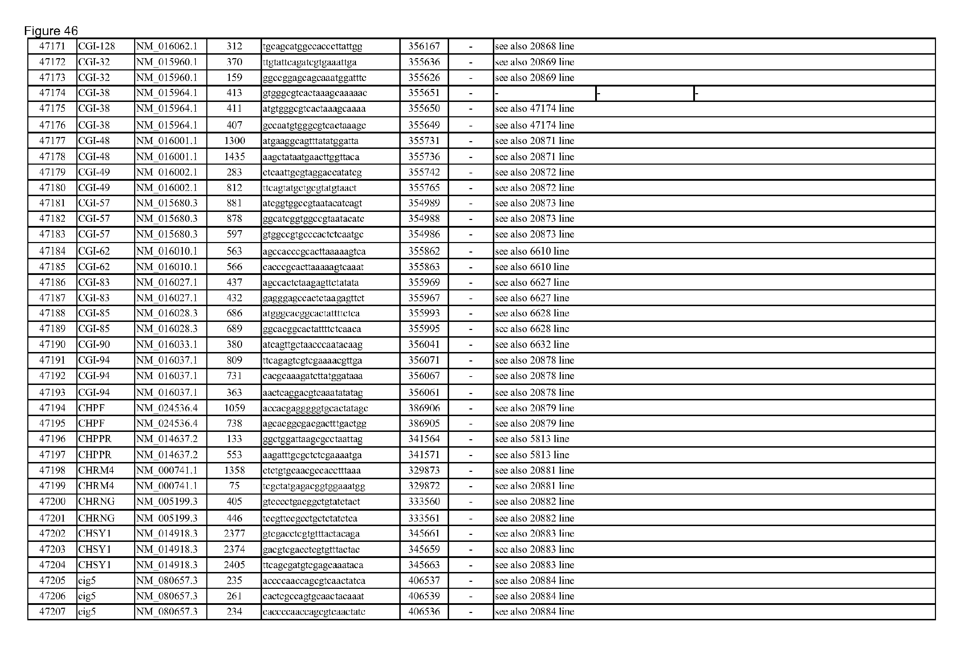

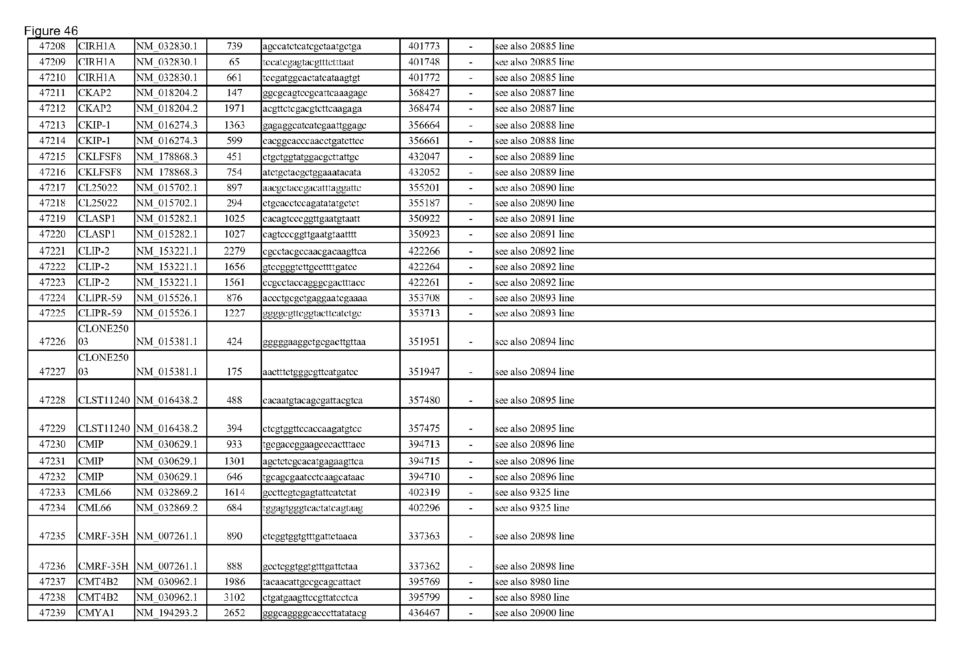

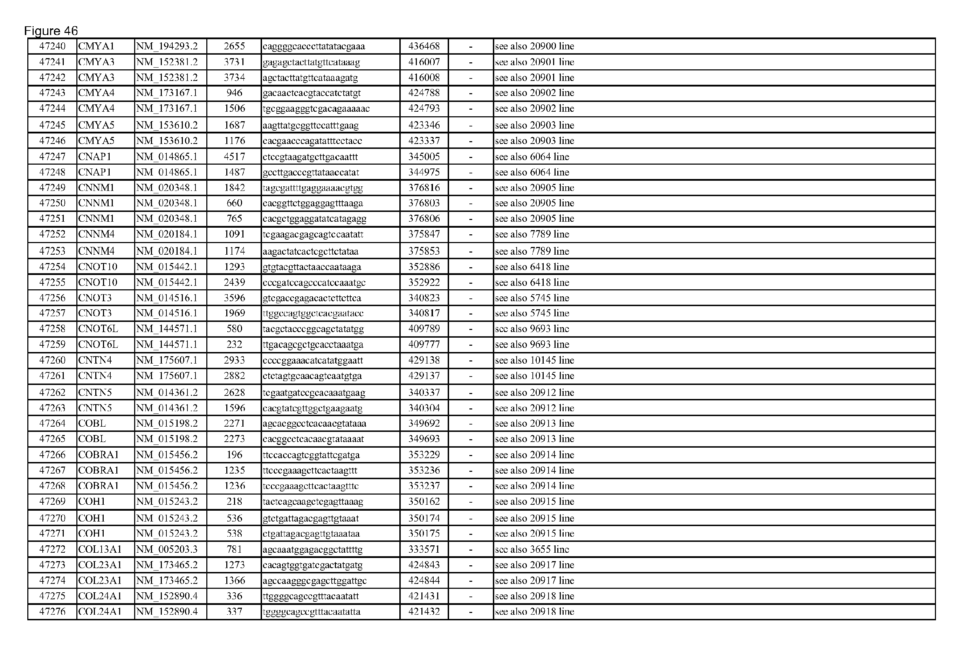

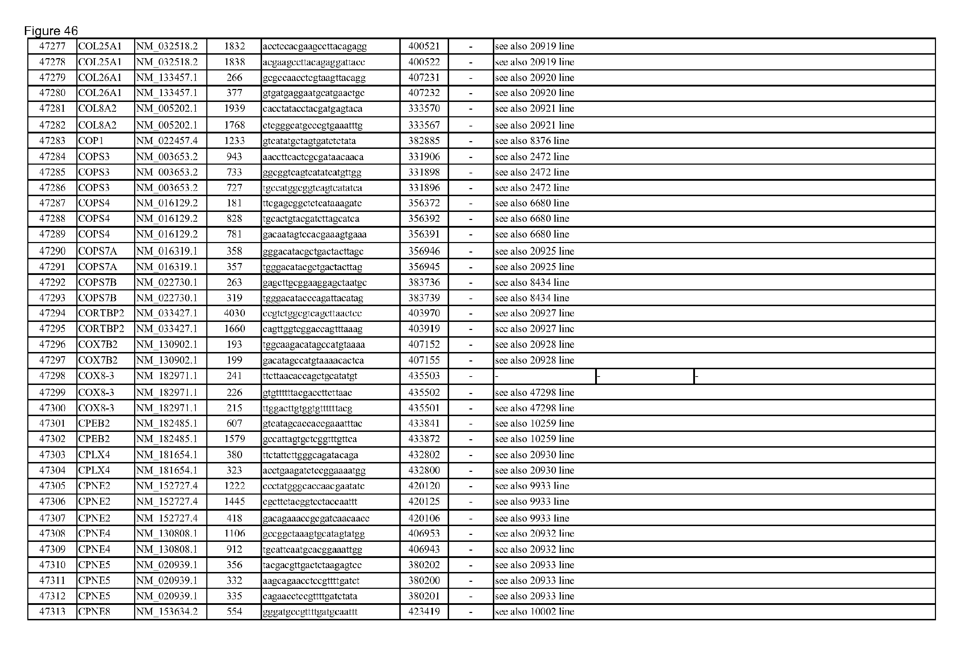

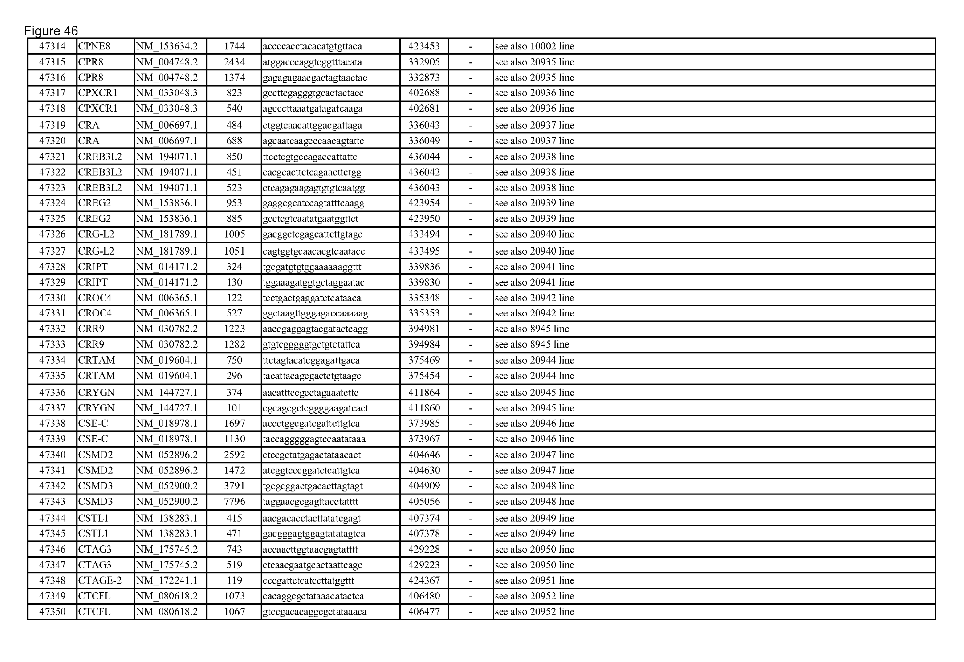

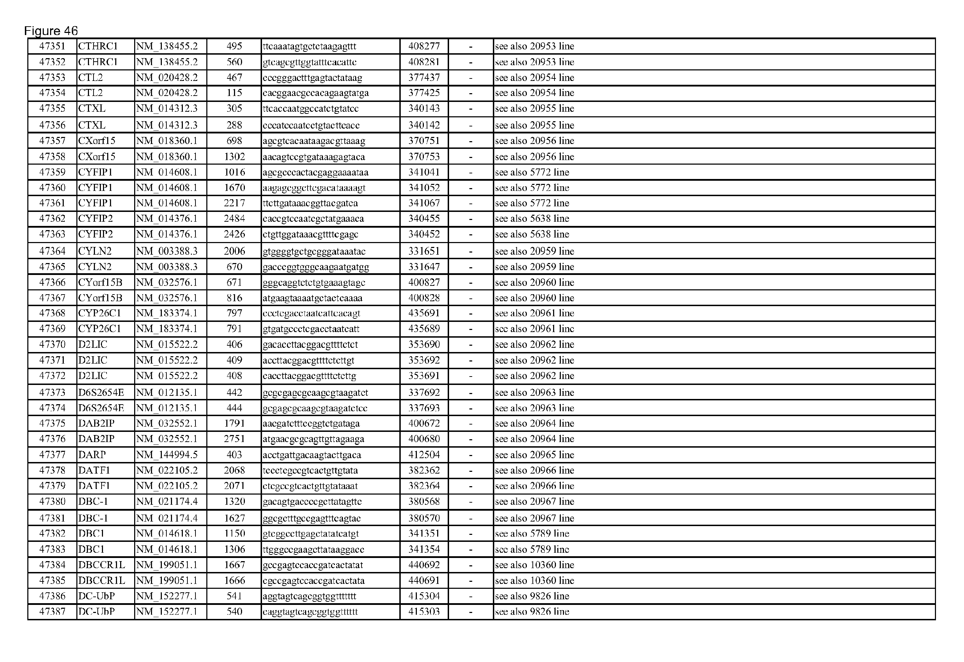

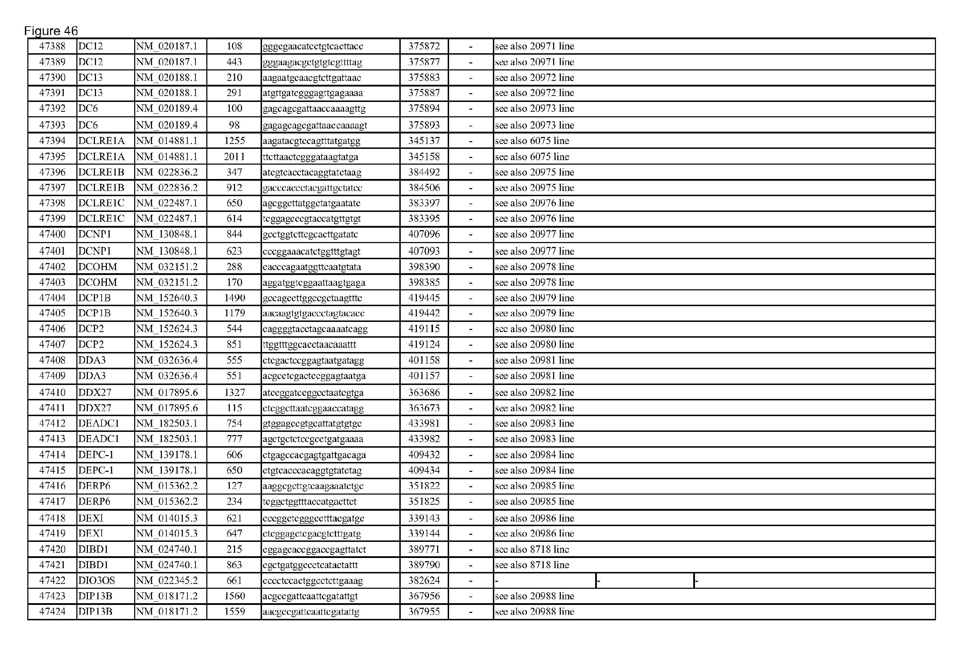

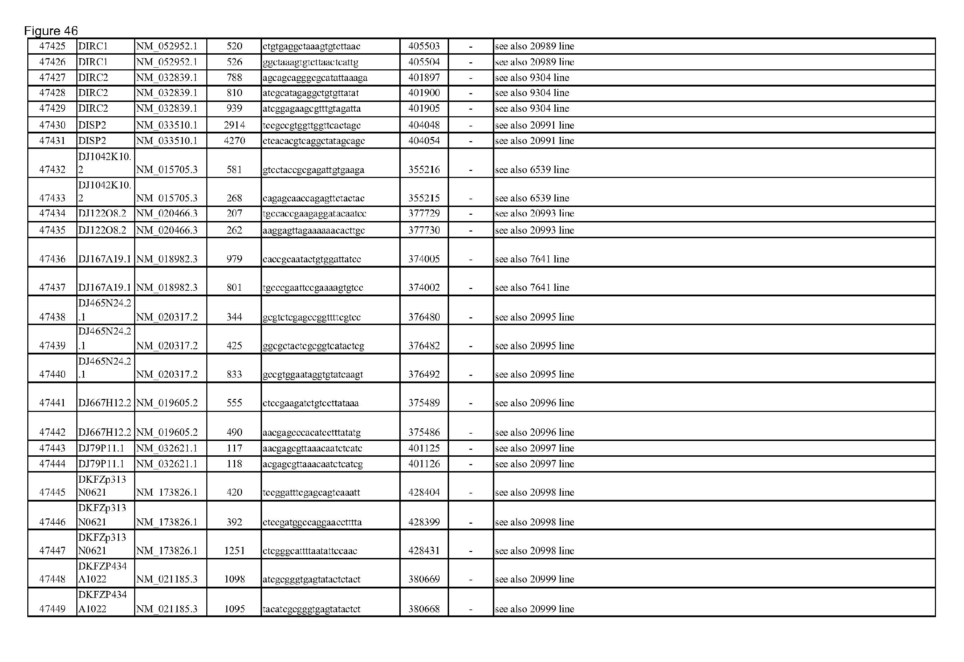

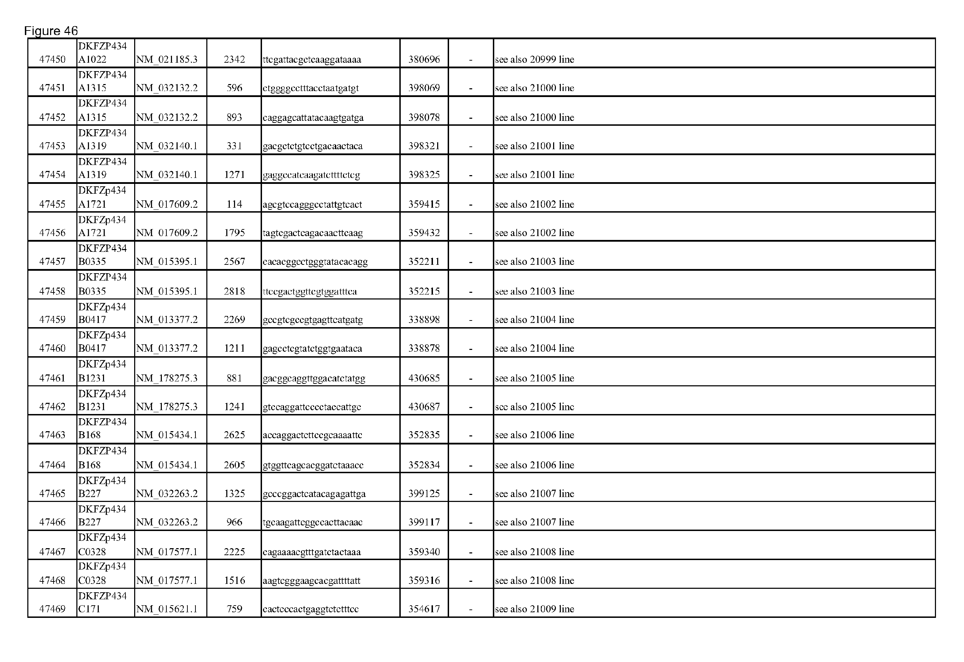

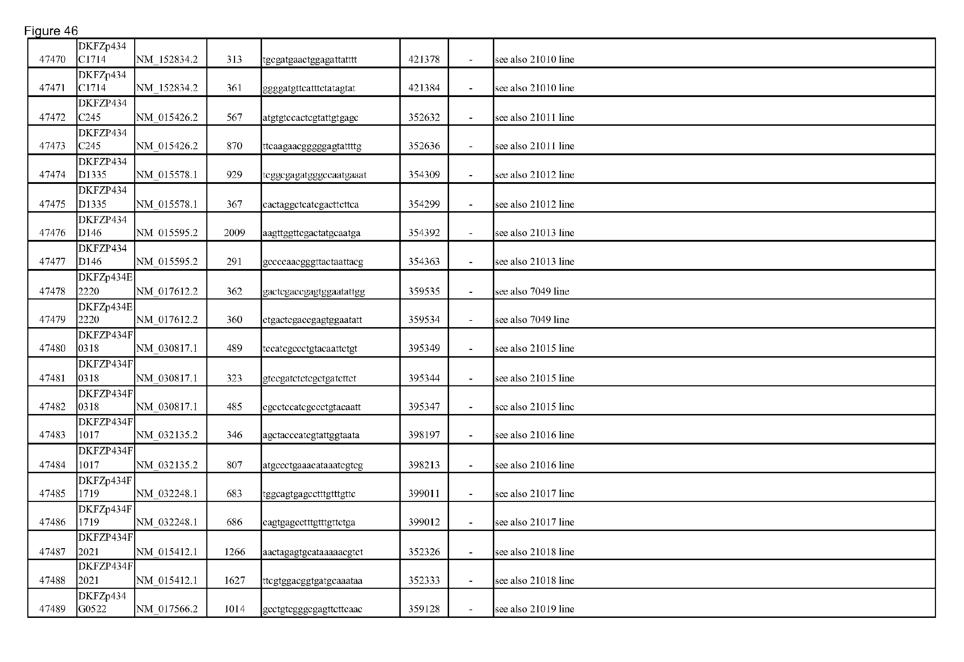

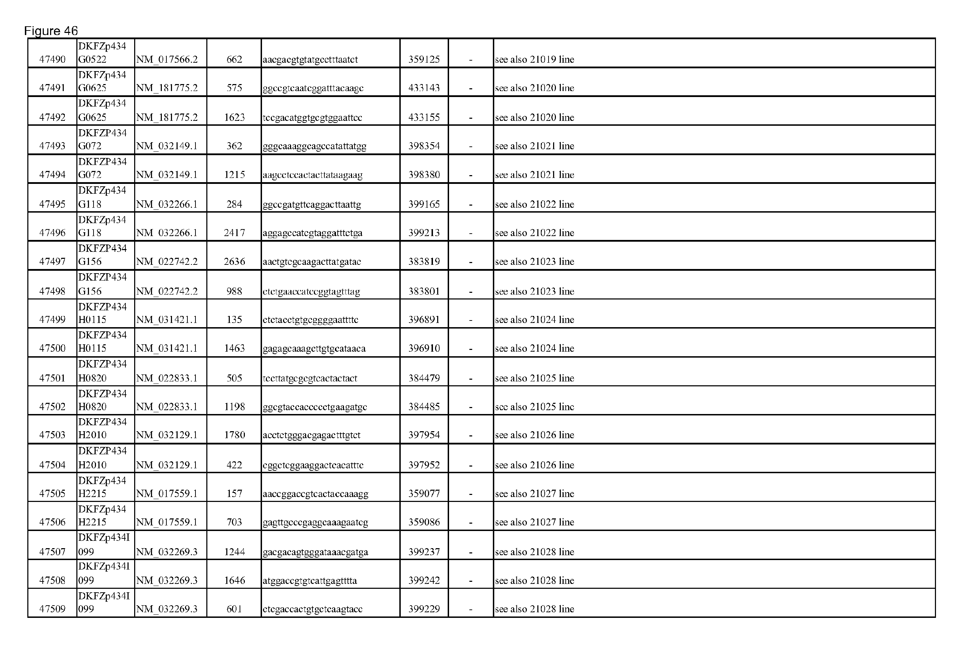

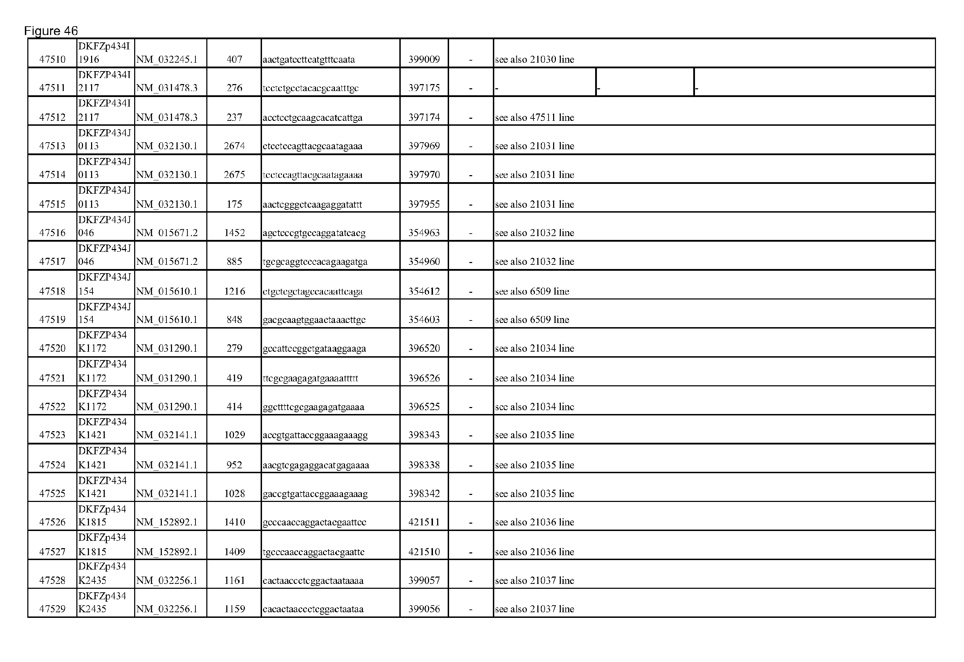

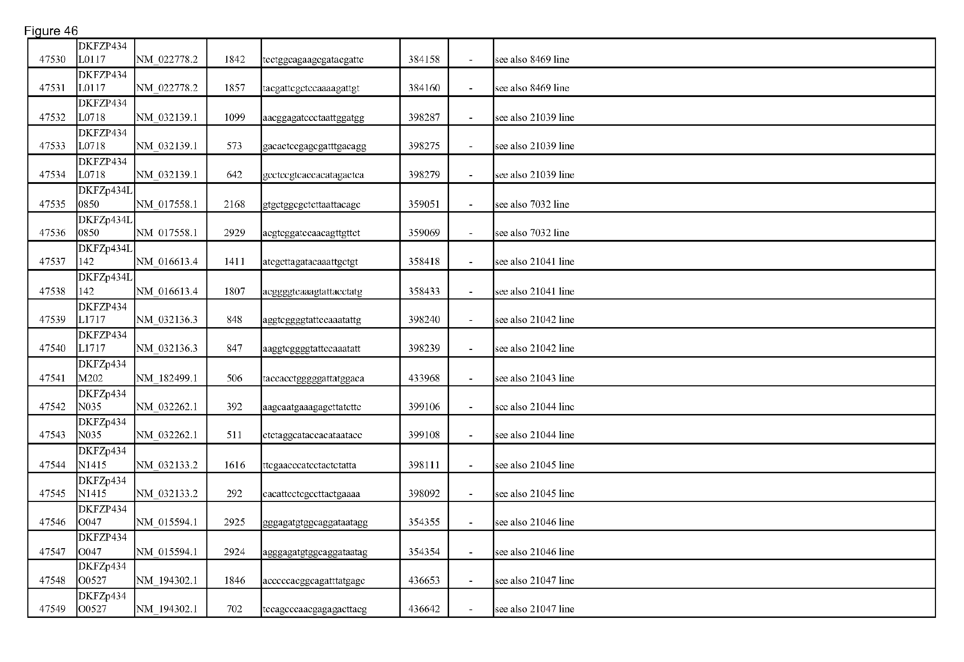

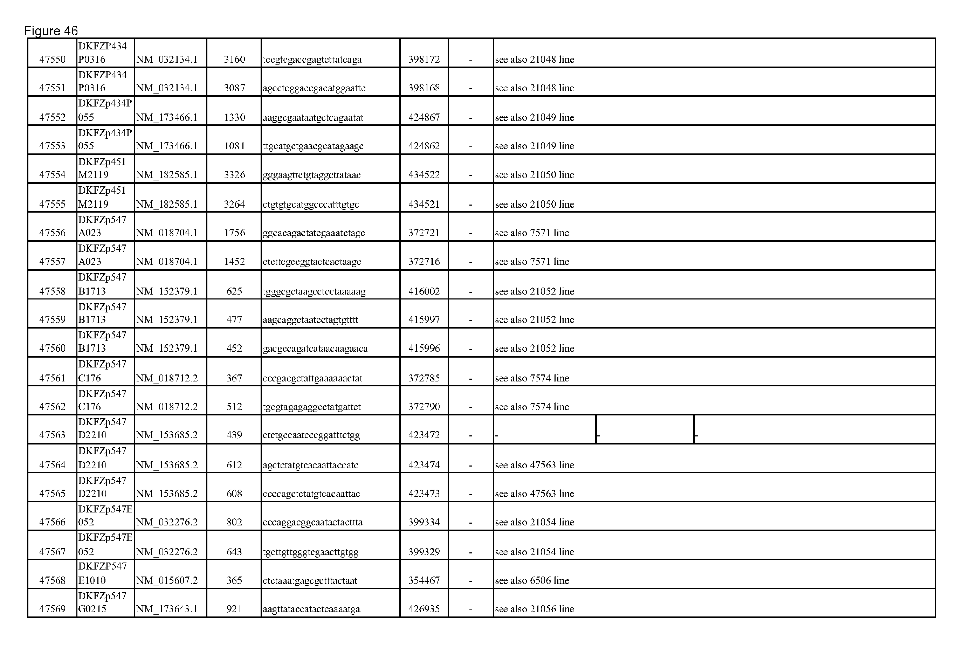

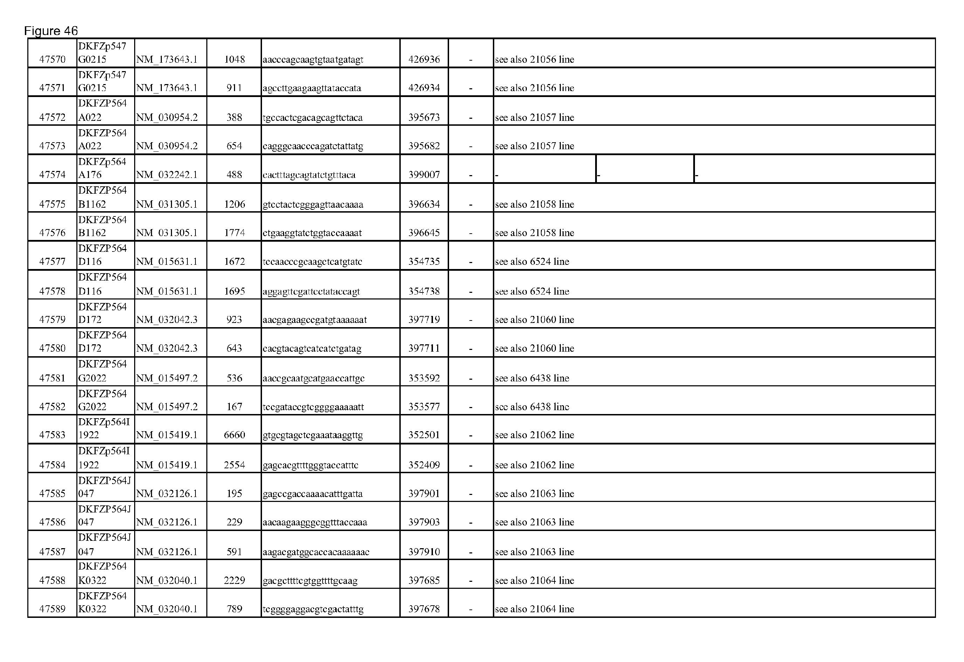

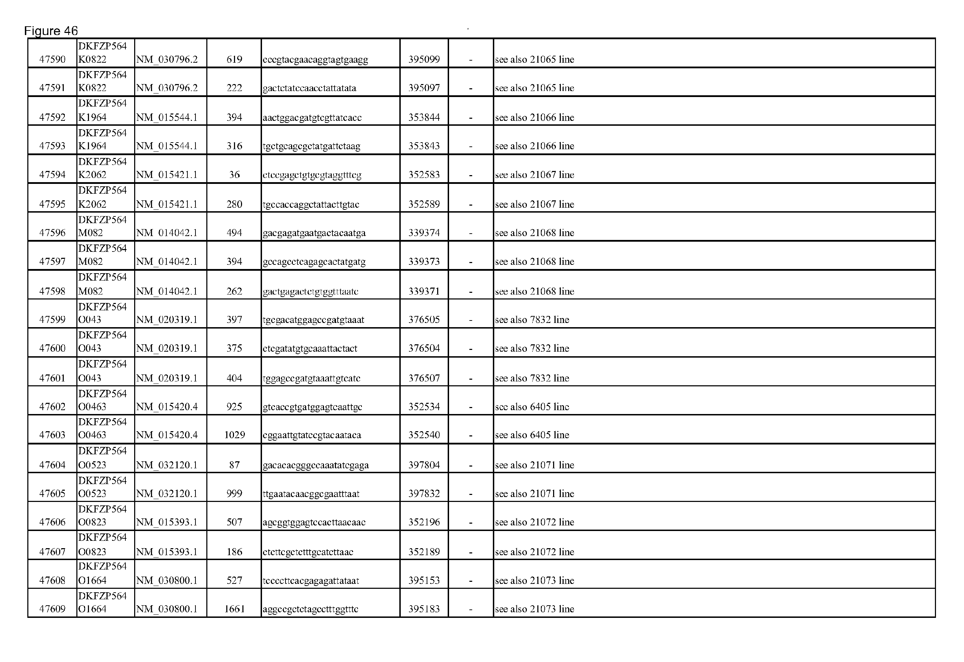

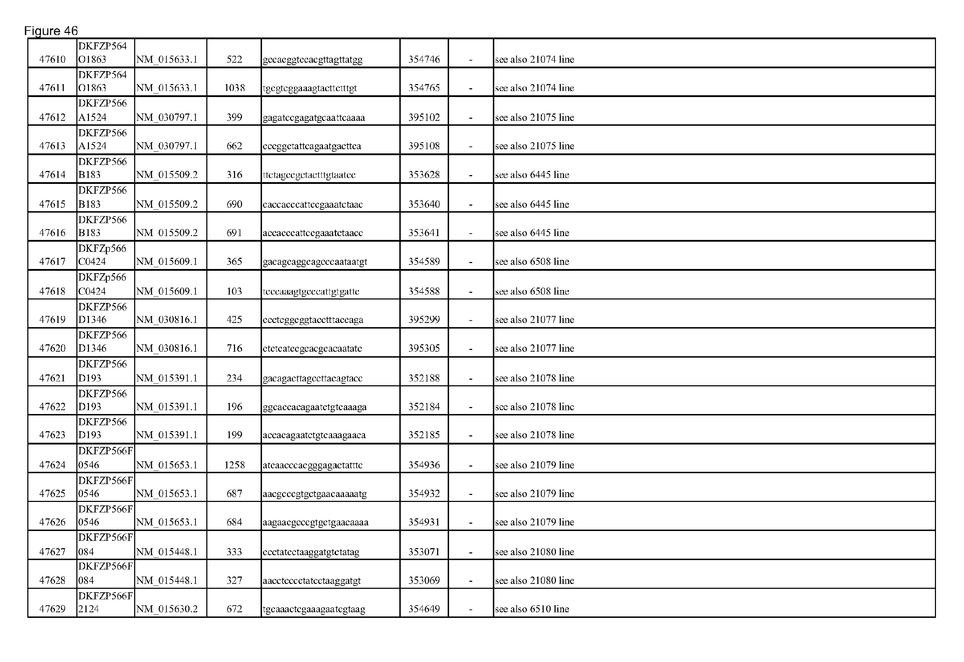

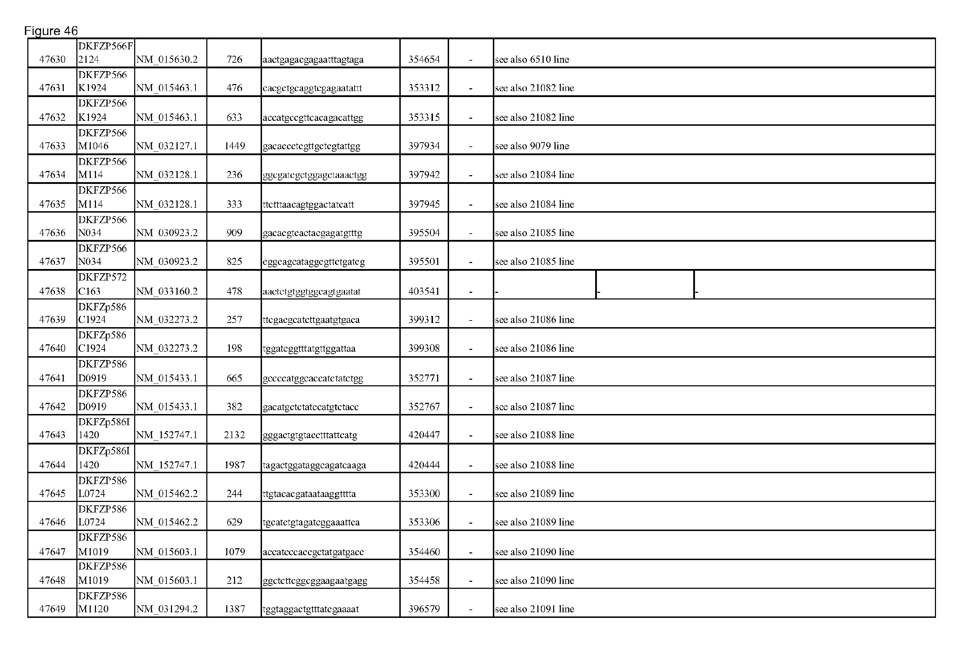

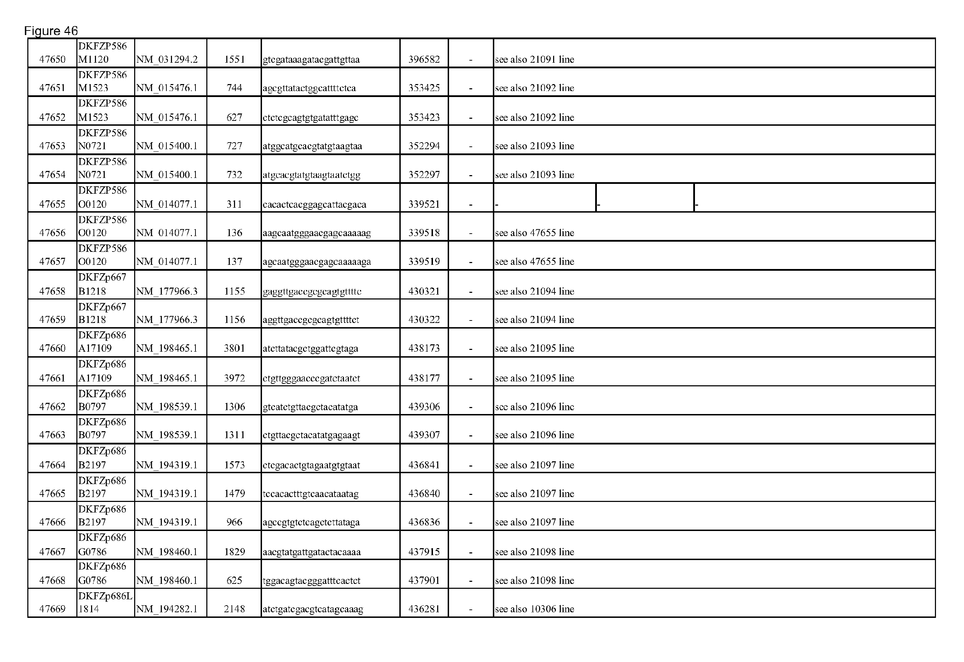

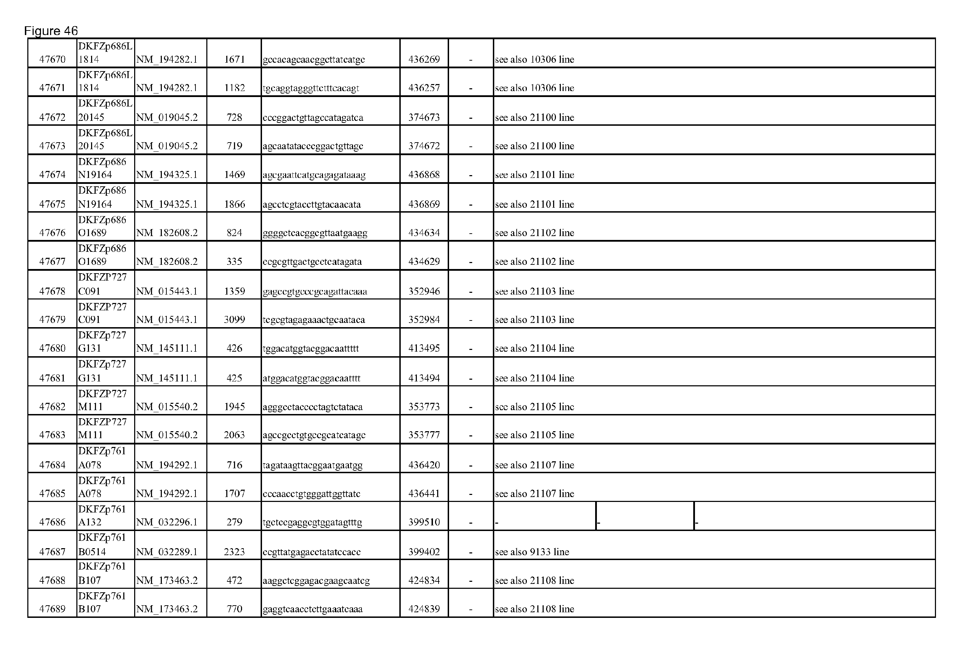

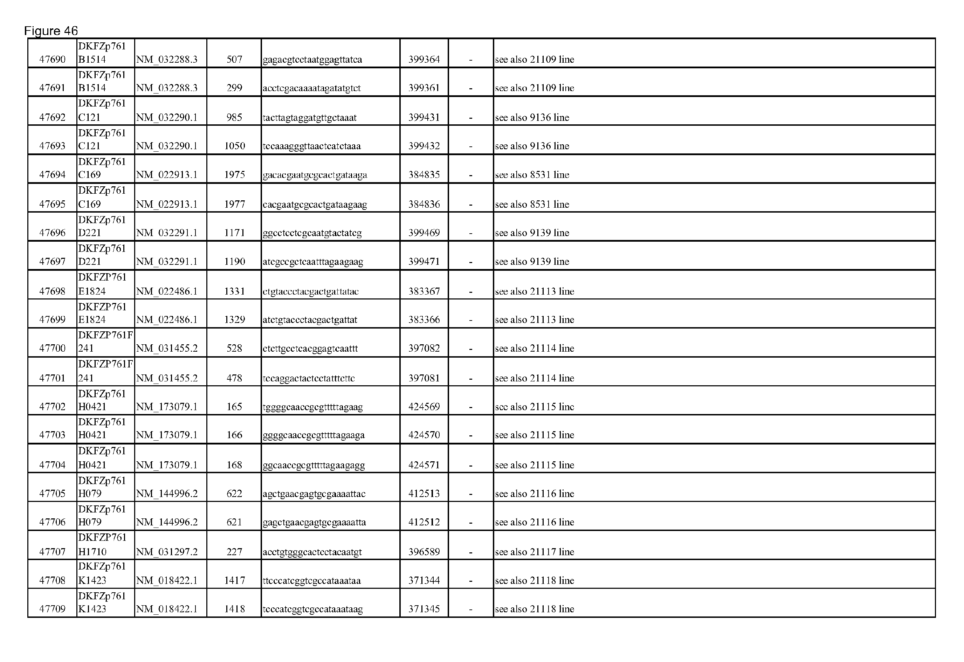

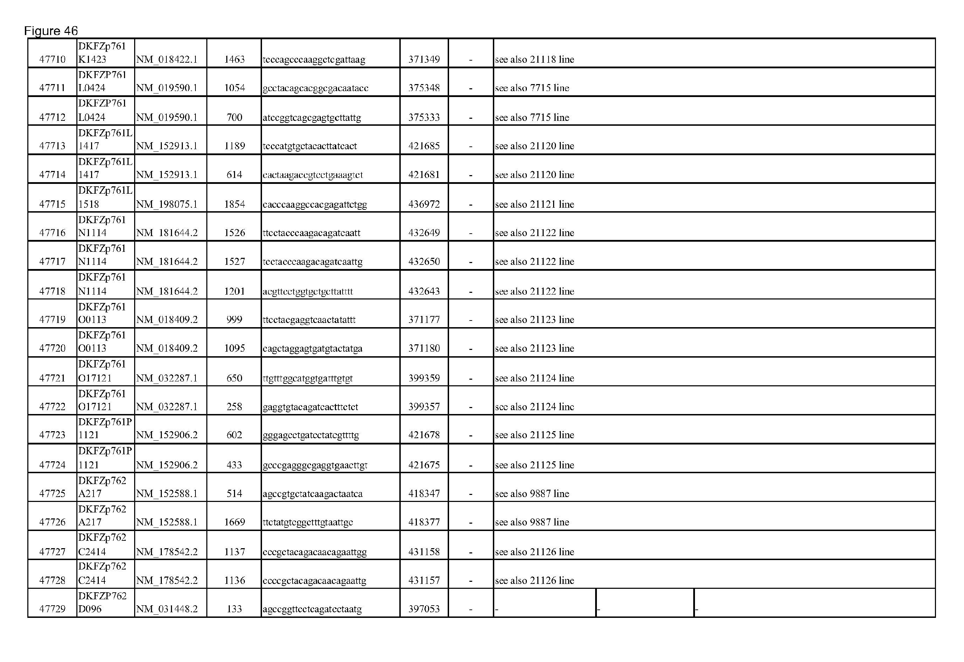

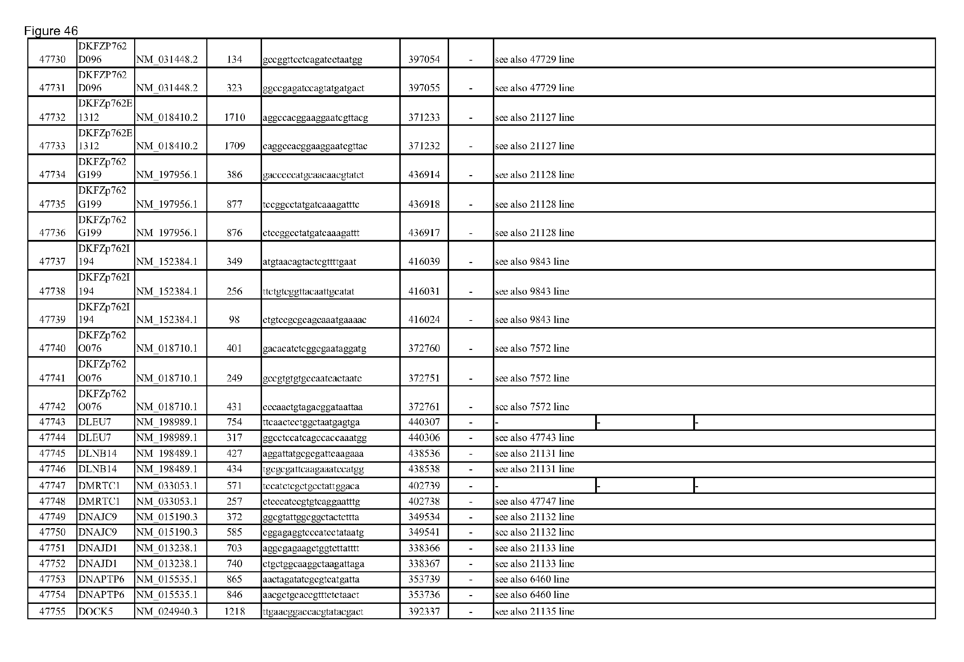

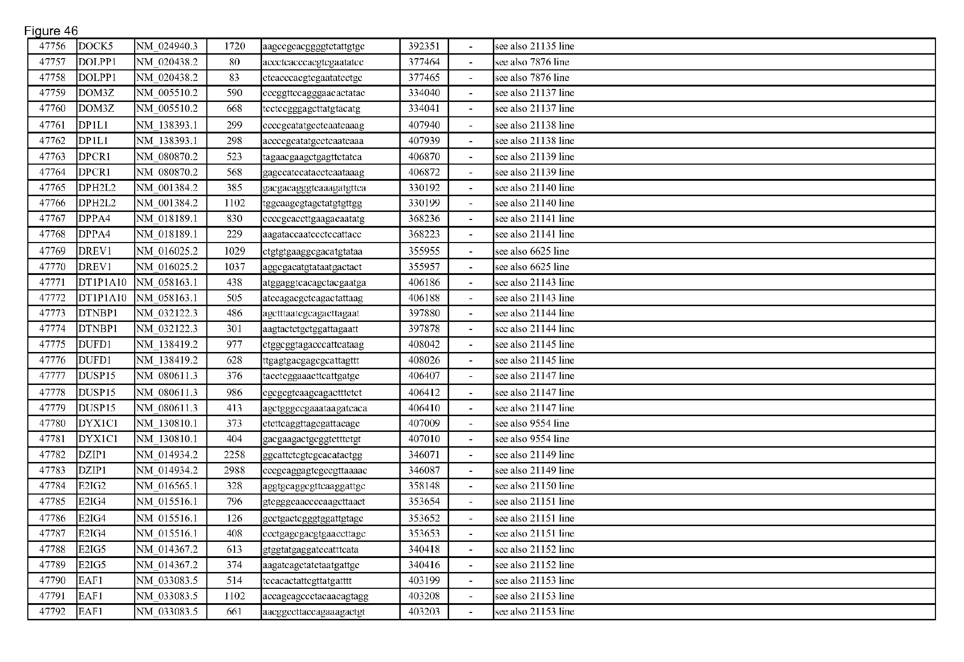

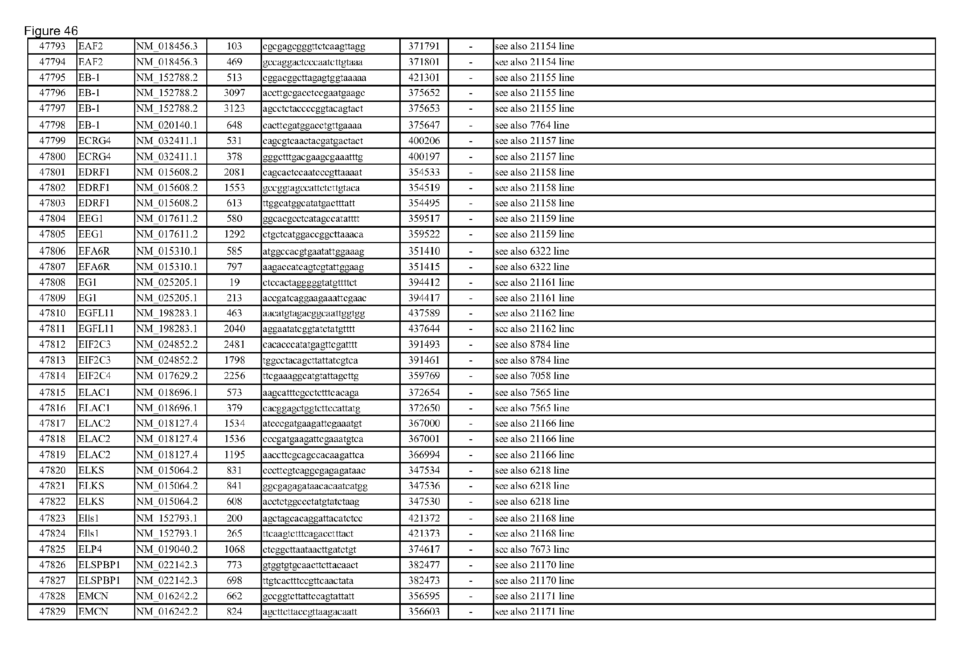

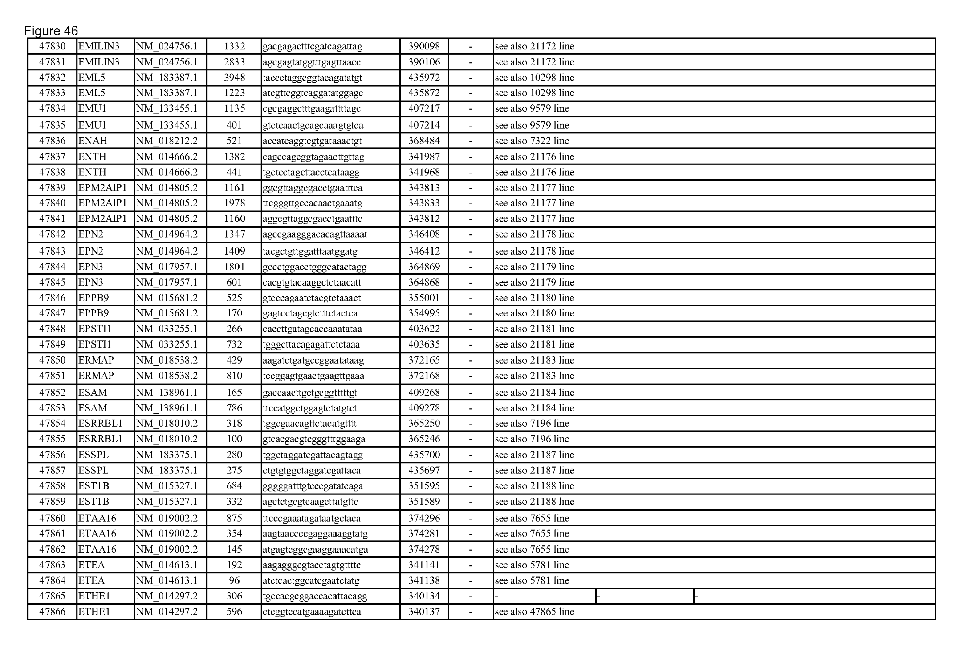

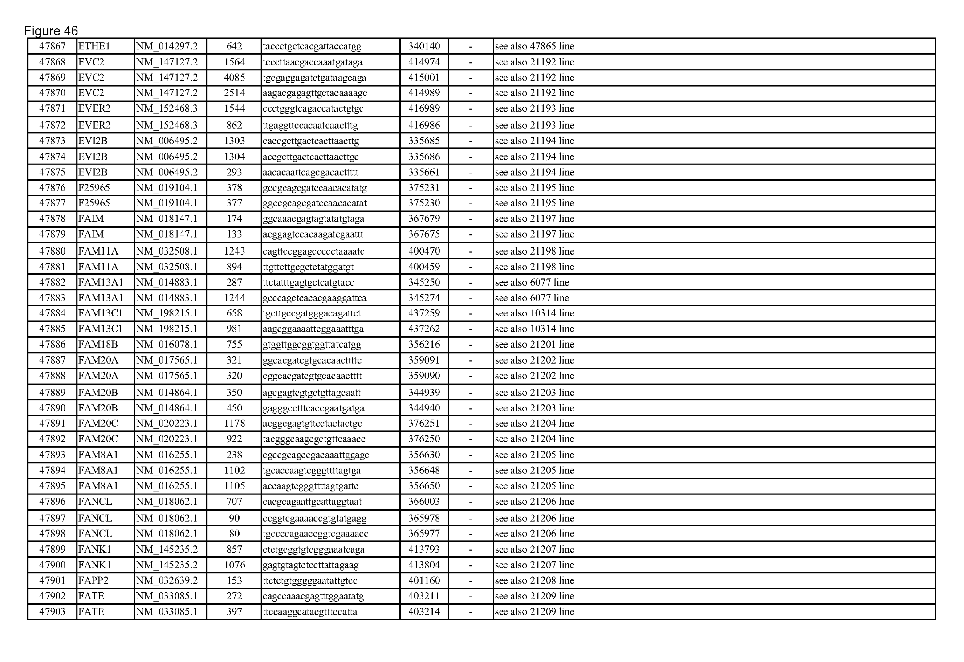

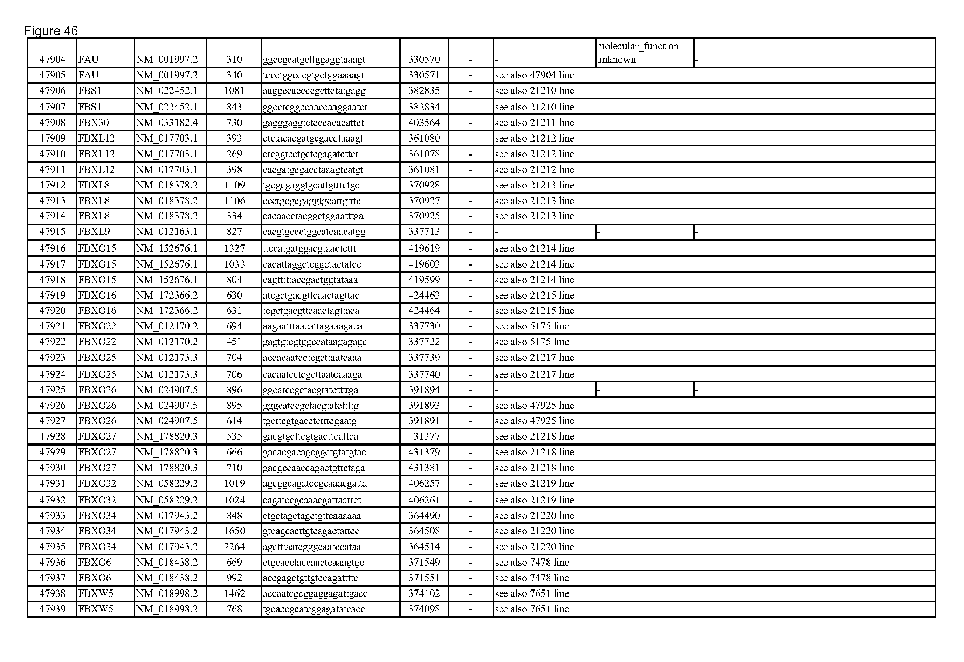

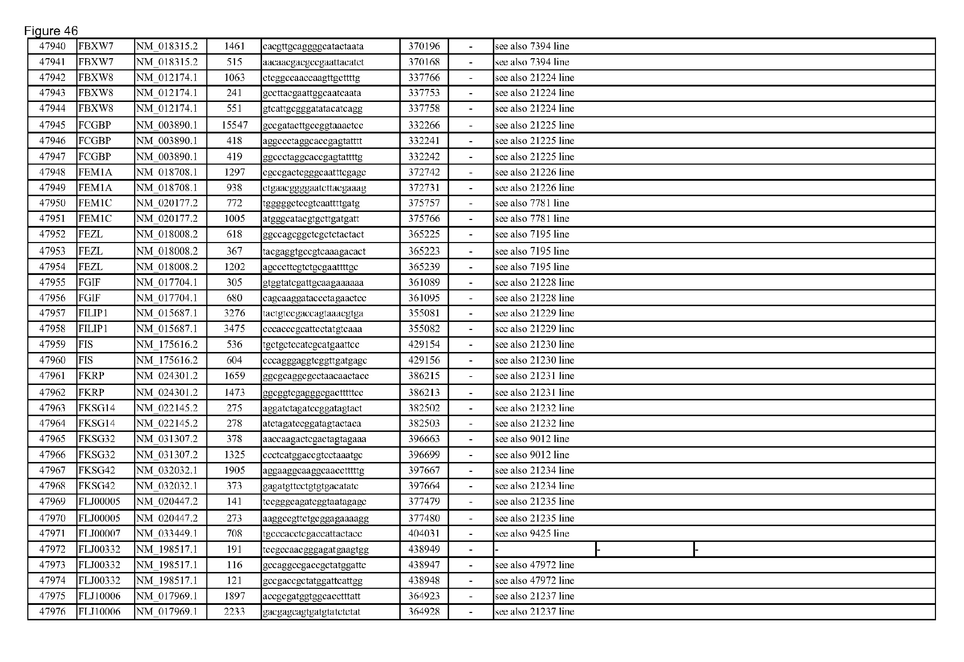

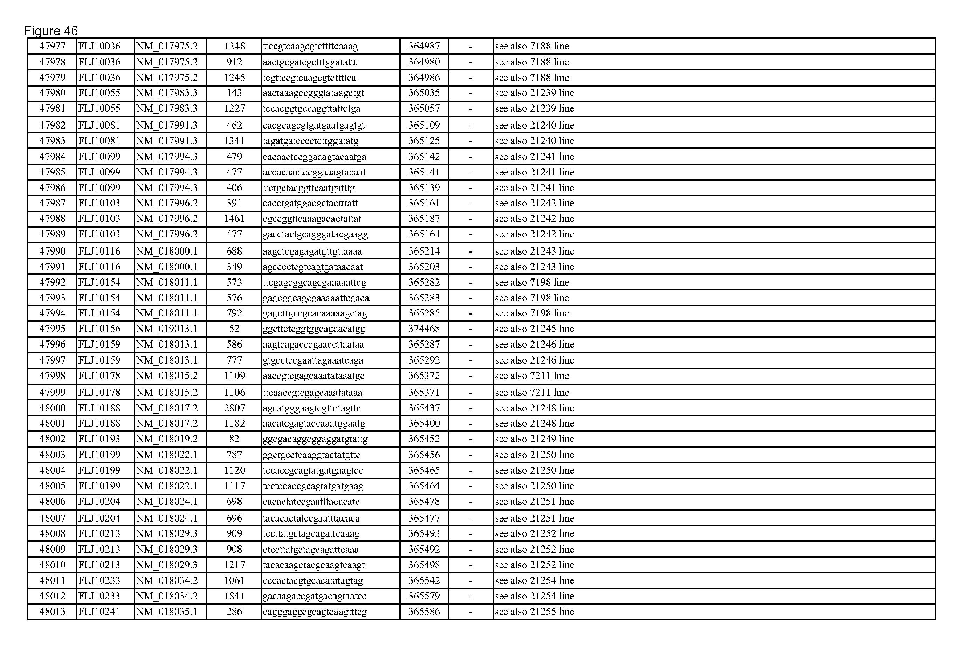

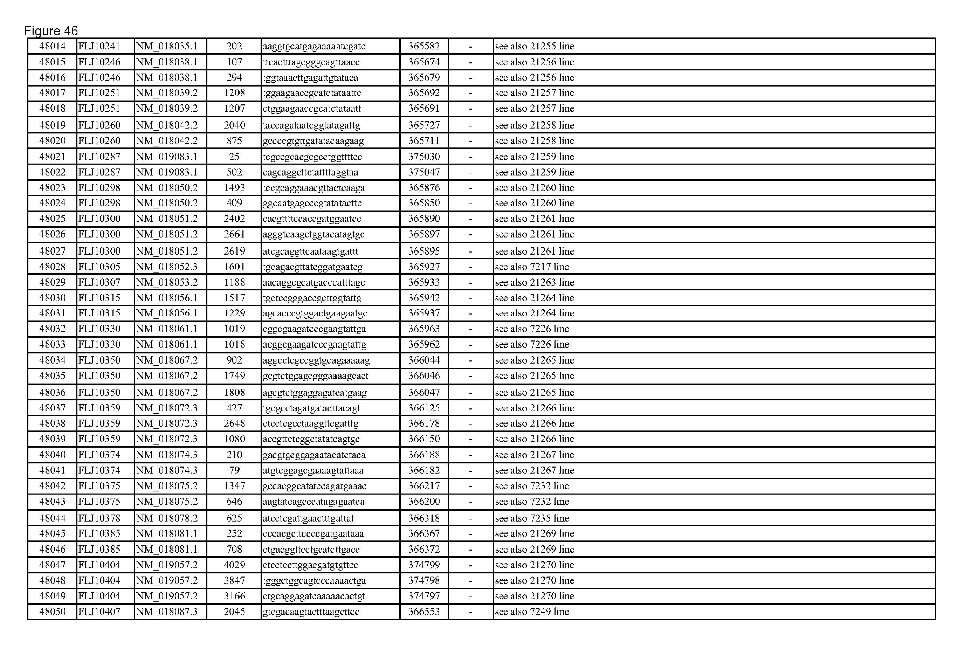

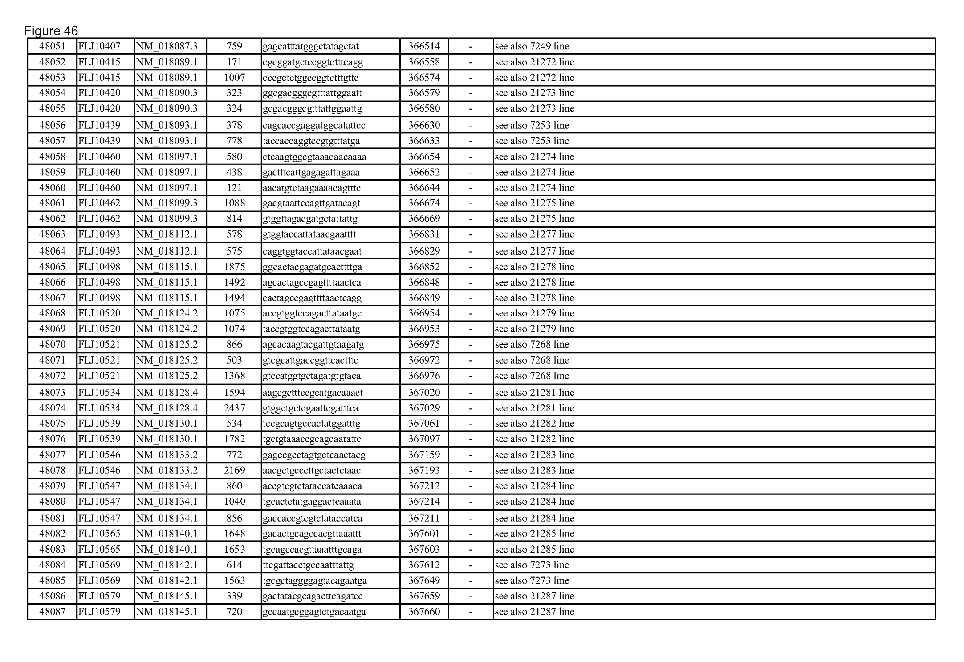

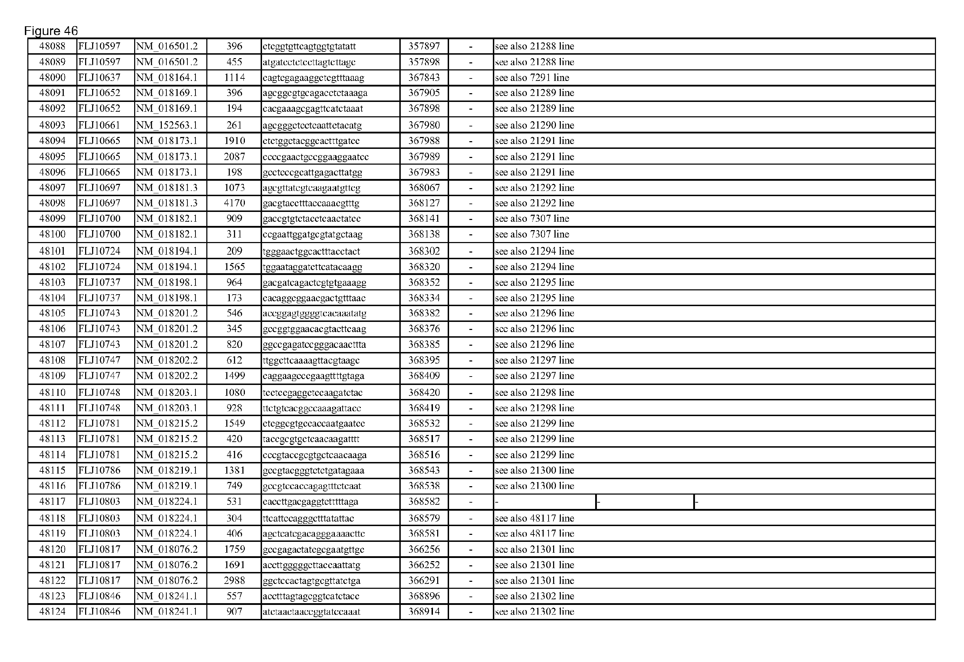

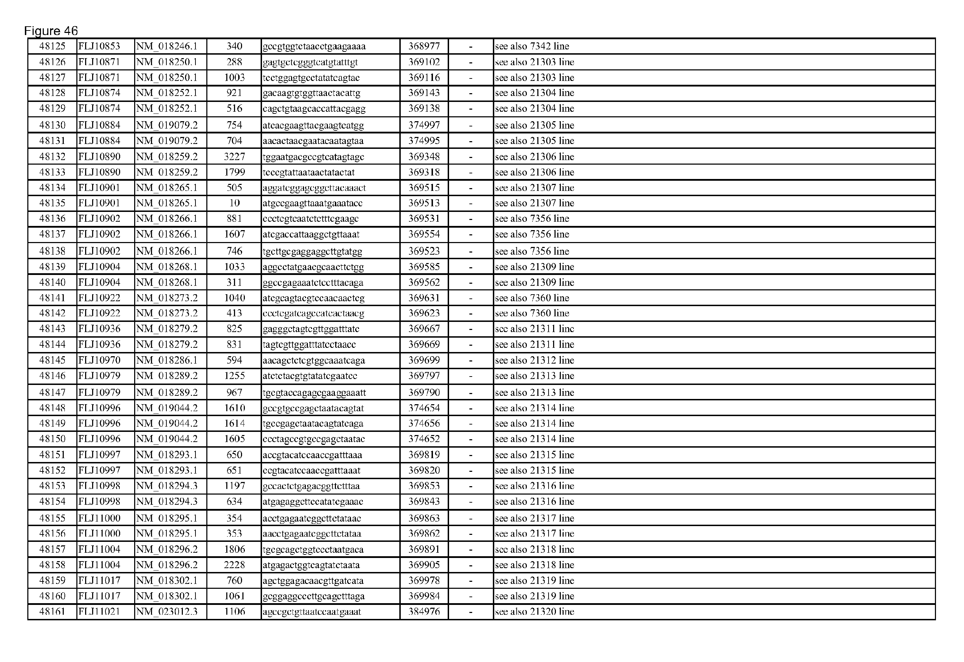

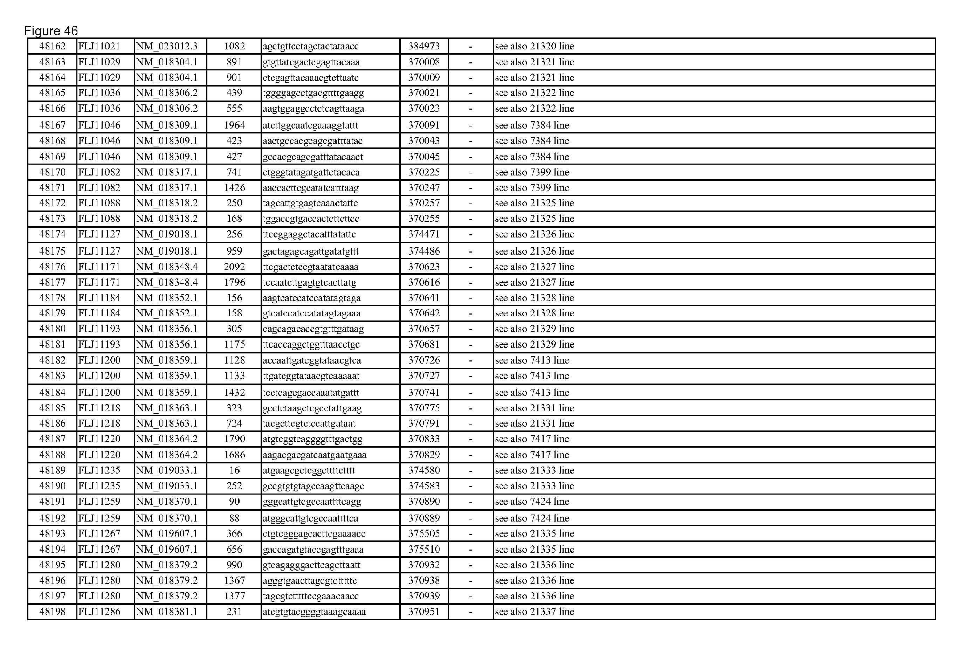

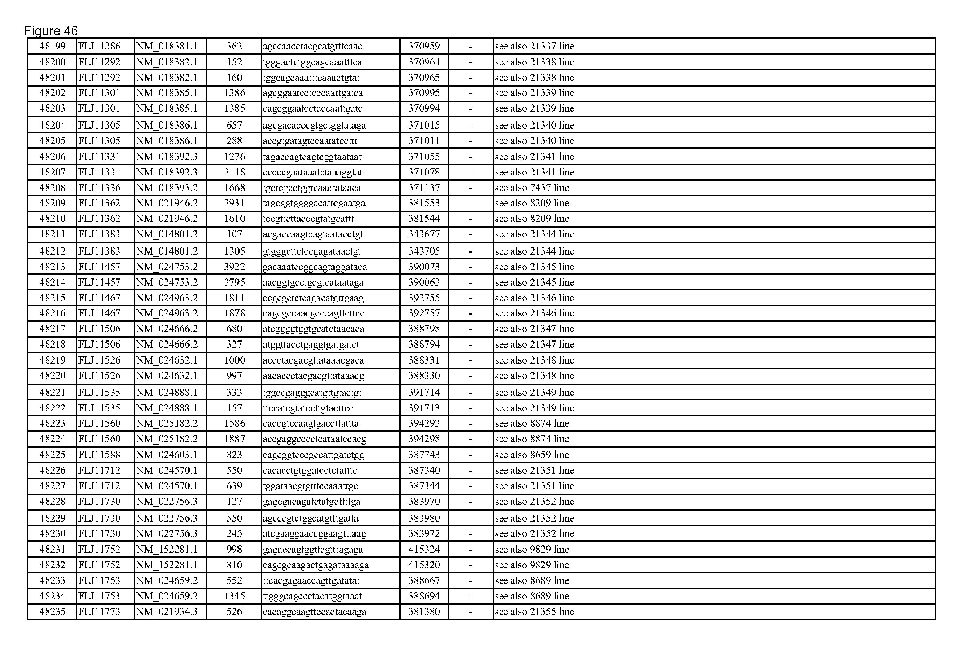

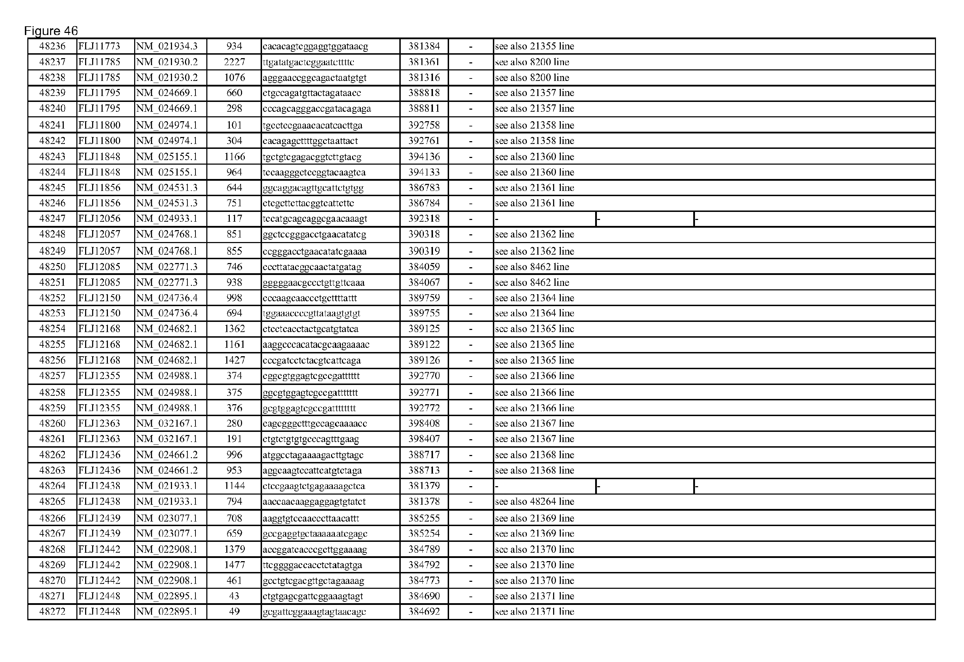

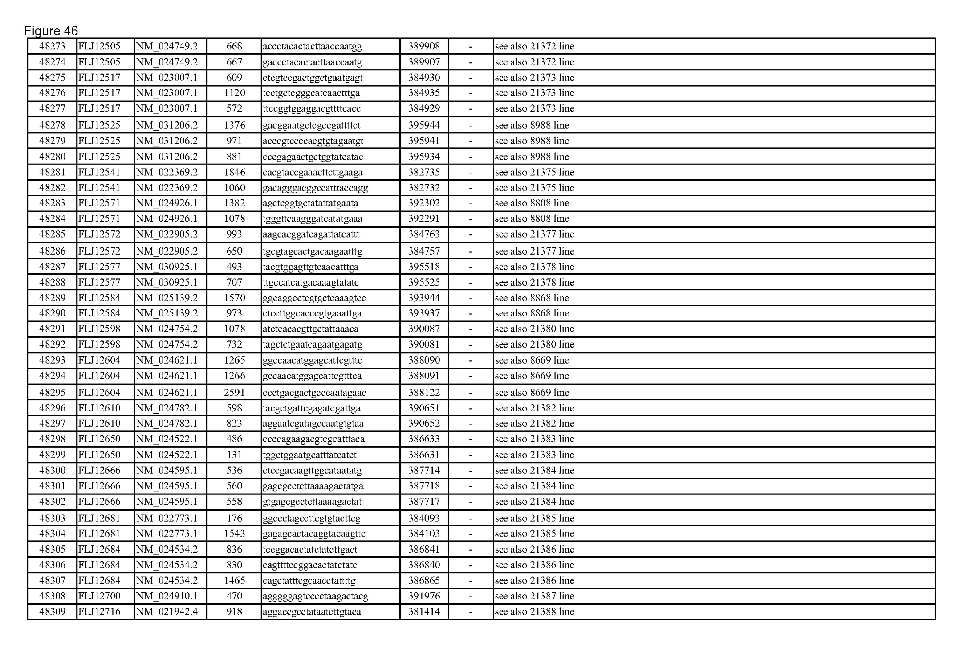

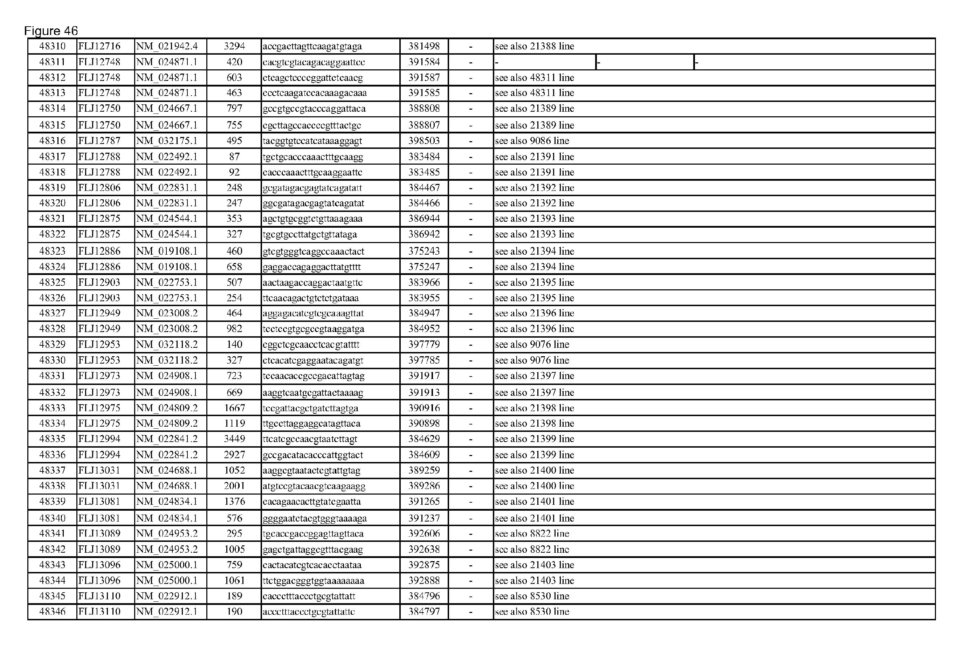

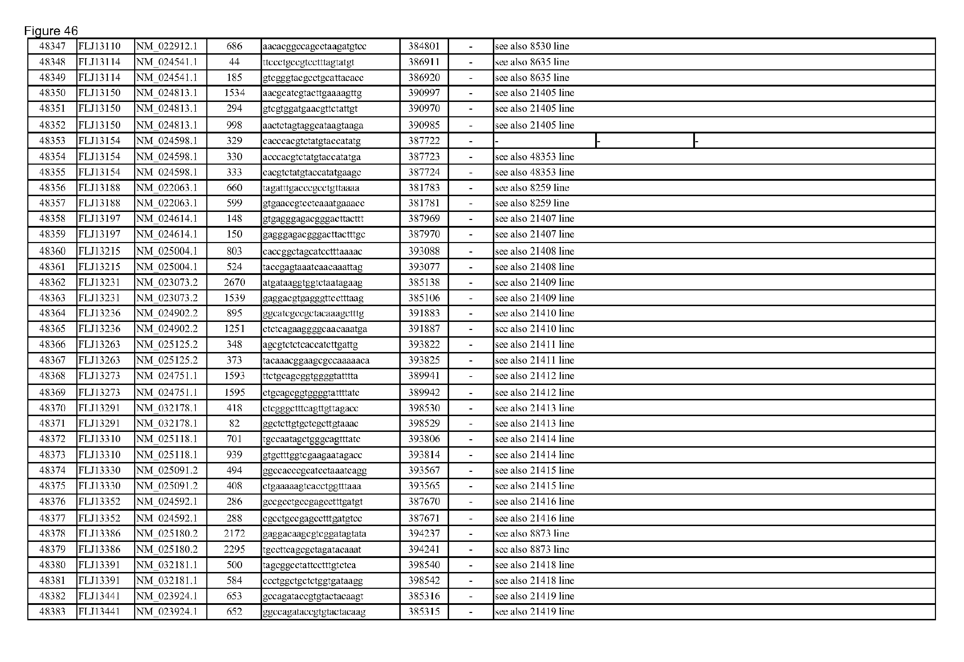

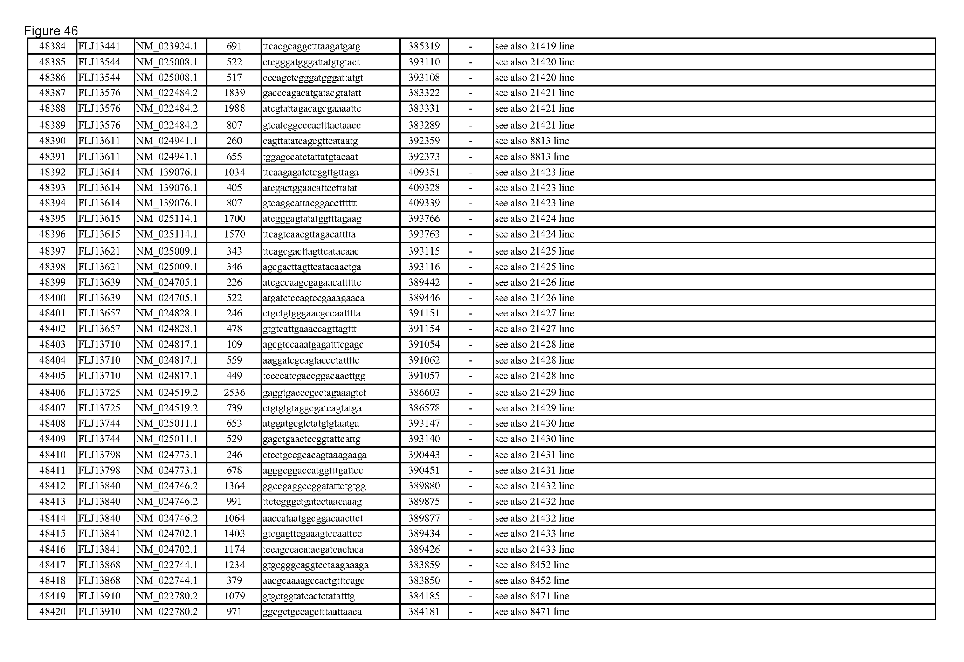

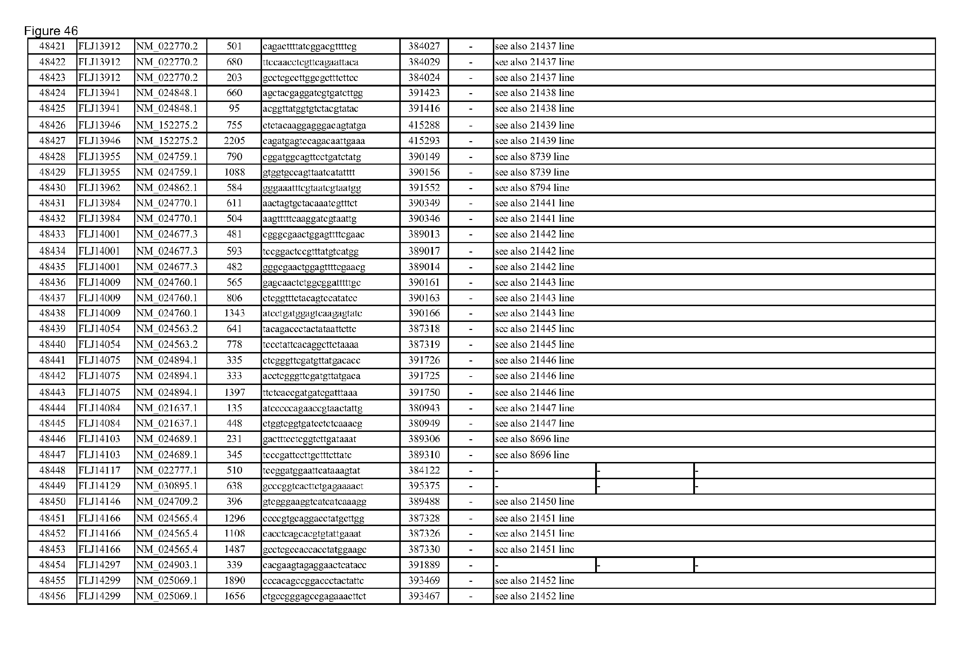

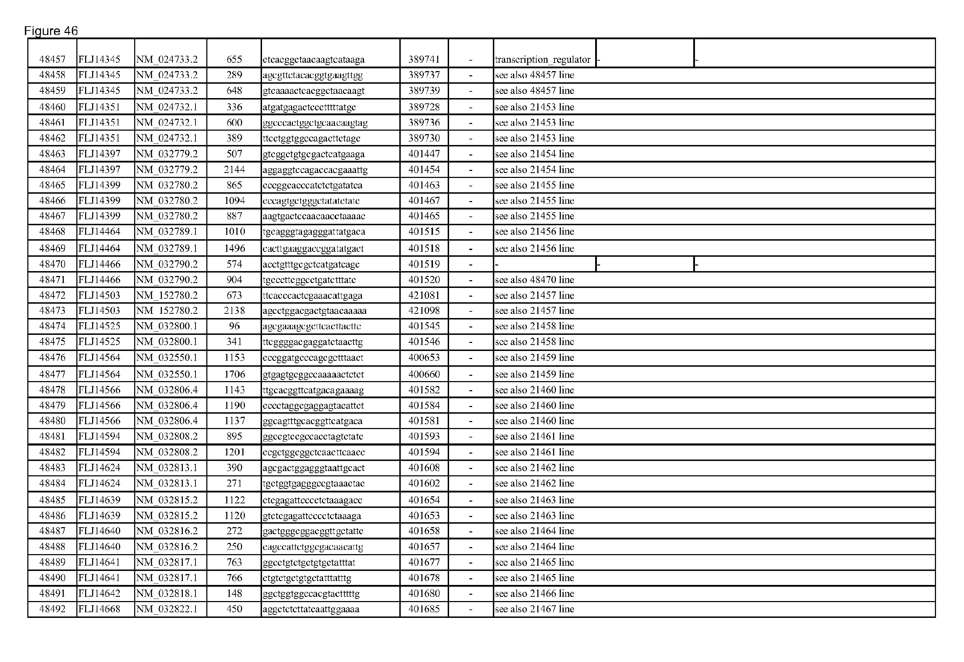

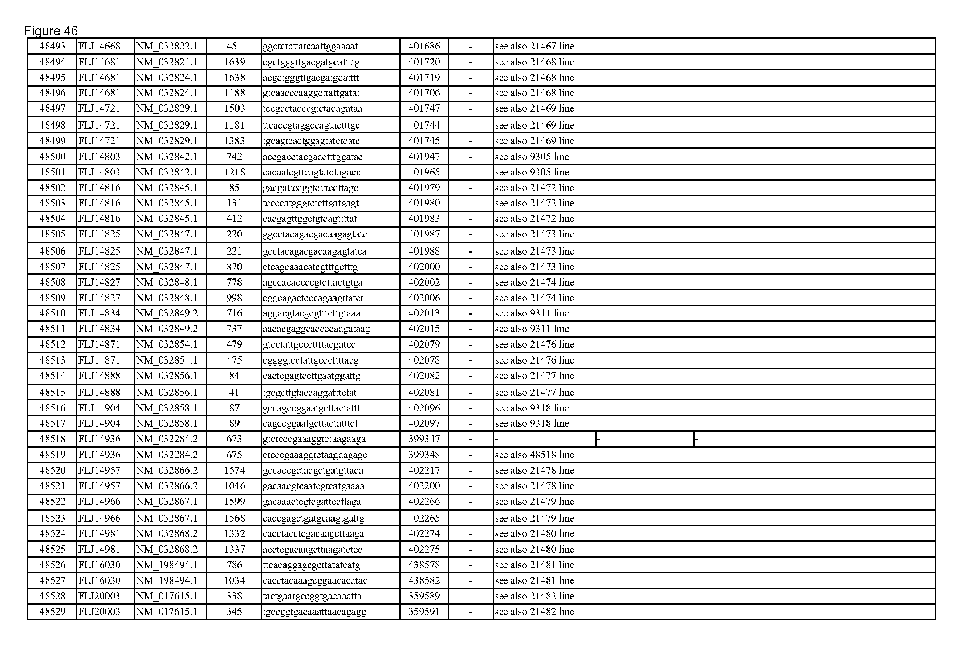

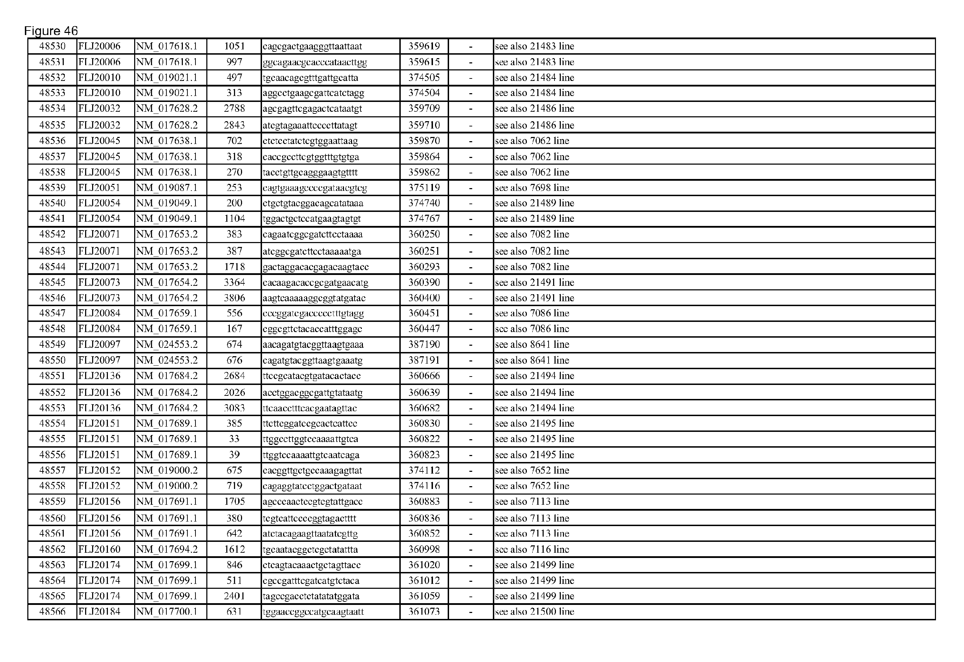

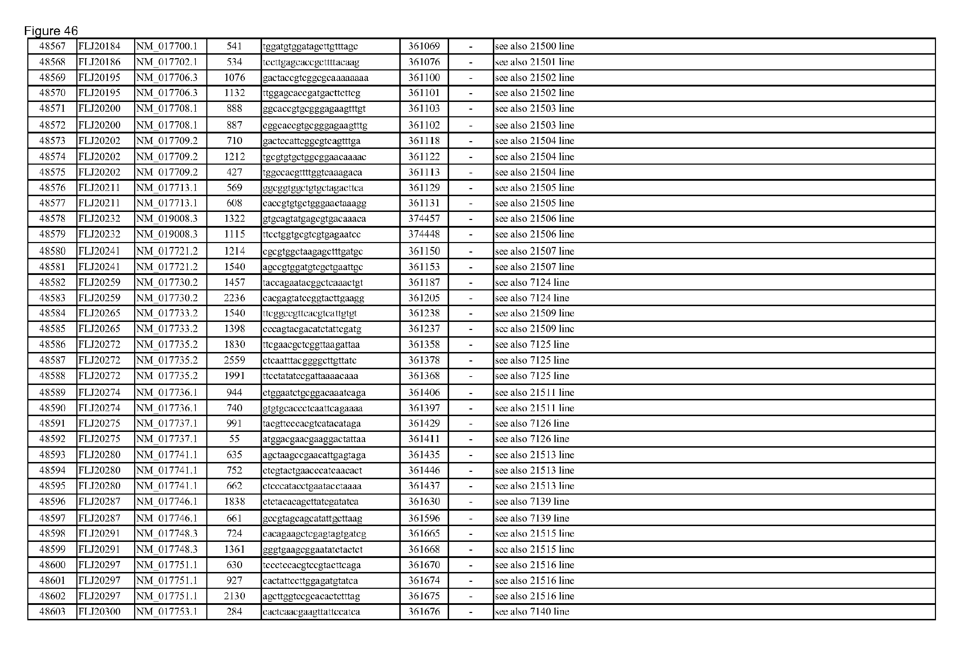

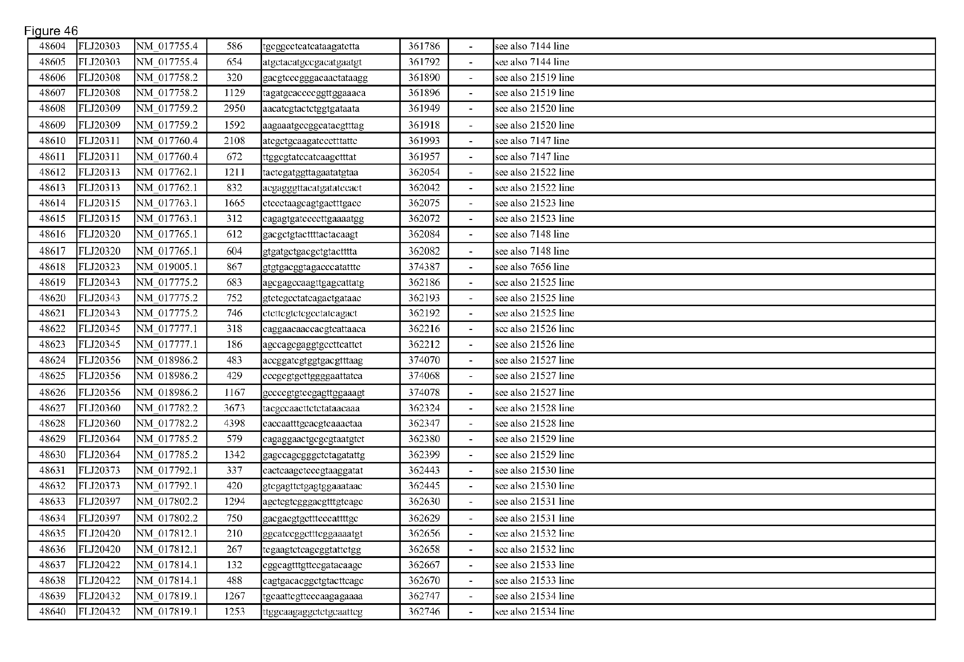

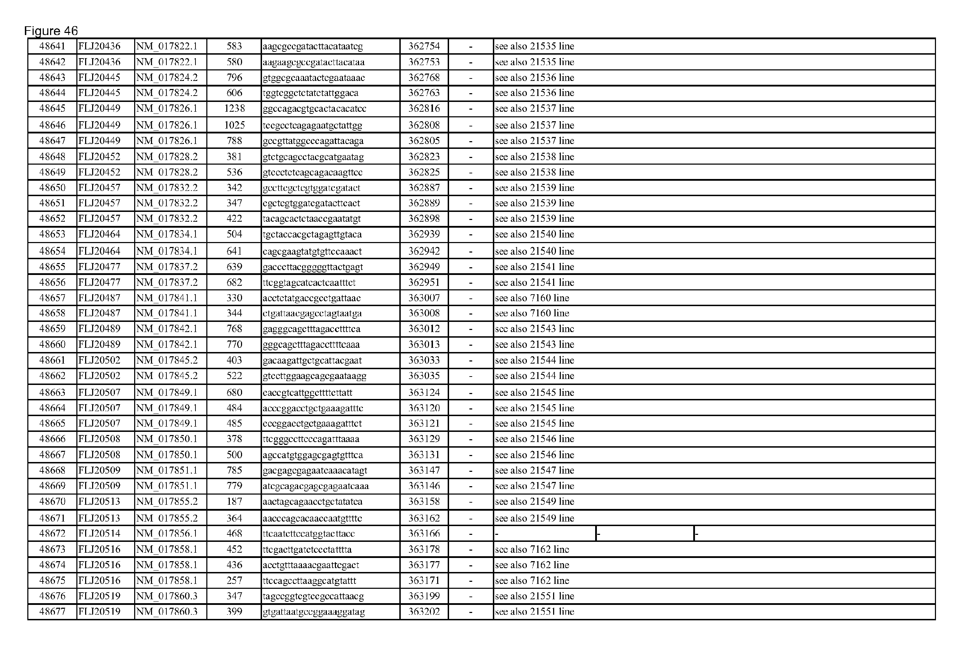

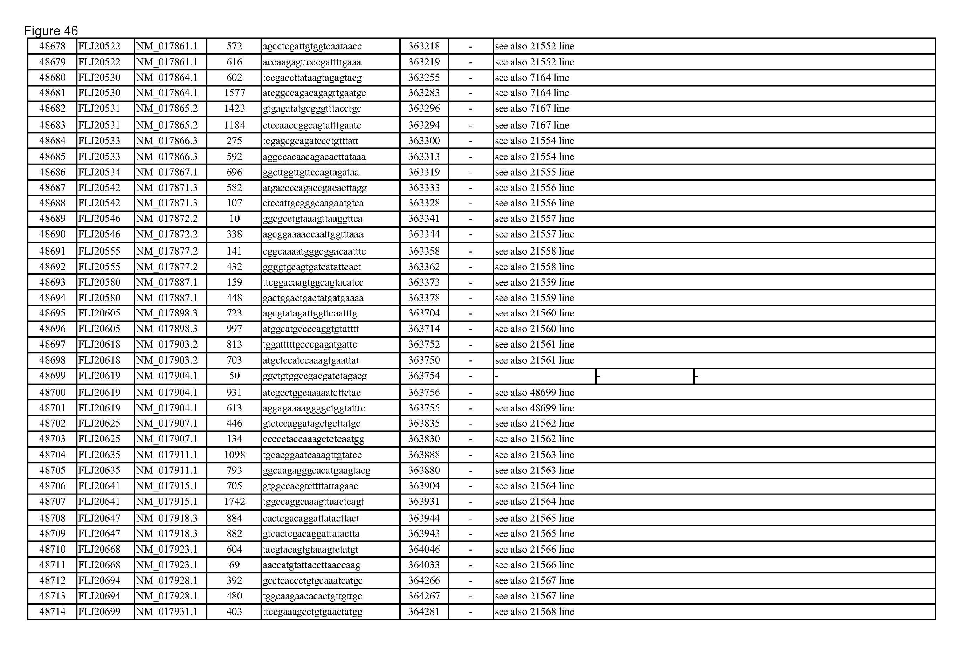

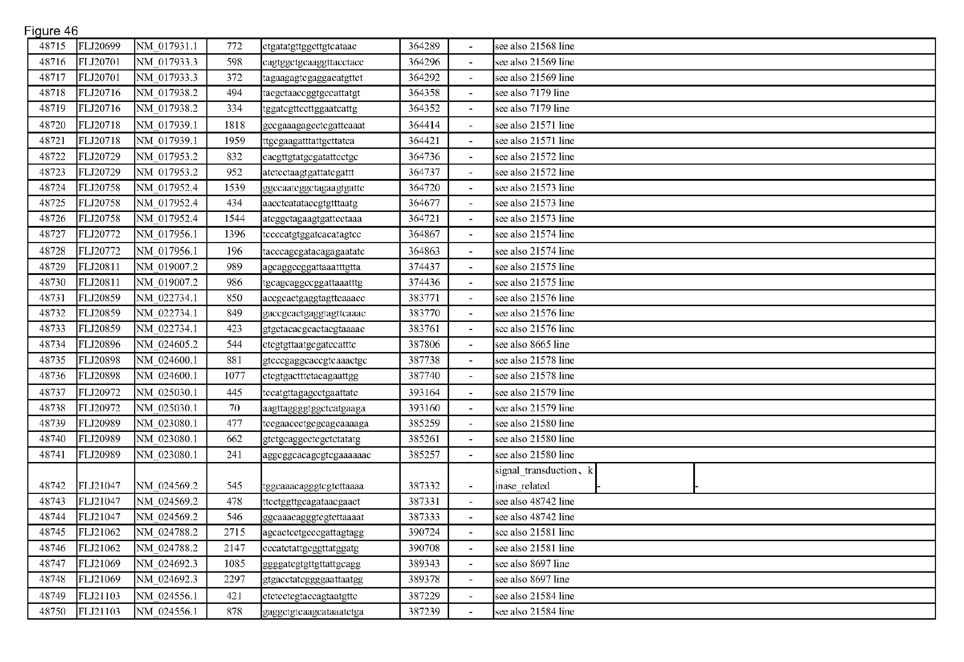

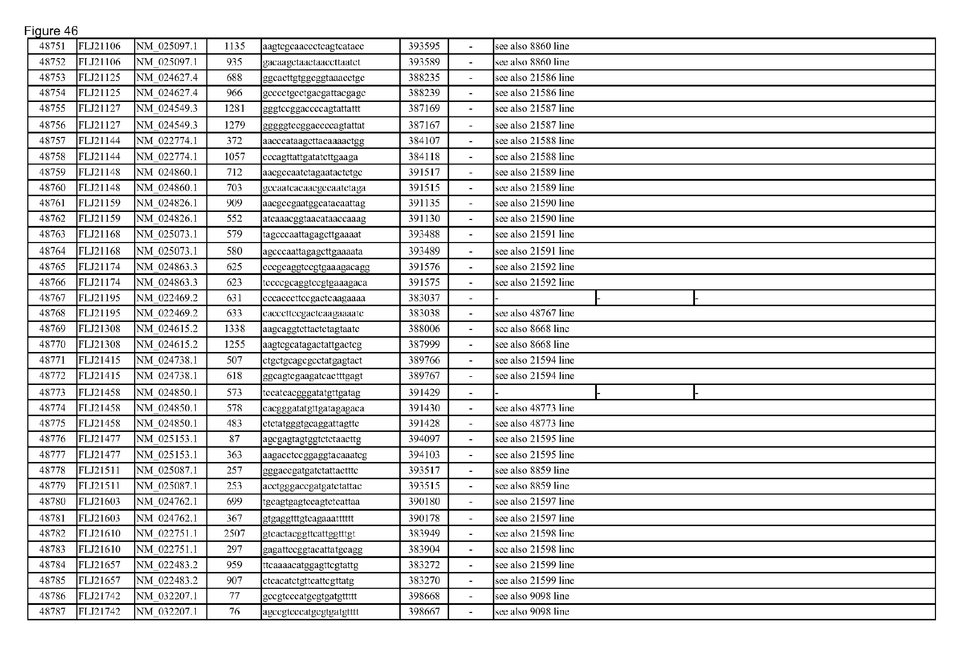

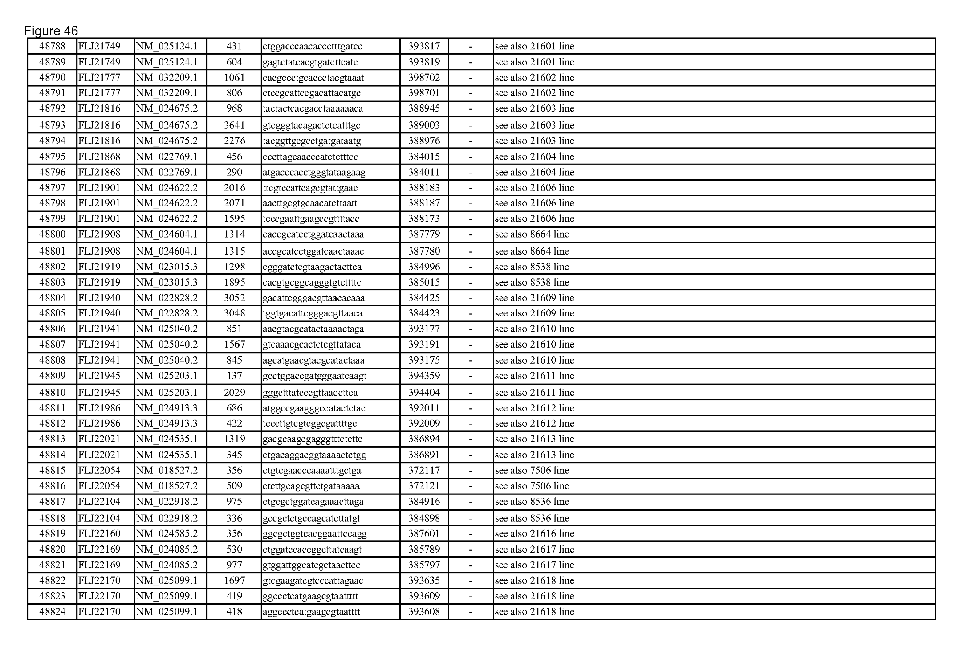

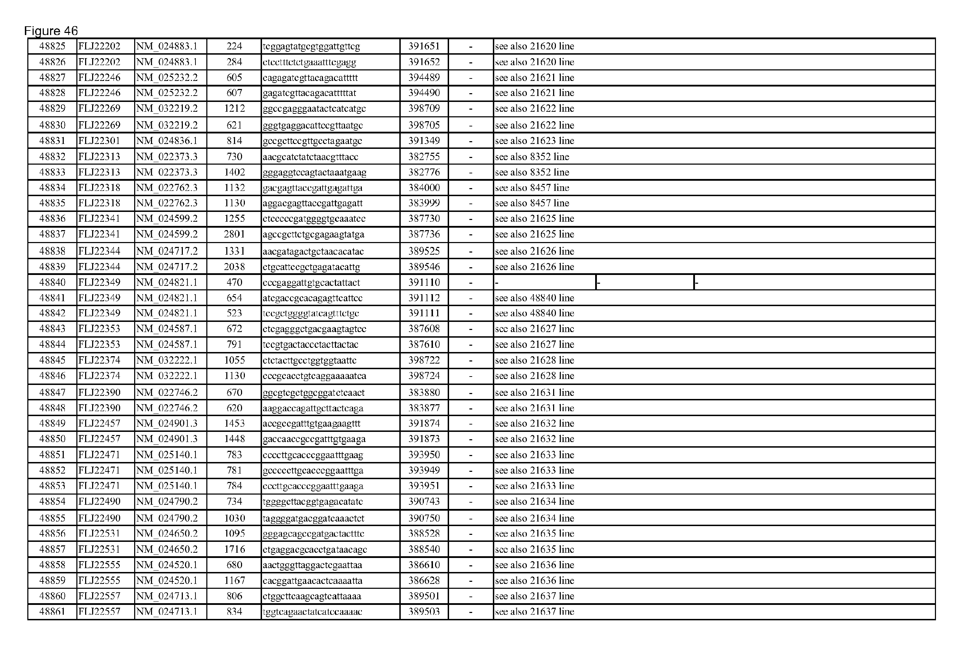

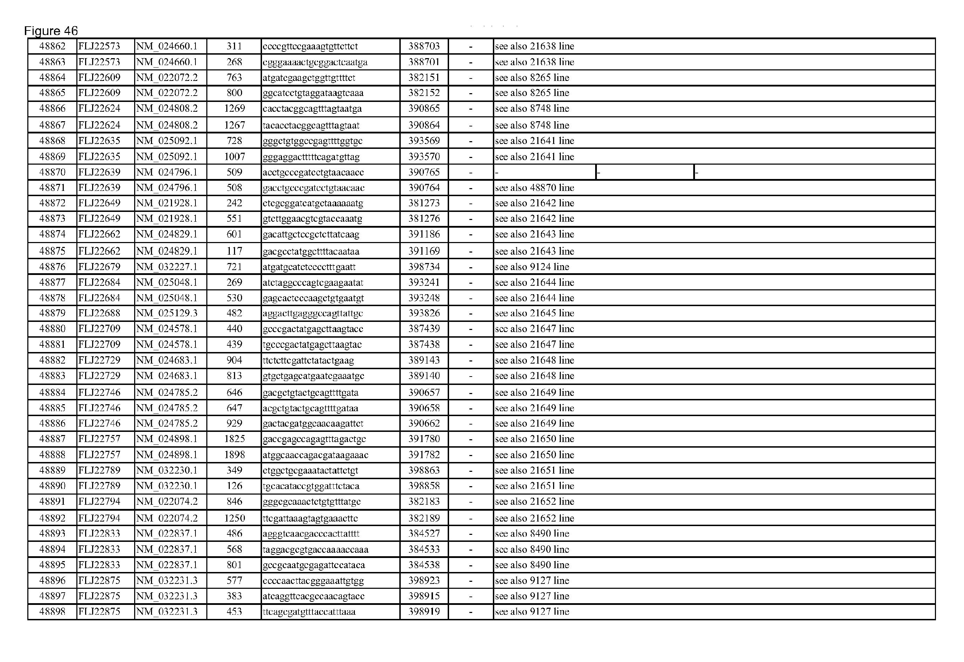

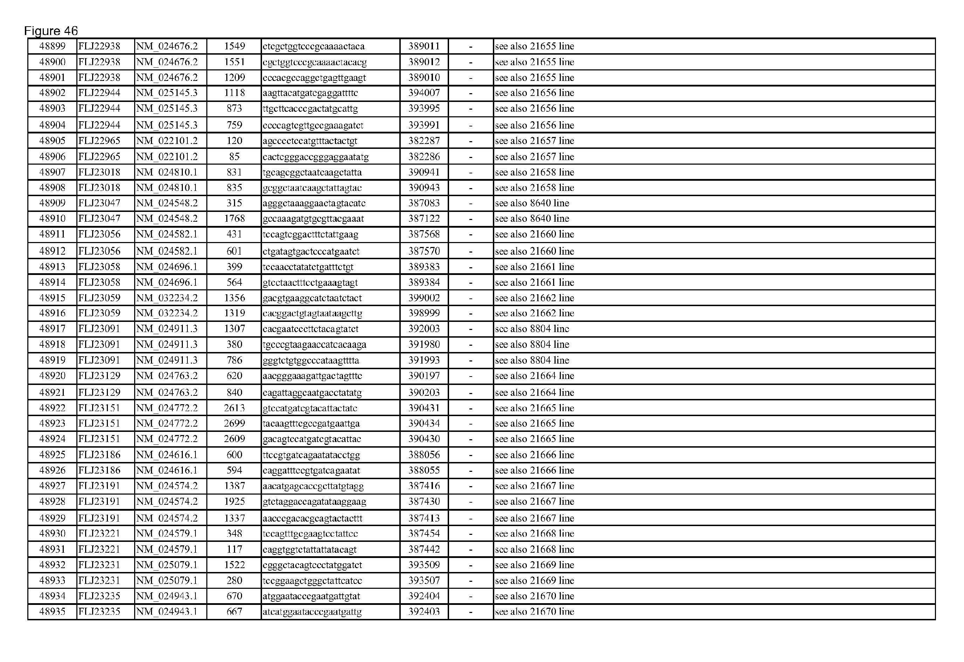

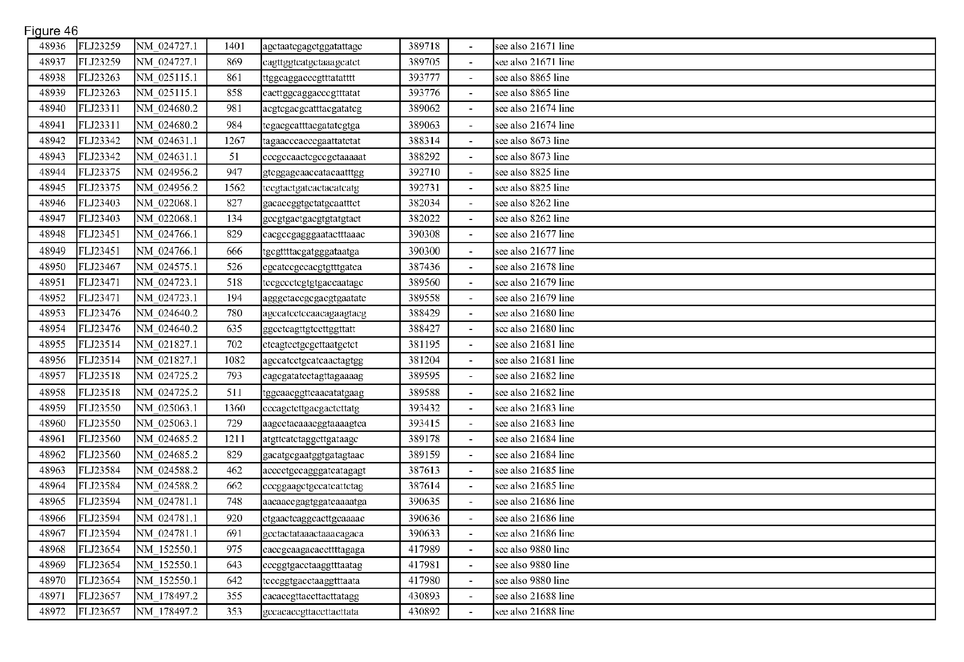

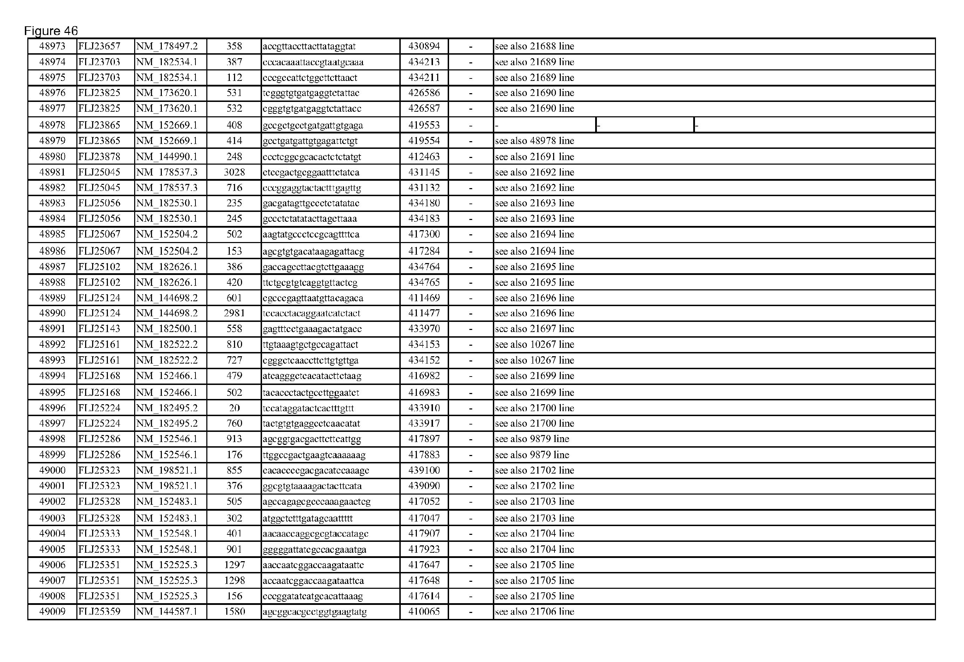

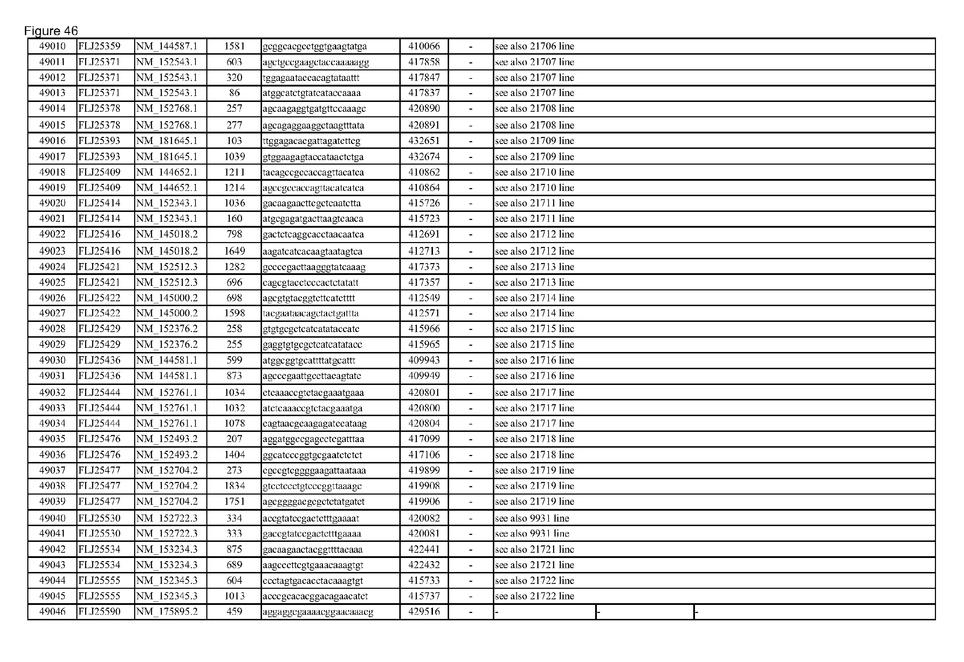

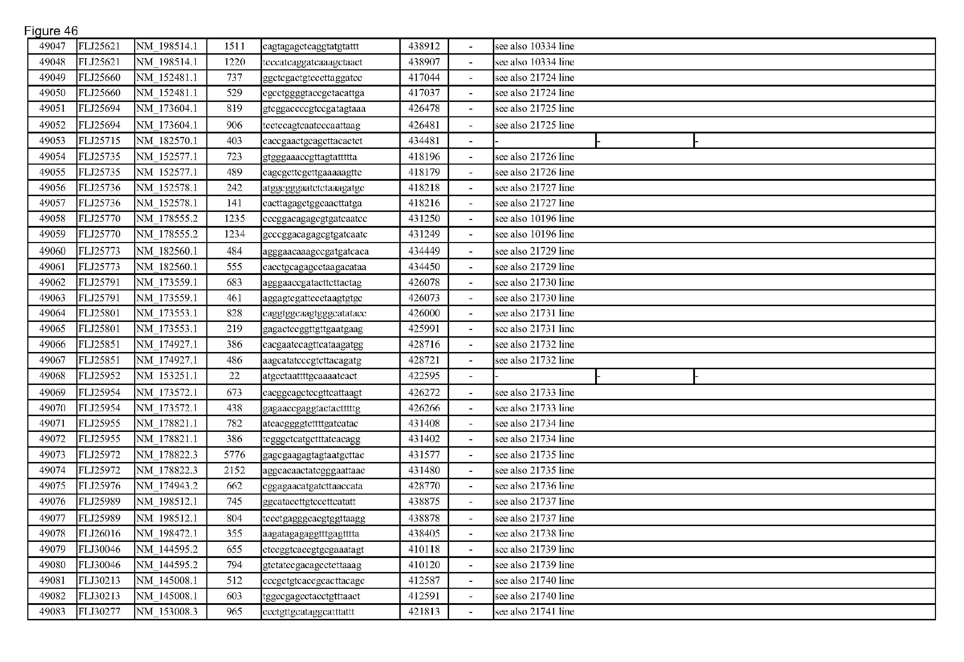

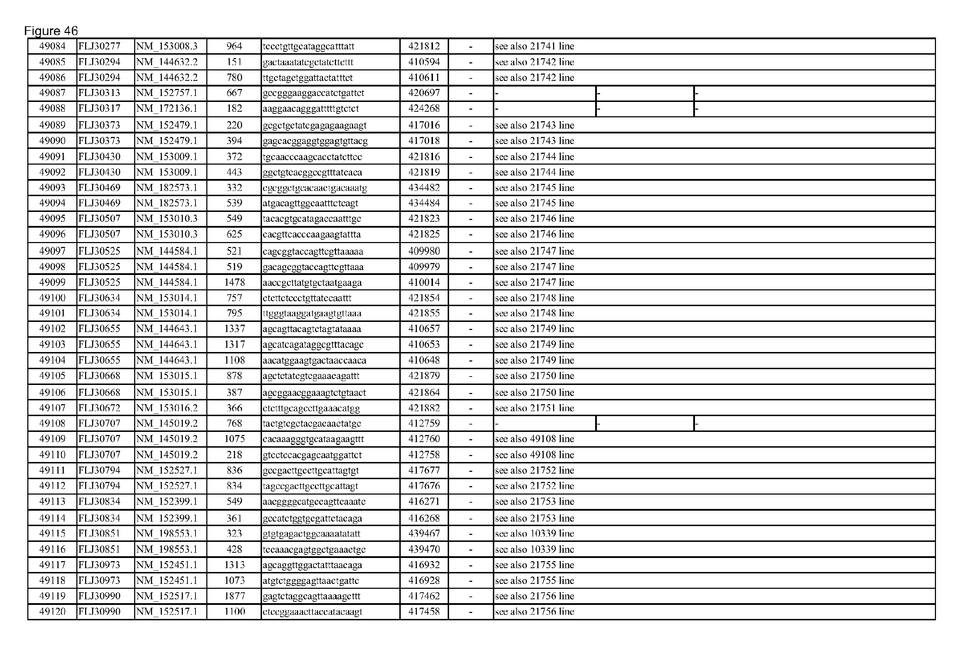

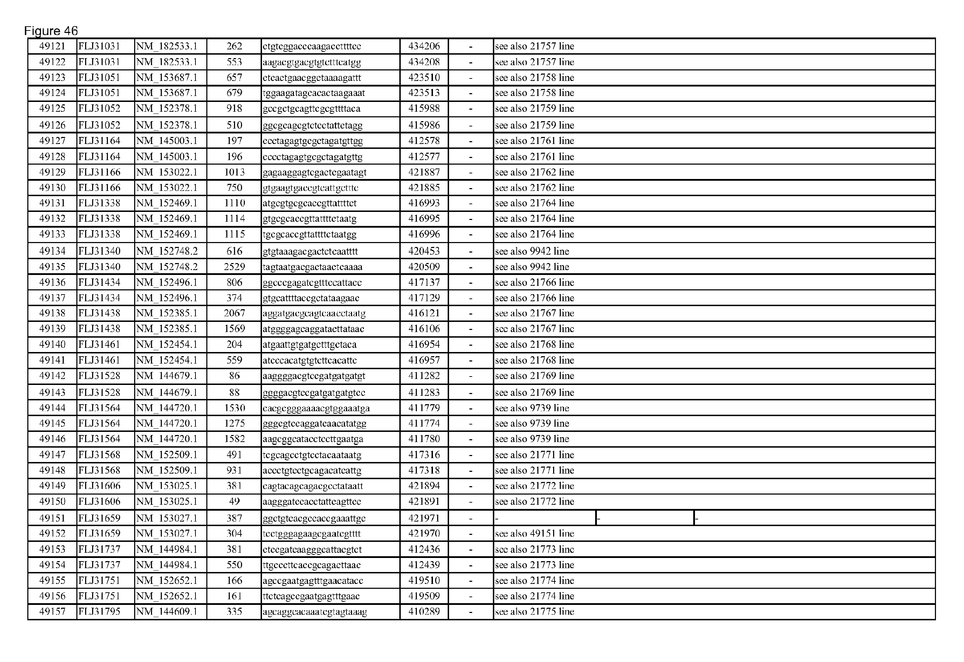

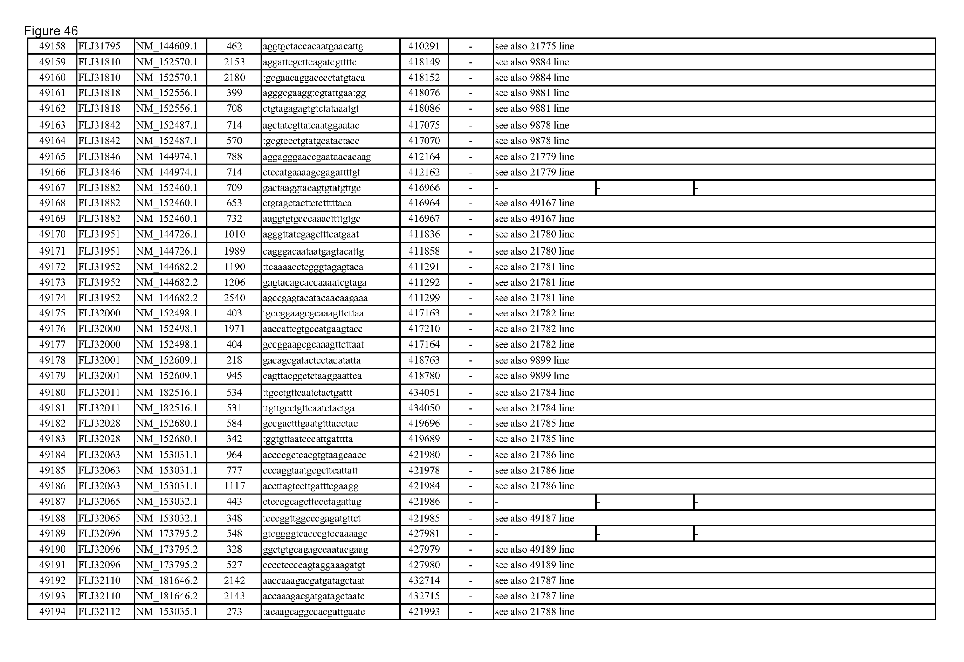

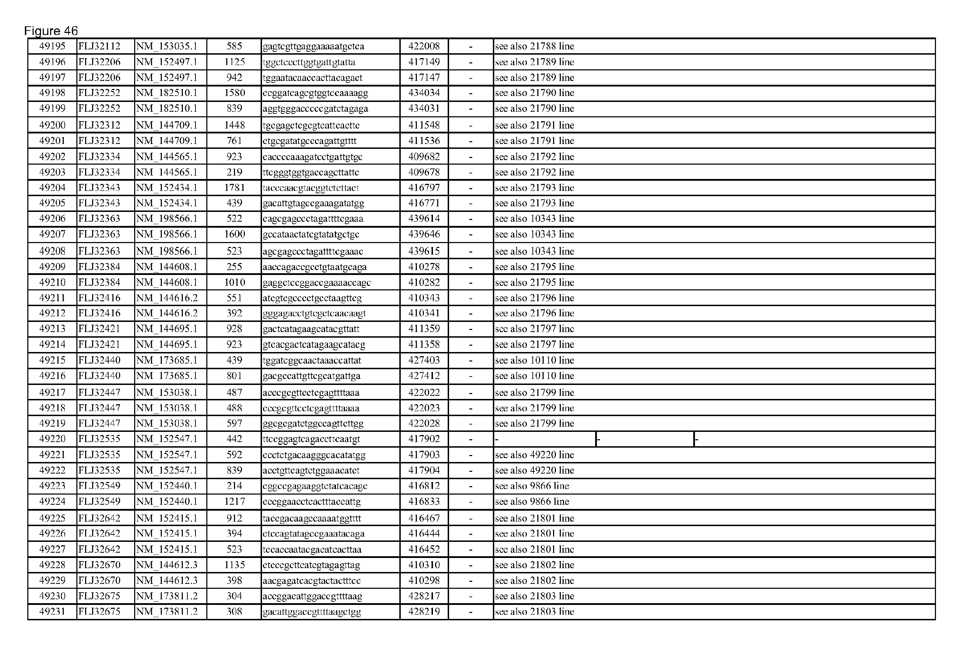

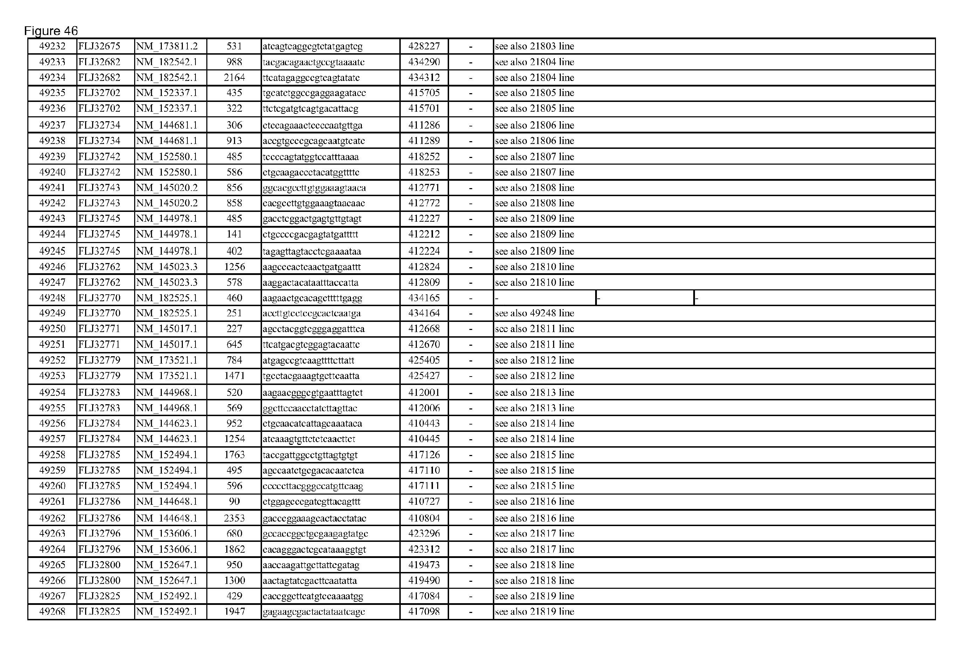

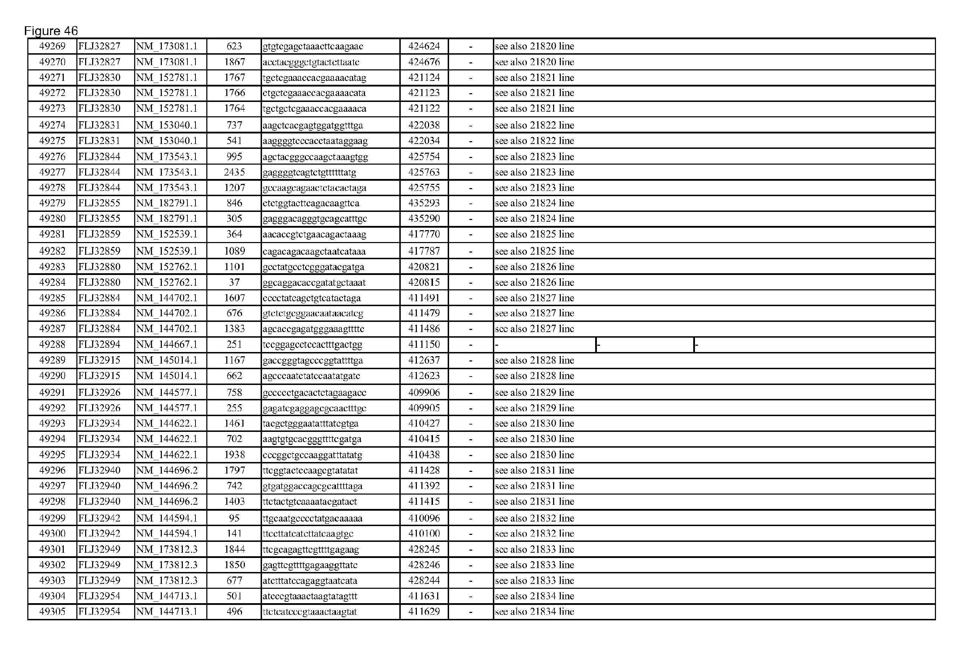

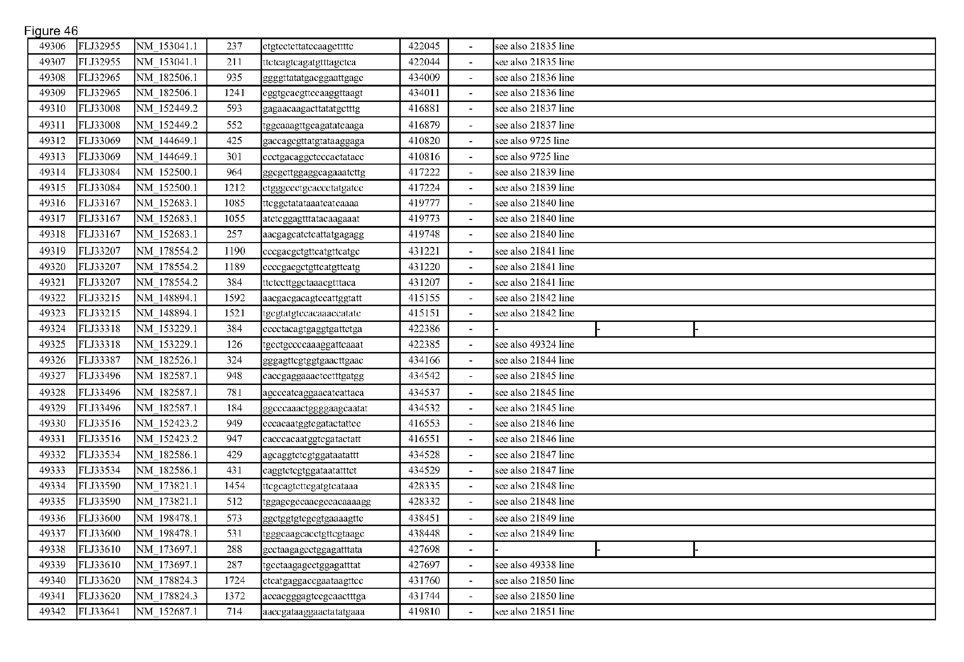

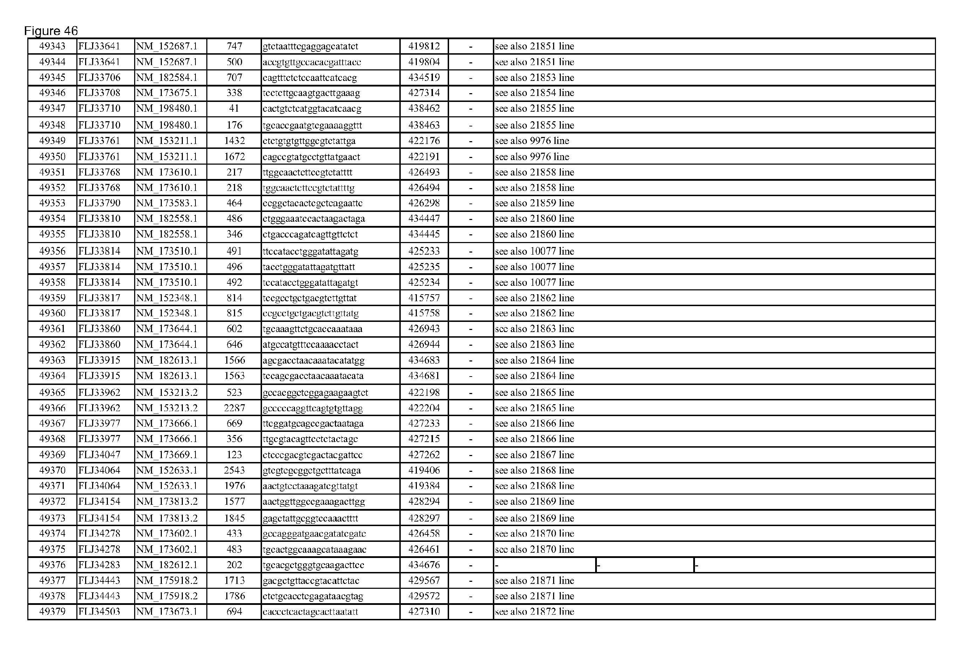

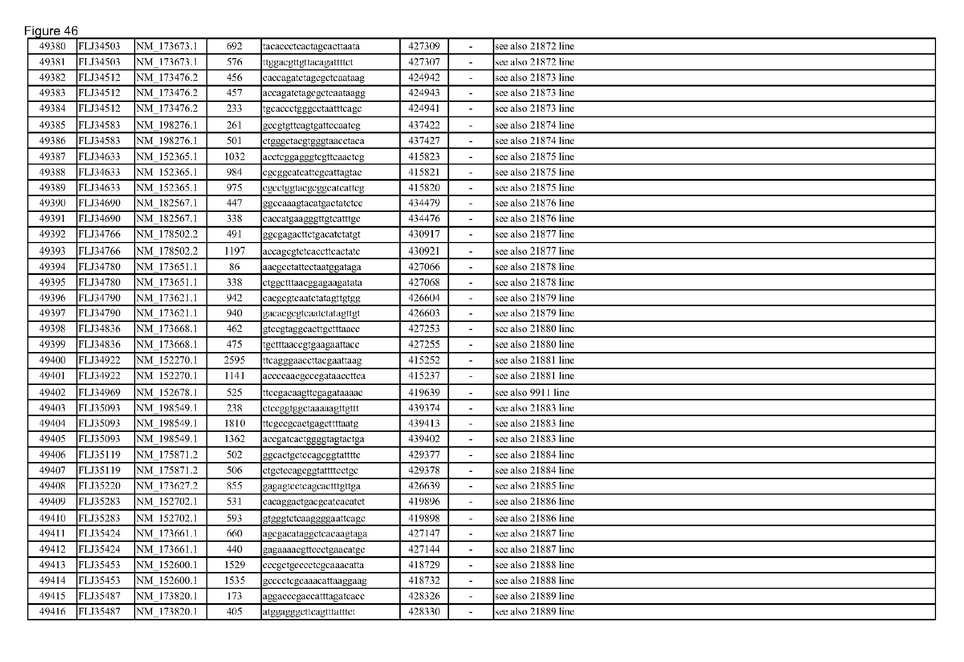

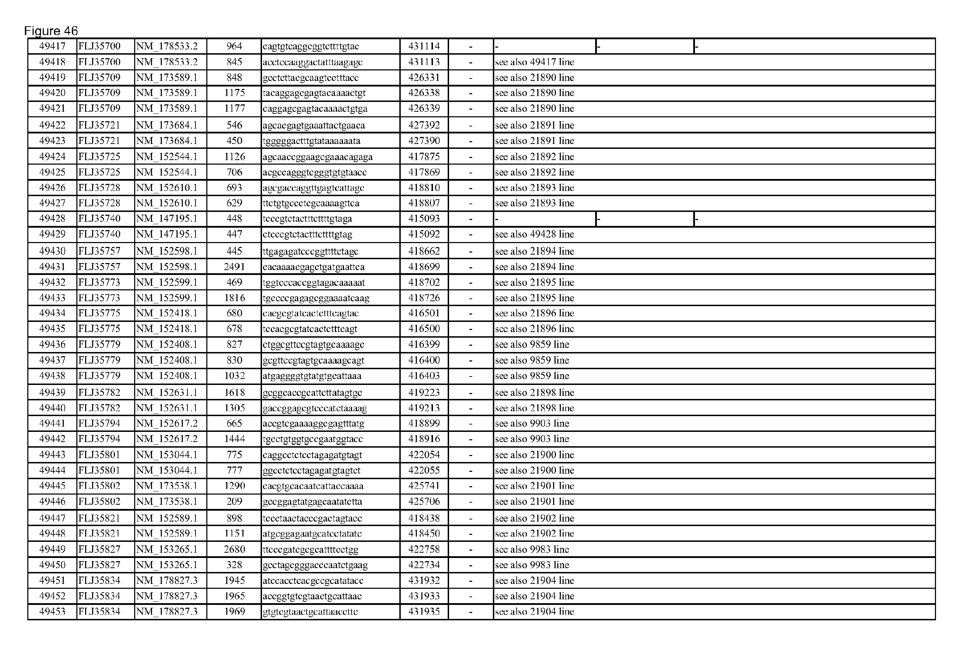

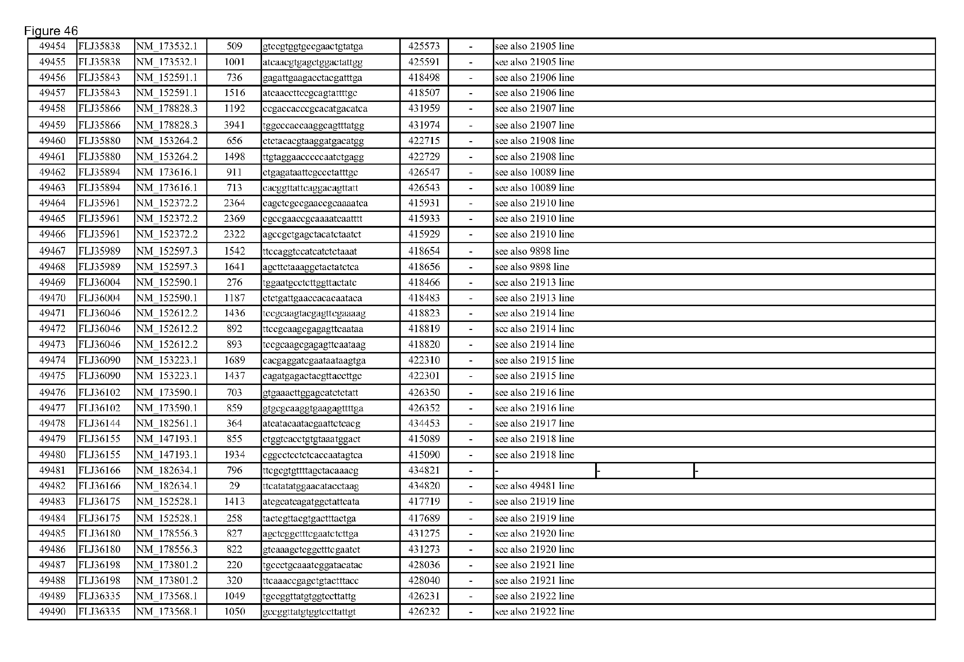

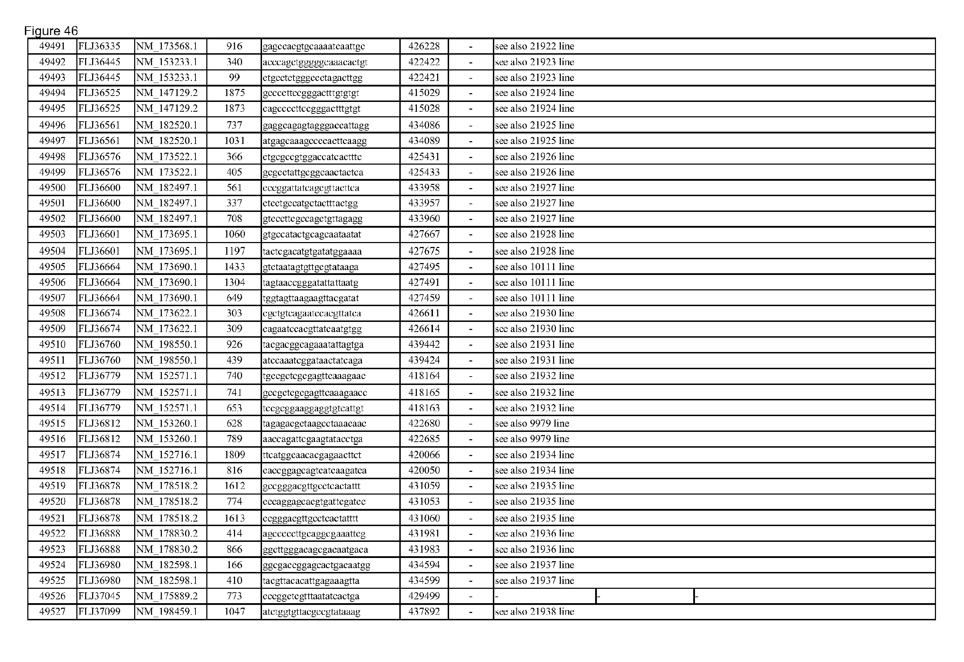

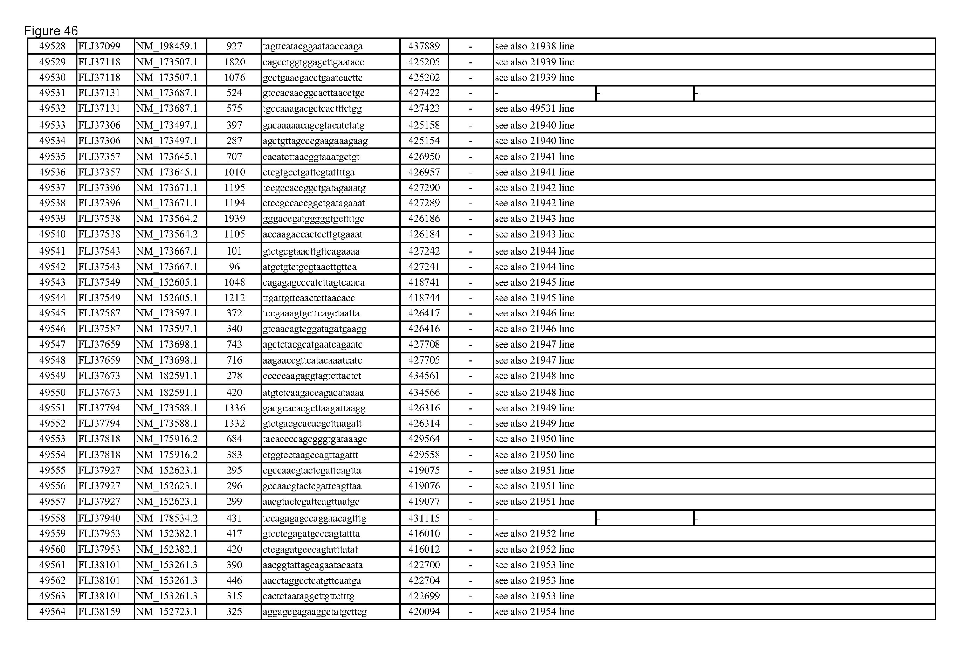

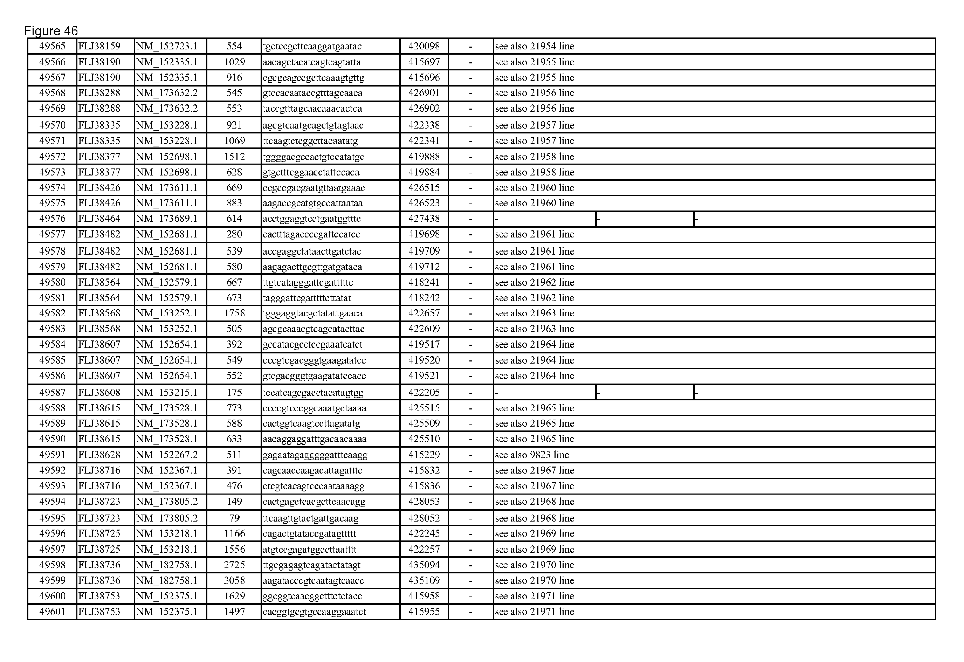

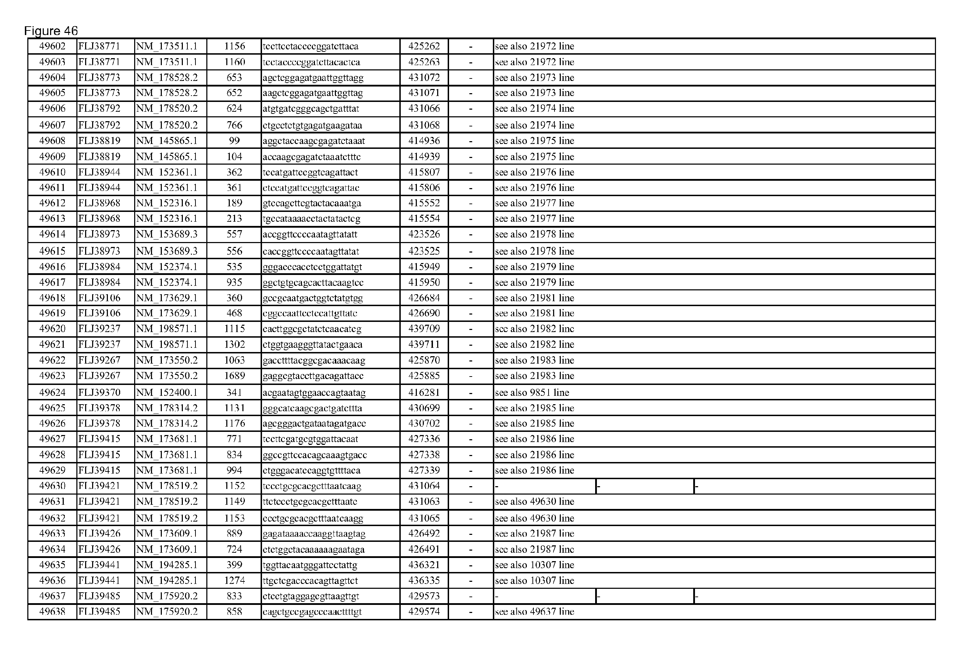

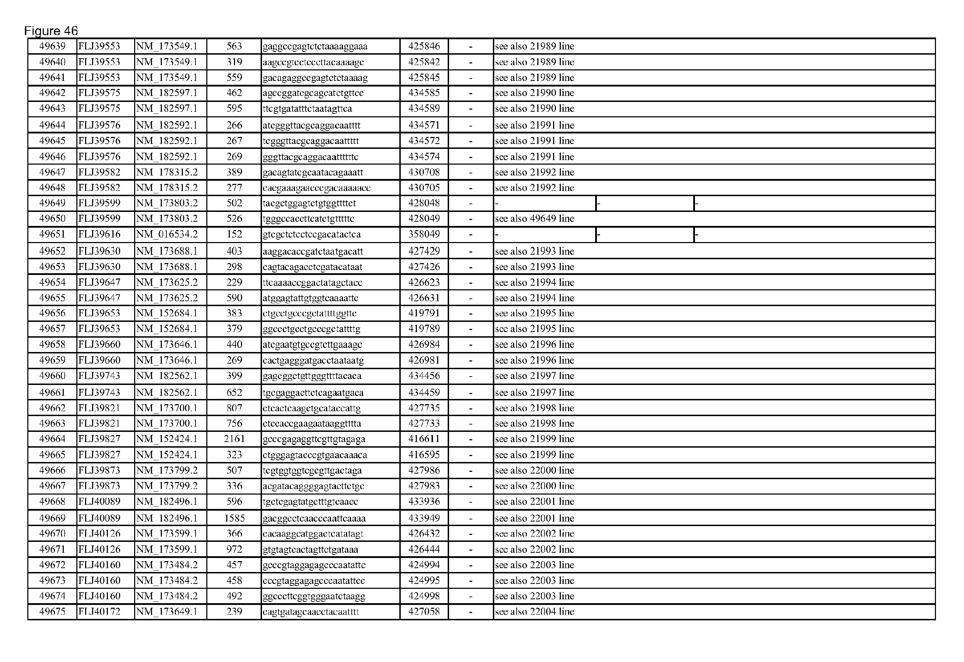

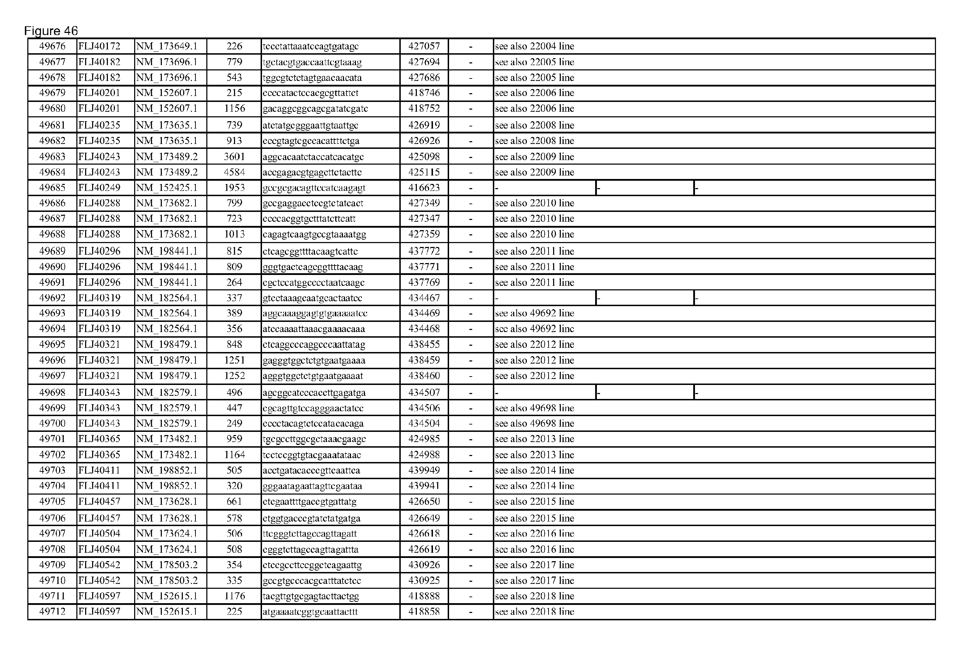

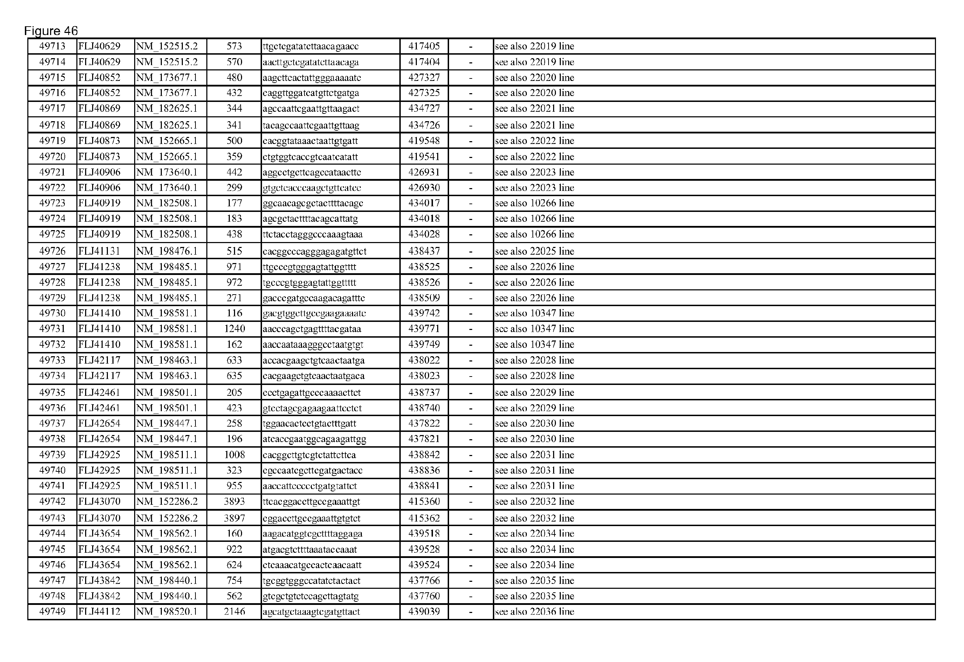

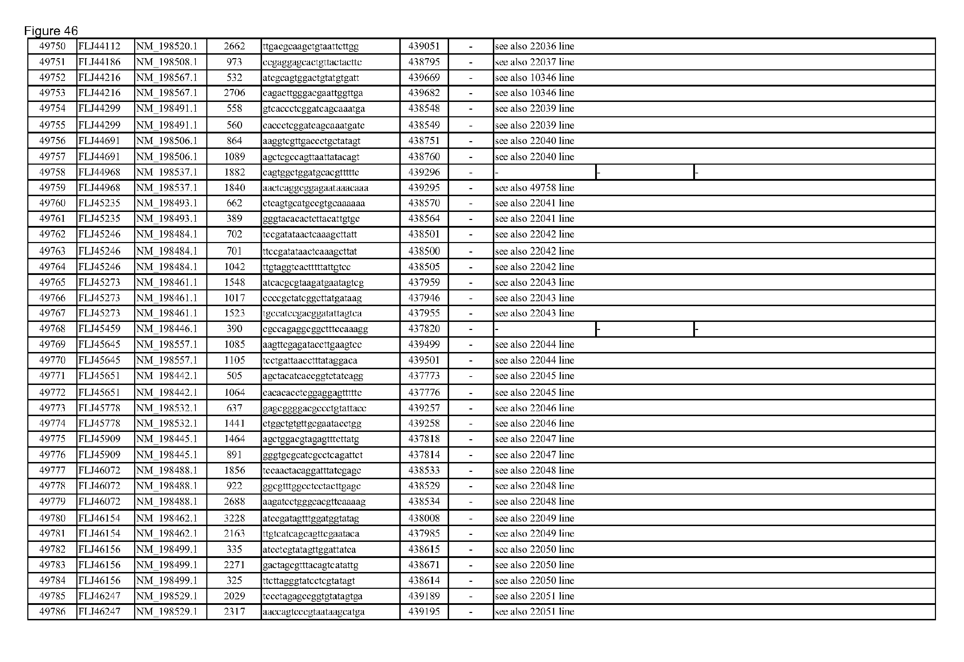

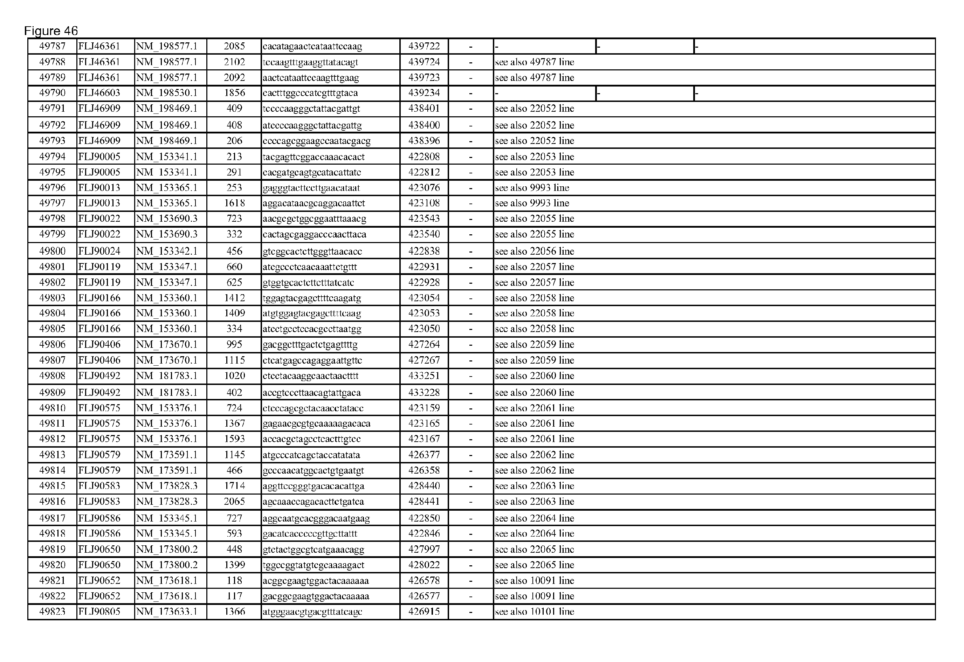

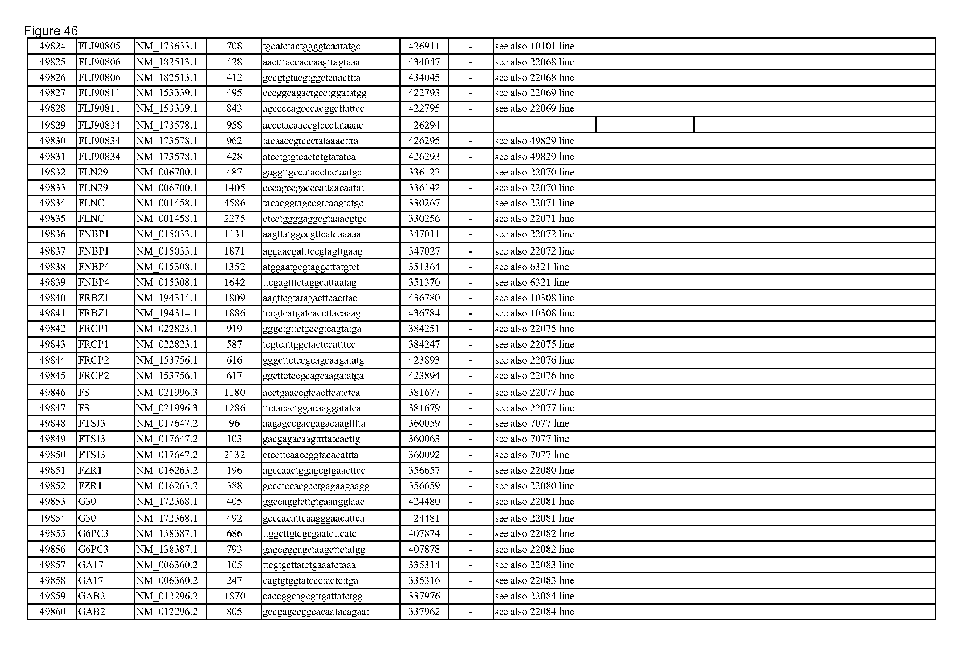

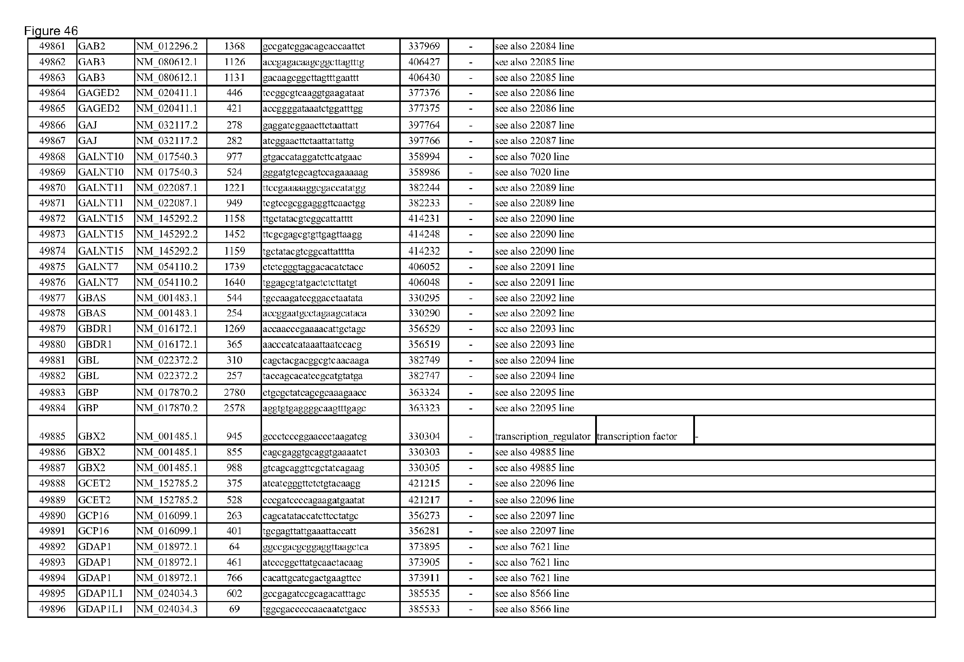

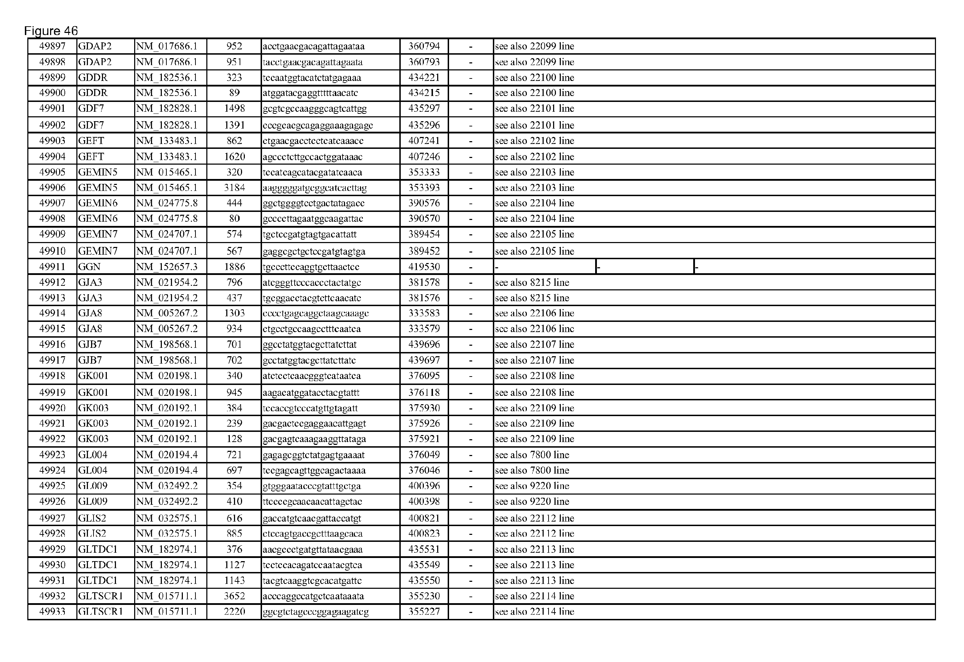

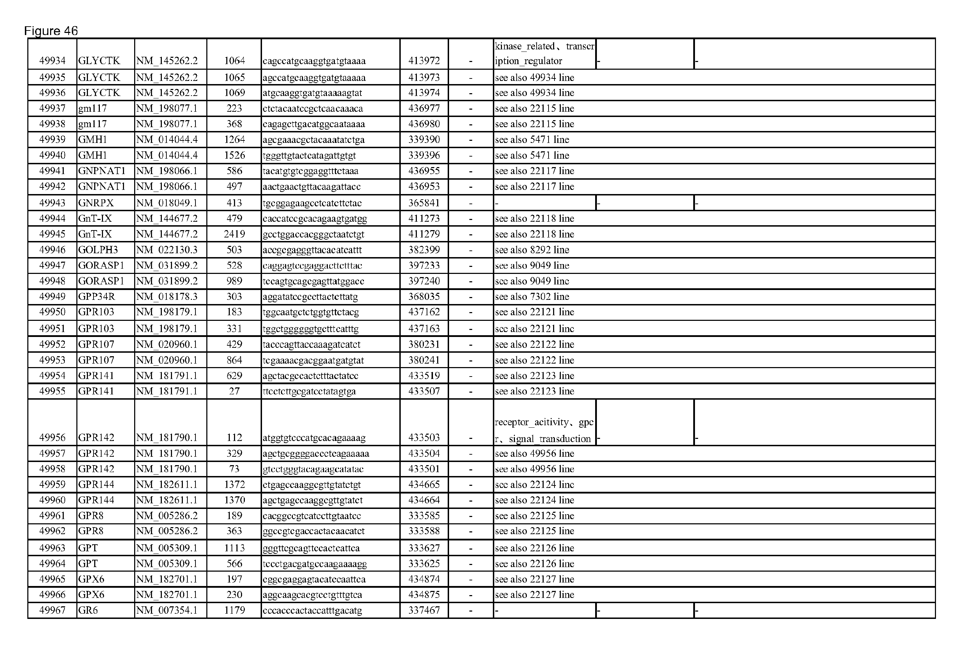

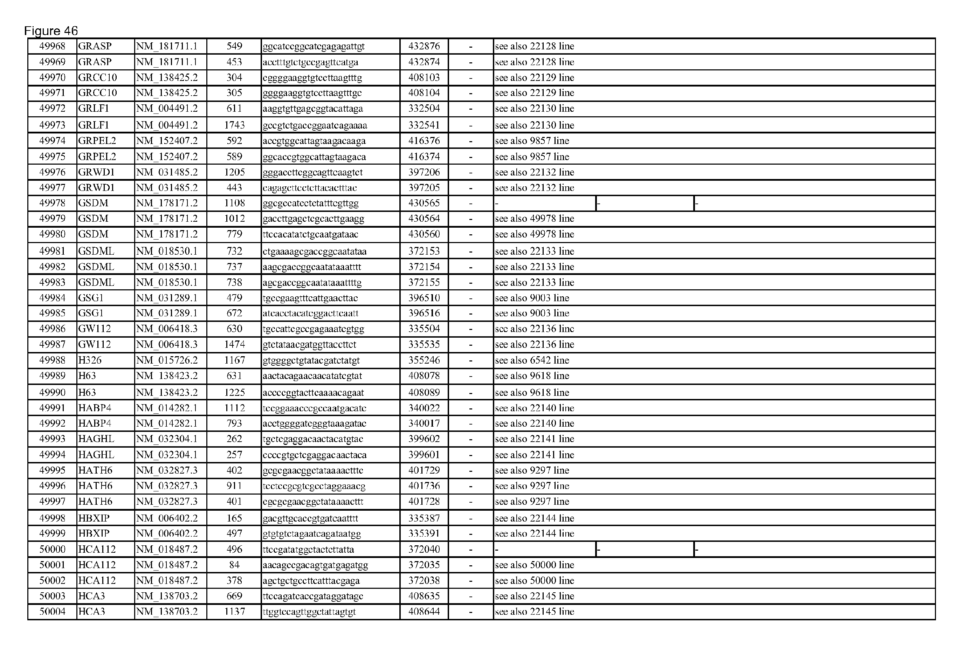

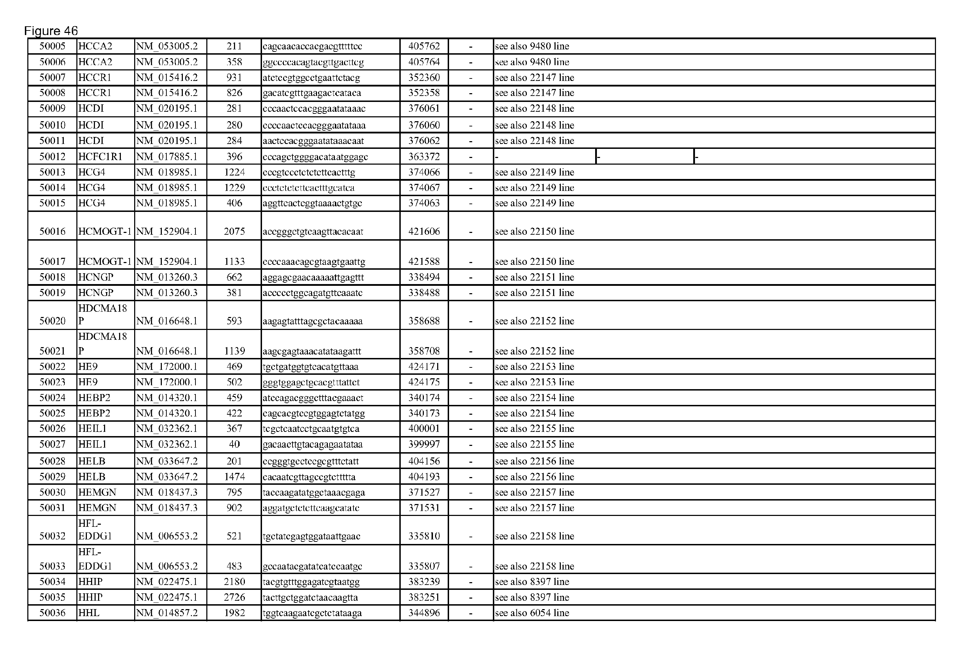

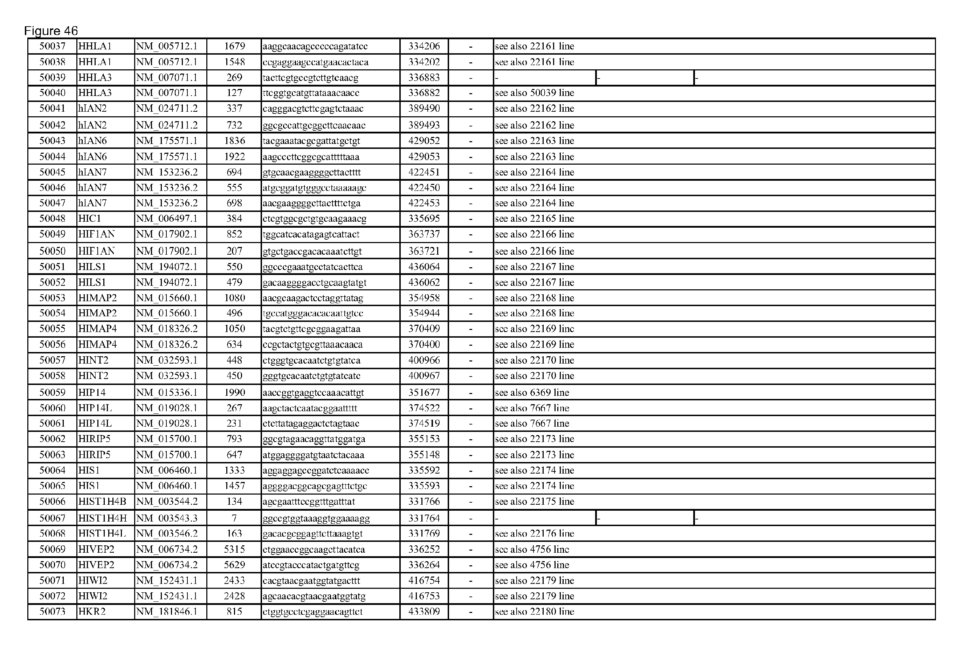

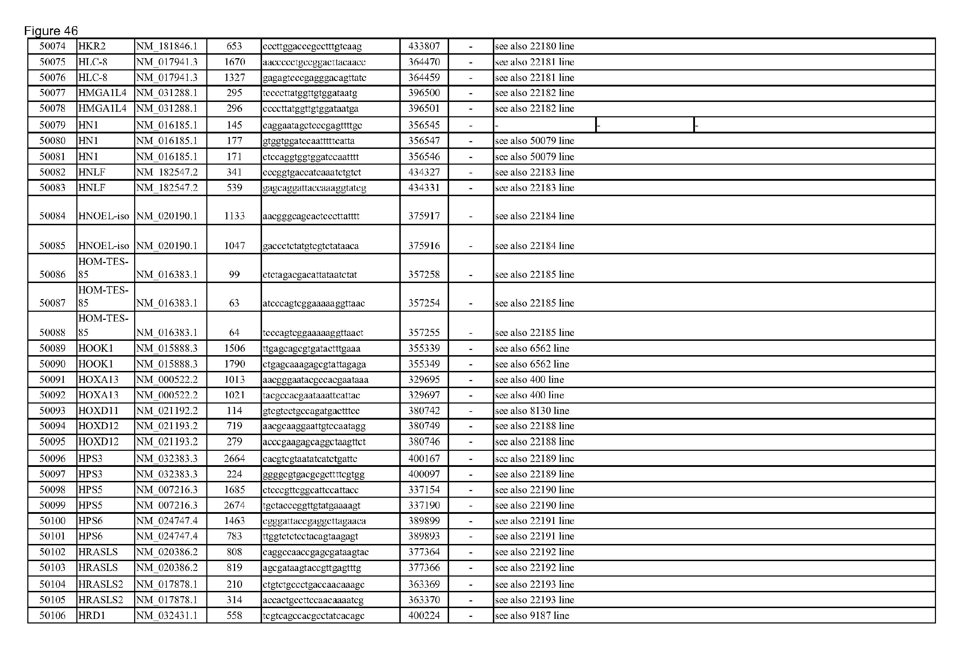

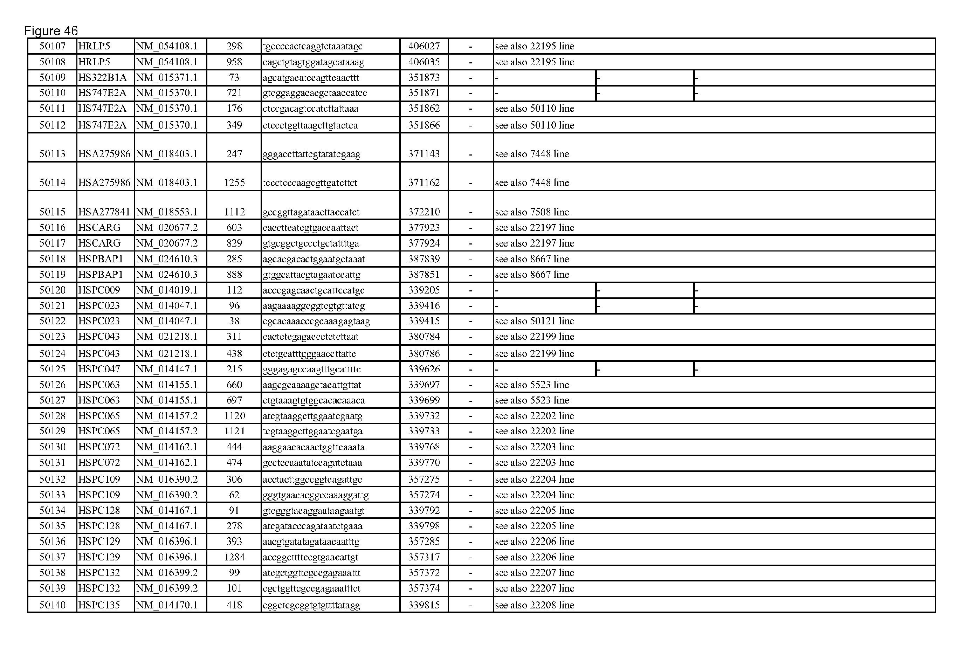

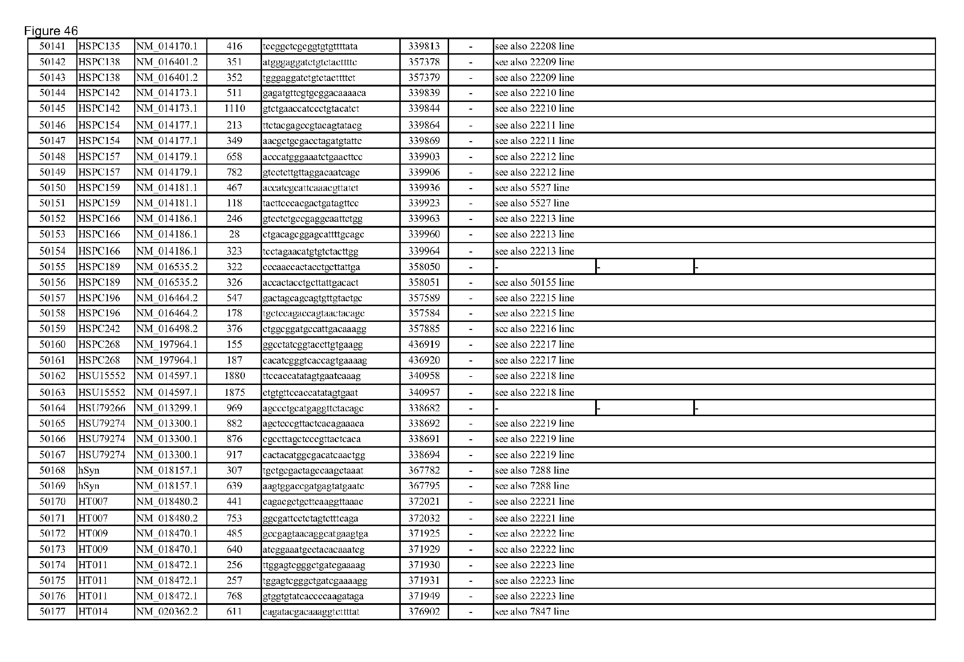

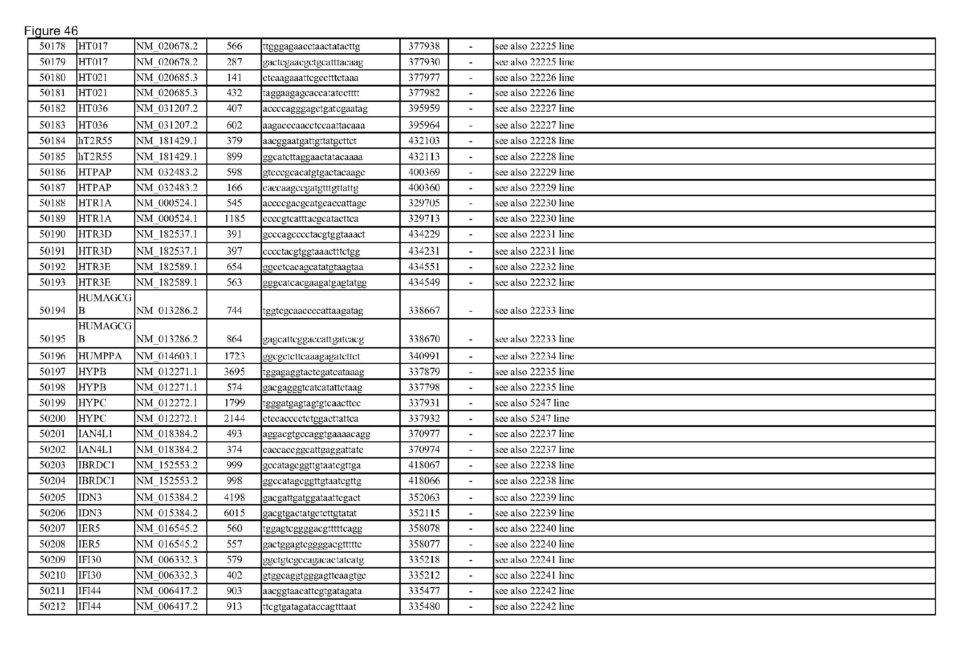

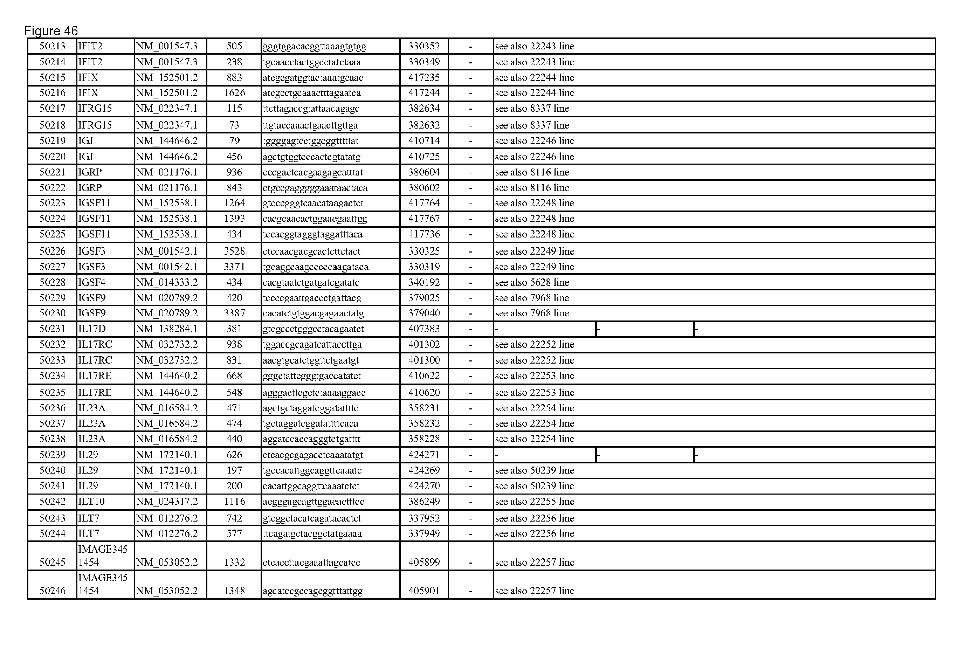

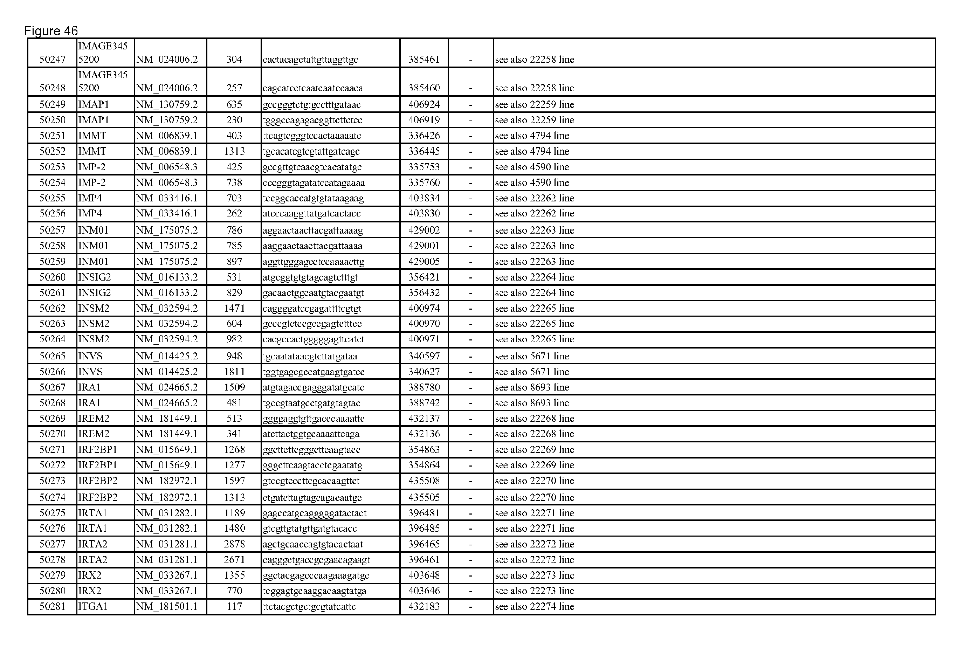

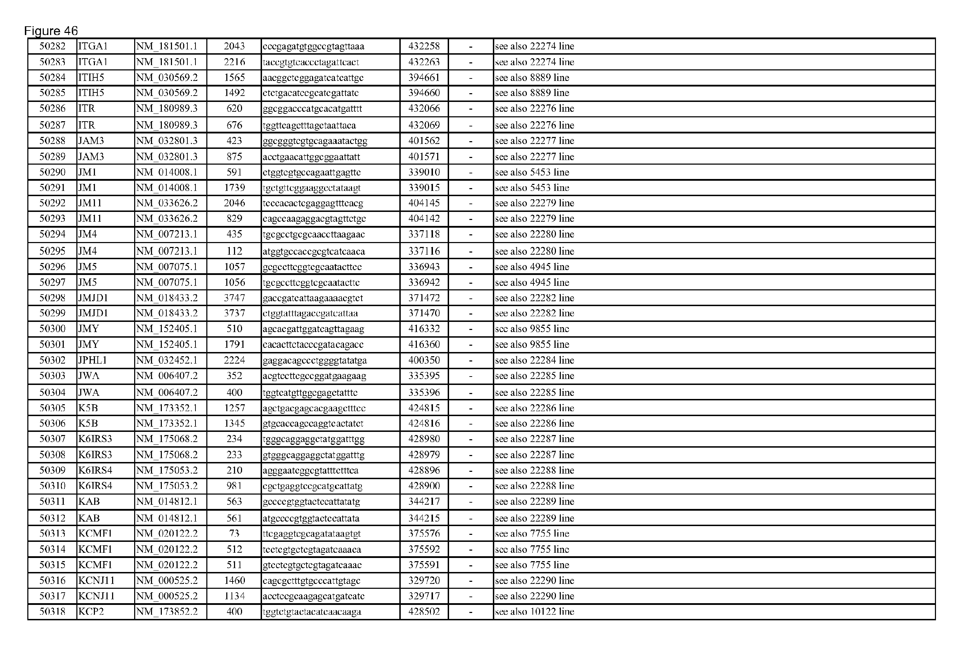

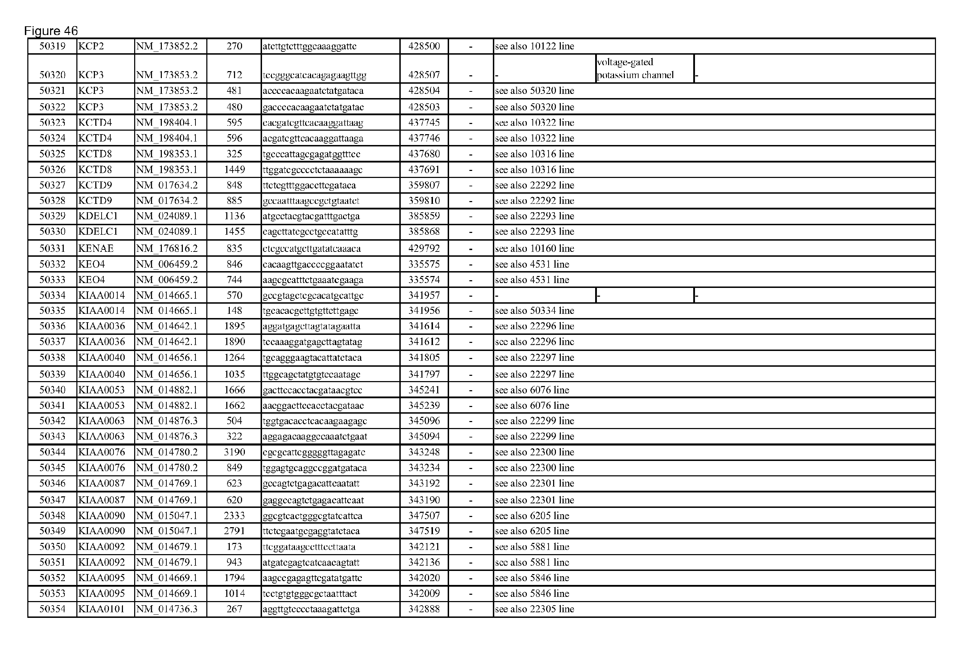

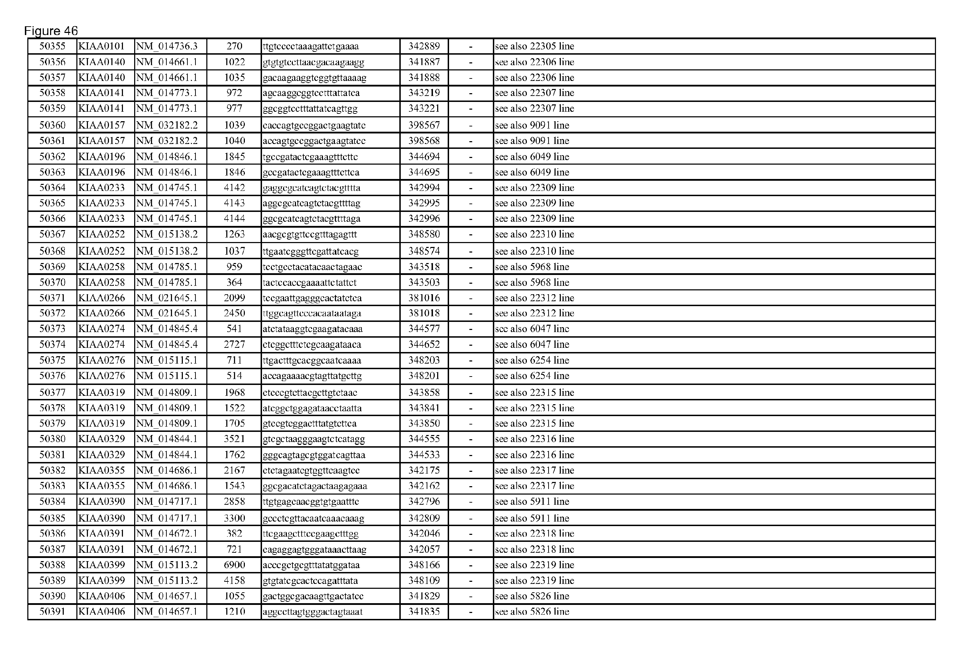

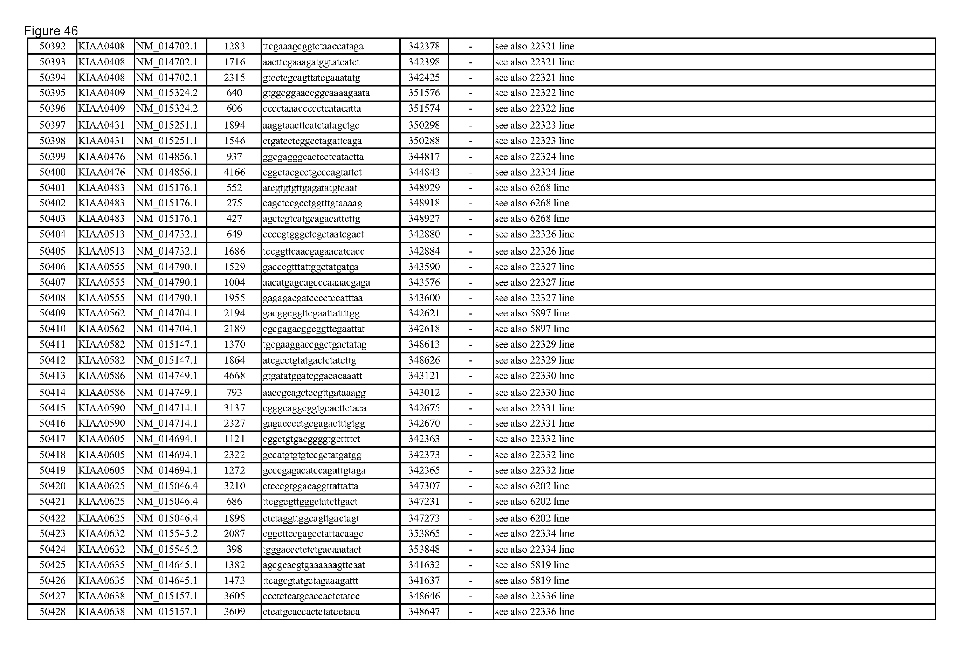

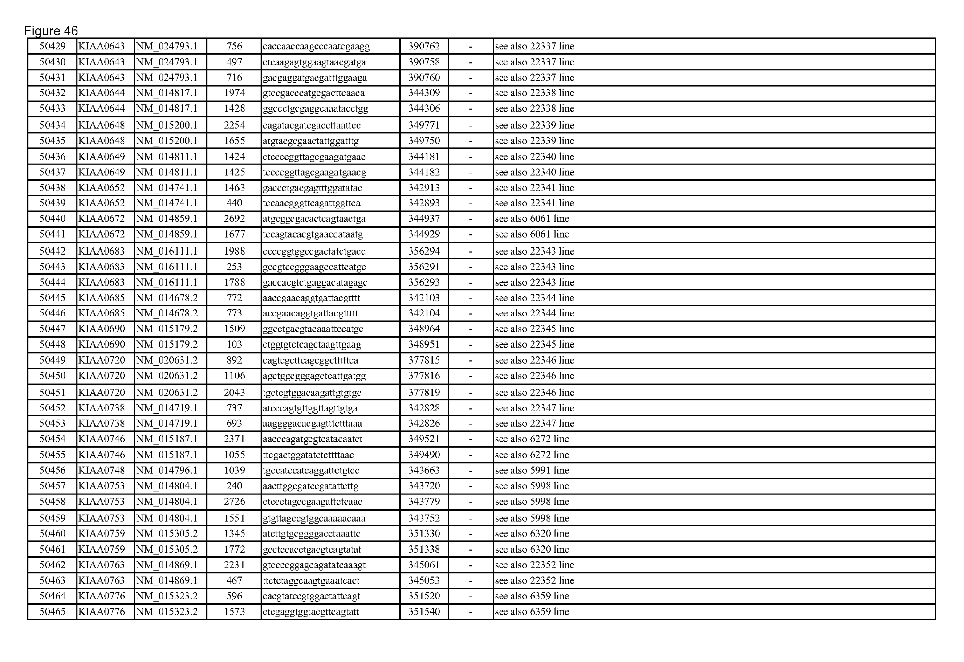

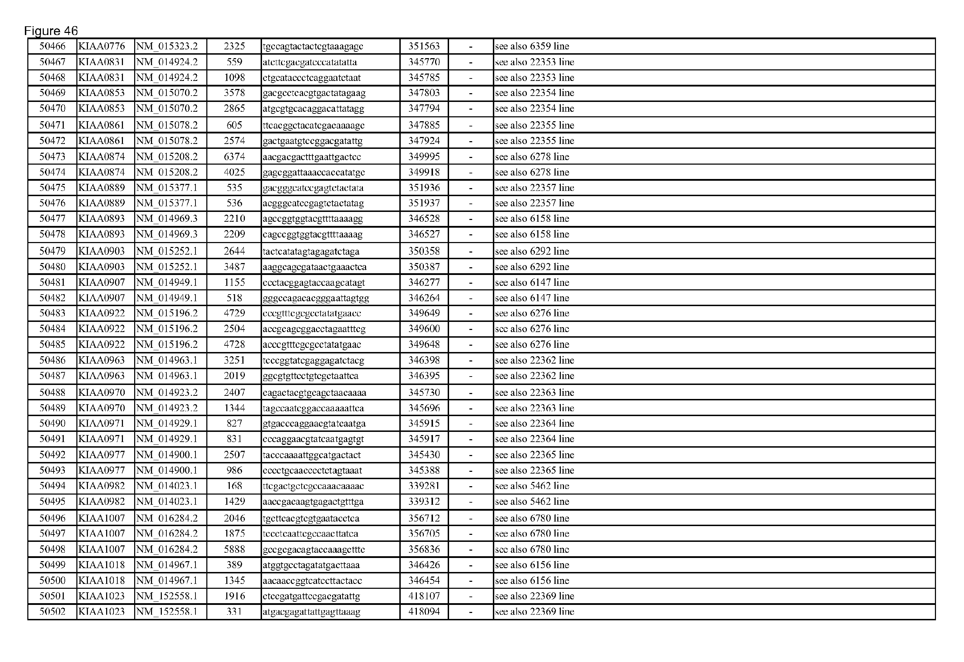

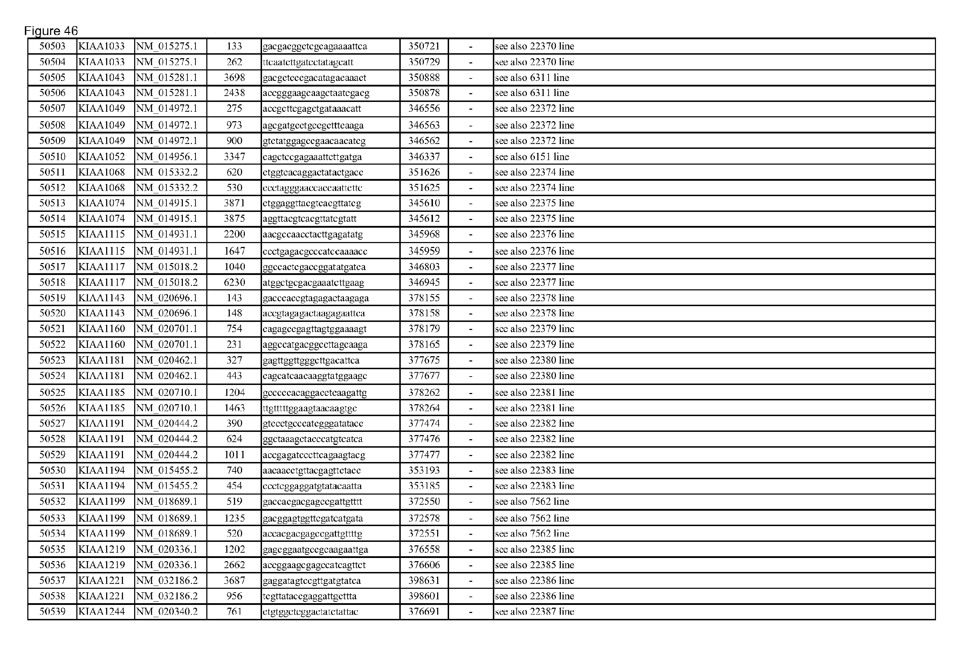

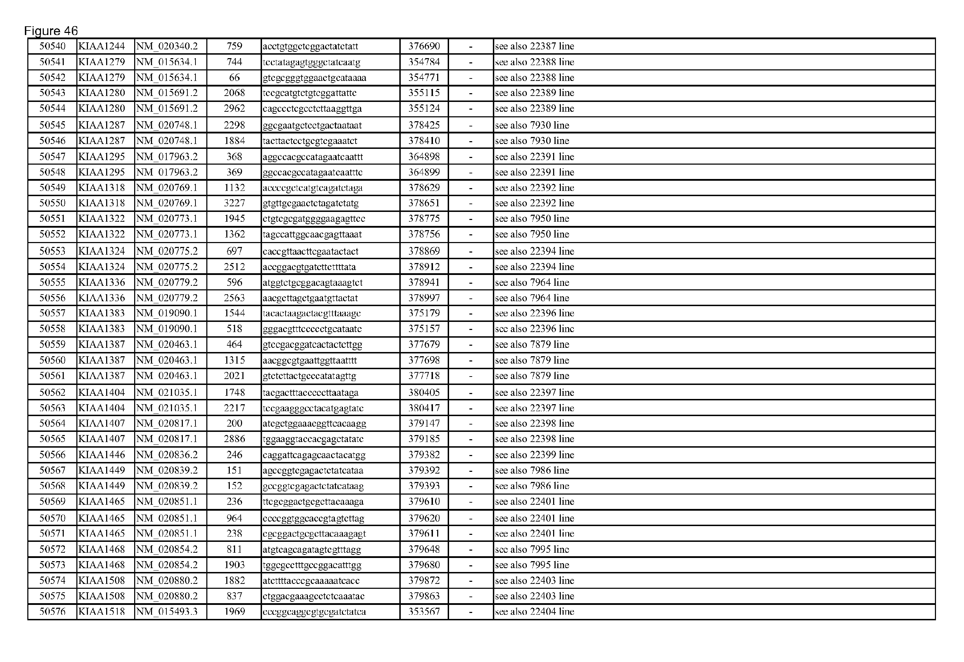

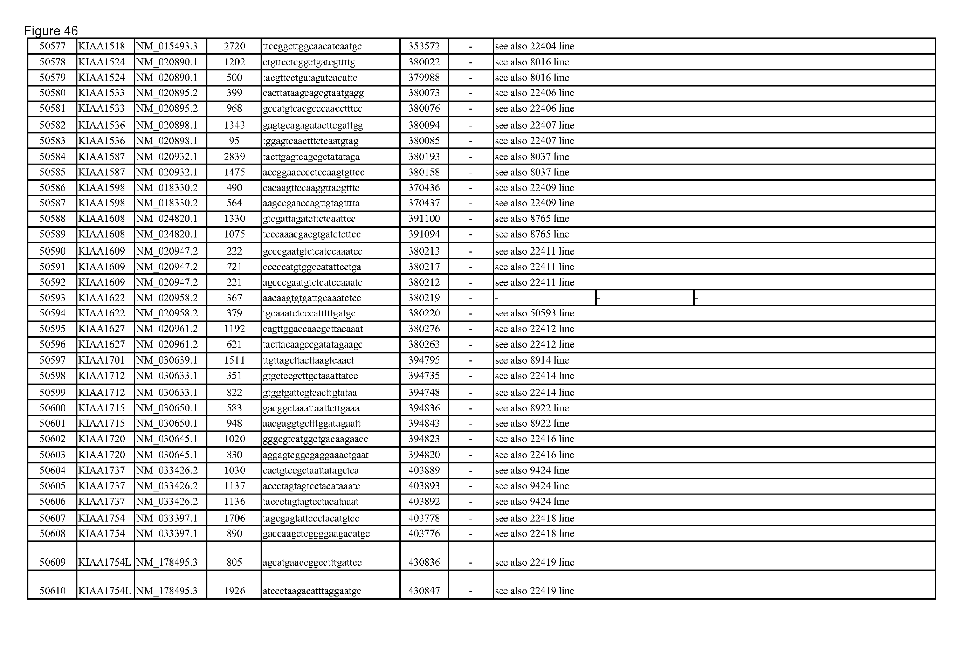

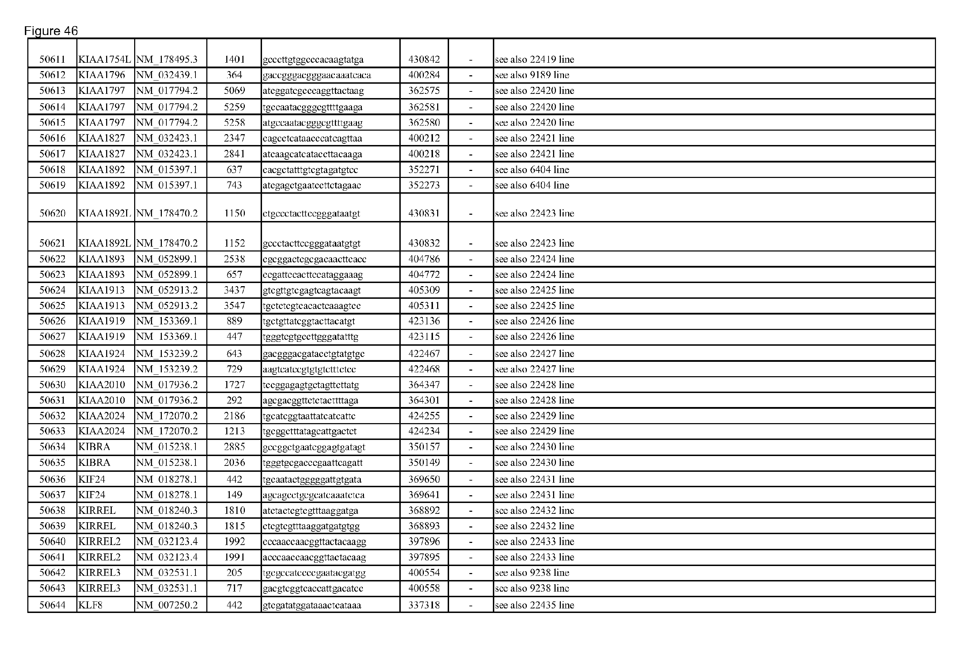

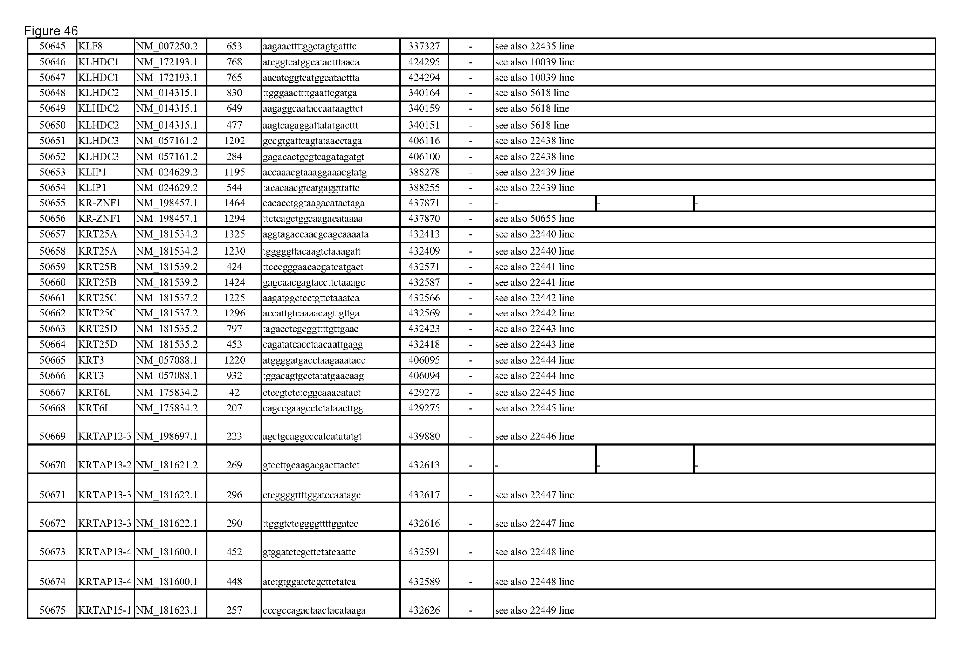

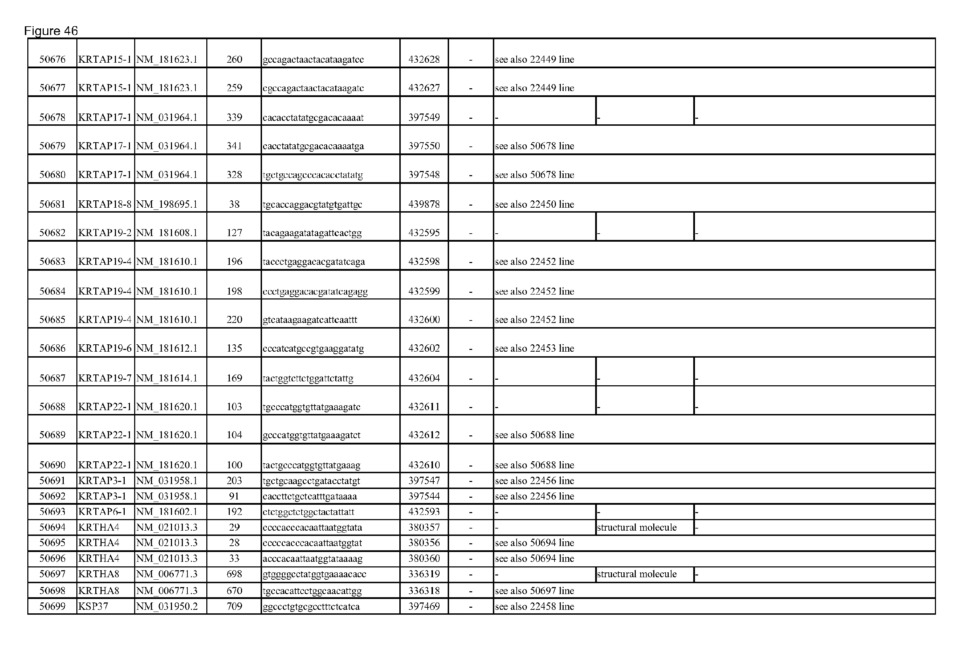

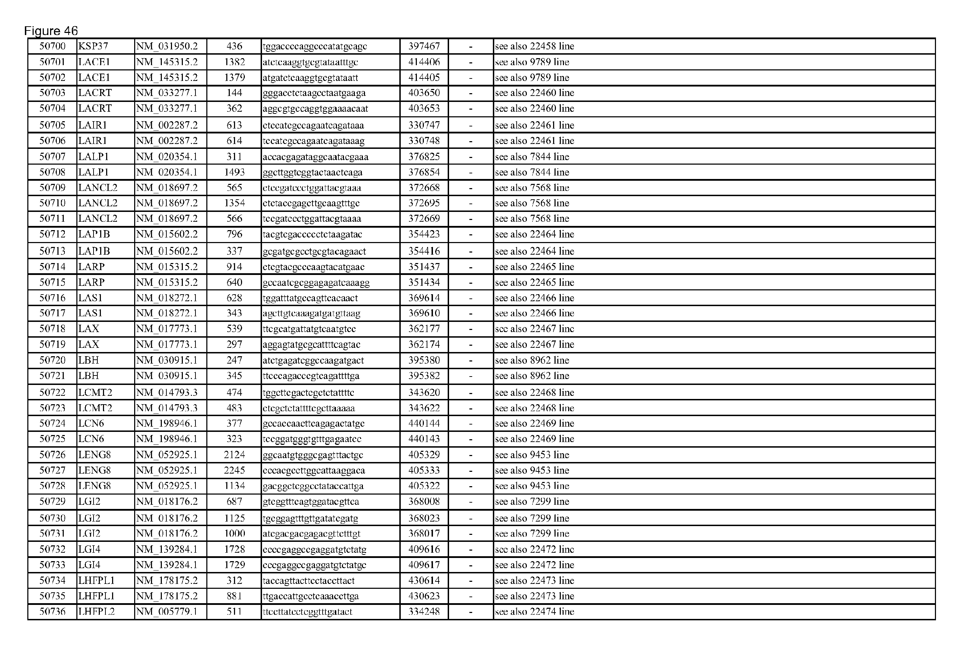

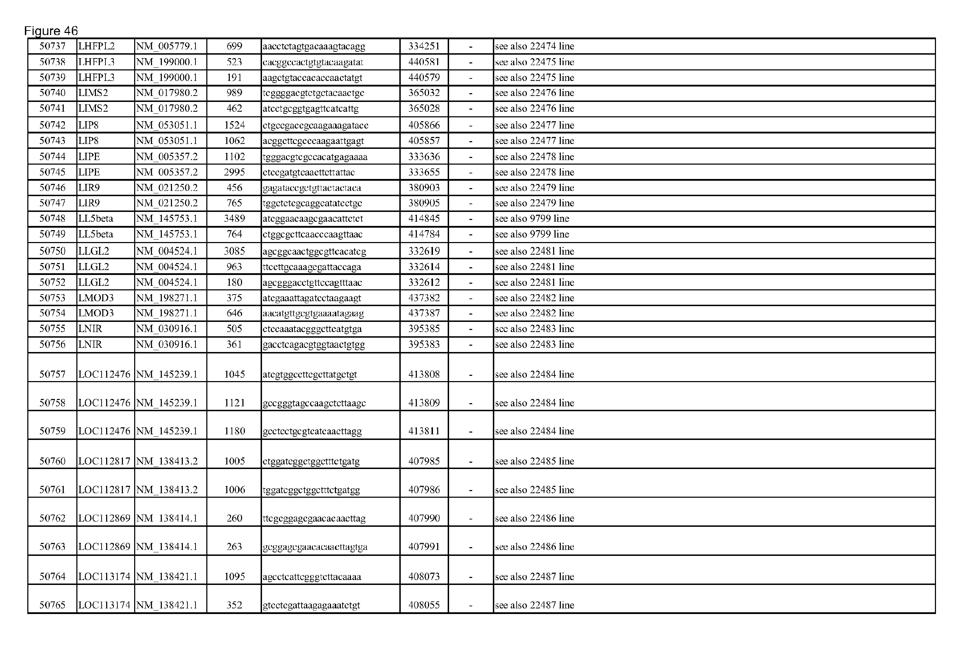

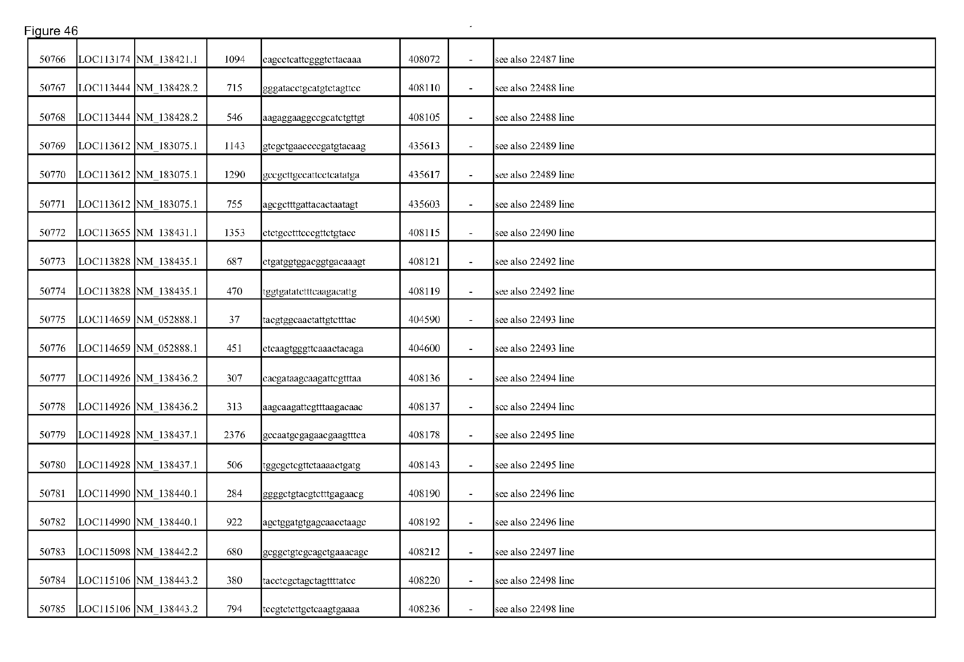

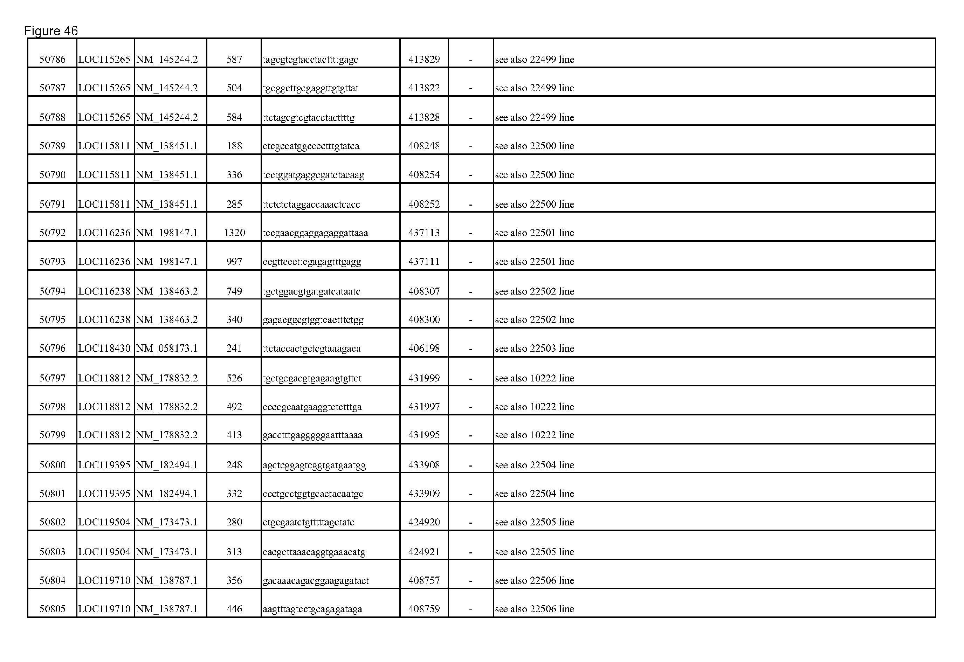

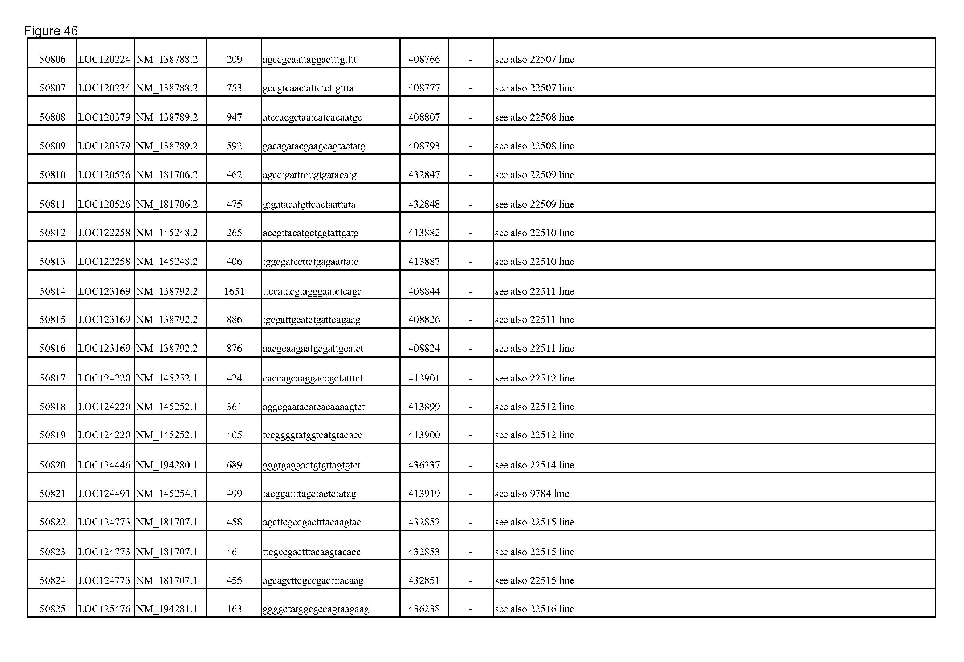

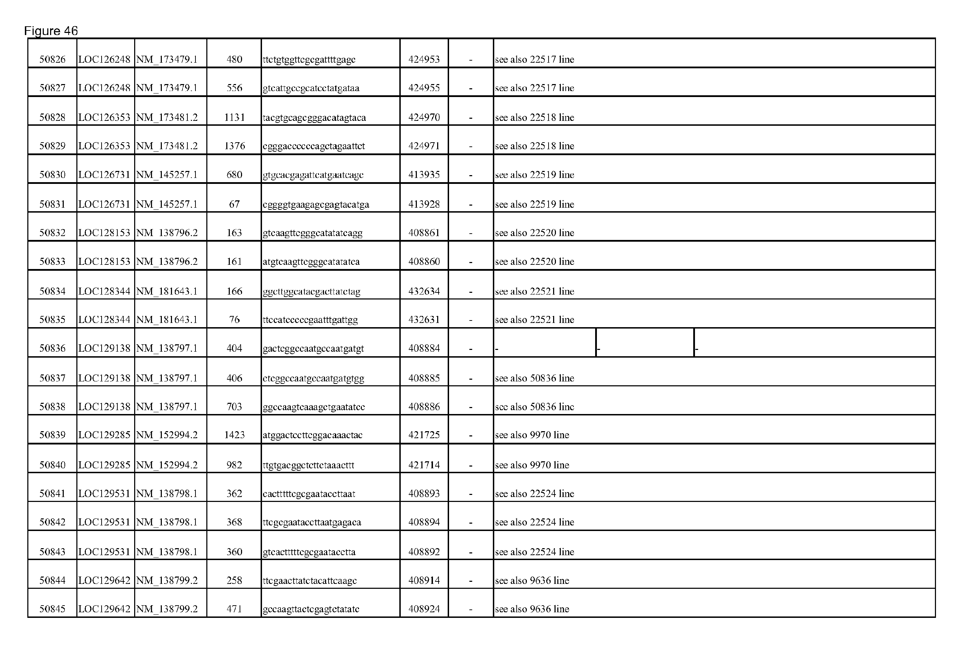

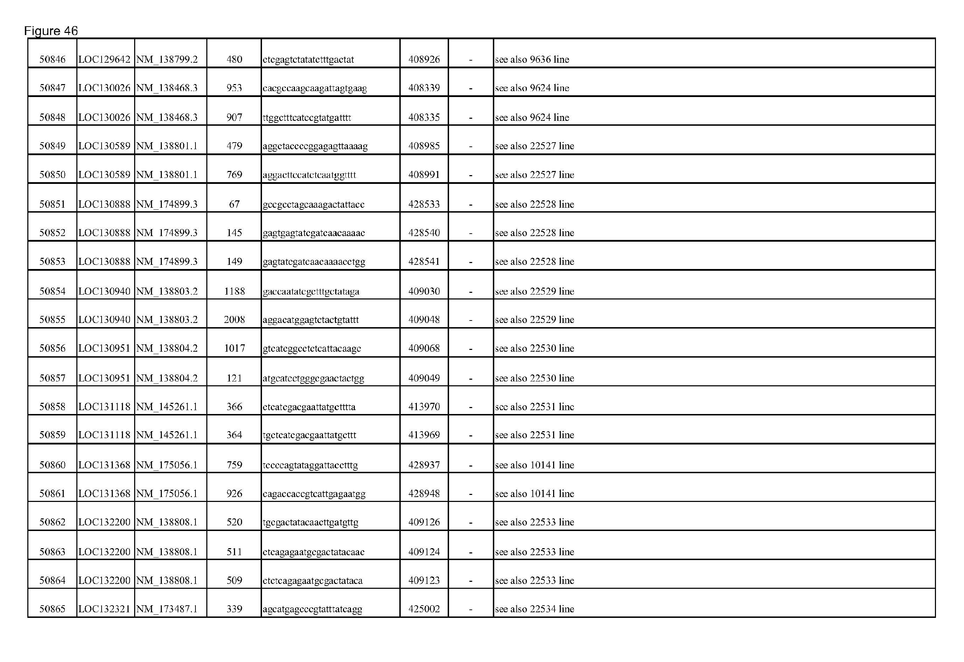

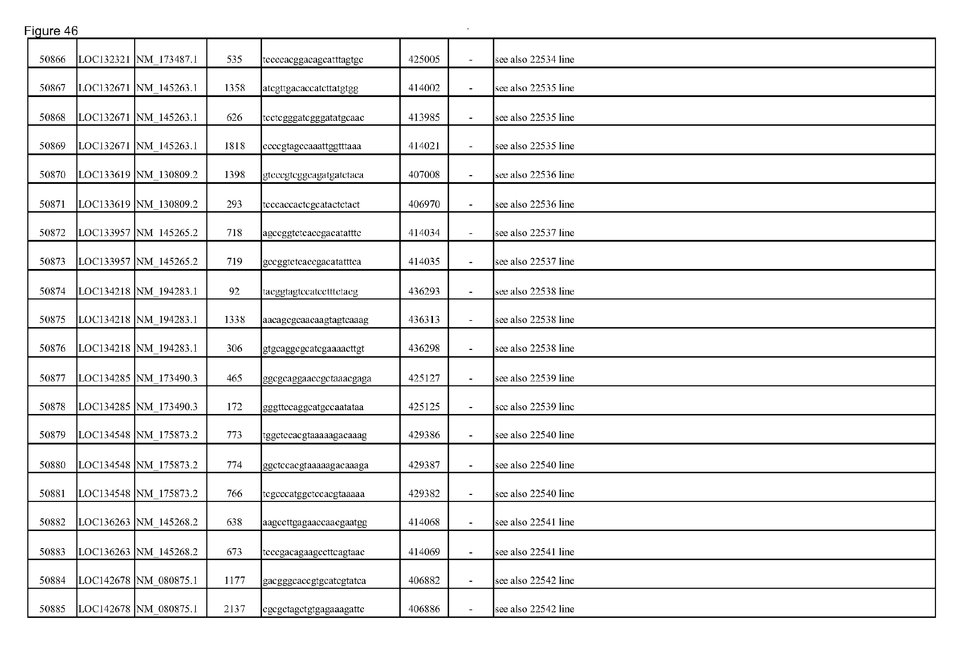

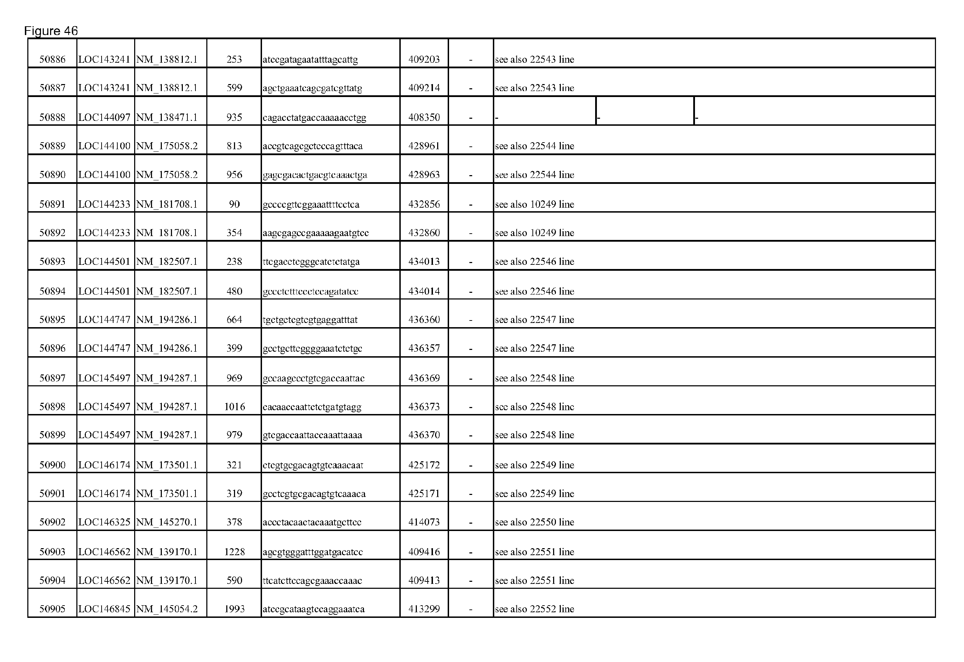

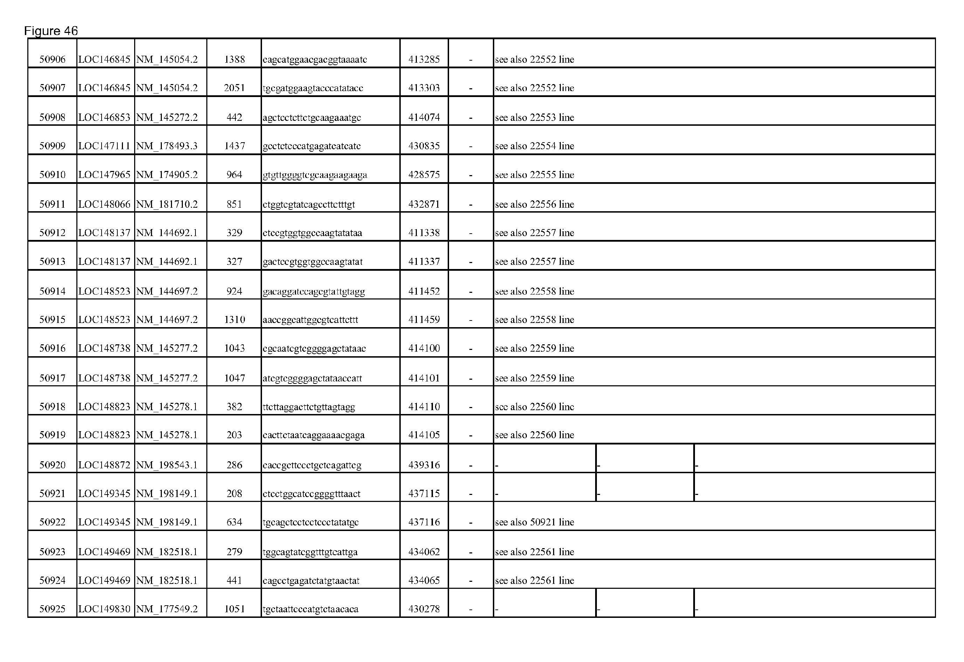

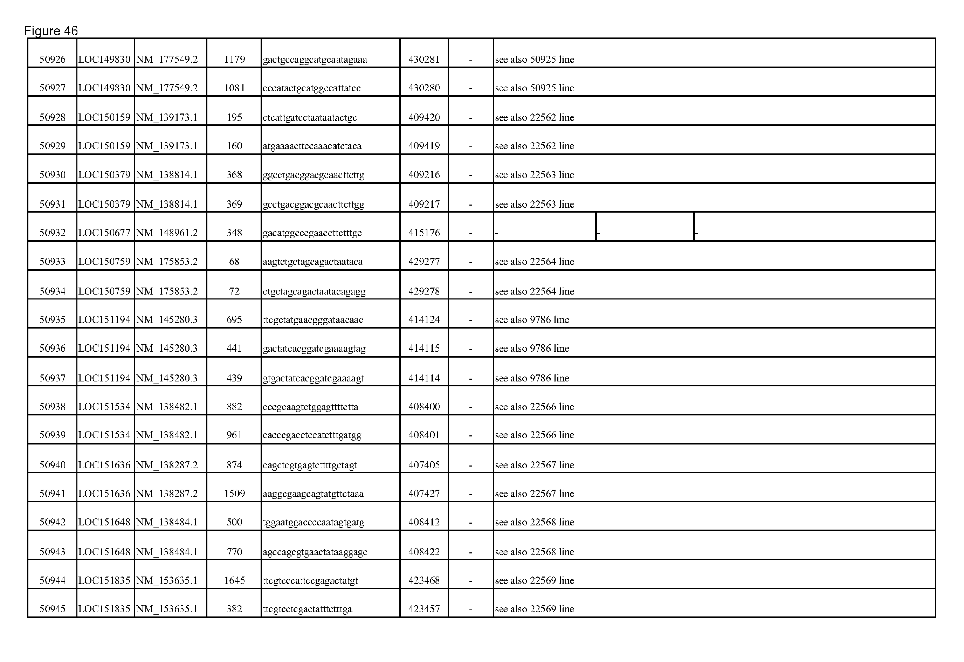

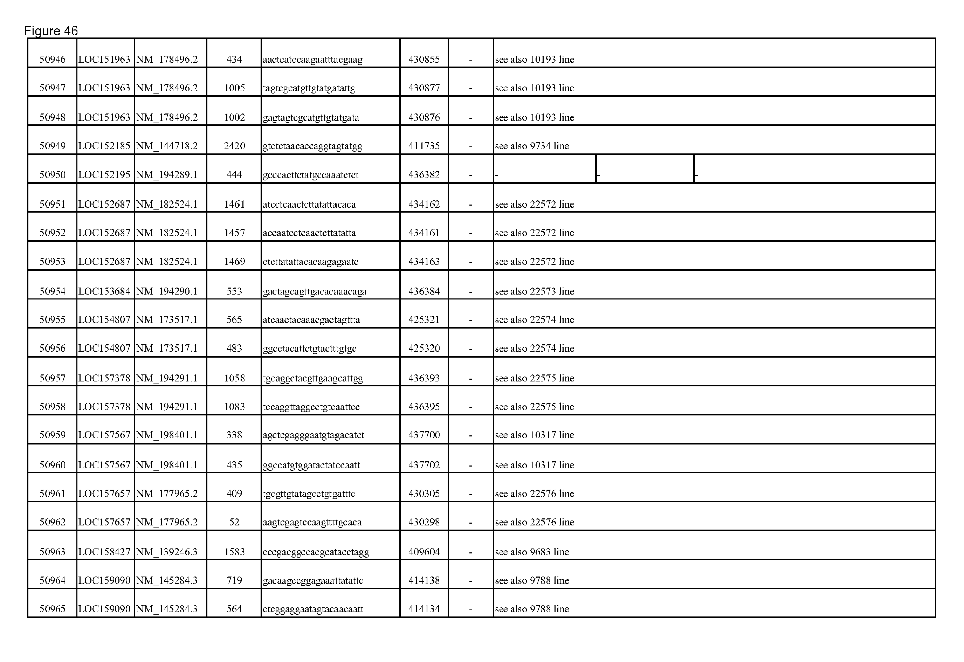

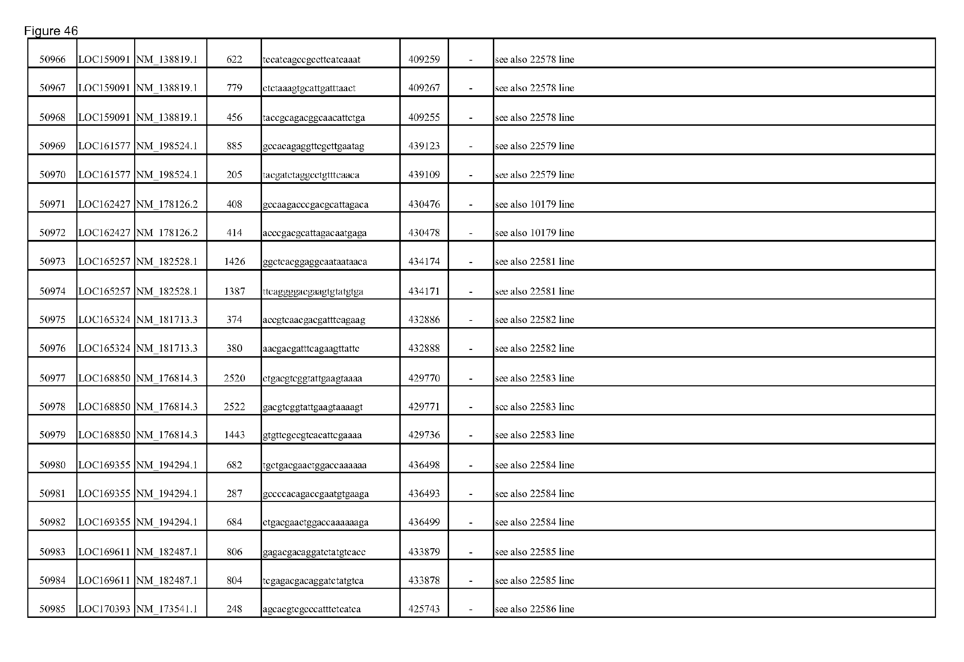

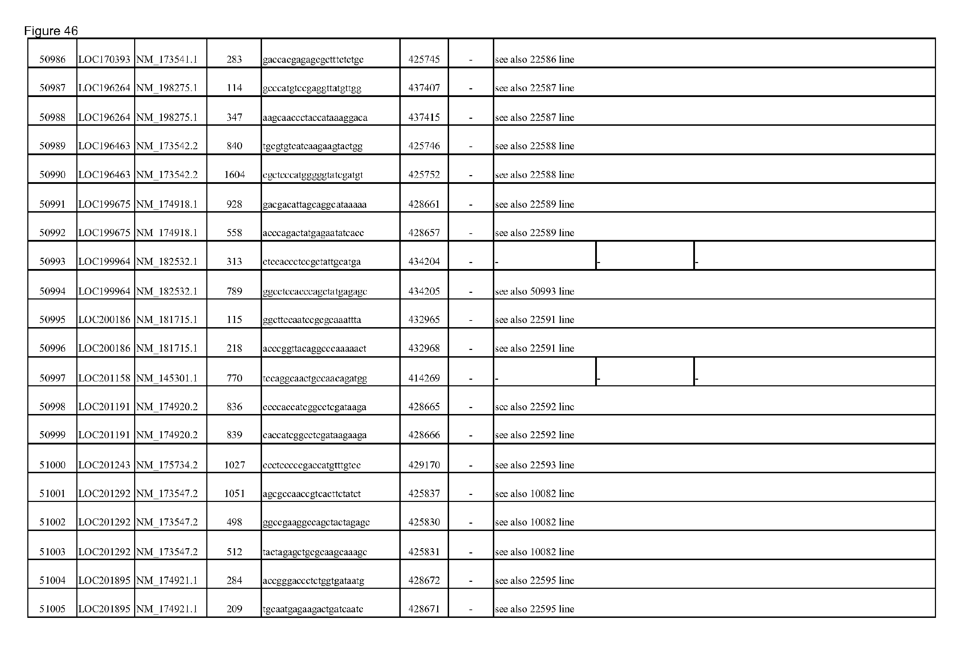

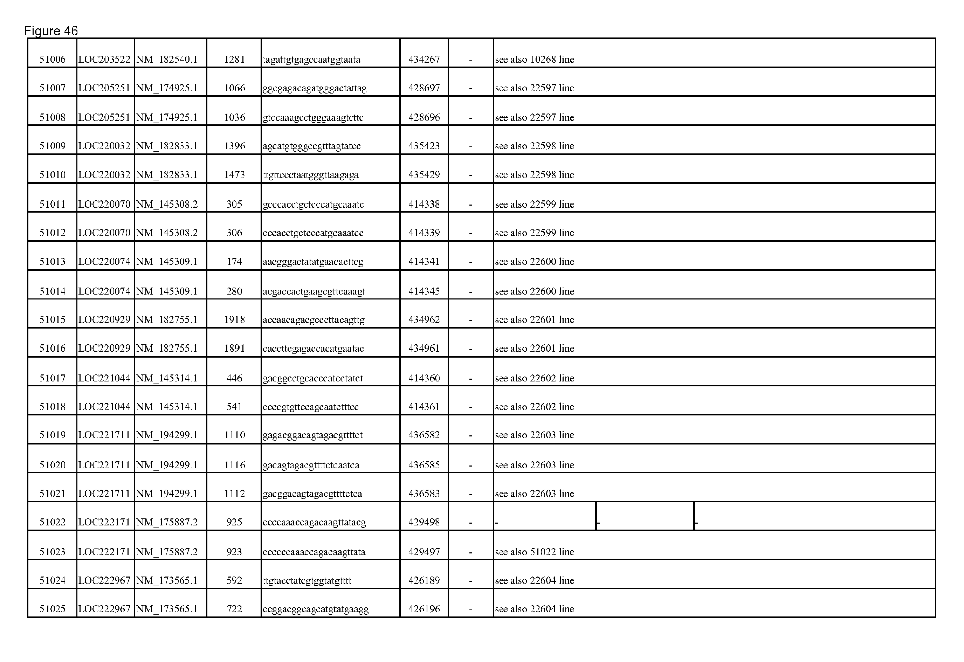

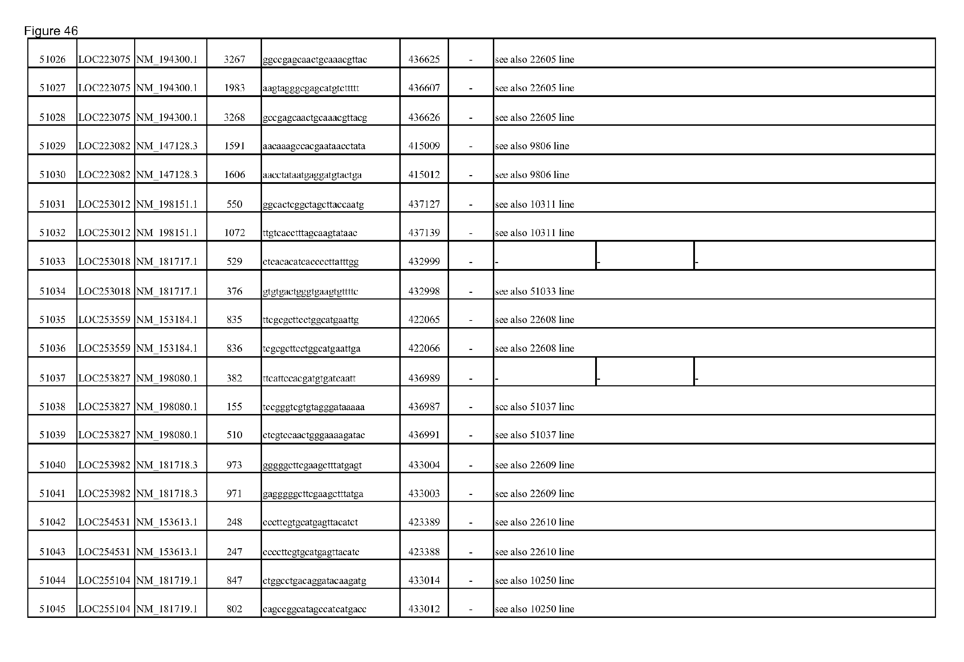

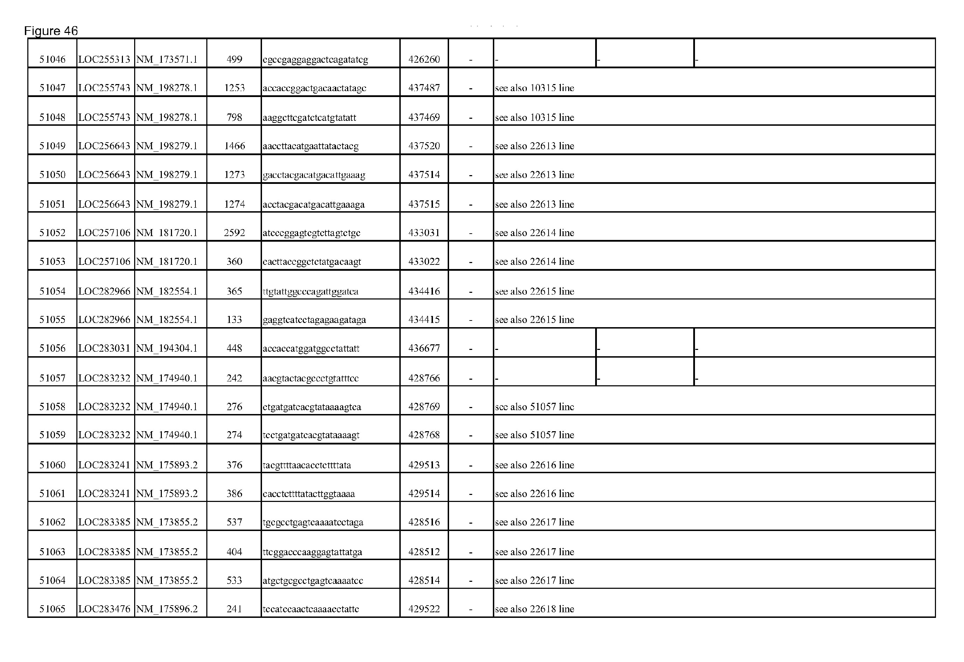

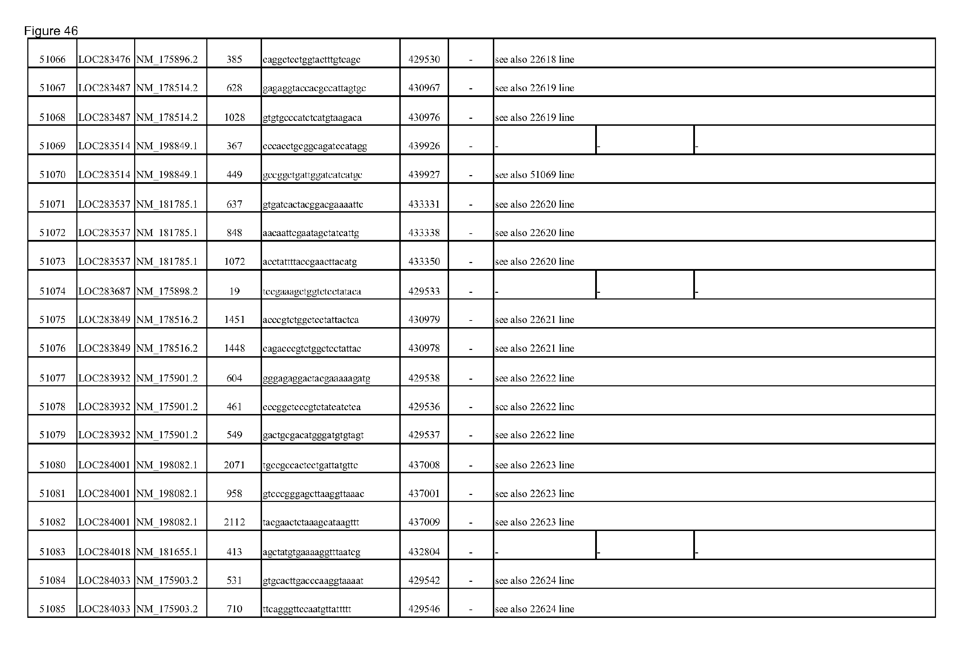

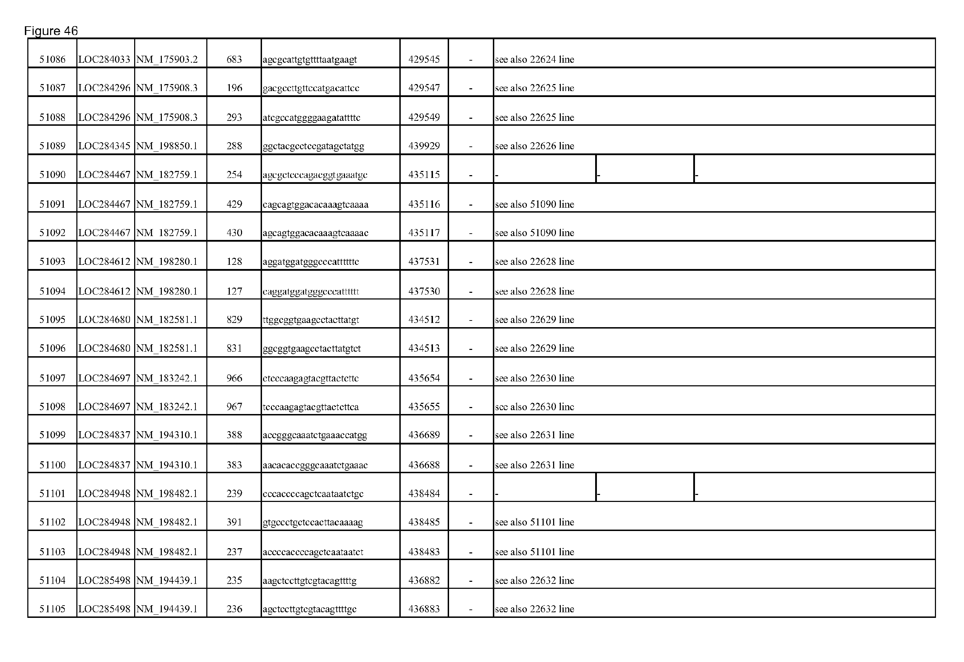

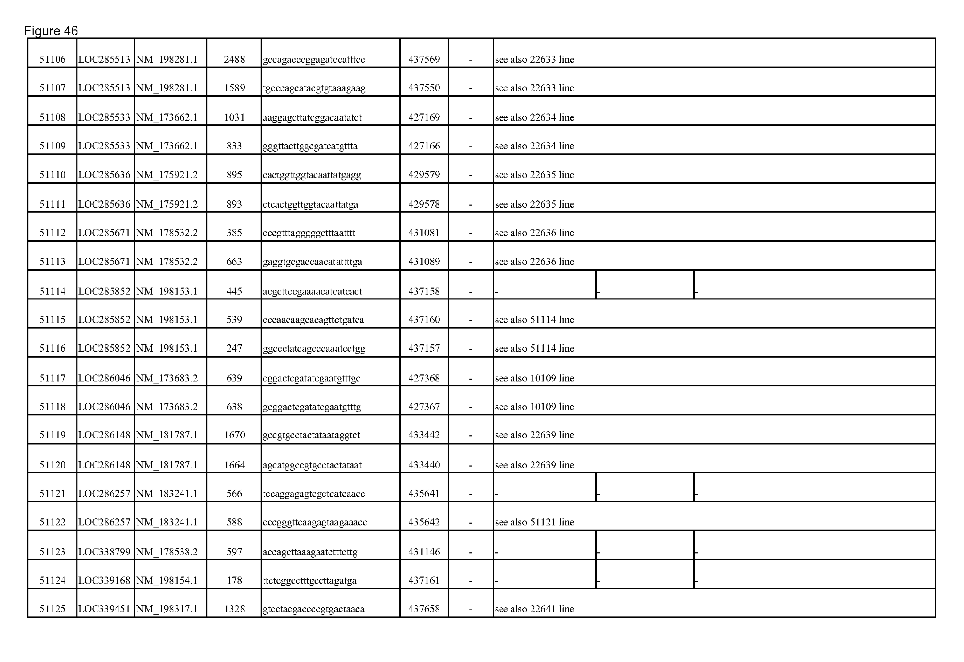

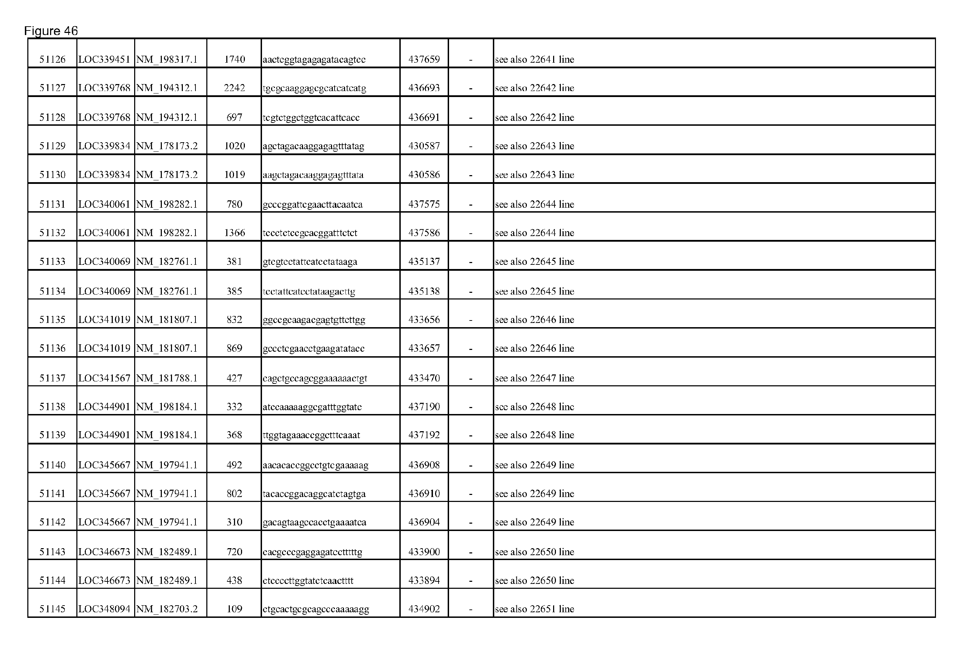

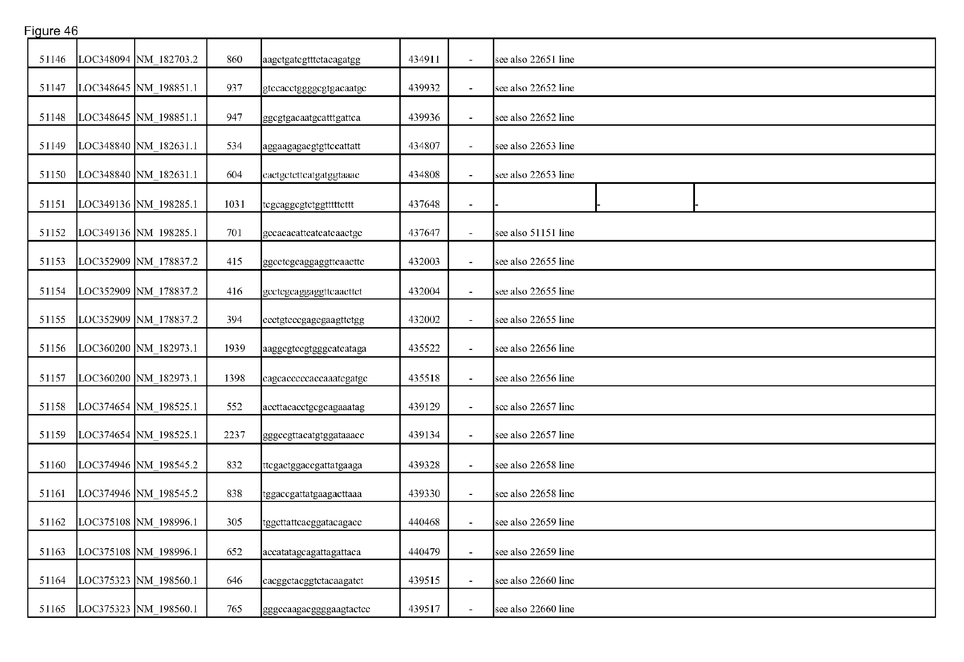

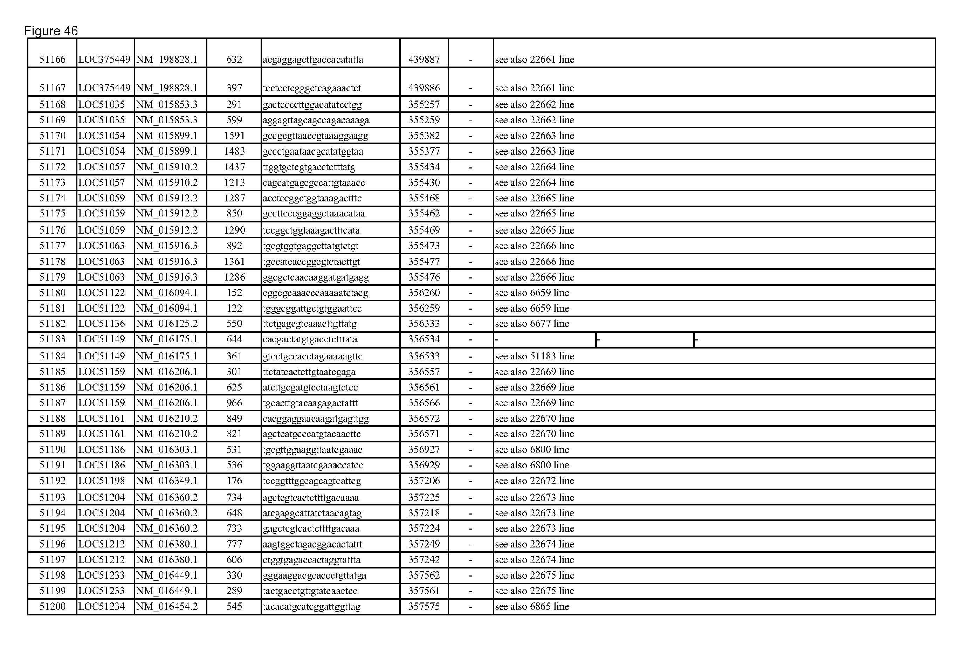

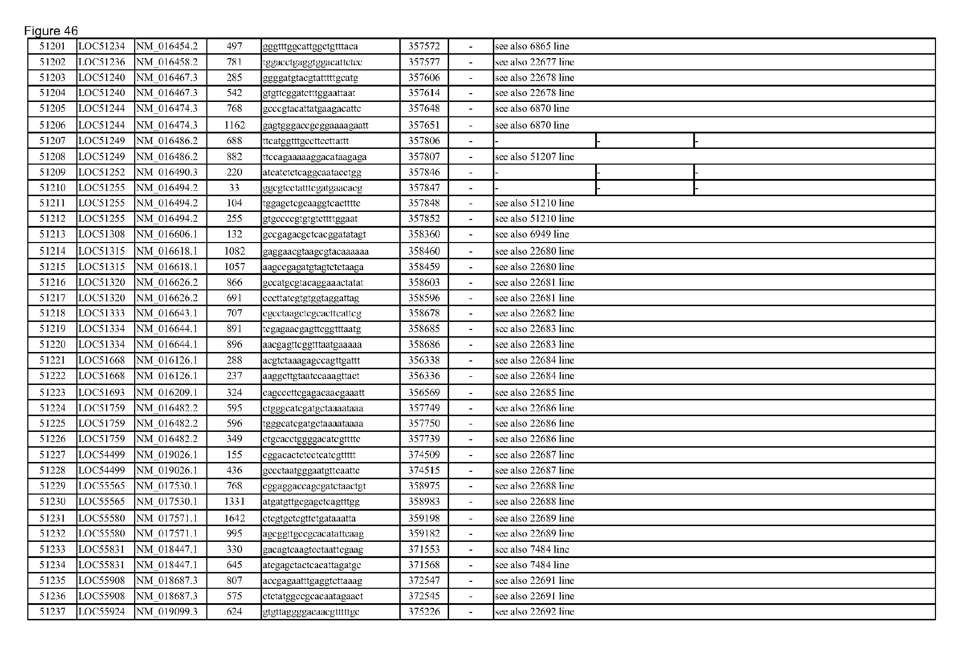

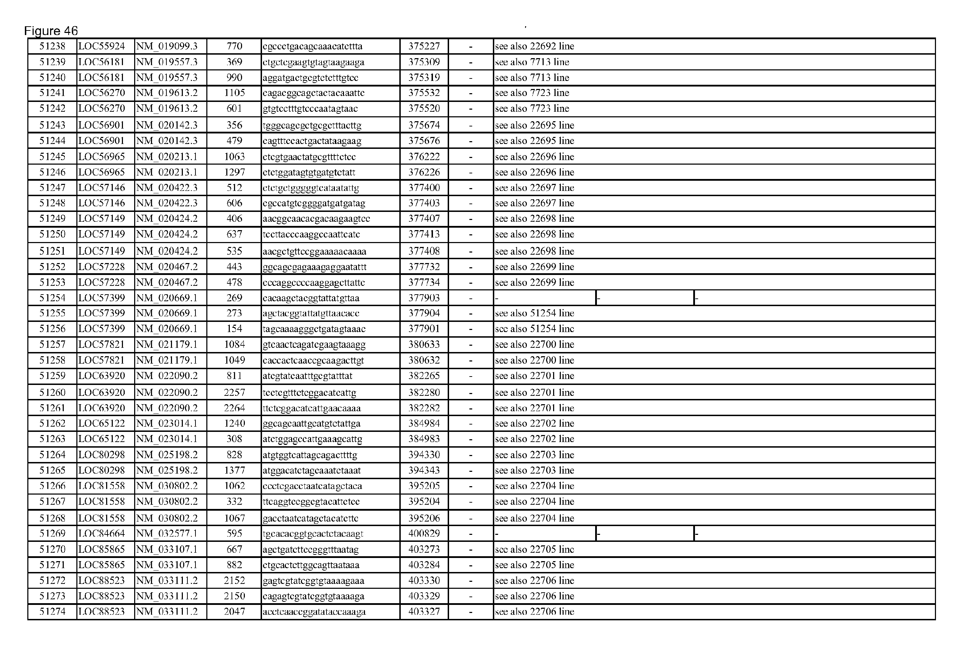

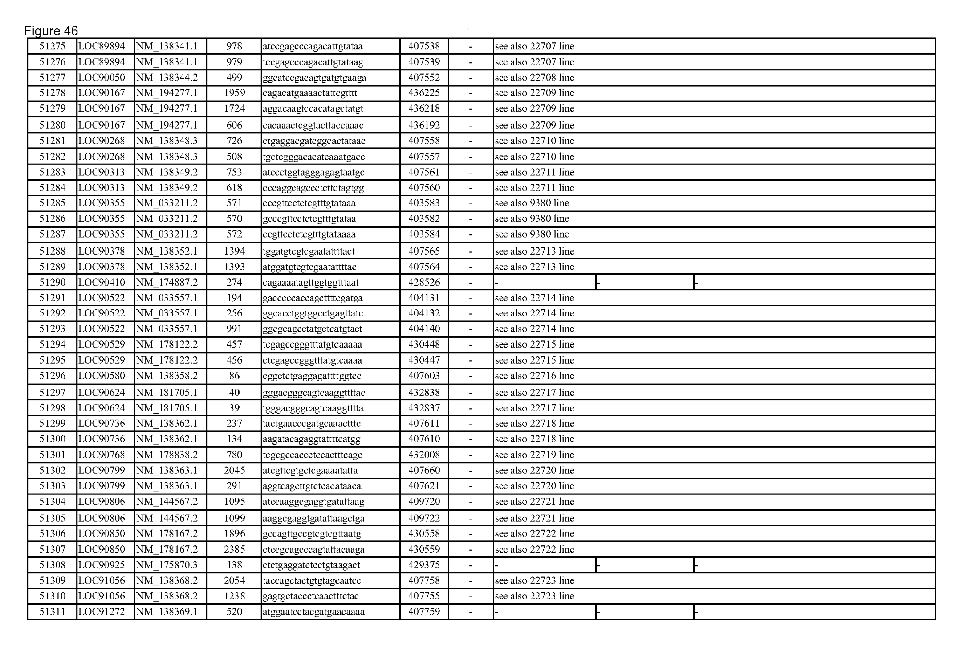

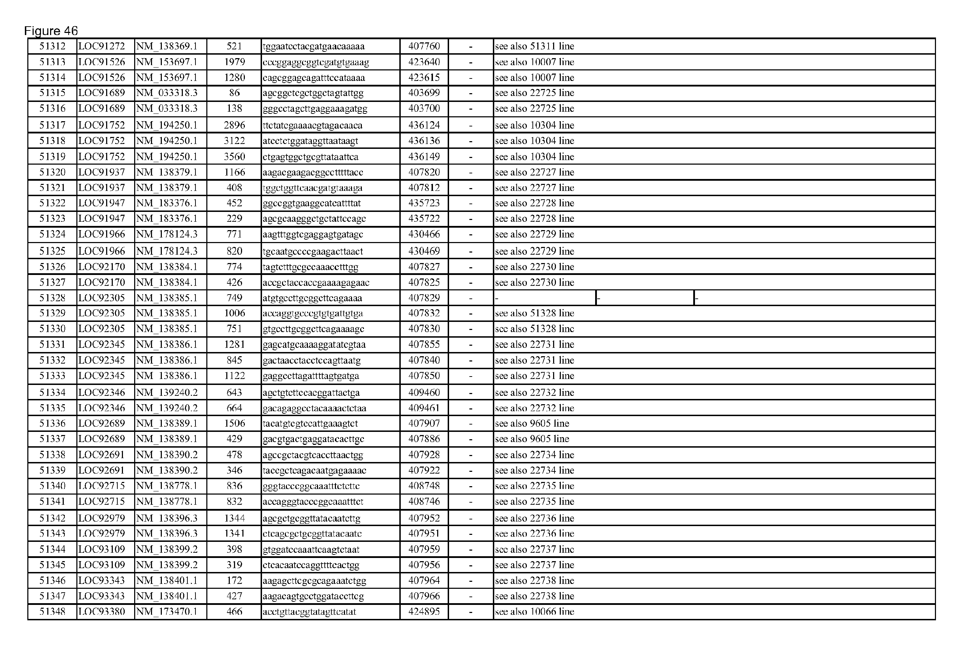

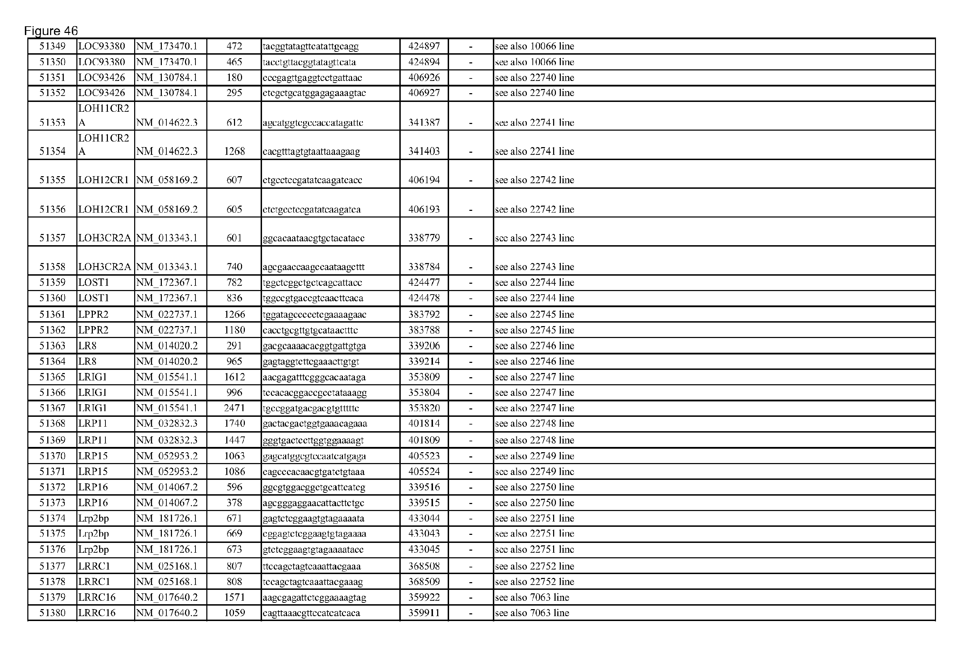

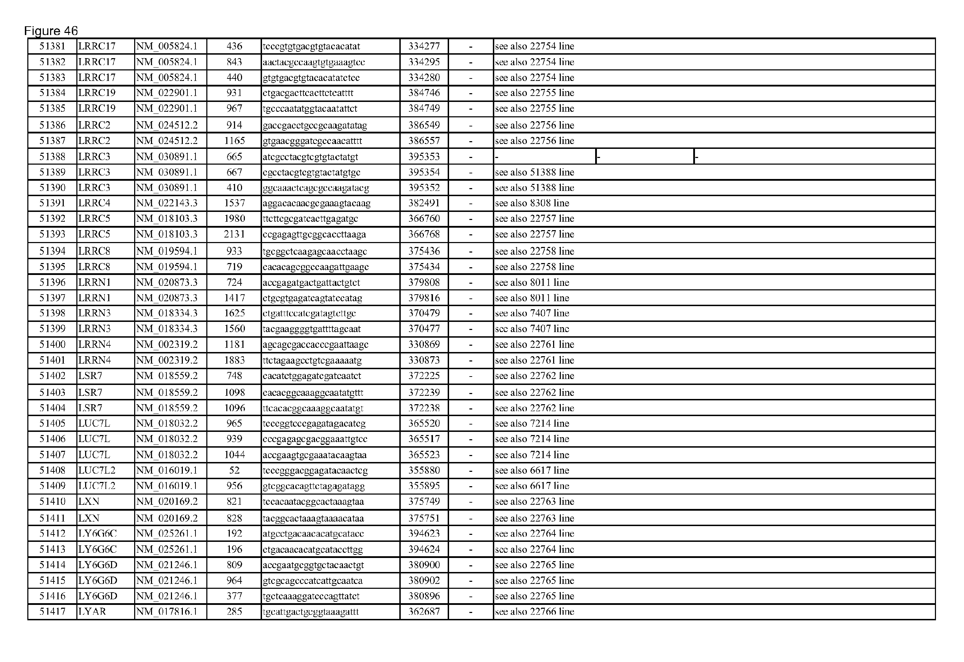

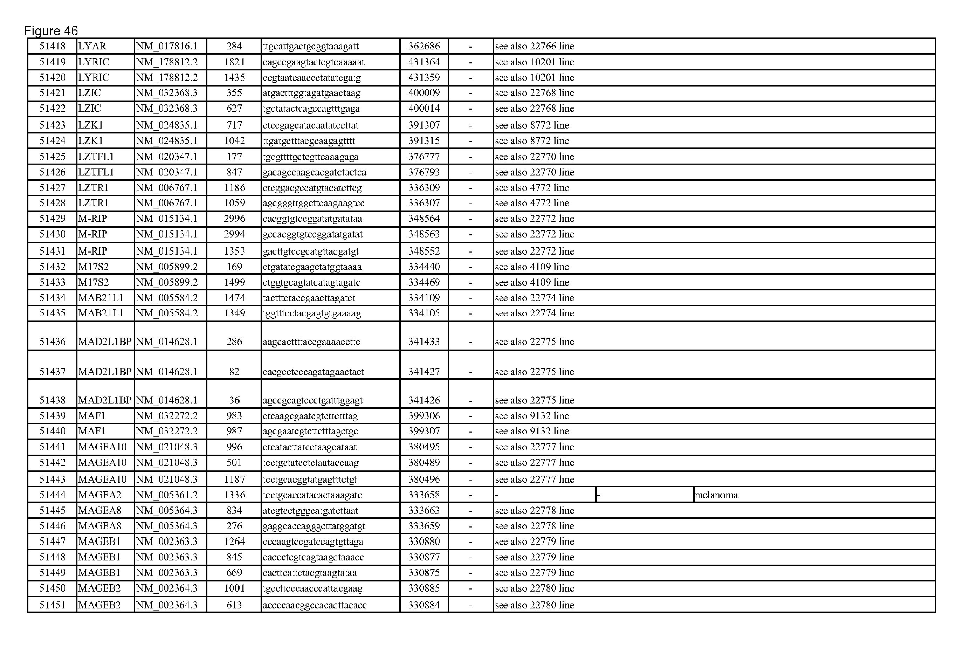

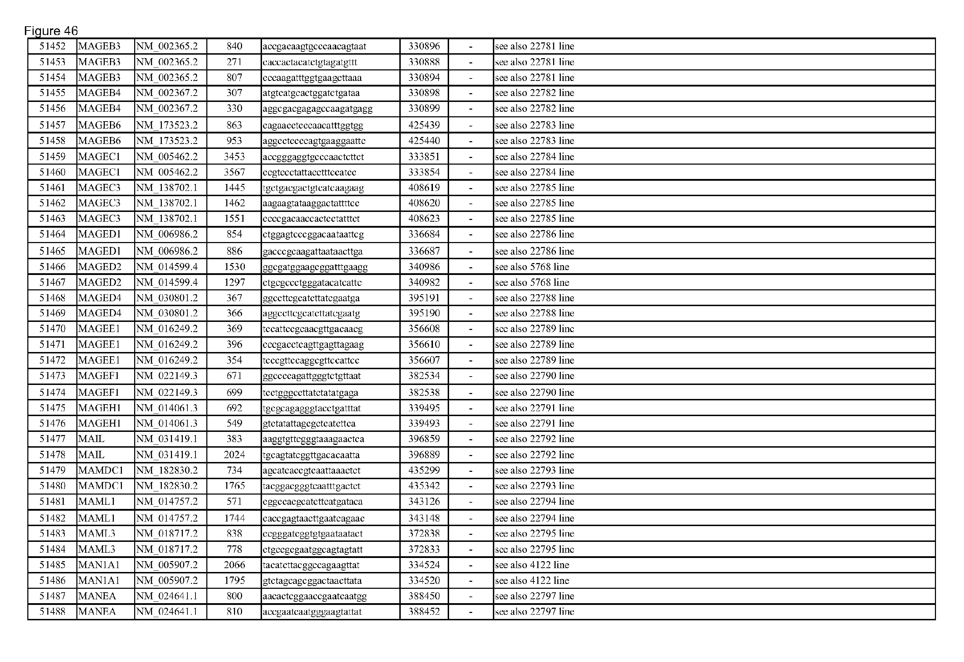

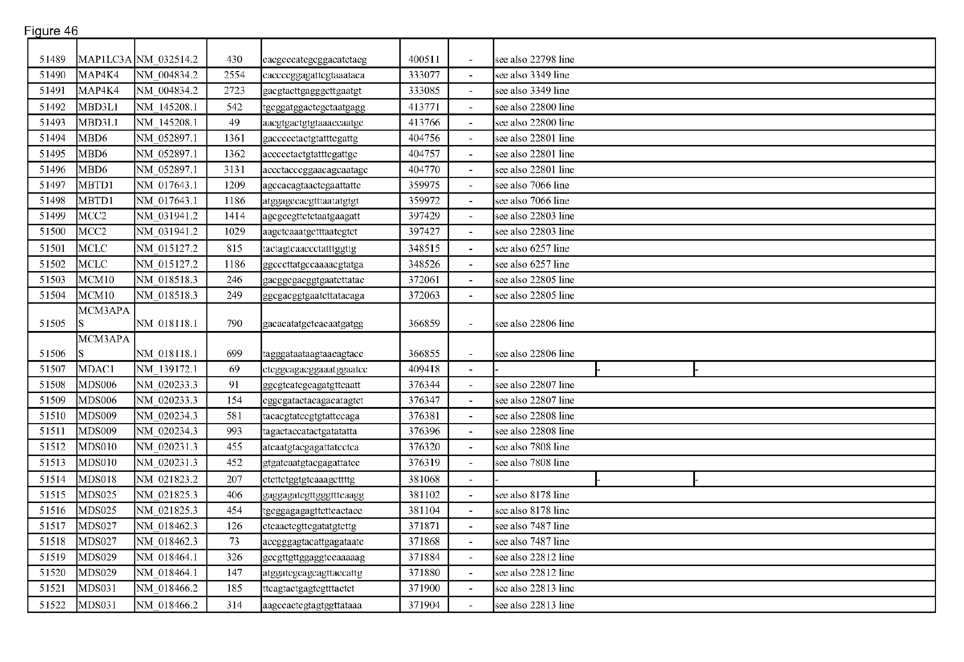

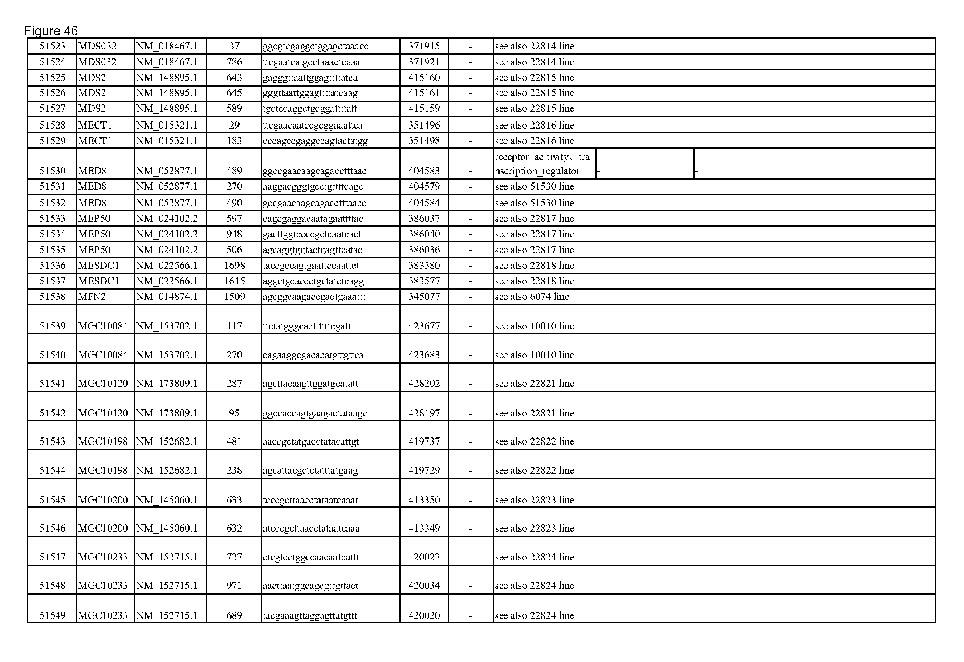

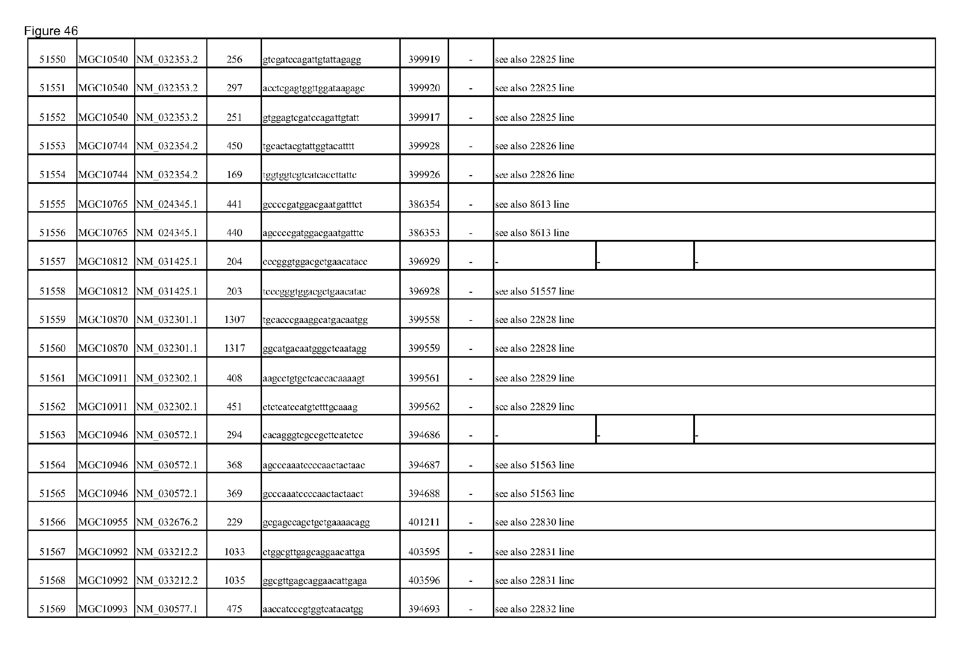

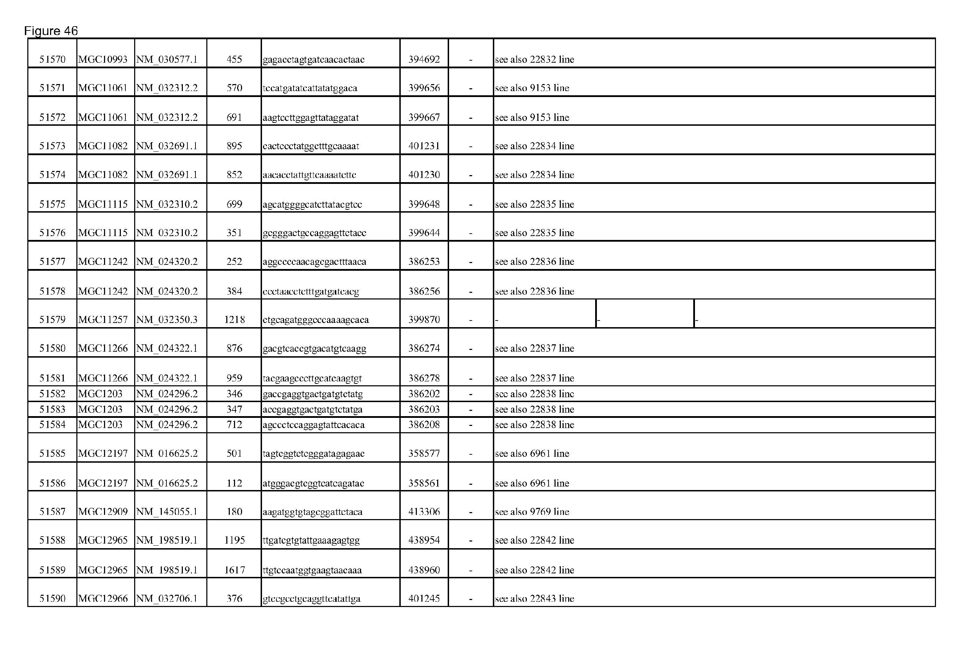

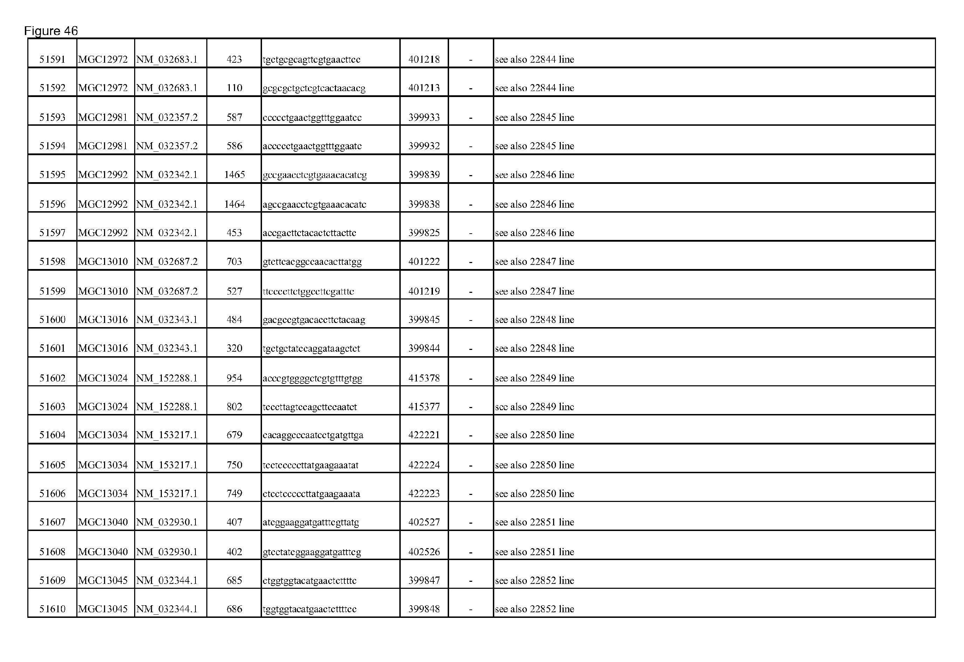

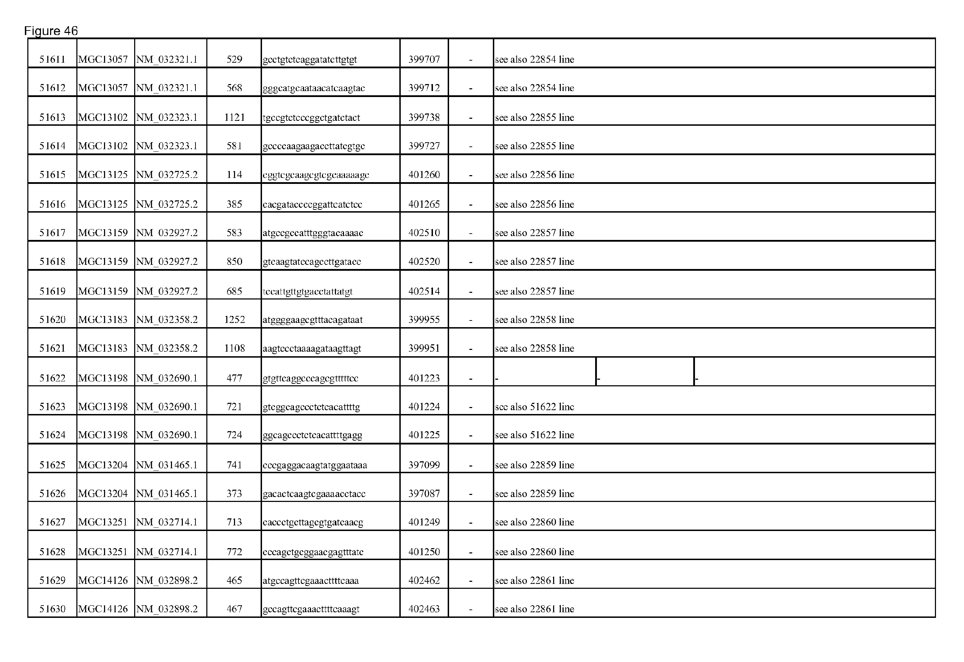

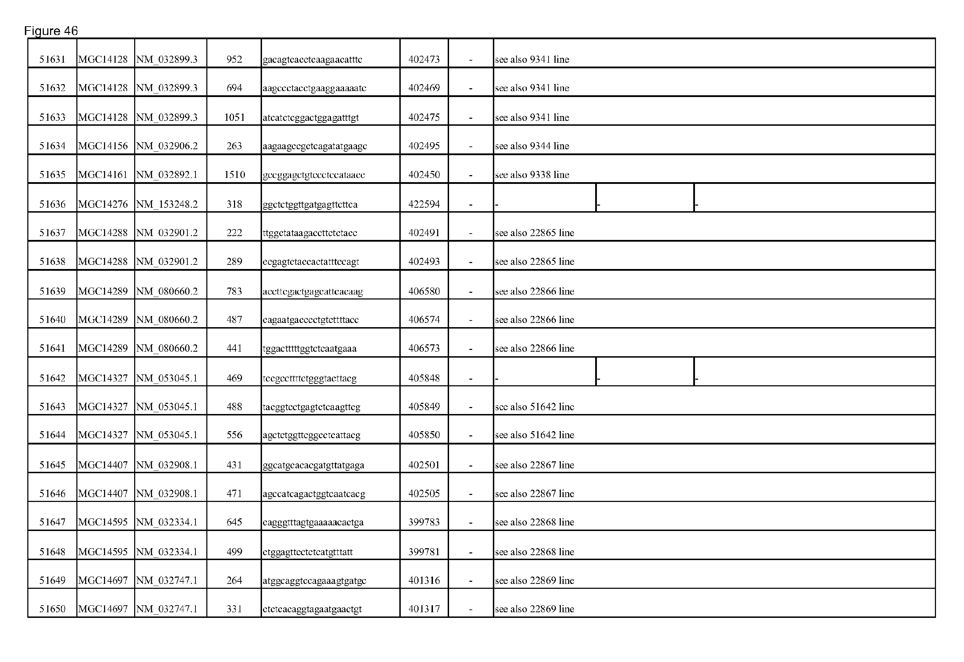

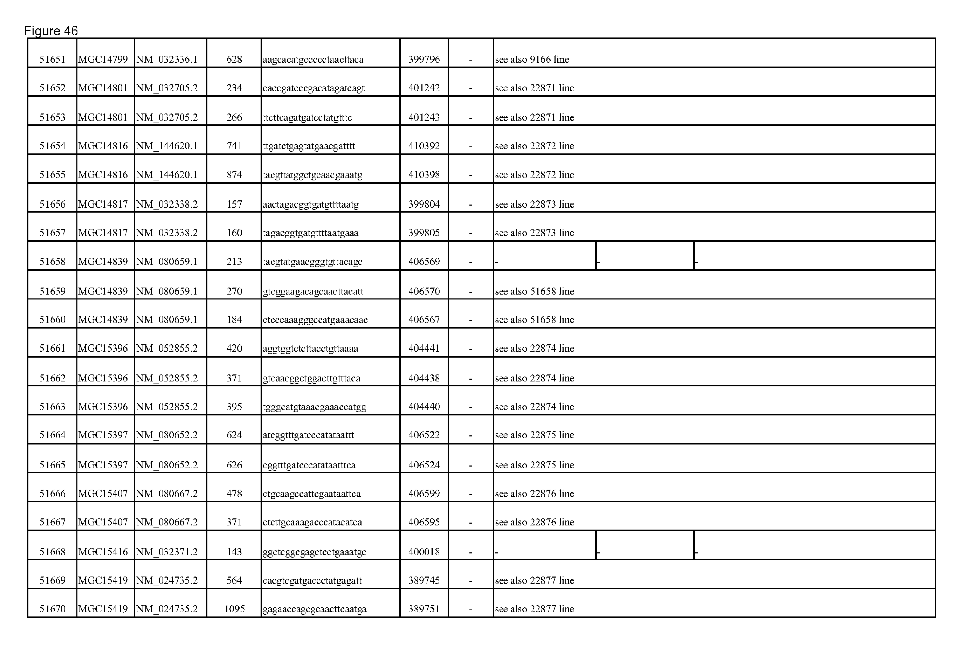

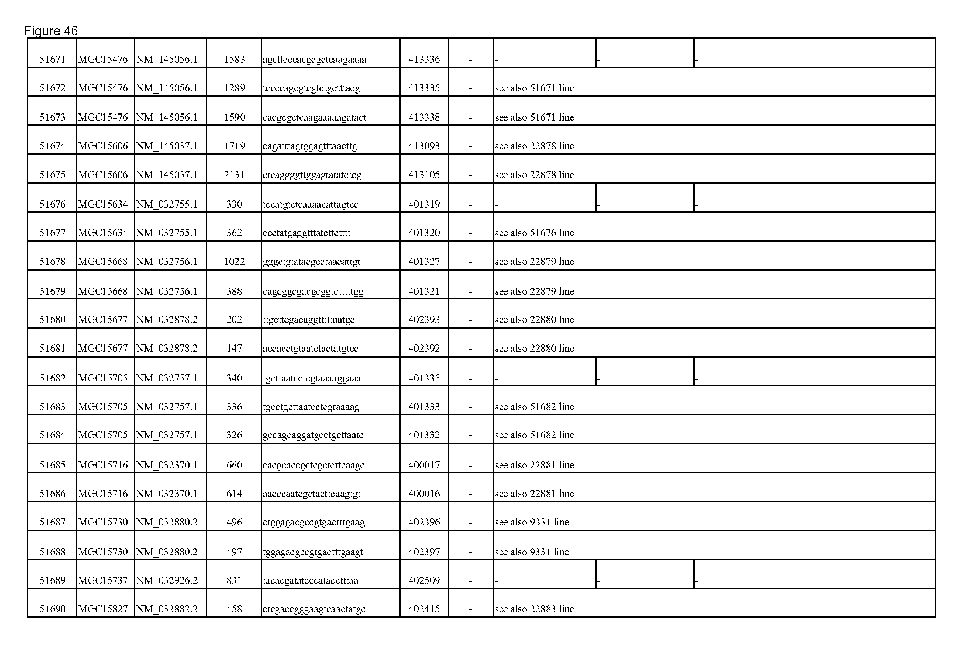

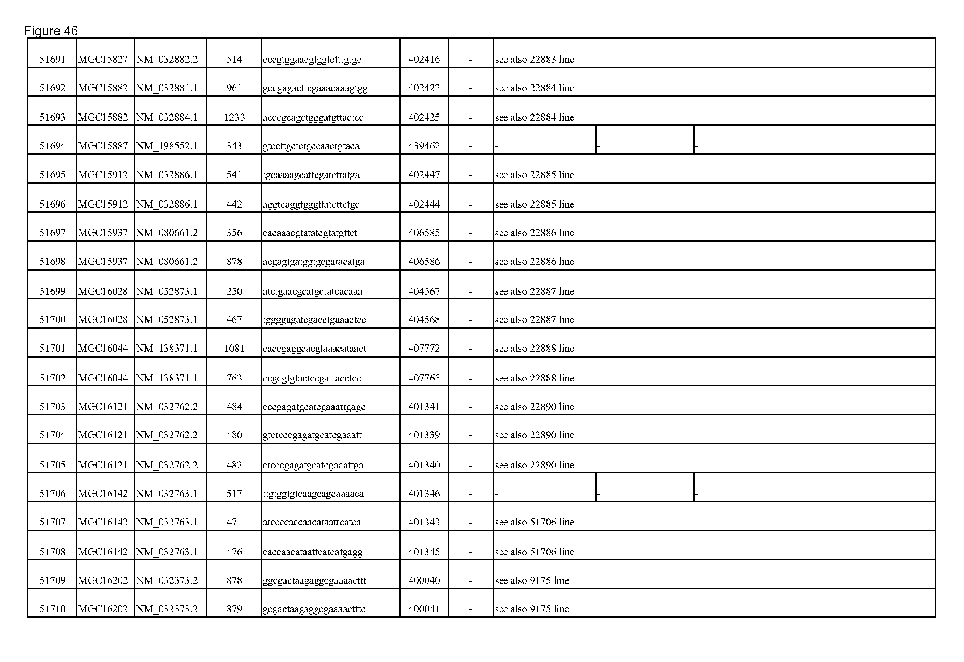

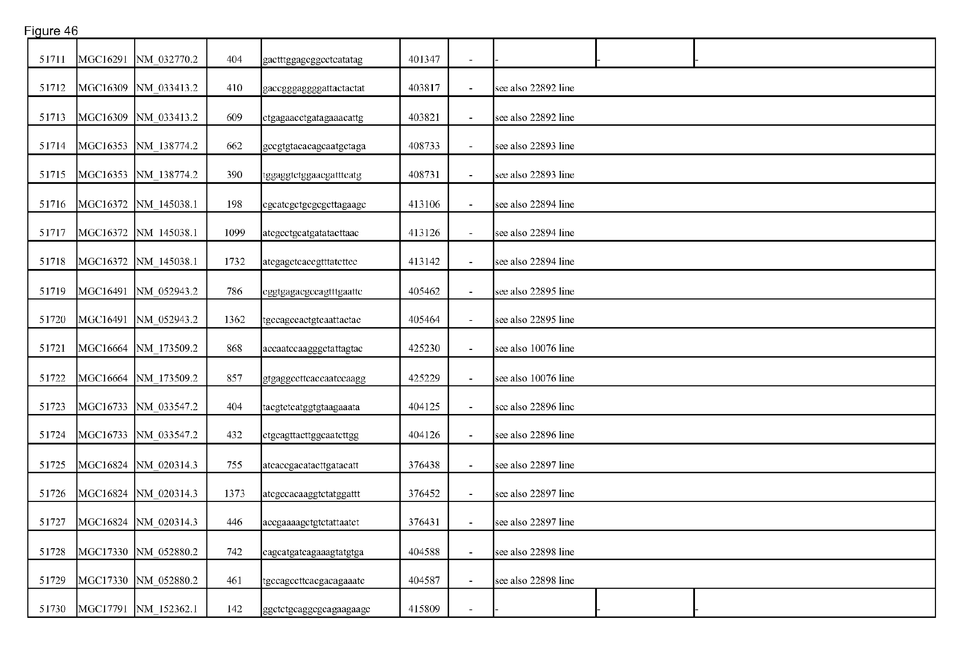

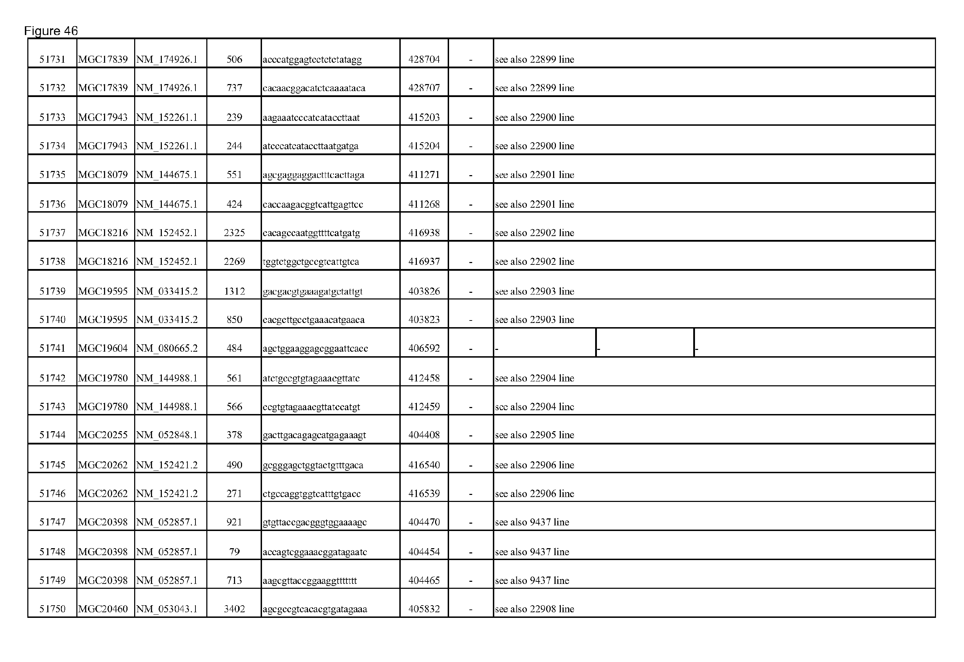

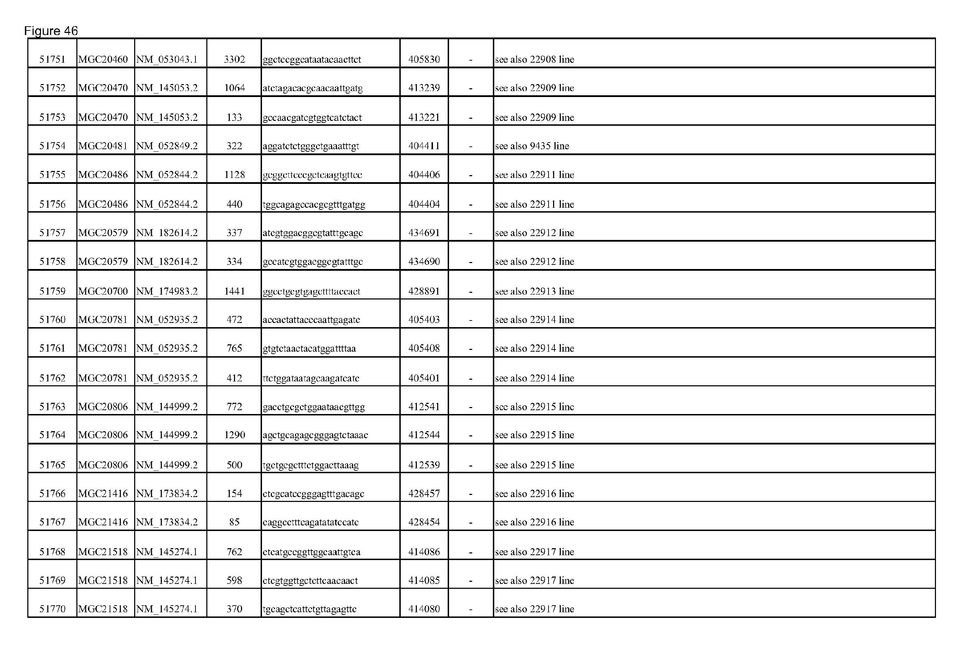

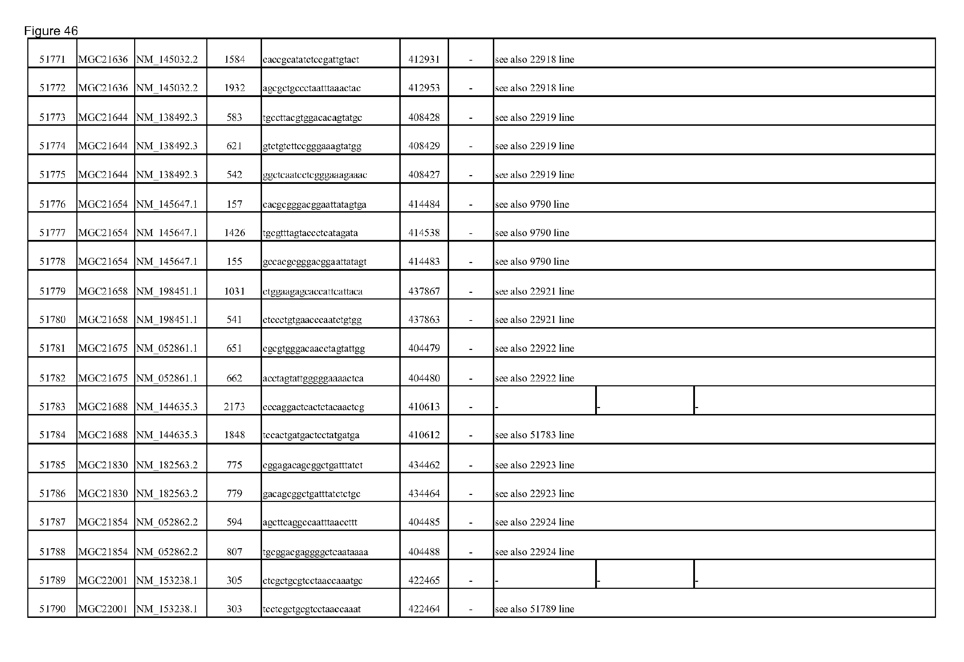

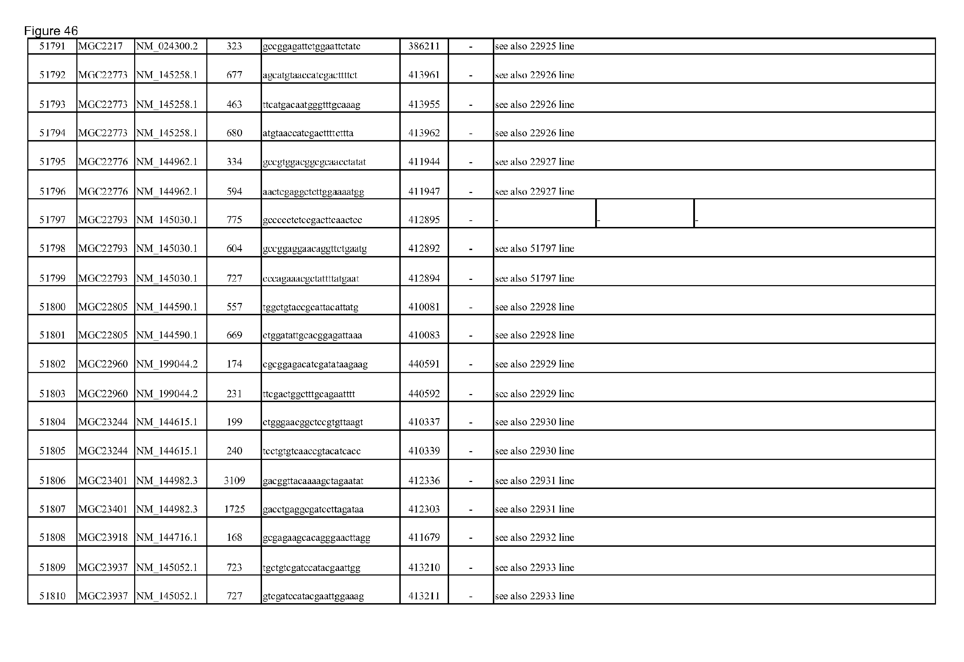

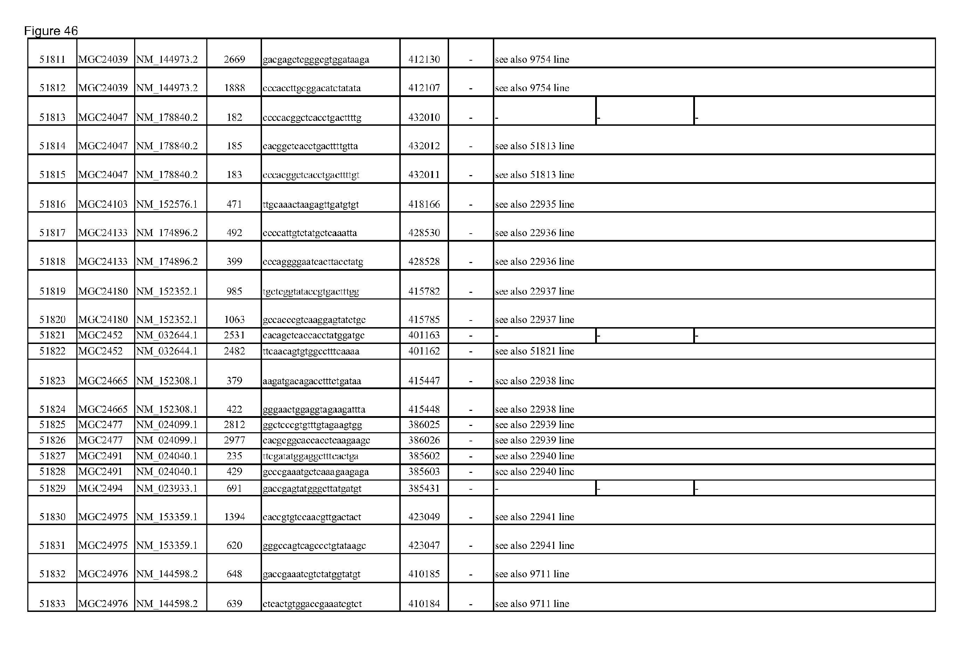

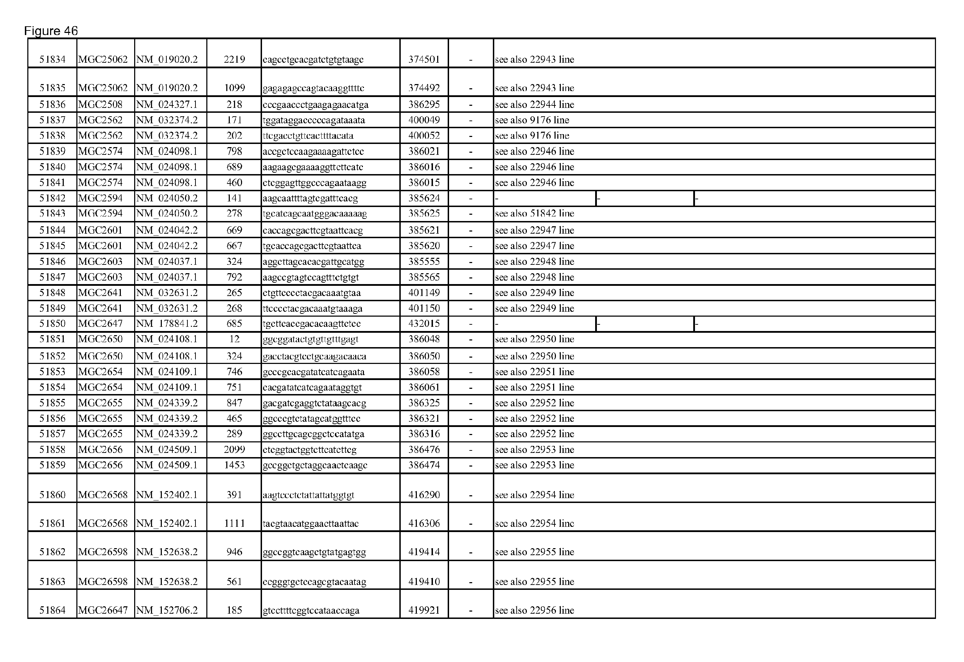

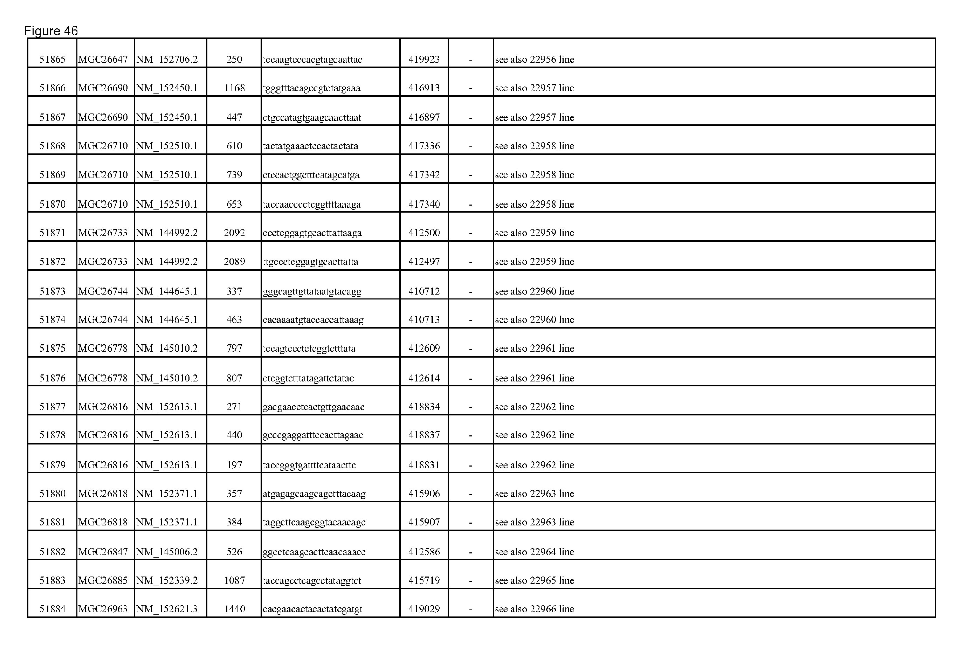

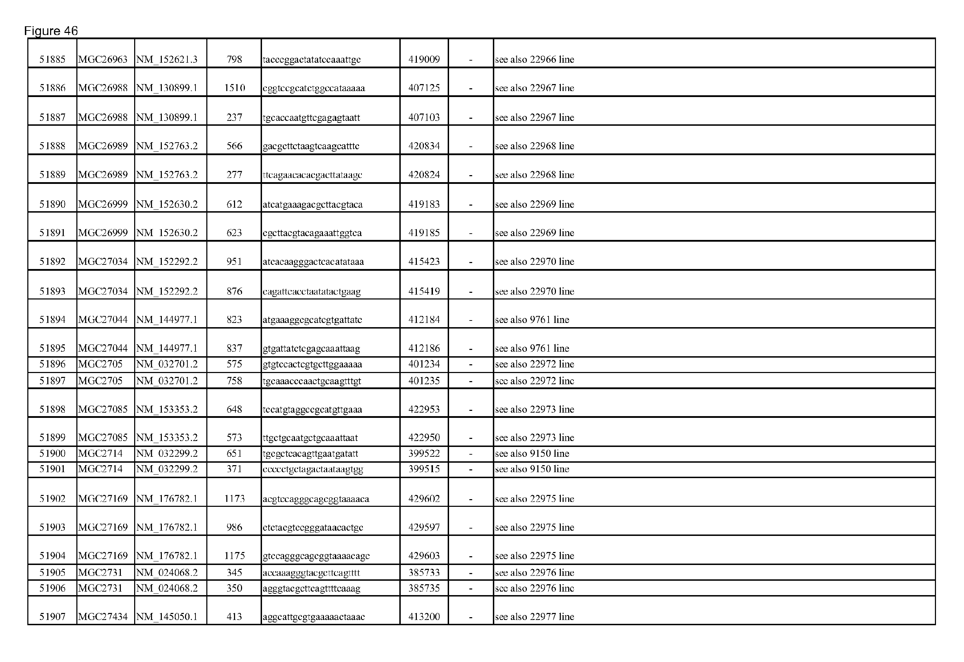

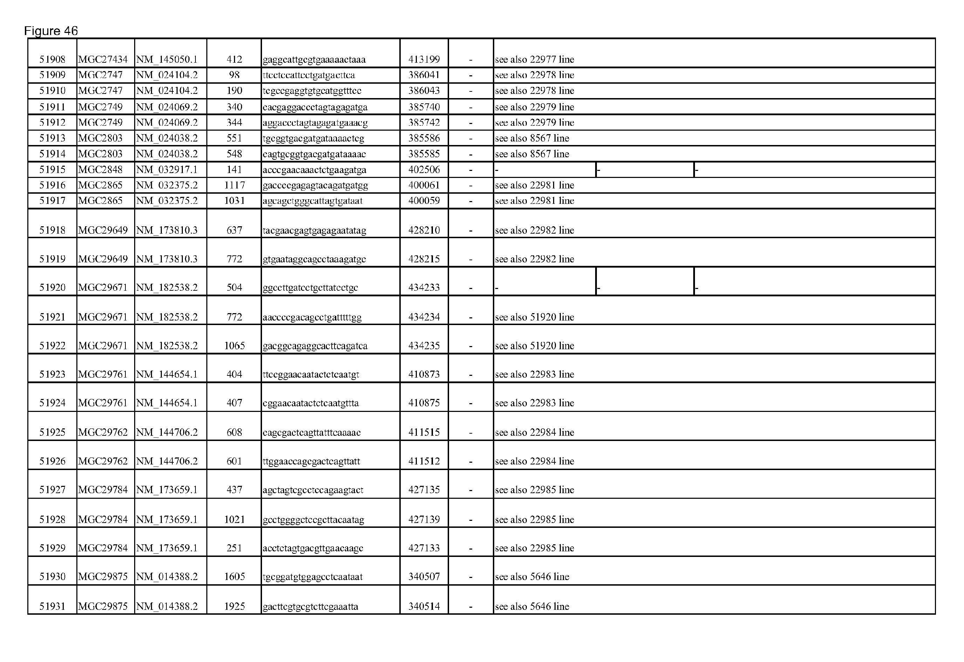

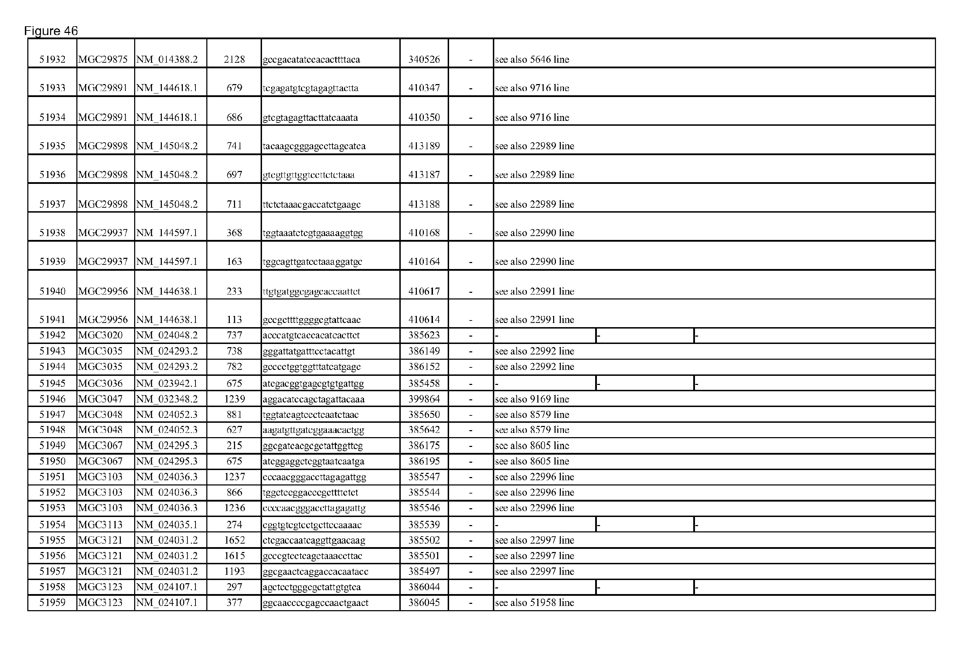

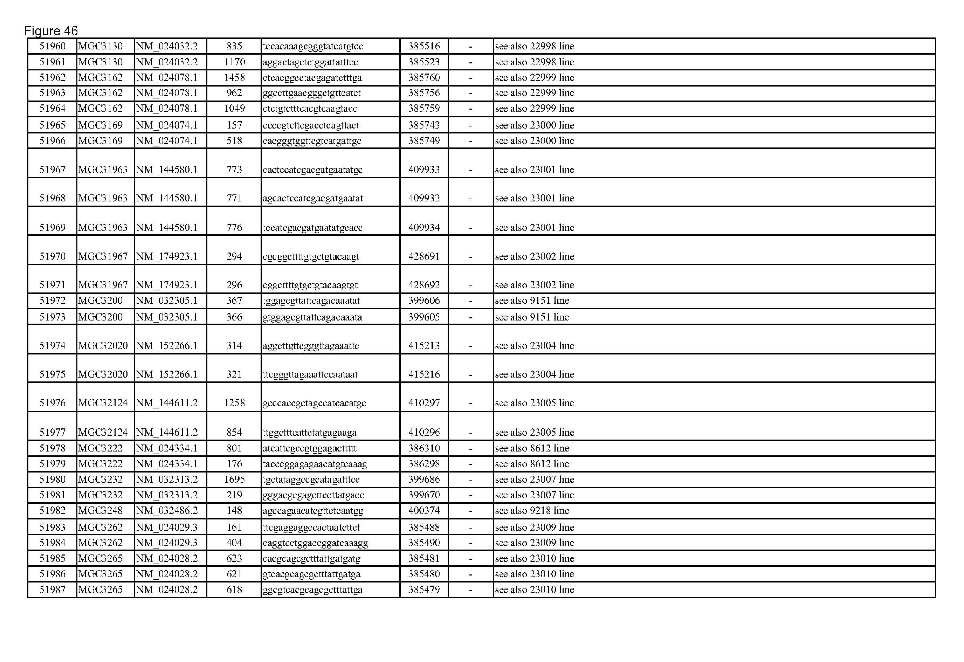

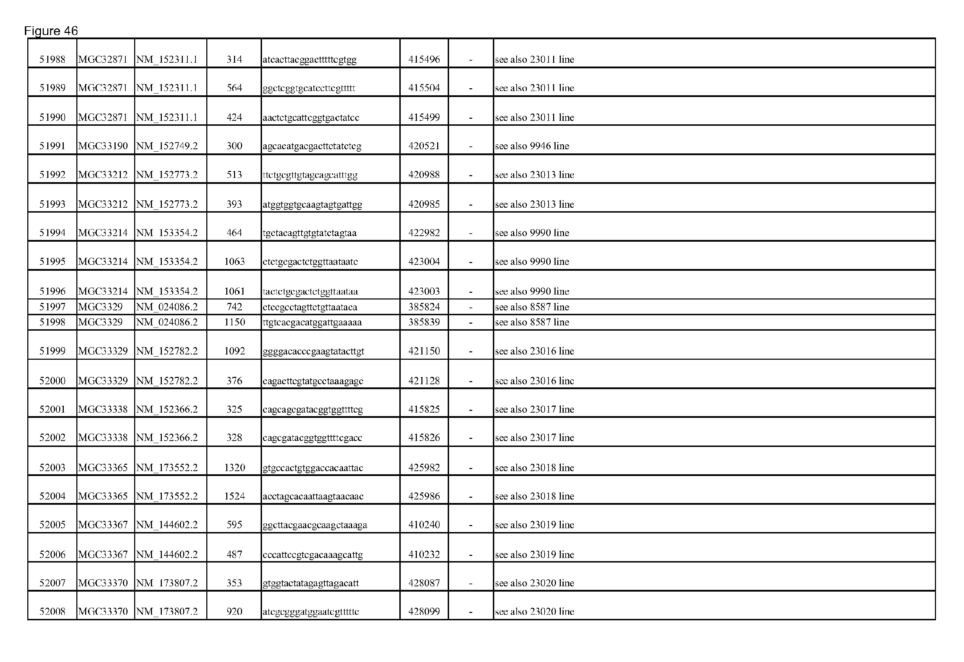

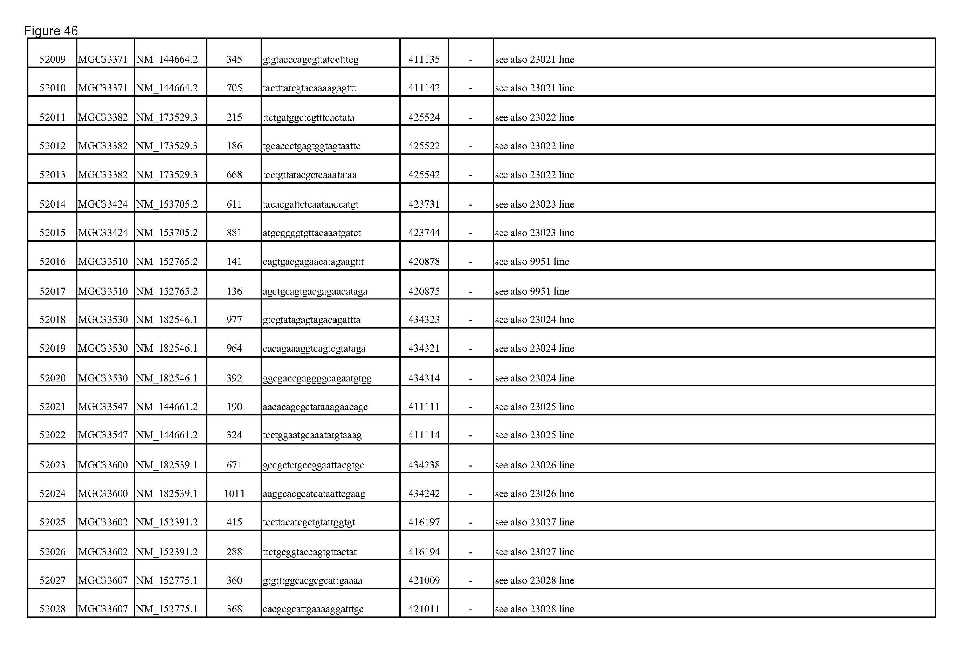

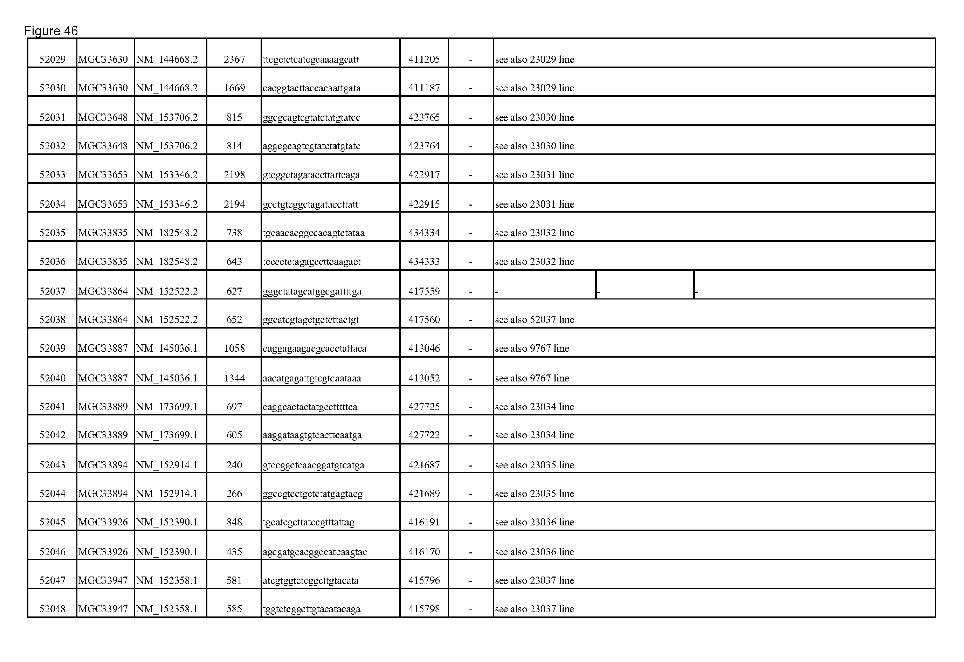

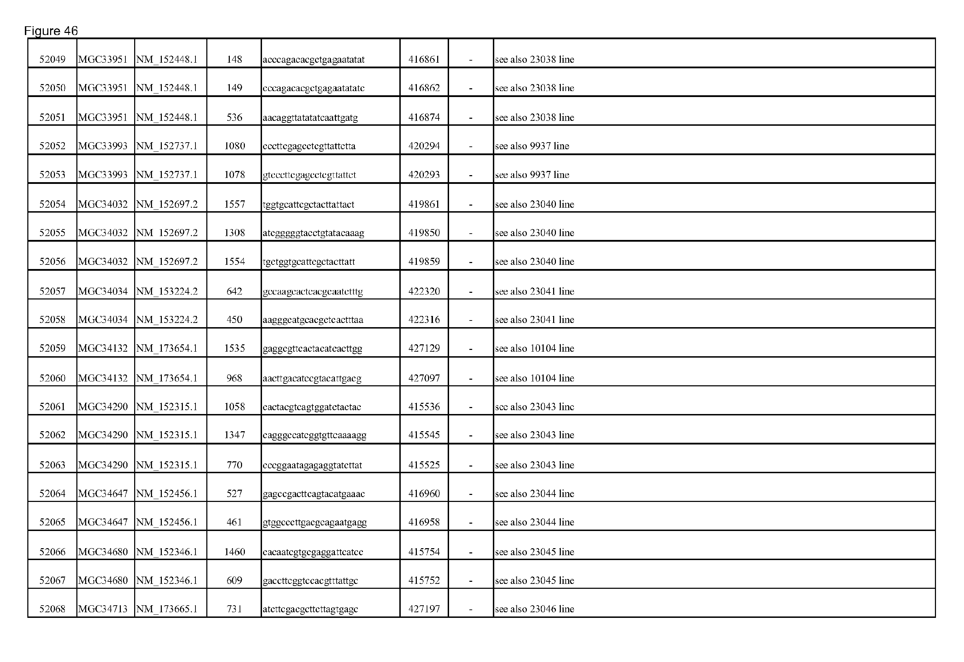

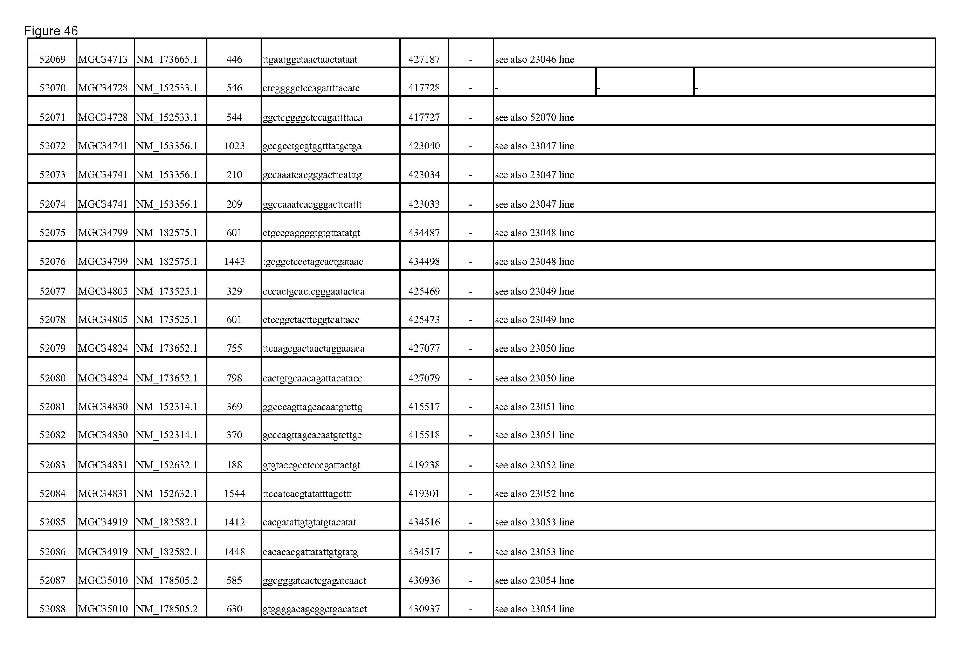

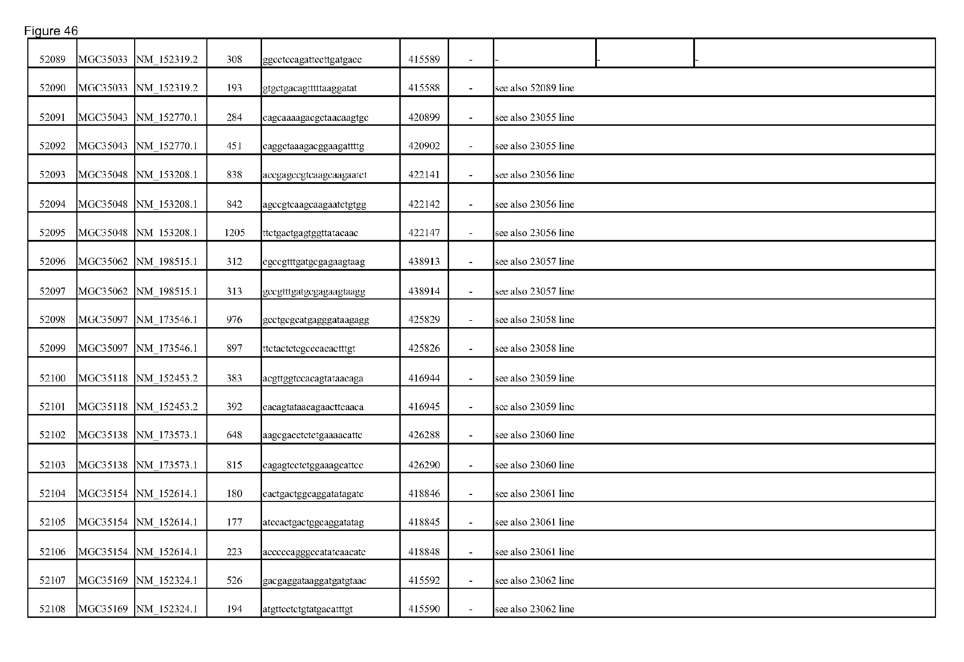

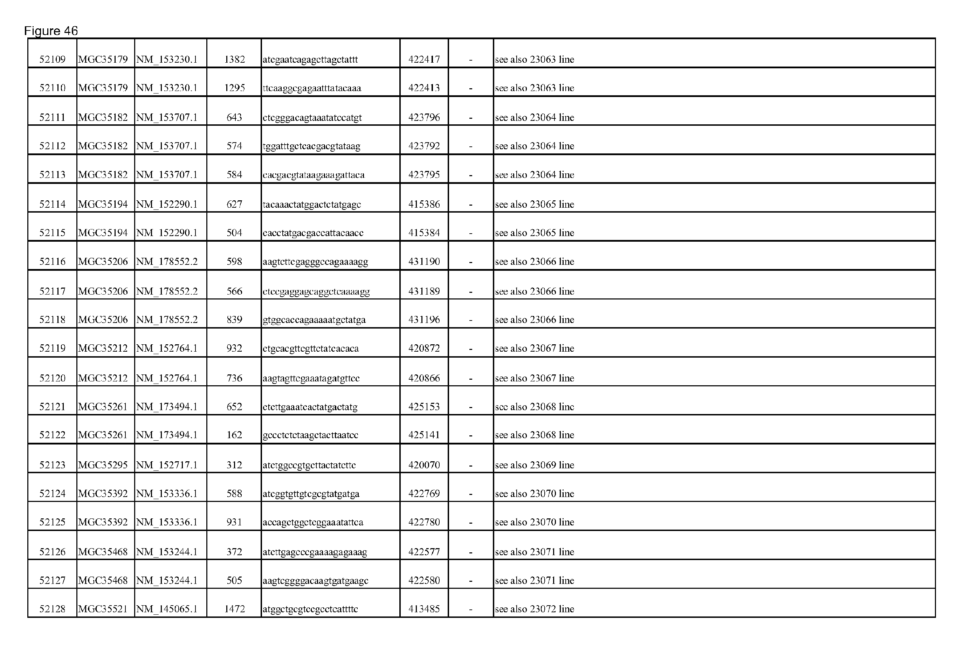

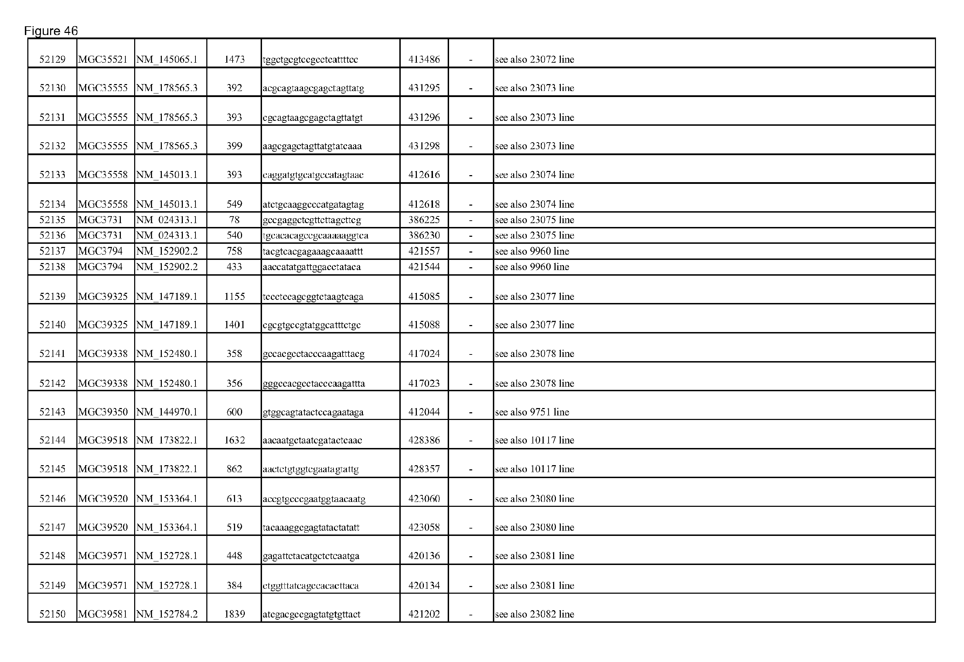

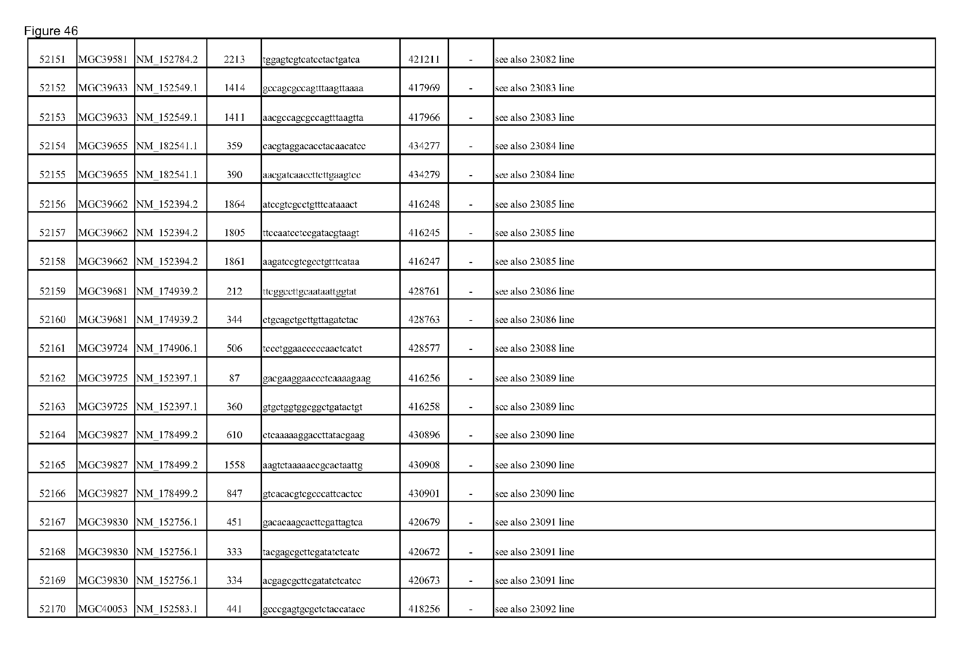

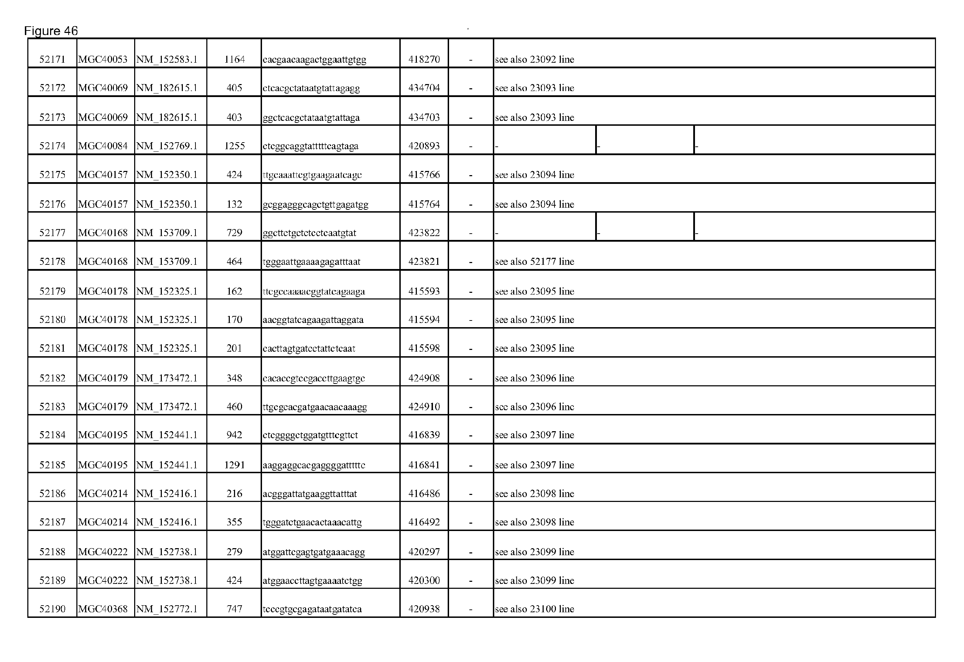

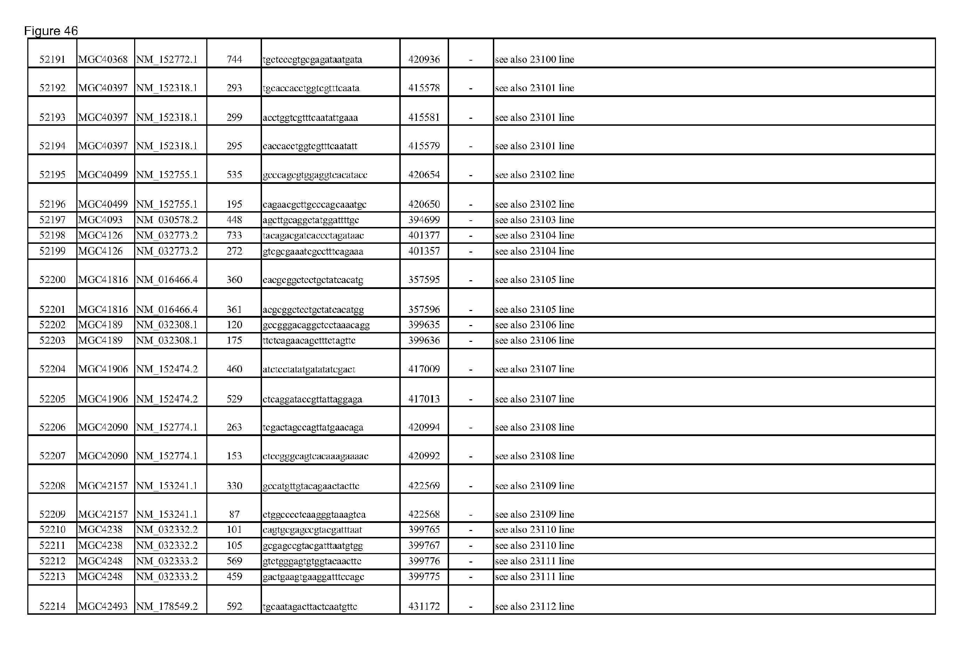

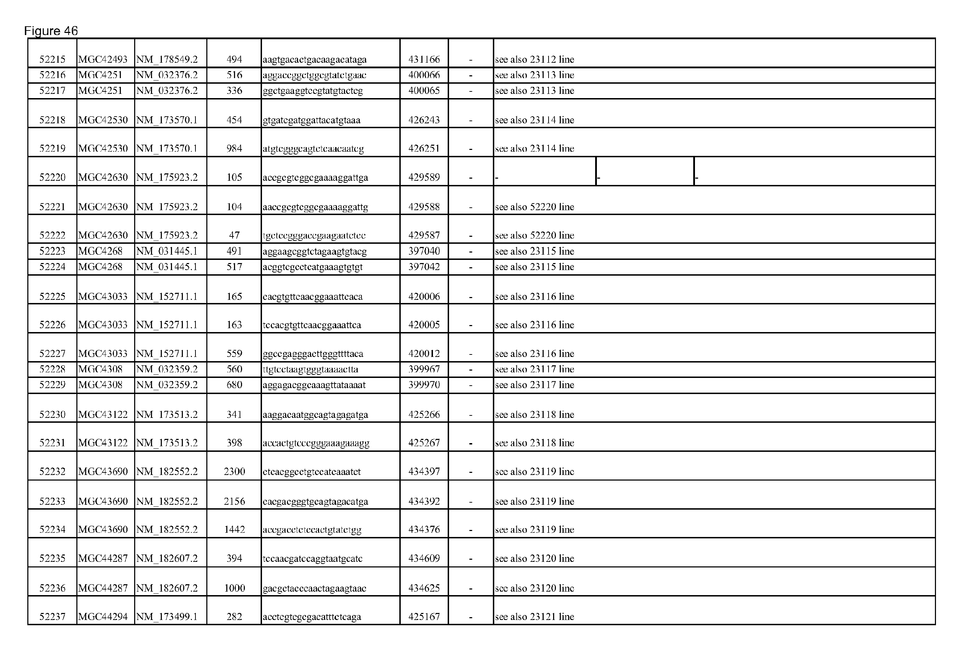

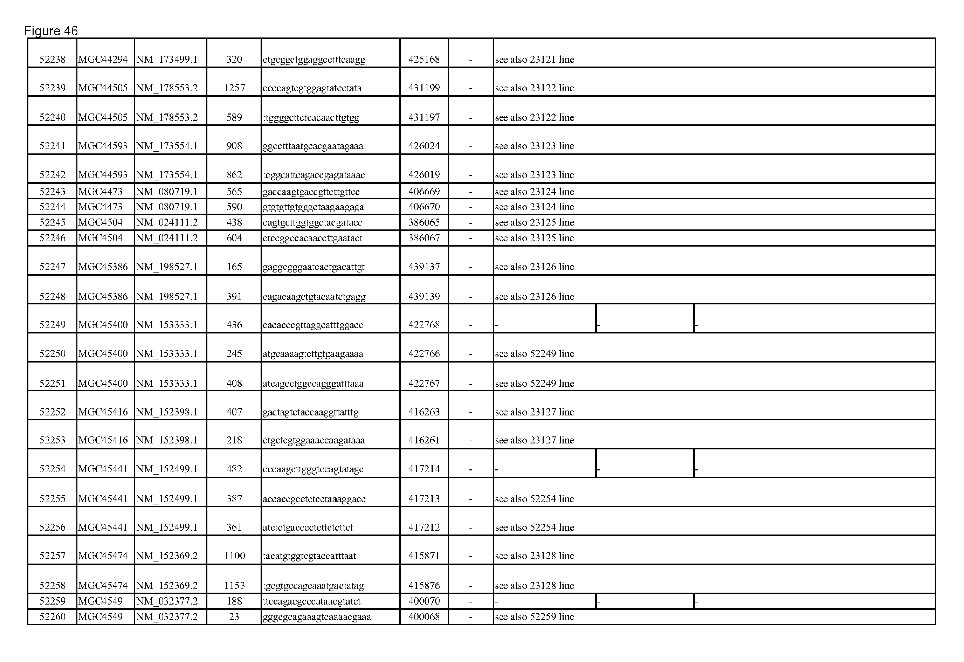

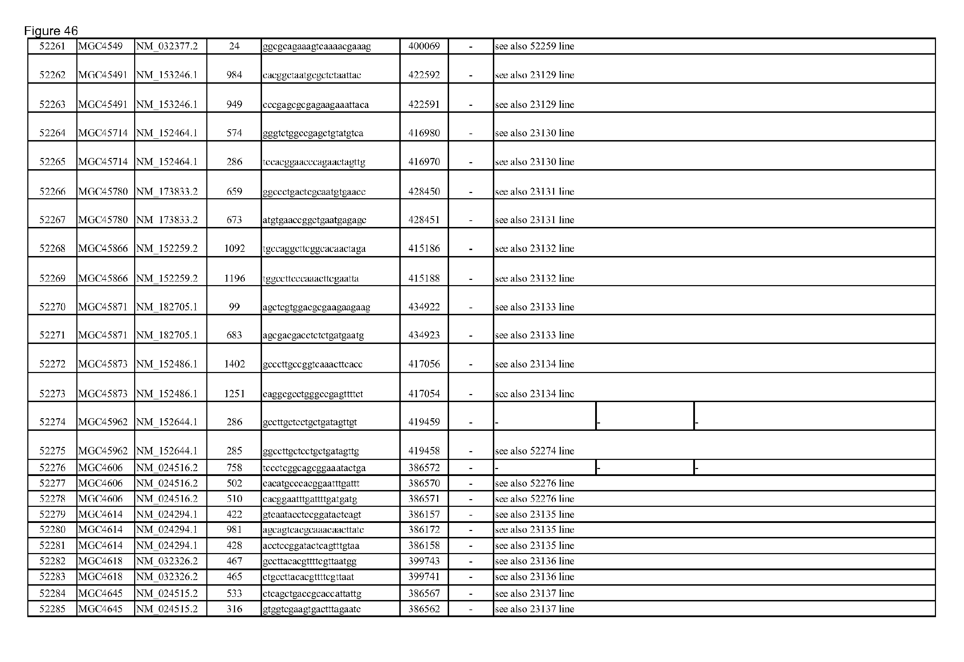

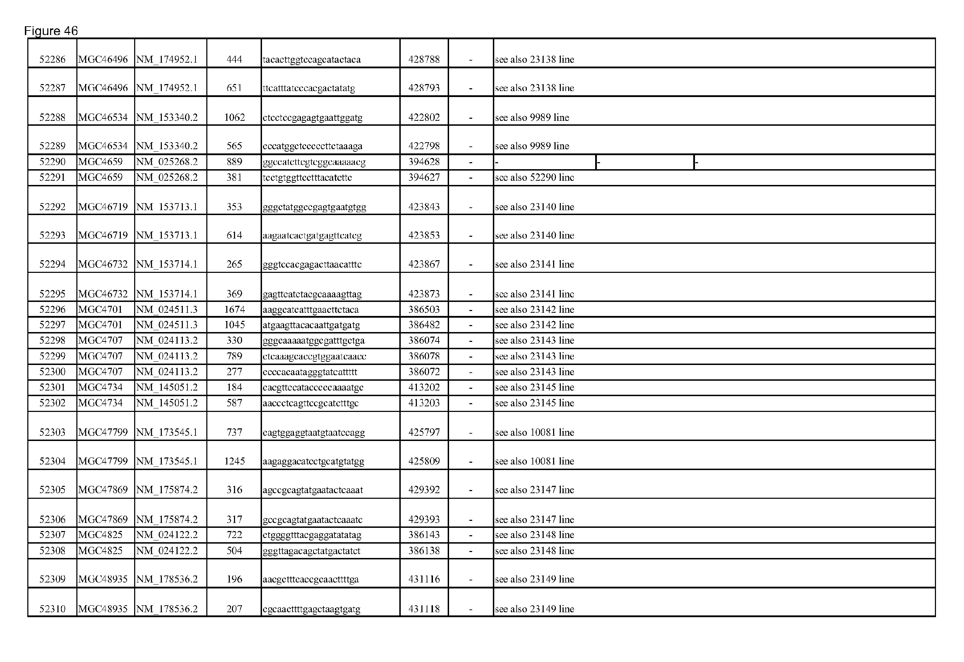

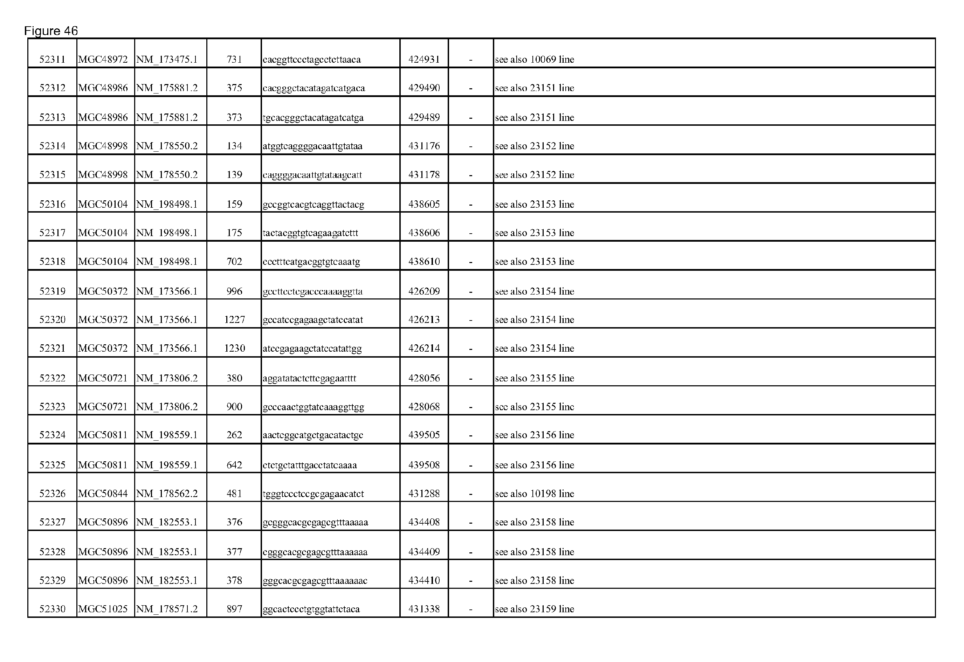

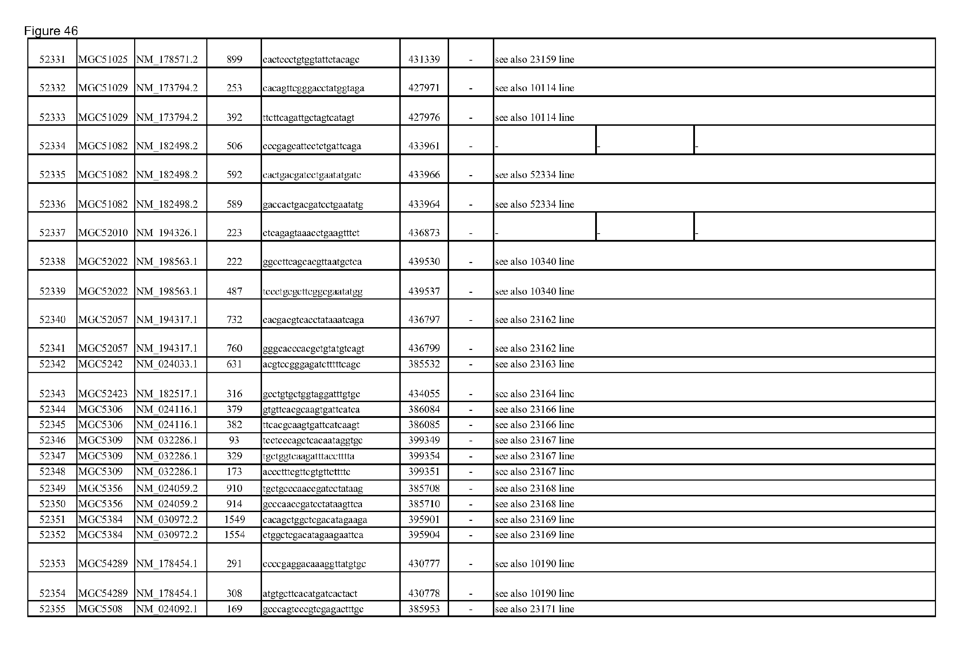

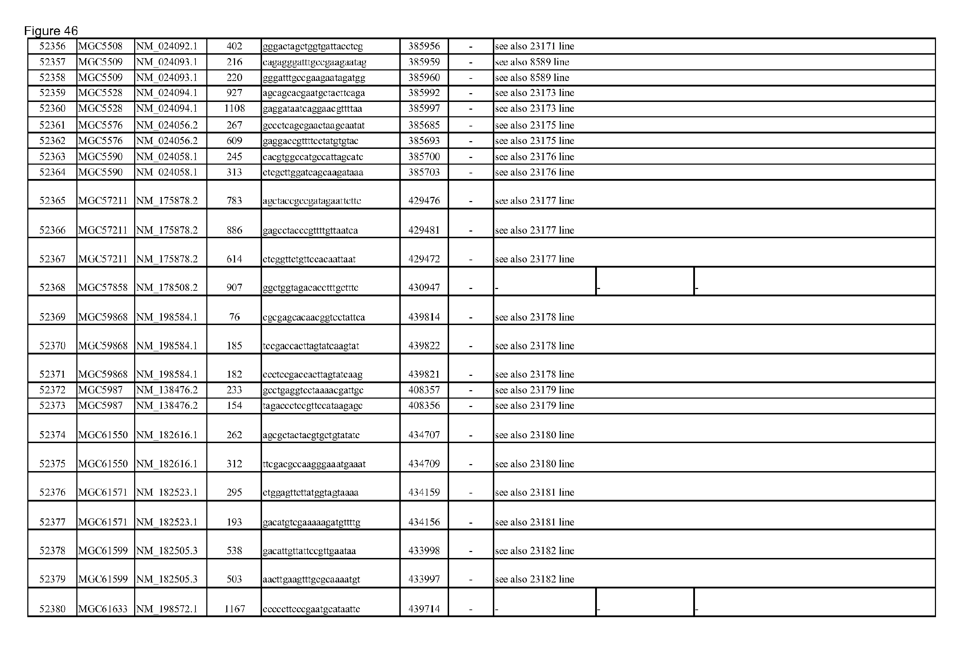

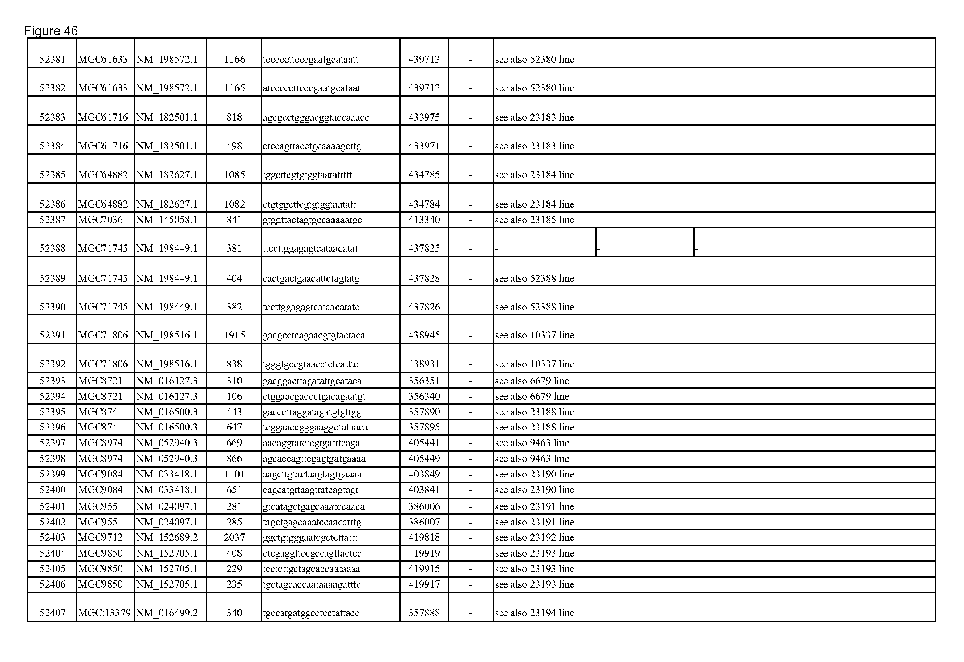

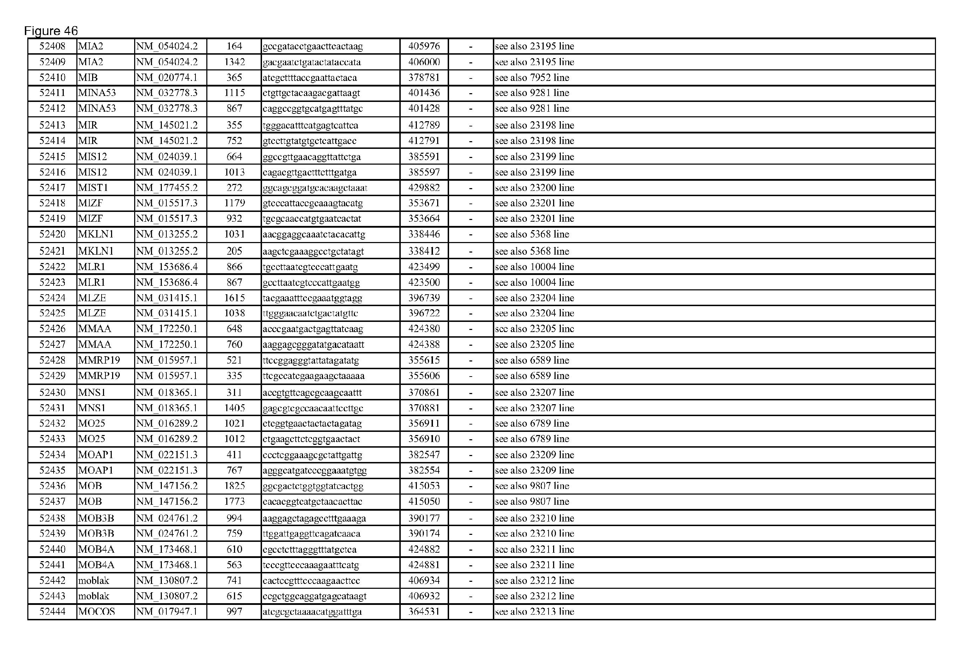

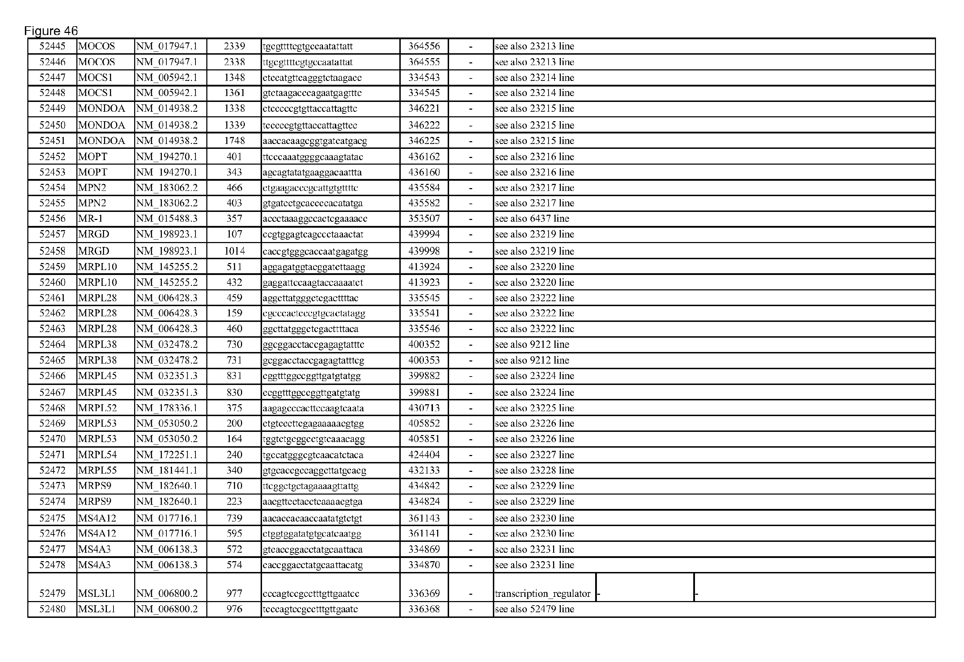

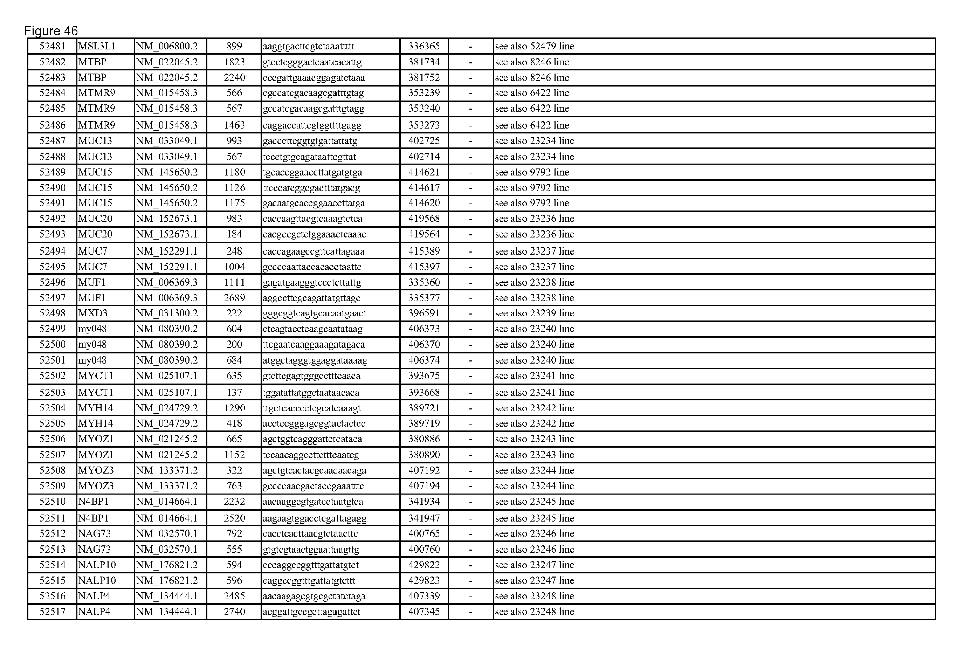

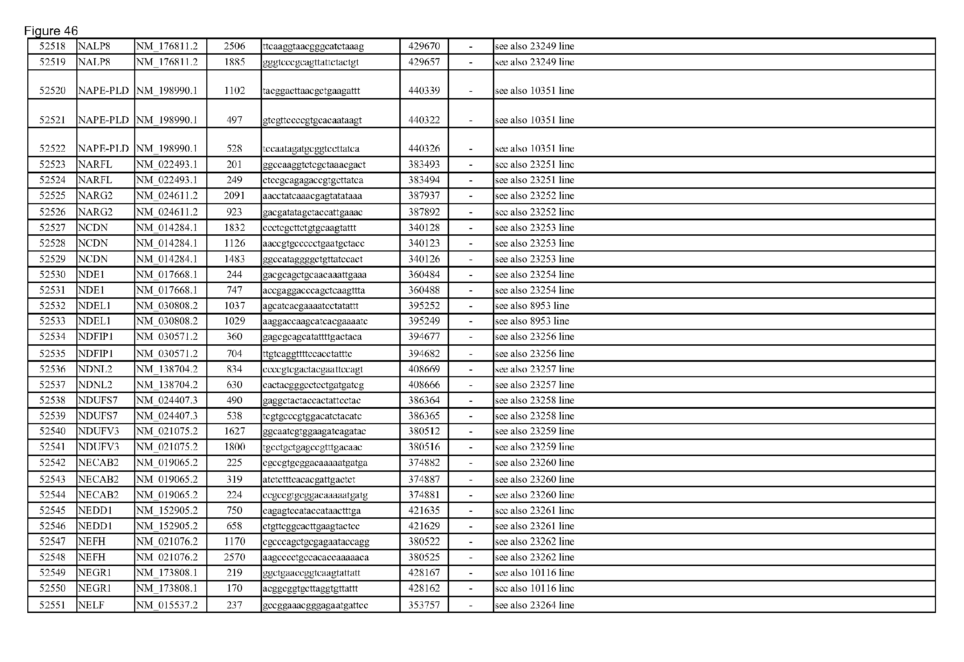

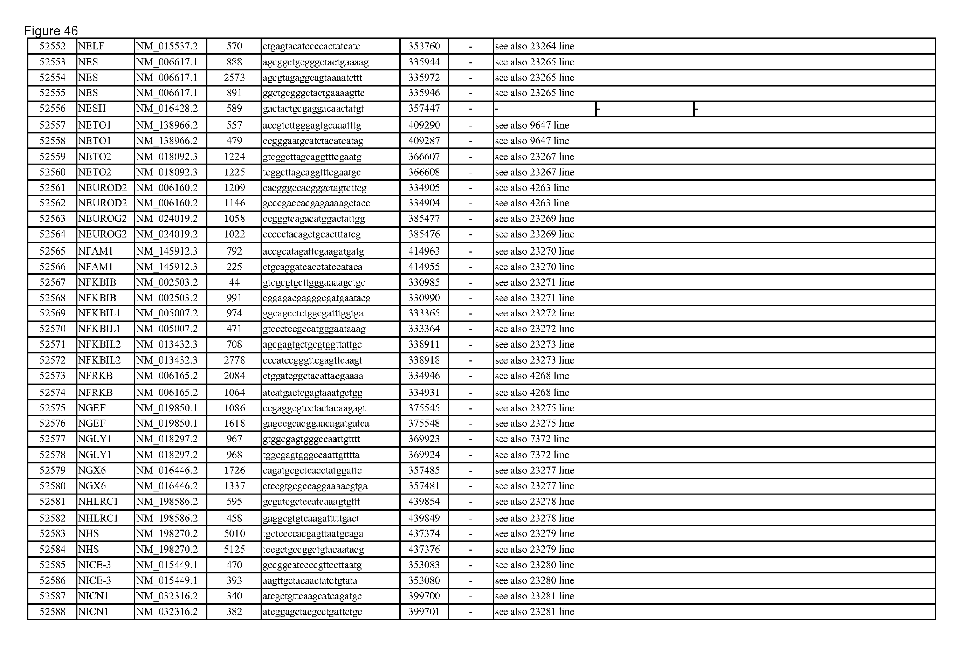

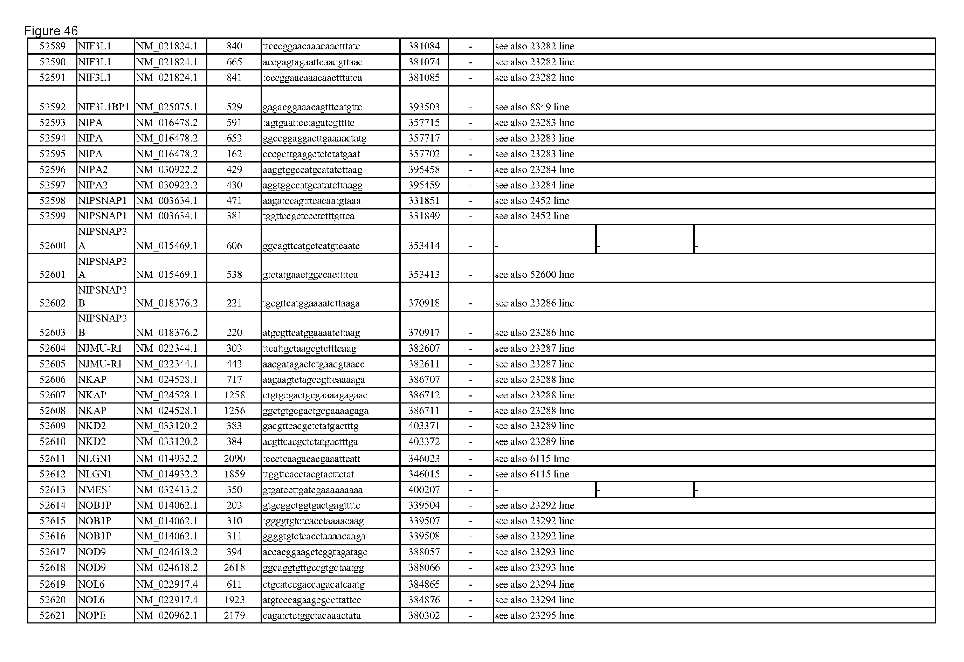

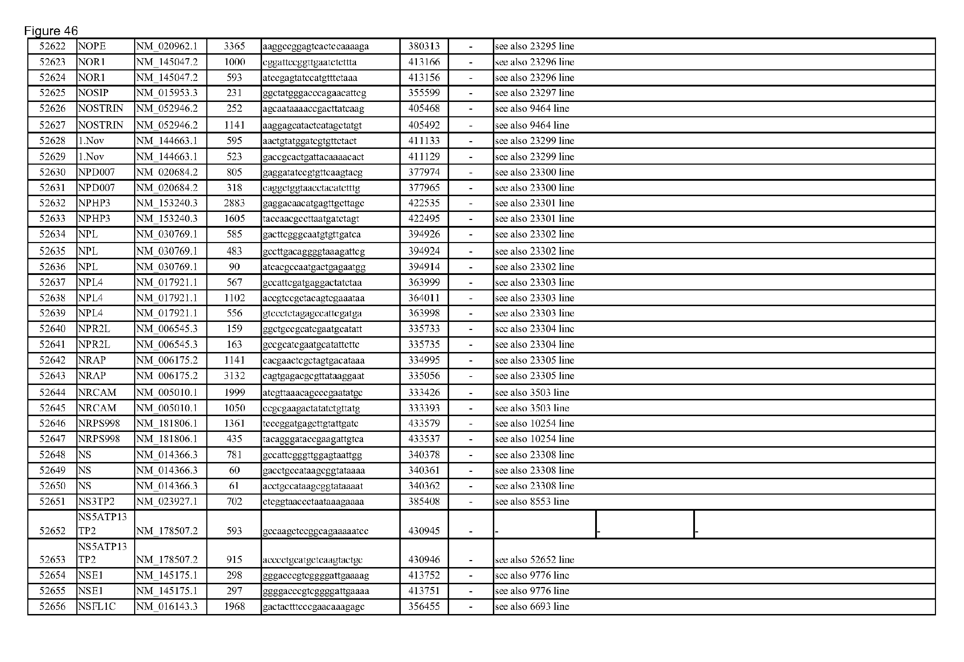

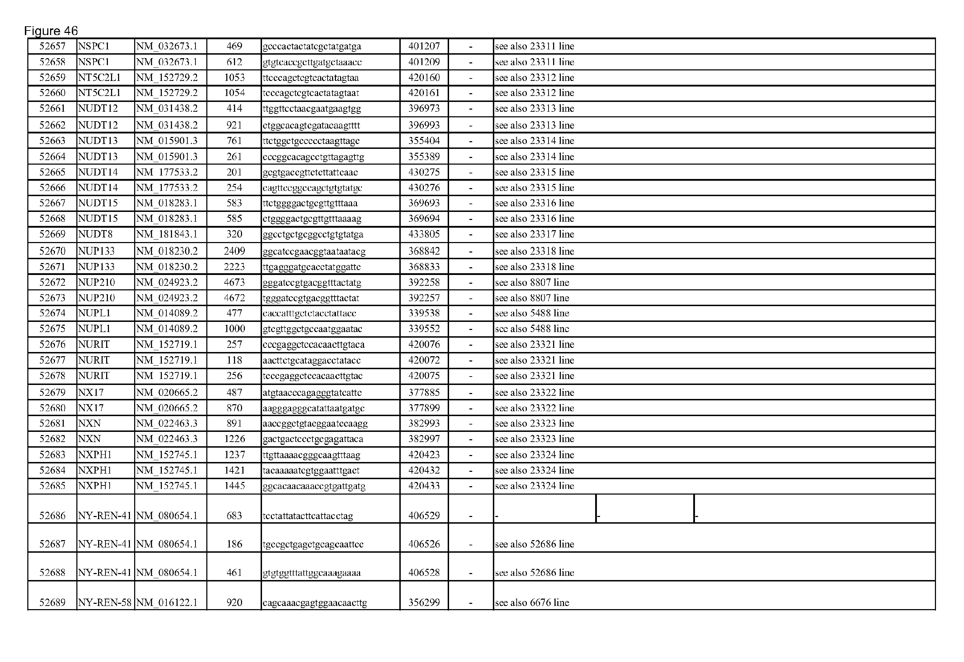

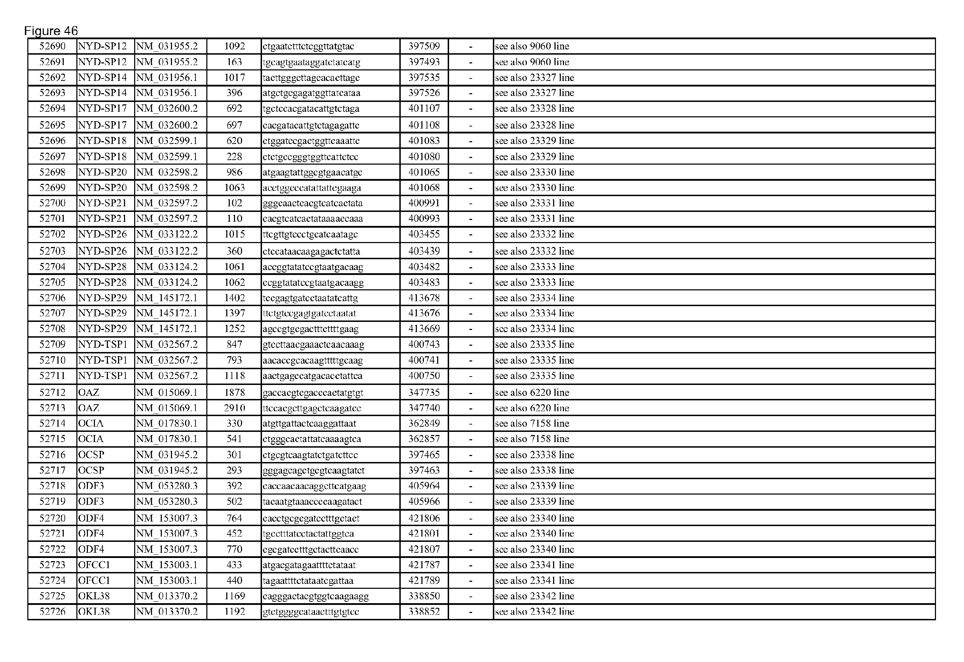

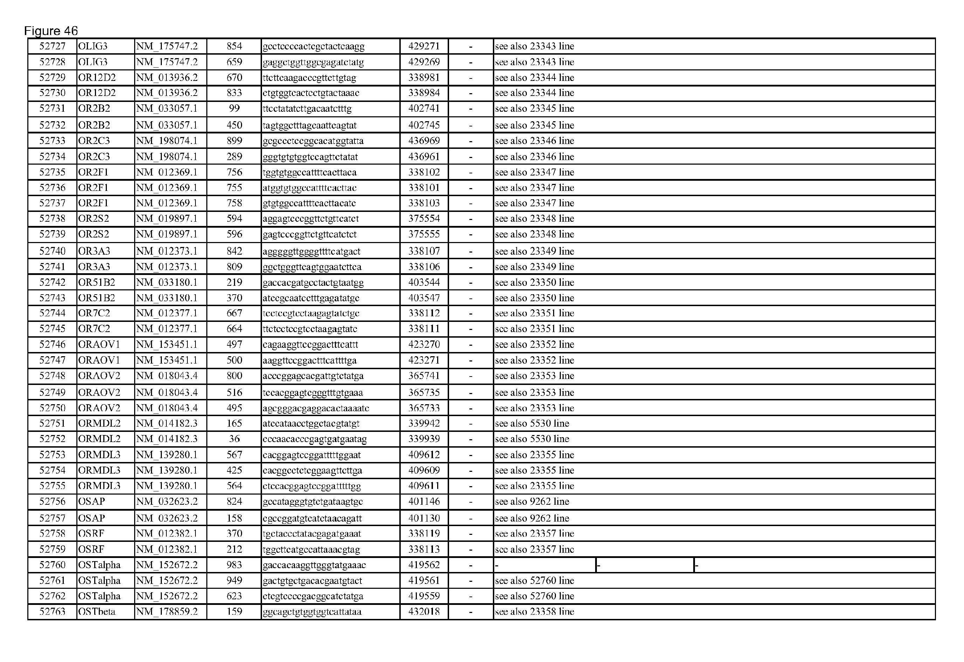

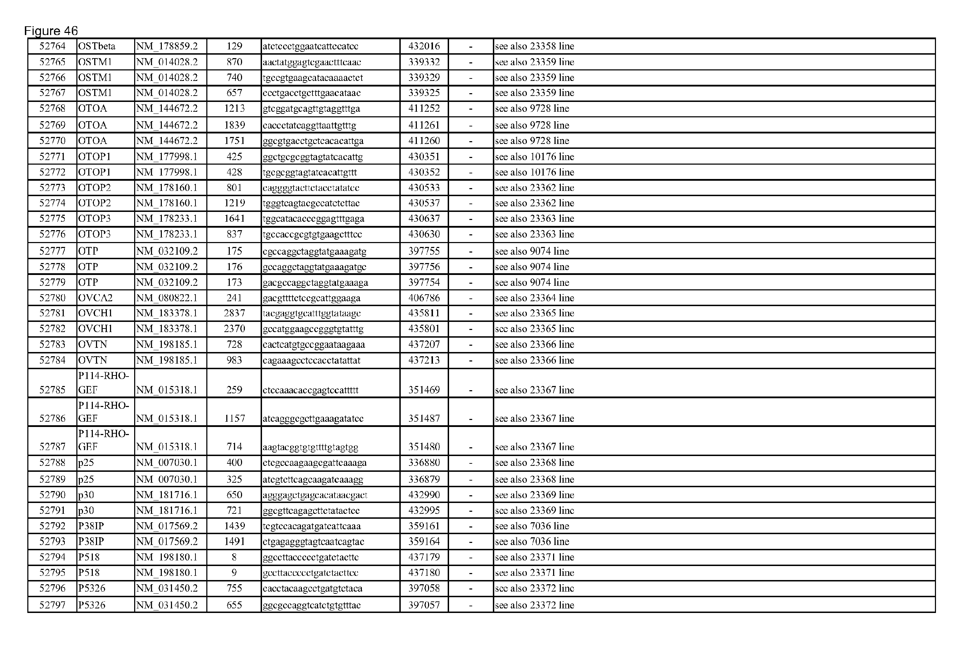

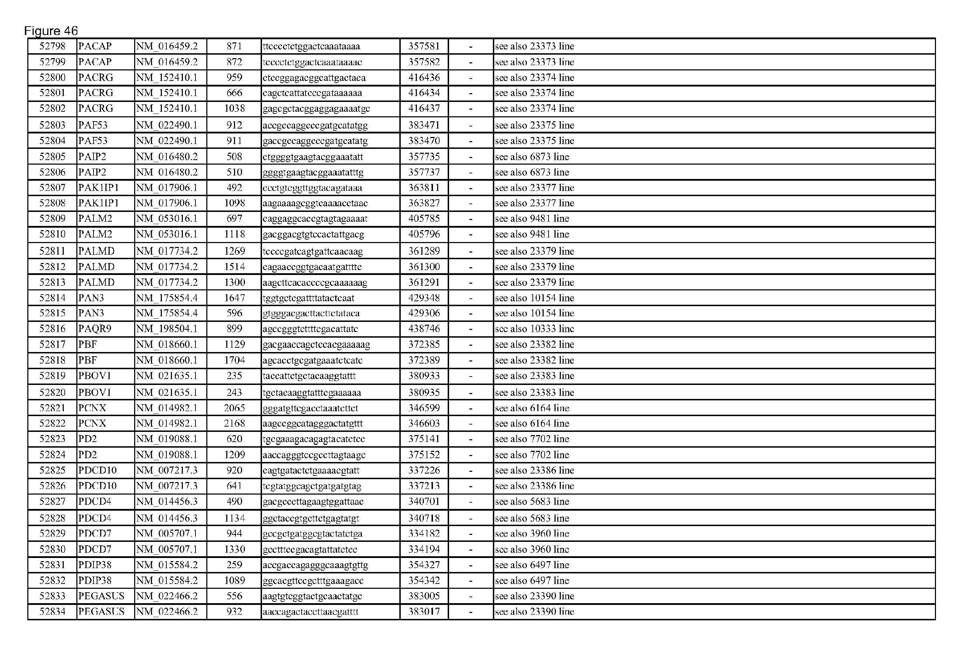

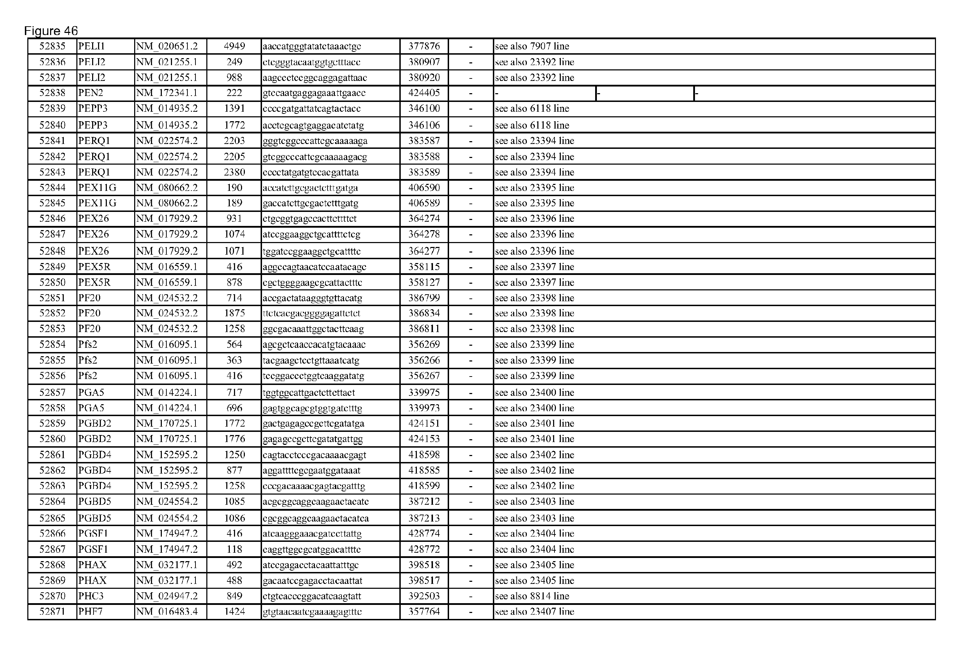

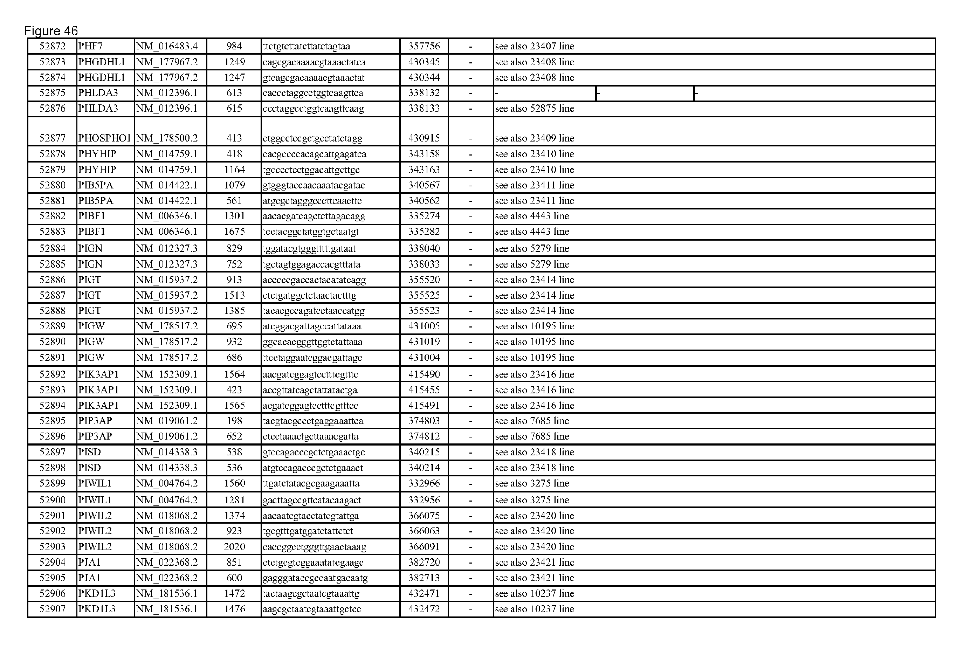

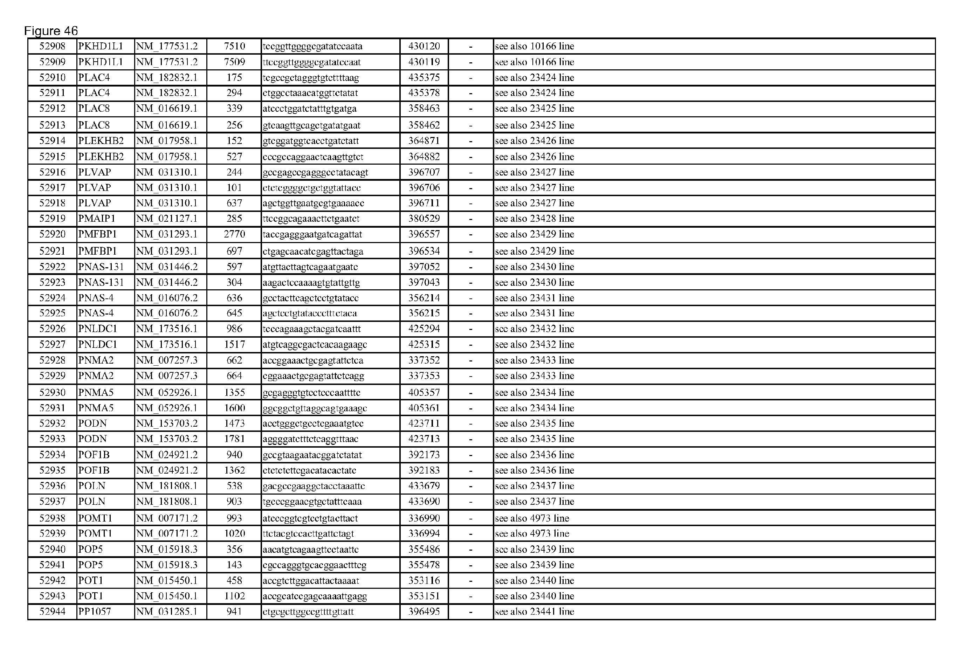

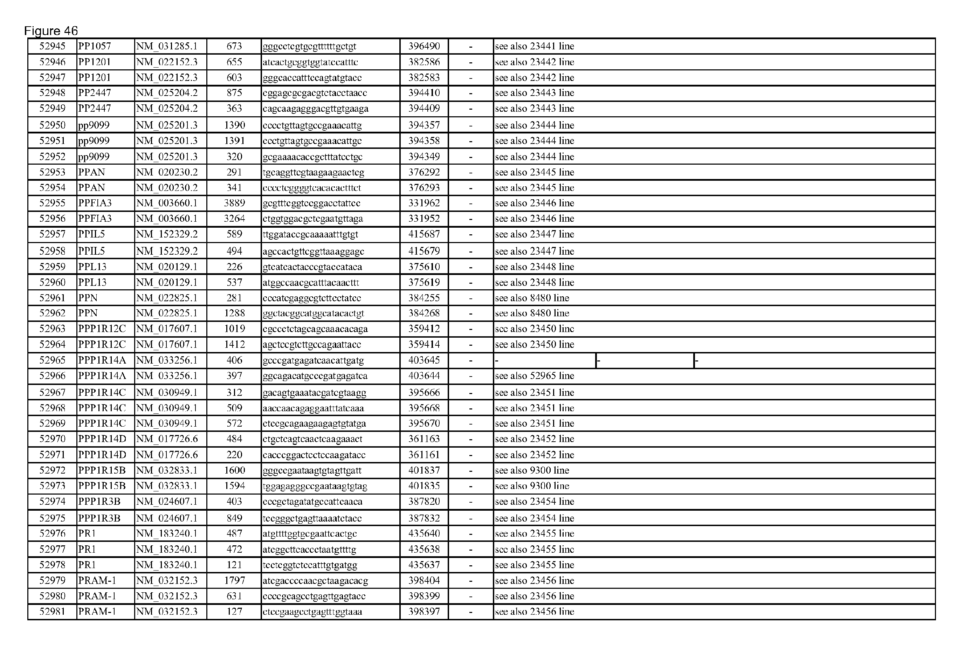

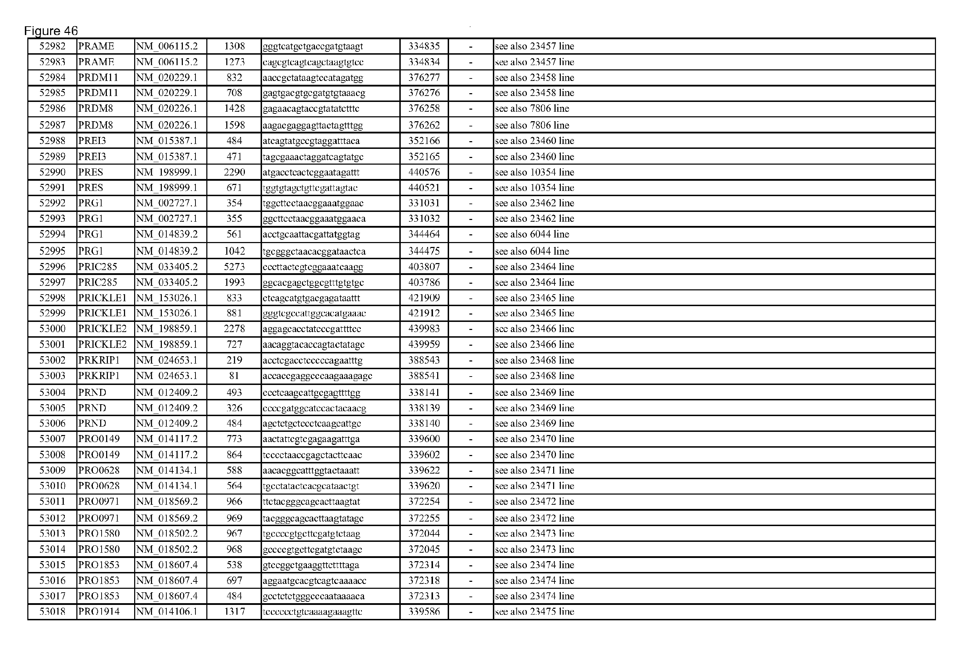

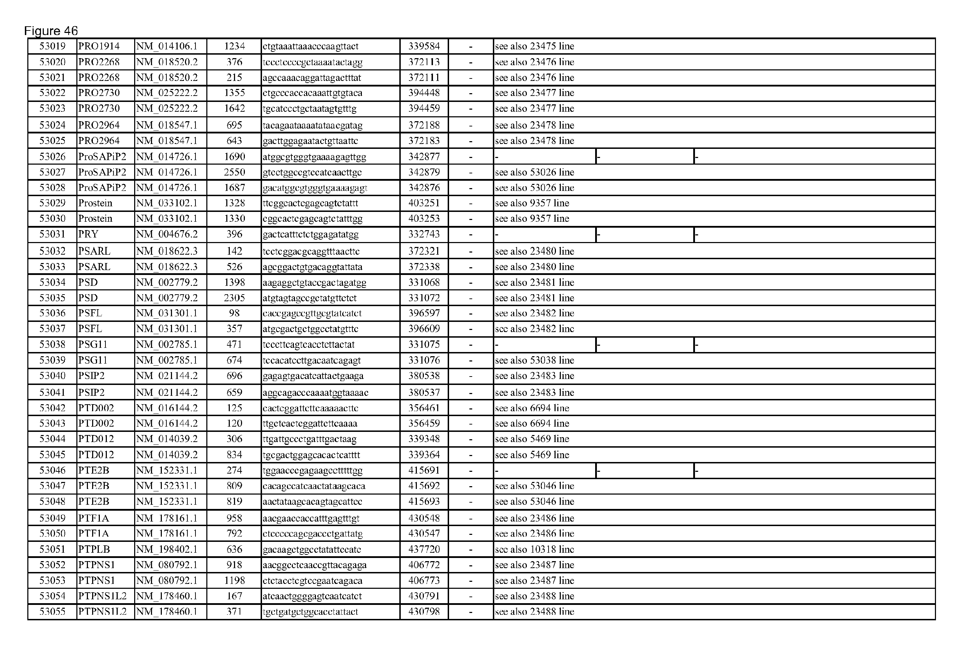

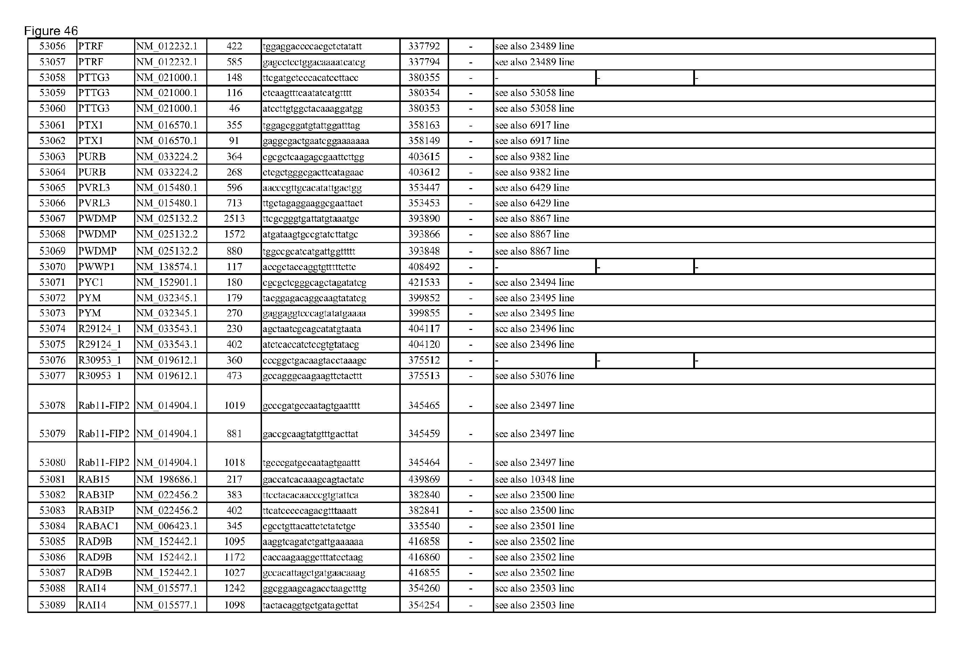

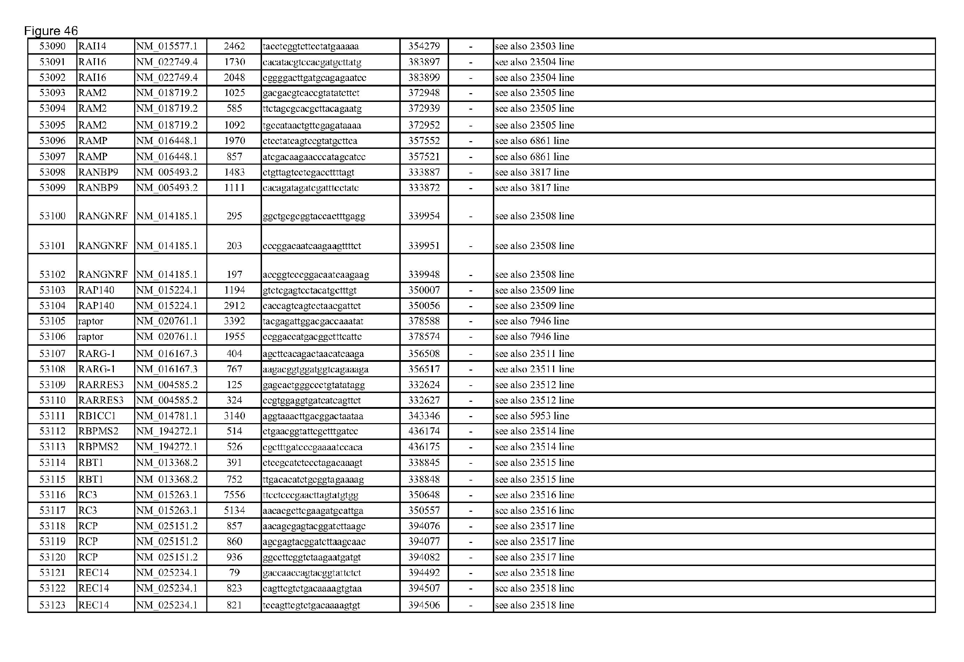

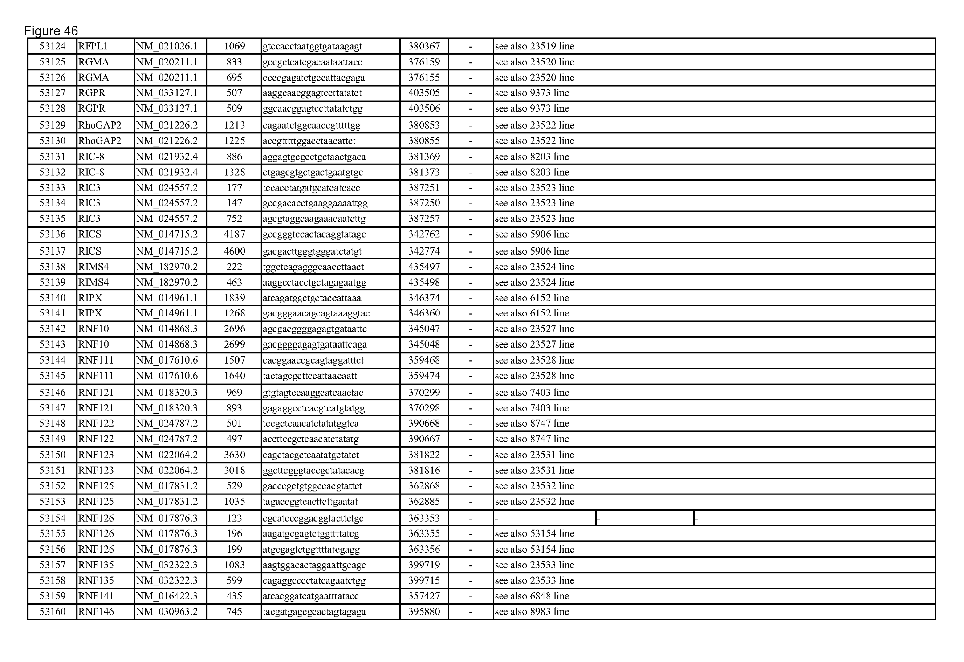

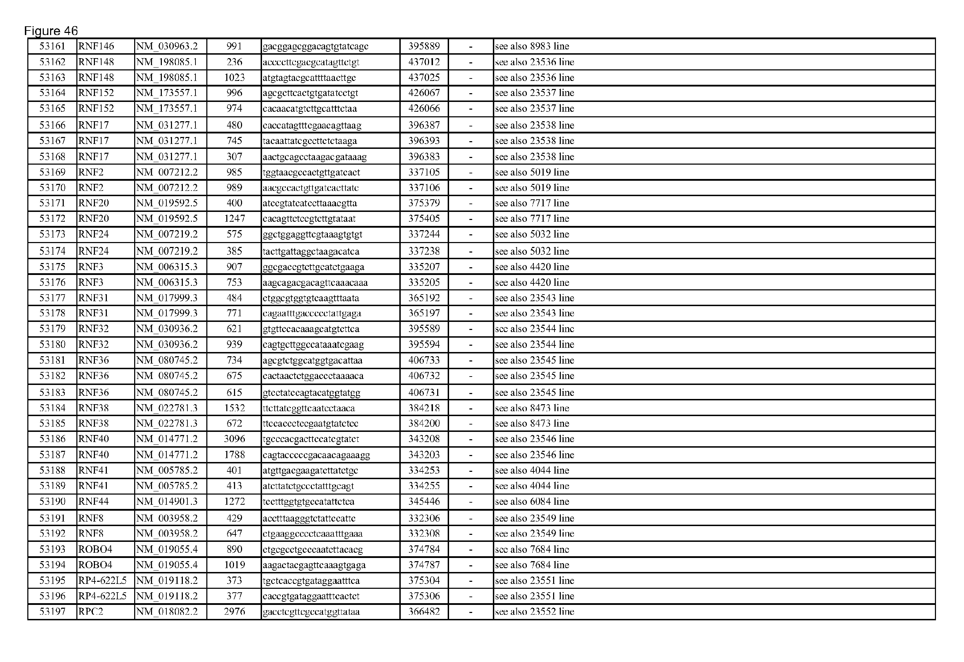

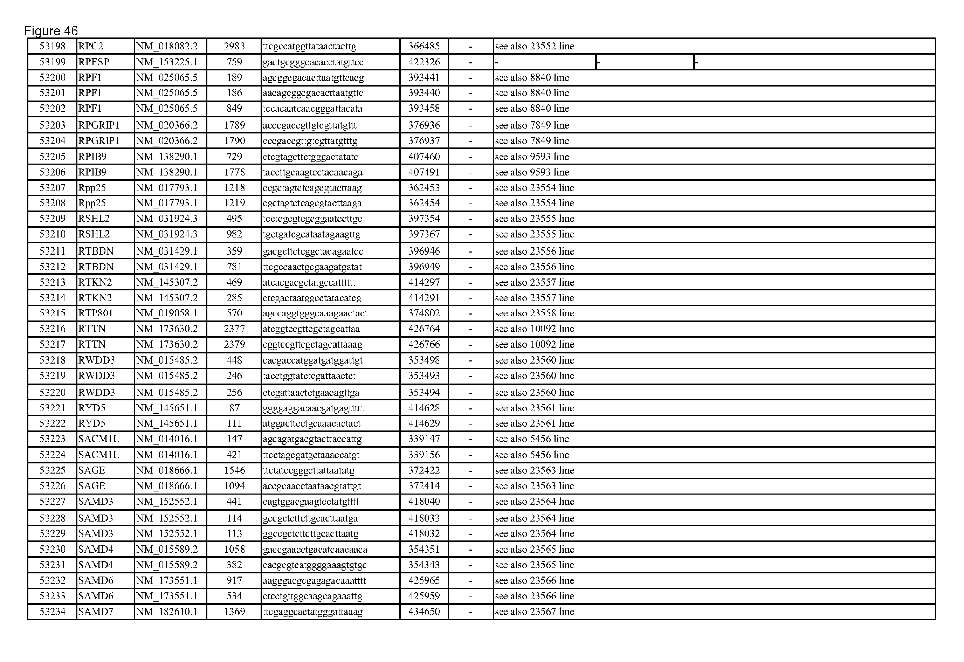

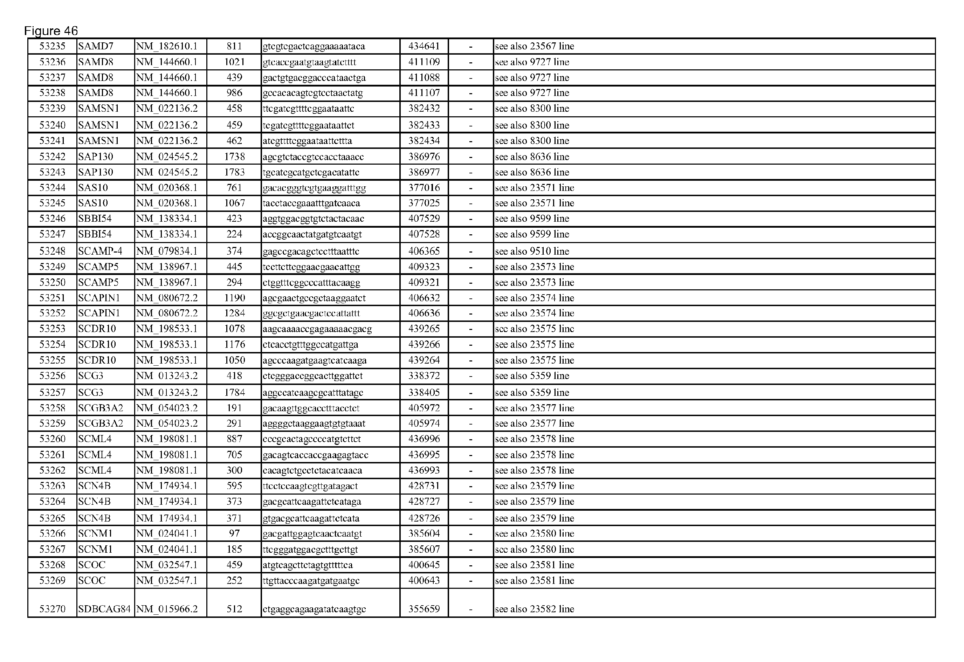

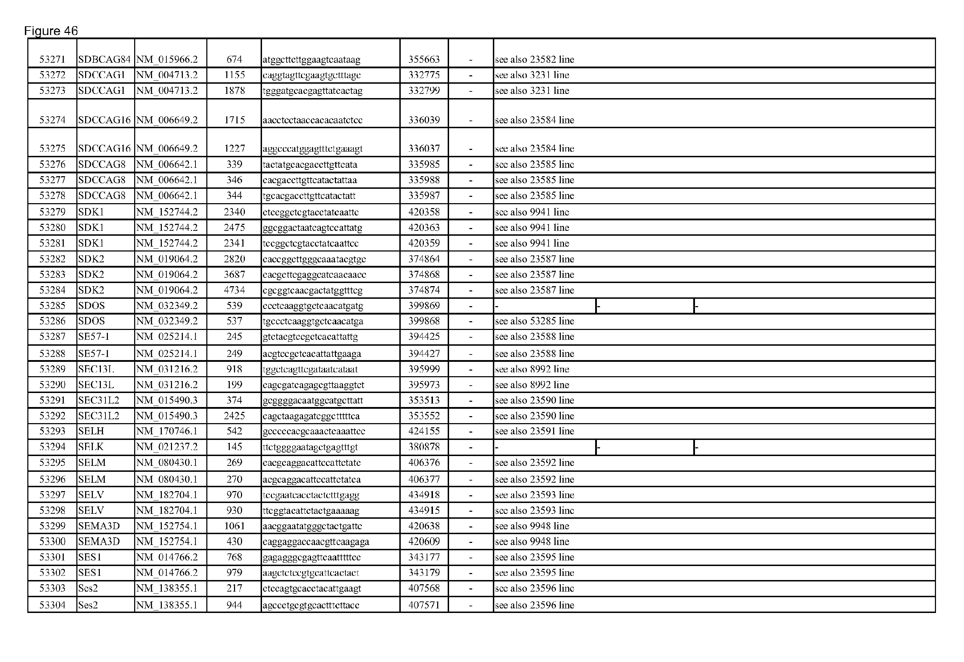

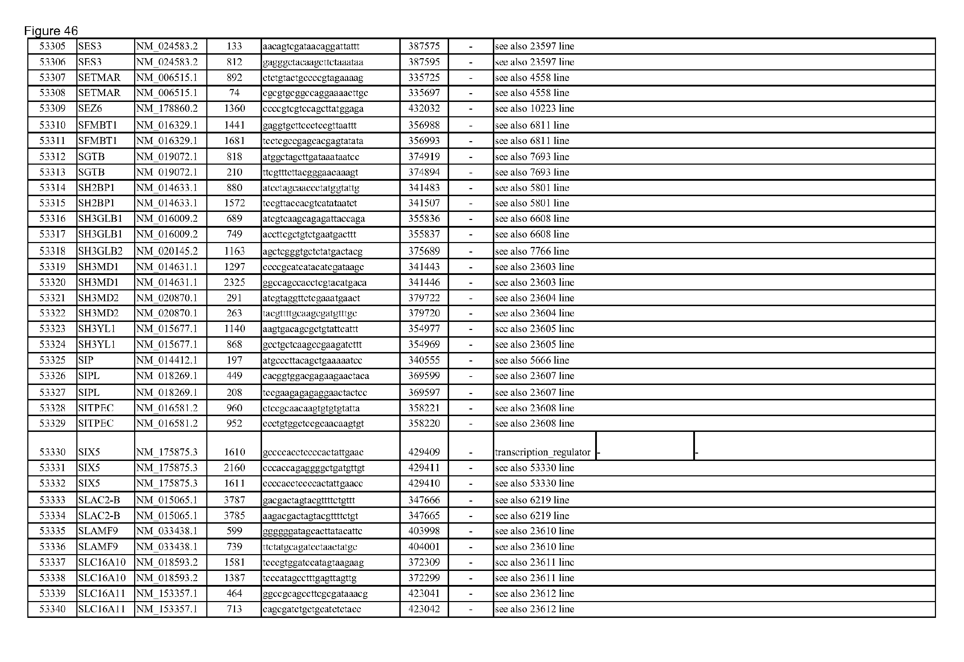

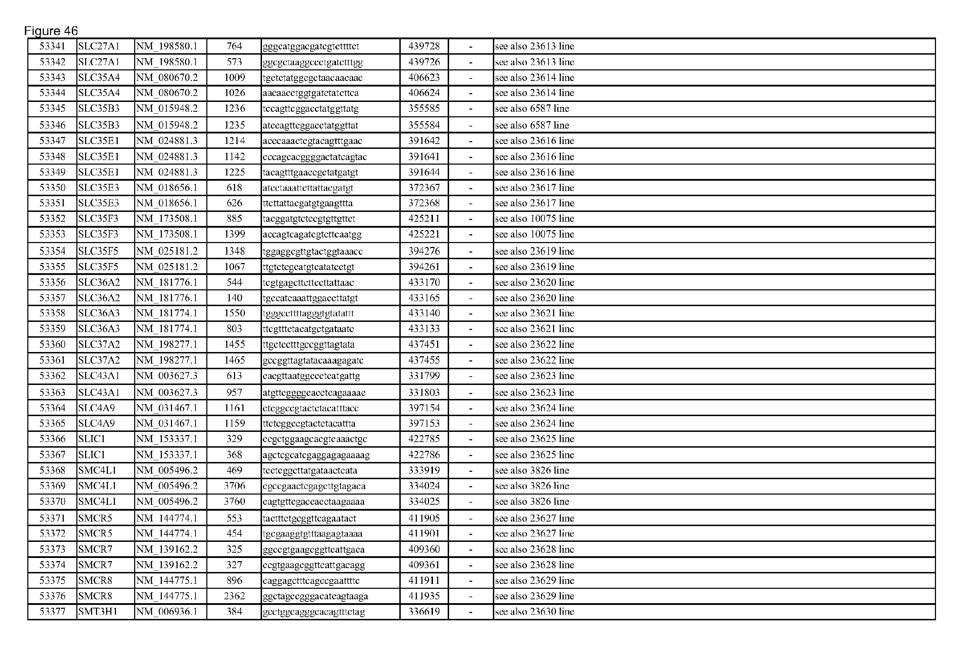

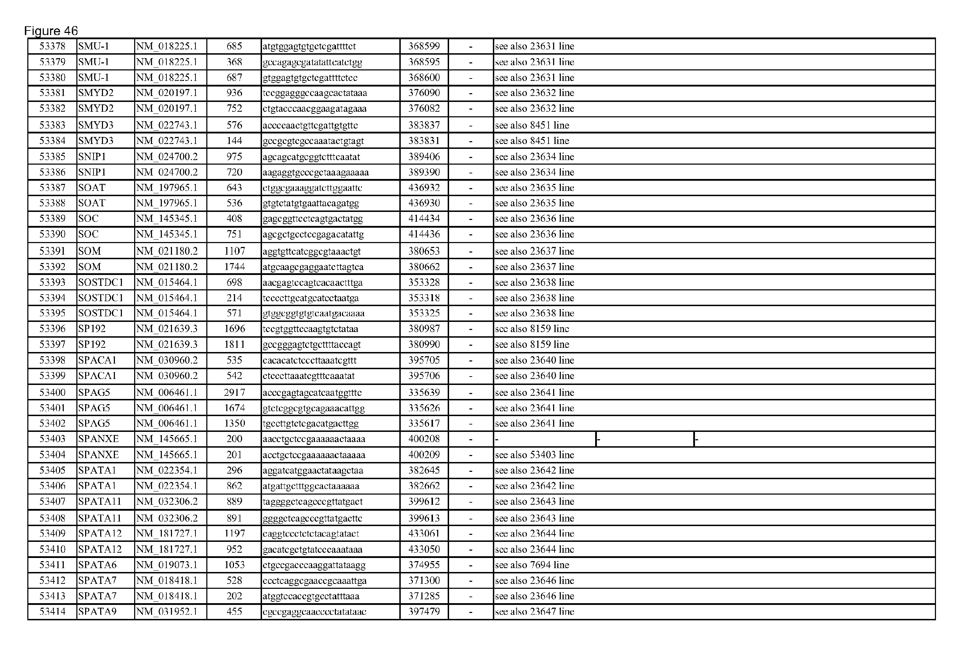

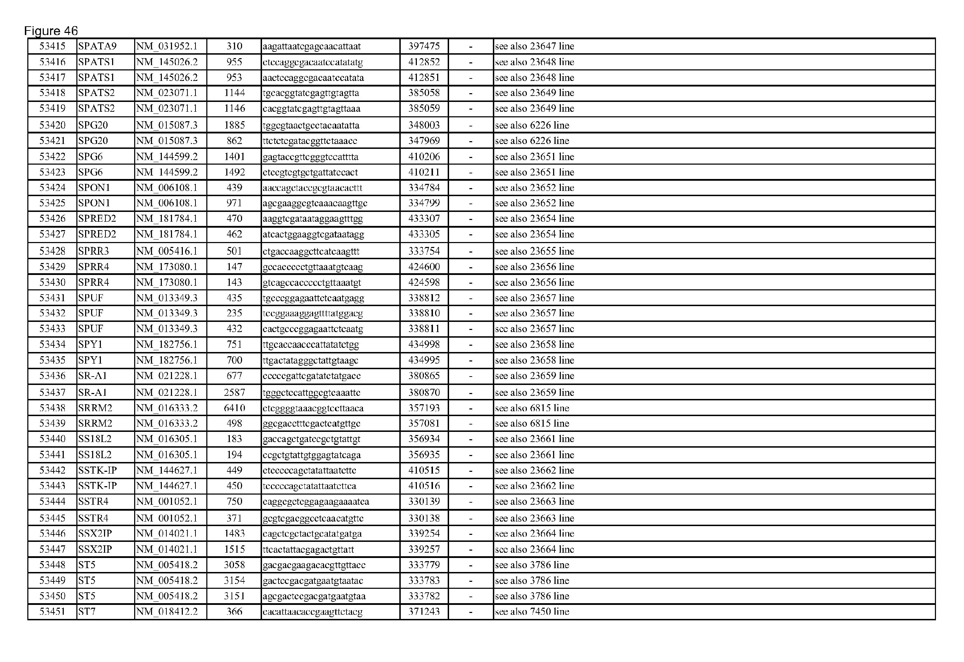

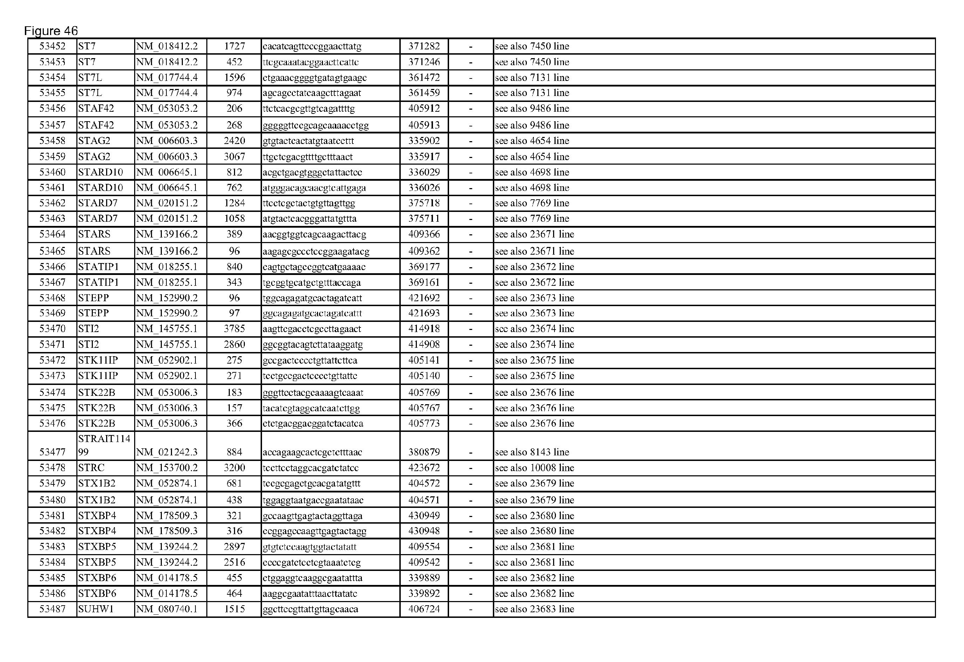

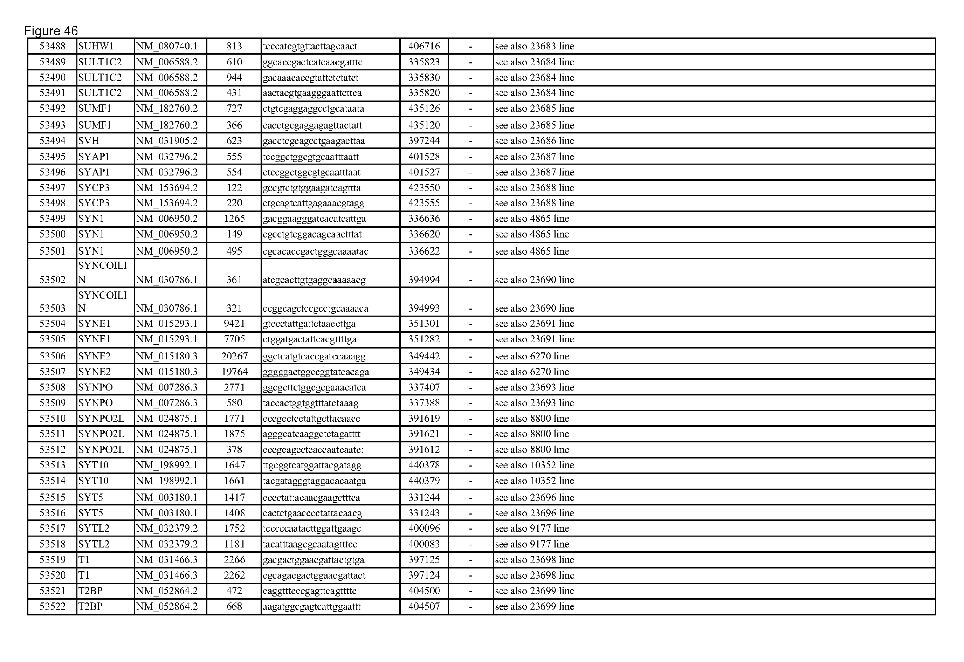

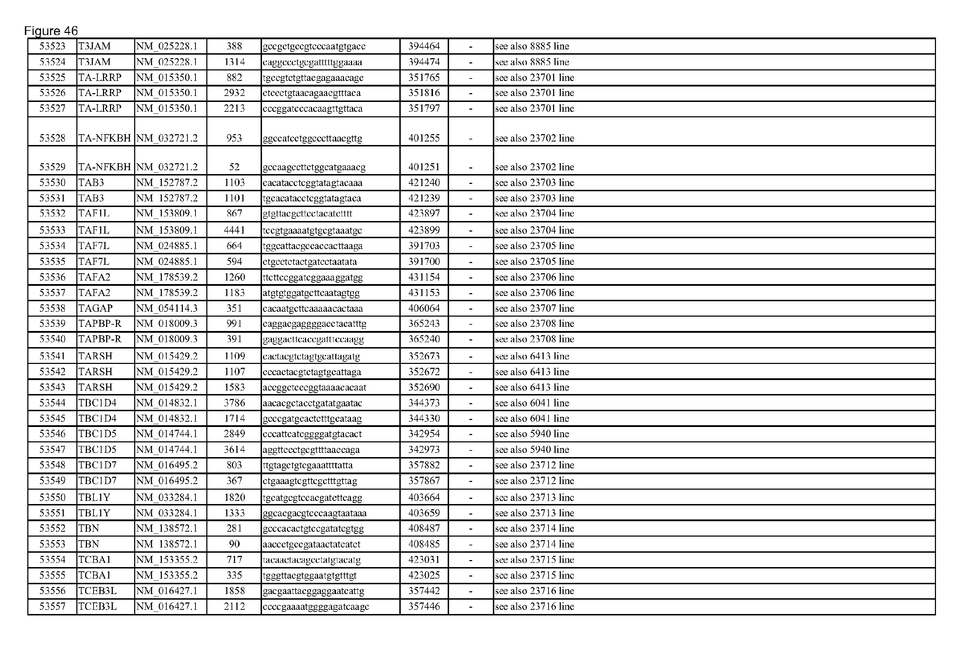

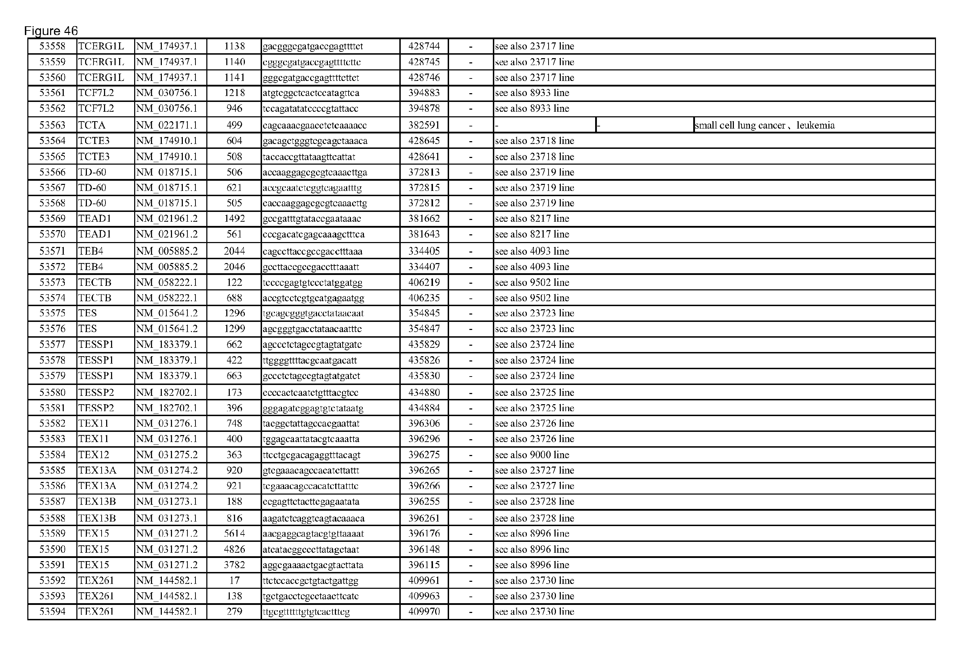

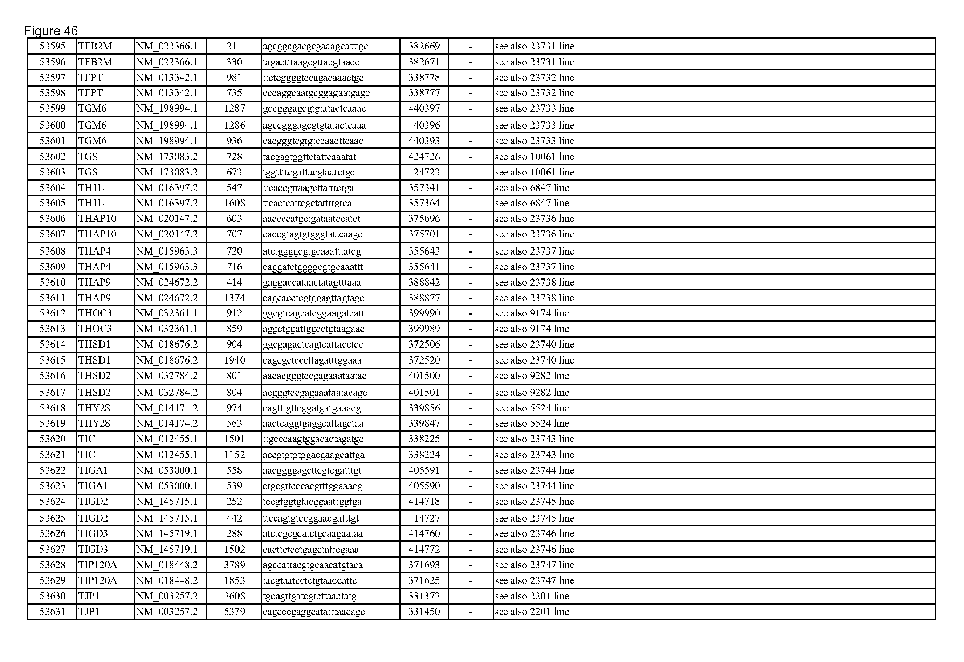

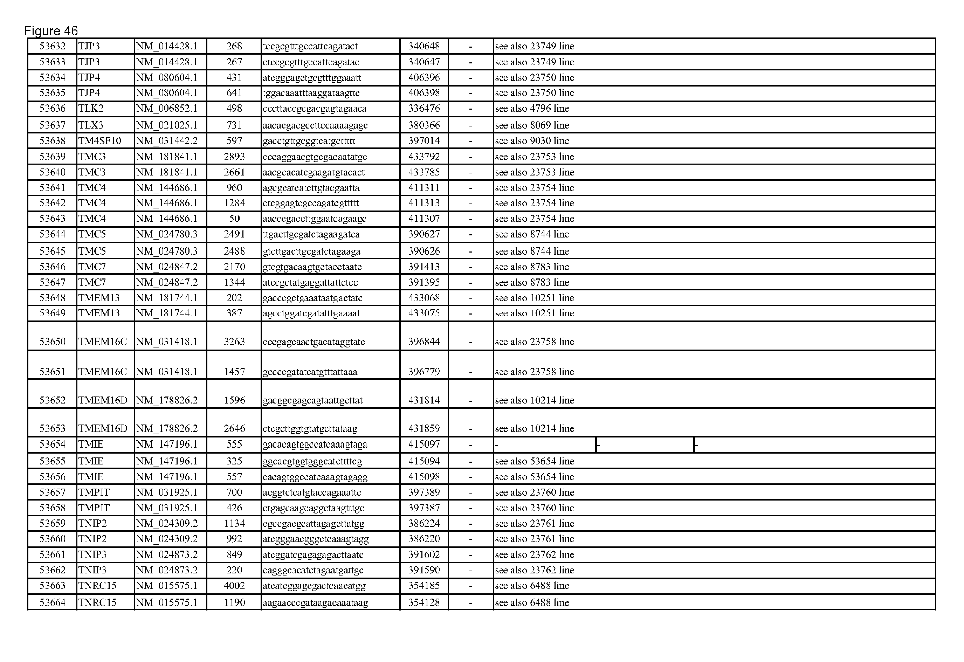

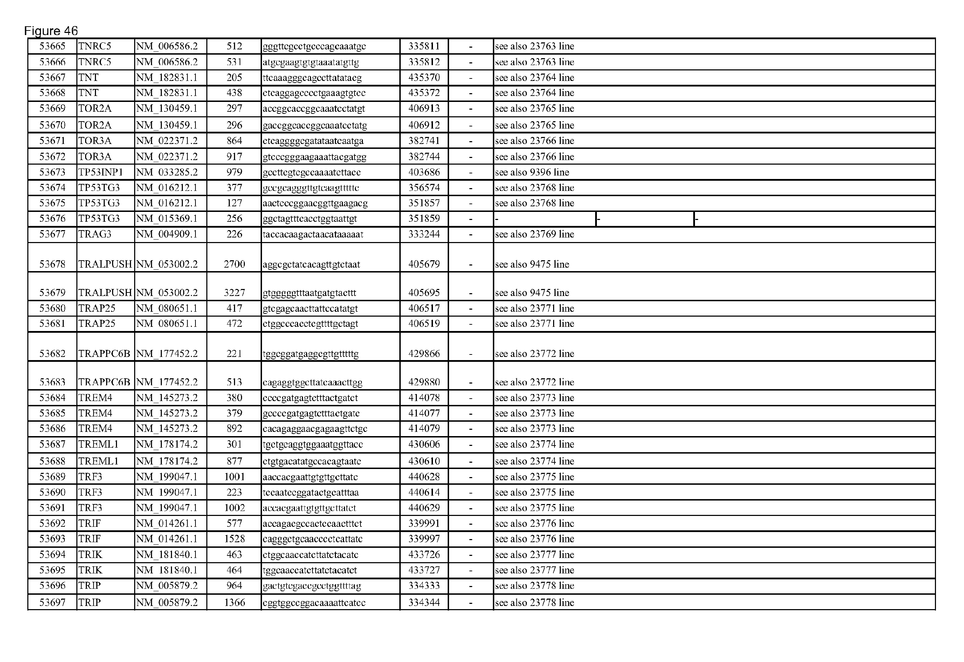

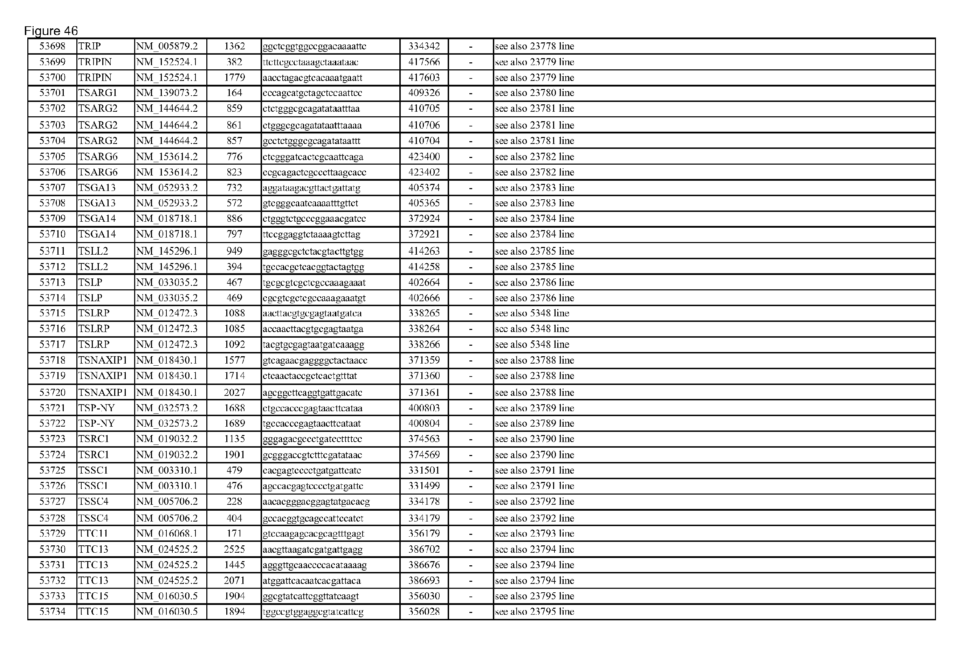

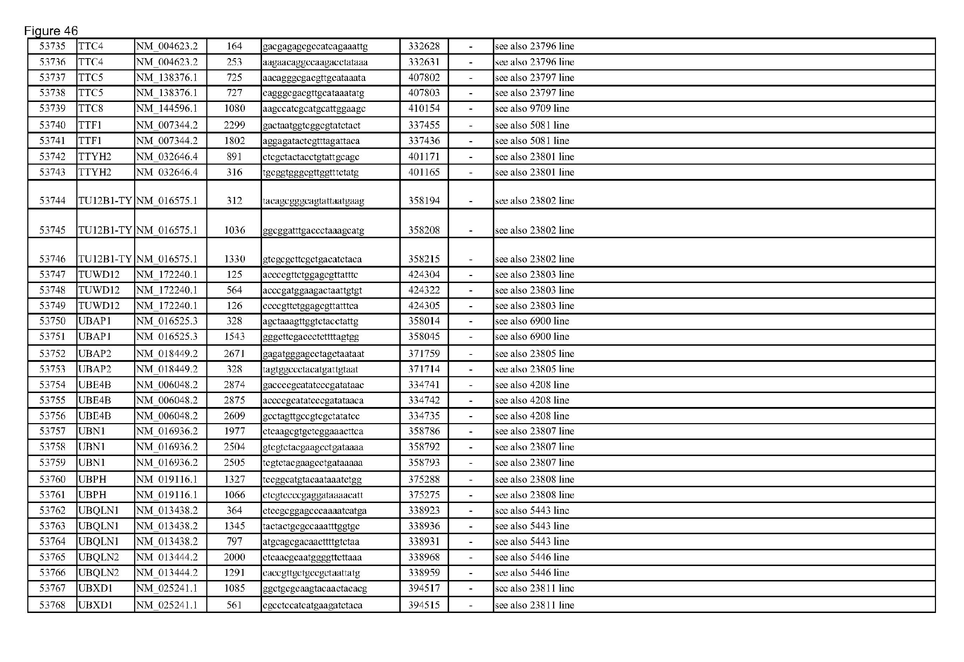

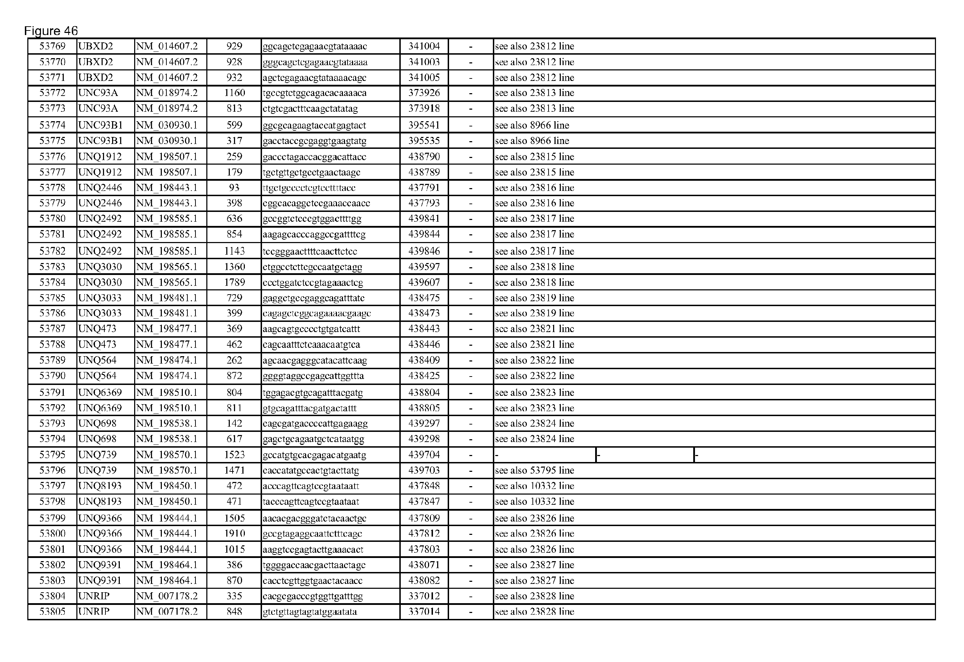

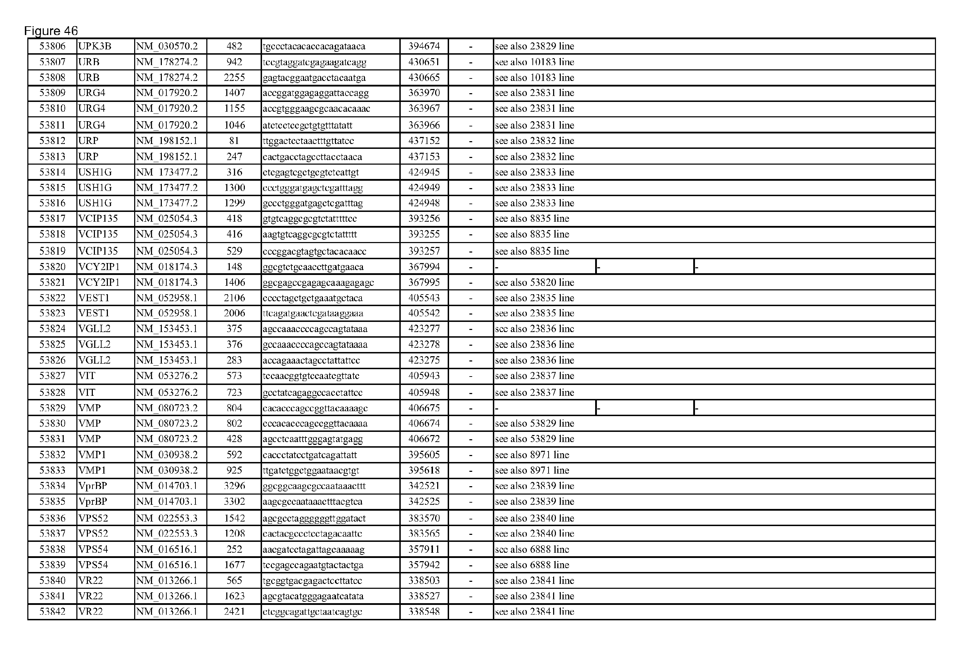

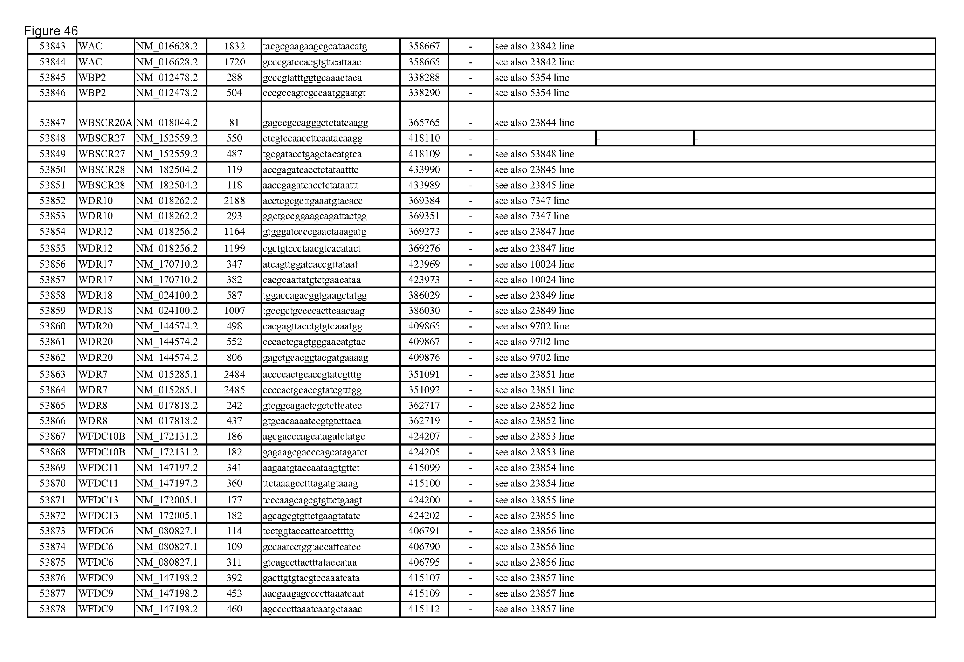

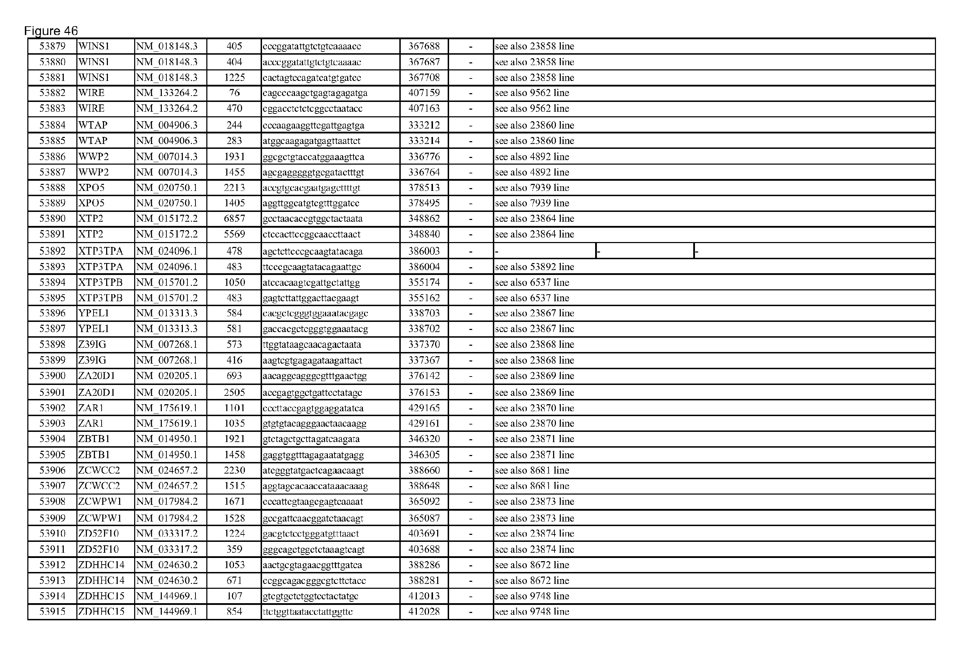

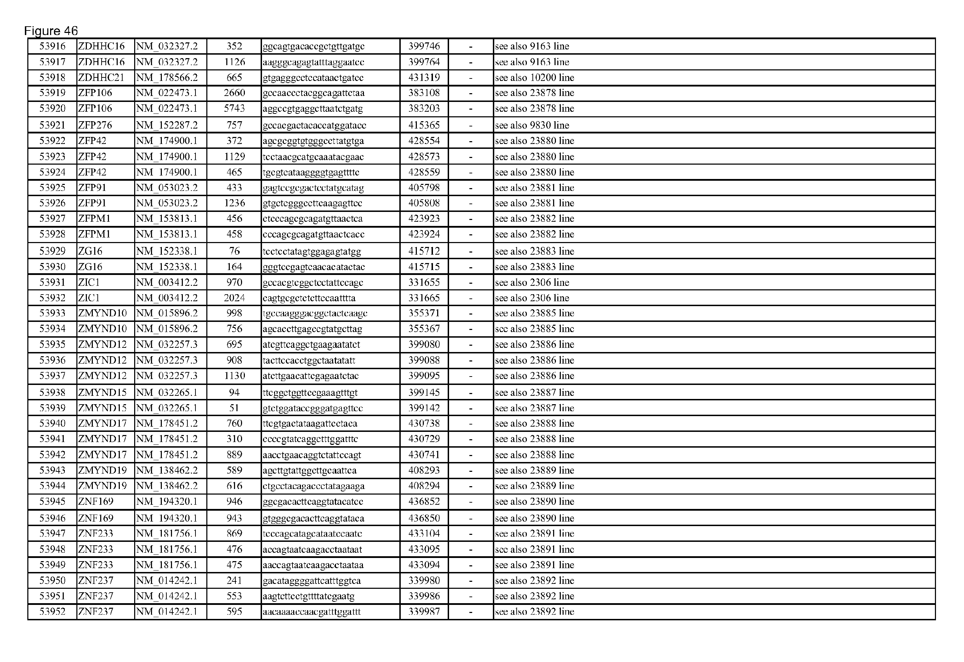

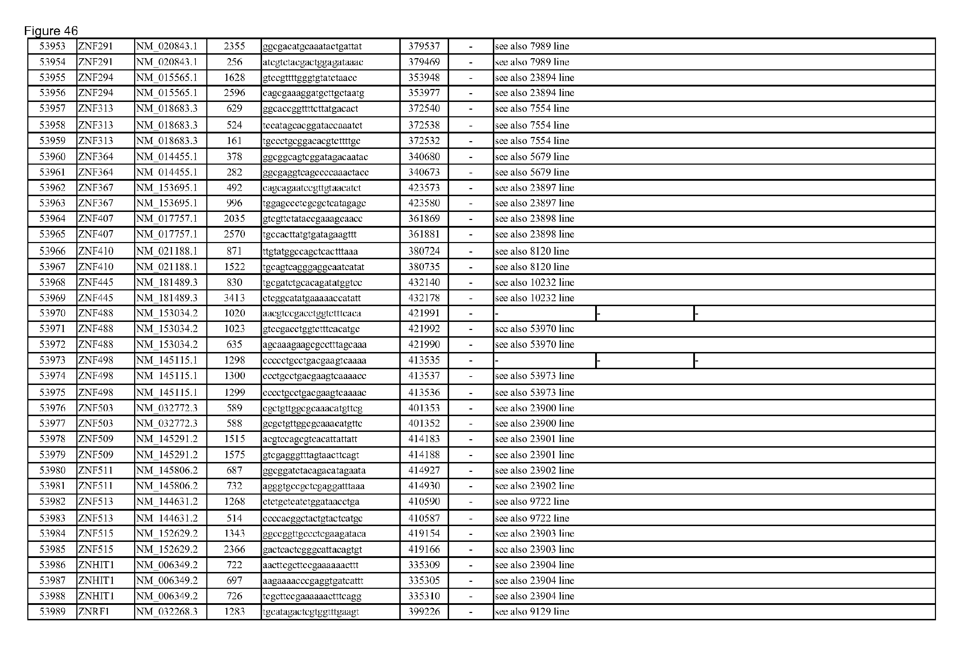

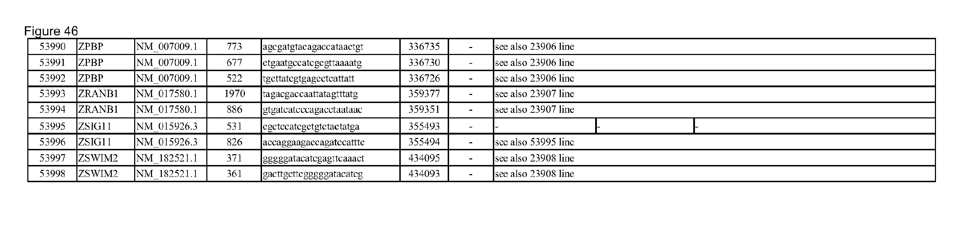

In Figure 46, "Gene Name" and "refseq_NO." correspond to the "RefSeq" database at NCBI (HYPERLINK "http://www.ncbi.nlm.nih.gov/" http://www.ncbi.nlm.nih.gov/), and information of each gene (including the sequence and function of the gene) can be obtained through access to the RefSeq database.

In Figure 46, within the sequences listed in the column "Target Sequence," actually targeted by RNAi is a portion covering the third base from the 5' end to the third base from the 3' end. Namely, the sequences listed in "Target Sequence" of Figure 46 have, at both their 5' and 3' ends, additional 2 bases adjacent to the sequence targeted by RNAi. In the specification and claims of the present application, the term "prescribed sequence" in the narrow sense means a sequence actually targeted by RNAi and corresponds to, for example, a portion covering the third base from the 5' end to the third base from the 3' end of each "target sequence" in Figure 46. For convenience sake, in the specification and claims of the present application, both terms "prescribed sequence" and "target sequence" are used, depending on the context, to mean the same sequence as the "prescribed sequence" in the narrow sense, or alternatively, to mean a sequence having additional 2 bases adjacent to the sequence targeted by RNAi, which are attached at both the 5' and 3' ends of the "prescribed sequence" in the narrow sense, as in the case of the "target sequences" in Figure 46.

In the present specification and the claims, unless otherwise specified, the term "5' end base" means the third base from the 5' end of a sequence shown in the column "Target Sequence" of Figure 46, while the term "3' end base" means the third base from the 3' end of a sequence shown in the column "Target Sequence" of Figure 46. Thus, "Target Position" in Figure 46 means a position in the sequence of each gene, which corresponds to the third base (for example, "g" in the case of the target sequence under Reference No. 1) from the 5' end of each sequence shown in the column "Target Sequence" of Figure 46.

In Figure 46, "SEQ ID NO (human)" and "SEQ ID NO (mouse)" represent the sequence identification numbers (SEQ ID NOs) of individual target sequences shown in the sequence listing attached to this specification. Target sequences under the same reference number are identical between human and mouse.

DETAILED DESCRIPTION OF THE INVENTION

In order to achieve the above object, the present inventors have studied a technique for easily obtaining siRNA, which is one of the steps requiring the greatest effort, time, and cost when the RNAi method is used. In view of the fact that preparation of siRNA is a problem especially in mammals, the present inventors have attempted to identify the sequence regularity of siRNA effective for RNA interference using mammalian cultured cell systems. As a result, it has been found that effective siRNA sequences have certain regularity, and thereby, the present invention has been completed. Namely, the present invention is as described below.

[1] A polynucleotide for causing RNA interference against a target gene selected from the genes of a target organism, which has at least a double-stranded region,
wherein one strand in the double-stranded region consists of a base sequence homologous to a prescribed sequence which is contained in the base sequences of the target gene and which conforms to the following rules (a) to (d):

    (a) The 3' end base is adenine, thymine or uracil;
    (b) The 5' end base is guanine or cytosine;
    (c) A 7-base sequence from the 3' end is rich in one or more types of bases selected from the group consisting of adenine, thymine and uracil; and
    (d) The number of bases is within a range that allows RNA interference to occur without causing cytotoxicity, and
    wherein the other strand in the double-stranded region consists of a base sequence having a sequence complementary to the base sequence homologous to the prescribed sequence.

[2] The polynucleotide according to [1], wherein at least 80% of bases in the base sequence homologous to the prescribed sequence corresponds to the base sequence of the prescribed sequence.

[3] The polynucleotide according to [1] or [2], wherein, in the rule (c), at least three bases among the seven bases are one or more types of bases selected from the group consisting of adenine, thymine and uracil.

[4] The polynucleotide according to any one of [1] to [3], wherein, in the rule (d), the number of bases is 13 to 28.

[5] The polynucleotide according to any one of [1] to [4], wherein the prescribed sequence further conforms to the following rule (e):

    (e) A sequence in which 10 or more bases of guanine or cytosine are continuously present is not contained.

[6] The polynucleotide according to [5], wherein the prescribed sequence further conforms to the following rule (f):

    (f) A sequence sharing at least 90% homology with the prescribed sequence is not contained in the base sequences of genes other than the target gene among all gene sequences of the target organism.

[7] The polynucleotide according to [6], wherein the prescribed sequence consists of the base sequence shown in any of SEQ ID NOs: 47 to 817081.

[8] The polynucleotide according to [6], wherein the prescribed sequence is any of the sequences listed in the column "Target Sequence" of Figure 46.

[9] The polynucleotide according to [6], which has any of the base sequences shown in SEQ ID NOs: 817102 to 817651.

[10] The polynucleotide according to any one of [1] to [9], which is a double-stranded polynucleotide.

[11] The polynucleotide according to [10], wherein one strand of the double-stranded polynucleotide consists of a base sequence having an overhanging portion at the 3' end of the base sequence homologous to the prescribed sequence, and the other strand of the double-stranded polynucleotide consists of a base sequence having an overhanging portion at the 3' end of the sequence complementary to the base sequence homologous to the prescribed sequence.

[12] The polynucleotide according to any one of [1] to [9], which is a single-stranded polynucleotide having a hairpin structure, wherein the single-stranded polynucleotide has a loop segment linking the 3' end of one strand in the double-stranded region and the 5' end of the other strand in the double-stranded region.

[13] A method for selecting a polynucleotide to be introduced into an expression system for a target gene whose expression is to be inhibited,

wherein the polynucleotide has at least a double-stranded region,

wherein one strand in the double-stranded region consists of a base sequence homologous to a prescribed sequence which is contained in the base sequences of the target gene and which conforms to the following rules (a) to (f):

(a) The 3' end base is adenine, thymine or uracil;
(b) The 5' end base is guanine or cytosine;
(c) A 7-base sequence from the 3' end is rich in one or more types of bases selected from the group consisting of adenine, thymine and uracil;
(d) The number of bases is within a range that allows RNA interference to occur without causing cytotoxicity;
(e) A sequence in which 10 or more bases of guanine or cytosine are continuously present is not contained; and
(f) A sequence sharing at least 90% homology with the prescribed sequence is not contained in the base sequences of genes other than the target gene among all gene sequences of the target organism, and

wherein the other strand in the double-stranded region consists of a base sequence having a sequence complementary to the base sequence homologous to the prescribed sequence.

[14] The method for selecting a polynucleotide according to [13], wherein a polynucleotide having a sequence wherein the base sequence homologous to the prescribed sequence of the target gene contains mismatches of at least 3 bases against the base sequences of genes other than the target gene, and for which there is only a minimum number of other genes having a base sequence containing the mismatches of at least 3 bases, is further selected from the selected polynucleotides.

[15] A method for inhibiting gene expression, which comprises introducing the polynucleotide according to any one of [1] to [12] into an expression system for a target gene whose expression is to be inhibited, thereby inhibiting the expression of the target gene.

[16] A method for inhibiting gene expression, which comprises introducing a polynucleotide selected by the method according to [13] or [14] into an expression system for a target gene whose expression is to be inhibited, thereby inhibiting the expression of the target gene.

[17] The method for inhibiting gene expression according to [15] or [16], wherein the expression is inhibited to 50% or below.

[18] A pharmaceutical composition which comprises a pharmaceutically effective amount of the polynucleotide according to any one of [1] to [12].

[19] The pharmaceutical composition according to [18], which is for use in treating or preventing the diseases listed in the column "Related Disease" of Figure 46.

[20] The pharmaceutical composition according to [18], which is for use in treating or preventing diseases related to the genes listed in the column "Gene Name" of Figure 46.

[21] The pharmaceutical composition according to [18], which is for use in treating or preventing a disease in which a gene belonging to any of the following 1) to 9) is involved:

1) an apoptosis-related gene;
2) phosphatase or a phosphatase activity-related gene;
3) a cell cycle-related gene;
4) a receptor-related gene;
5) an ion channel-related gene;
6) a signal transduction system-related gene;
7) kinase or a kinase activity-related gene;
8) a transcription regulation-related gene; or
9) G protein-coupled receptor or a G protein-coupled receptor-related gene.

[22] The pharmaceutical composition according to any one of [18] to [21], which comprises a polynucleotide targeting the base sequence shown in any of SEQ ID NOs listed in the column "SEQ ID NO (human)" or "SEQ ID NO (mouse)" of Figure 46.

[23] The pharmaceutical composition according to [18], which is for use in treating or preventing diseases related to the genes listed in the column "Gene Name" of Table 1.

[24] The pharmaceutical composition according to [18] or [23], which is for use in treating or preventing any cancer selected from bladder cancer, breast cancer, colorectal cancer, gastric cancer, hepatoma, lung cancer, melanoma, ovarian cancer, pancreas cancer, prostate cancer, oral cancer, skin cancer, and thyroid gland cancer.

[25] The pharmaceutical composition according to any one of [18], [23] or [24], which comprises a polynucleotide having any of the base sequences shown in SEQ ID NOs: 817102 to 817651.

[26] A composition for inhibiting gene expression to inhibit the expression of a target gene, which comprises the polynucleotide according to any one of [1] to [12].

[27] The composition for inhibiting gene expression according to [26], wherein the target gene is related to any of the diseases listed in the column "Related Disease" of Figure 46.

[28] The composition for inhibiting gene expression according to [26], wherein the target gene is any of the genes listed in the column "Gene Name" of Figure 46.

[29] The composition for inhibiting gene expression according to [26], wherein the target gene is a gene belonging to any of the following 1) to 9):

1) an apoptosis-related gene;
2) phosphatase or a phosphatase activity-related gene;
3) a cell cycle-related gene;
4) a receptor-related gene;
5) an ion channel-related gene;
6) a signal transduction system-related gene;
7) kinase or a kinase activity-related gene;
8) a transcription regulation-related gene; or
9) G protein-coupled receptor or a G protein-coupled receptor-related gene.

[30] The composition for inhibiting gene expression according to [26], wherein the target gene is any of the genes listed in the column "Gene Name" of Table 1.

[31] The composition for inhibiting gene expression according to [26], wherein the target gene is related to any cancer selected from bladder cancer, breast cancer, colorectal cancer, gastric cancer, hepatoma, lung cancer, melanoma, ovarian cancer, pancreas cancer, prostate cancer, oral cancer, skin cancer, and thyroid gland cancer.

[32] A method for treating or preventing the diseases listed in the column "Related Disease" of Figure 46, which comprises administering a pharmaceutically effective amount of the polynucleotide according to any one of [1] to [12].

## ADVANTAGES OF THE INVENTION

The polynucleotide of the present invention not only has a high RNA interference effect on its target gene, but also has a very small risk of causing RNA interference against a gene unrelated to the target gene, so that the polynucleotide of the present invention can cause RNA interference specifically only to the target gene whose expression is to be inhibited. Thus, the polynucleotide of the present invention is preferred for use in, e.g., tests and therapies using RNA interference, and is particularly effective in performing RNA interference in higher animals such as mammals, especially humans.

## EMBODIMENTS FOR CARRYING OUT THE INVENTION

The embodiments of the present invention will be described below in the order of the columns <1> to <7>.

<1> Method for searching target base sequence of RNA interference
<2> Method for designing base sequence of polynucleotide for causing RNA interference
<3> Method for producing polynucleotide
<4> Method for inhibiting gene expression
<5> siRNA sequence design program
<6> siRNA sequence design business model system
<7> Base sequence processing apparatus for running siRNA sequence design program, etc.
<8> Pharmaceutical composition

<9> Composition for inhibiting gene expression
<10> Method for treating or preventing diseases

<1> Method for searching target base sequence of RNA interference

The search method of the present invention is a method for searching a base sequence, which causes RNA interference, from the base sequences of a target gene selected from the genes of a target organism. The target organism, to which RNA interference is to be caused, is not particularly limited and may be a microorganism such as a prokaryotic organism (including E. coli), yeast or a fungus, an animal (including a mammal), an insect, a plant or the like.

Specifically, in the search method of the present invention, a sequence segment conforming to the following rules (a) to (d) is searched from the base sequences of a target gene for RNA interference.

(a) The 3' end base is adenine, thymine or uracil.
(b) The 5' end base is guanine or cytosine.
(c) A 7-base sequence from the 3' end is rich in one or more types of bases selected from the group consisting of adenine, thymine and uracil.
(d) The number of bases is within a range that allows RNA interference to occur without causing cytotoxicity.

The term "gene" in the term "target gene" means a medium which codes for genetic information. The "gene" consists of a substance, such as DNA, RNA, or a complex of DNA and RNA, which codes for genetic information. As the genetic information, instead of the substance itself, electronic data of base sequences can be handled in a computer or the like. The "target gene" may be set as one coding region, a plurality of coding regions, or all the polynucleotides whose sequences have been revealed. When a gene with a particular function is desired to be searched, by setting only the particular gene as the target, it is possible to efficiently search the base sequences which cause RNA interference specifically in the particular gene. Namely, RNA interference is known as a phenomenon which destructs mRNA by interference, and by selecting a particular coding region, search load can be reduced. Moreover, a group of transcription regions may be treated as the target region to be searched. Additionally, in the present specification, base sequences are shown on the basis of sense strands; i.e., sequences of mRNA, unless otherwise described. Furthermore, in the present specification, a base sequence which satisfies the rules (a) to (d) is referred to as a "prescribed sequence". In the rules, thymine corresponds to a DNA base sequence, and uracil corresponds to an RNA base sequence.

The rule (c) regulates so that a sequence in the vicinity of the 3' end contains a rich amount of type(s) of base (s) selected from the group consisting of adenine, thymine, and uracil, and more specifically, as an index for search, regulates so that a 7-base sequence from the 3' end is rich in one or more types of bases selected from adenine, thymine, and uracil.

In the rule (c), the phrase "sequence rich in" means that the frequency of a given base appearing is high, and schematically, a 5 to 10-base sequence, preferably a 7-base sequence, from the 3' end in the prescribed sequence contains one or more types of bases selected from adenine, thymine, and uracil in an amount of preferably at least 40% or more, and more preferably at least 50%. More specifically, for example, in a prescribed sequence of about 19 bases, among 7 bases from the 3' end, preferably at least 3 bases, more preferably at least 4 bases, and particularly preferably at least 5 bases, are one or more types of bases selected from the group consisting of adenine, thymine, and uracil.

The means for confirming the correspondence to the rule (c) is not particularly limited as long as it can be confirmed that preferably at least 3 bases, more preferably at least 4 bases, and particularly preferably at least 5 bases, among 7 bases are adenine, thymine, or uracil. For example, a case, wherein inclusion of 3 or more bases which correspond to one or more types of bases selected from the group consisting of adenine, thymine, and uracil in a 7-base sequence from the 3' end is defined as being rich, will be described below. Whether the base is any one of the three types of bases is checked from the first base at the 3' end one after another, and when three corresponding bases appear by the seventh base, conformation to the rule (c) is determined. For example, if three corresponding bases appear by the third base, checking of three bases is sufficient. That is, in the search with respect to the rule (c), it is not always necessary to check all of the seven bases at the 3' end. Conversely, non-appearance of three or more corresponding bases by the seventh base means being not rich, thus being determined that the rule (c) is not satisfied.

In a double-stranded polynucleotide, it is well-known that adenine complementarily forms hydrogen-bonds to thymine or uracil. In the complementary hydrogen bond between guanine and cytosine (G-C hydrogen bond), three hydrogen bonding sites are formed. On the other hand, the complementary hydrogen bond between adenine and thymine or uracil (A-(T/U) hydrogen bond) includes two hydrogen bonding sites. Generally speaking, the bonding strength of the A-(T/U) hydrogen bond is weaker than that of the G-C hydrogen bond.

In the rule (d), the number of bases of the base sequence to be searched is regulated. The number of bases of the base sequence to be searched corresponds to the number of bases capable of causing RNA interference. Depending on the conditions, for example the species of an organism, in cases of siRNA having an excessively large number of bases, cytotoxicity is known to occur. The upper limit of the number of bases varies depending on the species of organism to which RNA interference is desired to be caused. The number of bases of the single strand constituting siRNA is preferably 30 or less regardless of the species. Furthermore, in mammals, the number of bases is preferably 24 or less, and more preferably 22 or less. The lower limit, which is not particularly limited as long as RNA interference is caused, is preferably at least 15, more preferably at least 18, and still more preferably at least 20. With respect to the number of bases as a single strand constituting siRNA, searching with a number of 21 is particularly preferable.

Furthermore, although a description will be made below, in siRNA, an overhanging portion is provided at the 3' end of the prescribed sequence. The number of bases in the overhanging portion is preferably 2. Consequently, the upper limit of the number of bases in the prescribed sequence only, excluding the overhanging portion, is preferably 28 or less, more preferably 22 or less, and still more preferably 20 or less, and the lower limit is preferably at least 13, more preferably at least 16, and still more preferably at least 18. In the prescribed sequence, the most preferable number of bases is 19. The target base sequence for RNAi may be searched either including or excluding the overhanging portion.

Base sequences conforming to the prescribed sequence have an extremely high probability of causing RNA interference. Consequently, in accordance with the search method of the present invention, it is possible to search sequences that cause RNA interference with extremely high probability, and designing of polynucleotides which cause RNA interference can be simplified.

In another preferred example, the prescribed sequence may be a sequence further conforming to the following rule (e). (e) A sequence in which 10 or more bases of guanine or cytosine are continuously present is not contained.

The rule (e) regulates so that the base sequence to be searched does not contain a sequence in which 10 or more bases of guanine (G) and/or cytosine (C) are continuously present. Examples of the sequence in which 10 or more bases of guanine and/or cytosine are continuously present include a sequence in which either guanine or cytosine is continuously present as well as a sequence in which a mixed sequence of guanine and cytosine is present. More specific examples include GGGGGGGGGG, CCCCCCCCCC, and a mixed sequence of GCGGCCCGCG.

In order to prevent RNA interference from occurring in genes not related to the target gene, preferably, a search is made to determine whether a sequence that is identical or similar to the designed sequence is included in the other genes. A search for the sequence that is identical or similar to the designed sequence may be performed using software capable of performing a general homology search, etc. In this case, in consideration of the RNAi effect caused by two strands (sense and antisense strands) of siRNA, a search is more preferably made on both the "designed sequence" and a "sequence having a base sequence complementary to the designed sequence (complementary sequence)" to determine whether an identical or similar sequence is included in the other genes. When sequences having a sequence that is identical/similar to the designed sequence or its complementary sequence are excluded from the designed sequences, it is possible to design a sequence which causes RNA interference specifically to the target gene only.

Thus, when sequences for which other genes have similar sequences containing a small number of mismatches in their base sequences are excluded from the designed sequences, it is possible to select a sequence with high specificity. For example, in the case of designing a base sequence of 19 bases, it is preferable to exclude sequences for which other genes have similar sequences containing mismatches of 2 or less bases. In this case, if the number of mismatches, a threshold for similarity determination, is set at a higher value, a sequence to be designed will have a higher specificity. In the case of designing a base sequence of 19 bases, it is more preferable to exclude sequences for which other genes have similar sequences containing mismatches of 3 or less bases, and it is still more preferable to exclude sequences for which other genes have similar sequences containing mismatches of 4 or less bases. Moreover, when sequences for which other genes have similar sequences containing a small number of mismatches in their base sequences are excluded with respect to both a sequence having the prescribed sequence and its complementary sequence, such exclusion is preferred because it is possible to design a sequence with a higher specificity.

The number of mismatches, a criterion for determining sequence similarity, will also vary depending on the number of bases in a sequence to be designed, and is therefore difficult to define sweepingly. Given that the number of mismatches in a base sequence is defined by homology, a search may be made to determine whether the base sequence conforms to the following rule (f). (f) A sequence sharing at least 90% homology with the prescribed sequence is not contained in the base sequences of genes other than the target gene among all gene sequences of the target organism.

In the rule (f), the base sequences of genes other than the target gene preferably do not contain a sequence sharing at least 85% homology with the prescribed sequence, more preferably do not contain a sequence sharing at least 80% homology with the prescribed sequence, and still more preferably do not contain a sequence sharing at least 75% homology with the prescribed sequence. Moreover, when sequences for which other genes have similar sequences with high base sequence homology are excluded with respect to both a sequence having the prescribed sequence and its complementary sequence, such exclusion is preferred because it is possible to design a sequence with a higher specificity.

Furthermore, in the search of the prescribed sequence, detection can be efficiently performed by using a computer installed with a program which allows a search of segments conforming to the rules (a) to (c), etc., after determining the number of bases. More specific embodiments will be described below in the columns <5> siRNA sequence design program and <7> Base sequence processing apparatus for running siRNA sequence design program.

The polynucleotides shown in the sequence listing of the present application under SEQ ID NOs: 47 to 817081 are human and mouse sequences that are selected as prescribed sequences conforming to the above rules (a) to (f) or that are selected as target sequences containing the prescribed sequences.

<2> Method for designing base sequence of polynucleotide for causing RNA interference

In the method for designing a base sequence in accordance with the present invention, a base sequence of polynucleotide which causes RNA interference is designed on the basis of the base sequence searched by the search method described above. A polynucleotide for causing RNA interference is a polynucleotide having a double-stranded region designed on the basis of the prescribed sequence searched by the above search method. Such a polynucleotide is not particularly limited as long as it can cause RNA interference against a target gene.

Polynucleotides for causing RNA interference may be principally classified into a double-stranded type (e.g., siRNA) and a single-stranded type (e.g., RNA with a hairpin structure (short hairpin RNA: shRNA)).

Although siRNA and shRNA are mainly composed of RNA, they also include hybrid polynucleotides partially containing DNA. In the method for designing a base sequence in accordance with the present invention, a base sequence conforming to the rules (a) to (d) is searched from the base sequences of a target gene, and a base sequence homologous to the searched base sequence is designed. In another preferred design example, it may be possible to take into consideration the above rules (e) and (f), etc. The rules (a) to (d) and the search method are the same as those described above regarding the search method of the present invention.

With respect to the double-stranded region in the polynucleotide for causing RNA interference, one strand consists of a base sequence homologous to a prescribed sequence which is contained in the base sequences of a target gene and which conforms to the above rules (a) to (d), and the other strand consists of a base sequence having a sequence complementary to the base sequence homologous to the prescribed sequence. The term "homologous sequence" refers to the same sequence and a sequence in which mutations, such as deletions, substitutions, and additions, have occurred to the same sequence to an extent that the function of causing the RNA interference has not been lost. Although depending on the conditions, such as the type and sequence of the target gene, the range of the allowable mutation, in terms of homology, is preferably 80% or more, more preferably 90% or more, and still more preferably 95% or more. When homology in the range of the allowable mutation is calculated, desirably, the numerical values calculated using the same search algorithm are compared. The search algorithm is not particularly limited. A search algorithm suitable for searching for local sequences is preferable. More specifically, BLAST, ssearch, or the like is preferably used.

More specifically, the percent identity between nucleic acids (polynucleotides) can be determined by visual inspection and mathematical calculation. Alternatively, the percent identity of two nucleic acid sequences can be determined by visual inspection and mathematical calculation, or more preferably, the comparison is done by comparing sequence information using a computer program. An exemplary, preferred computer program is the Genetic Computer Group (GCG; Madison, WI) Wisconsin package version 10.0 program, "GAP" (Devereux et al., 1984, Nucl. Acids Res. 12:387). In addition to making a comparison between two nucleic acid sequences, this "GAP" program can be used for comparison between two amino acid sequences and between a nucleic acid sequence and an amino acid sequence. The preferred default parameters for the "GAP" program includes: (1) The GCG implementation of a unary comparison matrix (containing a value of 1 for identities and 0 for non-identities) for nucleotides, and the weighted amino acid comparison matrix of Gribskov and Burgess, Nucl. Acids Res. 14:6745, 1986, as described by Schwartz and Dayhoff, eds., Atlas of Polypeptide Sequence and Structure, National Biomedical Research Foundation, pp. 353-358, 1979; or other comparable comparison matrices; (2) a penalty of 30 for each gap and an additional penalty of 1 for each symbol in each gap for amino acid sequences, or penalty of 50 for each gap and an additional penalty of 3 for each symbol in each gap for nucleotide sequences; (3) no penalty for end gaps; and (4) no maximum penalty for long gaps. Other programs used by those skilled in the art of sequence comparison can also be used, such as, for example, the BLASTN program version 2.2.7, available for use via the National Library of Medicine website: http://www.ncbi.nlm.nih.gov/blast/bl2seq/bls.html, or the UW-BLAST 2.0 algorithm. Standard default parameter settings for UW-BLAST 2.0 are described at the following Internet site: http://blast.wustl.edu. In addition, the BLAST algorithm uses the BLOSUM62 amino acid scoring matrix, and optional parameters that can be used are as follows: (A) inclusion of a filter to mask segments of the query sequence that have low compositional complexity (as determined by the SEG program of Wootton and Federhen (Computers and Chemistry, 1993); also see Wootton and Federhen, 1996, Analysis of compositionally biased regions in sequence databases, Methods Enzymol. 266: 554-71) or segments consisting of short-periodicity internal repeats (as determined by the XNU program of Claverie and States (Computers and Chemistry, 1993)), and (B) a statistical significance threshold for reporting matches against database sequences, or E-score (the expected probability of matches being found merely by chance, according to the stochastic model of Karlin and Altschul, 1990; if the statistical significance ascribed to a match is greater than this E-score threshold, the match will not be reported.); preferred E-score threshold values are 0.5, or in order of increasing preference, 0.25, 0.1, 0.05, 0.01, 0.001, 0.0001, 1e-5, 1e-10, 1e-15, 1e-20, 1e-25, 1e-30, 1e-40, 1e-50, 1e-75, or 1e-100.

The polynucleotide of the present invention also includes a polynucleotide that is hybridizable, as a "base sequence homologous" to a prescribed sequence conforming to the above rules (a) to (d), to the prescribed sequence under stringent conditions (e.g., under moderately or highly stringent conditions) and that preferably has the ability to cause RNA interference.

The term "under stringent condition" means that two sequences can hybridize under moderately or highly stringent conditions. More specifically, moderately stringent conditions can be readily determined by those having ordinary skill in the art, e.g., depending on the length of DNA. The basic parameters affecting the choice of hybridization conditions are set forth by Sambrook et al., Molecular Cloning: A Laboratory Manual, third edition, chapters 6 and 7, Cold Spring Harbor Laboratory Press, 2001 and include the use of a prewashing solution for nitrocellulose filters 5 x SSC, 0.5% SDS, 1.0 mM EDTA (pH 8.0), hybridization conditions of about 50% formamide, 2 x SSC to 6 x SSC at about 40-50°C (or other similar hybridization solutions, such as Stark's solution, in about 50% formamide at about 42°C) and washing conditions of about 60°C, 0.5 x SSC, 0.1% SDS. Preferably, moderately stringent conditions may include hybridization at about 50°C and 6 x SSC. Highly stringent conditions can also be readily determined by those skilled in the art, e.g., depending on the length of DNA. Generally, such conditions include hybridization and/or washing at higher temperature and/or lower salt concentration (such as hybridization at about 65°C, 6 x SCC-0.2 x SSC, preferably 6 x SCC, more preferably 2 x SSC, most preferably 0.2 x SSC), compared to the moderately stringent conditions. For example, highly stringent conditions may include hybridization as defined above, and washing at approximately 68°C, 0.2 × SSC, 0.1% SDS. SSPE (1 × SSPE is 0.15 M NaCl, 10 mM $NaH_2PO_4$, and 1.25 mM EDTA, pH 7.4) can be substituted for SSC (1 × SSC is 0.15 M NaCl and 15 mM sodium citrate) in the hybridization and wash buffers; washes are performed for 15 minutes after hybridization is completed.

It should be understood that the wash temperature and wash salt concentration can be adjusted as necessary to achieve a desired degree of stringency by applying the basic principles that govern hybridization reactions and duplex stability, as known to those skilled in the art and described further below (see, e.g., Sambrook et al., 2001). When hybridizing a nucleic acid to a target nucleic acid of unknown sequence, the hybrid length is assumed to be that of the hybridizing nucleic acid. When nucleic acids of known sequence are hybridized, the hybrid length can be determined by aligning the sequences of the nucleic acids and identifying the region or regions of optimal sequence complementarity. The hybridization temperature for hybrids anticipated to be less than 50 base pairs in length should be 5°C to 25°C less than the melting temperature (Tm) of the hybrid, where Tm is determined according to the following equations. For hybrids less than 18 base pairs in length, Tm (°C) = 2(number of A + T bases) + 4(number of G + C bases). For hybrids above 18 base pairs in length, Tm (°C) = 81.5°C + 16.6($\log_{10}$ [Na$^+$]) + 0.41(molar fraction [G + C]) - 0.63(% formamide - (500/N), where N is the number of bases in the hybrid, and [Na$^+$] is the concentration of sodium ions in the hybridization buffer ([Na$^+$] for 1 ×SSC = 0.165 M).

As described above, although slight modification of the searched sequence is allowable, it is particularly preferred that the number of bases in the base sequence to be designed be the same as that of the searched sequence. For example, with respect to the allowance for change under the same number of bases, the bases of the base sequence to be designed correspond to those of the sequence searched at a rate of preferably 80% or more, more preferably 90% or more, and particularly preferably 95% or more. For example, when a base sequence having 19 bases is designed, preferably 16 or more bases, more preferably 18 or more bases, correspond to those of the searched base sequence. Furthermore, when a sequence homologous to the searched base sequence is designed, desirably, the 3' end base of the base sequence searched is the same as the 3' end base of the base sequence designed, and also desirably, the 5' end base of the base sequence searched is the same as the 5' end base of the base sequenced designed.

An overhanging portion is usually provided on a siRNA molecule. The overhanging portion is a protrusion provided on the 3' end of each strand in a double-stranded RNA molecule. Although depending on the species of organism, the number of bases in the overhanging portion is preferably 2. Basically, any base sequence is acceptable in the overhanging portion. In some cases, the same base sequence as that of the target gene to be searched, TT, UU, or the like may be preferably used. As described above, by providing the overhanging portion at the 3' end of the prescribed sequence which has been designed so as to be homologous to the base sequence searched, a sense strand constituting siRNA is designed.

Alternatively, it may be possible to search the prescribed sequence with the overhanging portion being included from the start to perform designing. The preferred number of bases in the overhanging portion is 2. Consequently, for example, in order to design a single strand constituting siRNA including a prescribed sequence having 19 bases and an overhanging portion having 2 bases, as the number of bases of siRNA including the overhanging portion, a sequence of 21 bases is searched from the target gene. Furthermore, when a double-stranded state is searched, a sequence of 23 bases may be searched.

shRNA is a single-stranded polynucleotide in which the 3' end of one strand in the double-stranded region and the 5' end of the other strand in the double-stranded region are linked through a loop segment. shRNA may have a protrusion in a single-stranded state at the 5' end of the one strand and/or at the 3' end of the other strand. Such shRNA can be designed according to known procedures as found in WO01/49844.

In the method for designing a base sequence in accordance with the present invention, as described above, a given sequence is searched from a desired target gene. The target to which RNA interference is intended to be caused does not necessarily correspond to the origin of the target gene, and is also applicable to an analogous species, etc. For example, it is possible to design siRNA used for a second species that is analogous to a first species using a gene isolated from the first species as a target gene. Furthermore, it is possible to design siRNA that can be widely applied to mammals, for example, by searching a common sequence from two or more species of mammals and searching a prescribed sequence from the common sequence to perform designing. The reason for this is that it is highly probable that the sequence common to two or more mammals exists in other mammals.

In the design method of the present invention, RNA molecules that cause RNA interference can be easily designed with high probability. Although synthesis of RNA still requires effort, time, and cost, the design method of the present invention can greatly minimize them.

<3> Method for producing polynucleotide

By the method for producing a polynucleotide in accordance with the present invention, a polynucleotide that has a high probability of causing RNA interference can be produced. For the polynucleotide of the present invention, a base sequence of the polynucleotide is designed in accordance with the method for designing the base sequence of the present invention described above, and a polynucleotide is synthesized so as to follow the sequence design. Although, as described above, the polynucleotide of the present invention includes both double-stranded type (e.g., siRNA) and single-stranded type (e.g., shRNA), the following explanation will be made principally for double-stranded polynucleotides.

Preferred embodiments in the sequence design are the same as those described above regarding the method for designing the base sequence. Additionally, the double-stranded polynucleotide produced by the production method of the present invention is preferably composed of RNA, but a hybrid polynucleotide which partially contains DNA may be acceptable. In this specification, double-stranded polynucleotides partially containing DNA are also included in the concept of siRNA. Also, RNA and DNA constituting the polynucleotide may have chemical modifications such as methylation of sugar hydroxyl groups. For example, siRNA in this specification may have a hybrid structure composed of a DNA strand and an RNA strand. Although such a hybrid structure is not particularly limited as long as it provides the ability to inhibit the expression of a target gene when introduced into a recipient, it is desired that such a hybrid polynucleotide is a double-stranded polynucleotide having a sense strand composed of DNA and an antisense strand composed of RNA.

Alternatively, siRNA in this specification may also have a chimeric structure. The chimeric structure refers to a structure containing both DNA and RNA in a single-stranded polynucleotide. Such a chimeric structure is not particularly limited as long as it provides the ability to inhibit the expression of a target gene when introduced into a recipient. According to the research conducted by the present inventors, siRNA tends to have structural and functional asymmetry, and in view of the object of causing RNA interference, a half of the sense strand at the 5' end side and a half of the antisense strand at the 3' end side are desirably composed of RNA.

Incidentally, in siRNA having a chimeric structure, the content of RNA is preferably minimized in terms of in vivo stability in a recipient and production costs, etc. To this end, the inventors have made extensive and intensive efforts to study siRNA whose RNA content can be reduced while maintaining a high inhibitory effect on the expression of a target gene. As a result, the inventors have obtained the results indicating that a portion of 9 to 13 nucleotides from the 5' end of the sense strand and a portion of 9 to 13 nucleotides from the 3' end of the antisense strand (e.g., portions of 11 nucleotides, preferably 10 nucleotides, more preferably 9 nucleotides, from the above respective ends of the sense and antisense strands) are desirably composed of RNA and, in particular, the 3' end side of the antisense strand desirably has such a structure. The positions of RNA portions in the sense and antisense strands are not necessarily matched.

In a double-stranded polynucleotide, one strand is formed by providing an overhanging portion to the 3' end of a base sequence homologous to the prescribed sequence conforming to the rules (a) to (d) contained in the base sequence of the target gene, and the other strand is formed by providing an overhanging portion to the 3' end of a base sequence complementary to the base sequence homologous to the prescribed sequence. The number of bases in each strand, including the overhanging portion, is 18 to 24, more preferably 20 to 22, and particularly preferably 21. The number of bases in the overhanging portion is preferably 2. siRNA having 21 bases in total in which the overhanging portion is composed of 2 bases is suitable for causing RNA interference with high probability without causing cytotoxicity even in mammals.

RNA may be synthesized, for example, by chemical synthesis or by standard biotechnology. In one technique, a DNA strand having a predetermined sequence is produced, single-stranded RNA is synthesized using the produced DNA strand as a template in the presence of a transcriptase, and the synthesized single-stranded RNA is formed into double-stranded RNA.

With respect to the basic technique for molecular biology, there are many standard, experimental manuals, for example, BASIC METHODS IN MOLECULAR BIOLOGY (1986); Sambrook et al., MOLECULAR CLONING; A LABORATORY MANUAL, Second Edition, Cold Spring Harbor Laboratory Press, Cold Spring Harbor, N.Y. (1989); Saibo-Kogaku Handbook (Handbook for cell engineering), edited by Toshio Kuroki et al., Yodosha (1992); and Shin-Idenshi-Kogaku Handbook (New handbook for genetic engineering), edited by Muramatsu et al., Yodosha (1999).

One preferred embodiment of polynucleotide produced by the production method of the present invention is a double-stranded polynucleotide produced by a method in which a sequence segment including 13 to 28 bases conforming to the rules (a) to (d) is searched from a base sequence of a target gene for RNA interference, one strand is formed by providing an overhanging portion at the 3' end of a base sequence homologous to the prescribed sequence following the rules (a) to (d), the other strand is formed by providing an overhanging portion at the 3' end of a sequence complementary to the base sequence homologous to the prescribed sequence, and synthesis is performed so that the number of bases in each strand is 15 to 30. The resulting polynucleotide has a high probability of causing RNA interference.

It is also possible to prepare an expression vector which expresses siRNA. By placing a vector which expresses a sequence containing the prescribed sequence under a condition of a cell line or cell-free system in which expression is allowed to occur, it is possible to supply predetermined siRNA using the expression vector.

Since conventional designing of siRNA has depended on the experiences and intuition of the researcher, trial and error have often been repeated. However, by the double-stranded polynucleotide production method in accordance with the present invention, it is possible to produce a double-stranded polynucleotide which causes RNA interference with high probability. In accordance with the search method, sequence design method, or polynucleotide production method of the present invention, it is possible to greatly reduce effort, time, and cost required for various experiments, manufacturing, etc., which use RNA interference. Namely, the present invention greatly simplifies various experiments, research, development, manufacturing, etc., in which RNA interference is used, such as gene analysis, search for targets for new drug development, development of new drugs, gene therapy, and research on differences between species, and thus efficiency can be improved.

In one embodiment, the present invention also provides a method for selecting the polypeptide of the present invention described above. More specifically, the present invention provides a method for selecting a polynucleotide to be introduced into an expression system for a target gene whose expression is to be inhibited,
wherein the polynucleotide has at least a double-stranded region,
wherein one strand in the double-stranded region consists of a base sequence homologous to a prescribed sequence which is contained in the base sequences of the target gene and which conforms to the following rules (a) to (f):

(a) The 3' end base is adenine, thymine or uracil;
(b) The 5' end base is guanine or cytosine;
(c) A 7-base sequence from the 3' end is rich in one or more types of bases selected from the group consisting of adenine, thymine and uracil;
(d) The number of bases is within a range that allows RNA interference to occur without causing cytotoxicity;
(e) A sequence in which 10 or more bases of guanine or cytosine are continuously present is not contained; and
(f) A sequence sharing at least 90% homology with the prescribed sequence is not contained in the base sequences of genes other than the target gene among all gene sequences of the target organism, and

wherein the other strand in the double-stranded region consists of a base sequence having a sequence complementary to the base sequence homologous to the prescribed sequence.

The sequence to be targeted by the polypeptide obtained by the selection method of the present invention is a sequence selected as a prescribed sequence conforming to the above rules (a) to (f). Preferably, such a sequence may be any of SEQ ID NOs: 47 to 817081.

In the selection method of the present invention, a polynucleotide having a sequence, wherein the base sequence homologous to the prescribed sequence of the target gene contains mismatches of at least 3 bases against the base sequences of genes other than the target gene, and for which there is only a minimum number of other genes having a base sequence containing the mismatches of at least 3 bases, may further be selected from the selected polynucleotides.

Namely, if the target sequence is a sequence highly specific to the target gene, the polynucleotide selectively produces an inhibitory effect only on the expression of the target gene containing the target sequence, but not on the other genes (i.e., the polynucleotide has less off-target effect), thus reducing influences of side effects, etc. It is therefore more preferred that the target sequence of the polynucleotide has high specificity to the target gene. Among the selected sequences (e.g., SEQ ID NOs: 47 to 817081), a sequence whose off-target effect can be further reduced is preferred as a prescribed sequence conforming to the above rules (a) to (f). As a preferred prescribed sequence of the target gene, it is possible to select a sequence which contains mismatches of at least 3 bases against the base sequences of other genes and for which there is a minimum number of other genes having a base sequence containing mismatches of at least 3 bases. The requirement "there is only a minimum number of other genes" means that "other genes having a base sequence containing mismatches of at least 3 bases" (i.e., similar genes) are as few in number as possible; for example, there are preferably 10 or less genes, more preferably 6 or less genes, still more preferably only one gene, or most preferably no gene.

For example, the 53998 sequences shown in Figure 46 are obtained among SEQ ID NOs: 47 to 817081 by selecting sequences which contain mismatches of 3 bases against the base sequences of other genes (i.e., prescribed sequences of 19 bases (in the narrow sense) in which 16 bases other than these 3 mismatched bases are the same as those of other genes) and for which there is only a minimum number of other genes having a base sequence containing mismatches of 3 bases. Thus, the target sequence is particularly preferably any of these sequences.

<4> Method for inhibiting gene expression

The method for inhibiting gene expression in accordance with the present invention includes a step of searching a predetermined base sequence, a step of designing and synthesizing a base sequence of a polynucleotide based on the searched base sequence, and a step of introducing the resulting polynucleotide into an expression system containing a target gene.

The step of searching a predetermined base sequence follows the method for searching a target base sequence for RNA interference described above. Preferred embodiments are the same as those described above. The step of designing and synthesizing the base sequence of siRNA based on the searched base sequence can be carried out in accordance with the method for designing the base sequence of a polynucleotide for causing RNA interference and the method for producing a polynucleotide described above. Preferred embodiments are the same as those described above.

The resulting polynucleotide is added to an expression system for a target gene to inhibit the expression of the target gene. The expression system for a target gene means a system in which the target gene is expressed, and more specifically, a system provided with a reaction system in which at least mRNA of the target gene is formed. Examples of the expression system for a target gene include both in vitro and in vivo systems. In addition to cultured cells, cultured tissues, and living bodies, cell-free systems can also be used as expression systems for target genes. The target gene whose expression is intended to be inhibited (inhibition target gene) is not necessarily a gene of a species corresponding to the origin of the searched sequence. However, as the relationship between the origin of the search target gene and the origin of the inhibition target gene becomes closer, a predetermined gene can be more specifically and effectively inhibited.

Introduction into an expression system for a target gene means incorporation into the expression reaction system for the target gene. For example, in one method, a double-stranded nucleotide is transfected to a cultured cell including a target gene and incorporated into the cell. In another method, an expression vector having a base sequence comprising a prescribed sequence and an overhanging portion is formed, and the expression vector is introduced into a cell having a target gene (WO01/36646, WO01/49844).

In accordance with the gene inhibition method of the present invention, since polynucleotides which cause RNA interference can be efficiently produced, it is possible to inhibit genes efficiently and simply. Thus, for example, in a case where the target gene is a disease-related gene, siRNA (or shRNA) targeting the disease-related gene or a vector expressing such siRNA (or shRNA) may be introduced into cells which express the disease-related gene, so that the disease-related gene can be made inactive.

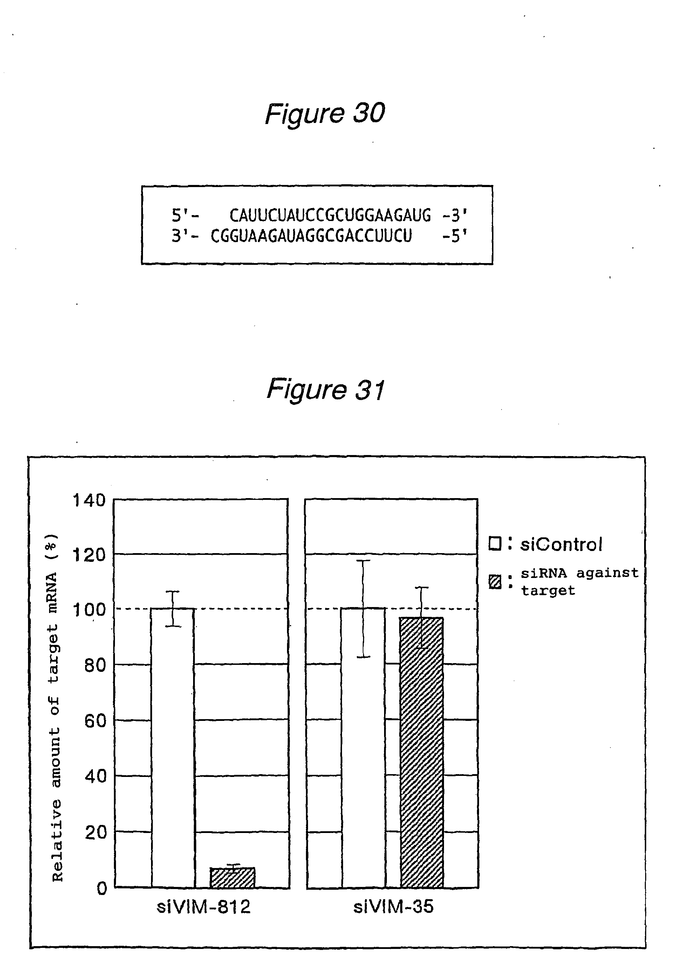

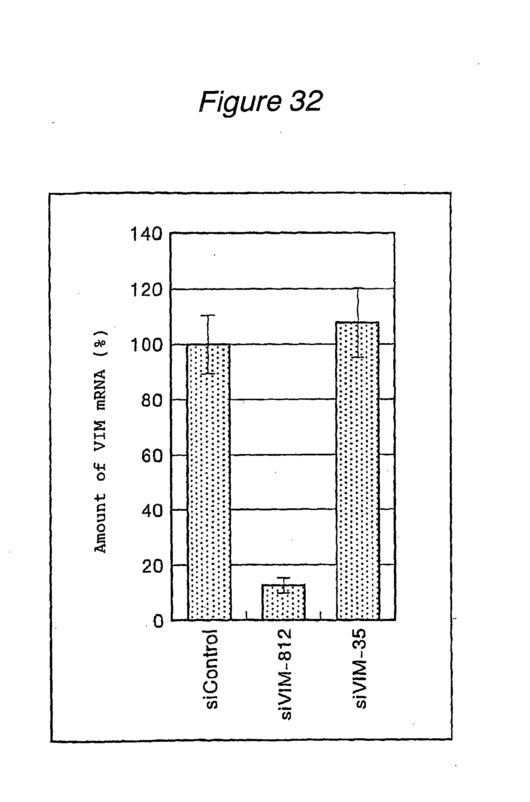

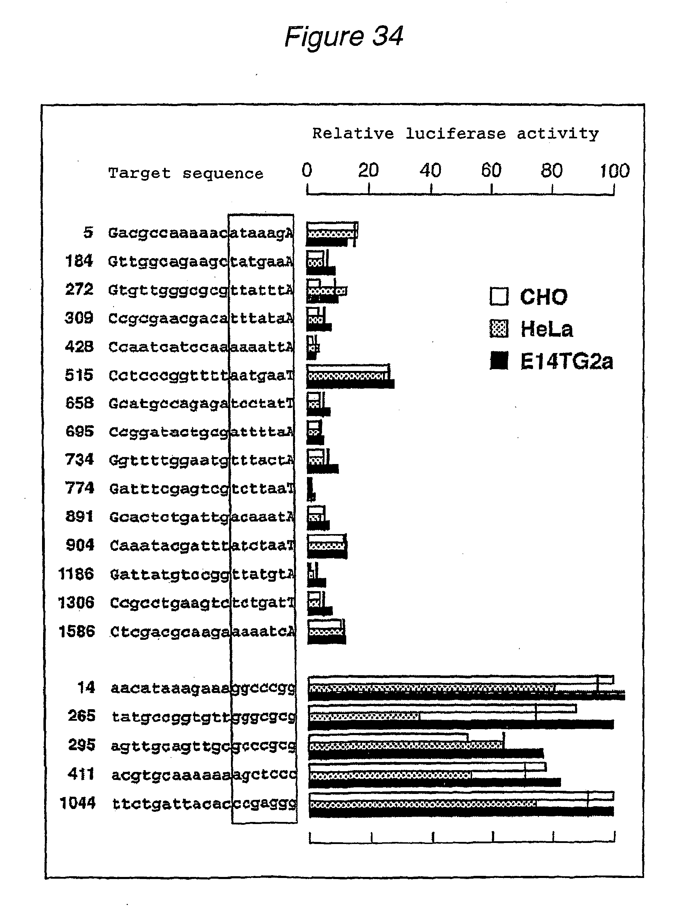

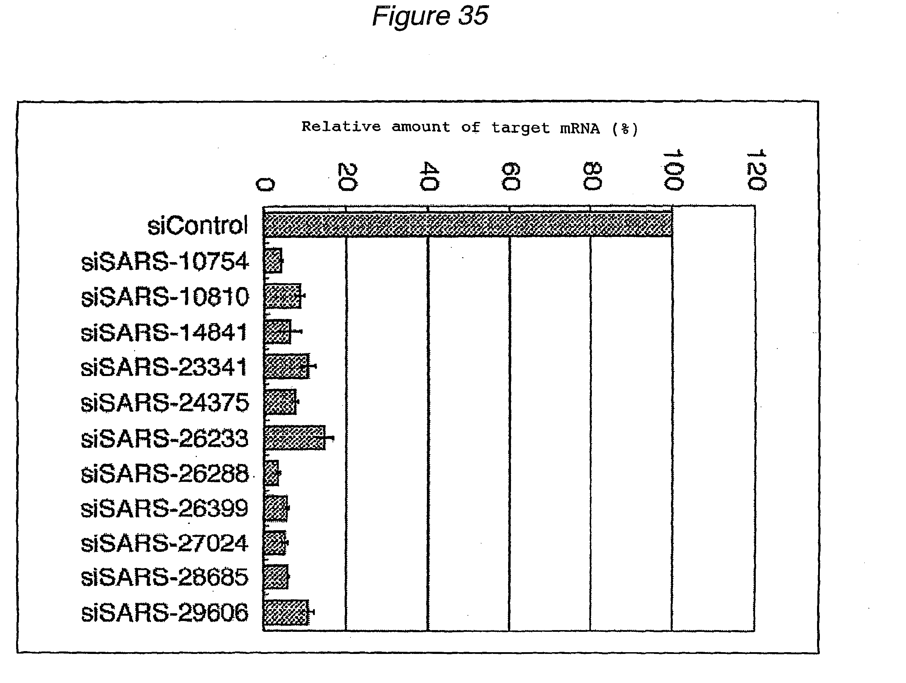

In Examples 2 to 5 described herein later, the RNAi effect of the polynucleotide of the present invention against the genes of human vimentin, luciferase, SARS virus and the like was examined as a relative expression level of mRNA compared to the control. Figures 31, 32 and 35 show the results of mRNA expression levels measured by quantitative PCR. In Figures 31, 32 and 35, the relative mRNA expression levels are respectively reduced to about 7-8% (Example 2, Figure 31), about 12-13% (Example 3, Figure 32), and a few % to less than about 15% (Example 5, Figure 35); the polynucleotide of the present invention was confirmed to have an inhibitory effect on the expression of each gene. Likewise, Figure 34 from Example 4 shows the results of mRNA expression levels (as RNAi effect) examined by luciferase activity. The luciferase activity was also reduced to a few % to less than about 20%, as compared to the control.

Moreover, in Example 8, among the genes shown in Figure 46 whose related diseases and/or biological functions have been identified, about 300 genes selected at random were examined for the expression levels of their mRNA in human-derived HeLa cells, expressed as relative expression levels. As shown in Table 1, the RQ values (described later) that were calculated to evaluate an inhibitory effect on the expression of these genes, i.e., an RNAi effect were all less than 1, and almost all less than 0.5.

In the method for inhibiting gene expression in accordance with the present invention, the phrase "inhibiting the expression of the target gene" means that the mRNA expression level of the target gene is substantially reduced. If the mRNA expression level has been substantially reduced, inhibited expression has been achieved regardless of the degree of change in the mRNA expression level. In particular, since a larger amount of reduction means a higher inhibitory effect on expression, the criterion for inhibited expression may be, without being limited to, a case where the mRNA expression level is preferably reduced to about 80% or below, more preferably reduced to about 50% or below, still more preferably reduced to about 20% or below, still even more preferably reduced to about 15% or below, and further preferably reduced to about 8% or below. In accordance with the gene inhibition method of the present invention which uses a polynucleotide selected according to the rules of the present invention, it becomes possible to preferably cause at least a 50% or more reduction in the mRNA expression level of the target gene.

<5> siRNA sequence design program

Embodiments of the siRNA sequence design program will be described below.

(5-1) Outline of the program

When species whose genomes are not sequenced, for example, horse and swine, are subjected to RNA interference, this program calculates a sequence of siRNA usable in the target species based on published sequence information regarding human beings and mice. If siRNA is designed using this program, RNA interference can be carried out rapidly without sequencing the target gene. In the design (calculation) of siRNA, sequences having RNAi activity with high probability are selected in consideration of the rules of allocation of G or C (the rules (a) to (d) described above), and checking is performed by homology search so that RNA interference does not occur in genes that are not related to the target gene. In this specification, "G or C" may also be written as "G/C", and "A or T" may also be written as "A/T". Furthermore, "T(U)" in "A/T(U)" means T (thymine) in the case of sequences of deoxyribonucleic acid and U (uracil) in the case of sequences of ribonucleic acid.

(5-2) Policy of siRNA design

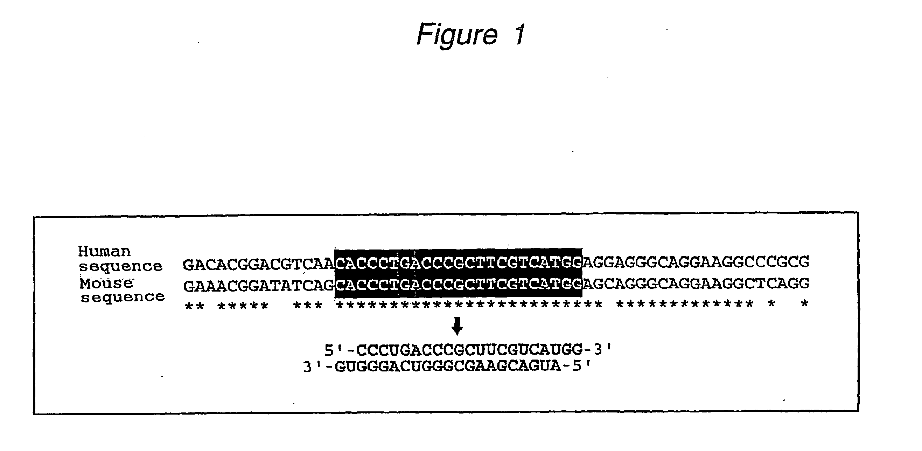

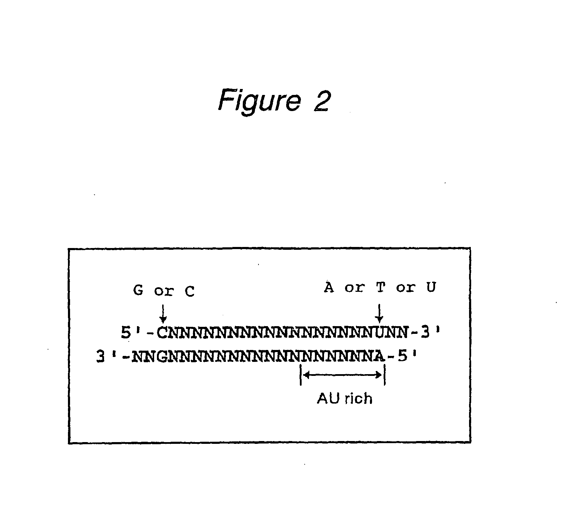

Sequences of human gene X and mouse gene X which are homologous to the human gene are assumed to be known. This program reads the sequences and searches completely common sequences each having 23 or more bases from the coding regions (CDS). By designing siRNA from the common portions, the resulting siRNA can target both human and mouse gene X (Figure 1).
Since the portions completely common to human beings and mice are believed to also exist in other mammals with high probability, the siRNA is expected to act not only on gene X of human beings and mice but also on gene X of other mammals. Namely, even if in an animal species in which the sequence of a target gene is not known, if sequence information is known regarding the corresponding homologues of human beings and mice, it is possible to design siRNA using this program.
Furthermore, in mammals, it is known that sequences of effective siRNA have regularity (Figure 2). In this program, only sequences conforming to the rules are selected. Figure 2 is a diagram which shows regularity of siRNA sequences exhibiting an RNAi effect (rules of G/C allocation of siRNA). In Figure 2, with respect to siRNA in which two RNA strands, each having a length of 21 bases and having an overhang of 2 bases on the 3' side, form base pairs between 19 bases at the 5' side of the two strands, the sequence in the coding side among the 19 bases forming the base pairs must satisfy the following conditions: 1) The 3' end is A/U; 2) the 5' end is G/C, and 3) 7 characters on the 3' side has a high ratio of A/U. In particular, the conditions 1) and 2) are important.

(5-3) Structure of program

This program consists of three parts, i.e., (5-3-1) a part which searches sequences of sites common to human beings and mice (partial sequences), (5-3-2) a part which scores the sequences according to the rules of G/C allocation, and (5-3-3) a part which performs checking by homology search so that unrelated genes are not targeted.

(5-3-1) Part which searches common sequences

This part reads a plurality of base sequence files (file 1, file 2, file 3,...) and finds all sequences of 23 characters that commonly appear in all the files.

(Calculation Example)

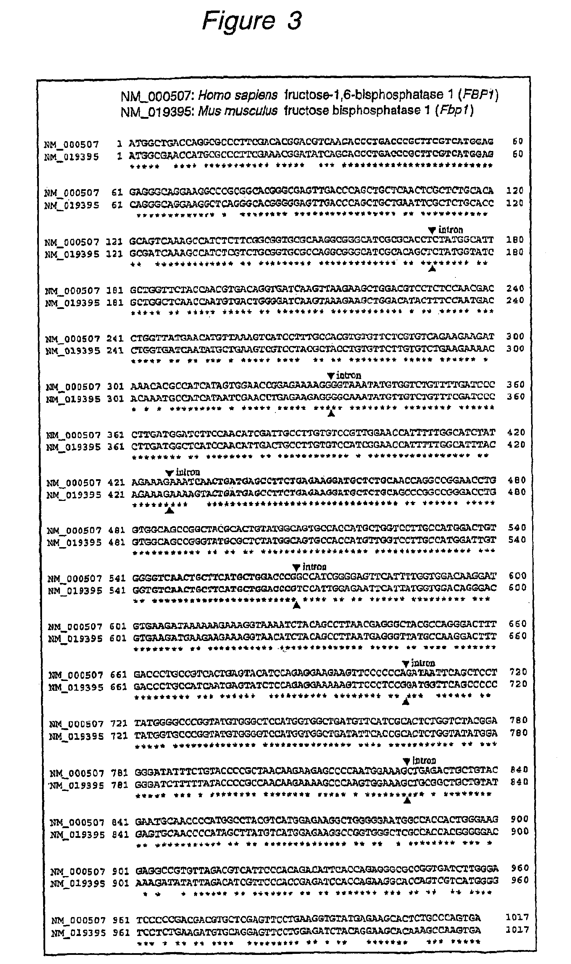

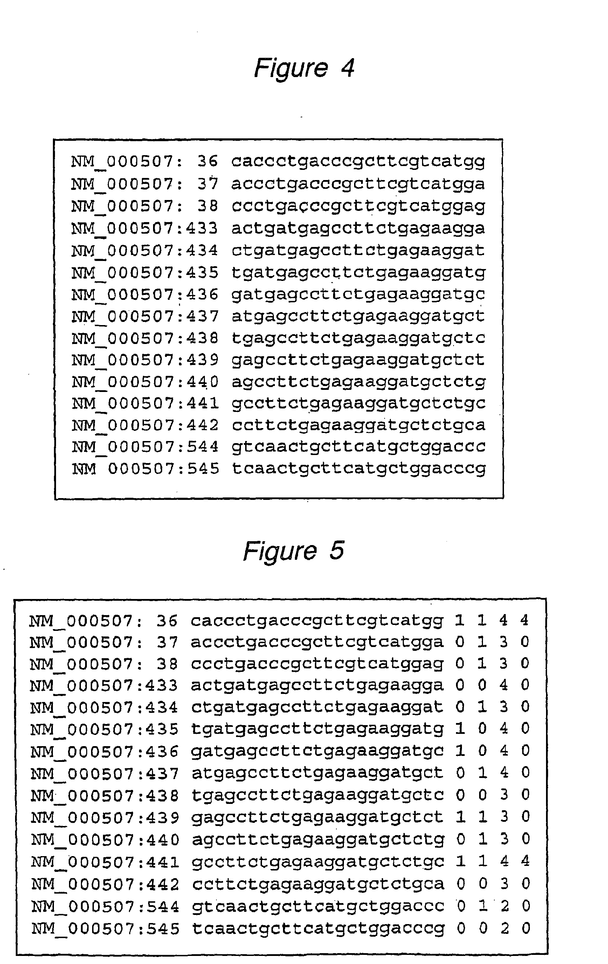

As file 1, sequences of human gene FBP1 (HM_000507: Homo sapiens fructose-1,6-bisphosphatase 1) and, as file 2, sequences of mouse gene Fbp1 (NM_019395: Mus musculus fructose bisphosphatase 1) were inputted into the program. As a result, from the sequences of the two (Figure 3), 15 sequences, each having 23 characters, that were common to the two (sequences common to human FBP1 and mouse Fbp1) were found (Figure 4).

(5-3-2) Part which scores sequences

This part scores the sequences each having 23 characters in order to only select the sequences conforming to the rules of G/C allocation.

(Method)

The sequences each having 23 characters are scored in the following manner.

Score 1: Is the 21st character from the head A/U?
[no = 0, yes = 1]
Score 2: Is the third character from the head G/C?
[no = 0, yes = 1]
Score 3: The number of A/U among 7 characters between the 15th character and 21st character from the head
[0 to 7]
Total score: Product of scores 1 to 3. However, if the product is 3 or less, the total score is considered as zero.

(Calculation Example)

With respect to 15 sequences in Figure 4, the results of calculation are shown in Figure 5. Figure 5 is a diagram in which the sequences common to human FBP1 and mouse Fbp1 are scored. Furthermore, score 1, score 2, score 3, and total score are described in this order after the sequences shown in Figure 5.

(5-3-3) Part which performs checking so that unrelated genes are not targeted

In order to prevent the designed siRNA from acting on genes unrelated to the target gene, homology search is performed against all the published mRNA of human beings and mice, and the degree of unrelated genes being hit is evaluated. Various search algorithms can be used in the homology search. Herein, an example in which BLAST is used will be described. Additionally, when BLAST is used, in view that the sequences to be searched are as short as 23 bases, it is desirable that Word Size be decreased sufficiently.
After the Blast search, among the hits with an E-value of 10.0 or less, with respect to all the hits other than the target gene, the total sum of the reciprocals of the E-values are calculated (hereinafter, the value is referred to as a homology score). Namely, the homology score (X) is found in accordance with the following expression.

$$X = \sum_{\text{all hits}} \frac{1}{E}$$

Note: A lower E value of the hit indicates higher homology to 23 characters of the query and higher risk of being targeted by siRNA. A larger number of hits indicates a higher probability that more unrelated genes are targeted. In consideration of these two respects, the risk that siRNA targets genes unrelated to the target gene is evaluated using the above expression.

(Calculation Example)

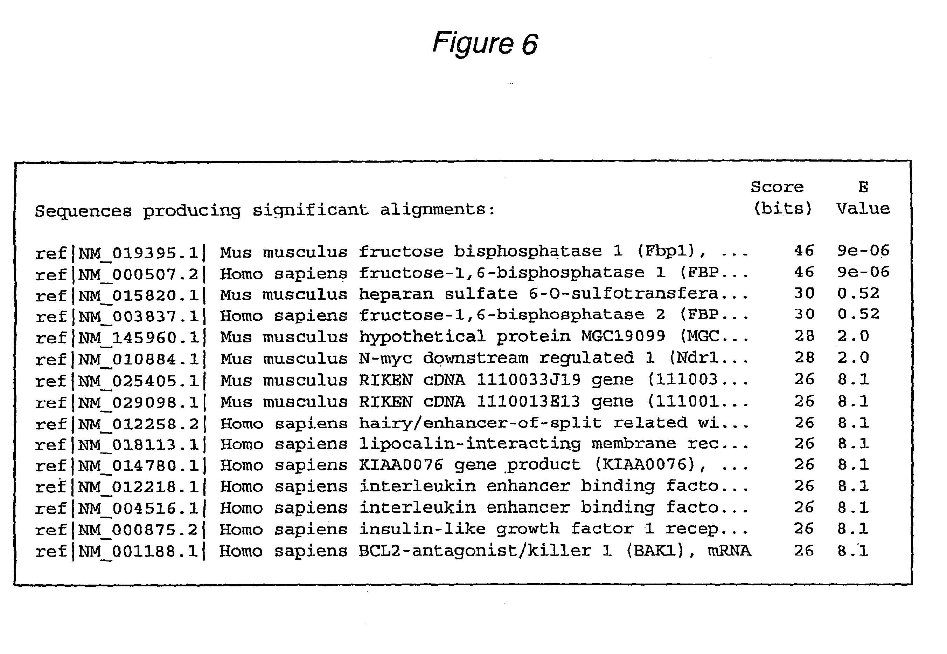

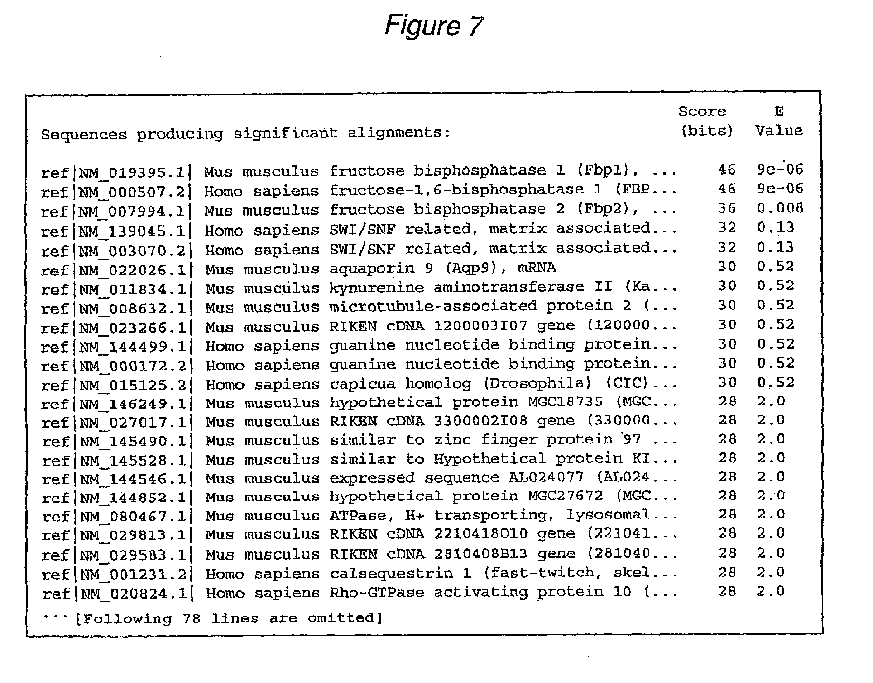

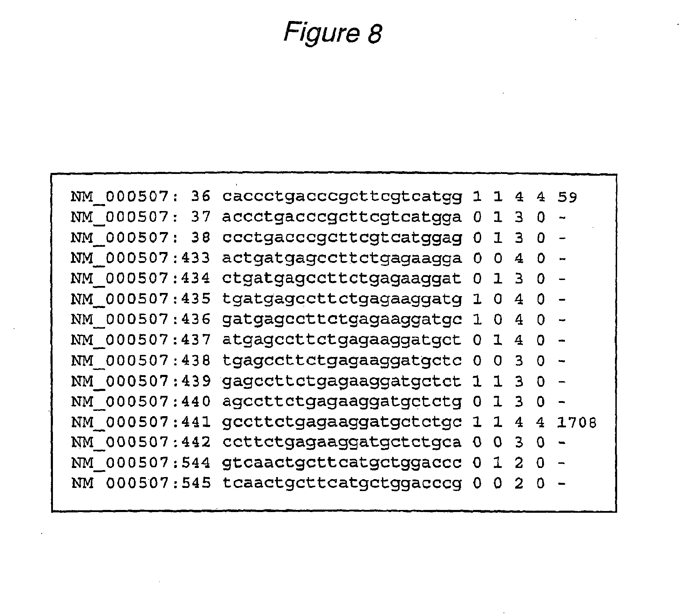

The results of homology search against the sequences each having 23 characters and the homology scores are shown (Figures 6 and 7). Figure 6 shows the results of BLAST searches of a sequence common to human FBP1 and mouse Fbp1, i.e., "caccctgacccgcttcgtcatgg", and the first two lines are the results in which both mouse Fbp1 and human FBP1 are hit. The homology score is 5.9, and this is an example of a small number of hits. The risk that siRNA of this sequence targets other genes is low. Furthermore, Figure 7 shows the results of BLAST searches of a sequence common to human FBP1 and mouse Fbp1, i.e., "gccttctgagaaggatgctctgc". This is an example of a large number of hits, and the homology score is 170.8. Since the risk of targeting other genes is high, the sequence is not suitable as siRNA.
In practice, the parts (5-3-1), (5-3-2) and (5-3-3) may be integrated, and when the sequences of human beings and mice shown in Figure 3 are inputted, an output as shown in Figure 8 is directly obtained. Herein, after the sequences shown in Figure 8, score 1, score 2, score 3, total score, and the tenfold value of homology score are described in this order. Additionally, in order to save processing time, the program may be designed so that the homology score is not calculated when the total score is zero. As a result, it is evident that the segment "36 caccctgacccgcttcgtcatgg" can be used as siRNA. Furthermore, one of the parts (5-3-1), (5-3-2) and (5-3-3) may be used independently.

(5-4) Actual calculation

With respect to about 6,400 gene pairs among the homologues between human beings and mice, siRNA was actually designed using this program. As a result, regarding about 70% thereof, it was possible to design siRNA which had a sequence common to human beings and mice and which satisfied the rules of effective siRNA sequence regularity so that unrelated genes were not targeted.

These siRNA sequences are expected to effectively inhibit target genes not only in human beings and mice but also in a wide range of mammals, and are believed to have a high industrial value, such as applications to livestock and pet animals. Moreover, it is possible to design siRNA which simultaneously targets two or more genes of the same species, e.g., eIF2C1 and eIF2C2, using this program. Thus, the method for designing siRNA provided by this program has a wide range of application and is extremely strong. In further application, by designing a PCR primer using a sequence segment common to human beings and mice, target genes can be amplified in a wide range of mammals.

Additionally, embodiments of the apparatus which runs the siRNA sequence design program will be described in detail below in the column <7> Base sequence processing apparatus for running siRNA sequence design program.

<6> siRNA sequence design business model system

In the siRNA sequence design business model system of the present invention, when the siRNA sequence design program is applied, the system refers to a genome database, an EST database, and a phylogenetic tree database, alone or in combination, according to the logic of this program, and effective siRNA in response to availability of gene sequence information is proposed to the client. The term "availability" means a state in which information is available.

(1) In a case in which it is difficult to specify an ORF although genome information is available, siRNA candidates effective against assumed exon sites are extracted based on EST information, etc., and siRNA sequences in consideration of splicing variants and evaluation results thereof are displayed.

(2) In a case in which a gene sequence and a gene name are known, after the input of the gene sequence or the gene name, effective siRNA candidates are extracted, and siRNA sequences and evaluation results thereof are displayed.

(3) In a case in which genome information is not available, using the gene sequences of a related species storing the same type of gene functions (congeneric or having the same origin) or gene sequences of two or more species which have a short distance in phylogenetic trees and of which genome sequences are available, effective siRNA candidates are extracted, and siRNA sequences and evaluation results thereof are displayed.

(4) In order to analyze functions of genes relating infectious diseases and search for targets for new drug development, a technique is effective in which the genome database and phylogenetic tree database of microorganisms are further combined with apoptosis induction site information and function expression site information of microorganisms to obtain exhaustive siRNA candidate sequences.

<7> Base sequence processing apparatus for running siRNA sequence design program, etc.

Embodiments of the base sequence processing apparatus which is an apparatus for running the siRNA sequence design program described above, the program for running a base sequence processing method on a computer, the recording medium, and the base sequence processing system in accordance with the present invention will be described in detail below with reference to the drawings. However, it is to be understood that the present invention is not restricted by the embodiments.

[Summary of the present invention]

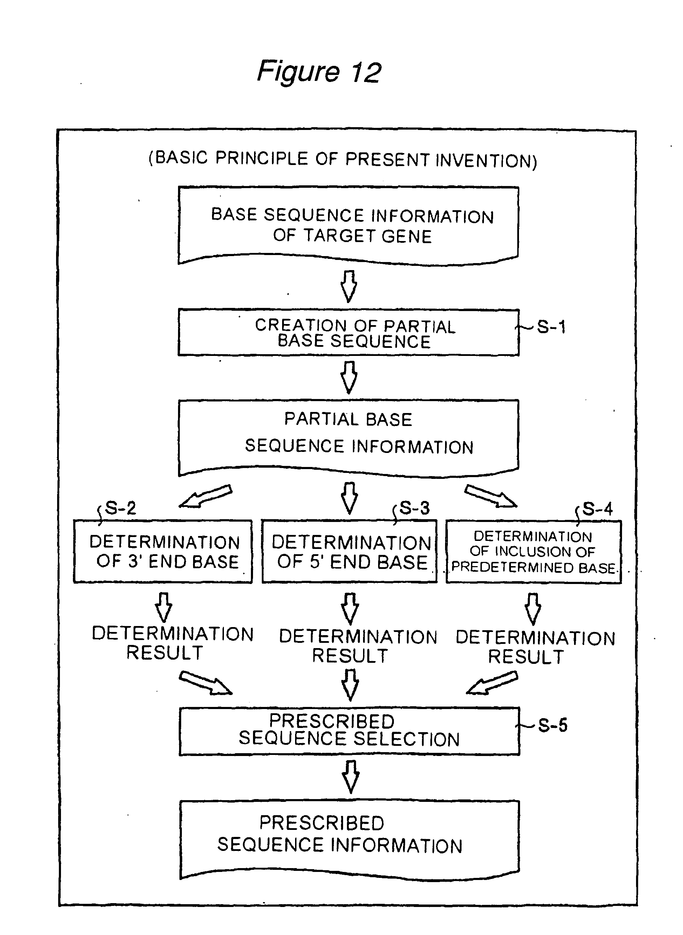

The summary of the present invention will be described below, and then the constitution, processing, etc., of the present invention will be described in detail. Figure 12 is a principle diagram showing the basic principle of the present invention.

Overall, the present invention has the following basic features. That is, in the present invention, base sequence information of a target gene for RNA interference is obtained, and partial base sequence information corresponding to a sequence segment having a predetermined number of bases in the base sequence information is created (step S-1).

In step S-1, partial base sequence information having a predetermined number of bases may be created from a segment corresponding to a coding region or transcription region of the target gene in the base sequence information. Furthermore, partial base sequence information having a predetermined number of bases which is common in a plurality of base sequence information derived from different organisms (e.g., human base sequence information and mouse base sequence information) may be created. Furthermore, partial base sequence information having a predetermined number of bases which is common in a plurality of analogous base sequence information in the same species may be created. Furthermore, common partial base sequence information having a predetermined number of bases may be created from segments corresponding to coding regions or transcription regions of the target gene in a plurality of base sequence information derived from different species. Furthermore, common partial base sequence information having a predetermined number of bases may be created from segments corresponding to coding regions or transcription regions of the target gene in a plurality of analogous base sequence information in the same species. Consequently, a prescribed sequence which specifically causes RNA interference in the target gene can be efficiently selected, and calculation load can be reduced.

Furthermore, in step S-1, partial base sequence information including an overhanging portion may be created. Specifically, for example, partial base sequence information to which overhanging portion inclusion information, which shows that an overhanging portion is included, is added may be created. Namely, partial base sequence information and overhanging portion inclusion information may be correlated with each other. Thereby, it becomes possible to select the prescribed sequence with the overhanging portion being included from the start to perform designing.

The upper limit of the predetermined number of bases is, in the case of not including the overhanging portion, preferably 28 or less, more preferably 22 or less, and still more preferably 20 or less, and in the case of including the overhanging portion, preferably 32 or less, more preferably 26 or less, and still more preferably 24 or less. The lower limit of the predetermined number of bases is, in the case of not including the overhanging portion, preferably at least 13, more preferably at least 16, and still more preferably at least 18, and in the case of including the overhanging portion, preferably at least 17, more preferably at least 20, and still more preferably at least 22. Most preferably, the predetermined number of bases is, in the case of not including the overhanging portion, 19, and in the case of including the overhanging portion, 23. Thereby, it is possible to efficiently select the prescribed sequence which causes RNA interference without causing cytotoxicity even in mammals.

Subsequently, it is determined whether the 3' end base in the partial base sequence information created in step S-1 is adenine, thymine, or uracil (step S-2). Specifically, for example, when the 3' end base is adenine, thymine, or uracil, "1" may be outputted as the determination result, and when it is not, "0" may be outputted.

Subsequently, it is determined whether the 5' end base in the partial base sequence information created in step S-1 is guanine or cytosine (step S-3). Specifically, for example, when the 5' end base is guanine or cytosine, "1" may be outputted as the determination result, and when it is not, "0" may be outputted.

Subsequently, it is determined whether base sequence information comprising 7 bases at the 3' end in the partial base sequence information created in step S-1 is rich in one or more types of bases selected from the group consisting of adenine, thymine, and uracil (step S-4). Specifically, for example, the number of bases of one or more types of bases selected from the group consisting of adenine, thymine, and uracil contained in the base sequence information comprising 7 bases at the 3' end in the partial base sequence information may be outputted as the determination result. The rule of determination in step S-4 regulates that base sequence information in the vicinity of the 3' end of the partial base sequence information created in step S-1 contains a rich amount of one or more types of bases selected from the group consisting of adenine, thymine, and uracil, and more specifically, as an index for search, regulates that the base sequence information in the range from the 3' end base to the seventh base from the 3' end is rich in one or more types of bases selected from the group consisting of adenine, thymine, and uracil.

In step S-4, the phrase "base sequence information rich in" corresponds to the phrase "sequence rich in" described in the column <1> Method for searching target base sequence for RNA interference. Specifically, for example, when the partial base sequence information created in step S-1 comprises about 19 bases, in the base sequence information comprising 7 bases in the partial base sequence information, preferably at least 3 bases, more preferably at least 4 bases, and particularly preferably at least 5 bases, are one or more types of bases selected from the group consisting of adenine, thymine, and uracil.

Furthermore, in steps S-2 to S-4, when partial base sequence information including the overhanging portion is determined, the sequence segment excluding the overhanging portion in the partial base sequence information is considered as the determination target.

Subsequently, based on the determination results in steps S-2, S-3, and S-4, prescribed sequence information which specifically causes RNA interference in the target gene is selected from the partial base sequence information created in step S-1 (Step S-5).

Specifically, for example, partial base sequence information in which the 3' end base has been determined as adenine, thymine, or uracil in step S-2, the 5' end base has been determined as guanine or cytosine in step S-3, and base sequence information comprising 7 bases at the 3' end in the partial base sequence information has been determined as being rich in one or more types of bases selected from the group consisting of adenine, thymine, and uracil is selected as prescribed sequence information. Specifically, for example, a product of the values outputted in steps S-2, S-3, and S-4 may be calculated, and based on the product, prescribed sequence information may be selected from the partial base sequence information created in step S-1.

Consequently, it is possible to efficiently and easily produce a siRNA sequence which has an extremely high probability of causing RNA interference, i.e., which is effective for RNA interference, in mammals, etc.

Here, an overhanging portion may be added to at least one end of the prescribed sequence information selected in step S-5. Additionally, for example, when a target is searched, the overhanging portion may be added to both ends of the prescribed sequence information. Consequently, designing of a polynucleotide which causes RNA interference can be simplified.

Additionally, the number of bases in the overhanging portion corresponds to the number of bases described in the column <2> Method for designing base sequence of polynucleotide for causing RNA interference. Specifically, for example, 2 is particularly suitable as the number of bases.

Furthermore, base sequence information that is identical or similar to the prescribed sequence information selected in step S-5 may be searched from other base sequence information (e.g., base sequence information published in a public database, such as RefSeq (Reference Sequence project) of NCBI) using a known homology search method, such as BLAST, FASTA, or ssearch, and based on the searched identical or similar base sequence information, evaluation may be made whether the prescribed sequence information targets genes unrelated to the target gene.

Specifically, for example, base sequence information that is identical or similar to the prescribed sequence information selected in step S-5 is searched from other base sequence information (e.g., base sequence information published in a public database, such as RefSeq of NCBI) using a known homology search method, such as BLAST, FASTA, or ssearch. Based on the total amount of base sequence information on the genes unrelated to the target gene in the searched identical or similar base sequence information and the values showing the degree of identity or similarity (e.g., "E value" in BLAST, FASTA, or ssearch) attached to the base sequence information on the genes unrelated to the target gene, the total sum of the reciprocals of the values showing the degree of identity or similarity is calculated, and based on the calculated total sum (e.g., based on the size of the total sum calculated), evaluation may be made whether the prescribed sequence information targets genes unrelated to the target gene.

Consequently, it is possible to select a sequence which specifically causes RNA interference only to the target gene.

If RNA is synthesized based on the prescribed sequence information which is selected in accordance with the present invention and which does not cause RNA interference in genes unrelated to the target gene, it is possible to greatly reduce effort, time, and cost required compared with conventional techniques.

[System configuration]

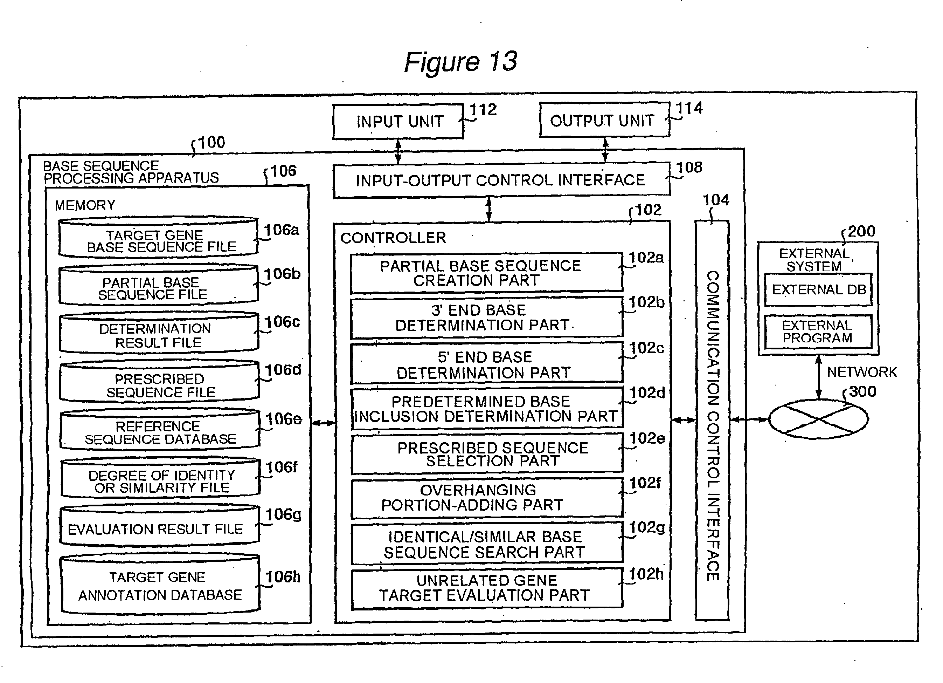

First, the configuration of this system will be described. Figure 13 is a block diagram which shows an example of the system to which the present invention is applied and which conceptually shows only the parts related to the present invention.

Schematically, in this system, a base sequence processing apparatus 100 which processes base sequence information of a target gene for RNA interference and an external system 200 which provides external databases regarding sequence information, structural information, etc., and external programs, such as homology search, are connected to each other via a network 300 in a communicable manner.

In Figure 13, the network 300 has a function of interconnecting between the base sequence processing apparatus 100 and the external system 200, and is, for example, the Internet.

In Figure 13, the external system 200 is connected to the base sequence processing apparatus 100 via the network 300, and has a function of providing the user with the external databases regarding sequence information, structural information, etc., and Web sites which execute external programs, such as homology search and motif search.

The external system 200 may be constructed as a WEB server, ASP server, or the like, and the hardware structure thereof may include a commercially available information processing apparatus, such as a workstation or a personal computer, and its accessories. Individual functions of the external system 200 are implemented by a CPU, a disk drive, a memory unit, an input unit, an output unit, a communication control unit, etc., and programs for controlling them in the hardware structure of the external system 200.

In Figure 13, the base sequence processing apparatus 100 schematically includes a controller 102, such as a CPU, which controls the base sequence processing apparatus 100 overall; a communication control interface 104 which is connected to a communication device (not shown in the drawing), such as a router, connected to a communication line or the like; an input-output control interface 108 connected to an input unit 112 and an output unit 114; and a memory 106 which stores various databases and tables. These parts are connected via given communication channels in a communicable manner. Furthermore, the base sequence processing apparatus 100 is connected to the network 300 in a communicable manner via a communication device, such as a router, and a wired or radio communication line.

Various databases and tables (a target gene base sequence file 106a ~ a target gene annotation database 106h) which are stored in the memory 106 are storage means, such as fixed disk drives, for storing various programs used for various processes, tables, files, databases, files for web pages, etc.

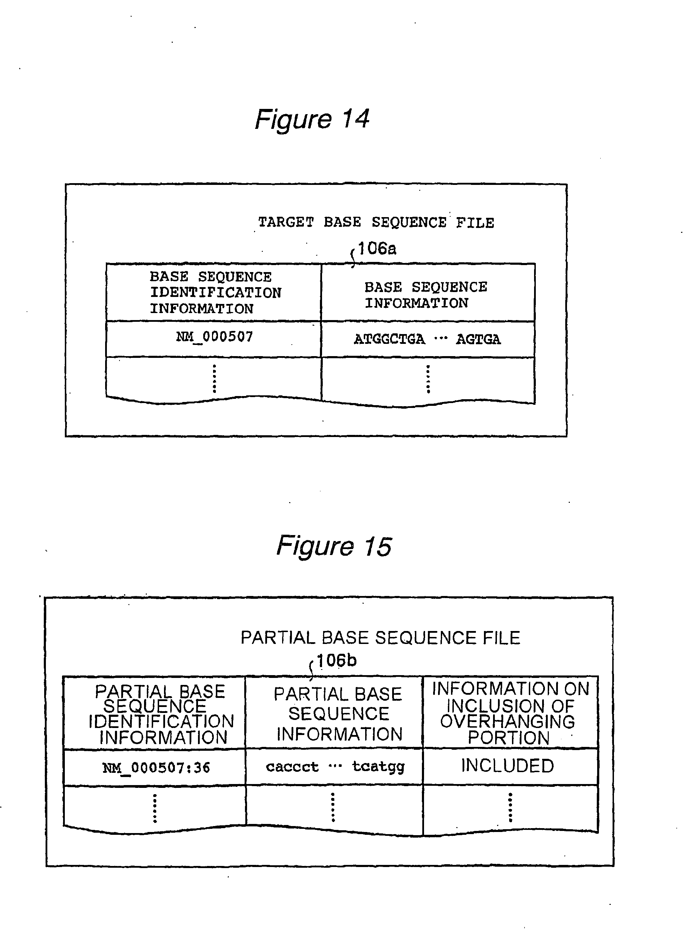

Among these components of the memory 106, the target gene base sequence file 106a is target gene base sequence storage means for storing base sequence information of the target gene for RNA interference. Figure 14 is a diagram which shows an example of information stored in the target gene base sequence file 106a.

As shown in Figure 14, the information stored in the target gene base sequence file 106a consists of base sequence identification information which uniquely identifies base sequence information of the target gene for RNA interference (e.g., "NM_000507" in Figure 14) and base sequence information (e.g., "ATGGCTGA... AGTGA" in Figure 14), the base sequence identification information and the base sequence information being associated with each other.

Furthermore, a partial base sequence file 106b is partial base sequence storage means for storing partial base sequence information, i.e., a sequence segment having a predetermined number of bases in base sequence information of the target gene for RNA interference. Figure 15 is a diagram which shows an example of information stored in the partial base sequence file 106b.

As shown in Figure 15, the information stored in the partial base sequence file 106b consists of partial base sequence identification information which uniquely identifies partial base sequence information (e.g., "NM_000507:36" in Figure 15), partial base sequence information (e.g., "caccct... tcatgg" in Figure 15), and information on inclusion of an overhanging portion which shows the inclusion of the overhanging portion (e.g., "included" in Figure 15), the partial base sequence identification information, the partial base sequence information, and the information on inclusion of the overhanging portion being associated with each other.

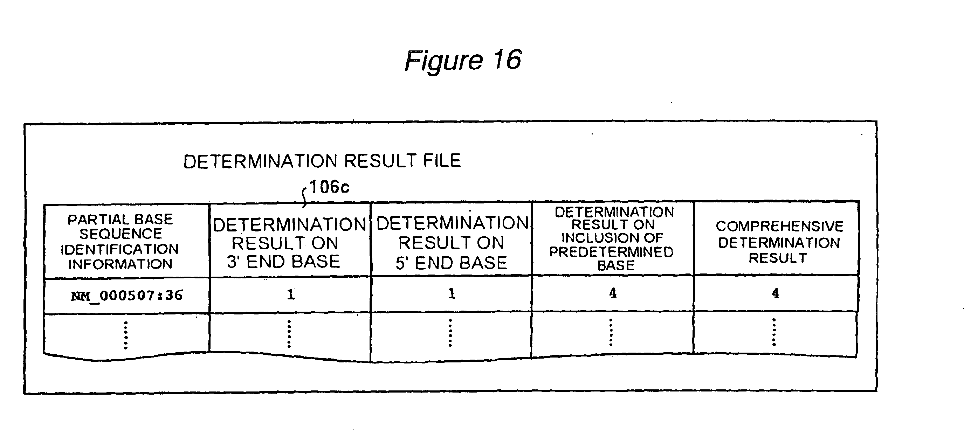

A determination result file 106c is determination result storage means for storing the results determined by a 3' end base determination part 102b, a 5' end base determination part 102c, and a predetermined base inclusion determination part 102d, which will be described below. Figure 16 is a diagram which shows an example of information stored in the determination result file 106c.

As shown in Figure 16, the information stored in the determination result file 106c consists of partial base sequence identification information (e.g., "NM_000507:36" in Figure 16), determination result on 3' end base corresponding to a result determined by the 3' end base determination part 102b (e.g., "1" in Figure 16), determination result on 5' end base corresponding to a result determined by the 5' end base determination part 102c (e.g., "1" in Figure 16), determination result on inclusion of predetermined base corresponding to a result determined by the predetermined base inclusion determination part 102d (e.g., "4" in Figure 16), and comprehensive determination result corresponding to a result obtained by putting together the results determined by the 3' end base determination part 102b, the 5' end base determination part 102c, and the predetermined base inclusion determination part 102d (e.g., "4" in Figure 16), the partial base sequence identification information, the determination result on 3' end base, the determination result on 5' end base, the determination result on inclusion of predetermined base, and the comprehensive determination result being associated with each other.

Additionally, Figure 16 shows an example of the case in which, with respect to the determination result on 3' end base and the determination result on 5' end base, "1" is set when determined as being "included" by each of the 3' end base determination part 102b and the 5' end base determination part 102c and "0" is set when determined as being "not included". Furthermore, Figure 16 shows an example of the case in which the determination result on inclusion of predetermined base is set as the number of bases corresponding to one or more types of bases selected from the group consisting of adenine, thymine, and uracil contained in the base sequence information comprising 7 bases at the 3' end in the partial base sequence information. Furthermore, Figure 16 shows an example of the case in which the comprehensive determination result is set as the product of the determination result on 3' end base, the determination result on 5' end base, and the determination result on inclusion of predetermined base. Specifically, for example, when the product is 3 or less, "0" may be set.

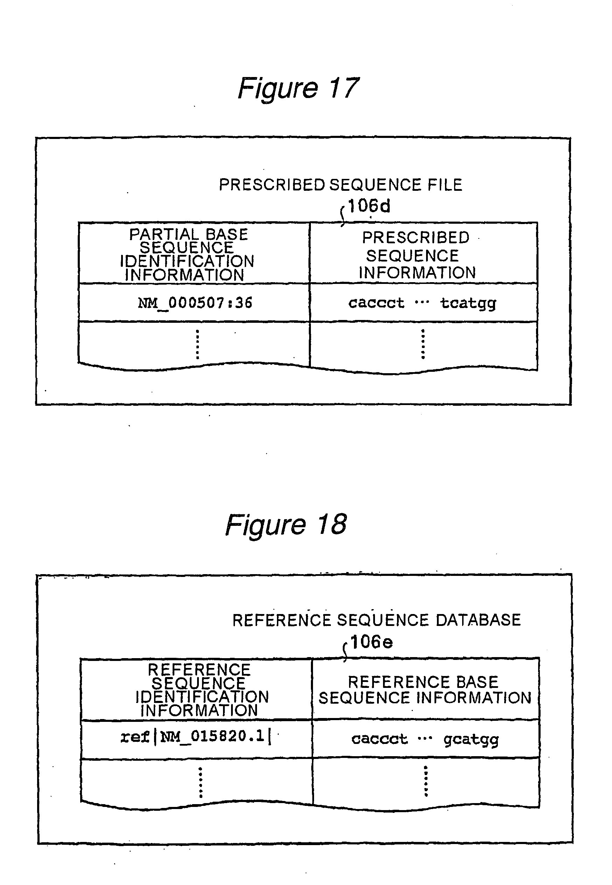

Furthermore, a prescribed sequence file 106d is prescribed sequence storage means for storing prescribed sequence information corresponding to partial base sequence information which specifically causes RNA interference in the target gene. Figure 17 is a diagram which shows an example of information stored in the prescribed sequence file 106d.

As shown in Figure 17, the information stored in the prescribed sequence file 106d consists of partial base sequence identification information (e.g., "NM_000507:36" in Figure 17) and prescribed sequence information corresponding to partial base sequence information which specifically causes RNA interference in the target gene (e.g., caccct... tcatgg" in Figure 17), the partial base sequence identification information and the prescribed sequence information being associated with each other.

Furthermore, a reference sequence database 106e is a database which stores reference base sequence information corresponding to base sequence information to which reference is made to search base sequence information identical or similar to the prescribed sequence information by an identical/similar base sequence search part 102g, which will be described below. The reference sequence database 106e may be an external base sequence information database accessed via the Internet or may be an in-house database created by copying such a database, storing the original sequence information, or further adding unique annotation information to such a database. Figure 18 is a diagram which shows an example of information stored in the reference sequence database 106e.

As shown in Figure 18, the information stored in the reference sequence database 106e consists of reference sequence identification information (e.g., "ref|NM_015820.1|" in Figure 18) and reference base sequence information (e.g., "caccct... gcatgg" in Figure 18), the reference sequence identification information and the reference base sequence information being associated with each other.

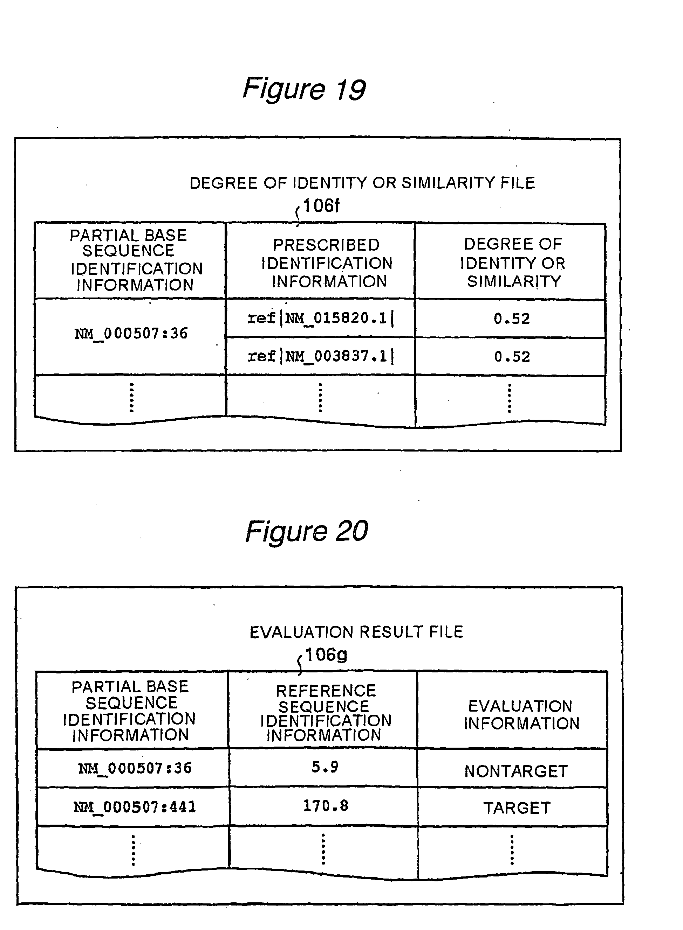

Furthermore, a degree of identity or similarity file 106f is degree of identity or similarity storage means for storing the degree of identity or similarity corresponding to a degree of identity or similarity of identical or similar base sequence information searched by an identical/similar base sequence search part 102g, which will be described below. Figure 19 is a diagram which shows an example of information stored in the degree of identity or similarity file 106f.

As shown in Figure 19, the information stored in the degree of identity or similarity file 106f consists of partial base sequence identification information (e.g., "NM_000507:36" in Figure 19), reference sequence identification information (e.g., "ref|NM_015820.1|" and "ref|NM_003837.1|" in Figure 19), and degree of identity or similarity (e.g., "0.52" in Figure 19), the partial base sequence identification information, the reference sequence identification information, and the degree of identity or similarity being associated with each other.

Furthermore, an evaluation result file 106g is evaluation result storage means for storing the result of evaluation on whether genes unrelated to the target gene are targeted by an unrelated gene target evaluation part 102h, which will be described below. Figure 20 is a diagram which shows an example of information stored in the evaluation result file 106g.

As shown in Figure 20, the information stored in the evaluation result file 106g consists of partial base sequence identification information (e.g., "NM_000507:36" and "NM_000507:441" in Figure 20), total sum calculated by a total sum calculation part 102m, which will be described below, (e.g., "5.9" and "170.8" in Figure 20), and evaluation result (e.g., "nontarget" and "target" in Figure 20), the partial base sequence identification information, the total sum, and the evaluation result being associated with each other. Additionally, in Figure 20, "nontarget" means that the prescribed sequence information does not target genes unrelated to the target gene, and "target" means that the prescribed sequence information targets genes unrelated to the target gene.

A target gene annotation database 106h is target gene annotation storage means for storing annotation information regarding the target gene. The target gene annotation database 106h may be an external annotation database which stores annotation information regarding genes and which is accessed via the Internet or may be an in-house database created by copying such a database, storing the original sequence information, or further adding unique annotation information to such a database.

The information stored in the target gene annotation database 106h consists of target gene identification information which identifies the target gene (e.g., the name of a gene to be targeted, and Accession number (e.g., "NM_000507" and "FBP1" described on the top in Figure 3)) and simplified information on the target gene (e.g., "Homo sapiens fructose-1,6-bisphosphatase 1" describe on the top in Figure 3), the target gene identification information and the simplified information being associated with each other.

In Figure 13, the communication control interface 104 controls communication between the base sequence processing apparatus 100 and the network 300 (or a communication device, such as a router). Namely, the communication control interface 104 performs data communication with other terminals via communication lines.

In Figure 13, the input-output control interface 108 controls the input unit 112 and the output unit 114. Here, as the output unit 114, in addition to a monitor (including a home television), a speaker may be used (hereinafter, the output unit 114 may also be described as a monitor). As the input unit 112, a keyboard, a mouse, a microphone, or the like may be used. The monitor cooperates with a mouse to implement a pointing device function.

In Figure 13, the controller 102 includes control programs, such as OS (Operating System), programs regulating various processing procedures, etc., and internal memories for storing required data, and performs information processing for implementing various processes using the programs, etc. The controller 102 functionally includes a partial base sequence creation part 102a, a 3' end base determination part 102b, a 5' end base determination part 102c, a predetermined base inclusion determination part 102d, a prescribed sequence selection part 102e, an overhanging portion-adding part 102f, an identical/similar base sequence search part 102g, and an unrelated gene target evaluation part 102h.

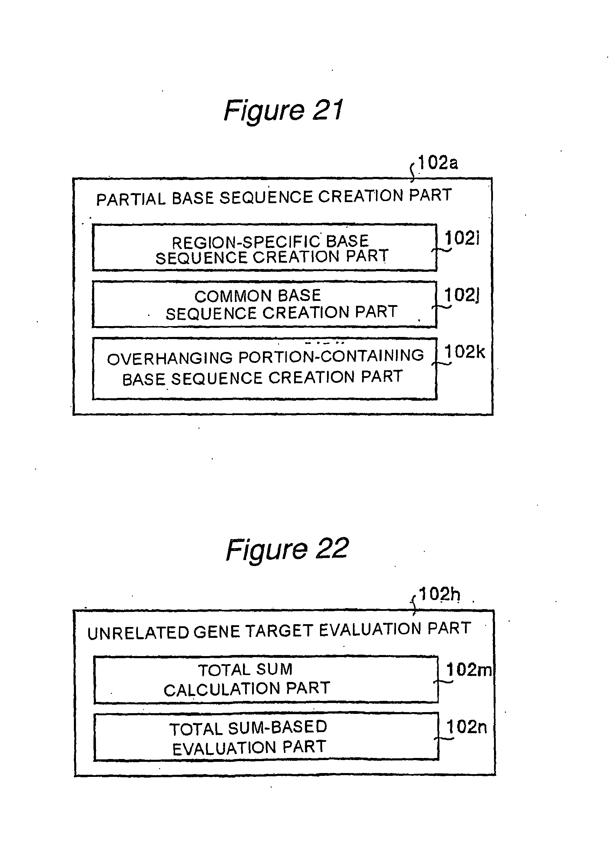

Among them, the partial base sequence creation part 102a is partial base sequence creation means for acquiring base sequence information of a target gene for RNA interference and creating partial base sequence information corresponding to a sequence segment having a predetermined number of bases in the base sequence information. As shown in Figure 21, the partial base sequence creation part 102a includes a region-specific base sequence creation part 102i, a common base sequence creation part 102j, and an overhanging portion-containing base sequence creation part 102k.

Figure 21 is a block diagram which shows an example of the structure of the partial base sequence creation part 102a of the system to which the present invention is applied and which shows only the parts related to the present invention.

In Figure 21, the region-specific base sequence creation part 102i is region-specific base sequence creation means for creating partial base sequence information having a predetermined number of bases from a segment corresponding to a coding region or transcription region of the target gene in the base sequence information.

The common base sequence creation part 102j is common base sequence creation means for creating partial base sequence information having a predetermined number of bases which is common in a plurality of base sequence information derived from different organisms.

The overhanging portion-containing base sequence creation part 102k is overhanging portion-containing base sequence creation means for creating partial base sequence information containing an overhanging portion.

Referring back to Figure 13, the 3' end base determination part 102b is 3' end base determination means for determining whether the 3' end base in the partial base sequence information is adenine, thymine, or uracil.

Furthermore, the 5' end base determination part 102c is 5' end base determination means for determining whether the 5' end base in the partial base sequence information is guanine or cytosine.

Furthermore, the predetermined base inclusion determination part 102d is predetermined base inclusion determination means for determining whether the base sequence information comprising 7 bases at the 3' end in the partial base sequence information is rich in one or more types of bases selected from the group consisting of adenine, thymine, and uracil.

Furthermore, the prescribed sequence selection part 102e is prescribed sequence selection means for selecting prescribed sequence information, which specifically causes RNA interference in the target gene, from the partial base sequence information based on the results determined by the 3' end base determination part 102b, the 5' end base determination part 102c, and the predetermined base inclusion determination part 102c.

Furthermore, the overhanging portion-adding part 102f is overhanging portion addition means for adding an overhanging portion to at least one end of the prescribed sequence information.

Furthermore, the identical/similar base sequence search part 102g is identical/similar base sequence search means for searching base sequence information, identical or similar to the prescribed sequence information, from other base sequence information.

Furthermore, the unrelated gene target evaluation part 102h is unrelated gene target evaluation means for evaluating whether the prescribed sequence information targets genes unrelated to the target gene based on the identical or similar base sequence information. As shown in Figure 22, the unrelated gene target evaluation part 102h further includes a total sum calculation part 102m and a total sum-based evaluation part 102n.

Figure 22 is a block diagram which shows an example of the structure of the unrelated gene target evaluation part 102h of the system to which the present invention is applied and which schematically shows only the parts related to the present invention.

In Figure 22, the total sum calculation part 102m is total sum calculation means for calculating the total sum of reciprocals of the values showing the degree of identity or similarity based on the total amount of base sequence information on the genes unrelated to the target gene in identical or similar base sequence information and the values showing the degree of identity or similarity attached to the base sequence information on the genes unrelated to the target gene (identity or similarity).

Furthermore, the total sum-based evaluation part 102n is total sum-based target evaluation means for evaluating whether the prescribed sequence information targets genes unrelated to the target gene based on the total sum calculated by the total sum calculation part 102m.

The details of processing of each part will be described later.

[Processing of the system]

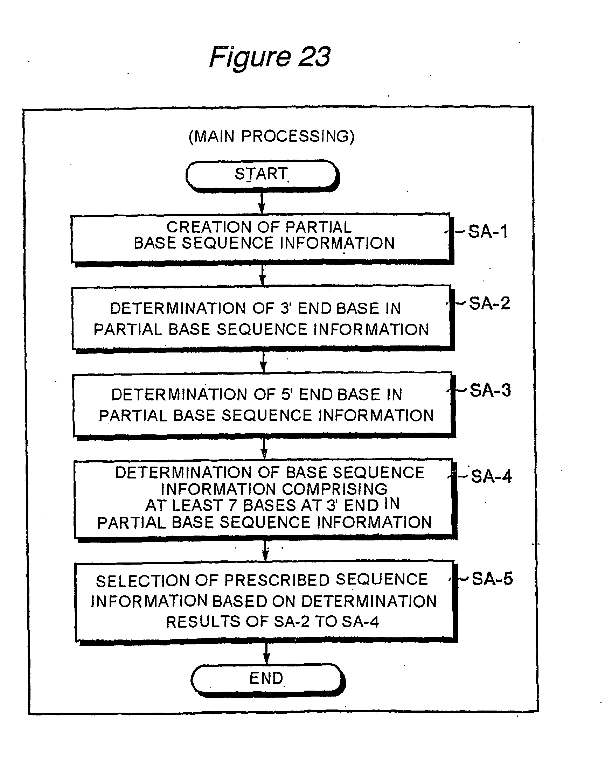

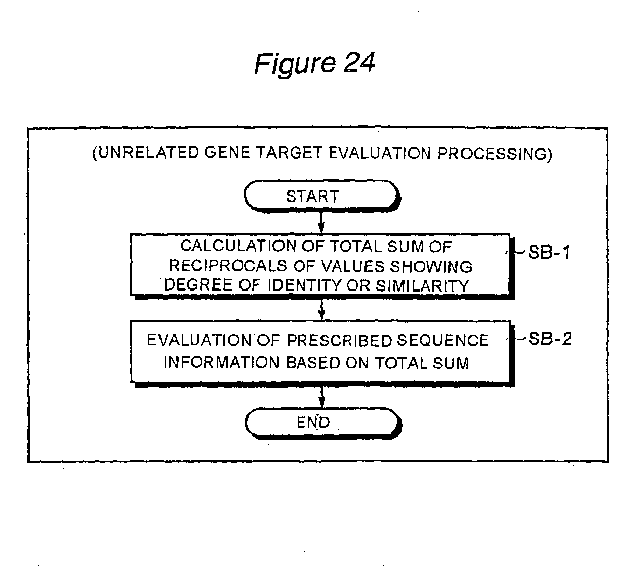

An example of processing of the system having the configuration described above in this embodiment will be described in detail with reference to Figures 23 and 24.

[Main processing]

First, the details of the main processing will be described with reference to Figure 23, etc. Figure 23 is a flowchart which shows an example of the main processing of the system in this embodiment.

The base sequence processing apparatus 100 acquires base sequence information of a target gene for RNA interference by the partial base sequence creation process performed by the partial base sequence creation part 102a, stores it in a predetermined memory region of the target gene base sequence file 106a, creates partial base sequence information corresponding to a sequence segment having a predetermined number of bases in the base sequence information, and stores the created partial base sequence information in a predetermined memory region of the partial base sequence file 106b (step SA-1).

In step SA-1, the partial base sequence creation part 102a may create partial base sequence information having a predetermined number of bases from a segment corresponding to a coding region or transcription region of the target gene in the base sequence information by the processing of the region-specific base sequence creation part 102i and may store the created partial base sequence information in a predetermined memory region of the partial base sequence file 106b.

In step SA-1, the partial base sequence creation part 102a may create partial base sequence information having a predetermined number of bases which is common in a plurality of base sequence information derived from different organisms (e.g., human base sequence information and mouse base sequence information) by the processing of the common base sequence creation part 102j and may store the created partial base sequence information in a predetermined memory region of the partial base sequence file 106b. Furthermore, common partial base sequence information having a predetermined number of bases which is common in a plurality of analogous base sequence information in the same species may be created.

In step SA-1, the partial base sequence creation part 102a may create partial base sequence information having a predetermined number of bases from segments corresponding to coding regions or transcription regions of the target gene in a plurality of base sequence information derived from different species by the processing of the region-specific base sequence creation part 102i and the common base sequence creation part 102j and may store the created partial base sequence information in a predetermined memory region of the partial base sequence file 106b. Furthermore, common partial base sequence information having a predetermined number of bases may be created from segments corresponding to coding regions or transcription regions of the target gene in a plurality of analogous base sequence information in the same species.

Furthermore, in step SA-1, the partial base sequence creation part 102a may create partial base sequence information containing an overhanging portion by the processing of the overhanging portion-containing base sequence creation part 102k. Specifically, for example, the partial base sequence creation part 102a may create partial base sequence information to which the overhanging portion inclusion information which shows the inclusion of the overhanging portion by the processing of the overhanging portion-containing base sequence creation part 102k and may store the created partial base sequence information and the overhanging portion inclusion information so as to be associated with each other in a predetermined memory region of the partial base sequence file 106b.

The upper limit of the predetermined number of bases is, in the case of not including the overhanging portion, preferably 28 or less, more preferably 22 or less, and still more preferably 20 or less, and in the case of including the overhanging portion, preferably 32 or less, more preferably 26 or less, and still more preferably 24 or less. The lower limit of the predetermined number of bases is, in the case of not including the overhanging portion, preferably at least 13, more preferably at least 16, and still more preferably at least 18, and in the case of including the overhanging portion, preferably at least 17, more preferably at least 20, and still more preferably at least 22. Most preferably, the predetermined number of bases is, in the case of not including the overhanging portion, 19, and in the case of including the overhanging portion, 23.

Subsequently, the base sequence processing apparatus 100 determines whether the 3' end base in the partial base sequence information created in step SA-1 is adenine, thymine, or uracil by the processing of the 3' end base determination part 102b and stores the determination result in a predetermined memory region of the determination result file 106c (step SA-2). Specifically, for example, the base sequence processing apparatus 100 may store "1" when the 3' end base in the partial base sequence information created in step SA-1 is adenine, thymine, or uracil, by the processing of the 3' end base determination part 102b, and "0" when it is not, in a predetermined memory region of the determination result file 106c.

Subsequently, the base sequence processing apparatus 100 determines whether the 5' end base in the partial base sequence information created in step SA-1 is guanine or cytosine by the processing of the 5' end base determination part 102c and stores the determination result in a predetermined memory region of the determination result file 106c (step SA-3). Specifically, for example, the base sequence processing apparatus 100 may store "1" when the 5' end base in the partial base sequence information created in step SA-1 is guanine or cytosine, by the processing of the 5' end base determination part 102c, and "0" when it is not, in a predetermined memory region of the determination result file 106c.

Subsequently, the base sequence processing apparatus 100 determines whether the base sequence information comprising 7 bases at the 3' end in the partial base sequence information created in step SA-1 is rich in one or more types of bases selected from the group consisting of adenine, thymine, and uracil by the processing of the predetermined base inclusion determination part 102d and stores the determination result in a predetermined memory region of the determination result file 106c (step SA-4). Specifically, for example, the base sequence processing apparatus 100, by the processing of the predetermined base inclusion determination part 102d, may store the number of bases corresponding to one or more types of bases selected from the group consisting of adenine, thymine, and uracil contained in the base sequence information comprising 7 bases at the 3' end in the partial base sequence information created in step SA-1 in a predetermined memory region of the determination result file 106c. The rule of determination in step SA-4 regulates that base sequence information in the vicinity of the 3' end of the partial base sequence information created in step SA-1 contains a rich amount of one or more types of bases selected from the group consisting of adenine, thymine, and uracil, and more specifically, as an index for search, regulates that the base sequence information in the range from the 3' end base to the seventh base from the 3' end is rich in one or more types of bases selected from the group consisting of adenine, thymine, and uracil.

In step SA-4, the phrase "base sequence information rich in" corresponds to the phrase "sequence rich in" described in the column <1> Method for searching target base sequence for RNA interference. Specifically, for example, when the partial base sequence information created in step SA-1 comprises about 19 bases, in the base sequence information comprising 7 bases at the 3' end in the partial base sequence information, preferably at least 3 bases, more preferably at least 4 bases, and particularly preferably at least 5 bases, are one or more types of bases selected from the group consisting of adenine, thymine, and uracil.

Furthermore, in steps SA-2 to SA-4, when partial base sequence information including the overhanging portion is determined, the sequence segment excluding the overhanging portion in the partial base sequence information is considered as the determination target.

Subsequently, based on the determination results in steps SA-2, SA-3, and SA-4, the base sequence processing apparatus 100, by the processing of the prescribed sequence selection part 102e, selects prescribed sequence information which specifically causes RNA interference in the target gene from the partial base sequence information created in step SA-1 and stores it in a predetermined memory region of the prescribed sequence file 106d (Step SA-5).

Specifically, for example, the base sequence processing apparatus 100, by the processing of the prescribed sequence selection part 102e, selects partial base sequence information, in which the 3' end base has been determined as adenine, thymine, or uracil in step SA-2, the 5' end base has been determined as guanine or cytosine in step SA-3, and base sequence information comprising 7 bases at the 3' end in the partial base sequence information has been determined as being rich in one or more types of bases selected from the group consisting of adenine, thymine, and uracil, as prescribed sequence information, and stores it in a predetermined memory region of the prescribed sequence file 106d. Specifically, for example, the base sequence processing apparatus 100, by the processing of the prescribed sequence selection part 102e, may calculate a product of the values outputted in steps SA-2, SA-3, and SA-4 and, based on the product, select prescribed sequence information from the partial base sequence information created in step SA-1.

Here, the base sequence processing apparatus 100 may add an overhanging portion to at least one end of the prescribed sequence information selected in step SA-5 by the processing of the overhanging portion-adding part 102f, and may store it in a predetermined memory region of the prescribed sequence file 106d. Specifically, for example, by the processing of the overhanging portion-adding part 102f, the base sequence processing apparatus 100 may change the prescribed sequence information stored in the prescribed sequence information section in the prescribed sequence file 106d to prescribed sequence information in which an overhanging portion is added to at least one end. Additionally, for example, when a target is searched, the overhanging portion may be added to both ends of the prescribed sequence information.

Additionally, the number of bases in the overhanging portion corresponds to the number of bases described in the column <2> Method for designing base sequence of polynucleotide for causing RNA interference. Specifically, for example, 2 is particularly suitable as the number of bases.

Furthermore, the base sequence processing apparatus 100, by the processing of the identical/similar base sequence search part 102g, may search base sequence information that is identical or similar to the prescribed sequence information selected in step SA-5 from other base sequence information (e.g., base sequence information published in a public database, such as RefSeq of NCBI) using a known homology search method, such as BLAST, FASTA, or ssearch, and based on the searched identical or similar base sequence information, by the unrelated gene target evaluation process performed by the unrelated gene target evaluation part 102h, may evaluate whether the prescribed sequence information targets genes unrelated to the target gene.

Specifically, for example, the base sequence processing apparatus 100, by the processing of the identical/similar base sequence search part 102g, may search base sequence information that is identical or similar to the prescribed sequence information selected in step SA-5 from other base sequence information (e.g., base sequence information published in a public database, such as RefSeq of NCBI) using a known homology search method, such as BLAST, FASTA, or ssearch. The unrelated gene target evaluation part 102h, by the processing of the total sum calculation part 102m, may calculate the total sum of the reciprocals of the values showing the degree of identity or similarity based on the total amount of base sequence information on the genes unrelated to the target gene in the searched identical or similar base sequence information and the values showing the degree of identity or similarity (e.g., "E value" in BLAST, FASTA, or ssearch) attached to the base sequence information on the genes unrelated to the target gene. The unrelated gene target evaluation part 102h, by the processing of the total sum-based evaluation part 102n, may evaluate whether the prescribed sequence information targets genes unrelated to the target gene based on the calculated total sum.

Here, the details of the unrelated gene target evaluation process performed by the unrelated gene target evaluation part 102h will be described with reference to Figure 24.

Figure 24 is a flowchart which shows an example of the unrelated gene evaluation process of the system in this embodiment.

First, the base sequence processing apparatus 100, by the processing of the identical/similar base sequence search part 102g, searches base sequence information that is identical or similar to the prescribed sequence information selected in step SA-5 from other base sequence information (e.g., base sequence information published in a public database, such as RefSeq of NCBI) using a known homology search method, such as BLAST, FASTA, or ssearch, and stores identification information of the prescribed sequence information ("partial base sequence identification information" in Figure 19), identification information of the searched identical or similar base sequence information ("reference sequence identification information" in Figure 19), and the value showing the degree of identity or similarity (e.g., "E value" in BLAST, FASTA, or ssearch) ("degree of identity or similarity" in Figure 19) attached to the searched identical or similar base sequence information so as to be associated with each other in a predetermined memory region of the degree of identity or similarity file 106f.

Subsequently, the unrelated gene target evaluation part 102h, by the processing of the total sum calculation part 102m, calculates the total sum of reciprocals of the values showing the degree of identity or similarity based on the total amount of base sequence information on the genes unrelated to the target gene in the searched identical or similar base sequence information and the values showing the degree of identity or similarity (e.g., "E value" in BLAST, FASTA, or ssearch) attached to the base sequence information on the genes unrelated to the target gene, and stores identification information of the prescribed sequence information ("partial base sequence identification information" in Figure 20) and the calculated total sum ("total sum" in Figure 20) so as to be associated with each other in a predetermined memory region of the evaluation result file 106g (step SB-1).

Subsequently, the unrelated gene target evaluation part 102h, by the processing of the total sum-based evaluation part 102n, evaluates whether the prescribed sequence information targets genes unrelated to the target gene based on the total sum calculated in step SB-1 (e.g., based on the size of the total sum calculated in step SB-1), and stores the evaluation results ("nontarget" and "target" in Figure 20) in a predetermined memory region of the evaluation result file 106g (Step SB-2).

The main process is thereby completed.

<8> Pharmaceutical composition

The present invention also provides a pharmaceutical composition comprising a pharmaceutically effective amount of the polynucleotide of the present invention. The use of the pharmaceutical composition of the present invention is not particularly limited. Since the pharmaceutical composition inhibits, through RNAi, the expression of a gene containing a target sequence of each polynucleotide, which is an active ingredient, it is useful in preventing and/or treating diseases in which such genes are involved.

The sequence to be targeted by the polynucleotide contained in the pharmaceutical composition of the present invention is a sequence selected as a prescribed sequence conforming to the above rules (a) to (f). Preferably, such a sequence may be any of SEQ ID NOs: 47 to 817081. In particular, if the target sequence is a sequence highly specific to the target gene, the polynucleotide selectively produces an inhibitory effect only on the expression of the target gene containing the target sequence, but not on the other genes (i.e., the polynucleotide has less off-target effect), thus reducing influences of side effects, etc. It is therefore more preferred that the target sequence of the polynucleotide has high specificity to the target gene. Among the selected sequences (e.g., SEQ ID NOs: 47 to 817081), a sequence whose off-target effect can be further reduced is preferred as a prescribed sequence conforming to the above rules (a) to (f). As a preferred prescribed sequence of the target gene, it is possible to select a sequence which contains mismatches of at least 3 bases against the base sequences of other genes and for which there is only a minimum number of other genes having a base sequence containing mismatches of at least 3 bases. The requirement "there is only a minimum number of other genes" means that "other genes having a base sequence containing mismatches of at least 3 bases" (i.e., similar genes) are as few in number as possible; for example, there are preferably 10 or less genes, more preferably 6 or less genes, still more preferably only one gene, or most preferably no gene.

For example, the 53998 sequences shown in Figure 46 are obtained among SEQ ID NOs: 47 to 817081 by selecting sequences which contain mismatches of 3 bases against the base sequences of other genes (i.e., prescribed sequences of 19 bases in which 16 bases other than these 3 mismatched bases are the same as those of other genes) and for which there is only a minimum number of other genes having a base sequence containing mismatches of 3 bases. Thus, the target sequence is particularly preferably any of these sequences. With respect to these sequences, most of their relationships have been identified, such as genes containing these target sequences, disease names related to these genes, biological function categories according to GO_ID of these genes in Gene Ontology, and biological functions reported in documents. These relationships are shown in Figure 46. The polynucleotide of the present invention inhibits the expression of a gene containing a target sequence through RNAi, and hence allows treatment and/or prevention of diseases related to the gene and control of its biological functions. Once a target sequence of the polynucleotide has been identified on the basis of the disclosures of the present specification, drawings and so on, those skilled in the art will readily understand diseases and/or biological functions on which the polynucleotide produces an effect.

Thus, the pharmaceutical composition of the present invention is preferably useful in treating and/or preventing the diseases listed in the column "Related Disease" of Figure 46 or diseases associated with the gene-related biological functions listed in the columns "Biological Function Category" and/or "Reported Biological Function" of Figure 46.

The pharmaceutical composition of the present invention is more preferably useful in treating and/or preventing a disease in which a gene belonging to any of the following 1) to 9) is involved:

1) an apoptosis-related gene;
2) phosphatase or a phosphatase activity-related gene;
3) a cell cycle-related gene;
4) a receptor-related gene;
5) an ion channel-related gene;
6) a signal transduction system-related gene;
7) kinase or a kinase activity-related gene;
8) a transcription regulation-related gene; or
9) G protein-coupled receptor or a G protein-coupled receptor-related gene.

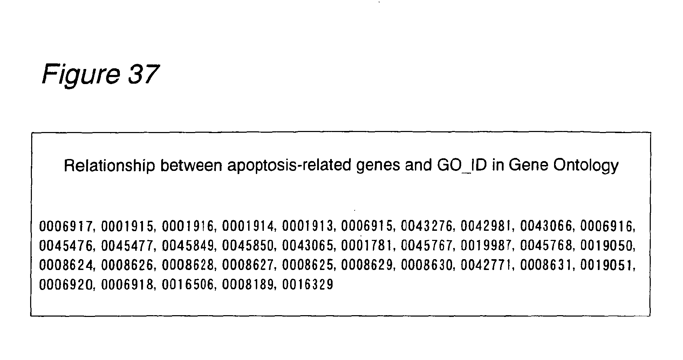

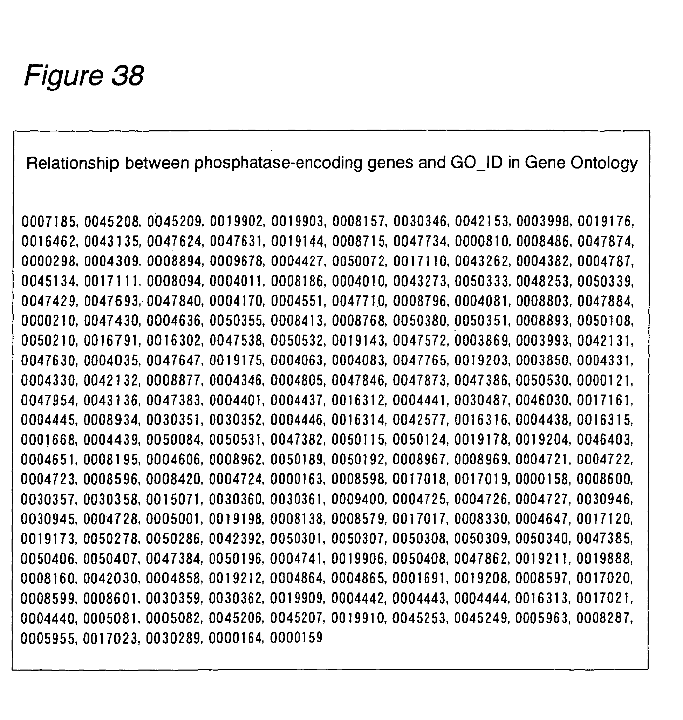

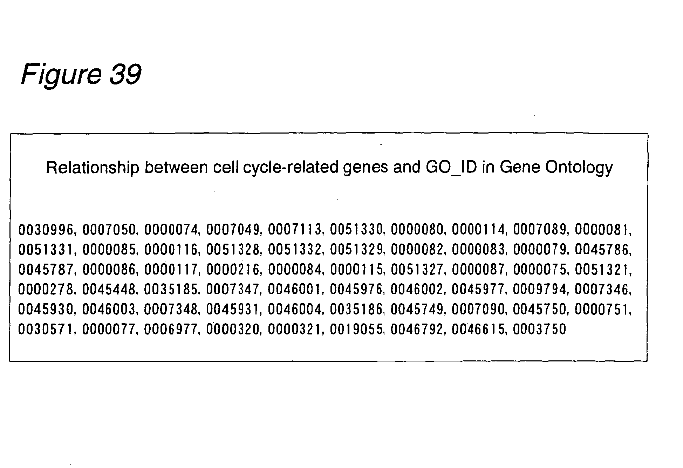

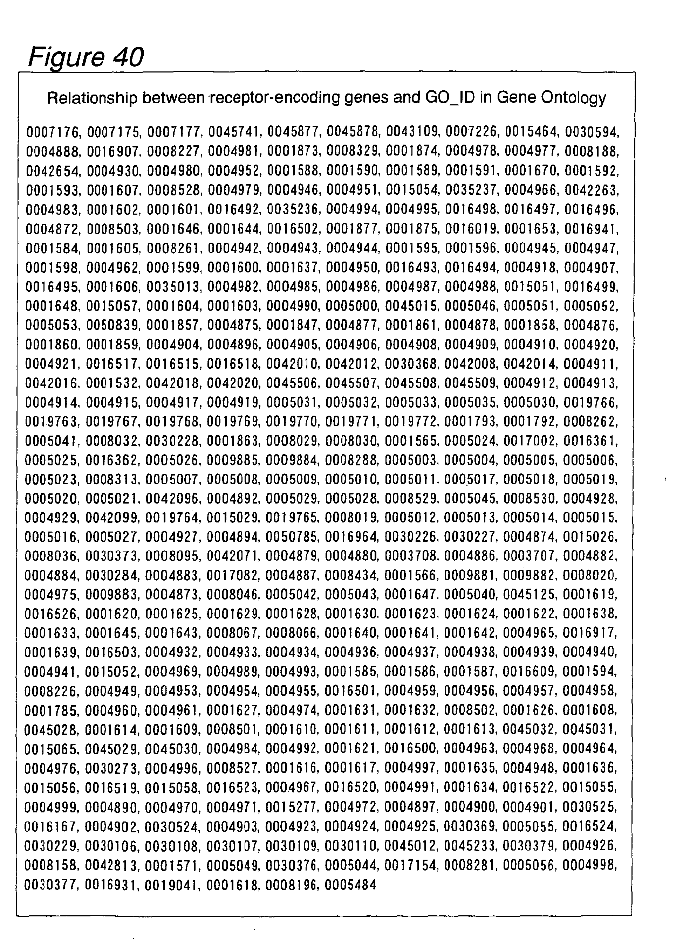

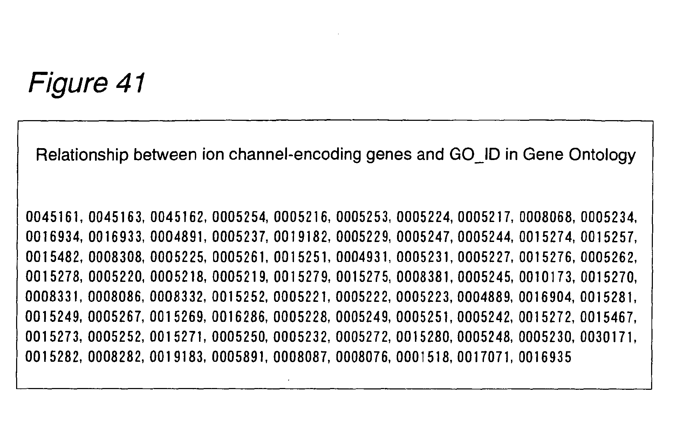

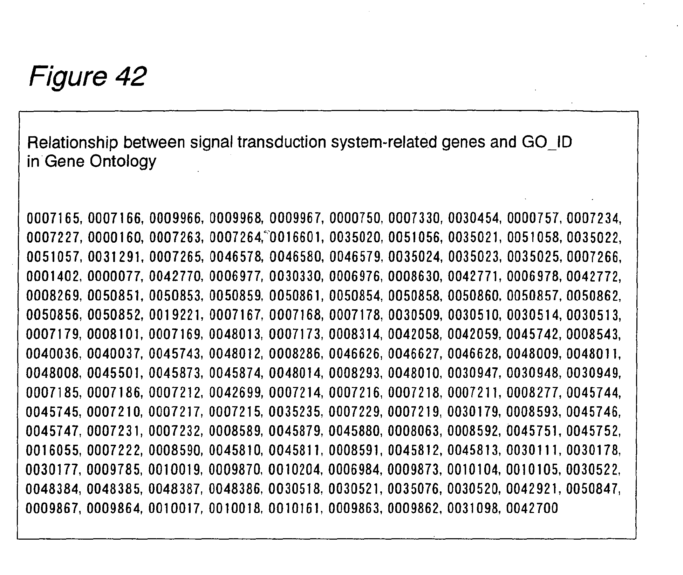

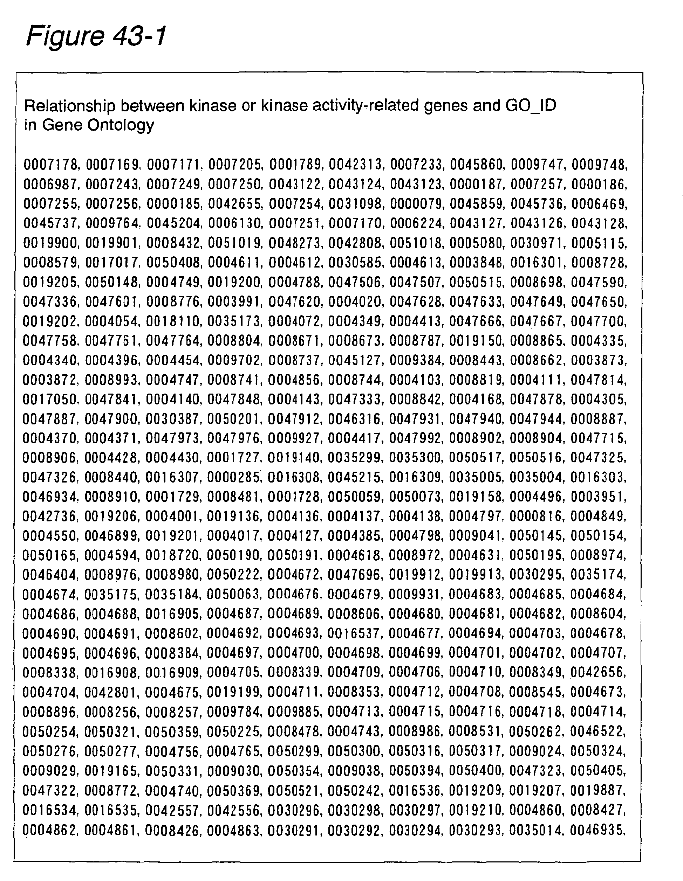

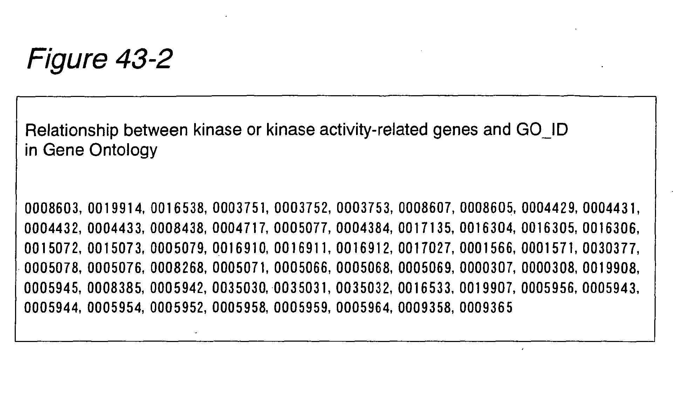

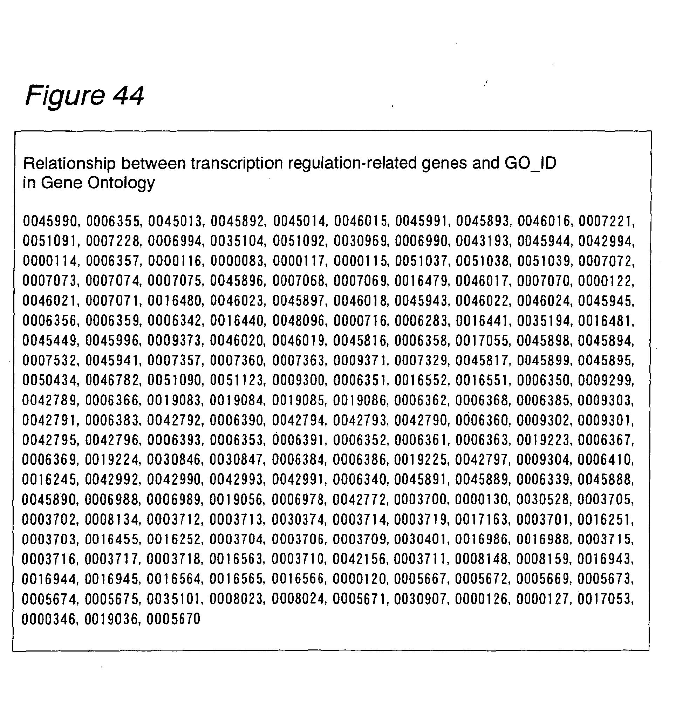

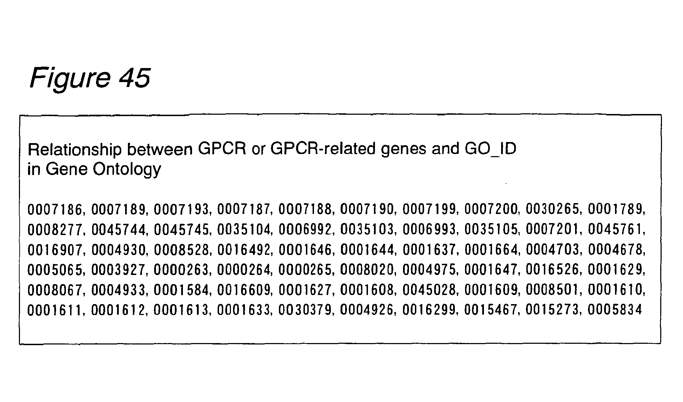

The column "Biological Function Category" of Figure 46 shows biological functions classified into the above 9 categories. To give more detailed information about what biological function is provided by genes belonging to each group of 1) to 9) above, the relationship with GO_ID in Gene Ontology is shown for each gene in Figures 37 to 45. The 7-digit numbers shown in Figures 37 to 45 each denote an attribute (more specifically an ID number) in Gene Ontology belonging to each group.

For details about Gene Ontology, refer to, e.g., the Gene Ontology Consortium, "Gene Ontology Consortium home page," [online], 1999, the Gene Ontology Consortium, [searched on October 25, 2004], Internet <URL: http: //www.geneontology.org/>.

For example, Gene Ontology defines gene attributes such as "signal transducer activity (GO:0004871)" and "receptor activity (GO:0004872)" and further defines inherited relationships between attributes to describe, e.g., that "the attribute of receptor activity inherits the attribute of signal transducer activity." The definitions of attributes and inherited relationships between attributes are available from the Gene Ontology Consortium (http://www.geneontology.org/). Likewise, corresponding relationships between individual human or mouse genes and Gene Ontology attributes are available from various databases including the Cancer genome Anatomy project (http://cgap.nci.nih.gov/). Gene Ontology data of genes, for example, indicate that the human ZYX gene (NM_003461) has receptor activity and further lead to the fact that the ZYX gene also has signal transducer activity when using inherited relationships between attributes.

With respect to gene attributes (annotations), Gene Ontology provides a definition for each attribute and defines inherited relationships between attributes. These inherited relationships between attributes in the ontology of genes form directed acyclic graphs (DAGs). In Gene Ontology, genes are classified and organized by "molecular function", "biological process" and "cellular component." Moreover, each classification defines inherited relationships between attributes. Once the ID numbers of attributes in Gene Ontology have been identified, those skilled in the art will understand the details of each attribute from its ID number.

In addition to the above 9 biological function categories according to Gene Ontology, Figure 46 shows biological functional information of each gene, which is obtained from the reported documents. More specifically, biological functional information of each gene reported in the documents obtained from PubMed is shown in the column "Reported Biological Function."

In a more preferred embodiment, the pharmaceutical composition of the present invention more preferably comprises a polynucleotide targeting the base sequence shown in any of SEQ ID NOs listed in the column "SEQ ID NO (human)" or "SEQ ID NO (mouse)" of Figure 46. Each polynucleotide is useful in treating and/or preventing a disease shown in the column "Related Disease" under the same reference number as that of its target sequence. Alternatively, each polynucleotide is useful in controlling a biological function(s) (e.g., inhibition and promotion) shown in the column "Biological Function Category" or "Reported Biological Function" under the same reference number, or in treating and/or preventing a disease(s) associated with the biological function(s).

Table 1 in Example 8 described herein later shows the polynucleotides of the present invention, more specifically, siRNA sense strands corresponding to these polynucleotides (whose base sequences are shown in the column "siRNA-sense" of Table 1), their antisense strands (whose base sequences are shown in the column "siRNA-antisense" of Table 1, provided that the sequences are shown in the direction from 3' to 5'), target genes to be targeted by these siRNA sequences for RNAi (which are shown in the column "Gene Name" of Table 1) and the positions of target sequences in these genes. As shown in Table 1, the polynucleotides of the present invention served as siRNA-sense or siRNA-antisense strands to produce an RNAi, effect against the genes listed in the column "Gene Name" of Table 1, thereby significantly inhibiting the expression of these genes. Thus, pharmaceutical compositions comprising the polynucleotides of the present invention are useful in treating or preventing diseases related to the genes listed in the column "Gene Name" of Table 1, more specifically, diseases corresponding to the genes, as listed in the column "Related Disease" of Figure 46, as well as diseases associated with biological functions corresponding to the genes, as listed in the columns "Biological Function Category" and/or "Reported Biological Function" of Figure 46.

In Example 8, the sequences used as targets of siRNA (see the column "Target Sequence" of Table 1) were selected at random from the 53998 target sequences shown in Figure 46 among possible target sequences to be targeted by the polynucleotides of the present invention. As described later, all the selected target sequences were confirmed to have an RNAi effect. When the results thus obtained in Example 8 were statistically processed by the "population ratio estimation method," it was found to be statistically reasonable that the polynucleotides of the present invention (more specifically, polynucleotides whose one strand in the double-stranded region is a sequence homologous to a prescribed sequence of a target gene shown in any of SEQ ID NOs: 47 to 817081) would produce an inhibitory effect on the expression of target genes, and that particularly when using polynucleotides in which the above prescribed sequence is any of the 53998 sequences shown in Figure 46, almost all of them would produce an inhibitory effect on the expression of target genes.

Genes to be targeted by the polynucleotides of the present invention may be those related to any of the diseases shown in Figure 46. Particularly when the target genes are those related to various cancers including bladder cancer, breast cancer, colorectal cancer, gastric cancer, hepatoma, lung cancer, melanoma, ovarian cancer, pancreas cancer, prostate cancer, oral cancer, skin cancer and thyroid gland cancer, it becomes possible to treat or prevent these various cancers through an inhibitory effect on the expression of the genes. Thus, without being limited thereto, the pharmaceutical composition of the present invention is useful in treating or preventing any cancer selected from those listed above.

The pharmaceutical composition of the present invention most preferably comprises a polynucleotide having any of the base sequences shown in SEQ ID NOs: 817102 to 817651. Each polynucleotide can inhibit the expression of its target gene (see the column "Gene Name" of Table 1) and hence is useful in treating and/or preventing a disease related to the gene (more specifically, see the column "Related Disease" of Figure 46 with respect to the gene) or a disease associated with a biological function(s) of the gene (more specifically, see the columns "Biological Function Category" and/or "Reported Biological Function" of Figure 46 with respect to the gene). It is also possible to use sequences having mutations (e.g., mismatches as described above) in the base sequences of these SEQ ID NOs, as long as their RNAi effect is not impaired.

In Examples 1 to 8 described later, a large number of polynucleotides selected according to the selection method of the present invention were demonstrated to produce a significant RNAi effect. Thus, those skilled in the art will easily understand that polynucleotides selected according to common rules produce the same RNAi effect. Moreover, the validity of these rules is also evident from the above statistically processed results. It is therefore easily understood that in the genes shown in Table 1, for example, when a sequence different from the disclosed target sequence is selected from the same gene according to the present invention from a different position than the actually disclosed target position of the target sequence, the same inhibitory effect on gene expression is obtained for the same gene. Moreover, once an inhibitory effect on the expression of a gene related to a certain disease has been identified, it will be easily understood that when its target sequence is selected according to the present invention to prepare a polynucleotide, treatment and/or prevention of the disease through an inhibitory effect on expression is also possible for other genes related to the same disease.

Moreover, Example 7 of the present invention has shown that even in the case of genes other than those containing a sequence completely homologous to a target sequence, when these other genes contain similar sequences having a small number (preferably 2 or less bases) of mismatches, these similar sequence portions may serve as targets for RNA interference. Thus, such genes containing similar sequences, which are other than those containing a sequence completely homologous to a target sequence, are also used as targets of the polynucleotide of the present invention and are expected to produce an RNA interference-based inhibitory effect on expression. The pharmaceutical composition of the present invention is therefore also useful in treating and/or preventing diseases in which these genes are involved.

In a case where a polynucleotide for causing RNAi is used for a pharmaceutical composition, a pharmaceutically acceptable carrier or diluent and the polynucleotide of the present invention may be blended into a pharmaceutical composition. In this case, the ratio of active ingredient to carrier or diluent ranges from about 0.01% to about 99.9% by weight.

The above carrier or diluent may be in gaseous, liquid or solid form. Examples of the carrier include aqueous or alcohol solutions or suspensions, oil solutions or suspensions, oil-in-water or water-in-oil emulsions, hydrophobic carriers, liquid vehicles, and microcrystals.

Moreover, the pharmaceutical composition of the present invention comprising the above polynucleotide may further comprise, for example, at least one of the following: other therapeutic agents, surfactants, fillers, buffers, dispersants, antioxidants and preservatives. Such a pharmaceutical composition may be a formulation for oral, intraoral, intrapulmonary, intrarectal, intrauterine, intratumoral, intracranial, nasal, intramuscular, subcutaneous, intravascular, intrathecal, percutaneous, intracutaneous, intraarticular, intracavitary, ocular, vaginal, ophthalmic, intravenous, intraglandular, interstitial, intralymphatic, implantable, inhalant or sustained release use, or an enteric-coated formulation.

For example, an oral formulation comprising a polynucleotide may be in a dosage form of powders, sugar-coated pills, tablets, capsules, syrups, aerosols, solutions, suspensions or emulsions (e.g., oil-in-water or water-in-oil emulsions). Alternatively, topical formulations are also acceptable, whose carrier is a cream, a gel, an ointment, a syrup, an aerosol, a patch, a solution, a suspension or an emulsion. Moreover, injectable formulations and percutaneous formulations are also acceptable, whose carrier is an aqueous or alcohol solution or suspension, an oil solution or suspension, or an oil-in-water or water-in-oil emulsion. Further, rectal formulations and suppositories are also acceptable. Furthermore, it is also possible to use formulations provided in the form of implants, capsules or cartridges, as well as respirable or inhalant formulations, and aerosols.

The dose of such a pharmaceutical composition comprising a polynucleotide will be selected as appropriate for the symptoms, age and body weight of a patient, etc. With respect to how to administer the pharmaceutical composition to a recipient, in a case where the recipient is a cell or tissue, administration may be accomplished by using techniques such as the calcium phosphate method, electroporation, lipofection, virus infection, and immersion in a polynucleotide solution. Likewise, when introducing into an embryo, it is possible to use microinjection, electroporation, virus infection, etc. For administration, conventionally used commercially available reagents, instruments, apparatuses, kits and the like may be used. For example, an introducing reagent such as TransIT®-In Vivo Gene Delivery System or TransIT®-QR Hydrodynamic Delivery Solution (both manufactured by Takara Bio Inc., Japan) may be used for administration to cells in living organisms. Likewise, for introduction by virus infection, retrovirus vectors (e.g., RNAi Ready pSIREN-RetroQ Vector, manufactured by BD Biosciences Clontech), adenovirus vectors (e.g., BD Knockout Adenoviral RNAi System, manufactured by BD Biosciences Clontech) or lentivirus vectors (e.g., RetroNectin, manufactured by Takara Bio Inc., Japan) may also be used.

In a case where the recipient is a plant, administration may be accomplished by using techniques for injection or spraying into a cavity or interstitial cells in the plant. Likewise, in a case where the recipient is an animal individual, administration may be accomplished, e.g., by oral, parenteral, transvaginal, transrectal, transnasal, transocular or intraperitoneal route. These techniques allow systemic or topical administration of one or more polynucleotides at the same time or at different times. By way of example for oral administration, a pharmaceutical agent or food incorporated with a polynucleotide(s) may be taken directly. Alternatively, by way of example for oral and transnasal routes, administration may be performed using an inhalator. Likewise, by way of example for parenteral route, syringes with or without needles may be used for, e.g., subcutaneous, intramuscular or intravenous administration.

<9> Composition for inhibiting gene expression

The present invention further provides a composition for inhibiting gene expression to inhibit the expression of a target gene, which comprises the polynucleotide of the present invention.

As has been shown in the present invention, the polynucleotide of the present invention produces an expression inhibitory effect against a gene containing each target sequence. Inhibited expression of the gene controls, preferably inhibits, biological functions of the gene.

Preferably, the target gene is related to any of the diseases listed in the column "Related Disease" of Figure 46.

Preferably, the target gene is any of the genes listed in the column "Gene Name" of Figure 46.

Alternatively, the target gene is a gene belonging to any of the following 1) to 9):

1) an apoptosis-related gene;
2) phosphatase or a phosphatase activity-related gene;
3) a cell cycle-related gene;
4) a receptor-related gene;
5) an ion channel-related gene;
6) a signal transduction system-related gene;
7) kinase or a kinase activity-related gene;
8) a transcription regulation-related gene; or
9) G protein-coupled receptor or a G protein-coupled receptor-related gene.

As described in the above section "Pharmaceutical composition," the polynucleotide of the present invention (more specifically siRNA) has been found to produce an RNAi effect based on the results of Example 8 and their statistically processed results. In particular, in Example 8 described later, the polynucleotide of the present invention was confirmed to produce an inhibitory effect on mRNA expression (i.e., RNAi effect) against all the genes listed in the column "Gene Name" of Table 1. Thus, the composition for inhibiting gene expression, which comprises the polynucleotide of the present invention, may target any of the target genes shown in Figure 46; the target gene is more preferably any of the genes listed in the column "Gene Name" of Table 1. With respect to each gene in Table 1, it is preferably desirable to use a sequence of siRNA-sense or siRNA-antisense shown in the same line as the gene. It is also possible to use sequences having mutations (e.g., mismatches as described above) in these base sequences, as long as their inhibitory effect on expression is not impaired.

If the target gene is any of the genes shown in Table 1, the composition is useful in treating and/or preventing a disease related to the gene (more specifically, see the column "Related Disease" of Figure 46 with respect to the gene) or a disease associated with a biological function(s) of the gene (more specifically, see the columns "Biological Function Category" and/or "Reported Biological Function" of Figure 46 with respect to the gene).

For example, genes to be targeted by the composition for inhibiting gene expression in accordance with the present invention may be those related to any of the diseases shown in Figure 46. Particularly when the target genes are those related to various cancers including bladder cancer, breast cancer, colorectal cancer, gastric cancer, hepatoma, lung cancer, melanoma, ovarian cancer, pancreas cancer, prostate cancer, oral cancer, skin cancer and thyroid gland cancer, it becomes possible to treat or prevent these various cancers through an inhibitory effect on the expression of the genes. Thus, without being limited thereto, the pharmaceutical composition of the present invention is useful in treating or preventing any cancer selected from those listed above.

In Examples 2 to 5 described herein later, the RNAi effect of the polynucleotide of the present invention against the genes of human vimentin, luciferase, SARS virus and the like was examined as a relative expression level of mRNA compared to the control. Figures 31, 32 and 35 show the results of mRNA expression levels measured by quantitative PCR. In Figures 31, 32 and 35, the relative mRNA expression levels are respectively reduced to about 7-8% (Example 2, Figure 31), about 12-13% (Example 3, Figure 32), and a few % to less than about 15% (Example 5, Figure 35); the polynucleotide of the present invention was confirmed to have an inhibitory effect on the expression of each gene. Likewise, Figure 34 from Example 4 shows the results of mRNA expression levels (as RNAi effect) examined by luciferase activity. The luciferase activity was also reduced to a few % to less than about 20%, as compared to the control.

Moreover, in Example 8, among the genes shown in Figure 46 whose related diseases and/or biological functions have been identified, about 300 genes selected at random were examined for the expression levels of their mRNA in human-derived HeLa cells, expressed as relative expression levels. As shown in Table 1, the RQ values (described later) that were calculated to evaluate an inhibitory effect on the expression of these genes, i.e., an RNAi effect were all less than 1, and almost all less than 0.5.

In the composition for inhibiting gene expression in accordance with the present invention, the phrase "inhibiting the expression of the target gene" means that the mRNA expression level of the target gene is substantially reduced. If the mRNA expression level has been substantially reduced, inhibited expression has been achieved regardless of the degree of change in the mRNA expression level. In light of the results from Examples 2-5 and 8 as described above, the composition for inhibiting gene expression in accordance with the present invention is identified to preferably cause at least a 50% or more reduction in the mRNA expression level of the target gene.

<10> Method for treating or preventing diseases

The present invention further provides a method for treating or preventing the diseases listed in the column "Related Disease" of Figure 46, which comprises administering a pharmaceutically effective amount of the polynucleotide of the present invention.

[Other embodiments]

One preferred embodiment of the present invention has been described above. However, it is to be understood that the present invention can be carried out in various embodiments other than the embodiment described above within the scope of the technical idea described in the claims.

For example, although the case in which the base sequence processing apparatus 100 performs processing on a stand-alone mode has been described, construction may be made such that processing is performed in accordance with the request from a client terminal which is constructed separately from the base sequence processing apparatus 100, and the processing results are sent back to the client terminal. Specifically, for example, the client terminal transmits a name of the target gene for RNA interference (e.g., gene name or accession number) or base sequence information regarding the target gene to the base sequence processing apparatus 100, and the base sequence processing apparatus 100 performs the processes described above in the controller 102 on base sequence information corresponding to the name or the base sequence information transmitted from the client terminal to select prescribed sequence information which specifically causes RNA interference in the target gene and transmits it to the client terminal. In such a case, for example, by acquiring sequence information from a public database, siRNA against the gene in query may be selected. Alternatively, for example, siRNA for all the genes may be calculated and stored preliminarily, and siRNA may be immediately selected in response to the request from the client terminal (e.g., gene name or accession number) and the selected siRNA may be sent back to the client terminal.

Furthermore, the base sequence processing apparatus 100 may check the specificity of prescribed sequence information with respect to genes unrelated to the target gene. Thereby, it is possible to select prescribed sequence information which specifically causes RNA interference only in the target gene.

Furthermore, in the system comprising a client terminal and the base sequence processing apparatus 100, an interface function may be introduced in which, for example, the results of RNA interference effect of siRNA (e.g., "effective" or "not effective") are fed back from the Web page users on the Web, and the experimental results fed back from the users are accumulated in the base sequence processing apparatus 100 so that the sequence regularity of siRNA effective for RNA interference is improved.

Furthermore, the base sequence processing apparatus 100 may calculate base sequence information of a sense strand of siRNA and base sequence information of an antisense strand complementary to the sense strand from the prescribed sequence information. Specifically, for example, when "caccctgacccgcttcgtcatgg" is selected as 23-base sequence information wherein 2-base overhanging portions are added to both ends of the prescribed sequence as a result of the processes described above, the base sequence processing apparatus 100 calculates the base sequence information of a sense strand "5'-CCCUGACCCGCUUCGUCAUGG-3'" and the base sequence information of an anti-sense strand "5'-AUGACGAAGCGGGUCAGGGUG-3'". Consequently, it is not necessary to manually arrange the sense strand and the antisense strand when a polynucleotide is ordered, thus improving convenience.

Furthermore, in the processes described in the embodiment, the processes described as being automatically performed may be entirely or partially performed manually, or the processes described as being manually performed may be entirely or partially performed automatically by a known method.

In addition, processing procedures, control procedures, specific names, information including various registration data and parameters, such as search conditions, examples of display screen, and database structures may be changed in any manner except when otherwise described.

Furthermore, with respect to the base sequence processing apparatus 100, the components are shown in the drawings only based on the functional concept, and it is not always necessary to physically construct the components as shown in the drawings.

For example, the process functions of the individual parts or individual units of the base sequence processing apparatus 100, in particular, the process functions performed in the controller 102, may be entirely or partially carried out by a CPU (Central Processing Unit) or programs which are interpreted and executed by the CPU. Alternatively, it may be possible to realize the functions based on hardware according to a wired logic. Additionally, the program is recorded in a recording medium which will be described below and is mechanically read by the base sequence processing apparatus 100 as required.

Namely, the memory 106, such as a ROM or HD, records a computer program which, together with OS (Operating System), gives orders to the CPU to perform various types of processing. The computer program is executed by being loaded into a RAM or the like, and, together with the CPU, constitutes the controller 102. Furthermore, the computer program may be recorded in an application program server which is connected to the base sequence processing apparatus 100 via any network 300, and may be entirely or partially downloaded as required.

The program of the present invention may be stored in a computer-readable recording medium. Here, examples of the "recording medium" include any "portable physical medium", such as a flexible disk, an optomagnetic disk, a ROM, an EPROM, an EEPROM, a CD-ROM, a MO, a DVD, or a flash disk; any "fixed physical medium", such as a ROM, a RAM, or a HD which is incorporated into various types of computer system; and a "communication medium" which holds the program for a short period of time, such as a communication line or carrier wave, in the case when the program is transmitted via a network, such as a LAN, a WAN, or Internet.

Furthermore, the "program" means a data processing method described in any language or by any description method, and the program may have any format (e.g., source code or binary code). The "program" is not always limited to the one having a single system configuration, and may have a distributed system configuration including a plurality of modules or libraries, or may achieve its function together with another program, such as OS (Operating System). With respect to specific configurations and procedures for reading the recording medium in the individual units shown in the embodiment, or installation procedures after reading, etc., known configurations and procedures may be employed.

The various types of databases, etc. (target gene base sequence file 106a ~ target gene annotation database 106h) stored in the memory 106 are storage means, such as memories (e.g., RAMs and ROMs), fixed disk drives (e.g., hard disks), flexible disks, and optical disks, which store various types of programs used for various processes and Web site provision, tables, files, databases, files for Web pages, etc.

Furthermore, the base sequence processing apparatus 100 may be produced by connecting peripheral apparatuses, such as a printer, a monitor, and an image scanner, to a known information processing apparatus, for example, an information processing terminal, such as a personal computer or a workstation, and installing software (including programs, data, etc.) which implements the method of the present invention into the information processing apparatus.

Furthermore, specific modes of distribution/integration of the base sequence processing apparatus 100, etc. are not limited to those shown in the specification and the drawings, and the base sequence processing apparatus 100, etc., may be entirely or partially distributed/integrated functionally or physically in any unit corresponding to various types of loading, etc. (e.g., grid computing). For example, the individual databases may be independently constructed as independent database units, or processing may be partially performed using CGI (Common Gateway Interface).

Furthermore, the network 300 has a function of interconnecting between the base sequence processing apparatus 100 and the external system 200, and for example, may include any one of the Internet, intranets, LANs (including both wired and radio), VANs, personal computer communication networks, public telephone networks (including both analog and digital), dedicated line networks (including both analog and digital), CATV networks, portable line exchange networks/portable packet exchange networks of the IMT2000 system, CSM system, or PDC/PDC-P system, radio paging networks, local radio networks, such as the Bluetooth, PHS networks, and satellite communication networks, such as CS, BS, and ISDB. Namely, the present system can transmit and receive various types of data via any network regardless of wired or radio.

EXAMPLES

The present invention will be described in more detail with reference to the examples. However, it is to be understood that the present invention is not restricted by the examples.

[Example 1]

<1> Gene for measuring RNAi effect and expression vector

As a target gene for measuring an RNAi effect by siRNA, a firefly (Photinus pyralis, P. pyralis) luciferase (luc) gene (P. pyralis luc gene: accession number: U47296) was used, and as an expression vector containing this gene, a pGL3-Control Vector (manufactured by Promega Corporation) was used. The segment of the P. pyralis luc gene is located between an SV40 promoter and a poly A signal within the vector. As an internal control gene, a luc gene of sea pansy (Renilla reniformis, R. reniformis) was used, and as an expression vector containing this gene, pRL-TK (manufactured by Promega Corporation) was used.

<2> Synthesis of 21-base double-stranded RNA (siRNA)

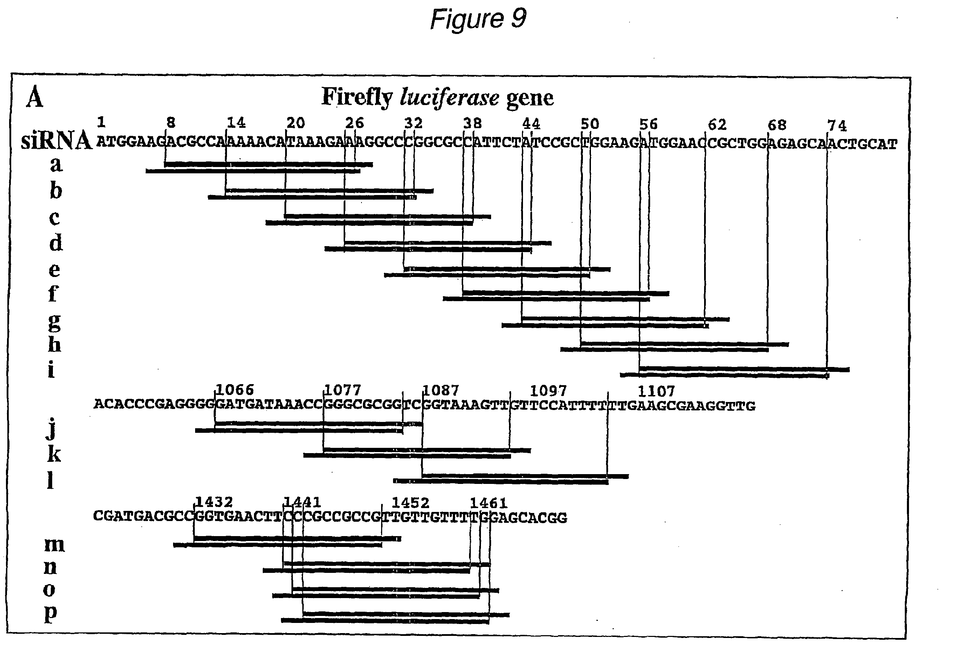

Synthesis of 21-base sense strand and 21-base antisense strand RNA (located as shown in Figure 9; a to p) was entrusted to Genset Corporation through Hitachi Instrument Service Co., Ltd.

The double-stranded RNA used for inhibiting expression of the P. pyralis luc gene was prepared by associating sense and antisense strands. In the association process, the sense strand RNA and the antisense strand RNA were heated for 3 minutes in a reaction liquid of 10 mM Tris-HCl (pH 7.5) and 20 mM NaCl, incubated for one hour at 37°C, and left to stand until the temperature reached room temperature. Formation of double-stranded polynucleotides was assayed by electrophoresis on 2% agarose gel in a TBE buffer, and it was confirmed that almost all the single-stranded polynucleotides were associated to form double-stranded polynucleotides.

<3> Mammalian cell cultivation

As mammalian cultured cells, human HeLa cells and HEK293 cells and Chinese hamster CHO-KI cells (RIKEN Cell bank) were used. As a medium, Dulbecco's modified Eagle's medium (manufactured by Gibco BRL) to which a 10% inactivated fetal bovine serum (manufactured by Mitsubishi Kasei) and as antibiotics, 10 units/ml of penicillin (manufactured by Meiji) and 50 $\mu$g/ml of streptomycin (manufactured by Meiji) had been added was used. Cultivation was performed at 37°C in the presence of 5% $CO_2$.

<4> Transfection of target gene, internal control gene, and siRNA into mammalian cultured cells

The mammalian cells were seeded at a concentration of 0.2 to 0.3 x $10^6$ cells/ml into a 24-well plate, and after one day, using a Ca-phosphate precipitation method (Saibo-Kogaku Handbook (Handbook for cell engineering), edited by Toshio Kuroki et al., Yodosha (1992)), 1.0 $\mu$g of pGL3-Control DNA, 0.5 or 1.0 $\mu$g of pRL-TK DNA, and 0.01, 0.1, 1, 10 or 100 nM of siRNA were introduced.

<5> Drosophila cell cultivation

As drosophila cultured cells, S2 cells (Schneider, I., et al., J. Embryol. Exp. Morph., 27, 353-365 (1972)) were used. As a medium, Schneider's Drosophila medium (manufactured by Gibco BRL) to which a 10% inactivated fetal bovine serum (manufactured by Mitsubishi Kasei) and as antibiotics, 10 units/ml of penicillin (manufactured by Meiji) and 50 $\mu$g/ml of streptomycin (manufactured by Meiji) had been added was used. Cultivation was performed at 25°C in the presence of 5% $CO_2$.

<6> Transfection of target gene, internal control gene, and siRNA into drosophila cultured cells

The S2 cells were seeded at a concentration of 1.0 x $10^6$ cells/ml into a 24-well plate, and after one day, using a Ca-phosphate precipitation method (Saibo-Kogaku Handbook (Handbook for cell engineering), edited by Toshio Kuroki et al., Yodosha (1992)), 1.0 $\mu$g of pGL3-Control DNA, 0.1 $\mu$g of pRL-TK DNA, and 0.01, 0.1, 1, 10 or 100 nM of siRNA were introduced.

<7> Measurement of RNAi effect

The cells transfected with siRNA were recovered 20 hours after transfection, and using a Dual-Luciferase Reporter Assay System (manufactured by Promega Corporation), the levels of expression (luciferase activities) of two types of luciferase (P. pyralis luc and reniformis luc) protein were measured. The amount of luminescence was measured using a Lumat LB9507 luminometer (EG&G Berthold).

<8> Results

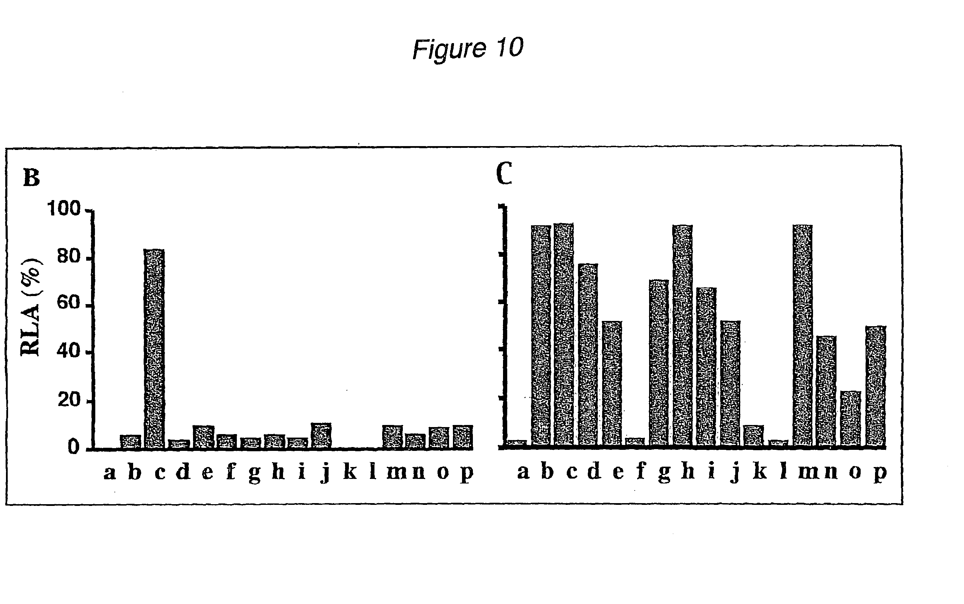

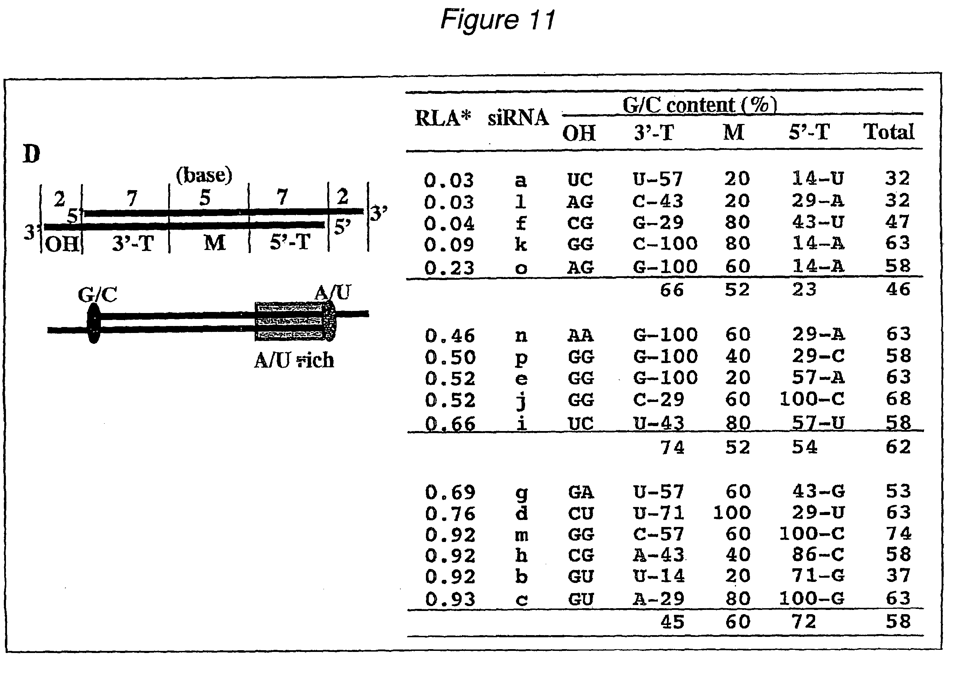

The measurement results on the luciferase activities are shown in Figure 10. Furthermore, the results of study on correspondence between the luciferase activities and the individual base sequences are shown in Figure 11.

In Figure 10, the graph represented by B shows the results in the drosophila cells, and the graph represented by C shows the results in the human cells. As shown in Figure 10, in the drosophila cells, by creating RNA with a base number of 21, it was possible to inhibit the luciferase activities in almost all the sequences. On the other hand, in the human cells, it was evident that it was difficult to obtain sequences which could inhibit the luciferase activities simply by setting the base number at 21.

Analysis was then conducted on the regularity of base sequence with respect to RNA a to p. As shown in Figure 11, with respect to 5 points of the double-stranded RNA, the base sequence was analyzed. With respect to siRNA a in the top row of the table shown in Figure 11, the relative luciferase activity (RLA) is 0.03. In the antisense strand, from the 3' end, the base sequence of the overhanging portion (OH) is UC; the G/C content (content of guanine or cytosine) in the subsequent 7 bases (3'- T in Figure 11) is 57%; the G/C content in the further subsequent 5 bases (M in Figure 11) is 20; the G/C content in the further subsequent 7 bases (5'-T in Figure 11) is 14%; the 5' end is U; and the G/C content in total is 32%. In the table, a lower RLA value indicates lower RLA activity, i.e., inhibition of the expression of luciferase.

As is evident from the results, in the base sequences of polynucleotides for causing RNA interference, it is highly probable that the 3' end is adenine or uracil and that the 5' end is guanine or cytosine. Furthermore, it has become clear that the 7-base sequence from the 3' end is rich in adenine or uracil.

[Example 2]

1. Construction of target expression vector pTREC

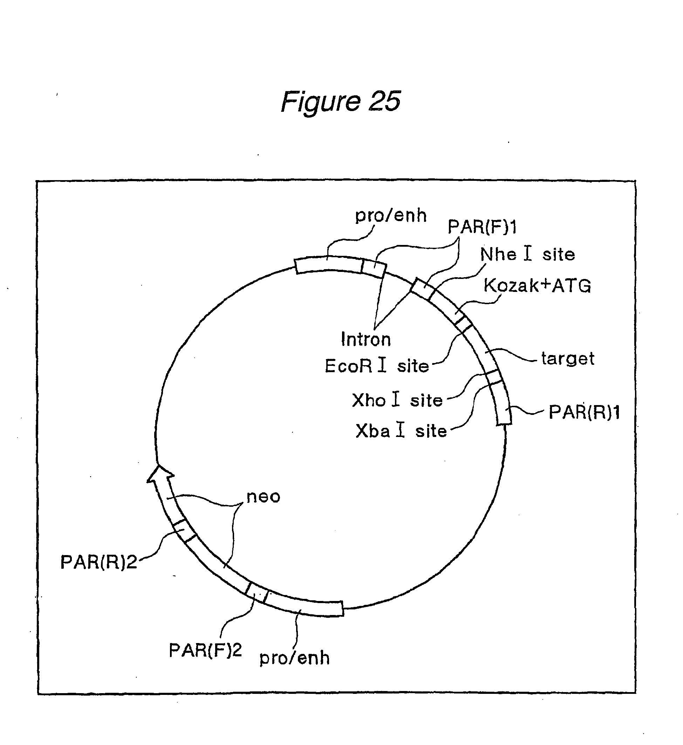

A target expression vector was constructed as follows. A target expression molecule is a molecule which allows expression of RNA having a sequence to be targeted by RNAi (hereinafter, also referred to as a "target sequence"). A target mRNA sequence was constructed downstream of the CMV enhancer/promoter of pCI-neo (GenBank Accession No. U47120, manufactured by Promega Corporation) (Figure 25). That is, the following double-stranded oligomer was synthesized, the oligomer including a Kozak sequence (Kozak), an ATG sequence, a cloning site having a 23 base-pair sequence to be targeted (target), and an identification sequence for restriction enzyme (NheI, EcoRI, XhoI) for recombination. The double-stranded oligomer consists of a sequence shown in SEQ ID NO: 1 in the sequence listing and its complementary sequence. The synthesized double-stranded oligomer was inserted into the NheI/XbaI site of the pCI-neo to construct a target expression vector pTREC (Figure 25). With respect to the intron, the intron site derived from β-globin originally incorporated in the pCI-neo was used.

5'-gctagccaccatggaattcacgcgtctcgagtctaga-3' (SEQ ID NO: 1)

The pTREC shown in Figure 25 is provided with a promoter and an enhancer (pro/enh) and regions PAR(F) 1 and PAR(R) 1 corresponding to the PCR primers. An intron (Intron) is inserted into PAR(F) 1, and the expression vector is designed such that the expression vector itself does not become a template of PCR. After transcription of RNA, in an environment in which splicing is performed in eukaryotic cultured cells or the like, the intron site of the pTREC is removed to join two neighboring PAR(F) 1's. RNA produced from the pTREC can be amplified by RT-PCR. With respect to the intron, the intron site derived from β-globin originally incorporated in the pCI-neo was used.

The pTREC is incorporated with a neomycin-resistant gene (neo) as a control, and by preparing PCR primers corresponding to a part of the sequence in the neomycin-resistant gene and by subjecting the part of the neomycin-resistant gene to RT-PCR, the neomycin-resistant gene can be used as an internal standard control (internal control). PAR(F) 2 and PAR(R) 2 represent the regions corresponding to the PCR primers in the neomycin-resistant gene. Although not shown in the example of Figure 25, an intron may be inserted into at least one of PAR(F) 2 and PAR(R) 2. 2. Effect of primer for detecting target mRNA

(1) Transfection into cultured cells

HeLa cells were seeded at 0.2 to 0.3 x $10^6$ cells per well of a 24-well plate, and after one day, using Lipofectamine 2000 (manufactured by Invitrogen Corp.), 0.5 μg of pTREC vector was transfected according to the manual.

(2) Recovery of cells and quantification of mRNA

One day after the transfection, the cells were recovered and total RNA was extracted with Trizol (manufactured by Invitrogen Corp.). One hundred nanograms of the resulting RNA was reverse transcribed by SuperScript II RT (manufactured by Invitrogen Corp.), using oligo (dT) primers, to synthesize cDNA. A control to which no reverse transcriptase was added was prepared. Using one three hundred and twentieth of the amount of the resulting cDNA as a PCR template, quantitative PCR was carried out in a 50-μl reaction system using SYBR Green PCR Master Mix (manufactured by Applied Biosystems Corp.) to quantify target mRNA (referred to as mRNA (T)) and, as an internal control, mRNA derived from the neomycin-resistant gene in the pTREC (referred to as mRNA (C)). A real-time monitoring apparatus ABI PRIZM7000 (manufactured by Applied Biosystems) was used for the quantitative PCR. A primer pair T (SEQ ID NOs: 2 and 3 in the sequence listing) and a primer pair C (SEQ ID NOs: 4 and 5 in the sequence listing) were used for the quantification of mRNA (T) and mRNA (C), respectively.

Primer pair T:

aggcactgggcaggtgtc (SEQ ID NO: 2)
tgctcgaagcattaaccctcacta (SEQ ID NO: 3)

Primer pair C

atcaggatgatctggacgaag (SEQ ID NO: 4)
ctcttcagcaatatcacgggt (SEQ ID NO: 5)

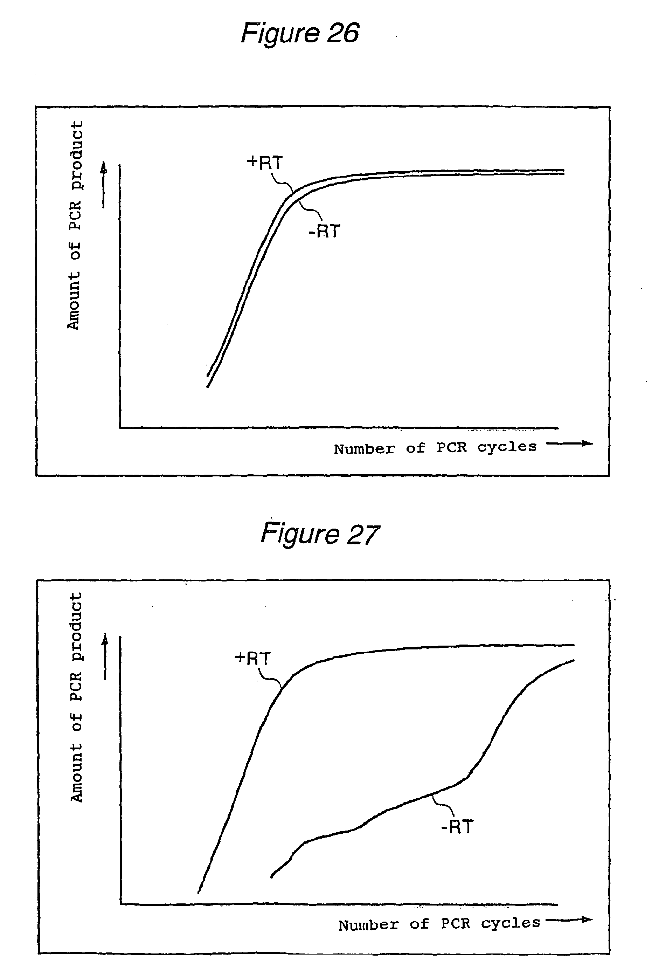

Figures 26 and 27 show the results of PCR. Each of Figures 26 and 27 is a graph in which the PCR product is taken on the axis of ordinate and the number of cycles of PCR is taken on the axis of abscissa. In the neomycin-resistant gene, there is a small difference in the amplification of the PCR product between the case in which cDNA was synthesized by the reverse transcriptase (+RT) and the control case which no reverse transcriptase was added (-RT) (Figure 26). This indicates that not only cDNA but also the vector remaining in the cells also acted as a template and was amplified. On the other hand, in target sequence mRNA, there is a large difference between the case in which the reverse transcriptase was added (+RT) and the case in which no transcriptase was added (-RT) (Figure 27). This result indicates that since one member of the primer pair T is designed so as to sandwich the intron, cDNA derived from intron-free mRNA is efficiently amplified, while the remaining vector having the intron does not easily become a template.

3. Inhibition of expression of target mRNA by siRNA

(1) Cloning of evaluation sequence to target expression vector

Sequences corresponding to the coding regions 812-834 and 35-57 of a human vimentin (VIM) gene (RefSeq ID: NM_003380) were targeted for evaluation. The following synthetic oligonucleotides (evaluation sequence fragments) of SEQ ID NOs: 6 and 7 in the sequence listing were produced, the synthetic oligonucleotides including these sequences and identification sequences for EcoRI and XhoI. Evaluation sequence VIM35 (corresponding to 35-57 of VIM) 5'-gaattcgcaggatgttcggcggcccgggcctcgag-3' (SEQ ID NO: 6)

Evaluation sequence VIM812 (corresponding to 812-834 of VIM)

5'-gaattcacgtacgtcagcaatatgaaagtctcgag-3' (SEQ ID NO: 7)
Using the EcoRI and XhoI sites located on both ends of each of the evaluation sequence fragments, each fragment was cloned as a new target sequence between the EcoRI and XhoI sites of the pTREC, and thereby pTREC-VIM35 and pTREC-VIM812 were constructed.

(2) Production of siRNA

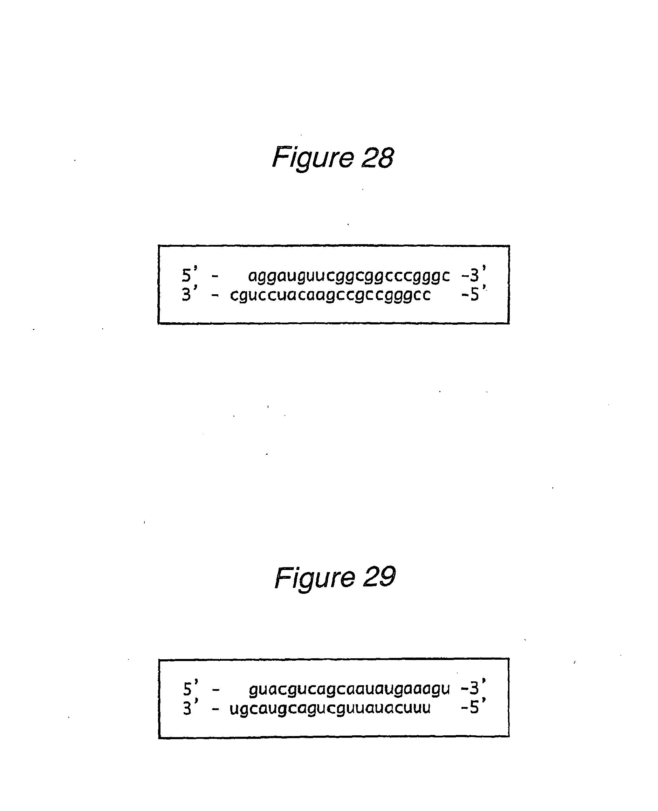

siRNA fragments corresponding to the evaluation sequence VIM35 (SEQ ID NO: 8 in the sequence list, Figure 28), the evaluation sequence VIM812 (SEQ ID NO: 9, Figure 29), and a control sequence (siContorol, SEQ ID NO: 10, Figure 30) were synthesized, followed by annealing. Each of the following siRNA sequences is provided with an overhanging portion on the 3' end.

siVIM35 5'-aggauguucggcggcccgggc-3' (SEQ ID NO: 8)
siVIM812 5'-guacgucagcaauaugaaagu-3' (SEQ ID NO: 9)
As a control, siRNA for the luciferase gene was used.
siControl 5'-cauucuauccgcuggaagaug-3' (SEQ ID NO: 10)

(3) Transfection into cultured cells

HeLa cells were seeded at 0.2 to 0.3 x $10^6$ cells per well of a 24-well plate, and after one day, using Lipofectamine 2000 (manufactured by Invitrogen Corp.), 0.5 $\mu$g of pTREC-VIM35 or pTREC-VIM812, and 100 nM of siRNA corresponding to the sequence derived from each VIM (siVIM35, siVIM812) were simultaneously transfected according to the manual. Into the control cells, 0.5 $\mu$g of pTREC-VIM35 or pTREC-VIM812 and 100 nM of siRNA for the luciferase gene (siControl) were simultaneously transfected.

(4) Recovery of cells and quantification of mRNA

One day after the transfection, the cells were recovered and total RNA was extracted with Trizol (Invitrogen). One hundred nanograms of the resulting RNA was reverse transcribed by SuperScript II RT (manufactured by Invitrogen Corp.), using oligo (dT) primers, to synthesize cDNA. Using one three hundred and twentieth of the amount of the resulting cDNA as a PCR template, quantitative PCR was carried out in a 50-$\mu$l reaction system using SYBR Green PCR Master Mix (manufactured by Applied Biosystems Corp.) to quantify mRNA (referred to as mRNA (T)) including the sequence derived from VIM to be evaluated and, as an internal control, mRNA derived from the neomycin-resistant gene in the pTREC (referred to as mRNA (C)).
A real-time monitoring apparatus ABI PRIZM7000 (manufactured by Applied Biosystems) was used for the quantitative PCR. The primer pair T (SEQ ID NOs: 2 and 3 in the sequence listing) and the primer pair C (SEQ ID NOs: 4 and 5 in the sequence listing) were used for the quantification of mRNA (T) and mRNA (C), respectively. The ratio (T/C) of the resulting values of mRNA was taken on the axis of ordinate (relative amount of target mRNA (%)) in a graph (Figure 31).
In the control cells, since siRNA for the luciferase gene does not affect target mRNA, the ratio T/C is substantially 1. In VIM812 siRNA, the ratio T/C is extremely decreased. The reason for this is that VIM812 siRNA cut mRNA having the corresponding sequence, and it was shown that VIM812 siRNA has the RNAi effect. On the other hand, in VIM35 siRNA, the T/C ratio was substantially the same as that of the control, and thus it was shown that the sequence of VIM35 does not substantially have the RNAi effect.

[Example 3]

1. Inhibition of expression of endogenous vimentin by siRNA

(1) Transfection into cultured cells

HeLa cells were seeded at 0.2 to 0.3 x $10^6$ cells per well of a 24-well plate, and after one day, using Lipofectamine 2000 (manufactured by Invitrogen Corp.), 100 nM of siRNA for VIM (siVIM35 or siVIM812) or control siRNA (siControl) and, as a control for transfection efficiency, 0.5 $\mu$g of pEGFP (manufactured by Clontech) were simultaneously transfected according to the manual. pEGFP is incorporated with EGFP.

(2) Assay of endogenous vimentin mRNA

Three days after the transfection, the cells were recovered and total RNA was extracted with Trizol (manufactured by Invitrogen Corp.). One hundred nanograms of the resulting RNA was reverse transcribed by SuperScript II RT (manufactured by Invitrogen Corp.), using oligo (dT) primers, to synthesize cDNA. PCR was carried out using the cDNA product as a template and using primers for vimentin, VIM-F3-84 and VIM-R3-274 (SEQ ID NOs: 11 and 12).
VIM-F3-84; gagctacgtgactacgtcca (SEQ ID NO: 11)
VIM-R3-274; gttcttgaactcggtgttgat (SEQ ID NO: 12)
Furthermore, as a control, PCR was carried out using β-actin primers ACTB-F2-481 and ACTB-R2-664 (SEQ ID NOs: 13 and 14). The level of expression of vimentin was evaluated under the common quantitative value of β-actin for each sample.
ACTB-F2-481; cacactgtgcccatctacga (SEQ ID NO: 13)
ACTB-R2-664; gccatctcttgctcgaagtc (SEQ ID NO: 14)
The results are shown in Figure 32. In Figure 32, the case in which siControl (i.e., the sequence unrelated to the target) is incorporated is considered as 100% for comparison, and the degree of decrease in mRNA of VIM when siRNA is incorporated into VIM is shown. siVIM-812 was able to effectively inhibit VIM mRNA. In contrast, use of siVIM-35 did not substantially exhibit the RNAi effect.

(3) Antibody staining of cells

Three days after the transfection, the cells were fixed with 3.7% formaldehyde, and blocking was performed in accordance with a conventional method. Subsequently, a rabbit anti-vimentin antibody (α-VIM) or, as an internal control, a rabbit anti-Yes antibody (α-Yes) was added thereto, and reaction was carried out at room temperature. Subsequently, the surfaces of the cells were washed with PBS (Phosphate Buffered Saline), and as a secondary antibody, a fluorescently-labeled anti-rabbit IgG antibody was added thereto. Reaction was carried out at room temperature. After the surfaces of the cells were washed with PBS, observation was performed using a fluorescence microscope.

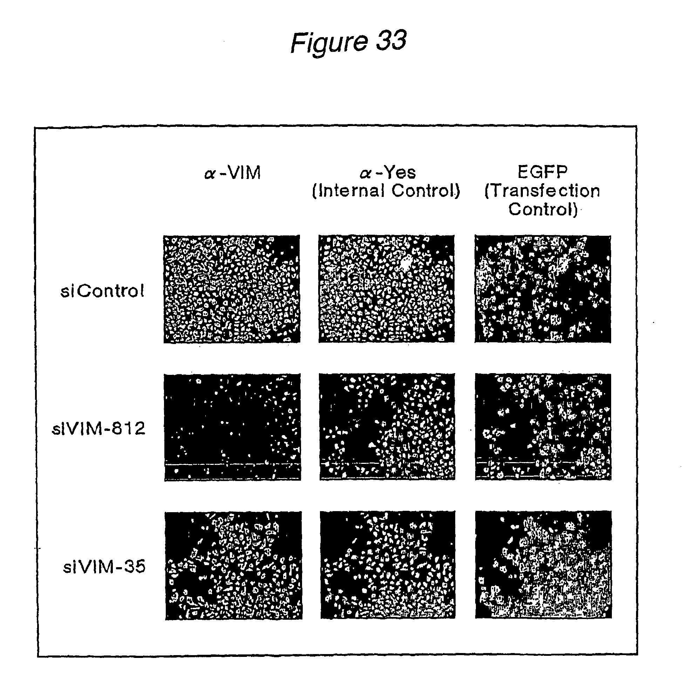

The fluorescence microscope observation results are shown in Figure 33. In the nine frames of Figure 33, the parts appearing white correspond to fluorescent portions. In EGFP and Yes, substantially the same expression was confirmed in all the cells. In the cells into which siControl and siVIM35 were introduced, fluorescence due to antibody staining of vimentin was observed, and the presence of endogenous vimentin was confirmed. On the other hand, in the cells into which siVIM812 was introduced, fluorescence was significantly weaker than that of the cells into which siControl and siVIM35 were introduced. The results show that endogenous vimentin mRNA was interfered by siVIM812, and consequently, the level of expression of vimentin protein was decreased. It has become evident that siVIM812 also has the RNAi effect against endogenous vimentin mRNA.

The results obtained in the assay system of the present invention [Example 2] matched well with the results obtained in the cases in which endogenous genes were actually treated with corresponding siRNA [Example 3]. Consequently, it has been confirmed that the assay system is effective as a method for evaluating the RNAi activity of any siRNA.

[Example 4]

Base sequences were designed based on the above predetermined rules (a) to (d). The base sequences were designed by a base sequence processing apparatus which runs the siRNA sequence design program. As the base sequences, 15 sequences (SEQ ID NOs: 15 to 29) which were expected to have RNAi activity and 5 sequences (SEQ ID NOs: 30 to 34) which were not expected to have RNAi activity were prepared.

RNAi activity was evaluated by measuring the luciferase activity as in Example 1 except that the target sequence and siRNA to be evaluated were prepared based on each of the designed sequences. The results are shown in Figure 34. A low luciferase relative activity value indicates an effective state, i.e., siRNA provided with RNAi activity. All of the siRNA which was expected to have RNAi activity by the program effectively inhibited the expression of luciferase.

[Sequences which exhibited RNAi activity; prescribed sequence portions, excluding overhanging portions]

5,gacgccaaaaacataaaga (SEQ ID NO: 15)
184,gttggcagaagctatgaaa (SEQ ID NO: 16)
272,gtgttgggcgcgttattta (SEQ ID NO: 17)
309,ccgcgaacgacatttataa (SEQ ID NO: 18)
428,ccaatcatccaaaaaatta (SEQ ID NO: 19)
515,cctcccggttttaatgaat (SEQ ID NO: 20)
658,gcatgccagagatcctatt (SEQ ID NO: 21)
695,ccggatactgcgattttaa (SEQ ID NO: 22)
734,ggttttggaatgtttacta (SEQ ID NO: 23)
774,gatttcgagtcgtcttaat (SEQ ID NO: 24)
891,gcactctgattgacaaata (SEQ ID NO: 25)
904,caaatacgatttatctaat (SEQ ID NO: 26)
1186,gattatgtccggttatgta (SEQ ID NO: 27)
1306,ccgcctgaagtctctgatt (SEQ ID NO: 28)
1586,ctcgacgcaagaaaaatca (SEQ ID NO: 29)

[Sequences which did not exhibit RNAi activity; prescribed sequence portions, excluding overhanging portions]

14,aacataaagaaaggcccgg (SEQ ID NO: 30)
265,tatgccggtgttgggcgcg (SEQ ID NO: 31)

295,agttgcagttgcgcccgcg (SEQ ID NO: 32)
411,acgtgcaaaaaaagctccc (SEQ ID NO: 33)
1044,ttctgattacacccgaggg (SEQ ID NO: 34)

[Example 5]

siRNA sequences against SARS virus were designed and examined for their RNAi activity. RNAi activity was evaluated by the same assay as used in Example 2, except that both the target sequence and the sequence to be evaluated were changed.

siRNA sequences were designed on the basis of the genome of SARS virus by using the above siRNA sequence design program, such that the resulting siRNA sequences satisfied a given regularity for 3CL-PRO, RdRp, Spike glycoprotein, Small envelope E protein, Membrane glycoprotein M, Nucleocapsid protein and s2m motif, respectively.

As a result of the assay shown in Figure 35, 11 siRNA sequences designed to satisfy the regularity were found to effectively inhibit RNA into which the respective corresponding siRNA sequences were incorporated as targets. The case in which siControl (the sequence unrelated to SARS) is incorporated is considered as 100%, and the relative amount of target mRNA when each siRNA of SARS is incorporated is shown. In the case of incorporating each siRNA, target RNA was reduced to around 10% or below; each siRNA was confirmed to have RNAi activity.

[Designed siRNA sequences (prescribed sequence portions, excluding overhanging portions)]

siControl; gggcgcggtcggtaaagtt (SEQ ID NO: 35)
3CL-PRO; SARS-10754; ggaattgccgtcttagata (SEQ ID NO: 36)
3CL-PRO; SARS-10810; gaatggtcgtactatcctt (SEQ ID NO: 37)
RdRp; SARS-14841; ccaagtaatcgttaacaat (SEQ ID NO: 38)
Spike glycoprotein; SARS-23341; gcttggcgcatatattcta (SEQ ID NO: 39)
Spike glycoprotein; SARS-24375; cctttcgcgacttgataaa (SEQ ID NO: 40)
Small envelope E protein; SARS-26233; gtgcgtactgctgcaatat (SEQ ID NO: 41)
Small envelope E protein; SARS-26288; ctactcgcgtgttaaaaat (SEQ ID NO: 42)
Membrane glycoprotein M; SARS-26399; gcagacaacggtactatta (SEQ ID NO: 43)
Membrane glycoprotein M; SARS-27024; ccggtagcaacgacaatat (SEQ ID NO: 44)
Nucleocapsid protein; SARS-28685; cgtagtcgcggtaattcaa (SEQ ID NO: 45)
s2m motif; SARS-29606; gatcgagggtacagtgaat (SEQ ID NO: 46)

[Example 6]

According to "<5> siRNA sequence design program" and "<7> Base sequence processing apparatus for running siRNA sequence design program, etc." described above, the following siRNA sequences were designed. Setting conditions for running the program are as shown below.

(Setting conditions)

(a) The 3' end base is adenine, thymine or uracil.
(b) The 5' end base is guanine or cytosine.
(c) In a 7-base sequence from the 3' end, 4 or more bases are one or more types of bases selected from the group consisting of adenine, thymine and uracil.
(d) The number of bases is 19.
(e) A sequence in which 10 or more bases of guanine or cytosine are continuously present is not contained.
(f) A similar sequence containing mismatches of 2 or less bases against the prescribed sequence is not contained in the base sequences of genes other than the target gene among all gene sequences of the target organism.

The designed siRNA sequences are shown in the sequence listing under SEQ ID NOs: 47 to 817081. The name of an organism targeted by each of the siRNA sequences shown in the sequence listing under SEQ ID NOs: 47 to 817081 is shown in (213) of the sequence listing. Likewise, the gene name of each target gene for RNAi, the accession of each target gene, and a prescribed sequence-corresponding portion in the base sequence of each target gene are shown in (223) (Other information) of the sequence listing. It should be noted that gene names and accession information in this context correspond to the "RefSeq" database at NCBI (HYPERLINK "http://www.ncbi.nlm.nih.gov/" http://www.ncbi.nlm.nih.gov/), and information of each gene (including the sequence and function of the gene) can be obtained through access to the RefSeq database.

An example will be given of siRNA shown in SEQ ID NO: 47. The target organism is Homo sapiens, the gene name of the target gene is ATBF1, the accession of the target gene is NM_006885.2, and the portion corresponding to the prescribed sequence is composed of 19 bases between bases 908 and 926 in the base sequence of NM_006885.2. Upon access to the RefSeq database, the target gene will be found to be a gene related to AT-binding transcription factor 1.

[Example 7]

To examine influences on other genes containing sequences with a small number of mismatches to siRNA, the same procedure as used in Example 5 was repeated to design siRNA against firefly luciferase, and the resulting siRNA was examined for its RNAi effect on the similar sequences with a small number of mismatches.

[Designed siRNA sequence (prescribed sequence portion, including overhanging portions of 2 bases)]

3-36 gccattctatccgctggaagatg (SEQ ID NO: 817082)

[Sequences similar to designed siRNA (bases indicated in uppercase letters represent mismatch sites)]

3-36.R1 gccattctatccgcGggCGgatg (SEQ ID NO: 817083)
3-36.R2 gccattctatccgcCggGGgatg (SEQ ID NO: 817084)
3-36.R3 gccattctatccgcGggaCgatg (SEQ ID NO: 817085)
3-36.R4 gccattctatccgctggCGgatg (SEQ ID NO: 817086)
3-36.R5 gccattctatccgctggaGgatg (SEQ ID NO: 817087)
3-36.R6 gccattctatccgctgTaaTatg (SEQ ID NO: 817088)
3-36.R7 gccattctatccgctAAaagatg (SEQ ID NO: 817089)
3-36.R8 gccattctatccgctATaaAatg (SEQ ID NO: 817090)
3-36.L1 gccGGCcCGtccgctggaagatg (SEQ ID NO: 817091)
3-36.L2 gccCGtcCGtccgctggaagatg (SEQ ID NO: 817092)
3-36.L3 gccGtCctGtccgctggaagatg (SEQ ID NO: 817093)
3-36.L4 gccaCCcGatccgctggaagatg (SEQ ID NO: 817094)
3-36.L5 gccattAtatccgctggaagatg (SEQ ID NO: 817095)
3-36.01A gcAattctatccgctggaagatg (SEQ ID NO: 817096)
3-36.01G gcGattctatccgctggaagatg (SEQ ID NO: 817097)
3-36.01U gcTattctatccgctggaagatg (SEQ ID NO: 817098)
3-36.19G gccattctatccgctggaagGtg (SEQ ID NO: 817099)
3-36.19C gccattctatccgctggaagCtg (SEQ ID NO: 817100)
3-36.19U gccattctatccgctggaagTtg (SEQ ID NO: 817101)

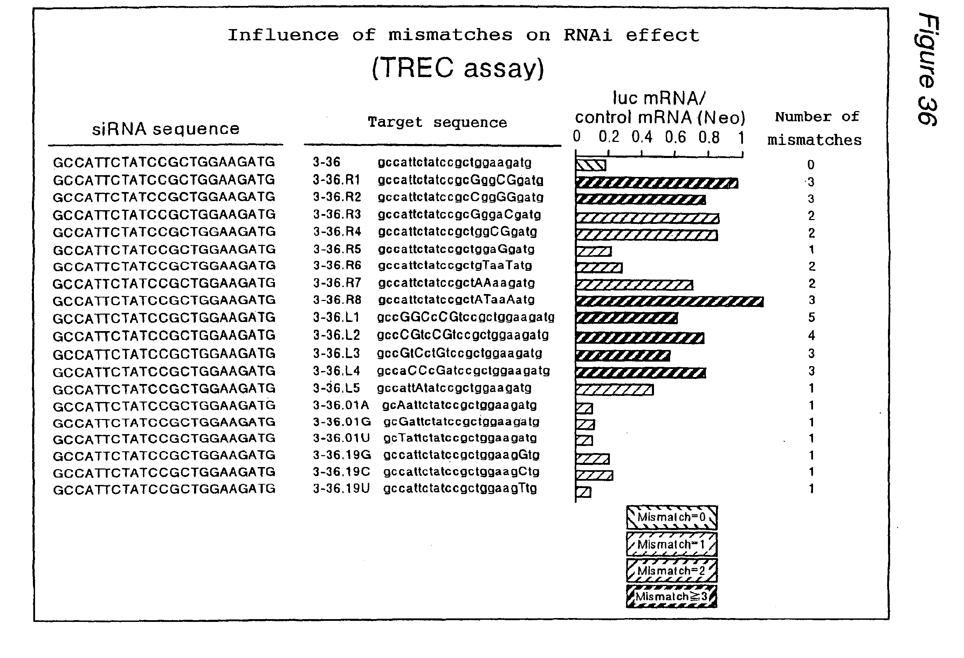

As a result of the assay shown in Figure 36, in the case of designing base sequences of 19 bases, when genes other than the target gene contain similar sequences with mismatches of 2 or less bases, these similar sequence portions were confirmed to have a high probability of being targeted by RNA interference.

[Example 8]

In this example, the siRNA sequences used were composed of 21-base sense strand RNA having the base sequences shown in Tables 1A to 1K (whose base sequences are shown in the column "siRNA-sense" of Table 1) and 21-base antisense strand RNA having the base sequences shown in Tables 1A to 1K (whose base sequences are shown in the column "siRNA-antisense" of Table 1, provided that the base sequences are shown in the direction from 3' to 5'). As shown in Table 1, each siRNA was appropriately designed on the basis of each target sequence (see the column "Target Sequence") located at a given position (see the column "Target Position") in the coding region of each gene to be targeted by RNAi (see the column "Gene Name"; hereinafter also referred to as a target gene), particularly on the basis of the so-called prescribed sequence corresponding to a portion covering the third base from the 5' end to the third base from the 3' end of each target sequence. Each siRNA was then examined for its RNAi effect using human-derived HeLa cells. More specifically, even-numbered base sequences among SEQ ID NOs: 817102 to 817650 were examined as sense strands (siRNA-sense), while odd-numbered base sequences among SEQ ID NOs: 817102 to 817650 were examined as antisense strands (siRNA-antisense). Detailed procedures used in this example will be explained below.

1. Synthesis of siRNA

Double-stranded siRNA composed of sense and antisense strands was suitably designed according to the above rules of the present invention (the rules (a) to (d) described in [1], etc.) on the basis of the above prescribed sequence of each target gene. Based upon such design, the synthesis was entrusted to Proligo Japan for preparation. As to detailed synthetic procedures used here, sense and antisense strands having given base sequences as shown in the table were heated in a reaction liquid of 10 mM Tris-HCl (pH 7.5) and 20 mM NaCl at 90°C for 3 minutes. Both strands were further incubated at 37°C for 1 hour and then associated by standing until room temperature to form double-stranded siRNA. The double-stranded siRNA thus formed was subjected to electrophoresis using a 2% agarose gel in TBE buffer so as to confirm the association between sense and antisense strands.

2. Cell cultivation

In this example, human-derived HeLa cells were used. The medium used for culturing HeLa cells (hereinafter also referred to as cell medium) was Dulbecco's Modified Eagle's medium (DMEM; manufactured by Invitrogen Corp.) which was supplemented with inactivated 10% fetal bovine serum (FBS; manufactured by Biomedicals, inc). In this medium, HeLa cells were cultured at 37°C in the presence of 5% $CO_2$.

3. Target gene to be targeted by RNAi

Since HeLa cells which are uterine cervical cancer cells are used in this example, the individual genes shown in Table 1 which are endogenous genes in the HeLa cells and are highly expressed in these cells are targets for RNAi by siRNA, i.e., target genes for RNAi. In this example, HeLa cells were used to examine the RNAi effect of each siRNA on these genes, thereby studying the effect of siRNA on diseases and/or biological functions related to these genes (more specifically, see the columns "Related Disease", "Biological Function Category" and/or "Reported Biological Function" of Figure 46), i.e., a prophylactic/therapeutic effect on the diseases and/or a controlling effect on the biological functions. As an internal control gene, the endogenous GAPDH gene in HeLa cells was used in this study.

4. Introduction (transfection) of siRNA into cells

HeLa cells were first seeded at a density of $5 \times 10^4$ cells/well into a 24-well plate and cultured for 24 hours under the cell culture conditions described above, followed by introducing 5 nM/well of siRNA. After the introduction, the HeLa cells were cultured at 37°C for 24 hours. In this introduction process, Lipofectamine 2000 (manufactured by Invitrogen Corp.) was used as an introducing reagent, while DMEM was used as a medium for introduction. As to detailed procedures for introduction, Opti-MEM medium (manufactured by Invitrogen Corp.) containing Lipofectamine 2000 and siRNA was added to the cell medium, followed by culturing the HeLa cells to introduce siRNA into the cells. The HeLa cells thus introduced with siRNA are hereinafter referred to as an "evaluation sample."

On the other hand, for correction of the level of target gene-derived mRNA in PCR described later, the following calibrator sample was prepared. The calibrator sample was prepared by the same treatment as used for the evaluation sample introduced with siRNA, except that Opti-MEM medium containing Lipofectamine 2000 but free from siRNA was added to the above cell medium to culture HeLa cells.

5. Measurement of RNAi effect

After the above introduction was performed, HeLa cells were recovered for both evaluation and calibrator samples described above. The recovered cells were then provided for an ABI PRISM® 6700 Automated Nucleic Acid Workstation (manufactured by Applied Biosystems Corp.), and this apparatus was operated according to the manual to perform RNA extraction and cDNA synthesis by reverse transcription.

Subsequently, the resulting cDNA was used as a template to perform quantitative PCR in a 50-μl reaction system using SYBR Green PCR Master Mix (manufactured by Applied Biosystems Corp.). In this quantitative PCR, an ABI PRISM® 7900HT Sequence Detection System was used as a real-time monitoring apparatus and operated according to the manual. In addition, the PCR primers used were optimal primers obtained as a result of various studies.

In this example, the results obtained from PCR quantification were analyzed by a method called the "comparative Ct method." With respect to this method, a detailed explanation is omitted here because an explanation of this method is disclosed in the home page of Applied Biosystems Corp. (http://www.appliedbiosystems.co.jp). The outline of this method is as follows: this method allows relative quantification by focusing on what number of cycles an evaluation sample reaches faster (or later) the Threshold Line, as compared to the calibrator sample.

More specifically, both evaluation and calibrator samples were first quantified by PCR to determine Ct1 that corresponds to a relative mRNA level including a target gene-derived base sequence(s) and Ct2 that corresponds to a relative mRNA level including an internal control gene-derived base sequence(s). In the following descriptions, the above Ct1 and Ct2 of an evaluation sample are referred to as "Ct1(E)" and "Ct2(E)," respectively. Likewise, the above Ct1 and Ct2 of the calibrator sample are referred to as "Ct1(C)" and "Ct2(C)," respectively.

As used herein, "Ct" denotes the number of cycles required before reaching the Threshold Line, and more specifically is defined by the following Equation (1). It should be noted that the amplification efficiency is set to 1 in this case. With respect to the numeric characters following Ct, "1" means a mRNA level derived from a target gene and "2" means a mRNA level derived from the internal control gene. With respect to the designations (E) and (C) following Ct, "E" means an evaluation sample and "C" means the calibration sample. Regardless of the designations "1", "2", "E" and "C", "Ct" is defined as follows:

$$\mathtt{Ct\ =\ (log[DNA]t - log[DNA]0)/log2} \qquad \mathtt{(1)}$$

wherein [DNA]t represents the amount of DNA at the time of reaching the Threshold Line, and [DNA]0 represents the initial amount of cDNA reverse-transcribed from mRNA.

Ct1(E), Ct2(E), Ct1(C) and Ct2(C) thus obtained by PCR quantification were subjected to and analyzed by the comparative Ct method to obtain a RQ value used for evaluating the RNAi effect of siRNA. The RQ value is a relative mRNA level of a target gene in an evaluation sample when the mRNA level of the target gene in the calibration sample is set to 1. More specifically, the RQ value is defined by the following Equation (2):

$$\mathtt{RQ\ =\ 2^{(-\Delta\Delta Ct)}} \qquad \mathtt{(2)}$$

wherein ΔΔCt is defined by the following Equation (3):

$$\mathtt{\Delta\Delta Ct\ =\ \Delta Ct(E) - \Delta Ct(C)} \qquad \mathtt{(3)}$$

wherein ΔⳆCt(E) is defined by the following Equation (4) and ΔCt(C) is defined by the following Equation (5):

$$\mathtt{\Delta Ct(E)\ =\ Ct1(E) - Ct2(E)} \qquad \mathtt{(4)}$$

$$\mathtt{\Delta Ct(C)\ =\ Ct1(C) - Ct2(C)} \qquad \mathtt{(5).}$$

It should be noted that the designations "1", "2", "E" and "C" in Equations (2) to (5) are as defined above.

6. Evaluation of RNAi effect

The RQ values thus obtained are shown in Tables 1A to 1K. In Table 1, the data in the columns "Gene Name" and "refseq_NO.", portions actually targeted by RNAi within the sequences listed in the column "Target Sequence" and the definition of "Target Position" are as described above for Figure 46 in the section "BRIEF DESCRIPTION OF THE DRAWINGS" of this specification.

In this example, on the basis of the RQ values thus calculated (see the column "RQ value" of Table 1), each siRNA was evaluated for RNAi effect on its target gene. As is evident from the table, siRNA sequences composed of sense strands having even-numbered base sequences among SEQ ID NOs: 817102 to 817650 and antisense strands having odd-numbered base sequences among SEQ ID NOs: 817102 to 817650 were all found to have a RQ value less than 1 and almost all found to have a RQ value less than 0.5, thus indicating that these siRNA sequences caused a 50% or more inhibition of the expression of the target genes shown in Table 1. Such an RNAi effect of each siRNA was also achieved when repeating the same procedure as shown above with COS cells.

Moreover, in light of the results from Example 8 showing that all the 294 tested siRNA sequences falling within the present invention were found to produce an RNAi effect, it was indicated that the polynucleotides (siRNA) of the present invention effectively produced an RNAi effect against their target genes in mammalian cells and caused a 50% or more inhibition of gene expression.

In Examples 1 to 8, the cases using siRNA sequences whose sense and antisense strands are each composed of RNA were shown. The same results as in Examples 1 to 8 are also obtained in the case of using siRNA having a chimeric structure. Although the detailed explanation for the case of siRNA having a chimeric structure is omitted here, for example, when siRNA having a chimeric structure is used in Example 8, this siRNA structurally differs in the following point from the siRNA sequences of Example 8 which are composed of sense and antisense strands shown under SEQ ID NOs: 817102 to 817651.

Namely, siRNA sequences of chimeric structure have the same base sequences as siRNA sequences composed of sense and antisense strands shown in Table 1 under SEQ ID NOs: 817102 to 817651. However, a portion of 8 to 12 nucleotides (e.g., 10 nucleotides, preferably 11 nucleotides, more preferably 12 nucleotides) from the 3' end of the sense strand (for example, "A" in the case of the sense strand shown in Table 1 under SEQ ID NO: 8102) and a portion of 8 to 12 nucleotides (e.g., 10 nucleotides, preferably 11 nucleotides, more preferably 12 nucleotides) from the 5' end of the antisense strand (for example, "A" in the case of the antisense strand shown in Table 1 under SEQ ID NO: 8103) are both composed of DNA. Thus, siRNA sequences of chimeric structure differ from the siRNA sequences shown under SEQ ID NOs: 817102 to 817651 in that U in the above polynucleotide portions is replaced by T within the base sequences of the sense and antisense strands shown in Table 1.

Table 1A

| Gene Name | refseq_ID | RQ | Target Position | Target sequence | SEQ ID NO | siRNA-sense | SEQ ID NO | siRNA-antisense | SEQ ID NO |
|---|---|---|---|---|---|---|---|---|---|
| PSEN1 | NM_000021.2 | 0.23 | 532 | tggtcgtggctaccattaagtca | 307985 | GUCGUGGCUACCAUUAAGUCA | 817102 | ACUUAAUGGUAGCCACGACCA | 817103 |
| JAG1 | NM_000214.1 | 0.28 | 794 | ctggccgaggtcctatacgttgc | 147021 | GGCCGAGGUCCUAUACGUUGC | 817104 | AACGUAUAGGACCUCGGCCAG | 817105 |
| POLR2A | NM_000937.2 | 0.34 | 2425 | tcctcatcgagggtcatactatt | 36223 | CUCAUCGAGGGUCAUACUAUU | 817106 | UAGUAUGACCCUCGAUGAGGA | 817107 |
| CDC6 | NM_001254.3 | 0.35 | 383 | gtctgggcgatgacaacctatgc | 76037 | CUGGGCGAUGACAACCUAUGC | 817108 | AUAGGUUGUCAUCGCCCAGAC | 817109 |
| CSE1L | NM_001316.2 | 0.15 | 393 | gccgatcgagtggccattaaagc | 128329 | CGAUCGAGUGGCCAUUAAAGC | 817110 | UUUAAUGGCCACUCGAUCGGC | 817111 |
| HDAC2 | NM_001527.1 | 0.15 | 1110 | tggctacacaatccgtaatgttg | 4714 | GCUACACAAUCCGUAAUGUUG | 817112 | ACAUUACGGAUUGUGUAGCCA | 817113 |
| HIF1A | NM_001530.2 | 0.2 | 809 | aactagccgaggaagaactatga | 237 | CUAGCCGAGGAAGAACUAUGA | 817114 | AUAGUUCUUCCUCGGCUAGUU | 817115 |
| IGFBP4 | NM_001552.1 | 0.064 | 706 | aagcacttcgccaaaattcgaga | 124916 | GCACUUCGCCAAAAUUCGAGA | 817116 | UCGAAUUUUGGCGAAGUGCUU | 817117 |
| CDC2 | NM_001786.2 | 0.18 | 656 | tggggtcagctcgttactcaact | 75723 | GGGUCAGCUCGUUACUCAACU | 817118 | UUGAGUAACGAGCUGACCCCA | 817119 |
| CDK2 | NM_001798.2 | 0.21 | 689 | tggagtccctgttcgtacttaca | 76134 | GAGUCCCUGUUCGUACUUACA | 817120 | UAAGUACGAACAGGGACUCCA | 817121 |
| CDK7 | NM_001799.2 | 0.3 | 575 | gggagccccaatagagcttatac | 76204 | GAGCCCCAAUAGAGCUUAUAC | 817122 | AUAAGCUCUAUUGGGGCUCCC | 817123 |
| CUTL1 | NM_001913.2 | 0.36 | 139 | gtccagaaagcggcttatcgaac | 2624 | CCAGAAAGCGGCUUAUCGAAC | 817124 | UCGAUAAGCCGCUUUCUGGAC | 817125 |
| E2F4 | NM_001950.3 | 0.2 | 1220 | cccgggagaccacgattatatct | 2910 | CGGGAGACCACGAUUAUAUCU | 817126 | AUAUAAUCGUGGUCUCCCGGG | 817127 |
| GNB1 | NM_002074.2 | 0.072 | 672 | tgcggtggcctggataacatttg | 192277 | CGGUGGCCUGGAUAACAUUUG | 817128 | AAUGUUAUCCAGGCCACCGCA | 817129 |
| HSPA4 | NM_002154.3 | 0.055 | 578 | aggtataaaggtgacatatatgg | 85169 | GUAUAAAGGUGACAUAUAUGG | 817130 | AUAUAUGUCACCUUUAUACCU | 817131 |
| KPNA1 | NM_002264.1 | 0.2 | 520 | ttctcttcagacccgaattgtga | 133261 | CUCUUCAGACCCGAAUUGUGA | 817132 | ACAAUUCGGGUCUGAAGAGAA | 817133 |
| KPNA3 | NM_002267.2 | 0.074 | 1921 | aggaggtacctacaattttgatc | 177084 | GAGGUACCUACAAUUUUGAUC | 817134 | UCAAAAUUGUAGGUACCUCCU | 817135 |
| KPNA4 | NM_002268.3 | 0.094 | 1595 | tagtactcgatggactaagtaat | 269352 | GUACUCGAUGGACUAAGUAAU | 817136 | UACUUAGUCCAUCGAGUACUA | 817137 |
| PAWR | NM_002583.2 | 0.21 | 984 | gtgggttccctagatataacagg | 113162 | GGGUUCCCUAGAUAUAACAGG | 817138 | UGUUAUAUCUAGGGAACCCAC | 817139 |
| POLD1 | NM_002691.1 | 0.29 | 2216 | ggggttcggacgtcagatgatcg | 35766 | GGUUCGGACGUCAGAUGAUCG | 817140 | AUCAUCUGACGUCCGAACCCC | 817141 |
| POLR2G | NM_002696.1 | 0.11 | 586 | tggctccctgatggacgattact | 53520 | GCUCCCUGAUGGACGAUUACU | 817142 | UAAUCGUCCAUCAGGGAGCCA | 817143 |
| PRKACB | NM_002731.1 | 0.1 | 944 | cacgacagattggattgctattt | 96985 | CGACAGAUUGGAUUGCUAUUU | 817144 | AUAGCAAUCCAAUCUGUCGUG | 817145 |
| PRKCA | NM_002737.2 | 0.19 | 429 | ctgcgatatgaacgttcacaagc | 97011 | GCGAUAUGAACGUUCACAAGC | 817146 | UUGUGAACGUUCAUAUCGCAG | 817147 |
| MAPK1 | NM_002745.2 | 0.086 | 383 | aagttcgagtagctatcaagaaa | 89815 | GUUCGAGUAGCUAUCAAGAAA | 817148 | UCUUGAUAGCUACUCGAACUU | 817149 |
| MAPK9 | NM_002752.3 | 0.22 | 261 | atcgtgaacttgtcctcttaaaa | 90069 | CGUGAACUUGUCCUCUUAAAA | 817150 | UUAAGAGGACAAGUUCACGAU | 817151 |
| MAP2K1 | NM_002755.2 | 0.21 | 114 | ccccgacggctctgcagttaacg | 88938 | CCGACGGCUCUGCAGUUAACG | 817152 | UUAACUGCAGAGCCGUCGGGG | 817153 |

Table 1B

| Gane Name | refseq_ID | RQ | Target Position | Target sequence | SEQ ID NO | siRNA-sense | SEQ ID NO | siRNA-antisense | SEQ ID NO |
|---|---|---|---|---|---|---|---|---|---|
| PSMA2 | NM_002787.3 | 0.022 | 56 | ttcagcccgtctggtaaacttgt | 185145 | CAGCCCGUCUGGUAAACUUGU | 817154 | AAGUUUACCAGACGGGCUGAA | 817155 |
| PSMA3 | NM_002788.2 | 0.074 | 599 | atgacctgccgtgatatcgttaa | 185178 | GACCUGCCGUGAUAUCGUUAA | 817156 | AACGAUAUCACGGCAGGUCAU | 817157 |
| PSMA4 | NM_002789.3 | 0.1 | 183 | gtcgcttataccaagttgaatat | 185191 | CGCUUAUACCAAGUUGAAUAU | 817158 | AUUCAACUUGGUAUAAGCGAC | 817159 |
| PSMA5 | NM_002790.2 | 0.39 | 107 | tacgacaggggcgtgaatacttt | 185211 | CGACAGGGGCGUGAAUACUUU | 817160 | AGUAUUCACGCCCCUGUCGUA | 817161 |
| PSMA6 | NM_002791.1 | 0.07 | 129 | ccggttttgaccgccacattacc | 53630 | GGUUUUGACCGCCACAUUACC | 817162 | UAAUGUGGCGGUCAAAACCGG | 817163 |
| PSMA7 | NM_002792.2 | 0.13 | 346 | cgccgatgcaaggatagtcatca | 185235 | CCGAUGCAAGGAUAGUCAUCA | 817164 | AUGACUAUCCUUGCAUCGGCG | 817165 |
| PSMB1 | NM_002793.2 | 0.035 | 130 | tgcgattttcgccctacgttttc | 185241 | CGAUUUUCGCCCUACGUUUUC | 817166 | AAACGUAGGGCGAAAAUCGCA | 817167 |
| PSMB2 | NM_002794.3 | 0.66 | 530 | cagtatcctcgaccgatactaca | 185279 | GUAUCCUCGACCGAUACUACA | 817168 | UAGUAUCGGUCGAGGAUACUG | 817169 |
| PSMB3 | NM_002795.2 | 0.063 | 312 | aggtcggcagatcaaaccttata | 185290 | GUCGGCAGAUCAAACCUUAUA | 817170 | UAAGGUUUGAUCUGCCGACCU | 817171 |
| PSMB4 | NM_002796.2 | 0.052 | 317 | ctctggcgactacgctgatttcc | 185302 | CUGGCGACUACGCUGAUUUCC | 817172 | AAAUCAGCGUAGUCGCCAGAG | 817173 |
| PSMB6 | NM_002798.1 | 0.067 | 683 | gggtagagcggcaagtacttttg | 185333 | GUAGAGCGGCAAGUACUUUG | 817174 | AAAGUACUUGCCGCUCUACCC | 817175 |
| PSMC3 | NM_002804.3 | 0.089 | 1197 | ccgccttgaccgcaagatagagt | 98373 | GCCUUGACCGCAAGAUAGAGU | 817176 | UCUAUCUUGCGGUCAAGGCGG | 817177 |
| PSMD7 | NM_002811.3 | 0.11 | 477 | ctgtcctaattccgtattggtca | 57203 | GUCCUAAUUCCGUAUUGGUCA | 817178 | ACCAAUACGGAAUUAGGACAG | 817179 |
| RAF1 | NM_002880.2 | 0.41 | 897 | gtcgacatccacacctaatgtcc | 98850 | CGACAUCCACACCUAAUGUCC | 817180 | ACAUUAGGUGUGGAUGUCGAC | 817181 |
| SHC1 | NM_003029.3 | 0.12 | 601 | gccgagtatgtcgcctatgttgc | 253033 | CGAGUAUGUCGCCUAUGUUGC | 817182 | AACAUAGGCGACAUACUCGGC | 817183 |
| SP3 | NM_003111.1 | 0.4 | 2324 | agctgcgcgagatgatactttga | 40777 | CUGCGCGAGAUGAUACUUUGA | 817184 | AAAGUAUCAUCUCGCGCAGCU | 817185 |
| TCF7 | NM_003202.1 | 0.23 | 92 | accgtctactccgccttcaatct | 41852 | CGUCUACUCCGCCUUCAAUCU | 817186 | AUUGAAGGCGGAGUAGACGGU | 817187 |
| TEAD4 | NM_003213.1 | 0.16 | 1386 | tgctgtgcattgcctatgtcttt | 13093 | CUGUGCAUUGCCUAUGUCUUU | 817188 | AGACAUAGGCAAUGCACAGCA | 817189 |
| TMPO | NM_003276.1 | 0.14 | 1609 | ctcactaccttaggtctagaagt | 127873 | CACUACCUUAGGUCUAGAAGU | 817190 | UUCUAGACCUAAGGUAGUGAG | 817191 |
| YWHAB | NM_003404.3 | 0.072 | 769 | cagcctacacacccaattcgtct | 126595 | GCCUACACACCCAAUUCGUCU | 817192 | ACGAAUUGGGUGUGUAGGCUG | 817193 |
| YWHAH | NM_003405.2 | 0.094 | 824 | cacactaaacgaggattcctata | 126608 | CACUAAACGAGGAUUCCUAUA | 817194 | UAGGAAUCCUCGUUUAGUGUG | 817195 |
| OGT | NM_003605.3 | 0.16 | 449 | ctggcagaagcttattcgaattt | 134296 | GGCAGAAGCUUAUUCGAAUUU | 817196 | AUUCGAAUAAGCUUCUGCCAG | 817197 |
| PPP2CB | NM_004156.1 | 0.23 | 801 | cagtgcacccaattactgttatc | 162283 | GUGCACCCAAUUACUGUUAUC | 817198 | UAACAGUAAUUGGGUGCACUG | 817199 |
| SCYE1 | NM_004757.2 | 0.11 | 305 | ctgcacgctaattctatggtttc | 49202 | GCACGCUAAUUCUAUGGUUUC | 817200 | AACCAUAGAAUUAGCGUGCAG | 817201 |
| HDAC1 | NM_004964.2 | 0.15 | 870 | tcggttaggttgcttcaatctaa | 4672 | GGUUAGGUUGCUUCAAUCUAA | 817202 | AGAUUGAAGCAACCUAACCGA | 817203 |
| PSMD5 | NM_005047.2 | 0.15 | 1476 | gtgaagggccatactatgtgaaa | 323491 | GAAGGGCCAUACUAUGUGAAA | 817204 | UCACAUAGUAUGGCCCUUCAC | 817205 |

EP 1 752 536 A1

Table 1C

| Gene Name | refseq_ID | RQ | Target Position | Target sequence | SEQ ID NO | siRNA-sense | SEQ ID NO | siRNA-antisense | SEQ ID NO |
|---|---|---|---|---|---|---|---|---|---|
| CEBPB | NM_005194.2 | 0.31 | 1001 | agcacagcgacgagtacaagatc | 2153 | CACAGGCGACGAGUACAAGAUC | 817206 | UCUUGUACUCGUCGCUGUGCU | 817207 |
| EGFR | NM_005228.2 | 0.27 | 2387 | cccgtcgctatcaaggaattaag | 81171 | CGUCGCUAUCAAGGAAUUAAG | 817208 | UAAUUCCUUGAUAGCGACGGG | 817209 |
| ELK1 | NM_005229.2 | 0.22 | 419 | ggccttgcggtactactatgaca | 3142 | CCUUGCGGUACUACUAUGACA | 817210 | UCAUAGUAGUACCGCAAGGCC | 817211 |
| EWSR1 | NM_005243.1 | 0.22 | 631 | ctctacacagccgactagttatg | 51496 | CUACACAGCCGACUAGUUAUG | 817212 | UAACUAGUCGGCUGUGUAGAG | 817213 |
| HCFC1 | NM_005334.1 | 0.22 | 5339 | gggcaccgtcctgactataacc | 4624 | GCACCGUCCCUGACUAUAACC | 817214 | UUAUAGUCAGGGACGGUGCCC | 817215 |
| JUND | NM_005354.2 | 0.22 | 1053 | ctcgcgcctggaagagaaagtga | 5612 | CGCGCCUGGAAGAGAAAGUGA | 817216 | ACUUUCUCUUCCAGGCGCGAG | 817217 |
| YES1 | NM_005433.3 | 0.1 | 839 | ttgcgactagaggtaaactagg | 107693 | GCCACUAGAGGUAAACUAGG | 817218 | UAGUUUAACCUCUAGUCGCAA | 817219 |
| TAF6 | NM_005641.2 | 0.13 | 748 | ttgactacgccttgaagctaaag | 41513 | GACUACGCCUUGAAGCUAAAG | 817220 | UUAGCUUCAAGGCGUAGUCAA | 817221 |
| TAF7 | NM_005642.2 | 0.39 | 1133 | ctggaaccacggaattactctgc | 112459 | GGAACCACGGAAUUACUCUGC | 817222 | AGAGUAAUUCCGUGGUUCCAG | 817223 |
| PRKCN | NM_005813.2 | 0.25 | 3090 | aactcgcattggagaacgttaca | 97488 | CUCGCAUUGGAGAACGUUACA | 817224 | UAACGUUCUCCAAUGCGAGUU | 817225 |
| PA2G4 | NM_006191.1 | 0.08 | 752 | gaggtacatgaagtatatgctgt | 186582 | GGUACAUGAAGUAUAUGCUGU | 817226 | AGCAUAUACUUCAUGUACCUC | 817227 |
| TAF10 | NM_006284.2 | 0.13 | 461 | gcctcagacccacgcataattcg | 12455 | CUCAGACCCACGCAUAAUUCG | 817228 | AAUUAUGCGUGGGUCUGAGGC | 817229 |
| COPS5 | NM_006837.2 | 0.22 | 726 | atgcaatcgggtggtatcatagc | 56199 | GCAAUCGGGUGGUAUCAUAGC | 817230 | UAUGAUACCACCCGAUUGCAU | 817231 |
| STAT1 | NM_007315.2 | 0.21 | 2177 | aaggggccatcacattcacatgg | 12048 | GGGGCCAUCACAUUCACAUGG | 817232 | AUGUGAAUGUGAUGGCCCCUU | 817233 |
| GALNT1 | NM_020474.2 | 0.092 | 1203 | tagattatggagatatatcgtca | 161846 | GAUUAUGGAGAUAUAUCGUCA | 817234 | ACGAUAUAUCUCCAUAAUCUA | 817235 |
| CDKN2A | NM_000077.3 | 0.16 | 677 | ggcaccagaggcagtaaccatgc | 219272 | CACCAGAGGCAGUAACCAUGC | 817236 | AUGGUUACUGCCUCUGGUGCC | 817237 |
| RB1 | NM_000321.1 | 0.094 | 2701 | agcgaccgtgtgctcaaaagaag | 10143 | CGACCGUGUGCUCAAAAGAAG | 817238 | UCUUUUGAGCACACGGUCGCU | 817239 |
| CD44 | NM_000610.2 | 0.16 | 233 | ctggcgcgagatcgattttgaatat | 126722 | GGGCCAGAUCGAUUUGAAUAU | 817240 | AUUCAAAUCGAUCGCUGGCCC | 817241 |
| COMT | NM_000754.2 | 0.093 | 922 | gtgcacacactaccaatcgttcc | 165318 | GCACACACUACCAAUCGUUCC | 817242 | AACGAUUGGUAGUGUGUGCAC | 817243 |
| GSTP1 | NM_000852.2 | 0.14 | 624 | tacgtgaacctccccatcaatgg | 216683 | CGUGAACCUCCCCAUCAAUGG | 817244 | AUUGAUGGGGAGGUUCACGUA | 817245 |
| IGF1R | NM_000875.2 | 0.27 | 279 | cacggtcattaccgagtacttgc | 85645 | CGGUCAUUACCGAGUACUUGC | 817246 | AAGUACUCGGUAAUGACCGUG | 817247 |
| ARHA | NM_001664.2 | 0.098 | 371 | tacccagataccgatgttatact | 108327 | CCCAGAUACCGAUGUUAUACU | 817248 | UAUAACAUCGGUAUCUGGGUA | 817249 |
| CTSC | NM_001814.2 | 0.29 | 236 | cggttccagcgcgatgtcaact | 188060 | GUUCCAGCGCGAUGUCAACU | 817250 | UUGACAUCGCGCUGGAACCG | 817251 |
| FN1 | NM_002026.1 | 0.13 | 473 | acctaggcaatgcgttggtttgt | 126771 | CUAGGCAAUGCGUUGGUUUGU | 817252 | AAACCAACGCAUUGCCUAGGU | 817253 |
| LGALS1 | NM_002305.2 | 0.047 | 367 | agctgccagatggatacgaattc | 174842 | CUGCCAGAUGGAUACGAAUUC | 817254 | AUUCGUAUCCAUCUGGCAGCU | 817255 |
| NRAS | NM_002524.2 | 0.11 | 445 | cagtgccatgagagaccaataca | 109675 | GUGCCAUGAGAGACCAAUACA | 817256 | UAUUGGUCUCUCAUGGCACUG | 817257 |

Table 1D

| Gane Name | refseq_ID | RQ | Target Position | Target sequence | SEQ ID NO | siRNA-sense | SEQ ID NO | siRNA-antisense | SEQ ID NO |
|---|---|---|---|---|---|---|---|---|---|
| PCNA | NM_002592.2 | 0.087 | 526 | cggataccttggcgctagtattt | 34625 | GAUACCUUGGCGCUAGUAUUU | 817258 | AUACUAGCGCCAAGGUAUCCG | 817259 |
| PKM2 | NM_002654.3 | 0.08 | 565 | tgctgtggctctagacactaaag | 166519 | CUGUGGCUCUAGACACUAAAG | 817260 | UUAGUGUCUAGAGCCACAGCA | 817261 |
| RXRA | NM_002957.3 | 0.47 | 1342 | tgcgctccatcgggctcaaatgc | 10866 | CGCUCCAUCGGGCUCAAAUGC | 817262 | AUUUGAGCCCGAUGGAGCGCA | 817263 |
| S100A4 | NM_002961.2 | 0.12 | 152 | agctcaacaagtcagaactaaag | 152374 | CUCAACAAGUCAGAACUAAAG | 817264 | UUAGUUCUGACUUGUUGAGCU | 817265 |
| TFAP2A | NM_003220.1 | 0.37 | 978 | tacgtgtgcgaaaccgaatttcc | 546 | CGUGUGCGAAACCGAAUUUCC | 817266 | AAAUUCGGUUUCGCACACGUA | 817267 |
| EIF3S10 | NM_003750.1 | 0.28 | 145 | ccctcaaacgcgccaacgaattt | 56509 | CUCAAACGCGCCAACGAAUUU | 817268 | AUUCGUUGGCGCGUUUGAGGG | 817269 |
| EIF3S9 | NM_003751.2 | 0.12 | 641 | gggacccgaccgacttgagaaac | 51229 | GACCCGACCGACUUGAGAAAC | 817270 | UUCUCAAGUCGGUCGGGUCCC | 817271 |
| EIF3S8 | NM_003752.2 | 0.1 | 417 | ctgacctagaggactatcttaat | 56770 | GACCUAGAGGACUAUCUUAAU | 817272 | UAAGAUAGUCCUCUAGGUCAG | 817273 |
| EIF3S7 | NM_003753.2 | 0.15 | 1729 | ctcggtaccacgtgaaagactcc | 56765 | CGGUACCACGUGAAAGACUCC | 817274 | AGUCUUUCACGUGGUACCGAG | 817275 |
| EIF3S4 | NM_003755.2 | 0.12 | 182 | aggtcatcaacggaaacataaag | 51220 | GUCAUCAACGGAAACAUAAAG | 817276 | UUAUGUUUCCGUUGAUGACCU | 817277 |
| EIF3S3 | NM_003756.1 | 0.19 | 601 | aagaagtgccgattgtaattaaa | 56655 | GAAGUGCCGAUUGUAAUUAAA | 817278 | UAAUUACAAUCGGCACUUCUU | 817279 |
| EIF3S2 | NM_003757.1 | 0.11 | 46 | agcggtccattacgcagattaag | 56617 | CGGUCCAUUACGCAGAUUAAG | 817280 | UAAUCUGCGUAAUGGACCGCU | 817281 |
| EIF3S1 | NM_003758.1 | 0.16 | 442 | gacctcgaattagcaaaggaaac | 56505 | CCUCGAAUUAGCAAAGGAAAC | 817282 | UUCCUUUGCUAAUUCGAGGUC | 817283 |
| BAG1 | NM_004323.2 | 0.25 | 697 | atggttgccgggtcatgttaatt | 129118 | GGUUGCCGGGUCAUGUUAAUU | 817284 | UUAACAUGACCCGGCAACCAU | 817285 |
| AKT1 | NM_005163.1 | 0.21 | 239 | aacgaggggagtacatcaagacc | 71961 | CGAGGGGAGUACAUCAAGACC | 817286 | UCUUGAUGUACUCCCCUCGUU | 817287 |
| NDRG1 | NM_006096.2 | 0.074 | 567 | gcctacatcctaactcgatttgc | 236862 | CUACAUCCUAACUCGAUUUGC | 817288 | AAAUCGAGUUAGGAUGUAGGC | 817289 |
| TSG101 | NM_006292.2 | 0.13 | 943 | atggttacccgtttagatcaaga | 43049 | GGUUACCCGUUUAGAUCAAGA | 817290 | UUGAUCUAAACGGGUAACCAU | 817291 |
| BRCA1 | NM_007294.1 | 0.22 | 4329 | gagggataccatgcaacataacc | 16042 | GGGAUACCAUGCAACAUAACC | 817292 | UUAUGUUGCAUGGUAUCCCUC | 817293 |
| NOTCH2 | NM_024408.2 | 0.085 | 6047 | cgcaaccgagtaactgatctaga | 149219 | CAACCGAGUAACUGAUCUAGA | 817294 | UAGAUCAGUUACUCGGUUGCG | 817295 |
| ARHC | NM_175744.3 | 0.11 | 194 | gtctacgtccctactgtctttga | 108338 | CUACGUCCCUACUGUCUUUGA | 817296 | AAAGACAGUAGGGACGUAGAC | 817297 |
| BLM | NM_000057.1 | 0.35 | 1998 | gagcgtttccaaagtcttagttt | 22786 | GCGUUUCCAAAGUCUUAGUUU | 817298 | ACUAAGACUUUGGAAACGCUC | 817299 |
| GSN | NM_000177.3 | 0.13 | 740 | cagcaatcggtatgaaagactga | 113910 | GCAAUCGGUAUGAAAGACUGA | 817300 | AGUCUUUCAUACCGAUUGCUG | 817301 |
| MLH1 | NM_000249.2 | 0.19 | 847 | aaccatcgtctggtagaatcaac | 91691 | CCAUCGUCUGGUAGAAUCAAC | 817302 | UGAUUCUACCAGACGAUGGUU | 817303 |
| MSH2 | NM_000251.1 | 0.14 | 1282 | accgactctatcagggtataaat | 16366 | CGACUCUAUCAGGGUAUAAAU | 817304 | UUAUACCCUGAUAGAGUCGGU | 817305 |
| SOD1 | NM_000454.4 | 0.037 | 343 | tggtgtggccgatgtgtctattg | 167035 | GUGUGGCCGAUGUGUCUAUUG | 817306 | AUAGACACAUCGGCCACACCA | 817307 |
| TOP2A | NM_001067.2 | 0.24 | 2525 | ctgctagtccacgatacatcttt | 42581 | GCUAGUCCACGAUACAUCUUU | 817308 | AGAUGUAUCGUGGACUAGCAG | 817309 |

Table 1E

| Gene Name | refseq_ID | RQ | Target Position | Target sequence | SEQ ID NO | siRNA-sense | SEQ ID NO | siRNA-antisense | SEQ ID NO |
|---|---|---|---|---|---|---|---|---|---|
| TOP2B | NM_001068.2 | 0.11 | 1011 | aggtggacggcacgtggattatg | 42675 | GUGGACGGCACGUGGAUUAUG | 817310 | UAAUCCACGUGCCGUCCACCU | 817311 |
| TUBG1 | NM_001070.3 | 0.11 | 603 | gtggtggtccagccttacaattc | 111063 | GGUGGUCCAGCCUUACAAUUC | 817312 | AUUGUAAGGCUGGACCACCAC | 817313 |
| SLC25A5 | NM_001152.1 | 0.035 | 670 | tgcttccggatcccaagaacact | 181277 | CUUCCGGAUCCCAAGAACACU | 817314 | UGUUCUUGGGAUCCGGAAGCA | 817315 |
| ANXA11 | NM_001157.2 | 0.17 | 1685 | cacgacatctcgggagatacttc | 128933 | CGACAUCUCGGGAGAUACUUC | 817316 | AGUAUCUCCCGAGAUGUCGUG | 817317 |
| AP2B1 | NM_001282.1 | 0.19 | 714 | tgccgtagcggcattatctgaaa | 273115 | CCGUAGCGGCAUUAUCUGAAA | 817318 | UCAGAUAAUGCCGCUACGGCA | 817319 |
| GTF2I | NM_001518.2 | 0.37 | 978 | atgctgacaggtcaatactatct | 4585 | GCUGACAGGUCAAUACUAUCU | 817320 | AUAGUAUUGACCUGUCAGCAU | 817321 |
| IGFBP7 | NM_001553.1 | 0.045 | 488 | tgcgagcaaggtccttccatagt | 124925 | CGAGCAAGGUCCUUCCAUAGU | 817322 | UAUGGAAGGACCUUGCUCGCA | 817323 |
| AXL | NM_001699.3 | 0.14 | 1857 | aagaaggagacccgttatggaga | 73957 | GAAGGAGACCCGUUAUGGAGA | 817324 | UCCAUAACGGGUCUCCUUCUU | 817325 |
| CAPG | NM_001747.1 | 0.17 | 790 | ggccgcagctctgtataaggtct | 113927 | CCGCAGCUCUGUAUAAGGUCU | 817326 | ACCUUAUACAGAGCUGCGGCC | 817327 |
| DUT | NM_001948.2 | 0.16 | 419 | tgcgaacggattttttatccaga | 165338 | CGAACGGAUUUUUUAUCCAGA | 817328 | UGGAUAAAAAAUCCGUUCGCA | 817329 |
| JUP | NM_002230.1 | 0.18 | 1133 | atccgtgtgtcccagcaataagc | 120947 | CCGUGUGUCCCAGCAAUAAGC | 817330 | UUAUUGCUGGGACACACGGAU | 817331 |
| KPNB1 | NM_002265.4 | 0.043 | 2885 | ggcggagatcgaagactaacaaa | 157208 | CGGAGAUCGAAGACUAACAAA | 817332 | UGUUAGUCUUCGAUCUCCGCC | 817333 |
| MYH9 | NM_002473.3 | 0.12 | 465 | caccgcctacaggagtatgatgc | 92428 | CCGCCUACAGGAGUAUGAUGC | 817334 | AUCAUACUCCUGUAGGCGGUG | 817335 |
| PFN2 | NM_002628.2 | 0.08 | 82 | cggctactgcgacgccaaatacg | 118724 | GCUACUGCGACGCCAAAUACG | 817336 | UAUUUGGCGUCGCAGUAGCCG | 817337 |
| PPP1CA | NM_002708.2 | 0.081 | 239 | ctcaagatctgcggtgacataca | 162170 | CAAGAUCUGCGGUGACAUACA | 817338 | UAUGUCACCGCAGAUCUUGAG | 817339 |
| PPP1CB | NM_002709.1 | 0.15 | 1028 | ttgctaaacgacagttggtaacc | 162204 | GCUAAACGACAGUUGGUAACC | 817340 | UUACCAACUGUCGUUUAGCAA | 817341 |
| PPP1CC | NM_002710.1 | 0.32 | 1084 | aacgcctccaaggggtatgatca | 162234 | CGCCUCCAAGGGGUAUGAUCA | 817342 | AUCAUACCCCUUGGAGGCGUU | 817343 |
| THBS1 | NM_003246.2 | 0.28 | 3224 | caccgaaagggacgatgactatg | 153751 | CCGAAAGGGACGAUGACUAUG | 817344 | UAGUCAUCGUCCCUUUCGGUG | 817345 |
| TTC1 | NM_003314.1 | 0.16 | 879 | accggctcgtactccatcaattt | 136959 | CGGCUCGUACUCCAUCAAUUU | 817346 | AUUGAUGGAGUACGAGCCGGU | 817347 |
| TXNRD1 | NM_003330.2 | 0.15 | 1777 | gacgattccgtcaagagataaca | 167072 | CGAUUCCGUCAAGAGAUAACA | 817348 | UUAUCUCUUGACGGAAUCGUC | 817349 |
| VIL2 | NM_003379.3 | 0.078 | 458 | aggaatccttagcgatgagatct | 292759 | GAAUCCUUAGCGAUGAGAUCU | 817350 | AUCUCAUCGCUAAGGAUUCCU | 817351 |
| VIM | NM_003380.1 | 0.073 | 1447 | tcctgattaagacggttgaaact | 287581 | CUGAUUAAGACGGUUGAAACU | 817352 | UUUCAACCGUCUUAAUCAGGA | 817353 |
| EXO1 | NM_003686.3 | 0.25 | 1631 | tggaacgagtgattagtactaaa | 26320 | GAACGAGUGAUUAGUACUAAA | 817354 | UAGUACUAAUCACUCGUUCCA | 817355 |
| RUVBL1 | NM_003707.1 | 0.085 | 215 | gaggcatgtggcgtcatagtaga | 100083 | GGCAUGUGGCGUCAUAGUAGA | 817356 | UACUAUGACGCCACAUGCCUC | 817357 |
| ADAM9 | NM_003816.1 | 0.15 | 1051 | agccacgcaggcgggattaatgt | 155099 | CCACGCAGGCGGGAUUAAUGU | 817358 | AUUAAUCCCGCCUGCGUGGCU | 817359 |
| TNFRSF10B | NM_003842.3 | 0.41 | 945 | tgcagccgtagtcttgattgtgg | 127913 | CAGCCGUAGUCUUGAUUGUGG | 817360 | ACAAUCAAGACUACGGCUGCA | 817361 |

Table 1F

| Gene Name | refseq_ID | RQ | Target Position | Target sequence | SEQ ID NO | siRNA-sense | SEQ ID NO | siRNA-antisense | SEQ ID NO |
|---|---|---|---|---|---|---|---|---|---|
| SHMT1 | NM_004169.3 | 0.12 | 975 | gccgagctggcatgatcttctac | 214683 | CGAGCUGGCAUGAUCUUCUAC | 817362 | AGAAGAUCAUGCCAGCUGCGGC | 817363 |
| CAD | NM_004341.2 | 0.36 | 5394 | ggggaggttgcctatatcgatgg | 74903 | GGAGGUUGCCUAUAUCGAUGG | 817364 | AUCGAUAUAGGCAACCUCCCC | 817365 |
| CSK | NM_004383.1 | 0.22 | 1132 | gacgcaactgcggcatagcaacc | 77629 | CGCAACUGCGGCAUAGCAACC | 817366 | UUGCUAUGCCGCAGUUGCGUC | 817367 |
| XPC | NM_004628.3 | 0.31 | 584 | ttcgagggcgatgaaacgtttc | 17754 | CGGAGGGCGAUGAAACGUUUC | 817368 | AACGUUUCAUCCCCUCCGAA | 817369 |
| HGS | NM_004712.3 | 0.35 | 1883 | cccaatgcacggcgtgtacatga | 157053 | CAAUGCACGGCGUGUACAUGA | 817370 | AUGUACACGCCGUGCAUUGGG | 817371 |
| LRRFIP1 | NM_004735.2 | 0.093 | 1426 | gtgtcctttagggcatagtgatg | 48486 | GUCCUUUAGGGCAUAGUGAUG | 817372 | UCACUAUGCCCUAAAGGACAC | 817373 |
| CALM3 | NM_005184.1 | 0.16 | 538 | caggtcaattatgaagagtttgt | 129585 | GGUCAAUUAUGAAGAGUUUGU | 817374 | AAACUCUUCAUAAAUUGACCUG | 817375 |
| DIAPH1 | NM_005219.2 | 0.2 | 885 | cagccgctgctggattgattaaa | 116504 | GCCCGCUGCUGGAUUGAUUAAA | 817376 | UAAUCCAUCCAGCAGCGGCUG | 817377 |
| NCL | NM_005381.2 | 0.036 | 1276 | gagcgagatgcgagaacactttt | 33529 | GCGAGAUGCGAGAACACUUUU | 817378 | AAGUGUUCUCGCAUCUCGCUC | 817379 |
| TOB1 | NM_005749.2 | 0.097 | 588 | accaagttcgctctaccaaaat | 136820 | CAAGUUCGGCUCUACCAAAAU | 817380 | UUUGGUAGAGCCGAACUUGGU | 817381 |
| MADH2 | NM_005901.2 | 0.088 | 1456 | aagccgtctatcagctaactaga | 133708 | GCCGUCUAUCAGCUAACUAGA | 817382 | UAGUUAGCUGAUAGACGGCUU | 817383 |
| GNB2L1 | NM_006098.3 | 0.16 | 380 | caccaccaggaggcgatttgtgg | 125242 | CCACCACGAGGCGAUUUGUGG | 817384 | ACAAAUCGCCUCGUGGUGGUG | 817385 |
| PPP2R5A | NM_006243.2 | 0.18 | 890 | gagtatgtttcaactaatcgtgg | 298747 | GUAUGUUUCAACUAAUCGUGG | 817386 | ACGAUUAGUUGAAACAUACUC | 817387 |
| HYOU1 | NM_006389.2 | 0.1 | 427 | tccaaaggctacgctacgttact | 85421 | CAAAGGCUACGCUACGUUACU | 817388 | UAACGUAGCGUAGCCUUUGGA | 817389 |
| KHDRBS1 | NM_006559.1 | 0.15 | 1248 | aaggctacgaaggctattacagc | 19623 | GGCUACGAAGGCUAUUACAGC | 817390 | UGUAAUAGCCUUCGUAGCCUU | 817391 |
| METAP2 | NM_006838.2 | 0.084 | 738 | atgccggtgacacaacagtatta | 186558 | GCCGGUGACACAACAGUAUUA | 817392 | AUACUGUUGUGUCACCGGCAU | 817393 |
| CALM1 | NM_006888.2 | 0.4 | 519 | tacgtcacgtcatgacaaactta | 129581 | CGUCACGUCAUGACAAACUUA | 817394 | AGUUUGUCAUGACGUGACGUA | 817395 |
| TOPBP1 | NM_007027.2 | 0.3 | 1047 | atgcaagttgcgtaagtgaatca | 126366 | GCAAGUUGCGUAAGUGAAUCA | 817396 | AUUCACUUACGCAACUUGCAU | 817397 |
| PIAS1 | NM_016166.1 | 0.37 | 1704 | gccttacgacttacaaggattag | 35123 | CUUACGACUUACAAGGAUUAG | 817398 | AAUCCUUGUAAGUCGUAAGGC | 817399 |
| NMT1 | NM_021079.3 | 0.25 | 1025 | ctgggctgcgaccaatggaaaca | 215454 | GGGCUGCGACCAAUGGAAACA | 817400 | UUUCCAUUGGUCGCAGCCCAG | 817401 |
| PPP2R4 | NM_021131.3 | 0.28 | 332 | cgctgactacatcggattcatcc | 298444 | CUGACUACAUCGGAUUCAUCC | 817402 | AUGAAUCCGAUGUAGUCAGCG | 817403 |
| MSH6 | NM_000179.1 | 0.17 | 3185 | tgcggcgactgttctataacttt | 16779 | CGGCGACUGUUCUAUAACUUU | 817404 | AGUAUAGAACAGUCGCCGCA | 817405 |
| EIF4A1 | NM_001416.1 | 0.16 | 493 | tggccgtgtgtttgatatgctta | 25763 | GCCGUGUGUUUGAUAUGCUUA | 817406 | AGCAUAUCAAACACACGGCCA | 817407 |
| ATP2A2 | NM_001681.2 | 0.2 | 2836 | atccccatacccgatgacaatgg | 72769 | CCCAUACCCGAUGACAAUGG | 817408 | AUUGUCAUCGGGUAUGGGAU | 817409 |
| HNRPK | NM_002140.2 | 0.24 | 458 | ccccgagcgcatattgagtatca | 28586 | CCGAGCGCAUAUUGAGUAUCA | 817410 | AUACUCAAUAUGCGCUCGGG | 817411 |
| MSN | NM_002444.2 | 0.058 | 1806 | agcgcattgacgaatttgagtct | 287504 | CGCAUUGACGAAUUUGAGUCU | 817412 | ACUCAAAUUCGUCAAUGCGCU | 817413 |

Table 1G

| Gene Name | refseq_ID | RQ | Target Position | Target sequence | SEQ ID NO | siRNA-sense | SEQ ID NO | siRNA-antisense | SEQ ID NO |
|---|---|---|---|---|---|---|---|---|---|
| MAPK6 | NM_002748.2 | 0.16 | 1854 | ttggcctgtacataacaactttg | 89971 | GGCCUGUACAUAACAACUUUG | 817414 | AAGUUGUUAUGUACAGGCCAA | 817415 |
| MAP2K3 | NM_002756.2 | 0.26 | 626 | ttctacggggcactattcagaga | 88970 | CUACGGGGCACUAUUCAGAGA | 817416 | UCUGAAUAGUGCCCCGUAGAA | 817417 |
| RDX | NM_002906.2 | 0.049 | 43 | atcaacgtaagagtaactacaat | 118854 | CAACGUAAGAGUAACUACAAU | 817418 | UGUAGUUACUCUUACGUUGAU | 817419 |
| BHLHB2 | NM_003670.1 | 0.31 | 1011 | gagaaaggatcggcgcaattaag | 1511 | GAAAGGAUCGGCGCAAUUAAG | 817420 | UAAUUGCGCCGAUCCUUUCUC | 817421 |
| RIPK2 | NM_003821.4 | 0.38 | 1028 | acctcaccgagcacgtatgatct | 99178 | CUCACCGAGCACGUAUGAUCU | 817422 | AUCAUACGUGCUCGGUGAGGU | 817423 |
| HSF1 | NM_005526.1 | 0.1 | 1137 | ccccgaccgccctcattgactcc | 5332 | CCGACCGCCCUCAUUGACUCC | 817424 | AGUCAAUGAGGGCGGUCGGGG | 817425 |
| POP4 | NM_006627.1 | 0.099 | 639 | gtgaacggtctgcgaagaagttc | 53540 | GAACGGUCUGCGAAGAAGUUC | 817426 | ACUUCUUCGCAGACCGUUCAC | 817427 |
| DDX18 | NM_006773.3 | 0.28 | 196 | ctgaccctatcggaaactcaaaa | 50595 | GACCCUAUCGGAAACUCAAAA | 817428 | UUGAGUUUCCGAUAGGGUCAG | 817429 |
| DDX24 | NM_020414.3 | 0.24 | 2275 | aaggagcgaatccgtttagctcg | 50750 | GGAGCGAAUCCGUUUAGCUCG | 817430 | AGCUAAACGGAUUCGCUCCUU | 817431 |
| IFNGR1 | NM_000416.1 | 0.18 | 220 | taccgtagaggtaaagaactatg | 124614 | CCGUAGAGGUAAAGAACUAUG | 817432 | UAGUUCUUUACCUCUACGGUA | 817433 |
| AK1 | NM_000476.1 | 0.25 | 517 | agcggctggagacctattacaag | 71895 | CGGCUGGAGACCUAUUACAAG | 817434 | UGUAAUAGGUCUCCAGCCGCU | 817435 |
| SERPINE1 | NM_000602.1 | 0.4 | 786 | cacgcccgatggccattactacg | 183595 | CGCCCGAUGGCCAUUACUACG | 817436 | UAGUAAUGGCCAUCGGGCGUG | 817437 |
| IGF2R | NM_000876.1 | 0.14 | 6206 | acggagtctcgtactatataaat | 206285 | GGAGUCUCGUACUAUAUAAAU | 817438 | UUAUAUAGUACGAGACUCCGU | 817439 |
| RRM1 | NM_001033.2 | 0.25 | 1880 | cagggcccatacgaaacctatga | 226213 | GGGCCCAUACGAAACCUAUGA | 817440 | AUAGGUUUCGUAUGGGCCCUG | 817441 |
| CSNK1G2 | NM_001319.5 | 0.34 | 228 | gagctccgcctaggaaagaatct | 77703 | GCUCCGCCUAGGAAAGAAUCU | 817442 | AUUCUUUCCUAGGCGGAGCUC | 817443 |
| CDC42 | NM_001791.2 | 0.15 | 160 | tcctgatatcctacacaacaaac | 108667 | CUGAUAUCCUACACAACAAAC | 817444 | UUGUUGUGUAGGAUAUCAGGA | 817445 |
| CDH2 | NM_001792.2 | 0.23 | 1737 | atgccggtaccatgttgacaaca | 139854 | GCCGGUACCAUGUUGACAACA | 817446 | UUGUCAACAUGGUACCGGCAU | 817447 |
| CLU | NM_001831.1 | 0.14 | 161 | aagtaagtacgtcaataaggaaa | 303887 | GUAAGUACGUCAAUAAGGAAA | 817448 | UCCUUAUUGACGUACUUACUU | 817449 |
| CSNK1A1 | NM_001892.3 | 0.16 | 796 | agggctaaaggctgcaacaaaga | 77640 | GGCUAAAGGCUGCAACAAAGA | 817450 | UUUGUUGCAGCCUUUAGCCCU | 817451 |
| CSNK1D | NM_001893.3 | 0.29 | 657 | gtcgcatcgaatacattcattca | 77650 | CGCAUCGAAUACAUUCAUUCA | 817452 | AAUGAAUGUAUUCGAUGCGAC | 817453 |
| CTNNA1 | NM_001903.2 | 0.22 | 653 | aacgttccgatcctctatactgc | 290175 | CGUUCCGAUCCUCUAUACUGC | 817454 | AGUAUAGAGGAUCGGAACGUU | 817455 |
| DDR1 | NM_001954.3 | 0.32 | 1176 | ggctatgcaggtccactgtaaca | 78045 | CUAUGCAGGUCCACUGUAACA | 817456 | UUACAGUGGACCUGCAUAGCC | 817457 |
| PLAGL1 | NM_002656.2 | 0.31 | 2951 | ctcctgctacccaaaataccttt | 35406 | CCUGCUACCCAAAAUACCUUU | 817458 | AGGUAUUUUGGGUAGCAGGAG | 817459 |
| PPM1B | NM_002706.3 | 0.18 | 1479 | ttgctggcaagcgtaatgttatt | 162107 | GCUGGCAAGCGUAAUGUUAUU | 817460 | UAACAUUACGCUUGCCAGCAA | 817461 |
| PTPRF | NM_002840.2 | 0.14 | 3438 | aggttcccgactcctataagtca | 37154 | GUUCCCGACUCCUAUAAGUCA | 817462 | ACUUAUAGGAGUCGGGAACCU | 817463 |
| RPA1 | NM_002945.2 | 0.12 | 1784 | tacaacgacgagtctcgaattaa | 19815 | CAACGACGAGUCUCGAAUUAA | 817464 | AAUUCGAGACUCGUCGUUGUA | 817465 |

EP 1 752 536 A1

## Table 1H

| Gane Name | refseq_ID | RQ | Target Position | Target sequence | SEQ ID NO | siRNA-sense | SEQ ID NO | siRNA-antisense | SEQ ID NO |
|---|---|---|---|---|---|---|---|---|---|
| SMARCA4 | NM_003072.2 | 0.18 | 3624 | tgcgtatcgcggctttaaatacc | 11695 | CGUAUCGCGGCUUUAAAUACC | 817466 | UAUUUAAAGCCGCGAUACGCA | 817467 |
| YY1 | NM_003403.3 | 0.15 | 904 | gacgacgactacattgaacaaac | 13964 | CGACGACUACAUUGAACAAAC | 817468 | UUGUUCAAUGUAGUCGUCGUC | 817469 |
| USP7 | NM_003470.1 | 0.25 | 1096 | ttgtcgagtgttgctcgataatg | 190309 | GUCGAGUGUUGCUCGAUAAUG | 817470 | UUAUCGAGCAACACUCGACAA | 817471 |
| IKBKG | NM_003639.2 | 0.44 | 1164 | aggcccaggcggatatctacaag | 254494 | GCCCAGGCGGAUAUCUACAAG | 817472 | UGUAGAUAUCCGCCUGGGCCU | 817473 |
| IQGAP1 | NM_003870.3 | 0.12 | 2335 | tcgctgccgtggatacttagttc | 121324 | GCUGCCGUGGAUACUUAGUUC | 817474 | ACUAAGUAUCCACGGCAGCGA | 817475 |
| CREBBP | NM_004380.1 | 0.19 | 2205 | gaggtcgcgtttacataaacaag | 2467 | GGUCGCGUUUACAUAAACAAG | 817476 | UGUUUAUGUAAACGCGACCUC | 817477 |
| CSNK1G3 | NM_004384.1 | 0.31 | 1252 | cccaccgcaggacgttcaaatgc | 77737 | CACCGCAGGACGUUCAAAUGC | 817478 | AUUUGAACGUCCUGCGGUGGG | 817479 |
| PPARBP | NM_004774.2 | 0.17 | 489 | atgttacatcacgtcagatatgt | 36486 | GUUACAUCACGUCAGAUAUGU | 817480 | AUAUCUGACGUGAUGUAACAU | 817481 |
| SFPQ | NM_005066.1 | 0.15 | 1533 | atggcacgtttgagtacgaatat | 39201 | GGCACGUUUGAGUACGAAUAU | 817482 | AUUCGUACUCAAACGUGCCAU | 817483 |
| ROCK1 | NM_005406.1 | 0.14 | 1201 | agcaatcgtagatacttatcttc | 99228 | CAAUCGUAGAUACUUAUCUUC | 817484 | AGAUAAGUAUCUACGAUUGCU | 817485 |
| TP53BP1 | NM_005657.1 | 0.28 | 396 | gacggtaatagtggggttcaatga | 20875 | CGGUAAUAGUGGGUUCAAUGA | 817486 | AUUGAACCCACUAUUACCGUC | 817487 |
| NCOR1 | NM_006311.2 | 0.36 | 6785 | cccgctcaccagggagtataagc | 33678 | CGCUCACCAGGGAGUAUAAGC | 817488 | UUAUACUCCCUGGUGAGCGGG | 817489 |
| TADA3L | NM_006354.2 | 0.1 | 1233 | ctgaccgaactggacactaaaga | 12451 | GACCGAACUGGACACUAAAGA | 817490 | UUUAGUGUCCAGUUCGGUCAG | 817491 |
| CTCF | NM_006565.1 | 0.25 | 1786 | cagtgtgattacgcttgtagaca | 2613 | GUGUGAUUACGCUUGUAGACA | 817492 | UCUACAAGCGUAAUCACACUG | 817493 |
| RUVBL2 | NM_006666.1 | 0.086 | 218 | gccggtcgggcagtccttattgc | 100117 | CGGUCGGGCAGUCCUUAUUGC | 817494 | AAUAAGGACUGCCCGACCGGC | 817495 |
| PRKDC | NM_006904.6 | 0.18 | 11629 | atgtataagggcgctaatcgtac | 205894 | GUAUAAGGGCGCUAAUCGUAC | 817496 | ACGAUUAGCGCCCUUAUACAU | 817497 |
| CNOT7 | NM_013354.4 | 0.12 | 844 | ttgagatccttcgattgtttttt | 2347 | GAGAUCCUUCGAUUGUUUUUU | 817498 | AAAACAAUCGAAGGAUCUCAA | 817499 |
| GSK3A | NM_019884.2 | 0.14 | 1477 | accccgtcctcacaagctttaac | 84369 | CCCGUCCUCACAAGCUUUAAC | 817500 | UAAAGCUUGUGAGGACGGGGU | 817501 |
| XRCC5 | NM_021141.2 | 0.83 | 1202 | tggccatagttcgatatgcttat | 20327 | GCCAUAGUUCGAUAUGCUUAU | 817502 | AAGCAUAUCGAACUAUGGCCA | 817503 |
| APP | NM_000484.1 | 0.14 | 1604 | ggcctcgtcacgtgttcaatatg | 128991 | CCUCGUCACGUGUUCAAUAUG | 817504 | UAUUGAACACGUGACGAGGCC | 817505 |
| ABCC5 | NM_005688.1 | 0.38 | 4297 | ttctaggctccgataggattatg | 70574 | CUAGGCUCCGAUAGGAUUAUG | 817506 | UAAUCCUAUCGGAGCCUAGAA | 817507 |
| NR2F2 | NM_021005.2 | 0.17 | 1106 | ctcgtacctgtccggatatattt | 8466 | CGUACCUGUCCGGAUAUAUUU | 817508 | AUAUAUCCGGACAGGUACGAG | 817509 |
| CDK4 | NM_000075.2 | 0.32 | 388 | ttcgtgaggtggctttactgagg | 76146 | CGUGAGGUGGCUUUACUGAGG | 817510 | UCAGUAAAGCCACCUCACGAA | 817511 |
| CLN2 | NM_000391.2 | 0.14 | 643 | tccgtaagcgatacaacttgacc | 183713 | CGUAAGCGAUACAACUUGACC | 817512 | UCAAGUUGUAUCGCUUACGGA | 817513 |
| AAMP | NM_001087.2 | 0.27 | 300 | tagcgaggtcacctttgcattgc | 177411 | GCGAGGUCACCUUUGCAUUGC | 817514 | AAUGCAAAGGUGACCUCGCUA | 817515 |
| ACLY | NM_001096.2 | 0.19 | 1229 | ggcatcgtgagagcaattcgaga | 71580 | CAUCGUGAGAGCAAUUCGAGA | 817516 | UCGAAUUGCUCUCACGAUGCC | 817517 |

EP 1 752 536 A1

Table 1I

| Gene Name | refseq_ID | RQ | Target Position | Target sequence | SEQ ID NO | siRNA-sense | SEQ ID NO | siRNA-antisense | SEQ ID NO |
|---|---|---|---|---|---|---|---|---|---|
| ADD1 | NM_001119.3 | 0.14 | 255 | aggtacttcgaccgagtagatga | 115154 | GUACUUCGACCGAGUAGAUGA | 817518 | AUCUACUCGGUCGAAGUACCU | 817519 |
| FLNA | NM_001456.1 | 0.13 | 4967 | aaccatgacggcacgtatacagt | 114101 | CCAUGACGGCACGUAUACAGU | 817520 | UGUAUACGUGCCGUCAUGGU | 817521 |
| IL13RA1 | NM_001560.2 | 0.077 | 497 | accagtccgacactaactatac | 244096 | CAGUCCCGACACUAACUAUAC | 817522 | AUAGUUAGUGUCGGGACUGGU | 817523 |
| IL18 | NM_001562.2 | 0.19 | 679 | ttcatcatacgaaggatactttc | 167828 | CAUCAUACGAAGGAUACUUUC | 817524 | AAGUAUCCUUCGUAUGAUGAA | 817525 |
| ARF6 | NM_001663.2 | 0.32 | 730 | gccgctctggccgcattactaca | 108231 | CGCUCUGGCCGCAUUACUACA | 817526 | UAGUAAUGCCGGCCAGAGCGGC | 817527 |
| ITGB4BP | NM_002212.2 | 0.13 | 82 | gagcttcgttcgagaacaactgt | 56942 | GCUUCGUUCGAGAACAACUGU | 817528 | AGUUGUUCUCGAACGAAGCUC | 817529 |
| MYBL2 | NM_002466.2 | 0.093 | 975 | caggagccatcggtacagatct | 7221 | GGAGCCAUCGGUACAGAUCU | 817530 | AUCUGUACCGAUGGCUCCUG | 817531 |
| NME2 | NM_002512.1 | 0.045 | 485 | aactggttgactacaagtcttgt | 8178 | CUGGUUGACUACAAGUCUUGU | 817532 | AAGACUUGUAGUCAACCAGGU | 817533 |
| RAP1A | NM_002884.1 | 0.072 | 529 | aagaacggccaaggtttgcact | 110570 | GAACGGCCAAGGUUUGCACU | 817534 | UGCAAAACCUUGGCCGUUCUU | 817535 |
| RPA2 | NM_002946.3 | 0.15 | 289 | aacagtggattcgaaagctatgg | 19817 | CAGUGGAUUCGAAAGCUAUGG | 817536 | AUAGCUUUCGAAUCCACUGUU | 817537 |
| SDC2 | NM_002998.3 | 0.27 | 1182 | aggcacctactaaggagttttat | 121066 | GCACCUACUAAGGAGUUUUAU | 817538 | AAAACUCCUUAGUAGGUGCCU | 817539 |
| SDC4 | NM_002999.2 | 0.14 | 338 | cccaccgaacccaagaaaactaga | 121072 | CACCGAACCCAAGAAAACUAGA | 817540 | UAGUUUUCUUGGGUUCGGUGGG | 817541 |
| TCF12 | NM_003205.2 | 0.17 | 1972 | cgcttacgcgtgcgggatattaa | 41688 | CUUACGCGUGCGGGAUAUUAA | 817542 | AAUUCCCGCACGCGUAAGCG | 817543 |
| TIMP1 | NM_003254.1 | 0.12 | 364 | accgcagcgaggagtttctcatt | 186907 | CGCAGCGAGGAGUUUCUCAUU | 817544 | UGAGAAACUCCUCGCUGCGGU | 817545 |
| TRA1 | NM_003299.1 | 0.22 | 827 | taggacgggaacgacacaattacc | 102862 | GGACGGGAACGACACAAUUACC | 817546 | UAAUUGUCGUCCCCGUCCUA | 817547 |
| VCL | NM_003373.2 | 0.09 | 2111 | gtctcggctgctcgtatcttact | 120054 | CUCGGCUGCUCGUAUCUUACU | 817548 | UAAGAUACGAGCAGCCGAGAC | 817549 |
| CXCR4 | NM_003467.1 | 0.37 | 90 | tggaggggatcagtatatacact | 124591 | GAGGGGAUCAGUAUAUACACU | 817550 | UGUAUAUACUGAUCCCCUCCA | 817551 |
| SUCLG1 | NM_003849.1 | 0.5 | 127 | ttctcggccaacatctctatgttg | 110935 | CUCGGCCAACAUCUCUAUGUUG | 817552 | ACAUAGAGAUGUUGGCCGAGAA | 817553 |
| MBD2 | NM_003927.3 | 0.1 | 902 | ctgcgaaacgatcctctcaatca | 20354 | GCGAAACGAUCCUCUCAAUCA | 817554 | AUUGAGAGGAUCGUUUCGCAG | 817555 |
| USP13 | NM_003940 | 0.19 | 2338 | ggcactacgagcaacgaataata | 154317 | CACUACGAGCAACGAAUAAUA | 817556 | UUAUUCGUUGCUCGUAGUGCC | 817557 |
| OSMR | NM_003999.1 | 0.31 | 3240 | ctccccgaccgagaatagcagc | 34501 | CCCCCGACCGAGAAUAGCAGC | 817558 | UGCUAUUCUCGGUCGGGGGAG | 817559 |
| GMFB | NM_004124.2 | 0.12 | 290 | gacaacctcgcttcattgtgtat | 117415 | CAACCUCGCUUCAUUGUGUAU | 817560 | ACACAAUGAAGCGAGGUUGUC | 817561 |
| EPHA2 | NM_004431.2 | 0.29 | 1535 | gtggaagtacgaggtcacttacc | 81348 | GGAGUACGAGGUCACUUACC | 817562 | UAAGUGACCUCGUACUCCUAC | 817563 |
| USP11 | NM_004651.2 | 0.089 | 643 | ttcagccataccgattctattgg | 188785 | CAGCCAUACCGAUUCUAUUGG | 817564 | AAUAGAAUCGGUAUGGCUGAA | 817565 |
| USP9X | NM_004652.2 | 0.057 | 3790 | gtggtcgttacagctagtattt | 190527 | GGGUCGUUACAGCUAGUAUUU | 817566 | AUACUAGCUGUAACGACCCAC | 817567 |
| USP14 | NM_005151.2 | 0.1 | 583 | tggcttcagcgcagtatattact | 188886 | GCUUCAGCGCAGUAUAUUACU | 817568 | UAAUAUACUGCGCUGAAGCCA | 817569 |

Table 1J

| Gane Name | refseq_ID | RQ | Target Position | Target sequence | SEQ ID NO | siRNA-sense | SEQ ID NO | siRNA-antisense | SEQ ID NO |
|---|---|---|---|---|---|---|---|---|---|
| USP10 | NM_005153.1 | 0.22 | 1353 | ccccgtgggctgatcaataaagg | 188770 | CCGUGGGCUGAUCAAUAAAGG | 817570 | UUUAUUGAUCAGCCCACGGGG | 817571 |
| USP8 | NM_005154.2 | 0.22 | 3283 | gagctcgacgggattctctaaaa | 190459 | GCUCGACGGGAUUCUCUAAAA | 817572 | UUAGAGAAUCCCGUCGAGCUC | 817573 |
| SDCBP | NM_005625.3 | 0.13 | 259 | tcctatccctcacgatggaaatc | 114865 | CUAUCCCUCACGAUGGAAAUC | 817574 | UUUCCAUCGUGAGGGAUAGGA | 817575 |
| CAPZA1 | NM_006135.1 | 0.16 | 101 | atgacgttcggctactacttaat | 113977 | GACGUUCGGCUACUACUUAAU | 817576 | UAAGUAGUAGCCGAACGUCAU | 817577 |
| CAPZA2 | NM_006136.2 | 0.15 | 762 | gacaatgtcggacactactttca | 114011 | CAAUGUCGGACACUACUUUCA | 817578 | AAAGUAGUGUCCGACAUUGUC | 817579 |
| NFE2L2 | NM_006164.2 | 0.21 | 324 | ttcgctcagttacaactagatga | 7735 | CGCUCAGUUACAACUAGAUGA | 817580 | AUCUAGUUGUAACUGAGCGAA | 817581 |
| USP16 | NM_006447.1 | 0.19 | 1205 | tggtggtgaactaactagtatga | 189070 | GUGGUGAACUAACUAGUAUGA | 817582 | AUACUAGUUAGUUCACCACCA | 817583 |
| LIM | NM_006457.1 | 0.19 | 1333 | ctccgatgtgcgcccattgtaac | 125275 | CCGAUGUGCGCCCAUUGUAAC | 817584 | UACAAUGGGCGCACAUCGGAG | 817585 |
| ADD3 | NM_016824.1 | 0.14 | 1732 | gacaatcgaacgtaaacaacaag | 121236 | CAAUCGAACGUAAACAACAAG | 817586 | UGUUGUUUACGUUCGAUUGUC | 817587 |
| MAP2K2 | NM_030662.2 | 0.16 | 670 | gtgacggggagatcagcatttgc | 88959 | GACGGGGAGAUCAGCAUUUGC | 817588 | AAAUGCUGAUCUCCCCGUCAC | 817589 |
| ADH5 | NM_000671.2 | 0.11 | 685 | ggcatttcaaccggttatggtgc | 155944 | CAUUUCAACCGGUUAUGGUGC | 817590 | ACCAUAACCGGUUGAAAUGCC | 817591 |
| ANXA1 | NM_000700.1 | 0.11 | 946 | ctcgccataaggcattgatcagg | 137873 | CGCCAUAAGGCAUUGAUCAGG | 817592 | UGAUCAAUGCCUUAUGGCGAG | 817593 |
| FOLR1 | NM_000802.2 | 0.32 | 259 | ttcctacctatatagattcaact | 179653 | CCUACCUAUAUAGAUUCAACU | 817594 | UUGAAUCUAUAUAGGUAGGAA | 817595 |
| POLR2B | NM_000938.1 | 0.16 | 2960 | ccctctcgtatgactattggtca | 36387 | CUCUCGUAUGACUAUUGGUCA | 817596 | ACCAAUAGUCAUACGAGAGGG | 817597 |
| CRIP2 | NM_001312.2 | 0.25 | 90 | gtgcgacaagaccgtgtacttcg | 156364 | GCGACAAGACCGUGUACUUCG | 817598 | AAGUACACGGUCUUGUCGCAC | 817599 |
| POLR2C | NM_002694.2 | 0.11 | 153 | ttcgattcggagggtcttcatcg | 36408 | CGAUUCGGAGGGUCUUCAUCG | 817600 | AUGAAGACCCUCCGAAUCGAA | 817601 |
| POLR2E | NM_002695.2 | 0.098 | 40 | gacgtaccggctctggaaaatcc | 36425 | CGUACCGGCUCUGGAAAAUCC | 817602 | AUUUUCCAGAGCCGGUACGUC | 817603 |
| RFC3 | NM_002915.2 | 0.38 | 120 | tgggacggctggactatcacaag | 38420 | GGACGGCUGGACUAUCACAAG | 817604 | UGUGAUAGUCCAGCCGUCCCA | 817605 |
| RFC4 | NM_002916.3 | 0.19 | 957 | ttcaaagcgctactcgattaaca | 38466 | CAAAGCGCUACUCGAUUAACA | 817606 | UUAAUCGAGUAGCGCUUUGAA | 817607 |
| SSB | NM_003142.2 | 0.1 | 276 | aggttgaaccgtctaacaacaga | 48020 | GUUGAACCGUCUAACAACAGA | 817608 | UGUUGUUAGACGGUUCAACCU | 817609 |
| HSPA9B | NM_004134.4 | 0.19 | 948 | ggccttgctacggcacattgtga | 85288 | CCUUGCUACGGCACAUUGUGA | 817610 | ACAAUGUGCCGUAGCAAGGCC | 817611 |
| FANCG | NM_004629.1 | 0.28 | 1405 | ctgctagttgaggccttgaatgt | 16276 | GCUAGUUGAGGCCUUGAAUGU | 817612 | AUUCAAGGCCUCAACUAGCAG | 817613 |
| POLR2K | NM_005034.2 | 0.17 | 182 | gtggatacagaataatgtacaag | 36435 | GGAUACAGAAUAAUGUACAAG | 817614 | UGUACAUUAUUCUGUAUCCAC | 817615 |
| PRCP | NM_005040.2 | 0.043 | 970 | tggcaatggtggactatccttat | 187207 | GCAAUGGUGGACUAUCCUUAU | 817616 | AAGGAUAGUCCACCAUUGCCA | 817617 |
| HSPA5 | NM_005347.2 | 0.21 | 1292 | gtggctcgactcgaattccaaag | 85234 | GGCUCGACUCGAAUUCCAAAG | 817618 | UUGGAAUUCGAGUCGAGCCAC | 817619 |
| POLR2H | NM_006232.2 | 0.099 | 262 | gtcatagctagtaccttgtatga | 159134 | CAUAGCUAGUACCUUGUAUGA | 817620 | AUACAAGGUACUAGCUAUGAC | 817621 |

Table 1K

| Gene Name | refseq_ID | RQ | Target Position | Target sequence | SEQ ID NO | siRNA-sense | SEQ ID NO | siRNA-antisense | SEQ ID NO |
|---|---|---|---|---|---|---|---|---|---|
| POLR2I | NM_006233.4 | 0.11 | 145 | acgcgtgccggaactgtgattac | 9602 | GCGUGCCGGAACUGUGAUUAC | 817622 | AAUCACAGUUCCGGCACGCGU | 817623 |
| POLR2J | NM_006234.3 | 0.15 | 359 | gcgctttcgggtggccataaaag | 36430 | GCUUUCGGGUGGCCAUAAAAG | 817624 | UUUAUGGCCACCCGAAAGCGC | 817625 |
| RFC5 | NM_007370.3 | 0.23 | 941 | aggggttggcactgcatgatatc | 38487 | GGGUUGGCACUGCAUGAUAUC | 817626 | UAUCAUGCAGUGCCAACCCCU | 817627 |
| TGFB1I1 | NM_015927.3 | 0.16 | 532 | gacttccgcgttcaaaaccatct | 112567 | CUUCCGCGUUCAAAACCAUCU | 817628 | AUGGUUUUGAACGCGGAAGUC | 817629 |
| PRKWNK1 | NM_018979.1 | 0.1 | 631 | gccgtgggaatgtctaacgatgg | 97669 | CGUGGGAAUGUCUAACGAUGG | 817630 | AUCGUUAGACAUUCCCACGGC | 817631 |
| POLR2F | NM_021974.2 | 0.052 | 105 | tggcgacgactttgatgatgtgg | 36427 | GCGACGACUUUGAUGAUGUGG | 817632 | ACAUCAUCAAAGUCGUCGCCA | 817633 |
| NME1 | NM_000269.2 | 0.045 | 185 | agcgttttgagcagaaaggattc | 94844 | CGUUUUGAGCAGAAAGGAUUC | 817634 | AUCCUUUCUGCUCAAAACGCU | 817635 |
| PEA15 | NM_003768.2 | 0.15 | 406 | ccgtcctgacctactcactatgg | 134551 | GUCCUGACCUACUCACUAUGG | 817636 | AUAGUGAGUAGGUCAGGACGG | 817637 |
| ARHGDIA | NM_004309.3 | 0.17 | 438 | ttccgggttaaccgagagatagt | 129087 | CCCGGGUUAACCGAGAGAUAGU | 817638 | UAUCUCUCGGUUAACCCGGAA | 817639 |
| ESRRA | NM_004451.3 | 0.3 | 1029 | ggccttcgctgaggacttagtcc | 3459 | CCUUCGCUGAGGACUUAGUCC | 817640 | ACUAAGUCCUCAGCGAAGGCC | 817641 |
| CAV1 | NM_001753.3 | 0.13 | 702 | cagtgcatcagccgtgtctattc | 289819 | GUGCAUCAGCCGUGUCUAUUC | 817642 | AUAGACACGGCUGAUGCACUG | 817643 |
| MKI67 | NM_002417.2 | 0.17 | 558 | cacgtcgtgtctcaagatctagc | 91497 | CGUCGUGUCUCAAGAUCUAGC | 817644 | UAGAUCUUGAGACACGACGUG | 817645 |
| CDKN1B | NM_004064.2 | 0.41 | 929 | ctgcaaccgacgattcttctact | 219267 | GCAACCGACGAUUCUUCUACU | 817646 | UAGAAGAAUCGUCGGUUGCAG | 817647 |
| ERBB2 | NM_004448.1 | 0.17 | 3386 | aaggggctggctccgatgtattt | 81940 | GGGGCUGGCUCCGAUGUAUUU | 817648 | AUACAUCGGAGCCAGCCCCUU | 817649 |
| MXI1 | NM_005962.2 | 0.14 | 920 | cacagcagcctgccgagtattgg | 33013 | CAGCAGCCUGCCGAGUAUUGG | 817650 | AAUACUCGGCAGGCUGCUGUG | 817651 |

INDUSTRIAL APPLICABILITY

In view of the foregoing, the polynucleotide of the present invention not only has a high RNA interference effect on its target gene, but also has a very small risk of causing RNA interference against a gene unrelated to the target gene, so that the polynucleotide of the present invention can cause RNA interference specifically only to the target gene whose expression is to be inhibited. Thus, the present invention is preferred for use in, e.g., tests and therapies using RNA interference, and is particularly effective in performing RNA interference in higher animals such as mammals, especially humans.

Incidentally, the sequence listing of the present application contains information on 817651 sequences. Its electronic file is too large in size (near 200 MB), making it difficult or impossible to handle the file depending on the computer environment used. Thus, the electric file was divided into two parts so that it became easier to handle. YCT1039 sequence listing (1) contains bibliographic data and information on SEQ ID NOs: 1 to 70000, while YCT1039 sequence listing (2) contains information on SEQ ID NOs: 700001 to 817651.

**Claims**

1. A polynucleotide for causing RNA interference against a target gene selected from the genes of a target organism, which has at least a double-stranded region,
   wherein one strand in the double-stranded region consists of a base sequence homologous to a prescribed sequence which is contained in the base sequences of the target gene and which conforms to the following rules (a) to (d):

   (a) The 3' end base is adenine, thymine or uracil;
   (b) The 5' end base is guanine or cytosine;
   (c) A 7-base sequence from the 3' end is rich in one or more types of bases selected from the group consisting of adenine, thymine and uracil; and
   (d) The number of bases is within a range that allows RNA interference to occur without causing cytotoxicity, and wherein the other strand in the double-stranded region consists of a base sequence having a sequence complementary to the base sequence homologous to the prescribed sequence.

2. The polynucleotide according to claim 1, wherein at least 80% of bases in the base sequence homologous to the prescribed sequence corresponds to the base sequence of the prescribed sequence.

3. The polynucleotide according to claim 1 or 2,
   wherein, in the rule (c), at least three bases among the seven bases are one or more types of bases selected from the group consisting of adenine, thymine and uracil.

4. The polynucleotide according to any one of claims 1 to 3, wherein, in the rule (d), the number of bases is 13 to 28.

5. The polynucleotide according to any one of claims 1 to 4, wherein the prescribed sequence further conforms to the following rule (e):

   (e) A sequence in which 10 or more bases of guanine or cytosine are continuously present is not contained.

6. The polynucleotide according to claim 5, wherein the prescribed sequence further conforms to the following rule (f):

   (f) A sequence sharing at least 90% homology with the prescribed sequence is not contained in the base sequences of genes other than the target gene among all gene sequences of the target organism.

7. The polynucleotide according to claim 6, wherein the prescribed sequence consists of the base sequence shown in any of SEQ ID NOs: 47 to 817081.

8. The polynucleotide according to claim 6, wherein the prescribed sequence is any of the sequences listed in the column "Target Sequence" of Figure 46.

9. The polynucleotide according to claim 6, which has any of the base sequences shown in SEQ ID NOs: 817102 to 817651.

**10.** The polynucleotide according to any one of claims 1 to 9, which is a double-stranded polynucleotide.

**11.** The polynucleotide according to claim 10, wherein
one strand of the double-stranded polynucleotide consists of a base sequence having an overhanging portion at the 3' end of the base sequence homologous to the prescribed sequence, and
the other strand of the double-stranded polynucleotide consists of a base sequence having an overhanging portion at the 3' end of the sequence complementary to the base sequence homologous to the prescribed sequence.

**12.** The polynucleotide according to any one of claims 1 to 9, which is a single-stranded polynucleotide having a hairpin structure,
wherein the single-stranded polynucleotide has a loop segment linking the 3' end of one strand in the double-stranded region and the 5' end of the other strand in the double-stranded region.

**13.** A method for selecting a polynucleotide to be introduced into an expression system for a target gene whose expression is to be inhibited,
wherein the polynucleotide has at least a double-stranded region,
wherein one strand in the double-stranded region consists of a base sequence homologous to a prescribed sequence which is contained in the base sequences of the target gene and which conforms to the following rules (a) to (f):

> (a) The 3' end base is adenine, thymine or uracil;
> (b) The 5' end base is guanine or cytosine;
> (c) A 7-base sequence from the 3' end is rich in one or more types of bases selected from the group consisting of adenine, thymine and uracil;
> (d) The number of bases is within a range that allows RNA interference to occur without causing cytotoxicity;
> (e) A sequence in which 10 or more bases of guanine or cytosine are continuously present is not contained; and
> (f) A sequence sharing at least 90% homology with the prescribed sequence is not contained in the base sequences of genes other than the target gene among all gene sequences of the target organism, and

wherein the other strand in the double-stranded region consists of a base sequence having a sequence complementary to the base sequence homologous to the prescribed sequence.

**14.** A method for selecting a polynucleotide according to claim 13, wherein a polynucleotide having a sequence, wherein the base sequence homologous to the prescribed sequence of the target gene contains mismatches of at least 3 bases against the base sequences of genes other than the target gene, and for which there is only a minimum number of other genes having a base sequence containing the mismatches of at least 3 bases, is further selected from the selected polynucleotides.

**15.** A method for inhibiting gene expression, which comprises introducing the polynucleotide according to any one of claims 1 to 12 into an expression system for a target gene whose expression is to be inhibited, thereby inhibiting the expression of the target gene.

**16.** A method for inhibiting gene expression, which comprises introducing a polynucleotide selected by the method according to claim 13 or 14 into an expression system for a target gene whose expression is to be inhibited, thereby inhibiting the expression of the target gene.

**17.** The method for inhibiting gene expression according to claim 15 or 16, wherein the expression is inhibited to 50% or below.

**18.** A pharmaceutical composition which comprises a pharmaceutically effective amount of the polynucleotide according to any one of claims 1 to 12.

**19.** The pharmaceutical composition according to claim 18, which is for use in treating or preventing the diseases listed in the column "Related Disease" of Figure 46.

**20.** The pharmaceutical composition according to claim 18, which is for use in treating or preventing diseases related to the genes listed in the column "Gene Name" of Figure 46.

**21.** The pharmaceutical composition according to claim 18, which is for use in treating or preventing a disease in which a gene belonging to any of the following 1) to 9) is involved:

1) an apoptosis-related gene;
2) phosphatase or a phosphatase activity-related gene;
3) a cell cycle-related gene;
4) a receptor-related gene;
5) an ion channel-related gene;
6) a signal transduction system-related gene;
7) kinase or a kinase activity-related gene;
8) a transcription regulation-related gene; or
9) G protein-coupled receptor or a G protein-coupled receptor-related gene.

**22.** The pharmaceutical composition according to any one of claims 18 to 21, which comprises a polynucleotide targeting the base sequence shown in any of SEQ ID NOs listed in the column "SEQ ID NO (human)" or "SEQ ID NO (mouse)" of Figure 46.

**23.** The pharmaceutical composition according to claim 18, which is for use in treating or preventing diseases related to the genes listed in the column "Gene Name" of Table 1.

**24.** The pharmaceutical composition according to claim 18 or 23, which is for use in treating or preventing any cancer selected from bladder cancer, breast cancer, colorectal cancer, gastric cancer, hepatoma, lung cancer, melanoma, ovarian cancer, pancreas cancer, prostate cancer, oral cancer, skin cancer, and thyroid gland cancer.

**25.** The pharmaceutical composition according to any one of claims 18, 23 or 24, which comprises a polynucleotide having any of the base sequences shown in SEQ ID NOs: 817102 to 817651.

**26.** A composition for inhibiting gene expression to inhibit the expression of a target gene, which comprises the polynucleotide according to any one of claims 1 to 12.

**27.** The composition for inhibiting gene expression according to claim 26, wherein the target gene is related to any of the diseases listed in the column "Related Disease" of Figure 46.

**28.** The composition for inhibiting gene expression according to claim 26, wherein the target gene is any of the genes listed in the column "Gene Name" of Figure 46.

**29.** The composition for inhibiting gene expression according to claim 26, wherein the target gene is a gene belonging to any of the following 1) to 9):

1) an apoptosis-related gene;
2) phosphatase or a phosphatase activity-related gene;
3) a cell cycle-related gene;
4) a receptor-related gene;
5) an ion channel-related gene;
6) a signal transduction system-related gene;
7) kinase or a kinase activity-related gene;
8) a transcription regulation-related gene; or
9) G protein-coupled receptor or a G protein-coupled receptor-related gene.

**30.** The composition for inhibiting gene expression according to claim 26, wherein the target gene is any of the genes listed in the column "Gene Name" of Table 1.

**31.** The composition for inhibiting gene expression according to claim 26, wherein the target gene is related to any cancer selected from bladder cancer, breast cancer, colorectal cancer, gastric cancer, hepatoma, lung cancer, melanoma, ovarian cancer, pancreas cancer, prostate cancer, oral cancer, skin cancer, and thyroid gland cancer.

**32.** A method for treating or preventing the diseases listed in the column "Related Disease" of Figure 46, which comprises administering a pharmaceutically effective amount of the polynucleotide according to any one of claims 1 to 12.

## Figure 1

Human
sequence    GACACGGACGTCAA<span style="background:black;color:white">CACCCTGACCCGCTTCGTCATGG</span>AGGAGGGCAGGAAGGCCCGCG
Mouse
sequence    GAAACGGATATCAG<span style="background:black;color:white">CACCCTGACCCGCTTCGTCATGG</span>AGCAGGGCAGGAAGGCTCAGG

            ** ***** *** ************************* ************* * *

                                    ↓

                    5'-CCCUGACCCGCUUCGUCAUGG-3'
                    3'-GUGGGACUGGGCGAAGCAGUA-5'

# Figure 2

```
         G or C                    A or T or U
            ↓                          ↓
    5'-CNNNNNNNNNNNNNNNNNNNNNUNN-3'
    3'-NNGNNNNNNNNNNNNNNNNNNNNA-5'
                              |←——————→|
                                AU rich
```

# Figure 3

NM_000507: *Homo sapiens* fructose-1,6-bisphosphatase 1 (*FBP1*)
NM_019395: *Mus musculus* fructose bisphosphatase 1 (*Fbp1*)

```
NM_000507    1 ATGGCTGACCAGGCGCCCTTCGACACGGACGTCAACACCCTGACCCGCTTCGTCATGGAG   60
NM_019395    1 ATGGCGAACCATGCGCCCTTCGAAACGGATATCAGCACCCTGACCCGCTTCGTCATGGAG   60
               *****  ****  *********** *****  *** ****************************

                                                         ▼intron
NM_000507  121 GCAGTCAAAGCCATCTCTTCGGCGGTGCGCAAGGCGGGCATCGCGCACCTCTATGGCATT  180
NM_019395  121 GCGGATCAAAGCCATCTCGTCTGCGGTGCGCGCCAGGCGGGCATCGCACAGCTCTATGGTATC  180
               **  ************** ** *********** ************** ** ********  **

NM_000507  181 GCTGGTTCTACCAACGTGACAGGTGATCAAGTTAAGAAGCTGGACGTCCTCTCCAACGAC  240
NM_019395  181 GCTGGCTCAACCAATGTGACTGGGGATCAAGTAAAGAAGCTGGACATACTTTCCAATGAC  240
               *****  ** ***** ***** **  ******** ************* *  ***** ***

NM_000507  241 CTGGTTATGAACATGTTAAAGTCATCCTTTGCCACGTGTGTTCTCGTGTCAGAAGAAGAT  300
NM_019395  241 CTGGTGATCAATATGCTGAAGTCGTCCTACGCTACCTGTGTGTTCTTGTGTCTGAAGAAAAC  300
               ***** ** ** *** * ***** ****  ** ** ********* ***** ****** *

                               ▼intron
NM_000507  301 AAACACGCCATCATAGTGGAACCGGAGAAAAAGGGGTAAATATGTGGTCTGTTTTGATCCC  360
NM_019395  301 ACAAATGCCATCATAATCGAACCTGAGAAGAGGGGGCAAATATGTTGTCTGTTTCGATCCC  360
               * * * ********** * ***** *****  ***** ******** ****** *****

NM_000507  361 CTTGATGGATCTTCCAACATCGATTGCCTTGTGTCCGTTGGAACCATTTTTGGCATCTAT  420
NM_019395  361 CTTGATGGCTCATCCAACATTGACTGCCTTGTGTCCATCGGAACCATTTTTGGCATTTAC  420
               ******** ** ******** ** ********** * ***************** **

                      ▼intron
NM_000507  421 AGAAAGAAATCAACTGATGAGCCTTCTGAGAAGGATGCTCTGCAACCAGGCCGGAACCTG  480
NM_019395  421 AGAAAGAAAAGTACTGATGAGCCTTCTGAGAAGGATGCTCTGCAGCCCGGCCGGGACCTG  480
               *********  ********************************* ** ******  *****

NM_000507  481 GTGGCAGCCGGCTACGCACTGTATGGCAGTGCCACCATGCTGGTCCTTGCCATGGACTGT  540
NM_019395  481 GTGGCAGCCGGGTATGCGCTCTATGGCAGTGCCACCATGTTGGTCCTTGCCATGGATTGT  540
               *********** **  ** ** ***************** ************** ***

                             ▼intron
NM_000507  541 GGGGTCAACTGCTTCATGCTGGACCCGGCCATCGGGGAGTTCATTTTGGTGGACAAGGAT  600
NM_019395  541 GGTGTCAACTGCTTCATGCTGGACCCGTCCATTGGAGAATTCATTATGGTGGACAGGGAC  600
               ** ***********************  **** ** ** ** ****** ******** ***

                                                         ▼intron
NM_000507  601 GTGAAGATAAAAAAGAAAGGTAAAATCTACAGCCTTAACGAGGGCTACGCCAGGGACTTT  660
NM_019395  601 GTGAAGATGAAGAAGAAAGGTAACATCTACAGCCTTAATGAGGGTTATGCCAAGGACTTT  660
               ******** ** *********** ************* ***** ** **** *******

                                   ▼intron
NM_000507  661 GACCCTGCCGTCACTGAGTACATCCAGAGGAAGAAGTTCCCCCCAGATAATTCAGCTCCT  720
NM_019395  661 GACCCTGCCATCAATGAGTATCTCCAGAGGAAAAAGTTCCCTCCGGATGGTTCAGCCCCC  720
               ********* *** ***** ********** ******** ** ***  ****** **

NM_000507  721 TATGGGGCCCGGTATGTGGGCTCCATGGTGGCTGATGTTCATCGCACTCTGGTCTACGGA  780
NM_019395  721 TATGGTGCCCGGTATGTGGGGTCCATGGTGGCTGATATTCACCGCACTCTGGTATATGGA  780
               *****  ************** ************** **** ********** ** **

                                   ▼intron
NM_000507  781 GGGATATTTCTGTACCCCGCTAACAAGAAGAGCCCCAATGGAAAGCTGAGACTGCTGTAC  840
NM_019395  781 GGGATCTTTTTATACCCCGCCAACAAGAAAAGCCCAAGTGGAAAGCTGCGGCTGCTGTAT  840
               ***** *** * ******** ******** ***** * ********* * ********

NM_000507  841 GAATGCAACCCCATGGCCTACGTCATGGAGAAGGCTGGGGGAATGGCCACCACTGGGAAG  900
NM_019395  841 GAGTGCAACCCCATAGCTTATGTCATGGAGAAGGCCGGTGGGCTCGCCACCACGGGGGAC  900
               ** ********** ** ** ************** ** **  * ********* *** *

NM_000507  901 GAGGCCGTGTTAGACGTCATTCCCACAGACATTCACCAGAGGGCGCCGGTGATCTTGGGA  960
NM_019395  901 AAAGATATATTAGACATCGTTCCCACCGAGATCCACCAGAAGGCACCAGTCGTCATGGGG  960
               * *   * ****** ** ******** ** ** ******* *** ** ** ** ** ***

NM_000507  961 TCCCCCGACGACGTGCTCGAGTTCCTGAAGGTGTATGAGAAGCACTCTGCCCAGTGA    1017
NM_019395  961 TCCTCTGAAGATGTGCAGGAGTTCCTGGAGATCTACAGGAAGCACAAAGCCAAGTGA    1017
               *** * ** ** ** ****  ********** ** * ** ****** *** *****
```

## Figure 4

```
NM_000507:  36  caccctgacccgcttcgtcatgg
NM_000507:  37  accctgacccgcttcgtcatgga
NM_000507:  38  ccctgacccgcttcgtcatggag
NM_000507:433  actgatgagccttctgagaagga
NM_000507:434  ctgatgagccttctgagaaggat
NM_000507:435  tgatgagccttctgagaaggatg
NM_000507:436  gatgagccttctgagaaggatgc
NM_000507:437  atgagccttctgagaaggatgct
NM_000507:438  tgagccttctgagaaggatgctc
NM_000507:439  gagccttctgagaaggatgctct
NM_000507:440  agccttctgagaaggatgctctg
NM_000507:441  gccttctgagaaggatgctctgc
NM_000507:442  ccttctgagaaggatgctctgca
NM_000507:544  gtcaactgcttcatgctggaccc
NM 000507:545  tcaactgcttcatgctggacccg
```

## Figure 5

```
NM_000507:  36  caccctgacccgcttcgtcatgg  1 1 4 4
NM_000507:  37  accctgacccgcttcgtcatgga  0 1 3 0
NM_000507:  38  ccctgacccgcttcgtcatggag  0 1 3 0
NM_000507:433  actgatgagccttctgagaagga  0 0 4 0
NM_000507:434  ctgatgagccttctgagaaggat  0 1 3 0
NM_000507:435  tgatgagccttctgagaaggatg  1 0 4 0
NM_000507:436  gatgagccttctgagaaggatgc  1 0 4 0
NM_000507:437  atgagccttctgagaaggatgct  0 1 4 0
NM_000507:438  tgagccttctgagaaggatgctc  0 0 3 0
NM_000507:439  gagccttctgagaaggatgctct  1 1 3 0
NM_000507:440  agccttctgagaaggatgctctg  0 1 3 0
NM_000507:441  gccttctgagaaggatgctctgc  1 1 4 4
NM_000507:442  ccttctgagaaggatgctctgca  0 0 3 0
NM_000507:544  gtcaactgcttcatgctggaccc  0 1 2 0
NM_000507:545  tcaactgcttcatgctggacccg  0 0 2 0
```

## Figure 6

EP 1 752 536 A1

| Sequences producing significant alignments: | Score (bits) | E Value |
|---|---|---|
| ref\|NM_019395.1\| Mus musculus fructose bisphosphatase 1 (Fbp1), ... | 46 | 9e-06 |
| ref\|NM_000507.2\| Homo sapiens fructose-1,6-bisphosphatase 1 (FBP... | 46 | 9e-06 |
| ref\|NM_015820.1\| Mus musculus heparan sulfate 6-O-sulfotransfera... | 30 | 0.52 |
| ref\|NM_003837.1\| Homo sapiens fructose-1,6-bisphosphatase 2 (FBP... | 30 | 0.52 |
| ref\|NM_145960.1\| Mus musculus hypothetical protein MGC19099 (MGC... | 28 | 2.0 |
| ref\|NM_010884.1\| Mus musculus N-myc downstream regulated 1 (Ndr1... | 28 | 2.0 |
| ref\|NM_025405.1\| Mus musculus RIKEN cDNA 1110033J19 gene (111003... | 26 | 8.1 |
| ref\|NM_029098.1\| Mus musculus RIKEN cDNA 1110013E13 gene (111001... | 26 | 8.1 |
| ref\|NM_012258.2\| Homo sapiens hairy/enhancer-of-split related wi... | 26 | 8.1 |
| ref\|NM_018113.1\| Homo sapiens lipocalin-interacting membrane rec... | 26 | 8.1 |
| ref\|NM_014780.1\| Homo sapiens KIAA0076 gene product (KIAA0076), ... | 26 | 8.1 |
| ref\|NM_012218.1\| Homo sapiens interleukin enhancer binding facto... | 26 | 8.1 |
| ref\|NM_004516.1\| Homo sapiens interleukin enhancer binding facto... | 26 | 8.1 |
| ref\|NM_000875.2\| Homo sapiens insulin-like growth factor 1 recep... | 26 | 8.1 |
| ref\|NM_001188.1\| Homo sapiens BCL2-antagonist/killer 1 (BAK1), mRNA | 26 | 8.1 |

# Figure 7

| Sequences producing significant alignments: | Score (bits) | E Value |
|---|---|---|
| ref\|NM_019395.1\| Mus musculus fructose bisphosphatase 1 (Fbp1), ... | 46 | 9e-06 |
| ref\|NM_000507.2\| Homo sapiens fructose-1,6-bisphosphatase 1 (FBP... | 46 | 9e-06 |
| ref\|NM_007994.1\| Mus musculus fructose bisphosphatase 2 (Fbp2), ... | 36 | 0.008 |
| ref\|NM_139045.1\| Homo sapiens SWI/SNF related, matrix associated... | 32 | 0.13 |
| ref\|NM_003070.2\| Homo sapiens SWI/SNF related, matrix associated... | 32 | 0.13 |
| ref\|NM_022026.1\| Mus musculus aquaporin 9 (Aqp9), mRNA | 30 | 0.52 |
| ref\|NM_011834.1\| Mus musculus kynurenine aminotransferase II (Ka... | 30 | 0.52 |
| ref\|NM_008632.1\| Mus musculus microtubule-associated protein 2 (... | 30 | 0.52 |
| ref\|NM_023266.1\| Mus musculus RIKEN cDNA 1200003I07 gene (120000... | 30 | 0.52 |
| ref\|NM_144499.1\| Homo sapiens guanine nucleotide binding protein... | 30 | 0.52 |
| ref\|NM_000172.2\| Homo sapiens guanine nucleotide binding protein... | 30 | 0.52 |
| ref\|NM_015125.2\| Homo sapiens capicua homolog (Drosophila) (CIC)... | 30 | 0.52 |
| ref\|NM_146249.1\| Mus musculus hypothetical protein MGC18735 (MGC... | 28 | 2.0 |
| ref\|NM_027017.1\| Mus musculus RIKEN cDNA 3300002I08 gene (330000... | 28 | 2.0 |
| ref\|NM_145490.1\| Mus musculus similar to zinc finger protein 97 ... | 28 | 2.0 |
| ref\|NM_145528.1\| Mus musculus similar to Hypothetical protein KI... | 28 | 2.0 |
| ref\|NM_144546.1\| Mus musculus expressed sequence AL024077 (AL024... | 28 | 2.0 |
| ref\|NM_144852.1\| Mus musculus hypothetical protein MGC27672 (MGC... | 28 | 2.0 |
| ref\|NM_080467.1\| Mus musculus ATPase, H+ transporting, lysosomal... | 28 | 2.0 |
| ref\|NM_029813.1\| Mus musculus RIKEN cDNA 2210418O10 gene (221041... | 28 | 2.0 |
| ref\|NM_029583.1\| Mus musculus RIKEN cDNA 2810408B13 gene (281040... | 28 | 2.0 |
| ref\|NM_001231.2\| Homo sapiens calsequestrin 1 (fast-twitch, skel... | 28 | 2.0 |
| ref\|NM_020824.1\| Homo sapiens Rho-GTPase activating protein 10 (... | 28 | 2.0 |

··· [Following 78 lines are omitted]

# Figure 8

```
NM_000507:  36 caccctgacccgcttcgtcatgg 1 1 4 4 59
NM_000507:  37 accctgacccgcttcgtcatgga 0 1 3 0 -
NM_000507:  38 ccctgacccgcttcgtcatggag 0 1 3 0 -
NM_000507:433 actgatgagccttctgagaagga 0 0 4 0 -
NM_000507:434 ctgatgagccttctgagaaggat 0 1 3 0 -
NM_000507:435 tgatgagccttctgagaaggatg 1 0 4 0 -
NM_000507:436 gatgagccttctgagaaggatgc 1 0 4 0 -
NM_000507:437 atgagccttctgagaaggatgct 0 1 4 0 -
NM_000507:438 tgagccttctgagaaggatgctc 0 0 3 0 -
NM_000507:439 gagccttctgagaaggatgctct 1 1 3 0 -
NM_000507:440 agccttctgagaaggatgctctg 0 1 3 0 -
NM_000507:441 gccttctgagaaggatgctctgc 1 1 4 4 1708
NM_000507:442 ccttctgagaaggatgctctgca 0 0 3 0 -
NM_000507:544 gtcaactgcttcatgctggaccc 0 1 2 0 -
NM 000507:545 tcaactgcttcatgctggacccg 0 0 2 0 -
```

EP 1 752 536 A1

## Figure 9

**A**    Firefly *luciferase* gene

siRNA ATGGAAGACGCCAAAACATAAAGAAAGGCCCGGCGCCATTCTATCCGCTGGAAGATGGAACCGCTGGAGAGCAACTGCAT

a
b
c
d
e
f
g
h
i

ACACCCGAGGGGGATGATAAACCGGGCGCGGTCGGTAAAGTTGTTCCATTTTTTGAAGCGAAGGTTG

j
k
l

CGATGACGCCGGTGAACTTCCCGCCGCCGTTGTTGTTTTGGAGCACGG

m
n
o
p

Figure 10

EP 1 752 536 A1

Figure 11

**D**

(base)

| 2 | 7 | 5 | 7 | 2 |
| OH | 3'-T | M | 5'-T | |

G/C      A/U

A/U rich

| RLA* | siRNA | G/C content (%) | | | | |
|------|-------|-----|------|-----|------|-------|
| | | OH | 3'-T | M | 5'-T | Total |
| 0.03 | a | UC | U-57 | 20 | 14-U | 32 |
| 0.03 | l | AG | C-43 | 20 | 29-A | 32 |
| 0.04 | f | CG | G-29 | 80 | 43-U | 47 |
| 0.09 | k | GG | C-100 | 80 | 14-A | 63 |
| 0.23 | o | AG | G-100 | 60 | 14-A | 58 |
| | | | 66 | 52 | 23 | 46 |
| 0.46 | n | AA | G-100 | 60 | 29-A | 63 |
| 0.50 | p | GG | G-100 | 40 | 29-C | 58 |
| 0.52 | e | GG | G-100 | 20 | 57-A | 63 |
| 0.52 | j | GG | C-29 | 60 | 100-C | 68 |
| 0.66 | i | UC | U-43 | 80 | 57-U | 58 |
| | | | 74 | 52 | 54 | 62 |
| 0.69 | g | GA | U-57 | 60 | 43-G | 53 |
| 0.76 | d | CU | U-71 | 100 | 29-U | 63 |
| 0.92 | m | GG | C-57 | 60 | 100-C | 74 |
| 0.92 | h | CG | A-43 | 40 | 86-C | 58 |
| 0.92 | b | GU | U-14 | 20 | 71-G | 37 |
| 0.93 | c | GU | A-29 | 80 | 100-G | 63 |
| | | | 45 | 60 | 72 | 58 |

EP 1 752 536 A1

## Figure 12

(BASIC PRINCIPLE OF PRESENT INVENTION)

BASE SEQUENCE INFORMATION
OF TARGET GENE

⇩

CREATION OF PARTIAL
BASE SEQUENCE — S-1

⇩

PARTIAL BASE
SEQUENCE INFORMATION

| S-2 | S-3 | S-4 |
|---|---|---|
| DETERMINATION OF 3' END BASE | DETERMINATION OF 5' END BASE | DETERMINATION OF INCLUSION OF PREDETERMINED BASE |
| ⇩ | ⇩ | ⇩ |
| DETERMINATION RESULT | DETERMINATION RESULT | DETERMINATION RESULT |

PRESCRIBED
SEQUENCE SELECTION — S-5

⇩

PRESCRIBED
SEQUENCE INFORMATION

## Figure 13

INPUT UNIT ⟜112    OUTPUT UNIT ⟜114

⟜100

BASE SEQUENCE
PROCESSING APPARATUS    ⟜106

INPUT-OUTPUT CONTROL INTERFACE    ⟜108

⟜104

⟜102

MEMORY

| TARGET GENE BASE SEQUENCE FILE | ⟜106a |

| PARTIAL BASE SEQUENCE FILE | ⟜106b |

| DETERMINATION RESULT FILE | ⟜106c |

| PRESCRIBED SEQUENCE FILE | ⟜106d |

| REFERENCE SEQUENCE DATABASE | ⟜106e |

| DEGREE OF IDENTITY OR SIMILARITY FILE | ⟜106f |

| EVALUATION RESULT FILE | ⟜106g |

| TARGET GENE ANNOTATION DATABASE | ⟜106h |

CONTROLLER

| PARTIAL BASE SEQUENCE CREATION PART | ⟜102a |

| 3' END BASE DETERMINATION PART | ⟜102b |

| 5' END BASE DETERMINATION PART | ⟜102c |

| PREDETERMINED BASE INCLUSION DETERMINATION PART | ⟜102d |

| PRESCRIBED SEQUENCE SELECTION PART | ⟜102e |

| OVERHANGING PORTION-ADDING PART | ⟜102f |

| IDENTICAL/SIMILAR BASE SEQUENCE SEARCH PART | ⟜102g |

| UNRELATED GENE TARGET EVALUATION PART | ⟜102h |

COMMUNICATION CONTROL INTERFACE

⟜200

EXTERNAL SYSTEM

EXTERNAL DB

EXTERNAL PROGRAM

NETWORK
⟜300

EP 1 752 536 A1

## Figure 14

| TARGET BASE SEQUENCE FILE 106a | |
|---|---|
| BASE SEQUENCE IDENTIFICATION INFORMATION | BASE SEQUENCE INFORMATION |
| NM_000507 | ATGGCTGA ··· AGTGA |
| ⋮ | ⋮ |

## Figure 15

| PARTIAL BASE SEQUENCE FILE 106b | | |
|---|---|---|
| PARTIAL BASE SEQUENCE IDENTIFICATION INFORMATION | PARTIAL BASE SEQUENCE INFORMATION | INFORMATION ON INCLUSION OF OVERHANGING PORTION |
| NM_000507:36 | caccct ··· tcatgg | INCLUDED |
| ⋮ | ⋮ | ⋮ |

Figure 16

DETERMINATION RESULT FILE

106c

| PARTIAL BASE SEQUENCE IDENTIFICATION INFORMATION | DETERMINATION RESULT ON 3' END BASE | DETERMINATION RESULT ON 5' END BASE | DETERMINATION RESULT ON INCLUSION OF PREDETERMINED BASE | COMPREHENSIVE DETERMINATION RESULT |
|---|---|---|---|---|
| NM_000507:36 | 1 | 1 | 4 | 4 |
| ⋮ | ⋮ | ⋮ | ⋮ | ⋮ |

EP 1 752 536 A1

# Figure 17

PRESCRIBED SEQUENCE FILE

⌐106d

| PARTIAL BASE SEQUENCE IDENTIFICATION INFORMATION | PRESCRIBED SEQUENCE INFORMATION |
|---|---|
| NM_000507:36 | caccct ⋯ tcatgg |
| ⋮ | ⋮ |

# Figure 18

REFERENCE SEQUENCE DATABASE

⌐106e

| REFERENCE SEQUENCE IDENTIFICATION INFORMATION | REFERENCE BASE SEQUENCE INFORMATION |
|---|---|
| ref\|NM_015820.1\| | caccct ⋯ gcatgg |
| ⋮ | ⋮ |

# Figure 19

| DEGREE OF IDENTITY OR SIMILARITY FILE 106f | | |
| --- | --- | --- |
| PARTIAL BASE SEQUENCE IDENTIFICATION INFORMATION | PRESCRIBED IDENTIFICATION INFORMATION | DEGREE OF IDENTITY OR SIMILARITY |
| NM_000507:36 | ref\|NM_015820.1\| | 0.52 |
| | ref\|NM_003837.1\| | 0.52 |
| ⋮ | ⋮ | ⋮ |

# Figure 20

| EVALUATION RESULT FILE 106g | | |
| --- | --- | --- |
| PARTIAL BASE SEQUENCE IDENTIFICATION INFORMATION | REFERENCE SEQUENCE IDENTIFICATION INFORMATION | EVALUATION INFORMATION |
| NM_000507:36 | 5.9 | NONTARGET |
| NM_000507:441 | 170.8 | TARGET |
| ⋮ | ⋮ | ⋮ |

# Figure 21

<sub>(</sub>102a

PARTIAL BASE SEQUENCE CREATION PART

REGION-SPECIFIC BASE
SEQUENCE CREATION PART — 102i

COMMON BASE
SEQUENCE CREATION PART — 102j

OVERHANGING PORTION-CONTAINING
BASE SEQUENCE CREATION PART — 102k

# Figure 22

<sub>(</sub>102h

UNRELATED GENE TARGET EVALUATION PART

TOTAL SUM
CALCULATION PART — 102m

TOTAL SUM-BASED
EVALUATION PART — 102n

# Figure 23

(MAIN PROCESSING)

START

CREATION OF PARTIAL
BASE SEQUENCE INFORMATION — SA-1

DETERMINATION OF 3' END BASE IN
PARTIAL BASE SEQUENCE INFORMATION — SA-2

DETERMINATION OF 5' END BASE IN
PARTIAL BASE SEQUENCE INFORMATION — SA-3

DETERMINATION OF BASE SEQUENCE
INFORMATION COMPRISING
AT LEAST 7 BASES AT 3' END IN
PARTIAL BASE SEQUENCE INFORMATION — SA-4

SELECTION OF PRESCRIBED SEQUENCE
INFORMATION BASED ON DETERMINATION
RESULTS OF SA-2 TO SA-4 — SA-5

END

# Figure 24

(UNRELATED GENE TARGET EVALUATION PROCESSING)

START

CALCULATION OF TOTAL SUM OF
RECIPROCALS OF VALUES SHOWING
DEGREE OF IDENTITY OR SIMILARITY — SB-1

EVALUATION OF PRESCRIBED SEQUENCE — SB-2
INFORMATION BASED ON TOTAL SUM

END

## Figure 25

## Figure 26

## Figure 27

## Figure 28

```
5' -    aggauguucggcggcccgggc -3'
3' - cguccuacaagccgccgggcc    -5'
```

## Figure 29

```
5' -    guacgucagcaauaugaaagu -3'
3' - ugcaugcagucguuauacuuu    -5'
```

## Figure 30

```
5'-    CAUUCUAUCCGCUGGAAGAUG -3'
3'- CGGUAAGAUAGGCGACCUUCU    -5'
```

## Figure 31

## Figure 32

# Figure 33

# Figure 34

Figure 35

Figure 36

EP 1 752 536 A1

## Influence of mismatches on RNAi effect
## (TREC assay)

| siRNA sequence | Target sequence | | luc mRNA/ control mRNA (Neo) 0 0.2 0.4 0.6 0.8 1 | Number of mismatches |
|---|---|---|---|---|
| GCCATTCTATCCGCTGGAAGATG | 3-36 | gccattctatccgctggaagatg | | 0 |
| GCCATTCTATCCGCTGGAAGATG | 3-36.R1 | gccattctatccgcGggCGgatg | | 3 |
| GCCATTCTATCCGCTGGAAGATG | 3-36.R2 | gccattctatccgcCggGGgatg | | 3 |
| GCCATTCTATCCGCTGGAAGATG | 3-36.R3 | gccattctatccgcGggaCgatg | | 2 |
| GCCATTCTATCCGCTGGAAGATG | 3-36.R4 | gccattctatccgctggCGgatg | | 2 |
| GCCATTCTATCCGCTGGAAGATG | 3-36.R5 | gccattctatccgctggaGgatg | | 1 |
| GCCATTCTATCCGCTGGAAGATG | 3-36.R6 | gccattctatccgctgTaaTatg | | 2 |
| GCCATTCTATCCGCTGGAAGATG | 3-36.R7 | gccattctatccgctAAaagatg | | 2 |
| GCCATTCTATCCGCTGGAAGATG | 3-36.R8 | gccattctatccgctATaaAatg | | 3 |
| GCCATTCTATCCGCTGGAAGATG | 3-36.L1 | gccGGCcCGtccgctggaagatg | | 5 |
| GCCATTCTATCCGCTGGAAGATG | 3-36.L2 | gccCGtcCGtccgctggaagatg | | 4 |
| GCCATTCTATCCGCTGGAAGATG | 3-36.L3 | gccGtCctGtccgctggaagatg | | 3 |
| GCCATTCTATCCGCTGGAAGATG | 3-36.L4 | gccaCCcGatccgctggaagatg | | 3 |
| GCCATTCTATCCGCTGGAAGATG | 3-36.L5 | gccattAtatccgctggaagatg | | 1 |
| GCCATTCTATCCGCTGGAAGATG | 3-36.01A | gcAattctatccgctggaagatg | | 1 |
| GCCATTCTATCCGCTGGAAGATG | 3-36.01G | gcGattctatccgctggaagatg | | 1 |
| GCCATTCTATCCGCTGGAAGATG | 3-36.01U | gcTattctatccgctggaagatg | | 1 |
| GCCATTCTATCCGCTGGAAGATG | 3-36.19G | gccattctatccgctggaaGtg | | 1 |
| GCCATTCTATCCGCTGGAAGATG | 3-36.19C | gccattctatccgctggaaCtg | | 1 |
| GCCATTCTATCCGCTGGAAGATG | 3-36.19U | gccattctatccgctggaaTtg | | 1 |

Mismatch=0
Mismatch=1
Mismatch=2
Mismatch≧3

*Figure 37*

Relationship between apoptosis-related genes and GO_ID in Gene Ontology

0006917, 0001915, 0001916, 0001914, 0001913, 0006915, 0043276, 0042981, 0043066, 0006916, 0045476, 0045477, 0045849, 0045850, 0043065, 0001781, 0045767, 0019987, 0045768, 0019050, 0008624, 0008626, 0008628, 0008627, 0008625, 0008629, 0008630, 0042771, 0008631, 0019051, 0006920, 0006918, 0016506, 0008189, 0016329

*Figure 38*

Relationship between phosphatase-encoding genes and GO_ID in Gene Ontology

0007185, 0045208, 0045209, 0019902, 0019903, 0008157, 0030346, 0042153, 0003998, 0019176,
0016462, 0043135, 0047624, 0047631, 0019144, 0008715, 0047734, 0000810, 0008486, 0047874,
0000298, 0004309, 0008894, 0009678, 0004427, 0050072, 0017110, 0043262, 0004382, 0004787,
0045134, 0017111, 0008094, 0004011, 0008186, 0004010, 0043273, 0050333, 0048253, 0050339,
0047429, 0047693, 0047840, 0004170, 0004551, 0047710, 0008796, 0004081, 0008803, 0047884,
0000210, 0047430, 0004636, 0050355, 0008413, 0008768, 0050380, 0050351, 0008893, 0050108,
0050210, 0016791, 0016302, 0047538, 0050532, 0019143, 0047572, 0003869, 0003993, 0042131,
0047630, 0004035, 0047647, 0019175, 0004063, 0004083, 0047765, 0019203, 0003850, 0004331,
0004330, 0042132, 0008877, 0004346, 0004805, 0047846, 0047873, 0047386, 0050530, 0000121,
0047954, 0043136, 0047383, 0004401, 0004437, 0016312, 0004441, 0030487, 0046030, 0017161,
0004445, 0008934, 0030351, 0030352, 0004446, 0016314, 0042577, 0016316, 0004438, 0016315,
0001668, 0004439, 0050084, 0050531, 0047382, 0050115, 0050124, 0019178, 0019204, 0046403,
0004651, 0008195, 0004606, 0008962, 0050189, 0050192, 0008967, 0008969, 0004721, 0004722,
0004723, 0008596, 0008420, 0004724, 0000163, 0008598, 0017018, 0017019, 0000158, 0008600,
0030357, 0030358, 0015071, 0030360, 0030361, 0009400, 0004725, 0004726, 0004727, 0030946,
0030945, 0004728, 0005001, 0019198, 0008138, 0008579, 0017017, 0008330, 0004647, 0017120,
0019173, 0050278, 0050286, 0042392, 0050301, 0050307, 0050308, 0050309, 0050340, 0047385,
0050406, 0050407, 0047384, 0050196, 0004741, 0019906, 0050408, 0047862, 0019211, 0019888,
0008160, 0042030, 0004858, 0019212, 0004864, 0004865, 0001691, 0019208, 0008597, 0017020,
0008599, 0008601, 0030359, 0030362, 0019909, 0004442, 0004443, 0004444, 0016313, 0017021,
0004440, 0005081, 0005082, 0045206, 0045207, 0019910, 0045253, 0045249, 0005963, 0008287,
0005955, 0017023, 0030289, 0000164, 0000159

*Figure 39*

Relationship between cell cycle-related genes and GO_ID in Gene Ontology

0030996, 0007050, 0000074, 0007049, 0007113, 0051330, 0000080, 0000114, 0007089, 0000081, 0051331, 0000085, 0000116, 0051328, 0051332, 0051329, 0000082, 0000083, 0000079, 0045786, 0045787, 0000086, 0000117, 0000216, 0000084, 0000115, 0051327, 0000087, 0000075, 0051321, 0000278, 0045448, 0035185, 0007347, 0046001, 0045976, 0046002, 0045977, 0009794, 0007346, 0045930, 0046003, 0007348, 0045931, 0046004, 0035186, 0045749, 0007090, 0045750, 0000751, 0030571, 0000077, 0006977, 0000320, 0000321, 0019055, 0046792, 0046615, 0003750

## Figure 40

Relationship between receptor-encoding genes and GO_ID in Gene Ontology

0007176, 0007175, 0007177, 0045741, 0045877, 0045878, 0043109, 0007226, 0015464, 0030594,
0004888, 0016907, 0008227, 0004981, 0001873, 0008329, 0001874, 0004978, 0004977, 0008188,
0042654, 0004930, 0004980, 0004952, 0001588, 0001590, 0001589, 0001591, 0001670, 0001592,
0001593, 0001607, 0008528, 0004979, 0004946, 0004951, 0015054, 0035237, 0004966, 0042263,
0004983, 0001602, 0001601, 0016492, 0035236, 0004994, 0004995, 0016498, 0016497, 0016496,
0004872, 0008503, 0001646, 0001644, 0016502, 0001877, 0001875, 0016019, 0001653, 0016941,
0001584, 0001605, 0008261, 0004942, 0004943, 0004944, 0001595, 0001596, 0004945, 0004947,
0001598, 0004962, 0001599, 0001600, 0001637, 0004950, 0016493, 0016494, 0004918, 0004907,
0016495, 0001606, 0035013, 0004982, 0004985, 0004986, 0004987, 0004988, 0015051, 0016499,
0001648, 0015057, 0001604, 0001603, 0004990, 0005000, 0045015, 0005046, 0005051, 0005052,
0005053, 0050839, 0001857, 0004875, 0001847, 0004877, 0001861, 0004878, 0001858, 0004876,
0001860, 0001859, 0004904, 0004896, 0004905, 0004906, 0004908, 0004909, 0004910, 0004920,
0004921, 0016517, 0016515, 0016518, 0042010, 0042012, 0030368, 0042008, 0042014, 0004911,
0042016, 0001532, 0042018, 0042020, 0045506, 0045507, 0045508, 0045509, 0004912, 0004913,
0004914, 0004915, 0004917, 0004919, 0005031, 0005032, 0005033, 0005035, 0005030, 0019766,
0019763, 0019767, 0019768, 0019769, 0019770, 0019771, 0019772, 0001793, 0001792, 0008262,
0005041, 0008032, 0030228, 0001863, 0008029, 0008030, 0001565, 0005024, 0017002, 0016361,
0005025, 0016362, 0005026, 0009885, 0009884, 0008288, 0005003, 0005004, 0005005, 0005006,
0005023, 0008313, 0005007, 0005008, 0005009, 0005010, 0005011, 0005017, 0005018, 0005019,
0005020, 0005021, 0042096, 0004892, 0005029, 0005028, 0008529, 0005045, 0008530, 0004928,
0004929, 0042099, 0019764, 0015029, 0019765, 0008019, 0005012, 0005013, 0005014, 0005015,
0005016, 0005027, 0004927, 0004894, 0050785, 0016964, 0030226, 0030227, 0004874, 0015026,
0008036, 0030373, 0008095, 0042071, 0004879, 0004880, 0003708, 0004886, 0003707, 0004882,
0004884, 0030284, 0004883, 0017082, 0004887, 0008434, 0001566, 0009881, 0009882, 0008020,
0004975, 0009883, 0004873, 0008046, 0005042, 0005043, 0001647, 0005040, 0045125, 0001619,
0016526, 0001620, 0001625, 0001629, 0001628, 0001630, 0001623, 0001624, 0001622, 0001638,
0001633, 0001645, 0001643, 0008067, 0008066, 0001640, 0001641, 0001642, 0004965, 0016917,
0001639, 0016503, 0004932, 0004933, 0004934, 0004936, 0004937, 0004938, 0004939, 0004940,
0004941, 0015052, 0004969, 0004989, 0004993, 0001585, 0001586, 0001587, 0016609, 0001594,
0008226, 0004949, 0004953, 0004954, 0004955, 0016501, 0004959, 0004956, 0004957, 0004958,
0001785, 0004960, 0004961, 0001627, 0004974, 0001631, 0001632, 0008502, 0001626, 0001608,
0045028, 0001614, 0001609, 0008501, 0001610, 0001611, 0001612, 0001613, 0045032, 0045031,
0015065, 0045029, 0045030, 0004984, 0004992, 0001621, 0016500, 0004963, 0004968, 0004964,
0004976, 0030273, 0004996, 0008527, 0001616, 0001617, 0004997, 0001635, 0004948, 0001636,
0015056, 0016519, 0015058, 0016523, 0004967, 0016520, 0004991, 0001634, 0016522, 0015055,
0004999, 0004890, 0004970, 0004971, 0015277, 0004972, 0004897, 0004900, 0004901, 0030525,
0016167, 0004902, 0030524, 0004903, 0004923, 0004924, 0004925, 0030369, 0005055, 0016524,
0030229, 0030106, 0030108, 0030107, 0030109, 0030110, 0045012, 0045233, 0030379, 0004926,
0008158, 0042813, 0001571, 0005049, 0030376, 0005044, 0017154, 0008281, 0005056, 0004998,
0030377, 0016931, 0019041, 0001618, 0008196, 0005484

*Figure 41*

Relationship between ion channel-encoding genes and GO_ID in Gene Ontology

0045161, 0045163, 0045162, 0005254, 0005216, 0005253, 0005224, 0005217, 0008068, 0005234, 0016934, 0016933, 0004891, 0005237, 0019182, 0005229, 0005247, 0005244, 0015274, 0015257, 0015482, 0008308, 0005225, 0005261, 0015251, 0004931, 0005231, 0005227, 0015276, 0005262, 0015278, 0005220, 0005218, 0005219, 0015279, 0015275, 0008381, 0005245, 0010173, 0015270, 0008331, 0008086, 0008332, 0015252, 0005221, 0005222, 0005223, 0004889, 0016904, 0015281, 0015249, 0005267, 0015269, 0016286, 0005228, 0005249, 0005251, 0005242, 0015272, 0015467, 0015273, 0005252, 0015271, 0005250, 0005232, 0005272, 0015280, 0005248, 0005230, 0030171, 0015282, 0008282, 0019183, 0005891, 0008087, 0008076, 0001518, 0017071, 0016935

## Figure 42

Relationship between signal transduction system-related genes and GO_ID
in Gene Ontology

0007165, 0007166, 0009966, 0009968, 0009967, 0000750, 0007330, 0030454, 0000757, 0007234,
0007227, 0000160, 0007263, 0007264, 0016601, 0035020, 0051056, 0035021, 0051058, 0035022,
0051057, 0031291, 0007265, 0046578, 0046580, 0046579, 0035024, 0035023, 0035025, 0007266,
0001402, 0000077, 0042770, 0006977, 0030330, 0006976, 0008630, 0042771, 0006978, 0042772,
0008269, 0050851, 0050853, 0050859, 0050861, 0050854, 0050858, 0050860, 0050857, 0050862,
0050856, 0050852, 0019221, 0007167, 0007168, 0007178, 0030509, 0030510, 0030514, 0030513,
0007179, 0008101, 0007169, 0048013, 0007173, 0008314, 0042058, 0042059, 0045742, 0008543,
0040036, 0040037, 0045743, 0048012, 0008286, 0046626, 0046627, 0046628, 0048009, 0048011,
0048008, 0045501, 0045873, 0045874, 0048014, 0008293, 0048010, 0030947, 0030948, 0030949,
0007185, 0007186, 0007212, 0042699, 0007214, 0007216, 0007218, 0007211, 0008277, 0045744,
0045745, 0007210, 0007217, 0007215, 0035235, 0007229, 0007219, 0030179, 0008593, 0045746,
0045747, 0007231, 0007232, 0008589, 0045879, 0045880, 0008063, 0008592, 0045751, 0045752,
0016055, 0007222, 0008590, 0045810, 0045811, 0008591, 0045812, 0045813, 0030111, 0030178,
0030177, 0009785, 0010019, 0009870, 0010204, 0006984, 0009873, 0010104, 0010105, 0030522,
0048384, 0048385, 0048387, 0048386, 0030518, 0030521, 0035076, 0030520, 0042921, 0050847,
0009867, 0009864, 0010017, 0010018, 0010161, 0009863, 0009862, 0031098, 0042700

## Figure 43-1

Relationship between kinase or kinase activity-related genes and GO_ID
in Gene Ontology

0007178, 0007169, 0007171, 0007205, 0001789, 0042313, 0007233, 0045860, 0009747, 0009748,
0006987, 0007243, 0007249, 0007250, 0043122, 0043124, 0043123, 0000187, 0007257, 0000186,
0007255, 0007256, 0000185, 0042655, 0007254, 0031098, 0000079, 0045859, 0045736, 0006469,
0045737, 0009764, 0045204, 0006130, 0007251, 0007170, 0006224, 0043127, 0043126, 0043128,
0019900, 0019901, 0008432, 0051019, 0048273, 0042808, 0051018, 0005080, 0030971, 0005115,
0008579, 0017017, 0050408, 0004611, 0004612, 0030585, 0004613, 0003848, 0016301, 0008728,
0019205, 0050148, 0004749, 0019200, 0004788, 0047506, 0047507, 0050515, 0008698, 0047590,
0047336, 0047601, 0008776, 0003991, 0047620, 0004020, 0047628, 0047633, 0047649, 0047650,
0019202, 0004054, 0018110, 0035173, 0004072, 0004349, 0004413, 0047666, 0047667, 0047700,
0047758, 0047761, 0047764, 0008804, 0008671, 0008673, 0008787, 0019150, 0008865, 0004335,
0004340, 0004396, 0004454, 0009702, 0008737, 0045127, 0009384, 0008443, 0008662, 0003873,
0003872, 0008993, 0004747, 0008741, 0004856, 0008744, 0004103, 0008819, 0004111, 0047814,
0017050, 0047841, 0004140, 0047848, 0004143, 0047333, 0008842, 0004168, 0047878, 0004305,
0047887, 0047900, 0030387, 0050201, 0047912, 0046316, 0047931, 0047940, 0047944, 0008887,
0004370, 0004371, 0047973, 0047976, 0009927, 0004417, 0047992, 0008902, 0008904, 0047715,
0008906, 0004428, 0004430, 0001727, 0019140, 0035299, 0035300, 0050517, 0050516, 0047325,
0047326, 0008440, 0016307, 0000285, 0016308, 0045215, 0016309, 0035005, 0035004, 0016303,
0046934, 0008910, 0001729, 0008481, 0001728, 0050059, 0050073, 0019158, 0004496, 0003951,
0042736, 0019206, 0004001, 0019136, 0004136, 0004137, 0004138, 0004797, 0000816, 0004849,
0004550, 0046899, 0019201, 0004017, 0004127, 0004385, 0004798, 0009041, 0050145, 0050154,
0050165, 0004594, 0018720, 0050190, 0050191, 0004618, 0008972, 0004631, 0050195, 0008974,
0046404, 0008976, 0008980, 0050222, 0004672, 0047696, 0019912, 0019913, 0030295, 0035174,
0004674, 0035175, 0035184, 0050063, 0004676, 0004679, 0009931, 0004683, 0004685, 0004684,
0004686, 0004688, 0016905, 0004687, 0004689, 0008606, 0004680, 0004681, 0004682, 0008604,
0004690, 0004691, 0008602, 0004692, 0004693, 0016537, 0004677, 0004694, 0004703, 0004678,
0004695, 0004696, 0008384, 0004697, 0004700, 0004698, 0004699, 0004701, 0004702, 0004707,
0008338, 0016908, 0016909, 0004705, 0008339, 0004709, 0004706, 0004710, 0008349, 0042656,
0004704, 0042801, 0004675, 0019199, 0004711, 0008353, 0004712, 0004708, 0008545, 0004673,
0008896, 0008256, 0008257, 0009784, 0009885, 0004713, 0004715, 0004716, 0004718, 0004714,
0050254, 0050321, 0050359, 0050225, 0008478, 0004743, 0008986, 0008531, 0050262, 0046522,
0050276, 0050277, 0004756, 0004765, 0050299, 0050300, 0050316, 0050317, 0009024, 0050324,
0009029, 0019165, 0050331, 0009030, 0050354, 0009038, 0050394, 0050400, 0047323, 0050405,
0047322, 0008772, 0004740, 0050369, 0050521, 0050242, 0016536, 0019209, 0019207, 0019887,
0016534, 0016535, 0042557, 0042556, 0030296, 0030298, 0030297, 0019210, 0004860, 0008427,
0004862, 0004861, 0008426, 0004863, 0030291, 0030292, 0030294, 0030293, 0035014, 0046935,

*Figure 43-2*

Relationship between kinase or kinase activity-related genes and GO_ID
in Gene Ontology


0008603, 0019914, 0016538, 0003751, 0003752, 0003753, 0008607, 0008605, 0004429, 0004431,
0004432, 0004433, 0008438, 0004717, 0005077, 0004384, 0017135, 0016304, 0016305, 0016306,
0015072, 0015073, 0005079, 0016910, 0016911, 0016912, 0017027, 0001566, 0001571, 0030377,
0005078, 0005076, 0008268, 0005071, 0005066, 0005068, 0005069, 0000307, 0000308, 0019908,
0005945, 0008385, 0005942, 0035030, 0035031, 0035032, 0016533, 0019907, 0005956, 0005943,
0005944, 0005954, 0005952, 0005958, 0005959, 0005964, 0009358, 0009365

*Figure 44*

Relationship between transcription regulation-related genes and GO_ID
in Gene Ontology

0045990, 0006355, 0045013, 0045892, 0045014, 0046015, 0045991, 0045893, 0046016, 0007221,
0051091, 0007228, 0006994, 0035104, 0051092, 0030969, 0006990, 0043193, 0045944, 0042994,
0000114, 0006357, 0000116, 0000083, 0000117, 0000115, 0051037, 0051038, 0051039, 0007072,
0007073, 0007074, 0007075, 0045896, 0007068, 0007069, 0016479, 0046017, 0007070, 0000122,
0046021, 0007071, 0016480, 0046023, 0045897, 0046018, 0045943, 0046022, 0046024, 0045945,
0006356, 0006359, 0006342, 0016440, 0048096, 0000716, 0006283, 0016441, 0035194, 0016481,
0045449, 0045996, 0009373, 0046020, 0046019, 0045816, 0006358, 0017055, 0045898, 0045894,
0007532, 0045941, 0007357, 0007360, 0007363, 0009371, 0007329, 0045817, 0045899, 0045895,
0050434, 0046782, 0051090, 0051123, 0009300, 0006351, 0016552, 0016551, 0006350, 0009299,
0042789, 0006366, 0019083, 0019084, 0019085, 0019086, 0006362, 0006368, 0006385, 0009303,
0042791, 0006383, 0042792, 0006390, 0042794, 0042793, 0042790, 0006360, 0009302, 0009301,
0042795, 0042796, 0006393, 0006353, 0006391, 0006352, 0006361, 0006363, 0019223, 0006367,
0006369, 0019224, 0030846, 0030847, 0006384, 0006386, 0019225, 0042797, 0009304, 0006410,
0016245, 0042992, 0042990, 0042993, 0042991, 0006340, 0045891, 0045889, 0006339, 0045888,
0045890, 0006988, 0006989, 0019056, 0006978, 0042772, 0003700, 0000130, 0030528, 0003705,
0003702, 0008134, 0003712, 0003713, 0030374, 0003714, 0003719, 0017163, 0003701, 0016251,
0003703, 0016455, 0016252, 0003704, 0003706, 0003709, 0030401, 0016986, 0016988, 0003715,
0003716, 0003717, 0003718, 0016563, 0003710, 0042156, 0003711, 0008148, 0008159, 0016943,
0016944, 0016945, 0016564, 0016565, 0016566, 0000120, 0005667, 0005672, 0005669, 0005673,
0005674, 0005675, 0035101, 0008023, 0008024, 0005671, 0030907, 0000126, 0000127, 0017053,
0000346, 0019036, 0005670

*Figure 45*

Relationship between GPCR or GPCR-related genes and GO_ID
in Gene Ontology

0007186, 0007189, 0007193, 0007187, 0007188, 0007190, 0007199, 0007200, 0030265, 0001789,
0008277, 0045744, 0045745, 0035104, 0006992, 0035103, 0006993, 0035105, 0007201, 0045761,
0016907, 0004930, 0008528, 0016492, 0001646, 0001644, 0001637, 0001664, 0004703, 0004678,
0005065, 0003927, 0000263, 0000264, 0000265, 0008020, 0004975, 0001647, 0016526, 0001629,
0008067, 0004933, 0001584, 0016609, 0001627, 0001608, 0045028, 0001609, 0008501, 0001610,
0001611, 0001612, 0001613, 0001633, 0030379, 0004926, 0016299, 0015467, 0015273, 0005834

Figure 46

| NO. | Gene Name | refseq_NO. | Target Position | Target Sequence | SEQ ID NO (human) | SEQ ID NO (mouse) | Biological Function category | Reported Biological Function | Related Disease |
|---|---|---|---|---|---|---|---|---|---|
| 1 | A2M | NM_000014.3 | 4299 | ttgataaggtgtcaaatcagaca | 127274 | 663769 | - | receptor | Alzheimer disease |
| 2 | ADSL | NM_000026.1 | 165 | ttgcctatcacagatgaacaaat | 234456 | 618797 | - | adenylosuccinate lyase | autism |
| 3 | ADSL | NM_000026.1 | 422 | tcggcttgccgactttgctaagg | 234462 | 618803 | see also 2 line | | |
| 4 | ALAS2 | NM_000032.1 | 1168 | tggggagcgtgatggaattatgc | 216068 | 603406 | - | general RNA polymerase II transcription factor | sideroblastic anemia |
| 5 | ABCD1 | NM_000033.2 | 1644 | cacgagatgttccaggtatttga | 70788 | 492568 | - | peroxisomal membrane transporter | adrenoleukodystrophy |
| 6 | ANK1 | NM_000037.2 | 4951 | gacgacacagtggattcagatgc | 286543 | 682368 | signal_transduction | - | spherocytosis |
| 7 | APC | NM_000038.2 | 44 | tgcagcttcatatgatcagttgt | 127993 | 530214 | signal_transduction、cell_cycle | beta-catenin binding | thyroid cancer、colorectal cancer、hepatoblastoma、adenomatous polyposis of the colon、cerebral astrocytoma、colorectal cancers、adrenocortical cancer、hepatocellular cancer、squamous cell cancer、familial nervous system tumor syndromes、familial adenomatous polyposis、gastric cancer |
| 8 | APC | NM_000038.2 | 128 | ttccaatcatcttacaaaactgg | 127997 | 530216 | see also 7 line | | |
| 9 | APC | NM_000038.2 | 993 | ttgtcaatgcttggtactcatga | 128014 | 530240 | see also 7 line | | |
| 10 | APC | NM_000038.2 | 1187 | cagtgcagcactccacaacatca | 128018 | 530245 | see also 7 line | | |
| 11 | APC | NM_000038.2 | 1898 | tacttaccggagccagacaaaca | 128044 | 530257 | see also 7 line | | |
| 12 | APC | NM_000038.2 | 1960 | atgtgtccagcttgatagctaca | 128048 | 530261 | see also 7 line | | |
| 13 | APC | NM_000038.2 | 2847 | ttcactaagtcggaaaattcaaa | 128069 | 530283 | see also 7 line | | |
| 14 | APC | NM_000038.2 | 4969 | ttgaagggacacctataaacttt | 128148 | 530338 | see also 7 line | | |
| 15 | APC | NM_000038.2 | 7025 | ttcccctggtagaaatggaataa | 128197 | 530399 | see also 7 line | | |
| 16 | AR | NM_000044.2 | 3598 | ttgatgaacttcgaatgaactac | 907 | 441709 | receptor_acitivity、transcription_regulator、signal_transduction | steroid binding | androgen insensitivity syndrome、breast cancer、androgen insensitivity、prostate cancer、spinal and bulbar muscular atrophy |
| 17 | ASPA | NM_000049.1 | 890 | tcctaatctgcaggatcaagact | 203366 | 712815 | - | aspartoacylase | Canavan disease |
| 18 | ATM | NM_000051.2 | 1660 | acctttcgtggtataagttctga | 21913 | 455385 | transcription_regulator、kinase_related、signal_transduction、cell_cycle | - | breast cancer、non-hodgkin lymphoma、ataxia telangiectasia、leukemia、lymphoma |
| 19 | ATM | NM_000051.2 | 7802 | atgaagtcctcaataatctaatc | 22090 | 455578 | see also 18 line | | |

Figure 46

| | | | | | | | | | |
|---|---|---|---|---|---|---|---|---|---|
| 20 | ATM | NM_000051.2 | 9184 | gaccagagtttcaacaaagtagc | 22121 | 455632 | see also 18 line | | |
| 21 | ATP7A | NM_000052.1 | 2179 | tgcaactcttcaccataatcaaa | 73448 | 495029 | - | plasma membrane cation-transporting ATPase | Menkes syndrome |
| 22 | ATP7A | NM_000052.1 | 2654 | gtggatgtggaacttgtacaacg | 73463 | 495049 | see also 21 line | | |
| 23 | ATP7A | NM_000052.1 | 2667 | ttgtacaacgtggagatatcatt | 73464 | 495050 | see also 21 line | | |
| 24 | ATP7A | NM_000052.1 | 3105 | ttgctttccaagcctctatcaca | 73481 | 495062 | see also 21 line | | |
| 25 | ATP7A | NM_000052.1 | 3940 | ttctcagtttggcattactaagg | 73500 | 495096 | see also 21 line | | |
| 26 | ATP7B | NM_000053.1 | 202 | gccagtcggaaaatcttatctaa | 73523 | 495119 | - | plasma membrane cation-transporting ATPase | Wilson disease |
| 27 | ATP7B | NM_000053.1 | 204 | cagtcggaaaattctatcctaagc | 73524 | 495120 | see also 26 line | | |
| 28 | ATP7B | NM_000053.1 | 1923 | gaggacaaatggcatcacttatg | 73555 | 495147 | see also 26 line | | |
| 29 | BCHE | NM_000055.1 | 1925 | aacgattacactagcaagaaaga | 199935 | 591637 | - | serine esterase | hypocholinesterasemia(-), butyrylcholinesterase deficiency, pseudocholinesterase deficiency, Alzheimer disease |
| 30 | BLM | NM_000057.1 | 2067 | ctgcataatttagaactaatca | 22788 | 456445 | - | ATP dependent DNA helicase | Bloom syndrome |
| 31 | BLM | NM_000057.1 | 2430 | aggaagctcttggcacgttttgt | 22797 | 456458 | see also 30 line | | |
| 32 | BLM | NM_000057.1 | 2943 | gacgtgcgatttgtgattcatgc | 22820 | 456477 | see also 30 line | | |
| 33 | BLM | NM_000057.1 | 3773 | tggtgtccattacttcaatatt | 22848 | 456502 | see also 30 line | | |
| 34 | BRCA2 | NM_000059.1 | 8051 | caggtgtggatccaaagcttatt | 19386 | 452248 | transcription_regulator, cell_cycle | USF43/USF44 complex | breast cancer, Fanconi anemia, Li-Fraumeni syndrome, ovarian cancer, pancreatic cancer, ocular melanoma |
| 35 | BTD | NM_000060.1 | 1574 | ctgggagaatgaccacactattcc | 204000 | 594332 | - | hydrolase, acting on carbon-nitrogen (but not peptide) bonds | multiple carboxylase deficiency, biotinidase deficiency |
| 36 | BTK | NM_000061.1 | 322 | gggttcaatagatgttgagaaga | 74370 | 496474 | kinase_related, apoptosis | protein tyrosine kinase | Bruton's agammaglobulinemia, Bruton agammaglobulinemia |
| 37 | BTK | NM_000061.1 | 645 | atgctatgggctgccaaattttg | 74375 | 496486 | see also 36 line | | |
| 38 | BTK | NM_000061.1 | 646 | tgctatgggctgccaaatttggg | 74376 | 496487 | see also 36 line | | |
| 39 | BTK | NM_000061.1 | 1731 | cagtctgaaacgtttggtaaac | 74396 | 496517 | see also 36 line | | |
| 40 | CACNA1A | NM_000068.2 | 898 | gtgtctcggaatccaagtttaca | 23493 | 456941 | channel, transcription regulator | - | episodica ataxia, familial periodic cerebellar ataxia, hemiplegic migraine, paroxysmal cerebellar ataxia, spinocerebellar ataxia |
| 41 | CACNA1A | NM_000068.2 | 903 | tggaatccaagtttacaagtcg | 23494 | 456942 | see also 40 line | | |
| 42 | CACNA1A | NM_000068.2 | 1867 | ctctttagrtccgaaatgttat | 23518 | 456962 | see also 40 line | | |

Figure 46

| 43 | CACNA1A | NM_000068.2 | 5867 | ctctcggcttaggcaagaaatgt | 23572 | 456997 | see also 40 line | | |
|---|---|---|---|---|---|---|---|---|---|
| 44 | CACNA1A | NM_000068.2 | 5869 | ctcggcttaggcaagaaatgtcc | 23573 | 456998 | see also 40 line | | |
| 45 | CBS | NM_000071.1 | 439 | cacccctatggtcagaatcaaca | 233577 | 618008 | - | cystathione beta-synthase | atherosclerosis、homocystinuria |
| 46 | CBS | NM_000071.1 | 441 | cccctatggtcagaatcaacaag | 233578 | 618009 | see also 45 line | | |
| 47 | CBS | NM_000071.1 | 442 | ccctatggtcagaatcaacaaga | 233579 | 618010 | see also 45 line | | |
| 48 | CD36 | NM_000072.1 | 327 | ttggatctttgatgtgcaaaatc | 247777 | 634387 | receptor_acitivity | receptor | atherosclerosis、platelet glycoprotein IV deficiency |
| 49 | CHRNE | NM_000080.2 | 953 | cgccacgctcattgtcatgaatt | 179197 | 572412 | channel、receptor_acitivity、signal_transduction | nicotinic acetylcholine-activated cation-selective channel | - |
| 50 | CHRNE | NM_000080.2 | 954 | gccacgctcattgtcatgaattg | 179198 | 572413 | see also 49 line | | |
| 51 | CHS1 | NM_000081.1 | 6245 | ttcaccctcctactaatacttac | 268177 | 651748 | signal_transduction | protein transporter | Chediak-Higashi syndrome |
| 52 | CHS1 | NM_000081.1 | 7098 | atgctgtggattatatgaactcc | 268207 | 651774 | see also 51 line | | |
| 53 | CHS1 | NM_000081.1 | 7327 | gccaaccagttgtatcttcatcg | 268213 | 651783 | see also 51 line | | |
| 54 | CHS1 | NM_000081.1 | 9250 | atcagtgttgcaccatctagaga | 268288 | 651830 | see also 51 line | | |
| 55 | CLCN1 | NM_000083.1 | 1305 | cccaccaggaatgggtcaattca | 263225 | 646852 | channel | voltage-gated chloride channel | Becker's disease、Thomsen's disease |
| 56 | CLCN5 | NM_000084.1 | 320 | gtgtagggacctatgatgatttc | 263357 | 646992 | channel | voltage-gated chloride channel | Fanconi syndrome、nephrolithiasis |
| 57 | CLCN5 | NM_000084.1 | 324 | agggacctatgatgatttcaata | 263359 | 646993 | see also 56 line | | |
| 58 | CLCN5 | NM_000084.1 | 386 | ggcaccgagagattaccaataaa | 263363 | 646995 | see also 56 line | | |
| 59 | CLCN5 | NM_000084.1 | 417 | gtcaacatgggccttaattcaca | 263365 | 646999 | see also 56 line | | |
| 60 | CLCN5 | NM_000084.1 | 966 | gtgccactgcttcaacaaataca | 263379 | 647013 | see also 56 line | | |
| 61 | CLCN5 | NM_000084.1 | 1831 | gtgactcggatgactgtttctct | 263398 | 647036 | see also 56 line | | |
| 62 | CLCN5 | NM_000084.1 | 1833 | gactcggatgactgtttctcttg | 263399 | 647037 | see also 56 line | | |
| 63 | CLCN5 | NM_000084.1 | 1981 | ctcaatggatacccctttcttga | 263404 | 647040 | see also 56 line | | |
| 64 | CLCN5 | NM_000084.1 | 2081 | tccttactcaggacagtatgact | 263406 | 647041 | see also 56 line | | |
| 65 | CLCN5 | NM_000084.1 | 2460 | taccaaaaaggatgtgttaaagc | 263410 | 647051 | see also 56 line | | |
| 66 | CNGA1 | NM_000087.1 | 813 | agggtggaactatccagaaatta | 111100 | 520711 | channel | potassium channel | retinitis pigmentosa |
| 67 | COL1A2 | NM_000089.2 | 3521 | cagacccaaggactatgaagttg | 288067 | 659376 | - | translation regulator | lipoblastoma、Ehlers-Danlos syndrome、osteogenesis imperfecta |
| 68 | COL5A1 | NM_000093.2 | 913 | caccttgatcctcgactgtaaaa | 177479 | 659682 | - | heparin binding | Ehlers-Danlos syndrome |
| 69 | CYP19A1 | NM_000103.2 | 811 | tggcaagctctcctcatcaaacc | 221666 | 608596 | - | - | breast cancer、gynecomastia、head and neck squamous cell carcinoma |
| 70 | DMD | NM_000109.2 | 1192 | cccctaagcctcgattcaagagc | 116679 | 521655 | - | - | cardiomyopathy、muscular dystrophy |
| 71 | DMD | NM_000109.2 | 1590 | atgaatctcctaaattcaagatg | 116688 | 521669 | see also 70 line | | |
| 72 | DMD | NM_000109.2 | 4112 | ttgggcatgttggcatgagttat | 116748 | 521728 | see also 70 line | | |

EP 1 752 536 A1

## Figure 46

| 73 | DMD | NM_000109.2 | 4114 | gggcatgttggcatgagttattg | 116749 | 521730 | see also 70 line | | |
|---|---|---|---|---|---|---|---|---|---|
| 74 | DMD | NM_000109.2 | 4364 | ttctcgttggagggaactacatg | 116757 | 521739 | see also 70 line | | |
| 75 | DMD | NM_000109.2 | 5154 | gcctggggaaaggctactcaaaa | 116780 | 521773 | see also 70 line | | |
| 76 | DMD | NM_000109.2 | 5159 | gggaaaggctactcaaaaagaga | 116781 | 521774 | see also 70 line | | |
| 77 | DMD | NM_000109.2 | 5950 | atcagtggtatcagtacaagagg | 116800 | 521802 | see also 70 line | | |
| 78 | DMD | NM_000109.2 | 6213 | cagtttcgaagactcaactttgc | 116804 | 521809 | see also 70 line | | |
| 79 | DMD | NM_000109.2 | 6725 | ttgggaacatgctaaatacaaat | 116820 | 521829 | see also 70 line | | |
| 80 | DMD | NM_000109.2 | 6726 | tgggaacatgctaaatacaaatg | 116821 | 521830 | see also 70 line | | |
| 81 | DMD | NM_000109.2 | 8066 | aacacaatggctggaagctaagg | 116852 | 521863 | see also 70 line | | |
| 82 | DMD | NM_000109.2 | 8640 | ctgttacaaagacgtttggataa | 116863 | 521870 | see also 70 line | | |
| 83 | DMD | NM_000109.2 | 8648 | aagacgtttggataacatgaact | 116864 | 521871 | see also 70 line | | |
| 84 | DMD | NM_000109.2 | 8888 | aactaaagaacctgtaatcatga | 116869 | 521877 | see also 70 line | | |
| 85 | DMD | NM_000109.2 | 9896 | gaggatccgtgtcctgtctttta | 116887 | 521885 | see also 70 line | | |
| 86 | DMD | NM_000109.2 | 9897 | aggatccgtgtcctgtcttttaa | 116888 | 521886 | see also 70 line | | |
| 87 | DMD | NM_000109.2 | 10270 | aagagtgtccaatcattggattc | 116895 | 521900 | see also 70 line | | |
| 88 | DMD | NM_000109.2 | 10560 | aacttctggccagtagattctgc | 116902 | 521914 | see also 70 line | | |
| 89 | DMD | NM_000109.2 | 10665 | aacagcaatggatcttatctaaa | 116906 | 521916 | see also 70 line | | |
| 90 | DMD | NM_000109.2 | 10707 | gagagcatagatgatgaacattt | 116907 | 521918 | see also 70 line | | |
| 91 | DMD | NM_000109.2 | 11206 | tccgagtggttggcagtcaaact | 116913 | 521927 | see also 70 line | | |
| 92 | DMD | NM_000109.2 | 11209 | gagtggttggcagtcaaacttcg | 116914 | 521928 | see also 70 line | | |
| 93 | DPYD | NM_000110.2 | 1174 | gagtgttcatcgtcttcagaaaa | 224018 | 611224 | - | electron transfer flavoprotein | 5-fluorouracil toxicity、dihydropyrimidine dehydrogenase deficiency |
| 94 | DPYD | NM_000110.2 | 2231 | gaccccaaatgtcactgatattg | 224049 | 611253 | see also 93 line | | |
| 95 | SLC26A2 | NM_000112.1 | 1175 | ctgtagatgcaatagctatttcc | 260084 | 643684 | - | sulfate porter | achondrogenesis、atelosteogenesis |
| 96 | DYT1 | NM_000113.1 | 895 | atgcagtcccgaggctatgaaat | 81116 | 501038 | - | heat shock protein | dystonia、alcoholism |
| 97 | EDNRB | NM_000115.1 | 941 | ctgaagccataggttttgatata | 176744 | 624199 | receptor_acitivity、gpcr | - | Hirschsprung disease、Waardenburg-Hirschsprung disease |
| 98 | ERCC5 | NM_000123.1 | 1065 | atcttgataaaaggtattcaagc | 19497 | 452330 | transcription_regulator | single-stranded DNA binding | xeroderma pigmentosum |
| 99 | ETFA | NM_000126.1 | 422 | ctgacacatttgtgagaactatt | 264770 | 648989 | - | electron transfer flavoprotein | leukemia、ethylmalonic-adipicaciduria、glutaricaciduria、multiple acyl-CoA dehydrogenase deficiency (MADD) |
| 100 | EXT1 | NM_000127.1 | 973 | ttgggatcaattggaaaacgagg | 213291 | 601643 | signal_transduction、cell_cycle | transferase、transferring glycosyl groups | osteochondroma、chondrosarcoma、hereditary multiple exostoses、multiple exostoses、trichorhinophalangeal syndrome、osteosarcoma |
| 101 | EXT1 | NM_000127.1 | 1071 | tggagtcctgcttcgatttcacc | 213292 | 601647 | see also 100 line | | |

Figure 46

| | | | | | | | | | |
|---|---|---|---|---|---|---|---|---|---|
| 102 | EXT1 | NM_000127.1 | 1143 | gggagaaaatcgccgaaagttac | 213296 | 601648 | see also 100 line | | |
| 103 | EXT1 | NM_000127.1 | 1283 | gtgcacaatttgagatccaaagt | 213299 | 601653 | see also 100 line | | |
| 104 | EXT1 | NM_000127.1 | 1711 | cagagacaacaccgagtatgaga | 213305 | 601662 | see also 100 line | | |
| 105 | EXT1 | NM_000127.1 | 1792 | caggcttgggtccttcagattcc | 213308 | 601665 | see also 100 line | | |
| 106 | EXT1 | NM_000127.1 | 1951 | caggtctattcatcaggataaaa | 213315 | 601674 | see also 100 line | | |
| 107 | EXT1 | NM_000127.1 | 2000 | ttcttgtgggaggcttatttttc | 213319 | 601676 | see also 100 line | | |
| 108 | EXT1 | NM_000127.1 | 2033 | aagattgtattaactacactaga | 213322 | 601677 | see also 100 line | | |
| 109 | EXT1 | NM_000127.1 | 2078 | aagcacatatcacgtaacagttt | 213324 | 601679 | see also 100 line | | |
| 110 | EXT1 | NM_000127.1 | 2079 | agcacatatcacgtaacagttta | 213325 | 601680 | see also 100 line | | |
| 111 | EXT1 | NM_000127.1 | 2081 | cacatatcacgtaacagtttaat | 213326 | 601681 | see also 100 line | | |
| 112 | EXT1 | NM_000127.1 | 2166 | ttccttactactatgctaattta | 213329 | 601689 | see also 100 line | | |
| 113 | EXT1 | NM_000127.1 | 2171 | tactactatgctaatttaggttt | 213330 | 601691 | see also 100 line | | |
| 114 | EXT1 | NM_000127.1 | 2174 | tactatgctaatttaggtttaaa | 213331 | 601692 | see also 100 line | | |
| 115 | F13A1 | NM_000129.2 | 564 | gtggggaaattccgcatgtatgt | 143366 | 736130 | - | protein-glutamine gamma-glutamyltransferase | - |
| 116 | F5 | NM_000130.2 | 1056 | tgcaggcttacattgacataaa | 162592 | 560209 | - | copper binding | thrombophlebitis、parahemophilia |
| 117 | F8 | NM_000132.2 | 1036 | ttcctcgaaggtcacacatttct | 162745 | 560360 | - | - | - |
| 118 | F8 | NM_000132.2 | 1806 | tcggtgcctgacccgctattact | 162770 | 560402 | see also 117 line | | |
| 119 | F8 | NM_000132.2 | 2073 | cagcatcaatggctatgtttttg | 162776 | 560408 | see also 117 line | | |
| 120 | FANCA | NM_000135.1 | 1617 | gactctacgaggatttgtcatca | 301347 | 706954 | - | - | aplastic anemia、Fanconi anemia、Fanconi pancytopenia |
| 121 | FANCC | NM_000136.1 | 1826 | ttcttgaccagaccttgtacaga | 301422 | 669813 | - | - | aplastic anemia、Fanconi anemia、Fanconi pancytopenia |
| 122 | FBN1 | NM_000138.2 | 1176 | agctgccacagtccataaccaaa | 144242 | 548411 | - | extracellular matrix structural constituent | Marfan syndrome |
| 123 | FBN1 | NM_000138.2 | 1499 | gtccgctatctctgtcaaaatgg | 144256 | 548419 | see also 122 line | | |
| 124 | FBN1 | NM_000138.2 | 6681 | atgtgattggaggttttgaatgc | 144381 | 548501 | see also 122 line | | |
| 125 | FBN1 | NM_000138.2 | 7485 | ctcccaaaccctgcaattttatc | 144394 | 548519 | see also 122 line | | |
| 126 | FECH | NM_000140.1 | 785 | gagaagcgaggtggtcattctgt | 164259 | 618850 | - | Smad3/Sp-1 heterodimer | protoporphyria erythropoietic |
| 127 | FGFR2 | NM_000141.2 | 1571 | ctctatattcggaatgtaacttt | 82241 | 502302 | receptor_acitivity、kinase_related | - | acrocephalosyndactyly、Apert syndrome、Beare-Stevenson cutis gyrata syndrome(-)、bladder cancer、craniofacial dysostosis、Jackson-Weiss syndrome、Pfeiffer syndrome |
| 128 | FH | NM_000143.2 | 157 | ctcgaatggcaagccaaaattcc | 233545 | 617971 | cell_cycle | fumarate hydratase | fumarase deficiency、herefitay multiple leiomyoma、renal cell cancer、fumaricaciduria |
| 129 | FH | NM_000143.2 | 251 | gagatctacgatgaactttaaga | 233554 | 617978 | see also 128 line | | |

Figure 46

| | | | | | | | | | |
|---|---|---|---|---|---|---|---|---|---|
| 130 | FH | NM_000143.2 | 254 | atctacgatgaactttaagattg | 233555 | 617979 | see also 128 line | | |
| 131 | FH | NM_000143.2 | 406 | atgaggtagctgaaggtaaatta | 233560 | 617983 | see also 128 line | | |
| 132 | GAMT | NM_000156.4 | 447 | ctgcctgacggtcactttgatgg | 214060 | 604097 | - | - | guanidinoacetate N-methyltransferase deficiency |
| 133 | GBE1 | NM_000158.1 | 738 | atgcaatgtactaccaagaatca | 202019 | 592961 | - | transferase、 transferring glycosyl groups | adult polyglucosan body disease(-)、Andersen disease |
| 134 | GBE1 | NM_000158.1 | 1877 | cacaggcctacgtgagtgaaaaa | 202062 | 593009 | see also 133 line | | |
| 135 | GBE1 | NM_000158.1 | 1879 | caggcctacgtgagtgaaaaaca | 202063 | 593010 | see also 133 line | | |
| 136 | GJB1 | NM_000166.2 | 515 | cactgtggtggacctatgtcatc | 263784 | 647383 | - | connexon channel | Charcot-Marie-Tooth peroneal muscular atrophy、deafness、Charcot-Marie-Tooth neuropathy |
| 137 | GJB1 | NM_000166.2 | 837 | ccgcctctcacctgaatacaagc | 263786 | 647388 | see also 136 line | | |
| 138 | GLI3 | NM_000168.2 | 1648 | atgttggtagtgcatatgagaag | 4481 | 459713 | transcription_regulator 、signal_transduction | transcription factor | Greig craniopolysyndactyly syndrome、Pallister-Hall syndrome、postaxial polydactyly |
| 139 | GLI3 | NM_000168.2 | 2208 | ccccatcagcaactattccaaca | 4488 | 459719 | see also 138 line | | |
| 140 | GLI3 | NM_000168.2 | 2357 | acccggcagggaccaaatggatg | 4492 | 459721 | see also 138 line | | |
| 141 | GLA | NM_000169.1 | 445 | ctgaagctagggatttatgcaga | 201967 | 593256 | - | alpha-galactosidase | angiokeratoma、Fabry dystopic lipidosis、lipidosis |
| 142 | GLDC | NM_000170.1 | 394 | ttggggctggcgagcattgatga | 225945 | 502985 | - | glycine dehydrogenase (decarboxylating) | - |
| 143 | GLDC | NM_000170.1 | 848 | tccagactcgagccaaatatact | 225956 | 502994 | see also 142 line | | |
| 144 | NR3C1 | NM_000176.1 | 776 | cagacctgttgatagatgaaaac | 8493 | 447229 | transcription_regulator 、receptor_acitivity、signal_transduction | zinc binding | cortisol resistance from glucocorticoid receptor defect |
| 145 | NR3C1 | NM_000176.1 | 990 | ctgcacccctggggtaattaagc | 8501 | 447234 | see also 144 line | | |
| 146 | NR3C1 | NM_000176.1 | 1550 | gcccagcatgccgctatcgaaaa | 8512 | 447245 | see also 144 line | | |
| 147 | NR3C1 | NM_000176.1 | 1551 | cccagcatgccgctatcgaaaat | 8513 | 447246 | see also 144 line | | |
| 148 | NR3C1 | NM_000176.1 | 1553 | cagcatgccgctatcgaaaatgt | 8514 | 447247 | see also 144 line | | |
| 149 | NR3C1 | NM_000176.1 | 1557 | atgccgctatcgaaaatgtcttc | 8515 | 447248 | see also 144 line | | |
| 150 | NR3C1 | NM_000176.1 | 2265 | ctggcagcggttttatcaactga | 8530 | 447264 | see also 144 line | | |
| 151 | GSN | NM_000177.3 | 1454 | tggaggcgacagctacatcattc | 113915 | 523090 | - | structural constituent of cytoskeleton | bladder transitional cell carcinoma、amyloidosis |
| 152 | GSN | NM_000177.3 | 1459 | gcgacagctacatcattctgtac | 113916 | 523092 | see also 151 line | | |
| 153 | MSH6 | NM_000179.1 | 3691 | atgcatgcaacagcacattctct | 16801 | 451734 | - | damaged DNA binding | colorectal carcinoma、colorectal cancer、ataxia telangiectasia、endometrial cancer)、colorectal cancers、gastric cancer |
| 154 | MSH6 | NM_000179.1 | 3886 | atggcatgcatggtagaaaatga | 16807 | 451742 | see also 153 line | | |

Figure 46

| | | | | | | | | | |
|---|---|---|---|---|---|---|---|---|---|
| 155 | HADHA | NM_000182.3 | 129 | tgctgaccagaacccatattaac | 215297 | 614219 | - | short-chain enoyl-CoA hydratase | long chain 3-hydroxyacyl-CoA dehydrogenase (LCHAD) deficiency、trifunctional protein deficiency |
| 156 | HADHA | NM_000182.3 | 192 | ctcccaattcaaaggtaaataca | 215301 | 614223 | see also 155 line | | |
| 157 | HK1 | NM_000188.1 | 1837 | ttggactacatggggatcaaagg | 84948 | 503636 | kinase_related | - | hemolytic anemia |
| 158 | HMGCL | NM_000191.1 | 283 | aagtcttgaagggcattcagaag | 234030 | 618393 | - | hydroxymethylglutaryl-CoA lyase | hydroxymethylglutaricaciduria、3-hydroxy-3-methylglutaryl CoA lyase deficiency |
| 159 | SGSH | NM_000199.2 | 457 | gggcggaacatcactagaattaa | 200618 | 592336 | - | sulfuric ester hydrolase | mucopolysaccharidosis、SanFilippo syndrome |
| 160 | IDS | NM_000202.2 | 1155 | acgtccaagccttaaacatcagt | 200480 | 592153 | - | - | Hunter syndrome、mucopolysaccharidosis |
| 161 | ITGB3 | NM_000212.1 | 426 | ctgtggacatctactacttgatg | 249368 | 636022 | receptor_acitivity、signal_transduction | cell adhesion receptor | alloimmune thrombocytopenia |
| 162 | JAG1 | NM_000214.1 | 1660 | gtgaggccaaaccttgtgtaaac | 147049 | 549033 | signal_transduction | - | arteriohepatic dysplasia、cholestasis with peripheral pulmonary stenosis、syndromatic hepatic ductular hypoplasia |
| 163 | JAG1 | NM_000214.1 | 1976 | ctgtcagctggacatcgattatt | 147066 | 549042 | see also 162 line | | |
| 164 | JAG1 | NM_000214.1 | 3267 | tcctattaccaggataactgtgc | 147087 | 549072 | see also 162 line | | |
| 165 | KCNJ1 | NM_000220.2 | 515 | ctgccatttttctgcttatcttt | 86366 | 504836 | channel | - | Bartter syndrome、hyperprostaglandin syndrome |
| 166 | KIT | NM_000222.1 | 886 | atgtgttatgccaataatacttt | 87921 | 506182 | receptor_acitivity、kinase_related、signal_transduction | Smad3/Sp-1 heterodimer | soft tissue sarcoma、leukemia、germ cell tumors、piebaldism、testicular cancer、astrocytic tumors、familial/sporadic gastrointestinal stromal tumors |
| 167 | KIT | NM_000222.1 | 1324 | gagcccacaatagattggtattt | 87935 | 506204 | see also 166 line | | |
| 168 | KIT | NM_000222.1 | 2009 | gagggcccaccctggtcattaca | 87954 | 506225 | see also 166 line | | |
| 169 | KIT | NM_000222.1 | 2011 | gggcccaccctggtcattacaga | 87955 | 506226 | see also 166 line | | |
| 170 | LAMB3 | NM_000228.1 | 646 | gtccaacttaaccttatggattt | 291317 | 649402 | - | structural molecule | epidermolysis bullosa |
| 171 | SGCB | NM_000232.2 | 726 | gggcaaaaccattgaatttcaca | 320157 | 687430 | - | - | limb girdle muscular dystrophy |
| 172 | LIG1 | NM_000234.1 | 1926 | cagccgcatccccaagattaaac | 30532 | 506493 | - | DNA ligase (ATP) | - |
| 173 | LIG1 | NM_000234.1 | 1927 | agccgcatccccaagattaaact | 30533 | 506494 | see also 172 line | | |
| 174 | KCNH2 | NM_000238.2 | 299 | atcgccttctaccggaaagatgg | 206431 | 596881 | channel、signal_transduction | - | long QT syndrome |
| 175 | MET | NM_000245.1 | 3190 | ttgtaagtgcccgaagtgtaagc | 91088 | 508341 | receptor_acitivity、kinase_related、signal_transduction | hepatocyte growth factor receptor | hepatocellular carcinoma、renal cell carcinoma、esophageal cancer、papillary renal carcinoma、astrocytic tumors |
| 176 | MET | NM_000245.1 | 3465 | agcctgattgtgcatttcaatga | 91098 | 508349 | see also 175 line | | |
| 177 | MET | NM_000245.1 | 3468 | ctgattgtgcatttcaatgaagt | 91099 | 508350 | see also 175 line | | |
| 178 | MET | NM_000245.1 | 3911 | tggtcttgccagagacatgtatg | 91113 | 508359 | see also 175 line | | |

Figure 46

| | | | | | | | | | |
|---|---|---|---|---|---|---|---|---|---|
| 179 | MET | NM_000245.1 | 4294 | atgtgaacgctacttatgtgaac | 91128 | 508377 | see also 175 line | | |
| 180 | MITF | NM_000248.2 | 221 | cagtacctttctaccactttagc | 32278 | 461704 | transcription_regulator | translation regulator | Tietz syndrome、 Waardenburg syndrome |
| 181 | MITF | NM_000248.2 | 668 | agggagctcacagcgtgtatttt | 32290 | 461713 | see also 180 line | | |
| 182 | MLH1 | NM_000249.2 | 1735 | ctgttctaccagatactcattta | 91706 | 508504 | cell_cycle | adenosinetriphosphatase | colorectal cancer、 colorectal carcinoma、 ataxia telangiectasia、 nonpolyposis colon cancer、 Turcot syndrome with glioblastoma、 cerebral astrocytoma、 colorectal cancers、 Muir-Torre syndrome、 pancreatic cancer、 familial nervous system tumor syndromes、 gastric cancer |
| 183 | MSH2 | NM_000251.1 | 1783 | aagcccaggatgccattgttaaa | 16380 | 451452 | cell_cycle | damaged DNA binding | colorectal cancer、 glioblastoma、 multiple cafe-au-lait spots with leukemia、 colorectal carcinoma、 ataxia telangiectasia、 nonpolyposis colon cancer、 colorectal cancers、 Muir-Torre syndrome、 pancreatic cancer、 skin cancer |
| 184 | MSH2 | NM_000251.1 | 2074 | gccccaatatgggaggtaaatca | 16392 | 451460 | see also 183 line | | |
| 185 | MSH2 | NM_000251.1 | 2076 | cccaatatgggaggtaaatcaac | 16393 | 451461 | see also 183 line | | |
| 186 | MSH2 | NM_000251.1 | 2349 | gggttagcatgggctatatcaga | 16408 | 451466 | see also 183 line | | |
| 187 | MSH2 | NM_000251.1 | 2412 | acccattttcatgaacttactgc | 16413 | 451471 | see also 183 line | | |
| 188 | MTM1 | NM_000252.1 | 213 | tcctttcaatggccccattaagg | 192910 | 586507 | phosphatase | protein serine/threonine phosphatase | myotubular myopathy |
| 189 | MTM1 | NM_000252.1 | 257 | atcgtctttatttaagaagtttg | 192913 | 586511 | see also 188 line | | |
| 190 | MTM1 | NM_000252.1 | 370 | tcctatggtctagatattacttg | 192921 | 586517 | see also 188 line | | |
| 191 | MTM1 | NM_000252.1 | 374 | atggtctagatattacttgtaaa | 192922 | 586518 | see also 188 line | | |
| 192 | MTM1 | NM_000252.1 | 441 | cagaagagatatgtttgagatcc | 192925 | 586520 | see also 188 line | | |
| 193 | MTM1 | NM_000252.1 | 784 | cagcctcttgtcggtatgagtgg | 192937 | 586526 | see also 188 line | | |
| 194 | MTP | NM_000253.1 | 2216 | cagtgatttgatgtccaaaatgc | 173590 | 570268 | - | lipid transporter | acanthocytosis |
| 195 | MTP | NM_000253.1 | 2347 | agggtggtctagctattgatatt | 173594 | 570274 | see also 194 line | | |
| 196 | MTP | NM_000253.1 | 2348 | gggtggtctagctattgatattt | 173595 | 570275 | see also 194 line | | |
| 197 | MTP | NM_000253.1 | 2350 | gtggtctagctattgatatttca | 173596 | 570276 | see also 194 line | | |
| 198 | MTP | NM_000253.1 | 2405 | gtctaaaacccgagtgaaaaata | 173600 | 570277 | see also 194 line | | |
| 199 | MUT | NM_000255.1 | 357 | gaccatatcctaccatgtatacc | 235110 | 619750 | - | methylmalonyl-CoA mutase | methylmalonicaciduria |
| 200 | MUT | NM_000255.1 | 547 | tggaatggctggagttgctattg | 235116 | 619758 | see also 199 line | | |
| 201 | MYO5A | NM_000259.1 | 1390 | ggctactgccacagagacataca | 93166 | 509668 | - | myosin ATPase | - |
| 202 | MYO5A | NM_000259.1 | 2342 | ccctcacggtggacttaccaaga | 93189 | 509691 | see also 201 line | | |

## Figure 46

| | | | | | | | | | |
|---|---|---|---|---|---|---|---|---|---|
| 203 | MYO5A | NM_000259.1 | 2738 | gtccgcaggaggtacaagattag | 93202 | 509709 | see also 201 line | | |
| 204 | MYO5A | NM_000259.1 | 2741 | cgcaggaggtacaagattagacg | 93203 | 509711 | see also 201 line | | |
| 205 | MYO7A | NM_000260.1 | 4383 | gtggtgcgaggagtcaagtttgg | 93506 | 509877 | - | myosin ATPase | deafness、Senear-Usher syndrome |
| 206 | NAGLU | NM_000263.2 | 715 | gcccaacaggtaccgctattacc | 200984 | 592630 | - | alpha-N-acetylglucosaminidase | mucopolysaccharidosis、SanFilippo syndrome |
| 207 | PTCH | NM_000264.2 | 417 | ctgtggctgagagcgaagtttca | 245625 | 633183 | receptor_acitivity、signal_transduction、cell_cycle | patched receptor | nevoid basal cell carcinoma syndrome、basal cell nevus syndrome、medulloblastoma、familial nervous system tumor syndromes、skin cancer |
| 208 | PTCH | NM_000264.2 | 920 | gacagcatacctcctaggtaaac | 245640 | 633200 | see also 207 line | | |
| 209 | PTCH | NM_000264.2 | 2197 | cccacacgcacgtgtactacacc | 245658 | 633227 | see also 207 line | | |
| 210 | PTCH | NM_000264.2 | 2466 | ttgctgggggtcagcctttatgg | 245661 | 633229 | see also 207 line | | |
| 211 | NF1 | NM_000267.1 | 260 | ttcgacgagcagcttccaataaa | 294444 | 665363 | signal_transduction、cell_cycle | enzyme inhibitor | bladder transitional cell carcinoma、pilocystic astrocytomas、brain stem glioma、neuroblastoma、neurofibromatosis、Watson syndrome、squamous cell carcinomacytic、nerve sheath tumors、phaeochromocytoma、visual Pathway and hypothalamic glioma、familial nervous system tumor syndromes |
| 212 | NF1 | NM_000267.1 | 487 | atgtcttgctgggcaaccaaagg | 294454 | 665370 | see also 211 line | | |
| 213 | NF1 | NM_000267.1 | 656 | aacttcaatgcagtctttagtcg | 294457 | 665375 | see also 211 line | | |
| 214 | NF1 | NM_000267.1 | 1478 | ttggattggtggcctaagattga | 294469 | 665397 | see also 211 line | | |
| 215 | NF1 | NM_000267.1 | 1623 | aagtaacaagccttaaatttaaa | 294475 | 665406 | see also 211 line | | |
| 216 | NF1 | NM_000267.1 | 4971 | atgttgcacggaggttcaaaact | 294514 | 665512 | see also 211 line | | |
| 217 | NF1 | NM_000267.1 | 5092 | cgggcctagcaatcgctttaaaa | 294517 | 665517 | see also 211 line | | |
| 218 | NF1 | NM_000267.1 | 5093 | gggcctagcaatcgctttaaaac | 294518 | 665518 | see also 211 line | | |
| 219 | NF1 | NM_000267.1 | 6995 | gacacttacaacagtcaagttct | 294578 | 665583 | see also 211 line | | |
| 220 | NF1 | NM_000267.1 | 7004 | aacagtcaagttctgatagaagc | 294580 | 665584 | see also 211 line | | |
| 221 | NF1 | NM_000267.1 | 8016 | tagctacactggtaaaatatacc | 294610 | 665616 | see also 211 line | | |
| 222 | NF1 | NM_000267.1 | 8162 | tgccaagatccaaatttgttaaa | 294619 | 665620 | see also 211 line | | |
| 223 | NF1 | NM_000267.1 | 8458 | tagtatcactgccaaccttaacc | 294630 | 665628 | see also 211 line | | |
| 224 | NF1 | NM_000267.1 | 8628 | ctggcagtttcaaacgtaatagc | 294637 | 665634 | see also 211 line | | |
| 225 | NF2 | NM_000268.2 | 1223 | gtgacaaggagtttactattaaa | 291993 | 661873 | cell_cycle | - | ependymoma、neurofibromatosis、CNS Tumours、meningioma、nerve sheath tumors、malignant mesothelioma、mesothelioma、astrocytic tumors、familial nervous system tumor syndromes |

Figure 46

| 226 | NF2 | NM_000268.2 | 1284 | ctcaaagcttcgtgttaataagc | 291997 | 661875 | see also 225 line | | |
| 227 | OCRL | NM_000276.3 | 1124 | ttgggatgatgcttcttatattt | 196543 | 588985 | phosphatase | - | oculocerebrorenal syndrome of Lowe |
| 228 | OCRL | NM_000276.3 | 1531 | cagcgcacacagaaaaaagcttt | 196559 | 589001 | see also 227 line | | |
| 229 | OCRL | NM_000276.3 | 2147 | tggatcgaggcaaagattacttc | 196574 | 589023 | see also 227 line | | |
| 230 | OCRL | NM_000276.3 | 2150 | atcgaggcaaagattacttcttg | 196575 | 589024 | see also 227 line | | |
| 231 | PAX2 | NM_000278.2 | 1449 | caggcatcagagcacatcaaatc | 8862 | 701931 | transcription_regulator | - | optic nerve coloboma with renal disease |
| 232 | PAX2 | NM_000278.2 | 1450 | aggcatcagagcacatcaaatca | 8863 | 701932 | see also 231 line | | |
| 233 | PAX2 | NM_000278.2 | 1503 | ctgacccctgggcttgatgaagt | 8864 | 701935 | see also 231 line | | |
| 234 | PAX6 | NM_000280.1 | 534 | gtgcgacatttcccgaattctgc | 8904 | 447556 | transcription_regulator | - | Wilms tumor、aniridia、keratitis、medulloblastoma、alcoholism |
| 235 | PAX6 | NM_000280.1 | 748 | gtctgtaccaacgataacatacc | 8913 | 447564 | see also 234 line | | |
| 236 | PAX6 | NM_000280.1 | 826 | atgggcgcagacggcatgtatga | 8917 | 447567 | see also 234 line | | |
| 237 | PAX6 | NM_000280.1 | 828 | gggcgcagacggcatgtatgata | 8918 | 447568 | see also 234 line | | |
| 238 | PAX6 | NM_000280.1 | 830 | gcgcagacggcatgtatgataaa | 8919 | 447570 | see also 234 line | | |
| 239 | PAX6 | NM_000280.1 | 831 | cgcagacggcatgtatgataaac | 8920 | 447571 | see also 234 line | | |
| 240 | PAX6 | NM_000280.1 | 845 | atgataaactaaggatgttgaac | 8921 | 447574 | see also 234 line | | |
| 241 | PAX6 | NM_000280.1 | 1140 | aagactagcagccaaaatagatc | 8925 | 447579 | see also 234 line | | |
| 242 | PAX6 | NM_000280.1 | 1180 | caggtatggttttctaatcgaag | 8926 | 447580 | see also 234 line | | |
| 243 | PAX6 | NM_000280.1 | 1507 | gtgaatgggcggagttatgatac | 8933 | 447585 | see also 234 line | | |
| 244 | PAX6 | NM_000280.1 | 1512 | tgggcggagttatgatacctaca | 8934 | 447586 | see also 234 line | | |
| 245 | PCBD | NM_000281.1 | 296 | ggccagcttcatcgaacaagtag | 112354 | 715632 | transcription_regulator | transcription co-activator | hyperphenylalaninemia |
| 246 | PCCA | NM_000282.1 | 315 | agctcccaccagtaaaagctacc | 95850 | 511332 | - | propionyl-CoA carboxylase | propionyl-CoA carboxylase deficiency、propionic acidemia |
| 247 | PCCA | NM_000282.1 | 771 | tcctcgtcatatagaaatccagg | 95867 | 511346 | see also 246 line | | |
| 248 | PEPD | NM_000285.1 | 627 | aggcccaccgtgaggtaatgaag | 162019 | 698237 | - | metallocarboxypeptidase D | - |
| 249 | PEPD | NM_000285.1 | 628 | ggcccaccgtgaggtaatgaagg | 162020 | 698238 | see also 248 line | | |
| 250 | PEX12 | NM_000286.1 | 614 | gtggtagcacaggacagtttaat | 134552 | 688537 | - | zinc binding | peroxisome biogenesis disorder、Zellweger syndrome |
| 251 | PEX12 | NM_000286.1 | 615 | tggtagcacaggacagtttaatg | 134553 | 688538 | see also 250 line | | |
| 252 | PEX12 | NM_000286.1 | 912 | ttcttccctatctgaaagtgaag | 134559 | 688545 | see also 250 line | | |
| 253 | PEX7 | NM_000288.1 | 416 | aggaggtgtatagtgttgattgg | 177198 | 652167 | receptor_acitivity | protein transporter | peroxisome biogenesis disorder、chondrodysplasia |
| 254 | PEX7 | NM_000288.1 | 731 | gggcggttgactgtagtttgaga | 177201 | 652175 | see also 253 line | | |
| 255 | PFKM | NM_000289.3 | 84 | tggtggagatgcccaaggtatga | 166401 | 563810 | kinase_related | magnesium binding | glycogen storage disease、diabetes |
| 256 | PFKM | NM_000289.3 | 2031 | taggaattttgccactaagatgg | 166437 | 563843 | see also 255 line | | |

Figure 46

| 257 | PHKA2 | NM_000292.1 | 1388 | gtgaaatcgatcccttaaataga | 122020 | 527221 | kinase_related | phosphorylase kinase | glycogen storage disease |
|---|---|---|---|---|---|---|---|---|---|
| 258 | PHKA2 | NM_000292.1 | 1393 | atcgatcccttaaatagaagatt | 122021 | 527222 | see also 257 line | | |
| 259 | PHKA2 | NM_000292.1 | 1394 | tcgatcccttaaatagaagattt | 122022 | 527223 | see also 257 line | | |
| 260 | PHKA2 | NM_000292.1 | 3360 | cagggtccccgtgggattctacc | 122082 | 527266 | see also 257 line | | |
| 261 | PHKB | NM_000293.1 | 2256 | gggtatactgctcaaaagagaag | 122162 | 816932 | kinase_related | phosphorylase kinase、regulator | - |
| 262 | PKD2 | NM_000297.2 | 792 | gagctccaatgtgtactactaca | 120987 | 538970 | channel | voltage-gated sodium channel | polycystic kidney disease |
| 263 | PKP1 | NM_000299.1 | 401 | agcggcagaagtccaagtcttcc | 128261 | 530408 | signal_transduction | structural constituent of epidermis | ectodermal dysplasia |
| 264 | POU3F4 | NM_000307.1 | 621 | ttcgccaaacaattcaaacaaag | 9688 | 447868 | transcription_regulator | transcription factor | deafness |
| 265 | POU3F4 | NM_000307.1 | 622 | tcgccaaacaattcaaacaaaga | 9689 | 447869 | see also 264 line | | |
| 266 | PXR1 | NM_000319.3 | 866 | gagccaagtcagctatagagttg | 177230 | 652140 | receptor_acitivity | protein transporter | adrenoleukodystrophy、peroxisome biogenesis disorder |
| 267 | QDPR | NM_000320.1 | 639 | accccgatgaacaggaaatcaat | 225728 | 614596 | - | electron transporter | phenylketonuria |
| 268 | RB1 | NM_000321.1 | 1256 | ctccagttaggactgttatgaac | 10093 | 698531 | transcription_regulator、cell_cycle | translation regulator | hepatocellular carcinoma、bladder transitional cell carcinoma、chondrosarcoma、bladder cancer、breast cancer、lung cancer、osteosarcoma、retinoblastoma、Ewing's sarcoma、leukemia、testicular cancer、nerve sheath tumors、squamous cell cancer |
| 269 | RB1 | NM_000321.1 | 1507 | atggaatccatgcttaaatcaga | 10105 | 698540 | see also 268 line | | |
| 270 | RB1 | NM_000321.1 | 1513 | tccatgcttaaatcagaagaaga | 10106 | 698541 | see also 268 line | | |
| 271 | RB1 | NM_000321.1 | 2492 | ttcctcgaagcccttacaagttt | 10134 | 698569 | see also 268 line | | |
| 272 | RB1 | NM_000321.1 | 2493 | tcctcgaagcccttacaagtttc | 10135 | 698570 | see also 268 line | | |
| 273 | RET | NM_000323.2 | 3346 | gccctcccttccacatggattga | 98994 | 513384 | - | - | thyroid cancer、papillary Thyroid Carcinomas、multiple endocrine neoplasia、thyroid carcinoma、ganglioneuromatosis of the alimentary tract、Hirschsprung disease、medullary thyroid carcinoma、pheochromocytoma and familial amyloid-producing thyroid carcinoma、phaeochromocytoma |
| 274 | RET | NM_000323.2 | 3504 | ctcagcggcaaaattaatggaca | 99000 | 513390 | see also 273 line | | |
| 275 | RLBP1 | NM_000326.3 | 1135 | atgtggtcaagcccttcttgaag | 179790 | 573082 | - | transporter | retinitis pigmentosa |
| 276 | RPE65 | NM_000329.1 | 547 | caggttgatctttgcaactatgt | 135280 | 741196 | - | - | dystrophy、amaurosis congenita of Leber、retinitis pigmentosa |
| 277 | RPE65 | NM_000329.1 | 782 | ttggtctgactcccaactatatc | 135291 | 741203 | see also 276 line | | |

Figure 46

| No. | Gene | Accession | Pos | Sequence | | | Process | Function | Disease |
|---|---|---|---|---|---|---|---|---|---|
| 278 | RPE65 | NM_000329.1 | 783 | tggtctgactcccaactatatcg | 135292 | 741204 | see also 276 line | | |
| 279 | RPE65 | NM_000329.1 | 792 | tcccaactatatcgtttttgtgg | 135294 | 741206 | see also 276 line | | |
| 280 | RPE65 | NM_000329.1 | 1149 | taggagatatgtacttcctttga | 135303 | 741213 | see also 276 line | | |
| 281 | SCA1 | NM_000332.1 | 946 | accaagagcggagcaacgaatgc | 54643 | 475439 | - | molecular_function unknown | spinocerebellar ataxia、schizophrenia |
| 282 | SCA1 | NM_000332.1 | 2518 | ttcccaagagcgagaacttcaac | 54653 | 475456 | see also 281 line | | |
| 283 | SCA7 | NM_000333.1 | 1354 | atggatggcacactactgaaatc | 284078 | 689105 | - | molecular_function unknown | spinocerebellar ataxia |
| 284 | SLC12A1 | NM_000338.1 | 896 | atgatggtggatccaaccaatga | 256601 | 640521 | - | symporter | Bartter syndrome |
| 285 | SLC12A1 | NM_000338.1 | 2402 | aactacgtgggaatcatacatga | 256645 | 640553 | see also 284 line | | |
| 286 | SLC12A1 | NM_000338.1 | 2584 | aggcatccgaggcttgtttaaaa | 256656 | 640558 | see also 284 line | | |
| 287 | SLC2A2 | NM_000340.1 | 904 | ggcattcttattagtcagattat | 259120 | 643348 | - | sugar porter | diabetes mellitus、Fanconi syndrome、hepatic glycogenosis、diabetes |
| 288 | SOX9 | NM_000346.2 | 1545 | gtcccagcgaacgcacatcaaga | 40642 | 468061 | transcription_regulator | specific RNA polymerase II transcription factor | campomelic dysplasia |
| 289 | SPTB | NM_000347.3 | 1206 | tcccggatgagagccaacaatca | 119235 | 524092 | - | structural constituent of cytoskeleton | spherocytosis |
| 290 | SRD5A2 | NM_000348.2 | 613 | cacaaggtggcttgtttacgtat | 223957 | 612219 | - | electron transporter | breast cancer、prostate cancer、pseudohermaphroditism、pseudovaginal perineoscrotal hypospadias |
| 291 | ABCA4 | NM_000350.1 | 897 | ttggggaggaatattatctgata | 68854 | 490816 | - | adenosinetriphosphatase | cone-rod dystrophy、age related macular degeneration、macular dystrophy、retinitis pigmentosa、Stargardt disease |
| 292 | ABCA4 | NM_000350.1 | 1639 | ttggtcctggataagtttgaaag | 68873 | 490841 | see also 291 line | | |
| 293 | ABCA4 | NM_000350.1 | 1874 | tggaagatttccggtacatctgg | 68879 | 490845 | see also 291 line | | |
| 294 | ABCA4 | NM_000350.1 | 3750 | gtgcattggtcaagaacttatct | 68897 | 490877 | see also 291 line | | |
| 295 | ABCA4 | NM_000350.1 | 4722 | gagcaaattctgggtcaatgaac | 68912 | 490900 | see also 291 line | | |
| 296 | TIMP3 | NM_000362.3 | 1683 | gaccgacatgctctccaatttcg | 284751 | 663661 | apoptosis | metalloendopeptidase inhibitor | Sorsby fundus dystrophy、pseudoinflammatory(-) |
| 297 | TSC1 | NM_000368.2 | 2292 | ctccgagaccagttgcttttact | 305939 | 712554 | cell_cycle、signal_transduction | - | bladder transitional cell carcinoma、tuberous sclerosis、renal cell carcinoma、squamous cell carcinomacytic、familial nervous system tumor syndromes |
| 298 | TSC1 | NM_000368.2 | 2427 | atgaaagatcagttgaagttaca | 305941 | 712555 | see also 297 line | | |
| 299 | UMPS | NM_000373.1 | 1311 | ggctcccgagtaagcatgaaacc | 212768 | 601412 | - | transferase、transferring glycosyl groups | oroticaciduria |

Figure 46

| 300 | VDR | NM_000376.1 | 813 | tggtcagttacagcatccaaaag | 13923 | 449829 | transcription_regulator, receptor_acitivity, signal_transduction | VDR/RXR alpha heterodimer | vitamin D-resistant rickets |
|---|---|---|---|---|---|---|---|---|---|
| 301 | WT1 | NM_000378.2 | 1322 | caggctgcaataagagatatttt | 13945 | 449857 | transcription_regulator, cell_cycle | - | desmoplastic small round cell tumor, Wilms tumor, bladder cancer, Denys-Drash syndrome, Frasier syndrome, WAGR syndrome, leukemia, nephroblastoma, squamous cell carcinomacytic, malignant mesothelioma, mesothelioma |
| 302 | XDH | NM_000379.2 | 3815 | tccgcgactgccccaacaagaag | 164749 | 562663 | - | TBP-associated factor 125 | xanthinuria |
| 303 | MID1 | NM_000381.1 | 654 | tacaggccatcgtctgattgagc | 133903 | 558397 | - | - | Opitz/BBB syndrome |
| 304 | MID1 | NM_000381.1 | 822 | gagtgagcgctatgacaaattga | 133905 | 558400 | see also 303 line | | |
| 305 | MID1 | NM_000381.1 | 824 | gtgagcgctatgacaaattgaag | 133906 | 558401 | see also 303 line | | |
| 306 | BLMH | NM_000386.2 | 553 | gacaaggttgaacgctgttattt | 187933 | 581695 | - | carboxypeptidase | Alzheimer disease |
| 307 | BLMH | NM_000386.2 | 557 | aggttgaacgctgttatttcttc | 187934 | 581696 | see also 306 line | | |
| 308 | BLMH | NM_000386.2 | 563 | aacgctgttatttcttcttgagt | 187936 | 581697 | see also 306 line | | |
| 309 | CASR | NM_000388.2 | 1578 | cagtgtcgagaccccttacatag | 241438 | 629675 | gpcr, receptor_acitivity, signal_transduction | metabotropic glutamate, GABA-B-like receptor | familial hypocalciuric hypercalcemia, hyperparathyroidism, hypocalcemia, hypocalciuric hypercalcemia |
| 310 | CHM | NM_000390.1 | 1129 | cggtatggcaacactccatttt | 216773 | 707669 | - | RAB-protein geranylgeranyltransferase | choroideremia |
| 311 | ABCC2 | NM_000392.1 | 3261 | cacccacaggccggattgtgaac | 70296 | 492270 | - | organic anion transporter | hyperbilirubinemia |
| 312 | ABCC2 | NM_000392.1 | 3262 | acccacaggccggattgtgaaca | 70297 | 492271 | see also 311 line | | |
| 313 | ABCC2 | NM_000392.1 | 3834 | ttgtggctgttgagcgaataact | 70302 | 492284 | see also 311 line | | |
| 314 | ABCC2 | NM_000392.1 | 4072 | ctgcctcttcagaatcttagagg | 70306 | 492294 | see also 311 line | | |
| 315 | COL5A2 | NM_000393.2 | 2668 | gccaactggagctgttggttttg | 288332 | 659739 | - | Homeobox protein PKNOX1 | Ehlers-Danlos syndrome |
| 316 | CRYAA | NM_000394.2 | 150 | cggcgagggccttttgagtatg | 289510 | 659900 | - | structural constituent of eye lens | - |
| 317 | CTSK | NM_000396.2 | 249 | atgaaatctctcggcgtttaatt | 187880 | 581636 | - | cathepsin K | rheumatoid arthritis, pycnodysostosis |
| 318 | CTSK | NM_000396.2 | 480 | tcgactatcgaaagaaaggatat | 187885 | 581647 | see also 317 line | | |
| 319 | CYBB | NM_000397.2 | 807 | aaccctcctatgacttggaaatg | 226516 | 613664 | channel | hematopoietic-associated factor 1A complex | atherosclerosis, chronic granulomatous disease |
| 320 | CYBB | NM_000397.2 | 1212 | agctatgaggtggtgatgttagt | 226523 | 613680 | see also 319 line | | |

Figure 46

| 321 | EXT2 | NM_000401.1 | 1122 | tggctggtggcggctttctacg | 213370 | 601718 | signal_transduction、cell_cycle | transferase、transferring glycosyl groups | chondrosarcoma、hereditary multiple exostoses、multiple exostoses |
|---|---|---|---|---|---|---|---|---|---|
| 322 | GM2A | NM_000405.3 | 237 | tcctgggataactgtgatgaagg | 298547 | 672482 | - | sphingolipid activator protein | GM2 gangliosidosis |
| 323 | GPD2 | NM_000408.1 | 1010 | ctgtgcgcaaaatggatgataaa | 146580 | 548897 | - | glycerol-3-phosphate dehydrogenase | diabetes mellitus、diabetes |
| 324 | GPD2 | NM_000408.1 | 1012 | gtgcgcaaaatggatgataaaga | 146581 | 548898 | see also 323 line | | |
| 325 | HLCS | NM_000411.3 | 2698 | accgcaggaaatgggcttaatag | 232175 | 617097 | - | biotin-[propionyl-CoA-carboxylase (ATP-hydrolyzing)] ligase | multiple carboxylase deficiency |
| 326 | HLCS | NM_000411.3 | 2927 | gtgaagtggcccaacgatattta | 232179 | 617101 | see also 325 line | | |
| 327 | LAMA2 | NM_000426.2 | 633 | atgaggtcatctgcacttcattt | 290696 | 661415 | - | structural molecule | congenital muscular dystrophy |
| 328 | LAMA2 | NM_000426.2 | 2467 | ctgtgcctgtccactcaatatcc | 290748 | 661462 | see also 327 line | | |
| 329 | LAMA2 | NM_000426.2 | 3888 | cacctactcatgctagaattatc | 290774 | 661486 | see also 327 line | | |
| 330 | LAMA2 | NM_000426.2 | 6968 | tactttgacaacaaacctatagg | 290849 | 661559 | see also 327 line | | |
| 331 | LAMA2 | NM_000426.2 | 6972 | ttgacaacaaacctataggtttg | 290850 | 661560 | see also 327 line | | |
| 332 | LAMA2 | NM_000426.2 | 7525 | ttccggctgcctcaaagatattg | 290873 | 661578 | see also 327 line | | |
| 333 | LTBP2 | NM_000428.1 | 2453 | agcggatcaccaagcagatatgc | 133567 | 549909 | - | - | Marfan syndrome |
| 334 | LTBP2 | NM_000428.1 | 3397 | cagagtgggagctgtgtagatgt | 133571 | 549920 | see also 333 line | | |
| 335 | LTBP2 | NM_000428.1 | 4281 | cggagactgcattgacatagacg | 133579 | 549926 | see also 333 line | | |
| 336 | PAFAH1B1 | NM_000430.2 | 568 | cagagacaacgagatgaactaaa | 200114 | 591784 | signal_transduction | 2-acetyl-1-alkylglycerophosphocholine esterase | lissencephaly、Miller-Dieker syndrome |
| 337 | PAFAH1B1 | NM_000430.2 | 591 | tcgagctatagcagattatcttc | 200115 | 591786 | see also 336 line | | |
| 338 | PAFAH1B1 | NM_000430.2 | 720 | gacatctgttattagattacaaa | 200117 | 591794 | see also 336 line | | |
| 339 | PAFAH1B1 | NM_000430.2 | 902 | tccatcctgtgttcagtgttatg | 200123 | 591801 | see also 336 line | | |
| 340 | PAFAH1B1 | NM_000430.2 | 1177 | gtgtctgcctcaagggataaaac | 200127 | 591810 | see also 336 line | | |
| 341 | PAFAH1B1 | NM_000430.2 | 1181 | ctgcctcaagggataaaactata | 200128 | 591811 | see also 336 line | | |
| 342 | PAFAH1B1 | NM_000430.2 | 1572 | ctgggtacgtggagttctgttcc | 200140 | 591820 | see also 336 line | | |
| 343 | PAFAH1B1 | NM_000430.2 | 1677 | gaccctcaatgcgcatgaacact | 200146 | 591822 | see also 336 line | | |

Figure 46

| | | | | | | | | | |
|---|---|---|---|---|---|---|---|---|---|
| 344 | NOTCH3 | NM_000435.1 | 1005 | gagctgcagtcagaatatcgatg | 149247 | 538219 | receptor_acitivity、 transcription_regulator | translation regulator | cerebral autosomal dominant arteriopathy with subcortical infarcts and leukoencephalopathy |
| 345 | PAX3 | NM_000438.3 | 696 | aacgcctgacgtggagaagaaaa | 8869 | 447542 | transcription_regulator、 apoptosis | - | rhabdomyosarcoma、 alveolar rhabdomyosarcoma、 craniofacial-deafness-hand syndrome、 Waardenburg syndrome |
| 346 | PCSK1 | NM_000439.3 | 754 | cagaggctagctatgatttaat | 183033 | 576748 | - | subtilase | diabetes mellitus、 obesity and endocrinopathy、 diabetes |
| 347 | PCSK1 | NM_000439.3 | 1306 | gcgctgacctgcacaatgactgc | 183044 | 576766 | see also 346 line | | |
| 348 | PDE6A | NM_000440.1 | 171 | tggctttgccaaacagtactaca | 197651 | 590210 | signal_transduction | cGMP-specific phosphodiesterase | retinitis pigmentosa |
| 349 | SLC26A4 | NM_000441.1 | 1934 | tggatttgatgccattagagtat | 260159 | 643730 | - | sulfate porter | enlarged vestibular aqueduct |
| 350 | ABCB4 | NM_000443.2 | 2472 | cacgtttttgcaggattcatag | 69678 | 491910 | - | - | colorectal cancer、 multiple drug resistance、 colorectal cancers |
| 351 | PON1 | NM_000446.3 | 796 | gtgtatgaaaagcatgctaattg | 197585 | 589761 | phosphatase | arylesterase | atherosclerosis、 diabetes mellitus、 diabetes |
| 352 | PON1 | NM_000446.3 | 967 | gtgcttcgaatccagaacattct | 197591 | 589763 | see also 351 line | | |
| 353 | RFX5 | NM_000449.2 | 250 | ggctccgaggtaccatttccaag | 10490 | 466251 | transcription_regulator | translation regulator | bare lymphocyte syndrome、 MHC class II deficiency |
| 354 | RFX5 | NM_000449.2 | 613 | ggggccagtccaaatattgctac | 10498 | 466255 | see also 353 line | | |
| 355 | TCF2 | NM_000458.1 | 209 | cacgtcgctccagcaagaactcc | 12922 | 449345 | transcription_regulator | - | diabetes mellitus、 diabetes、 maturity-onset diabetes of the young |
| 356 | TCF2 | NM_000458.1 | 1429 | cagtcagcaccttgacgaatatc | 12936 | 449360 | see also 355 line | | |
| 357 | TCF2 | NM_000458.1 | 1433 | cagcaccttgacgaatatccaca | 12937 | 449361 | see also 355 line | | |
| 358 | TEK | NM_000459.1 | 964 | gggatgcaagtcttatgtgttct | 102305 | 516105 | kinase_related、 receptor_acitivity、 signal_transduction | transmembrane receptor protein tyrosine kinase | venous malformations |
| 359 | TEK | NM_000459.1 | 3342 | atgaggtgtatgatctaatgaga | 102367 | 516159 | see also 358 line | | |
| 360 | TEK | NM_000459.1 | 3397 | atcatttgcccagatattggtgt | 102369 | 516163 | see also 358 line | | |
| 361 | THRB | NM_000461.2 | 769 | caggaatgtcgctttaagaaatg | 13344 | 449555 | transcription_regulator、 receptor_acitivity | Thyroid hormone receptor beta-2 | thyroid hormone resistance |
| 362 | THRB | NM_000461.2 | 1133 | tggtggattttgccaaaaagttg | 13355 | 449561 | see also 361 line | | |
| 363 | THRB | NM_000461.2 | 1443 | tgcctgtgttgagagaatagaaa | 13357 | 449565 | see also 361 line | | |
| 364 | UBE3A | NM_000462.2 | 168 | ctgtccaacttttcttcgtatgg | 231325 | 541872 | - | - | Angelman syndrome |
| 365 | UBE3A | NM_000462.2 | 831 | ctctcgagatcctaattatctga | 231344 | 541897 | see also 364 line | | |
| 366 | UBE3A | NM_000462.2 | 1472 | tgggattatattatgacaataga | 231360 | 541916 | see also 364 line | | |
| 367 | UBE3A | NM_000462.2 | 1855 | ctgggtctggctatttacaataa | 231373 | 541930 | see also 364 line | | |
| 368 | UBE3A | NM_000462.2 | 2029 | atgatcactttccagatatcaca | 231380 | 541934 | see also 364 line | | |
| 369 | UBE3A | NM_000462.2 | 2041 | cagatatcacagacagatctttt | 231381 | 541935 | see also 364 line | | |

Figure 46

| | | | | | | | | | |
|---|---|---|---|---|---|---|---|---|---|
| 370 | UBE3A | NM_000462.2 | 2127 | caggaaggaatttgtcaatcttt | 231385 | 541969 | see also 364 line | | |
| 371 | UBE3A | NM_000462.2 | 2280 | tggaagccggaatctagatttcc | 231391 | 541949 | see also 364 line | | |
| 372 | UBE3A | NM_000462.2 | 2283 | aagccggaatctagatttccaag | 231392 | 541950 | see also 364 line | | |
| 373 | BARD1 | NM_000465.1 | 2319 | gggttcggcagggcaaagtctgg | 324001 | 689791 | - | - | breast cancer、 ovarian cancer |
| 374 | PEX1 | NM_000466.1 | 1489 | atgcttcctttggtaatatcaga | 96420 | 794091 | - | peroxisome-assembly ATPase | adrenoleukodystrophy、 Refsum disease、 Zellweger syndrome |
| 375 | AMPD3 | NM_000480.1 | 1168 | ccgatggccccacgaaaacctat | 203684 | 594081 | - | AMP deaminase | - |
| 376 | AMPD3 | NM_000480.1 | 1190 | ttgtcaccggcgactgaactttc | 203687 | 594082 | see also 375 line | | |
| 377 | APP | NM_000484.1 | 679 | aagttccgaggggtagagtttgt | 128978 | 532117 | apoptosis、 signal_tran sduction | Upstream stimulatory factor 1 | Alzheimer disease、 angiopathy、 schizophrenia |
| 378 | APP | NM_000484.1 | 1723 | atccggtcccaggttatgacaca | 128995 | 532130 | see also 377 line | | |
| 379 | ATRX | NM_000489.2 | 6677 | tggacaaactaagcctgtttatg | 1285 | 455796 | transcription_regulator | - | mental retardation、 alpha thalassemia/mental retardation syndrome、 Juberg-Marsidi syndrome、 Sutherland-Haan X-linked mental retardation syndrome、 alpha thalassemia myelodysplasia syndrome |
| 380 | ATRX | NM_000489.2 | 6722 | aaccatggaagataagatttatg | 1288 | 455799 | see also 379 line | | |
| 381 | ATRX | NM_000489.2 | 7066 | gactgaccatgcgtttcaacata | 1298 | 455810 | see also 379 line | | |
| 382 | ATRX | NM_000489.2 | 7426 | gtgttcagcgaatacttatgaac | 1307 | 455828 | see also 379 line | | |
| 383 | ATRX | NM_000489.2 | 7559 | ttctaactaccagcagattgata | 1310 | 455832 | see also 379 line | | |
| 384 | CFTR | NM_000492.2 | 3058 | ttctccaaagatatagcaatttt | 76718 | 498044 | channel | - | - |
| 385 | CFTR | NM_000492.2 | 3423 | ctggttccaaatgagaatagaaa | 76727 | 498060 | see also 384 line | | |
| 386 | COL10A1 | NM_000493.2 | 1524 | cccagctggcatagcaactaagg | 287633 | 659247 | - | extracellular matrix structural constituent | metaphyseal chondrodysplasia |
| 387 | COL10A1 | NM_000493.2 | 1922 | atgtttgggtaggcctgtataag | 287641 | 659257 | see also 386 line | | |
| 388 | COL4A5 | NM_000495.3 | 4667 | ctcctgtatgtacaaggaaataa | 288263 | 659606 | - | - | diffuse esophageal leiomyomatosis、 Alport syndrome |
| 389 | CRYBB2 | NM_000496.1 | 321 | cagccaagagcacaagatcatcc | 289564 | 659933 | - | structural constituent of eye lens | cataract |
| 390 | EYA1 | NM_000503.3 | 747 | ctcccaatggcaccgaagttaaa | 204958 | 595733 | phosphatase、 transcrip tion_regulator | - | renal dysplasia |
| 391 | EYA1 | NM_000503.3 | 754 | tggcaccgaagttaaaacagagc | 204959 | 595736 | see also 390 line | | |
| 392 | EYA1 | NM_000503.3 | 1274 | cagcaggactatccgtcttatcc | 204973 | 595749 | see also 390 line | | |
| 393 | EYA1 | NM_000503.3 | 1308 | agggtcagtacgcacagtattat | 204974 | 595751 | see also 390 line | | |
| 394 | EYA1 | NM_000503.3 | 1309 | gggtcagtacgcacagtattata | 204975 | 595752 | see also 390 line | | |
| 395 | EYA1 | NM_000503.3 | 1662 | ctgggtcctacgccaacagatat | 204983 | 595756 | see also 390 line | | |
| 396 | EYA1 | NM_000503.3 | 1663 | tgggtcctacgccaacagatatg | 204984 | 595757 | see also 390 line | | |

Figure 46

| # | Gene | Accession | Pos | Sequence | ID1 | ID2 | Keywords | Molecular function | Disease |
|---|---|---|---|---|---|---|---|---|---|
| 397 | EYA1 | NM_000503.3 | 1959 | gggtaaaagagatctacaacacc | 204990 | 595765 | see also 390 line | | |
| 398 | GDNF | NM_000514.2 | 780 | aacctgtttaccatattcttaag | 169225 | 566029 | apoptosis、 signal_tran sduction | growth factor | Hirschsprung disease、 Parkinson disease |
| 399 | GDNF | NM_000514.2 | 783 | ctgtttaccatattcttaagaaa | 169227 | 566030 | see also 398 line | | |
| 400 | HOXA13 | NM_000522.2 | 34 | atgagccacgtcatgtttct | 329689 | 697112 | transcription_regulator | transcription factor | - |
| 401 | HOXA13 | NM_000522.2 | 849 | cggctgaacggcaaatgtact | 329694 | 697118 | see also 400 line | | |
| 402 | OTC | NM_000531.3 | 1004 | tggctcgagtgtataaacaatca | 180058 | 573200 | - | ornithine carbamoyltransferase | hyperammonemia |
| 403 | PLP1 | NM_000533.2 | 255 | agctaattgagacctatttctcc | 292352 | 662127 | - | structural molecule | Pelizaeus-Merzbacher disease、 spastic paraplegia |
| 404 | PMS1 | NM_000534.2 | 490 | ttggtcaaggtacaactgtaact | 35428 | 463715 | cell_cycle、 transcripti on_regulator | DNA binding | colorectal cancer、 colorectal carcinoma、 ataxia telangiectasia、 hereditary nonpolyposis colon cancer (HNPCC)、 colorectal cancers |
| 405 | PMS1 | NM_000534.2 | 2390 | gccactgaaaagccaattatgt | 35487 | 463774 | see also 404 line | | |
| 406 | RHO | NM_000539.2 | 332 | agccgtggctgacctcttcatg | 238421 | 629104 | gpcr、 receptor_acitivit y、 signal_transduction | G-protein coupled photoreceptor | congenital stationary night blindness、 retinitis pigmentosa |
| 407 | TSHB | NM_000549.1 | 150 | ttgtatgacacgggatatcaatg | 170144 | 567025 | signal_transduction、 g pcr | Thyroid hormone receptor beta-2 | Hypothyroidism |
| 408 | GDF5 | NM_000557.2 | 975 | agcagaggtacgtgtttgacatt | 131507 | 565568 | signal_transduction | protein binding | chondrodysplasia、 brachydactyly |
| 409 | C8A | NM_000562.1 | 1496 | tcctgttttatcgatttgaga | 312037 | 760675 | - | complement activity | C8 alpha deficiency |
| 410 | IGFBP5 | NM_000599.1 | 1041 | gcgggggtttgcctcaacgaaaag | 124917 | 528747 | signal_transduction | insulin-like growth factor binding | - |
| 411 | IGFBP5 | NM_000599.1 | 1405 | ttgtgaccgcaaaggattctaca | 124920 | 528751 | see also 410 line | | |
| 412 | IGFBP5 | NM_000599.1 | 1407 | gtgaccgcaaaggattctacaag | 124921 | 528752 | see also 410 line | | |
| 413 | IGFBP5 | NM_000599.1 | 1410 | accgcaaaggattctacaagaga | 124922 | 528753 | see also 410 line | | |
| 414 | FGFR1 | NM_000604.2 | 1657 | ttgaagactgctggagttaatac | 82211 | 502270 | receptor_acitivity、 kin ase_related、 signal_tra nsduction | | breast cancer、 Jackson-Weiss syndrome、 Li-Fraumeni syndrome、 pancreatic cancer、 Pfeiffer syndrome、 Pfeiffer type acroecephalosyndactyly、 leukemia、 Kallmann syndrome |
| 415 | SLC11A2 | NM_000617.1 | 277 | ttgttttagctttcgtaaactct | 266351 | 656771 | - | iron transporter | - |
| 416 | SLC11A2 | NM_000617.1 | 1562 | atgtactttgtagtgtttatgt | 266382 | 656795 | see also 415 line | | |
| 417 | NOS1 | NM_000620.1 | 2007 | acgggatgttcaactacatctgt | 121786 | 526894 | - | nitric oxide synthase | - |
| 418 | NOS1 | NM_000620.1 | 4908 | aagtgaccaaccgcctttagatct | 121821 | 526940 | see also 417 line | | |
| 419 | NOS2A | NM_000625.3 | 2166 | ctgcgcctttgctcatgacattg | 121846 | 526961 | - | - | diabetes |
| 420 | LTBP1 | NM_000627.1 | 1017 | atgccctaatggtgagtgtttga | 125052 | 709545 | receptor_acitivity | growth factor binding | - |

Figure 46

| | | | | | | | | | |
|---|---|---|---|---|---|---|---|---|---|
| 421 | SOD2 | NM_000636.1 | 563 | cacgcttactaccttcagtataa | 162345 | 561865 | transcription_regulator | translation regulator | diabetes |
| 422 | SOD2 | NM_000636.1 | 564 | acgcttactaccttcagtataaa | 162346 | 561866 | see also 421 line | | |
| 423 | GSR | NM_000637.1 | 290 | tggatgtgtacccaaaaaggtaa | 166871 | 564154 | - | heavy metal binding | hemolytic anemia |
| 424 | GSR | NM_000637.1 | 1048 | tcgtagacgaattccagaatacc | 166894 | 564176 | see also 423 line | | |
| 425 | ABAT | NM_000663.2 | 753 | gggaggaccatgggttgcttagc | 218139 | 606075 | - | 4-aminobutyrate aminotransferase | GABA-transaminase deficiency |
| 426 | ADRA1B | NM_000679.2 | 214 | gcccactggggagagttgaaaaa | 237746 | 623475 | gpcr、receptor_acitivity、kinase_related、signal_transduction | alpha1-adrenergic receptor | atherosclerosis |
| 427 | ADRA1B | NM_000679.2 | 999 | gagttccatagctgtcaaacttt | 237755 | 623484 | see also 426 line | | |
| 428 | ADRB1 | NM_000684.1 | 457 | gggagtacggctccttcttctgc | 237781 | 623522 | gpcr、receptor_acitivity、signal_transduction | beta1-adrenergic receptor | - |
| 429 | AGTR2 | NM_000686.3 | 710 | acgtcagaaccattgaatactta | 176672 | 623601 | gpcr、receptor_acitivity、kinase_related、apoptosis、signal_transduction | protein kinase inhibitor | - |
| 430 | AGTR2 | NM_000686.3 | 1141 | cagaagctccgcagtgtgtttag | 176683 | 623617 | see also 429 line | | |
| 431 | ATP1A2 | NM_000702.1 | 700 | ctgacctccggatcatctcttct | 72624 | 494392 | - | sodium/potassium-exchanging ATPase | hemiplegic migraine |
| 432 | ATP1A2 | NM_000702.1 | 814 | cccgcaatatctgtttcttctcc | 72628 | 494393 | see also 431 line | | |
| 433 | ATP4A | NM_000704.1 | 1819 | gcgggacttgtatccatgattga | 73068 | 494682 | - | plasma membrane cation-transporting ATPase | - |
| 434 | BCKDHA | NM_000709.2 | 406 | gccggatctccttctacatgacc | 225415 | 611917 | - | alpha-ketoacid dehydrogenase | maple syrup urine disease |
| 435 | CACNA1B | NM_000718.1 | 4111 | caggggtcagtatttggattatg | 74787 | 496912 | channel | voltage-gated calcium channel | - |
| 436 | CACNA1B | NM_000718.1 | 4515 | ggcagtcgttccagtataagacg | 74791 | 496923 | see also 435 line | | |
| 437 | CACNA1B | NM_000718.1 | 5361 | ctgcgtgtgggcgcatcagttac | 74800 | 496934 | see also 435 line | | |
| 438 | CACNA1B | NM_000718.1 | 5362 | tgcgtgtgggcgcatcagttaca | 74801 | 496935 | see also 435 line | | |
| 439 | CACNA1B | NM_000718.1 | 5370 | ggcgcatcagttacaatgacatg | 74803 | 496937 | see also 435 line | | |
| 440 | CACNA1B | NM_000718.1 | 5463 | agcgcctggttcgcatgaacatg | 74806 | 496939 | see also 435 line | | |
| 441 | CACNA1B | NM_000718.1 | 5464 | gcgcctggttcgcatgaacatgc | 74807 | 496940 | see also 435 line | | |
| 442 | CACNA1C | NM_000719.3 | 305 | aaccaccaaggttccaactatgg | 138479 | 544689 | channel | voltage-gated calcium channel | - |
| 443 | CACNA1C | NM_000719.3 | 3548 | aagtttgactttgacaatgttct | 138515 | 544735 | see also 442 line | | |

Figure 46

| No. | Gene | Accession | Pos | Sequence | ID1 | ID2 | Note | Description |
|---|---|---|---|---|---|---|---|---|
| 444 | CACNA1C | NM_000719.3 | 6371 | ctggtggaagcggctgatttc | 138528 | 544759 | see also 442 line | |
| 445 | CACNA1D | NM_000720.1 | 375 | agcaatacgccaagagcaaaaa | 138532 | 544775 | channel | dihydropyridine-sensitive calcium channel; alcoholism |
| 446 | CACNA1D | NM_000720.1 | 461 | atccgaagagcctgcattagtat | 138533 | 544779 | see also 445 line | |
| 447 | CACNA1D | NM_000720.1 | 462 | tccgaagagcctgcattagtata | 138534 | 544780 | see also 445 line | |
| 448 | CACNA1D | NM_000720.1 | 466 | aagagcctgcattagtatagtgg | 138535 | 544781 | see also 445 line | |
| 449 | CACNA1D | NM_000720.1 | 933 | ttcacatagcctttggtatta | 138547 | 544800 | see also 445 line | |
| 450 | CACNA1D | NM_000720.1 | 3792 | cgcctttcgaatacatgatgttt | 138594 | 544848 | see also 445 line | |
| 451 | CACNA1D | NM_000720.1 | 3793 | gcctttcgaatacatgatgtttg | 138595 | 544849 | see also 445 line | |
| 452 | CACNA1D | NM_000720.1 | 4004 | atcgtaatcggcagcattataga | 138601 | 544853 | see also 445 line | |
| 453 | CACNA1D | NM_000720.1 | 5006 | ttcctgatacaggactactttag | 138619 | 544868 | see also 445 line | |
| 454 | CACNA1D | NM_000720.1 | 5091 | ttgccctacaggcgggattaagg | 138621 | 544869 | see also 445 line | |
| 455 | CACNA1D | NM_000720.1 | 5094 | ccctacaggcgggattaaggaca | 138622 | 544870 | see also 445 line | |
| 456 | CACNA1D | NM_000720.1 | 5236 | tggaaaccatgtcaatcatgtta | 138630 | 544873 | see also 445 line | |
| 457 | CACNA1E | NM_000721.1 | 370 | ctgttcatcttcgagagaagataa | 138655 | 544902 | channel | voltage-gated calcium channel |
| 458 | CACNA1E | NM_000721.1 | 429 | gccgccatttgagtacatgatcc | 138657 | 544903 | see also 457 line | |
| 459 | CACNA1E | NM_000721.1 | 919 | ttcatgaacaattcaggtattct | 138664 | 544906 | see also 457 line | |
| 460 | CACNA1E | NM_000721.1 | 922 | atgaacaattcaggtattctaga | 138665 | 544907 | see also 457 line | |
| 461 | CACNA1E | NM_000721.1 | 1101 | cactgtgctgtacaataccaatg | 138669 | 544912 | see also 457 line | |
| 462 | CACNA1E | NM_000721.1 | 2196 | gtggtctgccatctacttcattg | 138701 | 544935 | see also 457 line | |
| 463 | CACNA1E | NM_000721.1 | 4087 | aaggagtgcataggcaactatgt | 138713 | 544951 | see also 457 line | |
| 464 | CACNA1E | NM_000721.1 | 4092 | gtgcataggcaactatgtagatc | 138715 | 544952 | see also 457 line | |
| 465 | CACNA1E | NM_000721.1 | 5369 | cagcatgtggccgcatccattac | 138731 | 544967 | see also 457 line | |
| 466 | CACNA1E | NM_000721.1 | 5370 | agcatgtggccgcatccattaca | 138732 | 544968 | see also 457 line | |
| 467 | CACNA1E | NM_000721.1 | 5429 | cgctaggcctcggcaagagatgt | 138733 | 544970 | see also 457 line | |
| 468 | CACNA1E | NM_000721.1 | 5546 | tccggacgctctggacattaaa | 138734 | 544972 | see also 457 line | |
| 469 | CACNA1E | NM_000721.1 | 6050 | ttcgggataagcgttcaaattcc | 138741 | 544983 | see also 457 line | |
| 470 | CACNA1E | NM_000721.1 | 6263 | ctgatgtctcccgctgcaattca | 138746 | 544986 | see also 457 line | |
| 471 | CACNA2D1 | NM_000722.1 | 388 | aagcaatgaagtgtctactaca | 261347 | 645130 | channel | dihydropyridine-sensitive calcium channel; malignant hyperthermia |
| 472 | CACNA2D1 | NM_000722.1 | 586 | ctggacaagtgccttagatgaag | 261360 | 645142 | see also 471 line | |
| 473 | CACNA2D1 | NM_000722.1 | 896 | ttcgtgaatgtagcttcatttaa | 261376 | 645158 | see also 471 line | |

Figure 46

| | | | | | | | | | |
|---|---|---|---|---|---|---|---|---|---|
| 474 | CACNA2D1 | NM_000722.1 | 1291 | aagaatcaatactcaggaatatt | 261389 | 645178 | see also 471 line | | |
| 475 | CACNB2 | NM_000724.1 | 999 | atcatctgctatagacatagatg | 261502 | 644952 | channel | voltage-gated calcium channel | Lambert-Eaton myasthenic syndrome |
| 476 | CACNB2 | NM_000724.1 | 1274 | aagggcggatatccatcacaagg | 261507 | 644957 | see also 475 line | | |
| 477 | CACNB2 | NM_000724.1 | 1448 | tggtccttgacgcggatacaatt | 261516 | 644962 | see also 475 line | | |
| 478 | CD3E | NM_000733.1 | 634 | ggccagcgggacctgtattctgg | 246051 | 633536 | receptor_acitivity、kinase_related、signal_transduction、gpcr、apoptosis | T-cell factor 1G | leukemia、diabetes |
| 479 | CHRM1 | NM_000738.2 | 1650 | tgctacgcactctgcaacaaagc | 178927 | 623958 | gpcr、receptor_acitivity、signal_transduction、kinase_related | muscarinic acetylcholine receptor | - |
| 480 | CHRNA7 | NM_000746.2 | 310 | atgtcttggacagatcactattt | 179127 | 572257 | channel、receptor_acitivity、kinase_related、signal_transduction | nicotinic acetylcholine-activated cation-selective channel | rheumatoid arthritis、schizophrenia |
| 481 | CYP7A1 | NM_000780.2 | 586 | ctgctaccgagtgatgtttgaag | 221198 | 672075 | - | steroid hormone receptor | - |
| 482 | CYP7A1 | NM_000780.2 | 1224 | tagctctttacccacagttaatg | 221215 | 672093 | see also 481 line | | |
| 483 | CYP24A1 | NM_000782.2 | 1219 | tggcctgggacaccattttcaaa | 221767 | 608692 | - | Retinoic acid receptor RXR-alpha | - |
| 484 | CYP26A1 | NM_000783.2 | 1039 | gagcaatcaagacaacaagttgg | 221583 | 608721 | - | - | - |
| 485 | CYP26A1 | NM_000783.2 | 1146 | ttcgggttgctctgaagactttt | 221585 | 608724 | see also 484 line | | |
| 486 | CYP26A1 | NM_000783.2 | 1148 | cgggttgctctgaagacttttga | 221586 | 608726 | see also 484 line | | |
| 487 | DBH | NM_000787.2 | 920 | tgggtgccaaggcattttactac | 162513 | 560127 | - | dopamine-beta-monooxygenase | congenital orthostatic hypotension、alcoholism |
| 488 | ACE | NM_000789.2 | 2678 | cagacctggtccaacatctatga | 154427 | 555577 | - | - | atherosclerosis、Alzheimer disease、diabetes |
| 489 | ACE | NM_000789.2 | 2992 | gggccacatccagtatttcatgc | 154434 | 555584 | see also 488 line | | |
| 490 | DRD2 | NM_000795.2 | 924 | caccgttatcatgaagtctaatg | 237892 | 624109 | receptor_acitivity、gpcr、signal_transduction | - | alcoholism、schizophrenia、Parkinson disease |
| 491 | DRD3 | NM_000796.2 | 560 | gacaggtggagtctggaatttca | 237899 | 624117 | receptor_acitivity、gpcr、signal_transduction | - | alcoholism、schizophrenia |
| 492 | FOLR2 | NM_000803.2 | 521 | ctggatgtgcccttatgcaaaga | 179656 | 572969 | receptor_acitivity | receptor | - |

Figure 46

| | | | | | | | channel、receptor_acitivity、signal_transduction | | |
|---|---|---|---|---|---|---|---|---|---|
| 493 | GABRA1 | NM_000806.3 | 1607 | ttggaatctttaacttagtctac | 178341 | 571601 | channel、receptor_acitivity、signal_transduction | GABA-A receptor | Parkinson disease |
| 494 | GABRA1 | NM_000806.3 | 1608 | tggaatctttaacttagtctact | 178342 | 571602 | see also 493 line | | |
| 495 | GABRA3 | NM_000808.2 | 994 | cagagataatccggtctagtaca | 178405 | 571671 | channel、receptor_acitivity、signal_transduction | benzodiazepine receptor | - |
| 496 | GABRA3 | NM_000808.2 | 996 | gagataatccggtctagtacagg | 178406 | 571672 | see also 495 line | | |
| 497 | GABRA3 | NM_000808.2 | 1018 | gagaatatgtcgtcatgacaacc | 178407 | 571674 | see also 495 line | | |
| 498 | GABRA3 | NM_000808.2 | 1570 | ctgagaccaagacctacaacagt | 178412 | 571684 | see also 495 line | | |
| 499 | GABRA4 | NM_000809.2 | 320 | aacaggctgcgtcctggatttgg | 178418 | 571692 | channel、receptor_acitivity、signal_transduction | benzodiazepine receptor | - |
| 500 | GABRA4 | NM_000809.2 | 1671 | ctggatctggcacaagtaaaata | 178456 | 571726 | see also 499 line | | |
| 501 | GABRA4 | NM_000809.2 | 1675 | atctggcacaagtaaaatagaca | 178458 | 571727 | see also 499 line | | |
| 502 | GABRA5 | NM_000810.2 | 1650 | cggcactttcaacttagtttact | 178486 | 571759 | channel、receptor_acitivity、signal_transduction | GABA-A receptor | alcoholism |
| 503 | GABRA6 | NM_000811.1 | 599 | accactttactcagtagaagtcc | 178501 | 571782 | channel、receptor_acitivity、signal_transduction | benzodiazepine receptor | alcoholism |
| 504 | GABRB1 | NM_000812.2 | 290 | tcgatgtcgccagcatagacatg | 178534 | 572443 | channel、receptor_acitivity、signal_transduction | GABA-A receptor | - |
| 505 | GABRB1 | NM_000812.2 | 789 | gtcactaagttttcgtctaaaga | 178542 | 572459 | see also 504 line | | |
| 506 | GABRB1 | NM_000812.2 | 791 | cactaagttttcgtctaaagaga | 178543 | 572460 | see also 504 line | | |
| 507 | GABRB1 | NM_000812.2 | 909 | agccagagtcgcactaggaatca | 178545 | 572464 | see also 504 line | | |
| 508 | GABRB2 | NM_000813.1 | 238 | ggggctactttgggatttggtcc | 178555 | 572479 | channel、receptor_acitivity、signal_transduction | - | alcoholism |
| 509 | GABRB2 | NM_000813.1 | 295 | gtgtcaatgaccctagtaatatg | 178557 | 572482 | see also 508 line | | |
| 510 | GABRB2 | NM_000813.1 | 308 | tagtaatatgtcgctggttaaag | 178558 | 572483 | see also 508 line | | |
| 511 | GABRB3 | NM_000814.3 | 449 | ctcaacctcacgcttgacaatcg | 178606 | 572525 | channel、receptor_acitivity、signal_transduction | - | alcoholism |

Figure 46

| | | | | | | | | | |
|---|---|---|---|---|---|---|---|---|---|
| 512 | GABRG2 | NM_000816.2 | 649 | gtggtatgacagacgtttgaaat | 178720 | 571925 | channel、receptor_acitivity、signal_transduction | benzodiazepine receptor | alcoholism、schizophrenia |
| 513 | GABRG2 | NM_000816.2 | 650 | tggtatgacagacgtttgaaatt | 178721 | 571926 | see also 512 line | | |
| 514 | GABRG2 | NM_000816.2 | 1615 | ctcctatgctcggatcttcttcc | 178744 | 571963 | see also 512 line | | |
| 515 | GAD1 | NM_000817.1 | 926 | aactatgtccgcaagacatttga | 233743 | 618087 | - | - | diabetes、Parkinson disease、schizophrenia |
| 516 | GAD2 | NM_000818.1 | 503 | atgtggtgaaaagtttcgataga | 233776 | 618132 | - | glutamate decarboxylase | diabetes mellitus、diabetes、Parkinson disease |
| 517 | GAD2 | NM_000818.1 | 506 | tggtgaaaagtttcgatagatca | 233777 | 618133 | see also 516 line | | |
| 518 | GAD2 | NM_000818.1 | 508 | gtgaaaagtttcgatagatcaac | 233778 | 618134 | see also 516 line | | |
| 519 | GAD2 | NM_000818.1 | 622 | atgcattgccaaacaactctaaa | 233781 | 618143 | see also 516 line | | |
| 520 | GGCX | NM_000821.2 | 1423 | gccccagatctactttgatattt | 233168 | 484418 | - | ligase | vitamin K-dependent coagulation defect |
| 521 | GGCX | NM_000821.2 | 1880 | ctggcatatctgcaagaattaaa | 233175 | 484437 | see also 520 line | | |
| 522 | GLRB | NM_000824.2 | 1399 | ttcagcattgttggaagcttacc | 179372 | 572682 | channel、receptor_acitivity、signal_transduction | glycine-inhibited chloride channel | - |
| 523 | GLRB | NM_000824.2 | 1409 | ttggaagcttaccaagagatttt | 179373 | 572683 | see also 522 line | | |
| 524 | GRIA2 | NM_000826.1 | 617 | gacagtgacagaggcttatcaac | 244269 | 632173 | receptor_acitivity、channel、signal_transduction | potassium channel | - |
| 525 | GRIA2 | NM_000826.1 | 2590 | gagcaacgttgctggagtattct | 244305 | 632213 | see also 524 line | | |
| 526 | GRIA2 | NM_000826.1 | 2677 | aagggccgaggcgaaacgaatga | 244306 | 632215 | see also 524 line | | |
| 527 | GRIA2 | NM_000826.1 | 2680 | ggccgaggcgaaacgaatgaagg | 244307 | 632216 | see also 524 line | | |
| 528 | GRIA1 | NM_000827.2 | 2405 | tgccctgagaaatccagtaaacc | 244250 | 632157 | receptor_acitivity、channel、signal_transduction | potassium channel | - |
| 529 | GRIA3 | NM_000828.2 | 291 | ggggcttttgggtcattctcacg | 244311 | 622076 | receptor_acitivity、channel、signal_transduction | - | - |
| 530 | GRIA3 | NM_000828.2 | 338 | taggtggacttttcatgagaaac | 244312 | 622078 | see also 529 line | | |
| 531 | GRIA3 | NM_000828.2 | 896 | aagtcgaaaggattaacacaatt | 244324 | 622090 | see also 529 line | | |
| 532 | GRIA3 | NM_000828.2 | 1126 | aagaatgcaccactaaagtatac | 244334 | 622094 | see also 529 line | | |
| 533 | GRIA3 | NM_000828.2 | 1358 | gacgtaggacaaattataccatc | 244337 | 622101 | see also 529 line | | |
| 534 | GRIA3 | NM_000828.2 | 1359 | acgtaggacaaattataccatcg | 244338 | 622102 | see also 529 line | | |
| 535 | GRIA3 | NM_000828.2 | 2808 | cacaaagaacacccaaaacttta | 244358 | 622138 | see also 529 line | | |

Figure 46

| | | | | | | | | | |
|---|---|---|---|---|---|---|---|---|---|
| 536 | GRIA4 | NM_000829.1 | 247 | ttctgttcccagtattctagagg | 244618 | 632236 | receptor_acitivity、channel、signal_transduction | potassium channel | - |
| 537 | GRIA4 | NM_000829.1 | 826 | atggatcgctggaagaaactaga | 244638 | 632257 | see also 536 line | | |
| 538 | GRIA4 | NM_000829.1 | 2272 | acgcccaagggttcctcattaag | 244673 | 632282 | see also 536 line | | |
| 539 | GRIA4 | NM_000829.1 | 2675 | caggattggctgtcattgcatcg | 244674 | 632291 | see also 536 line | | |
| 540 | GRIK1 | NM_000830.2 | 634 | caccacattaacctatgacatcc | 244369 | 632292 | receptor_acitivity、channel、signal_transduction | - | schizophrenia |
| 541 | GRIK1 | NM_000830.2 | 1944 | gggagtggaacgggatggttaaa | 244407 | 632325 | see also 540 line | | |
| 542 | GRIK1 | NM_000830.2 | 2209 | tacaccctacgagtggtataacc | 244417 | 632328 | see also 540 line | | |
| 543 | GRIK1 | NM_000830.2 | 2669 | gagtccaccagcattgagtatgt | 244423 | 632334 | see also 540 line | | |
| 544 | GRIN2A | NM_000833.2 | 2925 | atggagtgcacattgaagaaag | 165897 | 563328 | receptor_acitivity、channel、signal_transduction | magnesium binding | - |
| 545 | GRIN2A | NM_000833.2 | 4456 | cccctcggacccttacaaacact | 165916 | 563374 | see also 544 line | | |
| 546 | GRIN2B | NM_000834.2 | 534 | gcccagatcctcgatttcatttc | 165924 | 563388 | receptor_acitivity、channel、signal_transduction | magnesium binding | - |
| 547 | GRIN2B | NM_000834.2 | 535 | cccagatcctcgatttcatttca | 165925 | 563389 | see also 546 line | | |
| 548 | GRIN2B | NM_000834.2 | 2317 | atgcctacatgggaaagttcaac | 165958 | 563419 | see also 546 line | | |
| 549 | GRIN2B | NM_000834.2 | 2528 | gcgccaggtggaccttgctatcc | 165963 | 563422 | see also 546 line | | |
| 550 | GRM1 | NM_000838.2 | 352 | cagaatggacggagatgtcatca | 241558 | 629783 | gpcr、receptor_acitivity、signal_transduction | metabotropic glutamate、GABA-B-like receptor | - |
| 551 | GRM1 | NM_000838.2 | 1699 | agctaatcgctatgactatgtgc | 241579 | 629812 | see also 550 line | | |
| 552 | GRM3 | NM_000840.1 | 1574 | cggctccattcaacccaaataaa | 241642 | 629514 | gpcr、receptor_acitivity、signal_transduction | metabotropic glutamate、GABA-B-like receptor | - |
| 553 | GRM3 | NM_000840.1 | 2276 | atggcgctcagaggccaaaattc | 241668 | 629533 | see also 552 line | | |
| 554 | GRM3 | NM_000840.1 | 2277 | tggcgctcagaggccaaaattca | 241669 | 629534 | see also 552 line | | |
| 555 | GRM3 | NM_000840.1 | 2417 | agcgggaaacagtcatcctaaaa | 241672 | 629540 | see also 552 line | | |
| 556 | GRM3 | NM_000840.1 | 2420 | gggaaacagtcatcctaaaatgc | 241674 | 629542 | see also 552 line | | |
| 557 | GRM3 | NM_000840.1 | 2424 | aacagtcatcctaaaatgcaatg | 241675 | 629543 | see also 552 line | | |
| 558 | GRM3 | NM_000840.1 | 2450 | aagattccagcatgttgatctct | 241676 | 629544 | see also 552 line | | |

EP 1 752 536 A1

114

Figure 46

| | | | | | | | | | |
|---|---|---|---|---|---|---|---|---|---|
| 559 | GRM8 | NM_000845.1 | 481 | accaagcccgacaagatttctgg | 241825 | 629441 | gpcr、receptor_acitivity、signal_transduction | metabotropic glutamate、GABA-B-like receptor | - |
| 560 | GUCY1B3 | NM_000857.1 | 1509 | ctgttggtgacaagtatatgaca | 234199 | 618569 | receptor_acitivity、signal_transduction | receptor guanylate cyclase | - |
| 561 | HTR1F | NM_000866.1 | 892 | atcttgggtgcatttgtaatatg | 237585 | 625459 | receptor_acitivity、gpcr | serotonin receptor | - |
| 562 | HTR1F | NM_000866.1 | 1019 | cccttataaatccactgatttac | 237589 | 625462 | see also 561 line | | |
| 563 | HTR2C | NM_000868.1 | 811 | gcccagtagcagctatagtaact | 237633 | 625552 | receptor_acitivity、gpcr、signal_transduction | serotonin receptor | Alzheimer disease、alcoholism、schizophrenia |
| 564 | HTR2C | NM_000868.1 | 1968 | aacatttatcggcataccaatga | 237680 | 625598 | see also 563 line | | |
| 565 | HTR4 | NM_000870.2 | 1054 | ccgggttgaacccttttctctac | 237702 | 625624 | receptor_acitivity、gpcr | serotonin receptor | - |
| 566 | IGF1R | NM_000875.2 | 120 | ctggccgacgagtggagaaatct | 85643 | 504111 | - | insulin-like growth factor receptor | breast cancer、neuroblastoma、intraocular melanoma、Wilms tumor |
| 567 | IGF1R | NM_000875.2 | 122 | ggccgacgagtggagaaatctgc | 85644 | 504112 | see also 566 line | | |
| 568 | IGF1R | NM_000875.2 | 527 | tggatgcggtgtccaataactac | 85647 | 504118 | see also 566 line | | |
| 569 | IGF1R | NM_000875.2 | 530 | atgcggtgtccaataactacatt | 85648 | 504119 | see also 566 line | | |
| 570 | IGF1R | NM_000875.2 | 1207 | gtgaagatccgccattctcatgc | 85657 | 504133 | see also 566 line | | |
| 571 | IGF1R | NM_000875.2 | 3465 | ttgcatggtagccgaagatttca | 85699 | 504194 | see also 566 line | | |
| 572 | IGF2R | NM_000876.1 | 6848 | aggataagaccaagtctgtttct | 206300 | 635317 | receptor_acitivity、signal_transduction | transporter | hepatocellular carcinoma、Wilms tumor |
| 573 | IL7 | NM_000880.2 | 846 | tccgtgctgctcgcaagttgagg | 167530 | 564610 | - | interleukin-7 receptor ligand | - |
| 574 | IMPDH1 | NM_000883.2 | 1359 | cacccgtgaggatgacaaatacc | 220399 | 607727 | - | - | - |
| 575 | IMPDH1 | NM_000883.2 | 2085 | gaccatgtcggcccagattgagg | 220409 | 607742 | see also 574 line | | |
| 576 | ITGA4 | NM_000885.2 | 4194 | tacaaatctatcctacaagaaga | 249139 | 635660 | receptor_acitivity、signal_transduction | cell adhesion receptor | - |
| 577 | ITGA4 | NM_000885.2 | 4199 | atctatcctacaagaagaaaaca | 249140 | 635661 | see also 576 line | | |
| 578 | ITGB6 | NM_000888.3 | 595 | tgccaacccttgcagtagtattc | 249477 | 636098 | receptor_acitivity、signal_transduction | cell adhesion receptor | - |
| 579 | ITGB6 | NM_000888.3 | 2130 | ctccaaacattcccatgatcatg | 249511 | 636137 | see also 578 line | | |
| 580 | ITGB6 | NM_000888.3 | 2273 | aagtggcaaacgggaaccaatcc | 249517 | 636140 | see also 578 line | | |
| 581 | KCNJ5 | NM_000890.3 | 1326 | accttggaaaagggcttctatga | 260595 | 644201 | channel、gpcr | G-protein enhanced inward rectifier potassium channel | - |
| 582 | LTA4H | NM_000895.1 | 1001 | ttgggatcacttttggttaaatg | 157520 | 558128 | - | zinc binding | - |

Figure 46

| No. | Gene | Accession | | Sequence | | | Function | Description | Disease |
|---|---|---|---|---|---|---|---|---|---|
| 583 | MAOB | NM_000898.3 | 322 | accaaccagaatcgtatcttga | 225859 | 612498 | - | electron transporter | Parkinson disease |
| 584 | KITLG | NM_000899.1 | 456 | ttctgaaggcttgagtaattatt | 168209 | 565204 | signal_transduction | - | - |
| 585 | KITLG | NM_000899.1 | 911 | gtcttacaagggcagttgaaaat | 168220 | 565223 | see also 584 line | | - |
| 586 | MME | NM_000902.2 | 2300 | ctgccgcaagaattcatacatga | 158064 | 558608 | - | | leukemia，Alzheimer disease |
| 587 | MME | NM_000902.2 | 2302 | gccgcaagaattcatacatgaat | 158065 | 558609 | see also 586 line | | - |
| 588 | NPR2 | NM_000907.1 | 467 | tacacgggcacttcaattggact | 94941 | 510470 | kinase_related，recept or_acitivity，gpcr，si gnal_transduction | | |
| 589 | NPR2 | NM_000907.1 | 900 | accccaaatcctgagtatcagg | 94945 | 510477 | see also 588 line | | - |
| 590 | NPR2 | NM_000907.1 | 1330 | gacgacccatcctgataaaac | 94951 | 510485 | see also 588 line | | - |
| 591 | NPR2 | NM_000907.1 | 2404 | ctgctgccatggaacagtatgc | 94974 | 510514 | see also 588 line | | - |
| 592 | NPY2R | NM_000910.2 | 736 | atgcgcacagtaaccaactttt | 176060 | 571265 | receptor_acitivity，gpc r，signal_transduction | neuropeptide Y receptor | |
| 593 | P4HA1 | NM_000917.1 | 833 | cagagagctaatggtaacttaaa | 222271 | 609618 | | procollagen-proline，2-oxoglutarate-4-dioxygenase | |
| 594 | P4HA1 | NM_000917.1 | 1674 | aacgtggacaagaatttcgaaga | 222303 | 609651 | - | | - |
| 595 | PAM | NM_000919.2 | 963 | ctggcatgtaccttatgatgtct | 163786 | 560977 | see also 593 line | | - |
| 596 | PAM | NM_000919.2 | 1573 | aagggaagatgtgttcatgtgc | 163804 | 560993 | see also 595 line | | - |
| 597 | PC | NM_000920.2 | 1148 | ggcacggcaagcaactacttcatc | 95826 | 511462 | | | Leigh necrotizing encephalopathy |
| 598 | PC | NM_000920.2 | 2300 | tggaggctgccatctcatacacg | 95835 | 511475 | see also 597 line | | - |
| 599 | PDE3A | NM_000921.2 | 2112 | aagaaaatgtggccgtattctta | 198054 | 590063 | signal_transduction | cGMP-inhibited cyclic-nucleotide phosphodiesterase | |
| 600 | PDE3A | NM_000921.2 | 2363 | cagattctgacagtggatttaca | 198067 | 590069 | see also 599 line | | - |
| 601 | PDE3A | NM_000921.2 | 2367 | ttctgacagtgatttacacatg | 198068 | 590070 | see also 599 line | | - |
| 602 | PDHB | NM_000925.1 | 770 | aagctgcagcagttgctatctaaa | 225397 | 771138 | | pyruvate dehydrogenase (lipoamide) | - |
| 603 | PGR | NM_000926.2 | 2826 | ctggagtgcatcctgctacaaagc | 9185 | 447696 | transcription_regulator，receptor_acitivity，s ignal_transduction | steroid hormone receptor | - |
| 604 | ABCB1 | NM_000927.2 | 2536 | aactgaatggccttattttgttg | 69493 | 491830 | | Smad3/Sp-1 heterodimer | colchicin sensitivity，multiple drug resistance |
| 605 | ABCB1 | NM_000927.2 | 3056 | ttgtctggacaagcactgaaaga | 69504 | 491841 | see also 604 line | | - |
| 606 | PLCB4 | NM_000933.2 | 489 | atccagaagtaactaagcaatgg | 198348 | 590572 | - | | - |

Figure 46

| | | | | | | | | | |
|---|---|---|---|---|---|---|---|---|---|
| 607 | PLCB4 | NM_000933.2 | 787 | tcctcggacagatatagaagatc | 198358 | 590582 | see also 606 line | | |
| 608 | PLCB4 | NM_000933.2 | 791 | cggacagatatagaagatctttt | 198360 | 590583 | see also 606 line | | |
| 609 | PLCB4 | NM_000933.2 | 1372 | cagcaaatatcaacagtacaaga | 198376 | 590598 | see also 606 line | | |
| 610 | PLCB4 | NM_000933.2 | 1421 | ggggatctcctgttgaaacaagc | 198379 | 590603 | see also 606 line | | |
| 611 | PLCB4 | NM_000933.2 | 2094 | ccccagatttagcgatgcaattg | 198402 | 590627 | see also 606 line | | |
| 612 | PLCB4 | NM_000933.2 | 2097 | cagatttagcgatgcaattgaat | 198404 | 590628 | see also 606 line | | |
| 613 | PLCB4 | NM_000933.2 | 2568 | taccaacaattttctgcaatatt | 198425 | 590647 | see also 606 line | | |
| 614 | PLCB4 | NM_000933.2 | 2702 | ggcattgaaactagtgacatagc | 198431 | 590653 | see also 606 line | | |
| 615 | PLOD2 | NM_000935.1 | 1423 | gagatgaatgaaaggaactattt | 222200 | 609535 | - | - | - |
| 616 | POLR2B | NM_000938.1 | 197 | cagatgtctgttcaaagaattgt | 36277 | 464642 | transcription_regulator | DNA-directed RNA polymerase III | - |
| 617 | POLR2B | NM_000938.1 | 727 | ttcccgacccaccagtactatat | 36292 | 464663 | see also 616 line | | |
| 618 | POLR2B | NM_000938.1 | 728 | tcccgacccaccagtactatatg | 36293 | 464664 | see also 616 line | | |
| 619 | POLR2B | NM_000938.1 | 739 | cagtactatatgggttagcatgc | 36294 | 464666 | see also 616 line | | |
| 620 | POLR2B | NM_000938.1 | 1725 | ttgctgatgcaaccaagattttt | 36333 | 464698 | see also 616 line | | |
| 621 | POLR2B | NM_000938.1 | 1726 | tgctgatgcaaccaagatttttg | 36334 | 464699 | see also 616 line | | |
| 622 | POLR2B | NM_000938.1 | 1890 | cggatgcaggccgtatttgtaga | 36341 | 464713 | see also 616 line | | |
| 623 | POLR2B | NM_000938.1 | 2320 | gactacacggtctatggaatatc | 36355 | 464730 | see also 616 line | | |
| 624 | POLR2B | NM_000938.1 | 2323 | tacacggtctatggaatatctac | 36356 | 464731 | see also 616 line | | |
| 625 | PPP3R1 | NM_000945.2 | 675 | tccgtatctatgacatggataaa | 121131 | 552665 | phosphatase | calmodulin inhibitor | - |
| 626 | PRIM1 | NM_000946.1 | 149 | caccgtgaattttcattcacact | 208653 | 598403 | - | DNA primase | osteosarcoma |
| 627 | PRIM1 | NM_000946.1 | 258 | ttgatataggcgcagtatattct | 208656 | 598408 | see also 626 line | | |
| 628 | PRIM1 | NM_000946.1 | 281 | cacagacccaatcaacacaatac | 208657 | 598410 | see also 626 line | | |
| 629 | PRIM2A | NM_000947.1 | 1105 | aagtttgataaaggttactctta | 36952 | 464989 | - | DNA primase | - |
| 630 | PRIM2A | NM_000947.1 | 1154 | aaggcaagaggacagactataca | 36953 | 464992 | see also 629 line | | |
| 631 | RRM1 | NM_001033.2 | 1488 | acctgggaaccatcaaatgcagc | 226191 | 612911 | - | ribonucleoside-diphosphate reductase | - |
| 632 | RRM2 | NM_001034.1 | 442 | tccccatcgagtaccatgatatc | 226228 | 612952 | - | ribonucleoside-diphosphate reductase | - |
| 633 | RRM2 | NM_001034.1 | 443 | ccccatcgagtaccatgatatct | 226229 | 612953 | see also 632 line | | |
| 634 | RYR2 | NM_001035.1 | 2833 | tggtccggttagagatgacaaca | 122407 | 553142 | receptor_acitivity、channel、signal_transduction | receptor | ventricular tachycardia、arrhythmogenic right ventricular dysplasia |
| 635 | RYR2 | NM_001035.1 | 4440 | atgtctgggtgggctggattaca | 122450 | 553175 | see also 634 line | | |
| 636 | RYR2 | NM_001035.1 | 4442 | gtctgggtgggctggattacatc | 122451 | 553176 | see also 634 line | | |
| 637 | RYR2 | NM_001035.1 | 13550 | gagtcagcattctggaagaaaat | 122655 | 553343 | see also 634 line | | |

Figure 46

| 638 | RYR2 | NM_001035.1 | 13885 | tggatactactgcttgaaagtcc | 122670 | 553349 | see also 634 line | | |
| 639 | SCN1B | NM_001037.2 | 429 | caccagcgtcgtcaagaagatcc | 262232 | 647174 | channel | voltage-gated sodium channel | epilepsy |
| 640 | SLC6A3 | NM_001044.2 | 1732 | tggtcgtggtcagcattgtgacc | 256035 | 640137 | - | dopamine:sodium symporter | alcoholism |
| 641 | SLC12A2 | NM_001046.2 | 1019 | tggctggatcaagggtgtattag | 256692 | 640584 | - | symporter | - |
| 642 | SLC12A2 | NM_001046.2 | 1022 | ctggatcaagggtgtattagtac | 256693 | 640586 | see also 641 line | | |
| 643 | SLC12A2 | NM_001046.2 | 2079 | cagatgtttgctaaaggttatgg | 256724 | 640629 | see also 641 line | | |
| 644 | SLC12A2 | NM_001046.2 | 2273 | atggcgtcctgcattcaaatact | 256737 | 640643 | see also 641 line | | |
| 645 | SLC12A2 | NM_001046.2 | 2275 | ggcgtcctgcattcaaatactac | 256738 | 640644 | see also 641 line | | |
| 646 | SLC12A2 | NM_001046.2 | 2276 | gcgtcctgcattcaaatactaca | 256739 | 640645 | see also 641 line | | |
| 647 | SLC12A2 | NM_001046.2 | 2853 | atgagggatgtggatatgtatat | 256755 | 640669 | see also 641 line | | |
| 648 | SULT1C1 | NM_001056.2 | 829 | ttcctttatgtagctcgaaatgc | 217775 | 605789 | - | - | - |
| 649 | TACR2 | NM_001057.1 | 439 | agcaccaaggcggttattgctgg | 175980 | 629182 | gpcr、receptor_acitivity、signal_transduction | tachykinin receptor | - |
| 650 | TACR1 | NM_001058.2 | 938 | agcgcaaggtggtcaaaatgatg | 175975 | 629175 | receptor_acitivity、gpcr、signal_transduction | - | - |
| 651 | TBXAS1 | NM_001061.2 | 928 | atcatttccatccataatggtcc | 222083 | 609394 | - | - | thromboxane synthetase deficiency |
| 652 | TKT | NM_001064.1 | 431 | aggccagctaccgagtctattgc | 153885 | 554577 | - | transketolase | Wernicke-Korsakoff syndrome |
| 653 | TOP2A | NM_001067.2 | 320 | atgaagatgttggcattaactat | 42514 | 469461 | - | DNA topoisomerase (ATP-hydrolyzing) | colorectal cancer、esophageal cancer、lung cancer、small cell lung cancer、colorectal cancers |
| 654 | TOP2A | NM_001067.2 | 1279 | cccaagagctttggatcaacatg | 42546 | 469484 | see also 653 line | | |
| 655 | TOP2A | NM_001067.2 | 2102 | atgatcgaaaggaatggttaact | 42567 | 469510 | see also 653 line | | |
| 656 | TOP2B | NM_001068.2 | 417 | aggcattccagtagtagaacaca | 42659 | 469590 | - | DNA topoisomerase (ATP-hydrolyzing) | - |
| 657 | TOP2B | NM_001068.2 | 1050 | tggtaaactgattgaagtagtta | 42679 | 469619 | see also 656 line | | |
| 658 | TOP2B | NM_001068.2 | 1055 | aactgattgaagtagttaagaaa | 42680 | 469620 | see also 656 line | | |
| 659 | TOP2B | NM_001068.2 | 1088 | ctggtgtatcagtgaaaccattt | 42681 | 469621 | see also 656 line | | |
| 660 | TOP2B | NM_001068.2 | 1252 | gcctctaattgtggcattgtaga | 42684 | 469623 | see also 656 line | | |
| 661 | TOP2B | NM_001068.2 | 1254 | ctctaattgtggcattgtagaaa | 42685 | 469624 | see also 656 line | | |
| 662 | TOP2B | NM_001068.2 | 1995 | taccttggcatttagtaagaaga | 42704 | 469653 | see also 656 line | | |
| 663 | TOP2B | NM_001068.2 | 2242 | acctgtttcaagaggaatgataa | 42710 | 469671 | see also 656 line | | |
| 664 | TOP2B | NM_001068.2 | 2248 | ttcaagaggaatgataaacgtga | 42712 | 469672 | see also 656 line | | |
| 665 | TOP2B | NM_001068.2 | 2447 | ctgcaagccctcgttatattttc | 42718 | 469674 | see also 656 line | | |
| 666 | TOP2B | NM_001068.2 | 3174 | agcagaatctacaaagcttaaca | 42739 | 469706 | see also 656 line | | |

Figure 46

| 667 | TOP2B | NM_001068.2 | 3200 | aagcccgtttcattttagagaag | 42741 | 469709 | see also 656 line | | |
|---|---|---|---|---|---|---|---|---|---|
| 668 | TOP2B | NM_001068.2 | 3274 | atgttagtccagagaggttatga | 42742 | 469713 | see also 656 line | | |
| 669 | TOP2B | NM_001068.2 | 4179 | agcatctcccataacaaatgatg | 42764 | 469737 | see also 656 line | | |
| 670 | TOP2B | NM_001068.2 | 4604 | aaggccgaggggcaaagaaaagg | 42769 | 469749 | see also 656 line | | |
| 671 | TOP2B | NM_001068.2 | 4785 | cggtcgggctaggaaagaagtaa | 42772 | 469752 | see also 656 line | | |
| 672 | VCAM1 | NM_001078.2 | 695 | ttcttgtttgccgagctaaatta | 318025 | 682717 | - | - | atherosclerosis |
| 673 | VCAM1 | NM_001078.2 | 702 | ttgccgagctaaattacacattg | 318026 | 682718 | see also 672 line | | |
| 674 | VCAM1 | NM_001078.2 | 703 | tgccgagctaaattacacattga | 318027 | 682719 | see also 672 line | | |
| 675 | VCAM1 | NM_001078.2 | 705 | ccgagctaaattacacattgatg | 318028 | 682720 | see also 672 line | | |
| 676 | VCAM1 | NM_001078.2 | 2141 | ttggctcacaattaagaagttta | 318062 | 682756 | see also 672 line | | |
| 677 | PDE5A | NM_001083.2 | 2193 | atcatggaacaccatcattttga | 111263 | 520831 | signal_transduction | - | - |
| 678 | PDE5A | NM_001083.2 | 2321 | tacagacctagcactgtacatta | 111266 | 520836 | see also 677 line | | |
| 679 | PDE5A | NM_001083.2 | 2323 | cagacctagcactgtacattaag | 111267 | 520837 | see also 677 line | | |
| 680 | PDE5A | NM_001083.2 | 2326 | acctagcactgtacattaagagg | 111268 | 520838 | see also 677 line | | |
| 681 | PDE5A | NM_001083.2 | 2601 | atgcaagttgggttcatagatgc | 111277 | 520848 | see also 677 line | | |
| 682 | ACLY | NM_001096.2 | 1357 | tgggatccccatccatgtctttg | 71584 | 493200 | - | Sterol regulatory element binding proteins | - |
| 683 | ACLY | NM_001096.2 | 1358 | gggatccccatccatgtctttgg | 71585 | 493201 | see also 682 line | | |
| 684 | ACLY | NM_001096.2 | 1770 | accacaagcagaagttttactgg | 71590 | 493205 | see also 682 line | | |
| 685 | ACPP | NM_001099.2 | 929 | tacagatggcgctagatgtttac | 195118 | 589348 | phosphatase、cell_cycle | protein tyrosine phosphatase | prostate cancer |
| 686 | ACVR1 | NM_001105.2 | 621 | gccaaggggactggtgtaacagg | 71606 | 493238 | receptor_acitivity、signal_transduction、kinase_related | transforming growth factor-beta receptor | - |
| 687 | ACVR2B | NM_001106.2 | 232 | ctgctggctagatgacttcaact | 71740 | 493335 | receptor_acitivity、kinase_related、signal_transduction | transforming growth factor-beta receptor | - |
| 688 | ADAM10 | NM_001110.1 | 753 | ttgattatgatacctctcatatt | 154556 | 555676 | kinase_related | - | - |
| 689 | ADAM10 | NM_001110.1 | 1281 | tggtgaaacgcataagaatcaat | 154575 | 555692 | see also 688 line | | |
| 690 | ADAM10 | NM_001110.1 | 1287 | aacgcataagaatcaatacaact | 154576 | 555694 | see also 688 line | | |
| 691 | ADAM10 | NM_001110.1 | 1718 | atgtatgcaagagcaacatctgg | 154593 | 555701 | see also 688 line | | |
| 692 | ADAM10 | NM_001110.1 | 1738 | tggggacaaacttaacaacaata | 154594 | 555705 | see also 688 line | | |
| 693 | ADAM10 | NM_001110.1 | 2393 | ttcatgcggtgcagattagtaga | 154611 | 555716 | see also 688 line | | |
| 694 | ADAM10 | NM_001110.1 | 2396 | atgcggtgcagattagtagatgc | 154612 | 555717 | see also 688 line | | |
| 695 | ADAR | NM_001111.2 | 2938 | aggcttcatcaggtttctctaca | 48089 | 471673 | - | - | dyschromatosis symmetrica hereditaria |
| 696 | ADAR | NM_001111.2 | 3407 | gggcatctgacccgtgctatttg | 48102 | 471683 | see also 695 line | | |

Figure 46

| 697 | ADCY7 | NM_001114.1 | 2920 | gccggacttcaaagtgttctaca | 164984 | 562798 | - | | guanylate cyclase | alcoholism、Alzheimer disease |
|-----|-------|-------------|------|------------------------|--------|--------|---|---|-------------------|-------------------------------|
| 698 | ADCY8 | NM_001115.1 | 3318 | tcgctatgagaacgtcagtattc | 165005 | 562832 | - | | guanylate cyclase | - |
| 699 | ADCY8 | NM_001115.1 | 4079 | ctgggcctgaggaaattaacaag | 165017 | 562859 | see also 698 line | | | |
| 700 | ADCY8 | NM_001115.1 | 4192 | ctggagcacaagtattctcaaat | 165021 | 562864 | see also 698 line | | | |
| 701 | ADCY8 | NM_001115.1 | 4245 | ctgtgcatttatcgttcttctat | 165023 | 562868 | see also 698 line | | | |
| 702 | ADCY8 | NM_001115.1 | 4557 | ttcctcagctgtgtttacagata | 165033 | 562878 | see also 698 line | | | |
| 703 | ADCY8 | NM_001115.1 | 4558 | tcctcagctgtgtttacagatat | 165034 | 562879 | see also 698 line | | | |
| 704 | ADCY9 | NM_001116.1 | 1866 | tggcgcagactgtcaaaaccttt | 165073 | 562940 | signal_transduction | peptidyl-prolyl cis-trans isomerase | | - |
| 705 | ADCY9 | NM_001116.1 | 1867 | ggcgcagactgtcaaaacctttg | 165074 | 562941 | see also 704 line | | | |
| 706 | ADCY9 | NM_001116.1 | 2733 | tcgtgcactactgtaacttctgc | 165090 | 562956 | see also 704 line | | | |
| 707 | ADD1 | NM_001119.3 | 673 | ctcagcttatctacaatcatatc | 115165 | 526444 | - | - | | - |
| 708 | ADD1 | NM_001119.3 | 786 | atcaatctacaaggagatatagt | 115171 | 526448 | see also 707 line | | | |
| 709 | ADD1 | NM_001119.3 | 1131 | ttctattacatccataaccttgt | 115181 | 526456 | see also 707 line | | | |
| 710 | ADD1 | NM_001119.3 | 1358 | aactggctacccttatcgatacc | 115183 | 526458 | see also 707 line | | | |
| 711 | ADD1 | NM_001119.3 | 1590 | tcgaagactaagtggactaaaga | 115186 | 526467 | see also 707 line | | | |
| 712 | ADK | NM_001123.2 | 448 | tggatgcattgggatagataaat | 165108 | 598761 | kinase_related | - | | - |
| 713 | ADK | NM_001123.2 | 884 | gagcaaggctttgagactaaaga | 165126 | 598778 | see also 712 line | | | |
| 714 | ADSS | NM_001126.2 | 1337 | atgctcatatgatcaatggattt | 108151 | 518587 | - | adenylosuccinate synthase | | - |
| 715 | AP1B1 | NM_001127.2 | 1085 | gtggccctgcgcaacatcaatct | 267381 | 650284 | - | - | | meningioma |
| 716 | AP1B1 | NM_001127.2 | 1089 | ccctgcgcaacatcaatctcatc | 267382 | 650286 | see also 715 line | | | |
| 717 | AP1B1 | NM_001127.2 | 1740 | tgcgggaccgtggctacatctac | 267385 | 650291 | see also 715 line | | | |
| 718 | AP1B1 | NM_001127.2 | 1741 | gcgggaccgtggctacatctact | 267386 | 650292 | see also 715 line | | | |
| 719 | AP1G1 | NM_001128.4 | 555 | gacaaacgcattggctatttagg | 267397 | 650314 | - | protein transporter | | - |
| 720 | AP1G1 | NM_001128.4 | 616 | ttctcatgaccaactgtatcaag | 267400 | 650316 | see also 719 line | | | |
| 721 | AP1G1 | NM_001128.4 | 1063 | tacgaattttgcggttattaaga | 267414 | 650330 | see also 719 line | | | |
| 722 | AP1G1 | NM_001128.4 | 1074 | cggttattaagaattttaggacg | 267415 | 650332 | see also 719 line | | | |
| 723 | AP1G1 | NM_001128.4 | 1599 | ttgacaacggcaggaagttatgt | 267433 | 650355 | see also 719 line | | | |
| 724 | AP1G1 | NM_001128.4 | 1601 | gacaacggcaggaagttatgttc | 267434 | 650356 | see also 719 line | | | |
| 725 | AP1G1 | NM_001128.4 | 2649 | gggaccatcacacaagtcattaa | 267463 | 650385 | see also 719 line | | | |
| 726 | ALOX12B | NM_001139.1 | 805 | gggattcccaattctcatcaact | 164359 | 562122 | - | lipoxygenase | | - |
| 727 | ANGPT1 | NM_001146.3 | 561 | aagatataaccggattcaacatg | 171038 | 679381 | signal_transduction | - | | Alzheimer disease |
| 728 | ANGPT1 | NM_001146.3 | 581 | atgggcaatgtgcctacactttc | 171039 | 679382 | see also 727 line | | | |
| 729 | ANGPT1 | NM_001146.3 | 1910 | ggcccagttactccttacgttcc | 171068 | 679421 | see also 727 line | | | |
| 730 | ANK2 | NM_001148.2 | 2862 | gggcactgagaacttagacaacg | 286599 | 682378 | signal_transduction | - | | long QT syndrome |
| 731 | ANK2 | NM_001148.2 | 2876 | tagacaacgtggctctttcttct | 286600 | 682379 | see also 730 line | | | |

Figure 46

| | | | | | | | | | |
|---|---|---|---|---|---|---|---|---|---|
| 732 | ANK2 | NM_001148.2 | 4334 | tggtgcatcaagctatttgcaac | 286639 | 682418 | see also 730 line | | |
| 733 | ANXA6 | NM_001155.2 | 2080 | tccggagggaattcattgagaaa | 138007 | 544176 | - | - | - |
| 734 | APAF1 | NM_001160.2 | 1182 | cccagaggcttccacttaatatt | 72108 | 493852 | apoptosis | - | melanoma |
| 735 | APAF1 | NM_001160.2 | 1184 | cagaggcttccacttaatattga | 72109 | 493853 | see also 734 line | | |
| 736 | APAF1 | NM_001160.2 | 1186 | gaggcttccacttaatattgaag | 72110 | 493854 | see also 734 line | | |
| 737 | APAF1 | NM_001160.2 | 1343 | aagagtgttacagattcagtaat | 72114 | 493859 | see also 734 line | | |
| 738 | APAF1 | NM_001160.2 | 1345 | gagtgttacagattcagtaatgg | 72115 | 493860 | see also 734 line | | |
| 739 | APAF1 | NM_001160.2 | 2208 | ttggacgacagccatttcctaat | 72146 | 493879 | see also 734 line | | |
| 740 | APAF1 | NM_001160.2 | 4046 | cagaccttctacacaaatggaac | 72208 | 493917 | see also 734 line | | |
| 741 | NUDT2 | NM_001161.3 | 369 | gagcatgtggcttgatcatcttc | 192231 | 717104 | phosphatase、apoptosis | - | - |
| 742 | APBA1 | NM_001163.2 | 1541 | ttcccaacctacgttgaagttcc | 267862 | 680106 | - | protein transporter | Alzheimer disease |
| 743 | BIRC2 | NM_001166.2 | 2186 | gagtttgttgatgagattcaagg | 311123 | 676776 | apoptosis、signal_transduction、kinase_related | apoptosis inhibitor | - |
| 744 | ARHGAP5 | NM_001173.1 | 823 | atgatcaacttaaatttgtgaat | 192519 | 518704 | signal_transduction | - | - |
| 745 | ARHGAP5 | NM_001173.1 | 1166 | aacttggaaaactgttagtaata | 192529 | 518718 | see also 744 line | | |
| 746 | ARHGAP5 | NM_001173.1 | 1221 | atcaacttagagggaacaagaaa | 192531 | 518720 | see also 744 line | | |
| 747 | ARHGAP5 | NM_001173.1 | 2504 | tggtacatatcctcgtaaattta | 192566 | 518768 | see also 744 line | | |
| 748 | ARHGAP5 | NM_001173.1 | 2562 | ttggaatcagttaaacacaattt | 192569 | 518772 | see also 744 line | | |
| 749 | ARHGAP5 | NM_001173.1 | 2627 | agctgacttgagaattgtcatgt | 192572 | 518776 | see also 744 line | | |
| 750 | ARHGAP5 | NM_001173.1 | 2722 | ctgctcaagctggacagaataat | 192576 | 518777 | see also 744 line | | |
| 751 | ARHGAP5 | NM_001173.1 | 2723 | tgctcaagctggacagaataatt | 192577 | 518778 | see also 744 line | | |
| 752 | ARHGAP5 | NM_001173.1 | 2781 | aggcgaatacagatcacaatatt | 192579 | 518782 | see also 744 line | | |
| 753 | ARHGAP5 | NM_001173.1 | 2782 | ggcgaatacagatcacaatatta | 192580 | 518783 | see also 744 line | | |
| 754 | ARHGAP5 | NM_001173.1 | 3408 | gtgcctccacctattaaacctaa | 192592 | 518808 | see also 744 line | | |
| 755 | ARHGAP5 | NM_001173.1 | 3409 | tgcctccacctattaaacctaaa | 192593 | 518809 | see also 744 line | | |
| 756 | ARHGAP5 | NM_001173.1 | 3410 | gcctccacctattaaacctaaac | 192594 | 518810 | see also 744 line | | |
| 757 | ARHGAP5 | NM_001173.1 | 3425 | acctaaaccagttgtacctaaga | 192595 | 518811 | see also 744 line | | |
| 758 | ARHGAP5 | NM_001173.1 | 3603 | aagggctattctgatgagattta | 192599 | 518816 | see also 744 line | | |
| 759 | ARHGAP5 | NM_001173.1 | 3781 | tagtaaagccaagtcatactata | 192611 | 518824 | see also 744 line | | |
| 760 | ARHGAP5 | NM_001173.1 | 4426 | atcctgtaaactatgatgtattc | 192629 | 518844 | see also 744 line | | |
| 761 | ARHGAP5 | NM_001173.1 | 4429 | ctgtaaactatgatgtattcaga | 192630 | 518845 | see also 744 line | | |
| 762 | ARHGAP5 | NM_001173.1 | 4510 | cagacaacttatccatctgtttt | 192636 | 518851 | see also 744 line | | |
| 763 | ARHGAP5 | NM_001173.1 | 4780 | cggatcctcttggtattatatga | 192645 | 518862 | see also 744 line | | |
| 764 | ARHGAP6 | NM_001174.2 | 3049 | cggagacatctccccttatgaca | 252534 | 665231 | signal_transduction | - | - |
| 765 | ARHGAP6 | NM_001174.2 | 3051 | gagacatctcccttatgacaac | 252535 | 665232 | see also 764 line | | |

Figure 46

| | | | | | | | | | |
|---|---|---|---|---|---|---|---|---|---|
| 766 | ARHGAP6 | NM_001174.2 | 3264 | ggggaacttggcattcaacatta | 252549 | 665235 | see also 764 line | | |
| 767 | ARNTL | NM_001178.2 | 535 | gaccctcatggaaggttagaata | 981 | 441789 | transcription_regulator、signal_transduction | transcription factor | - |
| 768 | ARNTL | NM_001178.2 | 536 | accctcatggaaggttagaatat | 982 | 441790 | see also 767 line | | |
| 769 | ARNTL | NM_001178.2 | 537 | ccctcatggaaggttagaatata | 983 | 441791 | see also 767 line | | |
| 770 | ARNTL | NM_001178.2 | 1620 | gagtcgatggttcagtttcatga | 1016 | 441817 | see also 767 line | | |
| 771 | BAPX1 | NM_001189.2 | 41 | tccaggcgatcctcaacaagaaa | 1363 | 442040 | transcription_regulator | RNA polymerase II transcription factor | - |
| 772 | BCL2L1 | NM_001191.2 | 430 | tacagctggagtcagtttagtga | 311005 | 676924 | apoptosis | - | leukemia |
| 773 | BCL2L1 | NM_001191.2 | 432 | cagctggagtcagtttagtgatg | 311006 | 676925 | see also 772 line | | |
| 774 | PRDM1 | NM_001198.2 | 767 | acgggatgaacatctacttctac | 9804 | 464901 | transcription_regulator | - | - |
| 775 | PRDM1 | NM_001198.2 | 768 | cgggatgaacatctacttctaca | 9805 | 464902 | see also 774 line | | |
| 776 | PRDM1 | NM_001198.2 | 807 | tgccaaccaggaacttcttgtgt | 9806 | 464903 | see also 774 line | | |
| 777 | PRDM1 | NM_001198.2 | 2025 | cagtggagaacggcctttcaaat | 9840 | 464931 | see also 774 line | | |
| 778 | PRDM1 | NM_001198.2 | 2135 | aggtctgccacaagagatttagc | 9843 | 464933 | see also 774 line | | |
| 779 | BMP1 | NM_001199.1 | 1619 | ctctgacgggtccattaacaaag | 138155 | 544499 | - | - | amyloidosis |
| 780 | BMP2 | NM_001200.1 | 1064 | caggtctttgcaccaagatgaac | 168349 | 565363 | - | growth factor | - |
| 781 | BMP2 | NM_001200.1 | 1084 | aacacagctggtcacagataagg | 168350 | 565364 | see also 780 line | | |
| 782 | BMP4 | NM_001202.2 | 492 | ccgaatgctgatggtcgttttat | 168392 | 565371 | - | - | - |
| 783 | BMP4 | NM_001202.2 | 496 | atgctgatggtcgttttattatg | 168393 | 565372 | see also 782 line | | |
| 784 | BMP4 | NM_001202.2 | 534 | aggcgcgagccatgctagtttga | 168395 | 565373 | see also 782 line | | |
| 785 | BMP4 | NM_001202.2 | 995 | ggggcttccaccgtataaacatt | 168403 | 565380 | see also 782 line | | |
| 786 | BMP4 | NM_001202.2 | 996 | gggcttccaccgtataaacattt | 168404 | 565381 | see also 782 line | | |
| 787 | BMP4 | NM_001202.2 | 997 | ggcttccaccgtataaacattta | 168405 | 565382 | see also 782 line | | |
| 788 | BMPR2 | NM_001204.4 | 1269 | gagcgtccagttgctgtaaaagt | 74173 | 496235 | receptor_acitivity、kinase_related、signal_transduction | - | primary pulmonary hypertension |
| 789 | BMPR2 | NM_001204.4 | 1555 | taccacgaggagatcattataaa | 74188 | 496245 | see also 788 line | | |
| 790 | BMPR2 | NM_001204.4 | 2179 | atgaacgcaacctgtcacataat | 74204 | 496256 | see also 788 line | | |
| 791 | BMPR2 | NM_001204.4 | 2182 | aacgcaacctgtcacataatagg | 74205 | 496257 | see also 788 line | | |
| 792 | BMPR2 | NM_001204.4 | 3318 | agcaatccatgttcagaacaaga | 74236 | 496289 | see also 788 line | | |
| 793 | BMPR2 | NM_001204.4 | 3420 | ctggatctttcagccacaaatgt | 74239 | 496291 | see also 788 line | | |
| 794 | C1QBP | NM_001212.3 | 787 | gaccacctaatggatttccttgc | 313362 | 687499 | - | - | - |
| 795 | CALU | NM_001219.2 | 455 | tgccacctacggctacgttttag | 138965 | 545195 | - | calcium ion binding | - |
| 796 | CALU | NM_001219.2 | 456 | gccacctacggctacgttttaga | 138966 | 545196 | see also 795 line | | |

Figure 46

EP 1 752 536 A1

| 797 | CALU | NM_001219.2 | 458 | cacctacggctacgttttagatg | 138967 | 545197 | see also 795 line | | |
|---|---|---|---|---|---|---|---|---|---|
| 798 | CALU | NM_001219.2 | 459 | acctacggctacgttttagatga | 138968 | 545198 | see also 795 line | | |
| 799 | CALU | NM_001219.2 | 462 | tacggctacgttttagatgatcc | 138969 | 545199 | see also 795 line | | |
| 800 | CAMK2B | NM_001220.3 | 1362 | cccagtggacgggattaaggagt | 74991 | 497003 | kinase_related、signal_transduction | - | schizophrenia |
| 801 | CAMK2D | NM_001221.2 | 978 | tggcttagccatagaagttcaag | 75005 | 497015 | kinase_related | - | - |
| 802 | CAMK2D | NM_001221.2 | 1061 | tacgtaaagatccttatggaaag | 75008 | 497019 | see also 801 line | | |
| 803 | CAMK2G | NM_001222.2 | 379 | acctcgtgtttgaccttgttacc | 75033 | 497038 | kinase_related、phosphatase、signal_transduction | - | - |
| 804 | CAMK2G | NM_001222.2 | 643 | ctggcaccccaggttacttgtcc | 75039 | 497046 | see also 803 line | | |
| 805 | CASP1 | NM_001223.2 | 401 | ggcagagatttatccaataatgg | 187324 | 581022 | apoptosis、signal_transduction、kinase_related | - | - |
| 806 | CASP6 | NM_001226.2 | 306 | caggttttcagatctaggatttg | 187440 | 581174 | apoptosis | - | Alzheimer disease |
| 807 | CASP8 | NM_001228.2 | 995 | agcaaacctcggggatactgtct | 187514 | 581222 | apoptosis、kinase_related | - | neuroblastoma、caspase 8 deficiency |
| 808 | CASP8 | NM_001228.2 | 1005 | ggggatactgtctgatcatcaac | 187516 | 581223 | see also 807 line | | |
| 809 | CCNA2 | NM_001237.2 | 690 | acctaaagtgggttacatgaaga | 306565 | 673594 | cell_cycle、kinase_related | Transcription factor E2F4 | |
| 810 | CCNE1 | NM_001238.1 | 1139 | atcagtggtgcgacatagagaac | 307298 | 674473 | cell_cycle | - | bladder cancer |
| 811 | CCNT1 | NM_001240.2 | 523 | atcgattctacatgattcagtcc | 300273 | 668722 | cell_cycle、transcription_regulator、kinase_related | cyclin-dependent protein kinase、regulator | - |
| 812 | CDC27 | NM_001256.2 | 345 | tacctgcttgcaaaatgttgtgt | 307763 | 675223 | - | - | - |
| 813 | CDC27 | NM_001256.2 | 541 | accaaaagagccttagtttaaat | 307768 | 675230 | see also 812 line | | |
| 814 | CDC27 | NM_001256.2 | 610 | agccagatcctgaccaaacattt | 307770 | 675234 | see also 812 line | | |
| 815 | CDC27 | NM_001256.2 | 611 | gccagatcctgaccaaacattta | 307771 | 675235 | see also 812 line | | |
| 816 | CDC27 | NM_001256.2 | 911 | accaaaaactggtcgaagtttat | 307785 | 675256 | see also 812 line | | |
| 817 | CDC27 | NM_001256.2 | 1006 | gagatggatcctatttacaaaac | 307790 | 675259 | see also 812 line | | |
| 818 | CDH13 | NM_001257.2 | 1190 | atgatcactcaccaaaattcacc | 139531 | 545556 | - | calcium ion binding | breast cancer、lung cancer、non-small-cell lung cancer |
| 819 | CDK6 | NM_001259.2 | 420 | atcaagacttgaccacttacttg | 76173 | 497794 | kinase_related、cell_cycle | protein serine/threonine kinase | - |
| 820 | CDK6 | NM_001259.2 | 608 | ggccttgcccgcatctatagttt | 76180 | 497798 | see also 819 line | | |
| 821 | CDK6 | NM_001259.2 | 609 | gccttgcccgcatctatagtttc | 76181 | 497799 | see also 819 line | | |

123

Figure 46

| | | | | | | | | | |
|---|---|---|---|---|---|---|---|---|---|
| 822 | CDKN2C | NM_001262.2 | 1260 | gggggggacctagagcaacttact | 294098 | 674758 | kinase_related、cell_cycle | - | dysplastic nevus syndrome |
| 823 | CDKN2C | NM_001262.2 | 1262 | ggggacctagagcaacttactag | 294099 | 674759 | see also 822 line | | |
| 824 | CDKN2C | NM_001262.2 | 1265 | gacctagagcaacttactagttt | 294100 | 674761 | see also 822 line | | |
| 825 | CDKN2C | NM_001262.2 | 1266 | acctagagcaacttactagtttg | 294101 | 674762 | see also 822 line | | |
| 826 | CDKN2C | NM_001262.2 | 1336 | tgcgctgcaggttatgaaacttg | 294104 | 674767 | see also 822 line | | |
| 827 | CDS1 | NM_001263.2 | 1176 | gtcatccaaaatctgtttgaagg | 165228 | 598556 | signal_transduction | phosphatidate cytidylyltransferase | - |
| 828 | CHC1L | NM_001268.2 | 1163 | tggctacgcacacacattagtat | 129941 | 533680 | - | - | - |
| 829 | CHC1L | NM_001268.2 | 1167 | tacgcacacacattagtattaac | 129943 | 533681 | see also 828 line | | |
| 830 | CHC1L | NM_001268.2 | 1920 | aaccatctgactgtagtaacaca | 129962 | 533697 | see also 828 line | | |
| 831 | CHC1 | NM_001269.2 | 289 | gtcacccaagcgcatagctaaaa | 24332 | 748887 | cell_cycle | guanyl-nucleotide release factor | - |
| 832 | CHD1 | NM_001270.1 | 1904 | accaaacggttaaaatttaatat | 18113 | 450615 | transcription_regulator | chromatin binding | - |
| 833 | CHD1 | NM_001270.1 | 2460 | aaccaccagataataatgaattc | 18140 | 450628 | see also 832 line | | |
| 834 | CHD1 | NM_001270.1 | 2492 | caggaggccttacaacacttaat | 18141 | 450630 | see also 832 line | | |
| 835 | CHD1 | NM_001270.1 | 2493 | aggaggccttacaacacttaatt | 18142 | 450631 | see also 832 line | | |
| 836 | CHD1 | NM_001270.1 | 3559 | gcggtttatcaagagctataaga | 18176 | 450665 | see also 832 line | | |
| 837 | CHD1 | NM_001270.1 | 3569 | aagagctataagaaatttggtgg | 18177 | 450666 | see also 832 line | | |
| 838 | CHD4 | NM_001273.1 | 4170 | gacaaccagtccgattactcagt | 2213 | 442692 | transcription_regulator | zinc binding | rheumatoid arthritis |
| 839 | CHD4 | NM_001273.1 | 4257 | aagggcctgcggaatgataaaga | 2214 | 442694 | see also 838 line | | |
| 840 | CHEK1 | NM_001274.2 | 223 | aagttcaacttgctgtgaataga | 76833 | 498139 | kinase_related、cell_cycle、signal_transduction | protein serine/threonine kinase | - |
| 841 | CHEK1 | NM_001274.2 | 735 | tcctgtggaatagtacttactgc | 76852 | 498164 | see also 840 line | | |
| 842 | CHK | NM_001277.1 | 923 | ttgtattttgtcataatgactgt | 208466 | 598098 | kinase_related | signal transducer | - |
| 843 | CHK | NM_001277.1 | 938 | atgactgtcaagaaggtaatatc | 208467 | 598099 | see also 842 line | | |
| 844 | CHUK | NM_001278.2 | 779 | tacctggcatgagaagattaaga | 76933 | 498248 | kinase_related | transcription factor | - |
| 845 | CHUK | NM_001278.2 | 841 | caggagaagttcggtttagtagc | 76935 | 498249 | see also 844 line | | |
| 846 | CHUK | NM_001278.2 | 1449 | atgttaagtcttcttagatataa | 76958 | 498278 | see also 844 line | | |
| 847 | CLCN6 | NM_001286.1 | 691 | tacggaagatccagtttaacttc | 263417 | 647057 | channel、signal_transduction | - | - |
| 848 | CLIC1 | NM_001288.3 | 847 | ggggagtgcatcggtacttgagc | 263482 | 647125 | channel | voltage-gated chloride channel | Alzheimer disease |
| 849 | LDB2 | NM_001290.1 | 144 | ctcctttcggcccattttatagg | 30448 | 461162 | transcription_regulator | - | - |

Figure 46

| 850 | LDB2 | NM_001290.1 | 192 | cagagtaccgaatctatgagatg | 30450 | 461163 | see also 849 line | | |
|---|---|---|---|---|---|---|---|---|---|
| 851 | LDB2 | NM_001290.1 | 327 | atggaccaaagcgatacactatc | 30453 | 461172 | see also 849 line | | |
| 852 | LDB2 | NM_001290.1 | 328 | tggaccaaagcgatacactatcg | 30454 | 461173 | see also 849 line | | |
| 853 | LDB2 | NM_001290.1 | 496 | cgggaagcccatgtttaccaagg | 30465 | 461180 | see also 849 line | | |
| 854 | LDB2 | NM_001290.1 | 1052 | gaggacgaaaggctaatcactag | 30476 | 461185 | see also 849 line | | |
| 855 | LDB2 | NM_001290.1 | 1053 | aggacgaaaggctaatcactaga | 30477 | 461186 | see also 849 line | | |
| 856 | LDB2 | NM_001290.1 | 1060 | aaggctaatcactagattagaaa | 30478 | 461188 | see also 849 line | | |
| 857 | LDB2 | NM_001290.1 | 1154 | aacagcccgtggaacagtaaacc | 30482 | 461189 | see also 849 line | | |
| 858 | CNGA3 | NM_001298.1 | 1346 | gggttatccggtggtttgactac | 107955 | 517887 | channel、signal_transduction | potassium channel | achondroplasia |
| 859 | CPD | NM_001304.3 | 744 | gtggatccgcaggaacaagtttg | 185792 | 579930 | - | - | - |
| 860 | CPD | NM_001304.3 | 745 | tggatccgcaggaacaagtttgt | 185793 | 579931 | see also 859 line | | |
| 861 | CPD | NM_001304.3 | 794 | cagtggtagcaagctatcctttt | 185795 | 579932 | see also 859 line | | |
| 862 | CPD | NM_001304.3 | 796 | gtggtagcaagctatcctttttga | 185796 | 579933 | see also 859 line | | |
| 863 | CPD | NM_001304.3 | 1122 | tcgtgagtctttgatcacattga | 185807 | 579947 | see also 859 line | | |
| 864 | CPD | NM_001304.3 | 1221 | ctcagtggctggtattaatcata | 185811 | 579952 | see also 859 line | | |
| 865 | CPD | NM_001304.3 | 2840 | ttctgagaggacttgtaatgaac | 185882 | 580006 | see also 859 line | | |
| 866 | CPD | NM_001304.3 | 3504 | cagcatgaaggattatagtgtca | 185899 | 580028 | see also 859 line | | |
| 867 | CPD | NM_001304.3 | 3881 | cagataaccggatatttggtttg | 185917 | 580038 | see also 859 line | | |
| 868 | CPD | NM_001304.3 | 4049 | aagatgaaattcgcatgatgtct | 185927 | 580045 | see also 859 line | | |
| 869 | CSE1L | NM_001316.2 | 1177 | ttgtgcctaacatggaatttaga | 128369 | 530499 | receptor_acitivity、apoptosis | - | - |
| 870 | CSE1L | NM_001316.2 | 1789 | ttctgctaacaaacctttcaaa | 128388 | 530523 | see also 869 line | | |
| 871 | CSE1L | NM_001316.2 | 2403 | cacatgcctcctgaatcagttga | 128402 | 530537 | see also 869 line | | |
| 872 | CSNK2B | NM_001320.5 | 428 | atccaggacaaatttaatcttac | 205578 | 596375 | kinase_related、signal_transduction | protein kinase CK2、regulator | - |
| 873 | CSNK2B | NM_001320.5 | 942 | tccaagccgccagcaacttcaag | 205589 | 596383 | see also 872 line | | |
| 874 | CSTF2 | NM_001325.1 | 591 | ttctgcatcgccagacaaatatc | 50218 | 482223 | - | RNA binding | - |
| 875 | CSTF2 | NM_001325.1 | 722 | ttgggtggaatgcatgtcaatgg | 50224 | 482226 | see also 874 line | | |
| 876 | CSTF3 | NM_001326.1 | 254 | ggcacagaatcaacctatagaca | 50236 | 670535 | - | - | - |
| 877 | CSTF3 | NM_001326.1 | 550 | atcagatttgggtggattacatc | 50244 | 670545 | see also 876 line | | |
| 878 | CSTF3 | NM_001326.1 | 629 | agctgtccgaagagtttatcaac | 50246 | 670549 | see also 876 line | | |
| 879 | CSTF3 | NM_001326.1 | 779 | tagacgtgtagcaaaggaatatg | 50254 | 670556 | see also 876 line | | |
| 880 | CSTF3 | NM_001326.1 | 1177 | atgaagagagtcgcatgaagtat | 50266 | 670565 | see also 876 line | | |
| 881 | CSTF3 | NM_001326.1 | 1495 | ctcacctcaatgaggacaataat | 50282 | 670576 | see also 876 line | | |
| 882 | CSTF3 | NM_001326.1 | 1497 | cacctcaatgaggacaataatac | 50283 | 670577 | see also 876 line | | |
| 883 | CSTF3 | NM_001326.1 | 2186 | aaggcccaacgaggattcagatg | 50301 | 670589 | see also 876 line | | |

Figure 46

| | | | | | | | | | |
|---|---|---|---|---|---|---|---|---|---|
| 884 | CTNND1 | NM_001331.1 | 666 | gtccgggtctcaccacaagatgc | 278921 | 660498 | - | structural molecule | - |
| 885 | CTNND1 | NM_001331.1 | 1820 | ctgtggagctctcaagaatatct | 278941 | 660526 | see also 884 line | | |
| 886 | CTNND1 | NM_001331.1 | 1916 | tcgaaaggctcgtgatatggacc | 278945 | 660532 | see also 884 line | | |
| 887 | DAB2 | NM_001343.1 | 145 | atgtctaacgaagtagaaacaag | 309080 | 534276 | - | - | - |
| 888 | DAB2 | NM_001343.1 | 598 | gggcaacaggctgaaccattagt | 309087 | 534291 | see also 887 line | | |
| 889 | DAB2 | NM_001343.1 | 847 | ctgttagtggatctaaactctga | 309091 | 534303 | see also 887 line | | |
| 890 | DAB2 | NM_001343.1 | 1468 | ggcagaaggactgctaagtcttc | 309099 | 534315 | see also 887 line | | |
| 891 | DGKG | NM_001346.1 | 1562 | accggtgccacaaaagtatcaag | 141743 | 547240 | kinase_related | diacylglycerol kinase | - |
| 892 | DGKG | NM_001346.1 | 1563 | ccggtgccacaaaagtatcaagt | 141744 | 547241 | see also 891 line | | |
| 893 | DGKG | NM_001346.1 | 1627 | atgacgtttcaccgcaaatgtga | 141745 | 547242 | see also 891 line | | |
| 894 | DGKG | NM_001346.1 | 1629 | gacgtttcaccgcaaatgtgaat | 141746 | 547243 | see also 891 line | | |
| 895 | DGKG | NM_001346.1 | 1634 | ttcaccgcaaatgtgaattatca | 141748 | 547244 | see also 891 line | | |
| 896 | DARS | NM_001349.1 | 275 | tacgtgcaagagttcatacaagc | 58927 | 482667 | - | nucleic acid binding | Alzheimer disease |
| 897 | DARS | NM_001349.1 | 378 | tgcaagcaagcagatggttaaat | 58928 | 482669 | see also 896 line | | |
| 898 | DARS | NM_001349.1 | 1276 | ttggctgtaagacctttctatac | 58955 | 482695 | see also 896 line | | |
| 899 | DARS | NM_001349.1 | 1277 | tggctgtaagacctttctatacc | 58956 | 482696 | see also 896 line | | |
| 900 | DARS | NM_001349.1 | 1280 | ctgtaagacctttctataccatg | 58957 | 482697 | see also 896 line | | |
| 901 | DDX3X | NM_001356.2 | 2492 | aacgagaggaacataaatattac | 24899 | 457706 | - | - | - |
| 902 | DHX9 | NM_001357.2 | 259 | atgctgccagagactttgttaac | 25023 | 458216 | - | - | - |
| 903 | DHX9 | NM_001357.2 | 3014 | tactaacactggaccagataata | 25088 | 458315 | see also 902 line | | |
| 904 | DHX9 | NM_001357.2 | 3020 | cactggaccagataataatttgg | 25090 | 458316 | see also 902 line | | |
| 905 | DHX9 | NM_001357.2 | 3067 | ttggtgtgtaccccaatgtatgc | 25091 | 458318 | see also 902 line | | |
| 906 | DHX9 | NM_001357.2 | 3072 | gtgtaccccaatgtatgctatca | 25092 | 458319 | see also 902 line | | |
| 907 | DHX9 | NM_001357.2 | 3075 | taccccaatgtatgctatcataa | 25093 | 458320 | see also 902 line | | |
| 908 | DHX9 | NM_001357.2 | 3076 | accccaatgtatgctatcataag | 25094 | 458321 | see also 902 line | | |
| 909 | DHX9 | NM_001357.2 | 3077 | ccccaatgtatgctatcataagg | 25095 | 458322 | see also 902 line | | |
| 910 | DHX9 | NM_001357.2 | 3503 | agctgctggtatcaaccttatga | 25109 | 458330 | see also 902 line | | |
| 911 | DHX15 | NM_001358.1 | 524 | accccatactcctcgatactatg | 59548 | 483446 | - | RNA helicase | - |
| 912 | DHX15 | NM_001358.1 | 525 | ccccatactcctcgatactatga | 59549 | 483447 | see also 911 line | | |
| 913 | DHX15 | NM_001358.1 | 530 | tactcctcgatactatgatattc | 59550 | 483448 | see also 911 line | | |
| 914 | DHX15 | NM_001358.1 | 533 | tcctcgatactatgatattctaa | 59551 | 483449 | see also 911 line | | |
| 915 | DHX15 | NM_001358.1 | 535 | ctcgatactatgatattctaaag | 59552 | 483450 | see also 911 line | | |
| 916 | DHX15 | NM_001358.1 | 1007 | gagatcagatttaaaggttatag | 59563 | 483462 | see also 911 line | | |
| 917 | DHX15 | NM_001358.1 | 1010 | atcagatttaaaggttatagtta | 59564 | 483463 | see also 911 line | | |
| 918 | DHX15 | NM_001358.1 | 1062 | cagatttactttgataactgtcc | 59566 | 483468 | see also 911 line | | |
| 919 | DHX15 | NM_001358.1 | 1260 | aagagaataaagcgtgaagttga | 59575 | 483471 | see also 911 line | | |
| 920 | DHX15 | NM_001358.1 | 1262 | gagaataaagcgtgaagttgatg | 59576 | 483472 | see also 911 line | | |

Figure 46

| 921 | DHX15 | NM_001358.1 | 1990 | cagatgaggccaagatgagattt | 59595 | 483496 | see also 911 line | | |
|---|---|---|---|---|---|---|---|---|---|
| 922 | DHX15 | NM_001358.1 | 2201 | tacaagcagggactattatatta | 59605 | 483509 | see also 911 line | | |
| 923 | DECR1 | NM_001359.1 | 662 | tcgtgacactagaaattggaaaa | 224269 | 613899 | - | 2、4-dienoyl-CoA reductase (NADPH) | - |
| 924 | DHODH | NM_001361.1 | 255 | ggccataaattccgaaatccagt | 223970 | 612202 | - | dihydroorotate oxidase | - |
| 925 | DLG2 | NM_001364.1 | 2519 | gccagtacaatgacaatttatat | 209272 | 680961 | kinase_related | guanylate kinase | |
| 926 | DLG2 | NM_001364.1 | 2818 | aaccaatgcaagcttgttattga | 209279 | 680971 | see also 925 line | | |
| 927 | DLG4 | NM_001365.1 | 2475 | gacgagagtggtcaaggttaaag | 126176 | 643153 | kinase_related、signal _transduction | protein C-terminus binding | - |
| 928 | DLG4 | NM_001365.1 | 2644 | ctccgagttccccgacaagtttg | 126177 | 643157 | see also 927 line | | |
| 929 | DLG4 | NM_001365.1 | 2645 | tccgagttccccgacaagtttgg | 126178 | 643158 | see also 927 line | | |
| 930 | DNAH5 | NM_001369.1 | 1152 | tcctacacttataaatgcaatta | 79139 | 499970 | - | - | - |
| 931 | DNAH5 | NM_001369.1 | 3463 | ttcaaacgctacaatcacatttg | 79210 | 500028 | see also 930 line | | |
| 932 | DNAH5 | NM_001369.1 | 4837 | aggtacaatatgccattcaaagc | 79252 | 500055 | see also 930 line | | |
| 933 | DNAH5 | NM_001369.1 | 4887 | ttccaactcaacagacatcatcg | 79254 | 500056 | see also 930 line | | |
| 934 | DNAH5 | NM_001369.1 | 11541 | gcgcttggttaatgagatgtatc | 79449 | 500159 | see also 930 line | | |
| 935 | DNAH5 | NM_001369.1 | 11870 | ggctgaatttggtggaacttagc | 79460 | 500173 | see also 930 line | | |
| 936 | DNAH5 | NM_001369.1 | 13729 | ttgatgcctgtcataaggattta | 79502 | 500216 | see also 930 line | | |
| 937 | DNAH8 | NM_001371.1 | 1897 | aagctgtcatccgtcagtataac | 79944 | 500284 | - | - | - |
| 938 | DNAH8 | NM_001371.1 | 1898 | agctgtcatccgtcagtataaca | 79945 | 500285 | see also 937 line | | |
| 939 | DNAH8 | NM_001371.1 | 12430 | ctgtgcaggagcgacgaaaattt | 80270 | 500562 | see also 937 line | | |
| 940 | DNAH8 | NM_001371.1 | 13149 | aacatgtatgatgctcgtatacc | 80288 | 500593 | see also 937 line | | |
| 941 | DNAH8 | NM_001371.1 | 13152 | atgtatgatgctcgtatacctca | 80289 | 500594 | see also 937 line | | |
| 942 | DNASE2 | NM_001375.1 | 968 | cagaggaccactccaaatggtgc | 25323 | 591101 | apoptosis | deoxyribonuclease II | - |
| 943 | DNCH1 | NM_001376.2 | 5563 | agccagatgcgattttactttga | 80668 | 500704 | - | - | - |
| 944 | DNCH1 | NM_001376.2 | 7693 | gccaaggtgcctcagattgaagt | 80703 | 500735 | see also 943 line | | |
| 945 | DNCH1 | NM_001376.2 | 10962 | aagctacgatccagttttgaacc | 80763 | 500808 | see also 943 line | | |
| 946 | DNCH1 | NM_001376.2 | 12270 | aggctttaaccaagcagataagg | 80788 | 500825 | see also 943 line | | |
| 947 | DNCI2 | NM_001378.1 | 189 | ggctgagttggaacgtaagaagc | 299594 | 668038 | - | microtubule motor | - |
| 948 | DNCI2 | NM_001378.1 | 1410 | ttcccatccacaggatagcatgg | 299620 | 668071 | see also 947 line | | |
| 949 | DNMT1 | NM_001379.1 | 465 | gggctacctggctaaagtcaaat | 25332 | 458459 | transcription_regulator | zinc binding | schizophrenia |
| 950 | DPAGT1 | NM_001382.1 | 251 | ggcattcccccaccatgaatttg | 209558 | 598901 | - | transferase、transferring glycosyl groups | - |
| 951 | DPYSL2 | NM_001386.3 | 310 | cacgagcgatcgtcttctgatca | 203848 | 595572 | signal_transduction | dihydropyrimidinase | - |

Figure 46

| 952 | DPYSL2 | NM_001386.3 | 313 | gagcgatcgtcttctgatcaaag | 203849 | 595573 | see also 951 line | | |
|------|--------|-------------|------|-------------------------|--------|--------|-------------------|---|---|
| 953 | DPYSL2 | NM_001386.3 | 314 | agcgatcgtcttctgatcaaagg | 203850 | 595574 | see also 951 line | | |
| 954 | DPYSL3 | NM_001387.1 | 684 | ggggccattgctcaagttcatgc | 203885 | 595601 | signal_transduction | dihydropyrimidinase | - |
| 955 | DSCAM | NM_001389.2 | 504 | tccagagcttcgcgaatgtttc | 318849 | 742747 | - | - | - |
| 956 | DSCAM | NM_001389.2 | 509 | agcttcgcgaatgttttcagtga | 318850 | 742748 | see also 955 line | | |
| 957 | DSCAM | NM_001389.2 | 1955 | gtcctgtaccaggctcgaataaa | 318881 | 742763 | see also 955 line | | |
| 958 | DSCAM | NM_001389.2 | 1956 | tcctgtaccaggctcgaataaac | 318882 | 742764 | see also 955 line | | |
| 959 | DSCAM | NM_001389.2 | 5465 | cccagggctcagcttttgattga | 318964 | 742811 | see also 955 line | | |
| 960 | DYRK1A | NM_001396.2 | 740 | tgcatttgaaacgccactttatg | 80925 | 500911 | kinase_related | - | - |
| 961 | DYRK1A | NM_001396.2 | 749 | aacgccactttatgtttcgaaac | 80927 | 500912 | see also 960 line | | |
| 962 | DYRK1A | NM_001396.2 | 861 | acgaaagtttgcgcaacagatgt | 80929 | 500917 | see also 960 line | | |
| 963 | DYRK1A | NM_001396.2 | 899 | tccttgcgactccagaacttagt | 80930 | 500918 | see also 960 line | | |
| 964 | DYRK1A | NM_001396.2 | 906 | gactccagaacttagtatcattc | 80933 | 500919 | see also 960 line | | |
| 965 | DYRK1A | NM_001396.2 | 911 | cagaacttagtatcattcactgt | 80934 | 500920 | see also 960 line | | |
| 966 | DYRK1A | NM_001396.2 | 1326 | accaggaacccgtaaacttcata | 80946 | 500933 | see also 960 line | | |
| 967 | DYRK1A | NM_001396.2 | 1328 | caggaacccgtaaacttcataac | 80947 | 500934 | see also 960 line | | |
| 968 | DYRK1A | NM_001396.2 | 1329 | aggaacccgtaaacttcataaca | 80948 | 500935 | see also 960 line | | |
| 969 | DYRK1A | NM_001396.2 | 2159 | atggagctatggacgttaatttg | 80960 | 500953 | see also 960 line | | |
| 970 | DYRK1A | NM_001396.2 | 2160 | tggagctatggacgttaatttga | 80961 | 500954 | see also 960 line | | |
| 971 | DYRK1A | NM_001396.2 | 2252 | caggtcctgcacattacatgact | 80966 | 500956 | see also 960 line | | |
| 972 | ED1 | NM_001399.3 | 695 | taggcgtgttcgccgcaataaaa | 167628 | 564725 | signal_transduction、tr anscription_regulator | tumor necrosis factor receptor ligand | Christ-Siemens-Touraine syndrome、ectodermal dysplasia |
| 973 | ED1 | NM_001399.3 | 1002 | cagggccaagggtcagcaattca | 167634 | 564731 | see also 972 line | | |
| 974 | EDG2 | NM_001401.2 | 1040 | atgttcgccagaggactatgaga | 238570 | 622818 | receptor_acitivity、sig nal_transduction、gpcr 、kinase_related | - | - |
| 975 | CELSR3 | NM_001407.1 | 5084 | tacagtgcatctgagatactaca | 140657 | 546266 | gpcr、receptor_acitivit y、signal_transduction | structural molecule | - |
| 976 | CELSR3 | NM_001407.1 | 8700 | tacaacaacacggctctctttga | 140682 | 546310 | see also 975 line | | |
| 977 | EIF2B1 | NM_001414.1 | 741 | aagacgtcccagataagtttaag | 56318 | 476457 | - | translation initiation factor | - |
| 978 | EIF2B1 | NM_001414.1 | 744 | acgtcccagataagtttaagtat | 56319 | 476458 | see also 977 line | | |
| 979 | EIF4G2 | NM_001418.1 | 328 | gggggtgcttctcgtttcagtgc | 51321 | 704297 | cell_cycle | - | - |
| 980 | EIF4G2 | NM_001418.1 | 530 | atgcaatcttcaggaaagtaaga | 51326 | 704299 | see also 979 line | | |
| 981 | EIF4G2 | NM_001418.1 | 551 | gaggcatactaaataagcttact | 51330 | 704300 | see also 979 line | | |

Figure 46

| | | | | | | | | | |
|---|---|---|---|---|---|---|---|---|---|
| 982 | EIF4G2 | NM_001418.1 | 615 | gggtgtagagtctaaactcatcc | 51331 | 704302 | see also 979 line | | |
| 983 | EIF4G2 | NM_001418.1 | 642 | aggggtcatactgctgattgtgg | 51333 | 704303 | see also 979 line | | |
| 984 | EIF4G2 | NM_001418.1 | 723 | ggcagaagatgcaccaaactttg | 51337 | 704304 | see also 979 line | | |
| 985 | EIF4G2 | NM_001418.1 | 1236 | tcctcgcaaggcttttcttgaca | 51356 | 704315 | see also 979 line | | |
| 986 | EIF4G2 | NM_001418.1 | 1284 | tcgtcaagatgcagtaaaagatc | 51362 | 704319 | see also 979 line | | |
| 987 | EIF4G2 | NM_001418.1 | 1455 | tggtactggtccaggagttatcc | 51367 | 704324 | see also 979 line | | |
| 988 | EIF4G2 | NM_001418.1 | 1467 | aggagttatccaggatagatttt | 51368 | 704327 | see also 979 line | | |
| 989 | EIF4G2 | NM_001418.1 | 1498 | atgggacgtcatcgttcaaatca | 51369 | 704328 | see also 979 line | | |
| 990 | EIF4G2 | NM_001418.1 | 1655 | tgcaaggacagtcgaaggatatg | 51374 | 704329 | see also 979 line | | |
| 991 | EIF4G2 | NM_001418.1 | 2582 | ctcccaaacttcatgtagataaa | 51409 | 704361 | see also 979 line | | |
| 992 | EIF4G2 | NM_001418.1 | 2583 | tcccaaacttcatgtagataaag | 51410 | 704362 | see also 979 line | | |
| 993 | ELAVL1 | NM_001419.2 | 659 | taccagtttcaatggtcataaac | 47559 | 473690 | - | mRNA binding、3' UTR | - |
| 994 | ELF5 | NM_001422.2 | 773 | gagccctgagatactactataaa | 3132 | 443345 | transcription_regulator | - | - |
| 995 | EP300 | NM_001429.1 | 1234 | cagccaagcggcctaaactctca | 3235 | 526252 | transcription_regulator、signal_transduction、cell_cycle | transferase | - |
| 996 | EP300 | NM_001429.1 | 1277 | cgccagcgatggcacagattttg | 3236 | 526254 | see also 995 line | | |
| 997 | EP300 | NM_001429.1 | 1278 | gccagcgatggcacagattttgg | 3237 | 526255 | see also 995 line | | |
| 998 | EP300 | NM_001429.1 | 1739 | aggcaatggacaagggataatgc | 3251 | 526264 | see also 995 line | | |
| 999 | EP300 | NM_001429.1 | 2611 | ttggactaccctatcaagtaaat | 3268 | 526279 | see also 995 line | | |
| 1000 | EPB41L2 | NM_001431.1 | 1988 | tagtgaactcaagcgcaatttta | 117079 | 522634 | - | structural constituent of cytoskeleton | - |
| 1001 | EPB41L2 | NM_001431.1 | 2647 | cagagccaccagtggtaaaaaca | 117091 | 522643 | see also 1000 line | | |
| 1002 | ERN1 | NM_001433.1 | 415 | tccccatgccgaagttcagatgg | 26160 | 459255 | kinase_related | - | - |
| 1003 | ERN1 | NM_001433.1 | 1833 | tggagctgagggcacaattgtgt | 26182 | 459271 | see also 1002 line | | |
| 1004 | ERN1 | NM_001433.1 | 2711 | tggactggcgggagaacatcact | 26190 | 459296 | see also 1002 line | | |
| 1005 | ESR2 | NM_001437.1 | 919 | aggatgtaaggcctttttaaaa | 3446 | 443536 | receptor_acitivity、transcription_regulator、signal_transduction | transcription co-activator | breast cancer、Alzheimer disease |
| 1006 | ESRRG | NM_001438.1 | 835 | cagccaaaaagccatataacaag | 3487 | 443585 | - | translation regulator | - |
| 1007 | ESRRG | NM_001438.1 | 1120 | ttgtctatgcagacgattatata | 3491 | 443588 | see also 1006 line | | |
| 1008 | ESRRG | NM_001438.1 | 1122 | gtctatgcagacgattatataat | 3492 | 443589 | see also 1006 line | | |
| 1009 | EXTL3 | NM_001440.1 | 2141 | ggcggcaaggccgctttctctgg | 213486 | 601794 | - | transferase、transferring glycosyl groups | - |
| 1010 | GPC4 | NM_001448.2 | 322 | acgtgtccaaaggcttcaacaag | 246333 | 633648 | receptor_acitivity | - | - |

Figure 46

| 1011 | GPC4 | NM_001448.2 | 1150 | atcccatcgatgtgaagatttct | 246357 | 633674 | see also 1010 line | | |
|------|------|-------------|------|-------------------------|--------|--------|--------------------|---|---|
| 1012 | GPC4 | NM_001448.2 | 1251 | ctcccagctggacgaatttctcg | 246360 | 633680 | see also 1010 line | | |
| 1013 | GPC4 | NM_001448.2 | 1356 | ttggaccgactggttactgatgt | 246362 | 633683 | see also 1010 line | | |
| 1014 | FOXO3A | NM_001455.1 | 2384 | cacagtcggaccccttgatgtct | 4174 | 444142 | transcription_regulator、apoptosis | transcription factor | - |
| 1015 | FMO5 | NM_001461.1 | 672 | aggggatctggctgtagagatta | 221405 | 608316 | - | disulfide oxidoreductase | |
| 1016 | FMO5 | NM_001461.1 | 674 | gggatctggctgtagagattagc | 221406 | 608318 | see also 1015 line | | |
| 1017 | GABBR1 | NM_001470.1 | 1315 | atcatcgtgggactttctatga | 178893 | 629584 | receptor_acitivity、signal_transduction | - | alcoholism |
| 1018 | GABBR1 | NM_001470.1 | 1319 | tcgtgggacttctatgagact | 178894 | 629585 | see also 1017 line | | |
| 1019 | GABBR1 | NM_001470.1 | 1344 | agcccggaaagtttttgtgagg | 178897 | 629588 | see also 1017 line | | |
| 1020 | GAS8 | NM_001481.1 | 659 | gaggcaagttcgagaaattgagg | 280374 | 667234 | - | - | - |
| 1021 | GATM | NM_001482.1 | 108 | cgccgaggcggtgcactacatcg | 214929 | 604846 | - | glycine amidinotransferase | - |
| 1022 | GATM | NM_001482.1 | 657 | caggtcaattatcaaagactact | 214952 | 604863 | see also 1021 line | | |
| 1023 | GATM | NM_001482.1 | 873 | gagaagccaggttacaaactacc | 214954 | 604868 | see also 1021 line | | |
| 1024 | GATM | NM_001482.1 | 1188 | tgccaatgaagttccaattcaaa | 214962 | 604877 | see also 1021 line | | |
| 1025 | GATM | NM_001482.1 | 1196 | aagttccaattcaaaagatgttt | 214963 | 604879 | see also 1021 line | | |
| 1026 | NR6A1 | NM_001489.2 | 1100 | gcgagctctcaatcaaggattac | 8711 | 447418 | transcription_regulator、receptor_acitivity | - | - |
| 1027 | GCLC | NM_001498.1 | 1205 | tgctcatctctttattagagacc | 231947 | 616941 | - | Proto-oncogene C-JUN | - |
| 1028 | GCLC | NM_001498.1 | 1758 | ctgaactacctaaagctaattaa | 231955 | 616958 | see also 1027 line | | |
| 1029 | GCLC | NM_001498.1 | 1784 | gagagcatctggagaactaatga | 231956 | 616959 | see also 1027 line | | |
| 1030 | GMDS | NM_001500.1 | 745 | agccggtcagtagctaagattta | 233634 | 614190 | - | GDP-mannose 4、6-dehydratase | - |
| 1031 | GMDS | NM_001500.1 | 746 | gccggtcagtagctaagatttac | 233635 | 614191 | see also 1030 line | | |
| 1032 | GPLD1 | NM_001503.2 | 2300 | atggcttagatgaaatcatcatg | 197357 | 589683 | - | - | - |
| 1033 | GPLD1 | NM_001503.2 | 2301 | tggcttagatgaaatcatcatgg | 197358 | 589684 | see also 1032 line | | |
| 1034 | GRID2 | NM_001510.1 | 82 | cacatcggagcaattttgatga | 244680 | 696796 | receptor_acitivity、channel、signal_transduction | - | - |
| 1035 | GRID2 | NM_001510.1 | 662 | atcgctatcgagacactcttagg | 244692 | 696806 | see also 1034 line | | |
| 1036 | GRID2 | NM_001510.1 | 2128 | cggatgatcaaccgaagcaatgg | 244743 | 696845 | see also 1034 line | | |
| 1037 | GRID2 | NM_001510.1 | 2626 | cacctccatagacgtgtaaatag | 244758 | 696856 | see also 1034 line | | |
| 1038 | GRID2 | NM_001510.1 | 2627 | acctccatagacgtgtaaatagc | 244759 | 696857 | see also 1034 line | | |
| 1039 | GRID2 | NM_001510.1 | 2630 | tccatagacgtgtaaatagcttg | 244760 | 696858 | see also 1034 line | | |

Figure 46

| 1040 | GRID2 | NM_001510.1 | 2709 | ccctctggacattgacactttgc | 244764 | 696861 | see also 1034 line | | |
|------|-------|-------------|------|------------------------|--------|--------|--------------------|---|---|
| 1041 | GRID2 | NM_001510.1 | 2876 | agcaccgaactggcccttttagg | 244766 | 696863 | see also 1034 line | | |
| 1042 | GTF2B | NM_001514.2 | 439 | tagttgatcgaacaaataattta | 275434 | 657925 | transcription_regulator | general RNA polymerase II transcription factor | - |
| 1043 | GTF2I | NM_001518.2 | 555 | gacgtgcttttgtcaataccaga | 4582 | 459810 | - | - | - |
| 1044 | GTF2I | NM_001518.2 | 1009 | aagttgtggccccatcaaagtga | 4586 | 459820 | see also 1043 line | | |
| 1045 | GTF3C2 | NM_001521.1 | 46 | acctgcggggtcggctatgttgc | 131596 | 731311 | - | - | - |
| 1046 | GTF3C2 | NM_001521.1 | 1936 | gggagtgaccggaaaatcaaatt | 131619 | 731337 | see also 1045 line | | |
| 1047 | HDAC2 | NM_001527.1 | 1037 | cagtcaaaggtcatgctaaatgt | 4713 | 526353 | - | transcription factor | |
| 1048 | HDAC2 | NM_001527.1 | 1184 | atgagttgccatataatgattac | 4718 | 526362 | see also 1047 line | | |
| 1049 | HGFAC | NM_001528.2 | 1458 | caccctgtactcggtgttcaacc | 182160 | 575490 | - | trypsin | - |
| 1050 | HIF1A | NM_001530.2 | 323 | aagttctgaacgtcgaaaagaaa | 225 | 444481 | transcription_regulator, signal_transduction | - | - |
| 1051 | HIF1A | NM_001530.2 | 328 | ctgaacgtcgaaaagaaaagtct | 226 | 444482 | see also 1050 line | | |
| 1052 | HIF1A | NM_001530.2 | 1098 | ggccgctcaatttatgaatatta | 253 | 444511 | see also 1050 line | | |
| 1053 | HIF1A | NM_001530.2 | 1208 | tgccaaaagaggtggatatgtct | 256 | 444516 | see also 1050 line | | |
| 1054 | HIF1A | NM_001530.2 | 1240 | ctcaagcaactgtcatatataac | 257 | 444518 | see also 1050 line | | |
| 1055 | HIF1A | NM_001530.2 | 1789 | ccgatggaagcactagacaaagt | 279 | 444527 | see also 1050 line | | |
| 1056 | ID4 | NM_001546.2 | 555 | gtgcctgcagtgcgatatgaacg | 113122 | 690236 | transcription_regulator | transcription co-repressor | - |
| 1057 | IFRD1 | NM_001550.1 | 589 | atgactttaactgatagcattga | 319610 | 704459 | - | - | - |
| 1058 | IFRD1 | NM_001550.1 | 720 | aactcttggaccaatcctaaaga | 319613 | 704463 | see also 1057 line | | |
| 1059 | IGFBP7 | NM_001553.1 | 488 | tgcgagcaaggtccttccatagt | 124925 | 528755 | - | antigen binding | - |
| 1060 | IGFBP7 | NM_001553.1 | 719 | gtgctggtatctcctctaagtaa | 124927 | 528761 | see also 1059 line | | |
| 1061 | IGFBP7 | NM_001553.1 | 720 | tgctggtatctcctctaagtaag | 124928 | 528762 | see also 1059 line | | |
| 1062 | IGSF1 | NM_001555.1 | 1928 | tggctcaactaaggagtttgtgt | 319101 | 796876 | - | - | - |
| 1063 | IGSF1 | NM_001555.1 | 3024 | cccatggggcagaatgttactct | 319122 | 796897 | see also 1062 line | | |
| 1064 | IGSF1 | NM_001555.1 | 3769 | tccaggcctaccctgatatctgg | 319140 | 796923 | see also 1062 line | | |
| 1065 | INPP4A | NM_001566.1 | 462 | tactccatcgctagatcgaaagc | 317750 | 742140 | signal_transduction | - | - |
| 1066 | INPP4A | NM_001566.1 | 1271 | aagcagataaaaagttagaattt | 317772 | 742152 | see also 1065 line | | |
| 1067 | ABCA2 | NM_001606.2 | 1845 | gcggctggatccagttcatgtcc | 68768 | 490694 | - | ATP-binding cassette (ABC) transporter | - |
| 1068 | ABCA2 | NM_001606.2 | 6094 | cccgttcgagtgggacattgtca | 68792 | 490732 | see also 1067 line | | |
| 1069 | ACRV1 | NM_001612.4 | 906 | ttcccagcagtgcatgttaaaga | 320627 | 688456 | - | - | - |

Figure 46

| | | | | | | | | | |
|---|---|---|---|---|---|---|---|---|---|
| 1070 | ACVR2 | NM_001616.2 | 475 | ctgtgagggcaatatgtgtaatg | 71703 | 493299 | receptor_acitivity、 signal_transduction、 kinase_related | transforming growth factor-beta receptor | - |
| 1071 | ACVR2 | NM_001616.2 | 659 | atggcctaccctcctgtacttgt | 71714 | 493307 | see also 1070 line | | |
| 1072 | ACVR2 | NM_001616.2 | 1030 | gtgtcatattgcagaaaccatgg | 71726 | 493314 | see also 1070 line | | |
| 1073 | ACVR2 | NM_001616.2 | 1051 | ggctagaggattggcatatttac | 71727 | 493316 | see also 1070 line | | |
| 1074 | ACVR2 | NM_001616.2 | 1448 | caggaagttgttgtgcataaaaa | 71735 | 493324 | see also 1070 line | | |
| 1075 | ACVR2 | NM_001616.2 | 1683 | ctcccaaagaatctagtctatga | 71738 | 493331 | see also 1070 line | | |
| 1076 | AHR | NM_001621.2 | 693 | agccggtgcagaaaacagtaaag | 772 | 454610 | transcription_regulator、 receptor_acitivity、 apoptosis、 cell_cycle、 signal_transduction | translation regulator | - |
| 1077 | AHR | NM_001621.2 | 884 | agcttctttgatgttgcattaaa | 786 | 454620 | see also 1076 line | | |
| 1078 | AKT2 | NM_001626.2 | 244 | agcgtggtgaatacatcaagacc | 71965 | 493659 | kinase_related | protein serine/threonine kinase | breast cancer、 ovarian cancer、 pancreatic cancer、 malignant thymoma |
| 1079 | AKT2 | NM_001626.2 | 406 | cgcgacccaacacctttgtcata | 71969 | 493661 | see also 1078 line | | |
| 1080 | ALCAM | NM_001627.1 | 341 | cagaaaactacactttgtctatc | 170930 | 682253 | signal_transduction | receptor binding | - |
| 1081 | ALCAM | NM_001627.1 | 451 | gtgttcaagcaaccatctaaacc | 170934 | 682257 | see also 1080 line | | |
| 1082 | AMBP | NM_001633.2 | 554 | cacaaatccaaatggaacataac | 180342 | 655700 | - | transporter | Mediterranean fever、 periodic disease |
| 1083 | AOAH | NM_001637.1 | 1058 | gactcagctggggctcattttca | 199816 | 595112 | - | lipoprotein lipase | - |
| 1084 | APEX1 | NM_001641.2 | 1162 | atgttggttggcgccttgattac | 21395 | 591077 | transcription_regulator | - | - |
| 1085 | APEX1 | NM_001641.2 | 1165 | ttggttggcgccttgattacttt | 21396 | 591078 | see also 1084 line | | |
| 1086 | APEX1 | NM_001641.2 | 1169 | ttggcgccttgattacttttttgt | 21398 | 591079 | see also 1084 line | | |
| 1087 | APLP2 | NM_001642.1 | 1177 | ctgccaaccaatgatgttgatgt | 21427 | 454819 | - | serine protease inhibitor | - |
| 1088 | APLP2 | NM_001642.1 | 1179 | gccaaccaatgatgttgatgtgt | 21428 | 454820 | see also 1087 line | | |
| 1089 | APLP2 | NM_001642.1 | 1280 | accgaatggacagggtaaagaag | 21430 | 454822 | see also 1087 line | | |
| 1090 | APLP2 | NM_001642.1 | 2048 | tggatgttaaggaaatgattttc | 21444 | 454837 | see also 1087 line | | |
| 1091 | APOD | NM_001647.1 | 193 | aagatggtacgaaattgagaaga | 173246 | 570047 | - | transporter | - |
| 1092 | APXL | NM_001649.2 | 3683 | agcgggtgatggacaacaacacc | 262481 | 773670 | channel | amiloride-sensitive sodium channel | melanoma |
| 1093 | AQP4 | NM_001650.3 | 936 | tggagtggtgcatgtgattgacg | 263840 | 647510 | - | - | - |
| 1094 | ARAF1 | NM_001654.1 | 488 | ctgaccatgcacaattttgtacg | 72297 | 494006 | kinase_related | receptor signaling protein | |
| 1095 | ARFD1 | NM_001656.2 | 695 | cagatccgagcatcaattttaga | 108259 | 520579 | signal_transduction | - | - |

Figure 46

| 1096 | ARF6 | NM_001663.2 | 730 | gccgctctggcggcattactaca | 108231 | 518624 | signal_transduction | protein transporter | Alzheimer disease |
|---|---|---|---|---|---|---|---|---|---|
| 1097 | ARF6 | NM_001663.2 | 732 | cgctctggcggcattactacact | 108232 | 518625 | see also 1096 line | | |
| 1098 | ARF6 | NM_001663.2 | 819 | aggagctgcaccgcattatcaat | 108234 | 518626 | see also 1096 line | | |
| 1099 | ARF6 | NM_001663.2 | 1005 | atgaggggctcacatggttaacc | 108237 | 518630 | see also 1096 line | | |
| 1100 | ARF6 | NM_001663.2 | 1008 | aggggctcacatggttaacctct | 108238 | 518631 | see also 1096 line | | |
| 1101 | ARF6 | NM_001663.2 | 1015 | cacatggttaacctctaactaca | 108239 | 518632 | see also 1096 line | | |
| 1102 | ATP2A2 | NM_001681.2 | 360 | tactggcagcatgtatatctttt | 72712 | 494427 | - | - | Darier-White disease |
| 1103 | ATP2A2 | NM_001681.2 | 859 | tggcaagatccgggatgaaatgg | 72726 | 494446 | see also 1102 line | | |
| 1104 | ATP2B2 | NM_001683.1 | 3920 | ctcgaacctccatccataacttc | 72934 | 494528 | - | plasma membrane cation-transporting ATPase | - |
| 1105 | ATP2B2 | NM_001683.1 | 3953 | ctgaattccggatcgaagattcc | 72936 | 494529 | see also 1104 line | | |
| 1106 | ATP5J | NM_001685.3 | 756 | cggaggaacattggtgttacagc | 266178 | 653517 | - | hydrogen-transporting two-sector ATPase | - |
| 1107 | ATP6V1A | NM_001690.2 | 351 | gggagccatttttgatggtattc | 73168 | 494798 | - | hydrogen-translocating V-type ATPase | - |
| 1108 | ATP6V1A | NM_001690.2 | 484 | aacctacgggttggtagtcatat | 73173 | 494805 | see also 1107 line | | |
| 1109 | ATP6V1A | NM_001690.2 | 485 | acctacgggttggtagtcatatc | 73174 | 494806 | see also 1107 line | | |
| 1110 | ATP6V1B2 | NM_001693.2 | 994 | ttggctaatgacccaaccattga | 73261 | 494859 | - | hydrogen-translocating V-type ATPase | - |
| 1111 | ATP6V1C1 | NM_001695.2 | 1273 | aagaatactacccctatgtgtac | 73324 | 494909 | gpcr、 receptor_acitivity、 signal_transduction | hydrogen-translocating V-type ATPase | - |
| 1112 | AXL | NM_001699.3 | 2418 | ctgagtaccaagagattcataca | 73964 | 495684 | receptor_acitivity、 kinase_related | - | thyroid cancer、 chronic myeloid leukemia、 rheumatoid arthritis、 colorectal cancer、 leukemia、 colorectal cancers |
| 1113 | AXL | NM_001699.3 | 2420 | gagtaccaagagattcatacacc | 73965 | 495685 | see also 1112 line | | |
| 1114 | BAI1 | NM_001702.1 | 2959 | ctctccaccttcgccatcttagc | 129221 | 532473 | gpcr、 receptor_acitivity、 signal_transduction | protein binding | colorectal cancer、 colorectal cancers |
| 1115 | BAI2 | NM_001703.1 | 1920 | gtgtccagccttccatgagatgt | 242213 | 629997 | - | G-protein coupled receptor | - |
| 1116 | BAI3 | NM_001704.1 | 41 | ctgttcgtaacctgctgatttat | 242235 | 630026 | gpcr、 receptor_acitivity、 signal_transduction | G-protein coupled receptor | - |

Figure 46

| | | | | | | | | | | |
|---|---|---|---|---|---|---|---|---|---|---|
| 1117 | BAI3 | NM_001704.1 | 44 | ttcgtaacctgctgatttatata | 242236 | 630027 | see also 1116 line | | | |
| 1118 | BAI3 | NM_001704.1 | 218 | cagatccaaccaaatatagcatt | 242247 | 630033 | see also 1116 line | | | |
| 1119 | BAI3 | NM_001704.1 | 289 | ctggcttatcagtttgatcattt | 242250 | 630037 | see also 1116 line | | | |
| 1120 | BAI3 | NM_001704.1 | 290 | tggcttatcagtttgatcatttt | 242251 | 630038 | see also 1116 line | | | |
| 1121 | BAI3 | NM_001704.1 | 967 | caggtgcgaaccagaacttgtgt | 242268 | 630058 | see also 1116 line | | | |
| 1122 | BAI3 | NM_001704.1 | 970 | gtgcgaaccagaacttgtgtatc | 242269 | 630059 | see also 1116 line | | | |
| 1123 | BAI3 | NM_001704.1 | 971 | tgcgaaccagaacttgtgtatca | 242270 | 630060 | see also 1116 line | | | |
| 1124 | BAI3 | NM_001704.1 | 1749 | cagacacttgcagcattcaatta | 242284 | 630071 | see also 1116 line | | | |
| 1125 | BAI3 | NM_001704.1 | 1751 | gacacttgcagcattcaattaaa | 242285 | 630072 | see also 1116 line | | | |
| 1126 | BAI3 | NM_001704.1 | 2799 | ctcatccaatatcctcatactgg | 242319 | 630111 | see also 1116 line | | | |
| 1127 | BAI3 | NM_001704.1 | 3393 | gacagataaacgctccatattgt | 242330 | 630123 | see also 1116 line | | | |
| 1128 | BAI3 | NM_001704.1 | 3862 | ttgcggagaactgtgtacttatg | 242352 | 630145 | see also 1116 line | | | |
| 1129 | BAI3 | NM_001704.1 | 4069 | aaccctgaattcaatatgaatcc | 242358 | 630155 | see also 1116 line | | | |
| 1130 | BAI3 | NM_001704.1 | 4172 | ctcgcacagctgtgaagaatttc | 242360 | 630164 | see also 1116 line | | | |
| 1131 | BAI3 | NM_001704.1 | 4241 | ctggatcaacgatatcaatgagt | 242361 | 630168 | see also 1116 line | | | |
| 1132 | BCL6 | NM_001706.2 | 437 | ttgtgagccgtgagcagtttaga | 1478 | 456293 | transcription_regulator | - | | non-hodgkin lymphoma、leukemia |
| 1133 | BCL6 | NM_001706.2 | 439 | gtgagccgtgagcagtttagagc | 1479 | 456294 | see also 1132 line | | | |
| 1134 | BDNF | NM_001709.3 | 295 | tccttttccttactatggttatt | 168949 | 532682 | - | - | | Parkinson disease、schizophrenia |
| 1135 | BDNF | NM_001709.3 | 301 | tccttactatggttatttcatac | 168951 | 532683 | see also 1134 line | | | |
| 1136 | BDNF | NM_001709.3 | 965 | tggctggcgattcataaggatag | 168970 | 532696 | see also 1134 line | | | |
| 1137 | BDNF | NM_001709.3 | 966 | ggctggcgattcataaggataga | 168971 | 532697 | see also 1134 line | | | |
| 1138 | BDNF | NM_001709.3 | 970 | ggcgattcataaggatagacact | 168972 | 532698 | see also 1134 line | | | |
| 1139 | BGN | NM_001711.3 | 709 | cagcgggctccggaacatgaact | 287619 | 694898 | - | | extracellular matrix structural constituent | Alzheimer disease |
| 1140 | BGN | NM_001711.3 | 711 | gcgggctccggaacatgaactgc | 287620 | 694899 | see also 1139 line | | | |
| 1141 | BICD1 | NM_001714.1 | 545 | aggatgaaatccgagaatataag | 277310 | 685875 | - | - | | - |
| 1142 | BICD1 | NM_001714.1 | 652 | cagaaccaggttgaatacgaagg | 277314 | 685877 | see also 1141 line | | | |
| 1143 | BICD1 | NM_001714.1 | 1554 | cagcatagccaacgaaaatcaca | 277326 | 685899 | see also 1141 line | | | |
| 1144 | BNC | NM_001717.2 | 1708 | cagtatgcctatcaaaatagaga | 1561 | 456541 | transcription_regulator | transcription factor | | - |
| 1145 | BPAG1 | NM_001723.3 | 545 | tggccttaaggaacgaatgttct | 115349 | 522000 | cell_cycle、signal_transduction | - | | epidermolysis bullosa |
| 1146 | BPAG1 | NM_001723.3 | 547 | gccttaaggaacgaatgttcttc | 115350 | 522001 | see also 1145 line | | | |
| 1147 | BPAG1 | NM_001723.3 | 1983 | cagaattgagcaacagtatcaga | 115384 | 522042 | see also 1145 line | | | |

Figure 46

| 1148 | BRS3 | NM_001727.1 | 170 | ctcacctaatcagactttaattt | 176825 | 623729 | receptor_acitivity、gpcr、signal_transduction | bombesin receptor | Alzheimer disease |
|---|---|---|---|---|---|---|---|---|---|
| 1149 | C5 | NM_001735.2 | 2531 | tacgaggagaacagatccaattg | 167165 | 664212 | kinase_related、signal_transduction、gpcr | endopeptidase inhibitor | lupus erythematosus、Alzheimer disease |
| 1150 | C5 | NM_001735.2 | 2532 | acgaggagaacagatccaattga | 167166 | 664213 | see also 1149 line | | |
| 1151 | C5 | NM_001735.2 | 2534 | gaggagaacagatccaattgaaa | 167167 | 664214 | see also 1149 line | | |
| 1152 | CALB2 | NM_001740.2 | 541 | aaccatactacggatgtttgact | 138941 | 545153 | - | - | - |
| 1153 | CALCR | NM_001742.1 | 411 | atcgaacctggtccaactatact | 242001 | 630375 | receptor_acitivity、gpcr、signal_transduction | calcitonin receptor | lower lumbar bone mineral density |
| 1154 | CAMK4 | NM_001744.3 | 808 | tggatttgaaccattctatgatg | 75062 | 497054 | kinase_related、signal_transduction | calmodulin-dependent protein kinase I | - |
| 1155 | CAMK4 | NM_001744.3 | 886 | ctccccctggtgggatgaagtat | 75066 | 497058 | see also 1154 line | | |
| 1156 | CANX | NM_001746.2 | 551 | ttgttcagtatgaggttaatttc | 137406 | 542549 | - | chaperone | - |
| 1157 | CAPN2 | NM_001748.3 | 2145 | ggctggaaacgctattcaagata | 139059 | 545285 | - | calpain | - |
| 1158 | CAV1 | NM_001753.3 | 460 | atgacgtggtcaagattgacttt | 289813 | 533197 | - | Sterol regulatory element binding protein-1a | prostate cancer |
| 1159 | ENTPD1 | NM_001776.2 | 464 | aaggtcctggaatctcaaaattt | 165384 | 563044 | - | magnesium binding | - |
| 1160 | CDH2 | NM_001792.2 | 968 | atcaatgttattgacatgaatga | 139830 | 545603 | - | calcium ion binding | - |
| 1161 | CDH2 | NM_001792.2 | 1227 | aagtgcaacagtatacgttaata | 139833 | 545608 | see also 1160 line | | |
| 1162 | CDH2 | NM_001792.2 | 1229 | gtgcaacagtatacgttaataat | 139834 | 545609 | see also 1160 line | | |
| 1163 | CDH2 | NM_001792.2 | 1571 | aggatgtttgtccttactgttgc | 139847 | 545623 | see also 1160 line | | |
| 1164 | CDH2 | NM_001792.2 | 2171 | cggcttaatggtgattttgctca | 139874 | 545644 | see also 1160 line | | |
| 1165 | CDH4 | NM_001794.2 | 2136 | gtcaaggtgtgcccatgtgatga | 140173 | 545755 | - | calcium ion binding | - |
| 1166 | CDH8 | NM_001796.2 | 692 | tggagcctccttctgaatttatt | 140334 | 545946 | - | calcium ion binding | - |
| 1167 | CDH8 | NM_001796.2 | 1496 | ctcgtgaccctgatatcacttcc | 140360 | 545975 | see also 1166 line | | |
| 1168 | CDH8 | NM_001796.2 | 2338 | ccccgtaaggatattaaaccaga | 140383 | 545996 | see also 1166 line | | |
| 1169 | CDH8 | NM_001796.2 | 2403 | tggtgttgatgtcgatgaattta | 140385 | 545999 | see also 1166 line | | |
| 1170 | CDH8 | NM_001796.2 | 2405 | gtgttgatgtcgatgaatttata | 140386 | 546000 | see also 1166 line | | |
| 1171 | CDH8 | NM_001796.2 | 2550 | atcagactcagaccagaattttg | 140387 | 546006 | see also 1166 line | | |
| 1172 | CDH8 | NM_001796.2 | 2587 | tggggtccccgctttaagagact | 140388 | 546007 | see also 1166 line | | |
| 1173 | CDH11 | NM_001797.2 | 2021 | cagcctacccctgaaatcattc | 139418 | 545524 | - | - | - |
| 1174 | CDH11 | NM_001797.2 | 2751 | cagaactggggacctcgttttaa | 139435 | 545536 | see also 1173 line | | |
| 1175 | CDK2 | NM_001798.2 | 1003 | aagatggacggagcttgttatcg | 76141 | 497761 | kinase_related、cell_cycle | - | - |

Figure 46

| | | | | | | | | | |
|---|---|---|---|---|---|---|---|---|---|
| 1176 | CDK2 | NM_001798.2 | 1013 | gagcttgttatcgcaaatgctgc | 76142 | 497762 | see also 1175 line | | |
| 1177 | CHN1 | NM_001822.2 | 1658 | gagctgcttatcaaaaacgaaga | 172197 | 569581 | - | diacylglycerol binding | - |
| 1178 | CKB | NM_001823.3 | 271 | cggggccacccgtacatcatgacc | 209433 | 598786 | kinase_related | creatine kinase | - |
| 1179 | CKM | NM_001824.2 | 563 | cagcctgacgggcgagttcaaag | 209436 | 598793 | kinase_related | creatine kinase | - |
| 1180 | CKMT2 | NM_001825.1 | 803 | acctggctggccgctactacaag | 209455 | 813033 | kinase_related | creatine kinase | - |
| 1181 | CKMT2 | NM_001825.1 | 1176 | agctggtgtccacgttaggatcc | 209467 | 813044 | see also 1180 line | | |
| 1182 | CLCN3 | NM_001829.1 | 1305 | gtggcatcaggtttgagtttagg | 263284 | 646931 | channel | voltage-gated chloride channel | - |
| 1183 | CLCN3 | NM_001829.1 | 2140 | atcaccacgactggtttatcttt | 263302 | 646952 | see also 1182 line | | |
| 1184 | CLCN3 | NM_001829.1 | 2860 | ggcgcctccttggcattataaca | 263319 | 646963 | see also 1182 line | | |
| 1185 | CLCN3 | NM_001829.1 | 2919 | aaccaagaccccgcttcaataat | 263322 | 646964 | see also 1182 line | | |
| 1186 | CLCN3 | NM_001829.1 | 2920 | accaagaccccgcttcaataatg | 263323 | 646965 | see also 1182 line | | |
| 1187 | CLCN3 | NM_001829.1 | 2923 | aagacccgcttcaataatgttc | 263324 | 646966 | see also 1182 line | | |
| 1188 | CLCN4 | NM_001830.2 | 2380 | agggcattgtgagcaattccatc | 263351 | 646986 | channel | voltage-gated chloride channel | - |
| 1189 | CNTFR | NM_001842.3 | 603 | gtgctgcatggctccaaaattat | 243898 | 631226 | receptor_acitivity、signal_transduction | - | - |
| 1190 | CNTFR | NM_001842.3 | 696 | aagtacaaggtctccataagtgt | 243901 | 631227 | see also 1189 line | | |
| 1191 | CNTN1 | NM_001843.2 | 241 | ttgaagagcagccaatcaatacc | 24433 | 683291 | - | - | - |
| 1192 | CNTN1 | NM_001843.2 | 394 | taggaggaaaccttgttatcaac | 24437 | 683296 | see also 1191 line | | |
| 1193 | CNTN1 | NM_001843.2 | 827 | accatatcctgctgatattgtag | 24456 | 683307 | see also 1191 line | | |
| 1194 | CNTN1 | NM_001843.2 | 1345 | tggctccaactttttgaaatgaat | 24470 | 683321 | see also 1191 line | | |
| 1195 | COL4A6 | NM_001847.1 | 1014 | aggggtcccatgggttcagaagg | 288281 | 659631 | - | - | diffuse esophageal leiomyomatosis |
| 1196 | COL4A6 | NM_001847.1 | 4720 | ggcgcaatgataaatcttactgg | 288316 | 659669 | see also 1195 line | | |
| 1197 | COL9A1 | NM_001851.2 | 421 | atgtagacttcaggattccaact | 288594 | 659842 | - | - | - |
| 1198 | COL11A1 | NM_001854.2 | 844 | gtgacaatgattgttgattgtaa | 287652 | 659285 | - | - | Stickler syndrome、Marshall syndrome |
| 1199 | COL16A1 | NM_001856.2 | 1307 | agggagcacggggcaatgactgt | 287974 | 660874 | - | extracellular matrix structural constituent | - |
| 1200 | COL16A1 | NM_001856.2 | 4533 | gggagacatggtgaattatgatg | 287985 | 660883 | see also 1199 line | | |
| 1201 | COL19A1 | NM_001858.2 | 2587 | aacggcatgagcagtttatataa | 288040 | 660921 | - | extracellular matrix structural constituent | - |
| 1202 | CPA3 | NM_001870.1 | 214 | tccgagttagtgagaaggaatcc | 185566 | 579810 | - | carboxypeptidase A | - |
| 1203 | CPE | NM_001873.1 | 833 | tgcccagggaatagatctgaacc | 185934 | 580058 | signal_transduction | carboxypeptidase H | - |
| 1204 | ATF2 | NM_001880.2 | 1543 | tggctatcatactgctgataaag | 21850 | 455317 | transcription_regulator | Smad3/Sp-1 heterodimer | - |

Figure 46

| | | | | | | | | | |
|------|---------|-------------|------|------------------------|--------|--------|---------------------------------------------|-------------------------------------------|-------------------------------------------------------------|
| 1205 | CREM | NM_001881.2 | 642 | tggcagtaccaactagcatatat | 2540 | 442825 | transcription_regulator、 signal_transduction | cAMP-responsive element modulator、 alpha isoform | - |
| 1206 | CREM | NM_001881.2 | 643 | ggcagtaccaactagcatatatc | 2541 | 442826 | see also 1205 line | | |
| 1207 | CRHR2 | NM_001883.2 | 186 | gccgtgccccgagtacttcaacg | 242057 | 630174 | gpcr、 receptor_acitivity、 signal_transduction | corticotrophin-releasing factor receptor | - |
| 1208 | CRYAB | NM_001885.1 | 502 | tcccatcacccgtgaagagaagc | 289518 | 659909 | - | structural constituent of eye lens | myopathy |
| 1209 | CSNK1E | NM_001894.4 | 299 | tggggatcccgtccatcaagtgg | 77666 | 498932 | kinase_related、 signal_transduction | - | - |
| 1210 | CSNK2A2 | NM_001896.2 | 260 | taccaactggttcgaaaacttgg | 77741 | 499011 | kinase_related、 signal_transduction | protein kinase CK2 | Alzheimer disease |
| 1211 | CSNK2A2 | NM_001896.2 | 415 | tggaacaaatatcattaagctga | 77744 | 499022 | see also 1210 line | | |
| 1212 | CSNK2A2 | NM_001896.2 | 533 | gactttgatatccggtttatat | 77746 | 499028 | see also 1210 line | | |
| 1213 | CSNK2A2 | NM_001896.2 | 537 | ttgatatccggtttatatgtat | 77747 | 499029 | see also 1210 line | | |
| 1214 | CSNK2A2 | NM_001896.2 | 564 | tacttaaagctctggattactgc | 77749 | 499030 | see also 1210 line | | |
| 1215 | CSNK2A2 | NM_001896.2 | 619 | acctcacaatgtcatgatagatc | 77753 | 499032 | see also 1210 line | | |
| 1216 | CTNNA1 | NM_001903.1 | 436 | ggcagatgtctacaaattacttg | 290172 | 660400 | - | structural molecule | - |
| 1217 | CTNNA1 | NM_001903.1 | 1065 | atgctggacgtaaagaaagaagt | 290183 | 660423 | see also 1216 line | | |
| 1218 | CTPS | NM_001905.1 | 488 | tggaacctcaagtgtgtgttatt | 232084 | 616998 | - | carbon-nitrogen ligase、 with glutamine as amido-N-donor | - |
| 1219 | CTPS | NM_001905.1 | 492 | acctcaagtgtgtgttattgagc | 232085 | 616999 | see also 1218 line | | |
| 1220 | CTPS | NM_001905.1 | 1174 | gtgctggttccaggaggatttgg | 232090 | 617012 | see also 1218 line | | |
| 1221 | DAO | NM_001917.3 | 1049 | cacagaggtcatccacaactatg | 225944 | 499332 | - | electron transporter | Alzheimer disease、 schizophrenia |
| 1222 | DCT | NM_001922.2 | 973 | tggctccattattattctgttag | 162530 | 613861 | - | oxidoreductase | melanoma |
| 1223 | DDB1 | NM_001923.2 | 2764 | tagcacggtgcggctctatgagt | 16115 | 482784 | - | damaged DNA binding | xeroderma pigmentosum |
| 1224 | DDB1 | NM_001923.2 | 3102 | tccacctgggcgagtttgtcaat | 16121 | 482794 | see also 1223 line | | |
| 1225 | DDB1 | NM_001923.2 | 3104 | cacctgggcgagtttgtcaatgt | 16122 | 482795 | see also 1223 line | | |
| 1226 | DDB1 | NM_001923.2 | 3510 | aggagctaactcggatccattag | 16132 | 482805 | see also 1223 line | | |
| 1227 | GADD45A | NM_001924.2 | 705 | atcctgccttaagtcaacttatt | 285463 | 658316 | apoptosis、 cell_cycle、 kinase_related | structural constituent of ribosome | - |
| 1228 | GADD45A | NM_001924.2 | 706 | tcctgccttaagtcaacttattt | 285464 | 658317 | see also 1227 line | | |
| 1229 | DES | NM_001927.2 | 778 | aggagatcgcgttccttaagaaa | 287095 | 684762 | - | structural constituent of cytoskeleton | distal myopathy with cardiomyopathy、 myopathy、 cardiomyopathy |

Figure 46

| | | | | | | | | | |
|---|---|---|---|---|---|---|---|---|---|
| 1230 | DES | NM_001927.2 | 780 | gagatcgcgttccttaagaaagt | 287096 | 684763 | see also 1229 line | | |
| 1231 | DGUOK | NM_001929.2 | 280 | cagcacgatggtcctacacattc | 78242 | 499567 | kinase_related | - | mitochondrial DNA depletion syndrome |
| 1232 | DGUOK | NM_001929.2 | 281 | agcacgatggtcctacacattcc | 78243 | 499568 | see also 1231 line | | |
| 1233 | DLAT | NM_001931.2 | 539 | gaggatattgaggcctttaaaaa | 78425 | 499579 | - | protein binding | primary biliary cirrhosis |
| 1234 | DPP4 | NM_001935.2 | 892 | ctgtgaatccaactgtaaagttc | 183391 | 577202 | - | prolyl oligopeptidase | - |
| 1235 | DPP4 | NM_001935.2 | 2080 | cccggtgggagtactatgactca | 183435 | 577241 | see also 1234 line | | |
| 1236 | DR1 | NM_001938.1 | 855 | aggccagttctcgtttggaaaac | 25595 | 458688 | transcription_regulator | transcription co-repressor | |
| 1237 | DR1 | NM_001938.1 | 856 | ggccagttctcgtttggaaaacc | 25596 | 458689 | see also 1236 line | | |
| 1238 | DRPLA | NM_001940.2 | 3429 | gcctcggtgcaccctctcattga | 130909 | 672769 | - | protein binding | dentatorubral-pallidoluysian atrophy、 myoclonic epilepsy |
| 1239 | DSG3 | NM_001944.1 | 1434 | aaccgagattctactttcatagt | 142479 | 547646 | - | calcium ion binding | - |
| 1240 | E2F5 | NM_001951.2 | 1004 | gactacaactttaatttagatga | 2944 | 443165 | transcription_regulator、 cell_cycle | translation regulator | - |
| 1241 | EFNA5 | NM_001962.1 | 723 | aagaaggtcctgtctaaagctca | 319566 | 684374 | - | GPI-linked ephrin | - |
| 1242 | EFNA5 | NM_001962.1 | 916 | agccgccttttggcaatcctact | 319574 | 684376 | see also 1241 line | | |
| 1243 | EFNA5 | NM_001962.1 | 941 | tcctcctggcgatgcttttgaca | 319575 | 684377 | see also 1241 line | | |
| 1244 | EFNA5 | NM_001962.1 | 947 | tggcgatgcttttgacattatag | 319578 | 684379 | see also 1241 line | | |
| 1245 | EIF4A2 | NM_001967.2 | 1129 | gtggctataaactttgttactga | 25802 | 459072 | - | translation initiation factor | - |
| 1246 | EIF4A2 | NM_001967.2 | 1180 | gagactttctacaatactacagt | 25803 | 459073 | see also 1245 line | | |
| 1247 | EIF5 | NM_001969.3 | 543 | cagcgtgtcagaccagttctatc | 56794 | 476754 | - | - | - |
| 1248 | EIF5 | NM_001969.3 | 544 | agcgtgtcagaccagttctatcg | 56795 | 476755 | see also 1247 line | | |
| 1249 | EIF5 | NM_001969.3 | 612 | tggaatcaagacagttatagtca | 56798 | 476756 | see also 1247 line | | |
| 1250 | EIF5 | NM_001969.3 | 643 | gacgttgcaaaggcgcttaatcg | 56799 | 476757 | see also 1247 line | | |
| 1251 | EIF5 | NM_001969.3 | 668 | ctccaacgtatcccaccaaatat | 56800 | 476758 | see also 1247 line | | |
| 1252 | EIF5 | NM_001969.3 | 669 | tccaacgtatcccaccaaatatt | 56801 | 476759 | see also 1247 line | | |
| 1253 | EIF5 | NM_001969.3 | 710 | cacagacccagtttgatgttaag | 56802 | 476762 | see also 1247 line | | |
| 1254 | EIF5 | NM_001969.3 | 753 | tggatctcatgaggcgaataagc | 56803 | 476763 | see also 1247 line | | |
| 1255 | EIF5 | NM_001969.3 | 756 | atctcatgaggcgaataagctgc | 56804 | 476764 | see also 1247 line | | |
| 1256 | EIF5 | NM_001969.3 | 765 | ggcgaataagctgcaagacatgt | 56805 | 476765 | see also 1247 line | | |
| 1257 | EMR1 | NM_001974.3 | 2147 | cagctctcgcaacatcaagatgc | 143129 | 548064 | gpcr、 receptor_acitivity、 signal_transduction | calcium ion binding | - |
| 1258 | ENO2 | NM_001975.2 | 1014 | cactgatccttcccgatacatca | 165351 | 563022 | - | phosphopyruvate hydratase | - |
| 1259 | ENPEP | NM_001977.2 | 2117 | ttgggttttatcgtgtaaattat | 156684 | 557583 | - | zinc binding | - |
| 1260 | ENPEP | NM_001977.2 | 2118 | tgggttttatcgtgtaaattatg | 156685 | 557584 | see also 1259 line | | |

Figure 46

| # | Gene | Accession | Pos | Sequence | ID1 | ID2 | Function | Binding | Notes |
|---|---|---|---|---|---|---|---|---|---|
| 1261 | ENPEP | NM_001977.2 | 2344 | acctacatcattagcatgtttga | 156693 | 557596 | | | see also 1259 line |
| 1262 | ENPEP | NM_001977.2 | 2350 | atcattagcatgtttgaagatga | 156694 | 557597 | | | see also 1259 line |
| 1263 | EPS15 | NM_001981.1 | 548 | accagtgttgctcaactctaagt | 143200 | 548139 | signal_transduction | calcium ion binding | leukemia |
| 1264 | EPS15 | NM_001981.1 | 652 | ttgccatgttttggtatatctgt | 143203 | 548143 | | | see also 1263 line |
| 1265 | EPS15 | NM_001981.1 | 654 | gccatgttttggtatatctgtgc | 143204 | 548144 | | | see also 1263 line |
| 1266 | EPS15 | NM_001981.1 | 1193 | aagtgagttcaggatcttcaag | 143219 | 548161 | | | see also 1263 line |
| 1267 | ETV6 | NM_001987.2 | 1071 | ttctgacagccggtacgaaaact | 3635 | 443744 | transcription_regulator | transcription factor | leukemia, infantile fibrosarcoma |
| 1268 | ETV6 | NM_001987.2 | 1073 | ctgacagccggtacgaaaacttc | 3636 | 443745 | | | see also 1267 line |
| 1269 | ETV6 | NM_001987.2 | 1077 | cagccggtacgaaaacttcatcc | 3637 | 443746 | | | see also 1267 line |
| 1270 | ETV6 | NM_001987.2 | 1217 | tgcgccactacaaactaaac | 3639 | 443751 | | | see also 1267 line |
| 1271 | ETV6 | NM_001987.2 | 1218 | gcgccactacaaactaaaca | 3640 | 443752 | | | see also 1267 line |
| 1272 | ETV6 | NM_001987.2 | 1222 | cactactacaaactaaacattat | 3641 | 443753 | | | see also 1267 line |
| 1273 | EYA3 | NM_001990.2 | 340 | tacatccctcgctcatccaatga | 205028 | 595800 | phosphatase, transcription_regulator | | - |
| 1274 | EYA3 | NM_001990.2 | 343 | atccctcgctcatccaatgatta | 205029 | 595801 | | | see also 1273 line |
| 1275 | EYA3 | NM_001990.2 | 344 | tccctcgctcatccaatgattat | 205030 | 595802 | | | see also 1273 line |
| 1276 | EYA3 | NM_001990.2 | 735 | cagcatctcaaacaggattatc | 205042 | 595805 | | | see also 1273 line |
| 1277 | EYA3 | NM_001990.2 | 820 | gtcacaggtcagactaacagtga | 205043 | 595809 | | | see also 1273 line |
| 1278 | EZH1 | NM_001991.2 | 496 | atgagacggttttgtgcaatatt | 18532 | 459393 | gpcr, transcription_regulator, signal_transduction | chromatin binding | - |
| 1279 | FBLN1 | NM_001996.2 | 970 | ccgattttatcgtgtcagaatac | 144149 | 548312 | | | - |
| 1280 | FBLN1 | NM_001996.2 | 1629 | ctcctaccgctgcatcaacatcc | 144155 | 548325 | | | see also 1279 line |
| 1281 | FBLN1 | NM_001996.2 | 1705 | ggcgcgcaactgccaagacattga | 144157 | 548326 | | | see also 1279 line |
| 1282 | FBN2 | NM_001999.2 | 3485 | aacgtaaccctcctccttgtagg | 144483 | 548586 | | extracellular matrix structural constituent | congenital contractural arachnodactyly (Marfanoid-like) |
| 1283 | FBN2 | NM_001999.2 | 4649 | ctggtcgtatgagtgtaactgc | 144515 | 548609 | | | see also 1282 line |
| 1284 | FBN2 | NM_001999.2 | 8277 | agcatgctacgagtgcaaaatca | 144587 | 548667 | | | see also 1282 line |
| 1285 | FGF2 | NM_002006.2 | 785 | ttggaatctaataactacaatac | 169106 | 565907 | kinase_related, signal_transduction, cell_cycle | Ap-1(c-Jun/c-Fos heterodimer) | atherosclerosis, neuroblastoma, prostate cancer |
| 1286 | FGF2 | NM_002006.2 | 786 | tggaatctaataactacaatact | 169107 | 565908 | | | see also 1285 line |
| 1287 | FGF2 | NM_002006.2 | 850 | acgaactgggcagtataaacttg | 169109 | 565912 | | | see also 1285 line |
| 1288 | FGF9 | NM_002010.1 | 179 | atggctcccttaggtgaagttgg | 169188 | 565989 | signal_transduction, cell_cycle | heparin binding | - |
| 1289 | FGF9 | NM_002010.1 | 327 | cagtcacggacttggatcattta | 169196 | 565991 | | | see also 1288 line |

139

Figure 46

| | Gene | Accession | | Sequence | ID1 | ID2 | Notes | Function | Disease |
|---|---|---|---|---|---|---|---|---|---|
| 1290 | FGF9 | NM_002010.1 | 329 | gtcacggacttggatcatttaaa | 169197 | 565992 | see also 1288 line | | |
| 1291 | FKBP3 | NM_002013.2 | 722 | tggatgaggtccaccaaaatat | 179845 | 619012 | receptor_acitivity | receptor | - |
| 1292 | FKBP3 | NM_002013.2 | 830 | aagatgggacttgttttgatact | 179850 | 619018 | see also 1291 line | | |
| 1293 | FOXO1A | NM_002015.2 | 462 | cgctgcccaggccgagatttagc | 4129 | 444103 | transcription_regulator , apoptosis | transcription factor | rhabdomyosarcoma |
| 1294 | FOXO1A | NM_002015.2 | 1154 | atgacaacaacagtaaatttgc | 4132 | 444108 | see also 1293 line | | |
| 1295 | FOXO1A | NM_002015.2 | 1909 | cagccaggcatctcataacaaaa | 4157 | 444124 | see also 1293 line | | |
| 1296 | FLI1 | NM_002017.2 | 1053 | gcgccaacgccagctgatcacc | 3715 | 443884 | transcription_regulator | translation regulator | Ewing sarcoma, Ewing's sarcoma, Ewing's tumours |
| 1297 | FMR1 | NM_002024.1 | 447 | ttgctgttggtggttagctaaag | 47884 | 473787 | - | mRNA binding | mental retardation, Martin-Bell syndrome |
| 1298 | FMR1 | NM_002024.1 | 587 | aagatactttccataagatcaag | 47889 | 473799 | see also 1297 line | | |
| 1299 | FMR1 | NM_002024.1 | 1090 | aacttagtaggcaaagtaatagg | 47914 | 473821 | see also 1297 line | | |
| 1300 | FMR1 | NM_002024.1 | 1314 | ttctcaacctaacagtacaaaag | 47917 | 473822 | see also 1297 line | | |
| 1301 | FMR1 | NM_002024.1 | 1396 | aaggctttggcagggtatggtacc | 47919 | 473824 | see also 1297 line | | |
| 1302 | FMR1 | NM_002024.1 | 1401 | ttggcagggtatgtaccatttg | 47920 | 473825 | see also 1297 line | | |
| 1303 | FMR1 | NM_002024.1 | 1405 | cagggtatggtaccatttgttt | 47921 | 473827 | see also 1297 line | | |
| 1304 | FMR1 | NM_002024.1 | 1507 | ttgcgtttgagagattacaaat | 47924 | 473831 | see also 1297 line | | |
| 1305 | FMR1 | NM_002024.1 | 1568 | caccaccaaatcgtacagataag | 47927 | 473836 | see also 1297 line | | |
| 1306 | FMR1 | NM_002024.1 | 1569 | accaccaaatcgtacagataagg | 47928 | 473837 | see also 1297 line | | |
| 1307 | FMR1 | NM_002024.1 | 1572 | accaaatcgtacagataaggaaa | 47929 | 473838 | see also 1297 line | | |
| 1308 | FMR1 | NM_002024.1 | 1663 | ggcagacgcggtcctggatatac | 47931 | 473839 | see also 1297 line | | |
| 1309 | FMR1 | NM_002024.1 | 1667 | gacgcggtcctggatatacttca | 47932 | 473840 | see also 1297 line | | |
| 1310 | FMR1 | NM_002024.1 | 1669 | cgcggtcctggatatacttcagg | 47933 | 473841 | see also 1297 line | | |
| 1311 | FMR1 | NM_002024.1 | 2028 | tcggctgcacgggtaaagatc | 47938 | 473844 | see also 1297 line | | |
| 1312 | FMR1 | NM_002024.1 | 2029 | cggctgcgcacgggtaaagatcg | 47939 | 473845 | see also 1297 line | | |
| 1313 | FMR1 | NM_002024.1 | 2034 | gcgcacgggtaaagatcgttaac | 47940 | 473846 | see also 1297 line | | |
| 1314 | FMR2 | NM_002025.1 | 642 | tacaaggtagctgaatatacaaa | 314729 | 685592 | - | - | mental retardation |
| 1315 | FNTA | NM_002027.1 | 213 | agcagaatgggctgatatagatc | 216852 | 605143 | signal_transduction | protein geranylgeranyltransferase | |
| 1316 | FYN | NM_002037.3 | 1717 | tacatcgagcgcatgaattat | 83894 | 502856 | kinase_related | - | |
| 1317 | GABPB2 | NM_002041.2 | 615 | atgaagatttagcagagatatta | 4346 | 668921 | transcription_regulator | - | |
| 1318 | GABPB2 | NM_002041.2 | 627 | cagagatattacagattgctatg | 4347 | 668922 | see also 1317 line | | |
| 1319 | GALK2 | NM_002044.1 | 523 | aggttgaacttgcagaaatctgt | 83993 | 502940 | kinase_related | galactokinase | - |
| 1320 | GARS | NM_002047.1 | 1118 | tgcggatcttttgtgaactata | 84037 | 805196 | - | glycine-tRNA ligase | - |
| 1321 | GARS | NM_002047.1 | 1120 | cggatctttttgtgaactataat | 84038 | 805197 | see also 1320 line | | |

Figure 46

| | | | | | | | | | |
|---|---|---|---|---|---|---|---|---|---|
| 1322 | GATA1 | NM_002049.2 | 317 | tacagacactccccagtctttca | 4411 | 444323 | transcription_regulator | translation regulator | F-cell production、hereditary persistence of fetal hemoglobin |
| 1323 | GATA1 | NM_002049.2 | 319 | cagacactccccagtctttcagg | 4412 | 444324 | see also 1322 line | | |
| 1324 | GATA3 | NM_002051.1 | 1204 | cagaccctgactatgaagaagg | 4427 | 444353 | - | translation regulator | - |
| 1325 | GATA4 | NM_002052.2 | 1118 | ccgacaccccaatctcgatatgt | 4430 | 696689 | transcription_regulator | translation regulator | - |
| 1326 | GATA4 | NM_002052.2 | 1122 | caccccaatctcgatatgtttga | 4431 | 696691 | see also 1325 line | | |
| 1327 | GATA4 | NM_002052.2 | 1124 | ccccaatctcgatatgtttgacg | 4432 | 696692 | see also 1325 line | | |
| 1328 | GCG | NM_002054.2 | 392 | atgctgaagggacctttaccagt | 169830 | 566635 | signal_transduction、gpcr | peptide hormone | - |
| 1329 | GFPT1 | NM_002056.1 | 442 | gccaccccagcgctctgataaa | 174605 | 568492 | - | sugar binding | - |
| 1330 | GFPT1 | NM_002056.1 | 444 | caccccagcgctctgataaaaa | 174606 | 568493 | see also 1329 line | | |
| 1331 | GFPT1 | NM_002056.1 | 447 | cccagcgctctgataaaaataa | 174608 | 568494 | see also 1329 line | | |
| 1332 | GFPT1 | NM_002056.1 | 448 | cccagcgctctgataaaaataat | 174609 | 568495 | see also 1329 line | | |
| 1333 | GFPT1 | NM_002056.1 | 635 | taccttggtggagagagttatcc | 174618 | 568499 | see also 1329 line | | |
| 1334 | GFPT1 | NM_002056.1 | 1351 | cagtctttcgagatgatgtttgc | 174635 | 568519 | see also 1329 line | | |
| 1335 | GNA15 | NM_002068.1 | 572 | gccaggacccctataaagtgacc | 109115 | 519449 | gpcr、signal_transduction | signal transducer | - |
| 1336 | GNAL | NM_002071.1 | 533 | ttgtttcagcaatgagtactata | 109187 | 639017 | signal_transduction、gpcr | - | schizophrenia |
| 1337 | GNAL | NM_002071.1 | 619 | gcccctatcactgactttgaata | 109190 | 639021 | see also 1336 line | | |
| 1338 | GNAZ | NM_002073.1 | 990 | cgccaccgacaccagtaacatcc | 109265 | 519565 | signal_transduction、gpcr | receptor signaling protein | - |
| 1339 | GNB3 | NM_002075.2 | 749 | gggggctggacaacatgtgttcc | 192288 | 586103 | gpcr、signal_transduction | signal transducer | atherosclerosis、coronary artery disease |
| 1340 | GNS | NM_002076.1 | 688 | gtggactacctgacagatgtttt | 200514 | 592176 | - | N-acetylglucosamine-6-sulfatase | mucopolysaccharidosis、SanFillippo syndrome |
| 1341 | GOLGA1 | NM_002077.1 | 565 | tgctgaacaggtccgaaacttgc | 321565 | 686125 | - | - | - |
| 1342 | GOLGA1 | NM_002077.1 | 576 | tccgaaacttgcagaagataaaa | 321566 | 686126 | see also 1341 line | | |
| 1343 | GOLGA1 | NM_002077.1 | 637 | ttccatgcggaaatttcaagagc | 321567 | 686131 | see also 1341 line | | |
| 1344 | GOLGA4 | NM_002078.2 | 1328 | tgcgtgatgcaaagaacttaatt | 305552 | 686188 | - | - | - |
| 1345 | GOLGA4 | NM_002078.2 | 1329 | gcgtgatgcaaagaacttaattg | 305553 | 686189 | see also 1344 line | | |
| 1346 | GOT1 | NM_002079.1 | 799 | aagaacttcgggctctacaatga | 218012 | 605957 | - | aspartate aminotransferase | - |
| 1347 | GPRK6 | NM_002082.1 | 83 | agcgaacacggtgctactcaagg | 84283 | 503163 | gpcr、kinase_related、signal_transduction | G-protein-coupled receptor phosphorylating protein kinase | - |

Figure 46

| No. | Gene | Accession | Seq No. | Sequence | No. | No. | Category | Function | Disease |
|---|---|---|---|---|---|---|---|---|---|
| 1348 | GRB2 | NM_002086.2 | 358 | atggaagccatcgccaaatatga | 252679 | 535704 | - | SH3/SH2 adaptor protein | - |
| 1349 | GRB2 | NM_002086.2 | 363 | agccatcgccaaatatgacttca | 252681 | 535705 | see also 1348 line | | |
| 1350 | GRB2 | NM_002086.2 | 367 | atgccaaatatgacttcaaagc | 252682 | 535706 | see also 1348 line | | |
| 1351 | GRB2 | NM_002086.2 | 963 | cggcatgtttccccgcaattatg | 252691 | 535724 | see also 1348 line | | |
| 1352 | GRB2 | NM_002086.2 | 964 | ggcatgtttccccgcaattatgt | 252692 | 535725 | see also 1348 line | | |
| 1353 | GRIK5 | NM_002088.3 | 345 | tccaccgtgagccatatctgtgg | 244594 | 632447 | receptor_acitivity, cha nnel | potassium channel | - |
| 1354 | GRIK5 | NM_002088.3 | 734 | gggaatgacctcagcgtttaca | 244595 | 632451 | see also 1353 line | | |
| 1355 | GRIK5 | NM_002088.3 | 743 | ctcagcgtttacaagtacatcc | 244596 | 632453 | see also 1353 line | | |
| 1356 | GRSF1 | NM_002092.1 | 1125 | aagccaatgcccagacattata | 47970 | 484473 | - | mRNA binding | - |
| 1357 | GSK3B | NM_002093.2 | 1479 | gagaccgtggacagagaccaataat | 84399 | 503248 | kinase_related, signal_transduction | protein kinase CK2 | schizophrenia |
| 1358 | GTF2E2 | NM_002095.3 | 956 | ctgaagcgacagggtatttcttc | 275509 | 657965 | transcription_regulator | general RNA polymerase II transcription factor | - |
| 1359 | HAL | NM_002108.2 | 1620 | aggaaacttcatggtgaataacc | 232329 | 617276 | - | histidine ammonia-lyase | histidinemia |
| 1360 | HCK | NM_002110.2 | 999 | cttgatgccacctcaccaacaagc | 84739 | 503424 | kinase_related | protein tyrosine kinase | - |
| 1361 | HDC | NM_002112.1 | 1201 | cggtccttcgcggtgaagaatct | 233837 | 618212 | - | histidine decarboxylase | - |
| 1362 | HIVEP1 | NM_002114.1 | 941 | aactgagcacctgtcacaaaag | 28333 | 460093 | transcription_regulator | DNA binding | - |
| 1363 | HIVEP1 | NM_002114.1 | 5567 | cccagcaaatagtttagacatt | 28439 | 460194 | see also 1362 line | | |
| 1364 | HIVEP1 | NM_002114.1 | 5568 | cccagcaaaatagtttagacattg | 28440 | 460195 | see also 1362 line | | |
| 1365 | HLF | NM_002126.3 | 700 | ttccctatgacggagatacttc | 19948 | 460242 | transcription_regulator | double-stranded DNA binding | leukemia |
| 1366 | HLF | NM_002126.3 | 939 | ttgccagcaaaccgcaatacacc | 19950 | 460245 | see also 1365 line | | - |
| 1367 | HMGCS1 | NM_002130.3 | 895 | ttggcttcatgatctttcactca | 216522 | 603756 | - | | - |
| 1368 | HNRPD | NM_002138.2 | 658 | gactgcactcgaagttagatcc | 28567 | 460315 | transcription_regulator | | - |
| 1369 | HNRPD | NM_002138.2 | 667 | ctgaagttagatcctatcacagg | 28569 | 460316 | see also 1368 line | | |
| 1370 | HNRPD | NM_002138.2 | 726 | atcggagagtgtagataaggtca | 28571 | 460317 | see also 1368 line | | |
| 1371 | HOXB5 | NM_002147.2 | 121 | gcccggactcagttgctaaat | 5121 | 444755 | transcription_regulator | transcription factor | - |
| 1372 | HOXB5 | NM_002147.2 | 122 | cccggactatcagttgctaaatt | 5122 | 444756 | see also 1371 line | | |
| 1373 | HOXB5 | NM_002147.2 | 123 | ccggactatcagttgctaaatta | 5123 | 444757 | see also 1371 line | | |

Figure 46

| 1374 | HOXB5 | NM_002147.2 | 124 | cggactatcagttgctaaattat | 5124 | 444758 | see also 1371 line | | |
|---|---|---|---|---|---|---|---|---|---|
| 1375 | HOXB5 | NM_002147.2 | 199 | ccggctcttacggctacaattac | 5126 | 444759 | see also 1371 line | | |
| 1376 | HOXB5 | NM_002147.2 | 203 | ctcttacggctacaattacaatg | 5128 | 444760 | see also 1371 line | | |
| 1377 | HOXB5 | NM_002147.2 | 453 | agcgccaatttcaccgaaataga | 5129 | 444761 | see also 1371 line | | |
| 1378 | HOXD10 | NM_002148.2 | 123 | gcctgcaggagtgacagtttta | 5211 | 444854 | transcription_regulator | RNA polymerase II transcription factor | - |
| 1379 | HOXD10 | NM_002148.2 | 312 | ccgaacagatcttgtcgaataga | 5219 | 444862 | see also 1378 line | | |
| 1380 | HOXD10 | NM_002148.2 | 410 | ttctgataagcgcaacaaactca | 5222 | 444866 | see also 1378 line | | |
| 1381 | HOXD10 | NM_002148.2 | 412 | ctgataagcgcaacaaactcatt | 5223 | 444867 | see also 1378 line | | |
| 1382 | HOXD10 | NM_002148.2 | 539 | caccgggaaaacccaagagtaca | 5227 | 444869 | see also 1378 line | | |
| 1383 | HRC | NM_002152.1 | 2192 | tccaggaagctacgttgactatt | 146738 | 661291 | - | calcium ion binding | - |
| 1384 | HSPA4 | NM_002154.3 | 1178 | aactatgaatagaggcaaatttc | 85188 | 503887 | - | - | - |
| 1385 | TNC | NM_002160.1 | 6781 | gagatatggggacaataaccaca | 181468 | 662332 | - | AP-1(c-Jun homodimer) | - |
| 1386 | IARS | NM_002161.2 | 468 | ctgagtggaagtctactgttagc | 85465 | 501152 | - | - | autoimmune polymyositis/dermatomyositis |
| 1387 | ICSBP1 | NM_002163.1 | 1120 | ccccttgcgctccaaactcatt | 323 | 444951 | transcription_regulator | hematopoietic-associated factor 1B complex | arthritis |
| 1388 | ICSBP1 | NM_002163.1 | 1121 | cccttgcgctccaaactcattc | 324 | 444952 | see also 1387 line | | |
| 1389 | ICSBP1 | NM_002163.1 | 1122 | cccttgcgctccaaactcattct | 325 | 444953 | see also 1387 line | | |
| 1390 | IDH2 | NM_002168.2 | 446 | ggcccgtgtggaagagttcaagc | 220281 | 782259 | - | isocitrate dehydrogenase (NADP+) | - |
| 1391 | IDH2 | NM_002168.2 | 605 | cgcccatggcgaccagtacaagg | 220282 | 782261 | see also 1390 line | | |
| 1392 | IL1RAP | NM_002182.2 | 1684 | ggggcaacatcaacgtcatttta | 123185 | 527748 | receptor_acitivity | - | - |
| 1393 | IL1RAP | NM_002182.2 | 1760 | gacggtgctcacggtcattaaat | 123187 | 527749 | see also 1392 line | | |
| 1394 | IL1RAP | NM_002182.2 | 1762 | cggtgctcacggtcattaaatgg | 123188 | 527751 | see also 1392 line | | |
| 1395 | IL6ST | NM_002184.2 | 2309 | atggcaatttcactgatgtaagt | 124044 | 631943 | receptor_acitivity、signal_transduction | - | - |
| 1396 | IL7R | NM_002185.2 | 911 | cagtctccccgatcataagaaga | 123933 | 631974 | receptor_acitivity、signal_transduction | interleukin-7 receptor | - |
| 1397 | IL7R | NM_002185.2 | 996 | ttcctggactgccagattcatag | 123935 | 631980 | see also 1396 line | | |
| 1398 | INHA | NM_002191.2 | 1178 | atggaggttactctttcaagtat | 131888 | 535887 | cell_cycle、apoptosis、signal_transduction | hormone | - |
| 1399 | INHA | NM_002191.2 | 1179 | tggaggttactctttcaagtatg | 131889 | 535888 | see also 1398 line | | |
| 1400 | ACO1 | NM_002197.1 | 481 | atcattccatccaggttgatttc | 49588 | 471537 | - | aconitate hydratase | - |
| 1401 | ACO1 | NM_002197.1 | 506 | cagaagggcagacagtttacaga | 49589 | 471539 | see also 1400 line | | |

Figure 46

| | | | | | | | | | |
|---|---|---|---|---|---|---|---|---|---|
| 1402 | IRF1 | NM_002198.1 | 238 | tgcagattaattccaaccaaatc | 5452 | 697282 | transcription_regulator、cell_cycle | translation regulator | small cell lung cancer、arthritis、myelodysplastic syndrome |
| 1403 | ISL1 | NM_002202.1 | 366 | ttgaaatgtgcggagtgtaatca | 5572 | 445109 | - | RNA polymerase II transcription factor | diabetes mellitus、diabetes |
| 1404 | ISL1 | NM_002202.1 | 371 | atgtgcggagtgtaatcagtatt | 5573 | 445110 | see also 1403 line | | |
| 1405 | ISL1 | NM_002202.1 | 373 | gtgcggagtgtaatcagtatttg | 5574 | 445111 | see also 1403 line | | |
| 1406 | ISL1 | NM_002202.1 | 1260 | agcatggtagccagtcctattga | 5589 | 445123 | see also 1403 line | | |
| 1407 | ITGA7 | NM_002206.1 | 870 | gaccagctggtgtataaaacttt | 249277 | 635800 | receptor_acitivity、signal_transduction | cell adhesion receptor | myopathy |
| 1408 | ITGA7 | NM_002206.1 | 871 | accagctggtgtataaaactttg | 249278 | 635801 | see also 1407 line | | |
| 1409 | ITGA7 | NM_002206.1 | 1178 | gggtgccccctacttctttgagc | 249282 | 635807 | see also 1407 line | | |
| 1410 | ITIH2 | NM_002216.1 | 468 | aactgtgggccgagctctttatg | 292837 | 662737 | - | serine protease inhibitor | - |
| 1411 | ITPR1 | NM_002222.1 | 926 | atgaaatggagtgataacaaaga | 247242 | 636183 | channel、receptor_acitivity、signal_transduction | inositol-1、4、5-triphosphate receptor | - |
| 1412 | ITPR1 | NM_002222.1 | 1490 | aaggaagcatttgccatagttcc | 247251 | 636189 | see also 1411 line | | |
| 1413 | ITPR1 | NM_002222.1 | 5310 | tggtcaaccgttactatggaaac | 247311 | 636254 | see also 1411 line | | |
| 1414 | ITPR1 | NM_002222.1 | 5798 | gagcccacaacacagataacaga | 247316 | 636264 | see also 1411 line | | |
| 1415 | ITPR1 | NM_002222.1 | 6573 | atcagttggctcggcataacaaa | 247333 | 636276 | see also 1411 line | | |
| 1416 | ITPR1 | NM_002222.1 | 6580 | ggctcggcataacaaagaacttc | 247335 | 636278 | see also 1411 line | | |
| 1417 | JAK1 | NM_002227.1 | 682 | atcagctacaagcgatatattcc | 86052 | 504619 | kinase_related | protein tyrosine kinase | - |
| 1418 | JAK1 | NM_002227.1 | 692 | agcgatatattccagaaacattg | 86054 | 504620 | see also 1417 line | | |
| 1419 | JAK1 | NM_002227.1 | 1114 | atccgggaagagtggaacaattt | 86068 | 504635 | see also 1417 line | | |
| 1420 | JAK1 | NM_002227.1 | 1115 | tccgggaagagtggaacaatttt | 86069 | 504636 | see also 1417 line | | |
| 1421 | JAK1 | NM_002227.1 | 1443 | ctgcaccgactttgacaacatcc | 86076 | 504646 | see also 1417 line | | |
| 1422 | JAK1 | NM_002227.1 | 3019 | ttgggttctcggcaatacgttca | 86100 | 504675 | see also 1417 line | | |
| 1423 | JAK1 | NM_002227.1 | 3117 | agcaattgaaaccgataaggagt | 86103 | 504676 | see also 1417 line | | |
| 1424 | JUN | NM_002228.2 | 1942 | tgctaacgcagcagttgcaaaca | 5608 | 445136 | transcription_regulator | Smad3/Sp-1 heterodimer | - |
| 1425 | KCNA3 | NM_002232.2 | 1792 | ctctgagtaagtcggagtatatg | 132263 | 536241 | channel | protein binding | - |
| 1426 | KCNA3 | NM_002232.2 | 1796 | gagtaagtcggagtatatggtga | 132264 | 536242 | see also 1425 line | | |
| 1427 | KCNA4 | NM_002233.2 | 1787 | ttgaccctttgcgcaatgagtat | 132272 | 536255 | channel | voltage-gated potassium channel | - |
| 1428 | KCNA4 | NM_002233.2 | 1789 | gaccctttgcgcaatgagtattt | 132273 | 536256 | see also 1427 line | | |
| 1429 | KCNA4 | NM_002233.2 | 1791 | ccctttgcgcaatgagtattttt | 132274 | 536258 | see also 1427 line | | |
| 1430 | KCNA4 | NM_002233.2 | 1913 | aggaggtgaagttctatcagttg | 132276 | 536262 | see also 1427 line | | |

EP 1 752 536 A1

Figure 46

| | | | | | | | | | |
|---|---|---|---|---|---|---|---|---|---|
| 1431 | KCNA4 | NM_002233.2 | 2783 | gtgccattgcgggtgtcttaacc | 132282 | 536277 | see also 1427 line | | |
| 1432 | KCNA4 | NM_002233.2 | 3065 | cagataaaaacaactgttctaat | 132287 | 536284 | see also 1427 line | | |
| 1433 | KCNA6 | NM_002235.2 | 2120 | tacaatgaccacggtaggttacg | 132296 | 536315 | channel | voltage-gated potassium channel | - |
| 1434 | KCNJ3 | NM_002239.2 | 196 | atgtctgcactccgaaggaaatt | 260520 | 644151 | channel、gpcr | G-protein activated inward rectifier potassium channel | - |
| 1435 | KCNJ3 | NM_002239.2 | 210 | aaggaaatttggggacgattatc | 260521 | 644153 | see also 1434 line | | |
| 1436 | KCNJ3 | NM_002239.2 | 944 | aactggatgtaggttttagtaca | 260529 | 644162 | see also 1434 line | | |
| 1437 | KCNJ3 | NM_002239.2 | 946 | ctggatgtaggttttagtacagg | 260530 | 644163 | see also 1434 line | | |
| 1438 | KCNJ3 | NM_002239.2 | 1053 | aagcatgcaaactgaacagttcg | 260535 | 644165 | see also 1434 line | | |
| 1439 | KCNMA1 | NM_002247.2 | 802 | ttctacaaagatttcacattaca | 29330 | 460716 | - | - | - |
| 1440 | KCNMA1 | NM_002247.2 | 1008 | gagactgatacagttttcagaaa | 29335 | 460723 | see also 1439 line | | |
| 1441 | KCNMA1 | NM_002247.2 | 1076 | tggtgaatctgctctccatattt | 29336 | 460727 | see also 1439 line | | |
| 1442 | KCNMA1 | NM_002247.2 | 1585 | gtcctcaatccacatgatcttgc | 29349 | 460748 | see also 1439 line | | |
| 1443 | KCNMA1 | NM_002247.2 | 2359 | atgaggcatgaccccttgttaat | 29364 | 460763 | see also 1439 line | | |
| 1444 | KCNMA1 | NM_002247.2 | 2718 | gccattaagtcgggctgatttaa | 29377 | 460771 | see also 1439 line | | |
| 1445 | KCNMA1 | NM_002247.2 | 2760 | ctgtgacatgtgcgttatcctgt | 29378 | 460772 | see also 1439 line | | |
| 1446 | KCNMA1 | NM_002247.2 | 2784 | agccaatcagaataatattgatg | 29381 | 460774 | see also 1439 line | | |
| 1447 | KCNN3 | NM_002249.3 | 1213 | ttgttgttatggtgatagagacc | 121614 | 526743 | channel | - | schizophrenia |
| 1448 | KCNS3 | NM_002252.3 | 824 | gtcgtctctgtttgagaaagagc | 132593 | 536544 | channel | - | - |
| 1449 | KIF3C | NM_002254.4 | 811 | ccgtgaggcccctgatagactcc | 87656 | 505878 | - | motor | - |
| 1450 | KPNA1 | NM_002264.1 | 1090 | tagcccaaaggaatctatcaaaa | 133274 | 651337 | - | protein transporter | - |
| 1451 | KPNB1 | NM_002265.4 | 1560 | ggctatgcccaccctaatagaat | 157168 | 651515 | - | zinc binding | - |
| 1452 | KPNB1 | NM_002265.4 | 2253 | gggtggtgaattcctcaagtaca | 157183 | 651533 | see also 1451 line | | |
| 1453 | KPNB1 | NM_002265.4 | 2593 | gacatggtggattatctgaatga | 157196 | 651546 | see also 1451 line | | |
| 1454 | KPNA3 | NM_002267.2 | 754 | tgggattttaccaattctagtca | 177061 | 651364 | - | protein transporter | - |
| 1455 | KPNA3 | NM_002267.2 | 1075 | cacatgggtcattgtcaatctct | 177065 | 651372 | see also 1454 line | | |
| 1456 | KPNA4 | NM_002268.3 | 328 | gacaaccaacggctcaagaattt | 269321 | 651402 | - | protein transporter | - |
| 1457 | KPNA4 | NM_002268.3 | 375 | gactatgagaagacaacgaaatg | 269324 | 651409 | see also 1456 line | | |
| 1458 | KPNA4 | NM_002268.3 | 398 | aagttgtagttgaattaaggaag | 269326 | 651411 | see also 1456 line | | |
| 1459 | KPNA4 | NM_002268.3 | 495 | tggtgattatagagtgcaaaata | 269331 | 651417 | see also 1456 line | | |
| 1460 | KPNA4 | NM_002268.3 | 508 | gtgcaaaatacctctctagaagc | 269332 | 651418 | see also 1456 line | | |
| 1461 | KPNA4 | NM_002268.3 | 516 | tacctctctagaagctattgttc | 269333 | 651419 | see also 1456 line | | |
| 1462 | KPNA4 | NM_002268.3 | 1601 | tcgatggactaagtaatatatta | 269355 | 651447 | see also 1456 line | | |
| 1463 | KPNA4 | NM_002268.3 | 1604 | atggactaagtaatatattaaaa | 269356 | 651448 | see also 1456 line | | |
| 1464 | KPNB2 | NM_002270.2 | 1516 | cacgtacctgaagccattaatga | 177117 | 803341 | - | - | - |

EP 1 752 536 A1

Figure 46

| | | | | | | | | | |
|---|---|---|---|---|---|---|---|---|---|
| 1465 | KPNB2 | NM_002270.2 | 1639 | tccttaccttgcttatatacttg | 177122 | 803350 | see also 1464 line | | |
| 1466 | KPNB3 | NM_002271.3 | 3272 | ttcttggcccaaacaataccaat | 127655 | 651569 | - | protein transporter | - |
| 1467 | LAMB1 | NM_002291.1 | 643 | ttgctccaaaccgccttaagatt | 291166 | 661616 | - | structural molecule | - |
| 1468 | LAMB1 | NM_002291.1 | 644 | tgctccaaaccgccttaagattt | 291167 | 661617 | see also 1467 line | | |
| 1469 | LAMB1 | NM_002291.1 | 1025 | taccaacttgagaatcaagtttg | 291179 | 661629 | see also 1467 line | | |
| 1470 | LAMB2 | NM_002292.2 | 1604 | ggccagtgtcgctgcaaagaaca | 291279 | 549211 | - | structural molecule | - |
| 1471 | LAMC1 | NM_002293.2 | 904 | aggccttgtgtactgatgaattc | 291344 | 661732 | - | structural molecule | - |
| 1472 | LAMC1 | NM_002293.2 | 2845 | gcctgaagtgcatctataacact | 291381 | 661760 | see also 1471 line | | |
| 1473 | LBR | NM_002296.2 | 1325 | tggatgggtggttattaacttgg | 30204 | 484876 | receptor_acitivity | - | hydrops-ectopic calcification-moth-eaten skeletal dysplasia、 Pelger-Huet anomaly |
| 1474 | LCP1 | NM_002298.2 | 855 | ctgtggcaagtcatcaagattgg | 117912 | 523294 | - | calcium ion binding | non-hodgkin lymphoma |
| 1475 | LIFR | NM_002310.2 | 3235 | tacagacctcaggccaatgtaaa | 243815 | 632075 | receptor_acitivity、 sig nal_transduction | leukemia inhibitory factor receptor | - |
| 1476 | LIFR | NM_002310.2 | 3379 | gactctcctaaatctaatggagg | 243826 | 632079 | see also 1475 line | | |
| 1477 | LIFR | NM_002310.2 | 3399 | aggagggtggtcctttacaaact | 243827 | 632081 | see also 1475 line | | |
| 1478 | LIFR | NM_002310.2 | 3401 | gagggtggtcctttacaaacttt | 243828 | 632082 | see also 1475 line | | |
| 1479 | LIFR | NM_002310.2 | 3402 | agggtggtcctttacaaactttt | 243829 | 632083 | see also 1475 line | | |
| 1480 | LIG3 | NM_002311.2 | 449 | gtctggggggtgatatgaaagagt | 30543 | 461199 | - | - | - |
| 1481 | LIG3 | NM_002311.2 | 451 | ctgggggtgatatgaaagagtgg | 30544 | 461200 | see also 1480 line | | |
| 1482 | LIG3 | NM_002311.2 | 1597 | agcgagtccaggtgcataagaat | 30570 | 461228 | see also 1480 line | | |
| 1483 | LIG3 | NM_002311.2 | 1939 | accggatcatgttctcagaaatg | 30576 | 461237 | see also 1480 line | | |
| 1484 | LIG4 | NM_002312.2 | 282 | ctcacaaacttcacaaactgttg | 30599 | 506500 | - | DNA ligase (ATP) | - |
| 1485 | ABLIM1 | NM_002313.4 | 675 | acccggagaccgagtcacattca | 115022 | 521125 | - | - | - |
| 1486 | ABLIM1 | NM_002313.4 | 1213 | atctatgcaaaagtagacaatga | 115044 | 521145 | see also 1485 line | | |
| 1487 | ABLIM1 | NM_002313.4 | 1237 | atcctggattacaaggatttagc | 115045 | 521146 | see also 1485 line | | |
| 1488 | ABLIM1 | NM_002313.4 | 1614 | cgctcaggcccctaaacatttcc | 115058 | 521151 | see also 1485 line | | |
| 1489 | LIMK1 | NM_002314.2 | 786 | ctgcatgagcccagatgtgaaga | 88511 | 506595 | kinase_related、 signal _transduction | - | Williams-Beuren syndrome |
| 1490 | LOX | NM_002317.3 | 1354 | tgccagtggattgatattacaga | 163087 | 560801 | - | protein-lysine 6-oxidase | - |
| 1491 | LRP1 | NM_002332.1 | 2291 | gtggactggatgggagacaatct | 147393 | 549320 | receptor_acitivity | receptor | atherosclerosis、 Alzheimer disease |
| 1492 | LRP1 | NM_002332.1 | 2436 | cactcaatgggtggatgtactgg | 147403 | 549326 | see also 1491 line | | |
| 1493 | LRP1 | NM_002332.1 | 3150 | gcccctcggaccgattcaagtgc | 147411 | 549337 | see also 1491 line | | |
| 1494 | LRP1 | NM_002332.1 | 5189 | aacaccacctgctatgagtttaa | 147432 | 549359 | see also 1491 line | | |
| 1495 | LRP1 | NM_002332.1 | 5191 | caccacctgctatgagtttaaga | 147433 | 549360 | see also 1491 line | | |
| 1496 | LRP1 | NM_002332.1 | 7695 | accgctatgtgatcctaaagtca | 147454 | 549403 | see also 1491 line | | |
| 1497 | LRP1 | NM_002332.1 | 9663 | accgagagcagatgatctactgg | 147473 | 549426 | see also 1491 line | | |

146

Figure 46

| 1498 | LRP1 | NM_002332.1 | 12718 | cagcaaacgaggcctaagtcacc | 147503 | 549463 | see also 1491 line | | |
|------|------|-------------|-------|--------------------------|--------|--------|--------------------|---|---|
| 1499 | LRP6 | NM_002336.1 | 170 | gcgattggttgatgctacaaatg | 133382 | 636400 | receptor_acitivity、sig nal_transduction | - | - |
| 1500 | LRP6 | NM_002336.1 | 236 | agctgcggtggactttgtgttta | 133384 | 636402 | see also 1499 line | | |
| 1501 | LRP6 | NM_002336.1 | 288 | agcgaagaagccattaaacgaac | 133386 | 636404 | see also 1499 line | | |
| 1502 | LRP6 | NM_002336.1 | 486 | ttggatcaacccagagctattgc | 133396 | 636412 | see also 1499 line | | |
| 1503 | LRP6 | NM_002336.1 | 754 | atccttttgccttgacgttattt | 133405 | 636425 | see also 1499 line | | |
| 1504 | LRP6 | NM_002336.1 | 755 | tccttttgccttgacgttatttg | 133406 | 636426 | see also 1499 line | | |
| 1505 | LRP6 | NM_002336.1 | 949 | ggggttgttcccatttgtgtttg | 133411 | 636437 | see also 1499 line | | |
| 1506 | LRP6 | NM_002336.1 | 979 | cagtcaagcctttttatcagtgt | 133413 | 636439 | see also 1499 line | | |
| 1507 | LRP6 | NM_002336.1 | 2544 | ctcaaccgtgaagttatagcaga | 133485 | 636484 | see also 1499 line | | |
| 1508 | LRP6 | NM_002336.1 | 2907 | atcaaccgcatggtgattgatga | 133493 | 636488 | see also 1499 line | | |
| 1509 | LRP6 | NM_002336.1 | 3369 | ttcagtggcttaagtaaaccaat | 133505 | 636505 | see also 1499 line | | |
| 1510 | LRP6 | NM_002336.1 | 3437 | ttcagatctccggcgaattgaaa | 133508 | 636508 | see also 1499 line | | |
| 1511 | LRP6 | NM_002336.1 | 3439 | cagatctccggcgaattgaaagc | 133509 | 636509 | see also 1499 line | | |
| 1512 | LRP6 | NM_002336.1 | 3543 | tggctctattggattgataaaca | 133513 | 636517 | see also 1499 line | | |
| 1513 | LSAMP | NM_002338.1 | 83 | ctgttcgcagcgtggattttaac | 319178 | 789881 | - | - | - |
| 1514 | LSAMP | NM_002338.1 | 609 | ctcctcggcggatgtcaaacaag | 319187 | 789890 | see also 1513 line | | |
| 1515 | LSAMP | NM_002338.1 | 610 | tcctcggcggatgtcaaacaagt | 319188 | 789891 | see also 1513 line | | |
| 1516 | LSAMP | NM_002338.1 | 612 | ctcggcggatgtcaaacaagtca | 319189 | 789892 | see also 1513 line | | |
| 1517 | LSAMP | NM_002338.1 | 615 | ggcggatgtcaaacaagtcaagg | 319190 | 789893 | see also 1513 line | | |
| 1518 | LY75 | NM_002349.1 | 1585 | gagaaacctgttacaagatttat | 175337 | 568835 | receptor_acitivity | - | - |
| 1519 | LY75 | NM_002349.1 | 3551 | cagtcaagatgatgaactcaact | 175411 | 568903 | see also 1518 line | | |
| 1520 | LY75 | NM_002349.1 | 3980 | ttcatgggtcatgttaggaataa | 175426 | 568916 | see also 1518 line | | |
| 1521 | LY75 | NM_002349.1 | 4410 | tgggttgggctctcaagtcatga | 175434 | 568929 | see also 1518 line | | |
| 1522 | TACSTD1 | NM_002354.1 | 599 | acctactggatcatcattgaact | 327545 | 697557 | - | - | - |
| 1523 | MAD | NM_002357.1 | 315 | tagcagatcaactcacaatgaaa | 6240 | 461316 | transcription_regulator | transcription factor | - |
| 1524 | MAFG | NM_002359.1 | 639 | accagcgtcatcacaatagtaaa | 6311 | 461365 | transcription_regulator | p18(MAFG)/p45 heterodimer | - |
| 1525 | MAN2A1 | NM_002372.1 | 1173 | gtggtgggatattatagatattc | 201035 | 592687 | - | mannosyl- oligosaccharide 1、3- 1、6-alpha- mannosidase | - |
| 1526 | MAP2 | NM_002374.2 | 432 | gggtcacagggcacctattcaaa | 121650 | 526775 | - | - | - |
| 1527 | MAP2 | NM_002374.2 | 521 | tgcaaggatagttcaagtagtca | 121655 | 526780 | see also 1526 line | | |
| 1528 | MAP2 | NM_002374.2 | 5223 | cagcttcggcttattaaccaacc | 121754 | 526866 | see also 1526 line | | |

EP 1 752 536 A1

Figure 46

| 1529 | MARK3 | NM_002376.3 | 2201 | aacggcgaaccgcaacatataat | 90304 | 507995 | kinase_related | protein tyrosine kinase | - |
|---|---|---|---|---|---|---|---|---|---|
| 1530 | MARK3 | NM_002376.3 | 2203 | cggcgaaccgcaacatataatgg | 90305 | 507997 | see also 1529 line | | |
| 1531 | MATK | NM_002378.2 | 1551 | aagtcggatgtctggagttttgg | 90429 | 508071 | kinase_related、cell_cycle | - | - |
| 1532 | MATN1 | NM_002379.2 | 1262 | acggcaagacctgcaatgtctgc | 288884 | 686504 | - | extracellular matrix structural constituent | rheumatoid arthritis |
| 1533 | MATN1 | NM_002379.2 | 1646 | gccggagccaggactacattaat | 288887 | 686510 | see also 1532 line | | |
| 1534 | MATN2 | NM_002380.2 | 2081 | cccaattgacctggtctttgtga | 148148 | 706641 | - | - | - |
| 1535 | MAX | NM_002382.3 | 256 | atgcactggaacgaaaacgtagg | 6315 | 445426 | - | - | - |
| 1536 | MDM2 | NM_002392.1 | 482 | tggccagtatattatgactaaac | 233223 | 537472 | transcription_regulator、cell_cycle | - | Well-differentiated liposarcoma、rhabdomyosarcoma、liposarcoma、chondrosarcoma、osteosarcoma、ovarian cancer、leukemia、testicular cancer、nerve sheath tumors、astrocytic tumors |
| 1537 | MDM2 | NM_002392.1 | 1202 | ttccttagctgactattggaaat | 233232 | 537476 | see also 1536 line | | |
| 1538 | MDM4 | NM_002393.1 | 760 | atggctcaactgatttacagaca | 308821 | 690336 | apoptosis、transcription_regulator | protein binding | - |
| 1539 | MDM4 | NM_002393.1 | 794 | gggtactgccattgtttcagata | 308823 | 690337 | see also 1538 line | | |
| 1540 | MDM4 | NM_002393.1 | 1062 | agcaagaggtactgttttcgttg | 308829 | 690346 | see also 1538 line | | |
| 1541 | ME2 | NM_002396.2 | 829 | gccggaacacactcattcagttc | 220867 | 608008 | - | malate dehydrogenase (oxaloacetate decarboxylating) | - |
| 1542 | MEF2C | NM_002397.2 | 926 | ttctctgcagaggaatagtatgt | 6397 | 445447 | transcription_regulator | translation regulator | - |
| 1543 | MEF2C | NM_002397.2 | 1253 | ctcccagtcggctcagtcattgg | 6405 | 445459 | see also 1542 line | | |
| 1544 | MEF2C | NM_002397.2 | 1290 | tccgtagcaactcctactttacc | 6406 | 445461 | see also 1542 line | | |
| 1545 | MEIS1 | NM_002398.1 | 117 | ggcatcccctccacgatgtatgg | 6409 | 441268 | transcription_regulator | RNA polymerase II transcription factor | - |
| 1546 | MEIS1 | NM_002398.1 | 194 | tcctctgcactcgcatcagtacc | 6410 | 441269 | see also 1545 line | | |
| 1547 | MEIS1 | NM_002398.1 | 262 | ctgtcaatgacgctttaaagaga | 6411 | 441271 | see also 1545 line | | |
| 1548 | MEIS1 | NM_002398.1 | 270 | gacgctttaaagagagataaaga | 6413 | 441272 | see also 1545 line | | |
| 1549 | MEIS1 | NM_002398.1 | 398 | ctgctcgtcagagtcattcaatg | 6414 | 441274 | see also 1545 line | | |
| 1550 | MEIS1 | NM_002398.1 | 401 | ctcgtcagagtcattcaatgaag | 6415 | 441276 | see also 1545 line | | |
| 1551 | MEIS1 | NM_002398.1 | 444 | cagattcgcgcagaaaaacctct | 6419 | 441283 | see also 1545 line | | |
| 1552 | MEIS1 | NM_002398.1 | 598 | aagggaaaatgcctatcgatttg | 6421 | 441291 | see also 1545 line | | |
| 1553 | MEIS1 | NM_002398.1 | 608 | gcctatcgatttggtgatagacg | 6422 | 441292 | see also 1545 line | | |

Figure 46

| 1554 | MEIS1 | NM_002398.1 | 671 | aagatcagcaaatctaactgacc | 6423 | 441296 | see also 1545 line | | |
|------|-------|-------------|-----|-------------------------|------|--------|--------------------|---|---|
| 1555 | MEIS1 | NM_002398.1 | 867 | aaggacaaaaagcgtcacaaaaa | 6425 | 441299 | see also 1545 line | | |
| 1556 | MEIS2 | NM_002399.2 | 1830 | agcacagagccccacaatgttaa | 6459 | 445968 | transcription_regulator | - | - |
| 1557 | MGAT2 | NM_002408.2 | 490 | atgaggttccgcatctacaaacg | 210571 | 759589 | - | transferase、transferring glycosyl groups | carbohydrate-deficient glycoprotein syndrome(-) |
| 1558 | MGAT2 | NM_002408.2 | 1055 | caggtagtgaccctagagattgt | 210580 | 759598 | see also 1557 line | | |
| 1559 | MGAT2 | NM_002408.2 | 1620 | ctgtggtatgcatcacaagaaaa | 210597 | 759614 | see also 1557 line | | |
| 1560 | MGAT2 | NM_002408.2 | 1658 | ctcagagtgcccaaattgagtca | 210599 | 759616 | see also 1557 line | | |
| 1561 | MGAT3 | NM_002409.3 | 249 | gagacgctacaagctctttctca | 210498 | 701591 | - | transferase、transferring glycosyl groups | |
| 1562 | MGAT3 | NM_002409.3 | 251 | gacgctacaagctctttctcatg | 210499 | 701592 | see also 1561 line | | |
| 1563 | MMP1 | NM_002421.2 | 739 | ctcttggactctcccattctact | 148441 | 558789 | - | Ap-1(c-Jun/c-Fos heterodimer) | chondrosarcoma、epidermolysis bullosa dystrophica、melanoma |
| 1564 | MMP13 | NM_002427.2 | 1365 | gacccatacagtttgaatacagc | 148544 | 550267 | - | zinc binding | - |
| 1565 | MMP15 | NM_002428.1 | 1430 | tcccctatgaccgcattgacacg | 158130 | 558671 | - | zinc binding | - |
| 1566 | MNAT1 | NM_002431.1 | 206 | ttcagggtacaactctttgaaga | 158436 | 669139 | cell_cycle、kinase_related、transcription_regulator | zinc binding | |
| 1567 | MOG | NM_002433.1 | 1010 | gggtgccctgctggaagataacc | 314205 | 692698 | - | - | - |
| 1568 | MPP1 | NM_002436.2 | 453 | tgggggatgagatcctagaaatc | 209380 | 681179 | kinase_related、signal_transduction | guanylate kinase | - |
| 1569 | MPP1 | NM_002436.2 | 939 | ttcggctccctgcattcaagagg | 209395 | 681189 | see also 1568 line | | |
| 1570 | MRC1 | NM_002438.1 | 2119 | gtggtttgaatctcgagattttt | 148689 | 567659 | receptor_acitivity | receptor | - |
| 1571 | MRC1 | NM_002438.1 | 2120 | tggtttgaatctcgagatttttg | 148690 | 567660 | see also 1570 line | | |
| 1572 | MRC1 | NM_002438.1 | 3973 | aagtaaaaccaatttttggatag | 148738 | 567707 | see also 1570 line | | |
| 1573 | MRC1 | NM_002438.1 | 4076 | ccctctggtgaacggaatgattg | 148740 | 567708 | see also 1570 line | | |
| 1574 | MSH5 | NM_002441.2 | 819 | cggggttgaactggaagactata | 16641 | 451620 | - | - | - |
| 1575 | MSH5 | NM_002441.2 | 2197 | gtggcgaaagcagtgaacaatgc | 16667 | 451643 | see also 1574 line | | |
| 1576 | MSI1 | NM_002442.2 | 111 | cgcacgacccctgcaagatgttc | 52801 | 472417 | - | - | - |
| 1577 | MSI1 | NM_002442.2 | 162 | aggaagggctgcgcgaatacttc | 52802 | 472419 | see also 1576 line | | |
| 1578 | MSI1 | NM_002442.2 | 311 | cacgagctcgactccaaaacaat | 52805 | 472423 | see also 1576 line | | |
| 1579 | MSI1 | NM_002442.2 | 314 | gagctcgactccaaaacaattga | 52807 | 472424 | see also 1576 line | | |
| 1580 | MSN | NM_002444.1 | 866 | gtcatcaagcccattgacaaaaa | 287497 | 661802 | - | structural constituent of cytoskeleton | - |

Figure 46

| | | | | | | | | | |
|---|---|---|---|---|---|---|---|---|---|
| 1581 | MYBPC1 | NM_002465.1 | 829 | aagagcgcagcttttgcaaaaat | 118019 | 788818 | - | structural constituent of muscle | - |
| 1582 | MYBPC1 | NM_002465.1 | 831 | gagcgcagcttttgcaaaaattc | 118020 | 788819 | see also 1581 line | | |
| 1583 | MYBPC1 | NM_002465.1 | 2112 | aacttttgagcccaagaagatga | 118067 | 788856 | see also 1581 line | | |
| 1584 | MYBPC1 | NM_002465.1 | 2330 | atggctatgtgctagagtattgc | 118070 | 788859 | see also 1581 line | | |
| 1585 | MYBPC1 | NM_002465.1 | 2338 | gtgctagagtattgctttgaagg | 118071 | 788860 | see also 1581 line | | |
| 1586 | MYC | NM_002467.2 | 836 | accaggctgcgcgcaaagacagc | 7233 | 446140 | transcription_regulator、cell_cycle | transcription factor-7-like-2-E | mycosis Fungoides、chondrosarcoma、non-hodgkin lymphoma、breast cancer、osteosarcoma、Ewing's sarcoma、astrocytic tumors |
| 1587 | MYC | NM_002467.2 | 1641 | gcggaaacgacgagaacagttga | 7239 | 446146 | see also 1586 line | | |
| 1588 | NASP | NM_002482.2 | 299 | tccaggaagcagctagtctttta | 301258 | 530125 | - | - | - |
| 1589 | NASP | NM_002482.2 | 306 | agcagctagtcttttaggtaaga | 301260 | 530126 | see also 1588 line | | |
| 1590 | NUBP1 | NM_002484.1 | 886 | agcctacagaagtataattcaga | 95171 | 510662 | - | molecular_function unknown | - |
| 1591 | NBS1 | NM_002485.3 | 603 | ctcatttgtggacgtccaattgt | 16977 | 690423 | cell_cycle | - | Nijmegen breakage syndrome、non-hodgkin lymphoma |
| 1592 | NDUFA6 | NM_002490.2 | 178 | acgggataaagtccgagaaatgt | 222655 | 610001 | - | NADH dehydrogenase (ubiquinone) | - |
| 1593 | NDUFA6 | NM_002490.2 | 180 | gggataaagtccgagaaatgttt | 222656 | 610002 | see also 1592 line | | |
| 1594 | NDUFS4 | NM_002495.1 | 247 | ttgttcctgctcgcaataacatg | 222893 | 610140 | - | NADH dehydrogenase (ubiquinone) | complex I deficiency、Leigh syndrome |
| 1595 | NEO1 | NM_002499.1 | 582 | caggtcttccaagatttaccagc | 312575 | 683684 | - | molecular_function unknown | - |
| 1596 | NEO1 | NM_002499.1 | 1328 | ttgggtctggtgaaatcagatga | 312598 | 683702 | see also 1595 line | | |
| 1597 | NEO1 | NM_002499.1 | 1961 | gtggtggcctacaataaacatgg | 312619 | 683717 | see also 1595 line | | |
| 1598 | NEO1 | NM_002499.1 | 2302 | ctggctgtctgctgaaacttttg | 312628 | 683728 | see also 1595 line | | |
| 1599 | NEO1 | NM_002499.1 | 2485 | gaccatcaaagtggactataaac | 312630 | 683730 | see also 1595 line | | |
| 1600 | NEO1 | NM_002499.1 | 3137 | gagatacatgactgggttattga | 312651 | 683751 | see also 1595 line | | |
| 1601 | NEO1 | NM_002499.1 | 3294 | aagcggactcctctgataaaatg | 312659 | 683758 | see also 1595 line | | |
| 1602 | NEO1 | NM_002499.1 | 3295 | agcggactcctctgataaaatgc | 312660 | 683759 | see also 1595 line | | |
| 1603 | NEO1 | NM_002499.1 | 3895 | tcccatccattccctcgataacc | 312673 | 683773 | see also 1595 line | | |
| 1604 | NEO1 | NM_002499.1 | 4427 | gactccgagagtagctatgaacc | 312679 | 683781 | see also 1595 line | | |
| 1605 | NEO1 | NM_002499.1 | 4471 | ggcccacctggaaggactaatga | 312680 | 683782 | see also 1595 line | | |
| 1606 | NEUROD1 | NM_002500.1 | 1039 | ggcccaaagccacggatcaatct | 33741 | 462599 | transcription_regulator | translation regulator | diabetes、diabetes mellitus |

Figure 46

| 1607 | NFIX | NM_002501.1 | 540 | tggagtcacaatcaaagaactgg | 399 | 446675 | transcription_regulator | translation regulator | - |
|------|------|-------------|-----|-------------------------|-----|--------|--------------------------|------------------------|---|
| 1608 | NFIX | NM_002501.1 | 591 | tccggaatccggacaatcagata | 400 | 446676 | see also 1607 line | | |
| 1609 | NFIX | NM_002501.1 | 592 | ccggaatccggacaatcagatag | 401 | 446677 | see also 1607 line | | |
| 1610 | NFIX | NM_002501.1 | 593 | cggaatccggacaatcagatagt | 402 | 446678 | see also 1607 line | | |
| 1611 | NFIX | NM_002501.1 | 746 | cagcatcagggcccaacttctcc | 403 | 446683 | see also 1607 line | | |
| 1612 | NFIX | NM_002501.1 | 1169 | agcagtcgagcccgtatttcacg | 405 | 446686 | see also 1607 line | | |
| 1613 | NFX1 | NM_002504.3 | 2392 | gtgtgaccatccagtatatcatt | 8081 | 446844 | transcription_regulator | - | - |
| 1614 | NFX1 | NM_002504.3 | 2394 | gtgaccatccagtatatcattct | 8082 | 446845 | see also 1613 line | | |
| 1615 | NFX1 | NM_002504.3 | 2499 | tcccctgtcacctggttgatatc | 8085 | 446849 | see also 1613 line | | |
| 1616 | NFX1 | NM_002504.3 | 2505 | gtcacctggttgatatctcttgc | 8086 | 446851 | see also 1613 line | | |
| 1617 | GPNMB | NM_002510.1 | 1189 | agccaccatcacaattgtagagg | 308779 | 567336 | - | - | melanoma |
| 1618 | NPAS2 | NM_002518.2 | 589 | aacagacggcagcatcatctatg | 8189 | 446914 | - | transcription factor | - |
| 1619 | NPTX1 | NM_002522.1 | 575 | agcagtacagccgcctcaattcc | 305743 | 697955 | - | - | - |
| 1620 | NPTX2 | NM_002523.1 | 1280 | agctcagccagttcaacatatgg | 175569 | 569073 | - | molecular_function unknown | - |
| 1621 | NRD1 | NM_002525.1 | 371 | cccgtctaggagcggatgaatct | 184379 | 578252 | - | nardilysin | - |
| 1622 | NRD1 | NM_002525.1 | 473 | accgatacatcaaattacagaat | 184386 | 578261 | see also 1621 line | | |
| 1623 | NRD1 | NM_002525.1 | 1124 | gtgataatgcctcaactgattgt | 184400 | 578267 | see also 1621 line | | |
| 1624 | NRD1 | NM_002525.1 | 2570 | atgtggcacagctggagtataaa | 184441 | 578297 | see also 1621 line | | |
| 1625 | NRD1 | NM_002525.1 | 3207 | gacccttgggtaccatgtctacc | 184456 | 578317 | see also 1621 line | | |
| 1626 | NRD1 | NM_002525.1 | 3583 | atgctcagcgttcatgttgttgg | 184470 | 578322 | see also 1621 line | | |
| 1627 | NRD1 | NM_002525.1 | 3771 | cccctaccataaaatagtcaaat | 184476 | 578326 | see also 1621 line | | |
| 1628 | NT5E | NM_002526.1 | 829 | tgggaagtacccattcatagtca | 197269 | 589516 | - | IMP-GMP specific 5'-nucleotidase | - |
| 1629 | NTF3 | NM_002527.2 | 702 | aggaagccaggccggtcaaaaac | 169382 | 538391 | apoptosis、signal_transduction | growth factor | - |
| 1630 | NUP88 | NM_002532.3 | 80 | tcctaaccacgtcgtgttcttgc | 274032 | 772968 | - | - | - |
| 1631 | NVL | NM_002533.1 | 479 | aggaggatggtttattgacaaaa | 95177 | 488043 | - | - | - |
| 1632 | OAZ2 | NM_002537.1 | 214 | aacacccaggacagtagtattt | 149306 | 664356 | - | ornithine decarboxylase inhibitor | - |
| 1633 | OAZ2 | NM_002537.1 | 262 | cagtgctgcaggcacattgttcc | 149307 | 664358 | see also 1632 line | | |
| 1634 | OAZ2 | NM_002537.1 | 367 | ctcagctctaatatataaggacg | 149308 | 664361 | see also 1632 line | | |
| 1635 | ODF2 | NM_002540.3 | 410 | tggcgcacccagtgtaactgtga | 292164 | 704566 | - | - | - |
| 1636 | ODF2 | NM_002540.3 | 737 | ggcggagtgtccgggtgaaaacc | 292132 | 704571 | see also 1635 line | | |

EP 1 752 536 A1

Figure 46

| | | | | | | | | | |
|---|---|---|---|---|---|---|---|---|---|
| 1637 | OGDH | NM_002541.1 | 158 | ttcaacagattcggtgctattct | 225465 | 611870 | - | oxoglutarate dehydrogenase (lipoamide) | Parkinson disease |
| 1638 | OGDH | NM_002541.1 | 210 | tgggactagttcgaactatgtgg | 225466 | 611871 | see also 1637 line | | |
| 1639 | OMG | NM_002544.2 | 1397 | ctgtcttataaccactttactga | 319430 | 683951 | - | - | - |
| 1640 | OMG | NM_002544.2 | 2260 | agctgcaactctaactattcatc | 319462 | 683978 | see also 1639 line | | |
| 1641 | OPHN1 | NM_002547.1 | 1951 | gtgactgggacattaagacaatc | 296846 | 665686 | signal_transduction | Rho GTPase activator | mental retardation |
| 1642 | ORC4L | NM_002552.2 | 223 | aagaattttacgtgaaagatttt | 20638 | 463303 | - | - | - |
| 1643 | ORC4L | NM_002552.2 | 273 | ttggagtgcaagtacaatacaaa | 20641 | 463304 | see also 1642 line | | |
| 1644 | ORC4L | NM_002552.2 | 732 | gtcttacatgtagattggatatt | 20651 | 463312 | see also 1642 line | | |
| 1645 | ORC4L | NM_002552.2 | 1130 | ttggaaatctgtcttataatagc | 20672 | 463325 | see also 1642 line | | |
| 1646 | ORC5L | NM_002553.2 | 851 | aacgtgatactcgcaaactgtgg | 20695 | 510816 | - | - | - |
| 1647 | ORC5L | NM_002553.2 | 860 | ctcgcaaactgtggagaaatatt | 20696 | 510817 | see also 1646 line | | |
| 1648 | P2RX1 | NM_002558.2 | 907 | gtgaagacgtgtgagatctttgg | 95277 | 510908 | receptor_acitivity、channel、apoptosis、signal_transduction | ATP-gated cation channel | - |
| 1649 | P2RX1 | NM_002558.2 | 1511 | tgcctaagaggcactactacaag | 95281 | 510915 | see also 1648 line | | |
| 1650 | P2RY1 | NM_002563.2 | 1092 | atctccgtgtacatgttcaattt | 241073 | 628428 | receptor_acitivity、gpcr | purinergic nucleotide receptor、 G-protein coupled | - |
| 1651 | P2RY1 | NM_002563.2 | 1094 | ctccgtgtacatgttcaatttgg | 241074 | 628429 | see also 1650 line | | |
| 1652 | FURIN | NM_002569.2 | 765 | gcctcggtacacacagatgaatg | 182886 | 576424 | - | Smad3/Sp-1 heterodimer | - |
| 1653 | FURIN | NM_002569.2 | 768 | tcggtacacacagatgaatgaca | 182887 | 576425 | see also 1652 line | | |
| 1654 | PAFAH1B2 | NM_002572.1 | 663 | ctgccacgacatgtttgattttc | 200164 | 591840 | - | 2-acetyl-1-alkylglycerophosphocholine esterase | - |
| 1655 | PAFAH1B2 | NM_002572.1 | 664 | tgccacgacatgtttgattttct | 200165 | 591841 | see also 1654 line | | |
| 1656 | SERPINB2 | NM_002575.1 | 585 | ctgggtcaagactcaaaccaaag | 183575 | 577403 | apoptosis | Smad3/Sp-1 heterodimer | - |
| 1657 | PAK1 | NM_002576.2 | 428 | ccccagcccctccgatgagaaat | 95447 | 511039 | kinase_related、apoptosis | protein tyrosine kinase | - |
| 1658 | PAK3 | NM_002578.1 | 135 | agccaggcttcgctctatcttcc | 95493 | 511068 | kinase_related | protein tyrosine kinase | mental retardation |
| 1659 | PAK3 | NM_002578.1 | 262 | accggggaattcactggaattcc | 95496 | 511073 | see also 1658 line | | |
| 1660 | PAK3 | NM_002578.1 | 1370 | aaggtgaaccccccttaccttaat | 95519 | 511110 | see also 1658 line | | |

Figure 46

| No. | Gene | Accession | Length | Sequence | ID1 | ID2 | Annotation | Description | Disease |
|---|---|---|---|---|---|---|---|---|---|
| 1661 | PAK3 | NM_002578.1 | 1371 | aggtgaacccccttaccttaatg | 95520 | 511111 | | | |
| 1662 | PAK3 | NM_002578.1 | 1378 | cccccttaccttaatgaaaatcc | 95521 | 511112 | see also 1658 line | | |
| 1663 | PBX1 | NM_002585.1 | 1176 | gtcactcaatgggggattcttacc | 8968 | 447600 | transcription_regulator | PBX1b/PREP1 heterodimer | lymphoblastic leukemia |
| 1664 | PBX2 | NM_002586.3 | 1293 | caggcctcggcggctctttcaat | 8970 | 447605 | transcription_regulator | translation regulator | lymphoblastic leukemia |
| 1665 | PBX2 | NM_002586.3 | 1299 | ctggcgggctcttcaatctctca | 8971 | 447608 | see also 1664 line | - | |
| 1666 | PCDHGC3 | NM_002588.2 | 610 | aagcaactctttacaaaacctatg | 150532 | 744822 | - | | |
| 1667 | PCDHGC3 | NM_002588.2 | 1637 | cgctatttcacaataaatcgtga | 150557 | 744859 | see also 1666 line | | |
| 1668 | PCDHGC3 | NM_002588.2 | 1643 | ttcacaataaatcgtgacaatgg | 150558 | 744860 | see also 1666 line | | |
| 1669 | PCDH7 | NM_002589.2 | 3888 | tggacagaggtatgatagtgtca | 149954 | 551398 | - | | |
| 1670 | PCDH7 | NM_002589.2 | 3892 | cagaggtatgatagtgtcaatga | 149955 | 551399 | see also 1669 line | | |
| 1671 | PCDH7 | NM_002589.2 | 3894 | gaggtatgatagtgtcaatgaga | 149956 | 551400 | see also 1669 line | | |
| 1672 | PCDH8 | NM_002590.2 | 3191 | tcctctgcgcaggacaattact | 149984 | 551432 | - | | |
| 1673 | PCDH8 | NM_002590.2 | 3193 | ctctgcgcaggacaattactac | 149985 | 551433 | see also 1672 line | | |
| 1674 | PCDH8 | NM_002590.2 | 3248 | gagcgtctatgagaaagtactgc | 149986 | 551438 | see also 1672 line | | |
| 1675 | PCSK2 | NM_002594.2 | 1966 | ttgacaagtggcctttcatgacc | 183096 | 576823 | - | subtilase | |
| 1676 | PCTK2 | NM_002595.2 | 869 | gtctcagaaatcgtatacatga | 95975 | 511408 | kinase_related, cell_cycle | protein serine/threonine kinase | |
| 1677 | PCTK2 | NM_002595.2 | 1082 | ttggagaggtacatatgcaaca | 95982 | 511412 | see also 1676 line | | |
| 1678 | PCTK2 | NM_002595.2 | 1518 | gacctactcaaatgaagttgtca | 95992 | 511422 | see also 1676 line | | |
| 1679 | PCTK3 | NM_002596.1 | 1608 | aagacactgcctccatctctcc | 96011 | 511450 | - | | |
| 1680 | PDE4B | NM_002600.2 | 1305 | aaacctacatgatgacttagaag | 197842 | 590150 | signal_transduction | cAMP-specific phosphodiesterase | |
| 1681 | PDE4B | NM_002600.2 | 1306 | acctacatgatgactttagaaga | 197843 | 590151 | see also 1680 line | | |
| 1682 | PDE7A | NM_002603.1 | 221 | gtggagctatttcctatgcacagt | 197917 | 590389 | signal_transduction | | |
| 1683 | PDE7A | NM_002603.1 | 629 | ttgagtacttccatttagatatg | 197938 | 590408 | see also 1682 line | | |
| 1684 | PDE7A | NM_002603.1 | 1334 | acctagtggagcctttatttaca | 197966 | 590434 | see also 1682 line | | |
| 1685 | PDE8A | NM_002605.1 | 612 | tgcaggtctatcagatcatcaaa | 161884 | 561529 | signal_transduction | signal transducer | |
| 1686 | PDE8A | NM_002605.1 | 1134 | cactatgtgtccattatcagagt | 161903 | 561530 | see also 1685 line | | |
| 1687 | PDGFRB | NM_002609.2 | 3140 | tgcctccgacgagatctatgagaa | 96094 | 511603 | receptor_acitivity, signal_transduction, kinase_related, se_related | platelet-derived growth factor receptor | leukemia |
| 1688 | PDK1 | NM_002610.3 | 721 | aggaagtccatctcatcgaaaac | 96124 | 499219 | kinase_related, signal_transduction | pyruvate dehydrogenase (lipoamide) kinase | |

Figure 46

| # | Gene | Accession | Len | Sequence | ID1 | ID2 | Category | Function | Disease/Notes |
|---|---|---|---|---|---|---|---|---|---|
| 1689 | PDK2 | NM_002611.3 | 730 | agctcctgtgtgacaagtattac | 96143 | 511613 | kinase_related, signal transduction | pyruvate dehydrogenase (lipoamide) kinase | - |
| 1690 | PDZK1 | NM_002614.3 | 265 | taggatcaatgtgtgctttgtgg | 274109 | 681370 | - | - | - |
| 1691 | PER1 | NM_002616.1 | 2299 | ccgctcgcctggccaataagg | 254746 | 639329 | signal_transduction, transcription regulator | signal transducer | - |
| 1692 | PER1 | NM_002616.1 | 2394 | cccggagtcggacatcatcatg | 254747 | 639330 | see also 1691 line | | |
| 1693 | PER1 | NM_002616.1 | 2395 | ccggagtcggacatcatcatga | 254748 | 639331 | see also 1691 line | | |
| 1694 | PER1 | NM_002616.1 | 2397 | cggagtcggacatcatcatgatg | 254749 | 639332 | see also 1691 line | | |
| 1695 | PER1 | NM_002616.1 | 2944 | ggccttggctgctccctaactac | 254750 | 639338 | see also 1691 line | | |
| 1696 | PEX13 | NM_002618.1 | 356 | ccgcctccgtgtagatgatcttc | 269721 | 688579 | | protein transporter | adrenoleukodystrophy, peroxisome biogenesis disorder |
| 1697 | PEX13 | NM_002618.1 | 357 | cgcctccgtgatgatgatcttcc | 269722 | 688580 | see also 1696 line | | |
| 1698 | PFKP | NM_002627.2 | 1701 | atgtgccgggttccgatttcagc | 166453 | 563881 | kinase_related | magnesium binding | - |
| 1699 | PFN2 | NM_002628.2 | 82 | cggctactgcgacgccaatacg | 118724 | 523540 | | - | - |
| 1700 | PFN2 | NM_002628.2 | 177 | acgggaaggttctttaccaac | 118730 | 523546 | see also 1699 line | | |
| 1701 | PFN2 | NM_002628.2 | 178 | ccgggaaggttctttaccaacg | 118731 | 523547 | see also 1699 line | | |
| 1702 | PFN2 | NM_002628.2 | 269 | atggaacatccggacaaagagtca | 118734 | 523550 | see also 1699 line | | |
| 1703 | PFN2 | NM_002628.2 | 291 | aaggtgggagccaacatacaat | 118735 | 523551 | see also 1699 line | | |
| 1704 | PHF1 | NM_002636.3 | 375 | gagaagtgtcgccatgcttatca | 9227 | 463476 | transcription_regulator | transcription regulator | - |
| 1705 | PHF1 | NM_002636.3 | 983 | cagccacaaggaccgtttcattt | 9235 | 463483 | see also 1704 line | | |
| 1706 | PHF1 | NM_002636.3 | 984 | agccacaaggaccgtttcatttc | 9236 | 463484 | see also 1704 line | | |
| 1707 | PHF1 | NM_002636.3 | 985 | gccacaaggaccgtttcatttca | 9237 | 463485 | see also 1704 line | | |
| 1708 | PHKA1 | NM_002637.1 | 1017 | aagcttcagggtcgttatggttg | 121925 | 527098 | kinase_related | phosphorylase kinase, regulator | glycogen storage disease |
| 1709 | PHKA1 | NM_002637.1 | 1195 | cagaacaggttcaagaatataaa | 121930 | 527104 | see also 1708 line | | |
| 1710 | PHKA1 | NM_002637.1 | 3076 | gcgttcgtcccactgattcaaat | 121974 | 527155 | see also 1708 line | | |
| 1711 | PHKA1 | NM_002637.1 | 3080 | tcgtcccactgattcaaatgtca | 121975 | 527156 | see also 1708 line | | |
| 1712 | PHKA1 | NM_002637.1 | 3168 | atgcagttaaaaagtgagataaa | 121977 | 527158 | see also 1708 line | | |
| 1713 | SERPINB5 | NM_002639.1 | 556 | gtggttaatgctgcctactttgt | 293346 | 663068 | - | serpin | - |
| 1714 | PIGA | NM_002641.1 | 413 | ctgccattgctcaggtacatatt | 210221 | 602076 | - | | paroxysmal nocturnal hemoglobinuria |
| 1715 | PIGA | NM_002641.1 | 414 | tgccattgctcaggtacatattt | 210222 | 602077 | see also 1714 line | | |
| 1716 | PIGA | NM_002641.1 | 498 | ctctcttccacgccaagacaatg | 210227 | 602084 | see also 1714 line | | |
| 1717 | PIGA | NM_002641.1 | 804 | tcgatttgcttagtgtgtataata | 210241 | 602092 | see also 1714 line | | |
| 1718 | PIGF | NM_002643.2 | 509 | taggaccaaacctcaaagcatgg | 209524 | 667568 | - | | - |
| 1719 | PIK3C2A | NM_002645.1 | 1171 | ttgaagaccaaatttccatatac | 206862 | 597014 | kinase_related | | - |

Figure 46

| 1720 | PIK3C2A | NM_002645.1 | 1365 | ttgctgggtacatgatgacttga | 206868 | 597020 | see also 1719 line | | |
|------|---------|-------------|------|-------------------------|--------|--------|--------------------|---|---|
| 1721 | PIK3C2A | NM_002645.1 | 1368 | ctgggtacatgatgacttgaatc | 206869 | 597021 | see also 1719 line | | |
| 1722 | PIK3C2A | NM_002645.1 | 1600 | ctgtatcaaatagaaaaaccttg | 206875 | 597031 | see also 1719 line | | |
| 1723 | PIK3C2A | NM_002645.1 | 2220 | atgggatgaactaatcatttttc | 206902 | 597057 | see also 1719 line | | |
| 1724 | PIK3C2A | NM_002645.1 | 2221 | tgggatgaactaatcattttcc | 206903 | 597058 | see also 1719 line | | |
| 1725 | PIK3C2A | NM_002645.1 | 3434 | aagatatgttagctttacagatg | 206935 | 597094 | see also 1719 line | | |
| 1726 | PIK3C2A | NM_002645.1 | 3717 | ctgtgtagccacctatgttttag | 206942 | 597100 | see also 1719 line | | |
| 1727 | PIK3C2A | NM_002645.1 | 4406 | ttcacaataagctcagtattatt | 206959 | 597126 | see also 1719 line | | |
| 1728 | PIK3C3 | NM_002647.2 | 2219 | gtcatggacacttacgttaaaag | 207250 | 596961 | kinase_related | - | - |
| 1729 | PIK3CG | NM_002649.2 | 1967 | aagcaattggaggcgatcatagc | 207480 | 597475 | kinase_related、signal_transduction、gpcr | phosphatidylinositol 3-kinase、class III | - |
| 1730 | PIK3CG | NM_002649.2 | 2580 | atgacgtcagttcccaagttatt | 207494 | 597492 | see also 1729 line | | |
| 1731 | PIK3CG | NM_002649.2 | 2582 | gacgtcagttcccaagttatttc | 207495 | 597493 | see also 1729 line | | |
| 1732 | PIK3CG | NM_002649.2 | 3543 | aaggatggactgtgcagtttaat | 207527 | 597526 | see also 1729 line | | |
| 1733 | PIK4CB | NM_002651.1 | 498 | gaggctgtttgagtcaaaactgt | 207773 | 597921 | - | - | - |
| 1734 | PIK4CB | NM_002651.1 | 632 | tgccccagttgcttaacatgtac | 207776 | 597925 | see also 1733 line | | |
| 1735 | PITX1 | NM_002653.3 | 1128 | gcccaactcgggcctcaacaaca | 9347 | 447766 | transcription_regulator | translation regulator | - |
| 1736 | PLAG1 | NM_002655.1 | 490 | gtcattcctggtgatttgtcaga | 9406 | 709773 | transcription_regulator | - | lipoblastoma、pleiomorphic adenoma gene |
| 1737 | PLAG1 | NM_002655.1 | 497 | ctggtgatttgtcagaagtaaga | 9407 | 709774 | see also 1736 line | | |
| 1738 | PLAGL2 | NM_002657.2 | 312 | aagtgaagtgccaatgtgaaatt | 9447 | 707462 | transcription_regulator | translation regulator | - |
| 1739 | PLAGL2 | NM_002657.2 | 390 | cagagcagagaccatatagctgc | 9451 | 707465 | see also 1738 line | | |
| 1740 | PLAGL2 | NM_002657.2 | 1230 | agcttggatctacctcatacttg | 9460 | 707471 | see also 1738 line | | |
| 1741 | PLCG2 | NM_002661.1 | 2076 | gagtccaagccgtggtactatga | 151188 | 590823 | signal_transduction | signal transducer | - |
| 1742 | PLCG2 | NM_002661.1 | 3447 | ttcaagacgacggttgtgaatga | 151201 | 590846 | see also 1741 line | | |
| 1743 | PLD2 | NM_002663.2 | 758 | gtggccgagaccaagtttgttat | 197474 | 589697 | signal_transduction | phospholipase D | - |
| 1744 | PLEK | NM_002664.1 | 579 | tcgccaggaaggcctcatgattg | 151291 | 681438 | - | calcium ion binding | - |
| 1745 | PLRG1 | NM_002669.1 | 694 | gacttggctagtggcaaattaaa | 113200 | 706756 | transcription_regulator | - | - |
| 1746 | PLRG1 | NM_002669.1 | 1443 | agctgataaaaccattaaagtat | 113226 | 706780 | see also 1745 line | | |
| 1747 | PLRG1 | NM_002669.1 | 1514 | aaccagaaattatcaagagaaag | 113227 | 706782 | see also 1745 line | | |
| 1748 | PMSCL2 | NM_002685.1 | 1349 | ccgggatgacacccattacctgc | 53417 | 485373 | kinase_related | protein serine/threonine kinase | - |
| 1749 | PNN | NM_002687.2 | 268 | aggcgtggattctcagatagtgg | 292363 | 698357 | - | - | melanoma |
| 1750 | PNN | NM_002687.2 | 884 | ttgaaggtagacgcatcgaattt | 292374 | 698375 | see also 1749 line | | |

155

EP 1 752 536 A1

Figure 46

| | | | | | | | | | |
|---|---|---|---|---|---|---|---|---|---|
| 1751 | POLA2 | NM_002689.2 | 1232 | ttcctggacaggttgtaattatg | 283019 | 670162 | - | Smad3/Sp-1 heterodimer | - |
| 1752 | POLB | NM_002690.1 | 392 | gagttcatccatcaatttcctga | 35738 | 463967 | - | zeta DNA polymerase | - |
| 1753 | POLB | NM_002690.1 | 520 | agcgaattgggctgaaatatttt | 35741 | 463971 | see also 1752 line | | |
| 1754 | POLG | NM_002693.1 | 964 | ctggtggaagagcgttactcttg | 35939 | 464216 | - | zeta DNA polymerase | - |
| 1755 | POU2F1 | NM_002697.1 | 711 | ctcctgcaagcgcaaaatcttca | 9636 | 447815 | transcription_regulator | translation regulator | - |
| 1756 | POU2F1 | NM_002697.1 | 950 | aagacgaatcaaacttggattca | 9638 | 447820 | see also 1755 line | | |
| 1757 | POU2F2 | NM_002698.1 | 926 | acgtccgcttcgccttagagaag | 9668 | 447849 | transcription_regulator | Octamer-binding transcription factor 2 | - |
| 1758 | POU2F2 | NM_002698.1 | 1195 | agcacaacagttactaccttatc | 9670 | 447851 | see also 1757 line | | |
| 1759 | POU4F3 | NM_002700.1 | 113 | ccccgcagctgcagggtaatata | 9703 | 447885 | transcription_regulator | transcription factor | deafness |
| 1760 | POU4F3 | NM_002700.1 | 114 | cccgcagctgcagggtaatatat | 9704 | 447886 | see also 1759 line | | |
| 1761 | POU4F3 | NM_002700.1 | 115 | ccgcagctgcagggtaatatatt | 9705 | 447887 | see also 1759 line | | |
| 1762 | POU4F3 | NM_002700.1 | 770 | ccgcctaccgagagaagaacagc | 9710 | 447895 | see also 1759 line | | |
| 1763 | PPP1CB | NM_002709.1 | 1034 | aacgacagttggtaaccttattt | 162205 | 561726 | - | protein phosphatase type 2C | - |
| 1764 | PPP1CB | NM_002709.1 | 1035 | acgacagttggtaaccttatttt | 162206 | 561727 | see also 1763 line | | |
| 1765 | PPP1R3A | NM_002711.2 | 509 | tggcagacacattatgacatttt | 195620 | 746596 | phosphatase | - | diabetes mellitus、diabetes |
| 1766 | PPP1R3A | NM_002711.2 | 773 | ggctgcttaaaggtaaaatcaag | 195628 | 746608 | see also 1765 line | | |
| 1767 | PPP2CA | NM_002715.1 | 1025 | agctgcaatcatggaacttgacg | 162260 | 561750 | phosphatase、apoptosis、cell_cycle、transcription_regulator、signal_transduction | protein phosphatase type 2C | - |
| 1768 | PPP6C | NM_002721.3 | 316 | tagacagaggttactatagtttg | 162311 | 561822 | - | protein phosphatase type 2C | - |
| 1769 | PPP6C | NM_002721.3 | 332 | tagtttggagaccttcacttacc | 162312 | 561823 | see also 1768 line | | |
| 1770 | PPP6C | NM_002721.3 | 472 | atgctaatgcctggagatactgt | 162319 | 561828 | see also 1768 line | | |
| 1771 | PPP6C | NM_002721.3 | 515 | cacagtagcagctttaatagatg | 162320 | 561833 | see also 1768 line | | |
| 1772 | PRKAG1 | NM_002733.1 | 796 | ggggcgtgtggtggacatctact | 205928 | 671352 | kinase_related、signal_transduction | cAMP-dependent protein kinase | - |
| 1773 | PRKAR1A | NM_002734.2 | 1080 | ttggggccttctgattattttgg | 108038 | 539166 | kinase_related、transcription_regulator | cAMP-dependent protein kinase、regulator | lupus erythematosus、thyroid cancer、papillary Thyroid Carcinomas |

Figure 46

| | | | | | | | | | |
|---|---|---|---|---|---|---|---|---|---|
| 1774 | PRKCA | NM_002737.1 | 1473 | tgggatgtgcaaggaacacatga | 97034 | 512047 | kinase_related、cell_cycle、signal_transduction、apoptosis | protein kinase C | melanoma |
| 1775 | PRKCB1 | NM_002738.4 | 1618 | tggggtgacaaccaagacattct | 97060 | 512065 | kinase_related | protein tyrosine kinase | - |
| 1776 | PRKCI | NM_002740.3 | 877 | cagtaattccatataatccttca | 97228 | 512219 | kinase_related、signal_transduction | diacylglycerol binding | - |
| 1777 | PRKCI | NM_002740.3 | 893 | tccttcaagtcatgagagtttgg | 97229 | 512220 | see also 1776 line | | |
| 1778 | PRKCI | NM_002740.3 | 1339 | ttcatgagcgagggataatttat | 97234 | 512235 | see also 1776 line | | |
| 1779 | PRKCI | NM_002740.3 | 1342 | atgagcgagggataatttataga | 97235 | 512236 | see also 1776 line | | |
| 1780 | PRKCM | NM_002742.1 | 772 | gtgtggtctgaattaccataaga | 97359 | 512264 | kinase_related | diacylglycerol binding | - |
| 1781 | PRKCM | NM_002742.1 | 777 | gtctgaattaccataagagatgt | 97361 | 512265 | see also 1780 line | | |
| 1782 | PRKCM | NM_002742.1 | 786 | accataagagatgtgcatttaaa | 97362 | 512267 | see also 1780 line | | |
| 1783 | PRKCM | NM_002742.1 | 2145 | ttcatcaccctggtgttgtaaat | 97400 | 512295 | see also 1780 line | | |
| 1784 | PRKCM | NM_002742.1 | 2186 | acgcctgaaagagtgtttgttgt | 97401 | 512298 | see also 1780 line | | |
| 1785 | PRKCM | NM_002742.1 | 2194 | aagagtgtttgttgttatggaaa | 97402 | 512299 | see also 1780 line | | |
| 1786 | PRKCM | NM_002742.1 | 2756 | gactatcagacctggttagattt | 97417 | 512310 | see also 1780 line | | |
| 1787 | MAPK1 | NM_002745.2 | 821 | gggctccagaaattatgttgaat | 89826 | 507598 | kinase_related、cell_cycle、signal_transduction、apoptosis | - | - |
| 1788 | MAPK4 | NM_002747.2 | 1357 | tggagaagatcctgacctttaac | 89939 | 507705 | kinase_related、cell_cycle | MAP kinase | - |
| 1789 | MAPK6 | NM_002748.2 | 1281 | acggatcatggatcctcattatt | 89947 | 507722 | kinase_related、cell_cycle、signal_transduction | protein serine/threonine kinase | - |
| 1790 | MAPK7 | NM_002749.2 | 785 | cagtacttcatgactgagtatgt | 90011 | 507773 | kinase_related、cell_cycle、signal_transduction | - | - |
| 1791 | MAPK8 | NM_002750.2 | 31 | agcgtgacaacaatttttatagt | 90031 | 507786 | kinase_related、signal_transduction | - | - |
| 1792 | MAPK8 | NM_002750.2 | 353 | tgcaaatctttgccaagtgattc | 90039 | 507798 | see also 1791 line | | |
| 1793 | MAPK8 | NM_002750.2 | 544 | caggaacgagttttatgatgacg | 90047 | 507805 | see also 1791 line | | |
| 1794 | MAPK8 | NM_002750.2 | 815 | tgctggatatagctttgagaaac | 90051 | 507812 | see also 1791 line | | |
| 1795 | MAPK9 | NM_002752.3 | 222 | gccgtcctttcagaaccaaact | 90067 | 507824 | kinase_related | - | - |
| 1796 | MAPK9 | NM_002752.3 | 927 | aagccagagatctgttatcaaaa | 90091 | 507842 | see also 1795 line | | |
| 1797 | MAPK9 | NM_002752.3 | 1045 | cccaccacctcaaatttatgatg | 90095 | 507853 | see also 1795 line | | |

Figure 46

| 1798 | MAP2K1 | NM_002755.2 | 984 | ccgacctccatggcaattttg | 88950 | 506747 | kinase_related, signal transduction | protein tyrosine kinase | bladder cancer |
|---|---|---|---|---|---|---|---|---|---|
| 1799 | MAP2K3 | NM_002756.2 | 381 | tggactcccgaccttcatcacc | 88965 | 506758 | kinase_related, signal transduction | - | - |
| 1800 | MAP2K3 | NM_002756.2 | 469 | agcctatggggtcgttagagaagg | 88966 | 506759 | see also 1799 line | - | |
| 1801 | MAP2K5 | NM_002757.2 | 1593 | aagacgtatgttggaacaaatgc | 89004 | 506835 | kinase_related, signal transduction | - | |
| 1802 | MAP2K5 | NM_002757.2 | 1596 | acgtatgttggaacaaatgctta | 89005 | 506836 | see also 1801 line | - | |
| 1803 | PROX1 | NM_002763.3 | 495 | aagagggcgaactgtgatgaaga | 37093 | 465018 | - | specific RNA polymerase II transcription factor | - |
| 1804 | PROX1 | NM_002763.3 | 497 | gagggcgaactgtgatgaagatg | 37094 | 465019 | see also 1803 line | - | |
| 1805 | PROX1 | NM_002763.3 | 498 | aggggcgaactgtgatgaagatgc | 37095 | 465020 | see also 1803 line | | |
| 1806 | PROX1 | NM_002763.3 | 698 | cccttttggcaggcctactatga | 37101 | 465026 | see also 1803 line | | |
| 1807 | PROX1 | NM_002763.3 | 816 | gtggcattaagggcaatgaaaa | 37104 | 465027 | see also 1803 line | | |
| 1808 | PRPS2 | NM_002765.2 | 604 | ggggagccaaaagggttacatca | 166686 | 598861 | kinase_related | ribose-phosphate pyrophosphokinase | hyperuricemia |
| 1809 | PRPS2 | NM_002765.2 | 608 | agccaaaagggttacatcaattg | 166688 | 598862 | see also 1808 line | - | |
| 1810 | PRPS2 | NM_002765.2 | 609 | gccaaaagggttacatcaattgc | 166689 | 598863 | see also 1808 line | | |
| 1811 | PRPS2 | NM_002765.2 | 850 | gaccagctatttccagaataat | 166693 | 598874 | see also 1808 line | | |
| 1812 | PRSS11 | NM_002775.2 | 521 | ggcaggaagatccaacagtttg | 124939 | 528794 | - | trypsin | |
| 1813 | PRSS11 | NM_002775.2 | 1032 | gggagcccgttagtaaacctgg | 124958 | 528809 | see also 1812 line | - | |
| 1814 | PSMA1 | NM_002786.2 | 149 | cagggcaggattcatcaaattga | 53601 | 579410 | - | - | |
| 1815 | PSMA4 | NM_002789.3 | 172 | ttctccagaggtcgcttatacc | 185188 | 579437 | - | proteasome endopeptidase | - |
| 1816 | PSMA4 | NM_002789.3 | 175 | tccagaagtcgcttataccaag | 185189 | 579438 | see also 1815 line | - | |
| 1817 | PSMA4 | NM_002789.3 | 652 | gtcaatgttgaaacaagactata | 185206 | 579451 | see also 1815 line | | |
| 1818 | PSMA7 | NM_002792.2 | 652 | agccattgaaacagatgatctga | 185237 | 579483 | - | - | |
| 1819 | PSMC2 | NM_002803.2 | 385 | ttcggaggaccaaaatacatta | 98322 | 512704 | - | adenosinetriphosphatase | |
| 1820 | PSMC3 | NM_002804.3 | 697 | agggcccacggagccaatacagtga | 98365 | 512739 | transcription_regulator | transcription co-repressor | |
| 1821 | PSMC5 | NM_002805.4 | 909 | aaggttatcatgctactaatag | 98412 | 512788 | - | transcription cofactor | - |
| 1822 | PSMC6 | NM_002806.2 | 498 | cagcgtgtaggaataataacctcc | 98431 | 512816 | - | proteasome ATPase | - |
| 1823 | PSMC6 | NM_002806.2 | 644 | tggtgaaagtgctcgtttgatca | 98437 | 512820 | see also 1822 line | | |
| 1824 | PSMD2 | NM_002808.3 | 2266 | ctgcgccagttagctcaatatca | 323226 | 750781 | cell_cycle | | |
| 1825 | PSMD2 | NM_002808.3 | 2268 | gcgccagttagctcaatatcatg | 323227 | 750782 | see also 1824 line | - | |

Figure 46

| | | | | | | | | | |
|---|---|---|---|---|---|---|---|---|---|
| 1826 | PSMD2 | NM_002808.3 | 2269 | cgccagttagtcaatatcatgc | 323228 | 750783 | see also 1824 line | | |
| 1827 | PSMD3 | NM_002809.2 | 1546 | aggagatgattgacatctattcc | 323439 | 688073 | - | | |
| 1828 | PSMD7 | NM_002811.3 | 333 | atggttttagaccatgatatt | 57197 | 477171 | - | | |
| 1829 | PSMD7 | NM_002811.3 | 334 | tggtttttagaccatgattatt | 57198 | 477172 | see also 1828 line | | |
| 1830 | PSMD8 | NM_002812.3 | 532 | ctcaaatgctactacttgatta | 302313 | 670873 | cell_cycle | | |
| 1831 | PSMD8 | NM_002812.3 | 537 | atgctactacttgattacaagg | 302314 | 670874 | see also 1830 line | | |
| 1832 | PSMD11 | NM_002815.2 | 978 | tccggatgacccaatcatcagc | 323379 | 801915 | - | | |
| 1833 | PSMD12 | NM_002816.2 | 662 | ctgcctagctgtgaaggattaca | 323159 | 687996 | - | | |
| 1834 | PSMD13 | NM_002817.2 | 339 | atcctaatgtggctcttacttt | 323404 | 687615 | - | | |
| 1835 | PSMD13 | NM_002817.2 | 578 | cacgcgtcctactacaaagatgc | 323409 | 687632 | see also 1834 line | | |
| 1836 | PTPN4 | NM_002830.2 | 1018 | aggttggctggatccaaacaaacc | 193740 | 587025 | phosphatase | structural molecule | |
| 1837 | PTPN4 | NM_002830.2 | 1104 | aagtgacccaacaagttacaag | 193744 | 587031 | see also 1836 line | | |
| 1838 | PTPN4 | NM_002830.2 | 1108 | gacccaacaagttacaagaaga | 193745 | 587032 | see also 1836 line | | |
| 1839 | PTPN4 | NM_002830.2 | 1272 | gtcaggctacctctcagattatt | 193748 | 587041 | see also 1836 line | | |
| 1840 | PTPN4 | NM_002830.2 | 1302 | tcctaatcaacctcaagatttg | 193751 | 587043 | see also 1836 line | | |
| 1841 | PTPN4 | NM_002830.2 | 1401 | agcacgtaccttagaacttctag | 193758 | 587049 | see also 1836 line | | |
| 1842 | PTPN4 | NM_002830.2 | 1412 | tagaactctatggagttgaattc | 193759 | 587050 | see also 1836 line | | |
| 1843 | PTPN4 | NM_002830.2 | 2574 | tcgacctaatgctgtatatgatg | 193808 | 587098 | see also 1836 line | | |
| 1844 | PTPN4 | NM_002830.2 | 2642 | ctgagaaagcccactagatagt | 193811 | 587100 | see also 1836 line | | |
| 1845 | PTPN4 | NM_002830.2 | 2644 | gagaaagcccccactagatagtgt | 193812 | 587101 | see also 1836 line | | |
| 1846 | PTPN4 | NM_002830.2 | 3035 | aagttgaacgtggcagagttaaa | 193822 | 587114 | see also 1836 line | | |
| 1847 | PTPN4 | NM_002830.2 | 3038 | ttgaacgtggcagagttaaatgt | 193823 | 587115 | see also 1836 line | | |
| 1848 | PTPN4 | NM_002830.2 | 3371 | cagccatgtgtcattgaatgc | 193831 | 587128 | see also 1836 line | | |
| 1849 | PTPN4 | NM_002830.2 | 3451 | atgatccaaacacctagtcaata | 193839 | 587130 | see also 1836 line | | |
| 1850 | PTPN6 | NM_002831.3 | 991 | aagaaccgctacaagaacattct | 193861 | 586487 | phosphatase、 apoptosis s、 signal_transduction 、gpcr | | |
| 1851 | PTPN9 | NM_002833.2 | 1213 | gggttggtacgtcaaaattgatc | 193899 | 588247 | phosphatase | gpcr | transporter |
| 1852 | PTPN9 | NM_002833.2 | 1744 | tggccagtactggccttagaaa | 193909 | 588254 | see also 1851 line | | |
| 1853 | PTPN9 | NM_002833.2 | 1745 | ggccagtactggcctttagaaaa | 193910 | 588255 | see also 1851 line | | |
| 1854 | PTPN11 | NM_002834.3 | 381 | atgacatcgcggagatgtttca | 193079 | 539314 | - | | |
| 1855 | PTPN12 | NM_002835.2 | 134 | aagaagattgtctaccaaatata | 193138 | 586570 | phosphatase | prenylated protein tyrosine phosphatase | colon cancer, arthritis |
| 1856 | PTPN12 | NM_002835.2 | 387 | atgatatgggagtataatgtgt | 193151 | 586582 | see also 1855 line | | |
| 1857 | PTPN12 | NM_002835.2 | 658 | tggacatgataagcttaatgagg | 193164 | 586600 | see also 1855 line | | |
| 1858 | PTPRA | NM_002836.2 | 1179 | atcgtgtttattatcatagtttt | 193928 | 587241 | phosphatase | - | |

159

Figure 46

| 1859 | PTPRA | NM_002836.2 | 3073 | cagattatgccaacttcaagtaa | 193951 | 587266 | see also 1858 line | | |
| 1860 | PTPRB | NM_002837.2 | 5120 | ctgactccaactaccttctatcc | 194083 | 587401 | phosphatase | transmembrane receptor protein tyrosine phosphatase | - |
| 1861 | PTPRB | NM_002837.2 | 5687 | ctgtcagggactacatcaacaga | 194096 | 587417 | see also 1860 line | | |
| 1862 | PTPRG | NM_002841.2 | 1331 | aagtcagtccgagggacaattct | 159207 | 559408 | phosphatase、signal_transduction、kinase_related | zinc binding | - |
| 1863 | PTPRG | NM_002841.2 | 1350 | ttctgcactggatcctattatcc | 159208 | 559410 | see also 1862 line | | |
| 1864 | PTPRG | NM_002841.2 | 3001 | tggagagggtgtaacaaaataaa | 159238 | 559435 | see also 1862 line | | |
| 1865 | PTPRG | NM_002841.2 | 3221 | aacacatcggtgagctctattct | 159242 | 559441 | see also 1862 line | | |
| 1866 | PTPRG | NM_002841.2 | 4752 | tcccaccattgttcatgatgagt | 159283 | 559487 | see also 1862 line | | |
| 1867 | PTPRG | NM_002841.2 | 4863 | tgcaaaaatgatcaatcttatga | 159285 | 559490 | see also 1862 line | | |
| 1868 | PTPRJ | NM_002843.2 | 2807 | cagatggatcccctaatattaca | 194489 | 587599 | phosphatase、signal_transduction、kinase_related | transmembrane receptor protein tyrosine phosphatase | - |
| 1869 | PTPRJ | NM_002843.2 | 2835 | cagtcacaattcagtaaaggtca | 194490 | 587603 | see also 1868 line | | |
| 1870 | PTPRK | NM_002844.2 | 882 | gagatgctgtgcataacaagtta | 194546 | 587637 | receptor_acitivity、phosphatase | transmembrane receptor protein tyrosine phosphatase | - |
| 1871 | PTPRK | NM_002844.2 | 1495 | cactatctgctaccattacttcc | 194570 | 587651 | see also 1870 line | | |
| 1872 | PTPRK | NM_002844.2 | 3948 | ctgctttcatcgtcacacaatac | 194629 | 587700 | see also 1870 line | | |
| 1873 | PTPRK | NM_002844.2 | 3949 | tgctttcatcgtcacacaatacc | 194630 | 587701 | see also 1870 line | | |
| 1874 | PTPRK | NM_002844.2 | 4112 | atccaagtggaatgtatgtcttg | 194640 | 587707 | see also 1870 line | | |
| 1875 | PTPRM | NM_002845.2 | 1221 | aactcgttgccacagttataatc | 194696 | 587763 | receptor_acitivity、phosphatase | transmembrane receptor protein tyrosine phosphatase | - |
| 1876 | PTPRM | NM_002845.2 | 1224 | tcgttgccacagttataatctca | 194697 | 587764 | see also 1875 line | | |
| 1877 | PTPRM | NM_002845.2 | 3092 | tactggcagaatatgtgataaga | 194743 | 587813 | see also 1875 line | | |
| 1878 | PTPRM | NM_002845.2 | 3833 | atcactgcacatccgtagttatg | 194758 | 587823 | see also 1875 line | | |
| 1879 | PTPRM | NM_002845.2 | 3837 | ctgcacatccgtagttatgctaa | 194759 | 587824 | see also 1875 line | | |
| 1880 | PTPRN2 | NM_002847.2 | 2947 | aagatggccaaaggtgctaaaga | 194793 | 587890 | receptor_acitivity、phosphatase | - | diabetes |
| 1881 | PTPRN2 | NM_002847.2 | 2957 | aaggtgctaaagagattgatatc | 194794 | 587891 | see also 1880 line | | |

EP 1 752 536 A1

Figure 46

| 1882 | PTPRO | NM_002848.2 | 3324 | tagacacttccggatcaactatg | 194901 | 587908 | receptor_acitivity、ph osphatase | - | - |
|---|---|---|---|---|---|---|---|---|---|
| 1883 | RAD1 | NM_002853.2 | 382 | tggccagccttgacaacgttagg | 17364 | 451751 | cell_cycle | - | - |
| 1884 | RAD1 | NM_002853.2 | 386 | cagccttgacaacgttaggaatc | 17365 | 451752 | see also 1883 line | | |
| 1885 | RAD1 | NM_002853.2 | 387 | agccttgacaacgttaggaatct | 17366 | 451753 | see also 1883 line | | |
| 1886 | RAD1 | NM_002853.2 | 657 | atgtgttaccaaggttatggtta | 17380 | 451764 | see also 1883 line | | |
| 1887 | PVRL1 | NM_002855.2 | 968 | caggccaggtggaggtcaatatc | 247111 | 539459 | receptor_acitivity | coreceptor | - |
| 1888 | PVRL1 | NM_002855.2 | 973 | caggtggaggtcaatatcacaga | 247112 | 539462 | see also 1887 line | | |
| 1889 | PVRL1 | NM_002855.2 | 1505 | acgacgggtctttcatttccaag | 247114 | 539466 | see also 1887 line | | |
| 1890 | PXF | NM_002857.1 | 79 | aagtgctcttgatgatttcgata | 309908 | 688674 | - | - | Zellweger syndrome |
| 1891 | ABCD3 | NM_002858.2 | 121 | ctcggcctgcacggtaagaaaag | 70881 | 492662 | - | ATP-binding cassette (ABC) transporter | Zellweger syndrome |
| 1892 | ABCD3 | NM_002858.2 | 425 | ctcttatctctctggttaataac | 70893 | 492681 | see also 1891 line | | |
| 1893 | ABCD3 | NM_002858.2 | 616 | aacagtgtagtcgatctgtattc | 70902 | 492695 | see also 1891 line | | |
| 1894 | ABCD3 | NM_002858.2 | 620 | gtgtagtcgatctgtattcaaat | 70903 | 492696 | see also 1891 line | | |
| 1895 | ABCD3 | NM_002858.2 | 737 | ttgtttctgggctattcctaact | 70905 | 492698 | see also 1891 line | | |
| 1896 | ABCD3 | NM_002858.2 | 917 | aactggtggaacacctacataat | 70908 | 492703 | see also 1891 line | | |
| 1897 | ABCD3 | NM_002858.2 | 920 | tggtggaacacctacataatttc | 70910 | 492704 | see also 1891 line | | |
| 1898 | ABCD3 | NM_002858.2 | 923 | tggaacacctacataatttcatt | 70911 | 492705 | see also 1891 line | | |
| 1899 | ABCD3 | NM_002858.2 | 1154 | ctgggcgtgaaatgactagattg | 70921 | 492711 | see also 1891 line | | |
| 1900 | ABCD3 | NM_002858.2 | 1155 | tgggcgtgaaatgactagattgg | 70922 | 492712 | see also 1891 line | | |
| 1901 | ABCD3 | NM_002858.2 | 1188 | tgctcggattacagaattaatgc | 70923 | 492716 | see also 1891 line | | |
| 1902 | ABCD3 | NM_002858.2 | 1336 | aacattataaagtttgatcatgt | 70927 | 492722 | see also 1891 line | | |
| 1903 | PYCS | NM_002860.2 | 1968 | gagagtggaacaggtaaaaattc | 209727 | 599005 | kinase_related | glutamate-5-semialdehyde dehydrogenase | - |
| 1904 | PCYT2 | NM_002861.1 | 393 | ctgtgttcacggcaatgacatca | 209125 | 598591 | - | ethanolamine-phosphate cytidylyltransferase | - |
| 1905 | PYGL | NM_002863.1 | 1887 | aggatatcacatggccaaaatga | 211602 | 599970 | - | transferase、transferring glycosyl groups | glycogen storage disease |
| 1906 | RAB13 | NM_002870.2 | 151 | gggcaagacttgtctgatcattc | 37245 | 465084 | signal_transduction | protein transporter | - |
| 1907 | RABIF | NM_002871.3 | 288 | aggacgtgggcaacatcaagttt | 159498 | 557514 | signal_transduction | protein transporter | - |
| 1908 | RABIF | NM_002871.3 | 343 | tggctggcattgcctagatgaca | 159500 | 557515 | see also 1907 line | | |
| 1909 | RABIF | NM_002871.3 | 346 | ctggcattgcctagatgacaaga | 159501 | 557516 | see also 1907 line | | |

EP 1 752 536 A1

Figure 46

| | | | | | | | | | |
|---|---|---|---|---|---|---|---|---|---|
| 1910 | RAD17 | NM_002873.1 | 946 | gtcgaaacctggttaaaagctca | 37289 | 465175 | cell_cycle、signal_transduction | - | - |
| 1911 | RAD17 | NM_002873.1 | 1479 | ggcaccaacaattatgatgaaat | 37317 | 465201 | see also 1910 line | | |
| 1912 | RAD17 | NM_002873.1 | 1492 | atgatgaaatttcttaatcgaat | 37318 | 465202 | see also 1910 line | | |
| 1913 | RAD17 | NM_002873.1 | 1601 | gtgatatcagaagtgcaataaac | 37321 | 465206 | see also 1910 line | | |
| 1914 | RAD23B | NM_002874.2 | 929 | tggtaactgagatcatgtcaatg | 19727 | 669846 | - | single-stranded DNA binding | - |
| 1915 | RAD23B | NM_002874.2 | 1189 | cggaatcagcctcagtttcaaca | 19731 | 669855 | see also 1914 line | | |
| 1916 | RAD23B | NM_002874.2 | 1464 | aaggttaaaggcattaggatttc | 19739 | 669860 | see also 1914 line | | |
| 1917 | RAD51 | NM_002875.2 | 541 | ggcggtcagagatcatacagatt | 17460 | 451786 | - | - | breast cancer |
| 1918 | RAD51 | NM_002875.2 | 899 | taggtatgcactgcttattgtag | 17472 | 451801 | see also 1917 line | | |
| 1919 | RAD52 | NM_002879.2 | 312 | gggtcatcgggtaattaatctgg | 37342 | 670038 | - | - | - |
| 1920 | RAD52 | NM_002879.2 | 421 | tacgtgggagtctgtgcatttgt | 37343 | 670041 | see also 1919 line | | |
| 1921 | RAF1 | NM_002880.1 | 657 | tagcaccaaagtacctactatgt | 98840 | 513140 | kinase_related、apoptosis | receptor signaling protein | - |
| 1922 | RAF1 | NM_002880.1 | 1489 | cagggaatggactatttgcatgc | 98856 | 513159 | see also 1921 line | | |
| 1923 | RALB | NM_002881.1 | 258 | ctgacgcttcagttcatgtatga | 110457 | 520205 | signal_transduction | RAS small monomeric GTPase | - |
| 1924 | RASA1 | NM_002890.1 | 1239 | ttcttgtgaggccctcagataat | 297054 | 681574 | - | - | non-small-cell lung cancer |
| 1925 | RASA1 | NM_002890.1 | 1242 | ttgtgaggccctcagataatact | 297055 | 681575 | see also 1924 line | | |
| 1926 | RASA1 | NM_002890.1 | 1729 | gccaaactgttttcagatagtag | 297079 | 681592 | see also 1924 line | | |
| 1927 | RASA1 | NM_002890.1 | 2503 | caccttgatggagcagtatatga | 297104 | 681614 | see also 1924 line | | |
| 1928 | RASA1 | NM_002890.1 | 2631 | atgaagatgtgaacactaattta | 297108 | 681620 | see also 1924 line | | |
| 1929 | RASGRF1 | NM_002891.3 | 3664 | tcccatattatccgagagattcg | 295179 | 664866 | signal_transduction | - | - |
| 1930 | RBL1 | NM_002895.2 | 1425 | agccaggatctcacatagacttt | 300754 | 539656 | cell_cycle、transcription_regulator | - | - |
| 1931 | RBL1 | NM_002895.2 | 1426 | gccaggatctcacatagactttg | 300755 | 539657 | see also 1930 line | | |
| 1932 | RBL1 | NM_002895.2 | 3100 | tggcagcccttctaagagtttga | 300804 | 539712 | see also 1930 line | | |
| 1933 | RBM4 | NM_002896.1 | 604 | ggcagacttgaccgagcaatata | 54069 | 698585 | - | - | - |
| 1934 | RBM4 | NM_002896.1 | 702 | gagcgctcgatgcctactacaag | 54071 | 698591 | see also 1933 line | | |
| 1935 | RBM4 | NM_002896.1 | 703 | agcgctcgatgcctactacaagc | 54072 | 698592 | see also 1933 line | | |
| 1936 | RBMS1 | NM_002897.2 | 885 | aaccgaatgatcactcaaacttc | 19751 | 475200 | - | - | - |
| 1937 | RBMS1 | NM_002897.2 | 1094 | aggcagcaccggaacatacatgc | 19757 | 475208 | see also 1936 line | | |
| 1938 | RBMS1 | NM_002897.2 | 1231 | atgaccattctccatataccttt | 19760 | 475210 | see also 1936 line | | |
| 1939 | RBP3 | NM_002900.1 | 1651 | atgatcgccgcaccaacatcacg | 173170 | 585091 | - | retinoid binding | - |
| 1940 | REL | NM_002908.1 | 1730 | atcttgaaaacccctcatgtaat | 10411 | 448078 | transcription_regulator、kinase_related | p65/c-Rel heterodimer | - |

EP 1 752 536 A1

Figure 46

| No. | Symbol | Accession | Pos | Sequence | ID1 | ID2 | Note | Function |
|---|---|---|---|---|---|---|---|---|
| 1941 | RENT1 | NM_002911.2 | 623 | ctgcctgcgtggtttactgtaat | 54162 | 513300 | - | helicase |
| 1942 | RENT1 | NM_002911.2 | 625 | gcctgcgtggtttactgtaatac | 54163 | 513301 | see also 1941 line | |
| 1943 | RENT1 | NM_002911.2 | 629 | gcgtggtttactgtaataccagc | 54164 | 513302 | see also 1941 line | |
| 1944 | RENT1 | NM_002911.2 | 1227 | gtgggacctgggccttaacaaga | 54173 | 513307 | see also 1941 line | |
| 1945 | RENT1 | NM_002911.2 | 1229 | gggacctgggccttaacaagaag | 54174 | 513308 | see also 1941 line | |
| 1946 | REV3L | NM_002912.1 | 831 | cagtatcgacagagcacttaatg | 37999 | 465846 | | translation release factor, codon specific |
| 1947 | REV3L | NM_002912.1 | 881 | ctcagcatgtgttcaaagtgtca | 38002 | 465847 | see also 1946 line | |
| 1948 | REV3L | NM_002912.1 | 892 | ttcaaagtgtcattagtatcagg | 38004 | 465848 | see also 1946 line | |
| 1949 | REV3L | NM_002912.1 | 1039 | cagcctcatgaagcgcatattcc | 38006 | 465855 | see also 1946 line | |
| 1950 | REV3L | NM_002912.1 | 1124 | ctgtcaagttccgaaaagcaaga | 38008 | 465860 | see also 1946 line | |
| 1951 | REV3L | NM_002912.1 | 2162 | atggaactgcagatgaaaatagt | 38025 | 465892 | see also 1946 line | |
| 1952 | REV3L | NM_002912.1 | 2269 | cacaaagatgctgctacattaga | 38027 | 465895 | see also 1946 line | |
| 1953 | REV3L | NM_002912.1 | 3461 | ctctcaaatccgaaaacgaaga | 38065 | 465941 | see also 1946 line | |
| 1954 | REV3L | NM_002912.1 | 3673 | gactccctgcaaccaaatatcc | 38070 | 465944 | see also 1946 line | |
| 1955 | REV3L | NM_002912.1 | 8547 | gagagtacctccagatttacttt | 38240 | 466103 | see also 1946 line | |
| 1956 | REV3L | NM_002912.1 | 8549 | gagtacctccagatttacttac | 38241 | 466104 | see also 1946 line | |
| 1957 | REV3L | NM_002912.1 | 9237 | aagtctaattaaacagtatgttc | 38271 | 466129 | see also 1946 line | |
| 1958 | RFC1 | NM_002913.3 | 782 | gaggcagttgcatgaagatgaag | 38321 | 465706 | - | transcription regulator |
| 1959 | RFC1 | NM_002913.3 | 1259 | acgcactaattatcaagcttatc | 38336 | 465716 | see also 1958 line | |
| 1960 | RFC1 | NM_002913.3 | 1260 | cgcactaattatcaagcttatcg | 38337 | 465717 | see also 1958 line | |
| 1961 | RFC1 | NM_002913.3 | 1413 | aagtctaattgaacgttatgg | 38344 | 465721 | see also 1958 line | |
| 1962 | RFC1 | NM_002913.3 | 2517 | ccccctccagctatgaatgaaat | 38366 | 465758 | see also 1958 line | |
| 1963 | RFC4 | NM_002916.3 | 764 | aactatgtcagtcgaataattga | 38459 | 466173 | | |
| 1964 | RFX3 | NM_002919.2 | 518 | atggagcaatccgaacaacaacg | 474 | 466210 | transcription_regulator | |
| 1965 | RFX3 | NM_002919.2 | 795 | ggcgattgagacgctgcaaaagt | 482 | 466218 | see also 1964 line | |
| 1966 | RFX3 | NM_002919.2 | 1333 | ctgccagatggtactaccttga | 490 | 466227 | see also 1964 line | |
| 1967 | RFX3 | NM_002919.2 | 1341 | tggtactacttgaggatatca | 491 | 466228 | see also 1964 line | |
| 1968 | RFX3 | NM_002919.2 | 1762 | accaaggttgccgctgtaagtgc | 506 | 466236 | see also 1964 line | |
| 1969 | RFX3 | NM_002919.2 | 1964 | gactagaaacagacttcaagatg | 509 | 466238 | see also 1964 line | |
| 1970 | RGS13 | NM_002927.3 | 470 | ctggatgggcatgtgaaacctata | 254977 | 638731 | signal_transduction | signal transduction |
| 1971 | RIT2 | NM_002930.1 | 219 | aggcgggtccagagagtacaaag | 110751 | 520337 | signal_transduction | signal transduction |
| 1972 | RNF4 | NM_002938.2 | 884 | aacggtaccacccatttatata | 10712 | 669302 | transcription_regulator | |
| 1973 | RNF4 | NM_002938.2 | 886 | cggtaccccatttatatatg | 10713 | 669303 | see also 1972 line | zinc binding |

Figure 46

| 1974 | ABCE1 | NM_002940.1 | 702 | gacacaggcaattgtatgtcagc | 71004 | 492774 | - | ribonuclease inhibitor | - |
|------|-------|-------------|-----|-------------------------|-------|--------|---|------------------------|---|
| 1975 | ROBO1 | NM_002941.2 | 901 | gagctgcccaaatccagatatga | 245878 | 707979 | receptor_acitivity | - | - |
| 1976 | ROBO1 | NM_002941.2 | 920 | atgaaatccgagatgatcatacc | 245880 | 707980 | see also 1975 line | | |
| 1977 | ROBO1 | NM_002941.2 | 1161 | agggagtcagaatctacttttct | 245890 | 707992 | see also 1975 line | | |
| 1978 | ROBO1 | NM_002941.2 | 3058 | aacaaccaactggataacaaaca | 245949 | 708048 | see also 1975 line | | |
| 1979 | ROBO1 | NM_002941.2 | 3094 | ctccctgagtcaactgtttatgg | 245952 | 708049 | see also 1975 line | | |
| 1980 | ROBO1 | NM_002941.2 | 3113 | atggtgatgtggaccttagtaac | 245953 | 708050 | see also 1975 line | | |
| 1981 | ROBO1 | NM_002941.2 | 3114 | tggtgatgtggaccttagtaaca | 245954 | 708051 | see also 1975 line | | |
| 1982 | ROBO1 | NM_002941.2 | 3153 | aaccttcaatagcccaaatctga | 245955 | 708054 | see also 1975 line | | |
| 1983 | ROBO1 | NM_002941.2 | 3179 | atgggcgttttgtcaatccatca | 245957 | 708056 | see also 1975 line | | |
| 1984 | RORA | NM_002943.2 | 560 | gtcctcgtcagaagaactgtttg | 10721 | 448119 | transcription_regulator、receptor_acitivity、signal_transduction | - | - |
| 1985 | RORA | NM_002943.2 | 1245 | tgccaaacgcattgatggattta | 10740 | 448127 | see also 1984 line | | |
| 1986 | ROS1 | NM_002944.2 | 865 | tggacctattttgggttataact | 99507 | 513822 | receptor_acitivity、kinase_related、signal_transduction | transmembrane receptor protein tyrosine kinase | CNS Tumours、melanoma |
| 1987 | ROS1 | NM_002944.2 | 2461 | gtggctgggtcactttctctact | 99556 | 513861 | see also 1986 line | | |
| 1988 | ROS1 | NM_002944.2 | 4173 | atcaatgttcttgtaatgtgact | 99609 | 513905 | see also 1986 line | | |
| 1989 | RPA1 | NM_002945.2 | 1256 | atcccagaggcctataagcttcg | 19800 | 451214 | - | single-stranded DNA binding | - |
| 1990 | RPA2 | NM_002946.3 | 532 | gagaaggctccaaccaacattgt | 19822 | 486072 | - | single-stranded DNA binding | - |
| 1991 | RPA2 | NM_002946.3 | 535 | aaggctccaaccaacattgttta | 19823 | 486073 | see also 1990 line | | |
| 1992 | RPA2 | NM_002946.3 | 536 | aggctccaaccaacattgtttac | 19824 | 486074 | see also 1990 line | | |
| 1993 | RPN2 | NM_002951.2 | 1904 | ccgtggtgtccaatacattcact | 211315 | 708864 | - | dolichyl-diphospho-oligosaccharide-protein glycosyltransferase | - |
| 1994 | RSN | NM_002956.2 | 3738 | ctcaataatcagttgttagaaat | 65621 | 486147 | - | nucleic acid binding | Hodgkin disease、Hodgkin's disease |
| 1995 | RSN | NM_002956.2 | 4073 | agcccaggagagtcagattgatt | 65633 | 486149 | see also 1994 line | | |
| 1996 | SORT1 | NM_002959.3 | 2014 | cagaatggtcgagactatgttgt | 273022 | 637914 | receptor_acitivity、gpcr | protein transporter | - |
| 1997 | SORT1 | NM_002959.3 | 2193 | aacaaatgggtaccggaaaattc | 273027 | 637916 | see also 1996 line | | |
| 1998 | S100A10 | NM_002966.1 | 357 | atgcaatgactattttgtagtac | 152361 | 553385 | - | receptor binding | - |
| 1999 | SALL1 | NM_002968.1 | 14 | agcaagcgaagcctcaacatttc | 38807 | 466648 | transcription_regulator | transcription factor | Townes-Brocks syndrome |
| 2000 | SALL1 | NM_002968.1 | 19 | gcgaagcctcaacatttccaatc | 38808 | 466649 | see also 1999 line | | |

EP 1 752 536 A1

Figure 46

| No | Gene | Accession | Pos | Sequence | ID1 | ID2 | Annotation | Function | Disease |
|---|---|---|---|---|---|---|---|---|---|
| 2001 | SALL1 | NM_002968.1 | 325 | gactgcaggacctttcagaaca | 38814 | 466656 | see also 1999 line | | |
| 2002 | SALL1 | NM_002968.1 | 500 | caggtacctcagcgatcacaac | 38815 | 466657 | see also 1999 line | | |
| 2003 | SALL1 | NM_002968.1 | 3039 | gagtgccttggacattcactata | 38846 | 466693 | see also 1999 line | | |
| 2004 | SALL1 | NM_002968.1 | 3593 | ctgtggatggccccatgacatt | 38855 | 466701 | see also 1999 line | | |
| 2005 | SCA2 | NM_002973.1 | 376 | tccgcctcagactgttttggtag | 46703 | 471317 | - | | spinocerebellar ataxia |
| 2006 | SCA2 | NM_002973.1 | 377 | cgcctcgactgtttggtagc | 46704 | 471318 | see also 2005 line | | |
| 2007 | SCA2 | NM_002973.1 | 2939 | ctcagccaaagccttctactacc | 46746 | 471359 | see also 2005 line | | |
| 2008 | SCA2 | NM_002973.1 | 3096 | accttatcaccaatacctatga | 46753 | 471362 | see also 2005 line | | |
| 2009 | SCA2 | NM_002973.1 | 3469 | atgtatgcagtcccaaattacc | 46765 | 471369 | see also 2005 line | | |
| 2010 | SCA2 | NM_002973.1 | 3477 | atgtcccaaattaccatacaaca | 46766 | 471371 | see also 2005 line | | |
| 2011 | SCA2 | NM_002973.1 | 3479 | gtcccaaattaccatacaacaag | 46767 | 471372 | see also 2005 line | | |
| 2012 | SCA2 | NM_002973.1 | 3944 | cccatgcgccaatgatgctaatg | 46772 | 471379 | see also 2005 line | | |
| 2013 | SCA2 | NM_002973.1 | 3947 | atgcgccaatgatgctaatgacg | 46773 | 471380 | see also 2005 line | | |
| 2014 | SDC4 | NM_002999.2 | 585 | tgggcaagaaaccatctacaag | 121076 | 525392 | - | molecular_function unknown | |
| 2015 | SEC14L1 | NM_003003.1 | 1681 | ttcctcatttatgcaggaaatga | 180957 | 656738 | - | transporter | |
| 2016 | MAP2K4 | NM_003010.2 | 1196 | aggtcgcatgctatgtttgtaaa | 88977 | 506797 | kinase_related, signal transduction | protein tyrosine kinase | breast cancer, pancreatic cancer |
| 2017 | MAP2K4 | NM_003010.2 | 1198 | gtcgcatgctatgtttgtaaaat | 88978 | 506798 | see also 2016 line | | |
| 2018 | SFRP4 | NM_003014.2 | 560 | gcgagccctcatgaagatgtac | 246643 | 633820 | receptor_acitivity, sig nal transduction | | |
| 2019 | SFRP4 | NM_003014.2 | 817 | aactacagctatgttattcatgc | 246654 | 633827 | see also 2018 line | | |
| 2020 | SFRP5 | NM_003015.2 | 1025 | taccgctgggacaagaagaataa | 246667 | 633846 | receptor_acitivity, apo ptosis, signal_transdu ction | | |
| 2021 | SH3BGRL | NM_003022.1 | 82 | gtgatccgtgtatatattgcatc | 252794 | 709858 | | SH3/SH2 adaptor protein | |
| 2022 | SH3BGRL | NM_003022.1 | 162 | agccaacaaaataggatttgaag | 252799 | 709863 | see also 2021 line | | |
| 2023 | SH3BGRL | NM_003022.1 | 212 | atcggaagtggatgagagaaaat | 252800 | 709864 | see also 2021 line | | |
| 2024 | ITSN1 | NM_003024.1 | 878 | cagctggcttcaatatggaatct | 146829 | 535968 | - | molecular_function unknown | |
| 2025 | ITSN1 | NM_003024.1 | 881 | ctggcttcaaatatggaatcttc | 146830 | 535969 | see also 2024 line | | |
| 2026 | ITSN1 | NM_003024.1 | 996 | ctgtcctgcctccagaatacatt | 146832 | 535973 | see also 2024 line | | |
| 2027 | ITSN1 | NM_003024.1 | 3155 | gagcaaggagatttaacctttca | 146872 | 535992 | see also 2024 line | | |
| 2028 | ITSN1 | NM_003024.1 | 3247 | cggagtcttccctctaactatg | 146875 | 535995 | see also 2024 line | | |
| 2029 | SH3GL3 | NM_003027.2 | 1380 | ctgctgctggtgtctctatgact | 317866 | 540441 | signal transduction | | |
| 2030 | SH3GL3 | NM_003027.2 | 1444 | gacatcattacttaaccaatca | 317866 | 540443 | see also 2029 line | | |

Figure 46

| | | | | | | | | | |
|---|---|---|---|---|---|---|---|---|---|
| 2031 | SHC1 | NM_003029.3 | 197 | gccacgggagctttgtcaataag | 253028 | 540449 | kinase_related、recept or_acitivity | - | - |
| 2032 | SHOX2 | NM_003030.2 | 230 | gaggcgaagtcggaccaatttca | 11199 | 448383 | transcription_regulator | - | - |
| 2033 | SHOX2 | NM_003030.2 | 475 | atgtcaacgtaggtgctttaagg | 11205 | 448391 | see also 2032 line | | |
| 2034 | SIL | NM_003035.1 | 561 | accatccgacttgcttatcgtca | 309387 | 698744 | - | - | - |
| 2035 | SIL | NM_003035.1 | 1615 | cagtgagtcagaagatttctaag | 309421 | 698776 | see also 2034 line | | |
| 2036 | SLC5A2 | NM_003041.1 | 698 | ttcgggtctcttcgacaaatacc | 256190 | 656944 | - | sugar porter | renal glycosuria |
| 2037 | SLC5A2 | NM_003041.1 | 701 | gggtctcttcgacaaatacctgg | 256191 | 656945 | see also 2036 line | | |
| 2038 | SLC6A6 | NM_003043.1 | 1013 | ctgaacagtggtaccagttttgt | 255693 | 640155 | - | taurine:sodium symporter | - |
| 2039 | SLC6A6 | NM_003043.1 | 1032 | ttgtgtctggcttcgcaattttt | 255694 | 640156 | see also 2038 line | | |
| 2040 | SLC6A6 | NM_003043.1 | 1038 | ctggcttcgcaatttttccatc | 255696 | 640157 | see also 2038 line | | |
| 2041 | SLC6A6 | NM_003043.1 | 1280 | ttcctaaggaagggttatcgtcg | 255704 | 640162 | see also 2038 line | | |
| 2042 | SLC6A6 | NM_003043.1 | 1291 | gggttatcgtcgggaaatcttca | 255705 | 640164 | see also 2038 line | | |
| 2043 | SLC6A6 | NM_003043.1 | 1360 | gacggagggtggcatgtatgtgt | 255706 | 640166 | see also 2038 line | | |
| 2044 | SLC6A6 | NM_003043.1 | 1427 | tgggttgcattctttgaatgttt | 255707 | 640167 | see also 2038 line | | |
| 2045 | SLC6A6 | NM_003043.1 | 1596 | acgtacccctgacctacaacaaa | 255709 | 640172 | see also 2038 line | | |
| 2046 | SLC6A12 | NM_003044.2 | 694 | gggccaatggaccaacaagatgg | 255933 | 639937 | - | betaine/GABA:sodiu m symporter | - |
| 2047 | SLC18A1 | NM_003053.1 | 1334 | ttggtgtgttggccaacaagatg | 271976 | 656836 | - | monoamine transporter | - |
| 2048 | SLC18A2 | NM_003054.1 | 523 | aacagaattggctatccaattcc | 271986 | 775219 | - | monoamine transporter | - |
| 2049 | SLC18A2 | NM_003054.1 | 1376 | ctgctggtggtgctattgcaaag | 272002 | 775232 | see also 2048 line | | |
| 2050 | SLC18A2 | NM_003054.1 | 1377 | tgctggtggtgctattgcaagg | 272003 | 775233 | see also 2048 line | | |
| 2051 | SLC18A2 | NM_003054.1 | 1420 | atgacaattattgggataattga | 272005 | 775235 | see also 2048 line | | |
| 2052 | SLC18A2 | NM_003054.1 | 1422 | gacaattattgggataattgata | 272006 | 775236 | see also 2048 line | | |
| 2053 | SMARCA1 | NM_003069.2 | 833 | tggatgaatgaatttaaacgatg | 39639 | 467217 | transcription_regulator | - | - |
| 2054 | SMARCA1 | NM_003069.2 | 958 | ttcttatgagatggtaattaaag | 39644 | 467224 | see also 2053 line | | |
| 2055 | SMARCA1 | NM_003069.2 | 1359 | tgcaacgagaatggtatacaaaa | 39654 | 467236 | see also 2053 line | | |
| 2056 | SMARCA1 | NM_003069.2 | 1362 | aacgagaatggtatacaaaaatc | 39655 | 467237 | see also 2053 line | | |
| 2057 | SMARCA1 | NM_003069.2 | 2395 | gcgtgtcagcgagccaaagattc | 39687 | 467269 | see also 2053 line | | |
| 2058 | SMARCA1 | NM_003069.2 | 2771 | gaggaggtcatggagtattcagc | 39693 | 467277 | see also 2053 line | | |
| 2059 | SMARCA1 | NM_003069.2 | 3209 | aagaaacgggcaactaaaactcc | 39702 | 467296 | see also 2053 line | | |
| 2060 | SMARCB1 | NM_003073.2 | 263 | tccgaggttctctgtacaagaga | 300021 | 669410 | cell_cycle、transcripti on_regulator | translation regulator | chronic myeloid leukemia、malignant rhabdoid tumor、rhabdoid tumor、medulloblastoma |

Figure 46

| | | | | | | | | | |
|---|---|---|---|---|---|---|---|---|---|
| 2061 | SMARCB1 | NM_003073.2 | 267 | aggttctctgtacaagagatacc | 300022 | 669412 | see also 2060 line | | |
| 2062 | SMARCC1 | NM_003074.2 | 325 | cggatgctcctaccaataaaaca | 18996 | 451236 | transcription_regulator | - | - |
| 2063 | SMARCC2 | NM_003075.2 | 258 | ctgccgatcaaatgtttcctaga | 19069 | 812072 | transcription_regulator | - | - |
| 2064 | SMARCC2 | NM_003075.2 | 310 | ttcttgcagctgcctacaaattc | 19071 | 812073 | see also 2063 line | | |
| 2065 | SMARCC2 | NM_003075.2 | 316 | cagctgcctacaaattcaagagt | 19072 | 812075 | see also 2063 line | | |
| 2066 | SMARCC2 | NM_003075.2 | 1757 | tgggctgcgcacagacatgtaca | 19097 | 812094 | see also 2063 line | | |
| 2067 | SMARCD1 | NM_003076.3 | 475 | cagcagcaggcggtccaaaatcg | 284397 | 669676 | - | - | - |
| 2068 | SMARCD1 | NM_003076.3 | 478 | cagcaggcggtccaaaatcgaaa | 284398 | 669677 | see also 2067 line | | |
| 2069 | SMARCD1 | NM_003076.3 | 479 | agcaggcggtccaaaatcgaaac | 284399 | 669678 | see also 2067 line | | |
| 2070 | SMARCD1 | NM_003076.3 | 1608 | ggctgtgtgccgatacttctact | 284419 | 669699 | see also 2067 line | | |
| 2071 | SMARCD1 | NM_003076.3 | 1610 | ctgtgtgccgatacttctactcc | 284420 | 669700 | see also 2067 line | | |
| 2072 | SMARCD2 | NM_003077.2 | 1678 | agcgccgagacctcaagatcatc | 112404 | 743776 | transcription_regulator | - | - |
| 2073 | SMARCD2 | NM_003077.2 | 1679 | gcgccgagacctcaagatcatca | 112405 | 743777 | see also 2072 line | | |
| 2074 | SMARCD3 | NM_003078.2 | 1278 | cagtgctctggacagtaagatcc | 112415 | 605207 | transcription_regulator | - | - |
| 2075 | SNAPC1 | NM_003082.2 | 521 | aacttatcacttctgatgtatta | 300907 | 801396 | transcription_regulator | - | - |
| 2076 | SNAPC3 | NM_003084.1 | 878 | gactgctagaatggaagatttca | 40288 | 741106 | transcription_regulator | DNA binding | - |
| 2077 | SNAPC3 | NM_003084.1 | 880 | ctgctagaatggaagatttcacc | 40289 | 741107 | see also 2076 line | | |
| 2078 | SNAPC3 | NM_003084.1 | 1097 | cagatgggtgacgaacaatgaca | 40295 | 741114 | see also 2076 line | | |
| 2079 | SNAPC4 | NM_003086.1 | 1159 | gactgggagaagatttccaatat | 11718 | 454021 | transcription_regulator | - | - |
| 2080 | SNRPA1 | NM_003090.1 | 551 | acggggtgcacagcttgcaaagg | 54842 | 475624 | - | RNA binding | - |
| 2081 | SNX2 | NM_003100.2 | 912 | ctgccgacgctgtcaacaaaatg | 270695 | 652519 | - | protein transporter | - |
| 2082 | SNX2 | NM_003100.2 | 913 | tgccgacgctgtcaacaaaatga | 270696 | 652520 | see also 2081 line | | |
| 2083 | SNX2 | NM_003100.2 | 1029 | ttgaagccttggtctgtcataga | 270697 | 652525 | see also 2081 line | | |
| 2084 | SNX2 | NM_003100.2 | 1033 | agccttggtctgtcatagaaaag | 270699 | 652526 | see also 2081 line | | |
| 2085 | SNX2 | NM_003100.2 | 1163 | gagaagatagaccagttacatca | 270704 | 652529 | see also 2081 line | | |
| 2086 | SON | NM_003103.5 | 2907 | tagcatctagacgttctatgatg | 40387 | 467661 | apoptosis | - | - |
| 2087 | SON | NM_003103.5 | 2911 | atctagacgttctatgatgatgt | 40389 | 467662 | see also 2086 line | | |
| 2088 | SON | NM_003103.5 | 6086 | ttggataaggctcaattacttga | 40460 | 467744 | see also 2086 line | | |
| 2089 | SON | NM_003103.5 | 6345 | atcatcatccctttaaactcagt | 40468 | 467751 | see also 2086 line | | |
| 2090 | SON | NM_003103.5 | 6466 | tggatctcaacatcggaaaaaag | 40471 | 467756 | see also 2086 line | | |

Figure 46

| | | | | | | | | | |
|---|---|---|---|---|---|---|---|---|---|
| 2091 | SOX11 | NM_003108.3 | 184 | gacggcgtcgggccacatcaagc | 40575 | 467901 | transcription_regulator | DNA binding | - |
| 2092 | SP2 | NM_003110.3 | 1024 | aaccccactgtctaagactaaca | 40751 | 741963 | - | translation regulator | - |
| 2093 | SP4 | NM_003112.2 | 475 | tggaaacgcttggcaacttgttg | 40785 | 468091 | transcription_regulator | translation regulator | - |
| 2094 | SP4 | NM_003112.2 | 922 | tggtactcaggctcaagttgtaa | 40796 | 468103 | see also 2093 line | | |
| 2095 | SP4 | NM_003112.2 | 1729 | tggtgccccaataactttgaata | 40811 | 468126 | see also 2093 line | | |
| 2096 | SP4 | NM_003112.2 | 1731 | gtgccccaataactttgaatact | 40812 | 468127 | see also 2093 line | | |
| 2097 | SPARC | NM_003118.1 | 365 | cccccattggcgagtttgagaag | 127203 | 554215 | - | collagen binding | hepatocellular carcinoma、 esophageal cancer、 ovarian cancer、 prostate cancer |
| 2098 | SPARC | NM_003118.1 | 366 | ccccattggcgagtttgagaagg | 127204 | 554216 | see also 2097 line | | |
| 2099 | SPTA1 | NM_003126.1 | 6857 | acgctctgatccttgatatcaaa | 114806 | 524051 | - | structural constituent of cytoskeleton | pyropoikilocytosis、 spherocytosis、 elliptocytosis |
| 2100 | SPTA1 | NM_003126.1 | 6858 | cgctctgatccttgatatcaaat | 114807 | 524052 | see also 2099 line | | |
| 2101 | SPTBN1 | NM_003128.1 | 875 | gggtaccccaatgtcaacattca | 119261 | 524153 | - | - | - |
| 2102 | SPTBN1 | NM_003128.1 | 879 | accccaatgtcaacattcacaat | 119262 | 524154 | see also 2101 line | | |
| 2103 | SPTBN1 | NM_003128.1 | 881 | cccaatgtcaacattcacaattt | 119264 | 524155 | see also 2101 line | | |
| 2104 | SPTBN1 | NM_003128.1 | 1807 | cacagcgaggaaggacaatgtca | 119274 | 524171 | see also 2101 line | | |
| 2105 | SPTBN1 | NM_003128.1 | 1809 | cagcgaggaaggacaatgtcatc | 119275 | 524172 | see also 2101 line | | |
| 2106 | SPTBN1 | NM_003128.1 | 1810 | agcgaggaaggacaatgtcatcc | 119276 | 524173 | see also 2101 line | | |
| 2107 | SPTBN1 | NM_003128.1 | 1968 | ctcaagactatggcaaacactta | 119278 | 524179 | see also 2101 line | | |
| 2108 | SPTBN1 | NM_003128.1 | 3188 | taccacctcgagtgcaatgaaac | 119296 | 524193 | see also 2101 line | | |
| 2109 | SPTBN1 | NM_003128.1 | 3189 | accacctcgagtgcaatgaaacc | 119297 | 524194 | see also 2101 line | | |
| 2110 | SPTBN1 | NM_003128.1 | 3194 | ctcgagtgcaatgaaaccaaatc | 119298 | 524195 | see also 2101 line | | |
| 2111 | SPTBN1 | NM_003128.1 | 6933 | acgagtgggaggcccacaataag | 119345 | 790413 | see also 2101 line | | |
| 2112 | SPTBN1 | NM_003128.1 | 7093 | ctgcgaagtggcccttgattaca | 119351 | 790419 | see also 2101 line | | |
| 2113 | SRF | NM_003131.1 | 847 | gaggaagacgggcatcatgaaga | 11923 | 448692 | transcription_regulator、 signal_transduction | translation regulator | - |
| 2114 | SRF | NM_003131.1 | 922 | cagtgagacaggccatgtgtata | 11924 | 448693 | see also 2113 line | | |
| 2115 | SRP19 | NM_003135.1 | 107 | cggccgaccaggacaggtttatt | 49473 | 472570 | - | 7S RNA binding | - |
| 2116 | SRP54 | NM_003136.2 | 1043 | tggtacaggggaacatatagatg | 54866 | 703498 | - | protein signal sequence binding | - |
| 2117 | SRPK1 | NM_003137.2 | 204 | aggttatcatcttgtgaaaattg | 101236 | 515319 | kinase_related、 cell_cycle | - | - |
| 2118 | SRPK1 | NM_003137.2 | 436 | atggttgttcaactactagatga | 101247 | 515325 | see also 2117 line | | |
| 2119 | SRPK1 | NM_003137.2 | 835 | aagcgccaggcagaattactaga | 101250 | 515335 | see also 2117 line | | |

168

Figure 46

| | | | | | | | | | |
|---|---|---|---|---|---|---|---|---|---|
| 2120 | SRPR | NM_003139.2 | 1085 | tgcgtgatcatctcattgctaag | 110919 | 518061 | receptor_acitivity | signal recognition particle receptor | - |
| 2121 | SRPR | NM_003139.2 | 1086 | gcgtgatcatctcattgctaaga | 110920 | 518062 | see also 2120 line | | |
| 2122 | SRPR | NM_003139.2 | 1397 | tcctcattgctgcctgtgataca | 110928 | 518064 | see also 2120 line | | |
| 2123 | SRPR | NM_003139.2 | 1649 | ctgccctggccaaactcattact | 110931 | 518068 | see also 2120 line | | |
| 2124 | SSB | NM_003142.2 | 158 | ctgtcatcaaattgagtattatt | 48014 | 475688 | transcription_regulator | tRNA binding | - |
| 2125 | SSR1 | NM_003144.2 | 644 | aagatttgaacggcaatgtattc | 153226 | 720448 | - | signal sequence receptor | - |
| 2126 | SSR2 | NM_003145.2 | 451 | tggctcagcgggagtttgacagg | 177247 | 716472 | - | signal sequence receptor | - |
| 2127 | SSRP1 | NM_003146.2 | 945 | tggcaagacctttgactacaaga | 41220 | 468382 | transcription_regulator | DNA binding | - |
| 2128 | STAT4 | NM_003151.2 | 2112 | gtgagattccactctgtagaacc | 12196 | 448944 | transcription_regulator | translation regulator | - |
| 2129 | STIM1 | NM_003156.2 | 840 | agggaagacctcaattaccatga | 136457 | 540679 | cell_cycle | protein binding | - |
| 2130 | STIM1 | NM_003156.2 | 1207 | tgctggtggtgtctatcgttatt | 136460 | 540683 | see also 2129 line | | |
| 2131 | STIM1 | NM_003156.2 | 1209 | ctggtggtgtctatcgttattgg | 136461 | 540684 | see also 2129 line | | |
| 2132 | STIM1 | NM_003156.2 | 1212 | gtggtgtctatcgttattggtgt | 136462 | 540685 | see also 2129 line | | |
| 2133 | STIM1 | NM_003156.2 | 1241 | ctgctggtttgcctatatccaga | 136463 | 540687 | see also 2129 line | | |
| 2134 | RPS6KB1 | NM_003161.1 | 317 | ttggtaaagggggctatggaaag | 99950 | 488697 | kinase_related、signal_transduction | protein serine/threonine kinase | - |
| 2135 | STRN | NM_003162.1 | 1050 | gaggcccaataggtcaaaactac | 122837 | 527387 | - | calmodulin binding | - |
| 2136 | STRN | NM_003162.1 | 1051 | aggcccaataggtcaaaactaca | 122838 | 527388 | see also 2135 line | | |
| 2137 | STRN | NM_003162.1 | 2179 | ggcagtcatgactgttcaatacg | 122879 | 527422 | see also 2135 line | | |
| 2138 | STX5A | NM_003164.2 | 492 | aagcgcaagtccctctttgatga | 270976 | 652750 | - | protein transporter | - |
| 2139 | STX5A | NM_003164.2 | 561 | atcaatagcctcaacaaacaaat | 270980 | 652755 | see also 2138 line | | |
| 2140 | STX5A | NM_003164.2 | 683 | ggcttctatgtccaatgacttca | 270981 | 652758 | see also 2138 line | | |
| 2141 | SUPT5H | NM_003169.2 | 614 | aactgggcgagtattacatgaag | 12289 | 658125 | transcription_regulator、cell_cycle | transcription elongation factor | - |
| 2142 | SUPT6H | NM_003170.2 | 739 | ttcggtgtggactttgactatga | 12321 | 475733 | - | - | - |
| 2143 | SUPT6H | NM_003170.2 | 1325 | acctaacacggctgtttgagaag | 12331 | 475742 | see also 2142 line | | |
| 2144 | SUPT6H | NM_003170.2 | 3161 | gtcccaaagtcttcatgaattgt | 12362 | 475771 | see also 2142 line | | |
| 2145 | SUPT6H | NM_003170.2 | 4046 | agcagcagcggaccacatacatc | 12381 | 475799 | see also 2142 line | | |
| 2146 | SUPT6H | NM_003170.2 | 4284 | gtggatcaacagtgaggaattcg | 12385 | 475811 | see also 2142 line | | |
| 2147 | SUPT6H | NM_003170.2 | 4421 | aggagctgctcatcaaaactaag | 12389 | 475817 | see also 2142 line | | |
| 2148 | SUPT6H | NM_003170.2 | 4527 | gggtaaacccaggatagaatatg | 12391 | 475819 | see also 2142 line | | |

Figure 46

| | | | | | | | | | |
|---|---|---|---|---|---|---|---|---|---|
| 2149 | SUPV3L1 | NM_003171.2 | 539 | ttcttgagacatgccaaacaaat | 54973 | 486468 | - | - | - |
| 2150 | SUPV3L1 | NM_003171.2 | 582 | aggatgatctacgtaaaatcagt | 54975 | 486471 | see also 2149 line | | |
| 2151 | SUPV3L1 | NM_003171.2 | 1352 | ttgagcataaggagaattatttt | 55005 | 486490 | see also 2149 line | | |
| 2152 | SUPV3L1 | NM_003171.2 | 1640 | ctgtccaatctcattgatatttt | 55014 | 486496 | see also 2149 line | | |
| 2153 | SUPV3L1 | NM_003171.2 | 1697 | gtctgcaatatggatgattttaa | 55018 | 486501 | see also 2149 line | | |
| 2154 | SUPV3L1 | NM_003171.2 | 1972 | gtggctaagctaccgatttatgg | 55028 | 486512 | see also 2149 line | | |
| 2155 | SVIL | NM_003174.2 | 1881 | tgcaagtgccactgactataagt | 119440 | 524510 | - | - | - |
| 2156 | SYN2 | NM_003178.2 | 1540 | cagtatattctcgactgtaatgg | 314163 | 678858 | - | - | schizophrenia |
| 2157 | TAC1 | NM_003182.1 | 464 | atgggcaaaagagctttaaattc | 169655 | 567583 | signal_transduction | - | - |
| 2158 | TBCC | NM_003192.1 | 940 | ctggtttagataggagcaaaaat | 299101 | 801308 | - | co-chaperonin | - |
| 2159 | TBCC | NM_003192.1 | 941 | tggtttagataggagcaaaaata | 299102 | 801309 | see also 2158 line | | |
| 2160 | TCEB3 | NM_003198.1 | 1862 | ttgtcaccgagactttaaggaag | 136668 | 669445 | transcription_regulator | translation elongation factor | - |
| 2161 | TCF4 | NM_003199.1 | 470 | ctctctccacctttgtcaattc | 41821 | 468865 | transcription_regulator | translation regulator | - |
| 2162 | TCF4 | NM_003199.1 | 483 | ttgtcaattccagaatacaaagt | 41822 | 468866 | see also 2161 line | | |
| 2163 | TCF4 | NM_003199.1 | 514 | aaggggctcatactcatcttatg | 41823 | 468867 | see also 2161 line | | |
| 2164 | TCF4 | NM_003199.1 | 515 | aggggctcatactcatcttatgg | 41824 | 468868 | see also 2161 line | | |
| 2165 | TCF4 | NM_003199.1 | 786 | gggactcgccaggctatccttcc | 41829 | 468875 | see also 2161 line | | |
| 2166 | TCF4 | NM_003199.1 | 978 | atgaacgtttgagctatccatca | 41831 | 468879 | see also 2161 line | | |
| 2167 | NFE2L1 | NM_003204.1 | 1121 | atcctttggcgacaggatattga | 7710 | 462713 | transcription_regulator | translation regulator | - |
| 2168 | NFE2L1 | NM_003204.1 | 1126 | ttggcgacaggatattgatctgg | 7711 | 462714 | see also 2167 line | | |
| 2169 | NFE2L1 | NM_003204.1 | 1152 | ctgggcgtgaggtttttgactat | 7713 | 462716 | see also 2167 line | | |
| 2170 | NFE2L1 | NM_003204.1 | 1153 | tgggcgtgaggtttttgactata | 7714 | 462717 | see also 2167 line | | |
| 2171 | TCF12 | NM_003205.2 | 422 | atgattcatctagaggtttaca | 41655 | 468750 | transcription_regulator | RNA polymerase II transcription factor | extraskeletal myxoid chondrosarcoma |
| 2172 | TCF12 | NM_003205.2 | 466 | cacttgaatgacagtcgattagg | 41657 | 468751 | see also 2171 line | | |
| 2173 | TCF12 | NM_003205.2 | 469 | ttgaatgacagtcgattaggagc | 41658 | 468752 | see also 2171 line | | |
| 2174 | TCF12 | NM_003205.2 | 506 | ccccaacacctttcatgaactca | 41659 | 468753 | see also 2171 line | | |
| 2175 | TCF12 | NM_003205.2 | 871 | atgttcgctagcactttctttat | 41665 | 468762 | see also 2171 line | | |
| 2176 | TCF12 | NM_003205.2 | 1633 | gtcagtctcaatggcaatcattc | 41678 | 468783 | see also 2171 line | | |
| 2177 | TFAP2A | NM_003220.1 | 153 | ctgggcactgtaggtcaatctcc | 531 | 449365 | transcription_regulator、signal_transduction | Transcription factor AP-2 alphaB | breast cancer、ovarian cancer、melanoma |
| 2178 | TFAP2A | NM_003220.1 | 544 | atgtagaagacccgggtattaac | 532 | 449366 | see also 2177 line | | |
| 2179 | TFAP2A | NM_003220.1 | 547 | tagaagacccgggtattaacatc | 533 | 449367 | see also 2177 line | | |
| 2180 | TFAP2A | NM_003220.1 | 628 | ccgtctccgccatccctattaac | 535 | 449370 | see also 2177 line | | |

EP 1 752 536 A1

Figure 46

| | | | | | | | | | |
|---|---|---|---|---|---|---|---|---|---|
| 2181 | TFAP2A | NM_003220.1 | 632 | ctccgccatcccattaacaagg | 536 | 449371 | see also 2177 line | | |
| 2182 | TFAP2B | NM_003221.2 | 1351 | tggggaaggcccagtagtaaaa | 13117 | 449398 | transcription_regulator | translation regulator | breast cancer |
| 2183 | ARFRP1 | NM_003224.2 | 284 | aagggggatgagtctatccaaat | 108322 | 518638 | signal_transduction | small monomeric GTPase | |
| 2184 | TFRC | NM_003234.1 | 950 | tggtaaactggtccatgctaatt | 191826 | 585345 | receptor_activity | transferrin receptor | |
| 2185 | TG | NM_003235.3 | 6720 | ttccttggagttccatatgctgc | 170114 | 672661 | signal_transduction | hormone | multinodular goiter |
| 2186 | TG | NM_003235.3 | 7783 | atgccacccgggactactttatc | 170127 | 672672 | see also 2185 line | | |
| 2187 | TGM3 | NM_003245.2 | 89 | agcgcatcacacagacaagttct | 153572 | 554344 | - | protein-glutamine-gamma-glutamyltransferase | |
| 2188 | THBS1 | NM_003246.2 | 3169 | ctgtaggttatgatgagtttaat | 153750 | 554443 | - | structural molecule | colorectal cancer, colorectal cancers, mesothelioma |
| 2189 | THOP1 | NM_003249.3 | 1159 | gggacatgcgctactacatgaac | 160014 | 559690 | - | zinc binding | |
| 2190 | THRA | NM_003250.4 | 1121 | aagccttcagcgagtttaccaag | 13328 | 449537 | receptor_acitivity, transcription_regulator | thyroid hormone receptor | |
| 2191 | THRA | NM_003250.4 | 1122 | agccttcagcgagttaccaaga | 13329 | 449538 | see also 2190 line | | |
| 2192 | THRA | NM_003250.4 | 1128 | cagcgagtttaccaagatcatca | 13330 | 449539 | see also 2190 line | | |
| 2193 | THRA | NM_003250.4 | 1538 | acgtcaaccaccgcaaacacaac | 13333 | 449541 | see also 2190 line | | |
| 2194 | THRA | NM_003250.4 | 1543 | aaccaccgcaaacacacattcc | 13334 | 449542 | see also 2190 line | | |
| 2195 | TIAL1 | NM_003252.2 | 180 | aagcaatgacccatattgctttg | 41952 | 475866 | transcription_regulator, apoptosis | | |
| 2196 | TIAL1 | NM_003252.2 | 606 | cactaagcagttgagatttgaag | 41957 | 475878 | see also 2195 line | | |
| 2197 | TIAL1 | NM_003252.2 | 867 | gggtaaagaatctctgatatga | 41965 | 475887 | see also 2195 line | | |
| 2198 | TIMP2 | NM_003255.2 | 400 | acccgcaacaggcgtttgcaat | 293876 | 663642 | | metalloendopeptidase inhibitor | hepatocellular carcinoma, chondrosarcoma, bladder cancer, breast cancer, lung cancer, melanoma |
| 2199 | TIMP2 | NM_003255.2 | 402 | ccgcaacaggcgtttgcaatgc | 293877 | 663644 | see also 2198 line | | |
| 2200 | TIMP4 | NM_003256.1 | 271 | tccggtatgaaatcaaacagata | 293885 | 663671 | - | metalloendopeptidase inhibitor | |
| 2201 | TJP1 | NM_003257.2 | 598 | acgagataatcctcatttcaga | 331305 | 541229 | - | | |
| 2202 | TJP1 | NM_003257.2 | 690 | atgaccgagttgcaatggttaac | 331310 | 541235 | see also 2201 line | | |
| 2203 | TJP1 | NM_003257.2 | 1173 | atggtactgtgacagaaaatatg | 331325 | 541240 | see also 2201 line | | |
| 2204 | TJP1 | NM_003257.2 | 2885 | aaggccgatggtgctacaagtga | 331381 | 541303 | see also 2201 line | | |
| 2205 | TJP1 | NM_003257.2 | 2888 | gccgatggtgctacaagtgatga | 331382 | 541304 | see also 2201 line | | |
| 2206 | TJP1 | NM_003257.2 | 3026 | ggggcctacactgatcaagaact | 331389 | 541309 | see also 2201 line | | |
| 2207 | TJP1 | NM_003257.2 | 3745 | gtggtcatattatgatgacaaac | 331412 | 541327 | see also 2201 line | | |
| 2208 | TJP1 | NM_003257.2 | 4556 | tactttgaccgaagaagtttga | 331430 | 541346 | see also 2201 line | | |
| 2209 | ICAM5 | NM_003259.2 | 1758 | gtggccgtcacggtgaatatgg | 317988 | 682684 | - | | |
| 2210 | TLOC1 | NM_003262.2 | 654 | tggtgaaccatctaaagaagaga | 271117 | 650009 | receptor_acitivity | | |

Figure 46

| | | | | | | | | | |
|---|---|---|---|---|---|---|---|---|---|
| 2211 | TLOC1 | NM_003262.2 | 696 | tcgattcaactgtccaacaaagt | 271119 | 650012 | see also 2210 line | | |
| 2212 | TLOC1 | NM_003262.2 | 890 | gagccctaaaagtaatgaaaatg | 271129 | 650017 | see also 2210 line | | |
| 2213 | TLOC1 | NM_003262.2 | 1272 | gagagtaggtgtttattacctca | 271130 | 650024 | see also 2210 line | | |
| 2214 | TLR1 | NM_003263.2 | 651 | ttgatgccctgcctatatgcaaa | 123497 | 527927 | receptor_acitivity、kinase_related | triacylated lipoprotein binding | - |
| 2215 | NR2E1 | NM_003269.2 | 722 | cggatcaacaagccgcattttag | 8423 | 447146 | transcription_regulator、receptor_acitivity | translation regulator | - |
| 2216 | NR2E1 | NM_003269.2 | 728 | aacaagccgcattttagatatcc | 8424 | 447148 | see also 2215 line | | |
| 2217 | NR2E1 | NM_003269.2 | 1215 | gtgaatgggaccccaatgtatct | 8430 | 447157 | see also 2215 line | | |
| 2218 | TM4SF6 | NM_003270.2 | 438 | ttgttttcagacatgagattaag | 312818 | 693423 | kinase_related | - | - |
| 2219 | TNNC1 | NM_003280.1 | 325 | ctcttccgcatgtttgacaaaaa | 154066 | 554698 | - | calcium ion binding | - |
| 2220 | TNR | NM_003285.1 | 1131 | ggcagtgacggaatatgtgatct | 289224 | 678022 | - | - | - |
| 2221 | TNR | NM_003285.1 | 2060 | ctgagtatggagttggaatatct | 289241 | 678033 | see also 2220 line | | |
| 2222 | TOP1 | NM_003286.2 | 285 | agcggatttccgattgaatgatt | 42443 | 469396 | - | translation regulator | testicular cancer |
| 2223 | TOP1 | NM_003286.2 | 286 | gcggatttccgattgaatgattc | 42444 | 469397 | see also 2222 line | | |
| 2224 | TOP1 | NM_003286.2 | 674 | atgatgctgattataaacctaag | 42454 | 469408 | see also 2222 line | | |
| 2225 | TPP2 | NM_003291.1 | 177 | ccgggcatgcaggttacaactga | 183221 | 576952 | - | tripeptidyl-peptidase II | - |
| 2226 | TPP2 | NM_003291.1 | 1486 | aggctgacaatatagaagtattt | 183265 | 576987 | see also 2225 line | | |
| 2227 | TPR | NM_003292.1 | 3459 | aagcacagctttaagtaatgagc | 302448 | 690007 | - | - | papillary Thyroid Carcinomas |
| 2228 | TPR | NM_003292.1 | 6286 | gggactgatccaggtacagaaac | 302501 | 690080 | see also 2227 line | | |
| 2229 | NR2C1 | NM_003297.1 | 1635 | aaggcttatgtggaattccaaga | 8358 | 447139 | transcription_regulator、receptor_acitivity | steroid hormone receptor | - |
| 2230 | TRA1 | NM_003299.1 | 2325 | aagaatgcttcgccctcagtttga | 102866 | 516722 | - | heat shock protein | - |
| 2231 | TRAF3 | NM_003300.2 | 917 | aagcgctatggctgcgtttttca | 160069 | 559721 | apoptosis、signal_transduction | - | - |
| 2232 | TRHR | NM_003301.1 | 578 | tactactcacctatttacctaat | 239032 | 629222 | gpcr、receptor_acitivity、signal_transduction | thyrotropin-releasing hormone receptor | - |
| 2233 | TRPC1 | NM_003304.3 | 297 | gcgtgcgacaagggtgactatta | 261645 | 645305 | channel | store-operated calcium channel | - |
| 2234 | TRPC1 | NM_003304.3 | 307 | agggtgactattatatggttaaa | 261646 | 645309 | see also 2233 line | | |
| 2235 | TRPC1 | NM_003304.3 | 401 | taccataactattgaaaacgaaa | 261652 | 645312 | see also 2233 line | | |
| 2236 | TRPC1 | NM_003304.3 | 530 | agctgctcatcgtaacaactatg | 261658 | 645328 | see also 2233 line | | |
| 2237 | TRPC1 | NM_003304.3 | 1068 | aagcccacctgtaagaagataat | 261676 | 645352 | see also 2233 line | | |
| 2238 | TRPC1 | NM_003304.3 | 2036 | atggcttagctactttgatgaca | 261708 | 645377 | see also 2233 line | | |
| 2239 | TRPC1 | NM_003304.3 | 2325 | ctgcgccaagatctgtcaaaatt | 261720 | 645384 | see also 2233 line | | |
| 2240 | TRPC1 | NM_003304.3 | 2326 | tgcgccaagatctgtcaaaattc | 261721 | 645385 | see also 2233 line | | |

Figure 46

| # | Gene | Accession | Pos | Sequence | ID | Annotation | Function | Disease |
|---|------|-----------|-----|----------|----|-----------|---------|---------|
| 2241 | TRPC1 | NM_003304.3 | 2377 | ttcggacttctaaatatgctatg | 645386 | see also 2233 line | | |
| 2242 | TRPC1 | NM_003304.3 | 2378 | tcggacttctaaatatgctatgt | 645387 | see also 2233 line | | |
| 2243 | TRPC3 | NM_003305.1 | 2392 | aaactatggttggtcgtttactca | 645412 | channel | store-operated calcium channel | |
| 2244 | TRPC3 | NM_003305.1 | 2858 | tccagccttcgttatgaacttt | 645431 | see also 2243 line | - | |
| 2245 | TRPC3 | NM_003305.1 | 2860 | cagccttcgttatgaacttttgg | 645432 | see also 2243 line | - | |
| 2246 | TTC1 | NM_003314.1 | 730 | aagcttgtatgagattacctaag | 748223 | - | - | |
| 2247 | DNAJC7 | NM_003315.1 | 644 | aggtctttgcctttattacgaag | 709288 | - | protein binding | |
| 2248 | DNAJC7 | NM_003315.1 | 892 | atcgggtacggttaattccaag | 709295 | see also 2247 line | protein binding | |
| 2249 | DNAJC7 | NM_003315.1 | 1354 | tagatgaggaggcatgaatatg | 709306 | see also 2247 line | - | |
| 2250 | TTN | NM_003319.2 | 242 | acgtttacgcagccgttacaaag | 791084 | kinase_related, recept or acitivity | | cardiomyopathy, tardive tibial muscular dystrophy |
| 2251 | TTN | NM_003319.2 | 278 | gagggtagtaccgcaacctttga | 791085 | see also 2250 line | - | |
| 2252 | TTN | NM_003319.2 | 378 | ccggccgtgcagatctcctttagc | 791088 | see also 2250 line | - | |
| 2253 | TUFM | NM_003321.3 | 446 | ggggtcatgcagattatgttaaga | 477196 | - | translation elongation factor | |
| 2254 | TULP1 | NM_003322.2 | 1140 | accgttcacggttcttgacaac | 622780 | - | - | retinitis pigmentosa |
| 2255 | HIRA | NM_003325.3 | 658 | agcctcatgcagcgtggataaca | 668985 | transcription_regulator | transcription factor | |
| 2256 | TYK2 | NM_003331.3 | 3015 | ttccacaagcgtatttgaaaaa | 517327 | kinase_related | non-membrane spanning protein tyrosine kinase | |
| 2257 | UBE2B | NM_003337.2 | 570 | tactttaaactagtaatagaat | 616570 | - | ubiquitin-protein ligase | |
| 2258 | UBE2D1 | NM_003338.3 | 575 | cccttagtaccagatattgcaca | 756338 | - | ubiquitin-protein ligase | |
| 2259 | UBE2G1 | NM_003342.3 | 832 | ttgcccgctgtgaagaaaaagc | 616623 | - | - | |
| 2260 | UCN | NM_003353.2 | 451 | ccgagcagaaccgcatcatattc | 710954 | signal_transduction, g pcr | neuropeptide hormone | |
| 2261 | UCP2 | NM_003355.2 | 1018 | acctcccttgccacttcacttct | 574554 | - | uncoupling protein | obesity |
| 2262 | UCP2 | NM_003355.2 | 1090 | tggtcaagacagagatacatgaac | 574556 | see also 2261 line | - | |
| 2263 | UGCG | NM_003358.1 | 562 | atgatccagccattgatgtatgt | 606698 | - | transferase, transferring glycosyl groups | |
| 2264 | UGCG | NM_003358.1 | 833 | tgccaccttagacgcaggtatatt | 606711 | see also 2263 line | - | |
| 2265 | UGCG | NM_003358.1 | 834 | gccaccttagacgcaggtatatt | 606712 | see also 2263 line | - | |
| 2266 | UGCG | NM_003358.1 | 1142 | tcctgctacataatttgtgagc | 606723 | see also 2263 line | - | |
| 2267 | UGDH | NM_003359.1 | 1320 | cactgagtgggacatgtttaagg | 607861 | - | kinase | |

Figure 46

| | | | | | | | | | |
|---|---|---|---|---|---|---|---|---|---|
| 2268 | USP4 | NM_003363.2 | 151 | ggcgcagtggtatcttattgaca | 189875 | 583678 | - | ubiquitin-specific protease | - |
| 2269 | USP4 | NM_003363.2 | 683 | cagggtcaggtgctagtaattga | 189891 | 583692 | see also 2268 line | | |
| 2270 | USP4 | NM_003363.2 | 685 | gggtcaggtgctagtaattgagc | 189892 | 583693 | see also 2268 line | | |
| 2271 | UQCRC2 | NM_003366.1 | 518 | gcctcagctaaagattgacaaag | 184906 | 579161 | - | ubiquinol-cytochrome c reductase | - |
| 2272 | USF2 | NM_003367.1 | 481 | gtcagcggggaggcacgatttgc | 13882 | 702828 | transcription_regulator | USF43/USF44 complex | breast cancer |
| 2273 | UVRAG | NM_003369.2 | 327 | cccggaacattgttaatagaaat | 322266 | 802214 | - | - | - |
| 2274 | UVRAG | NM_003369.2 | 739 | tcgcaattcttacgatgtcttct | 322286 | 802226 | see also 2273 line | | |
| 2275 | VBP1 | NM_003372.3 | 434 | aaggtcagattcctgaaattaaa | 137010 | 542220 | - | protein binding | - |
| 2276 | VBP1 | NM_003372.3 | 730 | gtcaatatggccagggtttataa | 137020 | 542232 | see also 2275 line | | |
| 2277 | VEGF | NM_003376.3 | 1577 | aagatccgcagacgtgtaaatgt | 168110 | 566274 | signal_transduction、cell_cycle | vascular endothelial growth factor receptor ligand | bladder transitional cell carcinoma、rheumatoid arthritis、colorectal cancer、bladder cancer、neuroblastoma、prostate cancer、colorectal cancers、astrocytic tumors |
| 2278 | VEGF | NM_003376.3 | 1580 | atccgcagacgtgtaaatgttcc | 168111 | 566275 | see also 2277 line | | |
| 2279 | VEGF | NM_003376.3 | 1627 | aaggcgaggcagcttgagttaaa | 168112 | 566277 | see also 2277 line | | |
| 2280 | VRK1 | NM_003384.1 | 516 | aaggttttctcggaaaactgtct | 107469 | 517575 | kinase_related | - | - |
| 2281 | VRK1 | NM_003384.1 | 786 | cccatcaagacgtggtgatttgg | 107475 | 517583 | see also 2280 line | | |
| 2282 | VRK1 | NM_003384.1 | 789 | atcaagacgtggtgatttggaaa | 107476 | 517584 | see also 2280 line | | |
| 2283 | VSNL1 | NM_003385.3 | 712 | accagattacactggatgaattc | 154394 | 554848 | - | - | - |
| 2284 | VSNL1 | NM_003385.3 | 749 | gagcgacccttccattgttattac | 154395 | 554849 | see also 2283 line | | |
| 2285 | VSNL1 | NM_003385.3 | 750 | agcgacccttccattgttattact | 154396 | 554850 | see also 2283 line | | |
| 2286 | WASPIP | NM_003387.3 | 438 | cggccaacagggataatgattct | 120135 | 676041 | - | - | - |
| 2287 | WEE1 | NM_003390.2 | 2401 | gtctttggttcacatggatataa | 107634 | 517727 | kinase_related、cell_cycle | protein tyrosine kinase | - |
| 2288 | WEE1 | NM_003390.2 | 2405 | ttggttcacatggatataaaacc | 107635 | 517728 | see also 2287 line | | |
| 2289 | WEE1 | NM_003390.2 | 3028 | atctcgacttattggaaagaaaa | 107655 | 517749 | see also 2287 line | | |
| 2290 | WNT8B | NM_003393.2 | 273 | tgcccagagtggtattgaagaat | 255530 | 639736 | signal_transduction | signal transducer | - |
| 2291 | WNT8B | NM_003393.2 | 274 | gcccagagtggtattgaagaatg | 255531 | 639737 | see also 2290 line | | |
| 2292 | WNT8B | NM_003393.2 | 275 | cccagagtggtattgaagaatgc | 255532 | 639738 | see also 2290 line | | |
| 2293 | WNT10B | NM_003394.2 | 417 | gtcgggctctaagcaatgagatt | 255414 | 639565 | signal_transduction | signal transducer | - |
| 2294 | XPO1 | NM_003400.2 | 41 | gccagcaattatgacaatgttag | 271534 | 750091 | - | - | - |
| 2295 | XPO1 | NM_003400.2 | 84 | ctgcttgatttcagccaaaaact | 271536 | 750092 | see also 2294 line | | |
| 2296 | XPO1 | NM_003400.2 | 129 | gtggtgaattgcttataccatgg | 271537 | 750096 | see also 2294 line | | |

Figure 46

| | | | | | | | | | |
|---|---|---|---|---|---|---|---|---|---|
| 2297 | XPO1 | NM_003400.2 | 384 | gacccaacttgtgtagagaaaga | 271547 | 750105 | see also 2294 line | | |
| 2298 | XPO1 | NM_003400.2 | 386 | cccaacttgtgtagagaaagaaa | 271548 | 750106 | see also 2294 line | | |
| 2299 | XPO1 | NM_003400.2 | 469 | aacattggccaacttttatcagt | 271552 | 750108 | see also 2294 line | | |
| 2300 | XPO1 | NM_003400.2 | 2428 | aaccagaagtgcttagtactatg | 271615 | 750180 | see also 2294 line | | |
| 2301 | XPO1 | NM_003400.2 | 2486 | agctgaaatacctcaaatatttg | 271624 | 750186 | see also 2294 line | | |
| 2302 | XPO1 | NM_003400.2 | 2855 | aacaatgcatgcatcaattcttg | 271642 | 750195 | see also 2294 line | | |
| 2303 | XPO1 | NM_003400.2 | 3011 | tgctcaagtaaagctctttgtga | 271647 | 750197 | see also 2294 line | | |
| 2304 | YY1 | NM_003403.3 | 486 | ctcgggcgacaccctctacatcg | 13962 | 449865 | transcription_regulator | zinc binding | - |
| 2305 | YY1 | NM_003403.3 | 1376 | ctcataaaggctgcacaaagatg | 13968 | 449870 | see also 2304 line | | |
| 2306 | ZIC1 | NM_003412.2 | 1431 | cccaaaaagtcgtgcaacaaaac | 331657 | 450315 | transcription_regulator, signal_transduction | DNA binding | - |
| 2307 | ZIC1 | NM_003412.2 | 2026 | gtgcgctctcttccaattttaac | 331666 | 450320 | see also 2306 line | | |
| 2308 | ZIC3 | NM_003413.2 | 1764 | ctgccagttcaggctatgaatct | 44007 | 471080 | transcription_regulator | DNA binding | situs inversus、heterotaxy |
| 2309 | ZIC3 | NM_003413.2 | 1787 | tccactccacccgctatagcttc | 44009 | 471081 | see also 2308 line | | |
| 2310 | ZNF9 | NM_003418.1 | 511 | tgctataggtgtggtgaaactgg | 15678 | 457329 | - | transcription factor | myotonic dystrophy、diabetes |
| 2311 | ZNF9 | NM_003418.1 | 552 | cagcaagacaagtgaagtcaact | 15680 | 457332 | see also 2310 line | | |
| 2312 | ZNF9 | NM_003418.1 | 558 | gacaagtgaagtcaactgttacc | 15681 | 457333 | see also 2310 line | | |
| 2313 | ZNF9 | NM_003418.1 | 603 | tgcacgggaatgcacaattgagg | 15682 | 457334 | see also 2310 line | | |
| 2314 | ZNF76 | NM_003427.3 | 1692 | cgcagcccgtcacaatcattacc | 45907 | 776624 | transcription_regulator | DNA binding | - |
| 2315 | ZNF76 | NM_003427.3 | 1695 | agcccgtcacaatcattacctct | 45908 | 776625 | see also 2314 line | | |
| 2316 | ZNF189 | NM_003452.2 | 509 | aaccacagggtgtaatagttaca | 14753 | 481820 | transcription_regulator | - | - |
| 2317 | ZNF189 | NM_003452.2 | 516 | gggtgtaatagttacaagaatca | 14755 | 481822 | see also 2316 line | | |
| 2318 | ZNF189 | NM_003452.2 | 748 | gtccgcaaggcccatttcattca | 14759 | 481832 | see also 2316 line | | |
| 2319 | ZNF198 | NM_003453.2 | 921 | gagatatgaacttaatgattaca | 161558 | 669540 | transcription_regulator | - | stem-cell leukemia |
| 2320 | ZNF198 | NM_003453.2 | 1887 | atggaaaactgacaacttgtact | 161569 | 669564 | see also 2319 line | | |
| 2321 | ZNF198 | NM_003453.2 | 1962 | atggatatatggagccatattgt | 161572 | 669573 | see also 2319 line | | |
| 2322 | ZNF198 | NM_003453.2 | 2167 | atctccaaatggccagtttgtag | 161574 | 669579 | see also 2319 line | | |
| 2323 | ZNF198 | NM_003453.2 | 2312 | aagaagttgcattgcatagttac | 161579 | 669584 | see also 2319 line | | |
| 2324 | ZNF198 | NM_003453.2 | 2315 | aagttgcattgcatagttacata | 161580 | 669585 | see also 2319 line | | |
| 2325 | ZNF198 | NM_003453.2 | 2852 | tgcaaacctttaacaatgacaaa | 161599 | 669606 | see also 2319 line | | |
| 2326 | ZNF198 | NM_003453.2 | 2894 | cacatgcagaccaaatcttgtca | 161601 | 669609 | see also 2319 line | | |
| 2327 | ZNF198 | NM_003453.2 | 3404 | cagcccagacctcgatctaaaaa | 161615 | 669623 | see also 2319 line | | |

Figure 46

| | | | | | | | | | |
|---|---|---|---|---|---|---|---|---|---|
| 2328 | ZNF198 | NM_003453.2 | 3454 | aggataccagtctcatgatgata | 161616 | 669626 | see also 2319 line | | |
| 2329 | ZNF198 | NM_003453.2 | 3545 | gtcaaaactaggcaacttgatga | 161619 | 669631 | see also 2319 line | | |
| 2330 | ZNF198 | NM_003453.2 | 3638 | accacagctgagcttaactatgg | 161620 | 669636 | see also 2319 line | | |
| 2331 | ZNF198 | NM_003453.2 | 3877 | ctctcgagttgaagaagactatc | 161630 | 669648 | see also 2319 line | | |
| 2332 | ZNF198 | NM_003453.2 | 3891 | aagactatctctggaggataaaa | 161632 | 669649 | see also 2319 line | | |
| 2333 | ZNF198 | NM_003453.2 | 3899 | ctctggaggataaaacaactagg | 161634 | 669650 | see also 2319 line | | |
| 2334 | ZNF198 | NM_003453.2 | 4218 | ttgaatgctacttgtctaaaagt | 161643 | 669656 | see also 2319 line | | |
| 2335 | ZNF198 | NM_003453.2 | 4362 | ttgtacgggttcttctagtaaaa | 161652 | 669661 | see also 2319 line | | |
| 2336 | ZNF198 | NM_003453.2 | 4367 | cgggttcttctagtaaaagatat | 161653 | 669662 | see also 2319 line | | |
| 2337 | ZNF198 | NM_003453.2 | 4368 | gggttcttctagtaaaagatatt | 161654 | 669663 | see also 2319 line | | |
| 2338 | ZNF207 | NM_003457.1 | 230 | aagccgtggtgctggtattgtaa | 14881 | 690973 | transcription_regulator | zinc binding | - |
| 2339 | ZNF207 | NM_003457.1 | 231 | agccgtggtgctggtattgtaat | 14882 | 690974 | see also 2338 line | | |
| 2340 | ZNF207 | NM_003457.1 | 232 | gccgtggtgctggtattgtaata | 14883 | 690975 | see also 2338 line | | |
| 2341 | ZNF207 | NM_003457.1 | 483 | gacgacttcttgaacagaaaaca | 14894 | 690987 | see also 2338 line | | |
| 2342 | ZNF207 | NM_003457.1 | 597 | ttcaacctcagcaaggttatatt | 14896 | 690990 | see also 2338 line | | |
| 2343 | ZNF207 | NM_003457.1 | 1092 | aaggacctgttggtacagatttc | 14900 | 690993 | see also 2338 line | | |
| 2344 | ZNF207 | NM_003457.1 | 1273 | tagtgcaaccagtaagttgatcc | 14907 | 690998 | see also 2338 line | | |
| 2345 | BSN | NM_003458.1 | 4000 | tgcatacatggacccaatgaagc | 166757 | 564058 | - | - | - |
| 2346 | BSN | NM_003458.1 | 7759 | cagccgtagtggcatcaagaagc | 166780 | 564089 | see also 2345 line | | |
| 2347 | ZP2 | NM_003460.1 | 1609 | aacatcaagctggtcttagatga | 247161 | 525051 | receptor_acitivity | coreceptor | - |
| 2348 | PAX8 | NM_003466.2 | 374 | agcatccggcctggagtgatagg | 8953 | 463379 | transcription regulator、kinase_related、receptor_acitivity - | | thyroid hypoplasia、congenital hypothyroidism due to thyroid dysgenesis or hypoplasia |
| 2349 | STAM | NM_003473.2 | 422 | taggagcatgtgtatcaaactgt | 252915 | 673054 | signal_transduction | - | - |
| 2350 | STAM | NM_003473.2 | 1231 | atgggacctctcattgatgaaaa | 252933 | 673065 | see also 2349 line | | |
| 2351 | PDHX | NM_003477.1 | 1267 | accaaggaggatcttttagtatt | 134537 | 538609 | - | protein binding | lacticacidemia |
| 2352 | TAF15 | NM_003487.2 | 115 | ctgggggtgagcagcaaagttat | 19887 | 486586 | transcription_regulator | - | chondrosarcoma |
| 2353 | TAF15 | NM_003487.2 | 116 | tgggggtgagcagcaaagttatt | 19888 | 486587 | see also 2352 line | | |
| 2354 | TAF15 | NM_003487.2 | 281 | tggttatgagaatcaaaagcaga | 19895 | 486599 | see also 2352 line | | |
| 2355 | TAF15 | NM_003487.2 | 804 | gagggtgtgtctacagatcaagt | 19910 | 486611 | see also 2352 line | | |
| 2356 | ARD1 | NM_003491.2 | 388 | caggcctctcgagccatgataga | 215025 | 602299 | - | N-acetyltransferase | - |
| 2357 | ARD1 | NM_003491.2 | 393 | ctctcgagccatgatagagaact | 215026 | 602301 | see also 2356 line | | |
| 2358 | DYSF | NM_003494.2 | 5201 | cagtgagtgaccaggataaactac | 312260 | 692110 | - | - | myopathy、distal、with anterior tibial onset、limb girdle muscular dystrophy、Miyoshi myopathy |

EP 1 752 536 A1

Figure 46

| | | | | | | | | |
|---|---|---|---|---|---|---|---|---|
| 2359 | ACOX2 | NM_003500.1 | 612 | gccaccagagtttgtgataca | 224141 | 612283 | - | electron transfer flavoprotein |
| 2360 | CDC7 | NM_003503.2 | 969 | gtccagcgctctgttttggaga | 76107 | 497704 | kinase_related, cell_cycle | protein serine/threonine kinase |
| 2361 | CDC45L | NM_003504.3 | 633 | ttgactacgagcagtatgaatat | 301651 | 670073 | cell_cycle | - |
| 2362 | CDC45L | NM_003504.3 | 635 | gactacgagcagtatgaatatca | 301652 | 670074 | see also 2361 line | |
| 2363 | FZD6 | NM_003506.2 | 1682 | ctcgaccagaattggctttattt | 242598 | 630700 | receptor_acitivity, gpcr, signal_transduction | frizzled receptor |
| 2364 | NME5 | NM_003551.2 | 591 | aggactcacagagctttgtaagc | 94873 | 510398 | kinase_related, apoptosis | nucleoside-diphosphate kinase |
| 2365 | PIP5K1B | NM_003558.1 | 877 | caggctattacatgaatttaaac | 207565 | 597572 | kinase_related | - |
| 2366 | PIP5K2B | NM_003559.3 | 707 | aagatcaaggtggacaatcatct | 207644 | 597681 | kinase_related, signal_transduction | |
| 2367 | PLA2G6 | NM_003560.1 | 2391 | tggtcggcatccagtacttcaga | 199556 | 591471 | - | calcium-independent cytosolic phospholipase A2 |
| 2368 | PLA2G6 | NM_003560.1 | 2486 | gagaccgaggtctacatctatga | 199559 | 591472 | see also 2367 line | - |
| 2369 | SLC25A11 | NM_003562.3 | 580 | accagccgccgtggctacaaaat | 181005 | 574244 | - | oxoglutarate/malate antiporter |
| 2370 | SLC25A11 | NM_003562.3 | 582 | cagcgccgtggctacacaaaaagt | 181006 | 574245 | see also 2369 line | |
| 2371 | VAPA | NM_003574.4 | 365 | tagtcactacaaatcttaaattg | 292711 | 662451 | kinase_related | |
| 2372 | VAPA | NM_003574.4 | 1018 | aaggtagcacattcggataaacc | 292729 | 662464 | see also 2371 line | - |
| 2373 | STK24 | NM_003576.2 | 291 | agggtgttcaaaggcattgacaat | 101593 | 515606 | kinase_related, signal_transduction | protein tyrosine kinase |
| 2374 | RAD54L | NM_003579.2 | 2029 | ttgtaatcatccagctctaatct | 37467 | 465311 | - | |
| 2375 | RAD54L | NM_003579.2 | 2293 | tggcacgatgtccattaagaagc | 37477 | 465319 | see also 2374 line | |
| 2376 | NSMAF | NM_003580.2 | 1502 | ctggatggtgcaacggatttaa | 253666 | 701836 | signal_transduction | receptor signaling protein |
| 2377 | NSMAF | NM_003580.2 | 1503 | tggatggtgcaacggatttaaa | 253667 | 701837 | see also 2376 line | |
| 2378 | NSMAF | NM_003580.2 | 1507 | tggtgcaacggattttaagagt | 253668 | 701838 | see also 2376 line | |
| 2379 | DOC2B | NM_003585.1 | 681 | aggcaaataagctcagaacaaaa | 172619 | 656488 | - | |
| 2380 | DHX16 | NM_003587.3 | 2563 | caccagtctcgtcgaaagatgg | 59642 | 483537 | cell_cycle | RNA helicase |
| 2381 | CUL4B | NM_003588.2 | 988 | aagttagtgatcaagaactttaa | 279003 | 674956 | cell_cycle | molecular_function unknown |
| 2382 | CUL4B | NM_003588.2 | 1478 | atggtgaagcaattgatagaagt | 279017 | 674972 | see also 2381 line | |
| 2383 | CUL3 | NM_003590.1 | 501 | tgcatatacaatggtttgcata | 306105 | 674843 | - | |

Figure 46

| 2384 | CUL3 | NM_003590.1 | 535 | aagctctacactggactaagaga | 306107 | 674844 | see also 2383 line | | |
|------|------|-------------|-----|------------------------|--------|--------|--------------------|--|--|
| 2385 | CUL3 | NM_003590.1 | 1230 | tagtcgtgtgccaaatggtttga | 306145 | 674865 | see also 2383 line | | |
| 2386 | CUL3 | NM_003590.1 | 1470 | gtctcctgaatacctctcattat | 306150 | 674878 | see also 2383 line | | |
| 2387 | CUL3 | NM_003590.1 | 1595 | aagatgtatttgaacgttattat | 306152 | 674883 | see also 2383 line | | |
| 2388 | CUL3 | NM_003590.1 | 1733 | aaggaatgtttagggatatgagc | 306158 | 674886 | see also 2383 line | | |
| 2389 | CUL3 | NM_003590.1 | 2218 | tccctcgcctgtggtaaaccaac | 306167 | 674895 | see also 2383 line | | |
| 2390 | CUL3 | NM_003590.1 | 2219 | ccctcgcctgtggtaaaccaaca | 306168 | 674896 | see also 2383 line | | |
| 2391 | CUL3 | NM_003590.1 | 2525 | taccaagtccagttgttattaag | 306181 | 674901 | see also 2383 line | | |
| 2392 | CUL3 | NM_003590.1 | 2534 | cagttgttattaagaaacgtatt | 306182 | 674903 | see also 2383 line | | |
| 2393 | CUL3 | NM_003590.1 | 2558 | aaggacttattgagagagaatat | 306184 | 674904 | see also 2383 line | | |
| 2394 | CUL3 | NM_003590.1 | 2591 | cacctgaggatcgcaaagtatac | 306185 | 674906 | see also 2383 line | | |
| 2395 | CUL3 | NM_003590.1 | 2594 | ctgaggatcgcaaagtatacaca | 306187 | 674907 | see also 2383 line | | |
| 2396 | CUL3 | NM_003590.1 | 2596 | gaggatcgcaaagtatacacata | 306188 | 674908 | see also 2383 line | | |
| 2397 | CUL3 | NM_003590.1 | 2597 | aggatcgcaaagtatacacatat | 306189 | 674909 | see also 2383 line | | |
| 2398 | CUL2 | NM_003591.2 | 1092 | ggcaaatatgtacgtcttactcc | 306043 | 674800 | cell_cycle、apoptosis | - | - |
| 2399 | CUL2 | NM_003591.2 | 1892 | caggtgaagttaaaatgaactat | 306078 | 674825 | see also 2398 line | | |
| 2400 | CUL2 | NM_003591.2 | 2074 | atgattaaccatgattcagaaaa | 306085 | 674832 | see also 2398 line | | |
| 2401 | CUL2 | NM_003591.2 | 2346 | tcccagtatcagcatgattaaga | 306097 | 674835 | see also 2398 line | | |
| 2402 | CUL1 | NM_003592.2 | 779 | ttgggttcgccgtgaatgtgacg | 233081 | 533932 | cell_cycle、apoptosis | - | - |
| 2403 | CUL1 | NM_003592.2 | 1006 | ttgcaaagggccctacgttaaca | 233088 | 533940 | see also 2402 line | | |
| 2404 | CUL1 | NM_003592.2 | 1014 | ggccctacgttaacagtgtataa | 233089 | 533941 | see also 2402 line | | |
| 2405 | CUL1 | NM_003592.2 | 1110 | cagaacccagttactgaatatat | 233096 | 533944 | see also 2402 line | | |
| 2406 | CUL1 | NM_003592.2 | 1228 | aagtcctcattgaaaaacacttg | 233099 | 533946 | see also 2402 line | | |
| 2407 | CUL1 | NM_003592.2 | 1309 | gacgcatgtataatcttgtatct | 233103 | 533948 | see also 2402 line | | |
| 2408 | CUL1 | NM_003592.2 | 1339 | aggatggcctaggagaattgaaa | 233104 | 533951 | see also 2402 line | | |
| 2409 | CUL1 | NM_003592.2 | 1539 | aaggcttgtggtcgcttcataaa | 233107 | 533956 | see also 2402 line | | |
| 2410 | CUL1 | NM_003592.2 | 1540 | aggcttgtggtcgcttcataaac | 233108 | 533957 | see also 2402 line | | |
| 2411 | CUL1 | NM_003592.2 | 1541 | ggcttgtggtcgcttcataaaca | 233109 | 533958 | see also 2402 line | | |
| 2412 | CUL1 | NM_003592.2 | 1544 | ttgtggtcgcttcataaacaaca | 233110 | 533959 | see also 2402 line | | |
| 2413 | CUL1 | NM_003592.2 | 1546 | gtggtcgcttcataaacaacaac | 233111 | 533960 | see also 2402 line | | |
| 2414 | TPST1 | NM_003596.2 | 521 | atcacaaagatatgcctttaata | 217351 | 605333 | - | protein-tyrosine sulfotransferase | - |
| 2415 | TPST1 | NM_003596.2 | 1026 | gtgcatgttggttcactatgaac | 217369 | 605353 | see also 2414 line | | |
| 2416 | TPST1 | NM_003596.2 | 1061 | atcctgaacggtggatgagaaca | 217370 | 605355 | see also 2414 line | | |
| 2417 | OGT | NM_003605.3 | 263 | gagttggcacatcgagaatatca | 134293 | 601969 | signal_transduction | - | - |
| 2418 | OGT | NM_003605.3 | 4161 | ttgaaagaggcacgcatttttga | 134313 | 601981 | see also 2417 line | | |
| 2419 | OGT | NM_003605.3 | 5561 | agcagtaacacagctcttaatat | 134373 | 602035 | see also 2417 line | | |

Figure 46

| | | | | | | | | | |
|---|---|---|---|---|---|---|---|---|---|
| 2420 | OGT | NM_003605.3 | 5946 | tgccccagaaccgtatcattttt | 134392 | 602056 | see also 2417 line | | |
| 2421 | OGT | NM_003605.3 | 6326 | cagatgtgggagcattatgcagc | 134401 | 602065 | see also 2417 line | | |
| 2422 | RAE1 | NM_003610.2 | 730 | tgcagagaggggcctgattgtct | 53849 | 785562 | - | RNA binding | - |
| 2423 | RAE1 | NM_003610.2 | 736 | gaggggcctgattgtctatcagc | 53851 | 785564 | see also 2422 line | | |
| 2424 | RAE1 | NM_003610.2 | 1096 | aacttcggaacagttagatcagc | 53867 | 785574 | see also 2422 line | | |
| 2425 | OFD1 | NM_003611.1 | 361 | atgaactgcgcaaaaagctatac | 282704 | 796124 | - | molecular_function unknown | - |
| 2426 | SEMA7A | NM_003612.1 | 546 | aactccctggttctgtttgaagg | 313517 | 684674 | receptor_acitivity | - | - |
| 2427 | SEMA7A | NM_003612.1 | 584 | caccatccggaagcaggaataca | 313519 | 684675 | see also 2426 line | | |
| 2428 | CILP | NM_003613.1 | 2720 | atgaggatccacggggttaaaaag | 196111 | 783162 | phosphatase | - | - |
| 2429 | SIP1 | NM_003616.1 | 186 | gacttgacggaaggtttcgatcc | 47234 | 689180 | - | pre-mRNA splicing factor | - |
| 2430 | SIP1 | NM_003616.1 | 656 | aactagtgtcttggaatatctga | 47244 | 689193 | see also 2429 line | | |
| 2431 | PRSS12 | NM_003619.2 | 2636 | agcctttgtaccttggataaaaa | 182532 | 575884 | receptor_acitivity | trypsin | mental retardation |
| 2432 | PPFIA2 | NM_003625.2 | 1442 | tggaaatattgaagaacgtatga | 318313 | 795621 | - | - | - |
| 2433 | PPFIA2 | NM_003625.2 | 2008 | gtgacactgaaatgtctgatatt | 318332 | 795635 | see also 2432 line | | |
| 2434 | PPFIA2 | NM_003625.2 | 3887 | aagactgcagaggttagacaact | 318363 | 795682 | see also 2432 line | | |
| 2435 | PPFIA2 | NM_003625.2 | 3909 | tccactgttcgcacatactcatg | 318364 | 795683 | see also 2432 line | | |
| 2436 | PPFIA2 | NM_003625.2 | 3913 | ctgttcgcacatactcatgttga | 318365 | 795684 | see also 2432 line | | |
| 2437 | PEX3 | NM_003630.1 | 245 | accagaggacttgcaatatgaca | 309872 | 688640 | - | - | Zellweger syndrome |
| 2438 | PEX3 | NM_003630.1 | 285 | aacactgagagaggccttaatgc | 309873 | 688641 | see also 2437 line | | |
| 2439 | PEX3 | NM_003630.1 | 545 | aacagcagtatttatcaagtatt | 309884 | 688649 | see also 2437 line | | |
| 2440 | PEX3 | NM_003630.1 | 1013 | gtgtcagcctgcctttagctaag | 309899 | 688665 | see also 2437 line | | |
| 2441 | PEX3 | NM_003630.1 | 1018 | agcctgcctttagctaagataat | 309900 | 688666 | see also 2437 line | | |
| 2442 | PARG | NM_003631.1 | 1965 | ttgcactctgtctgccaaatatt | 202212 | 596060 | - | - | - |
| 2443 | PARG | NM_003631.1 | 1968 | cactctgtctgccaaatatttgc | 202213 | 596061 | see also 2442 line | | |
| 2444 | PARG | NM_003631.1 | 2111 | atgaaatcggagtattctagtta | 202219 | 596066 | see also 2442 line | | |
| 2445 | PARG | NM_003631.1 | 2128 | tagttacccagacattaacttca | 202220 | 596068 | see also 2442 line | | |
| 2446 | PARG | NM_003631.1 | 2359 | aggcatgctacaggtggattttg | 202223 | 596077 | see also 2442 line | | |
| 2447 | PARG | NM_003631.1 | 2363 | atgctacaggtggattttgcaaa | 202224 | 596078 | see also 2442 line | | |
| 2448 | PARG | NM_003631.1 | 2364 | tgctacaggtggattttgcaaat | 202225 | 596079 | see also 2442 line | | |
| 2449 | PARG | NM_003631.1 | 2653 | cagacgctacctcgatcagtttg | 202232 | 596084 | see also 2442 line | | |
| 2450 | PARG | NM_003631.1 | 2656 | acgctacctcgatcagtttgtgc | 202233 | 596085 | see also 2442 line | | |
| 2451 | ENC1 | NM_003633.1 | 1712 | cagtagtgagtgccaaacttaag | 116951 | 522569 | - | actin binding | Alzheimer disease、 Aarskog-Scott syndrome |
| 2452 | NIPSNAP1 | NM_003634.1 | 1019 | ctgggatgaaaatgtctactata | 331855 | 697952 | - | - | - |

EP 1 752 536 A1

Figure 46

| | | | | | | | | | |
|---|---|---|---|---|---|---|---|---|---|
| 2453 | NDST2 | NM_003635.2 | 947 | tacccatggccgctatgtcttgg | 217211 | 605629 | - | heparin N-deacetylase/N-sulfotransferase | - |
| 2454 | NDST2 | NM_003635.2 | 1150 | gggactaccaagtgaatccttct | 217214 | 605636 | see also 2453 line | | |
| 2455 | NDST2 | NM_003635.2 | 1626 | ttctcgggcaagttctatcatac | 217218 | 605648 | see also 2453 line | | |
| 2456 | NDST2 | NM_003635.2 | 2836 | tacaacgctggctgacttactac | 217242 | 605664 | see also 2453 line | | |
| 2457 | IKBKG | NM_003639.2 | 1164 | aggcccaggcggatatctacaag | 254494 | 669016 | apoptosis、kinase_related、transcription_regulator | signal transducer | - |
| 2458 | IKBKAP | NM_003640.2 | 1637 | tgccagtaaccagatttctgttt | 219429 | 721263 | kinase_related | signal transducer | - |
| 2459 | HAT1 | NM_003642.1 | 523 | aaggctgacatgacatgtagagg | 131657 | 721541 | - | protein binding | - |
| 2460 | HAT1 | NM_003642.1 | 537 | atgtagaggctttcgagaatatc | 131658 | 721542 | see also 2459 line | | |
| 2461 | HAT1 | NM_003642.1 | 540 | tagaggctttcgagaatatcatg | 131659 | 721543 | see also 2459 line | | |
| 2462 | HAT1 | NM_003642.1 | 630 | atggcactactttctagtatttg | 131661 | 721547 | see also 2459 line | | |
| 2463 | HAT1 | NM_003642.1 | 715 | tactatgtgtacccagacaaaac | 131664 | 721551 | see also 2459 line | | |
| 2464 | HAT1 | NM_003642.1 | 795 | tgctcaacttcttgaaacagttc | 131665 | 721553 | see also 2459 line | | |
| 2465 | HAT1 | NM_003642.1 | 836 | ttcctacagttcttgatattaca | 131668 | 721556 | see also 2459 line | | |
| 2466 | HAT1 | NM_003642.1 | 1017 | cgctagaagggtttatgaaattc | 131678 | 721564 | see also 2459 line | | |
| 2467 | DGKZ | NM_003646.1 | 921 | gccgctggagacccttcatcatc | 78230 | 597997 | kinase_related | diacylglycerol kinase | - |
| 2468 | DGKZ | NM_003646.1 | 1513 | cagccgctttcggaataagatgt | 78233 | 598010 | see also 2467 line | | |
| 2469 | DGKZ | NM_003646.1 | 1515 | gccgctttcggaataagatgttc | 78234 | 598011 | see also 2467 line | | |
| 2470 | DGKZ | NM_003646.1 | 1516 | ccgctttcggaataagatgttct | 78235 | 598012 | see also 2467 line | | |
| 2471 | DGKE | NM_003647.1 | 674 | atcagatgcgtaaagacaaaaaa | 78182 | 569597 | kinase_related | diacylglycerol kinase | - |
| 2472 | COPS3 | NM_003653.2 | 448 | aggaattggcatccttaagcaag | 331885 | 703717 | - | - | - |
| 2473 | COPS3 | NM_003653.2 | 610 | tggagcctatgatgcaaaacact | 331891 | 703722 | see also 2472 line | | |
| 2474 | COPS3 | NM_003653.2 | 613 | agcctatgatgcaaaacacttt | 331892 | 703723 | see also 2472 line | | |
| 2475 | COPS3 | NM_003653.2 | 741 | gtcatatcatgttggaatcatat | 331899 | 703727 | see also 2472 line | | |
| 2476 | CBX4 | NM_003655.1 | 1563 | acctggatgaacccatagacttg | 17913 | 450483 | transcription_regulator、apoptosis | transcription co-repressor | - |
| 2477 | BCAS1 | NM_003657.1 | 1448 | tggcgtttctcagacaaatgtca | 331936 | 740726 | - | - | breast cancer |
| 2478 | AGPS | NM_003659.1 | 1061 | gtcaaggacctcgtatgtcaaca | 217144 | 671981 | - | electron transfer flavoprotein | chondrodysplasia |
| 2479 | AGPS | NM_003659.1 | 1217 | gagtagcctgtttaagagaaatt | 217151 | 671988 | see also 2478 line | | |
| 2480 | AP3B1 | NM_003664.3 | 1262 | aactgatccaactatgatcaaga | 267605 | 556653 | - | protein transporter | - |
| 2481 | AP3B1 | NM_003664.3 | 1331 | atcaactcttcttcgagaatttc | 267606 | 556657 | see also 2480 line | | |
| 2482 | AP3B1 | NM_003664.3 | 1804 | atctcggcaagtatgatcaaaac | 267619 | 556669 | see also 2480 line | | |
| 2483 | AP3B1 | NM_003664.3 | 1808 | cggcaagtatgatcaaaactacg | 267620 | 556671 | see also 2480 line | | |

Figure 46

| | | | | | | | | | |
|---|---|---|---|---|---|---|---|---|---|
| 2484 | AP3B1 | NM_003664.3 | 1847 | aagatttattaggcagcttattg | 267624 | 556674 | see also 2480 line | | |
| 2485 | AP3B1 | NM_003664.3 | 1982 | tggcaccttatctcatactctca | 267631 | 556679 | see also 2480 line | | |
| 2486 | AP3B1 | NM_003664.3 | 1991 | atctcatactctcaacattaaag | 267632 | 556680 | see also 2480 line | | |
| 2487 | AP3B1 | NM_003664.3 | 2017 | ctgggtacctggaattatctaat | 267634 | 556684 | see also 2480 line | | |
| 2488 | AP3B1 | NM_003664.3 | 2018 | tgggtacctggaattatctaatt | 267635 | 556685 | see also 2480 line | | |
| 2489 | AP3B1 | NM_003664.3 | 2019 | gggtacctggaattatctaattg | 267636 | 556686 | see also 2480 line | | |
| 2490 | AP3B1 | NM_003664.3 | 2970 | atgggtattgacttttgtgattc | 267669 | 556707 | see also 2480 line | | |
| 2491 | BLZF1 | NM_003666.2 | 1104 | agggtaatggcagatgagttaac | 275556 | 716890 | transcription_regulator | - | - |
| 2492 | GPR49 | NM_003667.2 | 878 | ttgtaggcaacccttctcttatt | 239986 | 624966 | receptor_acitivity、 sig nal_transduction、 gpcr | protein-hormone receptor | - |
| 2493 | GPR49 | NM_003667.2 | 1368 | tgggttacatggtttaactcact | 240005 | 624989 | see also 2492 line | | |
| 2494 | GPR49 | NM_003667.2 | 1376 | atggtttaactcacttaaaatta | 240006 | 624990 | see also 2492 line | | |
| 2495 | GPR49 | NM_003667.2 | 1555 | atggacgaccttcataagaaaga | 240011 | 624998 | see also 2492 line | | |
| 2496 | MAPKAPK 5 | NM_003668.2 | 479 | aacatagttcagattattgaagt | 90141 | 507866 | kinase_related、signal _transduction | - | - |
| 2497 | BHLHB2 | NM_003670.1 | 385 | gagacgtgaccggattaacgagt | 1504 | 456374 | transcription_regulator | transcription factor | - |
| 2498 | DENR | NM_003677.2 | 162 | aggaacagtgccaagttagatgc | 56238 | 476424 | - | - | - |
| 2499 | MADD | NM_003682.2 | 215 | tcctcggttacttgactatctag | 219558 | 607005 | kinase_related、 signal _transduction | - | - |
| 2500 | MADD | NM_003682.2 | 927 | gagattgaggcctggatctatcg | 219572 | 607015 | see also 2499 line | | |
| 2501 | MADD | NM_003682.2 | 2849 | tgggctaaatactctaatggaga | 219609 | 607059 | see also 2499 line | | |
| 2502 | MADD | NM_003682.2 | 3319 | ttgcccagacccactactatagt | 219619 | 607066 | see also 2499 line | | |
| 2503 | MADD | NM_003682.2 | 3324 | cagacccactactatagtaaaga | 219620 | 607067 | see also 2499 line | | |
| 2504 | MADD | NM_003682.2 | 3327 | acccactactatagtaaagaacc | 219622 | 607068 | see also 2499 line | | |
| 2505 | EXO1 | NM_003686.3 | 816 | tgccatggcccacaaagtaatta | 26281 | 459321 | - | - | - |
| 2506 | CASK | NM_003688.1 | 85 | aagggtccttcagtgttgtacg | 75315 | 497205 | kinase_related | transferase | - |
| 2507 | CASK | NM_003688.1 | 158 | atgtagccaagttcacatcaagt | 75322 | 497210 | see also 2506 line | | |
| 2508 | CASK | NM_003688.1 | 183 | agggttaagtacagaagatctaa | 75323 | 497211 | see also 2506 line | | |
| 2509 | CASK | NM_003688.1 | 184 | gggttaagtacagaagatctaaa | 75324 | 497212 | see also 2506 line | | |
| 2510 | CASK | NM_003688.1 | 213 | agccagtatctgtcatatgctga | 75325 | 497213 | see also 2506 line | | |
| 2511 | CASK | NM_003688.1 | 809 | cagctgaaaggatcactgtttat | 75352 | 497225 | see also 2506 line | | |
| 2512 | CASK | NM_003688.1 | 956 | ccgctgtgtcaagtcacaaattc | 75356 | 497229 | see also 2506 line | | |
| 2513 | CASK | NM_003688.1 | 1485 | cagagttcggctggtacagtttc | 75367 | 497243 | see also 2506 line | | |
| 2514 | CASK | NM_003688.1 | 1602 | caggcaaggtacacttcatgttg | 75373 | 497250 | see also 2506 line | | |

Figure 46

| | | | | | | | | | |
|---|---|---|---|---|---|---|---|---|---|
| 2515 | CASK | NM_003688.1 | 1706 | gggggagtattaccttcaagatt | 75375 | 497257 | see also 2506 line | | |
| 2516 | CASK | NM_003688.1 | 1707 | ggggagtattaccttcaagattg | 75376 | 497258 | see also 2506 line | | |
| 2517 | CASK | NM_003688.1 | 1708 | gggagtattaccttcaagattgt | 75377 | 497259 | see also 2506 line | | |
| 2518 | CASK | NM_003688.1 | 1802 | atggtcatagcagcactaacaat | 75379 | 497262 | see also 2506 line | | |
| 2519 | CASK | NM_003688.1 | 1859 | aaggacgacagatctatgtaaga | 75382 | 497265 | see also 2506 line | | |
| 2520 | CASK | NM_003688.1 | 1870 | atctatgtaagagcacaatttga | 75383 | 497266 | see also 2506 line | | |
| 2521 | CASK | NM_003688.1 | 1874 | atgtaagagcacaatttgaatat | 75384 | 497267 | see also 2506 line | | |
| 2522 | CASK | NM_003688.1 | 1957 | gacatcatccagattattagtaa | 75385 | 497268 | see also 2506 line | | |
| 2523 | CASK | NM_003688.1 | 1997 | ggcagggtaaactggaaaactcc | 75387 | 497269 | see also 2506 line | | |
| 2524 | CASK | NM_003688.1 | 2674 | cactacttcgatctcacaattat | 75399 | 497275 | see also 2506 line | | |
| 2525 | STK16 | NM_003691.1 | 595 | ggggcagccagttttaatggact | 101404 | 515492 | kinase_related | protein kinase CK2 | - |
| 2526 | TNFSF11 | NM_003701.2 | 366 | cagagcgcagatggatcctaata | 167686 | 564801 | receptor_acitivity | - | arthritis |
| 2527 | SLC25A12 | NM_003705.2 | 187 | ttggcaggagtagctgatcaaac | 152972 | 553890 | - | solute:cation symporter | - |
| 2528 | SLC25A12 | NM_003705.2 | 192 | aggagtagctgatcaaaccaagg | 152973 | 553891 | see also 2527 line | | |
| 2529 | KLF7 | NM_003709.1 | 325 | tggacgtgttggctagttatagt | 5984 | 461071 | transcription_regulator | zinc binding | - |
| 2530 | KLF7 | NM_003709.1 | 328 | acgtgttggctagttatagtata | 5985 | 461073 | see also 2529 line | | |
| 2531 | KLF7 | NM_003709.1 | 993 | aacgggtgccggaaagtttatac | 6000 | 461076 | see also 2529 line | | |
| 2532 | KLF7 | NM_003709.1 | 996 | gggtgccggaaagtttatacaaa | 6002 | 461077 | see also 2529 line | | |
| 2533 | KLF7 | NM_003709.1 | 998 | gtgccggaaagtttatacaaaaa | 6003 | 461078 | see also 2529 line | | |
| 2534 | SPINT1 | NM_003710.2 | 1490 | atctccaagaaggatgtgtttgg | 293581 | 585313 | - | - | - |
| 2535 | PPAP2A | NM_003711.2 | 471 | ctggctggattgccttttgcaat | 196307 | 589833 | phosphatase、kinase_related、signal_transduction | - | - |
| 2536 | PPAP2B | NM_003713.3 | 875 | ggggaattctaccggatctatta | 196351 | 746768 | - | - | - |
| 2537 | PPAP2B | NM_003713.3 | 876 | gggaattctaccggatctattac | 196352 | 746769 | see also 2536 line | | |
| 2538 | VDP | NM_003715.1 | 1778 | cagttggtccaaggcttatgtgc | 271264 | 652866 | - | protein transporter | - |
| 2539 | CADPS | NM_003716.2 | 2357 | ctcttccaacccctgtaactttg | 305453 | 704207 | - | - | - |
| 2540 | CADPS | NM_003716.2 | 2380 | accacgcttccctctttgagatg | 305454 | 704208 | see also 2539 line | | |
| 2541 | CADPS | NM_003716.2 | 3281 | tgccccacttgttgttagatatg | 305475 | 704226 | see also 2539 line | | |
| 2542 | CADPS | NM_003716.2 | 3282 | gccccacttgttgttagatatgt | 305476 | 704227 | see also 2539 line | | |
| 2543 | CADPS | NM_003716.2 | 3375 | aagagtttaaccagtaacctacc | 305480 | 704233 | see also 2539 line | | |
| 2544 | CADPS | NM_003716.2 | 3733 | ttcgagtcccacagtcaatatgc | 305486 | 704241 | see also 2539 line | | |
| 2545 | CADPS | NM_003716.2 | 3736 | gagtcccacagtcaatatgcacc | 305487 | 704242 | see also 2539 line | | |
| 2546 | CADPS | NM_003716.2 | 4037 | ggccgacgcctacgtgactttcg | 305493 | 704251 | see also 2539 line | | |
| 2547 | PDE8B | NM_003719.1 | 780 | tgcttgctataatgaactgattc | 161941 | 589917 | signal_transduction | signal transducer | - |

Figure 46

| | | | | | | | | | |
|---|---|---|---|---|---|---|---|---|---|
| 2548 | PDE8B | NM_003719.1 | 783 | ttgctataatgaactgattcaaa | 161942 | 589918 | see also 2547 line | | |
| 2549 | PDE8B | NM_003719.1 | 1786 | ttctctcggtttggagtatgtga | 161975 | 589928 | see also 2547 line | | |
| 2550 | PDE8B | NM_003719.1 | 2187 | aacgctgcgccaggctattattg | 161980 | 589934 | see also 2547 line | | |
| 2551 | PTCH2 | NM_003738.2 | 434 | ctgccagtaaagtccaagtatca | 245681 | 633275 | receptor_acitivity | patched receptor | - |
| 2552 | PTCH2 | NM_003738.2 | 436 | gccagtaaagtccaagtatcact | 245682 | 633276 | see also 2551 line | | |
| 2553 | KCNK5 | NM_003740.2 | 1191 | ctccaaggacgtcaacatcttca | 261320 | 644805 | channel | voltage-gated ion channel | - |
| 2554 | ABCB11 | NM_003742.2 | 3264 | atcagctgcacgcttttttcaac | 69636 | 491776 | - | sodium-exporting ATPase | intrahepatic cholestasis |
| 2555 | ABCB11 | NM_003742.2 | 3267 | agctgcacgcttttttcaactgc | 69637 | 491777 | see also 2554 line | | |
| 2556 | ABCB11 | NM_003742.2 | 3819 | ggccattgtacgagatcctaaaa | 69655 | 491789 | see also 2554 line | | |
| 2557 | ABCB11 | NM_003742.2 | 3820 | gccattgtacgagatcctaaaat | 69656 | 491790 | see also 2554 line | | |
| 2558 | ABCB11 | NM_003742.2 | 3824 | ttgtacgagatcctaaaatcttg | 69657 | 491791 | see also 2554 line | | |
| 2559 | NCOA1 | NM_003743.3 | 716 | tgctaccaaaatcactagtaaat | 111936 | 450798 | receptor_acitivity、transcription_regulator、signal_transduction | - | - |
| 2560 | NCOA1 | NM_003743.3 | 1220 | cccacaccaagtgtaaactttgc | 111950 | 450813 | see also 2559 line | | |
| 2561 | NCOA1 | NM_003743.3 | 1224 | caccaagtgtaaactttgctacc | 111951 | 450814 | see also 2559 line | | |
| 2562 | NCOA1 | NM_003743.3 | 1551 | agccaatgttgccttaaaccaag | 111965 | 450820 | see also 2559 line | | |
| 2563 | NCOA1 | NM_003743.3 | 3708 | tccaccaaactatggtacaaatc | 112030 | 450870 | see also 2559 line | | |
| 2564 | NUMB | NM_003744.3 | 2146 | aaccctttctccagtgacttaca | 320780 | 684360 | - | - | - |
| 2565 | TNKS | NM_003747.1 | 2639 | atcttatgggcatgttgacatag | 136776 | 649805 | - | transferase、transferring glycosyl groups | |
| 2566 | TNKS | NM_003747.1 | 2881 | aggccttacctacctgttttaaa | 136782 | 649816 | see also 2565 line | | |
| 2567 | TNKS | NM_003747.1 | 3174 | ttggctgatatgggtcatgaaga | 136787 | 649820 | see also 2565 line | | |
| 2568 | EIF3S8 | NM_003752.2 | 284 | atccgtaatgccatgaagattcg | 56768 | 476258 | - | translation initiation factor | |
| 2569 | SLC4A4 | NM_003759.1 | 281 | tgcagaacgcatccgattcatct | 256978 | 642809 | - | - | proximal renal tubular acidosis |
| 2570 | SLC4A4 | NM_003759.1 | 283 | cagaacgcatccgattcatcttg | 256979 | 642810 | see also 2569 line | | |
| 2571 | SLC4A4 | NM_003759.1 | 1979 | ttctactgacatgtaccataata | 257039 | 642832 | see also 2569 line | | |
| 2572 | SLC4A4 | NM_003759.1 | 3171 | atggaacagcaacctttcctaag | 257065 | 642843 | see also 2569 line | | |
| 2573 | EIF4G3 | NM_003760.2 | 4131 | aacaggactttttcaaaggtttt | 49404 | 473665 | - | - | - |
| 2574 | VAMP4 | NM_003762.1 | 506 | atgtggtggcgtggatgcaaaat | 302771 | 686835 | - | - | - |
| 2575 | VAMP4 | NM_003762.1 | 508 | gtggtggcgtggatgcaaaataa | 302772 | 686836 | see also 2574 line | | |
| 2576 | VAMP4 | NM_003762.1 | 509 | tggtggcgtggatgcaaaataaa | 302773 | 686837 | see also 2574 line | | |
| 2577 | BECN1 | NM_003766.2 | 1151 | gagtctctgacagacaaatctaa | 311090 | 676459 | apoptosis | - | - |
| 2578 | BECN1 | NM_003766.2 | 1362 | gcggctcctattccatcaaaacc | 311098 | 676464 | see also 2577 line | | |

Figure 46

| | | | | | | | | |
|---|---|---|---|---|---|---|---|---|
| 2579 | DNAH1 | NM_003777.1 | 2135 | ttgaatcaacccttggttaaatt | 78529 | 499682 | - | |
| 2580 | DNAH1 | NM_003777.1 | 2139 | atcaacccttggttaaattcagt | 78530 | 499683 | see also 2579 line | |
| 2581 | DNAH1 | NM_003777.1 | 2252 | gacataccagattcagctttagc | 78534 | 499689 | see also 2579 line | |
| 2582 | DNAH1 | NM_003777.1 | 3572 | agccgacagagagctactgatga | 78556 | 499709 | see also 2579 line | |
| 2583 | DNAH1 | NM_003777.1 | 12350 | cagggctggagccgaagctatcc | 78790 | 499902 | see also 2579 line | |
| 2584 | B4GALT3 | NM_003779.2 | 1179 | tcctgtccgtaccagaattcc | 209958 | 601557 | - | transferase, transferring glycosyl groups |
| 2585 | B4GALT2 | NM_003780.2 | 128 | gtggccgtcatcctcactttga | 209934 | 601542 | - | |
| 2586 | B3GALT3 | NM_003781.2 | 542 | ctgatgtacttctatgagtatg | 165174 | 561293 | - | blood group, P system, P2(k) phenotype |
| 2587 | B3GALT2 | NM_003783.2 | 1401 | aactgggttgcaacatacgtcc | 209803 | 599067 | - | |
| 2588 | B3GALT2 | NM_003783.2 | 1700 | ccgtttgcacttggaagatgat | 209817 | 599076 | see also 2587 line | |
| 2589 | B3GALT2 | NM_003783.2 | 1814 | ctgtaaatacagccacctaatta | 209821 | 599079 | see also 2587 line | |
| 2590 | MBTPS1 | NM_003791.1 | 1433 | ttcattgatcatccgtttgttga | 182932 | 576460 | - | subtilase |
| 2591 | MBTPS1 | NM_003791.1 | 1452 | ttgacaaggtgtgggaattaaca | 182933 | 576461 | see also 2590 line | |
| 2592 | MBTPS1 | NM_003791.1 | 2669 | gactggctaacaacttcgttat | 182952 | 576481 | see also 2590 line | |
| 2593 | EED | NM_003797.2 | 907 | tgcattgacctatgatagcaata | 276004 | 535032 | transcription_regulator | transcription regulator |
| 2594 | EED | NM_003797.2 | 1360 | cagagacatacataggaattag | 276015 | 535048 | see also 2593 line | |
| 2595 | EED | NM_003797.2 | 1392 | tgcatggttaggcgatttgata | 276016 | 535049 | see also 2593 line | |
| 2596 | EED | NM_003797.2 | 1396 | atggttaggcgatttgatacttt | 276017 | 535051 | see also 2593 line | |
| 2597 | CTNNAL1 | NM_003798.1 | 2091 | ttctaaagttgacaagtgtatt | 311560 | 707307 | apoptosis | |
| 2598 | RNGTT | NM_003800.1 | 342 | gccggtggcaggaagattcttac | 196373 | 588723 | phosphatase | protein phosphatase |
| 2599 | RNGTT | NM_003800.1 | 1192 | atgttgattgatggcacaaatga | 196397 | 588750 | see also 2598 line | |
| 2600 | MYOM1 | NM_003803.1 | 533 | atgtctgagtttatcattaaa | 289302 | 701690 | - | structural constituent of muscle |
| 2601 | MYOM1 | NM_003803.1 | 760 | cgggcctcgcgatgaatgttaa | 289309 | 701700 | see also 2600 line | |
| 2602 | MYOM1 | NM_003803.1 | 1898 | tggtaggggacaaacttgatatc | 289339 | 701734 | see also 2600 line | |
| 2603 | RIPK2 | NM_003821.4 | 409 | ctccgctgctcgacagtgaaaga | 99159 | 513451 | kinase_related, apoptosis, signal_transduction | signal transducer |
| 2604 | NR5A2 | NM_003822.2 | 627 | ccgagccaatgacttaagctag | 422 | 447382 | transcription_regulator, receptor_acitivity | steroid hormone receptor |
| 2605 | NR5A2 | NM_003822.2 | 1139 | tcgagtgggccaggagtagtatc | 434 | 447397 | see also 2604 line | |
| 2606 | NR5A2 | NM_003822.2 | 1636 | aacgggatgtgccctataataa | 454 | 447405 | see also 2604 line | |
| 2607 | NR5A2 | NM_003822.2 | 1637 | acgggggatgtgccctataataac | 455 | 447406 | see also 2604 line | |
| 2608 | NAPA | NM_003827.1 | 163 | tggaggctcatccaaaatagagg | 269474 | 651932 | - | protein transporter |

Figure 46

| 2609 | NAPA | NM_003827.1 | 356 | ccccaagaggccattaactgttt | 269475 | 651940 | see also 2608 line | | |
|---|---|---|---|---|---|---|---|---|---|
| 2610 | NAPA | NM_003827.1 | 667 | cagcgccaaagactacttcttca | 269479 | 651944 | see also 2608 line | | |
| 2611 | RIOK3 | NM_003831.2 | 462 | gagatagcaaagtttccatttcc | 283356 | 667595 | kinase_related | - | - |
| 2612 | RIOK3 | NM_003831.2 | 888 | ctggaatgttggagacaatcact | 283363 | 667605 | see also 2611 line | | |
| 2613 | MATN4 | NM_003833.2 | 1104 | gcgcttcgtgaaccagattgtgg | 148167 | 686537 | - | - | - |
| 2614 | PEX11B | NM_003846.1 | 400 | ttgttttccctcatcatgaattt | 309856 | 701962 | - | - | - |
| 2615 | PEX11B | NM_003846.1 | 423 | gagccgtgatgcttatgagattc | 309857 | 701963 | see also 2614 line | | |
| 2616 | TIF1 | NM_003852.2 | 468 | gccacccaagttggagtcattcg | 41972 | 469965 | transcription_regulator | - | - |
| 2617 | TIF1 | NM_003852.2 | 1186 | atgtcatgcatttttctaaatgg | 41992 | 469993 | see also 2616 line | | |
| 2618 | TIF1 | NM_003852.2 | 2154 | gtcatgctggagccaattcgaat | 42023 | 470027 | see also 2616 line | | |
| 2619 | TIF1 | NM_003852.2 | 2158 | tgctggagccaattcgaataaaa | 42025 | 470028 | see also 2616 line | | |
| 2620 | TIF1 | NM_003852.2 | 2797 | ctctaactgtgcctgattattac | 42042 | 470045 | see also 2616 line | | |
| 2621 | TIF1 | NM_003852.2 | 2965 | aagtagccaatgctggtataaaa | 42048 | 470049 | see also 2616 line | | |
| 2622 | CCNK | NM_003858.2 | 487 | agctcgtagtttattaaatgatg | 308139 | 668701 | cell_cycle、transcription_regulator、kinase_related | - | - |
| 2623 | DPM1 | NM_003859.1 | 561 | cagattataccgaaaagaagttc | 212009 | 600282 | - | transferase、transferring glycosyl groups | |
| 2624 | FGF17 | NM_003867.1 | 88 | gagaatcacccgtctcctaattt | 169091 | 565882 | signal_transduction | growth factor | - |
| 2625 | FGF17 | NM_003867.1 | 92 | atcacccgtctcctaattttaac | 169092 | 565883 | see also 2624 line | | |
| 2626 | FGF17 | NM_003867.1 | 96 | cccgtctcctaattttaaccagt | 169093 | 565884 | see also 2624 line | | |
| 2627 | IQGAP1 | NM_003870.2 | 1856 | caggatgagtcagctgtgttatg | 121313 | 526584 | signal_transduction | calmodulin binding | - |
| 2628 | IQGAP1 | NM_003870.2 | 1857 | aggatgagtcagctgtgttatgg | 121314 | 526585 | see also 2627 line | | |
| 2629 | IQGAP1 | NM_003870.2 | 3296 | gtcgtgaaggaaattatggatga | 121341 | 526611 | see also 2627 line | | |
| 2630 | IQGAP1 | NM_003870.2 | 3808 | tgcccacttaagcatcattaatg | 121353 | 526622 | see also 2627 line | | |
| 2631 | IQGAP1 | NM_003870.2 | 3809 | gcccacttaagcatcattaatga | 121354 | 526623 | see also 2627 line | | |
| 2632 | IQGAP1 | NM_003870.2 | 4240 | acgtttaattgtggatgtcatcc | 121369 | 526639 | see also 2627 line | | |
| 2633 | NRP2 | NM_003872.1 | 184 | cccgaacccaaccagaagattgt | 206058 | 636757 | receptor_acitivity | - | - |
| 2634 | NRP2 | NM_003872.1 | 852 | gtctggccggattgctaatgaac | 206069 | 636773 | see also 2633 line | | |
| 2635 | NRP2 | NM_003872.1 | 2357 | ttgatgacattcggataagcact | 206091 | 636792 | see also 2633 line | | |
| 2636 | CD84 | NM_003874.1 | 867 | gagtccagaatctatgatgaaat | 313572 | 704292 | - | - | - |
| 2637 | SOCS2 | NM_003877.3 | 737 | ctggggaagtatgactgttaatg | 310206 | 676363 | signal_transduction | apoptosis inhibitor | - |
| 2638 | SOCS2 | NM_003877.3 | 740 | gggaagtatgactgttaatgaag | 310208 | 676364 | see also 2637 line | | |
| 2639 | WISP1 | NM_003882.2 | 395 | cgcccgaggtacgcaataggagt | 124967 | 528826 | signal_transduction | - | - |

Figure 46

| | | | | | | | | | |
|---|---|---|---|---|---|---|---|---|---|
| 2640 | HDAC3 | NM_003883.2 | 1020 | agcttccctatagtgaatacttc | 113623 | 526388 | transcription_regulator、apoptosis、cell_cycle | hydrolase | - |
| 2641 | HDAC3 | NM_003883.2 | 1081 | cagcacccgcatcgagaatcaga | 113625 | 526390 | see also 2640 line | | |
| 2642 | HDAC3 | NM_003883.2 | 1084 | cacccgcatcgagaatcagaact | 113626 | 526391 | see also 2640 line | | |
| 2643 | HDAC3 | NM_003883.2 | 1124 | cagatccgccagacaatctttga | 113627 | 526392 | see also 2640 line | | |
| 2644 | HDAC3 | NM_003883.2 | 1127 | atccgccagacaatctttgaaaa | 113628 | 526393 | see also 2640 line | | |
| 2645 | HDAC3 | NM_003883.2 | 1128 | tccgccagacaatctttgaaaac | 113629 | 526394 | see also 2640 line | | |
| 2646 | PROZ | NM_003891.1 | 1186 | ctggtttaaacagatcatgaact | 134886 | 552729 | - | trypsin | - |
| 2647 | LDB1 | NM_003893.3 | 851 | tggaccaaagagatataccattg | 30432 | 461145 | transcription_regulator | - | - |
| 2648 | LDB1 | NM_003893.3 | 1081 | acgacatgatgcggataaagacg | 30440 | 461152 | see also 2647 line | | |
| 2649 | LDB1 | NM_003893.3 | 1207 | ctcggtgtgggctgtccaattcc | 30441 | 461154 | see also 2647 line | | |
| 2650 | LDB1 | NM_003893.3 | 1239 | tacctccgactctgtgtgatact | 30442 | 461156 | see also 2647 line | | |
| 2651 | LDB1 | NM_003893.3 | 1630 | ttgacgacgaggacagctttaac | 30444 | 461159 | see also 2647 line | | |
| 2652 | LDB1 | NM_003893.3 | 1633 | acgacgaggacagctttaacaac | 30445 | 461160 | see also 2647 line | | |
| 2653 | SIAT9 | NM_003896.2 | 601 | ccggaagttctccagtaaagtcc | 212903 | 601049 | - | - | - |
| 2654 | SIAT9 | NM_003896.2 | 820 | aactactataaggatgacttatc | 212910 | 601056 | see also 2653 line | | |
| 2655 | SYNJ2 | NM_003898.1 | 1966 | agctggtgggcgtctgtctttat | 55246 | 486544 | phosphatase | inositol-1、4、5-trisphosphate 5-phosphatase | - |
| 2656 | ARHGEF7 | NM_003899.2 | 2391 | gcctgggatgagaccaatctata | 295476 | 707107 | signal_transduction | - | - |
| 2657 | SGPL1 | NM_003901.2 | 1060 | gtctgttctaccccacagtttcc | 233923 | 618275 | apoptosis | - | - |
| 2658 | SGPL1 | NM_003901.2 | 1441 | ttcggtgagaacggctatgttga | 233929 | 618282 | see also 2657 line | | |
| 2659 | FUBP1 | NM_003902.2 | 1753 | cagctggacaggttgattatacc | 4321 | 482533 | transcription_regulator | - | - |
| 2660 | CDC16 | NM_003903.2 | 1512 | atgggaacctttgttgaacaact | 306833 | 675198 | cell_cycle | - | - |
| 2661 | APPBP1 | NM_003905.2 | 606 | tagaggatctacgactagataag | 235785 | 621579 | apoptosis、cell_cycle、signal_transduction | - | - |
| 2662 | APPBP1 | NM_003905.2 | 706 | atggattgtgatcatagctaaat | 235790 | 621584 | see also 2661 line | | |
| 2663 | APPBP1 | NM_003905.2 | 707 | tggattgtgatcatagctaaata | 235791 | 621585 | see also 2661 line | | |
| 2664 | APPBP1 | NM_003905.2 | 711 | ttgtgatcatagctaaatattta | 235792 | 621586 | see also 2661 line | | |
| 2665 | APPBP1 | NM_003905.2 | 987 | tagctcgtgccttaaaggaattt | 235803 | 621603 | see also 2661 line | | |
| 2666 | APPBP1 | NM_003905.2 | 1042 | tcgaggcacaattcctgatatga | 235805 | 621605 | see also 2661 line | | |
| 2667 | EIF2S2 | NM_003908.2 | 680 | atcgagtgttcaacatcatgagg | 51216 | 476535 | - | translation initiation factor | - |
| 2668 | CPNE3 | NM_003909.1 | 1289 | aactctatggaccaactaatttt | 172604 | 730852 | kinase_related | transporter | Alzheimer disease |
| 2669 | PRPF4B | NM_003913.3 | 1671 | ccctaatagaacagagaagaatc | 98189 | 512641 | kinase_related | - | - |

Figure 46

| | | | | | | | | | |
|---|---|---|---|---|---|---|---|---|---|
| 2670 | PRPF4B | NM_003913.3 | 1887 | aagccaagcataatctaatgaca | 98197 | 512646 | see also 2669 line | | |
| 2671 | PRPF4B | NM_003913.3 | 2103 | gtgaagtcctagataaacgttac | 98209 | 512652 | see also 2669 line | | |
| 2672 | PRPF4B | NM_003913.3 | 2465 | ctggcattgaaactccttaaaag | 98224 | 512664 | see also 2669 line | | |
| 2673 | CPNE1 | NM_003915.2 | 67 | tacatgggcaatcgctttattca | 334655 | 738854 | - | | |
| 2674 | CPNE1 | NM_003915.2 | 124 | aagatagatatgattcgaaaaag | 334659 | 738860 | see also 2673 line | | |
| 2675 | CPNE1 | NM_003915.2 | 603 | tacgctttgaatctatgacata | 172532 | 771303 | see also 2673 line | | |
| 2676 | CPNE1 | NM_003915.2 | 1087 | ggcagtcccgctgagtttgaat | 172544 | 771311 | see also 2673 line | | |
| 2677 | CPNE1 | NM_003915.2 | 1089 | cagtcccggctgagtttgaatgc | 172545 | 771312 | see also 2673 line | | |
| 2678 | SGCE | NM_003919.1 | 308 | tagtaatgatcccataacattta | 318372 | 687442 | - | | alcoholism |
| 2679 | SGCE | NM_003919.1 | 356 | ccgacctgatggcttcgatata | 318373 | 687444 | see also 2678 line | | |
| 2680 | SGCE | NM_003919.1 | 358 | gacctggatggcttcgatatatc | 318374 | 687445 | see also 2678 line | | |
| 2681 | SGCE | NM_003919.1 | 359 | acctggatggcttcgatatatcc | 318375 | 687446 | see also 2678 line | | |
| 2682 | TIMELESS | NM_003920.1 | 1565 | gtgatggagtaccgagaactatt | 42235 | 469184 | - | | |
| 2683 | PHOX2B | NM_003924.2 | 485 | tccagtataaccgataaggaacc | 9264 | 447735 | transcription regulator | translation regulator | congenital central hypoventilation syndrome, Hirschsprung disease |
| 2684 | PHOX2B | NM_003924.2 | 493 | aaaccgataaggaccactttgg | 9265 | 447736 | see also 2683 line | | |
| 2685 | PHOX2B | NM_003924.2 | 1268 | gtgccaaagccgccttagtgaag | 9269 | 447744 | see also 2683 line | | |
| 2686 | PHOX2B | NM_003924.2 | 1269 | tgccaaagccgccttagtgaaga | 9270 | 447745 | see also 2683 line | | |
| 2687 | MBD3 | NM_003926.5 | 110 | cacagggatgtcttttacttatag | 31556 | 461411 | transcription regulator | | |
| 2688 | MBD2 | NM_003927.3 | 949 | tacaacattgccaattagacaaa | 20356 | 461396 | transcription regulator | | |
| 2689 | SCAP2 | NM_003930.3 | 328 | ccctccctgaggaaattaggaac | 252734 | 675299 | kinase related, signal transduction | | |
| 2690 | SCAP2 | NM_003930.3 | 1249 | agcgtgtgatgtgatttacatt | 252757 | 675328 | see also 2689 line | | |
| 2691 | SCAP2 | NM_003930.3 | 1270 | ttcttagcaaggaatacaataga | 252758 | 675331 | see also 2689 line | | |
| 2692 | WASF1 | NM_003931.1 | 958 | ctcagacatacgtgatcatatg | 114960 | 743743 | | actin polymerizing | |
| 2693 | WASF1 | NM_003931.1 | 962 | gacatacgttgatcatatggatg | 114961 | 743744 | see also 2692 line | | |
| 2694 | WASF1 | NM_003931.1 | 1133 | cagttctgctacagtttgatag | 114963 | 743747 | see also 2692 line | | |
| 2695 | AP3D1 | NM_003938.4 | 653 | cagagacctggcaatgacatca | 267718 | 695560 | - | protein transporter | |
| 2696 | BTRC | NM_003939.2 | 193 | gcgaaccccctaggaagataata | 233011 | 617583 | signal transduction | | |
| 2697 | BTRC | NM_003939.2 | 375 | cggaaactctcagcaagctatga | 233020 | 617590 | see also 2696 line | | |
| 2698 | BTRC | NM_003939.2 | 1715 | tagttcacatgatgacacaatcc | 233058 | 617631 | see also 2696 line | | |
| 2699 | SLC25A14 | NM_003951.2 | 209 | gggtatctttcccggaataatcc | 181011 | 574257 | - | | |
| 2700 | SLC25A14 | NM_003951.2 | 219 | cccggaataatctaattttct | 181013 | 574258 | see also 2699 line | | |
| 2701 | MAP3K14 | NM_003954.1 | 487 | gtgtgagagtagccaagagttca | 89159 | 507050 | kinase related | protein kinase CK2 | rheumatoid arthritis |
| 2702 | MAP3K14 | NM_003954.1 | 915 | aactgatcagcccttgcaatgt | 89164 | 507056 | see also 2701 line | | |

Figure 46

| | | | | | | | | | |
|---|---|---|---|---|---|---|---|---|---|
| 2703 | UBE1C | NM_003968.3 | 124 | aaggtcgctggaaccatgtaaag | 235388 | 620484 | apoptosis、cell_cycle | - | - |
| 2704 | UBE1C | NM_003968.3 | 125 | aggtcgctggaaccatgtaaaga | 235389 | 620485 | see also 2703 line | | |
| 2705 | UBE1C | NM_003968.3 | 127 | gtcgctggaaccatgtaaagaag | 235390 | 620486 | see also 2703 line | | |
| 2706 | UBE1C | NM_003968.3 | 207 | ctccagttcttgttagatacatg | 235394 | 620489 | see also 2703 line | | |
| 2707 | UBE1C | NM_003968.3 | 877 | tagagagagcatcacaatataat | 235415 | 620506 | see also 2703 line | | |
| 2708 | UBE1C | NM_003968.3 | 879 | gagagagcatcacaatataatat | 235416 | 620507 | see also 2703 line | | |
| 2709 | UBE1C | NM_003968.3 | 881 | gagagcatcacaatataatatta | 235417 | 620508 | see also 2703 line | | |
| 2710 | UBE2M | NM_003969.2 | 941 | ctggaagccagtccttacgataa | 231256 | 616555 | - | - | - |
| 2711 | UBE2M | NM_003969.2 | 942 | tggaagccagtccttacgataaa | 231257 | 616556 | see also 2710 line | | |
| 2712 | UBE2M | NM_003969.2 | 946 | agccagtccttacgataaactcc | 231258 | 616557 | see also 2710 line | | |
| 2713 | UBE2M | NM_003969.2 | 956 | tacgataaactccataatttatg | 231259 | 616559 | see also 2710 line | | |
| 2714 | SPAG9 | NM_003971.3 | 574 | atgatccataattatatggaaca | 320375 | 730029 | - | - | - |
| 2715 | SPAG9 | NM_003971.3 | 619 | cagctctcagggagtgatcaact | 320376 | 730030 | see also 2714 line | | |
| 2716 | SPAG9 | NM_003971.3 | 624 | ctcagggagtgatcaactagaat | 320377 | 730032 | see also 2714 line | | |
| 2717 | SPAG9 | NM_003971.3 | 3006 | gaggaaatgtctccattccatta | 320444 | 730083 | see also 2714 line | | |
| 2718 | SPAG9 | NM_003971.3 | 3419 | ttgagccttatgtaagcaaaatg | 320462 | 730096 | see also 2714 line | | |
| 2719 | SPAG9 | NM_003971.3 | 3594 | tggaagtgtaatccgtgtatatg | 320466 | 730098 | see also 2714 line | | |
| 2720 | DOK2 | NM_003974.1 | 662 | agggcaactttgagttcgaaacc | 130789 | 567080 | signal_transduction | protein binding | - |
| 2721 | SLC7A6 | NM_003983.1 | 1005 | aggttgggacacccttaattttg | 259871 | 641715 | - | - | - |
| 2722 | SLC7A6 | NM_003983.1 | 1015 | acccttaattttgtaacagaaga | 259872 | 641719 | see also 2721 line | | |
| 2723 | BBOX1 | NM_003986.1 | 851 | ttggagtggattactgtgatttt | 222379 | 609681 | - | oxidoreductase、acting on single donors with incorporation of molecular oxygen、incorporation of two atoms of oxygen | - |
| 2724 | NFKB1 | NM_003998.2 | 589 | atggcccataccttcaaatatta | 7941 | 446693 | transcription regulator、apoptosis、signal_tr ansduction | Nuclear factor NF-kappa-B p50 subunit | rheumatoid arthritis、atherosclerosis、diabetes mellitus、diabetes |
| 2725 | NFKB1 | NM_003998.2 | 2712 | gagaactttgagcctctctatga | 7987 | 446732 | see also 2724 line | | |
| 2726 | INPP4A | NM_004027.1 | 3014 | ttggaagtcgcaaatatgcattt | 317804 | 742167 | signal_transduction | - | - |
| 2727 | INPP4A | NM_004027.1 | 3015 | tggaagtcgcaaatatgcattta | 317805 | 742168 | see also 2726 line | | |
| 2728 | AMPD2 | NM_004037.5 | 775 | gacctgcgcacgtctatggatgg | 203654 | 594036 | - | - | - |
| 2729 | AMPD2 | NM_004037.5 | 786 | gtctatggatggcaaatgcaagg | 203655 | 594037 | see also 2728 line | | |
| 2730 | B2M | NM_004048.2 | 288 | cagcaaggactggtctttctatc | 245148 | 632904 | - | MHC class I receptor | carpal tunnel syndrome、hemodialysis-related amyloidosis、diabetes、multiple myeloma |

Figure 46

| | | | | | | | | | |
|---|---|---|---|---|---|---|---|---|---|
| 2731 | BDH | NM_004051.2 | 906 | cggactgcctgcgctatgagatg | 220848 | 613589 | - | enzyme | - |
| 2732 | CAPN5 | NM_004055.3 | 184 | gccctatgaggaccagaactact | 187615 | 581352 | signal_transduction | - | - |
| 2733 | CA8 | NM_004056.3 | 954 | ttccgatacccttaactatatc | 156295 | 557361 | - | zinc binding | - |
| 2734 | CA8 | NM_004056.3 | 955 | tccgataccctttaactatatcc | 156296 | 557362 | see also 2733 line | | |
| 2735 | CCBL1 | NM_004059.2 | 220 | aagatcctggcaagtttctttgg | 218155 | 606278 | - | - | - |
| 2736 | CCNG1 | NM_004060.3 | 356 | acgataatggcctcagaatgact | 306623 | 673685 | cell_cycle、 kinase_rel ated | cyclin-dependent protein kinase、 regulator | |
| 2737 | CCNG1 | NM_004060.3 | 683 | aagctactactgcctttcaattt | 306631 | 673699 | see also 2736 line | | |
| 2738 | CDH17 | NM_004063.2 | 489 | acccacgtttctccagtcaaagt | 139588 | 533475 | - | transporter | - |
| 2739 | CDH17 | NM_004063.2 | 771 | aggccagagtgagaattccttca | 139596 | 533489 | see also 2738 line | | |
| 2740 | CDH17 | NM_004063.2 | 1432 | aacgttattgatatcaatgatca | 139618 | 533512 | see also 2738 line | | |
| 2741 | CDH17 | NM_004063.2 | 2475 | gggcatggcagttggtatactgc | 139651 | 533535 | see also 2738 line | | |
| 2742 | CDKN1B | NM_004064.2 | 711 | cagcttgcccgagttctactaca | 219262 | 664292 | kinase_related、 cell_c ycle | Smad3/Sp-1 heterodimer | colorectal cancer、 atherosclerosis、 bladder cancer、 breast cancer、 ovarian cancer、 prostate cancer、 myocardial infarction、 Ewing's sarcoma、 colorectal cancers、 gastric cancer |
| 2743 | CNK | NM_004073.1 | 219 | gccgcatcagcgcgagaagatcc | 77274 | 705150 | kinase_related、 cell_c ycle | protein kinase CK2 | - |
| 2744 | CNK | NM_004073.1 | 224 | atcagcgcgagaagatcctaaat | 77275 | 705151 | see also 2743 line | | |
| 2745 | CNK | NM_004073.1 | 226 | cagcgcgagaagatcctaaatga | 77276 | 705152 | see also 2743 line | | |
| 2746 | CRY1 | NM_004075.2 | 1743 | ttgatgcagattggagcataaat | 24654 | 618356 | gpcr、 receptor_acitivit y | - | - |
| 2747 | CRY1 | NM_004075.2 | 1851 | cagatcccaatggagactatatc | 24655 | 618357 | see also 2746 line | | |
| 2748 | CRY1 | NM_004075.2 | 1963 | atgtttgataggagttaattatc | 24659 | 618363 | see also 2746 line | | |
| 2749 | CTSS | NM_004079.3 | 734 | gacgcttcctatccctacaaagc | 187864 | 581601 | - | cathepsin S | Alzheimer disease |
| 2750 | COCH | NM_004086.1 | 583 | ttgggcagcgccgatttaattta | 313664 | 695832 | - | translation regulator | deafness |
| 2751 | COCH | NM_004086.1 | 584 | tgggcagcgccgatttaatttac | 313665 | 695833 | see also 2750 line | | |
| 2752 | COCH | NM_004086.1 | 1024 | gtcggaataatggcttcttctct | 313675 | 695843 | see also 2750 line | | |
| 2753 | COCH | NM_004086.1 | 1181 | cagcagtgttggagatagcaatt | 313680 | 695848 | see also 2750 line | | |
| 2754 | DLG1 | NM_004087.1 | 190 | tgccggtccggaagcaagatacc | 120872 | 534487 | kinase_related | membrane-associated guanylate kinase | - |
| 2755 | DLG1 | NM_004087.1 | 1571 | aagggaacctagaaaagttgttc | 120906 | 534523 | see also 2754 line | | |
| 2756 | DLG1 | NM_004087.1 | 2137 | aagaacgagcccgattaaaaaca | 120922 | 534536 | see also 2754 line | | |
| 2757 | DLG1 | NM_004087.1 | 2303 | ttctaatgccagcgatagtgaaa | 120927 | 534540 | see also 2754 line | | |
| 2758 | DUSP3 | NM_004090.2 | 440 | gccgctccccaacgctagttatc | 195178 | 588346 | phosphatase | protein tyrosine/serine/threoni ne phosphatase | - |

Figure 46

| 2759 | DUSP3 | NM_004090.2 | 441 | ccgctccccaacgctagttatcg | 195179 | 588347 | see also 2758 line | | |
| 2760 | ECHS1 | NM_004092.2 | 150 | ttcgcctcgggtgctaactttga | 233513 | 621998 | - | short-chain enoyl-CoA hydratase | - |
| 2761 | ECHS1 | NM_004092.2 | 502 | agcttgccatgatgtgtgatatc | 233519 | 622005 | see also 2760 line | | |
| 2762 | EFNB2 | NM_004093.2 | 134 | tcctcgaactccaaatttctacc | 319586 | 684384 | - | transmembrane ephrin | breast cancer、neuroblastoma、melanoma |
| 2763 | EFNB2 | NM_004093.2 | 167 | ctggtactatacccacagatagg | 319588 | 684386 | see also 2762 line | | |
| 2764 | EFNB2 | NM_004093.2 | 231 | ctgttggccagtatgaatattat | 319590 | 684392 | see also 2762 line | | |
| 2765 | EMX2 | NM_004098.2 | 1403 | acggaaactcaggtaaaagtatg | 3174 | 443410 | transcription_regulator | transcription factor | - |
| 2766 | EMX2 | NM_004098.2 | 1541 | caggcgagtccggaggaaataga | 3177 | 443414 | see also 2765 line | | |
| 2767 | EMX2 | NM_004098.2 | 1543 | ggcgagtccggaggaaatagacg | 3178 | 443415 | see also 2765 line | | |
| 2768 | STOM | NM_004099.4 | 508 | cccgtcttttggcacaaactact | 322948 | 692211 | - | - | stomatocytosis |
| 2769 | EYA4 | NM_004100.2 | 781 | atggagcgcttgacacttttact | 205065 | 595828 | phosphatase、transcription_regulator | - | - |
| 2770 | EYA4 | NM_004100.2 | 794 | cacttttactgggtcagtaatta | 205066 | 595829 | see also 2769 line | | |
| 2771 | EYA4 | NM_004100.2 | 1771 | aggacttaagtacctacagtttt | 205090 | 595853 | see also 2769 line | | |
| 2772 | PTK2B | NM_004103.3 | 1026 | gtggaggtatgaccttcaaatcc | 98530 | 512887 | kinase_related、apoptosis、signal_transduction | - | - |
| 2773 | EFEMP1 | NM_004105.2 | 542 | gactggccgaaataactttgtca | 142802 | 547758 | - | - | Doyne honeycomb retinal degeneration、Alzheimer disease |
| 2774 | EFEMP1 | NM_004105.2 | 544 | ctggccgaaataactttgtcatc | 142803 | 547759 | see also 2773 line | | |
| 2775 | EFEMP1 | NM_004105.2 | 1455 | gagttctacctacgacaaacaag | 142830 | 547785 | see also 2773 line | | |
| 2776 | EFEMP1 | NM_004105.2 | 1586 | ctctgtgttaagattgacaataa | 142836 | 547788 | see also 2773 line | | |
| 2777 | FGF13 | NM_004114.2 | 706 | cccgcgagcgcgagaaatccaac | 169052 | 565837 | signal_transduction | - | - |
| 2778 | FGF13 | NM_004114.2 | 707 | ccgcgagcgcgagaaatccaacg | 169053 | 565838 | see also 2777 line | | |
| 2779 | FGF13 | NM_004114.2 | 767 | cagctgcgacaaaaacaagttaa | 169054 | 565840 | see also 2777 line | | |
| 2780 | FGF14 | NM_004115.2 | 153 | gcgcatcttcggcctcaagaagc | 169067 | 565849 | signal_transduction | - | - |
| 2781 | FGF14 | NM_004115.2 | 397 | ctctacccatcagaactttttac | 169075 | 565855 | see also 2780 line | | |
| 2782 | FKBP5 | NM_004117.2 | 245 | ggggagtattaaagattgtcaaa | 179867 | 619051 | - | cyclophilin-type peptidy-prolyl cis-trans isomerase | |
| 2783 | FKBP5 | NM_004117.2 | 246 | gggagtattaaagattgtcaaaa | 179868 | 619052 | see also 2782 line | | |
| 2784 | FLT3 | NM_004119.1 | 1345 | atgttcacgctgaatataagaag | 83688 | 502612 | receptor_acitivity、signal_transduction、kinase_related | vascular endothelial growth factor receptor | - |
| 2785 | FLT3 | NM_004119.1 | 1540 | tgggtgtcgagcagtactctaaa | 83692 | 502619 | see also 2784 line | | |

Figure 46

| | | | | | | | | | |
|---|---|---|---|---|---|---|---|---|---|
| 2786 | GUCY1B2 | NM_004129.1 | 1742 | atccatactggaccagtcttagc | 234158 | 618551 | - | receptor guanylate cyclase | - |
| 2787 | GYG | NM_004130.2 | 259 | ctgctcatctaaccttaatgaag | 211699 | 600158 | - | glycogenin glucosyltransferase | - |
| 2788 | GYG | NM_004130.2 | 512 | ttctgagcaaggtagttttgatg | 211707 | 600171 | see also 2787 line | | |
| 2789 | GYG | NM_004130.2 | 638 | ctcctacctcccggcatttaaag | 211710 | 600180 | see also 2787 line | | |
| 2790 | GYG | NM_004130.2 | 639 | tcctacctcccggcatttaaagt | 211711 | 600181 | see also 2787 line | | |
| 2791 | HSPA9B | NM_004134.4 | 983 | gagagacaggggttgatttgact | 85289 | 503973 | - | ATP binding | - |
| 2792 | IDH3G | NM_004135.2 | 612 | tgccaacgtcatccactgtaaga | 85635 | 504089 | - | - | - |
| 2793 | MYO9B | NM_004145.2 | 551 | tggccatcaaccccctttaagttc | 93749 | 509969 | - | myosin ATPase | - |
| 2794 | MYO9B | NM_004145.2 | 552 | ggccatcaacccctttaagttcc | 93750 | 509970 | see also 2793 line | | |
| 2795 | MYO9B | NM_004145.2 | 2215 | taccgcgatttgcataaccaaat | 93769 | 509990 | see also 2793 line | | |
| 2796 | MYO9B | NM_004145.2 | 2216 | accgcgatttgcataaccaaatg | 93770 | 509991 | see also 2793 line | | |
| 2797 | PRKAR2A | NM_004157.1 | 884 | tggaccgggtgacttttagaaga | 108055 | 518021 | kinase_related、signal _transduction | cAMP-dependent protein kinase、regulator | - |
| 2798 | RAB1A | NM_004161.2 | 182 | tccagcatgaatcccgaatatga | 109949 | 465070 | signal_transduction | protein transporter | - |
| 2799 | RAB1A | NM_004161.2 | 459 | cagatcaggagtccttcaataat | 109960 | 465075 | see also 2798 line | | |
| 2800 | RAB5A | NM_004162.3 | 497 | gtgaaaggccaatttcatgaatt | 110333 | 520132 | signal_transduction | protein transporter | - |
| 2801 | SLC1A3 | NM_004172.3 | 645 | ctgtggtgattggcataatcatt | 257661 | 641176 | - | sodium:dicarboxylate/ tricarboxylate cotransporter | - |
| 2802 | SLC1A3 | NM_004172.3 | 788 | ctggtagaagcctgctttaaaca | 257663 | 641179 | see also 2801 line | | |
| 2803 | SNRPD3 | NM_004175.3 | 443 | gtctattggtgtgccgattaaag | 47344 | 471383 | - | pre-mRNA splicing factor | - |
| 2804 | STX3A | NM_004177.3 | 716 | atcttcacttctgggatcattga | 270949 | 652739 | - | protein transporter | - |
| 2805 | TPH1 | NM_004179.1 | 923 | tggcaacgtgctactttttcact | 164528 | 562337 | - | tryptophan 5-monooxygenase | - |
| 2806 | WNT2B | NM_004185.2 | 942 | tgccacccggactgatcttgtct | 255462 | 639606 | - | - | - |
| 2807 | WNT2B | NM_004185.2 | 945 | cacccggactgatcttgtctact | 255463 | 639607 | see also 2806 line | | |
| 2808 | WNT2B | NM_004185.2 | 948 | ccggactgatcttgtctactttg | 255464 | 639608 | see also 2806 line | | |
| 2809 | SMCX | NM_004187.1 | 538 | ccggggtccgacgatttcctacc | 40182 | 704655 | transcription_regulator | molecular_function unknown | - |
| 2810 | STK19 | NM_004197.1 | 997 | gagctgggagattcatcaagtac | 101563 | 515559 | kinase_related | - | - |
| 2811 | SYT7 | NM_004200.2 | 139 | ctgggcaaacgctacaagaattc | 275152 | 657629 | - | - | - |
| 2812 | SYT7 | NM_004200.2 | 140 | tgggcaaacgctacaagaattcc | 275153 | 657630 | see also 2811 line | | |
| 2813 | PIGQ | NM_004204.2 | 475 | caggtcatgctcatcttctatga | 210278 | 599428 | - | - | - |
| 2814 | PIGQ | NM_004204.2 | 1323 | caggctgtactgcctgaagatcc | 210283 | 599436 | see also 2813 line | | |

Figure 46

| | | | | | | | | |
|---|---|---|---|---|---|---|---|---|
| 2815 | USP2 | NM_004205.3 | 1760 | cagcaagctcacacattgtga | 189142 | 583151 | - | - |
| 2816 | PDCD8 | NM_004208.2 | 386 | gtccttattgtgggcttatcaac | 224901 | 611337 | apoptosis, signal_tran sduction | - |
| 2817 | PDCD8 | NM_004208.2 | 387 | tccttattgtgggcttatcaaca | 224902 | 611338 | see also 2816 line | - |
| 2818 | PDCD8 | NM_004208.2 | 936 | atgaaaagtgcttgattgcaaca | 224920 | 611354 | see also 2816 line | |
| 2819 | PDCD8 | NM_004208.2 | 1223 | agcaactggaccatggaaaaagt | 224928 | 611363 | see also 2816 line | |
| 2820 | SLC6A5 | NM_004211.1 | 826 | acgagcaaggggatgagaataag | 256039 | 619960 | - | glycine:sodium symporter |
| 2821 | SLC6A5 | NM_004211.1 | 1870 | aacgcaaagtcaacattgagaat | 256062 | 619990 | see also 2820 line | - |
| 2822 | EBAG9 | NM_004215.3 | 827 | ctgaattaggtgacttagatacc | 248419 | 634783 | apoptosis | - |
| 2823 | STK17B | NM_004226.2 | 394 | ttgctgtggttagacaatgtata | 101449 | 515512 | kinase_related, apopto sis | protein serine/threonine kinase |
| 2824 | SOCS4 | NM_004232.2 | 1128 | tgctacctccaatgcagaataat | 310249 | 540543 | - | - |
| 2825 | SOCS4 | NM_004232.2 | 1723 | taccagaatagacttaattcaga | 310265 | 540572 | see also 2824 line | - |
| 2826 | TRIP15 | NM_004236.1 | 1027 | atggatgatcctttcattaagaga | 136908 | 690117 | - | protein binding |
| 2827 | TRIP15 | NM_004236.1 | 1106 | agccttaacacagaatacacatatt | 136914 | 690122 | see also 2826 line | - |
| 2828 | TRIP12 | NM_004238.1 | 2785 | aagccaagacctgaagatagtag | 230943 | 616046 | - | ubiquitin-protein ligase |
| 2829 | TRIP12 | NM_004238.1 | 2786 | agccaagacctgaagatagtagt | 230944 | 616047 | see also 2828 line | |
| 2830 | TRIP12 | NM_004238.1 | 2843 | aagttaactgatattttagtgt | 230946 | 616051 | see also 2828 line | |
| 2831 | TRIP12 | NM_004238.1 | 3080 | agccctcaaggtcgattaagtga | 230952 | 616056 | see also 2828 line | |
| 2832 | TRIP12 | NM_004238.1 | 3082 | ccctcaaggtcgattaagtgatg | 230953 | 616057 | see also 2828 line | |
| 2833 | TRIP12 | NM_004238.1 | 3088 | aggtcgattaagtgatgttctaa | 230954 | 616058 | see also 2828 line | |
| 2834 | TRIP12 | NM_004238.1 | 3203 | accactactcagtcacctaaatc | 230955 | 616065 | see also 2828 line | |
| 2835 | TRIP12 | NM_004238.1 | 3517 | tggagctgagtgccttgtagaaa | 230962 | 616073 | see also 2828 line | |
| 2836 | TRIP12 | NM_004238.1 | 3519 | gagctgagtgccttgtagaaatc | 230963 | 616074 | see also 2828 line | |
| 2837 | TRIP12 | NM_004238.1 | 3534 | tagaaatccgtagcatgtctca | 230964 | 616075 | see also 2828 line | |
| 2838 | TRIP12 | NM_004238.1 | 3762 | tagttcacagatgaacaactgc | 230969 | 616083 | see also 2828 line | |
| 2839 | TRIP12 | NM_004238.1 | 4276 | ttggacaaagactcatacaatat | 230983 | 616094 | see also 2828 line | |
| 2840 | TRIP12 | NM_004238.1 | 4277 | tggacaaagactcatacaatatg | 230984 | 616095 | see also 2828 line | |
| 2841 | TRIP12 | NM_004238.1 | 4474 | tcccacacctgaaaatatataa | 230991 | 616102 | see also 2828 line | |
| 2842 | TRIP12 | NM_004238.1 | 4561 | ctggtattacttgtatgataatg | 230997 | 616110 | see also 2828 line | |
| 2843 | TRIP12 | NM_004238.1 | 4562 | tggtattacttgtatgataatgc | 230998 | 616111 | see also 2828 line | |
| 2844 | TRIP12 | NM_004238.1 | 4823 | atcaaccagtctgattctcaaga | 231009 | 616132 | see also 2828 line | |
| 2845 | TRIP12 | NM_004238.1 | 5061 | gaggtgaagaagtaaactcttagc | 231019 | 616144 | see also 2828 line | |
| 2846 | TRIP12 | NM_004238.1 | 5178 | tcgcaaaggttaagatgaagtt | 231025 | 616147 | see also 2828 line | |

Figure 46

| 2847 | TRIP10 | NM_004240.1 | 661 | cagatgccccagatattcgataa | 136865 | 638243 | signal_transduction | protein binding | - |
|------|--------|-------------|-----|--------------------------|--------|--------|---------------------|------------------|---|
| 2848 | TRIP10 | NM_004240.1 | 666 | gccccagatattcgataagctcc | 136866 | 638244 | see also 2847 line | | |
| 2849 | TGM5 | NM_004245.1 | 224 | accccagaggcaggagtatgtca | 153650 | 736319 | - | protein-glutamine gamma-glutamyltransferase | - |
| 2850 | GLP2R | NM_004246.1 | 173 | tggtttccatcaagcaagttaca | 242669 | 630784 | gpcr、 receptor_acitivity、 signal_transduction | G-protein coupled receptor | - |
| 2851 | U5-116KD | NM_004247.1 | 393 | tacggtgtatgagatggatttct | 47430 | 477737 | - | translation elongation factor | - |
| 2852 | U5-116KD | NM_004247.1 | 1498 | aagatgtacagcacagatgatgg | 47454 | 477770 | see also 2851 line | | |
| 2853 | U5-116KD | NM_004247.1 | 2407 | ttgattcggaatgtcaagtttaa | 47477 | 477793 | see also 2851 line | | |
| 2854 | U5-116KD | NM_004247.1 | 2537 | gtctgatggagccttactacttt | 47480 | 477798 | see also 2851 line | | |
| 2855 | U5-116KD | NM_004247.1 | 2948 | aacaggatgttgtgctcaattac | 47486 | 477802 | see also 2851 line | | |
| 2856 | RAB9A | NM_004251.3 | 610 | gggacaacggcgactatccttat | 110407 | 520164 | signal_transduction | protein transporter | - |
| 2857 | PLAA | NM_004253.2 | 259 | tgctaaagtctggctgaatgaca | 298469 | 539012 | - | phospholipase A2 activator | - |
| 2858 | PLAA | NM_004253.2 | 658 | ccgacttccagctcagtctatat | 298483 | 539023 | see also 2857 line | | |
| 2859 | PLAA | NM_004253.2 | 906 | aaggacagactcgtctaatcaga | 298494 | 539031 | see also 2857 line | | |
| 2860 | PLAA | NM_004253.2 | 945 | aagcctatcagtggagtgttagt | 298495 | 539032 | see also 2857 line | | |
| 2861 | PLAA | NM_004253.2 | 2109 | cacttatcagtgatgattcaaat | 298538 | 539081 | see also 2857 line | | |
| 2862 | SEMA4F | NM_004263.2 | 528 | ttgtggcaccttcgcttttgatc | 251299 | 637591 | - | receptor | - |
| 2863 | SEMA4F | NM_004263.2 | 550 | ccgaagtgcgggggttattgatgt | 251300 | 637593 | see also 2862 line | | |
| 2864 | SEMA4F | NM_004263.2 | 580 | ttccagcaggttgaaagacttga | 251301 | 637594 | see also 2862 line | | |
| 2865 | FADS2 | NM_004265.2 | 1244 | acgtggagcagtccttcttcaac | 226740 | 609869 | - | - | - |
| 2866 | CRSP6 | NM_004268.3 | 1458 | gtcctcaagcttttgataaaaat | 248300 | 755134 | receptor_acitivity、 transcription_regulator、 signal_transduction | - | - |
| 2867 | CHST3 | NM_004273.2 | 875 | ctcgttcgtgggcgagttcttca | 217387 | 605580 | - | - | - |
| 2868 | EEF1E1 | NM_004280.2 | 456 | tcgctggttttgtcacattcagc | 302335 | 477476 | - | - | - |
| 2869 | BAG3 | NM_004281.2 | 568 | cagctccgaccaggctacattcc | 129134 | 532422 | apoptosis | protein binding | - |
| 2870 | GTPBP1 | NM_004286.3 | 255 | agggatgcggagagaccatatat | 57562 | 477593 | - | translation elongation factor | - |
| 2871 | GTPBP1 | NM_004286.3 | 256 | gggatgcggagagaccatatatg | 57563 | 477594 | see also 2870 line | | |
| 2872 | GTPBP1 | NM_004286.3 | 259 | atgcggagagaccatatatgtca | 57564 | 477595 | see also 2870 line | | |
| 2873 | PSCDBP | NM_004288.2 | 503 | tgctaacgatagagactcttaat | 317024 | 681513 | - | - | - |

Figure 46

| | | | | | | | | | |
|---|---|---|---|---|---|---|---|---|---|
| 2874 | RNF14 | NM_004290.3 | 389 | gtggagaaaccaggatctatttg | 112369 | 525685 | transcription_regulator、signal_transduction | - | - |
| 2875 | GDA | NM_004293.1 | 199 | gtgagcgacagcggcaaaatagt | 156998 | 594093 | - | zinc binding | - |
| 2876 | GDA | NM_004293.1 | 808 | agccatataagtgaaaatcgtga | 157023 | 594116 | see also 2875 line | | |
| 2877 | RGS6 | NM_004296.3 | 382 | gtcgtcacaggtactgacattgt | 192384 | 586167 | gpcr、signal_transduction | regulator of G-protein signaling | - |
| 2878 | RGS6 | NM_004296.3 | 433 | gaggacccagttgaagcaataca | 192385 | 586170 | see also 2877 line | | |
| 2879 | RGS6 | NM_004296.3 | 824 | tgccaggctgtgtgaacacaaca | 192392 | 586173 | see also 2877 line | | |
| 2880 | GNA14 | NM_004297.2 | 649 | aagtgggaaaagcacctttatca | 109096 | 519425 | gpcr、signal_transduction | signal transducer | - |
| 2881 | GNA14 | NM_004297.2 | 727 | cacgaagctggtttaccaaaaca | 109097 | 519428 | see also 2880 line | | |
| 2882 | ALK | NM_004304.3 | 3740 | aacaatgaccccgaaatggatgg | 49740 | 493740 | receptor_acitivity、kinase_related、signal_transduction | transmembrane receptor protein tyrosine kinase | inflammatory myofibroblastic tumors、non-hodgkin lymphoma、anaplastic large cell lymphoma、inflammatory myofibroblastic tumor、Hodgkin's disease |
| 2883 | ALK | NM_004304.3 | 4974 | ggcctgtataccggataatgact | 49754 | 493759 | see also 2882 line | | |
| 2884 | ARHGDIA | NM_004309.3 | 412 | gtgtgggagtaccggataaaaatc | 129085 | 664958 | signal_transduction | protein binding | - |
| 2885 | ARHGDIA | NM_004309.3 | 417 | gagtaccggataaaaatctcttt | 129086 | 664959 | see also 2884 line | | |
| 2886 | ARRB2 | NM_004313.3 | 276 | ctgcaagctcaccgtgtacttgg | 314063 | 678812 | signal_transduction | - | - |
| 2887 | ASPH | NM_004318.2 | 2273 | atctttccggctgatattcatcg | 138130 | 712787 | - | - | - |
| 2888 | ATP2A1 | NM_004320.2 | 1288 | gggagaggtcttgaagaatgata | 72697 | 585605 | - | - | muscle contracture |
| 2889 | ATP2A1 | NM_004320.2 | 1671 | gcgtcatcgaccgctgtaactat | 72701 | 585615 | see also 2888 line | | |
| 2890 | ATP2A1 | NM_004320.2 | 1673 | gtcatcgaccgctgtaactatgt | 72702 | 585616 | see also 2888 line | | |
| 2891 | ATP2A1 | NM_004320.2 | 2961 | ttgaccccctgccgatgatcttc | 72706 | 585626 | see also 2888 line | | |
| 2892 | ATP2A1 | NM_004320.2 | 2964 | accccctgccgatgatcttcaag | 72707 | 585627 | see also 2888 line | | |
| 2893 | ATSV | NM_004321.3 | 2879 | ctcctttgatgaccagcattttg | 73906 | 505198 | - | - | - |
| 2894 | BAG1 | NM_004323.2 | 871 | aagctctctgcaaacttgatagg | 129121 | 532399 | apoptosis、signal_transduction | receptor signaling protein | breast cancer |
| 2895 | BAG1 | NM_004323.2 | 1063 | ctgagcggctgcagtctacaaac | 129124 | 532408 | see also 2894 line | | |
| 2896 | BCS1L | NM_004328.3 | 307 | ccctactttggggctggatttgg | 73985 | 718831 | - | - | tubulopathy、encephalopathy、and liver failure due to complex III deficiency、gracile syndrome |
| 2897 | BMPR1A | NM_004329.1 | 674 | tagaatgttgtcggaccaattta | 74106 | 496133 | receptor_acitivity、kinase_related、signal_transduction | transforming growth factor-beta receptor | Alzheimer disease、polyposis |

Figure 46

| | | | | | | | | | |
|---|---|---|---|---|---|---|---|---|---|
| 2898 | BUB1 | NM_004336.1 | 1794 | ggggtattcgctgcaacaaaacc | 74436 | 496573 | kinase_related、cell_cycle | protein serine/threonine kinase | colorectal cancer |
| 2899 | BUB1 | NM_004336.1 | 2886 | acctgggtcagagtatagatatg | 74463 | 496607 | see also 2898 line | | |
| 2900 | BUB1 | NM_004336.1 | 2890 | gggtcagagtatagatatgaaac | 74465 | 496608 | see also 2898 line | | |
| 2901 | C8orf1 | NM_004337.1 | 1211 | gagctgctataaacaaaggaaag | 278089 | 758650 | - | molecular_function unknown | - |
| 2902 | C8orf1 | NM_004337.1 | 1333 | gtgtttcgcagacgagtaactga | 278093 | 758653 | see also 2901 line | | |
| 2903 | CETN2 | NM_004344.1 | 532 | tcctgcgcatcatgaaaaagacc | 140697 | 546329 | - | calcium ion binding | - |
| 2904 | RUNX2 | NM_004348.1 | 498 | aaggttcaacgatctgagatttg | 10851 | 466626 | transcription_regulator | RNA polymerase II transcription factor | cleido cranial dysplasia |
| 2905 | RUNX2 | NM_004348.1 | 499 | aggttcaacgatctgagatttgt | 10852 | 466627 | see also 2904 line | | |
| 2906 | RUNX2 | NM_004348.1 | 798 | aggacagagtcagattacagacc | 10857 | 466632 | see also 2904 line | | |
| 2907 | RUNX2 | NM_004348.1 | 1278 | cagcactccatatctctactatg | 10863 | 466638 | see also 2904 line | | |
| 2908 | RUNX2 | NM_004348.1 | 1393 | ggcagcacgctattaaatccaaa | 10864 | 466644 | see also 2904 line | | |
| 2909 | RUNX2 | NM_004348.1 | 1398 | cacgctattaaatccaaatttgc | 10865 | 466646 | see also 2904 line | | |
| 2910 | CBFA2T1 | NM_004349.2 | 1120 | agcaagcgaccatgcactattag | 1976 | 442547 | transcription_regulator | - | leukemia |
| 2911 | CBFA2T1 | NM_004349.2 | 1147 | ggccagcggtacagtccaaataa | 1977 | 442548 | see also 2910 line | | |
| 2912 | CBFA2T1 | NM_004349.2 | 1148 | gccagcggtacagtccaaataac | 1978 | 442549 | see also 2910 line | | |
| 2913 | CCNG2 | NM_004354.1 | 259 | gaggctaccccggagaatgataa | 306751 | 673724 | cell_cycle | cyclin-dependent protein kinase、regulator | - |
| 2914 | CCNG2 | NM_004354.1 | 260 | aggctaccccggagaatgataac | 306752 | 673725 | see also 2913 line | | |
| 2915 | CCNG2 | NM_004354.1 | 261 | ggctaccccggagaatgataaca | 306753 | 673726 | see also 2913 line | | |
| 2916 | CCNG2 | NM_004354.1 | 265 | accccggagaatgataacacttt | 306754 | 673727 | see also 2913 line | | |
| 2917 | CCNG2 | NM_004354.1 | 266 | ccccggagaatgataacactttg | 306755 | 673728 | see also 2913 line | | |
| 2918 | CCNG2 | NM_004354.1 | 267 | cccggagaatgataacactttgt | 306756 | 673729 | see also 2913 line | | |
| 2919 | CD151 | NM_004357.3 | 704 | gagaccatgcctccaacatctac | 318647 | 691950 | - | - | - |
| 2920 | CDH7 | NM_004361.2 | 1089 | ggctaaagaccagtatttgcttg | 140276 | 545872 | - | - | - |
| 2921 | CDH7 | NM_004361.2 | 1123 | aaggatatggttggtcaaaatgg | 140277 | 545874 | see also 2920 line | | |
| 2922 | CDH7 | NM_004361.2 | 1959 | atccaatggacaccagtttact | 140298 | 545905 | see also 2920 line | | |
| 2923 | CDH7 | NM_004361.2 | 2679 | cagctcaaactctgatcagaact | 140312 | 545923 | see also 2920 line | | |
| 2924 | CDH7 | NM_004361.2 | 2762 | gccaagagagtttgtactcatag | 140316 | 545928 | see also 2920 line | | |
| 2925 | CLGN | NM_004362.1 | 262 | ctcaacctataggagaagtatat | 137485 | 542601 | - | chaperone | - |
| 2926 | CLGN | NM_004362.1 | 554 | tggaggtgcatacattaaactcc | 137497 | 542604 | see also 2925 line | | |
| 2927 | CLCN2 | NM_004366.2 | 2437 | ctgggagtggaccatgcttatgt | 263258 | 646907 | channel | voltage-gated chloride channel | epilepsy、myoclonic epilepsy |

Figure 46

| 2928 | CLCN2 | NM_004366.2 | 2439 | gggagtggaccatgcttatgtca | 263259 | 646908 | see also 2927 line | | |
| 2929 | COL12A1 | NM_004370.4 | 301 | cggatgggcctactaaagaattt | 287699 | 695871 | - | - | - |
| 2930 | COL12A1 | NM_004370.4 | 6370 | aacctgacactccatataaaatt | 287853 | 696040 | see also 2929 line | | |
| 2931 | COL12A1 | NM_004370.4 | 6371 | acctgacactccatataaaatta | 287854 | 696041 | see also 2929 line | | |
| 2932 | COL12A1 | NM_004370.4 | 6373 | ctgacactccatataaaattact | 287855 | 696042 | see also 2929 line | | |
| 2933 | COPA | NM_004371.2 | 2752 | tgctacccatggcttagatgaag | 169779 | 700267 | - | protein transporter | - |
| 2934 | COPA | NM_004371.2 | 3951 | tgcacaacccctttgacatttgt | 169800 | 700289 | see also 2933 line | | |
| 2935 | CRABP1 | NM_004378.1 | 104 | gcgcagcagcgagaatttcgacg | 173398 | 570068 | signal_transduction | transporter | - |
| 2936 | CREB1 | NM_004379.2 | 785 | cagccgggtactaccattctaca | 2399 | 442805 | transcription_regulator、signal_transduction | - | Parkinson disease |
| 2937 | CREB1 | NM_004379.2 | 842 | gtgcccagcaaccaagttgttgt | 2401 | 442807 | see also 2936 line | | |
| 2938 | CREB1 | NM_004379.2 | 994 | gagagaggtccgtctaatgaaga | 2406 | 442810 | see also 2936 line | | |
| 2939 | CREBL1 | NM_004381.3 | 984 | cccgcctgaagtggatgcaaagc | 24607 | 457405 | transcription_regulator | translation regulator | - |
| 2940 | CRHR1 | NM_004382.2 | 795 | tggtgacagccgcctacaactac | 242048 | 630460 | gpcr、receptor_acitivity | corticotrophin-releasing factor receptor | alcoholism |
| 2941 | CSK | NM_004383.1 | 431 | gcctggccatccggtacagaatg | 77617 | 498857 | kinase_related、cell_cycle | protein tyrosine kinase | colorectal cancer、breast cancer、colorectal cancers |
| 2942 | CSK | NM_004383.1 | 434 | tggccatccggtacagaatgtat | 77618 | 498858 | see also 2941 line | | |
| 2943 | CSK | NM_004383.1 | 789 | tggagcactaccgcatcatgtac | 77623 | 498860 | see also 2941 line | | |
| 2944 | CSNK1G3 | NM_004384.1 | 184 | ggcaattttggagaattacgatt | 77705 | 793629 | kinase_related、signal_transduction | protein serine/threonine kinase | - |
| 2945 | CSNK1G3 | NM_004384.1 | 998 | atgactggattggtaaacagttg | 77730 | 488995 | see also 2944 line | | |
| 2946 | CSNK1G3 | NM_004384.1 | 1049 | atcctgctctgtcatcaaacaga | 77731 | 488997 | see also 2944 line | | |
| 2947 | CSNK1G3 | NM_004384.1 | 1208 | tacaggttgtaagttctacaaat | 77735 | 489000 | see also 2944 line | | |
| 2948 | CSPG2 | NM_004385.2 | 1235 | aaccctgtatcgttttgagaacc | 140996 | 546665 | - | lectin | Alzheimer disease |
| 2949 | CSPG2 | NM_004385.2 | 4300 | atccaatagattcagaatctaaa | 141092 | 546669 | see also 2948 line | | |
| 2950 | CSPG2 | NM_004385.2 | 9624 | agcggagaccagtgtgaacttga | 141241 | 546816 | see also 2948 line | | |
| 2951 | CTNNA2 | NM_004389.1 | 2518 | gggggcagggctagtcaactttc | 287025 | 660477 | - | structural constituent of cytoskeleton | - |
| 2952 | CYP8B1 | NM_004391.1 | 465 | tggctttccggaagaatatgttt | 221144 | 609201 | - | steroid hormone receptor | - |
| 2953 | DACH | NM_004392.2 | 1234 | gacttcgagaccctctacaatga | 310561 | 676504 | transcription_regulator | - | - |
| 2954 | DNCI1 | NM_004411.1 | 528 | caggcgatctggggccattaaca | 299916 | 534644 | - | motor | - |

196

Figure 46

| | | | | | | | | | |
|---|---|---|---|---|---|---|---|---|---|
| 2955 | DNCI1 | NM_004411.1 | 530 | ggcgatctggggccattaacaag | 299917 | 534645 | see also 2954 line | | |
| 2956 | DNMT2 | NM_004412.3 | 1058 | tacttaaactgcgatatttcact | 25427 | 458564 | - | - | - |
| 2957 | DUSP1 | NM_004417.2 | 858 | atcaacgtctcagccaattgtcc | 195078 | 588306 | phosphatase、 kinase_related、 cell_cycle | USF43/USF44 complex | - |
| 2958 | ECM1 | NM_004425.2 | 613 | gcggccttctccagacaatctga | 260490 | 644044 | kinase_related | - | lipoid proteinosis of Urbach and Wiethe |
| 2959 | PHC2 | NM_004427.2 | 418 | ccccccagaatggtgagaataaa | 134635 | 538642 | - | - | - |
| 2960 | PHC2 | NM_004427.2 | 463 | cccaaatcctgacgcatgttatc | 134637 | 538643 | see also 2959 line | | |
| 2961 | PHC2 | NM_004427.2 | 468 | atcctgacgcatgttatcgaagg | 134638 | 538644 | see also 2959 line | | |
| 2962 | EFNA1 | NM_004428.2 | 471 | aggacacagctactactacatct | 171737 | 694574 | - | - | - |
| 2963 | EGR3 | NM_004430.2 | 865 | cgcctattcccccaggattacc | 2967 | 459042 | transcription_regulator | transcription factor | - |
| 2964 | ELAVL2 | NM_004432.1 | 780 | gagtcctgtaagcttgtaagaga | 47566 | 473710 | transcription_regulator | mRNA binding、 3' UTR | - |
| 2965 | ELAVL2 | NM_004432.1 | 1076 | atcaaggggtgtagggtttattc | 47578 | 473723 | see also 2964 line | | |
| 2966 | ELAVL2 | NM_004432.1 | 1080 | aggggtgtagggtttattcgatt | 47579 | 473724 | see also 2964 line | | |
| 2967 | ELAVL2 | NM_004432.1 | 1081 | ggggtgtagggtttattcgattt | 47580 | 473725 | see also 2964 line | | |
| 2968 | ELAVL2 | NM_004432.1 | 1087 | tagggtttattcgatttgacaag | 47581 | 473727 | see also 2964 line | | |
| 2969 | ELAVL2 | NM_004432.1 | 1451 | gagtatcctgtggcaaatgtttg | 47586 | 473742 | see also 2964 line | | |
| 2970 | EPHA4 | NM_004438.2 | 1358 | cagcaccatcatccattgctttg | 81468 | 501708 | - | ephrin receptor | - |
| 2971 | EPHA4 | NM_004438.2 | 2919 | cacgcaccagaataagattttga | 81492 | 501743 | see also 2970 line | | |
| 2972 | EPHA5 | NM_004439.3 | 1700 | ttcagtcgaagatttgagtttga | 81532 | 501772 | receptor_acitivity、 kinase_related、 signal_transduction | - | - |
| 2973 | EPHA5 | NM_004439.3 | 1884 | ttgcccatccaagcctaatatgg | 81534 | 501775 | see also 2972 line | | |
| 2974 | EPHA7 | NM_004440.2 | 295 | caggctgcgaaggaagtactact | 81557 | 501894 | receptor_acitivity、 signal_transduction、 kinase_related | ephrin receptor | - |
| 2975 | EPHA7 | NM_004440.2 | 464 | ggctgcggactaactggatttcc | 81563 | 501901 | see also 2974 line | | |
| 2976 | EPHA7 | NM_004440.2 | 772 | caggatgtaggggcttgcatagc | 81572 | 501912 | see also 2974 line | | |
| 2977 | EPHA7 | NM_004440.2 | 1709 | cagcctccattaataatctgaaa | 81601 | 501941 | see also 2974 line | | |
| 2978 | EPHA7 | NM_004440.2 | 1710 | agcctccattaataatctgaaac | 81602 | 501942 | see also 2974 line | | |
| 2979 | EPHA7 | NM_004440.2 | 2050 | acctatgaggacccaaatagagc | 81613 | 501951 | see also 2974 line | | |
| 2980 | EPHB1 | NM_004441.2 | 2937 | cagacttcacggcctttaccacc | 81689 | 502033 | receptor_acitivity、 signal_transduction、 kinase_related | ephrin receptor | - |
| 2981 | EPS8 | NM_004447.3 | 545 | aagtgtggactcaagatatgatt | 248437 | 696421 | signal_transduction | SH3/SH2 adaptor protein | - |

Figure 46

| | | | | | | | | | |
|---|---|---|---|---|---|---|---|---|---|
| 2982 | EPS8 | NM_004447.3 | 549 | gtggactcaagatatgattcttc | 248438 | 696423 | | | see also 2981 line |
| 2983 | EPS8 | NM_004447.3 | 758 | aggttaaggcaaacctaattagt | 248444 | 696426 | | | see also 2981 line |
| 2984 | ERG | NM_004449.2 | 661 | aacctccacggttaatgcatgc | 3401 | 443484 | transcription_regulator, signal_transduction | - | leukemia, Ewing sarcoma, Ewing's sarcoma, Ewing's tumours |
| 2985 | ETFDH | NM_004453.1 | 1403 | taggactccatgtaactgaatat | 225783 | 614140 | - | electron-transferring-flavoprotein dehydrogenase | glutaricaciduria |
| 2986 | ETFDH | NM_004453.1 | 1404 | aggactccatgtaactgaatatg | 225784 | 614141 | see also 2985 line | | |
| 2987 | ETFDH | NM_004453.1 | 1418 | ctgaatatgaggacaatttgaag | 225785 | 614143 | see also 2985 line | | |
| 2988 | ETFDH | NM_004453.1 | 1692 | gagtggtactaatcatgaacatg | 225797 | 614156 | see also 2985 line | | |
| 2989 | ETFDH | NM_004453.1 | 1695 | tggtactaatcatgaacatgacc | 225798 | 614157 | see also 2985 line | | |
| 2990 | EZH2 | NM_004456.3 | 205 | ttgttggcggaagcgtgtaaaat | 26359 | 459433 | - | - | - |
| 2991 | EZH2 | NM_004456.3 | 292 | tagttccaatcgtcagaaaattt | 26361 | 459437 | see also 2990 line | | |
| 2992 | EZH2 | NM_004456.3 | 543 | ctgtttacataacattccttat | 26368 | 459451 | see also 2990 line | | |
| 2993 | EZH2 | NM_004456.3 | 847 | ttcctcaatgtttccagataagg | 26377 | 459458 | see also 2990 line | | |
| 2994 | EZH2 | NM_004456.3 | 1416 | ctcggtcaaacaccaataaag | 26387 | 459469 | see also 2990 line | | |
| 2995 | EZH2 | NM_004456.3 | 1564 | aacatgtagacaggtgtatgagt | 26391 | 459477 | see also 2990 line | | |
| 2996 | EZH2 | NM_004456.3 | 1567 | atgtagacaggtgtatgagttta | 26392 | 459478 | see also 2990 line | | |
| 2997 | EZH2 | NM_004456.3 | 1713 | acggctcctcaaccatgtttac | 26395 | 459479 | see also 2990 line | | |
| 2998 | EZH2 | NM_004456.3 | 1951 | cgctgaccattgggacagtaaaa | 26396 | 459488 | see also 2990 line | | |
| 2999 | EZH2 | NM_004456.3 | 1953 | ctgaccattgggacagtaaaaat | 26397 | 459489 | see also 2990 line | | |
| 3000 | EZH2 | NM_004456.3 | 2268 | atgcaaaagttatgatgttaac | 26408 | 459505 | see also 2990 line | | |
| 3001 | FACL4 | NM_004458.1 | 543 | aagttaattcttgggaattataa | 165617 | 563167 | - | - | mental retardation |
| 3002 | FACL4 | NM_004458.1 | 1056 | gtgatgatgcatcatagcaattt | 165638 | 563181 | see also 3001 line | | |
| 3003 | FACL4 | NM_004458.1 | 1121 | gggaccgaaggacacacatatattg | 165641 | 563185 | see also 3001 line | | |
| 3004 | FACL4 | NM_004458.1 | 1168 | tagaactgacagcagagatatct | 165642 | 563187 | see also 3001 line | | |
| 3005 | FACL4 | NM_004458.1 | 1535 | tcctcagacacccgattcatga | 165654 | 563202 | see also 3001 line | | |
| 3006 | FACL4 | NM_004458.1 | 1537 | ctcagacacaccgattcatgaat | 165655 | 563203 | see also 3001 line | | |
| 3007 | FACL4 | NM_004458.1 | 1539 | cagacacaccgattcatgaatgt | 165656 | 563204 | see also 3001 line | | |
| 3008 | FACL4 | NM_004458.1 | 1541 | gacacaccgattcatgaatgtct | 165657 | 563205 | see also 3001 line | | |
| 3009 | FACL4 | NM_004458.1 | 1890 | cagattatgatcgtaagaaaga | 165671 | 563218 | see also 3001 line | | |
| 3010 | FAP | NM_004460.2 | 671 | tggtcgcctgttgggagtaaatt | 183459 | 577276 | - | prolyl oligopeptidase | melanoma |
| 3011 | FAP | NM_004460.2 | 673 | gtcgcctgttgggagtaaattag | 183460 | 577277 | see also 3010 line | | |
| 3012 | FDFT1 | NM_004462.2 | 217 | ttgctacaagtatctcaatcaga | 165763 | 563240 | - | oxidoreductase | - |
| 3013 | FGD1 | NM_004463.2 | 1052 | gggaaaccggatcctgttaaaa | 156797 | 557695 | signal_transduction | zinc binding | mental retardation, faciogenital dysplasia, Aarskog-Scott syndrome |

Figure 46

EP 1 752 536 A1

| 3014 | FGD1 | NM_004463.2 | 1058 | ccggatcctggttaaaagtttgt | 156798 | 557698 | see also 3013 line | | |
|---|---|---|---|---|---|---|---|---|---|
| 3015 | FGD1 | NM_004463.2 | 1984 | ttgtccacggcatcttctctaac | 156803 | 557705 | see also 3013 line | | |
| 3016 | FGD1 | NM_004463.2 | 1987 | tccacggcatcttctctaacatc | 156804 | 557706 | see also 3013 line | | |
| 3017 | FGD1 | NM_004463.2 | 2634 | agcgtgcgggcacgcattgatgt | 156815 | 557725 | see also 3013 line | | |
| 3018 | FGD1 | NM_004463.2 | 2637 | gtgcgggcacgcattgatgtaga | 156816 | 557726 | see also 3013 line | | |
| 3019 | FGD1 | NM_004463.2 | 2639 | gcgggcacgcattgatgtagatg | 156817 | 557727 | see also 3013 line | | |
| 3020 | FGD1 | NM_004463.2 | 2640 | cgggcacgcattgatgtagatgg | 156818 | 557728 | see also 3013 line | | |
| 3021 | FGF5 | NM_004464.2 | 457 | tacccggatggcaaagtcaatgg | 169144 | 565956 | cell_cycle、signal_transduction | - | - |
| 3022 | FGF5 | NM_004464.2 | 529 | caggggattgtaggaatacgagg | 169150 | 565960 | see also 3021 line | | |
| 3023 | FGF5 | NM_004464.2 | 539 | taggaatacgaggagttttcagc | 169151 | 565961 | see also 3021 line | | |
| 3024 | FGF10 | NM_004465.1 | 193 | aggcatgtgcggagctacaatca | 169028 | 565814 | signal_transduction、cell_cycle、transcription_regulator | growth factor | - |
| 3025 | FLOT2 | NM_004475.1 | 159 | gacggccgagggggtagctttaa | 319055 | 683493 | - | - | - |
| 3026 | FLOT2 | NM_004475.1 | 160 | acggccgagggggtagctttaac | 319056 | 683494 | see also 3025 line | | |
| 3027 | FLOT2 | NM_004475.1 | 161 | cggccgagggggtagctttaact | 319057 | 683495 | see also 3025 line | | |
| 3028 | FOLH1 | NM_004476.1 | 798 | tgcacgaactgaagacttcttta | 183738 | 577603 | - | serine-type endopeptidase | - |
| 3029 | FRG1 | NM_004477.1 | 925 | tggacaggagagccaaattgaaa | 314795 | 704395 | - | - | - |
| 3030 | FUT8 | NM_004480.3 | 1129 | ctcagaattggcgctatgctact | 210747 | 599637 | - | - | - |
| 3031 | FUT8 | NM_004480.3 | 1455 | ggcttcaaacatccagttattgg | 210755 | 599644 | see also 3030 line | | |
| 3032 | FUT8 | NM_004480.3 | 1666 | atgaatttattagtgataactct | 210761 | 599650 | see also 3030 line | | |
| 3033 | GALNT2 | NM_004481.2 | 51 | gggcatcgcctactacatgtact | 210966 | 606538 | - | - | - |
| 3034 | GALNT2 | NM_004481.2 | 963 | ggggaagtacgacatgatgatgg | 210982 | 606551 | see also 3033 line | | |
| 3035 | GALNT3 | NM_004482.2 | 664 | ttggtggatgatgctagtgtaga | 211022 | 606578 | - | - | diabetes |
| 3036 | GALNT3 | NM_004482.2 | 1044 | aagaaggaaagatgaaacctacc | 211036 | 606586 | see also 3035 line | | |
| 3037 | GALNT3 | NM_004482.2 | 1486 | gtgtatgtgccagaccttaatcc | 211044 | 606603 | see also 3035 line | | |
| 3038 | GALNT3 | NM_004482.2 | 1494 | gccagaccttaatcctgttatat | 211045 | 606604 | see also 3035 line | | |
| 3039 | GALNT3 | NM_004482.2 | 1496 | cagaccttaatcctgttatatct | 211046 | 606605 | see also 3035 line | | |
| 3040 | GPC3 | NM_004484.2 | 790 | agcaagacgtgacctgaaagtat | 310629 | 684905 | receptor_acitivity | - | neuroblastoma、Simpson-Golabi-Behmel overgrowth syndrome、Wilms tumor、Simpson-Golabi-Behmel Syndrome |
| 3041 | GPC3 | NM_004484.2 | 1107 | ttgtgaatggcatgtacagaatc | 310638 | 684917 | see also 3040 line | | |
| 3042 | GPC3 | NM_004484.2 | 1759 | tgcagaactggcctatgatctgg | 310662 | 684937 | see also 3040 line | | |
| 3043 | GTF2A2 | NM_004492.1 | 245 | aagttctacttcagtttgataag | 4530 | 816107 | - | RNA polymerase II transcription factor | - |

199

Figure 46

| 3044 | HDGF | NM_004494.1 | 432 | atcaacagccaacaaataccaag | 169233 | 566033 | signal_transduction | heparin binding | - |
|------|------|-------------|-----|--------------------------|--------|--------|---------------------|-----------------|---|
| 3045 | HDGF | NM_004494.1 | 671 | cagagggtgacggtgataagaag | 169234 | 566035 | see also 3044 line | | |
| 3046 | FOXA3 | NM_004497.2 | 1190 | tggggagcctggagtctactacc | 4018 | 443909 | transcription_regulator | translation regulator | - |
| 3047 | ONECUT1 | NM_004498.1 | 1316 | gcgtcgaactctacatgcaatat | 8765 | 447510 | transcription_regulator | translation regulator | - |
| 3048 | ONECUT1 | NM_004498.1 | 1318 | gtcgaactctacatgcaatattc | 8766 | 447511 | see also 3047 line | | |
| 3049 | ONECUT1 | NM_004498.1 | 1319 | tcgaactctacatgcaatattca | 8767 | 447512 | see also 3047 line | | |
| 3050 | HNRPAB | NM_004499.2 | 415 | aggaggacgcgggaaaaatgttc | 52014 | 460295 | - | - | - |
| 3051 | HNRPAB | NM_004499.2 | 629 | ccgtgtcattgaccctaaaaagg | 52022 | 460301 | see also 3050 line | | |
| 3052 | HNRPAB | NM_004499.2 | 1004 | tggaggtcagggtagtacaaact | 52030 | 460306 | see also 3050 line | | |
| 3053 | HNRPU | NM_004501.2 | 1691 | aactacctgggttactaaacatg | 28614 | 460353 | - | - | - |
| 3054 | HOXC6 | NM_004503.2 | 174 | tccccaacgtcgccctcaattcc | 5181 | 444817 | transcription_regulator | - | - |
| 3055 | HOXC6 | NM_004503.2 | 257 | atctactcgactcccttttattc | 5183 | 444819 | see also 3054 line | | |
| 3056 | HOXC6 | NM_004503.2 | 317 | ccgtatgactatggatctaattc | 5184 | 444821 | see also 3054 line | | |
| 3057 | HOXC6 | NM_004503.2 | 404 | acctcaatcgctcaggattttag | 5186 | 444824 | see also 3054 line | | |
| 3058 | HRB | NM_004504.3 | 418 | agggatccacaaaaagtgaaaga | 28674 | 460451 | - | RNA binding | - |
| 3059 | HRB | NM_004504.3 | 743 | cagctacagccaattttgctaac | 28678 | 460455 | see also 3058 line | | |
| 3060 | HRB | NM_004504.3 | 744 | agctacagccaattttgctaact | 28679 | 460456 | see also 3058 line | | |
| 3061 | HRB | NM_004504.3 | 840 | gtctagtggttcgagtaattttg | 28681 | 460460 | see also 3058 line | | |
| 3062 | HRB | NM_004504.3 | 1080 | agcacttgctaatttagacaata | 28689 | 460469 | see also 3058 line | | |
| 3063 | HSF2 | NM_004506.2 | 617 | aagattgtccagtttattgttac | 5351 | 444916 | transcription_regulator | translation regulator | - |
| 3064 | HSF2 | NM_004506.2 | 673 | acgtaaaaggcctctacttctaa | 5353 | 444920 | see also 3063 line | | |
| 3065 | ILK | NM_004517.1 | 625 | cagaatctcaaccgtattccata | 85718 | 504293 | kinase_related、signal_transduction | protein serine/threonine kinase | - |
| 3066 | KCNQ3 | NM_004519.2 | 1505 | tggatcggggttcgcctttctaat | 261064 | 644518 | channel | voltage-gated potassium channel | benign familial neonatal convulsions(-)、epilepsy |
| 3067 | KCNQ3 | NM_004519.2 | 1526 | atcctcgtggtagcaatactaaa | 261066 | 644519 | see also 3066 line | | |
| 3068 | KCNQ3 | NM_004519.2 | 1527 | tcctcgtggtagcaatactaaag | 261067 | 644520 | see also 3066 line | | |
| 3069 | KCNQ3 | NM_004519.2 | 1552 | aagctatttacccctctgaatgt | 261071 | 644521 | see also 3066 line | | |
| 3070 | KCNQ3 | NM_004519.2 | 2074 | agcatgatggggaagtttgtaaa | 261087 | 644532 | see also 3066 line | | |
| 3071 | KCNQ3 | NM_004519.2 | 2258 | ccgatttgaaaaccatcatctgc | 261090 | 644536 | see also 3066 line | | |
| 3072 | KIF2 | NM_004520.1 | 456 | gagattgcaacagcaagaactta | 87252 | 505699 | - | motor | - |
| 3073 | KIF2 | NM_004520.1 | 1699 | cagagagatgatctaaaacttct | 87279 | 505744 | see also 3072 line | | |
| 3074 | KIF2 | NM_004520.1 | 1701 | gagagatgatctaaaacttcttt | 87280 | 505745 | see also 3072 line | | |

Figure 46

| 3075 | KIF5B | NM_004521.1 | 596 | atggagggtaaacttcatgatcc | 87732 | 505938 | - | microtubule motor | - |
|---|---|---|---|---|---|---|---|---|---|
| 3076 | KIF5B | NM_004521.1 | 615 | atccagaaggcatgggaattatt | 87733 | 505939 | see also 3075 line | | |
| 3077 | KIF5B | NM_004521.1 | 621 | aaggcatgggaattattccaaga | 87735 | 505940 | see also 3075 line | | |
| 3078 | KIF5B | NM_004521.1 | 1823 | cagaagtctcaggaagttgaaga | 87771 | 505968 | see also 3075 line | | |
| 3079 | KIF5B | NM_004521.1 | 2074 | cactgttgcaagactctacatta | 87777 | 505977 | see also 3075 line | | |
| 3080 | KIF5B | NM_004521.1 | 2079 | ttgcaagactctacattagcaaa | 87779 | 505978 | see also 3075 line | | |
| 3081 | KIF5C | NM_004522.1 | 1097 | tgccgagggagctgttcttgacg | 87810 | 506004 | - | microtubule motor | - |
| 3082 | MLLT3 | NM_004529.1 | 485 | aagtccgctttgattatgactta | 300617 | 669106 | transcription_regulator | translation regulator | leukemia |
| 3083 | MLLT3 | NM_004529.1 | 487 | gtccgctttgattatgacttatt | 300618 | 669107 | see also 3082 line | | |
| 3084 | MLLT3 | NM_004529.1 | 859 | tccagggatcacaacaaatcttc | 300628 | 669119 | see also 3082 line | | |
| 3085 | MLLT3 | NM_004529.1 | 1763 | tgcagcagatcgtgaaccttata | 300654 | 669131 | see also 3082 line | | |
| 3086 | MMP2 | NM_004530.1 | 606 | atgtggccaactacaacttcttc | 148556 | 550280 | - | Transcription factor AP-2 alpha | hepatocellular carcinoma、 oral cavity cancers、 chondrosarcoma、 colorectal cancer、 breast cancer、 Parkinson disease、 CNS Tumours、 melanoma、 colorectal cancers、 osteosarcoma |
| 3087 | MOCS2 | NM_004531.3 | 638 | tagggactacaagaaataacttt | 236842 | 671631 | - | - | molybdenum cofactor deficiency |
| 3088 | MOCS2 | NM_004531.3 | 892 | gccaaggtgcccatatggaaaaa | 236854 | 671641 | see also 3087 line | | |
| 3089 | MYT1 | NM_004535.1 | 2113 | cacctttgactacgcaagtttcg | 7367 | 446357 | transcription_regulator | zinc binding | - |
| 3090 | NAP1L1 | NM_004537.3 | 151 | gtctgaacttgatcaagatttgg | 33401 | 462273 | - | - | - |
| 3091 | NAP1L1 | NM_004537.3 | 742 | tggccagcctatgagttttgtct | 33413 | 462285 | see also 3090 line | | |
| 3092 | NAP1L1 | NM_004537.3 | 893 | gggtgccagatagattggaaaaa | 33415 | 462290 | see also 3090 line | | |
| 3093 | NAP1L1 | NM_004537.3 | 973 | gacagttcgtactgtgactaaaa | 33416 | 462293 | see also 3090 line | | |
| 3094 | NAP1L3 | NM_004538.2 | 1552 | cagccaagagtggttcctaatgc | 33489 | 462376 | - | DNA binding | - |
| 3095 | NARS | NM_004539.2 | 482 | ctgcgcaggcaaggaaagaattt | 64738 | 485221 | - | nucleic acid binding | - |
| 3096 | NARS | NM_004539.2 | 921 | cactcttcaagcttgactatttt | 64750 | 485233 | see also 3095 line | | |
| 3097 | NCAM2 | NM_004540.2 | 316 | gtgacaatttcacttagcaaagt | 317891 | 683610 | - | - | - |
| 3098 | NCAM2 | NM_004540.2 | 554 | cacaagaagctacagtagttttg | 317900 | 683614 | see also 3097 line | | |
| 3099 | NCAM2 | NM_004540.2 | 1976 | gacaaggagactacagtaaaata | 317952 | 683657 | see also 3097 line | | |
| 3100 | NFATC4 | NM_004554.3 | 704 | ggggacggctctcctagagatta | 7667 | 446605 | transcription_regulator | translation regulator | - |
| 3101 | NOTCH4 | NM_004557.2 | 756 | aaggcacctcctgccataacacc | 149267 | 550636 | receptor_acitivity、 signal_transduction、 transcription_regulator | translation regulator | breast cancer、 schizophrenia |
| 3102 | NOTCH4 | NM_004557.2 | 3926 | cacaacgggcactgtgagaaagg | 149280 | 550649 | see also 3101 line | | |

Figure 46

| | | | | | | | | | |
|---|---|---|---|---|---|---|---|---|---|
| 3103 | PET112L | NM_004564.1 | 386 | ctctgaactgccacataaacaag | 57936 | 617352 | - | translation factor、 nucleic acid binding | - |
| 3104 | PET112L | NM_004564.1 | 784 | ttgagagtggatgccaatatatc | 57948 | 617359 | see also 3103 line | | |
| 3105 | PEX14 | NM_004565.1 | 311 | aggctcccgatggcgagattacg | 323685 | 688621 | - | - | - |
| 3106 | PFKFB4 | NM_004567.2 | 515 | tgccaacatcgtgcaagtgaaac | 96640 | 621814 | kinase_related、 phosphatase | fructose-2、6-bisphosphate 2-phosphatase | - |
| 3107 | PIK3C2G | NM_004570.2 | 2815 | tgccattgaagattactttcatc | 207123 | 597158 | kinase_related | phosphatidylinositol 3-kinase、class III | - |
| 3108 | PIK3C2G | NM_004570.2 | 2816 | gccattgaagattactttcatca | 207124 | 597159 | see also 3107 line | | |
| 3109 | PIK3C2G | NM_004570.2 | 4191 | gcgcccagtgcacatgttgaatt | 207162 | 597192 | see also 3107 line | | |
| 3110 | PKNOX1 | NM_004571.3 | 209 | aagtatagacagctatcaagatg | 9365 | 441316 | transcription_regulator | - | - |
| 3111 | PNUTL2 | NM_004574.2 | 929 | ccggccattggatgttgaattca | 109850 | 520443 | cell_cycle、apoptosis | - | melanoma |
| 3112 | PNUTL2 | NM_004574.2 | 1389 | aaggaacggaatcgcaacaaact | 109856 | 520450 | see also 3111 line | | |
| 3113 | PNUTL2 | NM_004574.2 | 1393 | aacggaatcgcaacaaactgact | 109857 | 520451 | see also 3111 line | | |
| 3114 | PPP2R2B | NM_004576.2 | 707 | ttcatcgtaggggtgaatacaat | 298688 | 666252 | phosphatase、signal_transduction | - | - |
| 3115 | PPP2R2B | NM_004576.2 | 711 | tcgtaggggtgaatacaatgttt | 298689 | 666253 | see also 3114 line | | |
| 3116 | PPP2R2B | NM_004576.2 | 1394 | gccacagtgggaggtatatcatg | 298708 | 666266 | see also 3114 line | | |
| 3117 | RAB4A | NM_004578.2 | 494 | accagccgagaaacctacaatgc | 110310 | 520120 | signal_transduction | protein transporter | - |
| 3118 | RAB4A | NM_004578.2 | 505 | aacctacaatgcgcttactaatt | 110311 | 520121 | see also 3117 line | | |
| 3119 | RAB27A | NM_004580.3 | 472 | cagggcaggagaggtttcgtagc | 110077 | 519992 | signal_transduction | - | - |
| 3120 | RPS6KA3 | NM_004586.1 | 18 | ggcggacccgtggcagaagatgg | 99764 | 514239 | kinase_related、signal_transduction | protein tyrosine kinase | Coffin-Lowry syndrome(-)、diabetes mellitus、mental retardation、diabetes |
| 3121 | RPS6KA3 | NM_004586.1 | 342 | gacaaaaatggaacgtgatatct | 99766 | 514243 | see also 3120 line | | |
| 3122 | RPS6KA3 | NM_004586.1 | 886 | cagagtcttttacgaatgctttt | 99780 | 514256 | see also 3120 line | | |
| 3123 | RPS6KA3 | NM_004586.1 | 1139 | agcttttctcggggtttagtttt | 99786 | 514263 | see also 3120 line | | |
| 3124 | RPS6KA3 | NM_004586.1 | 1491 | aggaggtgaattgctggataaaa | 99796 | 514277 | see also 3120 line | | |
| 3125 | RPS6KA3 | NM_004586.1 | 1665 | atctattcgaatttgtgattttg | 99802 | 514287 | see also 3120 line | | |
| 3126 | RPS6KA3 | NM_004586.1 | 1966 | gtgtcaaagatgcttcatgtaga | 99813 | 514301 | see also 3120 line | | |
| 3127 | SSA2 | NM_004600.3 | 1158 | aaggctcgtatacatccatttca | 54951 | 475665 | transcription_regulator | RNA binding | Sjogren syndrome |
| 3128 | SSA2 | NM_004600.3 | 1160 | ggctcgtatacatccatttcata | 54952 | 475666 | see also 3127 line | | |
| 3129 | SSA2 | NM_004600.3 | 1762 | tggatatgtgcggctttgatact | 54963 | 475683 | see also 3127 line | | |
| 3130 | STAU | NM_004602.1 | 1646 | gcccacagccgagaccattttaa | 48776 | 472183 | - | - | - |
| 3131 | STAU | NM_004602.1 | 1647 | cccacagccgagaccattttaaa | 48777 | 472184 | see also 3130 line | | |
| 3132 | STAU | NM_004602.1 | 1649 | cacagccgagaccattttaaaga | 48778 | 472185 | see also 3130 line | | |

Figure 46

| | | | | | | | | | |
|---|---|---|---|---|---|---|---|---|---|
| 3133 | TGFBR1 | NM_004612.1 | 256 | gaccacagacaaagttatacaca | 102515 | 516315 | receptor_acitivity、kinase_related、signal_transduction | type I transforming growth factor-beta receptor | pancreatic cancer |
| 3134 | TGFBR1 | NM_004612.1 | 904 | ctggttggtgtcagattatcatg | 102541 | 516339 | see also 3133 line | | |
| 3135 | TOP3A | NM_004618.2 | 1473 | ctcccattcaccccaccaaatac | 42796 | 469782 | - | USF43/USF44 complex | Smith-Magenis syndrome chromosome region |
| 3136 | TRAF6 | NM_004620.2 | 1446 | ctcagcgctgtgcaaactatata | 67026 | 486995 | signal_transduction、kinase_related | - | - |
| 3137 | TRAF6 | NM_004620.2 | 1448 | cagcgctgtgcaaactatatatc | 67027 | 486996 | see also 3136 line | | |
| 3138 | TRAF6 | NM_004620.2 | 1527 | agggtacaatacgccttacaatt | 67029 | 487001 | see also 3136 line | | |
| 3139 | TRAF6 | NM_004620.2 | 1528 | gggtacaatacgccttacaattc | 67030 | 487002 | see also 3136 line | | |
| 3140 | TRAF6 | NM_004620.2 | 1531 | tacaatacgccttacaattcttg | 67031 | 487003 | see also 3136 line | | |
| 3141 | TRPC6 | NM_004621.3 | 1908 | tggagatgctcattatatcctgg | 261863 | 645090 | channel | non-selective cation channel | - |
| 3142 | TRPC6 | NM_004621.3 | 1925 | tcctgggtaataggcatgatatg | 261864 | 645092 | see also 3141 line | | |
| 3143 | WNT11 | NM_004626.2 | 182 | gcgtgtgctatggcatcaagtgg | 255418 | 639571 | signal_transduction | signal transducer | - |
| 3144 | FANCG | NM_004629.1 | 1258 | caccgtaagatgggaaatccaca | 16272 | 678757 | cell_cycle | - | aplastic anemia、Fanconi anemia |
| 3145 | SF1 | NM_004630.1 | 1950 | tgccatggcagcaaaatacgacg | 54694 | 470599 | transcription_regulator | - | - |
| 3146 | LRP8 | NM_004631.2 | 1880 | ccccctgcgagggttcatgtatt | 147953 | 537284 | receptor_acitivity、signal_transduction | - | - |
| 3147 | DAP3 | NM_004632.2 | 617 | tacaacaaacagcgctttgatca | 285443 | 658280 | apoptosis | - | - |
| 3148 | BRPF1 | NM_004634.1 | 3304 | atgccgaggctatccatcatacc | 23107 | 456735 | transcription_regulator | translation regulator | - |
| 3149 | MAPKAPK3 | NM_004635.3 | 1126 | ggccactatgcggggtagactacg | 90128 | 493610 | kinase_related、signal_transduction | - | - |
| 3150 | BAT2 | NM_004638.2 | 503 | tggttccaagcggggtaaagtcc | 332653 | 709975 | - | - | diabetes |
| 3151 | BAT2 | NM_004638.2 | 762 | cccggactccaaacttcatcatg | 332655 | 709977 | see also 3150 line | | |
| 3152 | BAT2 | NM_004638.2 | 1086 | ggcccatgaagaggttgactaca | 332664 | 709986 | see also 3150 line | | |
| 3153 | BAT3 | NM_004639.2 | 3580 | ggcagcagctccggtctgatata | 304896 | 532556 | - | - | - |
| 3154 | BAT1 | NM_004640.3 | 1014 | gtgctaccttgagcaaagagatc | 49793 | 481475 | - | - | - |
| 3155 | MLLT10 | NM_004641.1 | 215 | aggacgaggtctcccatagtatg | 6956 | 461797 | transcription_regulator | transcription factor | leukemia |
| 3156 | MLLT10 | NM_004641.1 | 218 | acgaggtctcccatagtatgaag | 6957 | 461798 | see also 3155 line | | |
| 3157 | MLLT10 | NM_004641.1 | 572 | atgatcgttataataagacttgc | 6963 | 461811 | see also 3155 line | | |
| 3158 | MLLT10 | NM_004641.1 | 636 | tggtgcttgcatgacatgtaata | 6966 | 461812 | see also 3155 line | | |
| 3159 | MLLT10 | NM_004641.1 | 638 | gtgcttgcatgacatgtaataaa | 6967 | 461813 | see also 3155 line | | |
| 3160 | MLLT10 | NM_004641.1 | 639 | tgcttgcatgacatgtaataaac | 6968 | 461814 | see also 3155 line | | |

Figure 46

| | | | | | | | | |
|---|---|---|---|---|---|---|---|---|
| 3161 | MLLT10 | NM_004641.1 | 642 | ttgcatgacatgtaataaacatg | 6969 | 461815 | see also 3155 line | |
| 3162 | MLLT10 | NM_004641.1 | 746 | tccaatactgtggctactgtaaa | 6977 | 461821 | see also 3155 line | |
| 3163 | PABPN1 | NM_004643.1 | 2115 | ctgattctacagtggtttaac | 53195 | 485345 | - | oculopharyngeal muscular dystrophy |
| 3164 | COIL | NM_004645.1 | 39 | cggttaggctacggcttcaattt | 324043 | 533869 | - | - |
| 3165 | USP11 | NM_004651.2 | 1147 | aagaacaaggttggccattttgc | 188792 | 582840 | - | ubiquitin C-terminal hydrolase |
| 3166 | USP9X | NM_004652.2 | 605 | tacacgtccatgtgaatcagttt | 190480 | 583957 | - | - |
| 3167 | USP9X | NM_004652.2 | 2567 | aagacaggaagctgttcgaatgg | 190505 | 583809 | see also 3166 line | |
| 3168 | USP9X | NM_004652.2 | 3246 | agcagcctagtccatctctga | 190520 | 583832 | see also 3166 line | |
| 3169 | USP9X | NM_004652.2 | 4147 | aacactcaggcgactactttact | 190534 | 583856 | see also 3166 line | |
| 3170 | USP9X | NM_004652.2 | 4149 | cactcaggcgactactttactct | 190535 | 583857 | see also 3166 line | |
| 3171 | USP9X | NM_004652.2 | 4153 | caggcgactacttttacttttta | 190537 | 583858 | see also 3166 line | |
| 3172 | USP9X | NM_004652.2 | 4154 | aggcgactactttactctttaa | 190538 | 583859 | see also 3166 line | |
| 3173 | USP9X | NM_004652.2 | 4155 | ggcgactacttttactctttaag | 190539 | 583860 | see also 3166 line | |
| 3174 | USP9X | NM_004652.2 | 4806 | ggcacaggtagtgatgtagatga | 190557 | 583875 | see also 3166 line | |
| 3175 | USP9X | NM_004652.2 | 6245 | ttggtatgatgcattgtgtattc | 190574 | 583914 | see also 3166 line | |
| 3176 | USP9X | NM_004652.2 | 6467 | tagtcaggctatgacacttaa | 190578 | 583918 | see also 3166 line | |
| 3177 | USP9X | NM_004652.2 | 6501 | cacttactaagcagcagtactaaa | 190579 | 583920 | see also 3166 line | |
| 3178 | USP9X | NM_004652.2 | 7132 | accgagatgggctgtttgacaca | 190582 | 583940 | see also 3166 line | |
| 3179 | DDX3Y | NM_004660.2 | 1233 | cagatgcttgctcgtgacttttt | 24920 | 483048 | - | RNA binding |
| 3180 | CDC23 | NM_004661.2 | 824 | ttgcagttgccatcaccaatatc | 302244 | 801154 | cell_cycle | |
| 3181 | CDC23 | NM_004661.2 | 1366 | gttggaagccaaaaagtgttattg | 302266 | 801173 | see also 3180 line | |
| 3182 | CDC23 | NM_004661.2 | 1562 | atctggcccagtactattttaag | 302273 | 801179 | see also 3180 line | |
| 3183 | CDC23 | NM_004661.2 | 1565 | tggcccagtactatttttaagtgc | 302274 | 801180 | see also 3180 line | |
| 3184 | RAB11A | NM_004663.3 | 210 | ctgtctgatttactctcgaaatga | 109882 | 519861 | - | protein transporter |
| 3185 | RAB11A | NM_004663.3 | 214 | ctgatttactctcgaaatgagttt | 109884 | 519862 | see also 3184 line | |
| 3186 | RAB11A | NM_004663.3 | 215 | tcgatttactcgaaatgagttta | 109885 | 519863 | see also 3184 line | |
| 3187 | RAB11A | NM_004663.3 | 561 | aagaatggtttgtcattcattga | 109892 | 519875 | see also 3184 line | |
| 3188 | HERC2 | NM_004667.2 | 537 | ttcaggagaatgtcaaagttaag | 229599 | 616186 | - | - |
| 3189 | HERC2 | NM_004667.2 | 1306 | gacatctcataaggggatcattgc | 229600 | 616195 | see also 3188 line | |
| 3190 | HERC2 | NM_004667.2 | 1334 | gtcataggttggggttaatagg | 229601 | 616196 | see also 3188 line | |
| 3191 | HERC2 | NM_004667.2 | 2082 | aagatggccaagtttattcatg | 229610 | 616209 | see also 3188 line | |
| 3192 | PAPSS2 | NM_004670.2 | 1517 | gggtcctgatcccaagtcaacc | 95768 | 511319 | kinase_related | sulfate adenylyltransferase (ATP) |
| 3193 | MIZ1 | NM_004671.1 | 70 | ggcttgctggacggaataaaag | 32313 | 461727 | - | - |
| 3194 | MIZ1 | NM_004671.1 | 144 | ctgcagccctgccggttcagatta | 32317 | 461731 | see also 3193 line | - |

Figure 46

| | | | | | | | | | |
|---|---|---|---|---|---|---|---|---|---|
| 3195 | MIZ1 | NM_004671.1 | 148 | agccctgcggttcagattaaaat | 32320 | 461732 | see also 3193 line | | |
| 3196 | MIZ1 | NM_004671.1 | 149 | gccctgcggttcagattaaaatc | 32321 | 461733 | see also 3193 line | | |
| 3197 | MIZ1 | NM_004671.1 | 150 | ccctgcggttcagattaaaatcc | 32322 | 461734 | see also 3193 line | | |
| 3198 | MIZ1 | NM_004671.1 | 428 | atcctgatgtgcagttaaaaat | 32329 | 461741 | see also 3193 line | | |
| 3199 | MIZ1 | NM_004671.1 | 478 | ctcatcaagcccacgagtttagt | 32330 | 461742 | see also 3193 line | | |
| 3200 | MIZ1 | NM_004671.1 | 484 | aagcccacgagtttagttcaaag | 32331 | 461744 | see also 3193 line | | |
| 3201 | MIZ1 | NM_004671.1 | 485 | agcccacgagtttagttcaaagc | 32332 | 461745 | see also 3193 line | | |
| 3202 | MIZ1 | NM_004671.1 | 892 | cagcttacatcagccatgttatt | 32353 | 461758 | see also 3193 line | | |
| 3203 | MIZ1 | NM_004671.1 | 1027 | tccttgatgtgccttaggaaa | 32356 | 461764 | see also 3193 line | | |
| 3204 | MIZ1 | NM_004671.1 | 1497 | cccaaccaaggggttctcatgt | 32365 | 461774 | see also 3193 line | | |
| 3205 | MIZ1 | NM_004671.1 | 1587 | aacagactacttcagtaccattcc | 32369 | 461777 | see also 3193 line | | |
| 3206 | MIZ1 | NM_004671.1 | 1689 | ctgtcctcctatgttttggata | 32371 | 461778 | see also 3193 line | | |
| 3207 | MAP3K6 | NM_004672.2 | 2095 | tgggctgctcaagatttctgact | 89545 | 507379 | - | | |
| 3208 | ASH2L | NM_004674.1 | 582 | aagataaggatattataccattt | 332722 | 703113 | transcription_regulator | transcription regulator | |
| 3209 | ASH2L | NM_004674.1 | 1161 | atgatcgagctcccagttaaag | 332730 | 703126 | see also 3208 line | | |
| 3210 | ASH2L | NM_004674.1 | 1611 | ctccccatagtgagataatattt | 332739 | 703138 | see also 3208 line | | |
| 3211 | RGN | NM_004683.3 | 864 | gtgaagttgcctgttgattaaaac | 152041 | 553004 | - | | |
| 3212 | RGN | NM_004683.3 | 867 | aagttgcctgttgataaaaacaac | 152042 | 553005 | see also 3211 line | | |
| 3213 | MTMR6 | NM_004685.2 | 1191 | aagggctttctgtcaatgattc | 195311 | 588602 | phosphatase | protein serine/threonine phosphatase | |
| 3214 | MTMR6 | NM_004685.2 | 1233 | cgggatggcttcgccatatcaaa | 195312 | 588603 | see also 3213 line | | |
| 3215 | MTMR4 | NM_004687.3 | 283 | tccccctccgatgattgacagt | 158503 | 559001 | phosphatase | | |
| 3216 | MTMR4 | NM_004687.3 | 1182 | tcggcactggagtaccaaatg | 158516 | 559011 | see also 3215 line | | |
| 3217 | MTMR4 | NM_004687.3 | 3625 | agctgaagaaacccattgctaca | 158558 | 559063 | see also 3215 line | | |
| 3218 | MTA1 | NM_004689.2 | 1065 | aagtttatattccaactactaac | 7117 | 446002 | transcription_regulator, signal_transduction | transcription factor | |
| 3219 | SLC16A4 | NM_004696.1 | 665 | ggcaccctttacaaaattcctga | 257394 | 760569 | - | symporter | |
| 3220 | PRPF3 | NM_004698.1 | 1308 | tggaatcacaaatcttgttgaac | 46510 | 729836 | - | | retinitis pigmentosa |
| 3221 | CCNB2 | NM_004701.2 | 166 | cccgacggtccagtgatttgg | 307138 | 673626 | cell_cycle | cyclin-dependent protein kinase, regulator | |
| 3222 | RABEP1 | NM_004703.2 | 212 | tcccagcctgacgttcttctca | 169470 | 566180 | - | | |
| 3223 | RABEP1 | NM_004703.2 | 1444 | atccaaagctttaggctataact | 169495 | 566206 | see also 3222 line | | |
| 3224 | RABEP1 | NM_004703.2 | 1449 | aagctttaggctataactacaa | 169496 | 566207 | see also 3222 line | | |
| 3225 | RABEP1 | NM_004703.2 | 1530 | cagtgtctgagaactttgatact | 169498 | 566210 | see also 3222 line | | |

Figure 46

| # | Gene | Accession | Pos | Sequence | ID1 | ID2 | Note | Function / Disease |
|---|------|-----------|-----|----------|-----|-----|------|--------------------|
| 3226 | RABEP1 | NM_004703.2 | 1773 | aacttcgtagcacgttgtgatatg | 169510 | 566218 | see also 3222 line | - |
| 3227 | RABEP1 | NM_004703.2 | 1812 | aacagttacaaggaattcagatt | 169515 | 566221 | see also 3222 line | - |
| 3228 | PRKRIR | NM_004705.2 | 115 | aggagagcagacttagaagataa | 37034 | 690545 | signal_transduction | protein binding |
| 3229 | PRKRIR | NM_004705.2 | 230 | ttcgagataatgcaataccaaca | 37042 | 690553 | see also 3228 line | - |
| 3230 | PRKRIR | NM_004705.2 | 257 | ttgatcttaccagtcatttgaac | 37043 | 690554 | see also 3228 line | - |
| 3231 | SDCCAG1 | NM_004713.2 | 323 | gagattagtcagtcaaaaacagc | 332754 | 716345 | - | colorectal cancer, colorectal cancers |
| 3232 | SDCCAG1 | NM_004713.2 | 1847 | tggcacaatggcactttgctaca | 332797 | 716393 | see also 3231 line | - |
| 3233 | SDCCAG1 | NM_004713.2 | 1904 | ttgttgggtgtaccatcatcagg | 332801 | 716397 | see also 3231 line | - |
| 3234 | SDCCAG1 | NM_004713.2 | 2011 | atctaatgatgsggtttagcttc | 332805 | 716401 | see also 3231 line | - |
| 3235 | DYRK1B | NM_004714.1 | 450 | agcacatcaatgaggtatactat | 80972 | 500960 | kinase_related | - |
| 3236 | DYRK1B | NM_004714.1 | 462 | aggtatactatgcgaagaagaag | 80973 | 500962 | see also 3235 line | - |
| 3237 | PCSK7 | NM_004716.2 | 1017 | ggcgttcaacaagcactatcaga | 183170 | 5776850 | - | subtilase |
| 3238 | ARHGEF2 | NM_004723.2 | 255 | tccacaaccgctgtaaagacaac | 172126 | 697376 | signal_transduction | guanyl-nucleotide release factor |
| 3239 | ARHGEF2 | NM_004723.2 | 952 | cagcacccggaactttgtcatcc | 172133 | 697383 | see also 3238 line | - |
| 3240 | DDX21 | NM_004728.1 | 1251 | tggacctgattgtaaaaaagact | 50674 | 473484 | - | ATP dependent RNA helicase |
| 3241 | DDX21 | NM_004728.1 | 1488 | gggaaatcaccctgaaaggtttt | 50680 | 473491 | see also 3240 line | - |
| 3242 | ETF1 | NM_004730.1 | 327 | acgagtaaaacgccttcagtcc | 51443 | 477889 | - | translation release factor, codon specific |
| 3243 | ETF1 | NM_004730.1 | 630 | aagagaagtcctgcacaaattca | 51457 | 477902 | see also 3242 line | - |
| 3244 | ETF1 | NM_004730.1 | 697 | ttgcgttttgcccgtttaagaat | 51458 | 477904 | see also 3242 line | - |
| 3245 | ETF1 | NM_004730.1 | 698 | tgcgttttgccgtttaagaatg | 51459 | 477905 | see also 3242 line | - |
| 3246 | ETF1 | NM_004730.1 | 921 | tggattcaaccaagctattgagt | 51466 | 477910 | see also 3242 line | - |
| 3247 | ETF1 | NM_004730.1 | 1369 | atcttgcgtaccgagtagatt | 51483 | 477927 | see also 3242 line | - |
| 3248 | ETF1 | NM_004730.1 | 1374 | gcggtaccgagtagatttccagg | 51484 | 477928 | see also 3242 line | - |
| 3249 | SLC16A7 | NM_004731.3 | 1044 | taggccttctgaggattgattaattg | 257473 | 641031 | - | symporter |
| 3250 | SLC33A1 | NM_004733.2 | 1519 | atgccctacacgattattgcttgg | 273097 | 705800 | - | - |
| 3251 | SLC33A1 | NM_004733.2 | 1563 | ttgttggactcctaaagtagaac | 273098 | 705803 | see also 3250 line | - |
| 3252 | SLC33A1 | NM_004733.2 | 1719 | aacatacatgacccttttaaata | 273111 | 705814 | see also 3250 line | - |
| 3253 | DCAMKL1 | NM_004734.1 | 1490 | tcgaggcaaagagagcacatgatcc | 77997 | 499455 | kinase_related | receptor signaling protein |
| 3254 | XPR1 | NM_004736.2 | 406 | caggcaggtttgctacacttca | 243626 | 638567 | receptor acitivity, gpcr, signal transduction | - |
| 3255 | XPR1 | NM_004736.2 | 412 | aggtttgctacacttcagaatga | 243627 | 638568 | see also 3254 line | - |
| 3256 | XPR1 | NM_004736.2 | 706 | gagttgccccattttatacatg | 243632 | 638575 | see also 3254 line | - |

Figure 46

| 3257 | XPR1 | NM_004736.2 | 976 | atctatcggggtggctttcttct | 243639 | 638580 | see also 3254 line | | |
| 3258 | XPR1 | NM_004736.2 | 1248 | ccccaccaaaactttctactata | 243650 | 638588 | see also 3254 line | | |
| 3259 | XPR1 | NM_004736.2 | 1249 | cccaccaaaactttctactataa | 243651 | 638589 | see also 3254 line | | |
| 3260 | XPR1 | NM_004736.2 | 1388 | tggacctggaatatatgatctgc | 243653 | 638592 | see also 3254 line | | |
| 3261 | XPR1 | NM_004736.2 | 1973 | ggcgatttgtgtggaacttcttc | 243675 | 638619 | see also 3254 line | | |
| 3262 | XPR1 | NM_004736.2 | 2209 | gacactaaggtattgatagaaga | 243687 | 638630 | see also 3254 line | | |
| 3263 | LARGE | NM_004737.2 | 634 | cccagccatcacctggatttacc | 212153 | 600702 | - | - | - |
| 3264 | LARGE | NM_004737.2 | 2158 | gggctaccacatcgtgtacaagg | 212169 | 600718 | see also 3263 line | | |
| 3265 | VAPB | NM_004738.3 | 482 | gtgatgttacagcctttcgatta | 292743 | 662473 | - | - | - |
| 3266 | VAPB | NM_004738.3 | 485 | atgttacagcctttcgattatga | 292744 | 662474 | see also 3265 line | | |
| 3267 | VAPB | NM_004738.3 | 600 | aaccggaagaccttatggattca | 292745 | 662477 | see also 3265 line | | |
| 3268 | VAPB | NM_004738.3 | 663 | aaccacatgatgtagaaataaat | 292748 | 662479 | see also 3265 line | | |
| 3269 | MTA2 | NM_004739.2 | 1289 | tagtccagttttattacatgtgg | 7145 | 446048 | transcription_regulator | - | - |
| 3270 | MTA2 | NM_004739.2 | 1314 | aaccacagaccggtatattcagc | 7146 | 446049 | see also 3269 line | | |
| 3271 | MTA2 | NM_004739.2 | 1930 | ctggcaactatcgtcaaagatct | 7159 | 446061 | see also 3269 line | | |
| 3272 | MTA2 | NM_004739.2 | 2114 | ctctggcttcagggattcgttca | 7162 | 446063 | see also 3269 line | | |
| 3273 | RPS6KA5 | NM_004755.2 | 1418 | acgtcctggagtgacaaatgttg | 99866 | 488938 | kinase_related、transcription_regulator、signal_transduction | - | - |
| 3274 | ITGB1BP1 | NM_004763.2 | 584 | atccggatggtgtgttacgatga | 126205 | 697308 | kinase_related | - | - |
| 3275 | PIWIL1 | NM_004764.2 | 1751 | aagtaatcggaaggacaaatacg | 332973 | 472480 | - | - | - |
| 3276 | COPB2 | NM_004766.1 | 945 | gggagcatcattgttaagcttgg | 125174 | 650989 | kinase_related | protein transporter | - |
| 3277 | COPB2 | NM_004766.1 | 1177 | gtggtgatgggggagtatatcatc | 125182 | 650993 | see also 3276 line | | |
| 3278 | COPB2 | NM_004766.1 | 1178 | tggtgatgggggagtatatcatct | 125183 | 650994 | see also 3276 line | | |
| 3279 | COPB2 | NM_004766.1 | 1183 | atggggagtatatcatctacaca | 125185 | 650995 | see also 3276 line | | |
| 3280 | COPB2 | NM_004766.1 | 1249 | ttgcatgggcccacgattcttca | 125186 | 650996 | see also 3276 line | | |
| 3281 | COPB2 | NM_004766.1 | 1257 | gcccacgattcttcagagtatgc | 125187 | 650997 | see also 3276 line | | |
| 3282 | COPB2 | NM_004766.1 | 1402 | atggcttagccttctatgactgg | 125192 | 651002 | see also 3276 line | | |
| 3283 | MMP20 | NM_004771.2 | 641 | aagtggactatgggaacgaatgg | 158266 | 558819 | - | zinc binding | - |
| 3284 | PPARBP | NM_004774.2 | 247 | ctggaacggctccatgcaaaatt | 36478 | 451027 | receptor_acitivity、transcription_regulator、signal_transduction | transcription co-activator | - |
| 3285 | PPARBP | NM_004774.2 | 248 | tggaacggctccatgcaaaattt | 36479 | 451028 | see also 3284 line | | |
| 3286 | PPARBP | NM_004774.2 | 251 | aacggctccatgcaaaatttaac | 36480 | 451029 | see also 3284 line | | |
| 3287 | PPARBP | NM_004774.2 | 411 | accagcaatgactgatcgtttgg | 36483 | 451035 | see also 3284 line | | |
| 3288 | PPARBP | NM_004774.2 | 498 | cacgtcagatatgttctatgtgg | 36487 | 451038 | see also 3284 line | | |

207

Figure 46

| | | | | | | | | |
|---|---|---|---|---|---|---|---|---|
| 3289 | PPARBP | NM_004774.2 | 712 | ttggctctccaatccttagaaca | 36496 | 451042 | see also 3284 line | |
| 3290 | PPARBP | NM_004774.2 | 828 | aaggagtggggtcatttaatga | 36500 | 451134 | see also 3284 line | |
| 3291 | PPARBP | NM_004774.2 | 1065 | cagtgccaacagtgttgatcttc | 36507 | 451052 | see also 3284 line | |
| 3292 | PPARBP | NM_004774.2 | 1389 | ccctggccgagttcctcttatcc | 36517 | 451058 | see also 3284 line | - |
| 3293 | VAMP3 | NM_004781.2 | 44 | ctgccactggcagtaatcgaaga | 302754 | 686818 | - | - |
| 3294 | VAMP3 | NM_004781.2 | 73 | cagacacaaatcaagtagatga | 302755 | 686819 | see also 3293 line | |
| 3295 | NDST3 | NM_004784.1 | 1980 | atgaccgactgggattatataca | 217267 | 605705 | - | - |
| 3296 | NDST3 | NM_004784.1 | 2142 | aacgccacagagacatttggtct | 217269 | 605709 | see also 3295 line | |
| 3297 | NDST3 | NM_004784.1 | 2256 | atccttccatccttagtaaactcc | 217273 | 605715 | see also 3295 line | |
| 3298 | NDST3 | NM_004784.1 | 2300 | gaggtacagttctttaatagaaa | 217274 | 605716 | see also 3295 line | |
| 3299 | NDST3 | NM_004784.1 | 2324 | aactaccacaggggggattgattg | 217275 | 605718 | see also 3295 line | |
| 3300 | TXNL | NM_004786.1 | 801 | acctaccccgatctatggatttt | 224744 | 649942 | apoptosis, signal_transduction | electron transporter |
| 3301 | TXNL | NM_004786.1 | 1011 | cagtccaggcaacaaatatgaat | 224756 | 649950 | see also 3300 line | |
| 3302 | SLIT2 | NM_004787.1 | 492 | aggagcattccaggatcttaaag | 136081 | 554042 | - | - |
| 3303 | SLIT2 | NM_004787.1 | 1102 | gagaccatcacagaaatacgttt | 136105 | 554058 | see also 3302 line | |
| 3304 | SLIT2 | NM_004787.1 | 1360 | tgccttcggctagatgctttca | 136117 | 554062 | see also 3302 line | |
| 3305 | SLIT2 | NM_004787.1 | 1668 | tacagaagattatcgatcaaaat | 136128 | 554070 | see also 3302 line | |
| 3306 | SLIT2 | NM_004787.1 | 2818 | gactggtgaagtcggaatataa | 136168 | 554114 | see also 3302 line | |
| 3307 | SLIT2 | NM_004787.1 | 4699 | cggcggaaatactctttcgaatg | 136200 | 554172 | see also 3302 line | |
| 3308 | SLIT2 | NM_004787.1 | 4700 | ggccggaaatactctttcgaatgc | 136201 | 554173 | see also 3302 line | |
| 3309 | UBE4A | NM_004788.2 | 503 | atcaagactggcttgatatgagc | 235455 | 670649 | | ubiquitin conjugating enzyme |
| 3310 | UBE4A | NM_004788.2 | 682 | aaggaagagattaccaaagttcc | 235467 | 670655 | see also 3309 line | |
| 3311 | UBE4A | NM_004788.2 | 809 | agcaactggtagattgatgtta | 235470 | 670656 | see also 3309 line | |
| 3312 | UBE4A | NM_004788.2 | 1698 | caccttgacttggatttcaca | 235500 | 670672 | see also 3309 line | |
| 3313 | UBE4A | NM_004788.2 | 2012 | tggcttatgtgccagaattttt | 235504 | 670677 | see also 3309 line | |
| 3314 | UBE4A | NM_004788.2 | 2134 | accattttcactggaagcataga | 235509 | 670678 | see also 3309 line | |
| 3315 | UBE4A | NM_004788.2 | 2142 | cactggaagcatagaaagaatga | 235510 | 670679 | see also 3309 line | |
| 3316 | UBE4A | NM_004788.2 | 2432 | atcgggagagcattaaggatttg | 235519 | 670687 | see also 3309 line | |
| 3317 | UBE4A | NM_004788.2 | 2456 | ctgactgcctctaagaattta | 235521 | 670690 | see also 3309 line | |
| 3318 | UBE4A | NM_004788.2 | 2458 | gactatgcctctaagaatttaga | 235522 | 670691 | see also 3309 line | |
| 3319 | UBE4A | NM_004788.2 | 2846 | aggacttcagcgagatttgacttc | 235531 | 670702 | see also 3309 line | |
| 3320 | UBE4A | NM_004788.2 | 2896 | tgcactatcacttaaatcttgg | 235533 | 670707 | see also 3309 line | |
| 3321 | LHX2 | NM_004789.3 | 1125 | tgccgctcggcgactggtttat | 6141 | 445314 | transcription_regulator | transcription factor |
| 3322 | LHX2 | NM_004789.3 | 1126 | gcgcggctcgggacttggtttatc | 6142 | 445315 | see also 3321 line | - |

Figure 46

| | | | | | | | | | |
|---|---|---|---|---|---|---|---|---|---|
| 3323 | LHX2 | NM_004789.3 | 1127 | cgcgctcgggacttggtttatca | 6143 | 445316 | see also 3321 line | | |
| 3324 | LHX2 | NM_004789.3 | 1129 | cgctcgggacttggtttatcacc | 6144 | 445317 | see also 3321 line | | |
| 3325 | SLC22A6 | NM_004790.3 | 976 | caccttgattggctatgtctaca | 267191 | 654134 | - | - | - |
| 3326 | SLC22A6 | NM_004790.3 | 1340 | ctgtggtttgccactagctttgc | 267194 | 654140 | see also 3325 line | | |
| 3327 | ITGBL1 | NM_004791.1 | 700 | aggtgtaagtgtgataattcaga | 290434 | 660346 | - | - | - |
| 3328 | ITGBL1 | NM_004791.1 | 801 | tggaggccatgggaagtgttact | 290438 | 660352 | see also 3327 line | | |
| 3329 | NRXN3 | NM_004796.2 | 1603 | agctcatggttaacttagactgt | 236909 | 775694 | receptor_acitivity | - | - |
| 3330 | NRXN3 | NM_004796.2 | 1609 | tggttaacttagactgtatcagg | 236910 | 775695 | see also 3329 line | | |
| 3331 | NRXN3 | NM_004796.2 | 3528 | agggacgaggggtcctatcaagt | 236933 | 775727 | see also 3329 line | | |
| 3332 | TM9SF2 | NM_004800.1 | 817 | aagattagtggctgctaaacttg | 275272 | 657721 | - | transporter | - |
| 3333 | TM9SF2 | NM_004800.1 | 1077 | ctggaatggtagctatgattatg | 275285 | 657736 | see also 3332 line | | |
| 3334 | TM9SF2 | NM_004800.1 | 1585 | gacgtttattggtgcatactttg | 275299 | 657743 | see also 3332 line | | |
| 3335 | TM9SF2 | NM_004800.1 | 1776 | cacaccagatgtattacatgttt | 275308 | 657751 | see also 3332 line | | |
| 3336 | NRXN1 | NM_004801.2 | 1557 | tgccaagcaaggagatcctaaga | 319254 | 683803 | - | - | - |
| 3337 | NRXN1 | NM_004801.2 | 1940 | acggacggtcaggtaccatttct | 319264 | 683818 | see also 3336 line | | |
| 3338 | NRXN1 | NM_004801.2 | 2428 | ttccgatcccagcgtgcatatgg | 319281 | 683828 | see also 3336 line | | |
| 3339 | NRXN1 | NM_004801.2 | 2528 | cggtcaatctagattgtatcagg | 319286 | 683836 | see also 3336 line | | |
| 3340 | NRXN1 | NM_004801.2 | 2920 | aagaccaaatcgagctatgttgc | 319296 | 683851 | see also 3336 line | | |
| 3341 | NRXN1 | NM_004801.2 | 3909 | agggcgtcagctcacaatcttca | 319330 | 683884 | see also 3336 line | | |
| 3342 | NMT2 | NM_004808.1 | 1033 | cagacttccagatgttacaaaga | 215486 | 602778 | - | glycyl-peptide N-tetradecanoyltransferase | - |
| 3343 | STOML1 | NM_004809.3 | 1002 | ggggcctgctaccagttcaatgt | 179471 | 572852 | - | - | - |
| 3344 | LPXN | NM_004811.1 | 648 | tggctccagtcccttctttgagc | 302628 | 671095 | signal_transduction | - | - |
| 3345 | PARG1 | NM_004815.2 | 946 | tggaattggagtccactagaaat | 296892 | 680607 | signal_transduction | - | - |
| 3346 | PARG1 | NM_004815.2 | 2522 | tcgattgtacaaggaatttatag | 296945 | 680651 | see also 3345 line | | |
| 3347 | TJP2 | NM_004817.1 | 2772 | aagaccgcatgtcctacttaact | 317477 | 682135 | kinase_related | membrane-associated guanylate kinase | - |
| 3348 | ECEL1 | NM_004826.1 | 1909 | ggcgctcaatgcctactatctac | 156533 | 557531 | signal_transduction | zinc binding | - |
| 3349 | MAP4K4 | NM_004834.2 | 1908 | aacgcttcagagtgagatcatca | 333066 | 507500 | - | - | - |
| 3350 | MAP4K4 | NM_004834.2 | 2076 | tacggccacctcacaaagtaacg | 333067 | 507504 | see also 3349 line | | |
| 3351 | MAP4K4 | NM_004834.2 | 2341 | tagtagcacactccagaaacaca | 333071 | 507509 | see also 3349 line | | |
| 3352 | MAP4K4 | NM_004834.2 | 3181 | tagcatcaaaccccatgcaatca | 333097 | 507533 | see also 3349 line | | |
| 3353 | EIF2AK3 | NM_004836.2 | 1086 | cactttgaacttcggtatattcc | 81227 | 501381 | - | protein serine/threonine kinase | epiphyseal dysplasia、diabetes |
| 3354 | EIF2AK3 | NM_004836.2 | 1290 | tgggagtaccagttttgtactcc | 81232 | 501383 | see also 3353 line | | |

Figure 46

| | | | | | | | | | |
|---|---|---|---|---|---|---|---|---|---|
| 3355 | ARHGEF6 | NM_004840.1 | 331 | tactcttttagctgtcaacaaag | 294833 | 767228 | apoptosis、kinase_related | guanyl-nucleotide release factor | mental retardation |
| 3356 | ARHGEF6 | NM_004840.1 | 1766 | ttggagcctcctcaaattataaa | 294873 | 767267 | see also 3355 line | | |
| 3357 | ARHGEF6 | NM_004840.1 | 1767 | tggagcctcctcaaattataaaa | 294874 | 767268 | see also 3355 line | | |
| 3358 | EIF4EL3 | NM_004846.1 | 172 | agcatcccctgcagtacaactac | 49297 | 473631 | - | - | - |
| 3359 | APG5L | NM_004849.1 | 419 | agcagaaccatactatttgcttt | 310375 | 676419 | apoptosis | - | - |
| 3360 | APG5L | NM_004849.1 | 461 | gacgttggtaactgacaaagtga | 310376 | 676421 | see also 3359 line | | |
| 3361 | APG5L | NM_004849.1 | 560 | atggcattatccaattggtttgc | 310380 | 676422 | see also 3359 line | | |
| 3362 | APG5L | NM_004849.1 | 586 | ttgatcttcttgcatcaagttca | 310383 | 676424 | see also 3359 line | | |
| 3363 | APG5L | NM_004849.1 | 834 | cggaaactcatggaatatcctgc | 310390 | 676433 | see also 3359 line | | |
| 3364 | ROCK2 | NM_004850.2 | 1575 | aactcagcagtgacatagacagc | 99302 | 513639 | kinase_related | - | - |
| 3365 | ROCK2 | NM_004850.2 | 1913 | gagattaccttacggaaaagtgt | 99314 | 513650 | see also 3364 line | | |
| 3366 | ROCK2 | NM_004850.2 | 2054 | aacagcttaaaagatcaacttga | 99318 | 513655 | see also 3364 line | | |
| 3367 | ROCK2 | NM_004850.2 | 2246 | aacaatagagatctacaagataa | 99328 | 513661 | see also 3364 line | | |
| 3368 | ROCK2 | NM_004850.2 | 2507 | aacatggaaatagatatgacata | 99331 | 513669 | see also 3364 line | | |
| 3369 | ROCK2 | NM_004850.2 | 3308 | gagatgatggctagacacaaaca | 99354 | 513687 | see also 3364 line | | |
| 3370 | ROCK2 | NM_004850.2 | 3547 | aactcaagctgtgaataagttgg | 99360 | 513692 | see also 3364 line | | |
| 3371 | ROCK2 | NM_004850.2 | 4473 | gggttagtcggttggtgaaaaag | 99389 | 513718 | see also 3364 line | | |
| 3372 | ONECUT2 | NM_004852.1 | 92 | aacccggagctgacaatggaaag | 8769 | 810109 | transcription_regulator | translation regulator | - |
| 3373 | ONECUT2 | NM_004852.1 | 93 | acccggagctgacaatggaaagt | 8770 | 810110 | see also 3372 line | | |
| 3374 | CHST10 | NM_004854.2 | 365 | ccgcatgcttttgggtgatttc | 217431 | 605528 | - | - | - |
| 3375 | CHST10 | NM_004854.2 | 869 | agcgattgaaaacatacttcaag | 217436 | 605538 | see also 3374 line | | |
| 3376 | SLC4A8 | NM_004858.1 | 44 | acggcgtcctcagctatcagaga | 258458 | 642848 | - | - | - |
| 3377 | FXR2 | NM_004860.2 | 719 | gggagccaactgcatctttctca | 51838 | 473899 | - | RNA binding | - |
| 3378 | SPTLC2 | NM_004863.2 | 356 | aagaccgtttaatgaagcttttg | 215982 | 603204 | - | transaminase | - |
| 3379 | SPTLC2 | NM_004863.2 | 702 | aacatgggttcctacaactatct | 215991 | 603212 | see also 3378 line | | |
| 3380 | SPTLC2 | NM_004863.2 | 705 | atgggttcctacaactatcttgg | 215992 | 603213 | see also 3378 line | | |
| 3381 | SPTLC2 | NM_004863.2 | 710 | ttcctacaactatcttggatttg | 215993 | 603214 | see also 3378 line | | |
| 3382 | SPTLC2 | NM_004863.2 | 1726 | cagcagctcataccaaagaaata | 216025 | 603246 | see also 3378 line | | |
| 3383 | TBPL1 | NM_004865.2 | 509 | tgcactggagcaacaagtgaaga | 41620 | 468729 | - | transcription co-activator | - |
| 3384 | TBPL1 | NM_004865.2 | 559 | agcccgtagtctgcagaaactag | 41621 | 468730 | see also 3383 line | | |
| 3385 | C1orf8 | NM_004872.3 | 763 | agccgatgacggaaaaatagtta | 326103 | 709314 | - | - | - |
| 3386 | C1orf8 | NM_004872.3 | 764 | gccgatgacggaaaaatagttat | 326104 | 709315 | see also 3385 line | | |
| 3387 | BAG4 | NM_004874.2 | 931 | ctgccccattatccttatggaga | 129153 | 532445 | apoptosis | receptor signaling protein | - |

210

Figure 46

| | | | | | | | | | |
|---|---|---|---|---|---|---|---|---|---|
| 3388 | EI24 | NM_004879.2 | 320 | aaccacgtattgttagtagaatt | 310981 | 696340 | apoptosis | - | - |
| 3389 | EI24 | NM_004879.2 | 321 | accacgtattgttagtagaattt | 310982 | 696341 | see also 3388 line | | |
| 3390 | EI24 | NM_004879.2 | 837 | aaggaattggccttactactttg | 310993 | 696359 | see also 3388 line | | |
| 3391 | CXCL14 | NM_004887.3 | 603 | ccgctacagcgacgtgaagaagc | 167388 | 564454 | signal_transduction | chemokine | - |
| 3392 | SPAG7 | NM_004890.1 | 310 | ctgtcgctatgtcatgatcttca | 66219 | 486356 | - | - | - |
| 3393 | SPAG7 | NM_004890.1 | 312 | gtcgctatgtcatgatcttcaaa | 66220 | 486357 | see also 3392 line | | |
| 3394 | SEC22L1 | NM_004892.3 | 202 | gggaccttcaacagtatcagagt | 305084 | 673306 | - | - | - |
| 3395 | SEC22L1 | NM_004892.3 | 221 | gagtcaggctaagcaactcttc | 305085 | 673307 | see also 3394 line | | |
| 3396 | SEC22L1 | NM_004892.3 | 226 | aggctaagcaactcttcgaaag | 305086 | 673308 | see also 3394 line | | |
| 3397 | SEC22L1 | NM_004892.3 | 227 | ggctaagcaactcttcgaaagt | 305087 | 673309 | see also 3394 line | | |
| 3398 | CLOCK | NM_004898.2 | 784 | ttgtggatcaaagtatatttaat | 2292 | 442742 | transcription_regulator、 signal_transduction | transcription factor | - |
| 3399 | CLOCK | NM_004898.2 | 1276 | atcatgtggatgacctagaaaat | 2299 | 442759 | see also 3398 line | | |
| 3400 | CLOCK | NM_004898.2 | 1386 | tggcttcagactcattattatat | 2304 | 442767 | see also 3398 line | | |
| 3401 | CLOCK | NM_004898.2 | 1387 | ggcttcagactcattattatatc | 2305 | 442768 | see also 3398 line | | |
| 3402 | CLOCK | NM_004898.2 | 1534 | ctgctgacaaaagccaagattct | 2311 | 442771 | see also 3398 line | | |
| 3403 | BRE | NM_004899.3 | 263 | tggatgctacaaactgtttgagg | 235339 | 620328 | signal_transduction | - | - |
| 3404 | LYSAL1 | NM_004901.1 | 1431 | ttcacttccagaacagtgaattc | 166356 | 563752 | phosphatase | uridine diphosphatase | - |
| 3405 | RNPC2 | NM_004902.2 | 684 | ttgcctcatagcatcaaattaag | 54322 | 475367 | transcription_regulator | - | - |
| 3406 | RNPC2 | NM_004902.2 | 685 | tgcctcatagcatcaaattaagc | 54323 | 475368 | see also 3405 line | | |
| 3407 | RNPC2 | NM_004902.2 | 1337 | tgcttcgagtgctagttcatttt | 54347 | 475392 | see also 3405 line | | |
| 3408 | RNPC2 | NM_004902.2 | 1340 | ttcgagtgctagttcatttttgg | 54348 | 475393 | see also 3405 line | | |
| 3409 | CREB5 | NM_004904.1 | 153 | cccaacagaggaccatctgatga | 24583 | 648472 | transcription_regulator | - | - |
| 3410 | CREB5 | NM_004904.1 | 1367 | aacagttgttgttaacacataaa | 24594 | 457395 | see also 3409 line | | |
| 3411 | CREB5 | NM_004904.1 | 1369 | cagttgttgttaacacataaaga | 24595 | 457396 | see also 3409 line | | |
| 3412 | PITPNM1 | NM_004910.1 | 1957 | cgccgtgggagcatgaacaatga | 166989 | 564196 | - | - | - |
| 3413 | CCM1 | NM_004912.3 | 1367 | gccttggataagtggttagatga | 297476 | 687216 | signal_transduction | small GTPase regulatory/interacting protein | cerebral cavernous malformations(-) |
| 3414 | CCM1 | NM_004912.3 | 1516 | gggctatagtgcactagaaataa | 297481 | 687226 | see also 3413 line | | |
| 3415 | CCM1 | NM_004912.3 | 1524 | gtgcactagaaataaagagtaaa | 297483 | 687227 | see also 3413 line | | |
| 3416 | CCM1 | NM_004912.3 | 1593 | ttggatcagatcttcagtataca | 297484 | 687228 | see also 3413 line | | |
| 3417 | CCM1 | NM_004912.3 | 1612 | tacaaatcgggtagataaagtgg | 297485 | 687230 | see also 3413 line | | |
| 3418 | CCM1 | NM_004912.3 | 1663 | tccagactactcaaaaatccaaa | 297491 | 687232 | see also 3413 line | | |

Figure 46

| | | | | | | | | | |
|---|---|---|---|---|---|---|---|---|---|
| 3419 | CCM1 | NM_004912.3 | 1853 | cactgggcaccattcattatgc | 297498 | 687239 | see also 3413 line | | |
| 3420 | CCM1 | NM_004912.3 | 1857 | gggcaccattcattatgcatgc | 297499 | 687241 | see also 3413 line | | |
| 3421 | CCM1 | NM_004912.3 | 2462 | gacccactagctattcttattct | 297513 | 687266 | see also 3413 line | | |
| 3422 | SEC24C | NM_004922.2 | 1401 | gaggaggcttcacgtatgttgt | 118912 | 776217 | - | protein transporter | - |
| 3423 | SEC24C | NM_004922.2 | 1580 | cggcaaacgtgtggatgcttatg | 118917 | 776219 | see also 3422 line | | |
| 3424 | SEC24C | NM_004922.2 | 2455 | ctggtatccgtgctgtagattc | 118936 | 776234 | see also 3422 line | | |
| 3425 | SEC24C | NM_004922.2 | 2456 | tggtatccgtgctgtagatttct | 118937 | 776235 | see also 3422 line | | |
| 3426 | SEC24C | NM_004922.2 | 2460 | atccgtgctgtagattcttggg | 118938 | 776236 | see also 3422 line | | |
| 3427 | ACTN4 | NM_004924.3 | 441 | tccatcgggcagaagagattgt | 114868 | 521238 | | structural constituent of cytoskeleton | - |
| 3428 | CALB1 | NM_004929.2 | 263 | aagtggttacctggaaggaaagg | 129558 | 545126 | | protein binding | - |
| 3429 | CAPZB | NM_004930.1 | 384 | tgctggatgatcctcataaaga | 114023 | 521041 | | structural constituent of cytoskeleton | - |
| 3430 | CAPZB | NM_004930.1 | 387 | tggagtgatcctcataaagaagg | 114025 | 521042 | see also 3429 line | | |
| 3431 | CDH6 | NM_004932.2 | 1562 | tgccaggaatcctgtcaagtact | 140235 | 545826 | - | calcium ion binding | - |
| 3432 | CDH6 | NM_004932.2 | 1976 | aactcggaaaatggctataata | 140248 | 545846 | see also 3431 line | | |
| 3433 | CTNS | NM_004937.1 | 722 | aggtgattggctggatctacttt | 271786 | 673448 | - | cystine transporter | - |
| 3434 | DAPK1 | NM_004938.1 | 945 | cggggtaataacctatatcctcc | 77895 | 499347 | kinase_related, apoptosis, signal_transduction | calmodulin-dependent protein kinase I | |
| 3435 | DAPK1 | NM_004938.1 | 3029 | cggctgagccgagtttggatat | 79952 | 499383 | see also 3434 line | | |
| 3436 | DAPK1 | NM_004938.1 | 3030 | ggctggaggcgagtttggatatg | 79953 | 499384 | see also 3434 line | | |
| 3437 | DAPK1 | NM_004938.1 | 3036 | aggcgagtttggatatgacaaag | 79954 | 499387 | see also 3434 line | | |
| 3438 | DDX1 | NM_004939.1 | 757 | gaactaggtactgacaagtttg | 50414 | 482818 | kinase_related | RNA helicase | neuroblastoma、retinoblastoma |
| 3439 | DDX1 | NM_004939.1 | 839 | aggaattcactatgcatgatacc | 50415 | 482820 | see also 3438 line | | |
| 3440 | DDX1 | NM_004939.1 | 1805 | tggatcaagcaattatcttctgt | 50450 | 482851 | see also 3438 line | | |
| 3441 | DDX1 | NM_004939.1 | 1811 | aagcaattatcttctgtatgaacc | 50452 | 482852 | see also 3438 line | | |
| 3442 | DDX1 | NM_004939.1 | 1982 | atgtaagattcttgatttgcaca | 50455 | 482858 | see also 3438 line | | |
| 3443 | DNM2 | NM_004945.1 | 521 | cgccacacgtgttgaacttgacc | 108850 | 519213 | transcription_regulator, signal_transduction, apoptosis, cell_cycle | motor | |
| 3444 | DOCK2 | NM_004946.1 | 4656 | atgggaggcttcgccaagtatga | 322766 | 676120 | - | - | |
| 3445 | SLC29A1 | NM_004955.1 | 402 | ctctcagtgccatcttcaacaat | 273046 | 654934 | - | nucleoside transporter | - |
| 3446 | FRAP1 | NM_004958.2 | 3388 | tggcgccaactggatgactacc | 207864 | 597826 | kinase_related, cell_cycle | inositol/phosphatidylinositol kinase | - |
| 3447 | FRAP1 | NM_004958.2 | 4880 | gaggttatccagtacaaaacttgt | 207892 | 597854 | see also 3446 line | | |

212

Figure 46

| | | | | | | | | | | |
|---|---|---|---|---|---|---|---|---|---|---|
| 3448 | FRAP1 | NM_004958.2 | 7219 | aagaatgttgaccaatgctatgg | 207936 | 597889 | see also 3446 line | transcription_regulator, receptor_acitivity, signal_transduction | transcription co-activator | - |
| 3449 | NR5A1 | NM_004959.3 | 452 | agggtgccggacaagtttgg | 8694 | 447361 | | | | - |
| 3450 | ICA1 | NM_004968.1 | 1244 | aacacgaattgctcaatgcatga | 323819 | 701224 | - | | | diabetes |
| 3451 | IDE | NM_004969.1 | 1152 | aggagcccgagtttatgtttt | 184320 | 578193 | signal_transduction | | signal transducer | Alzheimer disease |
| 3452 | IDE | NM_004969.1 | 2368 | ctccctgacagagatggtttgt | 184356 | 578215 | see also 3451 line | | | |
| 3453 | IDE | NM_004969.1 | 3015 | gcccacaacctgagtgattcaga | 184373 | 578242 | see also 3451 line | | | |
| 3454 | JAK2 | NM_004972.2 | 1366 | ctggaaacgtggaattcagtgg | 86143 | 504720 | kinase_related, cell_cycle | | protein tyrosine kinase | leukemia |
| 3455 | JAK2 | NM_004972.2 | 2916 | ttgtttactccagattatgaact | 86185 | 504774 | see also 3454 line | | | |
| 3456 | JAK2 | NM_004972.2 | 3296 | tggaagtttacgagactatcttc | 86203 | 504789 | see also 3454 line | | | |
| 3457 | JMJ | NM_004973.2 | 256 | aagacccaagaggaatatcattc | 29144 | 712150 | - | | DNA binding | - |
| 3458 | JMJ | NM_004973.2 | 518 | agcaacgatgttagttcttcaga | 29149 | 712152 | see also 3457 line | | | |
| 3459 | JMJ | NM_004973.2 | 2212 | ctgctttccggctgattaatg | 29169 | 712177 | see also 3457 line | | | |
| 3460 | JMJ | NM_004973.2 | 2253 | aagtgactgacctcaaaaaatgg | 29170 | 712179 | see also 3457 line | | | |
| 3461 | JMJ | NM_004973.2 | 2706 | ctctaacaactttatcgaaca | 29171 | 712185 | see also 3457 line | | | |
| 3462 | JMJ | NM_004973.2 | 2978 | gtgactattccctggctaaatat | 29176 | 712192 | see also 3457 line | | | |
| 3463 | JMJ | NM_004973.2 | 3073 | tggtgctgactgcatttggtatt | 29179 | 712198 | see also 3457 line | | | |
| 3464 | KCNA2 | NM_004974.2 | 1388 | ttggtaagagtctttaggatttt | 132241 | 536203 | channel | | protein binding | - |
| 3465 | KCNA2 | NM_004974.2 | 1937 | aactgtaccttggctaacacaaa | 132252 | 536218 | see also 3464 line | | | |
| 3466 | KCNA2 | NM_004974.2 | 1939 | ctgtaccttggctaacacacaaact | 132253 | 536219 | see also 3464 line | | | |
| 3467 | KCNA2 | NM_004974.2 | 1948 | ggctaacaaactatgtgaata | 132254 | 536221 | see also 3464 line | | | |
| 3468 | KCNB1 | NM_004975.2 | 2387 | cacagcaatagcgttcaactttg | 132337 | 536347 | channel | | protein binding | - |
| 3469 | KCNB1 | NM_004975.2 | 2571 | cccacctcccctaagttcttaag | 132345 | 536349 | see also 3468 line | | | |
| 3470 | KCNC1 | NM_004976.2 | 1460 | agctgggatcccaattattgt | 132414 | 536360 | channel | | voltage-gated potassium channel | - |
| 3471 | KCNC4 | NM_004978.2 | 197 | aagcctccgtccaaaacatgtct | 132467 | 536379 | channel | | | - |
| 3472 | KCNC4 | NM_004978.2 | 238 | ggggtgaggcgtcggagaagatca | 132468 | 536380 | see also 3471 line | | | |
| 3473 | KCND3 | NM_004980.3 | 1955 | ttgtggatgatcccccgttatct | 132532 | 536479 | channel | | | - |
| 3474 | KIF5A | NM_004984.1 | 1253 | cggtggcgcaatggagagaatgt | 87691 | 505912 | - | | motor | - |
| 3475 | KIF5A | NM_004984.1 | 1255 | gtggcgcaatggagagagaatgttgc | 87692 | 505913 | see also 3474 line | | | |
| 3476 | KIF5A | NM_004984.1 | 2539 | tcgcaagcgtttcagttcaagacg | 87711 | 505924 | see also 3474 line | | | |
| 3477 | KIF5A | NM_004984.1 | 2561 | gtcacgactcgagtcaagaaaag | 87712 | 505925 | see also 3474 line | | | |
| 3478 | KIF5A | NM_004984.1 | 2708 | ctgcgttgtgagcttcctaaatt | 87714 | 505930 | see also 3474 line | | | |

Figure 46

| # | Gene | Accession | Position | Sequence | ID1 | ID2 | Function | Class / GO term | Disease / phenotype |
|---|---|---|---|---|---|---|---|---|---|
| 3479 | KRAS2 | NM_004985.3 | 328 | aacctgtctggatattctg | 109472 | 519657 | cell_cycle, signal_tran sduction | | alveolar cell carcinoma, colorectal cancer, breast cancer, endometrial cancer, lung cancer, leukemia, ovarian cancer, pancreatic cancer, colorectal cancers |
| 3480 | KRAS2 | NM_004985.3 | 450 | ttgaagatattcaccattataga | 109475 | 519659 | see also 3479 line | | |
| 3481 | KTN1 | NM_004986.1 | 1866 | cagacctccgcttcagttctagc | 281216 | 687664 | - | - | - |
| 3482 | MECP2 | NM_004992.2 | 894 | acccaggtcatggtcatcaaacg | 32155 | 461638 | transcription_regulator | transcription co-repressor | Rett syndrome |
| 3483 | MECP2 | NM_004992.2 | 1603 | cgcccgtgaccgagagagtttagc | 32160 | 461644 | see also 3482 line | | |
| 3484 | MJD | NM_004993.2 | 614 | atgcatcgaccaaaacttattgg | 300594 | 669080 | transcription_regulator | | Machado-Joseph disease, spinocerebellar ataxia |
| 3485 | MJD | NM_004993.2 | 905 | aagagacgagagagcctactttga | 300601 | 669087 | see also 3484 line | | |
| 3486 | MJD | NM_004993.2 | 907 | gagacgagaagcctactttgaaa | 300602 | 669088 | see also 3484 line | | |
| 3487 | MJD | NM_004993.2 | 909 | gacgagaagcctactttgaaaaa | 300603 | 669089 | see also 3484 line | | |
| 3488 | MMP14 | NM_004995.2 | 1258 | tgggggtgaggaataaccaagt | 158115 | 558648 | | zinc binding | oral cavity cancers, chondrosarcoma, rheumatoid arthritis, laryngeal cancer, CNS Tumours, head and neck cancer |
| 3489 | ABCC1 | NM_004996.2 | 448 | ctgctgggcagacctcttctact | 69965 | 492031 | kinase_related | | small cell lung cancer |
| 3490 | ABCC1 | NM_004996.2 | 454 | ggcagacctcttctactctttct | 69966 | 492032 | see also 3489 line | | |
| 3491 | ABCC1 | NM_004996.2 | 1507 | ggccacgtacattaacatgatct | 69978 | 492059 | see also 3489 line | | |
| 3492 | MYBPH | NM_004997.1 | 1042 | ccgggtcttctcagaaaaacctgt | 289291 | 677879 | | structural constituent of muscle | |
| 3493 | MYO1E | NM_004998.1 | 1150 | ctgcacgccatgaatgtgattgg | 92876 | 805290 | | | |
| 3494 | MYO6 | NM_004999.1 | 424 | tgcagtgaatccatactttgaca | 93382 | 509776 | | structural constituent of muscle | |
| 3495 | MYO6 | NM_004999.1 | 729 | ctgttcgcaacaataatagccagt | 93391 | 509783 | see also 3494 line | | |
| 3496 | MYO6 | NM_004999.1 | 737 | aacaataatagccagtcgatttgg | 93392 | 509784 | see also 3494 line | | |
| 3497 | MYO6 | NM_004999.1 | 2509 | cagtcgctggaagaaagttcagt | 93445 | 509819 | see also 3494 line | | |
| 3498 | MYO6 | NM_004999.1 | 2998 | aaggcggatgaaaacttgaatgg | 93459 | 509827 | see also 3494 line | | |
| 3499 | NDUFS1 | NM_005006.5 | 1773 | gagacagtggaggttgtatcaca | 222822 | 610123 | | iron-sulfur electron transfer carrier | mitochondrial complex deficiency |
| 3500 | NDUFS1 | NM_005006.5 | 1805 | ttgccaaaggattgtttcattat | 222824 | 610125 | see also 3499 line | | |
| 3501 | NDUFS1 | NM_005006.5 | 2178 | ttgttccacctcagcttaactata | 222833 | 610134 | see also 3499 line | | |
| 3502 | NDUFS1 | NM_005006.5 | 2187 | ctcagctaactataaagacttc | 222834 | 610137 | see also 3499 line | | |
| 3503 | NRCAM | NM_005010.1 | 459 | cagagtgccctgattcttcc | 333368 | 791134 | channel | | |
| 3504 | NRCAM | NM_005010.1 | 590 | accctcgggagaatattgtaatc | 333371 | 791135 | see also 3503 line | | |
| 3505 | NRCAM | NM_005010.1 | 2379 | tagcccccattacaaattcatca | 333434 | 791217 | see also 3503 line | | |

Figure 46

| 3506 | ROR1 | NM_005012.1 | 712 | ggctctcggctgcggattagaaa | 99404 | 513730 | receptor_acitivity、 kinase_related、 signal_transduction | transmembrane receptor protein tyrosine kinase | - |
|---|---|---|---|---|---|---|---|---|---|
| 3507 | ROR1 | NM_005012.1 | 3155 | gacacaccgaatctatgatttct | 99464 | 513786 | see also 3506 line | | |
| 3508 | ROR1 | NM_005012.1 | 3157 | cacaccgaatctatgatttctgc | 99465 | 513787 | see also 3506 line | | |
| 3509 | NUCB2 | NM_005013.1 | 887 | caggaagcaaagatcaactaaaa | 34207 | 463190 | - | calcium ion binding | - |
| 3510 | OMD | NM_005014.1 | 414 | ctgtgactgcaaattcattcatc | 319394 | 683901 | - | - | - |
| 3511 | PDE1C | NM_005020.1 | 1502 | tacggacatgaccgagaagattg | 198011 | 590023 | signal_transduction | calmodulin-dependent cyclic-nucleotide phosphodiesterase | - |
| 3512 | PIK3CD | NM_005026.2 | 2069 | tggactgcgagctgaccaaattc | 207459 | 597438 | kinase_related、 signal_transduction | phosphatidylinositol 3-kinase、 class III | - |
| 3513 | PLS3 | NM_005032.3 | 1040 | aaccacggatagatattaacatg | 118817 | 523582 | - | calcium ion binding | - |
| 3514 | PLS3 | NM_005032.3 | 1041 | accacggatagatattaacatgt | 118818 | 523583 | see also 3513 line | | |
| 3515 | PLS3 | NM_005032.3 | 1043 | cacggatagatattaacatgtca | 118819 | 523584 | see also 3513 line | | |
| 3516 | PLS3 | NM_005032.3 | 1057 | aacatgtcaggtttcaatgaaac | 118820 | 523585 | see also 3513 line | | |
| 3517 | PLS3 | NM_005032.3 | 1112 | aacaagcagataaattaggttgc | 118824 | 523589 | see also 3513 line | | |
| 3518 | PMSCL1 | NM_005033.1 | 562 | tacgtgtagacctacatttatta | 53349 | 474843 | - | 3'-5' exoribonuclease | - |
| 3519 | PPARG | NM_005037.3 | 341 | gcctgcatctccaccttattatt | 9763 | 447955 | receptor_acitivity、 transcription_regulator、 signal_transduction | - | obesity、 atherosclerosis、 diabetes mellitus、 lipodystrophy、 diabetes |
| 3520 | PPARG | NM_005037.3 | 553 | gtcggatccacaaaaaaagtaga | 9770 | 447964 | see also 3519 line | | |
| 3521 | PPARG | NM_005037.3 | 679 | tggcggagatctccagtgatatc | 9772 | 447965 | see also 3519 line | | |
| 3522 | PPARG | NM_005037.3 | 801 | gcgatcttgacaggaaagacaac | 9774 | 447968 | see also 3519 line | | |
| 3523 | RELN | NM_005045.2 | 2104 | aggattcgctggagacaaacagg | 183909 | 577764 | - | - | schizophrenia |
| 3524 | RELN | NM_005045.2 | 5006 | tgcaagccaactggtatcgaatc | 183990 | 577826 | see also 3523 line | | |
| 3525 | ABCD4 | NM_005050.1 | 1457 | cccttcgggagcaggtgatatat | 70966 | 492765 | - | - | - |
| 3526 | RBBP5 | NM_005057.1 | 1573 | gcctaaaggatcaaaaggtaaag | 324882 | 775084 | - | - | - |
| 3527 | RBBP5 | NM_005057.1 | 1605 | ctccatttaaaccgaaactctac | 324883 | 775085 | see also 3526 line | | |
| 3528 | RBBP5 | NM_005057.1 | 1606 | tccatttaaaccgaaactctaca | 324884 | 775086 | see also 3526 line | | |
| 3529 | RORC | NM_005060.2 | 127 | aagacccacacctcacaaattga | 10795 | 448184 | receptor_acitivity、 transcription_regulator | steroid hormone receptor | - |
| 3530 | RORC | NM_005060.2 | 129 | gacccacacctcacaaattgaag | 10796 | 448185 | see also 3529 line | | |
| 3531 | SFPQ | NM_005066.1 | 1478 | cagaagaatccaatgtatcaaaa | 39200 | 486224 | - | pre-mRNA splicing factor | renal cell carcinoma |
| 3532 | SFPQ | NM_005066.1 | 1990 | aggtggtggtggcataggttatg | 39216 | 486235 | see also 3531 line | | |
| 3533 | SFPQ | NM_005066.1 | 1993 | tggtggtggcataggttatgaag | 39217 | 486236 | see also 3531 line | | |

EP 1 752 536 A1

Figure 46

| | Gene | Accession | Pos | Sequence | ID1 | ID2 | Function | Annotation |
|---|---|---|---|---|---|---|---|---|
| 3534 | SFPQ | NM_005066.1 | 1999 | tggcatagttatgaagctaatc | 39218 | 486237 | see also 3531 line | |
| 3535 | SIAH2 | NM_005067.4 | 619 | ggcacctggtgtgtaaccaatgc | 113317 | 670815 | transcription_regulator, apoptosis, cell_cycle, signal_transduction | - |
| 3536 | SIM1 | NM_005068.2 | 338 | gactcacgaccagctatctcaaa | 11210 | 448398 | transcription_regulator, signal_transduction | transcription factor — obesity |
| 3537 | SIM1 | NM_005068.2 | 342 | cacgaccagctatctcaaaatga | 11211 | 448400 | see also 3536 line | |
| 3538 | SIM2 | NM_005069.2 | 372 | gagacgccttcgtccattagg | 11252 | 467048 | transcription_regulator, signal_transduction | - |
| 3539 | SIM2 | NM_005069.2 | 1025 | ctgcatcgtgagtgtcaattatg | 11267 | 467060 | see also 3538 line | |
| 3540 | SLC4A3 | NM_005070.1 | 210 | agcgagcgggacttgagtttca | 258442 | 642769 | - | inorganic anion exchanger |
| 3541 | SLC1A6 | NM_005071.1 | 256 | accgccagatcaagtacttctct | 257697 | 641226 | - | sodium:dicarboxylate/ tricarboxylate cotransporter |
| 3542 | SLC1A6 | NM_005071.1 | 395 | gcgggcgagctgtgtactacatgg | 257699 | 641228 | see also 3541 line | |
| 3543 | SLC1A6 | NM_005071.1 | 901 | gcctcaatgaggctattgagg | 257701 | 641238 | see also 3541 line | |
| 3544 | SLC12A4 | NM_005072.3 | 1895 | cccggttcaagtactatccactgg | 257221 | 640750 | cell_cycle | |
| 3545 | SLC15A1 | NM_005073.1 | 1188 | gtgcaggtcggaaatcgataaaac | 256442 | 643992 | - | peptide:hydrogen symporter |
| 3546 | TLE3 | NM_005078.1 | 1055 | cgggcatggaccgataggtata | 300957 | 669480 | signal_transduction, transcription_regulator | - |
| 3547 | TLE3 | NM_005078.1 | 1056 | gggcatggaccgataggttataa | 300958 | 669481 | see also 3546 line | |
| 3548 | TLE3 | NM_005078.1 | 1057 | ggcatggaccgatataggttataat | 300959 | 669482 | see also 3546 line | |
| 3549 | XBP1 | NM_005080.2 | 428 | tagttgagaaccaggagttaaga | 13956 | 470342 | transcription_regulator | transcription factor |
| 3550 | FXR1 | NM_005087.1 | 55 | ttctacaagggatttatcaaga | 51758 | 473847 | apoptosis | RNA binding |
| 3551 | FXR1 | NM_005087.1 | 162 | accaccacctgatataaaaaag | 51768 | 473854 | see also 3550 line | |
| 3552 | FXR1 | NM_005087.1 | 347 | ttcggcctgtcaatcaaaataaa | 51773 | 473854 | see also 3550 line | |
| 3553 | FXR1 | NM_005087.1 | 1197 | cacctccggttatggtacaaatt | 51810 | 473881 | see also 3550 line | |
| 3554 | FXR1 | NM_005087.1 | 1198 | acctccggttatggtacaaattc | 51811 | 473882 | see also 3550 line | |
| 3555 | FXR1 | NM_005087.1 | 1201 | tccggttatggtacaaattctga | 51812 | 473883 | see also 3550 line | |
| 3556 | FXR1 | NM_005087.1 | 1203 | cggttatggtacaaattctgagc | 51813 | 473884 | see also 3550 line | |
| 3557 | FXR1 | NM_005087.1 | 1436 | atccatacagcttacttgataat | 51817 | 473887 | see also 3550 line | |

Figure 46

| | | | | | | | | | |
|---|---|---|---|---|---|---|---|---|---|
| 3558 | FXR1 | NM_005087.1 | 1443 | cagcttacttgataatacagaat | 51820 | 473888 | see also 3550 line | | |
| 3559 | ZNF261 | NM_005096.1 | 492 | ttcacctgaggggctactagagc | 44713 | 709357 | - | DNA binding | - |
| 3560 | ZNF261 | NM_005096.1 | 517 | ttggctccagattctccaataac | 44714 | 709358 | see also 3559 line | | |
| 3561 | ZNF261 | NM_005096.1 | 518 | tggctccagattctccaataaca | 44715 | 709359 | see also 3559 line | | |
| 3562 | ZNF261 | NM_005096.1 | 895 | atggtagatgaccccaatgatga | 44719 | 709362 | see also 3559 line | | |
| 3563 | ZNF261 | NM_005096.1 | 1409 | gggccaacaagggactgaaaacc | 44723 | 709366 | see also 3559 line | | |
| 3564 | ZNF261 | NM_005096.1 | 1584 | ctggtgcaagaccctgtgtaaga | 44726 | 709369 | see also 3559 line | | |
| 3565 | ZNF261 | NM_005096.1 | 1591 | aagaccctgtgtaagaactttga | 44727 | 709370 | see also 3559 line | | |
| 3566 | ZNF261 | NM_005096.1 | 1665 | gtgctgtaccacttcttacaaag | 44729 | 709372 | see also 3559 line | | |
| 3567 | ZNF261 | NM_005096.1 | 1666 | tgctgtaccacttcttacaaagt | 44730 | 709373 | see also 3559 line | | |
| 3568 | ZNF261 | NM_005096.1 | 2558 | cagcaacaccccgcaaaaacaag | 44739 | 709386 | see also 3559 line | | |
| 3569 | ZNF261 | NM_005096.1 | 3456 | tcggcccaatggtgaacgatatg | 44762 | 709402 | see also 3559 line | | |
| 3570 | ZNF261 | NM_005096.1 | 3457 | cggcccaatggtgaacgatatga | 44763 | 709403 | see also 3559 line | | |
| 3571 | ZNF261 | NM_005096.1 | 3578 | ttgttcaagaactcaacaagtct | 44768 | 709410 | see also 3559 line | | |
| 3572 | ZNF261 | NM_005096.1 | 3724 | atgttcttcaacactaagttttt | 44769 | 709414 | see also 3559 line | | |
| 3573 | ZNF261 | NM_005096.1 | 3841 | gtggtgagcatccgctactatgc | 44771 | 709415 | see also 3559 line | | |
| 3574 | LGI1 | NM_005097.1 | 260 | ctgcattcccctgaaaagaattg | 309168 | 710200 | - | - | CNS Tumours |
| 3575 | LGI1 | NM_005097.1 | 348 | tgccctgccgtgtgtacttgtac | 309169 | 710201 | see also 3574 line | | |
| 3576 | LGI1 | NM_005097.1 | 1870 | aacatgtcatagttgacttaagc | 309236 | 710263 | see also 3574 line | | |
| 3577 | ADAMTS4 | NM_005099.2 | 2613 | atgccctcaatggtgaatacacg | 155601 | 556380 | - | zinc binding | - |
| 3578 | FEZ1 | NM_005103.3 | 364 | atgtctgctttcggaactacaac | 312524 | 808706 | - | - | esophageal cancer、squamous cell cancer |
| 3579 | FEZ1 | NM_005103.3 | 472 | atgctctgacagacaattacatc | 312525 | 808707 | see also 3578 line | | |
| 3580 | FEZ1 | NM_005103.3 | 1286 | tactttgctaacggactacattt | 312533 | 808712 | see also 3578 line | | |
| 3581 | GFPT2 | NM_005110.1 | 1122 | gagaggtcgggtgaattttgaaa | 174684 | 568573 | - | sugar binding | - |
| 3582 | CRYZL1 | NM_005111.5 | 727 | tggcctgggagtagatattgtcc | 156392 | 557412 | - | - | - |
| 3583 | CRYZL1 | NM_005111.5 | 756 | ctggagtgagattatatagtaaa | 156394 | 557415 | see also 3582 line | | |
| 3584 | CRYZL1 | NM_005111.5 | 757 | tggagtgagattatatagtaaag | 156395 | 557416 | see also 3582 line | | |
| 3585 | WDR1 | NM_005112.3 | 776 | gtcaactgtgtgcgattctctcc | 120153 | 524969 | - | - | - |
| 3586 | WDR1 | NM_005112.3 | 1469 | gtcgtgtgcattggacagattgt | 120166 | 524987 | see also 3585 line | | |
| 3587 | GOLGA5 | NM_005113.2 | 2136 | ctgcgaaatgttcctgttctttt | 321683 | 686299 | - | - | - |
| 3588 | GOLGA5 | NM_005113.2 | 2171 | aactaatctggcaggaatgtacg | 321684 | 686301 | see also 3587 line | | |
| 3589 | SLC23A2 | NM_005116.4 | 852 | atgcaggttacccctgtttcagg | 256235 | 640358 | - | - | - |
| 3590 | TRAP150 | NM_005119.1 | 2062 | gcgtagcccctcagaactgtttg | 103037 | 637936 | receptor_acitivity、transcription_regulator、signal_transduction | - | - |
| 3591 | TRAP150 | NM_005119.1 | 2151 | tacatgaacgctttactaaatac | 103039 | 637941 | see also 3590 line | | |

Figure 46

| 3592 | TNRC11 | NM_005120.1 | 1357 | cccatgccagagggtaacagtgc | 112582 | 469273 | receptor_acitivity、 transcription_regulator、 signal_transduction | transcription co-activator | - |
|---|---|---|---|---|---|---|---|---|---|
| 3593 | TNRC11 | NM_005120.1 | 2398 | cagcggttggtcgtactgtttgg | 112607 | 469302 | see also 3592 line | | |
| 3594 | TNRC11 | NM_005120.1 | 2770 | atcttcgacctcatggaatattc | 112610 | 469305 | see also 3592 line | | |
| 3595 | TNRC11 | NM_005120.1 | 3051 | ctgtatccttgcttatctctatg | 112615 | 469314 | see also 3592 line | | |
| 3596 | TNRC11 | NM_005120.1 | 3380 | cagagctgaccggctattgcaag | 112621 | 469326 | see also 3592 line | | |
| 3597 | TNRC11 | NM_005120.1 | 4296 | ggccacaatcgaggttttccaac | 112633 | 469351 | see also 3592 line | | |
| 3598 | TNRC11 | NM_005120.1 | 4656 | cagccaggtgcaccagattgtga | 112635 | 469356 | see also 3592 line | | |
| 3599 | TNRC11 | NM_005120.1 | 5141 | ctggcttcgattccatcttcaag | 112645 | 469369 | see also 3592 line | | |
| 3600 | TNRC11 | NM_005120.1 | 5142 | tggcttcgattccatcttcaaga | 112646 | 469370 | see also 3592 line | | |
| 3601 | NUP153 | NM_005124.2 | 323 | cagggttacagaatctgttaaga | 34215 | 510679 | - | transporter | - |
| 3602 | NUP153 | NM_005124.2 | 353 | gccagggtggctacaaagatact | 34216 | 510681 | see also 3601 line | | |
| 3603 | NUP153 | NM_005124.2 | 1219 | ttgataggagtgggatagatatc | 34250 | 510696 | see also 3601 line | | |
| 3604 | NUP153 | NM_005124.2 | 2458 | gtgtgaagcgagcccttacattg | 34296 | 510734 | see also 3601 line | | |
| 3605 | NUP153 | NM_005124.2 | 2463 | aagcgagcccttacattgacagt | 34297 | 510735 | see also 3601 line | | |
| 3606 | THOC1 | NM_005131.1 | 225 | aacgttttagctattatttctct | 292491 | 682497 | - | - | - |
| 3607 | THOC1 | NM_005131.1 | 760 | tgcaatgctatgagaagatttca | 292511 | 682525 | see also 3606 line | | |
| 3608 | THOC1 | NM_005131.1 | 1067 | ttggattgaagatactacaaaat | 292517 | 682535 | see also 3606 line | | |
| 3609 | THOC1 | NM_005131.1 | 1140 | aagatggtagagcatatattaaa | 292518 | 682537 | see also 3606 line | | |
| 3610 | THOC1 | NM_005131.1 | 1517 | acggagaagccctcacttcttcc | 292523 | 682548 | see also 3606 line | | |
| 3611 | SLC12A6 | NM_005135.1 | 1752 | acctacctgggctttacttctaa | 256911 | 640798 | cell_cycle | - | - |
| 3612 | DGCR2 | NM_005137.1 | 1021 | tggatgaagggttctacttcacc | 174555 | 568444 | receptor_acitivity | receptor | - |
| 3613 | GIF | NM_005142.2 | 588 | gacctgtatgtacaacaagatcc | 180607 | 573917 | - | binding | pernicious anemia |
| 3614 | TBX19 | NM_005149.1 | 1016 | ctgacagctggacttccttatcc | 12738 | 449117 | transcription_regulator | RNA polymerase II transcription factor | adrenocorticotropic hormone deficiency |
| 3615 | USP10 | NM_005153.1 | 1352 | accccgtgggctgatcaataaag | 188769 | 582799 | - | ubiquitin C-terminal hydrolase | - |
| 3616 | USP10 | NM_005153.1 | 1353 | ccccgtgggctgatcaataaagg | 188770 | 582800 | see also 3615 line | | |
| 3617 | USP8 | NM_005154.1 | 348 | ctctacctcagttcttcactaaa | 190384 | 452437 | - | ubiquitin-specific protease | - |
| 3618 | USP8 | NM_005154.1 | 552 | ttcaagcaacagcaggattattt | 190389 | 452445 | see also 3617 line | | |
| 3619 | USP8 | NM_005154.1 | 2639 | agctcttactggacttcgtaact | 190435 | 452486 | see also 3617 line | | |
| 3620 | USP8 | NM_005154.1 | 2824 | ccctgtggacaggacagtataga | 190439 | 452490 | see also 3617 line | | |
| 3621 | USP8 | NM_005154.1 | 3184 | tagcatccacaagtaaatgtaca | 190457 | 452497 | see also 3617 line | | |
| 3622 | PPT2 | NM_005155.5 | 1344 | gggattcttttgggttgaagact | 200261 | 592089 | - | - | - |
| 3623 | ROD1 | NM_005156.3 | 922 | caccagccttaatgtgaaatata | 54378 | 486037 | - | - | - |

Figure 46

| | | | | | | | | | |
|---|---|---|---|---|---|---|---|---|---|
| 3624 | ROD1 | NM_005156.3 | 1263 | cacatgggcttttatcctattt | 54384 | 486045 | see also 3623 line | | |
| 3625 | ROD1 | NM_005156.3 | 1299 | atgtacatcgagtgaagattatg | 54385 | 486047 | see also 3623 line | | |
| 3626 | ACTC | NM_005159.2 | 1000 | ccccctgagcgtaaatactctgt | 286217 | 659061 | - | translation regulator | cardiomyopathy |
| 3627 | ADRBK2 | NM_005160.2 | 226 | ttgacaagattttcaatcagaaa | 71776 | 792555 | kinase_related、 gpcr、 signal_transduction | G-protein coupled receptor kinase | - |
| 3628 | ABCD2 | NM_005164.2 | 640 | ctgcggcatatgctctgaaaacc | 70795 | 492580 | - | ATP-binding cassette (ABC) transporter | - |
| 3629 | ABCD2 | NM_005164.2 | 973 | agcctcggactttcatcatcaaa | 70811 | 492588 | see also 3628 line | | |
| 3630 | ABCD2 | NM_005164.2 | 976 | ctcggactttcatcatcaaatta | 70813 | 492589 | see also 3628 line | | |
| 3631 | ABCD2 | NM_005164.2 | 1571 | gtggtacatcatgatagaacagt | 70834 | 492614 | see also 3628 line | | |
| 3632 | ABCD2 | NM_005164.2 | 1769 | aaggattatgtcttcatacaaag | 70842 | 492621 | see also 3628 line | | |
| 3633 | APLP1 | NM_005166.2 | 1025 | agcgtaggatgcgccagattaat | 177429 | 570593 | apoptosis | - | - |
| 3634 | APLP1 | NM_005166.2 | 1026 | gcgtaggatgcgccagattaatg | 177430 | 570594 | see also 3633 line | | |
| 3635 | MGC19531 | NM_005167.4 | 1831 | ccggctgggcctacaaaaagatc | 236821 | 531029 | - | - | breast cancer、 pancreatic cancer、 melanoma |
| 3636 | MGC19531 | NM_005167.4 | 1832 | cggctgggcctacaaaaagatcg | 236822 | 531030 | see also 3635 line | | |
| 3637 | ARHE | NM_005168.2 | 179 | atctatcatggatcctaatcaga | 108344 | 518665 | - | Rho small monomeric GTPase | |
| 3638 | ARHE | NM_005168.2 | 190 | atcctaatcagaacgtgaaatgc | 108345 | 518666 | see also 3637 line | | |
| 3639 | ARHE | NM_005168.2 | 664 | tcgaatgctcagctttacagtcg | 108360 | 518674 | see also 3637 line | | |
| 3640 | ARHE | NM_005168.2 | 823 | cagttgctacggacttacgaaag | 108365 | 518680 | see also 3637 line | | |
| 3641 | ATP6V0A1 | NM_005177.2 | 338 | atgtgaagaaatggatcgaaagc | 266206 | 653528 | - | hydrogen-transporting two-sector ATPase | - |
| 3642 | ATP6V0A1 | NM_005177.2 | 1471 | atgagaatgagatgtttagcact | 266229 | 653556 | see also 3641 line | | |
| 3643 | CACNA1F | NM_005183.1 | 4418 | gaggatctggtctgaatatgacc | 138791 | 545055 | channel | voltage-gated calcium channel | night blindness |
| 3644 | CBL | NM_005188.1 | 831 | ttgatctgacctgcaatgattat | 2064 | 533219 | transcription_regulator、 signal_transduction | transcription factor | Jacobsen syndrome |
| 3645 | CBL | NM_005188.1 | 834 | atctgacctgcaatgattatatt | 2065 | 533220 | see also 3644 line | | |
| 3646 | CBL | NM_005188.1 | 1160 | ttgtttcctgatggacgaaatca | 2072 | 533229 | see also 3644 line | | |
| 3647 | CBL | NM_005188.1 | 1720 | tggctcccttcataaagacaaac | 2085 | 533242 | see also 3644 line | | |

Figure 46

| | | | | | | | | | |
|---|---|---|---|---|---|---|---|---|---|
| 3648 | CCNC | NM_005190.2 | 230 | atccaagcattaggtgaacatct | 300218 | 668668 | cell_cycle、transcripti on_regulator | cyclin-dependent protein kinase、 regulator | - |
| 3649 | CCNC | NM_005190.2 | 367 | ggcatccaaagtagaggaatttg | 300222 | 668677 | see also 3648 line | | |
| 3650 | CCNC | NM_005190.2 | 529 | ttgttgcttgatagtgtatcatc | 300226 | 668679 | see also 3648 line | | |
| 3651 | CCNC | NM_005190.2 | 536 | ttgatagtgtatcatccttatag | 300227 | 668680 | see also 3648 line | | |
| 3652 | CCNC | NM_005190.2 | 619 | gaggatagtgaatgatacctaca | 300229 | 668681 | see also 3648 line | | |
| 3653 | CEBPB | NM_005194.2 | 1167 | cagcaccctgcggaacttgttca | 2154 | 457011 | transcription_regulator | translation regulator | - |
| 3654 | CEBPB | NM_005194.2 | 1170 | caccctgcggaacttgttcaagc | 2155 | 457012 | see also 3653 line | | |
| 3655 | COL13A1 | NM_005203.3 | 817 | aactgctggacgagaaatggaag | 333572 | 696105 | - | - | |
| 3656 | CRK | NM_005206.2 | 688 | gcctcagtatcggctctgattgg | 252589 | 533914 | transcription_regulator | - | - |
| 3657 | CRK | NM_005206.2 | 723 | tggtaaaggttacgaagattaat | 252593 | 533915 | see also 3656 line | | |
| 3658 | CRK | NM_005206.2 | 729 | aggttacgaagattaatgtgagt | 252594 | 533916 | see also 3656 line | | |
| 3659 | CRYBA1 | NM_005208.3 | 214 | aagtggcgcctggattggttatg | 289522 | 659911 | - | structural constituent of eye lens | - |
| 3660 | CRYBA1 | NM_005208.3 | 217 | tggcgcctggattggttatgagc | 289523 | 659912 | see also 3659 line | | |
| 3661 | CSF1R | NM_005211.2 | 2687 | ctggctagggacatcatgaatga | 77610 | 498854 | receptor_acitivity、sig nal_transduction、kina se_related | hematopoietic-associated factor 1A complex | anemia macrocytic、diabetes、arthritis |
| 3662 | DLX3 | NM_005220.2 | 464 | tactcgcccaagtcggaatatac | 2685 | 442992 | transcription_regulator | transcription factor | tricho-dento-osseous syndrome |
| 3663 | EDG3 | NM_005226.2 | 1388 | cgctcgacccaagcagaagtaaa | 173423 | 501260 | - | lysosphingolipid and lysophosphatidic acid receptor | - |
| 3664 | EFNA4 | NM_005227.2 | 462 | acctggagagacttactactaca | 206035 | 694588 | receptor_acitivity | - | - |
| 3665 | EFNA4 | NM_005227.2 | 465 | tggagagacttactactacatct | 206036 | 694590 | see also 3664 line | | |
| 3666 | EFNA4 | NM_005227.2 | 467 | gagagacttactactacatctcg | 206037 | 694591 | see also 3664 line | | |
| 3667 | ELK1 | NM_005229.2 | 419 | ggccttgcggtactactatgaca | 3142 | 443353 | transcription_regulator | translation regulator | soft tissue sarcoma、bladder cancer、synovial sarcoma |
| 3668 | EPHA1 | NM_005232.2 | 264 | tgggacacccctgtacatgtacc | 81316 | 501537 | receptor_acitivity、kin ase_related、signal_tra nsduction | ephrin receptor | non-small-cell lung cancer |
| 3669 | EPHA1 | NM_005232.2 | 320 | accactggcttcgctccaattgg | 81317 | 501538 | see also 3668 line | | |
| 3670 | EPHA1 | NM_005232.2 | 324 | ctggcttcgctccaattggatct | 81318 | 501539 | see also 3668 line | | |
| 3671 | ERCC4 | NM_005236.1 | 363 | tagaataccttcagatttaatta | 26080 | 484345 | - | endodeoxyribonucleas e | xeroderma pigmentosum |

Figure 46

| 3672 | ETS1 | NM_005238.1 | 302 | gccgactctcaccatcatcaaga | 3515 | 443603 | transcription_regulator | C-ets-1 protein | chondrosarcoma |
|---|---|---|---|---|---|---|---|---|---|
| 3673 | EVI1 | NM_005241.1 | 839 | aggtctgccataaatcctatact | 26202 | 696462 | - | molecular_function unknown | leukemia |
| 3674 | EVI1 | NM_005241.1 | 843 | ctgccataaatcctatactcagt | 26203 | 696463 | see also 3673 line | | |
| 3675 | EVI1 | NM_005241.1 | 943 | ttcagcactacgtcttccttaaa | 26204 | 696464 | see also 3673 line | | |
| 3676 | EVI1 | NM_005241.1 | 945 | cagcactacgtcttccttaaata | 26205 | 696465 | see also 3673 line | | |
| 3677 | EVI1 | NM_005241.1 | 948 | cactacgtcttccttaaataaac | 26207 | 696466 | see also 3673 line | | |
| 3678 | EVI1 | NM_005241.1 | 1063 | acgtccatggttaatatgagtca | 26210 | 696469 | see also 3673 line | | |
| 3679 | EVI1 | NM_005241.1 | 1201 | taccacaggcctcctttgatacc | 26211 | 696472 | see also 3673 line | | |
| 3680 | EVI1 | NM_005241.1 | 1668 | aggagctgtgaatgattctataa | 26219 | 696492 | see also 3673 line | | |
| 3681 | EVI1 | NM_005241.1 | 2507 | acctaacacggcacttgagaacc | 26240 | 696508 | see also 3673 line | | |
| 3682 | EWSR1 | NM_005243.1 | 531 | taggatatggacagagtaactac | 51493 | 473769 | transcription_regulator | - | extraskeletal myxoid chrondrosarcoma、 desmoplastic small round cell tumor、 extraskeletal myxoid chondrosarcoma、 chondrosarcoma、 sarcoma、 maxillary tumor、 paraspinal neurogenic tumor、 clear cell sarcoma、 Ewing sarcoma、 lipomatous tumors、 Askin tumor (esthesioneuroblastoma)、 myxoid liposarcoma、 Ewing's sarcoma、 clear cell sarcoma of the soft tissue、 malignant melanoma、 Ewing's tumours |
| 3683 | EWSR1 | NM_005243.1 | 532 | aggatatggacagagtaactaca | 51494 | 473770 | see also 3682 line | | |
| 3684 | EWSR1 | NM_005243.1 | 710 | cagagtagctatggtcaacaaag | 51497 | 473773 | see also 3682 line | | |
| 3685 | EWSR1 | NM_005243.1 | 857 | cccagtagcatgggtgtttatgg | 51500 | 473776 | see also 3682 line | | |
| 3686 | FER | NM_005246.1 | 2228 | atgatcatcccaatattgtcaaa | 82179 | 502228 | kinase_related | protein tyrosine kinase | - |
| 3687 | FER | NM_005246.1 | 2271 | aagacagcctgtctacatcatta | 82180 | 502230 | see also 3686 line | | |
| 3688 | FER | NM_005246.1 | 2660 | gtccgtaccctggaatgacaaat | 82190 | 502237 | see also 3686 line | | |
| 3689 | FER | NM_005246.1 | 2661 | tccgtaccctggaatgacaaatc | 82191 | 502238 | see also 3686 line | | |
| 3690 | FOXG1B | NM_005249.3 | 1431 | cgggccagaccagttactttttc | 4047 | 443972 | transcription_regulator | transcription factor | - |
| 3691 | FOXG1B | NM_005249.3 | 1460 | gtcccgcacccgtcaatgacttc | 4050 | 443973 | see also 3690 line | | |
| 3692 | FOXG1B | NM_005249.3 | 1461 | tcccgcacccgtcaatgacttcg | 4051 | 443974 | see also 3690 line | | |
| 3693 | FOXG1B | NM_005249.3 | 1550 | ctgccctgtgagtctttaagacc | 4053 | 443975 | see also 3690 line | | |
| 3694 | FOXG1B | NM_005249.3 | 1603 | tgggggactgtctgattatttca | 4056 | 443976 | see also 3690 line | | |
| 3695 | FOXG1B | NM_005249.3 | 1605 | ggggactgtctgattatttcaca | 4057 | 443977 | see also 3690 line | | |
| 3696 | FOXG1B | NM_005249.3 | 1645 | ttcttccaaccctttaatacatt | 4058 | 443978 | see also 3690 line | | |

Figure 46

| 3697 | FOSL2 | NM_005253.2 | 870 | caggattatcccgggaactttga | 3998 | 459550 | - | transcription factor | - |
|------|-------|-------------|-----|-------------------------|------|--------|---|----------------------|---|
| 3698 | GAS2 | NM_005256.2 | 185 | ttgtggttaaccaatctattagg | 306253 | 674272 | cell_cycle、apoptosis | - | - |
| 3699 | GATA6 | NM_005257.3 | 1556 | cagaagcgcgtgccttcatcacg | 4437 | 444368 | transcription_regulator | translation regulator | - |
| 3700 | GDF8 | NM_005259.1 | 671 | atgaaagacggtacaaggtatac | 168624 | 565594 | signal_transduction | growth factor | - |
| 3701 | GFRA1 | NM_005264.2 | 1834 | agccacataaccacaaaatcaat | 248779 | 634986 | receptor_acitivity、sig nal_transduction | - | - |
| 3702 | GPM6A | NM_005277.2 | 250 | ctggagacacactggatgttttt | 325643 | 692320 | - | - | - |
| 3703 | GPM6B | NM_005278.2 | 434 | aactgcacggtgagtttaaaaca | 280417 | 692352 | - | molecular_function unknown | - |
| 3704 | GPR12 | NM_005288.1 | 673 | atgaggcacgcccatcagatagc | 239407 | 696790 | gpcr、receptor_acitivit y、signal_transduction | rhodopsin-like receptor | - |
| 3705 | GPR22 | NM_005295.1 | 217 | atcaactctgtcagtaacattat | 239652 | 624708 | gpcr、receptor_acitivit y、signal_transduction | rhodopsin-like receptor | - |
| 3706 | GPR22 | NM_005295.1 | 224 | ctgtcagtaacattattacaatg | 239653 | 624709 | see also 3705 line | | |
| 3707 | GPR22 | NM_005295.1 | 1018 | ggcccaagtgaccttttagtaaa | 239679 | 624737 | see also 3705 line | | |
| 3708 | GPR22 | NM_005295.1 | 1019 | gcccaagtgaccttttagtaaaa | 239680 | 624738 | see also 3705 line | | |
| 3709 | GPR22 | NM_005295.1 | 1020 | cccaagtgaccttttagtaaaat | 239681 | 624739 | see also 3705 line | | |
| 3710 | GPR23 | NM_005296.1 | 164 | ttgatgattccttcaagtataat | 239690 | 624756 | gpcr、receptor_acitivit y、signal_transduction | purinergic nucleotide receptor、 G-protein coupled | - |
| 3711 | GPR34 | NM_005300.2 | 530 | aagtggacactaggtgtgattct | 239763 | 624833 | gpcr、receptor_acitivit y、signal_transduction | purinergic nucleotide receptor、 G-protein coupled | - |
| 3712 | GPR37 | NM_005302.2 | 1650 | ctgcaagatcgtgccctatatag | 239811 | 624886 | gpcr、receptor_acitivit y、signal_transduction | rhodopsin-like receptor | Parkinson disease |
| 3713 | HAS2 | NM_005328.1 | 862 | aacctaccctgggattaaagttg | 213525 | 601885 | - | transferase、 transferring glycosyl groups | lipoblastoma |
| 3714 | HAS2 | NM_005328.1 | 1810 | ctgccttagaggaaatatcgtca | 213553 | 601913 | see also 3713 line | | |
| 3715 | HAS2 | NM_005328.1 | 2172 | atgacatggtgcttgatgtatga | 213572 | 601929 | see also 3713 line | | |
| 3716 | HCFC1 | NM_005334.1 | 262 | ttgggggtctggccaatgatagc | 4597 | 696909 | transcription_regulator | transcription factor | - |
| 3717 | HDLBP | NM_005336.1 | 444 | aaggtgaacaagcaaaaatctgc | 51902 | 484484 | - | lipid transporter | atherosclerosis |
| 3718 | HDLBP | NM_005336.1 | 1307 | cagaacctggccaaaatcactca | 51925 | 484510 | see also 3717 line | | |

Figure 46

| | | | | | | | | | |
|---|---|---|---|---|---|---|---|---|---|
| 3719 | HDLBP | NM_005336.1 | 1531 | cagaatcaaagaccagtacaagg | 51930 | 484516 | see also 3717 line | | |
| 3720 | HDLBP | NM_005336.1 | 1702 | aagatttcatcgcacaatcattg | 51938 | 484524 | see also 3717 line | | |
| 3721 | HDLBP | NM_005336.1 | 1883 | ctggtggaaaatagctattcaat | 51946 | 484534 | see also 3717 line | | |
| 3722 | HDLBP | NM_005336.1 | 1919 | ttcaaacagtttcacaagaatat | 51949 | 484537 | see also 3717 line | | |
| 3723 | HDLBP | NM_005336.1 | 1946 | gggaaaggaggcgcaaacattaa | 51951 | 484540 | see also 3717 line | | |
| 3724 | HEM1 | NM_005337.2 | 767 | gccatgattaaccctgctaattc | 325695 | 769653 | - | - | - |
| 3725 | HEM1 | NM_005337.2 | 770 | atgattaaccctgctaattcaga | 325696 | 769654 | see also 3724 line | | |
| 3726 | HEM1 | NM_005337.2 | 793 | tacaatggcctgtgagtatctgt | 325698 | 769656 | see also 3724 line | | |
| 3727 | HIP1 | NM_005338.4 | 839 | tacaaagttgaaagatctgttct | 117491 | 523171 | - | structural constituent of cytoskeleton | - |
| 3728 | HIP2 | NM_005339.3 | 284 | tccccctaaggtccggtttatca | 229817 | 616450 | transcription_regulator | - | - |
| 3729 | HIP2 | NM_005339.3 | 333 | tccgtcacaggggctatttgttt | 229821 | 616452 | see also 3728 line | | |
| 3730 | HIP2 | NM_005339.3 | 341 | aggggctatttgtttggatatcc | 229822 | 616453 | see also 3728 line | | |
| 3731 | HIP2 | NM_005339.3 | 394 | tccgcacggtattattgtcattg | 229828 | 616458 | see also 3728 line | | |
| 3732 | HSPA5 | NM_005347.2 | 939 | ggccactaatggagatactcatc | 85228 | 503934 | - | Hsp70/Hsp90 organizing protein | - |
| 3733 | HSPA5 | NM_005347.2 | 1299 | gactcgaattccaaagattcagc | 85237 | 503944 | see also 3732 line | | |
| 3734 | RBPSUH | NM_005349.1 | 106 | tagggaagctatgcgaaattatt | 37901 | 465644 | transcription_regulator | - | - |
| 3735 | RBPSUH | NM_005349.1 | 111 | aagctatgcgaaattatttaaaa | 37902 | 465647 | see also 3734 line | | |
| 3736 | MADH4 | NM_005359.3 | 450 | accatacagagaacattggatgg | 6272 | 461328 | transcription_regulator | Smad3/Smad4 complex | colorectal cancer、 pancreatic carcinoma、 lung cancer、 pancreatic cancer、 polyposis、 colorectal cancers |
| 3737 | MADH4 | NM_005359.3 | 575 | ttgtcagtatgcgtttgacttaa | 6276 | 461331 | see also 3736 line | | |
| 3738 | MADH4 | NM_005359.3 | 577 | gtcagtatgcgtttgacttaaaa | 6277 | 461332 | see also 3736 line | | |
| 3739 | MADH4 | NM_005359.3 | 611 | ctgtgtgaatccatatcactacg | 6278 | 461334 | see also 3736 line | | |
| 3740 | MADH4 | NM_005359.3 | 1000 | agccagctacttaccatcataac | 6288 | 461346 | see also 3736 line | | |
| 3741 | MADH4 | NM_005359.3 | 1439 | tagtgaccacgcggtctttgtac | 6299 | 461350 | see also 3736 line | | |
| 3742 | MCF2 | NM_005369.2 | 347 | ttgttatgctgagctcagttagt | 296075 | 664673 | - | guanyl-nucleotide release factor | - |
| 3743 | MPL | NM_005373.1 | 364 | ctgcacctctgggtgaagaatgt | 244198 | 445935 | receptor_acitivity、 signal_transduction | translation regulator | megakaryocytic thrombocytopenia、 leukemia |
| 3744 | MPL | NM_005373.1 | 1917 | ttcctacctaccactaagctatt | 244211 | 445948 | see also 3743 line | | |
| 3745 | MPL | NM_005373.1 | 1918 | tcctacctaccactaagctattg | 244212 | 445949 | see also 3743 line | | |
| 3746 | NKTR | NM_005385.2 | 270 | ttctcagacatatgtccaaaaac | 177278 | 619087 | - | peptidyl-prolyl cis-trans isomerase | - |
| 3747 | NKTR | NM_005385.2 | 520 | tggcaaatcgagggaaacatacc | 177285 | 619094 | see also 3746 line | | |

Figure 46

| 3748 | NKTR | NM_005385.2 | 1409 | atcatggtcctataatggatatt | 177307 | 619120 | see also 3746 line | | |
|---|---|---|---|---|---|---|---|---|---|
| 3749 | NKTR | NM_005385.2 | 4004 | tcggcctgggtctctctttgatg | 177370 | 619178 | see also 3746 line | | |
| 3750 | NKTR | NM_005385.2 | 4231 | atcgatcaagaagctactctaga | 177379 | 619184 | see also 3746 line | | |
| 3751 | NNAT | NM_005386.2 | 224 | tgggtaggattcgcttttcgaaa | 272957 | 685409 | - | - | - |
| 3752 | NNAT | NM_005386.2 | 225 | gggtaggattcgcttttcgaaat | 272958 | 685410 | see also 3751 line | | |
| 3753 | NUP98 | NM_005387.3 | 700 | aagctggagttagcactaacata | 269558 | 712596 | - | - | chronic myeloid leukemia、 leukemia |
| 3754 | NUP98 | NM_005387.3 | 900 | agctcctccaccactaattcagg | 269562 | 712598 | see also 3753 line | | |
| 3755 | NUP98 | NM_005387.3 | 2558 | gggtgaagggctaaataggaagg | 269606 | 712644 | see also 3753 line | | |
| 3756 | NUP98 | NM_005387.3 | 3569 | ccccaactggactcttgctaata | 269642 | 712671 | see also 3753 line | | |
| 3757 | NUP98 | NM_005387.3 | 3570 | cccaactggactcttgctaatag | 269643 | 712672 | see also 3753 line | | |
| 3758 | NUP98 | NM_005387.3 | 3665 | gcccaatccagtagctgttaaac | 269644 | 712679 | see also 3753 line | | |
| 3759 | PDCL | NM_005388.2 | 342 | cccaaaaggtgtgatcaatgact | 253464 | 649532 | gpcr、 signal_transduction | regulator of G-protein signaling | - |
| 3760 | PDK3 | NM_005391.1 | 471 | ttctggatcggttttataccaac | 96165 | 511638 | kinase_related、 signal _transduction | pyruvate dehydrogenase (lipoamide) kinase | - |
| 3761 | PDK3 | NM_005391.1 | 915 | atcgtcttttaactacatgtat | 96184 | 511656 | see also 3760 line | | |
| 3762 | PDK3 | NM_005391.1 | 1146 | ccgcatggcgccattacaagacc | 96190 | 511663 | see also 3760 line | | |
| 3763 | PHF2 | NM_005392.2 | 1518 | aagatgcccaagccatccaaaat | 9249 | 463559 | transcription_regulator | - | - |
| 3764 | PHF2 | NM_005392.2 | 2129 | aagggacttgtccttcttgttgg | 9254 | 463569 | see also 3763 line | | |
| 3765 | PHF2 | NM_005392.2 | 3232 | ccgctgccaaaggaaaacgtacg | 9262 | 463577 | see also 3763 line | | |
| 3766 | PNLIPRP2 | NM_005396.3 | 161 | cccgctttcttctgtacacaaat | 199773 | 591521 | - | triacylglycerol lipase | - |
| 3767 | PTPN14 | NM_005401.3 | 605 | gaggccacaagatatcagtatta | 193429 | 586790 | phosphatase | structural molecule | - |
| 3768 | PTPN14 | NM_005401.3 | 620 | cagtattacctgcaagtcaaaaa | 193431 | 586792 | see also 3767 line | | |
| 3769 | PTPN14 | NM_005401.3 | 2340 | ctcagtatcaccacaagaagacc | 193462 | 586826 | see also 3767 line | | |
| 3770 | RALA | NM_005402.2 | 525 | cgctgcaattagagacaactact | 110444 | 520193 | signal_transduction | protein binding | - |
| 3771 | RALA | NM_005402.2 | 527 | ctgcaattagagacaactacttc | 110445 | 520194 | see also 3770 line | | |
| 3772 | ROCK1 | NM_005406.1 | 3 | gtcgactggggacagttttgaga | 99205 | 513525 | kinase_related、 apoptosis、 signal_transduction | - | - |
| 3773 | ROCK1 | NM_005406.1 | 529 | ttctatactgcagaagtagttct | 99215 | 513537 | see also 3772 line | | |
| 3774 | ROCK1 | NM_005406.1 | 551 | ttgcattggatgcaatccattcc | 99216 | 513538 | see also 3772 line | | |
| 3775 | ROCK1 | NM_005406.1 | 1652 | atgacttacttaggacagaatcg | 99236 | 513545 | see also 3772 line | | |
| 3776 | ROCK1 | NM_005406.1 | 2808 | cactgttagtcggcttgaagaag | 99269 | 513573 | see also 3772 line | | |
| 3777 | ROCK1 | NM_005406.1 | 3181 | gacatgcaagcgcaattggtaga | 99276 | 513580 | see also 3772 line | | |
| 3778 | ROCK1 | NM_005406.1 | 3188 | aagcgcaattggtagaagaatgt | 99277 | 513581 | see also 3772 line | | |

EP 1 752 536 A1

Figure 46

| No. | Gene | Accession | Pos | Sequence | ID1 | ID2 | Function | Class | Disease |
|---|---|---|---|---|---|---|---|---|---|
| 3779 | SIX3 | NM_005413.1 | 430 | gccaaacttgccgattctcacc | 11285 | 441336 | transcription_regulator | transcription factor | holoprosencephaly |
| 3780 | SIX3 | NM_005413.1 | 743 | gcgtgcgaggccatcaacaaaca | 11286 | 441338 | see also 3779 line | | |
| 3781 | SLC20A1 | NM_005415.3 | 555 | ttctccgtgggagccaatgatgt | 251489 | 637746 | receptor_acitivity、kinase_related | | |
| 3782 | SLC20A1 | NM_005415.3 | 565 | gagccaatgatgtagcaaattct | 251490 | 637749 | see also 3781 line | | |
| 3783 | SLC20A1 | NM_005415.3 | 680 | ggccaaagtgagcgaaaccatcc | 251491 | 637750 | see also 3781 line | | |
| 3784 | SLC20A1 | NM_005415.3 | 796 | aactcgtgcttcgttttgaag | 251495 | 637757 | see also 3781 line | | |
| 3785 | SLC20A1 | NM_005415.3 | 1099 | caccgttgctgggctttgacaaa | 251503 | 637768 | see also 3781 line | | |
| 3786 | ST5 | NM_005418.2 | 2189 | aggagcgggagcttttgagtac | 333771 | 740684 | - | | |
| 3787 | TECTA | NM_005422.1 | 444 | atgggaggaagtcacgtttatg | 289092 | 614734 | - | | deafness |
| 3788 | TECTA | NM_005422.1 | 5661 | cagggaccgcacgatcaatgtgg | 289174 | 614816 | see also 3787 line | | |
| 3789 | TP73 | NM_005427.1 | 1579 | accccagcctcgtcagtttta | 13473 | 449656 | transcription_regulator、apoptosis、cell_cycle、signal_transduction | translation regulator | bladder cancer、lung cancer、neuroblastoma |
| 3790 | VEGFC | NM_005429.2 | 1342 | tggaccccacaaagaactagaca | 169581 | 566298 | signal_transduction、cell_cycle | growth factor | neuroblastoma |
| 3791 | NCOA4 | NM_005437.1 | 244 | tccgggctgaacagcaaattaaa | 112242 | 709117 | transcription_regulator | translation regulator | thyroid cancer |
| 3792 | RQCD1 | NM_005444.1 | 455 | aagaagtaatcaactttttatta | 320588 | 679588 | - | | |
| 3793 | CSPG6 | NM_005445.2 | 375 | aagtttcacttcgaagagttatt | 77789 | 499056 | cell_cycle、signal_transduction | | |
| 3794 | CSPG6 | NM_005445.2 | 400 | tgccaaaaggatcagrtattct | 77791 | 499058 | see also 3793 line | | |
| 3795 | CSPG6 | NM_005445.2 | 1227 | agggtcgaggaagccagtttaca | 77817 | 499074 | see also 3793 line | | |
| 3796 | CSPG6 | NM_005445.2 | 1598 | aaggccatttaaatggaataga | 77825 | 499085 | see also 3793 line | | |
| 3797 | CSPG6 | NM_005445.2 | 1930 | gaggtacaatcccagatttgaca | 77838 | 499101 | see also 3793 line | | |
| 3798 | CSPG6 | NM_005445.2 | 2206 | aaggattaataatgaaattgatc | 77852 | 499110 | see also 3793 line | | |
| 3799 | CSPG6 | NM_005445.2 | 2788 | gagtatggagcgctggaaaaata | 77861 | 499126 | see also 3793 line | | |
| 3800 | ZNF297 | NM_005453.3 | 1678 | gccccgtgtgcaacaaaagttc | 45056 | 470769 | transcription_regulator | | |
| 3801 | ZNF297 | NM_005453.3 | 1679 | cccgtgtgcaacaacaaagttca | 45057 | 470770 | see also 3800 line | | |
| 3802 | HNRPDL | NM_005463.2 | 1011 | agcagatgaccgttaaaatgttt | 19563 | 484581 | - | | |
| 3803 | HNRPDL | NM_005463.2 | 1710 | atgattatactgggtataactat | 19579 | 484594 | see also 3802 line | | |
| 3804 | AKT3 | NM_005465.3 | 85 | agcgatgttaccattgtgaaaga | 71976 | 493669 | kinase_related、signal_transduction | | |
| 3805 | AKT3 | NM_005465.3 | 221 | tacttatccctcaacaacttt | 71980 | 493673 | see also 3804 line | | |
| 3806 | AKT3 | NM_005465.3 | 734 | aagaccgtttgttgtgatg | 71989 | 493688 | see also 3804 line | | |

Figure 46

| | | | | | | | | |
|---|---|---|---|---|---|---|---|---|
| 3807 | AKT3 | NM_005465.3 | 814 | gaggaccgcacacgtttctatgg | 71992 | 493689 | see also 3804 line | |
| 3808 | AKT3 | NM_005465.3 | 1219 | ctcttgataaaggatccaaataa | 72003 | 493703 | see also 3804 line | |
| 3809 | MED6 | NM_005466.1 | 376 | cagacttgggatcagttataaac | 249953 | 728439 | receptor_acitivity, tra nscription_regulator | - |
| 3810 | KCNE3 | NM_005472.2 | 452 | gcgtagtgaccctatcatgtgt | 260986 | 644348 | channel | voltage-gated potassium channel |
| 3811 | HDAC5 | NM_005474.3 | 1001 | gggacgcctcctcctcctacaaact | 113647 | 525928 | transcription_regulator, cell_cycle | - |
| 3812 | HDAC5 | NM_005474.3 | 3232 | ctctgtcacgcagoatgtttg | 113661 | 525956 | see also 3811 line | |
| 3813 | PIGK | NM_005482.1 | 177 | tacatccgattctgtttaatt | 187808 | 581545 | - | legumain |
| 3814 | ADPRTL2 | NM_005484.2 | 1426 | ttgcctctgcctaaagaataca | 21269 | 454581 | - | |
| 3815 | HMG2L1 | NM_005487.2 | 1518 | ctgaccatccaggtatagatttt | 28549 | 460286 | transcription_regulator | DNA binding |
| 3816 | HMG2L1 | NM_005487.2 | 1533 | tagatttgggggaacttagtaaa | 28550 | 460288 | see also 3815 line | |
| 3817 | RANBP9 | NM_005493.2 | 897 | gtcattggctgttgtgttaatct | 333865 | 709620 | - | - |
| 3818 | RANBP9 | NM_005493.2 | 945 | aagaatggacatagtttaggtat | 333867 | 709624 | see also 3817 line | |
| 3819 | RANBP9 | NM_005493.2 | 982 | taccgccaaatttgtatcctact | 333869 | 709627 | see also 3817 line | |
| 3820 | RANBP9 | NM_005493.2 | 1777 | gagaaacttcatccaatggtttc | 333899 | 709649 | see also 3817 line | |
| 3821 | RANBP9 | NM_005493.2 | 1784 | ttcatccaatggttcctaaatg | 333900 | 709650 | see also 3817 line | |
| 3822 | RANBP9 | NM_005493.2 | 1811 | cctaaacatgaccacgaaatgg | 333901 | 709651 | see also 3817 line | |
| 3823 | RANBP9 | NM_005493.2 | 1819 | atgaccacgaaatggaagattgt | 333902 | 709652 | see also 3817 line | |
| 3824 | RANBP9 | NM_005493.2 | 1821 | gaccacgaaatggaagattgtga | 333903 | 709653 | see also 3817 line | |
| 3825 | RANBP9 | NM_005493.2 | 2227 | cagtggaagactacctacattag | 333915 | 709659 | see also 3817 line | |
| 3826 | SMC4L1 | NM_005496.2 | 622 | tggctatgagcacaaaaaataa | 333923 | 452554 | cell_cycle | - |
| 3827 | SMC4L1 | NM_005496.2 | 1014 | gacggctaaatgaacctattaaa | 333943 | 452577 | see also 3826 line | |
| 3828 | SMC4L1 | NM_005496.2 | 1044 | gtcggagagttgaaatattaaat | 333946 | 452580 | see also 3826 line | |
| 3829 | SMC4L1 | NM_005496.2 | 1739 | cagtcagaacttgatatctatct | 333961 | 452594 | see also 3826 line | |
| 3830 | SMC4L1 | NM_005496.2 | 1741 | gtcagaacttgatatctatctca | 333962 | 452595 | see also 3826 line | |
| 3831 | SMC4L1 | NM_005496.2 | 1743 | cagaacttgatatctatctcagt | 333963 | 452596 | see also 3826 line | |
| 3832 | SMC4L1 | NM_005496.2 | 2181 | tagcccaagaatgtgtaaactc | 333975 | 452608 | see also 3826 line | |
| 3833 | SMC4L1 | NM_005496.2 | 3258 | aagatgcacttagtattaagttg | 334015 | 452644 | see also 3826 line | |
| 3834 | SMC4L1 | NM_005496.2 | 3262 | tgcacttagtattaagttgaaac | 334016 | 452646 | see also 3826 line | |
| 3835 | SMC4L1 | NM_005496.2 | 3979 | ttctcttcgaataatatgtttg | 334030 | 452672 | see also 3826 line | |
| 3836 | GJA7 | NM_005497.1 | 809 | aggatccgggtgctataattat | 263767 | 647341 | - | connexon channel |
| 3837 | GJA7 | NM_005497.1 | 815 | cgggtgcttataattatcctttc | 263768 | 647343 | see also 3836 line | |
| 3838 | GJA7 | NM_005497.1 | 854 | ctgctcccctggctataacatt | 263769 | 647345 | see also 3836 line | |
| 3839 | UBA2 | NM_005499.1 | 148 | ctccacatcgacctgattgatc | 235620 | 620532 | kinase_related | - |

Figure 46

| 3840 | UBA2 | NM_005499.1 | 149 | tcccacatcgacctgattgatct | 235621 | 620533 | see also 3839 line | | |
|---|---|---|---|---|---|---|---|---|---|
| 3841 | UBA2 | NM_005499.1 | 774 | atccagttaaactttttaccaag | 235653 | 620556 | see also 3839 line | | |
| 3842 | ABCA1 | NM_005502.2 | 588 | gagttgttggaaactttaacaaa | 67930 | 490541 | - | sterol transporter | atherosclerosis、 high density lipoprotein deficiency、 Alzheimer disease |
| 3843 | ABCA1 | NM_005502.2 | 2224 | ccggtcaatgcccctcttcatga | 67961 | 490583 | see also 3842 line | | |
| 3844 | ABCA1 | NM_005502.2 | 6974 | gccaaggaccaaagtgatgatga | 68038 | 490678 | see also 3842 line | | |
| 3845 | APBA2 | NM_005503.2 | 2422 | aggagaccccgctgtacatctag | 128951 | 650534 | - | protein transporter | - |
| 3846 | BCAT1 | NM_005504.3 | 707 | acctcaacatggatagaatgtat | 217957 | 605905 | cell_cycle | branched-chain amino acid aminotransferase | hyperleucine-isoleucinemia |
| 3847 | SCARB2 | NM_005506.2 | 1644 | caccaacataccctacatcatca | 251182 | 637521 | receptor_acitivity | receptor | - |
| 3848 | CCR4 | NM_005508.2 | 232 | tggttctggtcctgttcaaatac | 124543 | 528339 | receptor_acitivity、 gpcr、 signal_transduction | C-C chemokine receptor | |
| 3849 | HMGCS2 | NM_005518.1 | 176 | tccccctggccaaaacagatact | 216547 | 603785 | - | hydroxymethylglutaryl-CoA synthase | hydroxymethylglutaryl-CoA synthase deficiency、 HMG-CoA synthase-2 deficiency |
| 3850 | HNRPH1 | NM_005520.1 | 185 | atggggctcaaggtattcgtttc | 49032 | 484598 | - | poly(U) binding | - |
| 3851 | HNRPH1 | NM_005520.1 | 186 | tggggctcaaggtattcgtttca | 49033 | 484599 | see also 3850 line | | |
| 3852 | HNRPH1 | NM_005520.1 | 189 | ggctcaaggtattcgtttcatct | 49034 | 484600 | see also 3850 line | | |
| 3853 | HNRPH1 | NM_005520.1 | 635 | gagctgaagttagaactcattat | 49036 | 484603 | see also 3850 line | | |
| 3854 | TLX1 | NM_005521.2 | 1312 | cggctcctacaacgtgaacatgg | 13426 | 449577 | transcription_regulator | transcription factor | lymphoma |
| 3855 | TLX1 | NM_005521.2 | 1716 | ccctggccaaggcgctcaaaatg | 13429 | 449581 | see also 3854 line | | |
| 3856 | HOXA1 | NM_005522.3 | 493 | caccaccaccagggttatgctgg | 5004 | 444667 | transcription_regulator | - | - |
| 3857 | HOXA11 | NM_005523.4 | 429 | cagtctcgtccaatttctatagc | 5030 | 444694 | transcription_regulator | transcription factor | - |
| 3858 | HOXA11 | NM_005523.4 | 481 | ggctttcgaccagtttttcgaga | 5031 | 444696 | see also 3857 line | | |
| 3859 | HOXA11 | NM_005523.4 | 778 | tggccaacgcacccgcaaaaagc | 5032 | 444697 | see also 3857 line | | |
| 3860 | HSD11B1 | NM_005525.2 | 143 | atggcctactactactattctgc | 219961 | 607378 | - | - | cortisone reductase deficiency、 obesity |
| 3861 | ISLR | NM_005545.2 | 1383 | gggctgctatacggttgacaacg | 131936 | 704094 | - | - | - |
| 3862 | KARS | NM_005548.1 | 778 | ccgctctaagatcatcacatata | 62935 | 484767 | - | nucleic acid binding | - |
| 3863 | KARS | NM_005548.1 | 837 | ttgaaactcccatgatgaacatc | 62938 | 484770 | see also 3862 line | | |
| 3864 | KARS | NM_005548.1 | 1061 | tacatggcctatgcagactatca | 62953 | 484775 | see also 3862 line | | |
| 3865 | LAMA1 | NM_005559.2 | 8788 | cggatatgcagctcttgtcaaag | 290673 | 537070 | - | - | - |
| 3866 | LAMC2 | NM_005562.1 | 2907 | atgggcaatgccacttttttatga | 177607 | 570691 | - | - | epidermolysis bullosa |
| 3867 | LAMC2 | NM_005562.1 | 2909 | gggcaatgccacttttttatgaag | 177608 | 570692 | see also 3866 line | | |
| 3868 | LAMC2 | NM_005562.1 | 3628 | tgcccccaggctgctacaatacc | 177616 | 570698 | see also 3866 line | | |

Figure 46

| 3869 | LMAN1 | NM_005570.2 | 148 | atcgccgtttcgagtacaaatac | 174906 | 694628 | - | mannose binding lectin | coagulation factor |
|---|---|---|---|---|---|---|---|---|---|
| 3870 | LMAN1 | NM_005570.2 | 151 | gccgtttcgagtacaaatacagc | 174907 | 694630 | see also 3869 line | | |
| 3871 | LMAN1 | NM_005570.2 | 663 | atgatcaataatggctttacacc | 174922 | 694655 | see also 3869 line | | |
| 3872 | LMAN1 | NM_005570.2 | 1028 | aggacagaatcgtattcatcttg | 174928 | 694659 | see also 3869 line | | |
| 3873 | LMNB1 | NM_005573.2 | 915 | cagttagcagatgaaactttact | 291462 | 686471 | - | structural molecule | - |
| 3874 | LMNB1 | NM_005573.2 | 919 | tagcagatgaaactttacttaaa | 291463 | 686472 | see also 3873 line | | |
| 3875 | LMNB1 | NM_005573.2 | 1793 | tggagacacatcagtcagttata | 291483 | 686488 | see also 3873 line | | |
| 3876 | LMO2 | NM_005574.2 | 1359 | tgcgggtgaaagacaaagtgtat | 310695 | 690247 | - | - | leukemia |
| 3877 | LNPEP | NM_005575.1 | 1295 | tacccacttaagaaattggattt | 157309 | 555171 | - | zinc binding | - |
| 3878 | LNPEP | NM_005575.1 | 1596 | tagatgctcgatttaaaaccatg | 157318 | 555180 | see also 3877 line | | |
| 3879 | LPP | NM_005578.1 | 1636 | tactgcgagccctgctacattaa | 281521 | 649456 | - | - | pulmonary chondroid hamartoma、 lipomatous tumors |
| 3880 | ALDH6A1 | NM_005589.2 | 663 | tgccatgatcccccttggatgt | 225312 | 611700 | - | methylmalonate-semialdehyde dehydrogenase (acylating) | - |
| 3881 | ALDH6A1 | NM_005589.2 | 1043 | agcgctgcatggctctttcaaca | 225325 | 611711 | see also 3880 line | | |
| 3882 | MUSK | NM_005592.1 | 889 | atgcatagctaccaataagcatg | 92160 | 508889 | receptor_acitivity、 kinase_related、 signal_transduction | - | - |
| 3883 | NFIA | NM_005595.1 | 525 | ctgcctccgccaggcagataaag | 7839 | 446634 | transcription_regulator | - | - |
| 3884 | NFIA | NM_005595.1 | 584 | aaggtattccgctggaaagtact | 7842 | 446636 | see also 3883 line | | |
| 3885 | NFIA | NM_005595.1 | 1214 | agcatcaccgacctgtcattaca | 7855 | 446643 | see also 3883 line | | |
| 3886 | NFIB | NM_005596.1 | 279 | gtccgtgcaattgcctatacttg | 7857 | 446646 | transcription_regulator | translation regulator | - |
| 3887 | NFIB | NM_005596.1 | 313 | aggctcgaaaacgcaagtacttt | 7859 | 446647 | see also 3886 line | | |
| 3888 | NFIB | NM_005596.1 | 314 | ggctcgaaaacgcaagtacttta | 7860 | 446648 | see also 3886 line | | |
| 3889 | NFIB | NM_005596.1 | 316 | ctcgaaaacgcaagtactttaaa | 7861 | 446649 | see also 3886 line | | |
| 3890 | NFIB | NM_005596.1 | 756 | tacgtgcaggagcaagattctgg | 7873 | 446654 | see also 3886 line | | |
| 3891 | NFIB | NM_005596.1 | 765 | gagcaagattctggacaatcagg | 7874 | 446655 | see also 3886 line | | |
| 3892 | NIT1 | NM_005600.1 | 324 | tcctgcctgaggcatttgacttc | 203963 | 594354 | - | - | - |
| 3893 | PTK2 | NM_005607.3 | 427 | aagatagtggacagtcacaaagt | 98452 | 512844 | kinase_related、 signal_transduction | - | - |
| 3894 | PTK2 | NM_005607.3 | 829 | aagcgatttttcctaagagttt | 98465 | 512861 | see also 3893 line | | |
| 3895 | PTK2 | NM_005607.3 | 860 | ctgtcaaggccaaaacactaaga | 98467 | 512862 | see also 3893 line | | |
| 3896 | PTK2 | NM_005607.3 | 1033 | ctggcaatcggcccagaagaagg | 98476 | 512865 | see also 3893 line | | |

228

Figure 46

| | | | | | | | | | | |
|---|---|---|---|---|---|---|---|---|---|---|
| 3897 | PYGM | NM_005609.1 | 3116 | atgcaccatgaccggtttaaagt | 211655 | 600009 | - | transferase、transferring glycosyl groups | McArdle disease | |
| 3898 | RBL2 | NM_005611.2 | 324 | ttggttagcatgtgccttatatg | 37637 | 465566 | cell_cycle、transcription_regulator | protein binding | endometrial cancer、lung cancer、neuroblastoma、ocular melanoma | |
| 3899 | RBL2 | NM_005611.2 | 2480 | aagtggccattcaacagatttcc | 37709 | 465619 | see also 3898 line | | | |
| 3900 | REST | NM_005612.3 | 1414 | atgggcctaaacctcttaattgc | 276093 | 702077 | transcription_regulator | - | - | |
| 3901 | SAH | NM_005622.1 | 557 | ctgcgaacagggacagttttaat | 237210 | 617532 | - | - | - | |
| 3902 | SAH | NM_005622.1 | 558 | tgcgaacagggacagttttaatt | 237211 | 617533 | see also 3901 line | | | |
| 3903 | SGK | NM_005627.2 | 706 | aacatcgtttatagagacttaaa | 100303 | 514616 | kinase_related、apoptosis | protein serine/threonine kinase | - | |
| 3904 | SOS1 | NM_005633.2 | 1971 | agctgatcgcatagctatagaga | 40525 | 467848 | signal_transduction | guanyl-nucleotide release factor | gingval fibromatosis、diabetes | |
| 3905 | SOS1 | NM_005633.2 | 2746 | ttggcaaaactcaggtctattaa | 40545 | 467868 | see also 3904 line | | | |
| 3906 | SOS1 | NM_005633.2 | 2748 | ggcaaaactcaggtctattaatc | 40546 | 467870 | see also 3904 line | | | |
| 3907 | SOS1 | NM_005633.2 | 3111 | tcctggtgttcgtccatcaaacc | 40558 | 467880 | see also 3904 line | | | |
| 3908 | SOS1 | NM_005633.2 | 3661 | aggacacctgatgttttctcaag | 40570 | 467895 | see also 3904 line | | | |
| 3909 | SYBL1 | NM_005638.3 | 202 | gagcagattctggctaagatacc | 271092 | 702489 | transcription_regulator | protein transporter | - | |
| 3910 | SYBL1 | NM_005638.3 | 211 | ctggctaagataccttctgaaaa | 271093 | 702490 | see also 3909 line | | | |
| 3911 | SYBL1 | NM_005638.3 | 212 | tggctaagataccttctgaaaat | 271094 | 702491 | see also 3909 line | | | |
| 3912 | SYBL1 | NM_005638.3 | 644 | atcttgctcgagccatgtgtatg | 271110 | 702507 | see also 3909 line | | | |
| 3913 | SYBL1 | NM_005638.3 | 648 | tgctcgagccatgtgtatgaaga | 271111 | 702508 | see also 3909 line | | | |
| 3914 | SYT1 | NM_005639.1 | 1143 | tgccatcggcaaagtctttgtgg | 173134 | 657504 | - | transporter | - | |
| 3915 | TAF7 | NM_005642.2 | 1702 | tactggatgagctcaaacaaaag | 112471 | 658067 | transcription_regulator | TFIID complex | - | |
| 3916 | TAF11 | NM_005643.2 | 437 | ctgaaccgttatgaaatgtatcg | 41486 | 468574 | transcription_regulator | protein binding | - | |
| 3917 | TAF12 | NM_005644.2 | 333 | aggtattgaccaagaagaaatta | 41503 | 468585 | transcription_regulator | protein binding | - | |
| 3918 | TAF12 | NM_005644.2 | 567 | aagaaatccgaccctacaaaaaa | 41505 | 468590 | see also 3917 line | | | |
| 3919 | TCF20 | NM_005650.1 | 5547 | gggctcccttgtaaaaaagcagc | 41805 | 468847 | transcription_regulator | - | - | |
| 3920 | TDO2 | NM_005651.1 | 281 | atcataactcatcaagcttatga | 224360 | 610841 | - | tryptophan 2、3 dioxygenase | alcoholism | |
| 3921 | TERF2 | NM_005652.2 | 1566 | aaccgaacagctgtgatgattaa | 20737 | 469024 | cell_cycle | DNA binding | - | |

Figure 46

| | | | | | | | | | |
|---|---|---|---|---|---|---|---|---|---|
| 3922 | TERF2 | NM_005652.2 | 1567 | accgaacagctgtgatgattaag | 20738 | 469025 | see also 3921 line | | |
| 3923 | TERF2 | NM_005652.2 | 1591 | atcgctggcggaccatgaaaaga | 20739 | 469027 | see also 3921 line | | |
| 3924 | TP53BP1 | NM_005657.1 | 302 | atctcgacaccttcctaatctcc | 20870 | 541638 | transcription_regulator、signal_transduction | translation regulator | - |
| 3925 | TP53BP1 | NM_005657.1 | 2239 | tgcacctttctctgactgaaact | 20915 | 541679 | see also 3924 line | | |
| 3926 | TP53BP1 | NM_005657.1 | 4658 | gtcatccaatggctactttact | 20952 | 541714 | see also 3924 line | | |
| 3927 | TP53BP1 | NM_005657.1 | 5600 | ttgtcgaacccggaagtacttcc | 20971 | 541726 | see also 3924 line | | |
| 3928 | TP53BP1 | NM_005657.1 | 5869 | ctgtgaagcagcaccattcaagt | 20979 | 541732 | see also 3924 line | | |
| 3929 | UFD1L | NM_005659.3 | 166 | tgctagcagggcctaatgacagg | 190756 | 670825 | - | ubiquitin-specific protease | catch 22 syndrome(-) |
| 3930 | EVI5 | NM_005665.2 | 672 | agccttggacaggaggtttatt | 131157 | 696542 | - | - | - |
| 3931 | EVI5 | NM_005665.2 | 1786 | cacagaaccagatcaatagtaac | 131187 | 696567 | see also 3930 line | | |
| 3932 | RNF103 | NM_005667.2 | 1545 | cagtggacgcaagattgaagtag | 319777 | 699481 | - | - | Alzheimer disease |
| 3933 | RNF103 | NM_005667.2 | 1823 | tgccatcatacatacttagaact | 319785 | 699495 | see also 3932 line | | |
| 3934 | RNF103 | NM_005667.2 | 1892 | cccttcaggccatggattcattt | 319788 | 699498 | see also 3932 line | | |
| 3935 | RNF103 | NM_005667.2 | 2020 | aagcgatttgtggttctcataag | 319796 | 699504 | see also 3932 line | | |
| 3936 | RNF103 | NM_005667.2 | 2021 | agcgatttgtggttctcataagc | 319797 | 699505 | see also 3932 line | | |
| 3937 | RNF103 | NM_005667.2 | 2363 | acctcttccacccgattgcttct | 319809 | 699518 | see also 3932 line | | |
| 3938 | SIAT8D | NM_005668.3 | 623 | tggtgatgtcatacactatgtgc | 213075 | 600926 | - | - | - |
| 3939 | SIAT8D | NM_005668.3 | 1014 | aagcacgtggagtggggttaatgc | 213082 | 600935 | see also 3938 line | | |
| 3940 | SIAT8D | NM_005668.3 | 1017 | cacgtggagtgggttaatgcatt | 213083 | 600936 | see also 3938 line | | |
| 3941 | SIAT8D | NM_005668.3 | 1020 | gtggagtgggttaatgcattaat | 213084 | 600938 | see also 3938 line | | |
| 3942 | D8S2298E | NM_005671.1 | 763 | ctggaggacataggaataactgt | 320502 | 802421 | - | - | - |
| 3943 | RBM10 | NM_005676.3 | 896 | ttcgccttcgtcgagtttagtca | 53883 | 485658 | - | - | - |
| 3944 | RBM10 | NM_005676.3 | 898 | cgccttcgtcgagtttagtcact | 53884 | 485659 | see also 3943 line | | |
| 3945 | ABCC5 | NM_005688.1 | 835 | cactgacttgggcattgaattac | 70502 | 492306 | - | organic anion transporter | lung cancer |
| 3946 | ABCC5 | NM_005688.1 | 1042 | taggcatgatttataatgtaatt | 70508 | 492308 | see also 3945 line | | |
| 3947 | ABCC5 | NM_005688.1 | 2253 | gaccccctcagtgccttagatgc | 70527 | 492334 | see also 3945 line | | |
| 3948 | ABCC5 | NM_005688.1 | 4162 | gccactgtaagattctgatttta | 70572 | 492372 | see also 3945 line | | |
| 3949 | ABCB6 | NM_005689.1 | 2206 | ctctggctgcatccgaatagatg | 69844 | 492006 | - | ATP-binding cassette (ABC) transporter | - |
| 3950 | DNM1L | NM_005690.2 | 1226 | gtggccttaacactattgacatt | 108805 | 519181 | - | - | - |
| 3951 | DNM1L | NM_005690.2 | 1882 | cagcgagattgtgaggttattga | 108826 | 519199 | see also 3950 line | | |
| 3952 | DNM1L | NM_005690.2 | 1958 | tgccaaaggcagtaatgcatttt | 108832 | 519204 | see also 3950 line | | |
| 3953 | ABCC9 | NM_005691.1 | 422 | gggtaatggcctttattacaaaa | 70649 | 492446 | receptor_acitivity | - | diabetes mellitus、diabetes |

Figure 46

| 3954 | ABCC9 | NM_005691.1 | 2043 | gggcagtggtttagctacattat | 70700 | 492494 | see also 3953 line | | |
|------|-------|-------------|------|-------------------------|-------|--------|--------------------|---|---|
| 3955 | ABCC9 | NM_005691.1 | 4535 | atgcaggccttgttttagtcttt | 70772 | 492551 | see also 3953 line | | |
| 3956 | ABCF2 | NM_005692.3 | 1029 | tacgggtaattatgatcagtacg | 71026 | 492804 | - | - | - |
| 3957 | ABCF2 | NM_005692.3 | 1338 | gccttgcatctacaataatctag | 71033 | 492815 | see also 3956 line | | |
| 3958 | ABCF2 | NM_005692.3 | 1489 | gtcaagatagggcgttaccatca | 71035 | 492820 | see also 3956 line | | |
| 3959 | NR1H3 | NM_005693.1 | 1071 | atgaatgagctgcaactcaatga | 8249 | 447025 | transcription_regulator, receptor_acitivity | LXRalpha/RXRalpha hetedimer | atherosclerosis |
| 3960 | PDCD7 | NM_005707.1 | 1387 | caggcatgattgggatcagtacc | 334195 | 677351 | apoptosis | - | - |
| 3961 | GPC6 | NM_005708.2 | 1759 | gaccggctggtcacagacataaa | 246424 | 633744 | receptor_acitivity | - | - |
| 3962 | COL4A3BP | NM_005713.1 | 527 | ggcaggatcgttgggtagttttg | 205528 | 596297 | kinase_related | - | - |
| 3963 | COL4A3BP | NM_005713.1 | 530 | aggatcgttgggtagttttgaaa | 205529 | 596298 | see also 3962 line | | |
| 3964 | COL4A3BP | NM_005713.1 | 533 | atcgttgggtagttttgaaaaat | 205530 | 596299 | see also 3962 line | | |
| 3965 | COL4A3BP | NM_005713.1 | 1179 | aacactttctcattgtattgaac | 205549 | 596323 | see also 3962 line | | |
| 3966 | COL4A3BP | NM_005713.1 | 1827 | aactatagaaaactttcatgtgg | 205558 | 596347 | see also 3962 line | | |
| 3967 | COL4A3BP | NM_005713.1 | 1953 | accagccttgactgaaaatgacc | 205564 | 596355 | see also 3962 line | | |
| 3968 | UST | NM_005715.1 | 529 | caggcttactaaaaatgaacaaa | 217824 | 796007 | - | - | - |
| 3969 | ARPC4 | NM_005718.2 | 359 | tgcgagcagagaacttctttatc | 286848 | 659218 | - | structural constituent of cytoskeleton | - |
| 3970 | TM4SF9 | NM_005723.2 | 826 | ctgatgattggaacctaaatatt | 309537 | 675447 | - | - | - |
| 3971 | TM4SF8 | NM_005724.4 | 568 | cagcattcagaaagtgtataaga | 309520 | 693457 | - | - | - |
| 3972 | TSPAN-2 | NM_005725.2 | 50 | gggcctgcggtgcatcaagtacc | 309554 | 691504 | - | - | - |
| 3973 | RAD50 | NM_005732.2 | 511 | aagacgaccatcattgaatgtct | 98687 | 513032 | - | - | - |
| 3974 | RAD50 | NM_005732.2 | 513 | gacgaccatcattgaatgtctaa | 98688 | 513033 | see also 3973 line | | |
| 3975 | RAD50 | NM_005732.2 | 2748 | atgcctgacagatgttacaatta | 98771 | 513092 | see also 3973 line | | |
| 3976 | KIF20A | NM_005733.1 | 2358 | ttgtgatgacatcttaatcaaac | 87329 | 505402 | - | protein transporter | - |
| 3977 | HIPK3 | NM_005734.2 | 1047 | gtcggccagtcatgtatcaaaga | 84854 | 503553 | kinase_related | - | - |
| 3978 | HIPK3 | NM_005734.2 | 2275 | cagcctgccactaccaagaaaaa | 84874 | 503586 | see also 3977 line | | |
| 3979 | HIPK3 | NM_005734.2 | 3258 | tagtgttaccagtaatccattca | 84905 | 503603 | see also 3977 line | | |
| 3980 | ACTR1A | NM_005736.2 | 100 | gacaacggatccggtgtgattaa | 286293 | 659098 | - | structural constituent of cytoskeleton | - |
| 3981 | ACTR1A | NM_005736.2 | 103 | aacggatccggtgtgattaaagc | 286294 | 659099 | see also 3980 line | | |

EP 1 752 536 A1

Figure 46

| 3982 | ZNF263 | NM_005741.3 | 884 | ctgtaaaggagagggcattatcc | 15185 | 450102 | transcription_regulator | transcription factor | - |
|------|--------|-------------|-----|------------------------|-------|--------|------------------------|---------------------|---|
| 3983 | ZNF263 | NM_005741.3 | 1025 | ggcatcaggatcctagtaagagg | 15188 | 450105 | see also 3982 line | | |
| 3984 | ARIH1 | NM_005744.2 | 720 | agccacttcaattgggataaaga | 125887 | 670735 | - | ubiquitin-like-protein ligase | - |
| 3985 | ARIH1 | NM_005744.2 | 792 | gagtgtcatgtaattaatccaag | 125888 | 670737 | see also 3984 line | | |
| 3986 | ARIH1 | NM_005744.2 | 942 | cagtgctggagtgaatatttaac | 125895 | 670743 | see also 3984 line | | |
| 3987 | ARIH1 | NM_005744.2 | 1459 | atggatctgcctggtacaactgt | 125913 | 670761 | see also 3984 line | | |
| 3988 | ARIH1 | NM_005744.2 | 1717 | gtcgtgccacactcatgtacact | 125916 | 670765 | see also 3984 line | | |
| 3989 | ARIH1 | NM_005744.2 | 1736 | cacttatgtcttcgctttctacc | 125919 | 670766 | see also 3984 line | | |
| 3990 | ARIH1 | NM_005744.2 | 1832 | gggctaccttgaacgagatattt | 125921 | 670767 | see also 3984 line | | |
| 3991 | ARIH1 | NM_005744.2 | 1833 | ggctaccttgaacgagatatttc | 125922 | 670768 | see also 3984 line | | |
| 3992 | ARIH1 | NM_005744.2 | 1843 | aacgagatatttcccaagattct | 125923 | 670769 | see also 3984 line | | |
| 3993 | ARIH1 | NM_005744.2 | 1913 | gagtcgacgaagggttttgttac | 125926 | 670773 | see also 3984 line | | |
| 3994 | ARIH1 | NM_005744.2 | 1916 | tcgacgaagggttttgttacagc | 125927 | 670774 | see also 3984 line | | |
| 3995 | BCAP31 | NM_005745.5 | 371 | aaggtgaacctccagaacaatcc | 267935 | 650575 | apoptosis | - | - |
| 3996 | TOB1 | NM_005749.2 | 881 | aggtagtagtaccaatggaatgt | 136824 | 526164 | - | protein binding | - |
| 3997 | GPR64 | NM_005756.1 | 507 | tggagtcctgtctctaagtgaat | 243357 | 630846 | gpcr、receptor_acitivity、signal_transduction | - | - |
| 3998 | GPR64 | NM_005756.1 | 2325 | gactatatccccagataactatg | 243406 | 630905 | see also 3997 line | | |
| 3999 | GPR64 | NM_005756.1 | 2459 | tgctgaacgtcagcatgttcatt | 243410 | 630909 | see also 3997 line | | |
| 4000 | GPR64 | NM_005756.1 | 2488 | ctggttcagctctgtcgaattaa | 243412 | 630910 | see also 3997 line | | |
| 4001 | GPR64 | NM_005756.1 | 2489 | tggttcagctctgtcgaattaaa | 243413 | 630911 | see also 3997 line | | |
| 4002 | GPR64 | NM_005756.1 | 2731 | tggaggcggtatctttgttgtgg | 243422 | 630922 | see also 3997 line | | |
| 4003 | GPR64 | NM_005756.1 | 2790 | aactgctactaatggtttaaaga | 243424 | 630923 | see also 3997 line | | |
| 4004 | GPR64 | NM_005756.1 | 2792 | ctgctactaatggtttaaagaag | 243425 | 630924 | see also 3997 line | | |
| 4005 | GPR64 | NM_005756.1 | 3083 | agcggggaagcttacactttatt | 243435 | 630935 | see also 3997 line | | |
| 4006 | MBNL2 | NM_005757.3 | 1332 | gagttccagcgaggaaactgtgc | 64057 | 484942 | - | - | - |
| 4007 | MBNL2 | NM_005757.3 | 1422 | accgtaaccgtttgtatggatta | 64058 | 484943 | see also 4006 line | | |
| 4008 | MBNL2 | NM_005757.3 | 1428 | accgtttgtatggattacataaa | 64059 | 484944 | see also 4006 line | | |
| 4009 | MBNL2 | NM_005757.3 | 1453 | ggcgttgcatgagggagaaatgc | 64060 | 484946 | see also 4006 line | | |
| 4010 | MBNL2 | NM_005757.3 | 1874 | gccacagccaatcagataattct | 64067 | 484947 | see also 4006 line | | |
| 4011 | ABI-2 | NM_005759.3 | 699 | ctccagtggtaccaaatgattac | 21073 | 812048 | kinase_related | - | - |
| 4012 | ABI-2 | NM_005759.3 | 704 | gtggtaccaaatgattacgtacc | 21075 | 812049 | see also 4011 line | | |
| 4013 | ABI-2 | NM_005759.3 | 723 | tacctagcccaacccgtaatatg | 21076 | 812050 | see also 4011 line | | |
| 4014 | ABI-2 | NM_005759.3 | 724 | acctagcccaacccgtaatatgg | 21077 | 812051 | see also 4011 line | | |

Figure 46

| 4015 | ABI-2 | NM_005759.3 | 770 | aggacagcttctgtgtgaatcaaag | 21078 | 812052 | see also 4011 line | | |
|---|---|---|---|---|---|---|---|---|---|
| 4016 | ABI-2 | NM_005759.3 | 995 | gggggctcgttgccctatagacg | 21083 | 812058 | see also 4011 line | | |
| 4017 | CBF2 | NM_005760.1 | 1287 | aggctactcttccgctcaaatat | 23709 | 668796 | transcription_regulator | translation regulator | - |
| 4018 | PLXNC1 | NM_005761.1 | 1457 | ttcttggtgagaatttgacttca | 171970 | 637252 | receptor_acitivity | - | - |
| 4019 | PLXNC1 | NM_005761.1 | 3289 | cccttccttgactacaaacattt | 172024 | 637295 | see also 4018 line | | |
| 4020 | PLXNC1 | NM_005761.1 | 4115 | ttggccactatgagatatcaaat | 172054 | 637310 | see also 4018 line | | |
| 4021 | PLXNC1 | NM_005761.1 | 4136 | atggatccactataaaagtcttt | 172056 | 637313 | see also 4018 line | | |
| 4022 | PLXNC1 | NM_005761.1 | 4314 | gtcgaccaaggtggcaattcatt | 172060 | 637315 | see also 4018 line | | |
| 4023 | AASS | NM_005763.2 | 692 | gtgatgctggctatgaaatatct | 223292 | 610513 | - | - | - |
| 4024 | AASS | NM_005763.2 | 874 | gtgtatgggacggtgttaagtcg | 223303 | 610522 | see also 4023 line | | |
| 4025 | ATP6AP2 | NM_005765.2 | 397 | gagaatgcagttccttttagtct | 204548 | 729523 | receptor_acitivity | hydrolase | - |
| 4026 | DHRS9 | NM_005771.3 | 1819 | ctctggggtctgatcaataatgc | 226648 | 614078 | - | - | - |
| 4027 | DHRS9 | NM_005771.3 | 2025 | gggctatactccatccaaaatg | 226655 | 614086 | see also 4026 line | | |
| 4028 | DHRS9 | NM_005771.3 | 2302 | tgcatggaccacgctctaacaag | 226664 | 614096 | see also 4026 line | | |
| 4029 | DHRS9 | NM_005771.3 | 2305 | atggaccacgctctaacaagtct | 226665 | 614097 | see also 4026 line | | |
| 4030 | RCL1 | NM_005772.2 | 1030 | gcgtagactcgaccaaccaaagc | 232364 | 617398 | - | RNA-3'-phosphate cyclase | - |
| 4031 | RBM6 | NM_005777.1 | 1452 | ttcaggatcaagattataggacc | 37827 | 485866 | - | RNA binding | - |
| 4032 | RBM6 | NM_005777.1 | 2320 | tgggaagatggtagctgtaaacc | 37857 | 485872 | see also 4031 line | | |
| 4033 | RBM6 | NM_005777.1 | 2339 | aacctggccactggaaaacgaag | 37858 | 485873 | see also 4031 line | | |
| 4034 | RBM6 | NM_005777.1 | 2380 | ttctgaccacatgcattactatc | 37859 | 485875 | see also 4031 line | | |
| 4035 | RBM6 | NM_005777.1 | 2450 | gactggtcttcagatacaaatcg | 37861 | 485877 | see also 4031 line | | |
| 4036 | RBM6 | NM_005777.1 | 2515 | tgctactggctactattatgacc | 37864 | 485883 | see also 4031 line | | |
| 4037 | RBM6 | NM_005777.1 | 3460 | tgctgttcgaagagtcatgtttg | 37876 | 485891 | see also 4031 line | | |
| 4038 | RBM6 | NM_005777.1 | 3471 | gagtcatgtttgctcgatataaa | 37877 | 485892 | see also 4031 line | | |
| 4039 | RBM5 | NM_005778.1 | 158 | gacaaaagagtgagtagaacaga | 37729 | 485812 | cell_cycle | RNA binding | - |
| 4040 | RBM5 | NM_005778.1 | 481 | gagcgatattcgagaaatgatgg | 37741 | 485821 | see also 4039 line | | |
| 4041 | RBM5 | NM_005778.1 | 574 | cgccttcgtggagttttatcact | 37742 | 485822 | see also 4039 line | | |
| 4042 | RBM5 | NM_005778.1 | 725 | aacaatttcaggaaaagactaaa | 37747 | 485828 | see also 4039 line | | |
| 4043 | RBM5 | NM_005778.1 | 1440 | agcctagcactagcacaagtaca | 37764 | 485843 | see also 4039 line | | |
| 4044 | RNF41 | NM_005785.2 | 1096 | gtcaacgagctgattgaaaatgc | 334265 | 723159 | - | - | - |
| 4045 | ALG3 | NM_005787.3 | 449 | aggtacctcccttcgtcttttc | 213193 | 758492 | - | transferase、transferring glycosyl groups | congenital disorder of glycosylation |
| 4046 | TOPORS | NM_005802.2 | 899 | aagatctttgcggaaaattcaag | 235237 | 620205 | - | - | - |
| 4047 | TOPORS | NM_005802.2 | 1849 | aagagaaacgatctacatcattg | 235276 | 620241 | see also 4046 line | | |

Figure 46

| | | | | | | | | |
|---|---|---|---|---|---|---|---|---|
| 4048 | TOPORS | NM_005802.2 | 2178 | aggtctcgtagcagtgatcatg | 235281 | 620247 | | see also 4046 line |
| 4049 | TOPORS | NM_005802.2 | 2754 | cggagcctaagtgtagagatagt | 235302 | 620265 | | see also 4046 line |
| 4050 | TOPORS | NM_005802.2 | 2756 | gagcctaagtgtagagatagtt | 235303 | 620266 | | see also 4046 line |
| 4051 | PSMD14 | NM_005805.1 | 633 | tggatccattcagatgtaaaa | 302294 | 688026 | - | - |
| 4052 | PSMD14 | NM_005805.1 | 756 | agccatctatccaggcattaat | 302296 | 688032 | | see also 4051 line |
| 4053 | PSMD14 | NM_005805.1 | 757 | gccatctatccaggcattaattc | 302297 | 688033 | | see also 4051 line |
| 4054 | OLIG2 | NM_005806.1 | 614 | gcggactggtgacgcgagatctacg | 34352 | 463222 | DNA binding | transcription_regulator |
| 4055 | PRG4 | NM_005807.1 | 157 | gccacctgcaactgtgattataa | 309268 | 711344 | - | camptodactyly, osteosarcoma |
| 4056 | PRKCN | NM_005813.2 | 2590 | atggtcaacagatacttgttgc | 97478 | 512370 | protein kinase C | kinase_related |
| 4057 | PRKCN | NM_005813.2 | 3090 | aactcgcattggagaacgttaca | 97488 | 512374 | | see also 4056 line |
| 4058 | PRKCN | NM_005813.2 | 3094 | cgcattggagaacgttacattac | 97490 | 512375 | | see also 4056 line |
| 4059 | PRKCN | NM_005813.2 | 3099 | tggagaacgttacattacacacatg | 97491 | 512376 | | see also 4056 line |
| 4060 | DSCR1L1 | NM_005822.1 | 258 | taccaatcaggaggttaaggaaa | 125609 | 679663 | protein phosphatase 2B binding | phosphatase |
| 4061 | RASGRP2 | NM_005825.2 | 547 | cccggaaggacaactccaattcc | 151868 | 552845 | - | signal_transduction |
| 4062 | HNRPR | NM_005826.2 | 93 | ggctaatcaggtgaatggtaatg | 52137 | 484660 | RNA binding | - |
| 4063 | HNRPR | NM_005826.2 | 107 | atggtaatgcggtacagttaaaa | 52138 | 484661 | | see also 4062 line |
| 4064 | HNRPR | NM_005826.2 | 108 | tggtaatgcggtacagttaaaag | 52139 | 484662 | | see also 4062 line |
| 4065 | HNRPR | NM_005826.2 | 241 | cagacaggattggtagccttatgt | 52143 | 484664 | | see also 4062 line |
| 4066 | HNRPR | NM_005826.2 | 243 | gacaggattggtagccttatgtcg | 52144 | 484665 | | see also 4062 line |
| 4067 | SRRM1 | NM_005839.1 | 1251 | cagcagtcaaacgtacaagaaa | 47361 | 471407 | - | - |
| 4068 | SRRM1 | NM_005839.1 | 1252 | agcagtcaaacgtacaagaaaa | 47362 | 471408 | | see also 4067 line |
| 4069 | IRX5 | NM_005853.2 | 913 | agcctcagcgactgcgattttaa | 5545 | 445071 | transcription factor | transcription_regulator |
| 4070 | IRX5 | NM_005853.2 | 914 | gcctcagcgactcggattttag | 5546 | 445072 | | see also 4069 line |
| 4071 | IRX5 | NM_005853.2 | 918 | cagcgactcggattttaaggagc | 5547 | 445073 | | see also 4069 line |
| 4072 | ZMPSTE24 | NM_005857.2 | 1000 | ttgtttgacactctactagaaga | 184945 | 579209 | metalloexopeptidase | - |
| 4073 | AKAP8 | NM_005858.2 | 139 | ggccagctggcaaggttatgaaa | 21283 | 606865 | protein kinase A anchor protein | kinase_related, signal_transduction |
| 4074 | STAG1 | NM_005862.1 | 420 | cagtgttacaggattcaactaat | 307870 | 673796 | - | cell_cycle |
| 4075 | STAG1 | NM_005862.1 | 740 | gacatcgcacttctgattttaat | 307879 | 673808 | | see also 4074 line |
| 4076 | STAG1 | NM_005862.1 | 1047 | gagcttttaggcatacaagtacc | 307890 | 673825 | | see also 4074 line |
| 4077 | STAG1 | NM_005862.1 | 1520 | ttcaaggatcgcattgatcaat | 307901 | 673836 | | see also 4074 line |
| 4078 | STAG1 | NM_005862.1 | 1966 | gagtgctcttatagagctaatgg | 307914 | 673849 | | see also 4074 line |
| 4079 | STAG1 | NM_005862.1 | 2394 | ttgtagatcgattcaatcattct | 307929 | 673861 | | see also 4074 line |
| 4080 | STAG1 | NM_005862.1 | 2668 | tccttccaaagagagatttgttgg | 307938 | 673875 | | see also 4074 line |

Figure 46

| 4081 | STAG1 | NM_005862.1 | 3851 | cagatgcagatctcttggttagg | 307964 | 673912 | see also 4074 line | | |
|------|-------|-------------|------|-------------------------|--------|--------|--------------------|--|--|
| 4082 | STAG1 | NM_005862.1 | 3866 | tggttaggccagccgaagttaga | 307965 | 673913 | see also 4074 line | | |
| 4083 | STAG1 | NM_005862.1 | 4103 | gagctaaggccagacttctttga | 307974 | 673917 | see also 4074 line | | |
| 4084 | STAG1 | NM_005862.1 | 4131 | cagctatcatagaagatgattca | 307976 | 673919 | see also 4074 line | | |
| 4085 | SAP18 | NM_005870.3 | 79 | gcgcgttacccaggaggaaatta | 113297 | 669304 | transcription_regulator | transcription co-repressor | - |
| 4086 | SMNDC1 | NM_005871.2 | 766 | ggccaggtaaagaggagtatttt | 47293 | 478679 | apoptosis | - | - |
| 4087 | APEG1 | NM_005876.3 | 2799 | gtggcgatgctggtttctacact | 308368 | 493932 | - | - | - |
| 4088 | DNAJA2 | NM_005880.2 | 350 | atgggcaatcagagtagaagtcg | 299307 | 668393 | - | chaperone | - |
| 4089 | DNAJA2 | NM_005880.2 | 924 | gacgtcagattgtggtgaaatac | 299325 | 668411 | see also 4088 line | | |
| 4090 | DNAJA2 | NM_005880.2 | 1007 | cagtatcgtaatccctttgaaaa | 299330 | 668416 | see also 4088 line | | |
| 4091 | DNAJA2 | NM_005880.2 | 1212 | ggcgtgaagcctataatgatagc | 299338 | 668424 | see also 4088 line | | |
| 4092 | BCKDK | NM_005881.1 | 1135 | cacctagacactccctacaatgt | 73978 | 496014 | kinase_related、signal_transduction | two-component sensor molecule | - |
| 4093 | TEB4 | NM_005885.2 | 581 | tgctgtcaacggaaaatttgttg | 334366 | 776403 | - | - | - |
| 4094 | TEB4 | NM_005885.2 | 1019 | gacttgatggatcactagttttt | 334376 | 776410 | see also 4093 line | | |
| 4095 | TEB4 | NM_005885.2 | 1211 | tagcaataacactgataatttgt | 334383 | 776419 | see also 4093 line | | |
| 4096 | TEB4 | NM_005885.2 | 1668 | ttggcttcctatacgtataatta | 334397 | 776434 | see also 4093 line | | |
| 4097 | TEB4 | NM_005885.2 | 1669 | tggcttcctatacgtataattaa | 334398 | 776435 | see also 4093 line | | |
| 4098 | GOLGA3 | NM_005895.2 | 1119 | ttgatcctgagctcatgttaaac | 273658 | 535635 | - | - | - |
| 4099 | IDH1 | NM_005896.2 | 290 | cacgaatcatttgggaattgatt | 220238 | 607499 | - | isocitrate dehydrogenase (NADP+) | - |
| 4100 | IDH1 | NM_005896.2 | 644 | atcaatacagagcaactgatttt | 220254 | 607507 | see also 4099 line | | |
| 4101 | IDH1 | NM_005896.2 | 651 | cagagcaactgattttgttgttc | 220255 | 607508 | see also 4099 line | | |
| 4102 | IDH1 | NM_005896.2 | 680 | ctggaaaagtagagataaacctac | 220256 | 607509 | see also 4099 line | | |
| 4103 | IDH1 | NM_005896.2 | 1355 | aggacttggctgcttgcattaaa | 220267 | 607525 | see also 4099 line | | |
| 4104 | M11S1 | NM_005898.2 | 1340 | cacttgatcctgccattgtatct | 325779 | 706543 | - | - | - |
| 4105 | M11S1 | NM_005898.2 | 1736 | tgcaaacggtgttcaatatgaat | 325793 | 706554 | see also 4104 line | | |
| 4106 | M11S1 | NM_005898.2 | 2192 | atggatatcagcagaatttcaag | 325806 | 706563 | see also 4104 line | | |
| 4107 | M11S1 | NM_005898.2 | 2282 | gagggatgccgcaaatgaacact | 325808 | 706565 | see also 4104 line | | |
| 4108 | M11S1 | NM_005898.2 | 2296 | atgaacactcagcaagtgaatta | 325809 | 706566 | see also 4104 line | | |
| 4109 | M17S2 | NM_005899.2 | 91 | cacaggttactctaaatgtgact | 334439 | 697862 | - | - | ovarian cancer |
| 4110 | MADH1 | NM_005900.1 | 1384 | aaccggaattccactattgaaaa | 253557 | 537318 | - | transcriptional activator | - |
| 4111 | MADH2 | NM_005901.2 | 685 | ttgaaaactgcgaatatgctttt | 133677 | 526398 | signal_transduction、tr anscription_regulator | protein binding | colorectal cancer、lung cancer、colorectal cancers |

235

EP 1 752 536 A1

Figure 46

| 4112 | MADH2 | NM_005901.2 | 1285 | ttgctgagtgcctaagtgatagt | 133703 | 526412 | see also 4111 line | | |
|---|---|---|---|---|---|---|---|---|---|
| 4113 | MADH5 | NM_005903.4 | 1033 | gcctatatgccacctgatgatca | 253585 | 537339 | transcription_regulator、signal_transduction | transcriptional activator | - |
| 4114 | MADH5 | NM_005903.4 | 1227 | atcttctactagtgtgttagtag | 253596 | 537347 | see also 4113 line | | |
| 4115 | MADH5 | NM_005903.4 | 1230 | ttctactagtgtgttagtagatg | 253597 | 537348 | see also 4113 line | | |
| 4116 | MADH5 | NM_005903.4 | 1297 | ttgttgtcaaatgttaatcgtaa | 253602 | 537353 | see also 4113 line | | |
| 4117 | MADH5 | NM_005903.4 | 1300 | ttgtcaaatgttaatcgtaattc | 253603 | 537354 | see also 4113 line | | |
| 4118 | MADH5 | NM_005903.4 | 1426 | cagagtaggaactgcaactttca | 253606 | 537360 | see also 4113 line | | |
| 4119 | MADH7 | NM_005904.1 | 580 | cacgcactcggtgctcaagaaac | 133722 | 669029 | kinase_related、transcription_regulator、signal_transduction | Smad3/Smad4 complex | - |
| 4120 | MADH7 | NM_005904.1 | 581 | acgcactcggtgctcaagaaact | 133723 | 669030 | see also 4119 line | | |
| 4121 | MADH7 | NM_005904.1 | 1194 | agctcaattcggacaacaagagt | 133736 | 669032 | see also 4119 line | | |
| 4122 | MAN1A1 | NM_005907.2 | 1119 | gaggccattcaagcagtttgttt | 334500 | 550008 | - | mannosyl-oligosaccharide 1、2-alpha-mannosidase | - |
| 4123 | MAN1A1 | NM_005907.2 | 1121 | ggccattcaagcagtttgtttgg | 334502 | 550009 | see also 4122 line | | |
| 4124 | MAN1A1 | NM_005907.2 | 1603 | aggcctttatcctaactatctga | 334510 | 550024 | see also 4122 line | | |
| 4125 | MAP1B | NM_005909.2 | 473 | ctcgacactctgcaagattctct | 291588 | 675460 | - | - | - |
| 4126 | MAP1B | NM_005909.2 | 3455 | gccgagaacctgcatcttcaatt | 291642 | 675498 | see also 4125 line | | |
| 4127 | MAP1B | NM_005909.2 | 3456 | ccgagaacctgcatcttcaattc | 291643 | 675499 | see also 4125 line | | |
| 4128 | MAP1B | NM_005909.2 | 3544 | gaccagcctgaggaattcactgc | 291645 | 675500 | see also 4125 line | | |
| 4129 | MAP1B | NM_005909.2 | 5058 | atgcccaagaccgatgtcaattt | 291680 | 675544 | see also 4125 line | | |
| 4130 | MAP1B | NM_005909.2 | 5059 | tgcccaagaccgatgtcaatttc | 291681 | 675545 | see also 4125 line | | |
| 4131 | MAPT | NM_005910.2 | 1144 | gcggcagtgtgcaaatagtctac | 287475 | 537443 | - | - | Parkinsonism-dementia complex、Alzheimer disease、dementia、disinhibition-dementia-Parkinsonism-amyotrophy complex、pallidopontonigral degeneration(-)、Pick disease of brain、Wilhelmsen-Lynch disease (WLD)、Parkinson disease、progressive supranuclear palsy (PSP) |
| 4132 | MAPT | NM_005910.2 | 1145 | cggcagtgtgcaaatagtctaca | 287476 | 537444 | see also 4131 line | | |
| 4133 | MC4R | NM_005912.1 | 497 | ttgggatcatcataagttgtatc | 176439 | 625820 | gpcr、receptor_acitivity、signal_transduction | melanocortin receptor | obesity |
| 4134 | MC4R | NM_005912.1 | 498 | tgggatcatcataagttgtatct | 176440 | 625821 | see also 4133 line | | |

236

Figure 46

| 4135 | MC4R | NM_005912.1 | 627 | ttctctctatgtccacatgttcc | 176441 | 625822 | see also 4133 line | | |
|---|---|---|---|---|---|---|---|---|---|
| 4136 | MCM6 | NM_005915.4 | 1275 | cagctaagagccaatttctcaag | 31949 | 461574 | phosphatase、cell_cycle、transcription_regulator | DNA dependent adenosinetriphosphatase | - |
| 4137 | MCM6 | NM_005915.4 | 1599 | gacactatgacagatcaaaatca | 31959 | 461584 | see also 4136 line | | |
| 4138 | MDH1 | NM_005917.2 | 91 | cagctggtcaaattgcatattca | 220727 | 697766 | - | malic enzyme | - |
| 4139 | MAP3K4 | NM_005922.1 | 679 | tccagatgtggatctcaataagc | 89300 | 507181 | kinase_related | - | - |
| 4140 | MAP3K4 | NM_005922.1 | 3285 | aagaagttgttcgtttgatgtct | 89377 | 507225 | see also 4139 line | | |
| 4141 | MAP3K5 | NM_005923.3 | 3783 | ttcttcggaatcataacatcaag | 89514 | 507341 | kinase_related、apoptosis | protein tyrosine kinase | - |
| 4142 | MEP1B | NM_005925.1 | 666 | cagtaatgcactacagtaaaact | 157796 | 558346 | - | zinc binding | - |
| 4143 | MIPEP | NM_005932.1 | 685 | gagcagtggacctcaatgttaaa | 157935 | 558486 | - | zinc binding | - |
| 4144 | MLLT6 | NM_005937.1 | 333 | gcctcatgatcgcttcaacaaga | 32872 | 461869 | transcription_regulator | DNA binding | leukemia |
| 4145 | MLLT6 | NM_005937.1 | 335 | ctcatgatcgcttcaacaagacc | 32873 | 461870 | see also 4144 line | | |
| 4146 | MLLT7 | NM_005938.1 | 937 | tccgtccacgaagcagttcaaat | 7081 | 445917 | transcription_regulator、cell_cycle | transcription factor | leukemia |
| 4147 | MMP16 | NM_005941.2 | 273 | caccgactgaccccagaatgtca | 158143 | 558679 | - | - | - |
| 4148 | MMP16 | NM_005941.2 | 648 | aacgtgatgtggatataaccatt | 158153 | 558686 | see also 4147 line | | |
| 4149 | MMP16 | NM_005941.2 | 1236 | atggatacccaatgcaaattact | 158166 | 558698 | see also 4147 line | | |
| 4150 | MMP16 | NM_005941.2 | 1242 | acccaatgcaaattacttacttc | 158167 | 558699 | see also 4147 line | | |
| 4151 | MMP16 | NM_005941.2 | 1372 | acctggttaccctcatgacttga | 158175 | 558706 | see also 4147 line | | |
| 4152 | MMP16 | NM_005941.2 | 1375 | tggttaccctcatgacttgataa | 158176 | 558707 | see also 4147 line | | |
| 4153 | MMP16 | NM_005941.2 | 1413 | ttccccctcatggtattgattca | 158178 | 558710 | see also 4147 line | | |
| 4154 | MMP16 | NM_005941.2 | 1890 | cccgccacatactgtactgtaaa | 158195 | 558719 | see also 4147 line | | |
| 4155 | MMP16 | NM_005941.2 | 1904 | tactgtaaacgctctatgcaaga | 158196 | 558722 | see also 4147 line | | |
| 4156 | MTF1 | NM_005955.1 | 1183 | cagcaatcatctttgaatcaatg | 7188 | 446120 | transcription_regulator | transcription factor | - |
| 4157 | MTF1 | NM_005955.1 | 1641 | gccccaccacaaagtactactga | 7197 | 446127 | see also 4156 line | | |
| 4158 | MTF1 | NM_005955.1 | 1699 | gtgccaactctgtcctaactaat | 7198 | 446128 | see also 4156 line | | |
| 4159 | MTF1 | NM_005955.1 | 1700 | tgccaactctgtcctaactaata | 7199 | 446129 | see also 4156 line | | |
| 4160 | MTF1 | NM_005955.1 | 1701 | gccaactctgtcctaactaataa | 7200 | 446130 | see also 4156 line | | |
| 4161 | MTF1 | NM_005955.1 | 1779 | gtcatgggagaacagaacttaca | 7201 | 446132 | see also 4156 line | | |
| 4162 | MTHFD1 | NM_005956.2 | 361 | tagattcagagaattccattaac | 92099 | 508736 | - | methylenetetrahydrofolate dehydrogenase (NADP+) | - |
| 4163 | MTHFD1 | NM_005956.2 | 1428 | gccattgatgctcggatatttca | 92117 | 508756 | see also 4162 line | | |
| 4164 | MTHFD1 | NM_005956.2 | 1467 | gacaaggctctctttaatcgttt | 92119 | 508757 | see also 4162 line | | |

Figure 46

| 4165 | MTHFD1 | NM_005956.2 | 1725 | acggcccagtttgatatctctgt | 92128 | 508766 | see also 4162 line | | |
| 4166 | MTNR1B | NM_005959.2 | 315 | tccggaacgcaggtaatttgttc | 239004 | 628077 | receptor_acitivity、gpcr | melatonin receptor | - |
| 4167 | MTNR1B | NM_005959.2 | 316 | ccggaacgcaggtaatttgttct | 239005 | 628078 | see also 4166 line | | |
| 4168 | MYLK | NM_005965.2 | 368 | tcccagccgagcagatggattc | 92498 | 509147 | kinase_related | - | - |
| 4169 | HNRPM | NM_005968.2 | 472 | atgtgaaatggcagtcacttaaa | 52115 | 484622 | receptor_acitivity | - | - |
| 4170 | HNRPM | NM_005968.2 | 830 | aagacttggaagcacagtatttg | 52119 | 484634 | see also 4169 line | | |
| 4171 | HNRPM | NM_005968.2 | 850 | ttgtagcaaatctggattataaa | 52120 | 484636 | see also 4169 line | | |
| 4172 | RNF6 | NM_005977.2 | 583 | aggcctattatcagtttattaat | 159749 | 655091 | - | - | - |
| 4173 | RNF6 | NM_005977.2 | 1318 | atggggctagaagtaatgttaca | 159769 | 655113 | see also 4172 line | | |
| 4174 | RNF6 | NM_005977.2 | 1354 | accaaagattagagccaataaga | 159771 | 655115 | see also 4172 line | | |
| 4175 | RNF6 | NM_005977.2 | 1358 | aagattagagccaataagadattac | 159772 | 655116 | see also 4172 line | | |
| 4176 | RNF6 | NM_005977.2 | 1900 | cagtggctcttcggtcaatttta | 159797 | 655128 | see also 4172 line | | |
| 4177 | RNF6 | NM_005977.2 | 1902 | gtggctcttcggtcaattttaag | 159798 | 655129 | see also 4172 line | | |
| 4178 | RNF6 | NM_005977.2 | 1903 | tggctcttcggtcaattttaagg | 159799 | 655130 | see also 4172 line | | |
| 4179 | SIX1 | NM_005982.1 | 834 | agggagaacaccgaaaacaataa | 11278 | 448434 | transcription_regulator | transcription factor | - |
| 4180 | SIX1 | NM_005982.1 | 991 | gccacgccaggagctcaaactat | 11279 | 448436 | see also 4179 line | | |
| 4181 | SIX1 | NM_005982.1 | 994 | acgccaggagctcaaactattct | 11281 | 448437 | see also 4179 line | | |
| 4182 | SKP2 | NM_005983.2 | 1435 | tgcaaaagcccagttgtctatga | 219800 | 607241 | cell_cycle | - | - |
| 4183 | AKR1D1 | NM_005989.1 | 1020 | tcctgaatacccatttcatgatg | 226338 | 755774 | - | oxidoreductase | - |
| 4184 | STK10 | NM_005990.1 | 2495 | gacgcgcatggccatgtacaaga | 101387 | 515469 | kinase_related | protein kinase CK2 | - |
| 4185 | TSNAX | NM_005999.2 | 753 | atgcggatgtgtattaacagtgt | 43100 | 541775 | - | protein transporter | - |
| 4186 | UCHL3 | NM_006002.3 | 496 | cagactgaggcaccaagtataga | 190707 | 584223 | - | ubiquitin C-terminal hydrolase | - |
| 4187 | SIAT8B | NM_006011.2 | 177 | atctaatagagctgaagttgtaa | 213009 | 600858 | - | transferase、transferring glycosyl groups | - |
| 4188 | SMARCF1 | NM_006015.3 | 2588 | tccatgaaccaatcaagcattgc | 40100 | 744362 | transcription_regulator、signal_transduction | - | - |
| 4189 | SMARCF1 | NM_006015.3 | 3078 | ttgctgccaactctatccaaaac | 40110 | 744370 | see also 4188 line | | |
| 4190 | SMARCF1 | NM_006015.3 | 3519 | acctctatcgcctctatgtgtct | 40117 | 744381 | see also 4188 line | | |
| 4191 | SMARCF1 | NM_006015.3 | 4049 | gagccaaataaggatccttatgg | 40126 | 744387 | see also 4188 line | | |
| 4192 | SMARCF1 | NM_006015.3 | 4367 | cagcaacgacatgattcctatgg | 40130 | 744390 | see also 4188 line | | |
| 4193 | SMARCF1 | NM_006015.3 | 4481 | cggccaatggatggcacatatgg | 40135 | 744393 | see also 4188 line | | |
| 4194 | SMARCF1 | NM_006015.3 | 5338 | gaggcggctcacaatgaaagaca | 40145 | 744403 | see also 4188 line | | |
| 4195 | SMARCF1 | NM_006015.3 | 5340 | ggcggctcacaatgaaagacatt | 40146 | 744404 | see also 4188 line | | |

Figure 46

| 4196 | SMARCF1 | NM_006015.3 | 5370 | cggaggcatggcgggtaatgatg | 40148 | 744405 | see also 4188 line | | |
|---|---|---|---|---|---|---|---|---|---|
| 4197 | SMARCF1 | NM_006015.3 | 5373 | aggcatggcgggtaatgatgtcc | 40149 | 744406 | see also 4188 line | | |
| 4198 | SMARCF1 | NM_006015.3 | 5843 | gtggactgctcagataagcttgg | 40156 | 744419 | see also 4188 line | | |
| 4199 | SMARCF1 | NM_006015.3 | 6726 | tggaaaccctcagcaaactcagc | 40170 | 744435 | see also 4188 line | | |
| 4200 | CD164 | NM_006016.3 | 601 | ttgggtgtgcaggctgtaatttt | 308394 | 706873 | signal_transduction | - | - |
| 4201 | CD164 | NM_006016.3 | 602 | tgggtgtgcaggctgtaattttc | 308395 | 706874 | see also 4200 line | | |
| 4202 | C10orf7 | NM_006023.1 | 774 | ttctccgaaaatggtgtgaattg | 306001 | 748570 | cell_cycle | - | - |
| 4203 | C10orf7 | NM_006023.1 | 960 | atgaagactttgtgttcgatata | 306005 | 748585 | see also 4202 line | | |
| 4204 | P11 | NM_006025.2 | 863 | ggggactcgagtggctttgaaca | 183837 | 698414 | - | serine-type endopeptidase | - |
| 4205 | P11 | NM_006025.2 | 866 | gactcgagtggctttgaacatgt | 183838 | 698415 | see also 4204 line | | |
| 4206 | CDC42BPB | NM_006035.2 | 5023 | gagatccaaaatgatatccaacc | 76025 | 807883 | kinase_related | - | |
| 4207 | SPATA2 | NM_006038.2 | 1667 | ctgcacccagtgttcaaaagtct | 284480 | 771548 | - | molecular_function unknown | - |
| 4208 | UBE4B | NM_006048.2 | 845 | tggtctcaatgtccacaacatga | 334689 | 712301 | apoptosis | - | - |
| 4209 | UBE4B | NM_006048.2 | 988 | cacgttcccagagcatggatatc | 334691 | 712303 | see also 4208 line | | |
| 4210 | UBE4B | NM_006048.2 | 1157 | ggccttacagctggtctgtaaga | 334694 | 712310 | see also 4208 line | | |
| 4211 | UBE4B | NM_006048.2 | 1438 | caggaacaagccccatgttctgc | 334698 | 712317 | see also 4208 line | | |
| 4212 | UBE4B | NM_006048.2 | 1502 | agcctctggtggagcaagtaatt | 334701 | 712318 | see also 4208 line | | |
| 4213 | DSCR3 | NM_006052.1 | 569 | tggcgtgtttgtcaacattcagt | 279519 | 673038 | - | molecular_function unknown | - |
| 4214 | LANCL1 | NM_006055.1 | 704 | ggcaaagtctccactgatgtatg | 243478 | 692488 | gpcr、receptor_acitivity、signal_transduction | - | - |
| 4215 | TFG | NM_006070.3 | 411 | atgatgaattagtgctaatgatg | 284708 | 708966 | kinase_related | - | papillary Thyroid Carcinomas、thyroid cancer |
| 4216 | TFG | NM_006070.3 | 1494 | aacctggacctggttatcgataa | 284737 | 708993 | see also 4215 line | | |
| 4217 | TRDN | NM_006073.1 | 818 | cagtatgcattctgtcgatatat | 312504 | 740369 | - | - | - |
| 4218 | TRDN | NM_006073.1 | 829 | ctgtcgatatatgattgacatat | 312505 | 740372 | see also 4217 line | | |
| 4219 | CBARA1 | NM_006077.1 | 627 | caggaacgagaaaaatttgctga | 139225 | 545431 | - | - | - |
| 4220 | CBARA1 | NM_006077.1 | 729 | tccactcctcagagaaattttga | 139228 | 545433 | see also 4219 line | | |
| 4221 | CACNG2 | NM_006078.2 | 359 | tagctgtgggaaccgactattgg | 261560 | 645043 | channel | voltage-gated calcium channel | - |
| 4222 | SEMA3A | NM_006080.1 | 800 | acgctagaataggtcagatatgc | 319999 | 684425 | receptor_acitivity | - | - |
| 4223 | SEMA3A | NM_006080.1 | 938 | tgcaggatgtattcctaatgaac | 320004 | 684432 | see also 4222 line | | |
| 4224 | SEMA3A | NM_006080.1 | 1227 | aagtcatccagccatgtacaatc | 320010 | 684438 | see also 4222 line | | |
| 4225 | SEMA3A | NM_006080.1 | 1667 | gacgcacaagacgacaagatata | 320029 | 684452 | see also 4222 line | | |
| 4226 | SEMA3A | NM_006080.1 | 1668 | acgcacaagacgacaagatataa | 320030 | 684453 | see also 4222 line | | |

Figure 46

| 4227 | SEMA3A | NM_006080.1 | 1669 | cgcacaagacgacaagatataag | 320031 | 684454 | see also 4222 line | | |
|---|---|---|---|---|---|---|---|---|---|
| 4228 | CEPT1 | NM_006090.2 | 733 | aacattgcgatttggaataattg | 209494 | 748802 | - | - | - |
| 4229 | CEPT1 | NM_006090.2 | 813 | gaccaccttttggcaatctatg | 209496 | 748806 | see also 4228 line | | |
| 4230 | CEPT1 | NM_006090.2 | 814 | accacctttttggcaatctatga | 209497 | 748807 | see also 4228 line | | |
| 4231 | CEPT1 | NM_006090.2 | 885 | tagcagggaccatattttcctgt | 209501 | 748810 | see also 4228 line | | |
| 4232 | DLC1 | NM_006094.3 | 334 | agccggacaccatgatcctaaca | 130203 | 534422 | - | - | hepatocellular carcinoma |
| 4233 | DLC1 | NM_006094.3 | 339 | gacaccatgatcctaacacaaat | 130204 | 534423 | see also 4232 line | | |
| 4234 | DLC1 | NM_006094.3 | 1414 | cagaggacacggtgttctacatc | 130224 | 534449 | see also 4232 line | | |
| 4235 | DLC1 | NM_006094.3 | 1750 | aggggatgcagcggatagtcaat | 130228 | 534457 | see also 4232 line | | |
| 4236 | ATP8A1 | NM_006095.1 | 868 | gtggaacggattacaaatgtaca | 73618 | 495221 | - | plasma membrane cation-transporting ATPase | - |
| 4237 | ATP8A1 | NM_006095.1 | 1106 | cccaggcatacttcataaattgg | 73629 | 495224 | see also 4236 line | | |
| 4238 | ATP8A1 | NM_006095.1 | 1193 | aggaacttggccaggttaaatac | 73634 | 495228 | see also 4236 line | | |
| 4239 | ATP8A1 | NM_006095.1 | 1968 | aacagccattgaggataaattac | 73658 | 495250 | see also 4236 line | | |
| 4240 | ATP8A1 | NM_006095.1 | 1997 | aagtgcctgaaaccatagaaacg | 73660 | 495252 | see also 4236 line | | |
| 4241 | ATP8A1 | NM_006095.1 | 2007 | aaccatagaaacgctaatgaaag | 73661 | 495253 | see also 4236 line | | |
| 4242 | ATP8A1 | NM_006095.1 | 2243 | ccctcaaatatgccttaaccttt | 73666 | 495258 | see also 4236 line | | |
| 4243 | ATP8A1 | NM_006095.1 | 2816 | aggttttctgggttcattgttta | 73685 | 495276 | see also 4236 line | | |
| 4244 | GNB2L1 | NM_006098.2 | 439 | ctgacaaccggcagattgtctct | 125244 | 529148 | kinase_related、signal _transduction | protein kinase C binding | - |
| 4245 | HEC | NM_006101.1 | 1225 | atgaattgcagcagactattaat | 307598 | 713265 | - | - | - |
| 4246 | SKB1 | NM_006109.2 | 317 | agggactggaatacgctaattgt | 214904 | 604813 | - | - | - |
| 4247 | SKB1 | NM_006109.2 | 1897 | ctggcgatgcagcaattccaaga | 214926 | 604844 | see also 4246 line | | |
| 4248 | PPIE | NM_006112.1 | 194 | agcagctatcgacaacatgaatg | 53547 | 474972 | - | cyclophilin-type peptidy-prolyl cis-trans isomerase | small cell lung cancer |
| 4249 | VAV3 | NM_006113.3 | 1471 | aaggacaaaatgggttagaattt | 172366 | 569724 | signal_transduction | guanyl-nucleotide release factor | - |
| 4250 | VAV3 | NM_006113.3 | 1713 | ttgggaagagtagacaattgtgg | 172374 | 569731 | see also 4249 line | | |
| 4251 | VAV3 | NM_006113.3 | 1725 | gacaattgtggcagagttaattc | 172376 | 569732 | see also 4249 line | | |
| 4252 | VAV3 | NM_006113.3 | 2164 | atgcaattagcattaagtacaat | 172390 | 569744 | see also 4249 line | | |
| 4253 | VAV3 | NM_006113.3 | 2362 | cagctggacagaggggtaataga | 172399 | 569751 | see also 4249 line | | |
| 4254 | PECI | NM_006117.1 | 167 | caggaaacgaagtgaagctaaaa | 172499 | 569868 | - | dodecenoyl-CoA delta-isomerase | - |
| 4255 | NCK1 | NM_006153.3 | 215 | gtcctggtggcgagttcgaaatt | 316890 | 607144 | - | - | melanoma |
| 4256 | NCK1 | NM_006153.3 | 216 | tcctggtggcgagttcgaaattc | 316891 | 607145 | see also 4255 line | | |
| 4257 | NCK1 | NM_006153.3 | 221 | gtggcgagttcgaaattccatga | 316892 | 607146 | see also 4255 line | | |

Figure 46

| | | | | | | | | | |
|---|---|---|---|---|---|---|---|---|---|
| 4258 | NCK1 | NM_006153.3 | 373 | ctgcatctcctgctgatgatagt | 316901 | 607154 | see also 4255 line | | |
| 4259 | NCK1 | NM_006153.3 | 1036 | gtgaatcttcgccaaatgatttc | 316930 | 607183 | see also 4255 line | | |
| 4260 | NEDD4 | NM_006154.1 | 484 | aagtcacaaatcaagagttaaag | 230226 | 537858 | - | - | - |
| 4261 | NELL2 | NM_006159.1 | 176 | ccctacagattgacgtcttaaca | 148868 | 550502 | - | structural molecule | - |
| 4262 | NELL2 | NM_006159.1 | 187 | gacgtcttaacagagttagaact | 148869 | 550503 | see also 4261 line | | |
| 4263 | NEUROD2 | NM_006160.2 | 1264 | gagaatctcttgtcttacgatat | 334909 | 462610 | transcription_regulator | transcription factor | - |
| 4264 | NFE2 | NM_006163.1 | 708 | ctcaggattatccctcaactata | 7688 | 462685 | transcription_regulator | p18(MAFK)/p45 heterodimer | - |
| 4265 | NFE2 | NM_006163.1 | 1003 | aacttgccggtagatgactttaa | 7698 | 462695 | see also 4264 line | | |
| 4266 | NFE2L2 | NM_006164.2 | 149 | gtcccagcaggacatggatttga | 7730 | 462737 | transcription_regulator | translation regulator | - |
| 4267 | NFE2L2 | NM_006164.2 | 1262 | tgcccctggaagtgtcaaacaga | 7755 | 462766 | see also 4266 line | | |
| 4268 | NFRKB | NM_006165.2 | 1773 | agcaggagcgttacaggtatagc | 334941 | 778586 | transcription_regulator | - | - |
| 4269 | NFYB | NM_006166.2 | 206 | gaggacagcatgaatgatcatga | 8120 | 462862 | transcription_regulator | NF-YA/NF-YB/NF-YC trimeric complex | - |
| 4270 | NPY5R | NM_006174.2 | 310 | ttctgattgggctctatacattt | 176088 | 571293 | gpcr、receptor_acitivity、signal_transduction | neuropeptide Y receptor | - |
| 4271 | NSF | NM_006178.1 | 913 | gtcaatgggccagaaatccttaa | 95027 | 510580 | - | serine-type endopeptidase | schizophrenia |
| 4272 | NSF | NM_006178.1 | 2169 | ctggataggaatcaagaagttac | 95062 | 510606 | see also 4271 line | | |
| 4273 | NSF | NM_006178.1 | 2174 | taggaatcaagaagttactaatg | 95063 | 510607 | see also 4271 line | | |
| 4274 | NTRK2 | NM_006180.2 | 1380 | cactggtgcattccattcactgt | 95100 | 510634 | kinase_related、receptor_acitivity、signal_transduction | neurotrophin TRKB receptor | neuroblastoma |
| 4275 | NUMA1 | NM_006185.1 | 5696 | agccagacagcgccaactcatcg | 292106 | 694226 | - | - | - |
| 4276 | NR4A2 | NM_006186.2 | 586 | ccggacagcagtcctccattaag | 8626 | 447293 | transcription_regulator、receptor_acitivity、signal_transduction | - | rheumatoid arthritis、alcoholism、Parkinson disease |
| 4277 | ORC2L | NM_006190.3 | 1589 | ctggtatgaaactactacataca | 20553 | 670133 | transcription_regulator | DNA replication origin binding | - |
| 4278 | ORC2L | NM_006190.3 | 1604 | tacatacagtccttatactgaag | 20554 | 670134 | see also 4277 line | | |
| 4279 | ORC2L | NM_006190.3 | 1855 | gggcccagttaactgaatttagg | 20562 | 670143 | see also 4277 line | | |
| 4280 | PAX1 | NM_006192.1 | 602 | tcctgggcatccggacgtttatg | 34505 | 463364 | transcription_regulator | translation regulator | - |

Figure 46

| | | | | | | | | | |
|---|---|---|---|---|---|---|---|---|---|
| 4281 | PBX3 | NM_006195.2 | 432 | agctaatgagactggacaatatg | 8977 | 447622 | transcription_regulator | translation regulator | lymphoblastic leukemia |
| 4282 | PBX3 | NM_006195.2 | 439 | gagactggacaatatgctttgg | 8978 | 447623 | see also 4281 line | | |
| 4283 | PBX3 | NM_006195.2 | 775 | ttgtgaagcagttatgattttaa | 8985 | 447627 | see also 4281 line | | |
| 4284 | PBX3 | NM_006195.2 | 819 | gacggaaaaggcgtaacttcagt | 8987 | 447629 | see also 4281 line | | |
| 4285 | PBX3 | NM_006195.2 | 1240 | taccctccgtcatgttatcaatc | 8994 | 447636 | see also 4281 line | | |
| 4286 | PBX3 | NM_006195.2 | 1241 | accctccgtcatgttatcaatca | 8995 | 447637 | see also 4281 line | | |
| 4287 | PBX3 | NM_006195.2 | 1245 | tccgtcatgttatcaatcagacg | 8996 | 447638 | see also 4281 line | | |
| 4288 | PCBP1 | NM_006196.2 | 941 | ccggattcgccggaattgactcc | 19643 | 703420 | - | single-stranded DNA binding | - |
| 4289 | PCBP1 | NM_006196.2 | 1008 | tactcaaaccacccatgaactca | 19644 | 703422 | see also 4288 line | | |
| 4290 | PCM1 | NM_006197.2 | 719 | ttgcctatgcagattaatactaa | 304082 | 713989 | transcription_regulator | - | - |
| 4291 | PCM1 | NM_006197.2 | 721 | gcctatgcagattaatactaaca | 304083 | 713991 | see also 4290 line | | |
| 4292 | PCM1 | NM_006197.2 | 3244 | ttgggtgtcagagctctcttacg | 304170 | 714052 | see also 4290 line | | |
| 4293 | PCM1 | NM_006197.2 | 6020 | cccttagtgcctagagtcaaaga | 304261 | 714146 | see also 4290 line | | |
| 4294 | PCM1 | NM_006197.2 | 6094 | tactgaatctccagtgttagtga | 304263 | 714149 | see also 4290 line | | |
| 4295 | PCM1 | NM_006197.2 | 6096 | ctgaatctccagtgttagtgaat | 304264 | 714150 | see also 4290 line | | |
| 4296 | PCM1 | NM_006197.2 | 6100 | atctccagtgttagtgaatgact | 304265 | 714151 | see also 4290 line | | |
| 4297 | PCTK1 | NM_006201.2 | 1743 | gggggagcggatccacaaacttc | 95956 | 511400 | kinase_related、cell_cycle | - | - |
| 4298 | PCTK1 | NM_006201.2 | 1744 | ggggagcggatccacaaacttcc | 95957 | 511401 | see also 4297 line | | |
| 4299 | PDE4D | NM_006203.2 | 1214 | tgccagtgcaatacatgatgtag | 197891 | 590193 | signal_transduction | cAMP-specific phosphodiesterase | atherosclerosis |
| 4300 | PDE4D | NM_006203.2 | 1393 | aacaaagacaatctttaaggaaa | 197898 | 590198 | see also 4299 line | | |
| 4301 | PDE4D | NM_006203.2 | 1622 | gtggacggaccggataatggagg | 197907 | 590201 | see also 4299 line | | |
| 4302 | PDE6C | NM_006204.2 | 868 | atgtggtcagccaataaagtatt | 197727 | 590324 | signal_transduction | cGMP-specific phosphodiesterase | - |
| 4303 | ENPP1 | NM_006208.1 | 1412 | tactattcatttaactatgaagg | 60603 | 484175 | phosphatase | nucleotide pyrophosphatase | diabetes mellitus、diabetes |
| 4304 | ENPP2 | NM_006209.2 | 1482 | aacagcaccttcccaaacgtttg | 60695 | 484274 | transcription_regulator、phosphatase、signal_transduction、gpcr | - | - |
| 4305 | ENPP2 | NM_006209.2 | 1697 | aagactaaagtgcctccatttga | 60700 | 484278 | see also 4304 line | | |
| 4306 | PIK3CA | NM_006218.1 | 323 | cagtaggcaaccgtgaagaaaag | 207268 | 597220 | kinase_related、signal_transduction | phosphatidylinositol 3-kinase、class III | ovarian cancer、head and neck squamous cell carcinoma |
| 4307 | PIK3CA | NM_006218.1 | 327 | aggcaaccgtgaagaaaagatcc | 207269 | 597221 | see also 4306 line | | |
| 4308 | PIK3CA | NM_006218.1 | 1106 | aacccttatgtgacaatgtgaac | 207281 | 597243 | see also 4306 line | | |

Figure 46

| 4309 | PIK3CA | NM_006218.1 | 1117 | gacaatgtgaacactcaaagagt | 207283 | 597245 | see also 4306 line | | |
|---|---|---|---|---|---|---|---|---|---|
| 4310 | PIK3CA | NM_006218.1 | 1159 | tggaatgaatggctgaattatga | 207284 | 597247 | see also 4306 line | | |
| 4311 | PIK3CA | NM_006218.1 | 1425 | atgcttagagttggagtttgact | 207288 | 597259 | see also 4306 line | | |
| 4312 | PIK3CA | NM_006218.1 | 3040 | atgccagaactacaatctttttga | 207343 | 597310 | see also 4306 line | | |
| 4313 | PIK3CB | NM_006219.1 | 143 | gggaagctaccatttcttatatt | 207349 | 597323 | kinase_related、signal_transduction、cell_cycle、gpcr | phosphatidylinositol 3-kinase、class III | - |
| 4314 | PIK3CB | NM_006219.1 | 146 | aagctaccatttcttatattaag | 207350 | 597324 | see also 4313 line | | |
| 4315 | PIK3CB | NM_006219.1 | 776 | gtgatcatccactaattcagttc | 207372 | 597346 | see also 4313 line | | |
| 4316 | PIK3CB | NM_006219.1 | 3153 | aagctggactactaaagtgaact | 207444 | 597415 | see also 4313 line | | |
| 4317 | PITPN | NM_006224.2 | 268 | gtgtctgtagatgagtatcaagt | 173624 | 570292 | - | phosphatidylinositol transporter | - |
| 4318 | PITPN | NM_006224.2 | 704 | aagacccagcaaaatttaaatct | 173635 | 570299 | see also 4317 line | | |
| 4319 | PNOC | NM_006228.2 | 647 | cagaagcggttcagtgagtttat | 169649 | 567391 | signal_transduction | opioid peptide | - |
| 4320 | POLE | NM_006231.1 | 1271 | gtgggtgaagagggacagttacc | 35822 | 464061 | - | zeta DNA polymerase | - |
| 4321 | POLE | NM_006231.1 | 1297 | ctgtgggcagtcataatctcaag | 35823 | 464062 | see also 4320 line | | |
| 4322 | POLE | NM_006231.1 | 3161 | gtctcggaagctggaagattacg | 35845 | 464109 | see also 4320 line | | |
| 4323 | POLE | NM_006231.1 | 4486 | cagggagtatccgccatatctac | 35854 | 464135 | see also 4320 line | | |
| 4324 | POLE | NM_006231.1 | 4487 | agggagtatccgccatatctacc | 35855 | 464136 | see also 4320 line | | |
| 4325 | PPEF2 | NM_006239.2 | 1882 | gcctccaactactatgaagttgg | 151465 | 552635 | phosphatase | - | - |
| 4326 | PPP2R5B | NM_006244.2 | 837 | tggggtgatgtttgacttcttgg | 298774 | 666329 | phosphatase、signal_transduction | protein phosphatase type 2A、regulator | - |
| 4327 | PPP2R5D | NM_006245.2 | 864 | accattttgcatcgcatctatgg | 298811 | 666389 | phosphatase、signal_transduction | - | - |
| 4328 | PPP2R5D | NM_006245.2 | 874 | atcgcatctatggcaagttttg | 298812 | 666390 | see also 4327 line | | |
| 4329 | PPP2R5E | NM_006246.2 | 622 | tggataaagtagacggattttct | 298822 | 666404 | phosphatase、signal_transduction | protein phosphatase type 2A、regulator | - |
| 4330 | PPP2R5E | NM_006246.2 | 641 | ttctcggaagtccgtcagaaaag | 298823 | 666405 | see also 4329 line | | |
| 4331 | PPP2R5E | NM_006246.2 | 816 | ttcatggacacgctatctgatct | 298832 | 666406 | see also 4329 line | | |
| 4332 | PPP2R5E | NM_006246.2 | 819 | atggacacgctatctgatcttaa | 298833 | 666407 | see also 4329 line | | |
| 4333 | PPP2R5E | NM_006246.2 | 820 | tggacacgctatctgatcttaaa | 298834 | 666408 | see also 4329 line | | |
| 4334 | PPP2R5E | NM_006246.2 | 855 | aagcgctccactcttaatgaact | 298836 | 666410 | see also 4329 line | | |
| 4335 | PPP2R5E | NM_006246.2 | 857 | gcgctccactcttaatgaactgg | 298837 | 666411 | see also 4329 line | | |
| 4336 | PPP2R5E | NM_006246.2 | 876 | ctggtggactacattacaataag | 298838 | 666412 | see also 4329 line | | |
| 4337 | PRKCH | NM_006255.2 | 1024 | acgctgccatcatctaattgtta | 97165 | 512182 | - | protein kinase C | - |
| 4338 | PRKCH | NM_006255.2 | 1025 | cgctgccatcatctaattgttac | 97166 | 512183 | see also 4337 line | | |

Figure 46

| | | | | | | | | | |
|---|---|---|---|---|---|---|---|---|---|
| 4339 | PRKCH | NM_006255.2 | 1028 | tgccatcatctaattgttacagc | 97167 | 512184 | see also 4337 line | | |
| 4340 | PRKCH | NM_006255.2 | 1198 | gcgacaaggacttcagtgtaaaa | 97170 | 512188 | see also 4337 line | | |
| 4341 | PRKCL2 | NM_006256.1 | 656 | aacttcggatggaagaattaagg | 97289 | 489558 | kinase_related、 signal _transduction | protein serine/threonine kinase | - |
| 4342 | PRKCL2 | NM_006256.1 | 1287 | caggtcacgtgaactggaaattt | 97320 | 489578 | see also 4341 line | | |
| 4343 | PRKCQ | NM_006257.1 | 359 | gccgaatgctaatgaatgcaaga | 97505 | 512385 | kinase_related | protein tyrosine kinase | - |
| 4344 | PRKG1 | NM_006258.1 | 640 | gaccgtcaagactcttgtaaatg | 97576 | 512442 | kinase_related、 signal _transduction | protein tyrosine kinase | - |
| 4345 | PRKG1 | NM_006258.1 | 678 | ttgatcgacaatgttttcaaaca | 97581 | 512447 | see also 4344 line | | |
| 4346 | PRKG1 | NM_006258.1 | 1814 | ttgagggggattgacatgataga | 97612 | 512473 | see also 4344 line | | |
| 4347 | PRKG1 | NM_006258.1 | 1816 | gaggggggattgacatgatagaat | 97613 | 512474 | see also 4344 line | | |
| 4348 | PRKG2 | NM_006259.1 | 1564 | atcgtactttcaaggacaataag | 97648 | 512521 | kinase_related、 signal _transduction、 cell_cy cle | protein kinase CK2 | - |
| 4349 | PRKG2 | NM_006259.1 | 1565 | tcgtactttcaaggacaataagt | 97649 | 512522 | see also 4348 line | | |
| 4350 | PRKG2 | NM_006259.1 | 2134 | aacacaggtggttaaatggtttt | 97665 | 512538 | see also 4348 line | | |
| 4351 | PTPN13 | NM_006264.1 | 1262 | cagttcgaagatacaaaacttat | 193249 | 586652 | phosphatase | - | - |
| 4352 | PTPN13 | NM_006264.1 | 2711 | ttgagagagcttcgtttaggagc | 193294 | 586686 | see also 4351 line | | |
| 4353 | PTPN13 | NM_006264.1 | 2749 | gagtctgttagaggatttaatat | 193296 | 586687 | see also 4351 line | | |
| 4354 | PTPN13 | NM_006264.1 | 2751 | gtctgttagaggatttaatatgg | 193297 | 586688 | see also 4351 line | | |
| 4355 | PTPN13 | NM_006264.1 | 5577 | atccaaaacagtcagattagtta | 193371 | 586756 | see also 4351 line | | |
| 4356 | RAD21 | NM_006265.1 | 409 | tgcagactgtaatgaagcattca | 134896 | 552745 | apoptosis、 cell_cycle | - | - |
| 4357 | RALGDS | NM_006266.1 | 492 | ttcaaaaggtacggtagatgtga | 296460 | 570330 | signal_transduction | guanyl-nucleotide release factor | - |
| 4358 | RALGDS | NM_006266.1 | 517 | ccctcacggcctcctctagatac | 296461 | 570331 | see also 4357 line | | |
| 4359 | RANBP2 | NM_006267.3 | 9670 | ttggactttaagcatgtagtatt | 127752 | 619535 | - | peptidyl-prolyl cis- trans isomerase | - |
| 4360 | RANBP2 | NM_006267.3 | 9671 | tggactttaagcatgtagtattt | 127753 | 619536 | see also 4359 line | | |
| 4361 | RANBP2 | NM_006267.3 | 9683 | atgtagtatttgggtttgttaag | 127754 | 619538 | see also 4359 line | | |
| 4362 | DPF2 | NM_006268.3 | 149 | ccggagtagcccagagcaattgt | 25574 | 458649 | apoptosis、 transcriptio n_regulator | DNA binding | - |
| 4363 | ITSN2 | NM_006277.1 | 863 | cagtctctgattgatttaggatc | 146926 | 553822 | - | - | - |
| 4364 | ITSN2 | NM_006277.1 | 869 | ctgattgatttaggatctagtag | 146927 | 553823 | see also 4363 line | | |
| 4365 | ITSN2 | NM_006277.1 | 1331 | gacaaacggaaagccaactatga | 146934 | 553834 | see also 4363 line | | |
| 4366 | ITSN2 | NM_006277.1 | 2004 | aagaattatgccaaagacttaaa | 146948 | 553850 | see also 4363 line | | |
| 4367 | ITSN2 | NM_006277.1 | 2620 | tggttggctttatggtagttttc | 146965 | 553860 | see also 4363 line | | |

244

Figure 46

| | | | | | | | | | |
|---|---|---|---|---|---|---|---|---|---|
| 4368 | SIAT4C | NM_006278.1 | 215 | tcgtcatggtgtggtattccatc | 212786 | 601133 | - | transferase、transferring glycosyl groups | - |
| 4369 | SIAT4C | NM_006278.1 | 577 | gtggtcatcagattgaacaatgc | 212792 | 601144 | see also 4368 line | | |
| 4370 | SIAT6 | NM_006279.2 | 558 | tgggatcaaaggtcaagacaatc | 212947 | 601086 | - | - | - |
| 4371 | SIAT6 | NM_006279.2 | 567 | aggtcaagacaatctgatcaaag | 212948 | 601087 | see also 4370 line | | |
| 4372 | SIAT6 | NM_006279.2 | 1167 | cgcacccctgcactactatgaga | 212956 | 601096 | see also 4370 line | | |
| 4373 | STK3 | NM_006281.1 | 754 | tcccttggcattacttctataga | 101666 | 515671 | kinase_related、apoptosis、signal_transduction | protein kinase CK2 | - |
| 4374 | STK4 | NM_006282.2 | 603 | aggtcaacttacagataccatgg | 101933 | 515795 | kinase_related、apoptosis、signal_transduction | protein tyrosine kinase | - |
| 4375 | TACC1 | NM_006283.1 | 479 | cagctcggattctgaaggtaatt | 322156 | 792666 | - | - | - |
| 4376 | TFDP2 | NM_006286.1 | 635 | gaggcggatagaacggataaagc | 13235 | 441498 | transcription_regulator、cell_cycle | translation regulator | - |
| 4377 | TFDP2 | NM_006286.1 | 1032 | tggttaaatcagggactacttct | 13240 | 441503 | see also 4376 line | | |
| 4378 | THY1 | NM_006288.2 | 162 | ctggactgccgccatgagaatac | 284743 | 694836 | - | - | diabetes |
| 4379 | TLN1 | NM_006289.2 | 3224 | gtggacctttggagatggattct | 119519 | 524650 | - | structural constituent of cytoskeleton | - |
| 4380 | TLN1 | NM_006289.2 | 3300 | agctcgagatggcaagcttaaac | 119521 | 524653 | see also 4379 line | | |
| 4381 | TLN1 | NM_006289.2 | 4647 | tactgccaagcgccagtttgtac | 119537 | 524666 | see also 4379 line | | |
| 4382 | TLN1 | NM_006289.2 | 4650 | tgccaagcgccagtttgtacagt | 119538 | 524667 | see also 4379 line | | |
| 4383 | TLN1 | NM_006289.2 | 5749 | aaccagctgaccagtgactatgg | 119547 | 524685 | see also 4379 line | | |
| 4384 | TNFAIP3 | NM_006290.2 | 609 | ggcccgaagtggacttcagtaca | 42382 | 469217 | apoptosis、transcription_regulator | Transcription factor p65 | - |
| 4385 | TNFAIP3 | NM_006290.2 | 612 | ccgaagtggacttcagtacaact | 42383 | 469218 | see also 4384 line | | |
| 4386 | TSG101 | NM_006292.2 | 389 | ttgttaagcctactagttcaatg | 43033 | 541751 | transcription_regulator | transcription co-repressor | breast cancer |
| 4387 | TSG101 | NM_006292.2 | 394 | aagcctactagttcaatgactat | 43034 | 541752 | see also 4386 line | | |
| 4388 | TSG101 | NM_006292.2 | 395 | agcctactagttcaatgactatt | 43035 | 541753 | see also 4386 line | | |
| 4389 | TSG101 | NM_006292.2 | 396 | gcctactagttcaatgactatta | 43036 | 541754 | see also 4386 line | | |
| 4390 | VARS2 | NM_006295.1 | 2002 | ccgcacatgaccaaaatgactat | 107306 | 517520 | - | valine-tRNA ligase | - |
| 4391 | VRK2 | NM_006296.2 | 809 | gccataatgggacaatagagttt | 107505 | 517616 | kinase_related | - | - |
| 4392 | MAP3K12 | NM_006301.2 | 1148 | accacgaaatcgccatcattcc | 89078 | 507024 | kinase_related | protein tyrosine kinase | - |
| 4393 | MAP3K12 | NM_006301.2 | 1247 | ggcagagtggcgggaagaagtaa | 89079 | 507028 | see also 4392 line | | |
| 4394 | MAP3K12 | NM_006301.2 | 1253 | gtggcgggaagaagtaaaactgc | 89080 | 507030 | see also 4392 line | | |

Figure 46

| 4395 | JTV1 | NM_006303.2 | 769 | ctctgtttggccagaagcataat | 16318 | 760888 | - | - | - |
|------|------|-------------|-----|-------------------------|-------|--------|---|---|---|
| 4396 | SHFM1 | NM_006304.1 | 204 | gactgggctggcttagatgaaga | 284196 | 667692 | - | molecular_function unknown | split-hand and split-foot |
| 4397 | SMC1L1 | NM_006306.2 | 109 | tcgtacaagggtcgacagattat | 100453 | 514698 | cell_cycle、signal_transduction | - | - |
| 4398 | SMC1L1 | NM_006306.2 | 171 | tggctctggtaagtcaaatctca | 100454 | 514700 | see also 4397 line | | |
| 4399 | SMC1L1 | NM_006306.2 | 1727 | gccgggactgtattcagtatatc | 100468 | 514735 | see also 4397 line | | |
| 4400 | SMC1L1 | NM_006306.2 | 2301 | tgggcctcgcattaatgatatca | 100476 | 514752 | see also 4397 line | | |
| 4401 | SMC1L1 | NM_006306.2 | 2303 | ggcctcgcattaatgatatcaag | 100477 | 514753 | see also 4397 line | | |
| 4402 | SMC1L1 | NM_006306.2 | 2990 | cacgagaggccctcattgagatt | 100489 | 514768 | see also 4397 line | | |
| 4403 | SMC1L1 | NM_006306.2 | 3569 | ttggcaaggtggcaaattacatc | 100500 | 514779 | see also 4397 line | | |
| 4404 | NPEPPS | NM_006310.2 | 1018 | gagaactggggccttgttactta | 158624 | 559312 | - | zinc binding | - |
| 4405 | NPEPPS | NM_006310.2 | 1021 | aactggggccttgttacttatag | 158625 | 559313 | see also 4404 line | | |
| 4406 | NPEPPS | NM_006310.2 | 1959 | cactgtagaggttctaaaagtca | 158644 | 559340 | see also 4404 line | | |
| 4407 | NPEPPS | NM_006310.2 | 1991 | ttgtgaatgagcccaattatact | 158646 | 559341 | see also 4404 line | | |
| 4408 | NPEPPS | NM_006310.2 | 1993 | gtgaatgagcccaattatactgt | 158647 | 559342 | see also 4404 line | | |
| 4409 | NPEPPS | NM_006310.2 | 2235 | agcccgtcgtcggtttaaggacc | 158649 | 559346 | see also 4404 line | | |
| 4410 | NCOR1 | NM_006311.2 | 1246 | agcaaaacaagggaatactatga | 33565 | 462407 | transcription_regulator | transcription co-repressor | - |
| 4411 | NCOR1 | NM_006311.2 | 1440 | ctctgtgattccacctatgatgt | 33567 | 462413 | see also 4410 line | | |
| 4412 | NCOR1 | NM_006311.2 | 5205 | gaccaatatgcctccaacaattt | 33652 | 462496 | see also 4410 line | | |
| 4413 | NCOR1 | NM_006311.2 | 5666 | tgccagcctcccgttacaacact | 33662 | 462502 | see also 4410 line | | |
| 4414 | NCOR1 | NM_006311.2 | 6043 | gtgatcatcacccggcaaattgc | 33667 | 462510 | see also 4410 line | | |
| 4415 | NCOR1 | NM_006311.2 | 6804 | aagctacttgccttcattcttca | 33679 | 462519 | see also 4410 line | | |
| 4416 | USP15 | NM_006313.1 | 56 | tcgcgacgctgctcaaaacctcg | 188921 | 582942 | - | ubiquitin-specific protease | - |
| 4417 | USP15 | NM_006313.1 | 466 | agcaaagctgacacaatagatac | 188938 | 582958 | see also 4416 line | | |
| 4418 | USP15 | NM_006313.1 | 907 | gacaatcccttaggaatgagagg | 188952 | 582973 | see also 4416 line | | |
| 4419 | USP15 | NM_006313.1 | 969 | gtggtctggaaagtttagctacg | 188958 | 582977 | see also 4416 line | | |
| 4420 | RNF3 | NM_006315.3 | 484 | caggagctgcctggtgaagtacc | 335197 | 777911 | - | - | - |
| 4421 | CDIPT | NM_006319.2 | 374 | tcggttatgcccggattgtcttc | 161802 | 606496 | - | - | - |
| 4422 | CDIPT | NM_006319.2 | 375 | cggttatgcccggattgtcttcg | 161803 | 606497 | see also 4421 line | | |
| 4423 | TUBGCP3 | NM_006322.3 | 1074 | gagttgggatggttgcataataa | 309696 | 675978 | - | - | - |
| 4424 | TUBGCP3 | NM_006322.3 | 1506 | gaggacacttaccacgaattttt | 309707 | 675990 | see also 4423 line | | |
| 4425 | TUBGCP3 | NM_006322.3 | 1507 | aggacacttaccacgaatttttt | 309708 | 675991 | see also 4423 line | | |
| 4426 | TUBGCP3 | NM_006322.3 | 1509 | gacacttaccacgaattttttgt | 309709 | 675992 | see also 4423 line | | |
| 4427 | TUBGCP3 | NM_006322.3 | 1511 | cacttaccacgaatttttgtag | 309710 | 675993 | see also 4423 line | | |

Figure 46

| 4428 | TUBGCP3 | NM_006322.3 | 1662 | ttcttgcaccaagtttgtcatga | 309720 | 676001 | see also 4423 line | | |
|------|---------|-------------|------|--------------------------|--------|--------|--------------------|---|---|
| 4429 | TUBGCP3 | NM_006322.3 | 2717 | gtgctcacagttgcgaatattga | 309756 | 676029 | see also 4423 line | | |
| 4430 | RBM14 | NM_006328.1 | 167 | gagctgcgccgtcatgaaacagt | 65329 | 485704 | - | - | - |
| 4431 | RBM14 | NM_006328.1 | 170 | ctgcgccgtcatgaaacagttcg | 65330 | 485705 | see also 4430 line | | |
| 4432 | RBM14 | NM_006328.1 | 1314 | gcccagtctgccccatatgctgc | 65339 | 485720 | see also 4430 line | | |
| 4433 | FBLN5 | NM_006329.2 | 968 | ttgatgaatgtcgctatggttac | 144202 | 548380 | - | integrin binding | - |
| 4434 | FBLN5 | NM_006329.2 | 1768 | gtgatccgactgcggatatatgt | 144221 | 548397 | see also 4433 line | | |
| 4435 | FBLN5 | NM_006329.2 | 1772 | tccgactgcggatatatgtgtcg | 144222 | 548398 | see also 4433 line | | |
| 4436 | BAIAP2 | NM_006340.1 | 118 | atgcaccggctcacggaaaatgt | 120836 | 532492 | signal_transduction | - | - |
| 4437 | MERTK | NM_006343.1 | 1183 | tgcaagccctggctaattacagc | 90947 | 508215 | receptor_acitivity、 kinase_related、 signal_transduction | transmembrane receptor protein tyrosine kinase | rheumatoid arthritis、 retinitis pigmentosa |
| 4438 | MERTK | NM_006343.1 | 2496 | ggcgtgaccatgtgggaaatacg | 90982 | 508248 | see also 4437 line | | |
| 4439 | SLC30A9 | NM_006345.2 | 902 | ccggttcagcaagtatgttctca | 11467 | 654032 | transcription_regulator | - | - |
| 4440 | SLC30A9 | NM_006345.2 | 921 | ctcagaagctatacactcattat | 11468 | 654034 | see also 4439 line | | |
| 4441 | SLC30A9 | NM_006345.2 | 923 | cagaagctatacactcattatct | 11469 | 654035 | see also 4439 line | | |
| 4442 | SLC30A9 | NM_006345.2 | 927 | agctatacactcattatctgata | 11470 | 654036 | see also 4439 line | | |
| 4443 | PIBF1 | NM_006346.1 | 1713 | aagacgactaaagcaaagtgttc | 335284 | 738698 | - | - | - |
| 4444 | TIMM44 | NM_006351.2 | 1017 | ctcaaagactggtgctatgaagc | 102637 | 516461 | transcription_regulator | - | - |
| 4445 | ZNF238 | NM_006352.2 | 1367 | gagagtgatgttggcactaatga | 44617 | 470651 | transcription_regulator | specific RNA polymerase II transcription factor | - |
| 4446 | ZNF238 | NM_006352.2 | 1369 | gagtgatgttggcactaatgact | 44618 | 470652 | see also 4445 line | | |
| 4447 | ZNF238 | NM_006352.2 | 1377 | ttggcactaatgactatgacatg | 44619 | 470653 | see also 4445 line | | |
| 4448 | ZNF238 | NM_006352.2 | 1378 | tggcactaatgactatgacatgg | 44620 | 470654 | see also 4445 line | | |
| 4449 | SLC9A6 | NM_006359.1 | 927 | atcttcagtggatcttttgcaat | 258280 | 642492 | - | solute:hydrogen antiporter | - |
| 4450 | SLC9A6 | NM_006359.1 | 1339 | tacttaatttgggtagaagaagt | 258289 | 642505 | see also 4449 line | | |
| 4451 | NXF1 | NM_006362.3 | 677 | ccgaaggatatctatcatcatca | 53003 | 474517 | - | protein transporter | - |
| 4452 | NXF1 | NM_006362.3 | 1139 | cagcgccattcgcgaacgatttc | 53011 | 474537 | see also 4451 line | | |
| 4453 | NXF1 | NM_006362.3 | 1140 | agcgccattcgcgaacgatttcc | 53012 | 474538 | see also 4451 line | | |
| 4454 | NXF1 | NM_006362.3 | 1149 | cgcgaacgatttcccaagttact | 53013 | 474539 | see also 4451 line | | |
| 4455 | NXF1 | NM_006362.3 | 1691 | tagcaattcagggctatgtattg | 53022 | 474559 | see also 4451 line | | |
| 4456 | NXF1 | NM_006362.3 | 1692 | agcaattcagggctatgtattgt | 53023 | 474560 | see also 4451 line | | |
| 4457 | NXF1 | NM_006362.3 | 1700 | agggctatgtattgtaaatgatg | 53024 | 474561 | see also 4451 line | | |
| 4458 | SEC23B | NM_006363.3 | 670 | ttccgagggaccaaggatttaac | 270052 | 652343 | - | - | - |

Figure 46

| | | | | | | | | | |
|---|---|---|---|---|---|---|---|---|---|
| 4459 | SEC23B | NM_006363.3 | 671 | tccgagggaccaaggatttaact | 270053 | 652344 | see also 4458 line | | |
| 4460 | SEC23B | NM_006363.3 | 1339 | acctctcgggaactgaagattgc | 270071 | 652361 | see also 4458 line | | |
| 4461 | SEC23A | NM_006364.2 | 412 | gaccacttgccgtgcagttttga | 269988 | 652292 | - | protein transporter | - |
| 4462 | SEC23A | NM_006364.2 | 415 | cacttgccgtgcagttttgaatc | 269989 | 652293 | see also 4461 line | | |
| 4463 | SYNCRIP | NM_006372.3 | 672 | agggctagttgcacatagtgatt | 55053 | 471466 | - | - | - |
| 4464 | SYNCRIP | NM_006372.3 | 771 | cagtgatctctctcatgttcaga | 55057 | 471473 | see also 4463 line | | |
| 4465 | COVA1 | NM_006375.2 | 972 | gagtgtaaacagcgtatgctagc | 58753 | 482039 | - | - | - |
| 4466 | COVA1 | NM_006375.2 | 1059 | gtggtccactattcagatcatga | 58757 | 482040 | see also 4465 line | | |
| 4467 | COVA1 | NM_006375.2 | 1366 | aggcatgggaccacttcacaaaa | 58763 | 482047 | see also 4465 line | | |
| 4468 | COVA1 | NM_006375.2 | 1367 | ggcatgggaccacttcacaaaag | 58764 | 482048 | see also 4465 line | | |
| 4469 | UNC13B | NM_006377.2 | 1000 | aggtatggctcctcctgtaatgt | 252372 | 673208 | receptor_acitivity, apoptosis | - | - |
| 4470 | UNC13B | NM_006377.2 | 4170 | gacgggcacccagctgatcttca | 252418 | 673253 | see also 4469 line | | |
| 4471 | UNC13B | NM_006377.2 | 4867 | ggccggaagatccatatggatga | 252426 | 673261 | see also 4469 line | | |
| 4472 | SEMA3C | NM_006379.2 | 1774 | tcctctcatgtacaattccatct | 310770 | 684531 | - | - | rheumatoid arthritis |
| 4473 | APPBP2 | NM_006380.2 | 342 | ctccgctgtcgtggacaactaca | 299541 | 719356 | - | - | Peutz-Jeghers syndrome |
| 4474 | APPBP2 | NM_006380.2 | 344 | ccgctgtcgtggacaactacatc | 299542 | 719357 | see also 4473 line | | |
| 4475 | APPBP2 | NM_006380.2 | 1200 | ctgtcagtctgttgcaatttatc | 299566 | 719385 | see also 4473 line | | |
| 4476 | HTATIP | NM_006388.2 | 924 | tcgtcacccggatgaagaacatt | 18572 | 450735 | transcription_regulator | transcription co-activator | - |
| 4477 | HTATIP | NM_006388.2 | 972 | gcctcaagccgtggtacttctcc | 18573 | 450737 | see also 4476 line | | |
| 4478 | IPO7 | NM_006391.1 | 891 | aagtgggccttacatattttagc | 127431 | 530061 | signal_transduction | - | - |
| 4479 | IPO7 | NM_006391.1 | 997 | ctgttggtgtccagcaagtttta | 127435 | 530065 | see also 4478 line | | |
| 4480 | IPO7 | NM_006391.1 | 1003 | gtgtccagcaagttttattgaag | 127437 | 530066 | see also 4478 line | | |
| 4481 | IPO7 | NM_006391.1 | 1136 | gccccatatacaaggcattatcc | 127439 | 530069 | see also 4478 line | | |
| 4482 | IPO7 | NM_006391.1 | 1225 | acgaatatatacgcatgaagttt | 127444 | 530070 | see also 4478 line | | |
| 4483 | IPO7 | NM_006391.1 | 1925 | tactgctatgggaattctgaata | 127462 | 530091 | see also 4478 line | | |
| 4484 | IPO7 | NM_006391.1 | 3047 | gactcacggtcttaatgaagaac | 127495 | 530113 | see also 4478 line | | |
| 4485 | NOL5A | NM_006392.2 | 854 | ctccagtcgtgtggtgtctttat | 52878 | 676261 | - | RNA binding | - |
| 4486 | NEDD9 | NM_006403.2 | 2458 | gcccaggacattcgcaacaaagt | 307478 | 673793 | signal_transduction, cell_cycle | - | - |
| 4487 | TM9SF1 | NM_006405.3 | 718 | tccattggttgtccatcatcaac | 275224 | 657675 | - | transporter | - |
| 4488 | TM9SF1 | NM_006405.3 | 977 | tggcactggcattattgtcatgg | 275229 | 657684 | see also 4487 line | | |
| 4489 | TM9SF1 | NM_006405.3 | 1384 | cccagccctggtacaagtctact | 275233 | 657694 | see also 4487 line | | |
| 4490 | TM9SF1 | NM_006405.3 | 1453 | ctgtggagctgtactacatcttt | 275234 | 657695 | see also 4487 line | | |
| 4491 | TM9SF1 | NM_006405.3 | 1455 | gtggagctgtactacatctttgc | 275235 | 657696 | see also 4487 line | | |
| 4492 | TM9SF1 | NM_006405.3 | 1506 | ttgtacggcatcctcttctttgt | 275238 | 657697 | see also 4487 line | | |

Figure 46

| 4493 | PRDX4 | NM_006406.1 | 360 | atcgtgggaaatacttggttttc | 223674 | 610790 | - | thioredoxin peroxidase | - |
|------|-------|-------------|-----|-------------------------|--------|--------|---|-----------------------|---|
| 4494 | PRDX4 | NM_006406.1 | 792 | aacctggtagtgaaacaataatc | 223685 | 610807 | see also 4493 line | | |
| 4495 | ARPC1A | NM_006409.2 | 1160 | aagattgtcgcaaattttgcact | 115320 | 686926 | - | actin binding | - |
| 4496 | HTATIP2 | NM_006410.3 | 427 | tcgtgttgaccgagattatgtgc | 111912 | 706792 | transcription_regulator, apoptosis | - | small cell lung cancer |
| 4497 | HTATIP2 | NM_006410.3 | 474 | ctggagggtgcaaacatttcaac | 111914 | 706793 | see also 4496 line | | |
| 4498 | AKAP3 | NM_006422.2 | 2577 | ctgagctcaatgtccctattttg | 253194 | 531848 | kinase_related | protein kinase A anchor protein | - |
| 4499 | DPYSL4 | NM_006426.1 | 1308 | atgctgccaaaatcttcaatttt | 204799 | 595638 | - | - | - |
| 4500 | CCT4 | NM_006430.2 | 882 | aagattgggcttattcagttttg | 75475 | 497363 | cell_cycle | chaperone | - |
| 4501 | CCT4 | NM_006430.2 | 1349 | agctgagcgctccattcatgatg | 75495 | 497380 | see also 4500 line | | |
| 4502 | CCT4 | NM_006430.2 | 1421 | aggtggtgctccagaaatagagt | 75502 | 497382 | see also 4500 line | | |
| 4503 | CCT4 | NM_006430.2 | 1555 | ccggcctgaatccatttctaca | 75512 | 497387 | see also 4500 line | | |
| 4504 | MAB21L2 | NM_006439.3 | 899 | agctggtttaccagctcaataag | 319937 | 703152 | - | - | - |
| 4505 | SMC2L1 | NM_006444.1 | 3524 | ttcaaacctgctccaatttatat | 100718 | 514873 | - | - | - |
| 4506 | SMC2L1 | NM_006444.1 | 3659 | atgttcaacaatgcaaacgttct | 100725 | 514879 | see also 4505 line | | |
| 4507 | PRPF8 | NM_006445.2 | 1805 | gggcatgtatcgatacaaataca | 46589 | 752067 | - | - | - |
| 4508 | PRPF8 | NM_006445.2 | 3309 | tacattgatcgcatccatatttt | 46627 | 752110 | see also 4507 line | | |
| 4509 | PRPF8 | NM_006445.2 | 4134 | cccaacttgtaccgctacataca | 46642 | 752129 | see also 4507 line | | |
| 4510 | PRPF8 | NM_006445.2 | 4160 | atgggagagcgagttcattgatt | 46644 | 752130 | see also 4507 line | | |
| 4511 | PRPF8 | NM_006445.2 | 5198 | aggcagcaagcctctcatacaac | 46669 | 752153 | see also 4507 line | | |
| 4512 | PRPF8 | NM_006445.2 | 6000 | ttggctgactacggcaagaaaaa | 46687 | 752173 | see also 4507 line | | |
| 4513 | PRPF8 | NM_006445.2 | 6001 | tggctgactacggcaagaaaaac | 46688 | 752174 | see also 4507 line | | |
| 4514 | PRPF8 | NM_006445.2 | 6364 | atgtgcttaagaagttcatctgc | 46695 | 752189 | see also 4507 line | | |
| 4515 | USP16 | NM_006447.1 | 267 | atctgccaagactgtaagactga | 189039 | 583040 | cell_cycle | ubiquitin-specific protease | - |
| 4516 | USP16 | NM_006447.1 | 274 | aagactgtaagactgacaataaa | 189040 | 583041 | see also 4515 line | | |
| 4517 | USP16 | NM_006447.1 | 278 | ctgtaagactgacaataaagtga | 189041 | 583042 | see also 4515 line | | |
| 4518 | USP16 | NM_006447.1 | 709 | ccgtgaaaggactcagtaatttg | 189055 | 583055 | see also 4515 line | | |
| 4519 | USP16 | NM_006447.1 | 781 | cagtgcttagagaactactaaaa | 189057 | 583058 | see also 4515 line | | |
| 4520 | USP16 | NM_006447.1 | 788 | tagagaactactaaaagaagtga | 189059 | 583059 | see also 4515 line | | |
| 4521 | USP16 | NM_006447.1 | 2306 | cacccttaaatgtaagaatgttg | 189102 | 583101 | see also 4515 line | | |
| 4522 | USP16 | NM_006447.1 | 2371 | ttgaacacagtggtactatgagg | 189105 | 583105 | see also 4515 line | | |
| 4523 | CDC42EP3 | NM_006449.3 | 1288 | ccctccccggtgctcaaaaatgc | 120849 | 533422 | signal_transduction | - | - |
| 4524 | TBL3 | NM_006453.2 | 407 | gcctgtggaaggcgatacacacg | 253787 | 756016 | gpcr | receptor signaling protein | - |
| 4525 | SC65 | NM_006455.2 | 807 | cacgagctgaccgccaagtatct | 308076 | 790495 | - | - | - |

Figure 46

| | | | | | | | | | |
|---|---|---|---|---|---|---|---|---|---|
| 4526 | SC65 | NM_006455.2 | 811 | agctgaccgccaagtatctcaac | 308077 | 790496 | see also 4525 line | | |
| 4527 | SC65 | NM_006455.2 | 817 | ccgccaagtatctcaactactat | 308079 | 790497 | see also 4525 line | | |
| 4528 | SC65 | NM_006455.2 | 818 | cgccaagtatctcaactactatc | 308080 | 790498 | see also 4525 line | | |
| 4529 | LIM | NM_006457.1 | 1785 | gtgtgttgtgaaagtttggaagg | 125284 | 530668 | - | - | - |
| 4530 | TRIM3 | NM_006458.2 | 2048 | accgttgggtcagcatcttctcc | 126467 | 541579 | - | - | - |
| 4531 | KEO4 | NM_006459.2 | 163 | gaccaggctatcatatcatgttg | 335557 | 756704 | - | - | - |
| 4532 | KEO4 | NM_006459.2 | 276 | atctatattgaccgaatagaagt | 335562 | 756710 | see also 4531 line | | |
| 4533 | KEO4 | NM_006459.2 | 285 | gaccgaatagaagtggttaatat | 335563 | 756711 | see also 4531 line | | |
| 4534 | KEO4 | NM_006459.2 | 311 | ggctccttatgcagtgtttgata | 335565 | 756714 | see also 4531 line | | |
| 4535 | KEO4 | NM_006459.2 | 522 | caggctgtgcgtgttacaaaacc | 335570 | 756723 | see also 4531 line | | |
| 4536 | TGOLN2 | NM_006464.1 | 1340 | ggccagtgactaccaacgtttgg | 323507 | 687960 | - | - | - |
| 4537 | IVNS1ABP | NM_006469.2 | 561 | tcccaatggatatttgatgtttg | 132011 | 536024 | transcription_regulator | - | - |
| 4538 | IVNS1ABP | NM_006469.2 | 954 | ctgcatctcttaccgaaattttg | 132022 | 536047 | see also 4537 line | | |
| 4539 | IVNS1ABP | NM_006469.2 | 958 | atctcttaccgaaattttgcaag | 132023 | 536049 | see also 4537 line | | |
| 4540 | IVNS1ABP | NM_006469.2 | 986 | tgggagactcccgtttgttgaat | 132024 | 536052 | see also 4537 line | | |
| 4541 | IVNS1ABP | NM_006469.2 | 987 | gggagactcccgtttgttgaata | 132025 | 536053 | see also 4537 line | | |
| 4542 | IVNS1ABP | NM_006469.2 | 989 | gagactcccgtttgttgaataag | 132026 | 536054 | see also 4537 line | | |
| 4543 | IVNS1ABP | NM_006469.2 | 1087 | ttggaggtaatgcttgaagataa | 132031 | 536057 | see also 4537 line | | |
| 4544 | IVNS1ABP | NM_006469.2 | 1646 | cagagatgaatggcaaactcata | 132039 | 536067 | see also 4537 line | | |
| 4545 | OSF-2 | NM_006475.1 | 856 | ttccacgaggtgtcctagaaagg | 292196 | 683120 | - | - | - |
| 4546 | GAS2L1 | NM_006478.3 | 859 | ctgaccagtttcccatgatcaag | 306279 | 674185 | cell_cycle | - | - |
| 4547 | ERF | NM_006494.1 | 1073 | cacccaaagcgtctacaactacc | 3387 | 443466 | transcription_regulator, cell_cycle | transcription co-repressor | - |
| 4548 | POLH | NM_006502.1 | 305 | agcggcaaaatcctcatttgagg | 17057 | 598345 | - | - | xeroderma pigmentosum |
| 4549 | POLH | NM_006502.1 | 442 | aagaagttatgtccagatcttct | 17060 | 598352 | see also 4548 line | | |
| 4550 | RASA2 | NM_006506.2 | 538 | tgccatgggttgcctctcataaa | 297142 | 665773 | - | RAS GTPase activator | - |
| 4551 | RASA2 | NM_006506.2 | 868 | ttgctacagccaagagacaatgg | 297156 | 665784 | see also 4550 line | | |
| 4552 | RELB | NM_006509.2 | 1778 | gccttgtgggcagcaacatgttc | 10450 | 448116 | transcription_regulator | translation regulator | - |
| 4553 | SARS | NM_006513.2 | 94 | ttgtttcgggtggataaaggagg | 54573 | 514398 | - | serine-tRNA ligase | - |
| 4554 | SARS | NM_006513.2 | 218 | ttcgggcagacaacttgaacaag | 54578 | 514400 | see also 4553 line | | |
| 4555 | SARS | NM_006513.2 | 219 | tcgggcagacaacttgaacaagc | 54579 | 514401 | see also 4553 line | | |
| 4556 | SCN10A | NM_006514.1 | 984 | ttctgacaacccggattttaact | 152470 | 553539 | channel | - | - |
| 4557 | SCN10A | NM_006514.1 | 986 | ctgacaacccggattttaactac | 152471 | 553540 | see also 4556 line | | |
| 4558 | SETMAR | NM_006515.1 | 683 | tcctgtccgaattgactcaatgg | 335720 | 801375 | - | - | - |

Figure 46

| | | | | | | | | | |
|---|---|---|---|---|---|---|---|---|---|
| 4559 | SETMAR | NM_006515.1 | 687 | gtccgaattgactcaatggtacc | 335721 | 801376 | see also 4558 line | | renal cell carcinoma, alveolar soft part sarcoma, renal tumour, synovial sarcoma, soft tissue sarcoma |
| 4560 | TFE3 | NM_006521.3 | 1305 | gtcgcaggcgattcaacattaac | 13259 | 468944 | transcription_regulator | translation regulator | |
| 4561 | TFE3 | NM_006521.3 | 1306 | tcgcaggcgattcaacattaacg | 13260 | 468945 | see also 4560 line | | |
| 4562 | TFE3 | NM_006521.3 | 1309 | caggcgattcaacattaacgaca | 13261 | 468946 | see also 4560 line | | |
| 4563 | GLRA3 | NM_006529.1 | 1510 | gagaagtttaccgtttctcaga | 179335 | 572608 | channel, receptor_acit ivity | GABA-A receptor | - |
| 4564 | GAS41 | NM_006530.2 | 827 | agcaagtcgtgaaactataaatt | 4393 | 725927 | transcription_regulator | - | glioma, CNS Tumours |
| 4565 | TG737 | NM_006531.2 | 394 | ctgatatgggtccaagacatct | 314640 | 684824 | - | - | |
| 4566 | TG737 | NM_006531.2 | 1678 | ttgcaaatggtgattatgagaag | 314669 | 684852 | see also 4565 line | | |
| 4567 | NCOA3 | NM_006534.2 | 855 | tgcgccagagatatgaaaacaatg | 112150 | 602551 | transcription_regulator, signal_transduction | - | breast cancer, liposarcoma |
| 4568 | NCOA3 | NM_006534.2 | 1032 | aggttgtcaatatagatacaaat | 112158 | 602555 | see also 4567 line | | |
| 4569 | CLCA2 | NM_006536.3 | 1468 | gaggaattatcacgtcttacagg | 263088 | 646668 | channel | channel | |
| 4570 | USP3 | NM_006537.1 | 256 | ggccatgcaaaaaacattatga | 189441 | 583511 | | ubiquitin-specific protease | - |
| 4571 | USP3 | NM_006537.1 | 785 | aaggcagccagactgcatttagc | 189451 | 583527 | see also 4570 line | | |
| 4572 | CACNG3 | NM_006539.2 | 1217 | gaggtatccagatgttgatcacc | 261570 | 645235 | channel | voltage-gated calcium channel | - |
| 4573 | CACNG3 | NM_006539.2 | 1310 | gaggtgtgcaggactaaatct | 261576 | 645241 | see also 4572 line | | |
| 4574 | CACNG3 | NM_006539.2 | 1313 | gtgtgtgcaggactaaatctaca | 261577 | 645242 | see also 4572 line | | |
| 4575 | CACNG3 | NM_006539.2 | 1315 | gtgtgcaggactaaatctacaag | 261578 | 645243 | see also 4572 line | | |
| 4576 | CACNG3 | NM_006539.2 | 1318 | tgcaggactaaatctacaagtga | 261579 | 645244 | see also 4572 line | | |
| 4577 | CACNG3 | NM_006539.2 | 1417 | ttccgagccgtgtgcaagaaaat | 261582 | 645245 | see also 4572 line | | |
| 4578 | CACNG3 | NM_006539.2 | 1418 | tccgagccgtgtgcaagaaaatc | 261583 | 645246 | see also 4572 line | | |
| 4579 | CACNG3 | NM_006539.2 | 1422 | agccgtgtgcaagaaaaatcgatc | 261584 | 645248 | see also 4572 line | | |
| 4580 | CACNG3 | NM_006539.2 | 1423 | ggccgtgtgcaagaaaaatcgatca | 261585 | 645249 | see also 4572 line | | |
| 4581 | NCOA2 | NM_006540.1 | 1530 | cactcctcagggtagtaactatg | 112071 | 525635 | transcription_regulator, signal_transduction | translation regulator | - |
| 4582 | NCOA2 | NM_006540.1 | 1542 | tagtaactatgcactcaaaatga | 112072 | 525636 | see also 4581 line | | |
| 4583 | NCOA2 | NM_006540.1 | 2480 | cagatcctgccagtaacacaaaa | 112085 | 525656 | see also 4581 line | | |
| 4584 | NCOA2 | NM_006540.1 | 2665 | gacaagcaagccatcatcaatga | 112087 | 525662 | see also 4581 line | | |
| 4585 | NCOA2 | NM_006540.1 | 2867 | tcccaccaatcagaaacagtagt | 112091 | 525663 | see also 4581 line | | |
| 4586 | NCOA2 | NM_006540.1 | 3609 | tcccatgcaagatccaaacttc | 112109 | 525670 | see also 4581 line | | |

Figure 46

| 4587 | TXNL2 | NM_006541.1 | 784 | atgtggattcagcaaacaaattc | 265935 | 649959 | - | - | - |
|---|---|---|---|---|---|---|---|---|---|
| 4588 | IMP-3 | NM_006547.2 | 426 | atcgaggcgctttcaggtaaaat | 52210 | 484719 | - | - | - |
| 4589 | IMP-3 | NM_006547.2 | 936 | cagacccagtctaaaatcgatgt | 52229 | 484737 | see also 4588 line | | |
| 4590 | IMP-2 | NM_006548.3 | 238 | ctcgggtaaagtggaattgcatg | 335749 | 808102 | - | - | - |
| 4591 | IMP-2 | NM_006548.3 | 624 | ctcaggccagacagattgatttc | 335757 | 808108 | see also 4590 line | | |
| 4592 | MTX2 | NM_006554.1 | 465 | gtccaatagtccaatttgttaaa | 269454 | 651915 | - | protein transporter | - |
| 4593 | MTX2 | NM_006554.1 | 466 | tccaatagtccaatttgttaaag | 269455 | 651916 | see also 4592 line | | |
| 4594 | MTX2 | NM_006554.1 | 868 | caccattcttaccacacaattga | 269468 | 651926 | see also 4592 line | | |
| 4595 | KHDRBS3 | NM_006558.1 | 1413 | accagccatatggcagatactga | 52274 | 484854 | - | - | - |
| 4596 | CUGBP1 | NM_006560.2 | 185 | accagatcttgatgctatcaaga | 50307 | 471169 | - | RNA binding | - |
| 4597 | CUGBP1 | NM_006560.2 | 542 | ttcgtttggacagattgaagaat | 50314 | 471181 | see also 4596 line | | |
| 4598 | CUGBP1 | NM_006560.2 | 1340 | gggagccaacctgttcatctacc | 50330 | 471193 | see also 4596 line | | |
| 4599 | CUGBP1 | NM_006560.2 | 1430 | tgccaaggttttcatagacaagc | 50333 | 471199 | see also 4596 line | | |
| 4600 | CUGBP2 | NM_006561.1 | 267 | aaggttgttgtttcgtaacattt | 50340 | 471212 | - | - | - |
| 4601 | CUGBP2 | NM_006561.1 | 1303 | ggcaaacctctttatttaccacc | 50356 | 471230 | see also 4600 line | | |
| 4602 | CUGBP2 | NM_006561.1 | 1389 | ctgctaaagtcttcattgacaaa | 50358 | 471233 | see also 4600 line | | |
| 4603 | CTCF | NM_006565.1 | 1624 | gtcatagcccgaaaaagtgattt | 2611 | 457516 | transcription_regulator、cell_cycle | transcription co-repressor | - |
| 4604 | CTCF | NM_006565.1 | 1658 | tgcgaaagcagcattcctatatt | 2612 | 457518 | see also 4603 line | | |
| 4605 | CTCF | NM_006565.1 | 2324 | caggtgcaattgagaacattata | 2619 | 457530 | see also 4603 line | | |
| 4606 | GNA13 | NM_006572.3 | 321 | cagcaacgtgatcaaaggtatga | 109070 | 519407 | signal_transduction、gpcr | receptor signaling protein | - |
| 4607 | GNA13 | NM_006572.3 | 702 | tcctttcaaaatggttgatgtag | 109085 | 519416 | see also 4606 line | | |
| 4608 | MAP4K5 | NM_006575.3 | 1266 | atcattcgtcataccattagatc | 89767 | 715296 | kinase_related | - | - |
| 4609 | B3GNT1 | NM_006577.3 | 1286 | aggcttcaggacatttgatatcg | 209898 | 599279 | - | - | - |
| 4610 | FUT9 | NM_006581.2 | 557 | atcctgccaagcaatgttcaaca | 210854 | 599701 | - | - | - |
| 4611 | FUT9 | NM_006581.2 | 791 | ccgccgtgattcagatatccaag | 210864 | 599712 | see also 4610 line | | |
| 4612 | FUT9 | NM_006581.2 | 792 | cgccgtgattcagatatccaagt | 210865 | 599713 | see also 4610 line | | |
| 4613 | RRH | NM_006583.1 | 803 | ggcatggtccccttattccatcg | 241214 | 629141 | gpcr、receptor_acitivity、signal_transduction | rhodopsin-like receptor | |
| 4614 | CRN | NM_006587.2 | 630 | tcgcctcagttgctatcaacata | 181759 | 575626 | receptor_acitivity | trypsin | - |
| 4615 | CRN | NM_006587.2 | 631 | cgcctcagttgctatcaacatat | 181760 | 575627 | see also 4614 line | | |
| 4616 | CRN | NM_006587.2 | 2472 | ccctgctgcccgaatgaacaaaa | 181803 | 575664 | see also 4614 line | | |
| 4617 | POLD3 | NM_006591.1 | 159 | tacactaggggttcatgttaacc | 35771 | 598322 | - | - | - |
| 4618 | TBR1 | NM_006593.2 | 477 | atgacgaatcagtcagatacaga | 12682 | 449041 | transcription_regulator | transcription factor | - |

Figure 46

| | | | | | | | | | |
|---|---|---|---|---|---|---|---|---|---|
| 4619 | TBR1 | NM_006593.2 | 484 | atcagtcagatacagacaatttt | 12684 | 449042 | see also 4618 line | | |
| 4620 | TBR1 | NM_006593.2 | 572 | ctctgagcttcgtcacagtttcg | 12687 | 449044 | see also 4618 line | | |
| 4621 | TBR1 | NM_006593.2 | 576 | gagcttcgtcacagtttcgatgg | 12688 | 449045 | see also 4618 line | | |
| 4622 | TBR1 | NM_006593.2 | 1416 | taccagaacacggatattacaca | 12697 | 449059 | see also 4618 line | | |
| 4623 | TBR1 | NM_006593.2 | 2177 | ctccagcgactcggggatttacg | 12699 | 449066 | see also 4618 line | | |
| 4624 | TBR1 | NM_006593.2 | 2180 | cagcgactcggggatttacgagc | 12700 | 449067 | see also 4618 line | | |
| 4625 | AP4B1 | NM_006594.1 | 280 | ggctgcgctaccggaatgtcatc | 273167 | 708464 | - | - | - |
| 4626 | AP4B1 | NM_006594.1 | 705 | ctgcgtgaccaggatccaattgt | 273183 | 708465 | see also 4625 line | | |
| 4627 | POLQ | NM_006596.3 | 1992 | cagcatgatacgagctattctgg | 17162 | 464345 | - | - | - |
| 4628 | POLQ | NM_006596.3 | 2542 | atggccatccataaaaggttttt | 17181 | 464364 | see also 4627 line | | |
| 4629 | POLQ | NM_006596.3 | 2543 | tggccatccataaaaggtttttc | 17182 | 464365 | see also 4627 line | | |
| 4630 | POLQ | NM_006596.3 | 2544 | ggccatccataaaaggtttttca | 17183 | 464366 | see also 4627 line | | |
| 4631 | SLC12A7 | NM_006598.1 | 1822 | ctggcgtccacgcttcaagttct | 256830 | 640838 | - | - | - |
| 4632 | SLC12A7 | NM_006598.1 | 1824 | ggcgtccacgcttcaagttctac | 256831 | 640839 | see also 4631 line | | |
| 4633 | SLC12A7 | NM_006598.1 | 1825 | gcgtccacgcttcaagttctacc | 256832 | 640840 | see also 4631 line | | |
| 4634 | NFAT5 | NM_006599.2 | 1500 | accgacagcaaggctatgcaagt | 7393 | 446445 | transcription_regulator, signal_transduction | - | - |
| 4635 | NFAT5 | NM_006599.2 | 1537 | cagccgtgggggtaagtaacaga | 7395 | 446446 | see also 4634 line | | |
| 4636 | NFAT5 | NM_006599.2 | 1539 | gccgtgggggtaagtaacagagg | 7396 | 446447 | see also 4634 line | | |
| 4637 | NFAT5 | NM_006599.2 | 1738 | aggattccccctccaacttcagt | 7403 | 446454 | see also 4634 line | | |
| 4638 | NFAT5 | NM_006599.2 | 1766 | gagcaccagttcctacaatgata | 7407 | 446455 | see also 4634 line | | |
| 4639 | NFAT5 | NM_006599.2 | 1769 | caccagttcctacaatgataaca | 7408 | 446456 | see also 4634 line | | |
| 4640 | NFAT5 | NM_006599.2 | 1788 | aacactgaggtacctcgtaaatc | 7409 | 446457 | see also 4634 line | | |
| 4641 | NFAT5 | NM_006599.2 | 1801 | ctcgtaaatcacgaaaacgaaat | 7410 | 446458 | see also 4634 line | | |
| 4642 | NFAT5 | NM_006599.2 | 1839 | ggggtcaaacgacgagattgtga | 7413 | 446459 | see also 4634 line | | |
| 4643 | NFAT5 | NM_006599.2 | 1842 | gtcaaacgacgagattgtgaaga | 7414 | 446460 | see also 4634 line | | |
| 4644 | NFAT5 | NM_006599.2 | 1923 | cagcttaccacggacaacaaagg | 7416 | 446463 | see also 4634 line | | |
| 4645 | NFAT5 | NM_006599.2 | 2430 | agcactcgtgccagattggtttt | 7436 | 446481 | see also 4634 line | | |
| 4646 | NFAT5 | NM_006599.2 | 2432 | cactcgtgccagattggtttttc | 7437 | 446482 | see also 4634 line | | |
| 4647 | NFAT5 | NM_006599.2 | 2441 | cagattggttttcgagttaata | 7438 | 446483 | see also 4634 line | | |
| 4648 | NFAT5 | NM_006599.2 | 2446 | tggttttcgagttaatatcatg | 7439 | 446484 | see also 4634 line | | |
| 4649 | NFAT5 | NM_006599.2 | 2740 | atcatgaccaacatataactttg | 7448 | 446489 | see also 4634 line | | |
| 4650 | NFAT5 | NM_006599.2 | 5458 | cacaggctactttatttcacaac | 7513 | 446547 | see also 4634 line | | |
| 4651 | NUDC | NM_006600.2 | 216 | agcttccttcgacgcaaaacaga | 307525 | 701878 | cell_cycle | - | - |
| 4652 | TCFL5 | NM_006602.2 | 728 | gggcgctcaacaatttggtaact | 41880 | 541096 | transcription_regulator | - | - |

Figure 46

| 4653 | TCFL5 | NM_006602.2 | 1331 | ggcgcagaatccgcatttgctgt | 41892 | 541112 | see also 4652 line | | |
| 4654 | STAG2 | NM_006603.3 | 633 | cagaactctgagataattcgaaa | 335844 | 673927 | - | - | - |
| 4655 | STAG2 | NM_006603.3 | 1341 | gtgtctatgacccttgacaaaga | 335862 | 673954 | see also 4654 line | | |
| 4656 | STAG2 | NM_006603.3 | 1369 | atgttgcagtacaagcaataaaa | 335863 | 673955 | see also 4654 line | | |
| 4657 | STAG2 | NM_006603.3 | 1452 | gtctatcatctggtttattcagc | 335864 | 673958 | see also 4654 line | | |
| 4658 | STAG2 | NM_006603.3 | 2015 | taccactggacgattagaaaagc | 335880 | 673975 | see also 4654 line | | |
| 4659 | STAG2 | NM_006603.3 | 2100 | ttggaagcatgttctaaaactta | 335885 | 673979 | see also 4654 line | | |
| 4660 | STAG2 | NM_006603.3 | 2101 | tggaagcatgttctaaaacttac | 335886 | 673980 | see also 4654 line | | |
| 4661 | STAG2 | NM_006603.3 | 2342 | tgcttgtaattacaaactcttga | 335897 | 673993 | see also 4654 line | | |
| 4662 | STAG2 | NM_006603.3 | 2541 | ctgaccaacgtgaatactactgt | 335904 | 673999 | see also 4654 line | | |
| 4663 | STAG2 | NM_006603.3 | 2544 | accaacgtgaatactactgttaa | 335905 | 674000 | see also 4654 line | | |
| 4664 | STAG2 | NM_006603.3 | 2627 | aggagggcgtgacatgttagagc | 335907 | 674005 | see also 4654 line | | |
| 4665 | STAG2 | NM_006603.3 | 3336 | ctgatgtcttaccgaaattcttt | 335925 | 674026 | see also 4654 line | | |
| 4666 | STAG2 | NM_006603.3 | 3865 | cagcttcaattatggatgaatca | 335940 | 674036 | see also 4654 line | | |
| 4667 | MAP3K2 | NM_006609.2 | 470 | aagtacacaggctactaatttag | 89197 | 507089 | kinase_related | protein serine/threonine kinase | - |
| 4668 | MAP3K2 | NM_006609.2 | 631 | ttgcccggaatgggtcattcact | 89204 | 507099 | see also 4667 line | | |
| 4669 | MAP3K2 | NM_006609.2 | 633 | gcccggaatgggtcattcactag | 89205 | 507100 | see also 4667 line | | |
| 4670 | MAP3K2 | NM_006609.2 | 634 | cccggaatgggtcattcactagt | 89206 | 507101 | see also 4667 line | | |
| 4671 | MAP3K2 | NM_006609.2 | 1287 | gagaccagcaaggaagtaaatgc | 89231 | 507117 | see also 4667 line | | |
| 4672 | MAP3K2 | NM_006609.2 | 1348 | atgagcgaattgttcagtattat | 89233 | 507120 | see also 4667 line | | |
| 4673 | MAP3K2 | NM_006609.2 | 1424 | gccaggggttcaattaaggacc | 89237 | 507121 | see also 4667 line | | |
| 4674 | MAP3K2 | NM_006609.2 | 1881 | ttcctcaaacggatttttgtaga | 89244 | 507135 | see also 4667 line | | |
| 4675 | MAP3K2 | NM_006609.2 | 1937 | aaggcacatgtttgtgcattatc | 89245 | 507136 | see also 4667 line | | |
| 4676 | CHL1 | NM_006614.2 | 1386 | gagaacctcaacccacaatcaag | 317600 | 677689 | signal_transduction | - | - |
| 4677 | CHL1 | NM_006614.2 | 2487 | ctcaacccaaggaaatgattata | 317643 | 677725 | see also 4676 line | | |
| 4678 | CHL1 | NM_006614.2 | 3086 | aaggtcatcaaagttgataaaga | 317662 | 677748 | see also 4676 line | | |
| 4679 | PLU-1 | NM_006618.2 | 1294 | cgcggacccttcgctttcatcc | 9468 | 767571 | transcription_regulator | - | - |
| 4680 | HBS1L | NM_006620.2 | 666 | ttgtgccaaaagttgctaaaatg | 57613 | 477638 | signal_transduction | - | - |
| 4681 | HBS1L | NM_006620.2 | 983 | gtggtcattggtcatgttgatgc | 57620 | 477645 | see also 4680 line | | |
| 4682 | AHCYL1 | NM_006621.3 | 1244 | ctgccgagaatccattttggatg | 204262 | 594641 | - | adenosylhomocysteinase | - |
| 4683 | SNK | NM_006622.1 | 1171 | tgcataagggaagcaaggtatac | 100958 | 767335 | kinase_related、cell_cycle | protein serine/threonine kinase | - |

Figure 46

| | | | | | | | | |
|---|---|---|---|---|---|---|---|---|
| 4684 | BS69 | NM_006624.1 | 310 | tggtcttattgtcgaaactctaa | 23111 | 453068 | cell_cycle, transcription_regulator | DNA binding |
| 4685 | BS69 | NM_006624.1 | 446 | ttgcctgagaggtgttgatatg | 23112 | 453075 | see also 4684 line | - |
| 4686 | ZNF271 | NM_006629.3 | 981 | cagtaggctttcagatcttatta | 44920 | 470845 | - | DNA binding |
| 4687 | CYSLTR1 | NM_006639.2 | 530 | gtgtctatgtcctcataaaaac | 238895 | 623091 | receptor_acitivity, signal_transduction, gpcr | leukotriene receptor |
| 4688 | CYSLTR1 | NM_006639.2 | 565 | gtcagccttccaagtatacatga | 238896 | 623094 | see also 4687 line | |
| 4689 | CYSLTR1 | NM_006639.2 | 568 | agccttccaagtatacatgatta | 238897 | 623096 | see also 4687 line | |
| 4690 | CYSLTR1 | NM_006639.2 | 572 | ttccaagtatacatgattaattt | 238899 | 623097 | see also 4687 line | |
| 4691 | CYSLTR1 | NM_006639.2 | 627 | tgcctctccgtgtgtctattat | 238901 | 623100 | see also 4687 line | |
| 4692 | CYSLTR1 | NM_006639.2 | 632 | ctccgtgtggtcattatgttca | 238902 | 623102 | see also 4687 line | |
| 4693 | CYSLTR1 | NM_006639.2 | 634 | ccgtgtggtcattatgttcaca | 238903 | 623103 | see also 4687 line | |
| 4694 | CYSLTR1 | NM_006639.2 | 780 | ttccagtcagaacattaattg | 238910 | 623107 | see also 4687 line | |
| 4695 | CCR9 | NM_006641.2 | 770 | tgctgctatacatcatcattca | 124413 | 528431 | receptor_acitivity, gpcr, signal_transduction | |
| 4696 | HSPH1 | NM_006644.1 | 419 | ccgtcagtcatcatcatttggatc | 85330 | 503677 | - | heat shock protein |
| 4697 | HSPH1 | NM_006644.1 | 2626 | tccaaatggcccaaatattgata | 85381 | 503733 | see also 4696 line | |
| 4698 | STARD10 | NM_006645.1 | 891 | cccatgggcgctgattacatca | 336030 | 709869 | - | |
| 4699 | FRS2 | NM_006654.2 | 287 | cagataaccatcggaacaagttt | 170564 | 567118 | receptor_acitivity, phosphatase, gpcr, kinase_related, signal_transduction | |
| 4700 | FRS2 | NM_006654.2 | 516 | gtgtgccgtgcagaagaattat | 170571 | 567124 | see also 4699 line | |
| 4701 | FRS2 | NM_006654.2 | 521 | ccgtgcagaagaatttattaac | 170572 | 567127 | see also 4699 line | |
| 4702 | FRS2 | NM_006654.2 | 1386 | tggctaccataataatctagatc | 170590 | 567142 | see also 4699 line | |
| 4703 | FRS2 | NM_006654.2 | 1387 | ggctaccataataatctagatcc | 170591 | 567143 | see also 4699 line | |
| 4704 | TUBGCP2 | NM_006659.1 | 593 | tcccagcatgggtgatgagaga | 309650 | 675924 | - | |
| 4705 | TUBGCP2 | NM_006659.1 | 1025 | tgcagaagctgtgttctacatc | 309653 | 675930 | see also 4704 line | |
| 4706 | CLPX | NM_006660.3 | 592 | aactacctcgacaagtatgttgt | 77223 | 498488 | - | chaperonin ATPase |
| 4707 | CLPX | NM_006660.3 | 595 | tacctcgacaagtatgttgttgg | 77224 | 498489 | see also 4706 line | |
| 4708 | CLPX | NM_006660.3 | 766 | gaggatgagtacagatttacaaa | 77233 | 498499 | see also 4706 line | |
| 4709 | PDE10A | NM_006661.1 | 327 | accccccaggtattagatgaatt | 111139 | 589948 | - | 3', 5'-cyclic-nucleotide phosphodiesterase |
| 4710 | PDE10A | NM_006661.1 | 597 | tccttggagagtgcaataatagc | 111149 | 589954 | see also 4709 line | |

Figure 46

| | | | | | | | | |
|---|---|---|---|---|---|---|---|---|
| 4711 | PDE10A | NM_006661.1 | 1823 | cactgcatgtgccatacttca | 111189 | 589975 | | see also 4709 line |
| 4712 | PDE10A | NM_006661.1 | 2415 | ccgtggccattccctgctataca | 111207 | 589993 | | see also 4709 line |
| 4713 | HPSE | NM_006665.2 | 1219 | tggctttagtggctggataaat | 202288 | 767299 | - | - |
| 4714 | TPBG | NM_006670.3 | 1612 | atggaaaggtatcattacagata | 312851 | 702710 | - | - |
| 4715 | TPBG | NM_006670.3 | 1613 | tggaaggtatcattacagatat | 312852 | 702711 | - | see also 4714 line |
| 4716 | FGL2 | NM_006682.1 | 899 | ttcatcttctgaccaagagtaag | 329295 | 677587 | | - |
| 4717 | FGL2 | NM_006682.1 | 909 | gaccaagagtaaggaaatgattc | 329296 | 677588 | | see also 4716 line |
| 4718 | FGL2 | NM_006682.1 | 910 | accaagagtaaggaaatgattct | 329297 | 677589 | | see also 4716 line |
| 4719 | FGL2 | NM_006682.1 | 1230 | aggtgtccgtaatgggatttct | 329314 | 677600 | | see also 4716 line |
| 4720 | FGL2 | NM_006682.1 | 1232 | gtgtccgtaatgggatttctgg | 329315 | 677601 | | see also 4716 line |
| 4721 | ACTL7A | NM_006687.2 | 947 | tcctctgctcggagatgttcttc | 286237 | 659082 | - | |
| 4722 | CRF | NM_006688.3 | 684 | caccaacctaggcaacaactacg | 278825 | 688737 | molecular_function unknown | |
| 4723 | MMP24 | NM_006690.2 | 336 | ctccactatgcagcagtttacg | 158318 | 558900 | zinc binding | - |
| 4724 | MMP24 | NM_006690.2 | 619 | gttgccataccatgagatcaaag | 158326 | 558905 | | see also 4723 line |
| 4725 | MMP24 | NM_006690.2 | 1628 | gggactactgaagtttgacaac | 158334 | 558912 | | see also 4723 line |
| 4726 | CPSF4 | NM_006693.1 | 526 | ttcatgcaccctcgatttgaact | 58903 | 482196 | | - |
| 4727 | BRD8 | NM_006696.3 | 2109 | aagaagaatcagggactatttt | 1606 | 634333 | transcription_regulator | transcription_regulator |
| 4728 | BRD8 | NM_006696.3 | 2195 | agcctagagagagctaaggaaga | 1607 | 634334 | - | see also 4727 line |
| 4729 | BRD8 | NM_006696.3 | 2715 | cgcagttgattatgcaaacatcc | 1620 | 634345 | - | see also 4727 line |
| 4730 | SUGT1 | NM_006704.2 | 986 | aactggtctgatgtaggtaaaag | 307560 | 725349 | cell_cycle | cell cycle |
| 4731 | TCERG1 | NM_006706.2 | 343 | tcctcctccggtatgatgttc | 112492 | 708482 | transcription_regulator | transcription_regulator |
| 4732 | TCERG1 | NM_006706.2 | 442 | aactccagatggaaggtttatt | 112496 | 708483 | - | see also 4731 line |
| 4733 | TCERG1 | NM_006706.2 | 1178 | agctgccgtatgtaaagacagt | 112510 | 708496 | - | see also 4731 line |
| 4734 | ASK | NM_006716.2 | 1274 | aaccagtacaagtgttgatga | 58077 | 481017 | Alzheimer disease / kinase_related | |
| 4735 | LRBA | NM_006726.1 | 1022 | agcaaaggtcttggctattctgc | 281555 | 688224 | molecular_function unknown | |
| 4736 | LRBA | NM_006726.1 | 2709 | ttgcgaccctacttcgaaattct | 281620 | 688291 | - | see also 4735 line |
| 4737 | LRBA | NM_006726.1 | 2711 | gcgaccctacttcgaaattctcc | 281621 | 688292 | - | see also 4735 line |
| 4738 | LRBA | NM_006726.1 | 2933 | ctgctttaccatgcagtcaaata | 281626 | 688300 | - | see also 4735 line |
| 4739 | LRBA | NM_006726.1 | 4263 | cagttatggacttcgtgaatagc | 281655 | 688332 | - | see also 4735 line |
| 4740 | LRBA | NM_006726.1 | 4274 | ttcgtgaatagcagtgataatgt | 281656 | 688333 | - | see also 4735 line |
| 4741 | LRBA | NM_006726.1 | 6036 | atgagaagatgtgtgatcatttg | 281710 | 688360 | - | see also 4735 line |
| 4742 | LRBA | NM_006726.1 | 7356 | ttgtcaacttcaattattataat | 281761 | 688410 | - | see also 4735 line |
| 4743 | LRBA | NM_006726.1 | 7949 | gttggaaatcgatcctctcatagc | 281774 | 688424 | - | see also 4735 line |

Figure 46

| 4744 | LRBA | NM_006726.1 | 8736 | acgaccagaggtgcatcatttct | 281789 | 688436 | see also 4735 line | | |
|------|------|------|------|------|------|------|------|------|------|
| 4745 | DIAPH2 | NM_006729.2 | 513 | gtgcgtgaccgaattacaagttt | 116558 | 521554 | - | - | premature ovarian failure |
| 4746 | DIAPH2 | NM_006729.2 | 514 | tgcgtgaccgaattacaagtttt | 116559 | 521555 | see also 4745 line | | |
| 4747 | DIAPH2 | NM_006729.2 | 515 | gcgtgaccgaattacaagtttta | 116560 | 521556 | see also 4745 line | | |
| 4748 | DIAPH2 | NM_006729.2 | 757 | gtgagatggttgtccagtatatt | 116570 | 521565 | see also 4745 line | | |
| 4749 | DIAPH2 | NM_006729.2 | 3062 | aagcaaagtttcagctcaaattc | 116639 | 521628 | see also 4745 line | | |
| 4750 | FCMD | NM_006731.1 | 267 | cacacagtggcgtgcagttaaaa | 319895 | 687772 | - | - | muscular dystrophy |
| 4751 | FCMD | NM_006731.1 | 487 | tggtttcggatagctgagaatat | 319900 | 687781 | see also 4750 line | | |
| 4752 | FCMD | NM_006731.1 | 721 | aagttacagtttggtcgttatcc | 319911 | 687790 | see also 4750 line | | |
| 4753 | FSHPRH1 | NM_006733.1 | 747 | accaaggtactttttatcgttg | 320326 | 758330 | - | - | - |
| 4754 | FSHPRH1 | NM_006733.1 | 903 | gagaatgtcaaaccatttcgtgt | 320327 | 758342 | see also 4753 line | | |
| 4755 | FSHPRH1 | NM_006733.1 | 2418 | agctggtatttggactatttatt | 320354 | 758392 | see also 4753 line | | |
| 4756 | HIVEP2 | NM_006734.2 | 194 | cagctctaggacaaaaagctacc | 336155 | 701028 | transcription_regulator | DNA binding | - |
| 4757 | HIVEP2 | NM_006734.2 | 1781 | tagactcttcaccccttattaga | 336198 | 701073 | see also 4756 line | | |
| 4758 | HOXA2 | NM_006735.3 | 282 | tacgaatttgagcgagagattgg | 5037 | 444699 | transcription_regulator | transcription factor | - |
| 4759 | HOXA2 | NM_006735.3 | 349 | ttccccctgtcgctgatacattt | 5038 | 444701 | see also 4758 line | | |
| 4760 | HOXA2 | NM_006735.3 | 350 | tccccctgtcgctgatacatttc | 5039 | 444702 | see also 4758 line | | |
| 4761 | HOXA2 | NM_006735.3 | 1190 | tggagctggcctaaacaatgaca | 5046 | 444711 | see also 4758 line | | |
| 4762 | MCM5 | NM_006739.2 | 252 | atgaactcaagcggcattacaac | 31908 | 461512 | phosphatase、cell_cycle、transcription_regulator | DNA dependent adenosinetriphosphatase | - |
| 4763 | PPP1R1A | NM_006741.2 | 372 | tccacggcaacggaagaagatga | 294204 | 664349 | phosphatase、signal_transduction | type 1 serine/threonine specific protein phosphatase inhibitor | - |
| 4764 | PSKH1 | NM_006742.1 | 204 | agcccttcagtggcactaagagt | 98263 | 501062 | kinase_related | protein kinase CK2 | - |
| 4765 | SNTB2 | NM_006750.2 | 610 | tccgagaagtaacaccatatatc | 119001 | 523809 | - | - | - |
| 4766 | SNTB2 | NM_006750.2 | 611 | ccgagaagtaacaccatatatca | 119002 | 523810 | see also 4765 line | | |
| 4767 | SNTB2 | NM_006750.2 | 899 | agctatccacaccaacataatgg | 119008 | 523816 | see also 4765 line | | |
| 4768 | UGP2 | NM_006759.2 | 85 | atgtcgagatttgtacaagatct | 209034 | 449742 | kinase_related | kinase | - |
| 4769 | UGP2 | NM_006759.2 | 248 | tggatggatttcggaagctattt | 209038 | 449746 | see also 4768 line | | |
| 4770 | UGP2 | NM_006759.2 | 1356 | tcctacagtgcccttggttaaat | 209075 | 449795 | see also 4768 line | | |
| 4771 | N33 | NM_006765.2 | 1087 | atggggatggttcttctaaatga | 265525 | 742078 | - | - | prostate cancer |
| 4772 | LZTR1 | NM_006767.1 | 2344 | aacgcaggcactggacatgaagc | 336315 | 506701 | - | - | - |
| 4773 | BRAP | NM_006768.2 | 655 | gaccagtcatgacccttatgaagt | 177024 | 733323 | - | - | Alzheimer disease |

Figure 46

| | | | | | | | | | |
|---|---|---|---|---|---|---|---|---|---|
| 4774 | BRAP | NM_006768.2 | 658 | cagtcatgaccttatgaagtttg | 177025 | 733324 | see also 4773 line | | |
| 4775 | LMO4 | NM_006769.2 | 1154 | gagatcggtttcactacatcaat | 6213 | 445404 | - | transcription factor | - |
| 4776 | ZNF-kaiso | NM_006777.2 | 1447 | cactatgagttaatagtagatgg | 137230 | 535002 | - | - | - |
| 4777 | ZNF-kaiso | NM_006777.2 | 1977 | tggcagtactgagtttgaattta | 137254 | 535017 | see also 4776 line | | |
| 4778 | TRIM10 | NM_006778.2 | 842 | agcactctaataagatgtgaaac | 160242 | 541542 | - | - | - |
| 4779 | MALT1 | NM_006785.2 | 1544 | atgctccaaatccatataggtct | 133773 | 581304 | apoptosis、signal_transduction、kinase_related | - | non-hodgkin lymphoma、lymphoma |
| 4780 | MALT1 | NM_006785.2 | 1995 | gtgggccaaggctcatgaacttc | 133781 | 581315 | see also 4779 line | | |
| 4781 | TTID | NM_006790.1 | 1647 | ttcgaccaacattcagcaaatat | 289504 | 711795 | - | - | - |
| 4782 | SF3A3 | NM_006802.1 | 174 | aacctgagggatttgtatgatga | 46813 | 486183 | - | pre-mRNA splicing factor | - |
| 4783 | AP3M2 | NM_006803.2 | 387 | gagccagtgatcaaagacaatgt | 267805 | 672960 | - | protein transporter | - |
| 4784 | STARD3 | NM_006804.2 | 517 | cagtgcattcctcattgtcaagg | 173826 | 570503 | - | lipid binding | - |
| 4785 | PDIR | NM_006810.1 | 894 | cacctgaccgatgaagactttga | 224706 | 620013 | - | protein disulfide isomerase | - |
| 4786 | TDE1 | NM_006811.2 | 526 | atgggttttggttcttcaaaatt | 327575 | 693322 | - | - | - |
| 4787 | STIP1 | NM_006819.1 | 1338 | accttcatcaagggttatacacg | 314140 | 706499 | - | - | breast cancer |
| 4788 | COPS6 | NM_006833.4 | 307 | caccgtggaagagaagattatca | 56229 | 476404 | - | - | - |
| 4789 | COPS6 | NM_006833.4 | 386 | ctggagtttctgggttggtatac | 56230 | 476407 | see also 4788 line | | |
| 4790 | COPS6 | NM_006833.4 | 387 | tggagtttctgggttggtatacc | 56231 | 476408 | see also 4788 line | | |
| 4791 | COPS5 | NM_006837.2 | 400 | gagtatcgatgaaatctacaaat | 56190 | 704950 | - | - | - |
| 4792 | COPS5 | NM_006837.2 | 905 | ggctacaaacctcctgatgaagg | 56205 | 704971 | see also 4791 line | | |
| 4793 | COPS5 | NM_006837.2 | 996 | atgccttagaagtctcatatttc | 56210 | 704978 | see also 4791 line | | |
| 4794 | IMMT | NM_006839.1 | 2272 | aagcccgaatgaccctagaaacg | 336462 | 740186 | - | - | - |
| 4795 | IMMT | NM_006839.1 | 2325 | gccagcgccgtaggaataggaac | 336467 | 740189 | see also 4794 line | | |
| 4796 | TLK2 | NM_006852.1 | 371 | aggcactcctagggtgacataaaa | 336474 | 516557 | kinase_related、cell_cycle | - | - |
| 4797 | TLK2 | NM_006852.1 | 372 | ggcactcctaggggacataaaat | 336475 | 516558 | see also 4796 line | | |
| 4798 | TLK2 | NM_006852.1 | 1987 | gtgctggtacttattggtattta | 336482 | 516598 | see also 4796 line | | |
| 4799 | TLK2 | NM_006852.1 | 2187 | ttcccgccaaagccagtagtaac | 336485 | 516605 | see also 4796 line | | |
| 4800 | TLK2 | NM_006852.1 | 2188 | tcccgccaaagccagtagtaaca | 336486 | 516606 | see also 4796 line | | |
| 4801 | TLK2 | NM_006852.1 | 2190 | ccgccaaagccagtagtaacacc | 336487 | 516607 | see also 4796 line | | |
| 4802 | ATF7 | NM_006856.1 | 253 | aacgcctactccaactagattcc | 1155 | 759805 | transcription_regulator | translation regulator | - |
| 4803 | ATF7 | NM_006856.1 | 1211 | cagaacattcagctgagtaatga | 1164 | 759823 | see also 4802 line | | |
| 4804 | RY1 | NM_006857.1 | 438 | caggcagtacatgaatcgaaaag | 65645 | 717748 | - | - | - |

## Figure 46

| | | | | | | | | | |
|---|---|---|---|---|---|---|---|---|---|
| 4805 | RY1 | NM_006857.1 | 439 | aggcagtacatgaatcgaaaagg | 65646 | 717749 | see also 4804 line | | |
| 4806 | RAB35 | NM_006861.4 | 391 | cggggtcattgtggtttacgacg | 110212 | 812221 | signal_transduction | protein transporter | - |
| 4807 | RAB31 | NM_006868.2 | 272 | gccttgtggaaatgaacttcaca | 110136 | 518080 | signal_transduction | RAB small monomeric GTPase | - |
| 4808 | CENTA1 | NM_006869.1 | 1056 | cgcccgaggggaagtcttcattg | 23967 | 453697 | signal_transduction | - | - |
| 4809 | CENTA1 | NM_006869.1 | 1057 | gcccgaggggaagtcttcattgg | 23968 | 453698 | see also 4808 line | | |
| 4810 | ELF2 | NM_006874.1 | 240 | aagccctgcttcatatggaatct | 3058 | 443249 | transcription_regulator | - | - |
| 4811 | ELF2 | NM_006874.1 | 784 | aggacagaggcttgtatatcagt | 3077 | 443270 | see also 4810 line | | |
| 4812 | SHOX2 | NM_006884.1 | 40 | cggcgttcgtctccaagtctttt | 11207 | 448380 | transcription_regulator | transcription factor | - |
| 4813 | ATBF1 | NM_006885.2 | 1239 | ccgtgtacccgcagatcatcaac | 48 | 441838 | transcription_regulator | translation regulator | - |
| 4814 | ATBF1 | NM_006885.2 | 1276 | ctcatccttcgggaaatggtttg | 49 | 441839 | see also 4813 line | | |
| 4815 | ATBF1 | NM_006885.2 | 1433 | tcctgcgtatccaaagatgttcc | 53 | 441842 | see also 4813 line | | |
| 4816 | ATBF1 | NM_006885.2 | 2824 | gagctacacgtgtggttacaagc | 74 | 441862 | see also 4813 line | | |
| 4817 | ATBF1 | NM_006885.2 | 2850 | tccgctgcgaggtgtgtaactac | 75 | 441863 | see also 4813 line | | |
| 4818 | ATBF1 | NM_006885.2 | 9551 | gtcctcaagtcatgctttaatga | 177 | 441921 | see also 4813 line | | |
| 4819 | ATBF1 | NM_006885.2 | 9786 | ctgtacgtgaccatatcttttcc | 179 | 441927 | see also 4813 line | | |
| 4820 | ATBF1 | NM_006885.2 | 11366 | gccaaagagcaccctagtttatt | 196 | 441941 | see also 4813 line | | |
| 4821 | ATBF1 | NM_006885.2 | 11372 | gagcaccctagtttattacctca | 197 | 441942 | see also 4813 line | | |
| 4822 | ATBF1 | NM_006885.2 | 11760 | taggaacggacaccttcagattg | 198 | 441944 | see also 4813 line | | |
| 4823 | DNMT3B | NM_006892.3 | 1001 | acctcgtgtggggaaagatcaag | 25461 | 458606 | - | - | - |
| 4824 | DNMT3B | NM_006892.3 | 1163 | ggctgttcagccagcactttaat | 25464 | 458608 | see also 4823 line | | |
| 4825 | FMO3 | NM_006894.3 | 1203 | cccgctgggcagcacaagtaata | 221322 | 608295 | - | electron transfer flavoprotein | trimethylaminuria |
| 4826 | HNMT | NM_006895.1 | 657 | gtgggactttattcatatgattc | 214275 | 604599 | - | histamine N-methyltransferase | asthma |
| 4827 | HOXA7 | NM_006896.2 | 151 | gtgaacgcgctttttagcaaata | 5080 | 444737 | transcription_regulator | transcription factor | - |
| 4828 | HOXA7 | NM_006896.2 | 155 | acgcgcttttagcaaatatacg | 5082 | 444738 | see also 4827 line | | |
| 4829 | HOXC9 | NM_006897.1 | 139 | ctcatctctcacgacaatgaaga | 5197 | 444835 | transcription_regulator | transcriptional activator | - |
| 4830 | HOXC9 | NM_006897.1 | 226 | gtgccggactgtagcgattttcc | 5198 | 444836 | see also 4829 line | | |
| 4831 | HOXC9 | NM_006897.1 | 501 | ccgcagctacccggactacatgt | 5200 | 444838 | see also 4829 line | | |
| 4832 | IDH3B | NM_006899.2 | 463 | atgactcggcacaacaatctaga | 220218 | 607484 | - | - | - |
| 4833 | IDH3B | NM_006899.2 | 465 | gactcggcacaacaatctagacc | 220219 | 607485 | see also 4832 line | | |
| 4834 | MYO9A | NM_006901.1 | 6771 | ctctcccgaactcatctcaatac | 93720 | 679853 | - | - | - |

Figure 46

| | | | | | | | | | |
|---|---|---|---|---|---|---|---|---|---|
| 4835 | MYO9A | NM_006901.1 | 6776 | ccgaactcatctcaatacactgg | 93721 | 679855 | see also 4834 line | | |
| 4836 | MYO9A | NM_006901.1 | 6832 | tgcaggaagacactaatcgaatg | 93726 | 679857 | see also 4834 line | | |
| 4837 | MYO9A | NM_006901.1 | 6847 | atcgaatgctgctaatgcttg | 93727 | 679858 | see also 4834 line | | |
| 4838 | PRRX1 | NM_006902.2 | 1117 | ccctgcaggcgaaaagaacttc | 9996 | 447983 | transcription_regulator | - | |
| 4839 | PRRX1 | NM_006902.2 | 1707 | agcgccatgctacttattctgc | 10005 | 447993 | see also 4838 line | | |
| 4840 | PRRX1 | NM_006902.2 | 1797 | aaggccaaggaatatagtttaca | 10007 | 447997 | see also 4838 line | | |
| 4841 | PPA2 | NM_006903.3 | 266 | cacgaaatgatgaatatgagaat | 166554 | 585759 | phosphatase | - | |
| 4842 | PRKDC | NM_006904.6 | 3112 | gacccgttgacagtacttaag | 205670 | 596484 | kinase_related | protein kinase CK2 | agammaglobulinemia, severe combined immunodeficiency disease |
| 4843 | PRKDC | NM_006904.6 | 5594 | atgccattgatgtgttgaagtcc | 205747 | 596561 | see also 4842 line | | |
| 4844 | PRKDC | NM_006904.6 | 6680 | atgaagtgttagcaaatcgattg | 205771 | 596601 | see also 4842 line | | |
| 4845 | PRKDC | NM_006904.6 | 6685 | gtgttagcaaatcgattgcttaa | 205772 | 596602 | see also 4842 line | | |
| 4846 | PRKDC | NM_006904.6 | 7787 | tcgagcatcctctgtcagaatgc | 205810 | 596643 | see also 4842 line | | |
| 4847 | RASGRF2 | NM_006909.1 | 430 | atgcagaagtacattcatctagt | 296504 | 664876 | - | | |
| 4848 | RBBP6 | NM_006910.4 | 3251 | cagaggccaagagccgcaattacc | 135012 | 465522 | cell_cycle | - | |
| 4849 | RORB | NM_006914.2 | 1157 | ccgtacccaacttgtttacctat | 10778 | 448156 | transcription_regulator, receptor_acitivity | steroid hormone receptor | - |
| 4850 | RORB | NM_006914.2 | 1518 | ccgtgccttcaaccattaaaca | 10786 | 448170 | see also 4849 line | | |
| 4851 | RORB | NM_006914.2 | 1520 | gtgccttcaaccattaaacaac | 10787 | 448171 | see also 4849 line | | |
| 4852 | RP2 | NM_006915.1 | 301 | tggactgaaggatgaaaacagtag | 99703 | 514180 | kinase_related | protein binding | - |
| 4853 | SFRS1 | NM_006924.3 | 568 | gttggagtttgtacggaaagaaga | 46983 | 486244 | - | pre-mRNA splicing factor | |
| 4854 | SFRS1 | NM_006924.3 | 600 | tgcagttcgaaaactggataaaca | 46987 | 486248 | see also 4853 line | | |
| 4855 | SFRS1 | NM_006924.3 | 602 | cagttcgaaaactggataaacact | 46988 | 486249 | see also 4853 line | | |
| 4856 | SFRS1 | NM_006924.3 | 605 | ttcgaaaactggataaacactaag | 46989 | 486250 | see also 4853 line | | |
| 4857 | SFRS1 | NM_006924.3 | 606 | tcgaaaactggataaacactaagt | 46990 | 486251 | see also 4853 line | | |
| 4858 | SFRS1 | NM_006924.3 | 652 | gcctacatccgggttaaagttga | 46992 | 486252 | see also 4853 line | | |
| 4859 | SILV | NM_006928.2 | 1462 | gacatgtccagggtattgaaag | 303256 | 671730 | - | ATP dependent RNA helicase | - |
| 4860 | SKIV2L | NM_006929.3 | 2348 | tggcctggtcaaggtcttgtttg | 54817 | 486311 | - | | - |
| 4861 | SLC6A9 | NM_006934.1 | 338 | gggcaaccagatcgagtttgtac | 255776 | 640181 | | sodium:amino acid transporter | - |
| 4862 | SLC6A9 | NM_006934.1 | 339 | ggcaaccagatcgagtttgtact | 255777 | 640182 | see also 4861 line | | |
| 4863 | SLC6A9 | NM_006934.1 | 545 | gaggatcagcccatgttcaaag | 255778 | 640185 | see also 4861 line | | |
| 4864 | SLC6A9 | NM_006934.1 | 546 | aggatcagcccatgttcaaagg | 255779 | 640186 | see also 4861 line | | |
| 4865 | SYN1 | NM_006950.2 | 1200 | tggacacgtgctcagagatttt | 336634 | 524607 | - | | - |
| 4866 | SYN1 | NM_006950.2 | 1202 | gacacgtgctcagagattttgg | 336635 | 524608 | see also 4865 line | | |

Figure 46

| 4867 | TAF5 | NM_006951.2 | 869 | atctagtcttaccaaaaaggaac | 12575 | 795205 | transcription_regulator | - | |
|---|---|---|---|---|---|---|---|---|---|
| 4868 | TAF5 | NM_006951.2 | 1555 | cagatcttagtcttatagacaaa | 12595 | 795226 | see also 4867 line | | |
| 4869 | TAF5 | NM_006951.2 | 2200 | tggcatcaggttcaatggataat | 12621 | 795249 | see also 4867 line | | |
| 4870 | TAF5 | NM_006951.2 | 2346 | accaaatcaacaccagttgtaca | 12625 | 795258 | see also 4867 line | | |
| 4871 | ZNF183 | NM_006978.1 | 922 | gagggtcgctatggtgtctatga | 67370 | 479244 | - | nucleic acid binding | - |
| 4872 | ZNF183 | NM_006978.1 | 924 | gggtcgctatggtgtctatgagg | 67371 | 479245 | see also 4871 line | | |
| 4873 | SLC39A7 | NM_006979.1 | 1298 | tgctgacttggcacacaacttca | 267133 | 654338 | - | heavy metal ion transporter | - |
| 4874 | NR4A3 | NM_006981.2 | 1930 | gcccttgtccgagctttaacaga | 8661 | 447340 | transcription_regulator, receptor_acitivity | - | extraskeletal myxoid chrondrosarcoma、 chondrosarcoma |
| 4875 | NR4A3 | NM_006981.2 | 1953 | ctcaacacccagagatcttgatt | 8662 | 447341 | see also 4874 line | | |
| 4876 | NR4A3 | NM_006981.2 | 2182 | aggtcaaacactgctgaagataa | 8666 | 447348 | see also 4874 line | | |
| 4877 | NR4A3 | NM_006981.2 | 2300 | tgcagagcctgaaccttgatatc | 8676 | 447355 | see also 4874 line | | |
| 4878 | CART1 | NM_006982.1 | 1104 | gagcacaccgccaatatttcatg | 1962 | 442530 | transcription_regulator | transcription factor | - |
| 4879 | CART1 | NM_006982.1 | 1105 | agcacaccgccaatatttcatgg | 1963 | 442531 | see also 4878 line | | |
| 4880 | ADAMTS1 | NM_006988.2 | 2347 | atcgcatcatagactccaaaaag | 155214 | 556277 | signal_transduction | zinc binding | - |
| 4881 | SLC19A2 | NM_006996.1 | 1351 | ctgattgctgctgcagtgtatat | 179718 | 573018 | - | thiamin transporter | megaloblastic anemia |
| 4882 | SLC19A2 | NM_006996.1 | 1396 | tgggtgtgctatgcatcctatgt | 179721 | 573019 | see also 4881 line | | |
| 4883 | TACC2 | NM_006997.1 | 189 | gagtcaacgacccctgtcaaagc | 322198 | 686736 | - | - | - |
| 4884 | TACC2 | NM_006997.1 | 686 | tacccttaagcgaactaaaaaac | 322206 | 686746 | see also 4883 line | | |
| 4885 | TACC2 | NM_006997.1 | 687 | acccttaagcgaactaaaaaacc | 322207 | 686747 | see also 4883 line | | |
| 4886 | TACC2 | NM_006997.1 | 1110 | aggctggagtttgactattctga | 322212 | 686751 | see also 4883 line | | |
| 4887 | POLS | NM_006999.2 | 75 | aggtggtgaaacggatcgaaact | 36442 | 813358 | - | - | - |
| 4888 | TLE4 | NM_007005.3 | 919 | tcctgtgatcggattaaggaaga | 284757 | 526109 | - | - | - |
| 4889 | TLE4 | NM_007005.3 | 1801 | acccctactccacgaactgatgc | 284773 | 526123 | see also 4888 line | | |
| 4890 | DDX52 | NM_007010.2 | 529 | agcaacttgaccaggaatataaa | 59435 | 483376 | - | - | - |
| 4891 | DDX52 | NM_007010.2 | 1239 | ctggccatgagagaacttgttaa | 59451 | 483397 | see also 4890 line | | |
| 4892 | WWP2 | NM_007014.3 | 1333 | aggacaatggacgggtgtattac | 336761 | 614950 | - | - | - |
| 4893 | SOX30 | NM_007017.2 | 1244 | tactggagccttctgtaaaaatt | 40601 | 467943 | transcription_regulator | - | |
| 4894 | SOX30 | NM_007017.2 | 1398 | accagcactagccaaagctaacc | 40605 | 467946 | see also 4893 line | | |
| 4895 | SOX30 | NM_007017.2 | 1588 | ttccctctaagtgtttccaatgt | 40608 | 467950 | see also 4893 line | | |
| 4896 | SOX30 | NM_007017.2 | 1614 | ttctggtaccacacagaatatca | 40609 | 467952 | see also 4893 line | | |
| 4897 | CEP1 | NM_007018.3 | 477 | tacgtgaactcaacttatcatat | 275589 | 741261 | - | - | - |
| 4898 | CEP1 | NM_007018.3 | 478 | acgtgaactcaacttatcatata | 275590 | 741262 | see also 4897 line | | |

Figure 46

| | | | | | | | | |
|---|---|---|---|---|---|---|---|---|
| 4899 | CEP1 | NM_007018.3 | 480 | gtgaactcaacttatcatataac | 275591 | 741263 | | see also 4897 line |
| 4900 | CEP1 | NM_007018.3 | 1144 | tccatcagagtatgctgaaattg | 275608 | 741282 | | see also 4897 line |
| 4901 | CEP1 | NM_007018.3 | 1517 | aagcagttgagtggtagactaca | 275616 | 741291 | | see also 4897 line |
| 4902 | CEP1 | NM_007018.3 | 2057 | gagcttgcagagctagaaagtgc | 275635 | 704002 | | see also 4897 line |
| 4903 | CGEF2 | NM_007023.1 | 151 | tggatcgcctgcctgataaaag | 336786 | 531339 | kinase related, signal transduction | - |
| 4904 | CGEF2 | NM_007023.1 | 363 | agggtctttgatgttaaagtat | 336797 | 531354 | | see also 4903 line |
| 4905 | CGEF2 | NM_007023.1 | 473 | cccgccatgcaaccatcgttacc | 336798 | 531355 | | see also 4903 line |
| 4906 | CGEF2 | NM_007023.1 | 601 | atggaaacgggctctaacaatga | 336800 | 531359 | | see also 4903 line |
| 4907 | CGEF2 | NM_007023.1 | 1491 | ggcgaatacagtcagacttaaag | 336822 | 531376 | | see also 4903 line |
| 4908 | CGEF2 | NM_007023.1 | 1492 | gcgaatacagtcagacttaaaga | 336823 | 531377 | | see also 4903 line |
| 4909 | CGEF2 | NM_007023.1 | 1585 | cagcctcagcaaagtatactgt | 336824 | 531379 | | see also 4903 line |
| 4910 | CGEF2 | NM_007023.1 | 2302 | acgacgctcaccattaatggacg | 336841 | 531397 | | see also 4903 line |
| 4911 | CGEF2 | NM_007023.1 | 2503 | cacacatttggaaggcataattt | 336849 | 531402 | | see also 4903 line |
| 4912 | CGEF2 | NM_007023.1 | 2505 | cacatttggaaggcataatttta | 336850 | 531403 | | see also 4903 line |
| 4913 | CGEF2 | NM_007023.1 | 2742 | ggcactaacgtgggagaaactgc | 336860 | 531409 | | see also 4903 line |
| 4914 | CGEF2 | NM_007023.1 | 2914 | gaggggaacaagacgttcattga | 336866 | 531416 | | see also 4903 line |
| 4915 | CGEF2 | NM_007023.1 | 2920 | aacaagacgttcattgacaatct | 336868 | 531417 | | see also 4903 line |
| 4916 | CGEF2 | NM_007023.1 | 3049 | caggatgtccggagttatgtacg | 336876 | 531422 | | see also 4903 line |
| 4917 | CGEF2 | NM_007023.1 | 3058 | cggagttatgtacggcaattaaa | 336877 | 531423 | | see also 4903 line |
| 4918 | CGEF2 | NM_007023.1 | 3060 | gagttatgtacggcaattaaatg | 336878 | 531424 | | see also 4903 line |
| 4919 | TOPBP1 | NM_007027.2 | 484 | acgagtatacagagaccttaatg | 126343 | 620033 | - | - |
| 4920 | TOPBP1 | NM_007027.2 | 518 | cacttattgcaggagaagttgg | 126345 | 620035 | | see also 4919 line |
| 4921 | HRIHFB2122 | NM_007032.3 | 177 | gacgcccgatctgctcaacttca | 114044 | 751771 | myosin II binding | |
| 4922 | HRIHFB2122 | NM_007032.3 | 179 | cgcccgatctgctcaacttcaag | 114045 | 751772 | | see also 4921 line |
| 4923 | DNAJB4 | NM_007034.3 | 691 | cggatgaagatttctcgaaaaag | 299044 | 668209 | heat shock protein | - |
| 4924 | ADAMTS5 | NM_007038.1 | 1267 | cagacgttgggaccatatgttct | 155606 | 556405 | zinc binding | - |
| 4925 | ADAMTS5 | NM_007038.1 | 1269 | gacgttggaccatatgttctcc | 155607 | 556406 | | see also 4924 line |
| 4926 | ADAMTS5 | NM_007038.1 | 2033 | tggctatcagtctgatgcaaaag | 155621 | 556420 | | see also 4924 line |
| 4927 | PTPN21 | NM_007039.2 | 670 | aggagattaccaggtatcagtat | 193547 | 586953 | structural molecule phosphatase | - |
| 4928 | PTPN21 | NM_007039.2 | 3341 | aagggagaagagagctttaggtact | 193580 | 587002 | | see also 4927 line |
| 4929 | PTPN21 | NM_007039.2 | 3391 | ctgtcacctatggaagtttaag | 193581 | 587003 | | see also 4927 line |
| 4930 | ATE1 | NM_007041.1 | 1522 | atcatgccttaacggtgtttataa | 216113 | 603452 | arginyltransferase | - |

Figure 46

| 4931 | ATE1 | NM_007041.1 | 1526 | tgccttacggtgtttataagaaa | 216114 | 603453 | see also 4930 line | | |
|---|---|---|---|---|---|---|---|---|---|
| 4932 | ATE1 | NM_007041.1 | 1527 | gccttacggtgtttataagaaac | 216115 | 603454 | see also 4930 line | | |
| 4933 | KATNA1 | NM_007044.2 | 161 | atctgtactcagtcaaagataca | 86320 | 504812 | - | - | - |
| 4934 | PTPRT | NM_007050.3 | 4163 | cggccacaggatggttatcgtat | 194988 | 588039 | phosphatase、 receptor _acitivity、 signal_tran sduction、 kinase_relat ed | - | - |
| 4935 | PTPRT | NM_007050.3 | 4164 | ggccacaggatggttatcgtata | 194989 | 588040 | see also 4934 line | | |
| 4936 | FAF1 | NM_007051.2 | 696 | ttcagcgtttcgacctgtaatgc | 279805 | 677174 | apoptosis | - | - |
| 4937 | FAF1 | NM_007051.2 | 717 | gccatccaggcagattgtagaaa | 279806 | 677176 | see also 4936 line | | |
| 4938 | FAF1 | NM_007051.2 | 1818 | gggaaagcgatcatctaatgaag | 279826 | 677216 | see also 4936 line | | |
| 4939 | NOX1 | NM_007052.3 | 1652 | tggtcatgcagcattaaactttg | 223260 | 610237 | channel | - | - |
| 4940 | RPC155 | NM_007055.2 | 1208 | gaccccaacctccggattgatga | 38672 | 808656 | transcription_regulator | - | - |
| 4941 | RPC155 | NM_007055.2 | 1240 | gccagttcatgtggccaaaattc | 38674 | 808658 | see also 4940 line | | |
| 4942 | KPTN | NM_007059.1 | 200 | accaagacctccgacagaaaatc | 117887 | 747616 | - | - | - |
| 4943 | TBC1D8 | NM_007063.2 | 2126 | caggtttctagatcacattaaga | 309052 | 707368 | - | - | - |
| 4944 | TRAD | NM_007064.1 | 3624 | cggggttctgacatatgtcatgc | 102932 | 501241 | kinase_related | - | - |
| 4945 | JM5 | NM_007075.1 | 452 | gcgcatgcgccatgacaagatcg | 336932 | 772541 | - | - | - |
| 4946 | NUDT6 | NM_007083.3 | 508 | tggttgtacaagatcgaaataaa | 169397 | 770508 | - | - | - |
| 4947 | WDHD1 | NM_007086.1 | 438 | aggacatgatgcccctgttttaa | 43287 | 457784 | transcription_regulator | - | - |
| 4948 | WDHD1 | NM_007086.1 | 2384 | tggaacttgctgatctaatgact | 43352 | 457852 | see also 4947 line | | |
| 4949 | WDHD1 | NM_007086.1 | 2439 | ttctcgctctcggaaattaatac | 43356 | 457857 | see also 4947 line | | |
| 4950 | WDHD1 | NM_007086.1 | 2955 | ggcatctgcagcatcctatttcc | 43376 | 457873 | see also 4947 line | | |
| 4951 | NDUFV1 | NM_007103.2 | 136 | gacacgacagcacccaagaaaac | 222915 | 610147 | - | NADH dehydrogenase (ubiquinone) | Leigh syndrome |
| 4952 | SSR3 | NM_007107.2 | 430 | aagaatgaagttgctgattatga | 177258 | 721925 | - | signal sequence receptor | - |
| 4953 | TEP1 | NM_007110.3 | 1718 | tcggcagtttccattcagatttc | 55414 | 699062 | - | - | neuroblastoma |
| 4954 | TEP1 | NM_007110.3 | 3106 | ctgcccaagctctcatctacttc | 55447 | 699091 | see also 4953 line | | |
| 4955 | TEP1 | NM_007110.3 | 7754 | ttcgaaggacagagatgttaagc | 55512 | 699143 | see also 4953 line | | |
| 4956 | USF1 | NM_007122.2 | 591 | tggtcaattctttgtgatgatgt | 43241 | 470168 | transcription_regulator | USF43/USF44 complex | - |
| 4957 | USH2A | NM_007123.3 | 1191 | aagaatgcaagattttcgattat | 292573 | 662357 | - | - | retinitis pigmentosa、 Senear-Usher syndrome |
| 4958 | VIL1 | NM_007127.1 | 2168 | atcccttcaagtggagtaacacc | 114926 | 524894 | - | structural constituent of cytoskeleton | - |

Figure 46

| | | | | | | | | | |
|---|---|---|---|---|---|---|---|---|---|
| 4959 | VIL1 | NM_007127.1 | 2178 | gtggagtaacaccaaatcctatg | 114927 | 524895 | see also 4958 line | | |
| 4960 | ZNF184 | NM_007149.1 | 1050 | gagtgaaaaccttataaaccatc | 44362 | 807797 | - | zinc binding | - |
| 4961 | D1S155E | NM_007158.2 | 1035 | gtgatgttgaaagagatattc | 24691 | 457739 | transcription_regulator | RNA binding | Alzheimer disease |
| 4962 | SLMAP | NM_007159.1 | 347 | gcccaggagctagctagaaccag | 312457 | 693228 | see also 4962 line | - | |
| 4963 | SLMAP | NM_007159.1 | 348 | cccaggagctagctagaaacaagt | 312458 | 693229 | see also 4962 line | | |
| 4964 | SLMAP | NM_007159.1 | 418 | agcctatgaaatcaagttgagg | 312461 | 693232 | see also 4962 line | | |
| 4965 | SLC14A2 | NM_007163.2 | 1404 | ctggagaagttagactactact | 163849 | 561181 | - | urea transporter | |
| 4966 | PICALM | NM_007166.1 | 555 | ttcatacagacaagttgcatttg | 302707 | 761141 | - | - | |
| 4967 | PICALM | NM_007166.1 | 834 | atgcaagaaggtcttgacatct | 302713 | 761155 | see also 4966 line | | |
| 4968 | PICALM | NM_007166.1 | 1436 | atgatgccattccaagcttaaat | 302728 | 761178 | see also 4966 line | | |
| 4969 | PICALM | NM_007166.1 | 1587 | cccttcagctgccttaatgttg | 302732 | 761184 | see also 4966 line | | |
| 4970 | PICALM | NM_007166.1 | 1965 | aggcatgataggatatggaattc | 302738 | 761203 | see also 4966 line | | |
| 4971 | ABCA8 | NM_007168.1 | 2990 | atgggctacttgaatgttaaa | 69298 | 491492 | - | - | |
| 4972 | ABCA8 | NM_007168.1 | 2991 | tgggctacttgaatgttaaac | 69299 | 491493 | see also 4971 line | | |
| 4973 | POMT1 | NM_007171.2 | 1463 | ctcctgctaacttgactaaaca | 337000 | 600314 | - | - | Walker-Warburg syndrome |
| 4974 | POMT1 | NM_007171.2 | 2374 | aggccttcgctggaaagacagc | 337011 | 600328 | see also 4973 line | | |
| 4975 | CIT | NM_007174.1 | 1195 | ctgacgatgacacctccaatttt | 77012 | 498318 | kinase_related | - | |
| 4976 | CIT | NM_007174.1 | 1197 | gacgatgacacctccaatttga | 77013 | 498319 | see also 4975 line | | |
| 4977 | CIT | NM_007174.1 | 1219 | atgaaccagagaagaattcgtgg | 77015 | 498320 | see also 4975 line | | |
| 4978 | CIT | NM_007174.1 | 1461 | caggaaatgacccggttacatcg | 77022 | 498325 | see also 4975 line | | |
| 4979 | CIT | NM_007174.1 | 3810 | caggttcctctgcagtacaatga | 77059 | 498349 | see also 4975 line | | |
| 4980 | CIT | NM_007174.1 | 4207 | ttggacgccaggcatccaaatgt | 77065 | 498353 | see also 4975 line | | |
| 4981 | CIT | NM_007174.1 | 4888 | ggctctacgccctgaatgrcttg | 77086 | 498370 | see also 4975 line | | |
| 4982 | C14orf1 | NM_007176.1 | 147 | gagcttccgagaccacacttttc | 277438 | 667515 | - | molecular_function unknown | - |
| 4983 | C14orf1 | NM_007176.1 | 148 | agcttccgagaccacacttttct | 277439 | 667516 | see also 4982 line | | |
| 4984 | C14orf1 | NM_007176.1 | 189 | tggcaagccaaacttgtgaatg | 277442 | 667518 | see also 4982 line | | |
| 4985 | PKP3 | NM_007183.1 | 1223 | tgccatgcgcaacctcatctacg | 292246 | 709196 | - | structural molecule | - |
| 4986 | WBP4 | NM_007187.3 | 239 | aaggatcagtgagattaaaacaga | 55728 | 475958 | - | - | |
| 4987 | WIF1 | NM_007191.2 | 970 | tggaggtaaatgcattggtaaaa | 206349 | 596785 | kinase_related, signal transduction | structural molecule | - |
| 4988 | WIF1 | NM_007191.2 | 972 | gaggtaaatgcattggtaaaagc | 206350 | 596786 | see also 4987 line | | |
| 4989 | WIF1 | NM_007191.2 | 1226 | cggaatccacctgaatccaatta | 206357 | 596792 | see also 4987 line | | |
| 4990 | SUPT16H | NM_007192.2 | 1081 | cagaagtggtgcaactataatct | 57754 | 477813 | transcription_regulator | - | |
| 4991 | SUPT16H | NM_007192.2 | 1083 | gagtggtggcaactataatctca | 57755 | 477814 | see also 4990 line | | |

Figure 46

| 4992 | SUPT16H | NM_007192.2 | 2783 | gcctccttcagcccactagtagt | 57788 | 477865 | see also 4990 line | | |
|---|---|---|---|---|---|---|---|---|---|
| 4993 | SUPT16H | NM_007192.2 | 2785 | ctccttcagcccactagtagtgc | 57789 | 477866 | see also 4990 line | | |
| 4994 | SUPT16H | NM_007192.2 | 3035 | ccctcaactggactaaaatcatg | 57794 | 477870 | see also 4990 line | | |
| 4995 | ANXA10 | NM_007193.2 | 451 | gagaattgcctcattgaaatact | 137878 | 544044 | - | calcium-dependent phospholipid binding | - |
| 4996 | CHEK2 | NM_007194.2 | 1375 | ctgtagatgatcagtcagtttat | 76898 | 498207 | kinase_related、cell_cycle、signal_transduction | - | Li-Fraumeni Syndrome、breast cancer、prostate cancer、non-small-cell lung cancer、osteosarcoma |
| 4997 | CHEK2 | NM_007194.2 | 1381 | atgatcagtcagtttatcctaag | 76899 | 498208 | see also 4996 line | | |
| 4998 | CHEK2 | NM_007194.2 | 1419 | tacatcatgtcaaaaactcttgg | 76901 | 498212 | see also 4996 line | | |
| 4999 | POLI | NM_007195.1 | 611 | atgcgggaagccatgtataatca | 208824 | 669919 | - | - | - |
| 5000 | POLI | NM_007195.1 | 613 | gcgggaagccatgtataatcagt | 208825 | 669920 | see also 4999 line | | |
| 5001 | IRAK3 | NM_007199.1 | 224 | atgttcgtcatattgaaaagtat | 85898 | 504491 | receptor_acitivity、kinase_related、signal_transduction | - | - |
| 5002 | AKAP10 | NM_007202.2 | 1215 | ctgcgaagtcaccatttctgtaa | 128544 | 531760 | kinase_related、signal_transduction | signal transducer | - |
| 5003 | AKAP10 | NM_007202.2 | 1216 | tgcgaagtcaccatttctgtaaa | 128545 | 531761 | see also 5002 line | | |
| 5004 | AKAP10 | NM_007202.2 | 1498 | atgatgttgtacgattagaaatt | 128551 | 531770 | see also 5002 line | | |
| 5005 | AKAP10 | NM_007202.2 | 2000 | atgggtgcaaggaaatactgatg | 128561 | 531786 | see also 5002 line | | |
| 5006 | DDX20 | NM_007204.3 | 579 | gggcgctgcgggctcgatttaat | 24807 | 458064 | - | DNA binding | - |
| 5007 | DDX20 | NM_007204.3 | 580 | ggcgctgcgggctcgatttaatt | 24808 | 458065 | see also 5006 line | | |
| 5008 | DDX20 | NM_007204.3 | 581 | gcgctgcgggctcgatttaattg | 24809 | 458066 | see also 5006 line | | |
| 5009 | DDX20 | NM_007204.3 | 582 | cgctgcgggctcgatttaattgt | 24810 | 458067 | see also 5006 line | | |
| 5010 | DDX20 | NM_007204.3 | 958 | tccaggagcaaataaattggatt | 24825 | 458081 | see also 5006 line | | |
| 5011 | DDX20 | NM_007204.3 | 1490 | agctggccgttttggtacattgg | 24842 | 458104 | see also 5006 line | | |
| 5012 | DDX20 | NM_007204.3 | 1513 | ggctgacagtgacctactgttgc | 24843 | 458105 | see also 5006 line | | |
| 5013 | DUSP10 | NM_007207.3 | 343 | aggctgcgaatctgacgtatatg | 195510 | 588442 | phosphatase、kinase_related | - | - |
| 5014 | GALNT6 | NM_007210.2 | 534 | caccagcgtgatcattgtgttcc | 211132 | 599840 | - | - | - |
| 5015 | GALNT6 | NM_007210.2 | 892 | atcgtcaccatcgaccttaatac | 211134 | 599843 | see also 5014 line | | |
| 5016 | GALNT6 | NM_007210.2 | 1099 | gagcacatcggtacctatgataa | 211140 | 599847 | see also 5014 line | | |
| 5017 | GALNT6 | NM_007210.2 | 1100 | agcacatcggtacctatgataat | 211141 | 599848 | see also 5014 line | | |
| 5018 | GALNT6 | NM_007210.2 | 1431 | ttcctggtacctgcacaatgtct | 211147 | 599853 | see also 5014 line | | |
| 5019 | RNF2 | NM_007212.2 | 382 | gtgcccaatttgtttggatatgt | 337083 | 451147 | - | - | - |
| 5020 | RNF2 | NM_007212.2 | 990 | acgccactgttgatcacttatcc | 337107 | 451171 | see also 5019 line | | |
| 5021 | SEC63 | NM_007214.2 | 263 | ttcgattaaagaatatcagaaaa | 270475 | 652384 | - | protein transporter | - |

Figure 46

| | | | | | | | | | |
|---|---|---|---|---|---|---|---|---|---|
| 5022 | SEC63 | NM_007214.2 | 303 | gtggtatcgtttacggttattaa | 270478 | 652385 | see also 5021 line | | |
| 5023 | SEC63 | NM_007214.2 | 304 | tggtatcgtttacggttattaaa | 270479 | 652386 | see also 5021 line | | |
| 5024 | POLG2 | NM_007215.1 | 1206 | acccttgtttagcccctattaag | 19697 | 464274 | - | zeta DNA polymerase | - |
| 5025 | POLG2 | NM_007215.1 | 1207 | cccttgtttagcccctattaagg | 19698 | 464275 | see also 5024 line | | |
| 5026 | RNF139 | NM_007218.3 | 535 | atccagtattgttctgatcttgt | 250916 | 786616 | receptor_acitivity、sig nal_transduction | - | - |
| 5027 | RNF139 | NM_007218.3 | 1382 | gagagacttattcgcttaagtag | 250951 | 786651 | see also 5026 line | | |
| 5028 | RNF139 | NM_007218.3 | 1386 | gacttattcgcttaagtagaaac | 250952 | 786652 | see also 5026 line | | |
| 5029 | RNF139 | NM_007218.3 | 2067 | ggctgtacattcaagatacttgt | 250980 | 786682 | see also 5026 line | | |
| 5030 | RNF139 | NM_007218.3 | 2069 | ctgtacattcaagatacttgtcc | 250981 | 786683 | see also 5026 line | | |
| 5031 | RNF139 | NM_007218.3 | 2089 | tccaatgtgccatcagaaagtat | 250982 | 786684 | see also 5026 line | | |
| 5032 | RNF24 | NM_007219.2 | 280 | ttcaggatgcctaatattggatt | 337234 | 801749 | - | - | - |
| 5033 | CA5B | NM_007220.2 | 827 | gtctgtcacctggatcattaaga | 156253 | 557309 | - | zinc binding | - |
| 5034 | ZHX1 | NM_007222.2 | 775 | tgcactttctgagcataatctga | 14414 | 450237 | - | - | - |
| 5035 | ZHX1 | NM_007222.2 | 781 | ttctgagcataatctgaaatatc | 14415 | 450238 | see also 5034 line | | |
| 5036 | ZHX1 | NM_007222.2 | 1292 | acctacaatgctgcattggataa | 14436 | 450253 | see also 5034 line | | |
| 5037 | ZHX1 | NM_007222.2 | 1518 | tacctcagaccataactgttatt | 14439 | 450264 | see also 5034 line | | |
| 5038 | ZHX1 | NM_007222.2 | 1523 | cagaccataactgttattcctac | 14440 | 450266 | see also 5034 line | | |
| 5039 | ZHX1 | NM_007222.2 | 2006 | cagtgcttacatctcaacaatga | 14454 | 450284 | see also 5034 line | | |
| 5040 | ZHX1 | NM_007222.2 | 2776 | tgcaatacttaaggattattacc | 14466 | 450307 | see also 5034 line | | |
| 5041 | GPR45 | NM_007227.3 | 1108 | tccgcgaggcctgcatagagttg | 239881 | 745548 | receptor_acitivity、gpc r、signal_transduction | - | - |
| 5042 | GPR45 | NM_007227.3 | 1109 | ccgcgaggcctgcatagagttgc | 239882 | 745549 | see also 5041 line | | |
| 5043 | SLC6A14 | NM_007231.1 | 192 | accgtggtaactggtccaaaaaa | 255972 | 639971 | - | - | obesity |
| 5044 | SLC6A14 | NM_007231.1 | 374 | ctgggacaatttgctagcttagg | 255981 | 639976 | see also 5043 line | | |
| 5045 | KLHL2 | NM_007246.2 | 1488 | ctgtggaagcatacaacataaag | 117783 | 536853 | - | transporter | - |
| 5046 | KLHL2 | NM_007246.2 | 1622 | cagtgtcttagcacagtagaatg | 117787 | 536856 | see also 5045 line | | |
| 5047 | KLHL2 | NM_007246.2 | 1704 | gagcaggtgttggtgtgttaaac | 117788 | 536861 | see also 5045 line | | |
| 5048 | AP1GBP1 | NM_007247.3 | 1050 | gacagatatgggccttagctaat | 304913 | 810235 | - | - | - |
| 5049 | AP1GBP1 | NM_007247.3 | 1052 | cagatatgggccttagctaatcg | 304914 | 810236 | see also 5048 line | | |
| 5050 | KLF12 | NM_007249.3 | 321 | gtctccaaacgtccacaactatc | 5914 | 460956 | transcription_regulator | - | - |
| 5051 | KLF12 | NM_007249.3 | 434 | ctgagccagtggacttgtcaata | 5920 | 460961 | see also 5050 line | | |
| 5052 | KLF12 | NM_007249.3 | 801 | gtcacctggaaatgtgaacaaca | 5930 | 460965 | see also 5050 line | | |
| 5053 | KLF12 | NM_007249.3 | 932 | tgccaaatgtgaccttagatagc | 5933 | 460967 | see also 5050 line | | |

Figure 46

| 5054 | KLF12 | NM_007249.3 | 940 | gtgaccttagatagcgttaatga | 5934 | 460968 | see also 5050 line | | |
| 5055 | RPF-1 | NM_007252.1 | 1073 | gggggtcacaggtcaactagtta | 10818 | 442942 | transcription_regulator | - | - |
| 5056 | RPF-1 | NM_007252.1 | 1074 | ggggtcacaggtcaactagttac | 10819 | 442943 | see also 5055 line | | |
| 5057 | RPF-1 | NM_007252.1 | 1075 | gggtcacaggtcaactagttact | 10820 | 442944 | see also 5055 line | | |
| 5058 | RPF-1 | NM_007252.1 | 1079 | cacaggtcaactagttactaatg | 10821 | 442945 | see also 5055 line | | |
| 5059 | RPF-1 | NM_007252.1 | 1537 | tccgagaatttgccaaagctttt | 10823 | 442947 | see also 5055 line | | |
| 5060 | RPF-1 | NM_007252.1 | 1668 | ctggacatcacccctaaaagtgc | 10827 | 442949 | see also 5055 line | | |
| 5061 | RPF-1 | NM_007252.1 | 1931 | tagagtttggttctgcaataaga | 10836 | 442962 | see also 5055 line | | |
| 5062 | VPS45A | NM_007259.2 | 894 | ctgagattggtagcaatataaag | 271509 | 653152 | - | - | - |
| 5063 | HSGT1 | NM_007265.1 | 411 | aggaggtgttcctgctcatatgt | 111853 | 729544 | transcription_regulator | transcription co-activator | - |
| 5064 | HSGT1 | NM_007265.1 | 471 | atggtttattgtttatgtaataa | 111856 | 729550 | see also 5063 line | | |
| 5065 | HSGT1 | NM_007265.1 | 1143 | ctccaaatgtagcccacattttt | 111876 | 729570 | see also 5063 line | | |
| 5066 | HSGT1 | NM_007265.1 | 1149 | atgtagcccacatttttctgact | 111877 | 729572 | see also 5063 line | | |
| 5067 | STXBP3 | NM_007269.1 | 254 | accgtgaacctgtcagacaaatg | 271038 | 529996 | - | protein transporter | - |
| 5068 | STXBP3 | NM_007269.1 | 257 | gtgaacctgtcagacaaatgaaa | 271039 | 529998 | see also 5067 line | | |
| 5069 | STXBP3 | NM_007269.1 | 816 | ggcaatggcatatgatctactac | 271054 | 530002 | see also 5067 line | | |
| 5070 | STXBP3 | NM_007269.1 | 827 | atgatctactaccaattgagaat | 271056 | 530003 | see also 5067 line | | |
| 5071 | STK38 | NM_007271.2 | 307 | accttgctcatccatgagtaacc | 101801 | 515702 | kinase_related | - | - |
| 5072 | STK38 | NM_007271.2 | 383 | aaccttatcgctcaacatgaaga | 101806 | 515707 | see also 5071 line | | |
| 5073 | STK38 | NM_007271.2 | 691 | tgcggagcgtgacattctagtgg | 101812 | 515715 | see also 5071 line | | |
| 5074 | STK38 | NM_007271.2 | 845 | gaggagactcagttttatatagc | 101817 | 515718 | see also 5071 line | | |
| 5075 | STK38 | NM_007271.2 | 950 | agcaagggccatgtgaaactttc | 101818 | 515721 | see also 5071 line | | |
| 5076 | RNF13 | NM_007282.3 | 911 | tgcaagatttggttatagacttc | 191615 | 585151 | - | - | - |
| 5077 | BRCA1 | NM_007294.1 | 1247 | atgttccttggataacactaaat | 15947 | 456579 | transcription_regulator, apoptosis, cell_cycle, signal_transduction | - | breast cancer, Li-Fraumeni syndrome, ovarian cancer, non-small-cell lung cancer |
| 5078 | GRIN1 | NM_007327.1 | 3771 | ggcgtaggtcctccaaagacacg | 165848 | 563295 | receptor_acitivity, channel, signal_transduction | - | schizophrenia |
| 5079 | GRIN1 | NM_007327.1 | 3843 | tgccgcgacgcgctattgagagg | 165850 | 563296 | see also 5078 line | | |
| 5080 | TRPA1 | NM_007332.1 | 3102 | agctatgcaggtggaacttcata | 154202 | 544010 | channel | - | - |
| 5081 | TTF1 | NM_007344.2 | 2196 | aactctacaagggcatatcttgg | 337449 | 451388 | transcription_regulator | - | - |
| 5082 | PAXIP1L | NM_007349.2 | 3361 | cgctggactatgaatcatataag | 325589 | 690542 | - | - | - |

Figure 46

| | | | | | | | | | |
|---|---|---|---|---|---|---|---|---|---|
| 5083 | M96 | NM_007358.1 | 637 | ttgtgtcacacacctcatattga | 31512 | 461976 | transcription_regulator | DNA binding | - |
| 5084 | M96 | NM_007358.1 | 999 | cagttctggaccagaatacctca | 31520 | 461992 | see also 5083 line | | |
| 5085 | M96 | NM_007358.1 | 1049 | tagcacacctatgcctttacaac | 31524 | 461996 | see also 5083 line | | |
| 5086 | M96 | NM_007358.1 | 1060 | tgcctttacaacctaagtgttat | 31527 | 461998 | see also 5083 line | | |
| 5087 | M96 | NM_007358.1 | 1106 | ctgaacttgagcttatgacatac | 31530 | 461999 | see also 5083 line | | |
| 5088 | M96 | NM_007358.1 | 1114 | gagcttatgacatacattaatga | 31531 | 462002 | see also 5083 line | | |
| 5089 | M96 | NM_007358.1 | 1270 | ttgtttgggttgcgaattcgtgt | 31532 | 462008 | see also 5083 line | | |
| 5090 | MLN51 | NM_007359.3 | 1853 | cagtatcatggagggacattact | 282164 | 752231 | - | molecular_function unknown | - |
| 5091 | MLN51 | NM_007359.3 | 1887 | cagttccagggaccaatctatac | 282166 | 752232 | see also 5090 line | | |
| 5092 | NID2 | NM_007361.2 | 3944 | atccaatccgaggcaacttgtac | 127162 | 538028 | - | collagen binding | - |
| 5093 | NID2 | NM_007361.2 | 3945 | tccaatccgaggcaacttgtact | 127163 | 538029 | see also 5092 line | | |
| 5094 | RASA3 | NM_007368.2 | 1478 | gaccgtcatgtgtgacatcttct | 298219 | 665855 | - | GTPase activator | - |
| 5095 | DDX42 | NM_007372.1 | 857 | aagagtctcttaccttgtgtttg | 59206 | 483178 | - | - | - |
| 5096 | DDX42 | NM_007372.1 | 2008 | gagcccaaggagtcaacaacaca | 59236 | 483205 | see also 5095 line | | |
| 5097 | SHOC2 | NM_007373.1 | 794 | atgcttgatttacggcataataa | 284214 | 708879 | signal_transduction | molecular_function unknown | - |
| 5098 | SHOC2 | NM_007373.1 | 795 | tgcttgatttacggcataataaa | 284215 | 708880 | see also 5097 line | | |
| 5099 | SHOC2 | NM_007373.1 | 1004 | ctggatgtagctcacaatcaact | 284223 | 708885 | see also 5097 line | | |
| 5100 | SHOC2 | NM_007373.1 | 1477 | ttggaccagtatggtagaattga | 284240 | 708909 | see also 5097 line | | |
| 5101 | SHOC2 | NM_007373.1 | 1695 | tacagaaattagtcttgacaaac | 284246 | 708914 | see also 5097 line | | |
| 5102 | SHOC2 | NM_007373.1 | 1994 | aagatgcagggtccatatcgtgc | 284251 | 708917 | see also 5097 line | | |
| 5103 | MIP | NM_012064.1 | 556 | gggcacctctttgggatgtatta | 273968 | 508398 | - | Smad3/Sp-1 heterodimer | - |
| 5104 | ATF5 | NM_012068.2 | 937 | ccgaggggaccgcaagcaaaga | 21859 | 455344 | transcription_regulator , cell_cycle | translation regulator | - |
| 5105 | ATP1B4 | NM_012069.2 | 538 | ttcaacttcaacgtttctgaacc | 191991 | 585550 | - | sodium/potassium-exchanging ATPase | - |
| 5106 | ATRN | NM_012070.2 | 345 | aaggacagccaaatagaataatg | 173949 | 567972 | receptor_acitivity | - | - |
| 5107 | ATRN | NM_012070.2 | 2773 | gtgtatggaatggtatacgatga | 174021 | 568038 | see also 5106 line | | |
| 5108 | ATRN | NM_012070.2 | 3119 | cactgcgagacctgcatatctgg | 174030 | 568041 | see also 5106 line | | |
| 5109 | C10orf8 | NM_012071.1 | 294 | agcgaatagaactgttttgcacg | 337477 | 767006 | signal_transduction | - | - |
| 5110 | C10orf8 | NM_012071.1 | 536 | caggacttggtggggaaacttaa | 337490 | 767013 | see also 5109 line | | |
| 5111 | CRB1 | NM_012076.1 | 2184 | cagaggacgctgcatcaacttgt | 140907 | 546553 | - | calcium ion binding | amaurosis congenita of Leber、retinitis pigmentosa |
| 5112 | ZFPM2 | NM_012082.2 | 749 | cagctattgtcaataaggatata | 113403 | 526173 | - | - | - |
| 5113 | ZFPM2 | NM_012082.2 | 1676 | ttgagtgtaacataacattcaat | 113424 | 526188 | see also 5112 line | | |
| 5114 | ZFPM2 | NM_012082.2 | 2661 | tactgcacatcagcgtaatgacc | 113449 | 526224 | see also 5112 line | | |

Figure 46

| 5115 | APPL | NM_012096.1 | 106 | cccgcagacaaggtctttactag | 129011 | 755587 | kinase_related、signal _transduction | - | - |
|---|---|---|---|---|---|---|---|---|---|
| 5116 | APPL | NM_012096.1 | 184 | agctatgcatcggatttatgatg | 129019 | 755590 | see also 5115 line | | |
| 5117 | APPL | NM_012096.1 | 285 | gaggtgatgatgaagttatgagc | 129022 | 755593 | see also 5115 line | | |
| 5118 | APPL | NM_012096.1 | 672 | aagctcagataagtttctttaag | 129035 | 755609 | see also 5115 line | | |
| 5119 | APPL | NM_012096.1 | 1143 | atcatgaagagtggatctgtaca | 129053 | 755626 | see also 5115 line | | |
| 5120 | APPL | NM_012096.1 | 1482 | gaggcaggcgtacaaatccattt | 129063 | 755637 | see also 5115 line | | |
| 5121 | APPL | NM_012096.1 | 1483 | aggcaggcgtacaaatccatttg | 129064 | 755638 | see also 5115 line | | |
| 5122 | APPL | NM_012096.1 | 1484 | ggcaggcgtacaaatccatttgg | 129065 | 755639 | see also 5115 line | | |
| 5123 | APPL | NM_012096.1 | 1543 | ttctattcttcatcagttattta | 129067 | 755640 | see also 5115 line | | |
| 5124 | APPL | NM_012096.1 | 1853 | ctgtcatcagtctgctatatatt | 129076 | 755653 | see also 5115 line | | |
| 5125 | APPL | NM_012096.1 | 1858 | atcagtctgctatatatttgagt | 129077 | 755655 | see also 5115 line | | |
| 5126 | APPL | NM_012096.1 | 1949 | ctggatcgtagggcatcagaaaa | 129079 | 755659 | see also 5115 line | | |
| 5127 | ARL5 | NM_012097.2 | 543 | tactatactaacacagagtttgt | 108530 | 807605 | signal_transduction | - | - |
| 5128 | ARL5 | NM_012097.2 | 548 | tactaacacagagtttgtaatag | 108531 | 807606 | see also 5127 line | | |
| 5129 | ANGPTL2 | NM_012098.2 | 1911 | ttccgaggaggctcttactcact | 171174 | 685801 | - | receptor binding | Alzheimer disease |
| 5130 | ANGPTL2 | NM_012098.2 | 1919 | aggctcttactcactcaagaaag | 171175 | 685802 | see also 5129 line | | |
| 5131 | DNPEP | NM_012100.1 | 657 | tccatctgcagcgaaatatcaac | 186614 | 580444 | - | vacuolar aminopeptidase I | - |
| 5132 | AUP1 | NM_012103.2 | 154 | ggccggagcggctctttgactcg | 216116 | 603480 | - | - | - |
| 5133 | AUP1 | NM_012103.2 | 977 | ccctttcacggtgtatcaagtaa | 216128 | 603491 | see also 5132 line | | |
| 5134 | BACE | NM_012104.2 | 906 | gtcactgtgcgtgccaacattgc | 184961 | 579250 | - | - | Alzheimer disease |
| 5135 | BACE | NM_012104.2 | 1568 | cacgtcccaagacgactgttaca | 184971 | 579257 | see also 5134 line | | |
| 5136 | BACE | NM_012104.2 | 1571 | gtcccaagacgactgttacaagt | 184972 | 579258 | see also 5134 line | | |
| 5137 | CHIC2 | NM_012110.2 | 424 | tccggtcacgtcaccgtatttgg | 278676 | 736562 | - | - | - |
| 5138 | CHIC2 | NM_012110.2 | 651 | aacacgaagatcgattgagaagt | 278681 | 736569 | see also 5137 line | | |
| 5139 | CHIC2 | NM_012110.2 | 767 | atgtcatcctcatagaatttta | 278685 | 736574 | see also 5137 line | | |
| 5140 | AHSA1 | NM_012111.1 | 1081 | agggctggcagcggtactacttt | 337515 | 759648 | - | - | - |
| 5141 | AHSA1 | NM_012111.1 | 1082 | gggctggcagcggtactactttg | 337516 | 759649 | see also 5140 line | | |
| 5142 | AHSA1 | NM_012111.1 | 1083 | ggctggcagcggtactactttga | 337517 | 759650 | see also 5140 line | | |
| 5143 | CD2AP | NM_012120.1 | 380 | gcgtcagtgtaaagttcttttg | 286937 | 533336 | signal_transduction | - | - |
| 5144 | CD2AP | NM_012120.1 | 721 | ctgtgaaacttcggacaagaaca | 286942 | 533342 | see also 5143 line | | |
| 5145 | CD2AP | NM_012120.1 | 1808 | tagagcccagattattgaattgt | 286975 | 533363 | see also 5143 line | | |
| 5146 | CHORDC1 | NM_012124.1 | 381 | ccctaagccagtagaagcaataa | 278691 | 719563 | - | - | - |
| 5147 | DDAH1 | NM_012137.2 | 547 | ctgaaatcttggctgatactttt | 203785 | 595522 | signal_transduction | dimethylargininase | - |
| 5148 | DDX26 | NM_012141.1 | 1106 | gtgttctccaagaatgcttaatc | 58980 | 482925 | receptor_acitivity | - | non-small-cell lung cancer |
| 5149 | DDX26 | NM_012141.1 | 1109 | ttctccaagaatgcttaatcagt | 58981 | 482926 | see also 5148 line | | |

Figure 46

| 5150 | DDX26 | NM_012141.1 | 1140 | tccttggtgcagaaagtacaaag | 58984 | 482927 | see also 5148 line | | |
|------|-------|-------------|------|-------------------------|-------|--------|--------------------|---|---|
| 5151 | EHF | NM_012153.2 | 173 | aggtggtgtaatgaatctcaacc | 2979 | 443200 | - | - | - |
| 5152 | EPB41L1 | NM_012156.2 | 2523 | tccgatcatcgggaaagatgtcc | 117029 | 522610 | - | - | - |
| 5153 | EPB41L1 | NM_012156.2 | 2593 | accacccatgtcaccaaaactgt | 117030 | 522611 | see also 5152 line | | |
| 5154 | FBXL3A | NM_012158.1 | 584 | cagagctgatcaaacagattatt | 228309 | 706157 | - | - | - |
| 5155 | FBXL3A | NM_012158.1 | 1361 | cagaacgttgcaaaaatttgtca | 228346 | 706185 | see also 5154 line | | |
| 5156 | FBXL3A | NM_012158.1 | 1410 | ctcatgtagtgcctttgttgagt | 228348 | 706186 | see also 5154 line | | |
| 5157 | FBXL3A | NM_012158.1 | 1490 | ttcctgaccaaaagtatagtttg | 228352 | 706192 | see also 5154 line | | |
| 5158 | FBXL4 | NM_012160.3 | 384 | cagttctgaccatgttttattat | 324156 | 782018 | - | - | - |
| 5159 | FBXL4 | NM_012160.3 | 924 | gccagtttaaaccttgtattaag | 324184 | 782029 | see also 5158 line | | |
| 5160 | FBXL4 | NM_012160.3 | 1507 | ttgcagccactttcttaatgaaa | 324201 | 782044 | see also 5158 line | | |
| 5161 | FBXL4 | NM_012160.3 | 2048 | ctggacatattaggaacaagaat | 324220 | 782054 | see also 5158 line | | |
| 5162 | FBXL5 | NM_012161.2 | 176 | tactgcgacaagctttctaaaac | 228382 | 803640 | - | - | - |
| 5163 | FBXL5 | NM_012161.2 | 441 | aagagagattggaggcttttaca | 228386 | 803653 | see also 5162 line | | |
| 5164 | FBXL5 | NM_012161.2 | 1250 | cagagtcttcggcatcttgatct | 228408 | 803673 | see also 5162 line | | |
| 5165 | FBXW2 | NM_012164.2 | 333 | atggctcgatcctcagactttac | 228828 | 688783 | - | ubiquitin-protein ligase | - |
| 5166 | FBXW2 | NM_012164.2 | 334 | tggctcgatcctcagactttact | 228829 | 688784 | see also 5165 line | | |
| 5167 | FBXW2 | NM_012164.2 | 379 | cagtggaataaggtgataagtgc | 228832 | 688789 | see also 5165 line | | |
| 5168 | FBXW2 | NM_012164.2 | 412 | gtgtggcagactgcatgtaaaaa | 228833 | 688790 | see also 5165 line | | |
| 5169 | FBXW2 | NM_012164.2 | 414 | gtggcagactgcatgtaaaaatt | 228834 | 688791 | see also 5165 line | | |
| 5170 | FBXW2 | NM_012164.2 | 441 | ctggcagatagatgattctgttc | 228835 | 688793 | see also 5165 line | | |
| 5171 | FBXW2 | NM_012164.2 | 567 | tgccagagtgtatgcactttact | 228838 | 688798 | see also 5165 line | | |
| 5172 | FBXW2 | NM_012164.2 | 570 | cagagtgtatgcactttactaca | 228839 | 688799 | see also 5165 line | | |
| 5173 | FBXW2 | NM_012164.2 | 572 | gagtgtatgcacttttactacaaa | 228840 | 688800 | see also 5165 line | | |
| 5174 | FBXW2 | NM_012164.2 | 986 | ctggagactacatcctcttaagt | 228846 | 688807 | see also 5165 line | | |
| 5175 | FBXO22 | NM_012170.2 | 588 | tagcaatcgacctcaggaaatag | 337725 | 732191 | - | - | - |
| 5176 | FBXO22 | NM_012170.2 | 589 | agcaatcgacctcaggaaataga | 337726 | 732192 | see also 5175 line | | |
| 5177 | FBXO22 | NM_012170.2 | 593 | atcgacctcaggaaatagaaatt | 337727 | 732193 | see also 5175 line | | |
| 5178 | FBXO3 | NM_012175.2 | 358 | caggtgtcctcggatggttttat | 228598 | 710730 | - | - | - |
| 5179 | FBXO3 | NM_012175.2 | 359 | aggtgtcctcggatggttttatc | 228599 | 710731 | see also 5178 line | | |
| 5180 | FBXO7 | NM_012179.2 | 467 | ttggcttcatatgggattgtttc | 228708 | 768713 | - | ubiquitin-protein ligase | - |
| 5181 | FBXO7 | NM_012179.2 | 468 | tggcttcatatgggattgtttct | 228709 | 768714 | see also 5180 line | | |
| 5182 | FBXO8 | NM_012180.1 | 908 | gagagaatttttcgtcatatcc | 294965 | 670799 | - | guanyl-nucleotide release factor | - |
| 5183 | FBXO8 | NM_012180.1 | 1053 | ctgtgctactctttgattctact | 294969 | 670807 | see also 5182 line | | |

Figure 46

| 5184 | CABYR | NM_012189.2 | 2117 | cccagtagaagatgtagctaaaa | 298642 | 666171 | kinase_related、signal _transduction | - | - |
|---|---|---|---|---|---|---|---|---|---|
| 5185 | FZD4 | NM_012193.2 | 1140 | aaggatatcctgtgattttgaag | 242548 | 630620 | receptor_acitivity、gpc r、signal_transduction | frizzled receptor | - |
| 5186 | GAPCENA | NM_012197.2 | 3257 | accgaacactgagctccataaag | 120545 | 760448 | cell_cycle | - | - |
| 5187 | EIF2C1 | NM_012199.2 | 385 | gacatcaagccggataagtgtcc | 56437 | 769173 | - | translation initiation factor | Alzheimer disease |
| 5188 | EIF2C1 | NM_012199.2 | 2512 | tgggatgacaaccgtttcacagc | 56465 | 769198 | see also 5187 line | | |
| 5189 | B3GAT3 | NM_012200.2 | 243 | gaggccctgcctactatctatgt | 161744 | 561337 | - | manganese binding | - |
| 5190 | B3GAT3 | NM_012200.2 | 245 | ggccctgcctactatctatgttg | 161745 | 561338 | see also 5189 line | | |
| 5191 | GLG1 | NM_012201.1 | 684 | accaagatgacggccatcatttt | 171814 | 687819 | - | receptor binding | - |
| 5192 | GLG1 | NM_012201.1 | 1108 | ttgcccaggattataaagtcagt | 171828 | 687840 | see also 5191 line | | |
| 5193 | GLG1 | NM_012201.1 | 1514 | ccgcattgatcgagctttgaatg | 171840 | 687850 | see also 5191 line | | |
| 5194 | GLG1 | NM_012201.1 | 1515 | cgcattgatcgagctttgaatga | 171841 | 687851 | see also 5191 line | | |
| 5195 | GLG1 | NM_012201.1 | 2492 | agcctgcaagagtgacatcaaaa | 171861 | 687881 | see also 5191 line | | |
| 5196 | GLG1 | NM_012201.1 | 3341 | gtgcaaaaagcgcctcaatgacc | 171884 | 687896 | see also 5191 line | | |
| 5197 | GRHPR | NM_012203.1 | 560 | accattcggtgtccagagatttc | 220924 | 607913 | - | - | - |
| 5198 | ITGA11 | NM_012211.2 | 724 | gactacaggtctgtaaaagatgt | 126958 | 682940 | receptor_acitivity、sig nal_transduction | magnesium binding | - |
| 5199 | ITGA11 | NM_012211.2 | 979 | tacaaccgcagggggatcaatcc | 126962 | 682942 | see also 5198 line | | |
| 5200 | LTB4DH | NM_012212.2 | 590 | aagctcaagggctgcaaagttgt | 157566 | 558150 | - | zinc binding | - |
| 5201 | LTB4DH | NM_012212.2 | 753 | atgtaggtggagagtttcaaac | 157575 | 558158 | see also 5200 line | | |
| 5202 | MGAT4A | NM_012214.1 | 240 | tgcttatcaacgagaattccttg | 210456 | 600731 | - | - | - |
| 5203 | MGEA5 | NM_012215.1 | 713 | ttggcgagagatgtattcagtgg | 200800 | 714408 | - | - | - |
| 5204 | MGEA5 | NM_012215.1 | 762 | atctctgctgcacgagaatatga | 200802 | 714409 | see also 5203 line | | |
| 5205 | MGEA5 | NM_012215.1 | 764 | ctctgctgcacgagaatatgaga | 200803 | 714410 | see also 5203 line | | |
| 5206 | MGEA5 | NM_012215.1 | 1227 | tgggataacattcatgctaatga | 200827 | 714429 | see also 5203 line | | |
| 5207 | MGEA5 | NM_012215.1 | 2463 | cagcgtttgctgccaattgatgg | 200859 | 714455 | see also 5203 line | | |
| 5208 | MGEA5 | NM_012215.1 | 2529 | tccaaagtttatactatcagacc | 200861 | 714456 | see also 5203 line | | |
| 5209 | MID2 | NM_012216.2 | 381 | tgcagctctggtgaatccattga | 157876 | 558419 | - | - | - |
| 5210 | MID2 | NM_012216.2 | 807 | aacatgtactgtgtatctgatga | 157882 | 558426 | see also 5209 line | | |
| 5211 | MID2 | NM_012216.2 | 834 | ttgatctgtgccttatgcaaact | 157884 | 558428 | see also 5209 line | | |
| 5212 | MID2 | NM_012216.2 | 1302 | ctgattccagacatcaattttaa | 157900 | 558437 | see also 5209 line | | |
| 5213 | MID2 | NM_012216.2 | 1522 | ctggccaggctaacttcatcagt | 157909 | 558441 | see also 5209 line | | |
| 5214 | SIRT1 | NM_012238.3 | 592 | cacggataggtccatatactttt | 39382 | 467084 | apoptosis、transcriptio n_regulator | - | - |

Figure 46

| 5215 | SIRT1 | NM_012238.3 | 593 | acggataggtccatatacttttg | 39383 | 467085 | see also 5214 line | | |
|------|-------|-------------|-----|-------------------------|-------|--------|--------------------|--|--|
| 5216 | SIRT1 | NM_012238.3 | 594 | cggataggtccatatactttgt | 39384 | 467086 | see also 5214 line | | |
| 5217 | SIRT1 | NM_012238.3 | 640 | cagatcctcgaacaattcttaaa | 39388 | 467091 | see also 5214 line | | |
| 5218 | SIRT1 | NM_012238.3 | 711 | ctgtggcagattgttattaatat | 39393 | 467094 | see also 5214 line | | |
| 5219 | DKK1 | NM_012242.1 | 755 | tggtccaagatctgtaaacctgt | 169008 | 685372 | signal_transduction | growth factor | - |
| 5220 | SLC35A3 | NM_012243.1 | 949 | aggcacttggaggccttgtaata | 259264 | 654672 | - | sugar porter | - |
| 5221 | SLC7A8 | NM_012244.2 | 819 | gggcggagtagccctgaagaaag | 259454 | 641531 | - | - | - |
| 5222 | SEPHS2 | NM_012248.2 | 1283 | aacctctgggggattactgattt | 100276 | 514585 | kinase_related | selenium binding | - |
| 5223 | SEPHS2 | NM_012248.2 | 1284 | acctctgggggattactgatttg | 100277 | 514586 | see also 5222 line | | |
| 5224 | XRN2 | NM_012255.2 | 196 | tgccagtaaacctaatccaaatg | 55780 | 703542 | - | - | - |
| 5225 | XRN2 | NM_012255.2 | 221 | gtggagtttgataatctgtattt | 55782 | 703544 | see also 5224 line | | |
| 5226 | XRN2 | NM_012255.2 | 309 | atgaaatgatggttgcaattttt | 55784 | 703548 | see also 5224 line | | |
| 5227 | XRN2 | NM_012255.2 | 595 | tgctaaatgccttcgctattaca | 55794 | 703551 | see also 5224 line | | |
| 5228 | XRN2 | NM_012255.2 | 614 | tacatagctgatcgtttaaataa | 55795 | 703552 | see also 5224 line | | |
| 5229 | XRN2 | NM_012255.2 | 619 | agctgatcgtttaaataatgacc | 55796 | 703553 | see also 5224 line | | |
| 5230 | XRN2 | NM_012255.2 | 1167 | accgtttggttaacatatacaaa | 55813 | 703571 | see also 5224 line | | |
| 5231 | XRN2 | NM_012255.2 | 1246 | aagagtacagatgatcatgttag | 55816 | 703573 | see also 5224 line | | |
| 5232 | XRN2 | NM_012255.2 | 1404 | ctcatgccttgggttcaagaaat | 55820 | 703574 | see also 5224 line | | |
| 5233 | HBP1 | NM_012257.2 | 612 | tagcaaaagtttacattcctatg | 27938 | 459928 | transcription_regulator | - | - |
| 5234 | HBP1 | NM_012257.2 | 960 | ttctgatggtctaaagttgttat | 27944 | 459935 | see also 5233 line | | |
| 5235 | HBP1 | NM_012257.2 | 1007 | gcgagtctgtactgaagttgact | 27946 | 459937 | see also 5233 line | | |
| 5236 | HBP1 | NM_012257.2 | 1081 | gaccaccctttctatgttaaaaa | 27949 | 459939 | see also 5233 line | | |
| 5237 | HBP1 | NM_012257.2 | 1464 | gagtgccacttctcctaataagt | 27963 | 459951 | see also 5233 line | | |
| 5238 | HBP1 | NM_012257.2 | 1466 | gtgccacttctcctaataagtgc | 27964 | 459952 | see also 5233 line | | |
| 5239 | HEY1 | NM_012258.2 | 181 | tgcggacgagaatggaaacttga | 4736 | 459973 | transcription_regulator | transcription factor | - |
| 5240 | HEY2 | NM_012259.1 | 105 | gacgtggggagcgagaacaatta | 4753 | 459983 | transcription_regulator | - | - |
| 5241 | HPCL2 | NM_012260.1 | 277 | gagcaagcggcttgttatgctgc | 166019 | 562669 | - | magnesium binding | - |
| 5242 | HS2ST1 | NM_012262.2 | 418 | cgcggtggcgatgctcttcttgg | 218769 | 605372 | - | - | - |
| 5243 | HS2ST1 | NM_012262.2 | 639 | atgacctgtgtgcaaagaataaa | 218773 | 605380 | see also 5242 line | | |
| 5244 | DNAJB5 | NM_012266.2 | 535 | gtggctccaaccccttcgatatc | 299055 | 668489 | - | heat shock protein | - |
| 5245 | DNAJB5 | NM_012266.2 | 539 | ctccaaccccttcgatatcttct | 299056 | 668490 | see also 5244 line | | |
| 5246 | PLD3 | NM_012268.1 | 1563 | cccgtgtcaaccacaacaagtac | 197518 | 622520 | - | - | - |
| 5247 | HYPC | NM_012272.1 | 2251 | aggacttcgcccacgtcataagc | 337937 | 707413 | - | - | - |
| 5248 | HYPC | NM_012272.1 | 2256 | ttcgcccacgtcataagctttga | 337938 | 707414 | see also 5247 line | | |

Figure 46

| ID | Gene | Accession | Pos | Sequence | | | Description | Function | Note |
|---|---|---|---|---|---|---|---|---|---|
| 5249 | HYPC | NM_012272.1 | 2260 | cccacgtcataagctttgacaag | 337939 | 707415 | see also 5247 line | | |
| 5250 | HYPC | NM_012272.1 | 2293 | cactggacgcaggcaaacatcaag | 337940 | 707416 | see also 5247 line | | |
| 5251 | HYPC | NM_012272.1 | 2586 | cacaccaaaggcgaaagcatgg | 337942 | 707419 | see also 5247 line | | |
| 5252 | HYPC | NM_012272.1 | 2751 | tcctcacttgattcagttgaaag | 337943 | 707421 | see also 5247 line | | |
| 5253 | ITGB1BP2 | NM_012278.1 | 153 | tgctgccgaaagcgaacgtgtaga | 131991 | 704870 | signal_transduction | protein binding | - |
| 5254 | JAZ | NM_012279.2 | 347 | aagagataccttagccaatccatgg | 48456 | 472408 | apoptosis | - | |
| 5255 | KCND2 | NM_012281.2 | 2707 | aagagtgttaaaactaaactgt | 132526 | 536452 | channel | - | |
| 5256 | TRAM2 | NM_012288.1 | 1158 | cccacgactaagaaactcaagt | 302604 | 747263 | - | - | |
| 5257 | TLK1 | NM_012290.3 | 471 | tagcgactattttgaataccagg | 102664 | 516487 | kinase_related, cell_cycle | - | |
| 5258 | TLK1 | NM_012290.3 | 1619 | tggatacagatacgttttgtaca | 102698 | 516535 | see also 5257 line | | |
| 5259 | TLK1 | NM_012290.3 | 1916 | cccagggggcaggcaacttactgg | 102712 | 516547 | see also 5257 line | | |
| 5260 | TLK1 | NM_012290.3 | 1922 | gggcaggcacttactggtattta | 102713 | 516548 | see also 5257 line | | |
| 5261 | TLK1 | NM_012290.3 | 2310 | ttcttcaagcataattacttact | 102723 | 516556 | see also 5257 line | | |
| 5262 | GFR | NM_012294.1 | 145 | taccttcgatgtgccttatttca | 128296 | 664752 | signal_transduction | - | |
| 5263 | GFR | NM_012294.1 | 149 | ttcgatgtgccttatttcaaata | 128297 | 664753 | see also 5262 line | | |
| 5264 | G3BP2 | NM_012297.2 | 908 | ctggaattccaccccatgttaaa | 51883 | 473035 | - | receptor signaling complex scaffold protein | - |
| 5265 | G3BP2 | NM_012297.2 | 1193 | tggaacttcgcatcaataccaag | 51893 | 473039 | see also 5264 line | | |
| 5266 | G3BP2 | NM_012297.2 | 1422 | cggttgttccacgtggaattgtgg | 51897 | 473047 | see also 5264 line | - | |
| 5267 | FBXW1B | NM_012300.1 | 808 | gcgtgttggacgacacaaactgc | 228800 | 750217 | - | - | |
| 5268 | FBXW1B | NM_012300.1 | 839 | cagtgccgctgaaaatagtaa | 228801 | 750218 | see also 5267 line | | |
| 5269 | EPB41L3 | NM_012307.2 | 1114 | aacggaacaacttttacattag | 117128 | 522684 | - | structural molecule | non-small-cell lung cancer |
| 5270 | EPB41L3 | NM_012307.2 | 1337 | aagagccagtgcgttgatagatc | 117142 | 522687 | see also 5269 line | - | |
| 5271 | EPB41L3 | NM_012307.2 | 1435 | ctggccagtacgccacaacaaaa | 117146 | 522693 | see also 5269 line | - | |
| 5272 | KPNA6 | NM_012316.3 | 110 | atcgaatgaagagagctataagaac | 269399 | 651454 | - | - | |
| 5273 | KPNA6 | NM_012316.3 | 1353 | atgtgacttgctgactgtaatgg | 269424 | 651483 | see also 5272 line | - | |
| 5274 | LETM1 | NM_012318.1 | 1205 | aggaaaatcatgcgtttttccaaa | 147306 | 549276 | signal_transduction | - | |
| 5275 | MAPK8IP2 | NM_012324.2 | 191 | aggacataagcctggaagaattt | 120656 | 525247 | kinase_related, apoptosis | - | |
| 5276 | MAPK8IP2 | NM_012324.2 | 193 | gacataagcctggaagaatttga | 120657 | 525248 | see also 5275 line | | |
| 5277 | MAPK8IP2 | NM_012324.2 | 1175 | acctggtgagccgcatgatctcc | 120660 | 525257 | see also 5275 line | | |
| 5278 | MAPRE3 | NM_012326.2 | 196 | ctgagtcgccatgatatgcttgc | 120310 | 525103 | - | microtubule binding | - |
| 5279 | PIGN | NM_012327.3 | 828 | ctggatacgtgggtttttgataa | 338039 | 596150 | - | - | |
| 5280 | MMD | NM_012329.1 | 180 | cacacacgcattcctcattgttc | 250020 | 677605 | receptor_acitivity | receptor | - |

Figure 46

| | | | | | | | | | |
|---|---|---|---|---|---|---|---|---|---|
| 5281 | MMD | NM_012329.1 | 456 | atctcatatgcgttggtttatct | 250026 | 677614 | see also 5280 line | | |
| 5282 | MMD | NM_012329.1 | 489 | agctggaggaaccatttatgtat | 250027 | 677616 | see also 5280 line | | |
| 5283 | MYST4 | NM_012330.1 | 953 | ttccaatatgtagcttctgtttg | 33257 | 462177 | transcription_regulator | - | - |
| 5284 | MYST4 | NM_012330.1 | 1335 | acccattggacgaccgaaaaata | 33264 | 462187 | see also 5283 line | | |
| 5285 | MYST4 | NM_012330.1 | 1336 | cccattggacgaccgaaaaataa | 33265 | 462188 | see also 5283 line | | |
| 5286 | MYST4 | NM_012330.1 | 1343 | gacgaccgaaaaataaattaaag | 33266 | 462191 | see also 5283 line | | |
| 5287 | MYST4 | NM_012330.1 | 1375 | ttgtctgtaaccagtgatgaagg | 33267 | 462194 | see also 5283 line | | |
| 5288 | MYST4 | NM_012330.1 | 2533 | caggaatatgcaagattaccaaa | 33299 | 462204 | see also 5283 line | | |
| 5289 | MYST4 | NM_012330.1 | 2540 | atgcaagattaccaaagctttac | 33301 | 462205 | see also 5283 line | | |
| 5290 | MYST4 | NM_012330.1 | 2541 | tgcaagattaccaaagctttacc | 33302 | 462206 | see also 5283 line | | |
| 5291 | MYST4 | NM_012330.1 | 5771 | aactgcagcagttaactaataca | 33349 | 462247 | see also 5283 line | | |
| 5292 | MYST4 | NM_012330.1 | 5784 | aactaatacacttattgatcatt | 33352 | 462249 | see also 5283 line | | |
| 5293 | MYST4 | NM_012330.1 | 5843 | atgcaaacagtgcctctttgtcc | 33353 | 462251 | see also 5283 line | | |
| 5294 | MYCBP | NM_012333.2 | 129 | ttggtagccttatatgaagaacc | 111921 | 669153 | transcription_regulator | transcription co-activator | - |
| 5295 | MYO10 | NM_012334.1 | 2261 | aggacttttacaaaaggtataaa | 92564 | 509230 | signal_transduction | motor | - |
| 5296 | MYO10 | NM_012334.1 | 5273 | ttgtgccttcccgagatgaaata | 92609 | 509281 | see also 5295 line | | |
| 5297 | NARF | NM_012336.2 | 488 | gtcttggggtgcactatgtattt | 127828 | 723273 | - | - | - |
| 5298 | TM4SF12 | NM_012338.2 | 801 | tgggctctgtattatgatagaag | 327690 | 782252 | - | - | - |
| 5299 | GTPBP4 | NM_012341.1 | 339 | gtggacaatgttgctaaagatta | 109304 | 519583 | - | GTP binding | - |
| 5300 | GTPBP4 | NM_012341.1 | 340 | tggacaatgttgctaaagattat | 109305 | 519584 | see also 5299 line | | |
| 5301 | GTPBP4 | NM_012341.1 | 640 | ttgttgggcacatggattataag | 109311 | 519599 | see also 5299 line | | |
| 5302 | NNT | NM_012343.2 | 2930 | tggaaatttctggcacacatacg | 223124 | 610430 | - | electron transporter | - |
| 5303 | NNT | NM_012343.2 | 3123 | ccgaatgcctggtcagcttaatg | 223137 | 610433 | see also 5302 line | | |
| 5304 | OSTF1 | NM_012383.3 | 172 | cagtcaaaccagggcaagttaaa | 134462 | 540375 | signal_transduction | protein binding | - |
| 5305 | OSTF1 | NM_012383.3 | 178 | aaccagggcaagttaaagtcttc | 134463 | 540376 | see also 5304 line | | |
| 5306 | SPDEF | NM_012391.1 | 1333 | ccgctccatccgccagtattaca | 11879 | 448640 | transcription_regulator | molecular_function unknown | - |
| 5307 | SPDEF | NM_012391.1 | 1336 | ctccatccgccagtattacaaga | 11880 | 448641 | see also 5306 line | | |
| 5308 | PEF | NM_012392.1 | 599 | accgctcgggctccattagctac | 150607 | 542883 | - | - | - |
| 5309 | PFTK1 | NM_012395.2 | 826 | tacatccaccagcgttatatttt | 96662 | 511830 | kinase_related | pepsin A | - |
| 5310 | ARFIP2 | NM_012402.2 | 257 | gtcaggacccaacctcaatgaaa | 108301 | 740618 | signal_transduction | protein binding | - |
| 5311 | PRKCABP | NM_012407.1 | 366 | ctctatatcgtccaggtatttga | 126303 | 529561 | channel, kinase_related | - | - |
| 5312 | PTPN22 | NM_012411.2 | 376 | acccagggtcctttatctacaac | 193598 | 587156 | phosphatase | - | - |
| 5313 | PTPN22 | NM_012411.2 | 669 | ttcatctatagaccctattcttg | 193609 | 587166 | see also 5312 line | | |

Figure 46

| | | | | | | | | | |
|---|---|---|---|---|---|---|---|---|---|
| 5314 | SEC22L2 | NM_012430.2 | 330 | ttcagaaggagttcattactact | 274134 | 747454 | - | - | - |
| 5315 | SEC22L2 | NM_012430.2 | 335 | aaggagttcattactacttataa | 274135 | 747456 | see also 5314 line | | |
| 5316 | SEC22L2 | NM_012430.2 | 421 | gaggaccaagcagcgatataata | 274139 | 747457 | see also 5314 line | | |
| 5317 | SEC22L2 | NM_012430.2 | 422 | aggaccaagcagcgatataataa | 274140 | 747458 | see also 5314 line | | |
| 5318 | SEC22L2 | NM_012430.2 | 424 | gaccaagcagcgatataataatc | 274141 | 747459 | see also 5314 line | | |
| 5319 | SEC22L2 | NM_012430.2 | 773 | tgcctttaccagtgttatttact | 274151 | 747472 | see also 5314 line | | |
| 5320 | SEMA3E | NM_012431.1 | 1844 | gtgctgaaagtaatcacaattta | 312782 | 684588 | - | - | - |
| 5321 | SETDB1 | NM_012432.1 | 2058 | ttctgtttggatccatatgttct | 39066 | 466799 | - | - | - |
| 5322 | SETDB1 | NM_012432.1 | 3576 | acccgaggctttgctcttaaatc | 39094 | 466834 | see also 5321 line | | |
| 5323 | SETDB1 | NM_012432.1 | 3577 | cccgaggctttgctcttaaatca | 39095 | 466835 | see also 5321 line | | |
| 5324 | SETDB1 | NM_012432.1 | 3699 | gagtcttgctacatcattgatgc | 39102 | 466842 | see also 5321 line | | |
| 5325 | SF3B1 | NM_012433.1 | 36 | agcacagattcgagaaattcaag | 46833 | 670305 | - | pre-mRNA splicing factor | - |
| 5326 | SF3B1 | NM_012433.1 | 361 | aagcataggcggaccatgataat | 46849 | 670322 | see also 5325 line | | |
| 5327 | SF3B1 | NM_012433.1 | 387 | cccagagcgtcttgatccttttg | 46851 | 670323 | see also 5325 line | | |
| 5328 | SF3B1 | NM_012433.1 | 1834 | accatgagacctgatatagataa | 46890 | 670370 | see also 5325 line | | |
| 5329 | SF3B1 | NM_012433.1 | 1984 | cacactggtattaagattgtaca | 46897 | 670376 | see also 5325 line | | |
| 5330 | SF3B1 | NM_012433.1 | 2141 | aagcagcaactccttatggtatc | 46902 | 670380 | see also 5325 line | | |
| 5331 | SF3B1 | NM_012433.1 | 2397 | tggtgtagaagcaaactacatta | 46905 | 670390 | see also 5325 line | | |
| 5332 | SF3B1 | NM_012433.1 | 2763 | tggcaaacgagtcaaaccatact | 46918 | 670409 | see also 5325 line | | |
| 5333 | SF3B1 | NM_012433.1 | 3126 | tgctgacaggggagctgaatatg | 46928 | 670419 | see also 5325 line | | |
| 5334 | SSBP2 | NM_012446.2 | 619 | gcccccattgaggatacctaatc | 19868 | 714765 | transcription_regulator | - | - |
| 5335 | STAG3 | NM_012447.1 | 2633 | caggagcaggcttttgtcttatt | 308054 | 674579 | cell_cycle | - | - |
| 5336 | STAT5B | NM_012448.2 | 1914 | taccaggacggaattacactttc | 12239 | 448971 | transcription_regulator | translation regulator | - |
| 5337 | STAT5B | NM_012448.2 | 1915 | accaggacggaattacactttct | 12240 | 448972 | see also 5336 line | | |
| 5338 | TIMM13 | NM_012458.2 | 610 | agcgggaacgagccaacatgtga | 160027 | 559693 | - | zinc binding | - |
| 5339 | TLL1 | NM_012464.3 | 728 | ctgcgctggcctcgattatgatt | 153928 | 554595 | - | - | - |
| 5340 | TLL1 | NM_012464.3 | 729 | tgcgctggcctcgattatgatta | 153929 | 554596 | see also 5339 line | | |
| 5341 | TLL1 | NM_012464.3 | 1585 | ttctcccctcccgtgatgataat | 153956 | 554616 | see also 5339 line | | |
| 5342 | TLL1 | NM_012464.3 | 1587 | ctcccctcccgtgatgataatgg | 153957 | 554617 | see also 5339 line | | |
| 5343 | TLL2 | NM_012465.2 | 2229 | aaggcaatgacgtctgtaagtac | 154044 | 554681 | - | - | - |
| 5344 | TLL2 | NM_012465.2 | 2230 | aggcaatgacgtctgtaagtacg | 154045 | 554682 | see also 5343 line | | |
| 5345 | TRPC5 | NM_012471.1 | 2329 | atgtggcacccgactctgattgc | 261791 | 645539 | channel | store-operated calcium channel | - |
| 5346 | TRPC5 | NM_012471.1 | 2351 | cggaagcactcttcgcaatatcc | 261792 | 645540 | see also 5345 line | | |

Figure 46

| | | | | | | | | |
|---|---|---|---|---|---|---|---|---|
| 5347 | TRPC5 | NM_012471.1 | 3806 | atggacaggaggaacaagttaca | 261829 | 645592 | see also 5345 line | |
| 5348 | TSLRP | NM_012472.3 | 84 | atcacagaagatcttattagacg | 338239 | 708146 | - | |
| 5349 | USP21 | NM_012475.2 | 1257 | ctccacgacttcgaggttttt | 189181 | 583204 | - | |
| 5350 | USP21 | NM_012475.2 | 1258 | tccacgacttcgaggtttttg | 189182 | 583205 | see also 5349 line | |
| 5351 | USP21 | NM_012475.2 | 1263 | gacttcgaggttttttgtgacc | 189183 | 583206 | see also 5349 line | |
| 5352 | USP21 | NM_012475.2 | 1397 | gtgaccgatgtcggcagaaaact | 189186 | 583207 | see also 5349 line | |
| 5353 | USP21 | NM_012475.2 | 1418 | ctcgaagtaccaaaaagttgaca | 189189 | 583209 | see also 5349 line | |
| 5354 | WBP2 | NM_012478.2 | 507 | gccagtcgccaatggaatgtacc | 338291 | 542289 | - | |
| 5355 | E46L | NM_013236.1 | 779 | aggagaacctcaatattgcaatt | 279575 | 667669 | - | |
| 5356 | E46L | NM_013236.1 | 1322 | ttgtctgattggaaatctgtgt | 279590 | 667690 | see also 5355 line | |
| 5357 | GTL3 | NM_013242.1 | 636 | acggctggaaccagattcagttc | 320689 | 696877 | - | |
| 5358 | GTL3 | NM_013242.1 | 712 | cagagtgcagatccatgcaaatt | 320694 | 696879 | see also 5357 line | |
| 5359 | SCG3 | NM_013243.2 | 1178 | atggcttggaaaggaggaactaaa | 338395 | 698633 | receptor_actitivy, signal_transduction | |
| 5360 | HGNT-IV-H | NM_013244.2 | 1355 | tggctacattggtaaactctatc | 212131 | 600569 | - | |
| 5361 | HGNT-IV-H | NM_013244.2 | 1438 | gtgattggctattgactcattc | 212136 | 600576 | see also 5360 line | |
| 5362 | HGNT-IV-H | NM_013244.2 | 1656 | agcaaggcttacagtagtgttga | 212141 | 600585 | see also 5360 line | |
| 5363 | VPS4A | NM_013245.2 | 333 | aagctgaaggattatttacgaag | 107393 | 517538 | - | |
| 5364 | VPS4A | NM_013245.2 | 334 | agctgaaggattatttacgaagc | 107394 | 517539 | see also 5363 line | |
| 5365 | CLC | NM_013246.1 | 61 | ggggactcgtggggatgttagc | 168489 | 565446 | signal_transduction | |
| 5366 | PRSS25 | NM_013247.3 | 1355 | ccggcaagggagtttgttgttg | 182570 | 575947 | apoptosis | |
| 5367 | PRSS25 | NM_013247.3 | 1396 | cactgcagaacacgatcacatcc | 182572 | 575948 | see also 5366 line | |
| 5368 | MKLN1 | NM_013255.2 | 112 | taccttccgagaacatttagt | 338409 | 705070 | - | |
| 5369 | MKLN1 | NM_013255.2 | 401 | tcccttgcattcattaaaata | 338418 | 705085 | see also 5368 line | |
| 5370 | MKLN1 | NM_013255.2 | 725 | atgatggcttgttcaatcagtat | 338430 | 705099 | see also 5368 line | |
| 5371 | MKLN1 | NM_013255.2 | 729 | tggcttgttcaatcagtatatca | 338431 | 705101 | see also 5368 line | |
| 5372 | MKLN1 | NM_013255.2 | 733 | ttgttcaatcagtatatcagtca | 338432 | 705102 | see also 5368 line | |
| 5373 | MKLN1 | NM_013255.2 | 1211 | cagaaaaacatatgatctacaact | 338457 | 705121 | see also 5368 line | |
| 5374 | MKLN1 | NM_013255.2 | 1922 | ggcattgcaagtacctcattaaga | 338475 | 705142 | see also 5368 line | |
| 5375 | MKLN1 | NM_013255.2 | 2158 | gacaccttagttaatttctttcc | 338481 | 705147 | see also 5368 line | |
| 5376 | SGKL | NM_013257.3 | 907 | aacaactgaaaagctttattttg | 100346 | 514655 | kinase_related | |
| 5377 | SGKL | NM_013257.3 | 1300 | ttgccgagattgttgctgaatgt | 100356 | 514661 | see also 5376 line | |
| 5378 | PPARGC1 | NM_013261.2 | 927 | cccaaggttcccatttgaag | 36612 | 698438 | transcription_regulator | lipodystrophy |
| 5379 | PPARGC1 | NM_013261.2 | 1330 | ctccaagactctagacaactaga | 36622 | 698451 | see also 5378 line | |

Figure 46

| ID | Gene | Accession | Pos | Sequence | Num1 | Num2 | Annotation | Function |
|---|---|---|---|---|---|---|---|---|
| 5380 | PPARGC1 | NM_013261.2 | 1849 | tcgtgttcccgatcaccatattc | 36633 | 698463 | see also 5378 line | - |
| 5381 | BRD7 | NM_013263.1 | 523 | aacacccaatgatttttagttacc | 323755 | 704113 | - | - |
| 5382 | DDX25 | NM_013264.2 | 1137 | agcgagcttccatcattcagagg | 50779 | 473564 | - | - |
| 5383 | SLCO3A1 | NM_013272.2 | 2180 | ctgagcaacagtgagttctttgc | 274591 | 657330 | - | receptor signaling protein |
| 5384 | FLRT3 | NM_013281.2 | 285 | atcttcctcatcgggactaaaat | 146200 | 548749 | - | - |
| 5385 | FLRT3 | NM_013281.2 | 1135 | ttgatgatttggacaatataaca | 146228 | 548795 | see also 5384 line | - |
| 5386 | HUMAUANTIG | NM_013285.1 | 1017 | ttgggttcattggctatccaaat | 109383 | 757283 | - | GTPase |
| 5387 | HUMAUANTIG | NM_013285.1 | 1018 | tgggttcattggctatccaaatg | 109384 | 757284 | see also 5386 line | - |
| 5388 | TRA2A | NM_013293.2 | 224 | gggaactcctgctcgtgtaaat | 47370 | 811655 | - | pre-mRNA splicing factor |
| 5389 | LGN | NM_013296.3 | 921 | aacttcaggatgcagttatagc | 297951 | 665162 | signal_transduction, gpcr | GTPase activator |
| 5390 | LGN | NM_013296.3 | 924 | ttcaggatgcagttatagctca | 297952 | 665163 | see also 5389 line | - |
| 5391 | SLC30A4 | NM_013309.3 | 326 | gcgcgttggaagcgcctcaaatct | 266400 | 653931 | - | zinc ion transporter |
| 5392 | SLC30A4 | NM_013309.3 | 1181 | tagctgcatacatcatacgattc | 266420 | 653955 | see also 5391 line | - |
| 5393 | SLC30A4 | NM_013309.3 | 1182 | agctgcatacatcatacgattca | 266421 | 653956 | see also 5391 line | - |
| 5394 | SLC30A4 | NM_013309.3 | 1185 | tgcatacatacgattcaagc | 266422 | 653957 | see also 5391 line | - |
| 5395 | BLNK | NM_013314.2 | 776 | aagctcccatggtgaatagatca | 252563 | 680731 | kinase_related | - |
| 5396 | CNOT4 | NM_013316.1 | 1043 | atctacggttcagttgataaaa | 58667 | 481957 | - | - |
| 5397 | CNOT4 | NM_013316.1 | 1213 | cagtcatcccatcagttcatcc | 58671 | 481965 | see also 5396 line | - |
| 5398 | CNOT4 | NM_013316.1 | 1454 | ggctttggttcttctaaacaacc | 58677 | 481972 | see also 5396 line | - |
| 5399 | APG4B | NM_013325.3 | 1071 | aggtgccctgcccatgtttgagc | 338715 | 784968 | - | - |
| 5400 | P5CR2 | NM_013328.2 | 683 | cgggcctgcctatgcattcatgg | 225713 | 612587 | - | - |
| 5401 | C21orf66 | NM_013329.2 | 1828 | agcatggcgttcaaaatactaca | 23446 | 452957 | transcription_regulator | - |
| 5402 | GMPPB | NM_013334.2 | 829 | gccaaggagggggcagctatatgc | 209209 | 598682 | - | - |
| 5403 | GMPPA | NM_013335.2 | 522 | tacggctgcatcgttgaggaatcc | 209198 | 747483 | - | - |
| 5404 | ALG5 | NM_013338.3 | 808 | acgatgggcatttgattgtagaac | 211344 | 600150 | - | - |
| 5405 | SART2 | NM_013352.1 | 2124 | agggcagcttacctcttcatagg | 323344 | 774903 | - | - |
| 5406 | TMOD4 | NM_013353.1 | 557 | gtggtacagcctgacaagtataa | 119723 | 524752 | - | - |
| 5407 | TMOD4 | NM_013353.1 | 558 | tggtacagcctgacaagtataag | 119724 | 524753 | see also 5406 line | - |
| 5408 | PADI1 | NM_013358.1 | 1242 | ctgggtcctgactttggatatgt | 149327 | 550884 | - | protein-arginine deiminase |
| 5409 | PCOLCE2 | NM_013363.2 | 1194 | acgccacagtctgatcatcaac | 95940 | 739904 | - | - |
| 5410 | ANAPC4 | NM_013367.1 | 537 | aggaagctctggatttattgagc | 227521 | 714938 | cell_cycle | - |

277

Figure 46

| ID | Gene | Accession | Pos | Sequence | Num1 | Num2 | Function | Annotation | Disease |
|---|---|---|---|---|---|---|---|---|---|
| 5411 | ANAPC4 | NM_013367.1 | 816 | gtgtgaagcatgggaagaaatac | 227531 | 714953 | see also 5410 line | | |
| 5412 | ANAPC4 | NM_013367.1 | 1372 | gaggctccagacccttataatcg | 227552 | 714967 | see also 5410 line | | |
| 5413 | ABT1 | NM_013375.2 | 433 | gcgcagcccttccgttatgatc | 111413 | 657898 | transcription_regulator | - | |
| 5414 | TRHDE | NM_013381.1 | 1398 | caccatgtcattgttcatgaga | 160165 | 555497 | signal_transduction, g per | zinc binding | - |
| 5415 | POMT2 | NM_013382.3 | 1428 | ttcgactagaaacacaaagaaact | 211969 | 769441 | - | | - |
| 5416 | LASS2 | NM_013384.3 | 515 | gtggataaaccctggttctatga | 6024 | 445154 | transcription_regulator | | - |
| 5417 | LASS2 | NM_013384.3 | 552 | gggagggatatcccatacagagc | 6025 | 445158 | see also 5416 line | | |
| 5418 | LASS2 | NM_013384.3 | 592 | ttggtactacatgattgaacttt | 6028 | 445160 | see also 5416 line | | |
| 5419 | TMEM2 | NM_013390.1 | 3010 | tggctctgtgacaggatacaagg | 327931 | 743819 | - | | - |
| 5420 | TMEM2 | NM_013390.1 | 3583 | atctcaggatgtgaaagagtca | 327957 | 743838 | see also 5419 line | | - |
| 5421 | DMGDH | NM_013391.2 | 274 | tggcacgcagcagtttaacaac | 214724 | 736017 | - | oxidoreductase | |
| 5422 | DMGDH | NM_013391.2 | 275 | ggcacgcagcagtttaacaact | 214725 | 736018 | see also 5421 line | | - |
| 5423 | DMGDH | NM_013391.2 | 277 | cacgcagcagtttaacaactta | 214726 | 736019 | see also 5421 line | | - |
| 5424 | DMGDH | NM_013391.2 | 278 | acgcagcagtttaacaacttac | 214727 | 736020 | see also 5421 line | | - |
| 5425 | NRBP | | 354 | gtgcatacctggccatggataca | 94988 | 510523 | receptor_acitivity, kinase_related, signal_transduction | | |
| 5426 | NRBP | NM_013392.1 | 498 | ttgttaagtttcacaaattgg | 94992 | 510531 | see also 5425 line | | |
| 5427 | NRBP | NM_013392.1 | 774 | tggctcctgacactatcaacaat | 94999 | 510537 | see also 5425 line | | |
| 5428 | NRBP | NM_013392.1 | 1239 | aaccagttcagactttgtactct | 95007 | 510546 | see also 5425 line | | |
| 5429 | USP25 | NM_013396.2 | 948 | tagaagattagttctgattaca | 189204 | 583232 | | ubiquitin-specific protease | small cell lung cancer |
| 5430 | USP25 | NM_013396.2 | 2080 | tccagaatccatcgaacaattga | 189238 | 583262 | see also 5429 line | | |
| 5431 | STRN4 | NM_013403.1 | 386 | gggccaaatatcataaactgaag | 122962 | 527514 | signal_transduction | structural molecule | - |
| 5432 | NCKAP1 | NM_013436.2 | 861 | tacccatagcaagtctcttca | 311619 | 692786 | apoptosis | | Alzheimer disease |
| 5433 | NCKAP1 | NM_013436.2 | 1152 | ctctcttcgggatgaagtttc | 311629 | 692794 | see also 5432 line | | |
| 5434 | NCKAP1 | NM_013436.2 | 2089 | ggccaaacaagctcgaaatctca | 311667 | 692822 | see also 5432 line | | |
| 5435 | NCKAP1 | NM_013436.2 | 3118 | aactgatatgaagttgcaatga | 311700 | 692864 | see also 5432 line | | |
| 5436 | NCKAP1 | NM_013436.2 | 3128 | aaggttgcaatgaatgttatga | 311701 | 692865 | see also 5432 line | | |
| 5437 | NCKAP1 | NM_013436.2 | 3613 | tgcataccatgctgtctacaaac | 311719 | 692881 | see also 5432 line | | |
| 5438 | NCKAP1 | NM_013436.2 | 3622 | tgctgtctacaaacaagtgtta | 311721 | 692882 | see also 5432 line | | |
| 5439 | ST7 | NM_013437.2 | 48 | ctgtcgctggagcacaaaagagt | 284505 | 779254 | - | | - |
| 5440 | ST7 | NM_013437.2 | 50 | gtcgctggagcacaaaagagtct | 284506 | 779255 | see also 5439 line | | |
| 5441 | ST7 | NM_013437.2 | 975 | tggttgattatgtcaaaatatg | 284541 | 779295 | see also 5439 line | | |

278

Figure 46

| 5442 | ST7 | NM_013437.2 | 2548 | cagataccagacacttgcttaga | 284578 | 779338 | see also 5439 line | | |
|---|---|---|---|---|---|---|---|---|---|
| 5443 | UBQLN1 | NM_013438.2 | 492 | ctgaccaacttgtgttgatattt | 338927 | 767187 | - | - | - |
| 5444 | UBQLN1 | NM_013438.2 | 1526 | atgctgaataatcccctatttgc | 338939 | 727270 | see also 5443 line | | |
| 5445 | UBQLN1 | NM_013438.2 | 1529 | ctgaataatcccctatttgctgg | 338940 | 727271 | see also 5443 line | | |
| 5446 | UBQLN2 | NM_013444.2 | 1894 | tggacccaaccagcagttcattc | 338967 | 707437 | - | - | - |
| 5447 | BAZ1A | NM_013448.2 | 3231 | agccaaatcggtggtgcttttac | 22252 | 455851 | transcription_regulator | - | - |
| 5448 | BAZ1A | NM_013448.2 | 3237 | atcggtggtgcttttacagttct | 22254 | 455852 | see also 5447 line | | |
| 5449 | BAZ2A | NM_013449.2 | 358 | tggcttatctactgtatctcaca | 22306 | 455980 | transcription_regulator | - | - |
| 5450 | LAMP2 | NM_013995.1 | 1285 | ctggtctttcaggcttgattatc | 323637 | 506422 | - | - | diabetes |
| 5451 | LAMP2 | NM_013995.1 | 1292 | ttcaggcttgattatcgttatag | 323638 | 506424 | see also 5450 line | | |
| 5452 | ZNF297B | NM_014007.1 | 1228 | atgtgacgtctccattgttgtcc | 45063 | 470786 | - | - | - |
| 5453 | JM1 | NM_014008.1 | 97 | cggaccgaatcctcatccattcg | 339007 | 751896 | - | - | - |
| 5454 | ASTN2 | NM_014010.2 | 1135 | aagcccctgaaaagattctcagg | 339027 | 708542 | - | - | - |
| 5455 | ASTN2 | NM_014010.2 | 3120 | cactacaactctcactatgaaaa | 339046 | 708566 | see also 5454 line | | |
| 5456 | SACM1L | NM_014016.1 | 259 | atccatctggtggcaggtaatta | 339151 | 742326 | - | - | - |
| 5457 | SACM1L | NM_014016.1 | 550 | agggcagatcagcggtttgtatg | 339163 | 742346 | see also 5456 line | | |
| 5458 | SACM1L | NM_014016.1 | 551 | gggcagatcagcggtttgtatgg | 339164 | 742347 | see also 5456 line | | |
| 5459 | SACM1L | NM_014016.1 | 832 | cagactcgaggatcaatacctgt | 339169 | 742354 | see also 5456 line | | |
| 5460 | MAPBPIP | NM_014017.1 | 251 | aacgggaaccaagcgtttaatga | 323569 | 742898 | - | - | - |
| 5461 | MAPBPIP | NM_014017.1 | 253 | cgggaaccaagcgtttaatgaag | 323570 | 742899 | see also 5460 line | | |
| 5462 | KIAA0982 | NM_014023.1 | 208 | agcctttctatacgaagaactaa | 339286 | 773769 | - | - | - |
| 5463 | KIAA0982 | NM_014023.1 | 798 | tggagatcagactgctcatatct | 339301 | 773792 | see also 5462 line | | |
| 5464 | ASF1A | NM_014034.1 | 339 | aagtgaagaatacgatcaagttt | 17842 | 717115 | - | - | - |
| 5465 | ASF1A | NM_014034.1 | 341 | gtgaagaatacgatcaagtttta | 17843 | 717116 | see also 5464 line | | |
| 5466 | SNX24 | NM_014035.1 | 489 | ctgcagccagcgattttccaaat | 270722 | 682001 | - | protein transporter | - |
| 5467 | SNX24 | NM_014035.1 | 493 | agccagcgattttccaaatgtgg | 270723 | 682003 | see also 5466 line | | |
| 5468 | BZW2 | NM_014038.1 | 490 | aactatgctcaggtcttcaataa | 56142 | 476376 | - | - | - |
| 5469 | PTD012 | NM_014039.2 | 384 | ttggaggtgtgccttacttattg | 339351 | 747650 | - | - | - |
| 5470 | SPC12 | NM_014041.1 | 287 | tagttatggccggatttgctttt | 191689 | 584564 | - | peptidase | - |
| 5471 | GMH1 | NM_014044.4 | 1755 | tagttggaaataccttatggttg | 339409 | 721591 | - | - | - |
| 5472 | CDV-1 | NM_014055.2 | 1743 | aagaagcatcagataatagctga | 339438 | 685956 | - | - | - |
| 5473 | CDV-1 | NM_014055.2 | 2299 | aggcaattagggaacagtatacc | 339460 | 685974 | see also 5472 line | | |
| 5474 | OGN | NM_014057.2 | 836 | accagaaagtctacgtgtaattc | 169427 | 566126 | - | - | - |
| 5475 | MCTS1 | NM_014060.1 | 348 | gagcaatttccaggtattgaacc | 52483 | 474118 | cell_cycle | - | - |
| 5476 | MCTS1 | NM_014060.1 | 736 | aaggaattggcattgaaaatatc | 52496 | 474127 | see also 5475 line | | |

Figure 46

| | | | | | | | | | |
|---|---|---|---|---|---|---|---|---|---|
| 5477 | NCOA6 | NM_014071.2 | 688 | agggattctctccgttcagattg | 18644 | 450887 | transcription_regulator, signal_transduction | - | - |
| 5478 | NCOA6 | NM_014071.2 | 1071 | gacaaatgaacccagctaatttt | 18650 | 450894 | see also 5477 line | | |
| 5479 | NCOA6 | NM_014071.2 | 1646 | tcctccccaacggttaaccaaac | 18655 | 450902 | see also 5477 line | | |
| 5480 | NCOA6 | NM_014071.2 | 1651 | cccaacggttaaccaaactcagc | 18656 | 450903 | see also 5477 line | | |
| 5481 | NCOA6 | NM_014071.2 | 1683 | gaccaaggccacctcaaaataac | 18657 | 450905 | see also 5477 line | | |
| 5482 | NCOA6 | NM_014071.2 | 1747 | tcggaatcctatggttcaacagg | 18661 | 450908 | see also 5477 line | | |
| 5483 | NCOA6 | NM_014071.2 | 3224 | aaccggccaccaggttatccttc | 18693 | 450945 | see also 5477 line | | |
| 5484 | NCOA6 | NM_014071.2 | 4172 | gggcaagcaccttcaaatcttac | 18712 | 450968 | see also 5477 line | | |
| 5485 | NCOA6 | NM_014071.2 | 4173 | ggcaagcaccttcaaatcttacc | 18713 | 450969 | see also 5477 line | | |
| 5486 | NCOA6 | NM_014071.2 | 5181 | tgcagtctgcattgatgtcaaca | 18747 | 450991 | see also 5477 line | | |
| 5487 | NCOA6 | NM_014071.2 | 5421 | tacacatacctcagaacataaaa | 18752 | 450999 | see also 5477 line | | |
| 5488 | NUPL1 | NM_014089.2 | 549 | cagcagcatccacaggatttact | 339540 | 771332 | - | - | - |
| 5489 | NUPL1 | NM_014089.2 | 1137 | cagctcctgctgactacttcaga | 339556 | 771350 | see also 5488 line | | |
| 5490 | NUPL1 | NM_014089.2 | 1586 | acgccattcggctcaggtattgg | 339574 | 771364 | see also 5488 line | | |
| 5491 | NUPL1 | NM_014089.2 | 1617 | tgcaatcaagtggcttaggttct | 339576 | 771365 | see also 5488 line | | |
| 5492 | NUPL1 | NM_014089.2 | 1624 | aagtggcttaggttcttcaaacc | 339577 | 771366 | see also 5488 line | | |
| 5493 | NUPL1 | NM_014089.2 | 1746 | caggctttggcagctcaagtaca | 339579 | 771369 | see also 5488 line | | |
| 5494 | PRO2000 | NM_014109.2 | 3422 | gaggttgtagctcctccaaatat | 98117 | 488775 | - | - | - |
| 5495 | PRO2000 | NM_014109.2 | 3423 | aggttgtagctcctccaaatatg | 98118 | 488776 | see also 5494 line | | |
| 5496 | TRPS1 | NM_014112.1 | 1343 | ccccacagatctgattaagcact | 13595 | 449661 | transcription_regulator | zinc binding | trichorhinophalangeal syndrome |
| 5497 | TRPS1 | NM_014112.1 | 2085 | cagaatgatctagccaaaagttc | 13624 | 449674 | see also 5496 line | | |
| 5498 | TRPS1 | NM_014112.1 | 2296 | tccataacattcacaagtgtacc | 13633 | 449679 | see also 5496 line | | |
| 5499 | TRPS1 | NM_014112.1 | 2552 | gtcccaagcatcggatgtcaaac | 13637 | 449687 | see also 5496 line | | |
| 5500 | TRPS1 | NM_014112.1 | 2553 | tcccaagcatcggatgtcaaaca | 13638 | 449688 | see also 5496 line | | |
| 5501 | TRPS1 | NM_014112.1 | 3367 | atgcaaatggcggatatgtatgc | 13647 | 449699 | see also 5496 line | | |
| 5502 | TRPS1 | NM_014112.1 | 3652 | aagcccagggttcattgactaaa | 13656 | 449711 | see also 5496 line | | |
| 5503 | TRPS1 | NM_014112.1 | 3970 | tgcggttctggagtaaatataag | 13670 | 449722 | see also 5496 line | | |
| 5504 | TRPS1 | NM_014112.1 | 4001 | tcctgggaatccgcactacttga | 13671 | 449723 | see also 5496 line | | |
| 5505 | TRPS1 | NM_014112.1 | 4003 | ctgggaatccgcactacttgagt | 13672 | 449724 | see also 5496 line | | |
| 5506 | TRPS1 | NM_014112.1 | 4062 | gtgccttatcccaccttcaatct | 13673 | 449726 | see also 5496 line | | |
| 5507 | TRPS1 | NM_014112.1 | 4064 | gccttatcccaccttcaatctgc | 13674 | 449727 | see also 5496 line | | |
| 5508 | TRPS1 | NM_014112.1 | 4129 | tagatttggcgatcaagcattcc | 13675 | 449729 | see also 5496 line | | |
| 5509 | TRPS1 | NM_014112.1 | 4396 | cggacaaatatgacttcacaaca | 13683 | 449737 | see also 5496 line | | |
| 5510 | TRPS1 | NM_014112.1 | 4428 | aggggcctgcataggaacaatgc | 13684 | 449738 | see also 5496 line | | |

Figure 46

| | | | | | | | | | |
|---|---|---|---|---|---|---|---|---|---|
| 5511 | SCN11A | NM_014139.1 | 1503 | tggttgccatgggaattgacaga | 262139 | 553621 | channel | - | - |
| 5512 | SCN11A | NM_014139.1 | 4148 | aagttaggtggccaagacatttt | 262192 | 553692 | see also 5511 line | | |
| 5513 | SMARCAL1 | NM_014140.2 | 2667 | ccgggctttctgagaccaatttt | 40084 | 467432 | phosphatase、transcription_regulator | - | Schimke immunoosseous dysplasia |
| 5514 | HSPC055 | NM_014153.2 | 1331 | agggaacaccgcttaacaacagt | 62813 | 480869 | - | - | - |
| 5515 | HSPC055 | NM_014153.2 | 1337 | caccgcttaacaacagtaattct | 62814 | 480870 | see also 5514 line | | |
| 5516 | HSPC055 | NM_014153.2 | 1556 | caggccctaagttaatggatttc | 62821 | 480878 | see also 5514 line | | |
| 5517 | HSPC055 | NM_014153.2 | 2454 | acgtaagaagtggatgaacatcc | 62851 | 480914 | see also 5514 line | | |
| 5518 | HSPC055 | NM_014153.2 | 2563 | aactgttcctttgctcatagtcc | 62852 | 480920 | see also 5514 line | | |
| 5519 | HSPC056 | NM_014154.1 | 156 | agctagcagtcgccactatgttg | 339663 | 735922 | - | - | - |
| 5520 | HSPC056 | NM_014154.1 | 159 | tagcagtcgccactatgttgaca | 339664 | 735923 | see also 5519 line | | |
| 5521 | HSPC056 | NM_014154.1 | 169 | cactatgttgacaggctatttga | 339665 | 735924 | see also 5519 line | | |
| 5522 | HSPC056 | NM_014154.1 | 1042 | tacagagaatcgctagcataact | 352220 | 735962 | see also 5519 line | | |
| 5523 | HSPC063 | NM_014155.1 | 433 | cagccagctatatgcaaatgttc | 339693 | 531438 | - | - | - |
| 5524 | THY28 | NM_014174.2 | 962 | atggtggatgtacagtttgttcg | 339855 | 753462 | - | - | - |
| 5525 | HSPC150 | NM_014176.1 | 297 | tcctgagaggtacccatttgaac | 229845 | 615103 | - | - | - |
| 5526 | HSPC150 | NM_014176.1 | 417 | ttggagaccatccctcaacatcg | 229849 | 615108 | see also 5525 line | | |
| 5527 | HSPC159 | NM_014181.1 | 115 | gtgtacttcccacgactgatagt | 339922 | 784224 | - | - | - |
| 5528 | HSPC159 | NM_014181.1 | 123 | cccacgactgatagttccatttt | 339924 | 784225 | see also 5527 line | | |
| 5529 | HSPC159 | NM_014181.1 | 125 | cacgactgatagttccattttgt | 339925 | 784226 | see also 5527 line | | |
| 5530 | ORMDL2 | NM_014182.3 | 68 | ctggctggcctacatcatcttgg | 339940 | 714703 | - | - | - |
| 5531 | DNCL2A | NM_014183.2 | 299 | ttgcaccagataaagactatttc | 130771 | 720328 | - | - | - |
| 5532 | SCN8A | NM_014191.1 | 1710 | atcccgagaaggtgtttaagtca | 100201 | 514468 | channel | - | motor endplate disease |
| 5533 | SCN8A | NM_014191.1 | 2258 | aaggacagaatcaacagtataat | 100205 | 514473 | see also 5532 line | | |
| 5534 | SCN8A | NM_014191.1 | 4036 | ctcggaactaggtgccataaagt | 100224 | 514494 | see also 5532 line | | |
| 5535 | AP2A1 | NM_014203.2 | 354 | aagccttggatggctacagtaag | 267547 | 650479 | - | - | - |
| 5536 | AP2A1 | NM_014203.2 | 355 | agccttggatggctacagtaaga | 267548 | 650480 | see also 5535 line | | |
| 5537 | HOXD9 | NM_014213.2 | 591 | ccgagttcgcctcgtgtagtttt | 5256 | 444899 | transcription_regulator | RNA polymerase II transcription factor | - |
| 5538 | HOXD9 | NM_014213.2 | 593 | gagttcgcctcgtgtagttttgc | 5257 | 444900 | see also 5537 line | | |
| 5539 | INSRR | NM_014215.1 | 2843 | gtgtgtgtcccgtcttcgatatg | 85815 | 504426 | receptor_acitivity、signal_transduction、kinase_related | - | - |
| 5540 | ITPK1 | NM_014216.2 | 845 | accgtgagtccatcttcttcaac | 219498 | 775986 | kinase_related、signal_transduction | - | - |
| 5541 | RAGE | NM_014226.1 | 450 | ctggttctcttgcactaatatgt | 98877 | 513173 | - | - | - |
| 5542 | RAGE | NM_014226.1 | 454 | ttctcttgcactaatatgtgaac | 98878 | 513174 | see also 5541 line | | |

Figure 46

| 5543 | RAGE | NM_014226.1 | 459 | ttgcactaatatgtgaacttatg | 98879 | 513175 | see also 5541 line | | |
|---|---|---|---|---|---|---|---|---|---|
| 5544 | RAGE | NM_014226.1 | 469 | atgtgaacttatggacatgaata | 98880 | 513177 | see also 5541 line | | |
| 5545 | RAGE | NM_014226.1 | 471 | gtgaacttatggacatgaatatt | 98881 | 513178 | see also 5541 line | | |
| 5546 | RAGE | NM_014226.1 | 670 | ctgccggagtgtctattccaagc | 98887 | 513184 | see also 5541 line | | |
| 5547 | SLC6A11 | NM_014229.1 | 1507 | tggaagcaaccggttctatgata | 255925 | 639910 | - | betaine/GABA:sodiu m symporter | - |
| 5548 | SRP68 | NM_014230.2 | 170 | ggccggatcgaaggcaaacaaag | 54887 | 759511 | - | signal recognition particle receptor | - |
| 5549 | SRP68 | NM_014230.2 | 171 | gccggatcgaaggcaaacaaaga | 54888 | 759512 | see also 5548 line | | |
| 5550 | UBTF | NM_014233.1 | 416 | tcgatgctcaggaacatgttaaa | 43206 | 470144 | transcription_regulator | translation regulator | - |
| 5551 | HSD17B8 | NM_014234.3 | 617 | tcctcccagggttcattgcaaca | 219899 | 704451 | - | estradiol 17 beta-dehydrogenase | - |
| 5552 | PTPLA | NM_014241.2 | 578 | ctcgctattccttctacacattc | 195407 | 588685 | - | - | - |
| 5553 | ADAMTS2 | NM_014244.1 | 3186 | ctgccaaggcgacaagtcaatat | 184174 | 578364 | - | - | - |
| 5554 | CELSR1 | NM_014246.1 | 6132 | cacgctcggctgtgaagtgatct | 140532 | 546068 | gpcr、receptor_acitivit y、signal_transduction | structural molecule | - |
| 5555 | CELSR1 | NM_014246.1 | 6721 | atcgtcaccgccaacatgattct | 140535 | 546075 | see also 5554 line | | |
| 5556 | SLC25A13 | NM_014251.1 | 512 | tccatttaactgggattcagaat | 153035 | 553953 | - | carrier | - |
| 5557 | SLC25A13 | NM_014251.1 | 940 | aagttgacatcttgtttcagtta | 153046 | 553962 | see also 5556 line | | |
| 5558 | SLC25A13 | NM_014251.1 | 1173 | gtgtatcctatcgatcttgtaaa | 153050 | 553965 | see also 5556 line | | |
| 5559 | ODZ1 | NM_014253.1 | 1315 | ctggcgtttccagattactatcc | 177729 | 576518 | - | - | - |
| 5560 | ODZ1 | NM_014253.1 | 1541 | gagacaggtttcatagagtatat | 177737 | 576523 | see also 5559 line | | |
| 5561 | ODZ1 | NM_014253.1 | 1546 | aggtttcatagagtatatggatc | 177738 | 576525 | see also 5559 line | | |
| 5562 | ODZ1 | NM_014253.1 | 2207 | ggctgggtaggaccaacatgtga | 177753 | 576540 | see also 5559 line | | |
| 5563 | ODZ1 | NM_014253.1 | 2306 | gagggcgaccactgcacaattgc | 177755 | 576541 | see also 5559 line | | |
| 5564 | ODZ1 | NM_014253.1 | 3627 | cagcccacaacaacaaactcttt | 177782 | 576575 | see also 5559 line | | |
| 5565 | ODZ1 | NM_014253.1 | 4635 | accaagcccacctgaatgacatg | 177815 | 576601 | see also 5559 line | | |
| 5566 | ODZ1 | NM_014253.1 | 4739 | ttgataacaagggactatgttta | 177820 | 576609 | see also 5559 line | | |
| 5567 | ODZ1 | NM_014253.1 | 5708 | tggagctatacctacttagaaaa | 177858 | 576643 | see also 5559 line | | |
| 5568 | ODZ1 | NM_014253.1 | 5710 | gagctatacctacttagaaaaat | 177859 | 576644 | see also 5559 line | | |
| 5569 | ODZ1 | NM_014253.1 | 7817 | ctggtgctcatcggtaacactgg | 177919 | 576721 | see also 5559 line | | |
| 5570 | YME1L1 | NM_014263.2 | 366 | agggaccttggattatctgaact | 107713 | 517760 | - | - | - |
| 5571 | STK18 | NM_014264.2 | 345 | atggtacagagagtccaaaatga | 101478 | 702415 | kinase_related、cell_c ycle | - | - |
| 5572 | STK18 | NM_014264.2 | 2287 | ctcccaaaatcacttattttaca | 101531 | 702463 | see also 5571 line | | |

Figure 46

| | | | | | | | | | |
|---|---|---|---|---|---|---|---|---|---|
| 5573 | MAPRE2 | NM_014268.1 | 136 | gtccccaaatggcgagaacaaca | 120288 | 525085 | signal_transduction | - | - |
| 5574 | MAPRE2 | NM_014268.1 | 242 | atggcggtcaatgtgtattctac | 120290 | 525090 | see also 5573 line | | |
| 5575 | MAPRE2 | NM_014268.1 | 243 | tggcggtcaatgtgtattctacc | 120291 | 525091 | see also 5573 line | | |
| 5576 | MGAT4B | NM_014275.2 | 768 | gaccaaacagaacctcgattact | 210492 | 600789 | - | - | - |
| 5577 | APG-1 | NM_014278.2 | 382 | aagaactcgagccattggaaatg | 72218 | 510834 | - | heat shock protein | - |
| 5578 | APG-1 | NM_014278.2 | 458 | aagcttcatgggcgatcatttga | 72224 | 510836 | see also 5577 line | | |
| 5579 | APG-1 | NM_014278.2 | 966 | aggctttagtagactacttctgt | 72237 | 510843 | see also 5577 line | | |
| 5580 | APG-1 | NM_014278.2 | 1198 | cagggttgaaccacctttaaaag | 72245 | 510851 | see also 5577 line | | |
| 5581 | APG-1 | NM_014278.2 | 1199 | agggttgaaccacctttaaaagc | 72246 | 510852 | see also 5577 line | | |
| 5582 | APG-1 | NM_014278.2 | 1559 | aagaaggaaccatttgaactaga | 72257 | 510859 | see also 5577 line | | |
| 5583 | APG-1 | NM_014278.2 | 1562 | aaggaaccatttgaactagaagc | 72258 | 510860 | see also 5577 line | | |
| 5584 | APG-1 | NM_014278.2 | 1578 | tagaagcattttatactaattta | 72259 | 510861 | see also 5577 line | | |
| 5585 | OLFM1 | NM_014279.2 | 792 | aagcgaaaatggatgaacttagg | 319967 | 685416 | receptor_acitivity | - | - |
| 5586 | OLFM1 | NM_014279.2 | 842 | aaggccgatgccaaattggtatt | 319968 | 685417 | see also 5585 line | | |
| 5587 | OLFM1 | NM_014279.2 | 843 | aggccgatgccaaattggtattg | 319969 | 685418 | see also 5585 line | | |
| 5588 | OLFM1 | NM_014279.2 | 844 | ggccgatgccaaattggtattgc | 319970 | 685419 | see also 5585 line | | |
| 5589 | SIAHBP1 | NM_014281.3 | 1656 | accaggagcgttttgataacagt | 66153 | 478763 | - | - | - |
| 5590 | C1orf9 | NM_014283.2 | 1251 | ttgcagcactaaaatttggtttg | 340058 | 776854 | - | - | - |
| 5591 | C1orf9 | NM_014283.2 | 3130 | caggatcagcggcaaactgaagc | 340101 | 776909 | see also 5590 line | | |
| 5592 | RRP4 | NM_014285.4 | 851 | aacacgccagaggcttttggaac | 49472 | 475438 | - | 7S RNA binding | |
| 5593 | FREQ | NM_014286.2 | 525 | aagagggtggaccggatctttgc | 146515 | 708998 | - | calcium-dependent protein kinase inhibitor | - |
| 5594 | CAPN6 | NM_014289.2 | 1923 | ccgaaaattctgtgatcagttct | 187673 | 581429 | - | calpain | - |
| 5595 | CAPN6 | NM_014289.2 | 1974 | cagcgactgccgtgatctgaagt | 187674 | 581430 | see also 5594 line | | |
| 5596 | CAPN6 | NM_014289.2 | 1995 | gtctctgtacctgcgtaagaagg | 187675 | 581434 | see also 5594 line | | |
| 5597 | PCTAIRE2 BP | NM_014290.1 | 964 | agcataacaatggcatttggata | 64826 | 479997 | - | - | - |
| 5598 | PCTAIRE2 BP | NM_014290.1 | 969 | aacaatggcatttggatatctaa | 64827 | 479998 | see also 5597 line | | |
| 5599 | PCTAIRE2 BP | NM_014290.1 | 1341 | ttccctgaggatgccttaaaaaa | 64837 | 480007 | see also 5597 line | | |
| 5600 | CBX6 | NM_014292.2 | 121 | aggggtgggcgatcaagtacagc | 17924 | 636738 | transcription_regulator | chromatin binding | - |
| 5601 | TRAM1 | NM_014294.3 | 428 | tacatgctagtggcgataattat | 302534 | 732922 | receptor_acitivity | endoplasmic reticulum receptor | - |
| 5602 | TRAM1 | NM_014294.3 | 431 | atgctagtggcgataattattca | 302535 | 732923 | see also 5601 line | | |
| 5603 | CAPN7 | NM_014296.1 | 1053 | accaacaatcctacaatgatata | 187698 | 581461 | - | - | - |

Figure 46

| | | | | | | | | | |
|---|---|---|---|---|---|---|---|---|---|
| 5604 | CAPN7 | NM_014296.1 | 1091 | tagcataaagcagacaatagtat | 187702 | 581462 | see also 5603 line | | |
| 5605 | CAPN7 | NM_014296.1 | 1092 | agcataaagcagacaatagtatc | 187703 | 581463 | see also 5603 line | | |
| 5606 | CAPN7 | NM_014296.1 | 2296 | ttccttcaccatacaccttatca | 187744 | 581501 | see also 5603 line | | |
| 5607 | BRD4 | NM_014299.1 | 430 | gtgctcaagacactatggaaaca | 324010 | 710459 | - | - | - |
| 5608 | BRD4 | NM_014299.1 | 556 | aagcgcttggaaaacaactatta | 324014 | 710462 | see also 5607 line | | |
| 5609 | BRD4 | NM_014299.1 | 557 | agcgcttggaaaacaactattac | 324015 | 710463 | see also 5607 line | | |
| 5610 | BRD4 | NM_014299.1 | 805 | acggtaccaaacacaactcaagc | 324018 | 710470 | see also 5607 line | | |
| 5611 | BRD4 | NM_014299.1 | 2236 | acctcctgtttgcggaagaaaag | 324023 | 710498 | see also 5607 line | | |
| 5612 | HSPC117 | NM_014306.2 | 461 | gtggtgtccgcttgctaagaacc | 280741 | 667189 | - | - | - |
| 5613 | HSPC117 | NM_014306.2 | 466 | gtccgcttgctaagaaccaattt | 280742 | 667190 | see also 5612 line | | |
| 5614 | HSPC117 | NM_014306.2 | 467 | tccgcttgctaagaaccatttta | 280743 | 667191 | see also 5612 line | | |
| 5615 | HSPC117 | NM_014306.2 | 760 | ctgggagcaggcaaccattatgc | 280752 | 667197 | see also 5612 line | | |
| 5616 | HSPC117 | NM_014306.2 | 768 | aggcaaccattatgcagaaatcc | 280753 | 667198 | see also 5612 line | | |
| 5617 | RIG-I | NM_014314.2 | 2011 | tacaagaagagtaccacttaaac | 65440 | 480413 | - | - | - |
| 5618 | KLHDC2 | NM_014315.1 | 458 | ggcggctacaagagtaatcaagt | 340150 | 728114 | - | - | - |
| 5619 | ORC6L | NM_014321.2 | 354 | aagagacctagctgtacagttta | 34416 | 463343 | - | DNA binding | |
| 5620 | CORO1C | NM_014325.2 | 998 | acgtccactacctcaacacattc | 116311 | 521395 | signal_transduction | actin binding | - |
| 5621 | DAPK2 | NM_014326.2 | 523 | tccacacatcaagctgattgact | 77969 | 499414 | kinase_related、apoptosis | - | Alzheimer disease |
| 5622 | DAPK2 | NM_014326.2 | 551 | ctggctcacgaaatagaagatgg | 77970 | 499416 | see also 5621 line | | |
| 5623 | RCD-8 | NM_014329.2 | 239 | cggccatccagtgcctacaatgg | 283264 | 806609 | - | - | - |
| 5624 | RCD-8 | NM_014329.2 | 1752 | ctgcccacgaggacttcacattt | 283280 | 806635 | see also 5623 line | | |
| 5625 | RCD-8 | NM_014329.2 | 1893 | gtgagaccaagcccaagttgatg | 283281 | 806637 | see also 5623 line | | |
| 5626 | SLC7A11 | NM_014331.2 | 421 | gtgatatccctggcatttggacg | 258518 | 641395 | - | cystine/glutamate antiporter | - |
| 5627 | SLC7A11 | NM_014331.2 | 1219 | ctgatttatcttcgatacaaatg | 258549 | 641417 | see also 5626 line | | |
| 5628 | IGSF4 | NM_014333.2 | 1190 | ggcgctattttgccagacataaa | 340211 | 535837 | - | - | - |
| 5629 | IGSF4 | NM_014333.2 | 1191 | gcgctattttgccagacataaag | 340212 | 535838 | see also 5628 line | | |
| 5630 | ZNF318 | NM_014345.1 | 724 | cccttcttgggacaacttgatga | 55929 | 476015 | - | - | - |
| 5631 | SACS | NM_014363.3 | 421 | caggccaatgttggaatctcaaa | 283608 | 779167 | - | molecular_function unknown | - |
| 5632 | SACS | NM_014363.3 | 422 | aggccaatgttggaatctcaaag | 283609 | 779168 | see also 5631 line | | |
| 5633 | SACS | NM_014363.3 | 423 | ggccaatgttggaatctcaaaga | 283610 | 779169 | see also 5631 line | | |
| 5634 | NAKAP95 | NM_014371.1 | 302 | gccagtgccgattccgttttatc | 125866 | 607114 | - | DEAD/H-box RNA helicase binding | - |
| 5635 | NAKAP95 | NM_014371.1 | 1623 | cccgcagtattctcaacaacaag | 125877 | 607136 | see also 5634 line | | |
| 5636 | RNF11 | NM_014372.3 | 516 | cagtccgaccgggctagctttgg | 38636 | 539918 | - | zinc binding | - |

EP 1 752 536 A1

Figure 46

| 5637 | RNF11 | NM_014372.3 | 569 | gccgccatatcaggaacaagttc | 38637 | 539919 | see also 5636 line | | | |
| 5638 | CYFIP2 | NM_014376.1 | 371 | atcatgtaccaggctaactttga | 340421 | 557425 | - | - | - | |
| 5639 | CYFIP2 | NM_014376.1 | 425 | acgggcattgcaaggtacattga | 340424 | 557426 | see also 5638 line | | | |
| 5640 | CYFIP2 | NM_014376.1 | 2913 | agccttattacctctatggatcc | 340466 | 557465 | see also 5638 line | | | |
| 5641 | CYFIP2 | NM_014376.1 | 2929 | tggatccaagcctctcaacattg | 340467 | 557466 | see also 5638 line | | | |
| 5642 | ATP2C1 | NM_014382.1 | 534 | ttgatgatgccgtcagtatcact | 72975 | 494581 | - | plasma membrane cation-transporting ATPase | benign chronic pemphigus | |
| 5643 | ATP2C1 | NM_014382.1 | 2357 | agcacgagtatagcagcattaac | 73028 | 494632 | see also 5642 line | | | |
| 5644 | ACAD8 | NM_014384.1 | 501 | atgaggaacagaggcacaaattt | 223810 | 612120 | transcription_regulator | - | - | |
| 5645 | LAT | NM_014387.2 | 726 | cagccgggagtatgtgaatgtgt | 252717 | 682447 | signal_transduction | SH3/SH2 adaptor protein | - | |
| 5646 | MGC29875 | NM_014388.2 | 919 | ttcttaccgggacctgttctacc | 340490 | 756280 | - | - | - | |
| 5647 | MGC29875 | NM_014388.2 | 1333 | caggattggagtggcaatacttc | 340499 | 756291 | see also 5646 line | | | |
| 5648 | MGC29875 | NM_014388.2 | 1334 | aggattggagtggcaatacttca | 340500 | 756292 | see also 5646 line | | | |
| 5649 | PELP1 | NM_014389.1 | 2627 | aggatttgacagttattaatatc | 324837 | 738184 | - | - | - | |
| 5650 | SND1 | NM_014390.1 | 477 | ctgtttcacgatagaaaacaaga | 66167 | 481320 | - | - | - | |
| 5651 | SND1 | NM_014390.1 | 1148 | aagagcgcaggctgagaatatgg | 66184 | 481330 | see also 5650 line | | | |
| 5652 | SND1 | NM_014390.1 | 1161 | gagaatatggagagactatgtgg | 66185 | 481333 | see also 5650 line | | | |
| 5653 | SND1 | NM_014390.1 | 1637 | cagaggccagagctattaagaat | 66197 | 481339 | see also 5650 line | | | |
| 5654 | ANKRD1 | NM_014391.2 | 1030 | gactgaaccgctataagatgatc | 21374 | 525795 | signal_transduction | - | - | |
| 5655 | D4S234E | NM_014392.2 | 399 | agggtgtcaccgagagagtttaag | 323545 | 687923 | signal_transduction | - | - | |
| 5656 | D4S234E | NM_014392.2 | 400 | gggtgtcaccgagaggtttaagg | 323546 | 687924 | see also 5655 line | | | |
| 5657 | STAU2 | NM_014393.1 | 1532 | cagctgaagccataggtttaaaa | 48816 | 472205 | - | - | - | |
| 5658 | DAPP1 | NM_014395.1 | 82 | atgggcagagcagaacttctaga | 172844 | 596976 | phosphatase | - | - | |
| 5659 | DAPP1 | NM_014395.1 | 676 | cagatgtcaccagaaccaattcg | 172851 | 596988 | see also 5658 line | | | |
| 5660 | DAPP1 | NM_014395.1 | 696 | tcggatcctagacctaacagaat | 172852 | 596989 | see also 5658 line | | | |
| 5661 | VPS41 | NM_014396.2 | 117 | aagtatgaaaggctttccaatgg | 272883 | 771728 | - | - | - | |
| 5662 | VPS41 | NM_014396.2 | 659 | aagtcttcgcccagacatgtatc | 272896 | 771738 | see also 5661 line | | | |
| 5663 | CACNG4 | NM_014405.2 | 475 | atcggtatcatcgtctacatttc | 261602 | 645063 | - | voltage-gated calcium channel | - | |
| 5664 | TAF5L | NM_014409.2 | 1342 | gacgtacccgctgaggatatatg | 12654 | 749377 | transcription_regulator | - | - | |
| 5665 | TAF5L | NM_014409.2 | 1377 | cagatgtggactgtgtcaaattc | 12656 | 749379 | see also 5664 line | | | |

Figure 46

| 5666 | SIP | NM_014412.1 | 728 | atgatatgaagcgaaccattaat | 340556 | 699919 | - | - | - |
|------|------|-------------|------|--------------------------|--------|--------|----|----|----|
| 5667 | AF5Q31 | NM_014423.1 | 1515 | atggggaacaggattgtgataag | 727 | 744315 | transcription_regulator | - | - |
| 5668 | AF5Q31 | NM_014423.1 | 1567 | ctctgaaccttcacaccataata | 730 | 744318 | see also 5667 line | | |
| 5669 | AF5Q31 | NM_014423.1 | 3362 | ctgtacctgaggctgttcaaact | 760 | 744351 | see also 5667 line | | |
| 5670 | AF5Q31 | NM_014423.1 | 3732 | gccctctcatctttaatgcaagc | 764 | 744356 | see also 5667 line | | |
| 5671 | INVS | NM_014425.2 | 353 | tgggagaacaccacttatgtatt | 340578 | 701231 | - | - | - |
| 5672 | INVS | NM_014425.2 | 1320 | gtcagcaccgtgaagttattact | 340609 | 701245 | see also 5671 line | | |
| 5673 | SNX5 | NM_014426.2 | 275 | ttctgttacaaggcaacatgaag | 270843 | 652558 | - | - | - |
| 5674 | SNX5 | NM_014426.2 | 589 | atgatcaggatctaagtgttagg | 270850 | 652571 | see also 5673 line | | |
| 5675 | MORC | NM_014429.1 | 1160 | tggaatgttcatttacagtaata | 91740 | 508551 | - | - | - |
| 5676 | 76P | NM_014444.2 | 639 | tccctctgtgatggttgtagtag | 286166 | 675643 | - | structural constituent of cytoskeleton | - |
| 5677 | 76P | NM_014444.2 | 1292 | tgggtcagctgaagatcattaaa | 286183 | 675660 | see also 5676 line | | |
| 5678 | 76P | NM_014444.2 | 1865 | agctgcttcatcagatcaattct | 286192 | 675670 | see also 5676 line | | |
| 5679 | ZNF364 | NM_014455.1 | 292 | gccccaaactaccggaatatata | 340675 | 724764 | - | - | - |
| 5680 | ZNF364 | NM_014455.1 | 293 | ccccaaactaccggaatatatat | 340676 | 724765 | see also 5679 line | | |
| 5681 | ZNF364 | NM_014455.1 | 294 | cccaaactaccggaatatatatg | 340677 | 724766 | see also 5679 line | | |
| 5682 | ZNF364 | NM_014455.1 | 1096 | tacatgaccgatggactttctga | 340696 | 724784 | see also 5679 line | | |
| 5683 | PDCD4 | NM_014456.3 | 621 | acctggacaggtgtatgatgtgg | 340704 | 619807 | apoptosis | - | - |
| 5684 | PDCD4 | NM_014456.3 | 1018 | cagttggtgggccagtttattgc | 340716 | 619817 | see also 5683 line | | |
| 5685 | PDCD4 | NM_014456.3 | 1022 | tggtgggccagtttattgctaga | 340717 | 619818 | see also 5683 line | | |
| 5686 | AB026190 | NM_014458.3 | 665 | gagacaattagatccttctaact | 128441 | 457892 | - | - | - |
| 5687 | AB026190 | NM_014458.3 | 690 | ctgggcattcgggcttttgctga | 128443 | 457893 | see also 5686 line | | |
| 5688 | AB026190 | NM_014458.3 | 745 | cagacaagttcacccaacataac | 128445 | 457894 | see also 5686 line | | |
| 5689 | AB026190 | NM_014458.3 | 1036 | tggtagatgaggctaaaaactac | 128457 | 457907 | see also 5686 line | | |
| 5690 | CNTN6 | NM_014461.2 | 1934 | gagaatctgttggggatttgatg | 77341 | 498635 | - | - | - |
| 5691 | CNTN6 | NM_014461.2 | 1943 | ttggggatttgatgataaggaat | 77342 | 498637 | see also 5690 line | | |
| 5692 | CNTN6 | NM_014461.2 | 2976 | aagccaaccaccagcaaacattg | 77373 | 498668 | see also 5690 line | | |
| 5693 | CNTN6 | NM_014461.2 | 2977 | agccaaccaccagcaaacattgc | 77374 | 498669 | see also 5690 line | | |
| 5694 | LSM3 | NM_014463.1 | 106 | atgagcgaatttatgtgaaaatg | 46336 | 471255 | - | pre-mRNA splicing factor | - |
| 5695 | LSM3 | NM_014463.1 | 109 | agcgaatttatgtgaaaatgaga | 46337 | 471256 | see also 5694 line | | |
| 5696 | TINAG | NM_014464.1 | 913 | cgcatgctacccacttttcaaag | 191117 | 582768 | - | - | - |
| 5697 | TEKT2 | NM_014466.2 | 360 | tggacaagtgtctgacagattta | 292428 | 662220 | - | - | - |
| 5698 | SRPUL | NM_014467.1 | 928 | tggaggcgagcctgtatgtgtag | 304282 | 671835 | - | - | - |
| 5699 | HSA9761 | NM_014473.1 | 945 | ggcccgttccatggacatagatg | 213889 | 603916 | - | - | - |

Figure 46

| | | | | | | | | | |
|---|---|---|---|---|---|---|---|---|---|
| 5700 | ADAMDEC1 | NM_014479.2 | 340 | tggggccagactacactgaaaca | 155149 | 556200 | signal_transduction | - | - |
| 5701 | RBMS3 | NM_014483.1 | 306 | cagcggggaacagttgagtaaaa | 54115 | 472932 | - | - | - |
| 5702 | RBMS3 | NM_014483.1 | 1253 | tgcctccgacagctgtttctatt | 54139 | 472952 | see also 5701 line | | |
| 5703 | RBMS3 | NM_014483.1 | 1257 | tccgacagctgtttctattgaag | 54141 | 472953 | see also 5701 line | | |
| 5704 | RAB30 | NM_014488.3 | 377 | gagccacaattggagttgatttt | 110121 | 518269 | signal_transduction | RAB small monomeric GTPase | - |
| 5705 | RAB30 | NM_014488.3 | 406 | aagacagtggagattaatggtga | 110123 | 518271 | see also 5704 line | | |
| 5706 | RAB30 | NM_014488.3 | 841 | atcagctatttgacttgttgtaa | 110132 | 518281 | see also 5704 line | | |
| 5707 | FOXP2 | NM_014491.1 | 548 | aacaactacaagagttttacaag | 4223 | 444203 | transcription_regulator | - | - |
| 5708 | FOXP2 | NM_014491.1 | 1570 | gccatacgaaggcgacattcaga | 4237 | 444214 | see also 5707 line | | |
| 5709 | FOXP2 | NM_014491.1 | 1580 | ggcgacattcagacaaatacaac | 4238 | 444216 | see also 5707 line | | |
| 5710 | FOXP2 | NM_014491.1 | 1747 | tggtttacacggacatttgctta | 4241 | 444227 | see also 5707 line | | |
| 5711 | TNRC6 | NM_014494.1 | 3742 | aagtgcctcctccattactctcc | 66892 | 486925 | - | - | - |
| 5712 | TNRC6 | NM_014494.1 | 3887 | ctcccagttacagcgattgttag | 66893 | 486927 | see also 5711 line | | |
| 5713 | TNRC6 | NM_014494.1 | 4311 | agcaaacatggtgctatttcaag | 66902 | 486935 | see also 5711 line | | |
| 5714 | TNRC6 | NM_014494.1 | 4542 | gtcactcctggcagtgtcataaa | 66908 | 486940 | see also 5711 line | | |
| 5715 | TNRC6 | NM_014494.1 | 4544 | cactcctggcagtgtcataaaca | 66909 | 486941 | see also 5711 line | | |
| 5716 | TNRC6 | NM_014494.1 | 4641 | accacgctgccttcaactagtgc | 66912 | 486944 | see also 5711 line | | |
| 5717 | TNRC6 | NM_014494.1 | 4759 | ctcctggttcagttacaaacacc | 66913 | 486949 | see also 5711 line | | |
| 5718 | TNRC6 | NM_014494.1 | 4875 | cccttgtctacgtgggataattc | 66915 | 486950 | see also 5711 line | | |
| 5719 | TNRC6 | NM_014494.1 | 4878 | ttgtctacgtgggataattctcc | 66916 | 486951 | see also 5711 line | | |
| 5720 | RPS6KA6 | NM_014496.1 | 697 | ttgatgaaataggacatatcaaa | 99906 | 514338 | kinase_related、signal_transduction | protein serine/threonine kinase | - |
| 5721 | NP220 | NM_014497.2 | 519 | atgaatgttcaggtaactcaaca | 20094 | 474318 | - | - | - |
| 5722 | NP220 | NM_014497.2 | 864 | cgccgattacctaatttaccttc | 20104 | 474336 | see also 5721 line | | |
| 5723 | NP220 | NM_014497.2 | 865 | gccgattacctaatttaccttct | 20105 | 474337 | see also 5721 line | | |
| 5724 | NP220 | NM_014497.2 | 979 | atccacttgaagtacgtatttat | 20109 | 474341 | see also 5721 line | | |
| 5725 | NP220 | NM_014497.2 | 980 | tccacttgaagtacgtatttatg | 20110 | 474342 | see also 5721 line | | |
| 5726 | NP220 | NM_014497.2 | 1602 | ctgtgtaacgtagaatgtagtca | 20133 | 474355 | see also 5721 line | | |
| 5727 | NP220 | NM_014497.2 | 5559 | gtgactttggatgaagtaactga | 20258 | 474458 | see also 5721 line | | |
| 5728 | GOLPH4 | NM_014498.2 | 2289 | gccgagcaagtgagagaagaaaa | 323538 | 786327 | - | - | - |
| 5729 | CNOT2 | NM_014515.1 | 371 | atgaggggatgagcaacaatac | 275364 | 732379 | - | - | - |
| 5730 | CNOT2 | NM_014515.1 | 398 | cagttaaatcgcagcttatcaca | 275366 | 732381 | see also 5729 line | | |
| 5731 | CNOT2 | NM_014515.1 | 437 | agccacgtcacgccaacaacagg | 275367 | 732382 | see also 5729 line | | |
| 5732 | CNOT2 | NM_014515.1 | 567 | ttcctagcaggacaaatagcatg | 275369 | 732387 | see also 5729 line | | |

Figure 46

| 5733 | CNOT2 | NM_014515.1 | 568 | tcctagcaggacaaatagcatga | 275370 | 732388 | see also 5729 line | | |
|---|---|---|---|---|---|---|---|---|---|
| 5734 | CNOT2 | NM_014515.1 | 614 | aacagaagctcgccaagcataat | 275371 | 732389 | see also 5729 line | | |
| 5735 | CNOT2 | NM_014515.1 | 620 | agctcgccaagcataatatgtat | 275373 | 732390 | see also 5729 line | | |
| 5736 | CNOT2 | NM_014515.1 | 622 | ctcgccaagcataatatgtatgc | 275374 | 732391 | see also 5729 line | | |
| 5737 | CNOT2 | NM_014515.1 | 654 | agccttctcgacagccttttact | 275375 | 732392 | see also 5729 line | | |
| 5738 | CNOT2 | NM_014515.1 | 658 | ttctcgacagccttttactgtga | 275376 | 732393 | see also 5729 line | | |
| 5739 | CNOT2 | NM_014515.1 | 725 | atgaataactccttatcaagtaa | 275380 | 732395 | see also 5729 line | | |
| 5740 | CNOT2 | NM_014515.1 | 1065 | tccctggagataaaagttcaaca | 275393 | 732415 | see also 5729 line | | |
| 5741 | CNOT2 | NM_014515.1 | 1159 | agggatggtgacggaccaatttg | 275397 | 732418 | see also 5729 line | | |
| 5742 | CNOT2 | NM_014515.1 | 1160 | gggatggtgacggaccatttgg | 275398 | 732419 | see also 5729 line | | |
| 5743 | CNOT2 | NM_014515.1 | 1163 | atggtgacggaccatttggaat | 275399 | 732420 | see also 5729 line | | |
| 5744 | CNOT2 | NM_014515.1 | 1188 | ttggcctgttaacatttatcagg | 275402 | 732423 | see also 5729 line | | |
| 5745 | CNOT3 | NM_014516.1 | 1845 | ctcattgagacgcaaatggaacg | 340813 | 760977 | transcription_regulator | - | - |
| 5746 | CNOT3 | NM_014516.1 | 3757 | agcagggcacctacatctacttt | 340828 | 761000 | see also 5745 line | | |
| 5747 | CNOT3 | NM_014516.1 | 3764 | cacctacatctactttgactacg | 340829 | 761002 | see also 5745 line | | |
| 5748 | UBP1 | NM_014517.2 | 1764 | ccgatggaattcggctctataat | 13851 | 704790 | transcription_regulator | - | - |
| 5749 | UBP1 | NM_014517.2 | 1767 | atggaattcggctctataattca | 13852 | 704791 | see also 5748 line | | |
| 5750 | TMOD2 | NM_014548.2 | 222 | atgagcttcttggcaaactctca | 119677 | 524703 | - | - | - |
| 5751 | TMOD2 | NM_014548.2 | 777 | acctcaacaacattaagaacatt | 119687 | 524709 | see also 5750 line | | |
| 5752 | CARD10 | NM_014550.2 | 2109 | aggtgccgagcccttctacattc | 129594 | 533190 | apoptosis、kinase_rela ted | receptor signaling complex scaffold protein | - |
| 5753 | CARD10 | NM_014550.2 | 2281 | cggggcaccgtgcccaattatca | 129595 | 533192 | see also 5752 line | | |
| 5754 | OTX1 | NM_014562.2 | 1258 | cccccgactgtctggactataag | 8787 | 447527 | transcription_regulator | transcription factor | - |
| 5755 | GALNT5 | NM_014568.1 | 2373 | gtggatgtgtggtggtgaaattg | 211108 | 664128 | - | - | - |
| 5756 | ARFGAP3 | NM_014570.3 | 172 | cactaacaaggtgtgttttgatt | 21519 | 454984 | - | - | Alzheimer disease |
| 5757 | STRN3 | NM_014574.2 | 553 | aagatgttagagtatgcattaaa | 122893 | 527431 | cell_cycle | molecular_function unknown | - |
| 5758 | STRN3 | NM_014574.2 | 1187 | ctgaaccaataacgtttccatct | 122911 | 527462 | see also 5757 line | | |
| 5759 | STRN3 | NM_014574.2 | 1278 | tgcagacttgacggtaacaaatg | 122914 | 527463 | see also 5757 line | | |
| 5760 | STRN3 | NM_014574.2 | 1282 | gacttgacggtaacaaatgatgc | 122915 | 527464 | see also 5757 line | | |
| 5761 | STRN3 | NM_014574.2 | 1555 | ctgtcattagctattagttctaa | 122928 | 527476 | see also 5757 line | | |
| 5762 | SCHIP1 | NM_014575.1 | 1243 | aagtctcccgtcgctgatcttct | 284106 | 705740 | - | - | - |
| 5763 | LMCD1 | NM_014583.2 | 328 | ttgctgatggactccaagtattc | 281347 | 753876 | - | molecular_function unknown | - |

Figure 46

| | | | | | | | | | |
|---|---|---|---|---|---|---|---|---|---|
| 5764 | ERO1L | NM_014584.1 | 405 | ggcttttcccaagactacaaaaa | 143300 | 705919 | - | - | - |
| 5765 | SLC40A1 | NM_014585.3 | 830 | atcctgatcatcactattgcaaa | 181285 | 574465 | - | - | hemochromatosis |
| 5766 | CGBP | NM_014593.1 | 872 | cccgggaatcgtacaagtacttc | 21009 | 736581 | transcription_regulator | zinc binding | - |
| 5767 | CGBP | NM_014593.1 | 873 | ccgggaatcgtacaagtacttcc | 21010 | 736582 | see also 5766 line | | |
| 5768 | MAGED2 | NM_014599.4 | 1061 | ttgaacgagcaggctattccttg | 340980 | 742401 | - | - | - |
| 5769 | HERC3 | NM_014606.1 | 1591 | ctccatgattctagaacagattt | 229761 | 735449 | - | ubiquitin-protein ligase | |
| 5770 | HERC3 | NM_014606.1 | 2203 | tggagccaacctgcagaatgtct | 229785 | 735477 | see also 5769 line | | |
| 5771 | HERC3 | NM_014606.1 | 2514 | tagagcacaactggtttcacttg | 229789 | 735484 | see also 5769 line | | |
| 5772 | CYFIP1 | NM_014608.1 | 624 | ccgctcagtttttacgtaaaatg | 341031 | 702124 | - | - | - |
| 5773 | CYFIP1 | NM_014608.1 | 1771 | aagtggttccaagaaaaccttga | 341055 | 702144 | see also 5772 line | | |
| 5774 | CYFIP1 | NM_014608.1 | 1850 | ttcttctacactcacttgataaa | 341057 | 702148 | see also 5772 line | | |
| 5775 | CYFIP1 | NM_014608.1 | 1853 | ttctacactcacttgataaattt | 341058 | 702149 | see also 5772 line | | |
| 5776 | CYFIP1 | NM_014608.1 | 3675 | ttcgcaagttccagattctcaat | 341087 | 702192 | see also 5772 line | | |
| 5777 | G2AN | NM_014610.3 | 128 | ctgtggatagaagcaactttaag | 202784 | 535424 | - | - | - |
| 5778 | G2AN | NM_014610.3 | 632 | gggataagatcaagaacctttc | 202798 | 535439 | see also 5777 line | | |
| 5779 | MDN1 | NM_014611.1 | 16717 | gggattctatcttggacattaaa | 90848 | 748883 | - | - | - |
| 5780 | MDN1 | NM_014611.1 | 16721 | ttctatcttggacattaaagtac | 90849 | 748884 | see also 5779 line | | |
| 5781 | ETEA | NM_014613.1 | 257 | cacaggatctacagctatgttgt | 341144 | 801436 | - | - | - |
| 5782 | ETEA | NM_014613.1 | 333 | ttccattccggtttacctattac | 341149 | 801440 | see also 5781 line | | |
| 5783 | ETEA | NM_014613.1 | 334 | tccattccggtttacctattaca | 341150 | 801441 | see also 5781 line | | |
| 5784 | ETEA | NM_014613.1 | 346 | tacctattacacgatacttgata | 341151 | 801442 | see also 5781 line | | |
| 5785 | ETEA | NM_014613.1 | 347 | acctattacacgatacttgatat | 341152 | 801443 | see also 5781 line | | |
| 5786 | ETEA | NM_014613.1 | 502 | aacgtacagccaggcacttaacg | 341156 | 801445 | see also 5781 line | | |
| 5787 | ETEA | NM_014613.1 | 506 | tacagccaggcacttaacgatgc | 341157 | 801446 | see also 5781 line | | |
| 5788 | ETEA | NM_014613.1 | 773 | cggctagaaggcctcattcaacc | 341161 | 801459 | see also 5781 line | | |
| 5789 | DBC1 | NM_014618.1 | 564 | accactccaggagctacctatcc | 341337 | 709725 | cell_cycle | - | bladder transitional cell carcinoma、 bladder cancer |
| 5790 | DBC1 | NM_014618.1 | 606 | aaggatttacaaccagatataaa | 341339 | 709727 | see also 5789 line | | |
| 5791 | DBC1 | NM_014618.1 | 607 | aggatttacaaccagatataaaa | 341340 | 709728 | see also 5789 line | | |
| 5792 | DBC1 | NM_014618.1 | 703 | gcccctcatgccggagtttcaaa | 341341 | 709729 | see also 5789 line | | |
| 5793 | DBC1 | NM_014618.1 | 705 | ccctcatgccggagtttcaaagg | 341343 | 709730 | see also 5789 line | | |
| 5794 | GRIK4 | NM_014619.2 | 1071 | aggtcttaccggccacattgaat | 244561 | 632423 | receptor_acitivity、 channel、 signal_transduction | potassium channel | - |
| 5795 | GRIK4 | NM_014619.2 | 1073 | gtcttaccggccacattgaattc | 244562 | 632424 | see also 5794 line | | |
| 5796 | NPHS2 | NM_014625.1 | 457 | gagtatgaaagagtaattatatt | 314621 | 538324 | - | - | nephrotic syndrome |

Figure 46

| 5797 | NPHS2 | NM_014625.1 | 749 | cacatcgatccctcactgaaatt | 314626 | 538329 | see also 5796 line | | |
| 5798 | KIAA0211 | NM_014630.1 | 2267 | ccctctatgcgccaaatctcagc | 29414 | 803171 | - | DNA binding | - |
| 5799 | MICAL2 | NM_014632.1 | 559 | ttgcgcactgccattgaacttgc | 221996 | 608530 | - | - | - |
| 5800 | MICAL2 | NM_014632.1 | 3394 | gactgcgatgaaggcaaatttta | 222028 | 608557 | see also 5799 line | | |
| 5801 | SH2BP1 | NM_014633.1 | 200 | cacacaactgcacatatggattg | 341452 | 528911 | - | - | - |
| 5802 | SH2BP1 | NM_014633.1 | 802 | ctgttctagaactcaacaataaa | 341479 | 528933 | see also 5801 line | | |
| 5803 | SH2BP1 | NM_014633.1 | 860 | ttccagagcctatactattgatc | 341481 | 528935 | see also 5801 line | | |
| 5804 | SH2BP1 | NM_014633.1 | 861 | tccagagcctatactattgatcc | 341482 | 528936 | see also 5801 line | | |
| 5805 | SH2BP1 | NM_014633.1 | 1121 | tggtttgggacaaatgtatattt | 341489 | 528948 | see also 5801 line | | |
| 5806 | SH2BP1 | NM_014633.1 | 1290 | aagaaggtcacagaacagtatcc | 341496 | 528952 | see also 5801 line | | |
| 5807 | SH2BP1 | NM_014633.1 | 2178 | atcagcgccgttcagatgtatga | 341525 | 528970 | see also 5801 line | | |
| 5808 | SH2BP1 | NM_014633.1 | 2180 | cagcgccgttcagatgtatgaaa | 341526 | 528971 | see also 5801 line | | |
| 5809 | SH2BP1 | NM_014633.1 | 2378 | agctacctctgtcctgaaagatg | 341537 | 528975 | see also 5801 line | | |
| 5810 | RALGPS1A | NM_014636.1 | 1775 | accccgagcacccagatatcttc | 296489 | 664472 | - | - | - |
| 5811 | RALGPS1A | NM_014636.1 | 1776 | ccccgagcacccagatatcttcc | 296490 | 664473 | see also 5810 line | | |
| 5812 | RALGPS1A | NM_014636.1 | 1910 | aggtacctgcaaaccttatgtca | 296499 | 664477 | see also 5810 line | | |
| 5813 | CHPPR | NM_014637.2 | 222 | gtctcgaagtatcgtaaggaaaa | 341568 | 722199 | - | - | - |
| 5814 | CHPPR | NM_014637.2 | 224 | ctcgaagtatcgtaaggaaaatt | 341569 | 722200 | see also 5813 line | | |
| 5815 | KIAA0222 | NM_014643.1 | 1335 | gtgcgggaacctgtttacaaacc | 29437 | 808153 | - | DNA binding | - |
| 5816 | KIAA0222 | NM_014643.1 | 2895 | agccgctagcatgcctaagaata | 29448 | 808170 | see also 5815 line | | |
| 5817 | KIAA0222 | NM_014643.1 | 2896 | gccgctagcatgcctaagaataa | 29449 | 808171 | see also 5815 line | | |
| 5818 | KIAA0222 | NM_014643.1 | 2897 | ccgctagcatgcctaagaataag | 29450 | 808172 | see also 5815 line | | |
| 5819 | KIAA0635 | NM_014645.1 | 1998 | atcagcaccgactttccataaaa | 341658 | 815832 | - | - | - |
| 5820 | KIAA0635 | NM_014645.1 | 2000 | cagcaccgactttccataaaaag | 341659 | 815833 | see also 5819 line | | |
| 5821 | LKAP | NM_014647.1 | 421 | agcagtcccacttcctgatattc | 63309 | 770338 | - | - | - |
| 5822 | LKAP | NM_014647.1 | 426 | tcccacttcctgatattcgttct | 63311 | 770339 | see also 5821 line | | |
| 5823 | LKAP | NM_014647.1 | 1536 | ttgtgtcaactgatgtcaatttt | 63336 | 770351 | see also 5821 line | | |
| 5824 | IPO13 | NM_014652.1 | 2324 | ctgccgagagtgcaagtatgacc | 269252 | 651249 | - | - | - |
| 5825 | IPO13 | NM_014652.1 | 3019 | atcctgatattgttgattcattt | 269260 | 651258 | see also 5824 line | | |
| 5826 | KIAA0406 | NM_014657.1 | 1768 | agcaagctgccatgatccttaat | 341842 | 738559 | - | - | - |
| 5827 | KIAA0406 | NM_014657.1 | 3206 | aggtgagggtgacctgaataaag | 341874 | 738593 | see also 5826 line | | |
| 5828 | KIAA0406 | NM_014657.1 | 3258 | gtcaaacagcccgtgaaattaca | 341877 | 738595 | see also 5826 line | | |
| 5829 | KIAA0377 | NM_014659.1 | 2309 | ggcgctatgatatcagtaagatc | 196990 | 589391 | phosphatase | - | - |
| 5830 | KIAA0377 | NM_014659.1 | 2310 | gcgctatgatatcagtaagatcc | 196991 | 589392 | see also 5829 line | | |

Figure 46

| 5831 | PHF14 | NM_014660.1 | 1719 | gccaaggagtgtagcttttgtga | 34774 | 453843 | - | - | - |
|------|-------|-------------|------|------------------------|-------|--------|---|---|---|
| 5832 | PHF14 | NM_014660.1 | 2542 | aaggaggcacacaaaagacatct | 34798 | 453865 | see also 5831 line | | |
| 5833 | KIAA0645 | NM_014662.1 | 392 | tggggatatgtggcgactaaaga | 316193 | 799114 | - | - | - |
| 5834 | KIAA0645 | NM_014662.1 | 394 | gggatatgtggcgactaaagaaa | 316194 | 799115 | see also 5833 line | | |
| 5835 | KIAA0645 | NM_014662.1 | 399 | atgtggcgactaaagaaaagttt | 316195 | 799116 | see also 5833 line | | |
| 5836 | KIAA0645 | NM_014662.1 | 554 | ttctacgtcggctatggtttaca | 316200 | 799119 | see also 5833 line | | |
| 5837 | KIAA0645 | NM_014662.1 | 557 | tacgtcggctatggtttacatat | 316201 | 799120 | see also 5833 line | | |
| 5838 | KIAA0645 | NM_014662.1 | 725 | tagaactttctatgatgcaaaat | 316204 | 799126 | see also 5833 line | | |
| 5839 | KIAA0645 | NM_014662.1 | 1418 | tcccatccaagtagattatgacg | 316230 | 799158 | see also 5833 line | | |
| 5840 | KIAA0645 | NM_014662.1 | 2520 | ctgagcagtagcccactctatag | 316246 | 799167 | see also 5833 line | | |
| 5841 | KIAA0645 | NM_014662.1 | 2783 | gtggaattacttagatcagtata | 316251 | 799176 | see also 5833 line | | |
| 5842 | JMJD2 | NM_014663.1 | 1001 | tacccgtcggtggattgagtacg | 29212 | 772815 | - | - | - |
| 5843 | GREB1 | NM_014668.2 | 2116 | ctctgtccagagggtgacattga | 280557 | 705869 | - | - | - |
| 5844 | GREB1 | NM_014668.2 | 2118 | ctgtccagagggtgacattgaca | 280558 | 705870 | see also 5843 line | | |
| 5845 | GREB1 | NM_014668.2 | 5517 | gtgtgacgatgtagacttcaacc | 280602 | 705901 | see also 5843 line | | |
| 5846 | KIAA0095 | NM_014669.1 | 1872 | tagtgacacaaagcctattatca | 342027 | 773258 | - | - | - |
| 5847 | KIAA0095 | NM_014669.1 | 2154 | cacctttttgacgagtatcata | 342034 | 773265 | see also 5846 line | | |
| 5848 | BZW1 | NM_014670.1 | 109 | cgctatcaggccagcgttttaaa | 56121 | 476372 | - | - | - |
| 5849 | KIAA0010 | NM_014671.1 | 874 | gtggtgtcagtgattgaacaaat | 229946 | 615312 | - | - | - |
| 5850 | KIAA0010 | NM_014671.1 | 989 | ctcgagttcctatagcaaaaatt | 229952 | 615321 | see also 5849 line | | |
| 5851 | KIAA0010 | NM_014671.1 | 1784 | gtcgaatcatcccactcttttac | 229973 | 615344 | see also 5849 line | | |
| 5852 | KIAA0010 | NM_014671.1 | 1785 | tcgaatcatcccactcttttacc | 229974 | 615345 | see also 5849 line | | |
| 5853 | KIAA0010 | NM_014671.1 | 2176 | aaccactggctgtcagaacaaga | 229995 | 615354 | see also 5849 line | | |
| 5854 | KIAA0103 | NM_014673.2 | 413 | agcgtaagattgccattcgaaaa | 324430 | 689647 | - | - | - |
| 5855 | KIAA0103 | NM_014673.2 | 414 | gcgtaagattgccattcgaaaag | 324431 | 689648 | see also 5854 line | | |
| 5856 | KIAA0103 | NM_014673.2 | 749 | gtcatattgcttctaatccaaaa | 324440 | 689657 | see also 5854 line | | |
| 5857 | EDEM1 | NM_014674.1 | 1317 | atgtcaacgtgaacatgttcagt | 142701 | 547736 | - | mannosyl-oligosaccharide 1、2-alpha-mannosidase | - |
| 5858 | PUM1 | NM_014676.1 | 328 | acgctatggtggactacttcttt | 53643 | 474986 | - | - | - |
| 5859 | PUM1 | NM_014676.1 | 529 | agctcttgccaggtaaaaagttt | 53651 | 474996 | see also 5858 line | | |
| 5860 | PUM1 | NM_014676.1 | 691 | atgtgaacagtgaggtcaattct | 53653 | 474999 | see also 5858 line | | |
| 5861 | PUM1 | NM_014676.1 | 693 | gtgaacagtgaggtcaattctgt | 53654 | 475000 | see also 5858 line | | |
| 5862 | PUM1 | NM_014676.1 | 696 | aacagtgaggtcaattctgtact | 53655 | 475001 | see also 5858 line | | |
| 5863 | PUM1 | NM_014676.1 | 858 | aagaagaacaagggtacgtttga | 53658 | 475005 | see also 5858 line | | |
| 5864 | PUM1 | NM_014676.1 | 869 | gggtacgtttgatggagataagc | 53659 | 475007 | see also 5858 line | | |
| 5865 | PUM1 | NM_014676.1 | 872 | tacgtttgatggagataagctag | 53660 | 475008 | see also 5858 line | | |

EP 1 752 536 A1

Figure 46

| | | | | | | | | |
|---|---|---|---|---|---|---|---|---|
| 5866 | PUM1 | NM_014676.1 | 881 | tggagataagctaggagatttga | 53661 | 475009 | see also 5858 line | |
| 5867 | PUM1 | NM_014676.1 | 958 | ttgatgcagacgtcaaagattt | 53665 | 475010 | see also 5858 line | |
| 5868 | PUM1 | NM_014676.1 | 2228 | tggcagcagtgacctttataaga | 53683 | 475022 | see also 5858 line | |
| 5869 | PUM1 | NM_014676.1 | 2535 | tcctctcgtttgcgatatggaat | 53687 | 475027 | see also 5858 line | |
| 5870 | PUM1 | NM_014676.1 | 2537 | ctctcgtttgcgatatggaatgt | 53688 | 475028 | see also 5858 line | |
| 5871 | PUM1 | NM_014676.1 | 2724 | gagcgccagcttgcttcaatga | 53693 | 475036 | see also 5858 line | |
| 5872 | RIMS2 | NM_014677.1 | 1699 | ctgatactgtaggacatccttaga | 317087 | 539848 | - | |
| 5873 | RIMS2 | NM_014677.1 | 2091 | gggaggccaaggaatccttatgt | 317098 | 539859 | see also 5872 line | |
| 5874 | RIMS2 | NM_014677.1 | 2096 | gccaaggaatccttatgttaaaa | 317099 | 539861 | see also 5872 line | |
| 5875 | RIMS2 | NM_014677.1 | 2622 | gggtctcctcatcgagtagatgt | 317113 | 539874 | see also 5872 line | |
| 5876 | RIMS2 | NM_014677.1 | 2624 | gtctcctcatcgagtagagtta | 317114 | 539875 | see also 5872 line | |
| 5877 | RIMS2 | NM_014677.1 | 2626 | ctcctcatcgagtagatgttata | 317115 | 539876 | see also 5872 line | |
| 5878 | RIMS2 | NM_014677.1 | 2627 | tcctcatcgagtagatgttatag | 317116 | 539877 | see also 5872 line | |
| 5879 | RIMS2 | NM_014677.1 | 2632 | atcgagtagatgttataggaagg | 317117 | 539878 | see also 5872 line | |
| 5880 | RIMS2 | NM_014677.1 | 3013 | tccagacaagcccatcaagtact | 317131 | 539880 | see also 5872 line | |
| 5881 | KIAA0092 | NM_014679.1 | 878 | tgccgtgctagccaatgttcagc | 342132 | 726678 | - | |
| 5882 | KIAA0092 | NM_014679.1 | 1078 | aggatgaatttgggcaaatgagc | 342142 | 726689 | see also 5881 line | |
| 5883 | KIAA0092 | NM_014679.1 | 1088 | tgggcaaatgagctttgatcacc | 342143 | 726690 | see also 5881 line | |
| 5884 | ULK2 | NM_014683.2 | 580 | ggcaccgccagaaaacctgattgg | 107195 | 517407 | kinase_related | |
| 5885 | ULK2 | NM_014683.2 | 1051 | aggttattatgtctcaacattat | 107215 | 517420 | see also 5884 line | |
| 5886 | ULK2 | NM_014683.2 | 1187 | caggagcttaatgcctagtattc | 107221 | 517425 | see also 5884 line | |
| 5887 | ULK2 | NM_014683.2 | 1188 | aggagcttaatgcctagtattcc | 107222 | 517426 | see also 5884 line | |
| 5888 | ULK2 | NM_014683.2 | 1543 | gtgacacggatgacttgttttg | 107228 | 517436 | see also 5884 line | |
| 5889 | ULK2 | NM_014683.2 | 3379 | ctgagagaaactcatctataat | 107251 | 517488 | see also 5884 line | |
| 5890 | ULK2 | NM_014683.2 | 3384 | gagaaactcatctataattgtgc | 107253 | 517489 | see also 5884 line | |
| 5891 | AQR | NM_014691.1 | 540 | ctgtatggtgaatgagaagttta | 342218 | 699742 | - | |
| 5892 | AQR | NM_014691.1 | 795 | ggcacgattggaattagaattaa | 342227 | 699750 | see also 5891 line | |
| 5893 | AQR | NM_014691.1 | 2140 | ttggatccaaaccagtatcaaca | 342273 | 699787 | see also 5891 line | |
| 5894 | AQR | NM_014691.1 | 2931 | cagcaggtatggaagagttaatt | 342311 | 699816 | see also 5891 line | |
| 5895 | KIAA0296 | NM_014699.1 | 1065 | ggctgaatcagtagtgaacttca | 29463 | 778365 | - | DNA binding |
| 5896 | KIAA0296 | NM_014699.1 | 1067 | ctgaatcagtagtgaacttcaca | 29464 | 778366 | see also 5895 line | |
| 5897 | KIAA0562 | NM_014704.1 | 1771 | aagagccataaaggaccattgtga | 342607 | 797782 | - | transcription_regulator, cell_cycle |
| 5898 | HDAC9 | NM_014707.1 | 737 | ctccatcctacaagtacacatta | 113726 | 525966 | see also 5898 line | |
| 5899 | HDAC9 | NM_014707.1 | 823 | aaggtgcggtccaggttaaaaca | 113728 | 525972 | see also 5898 line | |
| 5900 | HDAC9 | NM_014707.1 | 1092 | ttccatgaacctgctaagtcttt | 113739 | 525978 | see also 5898 line | |
| 5901 | HDAC9 | NM_014707.1 | 1844 | ctggggagcaggctgctttatg | 113750 | 525994 | see also 5898 line | |

Figure 46

| | | | | | | | | molecular_function unknown | |
|---|---|---|---|---|---|---|---|---|---|
| 5902 | HDAC9 | NM_014707.1 | 1868 | aacaggtaataggcaaagattta | 113751 | 525997 | see also 5898 line | | |
| 5903 | HDAC9 | NM_014707.1 | 1870 | caggtaataggcaaagatttagc | 113752 | 525998 | see also 5898 line | | |
| 5904 | CP110 | NM_014711.3 | 3234 | ctgttcataggctacttagtaga | 278819 | 807694 | - | molecular_function unknown | - |
| 5905 | CP110 | NM_014711.3 | 3291 | cggccaaatgttgcgacaattta | 278821 | 807700 | see also 5904 line | | |
| 5906 | RICS | NM_014715.2 | 3145 | cagcgcacgcacagaactaatcg | 342742 | 795788 | - | - | - |
| 5907 | RICS | NM_014715.2 | 3554 | atgccaccagcagttcaaattat | 342747 | 795791 | see also 5906 line | | |
| 5908 | RICS | NM_014715.2 | 5309 | ctgttgtgtcccagtatgataac | 342782 | 795823 | see also 5906 line | | |
| 5909 | CENTB1 | NM_014716.1 | 414 | atggcggagtgtctggaaaaatt | 24000 | 457133 | - | - | - |
| 5910 | CENTB1 | NM_014716.1 | 415 | tggcggagtgtctggaaaaattc | 24001 | 457134 | see also 5909 line | | |
| 5911 | KIAA0390 | NM_014717.1 | 2862 | gagcaacggtgtgaatttccaag | 342797 | 772879 | - | - | - |
| 5912 | CLSTN3 | NM_014718.1 | 2849 | atcctgaggcaggctcgttatcg | 140826 | 546489 | - | - | - |
| 5913 | SLK | NM_014720.1 | 744 | tagagattgacatattagcatct | 39504 | 468412 | kinase_related | - | - |
| 5914 | SLK | NM_014720.1 | 788 | aagcttctagatgccttctatta | 39508 | 468415 | see also 5913 line | | |
| 5915 | STARD8 | NM_014725.2 | 768 | ttcctcaagcaccttgaatctct | 298327 | 815550 | - | - | - |
| 5916 | STARD8 | NM_014725.2 | 3344 | ttctcctgactggtacaacaaag | 298343 | 815573 | see also 5915 line | | |
| 5917 | STARD8 | NM_014725.2 | 3347 | tcctgactggtacaacaaagtct | 298344 | 815574 | see also 5915 line | | |
| 5918 | STARD8 | NM_014725.2 | 3351 | gactggtacaacaaagtctttgg | 298345 | 815575 | see also 5915 line | | |
| 5919 | TOX | NM_014729.1 | 308 | ccctactattgcaacaagtttga | 42850 | 469867 | - | - | - |
| 5920 | TOX | NM_014729.1 | 1621 | tactcttcaacccgactatcaga | 42877 | 469885 | see also 5919 line | | |
| 5921 | TOX | NM_014729.1 | 1629 | aacccgactatcagactattatc | 42879 | 469886 | see also 5919 line | | |
| 5922 | TOX | NM_014729.1 | 1630 | acccgactatcagactattatca | 42880 | 469887 | see also 5919 line | | |
| 5923 | ZFYVE16 | NM_014733.1 | 3798 | ccctgggcaaaggttttcctat | 161339 | 556975 | - | - | - |
| 5924 | PHF16 | NM_014735.2 | 736 | ctggcttcaggaactcaatgaag | 34839 | 816722 | transcription_regulator | - | - |
| 5925 | RASSF2 | NM_014737.1 | 1005 | acgacgtggcccagtatataaag | 314993 | 679317 | signal_transduction | - | - |
| 5926 | RASSF2 | NM_014737.1 | 1008 | acgtggcccagtatataaagttc | 314994 | 679318 | see also 5925 line | | |
| 5927 | KIAA0195 | NM_014738.1 | 3152 | gtgtgagatgataaagatcatgc | 86479 | 731802 | - | plasma membrane cation-transporting ATPase | - |
| 5928 | BTF | NM_014739.1 | 388 | gtctcgtagtagagatcgtatgt | 275909 | 771018 | transcription_regulator, apoptosis | - | - |
| 5929 | BTF | NM_014739.1 | 475 | aggaaggggtagagggtattatc | 275916 | 771024 | see also 5928 line | | |
| 5930 | BTF | NM_014739.1 | 1296 | tcctgcttgataggggtaatacc | 275938 | 771038 | see also 5928 line | | |
| 5931 | BTF | NM_014739.1 | 1460 | gaggagacagaggattatagaca | 275944 | 771042 | see also 5928 line | | |
| 5932 | BTF | NM_014739.1 | 1806 | atctggatgcacgagaaaagtct | 275957 | 771049 | see also 5928 line | | |
| 5933 | BTF | NM_014739.1 | 1876 | ttctcaccgtcctgaagtcaaac | 275962 | 771052 | see also 5928 line | | |
| 5934 | BTF | NM_014739.1 | 2637 | cacgaggaacctttttcgaatt | 275982 | 771065 | see also 5928 line | | |

EP 1 752 536 A1

Figure 46

| ID | Gene | Accession | Pos | Sequence | | | Note | Function | Disease |
|---|---|---|---|---|---|---|---|---|---|
| 5935 | BTF | NM_014739.1 | 2695 | gacaaatactggtccaaacaact | 275984 | 771068 | see also 5928 line | | |
| 5936 | BTF | NM_014739.1 | 2703 | ctggtccaaacaactcaaatact | 275986 | 771069 | see also 5928 line | | |
| 5937 | BTF | NM_014739.1 | 2806 | cagagatgatgtgtggattatt | 275987 | 771072 | see also 5928 line | | |
| 5938 | BTF | NM_014739.1 | 2839 | aggaagaggtcgtgtactttc | 275988 | 771074 | see also 5928 line | | |
| 5939 | TM9SF4 | NM_014742.1 | 1771 | gtggtacatgaaccgatttgtgg | 275345 | 655795 | | | |
| 5940 | TBC1D5 | NM_014744.1 | 2168 | aagaacgtttctgaaatgcagt | 342933 | 732838 | | | |
| 5941 | SNX17 | NM_014748.2 | 794 | gccttcggagtcaagagtataag | 126326 | 530202 | | receptor binding | |
| 5942 | SNX17 | NM_014748.2 | 797 | ttcggagtcaagagtataagatt | 126327 | 530203 | see also 5941 line | | |
| 5943 | DLG7 | NM_014750.3 | 1630 | tggtcaaacaagactccttatga | 279332 | 753583 | | | |
| 5944 | PTDSS1 | NM_014754.1 | 395 | cagccttatggcgaatggtttt | 219010 | 671362 | | | |
| 5945 | PTDSS1 | NM_014754.1 | 1098 | tcccacagtgagacagtactacg | 219032 | 671377 | see also 5944 line | | |
| 5946 | TRIP-Br2 | NM_014755.1 | 342 | tgggctggaaggcaaaatcgtgt | 300964 | 668649 | | | |
| 5947 | TRIP-Br2 | NM_014755.1 | 396 | caccttacagcgccagactatct | 300966 | 668652 | see also 5946 line | | |
| 5948 | TRIP-Br2 | NM_014755.1 | 401 | tacagcggcagactatcttcaac | 300967 | 668653 | see also 5946 line | | |
| 5949 | DHCR24 | NM_014762.1 | 966 | gcccagcaagctgaatagcattg | 226559 | 672141 | | oxidoreductase | desmosterolosis, Alzheimer disease |
| 5950 | DHCR24 | NM_014762.1 | 1610 | ccgaggtacgacaagatcgc | 226568 | 672151 | see also 5949 line | | |
| 5951 | KIAA0669 | NM_014779.1 | 2918 | cagtctggccacctgtattca | 5644 | 441385 | | | |
| 5952 | KIAA0669 | NM_014779.1 | 3205 | atgcactgttaaaatctcttca | 5647 | 441387 | see also 5951 line | | ataxia telangiectasia |
| 5953 | RB1CC1 | NM_014781.1 | 1129 | tggagtgcctaaccagacatagt | 343283 | 677374 | | | |
| 5954 | RB1CC1 | NM_014781.1 | 1131 | gagtgcctaaccagacatagtta | 343284 | 677375 | see also 5953 line | | |
| 5955 | RB1CC1 | NM_014781.1 | 1133 | gtgcctaaccagacatagttaca | 343285 | 677376 | see also 5953 line | | |
| 5956 | RB1CC1 | NM_014781.1 | 1443 | gactggataaatgttcaagatag | 343293 | 677382 | see also 5953 line | | |
| 5957 | RB1CC1 | NM_014781.1 | 1568 | tgccaaactgataatcagaata | 343299 | 677389 | see also 5953 line | | |
| 5958 | RB1CC1 | NM_014781.1 | 1681 | ttgctcaggagtttttagctaat | 343302 | 677394 | see also 5953 line | | |
| 5959 | RB1CC1 | NM_014781.1 | 1682 | tgctcaggagtttttagctaatc | 343303 | 677395 | see also 5953 line | | |
| 5960 | RB1CC1 | NM_014781.1 | 1687 | aggagtttttagctaatcagaag | 343304 | 677396 | see also 5953 line | | |
| 5961 | RB1CC1 | NM_014781.1 | 1939 | tcgtaatagagctgttagaaaga | 343315 | 677405 | see also 5953 line | | |
| 5962 | RB1CC1 | NM_014781.1 | 1982 | tagtacagttcctcagatgtact | 343316 | 677406 | see also 5953 line | | |
| 5963 | ALEX2 | NM_014782.3 | 517 | ctggtactgtgtctacaaataca | 343399 | 721687 | | | |
| 5964 | ALEX2 | NM_014782.3 | 1797 | aagccttaatggccatgaataac | 343406 | 721721 | see also 5963 line | | |
| 5965 | ARHGAP11A | NM_014783.2 | 3235 | ttcgtcagtccgtcagaagaatt | 343478 | 806036 | | | |
| 5966 | ARHGAP11A | NM_014783.2 | 3238 | gtcagtccgtcagaagaattaat | 343479 | 806038 | see also 5965 line | | |
| 5967 | ARHGAP11A | NM_014783.2 | 3243 | tccgtcagaagaattaattcttt | 343481 | 806039 | see also 5965 line | | |
| 5968 | KIAA0258 | NM_014785.1 | 341 | gagagaggggccagtgtatccttc | 343500 | 780031 | | | |

Figure 46

| ID | Gene | Accession | Pos | Sequence | ID1 | ID2 | Note | Annotation | Disease |
|---|---|---|---|---|---|---|---|---|---|
| 5969 | KIAA0258 | NM_014785.1 | 347 | ggccagtgtatcctttctactcc | 343501 | 780032 | see also 5968 line | | |
| 5970 | KIAA0258 | NM_014785.1 | 402 | atcctggagagtccaaatcatac | 343505 | 780036 | see also 5968 line | | |
| 5971 | DNAJC6 | NM_014787.1 | 677 | ctggttggctatgttcatttt | 196163 | 812979 | - | | |
| 5972 | DNAJC6 | NM_014787.1 | 2802 | ctgggcaaccctatgaacaatac | 196203 | 813015 | see also 5971 line | | |
| 5973 | DNAJC6 | NM_014787.1 | 2817 | aacaatacgcaaagatgatttc | 196204 | 813018 | see also 5971 line | | |
| 5974 | ZFHX1B | NM_014795.2 | 511 | cggtgcaagaggcgcaaacaagc | 14245 | 449939 | transcription_regulator, phosphatase | transcriptional repressor | Graves' disease |
| 5975 | ZFHX1B | NM_014795.2 | 544 | aggaaaaacgtggtgaactatga | 14246 | 449941 | see also 5974 line | | |
| 5976 | ZFHX1B | NM_014795.2 | 1174 | taccgccacgagaagaatgaaga | 14255 | 449951 | see also 5974 line | | |
| 5977 | ZFHX1B | NM_014795.2 | 1280 | cagatcagcaccaaatgctaac | 14256 | 449953 | see also 5974 line | | |
| 5978 | ZFHX1B | NM_014795.2 | 1496 | ctgtaaattggccgaatgagaaac | 14259 | 449959 | see also 5974 line | | |
| 5979 | ZFHX1B | NM_014795.2 | 1502 | atggccgaatgagaaaccaatatc | 14260 | 449960 | see also 5974 line | | |
| 5980 | ZFHX1B | NM_014795.2 | 1503 | tggccgaatgagaaaacaatatca | 14261 | 449961 | see also 5974 line | | |
| 5981 | ZFHX1B | NM_014795.2 | 1669 | ttcaatgacttataagttcttat | 14268 | 449970 | see also 5974 line | | |
| 5982 | ZFHX1B | NM_014795.2 | 2459 | aagcatactatgctatgaacatg | 14296 | 449975 | see also 5974 line | | |
| 5983 | ZFHX1B | NM_014795.2 | 2618 | agccgttagctccaacagtaac | 14299 | 449977 | see also 5974 line | | |
| 5984 | ZFHX1B | NM_014795.2 | 2619 | gccgttagctccaacagtaacc | 14300 | 449978 | see also 5974 line | | |
| 5985 | ZFHX1B | NM_014795.2 | 2667 | caggtctctgtaaaacctatgg | 14303 | 449982 | see also 5974 line | | |
| 5986 | ZFHX1B | NM_014795.2 | 2714 | cagaactccacaacagtgttacg | 14305 | 449986 | see also 5974 line | | |
| 5987 | ZFHX1B | NM_014795.2 | 2745 | tcctcctctcaggctaacaaaac | 14309 | 449988 | see also 5974 line | | |
| 5988 | ZFHX1B | NM_014795.2 | 3380 | atggagcacaagactacatgtca | 14322 | 450005 | see also 5974 line | | |
| 5989 | ZFHX1B | NM_014795.2 | 3469 | ggcatgtatgcatgtgacttatg | 14325 | 450006 | see also 5974 line | | |
| 5990 | ZFHX1B | NM_014795.2 | 3516 | ttccttctgggacataaatacg | 14328 | 450007 | see also 5974 line | | |
| 5991 | KIAA0748 | NM_014796.1 | 960 | tactcctatgtgtctaagacacc | 343661 | 809351 | - | | |
| 5992 | ZNF450 | NM_014797.1 | 285 | tactttaatcgtgagaatgtac | 45614 | 471101 | - | protein binding | |
| 5993 | ZNF450 | NM_014797.1 | 740 | cagttcagaatagacaaaaacttt | 45625 | 471121 | see also 5992 line | | |
| 5994 | ELMO1 | NM_014800.8 | 375 | gtctcttgtccaaccatgaatatt | 126479 | 673106 | apoptosis, signal_transduction | | |
| 5995 | ELMO1 | NM_014800.8 | 1845 | cccgattttggaactaaaggaga | 126515 | 673151 | see also 5994 line | | |
| 5996 | KIAA0528 | NM_014802.1 | 1658 | cagctcatctcacatattgctat | 265117 | 648145 | - | | |
| 5997 | KIAA0528 | NM_014802.1 | 1661 | ctcatctcacatattgctataac | 265118 | 648147 | see also 5996 line | | |
| 5998 | KIAA0753 | NM_014804.1 | 652 | cagatcttactgtgccaaattcg | 343736 | 737451 | - | | |
| 5999 | KIAA0753 | NM_014804.1 | 1050 | atgtttgtcactcagtttactga | 343741 | 737459 | see also 5998 line | | |
| 6000 | FARP2 | NM_014808.1 | 124 | gagatagaaggaacatacagagt | 322798 | 756918 | - | | |
| 6001 | FARP2 | NM_014808.1 | 402 | ctggatttggcttgaacctatga | 322807 | 756923 | see also 6000 line | | |
| 6002 | FARP2 | NM_014808.1 | 416 | aacctatgaaaccatcattagg | 322808 | 756924 | see also 6000 line | | |
| 6003 | FARP2 | NM_014808.1 | 1129 | ggctcctccttcagatacagtgg | 322827 | 756940 | see also 6000 line | | |

Figure 46

| Seq ID | Gene | Accession | Pos | Sequence | ID1 | ID2 | Cross-reference | Function |
|---|---|---|---|---|---|---|---|---|
| 6004 | FARP2 | NM_014808.1 | 3134 | tccgggctgagagcaagtacaca | 322852 | 756980 | see also 6000 line | |
| 6005 | p44S10 | NM_014814.1 | 784 | tccagcagttcggcagtatctgt | 192063 | 585734 | - | proteasome ATPase |
| 6006 | p44S10 | NM_014814.1 | 786 | cagcagttcggcagtatctgtt | 192064 | 585735 | see also 6005 line | |
| 6007 | p44S10 | NM_014814.1 | 900 | atcgatactatgtaagagaaatg | 192070 | 585744 | see also 6005 line | |
| 6008 | p44S10 | NM_014814.1 | 939 | gtcagctgctggaatcatatagg | 192073 | 585745 | see also 6005 line | |
| 6009 | p44S10 | NM_014814.1 | 1090 | aaccaacagacctgatagcaaga | 192079 | 585750 | see also 6005 line | |
| 6010 | TRAP100 | NM_014815.1 | 1071 | atgtcaactgtgctttgagttc | 102987 | 637960 | - | receptor_acitivity, transcription_regulator, signal_transduction |
| 6011 | TRAP100 | NM_014815.1 | 2113 | gacagccacgcagatcaagttc | 102999 | 637975 | see also 6010 line | |
| 6012 | PJA2 | NM_014819.1 | 285 | aggtcggctcgtgatgactatg | 35251 | 538775 | - | |
| 6013 | PJA2 | NM_014819.1 | 1617 | tccttggttacagtacaatgaag | 35295 | 538813 | see also 6012 line | |
| 6014 | TOMM70A | NM_014820.1 | 1541 | caggcattaacagatcaacaaca | 323009 | 687549 | - | |
| 6015 | TOMM70A | NM_014820.1 | 1551 | cagatcaacaacagtttggtaaa | 323011 | 687550 | see also 6014 line | |
| 6016 | TOMM70A | NM_014820.1 | 1881 | ttgcaagaatacggattaaaa | 323019 | 687557 | see also 6014 line | smolecular_function_unknown |
| 6017 | KIAA0317 | NM_014821.1 | 589 | cgccgaggggaccggactattta | 230032 | 614872 | - | |
| 6018 | KIAA0317 | NM_014821.1 | 590 | gccgaggggaccggactatttat | 230033 | 614873 | see also 6017 line | |
| 6019 | KIAA0317 | NM_014821.1 | 591 | ccgaggggaccggactatttatg | 230034 | 614874 | see also 6017 line | |
| 6020 | KIAA0317 | NM_014821.1 | 596 | gggaccggactatttatgactac | 230035 | 614875 | see also 6017 line | |
| 6021 | KIAA0317 | NM_014821.1 | 611 | atgactacgtgcgcggggaaattac | 230036 | 614876 | see also 6017 line | |
| 6022 | KIAA0317 | NM_014821.1 | 927 | tccctactacaaatttttcaac | 230048 | 614888 | see also 6017 line | |
| 6023 | KIAA0317 | NM_014821.1 | 1817 | agcttcggcaggtacatatgaaa | 230066 | 614906 | see also 6017 line | |
| 6024 | KIAA0317 | NM_014821.1 | 2243 | tgcgtatgcattacaagtacttt | 230075 | 614928 | see also 6017 line | |
| 6025 | KIAA0317 | NM_014821.1 | 2244 | gcgtatgcattacaagtacttg | 230076 | 614929 | see also 6017 line | |
| 6026 | KIAA0769 | NM_014824.1 | 292 | ctgatgatcggaatgattacagg | 226967 | 815385 | - | |
| 6027 | KIAA0769 | NM_014824.1 | 598 | aacttagtcttttcaatcaaga | 226980 | 815399 | see also 6026 line | |
| 6028 | KIAA0769 | NM_014824.1 | 973 | tccagccttgtgacagtgatact | 226997 | 815416 | see also 6026 line | |
| 6029 | KIAA0769 | NM_014824.1 | 975 | cagccttgtgacagtgatactag | 226998 | 815417 | see also 6026 line | |
| 6030 | KIAA0769 | NM_014824.1 | 1587 | gactgggtaaaggctcgaaattaa | 227020 | 815446 | see also 6026 line | |
| 6031 | KIAA0769 | NM_014824.1 | 1589 | ctgggtaaaggctcgaaataaag | 227021 | 815447 | see also 6026 line | |
| 6032 | DDX46 | NM_014829.1 | 1363 | atgtctgacgagatttgattgg | 59268 | 483209 | - | |
| 6033 | DDX46 | NM_014829.1 | 1623 | aagaggtgctgaaattattgttt | 59275 | 483214 | see also 6032 line | |
| 6034 | DDX46 | NM_014829.1 | 1644 | ttgcacctggtcgaatgattg | 59277 | 483215 | see also 6032 line | |
| 6035 | DDX46 | NM_014829.1 | 1653 | tgttcgaatgattgacatgttag | 59279 | 483219 | see also 6032 line | |

Figure 46

| ID | Gene | Accession | | Sequence | | | Notes | Function | Disease |
|---|---|---|---|---|---|---|---|---|---|
| 6036 | DDX46 | NM_014829.1 | 1681 | aacagtgtcggtcacaaatct | 59281 | 483221 | see also 6032 line | | |
| 6037 | DDX46 | NM_014829.1 | 1683 | cagtggtcgggtcacaaatcttc | 59282 | 483222 | see also 6032 line | | |
| 6038 | DDX46 | NM_014829.1 | 1996 | gagtcaggatcgtcattatatt | 59291 | 483229 | see also 6032 line | | |
| 6039 | DDX46 | NM_014829.1 | 1998 | gtcaggatcgtcattatattg | 59292 | 483230 | see also 6032 line | | |
| 6040 | KIAA0352 | NM_014830.1 | 815 | ctgccagccatacaaagttcaaa | 29491 | 811512 | | protein binding | |
| 6041 | TBC1D4 | NM_014832.1 | 1679 | gagtgccaagacgcagattaaac | 344327 | 783066 | | | |
| 6042 | TBC1D4 | NM_014832.1 | 1682 | tgccaagacgcagattaaactgt | 344328 | 783067 | see also 6041 line | | |
| 6043 | OSBPL2 | NM_014835.2 | 827 | ctggagctgctcaaacataatga | 303491 | 672296 | | | |
| 6044 | PRG1 | NM_014839.2 | 554 | ttgctggacctgcaattacgatt | 344462 | 797651 | | | |
| 6045 | PRG1 | NM_014839.2 | 731 | cagctcattacagatatcata | 344470 | 797658 | see also 6044 line | | |
| 6046 | SNAP91 | NM_014841.1 | 2070 | cactgctgacctcttatctgtgg | 328283 | 693886 | | | |
| 6047 | KIAA0274 | NM_014845.4 | 231 | tagagctagatactttctagttg | 344565 | 749639 | | | |
| 6048 | KIAA0274 | NM_014845.4 | 644 | acctatctagcaattttacttt | 344584 | 749653 | see also 6047 line | | |
| 6049 | KIAA0196 | NM_014846.1 | 2736 | tggtcgactctgcgagagaaatcc | 344735 | 770311 | | | |
| 6050 | SRGAP2 | NM_014850.1 | 1590 | ctcagtgtatagccataaaactct | 298263 | 534061 | | | |
| 6051 | SRGAP2 | NM_014850.1 | 1592 | cagtgtatagccataaactcttt | 298264 | 534062 | see also 6050 line | | |
| 6052 | SRGAP2 | NM_014850.1 | 1639 | aaggattcaggacaagctatacc | 298265 | 534065 | see also 6050 line | | |
| 6053 | KIAA0682 | NM_014852.1 | 973 | gcgggagcccgttcaatgtca | 52316 | 478161 | | | |
| 6054 | HHL | NM_014857.2 | 1066 | ctgtgcctaaggatgagagataaa | 344874 | 705286 | | | |
| 6055 | HHL | NM_014857.2 | 1148 | ttctaacaagaattagctattg | 344875 | 705292 | see also 6054 line | | |
| 6056 | HHL | NM_014857.2 | 1196 | cccaggtcgaaacgtgaagaaca | 344877 | 705293 | see also 6054 line | | |
| 6057 | HHL | NM_014857.2 | 1198 | caggtcgaaacgtgaagaacagt | 344878 | 705294 | see also 6054 line | | |
| 6058 | HHL | NM_014857.2 | 1913 | gagtgttattactcgagatattc | 344890 | 705308 | see also 6054 line | | |
| 6059 | HHL | NM_014857.2 | 1925 | tcgagatattcatcgtacattc | 344891 | 705309 | see also 6054 line | | |
| 6060 | HHL | NM_014857.2 | 2511 | caggcttgcaatattaaagtacc | 344904 | 705323 | see also 6054 line | | |
| 6061 | KIAA0672 | NM_014859.1 | 940 | tactttcaaacgctaatagaagt | 344914 | 785189 | | | |
| 6062 | ARNT2 | NM_014862.1 | 2119 | ggggactggcaactataacacatcg | 976 | 441785 | transcription_regulator, signal_transduction | transcription factor | |
| 6063 | GALNAC4S-6ST | NM_014863.1 | 721 | tggacagtaagcagatgaacttg | 217472 | 605454 | | | |
| 6064 | CNAP1 | NM_014865.1 | 2864 | gtccggaagccgtgcttaatgc | 344995 | 760941 | cell_cycle | | Alzheimer disease |
| 6065 | CNAP1 | NM_014865.1 | 4623 | agcctgaggcaaggctataata | 345007 | 760965 | see also 6064 line | | |
| 6066 | CNAP1 | NM_014865.1 | 4628 | gagggcaaggctataatagatga | 345008 | 760967 | see also 6064 line | | |
| 6067 | USP52 | NM_014871.1 | 516 | ttggagcgctactcatcctttca | 190206 | 581721 | | | |
| 6068 | ZBTB5 | NM_014872.1 | 1273 | gtggtcagtgccgagaagataga | 137145 | 783297 | | | |
| 6069 | KIAA0205 | NM_014873.1 | 319 | ttgctattccatcctacatctgc | 216334 | 603503 | | acyltransferase | |

Figure 46

| 6070 | KIAA0205 | NM_014873.1 | 743 | gagcagagatcgaaaatggattg | 216345 | 603519 | see also 6069 line | | |
|------|----------|-------------|------|-------------------------|--------|--------|--------------------|---|---|
| 6071 | KIAA0205 | NM_014873.1 | 970 | gcctccagtggataatagataca | 216350 | 603527 | see also 6069 line | | |
| 6072 | KIAA0205 | NM_014873.1 | 972 | ctccagtggataatagatacaac | 216351 | 603528 | see also 6069 line | | |
| 6073 | KIAA0205 | NM_014873.1 | 973 | tccagtggataatagatacaacg | 216352 | 603529 | see also 6069 line | | |
| 6074 | MFN2 | NM_014874.1 | 1569 | agctgcgaattaagcagattacg | 345082 | 537549 | - | - | - |
| 6075 | DCLRE1A | NM_014881.1 | 3710 | ttctcccaatgatgcaaattaat | 345219 | 669769 | - | - | - |
| 6076 | KIAA0053 | NM_014882.1 | 1937 | gtggtgaggctcaatgaagaact | 345246 | 789186 | - | - | - |
| 6077 | FAM13A1 | NM_014883.1 | 1614 | gccctgaggacattaaggatatg | 345284 | 679109 | - | - | - |
| 6078 | RBM16 | NM_014892.1 | 490 | gccctggggatgacaagagtaaa | 53975 | 485735 | - | - | - |
| 6079 | RBM16 | NM_014892.1 | 499 | atgacaagagtaaaatagtgaga | 53977 | 485736 | see also 6078 line | | |
| 6080 | RBM16 | NM_014892.1 | 1369 | cggttcgctcaagatcaagaaca | 54002 | 485750 | see also 6078 line | | |
| 6081 | RBM16 | NM_014892.1 | 3755 | tcctcatgctcgggtttttgatt | 54057 | 485776 | see also 6078 line | | |
| 6082 | RBM16 | NM_014892.1 | 3760 | atgctcgggttttttgattatttt | 54058 | 485777 | see also 6078 line | | |
| 6083 | RHOBTB3 | NM_014899.1 | 1583 | gccgatgcttgccgatgttgtct | 135192 | 539783 | - | - | - |
| 6084 | RNF44 | NM_014901.3 | 889 | cagcatgctccgtgatgttcagt | 345444 | 750546 | - | - | - |
| 6085 | CPEB3 | NM_014912.1 | 1050 | aacgctttccggaccgataatgg | 58824 | 482138 | - | - | - |
| 6086 | CPEB3 | NM_014912.1 | 1061 | gaccgataatggtaacaatctgt | 58826 | 482140 | see also 6085 line | | |
| 6087 | CPEB3 | NM_014912.1 | 1135 | tggagaactccttaatggatatg | 58832 | 482147 | see also 6085 line | | |
| 6088 | NTNG1 | NM_014917.1 | 1122 | agcctctccaggttaacatcact | 282629 | 661937 | - | - | - |
| 6089 | NTNG1 | NM_014917.1 | 1173 | cagacaacatagttattaccttt | 282631 | 661939 | see also 6088 line | | |
| 6090 | NTNG1 | NM_014917.1 | 1201 | tgggcgtccagaccaaatgatcc | 282633 | 661942 | see also 6088 line | | |
| 6091 | NTNG1 | NM_014917.1 | 1253 | tggcagccctatcagtattatgc | 282636 | 661944 | see also 6088 line | | |
| 6092 | NTNG1 | NM_014917.1 | 1475 | cagctggatacaaccaagaaact | 282644 | 661956 | see also 6088 line | | |
| 6093 | NTNG1 | NM_014917.1 | 1547 | gccgttggggaaatatttgtaga | 282645 | 661958 | see also 6088 line | | |
| 6094 | ICK | NM_014920.2 | 640 | ctgagtctgctataaggaatatc | 85587 | 504054 | - | - | - |
| 6095 | ICK | NM_014920.2 | 1914 | ggcttgtctggaaaatcttcagg | 85622 | 504084 | see also 6094 line | | |
| 6096 | CNK2 | NM_014927.1 | 486 | atggtctccgagtcaagtagtgg | 180358 | 573648 | - | - | - |
| 6097 | CNK2 | NM_014927.1 | 1132 | gggctgggtatgtatattaaatc | 180371 | 573677 | see also 6096 line | | |
| 6098 | CNK2 | NM_014927.1 | 1307 | aggacccgagtggtgttatctta | 180377 | 573684 | see also 6096 line | | |
| 6099 | CNK2 | NM_014927.1 | 1309 | gacccgagtggtgttatcttaac | 180378 | 573685 | see also 6096 line | | |
| 6100 | CNK2 | NM_014927.1 | 1310 | acccgagtggtgttatcttaact | 180379 | 573686 | see also 6096 line | | |
| 6101 | CNK2 | NM_014927.1 | 1340 | agcgacctcagagcatgcttacc | 180382 | 573688 | see also 6096 line | | |
| 6102 | CNK2 | NM_014927.1 | 1406 | ctctgcagcctcttatacctaga | 180383 | 573689 | see also 6096 line | | |
| 6103 | CNK2 | NM_014927.1 | 1411 | cagcctcttatacctagaagtcc | 180384 | 573690 | see also 6096 line | | |
| 6104 | CNK2 | NM_014927.1 | 1757 | atggcaagctacgacctatatct | 180390 | 573699 | see also 6096 line | | |
| 6105 | CNK2 | NM_014927.1 | 1759 | ggcaagctacgacctatatctat | 180391 | 573700 | see also 6096 line | | |
| 6106 | CNK2 | NM_014927.1 | 1798 | tgggtgggggactatgaagatcc | 180392 | 573702 | see also 6096 line | | |

Figure 46

| | | | | | | | | | |
|---|---|---|---|---|---|---|---|---|---|
| 6107 | CNK2 | NM_014927.1 | 2430 | caggtggcttaacagaattaata | 180415 | 573719 | see also 6096 line | | |
| 6108 | CNK2 | NM_014927.1 | 2749 | cgccgctcctggcaggatttaat | 180424 | 573726 | see also 6096 line | | |
| 6109 | CNK2 | NM_014927.1 | 2750 | gccgctcctggcaggatttaatt | 180425 | 573727 | see also 6096 line | | |
| 6110 | CNK2 | NM_014927.1 | 2751 | ccgctcctggcaggatttaattg | 180426 | 573728 | see also 6096 line | | |
| 6111 | CNK2 | NM_014927.1 | 2971 | gggggcaagcctcgaagttttac | 180435 | 573736 | see also 6096 line | | |
| 6112 | CNK2 | NM_014927.1 | 2972 | ggggcaagcctcgaagttttact | 180436 | 573737 | see also 6096 line | | |
| 6113 | CNK2 | NM_014927.1 | 2974 | ggcaagcctcgaagttttactct | 180437 | 573738 | see also 6096 line | | |
| 6114 | CNK2 | NM_014927.1 | 3002 | gagatagcgggttcaaccattgc | 180438 | 573739 | see also 6096 line | | |
| 6115 | NLGN1 | NM_014932.2 | 1387 | tgcaatgtttcagatacagtaga | 346003 | 752373 | channel | - | - |
| 6116 | NLGN1 | NM_014932.2 | 1395 | ttcagatacagtagagttagtgg | 346004 | 752374 | see also 6115 line | | |
| 6117 | NLGN1 | NM_014932.2 | 2688 | gaccgcctgtcccccagattaca | 346036 | 752403 | see also 6115 line | | |
| 6118 | PEPP3 | NM_014935.2 | 2601 | caggcagctctcaacaaagttgg | 346112 | 807562 | - | - | - |
| 6119 | ENPP4 | NM_014936.1 | 974 | atcgaattcagcccattattttg | 204851 | 815500 | - | - | - |
| 6120 | INPP5F | NM_014937.2 | 480 | agctctgtaagaagcatcatttt | 346128 | 802260 | - | - | - |
| 6121 | KIAA1012 | NM_014939.1 | 215 | ttcacatgagagatcctaataat | 272750 | 792134 | - | intracellular transporter | - |
| 6122 | KIAA1012 | NM_014939.1 | 220 | atgagagatcctaataatcaact | 272751 | 792136 | see also 6121 line | | |
| 6123 | KIAA1012 | NM_014939.1 | 262 | aagatagcagtaagcaacattgt | 272752 | 792139 | see also 6121 line | | |
| 6124 | KIAA1012 | NM_014939.1 | 373 | gtgattacagcaggagattatga | 272758 | 792144 | see also 6121 line | | |
| 6125 | KIAA1012 | NM_014939.1 | 381 | agcaggagattatgaccttaaca | 272759 | 792145 | see also 6121 line | | |
| 6126 | KIAA1012 | NM_014939.1 | 714 | aactcagggttgctatttactta | 272772 | 792165 | see also 6121 line | | |
| 6127 | KIAA1012 | NM_014939.1 | 718 | cagggttgctatttacttaaaat | 272773 | 792166 | see also 6121 line | | |
| 6128 | KIAA1012 | NM_014939.1 | 836 | atgaagatggcccttgtactata | 272775 | 792172 | see also 6121 line | | |
| 6129 | KIAA1012 | NM_014939.1 | 1809 | ttgtcaagccatgcaagtttaca | 272811 | 792209 | see also 6121 line | | |
| 6130 | ANKRD6 | NM_014942.1 | 1271 | aagtgatgcaggcaccaataaat | 346240 | 746715 | - | - | - |
| 6131 | ANKRD6 | NM_014942.1 | 1302 | atgtgaacctctaatcaacaagc | 346243 | 746721 | see also 6130 line | | |
| 6132 | ZHX2 | NM_014943.2 | 706 | gtggttatgagtgcaaatactgc | 14480 | 817048 | transcription_regulator | - | - |
| 6133 | ZHX2 | NM_014943.2 | 2475 | ggcctggtccgaactgagattgt | 14494 | 817072 | see also 6132 line | | |
| 6134 | KIAA0843 | NM_014945.1 | 882 | tacgctgccaccagatgttcacc | 117666 | 814188 | - | - | - |
| 6135 | KIAA0843 | NM_014945.1 | 1595 | tactcgccagacccctactatgc | 117681 | 814203 | see also 6134 line | | |
| 6136 | SPG4 | NM_014946.3 | 1193 | tgctaaccttataatgaatgaaa | 101155 | 515220 | - | adenosinetriphosphatase | spastic paraplegia |
| 6137 | SPG4 | NM_014946.3 | 1945 | gtgagatgagaaatattcgatta | 101180 | 515233 | see also 6136 line | | |
| 6138 | KIAA1041 | NM_014947.1 | 329 | aggcttgtccatctgtaacttca | 5727 | 442358 | - | transcription factor | - |
| 6139 | KIAA1041 | NM_014947.1 | 482 | atgcactccttgacccaaataca | 5731 | 442363 | see also 6138 line | | |
| 6140 | KIAA1041 | NM_014947.1 | 836 | aacgggcctcaactccatatagc | 5739 | 442376 | see also 6138 line | | |

Figure 46

| | | | | | | | |
|---|---|---|---|---|---|---|---|
| 6141 | KIAA1041 | NM_014947.1 | 840 | ggcctcaactccatatagcatag | 5741 | 442377 | see also 6138 line |
| 6142 | KIAA1041 | NM_014947.1 | 849 | tccatatagcatagattcagatt | 5743 | 442378 | see also 6138 line |
| 6143 | KIAA1041 | NM_014947.1 | 933 | gactaacaaagtaacattgtata | 5747 | 442380 | see also 6138 line |
| 6144 | KIAA1041 | NM_014947.1 | 1225 | gagcagtcacttagtcaacaagg | 5763 | 442396 | see also 6138 line |
| 6145 | KIAA1041 | NM_014947.1 | 2007 | accccctggttatcctcatatcc | 5790 | 442411 | see also 6138 line |
| 6146 | UBCE7IP5 | NM_014948.2 | 230 | agctgatggttacgaagtagaaa | 324904 | 746819 | - |
| 6147 | KIAA0907 | NM_014949.1 | 1045 | cacaaccctgctataagtagt | 346275 | 736418 | - |
| 6148 | KIAA0907 | NM_014949.1 | 1075 | ctcaaccacatattatccatcc | 346276 | 736420 | see also 6147 line |
| 6149 | KIAA0907 | NM_014949.1 | 1465 | agcatggaccattcatatgact | 346282 | 736427 | see also 6147 line |
| 6150 | KIAA0907 | NM_014949.1 | 1477 | ttcatatgactaatttaggtaca | 346284 | 736430 | see also 6147 line |
| 6151 | KIAA1052 | NM_014956.1 | 4407 | tggagaagtggcgcaagtatttt | 346343 | 775344 | - |
| 6152 | RIPX | NM_014961.1 | 608 | gagacactacctttgcaaatt | 346350 | 729776 | - |
| 6153 | RIPX | NM_014961.1 | 1893 | aacaggcaaagacttaaatagt | 346378 | 729806 | see also 6152 line |
| 6154 | DHX30 | NM_014966.2 | 2404 | ctgagacttccatcacaatcaat | 48212 | 483639 | - |
| 6155 | DHX30 | NM_014966.2 | 2594 | aagccggctggagaaaatggtcc | 48215 | 471798 | see also 6154 line |
| 6156 | KIAA1018 | NM_014967.1 | 1499 | aggctcttcaacgtaaattaag | 648463 | 648306 | see also 6156 line |
| 6157 | KIAA1018 | NM_014967.1 | 1500 | ggctcttcaacgtaaattaagc | 648464 | 648307 | see also 6156 line |
| 6158 | KIAA0893 | NM_014969.3 | 364 | gagaaggaaagtggagtcataaa | 346513 | 805639 | see also 6158 line |
| 6159 | KIAA0893 | NM_014969.3 | 501 | aaggtttcgttatattattcctga | 346517 | 805643 | signal transduction |
| 6160 | KIFAP3 | NM_014970.2 | 282 | aggacgccagatacctcaaaagg | 120564 | 525165 | see also 6160 line |
| 6161 | KIFAP3 | NM_014970.2 | 285 | acgccagatacctcaaaaggaaa | 120565 | 525166 | see also 6160 line |
| 6162 | KIFAP3 | NM_014970.2 | 2294 | aagtttcgctggcataactctca | 120642 | 525216 | see also 6160 line |
| 6163 | KIFAP3 | NM_014970.2 | 3191 | cagtacttgtatggtgatgatcg | 120645 | 525219 | see also 6160 line |
| 6164 | PCNX | NM_014982.1 | 3623 | ttctacctcagttcgatttatc | 346623 | 707483 | - |
| 6165 | PCNX | NM_014982.1 | 3647 | cagacatggtcataatcgtatca | 346643 | 707494 | see also 6164 line |
| 6166 | PCNX | NM_014982.1 | 4459 | tgcctacagtagaccagtttatt | 346645 | 707496 | see also 6164 line |
| 6167 | PCNX | NM_014982.1 | 5275 | tccgaaatgcagccactatgatg | 346680 | 707513 | see also 6164 line |
| 6168 | PCNX | NM_014982.1 | 6513 | agctgcgggacttgaattcaga | 346715 | 707529 | see also 6164 line |
| 6169 | WDFY3 | NM_014991.3 | 6576 | aggaagctaccaggtattggtga | 161003 | 780374 | - |
| 6170 | WDFY3 | NM_014991.3 | 8996 | ttggcaagcatgttcaacaaag | 161005 | 780378 | see also 6169 line |
| 6171 | WDFY3 | NM_014991.3 | 9021 | ttgatctaggctgtaaacaaat | 161087 | 780426 | see also 6169 line |
| 6172 | WDFY3 | NM_014991.3 | 10417 | caccaagcttggagatgttatcc | 161088 | 780428 | see also 6169 line |
| 6173 | WDFY3 | NM_014991.3 | 1474 | cagctcagtctagatgagagaaga | 161124 | 780458 | see also 6169 line |
| 6174 | STK38L | NM_015000.1 | | aagactgggttttctcaattat | 101859 | 515774 | - |
| 6175 | SHARP | NM_015001.2 | 621 | cagggagcgttatgtaacata | 65965 | 445757 | transcription regulator |
| 6176 | SHARP | NM_015001.2 | 622 | agggagcgtgcttattgaacatag | 65966 | 445758 | see also 6175 line |

Figure 46

| | | | | | | | | |
|---|---|---|---|---|---|---|---|---|
| 6177 | SHARP | NM_015001.2 | 623 | gggagcgtgcttatgaacatagt | 65967 | 445759 | see also 6175 line | |
| 6178 | SHARP | NM_015001.2 | 690 | aagacattacgatcaggattact | 65971 | 445762 | see also 6175 line | |
| 6179 | SHARP | NM_015001.2 | 692 | gacattacgatcaggattactat | 65972 | 445763 | see also 6175 line | |
| 6180 | SHARP | NM_015001.2 | 697 | tacgatcaggattactatagaga | 65973 | 445764 | see also 6175 line | |
| 6181 | SHARP | NM_015001.2 | 723 | tcgagacggactttacaacatg | 65975 | 445766 | see also 6175 line | |
| 6182 | SHARP | NM_015001.2 | 842 | gtgtggtacacagggatatctac | 65979 | 445769 | see also 6175 line | |
| 6183 | SHARP | NM_015001.2 | 4445 | agctacgtgagcgagatgaaaga | 66070 | 445854 | see also 6175 line | |
| 6184 | FBXO21 | NM_015002.1 | 283 | caccgactacgtcaattggttgg | 228531 | 757397 | - | |
| 6185 | FBXO21 | NM_015002.1 | 487 | agctttgacctggaaatactacg | 228542 | 757405 | see also 6184 line | |
| 6186 | USP33 | NM_015017.2 | 282 | trgtccacatttggattcagttg | 189609 | 583555 | - | |
| 6187 | USP33 | NM_015017.2 | 497 | ttcgagtatggttgttatgcttgc | 189619 | 583563 | see also 6186 line | |
| 6188 | USP33 | NM_015017.2 | 2518 | ttgatgctgcctcagaacattg | 189684 | 583621 | see also 6186 line | |
| 6189 | WDTC1 | NM_015023.2 | 866 | tgggcttatccgccagtatgacc | 43393 | 816599 | - | |
| 6190 | XPO7 | NM_015024.2 | 119 | gagcctggcccaactagagaatc | 245703 | 653378 | receptor_acitivity | protein transporter |
| 6191 | XPO7 | NM_015024.2 | 120 | agcctggcccaactagagaatct | 245704 | 653379 | see also 6190 line | |
| 6192 | XPO7 | NM_015024.2 | 359 | tcggaactatgtgctcaactacc | 245712 | 653382 | see also 6190 line | |
| 6193 | XPO7 | NM_015024.2 | 669 | ttcacacttctgcaatttact | 245724 | 653391 | see also 6190 line | |
| 6194 | XPO7 | NM_015024.2 | 1168 | tccgattgatagccaacttcaca | 245733 | 653419 | see also 6190 line | |
| 6195 | XPO7 | NM_015024.2 | 2026 | atcacacgaggcgagcacttttca | 245756 | 653454 | see also 6190 line | |
| 6196 | XPO7 | NM_015024.2 | 2028 | cacacgaggcgagcacttttcatt | 245757 | 653455 | see also 6190 line | |
| 6197 | XPO7 | NM_015024.2 | 2796 | caggaccacatatgaactttattgc | 245778 | 653471 | see also 6190 line | |
| 6198 | POP1 | NM_015029.1 | 312 | ctcaggagatcccaagtatata | 199107 | 724124 | - | ribonuclease P |
| 6199 | POP1 | NM_015029.1 | 315 | aggagatcccaagtatataact | 199109 | 724125 | see also 6198 line | |
| 6200 | NMNAT2 | NM_015039.2 | 523 | gccggcacgtctcatcatgtgt | 209018 | 598664 | - | |
| 6201 | NMNAT2 | NM_015039.2 | 1178 | cggccatgtgtggattacctgt | 209032 | 598672 | see also 6200 line | |
| 6202 | KIAA0625 | NM_015046.4 | 6196 | ctgtgtgtgcaccttccaatgc | 347416 | 811429 | - | |
| 6203 | KIAA0625 | NM_015046.4 | 6958 | tacagatgcatccagacatatg | 347433 | 811456 | see also 6202 line | |
| 6204 | KIAA0625 | NM_015046.4 | 7107 | aagacgggataatgaactcatata | 347443 | 811463 | see also 6202 line | |
| 6205 | KIAA0090 | NM_015047.1 | 2757 | acgcagagcgattcatcaactat | 347516 | 760782 | - | |
| 6206 | KIAA0090 | NM_015047.1 | 2828 | ctggagtccacttgttggttgt | 347522 | 760785 | see also 6205 line | |
| 6207 | KIAA0090 | NM_015047.1 | 2958 | tggttttgccaccatgatcact | 347526 | 760788 | see also 6205 line | |
| 6208 | ALS2CR3 | NM_015049.1 | 841 | ttggacaagcctttgatcaagt | 170988 | 773157 | - | receptor binding |
| 6209 | KIAA0082 | NM_015050.1 | 1492 | ctgaaggtgggcatagaatgatgt | 63086 | 478297 | - | |
| 6210 | KIAA0322 | NM_015052.1 | 961 | agctgcaattccgatttgagatc | 230102 | 615405 | - | |
| 6211 | KIAA0322 | NM_015052.1 | 3124 | gtcgtcttccaatcatctaact | 230122 | 615438 | see also 6210 line | |
| 6212 | KIAA0322 | NM_015052.1 | 3322 | ttcttcgccagccaaacatttt | 230129 | 615445 | see also 6210 line | |
| 6213 | KIAA0322 | NM_015052.1 | 3327 | cgccagccaaacatttttgaaat | 230130 | 615446 | see also 6210 line | |

Figure 46

| | | | | | | | | | |
|---|---|---|---|---|---|---|---|---|---|
| 6214 | SWAP70 | NM_015055.1 | 1336 | agctggaagacatgtacctaaag | 153507 | 468529 | - | - | - |
| 6215 | GASC1 | NM_015061.1 | 767 | agcccaagcttggtatgctata | 27445 | 753698 | transcription_regulator | - | - |
| 6216 | GASC1 | NM_015061.1 | 2318 | tacgggttcatgcaagttgttat | 27471 | 753732 | see also 6215 line | | |
| 6217 | GASC1 | NM_015061.1 | 2865 | gaccttctatgagctcatgtttg | 27487 | 753758 | see also 6215 line | | |
| 6218 | ELKS | NM_015064.2 | 1017 | aagcttccaaatcaccatttgg | 347538 | 745957 | - | | thyroid cancer |
| 6219 | SLAC2-B | NM_015065.1 | 590 | caggcaaaaatatacaattcacc | 347599 | 790719 | - | | |
| 6220 | OAZ | NM_015069.1 | 770 | cccacgccaaccagaaacacaag | 347728 | 744035 | - | | |
| 6221 | OAZ | NM_015069.1 | 2114 | tgcaggtgcagtcaaaacacagc | 347736 | 744049 | see also 6220 line | | |
| 6222 | KIF1B | NM_015074.1 | 246 | aggcaactcgaccagtattatta | 87140 | 505228 | - | motor | Charcot-Marie-Tooth neuropathy |
| 6223 | KIF1B | NM_015074.1 | 247 | ggcaactcgaccagtattaa | 87141 | 505229 | see also 6222 line | | |
| 6224 | KIF1B | NM_015074.1 | 1881 | ccgtatcatcatgggtaaaaacc | 87172 | 505262 | see also 6222 line | | |
| 6225 | SARM1 | NM_015077.1 | 1307 | atcacccgcaagagttctttag | 246630 | 633513 | receptor_acitivity | | |
| 6226 | SPG20 | NM_015087.3 | 1767 | gttgcagctaaatgcatcgttaac | 347992 | 754778 | - | | |
| 6227 | SPG20 | NM_015087.3 | 1768 | tgcagctaaatgcatcgttaaca | 347993 | 754779 | see also 6226 line | | |
| 6228 | SPG20 | NM_015087.3 | 1770 | cagctaaatgcatcgttaacaat | 347994 | 754780 | see also 6226 line | | |
| 6229 | SPG20 | NM_015087.3 | 1771 | agctaaatgcatcgttaacaatg | 347995 | 754781 | see also 6226 line | | |
| 6230 | MAP3K7IP2 | NM_015093.2 | 213 | tacatgacctgcgacaaaaattc | 219657 | 752416 | kinase_related | | |
| 6231 | MAP3K7IP2 | NM_015093.2 | 379 | ttctggtctacgcaatcacatga | 219662 | 752421 | see also 6230 line | | |
| 6232 | MAP3K7IP2 | NM_015093.2 | 384 | gtctacgcaatcacatgactct | 219663 | 752422 | see also 6230 line | | |
| 6233 | MAP3K7IP2 | NM_015093.2 | 1093 | ttctgtcccatagccaatataaca | 219681 | 752445 | see also 6230 line | | |
| 6234 | MAP3K7IP2 | NM_015093.2 | 1095 | ctgcccatagccaatataacatt | 219682 | 752446 | see also 6230 line | | |
| 6235 | MAP3K7IP2 | NM_015093.2 | 1096 | tgcccatagccaatataacattc | 219683 | 752447 | see also 6230 line | | |
| 6236 | CAMTA2 | NM_015099.2 | 1232 | ttgaccctgatcgtttctcaac | 348017 | 800882 | - | | |
| 6237 | CAMTA2 | NM_015099.2 | 3383 | cacttaagtttgcactctataag | 348033 | 800896 | see also 6236 line | | |
| 6238 | POGZ | NM_015100.1 | 325 | tcggcctgtacaaaatgcaatg | 35557 | 454263 | - | | |
| 6239 | POGZ | NM_015100.1 | 326 | ccggcctgtacaaaatgcaatga | 35558 | 454264 | see also 6238 line | | |
| 6240 | POGZ | NM_015100.1 | 1501 | ttcatagtccctatgaatctact | 35585 | 454297 | see also 6238 line | | |
| 6241 | C1orf17 | NM_015101.1 | 827 | ttcgaactgcgagggaaaaatgg | 303143 | 671514 | - | | |
| 6242 | NPHP4 | NM_015102.2 | 3866 | tggttccaagcccggagatcaaag | 134269 | 538284 | signal_transduction | | |
| 6243 | NPHP4 | NM_015102.2 | 4163 | cagccgctttgtccatctcaac | 134271 | 538287 | see also 6242 line | | |
| 6244 | SRISNF2L | NM_015106.1 | 807 | aagtccagcctcggttcttaaa | 41113 | 468293 | - | | |

Figure 46

| | | | | | | | |
|---|---|---|---|---|---|---|---|
| 6245 | SRISNF2L | NM_015106.1 | 1101 | atgagggacaccgcatcaaaaac | 41124 | 468304 | see also 6244 line |
| 6246 | SRISNF2L | NM_015106.1 | 2929 | caccacgacagatattgttattc | 41165 | 468344 | see also 6244 line |
| 6247 | SRISNF2L | NM_015106.1 | 4054 | tgggttcagctccaatgatgatg | 41175 | 468360 | see also 6244 line |
| 6248 | KIAA1111 | NM_015107.1 | 766 | aagtcctcaatgtcattagtttg | 29587 | 454351 | transcription_regulator |
| 6249 | SMC5L1 | NM_015110.1 | 1899 | ttgaacgggtaatacaagaaacc | 100786 | 514935 | - |
| 6250 | MAST205 | NM_015112.1 | 682 | agcatcaaacctgttcgaatga | 90341 | 508783 | kinase_related |
| 6251 | MAST205 | NM_015112.1 | 1379 | gcccgagactgcctggataaatc | 90352 | 508801 | see also 6250 line |
| 6252 | MAST205 | NM_015112.1 | 2282 | aactrgtatgagggtcatattga | 90365 | 508815 | see also 6250 line |
| 6253 | MAST205 | NM_015112.1 | 3603 | tccaccgagctggcaagaagtat | 90373 | 508836 | see also 6250 line |
| 6254 | KIAA0276 | NM_015115.1 | 510 | ttgaaccagaaaacgtagttatg | 348200 | 804724 | - |
| 6255 | KIAA0276 | NM_015115.1 | 820 | tggagcatggccagttttgttgg | 348210 | 804739 | see also 6254 line |
| 6256 | KIAA0290 | NM_015122.1 | 1407 | ggcccagggggcaccatgaaacg | 218840 | 606401 | - |
| 6257 | MCLC | NM_015127.2 | 765 | aagttcatggacctataaggatg | 348510 | 757146 | - |
| 6258 | SLC35D1 | NM_015139.1 | 361 | ttccactacctactatatttt | 259283 | 798586 | sugar porter |
| 6259 | RCOR | NM_015156.1 | 1275 | acgccgcttcaacatagatgaag | 37929 | 457609 | - |
| 6260 | MLC1 | NM_015166.2 | 509 | gtgattcccaactttcagatatt | 263621 | 747118 | channel |
| 6261 | PTDSR | NM_015167.1 | 75 | ttggaccggcacaactactacg | 250754 | 637378 | - |
| 6262 | PTDSR | NM_015167.1 | 582 | tgcctggaatgccttagttcagg | 250769 | 637385 | see also 6261 line |
| 6263 | SULF1 | NM_015170.1 | 807 | tacagcaggaacgaaaaacatc | 200620 | 592351 | apoptosis |
| 6264 | SULF1 | NM_015170.1 | 1247 | aagcatggatttgattatgcaaa | 200636 | 592362 | see also 6263 line |
| 6265 | SULF1 | NM_015170.1 | 1291 | cactaacgagagcattaattact | 200639 | 592365 | see also 6263 line |
| 6266 | TBC1D1 | NM_015173.1 | 467 | aggactttgaatccaaagcaaac | 186284 | 708811 | - |
| 6267 | TBC1D1 | NM_015173.1 | 1330 | cagtatcggccagacatgattat | 186304 | 708824 | see also 6266 line |
| 6268 | KIAA0483 | NM_015176.1 | 1078 | agccatagactctcttaggaaat | 348949 | 785647 | - |
| 6269 | RHOBTB2 | NM_015178.1 | 1646 | ccggaccaacggcgttaaggagt | 110717 | 520317 | signal_transduction |
| 6270 | SYNE2 | NM_015180.3 | 444 | cagttgcctcgggataaaggatc | 348988 | 791382 | receptor_acitivity |
| 6271 | CHAC | NM_015186.1 | 7220 | aagcaaataccatatatcagtgg | 272677 | 782533 | - |
| 6272 | KIAA0746 | NM_015187.1 | 1680 | cagctgtgtggataccataaag | 349500 | 777798 | - |
| 6273 | PLCB1 | NM_015192.2 | 665 | ctgaccrgaaattgataacatc | 198211 | 590467 | cell_cycle |
| 6274 | PLCB1 | NM_015192.2 | 1802 | aaggaacacgtgtggattcatcc | 198233 | 590492 | see also 6273 line |
| 6275 | PLCB1 | NM_015192.2 | 3145 | gagtgtcagaacaatcagttaaa | 198262 | 590514 | see also 6273 line |
| 6276 | KIAA0922 | NM_015196.2 | 1601 | gttgattctgaacttgcaaataa | 349575 | 777473 | - |
| 6277 | BOP1 | NM_015201.3 | 2136 | tgccatgcatggtgtacaatga | 324005 | 704273 | - |
| 6278 | KIAA0874 | NM_015208.2 | 216 | tgcccaaatctggttcacaaaa | 349833 | 720511 | - |
| 6279 | KIAA0433 | NM_015216.1 | 1237 | atggtactgatgttaaggtttat | 197029 | 784279 | phosphatase |
| 6280 | KIAA0433 | NM_015216.1 | 2767 | aggcattagcagatattgttatc | 197068 | 784319 | see also 6279 line |

Figure 46

| | | | | | | | |
|---|---|---|---|---|---|---|---|
| 6281 | KIAA0433 | NM_015216.1 | 3054 | cagtggaaacgagctatggatta | 197080 | 784328 | see also 6279 line |
| 6282 | KIAA0433 | NM_015216.1 | 3056 | gttggaaacgagctatggattt | 197081 | 784329 | see also 6279 line |
| 6283 | KIAA0433 | NM_015216.1 | 3085 | ttgtcaatgagctcaactacatg | 197083 | 784332 | see also 6279 line |
| 6284 | AGTPBP1 | NM_015239.1 | 761 | gacactcttgctgcattgctaaa | 185437 | 579707 | - |
| 6285 | AGTPBP1 | NM_015239.1 | 2129 | ctgcgcaagtaattcaaattag | 185479 | 579750 | see also 6284 line |
| 6286 | CENTD2 | NM_015242.2 | 2761 | accgctgtgtgactacatcacg | 24276 | 728341 | signal_transduction |
| 6287 | ANKS1 | NM_015245.1 | 3146 | gagcacgagatccggaacatttc | 350271 | 805899 | - |
| 6288 | ANKS1 | NM_015245.1 | 3147 | agcacgagatccggaacatttcc | 350272 | 805900 | see also 6287 line |
| 6289 | ANKS1 | NM_015245.1 | 3150 | acgagatccggaacatttcctgt | 350273 | 805901 | see also 6287 line |
| 6290 | CYLD | NM_015247.1 | 546 | atcgttctgtggggcattcaagg | 188193 | 582518 | - |
| 6291 | BICD2 | NM_015250.1 | 378 | ggcctccaaggagcagtactacg | 321247 | 532700 | - |
| 6292 | KIAA0903 | NM_015252.1 | 1950 | gactgttatgacttatctctatc | 350339 | 767837 | - |
| 6293 | KIAA0903 | NM_015252.1 | 3122 | gaggtggagatgaacttactaac | 350375 | 767861 | see also 6292 line |
| 6294 | SATB2 | NM_015265.1 | 245 | ggccgtggagtttgatgattc | 10967 | 448299 | - |
| 6295 | SATB2 | NM_015265.1 | 671 | ccctctcccagagtatgattt | 10979 | 448312 | see also 6294 line |
| 6296 | SATB2 | NM_015265.1 | 1023 | atggcccatctgataaaccaaca | 10993 | 448321 | see also 6294 line |
| 6297 | SATB2 | NM_015265.1 | 1490 | gacaacagacctccctattaagg | 10996 | 448327 | see also 6294 line |
| 6298 | SATB2 | NM_015265.1 | 1493 | aacagacctccctattaaggtgg | 10997 | 448328 | see also 6294 line |
| 6299 | SATB2 | NM_015265.1 | 1930 | ccggttctcgcacaagatcctcc | 11001 | 448331 | see also 6294 line |
| 6300 | ADCY6 | NM_015270.2 | 2489 | ttccgccagatgggcattgatga | 164960 | 562751 | - |
| 6301 | TRIM2 | NM_015271.2 | 1382 | ccgaggcagccgcggtttaagctga | 120704 | 525144 | - |
| 6302 | TRIM2 | NM_015271.2 | 1894 | tggaccgcaatgggcacattatt | 120716 | 525152 | see also 6301 line |
| 6303 | NEDD4L | NM_015277.2 | 731 | taggccgaacttactatgtcaac | 230363 | 616491 | - |
| 6304 | NEDD4L | NM_015277.2 | 732 | aggccgaacttactatgtcaacc | 230364 | 616492 | see also 6303 line |
| 6305 | NEDD4L | NM_015277.2 | 1393 | ctcagctgccaacagtaactt | 230376 | 616504 | see also 6303 line |
| 6306 | NEDD4L | NM_015277.2 | 1395 | cagctgccaacagtaactttat | 230377 | 616505 | see also 6303 line |
| 6307 | NEDD4L | NM_015277.2 | 1396 | agctcgccaacagtaactttatc | 230378 | 616506 | see also 6303 line |
| 6308 | NEDD4L | NM_015277.2 | 1996 | ctgtgattgagtttgaatcaga | 230397 | 616522 | see also 6303 line |
| 6309 | NEDD4L | NM_015277.2 | 1998 | gttgattgagtttgaatcagaga | 230398 | 616523 | see also 6303 line |
| 6310 | SASH1 | NM_015278.2 | 2277 | aagtccggcacgttcaagttcatc | 305876 | 785884 | cell_cycle |
| 6311 | KIAA1043 | NM_015281.1 | 1962 | ggcagcgccatgccatgtacacaca | 350873 | 715594 | - |
| 6312 | KIAA1043 | NM_015281.1 | 2236 | ccgggctgttaaaaatatggttg | 350875 | 715596 | see also 6311 line |
| 6313 | KIAA0467 | NM_015284.1 | 4744 | ggctgttacttcgtgtcaaaca | 166967 | 812270 | - |
| 6314 | PHF15 | NM_015288.4 | 1203 | ggcctggaaatgcggactatatt | 34819 | 816433 | transcription_regulator |
| 6315 | PHF15 | NM_015288.4 | 1206 | ctggaaatgcggactatattagc | 34821 | 816434 | see also 6314 line |
| 6316 | TRIM37 | NM_015294.1 | 72 | tgctgaggtttccgatgtttca | 160384 | 810777 | - |

Figure 46

| 6317 | TRIM37 | NM_015294.1 | 363 | ctggacttgtaagaagtgtatct | 160394 | 810782 | see also 6316 line | | |
|------|--------|-------------|-----|--------------------------|--------|--------|--------------------|---|---|
| 6318 | TRIM37 | NM_015294.1 | 572 | atgagcgtgttcgggaaattagg | 160400 | 810785 | see also 6316 line | | |
| 6319 | TRIM37 | NM_015294.1 | 1992 | agcatcatattctcgaaaagata | 160443 | 810837 | see also 6316 line | | |
| 6320 | KIAA0759 | NM_015305.2 | 719 | ggcgagaatgggaggatttctcc | 351321 | 753189 | - | - | - |
| 6321 | FNBP4 | NM_015308.1 | 1699 | cagactgagactcgaattgcaga | 351372 | 535392 | - | - | - |
| 6322 | EFA6R | NM_015310.1 | 713 | aagcttgaatgggcagtagatga | 351412 | 798137 | - | - | - |
| 6323 | ARHGEF12 | NM_015313.1 | 10 | gagtggcacacagtctactatca | 253948 | 638902 | - | - | - |
| 6324 | ARHGEF12 | NM_015313.1 | 80 | aaccgagagtcaccaacagataa | 253950 | 638904 | see also 6323 line | | |
| 6325 | ARHGEF12 | NM_015313.1 | 81 | accgagagtcaccaacagataag | 253951 | 638905 | see also 6323 line | | |
| 6326 | ARHGEF12 | NM_015313.1 | 84 | gagagtcaccaacagataagaag | 253952 | 638906 | see also 6323 line | | |
| 6327 | ARHGEF12 | NM_015313.1 | 1208 | ttgctctgttatctctattcaga | 253978 | 638923 | see also 6323 line | | |
| 6328 | PUM2 | NM_015317.1 | 97 | gggaacctgatgattcaacaaaa | 53719 | 475061 | - | - | - |
| 6329 | PUM2 | NM_015317.1 | 326 | ttctcctgctgataaattggatt | 53727 | 475070 | see also 6328 line | | |
| 6330 | PUM2 | NM_015317.1 | 442 | aggaccaaaacagagatcttaaa | 53730 | 475071 | see also 6328 line | | |
| 6331 | PUM2 | NM_015317.1 | 497 | cagaggtttgccaaatggaatgg | 53731 | 475073 | see also 6328 line | | |
| 6332 | PUM2 | NM_015317.1 | 517 | tggatgccgattgcaaagatttt | 53732 | 475075 | see also 6328 line | | |
| 6333 | PUM2 | NM_015317.1 | 520 | atgccgattgcaaagattttaat | 53733 | 475076 | see also 6328 line | | |
| 6334 | PUM2 | NM_015317.1 | 521 | tgccgattgcaaagattttaatc | 53734 | 475077 | see also 6328 line | | |
| 6335 | PUM2 | NM_015317.1 | 522 | gccgattgcaaagattttaatcg | 53735 | 475078 | see also 6328 line | | |
| 6336 | PUM2 | NM_015317.1 | 563 | agcctctccaactgaagtagttg | 53736 | 475079 | see also 6328 line | | |
| 6337 | PUM2 | NM_015317.1 | 564 | gcctctccaactgaagtagttga | 53737 | 475080 | see also 6328 line | | |
| 6338 | PUM2 | NM_015317.1 | 652 | aaccacttgttgaagaattttca | 53740 | 475087 | see also 6328 line | | |
| 6339 | PUM2 | NM_015317.1 | 729 | cagtttgactatcctggtaatca | 53746 | 475089 | see also 6328 line | | |
| 6340 | PUM2 | NM_015317.1 | 773 | agctactgtaggcctttttgact | 53748 | 475092 | see also 6328 line | | |
| 6341 | PUM2 | NM_015317.1 | 1331 | tccaactgcctattatgatcaga | 53757 | 475100 | see also 6328 line | | |
| 6342 | PUM2 | NM_015317.1 | 1744 | ttggcagttctgcaagtagtagt | 53767 | 475109 | see also 6328 line | | |
| 6343 | PUM2 | NM_015317.1 | 1798 | ctgacttgtacaaaagatctagt | 53769 | 475111 | see also 6328 line | | |
| 6344 | PUM2 | NM_015317.1 | 1829 | agcacccatagggcaaccatttt | 53770 | 475112 | see also 6328 line | | |
| 6345 | PUM2 | NM_015317.1 | 1832 | acccatagggcaaccattttaca | 53772 | 475113 | see also 6328 line | | |
| 6346 | PUM2 | NM_015317.1 | 1837 | tagggcaaccattttacaatagt | 53773 | 475114 | see also 6328 line | | |
| 6347 | PUM2 | NM_015317.1 | 1869 | tcctcctctccaagtccaatagg | 53774 | 475115 | see also 6328 line | | |
| 6348 | PUM2 | NM_015317.1 | 2317 | aagcagcctatcaattaatgact | 53790 | 475129 | see also 6328 line | | |
| 6349 | PUM2 | NM_015317.1 | 2403 | gccctggctactcgtattcgtgg | 53796 | 475131 | see also 6328 line | | |

Figure 46

| 6350 | PUM2 | NM_015317.1 | 2892 | agcaatgtagtagaaaagtgtgt | 53813 | 475150 | see also 6328 line | | |
| 6351 | PUM2 | NM_015317.1 | 2896 | atgtagtagaaaagtgtgttact | 53814 | 475151 | see also 6328 line | | |
| 6352 | PUM2 | NM_015317.1 | 2927 | ccgtgctgagagagctttactga | 53815 | 475152 | see also 6328 line | | |
| 6353 | PUM2 | NM_015317.1 | 2930 | tgctgagagagctttactgattg | 53817 | 475153 | see also 6328 line | | |
| 6354 | FEM1B | NM_015322.2 | 862 | cgcttcgacgggtatgtcattga | 248562 | 634847 | receptor_acitivity、 apoptosis | - | - |
| 6355 | FEM1B | NM_015322.2 | 1390 | ttgggtgcctcctttgcaaatga | 248578 | 634859 | see also 6354 line | | |
| 6356 | FEM1B | NM_015322.2 | 2350 | ccgctagacaaaagtacaactgg | 248601 | 634890 | see also 6354 line | | |
| 6357 | FEM1B | NM_015322.2 | 2432 | gagcagttcgggctaatgacatt | 248603 | 634891 | see also 6354 line | | |
| 6358 | FEM1B | NM_015322.2 | 2433 | agcagttcgggctaatgacatta | 248604 | 634892 | see also 6354 line | | |
| 6359 | KIAA0776 | NM_015323.2 | 118 | gagcggaactgcattgagattgt | 351503 | 722381 | - | - | - |
| 6360 | KIAA0776 | NM_015323.2 | 248 | tccgaggtggtcgagtaaacatt | 351508 | 722385 | see also 6359 line | | |
| 6361 | KIAA0776 | NM_015323.2 | 249 | ccgaggtggtcgagtaaacattg | 351509 | 722386 | see also 6359 line | | |
| 6362 | KIAA0776 | NM_015323.2 | 255 | tggtcgagtaaacattgttgatc | 351510 | 722388 | see also 6359 line | | |
| 6363 | KIAA0776 | NM_015323.2 | 816 | caggcagaatggctatctagaat | 351525 | 722405 | see also 6359 line | | |
| 6364 | KIAA0776 | NM_015323.2 | 817 | aggcagaatggctatctagaatt | 351526 | 722406 | see also 6359 line | | |
| 6365 | KIAA0776 | NM_015323.2 | 1626 | gggcagaaaacgcacaatcaagg | 351544 | 722428 | see also 6359 line | | |
| 6366 | KIAA0828 | NM_015328.1 | 1451 | tggaccgtatgaagaatagctgc | 204320 | 594613 | - | adenosylhomocysteinase | - |
| 6367 | NCSTN | NM_015331.1 | 302 | ctcaacgccactcatcagattgg | 282513 | 691583 | - | molecular_function unknown | Alzheimer disease |
| 6368 | NCSTN | NM_015331.1 | 2195 | atcgtcacctactgcatcaatgc | 282558 | 691616 | see also 6367 line | | |
| 6369 | HIP14 | NM_015336.1 | 964 | tagtcaaatactatatttcgaaa | 351634 | 775807 | kinase_related | - | - |
| 6370 | HIP14 | NM_015336.1 | 1146 | cagttcggacatacctcaattgt | 351639 | 775814 | see also 6369 line | | |
| 6371 | HIP14 | NM_015336.1 | 1190 | aggacaggatgtagatatgatgg | 351641 | 775819 | see also 6369 line | | |
| 6372 | HIP14 | NM_015336.1 | 1263 | gtggatccaactagattgctttt | 351646 | 775821 | see also 6369 line | | |
| 6373 | HIP14 | NM_015336.1 | 1278 | ttgcttttaacattcaatgtttc | 351647 | 775824 | see also 6369 line | | |
| 6374 | HIP14 | NM_015336.1 | 1721 | tgcattgccccttgggatatatt | 351667 | 775834 | see also 6369 line | | |
| 6375 | HIP14 | NM_015336.1 | 1943 | aggaagtctggacctcagtatat | 351673 | 775835 | see also 6369 line | | |
| 6376 | HIP14 | NM_015336.1 | 2393 | gtctattgaaagcccattcaacc | 351696 | 775850 | see also 6369 line | | |
| 6377 | HIP14 | NM_015336.1 | 2462 | tggcctctttcgtcctgttatcg | 351699 | 775852 | see also 6369 line | | |
| 6378 | ADNP | NM_015339.2 | 1810 | agcaaatgcctctactgtaatcg | 622 | 441591 | transcription_regulator | - | - |
| 6379 | ADNP | NM_015339.2 | 2175 | agctgcagtgccctataaaaaag | 641 | 441611 | see also 6378 line | | |
| 6380 | ADNP | NM_015339.2 | 3536 | ggcagaatagcacaattgacagt | 674 | 441651 | see also 6378 line | | |
| 6381 | LARS2 | NM_015340.2 | 720 | gtgtttgccagattactacaagt | 88291 | 768588 | - | leucine-tRNA ligase | - |
| 6382 | LARS2 | NM_015340.2 | 1774 | tggtactacttcagatacactga | 88317 | 768613 | see also 6381 line | | |

Figure 46

| | | | | | | | | | |
|---|---|---|---|---|---|---|---|---|---|
| 6383 | KIAA0073 | NM_015342.1 | 1793 | taccaacgccttggcttgataat | 234723 | 671084 | - | - | - |
| 6384 | KIAA0073 | NM_015342.1 | 1794 | accaacgccttggcttgataata | 234724 | 671085 | see also 6383 line | | |
| 6385 | KIAA0073 | NM_015342.1 | 1797 | aacgccttggcttgataataagc | 234725 | 671086 | see also 6383 line | | |
| 6386 | JJAZ1 | NM_015355.1 | 434 | ggctgaccacgagcttttcctcc | 300516 | 668800 | transcription_regulator | - | - |
| 6387 | JJAZ1 | NM_015355.1 | 1119 | cagcttttagatggggaatatga | 300520 | 668818 | see also 6386 line | | |
| 6388 | JJAZ1 | NM_015355.1 | 1714 | atcgccaacctggatttgctttt | 300540 | 668853 | see also 6386 line | | |
| 6389 | KIAA0052 | NM_015360.2 | 822 | accgtgaaatgtatgaagaattt | 62994 | 478964 | - | - | - |
| 6390 | KIAA0052 | NM_015360.2 | 1064 | gtctttctttcggctactattcc | 63002 | 478974 | see also 6389 line | | |
| 6391 | KIAA0052 | NM_015360.2 | 1068 | ttctttcggctactattccaaat | 63003 | 478975 | see also 6389 line | | |
| 6392 | KIAA0052 | NM_015360.2 | 2003 | cagcattatagagcaattccagg | 63025 | 479004 | see also 6389 line | | |
| 6393 | R3HDM | NM_015361.2 | 1000 | tggattagaccacaatgttgatc | 65201 | 485603 | - | - | - |
| 6394 | R3HDM | NM_015361.2 | 1338 | cccagcagaggcgccagatattt | 65215 | 485615 | see also 6393 line | | |
| 6395 | R3HDM | NM_015361.2 | 1345 | gaggcgccagatatttagagtta | 65218 | 485617 | see also 6393 line | | |
| 6396 | R3HDM | NM_015361.2 | 1809 | taccacctggcagtattctgatc | 65236 | 485622 | see also 6393 line | | |
| 6397 | AMMECR1 | NM_015365.1 | 631 | aacgagccctacccactgtttgt | 276376 | 708407 | - | - | - |
| 6398 | AMMECR1 | NM_015365.1 | 819 | gtctctgctcactaactttgaag | 276380 | 708417 | see also 6397 line | | |
| 6399 | AMMECR1 | NM_015365.1 | 1142 | cccttccgccatacaaccattat | 276394 | 708429 | see also 6397 line | | |
| 6400 | AMMECR1 | NM_015365.1 | 1145 | ttccgccatacaaccattattcc | 276395 | 708430 | see also 6397 line | | |
| 6401 | UNC84B | NM_015374.1 | 349 | gaggaaatccagcaacatgaagc | 120438 | 810277 | - | microtubule binding | - |
| 6402 | CGI-51 | NM_015380.3 | 445 | tagacaagtggatgtttgattg | 278480 | 801850 | - | molecular_function unknown | - |
| 6403 | COG4 | NM_015386.1 | 1233 | ctgtaccatgcaggagctaattg | 268573 | 650783 | - | - | Alzheimer disease |
| 6404 | KIAA1892 | NM_015397.1 | 1430 | gagaggctctcagcttgttatgg | 352287 | 727500 | - | - | - |
| 6405 | DKFZP564O0463 | NM_015420.4 | 1412 | gaggcaagctactcctttgaaaa | 352557 | 813491 | - | - | - |
| 6406 | AASDHPPT | NM_015423.2 | 645 | tggactcagctggatatgtttta | 352610 | 723376 | - | - | - |
| 6407 | AASDHPPT | NM_015423.2 | 729 | ttgcagcggcttgaatttgatct | 352615 | 723381 | see also 6406 line | | |
| 6408 | POLR1A | NM_015425.1 | 1255 | gagccacgtcaatattgtgtttg | 36027 | 466515 | transcription_regulator | DNA-directed RNA polymerase III | - |
| 6409 | POLR1A | NM_015425.1 | 1256 | agccacgtcaatattgtgtttga | 36028 | 466516 | see also 6408 line | | |
| 6410 | POLR1A | NM_015425.1 | 2357 | cactgctgctatgagatctatgg | 36038 | 466536 | see also 6408 line | | |

Figure 46

| | | | | | | | | | |
|---|---|---|---|---|---|---|---|---|---|
| 6411 | POLR1A | NM_015425.1 | 2633 | cagagggattttaacatgattga | 36043 | 466540 | see also 6408 line | | |
| 6412 | POLR1A | NM_015425.1 | 2679 | accattacagcaatgagattaac | 36045 | 466541 | see also 6408 line | | |
| 6413 | TARSH | NM_015429.2 | 639 | aagcccaacacagtttatgaatt | 352659 | 804444 | - | - | - |
| 6414 | DKFZP586H2123 | NM_015430.1 | 1190 | cagtcaagggagacaccattaca | 181885 | 547701 | - | - | - |
| 6415 | RNF19 | NM_015435.3 | 2413 | aacaacaaagcacgaactcaagt | 113879 | 705729 | transcription_regulator | - | - |
| 6416 | HOM-TES-103 | NM_015438.1 | 781 | gcgggagcgggacgagtacaagc | 352850 | 804382 | - | - | - |
| 6417 | FTHFSDC1 | NM_015440.3 | 3183 | aacaagttaaaggcttgttctaa | 83863 | 502823 | - | - | - |
| 6418 | CNOT10 | NM_015442.1 | 510 | aacacttaaccagctgaagaatc | 352858 | 770775 | - | - | - |
| 6419 | CNOT10 | NM_015442.1 | 1128 | gagatgcatgttctggaataacc | 352882 | 770795 | see also 6418 line | | |
| 6420 | CNOT10 | NM_015442.1 | 1132 | tgcatgttctggaataaccttgg | 352884 | 770796 | see also 6418 line | | |
| 6421 | ELYS | NM_015446.1 | 2201 | tgctacaactaccctgtaattca | 25817 | 724452 | transcription_regulator | - | - |
| 6422 | MTMR9 | NM_015458.3 | 594 | ctgggtaccatcatcataaaatg | 353242 | 796959 | - | - | - |
| 6423 | MTMR9 | NM_015458.3 | 1668 | atgctctttgagcatgcttatgc | 353278 | 796982 | see also 6422 line | | |
| 6424 | EHZF | NM_015461.1 | 1913 | tcccttggccctgaattatatcc | 264627 | 649021 | - | - | - |
| 6425 | SIN3A | NM_015477.1 | 2491 | gaggtctaagagcttactcaatg | 300867 | 669347 | transcription_regulator | - | - |
| 6426 | SIN3A | NM_015477.1 | 2969 | ctgaggctgctacggatttgttc | 300872 | 669358 | see also 6425 line | | |
| 6427 | SIN3A | NM_015477.1 | 3106 | acctatggatgttgatgtagaag | 300877 | 669362 | see also 6425 line | | |
| 6428 | SIN3A | NM_015477.1 | 3176 | aacatagactcatcacagtatga | 300878 | 669363 | see also 6425 line | | |
| 6429 | PVRL3 | NM_015480.1 | 274 | gagaagatacatggcaaaagttc | 353435 | 539470 | - | - | - |
| 6430 | PVRL3 | NM_015480.1 | 390 | tgcaacaattactctgcataaca | 353440 | 539471 | see also 6429 line | | |
| 6431 | PVRL3 | NM_015480.1 | 724 | aggcgaattacttgtgttgtaaa | 353454 | 539484 | see also 6429 line | | |
| 6432 | PVRL3 | NM_015480.1 | 726 | gcgaattacttgtgttgtaaaac | 353455 | 539486 | see also 6429 line | | |
| 6433 | PVRL3 | NM_015480.1 | 1296 | gacgtttcgtggagactactttg | 353477 | 539505 | see also 6429 line | | |
| 6434 | PVRL3 | NM_015480.1 | 1392 | ttcttacccagacagtgtaaaaa | 353480 | 539506 | see also 6429 line | | |
| 6435 | ZNF385 | NM_015481.1 | 766 | cagcttgaggcacataacaaagg | 56074 | 476101 | transcription_regulator | - | - |
| 6436 | ZNF385 | NM_015481.1 | 770 | ttgaggcacataacaaaggtact | 56075 | 476102 | see also 6435 line | | |
| 6437 | MR-1 | NM_015488.3 | 418 | ccctgtcctctcggacaactaca | 353508 | 709926 | - | - | - |
| 6438 | DKFZP564G2022 | NM_015497.2 | 788 | ctggagagatctcctgagaattc | 353601 | 784096 | - | - | - |
| 6439 | DKFZP564G2022 | NM_015497.2 | 882 | atccgatacaaaggagaatctgt | 353603 | 784100 | see also 6438 line | | |

Figure 46

| | | | | | | | | | |
|---|---|---|---|---|---|---|---|---|---|
| 6440 | DKFZP564G2022 | NM_015497.2 | 956 | ggctcgaaccctggtcatcatag | 353604 | 784101 | see also 6438 line | | |
| 6441 | SH2B | NM_015503.1 | 3879 | tggttccagtccattttcgatat | 317233 | 540373 | - | - | - |
| 6442 | TIPARP | NM_015508.2 | 1921 | atgtttggacaaggcagttattt | 66809 | 486846 | - | - | - |
| 6443 | TIPARP | NM_015508.2 | 1957 | agctactctcataacttttctaa | 66810 | 486848 | see also 6442 line | | |
| 6444 | TIPARP | NM_015508.2 | 2105 | atgactcttgtgtggataatttc | 66812 | 486852 | see also 6442 line | | |
| 6445 | DKFZP566B183 | NM_015509.2 | 393 | ggggagatgccttcgactttaat | 353630 | 723229 | - | - | - |
| 6446 | CRELD1 | NM_015513.2 | 942 | gtgtggccttggctactttgagg | 140956 | 546607 | - | - | heart disease |
| 6447 | DKFZP566E144 | NM_015523.1 | 706 | accgaaataacatcttcaagaaa | 161829 | 591399 | - | - | - |
| 6448 | DKFZP434P1750 | NM_015527.2 | 298 | ctgcgcaagacggacaagtatgg | 236088 | 620679 | - | - | - |
| 6449 | DKFZP434P1750 | NM_015527.2 | 501 | agcctggcagtacctgtctaata | 236089 | 620682 | see also 6448 line | | |
| 6450 | DKFZP434P1750 | NM_015527.2 | 506 | ggcagtacctgtctaatagcaag | 236091 | 620683 | see also 6448 line | | |
| 6451 | DKFZP566H073 | NM_015528.1 | 681 | gtcaatgggtcagtctttattgc | 191334 | 584760 | - | - | - |
| 6452 | MOXD1 | NM_015529.1 | 634 | tggtgccaaatgtttaagattcc | 163740 | 560929 | - | - | - |
| 6453 | MOXD1 | NM_015529.1 | 1209 | aggcatcaggctgcgtcattttc | 163750 | 560938 | see also 6452 line | | |
| 6454 | MOXD1 | NM_015529.1 | 1213 | atcaggctgcgtcattttcgaaa | 163751 | 560940 | see also 6452 line | | |
| 6455 | DKFZP586B1621 | NM_015533.2 | 384 | cactggggatcggctcaacttcg | 208575 | 598154 | - | - | - |
| 6456 | DKFZP564I052 | NM_015534.3 | 487 | tcccgatctactcgtgttacaag | 25160 | 813056 | - | - | - |
| 6457 | DKFZP564I052 | NM_015534.3 | 488 | cccgatctactcgtgttacaaga | 25161 | 813057 | see also 6456 line | | |
| 6458 | DKFZP564I052 | NM_015534.3 | 507 | aagatcaacagtggggttaaacg | 25162 | 813058 | see also 6456 line | | |
| 6459 | DKFZP564I052 | NM_015534.3 | 959 | aacgagcttgtcgatgtcttata | 25171 | 813064 | see also 6456 line | | |
| 6460 | DNAPTP6 | NM_015535.1 | 389 | cagcatcaaggcaacaaagatgc | 353723 | 754643 | - | - | - |
| 6461 | THEA | NM_015547.2 | 1562 | aggcggaagccttgtgacaatgg | 200091 | 591732 | - | - | - |
| 6462 | BPAG1 | NM_015548.2 | 11601 | ttcagcatgggattctctaaata | 115749 | 522309 | cell_cycle、signal_transduction | - | epidermolysis bullosa |
| 6463 | ZNF451 | NM_015555.1 | 737 | gtcctataatgcactgtaacaag | 265982 | 748345 | transcription_regulator | - | |
| 6464 | ZNF451 | NM_015555.1 | 738 | tcctataatgcactgtaacaagg | 265983 | 748346 | see also 6463 line | | |

Figure 46

| 6465 | SIPA1L1 | NM_015556.1 | 627 | tagccgttcaagccaggaaatag | 284258 | 679918 | - | - | - |
|------|---------|-------------|-----|------------------------|--------|--------|---|---|---|
| 6466 | SIPA1L1 | NM_015556.1 | 628 | agccgttcaagccaggaaataga | 284259 | 679919 | see also 6465 line | | |
| 6467 | SIPA1L1 | NM_015556.1 | 630 | ccgttcaagccaggaaatagaaa | 284260 | 679920 | see also 6465 line | | |
| 6468 | SIPA1L1 | NM_015556.1 | 708 | ttctgttagcctcaattccaatg | 284262 | 679921 | see also 6465 line | | |
| 6469 | SIPA1L1 | NM_015556.1 | 1886 | cagtggctgtgagcattcgaagg | 284293 | 679944 | see also 6465 line | | |
| 6470 | SIPA1L1 | NM_015556.1 | 3729 | tcctggttcggacatctatgtga | 284332 | 679973 | see also 6465 line | | |
| 6471 | SS18L1 | NM_015558.3 | 573 | acgtgtctcggaccaacatcaac | 169988 | 803922 | - | - | - |
| 6472 | SS18L1 | NM_015558.3 | 577 | gtctcggaccaacatcaacatgc | 169989 | 803923 | see also 6471 line | | |
| 6473 | OPA1 | NM_015560.1 | 1299 | aggaaactattttcagtatcagc | 109724 | 519748 | apoptosis | - | optic atrophy、glaucoma |
| 6474 | OPA1 | NM_015560.1 | 2541 | atccaagcttgattaaggatact | 109760 | 519784 | see also 6473 line | | |
| 6475 | LRRTM2 | NM_015564.1 | 442 | ccctatgctggcagcaatatatg | 326940 | 692545 | - | - | - |
| 6476 | LRRTM2 | NM_015564.1 | 607 | gtccctgaggcacaatcacatca | 326944 | 692550 | see also 6475 line | | |
| 6477 | LRRTM2 | NM_015564.1 | 673 | ttggctccacttagatcacaatc | 326947 | 692555 | see also 6475 line | | |
| 6478 | LRRTM2 | NM_015564.1 | 674 | tggctccacttagatcacaatca | 326948 | 692556 | see also 6475 line | | |
| 6479 | LRRTM2 | NM_015564.1 | 776 | tacttgccaaacacaacttttac | 326953 | 692564 | see also 6475 line | | |
| 6480 | LRRTM2 | NM_015564.1 | 885 | tgcagaccttgcatttacgttcc | 326955 | 692569 | see also 6475 line | | |
| 6481 | LRRTM2 | NM_015564.1 | 1053 | accagctgacgaagattaatttt | 326962 | 692579 | see also 6475 line | | |
| 6482 | LRRTM2 | NM_015564.1 | 1058 | ctgacgaagattaattttgctca | 326964 | 692580 | see also 6475 line | | |
| 6483 | LRRTM2 | NM_015564.1 | 1537 | tccaaccactgaatatacaaaaa | 326980 | 692592 | see also 6475 line | | |
| 6484 | LRRTM2 | NM_015564.1 | 1813 | atcccaaacacgactccatatgt | 326989 | 692597 | see also 6475 line | | |
| 6485 | LRRTM2 | NM_015564.1 | 1924 | gtgtcagcagctgccatacaaag | 326995 | 692605 | see also 6475 line | | |
| 6486 | KIAA0820 | NM_015569.1 | 397 | tgccgagtttctacattgcaaag | 109416 | 518450 | - | - | - |
| 6487 | KIAA0820 | NM_015569.1 | 398 | gccgagtttctacattgcaaagg | 109417 | 518451 | see also 6486 line | | |
| 6488 | TNRC15 | NM_015575.1 | 500 | ctcctagaggtcgaagttcttca | 354113 | 760278 | - | - | - |
| 6489 | TNRC15 | NM_015575.1 | 3470 | gtgtgtctaaccggcagaataag | 354168 | 760332 | see also 6488 line | | |
| 6490 | TNRC15 | NM_015575.1 | 3474 | gtctaaccggcagaataagaaag | 354169 | 760333 | see also 6488 line | | |
| 6491 | TNRC15 | NM_015575.1 | 3584 | atgcccttaatacggcaaataac | 354171 | 760337 | see also 6488 line | | |
| 6492 | TNRC15 | NM_015575.1 | 3585 | tgcccttaatacggcaaataact | 354172 | 760338 | see also 6488 line | | |
| 6493 | TNRC15 | NM_015575.1 | 3587 | cccttaatacggcaaataacttg | 354173 | 760339 | see also 6488 line | | |
| 6494 | TNRC15 | NM_015575.1 | 3677 | gggcctatttaggagatacttct | 354177 | 760345 | see also 6488 line | | |
| 6495 | TNRC15 | NM_015575.1 | 3962 | tccgagcagatcccagtttatta | 354183 | 760352 | see also 6488 line | | |
| 6496 | CAST | NM_015576.1 | 2766 | tggccaacctccggattgagagg | 354226 | 799416 | - | - | - |
| 6497 | PDIP38 | NM_015584.2 | 386 | ctggcaggccagactgtatgacc | 354330 | 724661 | - | - | - |
| 6498 | PDIP38 | NM_015584.2 | 514 | ctcgtgactgcccacatatatct | 354333 | 724665 | see also 6497 line | | |
| 6499 | PGM3 | NM_015599.1 | 947 | acgttaattagcagtttccttaa | 234972 | 619684 | - | phosphoacetylglucosamine mutase | Alzheimer disease |
| 6500 | PGM3 | NM_015599.1 | 1410 | cagacaggagagttattagcact | 234993 | 619697 | see also 6499 line | | |

Figure 46

| | | | | | | | |
|---|---|---|---|---|---|---|---|
| 6501 | C20orf22 | NM_015600.2 | 919 | tccagatgcctattattggaat | 235970 | 621268 | - |
| 6502 | DKFZP564G092 | NM_015601.2 | 596 | cagagtacccagaaatattaaaa | 228158 | 614980 | - |
| 6503 | DKFZP564G092 | NM_015601.2 | 812 | aggaggagcccatagttttgtac | 228170 | 614990 | see also 6502 line |
| 6504 | DKFZP564G092 | NM_015601.2 | 904 | atgataggtatgttcctaattta | 228174 | 614996 | see also 6502 line |
| 6505 | DKFZP564G092 | NM_015601.2 | 909 | aggtatgttcctaatttactaaa | 228176 | 614997 | see also 6502 line |
| 6506 | DKFZP547E1010 | NM_015607.2 | 749 | ctccccgaatgggcttaagaaga | 354479 | 713044 | - |
| 6507 | DKFZP547E1010 | NM_015607.2 | 751 | ccccgaatgggcttaagaagagg | 354480 | 713045 | see also 6506 line |
| 6508 | DKFZp566C0424 | NM_015609.1 | 78 | cagcgggcaggaaatccaaatct | 354587 | 785151 | - |
| 6509 | DKFZP434J154 | NM_015610.1 | 346 | gtccctagctgtttggttagtaagt | 354590 | 801464 | - |
| 6510 | DKFZP566F2124 | NM_015630.2 | 253 | ggcagagagcaacgtcaactatt | 354639 | 777132 | - |
| 6511 | DKFZP566F2124 | NM_015630.2 | 259 | gagcaacgtcaactattacaatc | 354640 | 777134 | see also 6510 line |
| 6512 | DKFZP566F2124 | NM_015630.2 | 344 | gacaacgagcaaccagattatga | 354641 | 777137 | see also 6510 line |
| 6513 | DKFZP566F2124 | NM_015630.2 | 476 | cagcttgtaacacttcaagaagc | 354643 | 777145 | see also 6510 line |
| 6514 | DKFZP566F2124 | NM_015630.2 | 886 | tagatcagaaaaagagttatatg | 354660 | 777156 | see also 6510 line |
| 6515 | DKFZP566F2124 | NM_015630.2 | 1000 | ttctgatgtgttcgtcaaaaga | 354663 | 777158 | see also 6510 line |
| 6516 | DKFZP566F2124 | NM_015630.2 | 1088 | ttgccctgatcaataagagtga | 354665 | 777160 | see also 6510 line |
| 6517 | DKFZP566F2124 | NM_015630.2 | 1225 | gcgggcaggatgccagtattatg | 354669 | 777162 | see also 6510 line |
| 6518 | DKFZP566F2124 | NM_015630.2 | 1546 | tagactgtcttttctgaaatat | 354683 | 777173 | see also 6510 line |
| 6519 | DKFZP566F2124 | NM_015630.2 | 1883 | agcacctctgactgtatgtctaa | 354692 | 777185 | see also 6510 line |
| 6520 | DKFZP566F2124 | NM_015630.2 | 1885 | cacctctgactgtatgtctaaaa | 354693 | 777186 | see also 6510 line |

Figure 46

| | | | | | | | | | |
|---|---|---|---|---|---|---|---|---|---|
| 6521 | DKFZP566F2124 | NM_015630.2 | 1886 | acctctgactgtatgtctaaaac | 354694 | 777187 | see also 6510 line | | |
| 6522 | DKFZP566F2124 | NM_015630.2 | 2275 | accatcgccaacagccttaaaac | 354704 | 777196 | see also 6510 line | | |
| 6523 | DKFZP566F2124 | NM_015630.2 | 2278 | atcgccaacagccttaaaacttg | 354705 | 777197 | see also 6510 line | | |
| 6524 | DKFZP564D116 | NM_015631.1 | 1040 | atgctgcctcttactataacttc | 354723 | 723175 | - | - | - |
| 6525 | EIF2B4 | NM_015636.2 | 513 | aggttcctacacgaaaggattat | 56365 | 476463 | - | translation initiation factor | leukoencephalopathy、ovarioleukodystrophy |
| 6526 | TRPC4AP | NM_015638.1 | 450 | ctctatccggggacagaaactga | 103259 | 516733 | - | - | - |
| 6527 | TRPC4AP | NM_015638.1 | 600 | aagtaagaagacattcttacaaa | 103264 | 516738 | see also 6526 line | | |
| 6528 | TRPC4AP | NM_015638.1 | 691 | ctgagttccttagtatccaattt | 103269 | 516743 | see also 6526 line | | |
| 6529 | TRPC4AP | NM_015638.1 | 696 | ttccttagtatccaatttcgatc | 103270 | 516745 | see also 6526 line | | |
| 6530 | TRPC4AP | NM_015638.1 | 793 | cacacgcttcttgccaaaaatgc | 103272 | 516749 | see also 6526 line | | |
| 6531 | TRPC4AP | NM_015638.1 | 1787 | atgcattcaagagattcaataaa | 103290 | 516763 | see also 6526 line | | |
| 6532 | ZNF288 | NM_015642.1 | 2420 | ctccgtctgcccagcaaagtttg | 45011 | 470744 | transcription_regulator | protein binding | - |
| 6533 | ZNF288 | NM_015642.1 | 2421 | tccgtctgcccagcaaagtttga | 45012 | 470745 | see also 6532 line | | |
| 6534 | ABCA12 | NM_015657.3 | 660 | cagcttactgtaccatgacaatg | 68131 | 786512 | - | - | ichthyosis congenita |
| 6535 | ABCA12 | NM_015657.3 | 1455 | aggcaaccacaccaaggattttt | 68166 | 786544 | see also 6534 line | | |
| 6536 | AAAS | NM_015665.3 | 1147 | gccgactgctgttcactgtattg | 276335 | 769490 | - | molecular_function unknown | - |
| 6537 | XTP3TPB | NM_015701.2 | 1197 | ttctgctatggcaaacatgtaca | 355180 | 718558 | - | - | - |
| 6538 | XTP3TPB | NM_015701.2 | 1417 | aggtgactgtaaaactaaagtgc | 355184 | 718568 | see also 6537 line | | |
| 6539 | DJ1042K10.2 | NM_015705.3 | 1048 | tgcgcatctgggacctgtttttc | 355218 | 750709 | - | - | - |
| 6540 | MINK | NM_015716.2 | 4059 | accgtaactgcatcatgaactgg | 297639 | 507581 | kinase_related | - | - |
| 6541 | NOX3 | NM_015718.1 | 1655 | ttcaagcagattgcctacaatca | 227200 | 815293 | - | - | - |
| 6542 | H326 | NM_015726.2 | 745 | gccatactggttgtgtcaatacc | 355241 | 770429 | - | DNA binding | - |
| 6543 | H326 | NM_015726.2 | 1324 | gtgagtccaaagcaaacatcacc | 355250 | 770434 | see also 6542 line | | |
| 6544 | H326 | NM_015726.2 | 1480 | cagtaaaaggcgtcaatttctat | 355252 | 770437 | see also 6542 line | | |
| 6545 | MBD1 | NM_015845.2 | 1135 | ctgctgcgacaagcccaaattcg | 6345 | 461385 | transcription_regulator | - | - |
| 6546 | C6orf32 | NM_015864.2 | 530 | caggtgtaactccttcattgaaa | 355262 | 802498 | - | - | - |
| 6547 | ZT86 | NM_015871.2 | 300 | ccgatccaaagaccacaagaaaa | 46005 | 453549 | transcription_regulator | transcription co-repressor | - |
| 6548 | OAZIN | NM_015878.3 | 1453 | ttggaagaggttaatcatgttat | 236972 | 622404 | - | - | - |

Figure 46

| No. | Gene | Accession | Pos. | Sequence | ID1 | ID2 | Note | Function |
|---|---|---|---|---|---|---|---|---|
| 6549 | OAZIN | NM_015878.3 | 1457 | aagagagttaatcatgttatcagc | 236974 | 622405 | see also 6548 line | |
| 6550 | OAZIN | NM_015878.3 | 1841 | atgtgggaagattggcttatcttt | 236984 | 622415 | see also 6548 line | |
| 6551 | OAZIN | NM_015878.3 | 1843 | gtgggagaattggcttatctttga | 236985 | 622416 | see also 6548 line | |
| 6552 | SIAT8C | NM_015879.1 | 2302 | tcggacagcgttttacatcaaa | 213030 | 600889 | - | |
| 6553 | SIAT8C | NM_015879.1 | 2334 | ttcttcagcatgtcgatgtaata | 213031 | 600891 | see also 6552 line | |
| 6554 | SIAT8C | NM_015879.1 | 2339 | cagcatgtcgatgtaataaaaaa | 213032 | 600893 | see also 6552 line | |
| 6555 | SIAT8C | NM_015879.1 | 2340 | agcatgtcgatgtaataaaaaat | 213033 | 600894 | see also 6552 line | |
| 6556 | SIAT8C | NM_015879.1 | 2959 | atgtgaagagatccacttgtatg | 213052 | 600914 | see also 6552 line | |
| 6557 | MBTPS2 | NM_015884.1 | 630 | agcagcttattagggaacaagttc | 157701 | 558180 | - | zinc binding |
| 6558 | MBTPS2 | NM_015884.1 | 740 | cagtccagcagctaaggatattt | 157704 | 558186 | see also 6557 line | |
| 6559 | MBTPS2 | NM_015884.1 | 745 | cagcagctaaggatattttgtgc | 157705 | 558187 | see also 6557 line | |
| 6560 | MBTPS2 | NM_015884.1 | 1057 | ttcccagttagagcatacaaacg | 157714 | 558202 | see also 6557 line | |
| 6561 | PI15 | NM_015886.1 | 896 | ttggtcaagccatgttatgatga | 292786 | 695400 | - | |
| 6562 | HOOK1 | NM_015888.3 | 343 | accttgtcaagatgtcaaacagc | 355302 | 685025 | - | |
| 6563 | HOOK1 | NM_015888.3 | 380 | gccatggcacaagttcttcatca | 355303 | 685026 | see also 6562 line | |
| 6564 | HOOK1 | NM_015888.3 | 430 | ttgttaagccgaattaaagagg | 355307 | 685029 | see also 6562 line | |
| 6565 | HOOK1 | NM_015888.3 | 1042 | caggcttgaagctgcaaaagatg | 355328 | 685041 | see also 6562 line | |
| 6566 | ARS2 | NM_015908.3 | 1636 | tcgaccgcagtgttaacattaaa | 276983 | 667120 | - | |
| 6567 | TLP19 | NM_015913.2 | 534 | ctcccataattttgttatggtaa | 265878 | 647604 | - | |
| 6568 | LOC51061 | NM_015914.2 | 618 | ctgtatcatcggagtttggacc | 265345 | 647791 | - | |
| 6569 | LOC51061 | NM_015914.2 | 629 | gagtttggaccaatcgaataca | 265346 | 647793 | see also 6568 line | |
| 6570 | SPG3A | NM_015915.2 | 1575 | ttggtttggacatcatagctagc | 40906 | 468191 | - | spastic paraplegia |
| 6571 | C6orf82 | NM_015921.1 | 538 | gtgaggtgctgatgatgattaaa | 355491 | 647597 | - | |
| 6572 | H105E3 | NM_015922.1 | 537 | gagctctttatagagtgaatta | 219912 | 607298 | - | 3-beta-hydroxy-delta(5)-steroid dehydrogenase |
| 6573 | LISCH7 | NM_015925.3 | 2078 | tggccctgagtcgggaaagttta | 227043 | 614352 | receptor_activity | |
| 6574 | LISCH7 | NM_015925.3 | 2079 | ggccctgagtcgggaaagtttag | 227044 | 614353 | see also 6573 line | |
| 6575 | LISCH7 | NM_015925.3 | 2080 | gccctgagtcgggaaagtttagt | 227045 | 614354 | see also 6573 line | |
| 6576 | NOP5/NOP58 | NM_015934.3 | 1464 | ctgttctaaaaaacgcaaaatag | 180325 | 472567 | - | snoRNP binding |
| 6577 | CGI-07 | NM_015938.2 | 851 | ctgtggaaattgttccaatatgc | 264381 | 648755 | - | |
| 6578 | CGI-07 | NM_015938.2 | 860 | ttgttccaatatgccaaggataat | 264382 | 648757 | see also 6577 line | |
| 6579 | CGI-07 | NM_015938.2 | 1263 | ggccaactgtaacttaaatgatg | 264390 | 648770 | see also 6577 line | |
| 6580 | CGI-07 | NM_015938.2 | 1538 | atgaaggagcacctcgaattagt | 264396 | 648776 | see also 6577 line | |
| 6581 | CGI-07 | NM_015938.2 | 1542 | aggagcacctcgaattagtctgg | 264397 | 648777 | see also 6577 line | |

Figure 46

| | | | | | | | | | | |
|---|---|---|---|---|---|---|---|---|---|---|
| 6582 | ATP6V1H | NM_015941.1 | 699 | atggaaggcagtgacttaaatta | 73368 | 494937 | - | hydrogen-translocating V-type ATPase | - |
| 6583 | ATP6V1H | NM_015941.1 | 700 | tggaaggcagtgacttaaattac | 73369 | 494938 | see also 6582 line | | |
| 6584 | ATP6V1H | NM_015941.1 | 704 | aggcagtgacttaaattactatt | 73370 | 494939 | see also 6582 line | | |
| 6585 | ATP6V1H | NM_015941.1 | 982 | tggcattcagtcctcaaatgtgt | 73380 | 494953 | see also 6582 line | | |
| 6586 | CGI-14 | NM_015944.2 | 244 | cagatcaacggtggatttggtgt | 191321 | 593771 | - | - | - |
| 6587 | SLC35B3 | NM_015948.2 | 710 | tagtgcagtttgccttttactcc | 355567 | 750516 | - | - | - |
| 6588 | CGI-26 | NM_015954.1 | 704 | atggcaggatcagattttattaa | 233987 | 618329 | - | - | - |
| 6589 | MMRP19 | NM_015957.1 | 414 | gtgattcatacccactctaaagc | 355609 | 709093 | - | - | - |
| 6590 | MRPS7 | NM_015971.2 | 320 | atcccttgattgacaaggaatat | 286131 | 658906 | - | - | - |
| 6591 | CAMK2A | NM_015981.2 | 1141 | gtccagttccagcgttcagttaa | 74973 | 496980 | kinase_related | - | - |
| 6592 | CAMK2A | NM_015981.2 | 1144 | cagttccagcgttcagttaatgg | 74974 | 496981 | see also 6591 line | | |
| 6593 | CAMK2A | NM_015981.2 | 1147 | ttccagcgttcagttaatggaat | 74975 | 496982 | see also 6591 line | | |
| 6594 | CAMK2A | NM_015981.2 | 1148 | tccagcgttcagttaatggaatc | 74976 | 496983 | see also 6591 line | | |
| 6595 | UCHL5 | NM_015984.1 | 270 | aagccagttcatgggttaatttt | 190723 | 584236 | - | ubiquitin C-terminal hydrolase | - |
| 6596 | CRLF3 | NM_015986.2 | 876 | gtctgagcagtcgaagaaatata | 248113 | 634637 | - | - | - |
| 6597 | CGI-41 | NM_015997.2 | 717 | atgcctacatcatcgaatttttc | 355706 | 770517 | - | - | - |
| 6598 | CGI-41 | NM_015997.2 | 718 | tgcctacatcatcgaatttttca | 355707 | 770518 | see also 6597 line | | |
| 6599 | CGI-41 | NM_015997.2 | 968 | agccagagctcccgactaacagc | 355709 | 770519 | see also 6597 line | | |
| 6600 | ADIPOR1 | NM_015999.2 | 791 | gtctctcggactttttccaaact | 325963 | 691456 | receptor_acitivity | - | - |
| 6601 | ADIPOR1 | NM_015999.2 | 1033 | gcccaccatgcactttactatcg | 325968 | 691459 | see also 6600 line | | |
| 6602 | TRNT1 | NM_016000.2 | 508 | atctcactataaattctatgttt | 55527 | 475908 | - | - | - |
| 6603 | C20orf9 | NM_016004.1 | 55 | gagctgcggagtaccattctttt | 355783 | 771876 | - | - | - |
| 6604 | C20orf9 | NM_016004.1 | 56 | agctgcggagtaccattctttc | 355784 | 771877 | see also 6603 line | | |
| 6605 | C20orf9 | NM_016004.1 | 59 | tgcggagtaccattctttcaat | 355785 | 771878 | see also 6603 line | | |
| 6606 | C20orf9 | NM_016004.1 | 363 | tggaatcatggttaataatgatg | 355789 | 771888 | see also 6603 line | | |
| 6607 | C20orf9 | NM_016004.1 | 638 | tggctttctatcactcaaagaac | 355795 | 771896 | see also 6603 line | | |
| 6608 | SH3GLB1 | NM_016009.2 | 227 | ctgaatgtaccaaaatatggaca | 355827 | 708314 | apoptosis | - | - |
| 6609 | SH3GLB1 | NM_016009.2 | 794 | atgcacagtgttaccagtatatg | 355838 | 708328 | see also 6608 line | | |
| 6610 | CGI-62 | NM_016010.1 | 479 | aagaacaggcagcacgtattagt | 355859 | 782795 | - | - | - |
| 6611 | CGI-62 | NM_016010.1 | 482 | aacaggcagcacgtattagtaat | 355860 | 782796 | see also 6610 line | | |
| 6612 | CGI-62 | NM_016010.1 | 484 | caggcagcacgtattagtaatga | 355861 | 782797 | see also 6610 line | | |
| 6613 | CGI-62 | NM_016010.1 | 1005 | atgtggcattcgaagaatgattc | 355877 | 782824 | see also 6610 line | | |
| 6614 | CIA30 | NM_016013.1 | 960 | acgattggtcccagttcaatact | 299273 | 668592 | - | binding | - |
| 6615 | CGI-67 | NM_016014.1 | 515 | aaccagtaaaggcaacagaattg | 236064 | 760138 | - | - | - |

Figure 46

| No. | Gene | Accession | Pos | Sequence | ID1 | ID2 | Function | Notes |
|---|---|---|---|---|---|---|---|---|
| 6616 | CGI-67 | NM_016014.1 | 992 | tgggactgaagatgaagtcattg | 236082 | 760154 | | see also 6615 line |
| 6617 | LUC7L2 | NM_016019.1 | 69 | aactcgtcaacgaatcaaattca | 355882 | 752521 | | - |
| 6618 | LUC7L2 | NM_016019.1 | 71 | ctcgtcaacgaatcaaattcagt | 355883 | 752522 | | see also 6617 line |
| 6619 | LUC7L2 | NM_016019.1 | 640 | ctgcacctggatttattgaaat | 355891 | 752540 | | see also 6617 line |
| 6620 | LUC7L2 | NM_016019.1 | 1105 | cggtcctatgagagtgctaatgg | 355896 | 752550 | | see also 6617 line |
| 6621 | TFB1M | NM_016020.1 | 506 | gtgtttcaactcactgattatc | 213910 | 603931 | | - |
| 6622 | UBE2J1 | NM_016021.2 | 432 | tagagataaccttttgaattg | 231200 | 616649 | | - |
| 6623 | UBE2J1 | NM_016021.2 | 705 | gagccatagttctcttagattac | 231212 | 616660 | | see also 6622 line |
| 6624 | UBE2J1 | NM_016021.2 | 1230 | ctcttatattccgacgaatatat | 231229 | 616672 | | see also 6622 line |
| 6625 | DREV1 | NM_016025.2 | 379 | aaggaacacagatcttcttaaac | 355939 | 712001 | | - |
| 6626 | RDH11 | NM_016026.2 | 589 | aaggatccacttccataacctgc | 227333 | 614627 | | - |
| 6627 | CGI-83 | NM_016027.1 | 559 | aagacctcatgattatatgaac | 355972 | 755876 | | - |
| 6628 | CGI-85 | NM_016028.3 | 886 | tgggttgtattgccgaactttca | 356000 | 754529 | | - |
| 6629 | CGI-85 | NM_016028.3 | 913 | ttgaggagaacatgctacttaga | 356002 | 754530 | | see also 6628 line |
| 6630 | CGI-85 | NM_016028.3 | 915 | gaggagaacatgctacttagaca | 356003 | 754531 | | see also 6628 line |
| 6631 | CGI-85 | NM_016028.3 | 1047 | gtgtcaactggtcgagatacagc | 356009 | 754534 | | see also 6628 line |
| 6632 | CGI-90 | NM_016033.1 | 674 | accctaaagatgctacttcaatt | 356046 | 716677 | | - |
| 6633 | CGI-90 | NM_016033.1 | 675 | ccctaaagatgctacttcaattc | 356047 | 716678 | | see also 6632 line |
| 6634 | CGI-90 | NM_016033.1 | 695 | ttcaccttatcggtatttggtgc | 356048 | 716679 | | see also 6632 line |
| 6635 | CGI-100 | NM_016040.2 | 602 | aagtccagactaagcaaaagtgg | 127284 | 529349 | protein carrier | - |
| 6636 | F-LANa | NM_016041.2 | 103 | ccgccgtgcagttggaattgatc | 326451 | 744259 | | - |
| 6637 | F-LANa | NM_016041.2 | 104 | cgccgtgcagttggaattgatca | 326452 | 744260 | | see also 6636 line |
| 6638 | CSL4 | NM_016046.2 | 39 | ccggcgaacgtctgtgtaacttgg | 50148 | 472722 | protein binding | - |
| 6639 | P14 | NM_016047.2 | 329 | gggaaatatggacctattcgtca | 53153 | 472591 | | - |
| 6640 | P14 | NM_016047.2 | 336 | atggacctattcgtcaaatcaga | 53154 | 472592 | | see also 6639 line |
| 6641 | C14orf122 | NM_016049.3 | 465 | ctggggaattgcagaattcttc | 277452 | 666631 | molecular_function unknown | |
| 6642 | CGI-116 | NM_016053.1 | 150 | ctgaccaaggtgccagctattca | 356116 | 721201 | | - |
| 6643 | CGI-116 | NM_016053.1 | 152 | gaccaaggtgccagctattcaac | 356117 | 721202 | | see also 6642 line |
| 6644 | CGI-116 | NM_016053.1 | 159 | gtgccagctattcaacgagaaag | 356118 | 721204 | | see also 6642 line |
| 6645 | MRPL48 | NM_016055.3 | 534 | ttggggacagattatgaatatgg | 285856 | 727733 | | - |
| 6646 | CGI-125 | NM_016060.1 | 142 | ttggaatttgtgcaatgtttagc | 356135 | 721189 | transcription_regulator | |
| 6647 | CGI-125 | NM_016060.1 | 189 | tgcccaaagagttacttcaaag | 356140 | 721193 | | see also 6646 line |
| 6648 | CGI-125 | NM_016060.1 | 190 | gcccaaagagttacttcaaaga | 356141 | 721194 | | see also 6646 line |
| 6649 | CGI-141 | NM_016072.2 | 313 | aggcatgatcttcgaaatttatg | 305507 | 673298 | kinase_related | |
| 6650 | CGI-141 | NM_016072.2 | 314 | ggccatgatcttcgaaatttatg | 305508 | 673299 | | see also 6649 line |

315

Figure 46

| 6651 | CGI-141 | NM_016072.2 | 317 | atgatcttcgaaatttatggatt | 305509 | 673300 | see also 6649 line | | |
|------|---------|-------------|-----|-------------------------|--------|--------|-------------------|--|--|
| 6652 | C13orf9 | NM_016075.1 | 637 | aagatgagaccatcaggtttaaa | 356196 | 729007 | - | - | - |
| 6653 | CDK5RAP1 | NM_016082.3 | 952 | ggctttaccaccaactataaaac | 356231 | 529098 | kinase_related | - | - |
| 6654 | CNR1 | NM_016083.3 | 510 | caggccttcctaccacttcatcg | 238961 | 624016 | - | - | alcoholism、Parkinson disease |
| 6655 | CNR1 | NM_016083.3 | 595 | cacgtgttccaccgcaaagatag | 238963 | 624019 | see also 6654 line | | |
| 6656 | CNR1 | NM_016083.3 | 596 | acgtgttccaccgcaaagatagc | 238964 | 624020 | see also 6654 line | | |
| 6657 | RBM7 | NM_016090.2 | 625 | tccctacctagcagatagacatt | 54081 | 478332 | - | RNA binding | - |
| 6658 | EIF3S6IP | NM_016091.1 | 1193 | acctccagctgcgggagaaatat | 56728 | 535090 | - | - | - |
| 6659 | LOC51122 | NM_016094.1 | 216 | cagcatggtgtggaaggattaac | 356261 | 785410 | - | - | - |
| 6660 | NAT5 | NM_016100.2 | 162 | ttggatccacttacagaaactta | 215173 | 602219 | - | - | - |
| 6661 | NAT5 | NM_016100.2 | 163 | tggatccacttacagaaacttat | 215174 | 602220 | see also 6660 line | | |
| 6662 | NAT5 | NM_016100.2 | 371 | tgctaaacttatggagttactag | 215185 | 602231 | see also 6660 line | | |
| 6663 | FKBP7 | NM_016105.2 | 337 | caccccaaatggtttgttcttgg | 145172 | 548700 | - | - | - |
| 6664 | ZFR | NM_016107.3 | 289 | cagtagctgctcacacagttact | 55855 | 476106 | - | - | - |
| 6665 | ZFR | NM_016107.3 | 823 | ctcagagtgctacttacagtacc | 55867 | 476116 | see also 6664 line | | |
| 6666 | ZFR | NM_016107.3 | 1103 | tggaccacagacttataaagaac | 55873 | 476123 | see also 6664 line | | |
| 6667 | ZFR | NM_016107.3 | 1360 | ttgttagccaagctacttcttca | 55880 | 476131 | see also 6664 line | | |
| 6668 | ZFR | NM_016107.3 | 2679 | ctgacatctccaattattcgaga | 55906 | 476174 | see also 6664 line | | |
| 6669 | PKD2L1 | NM_016112.2 | 933 | tcccactccttcatctactatga | 150726 | 544387 | channel | - | polycystic kidney disease |
| 6670 | PKD2L1 | NM_016112.2 | 1925 | cactcagttccggataatcctcg | 150743 | 544402 | see also 6669 line | | |
| 6671 | ASB3 | NM_016115.3 | 919 | atcttggacttgttaataccact | 315536 | 680359 | - | - | - |
| 6672 | NYREN18 | NM_016118.3 | 139 | aactttggaaacctccatataca | 282665 | 706430 | - | molecular_function unknown | - |
| 6673 | NYREN18 | NM_016118.3 | 1048 | gactggtccacataaaaggaaat | 282689 | 706450 | see also 6672 line | | |
| 6674 | RNF12 | NM_016120.2 | 1188 | aagagttcgtcctggagaatatc | 113260 | 669264 | transcription_regulator | - | - |
| 6675 | RNF12 | NM_016120.2 | 1419 | gaccatgttaaggcagataatga | 113268 | 669276 | see also 6674 line | | |
| 6676 | NY-REN-58 | NM_016122.1 | 1417 | aggatcgtgaattaatacgtaaa | 356314 | 740832 | - | - | - |
| 6677 | LOC51136 | NM_016125.2 | 494 | ctcagaattccgctatctcttca | 356329 | 740569 | - | - | - |
| 6678 | LOC51136 | NM_016125.2 | 595 | ttggaattgggctgctaacaact | 356335 | 740573 | see also 6677 line | | |
| 6679 | MGC8721 | NM_016127.3 | 370 | gtcctctgaagaccagtatgtac | 356353 | 724975 | - | - | - |
| 6680 | COPS4 | NM_016129.2 | 184 | gagcggctctcataaagatctgg | 356374 | 703915 | - | - | - |
| 6681 | COPS4 | NM_016129.2 | 438 | aggagcaggttgcttccataaga | 356379 | 703926 | see also 6680 line | | |
| 6682 | COPS4 | NM_016129.2 | 440 | gagcaggttgcttccataagaca | 356380 | 703927 | see also 6680 line | | |
| 6683 | COPS4 | NM_016129.2 | 767 | gagctctcttacaagacaatagt | 356390 | 703940 | see also 6680 line | | |

Figure 46

| | | | | | | | | |
|---|---|---|---|---|---|---|---|---|
| 6684 | RAB10 | NM_016131.2 | 520 | gacgtacgacctgcttttcaagc | 37221 | 519842 | - | protein transporter |
| 6685 | RAB10 | NM_016131.2 | 572 | aagacctggtcctttcgtt | 37223 | 519844 | see also 6684 line | |
| 6686 | RAB10 | NM_016131.2 | 714 | agcgatttcacacatcacaacc | 37229 | 519848 | see also 6684 line | |
| 6687 | RAB10 | NM_016131.2 | 969 | aagcaaatataaacatcgaaaag | 37239 | 519855 | see also 6684 line | |
| 6688 | MYEF2 | NM_016132.2 | 1292 | tagtagcatggagcgagatttg | 359 | 485201 | transcription_regulator | - |
| 6689 | MYEF2 | NM_016132.2 | 1386 | gggtttgcaggaagaataggatc | 364 | 485209 | see also 6688 line | |
| 6690 | DNCLI1 | NM_016141.1 | 205 | acgaggacgcggcagaacctttgg | 108709 | 519103 | signal_transduction | - |
| 6691 | DNCLI1 | NM_016141.1 | 541 | agccttggactgctttggattct | 108720 | 519108 | see also 6690 line | |
| 6692 | DNCLI1 | NM_016141.1 | 816 | aagtgtgatgccattagtgtatt | 108728 | 519109 | see also 6690 line | |
| 6693 | NSFL1C | NM_016143.3 | 1546 | agctaccaagacccatccaatgc | 356448 | 812920 | - | |
| 6694 | PTD002 | NM_016144.2 | 259 | tagaacaggcagtgtatcacaat | 356466 | 801263 | - | |
| 6695 | PME-1 | NM_016147.1 | 948 | tccatacacctgagaattgaac | 237150 | 594505 | phosphatase | - |
| 6696 | PIAS1 | NM_016166.1 | 98 | tgggggacagtgcggaactaaag | 35067 | 463579 | transcription_regulator , signal_transduction | zinc binding |
| 6697 | PIAS1 | NM_016166.1 | 99 | ggccggacagtgcggaactaaagc | 35068 | 463580 | see also 6696 line | |
| 6698 | PIAS1 | NM_016166.1 | 103 | gacagtgcggaactaaagcaaat | 35069 | 463581 | see also 6696 line | |
| 6699 | PIAS1 | NM_016166.1 | 105 | cagtgcggaactaaagcaaatgg | 35070 | 463582 | see also 6696 line | |
| 6700 | PIAS1 | NM_016166.1 | 108 | tgcggaactaaagcaaatggtta | 35071 | 463583 | see also 6696 line | |
| 6701 | PIAS1 | NM_016166.1 | 189 | cggacgcaaacacgaacttctca | 35074 | 463585 | see also 6696 line | |
| 6702 | PIAS1 | NM_016166.1 | 650 | tggatatttctgggaccaaatgt | 35089 | 463591 | see also 6696 line | |
| 6703 | PIAS1 | NM_016166.1 | 931 | tccatggcagtatatctgtaaa | 35097 | 463607 | see also 6696 line | |
| 6704 | PIAS1 | NM_016166.1 | 1003 | aggaatccggatcattctagagc | 35098 | 463610 | see also 6696 line | |
| 6705 | PIAS1 | NM_016166.1 | 1013 | atcattctagagctttaattaaa | 35100 | 463612 | see also 6696 line | |
| 6706 | PIAS1 | NM_016166.1 | 1239 | tccatatgaacaccttattattg | 35108 | 463615 | see also 6696 line | |
| 6707 | PIAS1 | NM_016166.1 | 1746 | aggagacaatcagcattacaaca | 35124 | 463631 | see also 6696 line | |
| 6708 | TRPC4 | NM_016179.1 | 233 | tggctcagttcattacaaaga | 252050 | 645438 | receptor_acitivity, cha mel | store-operated calcium channel |
| 6709 | TRPC4 | NM_016179.1 | 743 | cccacgaggtccgctgtaactgt | 252070 | 645448 | see also 6708 line | |
| 6710 | TRPC4 | NM_016179.1 | 745 | cacgaggtccgctgtaactgtgt | 252071 | 645449 | see also 6708 line | |
| 6711 | TRPC4 | NM_016179.1 | 1104 | ggccattaagtaccgtcaaaaag | 252079 | 645457 | see also 6708 line | |
| 6712 | TRPC4 | NM_016179.1 | 1111 | aagtaccgtcaaaaagagtttgt | 252080 | 645459 | see also 6708 line | |
| 6713 | TRPC4 | NM_016179.1 | 1287 | aagcccactggactgttcatca | 252086 | 645462 | see also 6708 line | |
| 6714 | ACTL6 | NM_016188.3 | 314 | gtcatgtgcccctaagaaatgg | 276358 | 531595 | - | |
| 6715 | ACTL6 | NM_016188.3 | 482 | atgttcgagcagtacaacattcc | 276359 | 531596 | see also 6714 line | |
| 6716 | ACTL6 | NM_016188.3 | 811 | ctggcataactacatgtgtaatg | 276362 | 531602 | see also 6714 line | |

Figure 46

| | | | | | | | | | |
|---|---|---|---|---|---|---|---|---|---|
| 6717 | TMEFF2 | NM_016192.2 | 1068 | gtcgatgtcaagataacacaact | 284855 | 663440 | - | - | - |
| 6718 | LSM8 | NM_016200.2 | 145 | aaggttttgaccagaccattaat | 46358 | 749226 | - | pre-mRNA splicing factor | - |
| 6719 | PRKAG2 | NM_016203.2 | 883 | atgaggtcacacaagtgttatga | 283148 | 756144 | - | molecular_function unknown | - |
| 6720 | PRKAG2 | NM_016203.2 | 885 | gaggtcacacaagtgttatgaca | 283149 | 756145 | see also 6719 line | | |
| 6721 | PRKAG2 | NM_016203.2 | 888 | gtcacacaagtgttatgacatcg | 283150 | 756146 | see also 6719 line | | |
| 6722 | PRKAG2 | NM_016203.2 | 1194 | aagcctcttcgatgctgtatact | 283159 | 756158 | see also 6719 line | | |
| 6723 | GDF2 | NM_016204.1 | 606 | ctgagctccgactctatgtctcc | 168575 | 565523 | - | growth factor | - |
| 6724 | TRIP4 | NM_016213.2 | 1165 | gcctttgggagttctggtaaatc | 112879 | 638291 | transcription_regulator | transcriptional activator | - |
| 6725 | DCTN4 | NM_016221.2 | 941 | cagaagtgagaatcatgtcaatt | 322468 | 723791 | - | - | - |
| 6726 | DCTN4 | NM_016221.2 | 962 | ttcccaaccttcgctacatgaag | 322469 | 723792 | see also 6725 line | | |
| 6727 | DDX41 | NM_016222.2 | 1147 | gccaccatgccgaagaagattca | 50801 | 483145 | apoptosis | RNA binding | - |
| 6728 | DDX41 | NM_016222.2 | 1149 | caccatgccgaagaagattcaga | 50802 | 483146 | see also 6727 line | | |
| 6729 | SNX9 | NM_016224.3 | 1056 | gagcgtctcctggttaagtttgg | 252881 | 652620 | - | protein transporter | - |
| 6730 | SNX9 | NM_016224.3 | 1839 | cacagtaaccggatctatgatta | 252899 | 652641 | see also 6729 line | | |
| 6731 | SNX9 | NM_016224.3 | 1841 | cagtaaccggatctatgattaca | 252900 | 652642 | see also 6729 line | | |
| 6732 | SNX9 | NM_016224.3 | 1846 | accggatctatgattacaacagt | 252901 | 652643 | see also 6729 line | | |
| 6733 | NLK | NM_016231.1 | 754 | gggtcttccgggaattgaagatg | 94811 | 510342 | kinase_related、transcription_regulator、signal_transduction | - | - |
| 6734 | NLK | NM_016231.1 | 760 | tccgggaattgaagatgttgtgt | 94812 | 510343 | see also 6733 line | | |
| 6735 | NLK | NM_016231.1 | 1084 | tagatgaatcccgtcatatgact | 94819 | 510355 | see also 6733 line | | |
| 6736 | NLK | NM_016231.1 | 1156 | gccgtcattacagcaatgctatt | 94820 | 510357 | see also 6733 line | | |
| 6737 | NLK | NM_016231.1 | 1157 | ccgtcattacagcaatgctattg | 94821 | 510358 | see also 6733 line | | |
| 6738 | NLK | NM_016231.1 | 1630 | ctgtccgacaggttaaagaaatt | 94839 | 510366 | see also 6733 line | | |
| 6739 | ANAPC5 | NM_016237.3 | 259 | tgctgcagggcccagatattaca | 227586 | 711897 | cell_cycle | - | - |
| 6740 | ANAPC5 | NM_016237.3 | 261 | ctgcagggcccagatattacact | 227587 | 711898 | see also 6739 line | | |
| 6741 | ANAPC5 | NM_016237.3 | 332 | ttcagtgcagatcagaatcaaac | 227588 | 711900 | see also 6739 line | | |
| 6742 | ANAPC5 | NM_016237.3 | 990 | cactgccgcttcggtcactatca | 227599 | 711919 | see also 6739 line | | |
| 6743 | ANAPC5 | NM_016237.3 | 993 | tgccgcttcggtcactatcaaca | 227601 | 711920 | see also 6739 line | | |
| 6744 | ANAPC7 | NM_016238.1 | 377 | atgggatcccttcaagacaaaga | 307029 | 709322 | cell_cycle | - | - |
| 6745 | ANAPC7 | NM_016238.1 | 666 | taccatctgttcactagagaaaa | 307038 | 709328 | see also 6744 line | | |
| 6746 | MYO15A | NM_016239.2 | 4997 | cgccatcgccaaggtcttgtatg | 92663 | 509351 | - | structural molecule | deafness |
| 6747 | MYO15A | NM_016239.2 | 4998 | gccatcgccaaggtcttgtatgc | 92664 | 509352 | see also 6746 line | | |
| 6748 | SCARA3 | NM_016240.2 | 269 | gccgctgccagaagaacctatct | 245119 | 776361 | receptor_acitivity | - | - |

Figure 46

| | | | | | | | | |
|---|---|---|---|---|---|---|---|---|
| 6749 | SCARA3 | NM_016240.2 | 273 | ctgccaagaactatcttgc | 245120 | 776362 | see also 6748 line | |
| 6750 | SCARA3 | NM_016240.2 | 1478 | gagaaatccttcgcaatgtcacc | 245134 | 776384 | see also 6748 line | |
| 6751 | IMPG2 | NM_016247.1 | 3234 | aagtttcaggcctgaatgaatt | 178141 | 785061 | - | - |
| 6752 | IMPG2 | NM_016247.1 | 3379 | atgatggaaagtgtgacattag | 178143 | 785067 | see also 6751 line | - |
| 6753 | NDRG2 | NM_016250.1 | 446 | tcctgcagtacctaaatttctct | 282566 | 684281 | - | molecular_function unknown - |
| 6754 | NDRG2 | NM_016250.1 | 449 | tgcagtacctaaatttctctaca | 282567 | 684283 | see also 6753 line | |
| 6755 | BIRC6 | NM_016252.1 | 610 | gccagtgccattgtaaatgaact | 232589 | 615585 | apoptosis | ubiquitin conjugating enzyme - |
| 6756 | BIRC6 | NM_016252.1 | 1418 | tggaagtaagccttgatataaca | 232609 | 615606 | see also 6755 line | |
| 6757 | BIRC6 | NM_016252.1 | 1619 | tagctggagtttattaacatat | 232616 | 615615 | see also 6755 line | |
| 6758 | BIRC6 | NM_016252.1 | 1622 | ctggagtttattaaacatataaa | 232617 | 615616 | see also 6755 line | |
| 6759 | BIRC6 | NM_016252.1 | 1649 | ctgctacctcacccattagtagt | 232618 | 615619 | see also 6755 line | |
| 6760 | BIRC6 | NM_016252.1 | 1654 | acctcacccattagtagtaattc | 232619 | 615620 | see also 6755 line | |
| 6761 | BIRC6 | NM_016252.1 | 2282 | ctgtagtgaatggtgcaaatatt | 232640 | 615637 | see also 6755 line | |
| 6762 | BIRC6 | NM_016252.1 | 3530 | gccccaaacttgttaaaggtatg | 232669 | 615676 | see also 6755 line | |
| 6763 | BIRC6 | NM_016252.1 | 3531 | ccccaaacttgttaaaggtatgg | 232670 | 615677 | see also 6755 line | |
| 6764 | BIRC6 | NM_016252.1 | 4136 | ttctagccttgtgcattagtaat | 232699 | 615698 | see also 6755 line | |
| 6765 | BIRC6 | NM_016252.1 | 5763 | tgctaacaatgcacagtacttt | 232738 | 615744 | see also 6755 line | |
| 6766 | BIRC6 | NM_016252.1 | 6983 | aggttccttgcacgcattgcaaat | 232783 | 615784 | see also 6755 line | |
| 6767 | BIRC6 | NM_016252.1 | 8660 | gagatcaactcatgtttgatttg | 232832 | 615828 | see also 6755 line | |
| 6768 | BIRC6 | NM_016252.1 | 8925 | cagtgccattgccaatgataatg | 232836 | 615840 | see also 6755 line | |
| 6769 | BIRC6 | NM_016252.1 | 9132 | tgctacttgccagttatctgagc | 232845 | 615849 | see also 6755 line | |
| 6770 | BIRC6 | NM_016252.1 | 9274 | aacactgcaagaagtatggtatc | 232852 | 615853 | see also 6755 line | |
| 6771 | BIRC6 | NM_016252.1 | 9900 | tggatgttacggttattacatc | 232870 | 615873 | see also 6755 line | |
| 6772 | BIRC6 | NM_016252.1 | 10647 | gtccatgacagatgatagcaaa | 232897 | 615911 | see also 6755 line | |
| 6773 | BIRC6 | NM_016252.1 | 12145 | atgggtggactggctcttattgc | 232943 | 615953 | see also 6755 line | |
| 6774 | BIRC6 | NM_016252.1 | 12830 | atgtggaacaagccttaacttaag | 232967 | 615967 | see also 6755 line | |
| 6775 | ZNFN1A2 | NM_016260.1 | 404 | agctctagaagaaccctaattga | 45966 | 702986 | transcription regulator | translation regulator - |
| 6776 | ZNFN1A2 | NM_016260.1 | 484 | ccggcttccgaattggtaaactga | 45969 | 702987 | see also 6775 line | |
| 6777 | ZNFN1A2 | NM_016260.1 | 486 | ggcttccgaattggtaaactgaaa | 45970 | 702988 | see also 6775 line | |
| 6778 | ZNFN1A2 | NM_016260.1 | 1228 | agcttattctcaggtctatcatc | 45988 | 703001 | see also 6775 line | |
| 6779 | AK3L1 | NM_016282.2 | 486 | gtgcccttgaggtcattaaaca | 71911 | 493631 | kinase_related | adenylate kinase - |
| 6780 | KIAA1007 | NM_016284.2 | 4147 | aacctttcattagacatcaatga | 356785 | 768407 | - | - |
| 6781 | KIAA1007 | NM_016284.2 | 4353 | cagtaccacgcacatcaatgtct | 356791 | 768408 | see also 6780 line | - |
| 6782 | KIAA1007 | NM_016284.2 | 4829 | gccctgagatggacaagagatta | 356806 | 768425 | see also 6780 line | |

Figure 46

| | | | | | | | | | |
|---|---|---|---|---|---|---|---|---|---|
| 6783 | KIAA1007 | NM_016284.2 | 5026 | ttcttacctacaaatgacttaag | 356811 | 768431 | see also 6780 line | | |
| 6784 | KIAA1007 | NM_016284.2 | 5410 | atcacaaggtgcctaattgaatg | 356824 | 768453 | see also 6780 line | | |
| 6785 | KIAA1007 | NM_016284.2 | 6604 | gagttcctttgtgattaccatta | 356864 | 768490 | see also 6780 line | | |
| 6786 | HP1-BP74 | NM_016287.1 | 110 | aaggcactcccacttatagtagg | 28641 | 460375 | - | - | - |
| 6787 | HP1-BP74 | NM_016287.1 | 825 | tggatccagaaccacaagtaaaa | 28657 | 460385 | see also 6786 line | | |
| 6788 | HP1-BP74 | NM_016287.1 | 1153 | taccactgctctgaagaagtatg | 28664 | 460393 | see also 6786 line | | |
| 6789 | MO25 | NM_016289.2 | 660 | gagaactcctactgttgaataca | 356900 | 748136 | - | - | - |
| 6790 | HSP70-4 | NM_016299.1 | 403 | aagatgttgccagactgatattt | 85083 | 503743 | - | - | - |
| 6791 | HSP70-4 | NM_016299.1 | 1034 | tacagcagatgatatcaacaagg | 85107 | 503762 | see also 6790 line | | |
| 6792 | ARPP-21 | NM_016300.3 | 1126 | atcttgaagcgagataactctag | 276934 | 485486 | - | molecular_function unknown | - |
| 6793 | ARPP-21 | NM_016300.3 | 1136 | gagataactctagtattgataaa | 276935 | 485488 | see also 6792 line | | |
| 6794 | ARPP-21 | NM_016300.3 | 1191 | tagagatgacagacgaagtaaat | 276937 | 485491 | see also 6792 line | | |
| 6795 | ARPP-21 | NM_016300.3 | 1198 | gacagacgaagtaaatcaattga | 276938 | 485493 | see also 6792 line | | |
| 6796 | ARPP-21 | NM_016300.3 | 1200 | cagacgaagtaaatcaattgaag | 276939 | 485494 | see also 6792 line | | |
| 6797 | ARPP-21 | NM_016300.3 | 1335 | atgcaatgagacctataagaaaa | 276942 | 485497 | see also 6792 line | | |
| 6798 | LOC51185 | NM_016302.2 | 529 | aagaacacagtcagatggaatcc | 191190 | 584509 | - | - | - |
| 6799 | LOC51185 | NM_016302.2 | 877 | ttgtcttcctattgatgatgtat | 191201 | 584522 | see also 6798 line | | |
| 6800 | LOC51186 | NM_016303.1 | 552 | accatccttacccctatttaatg | 356931 | 702947 | - | - | - |
| 6801 | DNAJB11 | NM_016306.3 | 817 | atggagtacccctttattggaga | 299397 | 534633 | - | chaperone | - |
| 6802 | DNAJB11 | NM_016306.3 | 876 | acggttccgaatcaaagttgtca | 299398 | 534635 | see also 6801 line | | |
| 6803 | DNAJB11 | NM_016306.3 | 1102 | aagggctctttgataatcacttt | 299405 | 534641 | see also 6801 line | | |
| 6804 | CED-6 | NM_016315.1 | 1198 | aaccttttgacccatttaactgt | 226447 | 729694 | apoptosis | - | - |
| 6805 | REV1L | NM_016316.1 | 359 | tagtggagttgccatctatgtta | 17558 | 598218 | - | - | - |
| 6806 | REV1L | NM_016316.1 | 362 | tggagttgccatctatgttaatg | 17559 | 598220 | see also 6805 line | | |
| 6807 | REV1L | NM_016316.1 | 1648 | cagcagatataccagattcatca | 17590 | 598253 | see also 6805 line | | |
| 6808 | REV1L | NM_016316.1 | 1766 | tggcattaagaacggaatgtttt | 17594 | 598256 | see also 6805 line | | |
| 6809 | REV1L | NM_016316.1 | 2619 | atgtttcatacaatgaaactaaa | 17624 | 598287 | see also 6805 line | | |
| 6810 | RAB14 | NM_016322.2 | 294 | ggctgattgtcctcacacaattg | 109910 | 519898 | - | protein transporter | - |
| 6811 | SFMBT1 | NM_016329.1 | 790 | gaggaatcctttagatctcattg | 356961 | 708227 | - | - | - |
| 6812 | SFMBT1 | NM_016329.1 | 2076 | gggctactgttgagatagtgaaa | 357009 | 708289 | see also 6811 line | | |
| 6813 | SFMBT1 | NM_016329.1 | 2868 | aactttgccatcacatagagagg | 357033 | 708311 | see also 6811 line | | |
| 6814 | ANC_2H01 | NM_016331.1 | 1807 | ttcagctgacttgcctcataaat | 357077 | 753152 | transcription_regulator | - | - |
| 6815 | SRRM2 | NM_016333.2 | 4549 | gggtcttctccaggacttagaga | 357164 | 786810 | - | - | - |
| 6816 | IPO11 | NM_016338.3 | 1086 | aagttggtgaaggcgttacattt | 269165 | 651164 | - | - | - |
| 6817 | IPO11 | NM_016338.3 | 1161 | atgcttataagccatccaaaaat | 269171 | 651171 | see also 6816 line | | |

Figure 46

| | | | | | | | | | |
|---|---|---|---|---|---|---|---|---|---|
| 6818 | IPO11 | NM_016338.3 | 1162 | tgcttataagccatccaaaaatt | 269172 | 651172 | see also 6816 line | | |
| 6819 | IPO11 | NM_016338.3 | 1206 | ctcttgaagcccataagattaag | 269173 | 651173 | see also 6816 line | | |
| 6820 | IPO11 | NM_016338.3 | 1226 | aagatggcattcttcacatatcc | 269174 | 651174 | see also 6816 line | | |
| 6821 | IPO11 | NM_016338.3 | 1270 | aagattagtctctcattatttcc | 269177 | 651179 | see also 6816 line | | |
| 6822 | IPO11 | NM_016338.3 | 1727 | atctgtaacttgcttcaagatca | 269189 | 651200 | see also 6816 line | | |
| 6823 | PDZGEF2 | NM_016340.1 | 372 | aagagcacgctatgagagataca | 296362 | 664518 | signal_transduction | - | - |
| 6824 | PDZGEF2 | NM_016340.1 | 488 | tgcctccttgcagttttggtaag | 296365 | 664524 | see also 6823 line | | |
| 6825 | PLCE1 | NM_016341.2 | 2645 | agccgatggcagtttataattgg | 198498 | 590693 | kinase_related、signal transduction、gpcr | - | - |
| 6826 | PLCE1 | NM_016341.2 | 3891 | ttgagctgttcaaatcattcagt | 198525 | 590714 | see also 6825 line | | |
| 6827 | PLCE1 | NM_016341.2 | 4665 | ctgacctgccaatcatcatatcg | 198553 | 590736 | see also 6825 line | | |
| 6828 | PLCE1 | NM_016341.2 | 5327 | aagtccatttttggcaacaatcc | 198565 | 590749 | see also 6825 line | | |
| 6829 | NIN | NM_016350.3 | 522 | acctcttgggcagggtacatttt | 148991 | 550533 | - | - | - |
| 6830 | NIN | NM_016350.3 | 545 | gaccaatttaaagaagcattaat | 148993 | 550535 | see also 6829 line | | |
| 6831 | NIN | NM_016350.3 | 630 | aagctcagcccaaatatgttaga | 148996 | 550538 | see also 6829 line | | |
| 6832 | CPA4 | NM_016352.1 | 671 | gtggccaatcctgatggatatgt | 185632 | 579855 | - | carboxypeptidase A | - |
| 6833 | ZDHHC2 | NM_016353.2 | 631 | ctccgtctgtgataaatgtattt | 328097 | 693641 | - | - | - |
| 6834 | ZDHHC2 | NM_016353.2 | 632 | tccgtctgtgataaatgtatttt | 328098 | 693642 | see also 6833 line | | |
| 6835 | ZDHHC2 | NM_016353.2 | 814 | tactcaagccaagttccatatta | 328104 | 693651 | see also 6833 line | | |
| 6836 | ZDHHC2 | NM_016353.2 | 819 | aagccaagttccatattatgttt | 328105 | 693652 | see also 6833 line | | |
| 6837 | ZDHHC2 | NM_016353.2 | 824 | aagttccatattatgtttttatt | 328108 | 693653 | see also 6833 line | | |
| 6838 | DDX47 | NM_016355.2 | 775 | aggatacctacctggtttatatt | 59336 | 483287 | - | - | - |
| 6839 | DDX47 | NM_016355.2 | 934 | taggatcccttaataagtttaag | 59345 | 483293 | see also 6838 line | | |
| 6840 | DCDC2 | NM_016356.1 | 797 | gtgggatcatgtactacaaatgg | 315693 | 680872 | - | - | - |
| 6841 | EPLIN | NM_016357.1 | 1313 | ggccaaccagcaggtgtttcaca | 322513 | 686947 | - | - | - |
| 6842 | RAB9B | NM_016370.1 | 406 | ggggtagagttcttaaatcgaga | 110413 | 518433 | signal_transduction | protein transporter | - |
| 6843 | RAB9B | NM_016370.1 | 651 | gggtaacaaggtagacaaagagg | 110418 | 518440 | see also 6842 line | | |
| 6844 | RBP1L1 | NM_016374.4 | 2271 | tcctttacttggtgcattactgc | 18925 | 810107 | - | - | - |
| 6845 | RBP1L1 | NM_016374.4 | 2290 | ctgcggatggaatgtgagatacg | 18927 | 810108 | see also 6844 line | | |
| 6846 | CD244 | NM_016382.1 | 855 | ttggtgatcatcgtgattctaag | 129819 | 634374 | receptor_acitivity | - | - |
| 6847 | TH1L | NM_016397.2 | 1435 | ctgcagctgcttgttaagctttt | 357357 | 710660 | - | - | - |
| 6848 | RNF141 | NM_016422.3 | 388 | cgggtggtctgtaccaagattaa | 357421 | 466360 | - | - | - |
| 6849 | RNF141 | NM_016422.3 | 389 | gggtggtctgtaccaagattaac | 357422 | 466361 | see also 6848 line | | |
| 6850 | LUC7A | NM_016424.2 | 239 | gggaccacgagagcgtttgtaaa | 301880 | 723825 | - | - | - |
| 6851 | LUC7A | NM_016424.2 | 242 | accacgagagcgtttgtaaatat | 301881 | 723827 | see also 6850 line | | |
| 6852 | LUC7A | NM_016424.2 | 244 | cacgagagcgtttgtaaatatta | 301882 | 723828 | see also 6850 line | | |
| 6853 | LUC7A | NM_016424.2 | 245 | acgagagcgtttgtaaatattat | 301883 | 723829 | see also 6850 line | | |

321

Figure 46

| | | | | | | | | | |
|---|---|---|---|---|---|---|---|---|---|
| 6854 | LUC7A | NM_016424.2 | 282 | tcctgcggaattgttcacaaata | 301884 | 723832 | see also 6850 line | | |
| 6855 | LUC7A | NM_016424.2 | 805 | atgggctatgccaaaattaaagc | 301902 | 723851 | see also 6850 line | | |
| 6856 | DSCR5 | NM_016430.2 | 113 | ccgtcgccattgccagaaagagc | 210208 | 708650 | - | | |
| 6857 | DSCR5 | NM_016430.2 | 122 | ttgccagaaagagcgatttatgg | 210209 | 708651 | see also 6856 line | | |
| 6858 | GLTP | NM_016433.2 | 242 | ctcccatcaaggcagacataagc | 305706 | 709349 | | | |
| 6859 | ARTS-1 | NM_016442.2 | 906 | atgctgtgccagacaagataaat | 156065 | 556803 | | | Alzheimer disease |
| 6860 | PLEK2 | NM_016445.1 | 230 | tgcccctgcctggagtatgaaaa | 309759 | 681446 | | zinc binding | - |
| 6861 | RAMP | NM_016448.1 | 947 | ctggctctacttattgctaat | 357528 | 740492 | | | - |
| 6862 | COPB | NM_016451.3 | 998 | aaggtctgtcatgctaatccatc | 268843 | 650897 | | protein transporter | - |
| 6863 | COPB | NM_016451.3 | 2105 | aagaagccaattactgatgatga | 268875 | 650926 | see also 6862 line | | - |
| 6864 | COPB | NM_016451.3 | 2825 | cagatgtgggccgaatttgaatg | 268895 | 650947 | see also 6862 line | | - |
| 6865 | LOC51234 | NM_016454.2 | 353 | ctccatcttcctactatgatgg | 357567 | 725721 | | | - |
| 6866 | TMEM9 | NM_016456.2 | 190 | ctgaagatatccggtgcaaatgc | 284892 | 673519 | | | - |
| 6867 | TMEM9 | NM_016456.2 | 193 | aagatatccggtgcaaatgcatc | 284893 | 673520 | see also 6866 line | | - |
| 6868 | C20orf111 | NM_016470.6 | 325 | acgggtctacaaggagagtcttca | 357625 | 717937 | | | - |
| 6869 | C20orf111 | NM_016470.6 | 602 | aaggctcccatcagagagtaaga | 357629 | 717940 | see also 6868 line | | - |
| 6870 | LOC51244 | NM_016474.3 | 625 | ctggagatggataaaaaatcttca | 357647 | 778103 | | | - |
| 6871 | LOC51244 | NM_016474.3 | 828 | tgcctttgcccgagacaaagagt | 357650 | 778105 | see also 6870 line | | - |
| 6872 | C14orf100 | NM_016475.2 | 302 | ttggttcttcattgaatggtact | 357665 | 714881 | | | - |
| 6873 | PAIP2 | NM_016480.2 | 176 | tagcccaagcatcatcaatgaag | 357728 | 724951 | | | - |
| 6874 | PAIP2 | NM_016480.2 | 201 | gtgattattaacggtcattctca | 357730 | 724954 | see also 6873 line | | - |
| 6875 | LOC51248 | NM_016484.3 | 340 | gagctcagttggagtttaacatc | 316691 | 679777 | | | - |
| 6876 | LOC51248 | NM_016484.3 | 341 | agctcagttggagtttaacatc | 316692 | 679778 | see also 6875 line | | - |
| 6877 | LOC51248 | NM_016484.3 | 465 | gtgaatgatgtgatttccaaga | 316693 | 679781 | see also 6875 line | | - |
| 6878 | LOC51248 | NM_016484.3 | 547 | tgcgcttctttcctacaattat | 316694 | 679782 | see also 6875 line | | - |
| 6879 | C6orf55 | NM_016485.3 | 335 | ctggacgatttcacaaaaaacatg | 357787 | 716258 | | | - |
| 6880 | C6orf55 | NM_016485.3 | 336 | tggacgatttcacaaaaaacatga | 357788 | 716259 | see also 6879 line | | - |
| 6881 | KBTBD4 | NM_016506.3 | 935 | aagatgactccatcagtgtaagt | 132135 | 720643 | | | - |
| 6882 | KBTBD4 | NM_016506.3 | 1322 | tggacttctttaccaaaccttcc | 132145 | 720656 | see also 6881 line | | - |
| 6883 | CRK7 | NM_016507.1 | 1954 | gacatggatagtccaaaagaaac | 77529 | 488349 | kinase_related | protein serine/threonine kinase | |
| 6884 | CRK7 | NM_016507.1 | 2666 | ttggacttgctcggtctataac | 77549 | 488362 | see also 6883 line | | - |
| 6885 | CRK7 | NM_016507.1 | 2667 | tggacttgctcggtctataact | 77550 | 488363 | see also 6883 line | | - |
| 6886 | CRK7 | NM_016507.1 | 2669 | gacttgctcggtctataactct | 77551 | 488364 | see also 6883 line | | - |
| 6887 | CRK7 | NM_016507.1 | 2700 | tcgccttaacaacaaagtca | 77554 | 488365 | see also 6883 line | | - |
| 6888 | VPS54 | NM_016516.1 | 175 | atgttgcccatctctagttaca | 357906 | 752784 | - | | |

Figure 46

| 6889 | VPS54 | NM_016516.1 | 496 | tggagcaagtacctaagattttt | 357918 | 752796 | see also 6888 line | | |
|------|-------|-------------|-----|-----------------------|--------|--------|-------------------|---|---|
| 6890 | VPS54 | NM_016516.1 | 499 | agcaagtacctaagatttttatg | 357919 | 752797 | see also 6888 line | | |
| 6891 | VPS54 | NM_016516.1 | 687 | cagatctctctacgttcagaagc | 357924 | 752803 | see also 6888 line | | |
| 6892 | VPS54 | NM_016516.1 | 780 | atgcttcgagataaaattgcaca | 357926 | 752807 | see also 6888 line | | |
| 6893 | VPS54 | NM_016516.1 | 1849 | gccatgatcgagctgtcaaattt | 357946 | 752841 | see also 6888 line | | |
| 6894 | VPS54 | NM_016516.1 | 2352 | aagtacttcaattcaagaagttg | 357964 | 752854 | see also 6888 line | | |
| 6895 | VPS54 | NM_016516.1 | 2363 | ttcaagaagttgccagttagttc | 357965 | 752855 | see also 6888 line | | |
| 6896 | VPS54 | NM_016516.1 | 2594 | agctaagcttgtagcgataatgg | 357979 | 752864 | see also 6888 line | | |
| 6897 | VPS54 | NM_016516.1 | 2598 | aagcttgtagcgataatggatag | 357980 | 752865 | see also 6888 line | | |
| 6898 | VPS54 | NM_016516.1 | 2599 | agcttgtagcgataatggatagc | 357981 | 752866 | see also 6888 line | | |
| 6899 | PIPOX | NM_016518.1 | 1043 | cacccaaagtatgacaacattgt | 225823 | 612704 | - | sarcosine oxidase | - |
| 6900 | UBAP1 | NM_016525.3 | 924 | aacccaatggctttataacctta | 358033 | 713281 | - | - | - |
| 6901 | UBAP1 | NM_016525.3 | 925 | acccaatggctttataaccttac | 358034 | 713282 | see also 6900 line | | |
| 6902 | UBAP1 | NM_016525.3 | 1497 | agcagattctcgactatctcttt | 358043 | 713298 | see also 6900 line | | |
| 6903 | UBAP1 | NM_016525.3 | 1499 | cagattctcgactatctctttgc | 358044 | 713299 | see also 6900 line | | |
| 6904 | ATP8A2 | NM_016529.3 | 830 | gcctctcaagagatcaaatgttg | 73723 | 495296 | - | plasma membrane cation-transporting ATPase | - |
| 6905 | ATP8A2 | NM_016529.3 | 2017 | acgccatcgcacagttttcctac | 73748 | 495341 | see also 6904 line | | |
| 6906 | ATP8A2 | NM_016529.3 | 2018 | cgccatcgcacagttttcctact | 73749 | 495342 | see also 6904 line | | |
| 6907 | KLF3 | NM_016531.2 | 705 | acctgtaattgaatcatatgaga | 5961 | 461016 | transcription_regulator | translation regulator | - |
| 6908 | MST4 | NM_016542.2 | 279 | cagaagaactgttcacaaaatta | 92044 | 488597 | kinase_related | - | - |
| 6909 | MST4 | NM_016542.2 | 939 | ttggagactttactaagtctttt | 92058 | 488612 | see also 6908 line | | |
| 6910 | LOC51277 | NM_016544.1 | 514 | agcaaagggttcctgtactttga | 109506 | 519088 | signal_transduction | - | - |
| 6911 | LOC51277 | NM_016544.1 | 518 | aagggttcctgtactttgaaact | 109507 | 519089 | see also 6910 line | | |
| 6912 | C1RL | NM_016546.1 | 1255 | aggtgttttctgacaatatgttc | 181657 | 805498 | - | - | - |
| 6913 | GOLPH2 | NM_016548.2 | 215 | acgggcgtcgcagcatgaagtcg | 323550 | 728900 | - | - | - |
| 6914 | BFAR | NM_016561.1 | 779 | gagtaaatggaaggttgcttta | 289781 | 720568 | apoptosis | - | - |
| 6915 | TLR7 | NM_016562.2 | 1783 | tccaacaaccggcttgatttact | 246947 | 528144 | receptor_acitivity | transmembrane receptor | - |
| 6916 | TLR7 | NM_016562.2 | 2180 | tggccaaaaatgggctcaaatct | 246956 | 528159 | see also 6915 line | | |
| 6917 | PTX1 | NM_016570.1 | 282 | aagtagacaaggatttttctagc | 358160 | 722046 | - | - | - |
| 6918 | PTX1 | NM_016570.1 | 1069 | ttcaacaacaggcatgttacatg | 358182 | 722079 | see also 6917 line | | |
| 6919 | GLULD1 | NM_016571.1 | 856 | tggcattagctcagctgataatg | 232272 | 617232 | - | - | - |
| 6920 | GMPR2 | NM_016576.2 | 478 | atcattgctgccaatatggatac | 223213 | 610308 | - | - | - |
| 6921 | RAB6B | NM_016577.1 | 729 | ctgataagaggcagataaccatc | 110379 | 784546 | signal_transduction | protein transporter | - |

323

Figure 46

| | | | | | | | | | |
|---|---|---|---|---|---|---|---|---|---|
| 6922 | RAB6B | NM_016577.1 | 774 | aagaactgagcgtcatgttcatt | 110380 | 784547 | | | - |
| 6923 | PCDH12 | NM_016580.2 | 1770 | ggccctgatgagaccaaacatgc | 149476 | 551021 | - | calcium ion binding | |
| 6924 | PCDH12 | NM_016580.2 | 4141 | aaccaaaccaccgaggaaataag | 149511 | 551066 | see also 6923 line | | - |
| 6925 | PCDH12 | NM_016580.2 | 4142 | accaaaccaccgaggaaataagt | 149512 | 551067 | see also 6923 line | | |
| 6926 | MBIP | NM_016586.1 | 205 | gtggtgaaaattacaatcgattg | 294176 | 756484 | | | - |
| 6927 | MBIP | NM_016586.1 | 535 | gacagacgaatatcgcatttat | 294184 | 756494 | see also 6926 line | | |
| 6928 | MBIP | NM_016586.1 | 696 | ttctagagttgtgaatacatacg | 294189 | 756502 | see also 6926 line | | - |
| 6929 | NRN1 | NM_016588.2 | 661 | aagttgaacggcagatatatttc | 358234 | 769877 | - | | |
| 6930 | NRN1 | NM_016588.2 | 664 | ttgaacgtgcagatatatttcact | 358235 | 769878 | see also 6929 line | | - |
| 6931 | NRN1 | NM_016588.2 | 846 | gtgcacatactgggaggatttcc | 358236 | 769882 | see also 6929 line | | |
| 6932 | NRN1 | NM_016588.2 | 898 | tgccaggaaggggcgaaagatat | 358238 | 769883 | see also 6929 line | | |
| 6933 | NRN1 | NM_016588.2 | 899 | gccaggaagggcgaaagatatg | 358239 | 769884 | see also 6929 line | | |
| 6934 | NRN1 | NM_016588.2 | 958 | atccaaggcagcttattcgaact | 358240 | 769886 | see also 6929 line | | - |
| 6935 | PART1 | NM_016590.2 | 69 | aaactcaattacgactacatatgc | 282921 | 560028 | | molecular_function unknown | - |
| 6936 | CYP39A1 | NM_016593.3 | 1223 | ctcctggtgtcattactagaaaa | 221051 | 608144 | - | human cytochromes P450 family | Alzheimer disease |
| 6937 | ZDHHC3 | NM_016598.1 | 439 | tagcatcatcaacggaattgtgt | 323573 | 671033 | | | - |
| 6938 | ZDHHC3 | NM_016598.1 | 1171 | ggcagaccgtaccagtatgtgg | 323580 | 671044 | see also 6937 line | | |
| 6939 | MYOZ2 | NM_016599.2 | 306 | aagatgcgtcaaagaagatctga | 125629 | 686979 | phosphatase | | - |
| 6940 | C5orf5 | NM_016603.1 | 465 | ctcctcccttccttgagtaact | 297702 | 665242 | | | |
| 6941 | C5orf5 | NM_016603.1 | 578 | tccgccagttgtggactatatt | 297707 | 665246 | see also 6940 line | | |
| 6942 | C5orf5 | NM_016603.1 | 579 | ccgccacgttgtgactatattg | 297708 | 665247 | see also 6940 line | | |
| 6943 | C5orf5 | NM_016603.1 | 580 | cgccacgttgtggactatattga | 297709 | 665248 | see also 6940 line | | |
| 6944 | C5orf5 | NM_016603.1 | 925 | gccaatgtagcatcacatcatga | 297717 | 665260 | see also 6940 line | | |
| 6945 | C5orf5 | NM_016603.1 | 1744 | ctggattctagtagccaagatatg | 297743 | 665285 | see also 6940 line | | |
| 6946 | C5orf5 | NM_016603.1 | 2758 | atgttacagccaatcatagaagg | 297771 | 665317 | see also 6940 line | | |
| 6947 | C5orf7 | NM_016604.2 | 4636 | agccaatgggctcaaactcaag | 358339 | 739507 | | | - |
| 6948 | C5orf7 | NM_016604.2 | 4637 | gccaatggtgctcaaactcaagg | 358340 | 739508 | see also 6947 line | | |
| 6949 | LOC51308 | NM_016606.1 | 310 | ccgagacaagagagctatgagacc | 358361 | 754464 | | | - |
| 6950 | SLC22A17 | NM_016609.2 | 445 | tcctcaactaccgcaacatctgg | 274354 | 514692 | | | - |
| 6951 | TTRAP | NM_016614.2 | 963 | ttgtttgatcgaatattttc | 113378 | 638449 | | receptor_activity, transcription_regulator, signal_transduction | - |
| 6952 | SLC6A13 | NM_016615.2 | 1603 | cgctgacctacaacaagaagtac | 255969 | 639967 | | neurotransmitter:sodium symporter | - |
| 6953 | BHC80 | NM_016621.2 | 1498 | gtctctctagggtttggtaacacatg | 22727 | 456359 | transcription_regulator | | - |

Figure 46

| | | | | | | | | | |
|---|---|---|---|---|---|---|---|---|---|
| 6954 | BHC80 | NM_016621.2 | 2248 | gagctgcacagcgaactgtaacc | 22734 | 456368 | see also 6953 line | | |
| 6955 | BM-009 | NM_016623.2 | 573 | tccactagtaggcaaattaaaga | 358534 | 754265 | - | - | - |
| 6956 | BM-009 | NM_016623.2 | 575 | cactagtaggcaaattaaagaaa | 358535 | 754266 | see also 6955 line | | |
| 6957 | BM-009 | NM_016623.2 | 1084 | agggtaatggtgggtgtcataat | 358547 | 754282 | see also 6955 line | | |
| 6958 | BM-009 | NM_016623.2 | 1085 | gggtaatggtgggtgtcataata | 358548 | 754283 | see also 6955 line | | |
| 6959 | BM-009 | NM_016623.2 | 1091 | tggtgggtgtcataatactctat | 358549 | 754284 | see also 6955 line | | |
| 6960 | BM-009 | NM_016623.2 | 1093 | gtgggtgtcataatactctatga | 358550 | 754285 | see also 6955 line | | |
| 6961 | MGC12197 | NM_016625.2 | 981 | tgctatcaagtaccaagatgaca | 358584 | 573379 | - | - | - |
| 6962 | LOC51321 | NM_016627.1 | 869 | acccatgagatcggacacatt | 157365 | 559951 | - | - | - |
| 6963 | C21orf66 | NM_016631.2 | 2721 | ttgcaagtgaccacaatgtgaaa | 23481 | 452987 | transcription_regulator | DNA binding | - |
| 6964 | MRPS30 | NM_016640.2 | 1087 | atgttactcgaccttttgtctcc | 227161 | 658824 | apoptosis | - | - |
| 6965 | MIR16 | NM_016641.3 | 495 | atgcacgataacacagtagatag | 198925 | 708750 | - | - | - |
| 6966 | MIR16 | NM_016641.3 | 496 | tgcacgataacacagtagatagg | 198926 | 708751 | see also 6965 line | | |
| 6967 | ZAK | NM_016653.1 | 699 | aggtttggaaggattacaagtag | 107786 | 495484 | kinase_related、cell_cycle、signal_transduction | - | - |
| 6968 | ZAK | NM_016653.1 | 1548 | gacaaagccaccatttgtaatgg | 107815 | 495509 | see also 6967 line | | |
| 6969 | ADD3 | NM_016824.1 | 146 | aggaacatgtctcctgatctacg | 121181 | 526499 | - | - | Alzheimer disease |
| 6970 | PER3 | NM_016831.1 | 601 | aaggttagtgcacatttctgaac | 254801 | 639406 | signal_transduction、transcription_regulator | signal transducer | - |
| 6971 | PER3 | NM_016831.1 | 1130 | atggttgccatacaccaaaaagt | 254816 | 639425 | see also 6970 line | | |
| 6972 | POLA | NM_016937.1 | 926 | gtctcttgttgggacattgatca | 35644 | 463846 | - | Nuclear factor 1 C-type-7 | - |
| 6973 | POLA | NM_016937.1 | 928 | ctcttgttgggacattgatcaag | 35645 | 463847 | see also 6972 line | | |
| 6974 | POLA | NM_016937.1 | 1498 | aggaccttgttggcttgaagtaa | 35661 | 463865 | see also 6972 line | | |
| 6975 | POLA | NM_016937.1 | 2960 | gagattttgatgcatacgaaaga | 35690 | 463907 | see also 6972 line | | |
| 6976 | POLA | NM_016937.1 | 4037 | tactatgatggctggttgatatg | 35720 | 463952 | see also 6972 line | | |
| 6977 | C21orf6 | NM_016940.1 | 248 | gagaatgagctcatagtgaatga | 192177 | 585901 | phosphatase | molecular_function unknown | - |
| 6978 | PARD6A | NM_016948.1 | 124 | tcgtcgaggtgaagagcaaattt | 308355 | 675122 | cell_cycle | - | - |
| 6979 | SPOCK3 | NM_016950.1 | 904 | tacaaactatgacctgctattgg | 153179 | 663346 | - | - | - |
| 6980 | TBX22 | NM_016954.2 | 467 | tggattccaaacgctataggtac | 12772 | 449177 | - | transcription factor | - |
| 6981 | SLA/LP | NM_016955.1 | 603 | gagcttggacaaaaattttatgg | 49238 | 774550 | - | - | - |
| 6982 | SLA/LP | NM_016955.1 | 626 | ttccagtaggtggtgctataatt | 49239 | 774551 | see also 6981 line | | |

Figure 46

| 6983 | HOXC13 | NM_017410.2 | 145 | ctggccggagagccttatgtacg | 5177 | 444792 | transcription_regulator | RNA polymerase II transcription factor | - |
|---|---|---|---|---|---|---|---|---|---|
| 6984 | FZD3 | NM_017412.2 | 1257 | atcgagtagcctgcaatgcatcc | 242503 | 630562 | receptor_acitivity、gpcr、signal_transduction | frizzled receptor | schizophrenia |
| 6985 | FZD3 | NM_017412.2 | 1653 | ctggcattatatccctaaacaga | 242515 | 630585 | see also 6984 line | | |
| 6986 | FZD3 | NM_017412.2 | 1988 | tgctttgaatgggccagtttttt | 242528 | 630599 | see also 6984 line | | |
| 6987 | IL1RAPL2 | NM_017416.1 | 1317 | gtggagaagcataataatacaga | 123290 | 527771 | receptor_acitivity | - | - |
| 6988 | IL1RAPL2 | NM_017416.1 | 1622 | aagaactggcaggtcacattaga | 123297 | 527777 | see also 6987 line | | |
| 6989 | IL1RAPL2 | NM_017416.1 | 2170 | atcgtgctaactccagactatat | 123311 | 527797 | see also 6987 line | | |
| 6990 | IL1RAPL2 | NM_017416.1 | 2173 | gtgctaactccagactatattct | 123312 | 527798 | see also 6987 line | | |
| 6991 | IL1RAPL2 | NM_017416.1 | 2194 | ctcagacggggatggagtatttt | 123313 | 527799 | see also 6987 line | | |
| 6992 | IL1RAPL2 | NM_017416.1 | 2200 | cggggatggagtattttcgaact | 123316 | 527800 | see also 6987 line | | |
| 6993 | SIX4 | NM_017420.1 | 604 | ggcgaggagacggtgtattgttt | 11292 | 448442 | transcription_regulator | transcription factor | - |
| 6994 | SIX4 | NM_017420.1 | 608 | aggagacggtgtattgtttcaag | 11294 | 448443 | see also 6993 line | | |
| 6995 | SIX4 | NM_017420.1 | 1421 | tccttaatgggagcattggattc | 11317 | 448457 | see also 6993 line | | |
| 6996 | SIX4 | NM_017420.1 | 1899 | atctgtcagcaacactaactatg | 11332 | 448471 | see also 6993 line | | |
| 6997 | COQ3 | NM_017421.2 | 800 | tcctatgccttgggaattgtttt | 214016 | 604452 | - | hexaprenyldihydroxybenzoate methyltransferase | - |
| 6998 | GALNT7 | NM_017423.1 | 268 | aagtggacttagagtctattaga | 211156 | 599865 | - | - | - |
| 6999 | GALNT7 | NM_017423.1 | 270 | gtggacttagagtctattagaag | 211157 | 599866 | see also 6998 line | | |
| 7000 | GALNT7 | NM_017423.1 | 271 | tggacttagagtctattagaaga | 211158 | 599867 | see also 6998 line | | |
| 7001 | SPA17 | NM_017425.2 | 154 | aacacccactaccgaattccaca | 298645 | 666176 | kinase_related、signal_transduction | cAMP-dependent protein kinase、regulator | - |
| 7002 | SPA17 | NM_017425.2 | 223 | gagcaaccggacaatataccagc | 298649 | 666180 | see also 7001 line | | |
| 7003 | BCDO1 | NM_017429.1 | 512 | tccggacccctgcaaaaacatat | 224474 | 711804 | - | - | - |
| 7004 | MYO3A | NM_017433.3 | 1518 | ttggaaaggctaataacagaacc | 92982 | 446193 | kinase_related | - | - |
| 7005 | MYO3A | NM_017433.3 | 2130 | tggtcactagaggagaaacaatt | 92999 | 446214 | see also 7004 line | | |
| 7006 | MYO3A | NM_017433.3 | 2358 | agctgtgcattaacattgcaaat | 93009 | 446220 | see also 7004 line | | |
| 7007 | MYO3A | NM_017433.3 | 2451 | tggatgctagagttattgaatat | 93013 | 446224 | see also 7004 line | | |
| 7008 | MYO3A | NM_017433.3 | 3525 | atcaggaattcgactacaagaaa | 93042 | 446257 | see also 7004 line | | |
| 7009 | MYO3A | NM_017433.3 | 3527 | caggaattcgactacaagaaaaa | 93043 | 446258 | see also 7004 line | | |
| 7010 | MYO3A | NM_017433.3 | 3528 | aggaattcgactacaagaaaaac | 93044 | 446259 | see also 7004 line | | |
| 7011 | CPSF2 | NM_017437.1 | 682 | ctgccagctggtcatatgatagg | 49980 | 473326 | - | - | - |
| 7012 | MDM1 | NM_017440.2 | 638 | atgccttgagaaattctgaatat | 324570 | 701589 | - | - | - |

Figure 46

| ID | Gene | Accession | Pos | Sequence | | | Note | Function |
|---|---|---|---|---|---|---|---|---|
| 7013 | MRPL39 | NM_017446.2 | 242 | tactgtcttcgtgatgaataaaa | 282245 | 658565 | - | |
| 7014 | MRPL39 | NM_017446.2 | 971 | atctcggaaaatgtaactgaag | 282270 | 658591 | see also 7013 line | |
| 7015 | MARK2 | NM_017490.1 | 1944 | cccccacggagagtaactgtga | 90260 | 507963 | kinase_related | |
| 7016 | HSGP25L2G | NM_017510.3 | 194 | accggacgcagctgtatgacaag | 127301 | 529333 | - | |
| 7017 | DNAH3 | NM_017539.1 | 9592 | cccttggctccaagatcaact | 79061 | 814679 | - | |
| 7018 | DNAH3 | NM_017539.1 | 10839 | cagctatccatcagagaagtttc | 79080 | 814711 | see also 7017 line | |
| 7019 | DNAH3 | NM_017539.1 | 11628 | aaggcaggagctcatcagattca | 79095 | 814722 | see also 7017 line | |
| 7020 | GALNT10 | NM_017540.3 | 896 | agggcaaccagctgtggaaatac | 358991 | 599750 | - | |
| 7021 | GALNT10 | NM_017540.3 | 897 | gggcaaccagctgtggaaatacc | 358992 | 599751 | see also 7020 line | |
| 7022 | POGK | NM_017542.3 | 1348 | aagttaccacgtacatcatttt | 35541 | 463814 | apoptosis | |
| 7023 | POGK | NM_017542.3 | 1352 | taccacgtacatcatttgagg | 35542 | 463815 | see also 7022 line | |
| 7024 | HAO1 | NM_017545.2 | 784 | gggatcttggtgtcgaatcatgg | 221002 | 608051 | - | glycolate oxidase |
| 7025 | C40 | NM_017546.3 | 1171 | ccctttagtcgctatagaaatgt | 277894 | 732130 | - | |
| 7026 | C40 | NM_017546.3 | 1275 | aagttgtaaatcgactaactaca | 277896 | 732134 | see also 7025 line | |
| 7027 | C40 | NM_017546.3 | 1424 | ttgatccgtaacaaaattattaa | 277905 | 732143 | see also 7025 line | |
| 7028 | H17 | NM_017547.2 | 1324 | cgctagtttgtcaacatgtacttt | 226932 | 613537 | - | |
| 7029 | H17 | NM_017547.2 | 1453 | tcctcttttaccgctttacttg | 226934 | 613541 | see also 7028 line | |
| 7030 | UCC1 | NM_017549.1 | 900 | ctgtccaggaaacctttaccata | 154236 | 745852 | - | calcium ion binding |
| 7031 | UCC1 | NM_017549.1 | 954 | ttgacatccagctgggtattaaa | 154239 | 745856 | see also 7030 line | |
| 7032 | DKFZp434L0850 | NM_017558.1 | 2884 | tagcctggatacccactattacc | 359066 | 781040 | - | |
| 7033 | IL17RD | NM_017563.1 | 639 | agcctgtgactgtgtttacagc | 246459 | 633756 | receptor acitivity | |
| 7034 | IL17RD | NM_017563.1 | 1327 | cacgagtccagttcatcattgt | 246476 | 633766 | see also 7033 line | |
| 7035 | NAGK | NM_017567.1 | 627 | ctgtatagggactttgataaatg | 93824 | 510032 | kinase_related | |
| 7036 | P38IP | NM_017569.2 | 971 | atgaaacatcgcccaatgaaacg | 359145 | 709899 | - | |
| 7037 | UBE2Q | NM_017582.5 | 772 | ggccatcctagagaaaattaaaa | 231266 | 620296 | - | |
| 7038 | UBE2Q | NM_017582.5 | 800 | cagaggcaagattacttaaatgg | 231267 | 620298 | see also 7037 line | |
| 7039 | UBE2Q | NM_017582.5 | 881 | taccgatcacagagtttcaaagg | 231269 | 620300 | see also 7037 line | |
| 7040 | UBE2Q | NM_017582.5 | 892 | gagtttcaaaggcggaaactatg | 231270 | 620301 | see also 7037 line | |
| 7041 | UBE2Q | NM_017582.5 | 913 | tgcagtcgaactcgttgaatgaca | 231271 | 620302 | see also 7037 line | |
| 7042 | UBE2Q | NM_017582.5 | 915 | cagtcgaactcgttgaatgacagt | 231272 | 620303 | see also 7037 line | |
| 7043 | UBE2Q | NM_017582.5 | 1281 | gagcacagcagtcctaccagtcc | 231279 | 620309 | see also 7037 line | |
| 7044 | TRIM44 | NM_017583.3 | 1254 | aacttgatacctctaatgaatca | 160593 | 710183 | - | |
| 7045 | BMP2K | NM_017593.3 | 398 | ttgaagcgaatgtatgtcaataa | 74022 | 496038 | kinase_related | Alzheimer disease |
| 7046 | DKFZp761A052 | NM_017602.2 | 1050 | atcggaatatccactataattca | 359399 | 751926 | - | |

Figure 46

| 7047 | DKFZp761 A052 | NM_017602.2 | 1069 | ttcagtggtgaatcctaacaagg | 359400 | 751928 | see also 7046 line | | |
|---|---|---|---|---|---|---|---|---|---|
| 7048 | DKFZp761 A052 | NM_017602.2 | 1713 | aggaatacctagacagtatgaag | 359404 | 751936 | see also 7046 line | | |
| 7049 | DKFZp434E 2220 | NM_017612.2 | 322 | gcgccgagaatcaagaacttaaa | 359529 | 480066 | - | - | - |
| 7050 | DKFZp434E 2220 | NM_017612.2 | 324 | gccgagaatcaagaacttaaacg | 359530 | 480068 | see also 7049 line | | |
| 7051 | DKFZp434E 2220 | NM_017612.2 | 634 | ttgtaggaagtgtcctgtatttt | 359545 | 480088 | see also 7049 line | | |
| 7052 | DKFZp434E 2220 | NM_017612.2 | 656 | tactaatttttgccttgataaat | 359546 | 480089 | see also 7049 line | | |
| 7053 | DKFZp434E 2220 | NM_017612.2 | 800 | aaggccaaagcctcactgtttca | 359550 | 480094 | see also 7049 line | | |
| 7054 | DONSON | NM_017613.2 | 525 | tacctgtggactggagtattaaa | 279475 | 712073 | - | - | - |
| 7055 | DONSON | NM_017613.2 | 526 | acctgtggactggagtattaaaa | 279476 | 712074 | see also 7054 line | | |
| 7056 | DONSON | NM_017613.2 | 528 | ctgtggactggagtattaaaacg | 279477 | 712075 | see also 7054 line | | |
| 7057 | DONSON | NM_017613.2 | 629 | cagcattgtagggcaacagaagt | 279480 | 712076 | see also 7054 line | | |
| 7058 | EIF2C4 | NM_017629.2 | 273 | ttcgactgttagccaatcatttt | 359736 | 768650 | - | - | - |
| 7059 | EIF2C4 | NM_017629.2 | 843 | agcctatcattgagttcatgtgt | 359750 | 768666 | see also 7058 line | | |
| 7060 | EIF2C4 | NM_017629.2 | 975 | gtggacagatgaaacgaaaatac | 359754 | 768667 | see also 7058 line | | |
| 7061 | EIF2C4 | NM_017629.2 | 2251 | agcaattcgaaaggcatgtatta | 359768 | 768689 | see also 7058 line | | |
| 7062 | FLJ20045 | NM_017638.1 | 576 | ttccgcatggaccatgagtttgt | 359867 | 720984 | - | - | - |
| 7063 | LRRC16 | NM_017640.2 | 801 | ttcatgccagcaccgaaaagatt | 359899 | 611600 | - | - | - |
| 7064 | LRRC16 | NM_017640.2 | 804 | atgccagcaccgaaaagatttct | 359900 | 611601 | see also 7063 line | | |
| 7065 | LRRC16 | NM_017640.2 | 828 | ttccacgtacctttgttaaaaat | 359904 | 611604 | see also 7063 line | | |
| 7066 | MBTD1 | NM_017643.1 | 171 | ggggacctgctggggtgatatct | 359936 | 749859 | transcription_regulator | - | - |
| 7067 | MBTD1 | NM_017643.1 | 849 | agctatagacccattaaatcttt | 359962 | 749884 | see also 7066 line | | |
| 7068 | MBTD1 | NM_017643.1 | 1106 | cagcaccagtaaaactatttaat | 359969 | 749895 | see also 7066 line | | |
| 7069 | MBTD1 | NM_017643.1 | 1323 | gtgtcagttaactggatatcaac | 359978 | 749907 | see also 7066 line | | |
| 7070 | DRE1 | NM_017644.2 | 831 | cactcaaaacactcttcacaaaa | 130842 | 531215 | - | - | - |
| 7071 | DRE1 | NM_017644.2 | 948 | gtgatgaacttgttattggtaaa | 130845 | 531220 | see also 7070 line | | |
| 7072 | DRE1 | NM_017644.2 | 1416 | gccgtgatgtctggatttataac | 130860 | 531231 | see also 7070 line | | |
| 7073 | DRE1 | NM_017644.2 | 1479 | aaggcagatggcgtcacaaaatg | 130864 | 531232 | see also 7070 line | | |
| 7074 | DRE1 | NM_017644.2 | 1480 | aggcagatggcgtcacaaaatgg | 130865 | 531233 | see also 7070 line | | |
| 7075 | IPT | NM_017646.3 | 374 | aggaaccaattattacattgaat | 85841 | 719784 | - | - | - |
| 7076 | IPT | NM_017646.3 | 718 | ttctagatgagcgcttggataag | 85845 | 719789 | see also 7075 line | | |

Figure 46

| | | | | | | | | | |
|---|---|---|---|---|---|---|---|---|---|
| 7077 | FTSJ3 | NM_017647.2 | 172 | agctcaatcgccgctttcagttc | 360066 | 715728 | - | - | - |
| 7078 | AHI1 | NM_017651.3 | 2229 | gtgtggccacctcaatatcattt | 360187 | 722537 | - | - | - |
| 7079 | AHI1 | NM_017651.3 | 2231 | gtggccacctcaatatcatttat | 360188 | 722538 | see also 7078 line | | |
| 7080 | AHI1 | NM_017651.3 | 2232 | tggccacctcaatatcatttatg | 360189 | 722539 | see also 7078 line | | |
| 7081 | AHI1 | NM_017651.3 | 3474 | tcgatcagatgaactaaccatcc | 360241 | 722587 | see also 7078 line | | |
| 7082 | FLJ20071 | NM_017653.2 | 2110 | tggtgatctccttctttagctca | 360308 | 730753 | - | - | - |
| 7083 | FLJ20080 | NM_017657.2 | 316 | tggagccagacatcattcgaatg | 145361 | 805795 | - | - | - |
| 7084 | FLJ20080 | NM_017657.2 | 2738 | gagtagcagtggccttactaacc | 145416 | 805854 | see also 7083 line | | |
| 7085 | BTBD5 | NM_017658.1 | 123 | tccgagtaggtgatgttaaaatt | 129404 | 531108 | - | - | - |
| 7086 | FLJ20084 | NM_017659.1 | 954 | tccccaggaagtgatgtacttcc | 360457 | 721510 | - | - | - |
| 7087 | TRPM6 | NM_017662.3 | 2793 | tcctcatgctgttcacttacacc | 103364 | 516833 | kinase_related、recept or_acitivity、channel | - | - |
| 7088 | TRPM6 | NM_017662.3 | 3101 | gacttctttgctgtgaatcaaca | 103372 | 516841 | see also 7087 line | | |
| 7089 | TRPM6 | NM_017662.3 | 3498 | accgctatcgctacatcatgacc | 103386 | 516851 | see also 7087 line | | |
| 7090 | C20orf42 | NM_017671.2 | 2066 | taccttgtcagatttaaaggaag | 322657 | 687056 | - | - | - |
| 7091 | TRPM7 | NM_017672.2 | 1453 | atctgcatttgaccagcttatcc | 103489 | 516963 | channel、kinase_relate d | - | - |
| 7092 | TRPM7 | NM_017672.2 | 1621 | tggagtaagcatgcataaattcc | 103496 | 516972 | see also 7091 line | | |
| 7093 | TRPM7 | NM_017672.2 | 1907 | cagttgcgaaagagtcatgaatc | 103514 | 516986 | see also 7091 line | | |
| 7094 | TRPM7 | NM_017672.2 | 2175 | aagaatcaatggctaaagcatta | 103527 | 516996 | see also 7091 line | | |
| 7095 | TRPM7 | NM_017672.2 | 2179 | atcaatggctaaagcattagttg | 103528 | 516997 | see also 7091 line | | |
| 7096 | TRPM7 | NM_017672.2 | 2777 | gccttttatcatgcaccaattgt | 103559 | 517026 | see also 7091 line | | |
| 7097 | TRPM7 | NM_017672.2 | 2870 | atggaacagttaccttcagttca | 103563 | 517029 | see also 7091 line | | |
| 7098 | TRPM7 | NM_017672.2 | 2886 | cagttcaagaatggattgttatt | 103564 | 517032 | see also 7091 line | | |
| 7099 | TRPM7 | NM_017672.2 | 2978 | cagaagattaaagtatggtttag | 103566 | 517035 | see also 7091 line | | |
| 7100 | TRPM7 | NM_017672.2 | 3328 | gactcttgctaaagatatagttt | 103578 | 517053 | see also 7091 line | | |
| 7101 | TRPM7 | NM_017672.2 | 3879 | ttggagatcgtgtcaactacata | 103594 | 517071 | see also 7091 line | | |
| 7102 | TRPM7 | NM_017672.2 | 3882 | gagatcgtgtcaactacataaaa | 103596 | 517072 | see also 7091 line | | |
| 7103 | TRPM7 | NM_017672.2 | 5312 | cccaaatccataccatattctcc | 103637 | 517123 | see also 7091 line | | |
| 7104 | C1orf26 | NM_017673.4 | 2210 | cagtacaaggtcaaattatgatg | 360542 | 719334 | - | - | - |
| 7105 | C1orf26 | NM_017673.4 | 2242 | cacagacctttgctcaagtaaac | 360543 | 719336 | see also 7104 line | | |
| 7106 | C1orf26 | NM_017673.4 | 2647 | ccctctataacttcctaatcaag | 360561 | 719349 | see also 7104 line | | |
| 7107 | FLJ20125 | NM_017676.1 | 559 | atgctataatcatgacagatttg | 27031 | 453654 | - | - | - |
| 7108 | C11orf33 | NM_017678.1 | 766 | tccgccagtggatggaatacttc | 360574 | 781444 | - | - | - |
| 7109 | BCAS3 | NM_017679.2 | 543 | ttgtgtggatctgtattcacttc | 310404 | 689820 | - | - | - |
| 7110 | BCAS3 | NM_017679.2 | 754 | gggagccgctggcttgcttatgc | 310414 | 689826 | see also 7109 line | | |
| 7111 | BCAS3 | NM_017679.2 | 1813 | cagtccaaacaagttgtagttga | 310432 | 689850 | see also 7109 line | | |

Figure 46

| | | | | | | | | | |
|---|---|---|---|---|---|---|---|---|---|
| 7112 | BCAS3 | NM_017679.2 | 1815 | gtccaaacaagttgtagttgagt | 310433 | 689851 | see also 7109 line | | |
| 7113 | FLJ20156 | NM_017691.1 | 584 | caccttcgtttgttgaactttca | 360850 | 757827 | - | - | - |
| 7114 | BIVM | NM_017693.2 | 1486 | gtggtttagacaaattaatgacc | 360936 | 753649 | - | - | - |
| 7115 | BIVM | NM_017693.2 | 1525 | atgctcttatgttctatataagc | 360941 | 753651 | see also 7114 line | | |
| 7116 | FLJ20160 | NM_017694.2 | 222 | ctggatagagaaacattgtgtta | 360964 | 800630 | - | - | - |
| 7117 | FLJ20160 | NM_017694.2 | 1419 | ttggttcatggggttttggatatg | 360988 | 748523 | see also 7116 line | | |
| 7118 | FLJ20160 | NM_017694.2 | 1822 | atcggaggcgtgttagtcaatta | 361003 | 748536 | see also 7116 line | | |
| 7119 | FLJ20160 | NM_017694.2 | 1823 | tcggaggcgtgttagtcaattat | 361004 | 748537 | see also 7116 line | | |
| 7120 | FLJ20160 | NM_017694.2 | 1824 | cggaggcgtgttagtcaattatt | 361005 | 748538 | see also 7116 line | | |
| 7121 | FLJ20160 | NM_017694.2 | 1826 | gaggcgtgttagtcaattatttt | 361006 | 748539 | see also 7116 line | | |
| 7122 | OBDPF | NM_017711.2 | 347 | aaccagcaagtgcgactgtatct | 198959 | 590948 | - | - | - |
| 7123 | C20orf13 | NM_017714.1 | 359 | aacttgaggattctccttttaca | 203322 | 593936 | - | - | - |
| 7124 | FLJ20259 | NM_017730.2 | 1700 | cagaccaagaatgctgaactaga | 361191 | 785766 | - | - | - |
| 7125 | FLJ20272 | NM_017735.2 | 671 | agcacgcattatcctagtgaatg | 361324 | 767456 | - | - | - |
| 7126 | FLJ20275 | NM_017737.1 | 104 | ctgactcagaacgcaaagttatt | 361414 | 768126 | - | - | - |
| 7127 | FLJ20275 | NM_017737.1 | 693 | cagtgacataaatcatcttgtaa | 361425 | 768139 | see also 7126 line | | |
| 7128 | FLJ20277 | NM_017739.1 | 213 | ctggaagtataaactgacaaacc | 210426 | 599378 | - | - | - |
| 7129 | FLJ20277 | NM_017739.1 | 769 | tgggccttcgtgggacgaaaagg | 210430 | 599386 | see also 7128 line | | |
| 7130 | FLJ20277 | NM_017739.1 | 772 | gccttcgtgggacgaaaaggagg | 210431 | 599387 | see also 7128 line | | |
| 7131 | ST7L | NM_017744.4 | 598 | ttgaatggtggtacttccataag | 361452 | 767905 | - | - | - |
| 7132 | ST7L | NM_017744.4 | 602 | atggtggtacttccataagcatg | 361453 | 767906 | see also 7131 line | | |
| 7133 | ST7L | NM_017744.4 | 910 | tacgtccttcagacacagttatg | 361458 | 767916 | see also 7131 line | | |
| 7134 | ST7L | NM_017744.4 | 1644 | cagcactggaaacgaatagaagg | 361474 | 767938 | see also 7131 line | | |
| 7135 | BCOR | NM_017745.4 | 868 | tcctcgactcgccaaataagtat | 361493 | 739394 | - | - | - |
| 7136 | BCOR | NM_017745.4 | 870 | ctcgactcgccaaataagtattc | 361494 | 739395 | see also 7135 line | | |
| 7137 | BCOR | NM_017745.4 | 875 | ctcgccaaataagtattcactga | 361497 | 739396 | see also 7135 line | | |
| 7138 | BCOR | NM_017745.4 | 1692 | gagattccgaaagaaacactatc | 361508 | 739409 | see also 7135 line | | |
| 7139 | FLJ20287 | NM_017746.1 | 855 | tccagtaggttgagagaaagtga | 361599 | 772466 | - | - | - |
| 7140 | FLJ20300 | NM_017753.1 | 394 | tgcatatccaaggattcttctgt | 361683 | 803942 | - | - | - |
| 7141 | FLJ20300 | NM_017753.1 | 411 | ttctgtcaggacggagacttaat | 361684 | 803943 | see also 7140 line | | |
| 7142 | FLJ20300 | NM_017753.1 | 413 | ctgtcaggacggagacttaatga | 361685 | 803944 | see also 7140 line | | |
| 7143 | FLJ20300 | NM_017753.1 | 415 | gtcaggacggagacttaatgaag | 361686 | 803945 | see also 7140 line | | |
| 7144 | FLJ20303 | NM_017755.4 | 633 | aagaccacacagttaattgaaat | 361790 | 755686 | - | - | - |
| 7145 | FLJ20303 | NM_017755.4 | 841 | tcctcttctatgatcgaattta | 361795 | 755694 | see also 7144 line | | |
| 7146 | FLJ20303 | NM_017755.4 | 843 | ctcttctatgatcgaattttatg | 361796 | 755695 | see also 7144 line | | |
| 7147 | FLJ20311 | NM_017760.4 | 3156 | gcctgtggttgagataagtcacc | 362013 | 747983 | - | - | - |
| 7148 | FLJ20320 | NM_017765.1 | 565 | cagacctacacggctgtgtatta | 362081 | 755895 | - | - | - |

Figure 46

| | | | | | | | | |
|---|---|---|---|---|---|---|---|---|
| 7149 | FLJ20331 | NM_017768.3 | 792 | tgctttattgcggaggaataaa | 196894 | 589223 | - | |
| 7150 | FLJ20331 | NM_017768.3 | 1539 | gactgcaaacgatcaatcttcc | 196923 | 589249 | see also 7149 line | |
| 7151 | PXK | NM_017771.2 | 1487 | ctgaaagaagtcaaaacgatct | 98633 | 499193 | kinase_related | |
| 7152 | OSBPL10 | NM_017784.3 | 2079 | gtccgtggggtctctatgatag | 303410 | 767177 | - | |
| 7153 | OSBPL10 | NM_017784.3 | 2080 | tccgtggggtctctatgatagg | 303411 | 767178 | see also 7152 line | |
| 7154 | C14orf58 | NM_017791.1 | 639 | cggctccatcaataacatcttca | 362426 | 756523 | - | |
| 7155 | C20orf21 | NM_017798.2 | 1819 | caggaggtgccettagaaaaagc | 362604 | 784370 | - | |
| 7156 | UBE2R2 | NM_017811.2 | 789 | gacaacagctcagattgcttta | 231306 | 616684 | - | |
| 7157 | SARS2 | NM_017827.2 | 829 | gtctggccacggtcctattacc | 100177 | 514435 | - | |
| 7158 | OCIA | NM_017830.1 | 168 | atgaatggagggctgatttcg | 362840 | 713507 | - | |
| 7159 | OCIA | NM_017830.1 | 333 | ttgattacttcaagagattaattag | 362850 | 713513 | see also 7158 line | |
| 7160 | FLJ20487 | NM_017841.1 | 363 | atgactgggatatttactactgg | 363009 | 715894 | - | |
| 7161 | C1orf27 | NM_017847.2 | 470 | aacgagtgacacttcacattgt | 363039 | 756783 | - | |
| 7162 | FLJ20516 | NM_017858.1 | 303 | aggtcatgaggctgaagacttga | 363173 | 716066 | - | |
| 7163 | FLJ20516 | NM_017858.1 | 433 | cagacctgtttaaaacgaattcg | 363176 | 716074 | see also 7162 line | |
| 7164 | FLJ20530 | NM_017864.1 | 1041 | atgttcgggaggacatrtgaat | 363266 | 800745 | - | |
| 7165 | FLJ20530 | NM_017864.1 | 1161 | ttcgatatacactcagtataat | 363269 | 800753 | see also 7164 line | |
| 7166 | FLJ20530 | NM_017864.1 | 1406 | ctcagagaaattgactacacaaac | 363279 | 800756 | see also 7164 line | |
| 7167 | FLJ20531 | NM_017865.2 | 339 | ttggaccggtacacttcttcagg | 363287 | 807703 | - | |
| 7168 | FLJ20558 | NM_017880.1 | 1446 | atcaaaccatgcactatcagttt | 188402 | 582242 | - | |
| 7169 | FLJ20558 | NM_017880.1 | 1451 | accatgcactatcagtttgatgg | 188403 | 582244 | see also 7168 line | |
| 7170 | PINX1 | NM_017884.3 | 238 | aagatgggtggtctaaaggaaa | 64934 | 478914 | cell_cycle | nucleic acid binding |
| 7171 | C6orf96 | NM_017909.1 | 1311 | ccgaagagttaaggtcatgaatg | 363876 | 715976 | - | |
| 7172 | C14orf10 | NM_017917.2 | 819 | ttccatcatgcagttcttaatt | 138236 | 543098 | - | |
| 7173 | C14orf10 | NM_017917.2 | 1145 | gtcaagaaacaaattggtttct | 138243 | 543106 | see also 7172 line | |
| 7174 | STX17 | NM_017919.1 | 604 | aaccttagaaagcggacttaattg | 305781 | 724222 | - | |
| 7175 | STX17 | NM_017919.1 | 605 | accttagaaagcggacttaattga | 305782 | 724223 | see also 7174 line | |
| 7176 | STX17 | NM_017919.1 | 688 | cagcattgcagaccatgtcaaca | 305783 | 724224 | see also 7174 line | |
| 7177 | PRPF39 | NM_017922.2 | 2446 | cagaatccttggaattatggaca | 302048 | 799341 | - | |
| 7178 | C14orf119 | NM_017924.2 | 368 | aggagcgcaatgaatttgtcaga | 364062 | 711572 | - | |
| 7179 | FLJ20716 | NM_017938.2 | 393 | tggcttcatcgtgtttatcagc | 364357 | 781541 | - | |
| 7180 | USP47 | NM_017944.2 | 995 | gtgaacgttgtaagaagaagtgt | 190068 | 794391 | - | |
| 7181 | CADPS2 | NM_017954.7 | 1012 | gtccggaatttaaattacaaaaa | 364780 | 768324 | - | |
| 7182 | CADPS2 | NM_017954.7 | 1744 | atgatgaacaggacagaatatta | 364812 | 768345 | see also 7181 line | |
| 7183 | CADPS2 | NM_017954.7 | 2452 | aagctgccttgatcaattacact | 364824 | 768358 | see also 7181 line | |
| 7184 | CADPS2 | NM_017954.7 | 2455 | ctgccttgatcaattacactaga | 364825 | 768359 | see also 7181 line | |
| 7185 | C14orf102 | NM_017970.2 | 3042 | atgtacagattcagaataagtcc | 321311 | 808617 | - | |

Figure 46

| 7186 | APG16L | NM_017974.2 | 1425 | tacgcagcaaagtctgcataaag | 364950 | 532060 | - | - | - |
|------|--------|-------------|------|-------------------------|--------|--------|---|---|---|
| 7187 | APG16L | NM_017974.2 | 1495 | atgtgtaatgagtggacattttg | 364955 | 532063 | see also 7186 line | | |
| 7188 | FLJ10036 | NM_017975.2 | 585 | caccatggctcacaatcctaata | 364968 | 725630 | - | - | - |
| 7189 | RUFY2 | NM_017987.2 | 60 | atggctaaactgagtatcaaagg | 159956 | 559603 | - | - | - |
| 7190 | PDPR | NM_017990.3 | 3094 | ctccgaattgagaagtttttgc | 214810 | 812901 | - | - | - |
| 7191 | FLJ10094 | NM_017993.1 | 522 | gactggaatcacccccattaacc | 60847 | 482372 | - | - | - |
| 7192 | FLJ10094 | NM_017993.1 | 928 | caggccagggatgacttctatga | 60861 | 482382 | see also 7191 line | | |
| 7193 | FLJ10094 | NM_017993.1 | 1330 | aacgcttctaccagacaaaaagc | 60865 | 482389 | see also 7191 line | | |
| 7194 | FLJ10094 | NM_017993.1 | 1375 | cagcgcaagaacatagacatttg | 60866 | 482391 | see also 7191 line | | |
| 7195 | FEZL | NM_018008.2 | 783 | cggcctggctgcggacaagttcc | 365229 | 746473 | - | - | - |
| 7196 | ESRRBL1 | NM_018010.2 | 824 | aactgaaagtcacgattaggact | 365267 | 687750 | receptor_acitivity | - | - |
| 7197 | ESRRBL1 | NM_018010.2 | 978 | cagcagccgagaaaagtacatca | 365271 | 687756 | see also 7196 line | | |
| 7198 | FLJ10154 | NM_018011.1 | 479 | gcccgaccgcatcgacatcttcg | 365281 | 790880 | - | - | - |
| 7199 | BCL11A | NM_018014.2 | 245 | aaggcaaacccagcacttaagc | 365293 | 525893 | - | - | - |
| 7200 | BCL11A | NM_018014.2 | 444 | tggcagcctctgcttagaaaaag | 365301 | 525895 | see also 7199 line | | |
| 7201 | BCL11A | NM_018014.2 | 548 | cagaggatgacgattgtttatca | 365305 | 525898 | see also 7199 line | | |
| 7202 | BCL11A | NM_018014.2 | 702 | cccgcagggtatttgtaaagatg | 365308 | 525900 | see also 7199 line | | |
| 7203 | BCL11A | NM_018014.2 | 736 | tacacatgtacaacttgcaaaca | 365311 | 525902 | see also 7199 line | | |
| 7204 | BCL11A | NM_018014.2 | 907 | ctccatgggattcatattgcaga | 365316 | 525905 | see also 7199 line | | |
| 7205 | BCL11A | NM_018014.2 | 936 | cccctttaacctgctaagaatac | 365317 | 525907 | see also 7199 line | | |
| 7206 | BCL11A | NM_018014.2 | 1154 | agcctcccgccatggatttctct | 365319 | 525912 | see also 7199 line | | |
| 7207 | BCL11A | NM_018014.2 | 1157 | ctcccgccatggatttctctagg | 365320 | 525913 | see also 7199 line | | |
| 7208 | BCL11A | NM_018014.2 | 1343 | ccccggtcaagtccaagtcatgc | 365322 | 525916 | see also 7199 line | | |
| 7209 | BCL11A | NM_018014.2 | 2384 | gagggagcacgccccatattagt | 365324 | 525922 | see also 7199 line | | |
| 7210 | BCL11A | NM_018014.2 | 2386 | gggagcacgccccatattagtgg | 365325 | 525923 | see also 7199 line | | |
| 7211 | FLJ10178 | NM_018015.2 | 164 | ctgcgatactatggaaaacctga | 365332 | 788141 | - | - | - |
| 7212 | SLC38A4 | NM_018018.2 | 1351 | ctcccggacggcctatgcaattc | 259747 | 642004 | - | - | - |
| 7213 | FLJ10210 | NM_018027.2 | 1091 | tccagtatgactaccatgataaa | 322883 | 774226 | - | - | - |
| 7214 | LUC7L | NM_018032.2 | 234 | tggctgggacgcgcatggattta | 365499 | 719873 | - | - | - |
| 7215 | FLJ10244 | NM_018037.1 | 737 | tgggcgttattaagtcgaaaaga | 295788 | 664416 | signal_transduction | - | - |
| 7216 | FLJ10244 | NM_018037.1 | 739 | ggcgttattaagtcgaaaagaca | 295789 | 664417 | see also 7215 line | | |
| 7217 | FLJ10305 | NM_018052.3 | 867 | gtcggtgccagacattaacctgc | 365923 | 761297 | - | - | - |
| 7218 | RICH1 | NM_018054.3 | 1116 | gtgtgcaggatcaagacaaaaaa | 99039 | 753280 | - | - | - |
| 7219 | RICH1 | NM_018054.3 | 1173 | caccacaaaattttgttaacttt | 99042 | 753285 | see also 7218 line | | |
| 7220 | NTT73 | NM_018057.2 | 1027 | aagttctgccaccacctattact | 255863 | 640046 | - | - | - |
| 7221 | FLJ10326 | NM_018060.2 | 241 | tagccaatcgattccatatgatg | 82478 | 814266 | - | - | - |
| 7222 | FLJ10326 | NM_018060.2 | 242 | agccaatcgattccatatgatga | 82479 | 814267 | see also 7221 line | | |

Figure 46

| 7223 | FLJ10326 | NM_018060.2 | 536 | gggcttggtttatcgatcttaca | 82486 | 814276 | see also 7221 line | | |
|------|----------|-------------|-----|-------------------------|-------|--------|-------------------|---|---|
| 7224 | FLJ10326 | NM_018060.2 | 540 | ttggtttatcgatcttacaaacc | 82487 | 814277 | see also 7221 line | | |
| 7225 | FLJ10326 | NM_018060.2 | 1476 | agccaaaccactgagcatattgt | 82518 | 814311 | see also 7221 line | | |
| 7226 | FLJ10330 | NM_018061.1 | 273 | tcctgtcgtcgccttacttcaaa | 365944 | 719588 | - | - | - |
| 7227 | FLJ10330 | NM_018061.1 | 275 | ctgtcgtcgccttacttcaaagt | 365945 | 719589 | see also 7226 line | | |
| 7228 | FLJ10330 | NM_018061.1 | 277 | gtcgtcgccttacttcaaagtac | 365946 | 719590 | see also 7226 line | | |
| 7229 | FLJ10330 | NM_018061.1 | 330 | tggtggacgagatctactttaag | 365948 | 719592 | see also 7226 line | | |
| 7230 | FLJ10330 | NM_018061.1 | 488 | ctgaagttaactcgaaagcaagt | 365949 | 719594 | see also 7226 line | | |
| 7231 | HELLS | NM_018063.3 | 1248 | gactggtactcccttgcaaaaca | 28096 | 697038 | - | - | - |
| 7232 | FLJ10375 | NM_018075.2 | 1055 | ctgcctttgctgtgttaaataac | 366212 | 749497 | - | - | - |
| 7233 | FLJ10375 | NM_018075.2 | 1056 | tgcctttgctgtgttaaataact | 366213 | 749498 | see also 7232 line | | |
| 7234 | FLJ10377 | NM_018077.1 | 2137 | ctcacaccgaggccccaaaatca | 60996 | 479318 | - | - | - |
| 7235 | FLJ10378 | NM_018078.2 | 362 | gtgggtaccactccacttagatg | 366309 | 758964 | - | - | - |
| 7236 | FLJ10378 | NM_018078.2 | 363 | tgggtaccactccacttagatgt | 366310 | 758965 | see also 7235 line | | |
| 7237 | FIGN | NM_018086.1 | 337 | gggctcccatctcaggaatatgc | 82305 | 502376 | - | - | - |
| 7238 | FIGN | NM_018086.1 | 1529 | gtcgccagccctcggtgattttt | 82331 | 502404 | see also 7237 line | | |
| 7239 | FIGN | NM_018086.1 | 1601 | gtccagtcagtcggatgagaacc | 82334 | 502408 | see also 7237 line | | |
| 7240 | FIGN | NM_018086.1 | 1653 | aacttcggctgaggaccaaatcg | 82338 | 502409 | see also 7237 line | | |
| 7241 | FIGN | NM_018086.1 | 1657 | tcggctgaggaccaaatcgtagt | 82340 | 502410 | see also 7237 line | | |
| 7242 | FIGN | NM_018086.1 | 1767 | cacagcgaggcaccagataatag | 82348 | 502414 | see also 7237 line | | |
| 7243 | FIGN | NM_018086.1 | 1770 | agcgaggcaccagataatagtac | 82349 | 502415 | see also 7237 line | | |
| 7244 | FIGN | NM_018086.1 | 1771 | gcgaggcaccagataatagtaca | 82350 | 502416 | see also 7237 line | | |
| 7245 | FIGN | NM_018086.1 | 1773 | gaggcaccagataatagtacaac | 82351 | 502417 | see also 7237 line | | |
| 7246 | FIGN | NM_018086.1 | 1965 | gaggcccgttacatatcaagact | 82359 | 502422 | see also 7237 line | | |
| 7247 | FIGN | NM_018086.1 | 1968 | gcccgttacatatcaagactttg | 82360 | 502423 | see also 7237 line | | |
| 7248 | FIGN | NM_018086.1 | 1969 | cccgttacatatcaagactttga | 82361 | 502424 | see also 7237 line | | |
| 7249 | FLJ10407 | NM_018087.3 | 1099 | ctcatggatactcttcaaaatct | 366526 | 734260 | - | - | - |
| 7250 | FLJ10407 | NM_018087.3 | 1308 | gtggacatccccacaattggaca | 366534 | 734272 | see also 7249 line | | |
| 7251 | FLJ10407 | NM_018087.3 | 1840 | gtcaaaacgggtgctgataatgt | 366546 | 734285 | see also 7249 line | | |
| 7252 | ELP3 | NM_018091.3 | 46 | ctgatgatgctgactataggaga | 215083 | 602379 | - | - | - |
| 7253 | FLJ10439 | NM_018093.1 | 1079 | tggagcataaggtttatctcaag | 366637 | 751055 | - | - | - |
| 7254 | ECT2 | NM_018098.4 | 541 | gggtggagttattcgaaaagact | 295691 | 664559 | kinase_related | guanyl-nucleotide release factor | - |
| 7255 | ECT2 | NM_018098.4 | 650 | aagccagaatggatttataaagc | 295697 | 664566 | see also 7254 line | | |
| 7256 | C14orf130 | NM_018108.2 | 1113 | acccttagcagcatgaatagagt | 227658 | 615174 | - | - | - |
| 7257 | FLJ10486 | NM_018109.2 | 1048 | aagttccgaactcctttatatat | 61024 | 478060 | - | - | - |
| 7258 | FLJ10486 | NM_018109.2 | 1051 | ttccgaactcctttatatatatg | 61025 | 478061 | see also 7257 line | | |

Figure 46

| 7259 | LIMR | NM_018113.1 | 1593 | gtgactttggacgcttcaactgg | 249751 | 737999 | - | - | - |
|---|---|---|---|---|---|---|---|---|---|
| 7260 | WDR11 | NM_018117.10 | 322 | ctggggctggcaaggtttaattg | 183605 | 577433 | - | - | - |
| 7261 | WDR11 | NM_018117.10 | 2556 | gagctgaagtgtgggatactaaa | 183676 | 577498 | see also 7260 line | | |
| 7262 | WDR11 | NM_018117.10 | 2557 | agctgaagtgtgggatactaaag | 183677 | 577499 | see also 7260 line | | |
| 7263 | WDR11 | NM_018117.10 | 3664 | gacgcttcacagcatgagatact | 183703 | 577526 | see also 7260 line | | |
| 7264 | RPC5 | NM_018119.2 | 2243 | atggtggcatgtggtaccttaaa | 208771 | 598535 | transcription_regulator | - | - |
| 7265 | C10orf6 | NM_018121.2 | 3317 | atctatacatccagaaggttacc | 366923 | 776804 | - | - | - |
| 7266 | FLJ10514 | NM_018122.3 | 999 | atgcgggaatatctctgtaatct | 82596 | 480171 | - | - | - |
| 7267 | FLJ10514 | NM_018122.3 | 1984 | aacactatgacttggttttaaat | 82626 | 480195 | see also 7266 line | | |
| 7268 | FLJ10521 | NM_018125.2 | 807 | caggactaagagagacatcttgg | 366973 | 773325 | - | - | - |
| 7269 | FLJ10525 | NM_018126.1 | 377 | tgcaattcatgatgaccaataaa | 280075 | 666649 | - | molecular_function unknown | - |
| 7270 | PNPO | NM_018129.1 | 420 | tggcttccgcttcttcactaact | 225920 | 612553 | - | - | - |
| 7271 | ASPM | NM_018136.2 | 4497 | gtgctttcaagcccaaaagttat | 367337 | 700035 | - | - | microcephaly |
| 7272 | ASPM | NM_018136.2 | 6140 | cagtcactatacagtctaaatac | 367402 | 700087 | see also 7271 line | | |
| 7273 | FLJ10569 | NM_018142.1 | 578 | gtgatgtccttcctctaataatt | 367609 | 730170 | - | - | - |
| 7274 | FLJ10569 | NM_018142.1 | 752 | aacgtagctctcagaagtacata | 367618 | 730178 | see also 7273 line | | |
| 7275 | FLJ10569 | NM_018142.1 | 753 | acgtagctctcagaagtacataa | 367619 | 730179 | see also 7273 line | | |
| 7276 | FLJ10569 | NM_018142.1 | 756 | tagctctcagaagtacataatag | 367620 | 730180 | see also 7273 line | | |
| 7277 | FLJ10572 | NM_018143.1 | 1254 | gccagggtttgctaaaactgtag | 131267 | 532599 | - | - | - |
| 7278 | FLJ10572 | NM_018143.1 | 1625 | tgccgcttattgacaattactgc | 131278 | 532612 | see also 7277 line | | |
| 7279 | FLJ10572 | NM_018143.1 | 1808 | gagcagccatttgctattacaaa | 131281 | 532616 | see also 7277 line | | |
| 7280 | SEC61A2 | NM_018144.2 | 1007 | atgctgtctgttcgatttagtgg | 255619 | 639801 | - | - | - |
| 7281 | FLJ10581 | NM_018146.2 | 595 | cagcctcattaaggtgaaatttg | 51626 | 472799 | - | - | - |
| 7282 | FLJ10581 | NM_018146.2 | 596 | agcctcattaaggtgaaatttga | 51627 | 472800 | see also 7281 line | | |
| 7283 | FLJ10581 | NM_018146.2 | 642 | tcgtaacgccacaaggaataatg | 51629 | 472801 | see also 7281 line | | |
| 7284 | FLJ10587 | NM_018149.2 | 923 | ccctggaggaccagatctataga | 248631 | 620846 | - | - | - |
| 7285 | FLJ10587 | NM_018149.2 | 1220 | caggccagctagtggatttcact | 248643 | 620849 | see also 7284 line | | |
| 7286 | FLJ10587 | NM_018149.2 | 2600 | ctgggcctgacaaagtaatgaaa | 248688 | 620894 | see also 7284 line | | |
| 7287 | FLJ10587 | NM_018149.2 | 2601 | tgggcctgacaaagtaatgaaag | 248689 | 620895 | see also 7284 line | | |
| 7288 | hSyn | NM_018157.1 | 232 | tggaagtactccgcattctctcc | 367780 | 808719 | - | - | - |
| 7289 | hSyn | NM_018157.1 | 1104 | agctatagagagggtctaactcc | 367811 | 808746 | see also 7288 line | | |
| 7290 | hSyn | NM_018157.1 | 1499 | tggtcatttagaggaaccaatgc | 367820 | 808762 | see also 7288 line | | |

Figure 46

| 7291 | FLJ10637 | NM_018164.1 | 1432 | tacagactaccggattacagatt | 367854 | 752723 | - | | - | | - |
|------|----------|-------------|------|-------------------------|--------|--------|---|---|---|---|---|
| 7292 | FLJ10637 | NM_018164.1 | 1434 | cagactaccggattacagatttt | 367855 | 752724 | see also 7291 line | | | | |
| 7293 | FLJ10637 | NM_018164.1 | 1436 | gactaccggattacagattttgg | 367856 | 752725 | see also 7291 line | | | | |
| 7294 | FLJ10637 | NM_018164.1 | 1496 | gaccccagatataaaatcgatgg | 367858 | 752729 | see also 7291 line | | | | |
| 7295 | FLJ10637 | NM_018164.1 | 1613 | atgcaagcggtagttccattagc | 367863 | 752741 | see also 7291 line | | | | |
| 7296 | FLJ10637 | NM_018164.1 | 1697 | tacaacttagttgatatggaaag | 367866 | 752748 | see also 7291 line | | | | |
| 7297 | PB1 | NM_018165.2 | 3633 | ctcctgcaggccaactgaaatac | 18871 | 453313 | transcription_regulator | - | | - | |
| 7298 | PB1 | NM_018165.2 | 3733 | aggttttcactctctgctaaagt | 18874 | 453317 | see also 7297 line | | | | |
| 7299 | LGI2 | NM_018176.2 | 340 | ctgttcattgaagggaacaaaat | 367999 | 755231 | - | | - | | - |
| 7300 | LGI2 | NM_018176.2 | 1551 | caccgacaggagagatttctttt | 368028 | 755251 | see also 7299 line | | | | |
| 7301 | LGI2 | NM_018176.2 | 1552 | accgacaggagagatttctttt | 368029 | 755252 | see also 7299 line | | | | |
| 7302 | GPP34R | NM_018178.3 | 428 | gccatgcggggtcgaatctatct | 368041 | 760545 | - | | - | | - |
| 7303 | GPP34R | NM_018178.3 | 511 | cagcccaacaggtgatgttttac | 368046 | 760547 | see also 7302 line | | | | |
| 7304 | ATF7IP | NM_018179.3 | 2086 | aggccaagatagccaggttaacc | 325255 | 525846 | - | | - | | - |
| 7305 | ATF7IP | NM_018179.3 | 2631 | gtgagtggtcttaccaaaaatcc | 325269 | 525858 | see also 7304 line | | | | |
| 7306 | ATF7IP | NM_018179.3 | 3461 | aacaaccacatatgttgtaaaca | 325288 | 525890 | see also 7304 line | | | | |
| 7307 | FLJ10700 | NM_018182.1 | 327 | tgctaagaaggtcgcaaacaacc | 368139 | 756431 | - | | - | | - |
| 7308 | FLJ10700 | NM_018182.1 | 921 | ctcaactatccccctttcaatgg | 368142 | 756436 | see also 7307 line | | | | |
| 7309 | BBS7 | NM_018190.2 | 923 | tgggtgatggggttaaagattta | 368262 | 730921 | - | | - | | - |
| 7310 | BBS7 | NM_018190.2 | 2030 | tggctaagaaagtgcagttaatt | 368298 | 730963 | see also 7309 line | | | | |
| 7311 | MLAT4 | NM_018192.2 | 2086 | gtgactgcctctataaaaccaaa | 222514 | 609776 | - | | - | | - |
| 7312 | TMLHE | NM_018196.1 | 1030 | tgccattgaagcatgaatatatt | 224603 | 611018 | - | | - | | - |
| 7313 | ZFP64 | NM_018197.2 | 730 | tgccaagtcacgcaccaaaaagc | 43962 | 699638 | transcription_regulator | - | | - | |
| 7314 | C14orf114 | NM_018199.1 | 744 | tgggctattctgccagaaaatca | 58317 | 481896 | - | | - | | - |
| 7315 | HMG20A | NM_018200.2 | 514 | tcccccttacaggatatgttcg | 4952 | 460250 | transcription_regulator | - | | - | |
| 7316 | HMG20A | NM_018200.2 | 672 | cagagataaggagcgttacatga | 4955 | 460253 | see also 7315 line | | | | |
| 7317 | HMG20A | NM_018200.2 | 674 | gagataaggagcgttacatgaag | 4956 | 460254 | see also 7315 line | | | | |
| 7318 | HMG20A | NM_018200.2 | 754 | gaccgtcagaaaggcaaatctca | 4957 | 460256 | see also 7315 line | | | | |
| 7319 | VPS35 | NM_018206.2 | 385 | cagttggagttgtatatgtcaag | 137058 | 653008 | - | | - | | - |
| 7320 | VPS35 | NM_018206.2 | 391 | gagttgtatatgtcaagtcattt | 137059 | 653009 | see also 7319 line | | | | |
| 7321 | ARFGAP1 | NM_018209.2 | 340 | agctggtgggaatgctaagttcc | 21503 | 454973 | - | | - | | Alzheimer disease |
| 7322 | ENAH | NM_018212.2 | 1505 | cagaagacaatcgcccttaact | 368495 | 528882 | - | | - | | - |
| 7323 | PANK4 | NM_018216.1 | 1860 | tgccttaattttcgcagataaca | 95680 | 782093 | kinase_related | - | | - | |
| 7324 | PANK4 | NM_018216.1 | 1872 | cgcagataacagtggaatagaca | 95681 | 782095 | see also 7323 line | | | | |

335

Figure 46

| | | | | | | | | |
|---|---|---|---|---|---|---|---|---|
| 7325 | PANK4 | NM_018216.1 | 1874 | cagataacagtggaatagacatc | 95682 | 782096 | see also 7323 line | |
| 7326 | PANK4 | NM_018216.1 | 1881 | cagtggaatagacatcatttttg | 95683 | 782097 | see also 7323 line | |
| 7327 | C20orf31 | NM_018217.1 | 757 | ctgggagccgtcagatatcg | 138431 | 543703 | - | |
| 7328 | CHFR | NM_018223.1 | 408 | acgtggcatacctctatgaatct | 306698 | 777938 | - | |
| 7329 | RNPEPL1 | NM_018226.2 | 1038 | gggctactgcttcgtgtactacc | 186409 | 478131 | - | membrane alanyl aminopeptidase |
| 7330 | CARP-1 | NM_018237.1 | 148 | cgccatgggctactcagtttaca | 23584 | 453367 | - | |
| 7331 | CARP-1 | NM_018237.1 | 293 | agcaaactatcagttaacacaaa | 23589 | 453372 | see also 7330 line | |
| 7332 | CARP-1 | NM_018237.1 | 728 | gagaattcaaacactaccaaatc | 23602 | 453383 | see also 7330 line | |
| 7333 | CARP-1 | NM_018237.1 | 737 | aacactaccaaatcagaatcagt | 23603 | 453384 | see also 7330 line | |
| 7334 | CARP-1 | NM_018237.1 | 1473 | atggctagccctagtatggaaga | 23621 | 453407 | see also 7330 line | |
| 7335 | CARP-1 | NM_018237.1 | 1558 | atcctgctagacttgttaagtt | 23625 | 453410 | see also 7330 line | |
| 7336 | CARP-1 | NM_018237.1 | 1559 | tcctgctagacttgttaagtttt | 23626 | 453411 | see also 7330 line | |
| 7337 | CARP-1 | NM_018237.1 | 1743 | tggtaccgttttgcagagattcg | 23637 | 453418 | see also 7330 line | |
| 7338 | CARP-1 | NM_018237.1 | 1785 | acccacaagggcggtacagttcc | 23638 | 453419 | see also 7330 line | |
| 7339 | FLJI0842 | NM_018238.2 | 441 | cagatgaggctaccttcagtaag | 208439 | 597966 | kinase_related | |
| 7340 | FLJI0842 | NM_018238.2 | 555 | atgccacacttgccattgtgaaa | 208441 | 597972 | see also 7339 line | |
| 7341 | FLJI0847 | NM_018242.2 | 1353 | atcggatcgcgctgatgtttgc | 258586 | 643019 | - | |
| 7342 | FLJI0853 | NM_018246.1 | 307 | ctgcaagatgaacaacgttaatg | 368974 | 758558 | - | |
| 7343 | FLJI0853 | NM_018246.1 | 308 | tgcaagatgaacaacgttaatgt | 368975 | 758559 | see also 7342 line | |
| 7344 | C6orf67 | NM_018247.1 | 547 | tacgtgaaatctcgagatgatag | 278013 | 747580 | - | |
| 7345 | C6orf67 | NM_018247.1 | 1175 | tgctagtaattaatcataaatat | 278043 | 747608 | see also 7344 line | |
| 7346 | FLJI0876 | NM_018254.2 | 241 | atcccagatgccaaattggatga | 26717 | 456831 | - | |
| 7347 | WDR10 | NM_018262.2 | 2096 | gaccaacaatgacctgtttctgg | 369382 | 742846 | - | |
| 7348 | ASXL2 | NM_018263.2 | 130 | gccgccaagacggtcttagaaaa | 369409 | 773392 | - | |
| 7349 | ASXL2 | NM_018263.2 | 131 | ccgccaagacggtcttagaaaaa | 369410 | 773393 | see also 7348 line | |
| 7350 | ASXL2 | NM_018263.2 | 1144 | gtgagaattgacaagagattga | 369430 | 773409 | see also 7348 line | |
| 7351 | ASXL2 | NM_018263.2 | 2920 | atccctgctaataatcctttagt | 369469 | 773450 | see also 7348 line | |
| 7352 | ASXL2 | NM_018263.2 | 4046 | cccagataggccaagctataga | 369502 | 773479 | see also 7348 line | |
| 7353 | ASXL2 | NM_018263.2 | 4059 | aagctatagaggcatgatcaatg | 369503 | 773481 | see also 7348 line | |
| 7354 | ASXL2 | NM_018263.2 | 4060 | agctatagaggcatgatcaatgt | 369504 | 773482 | see also 7348 line | |
| 7355 | ASXL2 | NM_018263.2 | 4126 | agccaggtattctagcaatgtagg | 369505 | 773486 | see also 7348 line | |
| 7356 | FLJI0902 | NM_018266.1 | 1317 | atgtgagcacctcattagtgt | 369543 | 724802 | - | |
| 7357 | FLJI0902 | NM_018266.1 | 1511 | tggtcggcacagcagagtttta | 369546 | 724808 | see also 7356 line | |
| 7358 | FLJI0902 | NM_018266.1 | 1514 | tcggcacagcagatgtttatat | 369547 | 724809 | see also 7356 line | |
| 7359 | FLJI0902 | NM_018266.1 | 1729 | tctgcaactactatgtttatt | 369558 | 724815 | see also 7356 line | |
| 7360 | FLJI0922 | NM_018273.2 | 214 | cagtaccgcgagcgcttcattcc | 369619 | 753887 | - | |

336

Figure 46

| | | | | | | | | | |
|---|---|---|---|---|---|---|---|---|---|
| 7361 | FLJ10925 | NM_018275.3 | 1321 | cggacctacgagcgtttcactgt | 236173 | 768297 | - | - | - |
| 7362 | PSPC1 | NM_018282.1 | 980 | tgctcaacctgggacatttgaat | 65119 | 485528 | - | - | - |
| 7363 | ARHGAP12 | NM_018287.4 | 657 | ttcgtgatgccaatcagaatttt | 369706 | 738416 | - | - | - |
| 7364 | ARHGAP12 | NM_018287.4 | 755 | gactcacattctcctaaagtttc | 369707 | 738423 | see also 7363 line | | |
| 7365 | ARHGAP12 | NM_018287.4 | 1361 | tggctcaagcatgttgatgatca | 369732 | 738443 | see also 7363 line | | |
| 7366 | ARHGAP12 | NM_018287.4 | 1364 | ctcaagcatgttgatgatcaagg | 369733 | 738444 | see also 7363 line | | |
| 7367 | ARHGAP12 | NM_018287.4 | 1951 | cactgttattaatgattggttta | 369747 | 738459 | see also 7363 line | | |
| 7368 | ARHGAP12 | NM_018287.4 | 1953 | ctgttattaatgattggtttaaa | 369748 | 738460 | see also 7363 line | | |
| 7369 | PHF10 | NM_018288.2 | 378 | ttgcgcagtgatgaagtgattga | 34654 | 452992 | transcription_regulator | - | - |
| 7370 | PHF10 | NM_018288.2 | 1138 | aggctgaatcacttatacactgc | 34673 | 453012 | see also 7369 line | | |
| 7371 | PHF10 | NM_018288.2 | 1364 | atcaggtcgctggatttgtgact | 34678 | 453020 | see also 7369 line | | |
| 7372 | NGLY1 | NM_018297.2 | 1354 | ttgtggagcttgttgaatttata | 369944 | 594004 | - | - | - |
| 7373 | FLJ11016 | NM_018301.2 | 516 | ctgatcgtcactccttccttaag | 61075 | 482452 | - | - | - |
| 7374 | SEC5 | NM_018303.4 | 910 | aagtattaggtcggaaagacaag | 270393 | 717359 | - | - | - |
| 7375 | SEC5 | NM_018303.4 | 1081 | cggaggtgcaagttttcaagaaa | 270396 | 717369 | see also 7374 line | | |
| 7376 | SEC5 | NM_018303.4 | 1084 | aggtgcaagttttcaagaaatat | 270397 | 717370 | see also 7374 line | | |
| 7377 | SEC5 | NM_018303.4 | 2512 | caggtgtcagaaactatttaaaa | 270434 | 717422 | see also 7374 line | | |
| 7378 | ARHT1 | NM_018307.2 | 351 | atggattcctctcataaatgaaa | 108428 | 518898 | signal_transduction | - | - |
| 7379 | ARHT1 | NM_018307.2 | 422 | ctgatctggtggaatatagtagt | 108429 | 518900 | see also 7378 line | | |
| 7380 | ARHT1 | NM_018307.2 | 865 | tggactgtgcttcgacgatttgg | 108439 | 518914 | see also 7378 line | | |
| 7381 | ARHT1 | NM_018307.2 | 869 | ctgtgcttcgacgatttggttat | 108440 | 518915 | see also 7378 line | | |
| 7382 | ARHT1 | NM_018307.2 | 1574 | ttgaatactgtgccaggattttt | 108473 | 518936 | see also 7378 line | | |
| 7383 | ARHT1 | NM_018307.2 | 1752 | tgcccccagtaaggatatctttg | 108478 | 518941 | see also 7378 line | | |
| 7384 | FLJ11046 | NM_018309.1 | 88 | agcaggaggttgtgatcttgaaa | 370031 | 723063 | - | - | - |
| 7385 | FLJ11046 | NM_018309.1 | 206 | ttggcatcatgggatggtatttt | 370035 | 723066 | see also 7384 line | | |
| 7386 | FLJ11046 | NM_018309.1 | 208 | ggcatcatgggatggtattttag | 370036 | 723068 | see also 7384 line | | |
| 7387 | FLJ11046 | NM_018309.1 | 214 | atgggatggtattttagacttgc | 370037 | 723070 | see also 7384 line | | |
| 7388 | FLJ11046 | NM_018309.1 | 1979 | aggtatttgattccaaatgcagg | 370093 | 723115 | see also 7384 line | | |
| 7389 | C11orf23 | NM_018312.2 | 650 | agcaaggtgctaagtattcttat | 370105 | 739212 | - | - | - |
| 7390 | C11orf23 | NM_018312.2 | 938 | ctgagcagagaccagatgttaca | 370117 | 739218 | see also 7389 line | | |
| 7391 | C11orf23 | NM_018312.2 | 1649 | aagaggaacacggtagatctagc | 370136 | 739248 | see also 7389 line | | |

337

Figure 46

| 7392 | C11orf23 | NM_018312.2 | 1689 | tgcaacaaatgacgtccaatttt | 370138 | 739250 | see also 7389 line | | |
|---|---|---|---|---|---|---|---|---|---|
| 7393 | C11orf23 | NM_018312.2 | 1692 | aacaaatgacgtccaattttatt | 370139 | 739251 | see also 7389 line | | |
| 7394 | FBXW7 | NM_018315.2 | 309 | tacagaaaatatgggtttctacg | 370162 | 443810 | - | - | - |
| 7395 | FBXW7 | NM_018315.2 | 319 | atgggtttctacggcacattaaa | 370163 | 443811 | see also 7394 line | | |
| 7396 | FBXW7 | NM_018315.2 | 320 | tgggtttctacggcacattaaaa | 370164 | 443812 | see also 7394 line | | |
| 7397 | FBXW7 | NM_018315.2 | 321 | gggtttctacggcacattaaaaa | 370165 | 443813 | see also 7394 line | | |
| 7398 | FBXW7 | NM_018315.2 | 1519 | gtgagtggatctcttgatacatc | 370201 | 443839 | see also 7394 line | | |
| 7399 | FLJ11082 | NM_018317.1 | 605 | tcgcaacccaaattatgaaaacg | 370217 | 753093 | - | - | - |
| 7400 | TDP1 | NM_018319.2 | 1000 | ctgccaggcaaagttggatattg | 199018 | 734207 | - | - | - |
| 7401 | TDP1 | NM_018319.2 | 1027 | tggaacacaccacacgaaaatga | 199019 | 734211 | see also 7400 line | | |
| 7402 | TDP1 | NM_018319.2 | 1620 | ggggctctcttccctatagcatc | 199041 | 734222 | see also 7400 line | | |
| 7403 | RNF121 | NM_018320.3 | 412 | gacaaccccaaggttggtttata | 370285 | 738138 | - | - | - |
| 7404 | BRIX | NM_018321.2 | 247 | atgttgatgcctcattctaaagc | 370303 | 676207 | - | molecular_function unknown | - |
| 7405 | BRIX | NM_018321.2 | 787 | aacatgcatcggcgtgtcataag | 370320 | 676226 | see also 7404 line | | |
| 7406 | BRIX | NM_018321.2 | 791 | tgcatcggcgtgtcataagatcc | 370322 | 676227 | see also 7404 line | | |
| 7407 | LRRN3 | NM_018334.3 | 778 | agctatcactacactagtacaag | 370439 | 701473 | - | - | - |
| 7408 | LRRN3 | NM_018334.3 | 1953 | atgttcggcaagtgcatttcagg | 370488 | 701523 | see also 7407 line | | |
| 7409 | LRRN3 | NM_018334.3 | 2457 | atgtcaaggtatataatcttact | 370507 | 701542 | see also 7407 line | | |
| 7410 | RIOK2 | NM_018343.1 | 258 | tccagggctatcggttgacaaat | 283310 | 666704 | - | - | - |
| 7411 | RIOK2 | NM_018343.1 | 260 | cagggctatcggttgacaaatgc | 283311 | 666705 | see also 7410 line | | |
| 7412 | ABCF3 | NM_018358.1 | 642 | gtgcgaattgagaactttgatgt | 71055 | 492837 | - | - | - |
| 7413 | FLJ11200 | NM_018359.1 | 1406 | aaggatgcatactataacttatg | 370738 | 752497 | - | - | - |
| 7414 | LPAAT-e | NM_018361.1 | 183 | caccggggtccagatattgctat | 215531 | 602847 | - | - | - |
| 7415 | LPAAT-e | NM_018361.1 | 184 | accggggtccagatattgctata | 215532 | 602848 | see also 7414 line | | |
| 7416 | LPAAT-e | NM_018361.1 | 190 | gtccagatattgctatatggaga | 215534 | 602849 | see also 7414 line | | |
| 7417 | FLJ11220 | NM_018364.2 | 834 | gcgaggaagacgccttaaaatgt | 370804 | 777541 | - | - | - |
| 7418 | FLJ11220 | NM_018364.2 | 840 | aagacgccttaaaatgtacaata | 370806 | 777542 | see also 7417 line | | |
| 7419 | FLJ11220 | NM_018364.2 | 1214 | aggagtttcttgctttgacattc | 370823 | 777550 | see also 7417 line | | |
| 7420 | PHCA | NM_018367.3 | 273 | agcactcacagtggtaggaatgg | 205409 | 596103 | - | hydrolase | - |
| 7421 | PHCA | NM_018367.3 | 516 | tggaatgttggtctttacattag | 205416 | 596113 | see also 7420 line | | |
| 7422 | PHCA | NM_018367.3 | 520 | atgttggtctttacattagtact | 205417 | 596114 | see also 7420 line | | |
| 7423 | XTP1 | NM_018369.1 | 819 | gcctatgtacttgggatttgaaa | 317535 | 680567 | - | - | - |
| 7424 | FLJ11259 | NM_018370.1 | 453 | aaggatatccacagaaatcaatg | 370894 | 731206 | - | - | - |
| 7425 | FLJ11259 | NM_018370.1 | 454 | aggatatccacagaaatcaatgg | 370895 | 731207 | see also 7424 line | | |
| 7426 | ChGn | NM_018371.3 | 1426 | atggccaacacgcttatcaatgt | 218544 | 606422 | - | - | - |
| 7427 | ChGn | NM_018371.3 | 1428 | ggccaacacgcttatcaatgtta | 218545 | 606423 | see also 7426 line | | |

Figure 46

| | | | | | | | | | |
|---|---|---|---|---|---|---|---|---|---|
| 7428 | ChGn | NM_018371.3 | 1429 | gccaacacgcttatcaatgttat | 218546 | 606424 | see also 7426 line | | |
| 7429 | FLJ11294 | NM_018383.2 | 1941 | gtctcagattcctcaaggttttc | 280182 | 655029 | - | molecular_function unknown | - |
| 7430 | FLJ11294 | NM_018383.2 | 4095 | ctggggtagagggagtaacatga | 280199 | 655040 | see also 7429 line | | |
| 7431 | FLJ11294 | NM_018383.2 | 4097 | ggggtagagggagtaacatgaac | 280200 | 655041 | see also 7429 line | | |
| 7432 | STRBP | NM_018387.2 | 928 | acctctaattagggacgaattgg | 48829 | 472119 | - | - | - |
| 7433 | STRBP | NM_018387.2 | 1127 | cccacatgggcaccattgaaagg | 48836 | 472122 | see also 7432 line | | |
| 7434 | STRBP | NM_018387.2 | 1222 | gagacgagtaatggagtgtttgg | 48842 | 472127 | see also 7432 line | | |
| 7435 | STRBP | NM_018387.2 | 2157 | ctggacccaatgcggcaaataat | 48868 | 472151 | see also 7432 line | | |
| 7436 | STRBP | NM_018387.2 | 2161 | acccaatgcggcaaataataaga | 48870 | 472152 | see also 7432 line | | |
| 7437 | FLJ11336 | NM_018393.2 | 1670 | ctcgcctggtcaactataacaag | 371138 | 793879 | - | - | - |
| 7438 | FLJ11336 | NM_018393.2 | 1701 | ctgtccctactacgatgcaatcc | 371139 | 793882 | see also 7437 line | | |
| 7439 | CHDH | NM_018397.1 | 1731 | acccctcgtgcacctgtaagatg | 226461 | 772106 | - | - | - |
| 7440 | CACNA2D3 | NM_018398.1 | 452 | accaacgaacatgtacaacaaag | 328423 | 694340 | - | - | - |
| 7441 | CACNA2D3 | NM_018398.1 | 501 | tggtctgaatctctaaacaaagt | 328427 | 694345 | see also 7440 line | | |
| 7442 | CACNA2D3 | NM_018398.1 | 1019 | ggccatcatgctcataactgatg | 328440 | 694353 | see also 7440 line | | |
| 7443 | CACNA2D3 | NM_018398.1 | 1366 | tagccatgcctgtgtttagtaag | 328449 | 694361 | see also 7440 line | | |
| 7444 | CACNA2D3 | NM_018398.1 | 1755 | atcaagggtactcctttcagttt | 328461 | 694377 | see also 7440 line | | |
| 7445 | CACNA2D3 | NM_018398.1 | 1758 | aagggtactcctttcagtttagg | 328462 | 694378 | see also 7440 line | | |
| 7446 | VNN3 | NM_018399.2 | 589 | acgctaccataagtacaatcttt | 203558 | 594459 | - | - | - |
| 7447 | SCN3B | NM_018400.2 | 1004 | gggcggtaaagatttccttattt | 262249 | 647235 | channel | voltage-gated sodium channel | - |
| 7448 | HSA275986 | NM_018403.1 | 672 | aagcaccgagactctagaagaaa | 371153 | 747920 | - | - | - |
| 7449 | CENTA2 | NM_018404.1 | 373 | cagagtcccagctttctactaca | 23984 | 457111 | - | - | - |
| 7450 | ST7 | NM_018412.2 | 515 | gaggggttgacaacaactcttcc | 371250 | 712438 | - | - | ovarian carcinoma |
| 7451 | ST7 | NM_018412.2 | 584 | gtgtctcagaatgcaaagtatgg | 371252 | 712441 | see also 7450 line | | |
| 7452 | ST7 | NM_018412.2 | 1585 | tacccaatctgtacagaaacagc | 371281 | 712475 | see also 7450 line | | |
| 7453 | CHST11 | NM_018413.2 | 1280 | cagctgtacgaagtctacaaact | 218568 | 605562 | - | - | - |
| 7454 | TRERF1 | NM_018415.1 | 1027 | aagggcacctgtattatgactac | 42885 | 469904 | transcription_regulator | - | - |
| 7455 | TRERF1 | NM_018415.1 | 1028 | agggcacctgtattatgactacc | 42886 | 469905 | see also 7454 line | | |

Figure 46

| | | | | | | | | |
|---|---|---|---|---|---|---|---|---|
| 7456 | TRERF1 | NM_018415.1 | 2419 | atgttgaaccagcatcaacatt | 42895 | 469916 | see also 7454 line | |
| 7457 | TRERF1 | NM_018415.1 | 2717 | atgtcatccttagccaaattacc | 42900 | 469923 | see also 7454 line | |
| 7458 | TRERF1 | NM_018415.1 | 2801 | cacttacagcaaagactttattt | 42903 | 469926 | see also 7454 line | |
| 7459 | FHX | NM_018416.2 | 1541 | aagacaccctccagatgatgatc | 3669 | 444015 | transcription_regulator | translation regulator |
| 7460 | FHX | NM_018416.2 | 2453 | gacccctgaccagcatcacattg | 3680 | 444020 | see also 7459 line | - |
| 7461 | EPB41L4B | NM_018424.1 | 582 | agcgtggacctgccgaaacatgc | 273441 | 656499 | - | |
| 7462 | EPB41L4B | NM_018424.1 | 1161 | ctgacccgacacaggcatattaat | 273453 | 656516 | see also 7461 line | |
| 7463 | EPB41L4B | NM_018424.1 | 1163 | gaccccgacacaggcatattaatct | 273454 | 656517 | see also 7461 line | |
| 7464 | EPB41L4B | NM_018424.1 | 1165 | ccccgacacaggcatattaatcttt | 273455 | 656518 | see also 7461 line | |
| 7465 | EPB41L4B | NM_018424.1 | 1166 | cccgacacaggcatattaatctttg | 273456 | 656519 | see also 7461 line | |
| 7466 | EPB41L4B | NM_018424.1 | 1182 | atctttgaaggagctaaccaaaat | 273458 | 656520 | see also 7461 line | |
| 7467 | PI4KII | NM_018425.2 | 684 | ccgtacaaggtagtataacctgg | 207982 | 597907 | kinase_related | - |
| 7468 | PI4KII | NM_018425.2 | 1131 | ctgagggcatatccttttact | 207989 | 597912 | see also 7467 line | - |
| 7469 | DOK5 | NM_018431.2 | 647 | ttgatgccatcctcaacttaga | 170553 | 567107 | - | |
| 7470 | RNF130 | NM_018434.4 | 493 | acgcggagccaggagtactacacg | 191635 | 585179 | - | |
| 7471 | RNF130 | NM_018434.4 | 868 | ctgtcatgataacagaattgaag | 191645 | 585186 | see also 7470 line | |
| 7472 | RNF130 | NM_018434.4 | 986 | ctctcttagttctcgtgtcaatat | 191650 | 585189 | see also 7470 line | |
| 7473 | RNF130 | NM_018434.4 | 1062 | cagaagatcaggtacacaaatgc | 191653 | 585193 | see also 7470 line | |
| 7474 | RNF130 | NM_018434.4 | 1126 | aagccatcagtaaattgacaacc | 191654 | 585194 | see also 7470 line | |
| 7475 | RNF130 | NM_018434.4 | 1310 | tcctatgtgcaaacttaatatat | 191657 | 585201 | see also 7470 line | |
| 7476 | RNF130 | NM_018434.4 | 1539 | atcaacattgcagtaacaaaaga | 191663 | 585210 | see also 7470 line | |
| 7477 | RNF130 | NM_018434.4 | 1628 | cacagctagcttgaatgctaatg | 191665 | 585211 | see also 7470 line | |
| 7478 | FBXO6 | NM_018438.2 | 518 | aagtcaagaagtatttgtcaca | 371545 | 706118 | - | - |
| 7479 | IMPACT | NM_018439.1 | 112 | gaggagtggtgtcattgatga | 280925 | 667318 | - | - |
| 7480 | SELS | NM_018445.2 | 239 | tggaacctgatgttgttgttaaa | 327348 | 715425 | receptor_acitivity | |
| 7481 | AD-017 | NM_018446.1 | 972 | cacctcctctgcttatcgtattt | 212046 | 600438 | - | - |
| 7482 | AD-017 | NM_018446.1 | 973 | acctcctctgcttatcgtatttt | 212047 | 600439 | see also 7481 line | |
| 7483 | AD-017 | NM_018446.1 | 976 | tcctctgcttatcgtatttatc | 212048 | 600441 | see also 7481 line | |
| 7484 | LOC55831 | NM_018447.1 | 686 | atcctggtacttcctcaatgtat | 371569 | 785469 | - | |
| 7485 | ARHGAP15 | NM_018460.2 | 588 | ttccttctacagtcagatattga | 371832 | 771272 | - | |
| 7486 | ARHGAP15 | NM_018460.2 | 974 | gtggtttgtaaagcaatgcattg | 371848 | 771284 | see also 7485 line | |
| 7487 | MDS027 | NM_018462.3 | 185 | agcccttgaacggagaatagagt | 371877 | 749216 | - | - |
| 7488 | MDS027 | NM_018462.3 | 187 | ccccttgaacggagaatagagtac | 371878 | 749217 | see also 7487 line | |
| 7489 | MDS027 | NM_018462.3 | 194 | acgggaataagagtacattgaag | 371879 | 749218 | see also 7487 line | |

Figure 46

| ID | Gene | Accession | Pos | Sequence | | | Note |
|---|---|---|---|---|---|---|---|
| 7490 | C9orf46 | NM_018465.1 | 352 | gtctcgggaattcctcaaatatt | 371888 | 724320 | - |
| 7491 | C9orf46 | NM_018465.1 | 354 | ctgggaattcctcaaaatattt | 371889 | 724321 | see also 7490 line |
| 7492 | ACTR10 | NM_018477.1 | 265 | aagaattatattcctacctaaag | 286251 | 531617 | - |
| 7493 | ACTR10 | NM_018477.1 | 275 | ttcctacctaaaggaattcatcc | 286252 | 531618 | see also 7492 line |
| 7494 | ACTR10 | NM_018477.1 | 463 | ttctgaagcttggaattaattct | 286263 | 531626 | see also 7492 line |
| 7495 | ASH1L | NM_018489.1 | 2485 | agcacatgccggtctccaaaagg | 21630 | 455113 | transcription regulator |
| 7496 | ASH1L | NM_018489.1 | 2636 | aacgccgattgccaaagttgagc | 21640 | 455125 | see also 7495 line |
| 7497 | ASH1L | NM_018489.1 | 3614 | ttcttccatcacctatttgtct | 21664 | 455155 | see also 7495 line |
| 7498 | ASH1L | NM_018489.1 | 3729 | cagacagggcagtatgattcaga | 21668 | 455159 | see also 7495 line |
| 7499 | ASH1L | NM_018489.1 | 5720 | aacgccaagctcggaaaatgtgc | 21719 | 455217 | see also 7495 line |
| 7500 | ASH1L | NM_018489.1 | 5726 | aagctcggaaaatgtgcaattac | 21720 | 455218 | see also 7495 line |
| 7501 | ASH1L | NM_018489.1 | 5727 | agctcggaaaatgtgcaattacg | 21721 | 455219 | see also 7495 line |
| 7502 | ASH1L | NM_018489.1 | 6480 | ttggcagttgaaacacaatcagc | 21741 | 455231 | see also 7495 line |
| 7503 | ASH1L | NM_018489.1 | 8457 | tgccttggtcaccattattcc | 21798 | 455280 | see also 7495 line |
| 7504 | ASH1L | NM_018489.1 | 8655 | ctgtgattatcggcttgacaagt | 21812 | 455283 | see also 7495 line |
| 7505 | ASH1L | NM_018489.1 | 8792 | acgtcccagacaactacaagagg | 21813 | 455289 | see also 7495 line |
| 7506 | FLJ22054 | NM_018527.2 | 1343 | tgctagtactccaactctaatag | 387276 | 719460 | - |
| 7507 | FLJ22054 | NM_018527.2 | 1346 | tagtactccaactctaatagaat | 387277 | 719461 | see also 7506 line |
| 7508 | HSA277841 | NM_018553.1 | 216 | ttcatgaagcgaagatatcatt | 372190 | 719237 | |
| 7509 | HSA277841 | NM_018553.1 | 290 | acgttggcttgacgtcgtataaa | 372191 | 719239 | see also 7508 line |
| 7510 | HSA277841 | NM_018553.1 | 294 | tggcttgacgtcgtataaacatc | 372192 | 719240 | see also 7508 line |
| 7511 | HSA277841 | NM_018553.1 | 794 | tacgccagcgttaggaaaaga | 372204 | 719249 | see also 7508 line |
| 7512 | LRP1B | NM_018557.1 | 2635 | ctggtaaaccctcgtgcttaga | 31100 | 549537 | receptor acitivity |
| 7513 | LRP1B | NM_018557.1 | 6176 | cagttggtctatcgatagactat | 31223 | 549627 | see also 7512 line |
| 7514 | LRP1B | NM_018557.1 | 6953 | gcctgatcaaccaagatctata | 31254 | 549653 | see also 7512 line |
| 7515 | LRP1B | NM_018557.1 | 6955 | ctggatcaaccaagatctatagc | 31255 | 549654 | see also 7512 line |
| 7516 | LRP1B | NM_018557.1 | 10497 | acctatggcactaacaatagatt | 31361 | 549755 | see also 7512 line |
| 7517 | LRP1B | NM_018557.1 | 10501 | atggcactaacaatagattatgt | 31362 | 549756 | see also 7512 line |
| 7518 | LRP1B | NM_018557.1 | 10503 | ggcactaacaatagattatgtta | 31363 | 549757 | see also 7512 line |
| 7519 | LRP1B | NM_018557.1 | 10587 | tagacacaagtccctaatcaag | 31367 | 549763 | see also 7512 line |
| 7520 | LRP1B | NM_018557.1 | 11287 | gactatttccagtgtaagactac | 31386 | 549794 | see also 7512 line |
| 7521 | LRP1B | NM_018557.1 | 13945 | tgccaccactattgtgtgaattc | 31468 | 549875 | see also 7512 line |
| 7522 | LRP1B | NM_018557.1 | 14018 | cgcgctatgaaggaccaaaatgt | 31473 | 549877 | see also 7512 line |

341

Figure 46

| | | | | | | | |
|---|---|---|---|---|---|---|---|
| 7523 | LRP1B | NM_018557.1 | 14584 | cgccccatctacctaaactctga | 31485 | 549903 | see also 7512 line |
| 7524 | GABRQ | NM_018558.1 | 1171 | acgtgcaggatggcctgattaac | 178839 | 572071 | receptor_acitivity, channel, signal_transduction, neurotransmitter transporter |
| 7525 | MGC20741 | NM_018561.3 | 1570 | ctcagtcaggtcacatgtatatc | 188585 | 582172 | - |
| 7526 | MGC20741 | NM_018561.3 | 1581 | cacatgtatatcatgcaattaca | 188586 | 582174 | see also 7525 line |
| 7527 | ALS2CR2 | NM_018571.4 | 879 | ttgaactatctgcaccaaaatgg | 72016 | 493781 | kinase_related |
| 7528 | GALNACT-2 | NM_018590.3 | 822 | ctcactgccatcctgaagagaaa | 218692 | 741612 | - |
| 7529 | GALNACT-2 | NM_018590.3 | 1091 | ccctttggacctctcatgaaag | 218696 | 741621 | see also 7528 line |
| 7530 | GALNACT-2 | NM_018590.3 | 1096 | ttggacctctcatgaaagtgaag | 218697 | 741622 | see also 7528 line |
| 7531 | DNAJA4 | NM_018602.2 | 361 | cccggatgaggcgcgagaagttta | 299353 | 668426 | - |
| 7532 | DNAJA4 | NM_018602.2 | 626 | ttggccctccagaaaaatgtaat | 299360 | 668432 | see also 7531 line |
| 7533 | DNAJA4 | NM_018602.2 | 627 | tggccctccagaaaaatgtaatt | 299361 | 668433 | see also 7531 line |
| 7534 | CHST12 | NM_018641.2 | 1327 | ctctacgaggccgactttgttct | 218575 | 606518 | - |
| 7535 | NOLA3 | NM_018648.2 | 227 | caccgaatcaccatcaagaaacg | 282604 | 716199 | - |
| 7536 | MARK1 | NM_018650.2 | 2804 | agcgaatatctgggacatctatt | 90231 | 507936 | kinase_related |
| 7537 | MARK1 | NM_018650.2 | 2805 | gcgaatatctgggacatctattg | 90232 | 507937 | see also 7536 line |
| 7538 | MYNN | NM_018657.2 | 1578 | tcctcaggagagctcaacaacaca | 7311 | 537836 | transcription_regulator |
| 7539 | SMPD3 | NM_018667.1 | 1448 | tggctgatttcgaaaatctacc | 199000 | 591016 | - |
| 7540 | VPS33B | NM_018668.2 | 837 | gagatcagcgttgatcaacact | 271391 | 652966 | - |
| 7541 | VPS33B | NM_018668.2 | 1153 | gagcctgaaggtgctactcaatg | 271394 | 652971 | see also 7540 line |
| 7542 | VPS33B | NM_018668.2 | 1769 | gccaagaagagcaatttcgtgc | 271409 | 652990 | see also 7540 line |
| 7543 | VPS33B | NM_018668.2 | 1786 | tcgtgccatcagcaaaaagctga | 271410 | 652991 | see also 7540 line |
| 7544 | ABCA5 | NM_018672.2 | 1257 | gaccagaacacttctactgaaga | 68951 | 490941 | - |
| 7545 | ABCA5 | NM_018672.2 | 1623 | gtcctatgaacttcgtttttc | 68964 | 490955 | see also 7544 line |
| 7546 | ABCA5 | NM_018672.2 | 4132 | ttcaacagtactatggtttattc | 69044 | 491029 | see also 7544 line |
| 7547 | ABCA5 | NM_018672.2 | 5765 | cagtacaacatctaaagagtaaa | 69091 | 491078 | see also 7544 line |
| 7548 | ACCN4 | NM_018674.3 | 983 | tactcgctatgggaagtgttaca | 262538 | 645992 | channel |
| 7549 | ACCN4 | NM_018674.3 | 1419 | gagaccatctgcccaccaaatat | 262544 | 645997 | see also 7548 line |
| 7550 | ACAS2 | NM_018677.2 | 625 | gggcttgcactccattgtgttt | 108108 | 617417 | - |
| 7551 | ACAS2 | NM_018677.2 | 1154 | ttggatcactggtcattcctacg | 108113 | 617426 | see also 7550 line |
| 7552 | ACAS2 | NM_018677.2 | 1564 | ctcctgcaatctgaatgagtcc | 108116 | 617433 | see also 7550 line |

342

Figure 46

| | | | | | | | | | |
|---|---|---|---|---|---|---|---|---|---|
| 7553 | ACAS2 | NM_018677.2 | 1644 | gggatcatcgcgcacagtctatgg | 108118 | 617435 | see also 7550 line | | |
| 7554 | ZNF313 | NM_018683.3 | 308 | gccatgctgccgtaagaattc | 372534 | 742521 | - | | |
| 7555 | ANLN | NM_018685.2 | 889 | cagcctggtaccgcttgtttatc | 115227 | 734481 | - | | |
| 7556 | ANLN | NM_018685.2 | 890 | agcctggtaccgcttgtttatcc | 115228 | 734482 | see also 7555 line | | |
| 7557 | ANLN | NM_018685.2 | 893 | ctggtaccgcttgtttatccaaa | 115229 | 734483 | see also 7555 line | | |
| 7558 | ANLN | NM_018685.2 | 1330 | cgctttggagagcgttgtcaaga | 115245 | 734491 | see also 7555 line | | |
| 7559 | ANLN | NM_018685.2 | 3280 | aagaatcccatagagaggataaa | 115296 | 734555 | see also 7555 line | | |
| 7560 | ANLN | NM_018685.2 | 3381 | tactgtccgaccacaaagagaag | 115299 | 734561 | see also 7555 line | | |
| 7561 | CMAS | NM_018686.3 | 608 | cagtttcgatggagtgaaattca | 208973 | 598636 | - | | |
| 7562 | KIAA1199 | NM_018689.1 | 865 | tccattctgaccggtttgacacc | 372564 | 742430 | - | | |
| 7563 | KIAA1199 | NM_018689.1 | 2461 | gggctggcatgatcatagacaac | 372613 | 742443 | see also 7562 line | | |
| 7564 | C5orf3 | NM_018691.1 | 1291 | aactcattatgggctactcttg | 111290 | 724190 | - | | |
| 7565 | ELAC1 | NM_018696.1 | 133 | aggcgagtgctggctcttgact | 372642 | 746223 | - | | |
| 7566 | ELAC1 | NM_018696.1 | 336 | tagggcttcgggactttatctgg | 372649 | 746231 | see also 7565 line | | |
| 7567 | ELAC1 | NM_018696.1 | 1088 | gtgacttcagcagaagatttat | 372656 | 746243 | see also 7565 line | | |
| 7568 | LANCL2 | NM_018697.2 | 1229 | tggagtgtagcgatgtgatttgg | 372691 | 747739 | - | | |
| 7569 | PRDM5 | NM_018699.1 | 1048 | gagatattgattgtgtcaagaatg | 36883 | 689617 | transcription_regulator | DNA binding | |
| 7570 | PRDM5 | NM_018699.1 | 1569 | tccatattgtggccagaaattg | 36901 | 689628 | see also 7569 line | | |
| 7571 | DKFZp547A023 | NM_018704.1 | 274 | cacagagatactttcattgaaga | 372699 | 742004 | - | | |
| 7572 | DKFZp762O076 | NM_018710.1 | 321 | atgaagctacgccaatcaaaaac | 372755 | 733592 | - | | |
| 7573 | DKFZp762O076 | NM_018710.1 | 597 | gcccacactgcaaaaaaatctcc | 372766 | 733599 | see also 7572 line | | |
| 7574 | DKFZp547C176 | NM_018712.2 | 855 | tgcaattgtgggcatcaataaa | 372801 | 798939 | - | | |
| 7575 | COG1 | NM_018714.1 | 576 | agccacttccggtcaactattct | 268370 | 650689 | - | | |
| 7576 | A2BP1 | NM_018723.2 | 1810 | gcggtgcacgtgtacaacac | 49521 | 472987 | - | | |
| 7577 | A2BP1 | NM_018723.2 | 2090 | taggagccatgctgatgatgtgg | 49525 | 473006 | see also 7576 line | | |
| 7578 | A2BP1 | NM_018723.2 | 2104 | atgatgtgggtctcgttcttct | 49526 | 472993 | see also 7576 line | | |
| 7579 | A2BP1 | NM_018723.2 | 2123 | ttcttcattgcaggctagtatat | 49530 | 472995 | see also 7576 line | | |
| 7580 | A2BP1 | NM_018723.2 | 2126 | ttcattgcaggctagtatatacc | 49531 | 472996 | see also 7576 line | | |
| 7581 | A2BP1 | NM_018723.2 | 2151 | gggggatacaaccgttttgctcc | 49532 | 472997 | see also 7576 line | | |
| 7582 | IL17RB | NM_018725.2 | 269 | tgccagcatccgcttgttgaagg | 124304 | 635399 | receptor_acitivity | | |
| 7583 | MATR3 | NM_018834.3 | 410 | tgctaccagtcttaagtatgc | 52413 | 474004 | transcription_regulator | structural molecule | |
| 7584 | MATR3 | NM_018834.3 | 461 | tactgcacgccttgctagtttaa | 52414 | 474005 | see also 7583 line | | |

Figure 46

| | | | | | | | | | |
|---|---|---|---|---|---|---|---|---|---|
| 7585 | MATR3 | NM_018834.3 | 466 | cacgcctgctagtttaatgaat | 52416 | 474007 | see also 7583 line | | |
| 7586 | MATR3 | NM_018834.3 | 495 | atgagttcttcattgaatcaaca | 52418 | 474010 | see also 7583 line | | |
| 7587 | MATR3 | NM_018834.3 | 534 | ctgtcttctgctagtacttcttc | 52419 | 474011 | see also 7583 line | | |
| 7588 | MATR3 | NM_018834.3 | 554 | ttcccataatttgcagtctatat | 52420 | 474013 | see also 7583 line | | |
| 7589 | MATR3 | NM_018834.3 | 556 | cccataatttgcagtctatatt | 52422 | 474014 | see also 7583 line | | |
| 7590 | MATR3 | NM_018834.3 | 928 | atggaagtcgttctcaagaatct | 52430 | 474028 | see also 7583 line | | |
| 7591 | MATR3 | NM_018834.3 | 947 | atctgttattgacagaatgg | 52431 | 474030 | see also 7583 line | | |
| 7592 | MATR3 | NM_018834.3 | 1024 | ttgttgagacctcgcataactat | 52432 | 474033 | see also 7583 line | | |
| 7593 | MATR3 | NM_018834.3 | 1025 | tggttgagacctcgcataactatc | 52433 | 474034 | see also 7583 line | | |
| 7594 | MATR3 | NM_018834.3 | 1029 | gagacctcgcataactatcataa | 52434 | 474035 | see also 7583 line | | |
| 7595 | MATR3 | NM_018834.3 | 1031 | gacctcgcataactatcataaat | 52435 | 474036 | see also 7583 line | | |
| 7596 | MATR3 | NM_018834.3 | 1034 | ctcgcataactatcataaattg | 52436 | 474038 | see also 7583 line | | |
| 7597 | MATR3 | NM_018834.3 | 1129 | gcgctcctccaagtagcaatatt | 52438 | 474041 | see also 7583 line | | |
| 7598 | MATR3 | NM_018834.3 | 1130 | cgctcctccaagtagcaatattg | 52439 | 474042 | see also 7583 line | | |
| 7599 | MCFP | NM_018843.1 | 1283 | ctgcttgtgccattatgatcagt | 180794 | 573610 | - | | |
| 7600 | BCAP29 | NM_018844.1 | 402 | gacgtcttgttacgcttattact | 267921 | 650563 | apoptosis | | |
| 7601 | SBBI26 | NM_018846.2 | 638 | aagatgctgaacctgatattatt | 135566 | 534174 | - | | |
| 7602 | SBBI26 | NM_018846.2 | 642 | tgctgaacctgatattattgaac | 135567 | 534175 | see also 7601 line | | |
| 7603 | MKKS | NM_018848.2 | 2542 | ctgtagagacagccaatttgatt | 91647 | 508452 | - | | diabetes |
| 7604 | CACNA1G | NM_018896.3 | 4752 | ccggtgggtccggcacaagtaca | 138816 | 644860 | channel | low voltage-gated calcium channel | |
| 7605 | CACNA1G | NM_018896.3 | 4755 | gtgggtccggcacaagtacaact | 138817 | 644861 | see also 7604 line | | |
| 7606 | CACNA1G | NM_018896.3 | 5822 | aggagtccactgctacaacacg | 138823 | 644872 | see also 7604 line | | |
| 7607 | NANS | NM_018946.2 | 767 | aaggtgttggaagtcacataac | 209008 | 620631 | - | | |
| 7608 | DAZAP1 | NM_018959.2 | 647 | ccgaggtttgatttattact | 50374 | 482716 | - | | |
| 7609 | DAZAP1 | NM_018959.2 | 692 | accaggctgtcaacatgcatttt | 50375 | 482717 | see also 7608 line | | |
| 7610 | C21orf107 | NM_018963.3 | 724 | taggacaggacatagaatcttta | 58358 | 487038 | - | | |
| 7611 | SLC37A1 | NM_018964.2 | 1347 | agcgctgccgattaggtattac | 259315 | 767752 | | sugar porter | |
| 7612 | SLC37A1 | NM_018964.2 | 1348 | gcgcctgccgattaggtattacc | 259316 | 767753 | see also 7611 line | | |
| 7613 | SNTG1 | NM_018967.1 | 1743 | tacagtgcaagaacctatgcatgt | 119056 | 523872 | - | | |
| 7614 | SREB3 | NM_018969.3 | 1173 | ctgctactggcgagtgtttgtga | 241250 | 729855 | gpcr, receptor acitivity, signal transduction | rhodopsin-like receptor | |
| 7615 | GPR85 | NM_018970.3 | 948 | gccatcaccgcttctatacaaa | 240378 | 625261 | - | rhodopsin-like receptor | |
| 7616 | GPR85 | NM_018970.3 | 949 | cccatcaccgcttctatacaaag | 240379 | 625262 | see also 7615 line | | |
| 7617 | GPR85 | NM_018970.3 | 952 | atcaccgcttctatacaaagagg | 240380 | 625263 | see also 7615 line | | |

Figure 46

| | | | | | | | |
|---|---|---|---|---|---|---|---|
| 7618 | GPR85 | NM_018970.3 | 1179 | gtctacctcaagctgatatttt | 240389 | 625269 | see also 7615 line |
| 7619 | GPR85 | NM_018970.3 | 1299 | gccaattggctagcaggatttg | 240393 | 625272 | see also 7615 line |
| 7620 | GPR85 | NM_018970.3 | 1667 | aagggaacctactgtgttatat | 240400 | 625279 | see also 7615 line |
| 7621 | GDAP1 | NM_018972.1 | 122 | aaggtgcgcttggtaattgctga | 373896 | 700815 | - |
| 7622 | GDAP1 | NM_018972.1 | 893 | gtcaacaatattattaatctctgc | 373912 | 700837 | see also 7621 line |
| 7623 | SLC38A2 | NM_018976.3 | 835 | tacctcttcatagtgaaatatga | 11517 | 448505 | - |
| 7624 | NLGN3 | NM_018977.1 | 525 | cccgatcggcgagaaaacgtttc | 373930 | 622305 | autism |
| 7625 | NLGN3 | NM_018977.1 | 526 | ccegatcggcgagaaaacgtttcc | 373931 | 622306 | see also 7624 line |
| 7626 | NLGN3 | NM_018977.1 | 773 | ggcgaggacttagccggataatga | 373934 | 622311 | see also 7624 line |
| 7627 | NLGN3 | NM_018977.1 | 2046 | ttcacatcgggctgaaaccaagg | 373945 | 622322 | see also 7624 line |
| 7628 | NLGN3 | NM_018977.1 | 2136 | tgcatgacatgttccactatacg | 373949 | 622323 | see also 7624 line |
| 7629 | NLGN3 | NM_018977.1 | 2724 | ttgccgcaggttcaacagtacc | 373955 | 622334 | see also 7624 line |
| 7630 | PRKWNK1 | NM_018979.1 | 1656 | gaccatgctaaagctatcaaag | 97694 | 814811 | kinase_related |
| 7631 | PRKWNK1 | NM_018979.1 | 3388 | gaccgagtagtagaatgtcaatt | 97722 | 814852 | see also 7630 line |
| 7632 | PRKWNK1 | NM_018979.1 | 3389 | accgagtagtagaatgtcaatta | 97723 | 814853 | see also 7630 line |
| 7633 | PRKWNK1 | NM_018979.1 | 3480 | aacaattattggtgaacaatgact | 97728 | 814857 | see also 7630 line |
| 7634 | PRKWNK1 | NM_018979.1 | 5831 | gacgtttcaggtgacaactaca | 97788 | 814936 | see also 7630 line |
| 7635 | PRKWNK1 | NM_018979.1 | 6134 | atctaagtcaaagccttagtaat | 97800 | 814946 | see also 7630 line |
| 7636 | ERdj5 | NM_018981.1 | 604 | tcctgataaaaacccggataaacc | 264679 | 647652 | - |
| 7637 | ERdj5 | NM_018981.1 | 614 | aacccgaataaccaaaatgcaca | 264680 | 647653 | see also 7636 line |
| 7638 | ERdj5 | NM_018981.1 | 687 | atctacggaaaaagtatgacaaa | 264682 | 647656 | see also 7636 line |
| 7639 | ERdj5 | NM_018981.1 | 690 | tacggaaaaagtatgacaaatat | 264683 | 647657 | see also 7636 line |
| 7640 | ERdj5 | NM_018981.1 | 2260 | ttgccaacagtatcattcttttt | 264745 | 647697 | see also 7636 line |
| 7641 | DJ1167A19.1 | NM_018982.3 | 1078 | gccagcgttcgtgaggataacc | 374006 | 757251 | - |
| 7642 | SEMA5B | NM_018987.1 | 837 | gggaacctcagccgactattga | 251401 | 637656 | - |
| 7643 | SEMA5B | NM_018987.1 | 845 | cagccgacttattgagaagatca | 251402 | 637658 | see also 7642 line |
| 7644 | RIN2 | NM_018993.2 | 754 | ctgcgtcagctctgccttataaa | 295222 | 735665 | - |
| 7645 | RIN2 | NM_018993.2 | 970 | ccgccaccgctattaatagtct | 295224 | 735669 | see also 7644 line |
| 7646 | RIN2 | NM_018993.2 | 972 | gccacccgctattaatagtctcc | 295225 | 735670 | see also 7644 line |
| 7647 | RIN2 | NM_018993.2 | 1029 | gacgagcatgccagaaaacagtca | 295226 | 735671 | see also 7644 line |
| 7648 | RIN2 | NM_018993.2 | 1717 | atgacccaggtcaagaactattt | 295232 | 735677 | see also 7644 line |

345

Figure 46

| | | | | | | | |
|---|---|---|---|---|---|---|---|
| 7649 | RIN2 | NM_018993.2 | 1719 | gaccccaggtcaagaactatttgt | 295233 | 735678 | see also 7644 line |
| 7650 | RIN2 | NM_018993.2 | 2132 | tgccagtcctgacctatgtcata | 295236 | 735687 | see also 7644 line |
| 7651 | FBXW5 | NM_018998.2 | 1636 | ctgggaccgccactacaacatct | 374103 | 705687 | - |
| 7652 | FLJ20152 | NM_019000.2 | 650 | cagtcttgtcctaagattagc | 374111 | 716564 | - |
| 7653 | FLJ20152 | NM_019000.2 | 677 | cggttgctgccaaagttatct | 374114 | 716566 | see also 7652 line |
| 7654 | FLJ20152 | NM_019000.2 | 681 | tgctgccaaagagttatctgt | 374115 | 716567 | see also 7652 line |
| 7655 | ETAA16 | NM_019002.2 | 614 | ttggtgaaactgctattccttgt | 374290 | 725942 | - |
| 7656 | FLJ20323 | NM_019005.1 | 432 | atggtcgagttgtacttacaagc | 374362 | 755811 | - |
| 7657 | FLJ20323 | NM_019005.1 | 453 | gccttggtcaagatcataactca | 374364 | 755813 | see also 7656 line |
| 7658 | FLJ20323 | NM_019005.1 | 541 | ctggaatccactggatgtaact | 374369 | 755819 | see also 7656 line |
| 7659 | FLJ20323 | NM_019005.1 | 625 | cagcaaatatactcctgatatag | 374376 | 755822 | see also 7656 line |
| 7660 | FLJ20323 | NM_019005.1 | 1832 | aggagatctgaatctcaatgtgg | 374422 | 755834 | see also 7656 line |
| 7661 | PEPP2 | NM_019012.2 | 2409 | tccagattatagactctacaaga | 172704 | 755048 | - |
| 7662 | PEPP2 | NM_019012.2 | 2411 | cagattatagactctacaagagt | 172705 | 755049 | see also 7661 line |
| 7663 | FLJ20793 | NM_019022.3 | 1302 | cagtatcattgtgctatggaatct | 137581 | 812616 | - |
| 7664 | FLJ10640 | NM_019023.1 | 1215 | ctggatgcagtgtgtgactcc | 214193 | 604403 | S-adenosylmethionine-dependent methyltransferase |
| 7665 | SMOX | NM_019025.2 | 575 | atggcgtggctgctaccttacc | 225896 | 612664 | - |
| 7666 | SMOX | NM_019025.2 | 632 | aggaattcagcggattattacaac | 225897 | 612666 | see also 7665 line |
| 7667 | HIP14L | NM_019028.1 | 686 | gggccagaaccaactggatttct | 374538 | 731956 | kinase_related |
| 7668 | HIP14L | NM_019028.1 | 861 | ctcttgatatggctctacaaaac | 374545 | 731963 | see also 7667 line |
| 7669 | PCDH18 | NM_019035.2 | 2896 | atgcttcaccaggggcaatatca | 149850 | 551281 | - |
| 7670 | DDX4 | NM_019039.1 | 637 | aacgaggtggttacaaagttta | 59141 | 483078 | - |
| 7671 | DDX4 | NM_019039.1 | 638 | acgaggtggttacaaaggtttaa | 59142 | 483079 | see also 7670 line |
| 7672 | DDX4 | NM_019039.1 | 640 | gaggtggttacaaaggtttaaat | 59143 | 483080 | see also 7670 line |
| 7673 | ELP4 | NM_019040.2 | 1019 | aaccatgtataaggattatca | 374610 | 714656 | - |
| 7674 | ELP4 | NM_019040.2 | 1021 | cccattgtataggattatcatg | 374611 | 714657 | see also 7673 line |
| 7675 | ELP4 | NM_019040.2 | 1065 | ttcctcggcttaataacttgatc | 374615 | 714659 | see also 7673 line |
| 7676 | APBB1IP | NM_019043.2 | 1343 | atgggaagactctctatgataac | 314871 | 679185 | signal_transduction |
| 7677 | SEC15L1 | NM_019053.2 | 1002 | cagttgatggctatagaagatat | 269943 | 650055 | - |
| 7678 | SEC15L1 | NM_019053.2 | 1018 | aagatatttcactcaaattgtag | 269945 | 650057 | see also 7677 line |
| 7679 | SEC15L1 | NM_019053.2 | 1118 | aacatggccctctcaaagataat | 269948 | 650060 | see also 7677 line |
| 7680 | SEC15L1 | NM_019053.2 | 1121 | atggccctctcaaagataattgc | 269949 | 650061 | see also 7677 line |
| 7681 | SEC15L1 | NM_019053.2 | 1715 | gaggactttataactaacattac | 269969 | 650078 | see also 7677 line |
| 7682 | SEC15L1 | NM_019053.2 | 2039 | atgctactgccagtgagttaaa | 269972 | 650087 | see also 7677 line |

Figure 46

| | | | | | | | | | |
|---|---|---|---|---|---|---|---|---|---|
| 7683 | SEC15L1 | NM_019053.2 | 2193 | tccttgacctgtttatggtttgg | 269977 | 650093 | see also 7677 line | | |
| 7684 | ROBO4 | NM_019055.4 | 1565 | tgggcccaggtctgtacagatat | 374788 | 736108 | receptor_acitivity | - | - |
| 7685 | PIP3AP | NM_019061.2 | 1569 | ttgtcagatagcctgtatatacc | 374834 | 781673 | - | - | - |
| 7686 | PIP3AP | NM_019061.2 | 1618 | cacctcatcaaaaagatactaac | 374836 | 781676 | see also 7685 line | | |
| 7687 | ING3 | NM_019071.2 | 231 | cagaatgcaatggatcaactaga | 28855 | 460590 | transcription_regulator | - | - |
| 7688 | ING3 | NM_019071.2 | 492 | gagaggcgatctttggaattaga | 28865 | 460602 | see also 7687 line | | |
| 7689 | ING3 | NM_019071.2 | 494 | gaggcgatctttggaattagaca | 28866 | 460603 | see also 7687 line | | |
| 7690 | ING3 | NM_019071.2 | 1190 | ttggacttacgacccaaatgaac | 28884 | 460628 | see also 7687 line | | |
| 7691 | ING3 | NM_019071.2 | 1191 | tggacttacgacccaaatgaacc | 28885 | 460629 | see also 7687 line | | |
| 7692 | ING3 | NM_019071.2 | 1251 | atggtgggatgtgataaccaaga | 28889 | 460632 | see also 7687 line | | |
| 7693 | SGTB | NM_019072.1 | 813 | ttgacatggctagcttgataaat | 374917 | 754195 | - | - | - |
| 7694 | SPATA6 | NM_019073.1 | 528 | ttcaaaccgccaggttaccatga | 374933 | 725279 | - | - | - |
| 7695 | SPATA6 | NM_019073.1 | 539 | aggttaccatgaggaggatttct | 374934 | 725280 | see also 7694 line | | |
| 7696 | SPATA6 | NM_019073.1 | 1297 | gagatccatgaccgggtaaaaaa | 374962 | 725310 | see also 7694 line | | |
| 7697 | SPATA6 | NM_019073.1 | 1564 | gacaagatgtacaggaacttata | 374966 | 725317 | see also 7694 line | | |
| 7698 | FLJ20051 | NM_019087.1 | 118 | ggcgtttctgtacatggattatc | 375116 | 776172 | - | - | - |
| 7699 | FLJ20051 | NM_019087.1 | 275 | gtgtcgaccacaggttttagtat | 375120 | 776176 | see also 7698 line | | |
| 7700 | FLJ20051 | NM_019087.1 | 538 | agctcgctcagtacaagagatca | 375130 | 776187 | see also 7698 line | | |
| 7701 | FLJ20051 | NM_019087.1 | 541 | tcgctcagtacaagagatcaaaa | 375131 | 776188 | see also 7698 line | | |
| 7702 | PD2 | NM_019088.1 | 320 | gccgagtcaagtactgcaatagc | 375134 | 708190 | - | - | - |
| 7703 | C20orf155 | NM_019095.3 | 713 | tgctaggttaaaaccaacattca | 218398 | 671407 | - | - | - |
| 7704 | CNGB3 | NM_019098.2 | 1243 | ttgggcagttcgaactttaatta | 247944 | 520793 | - | - | - |
| 7705 | CNGB3 | NM_019098.2 | 1244 | tgggcagttcgaactttaattac | 247945 | 520794 | see also 7704 line | | |
| 7706 | CNGB3 | NM_019098.2 | 1245 | gggcagttcgaactttaattacc | 247946 | 520795 | see also 7704 line | | |
| 7707 | APOM | NM_019101.2 | 497 | cagcgctttctcctctacaatcg | 271910 | 654558 | - | lipid transporter | - |
| 7708 | HOXA5 | NM_019102.2 | 108 | gcccggactaccagttgcataat | 5068 | 444714 | transcription_regulator | transcription factor | - |
| 7709 | HOXA5 | NM_019102.2 | 109 | cccggactaccagttgcataatt | 5069 | 444715 | see also 7708 line | | |
| 7710 | TNXB | NM_019105.4 | 13010 | cggtgcccttcacggaaatgaag | 42442 | 570944 | - | - | - |
| 7711 | KLHL4 | NM_019117.3 | 1051 | tcctgcttccagctaatgaaatt | 117847 | 532899 | - | - | - |
| 7712 | KLHL4 | NM_019117.3 | 1095 | gacattaatgtgcctgatgaaga | 117848 | 532903 | see also 7711 line | | |
| 7713 | LOC56181 | NM_019557.3 | 756 | atgcagcttcggcttcattaacg | 375314 | 740453 | - | - | - |
| 7714 | LOC56181 | NM_019557.3 | 1018 | cagcagctttgcagacatgatgg | 375320 | 740457 | see also 7713 line | | |
| 7715 | DKFZP761 L0424 | NM_019590.1 | 574 | tacagaaatgagggtttctatgc | 375329 | 800485 | - | - | - |

Figure 46

| | | | | | | | | | |
|---|---|---|---|---|---|---|---|---|---|
| 7716 | DKFZP761L0424 | NM_019590.1 | 999 | aggattatccagccttgtagacc | 375347 | 800502 | see also 7715 line | | |
| 7717 | RNF20 | NM_019592.5 | 406 | atcatccttaaacgttatgatct | 375382 | 807729 | - | - | - |
| 7718 | RNF20 | NM_019592.5 | 910 | ctgcagtgggatattgacaaaat | 375390 | 807736 | see also 7717 line | | |
| 7719 | RNF20 | NM_019592.5 | 2402 | atcagatccataagttgcttaaa | 375422 | 807761 | see also 7717 line | | |
| 7720 | RNF20 | NM_019592.5 | 2859 | gtgctgtaacatgcgtaaaaagg | 375426 | 807768 | see also 7717 line | | |
| 7721 | KIAA1434 | NM_019593.2 | 2193 | gtggatgccaacggcattgataa | 198901 | 590920 | - | - | - |
| 7722 | KIAA1434 | NM_019593.2 | 2194 | tggatgccaacggcattgataac | 198902 | 590921 | see also 7721 line | | |
| 7723 | LOC56270 | NM_019613.2 | 812 | ttcatcagggcatttaatccagg | 375526 | 718920 | - | - | - |
| 7724 | PARD3 | NM_019619.2 | 113 | tcgccaaggatccaaactactgg | 308266 | 538486 | cell_cycle、kinase_related | - | - |
| 7725 | PARD3 | NM_019619.2 | 2514 | tgccagtcaattggatttcgtta | 308326 | 538537 | see also 7724 line | | |
| 7726 | PARD3 | NM_019619.2 | 2515 | gccagtcaattggatttcgttaa | 308327 | 538538 | see also 7724 line | | |
| 7727 | PARD3 | NM_019619.2 | 2517 | cagtcaattggatttcgttaaaa | 308328 | 538539 | see also 7724 line | | |
| 7728 | PARD3 | NM_019619.2 | 2530 | ttcgttaaaacacgaaaatcaaa | 308330 | 538540 | see also 7724 line | | |
| 7729 | PARD3 | NM_019619.2 | 2571 | agctgacgagactaaactcaata | 308334 | 538542 | see also 7724 line | | |
| 7730 | PARD3 | NM_019619.2 | 2573 | ctgacgagactaaactcaataca | 308335 | 538543 | see also 7724 line | | |
| 7731 | PARD3 | NM_019619.2 | 2575 | gacgagactaaactcaatacagt | 308336 | 538544 | see also 7724 line | | |
| 7732 | ABCB9 | NM_019624.1 | 1240 | cacgggcgtggtggtcttcatgt | 69952 | 492021 | - | - | - |
| 7733 | EIF4ENIF1 | NM_019843.2 | 1003 | atgcttggcttcgatgatagaag | 269024 | 476720 | - | protein transporter | - |
| 7734 | EIF4ENIF1 | NM_019843.2 | 1004 | tgcttggcttcgatgatagaaga | 269025 | 476721 | see also 7733 line | | |
| 7735 | EIF4ENIF1 | NM_019843.2 | 1007 | ttggcttcgatgatagaagatgt | 269026 | 476722 | see also 7733 line | | |
| 7736 | EIF4ENIF1 | NM_019843.2 | 1009 | ggcttcgatgatagaagatgttt | 269027 | 476723 | see also 7733 line | | |
| 7737 | EIF4ENIF1 | NM_019843.2 | 1177 | tggacagaactcggggaattact | 269028 | 476726 | see also 7733 line | | |
| 7738 | METTL3 | NM_019852.2 | 1134 | agccaggagcttgctcttacaca | 52520 | 474143 | - | - | - |
| 7739 | METTL3 | NM_019852.2 | 1603 | aggttcgttccaccagtcataaa | 52524 | 474156 | see also 7738 line | | |
| 7740 | CTPS2 | NM_019857.3 | 615 | gtccaggagtgggttatgaatca | 232111 | 617034 | - | - | Alzheimer disease |
| 7741 | CTPS2 | NM_019857.3 | 1191 | tgctacgcctctgtgttcaaagc | 232130 | 617049 | see also 7740 line | | |
| 7742 | GRCA | NM_019858.1 | 1354 | cagcgccatcgtctttctctatg | 240437 | 625334 | gpcr、receptor_acitivity、signal_transduction | - | - |
| 7743 | GRCA | NM_019858.1 | 1355 | agcgccatcgtctttctctatga | 240438 | 625335 | see also 7742 line | | |
| 7744 | CHST7 | NM_019886.2 | 543 | agcacccggacgttttctacttg | 217395 | 605599 | - | - | - |
| 7745 | CHST7 | NM_019886.2 | 546 | acccggacgttttctacttgtat | 217396 | 605600 | see also 7744 line | | |
| 7746 | CHST7 | NM_019886.2 | 547 | cccggacgttttctacttgtatg | 217397 | 605601 | see also 7744 line | | |
| 7747 | ERO1LB | NM_019891.2 | 402 | gagactattttcgttattacaag | 226687 | 722219 | - | - | - |
| 7748 | ERO1LB | NM_019891.2 | 824 | ttcaagcctcgatctgtttatcg | 226706 | 722229 | see also 7747 line | | |

Figure 46

| | | | | | | | | | |
|---|---|---|---|---|---|---|---|---|---|
| 7749 | ERO1LB | NM_019891.2 | 827 | aagcctcgatctgtttatcgtcc | 226707 | 722230 | see also 7747 line | | |
| 7750 | ERO1LB | NM_019891.2 | 835 | atctgtttatcgtcctttaaatc | 226708 | 722231 | see also 7747 line | | |
| 7751 | ASAH2 | NM_019893.1 | 45 | cagtgggaccattgaaaaccaca | 303836 | 593825 | signal_transduction | - | - |
| 7752 | C3orf4 | NM_019895.1 | 1590 | ctcacaccaaccggaaagagtac | 326213 | 691313 | - | - | - |
| 7753 | C3orf4 | NM_019895.1 | 1599 | accggaaagagtacaccttaatg | 326214 | 691314 | see also 7752 line | | |
| 7754 | C3orf4 | NM_019895.1 | 1600 | ccggaaagagtacaccttaatga | 326215 | 691315 | see also 7752 line | | |
| 7755 | KCMF1 | NM_020122.2 | 434 | agccctagagatttagatgaat | 375588 | 709050 | channel | - | - |
| 7756 | KCMF1 | NM_020122.2 | 456 | tcgagtggtgttcgacatgtacg | 375589 | 709053 | see also 7755 line | | |
| 7757 | KCMF1 | NM_020122.2 | 466 | ttcgacatgtacgtagaatgttt | 375590 | 709054 | see also 7755 line | | |
| 7758 | SMBP | NM_020123.2 | 305 | gagcacgaacacacgtatcaaga | 274828 | 655211 | - | - | - |
| 7759 | SMBP | NM_020123.2 | 309 | acgaacacacgtatcaagataaa | 274829 | 655213 | see also 7758 line | | |
| 7760 | SMBP | NM_020123.2 | 417 | tggggtcaaaaaaaagtatcagt | 274838 | 655216 | see also 7758 line | | |
| 7761 | SMBP | NM_020123.2 | 715 | tggaaatcgaattgttgatgtta | 274848 | 655234 | see also 7758 line | | |
| 7762 | SMBP | NM_020123.2 | 1404 | tggccgtttgttgcatctgtttt | 274858 | 655256 | see also 7758 line | | |
| 7763 | SMBP | NM_020123.2 | 1594 | ttcaatctttattgaaatgtatt | 274866 | 655267 | see also 7758 line | | |
| 7764 | EB-1 | NM_020140.1 | 472 | ttgttggaaattggaatccttaa | 375642 | 805544 | - | - | - |
| 7765 | LOC56902 | NM_020143.2 | 371 | gggacttcagatacgctttaact | 63672 | 478404 | - | - | - |
| 7766 | SH3GLB2 | NM_020145.2 | 723 | caggagactagacctcgtaatta | 375680 | 752972 | - | - | - |
| 7767 | SH3GLB2 | NM_020145.2 | 724 | aggagactagacctcgtaattac | 375681 | 752973 | see also 7766 line | | |
| 7768 | SH3GLB2 | NM_020145.2 | 728 | gactagacctcgtaattacattc | 375682 | 752974 | see also 7766 line | | |
| 7769 | STARD7 | NM_020151.2 | 879 | accagtaccgagtttttggaacc | 375705 | 753039 | - | - | - |
| 7770 | STARD7 | NM_020151.2 | 1482 | ctgctcggattgagtatgcttga | 375724 | 753054 | see also 7769 line | | |
| 7771 | FLJ21827 | NM_020153.2 | 1134 | caggcccatgcccgacattgaca | 280327 | 714495 | - | - | - |
| 7772 | LOC56851 | NM_020154.1 | 367 | gagagcaagatatgtgaattaca | 111317 | 747840 | - | - | - |
| 7773 | DHX33 | NM_020162.2 | 1037 | gggctatcgcaaagtgatcattt | 59771 | 483565 | - | - | - |
| 7774 | DHX33 | NM_020162.2 | 1038 | ggctatcgcaaagtgatcatttc | 59772 | 483566 | see also 7773 line | | |
| 7775 | DHX33 | NM_020162.2 | 1042 | atcgcaaagtgatcatttcaacc | 59773 | 483567 | see also 7773 line | | |
| 7776 | DHX33 | NM_020162.2 | 1072 | ctgaaacctccataaccattaca | 59774 | 483568 | see also 7773 line | | |
| 7777 | DHX33 | NM_020162.2 | 1122 | atggttaaagcaaagaagtataa | 59775 | 483573 | see also 7773 line | | |
| 7778 | MCCC1 | NM_020166.2 | 279 | taccaaggtcctcattgcaaaca | 90434 | 508077 | - | methylcrotonyl-CoA carboxylase | methylcrotonylglycinuria |
| 7779 | MCCC1 | NM_020166.2 | 995 | aggccccagcgcctggtattaaa | 90452 | 508097 | see also 7778 line | | |
| 7780 | MCCC1 | NM_020166.2 | 996 | ggccccagcgcctggtattaaat | 90453 | 508098 | see also 7778 line | | |
| 7781 | FEM1C | NM_020177.2 | 885 | ctgtcaacaacacgactttaacc | 375761 | 783609 | - | - | - |
| 7782 | FEM1C | NM_020177.2 | 1138 | tggaagtttggacatcatgaaga | 375773 | 783618 | see also 7781 line | | |
| 7783 | FEM1C | NM_020177.2 | 1280 | agcaagacagaacgtattaatgc | 375778 | 783623 | see also 7781 line | | |
| 7784 | FEM1C | NM_020177.2 | 1827 | tagagcgagctatcaaacaaact | 375802 | 783653 | see also 7781 line | | |

Figure 46

| 7785 | FEM1C | NM_020177.2 | 1829 | gagcgagctatcaaacaaactca | 375803 | 783654 | see also 7781 line | | |
|---|---|---|---|---|---|---|---|---|---|
| 7786 | FEM1C | NM_020177.2 | 2028 | ctgtggacaagaatactacatgt | 375813 | 783660 | see also 7781 line | | |
| 7787 | FEM1C | NM_020177.2 | 2030 | gtggacaagaatactacatgtgt | 375814 | 783661 | see also 7781 line | | |
| 7788 | BRUNOL4 | NM_020180.1 | 735 | tggccagatgggcatgttcaacc | 49810 | 471162 | - | - | - |
| 7789 | CNNM4 | NM_020184.1 | 845 | gccaggagattcgcactgtttac | 375844 | 744478 | - | - | - |
| 7790 | DUSP22 | NM_020185.3 | 732 | tggtgatcgcatacatcatgacc | 195866 | 534955 | kinase_related、phosphatase、apoptosis | - | - |
| 7791 | MRPS22 | NM_020191.2 | 366 | gaggcagctaaagtacgattaaa | 282299 | 658747 | - | structural constituent of ribosome | - |
| 7792 | MRPS22 | NM_020191.2 | 367 | aggcagctaaagtacgattaaaa | 282300 | 658748 | see also 7791 line | | |
| 7793 | MRPS22 | NM_020191.2 | 368 | ggcagctaaagtacgattaaaaa | 282301 | 658749 | see also 7791 line | | |
| 7794 | C11orf30 | NM_020193.2 | 214 | aacgatgaacggttaacaacaat | 375949 | 772286 | - | - | - |
| 7795 | C11orf30 | NM_020193.2 | 819 | aacaacacagaaggttattatag | 375963 | 772294 | see also 7794 line | | |
| 7796 | C11orf30 | NM_020193.2 | 1427 | ctctacccaccagtagcaattcc | 375980 | 772305 | see also 7794 line | | |
| 7797 | C11orf30 | NM_020193.2 | 1456 | atggtggttagcagtaatggtgc | 375983 | 772307 | see also 7794 line | | |
| 7798 | C11orf30 | NM_020193.2 | 1472 | atggtgcaattatgacaactaaa | 375984 | 772309 | see also 7794 line | | |
| 7799 | C11orf30 | NM_020193.2 | 1699 | atcactacaatcccaatgacttc | 375989 | 772325 | see also 7794 line | | |
| 7800 | GL004 | NM_020194.4 | 349 | cagaaattagtcgaattcagtac | 376039 | 738905 | - | - | - |
| 7801 | GL004 | NM_020194.4 | 941 | tggcattttgtcgcttatccagt | 376055 | 738919 | see also 7800 line | | |
| 7802 | RNPEP | NM_020216.3 | 706 | tcccctcctatctgatagctttg | 159827 | 559531 | - | zinc binding | - |
| 7803 | RNPEP | NM_020216.3 | 900 | atgccaccgtcctttccatttgg | 159831 | 559538 | see also 7802 line | | |
| 7804 | RNPEP | NM_020216.3 | 1446 | atcattccaggttttgagtttga | 159845 | 559549 | see also 7802 line | | |
| 7805 | RNPEP | NM_020216.3 | 1517 | ccctggggactcactcatgaagc | 159846 | 559550 | see also 7802 line | | |
| 7806 | PRDM8 | NM_020226.1 | 1713 | cagccaacgtttccagtttgagt | 376266 | 741090 | - | - | - |
| 7807 | PRDM8 | NM_020226.1 | 1715 | gccaacgtttccagtttgagttc | 376267 | 741091 | see also 7806 line | | |
| 7808 | MDS010 | NM_020231.3 | 210 | aaccatgttcaagtcaaaactgc | 376310 | 772580 | - | - | - |
| 7809 | MDS010 | NM_020231.3 | 509 | gtcttctccttcagtaagacatc | 376321 | 772590 | see also 7808 line | | |
| 7810 | BBX | NM_020235.2 | 639 | aaccgaggtgctaccaagatact | 22512 | 456064 | - | - | - |
| 7811 | BBX | NM_020235.2 | 1553 | ttctgccagtagcaagataataa | 22525 | 456079 | see also 7810 line | | |
| 7812 | BBX | NM_020235.2 | 1556 | tgccagtagcaagataataatta | 22526 | 456081 | see also 7810 line | | |
| 7813 | BBX | NM_020235.2 | 2255 | aggaaactcctctgatcatgaag | 22544 | 456096 | see also 7810 line | | |
| 7814 | BBX | NM_020235.2 | 2774 | tactttggatgacaaaccaaagg | 22555 | 456110 | see also 7810 line | | |
| 7815 | BBX | NM_020235.2 | 3017 | gccgcaggctcctgtacttattt | 22557 | 456112 | see also 7810 line | | |
| 7816 | BBX | NM_020235.2 | 3019 | cgcaggctcctgtacttatttcc | 22558 | 456114 | see also 7810 line | | |
| 7817 | KNSL7 | NM_020242.1 | 3564 | aagatggaagagcctctaagact | 30102 | 506346 | - | - | - |
| 7818 | CHPT1 | NM_020244.1 | 800 | aacaatgtgggactatacgattc | 209533 | 671434 | - | - | - |
| 7819 | CHPT1 | NM_020244.1 | 1073 | tggatgtgtctttgctaaagtct | 209543 | 671451 | see also 7818 line | | |

Figure 46

| 7820 | TULP4 | NM_020245.2 | 2317 | gagtgccatggtcaagttctaca | 13787 | 682180 | transcription_regulator | transcription factor | - |
|---|---|---|---|---|---|---|---|---|---|
| 7821 | TULP4 | NM_020245.2 | 3211 | aaccaacctcggtgcagtaatct | 13799 | 682204 | see also 7820 line | | |
| 7822 | TULP4 | NM_020245.2 | 3215 | aacctcggtgcagtaatctataa | 13800 | 682205 | see also 7820 line | | |
| 7823 | TULP4 | NM_020245.2 | 3216 | acctcggtgcagtaatctataaa | 13801 | 682206 | see also 7820 line | | |
| 7824 | TULP4 | NM_020245.2 | 5879 | atgcagtttggacggattgatgg | 13818 | 682236 | see also 7820 line | | |
| 7825 | CCNL1 | NM_020307.1 | 512 | tccaccacctccgccagttaaga | 23760 | 674728 | - | - | - |
| 7826 | CCNL1 | NM_020307.1 | 514 | caccacctccgccagttaagagg | 23761 | 674729 | see also 7825 line | | |
| 7827 | CCNL1 | NM_020307.1 | 630 | gggattttgtgttcatgtcaagc | 23766 | 674735 | see also 7825 line | | |
| 7828 | CCNL1 | NM_020307.1 | 1174 | agcccttacaatggtgtaagaaa | 23777 | 674749 | see also 7825 line | | |
| 7829 | CCNL1 | NM_020307.1 | 1359 | aagccctcgaagacatcataatc | 23779 | 674753 | see also 7825 line | | |
| 7830 | CCNL1 | NM_020307.1 | 1553 | aacgatctcgctcctttgagagg | 23780 | 674757 | see also 7825 line | | |
| 7831 | CMKOR1 | NM_020311.1 | 1145 | ttcaagtactcggccaaaacagg | 239124 | 624012 | gpcr、receptor_acitivity、signal_transduction | - | - |
| 7832 | DKFZP564O043 | NM_020319.1 | 608 | tcctcgagagagactggattatt | 376516 | 759551 | - | - | - |
| 7833 | DKFZP564O043 | NM_020319.1 | 617 | gagactggattattacactaagc | 376517 | 759555 | see also 7832 line | | |
| 7834 | RAI17 | NM_020338.1 | 1040 | aggcgtataacagccaattcatg | 159506 | 809155 | - | - | - |
| 7835 | RAI17 | NM_020338.1 | 1041 | ggcgtataacagccaattcatga | 159507 | 809156 | see also 7834 line | | |
| 7836 | PAK7 | NM_020341.2 | 2151 | gacaagcgatggccggataaagt | 95565 | 511171 | kinase_related | - | - |
| 7837 | PAK7 | NM_020341.2 | 2154 | aagcgatggccggataaagttgt | 95566 | 511172 | see also 7836 line | | |
| 7838 | PAK7 | NM_020341.2 | 2379 | gcggaggatccgggacagtttac | 95576 | 511177 | see also 7836 line | | |
| 7839 | PAK7 | NM_020341.2 | 2380 | cggaggatccgggacagtttacc | 95577 | 511178 | see also 7836 line | | |
| 7840 | PAK7 | NM_020341.2 | 2406 | aagagtgaaggacctacacaagg | 95578 | 511180 | see also 7836 line | | |
| 7841 | PAK7 | NM_020341.2 | 2414 | aggacctacacaaggtttcttca | 95579 | 511181 | see also 7836 line | | |
| 7842 | SLC24A2 | NM_020344.1 | 1144 | ttggatcatatggaaaactaaaa | 257800 | 693107 | - | symporter | - |
| 7843 | PLSCR4 | NM_020353.1 | 307 | aggcttgcctatgggatactaca | 151342 | 803285 | - | phospholipid scramblase | - |
| 7844 | LALP1 | NM_020354.1 | 920 | gggaggagcctctctccaaattg | 376839 | 585973 | - | - | - |
| 7845 | LALP1 | NM_020354.1 | 1051 | acgtgtacagggtttatgtcaca | 376841 | 585974 | see also 7844 line | | |
| 7846 | LALP1 | NM_020354.1 | 1463 | tgccaaggctgctcaggattact | 376852 | 585982 | see also 7844 line | | |
| 7847 | HT014 | NM_020362.2 | 307 | cggaccgaccgctccaagtttgt | 376893 | 716210 | - | - | - |
| 7848 | HT014 | NM_020362.2 | 310 | accgaccgctccaagtttgttga | 376894 | 716212 | see also 7847 line | | |
| 7849 | RPGRIP1 | NM_020366.2 | 791 | cagagcttcgagcttccattaaa | 376913 | 540009 | - | - | - |
| 7850 | C12orf6 | NM_020367.2 | 319 | tagcagtgaagatatcgaaaaaa | 376982 | 805719 | - | - | - |
| 7851 | C12orf6 | NM_020367.2 | 382 | ttccaaattcagctacaagatag | 376984 | 805721 | see also 7850 line | | |

Figure 46

| | | | | | | | | |
|---|---|---|---|---|---|---|---|---|
| 7852 | C12orf6 | NM_020367.2 | 1044 | aagacgggagctatgtgaattta | 377009 | 805744 | | see also 7850 line |
| 7853 | FSCN3 | NM_020369.1 | 1517 | ggggatccttcggtcaataaca | 114134 | 523031 | actin cross-linking | - |
| 7854 | C12orf4 | NM_020374.2 | 1077 | tactagtagataatcgaattaat | 377121 | 751857 | | - |
| 7855 | C12orf4 | NM_020374.2 | 1080 | tagtagataatcgaattaattca | 377122 | 751858 | | see also 7854 line |
| 7856 | CYSLTR2 | NM_020377.2 | 275 | ttccctcaggcgtgactattat | 238937 | 623062 | leukotriene receptor | receptor_acitivity, signal transduction, gper... |
| 7857 | CYSLTR2 | NM_020377.2 | 277 | cccttcaggcgtgactattatct | 238938 | 623064 | | see also 7856 line |
| 7858 | CYSLTR2 | NM_020377.2 | 469 | tggatcctctgtgggatcatatg | 238947 | 623070 | | see also 7856 line |
| 7859 | XPNPEP1 | NM_020383.2 | 831 | gagacgatcatgcttcattga | 186679 | 580496 | | - |
| 7860 | XPNPEP1 | NM_020383.2 | 833 | gacgatcatgctcttcattgatg | 186680 | 580497 | | see also 7859 line |
| 7861 | TRPC7 | NM_020389.1 | 994 | ttgcttaccatgtggtatgaaaa | 252170 | 645608 | calcium channel | receptor_acitivity, cha... |
| 7862 | EIF5A2 | NM_020390.5 | 132 | tggcagacgaaattgatttcact | 56830 | 476778 | | - |
| 7863 | EIF5A2 | NM_020390.5 | 362 | aacatggatgttccaaatattaa | 56840 | 476785 | | see also 7862 line |
| 7864 | GOPC | NM_020399.1 | 1287 | ctggtgctagttgcaaagacaca | 316129 | 535701 | | - |
| 7865 | PLXDC1 | NM_020405.3 | 525 | tgccggcagatcaccatagcaact | 250548 | 636819 | | receptor_acitivity |
| 7866 | CGI-152 | NM_020410.1 | 1450 | ctgaccaaagatgagaaagtatt | 76819 | 498119 | plasma membrane cation-transporting ATPase | |
| 7867 | DDX24 | NM_020414.3 | 1649 | ctgtctgaatccttcataagaag | 50732 | 473533 | ATP dependent RNA helicase | |
| 7868 | PPP2R2C | NM_020416.2 | 66 | cacggacacgcggaaaattaacc | 298721 | 666280 | | phosphatase, signal_transduction |
| 7869 | PPP2R2C | NM_020416.2 | 902 | ccgtgtccgacgtgaagttcagc | 298737 | 666284 | | see also 7868 line |
| 7870 | PACE-1 | NM_020423.4 | 1400 | gaggagaacgaaccaagatcttc | 95427 | 488149 | kinase_related | - |
| 7871 | SMURF1 | NM_020429.1 | 2103 | aggtttatgagaggaatcgaagc | 230798 | 615146 | signal_transduction | |
| 7872 | PHTF2 | NM_020432.2 | 522 | ttgctgcaatagtattattctgc | 265697 | 782140 | | - |
| 7873 | PHTF2 | NM_020432.2 | 1220 | tgcagctctcgctttcaagttc | 265711 | 782153 | | see also 7872 line |
| 7874 | PHTF2 | NM_020432.2 | 1435 | tagttcccaccccaggattagaaa | 265713 | 782160 | | see also 7872 line |
| 7875 | SALL4 | NM_020436.2 | 559 | ccccccaggacataagctatttag | 38882 | 649639 | transcription_regulator | - |
| 7876 | DOLPP1 | NM_020438.2 | 79 | gaccctcaccacgtcgaatatc | 377463 | 710341 | | - |
| 7877 | NAV1 | NM_020443.2 | 638 | cacggactccgagaaaaagctgc | 94162 | 520914 | | - |
| 7878 | NAV1 | NM_020443.2 | 4864 | gtcgaggtgtcaataacatatca | 94206 | 520959 | | see also 7877 line |
| 7879 | KIAA1387 | NM_020463.1 | 794 | acctatccgtaggggaaaagctgg | 377690 | 750287 | | - |
| 7880 | KIAA1387 | NM_020463.1 | 1466 | cagatcagctgctacagatatat | 377702 | 750302 | | see also 7879 line |
| 7881 | KIAA1387 | NM_020463.1 | 1642 | tgggacttccttcgtgactctaatt | 377707 | 750306 | | see also 7879 line |

Figure 46

| ID | Gene | Accession | Pos | Sequence | No.1 | No.2 | Annotation | Function | Disease |
|---|---|---|---|---|---|---|---|---|---|
| 7882 | KIAA1387 | NM_020463.1 | 1643 | gggacttcttcgtactcttaattg | 377708 | 750307 | see also 7879 line | | |
| 7883 | KIAA1387 | NM_020463.1 | 2153 | accatctatattgcgtagtaaca | 377720 | 750333 | see also 7879 line | | |
| 7884 | GALNT1 | NM_020474.2 | 1189 | aggtgttacaaaggtagattatg | 161844 | 561416 | - | transferase, transferring glycosyl groups | - |
| 7885 | GALNT1 | NM_020474.2 | 1199 | aaggtagattatggagatatatc | 161845 | 561417 | see also 7884 line | | |
| 7886 | PBXIP1 | NM_020524.2 | 631 | tgggcatctccctccacatgtgc | 113167 | 694768 | transcription_regulator | | - |
| 7887 | EPHA8 | NM_020526.2 | 831 | gggacacaccaagatgtactgc | 81647 | 501985 | receptor_acitivity, kinase_related, signal_transduction | ephrin receptor | - |
| 7888 | EPHA8 | NM_020526.2 | 2229 | gccgcctggcaatgattgtgact | 81653 | 501995 | see also 7887 line | | |
| 7889 | PCBP3 | NM_020528.1 | 791 | aggtggtcaggcctacacaatcc | 34579 | 463428 | - | RNA binding | - |
| 7890 | PCBP3 | NM_020528.1 | 911 | cgctttcccccggagaaaagctgc | 34581 | 463429 | see also 7889 line | | - |
| 7891 | RTN4 | NM_020532.3 | 1824 | cagattagtcacacagataat | 135437 | 540131 | apoptosis | | - |
| 7892 | CSRP2BP | NM_020536.2 | 2404 | ctctcggatcttgaaacettata | 215054 | 602362 | - | | - |
| 7893 | CSRP2BP | NM_020536.2 | 2406 | ctcggatcttgaaacettatatc | 215055 | 602363 | see also 7892 line | | |
| 7894 | CSRP2BP | NM_020536.2 | 2419 | accttatatcaggcgtgattatg | 215057 | 602364 | see also 7892 line | | |
| 7895 | ADCY2 | NM_020546.1 | 1117 | tccctaaccatgccaagaactgt | 164830 | 618410 | - | guanylate cyclase | - |
| 7896 | ADCY2 | NM_020546.1 | 1327 | tccctggacgtttcacatttct | 164833 | 618418 | see also 7895 line | | |
| 7897 | ADCY2 | NM_020546.1 | 1669 | ttcaaaatcgcaccttaagaacc | 164840 | 618433 | see also 7895 line | | |
| 7898 | ADCY2 | NM_020546.1 | 3165 | accggagtcctggacaaaataca | 164871 | 618466 | see also 7895 line | | |
| 7899 | ADCY2 | NM_020546.1 | 3273 | gaacctgaagacgtactttgtaa | 164876 | 618470 | see also 7895 line | | |
| 7900 | AMHR2 | NM_020547.1 | 170 | gagtgcgcgggaagcacaaagaca | 72052 | 493801 | receptor_acitivity, kinase_related, signal_transduction | transforming growth factor-beta receptor | persistent Mullerian duct syndrome |
| 7901 | C11orf15 | NM_020644.1 | 384 | ctgtctacgctgtgaatgcaaat | 326080 | 691424 | kinase_related | | - |
| 7902 | C11orf15 | NM_020644.1 | 386 | gtctacgctgtgaatgcaaatat | 326081 | 691425 | see also 7901 line | | |
| 7903 | C11orf15 | NM_020644.1 | 424 | gtcacaatcaaggttaccattat | 326082 | 691426 | see also 7901 line | | |
| 7904 | C11orf15 | NM_020644.1 | 426 | cacaatcaaggttaccattataa | 326083 | 691427 | see also 7901 line | | |
| 7905 | JPH1 | NM_020647.1 | 1044 | ttctggtccgtgggataaggaag | 280953 | 659983 | - | | |
| 7906 | JPH1 | NM_020647.1 | 1871 | aagtctgaacttgctcataccaaa | 280961 | 659999 | see also 7905 line | | |
| 7907 | PELI1 | NM_020651.2 | 4039 | atgtttcctgatcaagaaaa | 377853 | 741295 | - | | |
| 7908 | PELI1 | NM_020651.2 | 4253 | aagaccagcatagcatatcatat | 377856 | 713331 | see also 7907 line | | - |
| 7909 | PELI1 | NM_020651.2 | 4258 | cagcatagcatatcatatacttt | 377858 | 713333 | see also 7907 line | | |
| 7910 | PELI1 | NM_020651.2 | 4576 | atggaatggcttgaccactaatgg | 377867 | 713347 | see also 7907 line | | |
| 7911 | PELI1 | NM_020651.2 | 5027 | atcgtgaatgtcctatgtgtagg | 377878 | 713360 | see also 7907 line | | |

Figure 46

| 7912 | CLK4 | NM_020666.1 | 686 | ttgatcatcatggtcatgtttgt | 77195 | 498456 | kinase_related | - | - |
|---|---|---|---|---|---|---|---|---|---|
| 7913 | CLK4 | NM_020666.1 | 886 | aagtctgactatgtagtcaaata | 77199 | 498461 | see also 7912 line | | |
| 7914 | RAB22A | NM_020673.2 | 693 | ttgtagagaccagcgcaaaaaac | 110039 | 715420 | signal_transduction | protein transporter | - |
| 7915 | ABHD6 | NM_020676.3 | 124 | atgtggttaacatgtttgtgatt | 204091 | 594511 | - | - | - |
| 7916 | DSCAML1 | NM_020693.2 | 445 | cccctccgccttcaatagcttt | 186074 | 737850 | - | - | - |
| 7917 | DSCAML1 | NM_020693.2 | 446 | ccctccgccttcaatagcttta | 186075 | 737851 | see also 7916 line | | |
| 7918 | DSCAML1 | NM_020693.2 | 447 | ccctccgccttcaatagctttat | 186076 | 737852 | see also 7916 line | | |
| 7919 | DSCAML1 | NM_020693.2 | 449 | ctccgccttcaatagctttatcc | 186077 | 737853 | see also 7916 line | | |
| 7920 | DSCAML1 | NM_020693.2 | 452 | cgccttcaatagctttatccacg | 186078 | 737854 | see also 7916 line | | |
| 7921 | DSCAML1 | NM_020693.2 | 565 | ccgtccgggtggaggatcaaagg | 186082 | 737858 | see also 7916 line | | |
| 7922 | KIAA1145 | NM_020698.1 | 841 | cagcgctaccatcgtgaacaaac | 326847 | 771702 | - | - | - |
| 7923 | KIAA1145 | NM_020698.1 | 842 | agcgctaccatcgtgaacaaacc | 326848 | 771703 | see also 7922 line | | |
| 7924 | P66beta | NM_020699.1 | 1279 | cccagtgccgcacagatttcacc | 8842 | 442442 | transcription_regulator | - | - |
| 7925 | P66beta | NM_020699.1 | 1515 | gagaccatcatgagacatcatac | 8847 | 442445 | see also 7924 line | | |
| 7926 | P66beta | NM_020699.1 | 1664 | aggtgtcaacattgcatacttga | 8851 | 442451 | see also 7924 line | | |
| 7927 | SLC12A5 | NM_020708.3 | 1026 | ctgtctcgccatggctttgatgt | 256792 | 640469 | - | - | - |
| 7928 | SLC12A5 | NM_020708.3 | 1028 | gtctcgccatggctttgatgtct | 256793 | 640470 | see also 7927 line | | |
| 7929 | SLC12A5 | NM_020708.3 | 1327 | gtgatatgacctcctacttcacc | 256795 | 640474 | see also 7927 line | | |
| 7930 | KIAA1287 | NM_020748.1 | 383 | cagcagcttaggcataaacttgg | 378367 | 729394 | - | - | - |
| 7931 | KIAA1287 | NM_020748.1 | 912 | tggctttgacattggatcatact | 378383 | 729403 | see also 7930 line | | |
| 7932 | KIAA1287 | NM_020748.1 | 1544 | aaccagcttagtgctatcattga | 378399 | 729410 | see also 7930 line | | |
| 7933 | KIAA1287 | NM_020748.1 | 1548 | agcttagtgctatcattgacttg | 378400 | 729411 | see also 7930 line | | |
| 7934 | KIAA1287 | NM_020748.1 | 2561 | tgggtaatgacggttaatgcact | 378435 | 729437 | see also 7930 line | | |
| 7935 | KIAA1287 | NM_020748.1 | 3089 | tacattgcagatcccaacattgc | 378450 | 729455 | see also 7930 line | | |
| 7936 | KIAA1287 | NM_020748.1 | 3094 | tgcagatcccaacattgctaagc | 378451 | 729456 | see also 7930 line | | |
| 7937 | KIAA1287 | NM_020748.1 | 3120 | ttcactttcagggttatccatgt | 378453 | 729457 | see also 7930 line | | |
| 7938 | KIAA1287 | NM_020748.1 | 3425 | ttgtatgaggatattatgtcttt | 378464 | 729472 | see also 7930 line | | |
| 7939 | XPO5 | NM_020750.1 | 154 | ctggaagccctcaagttttgtga | 378469 | 733124 | - | - | - |
| 7940 | XPO5 | NM_020750.1 | 3560 | tccgaaaagaagttcacattaag | 378537 | 733209 | see also 7939 line | | |
| 7941 | COG6 | NM_020751.1 | 432 | cagactcaagatttaatagtaaa | 268698 | 650804 | - | - | - |
| 7942 | COG6 | NM_020751.1 | 1112 | caggcctctaaaggttcgaattg | 268718 | 650824 | see also 7941 line | | |
| 7943 | COG6 | NM_020751.1 | 1113 | aggcctctaaaggttcgaattga | 268719 | 650825 | see also 7941 line | | |
| 7944 | COG6 | NM_020751.1 | 1663 | atgagcaagcctcttatgtttta | 268742 | 650849 | see also 7941 line | | |
| 7945 | TDE2 | NM_020755.2 | 378 | ttctctctttactaatgatcaaa | 327610 | 693348 | - | - | - |
| 7946 | raptor | NM_020761.1 | 1230 | gtcacactggatttgatagaaaa | 378565 | 736875 | - | - | - |
| 7947 | HACE1 | NM_020771.2 | 1652 | atgacttcacctcgtttcattga | 228965 | 615527 | - | - | - |

Figure 46

| No. | Gene | Accession | Pos | Sequence | ID1 | ID2 | Note | Function | Disease |
|---|---|---|---|---|---|---|---|---|---|
| 7948 | HACE1 | NM_020771.2 | 1780 | aagattcatgcatatcataaag | 228972 | 615533 | see also 7947 line | | |
| 7949 | HACE1 | NM_020771.2 | 1905 | tgctggttcacagagattctatt | 228977 | 615536 | see also 7947 line | | |
| 7950 | KIAA1322 | NM_020773.1 | 1503 | aagccagtctggagcttattaaa | 378760 | 749084 | - | - | |
| 7951 | KIAA1322 | NM_020773.1 | 1732 | accctgtcaaatggcgtttta | 378766 | 749091 | see also 7950 line | - | |
| 7952 | MIB | NM_020774.1 | 261 | ctgccgccagcaaccaatcattg | 378776 | 754311 | - | | |
| 7953 | MIB | NM_020774.1 | 262 | tgccgccagcaaccaatcattgg | 378777 | 754312 | see also 7952 line | | |
| 7954 | MIB | NM_020774.1 | 823 | atgtttgagactttaactacaac | 378793 | 754329 | see also 7952 line | | |
| 7955 | MIB | NM_020774.1 | 1084 | atgcttcaactttaggtaaagt | 378800 | 754335 | see also 7952 line | | |
| 7956 | MIB | NM_020774.1 | 1106 | ttggccgagtacaacagattat | 378801 | 754336 | see also 7952 line | | |
| 7957 | MIB | NM_020774.1 | 1107 | tggccgagtacaacagatttatt | 378802 | 754337 | see also 7952 line | | |
| 7958 | MIB | NM_020774.1 | 1109 | gccgagtacaacagatttattca | 378803 | 754339 | see also 7952 line | | |
| 7959 | MIB | NM_020774.1 | 1356 | aagaccagatgtggattgtaaatg | 378811 | 754344 | see also 7952 line | | |
| 7960 | MIB | NM_020774.1 | 1824 | tccagtgcaatgcgtgtttac | 378827 | 754354 | see also 7952 line | | |
| 7961 | MIB | NM_020774.1 | 2403 | gggttctcggagtccttctatga | 378843 | 754372 | see also 7952 line | | |
| 7962 | MIDORI | NM_020778.1 | 1310 | gcggctggttgccaagttgaagc | 91455 | 495543 | kinase_related | - | |
| 7963 | MIDORI | NM_020778.1 | 5292 | atctaccggcctgcaaacaatat | 91481 | 495572 | see also 7962 line | - | |
| 7964 | KIAA1336 | NM_020779.2 | 1380 | atgaccaaaaccaatgatagc | 378964 | 774067 | - | - | |
| 7965 | KIAA1336 | NM_020779.2 | 1473 | cagatcaacacgtctcgaaaaga | 378966 | 774069 | see also 7964 line | - | |
| 7966 | ZNF398 | NM_020781.2 | 1299 | gagggacaagtgactttactca | 45474 | 487630 | transcription_regulator | - | |
| 7967 | SYT4 | NM_020783.2 | 237 | aagactcctccatcacaagttgt | 275126 | 699006 | - | transporter | |
| 7968 | IGSF9 | NM_020789.2 | 2938 | cccttcttccgagagatgaargt | 379037 | 745429 | - | | |
| 7969 | IGSF9 | NM_020789.2 | 3579 | tgcccttcgggaggaattcctgg | 379044 | 745431 | see also 7968 line | - | |
| 7970 | KIAA1363 | NM_020792.1 | 1258 | tgggaatccggactaggaatagt | 205261 | 595226 | - | | |
| 7971 | KIAA1363 | NM_020792.1 | 1264 | tccggactaggaatagttacatc | 205262 | 595227 | see also 7970 line | - | |
| 7972 | SEMA6A | NM_020796.1 | 744 | ttcgcatggcaactattacaaac | 135728 | 540216 | receptor_acitivity, apoptosis, signal_transduction | - | non-small-cell lung cancer |
| 7973 | SEMA6A | NM_020796.1 | 2549 | agggagtgattcggaaagttac | 135779 | 540261 | see also 7972 line | | |
| 7974 | SEMA6A | NM_020796.1 | 2550 | gggagtgattcggaaagttacc | 135780 | 540262 | see also 7972 line | - | |
| 7975 | KIAA1376 | NM_020801.1 | 483 | cacgaaagatgatgatgataattc | 314094 | 804868 | - | - | |
| 7976 | KIAA1376 | NM_020801.1 | 532 | caggaaggcatgaaatatgcattc | 314097 | 804869 | see also 7975 line | | |
| 7977 | KIAA1376 | NM_020801.1 | 620 | ttgggtgaaagccgaattgcaca | 314099 | 804870 | see also 7975 line | | |
| 7978 | GPHN | NM_020806.3 | 1460 | aagctcggccaggccaagatatc | 236543 | 671592 | enzyme | - | |
| 7979 | GPHN | NM_020806.3 | 1630 | gggaatgagctgctaaatcctga | 236550 | 671597 | see also 7978 line | | |
| 7980 | GPHN | NM_020806.3 | 2063 | atcctcggccaaccatcatcaaa | 236565 | 671612 | see also 7978 line | | |
| 7981 | GPHN | NM_020806.3 | 2064 | tcctcggccaaccatcatcaaag | 236566 | 671613 | see also 7978 line | | |

Figure 46

| | | | | | | | | | |
|---|---|---|---|---|---|---|---|---|---|
| 7982 | PCDH10 | NM_020815.1 | 2221 | gccggtctacgacgtgtatgtga | 149438 | 550989 | - | - | - |
| 7983 | PCDH10 | NM_020815.1 | 3313 | cgccaagaccgacctgatgtttc | 149450 | 551007 | see also 7982 line | | |
| 7984 | SYT13 | NM_020826.1 | 1114 | gtggaacgagatgatcatgtttg | 275017 | 657563 | - | - | - |
| 7985 | KIAA1441 | NM_020832.1 | 1736 | gagcctggcacggcactatgacc | 265178 | 647973 | - | - | - |
| 7986 | KIAA1449 | NM_020839.2 | 748 | aacataccgagtccatgatgaag | 379417 | 722863 | - | - | - |
| 7987 | KIAA1449 | NM_020839.2 | 1685 | atgcccaaattcaacaaaattcc | 379452 | 722900 | see also 7986 line | | |
| 7988 | OSBPL8 | NM_020841.3 | 520 | tcgcttcttggtgatagcaaaga | 303670 | 789351 | - | - | - |
| 7989 | ZNF291 | NM_020843.1 | 47 | ttccagcgctccaatagtcatga | 379459 | 789702 | - | - | - |
| 7990 | ZNF291 | NM_020843.1 | 3350 | gtggtgaacatgggtctgattga | 379565 | 789766 | see also 7989 line | | |
| 7991 | ZNF291 | NM_020843.1 | 3846 | tcgtctgtgtgggctacttcact | 379580 | 789774 | see also 7989 line | | |
| 7992 | PITPNM2 | NM_020845.1 | 1765 | gggggcatcctggcatttgatgc | 150632 | 564226 | - | - | - |
| 7993 | KIAA1463 | NM_020849.1 | 812 | cactaggaggaatccatatatct | 236613 | 621453 | - | - | - |
| 7994 | KIAA1463 | NM_020849.1 | 2021 | ctgtcagcaccacttttggatca | 236633 | 621476 | see also 7993 line | | |
| 7995 | KIAA1468 | NM_020854.2 | 1126 | cagtgttgggaacagattaatca | 379656 | 782902 | - | - | - |
| 7996 | KIAA1468 | NM_020854.2 | 1128 | gtgttgggaacagattaatcaca | 379657 | 782903 | see also 7995 line | | |
| 7997 | KIAA1468 | NM_020854.2 | 2557 | ttccgagatgagtttgttatacc | 379707 | 782941 | see also 7995 line | | |
| 7998 | KIAA1474 | NM_020856.1 | 1708 | tggaccccatgagcatgctttc | 5825 | 441559 | transcription_regulator | - | - |
| 7999 | KIAA1474 | NM_020856.1 | 1998 | gagaatgccttgtcagatatatc | 5833 | 441564 | see also 7998 line | | |
| 8000 | SEMA6D | NM_020858.1 | 554 | aggcaatatccggttttttagagg | 320815 | 775529 | receptor_acitivity | - | - |
| 8001 | SEMA6D | NM_020858.1 | 617 | cagctgatgttgaaaattcgaga | 320817 | 775531 | see also 8000 line | | |
| 8002 | SEMA6D | NM_020858.1 | 1056 | ctgcccttcgcacaataaaatat | 320837 | 775542 | see also 8000 line | | |
| 8003 | SEMA6D | NM_020858.1 | 1114 | tgccatagaatatggaaactatg | 320839 | 775546 | see also 8000 line | | |
| 8004 | SEMA6D | NM_020858.1 | 1115 | gccatagaatatggaaactatgt | 320840 | 775547 | see also 8000 line | | |
| 8005 | DHX36 | NM_020865.1 | 1698 | ctggtgttcggaaaatagtaatt | 59907 | 483726 | - | - | - |
| 8006 | DHX36 | NM_020865.1 | 1699 | tggtgttcggaaaatagtaattg | 59908 | 483727 | see also 8005 line | | |
| 8007 | DHX36 | NM_020865.1 | 1704 | ttcggaaaatagtaattgctacc | 59909 | 483728 | see also 8005 line | | |
| 8008 | DHX36 | NM_020865.1 | 1838 | gagtgggttagtaaagctaatgc | 59915 | 483731 | see also 8005 line | | |
| 8009 | KLHL1 | NM_020866.1 | 1763 | ttgatgatgtgggtcaagtatga | 117703 | 523261 | - | actin binding | - |
| 8010 | DPP10 | NM_020868.1 | 2416 | tggctatattgcatcaatgatct | 186974 | 580813 | - | - | - |
| 8011 | LRRN1 | NM_020873.3 | 1706 | gggtcactccattggaaataag | 379821 | 697519 | - | - | - |
| 8012 | PHF12 | NM_020889.1 | 1468 | atggatgtgtccgaatcacatcg | 34707 | 463497 | transcription_regulator | - | - |
| 8013 | PHF12 | NM_020889.1 | 1477 | tccgaatcacatcgaacatgtgg | 34708 | 463498 | see also 8012 line | | |
| 8014 | PHF12 | NM_020889.1 | 1842 | ttgcgctccagtgcagcatattg | 34717 | 463506 | see also 8012 line | | |
| 8015 | PHF12 | NM_020889.1 | 1843 | tgcgctccagtgcagcatattga | 34718 | 463507 | see also 8012 line | | |
| 8016 | KIAA1524 | NM_020890.1 | 575 | ttgtcggcacaatctttctgttc | 379993 | 776544 | - | - | - |

Figure 46

| | | | | | | | | | |
|---|---|---|---|---|---|---|---|---|---|
| 8017 | KIAA1524 | NM_020890.1 | 1547 | tcctggtatggaagtaagcttct | 380038 | 776569 | see also 8016 line | | |
| 8018 | KIAA1524 | NM_020890.1 | 1555 | tggaagtaagcttctacaaaata | 380039 | 776571 | see also 8016 line | | |
| 8019 | OSBPL5 | NM_020896.2 | 999 | ctggagaacgatgcattctcaga | 179588 | 572865 | - | - | - |
| 8020 | HCN3 | NM_020897.1 | 681 | tcccgcctcatccgctacataca | 107983 | 517973 | - | - | - |
| 8021 | KIAA1560 | NM_020918.2 | 452 | ttgggtttgcggaatgttattta | 215822 | 603602 | - | - | - |
| 8022 | KIAA1560 | NM_020918.2 | 454 | gggtttgcggaatgttatttata | 215823 | 603603 | see also 8021 line | | |
| 8023 | ALS2 | NM_020919.2 | 1668 | aagtttcccccaggctcttaaga | 294669 | 735547 | - | - | primary lateral sclerosis、 spastic paralysis、 amyotrophic lateral sclerosis |
| 8024 | ALS2 | NM_020919.2 | 2185 | ctcctgtagtaagcttggatata | 294683 | 735560 | see also 8023 line | | |
| 8025 | ALS2 | NM_020919.2 | 2608 | gcctgccattgatttcctaaata | 294702 | 735574 | see also 8023 line | | |
| 8026 | ALS2 | NM_020919.2 | 2714 | ttgccaatcagacgacttcataa | 294705 | 735577 | see also 8023 line | | |
| 8027 | ALS2 | NM_020919.2 | 3654 | ttggccagtgggtaatggataag | 294728 | 735594 | see also 8023 line | | |
| 8028 | ALS2 | NM_020919.2 | 3659 | cagtgggtaatggataagaaagc | 294729 | 735595 | see also 8023 line | | |
| 8029 | ALS2 | NM_020919.2 | 4801 | gtcaatccttggagagagtaaaa | 294768 | 735622 | see also 8023 line | | |
| 8030 | ALS2 | NM_020919.2 | 4875 | tgcagcagatcagcacaacattt | 294772 | 735625 | see also 8023 line | | |
| 8031 | ZNF481 | NM_020924.2 | 719 | gccccagcactccctgataaatt | 45666 | 815735 | transcription_regulator | protein binding | - |
| 8032 | ZNF481 | NM_020924.2 | 930 | gagaactacgccaaccatttaaa | 45673 | 815741 | see also 8031 line | | |
| 8033 | KIAA1573 | NM_020925.1 | 1873 | tagttcggcaaaatatcctaagc | 265214 | 811570 | - | - | - |
| 8034 | KIAA1573 | NM_020925.1 | 2034 | ctgtgtattgtggtgatacaacc | 265220 | 811574 | see also 8033 line | | |
| 8035 | KIAA1573 | NM_020925.1 | 2409 | tacattgtccgccgttacatagc | 265222 | 811588 | see also 8033 line | | |
| 8036 | KIAA1573 | NM_020925.1 | 2435 | acccaatggcgtcctcagaattt | 265223 | 811590 | see also 8033 line | | |
| 8037 | KIAA1587 | NM_020932.1 | 2490 | aactaagctgcctataccaaaga | 380183 | 745932 | - | - | - |
| 8038 | USP37 | NM_020935.1 | 398 | atggtcctatcagaattcgaagt | 189734 | 791591 | - | - | - |
| 8039 | USP37 | NM_020935.1 | 530 | ttcagctaagtcataacattaaa | 189738 | 791595 | see also 8038 line | | |
| 8040 | USP37 | NM_020935.1 | 2012 | gtgctcttgtcaggcacaaattt | 189774 | 791651 | see also 8038 line | | |
| 8041 | USP37 | NM_020935.1 | 2013 | tgctcttgtcaggcacaaattta | 189775 | 791652 | see also 8038 line | | |
| 8042 | USP37 | NM_020935.1 | 2018 | ttgtcaggcacaaatttaacagg | 189776 | 791654 | see also 8038 line | | |
| 8043 | GBA2 | NM_020944.2 | 1321 | tgcctgtaggagtctttgtgtgg | 220527 | 607770 | - | - | - |
| 8044 | GBA2 | NM_020944.2 | 1797 | gtccactacaggcggtatacaag | 220530 | 607775 | see also 8043 line | | |
| 8045 | GBA2 | NM_020944.2 | 1938 | ctgcctgcctggtacaaatctgc | 220533 | 607778 | see also 8043 line | | |
| 8046 | TRPM3 | NM_020952.1 | 1120 | acgagacatgggccctcaaatac | 262014 | 646479 | channel | - | - |
| 8047 | TRPM3 | NM_020952.1 | 1127 | atgggccctcaaatacattgtac | 262016 | 646480 | see also 8046 line | | |
| 8048 | TRPM3 | NM_020952.1 | 2439 | gagtgacgggagggtcatctact | 262038 | 646505 | see also 8046 line | | |
| 8049 | TRPM3 | NM_020952.1 | 2441 | gtgacgggagggtcatctactgc | 262039 | 646506 | see also 8046 line | | |
| 8050 | TRPM3 | NM_020952.1 | 2500 | atcttcggcgtgaacaagtattt | 262041 | 646510 | see also 8046 line | | |
| 8051 | TRPM3 | NM_020952.1 | 3063 | aagggactacggcctgaaactct | 262051 | 646522 | see also 8046 line | | |

Figure 46

| | | | | | | | | | |
|---|---|---|---|---|---|---|---|---|---|
| 8052 | MOV10 | NM_020963.1 | 1338 | accgtgtcaagctgagcttttcc | 91822 | 508638 | - | - | - |
| 8053 | MAGI-3 | NM_020965.2 | 655 | aggatcaattgaccacaaactgc | 209324 | 606792 | kinase_related | - | - |
| 8054 | MAGI-3 | NM_020965.2 | 2483 | tacctgaaatctaagactttata | 209352 | 606837 | see also 8053 line | | |
| 8055 | MAGI-3 | NM_020965.2 | 2592 | atggacctgaccagtctatatat | 209354 | 606839 | see also 8053 line | | |
| 8056 | MAGI-3 | NM_020965.2 | 2593 | tggacctgaccagtctatatata | 209355 | 606840 | see also 8053 line | | |
| 8057 | MAGI-3 | NM_020965.2 | 2595 | gacctgaccagtctatatatatt | 209356 | 606841 | see also 8053 line | | |
| 8058 | MAGI-3 | NM_020965.2 | 3659 | aggccaggaactggcttgatacc | 209372 | 606862 | see also 8053 line | | |
| 8059 | SCUBE2 | NM_020974.1 | 1547 | aagtgtaacctttaagctaaatg | 152918 | 710091 | - | - | - |
| 8060 | SCUBE2 | NM_020974.1 | 2674 | atctggtgatgcggaaaacctct | 152948 | 710118 | see also 8059 line | | |
| 8061 | SCUBE2 | NM_020974.1 | 3030 | atccgattgctacgttccaaagt | 152952 | 710124 | see also 8059 line | | |
| 8062 | CKMT1 | NM_020990.2 | 624 | ttgatgagaggtatgtattgtcc | 209443 | 598805 | kinase_related | creatine kinase | - |
| 8063 | NR2F2 | NM_021005.2 | 1106 | ctcgtacctgtccggatatattt | 8466 | 447192 | transcription_regulator、receptor_acitivity、signal_transduction | transcription co-repressor | - |
| 8064 | NR2F2 | NM_021005.2 | 1107 | tcgtacctgtccggatatatttc | 8467 | 447193 | see also 8063 line | | |
| 8065 | NR2F2 | NM_021005.2 | 1703 | ctcctcagtcatagagcaattgt | 8474 | 447197 | see also 8063 line | | |
| 8066 | NR2F2 | NM_021005.2 | 1780 | tactgtccggcagcagttttaac | 8476 | 447200 | see also 8063 line | | |
| 8067 | LZTS1 | NM_021020.1 | 294 | gagcgaagacttcttctacatca | 6238 | 816826 | transcription_regulator、cell_cycle | - | - |
| 8068 | LZTS1 | NM_021020.1 | 1867 | atcccctacgaggacatcatagc | 6239 | 816839 | see also 8067 line | | |
| 8069 | TLX3 | NM_021025.1 | 211 | gaggacgcgggatcttacagtgt | 380365 | 449590 | transcription_regulator | transcription factor | - |
| 8070 | RAP2A | NM_021033.3 | 559 | ccgcgtgaagcggtatgagaaag | 110583 | 518355 | signal_transduction | RAS small monomeric GTPase | - |
| 8071 | MBNL1 | NM_021038.1 | 1505 | gacacggaatgtaaatttgcaca | 52460 | 474092 | - | RNA binding | - |
| 8072 | MBNL1 | NM_021038.1 | 1521 | ttgcacatccttcgaaaagctgc | 52461 | 474093 | see also 8071 line | | |
| 8073 | MBNL1 | NM_021038.1 | 1531 | ttcgaaaagctgccaagttgaaa | 52462 | 474094 | see also 8071 line | | |
| 8074 | MBNL1 | NM_021038.1 | 1753 | acccgtgccaatgtttcagttg | 52467 | 474099 | see also 8071 line | | |
| 8075 | MBNL1 | NM_021038.1 | 2279 | aacggtgccaccgcagtctttaa | 52475 | 474110 | see also 8071 line | | |
| 8076 | DHH | NM_021044.2 | 1435 | gccgactggcatgcattggtact | 191331 | 584819 | - | peptidase | - |
| 8077 | HCN1 | NM_021072.1 | 657 | aagtgatcaagatgaattattta | 261180 | 517904 | channel | - | - |
| 8078 | HCN1 | NM_021072.1 | 1238 | cggcagtatcaagagaagtataa | 261198 | 517921 | see also 8077 line | | |
| 8079 | NMT1 | NM_021079.3 | 932 | agctgattgaagtgaagttctcc | 215452 | 602739 | - | glycyl-peptide N-tetradecanoyltransferase | - |
| 8080 | DAB1 | NM_021080.3 | 835 | cagctcggggagacaagttatgt | 319866 | 534267 | - | - | - |
| 8081 | SLC15A2 | NM_021082.2 | 608 | atcaatgcagggagcttgatttc | 256486 | 643923 | - | peptide:hydrogen symporter | - |

Figure 46

| | | | | | | | | | |
|---|---|---|---|---|---|---|---|---|---|
| 8082 | XK | NM_021083.2 | 863 | tccccattccctgagaacataga | 271805 | 713658 | - | transporter | McLeod syndrome with neuroacanthosis、McLeod syndrome |
| 8083 | MTMR3 | NM_021090.2 | 1581 | gacccttattaccgaaccataga | 193004 | 558962 | phosphatase | - | - |
| 8084 | SLC8A1 | NM_021097.1 | 1892 | aacaatatcagtcaaggtaattg | 122771 | 527355 | - | sodium transporter | - |
| 8085 | SLC8A1 | NM_021097.1 | 2767 | atggggaacagttcaaagtgtcc | 122779 | 527372 | see also 8084 line | | |
| 8086 | RECK | NM_021111.1 | 222 | aaccaaatgtgccgtgatgtatg | 293083 | 662912 | cell_cycle | serine protease inhibitor | - |
| 8087 | RECK | NM_021111.1 | 1796 | ctgtatggaaatgcactgtatag | 293142 | 662945 | see also 8086 line | | |
| 8088 | RECK | NM_021111.1 | 2174 | ctgcctgacgacttttgataaat | 293149 | 662956 | see also 8086 line | | |
| 8089 | RECK | NM_021111.1 | 2175 | tgcctgacgacttttgataaatt | 293150 | 662957 | see also 8086 line | | |
| 8090 | RECK | NM_021111.1 | 2176 | gcctgacgacttttgataaattt | 293151 | 662958 | see also 8086 line | | |
| 8091 | CRY2 | NM_021117.1 | 754 | ggcctgggttgccaactatgaga | 234008 | 618377 | - | - | - |
| 8092 | CRY2 | NM_021117.1 | 1193 | gagagcggggtccgggtatttga | 234012 | 618383 | see also 8091 line | | |
| 8093 | CRY2 | NM_021117.1 | 1195 | gagcggggtccgggtatttgatg | 234013 | 618384 | see also 8091 line | | |
| 8094 | CRY2 | NM_021117.1 | 1385 | ttcccctctcgatacatctatga | 234018 | 618389 | see also 8091 line | | |
| 8095 | DLG3 | NM_021120.1 | 1946 | aagagctcgattgaaaactgtga | 209293 | 534591 | kinase_related | guanylate kinase | - |
| 8096 | DLG3 | NM_021120.1 | 2014 | cggggttaagtgacgattattat | 209297 | 534594 | see also 8095 line | | |
| 8097 | DLG3 | NM_021120.1 | 2016 | gggttaagtgacgattattatgg | 209298 | 534596 | see also 8095 line | | |
| 8098 | RTN1 | NM_021136.1 | 611 | ttctgattctggaatagagatga | 255128 | 466577 | signal_transduction | signal transducer | - |
| 8099 | RTN1 | NM_021136.1 | 2374 | gacttgtgaggactcacataaat | 255150 | 466605 | see also 8098 line | | |
| 8100 | UTX | NM_021140.1 | 1195 | ttgcagcacgaattaagtattta | 324923 | 690666 | - | - | - |
| 8101 | UTX | NM_021140.1 | 1196 | tgcagcacgaattaagtatttac | 324924 | 690667 | see also 8100 line | | |
| 8102 | UTX | NM_021140.1 | 1725 | ttggccaatggaccctttctgc | 324935 | 690676 | see also 8100 line | | |
| 8103 | UTX | NM_021140.1 | 1915 | agccgtggaaaaaccaactatct | 324938 | 690678 | see also 8100 line | | |
| 8104 | UTX | NM_021140.1 | 4180 | aagtctatgaccaatttacatta | 324955 | 690733 | see also 8100 line | | |
| 8105 | XRCC5 | NM_021141.2 | 1211 | ttcgatatgcttatgacaaaaga | 20329 | 470390 | kinase_related | Ku70/80 antigen complex | - |
| 8106 | XRCC5 | NM_021141.2 | 1316 | aagacttgcggcaatacatgttt | 20334 | 470391 | see also 8105 line | | |
| 8107 | XRCC5 | NM_021141.2 | 1318 | gacttgcggcaatacatgttttc | 20335 | 470392 | see also 8105 line | | |
| 8108 | DMTF1 | NM_021145.1 | 1608 | gcccgcttggaagataatacagc | 2792 | 458385 | transcription_regulator、kinase_related | - | - |
| 8109 | DMTF1 | NM_021145.1 | 1915 | atgctttatccccagaacatttg | 2805 | 458394 | see also 8108 line | | |
| 8110 | DMTF1 | NM_021145.1 | 1922 | atccccagaacatttgttgaaca | 2807 | 458395 | see also 8108 line | | |
| 8111 | RAP1GDS1 | NM_021159.3 | 726 | tagcagaacttgagtcaagtaaa | 298178 | 757187 | - | GTPase activator | - |
| 8112 | RAP1GDS1 | NM_021159.3 | 1586 | accatggcaactagtgaacatgt | 298200 | 757222 | see also 8111 line | | |
| 8113 | BAT5 | NM_021160.1 | 150 | ttccagctcctgggatacgtact | 235827 | 621643 | - | - | - |

Figure 46

| | | | | | | | | |
|---|---|---|---|---|---|---|---|---|
| 8114 | KCNK10 | NM_021161.3 | 986 | aggaggccaaatctttgtattt | 261240 | 644698 | channel, signal_transd uction | - |
| 8115 | KCNK10 | NM_021161.3 | 1041 | ttcttattggctggaattggaga | 261242 | 644702 | see also 8114 line | - |
| 8116 | IGRP | NM_021176.1 | 362 | gtgtctggtatgtcatggtaacc | 380592 | 711051 | - | - |
| 8117 | RAP2C | NM_021183.3 | 1055 | caggatatcaagccaatgagaga | 110597 | 520239 | signal_transduction | |
| 8118 | RAP2C | NM_021183.3 | 1262 | atcgtcaggcaaatgaactattc | 110601 | 520246 | see also 8117 line | |
| 8119 | RAP2C | NM_021183.3 | 1298 | aagcaagatcagtgttgtacaac | 110602 | 520249 | see also 8117 line | - |
| 8120 | ZNF410 | NM_021188.1 | 514 | aagatctacagccaagtgatagc | 380716 | 754132 | transcription_regulator | - |
| 8121 | ZNF410 | NM_021188.1 | 1315 | agccactaatgggcagtagtttg | 380730 | 754141 | see also 8120 line | |
| 8122 | ZNF410 | NM_021188.1 | 1316 | gccactaatgggcagtagtttgc | 380731 | 754142 | see also 8120 line | |
| 8123 | PTBP2 | NM_021190.1 | 463 | gacagataataacattaaaccaac | 65151 | 471286 | - | |
| 8124 | PTBP2 | NM_021190.1 | 576 | cagcccagtccagtacttaga | 65153 | 471290 | see also 8123 line | |
| 8125 | PTBP2 | NM_021190.1 | 581 | cagagtccagtacttagaataat | 65156 | 471291 | see also 8123 line | |
| 8126 | PTBP2 | NM_021190.1 | 583 | gagtccagtacttagaataatta | 65157 | 471292 | see also 8123 line | |
| 8127 | PTBP2 | NM_021190.1 | 793 | ctgtaccctaaggattgatttt | 65169 | 471297 | see also 8123 line | |
| 8128 | PTBP2 | NM_021190.1 | 849 | atgataaaagtagggattatact | 65170 | 471300 | see also 8123 line | |
| 8129 | PTBP2 | NM_021190.1 | 1317 | ttgatgatcaagggctaacaaaa | 65177 | 471309 | see also 8123 line | |
| 8130 | HOXD11 | NM_021192.2 | 841 | ctggaacgcgagtttttctttaa | 380743 | 697137 | transcription_regulator | transcription factor |
| 8131 | HOXD11 | NM_021192.2 | 842 | tggaacgcgagtttttctttaac | 380744 | 697138 | see also 8130 line | |
| 8132 | SLC30A1 | NM_021194.1 | 1395 | tagcatttcttgttttagaactta | 266803 | 653921 | - | cation transporter |
| 8133 | CTDSP1 | NM_021198.1 | 340 | gacagctcgccgtcattactca | 278890 | 690158 | phosphatase | |
| 8134 | CTDSP1 | NM_021198.1 | 342 | cagctcggccgtcattactcaga | 278891 | 690159 | see also 8133 line | |
| 8135 | CTDSP1 | NM_021198.1 | 756 | gcgaatggcggagctctttgaat | 278893 | 690161 | see also 8133 line | |
| 8136 | CTDSP1 | NM_021198.1 | 998 | cggttggcctcgtgtttgacaac | 278895 | 690164 | see also 8133 line | |
| 8137 | C20orf110 | NM_021202.2 | 976 | gccagcgccagttcaactactga | 380754 | 800661 | - | |
| 8138 | APMCF1 | NM_021203.2 | 546 | tagcctgcaataagcaagatatt | 108205 | 520540 | receptor_acitivity | receptor |
| 8139 | APMCF1 | NM_021203.2 | 547 | agcctgcaataagcaagatattg | 108206 | 520541 | see also 8138 line | |
| 8140 | C20orf77 | NM_021215.2 | 436 | gagctccgcaaagccaaatcaaa | 380765 | 729497 | - | |
| 8141 | C20orf77 | NM_021215.2 | 437 | agctccgcaaagccaaatcaaat | 380766 | 729498 | see also 8140 line | |
| 8142 | DMRT3 | NM_021240.2 | 1141 | acccgctgatgctgaggaatact | 2742 | 443062 | transcription_regulator | - |
| 8143 | STRAIT114 99 | NM_021242.3 | 904 | aacgccatgaatgcttcattgg | 380881 | 725741 | - | |
| 8144 | RRAGD | NM_021244.2 | 957 | ttgtatttatggtctcaaagaag | 192254 | 586027 | - | |
| 8145 | RAB18 | NM_021252.3 | 164 | agcaacaatagrtgttgactta | 109934 | 519919 | signal_transduction | protein transporter |
| 8146 | RAB18 | NM_021252.3 | 371 | aacatactacaagaaatgaca | 109945 | 519930 | see also 8145 line | - |

Figure 46

| 8147 | RAB18 | NM_021252.3 | 434 | tcgtgaagtcgatagaaatgaag | 109947 | 519934 | see also 8145 line | | |
|------|-------|-------------|-----|--------------------------|--------|--------|--------------------|---|---|
| 8148 | TRIM39 | NM_021253.2 | 1352 | gtcaggcttcgagatgcttaagg | 160500 | 559887 | - | - | - |
| 8149 | TRIM39 | NM_021253.2 | 1955 | acccaagcgggtaggcatattcc | 160510 | 559897 | see also 8148 line | | |
| 8150 | TRIM39 | NM_021253.2 | 1959 | aagcgggtaggcatattcctaga | 160511 | 559898 | see also 8148 line | | |
| 8151 | PLEKHA1 | NM_021622.2 | 2116 | aacacaataggctacttcaaatc | 173044 | 749304 | - | - | - |
| 8152 | RISC | NM_021626.1 | 183 | atgttctggtggctctattatgc | 187287 | 580950 | - | - | - |
| 8153 | RISC | NM_021626.1 | 834 | aacttctataacatcttaactaa | 187298 | 580966 | see also 8152 line | | |
| 8154 | SENP2 | NM_021627.1 | 320 | gaccaagattagattgctttatt | 135787 | 531256 | signal_transduction | - | - |
| 8155 | SENP2 | NM_021627.1 | 523 | ctgaaactgggtaataaatctcc | 135791 | 531266 | see also 8154 line | | |
| 8156 | C3IP1 | NM_021633.2 | 916 | agcctttcatccgctgtagttta | 129532 | 533032 | - | - | - |
| 8157 | C3IP1 | NM_021633.2 | 917 | gcctttcatccgctgtagtttac | 129533 | 533033 | see also 8156 line | | |
| 8158 | C3IP1 | NM_021633.2 | 921 | ttcatccgctgtagtttacaatg | 129534 | 533034 | see also 8156 line | | |
| 8159 | SP192 | NM_021639.3 | 1028 | ttgttcctgcttggctaaatttc | 380977 | 740874 | - | - | - |
| 8160 | SP192 | NM_021639.3 | 1031 | ttcctgcttggctaaatttctca | 380978 | 740875 | see also 8159 line | | |
| 8161 | SP192 | NM_021639.3 | 1252 | gtctctaagggagcatatgctgg | 380981 | 740882 | see also 8159 line | | |
| 8162 | TRB2 | NM_021643.1 | 1266 | atgggagatcgcggaacaaaacc | 103058 | 495583 | kinase_related | - | - |
| 8163 | TRB2 | NM_021643.1 | 1368 | cgcccgagactccgaacttgtcg | 103063 | 495586 | see also 8162 line | | |
| 8164 | TRB2 | NM_021643.1 | 1607 | gtgttctttgagcgaagctatgg | 103071 | 495589 | see also 8162 line | | |
| 8165 | TRB2 | NM_021643.1 | 2110 | gcggctgacctcgcaggaaattc | 103079 | 495598 | see also 8162 line | | |
| 8166 | OTX2 | NM_021728.2 | 839 | caggcttcaggttatagtcaagg | 8792 | 447533 | transcription_regulator | - | - |
| 8167 | OTX2 | NM_021728.2 | 953 | ctcagtcccatgggtaccaatgc | 8797 | 447537 | see also 8166 line | | |
| 8168 | OTX2 | NM_021728.2 | 1054 | aaccactgattgcttggattata | 8799 | 447538 | see also 8166 line | | |
| 8169 | FOXA2 | NM_021784.3 | 577 | ccccctacgccaacatgaactcc | 4012 | 443897 | transcription_regulator | - | - |
| 8170 | ACE2 | NM_021804.1 | 914 | tggggtagattttggacaaatct | 154479 | 555624 | - | - | - |
| 8171 | ACE2 | NM_021804.1 | 916 | gggtagattttggacaaatctgt | 154480 | 555625 | see also 8170 line | | |
| 8172 | ACE2 | NM_021804.1 | 2030 | ttccgatcatctgttgcatatgc | 154528 | 555658 | see also 8170 line | | |
| 8173 | SEC8 | NM_021807.2 | 1590 | cagtgtcctcttcgagagtttct | 65879 | 698701 | - | - | - |
| 8174 | SEC8 | NM_021807.2 | 2631 | aagaaaatgtgtaggaacatttt | 65900 | 698716 | see also 8173 line | | |
| 8175 | GALNT9 | NM_021808.2 | 566 | cacgtacggagaggtgagaaaca | 211247 | 812823 | - | - | - |
| 8176 | SLC5A7 | NM_021815.2 | 697 | tgggctgcagcaattttctctgc | 272091 | 657004 | - | - | - |
| 8177 | SAV1 | NM_021818.2 | 1142 | tagtgtacctcggtatgatcaac | 322087 | 686729 | signal_transduction | - | - |
| 8178 | MDS025 | NM_021825.3 | 637 | atgtcagcctggatactatctat | 381108 | 716979 | - | - | - |
| 8179 | MDS025 | NM_021825.3 | 702 | cagctaatggctcatttggatgc | 381111 | 716980 | see also 8178 line | | |
| 8180 | MDS025 | NM_021825.3 | 980 | ttcagagaccagtaataaaattg | 381115 | 716991 | see also 8178 line | | |
| 8181 | PEO1 | NM_021830.3 | 827 | aactgaaatccgccagtatttgc | 234645 | 618900 | - | - | - |

Figure 46

EP 1 752 536 A1

| 8182 | FLJ21839 | NM_021831.3 | 757 | ctggatggacttcgtgtgtagatct | 61654 | 480618 | - | - | - |
|------|----------|-------------|-----|---------------------------|-------|--------|---|---|---|
| 8183 | FLJ21839 | NM_021831.3 | 1525 | gtggaaaacatgctatatccaaa | 61666 | 480640 | see also 8182 line | | |
| 8184 | FLJ21839 | NM_021831.3 | 1692 | cagctacacacttgaatgcaact | 61671 | 480645 | see also 8182 line | | |
| 8185 | FLJ21839 | NM_021831.3 | 1700 | cacttgaatgcaactacaacact | 61672 | 480647 | see also 8182 line | | |
| 8186 | DTNB | NM_021907.3 | 309 | tagaaatgcgtgctcagaatttt | 130956 | 648920 | - | - | - |
| 8187 | DTNB | NM_021907.3 | 330 | ttgatgtcatacgactatcaact | 130957 | 648923 | see also 8186 line | | |
| 8188 | DTNB | NM_021907.3 | 388 | acgatgcaaccttcatcttgttg | 130965 | 648927 | see also 8186 line | | |
| 8189 | DTNB | NM_021907.3 | 642 | cagttaaagctatgttagcaacc | 130976 | 648938 | see also 8186 line | | |
| 8190 | DTNB | NM_021907.3 | 707 | tcccagatgtcagattccaatgg | 130978 | 648939 | see also 8186 line | | |
| 8191 | DTNB | NM_021907.3 | 717 | cagattccaatggcttaatgata | 130980 | 648940 | see also 8186 line | | |
| 8192 | DTNB | NM_021907.3 | 1139 | aagaagctgagccatgcaattag | 130989 | 648945 | see also 8186 line | | |
| 8193 | DTNB | NM_021907.3 | 1142 | aagctgagccatgcaattagtaa | 130990 | 648946 | see also 8186 line | | |
| 8194 | DTNB | NM_021907.3 | 1143 | agctgagccatgcaattagtaaa | 130991 | 648947 | see also 8186 line | | |
| 8195 | DTNB | NM_021907.3 | 1319 | cccaccaagaggttacagtatag | 130995 | 648955 | see also 8186 line | | |
| 8196 | CFL2 | NM_021914.5 | 272 | aagcgatgacaaaagacaaataa | 116139 | 521313 | - | actin binding | - |
| 8197 | ALX4 | NM_021926.1 | 110 | tgctgagacttgcgtctcttact | 871 | 441657 | transcription_regulator | translation regulator | - |
| 8198 | ALX4 | NM_021926.1 | 148 | ctgccatggacgcctactacagc | 872 | 441658 | see also 8197 line | | |
| 8199 | FLJ13220 | NM_021927.1 | 1079 | cagccaactcataaattatatgc | 57496 | 477388 | - | - | - |
| 8200 | FLJ11785 | NM_021930.2 | 376 | aagaacaggtacttacaatttca | 381290 | 795016 | - | - | - |
| 8201 | FLJ11785 | NM_021930.2 | 1126 | aagtacttatgtggattggaaac | 381319 | 795027 | see also 8200 line | | |
| 8202 | FLJ11785 | NM_021930.2 | 1577 | aactgacaggtataaaaatcttc | 381341 | 795033 | see also 8200 line | | |
| 8203 | RIC-8 | NM_021932.4 | 1549 | gagtgtgccccgattcatcaagt | 381377 | 745872 | - | - | - |
| 8204 | SMAP1 | NM_021940.2 | 247 | ctcgatgggcttcctggaatatt | 39590 | 467158 | - | - | - |
| 8205 | TEX27 | NM_021943.1 | 158 | ggggtccagcaagactatgaatc | 284689 | 469081 | - | - | - |
| 8206 | TEX27 | NM_021943.1 | 245 | aagtaacagccaatcagatttgt | 284691 | 469084 | see also 8205 line | | |
| 8207 | C14orf93 | NM_021944.1 | 1370 | caccaatgacaagagattcaatg | 381517 | 736759 | - | - | - |
| 8208 | C14orf93 | NM_021944.1 | 1371 | accaatgacaagagattcaatgg | 381518 | 736760 | see also 8207 line | | |
| 8209 | FLJ11362 | NM_021946.2 | 4199 | aggcccgtaagcagaagacttcc | 381565 | 804299 | - | - | - |
| 8210 | SRR | NM_021947.1 | 179 | aacagggcgcaatcttttcttca | 234541 | 618877 | - | racemase and epimerase、 acting on amino acids and derivatives | - |
| 8211 | ELAVL4 | NM_021952.2 | 475 | gacagagtttagggtatggattt | 47602 | 473754 | - | mRNA binding、 3' UTR | paraneoplastic encephalomyelitis |
| 8212 | ELAVL4 | NM_021952.2 | 485 | agggtatggatttgttaactata | 47603 | 473756 | see also 8211 line | | |
| 8213 | ELAVL4 | NM_021952.2 | 1315 | gagacagagtgttgcaagtttcc | 47621 | 473763 | see also 8211 line | | |

362

Figure 46

| | | | | | | | | | | |
|---|---|---|---|---|---|---|---|---|---|---|
| 8214 | ELAVL4 | NM_021952.2 | 1319 | cagagtgttgcaagtttcctta | 47622 | 473764 | see also 8211 line | | | cataract |
| 8215 | GJA3 | NM_021954.2 | 438 | gcggacctacgtcttcaacatca | 381577 | 647290 | - | | connexon channel | |
| 8216 | GRIK2 | NM_021956.2 | 923 | ggcattagctatgggaatgatga | 244459 | 632366 | receptor_acitivity, channel, signal_transduction | | | |
| 8217 | TEAD1 | NM_021961.2 | 750 | aggaaatctcgtgatttcattc | 381646 | 449457 | transcription_regulator | translation regulator | | |
| 8218 | TEAD1 | NM_021961.2 | 1142 | cccagactcgtacaacaacaaacacc | 381651 | 449462 | see also 8217 line | | | |
| 8219 | ZNF148 | NM_021964.1 | 433 | tgggcggcatagacgaaatgcagt | 44210 | 470481 | transcription_regulator | | specific RNA polymerase II transcription factor | |
| 8220 | ZNF148 | NM_021964.1 | 793 | cagagagccagtagacttacaga | 44215 | 470494 | see also 8219 line | | | |
| 8221 | ZNF148 | NM_021964.1 | 795 | gagagccagtagacttacagaaa | 44216 | 470495 | see also 8219 line | | | |
| 8222 | ZNF148 | NM_021964.1 | 797 | gagccagtagacttacagaaaa | 44217 | 470496 | see also 8219 line | | | |
| 8223 | ZNF148 | NM_021964.1 | 798 | agccagtagacttacagaaaaag | 44218 | 470497 | see also 8219 line | | | |
| 8224 | ZNF148 | NM_021964.1 | 892 | ccctaaatctcacgtttgtgagc | 44223 | 470500 | see also 8219 line | | | |
| 8225 | ZNF148 | NM_021964.1 | 931 | tagaacgaactatcacttacaga | 44225 | 470501 | see also 8219 line | | | |
| 8226 | ZNF148 | NM_021964.1 | 1065 | aaccatttcgctgtgatgaagt | 44226 | 470504 | see also 8219 line | | | |
| 8227 | ZNF148 | NM_021964.1 | 1186 | cagaacagatcgtgtattgaaac | 44232 | 470510 | see also 8219 line | | | |
| 8228 | ZNF148 | NM_021964.1 | 1189 | aacagatcgtgtattgaaacata | 44233 | 470511 | see also 8219 line | | | |
| 8229 | ZNF148 | NM_021964.1 | 1802 | ttgaagaagaagcggtatcttca | 44251 | 470523 | see also 8219 line | | | |
| 8230 | ZNF148 | NM_021964.1 | 1805 | aagaagaagcggtatcttcaagc | 44252 | 470524 | see also 8219 line | | | |
| 8231 | ZNF148 | NM_021964.1 | 1953 | aggcactgaatgtggagattaag | 44258 | 470528 | see also 8219 line | | | |
| 8232 | ZNF148 | NM_021964.1 | 2265 | gcccgagccttaactttgtgact | 44266 | 470541 | see also 8219 line | | | |
| 8233 | ZNF148 | NM_021964.1 | 2363 | agctttcgatcaggaatgaattc | 44267 | 470546 | see also 8219 line | | | |
| 8234 | ZNF148 | NM_021964.1 | 2410 | gtcccactttggactaatagttg | 44269 | 470548 | see also 8219 line | | | |
| 8235 | ZNF148 | NM_021964.1 | 2672 | gggacaagtgctgaatttcaga | 44276 | 470553 | see also 8219 line | | | |
| 8236 | RELA | NM_021975.1 | 664 | aggacattgaggtgtatttcacg | 10433 | 448092 | transcription_regulator, apoptosis, kinase_related | Rel A homodimer | | |
| 8237 | SLC22A3 | NM_021977.2 | 1250 | tcccctttgcggcaagcaatata | 266623 | 654401 | - | | | |
| 8238 | SLC22A3 | NM_021977.2 | 1251 | cccctttgcggcaagcaatatag | 266624 | 654402 | see also 8237 line | | | |
| 8239 | OPTN | NM_021980.3 | 313 | agccatgaagctaaataatcaag | 312174 | 807184 | signal_transduction | | | glaucoma |
| 8240 | OPTN | NM_021980.3 | 314 | gccatgaagctaaataatcaagc | 312175 | 807185 | see also 8239 line | | | |
| 8241 | ZNF6 | NM_021998.2 | 597 | ggctggtactgcacatatcgatg | 45769 | 798724 | - | | | |
| 8242 | CUZD1 | NM_022034.3 | 2364 | ttgtaaggtgtatccctatttg | 326373 | 688156 | - | | | |
| 8243 | TIA1 | NM_022037.1 | 715 | ctccaaagagtacatatgagtca | 47722 | 475847 | apoptosis | | | |

Figure 46

| | | | | | | | | |
|---|---|---|---|---|---|---|---|---|
| 8244 | TIA1 | NM_022037.1 | 835 | aactaatgctgcagactttca | 47728 | 475853 | | see also 8243 line |
| 8245 | TIA1 | NM_022037.1 | 1076 | gtggggccagtggtatggaaatg | 47739 | 475860 | | see also 8243 line |
| 8246 | MTBP | NM_022045.2 | 324 | tagttctgattggcaagagatac | 381685 | 537711 | | - |
| 8247 | DEF6 | NM_022047.2 | 1644 | ggccagcaccaacgtgaaacact | 141617 | 547128 | | - |
| 8248 | CSNK1G1 | NM_022048.1 | 291 | ttcggagagctcagattagttaa | 77677 | 498942 | kinase_related, signal transduction | protein serine/threonine kinase |
| 8249 | CSNK1G1 | NM_022048.1 | 292 | tcggagagctcagattaggttaaa | 77678 | 498943 | see also 8248 line | |
| 8250 | CSNK1G1 | NM_022048.1 | 297 | gagctcagattaggttaaaaatct | 77680 | 498944 | see also 8248 line | |
| 8251 | CSNK1G1 | NM_022048.1 | 722 | aaccaaaaaacatacaccttata | 77690 | 498962 | see also 8248 line | |
| 8252 | CSNK1G1 | NM_022048.1 | 984 | atggcaacctacctttcgatatgt | 77694 | 498970 | see also 8248 line | |
| 8253 | KCNK13 | NM_022054.2 | 1101 | aaggctggagctactttgactca | 261274 | 644773 | channel | |
| 8254 | SLC4A10 | NM_022058.2 | 1344 | aagattcctgctgtaccaaatgg | 11620 | 448575 | - | |
| 8255 | SLC4A10 | NM_022058.2 | 1433 | aaggatttttgggggacttattt | 11622 | 448576 | see also 8254 line | |
| 8256 | SLC4A10 | NM_022058.2 | 1641 | atgaccggatagcctattctct | 11628 | 448581 | see also 8254 line | |
| 8257 | SLC4A10 | NM_022058.2 | 2303 | gtgtatgtttaattgactatg | 11649 | 448596 | see also 8254 line | |
| 8258 | PKNOX2 | NM_022062.1 | 666 | agggcaacatcgccatgacaacc | 9401 | 447797 | transcription_regulator | - |
| 8259 | FLJ13188 | NM_022063.1 | 888 | tgcatgggtgtttgaagcaaaga | 381787 | 740082 | - | |
| 8260 | C14orf133 | NM_022067.2 | 1675 | gccatgatgtcgtcattgatacc | 382015 | 750411 | - | |
| 8261 | C14orf133 | NM_022067.2 | 1788 | ctcagcagctcgcaaattcgatg | 382018 | 750414 | see also 8260 line | |
| 8262 | FLJ23403 | NM_022068.1 | 282 | tggtgatgtcctcattcagttt | 382024 | 776689 | - | |
| 8263 | FLJ23403 | NM_022068.1 | 288 | tggtcctcattcagtttggaacc | 382025 | 776691 | see also 8262 line | |
| 8264 | FLJ23403 | NM_022068.1 | 417 | ctgtgtgactgagaggaaattc | 382028 | 776692 | see also 8262 line | |
| 8265 | FLJ22609 | NM_022072.2 | 655 | cacagcctttgataaatgtaatt | 382148 | 805049 | - | |
| 8266 | HPS4 | NM_022081.4 | 1233 | ggctgcatcctctattaaaggact | 127375 | 529504 | - | |
| 8267 | ZNF335 | NM_022095.3 | 1647 | ctgctcaaggagcacatgttca | 45266 | 815771 | transcription_regulator | DNA binding |
| 8268 | ANKRD5 | NM_022096.4 | 2287 | aaggcatatgctgattatagaat | 137840 | 543894 | | calcium ion binding |
| 8269 | ANKRD5 | NM_022096.4 | 2289 | ggcatatgctgattatagaataa | 137842 | 543895 | see also 8268 line | |
| 8270 | LOC63929 | NM_022098.2 | 679 | aagctgagacgaacatggtttgg | 186645 | 580463 | - | |
| 8271 | CTL1 | NM_022109.2 | 1662 | tgggattatgctgctcaactacc | 272063 | 749007 | - | |
| 8272 | CTL1 | NM_022109.2 | 1701 | atgggtgctgcctctgatcatcg | 272064 | 749010 | see also 8271 line | |
| 8273 | CLSPN | NM_022111.2 | 2622 | gagcctaagacactttcctagg | 130063 | 790077 | - | |
| 8274 | PRDM16 | NM_022114.1 | 101 | gtgacggtgacgttgtaaataat | 9848 | 649547 | transcription_regulator | DNA binding |
| 8275 | PRDM16 | NM_022114.1 | 103 | gacggtgacgttgtaaataatat | 9849 | 649548 | see also 8274 line | |
| 8276 | PRDM16 | NM_022114.1 | 109 | gacgttgtaaataatatgtatga | 9852 | 649549 | see also 8274 line | |

Figure 46

| | | | | | | | |
|---|---|---|---|---|---|---|---|
| 8277 | PRDM16 | NM_022114.1 | 1070 | aacgcttcgaatgtgaaaactgc | 9860 | 649558 | see also 8274 line |
| 8278 | FIGNL1 | NM_022116.2 | 625 | aagtggcagtctggattgtcaat | 82370 | 502440 | - |
| 8279 | FIGNL1 | NM_022116.2 | 627 | gttggcagtctggattgtcaataa | 82371 | 502441 | see also 8278 line |
| 8280 | FIGNL1 | NM_022116.2 | 2204 | ctgctgacattgctaccataaca | 82421 | 502485 | see also 8278 line |
| 8281 | SE20-4 | NM_022117.1 | 983 | aagacactttccgctacttgacc | 38932 | 458703 | cell_cycle, signal_transduction, kinase_related |
| 8282 | C13orf10 | NM_022118.3 | 1383 | cccaacttgataggactaacatc | 58267 | 478353 | - |
| 8283 | C13orf10 | NM_022118.3 | 1627 | aagttcctccagaattaaataat | 58272 | 478360 | see also 8282 line |
| 8284 | NPAS3 | NM_022123.1 | 885 | gaacgtgtcccagcaaatcatgg | 254678 | 639240 | signal_transduction, transcription_regulator |
| 8285 | NPAS3 | NM_022123.1 | 931 | ttgcctcccctacgatcaatga | 254679 | 639241 | see also 8284 line |
| 8286 | NPAS3 | NM_022123.1 | 933 | gcctcccctacgatcaatgaag | 254680 | 639242 | see also 8284 line |
| 8287 | NPAS3 | NM_022123.1 | 936 | tcccctacgatcaatgaagtca | 254681 | 639243 | see also 8284 line |
| 8288 | NPAS3 | NM_022123.1 | 942 | tacgatcaatgaagtcagaattg | 254682 | 639244 | see also 8284 line |
| 8289 | CDH23 | NM_022124.2 | 2647 | gtgggccacaaccagaaaaactg | 139969 | 533565 | - |
| 8290 | CDH23 | NM_022124.2 | 5051 | accgtgacatcgggatcaacagt | 139982 | 533587 | see also 8289 line |
| 8291 | RBSK | NM_022128.1 | 88 | ccgacctggtcagtcttacttct | 208524 | 768744 | kinase_related ribokinase |
| 8292 | GOLPH3 | NM_022130.3 | 546 | tggattacgttggctgtatgttaa | 382405 | 687900 | - |
| 8293 | GOLPH3 | NM_022130.3 | 663 | tccaacagggatttcttcttg | 382412 | 687902 | see also 8292 line |
| 8294 | GOLPH3 | NM_022130.3 | 800 | atgtacgggaacgattagcttaaa | 382417 | 687907 | see also 8292 line |
| 8295 | GOLPH3 | NM_022130.3 | 803 | tacgggaacgattagctaaaaac | 382418 | 687908 | see also 8292 line |
| 8296 | CLSTN2 | NM_022131.1 | 1415 | tacatggatggagcaacatatga | 140793 | 546433 | - |
| 8297 | CLSTN2 | NM_022131.1 | 1423 | tggagcaacatatgaaccatacc | 140794 | 546434 | see also 8296 line |
| 8298 | SNX16 | NM_022133.2 | 839 | ttcctccaaaacgctggtttaaa | 270610 | 681918 | - |
| 8299 | SNX16 | NM_022133.2 | 840 | tcctccaaaacgctggtttaaag | 270611 | 681919 | see also 8298 line |
| 8300 | SAMSN1 | NM_022136.2 | 591 | gagctatttcatggcaatgaag | 382437 | 553448 | - |
| 8301 | SMOC1 | NM_022137.2 | 708 | cagaataaaactcctgtagttc | 153078 | 554177 | - |
| 8302 | SMOC1 | NM_022137.2 | 1521 | ggctgcctgggtgttagcaaaga | 153091 | 554194 | see also 8301 line |
| 8303 | EPB41L4A | NM_022140.2 | 300 | aagaattttactgcggaagtttg | 279712 | 661188 | - |
| 8304 | EPB41L4A | NM_022140.2 | 524 | atcaacaactggacctccatatac | 279715 | 661194 | see also 8303 line |
| 8305 | EPB41L4A | NM_022140.2 | 532 | tggacctccatatactttgtatt | 279718 | 661196 | see also 8303 line |
| 8306 | EPB41L4A | NM_022140.2 | 534 | gacctccatatactttgtatttt | 279719 | 661197 | see also 8303 line |
| 8307 | EPB41L4A | NM_022140.2 | 1309 | aagaagtcgaagcaagacttacc | 279731 | 661218 | see also 8303 line |
| 8308 | LRRC4 | NM_022143.3 | 1579 | ctggttaccagccggccatatacc | 382492 | 752573 | structural molecule |
| 8309 | LRRC4 | NM_022143.3 | 2030 | caccaactatcctgaaccttata | 382496 | 752578 | see also 8308 line |

Figure 46

| | | | | | | | | | |
|---|---|---|---|---|---|---|---|---|---|
| 8310 | LRRC4 | NM_022143.3 | 2031 | accactatctctgaaccttatat | 382497 | 752579 | see also 8308 line | | |
| 8311 | LRRC4 | NM_022143.3 | 2033 | cactatctctgaaccttatataa | 382498 | 752580 | see also 8308 line | | |
| 8312 | LRRC4 | NM_022143.3 | 2037 | atctctgaaccttatataattca | 382499 | 752581 | see also 8308 line | | |
| 8313 | TNMD | NM_022144.1 | 738 | ttggatcaatcccactctaatat | 328002 | 691278 | - | - | - |
| 8314 | TNMD | NM_022144.1 | 739 | tggatcaatcccactctaatatc | 328003 | 691279 | see also 8313 line | | |
| 8315 | RRAGC | NM_022157.2 | 519 | aagttaacccagacatgaatttt | 173721 | 570435 | gpcr, apoptosis, transcription_regulator, signal_transduction | - | - |
| 8316 | RRAGC | NM_022157.2 | 1088 | ttccactgtttccgaaaagctat | 173740 | 570449 | see also 8315 line | | |
| 8317 | FN3K | NM_022158.2 | 597 | gtgaagatcccggatctgttttg | 218618 | 606531 | kinase_related | transferase | - |
| 8318 | CARD15 | NM_022162.1 | 1214 | accctgaccgtgtcctgttaacc | 75194 | 497107 | apoptosis | apoptosis regulator | arthritis |
| 8319 | CARD15 | NM_022162.1 | 1217 | ctgaccgtgtcctgttaaccttt | 75195 | 497108 | see also 8318 line | | |
| 8320 | XYLT2 | NM_022167.1 | 1876 | gtccagcgtgcacctgtatttct | 210706 | 599578 | - | - | - |
| 8321 | XYLT2 | NM_022167.1 | 1880 | agcgtgcacctgtatttctatga | 210707 | 599580 | see also 8320 line | | |
| 8322 | MDA5 | NM_022168.2 | 1078 | accatgggaagtgattcagatga | 64116 | 480529 | - | - | melanoma |
| 8323 | MDA5 | NM_022168.2 | 1144 | ctcaggccttaccaaatggaagt | 64118 | 480530 | see also 8322 line | | |
| 8324 | MDA5 | NM_022168.2 | 2762 | atgatttccgagagaagatgatg | 64155 | 480582 | see also 8322 line | | |
| 8325 | MDA5 | NM_022168.2 | 2767 | ttccgagagaagatgatgtataa | 64156 | 480583 | see also 8322 line | | |
| 8326 | ABCG4 | NM_022169.2 | 1343 | tagagatggcggtcttcatgagg | 71159 | 493002 | - | ATP-binding cassette (ABC) transporter | - |
| 8327 | TIA1 | NM_022173.1 | 477 | agcacacagcgttcacaagatca | 47745 | 475839 | apoptosis | poly(A) binding | - |
| 8328 | ICA1 | NM_022307.1 | 837 | aaggttatcaaccatatgaattt | 323802 | 701207 | - | - | diabetes |
| 8329 | ICA1 | NM_022307.1 | 838 | aggttatcaaccatatgaattta | 323803 | 701208 | see also 8328 line | | |
| 8330 | ICA1 | NM_022307.1 | 1087 | acccatagatgaactattagaca | 323816 | 701219 | see also 8328 line | | |
| 8331 | RAB38 | NM_022337.1 | 501 | ttcgtaggatggtttgaaacatc | 110242 | 513026 | signal_transduction | protein transporter | - |
| 8332 | ZFYVE20 | NM_022340.1 | 251 | gaggggttgatccttacatgtgg | 161383 | 555387 | - | - | - |
| 8333 | ZFYVE20 | NM_022340.1 | 336 | tagaattgaccactatgttgtgg | 161384 | 555388 | see also 8332 line | | |
| 8334 | ZFYVE20 | NM_022340.1 | 996 | aagtaagaagatcttaaccttgg | 161404 | 555402 | see also 8332 line | | |
| 8335 | KIF9 | NM_022342.2 | 993 | tggtcctcgtgacaaacatctat | 87853 | 506098 | - | motor | - |
| 8336 | KIF9 | NM_022342.2 | 995 | gtcctcgtgacaaacatctatgg | 87854 | 506099 | see also 8335 line | | |
| 8337 | IFRG15 | NM_022347.1 | 40 | cagtggttccctagtttagaact | 382626 | 712420 | - | - | - |
| 8338 | IFRG15 | NM_022347.1 | 42 | gtggttccctagtttagaactag | 382627 | 712421 | see also 8337 line | | |
| 8339 | IFRG15 | NM_022347.1 | 67 | cactggttgtaccaaactgaact | 382630 | 712422 | see also 8337 line | | |
| 8340 | IFRG15 | NM_022347.1 | 171 | tgctaatagaagccttgttcttc | 382637 | 712424 | see also 8337 line | | |
| 8341 | IFRG15 | NM_022347.1 | 272 | tgggtcctccactggttagaaaa | 382639 | 712426 | see also 8337 line | | |
| 8342 | EFCBP1 | NM_022351.2 | 586 | agcacttgaagacctgaatcttt | 142769 | 542731 | - | - | - |

Figure 46

| | | | | | | | | | |
|---|---|---|---|---|---|---|---|---|---|
| 8343 | OSGEPL1 | NM_022353.1 | 1267 | gtcctcttggagtagacatatca | 184293 | 732515 | - | - | - |
| 8344 | LEPRE1 | NM_022356.2 | 776 | ctgcgaagggccctatgactacg | 222438 | 609826 | - | - | - |
| 8345 | POPDC3 | NM_022361.3 | 1119 | tggcagtgacattgcagataaac | 283068 | 694810 | - | - | - |
| 8346 | DNAJC1 | NM_022365.2 | 254 | gagcggagacctggagttgtttg | 25253 | 458416 | - | - | - |
| 8347 | DNAJC1 | NM_022365.2 | 356 | agcatatcgtaagctttcactaa | 25255 | 458417 | see also 8346 line | | |
| 8348 | DNAJC1 | NM_022365.2 | 467 | acgaaggcagaggtatgatgata | 25258 | 458424 | see also 8346 line | | |
| 8349 | DNAJC1 | NM_022365.2 | 514 | ggcgacagcctgtattctactac | 25259 | 458427 | see also 8346 line | | |
| 8350 | DNAJC1 | NM_022365.2 | 794 | ttggttttgccttacactaaaag | 25272 | 458435 | see also 8346 line | | |
| 8351 | FLJ12287 | NM_022367.2 | 944 | gacagccagcgagtttgacttct | 3733 | 637533 | receptor_acitivity | receptor | - |
| 8352 | FLJ22313 | NM_022373.3 | 723 | aactaaaaacgcatctatctaac | 382754 | 710669 | - | - | - |
| 8353 | FLJ22313 | NM_022373.3 | 848 | gagtatcatatggttcatctagt | 382758 | 710673 | see also 8352 line | | |
| 8354 | FLJ22313 | NM_022373.3 | 1513 | tagtcggtttatcatggtaatgg | 382780 | 710693 | see also 8352 line | | |
| 8355 | ARL6IP2 | NM_022374.1 | 1452 | cggtcatgtttgctatgtatata | 108609 | 519018 | - | - | - |
| 8356 | ABCG8 | NM_022437.2 | 902 | agcctcgctctgacatcttcagg | 71206 | 493077 | - | ATP-binding cassette (ABC) transporter | Sitosterolemia(-)、atherosclerosis |
| 8357 | ABCG8 | NM_022437.2 | 1292 | cgctgatccgtcgtcagatttcc | 71212 | 493084 | see also 8356 line | | |
| 8358 | SLC13A1 | NM_022444.3 | 1617 | tagcaaatccacccaatgctatt | 257282 | 640893 | - | - | - |
| 8359 | SLC13A1 | NM_022444.3 | 1664 | gtcattgacatggttaaagctgg | 257284 | 640895 | see also 8358 line | | |
| 8360 | AD24 | NM_022451.9 | 985 | aggcctggttagccaatacaagt | 382806 | 710973 | - | - | - |
| 8361 | RNF25 | NM_022453.2 | 492 | gtgtcatctgcctctatggtttc | 233353 | 617673 | - | - | - |
| 8362 | NSD1 | NM_022455.3 | 139 | ttcggtaatggtcaatccaattt | 33973 | 462937 | transcription_regulator | - | - |
| 8363 | NSD1 | NM_022455.3 | 140 | tcggtaatggtcaatccaatttt | 33974 | 462938 | see also 8362 line | | |
| 8364 | NSD1 | NM_022455.3 | 141 | cggtaatggtcaatccaattttt | 33975 | 462939 | see also 8362 line | | |
| 8365 | NSD1 | NM_022455.3 | 147 | tggtcaatccaatttttctgagc | 33976 | 462940 | see also 8362 line | | |
| 8366 | NSD1 | NM_022455.3 | 167 | agccacttaatgggtgtactatg | 33977 | 462941 | see also 8362 line | | |
| 8367 | NSD1 | NM_022455.3 | 279 | ggcctccatgatcaatgtagagt | 33978 | 462945 | see also 8362 line | | |
| 8368 | NSD1 | NM_022455.3 | 2718 | ttctgcttctagtcagaatcaca | 34050 | 463017 | see also 8362 line | | |
| 8369 | NSD1 | NM_022455.3 | 4809 | aggaatccatacctgttttgtat | 34108 | 463078 | see also 8362 line | | |
| 8370 | NSD1 | NM_022455.3 | 5087 | tccttgcatctaatagtatcatc | 34114 | 463087 | see also 8362 line | | |
| 8371 | NSD1 | NM_022455.3 | 5100 | tagtatcatctgccctaatcact | 34115 | 463088 | see also 8362 line | | |
| 8372 | NSD1 | NM_022455.3 | 5141 | gccgaaatcatgagcatgttaat | 34116 | 463089 | see also 8362 line | | |
| 8373 | NSD1 | NM_022455.3 | 5304 | gccacactacagggagattgtct | 34119 | 463094 | see also 8362 line | | |
| 8374 | NSD1 | NM_022455.3 | 5681 | ttggcagggtacagatcttcact | 34132 | 463109 | see also 8362 line | | |
| 8375 | NSD1 | NM_022455.3 | 6690 | tggggagatccgtgagtatgtgc | 34158 | 463134 | see also 8362 line | | |
| 8376 | COP1 | NM_022457.4 | 1286 | ttgaccgggattgtgactatttt | 382888 | 703636 | - | - | - |

Figure 46

| | | | | | | | | |
|---|---|---|---|---|---|---|---|---|
| 8377 | COP1 | NM_022457.4 | 1422 | ctgtatcagttggagtagttacc | 382892 | 703641 | see also 8376 line | |
| 8378 | COP1 | NM_022457.4 | 1426 | atcagttggagtagttaccataa | 382893 | 703642 | see also 8376 line | |
| 8379 | COP1 | NM_022457.4 | 1428 | cagttggagtagttaccataaga | 382894 | 703643 | see also 8376 line | |
| 8380 | COP1 | NM_022457.4 | 1466 | gtgattatgaaggcactgttatt | 382896 | 703645 | see also 8376 line | |
| 8381 | COP1 | NM_022457.4 | 1542 | gaggtgttggagtgttgactta | 382897 | 703649 | see also 8376 line | |
| 8382 | XPO4 | NM_022459.3 | 256 | aagtatgttcgggaacagattct | 271663 | 653210 | - | protein transporter |
| 8383 | XPO4 | NM_022459.3 | 1573 | tacctcttagctgatgatactca | 271703 | 653247 | see also 8382 line | |
| 8384 | XPO4 | NM_022459.3 | 1802 | cagatccactcattcactactaagt | 271708 | 653252 | see also 8382 line | |
| 8385 | XPO4 | NM_022459.3 | 2070 | aagagaaaggcaaacttagtaa | 271714 | 653261 | see also 8382 line | |
| 8386 | XPO4 | NM_022459.3 | 2072 | gagaaaggcaaacttagtaatt | 271715 | 653262 | see also 8382 line | |
| 8387 | XPO4 | NM_022459.3 | 3177 | gtgtttgcaccaggctgaatatt | 271757 | 653300 | see also 8382 line | |
| 8388 | XPO4 | NM_022459.3 | 3362 | ttggtggtctccttgtgtaaaa | 271767 | 653305 | see also 8382 line | |
| 8389 | XPO4 | NM_022459.3 | 3363 | tggtggtctccttgtgtaaaat | 271768 | 653306 | see also 8382 line | |
| 8390 | CHST8 | NM_022467.3 | 1969 | acgacttctactacatggattac | 217303 | 605419 | - | |
| 8391 | WIG1 | NM_022470.2 | 981 | ctccaagtggccagttttactgc | 55775 | 476008 | - | |
| 8392 | MPP5 | NM_022474.2 | 1108 | tccacatattcaggccacttttac | 316853 | 681254 | - | |
| 8393 | MPP5 | NM_022474.2 | 1160 | atgcagctagagcccattacaga | 316854 | 681256 | see also 8392 line | |
| 8394 | MPP5 | NM_022474.2 | 1404 | atgtcaatgaggttttgacttg | 316862 | 681261 | see also 8392 line | |
| 8395 | MPP5 | NM_022474.2 | 2219 | ctccggaattcagatttgaaacc | 316878 | 681289 | see also 8392 line | |
| 8396 | MPP5 | NM_022474.2 | 2415 | aagcctatcaggaattgcttagg | 316885 | 681295 | see also 8392 line | |
| 8397 | HHIP | NM_022475.1 | 1219 | cagaggaccagcatctaactact | 383213 | 535736 | - | |
| 8398 | HHIP | NM_022475.1 | 1221 | gaggaccagcatctaactacttg | 383214 | 535737 | see also 8397 line | |
| 8399 | HHIP | NM_022475.1 | 1222 | aggaccagcatctaactacttg | 383215 | 535738 | see also 8397 line | |
| 8400 | HHIP | NM_022475.1 | 1515 | tcgcattccatcccaattacaag | 383221 | 535748 | see also 8397 line | |
| 8401 | NDRG3 | NM_022477.2 | 768 | tactgggccaaaatgataaacaaa | 236879 | 622288 | - | |
| 8402 | FLJ12587 | NM_022480.2 | 231 | ttcgcaagcactgcatgttcacc | 131292 | 532635 | - | |
| 8403 | ARAP3 | NM_022481.4 | 1997 | cacgagtgtctggagtaatgaga | 21469 | 458750 | signal transduction | |
| 8404 | ARAP3 | NM_022481.4 | 2549 | gccccaggatatgttatgtctgg | 21481 | 458755 | see also 8403 line | |
| 8405 | ARAP3 | NM_022481.4 | 4194 | ctgggaatccgcaagaagttaaa | 21495 | 458778 | see also 8403 line | |
| 8406 | ARAP3 | NM_022481.4 | 4195 | tgggaatccgcaagaagttaaag | 21496 | 458779 | see also 8403 line | |
| 8407 | ARAP3 | NM_022481.4 | 4196 | gggaatccgcaagaagttaaagc | 21497 | 458780 | see also 8403 line | |
| 8408 | ARAP3 | NM_022481.4 | 4460 | gaccctgcgcggcgactacacaacc | 21500 | 458787 | see also 8403 line | |
| 8409 | ZNF336 | NM_022482.3 | 161 | ctgccaccagcaagtttttag | 45280 | 542428 | transcription_regulator | |
| 8410 | ZNF336 | NM_022482.3 | 1426 | cacatgctgatttatcataaaag | 45304 | 542450 | see also 8409 line | |
| 8411 | ZNF336 | NM_022482.3 | 1499 | ttgcttctaaggagtacttaaaa | 45307 | 542453 | see also 8409 line | |
| 8412 | APG3 | NM_022488.3 | 170 | cccggtcctcaaggaatccaaagt | 383430 | 724734 | - | Alzheimer disease |

Figure 46

| ID | Gene | Accession | Pos | Sequence | | | Note | Type |
|---|---|---|---|---|---|---|---|---|
| 8413 | APG3 | NM_022488.3 | 274 | gggctacagggaagaattgaaa | 383431 | 724735 | see also 8412 line | |
| 8414 | APG3 | NM_022488.3 | 425 | cggatgggtagatacatatcaca | 383435 | 724742 | see also 8412 line | |
| 8415 | APG3 | NM_022488.3 | 428 | atgggtagatacatatcacaaca | 383436 | 724743 | see also 8412 line | |
| 8416 | APG3 | NM_022488.3 | 772 | atgagcaacggcagcctttaaca | 383446 | 724751 | see also 8412 line | |
| 8417 | APG3 | NM_022488.3 | 1006 | ctgtcattccaacaatagaatat | 383451 | 724756 | see also 8412 line | |
| 8418 | APG3 | NM_022488.3 | 1008 | gtcattccaacaatagaatatga | 383452 | 724757 | see also 8412 line | |
| 8419 | ACTR6 | NM_022496.2 | 84 | tggagcttacaacgccaaaatcg | 286378 | 720105 | - | |
| 8420 | DNMT3A | NM_022552.3 | 2675 | atccactgtgaatgataagctgg | 25452 | 458593 | - | |
| 8421 | ALDH8A1 | NM_022568.2 | 1170 | atgcttcccacggtgataacaga | 226366 | 613428 | - | |
| 8422 | NDST4 | NM_022569.1 | 1682 | ctgcgcactcaggttgcaaattt | 217707 | 605753 | - | |
| 8423 | NDST4 | NM_022569.1 | 1683 | tgcgcactcaggttgcaaatttt | 217708 | 605754 | see also 8422 line | |
| 8424 | HOXC8 | NM_022658.2 | 496 | gaggcgagcgtgtgtgcaatatcc | 5191 | 444830 | transcription_regulator | transcription factor |
| 8425 | HOXC8 | NM_022658.2 | 529 | tcctccgccaacactaacagtag | 5192 | 444831 | see also 8424 line | |
| 8426 | HOXC8 | NM_022658.2 | 555 | aggacaaggccacttaaatcaaa | 5193 | 444832 | see also 8424 line | |
| 8427 | ANAPC1 | NM_022662.1 | 2415 | atgttgagtctcatctttgaac | 383636 | 697650 | - | |
| 8428 | ANAPC1 | NM_022662.1 | 5279 | tactgggaactgctcatagattt | 383675 | 697724 | see also 8427 line | |
| 8429 | NEUROD6 | NM_022728.2 | 755 | gactttacgactggccaaaaact | 33777 | 462656 | transcription_regulator | translation regulator |
| 8430 | NEUROD6 | NM_022728.2 | 1012 | cagggcatggactcttgataat | 33783 | 462665 | see also 8429 line | |
| 8431 | NEUROD6 | NM_022728.2 | 1013 | agggcatggactcttgataatt | 33784 | 462666 | see also 8429 line | |
| 8432 | NEUROD6 | NM_022728.2 | 1019 | tgggactcttgataattccagt | 33788 | 462667 | see also 8429 line | |
| 8433 | NEUROD6 | NM_022728.2 | 1136 | tcctccccaattaacattaatg | 33793 | 462673 | see also 8429 line | |
| 8434 | COPS7B | NM_022730.1 | 404 | atcgtgagcttggcatcaagaat | 383740 | 781928 | - | |
| 8435 | COPS7B | NM_022730.1 | 478 | ccgggaactagaagacttatca | 383743 | 781930 | see also 8434 line | |
| 8436 | LOC64744 | NM_022733.1 | 273 | ctcgaccagtggactcaagaaca | 31012 | 747545 | - | |
| 8437 | LOC64744 | NM_022733.1 | 363 | acctttggcgacctcagataga | 31016 | 747547 | see also 8436 line | |
| 8438 | LOC64744 | NM_022733.1 | 438 | gaccgagtctggacatcaatgc | 31020 | 747549 | see also 8436 line | |
| 8439 | LOC64744 | NM_022733.1 | 574 | cacagtcacctcggaaaagctcc | 31023 | 747553 | see also 8436 line | |
| 8440 | LOC64744 | NM_022733.1 | 881 | tggatccagagcgctcaaatgc | 31028 | 747559 | see also 8436 line | |
| 8441 | ACBD3 | NM_022735.3 | 1608 | tactacagagtcattatactag | 172436 | 569835 | - | |
| 8442 | FLJ14153 | NM_022736.1 | 277 | ctgctgtatgcctgtattcttg | 273510 | 655047 | - | |
| 8443 | FLJ14153 | NM_022736.1 | 294 | ttcttggcccaatgtagttttgt | 273511 | 655048 | see also 8442 line | |
| 8444 | HIPK2 | NM_022740.1 | 131 | tggcctcacatgtgcaagtttc | 84765 | 503489 | | transcription co-repressor |
| 8445 | HIPK2 | NM_022740.1 | 240 | cggctcccagcaaagtgtaca | 84767 | 503490 | see also 8444 line | |
| 8446 | HIPK2 | NM_022740.1 | 528 | cacaagcagcgtgcagatcatcg | 84771 | 503498 | see also 8444 line | |

Figure 46

| | | | | | | | | | |
|---|---|---|---|---|---|---|---|---|---|
| 8447 | HIPK2 | NM_022740.1 | 1390 | gggacaaagacaactaggttttt | 84785 | 503507 | see also 8444 line | | |
| 8448 | HIPK2 | NM_022740.1 | 1766 | gtcgggtgaatatgtatgacacg | 84793 | 503516 | see also 8444 line | | |
| 8449 | HIPK2 | NM_022740.1 | 1928 | atcccgaagtctccatactaaac | 84795 | 503517 | see also 8444 line | | |
| 8450 | HIPK2 | NM_022740.1 | 1929 | tcccgaagtctccatactaaact | 84796 | 503518 | see also 8444 line | | |
| 8451 | SMYD3 | NM_022743.1 | 1174 | cagcctgattgaagatttgattc | 383848 | 728375 | - | - | - |
| 8452 | FLJ13868 | NM_022744.1 | 415 | tggctcgtgaaagattcaactgg | 383852 | 757575 | - | - | - |
| 8453 | FLJ13868 | NM_022744.1 | 1298 | tgctgttcccaaagttcttgaaa | 383860 | 757583 | see also 8452 line | | |
| 8454 | FLJ22059 | NM_022752.2 | 2043 | aggccccacggcgctttgagtgt | 324273 | 789197 | - | - | - |
| 8455 | C20orf81 | NM_022760.3 | 1796 | gagactgatccacacatacaaac | 383992 | 804032 | - | - | - |
| 8456 | C11orf1 | NM_022761.1 | 185 | gcgatgcaccactaatgagaata | 277415 | 713631 | - | - | - |
| 8457 | FLJ22318 | NM_022762.3 | 1337 | cagatgggaaacgcatcatattc | 384009 | 666461 | - | - | - |
| 8458 | FAD104 | NM_022763.2 | 293 | ttcatgtgcctccaggttatatc | 243973 | 631142 | - | - | - |
| 8459 | FAD104 | NM_022763.2 | 296 | atgtgcctccaggttatatctca | 243974 | 631143 | see also 8458 line | | |
| 8460 | FAD104 | NM_022763.2 | 2724 | ctgggaagagccgtgcaataacg | 244038 | 631192 | see also 8458 line | | |
| 8461 | FAD104 | NM_022763.2 | 2886 | tggaccatttagtcagttcatta | 244046 | 631204 | see also 8458 line | | |
| 8462 | FLJ12085 | NM_022771.3 | 42 | gtgagcgggaagattatatatga | 384032 | 717992 | - | - | - |
| 8463 | FLJ12085 | NM_022771.3 | 44 | gagcgggaagattatatatgaac | 384033 | 717993 | see also 8462 line | | |
| 8464 | FLJ12085 | NM_022771.3 | 176 | gagaccattggatgatgcattag | 384039 | 718000 | see also 8462 line | | |
| 8465 | FLJ12085 | NM_022771.3 | 1816 | ttgatgtggaagatatactctgc | 384089 | 718054 | see also 8462 line | | |
| 8466 | OSBPL11 | NM_022776.3 | 560 | agctgggctgttggagtactttg | 303425 | 672204 | - | - | - |
| 8467 | OSBPL11 | NM_022776.3 | 1091 | ggcaactatgaactgcttaaatg | 303444 | 672225 | see also 8466 line | | |
| 8468 | OSBPL11 | NM_022776.3 | 1593 | gagtactaccttacctcatttca | 303452 | 672236 | see also 8466 line | | |
| 8469 | DKFZP434L0117 | NM_022778.2 | 1473 | gtcaaaggtcgtgataaacatat | 384152 | 753227 | receptor_acitivity | - | - |
| 8470 | DKFZP434L0117 | NM_022778.2 | 1477 | aaggtcgtgataaacatatcaat | 384153 | 753228 | see also 8469 line | | |
| 8471 | FLJ13910 | NM_022780.2 | 553 | aaccatttgtggagttaaataga | 384171 | 715369 | - | - | - |
| 8472 | FLJ13910 | NM_022780.2 | 1114 | ttcgtcagcaaacaacagataac | 384187 | 715387 | see also 8471 line | | |
| 8473 | RNF38 | NM_022781.3 | 580 | atcgtctgtctcgacataattcc | 384195 | 786421 | - | - | - |
| 8474 | RNF38 | NM_022781.3 | 583 | gtctgtctcgacataattccatt | 384196 | 786422 | see also 8473 line | | |
| 8475 | RNF38 | NM_022781.3 | 669 | gccttccaccctccgaatgtatc | 384199 | 786423 | see also 8473 line | | |
| 8476 | RNF38 | NM_022781.3 | 951 | cagcacttaccagtaccatatgc | 384205 | 786425 | see also 8473 line | | |
| 8477 | FLJ12428 | NM_022783.1 | 886 | atcggaaatctaccagctttatg | 315870 | 681541 | - | - | - |
| 8478 | FLJ12428 | NM_022783.1 | 887 | tcggaaatctaccagctttatgt | 315871 | 681542 | see also 8477 line | | |
| 8479 | FLJ12428 | NM_022783.1 | 1104 | aggaagacattcacgattgttgg | 315873 | 681545 | see also 8477 line | | |
| 8480 | PPN | NM_022825.1 | 1363 | tggatcttctaccgtctcatagg | 384270 | 539124 | - | - | - |
| 8481 | AXOT | NM_022826.1 | 604 | gtcaccgaagtggtgatttcaca | 384287 | 710593 | - | - | - |

Figure 46

| | | | | | | | | |
|---|---|---|---|---|---|---|---|---|
| 8482 | AXOT | NM_022826.1 | 616 | gtgatttcacaacttcatcatat | 384290 | 710595 | see also 8481 line | |
| 8483 | AXOT | NM_022826.1 | 621 | ttcacaacttcatcatatgttca | 384291 | 710596 | see also 8481 line | |
| 8484 | AXOT | NM_022826.1 | 2120 | acgtgtccgatttattaaccttg | 384325 | 710643 | - | |
| 8485 | SLC13A3 | NM_022829.3 | 1231 | ctctcaagtggtggtttgacttc | 257304 | 640347 | - | |
| 8486 | SLC13A3 | NM_022829.3 | 1646 | tggacacttgctggtcaaagaca | 257311 | 640350 | see also 8485 line | |
| 8487 | USP46 | NM_022832.2 | 237 | ttcggattggtcaatttggaaa | 190015 | 581793 | - | |
| 8488 | USP46 | NM_022832.2 | 416 | tgtcgtcatccaccaaagaagt | 190023 | 581798 | see also 8487 line | |
| 8489 | USP46 | NM_022832.2 | 797 | gtgtagtgaacaaaatattatt | 190033 | 581804 | see also 8487 line | |
| 8490 | FLJ22833 | NM_022837.1 | 537 | aacttaaatgtcgtctttattgt | 384532 | 735404 | - | |
| 8491 | MRPS11 | NM_022839.2 | 773 | ctggaagtgatctcaatcacaga | 285914 | 658677 | | |
| 8492 | FLJ23017 | NM_022840.2 | 788 | tgggtggttactggatcatcttt | 27287 | 791012 | - | |
| 8493 | FLJ23017 | NM_022840.2 | 789 | gggtggttactggatcatcttc | 27288 | 791013 | see also 8492 line | |
| 8494 | PCDH20 | NM_022843.2 | 1360 | gagatccagaagagtaaatacaag | 149882 | 544612 | - | |
| 8495 | PCDH20 | NM_022843.2 | 2443 | atcgcttactggtgaaagtgagt | 149909 | 544646 | see also 8494 line | |
| 8496 | PAPOLG | NM_022894.2 | 416 | tggttcttggtaaattgaacaat | 53265 | 480681 | | polynucleotide adenylyltransferase |
| 8497 | PAPOLG | NM_022894.2 | 419 | ttcttggtaaattgaacaattta | 53266 | 480682 | see also 8496 line | |
| 8498 | PAPOLG | NM_022894.2 | 506 | ttggttggtaaatttcacatt | 53269 | 480684 | see also 8496 line | |
| 8499 | PAPOLG | NM_022894.2 | 714 | tggtattgaaattgatctagtct | 53273 | 480686 | see also 8496 line | |
| 8500 | PAPOLG | NM_022894.2 | 745 | ctggcaatacaaaccatatcaga | 53274 | 480689 | see also 8496 line | |
| 8501 | PAPOLG | NM_022894.2 | 1123 | gtctcggatcctcggtaaatcc | 53297 | 480708 | see also 8496 line | |
| 8502 | PAPOLG | NM_022894.2 | 1218 | cacatcaactcgaacagtaatgg | 53304 | 480712 | see also 8496 line | |
| 8503 | PAPOLG | NM_022894.2 | 1221 | atcaactcgaacagtaatggtag | 53305 | 480713 | see also 8496 line | |
| 8504 | PAPOLG | NM_022894.2 | 1413 | atctaaaatccgtgtacttgttg | 53313 | 480714 | see also 8496 line | |
| 8505 | PAPOLG | NM_022894.2 | 1438 | aacttggaacggaatgaatttat | 53314 | 480716 | see also 8496 line | |
| 8506 | PAPOLG | NM_022894.2 | 1442 | tggaacggaatgaatttattact | 53315 | 480717 | see also 8496 line | |
| 8507 | PAPOLG | NM_022894.2 | 2304 | atctatgcctattccaactattg | 53342 | 480747 | see also 8496 line | |
| 8508 | PAPOLG | NM_022894.2 | 2309 | tgcctattccaactattgataca | 53343 | 480749 | see also 8496 line | |
| 8509 | RANBP17 | NM_022897.2 | 146 | ctcagcaagtgtcaactttatt | 110478 | 652187 | - | protein transporter |
| 8510 | RANBP17 | NM_022897.2 | 446 | gtggaacactgcataataggagt | 110484 | 652199 | see also 8509 line | |
| 8511 | RANBP17 | NM_022897.2 | 1005 | gtcgattttggctgtcgtttaag | 110503 | 652210 | see also 8509 line | |
| 8512 | RANBP17 | NM_022897.2 | 1598 | cagcttatctttaatggatac | 110522 | 652233 | see also 8509 line | |
| 8513 | RANBP17 | NM_022897.2 | 1673 | ttggatcagtttcgtaaaacata | 110529 | 652237 | see also 8509 line | |
| 8514 | RANBP17 | NM_022897.2 | 1674 | tggatcagtttcgtaaaacatat | 110530 | 652238 | see also 8509 line | |
| 8515 | RANBP17 | NM_022897.2 | 2165 | ttgatcgggctggcaagatgatct | 110544 | 652250 | see also 8509 line | |
| 8516 | RANBP17 | NM_022897.2 | 2554 | cagcttggcgtcttcaagttgt | 110552 | 652261 | see also 8509 line | |
| 8517 | RANBP17 | NM_022897.2 | 2573 | ttgtatggggacaaccattttga | 110553 | 652262 | see also 8509 line | |

Figure 46

| 8518 | RANBP17 | NM_022897.2 | 3000 | gtcggaaccagtggtcagtatcc | 110565 | 652268 | see also 8509 line | | |
| 8519 | BCL11B | NM_022898.1 | 467 | tgggggacatcctggtttttata | 384701 | 675287 | transcription_regulator | - | - |
| 8520 | ACTR8 | NM_022899.3 | 1182 | ctgctttaccagtttcgattagg | 286456 | 659204 | - | - | - |
| 8521 | CAS1 | NM_022900.3 | 333 | cgccgctaccgaggcaatgattc | 218416 | 756042 | - | - | - |
| 8522 | CAS1 | NM_022900.3 | 334 | gccgctaccgaggcaatgattcg | 218417 | 756043 | see also 8521 line | | |
| 8523 | CAS1 | NM_022900.3 | 401 | ttggcaacctcacagttgtatga | 218418 | 756046 | see also 8521 line | | |
| 8524 | CAS1 | NM_022900.3 | 403 | ggcaacctcacagttgtatgatg | 218419 | 756047 | see also 8521 line | | |
| 8525 | CAS1 | NM_022900.3 | 413 | cagttgtatgatgcataaataca | 218420 | 756048 | see also 8521 line | | |
| 8526 | CAS1 | NM_022900.3 | 709 | ctgccacatggtccatcaagatt | 218429 | 756057 | see also 8521 line | | |
| 8527 | SLC30A5 | NM_022902.2 | 1786 | gggctgatagtaaaccttattgg | 266865 | 653989 | - | - | - |
| 8528 | SLC30A5 | NM_022902.2 | 1789 | ctgatagtaaaccttattggtat | 266866 | 653990 | see also 8527 line | | |
| 8529 | NDRG4 | NM_022910.1 | 878 | cactacgaccttcctgaagatgg | 310318 | 684303 | - | - | - |
| 8530 | FLJ13110 | NM_022912.1 | 324 | ttggtttccattctattatgaac | 384800 | 801757 | - | - | - |
| 8531 | DKFZp761C169 | NM_022913.1 | 1388 | tcccgttctcgtagcagtatttt | 384813 | 734847 | - | - | - |
| 8532 | DKFZp761C169 | NM_022913.1 | 1389 | cccgttctcgtagcagtattttc | 384814 | 734848 | see also 8531 line | | |
| 8533 | DKFZp761C169 | NM_022913.1 | 1390 | ccgttctcgtagcagtattttcc | 384815 | 734849 | see also 8531 line | | |
| 8534 | DKFZp761C169 | NM_022913.1 | 1933 | tcctgttgacaaacttaatcagc | 384833 | 734877 | see also 8531 line | | |
| 8535 | DKFZp761C169 | NM_022913.1 | 2320 | tcccttaactgaggatgaaatga | 384848 | 734892 | see also 8531 line | | |
| 8536 | FLJ22104 | NM_022918.2 | 557 | tacattttctgcacttaggttca | 384907 | 691473 | - | - | - |
| 8537 | TM6SF1 | NM_023003.1 | 1010 | atgctagaactgcttatgtctac | 284832 | 693494 | - | molecular_function unknown | - |
| 8538 | FLJ21919 | NM_023015.3 | 847 | tgcccaagcccagaagtgttacc | 384986 | 757061 | - | - | - |
| 8539 | FLJ21919 | NM_023015.3 | 1263 | ttcgggaacggttcatggaatgt | 384994 | 757071 | see also 8538 line | | |
| 8540 | FLJ21919 | NM_023015.3 | 2112 | aggatgagagttgctatgacaat | 385022 | 757095 | see also 8538 line | | |
| 8541 | FLJ21919 | NM_023015.3 | 2496 | ctctgtacctaatatttaggaac | 385028 | 757101 | see also 8538 line | | |
| 8542 | FLJ21919 | NM_023015.3 | 2602 | tggctaccacctgctctactacc | 385032 | 757104 | see also 8538 line | | |
| 8543 | FLJ21919 | NM_023015.3 | 2919 | ttctcaacatactcattcagagc | 385038 | 757108 | see also 8538 line | | |
| 8544 | FLJ21919 | NM_023015.3 | 3452 | ttctccctggcggaggaatatga | 385043 | 757113 | see also 8538 line | | |
| 8545 | 13CDNA73 | NM_023037.1 | 1138 | tggtcccaacactggcaatatgc | 67625 | 780538 | - | - | - |
| 8546 | 13CDNA73 | NM_023037.1 | 1729 | ttgggtttacatgattcgaatta | 67642 | 780548 | see also 8545 line | | |

Figure 46

| | | | | | | | | | |
|---|---|---|---|---|---|---|---|---|---|
| 8547 | 13CDNA73 | NM_023037.1 | 9362 | ctgggacagacgggtacttatgc | 67842 | 804256 | see also 8545 line | | |
| 8548 | FLJ13855 | NM_023079.2 | 375 | tactgttgacatgactaagattc | 228872 | 616007 | - | - | - |
| 8549 | FLJ13855 | NM_023079.2 | 485 | acccacctcgggtcaaactgatg | 228874 | 616008 | see also 8548 line | | |
| 8550 | FLJ13855 | NM_023079.2 | 486 | cccacctcgggtcaaactgatga | 228875 | 616009 | see also 8548 line | | |
| 8551 | FLJ13855 | NM_023079.2 | 491 | ctcgggtcaaactgatgacaacg | 228876 | 616010 | see also 8548 line | | |
| 8552 | CAPN10 | NM_023083.1 | 1232 | caggaggctgccggaacaacagc | 187580 | 581345 | - | - | diabetes mellitus、diabetes |
| 8553 | NS3TP2 | NM_023927.1 | 1424 | agcttacagctaacatagtgaaa | 385430 | 722953 | - | - | - |
| 8554 | RINZF | NM_023929.2 | 2696 | tgccctcattgtagctatgtagc | 135257 | 797645 | - | - | - |
| 8555 | EBF | NM_024007.2 | 1031 | tcggaaggtacgccctcttatct | 25683 | 458869 | transcription_regulator | - | - |
| 8556 | EBF | NM_024007.2 | 2015 | aacagcctgcaagcgatatctgg | 25696 | 458882 | see also 8555 line | | |
| 8557 | HOXB4 | NM_024015.2 | 496 | tacccctggatgcgcaaagttca | 5120 | 444754 | transcription_regulator | transcription factor | - |
| 8558 | HOXB9 | NM_024017.3 | 97 | ctgggacgcttagcagctattat | 5135 | 444770 | transcription_regulator | transcription factor | - |
| 8559 | HOXB9 | NM_024017.3 | 98 | tgggacgcttagcagctattatg | 5136 | 444771 | see also 8558 line | | |
| 8560 | HOXB9 | NM_024017.3 | 150 | gacgcgcctccagccaagtttcc | 5138 | 444773 | see also 8558 line | | |
| 8561 | HOXB9 | NM_024017.3 | 478 | agggcacgcccgagtacagtttg | 5140 | 444774 | see also 8558 line | | |
| 8562 | HOXB9 | NM_024017.3 | 479 | gggcacgcccgagtacagtttgg | 5141 | 444775 | see also 8558 line | | |
| 8563 | HOXB9 | NM_024017.3 | 544 | ccggctacggggacaataaaatt | 5150 | 444779 | see also 8558 line | | |
| 8564 | HOXB9 | NM_024017.3 | 545 | cggctacggggacaataaaattt | 5151 | 444780 | see also 8558 line | | |
| 8565 | HOXB9 | NM_024017.3 | 546 | ggctacggggacaataaaatttg | 5152 | 444781 | see also 8558 line | | |
| 8566 | GDAP1L1 | NM_024034.3 | 1118 | ctgggtgggatgggctactttgc | 385538 | 754743 | - | - | - |
| 8567 | MGC2803 | NM_024038.2 | 433 | agccctcaagacgggaatagtag | 385578 | 727130 | - | - | - |
| 8568 | MGC2803 | NM_024038.2 | 434 | gccctcaagacgggaatagtagc | 385579 | 727131 | see also 8567 line | | |
| 8569 | MGC2803 | NM_024038.2 | 473 | gaggatgaggtattaacaagtaa | 385583 | 727132 | see also 8567 line | | |
| 8570 | DDX50 | NM_024045.1 | 566 | aggtcgaggggtaacatatctct | 59384 | 483314 | - | - | - |
| 8571 | DDX50 | NM_024045.1 | 568 | gtcgaggggtaacatatctcttt | 59385 | 483315 | see also 8570 line | | |
| 8572 | DDX50 | NM_024045.1 | 569 | tcgaggggtaacatatctctttc | 59386 | 483316 | see also 8570 line | | |
| 8573 | DDX50 | NM_024045.1 | 578 | aacatatctcttcctattcaag | 59387 | 483317 | see also 8570 line | | |
| 8574 | DDX50 | NM_024045.1 | 838 | atggtggaacatcatatcaaagc | 59392 | 483324 | see also 8570 line | | |
| 8575 | DDX50 | NM_024045.1 | 1100 | gtgggtatacaaagttgcaaaaa | 59402 | 483334 | see also 8570 line | | |
| 8576 | DDX50 | NM_024045.1 | 1321 | tggccatgaatccacacataaaa | 59407 | 483341 | see also 8570 line | | |
| 8577 | DDX50 | NM_024045.1 | 2070 | ctctcagtgccagccaaattacc | 59423 | 483365 | see also 8570 line | | |
| 8578 | MGC8407 | NM_024046.2 | 1017 | ctgcttctgataagaacatcaag | 91441 | 495865 | kinase_related | - | - |
| 8579 | MGC3048 | NM_024052.3 | 844 | ctccatagaggggctactactacc | 385647 | 718669 | - | - | - |

Figure 46

| 8580 | Nup37 | NM_024057.2 | 626 | tggaacaatccggttttatgatc | 269534 | 651991 | - | - | - |
|------|-------|-------------|-----|------------------------|--------|--------|---|---|---|
| 8581 | C7orf26 | NM_024067.2 | 182 | cgcagcccaagagactgaattcc | 385712 | 778072 | - | - | - |
| 8582 | ALG8 | NM_024079.2 | 211 | tggtattatgaggcaacttcaga | 213199 | 815897 | - | transferase、transferring glycosyl groups | - |
| 8583 | ALG8 | NM_024079.2 | 846 | ttcccgactctttcctttcaaga | 213224 | 815917 | see also 8582 line | | |
| 8584 | ALG8 | NM_024079.2 | 848 | cccgactctttcctttcaagagg | 213225 | 815918 | see also 8582 line | | |
| 8585 | ALG8 | NM_024079.2 | 903 | ctgggctttgtacaatgctttgg | 213226 | 815919 | see also 8582 line | | |
| 8586 | TRPM8 | NM_024080.3 | 1730 | ctcttcatctgggccattcttca | 252242 | 638329 | receptor_acitivity | - | - |
| 8587 | MGC3329 | NM_024086.2 | 435 | ctccgaagaggaattgacatagg | 385814 | 722455 | - | - | - |
| 8588 | ELOVL6 | NM_024090.1 | 842 | ttccgagtctcccggaagtttgc | 326448 | 603722 | - | - | - |
| 8589 | MGC5509 | NM_024093.1 | 606 | ttgtccctgttggaactactcc | 385970 | 748436 | - | - | - |
| 8590 | MAPKAP1 | NM_024117.2 | 312 | ttggacaatccaactatcattct | 297377 | 795268 | - | RAS interactor | - |
| 8591 | MAPKAP1 | NM_024117.2 | 315 | gacaatccaactatcattctagc | 297378 | 795269 | see also 8590 line | | |
| 8592 | MAPKAP1 | NM_024117.2 | 327 | atcattctagctcatattcgaca | 297379 | 795271 | see also 8590 line | | |
| 8593 | MAPKAP1 | NM_024117.2 | 378 | atgtgtgagatggttctcattga | 297380 | 795272 | see also 8590 line | | |
| 8594 | MAPKAP1 | NM_024117.2 | 549 | cgctcaaacacagctcaaagatt | 297388 | 795280 | see also 8590 line | | |
| 8595 | MAPKAP1 | NM_024117.2 | 983 | cagtgcctactgcctgcatattg | 297400 | 795291 | see also 8590 line | | |
| 8596 | MAPKAP1 | NM_024117.2 | 1324 | tgcttagcagccaccattacaag | 297410 | 795306 | see also 8590 line | | |
| 8597 | MAPKAP1 | NM_024117.2 | 1335 | caccattacaagtcattcaaagt | 297411 | 795308 | see also 8590 line | | |
| 8598 | MAPKAP1 | NM_024117.2 | 1422 | gagatagaccctgttacgaatca | 297414 | 795310 | see also 8590 line | | |
| 8599 | MAPKAP1 | NM_024117.2 | 1428 | gaccctgttacgaatcagaaagc | 297416 | 795311 | see also 8590 line | | |
| 8600 | MAPKAP1 | NM_024117.2 | 1443 | cagaaagccagcactaagttttg | 297417 | 795312 | see also 8590 line | | |
| 8601 | MAPKAP1 | NM_024117.2 | 1454 | cactaagttttggattaagcaga | 297420 | 795314 | see also 8590 line | | |
| 8602 | MAPKAP1 | NM_024117.2 | 1627 | ttgtgctcaaggttaactacatc | 297427 | 795320 | see also 8590 line | | |
| 8603 | C20orf7 | NM_024120.2 | 587 | tggaggcgacacactctatgaac | 386120 | 728083 | - | - | - |

Figure 46

| | | | | | | | | | |
|---|---|---|---|---|---|---|---|---|---|
| 8604 | C20orf7 | NM_024120.2 | 590 | aggcgacacactctatgaacttc | 386121 | 728084 | see also 8603 line | | |
| 8605 | MGC3067 | NM_024295.3 | 649 | gggttatccttggattcaactat | 386193 | 714915 | - | - | - |
| 8606 | MGC3067 | NM_024295.3 | 779 | atccacacctcagtttttgtacc | 386201 | 714916 | see also 8605 line | | |
| 8607 | MMP28 | NM_024302.2 | 618 | accaaaatgaggcgtaagaaacg | 158404 | 558919 | - | - | - |
| 8608 | PLEKHF1 | NM_024310.2 | 814 | tacggagcgccaggaatggatta | 159104 | 554974 | - | - | - |
| 8609 | ET | NM_024311.2 | 338 | tggaaatgtggcgcaaactgtca | 279758 | 801965 | - | - | - |
| 8610 | ET | NM_024311.2 | 355 | ctgtcatcaggagcttaaatagg | 279759 | 801967 | see also 8609 line | | |
| 8611 | C20orf121 | NM_024331.2 | 770 | ttccccattcggataaaagcagt | 273306 | 655495 | - | - | - |
| 8612 | MGC3222 | NM_024334.1 | 634 | accgagagattggccacaaaaac | 386307 | 735910 | - | - | - |
| 8613 | MGC10765 | NM_024345.1 | 444 | ccgatggacgaatgatttcttcc | 386356 | 768567 | kinase_related | - | - |
| 8614 | MGC10765 | NM_024345.1 | 561 | tccgttctctgtactctcataat | 386357 | 768572 | see also 8613 line | | |
| 8615 | MGC10765 | NM_024345.1 | 562 | ccgttctctgtactctcataatg | 386358 | 768573 | see also 8613 line | | |
| 8616 | MGC10765 | NM_024345.1 | 565 | ttctctgtactctcataatgatg | 386359 | 768574 | see also 8613 line | | |
| 8617 | NOTCH2 | NM_024408.2 | 1902 | atcacccgaatggctatgaatgc | 149157 | 538121 | receptor_acitivity、transcription_regulator、apoptosis、cell_cycle、signal_transduction | translation regulator | - |
| 8618 | PLA2G4A | NM_024420.1 | 1785 | cagtgggctcacatttaacctgc | 199522 | 576934 | - | lysophospholipase | - |
| 8619 | ATP10A | NM_024490.2 | 1034 | gtcatctacgcaggacatgaaac | 72363 | 494062 | - | plasma membrane cation-transporting ATPase | - |
| 8620 | HIVEP3 | NM_024503.1 | 5198 | gccttgaacttaccatggaaacc | 4890 | 701419 | - | - | - |
| 8621 | HIVEP3 | NM_024503.1 | 5696 | atgtcagttggtgctatttaaac | 4895 | 701426 | see also 8620 line | | |
| 8622 | HIVEP3 | NM_024503.1 | 5703 | ttggtgctatttaaactacatta | 4896 | 701427 | see also 8620 line | | |
| 8623 | HIVEP3 | NM_024503.1 | 5767 | tacgctggttggtgcataagttt | 4900 | 701428 | see also 8620 line | | |
| 8624 | HIVEP3 | NM_024503.1 | 5775 | ttggtgcataagtttgtacaacc | 4901 | 701432 | see also 8620 line | | |
| 8625 | GCC1 | NM_024523.5 | 2138 | ttcagggaccgcacactgaaact | 321449 | 686101 | - | - | - |
| 8626 | GCC1 | NM_024523.5 | 2140 | cagggaccgcacactgaaactgg | 321450 | 686102 | see also 8625 line | | |
| 8627 | C1orf28 | NM_024529.3 | 185 | tagcgtcctgcgacagtacaaca | 386716 | 759027 | - | - | - |
| 8628 | C1orf28 | NM_024529.3 | 325 | gagagtactacacattggattcc | 386719 | 759032 | see also 8627 line | | |
| 8629 | C1orf28 | NM_024529.3 | 445 | aagatctacttggatatctcaat | 386722 | 759042 | see also 8627 line | | |
| 8630 | C1orf28 | NM_024529.3 | 600 | gagtgtgtgcgccttgataaaga | 386730 | 759047 | see also 8627 line | | |
| 8631 | C1orf28 | NM_024529.3 | 602 | gtgtgtgcgccttgataaagaga | 386731 | 759048 | see also 8627 line | | |

Figure 46

| | | | | | | | |
|---|---|---|---|---|---|---|---|
| 8632 | C1orf28 | NM_024529.3 | 604 | gtgtgcgccttgataaagagaga | 386732 | 759049 | see also 8627 line |
| 8633 | C1orf28 | NM_024529.3 | 789 | gccttaaacagaggagttttgt | 386741 | 759054 | see also 8627 line |
| 8634 | RNF128 | NM_024539.3 | 1169 | ccgcatcatctgatatgcttca | 191599 | 585141 | - |
| 8635 | FLJ13114 | NM_024541.1 | 302 | cccttatgattgtgaacctatt | 386925 | 812633 | - |
| 8636 | SAP130 | NM_024545.2 | 2881 | cagtgacgtccgggtcaaagagg | 387000 | 781806 | - |
| 8637 | C13orf7 | NM_024546.2 | 1712 | aacagcaagagccctgtaattaa | 387057 | 721083 | - |
| 8638 | C13orf7 | NM_024546.2 | 1714 | cagcaagagccctgtaattaacg | 387058 | 721084 | see also 8637 line |
| 8639 | C13orf7 | NM_024546.2 | 1722 | gccctgtaataacgtttttaag | 387059 | 721085 | see also 8637 line |
| 8640 | FLJ23047 | NM_024548.2 | 1090 | aacagtaatgatgatcagttatt | 387106 | 584299 | - |
| 8641 | FLJ20097 | NM_024553.2 | 462 | cagcctgcttatgtaaaggaact | 387181 | 715166 | - |
| 8642 | FLJ20097 | NM_024553.2 | 563 | tgcaaaggaaggtttactcaag | 387187 | 715169 | see also 8641 line |
| 8643 | FLJ21963 | NM_024560.2 | 468 | gggataagattgctatcatcat | 236350 | 813907 | - |
| 8644 | FLJ22054 | NM_024561.3 | 1601 | atgcttcgagcaaatatgataaa | 387282 | 719471 | - |
| 8645 | FLJ21616 | NM_024567.1 | 161 | gccttgcactaccaatcaaatg | 3754 | 443785 | transcription regulator |
| 8646 | FLJ21616 | NM_024567.1 | 370 | ttcttgccaatcggaggatttcc | 3758 | 443790 | see also 8645 line |
| 8647 | FLJ21616 | NM_024567.1 | 805 | aagtatataattggtttgctaac | 3774 | 443801 | see also 8645 line |
| 8648 | FLJI13119 | NM_024580.3 | 599 | tacttctaaagtcctagaagaaa | 574438 | 477259 | - |
| 8649 | OSBPL9 | NM_024586.3 | 862 | tacagtccagagttcttcttact | 303784 | 672408 | - |
| 8650 | OSBPL9 | NM_024586.3 | 864 | cagtccagagttcttcttactcc | 303785 | 672409 | see also 8649 line |
| 8651 | OSBPL9 | NM_024586.3 | 938 | tccccaaagcgcttaatagattc | 303788 | 672413 | see also 8649 line |
| 8652 | OSBPL9 | NM_024586.3 | 939 | ccccaaagcgcttaatagattct | 303789 | 672414 | see also 8649 line |
| 8653 | OSBPL9 | NM_024586.3 | 1125 | aggagcacagagcgttatcatg | 303797 | 672421 | see also 8649 line |
| 8654 | OSBPL9 | NM_024586.3 | 1187 | aaggtagttcttccaacgttat | 303801 | 672426 | see also 8649 line |
| 8655 | OSBPL9 | NM_024586.3 | 1308 | tggttcaggttgtgaaatggtac | 303806 | 672434 | see also 8649 line |
| 8656 | FLJ22386 | NM_024589.1 | 354 | ggcggatgtgaacctgaagatgc | 387616 | 746972 | - |
| 8657 | PANK3 | NM_024594.2 | 566 | cccaggacctgcctactttatc | 95638 | 511221 | - |
| 8658 | PANK3 | NM_024594.2 | 895 | gtccattccaaagacaacactataa | 95649 | 511230 | see also 8657 line |
| 8659 | FLJI11588 | NM_024603.1 | 879 | cggagttacggagcactttcagt | 387744 | 723420 | - |
| 8660 | FLJ11588 | NM_024603.1 | 906 | aagcaaatacgtcgtcctattac | 387745 | 723422 | see also 8659 line |
| 8661 | FLJ11588 | NM_024603.1 | 907 | agcaaatacgtcgtcctattac | 387746 | 723423 | see also 8659 line |
| 8662 | FLJ11588 | NM_024603.1 | 937 | tgcgcctgcatggacaagtata | 387747 | 723424 | see also 8659 line |
| 8663 | FLJ11588 | NM_024603.1 | 988 | aagcgggatttggttgatgagg | 387749 | 723426 | see also 8659 line |
| 8664 | FLJ21908 | NM_024604.1 | 197 | gagaatttacctcctattcgaaa | 387757 | 606373 | - |
| 8665 | FLJ20896 | NM_024605.2 | 478 | agcctctcatgacctatgagtta | 387802 | 532191 | - |
| 8666 | FLJ20896 | NM_024605.2 | 479 | gcctctcatgacctatgagttac | 387803 | 532192 | see also 8665 line |
| 8667 | HSPBAP1 | NM_024610.3 | 644 | gtcatctggactcctatggttgt | 387845 | 785532 | - |

Figure 46

| | | | | | | | | |
|---|---|---|---|---|---|---|---|---|
| 8668 | FLJ21308 | NM_024615.2 | 2329 | agcatatcatttggttacttcagg | 388040 | 728750 | | - |
| 8669 | FLJ12604 | NM_024621.1 | 2374 | acgtgtttggcttcagtgaaaca | 388116 | 734309 | | - |
| 8670 | FLJ12604 | NM_024621.1 | 2795 | tagagaagtaaccacatatctgt | 388123 | 734320 | | see also 8669 line |
| 8671 | SMC6L1 | NM_024624.2 | 1212 | atgcacgagcaccagaatgtatg | 100856 | 515003 | | - |
| 8672 | ZDHHC14 | NM_024630.2 | 1580 | gtgcattcagagcaccaaattcg | 388289 | 759975 | | - |
| 8673 | FLJ23342 | NM_024631.1 | 1514 | agccattggataccccttaaaat | 388323 | 761347 | | - |
| 8674 | FLJ23342 | NM_024631.1 | 1515 | gccattggatacccctaaaatt | 388324 | 761348 | | see also 8673 line |
| 8675 | GALNT12 | NM_024642.2 | 454 | ctccttcggacagtttacagtgt | 218714 | 777581 | | - |
| 8676 | C14orf169 | NM_024644.1 | 1416 | gccccgagacttcatggattaca | 388487 | 713888 | | - |
| 8677 | FLJ13842 | NM_024645.1 | 543 | gtggtcgcatctcctatcaaag | 51678 | 472971 | | - |
| 8678 | FLJ13842 | NM_024645.1 | 547 | tcgcatctcctatcaaagaaga | 51681 | 472972 | | see also 8677 line |
| 8679 | FLJ22329 | NM_024656.2 | 548 | tgctcatcgtgagaacaagaacg | 303177 | 671492 | | - |
| 8680 | FLJ22329 | NM_024656.2 | 1322 | acctgcgtttgagatcttcttc | 303182 | 671499 | | see also 8679 line |
| 8681 | ZCWCC2 | NM_024657.2 | 378 | gagcctggccaatgtagaatatg | 388609 | 489501 | | - |
| 8682 | ZCWCC2 | NM_024657.2 | 564 | aggagtaattggagtcattgagt | 388620 | 489507 | | see also 8681 line |
| 8683 | ZCWCC2 | NM_024657.2 | 573 | tggagtcattgagtgcaatttcc | 388622 | 489508 | | see also 8681 line |
| 8684 | ZCWCC2 | NM_024657.2 | 577 | gtcattgagtgcaatttcctaaa | 388623 | 489509 | | see also 8681 line |
| 8685 | ZCWCC2 | NM_024657.2 | 603 | tgcctacaacaaacaagacttg | 388624 | 489511 | | see also 8681 line |
| 8686 | ZCWCC2 | NM_024657.2 | 604 | gcctacaacaaacaagacttga | 388625 | 489512 | | see also 8681 line |
| 8687 | IPO4 | NM_024658.2 | 1424 | gccctacttccggagcttatgg | 146763 | 651280 | | - |
| 8688 | IPO4 | NM_024658.2 | 1429 | acttccggagcttatggaatgc | 146765 | 651281 | | see also 8687 line |
| 8689 | FLJ11753 | NM_024659.2 | 1350 | cagccctacatggtaaattcagg | 388697 | 777129 | | - |
| 8690 | FLJ10774 | NM_024662.1 | 1739 | tagagataccctctttgctacc | 82671 | 602267 | molecular_function unknown | - |
| 8691 | FLJ10774 | NM_024662.1 | 2892 | aaacggatcatccgcaaagttgt | 82683 | 602286 | | see also 8690 line |
| 8692 | FLJ10774 | NM_024662.1 | 2894 | ccggatcatccgcaaagttgtga | 82684 | 602287 | | see also 8690 line |
| 8693 | IRA1 | NM_024665.2 | 243 | atgaggtcaacttcttggtatat | 388734 | 742466 | | - |
| 8694 | IRA1 | NM_024665.2 | 913 | atctctagattggaatagtgaag | 388755 | 742488 | | see also 8693 line |
| 8695 | SUV39H2 | NM_024670.3 | 767 | gtcccaataggattgtacaaaaa | 19145 | 451350 | transcription_regulator | - |
| 8696 | FLJ14103 | NM_024689.1 | 236 | tcctcggtctgataaatgcaat | 389307 | 786701 | | - |
| 8697 | FLJ21069 | NM_024692.3 | 2235 | tgggtgacaagcgctatttcacc | 389374 | 741752 | | - |
| 8698 | ZFH4 | NM_024721.2 | 201 | acggatgagcgcaaaagtgaagc | 13981 | 442102 | | - |
| 8699 | ZFH4 | NM_024721.2 | 638 | ttcatccctcgggaaaccattta | 13991 | 442114 | | see also 8698 line |
| 8700 | ZFH4 | NM_024721.2 | 675 | ttcccaaataccteagccattagc | 13993 | 442115 | | see also 8698 line |
| 8701 | ZFH4 | NM_024721.2 | 704 | tggtcctgttgcacagtttcc | 13994 | 442116 | | see also 8698 line |
| 8702 | ZFH4 | NM_024721.2 | 714 | ttgcacagtttcgtgtctcatga | 13995 | 442117 | | see also 8698 line |

Figure 46

| | | | | | | |
|---|---|---|---|---|---|---|
| 8703 | ZFH4 | NM_024721.2 | 992 | atggtctccgccataatacagg | 14004 | see also 8698 line |
| 8704 | ZFH4 | NM_024721.2 | 1114 | atccaacctccgccggtttatgg | 14008 | see also 8698 line |
| 8705 | ZFH4 | NM_024721.2 | 1133 | atggagcgctttcatgttgaaa | 14009 | see also 8698 line |
| 8706 | ZFH4 | NM_024721.2 | 1134 | tggagcgctttcatgttgaaaa | 14010 | see also 8698 line |
| 8707 | ZFH4 | NM_024721.2 | 1288 | tagtgaacacccaattacctct | 14013 | see also 8698 line |
| 8708 | ZFH4 | NM_024721.2 | 2140 | tccgttgtgaggtttgtaactac | 14038 | see also 8698 line |
| 8709 | ZFH4 | NM_024721.2 | 2632 | tcggagagctgtcaccttatatc | 14048 | see also 8698 line |
| 8710 | ZFH4 | NM_024721.2 | 2988 | accaaacagtgtgataaattacg | 14059 | see also 8698 line |
| 8711 | ZFH4 | NM_024721.2 | 4210 | tccgtacattccaggctttaaaa | 14087 | see also 8698 line |
| 8712 | ZFH4 | NM_024721.2 | 4461 | aagcggaccttacctttagaaa | 14093 | see also 8698 line |
| 8713 | ZFH4 | NM_024721.2 | 4463 | gcggaacttacctttagaaaag | 14094 | see also 8698 line |
| 8714 | ZFH4 | NM_024721.2 | 6872 | cccaggatctttgacttgatta | 14154 | see also 8698 line |
| 8715 | ZFH4 | NM_024721.2 | 8038 | caccaagcccctttaatatccacc | 14177 | see also 8698 line |
| 8716 | ZFH4 | NM_024721.2 | 10467 | aagtcctggcctaatatccttt | 14240 | see also 8698 line |
| 8717 | C7orf10 | NM_024728.1 | 1136 | atccaactgtggggaagatttcc | 304744 | - |
| 8718 | DIBD1 | NM_024740.1 | 977 | atggacctgatctttatggtaca | 389793 | - |
| 8719 | DIBD1 | NM_024740.1 | 1523 | tgggaaagagtggtatcgattt | 389807 | see also 8718 line |
| 8720 | DIBD1 | NM_024740.1 | 1524 | gggaaaagagtggtatcgattc | 389808 | see also 8718 line |
| 8721 | FLJ13063 | NM_024742.1 | 997 | ccctttggtgaccattcttcagt | 131301 | - |
| 8722 | FLJ13063 | NM_024742.1 | 1345 | ctgtgctgagccagctaagtctgg | 131302 | see also 8721 line |
| 8723 | ALS2CR8 | NM_024744.12 | 1355 | taccgtggctactgtgtaagtga | 219205 | - |
| 8724 | ALS2CR8 | NM_024744.12 | 2124 | tccatgaggtacagaaatcctg | 219224 | see also 8723 line |
| 8725 | SHCBP1 | NM_024745.2 | 255 | gcgattcagagcctatccaagatt | 389821 | - |
| 8726 | SHCBP1 | NM_024745.2 | 261 | cagagcctatcaagattacattt | 389822 | see also 8725 line |
| 8727 | SHCBP1 | NM_024745.2 | 263 | gagcctatcaagattacatttta | 389823 | see also 8725 line |
| 8728 | SHCBP1 | NM_024745.2 | 361 | cgggcagtctggcacactaatgt | 389825 | see also 8725 line |
| 8729 | SHCBP1 | NM_024745.2 | 363 | ggcagtctggcacactaatgtgt | 389826 | see also 8725 line |
| 8730 | SHCBP1 | NM_024745.2 | 365 | cagtctggcacactaatgtgttc | 389827 | see also 8725 line |
| 8731 | SHCBP1 | NM_024745.2 | 746 | ttgttgactaccacaatcgttg | 389834 | see also 8725 line |
| 8732 | FLJ13213 | NM_024755.1 | 529 | aagaacggaaacgcttacagaga | 26958 | - |
| 8733 | FLJ13213 | NM_024755.1 | 601 | tggaaacgcgaacgcttggaaagg | 26959 | see also 8732 line |
| 8734 | FLJ13213 | NM_024755.1 | 620 | aagggaacgcgcattcgtattgaac | 26961 | see also 8732 line |
| 8735 | FLJ13213 | NM_024755.1 | 621 | agggaacgcgcattcgtattgaaca | 26962 | see also 8732 line |
| 8736 | FLJ13213 | NM_024755.1 | 778 | atccttactgcaggcgagaataaa | 26968 | see also 8732 line |
| 8737 | FLJ13213 | NM_024755.1 | 779 | tccttactgcaggcgagaataaaa | 26969 | see also 8732 line |

Figure 46

| | | | | | | | | | |
|---|---|---|---|---|---|---|---|---|---|
| 8738 | FLJ13213 | NM_024755.1 | 785 | ctggagcgagaataaaaagttgt | 26970 | 454081 | see also 8732 line | | |
| 8739 | FLJ13955 | NM_024759.1 | 762 | cagcaagaacagtacagtattac | 390146 | 756597 | - | - | - |
| 8740 | FLJ13955 | NM_024759.1 | 763 | agcaagaacagtacagtattacc | 390147 | 756598 | see also 8739 line | | |
| 8741 | C14orf161 | NM_024764.1 | 1407 | ttcaaacagtgtgctaatgtttc | 390264 | 782462 | - | - | - |
| 8742 | PIP5K2C | NM_024779.3 | 618 | ttgtgatgcgcaatatgtttagc | 207671 | 597708 | kinase_related | - | - |
| 8743 | PIP5K2C | NM_024779.3 | 697 | agccagcgataaggaaaaggtta | 207673 | 597712 | see also 8742 line | | |
| 8744 | TMC5 | NM_024780.3 | 2252 | caccctcattgtgctaatcatca | 390618 | 737183 | - | - | - |
| 8745 | TMC5 | NM_024780.3 | 2262 | gtgctaatcatcacctatctta | 390619 | 737185 | see also 8744 line | | |
| 8746 | ZBTB3 | NM_024784.2 | 1632 | cacatccgcaaggctcacaatga | 43878 | 531326 | transcription_regulator | - | - |
| 8747 | RNF122 | NM_024787.2 | 418 | tggtgtaacgggtgtttctgtgg | 390663 | 785695 | - | - | - |
| 8748 | FLJ22624 | NM_024808.2 | 144 | atgcaaataacaccagaaactcc | 390837 | 787250 | - | - | - |
| 8749 | FLJ22624 | NM_024808.2 | 374 | ttctcgaatagataaagatgtgg | 390845 | 787260 | see also 8748 line | | |
| 8750 | FLJ12529 | NM_024811.2 | 164 | ttcaacaatacagatcagattga | 61334 | 479938 | - | - | - |
| 8751 | FLJ12529 | NM_024811.2 | 290 | aacaacaagacccctgcaattct | 61335 | 479939 | see also 8750 line | | |
| 8752 | FLJ12529 | NM_024811.2 | 296 | aagaccctgcaattctgtatac | 61336 | 479940 | see also 8750 line | | |
| 8753 | FLJ12529 | NM_024811.2 | 638 | gtcccaagaggggaatacctcc | 61342 | 479955 | see also 8750 line | | |
| 8754 | FLJ12529 | NM_024811.2 | 730 | ctcatctgctcgtgtggataagc | 61344 | 479960 | see also 8750 line | | |
| 8755 | FLJ12529 | NM_024811.2 | 1008 | tggggcctccaccagatacttac | 61347 | 479962 | see also 8750 line | | |
| 8756 | FLJ12529 | NM_024811.2 | 1125 | agcgaaacagagcaatttccagc | 61350 | 479966 | see also 8750 line | | |
| 8757 | CBLL1 | NM_024814.1 | 579 | atcaaccatcgccatatgagagc | 391022 | 750457 | - | - | - |
| 8758 | CBLL1 | NM_024814.1 | 848 | atcggtcagtcaggaaacctttc | 391025 | 750472 | see also 8757 line | | |
| 8759 | CBLL1 | NM_024814.1 | 849 | tcggtcagtcaggaaacctttcg | 391026 | 750473 | see also 8757 line | | |
| 8760 | CBLL1 | NM_024814.1 | 854 | cagtcaggaaacctttcgtattt | 391027 | 750474 | see also 8757 line | | |
| 8761 | CBLL1 | NM_024814.1 | 858 | caggaaacctttcgtatttcaac | 391028 | 750476 | see also 8757 line | | |
| 8762 | CBLL1 | NM_024814.1 | 1109 | aggtactcctcacttggtatata | 391029 | 750482 | see also 8757 line | | |
| 8763 | CBLL1 | NM_024814.1 | 1199 | tgcccagatgccaccttatatga | 391032 | 750485 | see also 8757 line | | |
| 8764 | UBE1DC1 | NM_024818.2 | 488 | aggtagtgtgactgctgaaatgc | 220948 | 607946 | - | - | - |
| 8765 | KIAA1608 | NM_024820.1 | 471 | ttcgggttctgccgcttatcttc | 391078 | 760452 | - | - | - |
| 8766 | KIAA1608 | NM_024820.1 | 472 | tcgggttctgccgcttatcttca | 391079 | 760453 | see also 8765 line | | |
| 8767 | KIAA1608 | NM_024820.1 | 474 | gggttctgccgcttatcttcagg | 391080 | 760454 | see also 8765 line | | |
| 8768 | KIAA1608 | NM_024820.1 | 954 | tacctcataggaatccatttaag | 391091 | 760466 | see also 8765 line | | |
| 8769 | KIAA1608 | NM_024820.1 | 955 | acctcataggaatccatttaagt | 391092 | 760467 | see also 8765 line | | |
| 8770 | KIAA1608 | NM_024820.1 | 1528 | atcatgcaaaaatgggaataaaa | 391106 | 760482 | see also 8765 line | | |
| 8771 | RIN3 | NM_024832.3 | 2614 | gggagggttcctactatctgacc | 297443 | 665923 | - | - | - |
| 8772 | LZK1 | NM_024835.1 | 466 | atgtacatgggtccaaactaaat | 391299 | 691027 | - | - | - |
| 8773 | LZK1 | NM_024835.1 | 492 | atgatagatgctattccaaaaag | 391300 | 691028 | see also 8772 line | | |

Figure 46

| 8774 | LZK1 | NM_024835.1 | 499 | atgctattccaaaaagtaagaag | 391301 | 691030 | see also 8772 line | | |
|------|------|-------------|-----|------------------------|--------|--------|-------------------|---|---|
| 8775 | LZK1 | NM_024835.1 | 686 | caggttttgcactgattgcaaaa | 391306 | 691045 | see also 8772 line | | |
| 8776 | LZK1 | NM_024835.1 | 1391 | ggggtcccctaaaataaagaaag | 391324 | 691062 | see also 8772 line | | |
| 8777 | LZK1 | NM_024835.1 | 1832 | gccttggtttgagcataggaaaa | 391337 | 691073 | see also 8772 line | | |
| 8778 | LZK1 | NM_024835.1 | 1910 | aggtgccaagagcttagttgaac | 391339 | 691074 | see also 8772 line | | |
| 8779 | LZK1 | NM_024835.1 | 2118 | ttgacaacggctggagcaaatta | 391348 | 691084 | see also 8772 line | | |
| 8780 | FLJ22002 | NM_024838.3 | 1316 | cagcttatgcctcatattttgc | 83198 | 495626 | kinase_related | - | - |
| 8781 | FLJ14154 | NM_024845.1 | 219 | gagtacccagactcatggtatcg | 215146 | 602203 | - | - | - |
| 8782 | FLJ14154 | NM_024845.1 | 635 | cacctatgtcctctacatcaacg | 215153 | 602214 | see also 8781 line | | |
| 8783 | TMC7 | NM_024847.2 | 359 | gcggagactaagggacattcaag | 391368 | 774265 | - | - | - |
| 8784 | EIF2C3 | NM_024852.2 | 493 | tggagaccgtagaccagtttatg | 391434 | 769112 | - | - | - |
| 8785 | EIF2C3 | NM_024852.2 | 497 | gaccgtagaccagtttatgatgg | 391435 | 769113 | see also 8784 line | | |
| 8786 | EIF2C3 | NM_024852.2 | 601 | tggaaaagatcgacctttcaagg | 391438 | 769115 | see also 8784 line | | |
| 8787 | EIF2C3 | NM_024852.2 | 1377 | cagatagacaagaggaaattagc | 391458 | 769136 | see also 8784 line | | |
| 8788 | EIF2C3 | NM_024852.2 | 2346 | aagactatcaacctggaataacc | 391487 | 769155 | see also 8784 line | | |
| 8789 | EIF2C3 | NM_024852.2 | 2539 | tcgtccttcacactatcatgttt | 391499 | 769161 | see also 8784 line | | |
| 8790 | EIF2C3 | NM_024852.2 | 2542 | tccttcacactatcatgttttat | 391500 | 769163 | see also 8784 line | | |
| 8791 | EIF2C3 | NM_024852.2 | 2785 | tgccaaggctgtacagattcacc | 391508 | 769166 | see also 8784 line | | |
| 8792 | EIF2C3 | NM_024852.2 | 2812 | taccttacgcacaatgtacttcg | 391510 | 769168 | see also 8784 line | | |
| 8793 | FLJ12785 | NM_024855.2 | 644 | aactgcaagcgcatcaatcttgg | 287320 | 659228 | - | - | - |
| 8794 | FLJ13962 | NM_024862.1 | 543 | tagaatccatttggacatatatg | 391547 | 750915 | - | - | - |
| 8795 | FLJ13962 | NM_024862.1 | 581 | ttggggaaatttcgtaatcgtaa | 391549 | 750917 | see also 8794 line | | |
| 8796 | FLJ13962 | NM_024862.1 | 582 | tggggaaatttcgtaatcgtaat | 391550 | 750918 | see also 8794 line | | |
| 8797 | FLJ13962 | NM_024862.1 | 583 | ggggaaatttcgtaatcgtaatg | 391551 | 750919 | see also 8794 line | | |
| 8798 | FLJ13962 | NM_024862.1 | 1327 | tacacaacccatacaattggatc | 395030 | 750945 | see also 8794 line | | |
| 8799 | PKD1-like | NM_024874.3 | 1817 | atggcatcaccagctatgagtgg | 150692 | 544287 | - | - | - |
| 8800 | SYNPO2L | NM_024875.1 | 252 | ggcggcagagagccaagaagtac | 391611 | 679696 | - | - | - |
| 8801 | DHDDS | NM_024887.1 | 187 | accgtcgctatgccaagaagtgc | 218587 | 606523 | - | - | - |
| 8802 | DHDDS | NM_024887.1 | 968 | aaggacacgcttgcacaaactct | 218603 | 606529 | see also 8801 line | | |
| 8803 | C6orf134 | NM_024909.1 | 535 | cacagtcccacaggtgaacaact | 391969 | 734833 | - | - | - |
| 8804 | FLJ23091 | NM_024911.3 | 1128 | atccgacagggcatcttctatgc | 392000 | 726083 | - | - | - |
| 8805 | FLJ12687 | NM_024917.4 | 2219 | aagctgtacctgtggatttgttc | 180575 | 604254 | - | - | - |
| 8806 | FLJ12687 | NM_024917.4 | 2220 | agctgtacctgtggatttgttcc | 180576 | 604255 | see also 8805 line | | |
| 8807 | NUP210 | NM_024923.2 | 844 | tggtgggaacctccattcactac | 392213 | 707579 | - | - | - |
| 8808 | FLJ12571 | NM_024926.1 | 468 | ttggctcacaagtttaatgatga | 392272 | 770955 | - | - | - |
| 8809 | FLJ12571 | NM_024926.1 | 470 | ggctcacaagtttaatgatgaga | 392273 | 770956 | see also 8808 line | | |
| 8810 | FLJ22559 | NM_024928.3 | 239 | ttctagcctttgcaaaactctac | 61738 | 788504 | - | - | - |

Figure 46

EP 1 752 536 A1

| | | | | | | | | | |
|---|---|---|---|---|---|---|---|---|---|
| 8811 | FLJ21918 | NM_024939.1 | 225 | ctcggacgagaccgacttaatcc | 61681 | 480652 | - | - | - |
| 8812 | FLJ21918 | NM_024939.1 | 1360 | atgctgggtaagcgatacattga | 61698 | 480665 | see also 8811 line | | |
| 8813 | FLJ13611 | NM_024941.1 | 526 | aaccattggatgtgaaaaccaaa | 392366 | 719853 | - | - | - |
| 8814 | PHC3 | NM_024947.2 | 2469 | cacttagtcgttggaatcgtaag | 392537 | 769553 | - | - | - |
| 8815 | PHC3 | NM_024947.2 | 2562 | tccttaggcagcttccaattact | 392542 | 769556 | see also 8814 line | | |
| 8816 | FLJ13397 | NM_024948.1 | 305 | ctggacgcaagggtttgtgttta | 145244 | 542829 | - | - | - |
| 8817 | FLJ13397 | NM_024948.1 | 1020 | aaggttggttcttacttgaaatc | 145269 | 542849 | see also 8816 line | | |
| 8818 | FLJ13397 | NM_024948.1 | 1087 | ccgtatttttgccaaggatatg | 145271 | 542850 | see also 8816 line | | |
| 8819 | FLJ13397 | NM_024948.1 | 1097 | tgccaaggatatggctttagttg | 145272 | 542851 | see also 8816 line | | |
| 8820 | FLJ13397 | NM_024948.1 | 1379 | tggattgaagcagtcaaattata | 145283 | 542858 | see also 8816 line | | |
| 8821 | FLJ13397 | NM_024948.1 | 1499 | aaccaaatggccatacattgagt | 145287 | 542863 | see also 8816 line | | |
| 8822 | FLJ13089 | NM_024953.2 | 66 | cggcccatttacgattatcttga | 392598 | 778025 | - | - | - |
| 8823 | FLJ13089 | NM_024953.2 | 68 | gcccatttacgattatcttgaca | 392599 | 778026 | see also 8822 line | | |
| 8824 | FLJ13089 | NM_024953.2 | 813 | ttctatctgacttatttcgattc | 392629 | 778048 | see also 8822 line | | |
| 8825 | FLJ23375 | NM_024956.2 | 1172 | ttgatcacgacctctttagcttt | 392721 | 595258 | - | - | - |
| 8826 | PANK2 | NM_024960.3 | 405 | caggattttctcacaataggtga | 95605 | 769591 | kinase_related | - | - |
| 8827 | PANK2 | NM_024960.3 | 502 | atggacggtcacagtgctattac | 95606 | 769595 | see also 8826 line | | |
| 8828 | PANK2 | NM_024960.3 | 503 | tggacggtcacagtgctattact | 95607 | 769596 | see also 8826 line | | |
| 8829 | TAIP-2 | NM_024969.2 | 457 | ttgtgtcaccgtgtactacttca | 311811 | 769269 | apoptosis | - | - |
| 8830 | EFG1 | NM_024996.5 | 785 | tggagactttggtcagattgttc | 57261 | 477524 | - | - | - |
| 8831 | EFG1 | NM_024996.5 | 1451 | tcctgtcatttcaatagcaatga | 57289 | 477539 | see also 8830 line | | |
| 8832 | ADAMTS20 | NM_025003.2 | 1795 | aggatgaaatttcgatcatgtaa | 392946 | 796234 | - | - | - |
| 8833 | ADAMTS20 | NM_025003.2 | 2458 | gtgttgtgtgtgggtaatttata | 392968 | 796261 | see also 8832 line | | |
| 8834 | ADAMTS20 | NM_025003.2 | 2463 | gtgtgtgggtaatttatacaacc | 392969 | 796262 | see also 8832 line | | |
| 8835 | VCIP135 | NM_025054.3 | 814 | cggaagcctcctgtatttgcatg | 393262 | 783880 | signal_transduction | - | - |
| 8836 | VCIP135 | NM_025054.3 | 1052 | gagtgggaggacattatcaatga | 393266 | 783888 | see also 8835 line | | |
| 8837 | VCIP135 | NM_025054.3 | 1860 | acctaacagcttgtaattggtgc | 393292 | 783919 | see also 8835 line | | |
| 8838 | VCIP135 | NM_025054.3 | 1931 | ttggatggagacagaactaattc | 393295 | 783921 | see also 8835 line | | |
| 8839 | VCIP135 | NM_025054.3 | 2887 | tactggtaaactgtcatctaagg | 393322 | 783946 | see also 8835 line | | |
| 8840 | RPF1 | NM_025065.5 | 676 | gagcagtgttcgtcttcgtaaag | 393452 | 729126 | - | - | - |
| 8841 | RPF1 | NM_025065.5 | 677 | agcagtgttcgtcttcgtaaaga | 393453 | 729127 | see also 8840 line | | |
| 8842 | RPF1 | NM_025065.5 | 679 | cagtgttcgtcttcgtaaagaaa | 393454 | 729128 | see also 8840 line | | |
| 8843 | FRAS1 | NM_025074.2 | 1053 | atgcatttcaaggaatggttatt | 146282 | 649127 | - | - | - |
| 8844 | FRAS1 | NM_025074.2 | 7562 | tggggttgattcgttatgtgttg | 146391 | 649244 | see also 8843 line | | |

Figure 46

| 8845 | FRAS1 | NM_025074.2 | 7563 | ggggttgattcgttatgtgttgc | 146392 | 649245 | see also 8843 line | | |
|------|-------|-------------|------|-------------------------|--------|--------|--------------------|--|--|
| 8846 | FRAS1 | NM_025074.2 | 7649 | atgtggtcagcgacaatgtcttc | 146393 | 649247 | see also 8843 line | | |
| 8847 | FRAS1 | NM_025074.2 | 8143 | tgctacacagtccctaagtcagc | 146402 | 649253 | see also 8843 line | | |
| 8848 | FRAS1 | NM_025074.2 | 10642 | aagacccatgccaaattcagagg | 146465 | 649298 | see also 8843 line | | |
| 8849 | NIF3L1BP1 | NM_025075.1 | 51 | ccgtgactgacgacgaagttata | 393492 | 716315 | - | - | - |
| 8850 | NIF3L1BP1 | NM_025075.1 | 53 | gtgactgacgacgaagttatacg | 393493 | 716316 | see also 8849 line | | |
| 8851 | NIF3L1BP1 | NM_025075.1 | 249 | tagtatatgatatgaatctcaga | 393497 | 716325 | see also 8849 line | | |
| 8852 | NIF3L1BP1 | NM_025075.1 | 556 | tagtaccatccatgaacttcagc | 393504 | 716333 | see also 8849 line | | |
| 8853 | TBDN100 | NM_025085.2 | 740 | atgctattgcttaccatttatta | 219052 | 602466 | transcription_regulator | - | - |
| 8854 | TBDN100 | NM_025085.2 | 741 | tgctattgcttaccatttattag | 219053 | 602467 | see also 8853 line | | |
| 8855 | TBDN100 | NM_025085.2 | 907 | ttggaacatctttgtacctatga | 219060 | 602473 | see also 8853 line | | |
| 8856 | TBDN100 | NM_025085.2 | 1874 | tagatcatatgtggacttattaa | 219090 | 602500 | see also 8853 line | | |
| 8857 | TBDN100 | NM_025085.2 | 2011 | aagctgatacagcaaacatgtct | 219098 | 602509 | see also 8853 line | | |
| 8858 | TBDN100 | NM_025085.2 | 2561 | cagattatcagctgccaaaatgg | 219112 | 602529 | see also 8853 line | | |
| 8859 | FLJ21511 | NM_025087.1 | 1204 | gaggtgtctacgctagagaaaga | 393536 | 757362 | - | - | - |
| 8860 | FLJ21106 | NM_025097.1 | 1263 | cagttgatccaagcctcattata | 393603 | 726223 | - | - | - |
| 8861 | CCDC2 | NM_025103.1 | 975 | tggagtcccatcgagatcaaatg | 393641 | 723926 | - | - | Alzheimer disease |
| 8862 | CCDC2 | NM_025103.1 | 979 | gtcccatcgagatcaaatgattg | 393642 | 723927 | see also 8861 line | | |
| 8863 | CCDC2 | NM_025103.1 | 980 | tcccatcgagatcaaatgattgc | 393643 | 723928 | see also 8861 line | | |
| 8864 | FLJ22865 | NM_025109.3 | 2171 | ctggttaactcggaaacacatcc | 83299 | 487996 | - | - | - |
| 8865 | FLJ23263 | NM_025115.1 | 1538 | caggttggggatcctaactgtcc | 393791 | 755105 | - | - | - |
| 8866 | RNF34 | NM_025126.2 | 150 | ctgtgggctgctgaatgaagtca | 265777 | 466372 | - | - | - |
| 8867 | PWDMP | NM_025132.2 | 846 | ttggcaacattgtctgctataat | 393844 | 768838 | - | - | - |
| 8868 | FLJ12584 | NM_025139.2 | 1052 | ttggggagtgaccgcttgaaagc | 393938 | 729722 | - | - | - |
| 8869 | FLJ12584 | NM_025139.2 | 1062 | accgcttgaaagccttcttgttg | 393939 | 729723 | see also 8868 line | | |
| 8870 | FLJ12584 | NM_025139.2 | 1241 | tacatggccaggctcatcaatgc | 393941 | 729725 | see also 8868 line | | |
| 8871 | MAK3P | NM_025146.1 | 421 | gaggttggcgagctagcaaaact | 215160 | 602427 | - | - | - |
| 8872 | RUFY1 | NM_025158.2 | 1013 | cagctgcaacagaccgaatttgc | 159944 | 540190 | - | - | - |
| 8873 | FLJ13386 | NM_025180.2 | 1703 | cagcgagatatcactattgcttc | 394232 | 812839 | - | - | - |
| 8874 | FLJ11560 | NM_025182.2 | 859 | tgcacacacttgacactgatttg | 394289 | 777569 | - | - | - |
| 8875 | Lin10 | NM_025187.3 | 649 | taccccatggagcaactgtaaaa | 281309 | 757727 | - | - | - |
| 8876 | Lin10 | NM_025187.3 | 974 | tccttcccctcataaacaagttc | 281319 | 757735 | see also 8875 line | | |

Figure 46

| 8877 | EPC1 | NM_025209.2 | 353 | tgcacgaatacgcctcgataaac | 275827 | 729675 | transcription_regulator | - | - |
|------|------|-------------|-----|-------------------------|--------|--------|-------------------------|---|---|
| 8878 | EPC1 | NM_025209.2 | 428 | atcatcttcagcgggctatttca | 275829 | 696362 | see also 8877 line | | |
| 8879 | EPC1 | NM_025209.2 | 1568 | taggggatgtgcgatatagatac | 275864 | 696386 | see also 8877 line | | |
| 8880 | EPC1 | NM_025209.2 | 1569 | aggggatgtgcgatatagatact | 275865 | 696387 | see also 8877 line | | |
| 8881 | EPC1 | NM_025209.2 | 2594 | gacatatacctaggactttaagt | 275900 | 696407 | see also 8877 line | | |
| 8882 | ADAM33 | NM_025220.2 | 407 | cccccaaccacacggatcattgc | 155058 | 556092 | - | - | - |
| 8883 | KCNIP4 | NM_025221.4 | 261 | aacgtcgtctcctgctattcaaa | 147165 | 549165 | channel | - | - |
| 8884 | KCNIP4 | NM_025221.4 | 262 | acgtcgtctcctgctattcaaaa | 147166 | 549166 | see also 8883 line | | |
| 8885 | T3JAM | NM_025228.1 | 1407 | caggacctacaagatcaactaaa | 394479 | 694064 | - | - | - |
| 8886 | DPCK | NM_025233.3 | 1343 | aacagatattctccataaagatg | 80855 | 731290 | kinase_related | transferase | - |
| 8887 | SLC19A3 | NM_025243.2 | 1314 | tggtgattcagaccatcatgact | 179772 | 573060 | - | - | - |
| 8888 | MGC2776 | NM_025265.2 | 1475 | atcggaaaggccctccattttac | 199093 | 815871 | - | - | - |
| 8889 | ITIH5 | NM_030569.2 | 884 | gtgcactactttgctcctaaaga | 394653 | 774160 | - | - | - |
| 8890 | THAP7 | NM_030573.1 | 841 | cggagcctacatccagaatgaac | 41945 | 452942 | - | - | - |
| 8891 | STARD5 | NM_030574.2 | 541 | gaccaacctggtcacattcttcc | 173862 | 570546 | - | - | - |
| 8892 | CYB5-M | NM_030579.1 | 319 | atccatccgagtgaccttaaacc | 303906 | 717223 | - | - | - |
| 8893 | CPEB1 | NM_030594.2 | 1213 | ttcctccccggaactataagaac | 58799 | 482061 | - | - | - |
| 8894 | CPEB1 | NM_030594.2 | 1214 | tcctccccggaactataagaacc | 58800 | 482062 | see also 8893 line | | |
| 8895 | CPEB1 | NM_030594.2 | 1474 | cagatggcctgagtgaatattat | 58806 | 482066 | see also 8893 line | | |
| 8896 | CPEB1 | NM_030594.2 | 1735 | ctggtcgtgtgactttcaataac | 58814 | 482072 | see also 8893 line | | |
| 8897 | CPEB1 | NM_030594.2 | 1736 | tggtcgtgtgactttcaataacc | 58815 | 482073 | see also 8893 line | | |
| 8898 | DICER1 | NM_030621.2 | 283 | aagaagcaattcatgataacatt | 48224 | 471821 | - | - | Alzheimer disease |
| 8899 | DICER1 | NM_030621.2 | 613 | agcaccaggttctcattatgact | 48235 | 471830 | see also 8898 line | | |
| 8900 | DICER1 | NM_030621.2 | 657 | ttgaaaaatggttacttatcact | 48240 | 471833 | see also 8898 line | | |
| 8901 | DICER1 | NM_030621.2 | 669 | tacttatcactgtcagacattaa | 48241 | 471834 | see also 8898 line | | |
| 8902 | DICER1 | NM_030621.2 | 1689 | gaggtacttaggaaatttcgagc | 48274 | 471863 | see also 8898 line | | |
| 8903 | DICER1 | NM_030621.2 | 3003 | cagcctcatcgattttatgtagc | 48328 | 471898 | see also 8898 line | | |
| 8904 | DICER1 | NM_030621.2 | 3040 | atcttaccccactcagtaaattt | 48329 | 471899 | see also 8898 line | | |
| 8905 | DICER1 | NM_030621.2 | 3046 | ccccactcagtaaatttccttcc | 48331 | 471900 | see also 8898 line | | |
| 8906 | DICER1 | NM_030621.2 | 3463 | tagacttcgggtggaaaaaatct | 48345 | 471911 | see also 8898 line | | |
| 8907 | DICER1 | NM_030621.2 | 3735 | gccaatggcagttatgatttagc | 48359 | 471924 | see also 8898 line | | |
| 8908 | DICER1 | NM_030621.2 | 5279 | ttgttaccagcgcttagaattcc | 48405 | 471959 | see also 8898 line | | |
| 8909 | DICER1 | NM_030621.2 | 5325 | ctcataaccaagcacctttatga | 48406 | 471960 | see also 8898 line | | |
| 8910 | CPEB4 | NM_030627.1 | 2701 | gtgaccgccctcggcatatttca | 58896 | 482184 | - | - | - |
| 8911 | CPEB4 | NM_030627.1 | 2703 | gaccgccctcggcatatttcatt | 58897 | 482185 | see also 8910 line | | |
| 8912 | CPEB4 | NM_030627.1 | 2704 | accgccctcggcatatttcattc | 58898 | 482186 | see also 8910 line | | |

Figure 46

| | | | | | | | | |
|---|---|---|---|---|---|---|---|---|
| 8913 | CPEB4 | NM_030627.1 | 2705 | cgccctcggcatattcattcc | 58899 | 482187 | see also 8910 line | |
| 8914 | KIAA1701 | NM_030639.1 | 1043 | atgttattgggaactggtttg | 394778 | 812108 | - | |
| 8915 | KIAA1701 | NM_030639.1 | 1527 | gtcaactactgatcctcttattc | 394797 | 812126 | see also 8914 line | |
| 8916 | KIAA1701 | NM_030639.1 | 1530 | aactactgatcctcttattcata | 394798 | 812127 | see also 8914 line | |
| 8917 | DUSP16 | NM_030640.1 | 640 | atgagatgattggaactcaaatt | 195551 | 534880 | - | |
| 8918 | DUSP16 | NM_030640.1 | 642 | gagatgattggaactcaaattgt | 195552 | 534881 | see also 8917 line | |
| 8919 | DUSP16 | NM_030640.1 | 727 | ggccatttgtggaatacaataca | 195554 | 534885 | see also 8917 line | |
| 8920 | DUSP16 | NM_030640.1 | 800 | aaggttgcaacagagacaaagtgt | 195555 | 534924 | see also 8917 line | |
| 8921 | SET7 | NM_030648.1 | 974 | tgggaactaatactgttatgtct | 213643 | 603842 | - | |
| 8922 | KIAA1715 | NM_030650.1 | 510 | atgaagcattggatgatttaaaa | 394833 | 728200 | - | |
| 8923 | KIAA1715 | NM_030650.1 | 1080 | ttcgatgtgcctactgttttc | 394846 | 728214 | see also 8922 line | |
| 8924 | TCF8 | NM_030751.2 | 1915 | aagatgcaagctggacagattc | 13010 | 449917 | transcription_regulator | zinc binding |
| 8925 | TCP1 | NM_030752.1 | 857 | tggcaactggtgccaatgttatt | 102209 | 516018 | - | chaperone |
| 8926 | TCP1 | NM_030752.1 | 863 | ctgtgccaatgttattctaaacc | 102211 | 516019 | see also 8925 line | |
| 8927 | WNT3 | NM_030753.3 | 175 | tcctcgctgctacccatttgg | 255471 | 639613 | - | signal transducer |
| 8928 | WNT3 | NM_030753.3 | 286 | tgcgcttctgccgcaattacatc | 255474 | 639614 | see also 8927 line | |
| 8929 | WNT3 | NM_030753.3 | 296 | ccgcaattcatcatcgagatcatgc | 255475 | 639616 | see also 8927 line | |
| 8930 | WNT3 | NM_030753.3 | 948 | tacgagaactccccaactttg | 255482 | 639624 | see also 8927 line | |
| 8931 | WNT3 | NM_030753.3 | 1006 | gggaccggacttgcaatgtcacc | 255484 | 639628 | see also 8927 line | |
| 8932 | TXNDC | NM_030755.3 | 429 | gactaagaaggacttcataaact | 265891 | 649862 | - | |
| 8933 | TCF7L2 | NM_030756.1 | 510 | ctccggcctcgctcgaaagtttc | 394866 | 468916 | transcription_regulator, signal_transduction | transcription factor-7-like-2 |
| 8934 | TCF7L2 | NM_030756.1 | 511 | tccggcctcgctcgaaagtttcc | 394867 | 468917 | see also 8933 line | |
| 8935 | TCF7L2 | NM_030756.1 | 607 | tccggctaccccttcatcatga | 394870 | 468921 | see also 8933 line | |
| 8936 | TCF7L2 | NM_030756.1 | 998 | cccatccgctaggatggttagt | 394880 | 468927 | see also 8933 line | |
| 8937 | TCF7L2 | NM_030756.1 | 1691 | aagtgcgttcgctacatacaagg | 394889 | 468934 | see also 8933 line | |
| 8938 | BHLHB3 | NM_030762.1 | 198 | ctggactattcctctttgtatat | 1520 | 456377 | transcription_regulator | transcription factor |
| 8939 | BHLHB3 | NM_030762.1 | 199 | tggactattcctctttgtatatg | 1521 | 456378 | see also 8938 line | |
| 8940 | BHLHB3 | NM_030762.1 | 274 | taccgcacagattaatagaaaag | 1524 | 456383 | see also 8938 line | |
| 8941 | BHLHB3 | NM_030762.1 | 275 | accgcacagattaatagaaaaga | 1525 | 456384 | see also 8938 line | |
| 8942 | BHLHB3 | NM_030762.1 | 548 | cgccaagaagcttgcaataac | 1532 | 456396 | see also 8938 line | |
| 8943 | ILKAP | NM_030768.2 | 549 | ccctcattactgggtttcatat | 166133 | 620666 | phosphatase, kinase_related, signal_transduction | - |

Figure 46

| | | | | | | | | |
|---|---|---|---|---|---|---|---|---|
| 8944 | MFTC | NM_030780.2 | 229 | tccgctcgacctcgtgaagatcc | 180798 | 773067 | - | folate transporter |
| 8945 | CRR9 | NM_030782.2 | 831 | ctgcagcagttcgggtttcaga | 394973 | 759723 | - | - |
| 8946 | PTDSS2 | NM_030783.1 | 508 | aagctaaagacgggccatttcc | 303085 | 598919 | - | - |
| 8947 | GPR63 | NM_030784.1 | 925 | caggcttatgtgatttgattc | 240190 | 625094 | gpcr, receptor activity, signal transduction | rhodopsin-like receptor |
| 8948 | HM13 | NM_030789.2 | 376 | gggctctacctcttttcaaaat | 191399 | 584859 | - | |
| 8949 | HM13 | NM_030789.2 | 479 | gcccctttcatgaataagttttt | 191400 | 584863 | see also 8948 line | |
| 8950 | TDRD3 | NM_030794.1 | 408 | gactctaacaccacagttcttgg | 66527 | 486709 | - | |
| 8951 | APG16L | NM_030803.5 | 988 | tggaggccttctggattctatca | 395211 | 532049 | - | |
| 8952 | C1orf21 | NM_030806.2 | 584 | tggaagaggtcaaatacatgaaa | 395217 | 810877 | - | |
| 8953 | NDEL1 | NM_030808.2 | 293 | agtcgggatgagctagttgaat | 395225 | 714163 | - | |
| 8954 | NDEL1 | NM_030808.2 | 584 | aagggcaacaatagtttcactgg | 395240 | 714171 | see also 8953 line | |
| 8955 | NDEL1 | NM_030808.2 | 630 | caggccattgaacgaaatgcatt | 395243 | 714172 | see also 8953 line | |
| 8956 | NDEL1 | NM_030808.2 | 938 | tgctaggatcagcactaaaca | 395247 | 714175 | see also 8953 line | |
| 8957 | SKD3 | NM_030813.3 | 1719 | gagtttctgggacggatcaatga | 100450 | 514680 | - | |
| 8958 | CTNNBL1 | NM_030877.3 | 1356 | agcggacccggcttctgaataaa | 324095 | 690204 | apoptosis | |
| 8959 | CTNNBL1 | NM_030877.3 | 1357 | gccgacccggcttcgaataaat | 324096 | 690205 | see also 8958 line | |
| 8960 | TRIM8 | NM_030912.1 | 11 | tggcggagaattggaagaactgc | 160715 | 541617 | - | zinc binding |
| 8961 | TRIM8 | NM_030912.1 | 1631 | ggcagccgtccaccaaacactac | 160719 | 541629 | see also 8960 line | |
| 8962 | LBH | NM_030915.1 | 317 | ccgcaaggatggcctttcctacc | 395381 | 741239 | - | |
| 8963 | ANP32E | NM_030920.2 | 979 | gggcctctacttaatgaaag | 294316 | 713042 | phosphatase | |
| 8964 | ITM2C | NM_030926.3 | 373 | cgcagctggcccgagataacttc | 326677 | 504591 | - | |
| 8965 | DC-TM4F2 | NM_030927.1 | 278 | tggtgggcgtggtgatgttcacc | 326384 | 758398 | - | |
| 8966 | UNC93B1 | NM_030930.1 | 418 | cacctgtctcatcaggtttt | 395537 | 708128 | - | |
| 8967 | DIAPH3 | NM_030932.2 | 447 | ttgtggattgaaagagatattgc | 60019 | 483850 | - | actin binding |
| 8968 | DIAPH3 | NM_030932.2 | 1735 | cggccacggctcagtgctattct | 60050 | 483877 | see also 8967 line | |
| 8969 | DIAPH3 | NM_030932.2 | 1746 | cagtgctattctcttttaagcttc | 60051 | 483879 | see also 8967 line | |
| 8970 | DIAPH3 | NM_030932.2 | 2169 | tcctgaggactgcatgcacaagt | 60058 | 483884 | see also 8967 line | |
| 8971 | VMP1 | NM_030938.2 | 262 | cgctcattaccttgcagtattt | 395598 | 739296 | - | |
| 8972 | C6orf62 | NM_030939.2 | 883 | ttggcctcggaatgaatattgaaa | 395639 | 715545 | - | |
| 8973 | C1QTNF3 | NM_030945.1 | 142 | gccctgtcaagatgaatacatg | 395656 | 695194 | - | |
| 8974 | C1QTNF3 | NM_030945.1 | 610 | aggaagtgtatgtgaccttatg | 395661 | 695203 | see also 8973 line | |
| 8975 | SLCO5A1 | NM_030958.1 | 1695 | tgtgatattcccaatgtttact | 274712 | 655629 | - | |
| 8976 | SLCO5A1 | NM_030958.1 | 1731 | ttccacctgacacaagaaaaag | 274713 | 655632 | see also 8975 line | |
| 8977 | SLCO5A1 | NM_030958.1 | 1732 | tccacctgacacaagaaaaaga | 274714 | 655633 | see also 8975 line | |

Figure 46

| 8978 | SLCO5A1 | NM_030958.1 | 1887 | cagctgtcaggatcttaagcaac | 274717 | 655635 | see also 8975 line | | |
| 8979 | SLCO5A1 | NM_030958.1 | 1985 | aagttcatcgagtcacagtttgg | 274720 | 655636 | see also 8975 line | | |
| 8980 | CMT4B2 | NM_030962.1 | 988 | tggatggaggcactattaaaatt | 395731 | 643114 | - | - | - |
| 8981 | CMT4B2 | NM_030962.1 | 1957 | ctctacaggattgttcaagttta | 395766 | 643138 | see also 8980 line | | |
| 8982 | CMT4B2 | NM_030962.1 | 1962 | caggattgttcaagtttagaaga | 395767 | 643139 | see also 8980 line | | |
| 8983 | RNF146 | NM_030963.2 | 875 | atggacgtcgcaggaagattaag | 395883 | 725708 | - | - | - |
| 8984 | RNF146 | NM_030963.2 | 876 | tggacgtcgcaggaagattaagc | 395884 | 725709 | see also 8983 line | | |
| 8985 | RNF146 | NM_030963.2 | 889 | aagattaagcgagatataataga | 395885 | 725711 | see also 8983 line | | |
| 8986 | RNF146 | NM_030963.2 | 895 | aagcgagatataatagatatacc | 395886 | 725712 | see also 8983 line | | |
| 8987 | ARPC5L | NM_030978.1 | 497 | aggaggactaggctccattataa | 395921 | 736391 | - | - | - |
| 8988 | FLJ12525 | NM_031206.2 | 430 | ttggtcaggtttgtgaatcttat | 395924 | 767547 | - | - | - |
| 8989 | FLJ12525 | NM_031206.2 | 431 | tggtcaggtttgtgaatcttatc | 395925 | 767548 | see also 8988 line | | |
| 8990 | QTRT1 | NM_031209.1 | 452 | cgctgggctcggacatcatcatg | 212696 | 601459 | - | transferase、transferring glycosyl groups | - |
| 8991 | SLC25A28 | NM_031212.2 | 1488 | gccatcgcatggtctgtgtatga | 181232 | 574431 | - | - | - |
| 8992 | SEC13L | NM_031216.2 | 266 | aagacacatagtggatctgtatg | 395976 | 732590 | - | - | - |
| 8993 | SEC13L | NM_031216.2 | 682 | cgcaatggccaaggttcagattt | 395991 | 732599 | see also 8992 line | | |
| 8994 | DKFZP434 G2226 | NM_031217.2 | 907 | ttgtagaaggcacaaatattaat | 78313 | 505127 | - | - | - |
| 8995 | DKFZP434 G2226 | NM_031217.2 | 1102 | atgacacatataacactcttaag | 78322 | 505131 | see also 8994 line | | |
| 8996 | TEX15 | NM_031271.2 | 1780 | gtgtgtgtagcctcaaatgctgc | 396059 | 743007 | - | - | - |
| 8997 | TEX15 | NM_031271.2 | 2878 | tcccaaggacgaattaaaacatt | 396088 | 743032 | see also 8996 line | | |
| 8998 | TEX15 | NM_031271.2 | 6027 | gagatgtacagatcacttagaaa | 396200 | 743136 | see also 8996 line | | |
| 8999 | TEX14 | NM_031272.2 | 1550 | tactatgatattgttaagtcagg | 102449 | 516231 | kinase_related | - | - |
| 9000 | TEX12 | NM_031275.2 | 272 | agcagcagtagatgcatcttaca | 396272 | 717890 | - | - | - |
| 9001 | TEX12 | NM_031275.2 | 274 | cagcagtagatgcatcttacatt | 396273 | 717891 | see also 9000 line | | |
| 9002 | DKFZP434 B195 | NM_031284.3 | 415 | ctgagcgcttcttcagtgataag | 60140 | 732653 | kinase_related | - | - |
| 9003 | GSG1 | NM_031289.1 | 563 | caccccactctccgatttggagg | 396512 | 700980 | - | - | - |
| 9004 | DKFZP434 N1235 | NM_031291.1 | 376 | gcgtggcaatttggcaaatgtta | 180457 | 573396 | - | - | - |
| 9005 | DKFZP434 N1235 | NM_031291.1 | 378 | gtggcaatttggcaaatgttatt | 180458 | 573397 | see also 9004 line | | |
| 9006 | DKFZP434 N1235 | NM_031291.1 | 861 | cagttagaagacgtatgatgatg | 180478 | 573414 | see also 9004 line | | |
| 9007 | RAB33B | NM_031296.1 | 922 | ctgctaaaaaccccaatgataat | 110187 | 520038 | signal_transduction | protein transporter | - |

Figure 46

| 9008 | RAB33B | NM_031296.1 | 923 | tgctaaaaaccccaatgataatg | 110188 | 520039 | see also 9007 line | | |
|---|---|---|---|---|---|---|---|---|---|
| 9009 | DKFZP564B1023 | NM_031306.1 | 488 | gtggtcgttatggagagtatatc | 60355 | 478191 | - | - | - |
| 9010 | DKFZP564B1023 | NM_031306.1 | 489 | tggtcgttatggagagtatatct | 60356 | 478192 | see also 9009 line | | |
| 9011 | DKFZP564B1023 | NM_031306.1 | 1121 | atgttaaggttatcatagcaacc | 60381 | 478200 | see also 9009 line | | |
| 9012 | FKSG32 | NM_031307.2 | 402 | cagacagacatccaactatcacc | 396665 | 713204 | - | - | - |
| 9013 | FKSG32 | NM_031307.2 | 964 | atggagaagccagagattattga | 396688 | 713226 | see also 9012 line | | |
| 9014 | FKSG32 | NM_031307.2 | 1389 | acgaattgagcacccacatttat | 396702 | 713243 | see also 9012 line | | |
| 9015 | STK31 | NM_031414.2 | 2571 | tggatcacttcatcagaacaatg | 66353 | 486448 | kinase_related | - | - |
| 9016 | MARK4 | NM_031417.2 | 992 | cggggacaccaagagaattgagg | 90326 | 508022 | kinase_related | - | - |
| 9017 | CDCA1 | NM_031423.2 | 448 | gccgggtgaatgactttgagact | 278222 | 713164 | - | - | - |
| 9018 | COG3 | NM_031431.2 | 777 | ctggccaagttagatgattgtat | 268486 | 795851 | - | - | - |
| 9019 | COG3 | NM_031431.2 | 779 | ggccaagttagatgattgtataa | 268487 | 795853 | see also 9018 line | | |
| 9020 | COG3 | NM_031431.2 | 787 | tagatgattgtataacatatatc | 268489 | 795854 | see also 9018 line | | |
| 9021 | COG3 | NM_031431.2 | 1970 | tgctccatttcacactgaattca | 268528 | 795883 | see also 9018 line | | |
| 9022 | COG3 | NM_031431.2 | 2013 | gacctcaagaaaactagagatgc | 268530 | 795885 | see also 9018 line | | |
| 9023 | COG3 | NM_031431.2 | 2087 | tagcaacaatgccttgatagagt | 268532 | 795889 | see also 9018 line | | |
| 9024 | DKFZP564I0422 | NM_031435.1 | 618 | tcgcctggttaggcgcaaaaatt | 25134 | 718803 | - | - | - |
| 9025 | DKFZP564I0422 | NM_031435.1 | 619 | cgcctggttaggcgcaaaaattt | 25135 | 718804 | see also 9024 line | | |
| 9026 | DKFZP564I0422 | NM_031435.1 | 620 | gcctggttaggcgcaaaaatttt | 25136 | 718805 | see also 9024 line | | |
| 9027 | DKFZP564I0422 | NM_031435.1 | 622 | ctggttaggcgcaaaaattttgt | 25137 | 718806 | see also 9024 line | | |
| 9028 | RASSF5 | NM_031437.1 | 1013 | cactttttaagcggatacacaag | 315033 | 539601 | signal_transduction | - | - |
| 9029 | SOX7 | NM_031439.2 | 1053 | gggacatggatcgcaatgaattc | 40637 | 468039 | transcription_regulator | DNA binding | - |
| 9030 | TM4SF10 | NM_031442.2 | 722 | ctggcctggggtgcaactatatt | 397019 | 752652 | - | - | - |
| 9031 | TM4SF10 | NM_031442.2 | 723 | tggcctggggtgcaactatattt | 397020 | 752653 | see also 9030 line | | |
| 9032 | TM4SF10 | NM_031442.2 | 725 | gcctggggtgcaactatattttc | 397021 | 752655 | see also 9030 line | | |
| 9033 | SELO | NM_031454.1 | 743 | gacgtgttctatgatggtaatcc | 227448 | 731368 | - | - | - |
| 9034 | BAL | NM_031458.1 | 1855 | caccacatcattgagaataatca | 277192 | 671012 | - | - | - |
| 9035 | SESN2 | NM_031459.3 | 677 | cagctcctggcgccactacattg | 306419 | 674287 | cell_cycle | - | - |
| 9036 | SESN2 | NM_031459.3 | 678 | agctcctggcgccactacattgc | 306420 | 674288 | see also 9035 line | | |
| 9037 | SESN2 | NM_031459.3 | 1547 | ctgcgtctttggcatcagatatg | 306424 | 674301 | see also 9035 line | | |

Figure 46

| ID | Gene | Accession | Length | Sequence | Value 1 | Value 2 | Note | Function | Disease |
|---|---|---|---|---|---|---|---|---|---|
| 9038 | APG10L | NM_031482.3 | 818 | atgtcaactatatcacatcatgg | 397198 | 532021 | - | - | - |
| 9039 | L3MBTL2 | NM_031488.3 | 482 | gcctggtcgtgccaaaattggagc | 113125 | 477697 | transcription_regulator | - | |
| 9040 | LONP | NM_031490.2 | 580 | ctgtccctgcagttgctaaattg | 88754 | 488177 | - | - | |
| 9041 | LONP | NM_031490.2 | 1009 | atgtctgactagaaattattg | 88770 | 488196 | see also 9040 line | | |
| 9042 | ENAM | NM_031889.1 | 411 | atgcccgaatgcctggatttag | 289639 | 660048 | - | structural constituent of tooth enamel | amelogenesis imperfecta |
| 9043 | ENAM | NM_031889.1 | 412 | tgcccgaatgcctggatttagc | 289640 | 660049 | see also 9042 line | | |
| 9044 | ENAM | NM_031889.1 | 415 | ccgaatgcctggatttagcagt | 289641 | 660050 | see also 9042 line | | |
| 9045 | CDH20 | NM_031891.2 | 1224 | tggaacaaccatacagatcatt | 139914 | 545679 | - | calcium ion binding | |
| 9046 | SH3KBP1 | NM_031892.1 | 1097 | aggattactgcaaagtaatatt | 322110 | 711262 | - | | |
| 9047 | CACNG8 | NM_031895.4 | 543 | cggcctcccggtctacaagtcc | 261642 | 645291 | channel | voltage-gated calcium channel | |
| 9048 | CACNG7 | NM_031896.3 | 273 | gacggaaaacacggagaatattc | 261629 | 747014 | channel | voltage-gated calcium channel | |
| 9049 | GORASP1 | NM_031899.2 | 227 | tgggcactcgaggctgaacaagg | 397231 | 737502 | - | | |
| 9050 | C1QTNF4 | NM_031909.1 | 1115 | cagcaaccacggcaagtacatca | 397252 | 721946 | - | | |
| 9051 | C1QTNF7 | NM_031911.3 | 921 | ttcgggtccaagtcatctatc | 397272 | 788916 | - | | |
| 9052 | CSDUFD1 | NM_031919.1 | 350 | tgcaacaaggtcattagatataa | 278862 | 691115 | - | | |
| 9053 | REPS1 | NM_031922.2 | 1382 | ttcgtaggcaatccagtagttat | 151960 | 552909 | - | | |
| 9054 | REPS1 | NM_031922.2 | 1591 | ttgacactggatgagttttgtgc | 151968 | 552920 | see also 9053 line | | |
| 9055 | REPS1 | NM_031922.2 | 1881 | gacccaatttgattctaacattg | 151979 | 552924 | see also 9053 line | | |
| 9056 | RAB34 | NM_031934.3 | 974 | agctcaagccatcatcattgtct | 110203 | 520057 | signal transduction | protein transporter | |
| 9057 | RAB34 | NM_031934.3 | 976 | ctcaagccatcatcattgtcttc | 110204 | 520058 | see also 9056 line | | |
| 9058 | BCDO2 | NM_031938.1 | 820 | taccatagctttggaatgacaag | 224535 | 610957 | - | growth factor | |
| 9059 | B29 | NM_031939.2 | 803 | aggttgccaaggcttgcaaaaca | 397412 | 730767 | - | | |
| 9060 | NYD-SP12 | NM_031955.2 | 676 | tgccagctcttgctatagacaaa | 397499 | 730133 | - | | |
| 9061 | RASSF4 | NM_032023.3 | 966 | atgccggtgccggacagttttgt | 315027 | 679344 | signal transduction | | |
| 9062 | CDA11 | NM_032026.1 | 399 | aagatactcaactcaaatattt | 397635 | 785843 | - | | |
| 9063 | CDA11 | NM_032026.1 | 403 | tactcaactcaaatattttgaaa | 397636 | 785844 | see also 9062 line | | |
| 9064 | SSTK | NM_032037.1 | 268 | cccggacttcgtcaacaagttc | 101322 | 515391 | kinase_related | | |
| 9065 | SSTK | NM_032037.1 | 269 | cccggacttcgtcaacaagttcc | 101323 | 515392 | see also 9064 line | | |
| 9066 | NCALD | NM_032041.1 | 295 | ggggatgcttccaaatttgcaga | 118182 | 750731 | - | | |
| 9067 | NCALD | NM_032041.1 | 351 | tgggacaatagactttagagaat | 118184 | 750732 | see also 9066 line | | |
| 9068 | BRIP1 | NM_032043.1 | 1764 | tagcagatttgcagatgattata | 23005 | 496401 | - | | ataxia telangiectasia |
| 9069 | BRIP1 | NM_032043.1 | 2576 | ctggccgtcagtgtgatgaaatt | 23033 | 496425 | see also 9068 line | | |
| 9070 | BRIP1 | NM_032043.1 | 2578 | ggcccgtcagtgtatgtgaaattca | 23034 | 496427 | see also 9068 line | | |

Figure 46

| | | | | | | | | | |
|---|---|---|---|---|---|---|---|---|---|
| 9071 | KREMEN1 | NM_032045.3 | 654 | gcgcctgcggtgggaactactca | 281164 | 667356 | signal_transduction | - | - |
| 9072 | KREMEN1 | NM_032045.3 | 926 | ttcttctctgatcgcatcaatca | 281169 | 667361 | see also 9071 line | | |
| 9073 | EMILIN2 | NM_032048.1 | 3244 | ctggctcacacagactttgatga | 131048 | 755520 | - | - | - |
| 9074 | OTP | NM_032109.2 | 216 | accgggaggcggtgaagtgtagg | 397757 | 447519 | transcription_regulator | - | - |
| 9075 | MGC2599 | NM_032116.2 | 1028 | tgccctaccacgatcttcattg | 91257 | 508385 | - | - | - |
| 9076 | FLJ12953 | NM_032118.2 | 255 | tggagtgagtcccccacttatca | 397781 | 714387 | - | - | - |
| 9077 | FLJ12953 | NM_032118.2 | 257 | gagtgagtccccccacttatcact | 397782 | 714388 | see also 9076 line | | |
| 9078 | DKFZP564D0478 | NM_032125.1 | 150 | cccggtgctcacgtggtttttcc | 264579 | 648851 | - | - | - |
| 9079 | DKFZP566M1046 | NM_032127.1 | 1103 | atcactgcctcgaaagattgagg | 397928 | 815361 | - | - | - |
| 9080 | DKFZP566M1046 | NM_032127.1 | 1177 | cagcccttgcactcttcatgagt | 397930 | 815362 | see also 9079 line | | |
| 9081 | DKFZP566M1046 | NM_032127.1 | 1180 | cccttgcactcttcatgagttcc | 397931 | 815363 | see also 9079 line | | |
| 9082 | DKFZP566M1046 | NM_032127.1 | 3084 | gaggctcttcgagtcaagaatgc | 397938 | 815379 | see also 9079 line | | |
| 9083 | ARL6 | NM_032146.3 | 590 | ctgggaacactattataaagaag | 108551 | 708938 | signal_transduction | - | - |
| 9084 | DKFZP434K0427 | NM_032148.2 | 1084 | tacacccattgctgctagttttg | 266662 | 653764 | - | - | - |
| 9085 | TREX1 | NM_032166.2 | 1933 | tgctgtacatgtacatcacatca | 199326 | 778828 | - | - | - |
| 9086 | FLJ12787 | NM_032175.1 | 313 | aggcctgcatatcgaacctttat | 398492 | 804931 | - | - | - |
| 9087 | FLJ12787 | NM_032175.1 | 315 | gcctgcatatcgaacctttatta | 398493 | 804932 | see also 9086 line | | |
| 9088 | FLJ12787 | NM_032175.1 | 317 | ctgcatatcgaacctttattaaa | 398494 | 804933 | see also 9086 line | | |
| 9089 | FLJ12787 | NM_032175.1 | 318 | tgcatatcgaacctttattaaag | 398495 | 804934 | see also 9086 line | | |
| 9090 | FLJ12787 | NM_032175.1 | 323 | atcgaacctttattaaaggaaaa | 398496 | 804935 | see also 9086 line | | |
| 9091 | KIAA0157 | NM_032182.2 | 692 | aagagtgagcgagttgttgaatc | 398556 | 811153 | - | GTPase activator | - |
| 9092 | KIAA0157 | NM_032182.2 | 696 | gtgagcgagttgttgaatcttgt | 398557 | 811154 | see also 9091 line | | |
| 9093 | KIAA0157 | NM_032182.2 | 698 | gagcgagttgttgaatcttgtca | 398558 | 811155 | see also 9091 line | | |
| 9094 | KIAA0157 | NM_032182.2 | 941 | cacccaaacaatcaagaaagtac | 398562 | 811159 | see also 9091 line | | |
| 9095 | ASC1p100 | NM_032204.3 | 200 | aggcagaccggtattttgtgtta | 398643 | 738610 | transcription_regulator | - | - |
| 9096 | ASC1p100 | NM_032204.3 | 314 | tggccttgcctcacgataaattc | 398645 | 738615 | see also 9095 line | | |
| 9097 | ASC1p100 | NM_032204.3 | 315 | ggccttgcctcacgataaattct | 398646 | 738616 | see also 9095 line | | |
| 9098 | FLJ21742 | NM_032207.1 | 1848 | gagcctgacgcagcagttcatcc | 398697 | 732912 | - | - | - |
| 9099 | ANKRD17 | NM_032217.3 | 1177 | ttggaatccggtgctagtattga | 15757 | 742565 | - | - | - |
| 9100 | ANKRD17 | NM_032217.3 | 1191 | tagtattgaggaccataatgaaa | 15759 | 742566 | see also 9099 line | | |

Figure 46

| 9101 | ANKRD17 | NM_032217.3 | 1363 | atggtgcgatttctttggaagc | 15768 | 742573 | see also 9099 line | | |
|------|---------|-------------|------|------------------------|-------|--------|--------------------|---|---|
| 9102 | ANKRD17 | NM_032217.3 | 2088 | tagaaccacagctaataatgacc | 15785 | 742590 | see also 9099 line | | |
| 9103 | ANKRD17 | NM_032217.3 | 2521 | cagtctccagagagcattgtaga | 15792 | 742601 | see also 9099 line | | |
| 9104 | ANKRD17 | NM_032217.3 | 2545 | gaggctcagggaaagttaacaga | 15793 | 742603 | see also 9099 line | | |
| 9105 | ANKRD17 | NM_032217.3 | 3029 | cagggtccatcgcaaatcttaca | 15804 | 742610 | see also 9099 line | | |
| 9106 | ANKRD17 | NM_032217.3 | 3031 | gggtccatcgcaaatcttacaga | 15805 | 742611 | see also 9099 line | | |
| 9107 | ANKRD17 | NM_032217.3 | 3132 | aacacaacaagggttaatggtag | 15806 | 742615 | see also 9099 line | | |
| 9108 | ANKRD17 | NM_032217.3 | 3523 | ctggacaatggtgcagacattga | 15811 | 742618 | see also 9099 line | | |
| 9109 | ANKRD17 | NM_032217.3 | 4316 | aggtggtgcgctacttagtcaaa | 15827 | 742640 | see also 9099 line | | |
| 9110 | ANKRD17 | NM_032217.3 | 4318 | gtggtgcgctacttagtcaaaga | 15828 | 742641 | see also 9099 line | | |
| 9111 | ANKRD17 | NM_032217.3 | 5913 | gagccctgccagagctactaact | 15863 | 742685 | see also 9099 line | | |
| 9112 | ANKRD17 | NM_032217.3 | 6113 | ttgtcacagttgtgaagacatcc | 15867 | 742690 | see also 9099 line | | |
| 9113 | ANKRD17 | NM_032217.3 | 6221 | aagaacactatccagtatcatcc | 15870 | 742693 | see also 9099 line | | |
| 9114 | ANKRD17 | NM_032217.3 | 6770 | tccagctaccttcgaccttaagt | 15877 | 742710 | see also 9099 line | | |
| 9115 | ANKRD17 | NM_032217.3 | 6773 | agctaccttcgaccttaagtaca | 15879 | 742711 | see also 9099 line | | |
| 9116 | ANKRD17 | NM_032217.3 | 6869 | ttgggccctttagcacattgttt | 15883 | 742713 | see also 9099 line | | |
| 9117 | ANKRD17 | NM_032217.3 | 6870 | tgggccctttagcacattgtttg | 15884 | 742714 | see also 9099 line | | |
| 9118 | ANKRD17 | NM_032217.3 | 7065 | tccaagtccctcaggtattgtca | 15892 | 742720 | see also 9099 line | | |
| 9119 | ANKRD17 | NM_032217.3 | 7610 | aagcaatggagcgagatagtaca | 15902 | 742734 | see also 9099 line | | |
| 9120 | CHD6 | NM_032221.3 | 2189 | ctgcagtctatcctaaaaccaat | 18414 | 450420 | - | - | - |
| 9121 | CHD6 | NM_032221.3 | 2648 | tacacctatgagcgaattgatgg | 18421 | 450432 | see also 9120 line | | |
| 9122 | CHD6 | NM_032221.3 | 2698 | agccatcgaccggttctgtaagc | 18422 | 450433 | see also 9120 line | | |
| 9123 | FLJ22611 | NM_032226.1 | 300 | tggtggctatgagactatagaag | 61763 | 479545 | - | - | - |
| 9124 | FLJ22679 | NM_032227.1 | 908 | tggtcaccgaagtgaatttgaac | 398736 | 796943 | - | - | - |
| 9125 | FLJ22679 | NM_032227.1 | 1051 | atggctgggcccttgtgtaaata | 398737 | 796945 | see also 9124 line | | |
| 9126 | FLJ22679 | NM_032227.1 | 1052 | tggctgggcccttgtgtaaatat | 398738 | 796946 | see also 9124 line | | |
| 9127 | FLJ22875 | NM_032231.3 | 277 | aactatccgggacccagaaaagc | 398908 | 726291 | - | - | - |
| 9128 | FLJ23356 | NM_032237.2 | 741 | aacactaaacctttcaaagtacc | 83329 | 489177 | kinase_related | - | - |
| 9129 | ZNRF1 | NM_032268.3 | 1258 | ctgcctgtgcatctatcacaaaa | 399224 | 747080 | - | - | - |
| 9130 | AD158 | NM_032270.2 | 1131 | taccttatactggctgttctacc | 399266 | 749035 | - | - | - |
| 9131 | AD158 | NM_032270.2 | 2285 | ttccagatgaactctacttctgc | 399295 | 749050 | see also 9130 line | | |
| 9132 | MAF1 | NM_032272.2 | 886 | atgtgacatctacagctataacc | 399304 | 727312 | - | - | - |
| 9133 | DKFZp761 B0514 | NM_032289.1 | 1810 | gaggcgtggctggaagaaattct | 399397 | 735512 | - | - | - |
| 9134 | DKFZp761 B0514 | NM_032289.1 | 2184 | tgcggtctcatgagaataagttg | 399398 | 735518 | see also 9133 line | | |

Figure 46

| | Gene | Accession | | Sequence | | | | | Comment |
|---|---|---|---|---|---|---|---|---|---|
| 9135 | DKFZp761B0514 | NM_032289.1 | 2320 | aagccgttatgagacctatatcc | 399401 | 735522 | | | see also 9133 line |
| 9136 | DKFZp761C121 | NM_032290.1 | 409 | ttgctaaatgttcctcatcatta | 399413 | 750595 | | - | |
| 9137 | DKFZp761C121 | NM_032290.1 | 410 | tgctaaatgttcctcatcattaa | 399414 | 750596 | | | see also 9136 line |
| 9138 | DKFZp761C121 | NM_032290.1 | 609 | gacatcaatgttaaagacaatgc | 399423 | 750604 | | | see also 9136 line |
| 9139 | DKFZp761D221 | NM_032291.1 | 259 | atggggcaccaaatggatttat | 399444 | 754828 | | - | |
| 9140 | DKFZp761D221 | NM_032291.1 | 260 | tgggggcaccaaatggatttatg | 399445 | 754829 | | | see also 9139 line |
| 9141 | DKFZp761D221 | NM_032291.1 | 261 | ggggcaccaaatggatttatgc | 399446 | 754830 | | | see also 9139 line |
| 9142 | DKFZp761D221 | NM_032291.1 | 557 | cccgggtgcaattaaaaggaact | 399454 | 754834 | | | see also 9139 line |
| 9143 | DKFZp761D221 | NM_032291.1 | 978 | cccaagtctgtctgttaatgc | 399467 | 754837 | | | see also 9139 line |
| 9144 | DKFZp761D221 | NM_032291.1 | 1993 | atgccaataccaaggaattctgg | 399493 | 754850 | | | see also 9139 line |
| 9145 | DKFZp761J1523 | NM_032293.3 | 666 | gagatgttcagcaatgaaattgg | 297549 | 665006 | | - | |
| 9146 | CAMKK1 | NM_032294.2 | 578 | ctggcctacaacgaaagtgaaga | 75083 | 497064 | | kinase_related | |
| 9147 | SLC37A3 | NM_032295.1 | 1095 | ccgacaagctgtcatttggtac | 274417 | 656896 | | - | |
| 9148 | SLC37A3 | NM_032295.1 | 1170 | tggatgtgggtttctactttt | 274423 | 656907 | | | see also 9147 line |
| 9149 | SYT3 | NM_032298.1 | 1779 | tgcacttcagcgtcatgacttt | 275119 | 657603 | transporter | | |
| 9150 | MGC2714 | NM_032299.2 | 826 | atcaaagtatcgtgttatgaaca | 399528 | 740530 | | - | |
| 9151 | MGC3200 | NM_032305.1 | 320 | gaggcagctcccctacttcatcc | 399604 | 728540 | | - | |
| 9152 | PDIP46 | NM_032311.3 | 1132 | aagtgcaaccttcaacatgaatgg | 64903 | 802153 | | - | |
| 9153 | MGC11061 | NM_032312.2 | 601 | tggtctcattggattataaccatt | 399660 | 724945 | | - | |
| 9154 | GMRP-1 | NM_032320.3 | 370 | ttgcatagtagacctcgtaaact | 131518 | 530677 | | - | |
| 9155 | GMRP-1 | NM_032320.3 | 1139 | atgatggtgctcgtagacaattt | 131528 | 530684 | | | see also 9154 line |
| 9156 | GMRP-1 | NM_032320.3 | 1143 | tggtgctcgtagacaatttgaat | 131529 | 530685 | | | see also 9154 line |
| 9157 | GMRP-1 | NM_032320.3 | 1145 | gtgctcgtagacaatttgaatt | 131530 | 530686 | | | see also 9154 line |
| 9158 | GMRP-1 | NM_032320.3 | 1146 | tgctcgtagacaatttgaattt | 131531 | 530687 | | | see also 9154 line |
| 9159 | GMRP-1 | NM_032320.3 | 1537 | aagagtcggcatgtagacttca | 131539 | 530688 | | | see also 9154 line |
| 9160 | GMRP-1 | NM_032320.3 | 1539 | gagtcggcatgtagactttcagt | 131540 | 530689 | | | see also 9154 line |
| 9161 | GMRP-1 | NM_032320.3 | 1547 | atgtagactttcagtgtgtaaag | 131542 | 530690 | | | see also 9154 line |

Figure 46

| | | | | | | | | | |
|---|---|---|---|---|---|---|---|---|---|
| 9162 | GMRP-1 | NM_032320.3 | 1550 | tagactttcagtgtgtaaagagt | 131543 | 530691 | see also 9154 line | | |
| 9163 | ZDHHC16 | NM_032327.2 | 1067 | gaggtgagactagcatcgaaagg | 399760 | 714287 | - | - | - |
| 9164 | MGC12458 | NM_032328.1 | 435 | atgttgtctgctgcaattgatcc | 148208 | 542813 | - | - | - |
| 9165 | PHF6 | NM_032335.1 | 942 | aactgtacttcaggagattaaac | 35053 | 453807 | - | - | - |
| 9166 | MGC14799 | NM_032336.1 | 281 | agcaggcctggatgaatgaaaag | 399790 | 715045 | - | - | - |
| 9167 | MGC14799 | NM_032336.1 | 796 | cagcacttgatccgatacaaaac | 399799 | 715052 | see also 9166 line | | |
| 9168 | MGC14799 | NM_032336.1 | 797 | agcacttgatccgatacaaaacc | 399800 | 715053 | see also 9166 line | | |
| 9169 | MGC3047 | NM_032348.2 | 1241 | gacatccagctagattacaaaaa | 399865 | 715251 | - | - | - |
| 9170 | MGC13272 | NM_032355.2 | 840 | cagatggctacaaggtagtattc | 282002 | 666915 | - | - | - |
| 9171 | MGC13272 | NM_032355.2 | 844 | tggctacaaggtagtattcgtgc | 282003 | 666916 | see also 9170 line | | |
| 9172 | ACBD6 | NM_032360.2 | 516 | tagttctctatatcatgaagaaa | 172487 | 569848 | - | - | - |
| 9173 | ACBD6 | NM_032360.2 | 601 | accaaagccatcaaatcgaaaaa | 172489 | 569850 | see also 9172 line | | |
| 9174 | THOC3 | NM_032361.1 | 747 | ctgcatctgtatcaagtttgacc | 399985 | 735105 | - | - | - |
| 9175 | MGC16202 | NM_032373.2 | 397 | aggtgtggcaaccaagttcatga | 400026 | 739367 | - | - | - |
| 9176 | MGC2562 | NM_032374.2 | 156 | aagtcttgccatgattggatagg | 400047 | 725679 | - | - | - |
| 9177 | SYTL2 | NM_032379.2 | 1565 | tgggttcaggcctgaagatctga | 400092 | 540881 | - | - | - |
| 9178 | EFG2 | NM_032380.3 | 1848 | aagatcccagtttgaaagtgagg | 57363 | 477342 | - | - | - |
| 9179 | PRKWNK4 | NM_032387.2 | 531 | tggccgatacctcaagtttgaca | 97994 | 512546 | kinase_related | - | - |
| 9180 | PRKWNK4 | NM_032387.2 | 533 | gccgatacctcaagtttgacatc | 97995 | 512547 | see also 9179 line | | |
| 9181 | PRKWNK4 | NM_032387.2 | 534 | ccgatacctcaagtttgacatcg | 97996 | 512548 | see also 9179 line | | |
| 9182 | PRKWNK4 | NM_032387.2 | 1254 | ctgcatccgcacggataagaacg | 98001 | 512554 | see also 9179 line | | |
| 9183 | PRKWNK4 | NM_032387.2 | 3065 | ggcgtttccaagtgacttcatcc | 98021 | 512573 | see also 9179 line | | |
| 9184 | PINK1 | NM_032409.1 | 468 | gtcaggagatccaggcaattttt | 96834 | 511875 | kinase_related | - | - |
| 9185 | CARD11 | NM_032415.2 | 898 | aaggacgacaactacaacttagc | 129599 | 677018 | apoptosis | protein binding | - |
| 9186 | CARD11 | NM_032415.2 | 1973 | gccgcagcagcatcatgtcaatc | 129612 | 677028 | see also 9185 line | | |
| 9187 | HRD1 | NM_032431.1 | 370 | caccgtttttcgggatgacttca | 400222 | 735991 | - | - | - |
| 9188 | HRD1 | NM_032431.1 | 891 | gccgagccatccgcaacatgaac | 400227 | 736000 | see also 9187 line | | |

EP 1 752 536 A1

Figure 46

EP 1 752 536 A1

| 9189 | KIAA1796 | NM_032439.1 | 794 | ttgcaggacgcatgcttaagttt | 400298 | 802014 | - | - | - |
|---|---|---|---|---|---|---|---|---|---|
| 9190 | KIAA1796 | NM_032439.1 | 1050 | ccccaatactaacttatactttg | 400304 | 802027 | see also 9189 line | | |
| 9191 | KIAA1796 | NM_032439.1 | 1159 | cagcgccttcctcaactaaattc | 400308 | 802035 | see also 9189 line | | |
| 9192 | KIAA1796 | NM_032439.1 | 1160 | agcgccttcctcaactaaattct | 400309 | 802036 | see also 9189 line | | |
| 9193 | KIAA1796 | NM_032439.1 | 1198 | ttgacctgtacagaagaagatgg | 400311 | 802039 | see also 9189 line | | |
| 9194 | KIAA1796 | NM_032439.1 | 1314 | tggtcatcagctcatgagtttgt | 400313 | 802042 | see also 9189 line | | |
| 9195 | MLR2 | NM_032440.1 | 550 | cagcgaatgatccaacaatttgc | 400316 | 771919 | - | - | - |
| 9196 | MLR2 | NM_032440.1 | 635 | accaaagcctgccgaaagcatct | 400317 | 771922 | see also 9195 line | | |
| 9197 | MLR2 | NM_032440.1 | 1316 | aagttcgaggaatggatctttct | 400329 | 771942 | see also 9195 line | | |
| 9198 | MLR2 | NM_032440.1 | 1319 | ttcgaggaatggatctttcttgg | 400330 | 771943 | see also 9195 line | | |
| 9199 | MLR2 | NM_032440.1 | 1352 | ctggtgatcagtacagctatagc | 400331 | 771945 | see also 9195 line | | |
| 9200 | MLR2 | NM_032440.1 | 1365 | cagctatagctctttggtaatgg | 400332 | 771947 | see also 9195 line | | |
| 9201 | MLR2 | NM_032440.1 | 1395 | aacggagagcgcgcttagtaaaa | 400333 | 771948 | see also 9195 line | | |
| 9202 | MLR2 | NM_032440.1 | 1396 | acggagagcgcgcttagtaaaaa | 400334 | 771949 | see also 9195 line | | |
| 9203 | MLR2 | NM_032440.1 | 1399 | gagagcgcgcttagtaaaaaatt | 400335 | 771951 | see also 9195 line | | |
| 9204 | MLR2 | NM_032440.1 | 1401 | gagcgcgcttagtaaaaaattaa | 400336 | 771952 | see also 9195 line | | |
| 9205 | MLR2 | NM_032440.1 | 1402 | agcgcgcttagtaaaaaattaag | 400337 | 771953 | see also 9195 line | | |
| 9206 | MLR2 | NM_032440.1 | 1403 | gcgcgcttagtaaaaaattaagg | 400338 | 771954 | see also 9195 line | | |
| 9207 | MLR2 | NM_032440.1 | 1422 | aagggctattcttccaaaacaaa | 400339 | 771955 | see also 9195 line | | |
| 9208 | MLR2 | NM_032440.1 | 1575 | agggcgttacagacagtacaaca | 400342 | 771957 | see also 9195 line | | |
| 9209 | MLR2 | NM_032440.1 | 1577 | ggcgttacagacagtacaacagt | 400343 | 771958 | see also 9195 line | | |
| 9210 | MLR2 | NM_032440.1 | 1681 | cacagtacactggagtacaaagt | 400345 | 771960 | see also 9195 line | | |
| 9211 | PHF6 | NM_032458.1 | 1055 | cagcctggtgctactattggatg | 35060 | 453809 | - | - | - |
| 9212 | MRPL38 | NM_032478.2 | 1403 | gcggaccttccgcacttttgatt | 400356 | 714680 | - | - | - |
| 9213 | DOT1L | NM_032482.1 | 730 | atcgccaacacgagtgttatatt | 25543 | 816774 | transcription_regulator | - | - |
| 9214 | DOT1L | NM_032482.1 | 731 | tcgccaacacgagtgttatattt | 25544 | 816775 | see also 9213 line | | |
| 9215 | DOT1L | NM_032482.1 | 732 | cgccaacacgagtgttatatttg | 25545 | 816776 | see also 9213 line | | |
| 9216 | DOT1L | NM_032482.1 | 986 | tcgaccgcaccatacttgaaaac | 25550 | 816782 | see also 9213 line | | |
| 9217 | DOT1L | NM_032482.1 | 989 | accgcaccatacttgaaaactat | 25551 | 816783 | see also 9213 line | | |
| 9218 | MGC3248 | NM_032486.2 | 100 | gagacggcatctgggaacaaagt | 400371 | 712032 | - | - | - |
| 9219 | MGC3248 | NM_032486.2 | 102 | gacggcatctgggaacaaagtca | 400372 | 712033 | see also 9218 line | | |
| 9220 | GL009 | NM_032492.2 | 335 | aggtggccatgccctatcagtgg | 400395 | 724330 | - | - | - |
| 9221 | AP1M1 | NM_032493.2 | 594 | ggcatcaagtatcggaagaatga | 267495 | 650417 | - | protein transporter | - |
| 9222 | ACAS2L | NM_032501.2 | 1485 | tccttggctgggaggatcaatga | 232528 | 617446 | - | - | - |
| 9223 | LOC84549 | NM_032509.2 | 241 | gggaacgggtccggcttagtaaa | 63794 | 478778 | - | - | - |
| 9224 | LOC84549 | NM_032509.2 | 244 | aacgggtccggcttagtaaaaac | 63795 | 478779 | see also 9223 line | | |

Figure 46

| | | | | | | | |
|---|---|---|---|---|---|---|---|
| 9225 | LOC84549 | NM_032509.2 | 245 | acgggtccggcttagtaaaaact | 63796 | 478780 | see also 9223 line |
| 9226 | LOC84549 | NM_032509.2 | 247 | gggtccggcttagtaaaaactat | 63797 | 478781 | see also 9223 line |
| 9227 | LOC84549 | NM_032509.2 | 249 | gtccggcttagtaaaaactatga | 63798 | 478782 | see also 9223 line |
| 9228 | LOC84549 | NM_032509.2 | 251 | ccggcttagtaaaaactatgaga | 63799 | 478783 | - |
| 9229 | C6orf168 | NM_032511.1 | 528 | tccacgaactcctggtcattagg | 400480 | 786924 | - |
| 9230 | C6orf168 | NM_032511.1 | 593 | cagtttgcaagacctaacaatgg | 400482 | 786926 | see also 9229 line |
| 9231 | C6orf168 | NM_032511.1 | 754 | ttctggcacagaattcataattg | 400487 | 786930 | see also 9229 line |
| 9232 | CAB56184 | NM_032520.2 | 238 | tggtggagtccacgtacaagtat | 400545 | 775321 | - |
| 9233 | CAB56184 | NM_032520.2 | 655 | ttgaggatgctggctacttaaag | 400548 | 775334 | see also 9232 line |
| 9234 | MGC2629 | NM_032522.2 | 327 | ctgtgatattgtggtcaatgtgc | 133864 | 534137 | - |
| 9235 | MGC2629 | NM_032522.2 | 451 | gtcatcaagaaccctactgtttt | 133868 | 534142 | see also 9234 line |
| 9236 | MGC2629 | NM_032522.2 | 565 | atgcagcatattatagacacaaatg | 133871 | 534145 | see also 9234 line |
| 9237 | OSBPL6 | NM_032523.2 | 1271 | aggctaactgtgtagatatttca | 303597 | 672356 | - |
| 9238 | KIRREL3 | NM_032531.1 | 109 | gacaagtttcggagaatgaatga | 400550 | 723964 | - |
| 9239 | KIRREL3 | NM_032531.1 | 112 | aagtttcggagaatgaatgaagg | 400551 | 723965 | see also 9238 line |
| 9240 | KIRREL3 | NM_032531.1 | 1680 | tgcccgttcccagagaaatctca | 400564 | 723981 | see also 9238 line |
| 9241 | KIRREL3 | NM_032531.1 | 1914 | caggtcaacaccttcaaagagc | 400567 | 723987 | see also 9238 line |
| 9242 | ABTB1 | NM_032548.2 | 382 | tgcaggaggcgggattactatga | 128494 | 531575 | - |
| 9243 | ABTB1 | NM_032548.2 | 384 | caggaggcgggattactatgacg | 128495 | 531576 | see also 9242 line |
| 9244 | IL1F10 | NM_032556.4 | 432 | tccccatggcaagatactacata | 167877 | 565050 | - |
| 9245 | IL1F10 | NM_032556.4 | 434 | cccatggcaagatactacataat | 167878 | 565052 | see also 9244 line |
| 9246 | USP38 | NM_032557.4 | 3611 | tacagttgcagactcgtatttt | 189872 | 581896 | - |
| 9247 | FLJ14753 | NM_032558.1 | 451 | ttcatagctatggtaggaattct | 257938 | 642243 | - |
| 9248 | DGAT2 | NM_032564.2 | 683 | gaccaccaggaactatatctttg | 216209 | 602923 | - |
| 9249 | DGAT2 | NM_032564.2 | 1117 | gccgatgggtccagaagaagttc | 216216 | 602930 | see also 9248 line |
| 9250 | N-PAC | NM_032569.2 | 81 | ttggccagaaagattgttaatc | 33379 | 453613 | - |
| 9251 | N-PAC | NM_032569.2 | 1654 | gccgtgtaccgagcctacataca | 33400 | 453635 | see also 9250 line |
| 9252 | DRCTNNB1A | NM_032581.2 | 1059 | ttgctggttgctaatgcaataaa | 254391 | 745513 | - |
| 9253 | DRCTNNB1A | NM_032581.2 | 1060 | tgctggttgctaatgcaataaag | 254392 | 745514 | see also 9252 line |
| 9254 | DRCTNNB1A | NM_032581.2 | 1116 | aaggaagttacaaggtgtattca | 254396 | 745517 | see also 9252 line |
| 9255 | DRCTNNB1A | NM_032581.2 | 1267 | ctgaagttgacgagggatttat | 254401 | 745522 | see also 9252 line |
| 9256 | DRCTNNB1A | NM_032581.2 | 1270 | aagttgacgagggattttattcc | 254402 | 745523 | see also 9252 line |
| 9257 | PHACS | NM_032592.1 | 1631 | ctgggttgacttgagagaagtacc | 218292 | 606097 | - |

Figure 46

| | | | | | | | | | |
|---|---|---|---|---|---|---|---|---|---|
| 9258 | LOXL3 | NM_032603.2 | 1354 | gggcagtcgagtgcaaatagg | 163132 | 560846 | receptor_acitivity | scavenger receptor | - |
| 9259 | LOXL3 | NM_032603.2 | 2120 | atgtgaagccaggaaactacatt | 163136 | 560851 | see also 9258 line | | |
| 9260 | LOXL3 | NM_032603.2 | 2122 | gtgaagccaggaaactacattct | 163137 | 560852 | see also 9258 line | | - |
| 9261 | LNX | NM_032622.1 | 1287 | atctgattcaggccagtgaaaga | 316557 | 681119 | - | | |
| 9262 | OSAP | NM_032623.2 | 231 | cagtcagtgctggtggatattat | 401135 | 724308 | - | | |
| 9263 | SSBP4 | NM_032627.1 | 1330 | cccgttcccgagcgaaagctact | 19886 | 452008 | transcription_regulator | - | |
| 9264 | PAPOLA | NM_032632.2 | 1003 | tagcatcaactcttgtacataaa | 53212 | 474806 | transcription_regulator | polynucleotide adenylyltransferase | - |
| 9265 | PIGO | NM_032634.2 | 903 | tgcaggaaggcgtgtagtcttca | 205434 | 596212 | - | | |
| 9266 | PIGO | NM_032634.2 | 1119 | tggccctcaccaccctgaaatgg | 205436 | 596213 | see also 9265 line | | |
| 9267 | PIGO | NM_032634.2 | 2381 | gtggtcgagccaagaatttgtgg | 205452 | 596233 | see also 9265 line | | |
| 9268 | PIGO | NM_032634.2 | 2455 | tggcttcgccgctatggtaatct | 205454 | 596234 | see also 9265 line | | |
| 9269 | PIGO | NM_032634.2 | 2459 | ttcgccgctatggtaatctcaag | 205455 | 596235 | see also 9265 line | | |
| 9270 | PIGO | NM_032634.2 | 2460 | tcgccgctatggtaatctcaaga | 205456 | 596236 | see also 9265 line | | |
| 9271 | GATA2 | NM_032638.3 | 854 | accaagccacccaaagaagtgt | 4420 | 444339 | | translation regulator | |
| 9272 | FOXP1 | NM_032682.3 | 1420 | agcatgcgtgacgatagaagt | 4199 | 444170 | | transcription factor | |
| 9273 | FOXP1 | NM_032682.3 | 1739 | caggcggtactcagacaaataca | 4205 | 444177 | see also 9272 line | | |
| 9274 | FOXP1 | NM_032682.3 | 1816 | cagaagttagcaccacatttaca | 4207 | 444181 | see also 9272 line | | |
| 9275 | FOXP1 | NM_032682.3 | 2081 | tggtaacccttccttattaaaa | 4214 | 444190 | see also 9272 line | | |
| 9276 | FOXP1 | NM_032682.3 | 2121 | gcctactgcacacctctcaatgc | 4215 | 444191 | see also 9272 line | | |
| 9277 | MGC11332 | NM_032718.2 | 937 | cagtcatgctgtactacagtaac | 257995 | 642222 | - | | |
| 9278 | RTN4IP1 | NM_032730.2 | 466 | tgcctatcataacactatccaaat | 159893 | 559571 | - | | |
| 9279 | RTN4IP1 | NM_032730.2 | 507 | cacgctgccagtgtaaatcctat | 159895 | 559575 | see also 9278 line | | |
| 9280 | RTN4IP1 | NM_032730.2 | 1349 | gggaaagatccgcccagttattg | 159928 | 559599 | see also 9278 line | | |
| 9281 | MINA53 | NM_032778.3 | 982 | agcacctaccagaacaattcatg | 401433 | 720059 | - | | |
| 9282 | THSD2 | NM_032784.2 | 283 | atgcaacttgcgactgatttcttg | 401487 | 734174 | - | | |
| 9283 | THSD2 | NM_032784.2 | 537 | tggaactcgatatccagatataa | 401494 | 734181 | see also 9282 line | | |
| 9284 | THSD2 | NM_032784.2 | 540 | aactcgatatccagatataaata | 401495 | 734182 | see also 9282 line | | |
| 9285 | AMID | NM_032797.4 | 395 | ttgccaaaagacattcatttc | 224758 | 611097 | apoptosis | | |
| 9286 | SPPL2A | NM_032802.2 | 1073 | caggtgggcttggatttacagg | 191737 | 584637 | - | | |
| 9287 | SPPL2A | NM_032802.2 | 1388 | accaggccgtttgattgcatact | 191744 | 584644 | see also 9286 line | | |
| 9288 | FBXO18 | NM_032807.3 | 1123 | gtgtgctgaactcatcatacgatac | 234579 | 706032 | - | | |
| 9289 | FBXO18 | NM_032807.3 | 1466 | taggattcactcaacaactttct | 234582 | 706039 | see also 9288 line | | |
| 9290 | FBXO18 | NM_032807.3 | 1492 | gcctatatcttcaggagaattcc | 234583 | 706040 | see also 9288 line | | |
| 9291 | FBXO18 | NM_032807.3 | 2275 | cgcaccagagatctataacttc | 234604 | 706059 | see also 9288 line | | |

Figure 46

| 9292 | FLJ14600 | NM_032810.2 | 649 | gagtgatatagcaggtttagatg | 82973 | 489123 | - | - | - |
|------|----------|-------------|------|-------------------------|--------|--------|---|---|---|
| 9293 | FLJ14600 | NM_032810.2 | 1382 | gacctgcatcgggcaattgaaaa | 82990 | 489143 | see also 9292 line | | |
| 9294 | SLC35B4 | NM_032826.2 | 473 | caccagctatcccaataaggtac | 272173 | 711509 | - | - | - |
| 9295 | SLC35B4 | NM_032826.2 | 474 | accagctatcccaataaggtact | 272174 | 711510 | see also 9294 line | | |
| 9296 | SLC35B4 | NM_032826.2 | 1017 | gtggttctacctcctcatgaaca | 272189 | 711526 | see also 9294 line | | |
| 9297 | HATH6 | NM_032827.3 | 865 | ttcctactcgtcaatttcacacg | 401731 | 770982 | - | - | - |
| 9298 | HATH6 | NM_032827.3 | 871 | ctcgtcaatttcacacgtaattt | 401732 | 770983 | see also 9297 line | | |
| 9299 | HATH6 | NM_032827.3 | 872 | tcgtcaatttcacacgtaattta | 401733 | 770984 | see also 9297 line | | |
| 9300 | PPP1R15B | NM_032833.1 | 2340 | aagaagttactgagtattatata | 401863 | 748480 | - | - | - |
| 9301 | PPP1R15B | NM_032833.1 | 2413 | atgcaggttccagaaacgaattc | 401864 | 748481 | see also 9300 line | | |
| 9302 | PPP1R15B | NM_032833.1 | 2414 | tgcaggttccagaaacgaattca | 401865 | 748482 | see also 9300 line | | |
| 9303 | PPP1R15B | NM_032833.1 | 2416 | caggttccagaaacgaattcaag | 401866 | 748483 | see also 9300 line | | |
| 9304 | DIRC2 | NM_032839.1 | 438 | tgccctgcttcgcgttcatgtgg | 401886 | 770370 | - | - | - |
| 9305 | FLJ14803 | NM_032842.1 | 150 | ggctggaatgatatatactgaaa | 401926 | 802068 | - | - | - |
| 9306 | FLJ14803 | NM_032842.1 | 927 | ggcccatgtgtctaacaaagatg | 401955 | 802090 | see also 9305 line | | |
| 9307 | FLJ14803 | NM_032842.1 | 1399 | acctaccttgattccagattacc | 401970 | 802108 | see also 9305 line | | |
| 9308 | FLJ14803 | NM_032842.1 | 1653 | aggaatgcttgggagagttaatc | 401974 | 802123 | see also 9305 line | | |
| 9309 | FLJ14813 | NM_032844.1 | 537 | aaccggacaatatgcttatttct | 83000 | 488814 | kinase_related | - | - |
| 9310 | RAB2B | NM_032846.2 | 373 | aaccacctgacctcatggttaga | 110112 | 452900 | signal_transduction | - | - |
| 9311 | FLJ14834 | NM_032849.2 | 612 | aactgaccaactactgtgattat | 402008 | 729749 | - | - | - |
| 9312 | FLJ14834 | NM_032849.2 | 614 | ctgaccaactactgtgattataa | 402009 | 729750 | see also 9311 line | | |
| 9313 | ZFYVE19 | NM_032850.3 | 451 | gtggtcaccacctcagaactata | 161374 | 554951 | - | - | - |
| 9314 | LACTB | NM_032857.2 | 657 | ttctgtcacaacaagattactga | 285484 | 658350 | - | - | - |
| 9315 | LACTB | NM_032857.2 | 690 | aagtggaattcgtcattatgaaa | 285487 | 658351 | see also 9314 line | | |
| 9316 | LACTB | NM_032857.2 | 1124 | aagaaaacgagccagtgatttac | 285495 | 658364 | see also 9314 line | | |
| 9317 | LACTB | NM_032857.2 | 1201 | tacgtggataactcctataaatg | 285498 | 658368 | see also 9314 line | | |
| 9318 | FLJ14904 | NM_032858.1 | 585 | caggctgcaagtgattctagtca | 402105 | 787726 | - | - | - |
| 9319 | FLJ14904 | NM_032858.1 | 642 | aaccaagcaactgtgttacaaaa | 402107 | 787729 | see also 9318 line | | |
| 9320 | FLJ14904 | NM_032858.1 | 976 | ttgcgtactgcatcagtaattct | 402118 | 787744 | see also 9318 line | | |
| 9321 | FLJ14904 | NM_032858.1 | 1244 | aggaattacccgcttactagaga | 402128 | 787749 | see also 9318 line | | |
| 9322 | C6orf93 | NM_032860.3 | 500 | ttcaggacctcgactggatttg | 402140 | 806079 | - | - | - |
| 9323 | C6orf93 | NM_032860.3 | 1253 | aggaatacctctcaatgtcttac | 402160 | 806101 | see also 9322 line | | |
| 9324 | FLJ14936 | NM_032864.2 | 649 | gagagagtctgtgatatcattct | 402180 | 777615 | - | - | - |
| 9325 | CML66 | NM_032869.2 | 529 | atgggagattatgtttaatgaag | 402286 | 721827 | - | - | melanoma、non-small-cell lung cancer |
| 9326 | CML66 | NM_032869.2 | 796 | tggaaatggtctaatgattgtat | 402299 | 721841 | see also 9325 line | | |
| 9327 | CML66 | NM_032869.2 | 1325 | agctccagtttatgcagatttga | 402312 | 721862 | see also 9325 line | | |
| 9328 | CML66 | NM_032869.2 | 1591 | ctcgtatgcagcccttgtgagt | 402318 | 721869 | see also 9325 line | | |

Figure 46

| 9329 | KIAA1959 | NM_032873.2 | 1827 | tgggttgctgggagcacattacc | 236684 | 621056 | - |
|---|---|---|---|---|---|---|---|
| 9330 | JUB | NM_032876.4 | 1732 | caccgactaccacacaaaaattatg | 273698 | 701351 | - |
| 9331 | MGC15730 | NM_032880.2 | 452 | cggctacctgacagtcaacattg | 402394 | 813532 | receptor_acitivity |
| 9332 | MGC15730 | NM_032880.2 | 696 | gaggtccggatctcagacaatgg | 402404 | 813535 | see also 9331 line |
| 9333 | MGC15730 | NM_032880.2 | 709 | cagacaatggtccctatgagtgc | 402405 | 813536 | see also 9331 line |
| 9334 | COL27A1 | NM_032888.2 | 663 | gacgcaggtcgctcacaattact | 288087 | 659404 | - |
| 9335 | COL27A1 | NM_032888.2 | 667 | caggtcgctcacaattactgtac | 288089 | 659405 | see also 9334 line |
| 9336 | COL27A1 | NM_032888.2 | 5012 | acctcatccagagcattaagacg | 288108 | 659423 | see also 9334 line |
| 9337 | DISP1 | NM_032890.2 | 1121 | tagataattccaggatcagatct | 264471 | 633009 | - |
| 9338 | MGC14161 | NM_032892.1 | 2086 | tcgaacaattccactatcaatac | 402456 | 476222 | - |
| 9339 | MGC14161 | NM_032892.1 | 2089 | aacaattccactatcaatacttt | 402457 | 476223 | see also 9338 line |
| 9340 | MGC14161 | NM_032892.1 | 2122 | ggcgatggtttgcctgcaaaatc | 402458 | 476224 | see also 9338 line |
| 9341 | MGC14128 | NM_032899.3 | 1004 | gtgccaagtcaggcaggaaattc | 402474 | 784666 | - |
| 9342 | SPF45 | NM_032905.3 | 1223 | cagtacggatatttagaattt | 66243 | 485809 | rhodopsin-like receptor |
| 9343 | SPF45 | NM_032905.3 | 1301 | gttggacgggtggtaaaagcatgt | 66245 | 485811 | see also 9342 line |
| 9344 | MGC14156 | NM_032906.2 | 336 | ttgattggatccctaatatgata | 402500 | 717283 | - |
| 9345 | RERG | NM_032918.1 | 367 | ttctgaccaaacggttcatctgg | 110620 | 520274 | signal_transduction |
| 9346 | C7orf35 | NM_032936.2 | 463 | tggtacctcattgcaatgttact | 402564 | 797111 | - |
| 9347 | SYTL2 | NM_032943.1 | 978 | cagcttccagtgtgattgattatg | 402575 | 540825 | - |
| 9348 | SYTL2 | NM_032943.1 | 2090 | atgcttccaaaagtctagaaga | 402602 | 540853 | see also 9347 line |
| 9349 | AXUD1 | NM_033027.2 | 1736 | tccaggctctgccagattatagt | 311367 | 676918 | apoptosis |
| 9350 | AXUD1 | NM_033027.2 | 1738 | caggctctgccagattatagtct | 311368 | 676919 | see also 9349 line |
| 9351 | BOLL | NM_033030.3 | 620 | aacattggtccagcaataagaaa | 58201 | 479689 | - |
| 9352 | BOLL | NM_033030.3 | 625 | tggtccagcaataagaaagaaacaac | 58202 | 479690 | see also 9351 line |
| 9353 | DMRTC2 | NM_033052.1 | 237 | caggcttgcgagtgtcacaaatg | 2752 | 443099 | transcription_regulator |
| 9354 | DMRTC2 | NM_033052.1 | 242 | ttgccgagtgtcacaaatgtgtcc | 2753 | 443101 | see also 9353 line |
| 9355 | HIAT1 | NM_033055.2 | 75 | cggaggcacgcctcaaggaatag | 257952 | 642265 | - |
| 9356 | HIAT1 | NM_033055.2 | 198 | tcctaaacatacatttctgatga | 257957 | 642274 | see also 9355 line |

Figure 46

| | | | | | | | | | |
|---|---|---|---|---|---|---|---|---|---|
| 9357 | Prostein | NM_033102.1 | 1873 | gggtctggtcgccatttactttg | 403256 | 612771 | - | - | - |
| 9358 | STN2 | NM_033104.2 | 2558 | atgccttcaactccattgtgtgg | 305073 | 672946 | - | - | - |
| 9359 | STN2 | NM_033104.2 | 2771 | acgtcaggaagtgggtcaattat | 305081 | 672950 | see also 9358 line | | |
| 9360 | STN2 | NM_033104.2 | 2773 | gtcaggaagtgggtcaattattc | 305082 | 672951 | see also 9358 line | | |
| 9361 | MADP-1 | NM_033114.2 | 407 | cagttatttggtagagtgataaa | 63956 | 479071 | - | - | - |
| 9362 | MADP-1 | NM_033114.2 | 477 | ggcgaaactacttttgataaatct | 63961 | 479076 | see also 9361 line | | |
| 9363 | NEK9 | NM_033116.3 | 38 | gacactgcgattccatcaactcg | 94733 | 510276 | kinase_related、cell_cycle | - | - |
| 9364 | NEK9 | NM_033116.3 | 598 | ggcctagcaaagaaacttaattc | 94747 | 510284 | see also 9363 line | | |
| 9365 | NEK9 | NM_033116.3 | 1041 | atcacgaaccagtgaagtctata | 94763 | 510294 | see also 9363 line | | |
| 9366 | NEK9 | NM_033116.3 | 1422 | acccatgcagctgaacttcttcc | 94773 | 510306 | see also 9363 line | | |
| 9367 | NEK9 | NM_033116.3 | 1669 | gtgctggcctgtggactcaatga | 94781 | 510310 | see also 9363 line | | |
| 9368 | MGC2734 | NM_033117.2 | 178 | aggaaggacaccgattatggatt | 64357 | 478551 | - | - | - |
| 9369 | NKD1 | NM_033119.3 | 1243 | acgggagcaagcactttgtgagg | 149111 | 550621 | - | - | - |
| 9370 | PLCZ1 | NM_033123.2 | 1232 | ggccttatctgatcttgtcattt | 151250 | 552483 | - | - | - |
| 9371 | PLCZ1 | NM_033123.2 | 1233 | gccttatctgatcttgtcattta | 151251 | 552484 | see also 9370 line | | |
| 9372 | PLCZ1 | NM_033123.2 | 1920 | tgcatgaacaaaggttatcgtcg | 151277 | 552521 | see also 9370 line | | |
| 9373 | RGPR | NM_033127.1 | 1404 | tgcgcagatcgtggagaaattca | 403519 | 744089 | - | - | - |
| 9374 | RGPR | NM_033127.1 | 1959 | cccttctttccaggtgtataagc | 403527 | 744097 | see also 9373 line | | |
| 9375 | RGPR | NM_033127.1 | 1962 | ttctttccaggtgtataagctcc | 403528 | 744098 | see also 9373 line | | |
| 9376 | ZIC5 | NM_033132.2 | 346 | aacccgccagcgctgagattagc | 44021 | 712681 | - | - | - |
| 9377 | ZIC5 | NM_033132.2 | 1224 | cggctacggagccgtgaacttaa | 44024 | 712682 | see also 9376 line | | |
| 9378 | BC008967 | NM_033201.1 | 632 | ctgatttcctgagaatcaagtta | 403575 | 753124 | - | - | - |
| 9379 | BC008967 | NM_033201.1 | 657 | acctctagacaaagtaaccaaat | 403576 | 753126 | see also 9378 line | | |
| 9380 | LOC90355 | NM_033211.2 | 772 | tggactacctcaatctagataaa | 403590 | 748505 | - | - | - |
| 9381 | GABRG3 | NM_033223.1 | 1412 | ctgggttggatacctgtatctct | 178776 | 571998 | channel、receptor_acitivity、signal_transduction | GABA-A receptor | - |
| 9382 | PURB | NM_033224.2 | 865 | acgacagagggataagctttatg | 403620 | 465069 | - | - | - |
| 9383 | CSMD1 | NM_033225.1 | 10724 | accccatgtatgatacaaactta | 202781 | 745807 | - | - | - |
| 9384 | ABCC12 | NM_033226.1 | 1072 | caccatcccgatcctaatggtct | 70122 | 492169 | - | - | - |
| 9385 | ABCC12 | NM_033226.1 | 1488 | ttgccatgtttaatgtaatgaag | 70142 | 492182 | see also 9384 line | | |
| 9386 | PML | NM_033238.1 | 2334 | gacctacctggcgagaaacatga | 9591 | 463696 | transcription_regulator | - | leukemia |
| 9387 | BOC | NM_033254.2 | 2889 | cagcgacctgccctatctgattg | 22886 | 774826 | - | - | - |
| 9388 | BOC | NM_033254.2 | 2890 | agcgacctgccctatctgattgt | 22887 | 774827 | see also 9387 line | | |
| 9389 | SLC8A3 | NM_033262.3 | 975 | ttgccagggtcattgtctatttt | 122784 | 642616 | - | - | - |

Figure 46

| | | | | | | | | |
|---|---|---|---|---|---|---|---|---|
| 9390 | SLC8A3 | NM_033262.3 | 2639 | cagaaagcgctcctgttatctcc | 122807 | 642659 | see also 9389 line | |
| 9391 | SLC8A3 | NM_033262.3 | 2726 | ttgggtgaacacccaaactaga | 122808 | 642660 | see also 9389 line | |
| 9392 | KCNH7 | NM_033272.2 | 1156 | tgggatccacatcagattcaaac | 254528 | 644382 | channel, signal transduction, transcription regulator | |
| 9393 | KCNH7 | NM_033272.2 | 1920 | tggtatgcgattgggaatgtaga | 254551 | 644411 | see also 9392 line | |
| 9394 | LOC90701 | NM_033280.1 | 601 | atgggtgcatatgtgttactaaa | 191496 | 584596 | - | peptidase |
| 9395 | LOC90701 | NM_033280.1 | 602 | tgggtgcatatgtgttactaaaa | 191497 | 584597 | see also 9394 line | |
| 9396 | TP53INP1 | NM_033285.2 | 451 | ttcttgttgacttcatagatact | 403666 | 676882 | - | |
| 9397 | TP53INP1 | NM_033285.2 | 673 | caggtgattaaccactacatcaag | 403669 | 676884 | see also 9396 line | |
| 9398 | TP53INP1 | NM_033285.2 | 922 | ttcgccctccagtggataaaa | 403681 | 676890 | see also 9396 line | |
| 9399 | TP53INP1 | NM_033285.2 | 923 | tcgccctccagtggataaaag | 403682 | 676891 | see also 9396 line | |
| 9400 | SOX6 | NM_033326.1 | 855 | ctccgctcatgatcccaatttt | 11804 | 468005 | transcription_regulator | transcription factor |
| 9401 | SOX6 | NM_033326.1 | 933 | tcccccctggaataacatacacaaa | 11806 | 468008 | see also 9400 line | |
| 9402 | SOX6 | NM_033326.1 | 965 | aactacccgtacagttcattcc | 11808 | 468009 | see also 9400 line | |
| 9403 | SOX6 | NM_033326.1 | 1945 | ctccaacattagcaaaatcttag | 11826 | 468020 | see also 9400 line | |
| 9404 | SOX6 | NM_033326.1 | 1946 | tccaacattagcaaaatcttagg | 11827 | 468021 | see also 9400 line | |
| 9405 | SOX6 | NM_033326.1 | 2209 | cacaggaacaggtgttgtgtatc | 11835 | 468025 | see also 9400 line | |
| 9406 | CAPPA3 | NM_033328.2 | 746 | caggtgtgtcaaccttcatattg | 113954 | 695710 | - | structural constituent of cytoskeleton |
| 9407 | CAPPA3 | NM_033328.2 | 749 | gtgtgtcaaccttcatattgaaa | 113955 | 695712 | see also 9406 line | |
| 9408 | CAPPA3 | NM_033328.2 | 1033 | gtgtgttgtgcaactggataata | 113972 | 695727 | see also 9406 line | |
| 9409 | LHX4 | NM_033343.2 | 1024 | tggccaccaaataagattatg | 6162 | 445342 | transcription_regulator | transcription factor | short stature, pituitary and cerebellar defects, and small sella turcica |
| 9410 | LHX4 | NM_033343.2 | 1025 | ggccaccaaataggatttatg | 6163 | 445343 | see also 9409 line | |
| 9411 | LHX4 | NM_033343.2 | 1058 | gacgttacaggcggacagttaat | 6164 | 445344 | see also 9409 line | |
| 9412 | LHX4 | NM_033343.2 | 1059 | acgttacagcggacagttaatg | 6165 | 445345 | see also 9409 line | |
| 9413 | LHX4 | NM_033343.2 | 1063 | tacaggcgacagttaatgaatg | 6166 | 445346 | see also 9409 line | |
| 9414 | AAT1 | NM_033364.2 | 1950 | gggaggaggacgagagatatttaag | 403753 | 593464 | - | |
| 9415 | SSH2 | NM_033389.2 | 278 | ctgtaagactggaagtacttac | 195990 | 798236 | phosphatase | |
| 9416 | SSH2 | NM_033389.2 | 992 | accgaggggtacggtatatcttg | 196011 | 798257 | see also 9415 line | |
| 9417 | SSH2 | NM_033389.2 | 993 | ccgaggggtacggtatatcttga | 196012 | 798258 | see also 9415 line | |
| 9418 | SSH2 | NM_033389.2 | 996 | aggggtacggtatatcttgaatg | 196013 | 798260 | see also 9415 line | |
| 9419 | SSH2 | NM_033389.2 | 1531 | atggtccacaagatgcaaatca | 196035 | 798283 | see also 9415 line | |
| 9420 | CASPR4 | NM_033401.2 | 3922 | tgccatagctgttgcatttatc | 318533 | 533863 | - | |
| 9421 | RWDD2 | NM_033411.2 | 962 | aagtgagcatgttttcagatac | 192215 | 585935 | phosphatase | |

Figure 46

| | | | | | | | | | |
|---|---|---|---|---|---|---|---|---|---|
| 9422 | ZPR9 | NM_033414.2 | 1295 | gtcatcgctccttgatgagatac | 56096 | 473441 | - | - | - |
| 9423 | ZPR9 | NM_033414.2 | 1302 | ctccttgatgagatactacaaac | 56099 | 473442 | see also 9422 line | | |
| 9424 | KIAA1737 | NM_033426.2 | 1117 | aacataggcgctttcagaatacc | 403891 | 784134 | - | - | - |
| 9425 | FLJ00007 | NM_033449.1 | 1604 | ttgtccctgagcgatatctcaac | 404034 | 790306 | - | - | - |
| 9426 | FLJ00007 | NM_033449.1 | 1608 | ccctgagcgatatctcaacttcc | 404035 | 790307 | see also 9425 line | | |
| 9427 | ABCC10 | NM_033450.2 | 2375 | agcctgcgccctcaatgatgacc | 70026 | 492123 | - | - | - |
| 9428 | KIAA1309 | NM_033495.1 | 1981 | accacaccatcaccttctagaga | 132874 | 532771 | - | protein binding | - |
| 9429 | LUZP1 | NM_033631.1 | 690 | ttgagcggcttcagaaacgaatg | 180703 | 715451 | - | - | - |
| 9430 | LUZP1 | NM_033631.1 | 1323 | accttcaggataattacctaagt | 180716 | 715464 | see also 9429 line | | |
| 9431 | MID2 | NM_052817.1 | 1522 | ctggccaggctaacttcatcagc | 157909 | 558441 | - | - | - |
| 9432 | CG018 | NM_052818.1 | 544 | ctgacactcgctggaaattcaac | 404256 | 749062 | - | - | - |
| 9433 | SLC26A7 | NM_052832.2 | 399 | gggataatgttggcagttcaaca | 260211 | 643756 | - | - | - |
| 9434 | BRUNOL6 | NM_052840.2 | 1335 | ttgttagttttgacaatccaact | 58230 | 480363 | - | - | - |
| 9435 | MGC20481 | NM_052849.2 | 696 | aggtggctccagccagataaagt | 404417 | 816665 | - | - | - |
| 9436 | CREB3L1 | NM_052854.1 | 634 | gactcagctgccagtgatcaaag | 2414 | 703684 | - | - | - |
| 9437 | MGC20398 | NM_052857.1 | 944 | tacggaatcgccaggatgaaata | 404472 | 719903 | - | - | - |
| 9438 | MGC20398 | NM_052857.1 | 945 | acggaatcgccaggatgaaataa | 404473 | 719904 | see also 9437 line | | |
| 9439 | RFT1 | NM_052859.2 | 196 | gactaacgctgctttactcaacc | 327309 | 799435 | - | - | - |
| 9440 | MGC10485 | NM_052875.2 | 656 | cgggcagatcgaactctactacg | 133836 | 672880 | - | - | - |
| 9441 | MGC10485 | NM_052875.2 | 983 | atccaaataccacttgaaagatg | 133845 | 672889 | see also 9440 line | | |
| 9442 | MGC10485 | NM_052875.2 | 1003 | atgtcattgtagggaagatatac | 133846 | 672890 | see also 9440 line | | |
| 9443 | SORCS1 | NM_052918.2 | 560 | cagcagcgtgattctcattttga | 327436 | 652645 | receptor_acitivity、 sig nal_transduction | - | - |
| 9444 | SORCS1 | NM_052918.2 | 579 | ttgacaaagctctatgactataa | 327438 | 652646 | see also 9443 line | | |
| 9445 | SORCS1 | NM_052918.2 | 581 | gacaaagctctatgactataacc | 327439 | 652647 | see also 9443 line | | |
| 9446 | SORCS1 | NM_052918.2 | 1436 | gggaatgttcttggctaacaaga | 327464 | 652665 | see also 9443 line | | |
| 9447 | SORCS1 | NM_052918.2 | 1447 | tggctaacaagaagattgacaac | 327465 | 652666 | see also 9443 line | | |
| 9448 | SORCS1 | NM_052918.2 | 2640 | gacagcgccgtcctgtacttaca | 327496 | 652696 | see also 9443 line | | |
| 9449 | SORCS1 | NM_052918.2 | 2642 | cagcgccgtcctgtacttacatg | 327497 | 652697 | see also 9443 line | | |
| 9450 | SORCS1 | NM_052918.2 | 2646 | gccgtcctgtacttacatgtaac | 327498 | 652699 | see also 9443 line | | |
| 9451 | SORCS1 | NM_052918.2 | 2647 | ccgtcctgtacttacatgtaact | 327499 | 652700 | see also 9443 line | | |
| 9452 | SORCS1 | NM_052918.2 | 3426 | gactcatctctccgattgcaaag | 327514 | 652713 | see also 9443 line | | |

Figure 46

| 9453 | LENG8 | NM_052925.1 | 2149 | accgaatcctctactacatcttc | 405331 | 778163 | receptor_acitivity | - | - |
|---|---|---|---|---|---|---|---|---|---|
| 9454 | SLC26A9 | NM_052934.2 | 478 | cagatggtgccaggtacctttgc | 260344 | 643528 | - | - | - |
| 9455 | SLC26A9 | NM_052934.2 | 488 | caggtacctttgccgttatcagc | 260345 | 643529 | see also 9454 line | | |
| 9456 | SLC26A9 | NM_052934.2 | 961 | cccatccctacagagatgattgt | 260351 | 643534 | see also 9454 line | | |
| 9457 | SLC26A9 | NM_052934.2 | 1419 | agccctgatcgctgtcaatctca | 260357 | 643538 | see also 9454 line | | |
| 9458 | SLC26A9 | NM_052934.2 | 1421 | ccctgatcgctgtcaatctcaag | 260358 | 643539 | see also 9454 line | | |
| 9459 | SLC26A9 | NM_052934.2 | 1427 | tcgctgtcaatctcaagaactcc | 260359 | 643540 | see also 9454 line | | |
| 9460 | LOC115294 | NM_052937.1 | 367 | aaccggatatttaagtacaatgg | 213683 | 808089 | - | - | - |
| 9461 | LOC115294 | NM_052937.1 | 379 | aagtacaatggtgggcttaattt | 213684 | 808090 | see also 9460 line | | |
| 9462 | LOC115294 | NM_052937.1 | 382 | tacaatggtgggcttaattttag | 213685 | 808091 | see also 9460 line | | |
| 9463 | MGC8974 | NM_052940.3 | 1081 | aggaatttgatcatagtaactgc | 405454 | 741174 | - | - | - |
| 9464 | NOSTRIN | NM_052946.2 | 618 | aagaattcaacttttatgcaata | 405477 | 806318 | - | - | - |
| 9465 | NOSTRIN | NM_052946.2 | 646 | aaccagtacagccaacatatttc | 405478 | 806320 | see also 9464 line | | |
| 9466 | NOSTRIN | NM_052946.2 | 647 | accagtacagccaacatatttct | 405479 | 806321 | see also 9464 line | | |
| 9467 | WDFY2 | NM_052950.2 | 315 | caggacagttcgtgtttggttaa | 160802 | 557007 | - | - | - |
| 9468 | WDFY2 | NM_052950.2 | 435 | aggtctagacaatggtacaatct | 160807 | 557009 | see also 9467 line | | |
| 9469 | WDFY2 | NM_052950.2 | 652 | gcctcaggcctgcaatttgatgt | 160812 | 557015 | see also 9467 line | | |
| 9470 | WDFY2 | NM_052950.2 | 654 | ctcaggcctgcaatttgatgttg | 160813 | 557016 | see also 9467 line | | |
| 9471 | WDFY2 | NM_052950.2 | 836 | cagatcactctgtcatcatgtgg | 160817 | 557021 | see also 9467 line | | |
| 9472 | PANX3 | NM_052959.2 | 831 | agcaatatacaccatattggttc | 327103 | 773988 | - | - | - |
| 9473 | HSPA12B | NM_052970.3 | 1941 | cagaggatgcgcgcttcatcacc | 85121 | 503817 | - | - | - |
| 9474 | USH3A | NM_052995.2 | 604 | gtggctgtcttgtcatgatattg | 313865 | 768805 | - | - | Usher syndrome |
| 9475 | TRALPUSH | NM_053002.2 | 873 | cagtattcagatgagtttgttca | 405625 | 799728 | - | - | - |
| 9476 | TRALPUSH | NM_053002.2 | 1117 | tggtttatggacttagttgtatg | 405629 | 799733 | see also 9475 line | | |
| 9477 | TRALPUSH | NM_053002.2 | 1268 | gggtgggaacacggctttcaatc | 405630 | 799736 | see also 9475 line | | |
| 9478 | TRALPUSH | NM_053002.2 | 1290 | cagcaggttcgggcaaggattta | 405632 | 799737 | see also 9475 line | | |
| 9479 | TRALPUSH | NM_053002.2 | 1291 | agcaggttcgggcaaggatttat | 405633 | 799738 | see also 9475 line | | |
| 9480 | HCCA2 | NM_053005.2 | 234 | accacatcaacctgcagtatagc | 405763 | 733960 | - | - | - |
| 9481 | PALM2 | NM_053016.1 | 668 | tggtaccaaagtagtgtatgagg | 405783 | 780087 | - | - | - |
| 9482 | PALM2 | NM_053016.1 | 1262 | ctgtcaatgctgtgttgtcatgt | 405797 | 780095 | see also 9481 line | | |

Figure 46

| 9483 | PFN2 | NM_053024.1 | 370 | tccatggaggcggattgaataag | 118743 | 523557 | - | phosphatidylinositol-4、5-bisphosphate binding | - |
|---|---|---|---|---|---|---|---|---|---|
| 9484 | PFN2 | NM_053024.1 | 373 | atggaggcggattgaataagaag | 118744 | 523558 | see also 9483 line | | |
| 9485 | MYLK | NM_053025.1 | 695 | cacctggctcaagggaaatgttc | 92488 | 509129 | kinase_related | - | - |
| 9486 | STAF42 | NM_053053.2 | 66 | gggggacaacgtgaaacaatact | 405909 | 741979 | - | - | - |
| 9487 | STAF42 | NM_053053.2 | 407 | atgacgacttgaaactttgttcc | 405920 | 741990 | see also 9486 line | | |
| 9488 | CATSPER1 | NM_053054.2 | 2023 | atgactggtccctcatctacatg | 262608 | 646196 | - | - | - |
| 9489 | CLIC6 | NM_053277.1 | 2015 | aagagattgaacacgcatattca | 263549 | 647166 | channel | - | - |
| 9490 | DACH2 | NM_053281.2 | 267 | ctgcctgccgcaagtctttgatc | 310586 | 676534 | transcription_regulator | - | - |
| 9491 | DACH2 | NM_053281.2 | 1745 | tgcaaggaggtaactattactgt | 310610 | 676560 | see also 9490 line | | |
| 9492 | DACH2 | NM_053281.2 | 1748 | aaggaggtaactattactgttta | 310611 | 676561 | see also 9490 line | | |
| 9493 | DACH2 | NM_053281.2 | 1749 | aggaggtaactattactgtttag | 310612 | 676562 | see also 9490 line | | |
| 9494 | EAT2 | NM_053282.1 | 255 | taccgaatcttcagagagaaaca | 279601 | 681001 | - | molecular_function unknown | Ewing's sarcoma |
| 9495 | TEKT1 | NM_053285.1 | 333 | aaggagttagatgacaaacttga | 292413 | 662202 | - | - | - |
| 9496 | TEKT1 | NM_053285.1 | 602 | ccgctctgccaagtacaatcttg | 292418 | 662207 | see also 9495 line | | |
| 9497 | CATSPER2 | NM_054020.2 | 1233 | atggttactaactttcagaatat | 262625 | 646241 | channel | - | - |
| 9498 | CATSPER2 | NM_054020.2 | 1234 | tggttactaactttcagaatatc | 262626 | 646242 | see also 9497 line | | |
| 9499 | ANKH | NM_054027.3 | 809 | tgcctcaatctcagatgtcatag | 260025 | 654095 | - | molecular_function unknown | arthritis |
| 9500 | LOC117584 | NM_057178.2 | 365 | ctgcttggactgtaagaaaaatt | 30724 | 452916 | - | - | - |
| 9501 | TWIST2 | NM_057179.1 | 499 | gcgacgagatggacaataagatg | 43201 | 449741 | - | - | - |
| 9502 | TECTB | NM_058222.1 | 433 | aacgggagcatgggcacatttga | 406231 | 659886 | - | - | - |
| 9503 | TECTB | NM_058222.1 | 785 | tacactgtgagacgttcatctgc | 406238 | 659897 | see also 9502 line | | |
| 9504 | KIAA1622 | NM_058237.1 | 1792 | ttcacgaactctatgcatttttc | 406307 | 737562 | - | - | - |
| 9505 | WNT7B | NM_058238.1 | 259 | ggcccgatgccatcattgtgatt | 255506 | 639708 | - | signal transducer | - |
| 9506 | TIAF1 | NM_078471.2 | 999 | gagatcgactggtggagattaat | 17674 | 509409 | phosphatase | molecular_function unknown | - |
| 9507 | TIAF1 | NM_078471.2 | 3496 | gcgcatgtaccgccaaggttacc | 17690 | 509432 | see also 9506 line | | |
| 9508 | TIAF1 | NM_078471.2 | 3792 | cccgccagcacttcaagaagaga | 17694 | 509435 | see also 9506 line | | |
| 9509 | SLC36A1 | NM_078483.1 | 1118 | ctggggtacctgcaatttggagc | 259642 | 641848 | - | - | - |
| 9510 | SCAMP-4 | NM_079834.1 | 199 | atctaccggctgtggatgtttta | 406361 | 708698 | - | - | - |

## Figure 46

| 9511 | SCAMP-4 | NM_079834.1 | 371 | tccgagccgacagctcctttaat | 406363 | 708702 | see also 9510 line | | |
|------|---------|-------------|-----|-------------------------|--------|--------|--------------------|---|---|
| 9512 | SCAMP-4 | NM_079834.1 | 372 | ccgagccgacagctcctttaatt | 406364 | 708703 | see also 9510 line | | |
| 9513 | ABCA9 | NM_080283.2 | 2433 | agggatcttgatagatgttctaa | 69388 | 491593 | - | - | - |
| 9514 | VPS18 | NM_080432.1 | 536 | tacactgctccgcattgacttgg | 271307 | 772112 | - | - | - |
| 9515 | B3GALT6 | NM_080605.2 | 620 | ctggcaactctgcgactactacc | 210043 | 599083 | - | - | - |
| 9516 | CBLNL1 | NM_080617.3 | 404 | tccaggttaacttgatgttaaat | 406456 | 790201 | - | - | - |
| 9517 | MGC17337 | NM_080655.1 | 299 | aaggctcggaccaaaaaaattat | 406530 | 732717 | - | - | - |
| 9518 | MGC17337 | NM_080655.1 | 300 | aggctcggaccaaaaaaattatg | 406531 | 732718 | see also 9517 line | | |
| 9519 | MGC17337 | NM_080655.1 | 301 | ggctcggaccaaaaaaattatgg | 406532 | 732719 | see also 9517 line | | |
| 9520 | MGC9564 | NM_080669.2 | 344 | cccactggaccctctacatgaac | 273954 | 656301 | - | - | - |
| 9521 | SYTL4 | NM_080737.1 | 1874 | aggtgaccggaaaaagagtaaag | 275187 | 540945 | - | - | - |
| 9522 | TMC2 | NM_080751.1 | 727 | atctttctccgatggatgtatgg | 327748 | 693521 | - | - | - |
| 9523 | TMC2 | NM_080751.1 | 738 | atggatgtatggagttaaccttg | 327749 | 693522 | see also 9522 line | | |
| 9524 | TMC2 | NM_080751.1 | 739 | tggatgtatggagttaaccttgt | 327750 | 693523 | see also 9522 line | | |
| 9525 | GPR82 | NM_080817.1 | 338 | ttgccataagccgctatgctacc | 240302 | 625193 | gpcr、receptor_acitivity、signal_transduction | - | - |
| 9526 | GPR82 | NM_080817.1 | 345 | aagccgctatgctaccttaatgc | 240303 | 625194 | see also 9525 line | | |
| 9527 | GPR82 | NM_080817.1 | 614 | ttggaaccacatttattggattt | 240315 | 625209 | see also 9525 line | | |
| 9528 | PABPC5 | NM_080832.1 | 1107 | gggccaactgagagtgttaaagt | 53176 | 485327 | - | - | - |
| 9529 | STK35 | NM_080836.2 | 231 | cggcagctacggcgtggtttatg | 101741 | 488238 | kinase_related | - | - |
| 9530 | STK35 | NM_080836.2 | 232 | ggcagctacggcgtggtttatga | 101742 | 488239 | see also 9529 line | | |
| 9531 | STK35 | NM_080836.2 | 234 | cagctacggcgtggtttatgagg | 101743 | 488240 | see also 9529 line | | |
| 9532 | SOCS7 | NM_080867.2 | 863 | aggtggcattttattaaacgaca | 310278 | 676387 | - | SH3-domain binding | - |
| 9533 | UNC5D | NM_080872.1 | 490 | ctctgtgcgcatagcctatttac | 293608 | 638522 | signal_transduction | - | - |
| 9534 | UNC5D | NM_080872.1 | 501 | tagcctatttacggaaaaacttt | 293609 | 638523 | see also 9533 line | | |
| 9535 | UNC5D | NM_080872.1 | 502 | agcctatttacggaaaaactttg | 293610 | 638524 | see also 9533 line | | |
| 9536 | UNC5D | NM_080872.1 | 1168 | tgccagcgacattgctttgtact | 293619 | 638533 | see also 9533 line | | |
| 9537 | UNC5D | NM_080872.1 | 1698 | ggcgcttagtaatgccaaataca | 293626 | 638541 | see also 9533 line | | |
| 9538 | UNC5D | NM_080872.1 | 1700 | cgcttagtaatgccaaatacagg | 293627 | 638542 | see also 9533 line | | |
| 9539 | UNC5D | NM_080872.1 | 2523 | aacgagaaaccatcactttcttc | 293635 | 638548 | see also 9533 line | | |
| 9540 | UNC5D | NM_080872.1 | 2876 | aactacagcaggcaaaatggact | 293641 | 638556 | see also 9533 line | | |
| 9541 | DUSP19 | NM_080876.2 | 369 | gacctgcaagttggcgttattaa | 192896 | 534931 | phosphatase | - | - |
| 9542 | ESDN | NM_080927.2 | 1032 | tggaacaattcctcatggatata | 319005 | 683035 | - | - | - |

Figure 46

| 9543 | ASB15 | NM_080928.2 | 363 | ctggatgcatcctataagacact | 315451 | 455008 | - | - | - |
|------|-------|-------------|-----|-------------------------|--------|--------|---|---|---|
| 9544 | ASB12 | NM_130388.2 | 369 | tggtggtagcatctacaacaact | 406896 | 746918 | - | - | - |
| 9545 | UBE3B | NM_130466.2 | 3329 | ccgagtggatccgaatgttctca | 231467 | 616715 | - | - | - |
| 9546 | JDP2 | NM_130469.2 | 487 | ccgatgccggaacaagaagaagg | 29143 | 460714 | transcription_regulator | - | - |
| 9547 | CACH-1 | NM_130767.1 | 1597 | ttggtggctggtcaaaatccatt | 199981 | 736298 | - | - | - |
| 9548 | CACH-1 | NM_130767.1 | 1598 | tggtggctggtcaaaatccattg | 199982 | 736299 | see also 9547 line | | |
| 9549 | RGS3 | NM_130795.1 | 1281 | ctggtgcctgtcggagaacatcg | 255015 | 639521 | signal_transduction、g pcr | | |
| 9550 | GREAT | NM_130806.2 | 968 | ctgcctagaaatcaaattggttt | 240458 | 623240 | gpcr、 receptor_acitivit y、 signal_transduction | - | - |
| 9551 | GREAT | NM_130806.2 | 969 | tgcctagaaatcaaattggtttt | 240459 | 623241 | see also 9550 line | | |
| 9552 | GREAT | NM_130806.2 | 970 | gcctagaaatcaaattggttttg | 240460 | 623242 | see also 9550 line | | |
| 9553 | GREAT | NM_130806.2 | 1452 | ctcacgctatgtccatcaaaatc | 240476 | 623262 | see also 9550 line | | |
| 9554 | DYX1C1 | NM_130810.1 | 1494 | ttctgtcaactagaattgtatgt | 407036 | 723916 | - | - | - |
| 9555 | DNCL2B | NM_130897.1 | 307 | cagaacgacctgacttttcttag | 130777 | 534677 | - | - | - |
| 9556 | PPIL3 | NM_130906.1 | 269 | ttgtgccagtaattactacaatg | 234788 | 729064 | - | - | - |
| 9557 | LRPPRC | NM_133259.2 | 1470 | atcctgatcaggaaacatataca | 281844 | 733412 | - | - | - |
| 9558 | LRPPRC | NM_133259.2 | 2452 | aagaggtgaaattgaaacagtaa | 281878 | 733444 | see also 9557 line | | |
| 9559 | LRPPRC | NM_133259.2 | 3395 | acgcaagttaggcgggattattt | 281900 | 733469 | see also 9557 line | | |
| 9560 | LRPPRC | NM_133259.2 | 3396 | cgcaagttaggcgggattatttg | 281901 | 733470 | see also 9557 line | | |
| 9561 | LRPPRC | NM_133259.2 | 3399 | aagttaggcgggattatttgaaa | 281902 | 733471 | see also 9557 line | | |
| 9562 | WIRE | NM_133264.2 | 832 | cagagacacaattctttgcatag | 407167 | 810640 | - | - | - |
| 9563 | GSH-2 | NM_133267.1 | 649 | ctcctggggacgcgcagttttgc | 4520 | 444442 | transcription_regulator | transcription factor | - |
| 9564 | KCNG3 | NM_133329.4 | 872 | ctccttctacaacgagatgatct | 260995 | 536485 | channel | - | - |
| 9565 | KCNG3 | NM_133329.4 | 1686 | tgctggtgggtgattatctctat | 261010 | 536493 | see also 9564 line | | |
| 9566 | YT521 | NM_133370.1 | 1622 | ctggatttgcaggcgtgaattac | 325133 | 797848 | - | - | - |
| 9567 | YT521 | NM_133370.1 | 1623 | tggatttgcaggcgtgaattacc | 325134 | 797849 | see also 9566 line | | |
| 9568 | YT521 | NM_133370.1 | 1883 | aaggcgtcgaccagaagattatg | 325141 | 797859 | see also 9566 line | | |
| 9569 | YT521 | NM_133370.1 | 1886 | gcgtcgaccagaagattatgata | 325142 | 797861 | see also 9566 line | | |
| 9570 | YT521 | NM_133370.1 | 1888 | gtcgaccagaagattatgatatt | 325143 | 797862 | see also 9566 line | | |
| 9571 | YT521 | NM_133370.1 | 2015 | aggagttcgccgagatgtgtttt | 325147 | 797866 | see also 9566 line | | |
| 9572 | YT521 | NM_133370.1 | 2020 | ttcgccgagatgtgtttttaaat | 325149 | 797867 | see also 9566 line | | |
| 9573 | IDN3 | NM_133433.1 | 6868 | tggatgtcatcgctatttgctgt | 407200 | 812593 | cell_cycle | - | - |
| 9574 | IDN3 | NM_133433.1 | 6884 | ttgctgtccaaagtacaaagatc | 407202 | 812594 | see also 9573 line | | |

Figure 46

| | | | | | | | |
|---|---|---|---|---|---|---|---|
| 9575 | IDN3 | NM_133433.1 | 7072 | ttgctctaacgagtgctaataag | 407206 | 812597 | see also 9573 line |
| 9576 | IDN3 | NM_133433.1 | 7073 | tgctctaacgagtgctaataagc | 407207 | 812598 | see also 9573 line |
| 9577 | IDN3 | NM_133433.1 | 7092 | aagctgactaataaagttgttca | 407208 | 812600 | see also 9573 line |
| 9578 | GPT2 | NM_133443.1 | 326 | gagctgcagcggggtatcaaaaa | 218203 | 606118 | - |
| 9579 | EMU1 | NM_133455.1 | 1133 | tacgcgcaggctttgaagattta | 407216 | 535110 | transcription_regulator |
| 9580 | EMU1 | NM_133455.1 | 1179 | aacaatgattgggctctatgaac | 407218 | 535113 | see also 9579 line |
| 9581 | TTC14 | NM_133462.1 | 517 | atgtgttgatcgtgtttaggaag | 55565 | 472747 | - |
| 9582 | TTC14 | NM_133462.1 | 656 | ttcgagctggaatcaaggatatt | 55576 | 472752 | see also 9581 line |
| 9583 | TTC14 | NM_133462.1 | 936 | aggcctacaaagcaaaaatttct | 55589 | 472768 | see also 9581 line |
| 9584 | TTC14 | NM_133462.1 | 1290 | tgctgaaagttactataagaaag | 55597 | 472785 | see also 9581 line |
| 9585 | BMPER | NM_133468.2 | 559 | cacagacaacccttgcataatgt | 138176 | 734700 | Alzheimer disease |
| 9586 | BMPER | NM_133468.2 | 797 | ggggtgcgctgtgttgttcattg | 138181 | 734703 | see also 9585 line |
| 9587 | BMPER | NM_133468.2 | 2137 | gtgtgaatgtccagtccataaaa | 138216 | 734739 | see also 9585 line |
| 9588 | CD109 | NM_133493.1 | 1873 | tgggattgtagctgttgacaaaa | 293948 | 663979 | - |
| 9589 | NEK7 | NM_133494.1 | 616 | cacagctgcacattcttagttg | 94705 | 510248 | kinase_related |
| 9590 | DQX1 | NM_133637.1 | 2087 | ctccatacttcctgagtaacttg | 80886 | 500883 | - |
| 9591 | CLYBL | NM_138280.2 | 524 | ctgcaatggtttgctcaatttt | 216602 | 603817 | - |
| 9592 | C14orf147 | NM_138288.1 | 69 | agcagatgtctggttctactac | 407444 | 750507 | - |
| 9593 | RPIB9 | NM_138290.1 | 548 | ctgattgatcggtctgtcttga | 407452 | 813621 | - |
| 9594 | RPIB9 | NM_138290.1 | 753 | gagtgcttgccggaaagtttca | 407463 | 813625 | see also 9593 line |
| 9595 | RPIB9 | NM_138290.1 | 755 | gtggcttgccggaaagtttcaca | 407464 | 813626 | see also 9593 line |
| 9596 | PANK1 | NM_138316.2 | 714 | accctagttgctgttaacatg | 95591 | 511200 | kinase_related |
| 9597 | KCNK10 | NM_138317.1 | 214 | tccaatcgagacgccaagaaaac | 261267 | 644687 | channel, signal_transduction |
| 9598 | RHBDL4 | NM_138328.1 | 460 | accgccaagtgtactatgacagc | 152052 | 553022 | serine-type endopeptidase |
| 9599 | SBBI54 | NM_138334.1 | 220 | gggcaccggcaactatgatgtca | 407527 | 716042 | - |
| 9600 | GNPDA2 | NM_138335.1 | 160 | gaggcttgaattctgataact | 234833 | 619564 | - |
| 9601 | GNPDA2 | NM_138335.1 | 279 | caggggagtacacctttaggatgc | 234839 | 619567 | see also 9600 line |
| 9602 | ABHD3 | NM_138340.3 | 508 | cagcaccagacctatcttat | 235735 | 595055 | - |
| 9603 | ABHD3 | NM_138340.3 | 1216 | tggaggccatattggtttctgg | 235755 | 595073 | see also 9602 line |
| 9604 | LOC91768 | NM_138375.1 | 791 | aggagaagtttcctcacattag | 308255 | 533141 | - |
| 9605 | LOC92689 | NM_138389.1 | 602 | tgccacgtaggtcatggattga | 407888 | 726710 | - |
| 9606 | LOC92689 | NM_138389.1 | 1312 | cggccacacctgacaaacctcaat | 407900 | 726722 | see also 9605 line |
| 9607 | LOC92689 | NM_138389.1 | 1314 | gccacacctgacaaactcaataa | 407901 | 726723 | see also 9605 line |
| 9608 | LOC92689 | NM_138389.1 | 1316 | cacacctgacaaactcaataagg | 407902 | 726724 | see also 9605 line |

Figure 46

| 9609 | C1orf37 | NM_138391.3 | 546 | tccgtcctgaggacattgtgaat | 407935 | 710705 | - | - | - |
|------|---------|-------------|-----|-------------------------|--------|--------|---|---|---|
| 9610 | C1orf37 | NM_138391.3 | 547 | ccgtcctgaggacattgtgaatt | 407936 | 710706 | see also 9609 line | | |
| 9611 | LOC92906 | NM_138394.2 | 553 | tgggacaatatgctatgtgatga | 63886 | 479133 | - | - | - |
| 9612 | LOC92906 | NM_138394.2 | 951 | ctggatgttgcacactaaaaatt | 63906 | 479150 | see also 9611 line | | |
| 9613 | LOC92906 | NM_138394.2 | 990 | ctcgtctaaatgttattaggaat | 63909 | 479154 | see also 9611 line | | |
| 9614 | LOC92906 | NM_138394.2 | 991 | tcgtctaaatgttattaggaatg | 63910 | 479155 | see also 9611 line | | |
| 9615 | LOC92935 | NM_138395.1 | 946 | cacctctcatatcataggtaagg | 88730 | 496788 | - | - | - |
| 9616 | LOC92935 | NM_138395.1 | 960 | taggtaaggacattctcaaattc | 88731 | 496789 | see also 9615 line | | |
| 9617 | CKLFSF7 | NM_138410.2 | 654 | ctggtagccggagcgatctttgg | 328618 | 694526 | - | - | - |
| 9618 | H63 | NM_138423.2 | 611 | atgcgaggatgacaaggttaaac | 408076 | 792300 | - | - | - |
| 9619 | H63 | NM_138423.2 | 1406 | tccgcagcatggctctaaactgg | 408097 | 792331 | see also 9618 line | | |
| 9620 | H63 | NM_138423.2 | 1448 | tggtaacgtaggtgagtatgagg | 408098 | 792334 | see also 9618 line | | |
| 9621 | H63 | NM_138423.2 | 1481 | ggctgagctggcttacaatgagg | 408099 | 792335 | see also 9618 line | | |
| 9622 | FOXP4 | NM_138457.1 | 2053 | agcggagaccgccaaagatgaca | 4256 | 444263 | transcription_regulator | - | - |
| 9623 | LOC116143 | NM_138458.1 | 959 | ttcagcggtcaaagaaagattct | 88576 | 493575 | - | - | - |
| 9624 | LOC130026 | NM_138468.3 | 794 | tggagctagtcgctgcaatatgc | 408331 | 729283 | - | - | - |
| 9625 | LOC130026 | NM_138468.3 | 797 | agctagtcgctgcaatatgctat | 408332 | 729284 | see also 9624 line | | |
| 9626 | SP1 | NM_138473.2 | 1678 | aggcctccagaccattaacctca | 40667 | 704731 | - | - | - |
| 9627 | SP1 | NM_138473.2 | 2420 | ctgcagtccattaatatcagtgg | 40676 | 704746 | see also 9626 line | | |
| 9628 | TMC1 | NM_138691.2 | 1380 | ggggattatgtgcattggataca | 313824 | 678691 | - | - | - |
| 9629 | TMC1 | NM_138691.2 | 2267 | acgtcctcgctctgatcttcaac | 313849 | 678722 | see also 9628 line | | |
| 9630 | LOC91137 | NM_138773.1 | 1042 | ttgttctgacgttatactttacc | 180687 | 573364 | - | - | - |
| 9631 | SYTL5 | NM_138780.1 | 325 | atcgcgcagctaaggattataac | 265824 | 649702 | - | - | - |
| 9632 | SYTL5 | NM_138780.1 | 1183 | tacagtgaaacgggagactatgg | 265850 | 649730 | see also 9631 line | | |
| 9633 | SYTL5 | NM_138780.1 | 1328 | cagatgcttatgtcaagtcatat | 265852 | 649733 | see also 9631 line | | |
| 9634 | SYTL5 | NM_138780.1 | 1378 | aagcgtaagaccaaaatcagaac | 265854 | 649735 | see also 9631 line | | |
| 9635 | SYTL5 | NM_138780.1 | 1379 | agcgtaagaccaaaatcagaaca | 265855 | 649736 | see also 9631 line | | |
| 9636 | LOC129642 | NM_138799.2 | 253 | ttggtttcgaacttatctacatt | 408912 | 720899 | - | - | - |
| 9637 | LOC129642 | NM_138799.2 | 348 | ttggatggtatgccttacacttt | 408917 | 720904 | see also 9636 line | | |
| 9638 | LOC129642 | NM_138799.2 | 358 | tgccttacactttcttgtacaaa | 408918 | 720908 | see also 9636 line | | |

Figure 46

| 9639 | LOC129642 | NM_138799.2 | 1077 | ctcgctgggacttaatttccaat | 408950 | 720939 | see also 9636 line | | |
|------|-----------|-------------|------|------------------------|--------|--------|-------------------|---|---|
| 9640 | LOC130617 | NM_138802.1 | 452 | aagatcttcaccaataagtgtga | 281389 | 666534 | - | - | - |
| 9641 | C7orf31 | NM_138811.3 | 1437 | ccccttgaactggatgattacc | 409162 | 655427 | - | - | - |
| 9642 | VANGL1 | NM_138959.1 | 1030 | cacaacgagttgtattatgaaga | 328052 | 796648 | - | - | - |
| 9643 | MSI2 | NM_138962.2 | 1010 | aggacctgtcgccgatctctacg | 64712 | 472437 | - | - | - |
| 9644 | MSI2 | NM_138962.2 | 1050 | tccggagtggggaattacataag | 64713 | 472439 | see also 9643 line | | |
| 9645 | MSI2 | NM_138962.2 | 1125 | ttgattgcaacggcctttacaaa | 64716 | 472441 | see also 9643 line | | |
| 9646 | MSI2 | NM_138962.2 | 1133 | aacggcctttacaaatggatacc | 64717 | 472442 | see also 9643 line | | |
| 9647 | NETO1 | NM_138966.2 | 791 | accagcgtgtgagtttgagatgg | 409296 | 755268 | - | - | - |
| 9648 | ADAMTS18 | NM_139054.2 | 2651 | ttcaacttgcaagtttttataaag | 184855 | 579111 | - | - | - |
| 9649 | ADAMTS18 | NM_139054.2 | 2789 | tcgaagcctcagtcaaaagtatt | 184861 | 579114 | see also 9648 line | | |
| 9650 | ADAMTS16 | NM_139056.1 | 2112 | ctcggaggatagccgtaatgttt | 184770 | 579054 | - | - | - |
| 9651 | ADAMTS16 | NM_139056.1 | 2113 | tcggaggatagccgtaatgtttg | 184771 | 579055 | see also 9650 line | | |
| 9652 | ADAMTS16 | NM_139056.1 | 2114 | cggaggatagccgtaatgtttgt | 184772 | 579056 | see also 9650 line | | |
| 9653 | ADAMTS16 | NM_139056.1 | 2121 | tagccgtaatgtttgtatagatg | 184773 | 579059 | see also 9650 line | | |
| 9654 | ADAMTS16 | NM_139056.1 | 2122 | agccgtaatgtttgtatagatgg | 184774 | 579060 | see also 9650 line | | |
| 9655 | ARX | NM_139058.1 | 462 | ggcctgggacacgctcaagatca | 1084 | 441830 | transcription_regulator | - | lissencephaly with ambiguous genitalia、 infantile spasm、 mental retardation、 Partington syndrome、 myoclonic epilepsy with mental retardation |
| 9656 | CSNK1D | NM_139062.1 | 1511 | cagaatagcattcctttcgaaca | 77664 | 498931 | kinase_related、 signal _transduction | casein kinase I | - |
| 9657 | DNER | NM_139072.2 | 1220 | cgcgagctgtattgatgcaaatg | 141973 | 547337 | - | - | - |
| 9658 | DNER | NM_139072.2 | 1301 | ttgccagtccaagattgattact | 141974 | 547341 | see also 9657 line | | |
| 9659 | DNER | NM_139072.2 | 2314 | aacccttggtcacactgattaaa | 141993 | 547357 | see also 9657 line | | |
| 9660 | PPIL4 | NM_139126.2 | 508 | gtgattttagatgatccatttga | 64995 | 485450 | - | - | - |
| 9661 | PPIL4 | NM_139126.2 | 527 | ttgatgaccctcctgatttatta | 64996 | 485451 | see also 9660 line | | |
| 9662 | DPP9 | NM_139159.2 | 521 | gccccacgacttccagtttgtgc | 187142 | 580755 | - | - | - |
| 9663 | SGCZ | NM_139167.1 | 90 | atggcgaaagaggtgcttatact | 409386 | 540331 | - | - | - |

Figure 46

| | | | | | | | | | |
|---|---|---|---|---|---|---|---|---|---|
| 9664 | SGCZ | NM_139167.1 | 92 | ggcgaaagaggtgcttatacttc | 409387 | 540332 | see also 9663 line | | |
| 9665 | SGCZ | NM_139167.1 | 97 | aagaggtgcttatacttctttgt | 409388 | 540334 | see also 9663 line | | |
| 9666 | SGCZ | NM_139167.1 | 99 | gaggtgcttatacttctttgtcc | 409389 | 540335 | see also 9663 line | | |
| 9667 | SGCZ | NM_139167.1 | 124 | ctgctgttggttaccatgatagt | 409390 | 540336 | see also 9663 line | | |
| 9668 | SGCZ | NM_139167.1 | 153 | agccatgacaatatggatattga | 409392 | 540339 | see also 9663 line | | |
| 9669 | SGCZ | NM_139167.1 | 228 | gggaatccgacttgaaggtatat | 409394 | 540342 | see also 9663 line | | |
| 9670 | SGCZ | NM_139167.1 | 232 | atccgacttgaaggtatatctga | 409395 | 540343 | see also 9663 line | | |
| 9671 | SGCZ | NM_139167.1 | 234 | ccgacttgaaggtatatctgagt | 409396 | 540344 | see also 9663 line | | |
| 9672 | SFRS12 | NM_139168.1 | 653 | gccaactcggtttgcttttgtgg | 65930 | 481250 | - | - | - |
| 9673 | SFRS12 | NM_139168.1 | 758 | ctccaacaatgcaatagtaaaac | 65934 | 481252 | see also 9672 line | | |
| 9674 | SFRS12 | NM_139168.1 | 759 | tccaacaatgcaatagtaaaacc | 65935 | 481253 | see also 9672 line | | |
| 9675 | SFRS12 | NM_139168.1 | 990 | cacaggagtagatcccataatag | 65938 | 481256 | see also 9672 line | | |
| 9676 | FGD4 | NM_139241.1 | 1107 | tagatcaggtattttattgcaaa | 156860 | 557797 | - | - | - |
| 9677 | FGD4 | NM_139241.1 | 1586 | agccattctaatagtgcaataag | 156872 | 557812 | see also 9676 line | | |
| 9678 | FGD4 | NM_139241.1 | 1587 | gccattctaatagtgcaataagg | 156873 | 557813 | see also 9676 line | | |
| 9679 | FGD4 | NM_139241.1 | 2324 | gagattgaatcagcagaagtatc | 156887 | 557829 | see also 9676 line | | |
| 9680 | Tenr | NM_139243.1 | 1178 | aaggatcagcccagattaagtca | 48918 | 472249 | - | - | - |
| 9681 | Tenr | NM_139243.1 | 1529 | tagtcaacagacctcatattagt | 48931 | 472265 | see also 9680 line | | |
| 9682 | PPM1L | NM_139245.1 | 351 | agcaggcacaacgtgtttgattg | 237179 | 588423 | - | - | - |
| 9683 | LOC158427 | NM_139246.3 | 1138 | ttccctagctacgttgacaaaaa | 409594 | 782693 | - | - | - |
| 9684 | LOC158427 | NM_139246.3 | 1139 | tccctagctacgttgacaaaaat | 409595 | 782694 | see also 9683 line | | |
| 9685 | LOC158427 | NM_139246.3 | 1140 | ccctagctacgttgacaaaaatc | 409596 | 782695 | see also 9683 line | | |
| 9686 | TA-WDRP | NM_139281.1 | 977 | aagactggggacccattacttca | 237369 | 754427 | - | - | - |
| 9687 | TA-PP2C | NM_139283.1 | 1040 | cacctttttgcacagtttgcatgt | 237344 | 621288 | - | - | - |
| 9688 | KCNH5 | NM_139318.3 | 109 | gtcaggcgctccagtgaatcaag | 121485 | 596795 | channel, signal_transduction | - | - |
| 9689 | KCNH5 | NM_139318.3 | 1056 | taggaaactggaccattacctag | 121532 | 596835 | see also 9688 line | | |
| 9690 | KCNH5 | NM_139318.3 | 1335 | aagccttacaaccataggatttg | 121544 | 596838 | see also 9688 line | | |
| 9691 | KCNH5 | NM_139318.3 | 1742 | tagagttccaaaccattcactgt | 121548 | 596851 | see also 9688 line | | |
| 9692 | KCNH5 | NM_139318.3 | 2661 | aagtgaccttcgtttggataagg | 121570 | 596867 | see also 9688 line | | |
| 9693 | CNOT6L | NM_144571.1 | 98 | ctccagatcctcgcagaatttat | 409771 | 754869 | - | - | - |
| 9694 | CNOT6L | NM_144571.1 | 536 | cagcatcattcacggttatgtgt | 409787 | 754891 | see also 9693 line | | |
| 9695 | CNOT6L | NM_144571.1 | 544 | ttcacggttatgtgttacaatgt | 409788 | 754892 | see also 9693 line | | |
| 9696 | CNOT6L | NM_144571.1 | 673 | gacgcagatatcattagtcttca | 409791 | 754896 | see also 9693 line | | |

EP 1 752 536 A1

Figure 46

| | | | | | | | |
|---|---|---|---|---|---|---|---|
| 9697 | CNOT6L | NM_144571.1 | 1066 | tgggaccccagagtattctgatgt | 409800 | 754907 | see also 9693 line |
| 9698 | CNOT6L | NM_144571.1 | 1262 | tagctgacaaccataaagacttc | 409808 | 754914 | see also 9693 line |
| 9699 | CNOT6L | NM_144571.1 | 1301 | atgagtgtcttatgaacttcagc | 409810 | 754917 | see also 9693 line |
| 9700 | CNOT6L | NM_144571.1 | 1373 | aacttaagagcgcctatgaaaat | 409813 | 754918 | see also 9693 line |
| 9701 | CNOT6L | NM_144571.1 | 1380 | gagcgcctatgaaaataacttga | 409814 | 754919 | see also 9693 line |
| 9702 | WDR20 | NM_144574.2 | 711 | gggccctcaacgagtttgctttc | 409873 | 730322 | - |
| 9703 | WDR20 | NM_144574.2 | 1183 | ctgttatggacctttacagaag | 409881 | 730338 | see also 9702 line |
| 9704 | WDR20 | NM_144574.2 | 1598 | atggaagatgttccctgttaga | 409897 | 730351 | see also 9702 line |
| 9705 | SFXN5 | NM_144579.1 | 755 | ctgtagccagtgccaatatctgc | 266773 | 653889 | - |
| 9706 | ATP6V1C2 | NM_144583.1 | 47 | ccctggcgataaggaaaatttgc | 73330 | 494912 | - |
| 9707 | RHEBL1 | NM_144593.1 | 267 | atctttggcacatcaatttgtgg | 110632 | 518088 | signal_transduction |
| 9708 | RHEBL1 | NM_144593.1 | 315 | tcctacagtggagaatacttaca | 110633 | 518090 | see also 9707 line |
| 9709 | TTC8 | NM_144596.1 | 111 | tccagctctgcgccgatctatgc | 410125 | 739623 | - |
| 9710 | TTC8 | NM_144596.1 | 531 | ctgatggaccacattataaattta | 410139 | 739636 | see also 9709 line |
| 9711 | MGC24976 | NM_144598.2 | 493 | tacgtcatcttcgattagctaat | 410178 | 785621 | - |
| 9712 | MGC24976 | NM_144598.2 | 494 | acgtcatcttcgattagctaata | 410179 | 785622 | see also 9711 line |
| 9713 | MGC24976 | NM_144598.2 | 496 | gtcatcttcgattagctaataac | 410180 | 785623 | see also 9711 line |
| 9714 | MGC24976 | NM_144598.2 | 536 | tccagaggttggcgatttgaagg | 410181 | 785625 | see also 9711 line |
| 9715 | BHD | NM_144606.3 | 1032 | accggatctacctcatcaactcc | 410251 | 759397 | - |
| 9716 | MGC29891 | NM_144618.1 | 1212 | ttggccagccattattgtaact | 410371 | 774951 | - |
| 9717 | KIS | NM_144624.1 | 957 | gtggtgaatgccgcaattccagc | 52349 | 473965 | kinase_related |
| 9718 | KIS | NM_144624.1 | 1049 | ggcattgtgcagccccattctta | 52354 | 473968 | see also 9717 line |
| 9719 | KIS | NM_144624.1 | 1076 | tcctttgccctcatattgaag | 52356 | 473969 | see also 9717 line |
| 9720 | C20orf140 | NM_144628.1 | 1056 | acggcagcctctacttttcaaaga | 410542 | 714809 | - |
| 9721 | C20orf140 | NM_144628.1 | 1184 | aaggaccaaccgcttttgtgaaat | 410546 | 714812 | see also 9720 line |
| 9722 | ZNF513 | NM_144631.2 | 365 | aagtgcggaagtgaagttgagg | 410586 | 787881 | - |
| 9723 | ZNF513 | NM_144631.2 | 1501 | gtgccacctgcgcctataccacg | 410591 | 787883 | see also 9722 line |
| 9724 | MGC26610 | NM_144647.2 | 405 | tggaacaatagacttcaatgaat | 148236 | 542780 | - |
| 9725 | FLJ33069 | NM_144649.1 | 467 | atcttggctgcatggaagtatct | 410822 | 775029 | - |
| 9726 | BTBD14A | NM_144653.2 | 428 | agcagctcgtgtcatgtacacg | 129339 | 530568 | - |
| 9727 | SAMD8 | NM_144660.1 | 865 | cagtgtactgaaagatatatgg | 411101 | 723459 | - |
| 9728 | OTOA | NM_144672.2 | 791 | tacatggttcacctatcgtttga | 411248 | 693909 | - |

Figure 46

| 9729 | OTOA | NM_144672.2 | 794 | atggttcacctatcgtttgaaga | 411249 | 693910 | see also 9728 line |
|---|---|---|---|---|---|---|---|
| 9730 | TOM1L2 | NM_144678.2 | 1256 | atctgtcatggacgacattgagg | 102743 | 650207 | - |
| 9731 | C20orf40 | NM_144703.1 | 898 | gtccaactaacgttaaggaaaac | 411498 | 798554 | - |
| 9732 | C20orf40 | NM_144703.1 | 899 | tccaactaaacgttaaggaaaaca | 411499 | 798555 | see also 9731 line |
| 9733 | FLJ25200 | NM_144715.2 | 2321 | gtggtgttccaaccattcgatct | 145817 | 543059 | - |
| 9734 | LOC152185 | NM_144718.2 | 636 | aggcgcacagggtttccaaatgt | 411695 | 753489 | - |
| 9735 | LOC152185 | NM_144718.2 | 1390 | gtcgcctggttcggtaccttaaa | 411711 | 753506 | see also 9734 line |
| 9736 | LOC152185 | NM_144718.2 | 1391 | tcgcctggttcggtaccttaaag | 411712 | 753507 | see also 9734 line |
| 9737 | LOC152185 | NM_144718.2 | 1392 | cgcctggttcggtaccttaaaga | 411713 | 753508 | see also 9734 line |
| 9738 | LOC152185 | NM_144718.2 | 1396 | tggttcggtaccttaaagagagt | 411714 | 753509 | see also 9734 line |
| 9739 | FLJ31564 | NM_144720.1 | 2221 | aacgagctgttagaattcagagt | 411789 | 801420 | - |
| 9740 | FLJ31564 | NM_144720.1 | 2240 | gagtgctagaactcgaagtaaga | 411790 | 801421 | see also 9739 line |
| 9741 | FLJ31564 | NM_144720.1 | 2242 | gtgctagaactcgaagtaagaga | 411791 | 801422 | see also 9739 line |
| 9742 | FLJ31564 | NM_144720.1 | 2259 | aagagactctatcgttgtaaac | 411792 | 801423 | see also 9739 line |
| 9743 | FLJ31564 | NM_144720.1 | 2281 | ctctcaaacggagcagacattct | 411793 | 801424 | see also 9739 line |
| 9744 | FLJ31121 | NM_144723.1 | 22 | cgggcagcggacaaaaaaacttg | 51743 | 717431 | - |
| 9745 | FLJ31121 | NM_144723.1 | 23 | gggcagcgggacaacaaaaacttg | 51744 | 717432 | see also 9744 line |
| 9746 | FLJ31121 | NM_144723.1 | 51 | cgccgaaagtgggacaaagatga | 51746 | 717433 | see also 9744 line |
| 9747 | FLJ31455 | NM_144964.1 | 439 | aacactcaggaccaagactatgt | 145882 | 542922 | - |
| 9748 | ZDHHC15 | NM_144969.1 | 79 | ctgggtgccagtgctcgttattg | 412012 | 788480 | - |
| 9749 | ZDHHC15 | NM_144969.1 | 628 | accagtgttcgctctaagttcc | 412022 | 788493 | see also 9748 line |
| 9750 | ZDHHC15 | NM_144969.1 | 899 | ttccctagggtcatgaatga | 412029 | 788498 | see also 9748 line |
| 9751 | MGC39350 | NM_144970.1 | 401 | atgtgatgcagttgctcttttaa | 412034 | 785739 | - |
| 9752 | MGC39350 | NM_144970.1 | 415 | ctcttttaagtctcatcaactcc | 412035 | 785741 | see also 9751 line |
| 9753 | MGC39350 | NM_144970.1 | 533 | gtggcttcgagatttcagatga | 412041 | 785743 | see also 9751 line |
| 9754 | MGC24039 | NM_144973.2 | 509 | atggttctcgcacctatggtttt | 412059 | 793936 | - |
| 9755 | MGC24039 | NM_144973.2 | 510 | tggttctcgcacctatggtttg | 412060 | 793937 | see also 9754 line |
| 9756 | MGC24039 | NM_144973.2 | 513 | ttctcgcacctatggttgttc | 412061 | 793938 | see also 9754 line |

Figure 46

| | | | | | | | | | |
|---|---|---|---|---|---|---|---|---|---|
| 9757 | MGC24039 | NM_144973.2 | 551 | aagttacaagtaagcaaatctgc | 412062 | 793939 | see also 9754 line | | |
| 9758 | MGC24039 | NM_144973.2 | 1318 | gagttgcctgaagaatttccaca | 412090 | 793957 | see also 9754 line | | |
| 9759 | MGC24039 | NM_144973.2 | 1895 | tgcggacatctatatatcagaaa | 412110 | 793975 | see also 9754 line | | |
| 9760 | MGC24039 | NM_144973.2 | 2191 | atgcaagaggcacgaagtttagg | 412118 | 793980 | see also 9754 line | | |
| 9761 | MGC27044 | NM_144977.1 | 442 | aagttggacagcactttaccttt | 412173 | 809192 | - | - | - |
| 9762 | U2AF1L3 | NM_144987.1 | 25 | ctgaatatttagcttcgatattc | 67079 | 487013 | - | - | - |
| 9763 | U2AF1L3 | NM_144987.1 | 60 | gacaaggttaactgctctttta | 67080 | 487015 | see also 9762 line | | |
| 9764 | C10orf9 | NM_145012.3 | 223 | atcctcgggccagcacaatattc | 308111 | 674694 | - | - | - |
| 9765 | C10orf9 | NM_145012.3 | 224 | tcctcgggccagcacaatattcc | 308112 | 674695 | see also 9764 line | | |
| 9766 | C10orf9 | NM_145012.3 | 277 | aacgcaagagtctcttcattaac | 308113 | 674696 | see also 9764 line | | |
| 9767 | MGC33887 | NM_145036.1 | 1622 | aagcagctgattagagataatga | 413059 | 739840 | - | - | - |
| 9768 | MGC33887 | NM_145036.1 | 1754 | tacatacgacaagaatatgaaac | 413063 | 739787 | see also 9767 line | | |
| 9769 | MGC12909 | NM_145055.1 | 289 | tacaactggccgagtttatgaga | 413313 | 802706 | - | - | - |
| 9770 | C13orf3 | NM_145061.2 | 657 | aactcctaaattagaacactttg | 413378 | 813449 | - | - | - |
| 9771 | C6orf113 | NM_145062.1 | 300 | agctcacctaattgttcacatgg | 413407 | 733713 | - | - | - |
| 9772 | C6orf113 | NM_145062.1 | 1680 | tacatctaaacctcctatctatc | 413454 | 733791 | see also 9771 line | | |
| 9773 | MGC2793 | NM_145064.1 | 952 | gtggctctctatcggttcaaagc | 316743 | 680165 | - | - | - |
| 9774 | TRPV3 | NM_145068.2 | 599 | gtgacagagaccccatccaatcc | 252295 | 646606 | receptor_acitivity、cha nnel | - | - |
| 9775 | NSE1 | NM_145080.2 | 267 | tccaaaatggctacggattttgc | 265565 | 647737 | - | - | - |
| 9776 | NSE1 | NM_145175.1 | 331 | gggtcggggttgcctacttcttc | 413754 | 737803 | - | - | - |
| 9777 | NSE1 | NM_145175.1 | 334 | tcggggttgcctacttcttctcg | 413755 | 737804 | see also 9776 line | | |
| 9778 | SLC2A12 | NM_145176.1 | 287 | ctcctggtgggttatgaacttgg | 258989 | 643286 | - | - | - |
| 9779 | CABP4 | NM_145200.1 | 711 | cgcatcgccttccgagagtttga | 138463 | 544676 | signal_transduction | calcium ion binding | - |
| 9780 | SPATA5 | NM_145207.1 | 1395 | cactctacgacacccatcaatta | 101084 | 515144 | - | - | - |
| 9781 | SPATA5 | NM_145207.1 | 1397 | ctctacgacacccatcaattatt | 101085 | 515145 | see also 9780 line | | |
| 9782 | SPATA5 | NM_145207.1 | 1920 | agcaattgatgtcccaaatgtat | 101099 | 515167 | see also 9780 line | | |
| 9783 | LOC115704 | NM_145245.2 | 229 | aagtccatgcgctccatgaatgg | 88545 | 655850 | gpcr、signal_transduct ion | | - |

EP 1 752 536 A1

Figure 46

| | | | | | | | | |
|---|---|---|---|---|---|---|---|---|
| 9784 | LOC124491 | NM_145254.1 | 352 | tgggcatcgtgggaccaattact | 413913 | 718823 | | - |
| 9785 | LOC124491 | NM_145254.1 | 354 | ggcatcgtgggaccaattactgc | 413914 | 718824 | | see also 9784 line |
| 9786 | LOC151194 | NM_145280.3 | 535 | gagctgacttggggacaaaattt | 414121 | 720414 | | - |
| 9787 | LOC151194 | NM_145280.3 | 536 | agctgacttggggacaaaatttg | 414122 | 720415 | | see also 9786 line |
| 9788 | LOC159090 | NM_145284.3 | 1113 | cagtagcatgctccaattcaatgc | 414147 | 741661 | | - |
| 9789 | LACE1 | NM_145315.2 | 1210 | accgttgccgactgcacattga | 414399 | 758076 | | - |
| 9790 | MGC21654 | NM_145647.1 | 1647 | ttgctactctcataatcaattgg | 414548 | 816487 | | - |
| 9791 | SLC15A4 | NM_145648.1 | 1044 | taccttactggacagtgtatttc | 163883 | 561198 | | - |
| 9792 | MUC15 | NM_145650.2 | 1158 | aaccagttctgcgattagacaat | 414618 | 782012 | | - |
| 9793 | MUC15 | NM_145650.2 | 1159 | accagttctgcgattagacaatg | 414619 | 782013 | | see also 9792 line |
| 9794 | MAP4K4 | NM_145686.1 | 1937 | ctggcatctcaagaacaatgt | 414682 | 507498 | kinase_related | - |
| 9795 | ACBD5 | NM_145698.1 | 371 | ttgggatccattggaagatata | 172449 | 569759 | | - |
| 9796 | ACBD5 | NM_145698.1 | 372 | tgggatccattggaagatataa | 172450 | 569760 | | see also 9795 line |
| 9797 | CDIPT | NM_145752.1 | 467 | aaggttatgcccggattgtcttc | 161802 | 606496 | phosphatidylinositol transporter | - |
| 9798 | CDIPT | NM_145752.1 | 468 | aggttatgcccggattgtcttcg | 161803 | 606497 | | see also 9797 line |
| 9799 | LL5beta | NM_145753.1 | 273 | gagactattctggctcctattta | 414776 | 769312 | | - |
| 9800 | LL5beta | NM_145753.1 | 2471 | gagggctatatcagtgtaaatga | 414821 | 769355 | | see also 9799 line |
| 9801 | ISL2 | NM_145805.1 | 1112 | tcccggacaccccaacagtatg | 5597 | 445132 | transcription_regulator | transcription factor |
| 9802 | ISL2 | NM_145805.1 | 1113 | cccggacaccccaacagtatgg | 5598 | 445133 | | see also 9801 line |
| 9803 | A2BP1 | NM_145891.1 | 1198 | cggcggagtagtgtatcaagagc | 49538 | 473002 | | - |
| 9804 | SLC5A8 | NM_145913.2 | 870 | gggggtggtctgcacattctact | 274444 | 657065 | | - |
| 9805 | SLC5A8 | NM_145913.2 | 872 | gggtggtctgcacattctactgc | 274445 | 657066 | | see also 9804 line |
| 9806 | LOC223082 | NM_147128.3 | 1720 | aggctgcatagatgaattggtttg | 415017 | 816042 | | - |
| 9807 | MOB | NM_147156.2 | 1584 | ctgtacctgtccggtgtattac | 415045 | 753837 | kinase_related | - |
| 9808 | MOB | NM_147156.2 | 2035 | ggccatttcagtactttgaaaag | 415059 | 753854 | | see also 9807 line |
| 9809 | MOB | NM_147156.2 | 2066 | aggaattgtacctgcatcttacc | 415060 | 753855 | | see also 9807 line |
| 9810 | MOB | NM_147156.2 | 2071 | ttgtacctgcatcttaccattgg | 415061 | 753856 | | see also 9807 line |
| 9811 | HS6ST2 | NM_147174.2 | 909 | ctgtcatgcctgaaaagcaaaga | 218807 | 606648 | | - |
| 9812 | FBXO22 | NM_147188.1 | 928 | gtggttggactgtcatttagtgg | 415072 | 732203 | | - |

Figure 46

| | | | | | | | |
|---|---|---|---|---|---|---|---|
| 9813 | FBXO22 | NM_147188.1 | 1127 | aggctgatgcatttagaagagttt | 415078 | 732208 | see also 9812 line |
| 9814 | MMP21 | NM_147191.1 | 891 | gggatccataatgcaaccaaatt | 158294 | 558855 | - |
| 9815 | OTX3 | NM_147192.2 | 60 | ctcactcagcgcatgtacaacc | 8801 | 443010 | transcription_regulator |
| 9816 | C6orf4 | NM_147200.1 | 1495 | ttgacatattgaggatagaatc | 277970 | 749737 | kinase_related |
| 9817 | C6orf4 | NM_147200.1 | 1582 | tagcaatcagcccaaatacaaa | 277979 | 749746 | see also 9816 line |
| 9818 | C6orf4 | NM_147200.1 | 1648 | atggcttacatactaagtacatt | 277982 | 749750 | see also 9816 line |
| 9819 | C6orf4 | NM_147200.1 | 1650 | ggcttacatactaagtacattca | 277983 | 749752 | see also 9816 line |
| 9820 | SMC1L2 | NM_148674.1 | 1712 | tcgtctagattaccttgatatc | 100549 | 467501 | cell_cycle |
| 9821 | SMC1L2 | NM_148674.1 | 2129 | cacgactcaatattcacaaat | 100566 | 467515 | see also 9820 line |
| 9822 | SMC1L2 | NM_148674.1 | 3228 | ctcaattgatcaaatctacaaga | 100598 | 467553 | see also 9820 line |
| 9823 | FLJ38628 | NM_152267.2 | 331 | ttctgttggccgtgtttacatca | 415225 | 755725 | - |
| 9824 | FLJ38628 | NM_152267.2 | 520 | gggggatttcaagatttgatt | 415230 | 755727 | see also 9823 line |
| 9825 | MGC29816 | NM_152272.1 | 863 | ggcatgccgagcaggaaagaagc | 282114 | 667015 | - |
| 9826 | DC-UbP | NM_152277.1 | 123 | tacgcagcaagagggatgaattt | 415294 | 784580 | - |
| 9827 | SYT11 | NM_152280.2 | 1220 | atcaccggtctctcaggtaatcc | 274993 | 707453 | transporter |
| 9828 | SYT11 | NM_152280.2 | 1223 | accggtctctcaggtaatcctta | 274994 | 707454 | see also 9827 line |
| 9829 | FLJ11752 | NM_152281.1 | 470 | acctccaaagccagattgcaaat | 415313 | 804644 | - |
| 9830 | ZFP276 | NM_152287.2 | 1086 | acctccggatgatcgggtaaaaga | 415366 | 649974 | transcription_regulator |
| 9831 | ZFP276 | NM_152287.2 | 1088 | ctcggatgatcgggtaaaagacg | 415367 | 649975 | see also 9830 line |
| 9832 | TARS | NM_152295.3 | 1276 | cagggaccaagaactatatttct | 102101 | 515917 | threonine-tRNA ligase |
| 9833 | TARS | NM_152295.3 | 2287 | tgcacagttagcacagtataact | 102123 | 515946 | see also 9832 line |
| 9834 | ATP1A3 | NM_152296.2 | 232 | aagaggtcgccggaaatacaac | 72645 | 494407 | sodium/potassium-exchanging ATPase |
| 9835 | NIRF | NM_152306.2 | 488 | aagcagttgaaaatggatatac | 33800 | 453151 | transcription_regulator |
| 9836 | NIRF | NM_152306.2 | 883 | atccaaagagaacacaaataaat | 33817 | 453171 | see also 9835 line |
| 9837 | NIRF | NM_152306.2 | 1072 | tggtgatgtggtaatggttaatt | 33829 | 453181 | see also 9835 line |
| 9838 | NIRF | NM_152306.2 | 2064 | atgctccattgatgataaaatt | 33861 | 453211 | see also 9835 line |
| 9839 | GYLTL1B | NM_152312.2 | 639 | cggcctctatgggctaatgaagc | 212105 | 600680 | - |
| 9840 | SLC36A4 | NM_152313.2 | 425 | ttggtacgttgcagtcactttct | 259653 | 641874 | - |
| 9841 | FLJ30002 | NM_152341.2 | 136 | gccgcacctgcagttcaattaagt | 326532 | 691363 | receptor_acitivity |
| 9842 | MGC42174 | NM_152383.2 | 261 | gtgcttcgccaggtgacaaaaag | 52536 | 472820 | - |

Figure 46

| 9843 | DKFZp762I194 | NM_152384.1 | 145 | ttgtttagattccattgaagaca | 416028 | 733677 | - | - | - |
|---|---|---|---|---|---|---|---|---|---|
| 9844 | DKFZp762I194 | NM_152384.1 | 651 | taccatatctgcaaattcgttca | 416051 | 733702 | see also 9843 line | | |
| 9845 | DKFZp762I194 | NM_152384.1 | 682 | tagagattcaaaatttggtttag | 416054 | 733705 | see also 9843 line | | |
| 9846 | DKFZp762I194 | NM_152384.1 | 978 | aagatcgtgaacctgtattttca | 416060 | 733710 | see also 9843 line | | |
| 9847 | FLJ31322 | NM_152387.1 | 493 | tagaacttaaaaaggctttaaca | 131348 | 534116 | - | - | - |
| 9848 | FLJ31322 | NM_152387.1 | 562 | gggttctccactatcttaacaca | 131351 | 534123 | see also 9847 line | | |
| 9849 | FLJ31322 | NM_152387.1 | 565 | ttctccactatcttaacacatct | 131352 | 534124 | see also 9847 line | | |
| 9850 | FLJ31322 | NM_152387.1 | 593 | aagctgtgagagtagaattattg | 131354 | 534125 | see also 9847 line | | |
| 9851 | FLJ39370 | NM_152400.1 | 312 | atgggcttcacaaggatgtattt | 416278 | 729614 | - | - | - |
| 9852 | FLJ39370 | NM_152400.1 | 313 | tgggcttcacaaggatgtatttt | 416279 | 729615 | see also 9851 line | | |
| 9853 | FLJ39370 | NM_152400.1 | 314 | gggcttcacaaggatgtattttg | 416280 | 729616 | see also 9851 line | | |
| 9854 | FLJ39155 | NM_152403.2 | 1707 | aagtgagaccaaaatcaaactag | 146064 | 544333 | - | - | - |
| 9855 | JMY | NM_152405.1 | 154 | aagatttccatggagaatgatta | 416318 | 525524 | - | - | - |
| 9856 | JMY | NM_152405.1 | 989 | tggatagacttcgaacatttaaa | 416343 | 525548 | see also 9855 line | | |
| 9857 | GRPEL2 | NM_152407.2 | 585 | gcctggcaccgtggcattagtaa | 416372 | 668160 | - | - | - |
| 9858 | GRPEL2 | NM_152407.2 | 623 | aacttcatggccgcaccattagg | 416378 | 668161 | see also 9857 line | | |
| 9859 | FLJ35779 | NM_152408.1 | 939 | atgttatctggagctttggaaaa | 416402 | 722117 | - | - | - |
| 9860 | FLJ32370 | NM_152417.1 | 617 | ggcagaacttgccgagtagtagc | 216282 | 603285 | - | - | - |
| 9861 | FLJ32370 | NM_152417.1 | 827 | ggctttgctcaggttgcaattga | 216289 | 603293 | see also 9860 line | | |
| 9862 | MGC24137 | NM_152430.1 | 263 | ttgctgtgctaggtaacttgaca | 240800 | 626610 | receptor_acitivity、signal_transduction、gpcr | - | - |
| 9863 | KBTBD3 | NM_152433.2 | 883 | gagattacatcagttatctgagg | 132089 | 536120 | - | - | - |
| 9864 | KBTBD3 | NM_152433.2 | 1406 | cgggacattaaaagtctcttaga | 132105 | 536148 | see also 9863 line | | |
| 9865 | KBTBD3 | NM_152433.2 | 1419 | gtctcttagatgttgaatcttac | 132106 | 536149 | see also 9863 line | | |
| 9866 | FLJ32549 | NM_152440.1 | 142 | aggaacgagaggccaacaagagc | 416811 | 782335 | - | - | - |
| 9867 | FLJ32549 | NM_152440.1 | 240 | acctacctggggcagaaactagg | 416813 | 782336 | see also 9866 line | | |
| 9868 | C14orf44 | NM_152445.1 | 1741 | aaggccctatctctttgaacaag | 321340 | 685767 | - | - | - |
| 9869 | C14orf146 | NM_152447.2 | 861 | ctgactctatccaggaatacaat | 23248 | 454680 | - | - | - |
| 9870 | C14orf146 | NM_152447.2 | 869 | atccaggaatacaataagtttta | 23249 | 454681 | see also 9869 line | | |
| 9871 | C14orf146 | NM_152447.2 | 939 | ttgaatagcaacagattgactaa | 23253 | 454688 | see also 9869 line | | |
| 9872 | C14orf146 | NM_152447.2 | 1083 | ctgtcctataataatctagaaac | 23260 | 454695 | see also 9869 line | | |
| 9873 | C14orf146 | NM_152447.2 | 1971 | cagtacaatggtacttatgatga | 23288 | 454738 | see also 9869 line | | |
| 9874 | C14orf146 | NM_152447.2 | 2130 | gtgggttgcatccagtttactac | 23297 | 454744 | see also 9869 line | | |

Figure 46

| 9875 | C14orf146 | NM_152447.2 | 2147 | tactacggaacaggattatgtgc | 23298 | 454746 | see also 9869 line | | |
|---|---|---|---|---|---|---|---|---|---|
| 9876 | KLHL10 | NM_152467.1 | 835 | aagatgaatgactatgtcaaaga | 29975 | 461100 | - | - | - |
| 9877 | KLHL10 | NM_152467.1 | 1848 | tgcagctagacgggacaacttcc | 30007 | 461128 | see also 9876 line | | |
| 9878 | FLJ31842 | NM_152487.1 | 489 | aggctacctcatttctgatttgt | 417068 | 805132 | - | - | - |
| 9879 | FLJ25286 | NM_152546.1 | 87 | aactctgaacctcaataacgagg | 417878 | 720986 | - | - | - |
| 9880 | FLJ23654 | NM_152550.1 | 223 | atggatgatttgacgttacttga | 417976 | 781816 | - | - | - |
| 9881 | FLJ31818 | NM_152556.1 | 682 | aactgttggcatagatattgtac | 418084 | 787901 | - | - | - |
| 9882 | ATP6V0D2 | NM_152565.1 | 1074 | ttcacagaggcatcgaactaaaa | 304856 | 672844 | - | - | - |
| 9883 | ATP6V0D2 | NM_152565.1 | 1076 | cacagaggcatcgaactaaaatc | 304857 | 672845 | see also 9882 line | | |
| 9884 | FLJ31810 | NM_152570.1 | 969 | ctgcgttccctccgcctaaaagg | 418126 | 789576 | - | - | - |
| 9885 | FLJ31810 | NM_152570.1 | 1296 | atgcctgtgtatgcctttaaaag | 418134 | 789582 | see also 9884 line | | |
| 9886 | FLJ31810 | NM_152570.1 | 2375 | cagcattgaccttgagtatgtgc | 418158 | 789607 | see also 9884 line | | |
| 9887 | DKFZp762 A217 | NM_152588.1 | 461 | tggggctcgccttgtatctcaac | 418345 | 795531 | - | - | - |
| 9888 | DKFZp762 A217 | NM_152588.1 | 513 | cagccgtgctatcaagactaatc | 418346 | 795533 | see also 9887 line | | |
| 9889 | DKFZp762 A217 | NM_152588.1 | 516 | ccgtgctatcaagactaatcagg | 418348 | 795534 | see also 9887 line | | |
| 9890 | SPRED1 | NM_152594.1 | 119 | cggcgacttctgacaacgataat | 418525 | 744174 | signal_transduction | - | - |
| 9891 | SPRED1 | NM_152594.1 | 120 | ggcgacttctgacaacgataata | 418526 | 744175 | see also 9890 line | | |
| 9892 | SPRED1 | NM_152594.1 | 121 | gcgacttctgacaacgataatag | 418527 | 744176 | see also 9890 line | | |
| 9893 | SPRED1 | NM_152594.1 | 453 | ccgaagagctatagaggatattt | 418534 | 744180 | see also 9890 line | | |
| 9894 | SPRED1 | NM_152594.1 | 994 | ggggatgagactaagttaagttc | 418552 | 744204 | see also 9890 line | | |
| 9895 | SPRED1 | NM_152594.1 | 1097 | aacgttctcgctgcgtatactgc | 418556 | 744212 | see also 9890 line | | |
| 9896 | SPRED1 | NM_152594.1 | 1357 | tgctgctacgtccctttgagaat | 418565 | 744221 | see also 9890 line | | |
| 9897 | SPRED1 | NM_152594.1 | 1359 | ctgctacgtccctttgagaatgt | 418566 | 744222 | see also 9890 line | | |
| 9898 | FLJ35989 | NM_152597.3 | 631 | aagcaaggtctagaaatgagaat | 418639 | 730808 | - | - | - |
| 9899 | FLJ32001 | NM_152609.1 | 972 | agggatttaatggtgaagatttt | 418782 | 760250 | - | - | - |
| 9900 | FLJ32001 | NM_152609.1 | 973 | gggatttaatggtgaagattttg | 418783 | 760251 | see also 9899 line | | |
| 9901 | TRIM42 | NM_152616.3 | 1226 | agcagtccgatatgaaattgata | 160562 | 555320 | - | - | - |
| 9902 | TRIM42 | NM_152616.3 | 1228 | cagtccgatatgaaattg
ataat | 160563 | 555321 | see also 9901 line | | |
| 9903 | FLJ35794 | NM_152617.2 | 724 | tcggtaccatacccgaagaaatt | 418901 | 729089 | - | - | - |
| 9904 | FLJ35794 | NM_152617.2 | 725 | cggtaccatacccgaagaaattc | 418902 | 729090 | see also 9903 line | | |
| 9905 | MGC45428 | NM_152619.1 | 677 | agccgtgcggatccttctgaata | 91328 | 489634 | kinase_related | - | - |

415

EP 1 752 536 A1

Figure 46

| | | | | | | | | | |
|---|---|---|---|---|---|---|---|---|---|
| 9906 | MGC45428 | NM_152619.1 | 678 | gccgtgcggatccttctgaataa | 91329 | 489635 | see also 9905 line | | |
| 9907 | MGC45428 | NM_152619.1 | 681 | gtgcggatccttctgaataaaaa | 91330 | 489637 | see also 9905 line | | |
| 9908 | MGC45428 | NM_152619.1 | 738 | atcaccgaagccattaaactaga | 91333 | 489639 | see also 9905 line | | |
| 9909 | MGC45428 | NM_152619.1 | 740 | caccgaagccattaaactagact | 91334 | 489640 | see also 9905 line | | |
| 9910 | MGC34648 | NM_152660.1 | 729 | accaggcactgaccactttgtca | 265484 | 648625 | - | - | - |
| 9911 | FLJ34969 | NM_152678.1 | 576 | ttggttttgatcagcaaactacc | 419640 | 758800 | - | - | - |
| 9912 | FLJ34969 | NM_152678.1 | 1146 | cagcactggccacacattattcg | 419664 | 758825 | see also 9911 line | | |
| 9913 | FLJ34969 | NM_152678.1 | 1149 | cactggccacacattattcgaat | 419665 | 758826 | see also 9911 line | | |
| 9914 | FLJ34969 | NM_152678.1 | 1151 | ctggccacacattattcgaatag | 419666 | 758827 | see also 9911 line | | |
| 9915 | FLJ34969 | NM_152678.1 | 1152 | tggccacacattattcgaatagg | 419667 | 758828 | see also 9911 line | | |
| 9916 | FLJ34969 | NM_152678.1 | 1394 | aagtttcatcattccattagaaa | 419673 | 758837 | see also 9911 line | | |
| 9917 | MGC29463 | NM_152686.1 | 767 | tcccccatatagtctgttctata | 32200 | 453488 | - | - | - |
| 9918 | MGC29463 | NM_152686.1 | 768 | cccccatatagtctgttctataa | 32201 | 453489 | see also 9917 line | | |
| 9919 | HIPK1 | NM_152696.3 | 335 | ctctcaccaggtagcaaatttca | 28163 | 503439 | kinase_related | - | - |
| 9920 | HIPK1 | NM_152696.3 | 668 | cagcggagaaggggattaccagc | 28174 | 503445 | see also 9919 line | | |
| 9921 | HIPK1 | NM_152696.3 | 875 | ttcccgcctaagcagtgaaaatg | 28177 | 503446 | see also 9919 line | | |
| 9922 | HIPK1 | NM_152696.3 | 1788 | aagcggagggttcacatgtatga | 28196 | 503465 | see also 9919 line | | |
| 9923 | HIPK1 | NM_152696.3 | 1790 | gcggagggttcacatgtatgata | 28197 | 503466 | see also 9919 line | | |
| 9924 | HIPK1 | NM_152696.3 | 2084 | ttgcactcagacagatccattcc | 28204 | 503473 | see also 9919 line | | |
| 9925 | HIPK1 | NM_152696.3 | 2264 | gggaagctgtacaccactaatgg | 28209 | 503477 | see also 9919 line | | |
| 9926 | HIPK1 | NM_152696.3 | 2267 | aagctgtacaccactaatggtag | 28210 | 503478 | see also 9919 line | | |
| 9927 | MGC27076 | NM_152699.2 | 448 | gtggctactcaaaatgttagtac | 190800 | 584346 | - | - | - |
| 9928 | MGC27076 | NM_152699.2 | 2129 | aagtccactggtctctcattact | 190834 | 584387 | see also 9927 line | | |
| 9929 | MGC27076 | NM_152699.2 | 2131 | gtccactggtctctcattactgt | 190835 | 584388 | see also 9927 line | | |
| 9930 | ITM1 | NM_152713.2 | 1292 | agctgtaatggtgcgtctaatgc | 211437 | 600243 | - | oligosaccharyl transferase | - |
| 9931 | FLJ25530 | NM_152722.3 | 879 | ctggaatacatggatcagaatga | 420090 | 786284 | - | - | - |
| 9932 | FLJ34588 | NM_152726.1 | 620 | ctgcagaagatcataagtaaaca | 145980 | 542647 | - | - | - |
| 9933 | CPNE2 | NM_152727.4 | 1145 | ctcatgttcaccgttggaataga | 420115 | 769721 | - | - | - |

Figure 46

| | | | | | | | | | |
|---|---|---|---|---|---|---|---|---|---|
| 9934 | CPNE2 | NM_152727.4 | 1757 | gagttccgcaacgcagcaaaaga | 420126 | 769726 | see also 9933 line | | |
| 9935 | BTBD9 | NM_152733.1 | 1453 | tgctgaatggggacactaagaat | 129491 | 530823 | - | - | - |
| 9936 | BTBD9 | NM_152733.1 | 1460 | tggggacactaagaattatgact | 129492 | 530825 | see also 9935 line | | |
| 9937 | MGC33993 | NM_152737.1 | 561 | atgcctctacaagatcatagact | 420280 | 809137 | - | - | - |
| 9938 | MGC33993 | NM_152737.1 | 660 | tagcctgcccgatgacaacaaca | 420285 | 809138 | see also 9937 line | | |
| 9939 | MGC33993 | NM_152737.1 | 671 | atgacaacaacatccttgtaaac | 420286 | 809139 | see also 9937 line | | |
| 9940 | HOXA9 | NM_152739.2 | 455 | cggccttatggcattaaacctga | 5086 | 444739 | transcription_regulator | transcriptional activator | leukemia |
| 9941 | SDK1 | NM_152744.2 | 1704 | gtggctctgtccggattcctagg | 420346 | 800074 | - | - | - |
| 9942 | FLJ31340 | NM_152748.2 | 1397 | gtgaagatttattctatcacagc | 420475 | 777698 | - | - | - |
| 9943 | FLJ31340 | NM_152748.2 | 1820 | atggctctctgtaccaacaatat | 420484 | 777711 | see also 9942 line | | |
| 9944 | FLJ31340 | NM_152748.2 | 1872 | cagggtcagatgattacacaaat | 420489 | 777716 | see also 9942 line | | |
| 9945 | FLJ31340 | NM_152748.2 | 1874 | gggtcagatgattacacaaattt | 420490 | 777718 | see also 9942 line | | |
| 9946 | MGC33190 | NM_152749.2 | 198 | ctccgacaatgtagatttagaag | 420518 | 732724 | - | - | - |
| 9947 | MGC33190 | NM_152749.2 | 247 | agggaggttatgaggcttaataa | 420520 | 732725 | see also 9946 line | | |
| 9948 | SEMA3D | NM_152754.1 | 1250 | gtcacggttgagatacaaagact | 420644 | 685499 | receptor_acitivity | - | - |
| 9949 | SEMA3D | NM_152754.1 | 1252 | cacggttgagatacaaagactac | 420645 | 685500 | see also 9948 line | | |
| 9950 | SEMA3D | NM_152754.1 | 1267 | aagactacatccaaatccttagc | 420646 | 685502 | see also 9948 line | | |
| 9951 | MGC33510 | NM_152765.2 | 427 | ctgtgtgggaatcgagcatatgg | 420882 | 801489 | - | - | - |
| 9952 | MGC33302 | NM_152778.1 | 360 | tgggttattgcttcatatagtct | 273839 | 655341 | - | - | - |
| 9953 | MGC33302 | NM_152778.1 | 1125 | aagattggcgagcgtgctattct | 273863 | 655367 | see also 9952 line | | |
| 9954 | LOC149420 | NM_152835.1 | 300 | gccagccaaagtacgatctaata | 88585 | 495919 | kinase_related | - | - |
| 9955 | LOC149420 | NM_152835.1 | 302 | cagccaaagtacgatctaatacg | 88586 | 495920 | see also 9954 line | | |
| 9956 | LOC149420 | NM_152835.1 | 340 | tagttacggtgttgtgtatgaag | 88588 | 495921 | see also 9954 line | | |
| 9957 | LOC149420 | NM_152835.1 | 451 | ctgggcactaagcagtatcaaga | 88591 | 495929 | see also 9954 line | | |
| 9958 | LOC149420 | NM_152835.1 | 536 | aagatgtcccacggctctaattc | 88595 | 495933 | see also 9954 line | | |

Figure 46

| 9959 | LOC149420 | NM_152835.1 | 1192 | tgggcgaatgaaacaactgatta | 88605 | 495950 | see also 9954 line | | |
|------|-----------|-------------|------|--------------------------|--------|--------|--------------------|---|---|
| 9960 | MGC3794 | NM_152902.2 | 444 | tggacctatacaacagattataa | 421545 | 756823 | - | - | - |
| 9961 | MGC3794 | NM_152902.2 | 498 | aaggttgtacctacaacagatca | 421546 | 756826 | see also 9960 line | | |
| 9962 | MGC3794 | NM_152902.2 | 506 | acctacaacagatcatatagata | 421547 | 756828 | see also 9960 line | | |
| 9963 | MGC3794 | NM_152902.2 | 737 | gacctacatgttacgagaatata | 421555 | 756833 | see also 9960 line | | |
| 9964 | MGC3794 | NM_152902.2 | 738 | acctacatgttacgagaatatac | 421556 | 756834 | see also 9960 line | | |
| 9965 | MGC3794 | NM_152902.2 | 804 | tccctcttcacggaacctaatga | 421559 | 756839 | see also 9960 line | | |
| 9966 | MGC3794 | NM_152902.2 | 810 | ttcacggaacctaatgaaatatc | 421560 | 756841 | see also 9960 line | | |
| 9967 | DRB1 | NM_152945.1 | 551 | atggagatatcgagtattgcagc | 60458 | 483927 | - | - | - |
| 9968 | DRB1 | NM_152945.1 | 559 | atcgagtattgcagcattattaa | 60459 | 483928 | see also 9967 line | | |
| 9969 | DRB1 | NM_152945.1 | 1004 | tagcatcagctatttatgcaaaa | 60482 | 483953 | see also 9967 line | | |
| 9970 | LOC129285 | NM_152994.2 | 365 | cagtttgacatttcgaaatctgc | 421696 | 735260 | - | - | - |
| 9971 | SIAT7C | NM_152996.1 | 318 | cccttcgaactcactatggatac | 212806 | 601196 | - | - | - |
| 9972 | SIAT7C | NM_152996.1 | 325 | aactcactatggatacataaatg | 212808 | 601197 | see also 9971 line | | |
| 9973 | SIAT7C | NM_152996.1 | 843 | ttcacgtctacgggatgataaat | 212825 | 601210 | see also 9971 line | | |
| 9974 | KCTD7 | NM_153033.1 | 577 | tgggactcatgccctattacaaa | 132806 | 536790 | channel | - | - |
| 9975 | AEBP2 | NM_153207.2 | 905 | aagtgaacgacatcagttaaaaa | 422139 | 454604 | - | - | - |
| 9976 | FLJ33761 | NM_153211.1 | 898 | aaggcccctttagctacattagc | 422161 | 734145 | - | - | - |
| 9977 | FLJ33761 | NM_153211.1 | 1770 | ctctggctacgactttgaaaaca | 422195 | 734168 | see also 9976 line | | |
| 9978 | FLJ33990 | NM_153226.1 | 261 | ctggacttggcttgttttacaca | 328803 | 694535 | - | - | - |
| 9979 | FLJ36812 | NM_153260.1 | 611 | atgcaatctgaaaaaactagaga | 422679 | 717700 | - | - | - |
| 9980 | SYT14 | NM_153262.1 | 43 | gtggagtacatgaacttatctgt | 275020 | 806265 | - | - | - |
| 9981 | SYT14 | NM_153262.1 | 269 | tggttcatcctctgaaagtgaag | 275027 | 806274 | see also 9980 line | | |
| 9982 | SYT14 | NM_153262.1 | 1358 | cagaccacccaatacatatgtta | 275055 | 806295 | see also 9980 line | | |
| 9983 | FLJ35827 | NM_153265.1 | 588 | ctggatctcggaggagcaattac | 422736 | 754573 | - | - | - |
| 9984 | FLJ35827 | NM_153265.1 | 589 | tggatctcggaggagcaattaca | 422737 | 754574 | see also 9983 line | | |
| 9985 | MAMDC2 | NM_153267.3 | 1139 | accgctggaatcccaatgtgaac | 328913 | 693998 | - | - | - |
| 9986 | KCTD6 | NM_153331.2 | 402 | gagactggggctatatgatgact | 132773 | 536771 | channel | - | - |
| 9987 | KCTD6 | NM_153331.2 | 540 | gagaccctcaaggcaattacttt | 132778 | 536776 | see also 9986 line | | |
| 9988 | LYK5 | NM_153335.3 | 470 | aacctagaagcttgttccaatga | 88833 | 488635 | kinase_related | - | - |
| 9989 | MGC46534 | NM_153340.2 | 1280 | gcccaggatgctatgtgtttagc | 422805 | 786187 | - | - | - |
| 9990 | MGC33214 | NM_153354.2 | 136 | taggtatacagctggttgttacc | 422973 | 786236 | - | - | - |
| 9991 | MGC33214 | NM_153354.2 | 712 | gacttgaaacagggtttacaaat | 422991 | 786253 | see also 9990 line | | |

Figure 46

| | | | | | | | | | |
|---|---|---|---|---|---|---|---|---|---|
| 9992 | C6orf158 | NM_153362.1 | 428 | gagaatggcacccgaaccttaac | 181698 | 574813 | - | - | - |
| 9993 | FLJ90013 | NM_153365.1 | 1258 | gagttgtaacaagctcaattaaa | 423096 | 784388 | - | - | - |
| 9994 | PI16 | NM_153370.1 | 547 | gtgcgtgtggggccacaacaagg | 223636 | 584545 | - | - | - |
| 9995 | HS6ST3 | NM_153456.1 | 26 | tggatgaaaggttcaacaagtgg | 218818 | 606664 | - | - | - |
| 9996 | MYCD | NM_153604.1 | 2105 | ttccatccccaactttttctaag | 33091 | 468256 | transcription_regulator | - | - |
| 9997 | LOC153222 | NM_153607.1 | 567 | gggaacagtgggatacatactgt | 30762 | 454388 | - | - | - |
| 9998 | LOC153222 | NM_153607.1 | 1933 | agcccagtatgaagctaataaag | 30796 | 454413 | see also 9997 line | | |
| 9999 | LOC153222 | NM_153607.1 | 2078 | aagcttgaaatcctcattaaaga | 30802 | 454422 | see also 9997 line | | |
| 10000 | LOC153222 | NM_153607.1 | 2124 | ggcaaacctcagaatttgttaac | 30804 | 454424 | see also 9997 line | | |
| 10001 | SEMA6D | NM_153617.1 | 2161 | ttcaactacaccagattacaaaa | 320904 | 816402 | receptor_acitivity | - | - |
| 10002 | CPNE8 | NM_153634.2 | 351 | aagagagaatcttcgttttgact | 423415 | 719103 | - | - | - |
| 10003 | SLC24A4 | NM_153646.1 | 1774 | ttctccataatgatagagtttaa | 327419 | 691231 | - | - | - |
| 10004 | MLR1 | NM_153686.4 | 154 | ctgtgtaggttttgagagtattt | 423476 | 658021 | - | - | - |
| 10005 | MLR1 | NM_153686.4 | 683 | cagcaaggggatggagtgttaga | 423493 | 657984 | see also 10004 line | | |
| 10006 | MLR1 | NM_153686.4 | 919 | gagctgtacagttcataaccaaa | 423501 | 658029 | see also 10004 line | | |
| 10007 | LOC91526 | NM_153697.1 | 186 | ttgtcaggagctcgtgtaaatgc | 423582 | 799591 | - | - | - |
| 10008 | STRC | NM_153700.2 | 2988 | aggacgggtggcatatgaacttc | 423667 | 746549 | - | - | - |
| 10009 | STRC | NM_153700.2 | 2991 | acgggtggcatatgaacttctgg | 423668 | 746550 | see also 10008 line | | |
| 10010 | MGC10084 | NM_153702.1 | 692 | agggtattcttatgcaatagttg | 423700 | 803732 | apoptosis | - | - |
| 10011 | MGC10084 | NM_153702.1 | 693 | gggtattcttatgcaatagttgg | 423701 | 803733 | see also 10010 line | | |
| 10012 | MGC10084 | NM_153702.1 | 725 | tacagagatggcttatagcttac | 423703 | 803735 | see also 10010 line | | |
| 10013 | MGC26979 | NM_153704.2 | 1303 | aactcggcgcattttcttagtgg | 282059 | 799863 | - | - | - |
| 10014 | MGC26979 | NM_153704.2 | 1316 | ttcttagtggatgcagtaagtgg | 282060 | 799864 | see also 10013 line | | |
| 10015 | MGC26979 | NM_153704.2 | 1644 | gtcccatgattgatttacagaca | 282069 | 799880 | see also 10013 line | | |
| 10016 | C6orf188 | NM_153711.1 | 620 | taggatggtgcctgatttgttca | 423833 | 804810 | - | - | - |
| 10017 | TTL | NM_153712.2 | 619 | agggcaacgtttggattgcaaag | 231870 | 616780 | - | - | - |
| 10018 | TTL | NM_153712.2 | 620 | gggcaacgtttggattgcaaagt | 231871 | 616781 | see also 10017 line | | |
| 10019 | TTL | NM_153712.2 | 863 | ttcagaaccatatcatgttgata | 231880 | 616788 | see also 10017 line | | |

Figure 46

| ID | Gene | Accession | Pos | Sequence | | | Function | | Disease |
|---|---|---|---|---|---|---|---|---|---|
| 10020 | RTN4 | NM_153828.1 | 722 | ctcgggctcagtggttgttgacc | 135481 | 540177 | apoptosis | protein binding | |
| 10021 | GPR110 | NM_153840.1 | 1598 | aagatgtgtgttggcttaactgg | 242741 | 630241 | receptor_acitivity, gpcr, signal_transduction | | |
| 10022 | CSE-C | NM_170601.1 | 67 | gacagaagtgcaggtattggttt | 423958 | 591741 | - | | |
| 10023 | TCFL4 | NM_170607.2 | 337 | gtgtagtgtccagagcttaatagc | 13041 | 468964 | transcription_regulator | | |
| 10024 | WDR17 | NM_170710.2 | 352 | ttggatcaacgttataatgaatt | 423971 | 577957 | - | | |
| 10025 | WDR17 | NM_170710.2 | 1056 | aagaacaacaccattgataatc | 423991 | 577989 | see also 10024 line | | |
| 10026 | WDR17 | NM_170710.2 | 1613 | ttgcctggagtcataaagattct | 424011 | 578011 | see also 10024 line | | |
| 10027 | WDR17 | NM_170710.2 | 1925 | ccgttcgaatctgggattatact | 424025 | 578030 | see also 10024 line | | |
| 10028 | WDR17 | NM_170710.2 | 4100 | ctggaatacgactcaatccattc | 424112 | 578134 | see also 10024 line | | |
| 10029 | RAB39B | NM_171998.1 | 490 | tacacgttcagccctaccaaatt | 110266 | 518362 | signal_transduction | | |
| 10030 | RAB39B | NM_171998.1 | 566 | tcgccacgaggcgagaaactgg | 110268 | 518365 | see also 10029 line | | |
| 10031 | RAB39B | NM_171998.1 | 590 | tgctgcatacggcgatgaagtaca | 110269 | 518366 | see also 10029 line | | |
| 10032 | RAB39B | NM_171998.1 | 672 | gacatatatgagctgcttaaaag | 110274 | 518367 | see also 10029 line | | |
| 10033 | RDH10 | NM_172037.1 | 1201 | tcctaggagcggacaagtgtatg | 227316 | 614614 | - | | |
| 10034 | EYA4 | NM_172105.1 | 2186 | aagctgtttgagcgtatagtgt | 205107 | 595862 | phosphatase, transcription_regulator | hydrolase | |
| 10035 | EYA4 | NM_172105.1 | 2196 | gagcgtatagtgtccagattgg | 205108 | 595863 | see also 10034 line | | |
| 10036 | EYA4 | NM_172105.1 | 2207 | gtccagattggcactaacataa | 205109 | 595864 | see also 10034 line | | |
| 10037 | EYA4 | NM_172105.1 | 2208 | tccagattggcactaacataac | 205110 | 595865 | see also 10034 line | | |
| 10038 | KCNAB1 | NM_172159.1 | 521 | tagcctgcacagagcacaattg | 260932 | 644278 | channel | | |
| 10039 | KLHDC1 | NM_172193.1 | 113 | gggggctacgtgtctattgaaga | 424273 | 801025 | - | | |
| 10040 | KLHDC1 | NM_172193.1 | 115 | gggctacgtgtcattgaagaca | 424274 | 801026 | see also 10039 line | | |
| 10041 | KLHDC1 | NM_172193.1 | 119 | tacgtgtctattgaagacaatga | 424275 | 801027 | see also 10039 line | | |
| 10042 | TFAP2BL1 | NM_172238.1 | 566 | atcaaacagtaccgtttgatgc | 13119 | 449425 | transcription_regulator | | |
| 10043 | TFAP2BL1 | NM_172238.1 | 839 | gcccaccgactttattaacctgc | 13120 | 449431 | see also 10042 line | | |
| 10044 | KCNH1 | NM_172362.1 | 1134 | atgggttgatccagggaagattgg | 121480 | 526655 | channel, signal_transduction | | |
| 10045 | KCNH1 | NM_172362.1 | 1138 | gtgatccagggaagattggtttt | 121481 | 526656 | see also 10044 line | | |
| 10046 | ELF1 | NM_172373.2 | 344 | ttcctggtctgatattctcaat | 3007 | 443212 | transcription_regulator | hematopoietic-associated factor 1B complex | |
| 10047 | SCUBE1 | NM_173050.1 | 494 | ccgtccaatgagggtatgaact | 152885 | 553763 | receptor_acitivity | | |
| 10048 | SCUBE1 | NM_173050.1 | 613 | accagaaggactgcacactaacc | 152886 | 553764 | see also 10047 line | | |
| 10049 | ABCA12 | NM_173076.2 | 386 | tacctcgcacctcgaaaccttcc | 68328 | 786481 | - | | ichthyosis congenita |

Figure 46

| 10050 | SHPRH | NM_173082.1 | 1432 | aactctactataatccatataca | 39251 | 466905 | transcription_regulator | - | - |
|---|---|---|---|---|---|---|---|---|---|
| 10051 | SHPRH | NM_173082.1 | 1434 | ctctactataatccatatacagg | 39252 | 466906 | see also 10050 line | | |
| 10052 | SHPRH | NM_173082.1 | 2783 | acggaaccagaagcgctatatgg | 39284 | 466939 | see also 10050 line | | |
| 10053 | SHPRH | NM_173082.1 | 2837 | gtggaggatctgccttgatgaag | 39285 | 466940 | see also 10050 line | | |
| 10054 | SHPRH | NM_173082.1 | 2838 | tggaggatctgccttgatgaagc | 39286 | 466941 | see also 10050 line | | |
| 10055 | SHPRH | NM_173082.1 | 3978 | cagttcttacttacaacacaaat | 39322 | 466990 | see also 10050 line | | |
| 10056 | SHPRH | NM_173082.1 | 4162 | cagagtatgaatcaaagctattt | 39328 | 466994 | see also 10050 line | | |
| 10057 | SHPRH | NM_173082.1 | 4300 | cagagcgatctatgaaagcaata | 39332 | 467002 | see also 10050 line | | |
| 10058 | SHPRH | NM_173082.1 | 4953 | gtggtcagaactctgatgaaaat | 39351 | 467027 | see also 10050 line | | |
| 10059 | SHPRH | NM_173082.1 | 5015 | aacgtggcaagatgtattagata | 39355 | 467034 | see also 10050 line | | |
| 10060 | SHPRH | NM_173082.1 | 5016 | acgtggcaagatgtattagatat | 39356 | 467035 | see also 10050 line | | |
| 10061 | TGS | NM_173083.2 | 665 | cagaagattggttttcgattacg | 424721 | 786208 | - | - | - |
| 10062 | TPH2 | NM_173353.2 | 1435 | atgagggactttgcaaagtcaat | 164568 | 562360 | - | - | - |
| 10063 | TPH2 | NM_173353.2 | 1437 | gagggactttgcaaagtcaatta | 164569 | 562361 | see also 10062 line | | |
| 10064 | SNF1LK | NM_173354.1 | 242 | accaaaacgcaggttgcaataaa | 100934 | 515081 | kinase_related | protein tyrosine kinase | - |
| 10065 | UPP2 | NM_173355.2 | 339 | cacaacctaccagcaatgtttgg | 237471 | 740332 | - | - | - |
| 10066 | LOC93380 | NM_173470.1 | 369 | cgctgcgcagcatcgttcttata | 424887 | 761440 | - | - | - |
| 10067 | LOC93380 | NM_173470.1 | 371 | ctgcgcagcatcgttcttatatg | 424888 | 761441 | see also 10066 line | | |
| 10068 | LOC93380 | NM_173470.1 | 372 | tgcgcagcatcgttcttatatgc | 424889 | 761442 | see also 10066 line | | |
| 10069 | MGC48972 | NM_173475.1 | 733 | cggttccctagcctcttaacaga | 424932 | 783432 | - | - | - |
| 10070 | MGC48972 | NM_173475.1 | 779 | aggatctctaccggtttacattt | 424935 | 783433 | see also 10069 line | | |
| 10071 | LSM11 | NM_173491.1 | 664 | ctgactctcactaggctatttga | 46321 | 471148 | - | - | - |
| 10072 | LSM11 | NM_173491.1 | 666 | gactctcactaggctatttgatc | 46322 | 471149 | see also 10071 line | | |
| 10073 | TTBK2 | NM_173500.2 | 834 | ttggcttggctcgacaatttacc | 103671 | 517145 | kinase_related | - | - |
| 10074 | TTBK2 | NM_173500.2 | 2449 | atccgtgtttgacaatagcatca | 103684 | 517157 | see also 10073 line | | |
| 10075 | SLC35F3 | NM_173508.1 | 1424 | tccgggtcatcgccatcatcatc | 425222 | 662643 | - | - | - |
| 10076 | MGC16664 | NM_173509.2 | 415 | gtgatcactggcggaatcctagc | 425227 | 799616 | - | - | - |
| 10077 | FLJ33814 | NM_173510.1 | 582 | gagattgaggacaggataattga | 425238 | 750268 | - | - | - |
| 10078 | FLJ90709 | NM_173514.1 | 1318 | tgcttatatgctggtgacattaa | 259561 | 641631 | - | - | - |
| 10079 | FLJ90709 | NM_173514.1 | 1536 | gccatatcttcggtgacatttat | 259565 | 641639 | see also 10078 line | | |
| 10080 | FLJ38964 | NM_173527.1 | 758 | ccgctcccgggaggtatcactgg | 27399 | 465840 | signal_transduction | - | - |
| 10081 | MGC47799 | NM_173545.1 | 239 | cagaagacatgccattcttgagg | 425780 | 715098 | - | - | - |

Figure 46

| 10082 | LOC201292 | NM_173547.2 | 1016 | ggcagaattatcgcaatctgacc | 425835 | 555130 | - | - | - |
|---|---|---|---|---|---|---|---|---|---|
| 10083 | RFXDC1 | NM_173560.1 | 1120 | tgggaacagtgggtgtttcatc | 38588 | 466298 | transcription_regulator | - | - |
| 10084 | RFXDC1 | NM_173560.1 | 1708 | cagaatttattggacaagtatat | 38607 | 466322 | see also 10083 line | | |
| 10085 | C10orf48 | NM_173576.1 | 528 | acgctagtgcaggtgtcaaattg | 1852 | 796993 | transcription_regulator | - | - |
| 10086 | C10orf48 | NM_173576.1 | 529 | cgctagtgcaggtgtcaaattgg | 1853 | 796994 | see also 10085 line | | |
| 10087 | C10orf48 | NM_173576.1 | 621 | aagttatacaacaagtatgttca | 1861 | 797003 | see also 10085 line | | |
| 10088 | C10orf48 | NM_173576.1 | 852 | agcttgttgaaccgttaccttaa | 1867 | 797010 | see also 10085 line | | |
| 10089 | FLJ35894 | NM_173616.1 | 428 | gggaaattggtggtttgatgttt | 426532 | 453706 | - | - | - |
| 10090 | FLJ35894 | NM_173616.1 | 819 | ctgtgtcaacaatcctcaaaatg | 426546 | 453728 | see also 10089 line | | |
| 10091 | FLJ90652 | NM_173618.1 | 343 | cggaggggacacccaagttgtct | 426583 | 770685 | - | - | - |
| 10092 | RTTN | NM_173630.2 | 1797 | accaagccttgcgtagcttttcc | 426747 | 685199 | - | - | - |
| 10093 | RTTN | NM_173630.2 | 1800 | aagccttgcgtagcttttcctat | 426748 | 685200 | see also 10092 line | | |
| 10094 | RTTN | NM_173630.2 | 2163 | ttcagggacgattgatgatgaca | 426758 | 685213 | see also 10092 line | | |
| 10095 | RTTN | NM_173630.2 | 2276 | tgcattctccttctaagcaaagc | 426759 | 685218 | see also 10092 line | | |
| 10096 | RTTN | NM_173630.2 | 3154 | atggtatcatgggagtgataatc | 426789 | 685256 | see also 10092 line | | |
| 10097 | RTTN | NM_173630.2 | 3155 | tggtatcatgggagtgataatct | 426790 | 685257 | see also 10092 line | | |
| 10098 | RTTN | NM_173630.2 | 4388 | cagaatctccttgtaattccaat | 426831 | 685282 | see also 10092 line | | |
| 10099 | RTTN | NM_173630.2 | 5115 | tggaatacctatcctctttgtcc | 426852 | 685299 | see also 10092 line | | |
| 10100 | RTTN | NM_173630.2 | 5845 | tgccatgcagctcctaagaaact | 426870 | 685317 | see also 10092 line | | |
| 10101 | FLJ90805 | NM_173633.1 | 1222 | atggcgtgtttctgatcatgacc | 426914 | 809624 | - | - | - |
| 10102 | MGC47816 | NM_173642.1 | 982 | gaccaggcatgcaacttagatgt | 302193 | 670729 | - | - | - |
| 10103 | SLC9A9 | NM_173653.1 | 1318 | ttggagcctttctagcaattttt | 258349 | 642580 | - | - | - |
| 10104 | MGC34132 | NM_173654.1 | 1272 | cacagaataccggaaaatcctta | 427111 | 787957 | - | - | - |
| 10105 | MGC34132 | NM_173654.1 | 1400 | cacacaacacggacatatttatt | 427121 | 787966 | see also 10104 line | | |
| 10106 | DKFZp434 C1418 | NM_173655.1 | 592 | ttgcgcttcccgggaattaaaac | 78264 | 501848 | - | - | - |
| 10107 | DKFZp434 C1418 | NM_173655.1 | 593 | tgcgcttcccgggaattaaaact | 78265 | 501849 | see also 10106 line | | |
| 10108 | DCBLD1 | NM_173674.1 | 276 | ggcacaatgacatctaagaatta | 318788 | 717960 | - | - | - |
| 10109 | LOC286046 | NM_173683.2 | 595 | tagggatcgtgtacattttctgc | 427363 | 783253 | - | - | - |
| 10110 | FLJ32440 | NM_173685.1 | 803 | cgccattgttcgcatgattgagt | 427413 | 727061 | - | - | - |

Figure 46

| 10111 | FLJ36664 | NM_173690.1 | 958 | acgcactcattattggtaattgt | 427477 | 804186 | - | - | - |
|---|---|---|---|---|---|---|---|---|---|
| 10112 | FLJ36664 | NM_173690.1 | 1571 | tcctctaatggccttcctatttg | 427499 | 804207 | see also 10111 line | | |
| 10113 | FLJ36664 | NM_173690.1 | 1831 | cacaactgccaagggatgaaaca | 427506 | 804216 | see also 10111 line | | |
| 10114 | MGC51029 | NM_173794.2 | 155 | cccctccccaagactatgaaagt | 427968 | 732213 | - | - | - |
| 10115 | MGC51029 | NM_173794.2 | 260 | cgggacctatggtagaaaaatac | 427973 | 732223 | see also 10114 line | | |
| 10116 | NEGR1 | NM_173808.1 | 532 | ctccccatcagcaaaaccatttg | 428179 | 794574 | - | - | - |
| 10117 | MGC39518 | NM_173822.1 | 364 | agctgtttgagctctatcgtagc | 428344 | 774971 | - | - | - |
| 10118 | MGC39518 | NM_173822.1 | 1377 | gagagccctcgagattcagtagt | 428374 | 775003 | see also 10117 line | | |
| 10119 | MGC39518 | NM_173822.1 | 1736 | gtctcgatccccaagtttcaata | 428388 | 775018 | see also 10117 line | | |
| 10120 | MGC39518 | NM_173822.1 | 1738 | ctcgatccccaagtttcaatatg | 428389 | 775019 | see also 10117 line | | |
| 10121 | MGC39518 | NM_173822.1 | 1742 | atccccaagtttcaatatgcagc | 428390 | 775021 | see also 10117 line | | |
| 10122 | KCP2 | NM_173852.2 | 396 | tggttggtctgtactacatcaac | 428501 | 715865 | - | - | - |
| 10123 | HOXC12 | NM_173860.1 | 395 | cgcctgcgctcggcttcaagtac | 5173 | 444784 | transcription_regulator | transcription factor | - |
| 10124 | BPIL3 | NM_174897.1 | 1306 | ctggctgagctggacatagtaga | 173355 | 816555 | - | - | - |
| 10125 | UBR1 | NM_174916.1 | 84 | gtgggatcagcaagttgattttt | 231473 | 541972 | - | - | - |
| 10126 | UBR1 | NM_174916.1 | 1095 | tgcccgtaagatccttcatgaat | 231501 | 542009 | see also 10125 line | | |
| 10127 | UBR1 | NM_174916.1 | 1847 | ctggtcttcatgtacgtttaagc | 231522 | 542032 | see also 10125 line | | |
| 10128 | UBR1 | NM_174916.1 | 2524 | cagcatagcaaggctgaacatat | 231537 | 542054 | see also 10125 line | | |
| 10129 | UBR1 | NM_174916.1 | 2947 | gacacagtgaagcgattaagaga | 231551 | 542065 | see also 10125 line | | |
| 10130 | UBR1 | NM_174916.1 | 4613 | gtgcactctgtagctatctatct | 231613 | 542106 | see also 10125 line | | |
| 10131 | UBR1 | NM_174916.1 | 4750 | gtggtcaggtaccctagaaaaag | 231620 | 542112 | see also 10125 line | | |
| 10132 | UBR1 | NM_174916.1 | 4751 | tggtcaggtaccctagaaaaaga | 231621 | 542113 | see also 10125 line | | |
| 10133 | PCSK9 | NM_174936.2 | 1084 | ctcataggcctggagtttattcg | 183183 | 576881 | - | - | - |
| 10134 | MGC20553 | NM_174938.3 | 531 | gtgaaattctacccacatgaacc | 192136 | 687129 | - | - | - |
| 10135 | MGC20553 | NM_174938.3 | 1905 | gagtttgagcagtttcactatga | 192160 | 687162 | see also 10134 line | | |
| 10136 | C14orf39 | NM_174978.1 | 330 | tggaagccaacatgtgatgtttt | 428838 | 739069 | - | - | - |
| 10137 | C14orf39 | NM_174978.1 | 333 | aagccaacatgtgatgttttcg | 428839 | 739070 | see also 10136 line | | |
| 10138 | C14orf39 | NM_174978.1 | 549 | gtgttggcatgtactgaacaatt | 428850 | 739075 | see also 10136 line | | |

Figure 46

| | | | | | | | |
|---|---|---|---|---|---|---|---|
| 10139 | C14orf39 | NM_174978.1 | 1237 | cagtaagacaagtaagagaatca | 428871 | 739100 | see also 10136 line |
| 10140 | C14orf39 | NM_174978.1 | 1591 | cagatcagtttaatgaacattat | 428883 | 739115 | see also 10136 line |
| 10141 | LOC131368 | NM_175056.1 | 345 | cagatctggcactgaatggaagg | 428917 | 803417 | - |
| 10142 | LOC131368 | NM_175056.1 | 585 | tacctgggcttcttacaaattt | 428925 | 803420 | see also 10141 line |
| 10143 | LOC131368 | NM_175056.1 | 590 | ggggcttcttacaaattagttg | 428926 | 803423 | see also 10141 line |
| 10144 | LOC131368 | NM_175056.1 | 717 | tgctcctttataacgattcaacc | 428934 | 803433 | see also 10141 line |
| 10145 | CNTN4 | NM_175607.1 | 1314 | gagcaaggaacactcaacataac | 429101 | 498547 | - |
| 10146 | CNTN4 | NM_175607.1 | 1482 | gtgggaggtgaagttgtcattga | 429106 | 498558 | see also 10145 line |
| 10147 | CNTN4 | NM_175607.1 | 1885 | aggattcagctggtgatttgatg | 429118 | 498570 | see also 10145 line |
| 10148 | CNTN4 | NM_175607.1 | 1944 | gtctgcatggtccaaacaagtgt | 429119 | 498572 | see also 10145 line |
| 10149 | LOC152831 | NM_155737.2 | 126 | cagggaatgaatggattttcttc | 202946 | 593517 | - |
| 10150 | LOC152831 | NM_155737.2 | 786 | gggaccgtgtcaaatattggatt | 202972 | 593536 | see also 10149 line |
| 10151 | LOC152831 | NM_155737.2 | 788 | gaccgtgtcaaatattggattac | 202973 | 593537 | see also 10149 line |
| 10152 | LOC152831 | NM_155737.2 | 789 | accgtgtcaaatattggattaca | 202974 | 593538 | see also 10149 line |
| 10153 | LOC152831 | NM_155737.2 | 792 | gtgtcaaatattggattacaatt | 202975 | 593539 | see also 10149 line |
| 10154 | PAN3 | NM_175854.4 | 1751 | tagcaaaattgggaacaatcaat | 429353 | 733821 | kinase_related |
| 10155 | KIS | NM_175866.1 | 902 | aacagttctgctattattgatca | 52358 | 473963 | kinase_related |
| 10156 | KIS | NM_175866.1 | 904 | cagttctgctattattgatcaca | 52359 | 473964 | see also 10155 line |
| 10157 | MGC45594 | NM_175907.2 | 399 | aacgcatctgacatcaactattc | 157844 | 557137 | - |
| 10158 | MGC45594 | NM_175907.2 | 400 | acgcatctgacatcaactattca | 157845 | 557138 | see also 10157 line |
| 10159 | MGC50831 | NM_175924.2 | 276 | tggtgacatggcgcttcaagtcc | 148404 | 750792 | - |
| 10160 | KENAE | NM_176816.2 | 830 | aggccctcgccatgcttgatatc | 429790 | 808429 | - |
| 10161 | KENAE | NM_176816.2 | 833 | ccctcgccatgcttgatatcaaa | 429791 | 808431 | see also 10160 line |
| 10162 | STX12 | NM_177424.1 | 770 | agctgcttactactcagaaaaaat | 270893 | 652729 | - |
| 10163 | PAQR3 | NM_177453.2 | 641 | ctgctatgtctcaggagtatttt | 327123 | 813301 | receptor_acitivity |
| 10164 | KIAA1946 | NM_177454.2 | 2179 | ctcctttatagacctgaaaaagg | 29891 | 634735 | - |
| 10165 | KIAA1946 | NM_177454.2 | 2536 | aagagatagcaagactaacatct | 29897 | 634745 | see also 10164 line |

Figure 46

| | | | | | | | | |
|---|---|---|---|---|---|---|---|---|
| 10166 | PKHD1L1 | NM_177531.2 | 377 | tgcaagtcattcaactcaaatta | 429895 | 628565 | - | - |
| 10167 | PKHD1L1 | NM_177531.2 | 3225 | caggttgaagtctatgtcaatgg | 430000 | 628673 | | see also 10166 line |
| 10168 | PKHD1L1 | NM_177531.2 | 10323 | tggcatgcagcaattgagataaa | 430192 | 628906 | | see also 10166 line |
| 10169 | PKHD1L1 | NM_177531.2 | 11242 | taggaattggagactacagaatt | 430215 | 628946 | | see also 10166 line |
| 10170 | PKHD1L1 | NM_177531.2 | 12511 | atggcctagtggaaatacaaca | 430264 | 628996 | | see also 10166 line |
| 10171 | PKHD1L1 | NM_177531.2 | 12513 | ggccttagtgaaatacaacaat | 430265 | 628997 | | see also 10166 line |
| 10172 | ALEX2 | NM_177949.1 | 2016 | accagcacctgcttgtcaattcc | 430283 | 721725 | - | - |
| 10173 | SYT12 | NM_177963.2 | 87 | accatctgagcgtcatcaagagc | 274999 | 657548 | | - |
| 10174 | DSG4 | NM_177986.2 | 277 | aagcgggagtggatcaagtttgc | 142532 | 806365 | | - |
| 10175 | DSG4 | NM_177986.2 | 813 | cagagagcaacacagtatgtaca | 142552 | 806378 | | see also 10174 line |
| 10176 | OTOP1 | NM_177998.1 | 1764 | tggctttgaacctggataattg | 430367 | 777766 | | - |
| 10177 | OTOP1 | NM_177998.1 | 1765 | ggctttgaacctggataattgt | 430368 | 777767 | | see also 10176 line |
| 10178 | OTOP1 | NM_177998.1 | 1841 | ccctcttgaggtctattgtaag | 430370 | 777771 | | see also 10176 line |
| 10179 | LOC162427 | NM_178126.2 | 746 | gtgtccgtggctacatgatgtcc | 430483 | 725582 | | - |
| 10180 | FUT8 | NM_178154.1 | 737 | ctggttcctgcgttggattatg | 210774 | 599611 | | - |
| 10181 | FUT8 | NM_178154.1 | 738 | tggttcctggcgttggattatgc | 210775 | 599612 | | see also 10180 line |
| 10182 | FUT8 | NM_178154.1 | 741 | ttcctggcgttggattattgctca | 210776 | 599613 | | see also 10180 line |
| 10183 | URB | NM_178274.2 | 628 | aggctactaccgcctctcatgatga | 430647 | 725061 | | - |
| 10184 | URB | NM_178274.2 | 2301 | tggatctgagagtcaagcaatac | 430669 | 725081 | | see also 10183 line |
| 10185 | URB | NM_178274.2 | 2377 | gtccgaatcaaagatatggaga | 430675 | 725087 | | see also 10183 line |
| 10186 | URB | NM_178274.2 | 2873 | gggatgcgctgcccagaaagatga | 430681 | 725101 | | see also 10183 line |
| 10187 | SPTBN1 | NM_178313.1 | 438 | cggggcaggtgccttacaactac | 119355 | 524150 | structural constituent of cytoskeleton | |
| 10188 | SPTBN1 | NM_178313.1 | 6754 | gggatctccacgggttagttacc | 119357 | 524256 | | see also 10187 line |
| 10189 | MGC48332 | NM_178450.2 | 445 | caccagagccccttcaatgacc | 265489 | 648571 | | - |
| 10190 | MGC54289 | NM_178454.1 | 201 | tggagtaagtgcacttagcatgc | 430771 | 720832 | | - |
| 10191 | MGC54289 | NM_178454.1 | 416 | aagccaatttacatggattaacc | 430783 | 720838 | | see also 10190 line |
| 10192 | MGC54289 | NM_178454.1 | 428 | atggattaaccctctatgcaact | 430784 | 720839 | | see also 10190 line |
| 10193 | LOC151963 | NM_178496.2 | 779 | ttgatgaggggacaatcagtaaa | 430865 | 798399 | | - |
| 10194 | LOC151963 | NM_178496.2 | 1360 | tacttggtcaagaagactatgc | 430885 | 798412 | | see also 10193 line |
| 10195 | PIGW | NM_178517.2 | 1275 | taccatgttcttggaaacttatc | 431042 | 729250 | | - |

Figure 46

| | | | | | | | | | |
|---|---|---|---|---|---|---|---|---|---|
| 10196 | FLJ25770 | NM_178555.2 | 805 | ttgggctcagctattagtaaaat | 431235 | 794249 | - | - | - |
| 10197 | FLJ25770 | NM_178555.2 | 807 | gggctcagctattagtaaaatac | 431236 | 794251 | see also 10196 line | | |
| 10198 | MGC50844 | NM_178562.2 | 279 | tcgtcagcccgctggtgaaatac | 431285 | 691344 | - | - | - |
| 10199 | MGC50844 | NM_178562.2 | 283 | cagcccgctggtgaaatacctgc | 431286 | 691345 | see also 10198 line | | |
| 10200 | ZDHHC21 | NM_178566.2 | 907 | tacttgctacgcactgatgtttt | 431320 | 726525 | - | - | - |
| 10201 | LYRIC | NM_178812.2 | 899 | gcctgggaaactaaaattagtca | 431342 | 720726 | - | - | - |
| 10202 | LYRIC | NM_178812.2 | 970 | tggttcattggattcaactatcc | 431344 | 720729 | see also 10201 line | | |
| 10203 | LYRIC | NM_178812.2 | 985 | aactatccctgggatagaaaata | 431345 | 720730 | see also 10201 line | | |
| 10204 | LYRIC | NM_178812.2 | 1089 | atgttcaagttagcaactttaaa | 431347 | 720734 | see also 10201 line | | |
| 10205 | LYRIC | NM_178812.2 | 1400 | cagtctaccacttctgattatca | 431354 | 720741 | see also 10201 line | | |
| 10206 | LYRIC | NM_178812.2 | 1402 | gtctaccacttctgattatcagt | 431355 | 720742 | see also 10201 line | | |
| 10207 | LYRIC | NM_178812.2 | 1423 | gtgggatgttagccgtaatcaac | 431356 | 720744 | see also 10201 line | | |
| 10208 | LYRIC | NM_178812.2 | 2001 | cacagaccaagtctgaaactagc | 431372 | 720762 | see also 10201 line | | |
| 10209 | CKLFSF4 | NM_178818.2 | 628 | cagaaattgctgccgtgatattt | 328584 | 694512 | - | - | - |
| 10210 | DKFZp586 M1819 | NM_178819.2 | 1149 | gccctacaccaacggaatcattg | 216248 | 603458 | - | - | - |
| 10211 | DKFZp586 M1819 | NM_178819.2 | 1155 | caccaacggaatcattgcaaagg | 216250 | 603459 | see also 10210 line | | |
| 10212 | DKFZp586 M1819 | NM_178819.2 | 1211 | ttcgtcgaagtggtagtagtaag | 216252 | 603461 | see also 10210 line | | |
| 10213 | DKFZp586 M1819 | NM_178819.2 | 1212 | tcgtcgaagtggtagtagtaagg | 216253 | 603462 | see also 10210 line | | |
| 10214 | TMEM16D | NM_178826.2 | 416 | gtggccaaggatgtcaatattct | 431783 | 804105 | - | - | - |
| 10215 | TMEM16D | NM_178826.2 | 704 | gagaaagagtcctctctaataaa | 431787 | 804117 | see also 10214 line | | |
| 10216 | TMEM16D | NM_178826.2 | 710 | gagtcctctctaataaatagtga | 431788 | 804119 | see also 10214 line | | |
| 10217 | TMEM16D | NM_178826.2 | 717 | ctctaataaatagtgacattatc | 431790 | 804120 | see also 10214 line | | |
| 10218 | TMEM16D | NM_178826.2 | 727 | tagtgacattatctttgtgaagt | 431791 | 804121 | see also 10214 line | | |
| 10219 | TMEM16D | NM_178826.2 | 848 | ttcatgagcaggatcgataaaca | 431792 | 804123 | see also 10214 line | | |
| 10220 | TMEM16D | NM_178826.2 | 851 | atgagcaggatcgataaacaaat | 431793 | 804124 | see also 10214 line | | |

EP 1 752 536 A1

Figure 46

| 10221 | TMEM16D | NM_178826.2 | 1141 | tcccctgcatgagggaagttata | 431799 | 804135 | see also 10214 line | | |
|---|---|---|---|---|---|---|---|---|---|
| 10222 | LOC118812 | NM_178832.2 | 382 | gcgtcggagtcttcattcgatat | 431994 | 811892 | - | - | - |
| 10223 | SEZ6 | NM_178860.2 | 2282 | gccacttcaagctctttacctcc | 432039 | 710940 | - | - | - |
| 10224 | SEZ6 | NM_178860.2 | 2615 | agcacagccgacgcctcatatcc | 432041 | 710944 | see also 10223 line | | |
| 10225 | KCTD13 | NM_178863.2 | 431 | cggaggttgggtgctgattgacc | 132667 | 536640 | channel | - | - |
| 10226 | KCTD13 | NM_178863.2 | 718 | accgcagtaacaacaagtactcc | 132670 | 536643 | see also 10225 line | | |
| 10227 | NXF | NM_178864.2 | 2504 | tactgatgagttgcatcaactcc | 254728 | 639291 | signal_transduction | - | - |
| 10228 | ARHGAP8 | NM_181333.1 | 232 | tccgcaggttgaagttcatgagt | 297661 | 759769 | - | - | - |
| 10229 | ARHGAP8 | NM_181334.1 | 636 | gcccctcatcagtcacaagtttg | 297669 | 665127 | - | - | - |
| 10230 | OXR1 | NM_181354.2 | 1750 | aaggcatcgattacataagttct | 282887 | 671236 | - | - | - |
| 10231 | PAX3 | NM_181457.1 | 1216 | atggctttcaaccatctcattcc | 8880 | 447553 | transcription_regulator、apoptosis | - | rhabdomyosarcoma、alveolar rhabdomyosarcoma、craniofacial-deafness-hand syndrome、Waardenburg syndrome |
| 10232 | ZNF445 | NM_181489.3 | 2745 | cagatggagttccaatctctacc | 432164 | 783023 | transcription_regulator | - | - |
| 10233 | OIP2 | NM_181503.1 | 57 | ctggagtattacaggagatttct | 53127 | 472641 | - | - | - |
| 10234 | OIP2 | NM_181503.1 | 514 | tacagttgcctgaagttactata | 53138 | 472659 | see also 10233 line | | |
| 10235 | MRPL21 | NM_181512.1 | 130 | ttctcgaaggttcaattcacaga | 432372 | 473194 | - | - | - |
| 10236 | MRPL21 | NM_181512.1 | 429 | tggttggggcagacaacttcacg | 432378 | 473200 | see also 10235 line | | |
| 10237 | PKD1L3 | NM_181536.1 | 2681 | ggcatccctggaaccagtttaca | 432503 | 806191 | channel、signal_transduction | - | - |
| 10238 | CUTL1 | NM_181552.1 | 1402 | agcggggttaagtcaagactttt | 2644 | 442858 | transcription_regulator | RNA polymerase II transcription factor | leiomyoma |
| 10239 | CUTL1 | NM_181552.1 | 1403 | gcggggttaagtcaagacttttt | 2645 | 442859 | see also 10238 line | | |
| 10240 | CUTL1 | NM_181552.1 | 1404 | cggggttaagtcaagactttttc | 2646 | 442860 | see also 10238 line | | |
| 10241 | CUTL1 | NM_181552.1 | 1405 | ggggttaagtcaagactttttca | 2647 | 442861 | see also 10238 line | | |
| 10242 | CUTL1 | NM_181552.1 | 3605 | tggctgaacgaccccaacaatgt | 2664 | 442889 | see also 10238 line | | |
| 10243 | AUP1 | NM_181575.2 | 461 | ctgtagcacccctctactcaata | 216136 | 603484 | - | - | - |
| 10244 | AUP1 | NM_181575.2 | 465 | agcacccctctactcaatagtcc | 216137 | 603485 | see also 10243 line | | |
| 10245 | PITPNC1 | NM_181671.1 | 1048 | ttcgggaatacgagaaaaacatg | 271843 | 673432 | signal_transduction | - | - |
| 10246 | PITPNC1 | NM_181671.1 | 1049 | tcgggaatacgagaaaaacatgc | 271844 | 673433 | see also 10245 line | | |
| 10247 | OGT | NM_181672.1 | 250 | cacagaaccaacgaaacgtatgc | 134303 | 601967 | signal_transduction | - | - |
| 10248 | OGT | NM_181672.1 | 259 | aacgaaacgtatgctttccttcc | 134305 | 601968 | see also 10247 line | | |
| 10249 | LOC144233 | NM_181708.1 | 214 | ggctcgacgtggggtgtaactcc | 432857 | 738192 | - | - | - |

Figure 46

| | | | | | | | | | |
|---|---|---|---|---|---|---|---|---|---|
| 10250 | LOC255104 | NM_181719.1 | 845 | agctggcctgacaggatacaaga | 433013 | 740863 | - | - | - |
| 10251 | TMEM13 | NM_181744.1 | 106 | tagtggctggcttttacctaaca | 433063 | 807015 | - | - | - |
| 10252 | GPR120 | NM_181745.1 | 659 | tggtcattgtgatcagttactcc | 239414 | 806944 | receptor_acitivity、gpcr、signal_transduction | - | - |
| 10253 | NCOA7 | NM_181782.1 | 2826 | gacataagttctttagaacttgg | 303354 | 774639 | - | - | - |
| 10254 | NRPS998 | NM_181806.1 | 2317 | gtgggctgttataatggattagt | 433614 | 611792 | - | - | - |
| 10255 | NRPS998 | NM_181806.1 | 2319 | gggctgttataatggattagttt | 433615 | 611793 | see also 10254 line | | |
| 10256 | C6orf46 | NM_181842.1 | 1151 | aggtggaaacccccttaaagaaca | 129551 | 815102 | transcription_regulator | - | - |
| 10257 | BCL6B | NM_181844.2 | 239 | cagtggcttcttctattcaattt | 129250 | 456323 | - | - | - |
| 10258 | AMIGO2 | NM_181847.2 | 620 | tcgtcagctgcaccaacaaaaac | 325998 | 691746 | - | - | - |
| 10259 | CPEB2 | NM_182485.1 | 710 | tacccttacaggtgagatctagt | 433844 | 482092 | - | - | - |
| 10260 | CPEB2 | NM_182485.1 | 712 | cccttacaggtgagatctagttt | 433845 | 482093 | see also 10259 line | | |
| 10261 | CPEB2 | NM_182485.1 | 1070 | aacgcttctctcgaaaagttttt | 433856 | 482112 | see also 10259 line | | |
| 10262 | CPEB2 | NM_182485.1 | 1341 | acgtccttggaatttaagtgata | 433865 | 482125 | see also 10259 line | | |
| 10263 | USP12 | NM_182488.1 | 266 | tcctaatgacagtctccaaattc | 188824 | 702789 | - | - | - |
| 10264 | USP12 | NM_182488.1 | 815 | gtcttacttgtgaaactataagc | 188846 | 702808 | see also 10263 line | | |
| 10265 | USP12 | NM_182488.1 | 1203 | aggccattatattgcaatagtta | 188863 | 702821 | see also 10263 line | | |
| 10266 | FLJ40919 | NM_182508.1 | 240 | tgcagacccgctacattcacagc | 434021 | 797393 | - | - | - |
| 10267 | FLJ25161 | NM_182522.2 | 846 | tagcagtggcaataaagtcaaaa | 434154 | 794293 | - | - | - |
| 10268 | LOC203522 | NM_182540.1 | 819 | ttcgttgggatcaaaggttattt | 434254 | 480282 | - | - | - |
| 10269 | MGC57341 | NM_182559.1 | 571 | cagtgaggtataatgactatatt | 182419 | 574799 | - | - | - |
| 10270 | MGC57341 | NM_182559.1 | 573 | gtgaggtataatgactatattca | 182420 | 574800 | see also 10269 line | | |
| 10271 | FLJ36070 | NM_182574.1 | 799 | ttggaacttcagaccccttaaact | 27389 | 453921 | - | - | - |
| 10272 | SRPK2 | NM_182691.1 | 744 | cagtacgttgtgtgaagagtatc | 101280 | 515363 | - | - | - |
| 10273 | SRPK2 | NM_182691.1 | 747 | tacgttgtgtgaagagtatcatt | 101281 | 515364 | see also 10272 line | | |
| 10274 | SRPK2 | NM_182691.1 | 748 | acgttgtgtgaagagtatcattc | 101282 | 515365 | see also 10272 line | | |
| 10275 | SRPK2 | NM_182691.1 | 2104 | gagcctctttgatgtacttgtgg | 101317 | 515384 | see also 10272 line | | |
| 10276 | FLJ37306 | NM_182765.1 | 754 | atggaaaggtccacgaacaaaag | 435215 | 615240 | - | - | - |
| 10277 | FLJ37306 | NM_182765.1 | 1201 | ctgtcagtacccattcgttattt | 435227 | 615254 | see also 10276 line | | |
| 10278 | FLJ37306 | NM_182765.1 | 1565 | tggtttagcagctttaaatgtga | 435244 | 615270 | see also 10276 line | | |
| 10279 | NTT73 | NM_182767.1 | 1633 | tgctttgtttatggcatagataa | 255886 | 640078 | - | neurotransmitter:sodium symporter | - |

Figure 46

| | | | | | | | | | |
|---|---|---|---|---|---|---|---|---|---|
| 10280 | CHX10 | NM_182894.1 | 533 | agcggcgacacaggacaatcttt | 2261 | 442727 | - | | - |
| 10281 | CHX10 | NM_182894.1 | 536 | ggcgacacaggacaatctttacc | 2262 | 442728 | see also 10280 line | | - |
| 10282 | ERG | NM_182918.1 | 770 | aacacaggggtgcagctttat | 3413 | 443485 | transcription_regulator, signal_transduction | transcription factor | leukemia, Ewing sarcoma, Ewing's sarcoma, Ewing's tumours |
| 10283 | ERG | NM_182918.1 | 772 | cacagggggtgcagcttttattt | 3414 | 443486 | see also 10282 line | | - |
| 10284 | ADAMTS9 | NM_182920.1 | 4400 | ttgtggtcgagggcataaacaac | 155867 | 787989 | - | | |
| 10285 | FLJ20403 | NM_182976.1 | 438 | agccgcttcgaggtragttatgg | 435563 | 707220 | - | | - |
| 10286 | FLJ20403 | NM_182976.1 | 482 | ccggaatagccttgactctttcg | 435565 | 707222 | see also 10285 line | | |
| 10287 | FLJ20403 | NM_182976.1 | 536 | agcaccttactccegttcaaaat | 435566 | 707223 | see also 10285 line | | |
| 10288 | FLJ20403 | NM_182976.1 | 676 | atcctgaaagtacgtttggaagc | 435570 | 707224 | see also 10285 line | | |
| 10289 | FLJ20403 | NM_182976.1 | 737 | aggccgaggccgaggacatatgg | 435571 | 707226 | see also 10285 line | | |
| 10290 | GPRK2L | NM_182982.1 | 325 | gtggtcgtagtaaaaatggaag | 84256 | 503096 | gpcr, kinase_related, signal_transduction | | - |
| 10291 | GSTO2 | NM_183239.1 | 1133 | acctgtatatccatgattgatta | 216673 | 604901 | - | | - |
| 10292 | GSTO2 | NM_183239.1 | 1135 | ctgtatatccatgattgattacc | 216674 | 604902 | see also 10291 line | | |
| 10293 | GSTO2 | NM_183239.1 | 1299 | agggcttcttgaatctctatttt | 216678 | 604904 | see also 10291 line | | |
| 10294 | C6orf145 | NM_183373.1 | 48 | gggcacgtcgctcgtgaacatgt | 435667 | 679723 | - | | - |
| 10295 | C6orf145 | NM_183373.1 | 51 | cacgtcgctcgtgaacatgttcg | 435668 | 679724 | see also 10294 line | | |
| 10296 | ACCN1 | NM_183377.1 | 1311 | ctccaagggggacctctactatg | 262439 | 645846 | channel | amiloride-sensitive sodium channel | - |
| 10297 | BPAG1 | NM_183380.1 | 774 | aaggtcggatgcgttttcacaga | 115986 | 522172 | cell_cycle, signal_transduction | structural molecule | epidermolysis bullosa |
| 10298 | EML5 | NM_183387.1 | 5458 | gtggctattgaatgaaaaaatgg | 436019 | 577653 | - | | |
| 10299 | EML5 | NM_183387.1 | 5736 | ctgctataaacggcatgtctatg | 436025 | 577662 | see also 10298 line | | |
| 10300 | EML5 | NM_183387.1 | 5737 | tgctataaacggcatgtctatga | 436026 | 577663 | see also 10298 line | | |
| 10301 | EML5 | NM_183387.1 | 6014 | cccatgtgacaaatattcgattt | 436035 | 577668 | see also 10298 line | | |
| 10302 | EML5 | NM_183387.1 | 6017 | atgtgacaaatattcgatttacc | 436036 | 577669 | see also 10298 line | | |
| 10303 | OTOF | NM_194248.1 | 1377 | cccggttctatgtgaaaatttac | 282829 | 692911 | - | | deafness |
| 10304 | LOC91752 | NM_194250.1 | 2356 | tactggttccataaaagtagaag | 436112 | 789505 | - | | - |
| 10305 | GPR151 | NM_194251.1 | 630 | gagagcttatgaccaatgtaaaa | 239498 | 623808 | - | | - |
| 10306 | DKFZp686L1814 | NM_194282.1 | 1959 | tacaaaatcactgtgtttgaaat | 436277 | 777888 | - | | |
| 10307 | FLJ39441 | NM_194285.1 | 1588 | ggccctgggagatccataaagtgg | 436339 | 788043 | - | | |
| 10308 | FRBZ1 | NM_194314.1 | 1715 | tacgagaaagaactacttttgaaa | 436778 | 648522 | - | | |
| 10309 | PLEKHE1 | NM_194449.1 | 1414 | tggccataatcaaatatrgtgaac | 237094 | 621522 | - | | |
| 10310 | PLEKHE1 | NM_194449.1 | 3412 | cagtgatgagggcattgtcatca | 237126 | 621553 | see also 10309 line | | |

Figure 46

| | | | | | | | | | |
|---|---|---|---|---|---|---|---|---|---|
| 10311 | LOC253012 | NM_198151.1 | 768 | ttctgataaagggctaaaagtag | 437132 | 804770 | - | - | - |
| 10312 | LOC253012 | NM_198151.1 | 1086 | aagtataactggaatatcactat | 437141 | 804778 | see also 10311 line | | |
| 10313 | ASTN2 | NM_198186.1 | 1100 | aagcccctgaaaagattctcagc | 339027 | 708542 | - | - | - |
| 10314 | FAM13C1 | NM_198215.1 | 1636 | ttgaccatctccgagaaactagg | 437277 | 715077 | - | - | - |
| 10315 | LOC255743 | NM_198278.1 | 271 | gaggtggcccaggcaaatagtgt | 437457 | 550663 | - | - | - |
| 10316 | KCTD8 | NM_198353.1 | 1239 | ttcttccgaccacctcagaaaat | 437684 | 536809 | - | - | - |
| 10317 | LOC157567 | NM_198401.1 | 500 | aggtgctaccccttagttctgg | 437705 | 785692 | - | - | - |
| 10318 | PTPLB | NM_198402.1 | 166 | cacggcgtacctggtcatctaca | 437709 | 588693 | - | - | - |
| 10319 | PTPLB | NM_198402.1 | 256 | tagctaccatagcctttattatt | 437711 | 588698 | see also 10318 line | | |
| 10320 | PTPLB | NM_198402.1 | 547 | atgggccaggtacacacttttca | 437716 | 588711 | see also 10318 line | | |
| 10321 | PAQR10 | NM_198403.1 | 768 | tggtgctggtacccactactatg | 312143 | 677631 | - | - | - |
| 10322 | KCTD4 | NM_198404.1 | 256 | accctcaacgttggtggatattt | 437730 | 536724 | channel | - | - |
| 10323 | KCTD4 | NM_198404.1 | 257 | ccctcaacgttggtggatattta | 437731 | 536725 | see also 10322 line | | |
| 10324 | KCTD4 | NM_198404.1 | 303 | gaccaagtacccagacactttcc | 437733 | 536727 | see also 10322 line | | |
| 10325 | KCTD4 | NM_198404.1 | 307 | aagtacccagacactttccttga | 437734 | 536728 | see also 10322 line | | |
| 10326 | KCTD4 | NM_198404.1 | 415 | gtcctaaacttcctacgaaatgg | 437738 | 536730 | see also 10322 line | | |
| 10327 | KCTD4 | NM_198404.1 | 563 | gagagactactttcttggaaata | 437743 | 536734 | see also 10322 line | | |
| 10328 | KCTD4 | NM_198404.1 | 565 | gagactactttcttggaaataac | 437744 | 536735 | see also 10322 line | | |
| 10329 | KCTD4 | NM_198404.1 | 761 | atggcactcgacttgtactaaag | 437754 | 536743 | see also 10322 line | | |
| 10330 | KCTD4 | NM_198404.1 | 762 | tggcactcgacttgtactaaagg | 437755 | 536744 | see also 10322 line | | |
| 10331 | KCTD4 | NM_198404.1 | 882 | ttccaaagggtcaattgttcaca | 437759 | 536750 | see also 10322 line | | |
| 10332 | UNQ8193 | NM_198450.1 | 796 | tagatatgtacagcactagaagc | 437859 | 725896 | - | - | - |
| 10333 | PAQR9 | NM_198504.1 | 813 | cacactcttcgtgcacttctacc | 438745 | 813022 | - | - | - |
| 10334 | FLJ25621 | NM_198514.1 | 433 | atggcagggactcatcagatatg | 438891 | 647627 | - | - | - |
| 10335 | FLJ25621 | NM_198514.1 | 434 | tggcagggactcatcagatatgg | 438892 | 647628 | see also 10334 line | | |
| 10336 | FLJ25621 | NM_198514.1 | 838 | tacaatcacaagattaaagttgt | 438897 | 647634 | see also 10334 line | | |
| 10337 | MGC71806 | NM_198516.1 | 1320 | agctgtggtgccgctacctaaat | 438939 | 784162 | - | - | - |
| 10338 | ATP9B | NM_198531.2 | 457 | gggttttgtatgaacaattcaag | 439241 | 495395 | - | - | - |
| 10339 | FLJ30851 | NM_198553.1 | 229 | accccaattttctgaaggttacc | 439465 | 788943 | - | - | - |
| 10340 | MGC52022 | NM_198563.1 | 275 | gtccgtggaggatatggtttta | 439533 | 736463 | - | - | - |
| 10341 | MGC52022 | NM_198563.1 | 378 | ttgttcactgtacctcatcaact | 439536 | 736465 | see also 10340 line | | |

Figure 46

| ID | Gene | Accession | Pos | Sequence | Num1 | Num2 | Comment |
|---|---|---|---|---|---|---|---|
| 10342 | MGC52022 | NM_198563.1 | 711 | ttgggtagtggacaattcctca | 439546 | 736474 | see also 10340 line |
| 10343 | FLJ32363 | NM_198566.1 | 528 | gccctagatttttcgaaactcttc | 439616 | 721628 | - |
| 10344 | FLJ32363 | NM_198566.1 | 529 | ccctagatttttcgaaactcttca | 439617 | 721629 | see also 10343 line |
| 10345 | FLJ32363 | NM_198566.1 | 735 | cagcatgaatttacagtacatt | 439625 | 721636 | see also 10343 line |
| 10346 | FLJ44216 | NM_198567.1 | 2459 | caactgcagctccaataaaatg | 439675 | 791827 | - |
| 10347 | FLJ41410 | NM_198581.1 | 2114 | tacttccagtacctttacctaa | 439792 | 479439 | - |
| 10348 | RAB15 | NM_198686.1 | 119 | tccaccatcggtgttgactttaa | 439868 | 465095 | - |
| 10349 | GABRG2 | NM_198904.1 | 1436 | ttcaaggccctaccattgatat | 178750 | 571958 | channel, receptor_acitivity, signal_transduction |
| 10350 | ALS2CR17 | NM_198945.1 | 2982 | gccttagaaggatgattaataa | 440108 | 783975 | - |
| 10351 | NAPE-PLD | NM_198990.1 | 939 | gaggtggtttatgaaataccagc | 440335 | 803617 | - |
| 10352 | SYT10 | NM_198992.1 | 1338 | aagccacagtatggaaagatatt | 440368 | 657533 | - |
| 10353 | 24b2/STAC2 | NM_198993.1 | 625 | aacaggggcttgcgatgtraagatg | 440386 | 648616 | - |
| 10354 | PRES | NM_198999.1 | 810 | tgcctagtgtctgtaggtttgg | 440527 | 643576 | - |
| 10355 | PRES | NM_198999.1 | 939 | aagcggtacagtggaatctttc | 440531 | 643582 | see also 10354 line |
| 10356 | PRES | NM_198999.1 | 940 | agcggtacagtggaatcttttcc | 440532 | 643583 | see also 10354 line |
| 10357 | PRES | NM_198999.1 | 1179 | gtggatgtcgttggaacacttcc | 440540 | 643591 | see also 10354 line |
| 10358 | PRES | NM_198999.1 | 1463 | gacacagcttgcaggttgtttgg | 440544 | 643598 | see also 10354 line |
| 10359 | PRES | NM_198999.1 | 2191 | ttgattctgttggagtgaaaact | 440568 | 643619 | see also 10354 line |
| 10360 | DBCCR1L | NM_199051.1 | 438 | agggatttagcacaagatacaag | 440660 | 770051 | - |
| 10361 | DBCCR1L | NM_199051.1 | 1010 | ttgagctacattgcttgcaattc | 440677 | 770069 | see also 10360 line |
| 10362 | DBCCR1L | NM_199051.1 | 1131 | aagagaatcttctcgaataact | 440683 | 770073 | see also 10360 line |
| 10363 | DBCCR1L | NM_199051.1 | 1257 | tgcatttgggacaatggattct | 440688 | 770080 | see also 10360 line |
| 10364 | DBCCR1L | NM_199051.1 | 2350 | cagagaccgtgtaaataaactct | 440713 | 770100 | see also 10360 line |
| 10365 | DBCCR1L | NM_199051.1 | 2352 | gagaccgtgtaaataaaactctcc | 440714 | 770101 | see also 10360 line |
| 10366 | ATBF1 | NM_006885.2 | 7604 | gagccgcgtgagcttacaaatga | 139 | - | see also 4813 line |
| 10367 | GTF2IRD1 | NM_005685.2 | 2501 | gtgctggtccctataaactaat | 220 | - | transcription_regulator |
| 10368 | HIF1A | NM_001530.2 | 1097 | aggccgctcaatttatgaatatt | 252 | - | see also 1050 line |
| 10369 | ICSBP1 | NM_002163.1 | 454 | tggagtgcggtcgctcgaaatc | 318 | - | see also 1387 line |
| 10370 | MYEF2 | NM_016132.2 | 644 | aggagagacacgtccctgatatgg | 343 | - | see also 6688 line |

Figure 46

| | | | | | | | | | |
|---|---|---|---|---|---|---|---|---|---|
| 10371 | MYF5 | NM_005593.1 | 536 | atggcatgcccgaatgtaacagt | 386 | - | transcription_regulator | RNA polymerase II transcription factor、enhancer binding | - |
| 10372 | MYOD1 | NM_002478.3 | 573 | ctgcacgtcgagcaatccaaacc | 396 | - | transcription_regulator | MyoD/E47 | - |
| 10373 | NR5A2 | NM_003822.2 | 1397 | ttgatcaacgagagttcgtatgt | 444 | - | see also 2604 line | | |
| 10374 | RFX1 | NM_002918.2 | 93 | atggcaacacaggcgtatactga | 458 | - | transcription_regulator | translation regulator | - |
| 10375 | RFX3 | NM_002919.2 | 1803 | aagatacacgtcgcttaatcacc | 507 | - | see also 1964 line | | |
| 10376 | TFAP4 | NM_003223.1 | 287 | aggtgccctctttgcaacatttc | 548 | - | transcription_regulator | translation regulator | - |
| 10377 | AATF | NM_012138.2 | 1336 | gtgcctttgaacgctcaatcttg | 573 | - | transcription_regulator、apoptosis | - | - |
| 10378 | ADNP | NM_015339.2 | 2368 | atgaccgcctcaactatcactct | 646 | - | see also 6378 line | | |
| 10379 | AEBP1 | NM_001129.2 | 2431 | cggcgagcggctagtatcctacc | 686 | - | transcription_regulator | - | - |
| 10380 | AF5Q31 | NM_014423.1 | 784 | gaggcacgaccgtgagtcatata | 707 | - | see also 5667 line | | |
| 10381 | AHR | NM_001621.2 | 2990 | gtgggtcagatgcagtacaatcc | 861 | - | see also 1076 line | | |
| 10382 | ALX3 | NM_006492.1 | 875 | cggctcaccctggcatctactcc | 869 | - | transcription_regulator | transcription factor | - |
| 10383 | ALX4 | NM_021926.1 | 223 | gcgacaagttcggcacaactttc | 874 | - | see also 8197 line | | |
| 10384 | AR | NM_000044.2 | 2195 | gagtcgcgactactacaactttc | 890 | - | see also 16 line | | |
| 10385 | ARNT | NM_001668.2 | 334 | atgatcagatgtctaacgataag | 918 | - | transcription_regulator、signal_transduction | - | - |
| 10386 | ARNT2 | NM_014862.1 | 1220 | ctgtcggtcatgtatcgatttcg | 969 | - | see also 6062 line | | |
| 10387 | ARNTL | NM_001178.2 | 1395 | tcggcacgcgatagatggaaagt | 1010 | - | see also 767 line | | |
| 10388 | ARNTL2 | NM_020183.3 | 439 | gcgcgtaaactggacaaacttac | 1040 | - | transcription_regulator、signal_transduction | - | - |
| 10389 | ASCL1 | NM_004316.1 | 918 | gcgctcggcggtcgagtacatcc | 1090 | - | transcription_regulator | transcription factor | small cell lung cancer、Alzheimer disease |
| 10390 | ASCL2 | NM_005170.1 | 612 | gcgctcagccgtggagtacatcc | 1091 | - | transcription_regulator | transcription factor | |
| 10391 | ATF1 | NM_005171.2 | 623 | gggacttcagacattaaccatga | 1096 | - | transcription_regulator | translation regulator | sarcoma、clear cell sarcoma、osteosarcoma、clear cell sarcoma of the soft tissue、fibrous histiocytoma、malignant melanoma |

EP 1 752 536 A1

Figure 46

| | | | | | | | | | |
|---|---|---|---|---|---|---|---|---|---|
| 10392 | ATF3 | NM_001674.1 | 402 | gaggtagcccctgaagaagatga | 1105 | - | transcription_regulator | - | |
| 10393 | ATF6 | NM_007348.1 | 1037 | tagggttagaggcgagattaaag | 1128 | - | transcription_regulator, signal_transduction | translation regulator | - |
| 10394 | ATF7 | NM_006856.1 | 293 | gtgggactcttcaatgaactagc | 1157 | - | see also 4802 line | | |
| 10395 | ATOH1 | NM_005172.1 | 551 | atgttatcccgtcgttcaacaac | 1173 | - | transcription_regulator | translation regulator | - |
| 10396 | ATRX | NM_000489.2 | 7323 | caggcgttagcattaagtagaca | 1303 | - | see also 379 line | | |
| 10397 | BACH1 | NM_001186.1 | 831 | gagtccgtactggggaatctagt | 1323 | - | - | translation regulator | - |
| 10398 | BAPX1 | NM_001189.2 | 858 | gcgcgacgaccagagacaatacc | 1365 | - | see also 771 line | | |
| 10399 | BARHL1 | NM_020064.2 | 212 | ttgggatcgactccattctctcc | 1366 | - | transcription_regulator | - | - |
| 10400 | BARX1 | NM_021570.2 | 654 | tcgagaagcagaagtacctttcc | 1371 | - | transcription_regulator | transcription factor | - |
| 10401 | BARX2 | NM_003658.3 | 193 | aaggagacctgcgattactttga | 1376 | - | transcription_regulator | RNA polymerase II transcription factor | - |
| 10402 | BAZ1B | NM_023005.2 | 3145 | cagcattgactaccgattcaacc | 1450 | - | transcription_regulator | - | - |
| 10403 | BCL6 | NM_001706.2 | 679 | gtggacacttgccggaagtttat | 1488 | - | see also 1132 line | | |
| 10404 | BHLHB2 | NM_003670.1 | 1011 | gagaaaggatcggcgcaattaag | 1511 | - | see also 2497 line | | |
| 10405 | BHLHB3 | NM_030762.1 | 522 | gcgttccactcgggatttcaaac | 1531 | - | see also 8938 line | | |
| 10406 | BNC | NM_001717.2 | 859 | agggtcattgcccgaacaatata | 1546 | - | see also 1144 line | | |
| 10407 | BRD8 | NM_006696.3 | 605 | atggttcgctctcctatagattc | 1586 | - | see also 4727 line | | |
| 10408 | BRF1 | NM_001519.2 | 1149 | gaggctcacggaatttgaagaca | 1644 | - | transcription_regulator | - | - |
| 10409 | BTAF1 | NM_003972.1 | 2492 | ttggtaatcgagtaaacaacaat | 1720 | - | transcription_regulator | transcription factor | - |
| 10410 | BTG2 | NM_006763.2 | 161 | gagcgagcagaggcttaaggtct | 1823 | - | transcription_regulator | transcription factor | - |
| 10411 | C10orf1 | NM_020527.1 | 803 | ctggccgacctaattcaattagc | 1842 | - | - | transcription factor | - |
| 10412 | C10orf48 | NM_173576.1 | 1188 | cagaagtcgtcccatatagcaga | 1878 | - | see also 10085 line | | |
| 10413 | C20orf17 | NM_173485.2 | 2979 | ctgcagtccgtcctgaacaatca | 1912 | - | - | - | - |
| 10414 | CART1 | NM_006982.1 | 1101 | aaggagcacaccgccaatatttc | 1961 | - | see also 4878 line | | |
| 10415 | CBFA2T1 | NM_004349.2 | 1883 | gtggccgtaaagcgagtgaaacc | 1993 | - | see also 2910 line | | |
| 10416 | CBFA2T2 | NM_005093.2 | 940 | aaggtgcggactcttgttcttgc | 2005 | - | transcription_regulator | - | - |
| 10417 | CBFA2T3 | NM_005187.4 | 817 | ttgacgatcgaggagtttcattc | 2032 | - | transcription_regulator | - | - |

EP 1 752 536 A1

Figure 46

| 10418 | CBFB | NM_001755.1 | 74 | gagccgcgagtgtgagattaagt | 2034 | - | transcription_regulator | - | leukemia |
|---|---|---|---|---|---|---|---|---|---|
| 10419 | CBL | NM_005188.1 | 2321 | cagattgatagctgtacgtatga | 2097 | - | see also 3644 line | | |
| 10420 | CCRN4L | NM_012118.2 | 743 | ctggcctaccagcctgatatatt | 2113 | - | transcription_regulator | transcription factor | - |
| 10421 | CDX2 | NM_001265.2 | 724 | aggacgaaagacaaatatcgagt | 2139 | - | transcription_regulator | translation regulator | - |
| 10422 | CDX4 | NM_005193.1 | 759 | cagccctggggaactacctaaca | 2149 | - | transcription_regulator | transcription factor | - |
| 10423 | CHD4 | NM_001273.1 | 2019 | tggcgggacttaccttacgatca | 2181 | - | see also 838 line | | |
| 10424 | CHES1 | NM_005197.1 | 265 | ctgcctgacatccgattagaaga | 2240 | - | cell_cycle、transcription_regulator、signal_transduction | translation regulator | - |
| 10425 | CHX10 | NM_182894.1 | 453 | cagctcggattctgaagatgttt | 2259 | - | see also 10280 line | | |
| 10426 | CITED1 | NM_004143.2 | 539 | acgtcgaggcctgcacttgatgt | 2264 | - | transcription_regulator | transcription factor | melanoma |
| 10427 | CITED2 | NM_006079.2 | 867 | tcgacgaggaagttcttatgtcc | 2273 | - | transcription_regulator | transcription factor | - |
| 10428 | CLOCK | NM_004898.2 | 2508 | atggtacctagtactatgcttat | 2337 | - | see also 3398 line | | |
| 10429 | CNOT7 | NM_013354.4 | 956 | acggataggaccacaacatcagg | 2348 | - | transcription_regulator、signal_transduction | - | - |
| 10430 | CNOT8 | NM_004779.4 | 413 | tcgtagttccatagattaccaat | 2363 | - | transcription_regulator | transcription factor | myelodysplastic syndrome |
| 10431 | CREB3L1 | NM_052854.1 | 1017 | aggagagccgtcgtaagaagaag | 2416 | - | see also 9436 line | | |
| 10432 | CREBBP | NM_004380.1 | 2205 | gaggtcgcgtttacataaacaag | 2467 | - | transcription_regulator、signal_transduction | PEBP2/CBP heterodimer | Rubinstein-Taybi syndrome |
| 10433 | CREBL2 | NM_001310.2 | 290 | aggtggttggaggcaaagtaaag | 2523 | - | transcription_regulator、signal_transduction | - | - |
| 10434 | CRX | NM_000554.2 | 762 | atgggtctccgagctcctatttc | 2569 | - | transcription_regulator | translation regulator | cone-rod dystrophy |
| 10435 | CSDA | NM_003651.3 | 567 | aagaataacccacggaaatatct | 2577 | - | transcription_regulator | transcription co-repressor | - |
| 10436 | CTCF | NM_006565.1 | 1786 | cagtgtgattacgcttgtagaca | 2613 | - | see also 4603 line | | |
| 10437 | CUTL1 | NM_001913.2 | 368 | cagcgcctgcacgatattgaaac | 2630 | - | transcription_regulator | - | leiomyoma |

EP 1 752 536 A1

Figure 46

| | | | | | | | | | |
|---|---|---|---|---|---|---|---|---|---|
| 10438 | DLX1 | NM_178120.2 | 805 | ccggctggaaccctaactcttca | 2674 | - | transcription_regulator | transcription factor | - |
| 10439 | DLX2 | NM_004405.2 | 18 | cagtctagtggctgatatgcact | 2675 | - | transcription_regulator | transcription factor | - |
| 10440 | DLX3 | NM_005220.2 | 355 | cagcgctccccagcacgattact | 2678 | - | see also 3662 line | | |
| 10441 | DLX5 | NM_005221.3 | 629 | gcgacccaatggcgtgtaactcg | 2694 | - | transcription_regulator | transcription factor | - |
| 10442 | DMRT1 | NM_021951.1 | 923 | gggcctccccggaccttatgtgc | 2700 | - | transcription_regulator | transcription factor | dementia、gonadal dysgenesis |
| 10443 | DMRT2 | NM_006557.3 | 535 | gggctttccgggaaacagaataa | 2703 | - | transcription_regulator | - | - |
| 10444 | DMRT3 | NM_021240.2 | 517 | acggcaagtcggcagacaataca | 2734 | - | see also 8142 line | | |
| 10445 | DMRTB1 | NM_033067.1 | 980 | ctgaccgtcctgtttgatactga | 2751 | - | transcription_regulator | - | - |
| 10446 | DMTF1 | NM_021145.1 | 2044 | cagtcgatagtgatattcagtca | 2814 | - | see also 8108 line | | |
| 10447 | DRIL1 | NM_005224.1 | 1768 | tggagatcaacggcatcatgtac | 2839 | - | transcription_regulator | translation regulator | - |
| 10448 | DSCR1 | NM_004414.4 | 653 | atgcgaccccagtcataaactat | 2847 | - | transcription_regulator、signal_transduction | transcription factor | Alzheimer disease |
| 10449 | DSIPI | NM_004089.2 | 523 | gcgtgagaacaccctgttgaaga | 2852 | - | transcription_regulator | - | - |
| 10450 | E2F1 | NM_005225.1 | 949 | gtggactcttcggagaactttca | 2860 | - | transcription regulator、apoptosis、cell_cycle | E2F-1/DP-1complex | chondrosarcoma |
| 10451 | E2F2 | NM_004091.2 | 1276 | ctgaggacaacctgcagatatat | 2870 | - | transcription_regulator、cell_cycle | E2F-4/DP-1 complex | - |
| 10452 | E2F3 | NM_001949.2 | 1398 | cagcgatctcttcgatgcttacg | 2899 | - | transcription_regulator、cell_cycle | translation regulator | - |
| 10453 | E2F4 | NM_001950.2 | 772 | aggaagcctcacgtccaaatagt | 2908 | - | - | E2F-4/DP-1 complex | - |
| 10454 | E2F5 | NM_001951.2 | 394 | ctgtaatactaaagaagtcatag | 2918 | - | see also 1240 line | | |
| 10455 | E2F6 | NM_001952.3 | 483 | ttgatgtatcgctggtttattta | 2948 | - | transcription_regulator、cell_cycle | transcription factor | - |
| 10456 | E4F1 | NM_004424.2 | 1595 | gtggcaagcgctacaagactaag | 2955 | - | transcription_regulator | sodium:dicarboxylate/tricarboxylate cotransporter | - |
| 10457 | EGR3 | NM_004430.2 | 477 | gcgactcggtagtccattacaat | 2962 | - | see also 2963 line | | |
| 10458 | EGR4 | NM_001965.1 | 1057 | cggcgacggcggagagtttctgg | 2975 | - | transcription_regulator | transcription factor | - |

EP 1 752 536 A1

Figure 46

| | | | | | | | | | |
|---|---|---|---|---|---|---|---|---|---|
| 10459 | EHF | NM_012153.2 | 947 | cagccgagctatgagatattact | 2996 | - | see also 5151 line | | |
| 10460 | ELF1 | NM_172373.2 | 1741 | cagcaggtccttactagcaatgt | 3048 | - | see also 10046 line | | |
| 10461 | ELF2 | NM_006874.1 | 995 | gagaagggtgtagctagagttgt | 3083 | - | see also 4810 line | | |
| 10462 | ELF3 | NM_004433.3 | 4231 | ccggcgactcgtctacaagtttg | 3091 | - | transcription_regulator | - | - |
| 10463 | ELF4 | NM_001421.1 | 656 | atgtcaaccgcggaagtcttact | 3095 | - | transcription_regulator | - | - |
| 10464 | ELF5 | NM_001422.2 | 808 | gagcgggttgaccgaaggttagt | 3134 | - | see also 994 line | | |
| 10465 | ELK1 | NM_005229.2 | 573 | gtggcccctgctgctatacatgc | 3144 | - | see also 3667 line | | |
| 10466 | ELK3 | NM_005230.1 | 669 | aagcacgagccgcaacgaataca | 3149 | - | transcription_regulator, signal_transduction | translation regulator | - |
| 10467 | ELK4 | NM_001973.1 | 216 | atgatctgttggacctctaatga | 3156 | - | transcription_regulator | - | - |
| 10468 | EN1 | NM_001426.2 | 1838 | tgggtgtactgcacacgttattc | 3186 | - | transcription_regulator | transcription factor | - |
| 10469 | EOMES | NM_005442.2 | 1400 | gaggtcggtacggcgttcaatcc | 3215 | - | transcription_regulator | transcription factor | - |
| 10470 | EP300 | NM_001429.1 | 5911 | ccgggatgcccaatgtatctaac | 3348 | - | see also 995 line | | |
| 10471 | ERF | NM_006494.1 | 370 | gggccctgcgctattactataac | 3382 | - | see also 4547 line | | |
| 10472 | ERG | NM_004449.2 | 62 | aggaccagtcgttgtttgagtgt | 3391 | - | see also 2984 line | | |
| 10473 | ESR1 | NM_000125.1 | 1233 | ttggccaagcccgctcatgatca | 3423 | - | receptor_acitivity, transcription_regulator, signal_transduction | steroid binding | atherosclerosis, liposarcoma, breast cancer, estrogen resistance |
| 10474 | ESR2 | NM_001437.1 | 494 | gagcacggctccatatacatacc | 3437 | - | see also 1005 line | | |
| 10475 | ESRRB | NM_004452.2 | 501 | ctggactcgccacccatgtttgc | 3461 | - | transcription_regulator, receptor_acitivity | steroid hormone receptor | - |
| 10476 | ESRRG | NM_001438.1 | 774 | aagtacaagcgcaggatagatgc | 3486 | - | see also 1006 line | | |
| 10477 | ESX1L | NM_153448.2 | 652 | gagggtgctaatgttgagaaaca | 3506 | - | transcription_regulator | - | - |
| 10478 | ETS1 | NM_005238.1 | 734 | ccgctatacctcggattacttca | 3529 | - | see also 3672 line | | |
| 10479 | ETS2 | NM_005239.2 | 344 | atgactccgccaactgtgaattg | 3538 | - | transcription_regulator | Ets1/ Ets2 complex | leukemia, prostate cancer |
| 10480 | ETV1 | NM_004956.2 | 1109 | gagtaccacgacccagtgtatga | 3560 | - | transcription_regulator | transcription factor | Ewing sarcoma, Ewing's sarcoma, Ewing's tumours |
| 10481 | ETV3 | NM_005240.1 | 477 | tcggtcaagtggtaagatacaaa | 3577 | - | transcription_regulator | transcription factor | Ewing's sarcoma |
| 10482 | ETV4 | NM_001986.1 | 1258 | ccgctcgctccgatactattatg | 3595 | - | transcription_regulator | translation regulator | Ewing's sarcoma |

436

EP 1 752 536 A1

Figure 46

| | | | | | | | | | |
|---|---|---|---|---|---|---|---|---|---|
| 10483 | ETV5 | NM_004454.1 | 1542 | gagagcgatacgtctacaaattt | 3611 | - | transcription_regulator | transcription factor | - |
| 10484 | ETV6 | NM_001987.2 | 320 | aagaggactttcgctatcgatct | 3621 | - | see also 1267 line | | |
| 10485 | ETV7 | NM_016135.2 | 996 | ctgcgccactattataagcttaa | 3648 | - | transcription_regulator | specific RNA polymerase II transcription factor | - |
| 10486 | EVX1 | NM_001989.2 | 567 | cggacaccgagtcggatttctat | 3654 | - | transcription_regulator | transcription factor | - |
| 10487 | FHX | NM_018416.2 | 2365 | tggtgaatcggctcaattggtcc | 3678 | - | see also 7459 line | | |
| 10488 | FKHL18 | NM_004118.3 | 1038 | ccggacgccaggaatgttcttct | 3692 | - | transcription_regulator | transcription factor | - |
| 10489 | FLI1 | NM_002017.2 | 906 | gaggggcacaaacgatcagtaag | 3710 | - | see also 1296 line | | |
| 10490 | FLJ12287 | NM_022367.2 | 1669 | gggtgccccgagccaactgtagt | 3741 | - | see also 8351 line | | |
| 10491 | FLJ12895 | NM_023926.3 | 694 | gggcatcctgcctgataaggtcc | 3745 | - | transcription_regulator | - | - |
| 10492 | FLJ21616 | NM_024567.1 | 627 | ctgcgacgagggagtcgatttac | 3768 | - | see also 8645 line | | |
| 10493 | FLJ23614 | NM_152695.2 | 643 | gggccaccggagctgaactatgg | 3786 | - | transcription_regulator | - | - |
| 10494 | FLJ25169 | NM_152568.1 | 135 | cagacactcctgcatgtctttct | 3802 | - | transcription_regulator | - | - |
| 10495 | FLJ25680 | NM_153216.1 | 936 | cagtggatatcccccactataca | 3815 | - | transcription_regulator | - | - |
| 10496 | FLJ30678 | NM_144657.1 | 782 | aggcccagcattacataacttat | 3829 | - | - | - | - |
| 10497 | FLJ33779 | NM_182572.2 | 1417 | ctggatggtccacctcattgacc | 3868 | - | transcription_regulator | - | - |
| 10498 | FLJ35867 | NM_152455.2 | 2062 | cggagtgcacgactcattagaca | 3891 | - | transcription_regulator | - | - |
| 10499 | FLJ35936 | NM_173464.1 | 891 | acgattactggtgcgatgttaat | 3914 | - | transcription_regulator | - | - |
| 10500 | FMNL2 | NM_052905.1 | 1439 | atgtcgttcgggaaatctacaaa | 3959 | - | transcription_regulator | - | - |
| 10501 | FOSL2 | NM_005253.2 | 1511 | gggcgctgtagtggtgaaacagg | 4002 | - | see also 3697 line | | |
| 10502 | FOXA1 | NM_004496.2 | 395 | ccgtcccggtcagcaacatgaac | 4006 | - | transcription_regulator | translation regulator | - |
| 10503 | FOXA2 | NM_021784.3 | 316 | acggcatgaacacgtacatgagc | 4011 | - | see also 8169 line | | |
| 10504 | FOXC1 | NM_001453.1 | 1572 | cgggaatagtagctgtcaaatgg | 4026 | - | transcription_regulator | transcriptional activator | Axenfeld-Rieger anomaly(-)、 iridogoniodysgenesis(-) |
| 10505 | FOXD1 | NM_004472.1 | 1766 | ctgtccagtgtcgagaactttac | 4030 | - | transcription_regulator | transcription factor | - |

437

Figure 46

| 10506 | FOXD2 | NM_004474.1 | 3357 | gaggccttccttctctatagacc | 4033 | - | transcription_regulator | transcription factor | - |
|---|---|---|---|---|---|---|---|---|---|
| 10507 | FOXD3 | NM_012183.1 | 1348 | gtgccactctccggacagtttct | 4036 | - | transcription_regulator | transcriptional activator | - |
| 10508 | FOXF2 | NM_001452.1 | 1411 | cagagcgtctgtcaggatattaa | 4043 | - | transcription_regulator | translation regulator | - |
| 10509 | FOXG1B | NM_005249.3 | 1458 | acgtcccgcacccgtcaatgact | 4049 | - | see also 3690 line | | |
| 10510 | FOXH1 | NM_003923.1 | 1542 | agggctgctctgcgatctagacg | 4062 | - | transcription_regulator, signal_transduction | translation regulator | - |
| 10511 | FOXJ1 | NM_001454.1 | 695 | cagatcccacctggcagaattca | 4065 | - | transcription_regulator | transcription factor | - |
| 10512 | FOXL1 | NM_005250.1 | 906 | cagcctccgtccgcctttcaacg | 4069 | - | transcription_regulator | transcription factor | - |
| 10513 | FOXM1 | NM_021953.1 | 1151 | tgggcgcacggcggaagatgaag | 4099 | - | transcription_regulator | translation regulator | - |
| 10514 | FOXN1 | NM_003593.2 | 402 | ccgtacaagcggcctttccatga | 4112 | - | transcription_regulator | specific RNA polymerase II transcription factor | immunodeficiency |
| 10515 | FOXO1A | NM_002015.2 | 1712 | caggacaataagtcgagttatgg | 4149 | - | see also 1293 line | | |
| 10516 | FOXO3A | NM_001455.1 | 2648 | ctgtcagccagtctatgcaaacc | 4181 | - | see also 1014 line | | |
| 10517 | FOXP1 | NM_032682.3 | 1423 | atgcgctggacgatagaagtaca | 4200 | - | see also 9272 line | | |
| 10518 | FOXP2 | NM_014491.1 | 1010 | gagggctagacctcactactaac | 4225 | - | see also 5707 line | | |
| 10519 | FOXP3 | NM_014009.2 | 864 | gagaagggcagggcacaatgtct | 4247 | - | transcription_regulator | transcription factor | diabetes |
| 10520 | FOXP4 | NM_138457.1 | 2032 | tggacgagcgggagtatcagaag | 4255 | - | see also 9622 line | | |
| 10521 | FOXQ1 | NM_033260.2 | 588 | gtgcacgcagcaagccatatacg | 4257 | - | transcription_regulator | - | - |
| 10522 | FUBP1 | NM_003902.2 | 774 | atggtgttagagttaattcgtga | 4284 | - | see also 2659 line | | |
| 10523 | G10 | NM_003910.2 | 632 | cagcagagaactctatgaatatt | 4333 | - | transcription_regulator | transcription factor | - |
| 10524 | GABPB2 | NM_002041.2 | 377 | gtggaccgaacaccattacatat | 4338 | - | see also 1317 line | | |
| 10525 | GAS41 | NM_006530.2 | 270 | aagggtgttactatcgttaaacc | 4369 | - | see also 4564 line | | |
| 10526 | GAS7 | NM_003644.1 | 920 | caggcccagtccaaatggtttga | 4405 | - | transcription_regulator, cell_cycle | - | - |
| 10527 | GATA1 | NM_002049.2 | 848 | aagcgcctgattgtcagtaaacg | 4414 | - | see also 1322 line | | |
| 10528 | GATA2 | NM_032638.3 | 1366 | ccggcacctgttgtgcaaattgt | 4422 | - | see also 9271 line | | |
| 10529 | GATA5 | NM_080473.3 | 893 | gcggcctctggctatgaagaaag | 4436 | - | transcription_regulator | translation regulator | - |

Figure 46

| 10530 | GATA6 | NM_005257.3 | 1662 | atgcttgtggactctacatgaaa | 4438 | - | see also 3699 line | | |
|---|---|---|---|---|---|---|---|---|---|
| 10531 | GLI2 | NM_005270.2 | 1253 | ccgcacaccgctgctcaaagaga | 4449 | - | transcription_regulator、signal_transduction | - | - |
| 10532 | GLI3 | NM_000168.2 | 481 | gagggccgttaccattacgatcc | 4461 | - | see also 138 line | | |
| 10533 | GSH-2 | NM_133267.1 | 998 | tcgactccggaggattgaaatcg | 4523 | - | see also 9563 line | | |
| 10534 | GSH1 | NM_145657.1 | 535 | gagcgcgagttcgcttctaatat | 4525 | - | transcription_regulator | - | - |
| 10535 | GTF2A2 | NM_004492.1 | 315 | caggggctctctaaatacgtaca | 4531 | - | see also 3043 line | | |
| 10536 | GTF2H3 | NM_001516.3 | 535 | gtgcgttgcagtatatgaacttc | 4559 | - | transcription_regulator | translation factor、nucleic acid binding | - |
| 10537 | GTF2H4 | NM_001517.3 | 264 | ggggtattggaccgattgtatgg | 4569 | - | - | transcription factor | - |
| 10538 | GTF2I | NM_001518.2 | 978 | atgctgacaggtcaatactatct | 4585 | - | see also 1043 line | | |
| 10539 | HAND1 | NM_004821.1 | 568 | gagacgcactgagagcattaaca | 4591 | - | transcription_regulator | transcription factor | - |
| 10540 | HCFC1 | NM_005334.1 | 5339 | gggcaccgtccctgactataacc | 4624 | - | see also 3716 line | | |
| 10541 | HCLS1 | NM_005335.3 | 668 | aagagcgctgtcggcttcaatga | 4638 | - | transcription_regulator | transcription factor | - |
| 10542 | HDAC1 | NM_004964.2 | 870 | tcggttaggttgcttcaatctaa | 4672 | - | transcription_regulator、apoptosis | transcription factor | - |
| 10543 | HDAC2 | NM_001527.1 | 1110 | tggctacacaatccgtaatgttg | 4714 | - | see also 1047 line | | |
| 10544 | HESX1 | NM_003865.1 | 432 | aagactgtgttccattaatgaaa | 4725 | - | transcription_regulator | transcription factor | pituitary hormone deficiency、septo optic dysplasia |
| 10545 | HEY1 | NM_012258.2 | 286 | ccgacgagaccggatcaataaca | 4743 | - | see also 5239 line | | |
| 10546 | HEY2 | NM_012259.1 | 225 | aggcgtcgggatcggataaataa | 4758 | - | see also 5240 line | | |
| 10547 | HEYL | NM_014571.2 | 348 | ccgagccctggcagttgacttcc | 4768 | - | transcription_regulator | - | - |
| 10548 | HHEX | NM_002729.2 | 621 | caggagaaccctcaaagcaataa | 4776 | - | - | transcription factor | - |
| 10549 | HIF3A | NM_022462.2 | 900 | ctgttccgcctacgagtacatcc | 4785 | - | transcription_regulator、signal_transduction | - | - |
| 10550 | HIRA | NM_003325.3 | 2869 | aggaccgttagccataatccagg | 4843 | - | see also 2255 line | | |
| 10551 | HIVEP3 | NM_024503.1 | 2656 | cagccggcaatcgaattacctt | 4854 | - | see also 8620 line | | |
| 10552 | HKR3 | NM_005341.1 | 874 | atggggatggcgattacatgtct | 4915 | - | transcription_regulator | translation regulator | - |
| 10553 | HLX1 | NM_021958.2 | 1106 | cagttcttcgcatctctagatcc | 4929 | - | transcription_regulator | transcriptional activator | - |
| 10554 | HLXB9 | NM_005515.2 | 844 | gggcatgatcctgcctaagatgc | 4940 | - | transcription_regulator | RNA polymerase II transcription factor | sacral agenesis syndrome |

EP 1 752 536 A1

Figure 46

| | | | | | | | | | |
|---|---|---|---|---|---|---|---|---|---|
| 10555 | HMG20B | NM_006339.1 | 819 | cagcatccctttagctttcaatc | 4971 | - | transcription_regulator | - | |
| 10556 | HMGB2 | NM_002129.2 | 353 | aaggagaagtcgaagtttgaaga | 4976 | - | transcription_regulator | transcription factor | - |
| 10557 | HNF4A | NM_000457.3 | 610 | gcgacattcgggcgaagaagatt | 4985 | - | transcription_regulator、receptor_acitivity | - | diabetes、diabetes mellitus |
| 10558 | HOXA1 | NM_005522.3 | 182 | gcgcggtcagcgccaacagttgc | 4997 | - | see also 3856 line | | |
| 10559 | HOXA10 | NM_018951.2 | 1202 | gagctcacagccaactttaattt | 5022 | - | transcription_regulator | - | |
| 10560 | HOXA3 | NM_030661.3 | 1013 | cggagccggtggctatctgaact | 5058 | - | transcription_regulator | - | |
| 10561 | HOXA4 | NM_002141.2 | 658 | gcgccgttaaccccagttataac | 5063 | - | transcription_regulator | transcription factor | - |
| 10562 | HOXA5 | NM_019102.2 | 599 | atgcgcaagctgcacataagtca | 5072 | - | see also 7708 line | | |
| 10563 | HOXB1 | NM_002144.2 | 116 | aggcggttgacagctatgcaagc | 5094 | - | transcription_regulator | PBX1a/HOXB1 heterodimer | - |
| 10564 | HOXB13 | NM_006361.2 | 354 | cagctcccgtgccttatggttac | 5098 | - | transcription_regulator | transcription factor | - |
| 10565 | HOXB2 | NM_002145.2 | 232 | aaggagtcgacattaattcctcc | 5108 | - | transcription_regulator | translation regulator | - |
| 10566 | HOXB3 | NM_002146.3 | 1501 | ccggcctcgccgtgtagagatgg | 5116 | - | transcription_regulator | transcription factor | - |
| 10567 | HOXB5 | NM_002147.2 | 200 | cggctcttacggctacaattaca | 5127 | - | see also 1371 line | | |
| 10568 | HOXB6 | NM_018952.3 | 420 | atgcggacccgctgagacattac | 5131 | - | transcription_regulator | - | |
| 10569 | HOXB7 | NM_004502.2 | 338 | tcgagccgagttccttcaacatg | 5133 | - | transcription_regulator | transcription factor | - |
| 10570 | HOXC10 | NM_017409.2 | 91 | atgacatgccctcgcaatgtaac | 5153 | - | transcription_regulator | RNA polymerase II transcription factor | |
| 10571 | HOXC11 | NM_014212.2 | 800 | aagcgctgcccttattcgaaatt | 5168 | - | transcription_regulator | RNA polymerase II transcription factor | - |
| 10572 | HOXC9 | NM_006897.1 | 504 | cagctacccggactacatgtacg | 5201 | - | see also 4829 line | | |
| 10573 | HOXD1 | NM_024501.1 | 912 | gcgcgatccgcacgaatttcagc | 5205 | - | transcription_regulator | transcription factor | - |
| 10574 | HOXD13 | NM_000523.2 | 867 | aggggatgtggctctaaatcagc | 5233 | - | transcription_regulator | transcription factor | brachydactyly、synpolydactyly、Synpolydactyly |
| 10575 | HOXD3 | NM_006898.4 | 225 | atgcagaaggctgcttactatga | 5235 | - | transcription_regulator | transcription factor | - |
| 10576 | HOXD8 | NM_019558.2 | 1028 | acggatacgataacttacagaga | 5243 | - | transcription_regulator | transcription factor | - |

Figure 46

| | | | | | | | | | |
|---|---|---|---|---|---|---|---|---|---|
| 10577 | HOZFP | NM_152995.3 | 1989 | ctgtaggaccctactcttgtaaa | 5308 | - | transcription_regulator | - | - |
| 10578 | HR | NM_005144.2 | 3794 | gggccatgagcgaatacacatgg | 5328 | - | transcription_regulator | - | alopecia universalis、atrichia |
| 10579 | HSF1 | NM_005526.1 | 1641 | tgggagagggctcctacttctcc | 5334 | - | transcription_regulator | heat shock transcription factor 1B | - |
| 10580 | HSF2 | NM_004506.2 | 203 | ttggatgagcaacgatttgcaaa | 5338 | - | see also 3063 line | | |
| 10581 | HSFY | NM_033108.1 | 214 | acggtcaatgattgaagaacatg | 5366 | - | transcription_regulator | - | - |
| 10582 | HSRNAFEV | NM_017521.1 | 791 | gggcggtcacggcgagttcaagc | 5385 | - | transcription_regulator | - | maxillary tumor、paraspinal neurogenic tumor、Ewing sarcoma、Ewing's sarcoma |
| 10583 | HTLF | NM_002158.2 | 1053 | gaggagttacggcaatgcatttc | 5408 | - | transcription_regulator | transcription factor | - |
| 10584 | ILF1 | NM_004514.2 | 600 | caggttcccgagcacaaacatca | 5424 | - | transcription_regulator | - | - |
| 10585 | IPF1 | NM_000209.1 | 635 | tggctgtcatgttgaacttgacc | 5448 | - | - | translation regulator | diabetes mellitus、pancreatic agenesis、diabetes、maturity-onset diabetes of the young |
| 10586 | IRF1 | NM_002198.1 | 263 | ggggctcatctggattaataaag | 5454 | - | see also 1402 line | | |
| 10587 | IRF2 | NM_002199.2 | 289 | tggatgcatgcggctagacatgg | 5460 | - | transcription_regulator | RNA polymerase II transcription factor | arthritis |
| 10588 | IRF3 | NM_001571.1 | 429 | gtggaggcagtacttctgatacc | 5479 | - | transcription_regulator | translation regulator | - |
| 10589 | IRF4 | NM_002460.1 | 1378 | ctgaggggctacgatttaccaga | 5494 | - | transcription_regulator | translation regulator | chronic myeloid leukemia、non-hodgkin lymphoma、leukemia、multiple myeloma |
| 10590 | IRF5 | NM_002200.3 | 412 | ccgggacttccgcctcatctacg | 5502 | - | transcription_regulator | - | - |
| 10591 | IRF6 | NM_006147.2 | 1450 | cagtagtggctcggatgatctac | 5524 | - | transcription_regulator | transcription factor | popliteal pterygium syndrome、Van der Woude syndrome |
| 10592 | IRF7 | NM_001572.2 | 1560 | gtggctccccacgctataccatc | 5532 | - | transcription_regulator | - | - |
| 10593 | IRX1 | NM_024337.1 | 1605 | gcgacagccagcgctgttgaagg | 5537 | - | - | - | - |
| 10594 | IRX3 | NM_024336.1 | 1173 | gagatcgatttggagaacttaga | 5540 | - | - | - | - |
| 10595 | IRX5 | NM_005853.2 | 1586 | aagactctccctatgaattgaag | 5558 | - | see also 4069 line | | |
| 10596 | ISGF3G | NM_006084.3 | 547 | tgggggagcagtccattcagaca | 5561 | - | transcription_regulator、signal_transduction | translation regulator | - |
| 10597 | ISL1 | NM_002202.1 | 1189 | gaggaccgggctctaattccact | 5587 | - | see also 1403 line | | |

EP 1 752 536 A1

Figure 46

| | | | | | | | | | |
|---|---|---|---|---|---|---|---|---|---|
| 10598 | ISL2 | NM_145805.1 | 110 | tgggtgctatgggtgatcattcc | 5591 | - | see also 9801 line | | |
| 10599 | JUN | NM_002228.2 | 1032 | ccgtccgagagcggaccttatgg | 5600 | - | see also 1424 line | | |
| 10600 | KIAA0669 | NM_014779.1 | 2779 | tcgggcaagtcgacgatactaga | 5637 | - | see also 5951 line | | |
| 10601 | KIAA0863 | NM_014913.1 | 2941 | gtggggtctacacgggaaatatg | 5705 | - | - | - | - |
| 10602 | KIAA1041 | NM_014947.1 | 1603 | cagcatcagacgttaacacatca | 5772 | - | see also 6138 line | | |
| 10603 | KIAA1443 | NM_020834.1 | 1564 | tggtttgactctcgattacctca | 5801 | - | - | - | - |
| 10604 | KIAA1474 | NM_020856.1 | 1963 | ccgtcgtatcattcatgtcaaac | 5831 | - | see also 7998 line | | |
| 10605 | KIAA1618 | NM_020954.1 | 1614 | ctggcatcagtactatgacatag | 5874 | - | - | - | - |
| 10606 | KLF12 | NM_007249.3 | 394 | ccggaggactcgttatctgtaga | 5918 | - | see also 5050 line | | |
| 10607 | KLF3 | NM_016531.2 | 901 | gtggaatccccggatactcaaag | 5964 | - | see also 6907 line | | |
| 10608 | KLF4 | NM_004235.3 | 1115 | gcgcacgtgccccaagatcaagc | 5975 | - | transcription_regulator | translation regulator | - |
| 10609 | L3MBTL | NM_015478.4 | 1971 | acgccctccgaagtatcgaaaga | 6014 | - | transcription_regulator | - | - |
| 10610 | LASS2 | NM_013384.3 | 503 | atggccgtcattgtggataaacc | 6023 | - | see also 5416 line | | |
| 10611 | LASS3 | NM_178842.2 | 777 | taggagtcggcggaatcaagaga | 6063 | - | transcription_regulator | - | - |
| 10612 | LASS4 | NM_024552.1 | 1292 | aaggacattcgtagtgatgtaga | 6095 | - | transcription_regulator | - | - |
| 10613 | LASS5 | NM_147190.1 | 957 | gagataatcgggccttatgcttc | 6125 | - | transcription_regulator | - | - |
| 10614 | LBX1 | NM_006562.3 | 564 | gcgctttctataccagaagtacc | 6134 | - | transcription_regulator | transcription factor | - |
| 10615 | LHX1 | NM_005568.2 | 1227 | gagagcgcggagcaaagtgtttc | 6138 | - | transcription_regulator | RNA polymerase II transcription factor | - |
| 10616 | LHX5 | NM_022363.2 | 1259 | cagaaccgacggtccaaagaacg | 6177 | - | transcription_regulator | transcription factor | - |
| 10617 | LHX6 | NM_014368.2 | 80 | gcgaccacccgctgtcttgaagg | 6179 | - | transcription_regulator | - | |
| 10618 | LHX9 | NM_020204.2 | 666 | aagatctcggacaggtactatct | 6184 | - | transcription_regulator | - | |
| 10619 | LMO4 | NM_006769.2 | 1083 | gagggcgcaaggcaatgtgtatc | 6206 | - | see also 4775 line | | |
| 10620 | LMX1A | NM_177398.1 | 517 | aggccatcgctcccaatgagttt | 6216 | - | transcription_regulator | - | |
| 10621 | LMX1B | NM_002316.1 | 381 | acgcaagggcgacgaattcgtgc | 6226 | - | transcription_regulator | translation regulator | nail patella syndrome |
| 10622 | LOC51270 | NM_016521.2 | 493 | gcgagctggtcgccaagttcaga | 6229 | - | transcription_regulator, cell_cycle | - | - |
| 10623 | LZTS1 | NM_021020.1 | 288 | gggcaagagcgaagacttcttct | 6237 | - | see also 8067 line | | |

Figure 46

| 10624 | MADH3 | NM_005902.1 | 390 | gtgtgagttcgccttcaatatga | 6252 | - | transcription_regulator, signal_transduction | Smad3/Sp-1 heterodimer | - |
|---|---|---|---|---|---|---|---|---|---|
| 10625 | MADH4 | NM_005359.3 | 1711 | atgaccttcgtcgcttatgcata | 6303 | - | see also 3736 line | | |
| 10626 | MAFG | NM_002359.1 | 641 | cagcgtcatcacaatagtaaagt | 6312 | - | see also 1524 line | | |
| 10627 | MAX | NM_197957.1 | 208 | aagagcaaccgaggtttcaatct | 6313 | - | - | transcription factor | - |
| 10628 | MBD1 | NM_002384.1 | 223 | tggacgctcagacacctattacc | 6322 | - | transcription_regulator | - | - |
| 10629 | MDS1 | NM_004991.1 | 546 | ttgaacttcgagagtcaaatatg | 6351 | - | transcription_regulator | - | myelodysplasia syndrome |
| 10630 | MEF2A | NM_005587.1 | 648 | cagaaccaactcggatattgttg | 6362 | - | transcription_regulator | translation regulator | atherosclerosis、coronary artery disease |
| 10631 | MEF2C | NM_002397.2 | 428 | gaggattatggatgaacgtaaca | 6391 | - | see also 1542 line | | |
| 10632 | MEIS1 | NM_002398.1 | 427 | tagccgtgttcgccaaacagatt | 6416 | - | see also 1545 line | | |
| 10633 | MEIS2 | NM_002399.2 | 1408 | atgattgaccagtcaaatcgagc | 6447 | - | see also 1556 line | | |
| 10634 | MEOX1 | NM_004527.2 | 410 | aggaggcccaggcgatgactacg | 6463 | - | transcription_regulator | - | - |
| 10635 | MEOX2 | NM_005924.2 | 1019 | cagcaaacaggggactctatagc | 6472 | - | transcription_regulator | transcription factor | atherosclerosis |
| 10636 | MLL | NM_005933.1 | 10453 | cagacggtagacgctcctaatag | 6736 | - | transcription_regulator | zinc binding | leukemia |
| 10637 | MLL2 | NM_003482.1 | 14807 | aggccacgcgcatctattggagc | 6864 | - | transcription_regulator | - | leukemia |
| 10638 | MLL4 | NM_014727.1 | 8124 | gcgctgccgtcggttccttaact | 6955 | - | transcription_regulator | zinc binding | - |
| 10639 | MLLT10 | NM_004641.1 | 732 | tggtgccgataatgtccaatact | 6974 | - | see also 3155 line | | |
| 10640 | MLLT2 | NM_005935.1 | 2209 | aggcaaaccgttggaaccaaaca | 7049 | - | transcription_regulator | transcription factor | leukemia |
| 10641 | MLLT7 | NM_005938.1 | 555 | gagtggatggtccgtactgtacc | 7078 | - | see also 4146 line | | |
| 10642 | MNT | NM_020310.1 | 1061 | gcggctggcacgtgagaagattg | 7088 | - | transcription_regulator, cell_cycle | transcription factor | - |
| 10643 | MSC | NM_005098.2 | 814 | atgtggaaccaccgcttaaatcg | 7093 | - | transcription_regulator | transcription factor | - |
| 10644 | MSX1 | NM_002448.1 | 711 | ctgcaccctccgcaaacacaaga | 7097 | - | transcription_regulator | transcription factor | hypodontia withorofacial cleft、hypodontia、Witkop syndrome |
| 10645 | MSX2 | NM_002449.3 | 749 | ctgcaggcagcgtccatatatgg | 7105 | - | transcription_regulator | transcription factor | craniosynostosis |
| 10646 | MTA1 | NM_004689.2 | 1329 | caggaccaaaccgcagtaacatg | 7122 | - | see also 3218 line | | |
| 10647 | MTA2 | NM_004739.2 | 1821 | acggccttatgctcctatcaatg | 7156 | - | see also 3269 line | | |

Figure 46

| No. | Gene | Accession | | Sequence | | | Description | Factor | Disease |
|---|---|---|---|---|---|---|---|---|---|
| 10648 | MYBL2 | NM_002466.2 | 975 | caggaggcccatcgtacagatct | 7221 | - | transcription_regulator, apoptosis, cell_cycle | transcription factor | neuroblastoma |
| 10649 | MYC | NM_002467.2 | 1673 | ttgaacagctacggaactctgt | 7240 | - | see also 1586 line | | |
| 10650 | MYCL2 | NM_005377.1 | 1072 | aaggccacggaatacttacatga | 7251 | - | - | transcription factor | |
| 10651 | MYCN | NM_005378.3 | 1363 | gtgccggagttggtaaagaatga | 7263 | - | transcription_regulator | transcription factor | rhabdomyosarcoma, lung cancer, neuroblastoma, retinoblastoma, osteosarcoma, astrocytic tumors |
| 10652 | MYF6 | NM_002469.1 | 455 | gcggagcgccatcagctatattg | 7265 | - | transcription_regulator | Myf6/E47 | |
| 10653 | MYNN | NM_018657.2 | 943 | aagaacactctatgtctaatata | 7294 | - | see also 7538 line | | |
| 10654 | MYOG | NM_002479.2 | 86 | caggaacccgcttcatgatgg | 7324 | - | transcription_regulator | Myf4/E47 | |
| 10655 | MYST2 | NM_007067.3 | 642 | ccgtcgcttccatgaaagctaca | 7334 | - | transcription_regulator | | |
| 10656 | MYT1 | NM_004535.1 | 2015 | cagctcgaggtccctccatatg | 7366 | - | see also 3089 line | | |
| 10657 | NFAT5 | NM_006599.2 | 2353 | tggactgcgtaggggatattgaaa | 7432 | - | see also 4634 line | | |
| 10658 | NFATC1 | NM_006162.3 | 2049 | ctgtgccggaatcctgaaactca | 7526 | - | transcription_regulator | | |
| 10659 | NFATC2 | NM_012340.2 | 1809 | aaggccgagcacggacattggaaga | 7559 | - | transcription_regulator | | |
| 10660 | NFATC3 | NM_004555.2 | 2923 | cagccttgtctcagttacaacc | 7644 | - | transcription_regulator | | |
| 10661 | NFE2 | NM_006163.1 | 698 | cagaatcgactcaggattatcc | 7687 | - | see also 4264 line | | |
| 10662 | NFE2L1 | NM_003204.1 | 725 | gggcccagttctgcctatactca | 7703 | - | see also 2167 line | | |
| 10663 | NFE2L2 | NM_006164.2 | 624 | caggtagccctgttgatttaga | 7741 | - | see also 4266 line | | |
| 10664 | NFE2L3 | NM_004289.4 | 78 | ccgcgtagacctagatctttacc | 7769 | - | transcription_regulator | | |
| 10665 | NFIA | NM_005595.1 | 683 | ctgttaaggaactcgatttatat | 7848 | - | see also 3883 line | | |
| 10666 | NFIB | NM_005596.1 | 966 | atgaactcggggtcaatcttca | 7882 | - | see also 3886 line | | |
| 10667 | NFIC | NM_005597.1 | 854 | gtgggagcaagcggcacaaatcg | 7891 | - | transcription_regulator | Nuclear factor 1 C-type-7 | |
| 10668 | NFIL3 | NM_005384.1 | 868 | aagcaagagccgatggaattaga | 7916 | - | transcription_regulator | | |
| 10669 | NFKB1 | NM_003998.2 | 1101 | gttggtgcggctcatgtttacagc | 7954 | - | see also 2724 line | | |
| 10670 | NFKB2 | NM_002502.2 | 307 | gagaggcttccgatttcgatatg | 8010 | - | transcription_regulator, signal_transduction | transcription factor | rheumatoid arthritis, atherosclerosis |
| 10671 | NFX1 | NM_002504.3 | 2135 | cggttgttgtgacggccataaatg | 8070 | - | see also 1613 line | | |

Figure 46

| 10672 | NFYA | NM_002505.3 | 668 | agggcagaccatcgtctatcaac | 8110 | - | transcription_regulator | - | - |
|---|---|---|---|---|---|---|---|---|---|
| 10673 | NFYB | NM_006166.2 | 665 | atggtttacacaacatcatatca | 8122 | - | see also 4269 line | | |
| 10674 | NFYC | NM_014223.2 | 576 | gaggtgcgccagtctgtaactcc | 8139 | - | transcription_regulator | NF-YA/NF-YB/NF-YC trimeric complex | - |
| 10675 | NKX2-2 | NM_002509.2 | 683 | acgagtcaccggacaatgacaag | 8148 | - | transcription_regulator | RNA polymerase II transcription factor | - |
| 10676 | NKX2-3 | NM_145285.1 | 370 | aagacgagggcgagaaattgtcc | 8153 | - | transcription_regulator | - | - |
| 10677 | NKX2-8 | NM_014360.2 | 223 | gtgcgcagccttctagatttacc | 8160 | - | transcription_regulator | - | - |
| 10678 | NKX3-1 | NM_006167.2 | 336 | aaggcacttggggtcttatctgt | 8163 | - | transcription_regulator | transcription factor | - |
| 10679 | NKX6-1 | NM_006168.1 | 803 | gggctcgtttggcctattcgttg | 8173 | - | transcription_regulator | transcription factor | - |
| 10680 | NME2 | NM_002512.1 | 482 | aagaactggttgactacaagtct | 8177 | - | kinase_related、transcription_regulator、cell_cycle | translation regulator | neuroblastoma |
| 10681 | NPAS1 | NM_002517.2 | 699 | cagcagcgtcttcgactacattc | 8182 | - | transcription_regulator、signal_transduction | transcription factor | - |
| 10682 | NPAS2 | NM_002518.2 | 2204 | gcgctcccgccaagtctgaatct | 8208 | - | see also 1618 line | | |
| 10683 | NR0B1 | NM_000475.2 | 646 | gtgcccacgagcacaaatcaagc | 8219 | - | receptor_acitivity、transcription_regulator | steroid hormone receptor | Addison disease、adrenal hyperplasia、dosage-sensitive sex reversal(DSS)、DSS-AHC critical region on the X chromosome gene(DAX1) |
| 10684 | NR0B2 | NM_021969.1 | 460 | ctgcggtgcagtggcttcaatgc | 8230 | - | transcription_regulator、receptor_acitivity | transcription co-repressor | obesity |
| 10685 | NR1D1 | NM_021724.1 | 1640 | ctgcccccaatgacaacaacacc | 8234 | - | transcription_regulator、receptor_acitivity | transcription co-repressor | - |
| 10686 | NR1H2 | NM_007121.1 | 1215 | cagagtgtatcaccttcttgaag | 8245 | - | transcription_regulator、receptor_acitivity | steroid hormone receptor | atherosclerosis |
| 10687 | NR1H4 | NM_005123.1 | 481 | tggaaccatactcgcaatacagc | 8254 | - | receptor_acitivity、transcription_regulator | translation regulator | - |
| 10688 | NR1I2 | NM_003889.2 | 2570 | ctgacatgtcaacctacatgttc | 8287 | - | transcription_regulator、receptor_acitivity、signal_transduction | - | - |
| 10689 | NR1I3 | NM_005122.2 | 815 | cggggcctcttcgctacacaattg | 8301 | - | receptor_acitivity、transcription_regulator、signal_transduction | translation regulator | - |

445

Figure 46

| 10690 | NR2C1 | NM_003297.1 | 747 | aggtcaacaggactgttagattc | 8330 | - | see also 2229 line | | |
|---|---|---|---|---|---|---|---|---|---|
| 10691 | NR2C2 | NM_003298.2 | 488 | cagcgccaagcaactcatattca | 8375 | - | transcription_regulator、receptor_acitivity | transcription co-activator | - |
| 10692 | NR2E1 | NM_003269.2 | 1515 | gaggtggtggctcgatttagaca | 8439 | - | see also 2215 line | | |
| 10693 | NR2E3 | NM_014249.2 | 953 | gaggcgtggagtgaactctttct | 8456 | - | transcription_regulator、receptor_acitivity、signal_transduction | - | - |
| 10694 | NR2F1 | NM_005654.3 | 422 | aggagcgtccgcaggaacttaac | 8463 | - | receptor_acitivity、transcription_regulator、signal_transduction | translation regulator | - |
| 10695 | NR2F2 | NM_021005.2 | 1803 | tggccgtatatggcaattcaata | 8479 | - | see also 8063 line | | |
| 10696 | NR3C1 | NM_000176.1 | 2043 | ctgcatgtacgaccaatgtaaac | 8524 | - | see also 144 line | | |
| 10697 | NR3C2 | NM_000901.1 | 1932 | tgggtatccggtcttagaataca | 8587 | - | transcription_regulator、receptor_acitivity、signal_transduction | Smad3/Sp-1 heterodimer | pseudohypoaldosteronism |
| 10698 | NR4A1 | NM_002135.3 | 798 | tcgcccagccagacttacgaagg | 8614 | - | transcription_regulator、receptor_acitivity、signal_transduction | - | atherosclerosis |
| 10699 | NR4A2 | NM_006186.2 | 883 | ctggcacccggtgtctagttgc | 8634 | - | see also 4276 line | | |
| 10700 | NR5A1 | NM_004959.3 | 451 | gagggggtggccggaacaagtttg | 8693 | - | see also 3449 line | | |
| 10701 | NR6A1 | NM_001489.2 | 1500 | gtgcttacctgagattcgatata | 8721 | - | see also 1026 line | | |
| 10702 | NT5C | NM_014595.1 | 347 | cggacacgcaggtcttcatctgc | 8724 | - | - | - | - |
| 10703 | NY-BR-1 | NM_052997.1 | 308 | tagattctggtgccgatataaat | 8729 | - | transcription_regulator | - | - |
| 10704 | ODAG | NM_021167.2 | 752 | ttgatcccgcctcctatatcata | 8751 | - | transcription_regulator | - | - |
| 10705 | ONECUT2 | NM_004852.1 | 1160 | ccgtggagtaaactcaaatctgg | 8774 | - | see also 3372 line | | |
| 10706 | OTX1 | NM_014562.2 | 249 | atgatgtcttacctcaaacaacc | 8783 | - | see also 5754 line | | |
| 10707 | P1P373C6 | NM_019110.3 | 1688 | gaggctcccgtgtcttataaatg | 8812 | - | transcription_regulator | molecular_function unknown | - |
| 10708 | p66alpha | NM_017660.2 | 531 | aagttgcggcagagtcaaataca | 8816 | - | transcription_regulator | - | - |
| 10709 | P66beta | NM_020699.1 | 1254 | ctgcgggttgaaccctttgtatg | 8839 | - | see also 7924 line | | |
| 10710 | PAX4 | NM_006193.1 | 314 | acggatccttaaggtatctaatg | 8894 | - | transcription_regulator | transcription factor | - |
| 10711 | PAX7 | NM_002584.1 | 614 | cggcacggtaccgagaatgatgc | 8939 | - | transcription_regulator、apoptosis | - | rhabdomyosarcoma、alveolar rhabdomyosarcoma |
| 10712 | PBX1 | NM_002585.1 | 1286 | tcgcagccagtcagatgtacagt | 8969 | - | see also 1663 line | | |

446

Figure 46

| | | | | | | | | | |
|---|---|---|---|---|---|---|---|---|---|
| 10713 | PBX2 | NM_002586.3 | 1414 | aggggggctatggggataacctcg | 8974 | - | see also 1664 line | | |
| 10714 | PBX3 | NM_006195.2 | 945 | cagtgtcacaggtatccaattgg | 8991 | - | see also 4281 line | | |
| 10715 | PBX4 | NM_025245.1 | 392 | ctgtcccagatccgacagattta | 9003 | - | transcription_regulator | - | - |
| 10716 | PEG3 | NM_006210.1 | 4290 | atgagtacgggtcctcctatact | 9108 | - | - | - | - |
| 10717 | PGBD1 | NM_032507.2 | 2170 | tgggttaggtggaaatctagtga | 9149 | - | receptor_acitivity、transcription_regulator | - | - |
| 10718 | PGR | NM_000926.2 | 1591 | ctgaagtttcggccatacctatc | 9172 | - | see also 603 line | | |
| 10719 | PHF2 | NM_005392.2 | 2194 | tggactcggcagcgtacaagagt | 9255 | - | see also 3763 line | | |
| 10720 | PHTF1 | NM_006608.1 | 81 | gagcctacgatcagcagatatgg | 9273 | - | transcription_regulator | - | - |
| 10721 | PILB | NM_012228.2 | 337 | gcgacagtccactcttcagttct | 9344 | - | - | transcription factor | - |
| 10722 | PITX2 | NM_000325.4 | 882 | gcgccgaggacccgtctaagaag | 9349 | - | - | - | iridogoniodysgenesis(-)、iris hypoplasia with glaucoma、Rieger syndrome、Peters anomaly |
| 10723 | PKNOX1 | NM_004571.3 | 952 | atgatggttcatctaagaacaag | 9387 | - | see also 3110 line | | |
| 10724 | PKNOX2 | NM_022062.1 | 198 | ctgacaagcgagctgtatacagg | 9398 | - | see also 8258 line | | |
| 10725 | PLAG1 | NM_002655.1 | 1003 | aagtcgtctggtggggttaaaga | 9422 | - | see also 1736 line | | |
| 10726 | PLAGL2 | NM_002657.2 | 597 | gcggtaagaattacaatacgaag | 9456 | - | see also 1738 line | | |
| 10727 | PLU-1 | NM_006618.2 | 2868 | ctgtttaccgaactaatcagtgt | 9501 | - | see also 4679 line | | |
| 10728 | POLR2I | NM_006233.3 | 145 | acgcgtgccggaactgtgattac | 9602 | - | transcription_regulator | zinc binding | - |
| 10729 | POLR3K | NM_016310.2 | 120 | gtgcccctacgtgcacaacatca | 9606 | - | transcription_regulator | transcription factor | - |
| 10730 | POU1F1 | NM_000306.1 | 563 | aggatacacccagacaaatgttg | 9616 | - | transcription_regulator | Proto-oncogene C-JUN | pituitary hormone deficiency、pituitary dwarfism |
| 10731 | POU2F1 | NM_002697.1 | 987 | gggctcgctatggggaaactata | 9639 | - | see also 1755 line | | |
| 10732 | POU2F3 | NM_014352.1 | 221 | atgtccggaaaccaaatgtctgg | 9672 | - | transcription_regulator | - | - |
| 10733 | POU3F4 | NM_000307.1 | 385 | cggcaccgaacccgtctatcacg | 9684 | - | see also 264 line | | |
| 10734 | POU4F3 | NM_002700.1 | 858 | gcgttcactcgaggcctatttcg | 9711 | - | see also 1759 line | | |
| 10735 | POU5F1 | NM_002701.1 | 127 | gggccacacgtaggttcttgaat | 9713 | - | transcription_regulator | octamer factor-3 | - |
| 10736 | PPARA | NM_005036.2 | 499 | gagcattgaacatcgaatgtaga | 9728 | - | receptor_acitivity、transcription_regulator | PPARalpha/RXRalpha | - |
| 10737 | PPARD | NM_006238.2 | 1186 | caggttacccttctcaagtatgg | 9750 | - | transcription_regulator、receptor_acitivity | - | colorectal cancer、colorectal cancers |
| 10738 | PRDM1 | NM_001198.2 | 1344 | ccgggactcctacgcttacttga | 9826 | - | see also 774 line | | |
| 10739 | PRDM16 | NM_022114.1 | 3599 | acgaaggcggtctgttagctttg | 9878 | - | see also 8274 line | | |

Figure 46

| | | | | | | | | | |
|---|---|---|---|---|---|---|---|---|---|
| 10740 | PRDM2 | NM_012231.2 | 5752 | aagaacagaccggttcaatataa | 9991 | - | transcription_regulator | - | melanoma |
| 10741 | PRRX1 | NM_006902.2 | 1323 | aggaataggacaaccttcaatag | 9999 | - | see also 4838 line | | |
| 10742 | RARA | NM_000964.1 | 241 | cagcttccagttagtggatatag | 10011 | - | transcription_regulator, receptor_acitivity, signal_transduction | Smad3/Sp-1 heterodimer | leukemia |
| 10743 | RARB | NM_000965.2 | 1310 | acggccttaccctaaatcgaact | 10038 | - | - | - | breast cancer、lung cancer、Parkinson disease、schizophrenia |
| 10744 | RARG | NM_000966.3 | 1644 | aggcccgatgcctcccttaatcc | 10060 | - | - | - | - |
| 10745 | RAX | NM_013435.1 | 297 | aggccatcctggggtttaccaag | 10062 | - | transcription_regulator | RNA polymerase II transcription factor | - |
| 10746 | RB1 | NM_000321.1 | 2108 | tagcctatctccggctaaataca | 10121 | - | see also 268 line | | |
| 10747 | RBBP1 | NM_002892.2 | 1311 | ctgccgttcggcaaatattcagt | 10190 | - | transcription_regulator | - | - |
| 10748 | RBBP2 | NM_005056.1 | 3322 | ctgggcggacgtttcttaagaag | 10331 | - | transcription_regulator | transcription factor | - |
| 10749 | REL | NM_002908.1 | 1906 | gagtcgggaccatcaaacagtac | 10420 | - | see also 1940 line | | |
| 10750 | RELA | NM_021975.1 | 1482 | gagtaccctgaggctataactcg | 10443 | - | see also 8236 line | | |
| 10751 | RELB | NM_006509.2 | 994 | gagcccgtctatgacaagaaatc | 10446 | - | see also 4552 line | | |
| 10752 | RERE | NM_012102.2 | 1191 | atggtactaccgtcaatctgagg | 10454 | - | transcription_regulator | - | - |
| 10753 | RFX5 | NM_000449.2 | 493 | atgatgcctatcggaagtactgt | 10497 | - | see also 353 line | | |
| 10754 | RLF | NM_012421.1 | 3986 | aaggcttaattcgccattacaga | 10639 | - | transcription_regulator | - | - |
| 10755 | RNF110 | NM_007144.2 | 636 | cagcctctccatcgaattctacg | 10701 | - | transcription_regulator | transcription factor | - |
| 10756 | RORA | NM_002943.2 | 1369 | gtgtactttgatgggaagtatgc | 10746 | - | see also 1984 line | | |
| 10757 | RUNX1 | NM_001754.2 | 1056 | aagacatcggcagaaactagatg | 10842 | - | transcription_regulator | transcription factor | leukemia、familial platelet disorder with associated myeloid malignancy(FPDMM syndrome) |
| 10758 | RXRB | NM_021976.3 | 1496 | ctgcctgagggcaatcattctgt | 10884 | - | receptor_acitivity, transcription_regulator | retinoid X receptor beta-1 | Parkinson disease、schizophrenia |
| 10759 | RXRG | NM_006917.2 | 633 | aagcactacggggtatacagttg | 10899 | - | transcription_regulator, receptor_acitivity | Retinoic acid receptor RXR-alpha | Parkinson disease、schizophrenia |
| 10760 | SATB1 | NM_002971.2 | 1376 | gtgggtacgcgatgaactgaaac | 10948 | - | transcription_regulator | transcription factor | - |
| 10761 | SATB2 | NM_015265.1 | 834 | cagcgtccaatgcatttaccaaa | 10985 | - | see also 6294 line | | |
| 10762 | SBLF | NM_006873.2 | 1403 | aagcgagatgaatcctattatga | 11043 | - | - | - | - |
| 10763 | SCAND2 | NM_022050.2 | 450 | cagactccttcagtacaagatct | 11071 | - | transcription_regulator | - | - |

EP 1 752 536 A1

448

Figure 46

| 10764 | SCMH1 | NM_012236.1 | 1190 | cggacacccaagaccctaatttc | 11088 | - | transcription_regulator | - | - |
|---|---|---|---|---|---|---|---|---|---|
| 10765 | SCML1 | NM_006746.2 | 845 | ctgatggtgcaacgtatggttct | 11108 | - | transcription_regulator | - | - |
| 10766 | SCML2 | NM_006089.1 | 1098 | gagaccgtggcatgttatataaa | 11141 | - | - | - | - |
| 10767 | SCRT1 | NM_031309.2 | 450 | cggcggacgccttcttcatcacc | 11161 | - | transcription_regulator | - | small cell lung cancer、non-small-cell lung cancer |
| 10768 | SDCCAG33 | NM_005786.3 | 4342 | cagtcaaactgcaccttagtaag | 11196 | - | transcription_regulator | - | - |
| 10769 | SIM1 | NM_005068.2 | 1176 | ctgtatcgtcagcgtcaactatg | 11218 | - | see also 3536 line | | |
| 10770 | SIX1 | NM_005982.1 | 533 | gtggctgaaggcgcattacgtgg | 11276 | - | see also 4179 line | | |
| 10771 | SIX2 | NM_016932.3 | 394 | gagcaccttcacaagaatgaaag | 11282 | - | transcription_regulator | transcription factor | - |
| 10772 | SIX3 | NM_005413.1 | 1359 | gcgactcggaatgtgatgtatga | 11290 | - | see also 3779 line | | |
| 10773 | SIX6 | NM_007374.1 | 296 | ccgcgagctctatcatatcctgg | 11343 | - | transcription_regulator | transcription factor | - |
| 10774 | SLC26A3 | NM_000111.1 | 440 | gtggccgtactacaaggtttagc | 11360 | - | transcription_regulator | transcription factor | congenital chloride diarrhea |
| 10775 | SLC2A4RG | NM_020062.3 | 585 | ccgaggcagcccactttctgttt | 11443 | - | transcription_regulator | - | - |
| 10776 | SLC30A9 | NM_006345.2 | 1272 | aagtcgtgatcctagtacaaatg | 11479 | - | see also 4439 line | | |
| 10777 | SLC38A2 | NM_018976.3 | 1573 | tggcgtcatagtctcattacagt | 11550 | - | see also 7623 line | | |
| 10778 | SLC38A6 | NM_153811.1 | 324 | atggctaataccggtgtctttgg | 11560 | - | - | - | - |
| 10779 | SLC4A10 | NM_022058.2 | 2394 | tggtttgttacgcctttaggtcc | 11652 | - | see also 8254 line | | |
| 10780 | SMARCA4 | NM_003072.2 | 3623 | ttgcgtatcgcggctttaaatac | 11694 | - | transcription_regulator | translation regulator | - |
| 10781 | SNAPC2 | NM_003083.1 | 246 | ccgggaggccattcagaaagtgc | 11704 | - | transcription_regulator | transcription factor | - |
| 10782 | SNAPC4 | NM_003086.1 | 3202 | ccgggtcccaccgtcttaaatgt | 11730 | - | see also 2079 line | | |
| 10783 | SNAPC5 | NM_006049.1 | 95 | aagaattagccctccaatcaatg | 11737 | - | transcription_regulator | transcription factor | - |
| 10784 | SNFT | NM_018664.1 | 536 | ctgctctgccctatgaactttgt | 11745 | - | transcription_regulator | - | - |
| 10785 | SOX13 | NM_005686.1 | 305 | tggacttcaaccgaaatttgaaa | 11750 | - | transcription_regulator | transcription factor | diabetes |
| 10786 | SOX14 | NM_004189.2 | 728 | ctggcatagaccccttattcgtca | 11756 | - | transcription_regulator | transcription factor | - |
| 10787 | SOX2 | NM_003106.2 | 1060 | cagctcgcagacctacatgaacg | 11757 | - | transcription_regulator | - | anophthalmia |

Figure 46

| | | | | | | | | | |
|---|---|---|---|---|---|---|---|---|---|
| 10788 | SOX5 | NM_006940.4 | 1397 | ctgtcccagcagcgttagctagt | 11777 | - | transcription_regulator | - | |
| 10789 | SOX6 | NM_033326.1 | 376 | gagtcgggaccgtgagataatga | 11795 | - | see also 9400 line | | |
| 10790 | SOX8 | NM_014587.2 | 1271 | ctgcaggcctccagctactatgg | 11839 | - | transcription_regulator | RNA polymerase II transcription factor | - |
| 10791 | SP140 | NM_007237.2 | 575 | tagacccagattactaccatatg | 11854 | - | transcription_regulator | transcription factor | - |
| 10792 | SPBPBP | NM_006692.1 | 858 | tgggcactttactaaagctttac | 11876 | - | - | - | - |
| 10793 | SPI1 | NM_003120.1 | 280 | acgccaaacgcacgagtattacc | 11885 | - | transcription_regulator | hematopoietic-associated factor 1A complex | - |
| 10794 | SPIC | NM_152323.1 | 48 | aagctgggtcaagcatttgaaga | 11891 | - | - | - | - |
| 10795 | SREBF1 | NM_004176.2 | 2169 | acgcagccctggtctaccataag | 11916 | - | transcription_regulator | Sterol regulatory element binding proteins | - |
| 10796 | SRF | NM_003131.1 | 983 | gagaccggcaaggcactgattca | 11927 | - | see also 2113 line | | |
| 10797 | ST18 | NM_014682.1 | 712 | ctgcgtactcgctctaaaggaac | 11936 | - | transcription_regulator | - | - |
| 10798 | STAT1 | NM_007315.2 | 721 | caggctcagtcggggaatattca | 12016 | - | transcription_regulator、cell_cycle、kinase_related | - | STAT1 deficiency、atypical mycobacteriosis |
| 10799 | STAT2 | NM_005419.2 | 731 | aagcactgctaggccgattaact | 12065 | - | transcription_regulator | STAT1/STAT2 complex | - |
| 10800 | STAT3 | NM_003150.2 | 2121 | cagtccgtggaaccatacacaaa | 12123 | - | - | - | - |
| 10801 | STAT4 | NM_003151.2 | 1160 | aagagtcaccttcttgatctaca | 12158 | - | see also 2128 line | | |
| 10802 | STAT5A | NM_003152.2 | 962 | ctgcatccggcacattctgtaca | 12211 | - | transcription_regulator | translation regulator | - |
| 10803 | STAT5B | NM_012448.2 | 547 | ctgcatccgccatatattgtaca | 12231 | - | see also 5336 line | | |
| 10804 | STAT6 | NM_003153.3 | 514 | cagcacccttgagagcatatatc | 12258 | - | transcription_regulator | translation regulator | rheumatoid arthritis |
| 10805 | SUPT4H1 | NM_003168.1 | 121 | gtgttcgctggtcaagactatag | 12273 | - | transcription_regulator | transcription factor | - |
| 10806 | SUPT5H | NM_003169.2 | 521 | ttgaagcctccaatatcgataat | 12288 | - | see also 2141 line | | |
| 10807 | SUPT6H | NM_003170.2 | 2322 | ctgtagtcgaaagctctacaatt | 12346 | - | see also 2142 line | | |
| 10808 | T | NM_003181.2 | 1020 | ggggtccacagcgcatgatcacc | 12402 | - | transcription_regulator、signal_transduction | translation regulator | - |
| 10809 | TADA2L | NM_001488.2 | 540 | aagcgatcatacttatgaaataa | 12407 | - | transcription_regulator、cell_cycle | | |

EP 1 752 536 A1

450

Figure 46

| | | | | | | | transcription_regulator | | |
|---|---|---|---|---|---|---|---|---|---|
| 10810 | TADA3L | NM_006354.2 | 1233 | ctgaccgaactggacactaaaga | 12451 | - | , cell_cycle | - | - |
| 10811 | TAF10 | NM_006284.2 | 595 | ccgcaagtacactctaaccatgg | 12458 | - | transcription_regulator | RNA polymerase II transcription factor | - |
| 10812 | TAF13 | NM_005645.2 | 272 | ttgaagatatcgtcttcttgatt | 12469 | - | - | protein binding | |
| 10813 | TAF1B | NM_005680.1 | 485 | aaggaaacctacggtattagaag | 12479 | - | transcription_regulator | transcription factor | |
| 10814 | TAF4 | NM_003185.2 | 1912 | aggttataccgagaacttaattc | 12533 | - | transcription_regulator | transcription co-activator | - |
| 10815 | TAF5 | NM_006951.2 | 853 | atgacctacgagtattatctagt | 12573 | - | see also 4867 line | | |
| 10816 | TAF5L | NM_014409.2 | 1023 | tagacgtgtcccgcatccatttg | 12649 | - | see also 5664 line | | |
| 10817 | TARDBP | NM_007375.2 | 618 | atgtcacagcgacatatgataga | 12665 | - | - | transcription factor | - |
| 10818 | TBR1 | NM_006593.2 | 1197 | tggatgcgccaagaaatctcttt | 12695 | - | see also 4618 line | | |
| 10819 | TBX1 | NM_005992.1 | 578 | tggatcccatggccgactatatg | 12703 | - | transcription_regulator | - | - |
| 10820 | TBX19 | NM_005149.1 | 244 | aagaatggcagacggatgtttcc | 12727 | - | see also 3614 line | | |
| 10821 | TBX2 | NM_005994.2 | 497 | ctgacgattgccgctataagttc | 12741 | - | transcription_regulator | transcription factor | - |
| 10822 | TBX20 | NM_020417.1 | 648 | ctggatcaacatggccatataat | 12752 | - | transcription_regulator | - | - |
| 10823 | TBX21 | NM_013351.1 | 644 | ctgttgtggtccaagtttaatca | 12759 | - | transcription_regulator | translation regulator | - |
| 10824 | TBX22 | NM_016954.2 | 477 | acgctataggtacgtctatcaca | 12773 | - | see also 6980 line | | |
| 10825 | TBX3 | NM_005996.2 | 640 | aagtcgggaaggcgaatgtttcc | 12815 | - | transcription_regulator | - | ulnar mammary syndrome |
| 10826 | TBX4 | NM_018488.2 | 1615 | ttgtcccgagaatcttccttaca | 12852 | - | transcription_regulator | transcription factor | - |
| 10827 | TBX5 | NM_000192.3 | 990 | ctgccgacgatcacagatacaaa | 12862 | - | transcription_regulator | - | Holt-Oram syndrome |
| 10828 | TBX6 | NM_004608.2 | 1196 | ctgcctgaccgtgtctacattca | 12889 | - | transcription_regulator | - | - |
| 10829 | TCEA1 | NM_006756.1 | 163 | acgcggctggagcattggatttg | 12898 | - | transcription_regulator | general RNA polymerase II transcription factor | - |
| 10830 | TCEA2 | NM_003195.4 | 1060 | gtgatgccgtgcgcaacaagtgc | 12911 | - | transcription_regulator | - | - |
| 10831 | TCEAL1 | NM_004780.1 | 501 | ggggacatacatggcagaaattt | 12915 | - | transcription_regulator | - | - |

Figure 46

| | | | | | | | | | |
|---|---|---|---|---|---|---|---|---|---|
| 10832 | TCF1 | NM_000545.3 | 728 | gtgcaataggcgcggaatgcatcc | 12917 | - | transcription_regulator | transcription factor-7-like-2-E | atherosclerosis, diabetes mellitus, diabetes |
| 10833 | TCF19 | NM_007109.1 | 269 | gaggtcacaggctggaattgagt | 12919 | - | - | - | - |
| 10834 | TCF2 | NM_000458.1 | 913 | ccgcgtcccagcaaatcttgtac | 12928 | - | see also 355 line | - | - |
| 10835 | TCF3 | NM_003200.1 | 1191 | cgggcctgcagtagaagatagaag | 12954 | - | transcription_regulator | MyoD/E47 | - |
| 10836 | TCF7L1 | NM_031283.1 | 399 | caggacagcgcggttcttttaaagg | 12957 | - | transcription_regulator, signal_transduction | - | - |
| 10837 | TCF8 | NM_030751.2 | 554 | gaggctataaacgctttacctct | 12971 | - | see also 8924 line | - | - |
| 10838 | TCFL4 | NM_170607.2 | 878 | ctggatcgaggagcacgtgaagc | 13051 | - | see also 10023 line | - | - |
| 10839 | TEAD2 | NM_003598.1 | 940 | tggcctccgagagctatatgatc | 13062 | - | transcription_regulator | translation regulator | - |
| 10840 | TEAD3 | NM_003214.1 | 408 | ttgcacgctatattaaactgagg | 13077 | - | transcription_regulator | translation regulator | - |
| 10841 | TEAD4 | NM_003213.1 | 1205 | gagtatgctgctatgagaatgg | 13091 | - | transcription_regulator | translation regulator | - |
| 10842 | TFAP2B | NM_003221.2 | 927 | cagcaaatgtcacgttactcacc | 13111 | - | see also 2182 line | - | - |
| 10843 | TFAP2BL1 | NM_172238.1 | 1122 | gtggtcaacccacagactatt | 13124 | - | see also 10042 line | - | - |
| 10844 | TFAP2C | NM_003222.3 | 994 | ctgtccccactgaatgcttaaa | 13156 | - | transcription_regulator | translation regulator | breast cancer |
| 10845 | TFCP2 | NM_005653.3 | 1075 | gggggtgccattccgagtacaaat | 13183 | - | transcription_regulator | - | Alzheimer disease |
| 10846 | TFDP1 | NM_007111.3 | 1234 | ctgttggaggcgtgttcatcacg | 13219 | - | transcription_regulator, cell_cycle | E2F-4/DP-1 complex | - |
| 10847 | TFDP2 | NM_006286.1 | 1117 | gtgtaaaccaagggttatgcttg | 13243 | - | see also 4376 line | - | - |
| 10848 | TFEB | NM_007162.1 | 1037 | gagacgaaggttcaacatcaatg | 13265 | - | transcription_regulator | transcription factor | - |
| 10849 | TFEC | NM_012252.1 | 1088 | aagattggatggcatgctattgg | 13290 | - | transcription_regulator | - | - |
| 10850 | TGIF | NM_003244.2 | 476 | ctgtatgagcacgttacaatgc | 13291 | - | transcription_regulator | - | - |
| 10851 | TGIF2 | NM_021809.4 | 342 | cagtgctgcaaatatgtaactgg | 13313 | - | transcription_regulator | - | - |
| 10852 | THG-1 | NM_030935.3 | 794 | caggtgtcaccacggactatgagg | 13319 | - | transcription_regulator | transcriptional repressor | - |
| 10853 | THRB | NM_000461.2 | 566 | aagggtacatccccagttactta | 13341 | - | see also 361 line | - | - |

Figure 46

| | | | | | | | | | |
|---|---|---|---|---|---|---|---|---|---|
| 10854 | TIEG | NM_005655.1 | 1190 | aagtcactcctcagattgattca | 13376 | - | transcription_regulator, signal_transduction | transcription factor | - |
| 10855 | TIX1 | NM_015035.2 | 3225 | tggacgtcagctcgaaacagact | 13422 | - | transcription_regulator | - | |
| 10856 | TLX1 | NM_005521.2 | 1550 | gagagtaaccgcagatacacaaa | 13427 | - | see also 3854 line | | |
| 10857 | TNRC4 | NM_007185.2 | 1115 | gaggacgtccggaagatgtttga | 13437 | - | transcription_regulator | - | - |
| 10858 | TP53 | NM_000546.2 | 384 | ctgtccccggacgatattgaaca | 13447 | - | transcription_regulator, apoptosis, cell_cycle, signal_transduction | transcription factor | hepatocellular carcinoma, bladder transitional cell carcinoma, alveolar cell carcinoma, rhabdomyosarcoma, ependymoma, cancer of the Fallopian tube, breast cancer, soft tissue sarcoma, leukemia, chondrosarcoma, rheumatoid arthritis, laryngeal cancer, Li-Fraumeni Syndrome, colorectal cancer, non-hodgkin lymphoma, nasopharyngeal cancer, hepatoblastoma, endometrial cancer, Li-Fraumeni syndrome, lung cancer, medulloblastoma, osteosarcoma, ovarian cancer, prostate cancer, Ewing's sarcoma, carcinoma of the cervix, CNS Tumours, melanoma, dysplastic nevus syndrome, head and neck cancer, colorectal cancers, Beckwith-Wiedemann syndrome, adamantinoma, testicular cance, squamous cell carcinomacytic, nerve sheath tumors, adrenocortical cancer, intraocular melanoma, head and neck squamous cell carcinoma, mesothelioma, lymphoma, astrocytic tumors, hepatocellular cancer, squamous cell cancer, chronic lymphocytic leukaemia, bladder squamous cell carcinoma, familial nervous system tumor syndromes, gastric cancer |
| 10859 | TP73 | NM_005427.1 | 755 | ccgcgtggaaggcaataatctct | 13468 | - | see also 3789 line | | |
| 10860 | TP73L | NM_003722.3 | 331 | cagaagatggtgcgacaaacaag | 13479 | - | transcription_regulator, apoptosis | - | Limb-mammary syndrome, ectodermal dysplasia, split-hand and split-foot, adult syndrome, Hay-Wells syndrome |

Figure 46

| 10861 | TRIM16 | NM_006470.2 | 546 | ccgcatcaggtgaacatcaaact | 13515 | - | transcription_regulator | - | - |
|---|---|---|---|---|---|---|---|---|---|
| 10862 | TRIM22 | NM_006074.2 | 359 | gagagtcaaagaggtcaagatga | 13517 | - | - | - | - |
| 10863 | TRIM28 | NM_005762.2 | 894 | atggtgaacgtactgtctattgc | 13531 | - | transcription_regulator | - | - |
| 10864 | TRIM29 | NM_012101.2 | 1381 | atgcatgcgccacgttgagaaga | 13558 | - | transcription_regulator | - | - |
| 10865 | TRPS1 | NM_014112.1 | 2177 | gggagccgaggataatatggtaa | 13626 | - | see also 5496 line | | |
| 10866 | TSC22 | NM_006022.2 | 347 | tggtgcaagtgtggtagctattg | 13691 | - | transcription_regulator | - | - |
| 10867 | TULP4 | NM_020245.2 | 1462 | gtgcaggagacgctactatgagg | 13778 | - | see also 7820 line | | |
| 10868 | UBP1 | NM_014517.2 | 2025 | agggtcccaccggtattcacatt | 13862 | - | see also 5748 line | | |
| 10869 | UHRF1 | NM_013282.2 | 1683 | cagggtggtgcgcaatgtcaagg | 13876 | - | transcription_regulator | - | - |
| 10870 | USF2 | NM_003367.1 | 199 | cagttccgcacagagacaaatgg | 13881 | - | see also 2272 line | | |
| 10871 | VAV1 | NM_005428.2 | 1789 | tggccgacatgggcaagatttcc | 13906 | - | transcription_regulator | transcription factor | - |
| 10872 | VDR | NM_000376.1 | 575 | cggcctccagttcgtgtgaatga | 13920 | - | see also 300 line | | |
| 10873 | VENTX2 | NM_014468.2 | 257 | gagtaaggagccaaataccttgc | 13932 | - | transcription_regulator | - | - |
| 10874 | VSX1 | NM_014588.4 | 908 | ctggggctctgaccacttcaaag | 13935 | - | transcription_regulator | - | - |
| 10875 | WT1 | NM_000378.2 | 1146 | agggtacgagagcgataaccaca | 13941 | - | see also 301 line | | |
| 10876 | XBP1 | NM_005080.2 | 613 | tggattctggcggtattgactct | 13957 | - | see also 3549 line | | |
| 10877 | YY1 | NM_003403.3 | 1632 | gtgcccttcgatggttgtaata | 13971 | - | see also 2304 line | | |
| 10878 | ZFH4 | NM_024721.2 | 1507 | taggtgaactcaccgatagtatt | 14019 | - | see also 8698 line | | |
| 10879 | ZFHX1B | NM_014795.2 | 1133 | ggggctacaagcgcttgacatca | 14254 | - | see also 5974 line | | |
| 10880 | ZFP36L1 | NM_004926.2 | 187 | gggtaacaagatgctcaactata | 14343 | - | transcription_regulator | transcription factor | - |
| 10881 | ZFP36L2 | NM_006887.3 | 1652 | cggaccgcgacagctacctaagc | 14353 | - | transcription_regulator | transcription factor | - |
| 10882 | ZFP37 | NM_003408.1 | 43 | cggcgtccagattctgacaaagc | 14355 | - | transcription_regulator | zinc binding | - |
| 10883 | ZFP95 | NM_014569.2 | 1537 | cggtcgccattcgcatctgatcg | 14386 | - | transcription_regulator | - | - |
| 10884 | ZHX2 | NM_014943.2 | 2880 | gaggatgcgatctcagatagatc | 14499 | - | see also 6132 line | | |
| 10885 | ZNF11B | NM_006955.1 | 715 | gagtcatcgtgagaacactttgc | 14504 | - | transcription_regulator | transcription factor | - |

Figure 46

| | | | | | | | | | |
|---|---|---|---|---|---|---|---|---|---|
| 10886 | ZNF132 | NM_003433.1 | 499 | atgtgtgggccattcttgaaaga | 14505 | - | transcription_regulator | transcription factor | - |
| 10887 | ZNF133 | NM_003434.3 | 2453 | gggcaaccttcggattccttata | 14514 | - | transcription_regulator | transcription factor | - |
| 10888 | ZNF134 | NM_003435.1 | 1343 | tagtcgaagttctgactatattg | 14524 | - | transcription_regulator | transcription factor | - |
| 10889 | ZNF135 | NM_003436.2 | 318 | tgggcggtggagtctagacttcc | 14525 | - | transcription_regulator | - | - |
| 10890 | ZNF137 | NM_003438.1 | 201 | ctgtctaaggtttctaatcaaca | 14536 | - | transcription_regulator | transcription factor | - |
| 10891 | ZNF140 | NM_003440.2 | 1687 | ttgtcactcattccttattaaac | 14564 | - | transcription_regulator | transcription factor | - |
| 10892 | ZNF142 | NM_005081.1 | 2772 | gggtaggaaccttcaagacaact | 14588 | - | - | transcription factor | |
| 10893 | ZNF145 | NM_006006.3 | 1473 | tggggtcgagcttcctgataacg | 14617 | - | transcription_regulator | transcription co-repressor | leukemia |
| 10894 | ZNF147 | NM_005082.2 | 158 | gcgggtcgtgcctgaatgagacg | 14624 | - | transcription_regulator | transcription factor | - |
| 10895 | ZNF154 | NM_003444.1 | 560 | ctggggagcaatcaaatagcaaa | 14649 | - | - | - | - |
| 10896 | ZNF155 | NM_003445.2 | 612 | caggactacccacaattcataca | 14657 | - | transcription_regulator | transcription factor | - |
| 10897 | ZNF157 | NM_003446.2 | 1574 | ctggcgttgtacaatgaagaaag | 14675 | - | transcription_regulator | DNA binding | - |
| 10898 | ZNF165 | NM_003447.2 | 1436 | gaggaggcagtgaccatactaga | 14680 | - | transcription_regulator | transcription factor | - |
| 10899 | ZNF167 | NM_018651.1 | 659 | gtggggacttcgctcaatgtact | 14700 | - | transcription_regulator | - | - |
| 10900 | ZNF174 | NM_003450.1 | 1506 | gagcaaccgtttgcaacatcttg | 14714 | - | transcription_regulator | transcription factor | - |
| 10901 | ZNF175 | NM_007147.2 | 1346 | gtgtatatctgacagatcataca | 14733 | - | transcription_regulator | transcription factor | - |
| 10902 | ZNF187 | NM_152736.2 | 425 | cagaacgtgagcagagattcatc | 14747 | - | transcription_regulator | transcription factor | - |
| 10903 | ZNF189 | NM_003452.2 | 831 | tagtcgcagttcatttgttattg | 14761 | - | see also 2316 line | | |
| 10904 | ZNF19 | NM_006961.2 | 1019 | acgagtagttccgagtttgttat | 14793 | - | transcription_regulator | - | - |
| 10905 | ZNF192 | NM_006298.1 | 857 | atggctgtgtcccttattcgaga | 14807 | - | transcription_regulator | transcription factor | - |
| 10906 | ZNF193 | NM_006299.2 | 546 | gagctcgatgaaccacaacatga | 14809 | - | transcription_regulator | transcription factor | - |

Figure 46

| 10907 | ZNF197 | NM_006991.2 | 1231 | gcgttcgagccttctaatgcatt | 14839 | - | transcription_regulator | transcription factor | - |
|---|---|---|---|---|---|---|---|---|---|
| 10908 | ZNF202 | NM_003455.1 | 1149 | ttgtgaaccttcgggaaactaca | 14870 | - | transcription_regulator | specific RNA polymerase II transcription factor | atherosclerosis |
| 10909 | ZNF207 | NM_003457.1 | 387 | tagatgccgtaccaaatgcaata | 14890 | - | see also 2338 line | | |
| 10910 | ZNF211 | NM_006385.2 | 961 | aagcatgtacgcgaagatgtaac | 14926 | - | transcription_regulator | - | - |
| 10911 | ZNF213 | NM_004220.1 | 977 | gggagacatcccattctatttct | 14931 | - | transcription_regulator | - | - |
| 10912 | ZNF215 | NM_013250.1 | 2005 | cggagctcctctcttattcgaca | 14969 | - | transcription_regulator | zinc binding | Beckwith-Wiedemann syndrome |
| 10913 | ZNF217 | NM_006526.2 | 394 | agggaccgctgttgttccattcc | 14977 | - | transcription_regulator | transcription factor | breast cancer |
| 10914 | ZNF219 | NM_016423.1 | 2020 | cagtccggctcgctcaagtatca | 15051 | - | transcription_regulator | transcription factor | - |
| 10915 | ZNF232 | NM_014519.2 | 1073 | cggggacatcaccctaagagtgg | 15056 | - | transcription_regulator | transcription factor | - |
| 10916 | ZNF236 | NM_007345.1 | 3367 | gaggagggtttccgaactacagt | 15134 | - | transcription_regulator | transcription factor | diabetes |
| 10917 | ZNF24 | NM_006965.1 | 1116 | cagaattcggggcttattaatca | 15172 | - | transcription_regulator | zinc binding | - |
| 10918 | ZNF256 | NM_005773.2 | 798 | ctgctcacaccagaaagaagtca | 15180 | - | transcription_regulator | transcription factor | - |
| 10919 | ZNF263 | NM_005741.3 | 1238 | tggaaggtgttccgtctgtatgc | 15190 | - | see also 3982 line | | |
| 10920 | ZNF265 | NM_005455.2 | 199 | aggcctatttagtgctaatgact | 15215 | - | transcription_regulator | transcription factor | - |
| 10921 | ZNF274 | NM_016324.2 | 537 | cagatccaggccctatatgctga | 15238 | - | transcription_regulator | - | - |
| 10922 | ZNF277 | NM_021994.1 | 1299 | gtgggatcaactggagtattatt | 15285 | - | transcription_regulator | transcription factor | - |
| 10923 | ZNF287 | NM_020653.1 | 1432 | cagggcaaccttcaatcatgtct | 15317 | - | transcription_regulator | transcription factor | - |
| 10924 | ZNF3 | NM_032924.2 | 1574 | gagttacgaccgagttaaatatc | 15334 | - | transcription_regulator | DNA binding | - |
| 10925 | ZNF3 | NM_017715.1 | 390 | ggggtcctactgggaagatttca | 15337 | - | transcription_regulator | - | - |
| 10926 | ZNF305 | NM_014724.1 | 1333 | tcggcgttccatacttactcagc | 15366 | - | - | transcription factor | - |
| 10927 | ZNF306 | NM_024493.1 | 963 | aggagcatgggaagatatcgtgc | 15373 | - | transcription_regulator | - | - |

Figure 46

| 10928 | ZNF31 | NM_145238.2 | 1372 | ctgttccagagtcgtattgcagg | 15385 | - | transcription_regulator | DNA binding | - |
| 10929 | ZNF323 | NM_030899.2 | 1331 | aagcggacacttcttaagaaaca | 15404 | - | transcription_regulator | - | - |
| 10930 | ZNF33A | NM_006974.1 | 1347 | ccgtgtgacttcgcaccttaaag | 15410 | - | transcription_regulator | transcription factor | - |
| 10931 | ZNF35 | NM_003420.2 | 311 | gtgcactccgagaacatcaaagt | 15413 | - | transcription_regulator | transcription factor | small cell lung cancer |
| 10932 | ZNF38 | NM_145914.2 | 728 | aggtccgagattgcagattgagt | 15432 | - | transcription_regulator | zinc binding | - |
| 10933 | ZNF394 | NM_032164.2 | 1395 | aagccgtacacctgtctgaaatg | 15453 | - | transcription_regulator | - | - |
| 10934 | ZNF396 | NM_145756.1 | 676 | cagtccttaagaccacatgatga | 15470 | - | transcription_regulator | DNA binding | - |
| 10935 | ZNF397 | NM_032347.1 | 175 | caggatcctccggaacaagaact | 15485 | - | transcription_regulator | - | - |
| 10936 | ZNF41 | NM_007130.1 | 2424 | ctgaccgatcaaatctcataaca | 15523 | - | transcription_regulator | - | - |
| 10937 | ZNF42 | NM_003422.2 | 583 | gaggctgctgccctagtagatgg | 15526 | - | transcription_regulator | transcription factor | - |
| 10938 | ZNF434 | NM_017810.2 | 1756 | tagccgaagttcttatcttgttc | 15544 | - | - | - | - |
| 10939 | ZNF435 | NM_025231.1 | 501 | cagaaagacgggacatactcatg | 15550 | - | transcription_regulator | transcription factor | - |
| 10940 | ZNF45 | NM_003425.2 | 1149 | aagctcctctgatagttcatacg | 15577 | - | transcription_regulator | transcription factor | - |
| 10941 | ZNF494 | NM_152677.1 | 1132 | gaggatatgcccttaagagatgt | 15593 | - | transcription_regulator | - | - |
| 10942 | ZNF495 | NM_024303.1 | 1590 | gagccttcagtcggctgaaattg | 15624 | - | transcription_regulator | - | - |
| 10943 | ZNF496 | NM_032752.1 | 2143 | aagagcttcacgcagaactatga | 15631 | - | transcription_regulator | - | - |
| 10944 | ZNF70 | NM_021916.2 | 764 | cagcatgtgtcagataatccaca | 15635 | - | - | - | - |
| 10945 | ZNF75 | NM_007131.1 | 1196 | cagccttatacgtgtagcttatg | 15645 | - | transcription_regulator | zinc binding | - |
| 10946 | ZNF80 | NM_007136.1 | 617 | gggaccaagtaaagacactttgg | 15649 | - | transcription_regulator | transcription factor | - |
| 10947 | ZNF83 | NM_018300.2 | 1928 | ccgcgacaattcatatcttgtac | 15668 | - | transcription_regulator | transcription factor | - |
| 10948 | ZNF9 | NM_003418.1 | 468 | tggagaattcggacacattcaaa | 15676 | - | see also 2310 line | | |
| 10949 | ZNFN1A3 | NM_012481.3 | 1320 | gaggttccccgctcttacgaact | 15713 | - | - | - | - |

Figure 46

| 10950 | ZNFN1A4 | NM_022465.2 | 1752 | tggaccacgtcatgttcactatc | 15727 | - | transcription_regulator | - | - |
|---|---|---|---|---|---|---|---|---|---|
| 10951 | ZNRD1 | NM_014596.4 | 190 | cagtcggacctggatttctgttc | 15728 | - | transcription_regulator | - | - |
| 10952 | BRCA1 | NM_007294.1 | 4329 | gagggataccatgcaacataacc | 16042 | - | see also 5077 line | | |
| 10953 | DDB1 | NM_001923.2 | 1346 | ctgcggtctgaccctaatcgtga | 16096 | - | see also 1223 line | | |
| 10954 | DDB2 | NM_000107.1 | 467 | ctggattcttaccggatattaca | 16136 | - | - | damaged DNA binding | - |
| 10955 | DMC1 | NM_007068.2 | 1146 | atgaagccaccttcgcaataact | 16193 | - | phosphatase、cell_cycle | damaged DNA binding | Alzheimer disease |
| 10956 | ERCC1 | NM_001983.1 | 466 | gggcaatcccgtactgaagttcg | 16196 | - | - | endodeoxyribonuclease | |
| 10957 | ERCC3 | NM_000122.1 | 1920 | aaggtaggtgacacttcgtttga | 16250 | - | transcription_regulator、apoptosis | RNA polymerase II transcription factor | ichthyosiform erythroderma、xeroderma pigmentosum |
| 10958 | FANCG | NM_004629.1 | 1405 | ctgctagttgaggccttgaatgt | 16276 | - | see also 3144 line | | |
| 10959 | FEN1 | NM_004111.4 | 610 | aagcccgtgtatgtctttgatgg | 16296 | - | - | endonuclease | - |
| 10960 | JTV1 | NM_006303.2 | 396 | gcggatgagcccacgactttaac | 16306 | - | see also 4395 line | | |
| 10961 | MPG | NM_002434.1 | 960 | cggaaacccctccgcttctatgt | 16333 | - | - | alkylbase DNA N-glycosylase | - |
| 10962 | MSH2 | NM_000251.1 | 784 | aggacctcaaccggttgttgaaa | 16347 | - | see also 183 line | | |
| 10963 | MSH3 | NM_002439.1 | 2157 | ttgacgagatccgaatgcatttg | 16496 | - | - | damaged DNA binding | ataxia telangiectasia、endometrial cancer |
| 10964 | MSH4 | NM_002440.2 | 1085 | tagacgacttcgttctaatatat | 16566 | - | - | damaged DNA binding | - |
| 10965 | MSH6 | NM_000179.1 | 3186 | gcggcgactgttctataactttg | 16780 | - | see also 153 line | | |
| 10966 | N4BP2 | NM_018177.2 | 313 | ctggatcctgatgtagtgtattt | 16821 | - | - | - | - |
| 10967 | NBS1 | NM_002485.3 | 384 | aagtcgggggatggtattacttt | 16970 | - | see also 1591 line | | |
| 10968 | OGG1 | NM_002542.4 | 1436 | gtgtactagcggatcaagtatgg | 17028 | - | - | - | lung squamous cell carcinoma、renal cell carcinoma、non-small-cell lung cancer |
| 10969 | PNKP | NM_007254.2 | 306 | tgggagttaacccctcaactacc | 17050 | - | kinase_related、phosphatase | - | - |
| 10970 | POLH | NM_006502.1 | 1138 | atgtgccgagggattgaacatga | 17076 | - | see also 4548 line | | |
| 10971 | POLQ | NM_006596.3 | 4551 | aggagcgactaagatagatcatt | 17254 | - | see also 4627 line | | |
| 10972 | RAD1 | NM_002853.2 | 1048 | aggtatctattcggacagataac | 17396 | - | see also 1883 line | | |
| 10973 | RAD18 | NM_020165.2 | 1002 | aagactaggatgcgtcttgaagc | 17436 | - | - | damaged DNA binding | - |
| 10974 | RAD51 | NM_002875.2 | 885 | atgatggtagaatctaggtatgc | 17470 | - | see also 1917 line | | |
| 10975 | RAD51C | NM_002876.2 | 377 | ggggtggagtgcccttaatgaaa | 17486 | - | phosphatase | - | - |

458

Figure 46

| 10976 | RAD51L1 | NM_002877.3 | 936 | tagccgcactaggaaatacctgg | 17537 | - | phosphatase | - | leiomyoma、uterine leiomyoma |
|---|---|---|---|---|---|---|---|---|---|
| 10977 | RAD51L3 | NM_002878.2 | 992 | gtggtgaccaaccacataactcg | 17548 | - | phosphatase、transcription_regulator | - | - |
| 10978 | REV1L | NM_016316.1 | 2972 | cagtgtgcagtcgagacttaacc | 17630 | - | see also 6805 line | | |
| 10979 | TIAF1 | NM_004740.2 | 184 | cgggtccaggttcttaagaatga | 17662 | - | apoptosis、kinase_related | - | - |
| 10980 | XPA | NM_000380.2 | 587 | atggggtgatatgaaactctact | 17733 | - | - | - | - |
| 10981 | XPC | NM_004628.2 | 590 | gggcgatgaaacgtttcaataaa | 17758 | - | - | single-stranded DNA binding | xeroderma pigmentosum、melanoma |
| 10982 | XRCC1 | NM_006297.1 | 1834 | atgagtgaccgggttcagtttgt | 17804 | - | - | damaged DNA binding | head and neck squamous cell carcinoma |
| 10983 | XRCC2 | NM_005431.1 | 346 | ttgatatgctccggctagttaca | 17810 | - | phosphatase | damaged DNA binding | - |
| 10984 | XRCC3 | NM_005432.2 | 503 | gagaacggcctccttacacttgc | 17837 | - | phosphatase、transcription_regulator | damaged DNA binding | melanoma |
| 10985 | ASF1A | NM_014034.1 | 462 | aggcgtaactgttgtgctaatta | 17846 | - | see also 5464 line | | |
| 10986 | BAF53A | NM_004301.2 | 1295 | aagtatgcggttgaaattgattg | 17894 | - | signal_transduction | - | - |
| 10987 | CBX4 | NM_003655.1 | 264 | gaggctggtcgcccaaatataac | 17901 | - | see also 2476 line | | |
| 10988 | CBX5 | NM_012117.1 | 644 | gagactgacatggcatgcatatc | 17923 | - | - | lamin/chromatin binding | - |
| 10989 | CBX7 | NM_175709.1 | 536 | gcggctctcgcgcaagaagttcc | 17929 | - | transcription_regulator | chromatin binding | - |
| 10990 | CBX8 | NM_020649.1 | 191 | agggatggtcgcagaagtacagc | 17931 | - | transcription_regulator | chromatin binding | - |
| 10991 | CDYL | NM_004824.2 | 1580 | tggctgttctaccgttatgtttc | 17962 | - | - | - | - |
| 10992 | CDYL2 | NM_152342.1 | 351 | aggcccgtcggttgagaaactgt | 17971 | - | - | - | - |
| 10993 | CENPA | NM_001809.2 | 310 | aagagcacacacctcttgataag | 17987 | - | - | chromatin binding | - |
| 10994 | CENPB | NM_001810.4 | 1346 | aggaggagggtgatgttgatagt | 17995 | - | - | satellite DNA binding | - |
| 10995 | CHAF1A | NM_005483.1 | 1430 | cggcgtcggaccgctttccatcc | 18023 | - | cell_cycle | histone-specific chaperone | - |
| 10996 | CHAF1B | NM_005441.1 | 941 | ctgtccggtctactttgaactga | 18054 | - | gpcr、cell_cycle、transcription_regulator、signal_transduction | signal transducer | - |
| 10997 | CHD1 | NM_001270.1 | 2603 | gtgcggatgttagatatacttgc | 18153 | - | see also 832 line | | |
| 10998 | CHD2 | NM_001271.1 | 5733 | ggggaccggcgacatatggatgc | 18344 | - | transcription_regulator | chromatin binding | Alzheimer disease |
| 10999 | CHD5 | NM_015557.1 | 1697 | ctgtaccacacggtgatgtatcg | 18356 | - | transcription_regulator | - | - |

EP 1 752 536 A1

Figure 46

| 11000 | CHD6 | NM_032221.3 | 6386 | gaggctcgagctcccactattgc | 18496 | - | see also 9120 line | | |
|---|---|---|---|---|---|---|---|---|---|
| 11001 | EZH1 | NM_001991.2 | 192 | atgcgacttcgacaacttaaacg | 18525 | - | see also 1278 line | | |
| 11002 | HTATIP | NM_006388.2 | 1161 | ttgatggacgtaagaacaagagt | 18575 | - | see also 4476 line | | |
| 11003 | ING1L | NM_001564.1 | 352 | gagagcactaattaatagtcaag | 18590 | - | signal_transduction、tr anscription_regulator | - | - |
| 11004 | MGC10561 | NM_032647.2 | 143 | gcggctggtcctccaaacataac | 18606 | - | transcription_regulator | - | - |
| 11005 | MORF4L1 | NM_006791.1 | 816 | ttgggtacccagctactctataa | 18607 | - | - | chromatin binding | - |
| 11006 | MPO | NM_000250.1 | 574 | ctgtggcgaaggccattcaatgt | 18608 | - | apoptosis | Smad3/Sp-1 heterodimer | myeloperoxidase deficiency、Alzheimer disease |
| 11007 | MYST1 | NM_032188.1 | 935 | gagtccccggatggaaacaatgt | 18637 | - | - | - | - |
| 11008 | NCOA6 | NM_014071.2 | 598 | cagcgtgcgtgtgacattcaaca | 18641 | - | see also 5477 line | | |
| 11009 | NSBP1 | NM_030763.1 | 894 | gaggagccacagagtattgttta | 18775 | - | transcription_regulator | - | - |
| 11010 | PB1 | NM_018165.2 | 4641 | tcgaagacgcgacgtccatttgt | 18882 | - | see also 7297 line | | |
| 11011 | RBP1L1 | NM_016374.4 | 1428 | aagatagaggtacacctattaac | 18903 | - | see also 6844 line | | |
| 11012 | SMARCC1 | NM_003074.2 | 1325 | aggaactgtagcggatctagatg | 19028 | - | see also 2062 line | | |
| 11013 | SMARCC2 | NM_003075.2 | 601 | gggtacgaccagtcatgaagagg | 19080 | - | see also 2063 line | | |
| 11014 | SMARCE1 | NM_003079.3 | 1260 | aaggaaccagtgatagtaacact | 19109 | - | transcription_regulator | - | - |
| 11015 | SUV39H1 | NM_003173.1 | 1222 | aagaagcgggtccgtattgaatg | 19130 | - | - | - | - |
| 11016 | SUV39H2 | NM_024670.3 | 1007 | cggctcgatacggcaatgtgtct | 19154 | - | see also 8695 line | | |
| 11017 | BRCA2 | NM_000059.1 | 7223 | gtgtacccttcgcacaactaag | 19363 | - | see also 34 line | | |
| 11018 | ERCC5 | NM_000123.1 | 2729 | gggattggaccggaataagttaa | 19529 | - | see also 98 line | | |
| 11019 | HNRPDL | NM_005463.2 | 1293 | gtggattgagcccggatacttct | 19570 | - | see also 3802 line | | |
| 11020 | IGHMBP2 | NM_002180.1 | 2311 | caggctgcgggtccaccaaatag | 19605 | - | transcription_regulator | antigen binding | - |
| 11021 | KHDRBS1 | NM_006559.1 | 1248 | aaggctacgaaggctattacagc | 19623 | - | cell_cycle、signal_tran sduction | - | - |
| 11022 | PIR51 | NM_006479.2 | 52 | tggtgcggcctgtgagacataag | 19653 | - | - | - | - |
| 11023 | POLG2 | NM_007215.1 | 1210 | ttgtttagcccctattaaggttg | 19699 | - | see also 5024 line | | |
| 11024 | RAD23B | NM_002874.2 | 908 | cgggtcagtcttacgagaatatg | 19724 | - | see also 1914 line | | |
| 11025 | RBMS1 | NM_002897.2 | 1212 | gtggctgtcgagacgtctaatga | 19758 | - | see also 1936 line | | |
| 11026 | RPA1 | NM_002945.2 | 1160 | cagcccgtgttggctatcaaagg | 19795 | - | see also 1989 line | | |
| 11027 | RPA2 | NM_002946.3 | 1029 | gagcaatgaggggcacatctatt | 19831 | - | see also 1990 line | | |
| 11028 | RPA3 | NM_002947.2 | 1525 | tagggattgtgcaacatgattga | 19842 | - | - | single-stranded DNA binding | - |

EP 1 752 536 A1

Figure 46

| | | | | | | | | | |
|---|---|---|---|---|---|---|---|---|---|
| 11029 | SSBP1 | NM_003143.1 | 79 | atgtttcgaagacctgtattaca | 19847 | - | - | single-stranded DNA binding | |
| 11030 | SSBP2 | NM_012446.2 | 272 | aagttagcactcacgtatatga | 19865 | - | see also 5334 line | - | |
| 11031 | SSBP3 | NM_018070.2 | 1025 | cagtgacaacatctacacaatga | 19879 | - | transcription_regulator | - | |
| 11032 | SSBP4 | NM_032627.1 | 282 | tggcgctgacgtttatgagtac | 19882 | - | see also 9263 line | | |
| 11033 | WBP11 | NM_016312.2 | 1007 | aagtgacggggatgaatttgtgc | 19926 | - | - | - | |
| 11034 | CGGBP1 | NM_003663.2 | 357 | atggagcgatttgtagtaacagc | 19930 | - | - | - | |
| 11035 | G22P1 | NM_001469.2 | 2057 | aagcttcgcttcacatacagaag | 19941 | - | - | Ku70/80 antigen complex | |
| 11036 | IFI16 | NM_005531.1 | 2346 | gagatctgtaattcatagtcaca | 19978 | - | transcription_regulator | transcriptional repressor | |
| 11037 | MRE11A | NM_005590.2 | 690 | ttgcgctatatgtttaggatcc | 19993 | - | - | - | |
| 11038 | MTERF | NM_006980.2 | 825 | aagcgggtgaaagctaacattga | 20067 | - | transcription_regulator | transcription termination factor | |
| 11039 | NP220 | NM_014497.2 | 1833 | cggtctcgaagccaatgcatta | 20140 | - | see also 5721 line | - | |
| 11040 | SAFB | NM_002967.2 | 1728 | gggaccgaacgaactgtagtaat | 20293 | - | transcription_regulator | - | |
| 11041 | XRCC5 | NM_021141.2 | 1198 | atggtggccatagttcgatatgc | 20325 | - | see also 8105 line | - | |
| 11042 | MBD2 | NM_003927.3 | 902 | ctgcgaaacgatcctctcaatca | 20354 | - | see also 2688 line | - | |
| 11043 | MBD4 | NM_003925.1 | 1548 | aagcttcatcgctactatatt | 20394 | - | - | - | |
| 11044 | MLH3 | NM_014381.1 | 3516 | tagcgaatcgcttcagtctttgt | 20490 | - | - | satellite DNA binding | |
| 11045 | ORC2L | NM_006190.3 | 1344 | atgggtactttccgcagtatact | 20543 | - | see also 4277 line | - | |
| 11046 | ORC3L | NM_012381.2 | 165 | aagcttcgattcgaaacttatca | 20575 | - | - | - | |
| 11047 | ORC4L | NM_002552.2 | 251 | cagagtccacatagtaaccatt | 20640 | - | see also 1642 line | - | |
| 11048 | ORC5L | NM_002553.2 | 245 | atgtaacacaaacgttgttgaaa | 20680 | - | see also 1646 line | - | |
| 11049 | TERF2 | NM_005652.2 | 438 | cagtgtctgtcgcggattgaaga | 20722 | - | see also 3921 line | - | |
| 11050 | TERT | NM_003219.1 | 651 | gcgaacgggcctggaaccatagc | 20742 | - | - | telomeric template RNA reverse transcriptase | leukemia |
| 11051 | KIF2C | NM_006845.2 | 1942 | cagatgtccagctttaacgaagc | 20797 | - | - | microtubule motor | - |
| 11052 | ZW10 | NM_004724.2 | 1457 | ttgcccacatgcgtatcagtga | 20842 | - | cell_cycle | - | |

Figure 46

| | Gene | Accession | | Sequence | ID | | Function | | Disease / Notes |
|---|---|---|---|---|---|---|---|---|---|
| 11053 | HMGA2 | NM_003483.3 | 1100 | aggaaatggccacaacaagtgt | 20866 | - | transcription_regulator | AT DNA binding | Well-differentiated liposarcoma, pulmonary chondroid hamartoma, soft tissue sarcoma, liposarcoma, myofibroblastic inflammatory tumor, lipomatous tumors, osteosarcoma, pleomorphic salivary gland adenoma, uterine leiomyoma, lipoma |
| 11054 | TP53BP1 | NM_005657.1 | 3563 | aggacggagtactaataaggaaa | 20939 | - | see also 3924 line | | |
| 11055 | ZBP1 | NM_030776.1 | 665 | acgattaccgcccagaagattc | 20993 | - | - | | |
| 11056 | CGBP | NM_014593.1 | 327 | ctgccgcaaaccggcatcaact | 21006 | - | see also 5766 line | | |
| 11057 | 3'HEXO | NM_153332.2 | 1022 | aagaatatcgccgaatagcagt | 21049 | - | - | | |
| 11058 | ABL1 | NM_005157.2 | 867 | aagggagggtgaccattacagg | 21106 | - | kinase_related, cell_c ycle, transcription_reg ulator, signal_transdu ction, apoptosis | | leukemia, chronic myeloid leukemia, chronic myelogenous leukemia, Ewing's sarcoma |
| 11059 | ACINUS | NM_014977.1 | 381 | tggtaatcgagggtagaagtatg | 21127 | - | apoptosis | | |
| 11060 | ADPRT | NM_001618.2 | 2685 | aggccgaatgccagcgttacaagc | 21213 | - | transcription_regulator | transferase, transferring glycosyl groups | |
| 11061 | ADPRTL2 | NM_005484.2 | 1478 | aggtcagtgttaatgaactactag | 21272 | - | see also 3814 line | | |
| 11062 | AKAP8 | NM_005858.2 | 306 | cagagccatgcaacgcaacaattcc | 21284 | - | see also 4073 line | | |
| 11063 | ALF | NM_006872.2 | 1112 | tagatggaagcggtgatacatct | 21339 | - | transcription_regulator | | |
| 11064 | ALG1 | NM_019109.2 | 619 | gagaagacctggcggataactgg | 21356 | - | - | | |
| 11065 | ALTE | NM_004729.1 | 1585 | acgcccgagatcgacatgtttct | 21360 | - | - | | |
| 11066 | ANKRD1 | NM_014391.2 | 943 | atgagtgcgcggagcatcttatc | 21372 | - | see also 5654 line | | |
| 11067 | APLP2 | NM_001642.1 | 877 | gaggaccgagattactactatga | 21421 | - | see also 1087 line | | |
| 11068 | APOB48R | NM_018690.1 | 284 | gtggggagctcagctgtagaaca | 21446 | - | receptor_activity | | atherosclerosis |
| 11069 | ARAP3 | NM_022481.4 | 2374 | tggtggtgcgtgctacttacagc | 21478 | - | see also 8403 line | | |
| 11070 | ARFGAP1 | NM_018209.2 | 374 | gagtctcaggagggattacgatcc | 21504 | - | see also 7321 line | | |
| 11071 | ARFGAP3 | NM_014570.3 | 1624 | tggagtcgtgacttcaattcagg | 21557 | - | see also 5756 line | | |
| 11072 | ASH1L | NM_018489.1 | 8588 | gtgtgttggacctttatacgtat | 21806 | - | see also 7495 line | | |
| 11073 | ATF2 | NM_001880.2 | 426 | ttggtccagcacgtaatgacagt | 21826 | - | see also 1204 line | | |
| 11074 | ATF5 | NM_012068.2 | 380 | ctggatggctcgtagactatgg | 21857 | - | see also 5104 line | | |
| 11075 | ATM | NM_000051.2 | 4577 | tgttcttcgagacgttatttat | 21997 | - | see also 18 line | | |
| 11076 | AWP1 | NM_019006.2 | 476 | cagtagtcgtctgaatctttac | 22127 | - | - | | |
| 11077 | BACH2 | NM_021813.1 | 2733 | atgtgaaatccgcaaattggtgt | 22161 | - | transcription_regulator | translation regulator | |

Figure 46

| | | | | | | | | | | |
|---|---|---|---|---|---|---|---|---|---|---|
| 11078 | BAZ1A | NM_013448.2 | 2981 | ttgggtcgcgaccgcatgtatag | 22247 | - | see also 5447 line | | | |
| 11079 | BAZ2A | NM_013449.2 | 2054 | cggggtcgacctcgaaacactga | 22328 | - | see also 5449 line | | | |
| 11080 | BAZ2B | NM_013450.1 | 4263 | gtgcacgttgtcttatcagaaca | 22455 | - | transcription_regulator | - | | |
| 11081 | BBX | NM_020235.2 | 1063 | ttgccgagatatcttcaagtacg | 22523 | - | see also 7810 line | | | |
| 11082 | BDP1 | NM_018429.1 | 5592 | ctgtccacaaccgttaaacgaaa | 22667 | - | - | - | - | |
| 11083 | BHC80 | NM_016621.2 | 1762 | gtgttcccgtgtatatcatttgg | 22732 | - | see also 6953 line | | | |
| 11084 | BLM | NM_000057.1 | 1998 | gagcgtttccaaagtcttagttt | 22786 | - | see also 30 line | | | |
| 11085 | BOC | NM_033254.2 | 858 | cagaggtaactacctgatcatgc | 22869 | - | see also 9387 line | | | |
| 11086 | BRD1 | NM_014577.1 | 2391 | tagataactgcatgaagtacaat | 22932 | - | transcription_regulator | molecular_function unknown | Alzheimer disease |
| 11087 | BRIP1 | NM_032043.1 | 1790 | ttgcgattcaacagacttactcc | 23006 | - | see also 9068 line | | | |
| 11088 | BRPF1 | NM_004634.1 | 937 | gtgctacggtgtcccctatatcc | 23082 | - | see also 3148 line | | | |
| 11089 | BS69 | NM_006624.1 | 837 | ctgacattgcgaggatgctatat | 23123 | - | see also 4684 line | | | |
| 11090 | C11orf13 | NM_003475.2 | 350 | ccggaacgctgcctaattcgtgc | 23145 | - | signal_transduction、tr anscription_regulator | DNA binding | - |
| 11091 | C14orf106 | NM_018353.2 | 1673 | ttgatagacgtcactaacatata | 23182 | - | - | - | - | |
| 11092 | C14orf146 | NM_152447.2 | 1629 | gtgtatgataacggaacacttga | 23274 | - | see also 9869 line | | | |
| 11093 | C20orf100 | NM_032883.1 | 697 | aggagtcggaagtgcatttcaag | 23313 | - | transcription_regulator | - | | |
| 11094 | C20orf104 | NM_016436.3 | 230 | aagcgttggaaccatcgttatga | 23323 | - | transcription_regulator | - | Alzheimer disease |
| 11095 | C21orf127 | NM_013240.2 | 288 | gagacagcacgctgtaacaaagt | 23388 | - | - | S-adenosylmethionine-dependent methyltransferase | - |
| 11096 | C21orf66 | NM_013329.2 | 1166 | atgactcccgttactattgattt | 23418 | - | see also 5401 line | | | |
| 11097 | CACNA1A | NM_000068.2 | 5807 | cagcttgcggtcggattcattat | 23567 | - | see also 40 line | | | |
| 11098 | CARP-1 | NM_018237.1 | 957 | cagcctcctgttcgtatagtttc | 23607 | - | see also 7330 line | | | |
| 11099 | CBF2 | NM_005760.1 | 158 | aggcaccaagcaagattacctta | 23679 | - | see also 4017 line | | | |
| 11100 | CCNL1 | NM_020307.1 | 744 | cagtcttcgaaccaatgtgtttg | 23768 | - | see also 7825 line | | | |
| 11101 | CDC5L | NM_001253.2 | 1941 | tagaaccgcctttaacagattta | 23839 | - | transcription_regulator | - | Alzheimer disease |
| 11102 | CEBPG | NM_001806.2 | 324 | gggtgaacggaattagtgttatc | 23854 | - | transcription_regulator | translation regulator | - |
| 11103 | CENPC1 | NM_001812.1 | 2548 | aggatctgtcttgataacgatga | 23936 | - | - | DNA binding | - |
| 11104 | CENTA1 | NM_006869.1 | 1277 | tggaggcgcacttcaagcataaa | 23975 | - | see also 4808 line | | | |

Figure 46

| 11105 | CENTA2 | NM_018404.1 | 244 | ctgcggcgtccaccgtaacttcc | 23978 | - | see also 7449 line | | |
|---|---|---|---|---|---|---|---|---|---|
| 11106 | CENTB1 | NM_014716.1 | 233 | ccgtttccgagcctctattgagc | 23997 | - | see also 5909 line | | |
| 11107 | CENTB2 | NM_012287.3 | 397 | ctgtcgttgagacaagtttgacc | 24025 | - | - | GTPase activator | - |
| 11108 | CENTB5 | NM_030649.1 | 976 | gagagccctgacagttgctatag | 24087 | - | - | - | - |
| 11109 | CENTD1 | NM_015230.2 | 4348 | aggaacccgactgtagtattata | 24215 | - | signal_transduction | - | Alzheimer disease |
| 11110 | CENTD2 | NM_015242.2 | 1112 | ccggctctctagcgcttatctgc | 24265 | - | see also 6286 line | | |
| 11111 | CENTG1 | NM_014770.1 | 695 | atgaaacgctgcagctactatga | 24292 | - | - | - | - |
| 11112 | CENTG2 | NM_014914.1 | 839 | cggtacctgacgggcacatatgt | 24302 | - | - | - | - |
| 11113 | CENTG3 | NM_031946.3 | 261 | ccgctccgtaccggagcttaaag | 24319 | - | signal_transduction | - | - |
| 11114 | CHC1 | NM_001269.2 | 898 | aggccgtgtgcctgagttatttg | 24340 | - | see also 831 line | | |
| 11115 | CHD1L | NM_024568.1 | 1818 | gggccgatcactccgaaataaag | 24380 | - | - | - | - |
| 11116 | CHRAC1 | NM_017444.3 | 357 | atgcctagccacctattcctaca | 24385 | - | - | - | - |
| 11117 | CHTF18 | NM_022092.1 | 62 | gtgcggcgtcgaggatgatttcc | 24389 | - | - | - | - |
| 11118 | CIC | NM_015125.2 | 2220 | tagtgtattcggacaagaagtcg | 24411 | - | transcription_regulator | - | - |
| 11119 | CIP29 | NM_033082.1 | 336 | gagggctgaacgattcaatgtac | 24422 | - | transcription_regulator | DNA binding | - |
| 11120 | CNTN1 | NM_001843.2 | 1618 | cagatcctacgcgaattatattg | 24479 | - | see also 1191 line | | |
| 11121 | COPEB | NM_001300.3 | 842 | gggtgcaccggtgccactttaac | 24544 | - | transcription_regulator | translation regulator | prostate cancer |
| 11122 | CREB3 | NM_006368.4 | 635 | cagcgtcgttgaacattctcagc | 24552 | - | transcription_regulator | - | - |
| 11123 | CREB3L3 | NM_032607.1 | 1339 | ccgcgaacctgaccaattcgacg | 24568 | - | transcription_regulator | - | - |
| 11124 | CREB3L4 | NM_130898.2 | 1103 | cagctgcagacgctaattgctca | 24573 | - | transcription_regulator | - | - |
| 11125 | CREB5 | NM_004904.1 | 361 | cggaatatctcgatgcataatgc | 24588 | - | see also 3409 line | | |
| 11126 | CREBL1 | NM_004381.3 | 1724 | ctgtgggccagctgcaactatat | 24613 | - | see also 2939 line | | |
| 11127 | CRY1 | NM_004075.2 | 1015 | tggacaaccgcctctaacttata | 24633 | - | see also 2746 line | | |
| 11128 | CSEN | NM_013434.3 | 416 | gtggacgaagacaccttcaaact | 24677 | - | transcription_regulator , signal_transduction | transcription co-repressor | - |
| 11129 | D1S155E | NM_007158.2 | 497 | ctgcgtgaaactggggttattga | 24679 | - | see also 4961 line | | |
| 11130 | DC8 | NM_015471.2 | 150 | cgggctgtgaccgaaatgctaca | 24737 | - | - | - | - |
| 11131 | DDEF2 | NM_003887.1 | 477 | tggaccggatggttctttacaaa | 24751 | - | - | - | - |
| 11132 | DDX3X | NM_001356.2 | 1975 | gggtgtccgccacactatgatgt | 24892 | - | see also 901 line | | |
| 11133 | DDX3Y | NM_004660.2 | 1143 | cagatacgtcgtatagttgaaca | 24916 | - | see also 3179 line | | |

Figure 46

| | | | | | | | | |
|---|---|---|---|---|---|---|---|---|
| 11134 | DDX48 | NM_014740.1 | 1383 | gagatatgagcagtactattcc | 24965 | - | | translation initiation factor |
| 11135 | DEAF1 | NM_021008.2 | 1304 | ccgtacggtgccggaacatcagc | 24973 | - | | DNA binding |
| 11136 | DEDD | NM_004216.2 | 807 | gcgccagtttgagccgctttaacc | 24991 | - | apoptosis, transcription_regulator | - |
| 11137 | DEDD2 | NM_133328.2 | 805 | ccgtggtttgtgacatcaagttc | 24995 | - | apoptosis, transcription_regulator | - |
| 11138 | DEK | NM_003472.2 | 811 | aaggctaagcgaaccaaatgtcc | 25010 | - | transcription_regulator, signal_transduction | specific RNA polymerase II transcription factor |  leukemia |
| 11139 | DHX9 | NM_001357.2 | 3444 | cagttggacccgtaaatgaacg | 25108 | - | see also 902 line | - |
| 11140 | DKFZp434I1930 | NM_032255.1 | 1450 | cagctccatgctcgatcaagtgg | 25122 | - | | |
| 11141 | DKFZP564I0422 | NM_031435.1 | 756 | trgtaccatataaagtctatga | 25142 | - | see also 9024 line | |
| 11142 | DKFZP564I052 | NM_015534.3 | 1205 | gggatacggataccaaacttca | 25179 | - | see also 6456 line | |
| 11143 | DMAP1 | NM_019100.3 | 575 | ccgcaaggacggagcaatgttct | 25241 | - | transcription_regulator | transcriptional repressor |
| 11144 | DNAJC1 | NM_022365.2 | 1709 | ctgtatcgctaggttacaagttgc | 25292 | - | see also 8346 line | - |
| 11145 | DNASE1L1 | NM_006730.1 | 1083 | gagacgtatgtgtacttctatcg | 25297 | - | | endonuclease |
| 11146 | DNASE1L2 | NM_001374.1 | 158 | tcgcatcggagccttcaacattc | 25304 | - | | endonuclease |
| 11147 | DNASE1L3 | NM_004944.1 | 310 | gagaggcataacgtacaactatg | 25308 | - | apoptosis | endonuclease |
| 11148 | DNASE2 | NM_001375.1 | 872 | taggcatcctgccctctaactgc | 25322 | - | see also 942 line | |
| 11149 | DNMT1 | NM_001379.1 | 4097 | tggtccgcatgggtatcagtgc | 25382 | - | see also 949 line | |
| 11150 | DNMT2 | NM_004412.3 | 868 | ctgcgatatgctcttctgttaga | 25418 | - | see also 2956 line | |
| 11151 | DNMT3B | NM_006892.3 | 623 | cagtcgtccgaactcgaaataac | 25457 | - | see also 4823 line | |
| 11152 | DNTT | NM_004088.2 | 1039 | aagggttccgagagggtaagaaga | 25518 | - | | magnesium binding |
| 11153 | DOT1L | NM_032482.1 | 106 | ctgccggtctacgataaacatca | 25534 | - | see also 9213 line | - |
| 11154 | DPF1 | NM_004647.1 | 771 | aggacaccctcgtgtttacaat | 25569 | - | apoptosis, transcription_regulator | DNA binding |
| 11155 | DR1 | NM_001938.1 | 663 | tggtgaactgctgcactgaattc | 25590 | - | see also 1236 line | - |
| 11156 | DRAP1 | NM_006442.2 | 340 | cggcggtgcctgtcatcatctcc | 25601 | - | transcription_regulator | - |
| 11157 | DRF1 | NM_025104.2 | 478 | gtgtccagccgcgagagaagtaaa | 25603 | - | kinase_related | - |
| 11158 | DRIL2 | NM_006465.1 | 563 | gttgcaccatgtccaatctact | 25627 | - | | - |

Figure 46

| | | | | | | | | | |
|---|---|---|---|---|---|---|---|---|---|
| 11159 | DRLM | NM_153757.1 | 340 | aagcgatgcgataagatagaagc | 25636 | - | - | - | - |
| 11160 | EBF | NM_024007.2 | 1146 | ttgatgggttacaggtcatattc | 25686 | - | see also 8555 line | | |
| 11161 | EBF2 | NM_022659.1 | 644 | aagggtacatccgcaacacaagc | 25703 | - | transcription_regulator | - | - |
| 11162 | ECE2 | NM_014693.1 | 1676 | atgtttatgacgggtacgaaatt | 25736 | - | signal_transduction、gpcr | zinc binding | - |
| 11163 | EDF1 | NM_003792.2 | 339 | aggtgatcgcggactatgagagc | 25748 | - | transcription_regulator | - | - |
| 11164 | EGR2 | NM_000399.2 | 656 | ctgctacccagaaggcataatca | 25757 | - | transcription_regulator | Egr1/Egr2 | hereditary motor and sensory neuropathy、neuropathy、Charcot-Marie-Tooth neuropathy |
| 11165 | EIF4A1 | NM_001416.1 | 493 | tggccgtgtgtttgatatgctta | 25763 | - | - | translation initiation factor | - |
| 11166 | EIF4A2 | NM_001967.2 | 20 | ctggtggctccgcggattataac | 25768 | - | see also 1245 line | | |
| 11167 | ELYS | NM_015446.1 | 4504 | tggaaactcctcgctcactatct | 25886 | - | see also 6421 line | | |
| 11168 | ENDOG | NM_004435.1 | 786 | ctgatgggaaatcctacgtaaag | 25940 | - | - | magnesium binding | - |
| 11169 | EP400 | NM_015409.2 | 5850 | cagccgtaccacaggtataaacc | 26016 | - | - | - | - |
| 11170 | ERCC4 | NM_005236.1 | 2056 | gtggatatgcgtgaatttcgaag | 26128 | - | see also 3671 line | | |
| 11171 | ERN1 | NM_001433.1 | 1104 | cagcacggacgtcaagtttgatc | 26174 | - | see also 1002 line | | |
| 11172 | EVI1 | NM_005241.1 | 2105 | aaggaagcaacgtcgaatcaaga | 26231 | - | see also 3673 line | | |
| 11173 | EXO1 | NM_003686.3 | 2093 | cagatgtagcacgtaattcaagt | 26333 | - | see also 2505 line | | |
| 11174 | EZH2 | NM_004456.3 | 1407 | aagcaaattctcggtgtcaaaca | 26386 | - | see also 2990 line | | |
| 11175 | FALZ | NM_004459.5 | 5919 | ttggtccttatggcattcgatct | 26534 | - | transcription_regulator | - | - |
| 11176 | FBXL11 | NM_012308.1 | 3264 | atgccacgcttcgcctcataatt | 26667 | - | transcription_regulator | - | - |
| 11177 | FCRH3 | NM_052939.2 | 463 | ttggtatcacgatgagaagttgt | 26679 | - | - | - | - |
| 11178 | FLJ10876 | NM_018254.2 | 556 | caggctcgtaaactagctaatag | 26725 | - | see also 7346 line | | |
| 11179 | FLJ12178 | NM_025134.2 | 1724 | ctggtaactcgtaactcatatga | 26796 | - | - | - | - |
| 11180 | FLJ12800 | NM_022903.2 | 1403 | taggcggtcctcggatgatgagg | 26936 | - | - | - | - |
| 11181 | FLJ13111 | NM_025082.1 | 785 | tggctcccccaacgtgtatcact | 26945 | - | - | - | - |
| 11182 | FLJ14260 | NM_025027.2 | 1212 | gtgcccttacggaccaatcttgg | 26986 | - | transcription_regulator | - | - |
| 11183 | FLJ14281 | NM_024920.3 | 2317 | ctgtacctaacacacacacataaac | 27007 | - | - | - | - |
| 11184 | FLJ20125 | NM_017676.1 | 328 | gagctcatcatggtatatccagg | 27019 | - | see also 7107 line | | |
| 11185 | FLJ20203 | NM_032292.3 | 4062 | tggatcccctgagcgtgatattt | 27098 | - | - | - | - |
| 11186 | FLJ21439 | NM_025137.2 | 2893 | gagccgtattggaggtgtaatac | 27173 | - | - | - | - |
| 11187 | FLJ23017 | NM_022840.2 | 1311 | aagggtagtaagcccattacttt | 27304 | - | see also 8492 line | | |

Figure 46

| | | | | | | | | |
|---|---|---|---|---|---|---|---|---|
| 11188 | FLJ23119 | NM_024652.2 | 2364 | gagatcaggcctgcattgtata | - | kinase_related | - | |
| 11189 | FLJ23436 | NM_024671.2 | 943 | agggccctcggcctaacactctgc | - | transcription_regulator | - | |
| 11190 | FLJ36070 | NM_182574.1 | 1206 | gagggcgcagttgcttcctaacc | - | see also 10271 line | - | |
| 11191 | FLJ38964 | NM_173527.1 | 515 | gagacgcatcatgtggataagg | - | see also 10080 line | - | |
| 11192 | FOS | NM_005252.2 | 223 | ccggggatagcctcttactac | - | transcription_regulator | zinc binding | osteosarcoma |
| 11193 | FOSB | NM_006732.1 | 1477 | ttgttaaccctcgtacacttct | - | transcription_regulator, cell_cycle | transcription factor binding | |
| 11194 | FUS | NM_004960.1 | 651 | tggcggttatggcaatcaagaacc | - | | RNA binding | soft tissue sarcoma, leukemia, myxoid liposarcoma, osteosarcoma |
| 11195 | GASC1 | NM_015061.1 | 2989 | tggcccgatgccaaactctatgg | - | see also 6215 line | | |
| 11196 | GCM1 | NM_003643.1 | 623 | gaggcacgacggacgcctttatat | - | transcription_regulator | DNA binding | |
| 11197 | GCM2 | NM_004752.1 | 588 | aagcgccatcaagagacaaatgg | - | transcription_regulator | - | |
| 11198 | GFI1 | NM_005263.1 | 563 | cagcctccggagaagtcaatgtgc | - | transcription_regulator, cell_cycle | RNA polymerase II transcription factor | |
| 11199 | GIOT-1 | NM_153257.1 | 1623 | aaggcgtttaatcatcatgattaca | - | transcription_regulator | - | |
| 11200 | GIT1 | NM_014030.1 | 719 | tggacgcaagccgatcacaaga | - | signal_transduction, g pcr | DNA binding | |
| 11201 | GIT2 | NM_014776.2 | 434 | ctgccggatgacgatagtgtga | - | signal_transduction, g pcr | - | |
| 11202 | GLI | NM_005269.1 | 997 | gggtgccggaagtcatactcacg | - | transcription_regulator, signal_transduction | RNA polymerase II transcription factor | liposarcoma, glioblastoma multiforme |
| 11203 | GLI4 | NM_138465.2 | 615 | cggcaagagtttcaagtataact | - | - | molecular_function unknown | |
| 11204 | GMEB1 | NM_006582.2 | 1644 | cggcctaacctcggccaattcagg | - | transcription_regulator | - | |
| 11205 | GMEB2 | NM_012384.2 | 610 | ggggcgacctgacggaagataac | - | transcription_regulator | RNA polymerase II transcription factor | |
| 11206 | GTF2F1 | NM_002096.1 | 190 | aggccctagcagccagaatgtca | - | transcription_regulator, kinase_related | Transcription initiation factor IIF, complex | |
| 11207 | GTF2F2 | NM_004128.1 | 173 | gtggctagtcaagttcctaaat | - | - | Transcription initiation factor IIF, complex | |

Figure 46

| | | | | | | | | | |
|---|---|---|---|---|---|---|---|---|---|
| 11208 | GTF3A | NM_002097.1 | 743 | aaggcccacgagggctatgtatg | 27769 | - | transcription_regulator | RNA polymerase III transcription factor | - |
| 11209 | GTF3C1 | NM_001520.1 | 344 | aggatgtctaccccattcatatg | 27796 | - | transcription_regulator | - | - |
| 11210 | H1F0 | NM_005318.2 | 557 | ctgactcgcagatcaagttgtcc | 27880 | - | - | DNA binding | - |
| 11211 | H1FOO | NM_153833.1 | 268 | tggacgtcctccgcttcaagtac | 27883 | - | - | - | - |
| 11212 | H1FX | NM_006026.2 | 455 | cggcgttgtccccatctaagaag | 27889 | - | - | DNA binding | - |
| 11213 | H2AFY | NM_004893.2 | 1175 | cagttacttcgtgtctacaatgt | 27903 | - | - | - | - |
| 11214 | H2AFY2 | NM_018649.1 | 1051 | cagtggggctccgacaaatgtga | 27924 | - | - | DNA binding | - |
| 11215 | H3F3B | NM_005324.3 | 439 | gggtctgttcgaagataccaacc | 27928 | - | - | DNA binding | - |
| 11216 | HBP1 | NM_012257.2 | 1005 | tggcgagtctgtactgaagttga | 27945 | - | see also 5233 line | | |
| 11217 | HBXAP | NM_016578.3 | 2429 | atgtgggtcgtactttaagaaga | 28019 | - | transcription_regulator | - | - |
| 11218 | HELLS | NM_018063.3 | 2503 | ttgttagatcgaagtgatcttat | 28127 | - | see also 7231 line | | |
| 11219 | HEMK | NM_016173.1 | 306 | gggttatccagtgccatagaact | 28136 | - | - | protein methyltransferase | - |
| 11220 | Hes4 | NM_021170.2 | 252 | gcgccgagcgcgtattaacgaga | 28150 | - | transcription_regulator | - | - |
| 11221 | HIPK1 | NM_152696.3 | 1791 | cggagggttcacatgtatgatac | 28198 | - | see also 9919 line | | |
| 11222 | HIST1H1A | NM_005325.2 | 648 | agggtgacgaagccaaagactgc | 28243 | - | - | DNA binding | - |
| 11223 | HIST1H1B | NM_005322.2 | 216 | aggagcgcaatggcctttctttg | 28245 | - | - | DNA binding | - |
| 11224 | HIST1H1C | NM_005319.3 | 313 | gggcactctggtgcaaacgaaag | 28250 | - | - | DNA binding | - |
| 11225 | HIST1H1D | NM_005320.2 | 670 | ggggaagccgaaggttacaaagg | 28260 | - | - | DNA binding | - |
| 11226 | HIST1H1E | NM_005321.2 | 178 | gtgtccgagctcattactaaagc | 28263 | - | - | DNA binding | - |
| 11227 | HIST1H1T | NM_005323.3 | 122 | aggaagccggctggcttgataag | 28267 | - | - | DNA binding | - |
| 11228 | HIST1H2A A | NM_170745.2 | 253 | cagctagcgatccgcaatgatga | 28287 | - | - | - | - |
| 11229 | HIST1H2A K | NM_003510.2 | 41 | aggctaagacccgttcttcaagg | 28291 | - | - | DNA binding | - |
| 11230 | HIST1H2B A | NM_170610.2 | 97 | aggacccgtaaggagagttattc | 28295 | - | - | - | - |
| 11231 | HIST1H2B M | NM_003521.2 | 39 | aggctccaagaaggccattaaca | 28301 | - | - | DNA binding | - |
| 11232 | HIST1H2B O | NM_003527.4 | 39 | atgcccgacccggctaaatctgc | 28302 | - | - | DNA binding | - |
| 11233 | HIST1H3C | NM_003531.2 | 55 | aagcagcttgctactaaagcagc | 28306 | - | - | DNA binding | - |
| 11234 | HIST1H4A | NM_003538.3 | 135 | gcggatctctggtctgatctacg | 28307 | - | - | DNA binding | - |
| 11235 | HIST1H4F | NM_003540.3 | 17 | aaggtggtaaaggtttaggaaag | 28311 | - | - | - | - |

Figure 46

| 11236 | HIVEP1 | NM_002114.1 | 4723 | cagctcactagtacatctttagc | 28416 | - | see also 1362 line | | |
|---|---|---|---|---|---|---|---|---|---|
| 11237 | HKR1 | NM_181786.2 | 1324 | cagggctcaagccttatgtgtgc | 28515 | - | transcription_regulator | - | - |
| 11238 | HMG2L1 | NM_005487.2 | 726 | atggtggctcccacaaatcgaaa | 28527 | - | see also 3815 line | | |
| 11239 | HMG4L | NM_178467.1 | 219 | gggaggcaagaacgatcctaatg | 28559 | - | transcription_regulator | DNA binding | - |
| 11240 | HMGN3 | NM_004242.2 | 209 | gagggcaaagatggatccaaagt | 28561 | - | - | - | - |
| 11241 | HNRPD | NM_002138.2 | 722 | aagaatcggagagtgtagataag | 28570 | - | see also 1368 line | | |
| 11242 | HNRPK | NM_002140.2 | 460 | ccgagcgcatattgagtatcagt | 28587 | - | - | - | - |
| 11243 | HNRPU | NM_004501.2 | 2334 | gggaatcgtggcggatataatag | 28630 | - | see also 3053 line | | |
| 11244 | HP1-BP74 | NM_016287.1 | 140 | ctgatccacgcggacaagttagg | 28642 | - | see also 6786 line | | |
| 11245 | HRB | NM_004504.3 | 1271 | atgcgtatacttccacaagtaat | 28693 | - | see also 3058 line | | |
| 11246 | HRBL | NM_006076.3 | 247 | cggggtcacctacgtggatatca | 28704 | - | - | - | - |
| 11247 | HSPC133 | NM_014168.1 | 333 | aagacgcattggaaatatttaat | 28718 | - | - | - | - |
| 11248 | HSPC195 | NM_016463.5 | 1479 | cagcagttgtaggaatcgaaaga | 28721 | - | kinase_related | - | - |
| 11249 | HT001 | NM_014065.2 | 444 | tgggtatccgaggactaatgagt | 28722 | - | - | - | - |
| 11250 | HTR3A | NM_000869.1 | 388 | aagccaaccaccgtatccattga | 28777 | - | channel、receptor_acitivity | serotonin-activated cation-selective channel | - |
| 11251 | IDAX | NM_025212.1 | 341 | cagactgcccgcagaatcattcc | 28800 | - | signal_transduction | - | - |
| 11252 | ILF3 | NM_004516.2 | 715 | tcgacgatgctgcgattgtgata | 28819 | - | transcription_regulator | - | - |
| 11253 | ING1 | NM_005537.3 | 1578 | gtgcgtggggctcaatcataaac | 28849 | - | cell_cycle、transcription_regulator | - | breast cancer、gastric cancer |
| 11254 | ING3 | NM_019071.2 | 379 | tggcaaaccagatatatgacttg | 28861 | - | see also 7687 line | | |
| 11255 | ING4 | NM_016162.2 | 527 | gtgcgcacaagtcctgagtatgg | 28899 | - | transcription_regulator | - | - |
| 11256 | INSM1 | NM_002196.1 | 1593 | cggccttacgcggcacatcaaca | 28903 | - | transcription_regulator | DNA binding | - |
| 11257 | IRLB | NM_005848.1 | 2308 | gagcggtgaagtacgacttatag | 28983 | - | - | - | - |
| 11258 | JADE1 | NM_024900.2 | 391 | ctgaatccggatgagtactatgt | 29110 | - | apoptosis、transcription_regulator | - | - |
| 11259 | JDP2 | NM_130469.2 | 483 | cagcccgatgccggaacaagaag | 29140 | - | see also 9546 line | | |
| 11260 | JMJ | NM_004973.2 | 3383 | atgaagcgtcgccatatagctaa | 29183 | - | see also 3457 line | | |
| 11261 | JMJD2 | NM_014663.1 | 1666 | ctggctcttcacgggattctatc | 29221 | - | see also 5842 line | | |
| 11262 | JRK | NM_003724.1 | 1449 | gggcattcggagagatttcatga | 29266 | - | - | - | - |
| 11263 | JRKL | NM_003772.2 | 811 | gtgccgcaagttcgagagtattt | 29291 | - | - | - | - |
| 11264 | KCNMA1 | NM_002247.2 | 3144 | gagcgcgacgtacttcaatgaca | 29390 | - | see also 1439 line | | |

469

EP 1 752 536 A1

Figure 46

| 11265 | KIAA0211 | NM_014630.1 | 3582 | gagccacggtcggacattgaagc | 29423 | - | see also 5798 line | | |
|---|---|---|---|---|---|---|---|---|---|
| 11266 | KIAA0222 | NM_014643.1 | 858 | gagccagttcgagcgtaagaagg | 29435 | - | see also 5815 line | | |
| 11267 | KIAA0296 | NM_014699.1 | 1802 | tggaccatcggccctataagtgc | 29468 | - | see also 5895 line | | |
| 11268 | KIAA0352 | NM_014830.1 | 1235 | ccgaaactcccgggtaactcatg | 29495 | - | see also 6040 line | | |
| 11269 | KIAA0478 | NM_014870.2 | 446 | atgatgtacacgggcaaactacc | 29511 | - | transcription_regulator | DNA binding | - |
| 11270 | KIAA0628 | NM_014789.1 | 797 | ttgagcatcagcgcgttcattca | 29551 | - | - | DNA binding | - |
| 11271 | KIAA0945 | NM_014952.1 | 2545 | ttgccgccatgtctatgacttcc | 29575 | - | - | - | - |
| 11272 | KIAA1111 | NM_015107.1 | 211 | tggcctcggtgccggtgtattgc | 29576 | - | see also 6248 line | | |
| 11273 | KIAA1196 | NM_020713.1 | 405 | cagcatctacgggctcaagtacc | 29633 | - | transcription_regulator | - | - |
| 11274 | KIAA1259 | NM_017553.1 | 4648 | gggtacaacgtgtctaaaggaat | 29740 | - | - | - | - |
| 11275 | KIAA1333 | NM_017769.2 | 958 | atgtaggggtattatctacaatt | 29761 | - | transcription_regulator | - | - |
| 11276 | KIAA1706 | NM_030636.1 | 2210 | cagtgctagccgagttctacact | 29813 | - | - | - | - |
| 11277 | KIAA1750 | NM_033512.1 | 916 | aagaggtactgagctacttaaac | 29823 | - | - | - | - |
| 11278 | KIAA1946 | NM_177454.2 | 1971 | cagattggagccgatactcaagc | 29887 | - | see also 10164 line | | |
| 11279 | KIF22 | NM_007317.1 | 99 | tggtcgctgtcggctaagcaaga | 29902 | - | - | microtubule motor | - |
| 11280 | KLF15 | NM_014079.2 | 462 | gggggcagcggcagtagcattgg | 29923 | - | transcription_regulator | translation regulator | - |
| 11281 | KLF16 | NM_031918.1 | 41 | acgtgctcatggccatctcttcg | 29927 | - | transcription_regulator | DNA binding | - |
| 11282 | KLF5 | NM_001730.2 | 1005 | ctgaatacaccggatctagatat | 29944 | - | transcription_regulator | translation regulator | - |
| 11283 | KLHL10 | NM_152467.1 | 1518 | tggaataggcgtgattgcttatg | 29997 | - | see also 9876 line | | |
| 11284 | KNSL7 | NM_020242.1 | 3942 | aagtcgaacgaacccaaactttg | 30109 | - | see also 7817 line | | |
| 11285 | LAF4 | NM_002285.1 | 2971 | atgcctcgcagtgccgattattt | 30153 | - | transcription_regulator | translation regulator | - |
| 11286 | LBR | NM_002296.2 | 1768 | ttgttcgccaccccaattacttg | 30216 | - | see also 1473 line | | |
| 11287 | LCX | NM_030625.1 | 3877 | tggctcaaacgaggtccattatg | 30359 | - | - | - | - |
| 11288 | LDB1 | NM_003893.3 | 917 | gggtgctacggagctgtactatg | 30433 | - | see also 2647 line | | |
| 11289 | LDB2 | NM_001290.1 | 394 | aggggtgaccgacctgtattaca | 30458 | - | see also 849 line | | |
| 11290 | LEF1 | NM_016269.2 | 979 | tcgagttattccggggtacataat | 30488 | - | transcription_regulator, signal_transduction | Lymphoid enhancer binding factor 1 | - |
| 11291 | LHFP | NM_005780.1 | 712 | gagtggctggaggaattcagttt | 30519 | - | - | - | - |
| 11292 | LIG3 | NM_002311.2 | 2174 | aggcggcatgatgtcaatcttcc | 30581 | - | see also 1480 line | | |
| 11293 | LIG4 | NM_002312.2 | 2712 | tcgacgccacaccgtttatttgg | 30687 | - | see also 1484 line | | |

Figure 46

| 11294 | LIN28 | NM_024674.3 | 607 | cagagcatcagccatatggtagc | 30704 | - | transcription_regulator | - | |
|---|---|---|---|---|---|---|---|---|---|
| 11295 | LOC112885 | NM_138415.1 | 1388 | ctggccatcgtgcactcttatgt | 30709 | - | transcription_regulator | - | |
| 11296 | LOC115950 | NM_138783.1 | 1360 | atgtcagccatcatctatgaaat | 30717 | - | transcription_regulator | - | |
| 11297 | LOC117584 | NM_057178.2 | 1019 | gagagtgacccggctatacaagg | 30727 | - | see also 9500 line | | |
| 11298 | LOC151871 | NM_138815.2 | 609 | gaggcttcataggcatgcttacc | 30746 | - | - | - | - |
| 11299 | LOC153222 | NM_153607.1 | 1662 | ctgagtggaaccgagatactttg | 30785 | - | see also 9997 line | | |
| 11300 | LOC221143 | NM_174928.1 | 180 | aggcgaggatgataaatataaca | 30809 | - | - | - | - |
| 11301 | LOC283248 | NM_173587.2 | 920 | aagacccgcagccgaactagtgt | 30833 | - | - | - | - |
| 11302 | LOC56959 | NM_020207.1 | 267 | atgggcagcacatgacgtatttg | 30844 | - | - | - | - |
| 11303 | LOC57117 | NM_020395.2 | 1318 | ctggccttagtcgctcagttagt | 30918 | - | transcription_regulator | - | |
| 11304 | LOC58486 | NM_021211.1 | 1343 | ttgttcgttataggtttaataag | 30961 | - | - | - | - |
| 11305 | LOC64744 | NM_022733.1 | 607 | cagcgcctgtcatggatttgttg | 31024 | - | see also 8436 line | | |
| 11306 | LOC90668 | NM_138360.1 | 1082 | cagaaacgctgccgcaagattcg | 31035 | - | - | - | - |
| 11307 | LOC93349 | NM_138402.2 | 593 | gaggaaggatctctacctaatcc | 31043 | - | - | - | - |
| 11308 | LRP1B | NM_018557.1 | 5939 | tggattgggtgtcacgtaattta | 31211 | - | see also 7512 line | | |
| 11309 | MAZ | NM_002383.1 | 761 | gagttcaagaacggctacaatct | 31550 | - | transcription_regulator | RNA polymerase II transcription factor | - |
| 11310 | MBD3 | NM_003926.5 | 347 | aagcagccggtgaccaagattac | 31558 | - | see also 2687 line | | |
| 11311 | MBD5 | NM_018328.3 | 4424 | tagaccaaggacgttcaatgttg | 31679 | - | - | - | - |
| 11312 | MCM2 | NM_004526.2 | 103 | cagcgtcggcgaggcaatgatcc | 31691 | - | phosphatase、cell_cycle、transcription_regulator | DNA dependent adenosinetriphosphatase | - |
| 11313 | MCM3 | NM_002388.3 | 567 | aagaccatagagcgacgttattc | 31723 | - | phosphatase、cell_cycle、transcription_regulator | DNA dependent adenosinetriphosphatase | - |
| 11314 | MCM3AP | NM_003906.3 | 2217 | gcgtggcatacggaaggatatca | 31802 | - | - | adenosinetriphosphatase | |
| 11315 | MCM4 | NM_005914.2 | 1834 | tggcctcactgcgtacgtaatga | 31873 | - | phosphatase、transcription_regulator | - | |
| 11316 | MCM6 | NM_005915.4 | 2229 | ctgagtactgccgaatctctaac | 31977 | - | see also 4136 line | | |

EP 1 752 536 A1

Figure 46

| 11317 | MCM7 | NM_005916.3 | 1248 | gggaaatatccctcgtagtatca | 32003 | - | phosphatase、cell_cycle、transcription_regulator | DNA dependent adenosinetriphosphatase | - |
|---|---|---|---|---|---|---|---|---|---|
| 11318 | MCM8 | NM_032485.4 | 1050 | gtggttcgtgtcagtaatataaa | 32047 | - | phosphatase、cell_cycle、transcription_regulator | - | - |
| 11319 | MCMDC1 | NM_153255.2 | 240 | atgaatagcgatcaagttacact | 32100 | - | phosphatase | - | - |
| 11320 | MECP2 | NM_004992.2 | 517 | ctgggaagtatgatgtgtatttg | 32150 | - | see also 3482 line | | |
| 11321 | MGC23143 | NM_153219.1 | 656 | ctgccggctgcgctttacagagg | 32163 | - | transcription_regulator | - | |
| 11322 | MGC23920 | NM_173825.1 | 325 | ttgcgtcgttggtcattggaagc | 32171 | - | signal_transduction | - | |
| 11323 | MGC2941 | NM_024297.1 | 242 | cagagaaacggcggagaacaatt | 32182 | - | transcription_regulator | - | |
| 11324 | MGC29463 | NM_152686.1 | 925 | ttgattatatccagactagttgt | 32202 | - | see also 9917 line | | |
| 11325 | MGC33407 | NM_178525.2 | 193 | cagtcccaacgtggtgaacaagc | 32207 | - | - | - | - |
| 11326 | MGMT | NM_002412.1 | 371 | gtgatttcttaccagcaattagc | 32214 | - | - | methylated-DNA-[protein]-cysteine S-methyltransferase | hepatocellular carcinoma、oral cavity cancers、lung cancer、ovarian cancer、CNS Tumours |
| 11327 | MHC2TA | NM_000246.1 | 1366 | ccgcgtgagacacgagtgattgc | 32223 | - | transcription_regulator | hematopoietic-associated factor 1A complex | bare lymphocyte syndrome、MHC class II deficiency |
| 11328 | MI-ER1 | NM_020948.1 | 749 | ccgcccacgtcgatgtaaatatt | 32246 | - | - | - | - |
| 11329 | MITF | NM_000248.2 | 345 | tggctatgcttacgcttaactcc | 32279 | - | see also 180 line | | |
| 11330 | MKL1 | NM_020831.2 | 1673 | gagcgccttcgagcctatcaaga | 32380 | - | transcription_regulator | - | |
| 11331 | MLL3 | NM_021230.1 | 10745 | aagattgcaggccgctcaataaa | 32628 | - | transcription_regulator | - | |
| 11332 | MLL5 | NM_018682.2 | 3593 | ctggtttcggacggactgttaat | 32803 | - | transcription_regulator | - | |
| 11333 | MLLT1 | NM_005934.2 | 391 | cggggtacgctggcttcatcatg | 32863 | - | transcription_regulator | translation regulator | leukemia |
| 11334 | MLLT6 | NM_005937.1 | 1650 | aggcatctacaccagtaataagg | 32878 | - | see also 4144 line | | |
| 11335 | MNDA | NM_002432.1 | 990 | aaggtcattaccatatctgatta | 32901 | - | transcription_regulator | translation regulator | - |
| 11336 | MRTF-B | NM_014048.3 | 3074 | aggtatgctggaccattcacact | 32971 | - | transcription_regulator | - | |

472

Figure 46

| 11337 | MUS81 | NM_025128.3 | 387 | ccgcacgcgcttcgtatttcaga | 32974 | - | - | - | - |
|---|---|---|---|---|---|---|---|---|---|
| 11338 | MUTYH | NM_012222.1 | 428 | aagctggtacgaccaagagaaac | 32991 | - | - | - | - |
| 11339 | MXI1 | NM_005962.2 | 963 | gtgccagtgtcaaactttcattc | 33015 | - | transcription_regulator | - | prostate cancer、astrocytic tumors |
| 11340 | MYB | NM_005375.1 | 829 | ctgttaacaacgactattcctat | 33030 | - | transcription_regulator | transcription factor | liposarcoma、melanoma |
| 11341 | MYCD | NM_153604.1 | 219 | cagtgccgacttggttaatatgc | 33073 | - | see also 9996 line | | |
| 11342 | MYST3 | NM_006766.1 | 1378 | cggcgctatactaatccaatagg | 33140 | - | transcription_regulator | DNA binding | - |
| 11343 | MYST4 | NM_012330.1 | 6173 | agggtcctgcacggactttaacg | 33359 | - | see also 5283 line | | |
| 11344 | N-PAC | NM_032569.2 | 335 | atgacaagaatcgacgtaattcc | 33387 | - | see also 9250 line | | |
| 11345 | NAP1L1 | NM_004537.3 | 680 | caggaacacgatgaacctattct | 33411 | - | see also 3090 line | | |
| 11346 | NAP1L2 | NM_021963.2 | 1420 | ggggaacaatccgaactgtaact | 33445 | - | - | - | - |
| 11347 | NAP1L3 | NM_004538.2 | 474 | ccgcagccgcttgtatagaaaga | 33457 | - | see also 3094 line | | |
| 11348 | NAP1L4 | NM_005969.2 | 361 | caggtgagatgtgctcacataga | 33500 | - | - | chaperone | - |
| 11349 | NCL | NM_005381.1 | 1358 | ctgcggagatcagattagtcagc | 33534 | - | - | RNA binding | - |
| 11350 | NCOR1 | NM_006311.2 | 4356 | gagcacacggccgataattgagg | 33632 | - | see also 4410 line | | |
| 11351 | NCOR2 | NM_006312.1 | 860 | aggaatcacgctcggaaacaatg | 33700 | - | transcription_regulator | transcription co-repressor | - |
| 11352 | NDN | NM_002487.2 | 466 | atgtcatcggcagctacaagaag | 33733 | - | cell_cycle、transcription_regulator | DNA binding | - |
| 11353 | NEUROD1 | NM_002500.1 | 1088 | gagatccccatagacaatattat | 33744 | - | see also 1606 line | | |
| 11354 | NEUROD4 | NM_021191.1 | 721 | aagctcgccttgagagattcagg | 33752 | - | transcription_regulator | translation regulator | - |
| 11355 | NEUROG3 | NM_020999.1 | 424 | acgaccgcgagcgcaatcgaatg | 33799 | - | transcription_regulator | translation regulator | - |
| 11356 | NIRF | NM_152306.2 | 1638 | ttggtcgtacgagagaatgtact | 33847 | - | see also 9835 line | | |
| 11357 | NONO | NM_007363.3 | 1343 | gtgctatgggcataaacaacaga | 33889 | - | - | pre-mRNA splicing factor | renal cell carcinoma |
| 11358 | NRBF-2 | NM_030759.1 | 224 | cagacgagcagaccgtttattag | 33890 | - | receptor_acitivity、transcription_regulator | - | - |
| 11359 | NRF | NM_017544.1 | 1552 | ttggcatgtcctcctatgaattt | 33930 | - | transcription_regulator | nucleic acid binding | - |
| 11360 | NRF1 | NM_005011.2 | 856 | caggcactacggaccatagttaa | 33959 | - | transcription_regulator | Nuclear respiratory factor-1 | - |
| 11361 | NRL | NM_006177.1 | 744 | ccgggagcgcgatctctacaagg | 33970 | - | transcription_regulator | specific RNA polymerase II transcription factor | - |
| 11362 | NTHL1 | NM_002528.4 | 836 | gagctgtggcacgagatcaatgg | 34178 | - | - | - | - |

Figure 46

| | | | | | | | | | |
|---|---|---|---|---|---|---|---|---|---|
| 11363 | NUCB1 | NM_006184.3 | 899 | gcgcatgcgggagcatgtgatga | 34186 | - | - | calcium ion binding | - |
| 11364 | NUCB2 | NM_005013.1 | 730 | aagactcgtcatgaagaatttaa | 34205 | - | see also 3509 line | | |
| 11365 | NUP153 | NM_005124.2 | 2050 | tcgcatcgccgaagatagattct | 34282 | - | see also 3601 line | | |
| 11366 | OASL | NM_003733.2 | 1233 | caggtgctccttagccaaatatg | 34348 | - | - | transferase | - |
| 11367 | ORC1L | NM_004153.2 | 644 | atgtggtaccgacgaatctgaaa | 34368 | - | - | E2F-4/DP-1 complex | - |
| 11368 | OSMR | NM_003999.1 | 1346 | ctgactcatcgagtttatttaat | 34457 | - | receptor_acitivity、signal_transduction | - | - |
| 11369 | PAX1 | NM_006192.1 | 411 | cggaccgtacgaggcaagtaagc | 34504 | - | see also 4280 line | | |
| 11370 | PAX5 | NM_016734.1 | 1545 | ttggctccccctactattatagc | 34521 | - | transcription_regulator | DNA binding | bladder transitional cell carcinoma、medulloblastoma |
| 11371 | PAX9 | NM_006194.1 | 801 | agggtcattacgactcatacaag | 34523 | - | transcription_regulator | DNA binding | - |
| 11372 | PC326 | NM_018442.1 | 911 | caggctcattcgacatcatctcc | 34539 | - | - | - | - |
| 11373 | PCBP3 | NM_020528.1 | 790 | caggtggtcaggcctacacaatc | 34578 | - | see also 7889 line | | |
| 11374 | PCDH1 | NM_002587.3 | 973 | gaggccgaactatctgagaatag | 34595 | - | transcription_regulator | - | - |
| 11375 | PCNA | NM_002592.2 | 526 | cggataccttggcgctagtattt | 34625 | - | cell_cycle | zinc binding | laryngeal cancer、non-hodgkin lymphoma、breast cancer、ocular melanoma、skin cancer、Hodgkin's disease |
| 11376 | PDCD2 | NM_002598.2 | 603 | cagatcatctggaccatataatt | 34643 | - | apoptosis | - | - |
| 11377 | PHF11 | NM_016119.1 | 152 | ctgccccaaagatgtcgaatata | 34679 | - | transcription_regulator | - | - |
| 11378 | PHF12 | NM_020889.1 | 1187 | gagcggaaccccacccaatttca | 34701 | - | see also 8012 line | | |
| 11379 | PHF13 | NM_153812.1 | 841 | gagtcccaccttgcaggatatcc | 34740 | - | transcription_regulator | - | - |
| 11380 | PHF14 | NM_014660.1 | 2165 | ggggatcagtacagatatctttc | 34789 | - | see also 5831 line | | |
| 11381 | PHF15 | NM_015288.4 | 1441 | aggcactggtcgacttcatctac | 34822 | - | see also 6314 line | | |
| 11382 | PHF16 | NM_014735.2 | 2174 | ccgtcctcggagtgctatcatgg | 34870 | - | see also 5924 line | | |
| 11383 | PHF3 | NM_015153.1 | 627 | cagccgaaatagcggacaaattg | 34897 | - | transcription_regulator | - | - |
| 11384 | PHF6 | NM_032335.1 | 712 | atggaaagtagttcctatagaga | 35048 | - | see also 9165 line | | |
| 11385 | PIAS3 | NM_006099.2 | 838 | ctgagttcggacggaattactcc | 35134 | - | transcription_regulator | zinc binding | - |
| 11386 | PIASY | NM_015897.2 | 308 | ccgctggcaggccccaatattga | 35155 | - | transcription_regulator | - | - |
| 11387 | PIK4CA | NM_002650.1 | 1371 | gggctatgtgcgggagtatattc | 35180 | - | kinase_related、signal_transduction | - | - |
| 11388 | PJA2 | NM_014819.1 | 815 | tagattctgtaccattagtgaaa | 35275 | - | see also 6012 line | | |

EP 1 752 536 A1

Figure 46

| 11389 | PLAC3 | NM_020318.1 | 1598 | acgcttatctcggcaatcaaaga | 35317 | - | - | - | - |
|---|---|---|---|---|---|---|---|---|---|
| 11390 | PLAGL1 | NM_002656.2 | 2876 | ctgcagatgctgtgaacctaaca | 35404 | - | cell_cycle、apoptosis、transcription_regulator | - | - |
| 11391 | PMS1 | NM_000534.2 | 2724 | gtgcgtctatccagacaattacc | 35502 | - | see also 404 line | | |
| 11392 | PMS2 | NM_000535.2 | 608 | cagcaggcatccgtgtaagttgc | 35507 | - | - | DNA binding | colorectal cancer、colorectal carcinoma、ataxia telangiectasia、Turcot syndrome with glioblastoma、cerebral astrocytoma、colorectal cancers、familial nervous system tumor syndromes |
| 11393 | POGK | NM_017542.3 | 1778 | tcgcgtggaatagcatctcaagt | 35547 | - | see also 7022 line | | |
| 11394 | POGZ | NM_015100.1 | 387 | acggttagaccaattacactagt | 35561 | - | see also 6238 line | | |
| 11395 | POLA | NM_016937.1 | 3943 | ttgcagtaacatcgattgtaagg | 35715 | - | see also 6972 line | | |
| 11396 | POLB | NM_002690.1 | 172 | cagaactcgcaaactttgagaag | 35731 | - | see also 1752 line | | |
| 11397 | POLD1 | NM_002691.1 | 2216 | ggggttcggacgtcagatgatcg | 35766 | - | - | zeta DNA polymerase | - |
| 11398 | POLD3 | NM_006591.1 | 973 | aggggggaagcgagtagcattatc | 35787 | - | see also 4617 line | | |
| 11399 | POLE2 | NM_002692.2 | 758 | tagtactactagggcatactatg | 35904 | - | - | zeta DNA polymerase | - |
| 11400 | POLE3 | NM_017443.3 | 398 | tggagttccagcggttcgttatc | 35932 | - | - | - | - |
| 11401 | POLE4 | NM_019896.2 | 320 | gagagacttggataatgcaatag | 35935 | - | - | - | - |
| 11402 | POLG | NM_002693.1 | 2685 | gaggaacgcccataaacgtatca | 35960 | - | see also 1754 line | | |
| 11403 | POLL | NM_013274.2 | 1518 | cagtgacttcctggaacgtatgc | 35980 | - | - | zeta DNA polymerase | - |
| 11404 | POLM | NM_013284.1 | 816 | aggactgcgaaccttagatgacc | 35988 | - | - | zeta DNA polymerase | - |
| 11405 | POLR1A | NM_015425.1 | 207 | cggcaaacggcctgtacgattta | 36000 | - | see also 6408 line | | |
| 11406 | POLR1B | NM_019014.2 | 3352 | gtgtagtcgcagtgacactatcg | 36171 | - | - | - | - |
| 11407 | POLR1C | NM_004875.1 | 787 | gtgcttctcacctggtgttattg | 36192 | - | transcription_regulator | - | |
| 11408 | POLR2A | NM_000937.2 | 2695 | tcgtgtccggagctaaaggttcc | 36233 | - | transcription_regulator | DNA-directed RNA polymerase III | - |
| 11409 | POLR2B | NM_000938.1 | 3538 | gagtattgcaccgcgaatgatga | 36404 | - | see also 616 line | | |
| 11410 | POLR2C | NM_002694.2 | 139 | ctggcggtggccaattcgattcg | 36407 | - | transcription_regulator | - | |
| 11411 | POLR2F | NM_021974.2 | 105 | tggcgacgactttgatgatgtgg | 36427 | - | - | DNA-directed RNA polymerase III | - |
| 11412 | POLR2K | NM_005034.2 | 182 | gtggatacagaataatgtacaag | 36435 | - | transcription_regulator | zinc binding | - |

Figure 46

| | | | | | | | | | |
|---|---|---|---|---|---|---|---|---|---|
| 11413 | POLRMT | NM_005035.2 | 1843 | ccgtgctctaccacgtgtattcc | 36439 | - | transcription_regulator | DNA-directed RNA polymerase III | - |
| 11414 | POLS | NM_006999.2 | 995 | gagcgaatagccacatgcaatgg | 36466 | - | see also 4887 line | | |
| 11415 | PPARBP | NM_004774.2 | 489 | atgttacatcacgtcagatatgt | 36486 | - | see also 3284 line | | |
| 11416 | PPARGC1 | NM_013261.2 | 1692 | ttgggatggcacgcaatcctatt | 36628 | - | see also 5378 line | | |
| 11417 | PPFIBP1 | NM_003622.2 | 417 | gagcagatggatggtatcatagc | 36644 | - | - | - | - |
| 11418 | PQBP1 | NM_005710.1 | 867 | atggaccctagctcatactcaga | 36728 | - | transcription_regulator | - | mental retardation |
| 11419 | PRDM10 | NM_020228.1 | 1031 | atgaatgtaaccgccgatttata | 36743 | - | transcription_regulator | DNA binding | - |
| 11420 | PRDM12 | NM_021619.2 | 489 | tggcacggtgcgctacttcatcg | 36781 | - | transcription_regulator | - | - |
| 11421 | PRDM13 | NM_021620.1 | 2029 | aagctgtactcgcgcaagtatgg | 36795 | - | transcription_regulator | DNA binding | - |
| 11422 | PRDM14 | NM_024504.1 | 1079 | gtgaagacctacggagacaattc | 36806 | - | transcription_regulator | DNA binding | - |
| 11423 | PRDM4 | NM_012406.2 | 1137 | gggacaggtcatgtagatgtatc | 36844 | - | transcription_regulator、signal_transduction | zinc binding | - |
| 11424 | PRDM5 | NM_018699.1 | 336 | ctggcttcgcttcgttcatgagg | 36872 | - | see also 7569 line | | |
| 11425 | PREB | NM_013388.3 | 662 | gtgtgcttcaaccacgataatac | 36921 | - | transcription_regulator | translation regulator | - |
| 11426 | PRIM2A | NM_000947.1 | 620 | tggggttcgagtccatttataag | 36946 | - | see also 629 line | | |
| 11427 | PRKCBP1 | NM_012408.3 | 1024 | caggtcgatgcccgattctttgg | 36993 | - | transcription_regulator | - | - |
| 11428 | PRKRIR | NM_004705.2 | 1969 | gagcttccgtccaccatctatga | 37082 | - | see also 3228 line | | |
| 11429 | PTPRF | NM_002840.2 | 3438 | aggttcccgactcctataagtca | 37154 | - | receptor_acitivity、phosphatase、signal_transduction | - | small cell lung cancer |
| 11430 | PYGO1 | NM_015617.1 | 1204 | cagttaatgcgtactagagaaac | 37217 | - | transcription_regulator、signal_transduction | - | - |
| 11431 | PYGO2 | NM_138300.2 | 265 | gggcaaggccggtctgcaaatga | 37219 | - | transcription_regulator、signal_transduction | - | - |
| 11432 | RAB10 | NM_016131.2 | 745 | cagaggcgcaatgggtatcatgc | 37230 | - | see also 6684 line | | |
| 11433 | RAB25 | NM_020387.1 | 926 | ttgccctttggttccagatatca | 37252 | - | signal_transduction | protein transporter | - |
| 11434 | RABGEF1 | NM_014504.1 | 1286 | ttgcttaggcgtcaagcaaatgt | 37272 | - | - | - | - |
| 11435 | RAD17 | NM_002873.1 | 1214 | gagcgacaaagtataacaagtta | 37304 | - | see also 1910 line | | |

Figure 46

| | | | | | | | | | | |
|---|---|---|---|---|---|---|---|---|---|---|
| 11436 | RAD52 | NM_002879.2 | 310 | gagggtcatcgggtaattaatct | 37341 | - | see also 1919 line | | | |
| 11437 | RAD54B | NM_012415.2 | 2139 | aagcgtcatggatatgcttatac | 37419 | - | - | - | - | |
| 11438 | RAD54L | NM_003579.2 | 2260 | ccgtgcccgaaggtacttatacg | 37475 | - | see also 2374 line | | | |
| 11439 | RAG1 | NM_000448.1 | 2411 | tggcgttccaacccttaccatga | 37534 | - | - | endonuclease | severe combined immunodeficiency disease | |
| 11440 | RAG2 | NM_000536.1 | 728 | ttgggtgtgctacatcatacatt | 37565 | - | - | endonuclease | severe combined immunodeficiency disease | |
| 11441 | RAI1 | NM_030665.3 | 6118 | cagcgatgcaggttgcatattca | 37631 | - | transcription_regulator | - | schizophrenia | |
| 11442 | RBL2 | NM_005611.2 | 2797 | cagaacattatgcgttgttatag | 37718 | - | see also 3898 line | | | |
| 11443 | RBM5 | NM_005778.1 | 279 | atgatcggaggggtgatagatat | 37737 | - | see also 4039 line | | | |
| 11444 | RBM6 | NM_005777.1 | 519 | aaggaccacctatggactatagg | 37795 | - | see also 4031 line | | | |
| 11445 | RBPSUH | NM_005349.1 | 1410 | aggtgcccctaacgaatcaaac | 37890 | - | see also 3734 line | | | |
| 11446 | RBPSUHL | NM_014276.2 | 473 | ctgagacgcagaagctgaatttc | 37905 | - | - | - | - | |
| 11447 | RECQL | NM_002907.2 | 2156 | cagcttatgctaccatttcgtat | 37991 | - | - | - | - | |
| 11448 | REPIN1 | NM_013400.1 | 936 | cgggaagcgctttaccaataagc | 37998 | - | - | - | - | |
| 11449 | REV3L | NM_002912.1 | 8827 | gaggttggcgatagtattgttca | 38249 | - | see also 1946 line | | | |
| 11450 | RFC1 | NM_002913.3 | 1273 | aagcttatcgaagctacttaaat | 38338 | - | see also 1958 line | | | |
| 11451 | RFC2 | NM_002914.3 | 477 | gtgcagtcctccggtacacaaag | 38404 | - | - | - | Williams-Beuren syndrome | |
| 11452 | RFC3 | NM_002915.2 | 120 | tgggacggctggactatcacaag | 38420 | - | - | - | - | |
| 11453 | RFC4 | NM_002916.3 | 896 | gagggaatagcttatcttgttaa | 38464 | - | see also 1963 line | | | |
| 11454 | RFC5 | NM_007370.3 | 941 | aggggttggcactgcatgatatc | 38487 | - | - | - | - | |
| 11455 | RFP | NM_006510.3 | 978 | tagacttggccatctacaatagc | 38498 | - | kinase_related、transcription_regulator | - | - | |
| 11456 | RFX2 | NM_000635.2 | 1287 | ctgcgaggcaactgtagatgtgg | 38509 | - | transcription_regulator | - | - | |
| 11457 | RFX4 | NM_002920.3 | 1090 | ctgagacggcaaacatcactaaa | 38535 | - | transcription_regulator | - | - | |
| 11458 | RFXDC1 | NM_173560.1 | 1568 | ttggtgctcgagtaatgcataat | 38598 | - | see also 10083 line | | | |
| 11459 | RPC155 | NM_007055.2 | 253 | tggggtgctcgaccataggatgg | 38641 | - | see also 4940 line | | | |
| 11460 | RREB1 | NM_002955.3 | 1727 | ctgagcctgtcacctttcgaagc | 38758 | - | transcription_regulator、signal_transduction | translation regulator | - | |
| 11461 | RUNX3 | NM_004350.1 | 377 | atgcctcggccgtcatgaagaac | 38775 | - | transcription_regulator | transcription factor | gastric cancer | |
| 11462 | SAFB2 | NM_014649.1 | 1290 | acgcgctacggatctcaagaacc | 38788 | - | - | - | - | |
| 11463 | SALL1 | NM_002968.1 | 555 | gggcaacttctccgtaatcaaca | 38817 | - | see also 1999 line | | | |
| 11464 | SALL3 | NM_171999.1 | 3852 | ccgcccattcacgcggtttatcg | 38872 | - | transcription_regulator | DNA binding | - | |
| 11465 | SALL4 | NM_020436.2 | 275 | gagacgcacgtctgtgagaaatg | 38878 | - | see also 7875 line | | | |

Figure 46

| | | | | | | | | |
|---|---|---|---|---|---|---|---|---|
| 11466 | SCOTIN | NM_016479.3 | 281 | tggtacctgtgatgaccaatact | 38917 | - | kinase_related | - |
| 11467 | SE20-4 | NM_022117.1 | 374 | gggggatccgcgcatacttcacg | 38921 | - | see also 8281 line | - |
| 11468 | SETBP1 | NM_015559.1 | 1034 | aagtcatgtccggattactatcc | 38973 | - | transcription_regulator | - |
| 11469 | SETDB1 | NM_012432.1 | 2845 | cagtgtggcggagctatgctacc | 39079 | - | see also 5321 line | - |
| 11470 | SETDB2 | NM_031915.1 | 1379 | cagcactccagtgagttataaa | 39120 | - | - | - |
| 11471 | SFPQ | NM_005066.1 | 1533 | atggcacgtttgagtacgaatat | 39201 | - | see also 3531 line | - |
| 11472 | SHPRH | NM_173082.1 | 1560 | ctgacacatctgacaagatgt | 39254 | - | see also 10050 line | - |
| 11473 | SIRT1 | NM_012238.3 | 588 | aggccacggataggtccatatac | 39380 | - | see also 5214 line | - |
| 11474 | SIRT2 | NM_012237.2 | 312 | atggacttcctgcggaacttatt | 39445 | - | cell_cycle, transcription_regulator | - |
| 11475 | SIRT3 | NM_012239.3 | 964 | aagcccgacattgtgtttttgg | 39465 | - | - | - |
| 11476 | SIRT4 | NM_012240.1 | 667 | aggtcccaacctgcgttcaatgt | 39478 | - | transcription_regulator | - |
| 11477 | SIRT5 | NM_012241.2 | 313 | ttgtccaagtcgattgatttcc | 39481 | - | transcription_regulator | - |
| 11478 | SIRT6 | NM_016539.1 | 426 | aggcttcccagggacacaactgg | 39495 | - | transcription_regulator | - |
| 11479 | SLK | NM_014720.1 | 3446 | gagctcgaagaacgacacttaca | 39571 | - | see also 5913 line | - |
| 11480 | SMAP1 | NM_021940.2 | 1327 | tggctggcatgatatcagtagt | 39614 | - | see also 8204 line | - |
| 11481 | SMARCA1 | NM_003069.2 | 1007 | tggcggatacctggtcattgatga | 39647 | - | see also 2053 line | - |
| 11482 | SMARCA2 | NM_003070.2 | 2423 | ttgttcccctttcgactctatct | 39737 | - | transcription_regulator | - |
| 11483 | SMARCA3 | NM_003071.2 | 2319 | ttgcttagactgcggcaaatttg | 39841 | - | transcription_regulator | - |
| 11484 | SMARCA5 | NM_003601.2 | 1956 | aggttcaagagtactaatcttca | 39905 | - | transcription_regulator | - |
| 11485 | SMARCAD1 | NM_020159.1 | 1924 | ctgtttcgacggctgaaacttaa | 39999 | - | transcription_regulator | - |
| 11486 | SMARCAL1 | NM_014140.2 | 856 | tgggtacaatgcggaactcatg | 40062 | - | see also 5513 line | - |
| 11487 | SMARCF1 | NM_006015.3 | 6552 | tggttcactcgccaacatctcg | 40169 | - | see also 4188 line | - |
| 11488 | SMCY | NM_004653.2 | 741 | ctgctacgatcacattacgaacg | 40209 | - | transcription_regulator / molecular_function unknown | - |
| 11489 | SMYD1 | NM_198274.1 | 628 | tggccaactgactgtcatatt | 40243 | - | - | - |
| 11490 | SNAI1 | NM_005985.2 | 131 | gagctgcaggactctaatccaga | 40262 | - | DNA binding | - |
| 11491 | SNAI2 | NM_003068.3 | 587 | tgggctggccaaacataagcagc | 40270 | - | transcription_regulator / DNA binding | - |
| 11492 | SNAPC3 | NM_003084.1 | 313 | gggcggttgtgcggccttgataaa | 40274 | - | see also 2076 line | - |

478

## Figure 46

| | | | | | | | | | |
|---|---|---|---|---|---|---|---|---|---|
| 11493 | SNX7 | NM_015976.2 | 580 | atggtggaacgctttaacgatga | 40312 | - | - | - | - |
| 11494 | SON | NM_003103.5 | 4747 | aggaactagtaagggtattgaat | 40425 | - | see also 2086 line | | |
| 11495 | SOS1 | NM_005633.2 | 1829 | tagagaggcttacgtaccatatg | 40519 | - | see also 3904 line | | |
| 11496 | SOX10 | NM_006941.3 | 1642 | gggagcagccagtatatacgaca | 40573 | - | transcription_regulator | transcription co-activator | Waardenburg-Hirschsprung disease |
| 11497 | SOX11 | NM_003108.3 | 1229 | ctgtcgctggtggataaggattt | 40577 | - | see also 2091 line | | |
| 11498 | SOX15 | NM_006942.1 | 1133 | tggctctcccactccatacaacc | 40580 | - | transcription_regulator | RNA polymerase II transcription factor | - |
| 11499 | SOX17 | NM_022454.2 | 1074 | cagcgcccttcacgtgtactacg | 40581 | - | transcription_regulator | - | - |
| 11500 | SOX18 | NM_018419.2 | 1222 | acgccagcagcgcggtctattac | 40590 | - | transcription_regulator | translation regulator | - |
| 11501 | SOX3 | NM_005634.2 | 945 | cggcgctcagagctacatgaacg | 40593 | - | transcription_regulator | DNA binding | mental retardation |
| 11502 | SOX30 | NM_007017.2 | 1616 | ctggtaccacacagaatatcatc | 40610 | - | see also 4893 line | | |
| 11503 | SOX9 | NM_000346.2 | 537 | gggcgagcccgatctgaagaagg | 40640 | - | see also 288 line | | |
| 11504 | SP1 | NM_138473.2 | 1745 | ctgccgttggctatagcaaatgc | 40671 | - | see also 9626 line | | |
| 11505 | SP100 | NM_003113.2 | 1147 | ttgctcacgaccccagattgtac | 40697 | - | transcription_regulator | STAT-1 alpha | - |
| 11506 | SP110 | NM_004509.2 | 837 | ttgggctctatgccagagataag | 40725 | - | transcription_regulator | - | - |
| 11507 | SP2 | NM_003110.3 | 1538 | gagcgtccctgccaaagattgc | 40754 | - | see also 2092 line | | |
| 11508 | SP3 | NM_003111.1 | 1179 | ttgcctgttacgatagatagtac | 40767 | - | - | - | - |
| 11509 | SP7 | NM_152860.1 | 777 | ttgccccaagatgtctataaacc | 40833 | - | transcription_regulator | - | - |
| 11510 | SP8 | NM_182700.2 | 143 | atgcttgccgctacctgtaataa | 40835 | - | - | - | - |
| 11511 | SPATA13 | NM_153023.1 | 1756 | tagaccgaagctcagaattgatt | 40855 | - | - | - | - |
| 11512 | SPG3A | NM_015915.2 | 1087 | tagtcccgagagcctagatatta | 40891 | - | see also 6570 line | | |
| 11513 | SPO11 | NM_012444.2 | 536 | ctgtcgtcgacaatattatcaat | 40928 | - | - | hydrolase | - |
| 11514 | SRCAP | NM_006662.1 | 6671 | ctgtcatatccgcccatcaaact | 41032 | - | transcription_regulator | - | - |
| 11515 | SREBF2 | NM_004599.2 | 1159 | aaggcggacaacccataatatca | 41059 | - | transcription_regulator | translation regulator | - |
| 11516 | SRISNF2L | NM_015106.1 | 2092 | gagggagcggcttattaatcagt | 41142 | - | see also 6244 line | | |
| 11517 | SRY | NM_003140.1 | 165 | ctgctatgttaagcgtattcaac | 41177 | - | transcription_regulator | translation regulator | gonadal dysgenesis、 prostate cance、 sex reversal with partial ovarian function |
| 11518 | SSA1 | NM_003141.2 | 632 | taggattcacgcagagtttgtgc | 41197 | - | - | zinc binding | Sjogren syndrome、 systemic lupus erythematosis |
| 11519 | SSRP1 | NM_003146.2 | 1403 | ttgctcgtggtaccactactact | 41226 | - | see also 2127 line | | |

Figure 46

| 11520 | SUHW2 | NM_080764.2 | 1273 | atgaaagtcctcgtgtatcaaag | 41257 | - | transcription_regulator | - | |
|---|---|---|---|---|---|---|---|---|---|
| 11521 | SYCP1 | NM_003176.2 | 3066 | gaggaccgttgggctgtaattgc | 41322 | - | - | DNA binding | - |
| 11522 | SYCP2 | NM_014258.2 | 2832 | gtgcggataggtcaattagattg | 41408 | - | - | - | - |
| 11523 | TAF1 | NM_004606.2 | 5574 | cggtgggtatgaggtatcagagg | 41477 | - | transcription_regulator、kinase_related、cell_cycle | - | - |
| 11524 | TAF11 | NM_005643.2 | 651 | aagccgttagaaggttaaagtca | 41494 | - | see also 3916 line | | |
| 11525 | TAF12 | NM_005644.2 | 249 | aaggctccatggccaatagtact | 41497 | - | see also 3917 line | | |
| 11526 | TAF6 | NM_005641.2 | 748 | ttgactacgccttgaagctaaag | 41513 | - | transcription_regulator | - | - |
| 11527 | TAF6L | NM_006473.2 | 757 | tggcacggagcctatttcgtaat | 41555 | - | - | - | - |
| 11528 | TAF9L | NM_015975.3 | 536 | gtgctgttagtagcaaacctact | 41585 | - | transcription_regulator | - | - |
| 11529 | TAL1 | NM_003189.1 | 1131 | aagttgtgcggcgtatcttcacc | 41589 | - | transcription_regulator | translation regulator | lymphoma、leukemia |
| 11530 | TAL2 | NM_005421.1 | 267 | cagaactctgcttgagaactacc | 41590 | - | transcription_regulator | protein binding | leukemia |
| 11531 | TBP | NM_003194.2 | 789 | gtgcccgaaacgccgaatataat | 41598 | - | transcription_regulator | TFIID complex | - |
| 11532 | TBPL1 | NM_004865.2 | 691 | cagttacgaacctgaacttcatc | 41623 | - | see also 3383 line | | |
| 11533 | TBRG1 | NM_032811.1 | 536 | cagctctgtgggaactatacagg | 41630 | - | - | - | - |
| 11534 | TCF12 | NM_003205.2 | 1942 | gagaggcggatggctaacaatgc | 41687 | - | see also 2171 line | | |
| 11535 | TCF20 | NM_005650.1 | 2240 | tggaagtctgcgctatagttaca | 41731 | - | see also 3919 line | | |
| 11536 | TCF21 | NM_003206.2 | 761 | gtgaccgcgagccgcttatgtgg | 41817 | - | transcription_regulator | RNA polymerase II transcription factor | - |
| 11537 | TCF4 | NM_003199.1 | 2170 | gggagacgcatcgaatcacatgg | 41851 | - | see also 2161 line | | |
| 11538 | TCF7 | NM_003202.1 | 303 | ctgacctctctggcttctactcc | 41855 | - | transcription_regulator、signal_transduction | T-cell factor 1G | - |
| 11539 | TCFL1 | NM_005997.1 | 668 | gggcccataatcacctatcattc | 41865 | - | transcription_regulator | RNA polymerase II transcription factor | - |
| 11540 | THAP11 | NM_020457.2 | 265 | gcgtgccaggctgctacaacaac | 41910 | - | - | - | - |
| 11541 | THAP6 | NM_144721.2 | 498 | tcggcgagctagaggatacaaag | 41929 | - | - | - | - |
| 11542 | THAP7 | NM_030573.1 | 342 | cggcatccgagtacatctacttc | 41937 | - | see also 8890 line | | |
| 11543 | TIAL1 | NM_003252.2 | 820 | tcggtgaacggtactacgattga | 41961 | - | see also 2195 line | | |
| 11544 | TIF1 | NM_003852.2 | 1822 | ttggttcacctatgatcgatttg | 42006 | - | see also 2616 line | | |
| 11545 | TIGD4 | NM_145720.2 | 1560 | atgacctccgatgtatttgaaca | 42086 | - | - | - | - |

EP 1 752 536 A1

Figure 46

| | | | | | | | | | |
|---|---|---|---|---|---|---|---|---|---|
| 11546 | TIGD5 | NM_032862.2 | 180 | gggcactcagcgcaagaagatgc | 42105 | - | - | - | - |
| 11547 | TIGD6 | NM_030953.2 | 645 | gagatcgccacggaattgctttg | 42124 | - | - | - | - |
| 11548 | TIGD7 | NM_033208.2 | 1645 | gaggacgtcagggcattgttact | 42182 | - | - | - | - |
| 11549 | TIMELESS | NM_003920.1 | 2690 | ttggcccacctgaatactgttcc | 42253 | - | see also 2682 line | | |
| 11550 | TMF1 | NM_007114.1 | 131 | atggagagccgggaataagttcc | 42267 | - | transcription_regulator | transcription cofactor | - |
| 11551 | TMX2 | NM_015959.1 | 106 | atggctcgcccaaccttactacc | 42353 | - | - | - | - |
| 11552 | TNFAIP3 | NM_006290.2 | 1015 | tgggaccatggcacaactcatct | 42391 | - | see also 4384 line | | |
| 11553 | TNP1 | NM_003284.2 | 30 | atgtcgaccagccgcaaattaaa | 42402 | - | transcription_regulator | DNA binding | - |
| 11554 | TNXB | NM_019105.4 | 11684 | gggcggacagcttcaaagtctcc | 42440 | - | see also 7710 line | | |
| 11555 | TOP1MT | NM_052963.1 | 1160 | aagccggtgtacaagaacttaca | 42501 | - | - | DNA topoisomerase I | - |
| 11556 | TOP2A | NM_001067.2 | 2525 | ctgctagtccacgatacatcttt | 42581 | - | see also 653 line | | |
| 11557 | TOP2B | NM_001068.2 | 1011 | aggtggacggcacgtggattatg | 42675 | - | see also 656 line | | |
| 11558 | TOP3A | NM_004618.2 | 746 | gtgttgcgagcccgattctctga | 42780 | - | see also 3135 line | | |
| 11559 | TOP3B | NM_003935.3 | 2066 | tggtgcacggctactataagatt | 42843 | - | - | DNA topoisomerase I | - |
| 11560 | TOX | NM_014729.1 | 897 | aaggcgatgatacctctaagatc | 42863 | - | see also 5919 line | | |
| 11561 | TRERF1 | NM_018415.1 | 3313 | gtgggtactgttcggtaaagagc | 42907 | - | see also 7454 line | | |
| 11562 | TRIM26 | NM_003449.2 | 974 | aggacacgagagacttcctaaac | 42920 | - | - | zinc binding | - |
| 11563 | TRIM33 | NM_015906.2 | 3158 | tggccgatgtccgtttgatcttc | 43004 | - | transcription_regulator | - | thyroid carcinoma |
| 11564 | TRPV6 | NM_018646.2 | 1599 | ctggtgcaacgtcatgtacttcg | 43020 | - | receptor_acitivity、cha nnel | - | - |
| 11565 | TSG101 | NM_006292.2 | 943 | atggttacccgtttagatcaaga | 43049 | - | see also 4386 line | | |
| 11566 | TSN | NM_004622.2 | 812 | aagcgctacgacggattgaaata | 43072 | - | - | DNA binding | - |
| 11567 | TSNAX | NM_005999.2 | 501 | cagttccatcgagccattactac | 43088 | - | see also 4185 line | | |
| 11568 | TSPYL3 | NM_178465.1 | 625 | atgatcagccctcgagatgaaga | 43115 | - | - | - | - |
| 11569 | TSPYL4 | NM_021648.3 | 182 | atgcagcgcagaagtttcattat | 43118 | - | - | - | - |
| 11570 | TTF2 | NM_003594.3 | 1758 | tggttactatggcgagaaagtca | 43150 | - | transcription_regulator 、phosphatase | - | - |
| 11571 | UBTF | NM_014233.1 | 2065 | caggaccgtgcagcatataaaga | 43212 | - | see also 5550 line | | |
| 11572 | UPLC1 | NM_017707.2 | 273 | ccgagaggccgtggaatccttag | 43218 | - | - | - | - |
| 11573 | USF1 | NM_007122.2 | 449 | cagctgctgagacgcactatact | 43236 | - | see also 4956 line | | |
| 11574 | VAT1 | NM_006373.2 | 556 | ctgccttgctcgtcaattacatt | 43254 | - | - | zinc binding | - |
| 11575 | WBSCR14 | NM_032951.1 | 2582 | agggcacccttggcaaaccttta | 43272 | - | transcription_regulator | - | |

Figure 46

| | | | | | | | | | | |
|---|---|---|---|---|---|---|---|---|---|---|
| 11576 | WDHD1 | NM_007086.1 | 894 | tggtcgaatatcgtatactgatg | 43302 | - | see also 4947 line | | | |
| 11577 | WDTC1 | NM_015023.2 | 1130 | cggtgcagcccagtattacgtag | 43397 | - | see also 6189 line | | | |
| 11578 | WHSC1 | NM_007331.1 | 2262 | ctgtctgatgcatgtaaaccact | 43448 | - | transcription_regulator | - | | Wolf-Hirschhorn syndrome |
| 11579 | WHSC1L1 | NM_017778.2 | 1494 | cgggtacgagagtataaaggtca | 43519 | - | transcription_regulator | - | | - |
| 11580 | WRN | NM_000553.2 | 1826 | atgggacccaacacttgatcatt | 43646 | - | - | DNA helicase | | - |
| 11581 | WRNIP1 | NM_020135.2 | 1298 | gagccgctgtcgagtgattgttc | 43754 | - | - | - | | - |
| 11582 | YBX2 | NM_015982.1 | 369 | atggttacggattcatcaacagg | 43774 | - | - | - | | - |
| 11583 | ZBED3 | NM_032367.1 | 918 | gggacggctgcgtcatcacaaag | 43778 | - | - | - | | - |
| 11584 | ZBED4 | NM_014838.1 | 1087 | ccgtcccccgatcgaataacaga | 43793 | - | - | DNA binding | | - |
| 11585 | ZBTB2 | NM_020861.1 | 215 | cggttgcaatcggcgatgtatac | 43831 | - | transcription_regulator | - | | |
| 11586 | ZBTB3 | NM_024784.2 | 781 | cagatgagccaccaatgtctagt | 43873 | - | see also 8746 line | | | |
| 11587 | ZF | NM_021212.1 | 498 | cggaggatatggactttctgtct | 43884 | - | transcription_regulator | - | | - |
| 11588 | ZFD25 | NM_016220.2 | 2395 | cagttctcatctcttaatataca | 43891 | - | transcription_regulator | molecular_function unknown | | - |
| 11589 | ZFP161 | NM_003409.2 | 695 | aagttcggaaggtcaattgctac | 43912 | - | transcription_regulator | protein binding | | - |
| 11590 | ZFP28 | NM_020828.1 | 219 | aggaatggcctcgcatccaaagg | 43922 | - | transcription_regulator | - | | - |
| 11591 | ZFP36 | NM_003407.1 | 999 | cggcgactccccatcttcaatcg | 43954 | - | - | DNA binding | | rheumatoid arthritis |
| 11592 | ZFP64 | NM_018197.2 | 827 | aaggacatggagcggcatttaaa | 43964 | - | see also 7313 line | | | |
| 11593 | ZFX | NM_003410.1 | 769 | ctgactaccgacgtagtttcaga | 43987 | - | transcription_regulator | translation regulator | | - |
| 11594 | ZFY | NM_003411.2 | 2320 | atgtggctgtccacaaaggtaaa | 43999 | - | transcription_regulator | translation regulator | | - |
| 11595 | ZIC2 | NM_007129.2 | 1280 | ccgccagctccggctatgagtcg | 44003 | - | - | DNA binding | | holoprosencephaly |
| 11596 | ZIC3 | NM_003413.2 | 1141 | cggcgctcagtttcctaactaca | 44004 | - | see also 2308 line | | | |
| 11597 | ZIC4 | NM_032153.2 | 907 | gggaaggtctttgctagatcaga | 44018 | - | - | - | | - |
| 11598 | ZIC5 | NM_033132.2 | 2219 | aagttgtgcggacgatacattag | 44034 | - | see also 9376 line | | | |
| 11599 | ZIM3 | NM_052882.1 | 739 | tagaatgtgacggatctaagaaa | 44037 | - | - | DNA binding | | - |
| 11600 | ZNF10 | NM_015394.4 | 1833 | atgtgggacagcgcttgttaata | 44090 | - | transcription_regulator | DNA binding | | - |
| 11601 | ZNF124 | NM_003431.1 | 1329 | tcgtgctagtaccctttggaagc | 44108 | - | transcription_regulator | transcription factor | | - |
| 11602 | ZNF136 | NM_003437.2 | 970 | ttgttccccaaccttacgaatac | 44120 | - | transcription_regulator | transcription co-repressor | | |

482

Figure 46

| 11603 | ZNF14 | NM_021030.2 | 1114 | aagcttcgagcacatgtaataa | 44136 | - | transcription_regulator | DNA binding | - |
| 11604 | ZNF141 | NM_003441.1 | 1379 | cagatcggagtcaacataagaaa | 44145 | - | transcription_regulator | specific RNA polymerase II transcription factor | - |
| 11605 | ZNF143 | NM_003442.3 | 1119 | gcggtcggtccttacaacatca | 44177 | - | transcription_regulator | translation regulator | - |
| 11606 | ZNF146 | NM_007145.1 | 1439 | cagcgaacatcacttattgtaca | 44209 | - | transcription_regulator | zinc binding | - |
| 11607 | ZNF151 | NM_003443.1 | 1989 | tggcttcaaccgggtagacaacc | 44285 | - | transcription_regulator | transcription factor | - |
| 11608 | ZNF16 | NM_006958.2 | 1476 | aagcctcacgtgtgtaatgtatg | 44300 | - | - | DNA binding | - |
| 11609 | ZNF160 | NM_033288.2 | 786 | acgcgacagaagagacatagaaa | 44312 | - | transcription_regulator | - | - |
| 11610 | ZNF177 | NM_003451.1 | 1365 | aagcactaaccttataatgcaca | 44330 | - | transcription_regulator | DNA binding | - |
| 11611 | ZNF180 | NM_013256.1 | 1084 | tggatttagtgaccgtattcaat | 44339 | - | transcription_regulator | DNA binding | - |
| 11612 | ZNF184 | NM_007149.1 | 685 | ttgctagaaagttgggaatatga | 44355 | - | see also 4960 line | - | - |
| 11613 | ZNF195 | NM_007152.1 | 1007 | atgccggaggggaacattacaga | 44381 | - | transcription_regulator | zinc binding | - |
| 11614 | ZNF20 | NM_021143.1 | 1571 | tggtaaggcctttattccaatt | 44390 | - | - | - | - |
| 11615 | ZNF214 | NM_013249.1 | 853 | cagtatgttggggtgatatgtca | 44407 | - | - | zinc binding | - |
| 11616 | ZNF216 | NM_006007.1 | 770 | ttggtcttacagggtttgactgc | 44432 | - | - | - | - |
| 11617 | ZNF22 | NM_006963.1 | 183 | aagtgggggcatgactattcgatt | 44434 | - | transcription_regulator | zinc binding | - |
| 11618 | ZNF221 | NM_013359.1 | 1104 | gtgggaaaggcttccatagtaga | 44442 | - | transcription_regulator | DNA binding | - |
| 11619 | ZNF222 | NM_013360.1 | 452 | atgacaaccctccagattaaa | 44446 | - | transcription_regulator | DNA binding | - |
| 11620 | ZNF223 | NM_013361.2 | 590 | aggcctcaagactctaccataaa | 44452 | - | transcription_regulator | DNA binding | - |
| 11621 | ZNF224 | NM_013398.1 | 451 | aagcattccacagggatactttc | 44460 | - | transcription_regulator | DNA binding | - |
| 11622 | ZNF225 | NM_013362.1 | 537 | aaggcttgttcagtcatcaaacc | 44465 | - | transcription_regulator | DNA binding | - |
| 11623 | ZNF226 | NM_015919.2 | 297 | aagatgtatcaacctatagaaaga | 44475 | - | transcription_regulator | - | - |
| 11624 | ZNF228 | NM_013380.2 | 1144 | tagcttagaacctaatagtatt | 44521 | - | transcription_regulator | DNA binding | - |

Figure 46

| | | | | | | | | | |
|---|---|---|---|---|---|---|---|---|---|
| 11625 | ZNF23 | NM_145911.1 | 2185 | cagcgttaatgggaaactaatgc | 44557 | - | transcription_regulator | DNA binding | - |
| 11626 | ZNF230 | NM_006300.1 | 1506 | ttgtacaccggtctttctgtaaa | 44568 | - | transcription_regulator | DNA binding | - |
| 11627 | ZNF234 | NM_006630.1 | 1217 | ttgtagctcaaaccttcgtatcc | 44580 | - | transcription_regulator | DNA binding | - |
| 11628 | ZNF235 | NM_004234.3 | 1183 | atgctcatttgcctattcacaca | 44607 | - | transcription_regulator | DNA binding | - |
| 11629 | ZNF239 | NM_005674.1 | 929 | cagacgtctctttgtaatggaag | 44632 | - | transcription_regulator | RNA binding | - |
| 11630 | ZNF25 | NM_145011.2 | 120 | gggacccgtgacattaaaggatg | 44643 | - | transcription_regulator | DNA binding | - |
| 11631 | ZNF258 | NM_007167.1 | 395 | agggccaaactgcatatcataag | 44656 | - | - | - | - |
| 11632 | ZNF26 | NM_019591.2 | 1360 | ttgttcacatccgaatgcataca | 44708 | - | transcription_regulator | DNA binding | - |
| 11633 | ZNF261 | NM_005096.1 | 4141 | ctggctgtgcgcgagatttatga | 44784 | - | see also 3559 line | | |
| 11634 | ZNF262 | NM_005095.1 | 2669 | caggcacgttcccgaacaagacg | 44828 | - | - | - | - |
| 11635 | ZNF264 | NM_003417.1 | 1347 | aggctttctccggcactatgttg | 44866 | - | - | DNA binding | - |
| 11636 | ZNF266 | NM_006631.2 | 1647 | gagcaacgttctgtatttagtca | 44876 | - | transcription_regulator | DNA binding | - |
| 11637 | ZNF267 | NM_003414.3 | 1113 | gtggggatagcttaaaccatagt | 44907 | - | transcription_regulator | DNA binding | - |
| 11638 | ZNF271 | NM_006629.3 | 1654 | tagtcggcgttctgatctcatta | 44930 | - | see also 4686 line | | |
| 11639 | ZNF278 | NM_014323.2 | 1745 | aagatcttccgtgatgtgtatca | 44947 | - | transcription_regulator | - | Ewing's sarcoma |
| 11640 | ZNF282 | NM_003575.1 | 1256 | atggggaccacgcgactcaatgg | 44965 | - | transcription_regulator | - | - |
| 11641 | ZNF286 | NM_020652.1 | 739 | ctgacacggaatagtgtctatga | 44972 | - | transcription_regulator | DNA binding | - |
| 11642 | ZNF288 | NM_015642.1 | 2162 | gagggcataccagtgtagtatct | 45010 | - | see also 6532 line | | |
| 11643 | ZNF289 | NM_032389.2 | 1206 | aagcatccggcctatttcagaaa | 45031 | - | - | DNA binding | - |
| 11644 | ZNF297 | NM_005453.3 | 1572 | ggggacgggaataagatctttct | 45053 | - | see also 3800 line | | |
| 11645 | ZNF297B | NM_014007.1 | 2338 | tggggtctgcggtaagaaattca | 45082 | - | see also 5452 line | | |
| 11646 | ZNF300 | NM_052860.1 | 860 | ttgacctaccgagttgttataag | 45101 | - | transcription_regulator | DNA binding | - |
| 11647 | ZNF302 | NM_018443.1 | 594 | cagaatgcgacacatttagaagc | 45113 | - | transcription_regulator | - | - |
| 11648 | ZNF304 | NM_020657.1 | 732 | atggaggggcctcatttgtgaag | 45123 | - | transcription_regulator | - | - |

Figure 46

| 11649 | ZNF317 | NM_020933.2 | 1646 | aagctttcagcgcgagttcaaac | 45151 | - | transcription_regulator | DNA binding | - |
| 11650 | ZNF32 | NM_006973.1 | 389 | aggtagtctaacgttacatgaga | 45162 | - | transcription_regulator | DNA binding | - |
| 11651 | ZNF325 | NM_016265.1 | 1138 | atggggaaccttatactctaaat | 45175 | - | transcription_regulator | DNA binding | - |
| 11652 | ZNF331 | NM_018555.3 | 682 | tcgccgacgtagccatagacttt | 45178 | - | transcription_regulator | - | - |
| 11653 | ZNF333 | NM_032433.1 | 1929 | gtgccgggagaccctatcaatgt | 45222 | - | transcription_regulator | DNA binding | - |
| 11654 | ZNF334 | NM_018102.3 | 1262 | aagaaatcgtatcttgttgtaca | 45245 | - | transcription_regulator | DNA binding | - |
| 11655 | ZNF335 | NM_022095.3 | 1075 | gaggaggatagcgactataatcc | 45259 | - | see also 8267 line | | |
| 11656 | ZNF336 | NM_022482.3 | 213 | tggtactaggactaatgtctact | 45283 | - | see also 8409 line | | |
| 11657 | ZNF337 | NM_015655.2 | 361 | aggcccctgtgcaggaatatatg | 45311 | - | transcription_regulator | DNA binding | - |
| 11658 | ZNF339 | NM_021220.2 | 752 | caggcattcgtccctacaaatgc | 45351 | - | transcription_regulator | - | - |
| 11659 | ZNF341 | NM_032819.3 | 918 | aagacccgacgagctaaagctgc | 45364 | - | transcription_regulator | DNA binding | - |
| 11660 | ZNF345 | NM_003419.2 | 1768 | tgggagggattcagagtttcagc | 45386 | - | transcription_regulator | specific RNA polymerase II transcription factor | - |
| 11661 | ZNF347 | NM_032584.1 | 2526 | atgtaacgtgtggaaagttctaa | 45400 | - | transcription_regulator | - | - |
| 11662 | ZNF354A | NM_005649.2 | 702 | cagcccaaagtcagtgcttatta | 45408 | - | transcription_regulator | translation regulator | - |
| 11663 | ZNF366 | NM_152625.1 | 579 | gtgcaacgtgggcttccaattct | 45419 | - | transcription_regulator | - | - |
| 11664 | ZNF382 | NM_032825.2 | 1738 | tagaaaccacgggaattcagtaa | 45448 | - | - | - | - |
| 11665 | ZNF384 | NM_133476.2 | 534 | aagcgtccgttacccagaatatc | 45451 | - | transcription_regulator | - | - |
| 11666 | ZNF398 | NM_020781.2 | 1351 | ttggcgagcaggtgttctcatgc | 45475 | - | see also 7966 line | | |
| 11667 | ZNF408 | NM_024741.1 | 955 | aggaccgaatttccaaggatagc | 45492 | - | transcription_regulator | DNA binding | - |
| 11668 | ZNF409 | NM_014894.1 | 2075 | gtgcagctacgagacaaacatct | 45511 | - | - | - | - |
| 11669 | ZNF43 | NM_003423.1 | 2467 | ccgaccctcaaaccttattgaac | 45521 | - | transcription_regulator | DNA binding | - |
| 11670 | ZNF430 | NM_025189.2 | 404 | gagacatgcgatggtagatcaac | 45522 | - | transcription_regulator | - | - |

Figure 46

| | | | | | | | | | |
|---|---|---|---|---|---|---|---|---|---|
| 11671 | ZNF431 | NM_133473.1 | 537 | aggctccgcaagtgtagatgagt | 45524 | - | transcription_regulator | - | |
| 11672 | ZNF432 | NM_014650.1 | 1699 | gtgaatagcggactgatgttaca | 45551 | - | - | DNA binding | - |
| 11673 | ZNF433 | NM_152602.1 | 1971 | atgtgcctcgcgacttcaaatgc | 45566 | - | transcription_regulator | - | |
| 11674 | ZNF436 | NM_030634.1 | 779 | caggcatccgctatcatatatgt | 45576 | - | transcription_regulator | DNA binding | - |
| 11675 | ZNF44 | NM_016264.1 | 1187 | tggttcagcacgaattcatgaag | 45594 | - | transcription_regulator | - | |
| 11676 | ZNF441 | NM_152355.1 | 2210 | ttgtatcagttcctttcataaac | 45603 | - | transcription_regulator | - | |
| 11677 | ZNF442 | NM_030824.1 | 884 | cagccagatagtactgtgaatga | 45606 | - | transcription_regulator | - | - |
| 11678 | ZNF450 | NM_014797.1 | 1348 | aagagccattaccgagttcatac | 45632 | - | see also 5992 line | | |
| 11679 | ZNF481 | NM_020924.2 | 1025 | gcgtgtgcatgccggaattaaac | 45676 | - | see also 8031 line | | |
| 11680 | ZNF482 | NM_006626.3 | 1217 | cagtggggatcggccatacaaat | 45713 | - | transcription_regulator | - | - |
| 11681 | ZNF490 | NM_020714.1 | 608 | atgcatcaggtctcccttaatag | 45718 | - | transcription_regulator | DNA binding | - |
| 11682 | ZNF510 | NM_014930.1 | 1270 | gagaacccattggtaagtaatga | 45758 | - | - | DNA binding | - |
| 11683 | ZNF6 | NM_021998.2 | 1312 | gagtacaccagtggacattcagt | 45789 | - | see also 8241 line | | |
| 11684 | ZNF7 | NM_003416.1 | 2170 | gtggcgttcacacctaattatac | 45877 | - | transcription_regulator | DNA binding | - |
| 11685 | ZNF71 | NM_021216.3 | 521 | ccgtaaggcgtggcaagaacttc | 45883 | - | transcription_regulator | - | |
| 11686 | ZNF74 | NM_003426.1 | 1922 | cagccgggagaaggcttacaagt | 45888 | - | transcription_regulator | RNA binding | - |
| 11687 | ZNF76 | NM_003427.3 | 1185 | ccgagtactcgagcttgtataag | 45903 | - | see also 2314 line | | |
| 11688 | ZNF79 | NM_007135.1 | 1606 | gagtaccaatctcataatccacc | 45914 | - | transcription_regulator | molecular_function unknown | - |
| 11689 | ZNF8 | NM_021089.1 | 717 | gaggggagtatttgtatacttac | 45918 | - | transcription_regulator | - | |
| 11690 | ZNF84 | NM_003428.1 | 1591 | gagaggtcgagtctcattaatca | 45944 | - | transcription_regulator | zinc binding | - |
| 11691 | ZNFN1A1 | NM_006060.2 | 745 | aggacgcactccgttggtaaacc | 45959 | - | - | translation regulator | - |
| 11692 | ZNFN1A2 | NM_016260.1 | 812 | aaggaacgctgccacaactatct | 45974 | - | see also 6775 line | | |
| 11693 | ZT86 | NM_015871.2 | 257 | gcgcgaggtacttcatcgattcc | 46003 | - | see also 6547 line | | |
| 11694 | ZXDA | NM_007156.1 | 2072 | tggatgaggtctcaagtgtaagt | 46007 | - | - | molecular_function unknown | - |

EP 1 752 536 A1

Figure 46

| | | | | | | | | | |
|---|---|---|---|---|---|---|---|---|---|
| 11695 | BCAS2 | NM_005872.1 | 289 | ttgctcagtatgaaacgatatga | 46018 | - | - | - | - |
| 11696 | C20orf14 | NM_012469.2 | 954 | aaggctacctgacggatttaaat | 46049 | - | - | - | - |
| 11697 | DDX23 | NM_004818.1 | 1053 | aagactccgcaaacttcgtaaga | 46086 | - | - | - | - |
| 11698 | DHX38 | NM_014003.2 | 2577 | ttgacggcatcatgtttgttatc | 46137 | - | - | pre-mRNA splicing factor | - |
| 11699 | DHX8 | NM_004941.1 | 1995 | aggtgggctacaccattcgattt | 46193 | - | - | pre-mRNA splicing factor | - |
| 11700 | FUSIP1 | NM_054016.1 | 765 | aagaaccacctagatccaaatct | 46247 | - | - | - | - |
| 11701 | HPRP8BP | NM_004814.1 | 266 | caggatttgaccgactgatatta | 46254 | - | - | - | - |
| 11702 | KHSRP | NM_003685.1 | 1149 | tggcgtgcggatacagttcaagc | 46285 | - | transcription_regulator | - | - |
| 11703 | LSM1 | NM_014462.1 | 321 | cagactgtggagcgtattcatgt | 46305 | - | - | pre-mRNA splicing factor | - |
| 11704 | LSM10 | NM_032881.1 | 263 | tggcccacggacgcatagacaat | 46311 | - | - | pre-mRNA splicing factor | - |
| 11705 | LSM11 | NM_173491.1 | 1015 | caggtattcactcgacacataaa | 46327 | - | see also 10071 line | | |
| 11706 | LSM2 | NM_021177.3 | 250 | atgtggtcgtggaactaaagaat | 46331 | - | - | pre-mRNA splicing factor | - |
| 11707 | LSM3 | NM_014463.1 | 98 | cagcctagatgagcgaatttatg | 46333 | - | see also 5694 line | | |
| 11708 | LSM5 | NM_012322.1 | 60 | atgtataggatcaagaattcaca | 46347 | - | - | pre-mRNA splicing factor | - |
| 11709 | LSM6 | NM_007080.1 | 276 | ccgaggaaacaatgtgttgtaca | 46353 | - | - | pre-mRNA splicing factor | - |
| 11710 | LSM7 | NM_016199.1 | 240 | gggcacgtccgtggtgctaatct | 46354 | - | - | pre-mRNA splicing factor | - |
| 11711 | LSM8 | NM_016200.2 | 223 | aagtggtactaggattatacatt | 46362 | - | see also 6718 line | | |
| 11712 | MGC14151 | NM_032356.3 | 624 | gcgcggtctgagcgcttgttaca | 46366 | - | - | - | - |
| 11713 | MPHOSPH 10 | NM_005791.1 | 428 | gagtgcttcctcacgattcaaga | 46372 | - | - | - | - |
| 11714 | PPIG | NM_004792.1 | 654 | ttgcggaggtacggatactcagt | 46423 | - | - | - | - |
| 11715 | PRPF18 | NM_003675.2 | 659 | ttggcgtttgggctaaagaattg | 46473 | - | - | - | - |
| 11716 | PRPF3 | NM_004698.1 | 850 | gagcaagggcgcactgtagatgc | 46499 | - | see also 3220 line | | |
| 11717 | PRPF4 | NM_004697.3 | 1443 | ttgcttactggtgcctatgataa | 46560 | - | - | pre-mRNA splicing factor | - |
| 11718 | PRPF8 | NM_006445.2 | 4650 | tggtccccgaccattaatcgagc | 46656 | - | see also 4507 line | | |
| 11719 | SCA2 | NM_002973.1 | 3454 | gagcagacgcatgcgatgtatgc | 46764 | - | see also 2005 line | | |
| 11720 | SF3A1 | NM_005877.3 | 857 | ccgagtggaatgggccaaattcc | 46788 | - | - | pre-mRNA splicing factor | - |

EP 1 752 536 A1

Figure 46

| 11721 | SF3A3 | NM_006802.1 | 193 | atgataaggatggattacgaaag | 46814 | - | see also 4782 line | | |
|---|---|---|---|---|---|---|---|---|---|
| 11722 | SF3B1 | NM_012433.1 | 1727 | tggtcattgaaccgctattgatt | 46882 | - | see also 5325 line | | |
| 11723 | SF3B4 | NM_005850.3 | 295 | aagccaatacgggtgaacaaagc | 46964 | - | - | pre-mRNA splicing factor | - |
| 11724 | SFRS1 | NM_006924.3 | 309 | tcgcgacggctatgattacgatg | 46980 | - | see also 4853 line | | |
| 11725 | SFRS10 | NM_004593.1 | 769 | tcgccgtcgggattactatgaca | 47007 | - | - | pre-mRNA splicing factor | - |
| 11726 | SFRS11 | NM_004768.2 | 943 | gagacggcggtcacattctaagt | 47028 | - | - | pre-mRNA splicing factor | - |
| 11727 | SFRS2IP | NM_004719.1 | 3322 | ctgtcccaattggataacagaaa | 47091 | - | - | - | - |
| 11728 | SFRS4 | NM_005626.3 | 1541 | tcgccctcccgatctagatctag | 47156 | - | - | pre-mRNA splicing factor | - |
| 11729 | SFRS7 | NM_006276.36 | 68 | gcggtacggaggagaaaccaagg | 47159 | - | - | pre-mRNA splicing factor | - |
| 11730 | SFRS8 | NM_004592.2 | 1295 | cggaatcgacgtgactacttact | 47186 | - | transcription_regulator | - | rheumatoid arthritis |
| 11731 | SFRS9 | NM_003769.2 | 586 | tggggatggtcgagtatctcaga | 47226 | - | - | pre-mRNA splicing factor | - |
| 11732 | SIP1 | NM_003616.1 | 182 | ttgcgacttgacggaaggtttcg | 47233 | - | see also 2429 line | | |
| 11733 | SLU7 | NM_006425.3 | 492 | taggcgagttggagccaaattta | 47262 | - | - | - | - |
| 11734 | SMNDC1 | NM_005871.2 | 799 | gagagtgtgactggtaaagttgg | 47294 | - | see also 4086 line | | |
| 11735 | SNRP70 | NM_003089.3 | 295 | gcgccgtacattcgagagtttga | 47298 | - | - | pre-mRNA splicing factor | - |
| 11736 | SNRPB | NM_003091.3 | 411 | agggacctcctcccaaagatact | 47312 | - | - | pre-mRNA splicing factor | - |
| 11737 | SNRPB2 | NM_003092.3 | 650 | ctgattaccctccaaactatatt | 47324 | - | - | pre-mRNA splicing factor | - |
| 11738 | SNRPD1 | NM_006938.2 | 294 | ctggaaacgctgagtattcgagg | 47337 | - | - | pre-mRNA splicing factor | - |
| 11739 | SNRPD2 | NM_004597.4 | 431 | ctgccgcaacaataagaaactcc | 47342 | - | - | - | - |
| 11740 | SNRPD3 | NM_004175.3 | 504 | acgaacaccggtgaggtatatcg | 47347 | - | see also 2803 line | | |
| 11741 | SNRPF | NM_003095.1 | 307 | aaggtgtaataatgtcctttata | 47352 | - | - | pre-mRNA splicing factor | - |
| 11742 | SNRPN | NM_003097.3 | 490 | atgctgcagcacattgactatag | 47354 | - | - | - | Prader-Willi syndrome、diabetes |
| 11743 | TRA2A | NM_013293.2 | 848 | tggcagacgtcgagattcttact | 47390 | - | see also 5388 line | | |
| 11744 | U2AF1 | NM_006758.1 | 368 | ggggaacgtgtacgtcaagtttc | 47406 | - | - | pre-mRNA splicing factor | - |
| 11745 | U2AF2 | NM_007279.1 | 214 | gcgacgacgcagcaaacctttga | 47413 | - | - | pre-mRNA splicing factor | - |

Figure 46

| | | | | | | | | |
|---|---|---|---|---|---|---|---|---|
| 11746 | U5-116KD | NM_004247.1 | 61 | atggataccgacttatgatga | 47421 | - | see also 2851 line | | |
| 11747 | USP39 | NM_006590.1 | 674 | cgggtattgtgggactgaataac | 47489 | - | - | | |
| 11748 | AUH | NM_001698.1 | 492 | cagtgattaacgatattgctaat | 47519 | - | - | | methylglutaconic aciduria |
| 11749 | CPSF1 | NM_013291.1 | 1122 | ccgagcgttccacttgacaagg | 47537 | - | - | mRNA binding, 3' UTR | |
| 11750 | ELAVL1 | NM_001419.2 | 211 | gtgacatcgggagaacgaattg | 47553 | - | see also 993 line | | Alzheimer disease |
| 11751 | ELAVL3 | NM_001420.2 | 218 | cgggccactccttggtacaaatg | 47591 | - | - | mRNA binding, 3' UTR | |
| 11752 | ELAVL4 | NM_021952.2 | 265 | atggtccgacatccaatacaagc | 47600 | - | see also 8211 line | | |
| 11753 | PARN | NM_002582.1 | 1378 | cagcctaaacgtgatcatgttct | 47669 | - | transcription_regulator | | |
| 11754 | PABPC3 | NM_030979.1 | 1373 | aggtgctcctagagtaccattta | 47690 | - | - | | |
| 11755 | PABPC4 | NM_003819.2 | 914 | ccgggtctgccgcgatagatca | 47692 | - | - | poly(A) binding | |
| 11756 | TIA1 | NM_022037.1 | 1038 | cagcagaatcaaattggatatcc | 47738 | - | see also 8243 line | | |
| 11757 | PCF11 | NM_015885.2 | 2487 | cagccgacttacagctttagctga | 47817 | - | - | pre-mRNA cleavage factor | |
| 11758 | GRSF1 | NM_002092.1 | 548 | cagccgtatgtggaagtatatga | 47953 | - | see also 1356 line | | |
| 11759 | RBM8A | NM_005105.2 | 369 | aagcggtatactcagttgaata | 47989 | - | - | mRNA binding | |
| 11760 | SLBP | NM_006527.2 | 561 | ttgcctacgatcgttattattaaa | 48003 | - | - | mRNA binding | |
| 11761 | SSB | NM_003142.2 | 276 | aggttgaaccgtctaacaacaga | 48020 | - | see also 2124 line | | |
| 11762 | ADAR | NM_001111.2 | 1719 | atgcccagttcgctagtcaaacc | 48073 | - | see also 695 line | | |
| 11763 | ADARB1 | NM_001112.1 | 828 | ttgaggtctttcgttcagtttcc | 48116 | - | - | | |
| 11764 | ADARB2 | NM_018702.1 | 1706 | gaggactcagagcgatcgatatt | 48158 | - | - | | |
| 11765 | CARF | NM_017632.1 | 547 | gggatatcgagtagcaatgaagg | 48176 | - | - | | |
| 11766 | DHX30 | NM_014966.2 | 584 | aggtcccgaatgaagttgtttg | 48190 | - | see also 6154 line | | |
| 11767 | DICER1 | NM_030621.2 | 3665 | tggttaagctccacgttgaagttt | 48353 | - | see also 8898 line | | |
| 11768 | FLJ20399 | NM_017803.3 | 953 | cagatacgcggtgcagtatgaca | 48440 | - | - | | |
| 11769 | LOC161931 | NM_139174.2 | 1211 | gggccctgctgaggttctgttc | 48474 | - | - | | |
| 11770 | LRRFIP1 | NM_004735.1 | 1426 | gtgtcctttagggcatagtgatg | 48486 | - | - | | |
| 11771 | MRPL44 | NM_022915.2 | 243 | aagccgaactgggattaccatgc | 48498 | - | - | | |
| 11772 | PRKR | NM_002759.1 | 2003 | ctgaggatcgacctaacacatct | 48578 | - | kinase_related, phosphatase, apoptosis, cel1_cycle, transcription_regulator | | protein tyrosine kinase |
| 11773 | PRKRA | NM_003690.3 | 313 | gagtaaccgttggtgacataacc | 48583 | - | kinase_related, signal_transduction | | |
| 11774 | RNASE3L | NM_013235.2 | 1557 | acgagtaggcttcgtgacttata | 48628 | - | - | | |

Figure 46

| | | | | | | | | | |
|---|---|---|---|---|---|---|---|---|---|
| 11775 | SLC4A1AP | NM_018158.1 | 371 | gggacgtggacaggaacgtaaag | 48726 | - | - | - | - |
| 11776 | STAU | NM_004602.1 | 1882 | tggctgcgctgaacatcttaaag | 48783 | - | see also 3130 line | | |
| 11777 | STAU2 | NM_014393.1 | 1105 | atgcctcgacgtcgagaatttgt | 48801 | - | see also 5657 line | | |
| 11778 | STRBP | NM_018387.2 | 2089 | aggcgcaggtccaaataagaaag | 48866 | - | see also 7432 line | | |
| 11779 | TARBP2 | NM_004178.3 | 579 | tcgagtggagcgtttcattgaga | 48881 | - | transcription_regulator | - | - |
| 11780 | Tenr | NM_139243.1 | 269 | cagcgacaaagacgataactaca | 48891 | - | see also 9680 line | | |
| 11781 | TLR3 | NM_003265.2 | 304 | ccgccaacttcacaaggtatagc | 48948 | - | receptor_acitivity、sig nal_transduction、kina se_related | transmembrane receptor | - |
| 11782 | HNRPH1 | NM_005520.1 | 656 | atgatccaccacgaaagcttatg | 49037 | - | see also 3850 line | | |
| 11783 | AARS | NM_001605.1 | 165 | aagaggaacgagcatacgtatgt | 49045 | - | - | tRNA binding | - |
| 11784 | CARS | NM_001751.3 | 1415 | atggagtcagcgcttcaatatga | 49113 | - | - | - | - |
| 11785 | FARS1 | NM_006567.1 | 678 | tcgctctgcgcataaacaagaga | 49135 | - | - | phenylalanine-tRNA ligase | - |
| 11786 | MARS | NM_004990.2 | 949 | gtgggacagatgagtatggtaca | 49168 | - | - | tRNA binding | - |
| 11787 | SCYE1 | NM_004757.1 | 305 | ctgcacgctaattctatggtttc | 49202 | - | signal_transduction | tRNA binding | - |
| 11788 | SLA/LP | NM_016955.1 | 188 | tggcaactggtatgagtctaact | 49224 | - | see also 6981 line | | |
| 11789 | YARS | NM_003680.2 | 1057 | tagaactccgagtcagttactat | 49262 | - | apoptosis | tyrosine-tRNA ligase | - |
| 11790 | RPL5 | NM_000969.2 | 223 | gtgtgacaaacagagatatcatt | 49289 | - | - | structural constituent of ribosome | - |
| 11791 | RPS11 | NM_001015.3 | 257 | atgcccttcactggtaatgtgt | 49291 | - | - | structural constituent of ribosome | - |
| 11792 | EIF4EL3 | NM_004846.1 | 24 | aagttcgacgctttgaaagatga | 49292 | - | see also 3358 line | | |
| 11793 | EIF4G3 | NM_003760.2 | 2332 | aggcgtacctttgttgaatgttg | 49354 | - | see also 2573 line | | |
| 11794 | NCBP2 | NM_007362.2 | 143 | ctgtacgttatatgttggaaatc | 49430 | - | - | RNA cap binding | - |
| 11795 | RNUT1 | NM_005701.2 | 369 | atgctaatcaattgatgctttct | 49440 | - | - | - | - |
| 11796 | RRP4 | NM_014285.4 | 685 | ttgctgatcgagaggtgatatcc | 49467 | - | see also 5592 line | | |
| 11797 | SRP19 | NM_003135.1 | 370 | cagttcccatcacgtaagtcagt | 49489 | - | see also 2115 line | | |
| 11798 | PTBP1 | NM_002819.3 | 1601 | gcgtcgtcaaaggattcaagttc | 49505 | - | - | - | - |
| 11799 | A2BP1 | NM_018723.2 | 1808 | ccgcggtcgcaccgtgtacaaca | 49520 | - | see also 7576 line | | |
| 11800 | ACF | NM_014576.2 | 585 | gtggacaactgccgattatttgt | 49551 | - | - | - | - |
| 11801 | ACO1 | NM_002197.1 | 2204 | aggcctaactccacgagaattca | 49635 | - | see also 1400 line | | |
| 11802 | AD023 | NM_020679.2 | 533 | gggtctgctatgtcacctttatc | 49657 | - | - | - | - |
| 11803 | ADAT1 | NM_012091.2 | 127 | cagctatgctatgaacactatgg | 49666 | - | - | - | - |
| 11804 | AKAP1 | NM_003488.2 | 2544 | tggactacggcggatataagagg | 49712 | - | - | - | - |
| 11805 | ALK | NM_004304.3 | 4972 | tggggcctgtataccggataatga | 49753 | - | see also 2882 line | | |
| 11806 | APOBEC1 | NM_001644.2 | 95 | tgggagtttgacgtcttctatga | 49765 | - | - | - | - |

490

EP 1 752 536 A1

Figure 46

| 11807 | BAT1 | NM_004640.3 | 842 | gggactccaggccgtatcctagc | 49785 | - | see also 3154 line | | |
| 11808 | BRUNOL4 | NM_020180.1 | 89 | ccgggcacatgaacggattaagc | 49809 | - | see also 7788 line | | |
| 11809 | C14orf21 | NM_174913.1 | 483 | atgcggggtccatgtattacaaa | 49821 | - | - | - | - |
| 11810 | C15orf12 | NM_018285.1 | 548 | gcggcacgtgctagagtacaatg | 49838 | - | - | - | - |
| 11811 | C1orf25 | NM_030934.3 | 844 | atgttaggcgccacatgaataaa | 49864 | - | - | - | Alzheimer disease |
| 11812 | CGI-37 | NM_016101.2 | 414 | ctggattaccttgcaccttatgc | 49908 | - | - | - | - |
| 11813 | CGI-79 | NM_016024.1 | 855 | gggcgtagttatagaagtagaag | 49920 | - | - | RNA binding | - |
| 11814 | CHERP | NM_006387.4 | 2677 | gcggggacgtccgggataagtgg | 49941 | - | - | - | - |
| 11815 | CIRBP | NM_001280.1 | 485 | cggaggctccagagactactata | 49946 | - | - | RNA binding | - |
| 11816 | CIZ1 | NM_012127.1 | 2058 | ccgtttgcaaccgctacttcaaa | 49962 | - | cell_cycle | zinc binding | - |
| 11817 | CPSF2 | NM_017437.1 | 1191 | ccgtgtacctagccctaaagttg | 49988 | - | see also 7011 line | | |
| 11818 | CPSF3 | NM_016207.2 | 701 | ctgtaacactgtccacgatattg | 50057 | - | - | RNA binding | - |
| 11819 | CPSF5 | NM_007006.1 | 193 | cagcgcatgagggaagaatttga | 50105 | - | - | - | - |
| 11820 | CPSF6 | NM_007007.1 | 1573 | tagagaccgtcatgacgattatt | 50143 | - | - | - | - |
| 11821 | CSTF1 | NM_001324.1 | 1355 | ctgtcgttggggcacaacaatat | 50205 | - | - | RNA binding | - |
| 11822 | CSTF2 | NM_001325.1 | 1235 | agggatccccgaggaatagatgc | 50228 | - | see also 874 line | | |
| 11823 | CSTF3 | NM_001326.1 | 1693 | cggctttactagtagatagatac | 50288 | - | see also 876 line | | |
| 11824 | CUGBP1 | NM_006560.2 | 1233 | tcgggtatccagcaatatgctgc | 50327 | - | see also 4596 line | | |
| 11825 | CUGBP2 | NM_006561.1 | 208 | cggagccgtctaccagatcaacg | 50337 | - | see also 4600 line | | |
| 11826 | DAZAP1 | NM_018959.2 | 288 | tggagaagtcgtagattgtgtta | 50362 | - | see also 7608 line | | |
| 11827 | DAZL | NM_001351.2 | 902 | atgttgtacctccggcttattca | 50390 | - | - | - | - |
| 11828 | DDX1 | NM_004939.1 | 1066 | aaggcaccggatggttacattgt | 50422 | - | see also 3438 line | | |
| 11829 | DDX10 | NM_004398.2 | 1581 | gtggcaccacgcgtaagatttct | 50513 | - | - | RNA helicase | - |
| 11830 | DDX17 | NM_006386.3 | 2179 | gagctaccaatatgataggttac | 50587 | - | phosphatase | - | - |
| 11831 | DDX18 | NM_006773.3 | 1026 | aggccgtctgctggaccatatgc | 50615 | - | - | ATP dependent RNA helicase | - |
| 11832 | DDX19 | NM_007242.3 | 163 | gagcaacttgcatcttaaggaag | 50656 | - | - | RNA binding | - |
| 11833 | DDX21 | NM_004728.1 | 2112 | atgtacctaccgcatcagtaaca | 50700 | - | see also 3240 line | | |
| 11834 | DDX24 | NM_020414.3 | 2275 | aaggagcgaatccgtttagctcg | 50750 | - | see also 7867 line | | |
| 11835 | DDX25 | NM_013264.2 | 612 | atgccattcgagggaatcgaatt | 50758 | - | see also 5382 line | | |
| 11836 | DDX41 | NM_016222.2 | 415 | atggctaagggcattacgtatga | 50792 | - | see also 6727 line | | |
| 11837 | DDX43 | NM_018665.1 | 655 | aggagatcggccattgatagatt | 50820 | - | - | - | - |
| 11838 | DDX5 | NM_004396.2 | 662 | aggcgatgggcctatttgtttgg | 50877 | - | - | RNA helicase | - |
| 11839 | DDX56 | NM_019082.1 | 485 | gggaccccatctcgcatattaag | 50930 | - | - | ATP dependent RNA helicase | - |
| 11840 | DDX6 | NM_004397.3 | 1579 | gagacctatctccatcgtattgg | 50984 | - | - | RNA helicase | - |
| 11841 | DHX34 | NM_014681.3 | 2359 | aggagcatcgactgtacgaaatg | 51007 | - | - | RNA binding | - |

## Figure 46

| 11842 | DIS3 | NM_014953.2 | 235 | ctgcccgacactaatgtgttact | 51016 | - | - | ribonuclease | - |
|-------|------|-------------|-----|-------------------------|-------|---|---|--------------|---|
| 11843 | DKC1 | NM_001363.2 | 94 | tggcggatgcggaagtaattatt | 51105 | - | cell_cycle | pseudouridylate synthase | dyskeratosis congenita |
| 11844 | DSCR1L2 | NM_013441.2 | 798 | atgcgatgcctgttataaattat | 51156 | - | - | RNA binding | |
| 11845 | DUSP11 | NM_003584.1 | 829 | aggacctaggtataatctacatc | 51177 | - | phosphatase | prenylated protein tyrosine phosphatase | - |
| 11846 | EIF2S1 | NM_004094.3 | 249 | aaggcgtatccgttctatcaaca | 51189 | - | - | Nuclear respiratory factor-1 | |
| 11847 | EIF3S4 | NM_003755.1 | 182 | aggtcatcaacggaaacataaag | 51220 | - | - | translation initiation factor | - |
| 11848 | EIF3S9 | NM_003751.2 | 641 | gggacccgaccgacttgagaaac | 51229 | - | - | - | - |
| 11849 | EIF4B | NM_001417.1 | 1449 | ttgggtgaagcgaagttctaacc | 51261 | - | - | translation initiation factor | - |
| 11850 | EIF4G1 | NM_004953.2 | 3115 | ctgctcgcccagctactagtact | 51298 | - | - | translation initiation factor | - |
| 11851 | ERAL1 | NM_005702.1 | 497 | gagacccaggtgattctacttga | 51425 | - | - | RNA binding | - |
| 11852 | ETF1 | NM_004730.1 | 1043 | ttggcgttgaagatacactaaag | 51474 | - | see also 3242 line | | |
| 11853 | FBL | NM_001436.2 | 821 | gcgtaatggaggacactttgtga | 51515 | - | - | RNA binding | - |
| 11854 | FLJ10379 | NM_018079.3 | 2874 | ctgggctgattcccatacgaaat | 51601 | - | - | - | - |
| 11855 | FLJ10581 | NM_018146.2 | 377 | gggcttcgctacgataaagctta | 51614 | - | see also 7281 line | | |
| 11856 | FLJ20244 | NM_017722.2 | 607 | caggcctacgttccattcgattt | 51697 | - | - | - | - |
| 11857 | FLJ22419 | NM_024697.1 | 1157 | aaggcaaaggacctgttaataaa | 51715 | - | - | - | - |
| 11858 | FLJ25270 | NM_152520.2 | 1251 | cagcggagcccgagtattctagc | 51740 | - | - | - | - |
| 11859 | FLJ31121 | NM_144723.1 | 590 | tggttccaccaagaagagttact | 51757 | - | see also 9744 line | | |
| 11860 | FXR1 | NM_005087.1 | 1175 | gaggtcgtcggggacctaattac | 51807 | - | see also 3550 line | | |
| 11861 | FXR2 | NM_004860.2 | 585 | gagcgacttcggccagttaatcc | 51833 | - | see also 3377 line | | |
| 11862 | G3BP | NM_005754.2 | 1270 | gtggagttgcgcattaacagtgg | 51873 | - | signal_transduction | protein transporter | - |
| 11863 | G3BP2 | NM_012297.2 | 1190 | ttgtggaacttcgcatcaatacc | 51892 | - | see also 5264 line | | |
| 11864 | HDLBP | NM_005336.1 | 3096 | ttgaccttcaccgttacgttatt | 51967 | - | see also 3717 line | | |
| 11865 | HNRPA0 | NM_006805.2 | 699 | gtggattcggcttcgtgtatttc | 51990 | - | - | RNA binding | - |
| 11866 | HNRPA2B1 | NM_002137.2 | 974 | gggggctacggaggtggttatga | 52006 | - | - | - | - |
| 11867 | HNRPAB | NM_004499.2 | 499 | gagaggtcgttgactgtacaata | 52015 | - | see also 3050 line | | |
| 11868 | HNRPF | NM_004966.2 | 1219 | aagcgaccgagaacgacatttac | 52047 | - | - | RNA binding | - |
| 11869 | HNRPH2 | NM_019597.2 | 192 | tggcacatcaggtattcgtttca | 52055 | - | - | RNA binding | - |
| 11870 | HNRPH3 | NM_012207.1 | 443 | cagcgaccgggaccatatgatag | 52065 | - | - | - | - |
| 11871 | HNRPL | NM_001533.1 | 1120 | cggtgaccgagtcttcaatgtct | 52107 | - | - | RNA binding | - |

Figure 46

| | | | | | | | | | |
|---|---|---|---|---|---|---|---|---|---|
| 11872 | HNRPM | NM_005968.2 | 2391 | gagattgacgttcgaattgatag | 52135 | - | see also 4169 line | | |
| 11873 | HNRPR | NM_005826.2 | 907 | ttggtggacgttattctctatca | 52163 | - | see also 4062 line | | |
| 11874 | ILF2 | NM_004515.2 | 859 | cagcctttggccctaaacgttgc | 52201 | - | transcription_regulator | - | - |
| 11875 | IMP-3 | NM_006547.2 | 1126 | ttgttggacgtcttattggtaaa | 52242 | - | see also 4588 line | | |
| 11876 | KHDRBS3 | NM_006558.1 | 1027 | ccggccagttggagttgtagtac | 52264 | - | see also 4595 line | | |
| 11877 | KIAA0116 | NM_015004.2 | 531 | ggggtcgaaggacattgaattgt | 52283 | - | - | 3'-5' exoribonuclease | - |
| 11878 | KIAA0117 | NM_015014.1 | 139 | caggtcgccagtagcttatttcg | 52287 | - | - | - | - |
| 11879 | KIAA0427 | NM_014772.1 | 1517 | gcgagatcgtgcgcacaatctac | 52309 | - | - | RNA binding | - |
| 11880 | KIAA0682 | NM_014852.1 | 1551 | tgggatcgtcgtcctacaagaag | 52322 | - | see also 6053 line | | |
| 11881 | LGTN | NM_006893.1 | 1355 | ccgaacgatcgtcattaactacg | 52388 | - | receptor_acitivity | translation initiation factor | - |
| 11882 | LOC91801 | NM_138775.1 | 206 | aggcattgagacagtatcctatg | 52402 | - | - | - | - |
| 11883 | MBNL1 | NM_021038.1 | 1498 | acggccagacacggaatgtaaat | 52457 | - | see also 8071 line | | |
| 11884 | MCTS1 | NM_014060.1 | 412 | tagtccgatgccatgaacatata | 52487 | - | see also 5475 line | | |
| 11885 | METTL3 | NM_019852.2 | 816 | caggagatcctagagctattaaa | 52505 | - | see also 7738 line | | |
| 11886 | MGC42174 | NM_152383.2 | 1043 | gtggcacggcctaaagattatgc | 52551 | - | see also 9842 line | | |
| 11887 | MGC4562 | NM_133375.2 | 754 | aaggctcgaaaccgctcaattca | 52581 | - | - | - | - |
| 11888 | MOV10L1 | NM_018995.1 | 2520 | caggttcgaggagatagttattg | 52711 | - | - | hydrolase | - |
| 11889 | MRPL1 | NM_020236.2 | 712 | atgcctgaacttaatcgattaag | 52739 | - | - | - | - |
| 11890 | MRPL12 | NM_002949.2 | 493 | tagcgaaagaacggacacatttc | 52748 | - | - | structural constituent of ribosome | - |
| 11891 | MRPL23 | NM_021134.2 | 108 | gggtgttccgaaccaacttcttc | 52751 | - | - | - | - |
| 11892 | MRPL3 | NM_007208.2 | 1184 | cggtgcgccttctattacatttg | 52783 | - | - | structural constituent of ribosome | - |
| 11893 | MRPS28 | NM_014018.2 | 438 | agggtccggttgcggctattaga | 52793 | - | - | structural constituent of ribosome | - |
| 11894 | NHP2L1 | NM_005008.1 | 99 | ctgaggctgatgtgaatccaaag | 52816 | - | cell_cycle | structural constituent of ribosome | - |
| 11895 | NOL4 | NM_003787.1 | 1957 | atgggacctatagttaccatagt | 52848 | - | - | - | - |
| 11896 | NOL5A | NM_006392.2 | 1210 | ctgaggtgcccacgagtgtattc | 52884 | - | see also 4485 line | | |
| 11897 | NOPD1 | NM_182543.1 | 1530 | tagcacgtgcactataacactgg | 52922 | - | - | - | - |
| 11898 | NOVA1 | NM_002515.1 | 231 | tagttatgctgctggatctataa | 52930 | - | - | - | - |
| 11899 | NUFIP1 | NM_012345.1 | 835 | aaggggatgtccagacattcaca | 52965 | - | - | - | - |
| 11900 | NXF2 | NM_017809.2 | 894 | gggagttctttccgggataattt | 53028 | - | transcription_regulator | - | - |
| 11901 | NXF3 | NM_022052.1 | 319 | aagaccaaacccacgttaacatg | 53039 | - | - | - | - |

Figure 46

| | | | | | | | | | |
|---|---|---|---|---|---|---|---|---|---|
| 11902 | OAS1 | NM_002534.1 | 858 | tcggacggtctggaattagtca | 53064 | - | - | | |
| 11903 | OAS2 | NM_002535.1 | 1530 | cagggctcattgatctgtataaa | 53092 | - | - | | |
| 11904 | OAS3 | NM_006187.1 | 427 | ccgcctgacatccgtagatcttg | 53105 | - | - | nucleotidyltransferase | - |
| 11905 | OIP2 | NM_181503.1 | 133 | ctgtcaacatcggttcaattagt | 53129 | - | see also 10233 line | | - |
| 11906 | PABPC5 | NM_080832.1 | 723 | cagccagatgaccgcttaagaaa | 53168 | - | see also 9528 line | | - |
| 11907 | PABPN1 | NM_004643.1 | 1841 | gagctgaagctcacttcatg | 53188 | - | see also 3163 line | | - |
| 11908 | PAPOLA | NM_032632.2 | 309 | gagactgactgcgtacttacaca | 53199 | - | see also 9264 line | | - |
| 11909 | PAPOLB | NM_020144.3 | 1941 | cagggggtgcattaaacgaaagt | 53259 | - | transcription_regulator | polynucleotide adenylyltransferase | - |
| 11910 | PAPOLG | NM_022894.2 | 2054 | ccgtagtaggacgaaatgtcatt | 53333 | - | see also 8496 line | | - |
| 11911 | PMSCL1 | NM_005033.1 | 1244 | aggcggtggtgatcaagctatca | 53364 | - | see also 3518 line | | - |
| 11912 | PMSCL2 | NM_002685.1 | 310 | atgggtgtcgagcaacattaag | 53378 | - | see also 1748 line | | - |
| 11913 | PNPT1 | NM_033109.2 | 2040 | cagtataccgccacaataact | 53498 | - | - | | - |
| 11914 | POLR2G | NM_002696.1 | 586 | tggctccctgatggacgattact | 53520 | - | - | RNA binding | - |
| 11915 | POP4 | NM_006627.1 | 639 | gtgaacgtctgcgaagaagttc | 53540 | - | - | ribonuclease P | - |
| 11916 | PPIE | NM_006112.1 | 480 | cagatgctccgttctgatgt | 53553 | - | see also 4248 line | | - |
| 11917 | PPP1R8 | NM_002713.2 | 1058 | gagcaggcgcatgcaggaactttg | 53556 | - | phosphatase | | - |
| 11918 | PS1D | NM_016505.2 | 302 | aagccctcgagatagtagatgt | 53582 | - | - | | - |
| 11919 | PSMA1 | NM_002786.2 | 452 | cagataccaacaacaacgatatgg | 53616 | - | see also 1814 line | | - |
| 11920 | PSMA6 | NM_002791.1 | 209 | cagggtgccttacatcagtagc | 53636 | - | - | proteasome endopeptidase | - |
| 11921 | PUM1 | NM_014676.1 | 98 | gagcgttgcatgtgtcttgaaga | 53639 | - | see also 5858 line | | - |
| 11922 | PUM2 | NM_015317.1 | 3084 | aagattcgacctcacattactac | 53822 | - | see also 6328 line | | - |
| 11923 | RAE1 | NM_003610.2 | 635 | tgggatactgatcgtcaaatcc | 53844 | - | see also 2422 line | | - |
| 11924 | RALY | NM_007367.2 | 413 | gagaccatcttctctaagtatgg | 53876 | - | - | | - |
| 11925 | RBM10 | NM_005676.3 | 2103 | atgagacccggctactactat | 53892 | - | see also 3943 line | | - |
| 11926 | RBM11 | NM_144770.1 | 466 | ctgcagcttccttactatgaaat | 53913 | - | - | RNA binding | - |
| 11927 | RBM15 | NM_022768.3 | 2117 | cagtagccggatcgttacaaca | 53958 | - | - | RNA binding | leukemia |
| 11928 | RBM16 | NM_014892.1 | 1366 | tgccggttcgctcaagatcaaga | 54000 | - | see also 6078 line | | - |
| 11929 | RBM3 | NM_006743.2 | 92 | tgggaggctcaactttaacacc | 54064 | - | - | RNA binding | - |
| 11930 | RBM7 | NM_016090.2 | 675 | atgggtctgaccatcattacaga | 54085 | - | see also 6657 line | | - |
| 11931 | RBM9 | NM_014309.1 | 1210 | tggcgctggcgagtttatacc | 54101 | - | transcription_regulator, signal_transduction | RNA binding | - |
| 11932 | RBMS2 | NM_002898.1 | 1203 | cagagcatgggtctattctttc | 54113 | - | - | | - |
| 11933 | RBMS3 | NM_014483.1 | 1180 | ttggccacaacaggaacgtatat | 54137 | - | see also 5701 line | | - |

Figure 46

| | | | | | | | | | | |
|---|---|---|---|---|---|---|---|---|---|---|
| 11934 | RBPMS | NM_006867.1 | 708 | aaggctatgagggttcttat | 54145 | - | - | - | RNA binding | |
| 11935 | RDBP | NM_002904.4 | 433 | ccgttccagaggaggcatatctgc | 54153 | - | transcription_regulator | - | RNA binding | |
| 11936 | RENT1 | NM_002911.2 | 331 | gagttcaccgactttacttctcc | 54159 | - | see also 1941 line | - | | |
| 11937 | RNASE1 | NM_002933.3 | 250 | cagcagctcacctactgtaacc | 54203 | - | - | - | pancreatic ribonuclease | |
| 11938 | RNASEH1 | NM_002936.3 | 417 | aagcgcagagccgtatgcaaagc | 54206 | - | - | - | ribonuclease H1 | |
| 11939 | RNASEH2A | NM_063397.2 | 800 | aagaggcggaagatgttatatgg | 54217 | - | - | - | ribonuclease H | |
| 11940 | RNASEL | NM_021133.2 | 2058 | aagttgacgacgtaagattaatga | 54258 | - | kinase_related | - | protein kinase | prostate cancer |
| 11941 | RNASET2 | NM_003730.3 | 987 | cagacaattggtcagatagaact | 54276 | - | - | - | endoribonuclease | |
| 11942 | RNMT | NM_003799.1 | 1553 | ttgggaacttaagtaaatcaga | 54308 | - | - | - | - | |
| 11943 | RNPC1 | NM_017495.4 | 665 | tcgctgtcctgccctacattga | 54310 | - | - | - | - | |
| 11944 | RNPC2 | NM_004902.2 | 467 | aagtcgtagtcatgaacgaaaga | 54315 | - | see also 3405 line | - | - | |
| 11945 | RPL18 | NM_000979.2 | 108 | gaggctgttggtcaagttataca | 54396 | - | - | - | structural constituent of ribosome | |
| 11946 | RPL37A | NM_000998.2 | 36 | atggccaaacgtaccaagaaagt | 54403 | - | - | - | structural constituent of ribosome | |
| 11947 | RPL38 | NM_000999.2 | 214 | tcgatgcagcagatacctttaca | 54406 | - | - | - | structural constituent of ribosome | |
| 11948 | RPL3L | NM_005061.2 | 267 | gaggccggtgacaattgtagaaac | 54407 | - | - | - | structural constituent of ribosome | |
| 11949 | RPL4 | NM_000968.2 | 691 | atgaggataatggtatcatcaag | 54416 | - | - | - | structural constituent of ribosome | |
| 11950 | RPL8 | NM_000973.2 | 220 | gggatccgtatcggtttaagaag | 54419 | - | - | - | - | |
| 11951 | RPN1 | NM_002950.2 | 1524 | ttgacgagaccgtcaataggtac | 54454 | - | - | - | dolichyl-diphospho-oligosaccharide-protein glycosyltransferase | |
| 11952 | RPP14 | NM_007042.1 | 330 | aagtttgatgccgccttacctttg | 54462 | - | - | - | ribonuclease P | |
| 11953 | RPS21 | NM_001024.2 | 275 | ccgatgcatcgtctcaaagaac | 54475 | - | - | - | structural constituent of ribosome | |
| 11954 | RPS29 | NM_001032.2 | 159 | ccgtcagtacgcgaaggatatcg | 54481 | - | - | - | zinc binding | |
| 11955 | RPS3 | NM_001005.2 | 150 | caggacagaaatcattatcttag | 54483 | - | - | - | structural constituent of ribosome | |
| 11956 | RPS4Y | NM_001008.2 | 504 | agggaactggcaagataatcaact | 54486 | - | - | - | structural constituent of ribosome | |
| 11957 | RPS4Y2 | NM_138963.1 | 612 | ttgcgatgtggtacatgtgaagg | 54492 | - | - | - | structural constituent of ribosome | |

Figure 46

| | | | | | | | | | |
|---|---|---|---|---|---|---|---|---|---|
| 11958 | RPS5 | NM_001009.2 | 256 | ttgtggagcgcctcactaactcc | 54494 | - | - | structural constituent of ribosome | - |
| 11959 | RPS9 | NM_001013.2 | 328 | gggcctgaagatagaggatttct | 54498 | - | - | structural constituent of ribosome | - |
| 11960 | RRP40 | NM_016042.1 | 578 | gtgactctgggcttaattagaaa | 54512 | - | - | 3'-5' exoribonuclease | - |
| 11961 | RRP41 | NM_019037.1 | 407 | cagctccgccagactttcgaagc | 54520 | - | - | 3'-5' exoribonuclease | - |
| 11962 | RRP46 | NM_020158.1 | 255 | gtgatcctgaggccgaagattgg | 54531 | - | - | structural constituent of ribosome | - |
| 11963 | RTCD1 | NM_003729.1 | 1204 | aagacgccgctaaagatacttat | 54568 | - | - | RNA-3'-phosphate cyclase | - |
| 11964 | SARS | NM_006513.2 | 423 | gtgtgacgcggagcggataaagt | 54582 | - | see also 4553 line | | |
| 11965 | SBP2 | NM_024077.2 | 899 | ctgctaatgccgctaccaattct | 54611 | - | - | structural constituent of ribosome | - |
| 11966 | SCA1 | NM_000332.1 | 2809 | cgggcgtggccgtgatacagttc | 54660 | - | see also 281 line | | |
| 11967 | SF1 | NM_004630.1 | 1115 | cgggagttggctcgcttaaatgg | 54687 | - | see also 3145 line | | |
| 11968 | SF3A2 | NM_007165.3 | 217 | aggacccgtacttcatgaagaac | 54695 | - | - | RNA binding | - |
| 11969 | SF4 | NM_021164.2 | 55 | ttggggttgctccccctaaatct | 54702 | - | - | - | - |
| 11970 | SFRS14 | NM_014884.1 | 768 | cagaggtcgagctctaaacatcg | 54733 | - | - | - | - |
| 11971 | SFRS3 | NM_003017.3 | 288 | gagctagatggaagaacactatg | 54782 | - | - | RNA binding | - |
| 11972 | SKIV2L | NM_006929.3 | 1797 | ctgaagcgtcgtcagatctatgt | 54806 | - | see also 4860 line | | |
| 11973 | SNRPA | NM_004596.3 | 797 | aagaccgactcagatatcattgc | 54836 | - | - | RNA binding | - |
| 11974 | SNRPA1 | NM_003090.1 | 669 | aagaatgccatagcaaatgcttc | 54844 | - | see also 2080 line | | |
| 11975 | SRP54 | NM_003136.2 | 1550 | aggtggcgacatgtctaagaatg | 54881 | - | see also 2116 line | | |
| 11976 | SRP68 | NM_014230.2 | 1531 | atgctggcgccttcaagaacagc | 54924 | - | see also 5548 line | | |
| 11977 | SUPV3L1 | NM_003171.2 | 1874 | tggttacgccgatacatcaaatg | 55025 | - | see also 2149 line | | |
| 11978 | SURF6 | NM_006753.3 | 663 | cgggctgatcttcaataaggtgg | 55047 | - | - | RNA binding | - |
| 11979 | SYNCRIP | NM_006372.3 | 1427 | aggcaaggcgtaggttaatgagt | 55074 | - | see also 4463 line | | |
| 11980 | SYNJ1 | NM_003895.1 | 359 | ctgcgaatcgattcttcagatga | 55090 | - | phosphatase | inositol-1、4、5-trisphosphate 5-phosphatase | - |
| 11981 | SYNJ2 | NM_003898.1 | 2434 | ttggctcagccgcctacgataca | 55255 | - | see also 2655 line | | |
| 11982 | TARBP1 | NM_005646.2 | 3230 | ctgtgtttaggcgtgatcaaaga | 55336 | - | transcription_regulator | - | - |
| 11983 | TEP1 | NM_007110.3 | 7421 | tagtaggaccctaatatcgataa | 55502 | - | see also 4953 line | | |
| 11984 | TRNT1 | NM_016000.2 | 341 | tgggattcggatgataaacaaca | 55523 | - | see also 6602 line | | |
| 11985 | TTC14 | NM_133462.1 | 698 | tagcagtatctctgtatagctct | 55578 | - | see also 9581 line | | |
| 11986 | UPF2 | NM_015542.2 | 1216 | caggcgcattctacattctaaag | 55646 | - | - | - | - |
| 11987 | WBP4 | NM_007187.3 | 182 | taggcctagtgttgaatttcatg | 55726 | - | see also 4986 line | | |

Figure 46

| | | | | | | | | | |
|---|---|---|---|---|---|---|---|---|---|
| 11988 | WIG1 | NM_022470.2 | 540 | atggtaagaaactccgaaattac | 55759 | - | see also 8391 line | | |
| 11989 | XRN2 | NM_012255.2 | 1679 | gagaaattccgtcggaaagttgt | 55829 | - | see also 5224 line | | |
| 11990 | ZNF318 | NM_014345.1 | 3424 | aagcgcactgacaagataactgt | 55988 | - | see also 5630 line | | |
| 11991 | ZPR9 | NM_033414.2 | 122 | tggcgacgtacacctgcataact | 56077 | - | see also 9422 line | | |
| 11992 | BRF2 | NM_018310.2 | 460 | tggttgggtgctgcgtcttaatc | 56108 | - | - | | |
| 11993 | BZW1 | NM_014670.1 | 949 | cagttgtcatcggaatagtctgg | 56136 | - | see also 5848 line | | |
| 11994 | BZW2 | NM_014038.1 | 402 | tggaggaacgcgcatagatgatg | 56141 | - | see also 5468 line | | |
| 11995 | CGI-09 | NM_015939.3 | 388 | aagcggcactgataatcgaaat | 56160 | - | - | | |
| 11996 | COPS5 | NM_006837.2 | 726 | atgcaatcgggtgtatcatagc | 56199 | - | see also 4791 line | | |
| 11997 | COPS6 | NM_006833.4 | 772 | gggagaggtcccctttaatcatg | 56235 | - | see also 4788 line | | |
| 11998 | EIF1AY | NM_004681.2 | 321 | atgccatatcagagggaaattga | 56250 | - | | translation initiation factor | |
| 11999 | eIF2A | NM_032025.1 | 107 | aggatcagaaggactgtacatgg | 56253 | - | - | | |
| 12000 | EIF2B1 | NM_001414.1 | 437 | gcgcccaagaagcgatttagtgt | 56311 | - | see also 977 line | | |
| 12001 | EIF2B2 | NM_014239.1 | 163 | agggttgctgccagatcatca | 56321 | - | | translation initiation factor | |
| 12002 | EIF2B3 | NM_020365.1 | 664 | cagaagcatcctagaatacgttt | 56349 | - | | translation initiation factor | leukoencephalopathy |
| 12003 | EIF2B4 | NM_015636.2 | 1292 | gtggctcgagcccataatgtacc | 56379 | - | see also 6525 line | | |
| 12004 | EIF2B5 | NM_003907.1 | 1961 | gcgcgcagccgaccatttggaag | 56431 | - | - | | |
| 12005 | EIF2C1 | NM_012199.2 | 1039 | aagtaccgcgtgtgtaatgttac | 56442 | - | see also 5187 line | | |
| 12006 | EIF2C2 | NM_012154.2 | 464 | gcgttacacgatgcactttcagg | 56475 | - | | translation initiation factor | |
| 12007 | EIF3S1 | NM_003758.1 | 778 | ctggcagattatggtgttatga | 56506 | - | | translation initiation factor | |
| 12008 | EIF3S10 | NM_003750.1 | 1167 | cagcgtgccttgcaacactact | 56547 | - | | translation initiation factor | |
| 12009 | EIF3S2 | NM_003757.1 | 934 | atggttacgtccgtatccattac | 56637 | - | | translation initiation factor | |
| 12010 | EIF3S3 | NM_003756.1 | 601 | aagaagtgccgattgaattaaa | 56655 | - | | translation initiation factor | |
| 12011 | EIF3S6 | NM_001568.1 | 24 | tggcggagtacgacttgactact | 56664 | - | | translation initiation factor | breast cancer |
| 12012 | EIF3S6IP | NM_016091.1 | 34 | atgtcttatcccgctgatgatta | 56710 | - | see also 6658 line | | |
| 12013 | EIF3S7 | NM_003753.2 | 643 | cggcctaccagcggaatcgaatg | 56734 | - | | translation initiation factor | |
| 12014 | EIF3S8 | NM_003752.2 | 417 | ctgacctaggagactatcttaat | 56770 | - | see also 2568 line | | |
| 12015 | EIF4EBP2 | NM_004096.3 | 411 | ctgttggatcgtcgcgaattctcc | 56789 | - | - | | |

Figure 46

| 12016 | EIF5 | NM_001969.3 | 1158 | aaggcgtcgaatggatgaaatca | 56811 | - | see also 1247 line | | |
|---|---|---|---|---|---|---|---|---|---|
| 12017 | EIF5A2 | NM_020390.5 | 346 | ttgtccttctactcacaacatgg | 56839 | - | see also 7862 line | | |
| 12018 | FLJ20288 | NM_024668.1 | 1252 | ttggatatggttcgctttctact | 378012 | - | - | - | - |
| 12019 | GC20 | NM_005875.1 | 383 | cagggcattgcagatgattatga | 56856 | - | - | translation initiation factor | - |
| 12020 | IF2 | NM_015904.2 | 1981 | gagaaacggcgacttgaacatag | 56887 | - | - | translation initiation factor | - |
| 12021 | ITGB4BP | NM_002212.2 | 188 | gggcgagctctccgataccatcc | 56943 | - | - | - | - |
| 12022 | KIAA0664 | NM_015229.2 | 884 | ctgaatcagtccacagcttatca | 56951 | - | - | translation initiation factor | - |
| 12023 | LAMA5 | NM_005560.3 | 9880 | gggcccacagcgcgtatttgatc | 56983 | - | - | structural molecule | - |
| 12024 | LRIG2 | NM_014813.1 | 2325 | aggtgaaactgcggtgttacagt | 57039 | - | - | - | - |
| 12025 | MFHAS1 | NM_004225.1 | 1905 | acgacgccttcgggacaagttgc | 57078 | - | signal_transduction | - | osteosarcoma |
| 12026 | MGC11102 | NM_032325.2 | 682 | gaggagtccagctcagaagatga | 57100 | - | - | - | - |
| 12027 | MGC3207 | NM_032285.1 | 789 | cggcaaggagatcattattgaag | 57105 | - | - | - | - |
| 12028 | MRPL49 | NM_004927.2 | 352 | acgctctcggatgcacaacatcc | 57112 | - | - | structural constituent of ribosome | - |
| 12029 | MTIF2 | NM_002453.1 | 301 | ttgctacgatttcacactattta | 57115 | - | - | translation initiation factor | - |
| 12030 | MTIF3 | NM_152912.3 | 382 | tagtcagcgagttattcacttat | 57183 | - | - | - | - |
| 12031 | PSMD7 | NM_002811.3 | 477 | ctgtcctaattccgtattggtca | 57203 | - | see also 1828 line | | |
| 12032 | RRN3 | NM_018427.2 | 1054 | tcgcgacctgataaacatctttg | 57219 | - | transcription_regulator | - | - |
| 12033 | EEF1A2 | NM_001958.2 | 1464 | tggccgtaggcgtcatcaagaac | 57228 | - | - | translation elongation factor | - |
| 12034 | EEF2 | NM_001961.2 | 744 | aagcagtttgccgagatgtatgt | 57239 | - | - | translation elongation factor | - |
| 12035 | EFG1 | NM_024996.5 | 1948 | gagcacaccacatggttgattct | 57301 | - | see also 8830 line | | |
| 12036 | EFG2 | NM_032380.3 | 575 | ttgtactattccggatatacaag | 57326 | - | see also 9178 line | | |
| 12037 | ELL3 | NM_025165.1 | 240 | aaggccaccgagggtatctgaga | 57397 | - | - | - | - |
| 12038 | FLJ13119 | NM_024580.3 | 2058 | cagcgatgcctggatgacttaaa | 57456 | - | see also 8648 line | | |
| 12039 | FLJ13220 | NM_021927.1 | 1527 | ctgcacaattccccgataaatca | 57506 | - | see also 8199 line | | |
| 12040 | GSPT1 | NM_002094.1 | 1522 | ccgaacttcaatagatcagttga | 57534 | - | cell_cycle | translation elongation factor | - |
| 12041 | GSPT2 | NM_018094.1 | 1734 | tggacgcacgtttgatgttcaga | 57548 | - | - | - | - |
| 12042 | GTPBP2 | NM_019096.2 | 1012 | ttgctcagtcacccaatgtcacc | 57590 | - | - | - | - |
| 12043 | HBS1L | NM_006620.2 | 1830 | caggcgatcatgttagtcttact | 57647 | - | see also 4680 line | | |

Figure 46

| 12044 | HTATSF1 | NM_014500.3 | 409 | acgatggcgcatctagttctacc | 57674 | - | transcription_regulator | - | |
|---|---|---|---|---|---|---|---|---|---|
| 12045 | SELB | NM_021937.2 | 1222 | caggctagatgcggacattcaca | 57735 | - | | protein-synthesizing GTPase、elongation | - |
| 12046 | SUPT16H | NM_007192.2 | 1698 | aggttctcgggcagcattactta | 57764 | - | see also 4990 line | | |
| 12047 | TUFM | NM_003321.3 | 388 | atgcggctcatgtggagtatagc | 57803 | - | see also 2253 line | | |
| 12048 | FLJ38663 | NM_152269.1 | 646 | gagcaccgtgggtttatttcatt | 57820 | - | - | - | - |
| 12049 | ICT1 | NM_001545.1 | 223 | tagatcgcttgacaatatcttat | 57837 | - | - | translation release factor | - |
| 12050 | MTRF1 | NM_004294.2 | 932 | aggaacgatgtcggttattgtcc | 57862 | - | - | translation release factor | - |
| 12051 | ABCF1 | NM_001090.1 | 2423 | cagagtgttagccaaatcgatgg | 57906 | - | - | - | - |
| 12052 | EEF2K | NM_013302.2 | 275 | cagcgacgatgaggaaggttact | 57908 | - | kinase_related | translation factor、nucleic acid binding | - |
| 12053 | PET112L | NM_004564.1 | 1610 | tggtccggaaagcgactcaaagc | 57963 | - | see also 3103 line | | |
| 12054 | AF020591 | NM_014480.1 | 904 | gagctttctgtcagagtatttac | 57971 | - | transcription_regulator | - | |
| 12055 | AFG3L2 | NM_006796.1 | 222 | cggacgctttaccgatttgttac | 57988 | - | - | metalloendopeptidase | - |
| 12056 | ARIH2 | NM_006321.1 | 389 | ctgtcctaaaggtatctcattca | 58047 | - | - | zinc binding | - |
| 12057 | ASK | NM_006716.2 | 1277 | cagtatcaagttgttgatgatat | 58078 | - | see also 4734 line | | |
| 12058 | BANK1 | NM_017935.2 | 1276 | gggttcagacccccgcacatattg | 58119 | - | - | - | - |
| 12059 | BAT4 | NM_033177.2 | 1183 | gcggctcagctcgctgaagaagc | 58153 | - | - | - | - |
| 12060 | BDG29 | NM_015144.1 | 2707 | tggtgggcagtagcaatggttcc | 58187 | - | - | - | - |
| 12061 | BOLL | NM_033030.3 | 651 | tagggatccctcgttctagtata | 58203 | - | see also 9351 line | | |
| 12062 | BRUNOL6 | NM_052840.2 | 1000 | gggctccgacacgctctacaata | 58224 | - | see also 9434 line | | |
| 12063 | C13orf10 | NM_022118.3 | 849 | gtggaccataactcttacgtaag | 58257 | - | see also 8282 line | | |
| 12064 | C14orf114 | NM_018199.1 | 569 | gtggtcgacatccatttcgaag | 58314 | - | see also 7314 line | | |
| 12065 | C14orf6 | NM_145250.1 | 613 | aggtgccaataccatagtgttac | 58341 | - | - | - | - |
| 12066 | C20orf99 | NM_033089.4 | 1008 | cagcccaagacatgctttaaatg | 58347 | - | transcription_regulator | - | |
| 12067 | C21orf107 | NM_018963.3 | 3797 | aggaggctgtctgcgttagtttg | 58476 | - | see also 7610 line | | |
| 12068 | CGI-18 | NM_015947.1 | 768 | ccgaacacggattgatgttcttt | 58580 | - | transcription_regulator | - | |
| 12069 | CGI-72 | NM_016018.3 | 1147 | gagagctcgacttaacaagatta | 58626 | - | - | RNA binding | - |
| 12070 | CNOT4 | NM_013316.1 | 1722 | cagcaccgagcggtttataattc | 58683 | - | see also 5396 line | | |
| 12071 | COL4A3 | NM_000091.2 | 3842 | aggtctgcccggtgcaattatcc | 58723 | - | apoptosis、signal_transduction | - | Alport syndrome、Goodpasture syndrome、hematuria |

EP 1 752 536 A1

499

Figure 46

| # | Symbol | Accession | Len | Sequence | ID | Strand | Function | Note |
|---|--------|-----------|-----|----------|----|--------|----------|------|
| 12072 | COVA1 | NM_006375.2 | 1175 | cgtgctagcgccaataacttct | 58759 | - | | see also 4465 line |
| 12073 | CPEB1 | NM_030594.2 | 1547 | ctgggtattagccgacagtaact | 58808 | - | | see also 8893 line |
| 12074 | CPEB3 | NM_014912.1 | 1351 | gagtagaacgctactcctagaaag | 58843 | - | | see also 6085 line |
| 12075 | CPEB4 | NM_030627.1 | 1753 | aaggtcgtctaaactattcatat | 58872 | - | | see also 8910 line |
| 12076 | CPSF4 | NM_006693.1 | 609 | aagtaacaatccgccattacaaa | 58905 | - | | see also 4726 line |
| 12077 | CSTF2T | NM_015235.1 | 359 | ctgcagcgcccattattgactca | 58910 | - | | |
| 12078 | DARS | NM_001349.1 | 1503 | tgtattggaacgagttactatgc | 58959 | - | | see also 896 line |
| 12079 | DC50 | NM_031210.3 | 49 | cggcgctgcgtagaagtatcaat | 58962 | - | | |
| 12080 | DDX26 | NM_012141.1 | 2710 | tggaaccgccaaccaatcatatg | 59037 | - | | see also 5148 line |
| 12081 | DDX31 | NM_022779.7 | 1440 | acgcggctagctgatatcagtt | 59077 | - | | |
| 12082 | DDX39 | NM_005804.2 | 621 | tggcgctcgtgcggaataggagc | 59105 | - | | |
| 12083 | DDX4 | NM_019039.1 | 407 | aggcgtatcgaggatggaaata | 59127 | - | | see also 7670 line |
| 12084 | DDX42 | NM_007372.1 | 1381 | atgtggcacgagacattgatacc | 59225 | - | | see also 5095 line |
| 12085 | DDX46 | NM_014829.1 | 1799 | ctgatcgacagacggttatgttt | 59284 | - | | see also 6032 line |
| 12086 | DDX47 | NM_016355.2 | 550 | aagccgacggaatactgaatatg | 59325 | - | | see also 6838 line |
| 12087 | DDX49 | NM_019070.3 | 88 | tcgtggctcgtggaacaatgtcg | 59362 | - | | |
| 12088 | DDX51 | NM_175066.2 | 1527 | gatggttctctgcttcactaact | 59429 | - | | |
| 12089 | DDX52 | NM_007010.2 | 39 | atggagtccacgatctctttcg | 59430 | - | | see also 4890 line |
| 12090 | DDX53 | NM_182699.1 | 1298 | cagaccgacagactgttatgaca | 59498 | - | | |
| 12091 | DDX54 | NM_024072.2 | 335 | ctgatcaccggtgttcaaagg | 59534 | - | | |
| 12092 | DHX15 | NM_001358.1 | 913 | tggagcgttatgtgtaataatt | 59559 | - | | see also 911 line |
| 12093 | DHX16 | NM_003587.3 | 416 | aagaaccgatcttatagttact | 59620 | - | | see also 2380 line |
| 12094 | DHX29 | NM_019030.1 | 3507 | ctgacgatcacaatgcatatct | 59735 | - | | |
| 12095 | DHX35 | NM_021931.2 | 495 | atggttgatcgttgttaacaaa | 59799 | - | nucleic acid binding | |
| 12096 | DHX36 | NM_020865.1 | 2380 | taggcgacgtgtttcagatacg | 59930 | - | | see also 8005 line |
| 12097 | DHX37 | NM_032656.2 | 2779 | cagctgacaccgcagtcaatgc | 59975 | - | | |
| 12098 | DHX40 | NM_024612.3 | 2010 | atgtctttgtcgcggctatttca | 59992 | - | | |
| 12099 | DIAPH3 | NM_030932.2 | 1913 | atgctcaaaccttcggatttaac | 60055 | - | | see also 8967 line |
| 12100 | DKFZP434B1727 | NM_032143.1 | 242 | aagagatatcatcgattatgaaa | 60079 | - | | |
| 12101 | DKFZP434B195 | NM_031284.3 | 916 | tagagctggccagtatgactaac | 60152 | - | | see also 9002 line |
| 12102 | DKFZP434I116 | NM_015496.3 | 806 | gaggatgatcgacgaacagtaga | 60192 | - | | |
| 12103 | DKFZp547B0714 | NM_152606.2 | 2099 | tcgtagtgtagacttagaatac | 60347 | - | transcription_regulator | |
| 12104 | DKFZP564B1023 | NM_031306.1 | 935 | ctgttatcatgcgagcttattc | 60371 | - | | see also 9009 line |

Figure 46

| | | | | | | | | | |
|---|---|---|---|---|---|---|---|---|---|
| 12105 | DKFZp761J139 | NM_032280.1 | 3680 | ccggctcttggcgaatttccttt | 60422 | - | - | - | - |
| 12106 | DKFZp762I137 | NM_152411.1 | 182 | gagactctcgtctctctagatga | 60432 | - | transcription_regulator | - | - |
| 12107 | DND1 | NM_194249.1 | 298 | acgagttccgcctgatgatgacc | 60451 | - | - | - | - |
| 12108 | DRB1 | NM_152945.1 | 610 | ttgggctacgtacgatacttaaa | 60461 | - | see also 9967 line | | |
| 12109 | ECGP | NM_018025.2 | 1856 | gcgatacgacgagttcttagtac | 60515 | - | - | - | - |
| 12110 | EHD2 | NM_014601.2 | 1073 | gcgagttcacgcttacatcatca | 60538 | - | - | nucleic acid binding | - |
| 12111 | EHD3 | NM_014600.1 | 825 | gcgggttgaccgcatcattctgc | 60540 | - | - | nucleic acid binding | - |
| 12112 | ENDOGL1 | NM_005107.1 | 242 | cagaggcaaggtgttacactaat | 60547 | - | - | endonuclease | - |
| 12113 | ENPP1 | NM_006208.1 | 243 | aagtactctcgctggtattgtca | 60570 | - | see also 4303 line | | |
| 12114 | ENPP2 | NM_006209.2 | 470 | gagtcgcattgggttgatgatga | 60650 | - | see also 4304 line | | |
| 12115 | ENPP3 | NM_005021.2 | 1261 | ccgcatccgagctcataatatac | 60768 | - | phosphatase | - | - |
| 12116 | FAM12A | NM_006683.3 | 329 | aaggggagcgaccgatatagaaa | 60818 | - | - | - | - |
| 12117 | FAM12B | NM_022360.2 | 276 | tggatcgcttccgaaatgcatat | 60836 | - | - | molecular_function unknown | - |
| 12118 | FLJ10094 | NM_017993.1 | 700 | gtgtttgtcggaggattaccaga | 60854 | - | see also 7191 line | | |
| 12119 | FLJ10252 | NM_018040.1 | 250 | gagaccattctcgaagtatatct | 60889 | - | - | - | - |
| 12120 | FLJ10290 | NM_018047.1 | 254 | tggagtccgcatgcgtttcaaga | 60931 | - | - | - | - |
| 12121 | FLJ10377 | NM_018077.1 | 676 | ttgggccgtggcaaaggataaat | 60973 | - | see also 7234 line | | |
| 12122 | FLJ10486 | NM_018109.2 | 526 | acgcgtacggtcaagtaatcagt | 61014 | - | see also 7257 line | | |
| 12123 | FLJ10634 | NM_018163.1 | 374 | caggacactagagcaagagatcg | 61058 | - | - | - | - |
| 12124 | FLJ11016 | NM_018301.2 | 417 | cggagcgtcagcgcattctttgc | 61072 | - | see also 7373 line | | |
| 12125 | FLJ11126 | NM_018332.3 | 118 | ctgtcaagtcgatgaccaatttg | 61096 | - | - | - | - |
| 12126 | FLJ11142 | NM_018338.2 | 1602 | gggtaccccgaatggtaaacttc | 61144 | - | - | - | - |
| 12127 | FLJ11175 | NM_018349.1 | 353 | gtgaaagacgtcggcattctaca | 61183 | - | - | - | - |
| 12128 | FLJ11637 | NM_024967.1 | 493 | ctgcgcaagaattgttgtactag | 61220 | - | transcription_regulator | - | - |
| 12129 | FLJ11806 | NM_024824.2 | 1611 | cagacacgagatcttgtacaacc | 61290 | - | - | - | - |
| 12130 | FLJ12455 | NM_022078.1 | 91 | gtgttgcgctcggcccatttacg | 61314 | - | - | - | - |
| 12131 | FLJ12529 | NM_024811.2 | 398 | caggttattcgctctataggagt | 61339 | - | see also 8750 line | | |
| 12132 | FLJ12606 | NM_024804.1 | 1462 | gtgtagtgactcagcctataatc | 61362 | - | transcription_regulator | - | - |
| 12133 | FLJ12644 | NM_023074.2 | 1253 | cagaagggcaatctcaacataca | 61375 | - | transcription_regulator | - | - |
| 12134 | FLJ12998 | NM_022764.1 | 491 | aggctacgccgatctggaatatg | 61408 | - | - | - | - |
| 12135 | FLJ13590 | NM_024840.2 | 1002 | aagtctcgcctcatctatcatca | 61424 | - | transcription_regulator | - | - |

Figure 46

| | | | | | | | | | |
|---|---|---|---|---|---|---|---|---|---|
| 12136 | FLJ14451 | NM_032786.1 | 738 | aggacgacgtcatgatctctatg | 61444 | - | - | - | - |
| 12137 | FLJ14779 | NM_032838.2 | 207 | tggtttcaatggggcattctatt | 61457 | - | transcription_regulator | - | - |
| 12138 | FLJ20035 | NM_017631.3 | 506 | ccgagtatgcgtatttcaacttc | 61475 | - | - | - | - |
| 12139 | FLJ20070 | NM_017652.1 | 809 | aagtcctttgcttatacatctag | 61571 | - | transcription_regulator | - | - |
| 12140 | FLJ20171 | NM_017697.1 | 976 | gagttcgccacagatattcgtac | 61598 | - | - | - | - |
| 12141 | FLJ20249 | NM_015590.2 | 295 | gggcaggatggagtacagataag | 61604 | - | - | - | - |
| 12142 | FLJ20273 | NM_019027.1 | 572 | cggctacgccttcgtcatgtact | 61614 | - | - | - | - |
| 12143 | FLJ20344 | NM_017776.1 | 566 | aaggcaaagactccctaaatatt | 61626 | - | transcription_regulator | - | - |
| 12144 | FLJ20433 | NM_017820.2 | 1951 | gagggcgctgcccctcagattcc | 61628 | - | - | - | - |
| 12145 | FLJ20557 | NM_017879.1 | 561 | tggggcatgtgcggtctttcacc | 61638 | - | transcription_regulator | - | - |
| 12146 | FLJ21839 | NM_021831.3 | 1429 | gagagtggcgttgcttactatgt | 61663 | - | see also 8182 line | | |
| 12147 | FLJ21918 | NM_024939.1 | 1415 | aggtcttgaaccgctatgcatcc | 61699 | - | see also 8811 line | | |
| 12148 | FLJ21977 | NM_032213.3 | 800 | aagctgaccggctccaagtttga | 61717 | - | apoptosis | - | - |
| 12149 | FLJ22347 | NM_022830.1 | 1196 | cggtgatgtctccctcagtaacc | 61729 | - | - | - | - |
| 12150 | FLJ22559 | NM_024928.3 | 1245 | cagcacaatggagcactactaca | 61762 | - | see also 8810 line | | |
| 12151 | FLJ22611 | NM_032226.1 | 979 | tggacccagcggtactattcagc | 61778 | - | see also 9123 line | | |
| 12152 | FLJ23233 | NM_024691.2 | 536 | taggactgctcagttcaaacatt | 61792 | - | transcription_regulator | - | - |
| 12153 | FLJ23506 | NM_024833.1 | 1298 | aagagttcagtcggaaacacaca | 61803 | - | transcription_regulator | - | - |
| 12154 | FLJ25078 | NM_152485.2 | 688 | ctgcagagggacttatctctagc | 61809 | - | - | - | - |
| 12155 | FLJ30594 | NM_153011.1 | 564 | cagtgggggttacctcctaaatc | 61818 | - | - | - | - |
| 12156 | FLJ30791 | NM_144694.1 | 634 | aagacatctgtacagtaatgtga | 61826 | - | transcription_regulator | - | - |
| 12157 | FLJ30921 | NM_152360.2 | 1167 | ttgaacatgggcagtttcatact | 61839 | - | transcription_regulator | - | - |
| 12158 | FLJ30927 | NM_144690.1 | 951 | tagtcgaagctcacagttgattg | 61853 | - | transcription_regulator | - | - |
| 12159 | FLJ30932 | NM_144693.1 | 662 | aagtcatcgtggagtgaaactca | 61871 | - | transcription_regulator | - | - |
| 12160 | FLJ31030 | NM_152478.1 | 948 | gtgcaaacttggcgcaacataag | 61900 | - | transcription_regulator | - | - |
| 12161 | FLJ31100 | NM_173631.2 | 604 | tggaggaagaccgacatttgtga | 61907 | - | transcription_regulator | - | - |
| 12162 | FLJ31139 | NM_173657.1 | 380 | gtggacgattacgccgaataact | 61922 | - | - | - | - |

502

Figure 46

| | | | | | | | | | |
|---|---|---|---|---|---|---|---|---|---|
| 12163 | FLJ31295 | NM_152320.1 | 1060 | aagacctttgggcgaagacatca | 61954 | - | transcription_regulator | - | - |
| 12164 | FLJ31875 | NM_182531.1 | 601 | cagcacttcggcaggatagatct | 61965 | - | transcription_regulator | - | - |
| 12165 | FLJ31986 | NM_152476.1 | 2552 | cagctcgtattgcacatttgaaa | 62030 | - | transcription_regulator | - | - |
| 12166 | FLJ32130 | NM_152458.4 | 858 | ctgcaagagtcgcttcacttacc | 62032 | - | transcription_regulator | - | - |
| 12167 | FLJ32191 | NM_144689.3 | 462 | gagtccaattccagggattattt | 62039 | - | transcription_regulator | - | - |
| 12168 | FLJ32932 | NM_152909.2 | 645 | gaggggatgattggataccttca | 62060 | - | transcription_regulator | - | - |
| 12169 | FLJ33071 | NM_152457.1 | 987 | acgaatctactaactgtgataaa | 62079 | - | transcription_regulator | - | - |
| 12170 | FLJ33979 | NM_152636.1 | 756 | caggcacttgcatctatcctaag | 62089 | - | - | - | - |
| 12171 | FLJ34817 | NM_152303.1 | 980 | ttgtgtggcagcgagttagatat | 62111 | - | transcription_regulator | - | - |
| 12172 | FLJ34917 | NM_153263.1 | 1734 | aggcttctaccttgaggttaaac | 62135 | - | transcription_regulator | - | - |
| 12173 | FLJ35863 | NM_152604.1 | 1635 | tagtagtggctcggatctcattc | 62150 | - | transcription_regulator | - | - |
| 12174 | FLJ36040 | NM_173530.1 | 1601 | ttgggcgcaaattatacctaacc | 62156 | - | transcription_regulator | - | - |
| 12175 | FLJ36754 | NM_173829.2 | 576 | aagcattcttctacacctaatag | 62162 | - | - | - | - |
| 12176 | FLJ36991 | NM_152477.1 | 534 | ttgaaatcggggcattcaactgg | 62169 | - | transcription_regulator | - | - |
| 12177 | FLJ38499 | NM_173797.2 | 1264 | atgccagtcgtggtactttaagc | 62209 | - | - | - | - |
| 12178 | FLJ90396 | NM_153358.1 | 510 | tggatacgagctatttgagaagc | 62230 | - | - | - | - |
| 12179 | FLJ90430 | NM_178558.2 | 1573 | ctggcctgcaactcttgctaatc | 62242 | - | - | - | - |
| 12180 | FLJ90798 | NM_153367.1 | 752 | tggggcaggagtgcatcaagtgc | 62249 | - | - | - | - |
| 12181 | GPR65 | NM_003608.2 | 1326 | aagcgaacttacacaatgtatag | 62270 | - | gpcr、receptor_acitivity、apoptosis、signal_transduction | - | - |
| 12182 | HEL308 | NM_133636.1 | 2048 | aagcgcactatcccatttggagt | 62322 | - | - | - | - |
| 12183 | HELIC1 | NM_006828.1 | 4640 | ctgtcctcgtatggctagtatga | 62505 | - | transcription_regulator | - | - |
| 12184 | HELZ | NM_014877.1 | 4621 | aggggaggctttagatcgtatac | 62699 | - | - | nucleic acid binding | - |
| 12185 | HNRNPG-T | NM_014469.2 | 894 | ccgtgactacggggatcatctga | 62727 | - | - | - | - |

Figure 46

| | | | | | | | | | |
|---|---|---|---|---|---|---|---|---|---|
| 12186 | HRB2 | NM_007043.5 | 186 | gagcagtttcgcaactttgttcc | 62731 | - | - | - | - |
| 12187 | HSA9947 | NM_022089.1 | 288 | ccgaaacactcgttatcgaaata | 62756 | - | - | - | - |
| 12188 | HSPC055 | NM_014153.2 | 569 | aagctctgtatcggaaatctaag | 62797 | - | see also 5514 line | | |
| 12189 | HTF9C | NM_022727.3 | 1116 | ctgtcggctcggcaagtacaagg | 62867 | - | - | - | - |
| 12190 | HZF12 | NM_033204.2 | 228 | ctggcctcggtcggaatccaatg | 62876 | - | - | - | - |
| 12191 | IMP-1 | NM_006546.2 | 1654 | aagttcgtatggttatcatcact | 62912 | - | - | - | - |
| 12192 | KARS | NM_005548.1 | 566 | ctgcgtcggggagacataattgg | 62929 | - | see also 3862 line | | |
| 12193 | KIAA0052 | NM_015360.2 | 735 | gcgccgagtatgccattgcattg | 62988 | - | see also 6389 line | | |
| 12194 | KIAA0082 | NM_015050.1 | 748 | cagtgtaagagcgtgtttgatgt | 63077 | - | see also 6209 line | | |
| 12195 | KIAA0150 | NM_015117.1 | 1826 | gaggggtcctctacaaagtatct | 63107 | - | - | - | - |
| 12196 | KIAA0663 | NM_014827.1 | 1812 | cagtattagaacagaagctaaag | 63109 | - | - | - | chondrosarcoma |
| 12197 | KIAA1055 | NM_015079.2 | 1601 | cagccggagcaagcatttgttaa | 63123 | - | transcription_regulator | - | - |
| 12198 | KIAA1285 | NM_015694.1 | 1568 | cggtgtagggcccatctagttgg | 63151 | - | transcription_regulator | - | - |
| 12199 | KIAA1582 | NM_018996.2 | 3710 | aagttgcgcgcacaatcactaat | 63224 | - | - | - | - |
| 12200 | KIAA1847 | NM_032527.2 | 1166 | gaggtgcacacgcgaggtatagg | 63245 | - | - | - | - |
| 12201 | LEREPO4 | NM_018471.1 | 360 | ctgtcacacatcaagttaaattt | 63250 | - | - | - | - |
| 12202 | LGALS12 | NM_033101.2 | 804 | atgtggacacgctgggtatattt | 63277 | - | - | - | - |
| 12203 | LGP2 | NM_024119.1 | 225 | tggagcttcggtcctaccaatgg | 63284 | - | - | - | - |
| 12204 | LKAP | NM_014647.1 | 969 | tggtacctccggtttgtttaaag | 63323 | - | see also 5821 line | | |
| 12205 | LOC113251 | NM_052879.3 | 726 | aagtcataagcgttgtattgtaa | 63471 | - | - | - | - |
| 12206 | LOC115509 | NM_138447.1 | 582 | tgggaaccggctgctctagatcc | 63515 | - | transcription_regulator | - | - |
| 12207 | LOC115648 | NM_145326.1 | 310 | gggacacagtacggtagtcaaac | 63524 | - | transcription_regulator | - | - |
| 12208 | LOC124245 | NM_144604.2 | 240 | cagcgggtccgatcaggatttgg | 63525 | - | - | - | - |
| 12209 | LOC126295 | NM_173480.1 | 781 | atgggttcgcaagcttcactaga | 63569 | - | transcription_regulator | - | - |
| 12210 | LOC146542 | NM_145271.2 | 411 | aagagccaaagggcctagaaagg | 63580 | - | transcription_regulator | - | - |
| 12211 | LOC147837 | NM_145276.1 | 1247 | tcgtcccagtttagttcgatatc | 63585 | - | - | - | - |
| 12212 | LOC162655 | NM_145287.1 | 810 | ctgacccattctcaaagcttact | 63594 | - | - | - | - |
| 12213 | LOC169270 | NM_173539.1 | 555 | cagtgactggttatcctttaatc | 63610 | - | transcription_regulator | - | - |

Figure 46

| 12214 | LOC170261 | NM_173798.2 | 1220 | gggctccccaccgatagtgatcg | 63636 | - | - | | - |
|---|---|---|---|---|---|---|---|---|---|
| 12215 | LOC201514 | NM_173548.1 | 508 | ttgaggatgtgacggtatatttc | 63647 | - | transcription_regulator | - | - |
| 12216 | LOC220992 | NM_145312.2 | 680 | gtgggatcgcctttatgaacagt | 63659 | - | - | - | - |
| 12217 | LOC286075 | NM_173831.2 | 619 | gggaaggaagcagctttaggaag | 63668 | - | - | - | - |
| 12218 | LOC56902 | NM_020143.2 | 378 | cagatacgctttaacttgaaatc | 63674 | - | see also 7765 line | | |
| 12219 | LOC56931 | NM_020175.1 | 433 | caggagtcggctgctaagtgttt | 63684 | - | - | - | - |
| 12220 | LOC65243 | NM_023070.1 | 942 | gaggccaagagtctaatagattt | 63701 | - | transcription_regulator | - | - |
| 12221 | LOC81691 | NM_030941.1 | 1121 | gggccactccttagatttggatc | 63745 | - | - | - | - |
| 12222 | LOC84524 | NM_032494.1 | 595 | aggatggtaaacctaaagagaag | 63781 | - | - | - | - |
| 12223 | LOC90233 | NM_138347.2 | 1411 | tagctgcaaatcggaactcattc | 63834 | - | transcription_regulator | - | - |
| 12224 | LOC90462 | NM_183238.1 | 589 | taggactgcgatcccataaatgt | 63846 | - | - | - | - |
| 12225 | LOC91120 | NM_033196.1 | 717 | ctggtgctcgagtcttactaaac | 63879 | - | - | - | - |
| 12226 | LOC92906 | NM_138394.2 | 837 | aagtgcaacgtattgttatattc | 63900 | - | see also 9611 line | | |
| 12227 | MADP-1 | NM_033114.2 | 329 | aagagtaaaggggttgcatttat | 63954 | - | see also 9361 line | | |
| 12228 | MAN1 | NM_014319.3 | 1943 | gtgtcgttctgcgttacatgaaa | 64015 | - | - | nucleic acid binding | - |
| 12229 | MBNL2 | NM_005757.3 | 1147 | tagggacaaatacggctattagc | 64053 | - | see also 4006 line | | |
| 12230 | MBNL3 | NM_018388.2 | 320 | gagattaatgggcggaacaatct | 64074 | - | - | - | - |
| 12231 | MDA5 | NM_022168.2 | 441 | tggttggactcgggaattcgtgg | 64102 | - | see also 8322 line | | |
| 12232 | MGC10433 | NM_024321.3 | 799 | ggggttgggcctgaaagagaagg | 64173 | - | - | - | - |
| 12233 | MGC10520 | NM_030580.2 | 1079 | gagctgcgagttcatcaatcacc | 64187 | - | transcription_regulator | - | - |
| 12234 | MGC10715 | NM_024325.4 | 661 | tagtggtaccagttacattcagg | 64196 | - | - | - | - |
| 12235 | MGC10871 | NM_031492.2 | 697 | atggagcactcgactactataag | 64212 | - | - | - | - |
| 12236 | MGC12466 | NM_033213.1 | 1189 | gtggtaagtcgtttacttcttcc | 64224 | - | transcription_regulator | - | - |
| 12237 | MGC13105 | NM_032497.1 | 657 | gggtggttgacaaattactctca | 64226 | - | transcription_regulator | - | - |
| 12238 | MGC17986 | NM_153608.1 | 882 | aggcaccagcggaatccatttgg | 64273 | - | transcription_regulator | - | - |

505

Figure 46

| | | | | | | | | |
|---|---|---|---|---|---|---|---|---|
| 12239 | MGC2474 | NM_023931.2 | 536 | gcgtgggtttggcgttcttctcc | 64283 | - | transcription_regulator | - |
| 12240 | MGC26914 | NM_144976.1 | 768 | tcgccaagtttatttcagatac | 64290 | - | transcription_regulator | - |
| 12241 | MGC27016 | NM_144979.2 | 141 | aagttcgaactggtattcagaat | 64294 | - | - | - |
| 12242 | MGC32104 | NM_144684.1 | 1363 | aggcctttcagccatcttgtaa | 64372 | - | transcription_regulator | - |
| 12243 | MGC33637 | NM_152596.2 | 455 | tggtgctgcgatactccatatca | 64384 | - | - | - |
| 12244 | MGC4054 | NM_024341.1 | 678 | gagcaacactcaacgaatgtaat | 64424 | - | transcription_regulator | - |
| 12245 | MGC41917 | NM_153231.2 | 1127 | tagcaatagagcccacctaattc | 64444 | - | - | - |
| 12246 | MGC42415 | NM_153363.1 | 849 | ttgcatggtttcacaactacatc | 64451 | - | - | - |
| 12247 | MGC43537 | NM_178544.2 | 1269 | agggtacaacgacatatagtca | 64470 | - | transcription_regulator | - |
| 12248 | MGC4400 | NM_032679.1 | 1119 | aagtgccggctcaatagacatca | 64497 | - | transcription_regulator | - |
| 12249 | MGC45380 | NM_133466.1 | 1360 | tagcctggttatcatcttattc | 64521 | - | transcription_regulator | - |
| 12250 | MGC45586 | NM_152603.2 | 754 | ggggaggcctgattacacatagt | 64541 | - | transcription_regulator | - |
| 12251 | MGC48625 | NM_182609.1 | 472 | cagcctgtgggactatgatgtaa | 64556 | - | transcription_regulator | - |
| 12252 | MKI67IP | NM_032390.3 | 187 | gtgcgccaacctacctaacctact | 64568 | - | transcription_regulator | - |
| 12253 | MKRN2 | NM_014160.3 | 839 | ttgcaatcacacgtactgtttgt | 64596 | - | - | nucleic acid binding |
| 12254 | MKRN3 | NM_005664.1 | 1423 | caggcatactggcatcaactg | 64614 | - | - | nucleic acid binding |
| 12255 | MNAB | NM_018835.1 | 2113 | tagactttcgtgcggatttctca | 64679 | - | - | - |
| 12256 | MSI2 | NM_138962.2 | 330 | atgagagatcccactacgaaacg | 64711 | - | see also 9643 line | - |
| 12257 | MYEOV | NM_138768.1 | 794 | cagggactcgttgctcatgttca | 64718 | - | - | - |
| 12258 | NARS | NM_004539.2 | 416 | gtgaagattggtgcgttagaagg | 64735 | - | see also 3095 line | - |
| 12259 | NPM2 | NM_182795.1 | 367 | aggctgtgcttcatatacgatttg | 64769 | - | - | - |
| 12260 | NPM3 | NM_006993.1 | 230 | gggagccaaagacgagtgtaatg | 64780 | - | - | nucleic acid binding |
| 12261 | NUPL2 | NM_007342.1 | 1253 | ttggtagtccgggctcacattct | 64799 | - | receptor_acitivity | - |
| 12262 | PCTAIRE2 BP | NM_014290.1 | 2167 | aggttgaagcgatgctacacaat | 64864 | - | see also 5597 line | |

Figure 46

| ID | Name | Accession | | Sequence | | Annotation | | | Disease |
|---|---|---|---|---|---|---|---|---|---|
| 12263 | PDIP46 | NM_032311.3 | 145 | gcgggcgaaaggacggcttaatgc | 64884 | | - | see also 9152 line | |
| 12264 | PERC | NM_133263.2 | 2797 | aagaggcgcttgaagtgtttgg | 64927 | | - | receptor_acitivity, transcription_regulator, signal_transduction | |
| 12265 | PINX1 | NM_017884.3 | 1061 | aggacggctacactagaagaaacg | 64948 | | - | see also 7170 line | |
| 12266 | PNMA3 | NM_013364.1 | 398 | tcggtcggacaccaattgttcg | 64963 | | - | - | |
| 12267 | PPIL4 | NM_139126.2 | 1374 | gagggatggccattatagtaata | 65011 | | - | see also 9660 line | |
| 12268 | PPP1R10 | NM_002714.2 | 683 | tggtgagtcgatgcacttacttg | 65019 | | - | transcription_regulator | |
| 12269 | PRC | NM_015062.3 | 2749 | ttgtctatgggcgcagtactacc | 65075 | | - | - | |
| 12270 | PRR3 | NM_025263.1 | 452 | cagacacgatgccgaaacgaaag | 65100 | molecular_function unknown | - | | |
| 12271 | PSPC1 | NM_018282.1 | 627 | cggattcgcttcgctcacacatgg | 65111 | | - | see also 7362 line | |
| 12272 | PTBP2 | NM_021190.1 | 127 | cagtagtccgaactctaatga | 65137 | | - | see also 8123 line | |
| 12273 | RAD52B | NM_145654.2 | 219 | tggtttctatgccgtcattaagt | 65275 | | - | - | |
| 12274 | RAVER1 | NM_133452.1 | 340 | ccgagcgccgcaatcaatgcttc | 65297 | | - | - | |
| 12275 | RBAK | NM_021163.2 | 2628 | aggaaatatgaacgtactgatg | 65326 | | - | transcription_regulator | |
| 12276 | RBM14 | NM_006328.1 | 2027 | gggctcctataatgattcactgc | 65351 | | - | see also 4430 line | |
| 12277 | RECQL4 | NM_004260.1 | 1038 | ccgccatgacagggcgaattacg | 65355 | DNA helicase | - | | osteosarcoma, Rothmund-Thomson syndrome |
| 12278 | RECQL5 | NM_004259.3 | 1144 | caggtttgtcgcccattggaata | 65376 | DNA helicase | - | | |
| 12279 | RIG-I | NM_014314.2 | 1459 | ttgatgcgtcagtgatagcaaca | 65424 | | - | see also 5617 line | |
| 12280 | RNASE2 | NM_002934.1 | 184 | ttgaaacccagcacatcaatatg | 65470 | hematopoietic-associated factor 1A complex | - | | |
| 12281 | RNASE3 | NM_002935.1 | 366 | tagattccggtgcctttactcc | 65486 | toxin | - | | |
| 12282 | RNASE4 | NM_002937.3 | 709 | gtgatcgctactgcaacttgatg | 65491 | | - | | |
| 12283 | RNASE6 | NM_005615.2 | 695 | gagcgatccccctacaagttgg | 65505 | pancreatic ribonuclease | - | | |
| 12284 | RNASE8 | NM_138331.1 | 155 | cagccatgagcatcatcaataag | 65510 | | - | | |
| 12285 | RNPC4 | NM_018107.3 | 341 | tcgggagacaagcaatcgtagt | 65516 | | - | | |
| 12286 | RNPC6 | NM_153020.1 | 624 | ctgcagacagaccgaatgcaata | 65547 | | - | | |
| 12287 | RoXaN | NM_017590.4 | 711 | gcgagttcgcaaggcgtataaga | 65557 | | - | | |
| 12288 | RSN | NM_002956.2 | 4415 | ctgcaatgacgacgaaaacttct | 65642 | | - | see also 1994 line | |
| 12289 | RY1 | NM_006857.1 | 401 | atgcctatgccataaatgtctct | 65644 | | - | see also 4804 line | |
| 12290 | SART3 | NM_014706.2 | 1104 | ctgtacataaccgcgctattaga | 65663 | | - | | |
| 12291 | SBNO1 | NM_018183.2 | 3260 | aagatcgctcgggaattcttact | 65802 | | - | | |

Figure 46

| | | | | | | | | | |
|---|---|---|---|---|---|---|---|---|---|
| 12292 | SBZF3 | NM_020394.2 | 480 | gagaaattacgcttaaggaatga | 65854 | - | transcription_regulator | - | - |
| 12293 | SECP43 | NM_017846.3 | 610 | cagcagtaccagaactactatgc | 65918 | - | - | - | - |
| 12294 | SFRS12 | NM_139168.1 | 1640 | acgttcaacgtctatgagaaaga | 65949 | - | see also 9672 line | | |
| 12295 | SHARP | NM_015001.2 | 419 | gagacctacgcacggattataat | 65962 | - | see also 6175 line | | |
| 12296 | SIAHBP1 | NM_014281.3 | 402 | gcgtctacgtgggcctctatctac | 66147 | - | see also 5589 line | | |
| 12297 | SLM1 | NM_152688.1 | 1101 | tggctacggggggtgaatatgatg | 66157 | - | - | - | - |
| 12298 | SND1 | NM_014390.1 | 2637 | cagcgtagttcgggatatccaga | 66213 | - | see also 5650 line | | |
| 12299 | SPF45 | NM_032905.3 | 1238 | tagaatttgagagagttgaatca | 66244 | - | see also 9342 line | | |
| 12300 | SRP46 | NM_032102.1 | 852 | cagctcatctggttacagtaact | 66247 | - | - | - | - |
| 12301 | SRrp35 | NM_080743.2 | 230 | tggccctatagtagacgtttaca | 66252 | - | - | - | - |
| 12302 | SSX4 | NM_005636.3 | 92 | agggatgatgctcaaatatcaga | 66279 | - | transcription_regulator | - | synovial sarcoma |
| 12303 | STK31 | NM_031414.2 | 1205 | tggatgaagggtgctttactact | 66312 | - | see also 9015 line | | |
| 12304 | SZF1 | NM_016089.1 | 1212 | tgggcgaggctttatagttgagt | 66382 | - | - | - | - |
| 12305 | T54 | NM_015698.3 | 1115 | atgtacaaaggaggccaatatta | 66401 | - | - | molecular_function unknown | - |
| 12306 | TDRD1 | NM_198795.1 | 953 | agggtacggttaccgaattcaaa | 66427 | - | - | - | - |
| 12307 | TDRD3 | NM_030794.1 | 1387 | gtgatagaccgtattctagatat | 66558 | - | see also 8950 line | | |
| 12308 | TDRD4 | NM_019038.2 | 1472 | tggcgctgtcagagtacaatatt | 66625 | - | - | - | - |
| 12309 | TDRKH | NM_006862.2 | 599 | cggcgagacaattcgttctatct | 66669 | - | - | - | - |
| 12310 | TFIP11 | NM_012143.1 | 2674 | ttggccgcattgtgatctcacatc | 66747 | - | - | protein binding | - |
| 12311 | THOC4 | NM_005782.1 | 59 | tggacgacatcattaaactgaac | 66750 | - | - | - | - |
| 12312 | TIPARP | NM_015508.2 | 1324 | gagtatcccgagtcgtcattcg | 66790 | - | see also 6442 line | | |
| 12313 | TNRC6 | NM_014494.1 | 5324 | cagccggaccgatctcaatcact | 66924 | - | see also 5711 line | | |
| 12314 | TOE1 | NM_025077.2 | 1522 | gtgtcctcagtctcacgatattg | 66964 | - | transcription_regulator | - | - |
| 12315 | TRAF6 | NM_004620.2 | 1154 | gagggtcgccttgtaagacaaga | 67013 | - | see also 3136 line | | |
| 12316 | TRF4-2 | NM_022447.1 | 431 | atggacgacacttcaattattta | 67039 | - | - | - | - |
| 12317 | TRIM56 | NM_030961.1 | 1929 | tagcgcacggctctatctcatca | 67058 | - | - | - | - |
| 12318 | U1SNRNPBP | NM_007020.2 | 956 | gaggagcgtgccgtgatcaaagc | 67071 | - | - | - | - |
| 12319 | UNKL | NM_024023.1 | 1651 | gtgcgcgctcacggtgtttatgt | 67099 | - | - | - | - |
| 12320 | USP6 | NM_004505.1 | 1803 | ctgagcccgttggaatcaacagc | 67107 | - | - | ubiquitin C-terminal hydrolase | Ewing's sarcoma |
| 12321 | ZC3HAV1 | NM_020119.3 | 1943 | aagggttgtccgcttaatggtag | 67191 | - | - | - | - |
| 12322 | ZC3HDC1 | NM_022750.1 | 1793 | aagcacaactacgagttagattt | 67256 | - | - | - | - |
| 12323 | ZCCHC2 | NM_017742.2 | 764 | atgggcttaccatgcaatattct | 67287 | - | - | - | - |

Figure 46

| 12324 | ZCCHC5 | NM_152694.1 | 304 | aggacttagcagcctcctatatt | 67324 | - | - | - | - |
|---|---|---|---|---|---|---|---|---|---|
| 12325 | ZNF183L1 | NM_178861.3 | 720 | ttgaagagggtcgctactgtatc | 67378 | - | - | - | - |
| 12326 | ZNF2 | NM_021088.1 | 1462 | ctggggaccctcgctatcagtgt | 67386 | - | transcription_regulator | - | - |
| 12327 | ZNF227 | NM_182490.1 | 1204 | cagtagcagcacgggtcttatca | 67400 | - | transcription_regulator | - | - |
| 12328 | ZNF248 | NM_021045.1 | 1293 | tggaaatggagccgtatggatgc | 67433 | - | transcription_regulator | - | - |
| 12329 | ZNF350 | NM_021632.2 | 1457 | atgtcgtgtctggttaagcataa | 67454 | - | transcription_regulator | - | - |
| 12330 | ZNF354B | NM_058230.1 | 1024 | gtgcatcctttgtaagcattta | 67467 | - | transcription_regulator | - | - |
| 12331 | ZNF354C | NM_014594.1 | 1297 | acgtattgtaacccttatcgaac | 67487 | - | - | - | - |
| 12332 | ZNF415 | NM_018355.2 | 762 | gagtgcgacaaagccttgaatca | 67500 | - | - | - | - |
| 12333 | ZNF426 | NM_024106.1 | 1550 | ttgggtatccctcatgtcttaat | 67526 | - | transcription_regulator | - | - |
| 12334 | ZNF454 | NM_182594.1 | 950 | cagagtacgcaccttatccatca | 67537 | - | transcription_regulator | - | - |
| 12335 | ZNF471 | NM_020813.1 | 1545 | atgtacaccttgttagtcatttg | 67553 | - | transcription_regulator | - | - |
| 12336 | ZNF514 | NM_032788.1 | 728 | ttgtgatggccagttagagatgc | 67565 | - | transcription_regulator | - | - |
| 12337 | ZNF75A | NM_153028.1 | 918 | atggatcgtcacaagaaagattg | 67581 | - | - | - | - |
| 12338 | ZNF77 | NM_021217.1 | 491 | gagttaccctaccgaagctaaac | 67588 | - | transcription_regulator | - | - |
| 12339 | ZNF92 | NM_007139.2 | 902 | cagttctcgattcttaataaaca | 67605 | - | transcription_regulator | - | - |
| 12340 | 13CDNA73 | NM_023037.1 | 5470 | ctgggcgcttcatctaccattat | 67746 | - | see also 8545 line | | |
| 12341 | AAK1 | NM_014911.2 | 715 | acgtgagtagcggtgatgtatgg | 67853 | - | kinase_related | - | - |
| 12342 | AARSL | NM_020745.1 | 2252 | gtgggacgcacctgttacgtact | 67914 | - | - | - | - |
| 12343 | ABCA1 | NM_005502.2 | 3457 | cggggatctaaggttgtcattc | 67973 | - | see also 3842 line | | |
| 12344 | ABCA10 | NM_080282.2 | 3266 | aagtaactcgtgaaactcattgt | 68089 | - | kinase_related | - | - |
| 12345 | ABCA12 | NM_015657.3 | 5692 | cagtcggtcctagacttcttaaa | 68289 | - | see also 6534 line | | |
| 12346 | ABCA13 | NM_152701.2 | 12090 | gagaaggtcgtacgatcatcttc | 68687 | - | - | - | - |
| 12347 | ABCA2 | NM_001606.2 | 1595 | gtgggcaacgtgactcactatgc | 68766 | - | see also 1067 line | | |
| 12348 | ABCA3 | NM_001089.1 | 1027 | cagttacacacggagaaattaca | 68807 | - | - | ATP-binding cassette (ABC) transporter | - |

Figure 46

| | | | | | | | | | |
|---|---|---|---|---|---|---|---|---|---|
| 12349 | ABCA4 | NM_000350.1 | 867 | cagccgttctcaaggtatcaatc | 68851 | - | see also 291 line | | |
| 12350 | ABCA5 | NM_018672.2 | 5754 | atgtatcggaacagtacaacatc | 69087 | - | see also 7544 line | | |
| 12351 | ABCA6 | NM_080284.2 | 4342 | gaggctcgccatcgcaagattag | 69199 | - | - | - | - |
| 12352 | ABCA7 | NM_019112.2 | 5059 | atgtggtgctcacctgcataaac | 69220 | - | - | - | - |
| 12353 | ABCA8 | NM_007168.1 | 2565 | aagggttcgcttgttaaagttaa | 69288 | - | see also 4971 line | | |
| 12354 | ABCA9 | NM_080283.2 | 2942 | gaggcgacagaacatagctatag | 69403 | - | see also 9513 line | | |
| 12355 | ABCB1 | NM_000927.2 | 932 | gagcttaacacccgacttacaga | 69444 | - | see also 604 line | | |
| 12356 | ABCB10 | NM_012089.1 | 1097 | cagctagctgaggaacgtattgg | 69523 | - | - | - | - |
| 12357 | ABCB11 | NM_003742.2 | 1353 | tggttacaagttggatcgaatca | 69579 | - | see also 2554 line | | |
| 12358 | ABCB4 | NM_000443.2 | 3643 | ctgtctacggtccgaaatgcaga | 69705 | - | see also 350 line | | |
| 12359 | ABCB5 | NM_178559.3 | 503 | gcggcattatcgagaccatattg | 69758 | - | - | - | - |
| 12360 | ABCB6 | NM_005689.1 | 2033 | cagaagggccgtattgagtttga | 69839 | - | see also 3949 line | | |
| 12361 | ABCB7 | NM_004299.2 | 1269 | gtggcaggtacccttactgttgg | 69880 | - | - | heme transporter | - |
| 12362 | ABCB8 | NM_007188.2 | 483 | gggtgcggcactcgtgaatgtac | 69923 | - | - | ATP-binding cassette (ABC) transporter | - |
| 12363 | ABCB9 | NM_019624.1 | 1879 | cagcgcctacgaccacaagtact | 69956 | - | see also 7732 line | | |
| 12364 | ABCC1 | NM_004996.2 | 3159 | ccgcgctggcttccaactattgg | 69991 | - | see also 3489 line | | |
| 12365 | ABCC10 | NM_033450.2 | 1710 | gggcgactccgggtcatcaaata | 70017 | - | see also 9427 line | | |
| 12366 | ABCC11 | NM_032583.2 | 903 | gtgctatggacccctagtactga | 70056 | - | - | - | - |
| 12367 | ABCC12 | NM_033226.1 | 997 | tggcgaggtgctcaatatactgt | 70117 | - | see also 9384 line | | |
| 12368 | ABCC13 | NM_138726.2 | 597 | aagaaaccatctctactctatgc | 70207 | - | - | - | - |
| 12369 | ABCC2 | NM_000392.1 | 321 | cagtccctgctgttcgatatacc | 70234 | - | see also 311 line | | |
| 12370 | ABCC3 | NM_003786.2 | 2347 | cagtctggctcgagctgtttaca | 70337 | - | - | - | - |
| 12371 | ABCC4 | NM_005845.2 | 2848 | ctggaccatccgggcatacaaag | 70451 | - | channel | ATP-binding cassette (ABC) transporter | - |
| 12372 | ABCC5 | NM_005688.1 | 4301 | aggctccgataggattatggtgc | 70575 | - | see also 3945 line | | |
| 12373 | ABCC6 | NM_001171.2 | 1858 | ctggtgtcgtagactcaagttcc | 70589 | - | - | translation regulator | |
| 12374 | ABCC8 | NM_000352.2 | 2396 | cagcgaccgacttggatatcagg | 70623 | - | receptor_acitivity | sulfonylurea receptor | diabetes mellitus、diabetes |
| 12375 | ABCC9 | NM_005691.1 | 1397 | ttgcgccaattcagtactttatt | 70683 | - | see also 3953 line | | |
| 12376 | ABCD1 | NM_000033.2 | 1351 | tggaacgcctgtggtatgttatg | 70784 | - | see also 5 line | | |
| 12377 | ABCD2 | NM_005164.2 | 1816 | ctgctcgagtgtacaatatgttt | 70845 | - | see also 3628 line | | |
| 12378 | ABCD4 | NM_005050.1 | 93 | caggttagatctgcaatttctcc | 70948 | - | see also 3525 line | | |
| 12379 | ABCE1 | NM_002940.1 | 317 | ttggcgccttatcaattgtcaat | 70987 | - | see also 1974 line | | |
| 12380 | ABCF2 | NM_005692.3 | 1231 | agggtcgtgagcgataagacact | 71028 | - | see also 3956 line | | |
| 12381 | ABCF3 | NM_018358.1 | 135 | cggagcgagttccccgaaattga | 71046 | - | see also 7412 line | | |

Figure 46

| | | | | | | | | | |
|---|---|---|---|---|---|---|---|---|---|
| 12382 | ABCG1 | NM_004915.2 | 809 | aagggggtcgctccatcatttgc | 71078 | - | - | - | - |
| 12383 | ABCG2 | NM_004827.1 | 1917 | cagcattccacgatatggattta | 71138 | - | - | xenobiotic-transporting ATPase | - |
| 12384 | ABCG4 | NM_022169.2 | 370 | aaggggcagatcctggttaatgg | 71154 | - | see also 8326 line | | |
| 12385 | ABCG5 | NM_022436.2 | 962 | tggcacgccagcggaaatgcttg | 71168 | - | - | molecular_function unknown | Sitosterolemia(-)、atherosclerosis |
| 12386 | ABCG8 | NM_022437.2 | 1291 | acgctgatccgtcgtcagatttc | 71211 | - | see also 8356 line | | |
| 12387 | ABL2 | NM_005158.2 | 2496 | tggggatctagccattacagaga | 71284 | - | kinase_related | - | - |
| 12388 | ACACB | NM_001093.1 | 6822 | aaggatcgatccagcttacaaga | 71548 | - | - | Transcription factor E2-alpha (E47) | - |
| 12389 | ACK1 | NM_005781.2 | 831 | acgcaagtcgtggatgagtaagg | 71555 | - | kinase_related、signal_transduction | - | - |
| 12390 | ACLY | NM_001096.2 | 1271 | gagcacgaagtcacaatctttgt | 71582 | - | see also 682 line | | |
| 12391 | ACVR1 | NM_001105.2 | 824 | gtggagtatggcactatcgaagg | 71611 | - | see also 686 line | | |
| 12392 | ACVR1B | NM_004302.3 | 1253 | atgccctcgggcttgtatattgg | 71651 | - | receptor_acitivity、kinase_related、signal_transduction | - | - |
| 12393 | ACVR1C | NM_145259.1 | 1664 | ctgctcttcgtattaagaagact | 71684 | - | receptor_acitivity、kinase_related | - | - |
| 12394 | ACVR2 | NM_001616.2 | 786 | aagcccagttgcttaacgaatat | 71718 | - | see also 1070 line | | |
| 12395 | ACVR2B | NM_001106.2 | 935 | gagggccacaagccgtctattgc | 71744 | - | see also 687 line | | |
| 12396 | ACVRL1 | NM_000020.1 | 1006 | gagactgagatctataacacagt | 71754 | - | receptor_acitivity、signal_transduction | transforming growth factor-beta receptor | Rendu-Osler-Weber disease、hereditary hemorrhagic teleangiectasia |
| 12397 | ADRBK1 | NM_001619.2 | 956 | gaggctgacatgcgcttctatgc | 71770 | - | kinase_related、gpcr、signal_transduction | G-protein coupled receptor kinase | - |
| 12398 | ADRBK2 | NM_005160.2 | 448 | tagaacacgtacaaagtcattta | 71783 | - | see also 3627 line | | |
| 12399 | AFG3L1 | NM_001132.1 | 892 | atgcggcctggccggtttcatcg | 71829 | - | - | metallopeptidase | - |
| 12400 | AFURS1 | NM_024524.2 | 3343 | cagaggctattccggttaaattg | 71876 | - | - | - | - |
| 12401 | AK1 | NM_000476.1 | 550 | ccgtcatcgccttctatgagaaa | 71896 | - | kinase_related | adenylate kinase | hemolytic anemia |
| 12402 | AK3L1 | NM_016282.2 | 535 | ccgccagtggccgagtctataac | 71913 | - | see also 6779 line | | |
| 12403 | AK5 | NM_012093.2 | 2610 | ctgcacagctattgactctattt | 71959 | - | kinase_related | - | - |
| 12404 | AKT2 | NM_001626.2 | 291 | aagagcgacggctccttcattgg | 71966 | - | see also 1078 line | | |
| 12405 | ALS2CR2 | NM_018571.4 | 1223 | tagcccattggatatcagtattt | 72021 | - | see also 7527 line | | |
| 12406 | ALS2CR7 | NM_139158.1 | 282 | aaggctcttatgcgacagtttac | 72025 | - | kinase_related | - | - |
| 12407 | AMHR2 | NM_020547.1 | 138 | caggcgaacctgtgtgttctttg | 72049 | - | see also 7900 line | | |
| 12408 | ANKK1 | NM_178510.1 | 1118 | cagctctccgaccgtaagaattt | 72074 | - | kinase_related | - | - |
| 12409 | ANKRD3 | NM_020639.1 | 1411 | aagtggctgctgctcaacaatgc | 72091 | - | kinase_related | protein tyrosine kinase | - |

Figure 46

| | | | | | | | | | |
|---|---|---|---|---|---|---|---|---|---|
| 12410 | APAF1 | NM_001160.2 | 1215 | aagaccgtctccgcattctgatg | 72111 | - | see also 734 line | | |
| 12411 | APG-1 | NM_014278.2 | 420 | tagtcacgaacgtaagaaataca | 72220 | - | see also 5577 line | | |
| 12412 | ARAF1 | NM_001654.1 | 256 | gggcaccgtcaaagtatacctgc | 72293 | - | see also 1094 line | | |
| 12413 | ARK5 | NM_014840.1 | 2131 | atgcccttcgatggtttcgatca | 72324 | - | - | protein tyrosine kinase | - |
| 12414 | ASNA1 | NM_004317.1 | 639 | ccgctcagtcagcgaacagttca | 72347 | - | - | arsenite-transporting ATPase | |
| 12415 | ASS | NM_000050.2 | 553 | ctgatggagtacgcaaagcaaca | 72352 | - | - | - | citrullinuria |
| 12416 | ATP10A | NM_024490.2 | 923 | aggtttcgcggctgcatcataca | 72359 | - | see also 8619 line | | |
| 12417 | ATP10D | NM_020453.2 | 2347 | cagacgaagcggccttagtgtat | 72469 | - | - | plasma membrane cation-transporting ATPase | Alzheimer disease |
| 12418 | ATP12A | NM_001676.2 | 867 | aggggtgtcgggtggataactca | 72525 | - | - | plasma membrane cation-transporting ATPase | - |
| 12419 | ATP1A1 | NM_000701.5 | 1976 | atgtgaatttccctatcgataat | 72612 | - | - | sodium/potassium-exchanging ATPase | - |
| 12420 | ATP1A2 | NM_000702.1 | 1350 | tggacggccctgtctcgaattgc | 72633 | - | see also 431 line | | |
| 12421 | ATP1A3 | NM_152296.2 | 235 | aggtctgccggaaatacaacaca | 72646 | - | see also 9834 line | | |
| 12422 | ATP1A4 | NM_144699.1 | 3019 | ctggtgaaccaccgtctcattgg | 72670 | - | - | - | - |
| 12423 | ATP2A2 | NM_001681.2 | 566 | aagagtgtgcagcggattaaagc | 72716 | - | see also 1102 line | | |
| 12424 | ATP2A3 | NM_005173.2 | 1018 | gcgtggcgctgtctactacttca | 72784 | - | - | - | - |
| 12425 | ATP2B1 | NM_001682.1 | 3592 | aagaagttcgattcacaacttta | 72907 | - | - | plasma membrane cation-transporting ATPase | - |
| 12426 | ATP2B3 | NM_021949.1 | 1341 | gtgcacgccggtctatgtacaat | 72947 | - | - | plasma membrane cation-transporting ATPase | - |
| 12427 | ATP2C1 | NM_014382.1 | 2351 | cagctgagcacgagtatagcagc | 73027 | - | see also 5642 line | | |
| 12428 | ATP4A | NM_000704.1 | 2011 | gtgcccgtagaccaggttaatcg | 73069 | - | see also 433 line | | |
| 12429 | ATP5A1 | NM_004046.3 | 674 | cagcgtgaactgattattggtga | 73092 | - | - | hydrogen-transporting two-sector ATPase | - |
| 12430 | ATP5B | NM_001686.2 | 800 | aaggtagcgctggtatatggtca | 73127 | - | channel | hydrogen-translocating F-type ATPase | - |
| 12431 | ATP6AP1 | NM_001183.3 | 949 | ctgacctatgaacgactctttgg | 73153 | - | - | hydrogen-transporting two-sector ATPase | - |

Figure 46

| | | | | | | | | | |
|---|---|---|---|---|---|---|---|---|---|
| 12432 | ATP6V1A | NM_001690.2 | 565 | atgttaccccccacgaaacagagg | 73179 | - | see also 1107 line | | |
| 12433 | ATP6V1B1 | NM_001692.2 | 1540 | ccgtgatcgacgagttctattcc | 73243 | - | - | hydrogen-translocating V-type ATPase | renal tubular acidosis |
| 12434 | ATP6V1B2 | NM_001693.2 | 1234 | gggagaaacggctcgattactca | 73269 | - | see also 1110 line | | |
| 12435 | ATP6V1C2 | NM_144583.1 | 102 | aagtccaacctgtcttataatac | 73332 | - | see also 9706 line | | |
| 12436 | ATP6V1H | NM_015941.1 | 495 | cagaccgttcagtatatactaac | 73361 | - | see also 6582 line | | |
| 12437 | ATP7A | NM_000052.1 | 3772 | aggtcggactgctgtattagtag | 73498 | - | see also 21 line | | |
| 12438 | ATP7B | NM_000053.1 | 3624 | gaggcgcaacggtttaaccattt | 73587 | - | see also 26 line | | |
| 12439 | ATP8A1 | NM_006095.1 | 1163 | ctgctatggctcgaacatctaat | 73630 | - | see also 4236 line | | |
| 12440 | ATP8A2 | NM_016529.3 | 2434 | gtggtcatgctaccgactattta | 73762 | - | see also 6904 line | | |
| 12441 | ATP8B1 | NM_005603.1 | 2758 | atgtcgagtgactattcctttgc | 73869 | - | - | plasma membrane cation-transporting ATPase | benign recurrent intrahepatic cholestasis、familial intrahepatic cholestasis、intrahepatic cholestasis、Alzheimer disease |
| 12442 | ATSV | NM_004321.3 | 1585 | gcggcaccttgggcgtattctct | 73903 | - | see also 2893 line | | |
| 12443 | AURKB | NM_004217.1 | 240 | cagtgggacacccgacatcttaa | 73928 | - | kinase_related、cell_cycle | - | - |
| 12444 | AURKC | NM_003160.1 | 670 | cagcgcacagccacgataataga | 73946 | - | kinase_related、cell_cycle | - | - |
| 12445 | AXL | NM_001699.3 | 1857 | aagaaggagacccgttatgggaga | 73957 | - | see also 1112 line | | |
| 12446 | BCKDK | NM_005881.1 | 1224 | tggaggaatcgctcacaaagatc | 73982 | - | see also 4092 line | | |
| 12447 | BCS1L | NM_004328.3 | 933 | cagacgtggctacctgctttatg | 74000 | - | see also 2896 line | | |
| 12448 | BLK | NM_001715.2 | 1177 | aggccctggtgcagcactattct | 74011 | - | - | protein tyrosine kinase | - |
| 12449 | BMP2K | NM_017593.3 | 1368 | ctggtgaattcggtaaccataga | 74064 | - | see also 7045 line | | |
| 12450 | BMPR1A | NM_004329.1 | 658 | cagctacgccggacaatagaatg | 74102 | - | see also 2897 line | | |
| 12451 | BMPR1B | NM_001203.1 | 1498 | gaggttgctaggagatgtgtatc | 74141 | - | receptor_acitivity、signal_transduction、kinase_related | transforming growth factor-beta receptor | Alzheimer disease、Aarskog-Scott syndrome |
| 12452 | BMPR2 | NM_001204.4 | 1221 | ctgattggccgaggtcgatatgg | 74169 | - | see also 788 line | | |
| 12453 | BMS1L | NM_014753.1 | 1742 | cagctctccccactttcgattct | 74252 | - | - | - | - |
| 12454 | BMX | NM_001721.3 | 700 | tagcccaatatgacaacgaatca | 74270 | - | kinase_related | signal transducer | - |
| 12455 | BRAF | NM_004333.2 | 588 | cagtccgagacagtctaaagaaa | 74313 | - | kinase_related、apoptosis | receptor signaling protein | melanoma |
| 12456 | BTK | NM_000061.1 | 1147 | aggggtgatacgtcattatgttg | 74387 | - | see also 36 line | | |
| 12457 | BUB1 | NM_004336.1 | 676 | aagagtgatcacgatttctaaat | 74412 | - | see also 2898 line | | |

Figure 46

| | | | | | | | | | |
|---|---|---|---|---|---|---|---|---|---|
| 12458 | BUB1B | NM_001211.3 | 417 | gagagtaatatgtcaacgttatt | 74485 | - | kinase_related、cell_cycle | protein serine/threonine kinase | - |
| 12459 | C14orf127 | NM_025152.1 | 237 | ttgcactagcagcgaacgattcg | 74556 | - | - | - | - |
| 12460 | C14orf131 | NM_018335.1 | 1126 | ttgtcccctccaggtacaatagt | 74599 | - | - | - | - |
| 12461 | C14orf20 | NM_174944.1 | 1134 | cagctccacaacaccactaaaca | 74608 | - | kinase_related | - | - |
| 12462 | C20orf23 | NM_017683.1 | 1610 | atgcttccacggagcaagatatt | 74651 | - | - | - | - |
| 12463 | C20orf64 | NM_033550.2 | 562 | atgcttccaactgcttatatatg | 74712 | - | kinase_related | - | - |
| 12464 | C20orf97 | NM_021158.2 | 598 | gagttggatgacaacttagatac | 74721 | - | kinase_related、apoptosis | - | - |
| 12465 | C6orf199 | NM_145025.2 | 918 | ttgttatggatcgacttaaatat | 74740 | - | kinase_related | - | - |
| 12466 | C8FW | NM_025195.2 | 1614 | cgggtacatcgactcagaaatag | 74757 | - | kinase_related | - | - |
| 12467 | CACNA1B | NM_000718.1 | 4513 | ccggcagtcgttccagtataaga | 74790 | - | see also 435 line | | |
| 12468 | CAD | NM_004341.2 | 5393 | aggggaggttgcctatatcgatg | 74902 | - | - | dihydroorotase | - |
| 12469 | CAMK1 | NM_003656.3 | 572 | ctgcatgacctgggcattgtaca | 74917 | - | kinase_related、signal_transduction | calmodulin-dependent protein kinase I | - |
| 12470 | CAMK1D | NM_020397.1 | 296 | tagccgtcctgagaaagattaag | 74927 | - | kinase_related | - | - |
| 12471 | CAMK1G | NM_020439.1 | 405 | caggtcttgtcggcagtgaaata | 74955 | - | kinase_related | - | - |
| 12472 | CAMK2A | NM_015981.2 | 394 | gggacaccactacctgatcttcg | 74970 | - | see also 6591 line | | |
| 12473 | CAMK2B | NM_001220.3 | 399 | ccgccttctgaagcattccaaca | 74985 | - | see also 800 line | | |
| 12474 | CAMK2D | NM_001221.2 | 1778 | atgccgcctgcatagcatatatt | 75020 | - | see also 801 line | | |
| 12475 | CAMK2G | NM_001222.2 | 593 | tggcctagccatcgaagtacagg | 75038 | - | see also 803 line | | |
| 12476 | CAMK4 | NM_001744.3 | 216 | gggatgcgctgagcgatttcttc | 75051 | - | see also 1154 line | | |
| 12477 | CAMKK1 | NM_032294.2 | 303 | ctgtgatccctggcagtacttca | 75080 | - | see also 9146 line | | |
| 12478 | CAMKK2 | NM_006549.3 | 1723 | aggcatcgagtacttacactacc | 75102 | - | kinase_related、transcription_regulator | - | - |
| 12479 | CARD12 | NM_021209.3 | 2027 | cagggaacatccccgattactta | 75154 | - | apoptosis | apoptosis regulator | - |
| 12480 | CARD15 | NM_022162.1 | 582 | gggtttcgtcagccagtatgaat | 75187 | - | see also 8318 line | | |
| 12481 | CARD4 | NM_006092.1 | 2332 | ccgcgttcaggtcgaaagcttca | 75229 | - | apoptosis、signal_transduction、kinase_related | tropomyosin binding | - |
| 12482 | CARK | NM_015978.1 | 562 | ttgcatattgcagcgtactatgg | 75264 | - | kinase_related | - | - |
| 12483 | CASK | NM_003688.1 | 98 | gtgttgtacgacgatgtatcaac | 75317 | - | see also 2506 line | | |
| 12484 | CCRK | NM_012119.2 | 808 | gggcaagaacgatattgaacagc | 75403 | - | kinase_related | - | - |
| 12485 | CCT2 | NM_006431.1 | 542 | ttgcgggcacaacattatcctca | 75424 | - | cell_cycle | chaperone | - |
| 12486 | CCT3 | NM_005998.2 | 174 | cagatatcatccgaacatgtttg | 75434 | - | - | chaperone | - |
| 12487 | CCT4 | NM_006430.2 | 1440 | gagttggccctacgattaactga | 75504 | - | see also 4500 line | | |

Figure 46

| | | | | | | | | | |
|---|---|---|---|---|---|---|---|---|---|
| 12488 | CCT5 | NM_012073.2 | 535 | cagcggatagcgtccttgttgaca | 75531 | - | - | chaperone | |
| 12489 | CCT6A | NM_001762.2 | 1527 | aagtaggcgtatggcgataactat | 75574 | - | - | chaperone | |
| 12490 | CCT6B | NM_006584.1 | 381 | ctgcaccctagaataatagctga | 75583 | - | - | protein transporter | |
| 12491 | CCT7 | NM_006429.1 | 613 | cagtgatgatcgcgatgatttg | 75627 | - | cell_cycle | chaperone | |
| 12492 | CCT8 | NM_006585.1 | 885 | ctggtgcaaatgtcgtagtaaca | 75678 | - | - | chaperonin ATPase | |
| 12493 | CDC2 | NM_001786.2 | 656 | tggggtcagctcgttactcaact | 75723 | - | kinase_related, cell_cycle | | |
| 12494 | CDC2L5 | NM_003718.2 | 2730 | aagtcggccgtatactaacaagg | 75779 | - | kinase_related | | |
| 12495 | CDC42BPA | NM_003607.2 | 4748 | gtgatctcaggacgaaatcgtca | 75942 | - | kinase_related | | |
| 12496 | CDC42BPB | NM_006035.2 | 2733 | aagctcagattgcggaaatcatt | 75996 | - | see also 4206 line | | |
| 12497 | CDC6 | NM_001254.2 | 1142 | tggtgctgattgtattgctaat | 76053 | - | cell_cycle, kinase_related | E2F-4/DP-1 complex | |
| 12498 | CDC7 | NM_003503.2 | 1344 | ttgcttagttggacgatatccatt | 76111 | - | see also 2360 line | | |
| 12499 | CDK10 | NM_003674.2 | 980 | ccggaagcagccctacaacaacc | 76128 | - | kinase_related, cell_cycle | | |
| 12500 | CDK2 | NM_001798.2 | 689 | tggagtccctgttcgtacttaca | 76134 | - | see also 1175 line | | |
| 12501 | CDK3 | NM_001258.1 | 612 | tgggcagcaagttctataccaca | 76143 | - | kinase_related, cell_cycle | cyclin-dependent protein kinase | |
| 12502 | CDK4 | NM_000075.2 | 246 | gagccagtggctgaaattggtgt | 76145 | - | - | | Well-differentiated liposarcoma, rhabdomyosarcoma, ependynoma, lipo sarcoma, chondrosarcoma, osteosarcoma, Ewing's sarcoma, melanoma, dysplastic nevus syndrome, nerve sheath tumors, astrocytic tumors |
| 12503 | CDK5 | NM_004935.2 | 681 | atgtcgatgaccagttgaagagg | 76169 | - | kinase_related, cell_cycle | protein tyrosine kinase | Alzheimer disease |
| 12504 | CDK6 | NM_001259.2 | 606 | tcggccttgcccgcatctatagt | 76179 | - | see also 819 line | | |
| 12505 | CDK7 | NM_001799.2 | 575 | gggagccccaatagagcttatac | 76204 | - | kinase_related, transcription_regulator, cell_cycle | cyclin-dependent protein kinase | |
| 12506 | CDK8 | NM_001260.1 | 979 | tggacccaataaagcgaattacc | 76251 | - | kinase_related, cell_cycle, transcription_regulator | protein serine/threonine kinase | |
| 12507 | CDK9 | NM_001261.2 | 855 | tggccaaacgtggacaactatga | 76274 | - | transcription_regulator, kinase_related, cell_cycle | cyclin-dependent protein kinase | |

Figure 46

| ID | Gene | Accession | Length | Sequence | Number | | Classification / notes | Protein description | Disease |
|---|---|---|---|---|---|---|---|---|---|
| 12508 | CDKL1 | NM_004196.3 | 486 | ctggaccgagtgactactataca | 76285 | - | kinase_related, cell_cycle | cyclin-dependent protein kinase | - |
| 12509 | CDKL2 | NM_003948.2 | 1665 | ggggactgagactataccaattc | 76348 | - | kinase_related, signal transduction | - | - |
| 12510 | CDKL3 | NM_016508.2 | 498 | tagagctccgaattagtattaa | 76367 | - | - | - | - |
| 12511 | CDKL5 | NM_003159.1 | 1613 | gagtcggcatagctatattgaca | 76433 | - | kinase_related | protein kinase CK2 | infantile spasm |
| 12512 | CENPE | NM_001813.1 | 5573 | atgaacatcaacttacgtta | 76574 | - | - | kinetochore motor | - |
| 12513 | CFTR | NM_000492.2 | 1174 | atggcggtcactcggccaatttcc | 76667 | - | see also 384 line | - | - |
| 12514 | CGI-04 | NM_015936.1 | 1265 | tggtccccgagggtatcgaatga | 76793 | - | - | - | - |
| 12515 | CGI-152 | NM_020410.1 | 441 | cagcgtcttacgtcatccatgc | 76812 | - | see also 7866 line | - | - |
| 12516 | CHEK1 | NM_001274.2 | 1148 | cagccctcatacattgataaat | 76866 | - | see also 840 line | - | - |
| 12517 | CHEK2 | NM_007194.2 | 1030 | ctgcccctggctgattatgg | 76882 | - | see also 4996 line | - | - |
| 12518 | CIT | NM_007174.1 | 4713 | tgggtcaccgccttagaatcagt | 77080 | - | see also 4975 line | - | - |
| 12519 | CLK1 | NM_004071.1 | 359 | tagtcgacgctacattgatgagt | 77110 | - | kinase_related, cell_cycle | protein serine/threonine kinase | - |
| 12520 | CLK2 | NM_001291.1 | 331 | gtgtatgaccgggatactgtgg | 77152 | - | kinase_related | - | - |
| 12521 | CLK3 | NM_001292.1 | 245 | gcgccgtgacagcgatacatacc | 77165 | - | kinase_related | - | - |
| 12522 | CLK4 | NM_020666.1 | 207 | ccggagatacgttgacggaataca | 77181 | - | see also 7912 line | - | - |
| 12523 | CLPX | NM_006660.3 | 976 | ctggcacaaacctagctaaatg | 77237 | - | see also 4706 line | - | - |
| 12524 | CNK | NM_004073.1 | 174 | cagcgcctacgctgtcaaagtca | 77272 | - | see also 2743 line | - | - |
| 12525 | CNTN6 | NM_014461.2 | 1602 | aggaacggagaccccttagacaaa | 77329 | - | see also 5690 line | - | - |
| 12526 | CPS1 | NM_001875.2 | 3834 | tggtccattcaacgtccaattc | 77468 | - | - | carbamoyl-phosphate synthase (ammonia) | hyperammonemia |
| 12527 | CRK7 | NM_016507.1 | 381 | caggcgttcccgggacttactaa | 77494 | - | see also 6883 line | - | - |
| 12528 | CSF1R | NM_005211.2 | 2377 | aggcgtcgactataagaacatcc | 77607 | - | see also 3661 line | - | - |
| 12529 | CSNK1A1 | NM_001892.3 | 796 | agggctaaaggctgcaacaaaga | 77640 | - | - | casein kinase I | - |
| 12530 | CSNK1D | NM_001893.3 | 417 | aggttgccatcaagcttgaatgt | 77643 | - | kinase_related, signal transduction | - | - |
| 12531 | CSNK1E | NM_001894.4 | 450 | cagccgcatcgagtatatccact | 77668 | - | see also 1209 line | - | - |
| 12532 | CSNK1G1 | NM_022048.1 | 523 | gtgaccgaacatttactttgaag | 77685 | - | see also 8248 line | - | - |
| 12533 | CSNK1G2 | NM_001319.5 | 228 | gagctccgcctaggaaaagaatct | 77703 | - | kinase_related, signal transduction | casein kinase I | - |
| 12534 | CSNK1G3 | NM_004384.1 | 1199 | gtggctcgtacaggttgtaagt | 77732 | - | see also 2944 line | - | - |
| 12535 | CSNK2A2 | NM_001896.2 | 670 | ttggggtctgcagaattctatc | 77756 | - | see also 1210 line | - | - |
| 12536 | CSPG4 | NM_001897.2 | 3198 | cagtatcgtggccgtagatgagc | 77765 | - | - | - | melanoma |
| 12537 | CSPG6 | NM_005445.2 | 894 | aggatatcgaaccgccaagttaga | 77807 | - | see also 3793 line | - | - |

Figure 46

| | | | | | | | | | |
|---|---|---|---|---|---|---|---|---|---|
| 12538 | DAPK1 | NM_004938.1 | 2040 | gtgtttcgtcgattatcaagaca | 77935 | - | see also 3434 line | | |
| 12539 | DAPK3 | NM_001348.1 | 116 | acgtggaggaccattatgagatg | 77976 | - | kinase_related、apoptosis | - | - |
| 12540 | DCAMKL1 | NM_004734.1 | 2224 | caggccccaagccgaatagcaca | 78012 | - | see also 3253 line | | |
| 12541 | DCK | NM_000788.1 | 635 | atgactggatgaataaccaattt | 78030 | - | kinase_related | deoxycytidine kinase | - |
| 12542 | DDR1 | NM_001954.3 | 2303 | acgaccccctctgcatgattact | 78052 | - | receptor_acitivity、kinase_related、signal_transduction | | - |
| 12543 | DDR2 | NM_006182.1 | 1758 | tcgacttacgatcgcatctttcc | 78098 | - | receptor_acitivity、kinase_related、signal_transduction | transmembrane receptor protein tyrosine kinase | - |
| 12544 | DDX32 | NM_018180.2 | 1584 | gtgtacaacccgagaataagagc | 78145 | - | - | - | - |
| 12545 | DGKE | NM_003647.1 | 1022 | caggcaacgatctatccaataca | 78197 | - | see also 2471 line | | |
| 12546 | DKFZp434C1418 | NM_173655.1 | 1213 | ctggtcaatagcaacttagtatg | 78282 | - | see also 10106 line | | |
| 12547 | DKFZP434G2226 | NM_031217.2 | 2641 | gtgttcgacaagataattcaagt | 78367 | - | see also 8994 line | | |
| 12548 | DKFZp727A071 | NM_152268.2 | 378 | gagaagctcgtgcgagtgataga | 78371 | - | - | - | - |
| 12549 | DKFZp761P1010 | NM_018423.1 | 1461 | ttggtggtacctgaactgtatgc | 78413 | - | kinase_related | - | - |
| 12550 | DLAT | NM_001931.2 | 1323 | ttgcacagcgattaatgcaatca | 78449 | - | see also 1233 line | | |
| 12551 | DMPK | NM_004409.1 | 1566 | ctgggtgtattcgcctatgaaat | 78476 | - | kinase_related | protein serine/threonine kinase | - |
| 12552 | DNAH11 | NM_003777.1 | 3753 | atgcggaagtcactcttataagg | 78558 | - | see also 2579 line | | |
| 12553 | DNAH3 | NM_017539.1 | 8962 | ctggggatccgctatactaatct | 79039 | - | see also 7017 line | | |
| 12554 | DNAH5 | NM_001369.1 | 13811 | cagttcgaacggacttgaactac | 79506 | - | see also 930 line | | |
| 12555 | DNAH7 | NM_018897.1 | 5741 | cagatcgaactcgaaatacgttt | 79695 | - | - | - | - |
| 12556 | DNAH8 | NM_001371.1 | 1821 | cagctctatcgccggataagtga | 79942 | - | see also 937 line | | |
| 12557 | DNAH9 | NM_001372.2 | 11644 | ccgaccggatgacctatgctttg | 80481 | - | - | - | - |
| 12558 | DNCH1 | NM_001376.2 | 2392 | gagagcgttcgtacctatgaacg | 80578 | - | see also 943 line | | |
| 12559 | DNCLI2 | NM_006141.2 | 1467 | cagactctatggtaacaaactct | 80838 | - | - | motor | - |
| 12560 | DPCK | NM_025233.3 | 1140 | ctgcttcggcctccatatgaaag | 80848 | - | see also 8886 line | | |
| 12561 | DQX1 | NM_133637.1 | 561 | ctggggcgtgctggtactagatg | 80858 | - | see also 9590 line | | |
| 12562 | DTYMK | NM_012145.1 | 462 | tggccatgagcgctatgagaacg | 80901 | - | kinase_related、cell_cycle | thymidylate kinase | - |

Figure 46

| 12563 | DYRK1B | NM_004714.1 | 599 | tggctggagcgctacgaaattga | 80977 | - | see also 3235 line | | |
|---|---|---|---|---|---|---|---|---|---|
| 12564 | DYRK2 | NM_003583.2 | 318 | gtgctcacgacacaaccaaatgg | 80989 | - | kinase_related | - | - |
| 12565 | DYRK3 | NM_003582.1 | 945 | tggtagtatgaacgttatccaca | 81041 | - | kinase_related | protein tyrosine kinase | - |
| 12566 | DYRK4 | NM_003845.1 | 1605 | tggtagacgctcccaagaagtca | 81094 | - | kinase_related | protein tyrosine kinase | - |
| 12567 | DYT1 | NM_000113.1 | 527 | gtgcgaggtccatcttcatattt | 81105 | - | see also 96 line | | |
| 12568 | EGFR | NM_005228.2 | 2328 | gtgcgttcggcacggtgtataag | 81167 | - | receptor_acitivity、cell_cycle、signal_transduction | Thyroid hormone receptor beta-2 | esophageal cancer、CNS Tumours、squamous cell carcinomacytic、astrocytic tumors、squamous cell cancer |
| 12569 | EHD4 | NM_139265.2 | 1055 | cagcaggctaccggaaatctaca | 81204 | - | - | nucleic acid binding | - |
| 12570 | EIF2AK3 | NM_004836.2 | 3486 | atggaacgacctgaagctataaa | 81286 | - | see also 3353 line | | |
| 12571 | EPB42 | NM_000119.1 | 1443 | cggtgggcgtcttctcatagatg | 81300 | - | - | structural constituent of cytoskeleton | hereditary hemolytic anemia、hereditary spherocytosis |
| 12572 | EPHA1 | NM_005232.2 | 1726 | gagattgtagccgtcatctttgg | 81326 | - | see also 3668 line | | |
| 12573 | EPHA2 | NM_004431.2 | 1535 | gtggaagtacgaggtcacttacc | 81348 | - | receptor_acitivity、kinase_related、signal_transduction | ephrin receptor | - |
| 12574 | EPHA3 | NM_005233.3 | 1502 | ctgcggtcagcatcacaactaat | 81397 | - | receptor_acitivity、signal_transduction、kinase_related | - | - |
| 12575 | EPHA4 | NM_004438.2 | 554 | gggatgtagggccattaagcaaa | 81459 | - | see also 2970 line | | |
| 12576 | EPHA5 | NM_004439.3 | 1581 | aagagaccagctacacgattatc | 81526 | - | see also 2972 line | | |
| 12577 | EPHA7 | NM_004440.2 | 2544 | tggcctgtcccgagttatagagg | 81625 | - | see also 2974 line | | |
| 12578 | EPHA8 | NM_020526.2 | 829 | gcgggacacacccaagatgtact | 81646 | - | see also 7887 line | | |
| 12579 | EPHB1 | NM_004441.2 | 1906 | caggaaacgggcttatagcaaag | 81680 | - | see also 2980 line | | |
| 12580 | EPHB2 | NM_004442.4 | 231 | ctggctacggaccaagtttatcc | 81694 | - | receptor_acitivity、kinase_related、signal_transduction | - | bladder cancer、neuroblastoma |
| 12581 | EPHB3 | NM_004443.3 | 2376 | gtgtgccgtggtcgactgaaaca | 81732 | - | receptor_acitivity、kinase_related、signal_transduction | ephrin receptor | - |
| 12582 | EPHB4 | NM_004444.3 | 1241 | cagccaatagccactctaacacc | 81742 | - | receptor_acitivity、kinase_related、signal_transduction | ephrin receptor | - |
| 12583 | EPHB6 | NM_004445.1 | 2744 | aagtcgatcctgcttatatcaag | 81774 | - | receptor_acitivity、kinase_related、signal_transduction | ephrin receptor | neuroblastoma |

Figure 46

| | | | | | | | | | |
|---|---|---|---|---|---|---|---|---|---|
| 12584 | EPRS | NM_004446.1 | 3665 | ctgacaactcgaactattggtgt | 81892 | - | - | proline-tRNA ligase | - |
| 12585 | ERBB2 | NM_004448.1 | 3386 | aaggggctggctccgatgtattt | 81940 | - | receptor_acitivity、kin ase_related、signal_tra nsduction | receptor signaling protein tyrosine kinase | bladder transitional cell carcinoma、cancer of the Fallopian tube、breast cancer、Li-Fraumeni syndrome、lung cancer、osteosarcoma、ovarian cancer、prostate cancer、squamous cell carcinomacytic |
| 12586 | ERBB3 | NM_001982.1 | 2938 | gggctacgattggctgaagtacc | 82003 | - | receptor_acitivity、sig nal_transduction、kina se_related | epidermal growth factor receptor | breast cancer |
| 12587 | ERBB4 | NM_005235.1 | 3846 | tgggcggatccggcctattgtgg | 82119 | - | receptor_acitivity、sig nal_transduction、kina se_related | epidermal growth factor receptor | - |
| 12588 | ERK8 | NM_139021.1 | 283 | cagagaacgacagggacatttac | 82123 | - | kinase_related | - | - |
| 12589 | FER | NM_005246.1 | 1598 | atgcacgatcagttacatctatg | 82158 | - | see also 3686 line | | |
| 12590 | FES | NM_002005.2 | 1771 | cagattggacgggggaactttgg | 82200 | - | kinase_related | protein tyrosine kinase | - |
| 12591 | FGFR3 | NM_000142.2 | 938 | cggacggcacaccctacgttacc | 82277 | - | receptor_acitivity、kin ase_related、signal_tra nsduction | | bladder transitional cell carcinoma、thanatophoric dysplasia、achondroplasia、acrocephalosyndactyly、bl adder cancer、craniofacial dysostosis、Muenke syndrome、hypochondroplasia |
| 12592 | FGFR4 | NM_002011.2 | 1497 | cggcctcgtgagtctagatctac | 82286 | - | receptor_acitivity、kin ase_related、signal_tra nsduction | - | - |
| 12593 | FGR | NM_005248.1 | 719 | gaggcgatcatgtgaagcattac | 82292 | - | kinase_related | protein tyrosine kinase | - |
| 12594 | FIGN | NM_018086.1 | 1663 | gaggaccaaatcgtagtaatttg | 82342 | - | see also 7237 line | | |
| 12595 | FIGNL1 | NM_022116.2 | 376 | aagaattacttcgcaattacatc | 82366 | - | see also 8278 line | | |
| 12596 | FLJ10074 | NM_017988.2 | 102 | ttgcggttcgtgtaaattcatta | 82432 | - | kinase_related | - | - |
| 12597 | FLJ10326 | NM_018060.2 | 2483 | gtgtgcaatgcgagactcatttc | 82558 | - | see also 7221 line | | |
| 12598 | FLJ10496 | NM_018114.3 | 1451 | ctgtacgcacagagatgatatgc | 82582 | - | - | - | - |
| 12599 | FLJ10514 | NM_018122.3 | 1188 | caggttgcccgatgttatcgaga | 82607 | - | see also 7266 line | | |
| 12600 | FLJ10709 | NM_018188.1 | 377 | cagtccaagctcaaagagtatga | 82642 | - | - | - | - |
| 12601 | FLJ10774 | NM_024662.1 | 1118 | tgggtactccaatatctttgtta | 82658 | - | see also 8690 line | | |
| 12602 | FLJ12118 | NM_024537.1 | 1147 | aggacgcacgtgcctacatgaag | 82712 | - | - | - | - |
| 12603 | FLJ12528 | NM_025150.3 | 1005 | atgcactagtggcgtttatcagg | 82735 | - | - | - | - |
| 12604 | FLJ12735 | NM_024857.3 | 5562 | gagatccaaccaacgatcttact | 82889 | - | - | - | - |
| 12605 | FLJ12969 | NM_022838.1 | 2068 | tggttgcaccctttaacaagaat | 82946 | - | - | - | - |

Figure 46

| No. | Symbol | Accession | Length | Sequence | ID | | Annotation | | Description | Cancer |
|---|---|---|---|---|---|---|---|---|---|---|
| 12606 | FLJ13490 | NM_024942.1 | 1361 | ctggatacctctaagacattat | 82964 | - | - | | | |
| 12607 | FLJ14600 | NM_032810.2 | 1101 | aggaccttgactcggctataatg | 82984 | - | see also 9292 line | | | |
| 12608 | FLJ14813 | NM_033844.1 | 837 | aggacactacgccttattctagc | 83014 | - | see also 9309 line | | | |
| 12609 | FLJ20245 | NM_017723.1 | 428 | ccggagacctcgccgcaagtatcg | 83063 | - | - | | | |
| 12610 | FLJ20559 | NM_017881.1 | 651 | gtggcccatgtatctaaagtaca | 83082 | - | kinase_related | | | |
| 12611 | FLJ20574 | NM_017886.1 | 175 | gcgacggaaggaacaatcaatt | 83089 | - | kinase_related | | | |
| 12612 | FLJ20604 | NM_017897.1 | 670 | caggtcagcattcgatataaact | 83138 | - | - | | | |
| 12613 | FLJ21019 | NM_024927.3 | 1845 | ccgactccggtgggagactatgt | 83166 | - | - | | | |
| 12614 | FLJ22002 | NM_024838.3 | 1645 | aagttcggctaactccataaaact | 83215 | - | see also 8780 line | | | |
| 12615 | FLJ22761 | NM_025130.2 | 1780 | gaggtcagtcggaatgtacaaca | 83264 | - | kinase_related | | | |
| 12616 | FLJ22865 | NM_025109.3 | 1527 | ctgatcatgcgcggttgtttga | 83284 | - | see also 8864 line | | | |
| 12617 | FLJ22955 | NM_024819.3 | 560 | gggataccgctacgtgattctgg | 83304 | - | kinase_related | | | |
| 12618 | FLJ23074 | NM_025052.1 | 456 | gttggtacatcggatatcaaagg | 83312 | - | kinase_related | | | |
| 12619 | FLJ23356 | NM_032237.2 | 679 | gaggatgaacaacactatgcttac | 83325 | - | see also 9128 line | | | |
| 12620 | FLJ23441 | NM_024678.3 | 1481 | tggaccttcgtcgatttggatct | 83374 | - | - | | | |
| 12621 | FLJ25005 | NM_152334.2 | 1043 | gtgtacaggtgcggtccattaat | 83390 | - | - | | | |
| 12622 | FLJ25006 | NM_144610.1 | 821 | ctgtggcactcttcagtacatgg | 83437 | - | kinase_related | | | |
| 12623 | FLJ31364 | NM_152463.1 | 1643 | cagaatttgctgcgcagacataca | 83458 | - | - | | | |
| 12624 | FLJ32685 | NM_152534.1 | 1146 | ctgtgattcttcgaaatctcaaa | 83481 | - | kinase_related | | | |
| 12625 | FLJ32704 | NM_152572.2 | 1135 | ctggccaccgaatctgaaatcc | 83489 | - | kinase_related, signal_transduction | | | |
| 12626 | FLJ34389 | NM_152649.1 | 1151 | ctggcagcattgcaatagtgagg | 83527 | - | - | | | |
| 12627 | FLJ37300 | NM_153209.1 | 1219 | cagcgactcaagcgcaagattga | 83550 | - | - | | | |
| 12628 | FLT1 | NM_002019.2 | 723 | ccggttacgtcacctaacatca | 83563 | - | receptor_acitivity, sig nal_transduction, kina se_related | | vascular endothelial growth factor receptor | bladder cancer |
| 12629 | FLT3 | NM_004119.1 | 2546 | ttggattggctgagatatcatg | 83730 | - | see also 2784 line | | | |
| 12630 | FLT4 | NM_002020.1 | 1685 | ccgacgcttcaccatcgaatcc | 83757 | - | receptor_acitivity, sig nal_transduction, kina se_related | | | |
| 12631 | FPGS | NM_004957.2 | 413 | cgggagcggatccgcatcaatgg | 83770 | - | - | | folylpolyglutamate synthase | |
| 12632 | FRK | NM_002031.2 | 1871 | aggaacgacctacatttgagaca | 83822 | - | kinase_related, cell_c ycle | | non-membrane spanning protein tyrosine kinase | |
| 12633 | FTHFSDC1 | NM_015440.3 | 1466 | ttgtccgtgctagaaaggttaaa | 83842 | - | see also 6417 line | | | |
| 12634 | FUK | NM_145059.1 | 595 | ccggcctacgtcagaatcatgg | 83864 | - | kinase_related | | | non-small-cell lung cancer |

Figure 46

| 12635 | FYN | NM_002037.3 | 776 | gtgtgaactcttcgtctcatacg | 83874 | - | see also 1316 line | | |
| 12636 | GABARAPL2 | NM_007285.6 | 191 | gcgaagattcgagcgaaatatcc | 83910 | - | - | protein transporter | - |
| 12637 | GAK | NM_005255.1 | 1192 | cagtccgtcgctaattatgcaaa | 83944 | - | kinase_related、cell_cycle | protein serine/threonine kinase | - |
| 12638 | GALK1 | NM_000154.1 | 375 | tcggtgggccaactatgtcaagg | 83973 | - | kinase_related | galactokinase | - |
| 12639 | GALK2 | NM_002044.1 | 834 | caggctaaactagggattagtct | 84009 | - | see also 1319 line | | |
| 12640 | GARS | NM_002047.1 | 684 | ctggcgttacagcccaaagatga | 84022 | - | see also 1320 line | | |
| 12641 | GCK | NM_000162.2 | 948 | gaggcacgaagacatcgataagg | 84065 | - | kinase_related | - | diabetes mellitus、diabetes、maturity-onset diabetes of the young |
| 12642 | GK2 | NM_033214.1 | 874 | ctgtgtaatacgggtcgtaaatg | 84083 | - | kinase_related | glycerol kinase | - |
| 12643 | GMPS | NM_003875.1 | 856 | gtggagtagactcaacagtttgt | 84119 | - | - | carbon-nitrogen ligase、with glutamine as amido-N-donor | - |
| 12644 | GNE | NM_005476.3 | 1375 | acgaacctccgagttgcaatagt | 84185 | - | kinase_related | - | - |
| 12645 | GPRK2L | NM_005307.1 | 1405 | ctgatcctcatgccgtttactgt | 84251 | - | gpcr、kinase_related、signal_transduction | G-protein-coupled receptor phosphorylating protein kinase | - |
| 12646 | GPRK5 | NM_005308.1 | 358 | aaggaccatagacagagattact | 84262 | - | kinase_related、gpcr、signal_transduction | protein kinase C binding | - |
| 12647 | GPRK6 | NM_002082.1 | 1548 | caggacttctaccagaagtttgc | 84290 | - | see also 1347 line | | |
| 12648 | GPRK7 | NM_139209.1 | 1720 | aagtctggcgtgtgtttgttatt | 84311 | - | gpcr、kinase_related、signal_transduction | - | - |
| 12649 | GSG2 | NM_031965.1 | 2227 | ggggtgaatatcacccttatagt | 84354 | - | kinase_related、cell_cycle | - | - |
| 12650 | GSK3A | NM_019884.1 | 1480 | ccgtcctcacaagctttaactga | 84371 | - | kinase_related | protein kinase CK2 | diabetes mellitus、Alzheimer disease、diabetes |
| 12651 | GSK3B | NM_002093.2 | 558 | tagtccgattgcgttatttcttc | 84379 | - | see also 1357 line | | |
| 12652 | GSS | NM_000178.2 | 780 | gtgatccgacgaacatttgaaga | 84405 | - | - | glutathione synthase | 5-oxoprolinuria、hereditary hemolytic anemia |
| 12653 | GUCY2C | NM_004963.1 | 673 | ctggaggctagcgtttcctattt | 84436 | - | receptor_acitivity、kinase_related、signal_transduction | receptor guanylate cyclase | - |
| 12654 | GUCY2D | NM_000180.1 | 1417 | gtgctggttcgatccaaacaaca | 84504 | - | receptor_acitivity、kinase_related、signal_transduction | receptor guanylate cyclase | amaurosis congenita of Leber |

Figure 46

| | | | | | | | | | |
|---|---|---|---|---|---|---|---|---|---|
| 12655 | GUCY2F | NM_001522.1 | 3220 | aagtgccggtccgaattcgaatt | 84593 | - | receptor_acitivity、kinase_related、signal_transduction | receptor guanylate cyclase | - |
| 12656 | GUK1 | NM_000858.3 | 390 | gaggtgatgcagcgtgacatagc | 84602 | - | kinase_related | membrane-associated guanylate kinase | - |
| 12657 | HAK | NM_052947.1 | 2327 | aggagttatctgtcacagattca | 84637 | - | kinase_related | - | - |
| 12658 | HARS | NM_002109.3 | 577 | tggccgataccgggaattctacc | 84693 | - | - | histidine-tRNA ligase | - |
| 12659 | HARSL | NM_012208.2 | 1373 | gggttgagcgaatcttctacatt | 84723 | - | - | histidine-tRNA ligase | - |
| 12660 | HEAB | NM_006831.1 | 1241 | atgtcgaagagggtttctctatc | 84751 | - | - | GTP binding | - |
| 12661 | HIPK2 | NM_022740.1 | 431 | tcggcggaccacacaacctaatg | 84768 | - | see also 8444 line | | |
| 12662 | HIPK3 | NM_005734.2 | 805 | cagcaccgtaaccatacttgttt | 84838 | - | see also 3977 line | | |
| 12663 | HK1 | NM_000188.1 | 1311 | tggtgtcgacggatctctttaca | 84936 | - | see also 157 line | | |
| 12664 | HK2 | NM_000189.4 | 3136 | gagcggctgcgctctactattgg | 84972 | - | kinase_related、cell_cycle | hexokinase | diabetes mellitus、diabetes |
| 12665 | HK3 | NM_002115.1 | 1420 | cggaatgcgatgtctccttaatc | 84995 | - | kinase_related | hexokinase | - |
| 12666 | HRI | NM_014413.2 | 391 | gtgacgagtttagctcattgaga | 85012 | - | kinase_related | protein serine/threonine kinase | - |
| 12667 | HSA250839 | NM_018401.1 | 724 | gggctggaggccgtacgaaatcc | 85065 | - | - | - | - |
| 12668 | HSP70-4 | NM_016299.1 | 856 | ctgaagtagcgaaacattctttg | 85101 | - | see also 6790 line | | |
| 12669 | HSPA12B | NM_052970.3 | 2136 | gcgcgtccatcgactttctttcc | 85125 | - | see also 9473 line | | |
| 12670 | HSPA1L | NM_005527.2 | 1225 | atggacgtgatctcaacaagagc | 85146 | - | - | heat shock protein | - |
| 12671 | HSPA2 | NM_021979.2 | 1393 | cagcgtactggtgcaggtatacg | 85162 | - | - | heat shock protein | - |
| 12672 | HSPA4 | NM_002154.3 | 578 | aggtataaaggtgacatatatgg | 85169 | - | see also 1384 line | | |
| 12673 | HSPA5 | NM_005347.2 | 1292 | gtggctcgactcgaattccaaag | 85234 | - | see also 3732 line | | |
| 12674 | HSPA6 | NM_002155.2 | 1594 | gagcacaggtaaggctaacaaga | 85247 | - | - | heat shock protein | schizophrenia |
| 12675 | HSPA8 | NM_006597.3 | 1310 | ctgtcctcatcaagcgtaatacc | 85267 | - | - | - | - |
| 12676 | HSPA9B | NM_004134.4 | 235 | aggcgggattatgcatcagaagc | 85274 | - | see also 2791 line | | |
| 12677 | HSPCB | NM_007355.2 | 1816 | gtgacaatctccaatagacttgt | 85316 | - | - | translation regulator | breast cancer |
| 12678 | HSPD1 | NM_002156.3 | 190 | ccgatgctgtggccgttacaatg | 85323 | - | - | heat shock protein | spastic paraplegia |
| 12679 | HSPH1 | NM_006644.1 | 2566 | atgtgaacccgttgtaacacaac | 85380 | - | see also 4696 line | | |
| 12680 | HUNK | NM_014586.1 | 2058 | tcgcgacgaccacgtagaagtgc | 85408 | - | kinase_related、signal_transduction | protein serine/threonine kinase | - |
| 12681 | HYOU1 | NM_006389.2 | 929 | cagatccggggagtaggatttga | 85432 | - | - | chaperone | - |

Figure 46

| 12682 | IARS | NM_002161.2 | 3048 | aagtcatcaatcgcatacagaaa | 85555 | - | see also 1386 line | | |
|---|---|---|---|---|---|---|---|---|---|
| 12683 | ICK | NM_014920.2 | 743 | ctgcatgggaccagaacttgtga | 85593 | - | see also 6094 line | | |
| 12684 | IDH3G | NM_004135.2 | 1125 | cggcaagagtatcgccaataaga | 85641 | - | see also 2792 line | | |
| 12685 | IGF1R | NM_000875.2 | 3506 | ttggtatgacgcgagatatctat | 85700 | - | see also 566 line | | |
| 12686 | IKBKE | NM_014002.1 | 1624 | tggatgggcaggagctaatgttt | 85707 | - | - | NF-kappaB-inducing kinase | - |
| 12687 | ILK | NM_004517.1 | 324 | gggggcacggatcaatgtaatga | 85710 | - | see also 3065 line | | |
| 12688 | IMMP2L | NM_032549.1 | 659 | ccgtggtgacattgtatcattgg | 85739 | - | - | - | - |
| 12689 | INSL3 | NM_005543.2 | 356 | cagtggctgtacccaacaagacc | 85740 | - | - | peptide hormone | - |
| 12690 | INSR | NM_000208.1 | 2762 | gtgagttatcggcgatatggtga | 85783 | - | receptor_acitivity、kinase_related、signal_transduction | Smad3/Sp-1 heterodimer | diabetes mellitus、leprechaunism、diabetes、obesity |
| 12691 | INSRR | NM_014215.1 | 1956 | tggacacagtacgcagtgtttgt | 85809 | - | see also 5539 line | | |
| 12692 | IPT | NM_017646.3 | 905 | atgcacactggagactagtaacc | 85851 | - | see also 7075 line | | |
| 12693 | IRAK1 | NM_001569.2 | 153 | gggtcatgtgccgcttctacaaa | 85863 | - | receptor_acitivity、kinase_related、transcription_regulator、signal_transduction | protein binding | - |
| 12694 | IRAK2 | NM_001570.2 | 1283 | aggacttactcctcagtgatatt | 85892 | - | kinase_related、signal_transduction | NF-kappaB-inducing kinase | - |
| 12695 | IRAK3 | NM_007199.1 | 342 | gggacatcgtcgagctattcatt | 85903 | - | see also 5001 line | | |
| 12696 | IRAK4 | NM_016123.1 | 842 | atgcctaatggttcattgctaga | 85970 | - | - | - | - |
| 12697 | ITGB1BP3 | NM_014446.1 | 188 | aagccccaagaccaaatagcagt | 85991 | - | kinase_related、signal_transduction | - | - |
| 12698 | ITK | NM_005546.3 | 1907 | ctgcgtcaactggctgaaattgc | 86037 | - | kinase_related | non-membrane spanning protein tyrosine kinase | - |
| 12699 | JAK2 | NM_004972.2 | 881 | cagagcctatcggcatggaatat | 86126 | - | see also 3454 line | | |
| 12700 | JAK3 | NM_000215.1 | 2969 | gagcgaggcacacgtcaagatcg | 86246 | - | kinase_related | protein tyrosine kinase | - |
| 12701 | JIK | NM_016281.2 | 999 | atgactccccaacgttacagtct | 86273 | - | kinase_related | - | - |
| 12702 | KATNA1 | NM_007044.2 | 590 | ttgatagtaccggatatgataaa | 86331 | - | see also 4933 line | | |
| 12703 | KCNJ1 | NM_000220.2 | 346 | tggtatgcagtagcgtacattca | 86361 | - | see also 165 line | | |
| 12704 | KCNJ10 | NM_002241.2 | 749 | gcgggctgagaccattcgtttca | 86389 | - | channel | ATP-activated inward rectifier potassium channel | - |

EP 1 752 536 A1

Figure 46

| 12705 | KDR | NM_002253.1 | 4072 | cagacggacagtggtatggttct | 86462 | - | receptor_acitivity、signal_transduction、kinase_related | vascular endothelial growth factor receptor | hemangioma |
|---|---|---|---|---|---|---|---|---|---|
| 12706 | KIAA0195 | NM_014738.1 | 1032 | cagtcggtgatgctacactatgc | 86469 | - | see also 5927 line | | |
| 12707 | KIAA0703 | NM_014861.1 | 1152 | ggggaagccgagccatgtagtaa | 86483 | - | - | plasma membrane cation-transporting ATPase | - |
| 12708 | KIAA0999 | NM_025164.3 | 3522 | cagcgacgatgcttatgtacagc | 86564 | - | - | - | - |
| 12709 | KIAA1164 | NM_019092.1 | 675 | tagctgcgagttcaatagtgagg | 86570 | - | - | - | - |
| 12710 | KIAA1804 | NM_032435.1 | 1917 | ccgactccgagccatacagttga | 86600 | - | kinase_related | - | - |
| 12711 | KIAA1811 | NM_032430.1 | 1054 | gagcctcctgaggggaatgatcg | 86629 | - | kinase_related | - | - |
| 12712 | KIF11 | NM_004523.2 | 245 | tagcgcccattcaatagtagaat | 86640 | - | - | microtubule motor | - |
| 12713 | KIF12 | NM_138424.1 | 1546 | ccgagtcctggcagagatgttga | 86737 | - | - | - | - |
| 12714 | KIF13A | NM_022113.3 | 1654 | gaggaaacgtcgagattggttga | 86782 | - | - | - | - |
| 12715 | KIF13B | NM_015254.1 | 1119 | aggaccctaatgcccgaattatc | 86896 | - | kinase_related、signal_transduction | - | - |
| 12716 | KIF14 | NM_014875.1 | 2520 | atgctaaccaagcccgtttaata | 87042 | - | - | - | - |
| 12717 | KIF1B | NM_015074.1 | 5186 | tcgtccgtcggccttatgtcttc | 87232 | - | see also 6222 line | | |
| 12718 | KIF2 | NM_004520.1 | 1898 | atgatgtcgattcatatgctaca | 87286 | - | see also 3072 line | | |
| 12719 | KIF20A | NM_005733.1 | 2652 | acggcgttcccctttactcaaat | 87335 | - | see also 3976 line | | |
| 12720 | KIF21A | NM_017641.2 | 2415 | aaggttcgcctaatgaaacaaat | 87407 | - | - | - | - |
| 12721 | KIF25 | NM_005355.2 | 763 | cggcagcgcctcgaaactgatgg | 87502 | - | - | - | - |
| 12722 | KIF27 | NM_017576.1 | 832 | tggtattcccctcggcatattgt | 87512 | - | - | - | - |
| 12723 | KIF3A | NM_007054.3 | 1034 | atgtgccttatcgtaactctaaa | 87588 | - | - | motor | - |
| 12724 | KIF3B | NM_004798.2 | 800 | cagctcgcgttctcatgcaattt | 87630 | - | - | plus-end-directed kinesin ATPase | - |
| 12725 | KIF3C | NM_002254.4 | 2600 | atgaagaagcggccaacatctgc | 87665 | - | see also 1449 line | | |
| 12726 | KIF5A | NM_004984.1 | 1033 | ctgccggacgactatgttcatct | 87685 | - | see also 3474 line | | |
| 12727 | KIF5B | NM_004521.1 | 3068 | cagattgctaaacctattcgtcc | 87800 | - | see also 3075 line | | |
| 12728 | KIF5C | NM_004522.1 | 684 | atggggatcatcccacgaattgc | 87801 | - | see also 3081 line | | |
| 12729 | KIF9 | NM_022342.2 | 735 | aggcccattcccggacccttatca | 87850 | - | see also 8335 line | | |
| 12730 | KIFC2 | NM_145754.2 | 2024 | gcgggaggcccagaccataaacc | 87880 | - | - | - | - |
| 12731 | KIFC3 | NM_005550.2 | 1882 | cagcgctgcggagatctacaatg | 87887 | - | - | - | - |
| 12732 | KIT | NM_000222.1 | 2634 | tggaatgccggtcgattctaagt | 87978 | - | see also 166 line | | |
| 12733 | KPI2 | NM_014916.2 | 1261 | ctgcagatcagactaagtatagt | 88009 | - | kinase_related、phosphatase | - | - |
| 12734 | KSR2 | NM_173598.2 | 1474 | gagccaacgtcggagaatgaaga | 88098 | - | kinase_related | - | - |

Figure 46

| | | | | | | | | |
|---|---|---|---|---|---|---|---|---|
| 12735 | LAK | NM_025144.2 | 653 | ctggtgcagtcggtctgtataca | 88109 | - | - | |
| 12736 | LARS | NM_020117.8 | 3573 | tgcggatacaataatctatctgg | 88277 | - | - | |
| 12737 | LARS2 | NM_015340.2 | 1499 | gttggagacgtgacaagtgataaa | 88308 | - | see also 6381 line | |
| 12738 | LATS1 | NM_004690.2 | 3276 | ttgttggactcccaattatat | 88440 | - | kinase_related | |
| 12739 | LATS2 | NM_014572.1 | 2552 | gtgggtggtcaaactctactact | 88477 | - | kinase_related | |
| 12740 | LCK | NM_005356.2 | 106 | gtgtgtgagaactgccattatcc | 88490 | - | kinase_related, cell_cycle, signal_transduction | protein tyrosine kinase |
| 12741 | LIMK1 | NM_002314.2 | 982 | ctgagctccggcttatactcc | 88516 | - | see also 1489 line | |
| 12742 | LIMK2 | NM_005569.2 | 1461 | tggcctatttgcactctatgtgc | 88537 | - | kinase_related | |
| 12743 | LOC115704 | NM_145245.2 | 1552 | tcgctgcccgacgagaacaatgt | 88555 | - | see also 9783 line | |
| 12744 | LOC116143 | NM_138458.1 | 1115 | cggtgagtactgatcgttaca | 88582 | - | see also 9623 line | |
| 12745 | LOC200383 | NM_145299.1 | 757 | cagcctcgattgaattacttcg | 88633 | - | - | |
| 12746 | LOC340371 | NM_178564.2 | 1200 | tggaatctacccactgatgaact | 88664 | - | - | |
| 12747 | LOC84643 | NM_032559.2 | 1087 | caggaacggcccaagtggttct | 88671 | - | - | |
| 12748 | LOC91807 | NM_182493.1 | 2059 | caggacatcaaattaagatcatt | 88696 | - | kinase_related | |
| 12749 | LOC91862 | NM_052858.1 | 1074 | caggcgctgtaccaaagcaaagg | 88715 | - | - | |
| 12750 | LOC92935 | NM_138395.1 | 1413 | aggtagcagaccactatgataac | 88737 | - | see also 9615 line | |
| 12751 | LONP | NM_031490.2 | 2525 | aggcaacgtacgacgaggatttaa | 88805 | - | see also 9040 line | |
| 12752 | LTK | NM_002344.1 | 1557 | gtgtatgagggactggtaattgg | 88819 | - | receptor_acitivity, kinase_related, signal_transduction | transmembrane receptor protein tyrosine kinase |
| 12753 | LYK5 | NM_153335.3 | 450 | acgtgactgtacggaggattaac | 88831 | - | see also 9988 line | |
| 12754 | LYN | NM_002350.1 | 1755 | gagaccaacgtttgactactac | 88888 | - | kinase_related | receptor signaling protein tyrosine kinase |
| 12755 | MAK | NM_005906.3 | 797 | atggctgaactctatatgttaag | 88906 | - | kinase_related | |
| 12756 | MAP2K1 | NM_002755.2 | 743 | tgggcacaaggtcctacacgtcg | 88945 | - | see also 1798 line | |
| 12757 | MAP2K2 | NM_030662.2 | 670 | gtgacgggagatcagcagcatttgc | 88959 | - | kinase_related | protein serine/threonine kinase |
| 12758 | MAP2K3 | NM_002756.2 | 595 | gcgcacggtcgactgtttctaca | 88968 | - | see also 1799 line | |
| 12759 | MAP2K5 | NM_002757.2 | 1307 | gtgcgattcattcatatatcattg | 88995 | - | see also 1801 line | |

Figure 46

EP 1 752 536 A1

| | | | | | | | | | |
|---|---|---|---|---|---|---|---|---|---|
| 12760 | MAP2K6 | NM_002758.2 | 587 | cggatccgagccacagtaaatag | 89025 | - | kinase_related、 cell_cycle、 signal_transduction | - | - |
| 12761 | MAP2K7 | NM_145185.2 | 496 | acgtcattgccgttaagcaaatg | 89053 | - | - | - | - |
| 12762 | MAP3K10 | NM_002446.2 | 1227 | tggcgtggctatgaataagctga | 89062 | - | kinase_related、 signal_transduction、 apoptosis | protein tyrosine kinase | - |
| 12763 | MAP3K11 | NM_002419.2 | 2277 | atgactcatccccttaggatct | 89069 | - | kinase_related | - | - |
| 12764 | MAP3K12 | NM_006301.2 | 1472 | caggcgagagcaagctttagagc | 89084 | - | see also 4392 line | | |
| 12765 | MAP3K13 | NM_004721.3 | 1659 | aggcatgcgctggatattcgtga | 89128 | - | kinase_related、 signal_transduction | - | - |
| 12766 | MAP3K14 | NM_003954.1 | 1520 | aaggtgcggctggaagtatttcg | 89168 | - | see also 2701 line | | |
| 12767 | MAP3K3 | NM_002401.2 | 1619 | acgcctgcagacgatctgtatgt | 89276 | - | kinase_related | protein tyrosine kinase | - |
| 12768 | MAP3K4 | NM_005922.1 | 1809 | gggcacgtatagcattggtaaag | 89338 | - | see also 4139 line | | |
| 12769 | MAP3K5 | NM_005923.3 | 1262 | cagcgagtagataatatcgaagt | 89452 | - | see also 4141 line | | |
| 12770 | MAP3K6 | NM_004672.2 | 805 | ctgcatgtgtggccgtatctaca | 89535 | - | see also 3207 line | | |
| 12771 | MAP3K7 | NM_003188.2 | 744 | atgcaacccaaagcgctaattca | 89568 | - | kinase_related、 signal_transduction | - | - |
| 12772 | MAP3K8 | NM_005204.2 | 2073 | gggaccaccaacgcttgaatatg | 89629 | - | kinase_related | protein serine/threonine kinase | - |
| 12773 | MAP4K1 | NM_007181.2 | 75 | cggcacgtatggggaagtcttta | 89631 | - | kinase_related | - | - |
| 12774 | MAP4K2 | NM_004579.2 | 1685 | ctggctctactgcgtgaacaacg | 89663 | - | kinase_related | - | - |
| 12775 | MAP4K3 | NM_003618.2 | 2352 | ttgagacggtcaatccaaattct | 89732 | - | kinase_related | - | - |
| 12776 | MAP4K5 | NM_006575.3 | 2380 | tggtctgtgtagctattagcaaa | 89793 | - | see also 4608 line | | |
| 12777 | MAPK1 | NM_002745.2 | 383 | aagttcgagtagctatcaagaaa | 89815 | - | see also 1787 line | | |
| 12778 | MAPK10 | NM_002753.2 | 1179 | cagcggactccgagcacaataaa | 89858 | - | kinase_related、 signal_transduction | - | - |
| 12779 | MAPK11 | NM_002751.4 | 451 | gagcgacgagcacgttcaattcc | 89867 | - | kinase_related、 signal_transduction | - | - |
| 12780 | MAPK12 | NM_002969.2 | 1326 | atggaagcgtgttacttacaaag | 89873 | - | kinase_related、 cell_cycle、 signal_transduction | SAP kinase 3 | - |
| 12781 | MAPK13 | NM_002754.3 | 1062 | cagccgtttgatgattccttaga | 89881 | - | kinase_related、 cell_cycle | MAP kinase | - |
| 12782 | MAPK14 | NM_001315.1 | 688 | tgggggcagatctgaacaacatt | 89892 | - | kinase_related、 signal_transduction | | |

Figure 46

| 12783 | MAPK3 | NM_002746.1 | 992 | tggaccggatgttaacctttaac | 89928 | - | - | - | - |
|---|---|---|---|---|---|---|---|---|---|
| 12784 | MAPK4 | NM_002747.2 | 1007 | aaggatcgttgatcagcattact | 89935 | - | see also 1788 line | | |
| 12785 | MAPK6 | NM_002748.2 | 1854 | ttggcctgtacataacaactttg | 89971 | - | see also 1789 line | | |
| 12786 | MAPK7 | NM_002749.2 | 2277 | ttgtgacccagcagctatctaag | 90026 | - | see also 1790 line | | |
| 12787 | MAPK8 | NM_002750.2 | 571 | atgtagtgactcgctactacaga | 90048 | - | see also 1791 line | | |
| 12788 | MAPK9 | NM_002752.3 | 568 | ccggacagcgtgcactaacttca | 90082 | - | see also 1795 line | | |
| 12789 | MAPKAPK2 | NM_004759.3 | 944 | ttgaccactccttgttatacacc | 90108 | - | kinase_related | - | - |
| 12790 | MAPKAPK3 | NM_004635.3 | 715 | ctgctatactccctattatgtgg | 90126 | - | see also 3149 line | | |
| 12791 | MAPKAPK5 | NM_003668.2 | 1574 | tggaatggtcgtggattcacaga | 90164 | - | see also 2496 line | | |
| 12792 | MARK1 | NM_018650.2 | 2392 | gagcgacgcagcgttgcttataa | 90221 | - | see also 7536 line | | |
| 12793 | MARK2 | NM_004954.2 | 1302 | atggtgtccatgggttatacacg | 90244 | - | kinase_related | - | - |
| 12794 | MARK4 | NM_031417.2 | 954 | gaggagttgaagccatacacaga | 90325 | - | see also 9016 line | | |
| 12795 | MAST205 | NM_015112.1 | 2972 | gtgattggctccctgagatatt | 90371 | - | see also 6250 line | | |
| 12796 | MAT1A | NM_000429.1 | 597 | ctgcggcctgactctaagactca | 90388 | - | - | methionine adenosyltransferase | hypermethioninemia |
| 12797 | MAT2A | NM_005911.3 | 547 | aggagtgtatgcctttaaccatt | 90405 | - | - | methionine adenosyltransferase | - |
| 12798 | MATK | NM_002378.2 | 1117 | tggccgtgaagaatatcaagtgt | 90425 | - | see also 1531 line | | |
| 12799 | MCCC1 | NM_020166.2 | 1817 | tagccatagctgtaacgtataac | 90479 | - | see also 7778 line | | |
| 12800 | MDN1 | NM_014611.1 | 6432 | gagcaggttgatcttatacgacc | 90650 | - | see also 5779 line | | |
| 12801 | MELK | NM_014791.1 | 502 | ttgtcttccgtcagatagtatct | 90857 | - | kinase_related | - | - |
| 12802 | MERTK | NM_006343.1 | 1579 | ctgcacacggttgggtagattat | 90954 | - | see also 4437 line | | |
| 12803 | MGC13186 | NM_032324.1 | 220 | gggagaagaataggattcgatgt | 91130 | - | - | - | - |
| 12804 | MGC14560 | NM_016301.2 | 628 | tagatccagacatgtattcttta | 91155 | - | - | - | - |
| 12805 | MGC16169 | NM_033115.2 | 1406 | ctgccacgctccctttaatcatc | 91187 | - | kinase_related | - | - |
| 12806 | MGC22688 | NM_145001.1 | 365 | aagtgcgtggagcgcaatgaagt | 91233 | - | kinase_related | - | - |
| 12807 | MGC2599 | NM_032116.2 | 471 | aggcgtcccaatcgagaagtaag | 91242 | - | see also 9075 line | | |
| 12808 | MGC2744 | NM_025267.2 | 1062 | ctgggcccattcgggttgttaac | 91286 | - | - | - | - |
| 12809 | MGC33182 | NM_145203.2 | 1171 | cagaattcgccatgtacttgaac | 91300 | - | kinase_related、signal transduction | | |

Figure 46

| 12810 | MGC42105 | NM_153361.2 | 1226 | gaggctactatcccgagaaatct | 91303 | - | kinase_related | - | - |
|---|---|---|---|---|---|---|---|---|---|
| 12811 | MGC45428 | NM_152619.1 | 1149 | gagcttgaccgttgcataagtcc | 91357 | - | see also 9905 line | | |
| 12812 | MGC4796 | NM_032017.1 | 1028 | gaggacacatcggataaccatca | 91387 | - | kinase_related | - | - |
| 12813 | MGC5347 | NM_024063.1 | 1392 | aagcgtcattggattaatggata | 91404 | - | - | - | - |
| 12814 | MGC8407 | NM_024046.2 | 1580 | caggctccaccctctagtaaagg | 91448 | - | see also 8578 line | | |
| 12815 | MIDORI | NM_020778.1 | 5448 | tggctgtatcagtggacaaatgg | 91482 | - | see also 7962 line | | |
| 12816 | MKI67 | NM_002417.2 | 9612 | gagcccggaaacccatacctaga | 91582 | - | cell_cycle | ATP binding | breast cancer、 prostate cancer |
| 12817 | MKKS | NM_018848.2 | 1345 | tggtgcgtagtatattaacaagt | 91602 | - | see also 7603 line | | |
| 12818 | MKNK1 | NM_003684.3 | 335 | gggagcctatgccaaagttcaag | 91651 | - | kinase_related | - | - |
| 12819 | MKNK2 | NM_017572.2 | 985 | gtgcggctcggcggagtacatgg | 91664 | - | kinase_related | - | - |
| 12820 | MLH1 | NM_000249.2 | 686 | atgcctcaaccgtggacaatatt | 91686 | - | see also 182 line | | |
| 12821 | MORC | NM_014429.1 | 1715 | tagagagtcggtcataaagtatc | 91766 | - | see also 5675 line | | |
| 12822 | MOS | NM_005372.1 | 207 | agggtttggctcggtgtacaagg | 91794 | - | kinase_related、 cell_cycle | protein kinase CK2 | - |
| 12823 | MOV10 | NM_020963.1 | 167 | gagcggctgcggaccatttataa | 91808 | - | see also 8052 line | | |
| 12824 | MPHOSPH1 | NM_016195.1 | 4621 | tggatatcaagcccaaacgtatt | 91962 | - | cell_cycle | - | - |
| 12825 | MST1R | NM_002447.1 | 139 | tcgcgactttgacgtgaagtacg | 91990 | - | receptor_acitivity、 kinase_related、 signal_transduction | macrophage colony stimulating factor receptor | |
| 12826 | MST4 | NM_016542.2 | 1192 | gagctttaccaccgtacgaaaga | 92065 | - | see also 6908 line | | |
| 12827 | MT | NM_014507.1 | 386 | ctggccgctgtcgagaaactaca | 92074 | - | - | - | - |
| 12828 | MTHFD1 | NM_005956.2 | 1012 | ttgatatatcacgatcttgtaaa | 92112 | - | see also 4162 line | | |
| 12829 | MUSK | NM_005592.1 | 47 | atgagagagctcgtcaacattcc | 92149 | - | see also 3882 line | | |
| 12830 | MVD | NM_002461.1 | 76 | ctggggcaagcgcgatgaagagc | 92188 | - | kinase_related | diphosphomevalonate decarboxylase | |
| 12831 | MVK | NM_000431.1 | 452 | ctgccgagcctggatatcgtagt | 92199 | - | kinase_related | mevalonate kinase | familial periodic fever、 mevalonic aciduria、 hyperimmunoglobulinaemia D |
| 12832 | MYH1 | NM_005963.2 | 811 | ctggagaagtctagagttacttt | 92204 | - | - | muscle motor | - |
| 12833 | MYH11 | NM_002474.1 | 3743 | aaggcgaacctagacaagaataa | 92232 | - | - | - | leukemia |
| 12834 | MYH13 | NM_003802.1 | 3846 | gtgccggacggtagaagatcaat | 92280 | - | - | muscle motor | - |
| 12835 | MYH2 | NM_017534.1 | 4575 | agggaaacgtatccatgaactgg | 92302 | - | - | muscle motor | - |
| 12836 | MYH3 | NM_002470.1 | 3791 | tggagagtgtgtcgaaatctaag | 92322 | - | - | myosin ATPase | - |
| 12837 | MYH4 | NM_017533.1 | 1334 | aggtcggcaatgagttcgtaacc | 92343 | - | - | myosin ATPase | - |
| 12838 | MYH6 | NM_002471.1 | 3318 | aagaaggagtttgacattaatca | 92371 | - | - | Thyroid hormone receptor beta-2 | - |

Figure 46

| | | | | | | | | structural constituent of muscle | |
|---|---|---|---|---|---|---|---|---|---|
| 12839 | MYH7 | NM_000257.1 | 1961 | tggggctgatcgcctattgaga | 92382 | - | | structural constituent of muscle | cardiomyopathy |
| 12840 | MYH8 | NM_002472.1 | 249 | gagggaaagtaaccgtaaagact | 92397 | - | | structural constituent of muscle | |
| 12841 | MYH9 | NM_002473.2 | 2212 | caggccgtgcgtgtcatgataaa | 92439 | - | | motor | cochleosaccular degeneration |
| 12842 | MYLK | NM_005965.2 | 3194 | aggacgggaactgctcttaatt | 92486 | - | see also 4168 line | motor | |
| 12843 | MYLK2 | NM_033118.2 | 1367 | atgaccaaatctccgataagaca | 92509 | - | kinase_related | | |
| 12844 | MYO10 | NM_012334.1 | 6230 | gggcacccctggcgaatacgtat | 92625 | - | see also 5295 line | | |
| 12845 | MYO15A | NM_016239.2 | 7172 | tggccacgactaegtgttagacc | 92678 | - | see also 6746 line | | |
| 12846 | MYO18B | NM_032608.4 | 7911 | acgacgatgttgcgagcataatg | 92761 | - | | | non-small-cell lung cancer |
| 12847 | MYO1A | NM_005379.2 | 892 | aagcttgagcgggatacaactgg | 92772 | - | | myosin ATPase | |
| 12848 | MYO1C | NM_033375.1 | 1952 | tcgccgcaaatacgaagctttcc | 92845 | - | | motor | |
| 12849 | MYO1E | NM_004998.1 | 1098 | gggtcatacaaggttgatgaca | 92874 | - | see also 3493 line | | |
| 12850 | MYO1F | NM_012335.2 | 590 | ctgtgcgcaacaacaattccagc | 92932 | - | | motor | |
| 12851 | MYO3A | NM_017433.3 | 2482 | ctggccctcttagatatgtttc | 93014 | - | see also 7004 line | | |
| 12852 | MYO3B | NM_138995.1 | 262 | aagtctacaaggtaactaacaag | 93074 | - | | | |
| 12853 | MYO5A | NM_000259.1 | 2371 | cagccgctacgtgtcctaatga | 93191 | - | see also 201 line | | |
| 12854 | MYO5C | NM_018728.1 | 2653 | ccgacgattcgtgcttaatattc | 93325 | - | | | |
| 12855 | MYO6 | NM_004999.1 | 348 | aagccacactgtccattaatatc | 93378 | - | see also 3494 line | motor | |
| 12856 | MYO7A | NM_000260.1 | 1450 | acgtgcgcgagcgcttcgtaaag | 93493 | - | see also 205 line | | |
| 12857 | MYO9A | NM_006901.1 | 4957 | atgtactgggtcctatcatta | 93679 | - | see also 4834 line | | |
| 12858 | MYO9B | NM_004145.2 | 4444 | aagaaccgaaatgtcaagattgg | 93781 | - | see also 2793 line | | |
| 12859 | NADSYN1 | NM_018161.3 | 2091 | aggtttgatctgcgaccatttct | 93812 | - | | | |
| 12860 | NAGK | NM_017567.1 | 601 | cagatcggctaggggatactcact | 93823 | - | see also 7035 line | | |
| 12861 | NALP1 | NM_014922.2 | 3886 | gccgtgaccgttgagattgaatt | 93872 | - | | | |
| 12862 | NALP11 | NM_145007.1 | 2157 | tcgtaatcggagcctgacatacc | 93951 | - | | | |
| 12863 | NALP12 | NM_033297.1 | 1488 | cagttggcaacggcgttgattcc | 93991 | - | signal_transduction | | |
| 12864 | NALP2 | NM_017852.1 | 1845 | gcgatggcgacataagttgtaagg | 94032 | - | apoptosis | ATP binding | |
| 12865 | NALP5 | NM_153447.2 | 2501 | tggccaacgtaacctaagatcc | 94099 | - | | | |
| 12866 | NALP6 | NM_138329.1 | 525 | gcgctggacacgacacactttca | 94125 | - | | | |
| 12867 | NALP7 | NM_139176.1 | 1975 | aaggtgcacttacctaaccattc | 94147 | - | | | |
| 12868 | NAV1 | NM_020443.2 | 4859 | ttgccgtcgaggtgtcaataaca | 94203 | - | see also 7877 line | | |
| 12869 | NAV2 | NM_145117.2 | 2623 | gagatgaggtggccgtagcatact | 94243 | - | | | |
| 12870 | NAV3 | NM_014903.2 | 2340 | cagtggtaccagtcgattcatcc | 94363 | - | | | |
| 12871 | NDUFA10 | NM_004544.2 | 147 | cagccgttgaggaggaattcatag | 94474 | - | | | NADH dehydrogenase (ubiquinone) |

529

Figure 46

| | | | | | | | | | | |
|---|---|---|---|---|---|---|---|---|---|---|
| 12872 | NEK11 | NM_024800.2 | 750 | aggatacttcatcgagacttaaa | 94503 | - | - | - | - | |
| 12873 | NEK2 | NM_002497.1 | 218 | gagtgatgcaagatattagttt | 94548 | - | kinase_related, cell_cycle | protein kinase CK2 | - | |
| 12874 | NEK3 | NM_002498.1 | 969 | cagtccactgccgtccattact | 94594 | - | kinase_related, cell_cycle | - | | |
| 12875 | NEK4 | NM_003157.1 | 497 | caggtggtagagtcgttgtaca | 94616 | - | kinase_related | protein kinase CK2 | - | |
| 12876 | NEK6 | NM_014397.3 | 959 | gagcactactccgagaagttacg | 94686 | - | kinase_related | - | | |
| 12877 | NEK7 | NM_133494.1 | 393 | ctggcgacctatccagaatgatc | 94698 | - | see also 9589 line | - | | |
| 12878 | NEK8 | NM_178170.2 | 491 | tgggtacccatgctatatctcc | 94723 | - | - | - | | |
| 12879 | NEK9 | NM_033116.3 | 288 | acgtcgtgatgccttgaatgaga | 94734 | - | see also 9363 line | - | | |
| 12880 | NLK | NM_016231.1 | 1322 | aggcgctaaggcacatatactca | 94825 | - | see also 6733 line | - | | |
| 12881 | NME1 | NM_000269.2 | 229 | atgcaagcttcgaagatcttct | 94846 | - | kinase_related, cell_cycle | nucleoside-diphosphate kinase | - | neuroblastoma, melanoma, testicular cancer |
| 12882 | NME3 | NM_002513.2 | 663 | ctgggcactggctgatgagtag | 94852 | - | kinase_related, apoptosis | nucleoside-diphosphate kinase | - | |
| 12883 | NME4 | NM_005009.2 | 356 | atgtctggaaggtacaatgt | 94853 | - | kinase_related | nucleoside-diphosphate kinase | - | |
| 12884 | NME5 | NM_003551.2 | 314 | ttgtcgccatgatattagctaga | 94859 | - | see also 2364 line | - | | |
| 12885 | NME7 | NM_013330.3 | 465 | ttgactatgggatcaatataca | 94893 | - | kinase_related | - | | |
| 12886 | NOLC1 | NM_004741.1 | 1864 | aagcttcagacccctaacacatt | 94927 | - | cell_cycle | GTP binding | - | |
| 12887 | NPR2 | NM_000907.1 | 1328 | tggacgaccatcctgtgataaa | 94950 | - | see also 588 line | - | | |
| 12888 | NRBP | NM_013392.1 | 1617 | ctgaccagagccggttgacttct | 95011 | - | see also 5425 line | - | | |
| 12889 | NSF | NM_006178.1 | 2125 | aaggaacgcaccacaattgcaca | 95060 | - | see also 4271 line | - | | |
| 12890 | NTRK1 | NM_002529.2 | 1323 | atgtggacggagaacaagtttg | 95069 | - | receptor_acitivity, kinase_related, signal_transduction | neurotrophin TRKA receptor | - | thyroid cancer, papillary Thyroid Carcinomas, medullary thyroid carcinoma, neuroblastoma, neuropathy |
| 12891 | NTRK2 | NM_006180.2 | 1420 | cagcgcttcagtggttctataac | 95101 | - | see also 4274 line | - | | |
| 12892 | NTRK3 | NM_002530.1 | 1526 | tcgacggtccaaattggaatga | 95146 | - | kinase_related, receptor_acitivity, signal_transduction | neurotrophin TRKC receptor | - | neuroblastoma, infantile fibrosarcoma |
| 12893 | NUBP1 | NM_002484.1 | 264 | ttgcttcttagacatcgatata | 95159 | - | see also 1590 line | - | | |
| 12894 | NVL | NM_002533.1 | 1238 | gggaaggttcgaccgagaaatat | 95190 | - | see also 1631 line | - | | |
| 12895 | OSR1 | NM_005109.1 | 934 | atggaacagtgccgtggttatga | 95242 | - | kinase_related | - | | |
| 12896 | P2RX1 | NM_002558.2 | 576 | cgggtgggtgtttctctatgaga | 95274 | - | see also 1648 line | - | | |
| 12897 | P2RX2 | NM_012226.3 | 252 | tgggcgctgrtgrtgccctattacc | 95287 | - | receptor_acitivity, channel | - | | |

Figure 46

| | | | | | | | | | |
|---|---|---|---|---|---|---|---|---|---|
| 12898 | P2RX3 | NM_002559.2 | 146 | gggatcatcaaccgagtagttca | 95297 | - | receptor_acitivity、cha nnel、signal_transduct ion | ATP-gated cation channel | - |
| 12899 | P2RX4 | NM_002560.2 | 1149 | gggacgttgagcacaacgtatct | 95329 | - | receptor_acitivity、cha nnel、signal_transduct ion | - | - |
| 12900 | P2RX5 | NM_002561.2 | 761 | caggggcacctgtgagatctttg | 95341 | - | receptor_acitivity、cha nnel | - | - |
| 12901 | P2RX7 | NM_002562.4 | 1224 | ctgtgtggtcaacgaatactact | 95386 | - | receptor_acitivity、cha nnel、signal_transduct ion | - | - |
| 12902 | P2RXL1 | NM_005446.2 | 696 | ctgccgctatgaaccacaattca | 95402 | - | channel、receptor_acit ivity、signal_transduct ion | transmembrane receptor | - |
| 12903 | PAK1 | NM_002576.2 | 1182 | gggcgatcctaagaagaaatata | 95464 | - | see also 1657 line | | |
| 12904 | PAK2 | NM_002577.2 | 688 | ccgcgaccggatcatacgaaatc | 95477 | - | kinase_related、signal _transduction | protein tyrosine kinase | - |
| 12905 | PAK3 | NM_002578.1 | 1411 | ttgtatctgatagccactaatgg | 95522 | - | see also 1658 line | | |
| 12906 | PAK4 | NM_005884.2 | 1704 | ctggtcgctggggataatggtga | 95525 | - | kinase_related、signal _transduction | protein serine/threonine kinase | - |
| 12907 | PAK6 | NM_020168.3 | 580 | gtggacccttcgcgaatcacacg | 95526 | - | kinase_related | - | - |
| 12908 | PAK7 | NM_020341.2 | 1010 | ctgccgattatcactcacatttg | 95547 | - | see also 7836 line | | |
| 12909 | PANK1 | NM_138316.2 | 910 | gtgcgcgttgaatgagaacatag | 95593 | - | see also 9596 line | | |
| 12910 | PANK3 | NM_024594.2 | 765 | tggacaagccgagtgctattatt | 95642 | - | see also 8657 line | | |
| 12911 | PANK4 | NM_018216.1 | 125 | tcgacataggcgggtcgttaacc | 95661 | - | see also 7323 line | | |
| 12912 | PAPSS1 | NM_005443.3 | 292 | ctggatggtgacaatattcgtca | 95689 | - | kinase_related | sulfate adenylyltransferase (ATP) | - |
| 12913 | PAPSS2 | NM_004670.2 | 350 | atgtccgtcatggccttaacaga | 95730 | - | see also 3192 line | | |
| 12914 | PASK | NM_015148.2 | 526 | tggttgctaacgacaaagcttgc | 95784 | - | kinase_related、signal _transduction | - | - |
| 12915 | PC | NM_000920.2 | 2769 | tggcaactcggacgtgtatgaaa | 95836 | - | see also 597 line | | |
| 12916 | PCCA | NM_000282.1 | 731 | ttggcgatgatagactactaata | 95864 | - | see also 246 line | | |
| 12917 | PCOLCE2 | NM_013363.2 | 974 | gggtttattggtcactacatatt | 95931 | - | see also 5409 line | | |
| 12918 | PCTK1 | NM_006201.2 | 843 | ccgtcgtgtcagcctatctgaga | 95946 | - | see also 4297 line | | |
| 12919 | PCTK2 | NM_002595.2 | 867 | ttgtctcagaaatcgtatacata | 95974 | - | see also 1676 line | | |
| 12920 | PCTK3 | NM_002596.1 | 1250 | gagtactccacccccattgatat | 96009 | - | see also 1679 line | | |

EP 1 752 536 A1

Figure 46

| | | | | | | | | | |
|---|---|---|---|---|---|---|---|---|---|
| 12921 | PDGFRA | NM_006206.2 | 2539 | aagcacacggagctatgttattt | 96041 | - | receptor_acitivity、sig nal_transduction、kina se_related | platelet-derived growth factor、 alpha-receptor | piebaldism、familial/sporadic gastrointestinal stromal tumors |
| 12922 | PDGFRB | NM_002609.2 | 3404 | gggtgacaacgactatatcatcc | 96101 | - | see also 1687 line | | |
| 12923 | PDK1 | NM_002610.3 | 1186 | acgtctttacgcacaatacttcc | 96139 | - | see also 1688 line | | |
| 12924 | PDK2 | NM_002611.3 | 1161 | gggaccgatgctgtcatctatct | 96147 | - | see also 1689 line | | |
| 12925 | PDK3 | NM_005391.1 | 183 | ctgtgcggctggctaacacaatg | 96155 | - | see also 3760 line | | |
| 12926 | PDK4 | NM_002612.2 | 1108 | ttgaccgcctctttagttataca | 96222 | - | kinase_related、signal _transduction | pyruvate dehydrogenase (lipoamide) kinase | diabetes mellitus、diabetes |
| 12927 | PDPK1 | NM_002613.1 | 808 | tgggaacagcgcagtacgtttct | 96240 | - | kinase_related、signal _transduction | protein tyrosine kinase | - |
| 12928 | PDZK3 | NM_015022.2 | 1365 | gggaggaagttggccgaatatgg | 96270 | - | - | - | - |
| 12929 | PEX1 | NM_000466.1 | 495 | ttgggttgatcaacaaacgtaca | 96384 | - | see also 374 line | | |
| 12930 | PEX6 | NM_000287.2 | 2911 | aagcgcatccagcgcaagtttgc | 96506 | - | - | peroxisome-assembly ATPase | peroxisome biogenesis disorder、Zellweger syndrome |
| 12931 | PFAS | NM_012393.1 | 3794 | gcgcacggggaaggttacgtagc | 96540 | - | - | - | - |
| 12932 | PFKFB1 | NM_002625.1 | 330 | aggcagtgagctacaagaactat | 96559 | - | kinase_related、phosp hatase | transferase | - |
| 12933 | PFKFB2 | NM_006212.1 | 1149 | aagtatctgtatcgatatcctgg | 96603 | - | kinase_related、phosp hatase | fructose-2、6-bisphosphate 2-phosphatase | - |
| 12934 | PFKFB3 | NM_004566.1 | 574 | gtgtgcgacgaccctacagttgt | 96612 | - | kinase_related、phosp hatase | fructose-2、6-bisphosphate 2-phosphatase | - |
| 12935 | PFKFB4 | NM_004567.2 | 426 | cggagagcgaccatctttaattt | 96637 | - | see also 3106 line | | |
| 12936 | PFTK1 | NM_012395.2 | 882 | cagtgacacgggggagttaaagc | 96663 | - | see also 5309 line | | |
| 12937 | PHKG1 | NM_006213.2 | 1071 | tggcttcagtgcggatctactac | 96681 | - | kinase_related | protein tyrosine kinase | - |
| 12938 | PHKG2 | NM_000294.1 | 674 | tagcgccagagatccttaaatgc | 96687 | - | kinase_related | protein tyrosine kinase | cirrhosis due to liver phosphorylase kinase deficiency、glycogenosis |
| 12939 | PIK3R4 | NM_014602.1 | 1888 | aagaggttcctcgtaatgatatc | 96744 | - | kinase_related | - | - |
| 12940 | PIM1 | NM_002648.2 | 656 | cgggtttctccggcgtcattagg | 96826 | - | kinase_related | Smad3/Sp-1 heterodimer | - |
| 12941 | PINK1 | NM_032409.1 | 1027 | ccggacgctgttcctcgttatga | 96838 | - | see also 9184 line | | |
| 12942 | PKE | NM_173575.2 | 1386 | cagtccgagaatgactatcttca | 96859 | - | kinase_related | - | - |
| 12943 | PKMYT1 | NM_004203.3 | 1141 | atgtcaagcctgccaacatcttc | 96865 | - | kinase_related、cell_c ycle | | - |

EP 1 752 536 A1

Figure 46

| 12944 | PLK | NM_005030.3 | 552 | ctgcaccgaaaccgagttattca | 96870 | - | kinase_related、 cell_cycle | protein serine/threonine kinase | - |
|---|---|---|---|---|---|---|---|---|---|
| 12945 | PP13 | NM_013268.2 | 313 | gtgcatctacgtacattacaatg | 96887 | - | - | | - |
| 12946 | PRKAA2 | NM_006252.1 | 528 | tagccgatttcggattatctaat | 96901 | - | kinase_related、 signal_transduction | protein serine/threonine kinase | - |
| 12947 | PRKACA | NM_002730.2 | 1056 | atggggtcaacgatatcaagaac | 96955 | - | kinase_related | protein tyrosine kinase | - |
| 12948 | PRKACB | NM_002731.1 | 715 | gggcattaggagtgctaatctat | 96980 | - | kinase_related、 signal_transduction | protein tyrosine kinase | - |
| 12949 | PRKACG | NM_002732.2 | 860 | acggggttggcgacatcaagaac | 97005 | - | kinase_related | protein tyrosine kinase | - |
| 12950 | PRKCA | NM_002737.1 | 429 | ctgcgatatgaacgttcacaagc | 97011 | - | see also 1774 line | | |
| 12951 | PRKCB1 | NM_002738.4 | 1899 | ctgaaggcgaacgtgatatcaaa | 97063 | - | see also 1775 line | | |
| 12952 | PRKCD | NM_006254.2 | 1065 | ccgcttcaaggttcacaactaca | 97080 | - | kinase_related | protein tyrosine kinase | - |
| 12953 | PRKCE | NM_005400.1 | 1531 | gagcctcgttcacggttctatgc | 97117 | - | kinase_related、 apoptosis | protein kinase C | - |
| 12954 | PRKCG | NM_002739.2 | 408 | tcgacgatgccacgaatttgtga | 97128 | - | kinase_related | protein tyrosine kinase | retinitis pigmentosa |
| 12955 | PRKCH | NM_006255.2 | 1432 | ccgacttggtatcgacaactttg | 97177 | - | see also 4337 line | | |
| 12956 | PRKCI | NM_002740.3 | 776 | gtgccataaactcgtcacaattg | 97225 | - | see also 1776 line | | |
| 12957 | PRKCL1 | NM_002741.1 | 1593 | gcgtgctaggcagatgaacatcg | 97253 | - | kinase_related、 signal_transduction | protein serine/threonine kinase | - |
| 12958 | PRKCL2 | NM_006256.1 | 1137 | aagcggaagtagtcgaaatcttc | 97313 | - | see also 4341 line | | |
| 12959 | PRKCN | NM_005813.2 | 2792 | aagttctccggagcaaaggttac | 97481 | - | see also 4056 line | | |
| 12960 | PRKCQ | NM_006257.1 | 78 | ttgtcagggcgaggctgttaacc | 97496 | - | see also 4343 line | | |
| 12961 | PRKD2 | NM_016457.2 | 291 | tggcttctacggcctttacgaca | 97545 | - | kinase_related | protein serine/threonine kinase | - |
| 12962 | PRKG1 | NM_006258.1 | 671 | tgggccattgatcgacaatgttt | 97578 | - | see also 4344 line | | |
| 12963 | PRKG2 | NM_006259.1 | 1261 | atgcgaagcggtccatgtctaac | 97641 | - | see also 4348 line | | |
| 12964 | PRKWNK1 | NM_018979.1 | 1823 | ctgctagcaccggcatacctact | 97697 | - | see also 7630 line | | |
| 12965 | PRKWNK2 | NM_006648.3 | 3664 | ggggacgcacccgatgaaattgc | 97827 | - | kinase_related | - | - |

Figure 46

| | | | | | | | | | |
|---|---|---|---|---|---|---|---|---|---|
| 12966 | PRKWNK3 | NM_020922.1 | 2603 | ttggtcggtggcgattctgtatc | 97911 | - | kinase_related | - | - |
| 12967 | PRKWNK4 | NM_032387.2 | 2223 | cagacggattcgggagattatcc | 98014 | - | see also 9179 line | | |
| 12968 | PRO2000 | NM_014109.2 | 1224 | gagtcgtaatagggctatcaata | 98054 | - | see also 5494 line | | |
| 12969 | PRPF4B | NM_003913.3 | 2692 | ttgcaccttatacgaactctata | 98232 | - | see also 2669 line | | |
| 12970 | PRSS15 | NM_004793.2 | 521 | atgtcggcgtctttctaaagaga | 98244 | - | - | serine-type endopeptidase | - |
| 12971 | PSK | NM_016151.1 | 893 | ccgggatgtccggaatagtgagg | 332998 | - | kinase_related、apopto sis | - | - |
| 12972 | PSKH1 | NM_006742.1 | 395 | aagtttgacccacgtgttacagc | 98268 | - | see also 4764 line | | |
| 12973 | PSKH2 | NM_033126.1 | 339 | gcgggttagccatcgttacattg | 98285 | - | kinase_related | - | - |
| 12974 | PSMC1 | NM_002802.2 | 1033 | atggccacaaaccgaatagaaac | 98311 | - | - | proteasome ATPase | - |
| 12975 | PSMC2 | NM_002803.2 | 773 | gcgtgcttcattcgagttattgg | 98336 | - | see also 1819 line | | |
| 12976 | PSMC3 | NM_002804.3 | 1197 | ccgccttgaccgcaagatagagt | 98373 | - | see also 1820 line | | |
| 12977 | PSMC4 | NM_006503.2 | 267 | aggaggtgaagcgaatccaaagc | 98375 | - | - | - | - |
| 12978 | PSMC5 | NM_002805.4 | 660 | acggactgtacctttattcgtgt | 98410 | - | see also 1821 line | | |
| 12979 | PTK2 | NM_005607.3 | 740 | tagaaatacggcgatcatactgg | 98459 | - | see also 3893 line | | |
| 12980 | PTK2B | NM_004103.3 | 2403 | tcgtctccccatcaaatggatgt | 98549 | - | see also 2772 line | | |
| 12981 | PTK6 | NM_005975.2 | 260 | gagtcggaaccgtggttctttgg | 98559 | - | kinase_related | non-membrane spanning protein tyrosine kinase | - |
| 12982 | PTK7 | NM_002821.3 | 3012 | cagtgcccagagacaagtgaagg | 98587 | - | receptor_acitivity、kin ase_related、signal_tra nsduction | - | - |
| 12983 | PTOV1 | NM_017432.2 | 1178 | gtgtgagatccgcgtgcttatgc | 98591 | - | - | - | - |
| 12984 | PXK | NM_017771.2 | 617 | aggaacggctagtgttaagctgg | 98611 | - | see also 7151 line | | |
| 12985 | QARS | NM_005051.1 | 1252 | tagcctatcgagtcaagtataca | 98662 | - | - | glutamine-tRNA ligase | - |
| 12986 | RAD50 | NM_005732.2 | 1932 | caggaccctgcgtaaacttgacc | 98742 | - | see also 3973 line | | |
| 12987 | RAF1 | NM_002880.1 | 455 | aagcacgcttagattggaatact | 98834 | - | see also 1921 line | | |
| 12988 | RAGE | NM_014226.1 | 754 | tgggttctacacgtacaagatgg | 98892 | - | see also 5541 line | | |
| 12989 | RARSL | NM_020320.2 | 1556 | aggttcgacgaggtgctttataa | 98950 | - | - | - | - |
| 12990 | RET | NM_000323.2 | 1237 | cggcgaccgtacatgactatag | 98967 | - | see also 273 line | | |
| 12991 | RHOK | NM_002929.1 | 1343 | ccgtgaagtaccctgataagttc | 99009 | - | kinase_related、recept or_acitivity、gpcr、si gnal_transduction | G-protein-coupled receptor phosphorylating protein kinase | Oguchi disease |

Figure 46

| | | | | | | | | | |
|---|---|---|---|---|---|---|---|---|---|
| 12992 | RICH1 | NM_018054.3 | 176 | ctggacacggtgcggtcaatatg | 99014 | - | see also 7218 line | | |
| 12993 | RIOK1 | NM_031480.2 | 1254 | ttgcgccaacgtcaatgatttct | 99089 | - | kinase_related | - | - |
| 12994 | RIPK1 | NM_003804.2 | 1623 | tggagcctacaattatatggaga | 99147 | - | kinase_related、apoptosis、signal_transduction | protein tyrosine kinase | - |
| 12995 | RIPK2 | NM_003821.4 | 1036 | gagcacgtatgatctctctaata | 99180 | - | see also 2603 line | | |
| 12996 | ROCK1 | NM_005406.1 | 2479 | cagcttacgaaacagtatagagg | 99264 | - | see also 3772 line | | |
| 12997 | ROCK2 | NM_004850.2 | 2165 | ctgcgaacagagtctgatactgc | 99322 | - | see also 3364 line | | |
| 12998 | ROR1 | NM_005012.1 | 1109 | gtgacttgtgtcgcgatgaatgt | 99415 | - | see also 3506 line | | |
| 12999 | ROR2 | NM_004560.2 | 757 | tggcaaccggaccatttatgtgg | 99471 | - | receptor_acitivity、kinase_related、signal_transduction | transmembrane receptor protein tyrosine kinase | - |
| 13000 | ROS1 | NM_002944.2 | 3615 | atgctgacgttgtaaagtctaca | 99594 | - | see also 1986 line | | |
| 13001 | RP2 | NM_006915.1 | 1102 | atgtcaacttattgtaaacgaga | 99728 | - | see also 4852 line | | |
| 13002 | RPS6KA1 | NM_002953.2 | 1398 | tagtgacggctacgtggtaaagg | 99741 | - | kinase_related、signal_transduction | protein tyrosine kinase | - |
| 13003 | RPS6KA2 | NM_021135.3 | 1931 | ctgctacacggccaatttcgtgg | 99761 | - | kinase_related | protein tyrosine kinase | - |
| 13004 | RPS6KA3 | NM_004586.1 | 2077 | caggatgcaccacatctagtaaa | 99814 | - | see also 3120 line | | |
| 13005 | RPS6KA4 | NM_003942.1 | 1850 | gggcgtcattctgtacatgatgc | 99829 | - | kinase_related、transcription_regulator | - | - |
| 13006 | RPS6KA5 | NM_004755.2 | 309 | ctgcggactgctaatttgacagg | 99832 | - | see also 3273 line | | |
| 13007 | RPS6KA6 | NM_014496.1 | 1491 | ttgatgcgctatggacaacatcc | 99918 | - | see also 5720 line | | |
| 13008 | RPS6KB1 | NM_003161.1 | 737 | ttggactatgcaaagaatctatt | 99952 | - | see also 2134 line | | |
| 13009 | RPS6KB2 | NM_003952.1 | 1112 | aggacgtgagccagtttgatacc | 99968 | - | kinase_related、signal_transduction、cell_cycle | protein serine/threonine kinase | - |
| 13010 | RPS6KC1 | NM_012424.2 | 2417 | tgggcccaaatcctatagtataa | 100039 | - | - | - | - |
| 13011 | RPS6KL1 | NM_031464.2 | 818 | caggcgcactcccgacattctgg | 100077 | - | kinase_related | - | - |
| 13012 | RUVBL1 | NM_003707.1 | 215 | gaggcatgtggcgtcatagtaga | 100083 | - | transcription_regulator | structural molecule | - |
| 13013 | RUVBL2 | NM_006666.1 | 747 | acgagatcgacgtcatcaactct | 100119 | - | transcription_regulator | - | - |
| 13014 | RYK | NM_002958.1 | 1646 | gagttctctagcgctagtgatgt | 100173 | - | receptor_acitivity、kinase_related、signal_transduction | transmembrane receptor protein tyrosine kinase | - |
| 13015 | SARS2 | NM_017827.2 | 832 | tggccaccggtcctattacctgc | 100178 | - | see also 7157 line | | |
| 13016 | SCN8A | NM_014191.1 | 1301 | ttgtatcaattgactttacgagc | 100194 | - | see also 5532 line | | |

Figure 46

| 13017 | SCYL1 | NM_020680.2 | 323 | ctgtgaccccgttgggaatatac | 100243 | - | kinase_related | - | - |
|---|---|---|---|---|---|---|---|---|---|
| 13018 | SEPHS2 | NM_012248.2 | 819 | acggtggtcaacccttggattat | 100261 | - | see also 5222 line | | |
| 13019 | SGK | NM_005627.2 | 319 | tcgtccaatcctcatgctaaacc | 100295 | - | see also 3903 line | | |
| 13020 | SGK2 | NM_016276.3 | 562 | aagtctgatggggcgttctatgc | 100317 | - | kinase_related | - | - |
| 13021 | SGKL | NM_013257.3 | 481 | aagacgagcaggactaaacgaat | 100336 | - | see also 5376 line | | |
| 13022 | SIK2 | NM_015191.1 | 514 | ttgtcatggtcggaagattgtgc | 100385 | - | - | - | - |
| 13023 | SKD3 | NM_030813.3 | 1872 | gtgctggtcgacggctacaatgt | 100452 | - | see also 8957 line | | |
| 13024 | SMC1L2 | NM_148674.1 | 1702 | gagacattcctcgctctagatta | 100546 | - | see also 9820 line | | |
| 13025 | SMC2L1 | NM_006444.1 | 902 | ttgagtcgtttatatattgctta | 100643 | - | see also 4505 line | | |
| 13026 | SMC5L1 | NM_015110.1 | 1408 | gagtgtggacgatcatattgtac | 100771 | - | see also 6249 line | | |
| 13027 | SMC6L1 | NM_024624.2 | 2752 | ctgcccagagcgtatagaagtag | 100899 | - | see also 8671 line | | |
| 13028 | SNARK | NM_030952.1 | 1545 | acgcaggcgacgtgtttgtgagt | 100931 | - | kinase_related | - | - |
| 13029 | SNF1LK | NM_173354.1 | 338 | ctgaaccatccacacatcataaa | 100937 | - | see also 10064 line | | |
| 13030 | SNK | NM_006622.1 | 1258 | gaggatcgtcccagtttggatga | 100963 | - | see also 4683 line | | |
| 13031 | SNRK | NM_017719.3 | 1286 | ctgcaagcccgagcaatatcaag | 101023 | - | kinase_related | - | - |
| 13032 | SPATA5 | NM_145207.1 | 205 | ctgacggtgaccaacttattaga | 101043 | - | see also 9780 line | | |
| 13033 | SPG4 | NM_014946.3 | 2003 | cagcgtcagccctcaaactttag | 101183 | - | see also 6136 line | | |
| 13034 | SPG7 | NM_003119.2 | 240 | agggatcaacggattgttgttga | 101187 | - | - | metalloendopeptidase | - |
| 13035 | SPHK1 | NM_021972.2 | 1363 | atgaatgcccctacttggtatat | 101213 | - | kinase_related | diacylglycerol kinase | - |
| 13036 | SPHK2 | NM_020126.3 | 950 | tggcctggcagtggtgtaagaac | 101220 | - | kinase_related、apoptosis | sphinganine kinase | - |
| 13037 | SRC | NM_005417.3 | 770 | aggcgagcggctccagattgtca | 101224 | - | kinase_related | protein tyrosine kinase | colorectal cancer、colorectal cancers |
| 13038 | SRMS | NM_080823.2 | 388 | cagaccaaccctggtactttagc | 101228 | - | kinase_related | - | - |
| 13039 | SRPK1 | NM_003137.2 | 1030 | acgcttatggaacgtgatacaga | 101253 | - | see also 2117 line | | |
| 13040 | SRPK2 | NM_182691.1 | 1305 | tagaccctacgtggatagaatca | 101294 | - | see also 10272 line | | |
| 13041 | SSTK | NM_032037.1 | 143 | gagcgaactcggttataagctgg | 101321 | - | see also 9064 line | | |
| 13042 | STCH | NM_006948.3 | 707 | ttgggcggaggaactctagatgt | 101345 | - | - | adenosinetriphosphatase | - |
| 13043 | STK10 | NM_005990.1 | 101 | gagaaagtcccgcgaatatgagc | 101365 | - | see also 4184 line | | |
| 13044 | STK16 | NM_003691.1 | 427 | aggtacgctgtggaatgagatag | 101401 | - | see also 2525 line | | |
| 13045 | STK17A | NM_004760.1 | 985 | ttgtctgagtcggctgttgattt | 101432 | - | - | protein serine/threonine kinase | - |
| 13046 | STK17B | NM_004226.2 | 530 | ttgtccccgtgttattaatcttc | 101453 | - | see also 2823 line | | |
| 13047 | STK18 | NM_014264.2 | 1129 | ttggtcagccactcccaaataaa | 101498 | - | see also 5571 line | | |
| 13048 | STK19 | NM_004197.1 | 748 | tggatgcccatggaattatcttc | 101560 | - | see also 2810 line | | |

Figure 46

| | | | | | | | | |
|---|---|---|---|---|---|---|---|---|
| 13049 | STK22C | NM_052841.2 | 859 | ccggccttcaattgaagaagtta | - | kinase_related | - | |
| 13050 | STK23 | NM_014370.1 | 232 | aagatcgggacgtgttcaatgg | - | kinase_related | protein kinase CK2 | |
| 13051 | STK24 | NM_003576.2 | 492 | gaggctccgcactagatctatta | - | see also 2373 line | - | |
| 13052 | STK25 | NM_006374.3 | 313 | aggtcacaagggatcgataac | - | kinase_related, signal transduction | protein tyrosine kinase | - |
| 13053 | STK29 | NM_003957.1 | 2143 | cggtcaagttccaggttgatatc | - | kinase_related | - | |
| 13054 | STK3 | NM_006281.1 | 243 | agggtcttatggaagtgtattta | - | see also 4373 line | | |
| 13055 | STK33 | NM_030906.1 | 1840 | gagcatagtcgtctaatgtaca | - | kinase_related | - | |
| 13056 | STK36 | NM_015690.2 | 576 | ccgcatcctcaccgagatatga | - | kinase_related | - | |
| 13057 | STK38 | NM_007271.2 | 586 | tggtgaggtacggcttgttcaga | - | see also 5071 line | | |
| 13058 | STK38L | NM_015000.1 | 1327 | gtgtcgactggagcacataagg | - | see also 6174 line | - | |
| 13059 | STK39 | NM_013233.1 | 985 | gagcagcggcccttatcacaaatat | - | kinase_related | receptor signaling protein serine/threonine kinase | - |
| 13060 | STK4 | NM_006282.2 | 1379 | atggagactacgagtttcttaag | - | see also 4374 line | - | |
| 13061 | STK6 | NM_003600.2 | 1096 | ctgtcattcgaagagagttattc | - | - | - | |
| 13062 | SYK | NM_003177.3 | 2020 | cggctgcgcaattactactatga | - | kinase_related, signal transduction | protein tyrosine kinase | - |
| 13063 | SYN3 | NM_003490.2 | 1043 | tggacagctgctcggaaaatgtt | - | - | - | |
| 13064 | TAP1 | NM_000593.4 | 1078 | gtgacgggatctataaacaacacc | - | - | oligopeptide transporter | small cell lung cancer, diabetes, non-small-cell lung cancer |
| 13065 | TAP2 | NM_000544.2 | 137 | tggttggacgcggctttactgtgg | - | - | - | diabetes |
| 13066 | TARS | NM_152295.3 | 636 | ccgacaacaccgttattgctaaa | - | see also 9832 line | - | |
| 13067 | TBK1 | NM_013254.2 | 1128 | gggcgacgcttagtcttagaacc | - | kinase_related | - | |
| 13068 | TCEB3L2 | NM_145653.1 | 1329 | cggccgacagacaaagatgatct | - | transcription_regulator | - | |
| 13069 | TCP1 | NM_030752.1 | 1132 | acgtctgcatcgattatcttacg | - | see also 8925 line | - | |
| 13070 | TEC | NM_003215.1 | 1882 | ttggcgtccaactatgtgtatga | - | kinase_related | non-membrane spanning protein tyrosine kinase | - |
| 13071 | TEK | NM_000459.1 | 708 | gagtgtactggccaggtatata | - | see also 358 line | - | |
| 13072 | TESK1 | NM_006285.1 | 1801 | caggaccgtcctcaattaacaat | - | kinase_related | protein tyrosine kinase | - |
| 13073 | TESK2 | NM_007170.1 | 1107 | caggccgatccggactatcttcc | - | kinase_related, apoptosis | - | |
| 13074 | TEX14 | NM_031272.2 | 1611 | aagatatccgtatattctgaag | - | see also 8999 line | - | |
| 13075 | TGFBR1 | NM_004612.1 | 1221 | aagttctcgatgattccataaat | - | see also 3133 line | - | |

Figure 46

| | | | | | | | | | thyroid cancer, colorectal cancer, colorectal carcinoma, colorectal cancer, esophageal cancer, breast cancer, pancreatic cancer, small cell lung cancer, Ewing's sarcoma, pleuropulmonary blastoma, colorectal cancers, non-small-cell lung cancer, malignant mesothelioma, gastric cancer |
|---|---|---|---|---|---|---|---|---|---|
| 13076 | TGFBR2 | NM_003242.3 | 2008 | ctgaagacggctccctaaacact | 102584 | - | receptor_acitivity, sig nal_transduction, kina se_related | type II transforming growth factor-beta receptor | |
| 13077 | TIE | NM_005424.2 | 556 | gagcaacggatcctacttctaca | 102587 | - | receptor_acitivity, kin ase_related, signal_tra nsduction | transmembrane receptor protein tyrosine kinase | - |
| 13078 | TIMM44 | NM_006351.2 | 180 | ctgtccgccttgctagataatgt | 102619 | - | see also 4444 line | | - |
| 13079 | TK1 | NM_003258.1 | 528 | ccgggaagccgcctataccaaga | 102642 | - | kinase_related | thymidine kinase | - |
| 13080 | TK2 | NM_004614.2 | 666 | gggtcttacgctacagacttatg | 102647 | - | kinase_related | Smad3/Sp-1 heterodimer | - |
| 13081 | TLK1 | NM_012290.3 | 741 | gggacgtatagatgatttgctca | 102672 | - | see also 5257 line | | - |
| 13082 | TNK1 | NM_003985.1 | 614 | gcgagaggtatcggtcatgatga | 102728 | - | kinase_related | | - |
| 13083 | TOMIL2 | NM_144678.2 | 355 | ttgtggccaaccgagatttcatc | 102735 | - | see also 9730 line | | - |
| 13084 | TOPK | NM_018492.2 | 1032 | taggccaactattaatatggaag | 102769 | - | kinase_related | | - |
| 13085 | TOR1B | NM_014506.1 | 803 | ctgtcggagtcttcaataataaa | 102787 | - | | heat shock protein | - |
| 13086 | TPX2 | NM_012112.4 | 2211 | gaggacgaaccggtagtgataaa | 102828 | - | | GTP binding | - |
| 13087 | TRA1 | NM_003299.1 | 827 | taggacggggaacgacgacaattcac | 102862 | - | see also 2230 line | | - |
| 13088 | TRAD | NM_007064.1 | 1729 | ctgcaatcgccattgagtatca | 102888 | - | see also 4944 line | | - |
| 13089 | TRAP1 | NM_016292.1 | 816 | gagatgttgttaacgaagtacagc | 102956 | - | | tumor necrosis factor receptor ligand | - |
| 13090 | TRAP100 | NM_014815.1 | 602 | ctgtcgcacatcgccaaactaga | 102983 | - | see also 6010 line | | - |
| 13091 | TRAP150 | NM_005119.1 | 513 | atggaaactaccgctcaaattgg | 103012 | - | see also 3590 line | | - |
| 13092 | TRB2 | NM_021643.1 | 1385 | ttgtcgcattgcgtttcttgtat | 103064 | - | see also 8162 line | | - |
| 13093 | TRIO | NM_007118.1 | 8370 | ttgccgcttagacttagcttcc | 103215 | - | kinase_related, phosp hatase, signal_transdu ction | protein serine/threonine kinase | - |
| 13094 | TRIP13 | NM_004237.2 | 974 | aaggcattccaatgttgtgattc | 103240 | - | receptor_acitivity, tra nscription_regulator | transcription cofactor | - |
| 13095 | TRPC4AP | NM_015638.1 | 1709 | tggacagcgagtgtaagtcaagg | 103289 | - | see also 6526 line | | - |
| 13096 | TRPM6 | NM_017662.3 | 2312 | atgacgcttgacgtatgaact | 103345 | - | see also 7087 line | | - |
| 13097 | TRPM7 | NM_017672.2 | 2203 | ctgtaagatctatcgttcaatgg | 103531 | - | see also 7091 line | | - |
| 13098 | TTBK2 | NM_173500.2 | 2936 | gtgcgaaagttacgttccattca | 103698 | - | see also 10073 line | | - |
| 13099 | TTC12 | NM_017868.2 | 1757 | ctgcatcacgttatgctataaag | 103775 | - | - | | - |

Figure 46

| | | | | | | | | | |
|---|---|---|---|---|---|---|---|---|---|
| 13100 | TTK | NM_003318.3 | 1768 | ttgatagttaccggaacgaaata | 103836 | - | kinase_related、cell_cycle | protein tyrosine kinase | - |
| 13101 | TTN | NM_003319.2 | 9522 | atgttcgcatccgaagtattaaa | 104080 | - | see also 2250 line | | |
| 13102 | TXK | NM_003328.1 | 1251 | agggatttggcggcaaggaattg | 107043 | - | kinase_related | non-membrane spanning protein tyrosine kinase | - |
| 13103 | TXL-2 | NM_178130.2 | 532 | ctgtaccttggccatcattaaac | 107066 | - | kinase_related | - | - |
| 13104 | TXNDC3 | NM_016616.2 | 1327 | ttggcattacttcgaccaaatct | 107107 | - | kinase_related | - | - |
| 13105 | TYK2 | NM_003331.3 | 1835 | gagcttgcggctccgaaagttcc | 107134 | - | see also 2256 line | | |
| 13106 | TYRO3 | NM_006293.2 | 2758 | gtgactgtcggtacatactcacc | 107176 | - | receptor_acitivity、kinase_related、signal_transduction | transmembrane receptor protein tyrosine kinase | rheumatoid arthritis |
| 13107 | UCK1 | NM_031432.1 | 422 | gtggaggtgccgacctatgattt | 107181 | - | kinase_related | uridine kinase | - |
| 13108 | ULK1 | NM_003565.1 | 811 | ctgcggctcccccatgtacatgg | 107187 | - | kinase_related、signal_transduction | protein tyrosine kinase | - |
| 13109 | ULK2 | NM_014683.2 | 982 | ttgctcgttacctacatagtaac | 107208 | - | see also 5884 line | | |
| 13110 | UMP-CMPK | NM_016308.1 | 111 | ctgctctccacgtctcatgaagc | 107256 | - | kinase_related | uridine kinase | - |
| 13111 | UMPK | NM_012474.3 | 665 | cggctctcacgcagagtattaag | 107276 | - | kinase_related | uridine kinase | meningitis |
| 13112 | URKL1 | NM_017859.1 | 523 | ttgacttcgacctcatcatttcc | 107282 | - | kinase_related | uridine kinase | - |
| 13113 | VARS2 | NM_006295.1 | 3305 | gtgcctgaaacctgtactgaatg | 107312 | - | see also 4390 line | | |
| 13114 | VCP | NM_007126.2 | 537 | aagtacggcaaacgtatccatgt | 107325 | - | - | adenosinetriphosphatase | - |
| 13115 | VPS11 | NM_021729.3 | 667 | gagaacgtccagtcctatatagt | 107363 | - | - | - | - |
| 13116 | VPS4A | NM_013245.2 | 1387 | cagacgacctcctgaaagtgaag | 107415 | - | see also 5363 line | | |
| 13117 | VPS4B | NM_004869.2 | 944 | gtgaagccgcacgtagaattaag | 107431 | - | transcription_regulator | peroxisome-assembly ATPase | - |
| 13118 | VRK1 | NM_003384.1 | 375 | tcgtacccgtaagctgaagtacc | 107465 | - | see also 2280 line | | |
| 13119 | VRK2 | NM_006296.2 | 867 | cagacgaagtgacgttgagatcc | 107506 | - | see also 4391 line | | |
| 13120 | VRK3 | NM_016440.2 | 381 | ccgtcacctctcccgattatcc | 107531 | - | kinase_related | - | - |
| 13121 | WARS | NM_004184.2 | 475 | aggcatagactacgataagctca | 107543 | - | - | tryptophan-tRNA ligase | - |
| 13122 | WARS2 | NM_015836.1 | 397 | atggtcagactacctcgattaca | 107581 | - | - | tryptophan-tRNA ligase | - |
| 13123 | WEE1 | NM_003390.2 | 2012 | atgaagtcccggtatacaacaga | 107613 | - | see also 2287 line | | |
| 13124 | XAB1 | NM_007266.1 | 822 | aagggagtatcgtcctgaatatg | 107671 | - | signal_transduction | - | - |
| 13125 | YES1 | NM_005433.2 | 839 | ttgcgactagaggttaaactagg | 107693 | - | kinase_related | protein tyrosine kinase | - |

Figure 46

| | | | | | | | | | |
|---|---|---|---|---|---|---|---|---|---|
| 13126 | YME1L1 | NM_014263.2 | 955 | ctgtccgcttccggacaacaaca | 107730 | - | see also 5570 line | | |
| 13127 | ZAK | NM_016653.1 | 533 | ttggtgcctctcggttccataac | 107777 | - | see also 6967 line | | |
| 13128 | ZAP70 | NM_001079.2 | 721 | aggaggccgagcgcaaactttac | 107845 | - | kinase_related | | selective T-cell defect |
| 13129 | ZCWCC3 | NM_015358.1 | 2733 | ccgatctctcgagttaacgtag | 107941 | - | | ATP binding | |
| 13130 | CNGA3 | NM_001298.1 | 252 | caggctgtcgcgcctcatcttct | 107944 | - | see also 858 line | | |
| 13131 | EPAC | NM_006105.2 | 1001 | gggcacttcggtgtacattatct | 107963 | - | kinase_related, signal_transduction | | |
| 13132 | HCN3 | NM_020897.1 | 383 | trggatcgtcttcaacgtattgt | 107978 | - | see also 8020 line | | |
| 13133 | HCN4 | NM_005477.1 | 2520 | cttggccgacggctcctactttgg | 108004 | - | channel | | |
| 13134 | PRKAR1A | NM_002734.2 | 454 | atgcggcatcctatgttagaaag | 108016 | - | see also 1773 line | | |
| 13135 | PRKAR2A | NM_004157.1 | 1263 | atgcttagttattgatgatgtacaag | 108064 | - | see also 2797 line | | |
| 13136 | PRKAR2B | NM_002736.1 | 1346 | aggaacatcgtacctatgaaga | 108094 | - | kinase_related | cAMP-dependent protein kinase, regulator | |
| 13137 | ACAS2 | NM_018677.2 | 2064 | aggcgagtgcttcggaagattgc | 108124 | - | see also 7550 line | | |
| 13138 | ADSS | NM_001126.2 | 881 | aagttctagctaaccaatacaaa | 108134 | - | see also 714 line | | |
| 13139 | ADSSL1 | NM_152328.3 | 1094 | cttggctcgacctgatgattctaa | 108175 | - | | | |
| 13140 | APMCF1 | NM_021203.2 | 263 | aggttgttaacaggcctttatag | 108192 | - | see also 8138 line | | |
| 13141 | ARF1 | NM_001658.2 | 233 | tggaaaccgtggagtacaagaac | 108208 | - | signal_transduction | small monomeric GTPase | |
| 13142 | ARF4 | NM_001660.2 | 639 | agggcttcagtctttcgtaaca | 108219 | - | signal_transduction | protein transporter | |
| 13143 | ARF4L | NM_001661.1 | 433 | cggacagacggtctagtgtttgt | 108222 | - | signal_transduction | ARF small monomeric GTPase | |
| 13144 | ARF6 | NM_001663.2 | 1018 | atgttaacctaactaacaaat | 108240 | - | see also 1096 line | | |
| 13145 | ARFD1 | NM_001656.2 | 1237 | tccggtcgttacgttaggattgg | 108279 | - | see also 1095 line | | |
| 13146 | ARFIP2 | NM_012402.2 | 933 | caggcccatcggacaagtatga | 108314 | - | see also 5310 line | | |
| 13147 | ARFRP1 | NM_003224.2 | 430 | cggcgtcatctacgtcattgact | 108326 | - | see also 2183 line | | |
| 13148 | ARHA | NM_001664.1 | 375 | cagataccgatgttatactgatg | 108329 | - | | Rho small monomeric GTPase | breast cancer, Alzheimer disease |
| 13149 | ARHB | NM_004040.1 | 224 | cggacaccgacgtcattctcatg | 108336 | - | signal_transduction | Rho small monomeric GTPase | |
| 13150 | ARHD | NM_014578.2 | 384 | tggtaccagaagtgaatcattt | 108341 | - | signal_transduction | Rho small monomeric GTPase | |
| 13151 | ARHE | NM_005168.2 | 647 | tgagcagctacttattatcgaat | 108359 | - | see also 3637 line | | |
| 13152 | ARHF | NM_019034.2 | 566 | cttgaatgttccgccaagtttcg | 108368 | - | signal_transduction | Rho small monomeric GTPase | |
| 13153 | ARHG | NM_001665.1 | 384 | cagtccgccgtcctatgagaacg | 108371 | - | cell_cycle, signal_transduction | Rho small monomeric GTPase | |

Figure 46

| | | | | | | | | | |
|---|---|---|---|---|---|---|---|---|---|
| 13154 | ARHGAP1 | NM_004308.2 | 976 | gtggatttcgaccagtacaatga | 108386 | - | signal_transduction | SH3/SH2 adaptor protein | - |
| 13155 | ARHGEF5 | NM_005435.2 | 1546 | gtgcagcaggtggagttcatttc | 108397 | - | - | guanyl-nucleotide release factor | - |
| 13156 | ARHH | NM_004310.1 | 830 | ctgtggccaaccataactcattc | 108399 | - | kinase_related、transcription_regulator、signal_transduction | Rho small monomeric GTPase | - |
| 13157 | ARHI | NM_004675.1 | 325 | cggcaacttccgtcatgagtacc | 108401 | - | signal_transduction、cell_cycle、kinase_related | small monomeric GTPase | small cell lung cancer |
| 13158 | ARHJ | NM_020663.2 | 876 | tggcccgtttgctgtatatgaaa | 108412 | - | signal_transduction | - | - |
| 13159 | ARHN | NM_005440.2 | 630 | ggggaatgagggcgagatacaca | 108421 | - | signal_transduction | Rho small monomeric GTPase | - |
| 13160 | ARHT1 | NM_018307.2 | 871 | gtgcttcgacgatttggttatga | 108441 | - | see also 7378 line | | |
| 13161 | ARHT2 | NM_138769.1 | 1524 | gtgacgttgcctgcttgatgttt | 108485 | - | signal_transduction | - | - |
| 13162 | ARHU | NM_021205.3 | 1278 | tcgtcgctggcattcaatactcg | 108493 | - | signal_transduction | - | - |
| 13163 | ARHV | NM_133639.2 | 545 | gggacgatgtcaacgtactaatt | 108494 | - | signal_transduction | - | - |
| 13164 | ARL1 | NM_001177.3 | 271 | aagttgttactactatacctacc | 108501 | - | signal_transduction | small monomeric GTPase | - |
| 13165 | ARL3 | NM_004311.2 | 581 | aggactgaacctgcataccatcc | 108521 | - | signal_transduction、gpcr | small monomeric GTPase | - |
| 13166 | ARL5 | NM_012097.2 | 822 | atgtcacgacttaagattagatg | 108542 | - | see also 5127 line | | |
| 13167 | ARL6 | NM_032146.3 | 713 | ccgtcgaattccaatcttattct | 108556 | - | see also 9083 line | | |
| 13168 | ARL6IP2 | NM_022374.1 | 485 | gtgattgacagacctaatggaac | 108574 | - | see also 8355 line | | |
| 13169 | ARL8 | NM_178815.2 | 520 | agggaacgactagctattacaaa | 108628 | - | signal_transduction | - | - |
| 13170 | C14orf159 | NM_024952.3 | 1672 | aagacgcagatcccgatattaac | 108645 | - | - | - | - |
| 13171 | CDC42 | NM_001791.2 | 416 | ctgtccaaagactcctttcttgc | 108668 | - | - | - | - |
| 13172 | DIRAS2 | NM_017594.2 | 97 | ctggtgttgaggtttgtgaaagg | 108672 | - | signal_transduction | - | - |
| 13173 | DKFZP564J0863 | NM_015459.2 | 573 | atgctacgatacttatattctca | 108678 | - | - | - | - |
| 13174 | DNCLI1 | NM_016141.1 | 1402 | aaggcgttctggcaaatttcttc | 108742 | - | see also 6690 line | | |
| 13175 | DNM1 | NM_004408.1 | 1638 | ctggctgactatcaataatattg | 108759 | - | - | motor | - |
| 13176 | DNM1L | NM_005690.2 | 1895 | aggttattgaacgactcattaaa | 108828 | - | see also 3950 line | | |
| 13177 | DNM2 | NM_004945.1 | 1515 | gcgaatcgtcaccacttacatcc | 108861 | - | see also 3443 line | | |
| 13178 | DRG2 | NM_001388.3 | 273 | tgggtaagtccacattcttgagt | 108867 | - | signal_transduction | GTP binding | - |
| 13179 | FLJ10101 | NM_024718.2 | 928 | tcggcctaaagtaccttcataag | 108884 | - | signal_transduction | - | - |
| 13180 | FLJ10702 | NM_018184.1 | 359 | aaggtaacgtcacaataaagatc | 108889 | - | signal_transduction | - | - |
| 13181 | FLJ10849 | NM_018243.2 | 1048 | ttgtaccgacgctgtaagcttga | 108928 | - | cell_cycle | - | - |

Figure 46

| 13182 | FLJ21665 | NM_024803.1 | 836 | ctggtaccttatccgagaataca | 108946 | - | - | - | - |
|---|---|---|---|---|---|---|---|---|---|
| 13183 | FLJ22595 | NM_025047.1 | 736 | gagacactttggcgttcttcaag | 108965 | - | signal_transduction | - | - |
| 13184 | FLJ22655 | NM_024730.2 | 737 | gagacgtcccagtggatctaaat | 108974 | - | signal_transduction | - | - |
| 13185 | FLJ25410 | NM_144605.1 | 480 | gggctacatcaacgagcaatacg | 108978 | - | cell_cycle | - | - |
| 13186 | FLJ39249 | NM_173664.2 | 667 | tgggtctacaggctatcgataac | 108990 | - | - | - | - |
| 13187 | GBP1 | NM_002053.1 | 661 | cagatgagtacctgacatactcc | 108992 | - | - | RNA polymerase II transcription factor | - |
| 13188 | GBP2 | NM_004120.3 | 870 | tggagctttcgctaaagctaaga | 108996 | - | - | GTPase | - |
| 13189 | GBP3 | NM_018284.1 | 1862 | gagatatatgtcgcataagctaa | 109021 | - | - | - | - |
| 13190 | GBP4 | NM_052941.2 | 1878 | ttgacatggctagcaacataatg | 109034 | - | - | - | - |
| 13191 | GBP5 | NM_052942.2 | 2244 | cagaggactgttaataacgatga | 109051 | - | - | transcription factor | - |
| 13192 | GEM | NM_005261.2 | 696 | cggggacgcatacctgattgtct | 109055 | - | signal_transduction | - | - |
| 13193 | GNA12 | NM_007353.1 | 501 | cagccggagaagcgagtttcagc | 109062 | - | signal_transduction、gpcr | signal transducer | - |
| 13194 | GNA13 | NM_006572.3 | 543 | ccggcgtcgagaatttcaactgg | 109081 | - | see also 4606 line | | |
| 13195 | GNA14 | NM_004297.2 | 783 | tggacacgctaaggatacagtat | 109100 | - | see also 2880 line | | |
| 13196 | GNA15 | NM_002068.1 | 1146 | gaggttcatcctggacatgtaca | 109120 | - | see also 1335 line | | |
| 13197 | GNAI1 | NM_002069.4 | 985 | aggagtgacggcgatcatcttct | 109141 | - | gpcr、signal_transduction | signal transducer | - |
| 13198 | GNAI2 | NM_002070.1 | 546 | tggccgctcaagggaataccagc | 109150 | - | gpcr、signal_transduction | signal transducer | adrenal cortical tumor、pituitary adenoma、ventricular tachycardia |
| 13199 | GNAI3 | NM_006496.1 | 305 | tgggacggctaaagattgacttt | 109163 | - | signal_transduction、gpcr | signal transducer | - |
| 13200 | GNAL | NM_002071.1 | 765 | cagcgtcagcttggttgactaca | 109193 | - | see also 1336 line | | |
| 13201 | GNAO1 | NM_020988.1 | 394 | tggggcgactcaggaatccaaga | 109213 | - | signal_transduction、gpcr | receptor signaling protein | - |
| 13202 | GNAS | NM_000516.3 | 985 | cagaatttgctcgctacactact | 109228 | - | gpcr、signal_transduction | - | Albright hereditary osteodystrophy、McCune-Albright syndrome、panostotic fibrous dysplasia、pseudohypoparathyroidism、erythroid leukemia |
| 13203 | GNAT1 | NM_000172.2 | 919 | acgtcttcttcgagaagatcaag | 109244 | - | signal_transduction、gpcr | - | night blindness |
| 13204 | GNAT2 | NM_005272.2 | 920 | aagatgacgaagtgaatcgtatg | 109255 | - | gpcr、receptor_acitivity、signal_transduction | GTP binding | achondroplasia |
| 13205 | GNAZ | NM_002073.1 | 1027 | gcggtgacagacgtcatcataca | 109266 | - | see also 1338 line | | |
| 13206 | GNL1 | NM_005275.2 | 1250 | aggctacgacccaaatcgatacc | 109271 | - | signal_transduction | GTP binding | - |

Figure 46

| 13207 | GTPBP4 | NM_012341.1 | 1584 | gagaaggagatgcgtagtcttgg | 109334 | - | see also 5299 line | | |
| 13208 | GTPBP5 | NM_015666.2 | 1175 | gtggactcaagttgacgatttaa | 109349 | - | - | - | - |
| 13209 | KBRAS2 | NM_017595.4 | 304 | gcgagagcaggtgcgtttctatg | 109410 | - | kinase_related、signal_transduction | - | - |
| 13210 | KIAA0820 | NM_015569.1 | 1572 | aggaccaggtattgctattgatt | 109449 | - | see also 6486 line | | |
| 13211 | LOC51277 | NM_016544.1 | 87 | caggtctctccgcatcaaagtca | 109491 | - | see also 6910 line | | |
| 13212 | LOC51762 | NM_016530.1 | 498 | gggagaagctagcaattgactat | 109519 | - | signal_transduction | protein transporter | - |
| 13213 | MEL | NM_005370.4 | 520 | cggaactggattcgcaacattga | 109530 | - | - | protein transporter | - |
| 13214 | MGC10731 | NM_030907.2 | 518 | cagcagccgtgtcgtcatgtttc | 109548 | - | signal_transduction | - | - |
| 13215 | MGC2827 | NM_023940.2 | 333 | ccggttcctcaccaaacgattca | 109558 | - | signal_transduction | - | - |
| 13216 | MGC4083 | NM_032525.1 | 202 | gagtcatcgtctcagaaatatgt | 109566 | - | - | - | - |
| 13217 | MGC9726 | NM_177403.3 | 764 | atgtggtgcaagcgtttgagatg | 109578 | - | - | - | - |
| 13218 | MRAS | NM_012219.2 | 159 | aagactcctacctgaaacatacg | 109582 | - | signal_transduction | RAS small monomeric GTPase | - |
| 13219 | MSF | NM_006640.2 | 1230 | aagtccatcacgcacgatattga | 109592 | - | cell_cycle | - | - |
| 13220 | MX1 | NM_002462.2 | 854 | atgtcccggatctgactctaata | 109608 | - | signal_transduction、apoptosis | antiviral response protein | - |
| 13221 | MX2 | NM_002463.1 | 1752 | cagagcacgattgaagacataaa | 109647 | - | - | antiviral response protein | - |
| 13222 | NEDD5 | NM_004404.1 | 732 | tggacttaagcccttagatgtgg | 109668 | - | cell_cycle | GTPase | - |
| 13223 | NRAS | NM_002524.2 | 445 | cagtgccatgagagaccaataca | 109675 | - | cell_cycle、signal_transduction | pre-mRNA splicing factor | congenital neutropenia、breast cancer、neuroblastoma、melanoma |
| 13224 | OPA1 | NM_015560.1 | 428 | atgataccggaccttagtgaata | 109695 | - | see also 6473 line | | |
| 13225 | PCK1 | NM_002591.2 | 188 | ctgaacctctcggccaaagttgt | 109790 | - | kinase_related | phosphoenolpyruvate carboxykinase (GTP) | phosphoenolpyruvate carboxykinase deficiency |
| 13226 | PCK2 | NM_004563.1 | 1437 | aggggtacccctggtatacgagg | 109829 | - | kinase_related | phosphoenolpyruvate carboxykinase (GTP) | phosphoenolpyruvate carboxykinase deficiency |
| 13227 | PGPL | NM_012227.1 | 718 | gagtcggctcgcgctacatcatg | 109836 | - | - | - | - |
| 13228 | PNUTL1 | NM_002688.2 | 1100 | acgccgagactgagaagcttatc | 109842 | - | cell_cycle | structural molecule | - |
| 13229 | PNUTL2 | NM_004574.2 | 1066 | ttggaatcaagatctatcaattc | 109853 | - | see also 3111 line | | |
| 13230 | PTD004 | NM_013341.2 | 600 | ctgttcgcttctatcatgattgg | 109881 | - | - | - | - |
| 13231 | RAB11A | NM_004663.3 | 339 | gagcgatatcgagctataacatc | 109888 | - | see also 3184 line | | |
| 13232 | RAB11B | NM_004218.1 | 485 | acgtagaggaagcattcaagaac | 109904 | - | signal_transduction | protein transporter | - |
| 13233 | RAB14 | NM_016322.2 | 407 | ctgttacacggagctactacaga | 109914 | - | see also 6810 line | | |
| 13234 | RAB17 | NM_022449.1 | 1148 | caggtgtcggaggtgttcaatac | 109931 | - | signal_transduction | protein transporter | - |

Figure 46

| 13235 | RAB18 | NM_021252.3 | 286 | gaggtgcacagggtgttatatta | 109937 | - | see also 8145 line | | |
| 13236 | RAB1A | NM_004161.2 | 188 | atgaatcccgaatatgattattt | 109950 | - | see also 2798 line | | |
| 13237 | RAB2 | NM_002865.1 | 332 | gagttcggtgctcgaatgataac | 109977 | - | - | protein transporter | - |
| 13238 | RAB20 | NM_017817.1 | 659 | gaggatgcggtggccctttataa | 109994 | - | signal_transduction | small monomeric GTPase | - |
| 13239 | RAB21 | NM_014999.1 | 372 | ctgcgagaacaagtttaacgaca | 110002 | - | signal_transduction | protein transporter | - |
| 13240 | RAB22A | NM_020673.2 | 465 | gtgccttagcaccaatgtactat | 110023 | - | see also 7914 line | | |
| 13241 | RAB23 | NM_016277.3 | 1162 | cagaactaacgcattcaagtagt | 110057 | - | signal_transduction | protein transporter | - |
| 13242 | RAB24 | NM_130781.1 | 818 | gagggctgccaaatctacttatg | 110070 | - | signal_transduction | - | - |
| 13243 | RAB27A | NM_004580.3 | 856 | atgcctctacggatcagttaagt | 110085 | - | see also 3119 line | | |
| 13244 | RAB27B | NM_004163.3 | 566 | ctgacaaatatggcataccatat | 110092 | - | signal_transduction | RAB small monomeric GTPase | - |
| 13245 | RAB28 | NM_004249.1 | 637 | cagagtagaatctgttcagtaca | 110106 | - | signal_transduction | RAB small monomeric GTPase | - |
| 13246 | RAB2B | NM_032846.2 | 697 | cagcggaactctcgtgacatagg | 110117 | - | see also 9310 line | | |
| 13247 | RAB30 | NM_014488.3 | 464 | aagagagatttcggtccattacc | 110124 | - | see also 5704 line | | |
| 13248 | RAB31 | NM_006868.2 | 188 | aagcatcgtgtgtcgatttgtcc | 110134 | - | see also 4807 line | | |
| 13249 | RAB32 | NM_006834.2 | 436 | aggagcgatttggcaacatgacc | 110148 | - | signal_transduction | RAB small monomeric GTPase | - |
| 13250 | RAB33A | NM_004794.2 | 564 | aagcatggtcgagcattactacc | 110158 | - | signal_transduction | RAB small monomeric GTPase | - |
| 13251 | RAB33B | NM_031296.1 | 966 | atgaccttggctcataagcttaa | 110190 | - | see also 9007 line | | |
| 13252 | RAB34 | NM_031934.3 | 1205 | tggtgagaatgtccgagaattct | 110206 | - | see also 9056 line | | |
| 13253 | RAB35 | NM_006861.4 | 445 | gcggtggcttcacgaaatcaacc | 110214 | - | see also 4806 line | | |
| 13254 | RAB36 | NM_004914.2 | 388 | ggggactgtcgggctcaaactct | 110223 | - | signal_transduction | protein transporter | - |
| 13255 | RAB37 | NM_175738.2 | 711 | ccgaagcgtcacccatgcttatt | 110226 | - | signal_transduction | protein transporter | - |
| 13256 | RAB38 | NM_022337.1 | 128 | gcgctacgtgcaccagaacttct | 110236 | - | see also 8331 line | | |
| 13257 | RAB39 | NM_017516.1 | 232 | caggagcggttcagatcaataac | 110252 | - | - | - | - |
| 13258 | RAB3C | NM_138453.2 | 462 | aagattggtcaactcaaatcaaa | 110290 | - | signal_transduction | - | - |
| 13259 | RAB3D | NM_004283.2 | 579 | aggactgggccacgcaaatcaag | 110295 | - | signal_transduction | protein transporter | - |
| 13260 | RAB40C | NM_021168.1 | 139 | cagtaacgggatcgactacaaga | 110298 | - | signal_transduction | - | - |
| 13261 | RAB4A | NM_004578.2 | 434 | aggtccgtgacgagaagttatta | 110307 | - | see also 3117 line | | |
| 13262 | RAB4B | NM_016154.2 | 1172 | cggtcagtgacgcggagttatta | 110324 | - | - | protein transporter | - |
| 13263 | RAB5A | NM_004162.3 | 458 | gagtccgctgttggcaaatcaag | 110330 | - | see also 2800 line | | |
| 13264 | RAB5B | NM_002868.2 | 252 | aagtcaagcctggtattacgttt | 110351 | - | - | protein transporter | - |
| 13265 | RAB5C | NM_004583.1 | 179 | ctgggaacaagatctgtcaattt | 110366 | - | signal_transduction | protein transporter | - |
| 13266 | RAB7 | NM_004637.5 | 768 | aggtgggagctgtacaacgaattt | 110391 | - | - | protein transporter | Charcot-Marie-Tooth neuropathy |
| 13267 | RAB7L1 | NM_003929.1 | 190 | gtggtctgactacgagatagtgc | 110395 | - | signal_transduction | protein transporter | - |

Figure 46

| 13268 | RAB9B | NM_016370.1 | 349 | tcgttatgaaccgttacgtaac | 110410 | - | see also 6842 line | | |
|---|---|---|---|---|---|---|---|---|---|
| 13269 | RABL4 | NM_006860.2 | 595 | tgggagagtcccaatgtcttatg | 110421 | - | signal_transduction | small monomeric GTPase | - |
| 13270 | RAC3 | NM_005052.2 | 365 | ccggccctccttcgagaatgttcg | 110432 | - | - | Rho small monomeric GTPase | - |
| 13271 | RALA | NM_005402.2 | 515 | aggaggactacgctgcaattaga | 110442 | - | see also 3770 line | | - |
| 13272 | RAN | NM_006325.2 | 198 | acgtcatttgactgtgtgaatttg | 110473 | - | signal_transduction, cell_cycle | protein transporter | - |
| 13273 | RAP1A | NM_002884.1 | 545 | ttgcactagtatattcttattaca | 110573 | - | signal_transduction, cell_cycle | RAS small monomeric GTPase | - |
| 13274 | RAP1B | NM_015646.3 | 181 | aagtctgcttgactgtacaatt | 110580 | - | | RAS small monomeric GTPase | - |
| 13275 | RAP2A | NM_021033.3 | 684 | tggaaacttccgctaagagtaaa | 110586 | - | see also 8070 line | | - |
| 13276 | RAP2C | NM_021183.3 | 1302 | aagatcagtgttgtacaactgt | 110604 | - | see also 8117 line | | - |
| 13277 | RASD1 | NM_016084.3 | 248 | gagcgactcggagctgagtatcc | 110605 | - | signal_transduction, gpcr | | - |
| 13278 | RASD2 | NM_014310.3 | 798 | ccgccctgcatcgcaagatctcc | 110610 | - | signal_transduction | molecular_function unknown | - |
| 13279 | REM | NM_014012.4 | 746 | cagtgcctatgtcatcgtatact | 110612 | - | signal_transduction | | - |
| 13280 | RERG | NM_032918.1 | 341 | gggcaagtcagtcttgttagtga | 110617 | - | see also 9345 line | | - |
| 13281 | RHEBL1 | NM_144593.1 | 627 | ctgggtgcgacatttatggagt | 110639 | - | see also 9707 line | | - |
| 13282 | RHO6 | NM_014470.2 | 309 | caggatctccctactacgataat | 110650 | - | signal_transduction | Rho small monomeric GTPase | - |
| 13283 | RHOBTB1 | NM_014836.3 | 1769 | acgttctcggacgtgacatttaa | 110690 | - | signal_transduction | | - |
| 13284 | RHOBTB2 | NM_015178.1 | 351 | tgggccatcgaccaaatactgt | 110706 | - | see also 6269 line | | - |
| 13285 | RIS | NM_016563.2 | 336 | ctgaccaagaagtttatcagtga | 110724 | - | signal_transduction | | - |
| 13286 | RIT1 | NM_006912.2 | 636 | ctgctgcataccgctactatatt | 110742 | - | signal_transduction | | - |
| 13287 | RIT2 | NM_002930.1 | 530 | aggtccgccacacctatgaaatt | 110755 | - | see also 1971 line | | - |
| 13288 | RRAD | NM_004165.1 | 685 | cgggcgtgcacggcaaaacagatga | 110764 | - | signal_transduction | small monomeric GTPase | diabetes mellitus , diabetes |
| 13289 | RRAGA | NM_006570.3 | 749 | tgggatgagacgtctcacaaagc | 110776 | - | - | | - |
| 13290 | RRAGB | NM_006064.2 | 1205 | cagcgtgatgcccatagatttga | 110797 | - | signal_transduction | | - |
| 13291 | RRAS2 | NM_012250.3 | 731 | tagcacggcagcttaaggtaaca | 110819 | - | - | | - |
| 13292 | SARA1 | NM_020150.3 | 396 | cagcaattaatgggattgtcttt | 110831 | - | signal_transduction | small monomeric GTPase | - |
| 13293 | SARA2 | NM_016103.2 | 499 | ttgctaatgtgcctactactgatt | 110845 | | - | small monomeric GTPase | - |
| 13294 | 1.Sep | NM_052838.2 | 655 | ccgggcagtacacgagaaagtca | 110860 | - | cell_cycle | GTP binding | - |

Figure 46

| | | | | | | | | | |
|---|---|---|---|---|---|---|---|---|---|
| 13295 | 10.Sep | NM_144710.2 | 1710 | aggacaaggaccgtaagaactcc | 110879 | - | cell_cycle | - | - |
| 13296 | 3.Sep | NM_019106.4 | 628 | gcgacctctggatcttgagttca | 110885 | - | cell_cycle | - | - |
| 13297 | 6.Sep | NM_015129.4 | 266 | atggcagcgaccgatatagctcg | 110891 | - | cell_cycle | protein binding | - |
| 13298 | SGNE1 | NM_003020.1 | 589 | aggacagagactggataatgttg | 110911 | - | signal_transduction | enzyme activator | - |
| 13299 | SRPR | NM_003139.2 | 312 | tcggctgtttcgggacaagtacc | 110912 | - | see also 2120 line | | |
| 13300 | SUCLG1 | NM_003849.1 | 327 | gagcaacggcttctgtcatttat | 110940 | - | - | succinate-CoA ligase (GDP-forming) | - |
| 13301 | TUBA1 | NM_006000.1 | 366 | atgcccgtggtcactataccatt | 110950 | - | - | structural molecule | - |
| 13302 | TUBA4 | NM_025019.1 | 443 | tggagtggctttctgttaactat | 110954 | - | - | - | - |
| 13303 | TUBA8 | NM_018943.1 | 1379 | gggactgattcgtttgaagaaga | 110963 | - | - | structural molecule | - |
| 13304 | TUBB1 | NM_030773.1 | 222 | cagcattcgatctagcaaattag | 110969 | - | - | - | - |
| 13305 | TUBB4Q | NM_020040.2 | 731 | gggtacccttctgctcagtaaga | 110983 | - | - | structural molecule | - |
| 13306 | TUBD1 | NM_016261.1 | 811 | atgctgagtgttcgtaacattcc | 111012 | - | - | structural constituent of cytoskeleton | - |
| 13307 | TUBE1 | NM_016262.3 | 570 | atgtcataacctcaccttataat | 111043 | - | - | structural constituent of cytoskeleton | - |
| 13308 | TUBG1 | NM_001070.3 | 603 | gtggtggtccagccttacaattc | 111063 | - | - | structural constituent of cytoskeleton | - |
| 13309 | TUBG2 | NM_016437.1 | 739 | aagctagtgcagacttattcagt | 111065 | - | - | structural molecule | - |
| 13310 | VTS58635 | NM_033315.2 | 346 | tcgtgcgccagttcttgtacaac | 111068 | - | signal_transduction | - | - |
| 13311 | ZNF179 | NM_007148.2 | 1483 | gagggagttcgaggagtatgtga | 111073 | - | - | zinc binding | - |
| 13312 | CNGA1 | NM_000087.1 | 1018 | tgggtctaccctgatattaatga | 111106 | - | see also 66 line | | |
| 13313 | PDE10A | NM_006661.1 | 1525 | gtgcatttaccgggtaacgatgg | 111180 | - | see also 4709 line | | |
| 13314 | PDE5A | NM_001083.2 | 812 | ctgtatccgcttagaatggaaca | 111222 | - | see also 677 line | | |
| 13315 | C5orf3 | NM_018691.1 | 1198 | ccgaaatgctacactatctcagg | 111289 | - | see also 7564 line | | |
| 13316 | LOC56851 | NM_020154.1 | 228 | aagagcacgtcggtttccttaag | 111307 | - | see also 7772 line | | |
| 13317 | BIRC1 | NM_004536.1 | 511 | gagccgtacagctcatggatacc | 111332 | - | apoptosis | nucleotide binding | amyotrophic lateral sclerosis、 spinal muscular atrophy |
| 13318 | DHRS3 | NM_004753.3 | 962 | gaggagatccacaaattctcagg | 111357 | - | - | - | - |
| 13319 | NOX4 | NM_016931.2 | 1481 | aagtccatttgaggaatcactga | 111381 | - | - | - | - |
| 13320 | SLC22A4 | NM_003059.2 | 1159 | ctgttcaggactcggaatattgc | 111395 | - | - | - | - |
| 13321 | ABT1 | NM_013375.2 | 242 | gaggtcggacgcgtcttctttca | 111412 | - | see also 5413 line | | |
| 13322 | AIP | NM_003977.1 | 187 | gagctcccggactttcaagatgg | 111422 | - | transcription_regulator | transcription co-activator | - |
| 13323 | BRDT | NM_001726.1 | 1697 | tgggcgagtagttcacataatac | 111473 | - | transcription_regulator 、 kinase_related | - | - |

EP 1 752 536 A1

Figure 46

| 13324 | CRSP2 | NM_004229.2 | 395 | acgccaactcttcgttcgattat | 111517 | - | receptor_acitivity、tra nscription_regulator、s ignal_transduction | - | |
|-------|-------|-------------|-----|-------------------------|--------|---|------------------|------------------|---|
| 13325 | CRSP7 | NM_004831.2 | 1197 | tcgagactatacggttaacttgg | 111678 | - | receptor_acitivity、tra nscription_regulator | - | |
| 13326 | CRSP8 | NM_004269.2 | 667 | gggatataacgagaatgtctaca | 111699 | - | transcription_regulator | - | |
| 13327 | CRSP9 | NM_004270.3 | 525 | aagcacctggaacgagtaattga | 111719 | - | receptor_acitivity、tra nscription_regulator | - | |
| 13328 | DTX1 | NM_004416.1 | 850 | gcgcatggacggcctacgatatg | 111729 | - | transcription_regulator 、signal_transduction | - | |
| 13329 | GCN5L2 | NM_021078.1 | 878 | tggctacctacaaggtcaattac | 111738 | - | - | transcription co-activator | - |
| 13330 | GTF2A1 | NM_015859.1 | 907 | cagccaatggtgcccaatatatc | 111766 | - | transcription_regulator | transcription initiation factor | - |
| 13331 | HCF-2 | NM_013320.1 | 895 | gagtggaccaccctagtatcaga | 111804 | - | transcription_regulator | - | - |
| 13332 | HSGT1 | NM_007265.1 | 1924 | cagactaccgataacaattcaga | 111895 | - | see also 5063 line | | |
| 13333 | NCOA1 | NM_003743.3 | 3086 | aggggggtggattagatgtatta | 112014 | - | see also 2559 line | | |
| 13334 | NCOA2 | NM_006540.1 | 4441 | atgggtcccgagcaggttaatga | 112129 | - | see also 4581 line | | |
| 13335 | NCOA3 | NM_006534.2 | 950 | gtggcacgccgcattactacagg | 112153 | - | see also 4567 line | | |
| 13336 | NCOA4 | NM_005437.1 | 420 | tcgttattgggccagttcaattg | 112247 | - | see also 3791 line | | |
| 13337 | NRIP1 | NM_003489.1 | 3731 | aagcgtgctaacgataaagaaag | 112347 | - | transcription_regulator | - | - |
| 13338 | PCBD | NM_000281.1 | 313 | aagtagcagtgtccatgacatag | 112356 | - | see also 245 line | | |
| 13339 | PMF1 | NM_007221.1 | 146 | ccggcagctaccagagattcact | 112357 | - | - | - | - |
| 13340 | RNF14 | NM_004290.3 | 1532 | tagacggatgtaacaagatgaca | 112390 | - | see also 2874 line | | |
| 13341 | SMARCD2 | NM_003077.2 | 1322 | ccgttacttccgccagatcttca | 112403 | - | see also 2072 line | | |
| 13342 | SMARCD3 | NM_003078.2 | 1183 | aagacggcgtgctatgacattga | 112413 | - | see also 2074 line | | |
| 13343 | SNW1 | NM_012245.2 | 171 | aggctggatacctcggttattag | 112420 | - | transcription_regulator | transcription co-activator | - |
| 13344 | TAF7 | NM_005642.2 | 1133 | ctggaaccacggaattactctgc | 112459 | - | see also 3915 line | | |
| 13345 | TAZ | NM_015472.3 | 1096 | atgagatccatcactaataatag | 112479 | - | transcription_regulator | - | - |
| 13346 | TCERG1 | NM_006706.2 | 43 | gggcgagagtgaacgattcaacc | 112487 | - | see also 4731 line | | |
| 13347 | TGFB1I1 | NM_015927.3 | 874 | gagcgcttctcgccaagatgtgg | 112569 | - | transcription_regulator | - | - |

EP 1 752 536 A1

Figure 46

| No. | Symbol | Accession | Length | Sequence | ID | | Function | | Disease |
|---|---|---|---|---|---|---|---|---|---|
| 13348 | TNRC11 | NM_005120.1 | 5718 | ccgtcctgtcgcgttaccaatgc | 112654 | - | see also 3592 line | | |
| 13349 | TRAP95 | NM_005481.2 | 450 | atgccgacgggcagatcaagtgc | 112674 | - | receptor_acitivity, transcription_regulator, signal_transduction | | |
| 13350 | TRIM32 | NM_012210.2 | 1296 | ctgacgtggtaactatcgtata | 112709 | - | transcription_regulator | zinc binding | |
| 13351 | TRIP11 | NM_004239.1 | 4795 | aagcgttacaagagagactaacatg | 112816 | - | transcription_regulator | transcription co-activator | |
| 13352 | TRIP4 | NM_016213.2 | 1130 | cagccactgaccaaattggatag | 112877 | - | see also 6724 line | | |
| 13353 | UTF1 | NM_003577.1 | 363 | ccgctacaagttccttaaagaca | 112894 | - | transcription_regulator | | |
| 13354 | VGLL1 | NM_016267.2 | 911 | ctgtgggccaccacatcgatacc | 112913 | - | transcription_regulator | | |
| 13355 | CALR | NM_004343.2 | 773 | ctgggacaagccgccgagcatatcc | 112923 | - | transcription_regulator | transcription co-repressor | |
| 13356 | CREG | NM_003851.1 | 330 | cagcggggtgccctatttctacc | 112930 | - | transcription_regulator | | |
| 13357 | HD | NM_002111.4 | 5141 | gcgtccgtgagcgacttcaact | 113042 | - | transcription_regulator, apoptosis | transcription co-repressor | Huntington disease, diabetes, Huntington's chorea |
| 13358 | L3MBTL2 | NM_031488.3 | 955 | caggacgcttccgtggatttcc | 113130 | - | see also 9039 line | | |
| 13359 | NAB2 | NM_005967.1 | 1151 | agggacaacacgctcttattacg | 113153 | - | transcription_regulator | transcription co-repressor | |
| 13360 | PAWR | NM_002583.1 | 984 | gtgggttcctagatataacagg | 113162 | - | transcription_regulator, apoptosis | | Parkinson disease |
| 13361 | PBXIP1 | NM_020524.2 | 1233 | caggcattaaggcaagagttaga | 113169 | - | see also 7886 line | | |
| 13362 | PLRG1 | NM_002669.1 | 835 | aaggttatacggcattatcatgg | 113210 | - | see also 1745 line | | |
| 13363 | RNF12 | NM_016120.2 | 491 | atgtgactctataatagactgg | 113237 | - | see also 6674 line | | |
| 13364 | RYBP | NM_012234.3 | 676 | tgggcaacgtcaccgtcattatc | 113295 | - | transcription_regulator | | |
| 13365 | SAP18 | NM_005870.3 | 355 | aagacctggctatcgagttaagg | 113302 | - | see also 4085 line | | |
| 13366 | SAP30 | NM_003864.1 | 552 | gagcgcaaggcatcttacatat | 113306 | - | transcription_regulator | | |
| 13367 | SIAH2 | NM_005067.4 | 898 | acgcccacaagagcattaccacc | 113319 | - | see also 3535 line | | |
| 13368 | SUFU | NM_016169.2 | 1314 | ctgcatggacggcacttacata | 113338 | - | transcription_regulator, signal_transduction | | |
| 13369 | TTRAP | NM_016614.2 | 726 | ctgcggaacgaatgaatcagtta | 113365 | - | see also 6951 line | | |
| 13370 | TZFP | NM_014383.1 | 283 | cggccagcactcgtgatactct | 113381 | - | transcription_regulator | | |

Figure 46

| | | | | | | | | | |
|---|---|---|---|---|---|---|---|---|---|
| 13371 | YAF2 | NM_005748.2 | 418 | aagtcaccgcctgcatctagtgc | 113391 | - | transcription_regulator | transcription co-repressor | - |
| 13372 | ZFPM2 | NM_012082.2 | 2112 | cagccggcacgaaacatacatgg | 113432 | - | see also 5112 line | | |
| 13373 | NMI | NM_004688.1 | 554 | gtgagctcgaaagttccttatga | 113479 | - | transcription_regulator | transcription cofactor | - |
| 13374 | PCAF | NM_003884.3 | 1785 | ggggatattccgatggaattaat | 113527 | - | transcription_regulator、cell_cycle | - | - |
| 13375 | PIR | NM_003662.1 | 232 | tggggtcctccaagaaagttact | 113563 | - | transcription_regulator | transcription cofactor | - |
| 13376 | HDAC11 | NM_024827.1 | 244 | gtgcacacgaggcgctatcttaa | 113581 | - | transcription_regulator | - | - |
| 13377 | HDAC3 | NM_003883.2 | 359 | gagttctgctcgcgttacacagg | 113605 | - | see also 2640 line | | |
| 13378 | HDAC4 | NM_006037.2 | 1448 | gcggagtgtcgacctcctataac | 113639 | - | transcription_regulator、cell_cycle | transcriptional repressor | - |
| 13379 | HDAC5 | NM_005474.3 | 1219 | tggggcgtcgtccgtgtgtaaca | 113651 | - | see also 3811 line | | |
| 13380 | HDAC7A | NM_015401.1 | 2038 | tggcacagcggatgtttgtgatg | 113669 | - | transcription_regulator、cell_cycle | - | - |
| 13381 | HDAC8 | NM_018486.1 | 82 | ctggtcccggtttatatctatag | 113684 | - | transcription_regulator | - | - |
| 13382 | HDAC9 | NM_014707.1 | 266 | ctgttgtccgtgagaagcaattg | 113719 | - | see also 5898 line | | |
| 13383 | MYBBP1A | NM_014520.1 | 93 | ccgaccgctatggcctattgaag | 113783 | - | transcription_regulator | - | - |
| 13384 | NFKBIA | NM_020529.1 | 1026 | gaggccagcgtctgacgttatga | 113819 | - | transcription_regulator、apoptosis | Transcription factor p65 | diabetes mellitus、diabetes |
| 13385 | RNF19 | NM_015435.3 | 1621 | taggtatcggtgttcctattatg | 113855 | - | see also 6415 line | | |
| 13386 | VHL | NM_000551.2 | 756 | caggtcgctctacgaagatctgg | 113899 | - | transcription_regulator、apoptosis、cell_cycle | transcription factor binding | benign familial polycythemia、Von Hippel-Lindau desease、renal cell carcinoma、von Hippel-Lindau syndrome、CNS Tumours、phaeochromocytoma、pheochromocytoma、squamous cell cancer、familial nervous system tumor syndromes |
| 13387 | GSN | NM_000177.3 | 740 | cagcaatcggtatgaaagactga | 113910 | - | see also 151 line | | |
| 13388 | CAPG | NM_001747.1 | 864 | ttgcccttgaactgctgatatct | 113928 | - | - | barbed-end actin capping | - |
| 13389 | CAPPA3 | NM_033328.2 | 196 | gagtaattcgcagactgttatta | 113934 | - | see also 9406 line | | |
| 13390 | CAPZA1 | NM_006135.1 | 101 | atgacgttcggctactacttaat | 113977 | - | - | structural constituent of cytoskeleton | - |
| 13391 | CAPZA2 | NM_006136.1 | 766 | atgtcggacactactttcaaagc | 114012 | - | - | structural constituent of cytoskeleton | - |

Figure 46

| 13392 | CAPZB | NM_004930.1 | 482 | gcggtcgcaccgcccattacaag | 114028 | - | see also 3429 line | | |
|---|---|---|---|---|---|---|---|---|---|
| 13393 | EVL | NM_016337.1 | 323 | caggtctacggcttaaactttgc | 114039 | - | signal_transduction | - | - |
| 13394 | HRIHFB2122 | NM_007032.3 | 508 | aagaccgtacgtccaacttcagc | 114050 | - | see also 4921 line | | |
| 13395 | ARP3BETA | NM_020445.1 | 261 | gtggccgatacgacatggaatca | 114060 | - | - | - | - |
| 13396 | FLNA | NM_001456.1 | 3814 | tggcacgcacaccattacctaca | 114088 | - | signal_transduction、kinase_related | actin cross-linking | - |
| 13397 | FSCN3 | NM_020369.1 | 1456 | cagcccgaccgcattcatctact | 114129 | - | see also 7853 line | | |
| 13398 | MACF1 | NM_012090.3 | 9962 | gagatcaatgctcgatggaatac | 114333 | - | - | - | - |
| 13399 | PLS1 | NM_002670.1 | 1874 | aagatcggtgcccggatatatgc | 114666 | - | - | structural constituent of cytoskeleton | - |
| 13400 | SPTA1 | NM_003126.1 | 3411 | acgtcgtctattgcaacgttata | 114739 | - | see also 2099 line | | |
| 13401 | AVIL | NM_006576.2 | 710 | tggccgacgctccattatcaagc | 114835 | - | - | structural constituent of cytoskeleton | - |
| 13402 | CFL1 | NM_005507.1 | 288 | ctgccgctatgccctctatgatg | 114862 | - | signal_transduction | actin modulating | - |
| 13403 | ACTN4 | NM_004924.3 | 886 | ctgccgccaaccggatctgtaag | 114874 | - | see also 3427 line | | |
| 13404 | EPB49 | NM_001978.1 | 756 | cagccggtcgcctggaatcatct | 114884 | - | - | structural constituent of cytoskeleton | - |
| 13405 | FSCN1 | NM_003088.1 | 1153 | aggggcgtccaatggcaagtttgt | 114898 | - | - | actin bundling | - |
| 13406 | FSCN2 | NM_012418.1 | 1122 | ctgccaacaccatgtttgagatg | 114907 | - | - | actin bundling | - |
| 13407 | VIL1 | NM_007127.1 | 864 | cagtcacgaggactgttacatcc | 114916 | - | see also 4958 line | | |
| 13408 | DSTN | NM_006870.2 | 523 | aagttaggtggatccttaattgt | 114939 | - | - | actin filament severing | - |
| 13409 | WASF1 | NM_003931.1 | 457 | gtgtggaccgtttatctgttagt | 114948 | - | see also 2692 line | | |
| 13410 | WASF3 | NM_006646.3 | 906 | cggacgttacggattactcttac | 114992 | - | - | actin polymerizing | - |
| 13411 | ABLIM1 | NM_002313.4 | 1194 | tggctcaccaggtcatactatct | 115041 | - | see also 1485 line | | |
| 13412 | ABLIM2 | NM_032432.2 | 999 | gggtctccgagccgtgtgattta | 115085 | - | - | - | - |
| 13413 | ACTN1 | NM_001102.2 | 2638 | gagacagccgacacagatacagc | 115107 | - | - | structural constituent of cytoskeleton | - |
| 13414 | ACTN2 | NM_001103.1 | 2023 | tcgtgcccatccgcgatcaatcc | 115135 | - | - | structural constituent of muscle | - |
| 13415 | ACTN3 | NM_001104.1 | 2151 | gagcctggtgttcgacaataagc | 115151 | - | - | structural constituent of muscle | - |
| 13416 | ADD1 | NM_001119.3 | 255 | aggtacttcgaccgagtagatga | 115154 | - | see also 707 line | | |
| 13417 | ADD2 | NM_001617.1 | 508 | ccgcaggggcagccttactttga | 115196 | - | - | - | - |
| 13418 | ARPC1A | NM_006409.2 | 762 | ctgggtccacggggtaagcttct | 115310 | - | see also 4495 line | | |
| 13419 | BCL7B | NM_001707.2 | 232 | gtgacacgtccctgaggatattt | 115323 | - | - | - | - |

Figure 46

| No. | Symbol | Accession | Length | Sequence | ID | | Notes | Function | Disease |
|---|---|---|---|---|---|---|---|---|---|
| 13420 | BPAG1 | NM_001723.3 | 1239 | aagtcgtgcgactaatgaactta | 115366 | - | see also 1145 line | | |
| 13421 | C6orf209 | NM_018368.2 | 1542 | ctgttaccggacatacctattc | 116063 | - | | | |
| 13422 | C9orf40 | NM_017998.1 | 768 | ctggcagacattgaagatttaag | 116068 | - | | | |
| 13423 | CALD1 | NM_004342.4 | 587 | gagttcgaccaacaataacaga | 116074 | - | | | Alzheimer disease |
| 13424 | CEACAM1 | NM_001712.2 | 1306 | ctgaacgtaaactataatgctct | 116110 | - | | USF43/USF44 complex | |
| 13425 | CEACAM5 | NM_004363.1 | 2093 | tggctactggccgcaataattcc | 116131 | - | | USF43/USF44 complex | |
| 13426 | CFL2 | NM_021914.5 | 321 | gtgggtgacattggtgatacrgt | 116140 | - | see also 8196 line | | |
| 13427 | CGN | NM_020770.1 | 3702 | acgatccctcgtccattgcatca | 116178 | - | | | |
| 13428 | CLMN | NM_024734.2 | 1068 | gggagcgtacctacactgttaac | 116202 | - | | calmodulin binding | |
| 13429 | CNN1 | NM_001299.3 | 293 | ccgtccgcatcggccaacaacttca | 116243 | - | | calmodulin binding | |
| 13430 | CNN2 | NM_004368.1 | 30 | gagctccacgcagttcaacaagg | 116251 | - | | calmodulin binding | |
| 13431 | CNN3 | NM_001839.1 | 1049 | tagcgaccaaggcattgattatt | 116279 | - | | troponin C binding | |
| 13432 | CORO1A | NM_007074.1 | 967 | cagctcaatccggtactttgaga | 116285 | - | | structural molecule | |
| 13433 | CORO1B | NM_020441.1 | 517 | ctgcgacaacgtggtactcatct | 116287 | - | | | |
| 13434 | CORO1C | NM_014325.2 | 294 | tggtcgaattgacaaatcttacc | 116297 | - | see also 5620 line | | |
| 13435 | CORO2A | NM_003389.2 | 555 | gagatcgcctcctgttctgaaga | 116328 | - | | | |
| 13436 | CORO2B | NM_006091.1 | 1266 | ttgtggtcaacggaatagattta | 116351 | - | | membrane associated actin binding | |
| 13437 | COTL1 | NM_021149.2 | 450 | gtgaaggaggtcgtacagaattt | 116357 | - | | | |
| 13438 | DAAM1 | NM_014992.1 | 2415 | atgagccgaattaatcactatca | 116416 | - | | | |
| 13439 | DAAM2 | NM_015345.2 | 522 | gagtctgacgctttgatgagg | 116443 | - | | | |
| 13440 | DBN1 | NM_004395.2 | 1926 | gagggtacttcagtcaatcaca | 116491 | - | | | |
| 13441 | DIAPH1 | NM_005219.2 | 885 | cagccgctgctgatggattaaa | 116504 | - | | receptor binding | Konigsmark syndrome, deafness |
| 13442 | DIAPH2 | NM_006729.2 | 2625 | aagatactcaacgaattagcaga | 116629 | - | see also 4745 line | | |
| 13443 | DMD | NM_000109.2 | 9776 | ttgtttgaccactatttatgacc | 116884 | - | see also 70 line | | |
| 13444 | DNASE1 | NM_005223.2 | 1341 | gggacggaacagcctataaggagc | 116921 | - | apoptosis | deoxyribonuclease I | systemic lupus erythematosis, arthritis |
| 13445 | ENC1 | NM_003633.1 | 1025 | ttgtgtacgagtctgcaattaac | 116939 | - | see also 2451 line | | |
| 13446 | EPB41 | NM_004437.1 | 938 | gggataaacgcaacctctaagaca | 116969 | - | | | hereditary elliptocytosis |
| 13447 | EPB41L1 | NM_012156.2 | 1265 | aggtctgcatcgagcatcataca | 117022 | - | see also 5152 line | | |
| 13448 | EPB41L2 | NM_001431.1 | 1503 | gtgtgcgtggagcatcatactt | 117062 | - | see also 1000 line | | |
| 13449 | EPB41L3 | NM_012307.2 | 1057 | gcgaccggctgcgaataaacaga | 117121 | - | see also 5269 line | | |
| 13450 | FHOD1 | NM_013241.1 | 3413 | gcgcaaccgcaagtctttgaga | 117185 | - | | actin binding | |
| 13451 | FLII | NM_002018.2 | 2388 | ctgttggtccgacgtttcatct | 117202 | - | | actin binding | |
| 13452 | FLNB | NM_001457.1 | 6866 | tgggtcgtgcggtgatcttata | 117329 | - | signal transduction | | Alzheimer disease |
| 13453 | FMNL1 | NM_005892.3 | 896 | gtgcctacgcgccatcatgaact | 117345 | - | | | |

Figure 46

| 13454 | FXYD5 | NM_014164.3 | 216 | aagataccacgtccagttcttca | 117367 | - | channel | ion channel | - |
|---|---|---|---|---|---|---|---|---|---|
| 13455 | GC | NM_000583.2 | 1127 | atgtagagttgcccacaaacaaa | 117393 | - | - | vitamin D binding | diabetes mellitus、diabetes |
| 13456 | GMFB | NM_004124.2 | 288 | acgacaacctcgcttcattgtgt | 117414 | - | - | protein kinase inhibitor | - |
| 13457 | GMFG | NM_004877.1 | 286 | gggctgcaagccggaacaacaga | 117426 | - | kinase_related | protein kinase inhibitor | - |
| 13458 | HDAC6 | NM_006044.2 | 2403 | cagtggccgcattatccttatcc | 117467 | - | transcription_regulator、cell_cycle | transcriptional repressor | - |
| 13459 | HIP1 | NM_005338.4 | 946 | cagccctgtcagaacatatcagc | 117493 | - | see also 3727 line | | |
| 13460 | IPP | NM_005897.1 | 1764 | atgatcaccagtcgttgtgaagg | 117512 | - | - | actin binding | - |
| 13461 | IQGAP2 | NM_006633.1 | 4237 | atggtgagccgtgcaatgataga | 117641 | - | signal_transduction | calmodulin binding | - |
| 13462 | KIAA0843 | NM_014945.1 | 1057 | ctgcgctaaagtggataatgaga | 117669 | - | see also 6134 line | | |
| 13463 | KLHL1 | NM_020866.1 | 2614 | ctgttcccggctactggattatg | 117729 | - | see also 8009 line | | |
| 13464 | KLHL2 | NM_007246.2 | 1910 | ttggcgtcagtagaatattataa | 117793 | - | see also 5045 line | | |
| 13465 | KLHL3 | NM_017415.1 | 1641 | aagagcgttgaggtttacgatcc | 117821 | - | - | structural molecule | - |
| 13466 | KLHL4 | NM_019117.3 | 1727 | gggacgacagtggaattacgtag | 117859 | - | see also 7711 line | | |
| 13467 | KPTN | NM_007059.1 | 275 | ttgtctccatcgacactttcaac | 117888 | - | see also 4942 line | | |
| 13468 | LCP1 | NM_002298.2 | 1798 | aggacccgaagattagtacaagt | 117932 | - | see also 1474 line | | |
| 13469 | LSP1 | NM_002339.1 | 1076 | ccgggcatgggaagtatgagaag | 117941 | - | signal_transduction | signal transducer | - |
| 13470 | MEFV | NM_000243.1 | 1680 | tggaccactcctcaagagataaa | 117950 | - | - | zinc binding | Mediterranean fever |
| 13471 | MGC35136 | NM_152427.1 | 613 | ctgccgctacgccccttatgacg | 117967 | - | - | - | - |
| 13472 | MTSS1 | NM_014751.2 | 752 | tcgagcgctttaattgattgtct | 117973 | - | cell_cycle、signal_transduction、kinase_related | - | - |
| 13473 | MYBPC1 | NM_002465.1 | 501 | cagatgcgaggtcacctataagg | 118011 | - | see also 1581 line | | |
| 13474 | MYBPC2 | NM_004533.1 | 1266 | ggggtcgctatcaggtcataacc | 118108 | - | - | structural constituent of muscle | - |
| 13475 | MYBPC3 | NM_000256.2 | 3163 | aggtgacggtgcgcattgagaac | 118140 | - | - | structural constituent of muscle | cardiomyopathy |
| 13476 | MYRIP | NM_015460.1 | 1799 | caggtgtcggatgatttatcaga | 118165 | - | - | - | - |
| 13477 | NCALD | NM_032041.1 | 449 | tggacggaaatggctatatcagc | 118188 | - | see also 9066 line | | |
| 13478 | NEB | NM_004543.2 | 16552 | ttgccgactctccgatcaatagg | 118516 | - | - | structural constituent of muscle | nemaline myopathy |
| 13479 | NEBL | NM_006393.1 | 2571 | gagctaccactttaagtgtaact | 118673 | - | - | structural constituent of muscle | - |
| 13480 | PARVA | NM_018222.2 | 687 | cagtatttccgcgcaccaattcg | 118695 | - | - | actin binding | - |
| 13481 | PARVB | NM_013327.2 | 513 | gtggagcgtggactcaattcacg | 118707 | - | - | actin binding | - |

Figure 46

| | | | | | | | | | | |
|---|---|---|---|---|---|---|---|---|---|---|
| 13482 | PARVG | NM_022141.3 | 988 | aaggacgtcttgatgaattatt | 118719 | - | - | - | actin binding | - |
| 13483 | PIP | NM_002652.1 | 272 | aaggtgcatttaactaataagtat | 118754 | - | - | - | actin binding | - |
| 13484 | PLEC1 | NM_000445.1 | 12442 | ctgatggagcgttgtatcactga | 118795 | - | - | - | structural constituent of muscle | muscular dystrophy |
| 13485 | PLS3 | NM_005032.3 | 1034 | aaggtgaaccacggatagatatt | 118814 | - | see also 3513 line | - | - | - |
| 13486 | RDX | NM_002906.2 | 1697 | aagcaggccgtgataagtacaag | 118872 | - | - | - | structural constituent of cytoskeleton | - |
| 13487 | SCIN | NM_033128.1 | 1597 | aggacaccaattgtcatcataaa | 118898 | - | - | - | structural constituent of cytoskeleton | - |
| 13488 | SEC24C | NM_004922.2 | 2636 | gcgtcggctccgcatccataatc | 118941 | - | see also 3422 line | - | - | - |
| 13489 | SMTN | NM_006932.3 | 513 | ttgcgaagccgctggtgagtatga | 118957 | - | - | - | - | - |
| 13490 | SNTA1 | NM_003098.2 | 781 | aggacgtcaccgtatttcaag | 118973 | - | - | - | - | - |
| 13491 | SNTB1 | NM_021021.2 | 1648 | ccgtttgacatacattatgaga | 118991 | - | - | - | - | - |
| 13492 | SNTB2 | NM_067750.2 | 1568 | ttgtccgaagccgattgtattg | 119026 | - | see also 4765 line | - | - | - |
| 13493 | SNTG1 | NM_018967.1 | 747 | cagtaggaggatttgdattaagc | 119035 | - | see also 7613 line | - | - | - |
| 13494 | SNTG2 | NM_018968.1 | 498 | caggttaatgcatacatgtaga | 119075 | - | - | - | - | - |
| 13495 | SORBS1 | NM_006434.1 | 1896 | tagctatataccaacagaatgatg | 119110 | - | signal transduction | - | - | - |
| 13496 | SPTAN1 | NM_003127.1 | 4914 | ccggatccgtggggttatcgaca | 119200 | - | - | - | structural constituent of cytoskeleton | - |
| 13497 | SPTB | NM_000347.3 | 5791 | tagtggacacggcggataaattc | 119253 | - | see also 289 line | - | - | - |
| 13498 | SPTBN1 | NM_003128.1 | 2622 | acgtgggccacgatgagtattcc | 119289 | - | see also 2101 line | - | - | - |
| 13499 | SPTBN2 | NM_006946.1 | 6918 | gggcagcgtcgcctttgattacc | 119394 | - | - | - | structural constituent of cytoskeleton | - |
| 13500 | SPTBN4 | NM_025213.1 | 1665 | cagccgaaggctactacgatatc | 380328 | - | - | - | structural constituent of cytoskeleton | - |
| 13501 | SPTBN5 | NM_016642.1 | 1022 | cagatgagcgctctatcatgaac | 119400 | - | - | - | structural constituent of cytoskeleton | - |
| 13502 | SVIL | NM_003174.2 | 829 | gaggccgactccgtatttatc | 119433 | - | see also 2155 line | - | - | - |
| 13503 | TAGLN | NM_003186.2 | 654 | cggacgacctggcagatcatca | 119495 | - | - | - | actin binding | atherosclerosis |
| 13504 | TLN1 | NM_006289.2 | 1265 | ttgccggctacatcgatatcatc | 119505 | - | see also 4379 line | - | - | - |
| 13505 | TLN2 | NM_015059.1 | 3140 | gtggtccgatggaaatcgattca | 119604 | - | - | - | - | - |
| 13506 | TMOD1 | NM_003275.1 | 35 | atgtcgtacagacgagaactaga | 119653 | - | - | - | tropomyosin binding | - |
| 13507 | TMOD2 | NM_014548.2 | 364 | ccgcgagcacctcctcatgtacc | 119679 | - | see also 5750 line | - | - | - |
| 13508 | TMOD3 | NM_014547.3 | 1243 | gtgcgtaagagacgagttgaagg | 119719 | - | - | - | - | - |
| 13509 | TMOD4 | NM_013353.1 | 1055 | caggccatgaccgaaacaatga | 119731 | - | see also 5406 line | - | - | - |
| 13510 | TMSNB | NM_021992.1 | 209 | gagaaagagtgtgttcaaacatc | 119736 | - | - | - | molecular_function unknown | - |
| 13511 | TNNI1 | NM_003281.2 | 378 | tggacctccgtgggaagttcaag | 119737 | - | - | - | tropomyosin binding | - |

## Figure 46

| | | | | | | | | | |
|---|---|---|---|---|---|---|---|---|---|
| 13512 | TNNI3 | NM_000363.3 | 487 | ttgaccttcgaggcaagtttaag | 119744 | - | - | actin binding | hypertrophic cardiomyopathy |
| 13513 | TPM1 | NM_000366.4 | 962 | tggaccacgctctcaacgatatg | 119759 | - | - | structural constituent of muscle | cardiomyopathy |
| 13514 | UTRN | NM_007124.1 | 2680 | gaggatcgtcaacaacatctaga | 119821 | - | - | structural molecule | - |
| 13515 | VASP | NM_003370.1 | 451 | tcgtccggggtgtcaagtataac | 120006 | - | - | actin binding | - |
| 13516 | VCL | NM_003373.2 | 2028 | ctgcggttggtactgctaataaa | 120050 | - | - | - | - |
| 13517 | VILL | NM_015873.1 | 569 | gagggataaatcgattcatgtaa | 120086 | - | - | structural constituent of cytoskeleton | - |
| 13518 | WASL | NM_003941.2 | 740 | ttgggccgtcgacaaaggaaatc | 120111 | - | transcription_regulator | small GTPase regulatory/interacting protein | - |
| 13519 | WASPIP | NM_003387.3 | 785 | ttgggaggaggctcaatacgtca | 120136 | - | see also 2286 line | | |
| 13520 | WDR1 | NM_005112.3 | 1091 | ctgtccgggtacatcaactatct | 120159 | - | see also 3585 line | | |
| 13521 | DCX | NM_000555.2 | 467 | atgcgaggctcccggatgaatgg | 120188 | | - | - | subcortical laminar heterotopia and lissencephaly syndrome(-) |
| 13522 | HOOK3 | NM_032410.2 | 2078 | aagtgtcatccgtactttagatc | 120257 | - | - | - | - |
| 13523 | MAPRE1 | NM_012325.1 | 66 | tggcagtgaacgtatactcaacg | 120265 | - | cell_cycle | protein C-terminus binding | - |
| 13524 | MAPRE2 | NM_014268.1 | 363 | gagcggcctattgccaattcatg | 120297 | - | see also 5573 line | | |
| 13525 | TUBGCP5 | NM_052903.1 | 2369 | aagatagttcacgtctatctata | 120369 | - | - | microtubule binding | - |
| 13526 | TUBGCP6 | NM_020461.2 | 1053 | aagggacgctttcgacaagttct | 120406 | - | - | - | - |
| 13527 | UNC84B | NM_015374.1 | 2223 | aggacggcgagcctattcagacg | 120446 | - | see also 6401 line | | |
| 13528 | GABARAP | NM_007278.1 | 291 | ttgatccggaagcgaattcatct | 120455 | - | - | - | - |
| 13529 | GAPCENA | NM_012197.2 | 1916 | gtgctatcacccgggatattaac | 120516 | - | see also 5186 line | | |
| 13530 | LMOD1 | NM_012134.1 | 1126 | ctgaggtgaacgtcaacaactca | 120554 | - | - | tropomyosin binding | - |
| 13531 | TNNT1 | NM_003283.3 | 292 | cagacactcatcgatgtacattt | 120560 | - | - | tropomyosin binding | - |
| 13532 | TNNT3 | NM_006757.1 | 555 | gagacgcaagccgctcaacatcg | 120563 | - | - | tropomyosin binding | - |
| 13533 | KIFAP3 | NM_014970.2 | 840 | gtgtcgagttagctacaaacata | 120579 | - | see also 6160 line | | |
| 13534 | MAPK8IP1 | NM_005456.2 | 1778 | gggtgtctttcctgcctattacg | 120655 | - | kinase_related | protein kinase inhibitor | diabetes mellitus、diabetes |
| 13535 | MAPK8IP2 | NM_012324.2 | 2037 | ctggttccgtggcttcaacatgc | 120661 | - | see also 5275 line | | |
| 13536 | MAPK8IP3 | NM_015133.1 | 973 | gagatgcgcaacgtcagtatagg | 120669 | - | kinase_related | - | - |
| 13537 | TRIM2 | NM_015271.2 | 1444 | gcgtgaagaggcgcgttaagtcc | 120705 | - | see also 6301 line | | |
| 13538 | TNKS1BP1 | NM_033396.1 | 5352 | ccgcggtccagcgttcgaaatcc | 120753 | - | - | - | - |
| 13539 | ARGBP2 | NM_003603.2 | 867 | gtgagtcccatgagttactatca | 120762 | - | - | - | - |

Figure 46

| | | | | | | | | | | |
|---|---|---|---|---|---|---|---|---|---|---|
| 13540 | BAIAP2 | NM_006340.1 | 543 | cagcaagaatcctcagaagtact | 120844 | - | see also 4436 line | | | |
| 13541 | CDC42EP3 | NM_006449.3 | 1332 | aggatcccaagctctcatgttgc | 120850 | - | see also 4523 line | | | |
| 13542 | AF3P21 | NM_016453.2 | 1286 | cagcgcagttgggcactatatga | 120863 | - | signal_transduction | - | | - |
| 13543 | DLG1 | NM_004087.1 | 1086 | gggtctattgtacgcttgtatgt | 120895 | - | see also 2754 line | | | |
| 13544 | JUP | NM_002230.1 | 2169 | cagagcatgattcccatcaatga | 120949 | - | - | | - | Naxos disease |
| 13545 | MYLIP | NM_013262.3 | 1024 | gggctctaccgagcgataacaga | 120974 | - | - | | - | - |
| 13546 | PKD2 | NM_000297.2 | 2784 | acggctagtacgtgaagagttgg | 121032 | - | see also 262 line | | | |
| 13547 | RPH3AL | NM_006987.2 | 757 | agggctcccaagtatatcttgc | 121042 | - | - | | - | - |
| 13548 | SDC1 | NM_002997.3 | 886 | ttgaaacctcggggggagaatacg | 121046 | - | - | | cytoskeletal protein binding | multiple myeloma |
| 13549 | SDC2 | NM_002998.2 | 861 | tggaaaccacgacgctgaatata | 121055 | - | - | | molecular_function unknown | - |
| 13550 | SDC3 | NM_014654.1 | 1618 | atgcgcttcagcccagatgtagc | 121068 | - | - | | cytoskeletal protein binding | |
| 13551 | SSH3BP1 | NM_005470.1 | 129 | gagggcgctgatcgagagttacc | 121077 | - | - | | - | - |
| 13552 | PPP3R1 | NM_000945.2 | 549 | tagtacagcgagtaatagatata | 121127 | - | see also 625 line | | | |
| 13553 | ADCY1 | NM_021116.1 | 1051 | ctgtcgccgcatcaagattctcg | 121139 | - | - | | - | - |
| 13554 | ADD3 | NM_016824.1 | 1729 | gaggacaatcgaacgtaaacaac | 121234 | - | see also 6969 line | | | |
| 13555 | AKAP5 | NM_004857.2 | 2025 | aagcaagcccacttgaaacttca | 121257 | - | kinase_related、signal_transduction | protein transporter | - | |
| 13556 | GAP43 | NM_002045.2 | 504 | cagaaagtgccactaaagcttcc | 121274 | - | kinase_related | calmodulin binding | | |
| 13557 | IQGAP1 | NM_003870.2 | 2335 | tcgctgccgtggatacttagttc | 121324 | - | see also 2627 line | | | |
| 13558 | ITPKA | NM_002220.1 | 804 | atgggcgtcaggacttacctaga | 121392 | - | kinase_related、signal_transduction | calmodulin binding | - | |
| 13559 | ITPKB | NM_002221.2 | 916 | gggctgttcggagctagcttaac | 121405 | - | kinase_related、signal_transduction | calmodulin binding | - | |
| 13560 | KCNH1 | NM_002238.2 | 237 | cggcggtccaatgatactaattt | 121423 | - | channel、signal_transduction | - | - | |
| 13561 | KCNH5 | NM_139318.3 | 1240 | gggactccatatcgctacaatac | 121537 | - | see also 9688 line | | | |
| 13562 | KCNN1 | NM_002248.3 | 1695 | ccgtcatgtacgaccttgtatcg | 121578 | - | channel | - | - | |
| 13563 | KCNN2 | NM_021614.2 | 1497 | gagcgatgtggttgatatcaata | 121600 | - | channel | - | - | |
| 13564 | KCNN3 | NM_002249.3 | 1791 | caggacgtaactagtaactttct | 121624 | - | see also 1447 line | | | |
| 13565 | KCNN4 | NM_002250.2 | 936 | cagcatcggcgctctcaatcaag | 121639 | - | channel | small conductance calcium-activated potassium channel | - | |
| 13566 | NOS1 | NM_000620.1 | 4910 | gtgaccaaccgccttagatctga | 121822 | - | see also 417 line | | | |
| 13567 | NOS2A | NM_000625.3 | 1502 | aggctgtcgttgagatcaacatt | 121838 | - | see also 419 line | | | |
| 13568 | NRGN | NM_006176.1 | 267 | aagccggacgacgacattctaga | 121853 | - | signal_transduction | calmodulin binding | - | |

Figure 46

| | | | | | | | | | |
|---|---|---|---|---|---|---|---|---|---|
| 13569 | PDE1A | NM_005019.2 | 338 | atgttaacgtcgtcgacttaaag | 121861 | - | signal_transduction | calmodulin-dependent cyclic-nucleotide phosphodiesterase | - |
| 13570 | PDE1B | NM_000924.2 | 362 | ctgctggaagccgtctacataga | 121900 | - | apoptosis、signal_transduction | calmodulin-dependent cyclic-nucleotide phosphodiesterase | - |
| 13571 | PHKA1 | NM_002637.1 | 2904 | atgtttcgacttcgaattggtct | 121967 | - | see also 1708 line | | |
| 13572 | PHKA2 | NM_000292.1 | 1513 | cagagtatcgcggacattcatcc | 122026 | - | see also 257 line | | |
| 13573 | PHKB | NM_000293.1 | 730 | tcgagctcggttggtttagcaaa | 122102 | - | see also 261 line | | |
| 13574 | PPP3CA | NM_000944.2 | 1826 | aagggcttagaccgaattaatga | 122224 | - | phosphatase | protein phosphatase type 2C | - |
| 13575 | PPP3CB | NM_021132.1 | 1563 | aagggtttggataggatcaatga | 122268 | - | phosphatase、signal_transduction、cell_cycle、transcription_regulator | protein phosphatase type 2C | - |
| 13576 | PPP3CC | NM_005605.3 | 1070 | ctgtccgagggtgctcttatttc | 122286 | - | phosphatase | protein phosphatase type 2C | schizophrenia |
| 13577 | RGS1 | NM_002922.2 | 231 | aagtaatgcaatggtctcaatct | 122316 | - | signal_transduction、gpcr | calmodulin binding | - |
| 13578 | RGS16 | NM_002928.2 | 270 | aagttcgagtggggcagtaaaca | 122328 | - | signal_transduction、gpcr | calmodulin binding | - |
| 13579 | RGS2 | NM_002923.1 | 291 | ctgctagccagcaaatatggtct | 122336 | - | cell_cycle、signal_transduction、gpcr | calmodulin binding | - |
| 13580 | RGS4 | NM_005613.3 | 345 | cagctttcaaagctttcttgaag | 122347 | - | signal_transduction、gpcr | calmodulin binding | - |
| 13581 | RYR2 | NM_001035.1 | 12854 | ctgtttgcgctcaggtacaatat | 122643 | - | see also 634 line | | |
| 13582 | SLC8A1 | NM_021097.1 | 90 | atgcggcgattaagtctttcacc | 122717 | - | see also 8084 line | | |
| 13583 | STRN | NM_003162.1 | 1778 | atggcactctgcgtttatggaat | 122862 | - | see also 2135 line | | |
| 13584 | STRN3 | NM_014574.2 | 1182 | atgggctgaaccaataacgtttc | 122909 | - | see also 5757 line | | |
| 13585 | STRN4 | NM_013403.1 | 1836 | tggcgacaccgtcttgtatgaca | 122975 | - | see also 5431 line | | |
| 13586 | IL18R1 | NM_003855.2 | 789 | tggatcggatcctaatatacatg | 122997 | - | receptor_acitivity、signal_transduction | - | - |
| 13587 | IL18RAP | NM_003853.2 | 948 | gtgagtattccgcatcacataag | 123032 | - | receptor_acitivity、signal_transduction | - | - |

Figure 46

| | | | | | | | | | |
|---|---|---|---|---|---|---|---|---|---|
| 13588 | IL1R1 | NM_000877.2 | 1229 | aagacctatgacgcatatatact | 123118 | - | receptor_acitivity、sig nal_transduction | interleukin-1、Type I、activating receptor | diabetes |
| 13589 | IL1RAP | NM_002182.2 | 715 | gtgtcaaaccgactatcacttgg | 123159 | - | see also 1392 line | | |
| 13590 | IL1RAPL1 | NM_014271.2 | 767 | gagagcctgttcgaatcaaatgt | 123208 | - | receptor_acitivity、sig nal_transduction | - | mental retardation |
| 13591 | IL1RAPL2 | NM_017416.1 | 1727 | ctgacctggcgaattatacctgc | 123300 | - | see also 6987 line | | |
| 13592 | IL1RL1 | NM_003856.2 | 1167 | aggcacaccgtaagactaagtag | 123369 | - | receptor_acitivity、sig nal_transduction | - | - |
| 13593 | IL1RL2 | NM_003854.2 | 1270 | ctgtatgacgcctatgtcttata | 123454 | - | receptor_acitivity | - | - |
| 13594 | MYD88 | NM_002468.2 | 789 | gcgactgatccccatcaagtaca | 123479 | - | receptor_acitivity、sig nal_transduction、kina se_related | interleukin-1、Type I、activating receptor | atherosclerosis |
| 13595 | SIGIRR | NM_021805.1 | 485 | gtgaccagcactgaagtctatgg | 123480 | - | - | - | - |
| 13596 | TLR1 | NM_003263.2 | 1078 | ttggcatacaactgtatggtatt | 123518 | - | see also 2214 line | | |
| 13597 | TLR10 | NM_030956.1 | 2378 | atgtccgattccacgcatttatt | 123611 | - | receptor_acitivity | interleukin-1、Type I、activating receptor | - |
| 13598 | TLR2 | NM_003264.2 | 1570 | ttgaatttgccgcaactcaaaga | 123668 | - | receptor_acitivity、sig nal_transduction | interleukin-1、Type I、activating receptor | rheumatoid arthritis |
| 13599 | TLR6 | NM_006068.2 | 563 | ttgctcacttgcatctaagttat | 123702 | - | receptor_acitivity、sig nal_transduction、kina se_related | lipoprotein binding | - |
| 13600 | IL1R2 | NM_004633.3 | 235 | gcgcttgtacgtgttggtaatgg | 123743 | - | receptor_acitivity | - | - |
| 13601 | IL2RA | NM_000417.1 | 336 | aagcgggtcactctatatgctct | 123773 | - | receptor_acitivity、apo ptosis、cell_cycle、sig nal_transduction | Rel A homodimer | - |
| 13602 | IL2RB | NM_000878.2 | 561 | gagacccacagatgcaacataag | 123792 | - | receptor_acitivity、sig nal_transduction | interleukin-2 receptor | rheumatoid arthritis |
| 13603 | IL2RG | NM_000206.1 | 75 | gtggggctgaacacgacaattct | 123809 | - | receptor_acitivity、sig nal_transduction | interleukin-7 receptor | rheumatoid arthritis、agammaglobulinemia、thymic epithelial hypoplasia、immunodeficiency、severe combined immunodeficiency disease |
| 13604 | IL10RA | NM_001558.2 | 697 | ctgtcgcttcccgaagtaacaag | 123845 | - | receptor_acitivity | interleukin-10 receptor | - |
| 13605 | IL10RB | NM_000628.3 | 552 | atggacttataatgtgcaatact | 123870 | - | receptor_acitivity、sig nal_transduction | interleukin-10 receptor | - |

EP 1 752 536 A1

557

Figure 46

| | | | | | | | | | |
|---|---|---|---|---|---|---|---|---|---|
| 13606 | IL8RA | NM_000634.2 | 566 | gcgtcacttggtcaagtttgttt | 123887 | - | receptor_acitivity、gpc r、signal_transduction | interleukin-8 receptor | - |
| 13607 | IL8RB | NM_001557.2 | 461 | aagatcttagtaattacagttac | 123901 | - | gpcr、receptor_acitivity | interleukin-8 receptor | - |
| 13608 | IL6R | NM_000565.2 | 1210 | gggctgaacggtcaaagacattc | 123958 | - | receptor_acitivity、signal_transduction | - | - |
| 13609 | IL6ST | NM_002184.2 | 1682 | ccgtgcatcgcacctatttaaga | 124022 | - | see also 1395 line | | |
| 13610 | CSF2RB | NM_000395.1 | 2554 | gggacccggtcctgagatcaaga | 124073 | - | receptor_acitivity、signal_transduction | interleukin-5 receptor | pulmonary alveolar proteinosis |
| 13611 | IL5RA | NM_000564.2 | 1588 | gtggagccaacctatttatgtgg | 124102 | - | receptor_acitivity、signal_transduction | - | - |
| 13612 | IL4R | NM_000418.1 | 991 | ccgcctcgtggctataataatcc | 124120 | - | receptor_acitivity、signal_transduction | receptor signaling protein | diabetes |
| 13613 | IL3RA | NM_002183.2 | 481 | cagaaatgtgaccgatatcgagt | 124129 | - | receptor_acitivity | - | - |
| 13614 | IL9R | NM_002186.2 | 883 | tggtagaggaggagcgttataca | 124151 | - | receptor_acitivity、signal_transduction | - | - |
| 13615 | IL21R | NM_021798.2 | 771 | gagtgacccggtcatctttcaga | 124158 | - | receptor_acitivity | - | - |
| 13616 | IL17R | NM_014339.3 | 506 | ttgtgcgtcaggtttgagtttct | 124167 | - | receptor_acitivity、signal_transduction | interleukin-17 receptor | - |
| 13617 | IL12RB2 | NM_001559.2 | 1859 | ctgcccactcgtattaacataat | 124208 | - | receptor_acitivity、signal_transduction | interleukin receptor | - |
| 13618 | IL13RA2 | NM_000640.2 | 142 | tggctatcggatgcttatatacc | 124230 | - | receptor_acitivity | high molecular weight B-cell growth factor receptor ligand | - |
| 13619 | IL15RA | NM_002189.2 | 245 | gagcggtacatttgtaactctgg | 124283 | - | receptor_acitivity、signal_transduction | - | - |
| 13620 | IL17RB | NM_018725.2 | 958 | tggtggcagggatctatctaatg | 124321 | - | see also 7582 line | | |
| 13621 | IL18BP | NM_005699.2 | 1038 | ctgccactgcctcagttagaagc | 124335 | - | - | - | - |
| 13622 | CCR5 | NM_000579.1 | 1352 | ccgagcgagcaagctcagtttac | 124350 | - | receptor_acitivity、gpc r、signal_transduction | coreceptor | resistance to or association with human immunodeficiency virus |
| 13623 | CCR6 | NM_004367.3 | 1346 | cagtgagaccgcagataacgaca | 124376 | - | receptor_acitivity、gpc r、signal_transduction | - | - |
| 13624 | CCR8 | NM_005201.2 | 1158 | ccgttcctccagcgtagactaca | 124398 | - | receptor_acitivity、gpc r、signal_transduction | coreceptor | - |

Figure 46

| | | | | | | | | | |
|---|---|---|---|---|---|---|---|---|---|
| 13625 | CCR9 | NM_006641.2 | 150 | tggaagactacgttaacttcaac | 124402 | - | see also 4695 line | | |
| 13626 | CCRL1 | NM_016557.2 | 732 | ggggtgtgctactttatcacagc | 124443 | - | receptor_acitivity、gpcr、signal_transduction | - | - |
| 13627 | GPR2 | NM_016602.1 | 568 | acgacgctgtcgcctcatcttcc | 124454 | - | receptor_acitivity、gpcr、signal_transduction | C-C chemokine receptor | Peutz-Jeghers syndrome |
| 13628 | CXCR3 | NM_001504.1 | 613 | tcgccctcccagacttcatcttc | 124460 | - | receptor_acitivity、gpcr、signal_transduction | C-X-C chemokine receptor | - |
| 13629 | CCBP2 | NM_001296.3 | 948 | gtgctatggttcccatacaatct | 124473 | - | receptor_acitivity、gpcr、signal_transduction | C-C chemokine receptor | - |
| 13630 | CCL13 | NM_005408.2 | 350 | aagctcacaccctgaagacttga | 124478 | - | receptor_acitivity、signal_transduction | chemokine receptor | - |
| 13631 | CCR1 | NM_001295.1 | 816 | tggaccccctacaatttgactat | 124499 | - | receptor_acitivity、gpcr | C-C chemokine receptor | - |
| 13632 | CCR2 | NM_000647.3 | 264 | ctggtcgtcctcatcttaataaa | 124510 | - | receptor_acitivity、gpcr、signal_transduction | - | rheumatoid arthritis、atherosclerosis |
| 13633 | CCR3 | NM_001837.2 | 995 | cagaggatacagtatatagctgg | 124526 | - | receptor_acitivity、gpcr、signal_transduction | - | - |
| 13634 | CCR4 | NM_005508.2 | 54 | atgaacccacggatatagcaga | 124534 | - | see also 3848 line | | |
| 13635 | CCR7 | NM_001838.2 | 296 | tggtcgtgttgacctatatctat | 124556 | - | receptor_acitivity、gpcr、signal_transduction | C-C chemokine receptor | Peutz-Jeghers syndrome |
| 13636 | CMKLR1 | NM_004072.1 | 960 | cagcgttcgcctggcttacatgg | 124569 | - | receptor_acitivity、gpcr、signal_transduction | chemokine receptor | - |
| 13637 | CX3CR1 | NM_001337.2 | 916 | gtgactgagacggttgcatttag | 124587 | - | receptor_acitivity、gpcr、signal_transduction | coreceptor | atherosclerosis |
| 13638 | CXCR4 | NM_003467.1 | 90 | tggaggggatcagtatatacact | 124591 | - | - | coreceptor | Peutz-Jeghers syndrome、WHIM syndrome |
| 13639 | GPR17 | NM_005291.1 | 184 | caggtctgatcaccaacttctcc | 124608 | - | receptor_acitivity、gpcr、signal_transduction | purinergic nucleotide receptor、G-protein coupled | - |

Figure 46

| | | | | | | | | |
|---|---|---|---|---|---|---|---|---|
| 13640 | XCR1 | NM_005283.1 | 663 | ccgcacggtcaagctcatcttcg | - | receptor_acitivity, gpcr | chemokine receptor | - |
| 13641 | IFNGR1 | NM_000416.1 | 1463 | gagtccttgattggttatagacc | - | receptor_acitivity, signal_transduction | interferon-gamma receptor | atypical mycobacteriosis |
| 13642 | IFNGR2 | NM_005534.1 | 953 | cagcaggcttcccaatggatttc | - | receptor_acitivity, signal_transduction | interferon-gamma receptor | atypical mycobacteriosis |
| 13643 | IFNAR1 | NM_000629.1 | 418 | tggtatgaggttgactcatttac | - | receptor_acitivity, signal_transduction | interferon-alpha/beta receptor | - |
| 13644 | IFNAR2 | NM_000874.2 | 667 | gcgggaacaacgttgttcagt | - | receptor_acitivity, signal_transduction | interferon-alpha/beta receptor ligand | - |
| 13645 | IL22RA1 | NM_021258.2 | 1496 | aagggaccaacacagtaccctaaag | - | receptor_acitivity | - | - |
| 13646 | CRIM1 | NM_016441.1 | 1491 | tggtttcaaacgcgatcacaatg | - | receptor_acitivity | - | - |
| 13647 | CTGF | NM_001901.1 | 971 | atgaagacataccgagctaaatt | - | - | insulin-like growth factor binding | - |
| 13648 | CYR61 | NM_001554.3 | 690 | gggtctgttgacgaggatagtatc | - | - | insulin-like growth factor binding | - |
| 13649 | ESM1 | NM_007036.2 | 309 | ctgaggtcagccttcttaatgg | - | - | insulin-like growth factor binding | - |
| 13650 | IGFALS | NM_004970.1 | 1777 | ccgcgtacacctacaacacatc | - | - | insulin-like growth factor binding | - |
| 13651 | IGFBP1 | NM_000596.1 | 850 | tggtgcgtcgtcaccctggaatgg | - | signal_transduction | Hepatocyte nuclear factor 1 | alcoholism |
| 13652 | IGFBP3 | NM_000598.2 | 717 | tggtccctgccgtagagagaaatgg | - | - | insulin-like growth factor binding | breast cancer |
| 13653 | IGFBP4 | NM_001552.1 | 644 | agggtgaccaccccaacaacagc | - | signal_transduction | insulin-like growth factor binding | - |
| 13654 | IGFBP6 | NM_002178.1 | 385 | ctgttgcagagagaatcctaag | - | - | insulin-like growth factor binding | - |
| 13655 | IGFBP7 | NM_001553.1 | 759 | atgagtgccatgcatccaattcc | - | see also 1059 line | - | - |
| 13656 | NOV | NM_002514.2 | 380 | cggccggtagaggggagataactgt | - | - | insulin-like growth factor binding | - |
| 13657 | PRSS11 | NM_002775.2 | 590 | ctgccgttcatatcgaattg | - | see also 1812 line | - | - |
| 13658 | WISP1 | NM_003882.2 | 858 | tggacatccatacactcattaag | - | see also 2639 line | - | - |
| 13659 | WISP3 | NM_003880.2 | 858 | cagccttgcgcagcaatatatt | - | signal_transduction | - | - |
| 13660 | FIBP | NM_004214.4 | 148 | gggggaacacgacccttatcgacg | - | signal_transduction | amine oxidase (flavin-containing) | arthritis |
| 13661 | LTBP1 | NM_000627.1 | 4228 | ccgttgaataccgccttgaattt | - | see also 420 line | - | - |

560

Figure 46

EP 1 752 536 A1

| | | | | | | | | | |
|---|---|---|---|---|---|---|---|---|---|
| 13662 | AVPR1A | NM_000706.3 | 3175 | tggaaggactcgcctaaatcttc | 125137 | - | kinase_related、gpcr、receptor_acitivity、signal_transduction | vasopressin receptor | - |
| 13663 | AVPR1B | NM_000707.2 | 1404 | tggtccgtgtgggacaagaatgc | 125144 | - | kinase_related、gpcr、receptor_acitivity、signal_transduction | vasopressin receptor | - |
| 13664 | COPB2 | NM_004766.1 | 1858 | ctgtcatgcggagggactttagc | 125210 | - | see also 3276 line | | |
| 13665 | GNB2L1 | NM_006098.2 | 392 | gcgatttgtgggccataccaagg | 125243 | - | see also 4244 line | | |
| 13666 | LIM | NM_006457.1 | 970 | aagaatctgaagccgataataca | 125273 | - | see also 4529 line | | |
| 13667 | CDK5RAP3 | NM_025197.2 | 190 | acgatccgcgagaagatcaatgc | 125285 | - | kinase_related、cell_cycle | - | - |
| 13668 | CGI-121 | NM_016058.1 | 590 | ttggatgctatcatttgtagaat | 125298 | - | kinase_related | - | - |
| 13669 | NBEA | NM_015678.2 | 3778 | ccggtagcgtagacttaacttgt | 125392 | - | kinase_related | - | - |
| 13670 | PTPRR | NM_002849.2 | 1426 | gagggtccaacgtatctcttaca | 125587 | - | kinase_related、receptor_acitivity、phosphatase、signal_transduction | - | - |
| 13671 | DSCR1L1 | NM_005822.1 | 342 | cagacgtgtccgtataaacttca | 125616 | - | see also 4060 line | | |
| 13672 | MYOZ2 | NM_016599.2 | 811 | ctggcagacggtcctttaatagg | 125643 | - | see also 6939 line | | |
| 13673 | AKAP11 | NM_016248.2 | 2810 | gtggtgcatacgatagtaaatga | 125725 | - | kinase_related、phosphatase | - | - |
| 13674 | LILRB1 | NM_006669.2 | 1431 | atgacccatggcgtctaagatca | 125841 | - | receptor_acitivity | - | - |
| 13675 | PPP1R16B | NM_015568.2 | 1781 | atgggacctcggtatattacacg | 125857 | - | phosphatase、signal_transduction | - | - |
| 13676 | NAKAP95 | NM_014371.1 | 1344 | aggagtacgtcactaacaagacc | 125874 | - | see also 5634 line | | |
| 13677 | ARIH1 | NM_005744.2 | 1114 | tagagtgcaatcgactgttaaag | 125900 | - | see also 3984 line | | |
| 13678 | BIRC7 | NM_022161.2 | 438 | ttgcgtctggcctccttctatga | 125928 | - | apoptosis、kinase_related | - | melanoma |
| 13679 | CASP2 | NM_001224.3 | 137 | ctgttgttgagcgaattgttaga | 125934 | - | apoptosis | - | holoprosencephaly |
| 13680 | DNMT3L | NM_013369.2 | 744 | acgacgatgacgggtaccaatcc | 125965 | - | transcription_regulator | - | - |
| 13681 | HDAC10 | NM_032019.3 | 1599 | ctgacaacgccggatatcacatt | 125981 | - | transcription_regulator | - | - |
| 13682 | PREX1 | NM_020820.2 | 1051 | atgggacagcggattaccatagc | 125998 | - | - | - | - |
| 13683 | SERPINB12 | NM_080474.1 | 167 | aggtactacacttcaacgaattt | 126032 | - | - | trypsin inhibitor | - |

Figure 46

| | | | | | | | | | |
|---|---|---|---|---|---|---|---|---|---|
| 13684 | APBB1 | NM_001164.2 | 119 | ctgagccagtcggccattaatgc | 126065 | - | transcription_regulator 、cell_cycle、signal_tr ansduction | - | dementia Alzheimer type、Alzheimer disease |
| 13685 | BAIAP1 | NM_004742.1 | 771 | ctggaagtcggcacctatgaagg | 126091 | - | - | - | - |
| 13686 | CTBP1 | NM_001328.1 | 90 | gcgtccgacctccgatcatgaac | 126148 | - | - | protein C-terminus binding | |
| 13687 | DLG4 | NM_001365.1 | 1914 | gagaaccgaggcgaattgtgatc | 126169 | - | see also 927 line | | |
| 13688 | HSPB7 | NM_014424.3 | 946 | acggcgtcacccgcatacagaac | 126187 | - | - | protein C-terminus binding | |
| 13689 | ITGB1BP1 | NM_004763.2 | 214 | aagtagcgaaatcagtactaaga | 126193 | - | see also 3274 line | | |
| 13690 | ITGB3BP | NM_014288.3 | 528 | ttgacagctatgaattccttaaa | 126218 | - | apoptosis、signal_tran sduction、transcription _regulator | - | - |
| 13691 | MLLT4 | NM_005936.1 | 215 | tcgaaacgctcgcggagaaattt | 126222 | - | signal_transduction | protein C-terminus binding | leukemia |
| 13692 | PRKCABP | NM_012407.1 | 356 | ctgtccctgcctctatatcgtcc | 126302 | - | see also 5311 line | | |
| 13693 | SNX17 | NM_014748.2 | 896 | atgctcagacggtatcagatatt | 126329 | - | see also 5941 line | | |
| 13694 | TOPBP1 | NM_007027.2 | 1047 | atgcaagttgcgtaagtgaatca | 126366 | - | see also 4919 line | | |
| 13695 | TRIM3 | NM_006458.2 | 2294 | ttgtagtaacggacttccataac | 126472 | - | see also 4530 line | | |
| 13696 | ELMO1 | NM_014800.8 | 2074 | ctgccggtggcagatatcaaagc | 126523 | - | see also 5994 line | | |
| 13697 | LRRFIP2 | NM_006309.1 | 1066 | ggggacaccagcagcttaataga | 126563 | - | - | - | - |
| 13698 | KRT14 | NM_000526.3 | 521 | cagcggcctgctgagatcaaaga | 126586 | - | - | Transcription factor AP-2 alpha | epidermolysis bullosa simplex |
| 13699 | YWHAB | NM_003404.3 | 769 | cagcctacacacccaattcgtct | 126595 | - | - | - | - |
| 13700 | YWHAG | NM_012479.2 | 437 | ccgtgcgtaccgggagaagatag | 126601 | - | - | protein kinase C binding | - |
| 13701 | YWHAH | NM_003405.2 | 811 | tagctgagctggacacactaaac | 126606 | - | kinase_related、apopto sis、signal_transductio n | protein kinase C inhibitor | - |
| 13702 | FCGR1A | NM_000566.2 | 524 | tgggaaagcatcgctacacatca | 126616 | - | receptor_acitivity、sig nal_transduction | hematopoietic-associated factor 1A complex | familial deficiency of IgG receptor I |
| 13703 | FCGR2B | NM_004001.2 | 910 | ttggggctgagaacacaatcacc | 126629 | - | receptor_acitivity、sig nal_transduction | transmembrane receptor | - |
| 13704 | FCGR3A | NM_000569.4 | 500 | caggcctcgagctacttcattga | 126631 | - | receptor_acitivity | receptor | rheumatoid arthritis |
| 13705 | FCER1A | NM_002001.2 | 686 | aagctccgcgtgagaagtactgg | 126663 | - | receptor_acitivity | receptor signaling protein | atopy |

Figure 46

| | | | | | | | | | |
|---|---|---|---|---|---|---|---|---|---|
| 13706 | FCER1G | NM_004106.1 | 84 | gagagcctcagctgctgtatatc | 126668 | - | receptor_acitivity, signal_transduction | transmembrane receptor | - |
| 13707 | FCER2 | NM_002002.3 | 566 | gagaggaacgaagcttcagattt | 126676 | - | receptor_acitivity | receptor signaling protein | atopy |
| 13708 | LGALS3 | NM_002306.1 | 397 | gtgcctcgcatgctgataacaat | 126681 | - | - | galactose binding lectin | breast cancer |
| 13709 | MS4A2 | NM_000139.2 | 522 | tgggggaacgggaattaccatcc | 126706 | - | receptor_acitivity, channel, signal_transduction, gpcr | - | leukemia |
| 13710 | CD44 | NM_000610.2 | 233 | ctggcgcagatcgatttgaatat | 126722 | - | receptor_acitivity | hyaluronic acid binding | hepatocellular carcinoma, colorectal cancer, non-hodgkin lymphoma, breast cancer, neuroblastoma, Alzheimer disease, colorectal cancers, leukemia, osteosarcoma, gastric cancer, skin cancer, Hodgkin's disease |
| 13711 | FN1 | NM_002026.1 | 2091 | aagtggtcctgtcgaagtattta | 126806 | - | - | - | breast cancer, Ehlers-Danlos syndrome, melanoma |
| 13712 | GP6 | NM_016363.3 | 852 | tcggggctgtgatcctaataatc | 126907 | - | receptor_acitivity | - | - |
| 13713 | ITGA10 | NM_003637.3 | 1423 | tggggctcctcgatttagacatc | 126921 | - | receptor_acitivity, signal_transduction | magnesium binding | - |
| 13714 | ITGA11 | NM_012211.2 | 1890 | cagcatccacggcaattggacc | 126975 | - | see also 5198 line | - | - |
| 13715 | ITGA2 | NM_002203.2 | 850 | ggggcgacgaagtgctacgaaagt | 127013 | - | receptor_acitivity, signal_transduction | Proto-oncogene C-JUN | - |
| 13716 | NID2 | NM_007361.2 | 2718 | gtgccgagtggttatgagtttg | 127146 | - | see also 5092 line | - | - |
| 13717 | PCOLCE | NM_002593.2 | 1151 | ttgccgtgactgtcagtcttatt | 127183 | - | - | collagen binding | - |
| 13718 | SERPINH1 | NM_001235.2 | 943 | ccggacagagcctctacaactact | 127195 | - | - | serpin | - |
| 13719 | A2M | NM_000014.3 | 1966 | ctgcatcaatcgtcataatgtct | 127241 | - | see also 1 line | - | - |
| 13720 | CGI-100 | NM_016040.2 | 734 | gttggtgtcagccattcaagttta | 127288 | - | see also 6635 line | - | - |
| 13721 | CGI-109 | NM_181836.3 | 735 | aagccgagcagagaggatctaaata | 127294 | - | - | - | - |
| 13722 | HSGP25L2G | NM_017510.3 | 108 | aagcgcgctctactttcacatcg | 127300 | - | see also 7016 line | - | - |
| 13723 | IL1RL1LG | NM_006858.2 | 278 | aagcctcgagaccgaataccagg | 127303 | - | receptor_acitivity, signal_transduction | - | - |
| 13724 | MGC23911 | NM_144676.1 | 144 | tgggagctgatcgatatgactttg | 127307 | - | - | - | - |
| 13725 | P24B | NM_007364.1 | 376 | aagtcaagggcgtttatcagttt | 127332 | - | - | - | - |
| 13726 | RNP24 | NM_006815.2 | 73 | cggtctcggctatttcgttagc | 127342 | - | - | protein transporter | - |
| 13727 | TMP21 | NM_006827.4 | 538 | gtgataccaacgagtcaacaaac | 127358 | - | - | protein transporter | - |
| 13728 | HPS4 | NM_022081.4 | 1637 | atgcttgtctggccatgatctgg | 127376 | - | see also 8266 line | - | - |

Figure 46

| | | | | | | | | | |
|---|---|---|---|---|---|---|---|---|---|
| 13729 | INHBB | NM_002193.1 | 1176 | aggagcgcgttccgaaatcatc | 127392 | - | - | inhibin | - |
| 13730 | S100B | NM_006272.1 | 296 | tggcctttgtgccatggttact | 127407 | - | kinase_related, apoptosis | calcium ion binding | melanoma |
| 13731 | IPO7 | NM_006391.1 | 530 | cagtgcttggctaggaattc | 127420 | - | see also 4478 line | - | - |
| 13732 | IPO8 | NM_006390.1 | 2075 | ctgtctacggatcattgatcttg | 127559 | - | signal_transduction | - | - |
| 13733 | KPNB3 | NM_002271.3 | 751 | ccgtcgatcaggacgttatctgc | 127602 | - | see also 1466 line | - | - |
| 13734 | RANBP2 | NM_006267.3 | 9356 | tagggcggataactatggaatta | 127747 | - | see also 4359 line | - | - |
| 13735 | RANBP3 | NM_003624.1 | 879 | acgccaacgcaacgaactattt | 127763 | - | signal_transduction | - | - |
| 13736 | SNPH | NM_014723.2 | 1099 | ctgccagcaacacctatgagaag | 127779 | - | - | syntaxin-1 binding | - |
| 13737 | PLDN | NM_012388.2 | 292 | ggggagccgacgcctggttaag | 127780 | - | - | - | - |
| 13738 | STXBP2 | NM_006949.1 | 890 | tgagcttcgccacatgcatatc | 127800 | - | - | syntaxin-3 binding | - |
| 13739 | SNAPAP | NM_012437.3 | 287 | gcgacgcgttgtcttgttaaca | 127817 | - | - | - | - |
| 13740 | NARF | NM_012336.2 | 854 | gcgctgactgcgtgttaacatca | 127836 | - | see also 5297 line | - | - |
| 13741 | TMPO | NM_003276.1 | 1559 | ctggatccgaactgatgctct | 127871 | - | - | lamin/chromatin binding | - |
| 13742 | NLGN2 | NM_020795.2 | 639 | tggagggaccggaaacatgttc | 127884 | - | - | - | - |
| 13743 | TNFRSF10A | NM_003844.2 | 325 | gggtccacaagaccttcaagttt | 127894 | - | receptor_acitivity, apoptosis, signal_transduction, kinase_related | death receptor | - |
| 13744 | TNFRSF10B | NM_003842.3 | 1299 | gagacagtgcttcgatgactttg | 127916 | - | receptor_acitivity, signal_transduction, apoptosis, kinase_related | - | head and neck cancer |
| 13745 | DCTN6 | NM_006571.2 | 237 | tggcgaagggaacctaatagaag | 127919 | - | - | - | - |
| 13746 | GGA2 | NM_015044.2 | 706 | aggctgcaaaccgttaatcaag | 127947 | - | - | enzyme | - |
| 13747 | GGA3 | NM_014001.2 | 905 | tggacacgaccaacagtttgtcc | 127980 | - | - | - | - |
| 13748 | APC | NM_000038.2 | 1585 | aggctacgctatgcttcatgaaa | 128034 | - | see also 7 line | - | - |
| 13749 | APCL | NM_005883.1 | 4521 | ctgccacgcgaacatatgctgc | 128246 | - | signal_transduction, cell_cycle | - | - |
| 13750 | PKP1 | NM_000299.1 | 1509 | ccgccagcaccaggtaactact | 128269 | - | see also 263 line | - | - |
| 13751 | FRZB | NM_001463.2 | 780 | aagaccttccggaacaatta | 128277 | - | receptor_acitivity, signal_transduction | transmembrane receptor | - |
| 13752 | GFR | NM_012294.1 | 471 | atgttccgctaggaaacgtaaa | 128301 | - | see also 5262 line | - | - |
| 13753 | CSE1L | NM_001316.2 | 209 | ccgagccagctgagaaattc | 128323 | - | see also 869 line | - | - |
| 13754 | RBX1 | NM_014248.2 | 196 | caggcgtccgctacttcagaaga | 128424 | - | - | - | - |
| 13755 | AB026190 | NM_014458.3 | 553 | ttgcgtatacctcccagataaca | 128438 | - | see also 5686 line | - | - |

Figure 46

| | | | | | | | | | | |
|---|---|---|---|---|---|---|---|---|---|---|
| 13756 | ABTB1 | NM_032548.2 | 851 | ccgagctccgaggtgatctttgg | 128487 | - | see also 9242 line | | | |
| 13757 | ABTB2 | NM_145804.1 | 1201 | tgggtccggatggggttaacacc | 128505 | - | - | - | - | |
| 13758 | AKAP10 | NM_007202.2 | 271 | aagcttcaatatccgtacattcc | 128517 | - | see also 5002 line | | | |
| 13759 | AKAP9 | NM_005751.3 | 1459 | cagttcgaaactgatatagtaca | 128590 | - | signal_transduction | - | Alzheimer disease | |
| 13760 | AMFR | NM_001144.3 | 1840 | aagctcgcaaacgtttcttgaac | 128852 | - | receptor_acitivity、sig nal_transduction | | - | |
| 13761 | ANKFY1 | NM_016376.2 | 1177 | cagtgcaaacaactagatttaga | 128883 | - | - | - | - | |
| 13762 | ANXA11 | NM_001157.2 | 1686 | acgacatctcgggagatacttca | 128934 | - | - | - | - | |
| 13763 | APBA2 | NM_005503.2 | 1195 | atggacctgttgacaataacaac | 128939 | - | see also 3845 line | | | |
| 13764 | APBA3 | NM_004886.2 | 308 | cagtcggatggaacttgatgagt | 128952 | - | - | protein transporter | - | |
| 13765 | APBB3 | NM_006051.2 | 1245 | cagaagtacgaggcactgtatat | 128962 | - | - | - | Alzheimer disease | |
| 13766 | APP | NM_000484.1 | 1602 | tcggcctcgtcacgtgttcaata | 128989 | - | see also 377 line | | | |
| 13767 | APPL | NM_012096.1 | 1623 | atgaaactatgcgccaaatctta | 129069 | - | see also 5115 line | | | |
| 13768 | ARHGDIA | NM_004309.3 | 440 | ccgggttaaccgagagatagtgt | 129088 | - | see also 2884 line | | | |
| 13769 | ASE-1 | NM_012099.1 | 1095 | ttggggtcgccagaaatggatgt | 129092 | - | - | - | - | |
| 13770 | AVEN | NM_020371.1 | 404 | tgggatcgatatcaagatattga | 129101 | - | apoptosis | protein binding | - | |
| 13771 | BAG1 | NM_004323.2 | 697 | atggttgccgggtcatgttaatt | 129118 | - | see also 2894 line | | | |
| 13772 | BAG2 | NM_004282.2 | 426 | aagaactctcaccgttgaagtgt | 129125 | - | apoptosis | protein binding | - | |
| 13773 | BAG4 | NM_004874.2 | 681 | tggagcgtatggtccaacatacc | 129148 | - | see also 3387 line | | | |
| 13774 | BAG5 | NM_004873.1 | 574 | ctgcgtaactgatgagtttgaag | 129187 | - | apoptosis | protein binding | - | |
| 13775 | BAI1 | NM_001702.1 | 1902 | gtgtcccgagccccatgagatct | 129217 | - | see also 1114 line | | | |
| 13776 | BCL10 | NM_003921.2 | 941 | tggacacccttgttgaatctatt | 129233 | - | apoptosis、cell_cycle 、kinase_related | protein binding | hepatocellular carcinoma、non-hodgkin lymphoma、malignant mesothelioma、lymphoma | |
| 13777 | BCL2L10 | NM_020396.2 | 611 | ctgtcatgcttgttaacaacagc | 129248 | - | apoptosis | - | - | |
| 13778 | BCL6B | NM_181844.2 | 1229 | gtgcggctcgcgctttgtacagg | 129260 | - | see also 10257 line | | | |
| 13779 | BTBD1 | NM_025238.1 | 1119 | gggacgagtgatcgaatcagatt | 129283 | - | - | protein binding | - | |
| 13780 | BTBD11 | NM_152322.1 | 379 | ctgcatagagattggttatgtga | 129298 | - | - | - | - | |
| 13781 | BTBD12 | NM_032444.1 | 2267 | cagtgacccccaattccaattg | 129332 | - | - | - | - | |
| 13782 | BTBD14A | NM_144653.2 | 1668 | gagaactgacgccgtgaatgttg | 129345 | - | see also 9726 line | | | |
| 13783 | BTBD14B | NM_052876.2 | 1189 | gcgtctctcccggctgaacttat | 129348 | - | - | - | - | |
| 13784 | BTBD2 | NM_017797.2 | 1060 | aagccacgagtggagttcattga | 129359 | - | - | protein binding | - | |
| 13785 | BTBD3 | NM_014962.2 | 1370 | cggggtattaactctcaatgaga | 129391 | - | - | - | - | |
| 13786 | BTBD5 | NM_017658.1 | 169 | cagcgtcagcccgtatttcaaag | 129406 | - | see also 7085 line | | | |
| 13787 | BTBD6 | NM_033271.1 | 1168 | gtggacagaagggtatttattgc | 129436 | - | receptor_acitivity | - | - | |
| 13788 | BTBD9 | NM_152733.1 | 1067 | ccgttccggcatcgagattaagc | 129467 | - | see also 9935 line | | | |
| 13789 | C16orf44 | NM_024731.2 | 167 | gagtgtctcggccatacaagatc | 129504 | - | - | - | - | |
| 13790 | C3IP1 | NM_021633.2 | 1213 | atggccgttcccgccttagttca | 129538 | - | see also 8156 line | | | |

Figure 46

| | | | | | | | | | |
|---|---|---|---|---|---|---|---|---|---|
| 13791 | C6orf46 | NM_181842.1 | 1523 | cagtgtggccgagattaacgtcc | 129556 | - | see also 10256 line | | |
| 13792 | CALB1 | NM_004929.2 | 926 | ccgaacggatcttgctcttattc | 129573 | - | see also 3428 line | | |
| 13793 | CALM1 | NM_006888.2 | 520 | acgtcacgtcatgacaaacttag | 129582 | - | - | protein binding | - |
| 13794 | CALM3 | NM_005184.1 | 538 | caggtcaattatgaagagtttgt | 129585 | - | - | protein binding | - |
| 13795 | CARD11 | NM_032415.2 | 2088 | cgggtttgacgccttagatctgg | 129618 | - | see also 9185 line | | |
| 13796 | CARD14 | NM_024110.2 | 1749 | aggcgacccacacctggattatg | 129635 | - | apoptosis、kinase_related | - | - |
| 13797 | CBLB | NM_170662.2 | 1511 | aggaactgagcccataatcgtgg | 129676 | - | signal_transduction | zinc binding | diabetes |
| 13798 | CBLC | NM_012116.2 | 1226 | ctgggaggccgtgagtatctacc | 129726 | - | - | zinc binding | - |
| 13799 | CCIN | NM_005893.1 | 1260 | ttgccccaaggcggtatgtctcc | 129764 | - | - | structural constituent of cytoskeleton | - |
| 13800 | CD2 | NM_001767.2 | 462 | atggaactgaccccgaattaaac | 129791 | - | receptor_acitivity、apoptosis、signal_transduction | USF43/USF44 complex | - |
| 13801 | CD244 | NM_016382.1 | 1039 | gggggagcaccatctactctatg | 129824 | - | see also 6846 line | | |
| 13802 | CD28 | NM_006139.1 | 317 | acgacaatgcggtcaaccttagc | 129830 | - | receptor_acitivity、apoptosis、signal_transduction | protein binding | rheumatoid arthritis、diabetes |
| 13803 | CD3Z | NM_000734.2 | 161 | caggcacagttgccgattacaga | 129843 | - | receptor_acitivity、signal_transduction | receptor signaling protein | leukemia、immunodeficiency、diabetes |
| 13804 | CD48 | NM_001778.2 | 304 | gtggcgcactgtacatctctaag | 129856 | - | - | protein binding | - |
| 13805 | CD58 | NM_001779.1 | 456 | cagccatcgaggacttataatgt | 129884 | - | - | protein binding | Alzheimer disease |
| 13806 | CD8B1 | NM_004931.2 | 330 | cgggatgcaagccggttcattct | 129902 | - | receptor_acitivity、signal_transduction、kinase_related | - | lupus erythematosus |
| 13807 | CDC37 | NM_007065.3 | 750 | cagacaatcgtcatgcaatttat | 129919 | - | cell_cycle、kinase_related | protein binding | - |
| 13808 | CIB1 | NM_006384.2 | 419 | atgatgacggaaccttgaacaga | 129969 | - | - | protein binding | - |
| 13809 | CKN1 | NM_000082.1 | 615 | ctggtctccacgttatgactata | 129987 | - | transcription_regulator | RNA polymerase II transcription factor | Cockayne syndrome |
| 13810 | CKTSF1B1 | NM_013372.4 | 593 | ttgcatatccatcgatttggatt | 130018 | - | - | - | - |
| 13811 | CLSPN | NM_022111.2 | 3742 | atgccagtcgccctatggttatt | 130086 | - | see also 8273 line | | |
| 13812 | CNK1 | NM_006314.1 | 1489 | atgtgggtgcgtcatctcattac | 130105 | - | - | - | - |
| 13813 | CRHBP | NM_001882.2 | 494 | ctgagcggtacatagatttctgt | 130119 | - | signal_transduction | protein binding | - |
| 13814 | CRI1 | NM_014335.1 | 494 | tggcgggtttcagatgcattatg | 130136 | - | - | - | - |
| 13815 | DAG1 | NM_004393.1 | 1504 | ccggactcgaggcgccattattc | 130154 | - | receptor_acitivity | laminin receptor | - |
| 13816 | DBT | NM_001918.1 | 910 | ttgctcgtggaattaaactctcc | 130185 | - | - | protein binding | maple syrup urine disease |

Figure 46

| | | | | | | | | | |
|---|---|---|---|---|---|---|---|---|---|
| 13817 | DLG5 | NM_004747.2 | 1692 | tggccgcttaagggtcaatgact | 130338 | - | cell_cycle | - | - |
| 13818 | DLGAP2 | NM_004745.3 | 2092 | ccgcggtctgcgggaataccact | 130387 | - | - | protein binding | - |
| 13819 | DMN | NM_015286.4 | 2369 | aggagcccgtgagttatgtcagc | 130432 | - | - | - | - |
| 13820 | DMXL1 | NM_005509.3 | 4503 | cagtacagtccgacttactttgg | 130607 | - | - | - | - |
| 13821 | DNAJC7 | NM_003315.1 | 358 | gagccctagaactggatcataaa | 130750 | - | see also 2247 line | | |
| 13822 | DNCL2B | NM_130897.1 | 154 | cagagtcataaaggggttattgg | 130773 | - | see also 9555 line | | |
| 13823 | DOK2 | NM_003974.1 | 1017 | tcgacctgaccacatatacgata | 130792 | - | see also 2720 line | | |
| 13824 | DPT | NM_001937.3 | 580 | gtgccggatgactgaatacgact | 130820 | - | - | protein binding | - |
| 13825 | DRE1 | NM_017644.2 | 1098 | cagttgaagtggaccaattgatc | 130850 | - | see also 7070 line | | |
| 13826 | DRPLA | NM_001940.2 | 578 | cagcgaccctagggatatcgacc | 130890 | - | see also 1238 line | | |
| 13827 | DTNA | NM_001390.2 | 1147 | aagaagctgactaatgcattaag | 130927 | - | signal_transduction | - | - |
| 13828 | DTNB | NM_021907.3 | 333 | atgtcatacgactatcaacttac | 130958 | - | see also 8186 line | | |
| 13829 | EMILIN1 | NM_007046.1 | 2766 | tgggcaccggcacgagaaagtgg | 131009 | - | - | - | - |
| 13830 | EMILIN2 | NM_032048.1 | 2948 | tggtgaacgacggggatgtttac | 131046 | - | see also 9073 line | | |
| 13831 | ENG | NM_000118.1 | 462 | gagaggggcgaggtgacatatac | 131051 | - | - | protein binding | Rendu-Osler-Weber disease、hereditary hemorrhagic teleangiectasia |
| 13832 | ESPL1 | NM_012291.3 | 1054 | aagaggcgctatagacttgatgc | 131078 | - | apoptosis、cell_cycle | - | - |
| 13833 | EVI5 | NM_005665.2 | 1065 | gtgtttcgtgtaggattagcact | 131170 | - | see also 3930 line | | |
| 13834 | FBI1 | NM_015898.2 | 1870 | ccgccaccgacggtaacttcaca | 131204 | - | - | - | - |
| 13835 | FBXL2 | NM_012157.2 | 871 | ctgcccgatgctcccatttgact | 131229 | - | - | - | - |
| 13836 | FLJ10572 | NM_018143.1 | 470 | aagccgtaatcgagtacatgtac | 131243 | - | see also 7277 line | | |
| 13837 | FLJ11078 | NM_018316.1 | 1603 | gtgctggctgtggagtactatgt | 131290 | - | - | - | - |
| 13838 | FLJ12587 | NM_022480.2 | 1755 | ttgggacccagaggtgtaagact | 131296 | - | see also 8402 line | | |
| 13839 | FLJ13057 | NM_178439.3 | 323 | cggggccacggattctgttactgt | 131298 | - | - | - | - |
| 13840 | FLJ13063 | NM_024742.1 | 1420 | acgcgagggaaccattctgatcc | 131303 | - | see also 8721 line | | |
| 13841 | FLJ14360 | NM_032775.2 | 312 | tggctgcgtcctgcgattacttc | 131312 | - | - | - | - |
| 13842 | FLJ31322 | NM_152387.1 | 317 | tggacgtctatttaaataccttt | 131345 | - | see also 9847 line | | |
| 13843 | FMOD | NM_002023.2 | 145 | atgacccttacccgtatgagacc | 131360 | - | - | protein binding | - |
| 13844 | FYB | NM_001465.2 | 595 | aagccactaggcccgaaatctgg | 131369 | - | signal_transduction、k inase_related | protein binding | - |
| 13845 | G1P2 | NM_005101.1 | 508 | ctgagcaccgtgttcatgaatct | 131403 | - | - | translation regulator | - |
| 13846 | GAB1 | NM_002039.1 | 1259 | atgtcgccttcacgtagtaatac | 131434 | - | signal_transduction | - | - |
| 13847 | GAN | NM_022041.2 | 372 | agggatatcggtaatggttatga | 131456 | - | - | protein binding | - |
| 13848 | GDF5 | NM_000557.2 | 977 | cagaggtacgtgtttgacattag | 131508 | - | see also 408 line | | |
| 13849 | GMCL | NM_022471.2 | 240 | cggcgagctacggcttctgttac | 131513 | - | - | - | - |
| 13850 | GRF2 | NM_005312.2 | 3229 | gaggaacgacgacattataaact | 131593 | - | signal_transduction、k inase_related | - | - |

Figure 46

| | | | | | | | | | |
|---|---|---|---|---|---|---|---|---|---|
| 13851 | GTF3C2 | NM_001521.1 | 1142 | atggcacctctacgaataaac | 131608 | - | see also 1045 line | | |
| 13852 | HAT1 | NM_003642.1 | 683 | ttgcgaacgtaggctacatgaca | 131663 | - | see also 2459 line | | |
| 13853 | HLA-DMA | NM_006120.2 | 280 | gtgcctcgcctgccgaattgc | 131689 | - | receptor_acitivity | protein binding | - |
| 13854 | HOMER1 | NM_004272.2 | 405 | atgtgtataggataatcagttta | 131703 | - | - | - | - |
| 13855 | HOMER3 | NM_004838.2 | 692 | gcgcgagcggctaaagaagatgt | 131720 | - | signal transduction | - | - |
| 13856 | HSPB2 | NM_001541.2 | 87 | cggccaccgccgagtacgaattt | 131723 | - | - | protein binding | - |
| 13857 | HUMGT198A | NM_013290.3 | 538 | gagagagattgaagaacattaaa | 131734 | - | - | - | - |
| 13858 | HYPE | NM_007076.1 | 1156 | ttgcagccttagcccattataaa | 131741 | - | cell_cycle | - | - |
| 13859 | IFI35 | NM_005533.2 | 773 | gagagtctctcgtatgtgaatg | 131747 | - | - | protein binding | - |
| 13860 | INADL | NM_005799.2 | 3888 | ccgatcaacgcatgagcatattg | 131844 | - | - | - | - |
| 13861 | INHA | NM_002191.2 | 1182 | aggttactctttcaagtatgaga | 131890 | - | see also 1398 line | | - |
| 13862 | INHBA | NM_002192.1 | 335 | ctgaacgcgatcagaaaagcttca | 131895 | - | cell_cycle, apoptosis, signal_transduction | inhibin | - |
| 13863 | IRS1 | NM_005544.1 | 4311 | gaggcatagctccgagacttct | 131931 | - | kinase_related, signal transduction | transmembrane receptor protein tyrosine kinase docking protein | atherosclerosis, diabetes, diabetes mellitus, diabetes |
| 13864 | ISLR | NM_005545.2 | 389 | gtgcctaccgcgacctagaatcc | 131934 | - | receptor_acitivity, sig nal transduction | | - |
| 13865 | ITGB1 | NM_002211.2 | 1782 | ctgcgagtgtgataatttcaact | 131960 | - | - | - | - |
| 13866 | ITGB1BP2 | NM_012278.1 | 633 | gggttgcagagtcgtagaacatga | 132001 | - | see also 5253 line | | - |
| 13867 | IVNS1ABP | NM_006469.2 | 1208 | aagaggttcaaaccttgtactac | 132033 | - | see also 4537 line | | - |
| 13868 | KBTBD3 | NM_152433.2 | 1353 | cagttatggctctcgatagatta | 132100 | - | see also 9863 line | | - |
| 13869 | KBTBD4 | NM_016506.3 | 1583 | atgtcttccggacgatataaa | 132150 | - | see also 6881 line | | - |
| 13870 | KBTBD5 | NM_152393.1 | 968 | gaggaggccgaacgtatccttcc | 132155 | - | - | - | - |
| 13871 | KBTBD6 | NM_152903.1 | 1668 | tggtaattcgagactatcctat | 132161 | - | - | - | - |
| 13872 | KBTBD7 | NM_032138.3 | 2213 | atgcacggagtgagtctagtact | 132195 | - | - | - | - |
| 13873 | KCNA1 | NM_000217.1 | 1132 | atgtaccctgtgacaattggagg | 132204 | - | channel | protein binding | ataxia, paroxysmal ataxia |
| 13874 | KCNA10 | NM_005549.2 | 617 | gagccagtggtcctaaatgaagg | 132216 | - | channel | channel | - |
| 13875 | KCNA2 | NM_004974.2 | 1677 | gtgcgattgcaggtgttaact | 132247 | - | see also 3464 line | | - |
| 13876 | KCNA3 | NM_002232.2 | 1029 | acgtcgcaggactcatttcgaagc | 132257 | - | see also 1425 line | | - |
| 13877 | KCNA5 | NM_002234.2 | 1278 | cagggttcccggaacacatg | 132290 | - | channel | protein binding | - |
| 13878 | KCNA6 | NM_002235.2 | 1546 | atgaagacgattcctacacatt | 132293 | - | see also 1433 line | | - |
| 13879 | KCNA7 | NM_031886.2 | 1366 | gggtggactcccatttcactagc | 132308 | - | channel | channel | - |
| 13880 | KCNB1 | NM_004975.2 | 1908 | atgcgaagtatgtcaagcatttga | 132332 | - | see also 3468 line | | - |
| 13881 | KCNB2 | NM_004770.2 | 816 | gtgcgacgactataatctgaacg | 132350 | - | channel | protein binding | - |

568

Figure 46

| | | | | | | | |
|---|---|---|---|---|---|---|---|
| 13882 | KCNC1 | NM_004976.2 | 1372 | gtgaacaatttcgggatgtatta | - | see also 3470 line | |
| 13883 | KCNC2 | NM_139136.2 | 1648 | gagggtgcttggacatactcttc | - | channel | |
| 13884 | KCNC3 | NM_004977.2 | 1285 | cggggcacctccggagaacatca | - | channel | voltage-gated potassium channel |
| 13885 | KCNC4 | NM_004978.2 | 1692 | gggacagcacctgcagtgatacc | - | see also 3471 line | |
| 13886 | KCND1 | NM_004979.3 | 2005 | cagcctgtgtactcatattcaca | - | - | |
| 13887 | KCND2 | NM_012281.2 | 1735 | ttgtgcgtagtgtcatgagtatc | - | see also 5255 line | |
| 13888 | KCND3 | NM_004980.3 | 2118 | ctgctccgtcgtagtaagaaga | - | see also 3473 line | |
| 13889 | KCNF1 | NM_002236.4 | 751 | gtgtgtcatcgaggtgtactatt | - | channel | |
| 13890 | KCNG4 | NM_133490.2 | 411 | tcgtgcagctcgcgatgattac | - | channel | |
| 13891 | KCNRG | NM_173605.1 | 683 | cagccgccgacctggaatggtaac | - | channel | |
| 13892 | KCNS1 | NM_002251.3 | 410 | ctggtccggggaacaacactatga | - | channel | |
| 13893 | KCNS3 | NM_002252.3 | 920 | ctgcctgtccgctaagcttatcg | - | see also 1448 line | |
| 13894 | KCNV1 | NM_014379.2 | 672 | ccgatatgtcctgcactactacc | - | channel | |
| 13895 | KCTD10 | NM_031954.2 | 276 | gggaagcactttggtacgatact | - | channel | |
| 13896 | KCTD12 | NM_138444.2 | 1034 | gcgctacacctcgcgctattacc | - | channel | |
| 13897 | KCTD13 | NM_178863.2 | 458 | cggccgtcacttggtacaatcc | - | see also 10225 line | |
| 13898 | KCTD14 | NM_023930.2 | 317 | aggctcagttctacgaaatcaag | - | channel | |
| 13899 | KCTD15 | NM_024076.1 | 493 | aggataagccgcctcttcaatgg | - | channel | |
| 13900 | KCTD3 | NM_016121.2 | 212 | acgtaggggggaccagatttagt | - | channel | |
| 13901 | KCTD6 | NM_153331.2 | 763 | tagtactcggaagctttctaagt | - | see also 9986 line | |
| 13902 | KEAP1 | NM_012289.2 | 1591 | gaggaacgagtggcgaatgatca | - | transcription_regulator | protein binding |
| 13903 | KIAA0469 | NM_014851.1 | 1236 | gcgtgttgaggtacaactcaagc | - | - | |
| 13904 | KIAA0711 | NM_014867.1 | 2450 | tggctctgacggcttcatctac | - | - | protein binding |
| 13905 | KIAA0795 | NM_025010.1 | 758 | tggttgcgttgctgccacaaatgc | - | - | |
| 13906 | KIAA1309 | NM_033495.1 | 245 | caggaactacacgcatctttacc | - | see also 9428 line | |
| 13907 | KIAA1354 | NM_018847.1 | 616 | gtggtattgcaaggcttgatca | - | - | protein binding |
| 13908 | KIAA1900 | NM_052904.1 | 1886 | cggatccttgtcggccatataga | - | - | |
| 13909 | KIP2 | NM_006383.1 | 91 | aagctgcattcgcgattctatga | - | - | protein binding |
| 13910 | KLHL6 | NM_130446.1 | 119 | acgacgcgggaactcctcctaatt | - | - | |
| 13911 | KLHL8 | NM_020803.3 | 809 | atggaacatggcggatcagtatgc | - | - | protein binding |
| 13912 | KNTC1 | NM_014708.2 | 4171 | ttgtcgcttgagatcttgacc | - | cell_cycle | |
| 13913 | KPNA1 | NM_002264.1 | 1113 | aggaagcatgttggacgatatct | - | see also 1450 line | |
| 13914 | LCP2 | NM_005565.2 | 391 | gtgccgattctcagttaagttaag | - | signal_transduction, kinase_related | USF43/USF44 complex |

Figure 46

| | | | | | | | | | |
|---|---|---|---|---|---|---|---|---|---|
| 13915 | LGALS3BP | NM_005567.2 | 538 | gtgtggtctgcaccaatgaaacc | 133315 | - | receptor_acitivity、sig nal_transduction | - | - |
| 13916 | LIMD1 | NM_014240.1 | 238 | gggagtagaggccctgtcaatgg | 133325 | - | signal_transduction、tr anscription_regulator | - | - |
| 13917 | LIN7A | NM_004664.1 | 376 | atgcatgaaacgataactgttaa | 133351 | - | - | - | - |
| 13918 | LMNA | NM_005572.2 | 1620 | aagcgccagaatggagatgatcc | 133363 | - | - | - | mandibuloacral dysplasia、atherosclerosis、cardiomyopathy、limb girdle muscular dystrophy、lipodystrophy、progeria、Emery-Dreifuss muscular dystrophy、Charcot-Marie-Tooth disease、dilated cardiomyopathy with conduction defects |
| 13919 | LOC92799 | NM_138392.1 | 363 | gagttggatcgatcttcttgtgg | 133370 | - | channel | - | - |
| 13920 | LRP6 | NM_002336.1 | 3472 | caggtgctaaccggatagtatta | 133510 | - | see also 1499 line | | |
| 13921 | LRPAP1 | NM_002337.1 | 532 | gcgggagttcctgcatcacaaag | 133558 | - | receptor_acitivity | lipoprotein binding | Alzheimer disease |
| 13922 | LTBP2 | NM_000428.1 | 3329 | ctgatgggagatgcgtcaattcc | 133570 | - | see also 333 line | | |
| 13923 | LTBP4 | NM_003573.1 | 3163 | gtgaacgagtgtgaaacactaca | 133599 | - | - | - | - |
| 13924 | LY64 | NM_005582.1 | 1422 | tggcatttacccgcttacacatt | 133640 | - | receptor_acitivity | receptor | - |
| 13925 | MADH2 | NM_005901.2 | 1456 | aagccgtctatcagctaactaga | 133708 | - | see also 4111 line | | |
| 13926 | MADH6 | NM_005585.2 | 1813 | ctgtccgattccacattgtctta | 133719 | - | kinase_related、transcr iption_regulator | receptor signaling protein serine/threonine kinase signaling protein | - |
| 13927 | MADH7 | NM_005904.1 | 889 | tagccgactctgcgaactagagt | 133726 | - | see also 4119 line | | |
| 13928 | MALT1 | NM_006785.2 | 1866 | gggtaagtgtcaccttaccaaag | 133779 | - | see also 4779 line | | |
| 13929 | MAP3K7IP1 | NM_006116.1 | 216 | ggggtcttcaacggctatgatgg | 133811 | - | kinase_related | protein binding | - |
| 13930 | MEN1 | NM_000244.2 | 469 | aggtctccgatgtcatatggaac | 133824 | - | transcription_regulator | - | bronchial Adenomas/Carcinoids、lipomatous tumors、angiofibroma、carcinoid tumor of lung、hyperparathyroidism、lipoma、multiple endocrine neoplasia、pancreatic cancer、parathyroid adenoma |
| 13931 | MGC10485 | NM_052875.2 | 750 | caggccttcgactttgagtttac | 133837 | - | see also 9440 line | | |
| 13932 | MGC23166 | NM_152735.2 | 1617 | ccgccaccgggacctatgcaagg | 133860 | - | transcription_regulator | - | - |

Figure 46

| 13933 | MGEA6 | NM_005930.1 | 2434 | atgcaagaggcccattcttgaga | 133887 | - | - | stem cell factor receptor binding | - |
| 13934 | MICB | NM_005931.2 | 486 | ttggctatgaacgtcacaaattt | 133889 | - | - | - | - |
| 13935 | MID1 | NM_000381.1 | 284 | gcgcccaccgcatcctagtatca | 133896 | - | see also 303 line | | |
| 13936 | MPDZ | NM_003829.1 | 2287 | tggtgaccgactcatgtttgtaa | 133993 | - | - | - | - |
| 13937 | MPP2 | NM_005374.2 | 1609 | cggggctacgggcactactttga | 134113 | - | kinase_related、signal_transduction | guanylate kinase | - |
| 13938 | MPP6 | NM_016447.2 | 1375 | tagtattgaatcccactagattt | 134141 | - | - | - | - |
| 13939 | MPZL1 | NM_003953.3 | 938 | atgcggatatccgaaagaattaa | 134158 | - | signal_transduction、kinase_related | - | - |
| 13940 | MTPN | NM_145808.1 | 356 | cagattgtgggcagcttgaaatc | 134160 | - | - | protein binding | - |
| 13941 | NMNAT1 | NM_022787.2 | 257 | aggaaggtacacagttgtcaaag | 134162 | - | - | - | - |
| 13942 | NPHP1 | NM_000272.1 | 1156 | aggtcgagaccaagtcgtatttc | 134198 | - | signal_transduction | - | medullary cystic kidney disease、nephronophthisis、Senior-Loken syndrome |
| 13943 | NPHP4 | NM_015102.2 | 3223 | gggtcgccgagttctttgagttt | 134264 | - | see also 6242 line | | |
| 13944 | NUDT5 | NM_014142.2 | 994 | tggacgccagggtctattcctac | 134290 | - | phosphatase | protein binding | - |
| 13945 | OGT | NM_003605.3 | 449 | ctggcagaagcttattcgaattt | 134296 | - | see also 2417 line | | |
| 13946 | OIP106 | NM_014965.1 | 1075 | ctgctatcgcaaatagttgattt | 134424 | - | - | - | - |
| 13947 | OIP5 | NM_007280.1 | 481 | gggattcccgttggtttccatct | 134454 | - | - | protein binding | - |
| 13948 | OSTF1 | NM_012383.3 | 736 | atgcagttcgaacattaagcaat | 134478 | - | see also 5304 line | | |
| 13949 | P29 | NM_015484.1 | 604 | tagccggagacgtccttataatg | 134492 | - | - | - | - |
| 13950 | PDHX | NM_003477.1 | 194 | tcggggtgatcccattaagatac | 134505 | - | see also 2351 line | | |
| 13951 | PEA15 | NM_003768.2 | 406 | ccgtcctgacctactcactatgg | 134551 | - | - | molecular_function unknown | - |
| 13952 | PEX12 | NM_000286.1 | 1401 | ctgtacacctagactataactct | 134576 | - | see also 250 line | | |
| 13953 | PFDN4 | NM_002623.2 | 77 | ttgcacggaatacaagtagaatc | 134586 | - | - | tubulin-specific chaperone | - |
| 13954 | PGLYRPIalpha | NM_052891.1 | 714 | cagactgtcgtccgaaacataca | 134601 | - | receptor_acitivity | - | - |
| 13955 | PGLYRPIbeta | NM_020393.1 | 758 | atgactctcccagcgaagtatgg | 134618 | - | receptor_acitivity | - | - |
| 13956 | PGRP-L | NM_052890.2 | 149 | tggatcctactcggattgctact | 134626 | - | receptor_acitivity | - | - |
| 13957 | PHC2 | NM_004427.2 | 1153 | tagaagacgtctacgaattcatc | 134649 | - | see also 2959 line | | |
| 13958 | PHIP | NM_017934.4 | 3803 | tggcatatccaacggatctaagt | 134762 | - | signal_transduction | - | - |
| 13959 | PI3 | NM_002638.2 | 139 | acgggagttcctgttaaaggtca | 134821 | - | - | serine protease inhibitor | - |
| 13960 | PMPCA | NM_015160.1 | 90 | gcgtacagacggtttagtagtgg | 134827 | - | - | - | - |
| 13961 | PNRC1 | NM_006813.1 | 726 | ttgtatgagcaaccaaagataaa | 134860 | - | - | protein binding | - |

Figure 46

| | | | | | | | | | |
|---|---|---|---|---|---|---|---|---|---|
| 13962 | PROZ | NM_003891.1 | 122 | aagcgtgcgggctcctatcttct | 134867 | - | see also 2646 line | | |
| 13963 | RAD21 | NM_006265.1 | 369 | gagtagttcgaatctatcacagg | 134895 | - | see also 4356 line | | |
| 13964 | RBBP6 | NM_006910.4 | 1189 | atgcgcagacgaaagaagaatat | 134943 | - | see also 4848 line | | |
| 13965 | RCBTB1 | NM_018191.2 | 824 | ctggacaatggcgaggtatatgg | 135100 | - | - | - | - |
| 13966 | RCN2 | NM_002902.1 | 860 | aagaggaggcgcttcatctaatt | 135134 | - | - | protein binding | - |
| 13967 | RGS19IP1 | NM_005716.1 | 729 | tcgcaaggccttcgacatgatca | 135138 | - | signal_transduction、gpcr | protein binding | - |
| 13968 | RGS20 | NM_003702.2 | 705 | cagtccttatcggagaaatctat | 135150 | - | signal_transduction、gpcr | protein binding | - |
| 13969 | RHOBTB3 | NM_014899.1 | 2087 | atggccgtcgaatatgtacttga | 135206 | - | see also 6083 line | | |
| 13970 | RIN1 | NM_004292.1 | 395 | acgttcctcgtgcggaaatctaa | 135214 | - | - | protein binding | - |
| 13971 | RINZF | NM_023929.2 | 1950 | ttgttcaaacttgccgaaatttc | 135236 | - | see also 8554 line | | |
| 13972 | RPC8 | NM_138338.1 | 279 | ctgcgtggtgtttcatccattcc | 135267 | - | transcription_regulator | - | - |
| 13973 | RPE65 | NM_000329.1 | 1202 | tagtcacgctccccaatacaact | 135304 | - | see also 276 line | | |
| 13974 | RPGR | NM_000328.1 | 1770 | cagccagctacgactatcgaagc | 135365 | - | - | - | - |
| 13975 | RTKN | NM_033046.1 | 1211 | gggaagagccgctgcttactatt | 135383 | - | signal_transduction | oligosaccharyl transferase | - |
| 13976 | RTN4 | NM_007008.1 | 552 | caggcgcctcttcttagttgatg | 135398 | - | apoptosis | - | - |
| 13977 | SAG | NM_000541.2 | 716 | gagctccgtgcgattactgatcc | 135496 | - | phosphatase、signal_transduction | protein phosphatase inhibitor | Oguchi disease |
| 13978 | SARCOSIN | NM_006063.1 | 449 | ctgtctagccatcctaagattag | 135520 | - | - | protein binding | - |
| 13979 | SBBI26 | NM_018846.2 | 2001 | aggacacattctcgaatataata | 135615 | - | see also 7601 line | | |
| 13980 | SCAP1 | NM_003726.2 | 618 | aggataggcgcagctatgagttt | 135633 | - | kinase_related、signal_transduction | - | - |
| 13981 | SEC23IP | NM_007190.2 | 1020 | agggcgctacgatgtttacctct | 135661 | - | - | - | - |
| 13982 | SEMA6A | NM_020796.1 | 1112 | tggaaccattcggggatgaattc | 135738 | - | see also 7972 line | | |
| 13983 | SENP2 | NM_021627.1 | 666 | ccgtcgccatagcaaaggtaatc | 135797 | - | see also 8154 line | | |
| 13984 | SERPINA4 | NM_006215.2 | 1209 | cagcttcgcgatcaaattcttct | 135858 | - | - | serpin | - |
| 13985 | SERPINA7 | NM_000354.2 | 588 | cagcacccaaactgagattgtgg | 135864 | - | - | transporter | hyperthyroxinemia |
| 13986 | SERPINB10 | NM_005024.1 | 1066 | gggagtgagatagatatacgaat | 135912 | - | - | serpin | - |
| 13987 | SERPINB6 | NM_004568.3 | 228 | cagatggcccagatactttcttt | 135918 | - | - | serpin | - |
| 13988 | SERPINB8 | NM_002640.3 | 217 | aggacaactcaagaaacgtattc | 135925 | - | - | serpin | - |
| 13989 | SERPINB9 | NM_004155.3 | 525 | ttgccgggtagctcaattgatgc | 135949 | - | - | serpin | - |
| 13990 | SGTA | NM_003021.3 | 840 | gagcatggcttcgaacctaatga | 135969 | - | - | protein binding | - |
| 13991 | SHANK2 | NM_012309.1 | 40 | atgatgacgggctacaataatgg | 135973 | - | - | - | - |

Figure 46

| ID | Symbol | Accession | Len | Sequence | ID2 | | Note | | Function | | Disease |
|---|---|---|---|---|---|---|---|---|---|---|---|
| 13992 | SLC12A9 | NM_020246.2 | 2802 | tgggctactggagactctaacc | 136035 | - | - | | | | |
| 13993 | SLC9A3R2 | NM_004785.1 | 576 | tgggttcaacctgcatagtgaca | 136036 | - | - | | | | |
| 13994 | SLIT1 | NM_003061.1 | 4072 | cagcgaggcgccactcctatgtgg | 136071 | - | - | | | | |
| 13995 | SLIT2 | NM_004787.1 | 3482 | atgcagtgaacggtattacgtgc | 136185 | - | see also 3302 line | | | | |
| 13996 | SLIT3 | NM_003062.1 | 1179 | ccggagggcatgtcgaaatacg | 136211 | - | - | | | | |
| 13997 | SMO | NM_005631.3 | 1732 | ccgggactatgtgctatgtcagg | 136262 | - | gper, receptor_acitivity, signal_transduction | | protein binding | | skin cancer |
| 13998 | SNCAIP | NM_005460.1 | 322 | aagcgggtttcgccactgaaaca | 136267 | - | - | | protein binding | | Parkinson disease |
| 13999 | SNX6 | NM_021249.2 | 1156 | atgttctacagccgaaacttcc | 136327 | - | receptor_acitivity | | | | |
| 14000 | SOCS5 | NM_014011.3 | 597 | aggccgctacggcgtaagttctgt | 136355 | - | kinase_related, signal_transduction | | | | |
| 14001 | SORCS2 | NM_020777.1 | 1293 | aagtgatgacgcttataaacctac | 136411 | - | receptor_acitivity, signal_transduction | | | | |
| 14002 | SPEC1 | NM_020239.2 | 429 | cggattgaccggaccatgattgg | 136427 | - | signal_transduction | | | | |
| 14003 | SPEC2 | NM_020240.1 | 63 | acgccgattgacagaagatga | 136434 | - | kinase_related, signal_transduction | | | | |
| 14004 | SPOP | NM_003563.2 | 1198 | gtggagaacgctgcagaaatct | 136451 | - | | | protein binding | | |
| 14005 | STIM1 | NM_003156.2 | 2526 | gaggaggataatggctctattgg | 136475 | - | see also 2129 line | | | | |
| 14006 | STIM2 | NM_020860.1 | 2441 | aggggattcgcctgtaactgtgg | 136517 | - | | | protein binding | | |
| 14007 | TA-KRP | NM_032505.1 | 279 | ctgaatcgatggatttagtgttg | 136533 | - | | | | | |
| 14008 | TANK | NM_004180.2 | 261 | gagaactatgagcagagaatacg | 136596 | - | signal_transduction | | | | |
| 14009 | TCEB3 | NM_003198.1 | 1947 | cagcggctacgagtactaacaaa | 136671 | - | see also 2160 line | | | | |
| 14010 | TGFA | NM_003236.1 | 363 | tggctgtcttatcatcacatgt | 136682 | - | kinase_related, cell_cycle | | protein tyrosine kinase | | neuroblastoma, squamous cell cancer |
| 14011 | TIP-1 | NM_014604.1 | 304 | aagacggacaagggtatttatgt | 136689 | - | - | | | | |
| 14012 | TNFAIP1 | NM_021137.3 | 1103 | aagcgctacagccacttacgatga | 136697 | - | channel | | voltage-gated potassium channel | | |
| 14013 | TNIP1 | NM_006058.2 | 2066 | aggggttcgaaatccaaatcaga | 136711 | - | - | | protein binding | | |
| 14014 | TNKS | NM_003747.1 | 1924 | gtgagagtacacctatacgtact | 136758 | - | see also 2565 line | | | | |
| 14015 | TOB1 | NM_005749.2 | 908 | aggtgacgccccttaacctca | 136825 | - | see also 3996 line | | | | |
| 14016 | TOLLIP | NM_019009.2 | 370 | gagacgcccaggcacacaatgg | 136832 | - | | | protein binding | | |
| 14017 | TRIM14 | NM_014788.2 | 657 | tggaagccgtggagtacatta | 136852 | - | | | | | |
| 14018 | TRIP10 | NM_004240.1 | 1533 | cagcgaggcgactatcctatgg | 136872 | - | see also 2847 line | | | | |
| 14019 | TRIP15 | NM_004236.1 | 544 | cagtcgtgccagactgatgatgg | 136891 | - | see also 2826 line | | | | |
| 14020 | TRIP6 | NM_003302.1 | 980 | tgggctgctttgtatgttctaca | 136930 | - | | | ligand-dependent thyroid hormone receptor interactor | | |

Figure 46

| | | | | | | | | | |
|---|---|---|---|---|---|---|---|---|---|
| 14021 | TTC1 | NM_003314.1 | 881 | cggctcgtactccatcaatttcg | 136961 | - | see also 2246 line | | |
| 14022 | TWSG1 | NM_020648.3 | 566 | atgttcacgcgccttattccagt | 136975 | - | - | - | - |
| 14023 | USH1C | NM_005709.1 | 497 | tcgtccggatcaatggatattcc | 136989 | - | - | protein binding | - |
| 14024 | VBP1 | NM_003372.3 | 695 | ttgactttcttcgagatcaattt | 137017 | - | see also 2275 line | | |
| 14025 | VPS26 | NM_004896.2 | 114 | gtgagatcgatattgttcttaat | 137025 | - | - | protein transporter | - |
| 14026 | VPS35 | NM_018206.2 | 645 | cagggacatagccgagatagaga | 137065 | - | see also 7319 line | | |
| 14027 | WWP1 | NM_007013.3 | 414 | ttgtagatggagaaattacgaaa | 137069 | - | signal_transduction、tr anscription_regulator | - | - |
| 14028 | XAB2 | NM_020196.1 | 179 | caggctcaatcagctatacgagc | 137078 | - | transcription_regulator | - | - |
| 14029 | YAP1 | NM_006106.2 | 1363 | gagatggatacaggtgatactat | 137108 | - | - | protein binding | - |
| 14030 | ZBTB4 | NM_020899.2 | 1281 | aagtacccctgccgctattgtga | 137114 | - | - | - | - |
| 14031 | ZBTB5 | NM_014872.1 | 954 | tagtgcgatcatgtttgatcagt | 137142 | - | see also 6068 line | | |
| 14032 | ZBTB8 | NM_144621.2 | 1266 | cagatccaaccgtccaatcatct | 137171 | - | transcription_regulator | protein kinase C inhibitor | |
| 14033 | ZFP67 | NM_015872.1 | 229 | atgacctgattgggattccattc | 137177 | - | transcription_regulator | - | - |
| 14034 | ZNF-kaiso | NM_006777.2 | 1037 | ctgactcggccgtcagtaataca | 137207 | - | see also 4776 line | | |
| 14035 | ZNF-U69274 | NM_014415.1 | 529 | tagatcgatcccgtccaatatca | 137263 | - | transcription_regulator | | |
| 14036 | ZNF259 | NM_003904.1 | 324 | agggagtgcgctacactttgtct | 137349 | - | signal_transduction | zinc binding | - |
| 14037 | ZNF499 | NM_032792.2 | 619 | tcgcatacagacagttatcgacg | 137372 | - | transcription_regulator | - | - |
| 14038 | CALR3 | NM_145046.2 | 652 | gagtacgactggaacttaacatc | 137388 | - | - | - | - |
| 14039 | CANX | NM_001746.2 | 1431 | ttgtgctgatcgaagaatagttg | 137433 | - | see also 1156 line | | |
| 14040 | CASQ1 | NM_001231.2 | 1184 | aggagtcgtcaatgttactgatg | 137457 | - | - | calcium ion storage | - |
| 14041 | CASQ2 | NM_001232.1 | 201 | aggaccgagtggtaagtctttcc | 137463 | - | - | calcium ion storage | ventricular tachycardia |
| 14042 | CLGN | NM_004362.1 | 1227 | ctggtcgataatcctaactatca | 137517 | - | see also 2925 line | | |
| 14043 | FLJ20793 | NM_019022.3 | 171 | ctgcgccacagttgttgtacttg | 137538 | - | see also 7663 line | | |
| 14044 | AGPAT2 | NM_006412.2 | 263 | tcgtgcgaagcttcaagtacttt | 137588 | - | - | lysophosphatidate acyltransferase | Berardinelli-Seip congenital lipodystrophy |
| 14045 | AIF1 | NM_001623.3 | 237 | atggagtttgaccttaatggaaa | 137598 | - | cell_cycle | - | - |
| 14046 | AKAP13 | NM_006738.4 | 5754 | ctgcccacggtcattatgagaaa | 137704 | - | kinase_related、recept or_acitivity | guanyl-nucleotide release factor | Alzheimer disease |
| 14047 | ANKRD5 | NM_022096.4 | 1737 | tggtgggccaccgtattacatga | 137817 | - | see also 8268 line | | |
| 14048 | ANXA1 | NM_000700.1 | 175 | cagcggtgagcccctatcctacc | 137855 | - | signal_transduction | receptor binding | - |
| 14049 | ANXA10 | NM_007193.2 | 1037 | ttgcttcagggcattataagaaa | 137890 | - | see also 4995 line | | |

Figure 46

| 14050 | ANXA13 | NM_004306.1 | 689 | cagttacgagccacctttcaagc | 137902 | - | - | calcium-dependent phospholipid binding | - |
|---|---|---|---|---|---|---|---|---|---|
| 14051 | ANXA3 | NM_005139.1 | 948 | cagcaattaaatcggatacttct | 137933 | - | phosphatase | phospholipase A2 inhibitor | - |
| 14052 | ANXA4 | NM_001153.2 | 670 | ctgttcccggaaccgaaatcacc | 137941 | - | - | phospholipase inhibitor | - |
| 14053 | ANXA5 | NM_001154.2 | 316 | ttgacatcccgaagtaatgctca | 137957 | - | - | phospholipase inhibitor | - |
| 14054 | ANXA6 | NM_001155.2 | 1307 | cgggactgacgaagacacaatca | 137996 | - | see also 733 line | | |
| 14055 | ANXA7 | NM_001156.2 | 760 | atgcctcctacgtattacgatgc | 138022 | - | channel | - | - |
| 14056 | ANXA8 | NM_001630.1 | 921 | gtgatgggaccctgataagaaac | 138055 | - | - | calcium-dependent phospholipid binding | - |
| 14057 | ANXA9 | NM_003568.1 | 1201 | cggcctagcttcggtgatcaaga | 138069 | - | - | calcium-dependent phospholipid binding | - |
| 14058 | APBA2BP | NM_031231.2 | 354 | gggcatctcaccgacaatttaga | 138076 | - | - | - | - |
| 14059 | ASPH | NM_004318.2 | 296 | ttggtttgatcttgttgactatg | 138085 | - | see also 2887 line | | |
| 14060 | BMP1 | NM_001199.1 | 1615 | tcgtctctgacgggtccattaac | 138154 | - | see also 779 line | | |
| 14061 | BMPER | NM_133468.2 | 1354 | caggacggagtgtcgcaataagc | 138199 | - | see also 9585 line | | |
| 14062 | C14orf10 | NM_017917.2 | 692 | gagaggaagcgatgatcaattac | 138231 | - | see also 7172 line | | |
| 14063 | C14orf143 | NM_145231.1 | 461 | tggtcacgtcagctttagagact | 138275 | - | - | - | - |
| 14064 | C1orf10 | NM_016190.1 | 275 | ctggtcttagtgtttaaagttgc | 138282 | - | receptor_acitivity | - | - |
| 14065 | C1orf22 | NM_025191.2 | 197 | aagtcgcggtgacgttgatgatg | 138290 | - | - | peptidase | - |
| 14066 | C1QR1 | NM_012072.2 | 510 | aggacacgccttactctaactgg | 138352 | - | receptor_acitivity | receptor | - |
| 14067 | C1R | NM_001733.2 | 959 | gcgctacaccaccgagatcatca | 138362 | - | - | trypsin | - |
| 14068 | C1S | NM_001734.1 | 563 | acggggtttgctgcatactatgt | 138372 | - | - | trypsin | complement C1S deficiency |
| 14069 | Cab45 | NM_016176.2 | 730 | acgtgtcttgggacgagtataag | 138451 | - | - | - | - |
| 14070 | CABP1 | NM_004276.3 | 473 | caggtgggacaccgagacataga | 138458 | - | - | - | - |
| 14071 | CABP2 | NM_016366.1 | 692 | ctggtcgacttcgaagagtttgt | 138462 | - | signal_transduction | - | - |
| 14072 | CABP4 | NM_145200.1 | 735 | agggacagggatggacgaattac | 138464 | - | see also 9779 line | | |
| 14073 | CABP5 | NM_019855.3 | 250 | aggaccgagatgggttcatctct | 138467 | - | signal_transduction | calcium ion binding | - |
| 14074 | CABP7 | NM_182527.1 | 406 | tggcaccgactttgatactgtct | 138476 | - | - | - | - |
| 14075 | CACNA1C | NM_000719.3 | 2320 | gaggagcacattcgataacttcc | 138504 | - | see also 442 line | | |
| 14076 | CACNA1D | NM_000720.1 | 4750 | cagggtagcgtgcaagagattag | 138611 | - | see also 445 line | | |
| 14077 | CACNA1E | NM_000721.1 | 4867 | ctgcgtcagggctataccatacg | 138727 | - | see also 457 line | | |
| 14078 | CACNA1F | NM_005183.1 | 1380 | ctgcgctggttcagtcattctac | 138755 | - | see also 3643 line | | |
| 14079 | CACNA1G | NM_018896.3 | 591 | gtgtctccgcacggtctgtaacc | 138799 | - | see also 7604 line | | |
| 14080 | CACNA1H | NM_021098.1 | 6449 | ccgcactcgtaagcataccttcg | 138852 | - | channel | low voltage-gated calcium channel | - |

Figure 46

| | | | | | | | | | | |
|---|---|---|---|---|---|---|---|---|---|---|
| 14081 | CACNA1I | NM_021096.2 | 161 | tggcgcctattgccttcttctgc | 138855 | - | channel、 signal_transd uction | - | - |
| 14082 | CACNA1S | NM_000069.1 | 4747 | caggtcatccctccaataggaga | 138930 | - | channel | dihydropyridine-sensitive calcium channel | hypokalemic periodic paralysis、 malignant hyperthermia |
| 14083 | CALB2 | NM_001740.2 | 388 | gggctccagcgccgagtttatgg | 138939 | - | see also 1152 line | | |
| 14084 | CALB3 | NM_004057.1 | 192 | aggtccaaacaccctagatgatc | 138947 | - | - | calcium ion binding | - |
| 14085 | CALML5 | NM_017422.3 | 282 | gcgacggcgacggcgaaatcagc | 138951 | - | signal_transduction | calcium ion binding | - |
| 14086 | CALN1 | NM_031468.1 | 649 | aagagttgaagcacattctctat | 138954 | - | - | calcium ion binding | - |
| 14087 | CALU | NM_001219.2 | 772 | ttgttgagtttcgggataagaac | 138978 | - | see also 795 line | | |
| 14088 | CAPN1 | NM_005186.2 | 2145 | ctggcggtcgactttgacaattt | 138995 | - | - | calpain | - |
| 14089 | CAPN11 | NM_007058.1 | 1621 | cagcgagtcatgggaattggatg | 139015 | - | - | calpain | - |
| 14090 | CAPN2 | NM_001748.3 | 1264 | caggaactacccgaacacattct | 139039 | - | see also 1157 line | | |
| 14091 | CAPN3 | NM_000070.2 | 2389 | gagtcctgccgtagcatgattgc | 139102 | - | - | - | muscular dystrophy、 limb girdle muscular dystrophy |
| 14092 | CAPN9 | NM_006615.1 | 845 | ccggacgccgtttggtcttatta | 139125 | - | - | - | - |
| 14093 | CAPNS1 | NM_001749.1 | 503 | cagcgccacagaactcatgaaca | 139149 | - | - | calpain | - |
| 14094 | CAPNS2 | NM_032330.1 | 319 | taggcgatttcggcaacaattta | 139164 | - | - | - | - |
| 14095 | CAPS | NM_004058.2 | 603 | cggaattccaggactactacagc | 139179 | - | - | - | - |
| 14096 | CAPS2 | NM_032606.2 | 1223 | aggatcgtatcacgtaatggacg | 139183 | - | - | - | - |
| 14097 | CBARA1 | NM_006077.1 | 571 | tgggtctggatcaatatataata | 139222 | - | see also 4219 line | | |
| 14098 | CD164L1 | NM_020404.1 | 997 | gtggcttcgagtgttattgtagc | 139241 | - | - | - | melanoma |
| 14099 | CD97 | NM_001784.2 | 1381 | gagcagctttgcgatccttatgg | 139255 | - | gpcr、 receptor_acitivit y、 signal_transduction | - | - |
| 14100 | CDH1 | NM_004360.2 | 1284 | acgaggctaacgtcgtaatcacc | 139286 | - | - | molecular_function unknown | ataxia telangiectasia、 breast cancer、 endometrial carcinoma、 ovarian carcinoma、 melanoma、 gastric cancer |
| 14101 | CDH10 | NM_006727.2 | 2385 | aagctccggcgagatattattcc | 139372 | - | - | calcium ion binding | - |
| 14102 | CDH11 | NM_001797.2 | 1007 | tggaaatagcgccaagttagtgt | 139391 | - | see also 1173 line | | |
| 14103 | CDH12 | NM_004061.2 | 1833 | tagccggaacaacaatagtcaac | 139452 | - | - | calcium ion binding | - |
| 14104 | CDH13 | NM_001257.2 | 956 | atgccctcctgcggtataatatc | 139522 | - | see also 818 line | | |
| 14105 | CDH15 | NM_004933.2 | 742 | tggtcgcggtgtacaatctgacc | 139553 | - | - | calcium ion binding | - |
| 14106 | CDH16 | NM_004062.2 | 269 | gagggcccatttgctatggatcc | 139558 | - | - | calcium ion binding | - |
| 14107 | CDH17 | NM_004063.2 | 2010 | aagtccatatcgggtacaagtgg | 139636 | - | see also 2738 line | | |
| 14108 | CDH18 | NM_004934.2 | 1028 | tagaacggccttacataacatgg | 139676 | - | - | calcium ion binding | - |
| 14109 | CDH19 | NM_021153.2 | 1117 | cagaaccactacggtattagagc | 139764 | - | - | calcium ion binding | - |

Figure 46

| | | | | | | | | | |
|---|---|---|---|---|---|---|---|---|---|
| 14110 | CDH2 | NM_001792.2 | 1737 | atgccgtaccatgttgacaaca | 139854 | - | | see also 1160 line | |
| 14111 | CDH20 | NM_031891.2 | 231 | tgggaccgacccttgtatgtcg | 139898 | - | | see also 9045 line | |
| 14112 | CDH22 | NM_021248.1 | 2388 | atgcgggacaacgtcatcaaata | 139947 | - | - | | |
| 14113 | CDH23 | NM_022124.2 | 1608 | ggggaaggcggacattcgtattc | 139956 | - | | see also 8289 line | |
| 14114 | CDH24 | NM_022478.2 | 349 | gaggggcaggcaccgtatttgt | 140026 | - | - | | |
| 14115 | CDH26 | NM_021810.3 | 272 | aggaccacgcagggagttaagg | 140048 | - | - | | |
| 14116 | CDH3 | NM_001793.2 | 825 | tggttgcttactccatccatagc | 140127 | - | calcium ion binding | | breast cancer |
| 14117 | CDH4 | NM_001794.2 | 587 | gagatccggtccgacaaagaca | 140150 | - | | see also 1165 line | |
| 14118 | CDH5 | NM_001795.2 | 1424 | gagaagtctacccctggtataac | 140200 | - | calcium ion binding | - | |
| 14119 | CDH6 | NM_004932.2 | 1733 | tagtcgagtacctctatatatta | 140240 | - | | see also 3431 line | |
| 14120 | CDH7 | NM_004361.2 | 1218 | aaggtcttatcaataacgtcc | 140281 | - | | see also 2920 line | |
| 14121 | CDH8 | NM_001796.2 | 371 | gtggatcccctttggaactaaac | 140323 | - | | see also 1166 line | |
| 14122 | CDH9 | NM_016279.2 | 670 | tggagtcgtacatcgttatac | 140406 | - | molecular_function unknown | - | |
| 14123 | CEGF3 | NM_152753.2 | 1920 | ctgcccgcaggagacgtattacc | 140487 | - | receptor acitivity | | |
| 14124 | CELSR1 | NM_014246.1 | 2937 | tagcgcctcggtagaaatccagg | 140512 | - | | see also 5554 line | |
| 14125 | CELSR2 | NM_001408.1 | 4259 | gcgcgacgggttgcttgttgtaca | 140581 | - | gpcr, receptor_acitivity, signal_transduction | structural molecule | |
| 14126 | CELSR3 | NM_001407.1 | 3971 | cagccgaaagctagacaataacc | 140645 | - | | see also 975 line | |
| 14127 | CETN1 | NM_004066.1 | 275 | gggaaggcacggggaagatcagc | 140689 | - | calcium ion binding | | |
| 14128 | CETN2 | NM_004344.1 | 182 | tggcaccatagatgttaaagaac | 140695 | - | | see also 2903 line | |
| 14129 | CETN3 | NM_004365.1 | 367 | ttgcgacgtgtgctgctagagaatt | 140705 | - | calcium ion binding | | |
| 14130 | CHGA | NM_001275.2 | 326 | aggtcatccgacacacttcc | 140716 | - | calcium ion binding | | small cell lung cancer |
| 14131 | CHP | NM_007236.3 | 384 | ccgttgattcatcgaactttgg | 140722 | - | potassium channel regulator | signal_transduction | |
| 14132 | CLSTN1 | NM_014944.1 | 968 | ctgatcgcgctgataaagatgc | 140727 | - | | see also 8296 line | |
| 14133 | CLSTN2 | NM_022131.1 | 2640 | acgattctgcgctgactatcaca | 140809 | - | | see also 5912 line | |
| 14134 | CLSTN3 | NM_014718.1 | 1752 | aggaaacatcgtatgtaacact | 140818 | - | - | | |
| 14135 | CLTA | NM_001833.1 | 402 | ctgaaagtatcgtaaatggaga | 140837 | - | - | | |
| 14136 | CLTB | NM_001834.2 | 596 | cagcggccagagtgaacaagtaga | 140844 | - | - | | |
| 14137 | CPNE6 | NM_006032.2 | 606 | cagcaagtctgacccttcatgg | 140852 | - | transporter | - | |
| 14138 | CRB1 | NM_012076.1 | 1916 | aagctcctactccacttgaaagt | 140894 | - | | see also 5111 line | |
| 14139 | CRELD1 | NM_015513.2 | 1212 | agggccagtcgctgtaagaagt | 140961 | - | | see also 6446 line | |
| 14140 | CRTAC1 | NM_018058.1 | 1358 | tcgagacaagccgtatgtgtca | 140971 | - | - | | |
| 14141 | CSN2 | NM_001891.1 | 350 | cagagtgatgcctgtccttaaat | 140977 | - | enzyme inhibitor | - | |
| 14142 | CSPG2 | NM_004385.2 | 1312 | aagaggctacaaccatcgatttg | 140998 | - | | see also 2948 line | |

Figure 46

| 14143 | CSPG3 | NM_004386.1 | 640 | ccgcactgttcggtatcctatca | 141261 | - | - | - | - |
|---|---|---|---|---|---|---|---|---|---|
| 14144 | CUBN | NM_001081.2 | 2467 | aagtcgcttgcggggatgaatta | 141356 | - | receptor_acitivity | - | Imerslund-Grasbeck syndrome、megaloblastic anemia |
| 14145 | DCSTAMP | NM_030788.2 | 1360 | aagacggctgagtctctatctta | 141606 | - | - | - | - |
| 14146 | DEF6 | NM_022047.2 | 193 | gaggaacacttccgagatgatga | 141611 | - | see also 8247 line | | |
| 14147 | DGKA | NM_001345.3 | 1441 | ctgagatagggctccgattattc | 141638 | - | kinase_related | diacylglycerol kinase | - |
| 14148 | DGKB | NM_004080.1 | 2040 | acgaagccatcgacgaatagaga | 141713 | - | kinase_related | - | - |
| 14149 | DKFZp566 D234 | NM_020116.2 | 740 | tggatctgacggagaattctatg | 141772 | - | - | - | - |
| 14150 | DKFZp586 G0123 | NM_013386.2 | 1418 | ttggcctctttcgacgaattatt | 141871 | - | - | - | - |
| 14151 | DLK1 | NM_003836.3 | 392 | gggacggggagctctgtgataga | 141882 | - | - | structural molecule | - |
| 14152 | DLL1 | NM_005618.2 | 2459 | aggatgagtgcgtcatagcaact | 141904 | - | signal_transduction | structural molecule | - |
| 14153 | DLL3 | NM_016941.1 | 1678 | gagctcgtccgtagattggaatc | 141909 | - | signal_transduction | calcium ion binding | - |
| 14154 | DLL4 | NM_019074.2 | 2242 | aggcgccactgcggttacacagt | 141930 | - | signal_transduction | structural molecule | - |
| 14155 | DMP1 | NM_004407.1 | 1552 | gagatagagagccggaaattaac | 141955 | - | - | integrin binding | - |
| 14156 | DNER | NM_139072.2 | 1477 | tggacggggtacactttacctgc | 141978 | - | see also 9657 line | | |
| 14157 | DSC1 | NM_004948.2 | 885 | ttgcgttatatggctatgcaaca | 142010 | - | - | - | - |
| 14158 | DSC2 | NM_004949.2 | 1045 | tagatcgtgagcagtatgaatct | 142095 | - | - | - | - |
| 14159 | DSC3 | NM_001941.2 | 314 | ctgttgcgctgtctgataagaaa | 142157 | - | - | - | - |
| 14160 | DSG1 | NM_001942.1 | 3334 | aagtatagtaccgtgcaatatag | 142345 | - | - | calcium ion binding | - |
| 14161 | DSG2 | NM_001943.1 | 1195 | atgttagcgagagcatggataga | 142392 | - | - | calcium ion binding | - |
| 14162 | DSG3 | NM_001944.1 | 1608 | gtggttgtctccgctagaacact | 142491 | - | see also 1239 line | | |
| 14163 | DSG4 | NM_177986.2 | 3075 | tagcacgactgagggttgtatgg | 142624 | - | see also 10174 line | | |
| 14164 | DUOX1 | NM_017434.3 | 652 | tggctatcacgtgctttcagacc | 142635 | - | - | - | - |
| 14165 | DUOX2 | NM_014080.3 | 311 | gcgctatgacggctggtttaaca | 142654 | - | - | - | - |
| 14166 | EDEM1 | NM_014674.1 | 585 | atgatgtactagggaactactca | 142673 | - | see also 5857 line | | |
| 14167 | EDIL3 | NM_005711.3 | 697 | aagcataccgaggggatacattc | 142723 | - | - | - | - |
| 14168 | EFCBP1 | NM_022351.2 | 663 | tggaacaattcgtaactagattt | 142773 | - | see also 8342 line | | |
| 14169 | EFEMP2 | NM_016938.1 | 1199 | gtgcctacaatgcctttcagatc | 142840 | - | receptor_acitivity | transmembrane receptor | Alzheimer disease |
| 14170 | EFHC1 | NM_018100.1 | 1678 | aagcactcgcgtcaattcagaac | 142905 | - | - | - | - |
| 14171 | EGF | NM_001963.2 | 2374 | cagtggaataacgattgacttct | 142972 | - | kinase_related、signal _transduction | growth factor | melanoma |
| 14172 | EGFL6 | NM_015507.2 | 626 | atgctacgtgtgtgaactctagg | 143012 | - | cell_cycle | - | - |
| 14173 | EGFL7 | NM_016215.2 | 613 | ggggcctgtggagcagcaatatg | 143041 | - | - | - | - |
| 14174 | EGFL9 | NM_023932.1 | 717 | aagacctgtgagcttgtcttacc | 143043 | - | - | - | - |
| 14175 | ELA1 | NM_001971.3 | 398 | gagcgttaccctcaatagctatg | 143051 | - | - | trypsin | - |

Figure 46

| ID | Symbol | Accession | Length | Sequence | See also | Function | Disease |
|---|---|---|---|---|---|---|---|
| 14176 | EML2 | NM_012155.1 | 1744 | ggggcggacggcactgatatcaac | | | |
| 14177 | EMR1 | NM_001974.3 | 1916 | gtgtcgctccatccgaaatcaca | see also 1257 line | | |
| 14178 | EMR2 | NM_013447.2 | 2803 | cagcaggtccgggagcaatatgg | | gpcr_receptor_acitivity, signal_transduction | |
| 14179 | EMR3 | NM_032571.2 | 1450 | gtggggattgatcgaactgaacc | see also 1263 line | gpcr_receptor_acitivity | |
| 14180 | EPS15 | NM_001981.1 | 2699 | gaggcttgccgactaaatcagc | | | |
| 14181 | EPS15R | NM_021235.1 | 79 | caggtcgatccggcatacacag | | | |
| 14182 | ERO1L | NM_014584.1 | 981 | ctggcctacatgcaagcattaat | see also 5764 line | | |
| 14183 | F10 | NM_000504.2 | 244 | cacgcacaagacagaatgaattct | | translation regulator | Stuart-Prower disease |
| 14184 | F12 | NM_000505.2 | 212 | cggcagctgtaccacaaatgtac | | trypsin | Hageman clotting factor deficiency |
| 14185 | F13A1 | NM_000129.2 | 142 | cagttccaccaataactctaat | see also 115 line | | |
| 14186 | F2 | NM_000506.2 | 880 | tgggtactgcgacctcaactatt | apoptosis, cell_cycle | trypsin | disprothrombinemia or hypoprothrombinemia |
| 14187 | F7 | NM_000131.2 | 416 | aggaccagctccagtcctatatc | | trypsin | atherosclerosis, hypoproconvertinemia |
| 14188 | F9 | NM_000133.2 | 782 | ctgtggaggctctatcgttaatg | | steroid hormone receptor | hemophilia B |
| 14189 | FAT | NM_005245.1 | 12855 | gggaccgctacggcctttcttgc | | calcium ion binding | |
| 14190 | FAT2 | NM_001447.1 | 8778 | gcgaactggttggcgaactctaaag | | calcium ion binding | |
| 14191 | FBLN1 | NM_001996.2 | 308 | atgctaacggaatccaaagaatgc | see also 1279 line | | |
| 14192 | FBLN2 | NM_001998.1 | 1692 | gggctatcctgcaatcatgtca | | calcium ion binding | |
| 14193 | FBN1 | NM_000138.2 | 6299 | ttgcgaatgagctactgttatgc | see also 122 line | | |
| 14194 | FBN2 | NM_001999.2 | 2794 | gagccgtgtgaactagatacagc | see also 1282 line | | |
| 14195 | FBN3 | NM_032447.2 | 8641 | ccgcggaaacgagcaaggtttct | | | |
| 14196 | FCN1 | NM_002003.2 | 1014 | gcgggcgaaggggtaacaaatatag | | opsonin | |
| 14197 | FIBL-6 | NM_031935.1 | 11608 | tcgcaggcgaaatagatttacagg | | receptor_acitivity | |
| 14198 | FKBP10 | NM_021939.2 | 1282 | gggacttgttcgataccattac | | | |
| 14199 | FKBP14 | NM_017946.1 | 318 | aggacggctcctttatttcactcc | | | |
| 14200 | FKBP7 | NM_016105.2 | 754 | aagccacgtgacaagtcatatca | see also 6663 line | | |
| 14201 | FKBP9 | NM_007270.2 | 666 | ctggacggaactctgtttgattc | | | |
| 14202 | FLJ11767 | NM_024593.2 | 223 | ctgatcgtaatgcatttcgaaa | | | |
| 14203 | FLJ12443 | NM_024830.3 | 756 | caggacctgcctaattaccttca | | | |
| 14204 | FLJ13612 | NM_025202.2 | 686 | tgtcatcggccagtaagtttga | | | |
| 14205 | FLJ20047 | NM_017639.1 | 623 | ttggaagcacgcttgtacattt | | | |
| 14206 | FLJ20080 | NM_017657.2 | 2773 | tggatccggagttgtatgagtta | see also 7083 line | | |
| 14207 | FLJ20254 | NM_017727.2 | 938 | ttgcctatatgccggaacaactcc | | | |
| 14208 | FLJ20298 | NM_017752.2 | 1685 | tggctactaatcctgactattat | | | |

Figure 46

| 14209 | FLJ22843 | NM_025184.2 | 1033 | tggctacctgttcgatagatata | 145567 | - | - | - | - |
|---|---|---|---|---|---|---|---|---|---|
| 14210 | FLJ23588 | NM_022785.2 | 1779 | gggcgcattggccgaaataattt | 145689 | - | - | - | - |
| 14211 | FLJ25818 | NM_173503.1 | 1041 | tggccgatgcaactgctattaaa | 145852 | - | - | - | - |
| 14212 | FLJ30681 | NM_133459.1 | 314 | cagcagtgcacggacaactttgg | 145871 | - | - | - | - |
| 14213 | FLJ31455 | NM_144964.1 | 925 | ttgatatcctgtccacttactta | 145899 | - | see also 9747 line | | |
| 14214 | FLJ31614 | NM_152573.2 | 1844 | tggtagatgacaacgctaaatct | 145939 | - | signal_transduction | - | - |
| 14215 | FLJ32009 | NM_152718.1 | 1203 | tgggagccatgcatgaatcaagg | 145960 | - | - | - | - |
| 14216 | FLJ34588 | NM_152726.1 | 477 | ttggcgataaagggctaatttca | 145976 | - | see also 9932 line | | |
| 14217 | FLJ37440 | NM_153214.1 | 545 | aagtttggaagcaagtacttagt | 145994 | - | - | - | - |
| 14218 | FLJ37874 | NM_182603.1 | 953 | atgagcgtgctgataatggatca | 146018 | - | - | - | - |
| 14219 | FLJ39116 | NM_152635.1 | 1549 | tcgacgaggtcctgaaatactac | 146033 | - | - | - | - |
| 14220 | FLJ39155 | NM_152403.2 | 3066 | tggtcggtggcaccgagttaagg | 146093 | - | see also 9854 line | | |
| 14221 | FLJ40342 | NM_152347.3 | 3079 | atgactaaattatccgatatatt | 146184 | - | - | - | - |
| 14222 | FLRT1 | NM_013280.3 | 2333 | cggcatccccgacatagactact | 146199 | - | - | receptor signaling protein | - |
| 14223 | FLRT3 | NM_013281.2 | 1279 | aggttcgtgggatggctattaag | 146234 | - | see also 5384 line | | |
| 14224 | FRAS1 | NM_025074.2 | 4378 | atgttcaacatcgcgatcttacc | 146329 | - | see also 8843 line | | |
| 14225 | FSTL1 | NM_007085.3 | 875 | atgacctgtgacggaaagaatca | 146523 | - | - | heparin binding | rheumatoid arthritis |
| 14226 | GAS6 | NM_000820.1 | 1624 | tggacgtcgggactgaatcaacc | 146535 | - | signal_transduction | - | leukemia |
| 14227 | GCA | NM_012198.2 | 259 | tggtgactccgtgtatacttact | 146543 | - | - | calcium ion binding | - |
| 14228 | GPD2 | NM_000408.1 | 2214 | tggaagccggcttgctatactaa | 146621 | - | see also 323 line | | |
| 14229 | GRN | NM_002087.1 | 1631 | aggacacttctgccatgataacc | 146636 | - | signal_transduction | growth factor | - |
| 14230 | GUCA1B | NM_002098.2 | 99 | cagcggcacactctttatgcatg | 146641 | - | signal_transduction | calcium sensitive guanylate cyclase activator | - |
| 14231 | HGF | NM_000601.3 | 1847 | tagtacgattgatttacctaatt | 146713 | - | - | trypsin | atherosclerosis、 renal cell carcinoma、 multiple myeloma |
| 14232 | HPCAL4 | NM_016257.2 | 648 | gagtgacccatccattgtgttgc | 146730 | - | - | - | - |
| 14233 | HRC | NM_002152.1 | 1532 | aagagggtccatcaaagagatga | 146736 | - | see also 1383 line | | |
| 14234 | HUMMLC2B | NM_013292.2 | 174 | cagaaccgtgatggtattataga | 146741 | - | - | - | - |
| 14235 | IPO4 | NM_024658.2 | 1578 | ctgcgggaattcctgttaacagg | 146768 | - | see also 8687 line | | |
| 14236 | ITIH1 | NM_002215.1 | 1140 | aggtttgctccggggaattgaga | 146790 | - | - | serine protease inhibitor | - |
| 14237 | ITSN1 | NM_003024.1 | 829 | aggtccccaagcaagaactattc | 146826 | - | see also 2024 line | | |
| 14238 | ITSN2 | NM_006277.1 | 858 | aagcgcagtctctgattgattta | 146923 | - | see also 4363 line | | |
| 14239 | JAG1 | NM_000214.1 | 794 | ctggccgaggtcctatacgttgc | 147021 | - | see also 162 line | | |

Figure 46

| | | | | | | | | | |
|---|---|---|---|---|---|---|---|---|---|
| 14240 | JAG2 | NM_002226.3 | 1495 | aggtgccgtccggcttcgaatgc | 147117 | - | cell_cycle、signal_transduction | - | - |
| 14241 | KCNIP1 | NM_014592.2 | 790 | cagcacgtatgcccattacctct | 147137 | - | channel、signal_transduction | - | - |
| 14242 | KCNIP2 | NM_014591.3 | 603 | gcgtggacgatgaatttgaattg | 147157 | - | channel、signal_transduction | - | - |
| 14243 | KIAA0494 | NM_014774.1 | 2439 | gagctaaggggtagctttaggtat | 147203 | - | - | - | - |
| 14244 | KIAA0676 | NM_198868.1 | 2456 | aggagtgtggtccgagctatacc | 147220 | - | - | - | - |
| 14245 | KIAA0980 | NM_025176.3 | 1933 | tggagaccaaggtaaattactac | 147238 | - | - | - | - |
| 14246 | KIP3 | NM_054113.1 | 122 | tcgtgcccctcgactataccacc | 147259 | - | - | - | - |
| 14247 | LALBA | NM_002289.1 | 111 | cagctgctgaaagacatagatgg | 147262 | - | signal_transduction、apoptosis | lactose synthase | - |
| 14248 | LDLR | NM_000527.2 | 798 | ctgtcgccctgacgaattccagt | 147272 | - | receptor_acitivity | Sterol regulatory element binding proteins | atherosclerosis、familial hypercholesterolemia |
| 14249 | LETM1 | NM_012318.1 | 795 | tggcttccgcctgctatggatcg | 147305 | - | see also 5274 line | | |
| 14250 | LOC286097 | NM_181723.1 | 474 | cgggagaggcgatttcgtttatt | 147318 | - | - | - | - |
| 14251 | LOC63928 | NM_022097.1 | 613 | tgggagaccgaattatagaaagc | 147359 | - | - | calcium ion binding | - |
| 14252 | LPA | NM_005577.1 | 11829 | tcggaggatcccattatactatc | 147372 | - | - | - | - |
| 14253 | LRP1 | NM_002332.1 | 6547 | gggtcagtatccgcgtattgagc | 147443 | - | see also 1491 line | | |
| 14254 | LRP2 | NM_004525.1 | 4899 | tggccctgcggcttaactattga | 147673 | - | receptor_acitivity | receptor | - |
| 14255 | LRP8 | NM_004631.2 | 1584 | aagaatgtcgtggcactagatgt | 147947 | - | see also 3146 line | | |
| 14256 | LTBP3 | NM_021070.2 | 2850 | gggcgaccactgcgaaatctacc | 147980 | - | - | - | - |
| 14257 | MAN1B1 | NM_007230.1 | 775 | aaggataccgcaagtttgcatgg | 147989 | - | - | - | - |
| 14258 | MAN1C1 | NM_020379.1 | 891 | gagctataagcgttatgcaatgg | 148007 | - | - | - | - |
| 14259 | MASP1 | NM_001879.3 | 138 | cagatccagtcgcctggttatcc | 148029 | - | - | - | - |
| 14260 | MASP2 | NM_006610.2 | 403 | acggggttcgaggccttctatgc | 148080 | - | - | - | - |
| 14261 | MATN2 | NM_002380.2 | 1719 | ggggaccacggttgtgaacattc | 148144 | - | see also 1534 line | | |
| 14262 | MATN4 | NM_003833.2 | 227 | ttggcgtgatccagtattcgagt | 148161 | - | see also 2613 line | | |
| 14263 | MCFD2 | NM_139279.2 | 430 | aagatgaactgattaacataata | 148172 | - | - | - | - |
| 14264 | MCSC | NM_052901.1 | 1273 | atgcaggcgcaagcctctattga | 148184 | - | - | - | - |
| 14265 | MGC11256 | NM_024324.2 | 797 | cagcgctgcgcagttctgtaaga | 148189 | - | - | - | - |
| 14266 | MGC12458 | NM_032328.1 | 106 | ttgaccatgagtcgaataataca | 148196 | - | see also 9164 line | | |
| 14267 | MGC2615 | NM_024103.1 | 1403 | tggcctgcggtaccatatccagc | 148226 | - | - | - | - |

EP 1 752 536 A1

Figure 46

| | | | | | | | | | | |
|---|---|---|---|---|---|---|---|---|---|---|
| 14268 | MGC26610 | NM_144647.2 | 280 | atggatgacgataataatcgaac | 148232 | - | see also 9724 line | | | |
| 14269 | MGC39650 | NM_152465.1 | 204 | aggacgacgctcacaattgaaag | 148246 | - | - | - | - | - |
| 14270 | MGC39821 | NM_182576.1 | 627 | cagcgatgatgaccgtaatgaaa | 148258 | - | - | - | - | - |
| 14271 | MGC4170 | NM_024312.3 | 1592 | cagtggagggagtcgctatattg | 148318 | - | - | - | - | - |
| 14272 | MGC4266 | NM_032680.2 | 916 | gggcgacatgggcgaagatgagg | 148388 | - | - | - | - | - |
| 14273 | MGC4342 | NM_024329.3 | 326 | aagatgttcaagcagtatgatgc | 148397 | - | - | calcium ion binding | - |
| 14274 | MGP | NM_000900.1 | 282 | gcgaacgctacgccatggtttat | 148425 | - | - | structural constituent of bone | - |
| 14275 | MLC1SA | NM_002475.2 | 214 | aagtggtgatcgagtttaacaag | 148429 | - | - | structural constituent of muscle | - |
| 14276 | MMP1 | NM_002421.2 | 760 | ctgatatcggggctttgatgtac | 148443 | - | see also 1563 line | | | |
| 14277 | MMP11 | NM_005940.2 | 773 | aggggcgttcaacacctatatgg | 148464 | - | - | zinc binding | esophageal cancer、breast cancer、lung cancer、skin cancer |
| 14278 | MMP12 | NM_002426.1 | 881 | ctgtcactaccgtgggaaataag | 148494 | - | - | zinc binding | hepatocellular carcinoma、atherosclerosis、arthritis、skin cancer |
| 14279 | MMP13 | NM_002427.2 | 144 | cagagcgctacctgagatcatac | 148508 | - | see also 1564 line | | | |
| 14280 | MMP2 | NM_004530.1 | 1987 | gcgagtggatgccgcctttaact | 148567 | - | see also 3086 line | | | |
| 14281 | MMP3 | NM_002422.2 | 1085 | atgccgcatatgaagttactagc | 148581 | - | - | translation regulator | atherosclerosis、melanoma |
| 14282 | MMP7 | NM_002423.2 | 370 | tggtcacctacaggatcgtatca | 148599 | - | - | transcription factor-7-like-2-E | esophageal cancer |
| 14283 | MMP8 | NM_002424.1 | 1184 | cagcagcgtccaagcaattgacg | 148631 | - | - | zinc binding | - |
| 14284 | MRC1 | NM_002438.1 | 1419 | atgggacccctgtaacgtttacc | 148672 | - | see also 1570 line | | | |
| 14285 | MUCDHL | NM_017717.3 | 1510 | tcggccatcacatatcgaattac | 148763 | - | - | - | - | - |
| 14286 | MYL2 | NM_000432.1 | 245 | aggctccgggtccaattaacttt | 148778 | - | - | structural constituent of muscle | - |
| 14287 | MYLC2PL | NM_138403.3 | 497 | tcgacactgaagggaaaggtttc | 148792 | - | - | - | - | - |
| 14288 | NELL1 | NM_006157.1 | 736 | atgccgaatggatatataacaca | 148808 | - | kinase_related | structural molecule | - |
| 14289 | NELL2 | NM_006159.1 | 1960 | ttggatggcggatatgattgtcg | 148904 | - | see also 4261 line | | | |
| 14290 | NID | NM_002508.1 | 2202 | cggggcgaacctgctatgatattg | 148951 | - | - | extracellular matrix structural constituent | - |
| 14291 | NIN | NM_016350.3 | 5609 | cagagtgcgagcttaaagtcaca | 149090 | - | see also 6829 line | | | |
| 14292 | NKD1 | NM_033119.3 | 809 | gaggagcgtccttgtcaatcagg | 149108 | - | see also 9369 line | | | |
| 14293 | NOTCH1 | NM_017617.2 | 2423 | acgacgttgccgggtacaagtgc | 149122 | - | receptor_acitivity、transcription_regulator | translation regulator | leukemia |
| 14294 | NOTCH2 | NM_024408.2 | 6041 | ctgattcgcaaccgagtaactga | 149218 | - | see also 8617 line | | | |

Figure 46

| 14295 | NOTCH3 | NM_000435.1 | 3538 | ggggtgctctgcgagattaatga | 149258 | - | see also 344 line | | |
|-------|--------|-------------|------|-------------------------|--------|---|-------------------|---|---|
| 14296 | NOTCH4 | NM_004557.2 | 5905 | tcggcccaaccctgcgataatgc | 149285 | - | see also 3101 line | | |
| 14297 | NOX5 | NM_024505.2 | 1660 | tagacctggtgactacttgtatc | 149294 | - | channel、apoptosis | - | - |
| 14298 | OAZ2 | NM_002537.1 | 407 | caggacctccctgtgaatgatgg | 149309 | - | see also 1632 line | | |
| 14299 | PADI1 | NM_013358.1 | 323 | gggcacagccagtaaggaattaa | 149319 | - | see also 5408 line | | |
| 14300 | PADI2 | NM_007365.1 | 338 | aaggtcaccgtcaactactatga | 149336 | - | - | protein-arginine deiminase | - |
| 14301 | PADI3 | NM_016233.1 | 111 | gagaccctcgtggacatttatgg | 149344 | - | - | protein-arginine deiminase | - |
| 14302 | PADI4 | NM_012387.1 | 1396 | aggcccctgtgaagctctattct | 149372 | - | - | protein-arginine deiminase | - |
| 14303 | PATE | NM_138294.1 | 256 | gggatggtaatccctggttaacc | 149380 | - | - | - | - |
| 14304 | PC-LKC | NM_017675.2 | 2373 | caggggggtgagaccatagtaga | 149406 | - | - | - | - |
| 14305 | PCDH10 | NM_020815.1 | 2685 | tcgtgcgtggcaacgaaatgaac | 149444 | - | see also 7982 line | | |
| 14306 | PCDH12 | NM_016580.2 | 2127 | ctgcgtcgacctctagactatga | 149485 | - | see also 6923 line | | |
| 14307 | PCDH15 | NM_033056.2 | 4091 | gagcggcaaagccgatgtactcg | 149639 | - | - | calcium ion binding | - |
| 14308 | PCDH16 | NM_003737.1 | 3022 | caggccatgtacggcttatgagg | 149717 | - | - | calcium ion binding | - |
| 14309 | PCDH17 | NM_014459.2 | 2103 | aagtcgttcgcgatcaagattct | 149768 | - | - | - | - |
| 14310 | PCDH18 | NM_019035.2 | 2595 | cagggtggccgaatcaacttacc | 149839 | - | see also 7669 line | | |
| 14311 | PCDH20 | NM_022843.2 | 789 | gagcgcaccccctacctaattgt | 149872 | - | see also 8494 line | | |
| 14312 | PCDH7 | NM_002589.2 | 3940 | gggcgatacaggtccgttaatgg | 149957 | - | see also 1669 line | | |
| 14313 | PCDH9 | NM_020403.2 | 3155 | tcgcagcgccgtgttacgtttca | 150083 | - | - | - | - |
| 14314 | PCDHA5 | NM_018908.2 | 765 | aagtgggacattagttattaaac | 150103 | - | - | - | - |
| 14315 | PCDHB1 | NM_013340.2 | 1395 | gtgctaatatccgacgttaatga | 150158 | - | - | calcium ion binding | - |
| 14316 | PCDHB10 | NM_018930.2 | 2299 | aagttcttgaaaccagttatttc | 150209 | - | - | calcium-dependent cell adhesion molecule | - |
| 14317 | PCDHB11 | NM_018931.2 | 1126 | ggggacaacggaagaattgtttg | 150217 | - | - | calcium-dependent cell adhesion molecule | melanoma |
| 14318 | PCDHB12 | NM_018932.2 | 457 | ttgcttgaaagtgcaaaggattt | 150221 | - | - | calcium ion binding | - |
| 14319 | PCDHB13 | NM_018933.2 | 1464 | tggggaccccctatgctgataaca | 150231 | - | - | calcium-dependent cell adhesion molecule | - |
| 14320 | PCDHB14 | NM_018934.2 | 1039 | gtgaccatatcgtcgattacaaa | 150265 | - | - | calcium-dependent cell adhesion molecule | - |
| 14321 | PCDHB15 | NM_018935.2 | 238 | cagaccgggcagttgatattaaa | 150280 | - | - | calcium ion binding | - |

EP 1 752 536 A1

Figure 46

| | | | | | | | | | |
|---|---|---|---|---|---|---|---|---|---|
| 14322 | PCDHB16 | NM_020957.1 | 2002 | cagccttcggaggatattagtaa | 150327 | - | - | calcium-dependent cell adhesion molecule | melanoma |
| 14323 | PCDHB2 | NM_018936.2 | 696 | cagtctcgatggcataatattac | 150349 | - | - | calcium-dependent cell adhesion molecule | - |
| 14324 | PCDHB3 | NM_018937.2 | 757 | gagaataccccgttaactctgt | 150380 | - | - | calcium-dependent cell adhesion molecule | - |
| 14325 | PCDHB4 | NM_018938.2 | 666 | tggcacggtcatggttcgaatcc | 150402 | - | - | calcium-dependent cell adhesion molecule | - |
| 14326 | PCDHB5 | NM_015669.2 | 1042 | gagtgtagcctatgctctattcc | 150443 | - | - | calcium-dependent cell adhesion molecule | - |
| 14327 | PCDHB6 | NM_018939.2 | 75 | gggttctgaatcgattcagtatt | 150456 | - | - | calcium-dependent cell adhesion molecule | - |
| 14328 | PCDHB7 | NM_018940.2 | 294 | gagaggcacctttcttaccaact | 150479 | - | - | calcium ion binding | - |
| 14329 | PCDHB8 | NM_019120.2 | 944 | ctgaggacagtccaataagcttc | 150510 | - | - | calcium ion binding | - |
| 14330 | PCDHB9 | NM_019119.3 | 767 | cagtagggtcccttattgttaaa | 150520 | - | - | calcium-dependent cell adhesion molecule | - |
| 14331 | PDCD6 | NM_013232.2 | 569 | ctgcagaggttgacggatatatt | 150592 | - | apoptosis | calcium ion binding | - |
| 14332 | PEF | NM_012392.1 | 596 | gggaccgctcgggctccattagc | 150606 | - | see also 5308 line | | |
| 14333 | PH-4 | NM_017732.2 | 869 | ctgcctactgaagagtatgaaga | 150610 | - | - | - | - |
| 14334 | PITPNM2 | NM_020845.1 | 518 | ctgtgccccacaacgagtataag | 150626 | - | see also 7992 line | | |
| 14335 | PITPNM3 | NM_031220.1 | 2227 | cagtggtcgcatcacatacaatg | 150648 | - | kinase_related | - | - |
| 14336 | PKD1-like | NM_024874.3 | 3004 | gagtggagcgtgttatatgttat | 150709 | - | see also 8799 line | | |
| 14337 | PKD2L1 | NM_016112.2 | 2549 | aggagtagtgtcccagattgatg | 150750 | - | see also 6669 line | | |
| 14338 | PKD2L2 | NM_014386.1 | 258 | aagtccattcgcagcataactga | 150758 | - | channel | - | - |
| 14339 | PKDREJ | NM_006071.1 | 1452 | ttgtgcaagccgtgatttctata | 150835 | - | channel、signal_transduction | - | - |
| 14340 | PLA2G10 | NM_003561.1 | 791 | gtgcggaccggcagagaacaaat | 150996 | - | - | calcium-independent cytosolic phospholipase A2 | - |
| 14341 | PLA2G1B | NM_000928.2 | 204 | aggcacccccgtggatgaactgg | 151003 | - | signal_transduction | calcium-independent cytosolic phospholipase A2 | - |

Figure 46

| No. | Gene | Accession | Length | Sequence | ID | | Note | Function | Disease/Association |
|---|---|---|---|---|---|---|---|---|---|
| 14342 | PLA2G2A | NM_000300.2 | 375 | aaggaagccgcactcagttatgg | 151011 | - | - | calcium-independent cytosolic phospholipase A2 | rheumatoid arthritis, ApcMin-induced intestinal neoplasia |
| 14343 | PLA2G2E | NM_014589.1 | 418 | gggcaactacaaccgcaaatatg | 151022 | - | - | calcium-independent cytosolic phospholipase A2 | |
| 14344 | PLA2G2F | NM_022819.1 | 390 | ccgtggcttcctcaatgtctact | 151029 | - | - | calcium-independent cytosolic phospholipase A2 | |
| 14345 | PLA2G3 | NM_015715.1 | 1181 | aaggggcaccaccatcagaaagg | 151032 | - | - | calcium-independent cytosolic phospholipase A2 | |
| 14346 | PLA2G5 | NM_000929.1 | 375 | tcgcacacagtcctacaaataca | 151038 | - | - | calcium-independent cytosolic phospholipase A2 | atherosclerosis |
| 14347 | PLCD1 | NM_006225.1 | 398 | aaggaccagcgcaatacaactaga | 151040 | - | - | signal transducer | Alzheimer disease |
| 14348 | PLCD3 | NM_133373.1 | 1271 | aaggagsggagcccgtcatctatc | 151056 | - | - | | |
| 14349 | PLCD4 | NM_032726.2 | 1146 | gagcagcgtcgagggatatatac | 151078 | - | - | | |
| 14350 | PLCG1 | NM_002660.2 | 616 | tcggaatcgtgaggatcgtatat | 151112 | - | - | receptor signaling protein | |
| 14351 | PLCG2 | NM_002661.1 | 1769 | gaggacgagtgccgagaagttgc | 151187 | - | see also 1741 line | | |
| 14352 | PLCZ1 | NM_033123.2 | 603 | atggattcacgtgaatgtctact | 151225 | - | see also 9370 line | | |
| 14353 | PLEK | NM_002664.1 | 101 | aggggagcgtgttcaatacgtgg | 151280 | - | see also 1744 line | | |
| 14354 | PLG | NM_000301.1 | 2135 | ctgaccggaccgaatgtttcatc | 151313 | - | - | trypsin | |
| 14355 | PLSCR1 | NM_021105.1 | 693 | ctgattgctgtacccgaaattgc | 151321 | - | - | phospholipid scramblase | |
| 14356 | PLSCR2 | NM_020359.1 | 360 | ctgtccgccaggattggaatact | 151324 | - | - | phospholipid scramblase | |
| 14357 | PLSCR3 | NM_020360.2 | 877 | aggtgaagactcgggatgaatcc | 151341 | - | - | phospholipid scramblase | |
| 14358 | PLSCR4 | NM_020353.1 | 685 | ttgtatgggccgagaaatcatga | 151353 | - | see also 7843 line | | |
| 14359 | PPEF1 | NM_006240.2 | 1288 | ctggctcccaatcggtacaatcg | 151385 | - | phosphatase | | |
| 14360 | PPEF2 | NM_006239.2 | 2409 | cagtctcacatgaatatcgaca | 151476 | - | see also 4325 line | | |
| 14361 | PPHLN1 | NM_016488.3 | 161 | atggctacaatagactagttaat | 151495 | - | - | | |
| 14362 | PPM2C | NM_018444.1 | 1438 | aggtaacttaccacgcgattaagg | 151560 | - | phosphatase | pyruvate dehydrogenase (lipoamide) phosphatase, regulator | |

Figure 46

| 14363 | PPP2R3A | NM_002718.3 | 3300 | ctgatcacgacctctacatcagc | 151672 | - | phosphatase | protein phosphatase type 2A、 regulator | - |
| 14364 | PR48 | NM_013239.2 | 1141 | acgtcatctactgcaagttctgg | 151691 | - | phosphatase、 cell_cycle | protein phosphatase type 2A、 regulator | - |
| 14365 | PRKCSH | NM_002743.1 | 376 | tggctataagcccctgtatatcc | 151696 | - | kinase_related | molecular_function unknown | - |
| 14366 | PROC | NM_000312.1 | 619 | gtgtcacccccgcagtgaagttcc | 151704 | - | apoptosis | Smad3/Sp-1 heterodimer | thrombophilia |
| 14367 | PROM2 | NM_144707.1 | 1429 | gtgctccgtggtcctattcgtgg | 151713 | - | - | - | - |
| 14368 | PROS1 | NM_000313.1 | 261 | aagcgtcgtgcaaattctttact | 151720 | - | - | endopeptidase inhibitor | thromboembolic disease、 protein S deficiency |
| 14369 | PRRG1 | NM_000950.1 | 617 | atgtagttgggcgctcagattcc | 151791 | - | - | calcium ion binding | - |
| 14370 | PVALB | NM_002854.1 | 283 | gagacctgtctgctaaagaaacc | 151794 | - | - | calcium ion binding | - |
| 14371 | Rab11-FIP3 | NM_014700.1 | 737 | cagaactcgacccgttgttctcc | 151795 | - | - | calcium ion binding | - |
| 14372 | RAB11-FIP4 | NM_032932.2 | 1027 | cagcgaattgctagatgtttact | 151807 | - | - | - | - |
| 14373 | RASGRP1 | NM_005739.2 | 1135 | tggagagtgcaccgacttcaaga | 151838 | - | signal_transduction | - | - |
| 14374 | RASGRP2 | NM_005825.2 | 1452 | acggtgtctctggatcagtatca | 151873 | - | see also 4061 line | | |
| 14375 | RASGRP3 | NM_015376.1 | 1657 | aagatcaggatggcctaattagt | 151915 | - | - | - | - |
| 14376 | RCN1 | NM_002901.1 | 499 | aggaaaccccgcagagtttcatg | 151929 | - | - | calcium ion binding | - |
| 14377 | RCN3 | NM_020650.2 | 567 | gagcgacgctccccatgatgacg | 151932 | - | - | - | - |
| 14378 | RCV1 | NM_002903.1 | 390 | atgtgttccgcagcttcgattcc | 151940 | - | signal_transduction | calcium sensitive guanylate cyclase activator | - |
| 14379 | REPS1 | NM_031922.2 | 1001 | agggatccgtaagccatgatacg | 151950 | - | see also 9053 line | | |
| 14380 | REPS2 | NM_004726.1 | 1015 | cagcgcgagtactatgtcaatca | 152009 | - | - | - | - |
| 14381 | RGN | NM_004683.3 | 265 | aggtttgccggtgggattcattc | 152030 | - | see also 3211 line | | |
| 14382 | RHBDL1 | NM_003961.1 | 37 | gtggaagacgggggaacaactga | 152044 | - | signal_transduction | serine-type endopeptidase | - |
| 14383 | RHBDL4 | NM_138328.1 | 401 | gagcctctcgcagcgacttatcc | 152051 | - | see also 9598 line | | |
| 14384 | RYR3 | NM_001036.2 | 1964 | cagacacgactgattaacgatgt | 152102 | - | receptor_acitivity、 channel | receptor | - |
| 14385 | S100A11 | NM_005620.1 | 330 | cagtgatggtcagctagatttct | 152363 | - | - | calcium ion binding | - |
| 14386 | S100A12 | NM_005621.1 | 245 | aggcctggatgctaatcaagatg | 152370 | - | - | calcium ion binding | - |
| 14387 | S100A14 | NM_020672.1 | 165 | gagaccctcatcaagaactttca | 152372 | - | - | - | - |
| 14388 | S100A16 | NM_080388.1 | 303 | cagaacctggatgccaatcatga | 152373 | - | - | calcium ion binding | - |
| 14389 | S100A5 | NM_002962.1 | 432 | cagcatcgatgacttgatgaaga | 152377 | - | - | calcium ion binding | - |

Figure 46

| | | | | | | | | | |
|---|---|---|---|---|---|---|---|---|---|
| 14390 | S100A8 | NM_002964.3 | 207 | cagacgtctggttcaaagagttg | 152381 | - | - | calcium ion binding | rheumatoid arthritis |
| 14391 | S100A9 | NM_002965.2 | 61 | tcgcagctggaacgcaacataga | 152384 | - | - | translation regulator | - |
| 14392 | S100Z | NM_130772.1 | 480 | aggaaacccagttggttgataag | 152388 | - | - | calcium ion binding | - |
| 14393 | SCG2 | NM_003469.2 | 1614 | tggccaggatgctagttaaatac | 152416 | - | - | calcium ion binding | - |
| 14394 | SCGN | NM_006998.2 | 396 | tggtcgcattcggatgaaagagc | 152428 | - | - | calcium ion binding | - |
| 14395 | SCN10A | NM_006514.1 | 3959 | ttgtacctttgtcgattgtgaat | 152517 | - | see also 4556 line | | |
| 14396 | SCN1A | NM_006920.2 | 2534 | ctgttctccgttcatttcgattg | 152567 | - | channel | voltage-gated sodium channel | epilepsy、myoclonic epilepsy |
| 14397 | SCN2A2 | NM_021007.1 | 6013 | acgtctccaccctcgtatgatag | 152691 | - | channel | voltage-gated sodium channel | Seizures、afebrile、benign familial seizures(-) |
| 14398 | SCN3A | NM_006922.2 | 2326 | gagcgacgcaacagtaacgttag | 152708 | - | channel | voltage-gated sodium channel | - |
| 14399 | SCN4A | NM_000334.3 | 620 | ctgtgtcgacgacttcacattcc | 152758 | - | channel | voltage-gated sodium channel | adynamia episodica hereditaria、hyperkalemic periodic paralysis、paramyotonia congenita |
| 14400 | SCN9A | NM_002977.1 | 4187 | atgtcggacttggttacctatct | 152867 | - | channel | - | - |
| 14401 | SCUBE1 | NM_173050.1 | 623 | ctgcacactaacctgtaattatg | 152887 | - | see also 10047 line | | |
| 14402 | SCUBE2 | NM_020974.1 | 2000 | ttgcagggctgggacctattatg | 152930 | - | see also 8059 line | | |
| 14403 | SEPN1 | NM_020451.1 | 297 | ctgacggggatatgtacatcagc | 152955 | - | - | selenium binding | rigid spine muscular dystrophy |
| 14404 | SLC25A12 | NM_003705.2 | 701 | ttgttcgtaagatatatagcact | 152990 | - | see also 2527 line | | |
| 14405 | SLC25A13 | NM_014251.1 | 256 | ttgtcactcgatacttgaacatt | 153030 | - | see also 5556 line | | |
| 14406 | SMOC1 | NM_022137.2 | 607 | gtggcgaggatggctcctttacc | 153075 | - | see also 8301 line | | |
| 14407 | SMOC2 | NM_022138.1 | 876 | aggtacgagcagccgaaatgtga | 153103 | - | - | - | - |
| 14408 | SPARCL1 | NM_004684.2 | 1768 | ctgtcatctattcgctactaaat | 153141 | - | - | - | - |
| 14409 | SPOCK3 | NM_016950.1 | 681 | cagataagcccaccagtacaagc | 153170 | - | see also 6979 line | | |
| 14410 | SRI | NM_003130.2 | 274 | ttgccggcttatggtttcaatgc | 153194 | - | - | receptor binding | multidrug-resistance complex |
| 14411 | SSR1 | NM_003144.2 | 602 | tgggcggacgaccatttggtttg | 153221 | - | see also 2125 line | | |
| 14412 | SSR4 | NM_006280.1 | 345 | caggcacctatgaggttagattc | 153240 | - | - | calcium ion binding | - |
| 14413 | STAB1 | NM_015136.1 | 7106 | tggatgatgagctcacgtataag | 153306 | - | - | - | - |
| 14414 | STAB2 | NM_017564.8 | 440 | ccggatgccgccatatttgtagg | 153314 | - | - | - | - |
| 14415 | SWAP70 | NM_015055.1 | 1686 | gtggcccatcatgaaggattaat | 153511 | - | see also 6214 line | | |
| 14416 | SYP | NM_003179.1 | 446 | tcgccttcatgtggctagttagc | 153523 | - | - | transporter | renal cell carcinoma |
| 14417 | TGM1 | NM_000359.1 | 395 | ccgagagggcatgctagtagtga | 153525 | - | - | AP-1(jun-D/c-fos heterodimer) | ichthyosiform erythroderma、ichthyosis congenita |
| 14418 | TGM2 | NM_004613.2 | 985 | ccgcgtcgtgaccaactacaact | 153560 | - | - | protein-glutamine gamma-glutamyltransferase | Alzheimer disease、Parkinson disease |
| 14419 | TGM3 | NM_003245.2 | 1695 | aagatctcgtacgctcagtatga | 153596 | - | see also 2187 line | | |

Figure 46

| | | | | | | | | | |
|---|---|---|---|---|---|---|---|---|---|
| 14420 | TGM4 | NM_003241.1 | 2015 | tggacccaagaaatttatcgtca | 153647 | - | - | protein-glutamine gamma-glutamyltransferase | prostate cancer |
| 14421 | TGM5 | NM_004245.1 | 1622 | aagcatcacgattaatgttctag | 153665 | - | see also 2849 line | | |
| 14422 | TGM7 | NM_052955.1 | 483 | gagtatatcatgcgagattatgg | 153680 | - | - | protein-glutamine gamma-glutamyltransferase | - |
| 14423 | THBD | NM_000361.2 | 489 | gggttacgggagacaacaacacc | 153705 | - | receptor_acitivity | transmembrane receptor | - |
| 14424 | THBS1 | NM_003246.2 | 1764 | aaggactgcgttggtgatgtaac | 153728 | - | see also 2188 line | | |
| 14425 | THBS2 | NM_003247.2 | 3562 | aggactacacggcctataggtgg | 153790 | - | - | structural molecule | colorectal cancer、lung cancer、colorectal cancers |
| 14426 | THBS3 | NM_007112.3 | 181 | ctggggacatctacctcttatcc | 153798 | - | - | structural molecule | - |
| 14427 | THBS4 | NM_003248.3 | 1727 | cagttaccccgacgaagaactgc | 153860 | - | - | structural molecule | - |
| 14428 | TKT | NM_001064.1 | 1373 | tggaactagccgccaatacaaag | 153895 | - | see also 652 line | | |
| 14429 | TKTL1 | NM_012253.1 | 890 | atggtgacaccaggtactctact | 153905 | - | - | transketolase | - |
| 14430 | TLL1 | NM_012464.3 | 3303 | gagtgtggcggacgattgaaagc | 154004 | - | see also 5339 line | | |
| 14431 | TMG3 | NM_024082.2 | 613 | cagagctcagatgccatgtatgt | 154060 | - | - | calcium ion binding | - |
| 14432 | TNNC1 | NM_003280.1 | 479 | atgagttcctggagttcatgaag | 154067 | - | see also 2219 line | | |
| 14433 | TNNC2 | NM_003279.2 | 457 | gagatcgaatctctgatgaaaga | 154069 | - | - | calcium ion binding | - |
| 14434 | TPO | NM_000547.3 | 421 | cagcatccaacggatgctttatc | 154071 | - | - | - | thyroid cancer、thyroid hormone organification defect IIA、iodide organification defect |
| 14435 | TRPA1 | NM_007332.1 | 2124 | ctgccgagactattatatcgagt | 154158 | - | see also 5080 line | | |
| 14436 | TSC | NM_017899.1 | 283 | gagcagctccatcggagatttaa | 154217 | - | - | - | - |
| 14437 | UCC1 | NM_017549.1 | 825 | aggagtggtcggacagaaagtca | 154234 | - | see also 7030 line | | |
| 14438 | UMOD | NM_003361.1 | 1010 | tagacgaggactgcaaatcgaat | 154243 | - | - | calcium ion binding | - |
| 14439 | USP13 | NM_003940.1 | 2336 | caggcactacgagcaacgaataa | 154315 | - | - | ubiquitin-specific protease | - |
| 14440 | USP32 | NM_032582.2 | 839 | ttggtttcaccacctattcgtcc | 154348 | - | - | - | - |
| 14441 | ACE | NM_000789.2 | 2342 | atggccacgtcccggaaatatga | 154420 | - | see also 488 line | | |
| 14442 | ACE2 | NM_021804.1 | 2219 | atgtcccggagccgtatcaatga | 154534 | - | see also 8170 line | | |
| 14443 | ADAM12 | NM_003474.2 | 2235 | ctgcctgaatcgtcaatgtcaaa | 154651 | - | - | - | - |
| 14444 | ADAM15 | NM_003815.2 | 1266 | tggctgctttctgcggaaatatg | 154677 | - | - | zinc binding | - |
| 14445 | ADAM17 | NM_003183.3 | 276 | ttgctctcagactacgatattct | 154687 | - | - | - | osteoarthritis |
| 14446 | ADAM19 | NM_023038.2 | 763 | gagatcgccaactatgttgataa | 154766 | - | - | - | - |
| 14447 | ADAM20 | NM_003814.3 | 816 | tggtcgtggataatattagatat | 154802 | - | - | zinc binding | - |
| 14448 | ADAM21 | NM_003813.2 | 388 | gggggctttcgaggagtattaaa | 154852 | - | - | zinc binding | - |
| 14449 | ADAM28 | NM_014265.1 | 1025 | cagcgataatcttcttagagttg | 154928 | - | - | - | - |
| 14450 | ADAM30 | NM_021794.2 | 882 | atgttcgtatgaggatacactta | 154997 | - | - | zinc binding | - |

Figure 46

| | | | | | | | | | |
|---|---|---|---|---|---|---|---|---|---|
| 14451 | ADAM33 | NM_025220.2 | 1569 | ctgtccccagacgttacctac | 155062 | - | see also 8882 line | | - |
| 14452 | ADAM8 | NM_001109.1 | 1816 | caggacttacacgtttacagatc | 155070 | - | - | zinc binding | - |
| 14453 | ADAM9 | NM_003816.1 | 1055 | acgcaggcgggattaatgtgtt | 155100 | - | kinase_related | - | - |
| 14454 | ADAMDEC1 | NM_014479.2 | 634 | aaggcccaattcgaatcctaga | 155153 | - | see also 5700 line | - | - |
| 14455 | ADAMTS1 | NM_006988.2 | 2339 | tggttgtgatcgcatcatagact | 155213 | - | see also 4880 line | - | - |
| 14456 | ADAMTS10 | NM_030957.1 | 1242 | gagaacggtgtggctaaccatga | 155245 | - | - | zinc binding | - |
| 14457 | ADAMTS12 | NM_030955.1 | 489 | gtgggtccagtccgagtagatgc | 155259 | - | - | zinc binding | - |
| 14458 | ADAMTS13 | NM_139025.1 | 2136 | tggcagagcgagagaatatgtca | 155338 | - | signal_transduction | | Schulman-Upshaw syndrome, thrombotic thrombocytopenic purpura |
| 14459 | ADAMTS15 | NM_139055.1 | 292 | ctgcgacgctgcttctattctgg | 155356 | - | - | - | - |
| 14460 | ADAMTS17 | NM_139057.1 | 977 | gcgatacctcggcaataaccagg | 155375 | - | - | - | - |
| 14461 | ADAMTS19 | NM_133638.1 | 2467 | aagccgcacatagctatttagc | 155469 | - | - | - | - |
| 14462 | ADAMTS3 | NM_014243.1 | 2354 | aagtcgcggacttcatagatct | 155557 | - | - | zinc binding | - |
| 14463 | ADAMTS4 | NM_005099.2 | 851 | ctgttaggcgtgttacaaatcg | 155593 | - | see also 3577 line | - | - |
| 14464 | ADAMTS5 | NM_007038.1 | 1264 | tggcagacgttgggaccatagt | 155605 | - | see also 4924 line | - | - |
| 14465 | ADAMTS6 | NM_014273.2 | 434 | aagatcaacgtagtacaactaaa | 155663 | - | - | zinc binding | - |
| 14466 | ADAMTS8 | NM_007037.2 | 1453 | ctgtgccagcccgaatctacaag | 155732 | - | - | zinc binding | - |
| 14467 | ADAMTS9 | NM_020249.2 | 242 | aagaacgcgacgagcgagcattaact | 155755 | - | - | zinc binding | - |
| 14468 | ADH1A | NM_000667.2 | 1172 | aagtatccgtaccattctgatgt | 155903 | - | - | zinc binding | - |
| 14469 | ADH1C | NM_000669.2 | 1155 | ctgcttcgctctggaaagagtat | 155908 | - | - | RXR alpha/RXR alpha heterodimer | - |
| 14470 | ADH4 | NM_000670.2 | 1146 | cagaatcataggtattgacatca | 155924 | - | - | zinc binding | alcoholism |
| 14471 | ADH6 | NM_000672.2 | 583 | tcgctcctctagagaagtatgc | 155955 | - | - | zinc binding | - |
| 14472 | ADH7 | NM_000673.2 | 183 | aagttgccccaccaaagactaaa | 155969 | - | transcription_regulator | zinc binding | - |
| 14473 | AICDA | NM_020661.1 | 213 | ttggttatcttcgcaataagaac | 155991 | - | - | - | - |

Figure 46

| ID | Gene | Accession | No. | Sequence | No. | | Notes | Molecular function | Disease/Other |
|---|---|---|---|---|---|---|---|---|---|
| 14474 | ANPEP | NM_001150.1 | 321 | aagtaaagcgtggaatcgttacc | 156005 | - | receptor_acitivity | zinc binding | - |
| 14475 | APOBEC3G | NM_021822.1 | 401 | aggtgtattccgaacttaagtac | 156035 | - | - | - | - |
| 14476 | ARTS-1 | NM_016442.2 | 1023 | ctgctattccgactttcagtct | 156072 | - | see also 6859 line | - | - |
| 14477 | BSPRY | NM_017688.1 | 952 | tggccgatcagaccatttctatc | 156120 | - | - | - | - |
| 14478 | CA1 | NM_001738.1 | 766 | gtgtaacttgatcatctgtaag | 156134 | - | - | translation regulator | - |
| 14479 | CA10 | NM_020178.2 | 1156 | atgagctatatacgaatgtcaca | 156147 | - | - | zinc binding | - |
| 14480 | CA11 | NM_001217.2 | 787 | cggagccggctcggaacatcaga | 156166 | - | - | zinc binding | - |
| 14481 | CA12 | NM_001218.2 | 750 | cggagaggacgcgtgaatattac | 156176 | - | - | zinc binding | - |
| 14482 | CA14 | NM_012113.1 | 1148 | aagcaggatcctcgtataccaca | 156187 | - | - | zinc binding | - |
| 14483 | CA2 | NM_000067.1 | 77 | ctggggtacggcaaacacacaacg | 156189 | - | - | zinc binding | osteopetrosis, osteopetrosis with renal tubular acidosis |
| 14484 | CA3 | NM_005181.2 | 873 | aagacctgccgagttgtatttga | 156208 | - | - | zinc binding | - |
| 14485 | CA4 | NM_000717.2 | 873 | tggggcagcgcacggtgataaag | 156231 | - | - | zinc binding | - |
| 14486 | CA5A | NM_001739.1 | 172 | gtgcattggcaaaccagcaataac | 156234 | - | - | zinc binding | - |
| 14487 | CA5B | NM_007220.2 | 206 | cagcctctatacttgtacttaca | 156241 | - | see also 5033 line | - | - |
| 14488 | CA6 | NM_001215.1 | 235 | gagttcccatggtcaacaatgg | 156258 | - | - | zinc binding | - |
| 14489 | CA8 | NM_004056.3 | 628 | ctgagcaacgttaatttcaaa | 156286 | - | see also 2733 line | - | - |
| 14490 | CA9 | NM_001216.1 | 1167 | ccgagcgacgcagccttgaatg | 156305 | - | - | zinc binding | renal cell carcinoma |
| 14491 | CDA | NM_001785.1 | 356 | aggatttcaggcaattgctatc | 156310 | - | - | zinc binding | - |
| 14492 | CDADC1 | NM_030911.1 | 554 | ttgcttacggaggcttcagttc | 156329 | - | kinase_related | - | - |
| 14493 | CGI-63 | NM_016011.1 | 647 | gggcctaagaaccatcaatgtgg | 156359 | - | receptor_acitivity | - | - |
| 14494 | CRIP2 | NM_001312.2 | 90 | gtgcgacagaccgtgtacttcg | 156364 | - | - | zinc binding | - |
| 14495 | CRYZ | NM_001889.2 | 879 | atggcaaaggagtcgagtataat | 156374 | - | - | zinc binding | - |
| 14496 | CSRP1 | NM_004078.1 | 90 | gtgtcagaagacggtttacttg | 156402 | - | - | zinc binding | - |
| 14497 | DCTD | NM_001921.1 | 448 | gtgacattccggaaattcatacc | 156421 | - | - | zinc binding | - |
| 14498 | DKFZP547N043 | NM_032018.2 | 607 | atgtcaaacgagctactaacagg | 156444 | - | - | - | - |
| 14499 | DPF3 | NM_012074.1 | 630 | aagcaacagtggcgttataggaaa | 156471 | - | transcription_regulator | - | - |
| 14500 | DUSP12 | NM_007240.1 | 368 | cagtcgaagtgtgcccataataa | 156475 | - | phosphatase | zinc binding | - |
| 14501 | ECE1 | NM_001397.1 | 525 | gagaaaggcgcaagtatactacc | 156499 | - | - | zinc binding | - |
| 14502 | ECEL1 | NM_004826.1 | 2147 | gagttcatcgtccgtctctatga | 156536 | - | see also 3348 line | - | - |
| 14503 | EEA1 | NM_003566.1 | 3913 | tagtcaacggagagttagtgagc | 156630 | - | - | - | - |
| 14504 | ENPEP | NM_001977.2 | 1192 | aagcacacagccgaatatgctgc | 156661 | - | see also 1259 line | - | - |
| 14505 | FASN | NM_004104.3 | 2312 | acgtcctccgccgagtacaatgt | 156723 | - | | Sterol regulatory element binding proteins | - |

590

Figure 46

| | | | | | | | | | |
|---|---|---|---|---|---|---|---|---|---|
| 14506 | FBXO30 | NM_032145.3 | 1827 | aggtgccctttagcttactatgg | 156778 | - | - | - | - |
| 14507 | FGD2 | NM_173558.2 | 1092 | gggcctcgaggacgacatagtag | 156829 | - | - | - | faciogenital dysplasia |
| 14508 | FGD3 | NM_033086.1 | 621 | ggggacggctctccagacatagg | 156836 | - | - | - | - |
| 14509 | FGD4 | NM_139241.1 | 1493 | atgctccttaaggactatctaag | 156869 | - | see also 9676 line | | |
| 14510 | FLJ10759 | NM_018207.1 | 1015 | gagaccaacctcacatatgaaga | 156895 | - | - | - | - |
| 14511 | FLJ23049 | NM_024687.1 | 1084 | cagaccatagcttaagtgaatat | 156928 | - | - | - | - |
| 14512 | FLJ32919 | NM_144588.3 | 563 | ctgatgcggaggaagtatcatag | 156944 | - | - | - | - |
| 14513 | FLJ40722 | NM_173678.1 | 1308 | ccgcaaggcgctctctcaattcc | 156955 | - | - | - | - |
| 14514 | FYCO1 | NM_024513.1 | 4131 | atgccggctgaacaggatactaca | 156990 | - | - | - | - |
| 14515 | GDA | NM_004293.1 | 267 | gtgcttcaagccgtgtgaaataa | 157002 | - | see also 2875 line | | |
| 14516 | HGS | NM_004712.3 | 1048 | ctgagctcgcacggtatctcaac | 157050 | - | kinase_related、signal _transduction | | |
| 14517 | HS3ST1 | NM_005114.1 | 1014 | ttgttggcagaacatttgactgg | 157065 | - | - | - | - |
| 14518 | HSXIAPAF1 | NM_017523.2 | 1068 | ctgccgatcctaaatcaacatca | 157083 | - | - | - | - |
| 14519 | KEL | NM_000420.2 | 1746 | ccggtccctccgagctagaattg | 157107 | - | - | zinc binding | - |
| 14520 | KIAA1576 | NM_020927.1 | 437 | ggggacagcgtgaaaggatatga | 157128 | - | - | - | - |
| 14521 | KPNB1 | NM_002265.4 | 2792 | ctggactaataggggacttatgt | 157201 | - | see also 1451 line | | |
| 14522 | KUB3 | NM_033276.1 | 501 | aggttcgagctgctaaccttagt | 157222 | - | kinase_related | - | - |
| 14523 | LAP3 | NM_015907.1 | 1106 | gaggagctgcaactatatgctca | 157238 | - | - | zinc binding | - |
| 14524 | LMLN | NM_033029.1 | 1003 | atgggatgttcgagataataaga | 157260 | - | - | - | - |
| 14525 | LNPEP | NM_005575.1 | 816 | tagcagggcacaattatacgttg | 157297 | - | see also 3877 line | | |
| 14526 | LOC51321 | NM_016627.1 | 693 | cagatggtgtggggatattcagc | 157359 | - | see also 6962 line | | |
| 14527 | LOC51725 | NM_016298.1 | 1057 | atgggcggatgctgatacacttt | 157389 | - | - | - | - |
| 14528 | LRAP | NM_022350.1 | 2006 | tggttactacatcgttcactatg | 157470 | - | - | - | - |
| 14529 | LTA4H | NM_000895.1 | 316 | agggatcgccaatggaaatctct | 157501 | - | see also 582 line | | |
| 14530 | LTB4DH | NM_012212.2 | 145 | tggctatcctactaatagtgact | 157552 | - | see also 5200 line | | |
| 14531 | MADHIP | NM_004799.1 | 578 | ttgttgtagccgtcatgcataac | 157584 | - | receptor_acitivity | - | - |
| 14532 | MBTPS2 | NM_015884.1 | 1312 | cagattgatatgttatacgtagg | 157725 | - | see also 6557 line | | |
| 14533 | MEP1A | NM_005588.1 | 514 | aggacggaccgggatgattatgt | 157736 | - | - | zinc binding | - |
| 14534 | MEP1B | NM_005925.1 | 541 | gcgttctgaccgggatgactatg | 157787 | - | see also 4142 line | | |
| 14535 | MGC16186 | NM_032372.2 | 444 | cagtgacccaatagaatacctgg | 157842 | - | - | - | - |
| 14536 | MGC45594 | NM_175907.2 | 827 | ctgtgatcgtcctatcaactata | 157856 | - | see also 10157 line | | |
| 14537 | MID2 | NM_012216.2 | 1371 | ctgctagaggggttagattatttt | 157901 | - | see also 5209 line | | |
| 14538 | MIPEP | NM_005932.1 | 984 | agggctctccaaggaacgatagc | 157951 | - | see also 4143 line | | |

Figure 46

| | | | | | | | | | |
|---|---|---|---|---|---|---|---|---|---|
| 14539 | MIRAB13 | NM_033386.1 | 904 | ccgggttcctggcaaactacagg | 157985 | - | transcription_regulator | - | |
| 14540 | MME | NM_000902.2 | 361 | gcggaggctggttgaaacgtaat | 157997 | - | see also 586 line | | |
| 14541 | MMEL2 | NM_033467.1 | 1334 | gaggtgcgctggcgtgaatgtgt | 158078 | - | - | - | - |
| 14542 | MMP10 | NM_002425.1 | 1427 | aagagtaacagctggttacattg | 158109 | - | - | zinc binding | - |
| 14543 | MMP14 | NM_004995.2 | 1459 | gggctgcctaccgacaagattga | 158118 | - | see also 3488 line | | |
| 14544 | MMP15 | NM_002428.1 | 394 | cagttcggggtacgagtgaaagc | 158125 | - | see also 1565 line | | |
| 14545 | MMP16 | NM_005941.2 | 214 | ctgcggaacggagcagtatttca | 158139 | - | see also 4147 line | | |
| 14546 | MMP17 | NM_016155.2 | 572 | cggtgcgtgcactcatgtactac | 158206 | - | - | zinc binding | - |
| 14547 | MMP19 | NM_002429.2 | 1175 | aggtgtggcgctacattaatttc | 158233 | - | - | - | - |
| 14548 | MMP20 | NM_004771.2 | 499 | aggagaagcggatattatgatat | 158259 | - | see also 3283 line | | |
| 14549 | MMP21 | NM_147191.1 | 1673 | atgtttgtgacgtccatatctcc | 158316 | - | see also 9814 line | | |
| 14550 | MMP24 | NM_006690.2 | 1307 | gggccgatgggagatttgtcttc | 158330 | - | see also 4723 line | | |
| 14551 | MMP25 | NM_022468.3 | 406 | aagttgcgcgatgccatcaaagt | 158335 | - | - | - | - |
| 14552 | MMP26 | NM_021801.2 | 19 | atgcagctcgtcatcttaagagt | 158338 | - | - | zinc binding | - |
| 14553 | MMP27 | NM_022122.1 | 915 | gtggaggatctattatgatatca | 158376 | - | - | - | - |
| 14554 | MMP28 | NM_024302.2 | 399 | aagtacggatacctcaatgaaca | 158399 | - | see also 8607 line | | |
| 14555 | MMP9 | NM_004994.1 | 164 | ctgtaccgctatggttacactcg | 158412 | - | - | Transcription factor p65 | oral cavity cancers、 chondrosarcoma、 colorectal cancer、 CNS Tumours、 colorectal cancers |
| 14556 | MNAT1 | NM_002431.1 | 45 | agggttgccctcggtgtaagacc | 158432 | - | see also 1566 line | | |
| 14557 | MPI | NM_002435.1 | 458 | ccggccagttgaggagattgtaa | 158439 | - | - | zinc binding | carbohydrate-deficient glycoprotein syndrome(-) |
| 14558 | MPRP-1 | NM_145243.2 | 1422 | tggcaatcgagttgagtacttgg | 158488 | - | - | - | - |
| 14559 | MTMR4 | NM_004687.3 | 304 | gtgtggagagccgtgatatgttc | 158505 | - | see also 3215 line | | |
| 14560 | NLN | NM_020726.1 | 994 | tggccaaactactcggttatagc | 158577 | - | - | zinc binding | - |
| 14561 | NPEPPS | NM_006310.2 | 2482 | caggacactgtatcggtaattgg | 158656 | - | see also 4404 line | | |
| 14562 | PAM | NM_015057.1 | 3444 | tggaggtttcgaccaaatactag | 158740 | - | - | - | - |
| 14563 | PAPPA | NM_002581.3 | 3688 | ctggtgacggatgggacatatta | 159080 | - | - | zinc binding | - |
| 14564 | PLEKHF1 | NM_024310.2 | 582 | agggcgtgctgaccaaagagtgc | 159103 | - | see also 8608 line | | |
| 14565 | PLEKHF2 | NM_024613.2 | 535 | aaggaatggatggctaatcaaga | 159120 | - | - | - | - |
| 14566 | POLR2H | NM_006232.2 | 197 | tggatctaatcttagatgtaaac | 159133 | - | transcription_regulator | zinc binding | |
| 14567 | PTER | NM_030664.2 | 1137 | aggtcacggctattctcatatac | 159184 | - | - | - | - |
| 14568 | PTPRG | NM_002841.2 | 1818 | gagccagccggagactatctacc | 159223 | - | see also 1862 line | | |
| 14569 | PTPRZ1 | NM_002851.1 | 3254 | atgataataaggcgctttctaaa | 159395 | - | phosphatase | zinc binding | - |
| 14570 | RAI17 | NM_020338.1 | 3223 | ctgccgagcaatagtaacgatga | 159528 | - | see also 7834 line | | |
| 14571 | RFP2 | NM_005798.2 | 908 | atgtgtcagaacccattgtattt | 159546 | - | cell_cycle、 kinase_rel ated | - | - |

Figure 46

| | | | | | | | | | |
|---|---|---|---|---|---|---|---|---|---|
| 14572 | RFWD1 | NM_032271.1 | 1061 | cgggacgcatccatgttaaatga | 159570 | - | - | - | - |
| 14573 | RIMS1 | NM_014989.1 | 2863 | atgatgaaccgcattggtataaa | 159627 | - | - | - | - |
| 14574 | RING1 | NM_002931.2 | 1096 | ggggcgttcacgacgttgaatgg | 159652 | - | transcription_regulator | zinc binding | - |
| 14575 | RNF129 | NM_152620.2 | 1327 | gggcaattgggcgatacatgaag | 159664 | - | - | - | - |
| 14576 | RNF137 | NM_018073.5 | 1450 | ccgagcaggcaccgatgagtacc | 159689 | - | - | - | - |
| 14577 | RNF26 | NM_032015.3 | 872 | tggcctagagagcctaaagctcc | 159694 | - | - | zinc binding | - |
| 14578 | RNF28 | NM_032588.2 | 767 | cagaagtttgacacgttgtatgc | 159702 | - | signal_transduction | zinc binding | - |
| 14579 | RNF29 | NM_033058.2 | 485 | ttggatagacatggggtatatgg | 159708 | - | signal_transduction | - | - |
| 14580 | RNF30 | NM_032546.2 | 392 | aagagaagatcaatatttactgc | 159727 | - | signal_transduction | - | - |
| 14581 | RNF39 | NM_025236.2 | 658 | aagtcccaacatccaagtcatct | 159740 | - | - | - | - |
| 14582 | RNF6 | NM_005977.2 | 477 | cagtctagatcgagatcagatgg | 159743 | - | see also 4172 line | | |
| 14583 | RNPEP | NM_020216.3 | 1237 | ttgacccggacgacacctataat | 159836 | - | see also 7802 line | | |
| 14584 | RPH3A | NM_014954.1 | 1468 | aggaagccaacagctacgattcg | 159868 | - | - | zinc binding | - |
| 14585 | RTN4IP1 | NM_032730.2 | 1102 | ttggcggatccactgaaacatgg | 159919 | - | see also 9278 line | | |
| 14586 | RUFY1 | NM_025158.2 | 1016 | ctgcaacagaccgaatttgctca | 159945 | - | see also 8872 line | | |
| 14587 | RUFY2 | NM_017987.2 | 418 | atgagtatcacgcactaatgatg | 159973 | - | see also 7189 line | | |
| 14588 | THOP1 | NM_003249.3 | 1964 | tggctacgacgcccagtactacg | 160017 | - | see also 2189 line | | |
| 14589 | TIMM10 | NM_012456.1 | 281 | tggaccgatgtgtctctaagtac | 160022 | - | - | zinc binding | - |
| 14590 | TIMM13 | NM_012458.2 | 580 | ccgtgtctcgcgcctacaactcg | 160026 | - | see also 5338 line | | |
| 14591 | TIMM8B | NM_012459.1 | 188 | gtgtagaccgcttcattgacacc | 160035 | - | - | zinc binding | - |
| 14592 | TIMM9 | NM_012460.2 | 478 | ctggggacctacaataaacttac | 160037 | - | - | zinc binding | - |
| 14593 | TP53I3 | NM_004881.2 | 952 | tggagactatgtgctaatccatg | 160042 | - | apoptosis | - | - |
| 14594 | TRAF2 | NM_021138.2 | 1428 | gaggccagtcaacgacatgaaca | 160057 | - | - | - | - |
| 14595 | TRAF3 | NM_003300.2 | 1717 | ggggtcctctcgacgtcatttgg | 160079 | - | see also 2231 line | | |
| 14596 | TRAF4 | NM_004295.2 | 1438 | gcgggatgatgcagtcttcatcc | 160088 | - | apoptosis | - | - |
| 14597 | TRAF5 | NM_004619.2 | 1788 | atgccaagaacgcctacattaaa | 160128 | - | apoptosis、signal_transduction、kinase_related | | |
| 14598 | TRHDE | NM_013381.1 | 1140 | tggggttgtagtacgattatatg | 160154 | - | see also 5414 line | | |
| 14599 | TRIM10 | NM_006778.2 | 755 | ggggagatctgccggtttagtgc | 160238 | - | see also 4778 line | | |
| 14600 | TRIM11 | NM_145214.2 | 1596 | cagcccgaccccgatgactatct | 160252 | - | - | - | - |
| 14601 | TRIM17 | NM_016102.1 | 951 | cagagttcccggacagattgaag | 160269 | - | - | - | - |
| 14602 | TRIM31 | NM_007028.2 | 1130 | ctgcaggcggcagaactacatcg | 160281 | - | - | - | - |
| 14603 | TRIM34 | NM_021616.3 | 749 | aagaagacgctggataagtttgc | 160293 | - | - | - | - |
| 14604 | TRIM35 | NM_015066.1 | 1386 | aggagggcgagctgtctttctat | 160311 | - | - | - | - |
| 14605 | TRIM36 | NM_018700.2 | 715 | atgttggtccaactactaacttc | 160333 | - | - | - | - |

Figure 46

| | | | | | | | | | |
|---|---|---|---|---|---|---|---|---|---|
| 14606 | TRIM37 | NM_015294.1 | 1001 | ttgtgcgaggttactacttatct | 160415 | - | see also 6316 line | | |
| 14607 | TRIM38 | NM_006355.2 | 618 | gtgtcgggctccatttcatatgg | 160474 | - | kinase_related | zinc binding | - |
| 14608 | TRIM4 | NM_033017.2 | 1323 | gggaggacgtcatgggaattact | 160527 | - | - | - | - |
| 14609 | TRIM40 | NM_138700.2 | 847 | aaggacggccaaggaattagaca | 160538 | - | - | - | - |
| 14610 | TRIM41 | NM_033549.2 | 1267 | tgggtgctgcccgtgaatcaacc | 160545 | - | - | - | - |
| 14611 | TRIM42 | NM_152616.3 | 1094 | ctgccgcaatgacaacaaattgc | 160559 | - | see also 9901 line | | |
| 14612 | TRIM44 | NM_017583.3 | 1127 | gagcagaaggccttcatctagt | 160591 | - | see also 7044 line | | |
| 14613 | TRIM45 | NM_025188.1 | 2233 | gcgcaccgaggagtctacttagg | 160627 | - | - | - | - |
| 14614 | TRIM46 | NM_025058.1 | 135 | acgcactggtccgcatcagtacc | 160629 | - | - | - | - |
| 14615 | TRIM47 | NM_033452.1 | 1716 | ctgtacgggacggcaagatgagc | 160653 | - | - | - | - |
| 14616 | TRIM5 | NM_033034.1 | 265 | ctggaatcctggttaatgtaaag | 160655 | - | - | - | - |
| 14617 | TRIM50A | NM_178125.2 | 1443 | cgggctctacctgcactatgagc | 160679 | - | - | - | - |
| 14618 | TRIM52 | NM_032765.2 | 646 | gggacaacgtagactatatgtgg | 160686 | - | - | - | - |
| 14619 | TRIM6 | NM_058166.2 | 754 | aaggggctacgaattatagaaga | 160699 | - | - | - | - |
| 14620 | TRIM9 | NM_015163.3 | 328 | cggggtctccgactatgactatc | 160723 | - | - | - | - |
| 14621 | TSBF1 | NM_173084.1 | 1004 | ctgaggttcaacccgttgaaatt | 160764 | - | - | - | - |
| 14622 | WDFY1 | NM_020830.2 | 1197 | tgggaccgaccgcattgtaaaga | 160798 | - | - | - | - |
| 14623 | ZADH1 | NM_152444.1 | 652 | ctgctggatctaataagacaatg | 161153 | - | - | - | - |
| 14624 | ZFPL1 | NM_006782.2 | 673 | ctgctgccccagccttctacagc | 161173 | - | - | - | - |
| 14625 | ZFYVE1 | NM_021260.1 | 2460 | gtgcgacgtcctttaaagataac | 161211 | - | - | - | - |
| 14626 | ZFYVE16 | NM_014733.1 | 985 | ctgctttgactcgacaaagttcc | 161238 | - | see also 5923 line | | |
| 14627 | ZFYVE19 | NM_032850.3 | 193 | caggtcgctggagttagattacc | 161369 | - | see also 9313 line | | |
| 14628 | ZFYVE20 | NM_022340.1 | 829 | aagctctacgagaaattacgact | 161398 | - | see also 8332 line | | |
| 14629 | ZFYVE21 | NM_024071.2 | 490 | cagccactatgaaatcgaaattg | 161421 | - | - | - | - |
| 14630 | ZFYVE26 | NM_015346.2 | 3119 | ccgaacggcgtttgaatagtagc | 161462 | - | - | - | - |
| 14631 | ZNF185 | NM_007150.1 | 1104 | cagtatctgcacgctatagcaac | 161533 | - | - | zinc binding | - |
| 14632 | ZNF198 | NM_003453.2 | 3680 | gagatccgacggccaaatggaga | 161625 | - | see also 2319 line | | |
| 14633 | ZNF505 | NM_031218.2 | 503 | atggacttaaccagtgtagtaca | 161658 | - | - | - | - |
| 14634 | AGMAT | NM_024758.3 | 515 | aggcgatgtgaatgtcaatcttt | 161662 | - | - | manganese binding | - |
| 14635 | ARG1 | NM_000045.2 | 860 | gggctactctcaggattagatat | 161694 | - | - | manganese binding | hyperargininemia |
| 14636 | ARG2 | NM_001172.2 | 402 | ctggcaatcggtaccattagtgg | 161706 | - | - | manganese binding | - |
| 14637 | B3GAT1 | NM_018644.2 | 355 | ctgctcgcggtacataaggatga | 161723 | - | - | - | - |
| 14638 | B3GAT2 | NM_080742.1 | 802 | cagccagggatgcaagaatctga | 161738 | - | - | manganese binding | - |
| 14639 | B4GALT6 | NM_004775.2 | 1317 | ctggttgataggttgtatacaaa | 161799 | - | - | transferase、transferring glycosyl groups | - |
| 14640 | CDIPT | NM_006319.2 | 719 | cggtgcttcggatctactacacc | 161807 | - | see also 4421 line | | |

Figure 46

| | Symbol | Accession | Length | Sequence | ID | | Annotation | | Function | Disease |
|---|---|---|---|---|---|---|---|---|---|---|
| 14641 | DCD | NM_053283.1 | 348 | aggagccgtccatgacgttaaag | 161812 | - | - | - | phosphoric monoester hydrolase | |
| 14642 | DKFZP566E144 | NM_015523.1 | 304 | ctgaaggtcctaacctgattata | 161819 | - | see also 6447 line | - | | |
| 14643 | GALNT1 | NM_020474.2 | 1203 | tagattatggagatatcgtca | 161846 | - | see also 7884 line | - | | |
| 14644 | NPEPL1 | NM_024663.2 | 1110 | cagatgcgtgtcctatgcttgc | 161869 | - | - | - | | |
| 14645 | PDE8A | NM_002605.1 | 996 | ttgctcgatactataaattcatg | 161899 | - | see also 1685 line | - | | |
| 14646 | PDE8B | NM_003719.1 | 818 | aagttcgctccagttcaaatta | 161943 | - | see also 2547 line | - | | |
| 14647 | PDE9A | NM_002606.1 | 1168 | gagctggcggtccgctacaatga | 162007 | - | signal_transduction | - | manganese binding | |
| 14648 | POFUT1 | NM_015352.1 | 203 | ttgggctctgcattgcaaa | 162023 | - | signal_transduction, transcription_regulator | - | | |
| 14649 | PPM1A | NM_021003.2 | 1163 | tagtcgacacgttgtataag | 162067 | - | phosphatase, kinase_related | - | | |
| 14650 | PPM1B | NM_002706.3 | 1479 | ttgctggcaagctaatgttatt | 162107 | - | phosphatase | - | | |
| 14651 | PPM1D | NM_003620.2 | 616 | tcgtccgagccggctaaggtttg | 162119 | - | phosphatase, cell_cycle | - | protein phosphatase type 2C | breast cancer |
| 14652 | PPM1F | NM_014634.2 | 1089 | cagagccatcggggatgtcttcc | 162155 | - | phosphatase, apoptosis | - | | |
| 14653 | PPM1G | NM_002707.3 | 403 | tggctattgacgccaaatgacc | 162159 | - | phosphatase, cell_cycle | - | | |
| 14654 | PPP1CA | NM_002708.2 | 485 | aagagacgctacaacatccaaact | 162174 | - | | - | protein phosphatase type 2C | |
| 14655 | PPP1CB | NM_002709.1 | 1028 | ttgctaaacgacagttggtaacc | 162204 | - | see also 1763 line | - | | |
| 14656 | PPP1CC | NM_002710.1 | 156 | tgggcggatttagataaactcaac | 162211 | - | phosphatase | - | protein phosphatase type 2C | |
| 14657 | PPP2CA | NM_002715.1 | 1035 | atggaacttgacgatactctaaa | 162261 | - | see also 1767 line | - | | |
| 14658 | PPP2CB | NM_004156.1 | 801 | cagtgcaccaattactgttatc | 162283 | - | | - | protein phosphatase type 2C | |
| 14659 | PPP4C | NM_002720.1 | 636 | cggacaatcgaccgaaagcaaga | 162290 | - | | - | protein phosphatase type 2C | |
| 14660 | PPP5C | NM_006247.2 | 841 | tcggagaccaacccctatatatt | 162303 | - | phosphatase, transcription_regulator, kinase_related | - | protein phosphatase type 2C | |
| 14661 | PPP6C | NM_002721.3 | 959 | cagaacgacaacgccatatttcc | 162332 | - | see also 1768 line | - | | |
| 14662 | ABP1 | NM_001091.1 | 662 | cccgcagttgcttatcattacagc | 162356 | - | | - | heparin binding | |
| 14663 | AOC2 | NM_001158.2 | 1787 | ctggctagcaaccagactaatgc | 162394 | - | | - | | |
| 14664 | AOC3 | NM_003734.2 | 772 | gggacggaacctggtgacaatga | 162398 | - | | - | electron transporter | |

Figure 46

| | | | | | | | | | |
|---|---|---|---|---|---|---|---|---|---|
| 14665 | ATOX1 | NM_004045.2 | 281 | ctgtctctcgggtcctcaataag | 162405 | - | - | copper binding | - |
| 14666 | CCS | NM_005125.1 | 379 | ctgcctcatcgagggaactattg | 162408 | - | - | intracellular copper transporter | - |
| 14667 | CP | NM_000096.1 | 1954 | gaggccgatgtacatggaatata | 162493 | - | - | multicopper ferroxidase iron transport mediator | hypoceruloplasminemia (aceruloplasminemia)、hypoceruloplasminemia、hemo siderosis |
| 14668 | DBH | NM_000787.2 | 1343 | aggagatccgcatgttgaagaag | 162520 | - | see also 487 line | | |
| 14669 | DCT | NM_001922.2 | 1214 | cagacgatccgactctgattagt | 162536 | - | see also 1222 line | | |
| 14670 | F5 | NM_000130.2 | 2633 | gggatacgtctactttcacttgg | 162636 | - | see also 116 line | | |
| 14671 | F8 | NM_000132.2 | 7132 | ctgactcgctaccttcgaattca | 162915 | - | see also 117 line | | |
| 14672 | FLJ12892 | NM_022757.3 | 5892 | gtgactcgactaagagaattaac | 162962 | - | - | - | - |
| 14673 | HEPH | NM_014799.2 | 2159 | gggatgagggcaatctataatgt | 163005 | - | - | - | - |
| 14674 | HYAL4 | NM_012269.1 | 1177 | cagctaccgatattgaatattta | 163061 | - | - | - | - |
| 14675 | LOX | NM_002317.3 | 806 | ctgacgacaacccttattacaac | 163077 | - | see also 1490 line | | |
| 14676 | LOXL1 | NM_005576.1 | 1419 | ttggtcccagaccccaactatgt | 163093 | - | - | protein-lysine 6-oxidase | - |
| 14677 | LOXL2 | NM_002318.1 | 2403 | aggttgcagaatccgattactcc | 163119 | - | receptor_acitivity | scavenger receptor | - |
| 14678 | LOXL4 | NM_032211.5 | 476 | ttggaccagtgcgggtctaatgg | 163143 | - | receptor_acitivity | scavenger receptor | - |
| 14679 | MASS1 | NM_032119.1 | 17786 | atgctcggactgacaacttgtct | 163709 | - | receptor_acitivity、gpc r、signal_transduction | - | - |
| 14680 | MOXD1 | NM_015529.1 | 1696 | tggtcgattcaaggaatgacagc | 163759 | - | see also 6452 line | | |
| 14681 | PAM | NM_000919.2 | 866 | caggtacactatggggatattag | 163782 | - | see also 595 line | | |
| 14682 | SCO2 | NM_005138.1 | 694 | cagttaccgcgtgtactacaatg | 163842 | - | - | copper binding | - |
| 14683 | SLC14A2 | NM_007163.2 | 1188 | agggaccgctcaggtgatgttcg | 163848 | - | see also 4965 line | | |
| 14684 | SLC15A4 | NM_145648.1 | 1462 | aagtatcgcaggcctggaatttg | 163895 | - | see also 9791 line | | |
| 14685 | SLC35B2 | NM_178148.1 | 994 | atgccctgtttgcctataagatg | 163906 | - | kinase_related | - | - |
| 14686 | TRRAP | NM_003496.1 | 4287 | ccgtcactcgtacctttacgaat | 163987 | - | - | - | - |
| 14687 | LTF | NM_002343.1 | 1019 | ctgaaagggacgagtatgagtta | 164140 | - | - | Smad3/Sp-1 heterodimer | - |
| 14688 | MFI2 | NM_005929.3 | 354 | gggcgaagtgtacgatcaagagg | 164151 | - | - | - | melanoma |
| 14689 | MTF | NM_177478.1 | 275 | atgcgtcctacgtgtacttgtcc | 164174 | - | - | - | - |
| 14690 | RSHL1 | NM_030785.1 | 1489 | tcgtgaacgcccgaaagatcaag | 164186 | - | - | - | - |
| 14691 | TF | NM_001063.2 | 1888 | aagcttgcgtccacaagatatta | 164230 | - | - | Nuclear factor 1 C-type-7 | atransferrinemia、alcohol abuse liver dysfunction |
| 14692 | FECH | NM_000140.1 | 410 | aggcggatcccccatcaagatat | 164246 | - | see also 126 line | | |
| 14693 | ACO2 | NM_001098.1 | 1522 | ttgcgggaaccctcaagttcaac | 164296 | - | - | iron binding | - |
| 14694 | ADHFE1 | NM_144650.1 | 534 | caggaaccgggagtgaaactact | 164310 | - | - | - | - |

Figure 46

| 14695 | ALOX12 | NM_000697.1 | 1063 | aagtcctgggtccgaaattcaga | 164341 | - | - | Nuclear factor NF-kappa-B p50 subunit | - |
|---|---|---|---|---|---|---|---|---|---|
| 14696 | ALOX12B | NM_001139.1 | 1946 | ctgatccgatatgtcactatagt | 164369 | - | see also 726 line | | |
| 14697 | ALOX15 | NM_001140.3 | 1143 | aggtgcctgccgtcgatacatcc | 164380 | - | - | lipoxygenase | - |
| 14698 | ALOX15B | NM_001141.1 | 580 | tggagctcaatatcaaatactcc | 164392 | - | apoptosis、cell_cycle | lipoxygenase | - |
| 14699 | ALOX5 | NM_000698.1 | 380 | gagggatggacgcgcaaagttgg | 164399 | - | - | lipoxygenase | atherosclerosis、arachidonate 5-lipoxygenase(-) |
| 14700 | ALOXE3 | NM_021628.1 | 640 | tggatcggtacagaagtacaagg | 164434 | - | - | - | - |
| 14701 | NIFU | NM_014301.1 | 313 | cagcatgtggtgacgtaatgaaa | 164455 | - | - | - | - |
| 14702 | PAH | NM_000277.1 | 1078 | ttgctatgagtacaatcacattt | 164468 | - | - | phenylalanine 4-monooxygenase | phenylketonuria |
| 14703 | SCD4 | NM_024906.1 | 718 | tcgcaagcatcgagatgttattg | 164486 | - | - | - | - |
| 14704 | TH | NM_000360.2 | 804 | gcggctaccgggaagacaatatc | 164500 | - | - | tyrosine 3-monooxygenase | schizophrenia、Segawa syndrome |
| 14705 | TPH1 | NM_004179.1 | 367 | ctgatgtatggatctgaactaga | 164511 | - | see also 2805 line | | |
| 14706 | TPH2 | NM_173353.2 | 842 | atgcttgccgagagtatttgaaa | 164552 | - | see also 10062 line | | |
| 14707 | AOX1 | NM_001159.2 | 1605 | atgcgctagcgatagtcaattca | 164606 | - | - | xanthine dehydrogenase | - |
| 14708 | SUOX | NM_000456.1 | 1771 | ctgctggcatatgagatgaatgg | 164682 | - | - | sulfite oxidase | sulfite oxidase deficiency |
| 14709 | XDH | NM_000379.2 | 2048 | ttgcgaaggataaggttacttgt | 164710 | - | see also 302 line | | |
| 14710 | AFP | NM_001134.1 | 970 | tggaacgtggtcaatgtataatt | 164775 | - | - | nickel binding | hereditary persistence of alpha-fetoprotein (HPAFP) |
| 14711 | ADCY2 | NM_020546.1 | 1402 | gtgacattagggacccatattta | 164836 | - | see also 7895 line | | |
| 14712 | ADCY3 | NM_004036.2 | 1937 | gagagtccgcccaagtagtaaag | 164881 | - | - | guanylate cyclase | - |
| 14713 | ADCY4 | NM_139247.2 | 1318 | gagctaggggagcctacctatct | 164925 | - | - | - | - |
| 14714 | ADCY5 | NM_183357.1 | 2266 | caggtagacgaccgatttggtgc | 164950 | - | - | - | - |
| 14715 | ADCY6 | NM_015270.2 | 3361 | agggagggtggccctcaaatata | 164967 | - | see also 6300 line | | |
| 14716 | ADCY7 | NM_001114.1 | 3223 | ccgcctccgcgtcggcataaacc | 164986 | - | see also 697 line | | |
| 14717 | ADCY8 | NM_001115.1 | 3765 | ctgtctcaacggtgactataacg | 165013 | - | see also 698 line | | |
| 14718 | ADCY9 | NM_001116.1 | 2727 | ttgccgtcgtgcactactgtaac | 165087 | - | see also 704 line | | |
| 14719 | ADK | NM_001123.2 | 958 | gaggcagcgaatcgtgatcttca | 165127 | - | see also 712 line | | |
| 14720 | ADPRH | NM_001125.2 | 1311 | aagagacagctagggctttatat | 165151 | - | - | magnesium binding | - |
| 14721 | ALPI | NM_001631.2 | 1467 | gagcttcgtagcgcatgtcatgg | 165157 | - | phosphatase | magnesium binding | - |
| 14722 | ALPL | NM_000478.2 | 1662 | cggcgtccacgagcagaactacg | 165173 | - | phosphatase | magnesium binding | childhood hypophosphatasia、hypophosphatasia、phosphoethanol aminuria |
| 14723 | B3GALT3 | NM_003781.2 | 1204 | atgtcgggatctgtttgaattta | 165204 | - | see also 2586 line | | |
| 14724 | CDS1 | NM_001263.2 | 895 | atgatctaccatggtttagaaca | 165218 | - | see also 827 line | | |
| 14725 | CERK | NM_022766.4 | 1459 | aagtttatcgcgtcaagaaattc | 165273 | - | kinase_related | - | - |
| 14726 | CLDN16 | NM_006580.2 | 505 | tggtaactcgagcgttgatgatt | 165299 | - | - | structural molecule | hypercalciuria、hypomagnesemia |

Figure 46

| 14727 | COMT | NM_000754.2 | 922 | gtgcacacactaccaatcgttcc | 165318 | - | - | - | breast cancer、alcoholism、Parkinson disease、schizophrenia |
|---|---|---|---|---|---|---|---|---|---|
| 14728 | DKFZp761G058 | NM_152542.2 | 532 | gtggggtgcgcctcacagattgg | 165323 | - | phosphatase | - | - |
| 14729 | ENO2 | NM_001975.2 | 773 | taggtgcagaggtctaccataca | 165343 | - | see also 1258 line | | |
| 14730 | ENO3 | NM_001976.2 | 870 | tgggaagtacgatcttgacttca | 165364 | - | - | - | - |
| 14731 | ENTPD1 | NM_001776.2 | 694 | cagggtgccaggatcattactgg | 165388 | - | see also 1159 line | | |
| 14732 | ENTPD3 | NM_001248.1 | 1326 | aggtatatgcccgctcttactgc | 165436 | - | - | magnesium binding | - |
| 14733 | ENTPD5 | NM_001249.1 | 750 | tggatccgacgaaggcatattag | 165454 | - | phosphatase | uridine diphosphatase | - |
| 14734 | ENTPD6 | NM_001247.1 | 443 | aggtcttctacgggatcatgttt | 165470 | - | phosphatase | - | - |
| 14735 | FACL2 | NM_001995.2 | 388 | acgaagatccgcactacttgaca | 165487 | - | - | magnesium binding | - |
| 14736 | FACL3 | NM_004457.2 | 1560 | agggatacgggctcactgaatct | 165587 | - | - | magnesium binding | - |
| 14737 | FACL5 | NM_016234.2 | 730 | tggcttgttacacgtactctatg | 165694 | - | - | magnesium binding | - |
| 14738 | FACL6 | NM_015256.1 | 352 | cagctacttacccactactatga | 165727 | - | - | magnesium binding | - |
| 14739 | FDFT1 | NM_004462.2 | 112 | tggccacccgaagagttctaca | 165759 | - | see also 3012 line | | |
| 14740 | FMO2 | NM_001460.1 | 1336 | cagacgttgcagaccaattatgt | 165819 | - | - | magnesium binding | - |
| 14741 | GRIN1 | NM_000832.4 | 3151 | gagctccgtggatatctacttcc | 165839 | - | receptor_acitivity、channel、signal_transduction | - | schizophrenia |
| 14742 | GRIN2A | NM_000833.2 | 4425 | gggatgaccaacgcttggttatt | 165915 | - | see also 544 line | | |
| 14743 | GRIN2B | NM_000834.2 | 2738 | ttggcagttccgacattgcttta | 165965 | - | see also 546 line | | |
| 14744 | GRIN2C | NM_000835.2 | 1312 | gggagcatggcgtcctatacatg | 165991 | - | - | magnesium binding | - |
| 14745 | GRIN2D | NM_000836.1 | 1156 | cggcgagagtctgcataggtact | 166003 | - | receptor_acitivity、channel、signal_transduction | magnesium binding | - |
| 14746 | HPCL2 | NM_012260.1 | 1812 | ttggctgacccgctctaatatgt | 166067 | - | see also 5241 line | | |
| 14747 | HPRT1 | NM_000194.1 | 652 | aggatatgcccttgactataatg | 166088 | - | - | transferase、transferring glycosyl groups | arthritis |
| 14748 | IDI1 | NM_004508.2 | 533 | acgaatacgtgttgtagtcatcc | 166097 | - | - | magnesium binding | - |
| 14749 | IDI2 | NM_033261.2 | 317 | ctgtagtagccacccattataca | 166120 | - | - | - | - |
| 14750 | ILKAP | NM_030768.2 | 834 | gagatagtcgggcaatcttgtgt | 166141 | - | see also 8943 line | | |
| 14751 | IMPA1 | NM_005536.2 | 793 | gtggcgtgctaatggatgttaca | 166161 | - | phosphatase、signal_transduction | myo-inositol-1(or 4)-monophosphatase | - |
| 14752 | ITGAE | NM_002208.3 | 2221 | gcgtcgtgaatgtccgtttatgt | 166183 | - | receptor_acitivity、signal_transduction | magnesium binding | - |

EP 1 752 536 A1

Figure 46

| | | | | | | | | | |
|---|---|---|---|---|---|---|---|---|---|
| 14753 | ITGAL | NM_002209.1 | 2752 | gggggactcggttgaattgcacg | 166243 | - | receptor_acitivity、sig nal_transduction | magnesium binding | - |
| 14754 | ITGAM | NM_000632.2 | 656 | aagaattccggattcactttacc | 166252 | - | receptor_acitivity、sig nal_transduction | Smad3/Sp-1 heterodimer | atherosclerosis |
| 14755 | ITGAX | NM_000887.3 | 2328 | ccgccgatgctcagagatacttc | 166312 | - | receptor_acitivity、sig nal_transduction | Smad3/Sp-1 heterodimer | - |
| 14756 | LYSAL1 | NM_004901.1 | 318 | ctgttgtcataatccgaaataag | 166324 | - | see also 3404 line | | |
| 14757 | MTHFD2 | NM_006636.2 | 1066 | gcgagaagtgctgaagtctaaag | 166373 | - | - | methylenetetrahydrofo late dehydrogenase (NAD+) | - |
| 14758 | MTHFS | NM_006441.1 | 452 | gggaggggcaagggctactatga | 166379 | - | - | magnesium binding | - |
| 14759 | PFKL | NM_002626.2 | 1255 | tgggtcccgactgaacatcatca | 166390 | - | kinase_related | magnesium binding | - |
| 14760 | PFKM | NM_000289.3 | 1705 | aagcgtcgggtgtttatcattga | 166427 | - | see also 255 line | | |
| 14761 | PFKP | NM_002627.2 | 523 | caggaacggccagatcgataagg | 166444 | - | see also 1698 line | | |
| 14762 | PGM1 | NM_002633.2 | 455 | acgggatcggtcgcttggttatc | 166468 | - | - | phosphoglucomutase | - |
| 14763 | PKLR | NM_000298.3 | 780 | cagccggaagggcgtgaacttgc | 166509 | - | kinase_related | - | pyruvate kinase deficiency、Gaucher disease、hemolytic anemia |
| 14764 | PP | NM_021129.2 | 647 | gtggactggtttagaaggtataa | 166543 | - | phosphatase | magnesium binding | - |
| 14765 | PPA2 | NM_006903.3 | 154 | cagaattaccgcctcttctttaa | 166549 | - | see also 4841 line | | |
| 14766 | PPAT | NM_002703.3 | 367 | gagttcggtgccaacattcaaat | 166579 | - | - | transferase、transferring glycosyl groups | - |
| 14767 | PPM1E | NM_014906.3 | 1752 | atgatgggcgtgtggattcattc | 166639 | - | phosphatase | - | - |
| 14768 | PRPS1 | NM_002764.2 | 540 | cagagccggctgtcctaaagtgg | 166664 | - | kinase_related | ribose-phosphate pyrophosphokinase | PRPS1 superactivity、arthritis |
| 14769 | PRPS1L1 | NM_175886.2 | 385 | aagagccggtccccaatctctgc | 166668 | - | - | - | - |
| 14770 | PRPS2 | NM_002765.2 | 257 | gagcggctgcggggaaattaacg | 166674 | - | see also 1808 line | | |
| 14771 | PSPH | NM_004577.2 | 346 | tggtggctttaggagtattgtag | 166703 | - | phosphatase | phosphoserine phosphatase | - |
| 14772 | SMPD2 | NM_003080.1 | 1326 | tgggccaccttcgctagctatgt | 166725 | - | - | sphingomyelin phosphodiesterase | - |
| 14773 | BSN | NM_003458.1 | 10406 | gtggaggacgaccgcatttatgg | 166793 | - | see also 2345 line | | |
| 14774 | DLD | NM_000108.2 | 1020 | ttggccgacgaccctttactaag | 166835 | - | - | heavy metal binding | lactoacidosis |
| 14775 | FLJ10511 | NM_018120.3 | 270 | ctgacgctctatcggtagttaac | 166844 | - | - | - | - |
| 14776 | GSR | NM_000637.1 | 759 | cggcatgataaggtacttagaag | 166887 | - | see also 423 line | | |
| 14777 | KIAA0467 | NM_015284.1 | 992 | cagagcgttccctcacacaattc | 166925 | - | see also 6313 line | | |
| 14778 | PITPNM1 | NM_004910.1 | 3363 | cggctacctgatcgtgtatgtca | 166993 | - | see also 3412 line | | |
| 14779 | PKD1 | NM_000296.2 | 3516 | ctgaccgtgctggcatctaatgc | 167000 | - | signal_transduction | lectin | polycystic kidney disease |

Figure 46

| | | | | | | | | | | |
|---|---|---|---|---|---|---|---|---|---|---|
| 14780 | SOD1 | NM_000454.3 | 343 | tggtgtggccgatgtgtctattg | 167035 | - | - | Smad3/Sp-1 heterodimer | atherosclerosis、amyotrophic lateral sclerosis、alcoholism |
| 14781 | TXNRD1 | NM_003330.2 | 1185 | tagacgtcactgttatggttagg | 167055 | - | signal_transduction | - | - |
| 14782 | C5 | NM_001735.2 | 601 | ccgtctaatcctagatatggtat | 167097 | - | see also 1149 line | | |
| 14783 | CCL11 | NM_002986.2 | 380 | gggtgcaggattccatgaagtat | 167257 | - | signal_transduction | chemokine | - |
| 14784 | CCL15 | NM_004167.3 | 793 | aagctgaagccctactcaatata | 167264 | - | signal_transduction | - | - |
| 14785 | CCL16 | NM_004590.2 | 244 | ctgtcacctgccagcaatcatct | 167268 | - | - | chemokine | - |
| 14786 | CCL18 | NM_002988.2 | 184 | aagttcatagttgactattctga | 167276 | - | signal_transduction | chemokine | - |
| 14787 | CCL19 | NM_006274.2 | 303 | cagggtgcctgctgtagtgttca | 167277 | - | signal_transduction | chemokine | - |
| 14788 | CCL2 | NM_002982.2 | 210 | aggctcgcgagctatagaagaat | 167280 | - | kinase_related、apoptosis、gpcr | protein kinase | rheumatoid arthritis、atherosclerosis |
| 14789 | CCL20 | NM_004591.1 | 160 | tggatacacagaccgtattcttc | 167282 | - | signal_transduction | chemokine | - |
| 14790 | CCL22 | NM_002990.3 | 110 | cagcgtctgctgccgtgattacg | 167288 | - | signal_transduction | chemokine | - |
| 14791 | CCL23 | NM_005064.3 | 384 | aagaaggggcgacgtttctgtgc | 167294 | - | signal_transduction、gpcr | - | - |
| 14792 | CCL24 | NM_002991.2 | 240 | gtgggtccagaggtacatgaaga | 167295 | - | signal_transduction | chemokine | - |
| 14793 | CCL25 | NM_005624.2 | 367 | aagttatcatcgtccaagtttag | 167307 | - | signal_transduction、gpcr | - | - |
| 14794 | CCL26 | NM_006072.3 | 211 | tggacctgggtgcgaagctatga | 167310 | - | signal_transduction | chemokine | - |
| 14795 | CCL28 | NM_019846.3 | 141 | ctgttgcacggaggtttcacatc | 167318 | - | - | - | - |
| 14796 | CCL5 | NM_002985.2 | 305 | atgggttcgggagtacatcaact | 167337 | - | signal_transduction | translation regulator | - |
| 14797 | CCL7 | NM_006273.2 | 147 | ttgggattaatacttcaactacc | 167340 | - | signal_transduction | heparin binding | - |
| 14798 | CCL8 | NM_005623.2 | 520 | ttgctcagccagattcagtttcc | 167343 | - | signal_transduction | heparin binding | - |
| 14799 | CX3CL1 | NM_002996.1 | 250 | atgcggcaaacgcgcaatcatct | 167351 | - | signal_transduction | chemokine | atherosclerosis |
| 14800 | CXCL1 | NM_001511.1 | 374 | aagatgctgaacagtgacaaatc | 167359 | - | signal_transduction、gpcr | translation regulator | melanoma |
| 14801 | CXCL10 | NM_001565.1 | 234 | ttgtccacgtgttgagatcattg | 167364 | - | kinase_related、signal_transduction | chemokine | - |
| 14802 | CXCL11 | NM_005409.3 | 116 | tagccttggctgtgatattgtgt | 167369 | - | signal_transduction | chemokine | - |
| 14803 | CXCL12 | NM_000609.3 | 302 | gtgcattgacccgaagctaaagt | 167374 | - | signal_transduction、gpcr | growth factor | resistance to or association with human immunodeficiency virus、Peutz-Jeghers syndrome、melanoma |
| 14804 | CXCL13 | NM_006419.1 | 222 | tagacgcttcattgatcgaattc | 167381 | - | - | chemokine | - |
| 14805 | CXCL14 | NM_004887.3 | 557 | gtgtggacgggtccaaatgcaag | 167387 | - | see also 3391 line | | |
| 14806 | CXCL5 | NM_002994.2 | 256 | cagaccacgcaaggagttcatcc | 167393 | - | signal_transduction | chemokine | - |
| 14807 | CXCL6 | NM_002993.1 | 219 | acgcgttacgctgagagtaaacc | 167397 | - | signal_transduction | heparin binding | - |

Figure 46

| | | | | | | | | | |
|---|---|---|---|---|---|---|---|---|---|
| 14808 | CXCL9 | NM_002416.1 | 102 | aggaaccccagtagtgagaaagg | 167400 | - | signal_transduction、gpcr | chemokine | - |
| 14809 | IL8 | NM_000584.2 | 281 | ctgcgccaacacagaaattattg | 167419 | - | cell_cycle、 signal_transduction、 gpcr | Transcription factor p65 | - |
| 14810 | PPBP | NM_002704.1 | 242 | ctgaactccgctgcatgtgtata | 167422 | - | cell_cycle | growth factor | - |
| 14811 | IFNB1 | NM_002176.1 | 66 | gagctacaacttgcttggattcc | 167429 | - | signal_transduction | Nuclear factor NF-kappa-B p50 subunit | - |
| 14812 | IFNW1 | NM_002177.1 | 964 | gagaatctgctggggcaattagc | 167444 | - | cell_cycle | interferon-alpha/beta receptor ligand | - |
| 14813 | LIF | NM_002309.2 | 243 | atgccctctttattctctattac | 167447 | - | signal_transduction | oncostatin-M receptor ligand | CNS Tumours |
| 14814 | OSM | NM_020530.3 | 136 | aggcagctgctcgaaagagtacc | 167450 | - | - | translation regulator | melanoma |
| 14815 | CTF1 | NM_001330.2 | 135 | ttgcgcacctcctcaccaaatac | 167455 | - | signal_transduction | leukemia inhibitory factor receptor ligand | - |
| 14816 | IL12A | NM_000882.2 | 676 | tggccctgtgccttagtagtatt | 167464 | - | - | interleukin-12 receptor ligand | - |
| 14817 | IL12B | NM_002187.2 | 429 | aagatgcgaggccaagaattatt | 167481 | - | receptor_acitivity | interleukin-12 receptor ligand | diabetes |
| 14818 | IL5 | NM_000879.2 | 420 | atgaacaccgagtggataataga | 167497 | - | - | translation regulator | myelodysplastic syndrome |
| 14819 | CSF2 | NM_000758.2 | 114 | cagccctgggagcatgtgaatgc | 167500 | - | signal_transduction | hematopoietic-associated factor 1A complex | myelodysplastic syndrome |
| 14820 | EPO | NM_000799.1 | 666 | ctgacactttccgcaaactcttc | 167510 | - | signal_transduction | steroid hormone receptor | - |
| 14821 | IL11 | NM_000641.2 | 170 | ctgagcctgtggccagatacagc | 167512 | - | - | interleukin-11 receptor ligand | - |
| 14822 | IL10 | NM_000572.2 | 232 | aggatcagctggacaacttgttg | 167513 | - | apoptosis | interleukin-10 receptor ligand | rheumatoid arthritis、 atherosclerosis |
| 14823 | IL9 | NM_000590.1 | 365 | aggcaacgcgctgacatttctga | 167522 | - | - | interleukin-9 receptor ligand | - |
| 14824 | IL7 | NM_000880.2 | 730 | cagcatcgatcaattattggaca | 167527 | - | see also 573 line | | |
| 14825 | IL6 | NM_000600.1 | 420 | ttggagtttgaggtatacctaga | 167541 | - | signal_transduction | Rel A homodimer | rheumatoid arthritis、 kaposi sarcoma、 non-hodgkin lymphoma、 atherosclerosis、 breast cancer、 ovarian cancer、 prostate cancer、 Alzheimer disease、 renal cell carcinoma、 multiple myeloma、 juvenile rheumatoid arthritis、 Hodgkin's disease |
| 14826 | IL4 | NM_000589.2 | 438 | acggacacaagtgcgatatcacc | 167545 | - | - | - | melanoma |
| 14827 | IL3 | NM_000588.3 | 146 | ctgggttaactgctctaacatga | 167551 | - | - | receptor signaling protein | rheumatoid arthritis、 myelodysplastic syndrome |

Figure 46

| | | | | | | | | | |
|---|---|---|---|---|---|---|---|---|---|
| 14828 | IL2 | NM_000586.2 | 612 | cagcaatatcaacgtaatagttc | 167575 | - | - | Retinoic acid receptor RXR-alpha | severe combined immunodeficiency due to IL2 deficiency |
| 14829 | IFNG | NM_000619.1 | 236 | cagatgtagcggataatggaact | 167583 | - | signal_transduction | FOS-related antigen 1 | rheumatoid arthritis |
| 14830 | IFNA6 | NM_021002.1 | 307 | aggcttctagacaaactctatac | 167592 | - | - | - | - |
| 14831 | IFNA8 | NM_002170.1 | 405 | aggaagtgggggtgatagagtct | 167595 | - | - | - | - |
| 14832 | IFNK | NM_020124.1 | 385 | aagcagagtacctgaaccaatgc | 167602 | - | transcription_regulator、 signal_transduction | - | - |
| 14833 | IFNT1 | NM_176891.2 | 498 | ctgggccattgtccaagtagaaa | 167611 | - | - | - | - |
| 14834 | IL15 | NM_000585.2 | 673 | gagtccggagatgcaagtattca | 167623 | - | signal_transduction | - | - |
| 14835 | ED1 | NM_001399.3 | 1061 | ctggtctcgcatcactatgaacc | 167637 | - | see also 972 line | | |
| 14836 | LTA | NM_000595.2 | 719 | cagccctagtactgtcttctttg | 167645 | - | signal_transduction、a poptosis | | - |
| 14837 | TNF | NM_000594.2 | 736 | ctggtatgagcccatctatctgg | 167656 | - | apoptosis、signal_tran sduction、transcription _regulator | - | - |
| 14838 | TNFSF10 | NM_003810.2 | 140 | ctgcgtgctgatcgtgatcttca | 167660 | - | apoptosis、signal_tran sduction、kinase_relat ed | tumor necrosis factor receptor ligand | arthritis |
| 14839 | TNFSF11 | NM_003701.2 | 376 | atggatcctaatagaatatcaga | 167687 | - | see also 2526 line | | |
| 14840 | TNFSF13 | NM_003808.2 | 1312 | gagactctattccgatgtataag | 167700 | - | signal_transduction | - | rheumatoid arthritis |
| 14841 | TNFSF13B | NM_006573.3 | 289 | gagcagtcacgccttacttcttg | 167706 | - | signal_transduction | tumor necrosis factor receptor ligand | lupus erythematosus、rheumatoid arthritis、systemic lupus erythematosis |
| 14842 | TNFSF15 | NM_005118.2 | 541 | cagagtcgggagactacttcatt | 167719 | - | signal_transduction、c ell_cycle | tumor necrosis factor receptor ligand | - |
| 14843 | TNFSF4 | NM_003326.2 | 653 | atggcggagaactgattcttatc | 167731 | - | signal_transduction | tumor necrosis factor receptor ligand | - |
| 14844 | TNFSF5 | NM_000074.1 | 799 | acgtcctttggcttactcaaact | 167754 | - | apoptosis、signal_tran sduction | translation regulator | atherosclerosis、immunodeficiency |
| 14845 | TNFSF6 | NM_000639.1 | 890 | ctgggggcagtgttcaatcttac | 167765 | - | apoptosis、signal_tran sduction、kinase_relat ed | translation regulator | autoimmune lymphoproliferative syndrome、systemic lupus erythematosis、melanoma |
| 14846 | TNFSF8 | NM_001244.2 | 570 | atgggaatctggtgatccaattc | 167776 | - | signal_transduction、a poptosis | tumor necrosis factor receptor ligand | - |
| 14847 | TNFSF9 | NM_003811.2 | 421 | aggctggagtctactatgtcttc | 167792 | - | apoptosis、signal_tran sduction | tumor necrosis factor receptor ligand | - |
| 14848 | IL1RN | NM_000577.3 | 563 | aaggcgtcatggtcaccaaattc | 167807 | - | - | - | - |

602

EP 1 752 536 A1

Figure 46

| | | | | | | | | | |
|---|---|---|---|---|---|---|---|---|---|
| 14849 | IL18 | NM_001562.2 | 400 | aggaaatcggcctctatttgaag | 167819 | - | apoptosis | interleukin-1 receptor ligand | diabetes、melanoma |
| 14850 | IL1A | NM_000575.3 | 1370 | atgacgccctcaatcaaagtata | 167845 | - | apoptosis、cell_cycle | interleukin-1 receptor ligand | rheumatoid arthritis、Alzheimer disease、osteoarthritis |
| 14851 | IL1B | NM_000576.2 | 167 | atggccctaaacagatgaagtgc | 167867 | - | apoptosis、signal_transduction、cell_cycle | translation regulator | Alzheimer disease、Parkinson disease、melanoma |
| 14852 | IL1F10 | NM_032556.4 | 437 | atggcaagatactacataattaa | 167879 | - | see also 9244 line | | |
| 14853 | IL1F6 | NM_014440.1 | 122 | cagtcactattgccttaatctca | 167890 | - | - | - | - |
| 14854 | IL1F7 | NM_014439.3 | 573 | ctgcacctcctgcaattgtaatg | 167900 | - | - | - | - |
| 14855 | IL1F8 | NM_014438.3 | 124 | cagcacccaaatcctatgctatt | 167909 | - | - | - | - |
| 14856 | IL1F9 | NM_019618.2 | 105 | aagggccgtctatcaatcaatgt | 167926 | - | - | - | - |
| 14857 | INHBC | NM_005538.2 | 585 | ttgtgctgggtccacataatacc | 167944 | - | - | inhibin | - |
| 14858 | TGFB1 | NM_000660.1 | 1677 | gagccctggacaccaactattgc | 167965 | - | apoptosis、cell_cycle、signal_transduction | Smad3/Sp-1 heterodimer | atherosclerosis、diabetes mellitus、Alzheimer disease、diabetes |
| 14859 | TGFB2 | NM_003238.1 | 1092 | tggatgcggcctattgctttaga | 167989 | - | - | AP-1(c-Jun/ATF2 heterodimer) | - |
| 14860 | TGFB3 | NM_003239.1 | 899 | gagtccaacttaggtctagaaat | 168016 | - | signal_transduction、cell_cycle | transforming growth factor-beta receptor ligand | - |
| 14861 | DTR | NM_001945.1 | 857 | aagtgaagttgggcatgactaat | 168036 | - | receptor_acitivity、signal_transduction | receptor | diphtheria toxin sensitivity |
| 14862 | EREG | NM_001432.1 | 342 | gtgtggctcaagtgtcaataaca | 168038 | - | cell_cycle、signal_transduction | growth factor | - |
| 14863 | ECGF1 | NM_001953.2 | 541 | tggctgcaaggtgccaatgatca | 168055 | - | signal_transduction | thymidine phosphorylase | - |
| 14864 | FIGF | NM_004469.2 | 658 | tggaggaactacttcgaattact | 168069 | - | cell_cycle | platelet-derived growth factor receptor ligand | - |
| 14865 | PDGFB | NM_002608.1 | 1131 | tcgatccgctcctttgatgatct | 168099 | - | cell_cycle | - | Ewing's sarcoma、skin cancer、dermatofibrosarcoma protuberans |
| 14866 | VEGFB | NM_003377.3 | 146 | gtgtcatggatagatgtgtatac | 168114 | - | signal_transduction、cell_cycle | vascular endothelial growth factor receptor ligand | neuroblastoma |
| 14867 | MUC4 | NM_004532.2 | 1514 | gggacgacgggacttacgattcc | 168131 | - | - | - | lung squamous cell carcinoma、lung cancer |
| 14868 | KITLG | NM_000899.1 | 270 | caggaatcgtgtgactaataatg | 168198 | - | see also 584 line | | |
| 14869 | CSF1 | NM_000757.3 | 590 | gcgtccgaactttctatgagaca | 168236 | - | - | - | Alzheimer disease |
| 14870 | IL22 | NM_020525.3 | 380 | ctgataggttccagccttatatg | 168253 | - | - | cytokine | - |

EP 1 752 536 A1

Figure 46

| | | | | | | | | | |
|---|---|---|---|---|---|---|---|---|---|
| 14871 | DKFZp451J0118 | NM_175852.3 | 90 | ggggctgccaaacaatccaatcc | 168258 | - | - | high molecular weight B-cell growth factor receptor ligand | - |
| 14872 | AREG | NM_001657.2 | 491 | ctggctatattgtcgatgattca | 168273 | - | - | growth factor | - |
| 14873 | ARS | NM_020427.2 | 127 | gtgcttcctgcaggaccattacc | 168281 | - | - | cytokine | - |
| 14874 | BMP10 | NM_014482.1 | 615 | aagggatcgtatgatatacgatg | 168292 | - | - | growth factor | - |
| 14875 | BMP15 | NM_005448.1 | 1068 | ctgtgtcccgtataagtatgttc | 168335 | - | - | growth factor | amyloidosis |
| 14876 | BMP2 | NM_001200.1 | 980 | gggacacgccaaccatggattcg | 168347 | - | see also 780 line | | |
| 14877 | BMP3 | NM_001201.1 | 1355 | gagccagacgctccaatttgatg | 168383 | - | - | growth factor | - |
| 14878 | BMP5 | NM_021073.2 | 996 | aagagtcggagtactcagtaagg | 168420 | - | - | growth factor | - |
| 14879 | BMP6 | NM_001718.2 | 1053 | ttgttggacacccgtgtagtatg | 168455 | - | - | growth factor | - |
| 14880 | BMP7 | NM_001719.1 | 687 | acgttccggatcagcgtttatca | 168470 | - | - | growth factor | - |
| 14881 | CXCL16 | NM_022059.1 | 1196 | cagatctgccggttcattatata | 168501 | - | - | cytokine | - |
| 14882 | FAM3B | NM_058186.2 | 146 | aaggacgtttgttggttaagtta | 168506 | - | - | cytokine | - |
| 14883 | FAM3C | NM_014888.1 | 262 | atgcaagtttaggaaatctattt | 168519 | - | - | cytokine | - |
| 14884 | FAM3D | NM_138805.2 | 524 | acgtcgtgggccctactatgtgc | 168540 | - | - | - | - |
| 14885 | FLT3LG | NM_001459.2 | 387 | gcgcgtgaacacggagatacact | 168551 | - | signal_transduction | cytokine | - |
| 14886 | GDF10 | NM_004962.2 | 1633 | tggataatctcaccgaaatcttt | 168558 | - | signal_transduction | growth factor | - |
| 14887 | GDF11 | NM_005811.2 | 1063 | ccggccagtgcgagtacatgttc | 168569 | - | - | growth factor | - |
| 14888 | GDF2 | NM_016204.1 | 667 | aagcgtggtcatttatgatgttc | 168577 | - | see also 6723 line | | |
| 14889 | GDF3 | NM_020634.1 | 1097 | aagacatggtagtcgatgaatgt | 168602 | - | - | growth factor | - |
| 14890 | GDF8 | NM_005259.1 | 976 | ctgtcgttaccctctaactgtgg | 168629 | - | see also 3700 line | | |
| 14891 | GDF9 | NM_005260.2 | 1126 | aggtacaaccctcgatactgtaa | 168666 | - | signal_transduction | growth factor | - |
| 14892 | GPI | NM_000175.2 | 410 | ctgcggaaccggtcaaacacacc | 168676 | - | - | growth factor | rheumatoid arthritis、anemia hemolytic |
| 14893 | IL16 | NM_004513.3 | 2126 | atggacctgtcacgattgtcatc | 168721 | - | - | - | - |
| 14894 | IL17 | NM_002190.2 | 228 | aaggtcctcagattactacaacc | 168759 | - | apoptosis | cytokine | rheumatoid arthritis |
| 14895 | IL17C | NM_013278.3 | 437 | ctgtgcagaggctgtatcgatgc | 168761 | - | signal_transduction | cytokine | - |
| 14896 | IL17F | NM_052872.2 | 273 | ccggtacccctcggaagttgtac | 168768 | - | - | - | - |
| 14897 | IL19 | NM_013371.2 | 1410 | gggagagctcgacgtctttctag | 168780 | - | signal_transduction | - | - |
| 14898 | IL20 | NM_018724.2 | 339 | cagacccctgaccattatactct | 168792 | - | - | cytokine | - |
| 14899 | IL21 | NM_021803.1 | 284 | cagaaggcccaactaaagtcagc | 168804 | - | - | - | - |
| 14900 | IL26 | NM_018402.1 | 157 | ctgttgacgctctctatatcaaa | 168813 | - | - | cytokine | - |
| 14901 | MDK | NM_002391.2 | 265 | tggagccgactgcaagtacaagt | 168825 | - | signal_transduction、cell_cycle | heparin binding | - |
| 14902 | MIF | NM_002415.1 | 367 | cagcccggacagggtctacatca | 168827 | - | - | isomerase | rheumatoid arthritis、atherosclerosis、lung cancer |
| 14903 | NODAL | NM_018055.3 | 991 | gtgctcctagatcaccataaaga | 168846 | - | - | - | situs ambiguus |

Figure 46

| ID | Gene | Accession | Length | Sequence | | Process | Molecular function | Disease |
|---|---|---|---|---|---|---|---|---|
| 14904 | PGLYRP | NM_005091.1 | 341 | tggagaagacggctgtatacg | - | receptor_activity | peptidoglycan recognition | - |
| 14905 | PLAB | NM_004864.1 | 899 | gtgtcgctccagacctatgatga | - | signal_transduction | translation regulator | atherosclerosis |
| 14906 | PTN | NM_002825.4 | 566 | ctggagctgagtgcaagcaaacc | - | phosphatase, cell_cycle, signal_transduction | protein phosphatase inhibitor | - |
| 14907 | SCGB1A1 | NM_003357.3 | 151 | atgacacaccctccagttatga | - | signal_transduction | steroid binding | - |
| 14908 | SPP1 | NM_000582.2 | 436 | tcgaacgactctgatgatgtaga | - | apoptosis | integrin binding | arthritis |
| 14909 | THPO | NM_000460.2 | 959 | caggatacacgaactcttgaatg | - | - | growth factor | essential thrombocythemia, leukemia |
| 14910 | TNFRSF11B | NM_002546.2 | 1280 | gggcctaatgcacgcactaaagc | - | receptor_acitivity, apoptosis, signal_transduction | receptor | arthritis |
| 14911 | TNFSF18 | NM_005092.2 | 241 | ctgaacctccttgcgtgaataag | - | apoptosis, signal_transduction | cytokine | - |
| 14912 | BDNF | NM_001709.3 | 510 | cagaaagttcggcccaatgaaga | - | see also 1134 line | | - |
| 14913 | CECR1 | NM_017424.2 | 1253 | tggcgttaagctgccttacttct | - | | | - |
| 14914 | DKK1 | NM_012242.1 | 160 | gggagctaccccggtcttgtcg | - | see also 5219 line | | - |
| 14915 | EDN2 | NM_001956.2 | 522 | gggagcctcggtccacacattcc | - | kinase_related, signal_transduction | toxin | - |
| 14916 | FGF1 | NM_000800.2 | 504 | ttggtttgttggcctccaagaaga | - | | | - |
| 14917 | FGF11 | NM_004112.2 | 799 | agggaaaccgagttaagaagaac | - | signal_transduction | growth factor | prostate cancer |
| 14918 | FGF12 | NM_004113.3 | 303 | atgcaccagatggtaccattga | - | | | - |
| 14919 | FGF13 | NM_004114.2 | 993 | gttgctatccaaggagttcaaac | - | see also 2777 line | | - |
| 14920 | FGF14 | NM_004115.2 | 206 | agggtatagtgaccaggttatat | - | see also 2780 line | | - |
| 14921 | FGF18 | NM_003862.1 | 558 | ctgcacttgcctgtgtttacact | - | signal_transduction | | - |
| 14922 | FGF19 | NM_005117.2 | 1000 | cagggggccacttggaatctgaca | - | | growth factor | - |
| 14923 | FGF2 | NM_002006.2 | 777 | ttgaacgattggaatctaataac | - | see also 1285 line | | - |
| 14924 | FGF20 | NM_019851.1 | 444 | cagtgggactggtcagtattaga | - | signal_transduction | growth factor | - |
| 14925 | FGF21 | NM_019113.1 | 256 | ttgaagccgggagttattcaaat | - | signal_transduction | growth factor | - |
| 14926 | FGF22 | NM_020637.1 | 241 | gtgggcgtggtcatcaaagc | - | | growth factor | - |
| 14927 | FGF23 | NM_020638.2 | 627 | aggaacgagatcccctaattca | - | | growth factor | - |
| 14928 | FGF3 | NM_005247.2 | 824 | cagcggccgagtgcgagttttgtgg | - | signal_transduction, cell_cycle | growth factor | melanoma, head and neck squamous cell carcinoma, squamous cell cancer |
| 14929 | FGF4 | NM_002007.1 | 819 | acgggccacgatcctacagagtac | - | signal_transduction, cell_cycle | growth factor | melanoma, head and neck squamous cell carcinoma, squamous cell cancer |
| 14930 | FGF5 | NM_004464.2 | 477 | tggatcccacgaagccaatatgt | - | see also 3021 line | growth factor | - |

Figure 46

| | | | | | | | | | |
|---|---|---|---|---|---|---|---|---|---|
| 14931 | FGF6 | NM_020996.1 | 163 | caggcaccgtgccaacaacacg | 169162 | - | signal_transduction, cell_cycle | growth factor | - |
| 14932 | FGF7 | NM_002009.2 | 518 | gtgggtactatatctttagcttg | 169170 | - | signal_transduction, cell_cycle | growth factor | - |
| 14933 | FGF8 | NM_006119.2 | 677 | gcgtgaggtccacttcatgaagc | 169185 | - | signal_transduction, cell_cycle | - | breast cancer, prostate cancer |
| 14934 | FRA | NM_024816.1 | 1161 | gtgcctgcaccatgaggtaaagc | 169211 | - | - | - | - |
| 14935 | GDNF | NM_000514.2 | 551 | ttgtgtcttaactgcaatacatt | 169216 | - | see also 398 line | - | - |
| 14936 | GRP | NM_002091.3 | 227 | ctgggcggtggggcacttaatgg | 169229 | - | signal_transduction | growth factor | - |
| 14937 | IGF1 | NM_000618.2 | 181 | cagtcttccaaccaattattta | 169237 | - | - | peptide hormone | hepatocellular carcinoma, thyroid cancer, colorectal cancer, breast cancer, growth retardation with sensorineural deafness and mental retardation, lung cancer, prostate cancer, pygmy dwarfism, colorectal cancers |
| 14938 | INHBE | NM_031479.3 | 720 | ctgggctgcataccttaactct | 169245 | - | - | inhibin | - |
| 14939 | LOC145957 | NM_138573.1 | 388 | ttgtgcggtcctagtaacacttat | 169261 | - | - | - | - |
| 14940 | MIA | NM_006533.1 | 329 | cagcgttcagggagattactatg | 169269 | - | - | growth factor | melanoma |
| 14941 | NDP | NM_000266.1 | 522 | ctgcatgaggcaccactatgtgg | 169274 | - | signal_transduction | growth factor | Coats disease, exudative vitreoretinopathy, Norrie disease (pseudoglioma) |
| 14942 | NGFB | NM_002506.1 | 827 | tggcggtttatccggatagatac | 169285 | - | - | growth factor | - |
| 14943 | NRG1 | NM_013962.1 | 1295 | tggaatcaaacgctacatctaca | 169338 | - | kinase_related | - | schizophrenia |
| 14944 | NRG2 | NM_004883.1 | 689 | cgggtcgggtggcgttggtaaag | 169339 | - | apoptosis, signal_transduction | - | - |
| 14945 | NTF3 | NM_002527.2 | 527 | cggtacgcggagcataagagtca | 169376 | - | see also 1629 line | - | - |
| 14946 | NTF5 | NM_006179.3 | 528 | cagtccctccgccagtacttct | 169387 | - | - | growth factor | - |
| 14947 | OGN | NM_014057.2 | 842 | aagtctacgtgtaattcatcttc | 169428 | - | see also 5474 line | - | - |
| 14948 | PDGFC | NM_016205.1 | 1235 | cagctgcacctcgtaacttct | 169456 | - | cell_cycle | - | - |
| 14949 | PGF | NM_002632.3 | 601 | ctgctgcgcgatgagaatctgc | 169464 | - | signal_transduction, cell_cycle | heparin binding | atherosclerosis, arthritis |
| 14950 | PROK1 | NM_032414.1 | 62 | gtgccacgcgagtctcaatcatg | 169468 | - | cell_cycle | growth factor | - |
| 14951 | RABEP1 | NM_004703.2 | 1705 | tggttatcgtttagttagtgaaa | 169508 | - | see also 3222 line | - | - |
| 14952 | SCDGF-B | NM_025208.2 | 253 | gagccgcatccatcaaagctttgc | 169537 | - | - | - | - |
| 14953 | SCGF | NM_002975.2 | 368 | gagaatctgccgcgaactgttga | 169560 | - | - | - | - |
| 14954 | TFF1 | NM_003225.1 | 241 | gtgcttcatccttaataccatcg | 169563 | - | - | translation regulator | - |
| 14955 | VEGFC | NM_005429.2 | 1414 | tggggccaaccgagaatttgatg | 169583 | - | see also 3790 line | - | breast cancer, pancreatic cancer |

Figure 46

| 14956 | VGF | NM_003378.2 | 707 | caggaactgcgagatttcagtcc | 169593 | - | - | growth factor | - |
|---|---|---|---|---|---|---|---|---|---|
| 14957 | ADCYAP1 | NM_001117.2 | 507 | aaggacgccgaatagcttatttg | 169604 | - | gpcr | neuropeptide hormone | - |
| 14958 | ASMT | NM_004043.1 | 468 | cggtcagcccgacgtcacaatgc | 169611 | - | - | hematopoietic-associated factor 1A complex | - |
| 14959 | CORT | NM_001302.1 | 169 | cggccgagacagcgagcatatgc | 169618 | - | - | neuropeptide hormone | - |
| 14960 | CRH | NM_000756.1 | 383 | ccgcatgggagaggagtacttcc | 169620 | - | signal_transduction | translation regulator | Alzheimer disease |
| 14961 | GAL | NM_015973.2 | 435 | atgcgcacaatcattgagtttct | 169622 | - | signal_transduction | neuropeptide hormone | Alzheimer disease |
| 14962 | GALP | NM_033106.1 | 353 | acgtttgccaaaccagagattgg | 169626 | - | signal_transduction | neuropeptide hormone | - |
| 14963 | NPY | NM_000905.2 | 235 | cggcgctgcgacactacatcaac | 169628 | - | gpcr、receptor_acitivity、signal_transduction | Smad3/Sp-1 heterodimer | atherosclerosis、elevated plasma LDL、alcoholism |
| 14964 | NTS | NM_006183.3 | 513 | aaggaagaagtcataaagagaaa | 169634 | - | signal_transduction | peptide hormone | alcoholism |
| 14965 | TAC1 | NM_003182.1 | 211 | aggagccaatgatgatctgaatt | 169652 | - | see also 2157 line | | |
| 14966 | PTH | NM_000315.2 | 129 | aagacatggctaaagttatgatt | 169671 | - | apoptosis、signal_transduction、gpcr | translation regulator | hyperparathyroidism、hypocalcemia |
| 14967 | PTHLH | NM_002820.2 | 462 | aggggaagtccatccaagattta | 169684 | - | - | VDR/RXR alpha heterodimer | humoral hypercalcemia of malignancy、non-small-cell lung cancer |
| 14968 | GNRH1 | NM_000825.2 | 1241 | cagaaacccaacgcttcgaatgc | 169689 | - | signal_transduction | luteinizing hormone-releasing factor | adrenal hyperplasia、familial idiopathic hypogonadotropic hypogonadism |
| 14969 | TRH | NM_007117.1 | 321 | ccgcgtcccagatctttcaatct | 169693 | - | signal_transduction | thyrotropin-releasing hormone | - |
| 14970 | ADM | NM_001124.1 | 230 | atgtcgcgtcggagtttcgaaag | 169696 | - | signal_transduction | peptide hormone | - |
| 14971 | AGT | NM_000029.1 | 1189 | ctggtgctgcaaggatcttatga | 169702 | - | signal_transduction | USF43/USF44 complex | essential hypertension |
| 14972 | APM1 | NM_004797.1 | 376 | tgggattggagacttacgttact | 169707 | - | - | hormone | atherosclerosis |
| 14973 | CALCA | NM_001741.1 | 383 | acgttcccccaaactgcaattgg | 169714 | - | gpcr | peptide hormone | - |
| 14974 | CCK | NM_000729.2 | 285 | ctggacgaatgtccatcgttaag | 169716 | - | signal_transduction | peptide hormone | Parkinson disease、Ewing's sarcoma、peripheral neuroepithelioma |
| 14975 | CGA | NM_000735.2 | 412 | cggcgtgccactgcagtacttgt | 169726 | - | signal_transduction | Thyroid hormone receptor beta-2 | - |
| 14976 | COPA | NM_004371.2 | 2133 | gggaccacgggatcattcgaact | 169762 | - | see also 2933 line | | |
| 14977 | FLJ14800 | NM_032840.1 | 1098 | ccggaacgtgcggaatgtcatgt | 169816 | - | kinase_related、receptor_acitivity | - | - |

607

EP 1 752 536 A1

Figure 46

| | | | | | | | | | |
|---|---|---|---|---|---|---|---|---|---|
| 14978 | FSHB | NM_000510.1 | 102 | atgtcgtttctgcataagcatca | 169819 | - | signal_transduction | glycopeptide hormone | bladder cancer |
| 14979 | GCG | NM_002054.2 | 474 | aggccgaggaaggcgagatttcc | 169832 | - | see also 1328 line | | |
| 14980 | GH2 | NM_002059.3 | 544 | ctgggcagatcttcaatcagtcc | 169840 | - | - | - | - |
| 14981 | GIP | NM_004123.1 | 400 | tggccagtcaagctaataggaag | 169844 | - | signal_transduction | peptide hormone | - |
| 14982 | GPB5 | NM_145171.2 | 107 | gtgccgtgagggagtttactttc | 169848 | - | - | - | - |
| 14983 | GPHA2 | NM_130769.2 | 280 | cagtggttaccgacacaacatca | 169850 | - | - | - | - |
| 14984 | GRTP1 | NM_024719.1 | 507 | acgaaggctcgaagattatcttc | 169859 | - | - | - | - |
| 14985 | HAMP | NM_021175.1 | 248 | cggctgctgtcatcgatcaaagt | 169870 | - | - | hormone | arthritis |
| 14986 | IAPP | NM_000415.1 | 303 | ttggtgccattctctcatctacc | 169873 | - | apoptosis、signal_transduction | structural molecule | diabetes mellitus、diabetes |
| 14987 | INS | NM_000207.1 | 173 | cggggaacgaggcttcttctaca | 169879 | - | signal_transduction | translation regulator | atherosclerosis、proinsulinemia、diabetes、obesity |
| 14988 | INSL4 | NM_002195.1 | 151 | ctgagccaactccttagagaaag | 169881 | - | signal_transduction | peptide hormone | - |
| 14989 | INSL5 | NM_005478.3 | 406 | atggctgttccatgactgatttg | 169910 | - | - | peptide hormone | - |
| 14990 | INSL6 | NM_007179.2 | 580 | tagcattgcatgtcttccatata | 169922 | - | - | peptide hormone | - |
| 14991 | LEP | NM_000230.1 | 360 | gagaacctccgggatcttcttca | 169932 | - | signal_transduction | hormone | atherosclerosis、diabetes mellitus、obesity、diabetes |
| 14992 | LRSAM1 | NM_138361.2 | 1088 | gggcccacggacagattctcaag | 169940 | - | - | - | - |
| 14993 | MGC45438 | NM_152459.2 | 911 | cagatggaccagtcatgttctgt | 169954 | - | - | - | - |
| 14994 | NPPA | NM_006172.1 | 283 | gtgctcagtgagccgaatgaaga | 169958 | - | - | peptide hormone | - |
| 14995 | PPY | NM_002722.1 | 59 | ctgcgtggctctgttactacagc | 169961 | - | - | peptide hormone | - |
| 14996 | RETNLB | NM_032579.1 | 230 | cagtccctctcctataagcaaga | 169968 | - | - | molecular_function unknown | - |
| 14997 | RLN1 | NM_006911.2 | 209 | aaggacgatgttattaaattatg | 169973 | - | signal_transduction | peptide hormone | - |
| 14998 | SRP | NM_033199.2 | 318 | cggcctcttgcagatcttactgg | 169987 | - | - | adrenocorticotropin | - |
| 14999 | SS18L1 | NM_015558.3 | 944 | gagtccacgcagcactactatga | 169991 | - | see also 6471 line | | |
| 15000 | STC1 | NM_003155.1 | 516 | ttgtacagcgctgctaaatttga | 169995 | - | signal_transduction | glycopeptide hormone | - |
| 15001 | STC2 | NM_003714.1 | 1006 | aagacgaacagtctgagtattct | 170008 | - | signal_transduction | glycopeptide hormone | - |
| 15002 | TG | NM_003235.3 | 888 | cggttcctcgcagttcaatcagt | 170024 | - | see also 2185 line | | |
| 15003 | UCN3 | NM_053049.1 | 371 | tcgacgtccccaccaacatcatg | 170154 | - | - | hormone | - |
| 15004 | UTS2 | NM_006786.2 | 294 | caggatttctctggacaagatcc | 170163 | - | - | - | - |
| 15005 | ADAM11 | NM_002390.2 | 2164 | ggggagacggagagatataaagg | 170184 | - | signal_transduction | - | |
| 15006 | ADAM2 | NM_001464.2 | 404 | tggactcaggggcgtactacagt | 170201 | - | - | metalloendopeptidase | - |
| 15007 | ADAM22 | NM_004194.2 | 760 | tcgtcgatatcctcgtaatgtag | 170276 | - | - | - | - |

Figure 46

| 15008 | ADAM23 | NM_003812.1 | 1913 | gtgaacgagtgtgatattactga | 170390 | - | - | - | - |
|---|---|---|---|---|---|---|---|---|---|
| 15009 | ECM2 | NM_001393.1 | 1608 | atgtcattgtactacgttataac | 170445 | - | - | - | - |
| 15010 | ICAM2 | NM_000873.2 | 116 | aggatcggatgagaaggtattcg | 170463 | - | - | integrin binding | - |
| 15011 | ICAM3 | NM_002162.2 | 1504 | ctgggcgtggtgactatcgtact | 170488 | - | - | integrin binding | - |
| 15012 | ICAM4 | NM_001544.2 | 518 | atggaagccgggtcatctattcc | 170492 | - | - | - | - |
| 15013 | TGFBI | NM_000358.1 | 1347 | ttgcttcggaaccacataattaa | 170521 | - | - | structural molecule | corneal stromal dystrophy |
| 15014 | DOK4 | NM_018110.2 | 596 | gagtggtacaagacactatctgt | 170533 | - | kinase_related | - | - |
| 15015 | DOK5 | NM_018431.2 | 773 | cggcggtatggacgtgatactac | 170557 | - | see also 7469 line | | |
| 15016 | FRS3 | NM_006653.3 | 405 | ccgtccgctggccttatctctgc | 170603 | - | receptor_acitivity、signal_transduction | | - |
| 15017 | IRS4 | NM_003604.1 | 752 | cggcggagccacccttctataaa | 170630 | - | receptor_acitivity、signal_transduction | | - |
| 15018 | MGC20785 | NM_152721.1 | 854 | gcggtccagcagctatggattca | 170706 | - | kinase_related | - | - |
| 15019 | CD4 | NM_000616.2 | 382 | atgatcgcgctgactcaagaaga | 170709 | - | receptor_acitivity、signal_transduction、kinase_related | transmembrane receptor | rheumatoid arthritis、T cell OKT4/CD4 deficiency |
| 15020 | TLR8 | NM_016610.2 | 1061 | ttgacccaacttcgatacctaaa | 170760 | - | receptor_acitivity、kinase_related | | - |
| 15021 | PDYN | NM_024411.2 | 691 | atggagactggcacactctatct | 170846 | - | signal_transduction | - | - |
| 15022 | REA | NM_007273.3 | 404 | ttggttccagtaccccattatct | 170852 | - | receptor_acitivity、transcription_regulator | | - |
| 15023 | BID | NM_001196.2 | 203 | cagggatgagtgcatcacaaacc | 170869 | - | apoptosis | - | - |
| 15024 | GPRC5D | NM_018654.1 | 96 | tggcatcgtggtcacaattctgc | 170876 | - | gpcr、receptor_acitivity、signal_transduction | | - |
| 15025 | BZRAP1 | NM_004758.1 | 2164 | aggtcttcctagcacgttatagc | 170900 | - | - | - | - |
| 15026 | ALCAM | NM_001627.1 | 602 | ttgaaggagcggtggtcataatt | 170941 | - | see also 1080 line | | |
| 15027 | ALS2CR3 | NM_015049.1 | 757 | ctggaactcgctgctcgaattgg | 170987 | - | see also 6208 line | | |
| 15028 | ANGPT1 | NM_001146.3 | 1617 | ccgagcctattcacagtatgaca | 171061 | - | see also 727 line | | |
| 15029 | ANGPT2 | NM_001147.1 | 1233 | atggcatctacacgttaacattc | 171094 | - | signal_transduction | receptor binding | neuroblastoma |
| 15030 | ANGPTL1 | NM_004673.3 | 1125 | caggtggtgccacaacatattcc | 171133 | - | - | - | - |
| 15031 | ANGPTL2 | NM_012098.2 | 596 | gggctcgccaagagagttcattt | 171165 | - | see also 5129 line | | |
| 15032 | APOB | NM_000384.1 | 1468 | acgcggtcaacaactatcataag | 171197 | - | signal_transduction | specific RNA polymerase II transcription factor | hypercholesterolemia、atherosclerosis、acanthocytosis、familial hypobetalipoproteinemia、normotriglyceridemic abetalipoproteinemia |

Figure 46

| 15033 | APOE | NM_000041.1 | 320 | aggagttgaaggcctacaaatcg | 171585 | - | - | translation regulator | atherosclerosis、Alzheimer disease、hyperlipoproteinemia、alcoholism、Parkinson disease、schizophrenia |
|---|---|---|---|---|---|---|---|---|---|
| 15034 | APOF | NM_001638.1 | 970 | gggggtgggccataatcaagagc | 171604 | - | - | receptor binding | - |
| 15035 | APOL2 | NM_030882.2 | 399 | cagagagaatctgctacaactgc | 171607 | - | - | - | Alzheimer disease、schizophrenia |
| 15036 | ASIP | NM_001672.2 | 165 | ttgtggcgctgaacaagaaatcc | 171608 | - | signal_transduction | receptor binding | obesity |
| 15037 | C17 | NM_018659.1 | 205 | caggctgtacctggacatacaca | 171610 | - | signal_transduction | - | - |
| 15038 | C3 | NM_000064.1 | 3450 | tggtggattacggaacaacaacg | 171649 | - | signal_transduction、gpcr | receptor binding | nephritis membranoproliferative |
| 15039 | CD1D | NM_001766.2 | 1456 | gggctttacctcccggtttaaga | 171683 | - | receptor_acitivity | receptor binding | - |
| 15040 | CD72 | NM_001782.1 | 804 | ctgtccgtcgggatggataatgc | 171688 | - | receptor_acitivity | receptor binding | - |
| 15041 | CD80 | NM_005191.2 | 754 | ctggctgaagtgacgttatcagt | 171709 | - | receptor_acitivity、transcription_regulator、signal_transduction | receptor binding | rheumatoid arthritis |
| 15042 | DBI | NM_020548.2 | 171 | aagcaactgtgggcgacataaat | 171726 | - | - | receptor binding | pathologic anxiety |
| 15043 | EFNA1 | NM_004428.2 | 617 | cagaggtgcgggttctacatagc | 171739 | - | see also 2962 line | | |
| 15044 | ENSA | NM_004436.1 | 469 | ttgcgggtggccaagttgaatga | 171740 | - | - | - | - |
| 15045 | F2R | NM_001992.2 | 1482 | aggtacgtctacagtatcttatg | 171772 | - | receptor_acitivity、gpcr、apoptosis、signal_transduction、cell_cycle、kinase_related | thrombin receptor | - |
| 15046 | F2RL1 | NM_005242.3 | 1001 | aagagggccatcaaactcattgt | 171788 | - | receptor_acitivity、gpcr、signal_transduction | thrombin receptor | arthritis |
| 15047 | GFRA3 | NM_001496.2 | 783 | cagatcacgcctggtggatttcc | 171800 | - | - | receptor binding | - |
| 15048 | GLG1 | NM_012201.1 | 1294 | tggattaccgacgcatgttgatg | 171832 | - | see also 5191 line | | |
| 15049 | GLMN | NM_007070.2 | 616 | cagtgttgcaaggccttaataga | 171913 | - | - | - | - |
| 15050 | ICOSL | NM_015259.2 | 425 | aggacgagcagaagtttcactgc | 171950 | - | signal_transduction | receptor binding | - |
| 15051 | NMU | NM_006681.1 | 210 | ctgccgaggtgctccaatattac | 171955 | - | signal_transduction | receptor binding | - |
| 15052 | PLXNC1 | NM_005761.1 | 4451 | cagatcctgacgtcgtacatatt | 172065 | - | see also 4018 line | | |
| 15053 | SELPLG | NM_003006.2 | 929 | cagcaatttgtccgtcaactacc | 172081 | - | - | receptor binding | - |
| 15054 | STOML2 | NM_013442.1 | 335 | ctgtgactctcgacaatgtaact | 172086 | - | - | - | - |
| 15055 | TAC3 | NM_013251.1 | 359 | acgtgacatgcatgacttctttg | 172102 | - | signal_transduction | tachykinin | - |
| 15056 | WNT5A | NM_003392.3 | 456 | gtggtcgctaggtatgaataacc | 172110 | - | signal_transduction | receptor binding | - |
| 15057 | WNT7A | NM_004625.3 | 514 | tggacgagtgtcagtttcagttc | 172121 | - | signal_transduction | receptor binding | - |
| 15058 | ZP3 | NM_007155.4 | 548 | aggactaaccgcgcagagattcc | 172123 | - | receptor_acitivity | coreceptor | - |
| 15059 | ARHGEF2 | NM_004723.2 | 1788 | aggcttacctgcggcgaattaag | 172143 | - | see also 3238 line | | |

Figure 46

| # | Gene | Accession | Length | Sequence | ID | - | Note | Function | Disease |
|---|------|-----------|--------|----------|-----|---|------|----------|---------|
| 15060 | CHN1 | NM_001822.2 | 722 | atgacgataaacccaatttatga | 172169 | - | see also 1177 line | | |
| 15061 | CHN2 | NM_004067.1 | 673 | atgacaactaacccatccatga | 172216 | - | | diacylglycerol binding | |
| 15062 | DGKD | NM_003648.2 | 178 | tcgagggcgaacgctttacatg | 172243 | - | kinase_related, signal transduction | | |
| 15063 | DGKQ | NM_001347.2 | 988 | gcgacgacgcggtgagaagaagc | 172295 | - | kinase_related | diacylglycerol kinase | |
| 15064 | VAV2 | NM_003371.2 | 2086 | ctgagcgcttgcaataagcatc | 172327 | - | | guanyl-nucleotide release factor | |
| 15065 | VAV3 | NM_006113.3 | 2422 | ttgccatcgctcggtatgacttc | 172401 | - | see also 4249 line | | |
| 15066 | ACBD3 | NM_022735.3 | 1170 | tggaacacgacctcagatcaaga | 172430 | - | see also 8441 line | | |
| 15067 | ACBD4 | NM_024722.1 | 643 | gggacccattggacgatataag | 172440 | - | | | |
| 15068 | ACBD5 | NM_145698.1 | 1587 | gtgctaagtttgccatcatatg | 172472 | - | see also 9795 line | | |
| 15069 | ACBD6 | NM_032360.2 | 505 | sggcagttattagttctctata | 172484 | - | see also 9172 line | | |
| 15070 | PECI | NM_006117.1 | 588 | tagtgggaatgatctgactaact | 172510 | - | see also 4254 line | | |
| 15071 | NDUFAB1 | NM_005003.2 | 267 | aggaccgtgtcttacgtattg | 172527 | - | | acyl carrier | |
| 15072 | CPNE1 | NM_003915.2 | 599 | aagctacgctttggaatctatga | 172531 | - | see also 2673 line | | |
| 15073 | CPNE3 | NM_003909.1 | 1258 | gaggcgatcggtcttgtcttcc | 172602 | - | see also 2668 line | | |
| 15074 | PEPP2 | NM_019012.2 | 1913 | atgccaagttaagccgattatgt | 172682 | - | see also 7661 line | | |
| 15075 | PIK3R1 | NM_181504.2 | 319 | tggtacgaagatgcgtctactaa | 172719 | - | kinase_related | | |
| 15076 | PLEKHA4 | NM_020904.1 | 1891 | caggagctgcggcaacttaagaga | 172803 | - | | | |
| 15077 | PSCD3 | NM_004227.3 | 253 | cagagatcgacaatctaacttcc | 172810 | - | receptor_activivity | phosphatidylinositol binding | |
| 15078 | TULP3 | NM_003324.2 | 1271 | atgaccctgattatatagtcatg | 172841 | - | transcription_regulator, signal_transduction, gpcr | phosphatidylinositol-4, 5-bisphosphate binding | |
| 15079 | DAPP1 | NM_014395.1 | 722 | cagctgtacaattcgattattca | 172855 | - | see also 5658 line | | |
| 15080 | LYPLA3 | NM_012320.2 | 447 | aagcagcgtgggttcctatttcc | 172866 | - | | | |
| 15081 | OSBPL1A | NM_018030.3 | 1457 | ctgctgtgtgcataatatcattg | 172900 | - | | | |
| 15082 | PAFAH2 | NM_000437.2 | 762 | cagcgggtaagcgagtgtttacg | 172970 | - | | phospholipid binding | atherosclerosis |
| 15083 | PLA2G7 | NM_005084.2 | 446 | agggcacctcttgcgtttatat | 172995 | - | | phospholipid binding | platelet-activating factor acetylhydrolase deficiency |
| 15084 | PLEKHA1 | NM_021622.2 | 1901 | ttggtgggtacccatcattact | 173038 | - | see also 8151 line | | |
| 15085 | PLEKHA3 | NM_019091.2 | 760 | aagatagccaatgccaagtttaa | 173067 | - | | | |
| 15086 | RASAL1 | NM_004658.1 | 558 | ctgagcaggagaggcgattacagc | 173073 | - | | phospholipid binding | |
| 15087 | SDPR | NM_004657.3 | 589 | ccgcgcaacatttcaaagtgc | 173092 | - | | | |
| 15088 | SEC14L2 | NM_012429.1 | 261 | ctgccgaatccagatgactattt | 173100 | - | transcription_regulator | translation regulator | |

Figure 46

| 15089 | SYT1 | NM_005639.1 | 942 | gggtggcttatccgatccttatg | 173131 | - | see also 3914 line | | |
|---|---|---|---|---|---|---|---|---|---|
| 15090 | FABP2 | NM_000134.2 | 256 | tggtgtcacctttaattacaacc | 173142 | - | - | lipid transporter | diabetes mellitus、diabetes |
| 15091 | FABP3 | NM_004102.2 | 79 | gtggacagcaagaatttcgatga | 173146 | - | - | lipid transporter | - |
| 15092 | FABP4 | NM_001442.1 | 390 | gtggtggaatgcgtcatgaaagg | 173158 | - | - | transporter | - |
| 15093 | FABP6 | NM_001445.1 | 194 | tgggatctccagcgatgtaatcg | 173163 | - | - | transporter | - |
| 15094 | RBP3 | NM_002900.1 | 3574 | cggaggagttcacctatatcgtg | 173185 | - | see also 1939 line | | |
| 15095 | ALB | NM_000477.3 | 881 | gggcggaccttgccaagtatatc | 173202 | - | - | Hepatocyte nuclear factor 1 | atherosclerosis、analbuminemia、dysalbuminemic hyperthyroxinemia、hyperzincemia |
| 15096 | APOA4 | NM_000482.2 | 200 | cagtgatgtgggactacttcagc | 173229 | - | - | lipid transporter | atherosclerosis |
| 15097 | APOA5 | NM_052968.2 | 979 | tcgccccagagtttcaacaaaca | 173239 | - | - | - | - |
| 15098 | ATP5G1 | NM_005175.1 | 164 | ctgttgtaccaggggtctaatca | 173250 | - | - | lipid binding | - |
| 15099 | ATP5G2 | NM_005176.3 | 60 | atgttcgcctgctccaagtttgt | 173253 | - | - | lipid binding | - |
| 15100 | ATP5G3 | NM_001689.1 | 372 | gaggggctctacggtatttaatgg | 173257 | - | - | lipid binding | - |
| 15101 | BPI | NM_001725.1 | 614 | ctgcgcttcgaaacaagatgaac | 173273 | - | - | lipid binding | - |
| 15102 | BPIL1 | NM_025227.1 | 294 | ttgagctacgtgtctgaaattgg | 173291 | - | - | - | - |
| 15103 | BPIL2 | NM_174932.1 | 839 | ttggaatcgccgagtatttcttt | 173313 | - | - | - | - |
| 15104 | BPIL3 | NM_174897.1 | 598 | atgggcaccgtcaaatatgttct | 173344 | - | see also 10124 line | | |
| 15105 | C20orf114 | NM_033197.2 | 1118 | aagcatcgggctgatcaatgaaa | 173366 | - | - | - | - |
| 15106 | CETP | NM_000078.1 | 1451 | cggctcgaggtagtgtttacagc | 173396 | - | - | Smad3/Sp-1 heterodimer | atherosclerosis |
| 15107 | CRABP2 | NM_001878.2 | 367 | tggatgggaggccctgtaagagc | 173407 | - | - | signal_transduction、transcription_regulator | transporter |
| 15108 | CRBPIV | NM_052960.1 | 107 | ctggccctaggtattgactttgc | 173410 | - | - | - | transporter | - |
| 15109 | EDG3 | NM_005226.2 | 797 | tcgagcggcacttgacaatgatc | 173415 | - | see also 3663 line | | |
| 15110 | EDG5 | NM_004230.1 | 821 | tcgccgtctccaccctgaattcc | 173435 | - | - | receptor_acitivity、kinase_related、signal_transduction、gpcr | - | - |
| 15111 | EDG6 | NM_003775.1 | 251 | tcgcgacgctgggtctactattg | 173439 | - | - | receptor_acitivity、signal_transduction、gpcr | - | - |
| 15112 | EDG7 | NM_012152.1 | 695 | acgtcttgtctccgcatacaagt | 173447 | - | - | receptor_acitivity、gpcr | - | - |
| 15113 | FABP1 | NM_001443.1 | 347 | ccgaactcaacggcgacataatc | 173458 | - | - | lipid transporter | - |
| 15114 | FABP7 | NM_001446.3 | 400 | gtgaccaaaccaacggtaattat | 173462 | - | - | lipid binding | - |
| 15115 | LBP | NM_004139.2 | 954 | atgataccgcctgactctaatat | 173492 | - | - | lipid binding | - |
| 15116 | LIPF | NM_004190.1 | 867 | ttggatgtgtatctatcacataa | 173523 | - | - | triacylglycerol lipase | - |

Figure 46

| | | | | | | | | | |
|---|---|---|---|---|---|---|---|---|---|
| 15117 | PCTP | NM_021213.1 | 458 | gggtgatccgggtgaagcaatac | 173616 | - | - | phosphatidylcholine transporter | - |
| 15118 | PITPN | NM_006224.2 | 638 | tggaagccgtatatatagacatt | 173632 | - | see also 4317 line | | |
| 15119 | PITPNB | NM_012399.2 | 307 | gcgtacccctactgtagaacaat | 173651 | - | - | phosphatidylinositol transporter | - |
| 15120 | PLIN | NM_002666.1 | 214 | gagtggcacctgcgaatgcttcc | 173656 | - | - | lipid binding | diabetes |
| 15121 | PLTP | NM_006227.2 | 1248 | ctgcgcaggttccgaatctattc | 173670 | - | - | - | atherosclerosis |
| 15122 | PMP2 | NM_002677.2 | 367 | gagaggatcactgaatcaagtgc | 173679 | - | - | transporter | - |
| 15123 | PSAP | NM_002778.1 | 742 | ctggcatggccgacatatgcaag | 173684 | - | - | lipid binding | combined SAP deficiency (deficiency of SAP1 and SAP2)(-)、 Gaucher disease、 metachromatic leukodystrophy |
| 15124 | RBP1 | NM_002899.2 | 259 | aggacggtgaccatatgatcatc | 173695 | - | - | transporter | - |
| 15125 | RBP2 | NM_004164.2 | 216 | tagcacattccgcaactatgatg | 173703 | - | - | transporter | - |
| 15126 | RRAGC | NM_022157.2 | 1178 | ctgaaagcgctgacacacaatgg | 173742 | - | see also 8315 line | | |
| 15127 | SCP2 | NM_002979.2 | 1606 | atgggtctcgctatgaagttaca | 173780 | - | - | sterol carrier | Zellweger syndrome |
| 15128 | SEC14L3 | NM_174975.2 | 448 | ttgagaccatcgtgatgatattt | 173795 | - | - | - | - |
| 15129 | SEC14L4 | NM_174977.2 | 643 | aagaatttaattgttattcgagc | 173814 | - | - | - | - |
| 15130 | STAR | NM_000349.1 | 168 | cagacacatgcgcaacatgaagg | 173815 | - | - | transcription co-activator | lipoid congenital adrenal hyperplasia |
| 15131 | STARD3 | NM_006804.2 | 1394 | gcggccaccatgtttgaatttgc | 173835 | - | see also 4784 line | | |
| 15132 | STARD4 | NM_139164.1 | 299 | cagggccttgtcgtttggattgg | 173842 | - | - | - | - |
| 15133 | STARD6 | NM_139171.1 | 255 | caggattgattcggacacattca | 173869 | - | - | - | - |
| 15134 | CD209 | NM_021155.2 | 1098 | ctggaacgacgacaaatgtaatc | 173896 | - | receptor_acitivity | - | - |
| 15135 | LMAN2L | NM_030805.1 | 160 | gggtcaaacgttcgagtacttga | 173903 | - | - | - | - |
| 15136 | MBL2 | NM_000242.1 | 109 | tggcagcgtcttactcagaaact | 173921 | - | - | opsonin | recurrent infections、 predisposition(-) |
| 15137 | APCS | NM_001639.2 | 356 | ttggagagtatagtctatacatt | 173939 | - | - | structural molecule | juvenile rheumatoid arthritis |
| 15138 | ASGR1 | NM_001671.2 | 701 | ctgggctgacgccgacaactact | 173944 | - | receptor_acitivity | lectin | - |
| 15139 | ATRN | NM_012070.2 | 2424 | gggtcggccttcggaagatcaat | 174014 | - | see also 5106 line | | |
| 15140 | BCAN | NM_021948.3 | 824 | agggctctgcccgctatgctttc | 174047 | - | - | - | - |
| 15141 | C14orf27 | NM_175060.1 | 1002 | cagctcccgatctcagttacttg | 174061 | - | - | - | - |
| 15142 | C21orf63 | NM_058187.3 | 1573 | gggaactgtcggggttctgtagg | 174094 | - | - | lectin | - |
| 15143 | CD207 | NM_015717.1 | 801 | tggattggcctgactaaagcagg | 174113 | - | - | - | - |
| 15144 | CD209L | NM_014257.2 | 1105 | gtggctggaacgacaatcgatgt | 174117 | - | receptor_acitivity | - | - |
| 15145 | CD22 | NM_001771.1 | 560 | tgggtatccgatccaattgcagt | 174129 | - | - | lectin | - |
| 15146 | CD33 | NM_001772.1 | 1067 | aggacacctccaccgaatactca | 174167 | - | receptor_acitivity、 signal_transduction | receptor | leukemia |

Figure 46

| | | | | | | | | | |
|---|---|---|---|---|---|---|---|---|---|
| 15147 | CD69 | NM_001781.1 | 287 | caggccaatacacattctcaatg | 174172 | - | receptor_acitivity、sig nal_transduction | transmembrane receptor | - |
| 15148 | CHODL | NM_024944.2 | 862 | tgggggtccctacctttaccagt | 174181 | - | - | lectin | - |
| 15149 | CHST4 | NM_005769.1 | 1037 | cagacttcccgaatgtatgaatt | 174205 | - | - | - | - |
| 15150 | CLEC1 | NM_016511.2 | 363 | aggaaatacgtcccaagagttgc | 174219 | - | - | - | - |
| 15151 | CLEC2 | NM_016509.1 | 574 | gaggatggctcggttatctcaga | 174237 | - | receptor_acitivity、sig nal_transduction | - | - |
| 15152 | CLECSF1 | NM_005752.1 | 482 | atggtcacggaaggcaagtttgt | 174248 | - | - | lectin | - |
| 15153 | CLECSF11 | NM_130441.1 | 1021 | atgagcgttgtgcgataataaat | 174268 | - | - | - | - |
| 15154 | CLECSF12 | NM_022570.3 | 194 | tggatatactcaattacacttcg | 174274 | - | receptor_acitivity | - | - |
| 15155 | CLECSF13 | NM_006344.1 | 489 | cagcagcttggaagaaacgatag | 174304 | - | - | - | - |
| 15156 | CLECSF13 | NM_173535.1 | 546 | aaggatgccactacattgagttt | 174323 | - | - | - | - |
| 15157 | CLECSF2 | NM_005127.2 | 860 | ccgacctaactataattgacaac | 174356 | - | - | lectin | - |
| 15158 | CLECSF5 | NM_013252.2 | 679 | gtgcgaccattggcctaacaaag | 174374 | - | receptor_acitivity、sig nal_transduction | - | - |
| 15159 | CLECSF6 | NM_016184.2 | 848 | gagcgctgcgttgtgctaaattt | 174384 | - | receptor_acitivity、sig nal_transduction | - | - |
| 15160 | CLECSF8 | NM_080387.4 | 596 | ctggatagacggctttcctattt | 174406 | - | - | - | - |
| 15161 | CLECSF9 | NM_014358.1 | 608 | gtggacggcacacctttgacaaa | 174422 | - | - | - | - |
| 15162 | CLL-1 | NM_138337.2 | 365 | aagcatgtttcacgtaactttga | 174429 | - | receptor_acitivity | - | - |
| 15163 | COLEC10 | NM_006438.2 | 604 | aggattcggggtggaatgctagc | 174462 | - | - | - | - |
| 15164 | COLEC12 | NM_030781.2 | 686 | aggcgtataatggctatgtcacg | 174488 | - | receptor_acitivity | - | - |
| 15165 | CSAD | NM_015989.2 | 738 | gagctctggggacggaatcttct | 174526 | - | - | sulfinoalanine decarboxylase | - |
| 15166 | DCL-1 | NM_014880.3 | 664 | ccgcaccccaatcaccttataat | 174549 | - | receptor_acitivity | - | - |
| 15167 | DGCR2 | NM_005137.1 | 953 | ctgctacgagaagtcttcatttc | 174553 | - | see also 3612 line | | |
| 15168 | DTX2 | NM_020892.1 | 2016 | ctgcgggaccatcctcatagttt | 174570 | - | - | - | - |
| 15169 | FCN3 | NM_003665.2 | 707 | atgacgctgaccacgattcaagc | 174576 | - | - | - | - |
| 15170 | GCKR | NM_001486.2 | 282 | gagactctacagcgaatccattc | 174582 | - | - | sugar binding | - |
| 15171 | GFPT1 | NM_002056.1 | 577 | ttgccaagctcgttaagtatatg | 174615 | - | see also 1329 line | | |
| 15172 | GFPT2 | NM_005110.1 | 217 | gtggcgatcgatgggaataatca | 174655 | - | see also 3581 line | | |
| 15173 | ITLN1 | NM_017625.2 | 1010 | cagcagccgtgagataactgagg | 174706 | - | - | - | - |
| 15174 | ITLN2 | NM_080878.2 | 767 | ccgtatggtcaacgggaatttgt | 174714 | - | - | - | - |
| 15175 | KLRB1 | NM_002258.1 | 490 | ctgatacgtgacaaagcaattct | 174742 | - | receptor_acitivity、sig nal_transduction | | - |
| 15176 | KLRC1 | NM_002259.2 | 503 | gtggtaacgatagttgttattcc | 174758 | - | receptor_acitivity、sig nal_transduction | | - |

Figure 46

| 15177 | KLRC4 | NM_013431.1 | 473 | tggagtactggagcagaacaatt | 174760 | - | receptor_acitivity、signal_transduction | receptor | - |
|---|---|---|---|---|---|---|---|---|---|
| 15178 | KLRD1 | NM_002262.2 | 708 | aagaactgcatagcgtataatcc | 174775 | - | receptor_acitivity、signal_transduction | - | - |
| 15179 | KLRF1 | NM_016523.1 | 573 | acgtatggattgggcttaacttt | 174799 | - | receptor_acitivity、signal_transduction | - | - |
| 15180 | KLRG1 | NM_005810.2 | 250 | ccgctggatgaaatatggtaacc | 174812 | - | receptor_acitivity、signal_transduction | - | - |
| 15181 | KLRK1 | NM_007360.1 | 757 | aagaggaccaggatttacttaaa | 174830 | - | - | - | - |
| 15182 | LGALS1 | NM_002305.2 | 373 | cagatggatacgaattcaagttc | 174844 | - | apoptosis、kinase_related | galactose binding lectin | breast cancer |
| 15183 | LGALS2 | NM_006498.1 | 32 | gggggaacttgaggttaagaaca | 174847 | - | - | galactose binding lectin | - |
| 15184 | LGALS4 | NM_006149.2 | 186 | aagcggttcttcgtgaactttgt | 174851 | - | - | galactose binding lectin | - |
| 15185 | LGALS8 | NM_006499.2 | 1100 | tggacgaactgtcgtcgttaaag | 174879 | - | - | galactose binding lectin | - |
| 15186 | LGALS9 | NM_002308.2 | 926 | tggtcagcacctgtttgaatact | 174887 | - | kinase_related | - | - |
| 15187 | LLT1 | NM_013269.1 | 171 | ttgctgctttaagcgcaataaga | 174895 | - | receptor_acitivity、signal_transduction | - | - |
| 15188 | LMAN1 | NM_005570.2 | 1361 | gagggacatagataacttagtgc | 174942 | - | see also 3869 line | | |
| 15189 | LMAN1L | NM_021819.1 | 52 | gtggtccaggtcccttattctgc | 174958 | - | - | | |
| 15190 | LMAN2 | NM_006816.1 | 777 | cggcgacctgtctgacaatcatg | 174977 | - | - | protein transporter | - |
| 15191 | LOC143903 | NM_178834.2 | 1179 | tagaggtctacaatgtcataaga | 174992 | - | - | - | - |
| 15192 | LPHN1 | NM_014921.1 | 1084 | gggcggaaagaccgacattgacc | 175008 | - | - | - | - |
| 15193 | LPHN2 | NM_012302.1 | 936 | tggcgaggccataattaactatg | 175060 | - | receptor_acitivity、gpcr、signal_transduction | - | - |
| 15194 | LPHN3 | NM_015236.1 | 1204 | ttgatttgcggactaggataaag | 175192 | - | - | - | - |
| 15195 | LY6G5C | NM_025262.1 | 325 | gagcagatgagtgattgttcaaa | 175296 | - | - | - | - |
| 15196 | LY75 | NM_002349.1 | 4608 | aagctgtcccgtcttacatattc | 175444 | - | see also 1518 line | | |
| 15197 | MAG | NM_002361.2 | 1199 | gagcaacccggaccctattctca | 175477 | - | - | lectin | neuropathy |
| 15198 | MRC2 | NM_006039.1 | 1442 | cggcctcaacgatttgaaactgc | 175493 | - | kinase_related、receptor_acitivity | - | - |
| 15199 | NALP9 | NM_176820.2 | 1408 | acgacccaaagacgatcctaacc | 175535 | - | - | - | - |
| 15200 | NPTX2 | NM_002523.1 | 806 | gaggcaatagcgcctttaagtca | 175561 | - | see also 1620 line | | |
| 15201 | NRIP3 | NM_020645.1 | 441 | caggctgcctatataatctcatc | 175584 | - | - | - | - |

Figure 46

| | | | | | | Function | Description | Disease |
|---|---|---|---|---|---|---|---|---|
| 15202 | OLR1 | NM_002543.2 | 248 | gtgtctgacctcctaacacaaga | - | receptor_acitivity | | atherosclerosis、Alzheimer disease |
| 15203 | PLA2R1 | NM_007366.2 | 1001 | aggaggtacgctgttaagtatta | - | receptor_acitivity | - | - |
| 15204 | PRG2 | NM_002728.3 | 447 | tgtttccatccacaacttcaat | - | - | translation regulator | - |
| 15205 | PRG3 | NM_006093.2 | 472 | ttgcagaagctcagaatgtctgc | - | - | - | - |
| 15206 | REG4 | NM_032044.2 | 527 | gagaagccagccgatatggattg | - | - | - | - |
| 15207 | SELE | NM_000450.1 | 1236 | gagcgaggctacatgaattgtct | - | | Transcription factor p65 | atherosclerosis |
| 15208 | SELL | NM_000655.2 | 519 | atgacgctgccacaaactaaag | - | - | lectin | - |
| 15209 | SELP | NM_003005.2 | 1270 | ctgtccacggaagcatggattgc | - | - | Nuclear factor NF-kappa-B p50 subunit | atherosclerosis、myocardial infarction |
| 15210 | SFTPD | NM_003019.2 | 738 | gaggccttacagggacaagtaca | - | - | surfactant | - |
| 15211 | SIGLEC10 | NM_033130.2 | 480 | gagaggaagctatgtgagatata | - | - | - | - |
| 15212 | SIGLEC11 | NM_052884.1 | 1353 | tggagctgccaccattcaaatg | - | - | - | - |
| 15213 | SIGLEC6 | NM_001245.2 | 1363 | cagttccagacagggcatagtttc | - | - | - | - |
| 15214 | SIGLEC7 | NM_014385.1 | 264 | gggccagggaatgatataagctgg | - | receptor_acitivity | - | - |
| 15215 | SIGLEC8 | NM_014442.1 | 406 | aagagggataagggtcatattt | - | receptor_acitivity、signal_transduction | | - |
| 15216 | SIGLECL1 | NM_033329.1 | 733 | ctgtccgactcaacatatcctat | - | - | - | - |
| 15217 | SN | NM_023068.2 | 2768 | cagagggacctcatatcgtttgg | - | - | lectin | - |
| 15218 | TNA | NM_003278.1 | 203 | atgtttgaacacacaaagatgttt | - | - | lectin | - |
| 15219 | ZDHHC19 | NM_144637.2 | 846 | cagcaactggtatttaacaattt | - | - | - | - |
| 15220 | CHIA | NM_021797.1 | 1027 | cagcccattgagccaataactgc | - | - | - | - |
| 15221 | CHIT1 | NM_003465.1 | 1307 | gggaacggtccagcttctacagc | - | - | - | atherosclerosis、chitotriosidase deficiency |
| 15222 | TACR1 | NM_001058.2 | 740 | gagtcgtgtgcatgatcgaatgg | - | see also 650 line | - | - |
| 15223 | TACR2 | NM_001057.1 | 622 | ctgccgctcgcggtgatgtttgt | - | see also 649 line | - | - |
| 15224 | TACR3 | NM_001059.1 | 689 | caggtatatggctatattgatc | - | gpcr、receptor_acitivity、signal_transduction | tachykinin receptor | - |
| 15225 | NPY1R | NM_000909.3 | 1434 | aagcaagcccagtgcatttaaa | - | gpcr、receptor_acitivity、y | neuropeptide Y receptor | - |
| 15226 | NPY2R | NM_000910.2 | 1053 | ccgggagtattcgctgattgaga | - | see also 592 line | - | - |
| 15227 | NPY5R | NM_006174.2 | 190 | tagagctcgacgagtattataac | - | see also 4270 line | - | - |
| 15228 | GPR66 | NM_006056.2 | 547 | ccgcacgctactgtttgagatgg | - | receptor_acitivity、gpcr、signal_transduction | | - |
| 15229 | NMU2R | NM_020167.3 | 1266 | gagctgacgaagatataggtcc | - | receptor_acitivity、gpcr、channel | - | - |

Figure 46

| | | | | | | | | | |
|---|---|---|---|---|---|---|---|---|---|
| 15230 | GPR74 | NM_004885.1 | 663 | ctggccataagtgatttactagt | 176150 | - | receptor_acitivity、gpcr、signal_transduction | - | - |
| 15231 | HCRTR2 | NM_001526.2 | 514 | gtgttcgtcgtggctctcattgg | 176184 | - | receptor_acitivity、gpcr、signal_transduction | orexin receptor | - |
| 15232 | OT7T022 | NM_022146.1 | 549 | ccgtgaggagcaccacttcatgg | 176221 | - | gpcr、receptor_acitivity、signal_transduction | neuropeptide receptor | - |
| 15233 | SORCS3 | NM_014978.1 | 1302 | gtgcggaccacggatggatatgc | 176236 | - | receptor_acitivity、signal_transduction | - | - |
| 15234 | OPRM1 | NM_000914.1 | 849 | aggcaaggttccatagattgtac | 176326 | - | receptor_acitivity、gpcr、signal_transduction | mu-opioid receptor | alcoholism |
| 15235 | OPRK1 | NM_000912.2 | 620 | tcgtgatcatccgatacacaaag | 176338 | - | gpcr、receptor_acitivity、signal_transduction | kappa-opioid receptor | - |
| 15236 | OPRD1 | NM_000911.2 | 454 | tcgtccggtacactaagatgaag | 176357 | - | gpcr、receptor_acitivity | delta-opioid receptor | alcoholism、melanoma |
| 15237 | GPR7 | NM_005285.1 | 338 | tggctatcgaccagtacaacacc | 176363 | - | receptor_acitivity、gpcr、signal_transduction | opioid receptor | - |
| 15238 | OGFR | NM_007346.2 | 251 | gtgtaggtatcggcacaactatc | 176368 | - | receptor_acitivity | - | - |
| 15239 | OPCML | NM_002545.2 | 423 | acgtcccgggttcacctaatagt | 176383 | - | receptor_acitivity | opioid receptor | - |
| 15240 | MC2R | NM_000529.1 | 192 | tagcttggccatatctgatatgc | 176397 | - | gpcr、receptor_acitivity | transcription co-activator | familial glucocorticoid deficiency、adrenocortical cancer |
| 15241 | MC1R | NM_002386.2 | 1054 | tcgccgtggaccgctacatctcc | 176408 | - | gpcr、receptor_acitivity | melanocyte stimulating hormone receptor | melanoma、dysplastic nevus syndrome |
| 15242 | MC3R | NM_019888.2 | 1022 | tggaattgcgcaacacctttagg | 176418 | - | gpcr、receptor_acitivity | melanocortin receptor | diabetes mellitus、diabetes、obesity |
| 15243 | MC4R | NM_005912.1 | 490 | aagcgggttgggatcatcataag | 176435 | - | see also 4133 line | | |
| 15244 | SSTR1 | NM_001049.2 | 1024 | tggacgcggtcaacatgttcacc | 176460 | - | gpcr、receptor_acitivity | somatostatin receptor | neuroblastoma |
| 15245 | SSTR2 | NM_001050.1 | 523 | ttgcccatcatgatatatgctgg | 176479 | - | gpcr、receptor_acitivity | somatostatin receptor | neuroblastoma、small cell lung cancer |
| 15246 | SSTR3 | NM_001051.1 | 603 | ctggcgagccggcttcatcatct | 176486 | - | gpcr、receptor_acitivity、apoptosis | somatostatin receptor | - |

Figure 46

| | | | | | | | | | |
|---|---|---|---|---|---|---|---|---|---|
| 15247 | AVPR2 | NM_000054.2 | 1045 | gaggatgacgctagtgattgtgg | 176490 | - | gpcr、receptor_acitivity、signal_transduction | vasopressin receptor | diabetes insipidus |
| 15248 | OXTR | NM_000916.3 | 1539 | aggaagcctcggccttcatcatc | 176494 | - | receptor_acitivity、gpcr | vasopressin receptor | - |
| 15249 | F2RL2 | NM_004101.2 | 1215 | ttgtgcctgggtagtcttaatag | 176532 | - | receptor_acitivity、gpcr、signal_transduction | thrombin receptor | - |
| 15250 | GP1BA | NM_000173.1 | 631 | gagaactcgctgtatacaatacc | 176539 | - | receptor_acitivity、signal_transduction | thrombin receptor | giant platelet syndrome (Bernard-Soulier macrothrombocytopenia)、von Willebrand pseudohemophilia |
| 15251 | FPR1 | NM_002029.3 | 889 | cagtcagaatccgtgagttattg | 176564 | - | receptor_acitivity、gpcr、kinase_related、signal_transduction | N-formyl peptide receptor | - |
| 15252 | FPRL1 | NM_001462.1 | 1205 | gtgatcgtcggaccttggattct | 176572 | - | gpcr、receptor_acitivity、signal_transduction | N-formyl peptide receptor | |
| 15253 | FPRL2 | NM_002030.3 | 1098 | tgggtcgtaacttccaagaaaga | 176599 | - | gpcr、receptor_acitivity、signal_transduction | N-formyl peptide receptor | - |
| 15254 | GALR1 | NM_001480.2 | 1702 | caggaaggcctataaacaagtgt | 176609 | - | receptor_acitivity、gpcr、signal_transduction | galanin receptor | - |
| 15255 | GALR2 | NM_003857.2 | 1218 | cagcatcctgacggttgatgtgg | 176615 | - | receptor_acitivity、gpcr、signal_transduction | galanin receptor | - |
| 15256 | AGTR1 | NM_000685.3 | 1174 | ttgattcaactaggcatcatacg | 176644 | - | gpcr、receptor_acitivity、signal_transduction | - | Alzheimer disease、essential hypertension |
| 15257 | AGTR2 | NM_000686.3 | 1095 | ctgcgttaatccgtttctgtatt | 176680 | - | see also 429 line | | |
| 15258 | MAS1 | NM_002377.2 | 273 | ttgtctatcgactatgctttaga | 176690 | - | gpcr、receptor_acitivity、signal_transduction | angiotensin type II receptor | - |
| 15259 | EDNRA | NM_001957.1 | 669 | atggctcaatgcacaactattgc | 176709 | - | gpcr、receptor_acitivity、signal_transduction | endothelin receptor | Alzheimer disease |
| 15260 | EDNRB | NM_000115.1 | 633 | gcgaaacggtcccaatatcttga | 176739 | - | see also 97 line | | |

Figure 46

| | | | | | | | | | |
|---|---|---|---|---|---|---|---|---|---|
| 15261 | CCKAR | NM_000730.1 | 670 | atgactccgtaccccatttatag | 176776 | - | gpcr, receptor_acitivity | cholecystokinin receptor | diabetes mellitus, alcoholism, diabetes |
| 15262 | CCKBR | NM_176875.2 | 328 | cggagccgggacacgagaattgg | 176788 | - | - | phosphatidylinositol 3-kinase, class I, regulator | |
| 15263 | BDKRB1 | NM_000710.2 | 1120 | cagctcctgatccagtaattt | 176810 | - | receptor_acitivity, gpcr, signal transduction | bradykinin receptor | |
| 15264 | BDKRB2 | NM_000623.2 | 1058 | acgagcgcatcatcgatgtaatc | 176817 | - | receptor_acitivity, gpcr, signal transduction, kinase_related | bradykinin receptor | |
| 15265 | BRS3 | NM_001727.1 | 656 | ctgcgtctggatcgtgtcttatga | 176842 | - | see also 1148 line | | |
| 15266 | NMBR | NM_002511.1 | 998 | atgtatcggtctttcaactataa | 176882 | - | receptor_acitivity, gpcr | bombesin receptor | |
| 15267 | C5R1 | NM_001736.1 | 673 | acgctcacgatttgttacacttt | 176898 | - | receptor_acitivity, gpcr, kinase_related, signal transduction | C5a anaphylatoxin receptor | |
| 15268 | C3AR1 | NM_004054.2 | 1090 | tcgtggcaataacgatcactagg | 176932 | - | gpcr, receptor_acitivity, signal transduction | complement component C3a receptor | |
| 15269 | HCRTR1 | NM_001525.1 | 1097 | gcgtcctcaatgtccttaagagg | 176941 | - | receptor_acitivity, gpcr, signal transduction | orexin receptor | |
| 15270 | NTSR1 | NM_002531.1 | 536 | acgtgaacaccgacatcactcc | 176942 | - | gpcr, receptor_acitivity, signal transduction | neurotensin receptor, G-protein coupled | |
| 15271 | NTSR2 | NM_012344.2 | 1064 | cagagccatcgtggtcatgtag | 176957 | - | gpcr, receptor_acitivity, signal transduction | neurotransmitter:sodium symporter | |
| 15272 | GRPR | NM_005314.1 | 1107 | ctgatccagagtgcttacatct | 176982 | - | receptor_acitivity, signal transduction, gpcr | bombesin receptor | autism, non-small-cell lung cancer |
| 15273 | NPR3 | NM_000908.1 | 1283 | gagaccgatatgggattctct | 177000 | - | receptor_acitivity, gpcr | peptide receptor, G-protein coupled | |
| 15274 | BRAP | NM_006768.2 | 1158 | atgtcagtcgacatgcttataag | 177044 | - | see also 4773 line | | |
| 15275 | KPNA3 | NM_002267.2 | 1647 | atgtaataccaccgttcgtaat | 177077 | - | see also 1454 line | | |
| 15276 | KPNB2 | NM_002270.2 | 283 | aagatcattgagtggtcttatct | 177088 | - | see also 1464 line | | |
| 15277 | TNPO2 | NM_013433.2 | 1385 | atgctctgtccgactggaatttg | 177151 | - | - | | |

Figure 46

| | | | | | | | | |
|---|---|---|---|---|---|---|---|---|
| 15278 | KDELR1 | NM_006801.2 | 436 | aagttcaaagtacttacgatgg | - | receptor_acitivity | protein transporter | - |
| 15279 | KDELR2 | NM_006854.2 | 563 | gcgtagtccagaccatcctatac | - | receptor_acitivity | protein transporter | - |
| 15280 | PEX7 | NM_000288.1 | 853 | ctggcctcttgctgtatgatt | - | see also 253 line | | - |
| 15281 | PXR1 | NM_000319.3 | 581 | ccgatcgctgatgatgaatat | - | see also 266 line | | - |
| 15282 | SSR2 | NM_003145.2 | 208 | ctgcattagacgtggaactatct | - | see also 2126 line | | - |
| 15283 | SSR3 | NM_007107.2 | 250 | atgaccctagtaagcacatatt | - | see also 4952 line | | - |
| 15284 | TOMM34 | NM_006809.4 | 405 | ctgcggcgagcatctgcttatga | - | | | - |
| 15285 | NKTR | NM_005385.2 | 4092 | acgcctagtcggatgatgatag | - | see also 3746 line | | - |
| 15286 | PPIH | NM_006347.2 | 87 | aggaagttggccgcatgaagatc | - | | | - |
| 15287 | AAMP | NM_001087.1 | 300 | tagcgagtcaccttgcattgc | - | | heparin binding | - |
| 15288 | APOH | NM_000042.1 | 308 | atggagccgtacgctatacgact | - | | heparin binding | - |
| 15289 | COL5A1 | NM_000093.2 | 1747 | gggagaaccacgcgattatcgagc | - | see also 68 line | | - |
| 15290 | HBP17 | NM_005130.1 | 422 | gagagagtcattggaaacaagt | - | signal_transduction | | - |
| 15291 | HRG | NM_000412.2 | 121 | aagtctagacctgatcaataaa | - | | heparin binding | thrombophilia due to HRG deficiency |
| 15292 | LAMC2 | NM_005562.1 | 1796 | gtgtcgagcttgcaactgtaacc | - | see also 3866 line | | - |
| 15293 | LIPC | NM_000236.1 | 240 | ctgtcagattcgaatcaatcatc | - | | triacylglycerol lipase | atherosclerosis, hepatic lipase deficiency |
| 15294 | LIPG | NM_006033.1 | 525 | atggacgatgagcggtatctttg | - | | triacylglycerol lipase | atherosclerosis |
| 15295 | LPL | NM_000237.1 | 1370 | ttggagaactactcagttgaag | - | | lipoprotein lipase | atherosclerosis, hyperlipoproteinemia |
| 15296 | ODZ1 | NM_014253.1 | 6569 | cagtggcgttatagttacgatct | - | see also 5559 line | | - |
| 15297 | SERPINC1 | NM_000488.1 | 254 | aggccaattccgcgctttgctcc | - | | Thyroid hormone receptor beta-2 | thrombophilia due to antithrombin III deficiency |
| 15298 | SERPIND1 | NM_000185.2 | 951 | aggatcctgggtgaataaattcc | - | | serpin | thrombophilia due to heparin cofactor II deficiency |
| 15299 | SERPINE2 | NM_006216.2 | 528 | gttggctaacgccgtgttgttaa | - | | serpin | - |
| 15300 | VTN | NM_000638.2 | 1820 | aagtactaccgagtcaatcttcg | - | | heparin binding | - |
| 15301 | HAPLN3 | NM_178232.2 | 1172 | gagccgccttgacggtgtttact | - | | | - |
| 15302 | HMMR | NM_012484.1 | 239 | aagttaagtcttcggaatcaaag | - | | | - |
| 15303 | IMPG2 | NM_016247.1 | 3477 | gtgtctgagccgtgatccatagg | - | see also 6751 line | | - |
| 15304 | TM6SF2 | NM_023002.1 | 661 | gcgcgagcggctcagtgcaatacc | - | | | - |
| 15305 | TNFAIP6 | NM_007115.2 | 261 | gcggccatctgcaacttaacaag | - | signal_transduction | | - |
| 15306 | XLKD1 | NM_006691.2 | 475 | atggattcgtggtcatctctagg | - | | damaged DNA binding | - |
| 15307 | DSPG3 | NM_004950.2 | 964 | aggcactagaggacattcgattg | - | | | - |
| 15308 | HABP2 | NM_004132.2 | 228 | cagctacgaggattataatcagg | - | | | - |
| 15309 | PODLX2 | NM_015720.1 | 393 | gagaaggcaggttccattgaaga | - | | | - |
| 15310 | TGFBR3 | NM_003243.1 | 1886 | atggtgtgtctactactcctcc | - | receptor_acitivity, signal_transduction | receptor | - |
| 15311 | GABRA1 | NM_000806.3 | 397 | cagccgtcattacaagatgaact | - | see also 493 line | | - |

Figure 46

| | | | | | | | | | |
|---|---|---|---|---|---|---|---|---|---|
| 15312 | GABRA2 | NM_000807.1 | 1283 | aggcttccgttatgatacagaac | 178377 | - | channel、receptor_acitivity、signal_transduction | benzodiazepine receptor | Parkinson disease |
| 15313 | GABRA4 | NM_000809.2 | 990 | cagttcccgctaggactgtattt | 178438 | - | see also 499 line | | |
| 15314 | GABRA5 | NM_000810.2 | 1352 | ttgccacggtcaattactttacc | 178475 | - | see also 502 line | | |
| 15315 | GABRA6 | NM_000811.1 | 988 | tcgcagctgtcaactactttacc | 178517 | - | see also 503 line | | |
| 15316 | GABRB1 | NM_000812.2 | 1001 | aagcgattgatatttatctgatg | 178548 | - | see also 504 line | | |
| 15317 | GABRB3 | NM_000814.3 | 1496 | aggatcgtgtttccattcacttt | 178616 | - | see also 511 line | | |
| 15318 | GABRD | NM_000815.2 | 759 | gcggcgtgtacatcatccaatcc | 178622 | - | channel、receptor_acitivity、signal_transduction | GABA-A receptor | - |
| 15319 | GABRE | NM_004961.2 | 1425 | ctgcatggtccccgattgtgagg | 178657 | - | channel、receptor_acitivity、signal_transduction | - | - |
| 15320 | GABRG1 | NM_173536.2 | 807 | ttgtagggttacggaactcaact | 178691 | - | channel、receptor_acitivity、signal_transduction | - | alcoholism |
| 15321 | GABRG2 | NM_000816.2 | 592 | tggtccagtgaacgctatcaata | 178715 | - | see also 512 line | | |
| 15322 | GABRG3 | NM_033223.1 | 160 | aagacaccgacgtgactcttatt | 178751 | - | see also 9381 line | | |
| 15323 | GABRP | NM_014211.1 | 986 | ttggagtgacgaccgtgttatca | 178785 | - | channel、receptor_acitivity | GABA-A receptor | - |
| 15324 | GABRQ | NM_018558.1 | 213 | agggtgctgtcaagatacgatgt | 178806 | - | see also 7524 line | | |
| 15325 | GABRR1 | NM_002042.2 | 357 | aggttgacatggactttacgatg | 178860 | - | channel、receptor_acitivity、signal_transduction | GABA-A receptor | - |
| 15326 | GABRR2 | NM_002043.1 | 180 | atgcccaagccaagtcacttata | 178873 | - | channel、receptor_acitivity、signal_transduction | GABA-A receptor | - |
| 15327 | GABBR1 | NM_001470.1 | 742 | gtgtacatcggggcactgtttcc | 178884 | - | see also 1017 line | | |
| 15328 | CHRM1 | NM_000738.2 | 1289 | tggctccgaagtggtgatcaaga | 178923 | - | see also 479 line | | |
| 15329 | CHRM2 | NM_000739.1 | 514 | gaggatggggagtgctacattca | 178937 | - | gpcr、receptor_acitivity | muscarinic acetylcholine receptor | - |
| 15330 | CHRM3 | NM_000740.1 | 202 | gtggtcttcatcgctttcttaac | 178958 | - | receptor_acitivity、gpcr、signal_transduction | muscarinic acetylcholine receptor | - |

Figure 46

| 15331 | CHRM5 | NM_012125.2 | 322 | gggattcttaccacaatgcaacc | 178981 | - | gpcr、 receptor_acitivity | muscarinic acetylcholine receptor | - |
|-------|--------|-------------|------|-------------------------|--------|---|--------------------------|-----------------------------------|---|
| 15332 | CHRNA1 | NM_000079.1 | 123 | ccgtctggtggcaaagctattta | 178994 | - | channel、 receptor_acitivity、 signal_transduction | nicotinic acetylcholine-activated cation-selective channel | myasthenic syndrome |
| 15333 | CHRNA2 | NM_000742.1 | 1962 | ctggaaggtgtgcactacattgc | 179010 | - | channel、 receptor_acitivity、 signal_transduction | nicotinic acetylcholine-activated cation-selective channel | - |
| 15334 | CHRNA3 | NM_000743.2 | 717 | cggttcctggtcctacgataagg | 179020 | - | channel、 receptor_acitivity、 signal_transduction | nicotinic acetylcholine-activated cation-selective channel | - |
| 15335 | CHRNA4 | NM_000744.2 | 1933 | gtggagggcgtccagtacattgc | 179045 | - | channel、 receptor_acitivity、 signal_transduction | nicotinic acetylcholine-activated cation-selective channel | - |
| 15336 | CHRNA5 | NM_000745.2 | 639 | ctggactccaccggcaaactaca | 179061 | - | channel、 receptor_acitivity、 signal_transduction | nicotinic acetylcholine-activated cation-selective channel | - |
| 15337 | CHRNA6 | NM_004198.2 | 302 | ctgcgtcacatctggaatgatta | 179099 | - | channel、 receptor_acitivity、 signal_transduction | nicotinic acetylcholine-activated cation-selective channel | - |
| 15338 | CHRNA7 | NM_000746.2 | 357 | aggggtgaagactgttcgtttcc | 179129 | - | see also 480 line | | |
| 15339 | CHRNB1 | NM_000747.1 | 1048 | ttgggtccgtcagatcttcattc | 179139 | - | channel、 receptor_acitivity、 signal_transduction | nicotinic acetylcholine-activated cation-selective channel | - |
| 15340 | CHRNB2 | NM_000748.1 | 926 | acgtggacatcacgtatgacttc | 179164 | - | channel、 receptor_acitivity、 signal_transduction | nicotinic acetylcholine-activated cation-selective channel | epilepsy |
| 15341 | CHRNB4 | NM_000750.1 | 771 | cagctacgtggacgtgacttacg | 179170 | - | channel、 receptor_acitivity、 signal_transduction | nicotinic acetylcholine-activated cation-selective channel | - |

Figure 46

| | | | | | | | | | |
|---|---|---|---|---|---|---|---|---|---|
| 15342 | CHRND | NM_000751.1 | 86 | ctgatccggcacctgtttcaaga | 179175 | - | channel、receptor_acit ivity、signal_transduct ion | nicotinic acetylcholine-activated cation-selective channel | myasthenic syndrome |
| 15343 | CHRNE | NM_000080.2 | 239 | tggaatcgattggcaggattacc | 179185 | - | see also 49 line | | |
| 15344 | CHRNA10 | NM_020402.2 | 409 | cggccagacatcgtactctataa | 179205 | - | channel、receptor_acit ivity | nicotinic acetylcholine receptor-associated protein | - |
| 15345 | CHRNA9 | NM_017581.1 | 197 | cagattacgctctctcagattaa | 179216 | - | channel、receptor_acit ivity | nicotinic acetylcholine-activated cation-selective channel | - |
| 15346 | CHRNB3 | NM_000749.2 | 254 | atgggtccgccctgtattacatt | 179258 | - | channel、receptor_acit ivity、signal_transduct ion | nicotinic acetylcholine-activated cation-selective channel | - |
| 15347 | GLRA1 | NM_000171.1 | 297 | atgtacagcttcaatactcttcg | 179286 | - | channel、receptor_acit ivity、signal_transduct ion | glycine-inhibited chloride channel | startle disease、diabetes |
| 15348 | GLRA3 | NM_006529.1 | 676 | gagacgaccatggattacagagt | 179313 | - | see also 4563 line | | |
| 15349 | GLRB | NM_000824.2 | 1430 | ttgaactatccaattatgactgc | 179375 | - | see also 522 line | | |
| 15350 | HTR3C | NM_130770.1 | 466 | cagcagtgaaggtcgaattaagt | 179397 | - | channel、receptor_acit ivity | - | - |
| 15351 | C20orf79 | NM_178483.1 | 336 | gcggtggaccattgatctgaaga | 179408 | - | - | - | - |
| 15352 | HSD17B4 | NM_000414.1 | 1353 | aggatccggtgtagtgattatta | 179449 | - | - | sterol transporter | D-bifunctional protein deficiency(-) |
| 15353 | STOML1 | NM_004809.3 | 296 | ctggtttgccctgaagattgtgc | 179466 | - | see also 3343 line | | |
| 15354 | ALDH1A1 | NM_000689.3 | 474 | cagggccgtacaataccaattga | 179492 | - | - | electron transporter | alcoholism |
| 15355 | SCGB2A1 | NM_002407.1 | 328 | ttggtgtaatatgaagagtaatt | 179536 | - | - | androgen binding | - |
| 15356 | SHBG | NM_001040.2 | 259 | ggggataccaaccctaaggatga | 179540 | - | - | androgen binding | - |
| 15357 | OSBP | NM_002556.2 | 1256 | acgaacgagccacactctttagg | 179551 | - | - | oxysterol binding | - |
| 15358 | OSBPL5 | NM_020896.2 | 1786 | tcgagtgtgcgaagaacaacttc | 179592 | - | see also 8019 line | | |
| 15359 | PGRMC1 | NM_006667.2 | 545 | gggagtctcagttcactttcaag | 179594 | - | receptor_acitivity | steroid binding | - |
| 15360 | PGRMC2 | NM_006320.1 | 647 | aagataccaaggatcacaataaa | 179598 | - | receptor_acitivity | steroid hormone receptor | - |
| 15361 | SCGB2A2 | NM_002411.1 | 209 | tagacgacaatgccactacaaat | 179600 | - | - | steroid binding | - |
| 15362 | SERPINA6 | NM_001756.2 | 976 | aggtggacctgtacattccaaag | 179611 | - | - | steroid binding | transcortin deficiency |
| 15363 | STE | NM_005420.2 | 662 | gagtccacgtgtactatttcttt | 179630 | - | - | steroid binding | - |
| 15364 | TTR | NM_000371.1 | 169 | tggccgtgcatgtgttcagaaag | 179642 | - | - | - | - |

EP 1 752 536 A1

623

Figure 46

| 15365 | DKFZp547 H025 | NM_020161.1 | 445 | gtgagggagggagcatgtaatga | 179647 | - | - | - | - |
|---|---|---|---|---|---|---|---|---|---|
| 15366 | FOLR1 | NM_000802.2 | 255 | atgtttcctacctatatagattc | 179652 | - | receptor_acitivity | - | ovarian cancer |
| 15367 | FOLR3 | NM_000804.2 | 762 | ccgtctcgtgggattattgattc | 179662 | - | receptor_acitivity | folic acid binding | - |
| 15368 | FTCD | NM_006657.1 | 242 | ccgggtagcttcccgacttatcg | 179665 | - | - | glutamate formimidoyltransferase | - |
| 15369 | GNMT | NM_018960.2 | 587 | aagaacatctactataagagtga | 179673 | - | - | glycine N-methyltransferase | - |
| 15370 | SLC19A2 | NM_006996.1 | 1055 | cagaccgtctccttgtattgaaa | 179709 | - | see also 4881 line | | |
| 15371 | SLC19A3 | NM_025243.2 | 431 | gtggcctactacgcctacatata | 179750 | - | see also 8887 line | | |
| 15372 | RLBP1 | NM_000326.3 | 1250 | gggcacgctgcccaagtatgatg | 179791 | - | see also 275 line | | |
| 15373 | SLC5A6 | NM_021095.1 | 1609 | tgggctgctttgtctaggaatgg | 179802 | - | - | sodium dependent multivitamin transporter | - |
| 15374 | TTPA | NM_000370.1 | 557 | ctgctgtacttacggattcattt | 179825 | - | - | transporter | atherosclerosis、ataxia |
| 15375 | FKBP3 | NM_002013.2 | 905 | aggtcggagtaggcaaagttatc | 179853 | - | see also 1291 line | | |
| 15376 | FKBP4 | NM_002014.2 | 668 | ttgcactggaagggtactacaag | 179864 | - | - | cyclophilin-type peptidy-prolyl cis-trans isomerase | - |
| 15377 | FKBP5 | NM_004117.2 | 289 | acgccgatgattggagacaaagt | 179869 | - | see also 2782 line | | |
| 15378 | FKBP6 | NM_003602.2 | 903 | aaggagcaacccttcaatcatga | 179905 | - | - | cyclophilin-type peptidy-prolyl cis-trans isomerase | - |
| 15379 | PPID | NM_005038.1 | 185 | ttggtcgaattgtcttagaattg | 179909 | - | - | cyclophilin-type peptidy-prolyl cis-trans isomerase | - |
| 15380 | APOL5 | NM_030642.1 | 81 | gtggcttcgaaaggtaatctacg | 179948 | - | - | high-density lipoprotein binding | schizophrenia |
| 15381 | ANKRA2 | NM_023039.2 | 1302 | ctgtcgttggcctgtagtaaagg | 179979 | - | - | - | - |
| 15382 | SCARF1 | NM_003693.2 | 845 | tggccgctgcaaacacaatgagc | 179993 | - | receptor_acitivity | - | - |
| 15383 | LAG3 | NM_002286.4 | 1383 | ctgtcacattggcaatcatcaca | 180008 | - | - | antigen binding | - |
| 15384 | LILRA3 | NM_006865.2 | 345 | gggcggtattgctgtatctatgg | 180013 | - | receptor_acitivity | - | - |
| 15385 | SLAMF1 | NM_003037.1 | 207 | caggtgggcgcatgatgaactgc | 180016 | - | receptor_acitivity | transmembrane receptor | rheumatoid arthritis |
| 15386 | OTC | NM_000531.3 | 998 | cagtattggctcgagtgtataaa | 180056 | - | see also 402 line | | |
| 15387 | PP591 | NM_025207.2 | 1241 | aggacactatcaagaggtataat | 180081 | - | - | - | - |
| 15388 | DIO1 | NM_000792.2 | 329 | gggagtttatgcaaggtaatagg | 180092 | - | - | thyroxine deiodinase | hyperthyroxinemia |
| 15389 | DIO2 | NM_000793.2 | 763 | gcgcccactagtggtcaactttg | 180101 | - | - | - | - |

Figure 46

| | | | | | | | | | |
|---|---|---|---|---|---|---|---|---|---|
| 15390 | SELENBP1 | NM_003944.2 | 714 | ctgtacgggagccacttatatgt | 180116 | - | - | selenium binding | - |
| 15391 | 15.Sep | NM_004261.2 | 322 | gagtgataaacccaaactgttca | 180123 | - | - | selenium binding | - |
| 15392 | SEPP1 | NM_005410.1 | 453 | atgtggccgtcttgtatatcatc | 180139 | - | - | selenium binding | - |
| 15393 | ERAF | NM_016633.1 | 135 | ctgaatcagcaggtcttcaatga | 180168 | - | - | - | - |
| 15394 | HP | NM_005143.1 | 391 | atggagtgtacaccttaaacaat | 180171 | - | - | trypsin | - |
| 15395 | HPR | NM_020995.2 | 737 | ctgaccaatacgattgcataacg | 180177 | - | - | trypsin | - |
| 15396 | PLUNC | NM_016583.2 | 784 | tggtgcatgacattgttaacatg | 180190 | - | - | - | - |
| 15397 | TLR4 | NM_003266.2 | 1475 | tgggacaaccagcctaaagtatt | 180234 | - | receptor_acitivity、 signal_transduction、 kinase_related | - | rheumatoid arthritis、 atherosclerosis |
| 15398 | NOP5/NOP58 | NM_015934.3 | 1143 | tagacgggatacccctaagtatg | 180320 | - | see also 6576 line | | |
| 15399 | AKR1C2 | NM_001354.3 | 120 | aagctctagaggccgtcaaattg | 180327 | - | - | oxidoreductase | - |
| 15400 | ALOX5AP | NM_001629.2 | 360 | aagtactttgtcggttacctagg | 180333 | - | - | enzyme activator | atherosclerosis |
| 15401 | AMBP | NM_001633.2 | 1015 | ctgcatgggcaacggtaacaact | 180349 | - | see also 1082 line | | |
| 15402 | CGI-69 | NM_016016.1 | 346 | ctgtacctgtgcccaaatggtgc | 180353 | - | - | binding | - |
| 15403 | CRP | NM_000567.1 | 341 | ttggtctaaggatataggataca | 180443 | - | - | translation regulator | coronary heart disease、 colorectal cancer、 atherosclerosis、 colorectal cancers |
| 15404 | DKFZP434N1235 | NM_031291.1 | 891 | gtgaggctaaacggcaatataaa | 180479 | - | see also 9004 line | | |
| 15405 | DKFZp547P234 | NM_153045.2 | 414 | ctgttctccggattgacaatacc | 180489 | - | - | - | - |
| 15406 | DOC2A | NM_003586.1 | 129 | gcgatcgcatgaccatcaacatc | 180501 | - | - | - | - |
| 15407 | FLJ10618 | NM_018155.1 | 697 | gtgttcgtaaagtgtatcagaca | 180524 | - | - | - | - |
| 15408 | FLJ12687 | NM_024917.4 | 1372 | gtggcgcagtactatgaagtatt | 180549 | - | see also 8805 line | | |
| 15409 | FLJ20551 | NM_017875.1 | 1239 | atggcgtggacggtgtatgaaga | 180595 | - | - | - | - |
| 15410 | GIF | NM_005142.2 | 359 | gagaccctggggataaagtatcc | 180603 | - | see also 3613 line | | |
| 15411 | HEBP1 | NM_015987.2 | 487 | aagtctggttccggattccaaac | 180641 | - | - | - | - |
| 15412 | HPX | NM_000613.1 | 995 | agggaggctatacccctagtaagc | 180651 | - | - | translation regulator | - |
| 15413 | LOC153328 | NM_145282.1 | 543 | gaggtgaacacaggatgactaca | 180659 | - | - | - | - |
| 15414 | LOC283130 | NM_182556.1 | 562 | ctgtcctgtttggggtctatagc | 180667 | - | - | - | - |
| 15415 | LUZP1 | NM_033631.1 | 1877 | aagacaacacgaacgtttagtga | 180723 | - | see also 9429 line | | |
| 15416 | MCFP | NM_018843.1 | 1251 | aggcctaattcctcgcttaatta | 180792 | - | see also 7599 line | | |
| 15417 | MFTC | NM_030780.2 | 636 | aaggtgtgcgtggattatataag | 180814 | - | see also 8944 line | | |
| 15418 | MGC34725 | NM_173637.1 | 897 | cggctacctgcccaatatgctcg | 180832 | - | - | - | - |

Figure 46

| 15419 | MSCP | NM_016612.1 | 356 | gcgaggcgtcaacgtcatgatca | 180834 | - | - | - | - |
|---|---|---|---|---|---|---|---|---|---|
| 15420 | MTCH1 | NM_014341.1 | 418 | gtgcaagtggatggtaagatagg | 180849 | - | channel、apoptosis、signal_transduction | - | - |
| 15421 | NALP14 | NM_176822.2 | 310 | tggtcggcccagactataggacc | 180859 | - | - | - | - |
| 15422 | SEC14L1 | NM_003003.1 | 2282 | aggtctcttcgcacaagtgtaaa | 180962 | - | see also 2015 line | | |
| 15423 | SFRS16 | NM_007056.1 | 415 | cggacgaacggaagtgtaactac | 180978 | - | - | - | - |
| 15424 | SLC25A1 | NM_005984.1 | 846 | gggcctggaggcgcacaaatacc | 180992 | - | - | citrate transporter | - |
| 15425 | SLC25A11 | NM_003562.3 | 951 | aagggcttcacgccgtactatgc | 181010 | - | see also 2369 line | | |
| 15426 | SLC25A14 | NM_003951.2 | 584 | aagcttgaagcgcttattcgtag | 181022 | - | see also 2699 line | | |
| 15427 | SLC25A15 | NM_014252.1 | 354 | gggtaccagtccagcactaatcg | 181055 | - | - | ornithine transporter | hyperornithinemia-hyperammonemia-homocitrullinuria |
| 15428 | SLC25A16 | NM_152707.2 | 1345 | aaggactctatcgtggtttatct | 181102 | - | - | solute:solute antiporter | - |
| 15429 | SLC25A17 | NM_006358.2 | 831 | aggtttgggcgtcatagactaaa | 181125 | - | - | carrier | - |
| 15430 | SLC25A18 | NM_031481.1 | 1407 | gggattggagagcgcatcttaaa | 181141 | - | - | binding | - |
| 15431 | SLC25A19 | NM_021734.3 | 709 | acgccgtggggaccatgtatagg | 181152 | - | - | deoxynucleotide transporter | - |
| 15432 | SLC25A2 | NM_031947.1 | 464 | ctgtcgtgaaggggtatccttaaa | 181158 | - | - | ornithine transporter | - |
| 15433 | SLC25A20 | NM_000387.2 | 232 | gagggcatcacggggctatatcg | 181170 | - | - | lipid transporter | carnitine-acylcarnitine translocase deficiency |
| 15434 | SLC25A21 | NM_030631.1 | 806 | cagggcctcaacaaaggattaac | 181195 | - | - | binding | - |
| 15435 | SLC25A26 | NM_173471.1 | 318 | gagggtatccaagggttgtatcg | 181206 | - | - | - | - |
| 15436 | SLC25A27 | NM_004277.2 | 555 | tggaggtcgaatggtcacatatg | 181220 | - | - | uncoupling protein | - |
| 15437 | SLC25A28 | NM_031212.2 | 1424 | aggtggggtgaccgcctatttcc | 181229 | - | see also 8991 line | | |
| 15438 | SLC25A3 | NM_002635.1 | 984 | gggactgtttgcccgtatcatca | 181257 | - | - | - | - |
| 15439 | SLC25A4 | NM_001151.1 | 798 | ctgttcgtcgtagaatgatgatg | 181273 | - | - | binding | - |
| 15440 | SLC25A5 | NM_001152.1 | 730 | ctgctgttgccgggttgacttcc | 181278 | - | - | binding | - |
| 15441 | SLC40A1 | NM_014585.3 | 1989 | ttggcttgctcgtattgatttca | 181321 | - | see also 5765 line | | |
| 15442 | TCN1 | NM_001062.2 | 889 | gtgctcacggaaatttctcaagg | 181359 | - | - | binding | - |
| 15443 | TCN2 | NM_000355.2 | 890 | cagcaccccattggcattacagt | 181380 | - | - | vitamin B12 transporter | macrocytic anemia |
| 15444 | TNC | NM_002160.1 | 4802 | gggctacccctagtactgattt | 181431 | - | see also 1385 line | | |
| 15445 | UCP1 | NM_021833.3 | 261 | atgcccgacgtccagtgttatta | 181469 | - | - | uncoupling protein | obesity |
| 15446 | UCP2 | NM_003355.2 | 832 | gaggtggtcggagataccaaagc | 181505 | - | see also 2261 line | | |
| 15447 | UCP3 | NM_003356.2 | 965 | cagcccctcgactgtatgataa | 181510 | - | - | - | obesity |
| 15448 | VWF | NM_000552.2 | 2751 | tggtccggcatgagaacagatgt | 181534 | - | - | - | - |
| 15449 | ACR | NM_001097.1 | 59 | gtgtccgtggttgctaaagataa | 181593 | - | - | trypsin | - |

Figure 46

| | | | | | | | | | hematopoietic-associated factor 1B complex | |
|---|---|---|---|---|---|---|---|---|---|---|
| 15450 | BF | NM_001710.3 | 1090 | gtgtctagtcaacttaattgaga | 181610 | - | - | | | - |
| 15451 | C1RL | NM_016546.1 | 1156 | tggagatgggctggctaactact | 181651 | - | see also 6912 line | | | |
| 15452 | C2 | NM_000063.3 | 645 | ctgccgccaaccctactcttatg | 181664 | - | - | trypsin | | systemic lupus erythematosis) |
| 15453 | C6orf158 | NM_153362.1 | 1138 | cagtgtgtccgacgaatccaatg | 181717 | - | see also 9992 line | | | |
| 15454 | CMA1 | NM_001836.2 | 136 | atggcctacctggaaattgtaac | 181728 | - | - | trypsin | | - |
| 15455 | CRN | NM_006587.2 | 368 | aagggtccgatgttattcttaca | 181753 | - | see also 4614 line | | | |
| 15456 | CTRC | NM_007272.1 | 176 | gtggcgggactttgattgctagc | 181827 | - | - | trypsin | | Alzheimer disease |
| 15457 | CTRL | NM_001907.1 | 273 | gcgagtatgaccgatcatcaaac | 181834 | - | - | trypsin | | - |
| 15458 | CTSG | NM_001911.2 | 573 | acgacccccgaaggcagatttgt | 181850 | - | - | trypsin | | - |
| 15459 | DKFZP586 H2123 | NM_015430.1 | 787 | cggcacgtgcgtccttgacaagg | 181871 | - | see also 6414 line | | | |
| 15460 | DKFZp686L 1818 | NM_182502.1 | 1210 | ctgatgcatgtcagaatgattct | 181932 | - | - | - | | - |
| 15461 | ELA2 | NM_001972.1 | 359 | cggctacgaccccgtaaacttgc | 181943 | - | - | trypsin | | congenital neutropenia |
| 15462 | ELA2B | NM_015849.1 | 741 | cggtccttggttgcaactactac | 181950 | - | - | trypsin | | - |
| 15463 | ELA3B | NM_007352.1 | 556 | ctgccggtggtggactatgaaca | 181953 | - | - | trypsin | | - |
| 15464 | F11 | NM_000128.2 | 376 | aggatcccacccgatggtttact | 181958 | - | - | - | | hypothromboplastinemia |
| 15465 | FLJ90661 | NM_173502.1 | 634 | tgggcgaggccacctgtcaatgt | 182001 | - | - | - | | - |
| 15466 | FLJ90724 | NM_153692.2 | 1366 | gagatcacgatgtaattgtcaac | 182034 | - | - | - | | - |
| 15467 | GZMA | NM_006144.2 | 277 | ttggggctcactcaataaccagg | 182046 | - | apoptosis | trypsin | | - |
| 15468 | GZMB | NM_004131.3 | 258 | ctgttggggaagctccataaatg | 182067 | - | apoptosis | trypsin | | - |
| 15469 | GZMH | NM_033423.2 | 670 | aaggacgtagcccaaggtattct | 182079 | - | apoptosis | trypsin | | - |
| 15470 | GZMK | NM_002104.1 | 703 | aggtgtcttccacgctatagtct | 182105 | - | - | trypsin | | - |
| 15471 | GZMM | NM_005317.2 | 557 | tggacacccgcatgtgtaacaac | 182110 | - | - | trypsin | | - |
| 15472 | HAT | NM_004262.1 | 399 | gagcggatgttgtcatgaaattt | 182125 | - | - | trypsin | | - |
| 15473 | HGFAC | NM_001528.2 | 591 | ctgcggcacagagaaatgctttg | 182157 | - | see also 1049 line | | | |
| 15474 | HPN | NM_002151.1 | 1177 | aggctcgagtccccataatcagc | 182166 | - | receptor_acitivity | trypsin | | - |
| 15475 | IF | NM_000204.1 | 1209 | tggatacaccccgaccttaaacg | 182204 | - | receptor_acitivity | trypsin | | C3b/C4b inactivator deficiency |
| 15476 | KLK1 | NM_002257.2 | 155 | aggcggctctgtaccatttcagc | 182233 | - | - | trypsin | | - |
| 15477 | KLK12 | NM_019598.2 | 800 | ctggagtctacacctatatttgc | 182251 | - | - | - | | - |
| 15478 | KLK13 | NM_015596.1 | 281 | gaggggctcaaagtttacctagg | 182255 | - | - | trypsin | | - |
| 15479 | KLK14 | NM_022046.3 | 990 | aagctggattgaggaaacgatgc | 182265 | - | - | trypsin | | - |
| 15480 | KLK15 | NM_017509.2 | 152 | gagcgtggacgctttaactgtgg | 182267 | - | - | - | | - |
| 15481 | KLK4 | NM_004917.2 | 74 | ctggtagctgcagccaaatcata | 182274 | - | - | trypsin | | - |
| 15482 | KLK5 | NM_012427.3 | 768 | aagaattcgtcccactaaagatg | 182290 | - | - | trypsin | | - |

Figure 46

| | | | | | | | | | | |
|---|---|---|---|---|---|---|---|---|---|---|
| 15483 | KLK6 | NM_002774.2 | 319 | gcggaccctgcgacaagacatct | 182297 | - | - | | trypsin | Alzheimer disease |
| 15484 | KLK7 | NM_005046.2 | 623 | aggactgcacgaaggtttacaag | 182311 | - | - | | - | - |
| 15485 | KLK8 | NM_007196.2 | 895 | ctggcgtctataccaacatctgc | 182317 | - | - | | - | Alzheimer disease |
| 15486 | KLK9 | NM_012315.1 | 308 | gagagcaccacctctggaaatgg | 182319 | - | - | | trypsin | - |
| 15487 | KLKB1 | NM_000892.2 | 397 | gtgcttgccatcgagacatttat | 182331 | - | - | | trypsin | Fletcher factor deficiency |
| 15488 | LOC339967 | NM_182606.1 | 717 | tggggccaccttgattagtaaca | 182390 | - | - | | - | - |
| 15489 | MGC34695 | NM_173555.1 | 190 | gagctgcagcggggtaatcctga | 182404 | - | - | | - | - |
| 15490 | MGC52282 | NM_178453.2 | 355 | tggtctgccccatcaatgatacg | 182407 | - | - | | - | - |
| 15491 | MGC57341 | NM_182559.1 | 406 | aagacgctagcgatcctttaatg | 182414 | - | see also 10269 line | | | |
| 15492 | MPN | NM_031948.2 | 342 | ccgggaccacacgctatgtatgc | 182437 | - | - | | trypsin | - |
| 15493 | MSP | NM_032046.1 | 906 | atgcccttcccagcggtatatct | 182444 | - | receptor_acitivity | | - | - |
| 15494 | PLAT | NM_000930.2 | 466 | cagcgagccaaggtgtttcaacg | 182458 | - | - | | - | - |
| 15495 | PLAU | NM_002658.1 | 974 | ctgccctcgatgtataacgatcc | 182477 | - | signal_transduction | ETS | | breast cancer、Alzheimer disease |
| 15496 | PRSS12 | NM_003619.2 | 1238 | gggtcgcttggaggtatattaca | 182503 | - | see also 2431 line | | | |
| 15497 | PRSS21 | NM_006799.2 | 684 | aagttcaggtcgccatcataaac | 182547 | - | - | | - | - |
| 15498 | PRSS22 | NM_022119.2 | 415 | tggagccccaccctgtgtattcc | 182557 | - | - | | trypsin | - |
| 15499 | PRSS25 | NM_013247.3 | 1072 | cagcacctgccgtggtctatatc | 182565 | - | see also 5366 line | | | |
| 15500 | PRSS7 | NM_002772.1 | 515 | ttgaacagcgttgatatcctaga | 182607 | - | receptor_acitivity | | trypsin | - |
| 15501 | PRSS8 | NM_002773.2 | 375 | gtggccattctgctctatcttgg | 182684 | - | - | | trypsin | - |
| 15502 | PRTN3 | NM_002777.2 | 678 | tggcatcatccaaggaatagact | 182689 | | - | hematopoietic-associated factor 1A complex | | Wegener granulomatosis autoantigen |
| 15503 | SPUVE | NM_007173.3 | 1089 | cagcgggtctggggtctatgtga | 182701 | - | - | | trypsin | - |
| 15504 | ST14 | NM_021978.2 | 1169 | ctgtggaggccgcttacgtaaag | 182719 | - | - | | trypsin | - |
| 15505 | TMPRSS2 | NM_005656.2 | 769 | aagtgccggcaatgtcgatatct | 182734 | - | receptor_acitivity | | trypsin | prostate cancer |
| 15506 | TMPRSS3 | NM_024022.1 | 601 | aagggtcactacgcaaatgttgc | 182776 | - | - | | - | - |
| 15507 | TMPRSS4 | NM_019894.2 | 912 | cagcatccagtacgacaaacagc | 182797 | - | receptor_acitivity | | - | - |
| 15508 | TMPRSS5 | NM_030770.1 | 350 | atggctcctagtgctgtatctgt | 182806 | - | - | | trypsin | - |
| 15509 | TMPRSS6 | NM_153019.2 | 922 | cgggaccgactggccatgtatga | 182822 | - | - | | - | - |
| 15510 | TPSD1 | NM_012217.1 | 501 | gggcgacgtggacaataatgtgc | 182832 | - | - | | trypsin | - |
| 15511 | TSP50 | NM_013270.2 | 734 | tggcacggactatgtgttgaagg | 182839 | - | - | | - | - |
| 15512 | C9orf43 | NM_152786.1 | 1565 | gagaccaaagatgaactactatg | 182871 | - | - | | - | - |
| 15513 | FLJ10504 | NM_018116.2 | 129 | gggccgagcgaccgattccaagg | 182877 | - | - | | - | - |
| 15514 | FURIN | NM_002569.2 | 514 | gtggcaaagcgacggactaaacg | 182885 | - | see also 1652 line | | | |

Figure 46

| | | | | | | | | | |
|---|---|---|---|---|---|---|---|---|---|
| 15515 | MBTPS1 | NM_003791.1 | 1504 | tggcaatgacggacctctttatg | 182934 | - | see also 2590 line | | - |
| 15516 | PACE4 | NM_002570.2 | 851 | ctgccggtcggaaatgaatgtcc | 182982 | - | - | | - |
| 15517 | PCSK1 | NM_000439.3 | 1507 | tggtgaatagtcgatttggattt | 183047 | - | see also 346 line | | - |
| 15518 | PCSK2 | NM_002594.2 | 890 | cagcaacgaccctatccttacc | 183084 | - | see also 1675 line | | - |
| 15519 | PCSK5 | NM_006200.2 | 639 | cagcaagtacgattcatcaaca | 183099 | - | | subtilase | - |
| 15520 | PCSK7 | NM_004716.2 | 784 | caggacattgcacccaactatag | 183167 | - | see also 3237 line | | - |
| 15521 | PCSK9 | NM_174936.2 | 861 | gagtgaccaccgggaaatgaag | 183181 | - | see also 10133 line | | - |
| 15522 | TNS | NM_022648.2 | 849 | aagaagtacgtgcattacttca | 183189 | - | | structural molecule | - |
| 15523 | TPP2 | NM_003291.1 | 3186 | ctgtacgttgcacgacttcatca | 183317 | - | see also 2225 line | | - |
| 15524 | APEH | NM_001640.3 | 1984 | gggcttgcggtactactagtga | 183354 | - | | prolyl oligopeptidase | - |
| 15525 | DPP4 | NM_001935.2 | 858 | aagactacggttccatatcc | 183387 | - | see also 1234 line | | - |
| 15526 | FAP | NM_004460.2 | 1091 | tggctcacgtgggttactgatga | 183471 | - | see also 3010 line | | - |
| 15527 | PREP | NM_002726.2 | 37 | gagaccgcgtacaggattatca | 183500 | - | | sulfate porter | - |
| 15528 | SERPINB2 | NM_002575.1 | 1075 | aagggacgggccaatttctcagg | 183584 | - | see also 1656 line | | - |
| 15529 | SERPINE1 | NM_000602.1 | 1052 | aggctgacttcacgagtctttca | 183599 | - | | Proto-oncogene C-JUN | atherosclerosis |
| 15530 | CLPP | NM_006012.1 | 690 | tggaagaggaaccggctacatgagc | 183604 | - | | endopeptidase Clp | - |
| 15531 | WDR11 | NM_018117.10 | 2644 | ctggtgtacgtcagataaagtga | 183682 | - | see also 7260 line | | |
| 15532 | CLN2 | NM_000391.2 | 1474 | gggggatcctcatcctgatcaat | 183716 | - | | tripeptidyl-peptidase I | amaurotic idiocy, ceroid-lipofuscinosis |
| 15533 | DPP7 | NM_013379.1 | 134 | tcgagcgcttcggccaacaagacc | 183721 | - | | serine-type endopeptidase | - |
| 15534 | FOLH1 | NM_004476.1 | 776 | gcgatctagtgtatgttaactat | 183737 | - | see also 3028 line | | - |
| 15535 | NAALAD2 | NM_005467.2 | 60 | gggccgttctacctttgatgt | 183740 | - | | serine-type endopeptidase | - |
| 15536 | NAALADL1 | NM_005468.1 | 694 | gagctcaccgacgaaaccttc | 183809 | - | | serine-type endopeptidase | - |
| 15537 | P11 | NM_006025.2 | 1176 | tagctgtccggacatataccgg | 183848 | - | see also 4204 line | | - |
| 15538 | PRSS16 | NM_005865.2 | 683 | tggtatcccgaagcctaatgagc | 183855 | - | | serine-type endopeptidase | - |
| 15539 | RELN | NM_005045.2 | 847 | cagtgtggcgcgattatgcatg | 183876 | - | see also 3523 line | | - |
| 15540 | RHBDL2 | NM_017821.2 | 905 | ttggctcacacggtgtttagctgc | 184159 | - | | serine-type endopeptidase | - |
| 15541 | ADAMTS2 | NM_014244.1 | 871 | ctgctgacactcatgaacattgt | 184164 | - | see also 5553 line | | - |
| 15542 | PMPCB | NM_004279.1 | 1166 | tggatgcgactctgttacaagtgt | 184218 | - | | mitochondrial processing peptidase | - |

Figure 46

| 15543 | OSGEP | NM_017807.1 | 501 | cagcccaaccgtgttgtatgtga | 184232 | - | - | - | - |
|---|---|---|---|---|---|---|---|---|---|
| 15544 | OSGEPL1 | NM_022353.1 | 1069 | ctggtggtgtcgcaagtaacttc | 184277 | - | see also 8343 line | | |
| 15545 | IDE | NM_004969.1 | 2048 | aagcatatatgcgatctcttaac | 184350 | - | see also 3451 line | | |
| 15546 | NRD1 | NM_002525.1 | 1578 | tggccatttaacggatccatttg | 184412 | - | see also 1621 line | | |
| 15547 | RCE1 | NM_005133.1 | 533 | ttggctgcggaaccaagtgatcg | 184479 | - | - | prenyl-dependent CAAX protease | - |
| 15548 | ADAM18 | NM_014237.1 | 1080 | cagcatgcacgactatagatatt | 184522 | - | - | metalloendopeptidase | - |
| 15549 | ADAM32 | NM_145004.4 | 728 | tggggctctgatagcatgatagt | 184579 | - | signal_transduction | - | - |
| 15550 | ADAM7 | NM_003817.1 | 2122 | ttgtcggtatcggagttcttata | 184708 | - | - | - | - |
| 15551 | ADAMTS14 | NM_080722.2 | 917 | ttgccctcgtccgcttgatcatg | 184725 | - | - | - | - |
| 15552 | ADAMTS18 | NM_139054.2 | 625 | tagactcagccgggtcatatatt | 184797 | - | see also 9648 line | | |
| 15553 | UQCRC1 | NM_003365.1 | 1193 | aggacgctgtgcccactcttact | 184879 | - | - | ubiquinol-cytochrome c reductase | - |
| 15554 | UQCRC2 | NM_003366.1 | 1107 | aaggctgcctataatcaagtaaa | 184921 | - | see also 2271 line | | |
| 15555 | ZMPSTE24 | NM_005857.2 | 790 | ctggttcttgtcacaatctatgc | 184940 | - | see also 4072 line | | |
| 15556 | BACE | NM_012104.2 | 1316 | tggcaccaccaaccttcgtttgc | 184966 | - | see also 5134 line | | |
| 15557 | BACE2 | NM_012105.3 | 1253 | tggtataccctattaaggaaga | 184986 | - | - | - | - |
| 15558 | CTSD | NM_001909.3 | 199 | caggatcccgctgcacaagttca | 185003 | - | - | Smad3/Sp-1 heterodimer | breast cancer、Alzheimer disease |
| 15559 | NAP1 | NM_004851.1 | 253 | tcgtacctctctcgaactacagg | 185013 | - | - | pepsin A | - |
| 15560 | PGC | NM_002630.1 | 109 | aagtgcccctgaagaaatttaag | 185018 | - | - | pepsin A | - |
| 15561 | REN | NM_000537.2 | 1082 | cagcgcggactatgtatttcagg | 185047 | - | - | renin | hyperproreninemia |
| 15562 | FLJ13687 | NM_024935.2 | 1472 | ctgataggctatggcactttacc | 185088 | - | - | - | - |
| 15563 | FLJ25084 | NM_152792.1 | 959 | gggaccatatcaccaagctaagg | 185112 | - | - | - | - |
| 15564 | MGC26605 | NM_144662.1 | 529 | atgatggtatctcaagattgtat | 185136 | - | - | - | - |
| 15565 | PSMA2 | NM_002787.2 | 185 | ctgtatgatgagcgaagtgtaca | 185152 | - | - | proteasome endopeptidase | - |
| 15566 | PSMA3 | NM_002788.2 | 599 | atgacctgccgtgatatcgttaa | 185178 | - | - | - | - |
| 15567 | PSMA4 | NM_002789.3 | 181 | aggtcgcttataccaagttgaat | 185190 | - | see also 1815 line | | |
| 15568 | PSMA5 | NM_002790.2 | 575 | cagtgtgatgctcgagcaattgg | 185229 | - | - | proteasome endopeptidase | - |
| 15569 | PSMA7 | NM_002792.2 | 223 | aggaagagacattgttgttcttg | 185232 | - | see also 1818 line | | |

EP 1 752 536 A1

Figure 46

| | | | | | | | | | |
|---|---|---|---|---|---|---|---|---|---|
| 15570 | PSMB1 | NM_002793.2 | 240 | acgcgggatagcccaagtta | 185245 | - | - | proteasome endopeptidase | |
| 15571 | PSMB10 | NM_002801.2 | 406 | tggagctacacgcgttatctacg | 185268 | - | - | proteasome endopeptidase | |
| 15572 | PSMB2 | NM_002794.3 | 530 | cagtatcctgaccgatactaca | 185279 | - | - | proteasome endopeptidase | |
| 15573 | PSMB3 | NM_002795.2 | 312 | aggtcggcagatcaaaccttata | 185290 | - | - | proteasome endopeptidase | |
| 15574 | PSMB4 | NM_002796.2 | 266 | ccgcaacatctctcgcattatgc | 185300 | - | - | proteasome endopeptidase | |
| 15575 | PSMB5 | NM_002797.2 | 611 | atggatcggggctattcctatga | 185323 | - | | proteasome endopeptidase | |
| 15576 | PSMB7 | NM_002799.2 | 647 | gggctccggaagcaacattgacc | 185344 | - | - | proteasome endopeptidase | |
| 15577 | PSMB8 | NM_004159.3 | 914 | cagtggctatcggcctaatctta | 185356 | - | - | | diabetes |
| 15578 | PSMB9 | NM_002800.3 | 431 | aaggaggtcaggtatatggaacc | 185365 | - | - | | |
| 15579 | SENP1 | NM_014554.1 | 1337 | aagcatttcgcctgaccattaca | 185406 | - | - | | |
| 15580 | AGTPBP1 | NM_015239.1 | 2801 | cagagcttacgagaatcttatat | 185506 | - | see also 6284 line | | |
| 15581 | CPA1 | NM_001868.1 | 568 | cagtgggtctgtttgcaaaga | 185529 | - | - | carboxypeptidase A | |
| 15582 | CPA2 | NM_001869.1 | 333 | gagaacggagtggttaacttcaat | 185539 | - | - | carboxypeptidase A | |
| 15583 | CPA3 | NM_001870.1 | 204 | atggtggattccgagttagtga | 185565 | - | see also 1202 line | | |
| 15584 | CPA4 | NM_016352.1 | 725 | aagacgcggtccgaaatcctgg | 185634 | - | see also 6832 line | | |
| 15585 | CPA5 | NM_080385.2 | 1597 | tggagcattccgatattgtcta | 185647 | - | - | | |
| 15586 | CPA6 | NM_020361.2 | 1303 | gggggtacgatacagatatggacc | 185702 | - | - | | |
| 15587 | CPB1 | NM_001871.1 | 99 | aaggtgttccgttaacgttga | 185709 | - | - | carboxypeptidase A | |
| 15588 | CPB2 | NM_001872.2 | 1128 | tggggacgattggatctatgatt | 185770 | - | - | | |
| 15589 | CPD | NM_001304.3 | 3770 | tagcgccaggtgtccataacatt | 185910 | - | see also 859 line | | |
| 15590 | CPE | NM_001873.1 | 1078 | gtggtagtgctcacgaatacagc | 185947 | - | see also 1203 line | | |
| 15591 | CPN1 | NM_001308.1 | 1017 | ttggaactgcgggattacttcc | 185981 | - | - | protein stabilization | episodic angioedema |
| 15592 | CPO | NM_173077.1 | 535 | gggacggatctcaatcgaaattt | 186012 | - | - | | |
| 15593 | CPXM | NM_019609.2 | 1335 | gagcatcgatcttaaccataatt | 186049 | - | - | | |
| 15594 | CPZ | NM_003652.1 | 1368 | aggcatgtccgatttcaactacc | 186067 | - | receptor_ acitivity | | |
| 15595 | DSCAML1 | NM_020693.2 | 1663 | ctgaatatcaggcgcgaataaac | 186087 | - | see also 7916 line | | |
| 15596 | FLJ21347 | NM_022827.2 | 1485 | gtgcggaccttgctcaattcagg | 186134 | - | - | | |
| 15597 | FLJ23598 | NM_024783.2 | 1933 | aggaacagacgagagttgttatgt | 186189 | - | - | | |
| 15598 | FLJ32310 | NM_152336.1 | 929 | ctggtcaacgcagatgtgaatag | 186226 | - | - | | |
| 15599 | LOC119587 | NM_198148.1 | 1295 | atggccagttgatgatgaaagttgt | 186241 | - | - | | |

631

Figure 46

| | | | | | | | | | |
|---|---|---|---|---|---|---|---|---|---|
| 15600 | MGC20452 | NM_153232.2 | 779 | aagaacttggttgcgataagttt | 186275 | - | - | - | - |
| 15601 | TBC1D1 | NM_015173.1 | 1161 | aggacagctatcgctttacaaca | 186299 | - | see also 6266 line | | |
| 15602 | PGCP | NM_016134.2 | 1074 | atggcggtggagcctttatatca | 186339 | - | - | - | - |
| 15603 | FLJ14675 | NM_032823.3 | 930 | ctgaagggcgatcggttacatgg | 186372 | - | - | - | - |
| 15604 | RNPEPL1 | NM_018226.2 | 1531 | gaggtccgcaacgactactatcc | 186411 | - | see also 7329 line | | |
| 15605 | TAF2 | NM_003184.2 | 3017 | atgaccctgtaccctatgtaagg | 186499 | - | transcription_regulator | - | - |
| 15606 | METAP1 | NM_015143.1 | 627 | atgaagtcgggccatgtatttac | 186542 | - | - | methionyl aminopeptidase | - |
| 15607 | METAP2 | NM_006838.2 | 738 | atgccggtgacacaacagtatta | 186558 | - | - | methionyl aminopeptidase | - |
| 15608 | PA2G4 | NM_006191.1 | 752 | gaggtacatgaagtatatgctgt | 186582 | - | cell_cycle | methionyl aminopeptidase | - |
| 15609 | DPEP1 | NM_004413.1 | 377 | agggactcccctgtcattgatgg | 186586 | - | - | membrane dipeptidase | - |
| 15610 | DPEP2 | NM_022355.1 | 1045 | tggcgtcgtgatggtgtctttgt | 186596 | - | - | - | - |
| 15611 | DPEP3 | NM_022357.1 | 889 | atgcatcggacaccttgataaga | 186601 | - | - | - | - |
| 15612 | DNPEP | NM_012100.1 | 1025 | gtgcgcatggtcacactctatga | 186619 | - | see also 5131 line | | |
| 15613 | LOC63929 | NM_022098.2 | 210 | aggattccaaaccgatacttagg | 186631 | - | see also 8270 line | | |
| 15614 | XPNPEP1 | NM_020383.2 | 496 | tggacccccttgatcattcctaca | 186673 | - | see also 7859 line | | |
| 15615 | XPNPEP2 | NM_003399.3 | 1956 | tgggatccgtctcgaagatgtgg | 186730 | - | - | - | - |
| 15616 | ACY1 | NM_000666.1 | 832 | gggtggcgtggcctataacgtga | 186738 | - | - | metallopeptidase | small cell lung cancer |
| 15617 | CN1 | NM_032649.4 | 1166 | ccgtctagtccctcacatgaatg | 186771 | - | - | - | - |
| 15618 | CN2 | NM_018235.1 | 543 | gggaggttcgactgatgataagg | 186794 | - | - | - | - |
| 15619 | FLJ32569 | NM_152491.1 | 196 | gaggaacgcgtcgcgatgaaaga | 186825 | - | - | - | - |
| 15620 | PITRM1 | NM_014889.1 | 1240 | cagaacgattgatgaagtagttg | 186871 | - | - | transcription factor | - |
| 15621 | TIMP1 | NM_003254.1 | 365 | ccgcagcgaggagtttctcattg | 186908 | - | - | ETS | renal cell carcinoma、 chondrosarcoma、 Parkinson disease、 melanoma |
| 15622 | DPP10 | NM_020868.1 | 1247 | atggttatccctcggtttactgg | 186926 | - | see also 8010 line | | |
| 15623 | DPP6 | NM_001936.2 | 1047 | cagcatttccctacacgttattg | 187007 | - | - | - | - |
| 15624 | DPP8 | NM_017743.3 | 2249 | aggatacacggaacgttatatgg | 187110 | - | - | - | - |
| 15625 | DPP9 | NM_139159.2 | 2901 | gagtcgggcgagcactatgaagt | 187176 | - | see also 9662 line | | |
| 15626 | PRCP | NM_005040.2 | 970 | tggcaatggtggactatccttat | 187207 | - | - | lysosomal pro-X carboxypeptidase | - |
| 15627 | PPGB | NM_000308.1 | 1131 | aagcatgaactcccagtatctga | 187244 | - | - | vacuolar carboxypeptidase Y | galactosialidosis |
| 15628 | CPVL | NM_019029.1 | 688 | atgtagcacgggatttatacagt | 187259 | - | - | - | - |

Figure 46

| 15629 | RISC | NM_021626.1 | 857 | aagcactcccacgtctacaatgg | 187299 | - | see also 8152 line | | |
|---|---|---|---|---|---|---|---|---|---|
| 15630 | CASP10 | NM_001230.2 | 205 | aaggtcaacattggtattccagt | 187338 | - | apoptosis | - | non-hodgkin lymphoma、autoimmune lymphoproliferative syndrome、gastric cancer |
| 15631 | CASP14 | NM_012114.1 | 536 | cagatgccttgcacgtttattcc | 187374 | - | apoptosis | caspase | - |
| 15632 | CASP3 | NM_004346.2 | 669 | gggatcgttgtagaagtctaact | 187381 | - | apoptosis | - | Alzheimer disease |
| 15633 | CASP4 | NM_001225.2 | 546 | ctggactatagtgtagatgtaga | 187407 | - | apoptosis | - | - |
| 15634 | CASP5 | NM_004347.1 | 347 | aagaatcgcgtggctcatcaaat | 187419 | - | apoptosis | caspase | - |
| 15635 | CASP6 | NM_001226.2 | 745 | gtgaacggctcatggtacattca | 187457 | - | see also 806 line | | |
| 15636 | CASP7 | NM_001227.2 | 929 | atgctaatcctcgatacaagatc | 187480 | - | - | - | craniofacial dysostosis |
| 15637 | CASP8 | NM_001228.2 | 479 | ctgattacctacctaaacactag | 187494 | - | see also 807 line | | |
| 15638 | CASP9 | NM_001229.2 | 1077 | cagctggacgccatatctagttt | 187543 | - | apoptosis | - | - |
| 15639 | CFLAR | NM_003879.3 | 1389 | gagactacgacagctttgtgtgt | 187568 | - | apoptosis、kinase_related | caspase | lupus erythematosus、Alzheimer disease |
| 15640 | CAPN13 | NM_144575.1 | 1277 | aaggatggtcccaaataatgttt | 187607 | - | - | - | - |
| 15641 | CAPN5 | NM_004055.3 | 1149 | aggacgtgtgccggtacttcacg | 187623 | - | see also 2732 line | | |
| 15642 | CAPN6 | NM_014289.2 | 1700 | gtggctacccgaaagtagttact | 187662 | - | see also 5594 line | | |
| 15643 | CAPN7 | NM_014296.1 | 2454 | aggcaatatagcgttggatttga | 187754 | - | see also 5603 line | | |
| 15644 | CTSL | NM_001912.2 | 442 | aggcgatgcacaacagattatac | 187763 | - | - | - | Alzheimer disease |
| 15645 | CTSL2 | NM_001333.2 | 1116 | tggggctcgaatggctatgtaaa | 187780 | - | - | cathepsin L | - |
| 15646 | LGMN | NM_005606.3 | 733 | ctgccggataacatcaatgttta | 187789 | - | - | legumain | - |
| 15647 | PIGK | NM_005482.1 | 93 | cagcgtggccgctagtcatatcg | 187806 | - | see also 3813 line | | |
| 15648 | CTSS | NM_004079.3 | 335 | atgggaatgcactcatacgatct | 187855 | - | see also 2749 line | | |
| 15649 | CTSK | NM_000396.2 | 471 | cagactctgtcgactatcgaaag | 187884 | - | see also 317 line | | |
| 15650 | CTSH | NM_004390.2 | 285 | aagataaacgcccacaacaatgg | 187899 | - | - | - | - |
| 15651 | CTSB | NM_001908.2 | 1098 | aggatcactgtggaatcgaatca | 187928 | - | - | - | Alzheimer disease |
| 15652 | BLMH | NM_000386.2 | 790 | atgagagaattctgtatacgact | 187949 | - | see also 306 line | | |
| 15653 | AQP11 | NM_173039.1 | 1139 | ctgcataacaaccatacaattaa | 187975 | - | - | - | - |
| 15654 | ARHGEF10 | NM_014629.1 | 5454 | gggcgaccgacgttctttacagc | 188020 | - | - | - | - |
| 15655 | CT120 | NM_024792.1 | 383 | aagtcgaaaccgcctcatgatca | 188049 | - | - | - | - |
| 15656 | CTSC | NM_001814.2 | 236 | cggttcccagcgcgatgtcaact | 188060 | - | - | - | - |
| 15657 | CTSF | NM_003793.2 | 1109 | atgcctactcggccataaagaat | 188116 | - | - | lysosomal cysteine-type endopeptidase | - |
| 15658 | CTSO | NM_001334.2 | 524 | caggtcattgactgttcgtataa | 188137 | - | - | cysteine-type endopeptidase | - |
| 15659 | CTSW | NM_001335.2 | 630 | ctgggacgcgttcataactgtcc | 188170 | - | - | cysteine-type endopeptidase | - |

Figure 46

| | | | | | | | | | |
|---|---|---|---|---|---|---|---|---|---|
| 15660 | CTSZ | NM_001336.2 | 427 | tggcggatcgatcaacatcaag | 188173 | - | - | cysteine-type endopeptidase | - |
| 15661 | CYLD | NM_015247.1 | 2057 | gagcgctgaactctttagcatt | 188229 | - | see also 6290 line | | |
| 15662 | D13S106E | NM_005800.3 | 790 | tcgtcaaacttacctgatagtag | 188284 | - | - | - | - |
| 15663 | FLJ20558 | NM_017880.1 | 1712 | cagaaccgagatgggacttatga | 188412 | - | see also 7168 line | | - |
| 15664 | GPR114 | NM_153837.1 | 1003 | gagtcatgggcacgtgaacaac | 188429 | - | - | receptor_acitivity、gpcr、signal_transduction | - |
| 15665 | GRP58 | NM_005313.3 | 614 | cagcaacttgagggataactacc | 188448 | - | signal transduction | protein disulfide isomerase | |
| 15666 | KIAA1203 | NM_020718.1 | 1188 | ttgaggcccacggtttgcattca | 188505 | - | - | - | - |
| 15667 | LCN7 | NM_022164.1 | 768 | ccgatcgtgtctcaatccattct | 188536 | - | - | - | - |
| 15668 | LOC83693 | NM_031463.3 | 526 | cagcagcgggtcgtgagatctaac | 188555 | - | - | - | - |
| 15669 | MGC20741 | NM_018561.3 | 1891 | aggtggtctggccgtaatcatcg | 188599 | - | see also 7525 line | | |
| 15670 | MGC26717 | NM_173824.2 | 126 | atggagggctgccgtaacctact | 188605 | - | - | | |
| 15671 | PARK2 | NM_004562.1 | 106 | tagtgtttgtcaggttcaactcc | 188629 | - | - | | Parkinson disease |
| 15672 | USP1 | NM_003368.3 | 709 | ctgaataatctggcaatacttg | 188661 | - | - | ubiquitin-specific protease | |
| 15673 | USP10 | NM_005153.1 | 1502 | cagcttgttcggctaatgaatg | 188774 | - | see also 3615 line | | |
| 15674 | USP11 | NM_004651.2 | 2213 | atgcccagccgtacattgcttatc | 188809 | - | see also 3165 line | | |
| 15675 | USP12 | NM_182488.1 | 707 | atggtcgtttacctaatggtaat | 188841 | - | see also 10263 line | | |
| 15676 | USP14 | NM_005151.2 | 583 | tggcttcagcgcagtatattact | 188886 | - | - | ubiquitin-specific protease | |
| 15677 | USP15 | NM_006313.1 | 2532 | aggtccttgccgctataatctga | 189028 | - | see also 4416 line | | |
| 15678 | USP16 | NM_006447.1 | 1205 | tggtggtgaactaactagtatga | 189070 | - | see also 4515 line | | |
| 15679 | USP18 | NM_017414.2 | 354 | gggctcctgaggcaaatctgtca | 189114 | - | - | ubiquitin-specific protease | melanoma |
| 15680 | USP2 | NM_004205.3 | 1448 | cagtaggatcggggatctctttg | 189134 | - | see also 2815 line | - | |
| 15681 | USP20 | NM_006676.2 | 259 | gggtcaccggaccaaacctatgg | 189148 | - | - | ubiquitin C-terminal hydrolase | - |
| 15682 | USP21 | NM_012475.2 | 1142 | atgaccgagccaacctaatgtgg | 189179 | - | see also 5349 line | - | |
| 15683 | USP25 | NM_013396.2 | 3579 | gcgatttgcccgaatcatgttgt | 189298 | - | see also 5429 line | - | |
| 15684 | USP26 | NM_031907.1 | 679 | ttgttcgaggtggtaagctata | 189314 | - | - | ubiquitin C-terminal hydrolase | - |
| 15685 | USP29 | NM_020903.1 | 1517 | ttgcaaggcatacatttagtagg | 189412 | - | - | - | - |
| 15686 | USP3 | NM_006537.1 | 1179 | ttgttcgttacgagattgtcttc | 189468 | - | see also 4570 line | - | |

Figure 46

| | | | | | | | | | |
|---|---|---|---|---|---|---|---|---|---|
| 15687 | USP30 | NM_032663.2 | 173 | cggccgtcagatataaagtcatg | 189477 | - | - | - | - |
| 15688 | USP31 | NM_032236.3 | 611 | cagtaataggcgatacattgatc | 189520 | - | - | - | - |
| 15689 | USP33 | NM_015017.2 | 818 | gaggactagctcgaacagataag | 189625 | - | see also 6186 line | | |
| 15690 | USP36 | NM_025090.2 | 1043 | ccggcaagctgcgaatattgtgc | 189711 | - | - | - | - |
| 15691 | USP38 | NM_032557.4 | 1268 | gagccttactacggatccaaatg | 189809 | - | see also 9246 line | | |
| 15692 | USP4 | NM_003363.2 | 2520 | tggtggtccacctcaaacgtttc | 189933 | - | see also 2268 line | | |
| 15693 | USP44 | NM_032147.1 | 536 | aagttactacgacgtacattaag | 189952 | - | - | - | - |
| 15694 | USP46 | NM_022832.2 | 651 | cagggaacgcttaccaatgaaac | 190025 | - | see also 8487 line | | |
| 15695 | USP47 | NM_017944.2 | 1956 | aggtggcgtcaagtcaacatata | 190107 | - | see also 7180 line | | |
| 15696 | USP5 | NM_003481.1 | 805 | tggagctgacgtgtactcatatg | 190188 | - | - | ubiquitin isopeptidase T | - |
| 15697 | USP52 | NM_014871.1 | 1818 | gagcctgtacgctgtctaattca | 190230 | - | see also 6067 line | | |
| 15698 | USP7 | NM_003470.1 | 3097 | gagccggacgtttcgaatagagg | 190373 | - | - | ubiquitin-specific protease | - |
| 15699 | USP8 | NM_005154.1 | 3642 | ttgggaccacgagtaactgatgt | 190473 | - | see also 3617 line | | |
| 15700 | USP9X | NM_004652.2 | 3790 | gtgggtcgttacagctagtattt | 190527 | - | see also 3166 line | | |
| 15701 | USP9Y | NM_004654.2 | 3689 | cagtacggcgatgtattgttaat | 190610 | - | - | ubiquitin-specific protease | - |
| 15702 | BAP1 | NM_004656.2 | 2033 | gaggctgagattgcaaactatga | 190688 | - | - | - | - |
| 15703 | UCHL1 | NM_004181.3 | 335 | tggcacaatcggacttattcacg | 190695 | - | - | ubiquitin C-terminal hydrolase | Parkinson disease |
| 15704 | UCHL3 | NM_006002.3 | 670 | gagcgcgaccctgatgaactaag | 190715 | - | see also 4186 line | | |
| 15705 | UCHL5 | NM_015984.1 | 394 | gtgctactcaagccatagtgagt | 190728 | - | see also 6595 line | | |
| 15706 | UFD1L | NM_005659.3 | 245 | cagccgacttaacattacctatc | 190759 | - | see also 3929 line | | |
| 15707 | PGPEP1 | NM_017712.1 | 370 | gaggacgggcctgaaagcattga | 190785 | - | - | pyroglutamyl-peptidase I | - |
| 15708 | MGC27076 | NM_152699.2 | 2150 | ctgtgacactctctaatcgaatt | 190836 | - | see also 9927 line | | |
| 15709 | PRSC2 | NM_145204.2 | 592 | atgtacgtgatatgtaacactga | 190863 | - | - | - | - |
| 15710 | SENP3 | NM_015670.2 | 1200 | ggggcttccgagtggcttataag | 190874 | - | - | - | - |
| 15711 | SENP7 | NM_020654.2 | 419 | aaggatacccagagttatattga | 190898 | - | - | cysteine-type peptidase | - |
| 15712 | SUSP1 | NM_015571.1 | 205 | aagttaaatcgtcgatctgaaat | 190987 | - | - | cysteine-type peptidase | - |
| 15713 | TINAG | NM_014464.1 | 5 | tggaccggatataagatcttaat | 191085 | - | see also 5696 line | | |
| 15714 | ABHD5 | NM_016006.3 | 189 | tggtgccctacgtctatatcaca | 191128 | - | - | - | Chanarin-Dorfman syndrome |
| 15715 | GGH | NM_003878.1 | 229 | ctgcgtcctatgtaaagtacttg | 191164 | - | - | gamma-glutamyl hydrolase | - |

635

Figure 46

| | | | | | | | | | |
|---|---|---|---|---|---|---|---|---|---|
| 15716 | LOC51185 | NM_016302.2 | 935 | cagcgacttcgctgtgaattaga | 191204 | - | see also 6798 line | | |
| 15717 | RNF127 | NM_024778.3 | 1434 | ttgaatgcgctctatgtatgaga | 191236 | - | - | - | - |
| 15718 | C14orf137 | NM_023112.2 | 319 | cagggccttgggctattcctacc | 191262 | - | - | - | - |
| 15719 | C2orf7 | NM_032319.1 | 179 | tggggacattcgatacatcttca | 191274 | - | - | - | - |
| 15720 | C9orf52 | NM_152574.1 | 875 | aggattatcagactactagaatt | 191293 | - | - | - | - |
| 15721 | DHH | NM_021044.2 | 636 | tggccattgccgtgatgaacatg | 191326 | - | see also 8076 line | | |
| 15722 | DKFZP566H073 | NM_015528.1 | 767 | tggtgccgctgtagtacacaatg | 191335 | - | see also 6451 line | | |
| 15723 | FLJ10613 | NM_019067.3 | 1679 | ccgccctaagagcaacagtatgg | 191369 | - | - | - | - |
| 15724 | FLJ25461 | NM_144966.1 | 172 | tggctgctacgggatttacatca | 191375 | - | - | - | - |
| 15725 | IMP5 | NM_175882.1 | 990 | gtgcgcaaccgtgatcatcttct | 191418 | - | - | - | - |
| 15726 | KYNU | NM_003937.1 | 983 | aagcatgcccatacgattaaacc | 191451 | - | - | peptidase | - |
| 15727 | LOC56926 | NM_020170.1 | 347 | aggacgtcgtccggcaattcatg | 191471 | - | - | - | - |
| 15728 | MGC10067 | NM_145049.1 | 931 | atgaaagcgcacctaaatcgtga | 191519 | - | - | - | - |
| 15729 | OTUB1 | NM_017670.1 | 199 | tggagctctcggtcctatacaag | 191525 | - | - | - | - |
| 15730 | PACS1 | NM_018026.2 | 2153 | ggggtcagtcgacagtaaataca | 191558 | - | - | - | - |
| 15731 | RNF128 | NM_024539.3 | 947 | atgatttggtacgcatcttaacg | 191589 | - | see also 8634 line | | |
| 15732 | RNF13 | NM_007282.3 | 1025 | tggcactttcatcgtgttaatta | 191620 | - | see also 5076 line | | |
| 15733 | RNF130 | NM_018434.4 | 1369 | ctgataacgtagcattcgatatg | 191660 | - | see also 7470 line | | |
| 15734 | RNF149 | NM_173647.2 | 575 | tagtggtcattatgattagctat | 191671 | - | - | - | - |
| 15735 | SHH | NM_000193.2 | 451 | gaggtgtaaggacaagttgaacg | 191683 | - | - | peptidase | holoprosencephaly |
| 15736 | SPC12 | NM_014041.1 | 270 | cgggtggactgtctatatagtta | 191688 | - | see also 5470 line | | |
| 15737 | SPC18 | NM_014300.2 | 500 | aagtgaaagtccgattgtagtgg | 191692 | - | - | peptidase | - |
| 15738 | SPPL2A | NM_032802.2 | 633 | tcgtggcctaactttgattatac | 191710 | - | see also 9286 line | | |
| 15739 | SPPL2B | NM_020172.1 | 417 | aagacgcagtatgatgagattgg | 191754 | - | - | - | - |
| 15740 | SPPL3 | NM_139015.3 | 1281 | tgggcatcggagacatcgttatg | 191779 | - | - | - | - |
| 15741 | TFR2 | NM_003227.2 | 1218 | gcggctagtggtcaacaatcaca | 191787 | - | receptor_acitivity | transferrin receptor | - |
| 15742 | TFRC | NM_003234.1 | 913 | ggggttatgtggcgtatagtaag | 191824 | - | see also 2184 line | | |
| 15743 | WFIKKN | NM_053284.1 | 691 | atgcgccctgatcagatgtatgg | 191868 | - | - | - | - |
| 15744 | WFIKKNRP | NM_175575.4 | 974 | ttgtaacctgccgctatcacttc | 191870 | - | - | - | - |
| 15745 | OCA2 | NM_000275.1 | 1137 | gcgtctacgcgctgatcatattt | 191890 | - | - | - | albinism |
| 15746 | ATP6V1E1 | NM_001696.2 | 513 | ttgctggagccccgaatgattgt | 191919 | - | - | hydrogen-translocating V-type ATPase | - |
| 15747 | ATP6V1E2 | NM_080653.2 | 1563 | aagccatccccgagtacatgaca | 191930 | - | - | - | - |

Figure 46

| | | | | | | | | |
|---|---|---|---|---|---|---|---|---|
| 15748 | ATP1B1 | NM_001677.2 | 915 | aaggcgtacggtggagaacattgg | 191963 | - | | steroid hormone receptor |
| 15749 | ATP1B3 | NM_001679.1 | 260 | cagtgaccgcattggaatataca | 191971 | - | | sodium/potassium-exchanging ATPase |
| 15750 | ATP1B4 | NM_012069.2 | 451 | ctgaccatcagtccctatatacc | 191989 | - | see also 5105 line | |
| 15751 | ATP4B | NM_000705.1 | 341 | ttgtctacaacgtctctgataac | 192012 | - | | sodium/potassium-exchanging ATPase |
| 15752 | FXYD2 | NM_001680.3 | 82 | gggacgtgaccgttctactat | 192020 | - | channel | |
| 15753 | KATNB1 | NM_005886.1 | 1585 | gttggaccatgggcgacatcaaga | 192032 | - | | |
| 15754 | p44S10 | NM_014814.1 | 316 | gagcgaaattcgcgatgcaatga | 192041 | - | see also 6005 line | |
| 15755 | NUDT9 | NM_024047.3 | 1094 | cagccaagaccaactagtgatat | 192102 | - | phosphatase, channel | |
| 15756 | HTCD37 | NM_021222.1 | 447 | ttgtcgaccatcatatcttatcc | 192117 | - | phosphatase | |
| 15757 | MGC20553 | NM_174938.3 | 1186 | aaggaagtagatttcgatatagt | 192151 | - | see also 10134 line | |
| 15758 | SHAPY | NM_138793.2 | 733 | cggctggattcggtatcgaatc | 192164 | - | kinase_related | |
| 15759 | C21orf6 | NM_016940.1 | 447 | ctgtcagatcagtattattgagt | 192183 | - | see also 6977 line | |
| 15760 | RWDD2 | NM_033411.2 | 754 | aggagttctggcacacaattagg | 192206 | - | see also 9421 line | |
| 15761 | NUDT3 | NM_006703.2 | 570 | aagcacagacgtatgtctatgt | 192217 | - | phosphatase | |
| 15762 | ITPA | NM_033453.2 | 601 | ctgctttcagcctgatggatatg | 192228 | - | phosphatase | |
| 15763 | NUDT2 | NM_001161.3 | 367 | gagagcatgtggctgatcatct | 192230 | - | see also 741 line | |
| 15764 | RRAGD | NM_021244.2 | 282 | aagcggcaagtgtctattcaga | 192238 | - | see also 8144 line | |
| 15765 | FLJ10458 | NM_018096.2 | 1372 | cggccacgcggatgaggtatatg | 192267 | - | gpcr, signal_transduction | |
| 15766 | GNB1 | NM_002074.2 | 671 | ctgcgggtgcctggataacattt | 192276 | - | gpcr, signal_transduction | signal transducer |
| 15767 | GNB2 | NM_005273.2 | 839 | ccgatggccgcacgtttgtgtca | 192284 | - | gpcr, signal_transduction | signal transducer |
| 15768 | GNB3 | NM_002075.2 | 1050 | ggggacctgccgtcagactttca | 192292 | - | see also 1339 line | |
| 15769 | GNB4 | NM_021629.2 | 389 | atggactctgtgggtcgaataca | 192298 | - | gpcr, signal_transduction | signal transducer |
| 15770 | GNB5 | NM_006578.2 | 451 | gtggtgtttggataataagtgt | 192335 | - | gpcr, signal_transduction | signal transducer |
| 15771 | GNG10 | NM_004125.2 | 427 | ggggttccagctggaagtaacc | 192348 | - | gpcr, signal_transduction | signal transducer |
| 15772 | GNG13 | NM_016541.1 | 90 | gtgggacggtgccacagatgaaga | 192349 | - | gpcr, signal_transduction | signal transducer |

Figure 46

| | | | | | | | | | |
|---|---|---|---|---|---|---|---|---|---|
| 15773 | GNG3 | NM_012202.1 | 341 | cagcttgtgtcggataaaggtgt | 192352 | - | gpcr、 signal_transduct ion、 kinase_related | signal transducer | - |
| 15774 | GNG4 | NM_004485.2 | 363 | gcgggaagatcctctcatcattc | 192353 | - | gpcr、 signal_transduct ion | signal transducer | - |
| 15775 | GNGT1 | NM_021955.1 | 152 | tggcgaggatccactggtaaagg | 192363 | - | gpcr、 signal_transduct ion | signal transducer | - |
| 15776 | GNGT2 | NM_031498.1 | 427 | gaggcccaagcaggaaacgatcc | 192364 | - | gpcr、 signal_transduct ion | signal transducer | - |
| 15777 | RGS11 | NM_003834.1 | 451 | atgaccggctacacaagaagatc | 192374 | - | gpcr、 signal_transduct ion | - | - |
| 15778 | RGS6 | NM_004296.3 | 1245 | gcgggaccagtttctacgattcc | 192411 | - | see also 2877 line | | |
| 15779 | RGS7 | NM_002924.2 | 1168 | aagtcgctgacagtctactaagt | 192442 | - | gpcr、 signal_transduct ion | regulator of G-protein signaling | - |
| 15780 | RGS9 | NM_003835.1 | 1015 | aggcgctggatcaacatagatgg | 192469 | - | gpcr、 signal_transduct ion | heterotrimeric G-protein GTPase | - |
| 15781 | WDR5B | NM_019069.2 | 689 | ctgataggctaatcataatttgg | 192480 | - | gpcr、 signal_transduct ion | - | - |
| 15782 | ARHGAP5 | NM_001173.1 | 2037 | ttggatcatggccgcttaagatt | 192548 | - | see also 744 line | | |
| 15783 | GTPBP3 | NM_032620.1 | 644 | acgtggaggcctatatcgatttc | 192649 | - | - | - | - |
| 15784 | MFN1 | NM_017927.1 | 693 | ttggtcgcaaactctgaatcaac | 192670 | - | - | - | - |
| 15785 | SAMHD1 | NM_015474.2 | 1814 | ttgtatgccgcaagacaatattt | 192784 | - | signal_transduction | - | - |
| 15786 | CDC25A | NM_001789.1 | 1799 | gagatcgcctgggtaatgaatac | 192828 | - | phosphatase、 cell_cycl e、 kinase_related | prenylated protein tyrosine phosphatase | |
| 15787 | CDC25B | NM_004358.2 | 577 | caggcagccagccggatcattcg | 192831 | - | phosphatase、 cell_cycl e | - | |
| 15788 | CDC25C | NM_001790.2 | 1257 | ttgtcgctatccatatgagtatc | 192875 | - | phosphatase、 cell_cycl e、 kinase_related | - | myelodysplastic syndrome |
| 15789 | DUSP18 | NM_152511.2 | 554 | gcggcctctcgcagataaccaaa | 192886 | - | phosphatase | - | - |
| 15790 | DUSP19 | NM_080876.2 | 765 | gagcagcttcgtacatatcaaga | 192903 | - | see also 9541 line | | |
| 15791 | MTM1 | NM_000252.1 | 1736 | tagccttacgcgacgaatacata | 192972 | - | see also 188 line | | |
| 15792 | MTMR3 | NM_021090.2 | 518 | aagtgttgaatgccgagatatat | 192980 | - | see also 8083 line | | |
| 15793 | PTP4A1 | NM_003463.2 | 1131 | aagattccaacggtcatagaaac | 193055 | - | phosphatase | - | - |
| 15794 | PTPN1 | NM_002827.2 | 1241 | ccgcaccctacggcatcgaaagc | 193077 | - | phosphatase、 signal_tr ansduction | prenylated protein tyrosine phosphatase | - |
| 15795 | PTPN11 | NM_002834.3 | 857 | gagcaatgacggcaagtctaaag | 193096 | - | see also 1854 line | | |
| 15796 | PTPN12 | NM_002835.2 | 245 | cagccgagttaaattgacattaa | 193141 | - | see also 1855 line | | |

Figure 46

| | | | | | | | | |
|---|---|---|---|---|---|---|---|---|
| 15797 | PTPN13 | NM_006264.1 | 5644 | ttgctaccggacataacactaac | 193373 | - | see also 4351 line | |
| 15798 | PTPN14 | NM_005401.3 | 647 | gtgcttgaagggcgattacgatg | 193433 | - | see also 3767 line | |
| 15799 | PTPN18 | NM_014369.2 | 444 | ctgatggcctgtcgagagataga | 193493 | - | receptor_acitivity, ph osphatase | prenylated protein tyrosine phosphatase |
| 15800 | PTPN2 | NM_002828.2 | 252 | ccgctgtacttggaaattcgaaa | 193505 | - | receptor_acitivity, ph osphatase | |
| 15801 | PTPN21 | NM_007039.2 | 3589 | ctgttcgacgccatacaaatagc | 193586 | - | see also 4927 line | |
| 15802 | PTPN22 | NM_012411.2 | 284 | tagaactatcctgataaacctct | 193595 | - | see also 5312 line | |
| 15803 | PTPN3 | NM_002829.2 | 1688 | aggggatcaaatcgtgttaatca | 193714 | - | phosphatase | structural molecule |
| 15804 | PTPN4 | NM_002830.2 | 1100 | ttgtaagtgacccaacaagtta | 193743 | - | see also 1836 line | |
| 15805 | PTPN5 | NM_032781.2 | 1509 | acgagaaatgcaccgagtattgg | 193849 | - | phosphatase | prenylated protein tyrosine phosphatase |
| 15806 | PTPN6 | NM_002831.3 | 1276 | gtgggcatgcagcgtgcttatgg | 193865 | - | see also 1850 line | |
| 15807 | PTPN7 | NM_002832.2 | 564 | gaggacggagattacatcaatgc | 193880 | - | phosphatase | |
| 15808 | PTPN9 | NM_002833.2 | 1134 | aagtcggggaggaggattcaaata | 193895 | - | see also 1851 line | |
| 15809 | PTPRA | NM_002836.2 | 2451 | gtgaacgcatcctttattgatgg | 193945 | - | see also 1858 line | |
| 15810 | PTPRB | NM_002837.2 | 1981 | gtgaacaattccggtcgtaatga | 194004 | - | see also 1860 line | |
| 15811 | PTPRD | NM_002839.1 | 899 | caggccgaagcgttaatatcacc | 194229 | - | receptor_acitivity, ph osphatase, signal_tran sduction | |
| 15812 | PTPRE | NM_006504.3 | 2033 | gagtagtgcgccagtttcacttc | 194410 | - | phosphatase | |
| 15813 | PTPRJ | NM_002843.2 | 1517 | gcgataacgagtcgtcatctaac | 194449 | - | see also 1868 line | |
| 15814 | PTPRK | NM_002844.2 | 2208 | gtggggggtgcaccgtattacttt | 194590 | - | see also 1870 line | |
| 15815 | PTPRM | NM_002845.2 | 995 | cagtcgattccacgagctataaa | 194684 | - | see also 1875 line | |
| 15816 | PTPRN2 | NM_002847.2 | 1202 | aggacgacgatgatagactttac | 194780 | - | see also 1880 line | |
| 15817 | PTPRO | NM_002848.2 | 3603 | gaggtcataccggatgtctatgg | 194903 | - | see also 1882 line | |
| 15818 | PTPRS | NM_002850.2 | 1562 | cagcgcgaccaccatgattgtgc | 194918 | - | receptor_acitivity, ph osphatase | |
| 15819 | PTPRT | NM_007050.3 | 1200 | tagtcgactctcccaactataag | 194951 | - | see also 4934 line | |
| 15820 | PTPRU | NM_005704.2 | 1370 | acgcgttgcacacctatactgt | 194999 | - | receptor_acitivity, ph osphatase, signal_tran sduction | |
| 15821 | TPTE | NM_013315.2 | 1718 | atgttcggtacttgataacatta | 195032 | - | channel, phosphatase, signal_transduction | prenylated protein tyrosine phosphatase |
| 15822 | PTPRH | NM_002842.1 | 1951 | tggaaccecggacgttgtacaat | 195042 | - | phosphatase | |

639

Figure 46

| | | | | | | | | | |
|---|---|---|---|---|---|---|---|---|---|
| 15823 | PTPRN | NM_002846.2 | 341 | aggtgtgctccgacaactcatgt | 195056 | - | receptor_acitivity、ph osphatase | transmembrane receptor protein tyrosine phosphatase | diabetes |
| 15824 | DUSP1 | NM_004417.2 | 892 | agggtcactaccagtacaagagc | 195081 | - | see also 2957 line | | |
| 15825 | ACPP | NM_001099.2 | 115 | tggctagaccgaagtgtactagc | 195087 | - | see also 685 line | | |
| 15826 | CDC14A | NM_003672.2 | 1536 | tggcctagatgatatgtctattg | 195147 | - | phosphatase、cell_cycl e | - | Alzheimer disease |
| 15827 | DUSP2 | NM_004418.2 | 794 | caggaggccataggcttcattga | 195174 | - | phosphatase、kinase_r elated | protein tyrosine/threonine phosphatase | - |
| 15828 | DUSP5 | NM_004419.2 | 1029 | ctgcatggcttaccttatgaaga | 195185 | - | phosphatase、kinase_r elated | protein tyrosine/serine/threoni ne phosphatase | - |
| 15829 | DUSP6 | NM_001946.1 | 1212 | tggtgtcttggtacattgcttgg | 195194 | - | phosphatase、kinase_r elated、cell_cycle | - | - |
| 15830 | DUSP7 | NM_001947.1 | 634 | ctggacgtgctcggcaagtatgg | 195201 | - | - | - | - |
| 15831 | EPM2A | NM_005670.2 | 1188 | ctgcggctggctccagtatgtga | 195227 | - | phosphatase | - | myoclonic epilepsy |
| 15832 | MTMR1 | NM_003828.1 | 701 | gaggaacttgcggcttgcttata | 195245 | - | phosphatase | - | - |
| 15833 | MTMR6 | NM_004685.2 | 2087 | ccggcagataatcgttatagtga | 195337 | - | see also 3213 line | | |
| 15834 | PTP9Q22 | NM_152422.3 | 708 | atggtgtagcgtctcttactact | 195352 | - | phosphatase | - | - |
| 15835 | PTPLA | NM_014241.2 | 608 | ttgaccacttgccatacttcatt | 195408 | - | see also 5552 line | | |
| 15836 | PTPN23 | NM_015466.1 | 945 | aagacgcgcttcgcttcactatg | 195435 | - | phosphatase、signal_tr ansduction | - | small cell lung cancer |
| 15837 | TENS1 | NM_022748.6 | 1845 | caggacctcgatattatcgatgg | 195464 | - | phosphatase | - | - |
| 15838 | CDKN3 | NM_005192.2 | 632 | gagtttcgggacaaattagctgc | 195505 | - | kinase_related、phosp hatase、cell_cycle | receptor-associated protein | - |
| 15839 | DUSP10 | NM_007207.3 | 1498 | aaggcaaacgaccaattatctcc | 195533 | - | see also 5013 line | | |
| 15840 | DUSP14 | NM_007026.1 | 434 | ctggccccaatttgagtatgtta | 195543 | - | phosphatase | protein tyrosine/serine/threoni ne phosphatase | - |
| 15841 | DUSP16 | NM_030640.1 | 879 | cagaaggttgtagtttacgatca | 195561 | - | see also 8917 line | | |
| 15842 | DUSP4 | NM_001394.4 | 1100 | aaggacactatcagtacaagtgc | 195600 | - | phosphatase、kinase_r elated、cell_cycle | - | - |
| 15843 | DUSP9 | NM_001395.1 | 900 | cagaacctgtcgcggttctttcc | 195604 | - | phosphatase、kinase_r elated | protein tyrosine/serine/threoni ne phosphatase | - |
| 15844 | PPP1R3A | NM_002711.2 | 1490 | acgtcggcatgtctcaaagaatc | 195652 | - | see also 1765 line | | |

Figure 46

| | | | | | | | | | |
|---|---|---|---|---|---|---|---|---|---|
| 15845 | PPP1R3C | NM_005398.2 | 647 | ctgacgtagactgtgtctatatg | 195725 | - | phosphatase | - | - |
| 15846 | PPP2R1A | NM_014225.3 | 1464 | tggcttgtggatcatgtatatgc | 195746 | - | phosphatase、apoptosis、cell_cycle、transcription_regulator、signal_transduction | protein phosphatase type 2A | - |
| 15847 | PPP2R1B | NM_002716.3 | 1235 | ctgacgttcgtttgaatatcatc | 195777 | - | phosphatase、apoptosis、cell_cycle、transcription_regulator、signal_transduction | - | colorectal cancer、lung cancer、melanoma、colorectal cancers |
| 15848 | PPP2R2A | NM_002717.2 | 1181 | cagccatagtggtcgatatatga | 195825 | - | phosphatase、signal_transduction | protein phosphatase type 2A、regulator | - |
| 15849 | PPP1R3D | NM_006242.3 | 977 | tggctgtgcgctacactttctcg | 195848 | - | phosphatase | - | - |
| 15850 | DUSP21 | NM_022076.2 | 293 | cggtggaagtggtcaacgtattc | 195852 | - | phosphatase | - | - |
| 15851 | FLJ20442 | NM_017823.2 | 464 | cagttctaccagcgaacgaaata | 195867 | - | phosphatase | - | - |
| 15852 | MGC1136 | NM_024025.1 | 689 | acgactcgccagcctttgacatg | 195870 | - | phosphatase | - | - |
| 15853 | MK-STYX | NM_016086.2 | 968 | ctgcacatccggatagaagattc | 195883 | - | phosphatase | - | - |
| 15854 | MTMR2 | NM_016156.2 | 658 | ctgtcacgaattataggttatat | 195903 | - | - | protein tyrosine/serine/threonine phosphatase | - |
| 15855 | SBF1 | NM_002972.1 | 4879 | ctgtgttccacaattacatgtat | 195969 | - | - | - | - |
| 15856 | SSH-3 | NM_017857.2 | 1273 | aaggagacgcaccgcttcattga | 195979 | - | phosphatase | - | - |
| 15857 | SSH2 | NM_033389.2 | 435 | gagcgacacgctaattcatttgg | 195998 | - | see also 9415 line | | |
| 15858 | STYX | NM_145251.2 | 367 | ttgttcttaggcccatattcatc | 196074 | - | phosphatase | - | - |
| 15859 | CILP | NM_003613.1 | 2875 | cagattgaggggggatcgatatga | 196112 | - | see also 2428 line | | |
| 15860 | CTDP1 | NM_004715.2 | 1053 | cagggcacgggtgatatgaatgc | 196136 | - | phosphatase | - | - |
| 15861 | DNAJC6 | NM_014787.1 | 248 | gggaggctctttagtaacctaaa | 196150 | - | see also 5971 line | | |
| 15862 | FLJ22405 | NM_022485.2 | 518 | tggacgctcaggctacaactatt | 196207 | - | phosphatase | - | - |
| 15863 | ILVBL | NM_006844.3 | 315 | atggtgtgcggttcatcttcacg | 196220 | - | - | - | - |
| 15864 | MTMR8 | NM_017677.2 | 1590 | cagcccaagcagagtatgctaga | 196294 | - | phosphatase | - | - |
| 15865 | PPAP2A | NM_003711.2 | 1197 | gaggaggactctcatacaactct | 196344 | - | see also 2535 line | | |
| 15866 | PPAP2C | NM_003712.2 | 351 | tcgctcggacttcaacaactacg | 196369 | - | phosphatase | - | - |
| 15867 | RNGTT | NM_003800.1 | 810 | tcgtcggtatggtgacatagagg | 196386 | - | see also 2598 line | | |
| 15868 | RSC1A1 | NM_006511.1 | 1037 | aagaatcttgcccgtctataacg | 196454 | - | - | - | - |
| 15869 | TPIP | NM_130785.2 | 1193 | tcgattcgtggtgatgtatgtga | 196473 | - | phosphatase | - | - |
| 15870 | INPP5A | NM_005539.2 | 1105 | cggacaacgaccggaaggttatg | 196489 | - | phosphatase | inositol-1、4、5-trisphosphate 5-phosphatase | - |

EP 1 752 536 A1

Figure 46

| | | | | | | | | | | |
|---|---|---|---|---|---|---|---|---|---|---|
| 15871 | INPP5E | NM_019892.2 | 1768 | cggaccgcgtcttgtacagaagc | 196503 | - | phosphatase | protein binding | - | |
| 15872 | SKIP | NM_016532.2 | 1265 | ggggctgcgggacgttaatgact | 196524 | - | - | - | - | |
| 15873 | OCRL | NM_000276.3 | 1742 | ctgttagcgccctcttccatatt | 196564 | - | see also 227 line | | | |
| 15874 | IMPA2 | NM_014214.1 | 560 | gtggcggttagcattggatttgc | 196593 | - | phosphatase、signal_tr ansduction | myo-inositol-1(or 4)-monophosphatase | - | |
| 15875 | INPP1 | NM_002194.2 | 758 | tggaccccatagattcaacttat | 196614 | - | phosphatase、signal_tr ansduction | inositol-1、4-bisphosphate 1-phosphatase | - | |
| 15876 | INPP4B | NM_003866.1 | 279 | ctgtccgtgatcgtaaactgaat | 196645 | - | phosphatase、signal_tr ansduction | - | - | |
| 15877 | BPNT1 | NM_006085.3 | 235 | atgatagtcagacgtgttattgc | 196718 | - | - | - | - | |
| 15878 | FLJ20421 | NM_017813.1 | 661 | cagcgtccagattaatactgagg | 196738 | - | phosphatase | - | - | |
| 15879 | INPPL1 | NM_001567.2 | 2197 | gcggggttcccgggacacatatg | 196779 | - | phosphatase | - | - | |
| 15880 | MINPP1 | NM_004897.2 | 293 | ccgctaccccacggtcaaacaga | 196801 | - | phosphatase | - | - | |
| 15881 | ACP2 | NM_001610.1 | 491 | gggcccatgtccccgttatgagc | 196831 | - | phosphatase | acid phosphatase | - | |
| 15882 | ACP5 | NM_001611.2 | 800 | gtgcggccacgatcacaatctgc | 196848 | - | phosphatase | acid phosphatase | - | |
| 15883 | ACP6 | NM_016361.2 | 734 | ctggtggtccgaaaccatattct | 196857 | - | phosphatase | - | - | |
| 15884 | ACPT | NM_033068.2 | 961 | ctggggaatcccgccaaagatgg | 196874 | - | phosphatase | - | - | |
| 15885 | FLJ20331 | NM_017768.3 | 643 | cagtctggtgcgactcaatcttt | 196884 | - | see also 7149 line | | | |
| 15886 | FLJ23751 | NM_152282.1 | 211 | ccggtgtcgactcctaagaatgg | 196934 | - | phosphatase | - | - | |
| 15887 | KIAA0377 | NM_014659.1 | 2609 | acgttcgaacgcgtctctatttc | 196995 | - | see also 5829 line | | | |
| 15888 | KIAA0433 | NM_015216.1 | 2612 | acgtagatggtccaagttagaga | 197060 | - | see also 6279 line | | | |
| 15889 | FBP1 | NM_000507.2 | 576 | atggatcttccaacatcgattgc | 197123 | - | phosphatase | fructose-bisphosphatase | diabetes mellitus、lactacidemia、diabetes | |
| 15890 | FBP2 | NM_003837.2 | 321 | tggtccaatcctcctatagtacc | 197134 | - | phosphatase | fructose-bisphosphatase | - | |
| 15891 | G6PC | NM_000151.1 | 112 | tgggatccagtcaacacattacc | 197144 | - | phosphatase | glucose-6-phosphatase | atherosclerosis、glycogen storage disease | |
| 15892 | NT5C1A | NM_032526.1 | 693 | ccgattcttcgacgatgagaagg | 197173 | - | - | - | - | |
| 15893 | NT5C1B | NM_033253.1 | 1020 | aagtgggagtgcggcttataaac | 197185 | - | - | - | - | |
| 15894 | NT5C2 | NM_012229.2 | 1711 | cagctgacacggtcaattagtga | 197246 | - | - | IMP-GMP specific 5'-nucleotidase | - | |
| 15895 | NT5E | NM_002526.1 | 1397 | ctgcaggtgggcggaatccatgt | 197286 | - | see also 1628 line | | | |
| 15896 | NT5M | NM_020201.2 | 647 | atgcctgggtggagaagtacttt | 197300 | - | - | - | - | |
| 15897 | GPLD1 | NM_001503.2 | 538 | aggaccatgggagctattgattt | 197312 | - | see also 1032 line | | | |
| 15898 | PLD1 | NM_002662.1 | 2301 | gggtctgtccatgctaacgtaca | 197434 | - | signal_transduction | phospholipase D | - | |
| 15899 | PLD2 | NM_002663.2 | 1652 | tggaccggcctttcgaagatttc | 197485 | - | see also 1743 line | | | |
| 15900 | PLD3 | NM_012268.1 | 1473 | ctgccctgcgtgacaaccatacc | 197517 | - | see also 5246 line | | | |

Figure 46

| | | | | | | | | | |
|---|---|---|---|---|---|---|---|---|---|
| 15901 | BPGM | NM_001724.3 | 650 | ctgccacggtcggaaagcttaaa | 197533 | - | phosphatase | bisphosphoglycerate phosphatase | hemolytic anemia |
| 15902 | PGAM2 | NM_000290.1 | 669 | acggggatccccattgtgtatga | 197553 | - | phosphatase | phosphoglycerate mutase | myopathy |
| 15903 | PON1 | NM_000446.3 | 154 | gaggtacaacccgtagaacttcc | 197556 | - | see also 351 line | | |
| 15904 | PON2 | NM_000305.1 | 319 | gggcacgggaattaagaatcagt | 197598 | - | phosphatase | arylesterase | atherosclerosis、coronary artery disease、diabetes mellitus、elevated fasting plasma glucose in NIDDM、diabetes |
| 15905 | PON3 | NM_000940.1 | 55 | gagatgttcctggcgtttagaga | 197614 | - | - | arylesterase | - |
| 15906 | PHP14 | NM_014172.2 | 629 | ctgcccagcacgccatttcaact | 197647 | - | phosphatase | - | - |
| 15907 | PDE6A | NM_000440.1 | 548 | atgtcgcacactctaagaagatt | 197656 | - | see also 348 line | | |
| 15908 | PDE6B | NM_000283.1 | 1097 | atgcttccgctgacgaaatgttc | 197699 | - | signal_transduction | cGMP-specific phosphodiesterase | congenital stationary night blindness、retinitis pigmentosa |
| 15909 | PDE6C | NM_006204.2 | 2128 | ttgttcgaggtcgcaataatagc | 197764 | - | see also 4302 line | | |
| 15910 | PDE6G | NM_002602.1 | 335 | ctgcacgagctggcccaatatgg | 197779 | - | - | enzyme inhibitor | - |
| 15911 | PDE6H | NM_006205.1 | 168 | gaggcagactcgccaattcaaga | 197780 | - | - | enzyme inhibitor | - |
| 15912 | PDE4A | NM_006202.1 | 1446 | gcgcgagcgtggcatggaaatca | 197800 | - | signal_transduction | cAMP-specific phosphodiesterase | - |
| 15913 | PDE4B | NM_002600.2 | 373 | ttgacaacgcttccaagcattgc | 197817 | - | see also 1680 line | | |
| 15914 | PDE4C | NM_000923.1 | 1445 | cagacgtggcgcttatgtacaac | 197863 | - | signal_transduction | cAMP-specific phosphodiesterase | - |
| 15915 | PDE4D | NM_006203.2 | 416 | cagtctgcgaactgtacgaaaca | 197875 | - | see also 4299 line | | |
| 15916 | PDE7A | NM_002603.1 | 421 | cagcgatatcttagatcttcacg | 197930 | - | see also 1682 line | | |
| 15917 | PDE1C | NM_005020.1 | 715 | gtggggatcatgcactgaaattt | 197990 | - | see also 3511 line | | |
| 15918 | PDE3A | NM_000921.2 | 2563 | caggcggtgctatataacgatcg | 198077 | - | see also 599 line | | |
| 15919 | PDE2A | NM_002599.1 | 478 | aagtccgggaggctatcatctcc | 198091 | - | signal_transduction | cGMP-stimulated cyclic-nucleotide phosphodiesterase | - |
| 15920 | PDE11A | NM_016953.2 | 2992 | aagccgatgctagattcagtagc | 198167 | - | signal_transduction | - | - |
| 15921 | PDE6D | NM_002601.1 | 551 | ttgacgacgatcttcttgtaagc | 198179 | - | - | 3'、5'-cyclic-nucleotide phosphodiesterase | - |
| 15922 | CNP | NM_033133.3 | 935 | gaggagtacgctcaacaagatgt | 198185 | - | - | 2'、3'-cyclic nucleotide 3'-phosphodiesterase | - |
| 15923 | PLCB1 | NM_015192.2 | 760 | cagcgagatcctcggcttaatga | 198215 | - | see also 6273 line | | |

Figure 46

| | | | | | | | | 1-phosphatidylinositol-4、5-bisphosphate phosphodiesterase beta | |
|---|---|---|---|---|---|---|---|---|---|
| 15924 | PLCB2 | NM_004573.1 | 1428 | gagtattgccggacgatctttgg | 198288 | - | - | | - |
| 15925 | PLCB3 | NM_000932.1 | 1045 | atgtaccgccaggcactactatg | 198324 | - | - | 1-phosphatidylinositol-4、5-bisphosphate phosphodiesterase beta | - |
| 15926 | PLCB4 | NM_000933.2 | 340 | tcggtcgggagccataccaaagg | 198340 | - | see also 606 line | | |
| 15927 | PLCE1 | NM_016341.2 | 4418 | cagctacccctctcatactatta | 198543 | - | see also 6825 line | | |
| 15928 | PLCL1 | NM_006226.1 | 2335 | tagggcaatatacgataccattt | 198684 | - | - | - | lung carcinoma |
| 15929 | PLCL2 | NM_015184.2 | 1273 | ttgccggagtgttgaattagatg | 198737 | - | - | - | - |
| 15930 | FLJ11323 | NM_018390.1 | 797 | tggtcgcctgtatcaagaacatc | 198784 | - | - | - | - |
| 15931 | FLJ31579 | NM_153268.1 | 1284 | aagtgtgcgcaaactaatcctct | 198798 | - | - | - | - |
| 15932 | FLJ37451 | NM_182569.1 | 642 | gcggtataatcgagaacacttaa | 198820 | - | - | - | - |
| 15933 | KIAA1434 | NM_019593.2 | 600 | cagactgaaataagattacgttt | 198847 | - | see also 7721 line | | |
| 15934 | MGC4171 | NM_024307.1 | 785 | gagacgctatgaccgtaatgaaa | 198909 | - | - | - | - |
| 15935 | MIR16 | NM_016641.3 | 487 | ctgtcttaatgcacgataacaca | 198923 | - | see also 6965 line | | |
| 15936 | OBDPF | NM_017711.2 | 839 | tggagttgtcctggtcatctact | 198965 | - | see also 7122 line | | |
| 15937 | PP1665 | NM_030792.4 | 1640 | caggcctgcctccatgcttaact | 198985 | - | - | - | - |
| 15938 | SMPD3 | NM_018667.1 | 1514 | acgtcgtctgtggagatttcaac | 199001 | - | see also 7539 line | | |
| 15939 | TDP1 | NM_018319.2 | 2043 | cggatacgcatgggaacatgtgg | 199053 | - | see also 7400 line | | |
| 15940 | LENG5 | NM_024075.1 | 715 | ctggcgtgtccagtctaaagact | 199056 | - | - | - | - |
| 15941 | MGC2776 | NM_025265.2 | 1400 | tggcctaccattactttcgaagc | 199089 | - | see also 8888 line | | |
| 15942 | POP1 | NM_015029.1 | 2101 | acggcccaactacgttaagcttg | 199164 | - | see also 6198 line | | |
| 15943 | RNASEP1 | NM_006638.1 | 93 | ttgctatgcactaacatacaata | 199177 | - | - | ribonuclease P | - |
| 15944 | RPP20 | NM_005837.1 | 361 | gcgtgctctgagatctacattca | 199194 | - | - | ribonuclease P | - |
| 15945 | RPP30 | NM_006413.2 | 658 | gtggatttagtctgcataactgt | 199206 | - | - | ribonuclease P | - |
| 15946 | RPP38 | NM_006414.1 | 179 | ttgaggacaggcttaaagctatt | 199219 | - | - | structural constituent of ribosome | - |
| 15947 | DLAD | NM_021233.2 | 1086 | aggattagtattatactatgaaa | 199274 | - | - | - | - |
| 15948 | APEX2 | NM_014481.2 | 792 | atgtagggcccttcatcgatagc | 199287 | - | - | - | - |
| 15949 | EloA-BP1 | NM_020695.2 | 551 | ccggcagccacggcctattaagc | 199340 | - | - | - | - |
| 15950 | FLJ12484 | NM_022767.2 | 837 | cagcacgggcactcatcagtaga | 199351 | - | - | - | - |
| 15951 | FLJ12671 | NM_030980.1 | 854 | tggctcgatgtagcattgtcaac | 199365 | - | - | - | - |
| 15952 | ISG20 | NM_002201.4 | 592 | aggacatgagcggctacacaatc | 199377 | - | - | - | - |

Figure 46

| 15953 | MGC16943 | NM_080663.1 | 689 | ttggacgattctcggaatactgc | 199397 | - | - | | - |
|---|---|---|---|---|---|---|---|---|---|
| 15954 | DFFA | NM_004401.1 | 91 | ctggcgagatccggactctaaag | 199404 | - | apoptosis | caspase-activated deoxyribonuclease | - |
| 15955 | DFFB | NM_004402.1 | 897 | tggaacctggatcacataataga | 199428 | - | apoptosis | caspase-activated deoxyribonuclease | - |
| 15956 | KIAA0218 | NM_014760.1 | 1634 | ccggagccgcatgagtgattatt | 199446 | - | - | nuclease | - |
| 15957 | PLA2G13 | NM_032562.1 | 230 | cagtacaggtgccgatatggaaa | 199470 | - | - | calcium-independent cytosolic phospholipase A2 | - |
| 15958 | PLA2G4A | NM_024420.1 | 1226 | tggctcccgacttatttggaagc | 199504 | - | see also 8618 line | | |
| 15959 | PLA2G6 | NM_003560.1 | 366 | aggctgacgccctagtgaatttc | 199539 | - | see also 2367 line | | |
| 15960 | PRDX6 | NM_004905.2 | 250 | aagttgattgccctttcaataga | 199564 | - | - | phospholipase A2 | Alzheimer disease |
| 15961 | CLC | NM_001828.4 | 148 | gcgaccacttgcctgtttcttga | 199573 | - | - | serine esterase | - |
| 15962 | LYPLA1 | NM_006330.2 | 818 | cagttcgtgtcaacaggaaatga | 199596 | - | - | - | atherosclerosis |
| 15963 | PLA1A | NM_015900.1 | 356 | ctgcgtgcaacgaatgctaatgt | 199605 | - | - | - | melanoma |
| 15964 | PLA2G4B | NM_005090.1 | 42 | gcggagcgagttacgagaattcc | 199624 | - | - | - | - |
| 15965 | PLA2G4C | NM_003706.1 | 617 | ctgacctgaaacatcgatttacc | 199647 | - | - | - | - |
| 15966 | C11orf11 | NM_006133.1 | 1628 | aggatgcgatggagtattccaag | 199673 | - | - | - | - |
| 15967 | LOC221955 | NM_139179.1 | 1655 | gtggtcgcgcactgcaataaacc | 199710 | - | - | - | - |
| 15968 | PNLIP | NM_000936.1 | 469 | cagtcggcgttcggttactcacc | 199727 | - | - | triacylglycerol lipase | diarrhea congenital |
| 15969 | PNLIPRP1 | NM_006229.1 | 277 | cagaaagacccggttcatcatcc | 199756 | - | - | triacylglycerol lipase | - |
| 15970 | PNLIPRP2 | NM_005396.3 | 1200 | aagtcacacgtgtgctattgatg | 199794 | - | see also 3766 line | | |
| 15971 | AOAH | NM_001637.1 | 563 | ctgcttagcgcagatatgaatgc | 199804 | - | see also 1083 line | | |
| 15972 | APOC2 | NM_000483.3 | 293 | ctgcccgctgtagatgagaaact | 199839 | - | - | lipoprotein lipase | hyperlipoproteinemia |
| 15973 | LIPA | NM_000235.1 | 1086 | atgtctacgacgtcaatatctta | 199865 | - | - | sterol esterase | atherosclerosis、Wolman disease |
| 15974 | ACHE | NM_000665.2 | 1616 | atgcgatactgggccaactttgc | 199880 | - | - | - | Alzheimer disease |
| 15975 | BCHE | NM_000055.1 | 964 | cagaacgttgaacttagctaaat | 199916 | - | see also 29 line | | |
| 15976 | CACH-1 | NM_130767.1 | 848 | gggccgagggcgtcacatcaaca | 199964 | - | see also 9547 line | | |
| 15977 | CEL | NM_001807.2 | 372 | gggacctgcccgttatgatctgg | 199991 | - | - | sterol esterase | - |
| 15978 | CES1 | NM_001266.3 | 1132 | tggctggttgattccaatgttga | 199999 | - | - | serine esterase | rheumatoid arthritis |
| 15979 | CES2 | NM_003869.4 | 2424 | tggcggactccatgtttgtgatc | 200019 | - | - | - | - |
| 15980 | ESD | NM_001984.1 | 325 | ctgtattggctctcaggtttaac | 200028 | - | - | - | - |
| 15981 | NTE | NM_006702.2 | 2536 | ccgacgctactccttaacagtga | 200058 | - | - | - | - |
| 15982 | PTE1 | NM_005469.2 | 598 | ttggcgctcaaccgaattgctgc | 200082 | - | - | serine esterase | - |
| 15983 | THEA | NM_015547.2 | 623 | cggcgcatgcgccttgtctatgc | 200088 | - | see also 6461 line | | |
| 15984 | ZAP128 | NM_006821.3 | 252 | gagcgtcccggctgtaccaatgg | 200095 | - | - | - | - |

Figure 46

| 15985 | PAFAH1B1 | NM_000430.2 | 1266 | acgtatggtacggccaaatcaag | 200131 | - | see also 336 line | | |
|---|---|---|---|---|---|---|---|---|---|
| 15986 | PAFAH1B2 | NM_002572.1 | 451 | gggatcgaggccattgtacaact | 200155 | - | see also 1654 line | | |
| 15987 | PAFAH1B3 | NM_002573.2 | 817 | cagccatcatgacatgtatgatt | 200167 | - | - | 2-acetyl-1-alkylglycerophosphocholine esterase | - |
| 15988 | H6PD | NM_004285.1 | 1370 | gggctacgctcggatcttgttca | 200186 | - | - | glucose-6-phosphate 1-dehydrogenase | - |
| 15989 | PGLS | NM_012088.1 | 763 | ccgtgcccttcgagaagcattcc | 200197 | - | - | 6-phosphogluconolactonase | - |
| 15990 | HIBCH | NM_014362.2 | 211 | aaggttgcacgggagtcataaca | 200203 | - | - | - | - |
| 15991 | PPT1 | NM_000310.1 | 729 | tagattcggagtggtttggattt | 200248 | - | - | palmitoyl-protein hydrolase | ceroid-lipofuscinosis |
| 15992 | PPT2 | NM_005155.5 | 996 | atggagacacggactacttgaag | 200254 | - | see also 3622 line | | |
| 15993 | HAGH | NM_005326.3 | 638 | gagaaagcacggggtgaaactga | 200267 | - | - | hydroxyacylglutathione hydrolase | - |
| 15994 | ARSB | NM_000046.2 | 1760 | ccgccgaggatttgatacctact | 200282 | - | - | arylsulfatase | mucopolysaccharidosis |
| 15995 | ARSD | NM_001669.1 | 572 | ctgaaccacggatttgactattt | 200320 | - | - | - | - |
| 15996 | ARSE | NM_000047.1 | 874 | aagaacgacacccctttttctgc | 200349 | - | - | arylsulfatase | chondrodysplasia punctata |
| 15997 | ARSF | NM_004042.3 | 872 | acgagctggatccattatggtga | 200381 | - | - | arylsulfatase | - |
| 15998 | GALNS | NM_000512.2 | 1519 | gtgcaactgggcggtcatgaact | 200414 | - | - | N-acetylgalactosamine-4-sulfatase | mucopolysaccharidosis |
| 15999 | STS | NM_000351.2 | 288 | aagcatcaaggccgaacatcatc | 200416 | - | - | steryl-sulfatase | - |
| 16000 | IDS | NM_000202.2 | 1152 | aagacgtccaagccttaaacatc | 200479 | - | see also 160 line | | |
| 16001 | GNS | NM_002076.1 | 1229 | ttgctggctacgacctaaataag | 200527 | - | see also 1340 line | | |
| 16002 | DKFZp313 G1735 | NM_198150.1 | 589 | caggcccatggttaatcttatcc | 200565 | - | - | - | - |
| 16003 | KIAA1001 | NM_014960.1 | 842 | gggttacgtcactgggataatag | 200601 | - | - | - | - |
| 16004 | SGSH | NM_000199.2 | 116 | ttgagagtggcgcgtacaacaac | 200614 | - | see also 159 line | | |
| 16005 | SULF1 | NM_015170.1 | 3001 | ctgtgcttgcacgagttctaaca | 200673 | - | see also 6263 line | | |
| 16006 | SULF2 | NM_018837.2 | 1250 | tggacaacacgtacatcgtatac | 200696 | - | - | - | - |
| 16007 | C14orf126 | NM_080664.1 | 253 | atggcaagcatgtctctatattg | 200718 | - | - | - | - |
| 16008 | FLJ11106 | NM_018324.1 | 1068 | tggatcctgcgaacgagaaatta | 200759 | - | - | - | - |
| 16009 | HARS2 | NM_080820.3 | 492 | caggccggagcttatcaaagatg | 200777 | - | - | - | - |
| 16010 | HYAL2 | NM_003773.2 | 889 | ccgccatggccgcaactttgtga | 200789 | - | receptor_acitivity | - | non-small-cell lung cancer |
| 16011 | HYAL3 | NM_003549.2 | 888 | tggcaatggctggcatagtatgg | 200798 | - | - | - | non-small-cell lung cancer |

Figure 46

| | | | | | | | | |
|---|---|---|---|---|---|---|---|---|
| 16012 | SPAM1 | NM_003117.3 | 684 | tggctactatccttacatagatt | 200881 | - | - | - |
| 16013 | HEXA | NM_000520.2 | 1104 | ctggacatgctcttcttatgg | 200925 | - | beta-N-acetylhexosaminidase | GM2 gangliosidosis |
| 16014 | HEXB | NM_000521.2 | 1681 | atggtcgaacgtggaatagctgc | 200974 | - | beta-N-acetylhexosaminidase | GM2 gangliosidosis, motor neuron disease, juvenile spinal muscular atrophy |
| 16015 | NAGPA | NM_016256.1 | 602 | gggtcgtggcgctgattcgtaat | 200976 | - | - | |
| 16016 | NAGLU | NM_000263.2 | 1898 | cggccgtccctacagatgaatac | 200994 | see also 206 line | - | - |
| 16017 | NAGA | NM_000262.1 | 1102 | ctggcgttaactatgatgacatcc | 201009 | - | alpha-N-acetylgalactosaminidase | alpha-galactosidase deficiency |
| 16018 | MAN2A1 | NM_002372.1 | 1356 | tcggtccggctgggctattgatc | 201042 | see also 1525 line | - | - |
| 16019 | MAN2A2 | NM_006122.1 | 1941 | gttggatgacactcgcttaagtca | 201155 | - | mannosyl-oligosaccharide 1, 3-1, 6-alpha-mannosidase | - |
| 16020 | MAN2B1 | NM_000528.1 | 2233 | tggttacaacgccagtatagttga | 201200 | - | alpha-mannosidase | mannosidosis |
| 16021 | MAN2C1 | NM_006715.1 | 2556 | cagcgacctaccccactacaatac | 201228 | - | - | - |
| 16022 | MANBA | NM_005908.2 | 2004 | ccgtagtcgcagcgagatagtgg | 201295 | - | beta-mannosidase | mannosidosis |
| 16023 | GAA | NM_000152.2 | 1195 | ctgccctgccagtatatcacagg | 201311 | - | alpha-glucosidase glucan 1, 4-alpha-glucosidase | glycogen storage disease |
| 16024 | MGAM | NM_004668.1 | 4171 | gttgggagctatatcgagcttatgt | 201411 | - | Hepatocyte nuclear factor 1 | - |
| 16025 | SI | NM_001041.1 | 5410 | atgcagttacttaacgctataac | 201607 | - | mannosyl-oligosaccharide glucosidase (processing A-glucosidase I) | disaccharide intolerance |
| 16026 | GCS1 | NM_006302.1 | 958 | atggtaaagagtcgcctaaatag | 201619 | - | - | glucosidase I deficiency |
| 16027 | AGL | NM_000028.1 | 3812 | ctgattactggacgctagtaga | 201738 | - | - | glycogen storage disease |
| 16028 | KIAA1128 | NM_018999.1 | 1076 | atgggccaccacaggatatgttt | 201793 | - | - | - |
| 16029 | NEU1 | NM_000434.2 | 724 | tggaccgggcctcggtattcaga | 201831 | - | exo-alpha-sialidase | neuraminidase deficiency |
| 16030 | NEU2 | NM_005383.1 | 1070 | atgattacgaggagattgtcttt | 201843 | - | exo-alpha-sialidase | - |
| 16031 | NEU3 | NM_006656.4 | 2043 | gagggttgacctaggtatctatc | 201865 | - | exo-alpha-sialidase | - |
| 16032 | FLJ90231 | NM_173581.1 | 328 | gggacgccatcctacttaaat | 201877 | - | - | - |
| 16033 | GLB1 | NM_000404.1 | 583 | cagaatgaggcccagttataac | 201894 | - | beta-galactosidase | GM1 gangliosidosis, mucopolysaccharidosis |
| 16034 | LOC112937 | NM_138416.1 | 1043 | atggctcattcaataaggataaa | 201937 | - | - | - |
| 16035 | LOC89944 | NM_138342.2 | 712 | aagcgtggggggaccatcattgc | 201943 | - | - | - |

Figure 46

| | | | | | | | | |
|---|---|---|---|---|---|---|---|---|
| 16036 | GLA | NM_000169.1 | 406 | gggattcgccagctagctaatta | - | see also 141 line | | |
| 16037 | GBE1 | NM_000158.1 | 528 | cggagagatcttgtatcgtattt | - | see also 133 line | | |
| 16038 | SLC3A1 | NM_000341.2 | 1439 | tcgcgtttgggggaatcagtatgt | - | - | cystine transporter | cystinuria |
| 16039 | SLC3A2 | NM_002394.3 | 1204 | caggttcgggacatagagaatct | - | - | AP-1 (c-Jun homodimer) | - |
| 16040 | LOC129530 | NM_174898.1 | 373 | aagacgtcacggcctgaactact | - | - | - | - |
| 16041 | LYG2 | NM_175735.3 | 715 | ttgctcgagctaagtfctataaa | - | - | - | - |
| 16042 | LYZ | NM_000239.1 | 259 | tagccgctactggtgtaatgatg | - | - | structural molecule | - |
| 16043 | PARG | NM_003631.1 | 1225 | taggaaacggtactctactaagg | - | see also 2442 line | - | - |
| 16044 | GUSB | NM_000181.1 | 1644 | gagtatgagcagaaaacgattgc | - | - | beta-glucuronidase | mucopolysaccharidosis |
| 16045 | HPSE | NM_066665.2 | 1182 | gaggcggagcgccctgctatcc | - | see also 4713 line | - | - |
| 16046 | IDUA | NM_000203.2 | 846 | gcgtgcggctgactacatctcc | - | - | L-iduronidase | Hurler syndrome, Hurler-Scheie syndrome, mucopolysaccharidosis, Scheie syndrome |
| 16047 | GBA | NM_000157.1 | 568 | ctgaagaaggaatcggatataac | - | - | glucosylceramidase | Gaucher disease |
| 16048 | GALC | NM_000153.1 | 1210 | gtcggcactacgtggtagaatct | - | - | galactosylceramidase | Krabbe disease |
| 16049 | FUCA1 | NM_000147.2 | 641 | cagagctgacgaccttgttaac | - | - | alpha-L-fucosidase | fucosidosis |
| 16050 | TREH | NM_007180.1 | 1243 | cggagcctggttcgattacgacc | - | - | alpha, alpha-trehalase | trehalase deficiency |
| 16051 | LCT | NM_002299.2 | 2711 | cggacgtttcgggatgactttc | - | - | lactase | hypolactasia, adult type, disaccharide intolerance |
| 16052 | CSMD1 | NM_033225.1 | 475 | acgggcgagcgcaataggatdaca | - | see also 9383 line | - | - |
| 16053 | G2AN | NM_014610.3 | 388 | ttggtggctgatccaccaatagc | - | see also 5777 line | - | - |
| 16054 | GBA3 | NM_020973.2 | 1092 | tggtaccatggggagtatgtaaa | - | - | - | - |
| 16055 | KL | NM_004795.2 | 688 | tcggcggtcaggtcaagtactgg | - | - | - | - |
| 16056 | LOC152831 | NM_175737.2 | 185 | atgtccaacggggatttgcaaag | - | see also 10149 line | - | - |
| 16057 | RIMS3 | NM_014747.1 | 1047 | cagccacctatatcaaggtttac | - | - | - | - |
| 16058 | UNG | NM_003362.2 | 897 | aggaagcggcaccatgtactaca | - | - | - | - |
| 16059 | BST1 | NM_004334.1 | 893 | cagtacagctgtatttaatgatta | - | - | NAD+ nucleosidase | rheumatoid arthritis |
| 16060 | CD38 | NM_001775.2 | 298 | tggcggcgatgcgtcaagtacact | - | signal_transduction, apoptosis | NAD+ nucleosidase | leukemia, diabetes mellitus, diabetes |
| 16061 | ASML3B | NM_014474.1 | 779 | gtgctgaccgatgcatccaaagc | - | - | hydrolase, acting on glycosyl bonds | - |
| 16062 | CTBS | NM_004388.1 | 933 | gcgggctccttattaactata | - | - | hydrolase, acting on glycosyl bonds | - |
| 16063 | FLJ21865 | NM_022759.1 | 208 | cggaccccctgccagttagatat | - | - | - | - |

Figure 46

| 16064 | SMPDL3A | NM_006714.2 | 510 | gcgctgggtaatcatgactattg | 203164 | - | - | hydrolase、 acting on glycosyl bonds | - |
|---|---|---|---|---|---|---|---|---|---|
| 16065 | TDG | NM_003211.2 | 587 | tggcataaacccgggactaatgg | 203212 | - | - | pyrimidine-specific mismatch base pair DNA N-glycosylase | - |
| 16066 | PIGL | NM_004278.2 | 257 | aggagagactcgtaagaaagaac | 203233 | - | - | N-acetylglucosaminylphosphatidylinositol deacetylase | - |
| 16067 | ASAH1 | NM_004315.2 | 352 | cagttccatggtacaccataaat | 203246 | - | - | - | Farber lipogranulomatosis |
| 16068 | AGA | NM_000027.2 | 398 | tggtgtggcacggaaagtactgg | 203277 | - | - | N4-(beta-N-acetylglucosaminyl)-L-asparaginase | aspartylglycosaminuria |
| 16069 | ASRGL1 | NM_025080.2 | 836 | cggacctatcgttgggttatatg | 203313 | - | - | - | - |
| 16070 | C20orf13 | NM_017714.1 | 544 | aagctctcggctggcagaattcc | 203327 | - | see also 7123 line | | |
| 16071 | ASPA | NM_000049.1 | 672 | aagtatcctgtgggtatagaagt | 203358 | - | see also 17 line | | |
| 16072 | GA | NM_013267.2 | 1244 | atgccatcggctattatctcaag | 203389 | - | - | glutaminase | - |
| 16073 | GLS | NM_014905.2 | 1971 | cagcgggactatgattctagaac | 203454 | - | - | glutaminase | - |
| 16074 | FLJ31204 | NM_174912.2 | 1118 | ttgtggttcaccttgaaactatt | 203479 | - | - | - | - |
| 16075 | QRSL1 | NM_018292.2 | 1299 | gtgagacgcctcattgctaatga | 203526 | - | - | - | - |
| 16076 | UPB1 | NM_016327.1 | 735 | cggaaggatcgcggtgaacattt | 203543 | - | - | enzyme | - |
| 16077 | VNN3 | NM_018399.2 | 337 | gagcatttacccctatctagagg | 203557 | - | see also 7446 line | | |
| 16078 | ADA | NM_000022.1 | 1031 | acgggacatgggctttactgaag | 203590 | - | - | Smad3/Sp-1 heterodimer | immunodeficiency |
| 16079 | AMPD1 | NM_000036.1 | 722 | gtgtagtttacgtctatcctaat | 203611 | - | - | AMP deaminase | exercise-related myopathy、 myoadenylate deaminase deficiency |
| 16080 | AMPD2 | NM_004037.5 | 855 | ccgtagtgccccgtatgagttcc | 203657 | - | see also 2728 line | | |
| 16081 | AMPD3 | NM_000480.1 | 2213 | gagtccggtattgcagtatctct | 203703 | - | see also 375 line | | |
| 16082 | ATIC | NM_004044.4 | 1205 | atggtgtcgtcgacaagtcatta | 203746 | - | - | phosphoribosylaminoimidazole-carboxamide formyltransferase | - |
| 16083 | GCH1 | NM_000161.1 | 492 | atgtcctaaacgatgctatattt | 203768 | - | - | GTP cyclohydrolase I | dystonia、 phenylketonuria、 Segawa syndrome、 Parkinson disease |
| 16084 | DDAH1 | NM_012137.2 | 652 | tcgcaattgggtctagtgaatct | 203788 | - | see also 5147 line | | |
| 16085 | DDAH2 | NM_013974.1 | 606 | ggggctccgaattgtggaaatag | 203798 | - | signal_transduction | dimethylargininase | - |
| 16086 | CRMP1 | NM_001313.2 | 405 | atggttattcccggaggtattga | 203802 | - | - | dihydropyrimidinase | - |
| 16087 | DPYS | NM_001385.1 | 1187 | aagatcggatgtccgtaatatgg | 203839 | - | - | dihydropyrimidinase | dihydropyrimidinuria |

Figure 46

| 16088 | DPYSL3 | NM_001387.1 | 1523 | cagcccgttctccgactatgtct | 203895 | - | see also 954 line | | |
| 16089 | IGSF2 | NM_004258.1 | 2331 | cagcgatcggggcaaatatcact | 203932 | - | signal_transduction | - | - |
| 16090 | MGC35366 | NM_152435.1 | 1319 | cagttcggaggccatcatgaatt | 203960 | - | - | - | - |
| 16091 | NIT1 | NM_005600.1 | 735 | cagagatacttacctatccttca | 203967 | - | see also 3892 line | | |
| 16092 | BTD | NM_000060.1 | 655 | ttgaccgctaccgtaaacacaac | 203979 | - | see also 35 line | | |
| 16093 | NIT2 | NM_020202.2 | 577 | aggctgccagctgttggtatatc | 204012 | - | - | - | - |
| 16094 | VNN1 | NM_004666.1 | 1179 | gtggaagggcgctattatctaca | 204036 | - | - | hydrolase、 acting on carbon-nitrogen (but not peptide) bonds | - |
| 16095 | VNN2 | NM_004665.2 | 223 | agggtgctcgaatcattgtgact | 204057 | - | - | - | - |
| 16096 | ABHD6 | NM_020676.3 | 913 | aggttccgacgcagatcatctgg | 204107 | - | see also 7915 line | | |
| 16097 | ABHD7 | NM_173567.2 | 543 | tagattctttagggtatagcaaa | 204121 | - | - | - | - |
| 16098 | ABHD8 | NM_024527.2 | 201 | cggggtgaccgacggtatcttct | 204140 | - | - | - | - |
| 16099 | ABHD9 | NM_024794.1 | 810 | atgtcggtgtaccaagactattc | 204153 | - | - | - | - |
| 16100 | EPHX1 | NM_000120.2 | 1071 | cagcgtttcgggaggtttcttgg | 204173 | - | - | epoxide hydrolase | - |
| 16101 | EPHX2 | NM_001979.3 | 284 | gtgctccgagaccgctaaagtct | 204185 | - | - | epoxide hydrolase | - |
| 16102 | MEST | NM_002402.2 | 706 | tcgatctggtcggcttaccataa | 204226 | - | - | - | - |
| 16103 | AHCY | NM_000687.1 | 1021 | tggaccggtatcggttgaagaat | 204243 | - | - | adenosylhomocysteinase | - |
| 16104 | AHCYL1 | NM_006621.3 | 1465 | aagtcgatgtcgtaataacttgc | 204272 | - | see also 4682 line | | |
| 16105 | KIAA0828 | NM_015328.1 | 527 | cagcggcttcatatacagatagc | 204296 | - | see also 6366 line | | |
| 16106 | FAH | NM_000137.1 | 978 | atgagccaggcggctaccatatg | 204340 | - | - | fumarylacetoacetase | tyrosinemia |
| 16107 | GPAA1 | NM_003801.2 | 612 | cagatttattgggccaaagatat | 204346 | - | - | - | - |
| 16108 | AADAC | NM_001086.1 | 116 | gtggggatcctcatagcatatta | 204352 | - | - | carboxylic ester hydrolase | - |
| 16109 | ABHD1 | NM_032604.2 | 1003 | ccgtacaatccgccagtttgatg | 204400 | - | - | - | - |
| 16110 | ABHD4 | NM_022060.1 | 933 | gggtccgacacctggatagatac | 204417 | - | - | - | - |
| 16111 | ACATE2 | NM_012332.1 | 819 | gaggaccaccatacatgagatgt | 204445 | - | - | - | - |
| 16112 | ADPRHL1 | NM_138430.3 | 306 | ctggtgcctggatgatctgtacc | 204464 | - | - | - | - |
| 16113 | ADPRHL2 | NM_017825.1 | 847 | cggtacccaccgccatctactgc | 204478 | - | - | - | - |
| 16114 | ASAHL | NM_014435.1 | 577 | ttggctatgtaggattatggact | 204488 | - | - | - | - |
| 16115 | ATP5C1 | NM_005174.1 | 674 | atgagtatctatgacgatattga | 204505 | | - | hydrogen-transporting two-sector ATPase | - |

650

Figure 46

| | | | | | | | | | |
|---|---|---|---|---|---|---|---|---|---|
| 16116 | ATP5D | NM_001687.3 | 370 | aggacggcaccacctccaaatac | 204516 | - | - | hydrogen-transporting two-sector ATPase | - |
| 16117 | ATP5O | NM_001697.1 | 595 | gtgcgcattggcgagaaatatgt | 204531 | - | - | hydrogen-transporting two-sector ATPase | - |
| 16118 | ATP6AP2 | NM_005765.2 | 146 | tggggaacgagtttagtatatta | 204540 | - | see also 4025 line | | |
| 16119 | ATP6V0B | NM_004047.2 | 670 | ggggtcatcgtcgcaattcttca | 204578 | - | - | hydrogen-transporting two-sector ATPase | - |
| 16120 | ATP6V0D1 | NM_004691.3 | 244 | ctgcagagcactgattatggtaa | 204580 | - | - | molecular_function unknown | - |
| 16121 | ATP6V1D | NM_015994.2 | 715 | ttgaacgtactcttgcttatatc | 204598 | - | - | hydrogen-transporting two-sector ATPase | - |
| 16122 | ATP6V1G1 | NM_004888.2 | 357 | ccggcagaacagggatgaagtct | 204607 | - | - | hydrogen-transporting two-sector ATPase | - |
| 16123 | ATP6V1G2 | NM_130463.2 | 435 | cagagagcgagagcacgaattcc | 204613 | - | - | - | - |
| 16124 | ATP6V1G3 | NM_133262.2 | 361 | tggacactacaataagtatatgg | 204623 | - | - | - | - |
| 16125 | BPHL | NM_004332.1 | 582 | ggggcataaccgcactcattgc | 204630 | - | - | - | - |
| 16126 | C11orf8 | NM_001584.1 | 191 | aggcattcacgcactacaacatc | 204644 | - | - | hydrolase | - |
| 16127 | C20orf147 | NM_152667.1 | 583 | gtgatggtcggtgacacattaga | 204686 | - | phosphatase | - | - |
| 16128 | C22orf1 | NM_001585.2 | 970 | gggacgaccacctatgtgaatgc | 204695 | - | - | - | - |
| 16129 | CHI3L1 | NM_001276.1 | 468 | gagtcgccggactttcatcaagt | 204700 | - | - | hydrolase | rheumatoid arthritis |
| 16130 | CHI3L2 | NM_004000.1 | 732 | gggccaagctcctactacaatgt | 204728 | - | - | hydrolase | - |
| 16131 | DBR1 | NM_016216.1 | 977 | gggctacggatgatcttattaat | 204760 | - | - | - | - |
| 16132 | DIO3 | NM_001362.1 | 337 | gtgtatccgcaagcatttcctgg | 204778 | - | - | thyroxine deiodinase | - |
| 16133 | DKFZP564 D1378 | NM_032124.3 | 809 | gggaaatatcgagcatcagatga | 204790 | - | - | - | - |
| 16134 | DKFZP564 O243 | NM_015407.3 | 470 | tgggaactcggcaccttcaaagg | 204793 | - | - | - | - |
| 16135 | DPYSL4 | NM_006426.1 | 1287 | tcgtcgcggtgaccagtacaaat | 204796 | - | see also 4499 line | | |
| 16136 | DPYSL5 | NM_020134.2 | 896 | tggtcaacgtgtccagtatctcg | 204818 | - | - | - | - |
| 16137 | ENPP4 | NM_014936.1 | 1262 | cagaagccatcgcgattgttatc | 204861 | - | see also 6119 line | | |
| 16138 | ENPP5 | NM_021572.4 | 1216 | aggcaaccacggttacgataatg | 204904 | - | - | - | - |
| 16139 | ENPP6 | NM_153343.2 | 1197 | aggtcggtggacgtctacaatgt | 204952 | - | - | - | - |

EP 1 752 536 A1

651

Figure 46

| | | | | | | | |
|---|---|---|---|---|---|---|---|
| 16140 | EYA1 | NM_000503.3 | 807 | cagccgacgggtctttaaacaat | 204960 | - | see also 390 line |
| 16141 | EYA2 | NM_005244.3 | 363 | cagcaggctaccccctatacagc | 205003 | - | phosphatase、 transcription_regulator |
| 16142 | EYA3 | NM_001990.2 | 1156 | aaggacccaacagtagtgattgg | 205046 | - | see also 1273 line |
| 16143 | EYA4 | NM_004100.2 | 1596 | ctgctcaccggtcttatgcaca | 205086 | - | see also 2769 line |
| 16144 | FAM16AX | NM_012080.1 | 343 | ccgcgtcgttcgatatgaagaca | 205122 | - | - |
| 16145 | FLJ11151 | NM_018340.1 | 1008 | acgatctcatggatttgatcaag | 205132 | - | - |
| 16146 | FLJ11342 | NM_018394.1 | 646 | aggagtttataacgttcagtaca | 205162 | - | - |
| 16147 | FLJ21736 | NM_024922.3 | 489 | ccggtagccggtcatggtatgg | 205176 | - | - |
| 16148 | FLJ31547 | NM_145024.1 | 379 | ctgcatcgccctggataactg | 205196 | - | - |
| 16149 | GNPDA1 | NM_005471.2 | 785 | aggtgggatcgagctatttgttg | 205225 | - | hydrolase |
| 16150 | KIAA1363 | NM_020792.1 | 868 | ttgtgcagcaatgatcgttaac | 205251 | - | see also 7970 line |
| 16151 | LHPP | NM_022126.2 | 520 | tggacgttggtccctacatgaag | 205270 | - | - |
| 16152 | LOC134147 | NM_138809.2 | 245 | atggctaacgaagcttatccttg | 205275 | - | - |
| 16153 | LOC339221 | NM_178543.2 | 788 | tgggcgacctgttgaattccaca | 205294 | - | - |
| 16154 | MASA | NM_021204.1 | 493 | aggccgttagataatgtgtgc | 205305 | - | - |
| 16155 | MGC12904 | NM_031219.2 | 1448 | aggggattctgtacctaaagaaca | 205324 | - | phosphatase |
| 16156 | MGC3234 | NM_023947.2 | 434 | gagacgtggccgttgagatgtttg | 205328 | - | - |
| 16157 | MPPE1 | NM_023075.2 | 1963 | ttgctcggaaagcgtaagacaag | 205355 | - | - |
| 16158 | OVGP1 | NM_002557.2 | 1301 | cggcatggaccactgatagtaag | 205388 | - | hydrolase |
| 16159 | PIGO | NM_032634.2 | 3523 | gtgtttgcccctaagttcatatt | 205464 | - | see also 9265 line |
| 16160 | PLP | NM_020315.3 | 202 | cggcaaggcggctctgtttgtga | 205466 | - | - |
| 16161 | SGPP2 | NM_152386.2 | 232 | aagtacgtcgtgaagaattattt | 205470 | - | - |
| 16162 | THEM2 | NM_018473.2 | 465 | gtgtcgatatgaacataacgtac | 205508 | - | - |
| 16163 | VPS29 | NM_016226.2 | 578 | aagtagaacgaatcgaatacaaa | 205526 | - | - |
| 16164 | COL4A3BP | NM_005713.1 | 2203 | aagcgagagatcctaaattct | 205574 | - | see also 3962 line |
| 16165 | CSNK2B | NM_001320.5 | 895 | caggctcacgtttcaagatcc | 205588 | - | see also 872 line |
| 16166 | PRKDC | NM_006904.6 | 11629 | atgtataaggcgctaatcgtac | 205894 | - | see also 4842 line |
| 16167 | PRKAG1 | NM_002733.1 | 297 | gtgtacgagctgccccttatgg | 205912 | - | see also 1772 line |
| 16168 | CKS2 | NM_001827.1 | 145 | acgagtaccggcgatgttatgtta | 205934 | - | kinase_related、 cell_cycle | cyclin-dependent protein kinase |
| 16169 | PRKAG3 | NM_017431.1 | 764 | tggtgctgcatcgctactacagg | 205945 | - | kinase_related | SNF1A/AMP-activated protein kinase |

Figure 46

| 16170 | GTF2H1 | NM_005316.2 | 1711 | aagcacaaatttggtaagtcaca | 205964 | - | - | general RNA polymerase II transcription factor | - |
|---|---|---|---|---|---|---|---|---|---|
| 16171 | BCR | NM_004327.2 | 2999 | cagccgcaacggcaagagttaca | 205971 | - | kinase_related | - | leukemia、chronic myeloid leukemia、chronic myelogenous leukemia、Ewing's sarcoma |
| 16172 | BRD2 | NM_005104.2 | 3926 | atgtcagcggacagctcaattct | 206009 | - | kinase_related | protein serine/threonine kinase | - |
| 16173 | FASTK | NM_006712.3 | 1036 | tggctacagtcaacatcttgatg | 206017 | - | - | - | - |
| 16174 | H11 | NM_014365.2 | 966 | aggtggatcctgtgacagtattt | 206024 | - | kinase_related | protein serine/threonine kinase | - |
| 16175 | EFNA4 | NM_005227.2 | 229 | ctgggcctcaacgattacctaga | 206031 | - | see also 3664 line | | |
| 16176 | EFNB3 | NM_001406.3 | 784 | ccgctcgcaccacgattactaca | 206048 | - | receptor_acitivity | transmembrane-ephrin receptor | neuroblastoma |
| 16177 | NRP2 | NM_003872.1 | 774 | tggcttctctgcgcgttactacc | 206068 | - | see also 2633 line | | |
| 16178 | FGFRL1 | NM_021923.2 | 966 | ccgacggctcctacctcaataag | 206105 | - | receptor_acitivity | - | - |
| 16179 | PDGFRL | NM_006207.1 | 718 | tggaacggacattgtttatgaca | 206125 | - | receptor_acitivity | - | - |
| 16180 | IGF2R | NM_000876.1 | 6206 | acggagtctcgtactatataaat | 206285 | - | see also 572 line | | |
| 16181 | CRKL | NM_005207.2 | 535 | cggaccgtccgcctggtatatg | 206306 | - | kinase_related、signal _transduction | protein tyrosine kinase | - |
| 16182 | WIF1 | NM_007191.2 | 249 | aggcaagagtactcataggattt | 206333 | - | see also 4987 line | | |
| 16183 | C9orf39 | NM_017738.1 | 2846 | aagcttactctcctagtatcaag | 206417 | - | - | - | - |
| 16184 | KCNH2 | NM_000238.2 | 1845 | atgtgacggcgctctacttcacc | 206435 | - | see also 174 line | | |
| 16185 | KCNH3 | NM_012284.1 | 2416 | gcggcgacaatacccttatgtcc | 206450 | - | - | - | - |
| 16186 | KCNH4 | NM_012285.1 | 1198 | cagtccggccaggtaatctctgc | 206463 | - | channel、signal_transduction | - | - |
| 16187 | KCNH8 | NM_144633.2 | 1441 | tcgatccgaagtgcctatattgc | 206516 | - | channel、signal_transduction | - | - |
| 16188 | ATR | NM_001184.2 | 6429 | ccgctccgatcgtgtacaaatga | 206764 | - | kinase_related、cell_cycle | - | Seckel syndrome |
| 16189 | CDK5R1 | NM_003885.2 | 283 | cggccaccgtgggccactatacg | 206826 | - | kinase_related、cell_cycle | cyclin-dependent protein kinase 5 activator | - |

Figure 46

| | | | | | | | | | |
|---|---|---|---|---|---|---|---|---|---|
| 16190 | CDKN1A | NM_000389.2 | 235 | gcgatggaacttcgactttgtca | 206829 | - | kinase_related、cell_cycle、apoptosis | - | bladder transitional cell carcinoma、laryngeal cancer、colorectal cancer、bladder cancer、ovarian cancer、prostate cancer、colorectal cancers、non-small-cell lung cancer、squamous cell carcinomacytic、gastric cancer |
| 16191 | PIK3C2A | NM_002645.1 | 3064 | cagtttagtacccgatacgaaca | 206921 | - | see also 1719 line | | |
| 16192 | PIK3C2B | NM_002646.2 | 4822 | aggagctaaacggttacatctgg | 207041 | - | kinase_related | phosphatidylinositol 3-kinase、class III | - |
| 16193 | PIK3C2G | NM_004570.2 | 4394 | ctgtagtgtcccactcgataaag | 207171 | - | see also 3107 line | | |
| 16194 | PIK3C3 | NM_002647.2 | 1738 | acgcgaaagtggaaatcgtaaga | 207237 | - | see also 1728 line | | |
| 16195 | PIK3CA | NM_006218.1 | 2968 | aaggcttatctagctattcgaca | 207342 | - | see also 4306 line | | |
| 16196 | PIK3CB | NM_006219.1 | 1310 | ctgtagcgtgggtaaatacgatg | 207382 | - | see also 4313 line | | |
| 16197 | PIK3R2 | NM_005027.1 | 1664 | cagatgaagcgtactgcaattga | 207536 | - | kinase_related | phosphatidylinositol 3-kinase | - |
| 16198 | MGC26597 | NM_152700.2 | 1432 | aagtgtcaatcgacactcaaaga | 207545 | - | kinase_related | - | - |
| 16199 | MGC46424 | NM_173492.1 | 322 | gtgctgaaggacctcaactttca | 207550 | - | kinase_related | - | - |
| 16200 | PIP5K1A | NM_003557.1 | 1373 | gagcccttaagcagtgaaacaca | 207555 | - | kinase_related、signal_transduction | - | - |
| 16201 | PIP5K1B | NM_003558.1 | 669 | aagacatacgctccattagcatt | 207559 | - | see also 2365 line | | |
| 16202 | PIP5K1C | NM_012398.1 | 254 | ccggcgaaaccacctacaagaag | 207601 | - | kinase_related | - | - |
| 16203 | PIP5K2A | NM_005028.3 | 770 | atgtaccggcttaatgttgatgg | 207626 | - | kinase_related | 1-phosphatidylinositol-4-phosphate 5-kinase | - |
| 16204 | PIP5K2C | NM_024779.3 | 341 | aggaacctccgtgatcgatttgg | 207664 | - | see also 8742 line | | |
| 16205 | IHPK1 | NM_153273.1 | 715 | aggaccgaaagctctacaagttc | 207687 | - | - | - | - |
| 16206 | IHPK2 | NM_016291.1 | 384 | aggaacttgtgtctaatagcata | 207699 | - | kinase_related | - | - |
| 16207 | IHPK3 | NM_054111.1 | 1271 | cagagttcataccgcttctattc | 207720 | - | kinase_related、apoptosis | - | - |
| 16208 | IPMK | NM_152230.1 | 1165 | gtgttccctagcaacacaataga | 207761 | - | kinase_related | - | - |
| 16209 | PIK4CB | NM_002651.1 | 361 | gagatccgttgcctagatgatcc | 207772 | - | see also 1733 line | | |
| 16210 | FRAP1 | NM_004958.2 | 3674 | ctggtgcgacaccgaatcaatca | 207868 | - | see also 3446 line | | |
| 16211 | PI4K2B | NM_018323.2 | 568 | gagccttatggtcaactcaatcc | 207950 | - | kinase_related | - | - |
| 16212 | SMG1 | NM_014006.2 | 8210 | atgcggcgttatttgaactaatc | 208207 | - | kinase_related | - | - |
| 16213 | DGKH | NM_152910.3 | 3387 | cagaagcaccgtatttcgaatag | 208357 | - | kinase_related | - | - |
| 16214 | DGKI | NM_004717.2 | 2348 | gagacttgccgtatgtacataga | 208409 | - | kinase_related | diacylglycerol kinase | - |

EP 1 752 536 A1

Figure 46

| | | | | | | | | | |
|---|---|---|---|---|---|---|---|---|---|
| 16215 | FLJ10842 | NM_018238.2 | 986 | cagcttgacccgacaagcaaaga | 208449 | - | see also 7339 line | | |
| 16216 | CHK | NM_001277.1 | 554 | tggttctggagagcgttatgttt | 208455 | - | see also 842 line | | |
| 16217 | FLJ10761 | NM_018208.1 | 558 | caggttaatcgccttagaaatgg | 208481 | - | kinase_related | - | - |
| 16218 | XYLB | NM_005108.2 | 1336 | gtggaggttcgagcactaattga | 208513 | - | kinase_related | - | - |
| 16219 | RBSK | NM_022128.1 | 216 | acgtccatggtgtgtaaggttgg | 208527 | - | see also 8291 line | | |
| 16220 | FLJ13052 | NM_023018.2 | 383 | aggaggacacgctctcttcatgg | 208551 | - | kinase_related | NAD+ kinase | - |
| 16221 | KHK | NM_000221.1 | 82 | aggaggactcggagataaggtgt | 208562 | - | kinase_related | - | fructosuria |
| 16222 | DKFZP586 B1621 | NM_015533.2 | 1587 | aggagctgatctgttacaagtcc | 208587 | - | see also 6455 line | | |
| 16223 | EKI1 | NM_018638.3 | 670 | gtgcaccacaactctactgtacc | 208596 | - | kinase_related | choline kinase | - |
| 16224 | FLJ11149 | NM_018339.3 | 544 | atggaacccatattacaagaata | 208627 | - | kinase_related | - | - |
| 16225 | PDXK | NM_003681.2 | 602 | tggtgccgcttgcagacattatc | 208645 | - | kinase_related | protein binding | - |
| 16226 | POLR2L | NM_021128.2 | 190 | gagaagctgctcaattatgcacc | 208702 | - | transcription_regulator | DNA-directed RNA polymerase III | - |
| 16227 | POLR3D | NM_001722.1 | 549 | gaggaacgacactcgaaatatgc | 208704 | - | transcription_regulator, cell_cycle | | - |
| 16228 | POLR3F | NM_006466.2 | 263 | cagcgggcagtagccatcaatag | 208717 | - | transcription_regulator | DNA-directed RNA polymerase III | - |
| 16229 | RPC5 | NM_018119.2 | 2139 | ccgggtacgccgaaacatgatcc | 208765 | - | see also 7264 line | | |
| 16230 | USP49 | NM_004275.2 | 245 | cagaccgggaagctgatgtatgt | 208773 | - | transcription_regulator | - | - |
| 16231 | POLD2 | NM_006230.1 | 513 | aggcaccattgacgtgtcaaagc | 208786 | - | - | zeta DNA polymerase | - |
| 16232 | POLD4 | NM_021173.1 | 86 | gcggctcatcactgattcctacc | 208801 | - | - | delta DNA polymerase | - |
| 16233 | POLI | NM_007195.1 | 521 | gtgtcgggtcatgtatacaataa | 208818 | - | see also 4999 line | | |
| 16234 | CDS2 | NM_003818.2 | 552 | atgtgcgttcagattaagtgttt | 208877 | - | transcription_regulator | phosphatidate cytidylyltransferase | - |
| 16235 | GALT | NM_000155.2 | 1053 | ctgccactgtccggaaattcatg | 208918 | - | - | - | - |
| 16236 | PCYT1A | NM_005017.2 | 645 | ctggccgaacaccggattgattt | 208933 | - | - | choline-phosphate cytidylyltransferase | - |
| 16237 | PCYT1B | NM_004845.2 | 421 | tcgctacgtagacgaagttatca | 208954 | - | - | choline-phosphate cytidylyltransferase | - |
| 16238 | CMAS | NM_018686.3 | 999 | atgctattgggataagtttatta | 208985 | - | see also 7561 line | | |
| 16239 | NMNAT2 | NM_015039.2 | 549 | ctggccgtccagaattctgattg | 209021 | - | see also 6200 line | | |
| 16240 | UGP2 | NM_006759.2 | 755 | gtgatatttacgccagtttctac | 209050 | - | see also 4768 line | | |
| 16241 | UAP1 | NM_003115.3 | 1077 | gtggcagacccacggttcattgg | 209094 | - | - | UDP-N-acetylglucosamine pyrophosphorylase | - |

Figure 46

| 16242 | PCYT2 | NM_002861.1 | 981 | gtgtcacggcaagacagaaatta | 209130 | - | see also 1904 line | | |
|-------|-------|-------------|-----|--------------------------|--------|---|---------------------|---|---|
| 16243 | FPGT | NM_003838.1 | 801 | ggggtgacattgccgatcttaaa | 209156 | - | - | - | - |
| 16244 | GMPPA | NM_013335.2 | 503 | caggacgcaatccctcaactacg | 209197 | - | see also 5403 line | | |
| 16245 | GMPPB | NM_013334.2 | 1283 | tgggtgaggacgtcatagttaat | 209211 | - | see also 5402 line | | |
| 16246 | NMNAT3 | NM_178177.2 | 878 | atgagatcagtgccacatacatc | 209221 | - | - | - | - |
| 16247 | DLG2 | NM_001364.1 | 720 | ttgtatcttgcgggtgaatgagg | 209229 | - | see also 925 line | | |
| 16248 | DLG3 | NM_021120.1 | 2213 | ccggcctcgacgtgataatgagg | 209305 | - | see also 8095 line | | |
| 16249 | MAGI-3 | NM_020965.2 | 623 | ttgcggcattacctaagtcttca | 209322 | - | see also 8053 line | | |
| 16250 | MPP1 | NM_002436.2 | 219 | ctgtatcgcatccattgaatact | 209375 | - | see also 1568 line | | |
| 16251 | MPP3 | NM_001932.2 | 487 | gagaagcttcgctattatgaaag | 209414 | - | kinase_related、signal_transduction | guanylate kinase | - |
| 16252 | PMVK | NM_006556.2 | 848 | tggagaacctgatagaatttatc | 209432 | - | kinase_related | phosphomevalonate kinase | - |
| 16253 | CKMT1 | NM_020990.2 | 412 | ttggacgctagatcagtgtatcc | 209441 | - | see also 8062 line | | |
| 16254 | CKMT2 | NM_001825.1 | 654 | agggcagttcgacgagcattacg | 209454 | - | see also 1180 line | | |
| 16255 | CEPT1 | NM_006090.2 | 703 | ttgtgcgcactggcaaacgtatg | 209489 | - | see also 4228 line | | |
| 16256 | PIGF | NM_002643.2 | 272 | ctgctgtcaatctagtactatat | 209518 | - | see also 1718 line | | |
| 16257 | CHPT1 | NM_020244.1 | 686 | ttgcgctcattggcagacttatg | 209530 | - | see also 7818 line | | |
| 16258 | DPAGT1 | NM_001382.1 | 1185 | cagctcgttcgactcttctatga | 209578 | - | see also 950 line | | |
| 16259 | PRPSAP1 | NM_002766.1 | 874 | aggcgcctataagatctatgtta | 209597 | - | kinase_related | - | - |
| 16260 | PRPSAP2 | NM_002767.2 | 217 | ttgtgtgacgccacctgaattag | 209604 | - | kinase_related | - | - |
| 16261 | TPK1 | NM_022445.2 | 228 | tggaggtgccaaccgcttatatg | 209626 | - | kinase_related | - | - |
| 16262 | PGK1 | NM_000291.2 | 115 | gggaagcgggtcgttatgagagt | 209647 | - | kinase_related | HIF-1alpha/HIF-1beta-B complex | hemolytic anemia |
| 16263 | PGK2 | NM_138733.2 | 1121 | tgggccgttaggagtatttgaat | 209680 | - | kinase_related | phosphoglycerate kinase | - |
| 16264 | PYCS | NM_002860.2 | 299 | tggtgacccgaggggatgaatgt | 209686 | - | see also 1903 line | | |
| 16265 | MGC11134 | NM_031472.2 | 527 | tggcatgcggtcccattgtgaaa | 209745 | - | - | - | - |
| 16266 | B3GALT1 | NM_020981.2 | 1237 | aggtatcgccgagttatcactgt | 209781 | - | - | - | - |
| 16267 | B3GALT2 | NM_003783.2 | 1938 | aggtatcgccaccgtaaactaca | 209829 | - | see also 2587 line | | |
| 16268 | B3GALT4 | NM_003782.2 | 974 | gggcaggcaccgcgtatcagagg | 209834 | - | - | - | - |
| 16269 | B3GALT5 | NM_006057.1 | 1106 | gtggcgagtcaggtgtacaatgt | 209853 | - | - | - | - |
| 16270 | B3GNT1 | NM_006577.3 | 1070 | tggacctcatcgggataagaagc | 209886 | - | see also 4609 line | | |
| 16271 | B4GALT1 | NM_001497.2 | 174 | tggcgtcaccctcgtttactacc | 209905 | - | - | transferase、transferring glycosyl groups | - |
| 16272 | B4GALT2 | NM_003780.2 | 574 | gcgctacggcgtctatgtcatca | 209936 | - | see also 2585 line | | |

Figure 46

| | | | | | | | | |
|---|---|---|---|---|---|---|---|---|
| 16273 | B4GALT3 | NM_003779.2 | 909 | atgttgccgttgctatgaacaag | 209953 | - | see also 2584 line | |
| 16274 | B4GALT4 | NM_003778.2 | 977 | gggtacaggttacgttacagtgg | 209980 | - | | transferase, transferring glycosyl groups |
| 16275 | ABO | NM_020469.1 | 285 | ctgggaggggcacattcaacatcg | 209993 | - | | transferase, transferring glycosyl groups |
| 16276 | B4GALT7 | NM_007255.1 | 715 | ccgcgaggacgacgagttctacc | 209998 | - | | xylosylprotein 4-beta-galactosyltransferase |
| 16277 | C1GALT1 | NM_020156.1 | 393 | tgggcccagcgttgtaacaaagt | 210011 | - | | |
| 16278 | A4GALT | NM_017436.3 | 791 | ctggacacggacttcattgttct | 210035 | - | | |
| 16279 | B3GNT3 | NM_014256.2 | 901 | gagcggtacccaccccattgtgg | 210049 | - | | transferase, transferring glycosyl groups |
| 16280 | B3GNT4 | NM_030765.1 | 759 | acgtcccaacgttagagttc | 210059 | - | | |
| 16281 | B3GNT5 | NM_032047.2 | 762 | atgatcgacgttccggaattaga | 210083 | - | | |
| 16282 | B3GNT7 | NM_145236.1 | 425 | gggacgtgaccaccaactacgc | 210112 | - | | |
| 16283 | B4GALT5 | NM_004776.2 | 834 | gtgatcgcaactattatggatgt | 210138 | - | | transferase, transferring glycosyl groups |
| 16284 | IMAGE:4907098 | NM_138706.1 | 773 | gcgacagctgagcaagtacttc | 210144 | - | | |
| 16285 | MGC39558 | NM_152490.1 | 1200 | atgttgtcgacacttatcgtaat | 210169 | - | | |
| 16286 | MGC4655 | NM_033309.1 | 1109 | gcgggctagcaagtactacatcc | 210196 | - | | |
| 16287 | DSCR5 | NM_016430.2 | 637 | ttgcagccaaagaactttacacc | 210207 | - | see also 6856 line | |
| 16288 | PIGA | NM_002641.1 | 1465 | acgggtgcctggacctaataact | 210251 | - | see also 1714 line | |
| 16289 | PIGC | NM_153747.1 | 1030 | tgggaggcctactgtccattagt | 210272 | - | | enzyme |
| 16290 | PIGQ | NM_004204.2 | 863 | tggagcacctcaagctaatcttc | 210279 | - | see also 2813 line | |
| 16291 | C2GNT3 | NM_016591.1 | 1077 | ctgttcggtatctatgaacagg | 210294 | - | | |
| 16292 | GCNT2 | NM_001491.2 | 1634 | ctggctctatgccaaatgcatcc | 210351 | - | | |
| 16293 | GCNT3 | NM_004751.1 | 1512 | cagcgggctatctgcgtttatgg | 210423 | - | | lung squamous cell carcinoma |
| 16294 | FLJ20277 | NM_017739.1 | 436 | ctggacgtagaggtgttattcaag | 210428 | - | see also 7128 line | |
| 16295 | MGAT1 | NM_002406.2 | 1641 | gggaggtgcgggtgcagtatacg | 210446 | - | | transferase, transferring glycosyl groups |
| 16296 | MGAT4A | NM_012214.1 | 139 | aggctccgcaatgaactgtagc | 210451 | - | see also 5202 line | |

Figure 46

| 16297 | MGAT4B | NM_014275.2 | 138 | aggcgacgttgtggacgtttacc | 210490 | - | see also 5576 line | | | |
| 16298 | GCNT1 | NM_001490.3 | 1252 | aagcggtatgaggtcgttaatgg | 210552 | - | - | transferase、transferring glycosyl groups | | - |
| 16299 | B3GNT6 | NM_006876.1 | 675 | atgcgctgggcaccaatgtctcc | 210565 | - | - | - | | - |
| 16300 | MGAT2 | NM_002408.2 | 954 | ctggtcgaccgagatcaatcagc | 210578 | - | see also 1557 line | | | |
| 16301 | MGAT5 | NM_002410.2 | 3941 | ttggtactgaacccgaatttaat | 210634 | - | - | | Ets1/ Ets2 complex | - |
| 16302 | alpha4GnT | NM_016161.1 | 734 | aggcttctcggtactctagtaat | 210667 | - | - | - | | - |
| 16303 | XYLT1 | NM_022166.1 | 2333 | gagaggctattccgcaactttgg | 210691 | - | - | - | | - |
| 16304 | XYLT2 | NM_022167.1 | 2351 | ttgaccttcaaccgcaaactacc | 210711 | - | see also 8320 line | | | |
| 16305 | FUT1 | NM_000148.1 | 766 | tggggactatctgcaggttatgc | 210719 | - | - | transferase、transferring glycosyl groups | | Bombay phenotype |
| 16306 | FUT2 | NM_000511.1 | 191 | aggacctgttcactatcaactcc | 210725 | - | - | transferase、transferring glycosyl groups | | - |
| 16307 | FUT8 | NM_004480.3 | 2085 | acggtcaagtaccccacatatcc | 210773 | - | see also 3030 line | | | |
| 16308 | FUT10 | NM_032664.3 | 514 | aggaagcacctacgcatcttaat | 210788 | - | - | - | | - |
| 16309 | FUT11 | NM_173540.1 | 977 | agggactggatgccgaacaatca | 210828 | - | - | - | | - |
| 16310 | FUT4 | NM_002033.1 | 1347 | gggcagccggtgcccgaaattgg | 210841 | - | - | transferase、transferring glycosyl groups | | leukemia |
| 16311 | FUT7 | NM_004479.1 | 554 | gcgcgactcggacatctttgtgc | 210846 | - | - | transferase、transferring glycosyl groups | | - |
| 16312 | FUT9 | NM_006581.2 | 1319 | atgtttggcttgcgatcatgtga | 210887 | - | see also 4610 line | | | |
| 16313 | UGT1A10 | NM_019075.1 | 1096 | gcgaacaacacgatacttgttaa | 329679 | - | - | human glucuronosyltransferases | | - |
| 16314 | UGT2A1 | NM_006798.1 | 525 | ttgtggcgatatagtagctttaa | 210903 | - | - | - | | - |
| 16315 | UGT2B10 | NM_001075.2 | 355 | gtgggcaattaatgacataatta | 210933 | - | - | human glucuronosyltransferases | | - |
| 16316 | UGT2B11 | NM_001073.1 | 209 | atgatgcatccactcttaaattt | 210934 | - | - | human glucuronosyltransferases | | - |
| 16317 | UGT2B15 | NM_001076.1 | 529 | ctgtacagtcttcgattctctgt | 210943 | - | - | human glucuronosyltransferases | | - |

Figure 46

EP 1 752 536 A1

| 16318 | UGT2B17 | NM_001077.1 | 1078 | aagaagccaaatactttaggttc | 210947 | - | - | human glucuronosyltransferases | - |
|---|---|---|---|---|---|---|---|---|---|
| 16319 | UGT2B28 | NM_053039.1 | 82 | tggtgtggaccggtgaatacagc | 210948 | - | - | human glucuronosyltransferases | - |
| 16320 | UGT2B4 | NM_021139.1 | 238 | cagcccatctactcttaaatttg | 210950 | - | - | human glucuronosyltransferases | - |
| 16321 | UGT2B7 | NM_001074.1 | 346 | aagtacaggaaatcatgtcaata | 210962 | - | - | human glucuronosyltransferases | - |
| 16322 | GALNT2 | NM_004481.2 | 569 | ttgagaaagtgcgagttcttaga | 210970 | - | see also 3033 line | | |
| 16323 | GALNT3 | NM_004482.2 | 592 | tggtccacgttgcttagaactgt | 211020 | - | see also 3035 line | | |
| 16324 | GALNT5 | NM_014568.1 | 2089 | gtggcctgtccagtaatcgaagt | 211101 | - | see also 5755 line | | |
| 16325 | GALNT7 | NM_017423.1 | 1011 | gtgccgcttatagatgtcataaa | 211179 | - | see also 6998 line | | |
| 16326 | GALNT8 | NM_017417.1 | 370 | atgaagtacgccctcttctaaag | 211213 | - | - | - | - |
| 16327 | GALNT9 | NM_021808.2 | 538 | cggagatgagggtctacaacaac | 211246 | - | see also 8175 line | | |
| 16328 | GALGT | NM_001478.2 | 1283 | aagaccttcctccgttatgatcg | 211255 | - | - | transferase、 transferring glycosyl groups | - |
| 16329 | DDOST | NM_005216.3 | 1033 | ctgacctagtggagtatagcatc | 211278 | - | - | dolichyl-diphospho-oligosaccharide-protein glycosyltransferase | - |
| 16330 | RPN2 | NM_002951.2 | 1349 | gtgacaaccggtatattgcaaat | 211303 | - | see also 1993 line | | |
| 16331 | ALG5 | NM_013338.3 | 622 | tggatctcgagctcatttagaaa | 211336 | - | see also 5404 line | | |
| 16332 | ALG6 | NM_013339.2 | 1142 | ttgccacgtcacatccaattaat | 211386 | - | - | - | congenital disorder of glycosylation |
| 16333 | ITM1 | NM_152713.2 | 1575 | ggggatggcagtaggatcatatt | 211443 | - | see also 9930 line | | |
| 16334 | SIMP | NM_178862.1 | 299 | acgagttcgacccgtggtttaac | 211461 | - | - | - | - |
| 16335 | PYGB | NM_002862.3 | 1990 | cggcgacgtcgtcaatcatgacc | 211568 | - | - | transferase、 transferring glycosyl groups | colorectal cancer、 colorectal cancers、 gastric cancer |
| 16336 | PYGL | NM_002863.1 | 788 | ctgctcgggcaccaaatgacttt | 211582 | - | see also 1905 line | | |
| 16337 | PYGM | NM_005609.1 | 2522 | aagcggattcacgaatataaacg | 211646 | - | see also 3897 line | | |
| 16338 | MTAP | NM_002451.2 | 144 | ccgccgtgaagattggaataatt | 211661 | - | - | transferase、 transferring glycosyl groups | - |

659

Figure 46

| | | | | | | | | | |
|---|---|---|---|---|---|---|---|---|---|
| 16339 | NP | NM_000270.1 | 600 | atgcctacgaccggactatgagg | 211694 | - | - | transferase、transferring glycosyl groups | immunodeficiency |
| 16340 | GYG | NM_004130.2 | 369 | ctggtcctagcaaatattgatga | 211702 | - | see also 2787 line | | |
| 16341 | GYG2 | NM_003918.1 | 1296 | cgggcgtgccgtgtgcaaattca | 211744 | - | - | glycogenin glucosyltransferase | - |
| 16342 | UGCGL1 | NM_020120.1 | 310 | atgacggtaccgattattcctac | 211765 | - | - | - | - |
| 16343 | GYS1 | NM_002103.3 | 1282 | atgccagcgcggaccaacaattt | 211901 | - | - | transferase、transferring glycosyl groups | diabetes mellitus、diabetes |
| 16344 | UGCG | NM_003358.1 | 1355 | ccgcgaatccatgacaatataca | 211956 | - | see also 2263 line | | |
| 16345 | POMT2 | NM_013382.3 | 1895 | tggcactggcctatcaactatca | 211986 | - | see also 5415 line | | |
| 16346 | SDF2 | NM_006923.2 | 336 | tggccgaaacctccatagtcacc | 211992 | - | - | dolichyl-phosphate-mannose-protein mannosyltransferase | - |
| 16347 | DPM1 | NM_003859.1 | 672 | tggcgaggttccaatatcatttg | 212013 | - | see also 2623 line | | |
| 16348 | AD-017 | NM_018446.1 | 246 | tagggcctcaacctatagacttt | 212018 | - | see also 7481 line | | |
| 16349 | FLJ21934 | NM_024743.1 | 416 | gtgcctatgacaggactaacaga | 212073 | - | - | - | - |
| 16350 | FLJ34658 | NM_152404.2 | 507 | ttgggactcaatgtagttatttg | 212083 | - | - | - | - |
| 16351 | FLJ35155 | NM_152531.3 | 1190 | acgtcttcgaggcctatttcagg | 212099 | - | - | - | - |
| 16352 | HGNT-IV-H | NM_013244.2 | 872 | aagaaagcggtatcttacaattg | 212114 | - | see also 5360 line | | |
| 16353 | LARGE | NM_004737.2 | 1784 | ctggagtatgacggcaatcttct | 212167 | - | see also 3263 line | | |
| 16354 | LOC167127 | NM_174914.2 | 233 | aagatcacggtcataatgtcacc | 212175 | - | - | - | - |
| 16355 | LOC83468 | NM_031302.2 | 669 | cggaatactctgactcgaatacg | 212193 | - | - | - | - |
| 16356 | UGCGL2 | NM_020121.2 | 1682 | ctggcgagctttcaactatattg | 212267 | - | - | - | - |
| 16357 | ADPRTL1 | NM_006437.2 | 3126 | cagcaaatcgtcacgtcttaagg | 212437 | - | - | transferase、transferring glycosyl groups | - |
| 16358 | ADPRTL3 | NM_005485.2 | 687 | gggcaagtacacacttatcgaag | 212481 | - | - | transferase、transferring glycosyl groups | - |
| 16359 | ART1 | NM_004314.1 | 962 | tgggccttgccatctggataatt | 212493 | - | - | transferase、transferring glycosyl groups | - |
| 16360 | ART3 | NM_001179.2 | 152 | atgctgctggcaaccatgattct | 212495 | - | - | transferase、transferring glycosyl groups | - |

Figure 46

| | | | | | | | | | |
|---|---|---|---|---|---|---|---|---|---|
| 16361 | TNKS2 | NM_025235.2 | 1824 | aagagggtatctcattaggtaat | 212555 | - | - | transferase、 transferring glycosyl groups | - |
| 16362 | ART5 | NM_053017.2 | 1168 | gggaacgggtgaccttcatatga | 212621 | - | - | transferase、 transferring glycosyl groups | - |
| 16363 | DO | NM_021071.1 | 914 | ctgggaacctgagcacatataac | 212654 | - | - | transferase、 transferring glycosyl groups | Dombrock blood group -(peripheromyopathy-) |
| 16364 | PRTFDC1 | NM_020200.5 | 62 | cgggcgaggcgtcgtgattatgg | 212658 | - | - | - | - |
| 16365 | QTRT1 | NM_031209.1 | 761 | aggacaagccccgatatctgatg | 212697 | - | see also 8990 line | | |
| 16366 | QTRTD1 | NM_024638.2 | 239 | gagtcttaccaaggtagttaatg | 212698 | - | - | - | - |
| 16367 | UPP1 | NM_003364.2 | 858 | gtgctccaacgtcactatcatcc | 212736 | - | - | - | - |
| 16368 | UMPS | NM_000373.1 | 413 | tggaactaagcgtcttgtagaag | 212747 | - | see also 299 line | | |
| 16369 | SIAT4A | NM_003033.2 | 1328 | gggagaagaagcccaataacttg | 212777 | - | - | - | - |
| 16370 | SIAT4B | NM_006927.2 | 1401 | cagccactttgacggtaacattt | 212780 | - | - | - | - |
| 16371 | SIAT4C | NM_006278.1 | 1064 | cggggtcaggccataatgtctcc | 212800 | - | see also 4368 line | | |
| 16372 | SIAT7C | NM_152996.1 | 610 | ttggggacctttccgcaaatga | 212818 | - | see also 9971 line | | |
| 16373 | SIAT7D | NM_014403.3 | 284 | gggtcggctcgtgctcatcatcc | 212832 | - | - | - | - |
| 16374 | SIAT1 | NM_003032.2 | 1115 | tggtaccagaatccggattataa | 212862 | - | - | - | - |
| 16375 | SIAT8A | NM_003034.2 | 932 | tggctgtggccgtcaaatagatg | 212883 | - | - | transferase、 transferring glycosyl groups | melanoma |
| 16376 | SIAT9 | NM_003896.2 | 514 | caggtatagcgtggacttactcc | 212902 | - | see also 2653 line | | |
| 16377 | SIAT7A | NM_018414.2 | 1334 | tgggcaatcggggtttcaagaac | 212929 | - | - | - | - |
| 16378 | SIAT10 | NM_006100.2 | 690 | ttgtatggtagcgataagtttga | 212970 | - | - | molecular_function unknown | - |
| 16379 | SIAT7E | NM_030965.1 | 1093 | ctgggcacggacattcaatattc | 213003 | - | - | transferase、 transferring glycosyl groups | - |
| 16380 | SIAT8B | NM_006011.2 | 162 | cagcttacatagcaaatctaata | 213008 | - | see also 4187 line | | |
| 16381 | SIAT8C | NM_015879.1 | 2192 | gcgggattccggtcacaatttgc | 213026 | - | see also 6552 line | | |
| 16382 | SIAT8D | NM_005668.3 | 333 | tggacgatctgcacaataagtct | 213060 | - | see also 3938 line | | |
| 16383 | SIAT8E | NM_013305.2 | 630 | atggcaggaactacattaagagg | 213098 | - | - | - | - |
| 16384 | ST6GalII | NM_032528.1 | 1080 | caggcgcaatcctcaactcttcc | 213126 | - | - | - | - |
| 16385 | ST6GALNAC6 | NM_013443.3 | 507 | gagtgtacaatccgcatgaatga | 213146 | - | - | - | - |

Figure 46

| 16386 | STHM | NM_006456.1 | 464 | gggacacccctccaaagtgtatc | 213158 | - | - | transferase、transferring glycosyl groups | - |
|---|---|---|---|---|---|---|---|---|---|
| 16387 | ALG12 | NM_024105.2 | 1151 | gagctacgcttcatcatctatgc | 213182 | - | - | - | - |
| 16388 | ALG3 | NM_005787.3 | 308 | cagctggtttcgtgtgtacatcttt | 213190 | - | see also 4045 line | | |
| 16389 | ALG8 | NM_024079.2 | 407 | ctgtccgtgagtgctgtaaatgc | 213205 | - | see also 8582 line | | |
| 16390 | C1GALT2 | NM_152692.2 | 685 | acgccccactacgtttgctatca | 213263 | - | - | - | - |
| 16391 | DPM2 | NM_003863.2 | 145 | cagcatgtcatccacaagtattt | 213274 | - | - | - | - |
| 16392 | EXT1 | NM_000127.1 | 2708 | gtggaccaattggccaattgtga | 213342 | - | see also 100 line | | |
| 16393 | EXT2 | NM_000401.1 | 1022 | aggtgggatcgaggtacgaatca | 213364 | - | see also 321 line | | |
| 16394 | EXTL1 | NM_004455.1 | 1141 | ggggcgatggccttaaggtattc | 213408 | - | - | transferase、transferring glycosyl groups | - |
| 16395 | EXTL2 | NM_001439.1 | 330 | gggattcgagtgcttcgattatc | 213430 | - | - | transferase、transferring glycosyl groups | - |
| 16396 | EXTL3 | NM_001440.1 | 1320 | ctgcctttacgtgatactagtgg | 213472 | - | see also 1009 line | | |
| 16397 | HAS1 | NM_001523.1 | 952 | gcggtcctctaggcctatatagg | 213501 | - | - | transferase、transferring glycosyl groups | - |
| 16398 | HAS2 | NM_005328.1 | 796 | ttgcatcgctgcctatcaagaag | 213519 | - | see also 3713 line | | |
| 16399 | MFNG | NM_002405.2 | 1053 | gaggaggacccctccagatttcg | 213592 | - | - | transferase、transferring glycosyl groups | - |
| 16400 | SMP3 | NM_025163.2 | 965 | tggccacggacagctggtatttc | 213595 | - | - | - | - |
| 16401 | BAT8 | NM_006709.2 | 134 | ggggacacccctcgtagtgaaga | 213597 | - | - | - | - |
| 16402 | SET7 | NM_030648.1 | 478 | cagggagtttacacttacgaaga | 213631 | - | see also 8921 line | | |
| 16403 | C20orf36 | NM_018257.1 | 602 | cagtatgatcgtgtatactgtgg | 213667 | - | - | - | - |
| 16404 | LOC115294 | NM_052937.1 | 946 | gagaattaacacttacgtatttg | 213707 | - | see also 9460 line | | |
| 16405 | PCMT1 | NM_005389.1 | 572 | caggcgctaatagatcagttaaa | 213727 | - | - | protein-L-isoaspartate (D-aspartate) O-methyltransferase | - |
| 16406 | BHMT | NM_001713.1 | 281 | caggggcaactatgtcttagaga | 213744 | - | - | homocysteine S-methyltransferase | - |
| 16407 | BHMT2 | NM_017614.3 | 362 | ctgccagacatcaatatacaaat | 213766 | - | - | - | - |
| 16408 | MTR | NM_000254.1 | 717 | gggctctgggtccgactaataag | 213779 | - | - | homocysteine S-methyltransferase | homocystinuria、Methylcobalamin deficiency |

Figure 46

| | | | | | | | | | |
|---|---|---|---|---|---|---|---|---|---|
| 16409 | METTL1 | NM_005371.3 | 961 | gggggctggtgtataccataacc | 213858 | - | - | - | - |
| 16410 | HSA9761 | NM_014473.1 | 745 | tgggatggtctagtaaggataac | 213881 | - | see also 5699 line | | |
| 16411 | TFB1M | NM_016020.1 | 648 | gcgtagtcgcctctctgttatgg | 213916 | - | see also 6621 line | | |
| 16412 | FLJ10140 | NM_018006.2 | 627 | gggggggattaacgaaagagtttg | 213941 | - | - | - | - |
| 16413 | ICMT | NM_012405.2 | 679 | ttggtttcggcatccttcttacg | 213966 | - | - | - | - |
| 16414 | PEMT | NM_007169.2 | 514 | gggttcgctggaactttcctagg | 213971 | - | - | - | - |
| 16415 | PNMT | NM_002686.2 | 974 | gggacctccgcacctatatcatg | 213986 | - | - | phenylethanolamine N-methyltransferase | - |
| 16416 | COQ3 | NM_017421.2 | 105 | ctgcgcgtcccttaatttcctcg | 213989 | - | see also 6997 line | | |
| 16417 | CGI-30 | NM_015958.1 | 787 | cagttaccgaggagacactttgt | 214045 | - | - | - | - |
| 16418 | INMT | NM_006774.3 | 181 | aggggacacgctgattgacattg | 214054 | - | - | methyltransferase | - |
| 16419 | TPMT | NM_000367.1 | 609 | ctgtgtgttctttcttatgatcc | 214065 | - | - | thiopurine S-methyltransferase | thiopurine S-methyltransferase deficiency、leukemia |
| 16420 | NNMT | NM_006169.1 | 823 | gaggtgatctcgcaaagttattc | 214077 | - | - | nicotinamide N-methyltransferase | - |
| 16421 | AD-003 | NM_014064.1 | 397 | aggtggatatggtcgacataacg | 214081 | - | - | - | - |
| 16422 | C2orf8 | NM_025264.2 | 1197 | ttggcggaaccattgtattgttg | 214115 | - | - | - | - |
| 16423 | CGI-01 | NM_015935.2 | 1499 | acggccttgctacgatgtcataa | 214141 | - | - | molecular_function unknown | - |
| 16424 | CYT19 | NM_020682.2 | 603 | aagcatggtggggagttatattt | 214166 | - | - | - | - |
| 16425 | FLJ10640 | NM_019023.1 | 1703 | tgggcgagccgttcttcactacc | 214200 | - | see also 7664 line | | |
| 16426 | FLJ20628 | NM_017910.2 | 70 | ggggctcggaaccaattcattcc | 214205 | - | - | - | - |
| 16427 | FLJ23841 | NM_144589.2 | 774 | gtgtgtgcgaaacctaaacgaac | 214260 | - | - | - | - |
| 16428 | HNMT | NM_006895.1 | 875 | tggacaacctagggcttaagtat | 214277 | - | see also 4826 line | | |
| 16429 | HRMT1L1 | NM_001535.1 | 394 | gggtacattccggcaaaccatgt | 214286 | - | signal_transduction | signal transducer | - |
| 16430 | HRMT1L2 | NM_001536.2 | 226 | ccgcaactccatgtttcataacc | 214303 | - | signal_transduction | S-adenosylmethionine-dependent methyltransferase | - |
| 16431 | HRMT1L3 | NM_019854.2 | 696 | ttgtacgtggtagcgattgaaga | 214318 | - | - | S-adenosylmethionine-dependent methyltransferase | - |
| 16432 | LCMT1 | NM_016015.2 | 752 | aagagatatgccgttattggagc | 214341 | - | - | - | - |
| 16433 | MGC17301 | NM_152637.1 | 692 | gagcccacctggaaacacattgg | 214361 | - | - | - | - |
| 16434 | MGC2408 | NM_032331.2 | 135 | ccgattctgcccctacgattgg | 214362 | - | - | - | - |
| 16435 | MGC24132 | NM_152396.1 | 788 | tcgtgactacggactgtatgatc | 214391 | - | - | - | - |

Figure 46

| 16436 | MGC50559 | NM_173802.2 | 743 | atggctattacactaaattgtga | 214410 | - | - | - | - |
|---|---|---|---|---|---|---|---|---|---|
| 16437 | NCOA6IP | NM_024831.5 | 498 | ccgcgtggcggaaatgtttctct | 214422 | - | - | - | - |
| 16438 | SRM | NM_003132.1 | 576 | atgccttcgacgtgatcatcact | 214505 | - | - | spermidine synthase | - |
| 16439 | WBSCR22 | NM_017528.2 | 677 | gggccttcgacctttataccaga | 214518 | - | - | S-adenosylmethionine-dependent methyltransferase | - |
| 16440 | GART | NM_000819.3 | 1349 | tcggctttcgtgccatagctttc | 214559 | - | - | - | - |
| 16441 | MtFMT | NM_139242.2 | 726 | gcgacttacgcccctaagatttc | 214621 | - | - | - | - |
| 16442 | FTHFD | NM_012190.2 | 1393 | gggcgagtgcagcattgactacg | 214656 | - | - | - | - |
| 16443 | SHMT1 | NM_004169.3 | 274 | cagtgatgttgaggtttacaaca | 214673 | - | - | - | - |
| 16444 | SHMT2 | NM_005412.3 | 931 | ccgggagatcccttacacatttg | 214698 | - | - | glycine hydroxymethyltransferase | - |
| 16445 | AMT | NM_000481.1 | 1326 | ttgtgcccacaaactactatacc | 214720 | - | - | transaminase | glycine encephalopathy |
| 16446 | DMGDH | NM_013391.2 | 1480 | cagccgcactggttctacaaacc | 214772 | - | see also 5421 line | | |
| 16447 | PDPR | NM_017990.3 | 3087 | tcgcagtctccgaattgagaagt | 214809 | - | see also 7190 line | | |
| 16448 | SARDH | NM_007101.2 | 1774 | gagcgaccgggatggtttcatcc | 214831 | - | - | - | - |
| 16449 | TYMS | NM_001071.1 | 623 | acgacagaagaatcatcatgtgc | 214846 | - | - | thymidylate synthase | - |
| 16450 | FLJ20257 | NM_019606.3 | 2046 | ttgtcttcgtcacgggtaattat | 214864 | - | - | - | - |
| 16451 | FTSJ1 | NM_012280.2 | 667 | gtgacggggctcctgatgtaacc | 214874 | - | - | - | - |
| 16452 | FTSJ2 | NM_013393.1 | 244 | ccggccttcgggtgttagactgt | 214883 | - | - | - | - |
| 16453 | LOC57019 | NM_020313.1 | 719 | tcgaagcctgcagtcactttagc | 214900 | - | - | - | - |
| 16454 | GATM | NM_001482.1 | 544 | gagtctacgggtttatacagtgc | 214941 | - | see also 1021 line | | |
| 16455 | NAT1 | NM_000662.3 | 621 | tggtatctagaccaaatcagaac | 214971 | | - | arylamine N-acetyltransferase | bladder cancer、lung cancer |
| 16456 | NAT2 | NM_000015.1 | 684 | acgcttgaacctcgaacaattga | 214986 | - | - | arylamine N-acetyltransferase | bladder cancer、Parkinson disease |
| 16457 | SAT | NM_002970.1 | 563 | atgaaccatccatcaacttctat | 215007 | - | - | diamine N-acetyltransferase | - |
| 16458 | NAGS | NM_153006.1 | 897 | gcgcgacagcagtcataaggtcc | 215017 | - | kinase_related | - | - |
| 16459 | CML2 | NM_016347.1 | 278 | ctggacgcggtatgtagacatag | 215029 | - | - | - | - |
| 16460 | CSRP2BP | NM_020536.2 | 2180 | ttgttagttgacgggatttatgg | 215051 | - | see also 7892 line | | |
| 16461 | DKFZP564C103 | NM_015654.3 | 477 | acgctaggtctgaccaagtttga | 215078 | - | - | - | - |
| 16462 | ELP3 | NM_018091.3 | 52 | atgctgactataggagatgttat | 215084 | - | see also 7252 line | | |

EP 1 752 536 A1

Figure 46

| | | | | | | | | | |
|---|---|---|---|---|---|---|---|---|---|
| 16463 | FLJ13848 | NM_024771.1 | 713 | gggaggattgctcctatgagatc | 215144 | - | - | - | - |
| 16464 | KLP1 | NM_020378.2 | 417 | ctggcccctggcacaaatgcagg | 215157 | - | transcription_regulator | - | - |
| 16465 | MAK3P | NM_025146.1 | 484 | agggtggatcattcacagaatca | 215164 | - | see also 8871 line | | |
| 16466 | NAT5 | NM_016100.2 | 361 | ttggtttggctgctaaacttatg | 215183 | - | see also 6660 line | | |
| 16467 | SAT2 | NM_133491.2 | 432 | atgggatatactatttcatctac | 215203 | - | - | - | - |
| 16468 | GTF3C4 | NM_012204.1 | 1407 | gtgtagttgcagcttagtagtgg | 215233 | - | transcription_regulator | - | - |
| 16469 | ACAT1 | NM_000019.2 | 401 | ttgggtgcaggcttacctatttc | 215258 | - | - | acetyl-CoA C-acetyltransferase | alpha-methylacetoaceticaciduria |
| 16470 | HADHA | NM_000182.3 | 1898 | aagggcttcctaggtcgtaaatc | 215339 | - | see also 155 line | | |
| 16471 | GCAT | NM_014291.2 | 1203 | gtgcatagcgaggaagacattga | 215362 | - | - | transaminase | - |
| 16472 | CHAT | NM_020549.2 | 1272 | caggacggtcctcgtgaaagact | 215367 | - | - | - | - |
| 16473 | CRAT | NM_000755.2 | 692 | cagggtcagctccgatttgctgc | 215382 | - | - | - | Alzheimer disease |
| 16474 | GNPAT | NM_014236.1 | 1183 | gagtcggagctcatataacttgg | 215427 | - | - | glycerone-phosphate O-acyltransferase | chondrodysplasia |
| 16475 | NMT1 | NM_021079.3 | 1025 | ctgggctgcgaccaatggaaaca | 215454 | - | see also 8079 line | | |
| 16476 | NMT2 | NM_004808.1 | 560 | aaggagttatacacgttgttaaa | 215471 | - | see also 3342 line | | |
| 16477 | AGPAT1 | NM_006411.2 | 495 | aggacgcaacgtcgagaacatga | 215505 | - | - | - | - |
| 16478 | AGPAT4 | NM_020133.1 | 1205 | gtgggagttcgatggatgattgg | 215529 | - | - | - | - |
| 16479 | LPAAT-e | NM_018361.1 | 1044 | ctgcctgtgggttactattaaag | 215556 | - | see also 7414 line | | |
| 16480 | CPT1A | NM_001876.1 | 2050 | ctgcctttacgtggtgtctaaat | 215598 | - | - | Peroxisome proliferator activated receptor alpha | hypoglycemia |
| 16481 | CPT1B | NM_004377.2 | 1705 | cagacgacgtggagttgtactgc | 215621 | - | - | - | - |
| 16482 | CPT2 | NM_000098.1 | 1980 | ctgtagcactgccgcattcaagc | 215651 | - | - | carnitine O-palmitoyltransferase | hypoglycemia、myopathy、Alzheimer diseasecarnitine palmitoyltransferase II (CPT II) deficiency |
| 16483 | CROT | NM_021151.2 | 1372 | ttgcggcttatgcctttacatct | 215715 | - | - | carnitine O-octanoyltransferase | - |
| 16484 | SOAT1 | NM_003101.3 | 2219 | gtgcctcgggtactaaattcagc | 215761 | - | - | sterol O-acyltransferase | - |
| 16485 | SOAT2 | NM_003578.2 | 255 | gcgggaggctatacaatcctacc | 215789 | - | - | sterol O-acyltransferase | atherosclerosis |
| 16486 | LCAT | NM_000229.1 | 295 | gggtagactgctggatcgataac | 215804 | - | - | phosphatidylcholine-sterol O-acyltransferase | atherosclerosis、fish-eye disease、Norum disease (lecithin-cholesterol acyltransferase deficiency) |
| 16487 | KIAA1560 | NM_020918.2 | 735 | ctgtctcaccggcaatgatcaga | 215830 | - | see also 8021 line | | |

665

EP 1 752 536 A1

Figure 46

| | | | | | | | | | |
|---|---|---|---|---|---|---|---|---|---|
| 16488 | ACAA1 | NM_001607.2 | 1104 | gtggacatcttcgagatcaatga | 215903 | - | - | acetyl-CoA C-acyltransferase | pseudo-Zellweger syndrome |
| 16489 | ACAA2 | NM_006111.1 | 55 | ctgctccgaggtgtgtttgtagt | 215907 | - | - | acetyl-CoA C-acyltransferase | - |
| 16490 | HADHB | NM_000183.1 | 626 | cagcgactgtctttaatctctaa | 215925 | - | - | enoyl-CoA hydratase | trifunctional protein deficiency |
| 16491 | SPTLC2 | NM_004863.2 | 1007 | aggagcaaccattagaatcttca | 216004 | - | see also 3378 line | | |
| 16492 | DLST | NM_001933.3 | 939 | gtgattgacgacacaaccaaaga | 216031 | - | - | dihydrolipoamide S-succinyltransferase | - |
| 16493 | ALAS1 | NM_000688.4 | 1381 | cagccgagtgccaaagtacatct | 216054 | - | - | transaminase | - |
| 16494 | ALAS2 | NM_000032.1 | 1723 | ctgtcgccgtcctgtacactttg | 216076 | - | see also 4 line | | |
| 16495 | ATE1 | NM_007041.1 | 997 | caggatccacccgatgaatgtgg | 216101 | - | see also 4930 line | | |
| 16496 | AUP1 | NM_012103.2 | 1303 | gtgtagacttgactatcactaat | 216131 | - | see also 5132 line | | |
| 16497 | BAAT | NM_001701.1 | 1271 | ctgatagaacctccctattctcc | 216177 | - | - | - | - |
| 16498 | CPT1C | NM_152359.1 | 1542 | atgatcgctggtttgacaaatcc | 216190 | - | - | - | - |
| 16499 | DGAT2 | NM_032564.2 | 629 | gcgctactttcgagactactttc | 216207 | - | see also 9248 line | | |
| 16500 | DGAT2L1 | NM_058165.1 | 953 | aggagttacatcagacctatatg | 216239 | - | - | - | - |
| 16501 | DKFZp586M1819 | NM_178819.2 | 1740 | gggactcatgggtgtgattcaga | 216268 | - | see also 10210 line | | |
| 16502 | FLJ32370 | NM_152417.1 | 772 | tcgagaagccctaattagtgatg | 216287 | - | see also 9860 line | | |
| 16503 | FLJ37965 | NM_182551.1 | 607 | ttgcctgatgcgatatagctacc | 216301 | - | - | - | - |
| 16504 | GGTLA1 | NM_004121.1 | 1204 | ggggtgccattctcagctttatc | 216311 | - | - | gamma-glutamyl transferase | - |
| 16505 | GLYAT | NM_005838.1 | 284 | atgaccttgatcactataccaat | 216319 | - | - | - | - |
| 16506 | KIAA0205 | NM_014873.1 | 377 | cagtaagcggttctggtatatcg | 216335 | - | see also 6069 line | | |
| 16507 | LRAT | NM_004744.1 | 713 | ctgtacgggcttggtatcataca | 216374 | - | - | - | retinal dystrophy |
| 16508 | MGAT2 | NM_025098.2 | 717 | tggctcctggttacgctatatcc | 216387 | - | - | - | - |
| 16509 | MGAT3 | NM_178176.2 | 631 | cggtctatcgcgactacatcatg | 216403 | - | - | - | - |
| 16510 | MGC11324 | NM_032717.3 | 264 | gtgggccacaatacgaattgaaa | 216413 | - | - | - | - |
| 16511 | QPCT | NM_012413.2 | 357 | gggtaccggtctttctcaaatat | 216450 | - | - | glutaminyl-peptide cyclotransferase | - |
| 16512 | TAZ | NM_000116.2 | 937 | ctgtggcatgtcggaatgaatga | 216476 | - | - | - | methylglutaconic aciduria、cardiomyopathy、endocardial fibroelastosis |
| 16513 | GGTL3 | NM_052830.3 | 632 | atgagagccacctaattgatttc | 216485 | - | - | - | - |
| 16514 | HMGCS1 | NM_002130.3 | 802 | gtgcattagaccgctgctattct | 216520 | - | see also 1367 line | | |
| 16515 | HMGCS2 | NM_005518.1 | 985 | ctggctcgcctgatgttcaatga | 216567 | - | see also 3849 line | | |

Figure 46

| | | | | | | | | | |
|---|---|---|---|---|---|---|---|---|---|
| 16516 | CS | NM_004077.2 | 922 | aggctatactgatcatcagttca | 216588 | - | - | citrate (Sl)-synthase | - |
| 16517 | CLYBL | NM_138280.2 | 818 | aggtgattcaccctaaccaaatt | 216609 | - | see also 9591 line | | - |
| 16518 | GSTA4 | NM_001512.2 | 722 | aagaagcctcccctgatgaaat | 216622 | - | - | glutathione transferase | - |
| 16519 | GSTM1 | NM_000561.2 | 385 | ctggccatgatctgctacaatcc | 216626 | - | - | | bladder cancer, breast cancer, lung cancer, head and neck cancer, non-small-cell lung cancer, head and neck squamous cell carcinoma |
| 16520 | GSTM2 | NM_000848.2 | 513 | gtggatttcatcgcttatgatgt | 216629 | - | - | glutathione transferase | - |
| 16521 | GSTM3 | NM_000849.3 | 207 | gagttcacggatacctccttatga | 216632 | - | - | glutathione transferase | - |
| 16522 | GSTM4 | NM_000850.3 | 736 | gggaagaggccatggttgttgg | 216643 | - | - | | - |
| 16523 | GSTO1 | NM_004832.1 | 71 | aggggctcgatccgcatctacagc | 216645 | - | - | | - |
| 16524 | GSTO2 | NM_183239.1 | 690 | agggggctcgatccgcatctacagc | 216658 | - | see also 10291 line | | - |
| 16525 | GSTP1 | NM_000852.2 | 144 | tggcaggagggctcactcaaagc | 216680 | - | - | Smad3/Sp-1 heterodimer | breast cancer, Parkinson disease |
| 16526 | GSTT1 | NM_000853.1 | 88 | ctgcgcatcgtgatctgattaa | 216684 | - | - | glutathione transferase | breast cancer, head and neck cancer, head and neck squamous cell carcinoma |
| 16527 | GSTT2 | NM_000854.2 | 262 | aagtcggccatcctgatttacc | 216692 | - | - | glutathione transferase | - |
| 16528 | GSTZ1 | NM_001513.2 | 761 | gggcccagaacgccatcaacttgt | 216702 | - | - | | - |
| 16529 | LOC51064 | NM_015917.1 | 410 | atgcggctcggtcaaggaatga | 216712 | - | - | protein disulfide oxidoreductase | - |
| 16530 | MGST1 | NM_020300.3 | 135 | ttgcatcctatgcaacaattatt | 216718 | - | - | | - |
| 16531 | MGST2 | NM_002413.3 | 515 | atggccgtcacctatacttctgg | 216739 | - | signal_transduction | glutathione transferase | - |
| 16532 | MGST3 | NM_004528.2 | 152 | gtggcccacctagccatcaatgt | 216747 | - | signal_transduction | peroxidase | - |
| 16533 | CHM | NM_000390.1 | 1727 | atgatttaccatccaacgtttat | 216793 | - | see also 310 line | | - |
| 16534 | RABGGTA | NM_004581.2 | 1418 | cagttgccccaacatacatttcg | 216802 | - | | RAB-protein geranylgeranyltransferase | - |
| 16535 | PGGT1B | NM_005023.2 | 108 | ttgccggagcgctattcttcact | 216818 | - | | CAAX-protein geranylgeranyltransferase | - |
| 16536 | FNTA | NM_002027.1 | 358 | aagctaaccggggatgcttattga | 216860 | - | see also 1315 line | | - |
| 16537 | FNTB | NM_002028.2 | 849 | aagccggcagatgcgatttgaag | 216875 | - | | protein farnesyltransferase | - |
| 16538 | NS3TP1 | NM_019048.1 | 2284 | gttggacggctccaaatcatgtcc | 216947 | - | - | | - |

Figure 46

| | | | | | | | | | |
|---|---|---|---|---|---|---|---|---|---|
| 16539 | COX10 | NM_001303.2 | 1422 | gagctcgcggagactgttcttct | 216969 | - | - | farnesyltranstransferase | - |
| 16540 | GGPS1 | NM_004837.2 | 910 | aagcacccaggtgcagaatatct | 216979 | - | - | geranyltranstransferase | - |
| 16541 | FDPS | NM_002004.1 | 467 | atgccattggaggcaagtataac | 216991 | - | - | zinc binding | - |
| 16542 | CL640 | NM_015697.3 | 416 | tggagcaggctgtactattaatg | 217008 | - | - | - | - |
| 16543 | RABGGTB | NM_004582.2 | 106 | cagatgcaccggacactttgtta | 217035 | - | - | prenyltransferase | retinal degeneration |
| 16544 | SMS | NM_004595.2 | 838 | gagactgctatcaggttctaata | 217079 | - | - | spermine synthase | - |
| 16545 | HMBS | NM_000190.2 | 1009 | cggctcagatagcatacaagaga | 217098 | - | - | specific RNA polymerase II transcription factor | porphyria、diabetes |
| 16546 | DHPS | NM_001930.2 | 788 | gtgtttagtcccgcacttacaga | 217106 | - | - | - | - |
| 16547 | AGPS | NM_003659.1 | 1711 | agggtgacgcagacttacgatgc | 217166 | - | see also 2478 line | | |
| 16548 | HS3ST2 | NM_006043.1 | 629 | aggctcctcgacgcatcttcaac | 217186 | - | - | - | - |
| 16549 | HS3ST3A1 | NM_006042.1 | 855 | cagcatcttccggaagttcttgc | 217204 | - | - | - | - |
| 16550 | NDST2 | NM_003635.2 | 3053 | aggccggaggtatccagatatgg | 217245 | - | see also 2453 line | | |
| 16551 | NDST3 | NM_004784.1 | 1678 | gggcgtctaccctgtacatgttc | 217263 | - | see also 3295 line | | |
| 16552 | CHST8 | NM_022467.3 | 1769 | ctgcctcatcgactacgatttcg | 217299 | - | see also 8390 line | | |
| 16553 | CHST9 | NM_031422.1 | 523 | gaggggatcaagctttaagtaag | 217316 | - | - | - | - |
| 16554 | TPST1 | NM_003596.2 | 977 | gtgctatagagaccatgtataac | 217364 | - | see also 2414 line | | |
| 16555 | TPST2 | NM_003595.2 | 382 | gggcaccaaccacgtggaatacc | 217379 | - | - | protein-tyrosine sulfotransferase | - |
| 16556 | CHST3 | NM_004273.2 | 1139 | ctgtacgcccttcgtcaagaagg | 217389 | - | see also 2867 line | | |
| 16557 | CHST7 | NM_019886.2 | 1380 | atgccttcgcgctcaacatgact | 217401 | - | see also 7744 line | | |
| 16558 | SULT2A1 | NM_003167.2 | 261 | gggatgccaagtggatccaatct | 217409 | - | - | alcohol sulfotransferase | - |
| 16559 | CHST6 | NM_021615.2 | 1289 | gcgcaacctcgcccttgatctgg | 217425 | - | - | - | Alzheimer disease |
| 16560 | CHST1 | NM_003654.2 | 1327 | tggtggaagacccgcgattaaac | 217428 | - | - | - | - |
| 16561 | CHST10 | NM_004854.2 | 1268 | cgggcattaccgtgtataacaga | 217449 | - | see also 3374 line | | |
| 16562 | CHST2 | NM_004267.2 | 1137 | aagtaccgcacactagtcataaa | 217459 | - | - | - | - |
| 16563 | CHST5 | NM_012126.1 | 624 | gcgacctgatgcgctctatcttt | 217463 | - | - | - | - |
| 16564 | GALNAC4S-6ST | NM_014863.1 | 1499 | ccgctatcccgtggaagattatc | 217492 | - | see also 6063 line | | |
| 16565 | GP3ST | NM_022134.1 | 969 | ctggcgcctgtacgagcatttca | 217512 | - | - | - | - |
| 16566 | HS3ST5 | NM_153612.1 | 428 | ctgcttgcggtttaatattatct | 217536 | - | - | - | - |
| 16567 | NCAG1 | NM_032160.1 | 5107 | ctgatggatcgcctaggatatcc | 217670 | - | - | - | - |
| 16568 | NDST4 | NM_022569.1 | 3037 | gggagttacacctcgttataatt | 217737 | - | see also 8422 line | | |

Figure 46

| | | | | | See also | Function | Disease |
|---|---|---|---|---|---|---|---|
| 16569 | SULT1B1 | NM_014465.2 | 672 | atgatttatctggtcgtaatgc | | | |
| 16570 | SULT1C1 | NM_001056.2 | 839 | tagctcgaaatgccaaagactgt | see also 648 line | | |
| 16571 | SULT2B1 | NM_004605.2 | 310 | gggacgacgacatctttatcatc | | | |
| 16572 | SULT4A1 | NM_014351.2 | 471 | gagactccaagtcatctatatg | | | |
| 16573 | UST | NM_005715.1 | 675 | ccgtcaaccggttcttatccaac | see also 3968 line | | |
| 16574 | MPST | NM_021126.3 | 730 | gagccccgagacggcattgaacc | | thiosulfate sulfurtransferase | |
| 16575 | TST | NM_003312.4 | 429 | tggccaccgcaccgtatcagtgc | | structural constituent of cytoskeleton | |
| 16576 | OXCT | NM_000436.1 | 1271 | tggacacgtcgatctgacaatgc | | 3-oxoacid CoA-transferase | ketoacidosis |
| 16577 | AGXT | NM_000030.1 | 1096 | ctgtaccgctggctatgactgg | | serine-pyruvate aminotransferase | hyperoxaluria |
| 16578 | AGXT2 | NM_031900.1 | 546 | agggcgcactcaaacaacatga | | alanine-glyoxylate aminotransferase | |
| 16579 | AGXT2L1 | NM_031279.2 | 597 | gagattagcccatataagtttca | | | |
| 16580 | BCAT1 | NM_005504.3 | 1396 | ctgtacaaaggcgagacaataca | see also 3846 line | | |
| 16581 | BCAT2 | NM_001190.1 | 1039 | gtgcaccgaatcctgtacaaaga | | branched-chain amino acid aminotransferase | hyperleucine-isoleucinemia |
| 16582 | GOT1 | NM_002079.1 | 219 | tagcctaaatcacgtagtatctgc | see also 1346 line | | |
| 16583 | GOT2 | NM_002080.1 | 199 | ttggtgcctacgggatgataat | | aspartate aminotransferase | |
| 16584 | TAT | NM_000353.1 | 397 | ctggactcgggcaaatataatgg | | tyrosine aminotransferase | keratosis palmoplantaris, tyrosinosis |
| 16585 | PSAT1 | NM_021154.3 | 502 | atgcctcctacgtgtatattgc | | phosphoserine transaminase | |
| 16586 | OAT | NM_000274.1 | 1233 | gtgtctacgacttcgagataatg | | ornithine-oxo-acid aminotransferase | gyrate atrophy of choroid and retina |
| 16587 | ABAT | NM_000663.2 | 675 | gagctgacgtgcatgattaa | see also 425 line | | |
| 16588 | CCBL1 | NM_004059.2 | 378 | ctgctacgagcccatgacaatga | see also 2735 line | | |
| 16589 | AADAT | NM_016228.3 | 452 | ctggttgcttaccaaatccaaac | | | |
| 16590 | GPT2 | NM_133443.1 | 651 | gagctagtgacggcgatttctacg | see also 9578 line | | |
| 16591 | MGC15875 | NM_032921.1 | 977 | aggctgcctacttggtatcaagg | | | |
| 16592 | MGC33309 | NM_152413.1 | 264 | aagactcgactacagatttcaca | | | |
| 16593 | PHACS | NM_032592.1 | 544 | tgggtgatcctgccatgatctcc | see also 9257 line | | |
| 16594 | SCLY | NM_016510.3 | 760 | gggcgtggacttccttacaatcg | | | |

Figure 46

| | | | | | | | | | | |
|---|---|---|---|---|---|---|---|---|---|---|
| 16595 | TALDO1 | NM_006755.1 | 616 | ttgttgggcgcatccttgattgg | 218326 | - | - | | transaldolase | - |
| 16596 | ACAT2 | NM_005891.1 | 895 | gggtgtggagccttccattatgg | 218345 | - | - | | - | - |
| 16597 | ALG2 | NM_033087.1 | 150 | gcgggtgtagcgtgaagatctgg | 218353 | - | - | | - | - |
| 16598 | C20orf155 | NM_019095.3 | 603 | atgatcatttcgagagatgtaat | 218396 | - | see also 7703 line | | | |
| 16599 | CARKL | NM_013276.1 | 628 | cggtaccatccacgactatgtgg | 218411 | - | kinase_related | transferase | | - |
| 16600 | CAS1 | NM_022900.3 | 2012 | gaggttagaccgttatgtagttt | 218483 | - | see also 8521 line | | | |
| 16601 | CDSN | NM_001264.2 | 193 | gtgactccagcggcttcagtagc | 218513 | - | - | | molecular_function unknown | - |
| 16602 | ChGn | NM_018371.3 | 2005 | gtgcacctttatcgcaagtatct | 218559 | - | see also 7426 line | | | |
| 16603 | CHST12 | NM_018641.2 | 1305 | ctggaggcagcagctgtataaac | 218573 | - | see also 7534 line | | | |
| 16604 | D4ST-1 | NM_130468.2 | 1193 | cagccccgaaagcctgcattacc | 218582 | - | - | | - | - |
| 16605 | DHDDS | NM_024887.1 | 643 | ttgataagtgcctctataccaac | 218594 | - | see also 8801 line | | | |
| 16606 | FLJ12171 | NM_024619.2 | 131 | aggacgagtgttcgtgaaagtga | 218604 | - | kinase_related | transferase | | - |
| 16607 | FN3K | NM_022158.2 | 110 | aggcccagtgttcgtcaaagtca | 218612 | - | see also 8317 line | | | |
| 16608 | GAL3ST-4 | NM_024637.3 | 1435 | gagaggccctagcgaaacattgt | 218630 | - | - | | - | - |
| 16609 | GAL3ST2 | NM_033036.1 | 363 | cagcaccgtaagccttctcatcc | 218635 | - | - | | - | - |
| 16610 | GALGT2 | NM_153446.1 | 245 | ccgtcagggctcgagatttctgt | 218640 | - | - | | - | - |
| 16611 | GALNACT-2 | NM_018590.3 | 982 | atgacttcgtagaaggttattat | 218693 | - | see also 7528 line | | | |
| 16612 | GALNT12 | NM_024642.2 | 290 | gcgtgcggctgcaccagattaac | 218710 | - | see also 8675 line | | | |
| 16613 | GALNT14 | NM_024572.1 | 461 | aggaccatccgcagtgtattaaa | 218744 | - | - | | - | - |
| 16614 | HS2ST1 | NM_012262.2 | 714 | aagatcaggtgcgctttgtaaag | 218774 | - | see also 5242 line | | | |
| 16615 | HS6ST2 | NM_147174.2 | 421 | ccgccacttggtgcgtaacatcc | 218800 | - | see also 9811 line | | | |
| 16616 | HS6ST3 | NM_153456.1 | 980 | tggtgggctgctataacttgact | 218824 | - | see also 9995 line | | | |
| 16617 | KIAA0290 | NM_015122.1 | 2026 | tggaacggcccagcttcttatcc | 218845 | - | see also 6256 line | | | |
| 16618 | MAT2B | NM_013283.3 | 364 | cagccccatgttatagtacattg | 218849 | - | - | | - | - |
| 16619 | MMAB | NM_052845.1 | 432 | cgggaggctcacttaaagtatac | 218877 | - | - | | - | - |
| 16620 | MTRR | NM_002454.1 | 193 | tagtgaatccgataagtatgacc | 218883 | - | - | | - | homocystinuria |
| 16621 | PIGM | NM_145167.1 | 645 | ccgcggtaatgcggactctattg | 218961 | - | - | | - | - |
| 16622 | PNR | NM_003967.1 | 479 | gggtgcccgcagcatacacttcg | 219001 | - | receptor_acitivity、gpc r、signal_transduction | - | | - |
| 16623 | PTDSS1 | NM_014754.1 | 916 | tggacctatgttcgatggtttga | 219021 | - | see also 5944 line | | | |
| 16624 | TBDN100 | NM_025085.2 | 481 | ttggttcgtagaggtttgagaaa | 219044 | - | see also 8853 line | | | |
| 16625 | TPT | NM_014317.2 | 1136 | cagagctcgacagtatgtactac | 219145 | - | - | | - | - |
| 16626 | WBSCR17 | NM_022479.1 | 256 | gggctcattgaaggttatggtgg | 219151 | - | - | | - | - |
| 16627 | AKAP28 | NM_178813.3 | 117 | aagcaatggatgaggataacaaa | 219169 | - | - | | - | - |

Figure 46

| | | | | | | | | |
|---|---|---|---|---|---|---|---|---|
| 16628 | ALS2CR8 | NM_024744.12 | 1034 | caggtacttgtgtaattccacc | 219196 | - | see also 8723 line | |
| 16629 | C9orf12 | NM_022755.3 | 505 | gaggtcgttcagctacctttaga | 219243 | - | kinase_related | |
| 16630 | CDKN1B | NM_004064.2 | 929 | ctgcaaccgacgattcttctact | 219267 | - | see also 2742 line | |
| 16631 | CINP | NM_032630.2 | 144 | gcggctgattggcacaatttaat | 219279 | - | - | |
| 16632 | DKFZP434N1923 | NM_030974.2 | 1400 | ggggaacttggacgcttgtttcc | 219288 | - | kinase_related | |
| 16633 | DVL3 | NM_004423.2 | 850 | gagcatgcaaccggctaaatgg | 219295 | - | kinase_related, signal transduction | signal transducer |
| 16634 | FLJ12476 | NM_022784.1 | 2062 | ccggccttaacctcgcatatagt | 219351 | - | kinase_related | |
| 16635 | GKAP42 | NM_025211.2 | 402 | atggcctcagcagtacttagttc | 219358 | - | kinase_related, signal transduction | |
| 16636 | HINT3 | NM_138571.3 | 642 | gtggatcagcttggcttcttatc | 219394 | - | - | |
| 16637 | IKBKAP | NM_003640.2 | 3701 | cagtcgccacaagaaaacgtttat | 219479 | - | see also 2458 line | |
| 16638 | ITPK1 | NM_014216.2 | 1038 | agggcagcagcgccgtcattgaca | 219500 | - | see also 5540 line | |
| 16639 | LOC147463 | NM_173505.1 | 987 | atgaacttccggcagaactaacc | 219513 | - | - | |
| 16640 | LOC55971 | NM_018842.3 | 663 | aagccgaaacgcactcaaatatg | 219531 | - | - | |
| 16641 | MADD | NM_003682.2 | 212 | ctgtcctcggttacttgactatc | 219557 | - | see also 2499 line | |
| 16642 | MAP3K7IP2 | NM_015093.2 | 1492 | aagtcgagcaataggcaattaact | 219695 | - | see also 6230 line | |
| 16643 | NYD-SP25 | NM_033516.2 | 383 | gagacgctgtggggaactcaaga | 219722 | - | | |
| 16644 | P101-PI3K | NM_014308.1 | 1915 | ctggtatgagcgcaatgtactgg | 219730 | - | | |
| 16645 | P15RS | NM_018170.2 | 651 | tggtagaggatgcgtgtatgttg | 219749 | - | kinase_related | |
| 16646 | SKP2 | NM_005983.2 | 739 | tgaactatcgaactcagttata | 219765 | - | see also 4182 line | |
| 16647 | STAP2 | NM_017720.1 | 745 | tggacgccgtgtcaactattc | 219811 | - | kinase_related | |
| 16648 | TSKS | NM_021733.1 | 564 | ttggaggggtactgcattcaact | 219818 | - | | |
| 16649 | DHRS2 | NM_005794.2 | 1012 | ctgctgggtctcactagaacact | 219829 | - | | |
| 16650 | HSD17B2 | NM_002153.1 | 217 | cagtactatgtgggacagtattt | 219839 | - | | estradiol 17 beta-dehydrogenase |
| 16651 | HSD17B3 | NM_000197.1 | 110 | tggccgaagtgcgtgagattctcc | 219866 | - | | estradiol 17 beta-dehydrogenase |
| 16652 | HSD17B7 | NM_016371.1 | 679 | caggtacagcattgaccaatttg | 219890 | - | | estradiol 17 beta-dehydrogenase |
| 16653 | HSD17B8 | NM_014234.3 | 465 | ttgtcgtgttccatcatcaaca | 219898 | - | see also 5551 line | |
| 16654 | H105E3 | NM_015922.1 | 229 | aagtcgcacggactcatttgaca | 219901 | - | see also 6572 line | |
| 16655 | HSD3B1 | NM_000862.1 | 679 | gtgccttacgaccatgtatatc | 219918 | - | | steroid delta-isomerase |

Figure 46

| | | | | | | | | | |
|---|---|---|---|---|---|---|---|---|---|
| 16656 | HSD3B2 | NM_000198.1 | 691 | gtgcgttaagaccaccatatatc | 219921 | - | | transcription co-activator | adrenal hyperplasia |
| 16657 | HSD3B7 | NM_025193.2 | 678 | cggtgaaggccaccagatcatga | 219927 | - | - | - | - |
| 16658 | HSPC105 | NM_145168.1 | 599 | gaggcgacggtgttaagaacc | 219946 | - | - | - | - |
| 16659 | HSD11B1 | NM_005525.2 | 430 | aggactagacatgtcattctca | 219964 | - | see also 3860 line | - | - |
| 16660 | HADH2 | NM_004493.1 | 459 | aggccaacgtggggtcatcatca | 219995 | - | | 7-alpha-hydroxysteroid dehydrogenase | Alzheimer disease |
| 16661 | LDHA | NM_005566.1 | 146 | cagacccccagaataagattac | 219998 | - | | L-lactate dehydrogenase | exertional myoglobinuria |
| 16662 | LDHB | NM_002300.3 | 411 | aggggagagtcggctcaatctgg | 220016 | - | | L-lactate dehydrogenase | - |
| 16663 | LDHC | NM_002301.2 | 267 | ttgtgctattagtatcttactga | 220041 | - | - | - | - |
| 16664 | LDHL | NM_033195.1 | 144 | tcggtgggagcgaattcctatg | 220061 | - | | L-lactate dehydrogenase | - |
| 16665 | MGC23940 | NM_144972.1 | 309 | aggaggcattcatcacaataag | 220067 | - | | - | - |
| 16666 | UEV3 | NM_018314.2 | 233 | gtgatgtatcagggtaatacata | 220075 | - | | - | - |
| 16667 | EHHADH | NM_001966.1 | 1314 | cagctcatgtcatgaagttgtta | 220131 | - | | enoyl-CoA hydratase | - |
| 16668 | HADHSC | NM_005327.1 | 579 | ccgattccgtcggcctccatttct | 220174 | - | | 3-hydroxyacyl-CoA dehydrogenase | SCHAD deficiency |
| 16669 | IDH3A | NM_005530.2 | 338 | cagccggtcacccatctatgaat | 220187 | - | | isocitrate dehydrogenase (NAD+) | - |
| 16670 | IDH1 | NM_005896.2 | 344 | tggatctacatagctatgattta | 220240 | - | see also 4099 line | | - |
| 16671 | IDH2 | NM_002168.2 | 250 | gtgatgagatgacccgtattatc | 220275 | - | see also 1390 line | | - |
| 16672 | RDH5 | NM_002905.1 | 881 | ctgtgaccegacctaaccaagg | 220300 | - | | retinol dehydrogenase | - |
| 16673 | RODH-4 | NM_003708.2 | 1558 | gaggcctatggcgagaagtttgt | 220319 | - | | - | - |
| 16674 | AKR1A1 | NM_006066.2 | 959 | cagtcggcagattgatgacatac | 220329 | - | | - | - |
| 16675 | AKR1B1 | NM_001628.2 | 983 | ctgtacctcccaacaaggattacc | 220344 | - | | electron transporter | diabetes mellitus, diabetes |
| 16676 | HIBADH | NM_152740.1 | 848 | gtgttggtcaagtgacacttata | 220376 | - | | phosphogluconate dehydrogenase (decarboxylating) | |
| 16677 | IMPDH1 | NM_000883.2 | 1403 | ccgggcgtcgacgtcatagtcttg | 220400 | - | see also 574 line | | - |
| 16678 | IMPDH2 | NM_000884.1 | 48 | atggccgactacctgattagtgg | 220412 | - | | IMP dehydrogenase | - |

Figure 46

| | | | | | | | | | |
|---|---|---|---|---|---|---|---|---|---|
| 16679 | ME1 | NM_002395.2 | 1133 | gggacgtgcttccttaacacaag | 220463 | - | - | malate dehydrogenase (oxaloacetate decarboxylating) (NADP+) | - |
| 16680 | ME3 | NM_006680.1 | 1690 | tcgactacgcgtacaaacacaac | 220502 | - | - | malate dehydrogenase (oxaloacetate decarboxylating) (NADP+) | - |
| 16681 | GBA2 | NM_020944.2 | 2331 | tggctccgcgtcaatgcatattt | 220541 | - | see also 8043 line | | |
| 16682 | HMGCR | NM_000859.1 | 1963 | tagctggacgcaacctttatatc | 220626 | - | - | hydroxymethylglutaryl-CoA reductase (NADPH) | - |
| 16683 | GPD1 | NM_005276.2 | 421 | aagctcatctcggaagtgattgg | 220655 | - | - | glycerol-3-phosphate dehydrogenase (NAD+) | - |
| 16684 | KIAA0089 | NM_015141.2 | 284 | tggttgccatgtcaaatcttagc | 220663 | - | - | - | - |
| 16685 | G6PD | NM_000402.2 | 1905 | aagcccatccctatatttatgg | 220681 | - | - | glucose-6-phosphate 1-dehydrogenase | - |
| 16686 | CBR1 | NM_001757.1 | 877 | gagggtccccatggacaatttgt | 220687 | - | - | carbonyl reductase (NADPH) | - |
| 16687 | CBR3 | NM_001236.2 | 390 | acgagttactgccgataatgaaa | 220694 | - | - | carbonyl reductase (NADPH) | - |
| 16688 | HPGD | NM_000860.2 | 404 | aaggcggcatcattatcaatatg | 220708 | - | - | prostaglandin-D synthase | - |
| 16689 | MDH1 | NM_005917.2 | 958 | aaggtctccctattaatgatttc | 220753 | - | see also 4138 line | | |
| 16690 | MDH2 | NM_005918.2 | 505 | ttgccaatccggttaattccacc | 220768 | - | - | L-malate dehydrogenase | - |
| 16691 | SPR | NM_003124.2 | 539 | aggaaaggctgctcgtgatatgc | 220777 | - | - | sepiapterin reductase | sepiapterin reductase deficiency |
| 16692 | PHGDH | NM_006623.2 | 1188 | aagggaccatccaggtgataaca | 220784 | - | - | phosphoglycerate dehydrogenase | 3-phosphoglycerate dehydrogenase deficiency |
| 16693 | UGDH | NM_003359.1 | 492 | aagtatccgtcgcatatttgatg | 220802 | - | see also 2267 line | | |
| 16694 | BDH | NM_004051.2 | 368 | ccgattggccgtcggacttatgc | 220842 | - | see also 2731 line | | |
| 16695 | ME2 | NM_002396.2 | 93 | ttgtcccggttaagagtagtttc | 220856 | - | see also 1541 line | | |
| 16696 | AKR1C4 | NM_001818.2 | 690 | gggaacccaacgacataaactat | 220906 | - | - | electron transporter | - |
| 16697 | AKR1C3 | NM_003739.4 | 652 | ccgtatttcaaccggagtaaatt | 220913 | - | - | electron transporter | - |
| 16698 | GRHPR | NM_012203.1 | 44 | gagaccggtgcgactcatgaagg | 220920 | - | see also 5197 line | | |
| 16699 | UBE1DC1 | NM_024818.2 | 449 | ccgtacctttgccgtagcaatag | 220946 | - | see also 8764 line | | |

Figure 46

| | | | | | | | | | |
|---|---|---|---|---|---|---|---|---|---|
| 16700 | HAO1 | NM_017545.2 | 530 | atgatgtgcgtaacagattcaaa | 220995 | - | see also 7024 line | | |
| 16701 | HAO2 | NM_016527.1 | 1078 | ccggtcggtcgctgagatcaatc | 221022 | - | - | glycolate oxidase | - |
| 16702 | CYP11B2 | NM_000498.2 | 889 | aaggcggaactgtcactagaagc | 221024 | - | - | transcription co-activator | hyperaldosteronism、hypoaldosteronism、low renin hypertension |
| 16703 | CYP39A1 | NM_016593.3 | 1578 | agggcaatgccgaattgaatata | 221061 | - | see also 6936 line | | |
| 16704 | CYP7B1 | NM_004820.2 | 395 | aagaccccttaaggttcatgaaa | 221071 | - | - | human cytochromes P450 family | giant cell hepatitis |
| 16705 | CYP21A2 | NM_000500.4 | 659 | aagatcaaggacgacaacttaat | 221111 | - | - | Smad3/Sp-1 heterodimer | adrenal hyperplasia |
| 16706 | CYP17A1 | NM_000102.2 | 1297 | atgcctgagcgtttcttgaatcc | 221139 | - | - | transcription co-activator | adrenal hyperplasia、breast cancer、prostate cancer |
| 16707 | CYP8B1 | NM_004391.1 | 1621 | ctggggttcgggcgtttccatct | 221160 | - | see also 2952 line | | |
| 16708 | CYP11A1 | NM_000781.1 | 173 | cagtcctcgccccttcaatgaga | 221164 | - | - | human cytochromes P450 family | adrenal hyperplasia、Alzheimer disease |
| 16709 | CYP7A1 | NM_000780.2 | 1374 | ttggatcgggagctacaatatgt | 221220 | - | see also 481 line | | |
| 16710 | CH25H | NM_003956.2 | 522 | tcgcgctggcaacgcagtatatg | 221225 | - | - | - | - |
| 16711 | CYP27A1 | NM_000784.2 | 704 | cagcggaagcagcgctctatacg | 221233 | - | - | human cytochromes P450 family | atherosclerosis、cerebrotendinous xanthomatosis |
| 16712 | CYP46A1 | NM_006668.1 | 1195 | gggcggatggacacatactttga | 221259 | - | - | human cytochromes P450 family | Alzheimer disease |
| 16713 | FMO1 | NM_002021.1 | 753 | tgggtgatcagccgaatctttga | 221275 | - | - | disulfide oxidoreductase | - |
| 16714 | FMO3 | NM_006894.3 | 746 | ctgctcgtcactcgatttggaac | 221315 | - | see also 4825 line | | |
| 16715 | FMO4 | NM_002022.1 | 1859 | atgtccccttacctagtaagtct | 221390 | - | - | dimethylaniline monooxygenase (N-oxide forming) | - |
| 16716 | FMO5 | NM_001461.1 | 922 | cagcatccaaccttaaatgatga | 221412 | - | see also 1015 line | | |
| 16717 | CYP4F8 | NM_007253.2 | 324 | tcgtccgatctgtcatcaatacc | 221442 | - | - | human cytochromes P450 family | - |
| 16718 | SQLE | NM_003129.2 | 1874 | ttgtacaaagcgtgttctgtaat | 221490 | - | - | squalene monooxygenase | - |
| 16719 | TYR | NM_000372.2 | 1330 | tagccgattggaggagtacaaca | 221509 | - | - | monophenol monooxygenase | oculocutaneous albinism |
| 16720 | KMO | NM_003679.1 | 621 | atgggtacatggagttgactatt | 221539 | - | - | - | - |
| 16721 | CYP27B1 | NM_000785.2 | 1279 | ctggtcactctgtgtcactatgc | 221565 | - | - | human cytochromes P450 family | pseudovitamin D deficiency rickets |
| 16722 | CYP26A1 | NM_000783.2 | 460 | ccgcgaggcactcgaatgctacg | 221573 | - | see also 484 line | | |
| 16723 | CYP2J2 | NM_000775.2 | 342 | gtgacccctatgcgagaacatat | 221597 | - | - | human cytochromes P450 family | - |

Figure 46

| | | | | | | | | | |
|---|---|---|---|---|---|---|---|---|---|
| 16724 | COQ6 | NM_182476.1 | 497 | gggctatatcgtggagaatgatg | 221624 | - | - | - | - |
| 16725 | CYP19A1 | NM_000103.2 | 510 | gtgcatcggtatgcatgagaaag | 221658 | - | see also 69 line | | |
| 16726 | CYP1A1 | NM_000499.2 | 1432 | gtgctatcgacaaggtgttaagt | 221691 | - | - | CYP1A1.4 | oral cavity cancers、breast cancer、lung cancer |
| 16727 | CYP1A2 | NM_000761.2 | 613 | tgggcacttcgacccttacaatc | 221696 | - | - | CYP1A2 E225I | lung cancer |
| 16728 | CYP1B1 | NM_000104.2 | 1887 | cagttatggtctaaccattaaac | 221711 | - | - | CYP1B1.7 | breast cancer、glaucoma、Peters anomaly |
| 16729 | CYP20A1 | NM_020674.2 | 595 | caggcgcctcgtggttagtttgg | 221717 | - | - | - | - |
| 16730 | CYP24A1 | NM_000782.2 | 1832 | ttggattgtccgcaaatacgaca | 221787 | - | see also 483 line | | |
| 16731 | CYP2B6 | NM_000767.4 | 331 | ccggggatatggtgtgatctttg | 221791 | - | - | human cytochromes P450 family | lung cancer |
| 16732 | CYP2C18 | NM_000772.1 | 973 | cagtgctcgggactttattgatt | 221800 | - | - | human cytochromes P450 family | - |
| 16733 | CYP2C8 | NM_000770.2 | 314 | aggcaattccccaatatctcaaa | 221807 | - | - | - | - |
| 16734 | CYP2C9 | NM_000771.2 | 823 | aagcacaaccaaccatctgaatt | 221825 | - | - | CYP2C9-Arg | Warfarin sensitivity、diabetes mellitus、mephenytoin 4-hydroxylase C9 defect、diabetes |
| 16735 | CYP2E1 | NM_000773.2 | 423 | ctgaccaccctccggaactatgg | 221831 | - | - | human cytochromes P450 family | non-hodgkin lymphoma、excessive alcohol consumption、lung cancer、alcoholism |
| 16736 | CYP2F1 | NM_000774.3 | 686 | cagccctggggcgagttgtacg | 221856 | - | - | human cytochromes P450 family | - |
| 16737 | CYP2S1 | NM_030622.5 | 648 | gggtcagacctacgagatgttct | 221858 | - | - | - | - |
| 16738 | CYP3A43 | NM_022820.3 | 178 | tgggacccattcacataaacttt | 221865 | - | - | - | - |
| 16739 | CYP3A5 | NM_000777.2 | 971 | tcgcagcccagtcaataatcttc | 221880 | - | - | human cytochromes P450 family | |
| 16740 | CYP4B1 | NM_000779.2 | 1419 | gtgcttgctccgctttgagttct | 221897 | - | - | human cytochromes P450 family | - |
| 16741 | CYP4F11 | NM_021187.1 | 452 | ctgctgtcgcacccaaggatatg | 221898 | - | - | human cytochromes P450 family | - |
| 16742 | CYP4F2 | NM_001082.3 | 601 | agggtagtgcctgtttggatatg | 221906 | - | - | human cytochromes P450 family | - |
| 16743 | CYP4F3 | NM_000896.1 | 259 | cggaggaaggtctcctatacaca | 221909 | - | - | human cytochromes P450 family | Alzheimer disease |
| 16744 | CYP4X1 | NM_178033.1 | 1364 | aaggagacgtgccgattgattcc | 221934 | - | - | - | - |
| 16745 | CYP51A1 | NM_000786.2 | 1830 | cagttatccgttacaaacgaaga | 221953 | - | - | human cytochromes P450 family | - |
| 16746 | FLJ20359 | NM_017781.1 | 94 | ggggttcgaggcggtcaaagagg | 221956 | - | - | - | - |
| 16747 | FLJ39501 | NM_173483.1 | 745 | cagcggtctcccttgatatgttt | 221970 | - | - | - | - |
| 16748 | LOC116123 | NM_138784.1 | 746 | tagtgatcggcattggaaattct | 221986 | - | - | - | - |

Figure 46

| 16749 | MICAL2 | NM_014632.1 | 326 | aggcatccacgtgcaaaggtacc | 221991 | - | see also 5799 line | | |
|---|---|---|---|---|---|---|---|---|---|
| 16750 | P450RAI-2 | NM_019885.2 | 220 | tcgtcgcggagggagaagtatgg | 222032 | - | - | - | - |
| 16751 | PAMCI | NM_005447.2 | 298 | gaggctacgtttggagagaaacg | 222039 | - | signal_transduction | - | - |
| 16752 | TBXAS1 | NM_001061.2 | 484 | tggtcgtcggatgtttattgtta | 222066 | - | see also 651 line | | |
| 16753 | TYRP1 | NM_000550.1 | 1517 | tggccaagtcgggagtttagtgt | 222129 | - | - | monooxygenase | albinism、xanthism |
| 16754 | PLOD | NM_000302.2 | 2194 | tggacgactcacgcattaccatg | 222150 | - | - | procollagen-lysine 5-dioxygenase | Ehlers-Danlos syndrome |
| 16755 | PLOD2 | NM_000935.1 | 878 | atgtccatccaaacgtatcaata | 222187 | - | see also 615 line | | |
| 16756 | PLOD3 | NM_001084.3 | 849 | tcgtgcgccagtggaagtacaag | 222234 | - | - | procollagen-lysine 5-dioxygenase | |
| 16757 | P4HA1 | NM_000917.1 | 302 | aagttagatcggctaactagtac | 222255 | - | see also 593 line | | |
| 16758 | P4HA2 | NM_004199.1 | 1528 | ggggaatcgtgtggctactttct | 222326 | - | - | procollagen-proline、2-oxoglutarate-4-dioxygenase | breast cancer |
| 16759 | P4HB | NM_000918.2 | 1397 | caggacggtcattgattacaacg | 222336 | - | - | protein disulfide isomerase | - |
| 16760 | LOC57168 | NM_020437.3 | 1147 | cggacctgtattgggaacaatgt | 222345 | - | - | - | - |
| 16761 | BBOX1 | NM_003986.1 | 1183 | atgtcaaggcttcgtatcttaag | 222388 | - | see also 2723 line | | |
| 16762 | EGLN1 | NM_022051.1 | 3902 | aggacatccgaggcgataagatc | 222389 | - | - | - | atherosclerosis |
| 16763 | EGLN3 | NM_022073.2 | 815 | gggatcctgcggatatttccaga | 222400 | - | apoptosis | - | - |
| 16764 | FLJ10826 | NM_018233.1 | 1189 | aagaccggtcactacactttaat | 222416 | - | - | - | - |
| 16765 | FLJ22222 | NM_024648.1 | 337 | aggtcgtggcagggagattcatc | 222423 | - | - | - | - |
| 16766 | LEPRE1 | NM_022356.2 | 1458 | gtgatggacggcgtaatctctga | 222450 | - | see also 8344 line | | |
| 16767 | LEPREL2 | NM_014262.2 | 780 | gagggcctattaccagttgaaga | 222466 | - | - | - | - |
| 16768 | MLAT4 | NM_018192.2 | 1808 | aggctcgaaggattgtagaatct | 222507 | - | see also 7311 line | | |
| 16769 | PTGS1 | NM_000962.2 | 567 | gagctattacactcgtattctgc | 222527 | - | - | - | - |
| 16770 | PTGS2 | NM_000963.1 | 1022 | cagagtatgcgatgtgcttaaac | 222571 | - | - | Rel A homodimer | lupus erythematosus、colorectal cancer、esophageal cancer、head and neck cancer、colorectal cancers、gastric cancer、skin cancer |
| 16771 | HMOX1 | NM_002133.1 | 406 | cagccatgcagcgctatgtgaag | 222595 | - | kinase_related | USF43/USF44 complex | - |
| 16772 | HMOX2 | NM_002134.2 | 487 | gtggagcggatccactacatagg | 222608 | - | - | heme oxygenase (decyclizing) | - |
| 16773 | DAP13 | NM_018838.3 | 366 | ctgctcgtaaattcatttggacg | 222621 | - | - | NADH dehydrogenase (ubiquinone) | - |

Figure 46

| | | | | | | | | | |
|---|---|---|---|---|---|---|---|---|---|
| 16774 | GRIM19 | NM_015965.3 | 323 | gcgcggtcggatagttacactac | 222633 | - | apoptosis、transcription_regulator | NADH dehydrogenase (ubiquinone) | - |
| 16775 | NDUFA11 | NM_175614.1 | 303 | gggagcacgcacgcacaactacg | 222642 | - | - | - | - |
| 16776 | NDUFA2 | NM_002488.2 | 123 | gtgagattcgcatccacttatgt | 222644 | - | - | NADH dehydrogenase (ubiquinone) | - |
| 16777 | NDUFA6 | NM_002490.2 | 15 | gcgtccgccaagctacttctacc | 222652 | - | see also 1592 line | | |
| 16778 | NDUFA7 | NM_005001.1 | 168 | aagctctccaacaattactattg | 222662 | - | - | NADH dehydrogenase (ubiquinone) | - |
| 16779 | NDUFA8 | NM_014222.2 | 398 | tggacttgcattgattatactgg | 222667 | - | - | NADH dehydrogenase (ubiquinone) | - |
| 16780 | NDUFA9 | NM_005002.3 | 668 | atgcatcggtttggtcctatacc | 222689 | - | - | NADH dehydrogenase (ubiquinone) | - |
| 16781 | NDUFB1 | NM_004545.3 | 280 | ctgccttccggaacaagagtatg | 222703 | - | - | NADH dehydrogenase (ubiquinone) | - |
| 16782 | NDUFB10 | NM_004548.1 | 376 | aagtcgaccaagaaattatcaac | 222711 | - | - | NADH dehydrogenase (ubiquinone) | - |
| 16783 | NDUFB5 | NM_002492.2 | 391 | ttgcccgtaatttctatgatagt | 222723 | - | - | NADH dehydrogenase (ubiquinone) | - |
| 16784 | NDUFB6 | NM_002493.3 | 130 | ggggtacactccggatgagaaac | 222736 | - | - | NADH dehydrogenase (ubiquinone) | - |
| 16785 | NDUFB7 | NM_004146.4 | 341 | ctgcgagcaccgcgactatgtga | 222740 | - | - | NADH dehydrogenase (ubiquinone) | - |
| 16786 | NDUFB8 | NM_005004.1 | 273 | atgagagagatccatggtatagc | 222741 | - | - | NADH dehydrogenase (ubiquinone) | - |
| 16787 | NDUFB9 | NM_005005.1 | 213 | gagcccggtttgaagaacataag | 222745 | - | - | NADH dehydrogenase (ubiquinone) | - |

Figure 46

| | | | | | | | | | |
|---|---|---|---|---|---|---|---|---|---|
| 16788 | NDUFC1 | NM_002494.2 | 68 | cggcccttcagtcgcgatcaaagt | 222751 | - | - | NADH dehydrogenase (ubiquinone) | - |
| 16789 | NDUFC2 | NM_004549.3 | 310 | ttgcatgccagttctatatat | 222761 | - | - | NADH dehydrogenase (ubiquinone) | - |
| 16790 | NDUFS1 | NM_005006.5 | 1426 | cagtccagtggacctcacttaca | 222811 | - | see also 3499 line | | |
| 16791 | NDUFS2 | NM_004550.3 | 855 | ttgaaccggctagagactatgtgtcc | 222848 | - | | NADH dehydrogenase (ubiquinone) | - |
| 16792 | NDUFS3 | NM_004551.1 | 627 | tggctatgttgagttacgttatg | 222887 | - | | iron-sulfur electron transfer carrier | - |
| 16793 | NDUFS5 | NM_004552.1 | 199 | gtgcaatggaatcggttatact | 222899 | - | | NADH dehydrogenase (ubiquinone) | - |
| 16794 | NDUFS6 | NM_004553.2 | 141 | acgcacactgccaggtttatga | 222900 | - | | iron-sulfur electron transfer carrier | - |
| 16795 | NDUFS8 | NM_002496.1 | 506 | ccggaccaccgctatgacacatcg | 222914 | - | | NADH dehydrogenase (ubiquinone) | Leigh syndrome |
| 16796 | NDUFV1 | NM_007103.2 | 522 | ccgcgctgcctatatctacatcc | 222918 | - | see also 4951 line | | |
| 16797 | NDUFV2 | NM_021074.1 | 599 | aggatttgacagctaaggatatt | 222933 | - | | iron-sulfur electron transfer carrier | Parkinson disease |
| 16798 | NQO1 | NM_000903.1 | 328 | aagccgcagacacttgtgatatc | 222947 | - | | Transcription factor JUN-D | lung cancer |
| 16799 | NQO2 | NM_000904.1 | 622 | gggaaggcacgccgagaatgtaca | 222971 | - | | electron transporter | - |
| 16800 | CYB561 | NM_001915.2 | 651 | gggcaagtatagcgccatttgagc | 222976 | - | | electron transporter cytochrome b5 reductase | - |
| 16801 | CYB5R2 | NM_016229.1 | 383 | aggctttgtggacctaattataa | 222984 | - | | | - |
| 16802 | DIA1 | NM_000398.3 | 725 | aggaactcaggaacaaacattct | 223005 | - | | | methemoglobinemia |
| 16803 | NCB5OR | NM_016230.2 | 428 | aggttcatcgttggtcaattat | 223015 | - | | | - |
| 16804 | NNT | NM_012343.2 | 604 | gtgcggagccctatggttaatcc | 223062 | - | see also 5302 line | | |
| 16805 | DHDH | NM_014475.2 | 933 | gtgcctacgcaagggtatgaagg | 223159 | - | | | - |
| 16806 | POR | NM_000941.1 | 65 | aggcggttggccgaagaagtatct | 223161 | - | | flavodoxin | - |
| 16807 | TXNRD2 | NM_006440.2 | 1463 | aggtctatcacgccattataaa | 223183 | - | | | - |
| 16808 | PRDX3 | NM_006793.2 | 786 | aagagtactttcagaagtaaat | 223195 | - | | | - |
| 16809 | GMPR | NM_006877.2 | 313 | atgtcacagcactccatgtttac | 223201 | - | | GMP reductase | - |
| 16810 | GMPR2 | NM_016576.2 | 413 | gtgaggttggatctcacaagatcc | 223209 | - | see also 6920 line | | |
| 16811 | NOX1 | NM_007052.3 | 654 | aagggcgggttcttggctaaatcc | 223230 | - | see also 4939 line | | |

## Figure 46

| 16812 | AASS | NM_005763.2 | 1962 | gggagccacgattgaatcatata | 223348 | - | see also 4023 line | | | |
| 16813 | CAT | NM_001752.1 | 1369 | ctgccaatgatgataacgttact | 223414 | - | - | | peroxidase | acatalasemia、 bladder cancer |
| 16814 | EPX | NM_000502.1 | 321 | gcggtccggacccttcaatgtca | 223421 | - | - | | myeloperoxidase | hereditary eosinophil peroxidase deficiency(-) |
| 16815 | FLJ20473 | NM_017836.2 | 852 | gagcccacccatcgtgaagatcc | 223449 | - | - | | - | - |
| 16816 | FLJ25471 | NM_144651.1 | 548 | aaggccaccttccgttccataaa | 223468 | - | - | | - | - |
| 16817 | GPR156 | NM_153002.1 | 1609 | cggcggtcacgggttagttcagt | 223516 | - | receptor_acitivity | | - | - |
| 16818 | GPX2 | NM_002083.2 | 134 | tggatggggagaaggtagatttc | 223528 | - | - | | selenium binding | - |
| 16819 | GPX3 | NM_002084.2 | 521 | ctgacgggccagtacattgaact | 223535 | - | - | | selenium binding | - |
| 16820 | GPX4 | NM_002085.1 | 451 | acgtcaaattcgatatgttcagc | 223548 | - | - | | selenium binding | - |
| 16821 | GPX5 | NM_001509.1 | 130 | atgaggccatcgcacttaataag | 223549 | - | - | | - | - |
| 16822 | GPX6 | NM_015696.2 | 567 | gaggaagctcatcctactgaagc | 223566 | - | - | | - | - |
| 16823 | HCCA3 | NM_020232.3 | 297 | tggtggctctacagttaagatcc | 223573 | - | - | | - | - |
| 16824 | MMS19L | NM_022362.2 | 2594 | atgccgaagtgcggatcatgttc | 223626 | - | transcription_regulator、 signal_transduction | | - | |
| 16825 | PRDX1 | NM_002574.2 | 598 | atgggtcaatacacctaagaaac | 223647 | - | - | | - | - |
| 16826 | PRDX2 | NM_005809.4 | 208 | gtggttgatggcgccttcaaaga | 223653 | - | - | | thioredoxin peroxidase | - |
| 16827 | PRDX4 | NM_006406.1 | 344 | ctgaagttaactgattatcgtgg | 223671 | - | see also 4493 line | | | |
| 16828 | RTDR1 | NM_014433.2 | 455 | gggcagatacgcctttctagagc | 223693 | - | - | | - | - |
| 16829 | TPRA40 | NM_016372.1 | 1387 | gagcgctggaaggccatcaatgc | 223707 | - | receptor_acitivity、 gpcr | | | |
| 16830 | ACADL | NM_001608.1 | 772 | aggataccgcagaactattcttt | 223732 | - | - | | translation regulator | dicarboxylicaciduria、 hypoglycemia |
| 16831 | ACADVL | NM_000018.1 | 279 | cggccaaggcggaatctaagtcc | 223753 | | - | | long-chain acyl-CoA dehydrogenase | hypoketotic hypoglycemia |
| 16832 | GCDH | NM_000159.1 | 682 | tcgcctatggccgatctgtttgt | 223773 | - | - | | - | glutaricaciduria |
| 16833 | ACADS | NM_000017.1 | 296 | tggcggtgctggcctcgattacc | 223779 | - | - | | electron transfer flavoprotein | hereditary SCAD deficiency、 lipid storage myopathy |
| 16834 | IVD | NM_002225.2 | 1348 | gtgtcggcagtatgtctacaatg | 223801 | - | - | | isovaleryl-CoA dehydrogenase | isovalericacidemia |
| 16835 | ACAD8 | NM_014384.1 | 725 | aagggcatctcatgcatagttgt | 223812 | - | see also 5644 line | | | |
| 16836 | ACAD9 | NM_014049.3 | 1365 | atgagattctccggatgtacatc | 223844 | - | - | | - | - |
| 16837 | ACADM | NM_000016.2 | 1228 | ctggtcgtcgaaatacctattat | 223879 | - | - | | Smad3/Sp-1 heterodimer | dicarboxylicaciduria、 hypoglycemia、 Reye syndrome、 medium-chain acyl coenzyme A dehydrogenase deficiency (MCAD deficiency) |

Figure 46

| | | | | | | | | | |
|---|---|---|---|---|---|---|---|---|---|
| 16838 | ACADSB | NM_001609.2 | 1109 | aagcgtcaatggccaaatactat | 223919 | - | | electron transfer flavoprotein | - |
| 16839 | SRD5A1 | NM_001047.1 | 690 | gaggcttatttgaatacgtaact | 223941 | - | | electron transporter | pseudohermaphroditism |
| 16840 | SRD5A2 | NM_000348.2 | 381 | gggaggccttatccagctatact | 223948 | - | see also 290 line | | |
| 16841 | DHODH | NM_001361.1 | 313 | ccgtggacggactttataagatg | 223972 | - | see also 924 line | | |
| 16842 | DPYD | NM_000110.2 | 807 | ctgccgtatgatgtagtgaattt | 224006 | - | see also 93 line | | |
| 16843 | SDHC | NM_003001.2 | 141 | gagcggttctggaataagaatat | 224072 | - | | succinate dehydrogenase | paraganglioma |
| 16844 | ACOX1 | NM_004035.4 | 1714 | aagcctaaccgaagcatataaac | 224112 | - | | - | pseudoneonatal adrenoleukodystrophy |
| 16845 | ACOX2 | NM_003500.1 | 1845 | ttgactaactcgggtgactttct | 224152 | - | see also 2359 line | | |
| 16846 | ACOX3 | NM_003501.1 | 553 | aagccacggcagtaataccaagg | 224161 | - | | electron transfer flavoprotein | - |
| 16847 | CPO | NM_000097.3 | 781 | gggcgtgagctctgttatccacc | 224183 | - | | coproporphyrinogen oxidase | harderoporphyrinuria、coproporphyria |
| 16848 | FLJ11164 | NM_018346.1 | 500 | aggggttaacaggttgtctatag | 224204 | - | - | - | - |
| 16849 | PPOX | NM_000309.1 | 1109 | gaggctgaccacgttattagtgc | 224221 | | - | protoporphyrinogen oxidase | porphyria、diabetes |
| 16850 | BLVRA | NM_000712.3 | 657 | gagcgaaaggaagatcagtatat | 224233 | - | - | electron transporter | - |
| 16851 | BLVRB | NM_000713.1 | 176 | gcggtgcaagcaggttacgaagt | 224247 | - | - | biliverdin reductase | - |
| 16852 | DECR1 | NM_001359.1 | 287 | aagcgatgctaccacctaatagt | 224255 | - | see also 923 line | | |
| 16853 | SDHB | NM_003000.1 | 544 | aggatcttgttcccgatttgagc | 224297 | - | | succinate dehydrogenase (ubiquinone) | paraganglioma、phaeochromocytoma |
| 16854 | FADS1 | NM_013402.3 | 1351 | ccgacatcatccactcactaaag | 224311 | - | - | - | - |
| 16855 | SC5DL | NM_006918.2 | 263 | aagtccgtcgagagattaagttt | 224324 | - | - | C-5 sterol desaturase | - |
| 16856 | TDO2 | NM_005651.1 | 1092 | gtggttcctcaggctatcactac | 224378 | - | see also 3920 line | | |
| 16857 | HPD | NM_002150.2 | 327 | ttgcgttcgaggtggaagattgt | 224388 | - | - | 4-hydroxyphenylpyruvate dioxygenase | - |
| 16858 | INDO | NM_002164.3 | 1143 | gagctaccatctgcaaatcgtga | 224422 | - | - | indoleamine-pyrrole 2、3-dioxygenase | - |
| 16859 | HGD | NM_000187.1 | 317 | ttgtccacggagcaccaataaga | 224429 | - | - | homogentisate 1、2-dioxygenase | alkaptonuria |
| 16860 | CDO1 | NM_001801.1 | 422 | caggtatacccgaaatcttgtgg | 224458 | - | - | electron transporter | Parkinson disease |
| 16861 | BCDO1 | NM_017429.1 | 1623 | gaggatgacggagtaatcttatc | 224499 | - | see also 7003 line | | |
| 16862 | BCDO2 | NM_031938.1 | 951 | gtgtaatacgcggtttcatgtgg | 224539 | - | see also 9058 line | | |
| 16863 | FLJ32115 | NM_152321.1 | 473 | gggttattcaacagcgtaattca | 224572 | - | - | - | - |
| 16864 | TMLHE | NM_018196.1 | 1029 | gtgccattgaagcatgaatatat | 224602 | - | see also 7312 line | | |

Figure 46

| 16865 | ALDRL6 | NM_017584.4 | 842 | aggggctcattgacaagtactgc | 224612 | - | - | - | - |
|-------|--------|-------------|-----|-------------------------|--------|---|---|---|---|
| 16866 | ERP70 | NM_004911.3 | 525 | aagcagtttgctccggaatatga | 224622 | - | - | protein disulfide isomerase | - |
| 16867 | P5 | NM_005742.2 | 221 | tcgaattaactccatcgaatttc | 224650 | - | - | protein disulfide isomerase | - |
| 16868 | PDIP | NM_006849.1 | 1137 | ttgggatcagcggccagttaaga | 224680 | - | - | protein disulfide isomerase | - |
| 16869 | PDIR | NM_006810.1 | 1105 | cagagtttcctacgttgaagtat | 224708 | - | see also 4785 line | | |
| 16870 | TXN | NM_003329.1 | 106 | ttggacgctgcaggtgataaact | 224717 | - | signal_transduction | electron transporter | - |
| 16871 | TXN2 | NM_012473.3 | 270 | caggatggacctgactttcaaga | 224722 | - | - | electron transporter | - |
| 16872 | TXNL | NM_004786.1 | 271 | cagactcgccgtggtcaagttca | 224727 | - | see also 3300 line | | |
| 16873 | AMID | NM_032797.4 | 867 | atgagtatcgagagtacatcaaa | 224761 | - | see also 9285 line | | |
| 16874 | FLJ22028 | NM_024854.1 | 332 | tggcaatcagcacgtatataaga | 224772 | - | - | - | - |
| 16875 | FLJ23322 | NM_024955.4 | 1213 | tcgagctagctacgaatccaaag | 224798 | - | - | - | - |
| 16876 | FLJ30473 | NM_144704.1 | 1681 | gcgcgtggcagcccagaacatgt | 224821 | - | - | - | - |
| 16877 | MTO1 | NM_012123.1 | 1097 | atgacgctaccagctgagttaca | 224845 | - | - | - | - |
| 16878 | PDCD8 | NM_004208.2 | 1494 | ctgcatgcttctacgatataaag | 224934 | - | see also 2816 line | | |
| 16879 | SQRDL | NM_021199.1 | 479 | aagatctcctaccgatatcttat | 224959 | - | - | - | - |
| 16880 | ALDH1A2 | NM_003888.2 | 361 | aaggggacgtctgttggataagc | 225006 | - | - | - | - |
| 16881 | ALDH2 | NM_000690.2 | 1843 | gtgtgggtcaactgctatgatgt | 225053 | - | - | electron transporter | colorectal cancer、 alcohol abuse inhibitory factor、 uterine leiomyoma、 alcoholism、 colorectal cancers |
| 16882 | ALDH3A2 | NM_000382.1 | 911 | ctgcattgcacccgactatattc | 225071 | - | - | aldehyde dehydrogenase (NAD+) | Sjogren-Larsson syndrome |
| 16883 | ALDH4A1 | NM_003748.2 | 733 | gggcaacgtggtcctatggaagc | 225098 | - | - | - | hyperprolinemia |
| 16884 | ALDH7A1 | NM_001182.1 | 1056 | atgggaccctaatgttctctatg | 225117 | - | - | aldehyde dehydrogenase (NAD+) | - |
| 16885 | ALDH9A1 | NM_000696.2 | 781 | caggtggatcgtttggttatacc | 225155 | - | - | electron transporter | - |
| 16886 | ALDH1A3 | NM_000693.1 | 460 | ctgtattagaaccctcagatact | 225202 | - | - | aldehyde dehydrogenase (NAD(P)+) | - |
| 16887 | ALDH3A1 | NM_000691.3 | 1146 | cagcaacgacaaggtgattaaga | 225237 | - | - | electron transporter | - |
| 16888 | ALDH3B2 | NM_000695.2 | 421 | acggtccacgaacctgttcatga | 225240 | - | - | aldehyde dehydrogenase (NAD(P)+) | - |

Figure 46

| | | | | | | | | | |
|---|---|---|---|---|---|---|---|---|---|
| 16889 | GAPDS | NM_014364.3 | 704 | ctggctccatgaacattgtgagc | 225249 | - | - | glyceraldehyde 3-phosphate dehydrogenase (phosphorylating) | - |
| 16890 | ALDH5A1 | NM_001080.3 | 1124 | aagaacctgcgcgtaggtaatgg | 225278 | - | - | - | - |
| 16891 | ALDH6A1 | NM_005589.2 | 843 | ttgcgatcatccggacatcaaag | 225320 | - | see also 3880 line | | |
| 16892 | PDHA1 | NM_000284.1 | 1042 | aagagtgaccctattatgcttct | 225349 | - | - | pyruvate dehydrogenase (lipoamide) | - |
| 16893 | PDHA2 | NM_005390.3 | 660 | cagatagccgaagctttcaatat | 225370 | - | - | - | - |
| 16894 | PDHB | NM_000925.1 | 115 | gtgacagttcgtgatgctataaa | 225382 | - | see also 602 line | | |
| 16895 | BCKDHA | NM_000709.2 | 195 | ggggcctcggcggagtttataga | 225409 | - | see also 434 line | | |
| 16896 | BCKDHB | NM_000056.2 | 233 | ccgggagtacgggcaaactcaga | 225434 | - | - | 3-methyl-2-oxobutanoate dehydrogenase (lipoamide) | maple syrup urine disease |
| 16897 | DHTKD1 | NM_018706.4 | 1314 | cagttccgcaaggatgtgattat | 225522 | - | - | - | - |
| 16898 | FLJ10851 | NM_018245.1 | 2978 | gcgcgcacggcccatatggtatg | 225581 | - | - | - | - |
| 16899 | COX11 | NM_004375.2 | 270 | aagagctcgaaccctttcacacg | 225585 | - | - | electron transporter | - |
| 16900 | COX15 | NM_004376.3 | 378 | ctggcctctcgatggtagattgg | 225587 | - | - | - | - |
| 16901 | COX4I2 | NM_032609.2 | 442 | cagcgggtctacgtatttcctcc | 225609 | - | - | cbb3-type cytochrome c oxidase | - |
| 16902 | COX5A | NM_004255.2 | 297 | cgggcatgcagacggttaaatga | 225624 | - | - | electron transporter | - |
| 16903 | COX5B | NM_001862.2 | 134 | tggtgttcccactgatgaagagc | 225630 | - | - | cbb3-type cytochrome c oxidase | - |
| 16904 | COX6B | NM_001863.3 | 296 | ccgctaaaggaggcgatatctct | 225636 | - | - | cbb3-type cytochrome c oxidase | - |
| 16905 | COX6C | NM_004374.2 | 108 | caggcgtctgcgaaatcatatgg | 225638 | - | - | cbb3-type cytochrome c oxidase | leiomyoma |
| 16906 | COX7A2L | NM_004718.2 | 171 | ttgccacaccaactaaactgacc | 225646 | - | - | electron transporter | - |
| 16907 | COX7A3 | NM_183003.1 | 263 | cagcaatcacttggttcacattc | 225655 | - | - | - | - |
| 16908 | COX7B | NM_001866.2 | 247 | tggacatatgtagcaacacaagt | 225660 | - | - | cbb3-type cytochrome c oxidase | - |
| 16909 | COX7C | NM_001867.2 | 191 | gtggtcgttactagctaagatgt | 225664 | - | - | cbb3-type cytochrome c oxidase | - |
| 16910 | CYB5 | NM_001914.1 | 98 | aggtgtacgatttgaccaaattt | 225672 | - | - | cytochrome c oxidase | methemoglobinemia |
| 16911 | SURF1 | NM_003172.2 | 748 | caggcgcagagcccatcttcatt | 225680 | - | - | cytochrome c oxidase | Leigh syndrome |
| 16912 | FLJ13852 | NM_023078.1 | 735 | ccgggtggcaccaccatctatgg | 225688 | - | | | |

Figure 46

| | | | | | | | | | |
|---|---|---|---|---|---|---|---|---|---|
| 16913 | LOC122945 | NM_138791.1 | 562 | ttgcggcctcagtatcagtatga | 225705 | - | - | - | - |
| 16914 | P5CR2 | NM_013328.2 | 228 | ctgtcggctcacaagataatagc | 225712 | - | see also 5400 line | | |
| 16915 | QDPR | NM_000320.1 | 789 | acggaactcaccccagcatattt | 225737 | - | see also 267 line | | |
| 16916 | PRODH | NM_016335.2 | 503 | taggacagaggctattcaacaag | 225740 | - | - | proline dehydrogenase | hyperprolinemia、schizophrenia |
| 16917 | PRODH2 | NM_021232.1 | 1147 | aagctggtacgaggtgcatatct | 225752 | - | - | - | - |
| 16918 | ETFDH | NM_004453.1 | 1717 | cagccggcacacttaaccttaag | 225801 | - | see also 2985 line | | |
| 16919 | PIPOX | NM_016518.1 | 477 | ccggaggagttatctatgcatat | 225817 | - | see also 6899 line | | |
| 16920 | MAOA | NM_000240.2 | 972 | atgagtgcaaatacgtaattaat | 225842 | - | - | amine oxidase (flavin-containing) | alcoholism |
| 16921 | MAOB | NM_000898.3 | 319 | tggaccaacccagaatcgtatct | 225858 | - | see also 583 line | | |
| 16922 | PNPO | NM_018129.1 | 781 | ctgcatgaccggatagtctttcg | 225925 | - | see also 7270 line | | |
| 16923 | DAO | NM_001917.3 | 537 | tggttccacacaagcctaattct | 225929 | - | see also 1221 line | | |
| 16924 | GLDC | NM_000170.1 | 401 | tggcgagcattgatgaattgatc | 225947 | - | see also 142 line | | |
| 16925 | MEGF11 | NM_032445.1 | 2930 | aagaaggttgcggtcataactcc | 226015 | - | - | - | - |
| 16926 | QP-C | NM_014402.2 | 177 | tcgcgtggtgccgcagtttgtag | 226023 | - | - | PALNuclear respiratory factor-1-1 | - |
| 16927 | UQCR | NM_006830.2 | 73 | gggtcccgacggcctacacatgg | 226029 | - | - | ubiquinol-cytochrome c reductase | - |
| 16928 | UQCRFS1 | NM_006003.1 | 888 | cagtgacgatatggtgattgttg | 226041 | - | - | ubiquinol-cytochrome c reductase | - |
| 16929 | AKR1B10 | NM_020299.3 | 1155 | gggcctgtaacgtgttgcaatcc | 226043 | - | - | electron transporter | - |
| 16930 | PCYOX1 | NM_016297.2 | 584 | atgcttaatcgaacacttcttga | 226070 | - | - | lyase | - |
| 16931 | MSRA | NM_012331.2 | 496 | ttgcaggaggctatacttcaaat | 226113 | - | - | protein-methionine-S-oxide reductase | - |
| 16932 | RRM1 | NM_001033.2 | 1880 | cagggcccatacgaaacctatga | 226213 | - | see also 631 line | | |
| 16933 | RRM2 | NM_001034.1 | 1222 | tagagaacccatttgactttatg | 226234 | - | see also 632 line | | |
| 16934 | RRM2B | NM_015713.2 | 264 | cagcagaagaggtcgacttatca | 226239 | - | - | - | - |
| 16935 | MDGA1 | NM_153487.2 | 1456 | ctgcgcatcgagcgtattgcacg | 226256 | - | - | - | - |
| 16936 | FDXR | NM_004110.2 | 553 | ctgtgacacagccgtgattctgg | 226280 | - | - | - | - |
| 16937 | SC4MOL | NM_006745.2 | 731 | gggcatgggtgaccattcgttta | 226300 | - | - | C-4 methyl sterol oxidase | - |
| 16938 | AKR1D1 | NM_005989.1 | 542 | aagacgctggcttggtgaaatcc | 226324 | - | see also 4183 line | | |

Figure 46

| 16939 | ALDH1B1 | NM_000692.3 | 718 | aggcaacactgtggttatgaagg | 226341 | - | - | aldehyde dehydrogenase (NAD+) | - |
|---|---|---|---|---|---|---|---|---|---|
| 16940 | ALDH8A1 | NM_022568.2 | 1283 | aagagccaacaacgttaagtatg | 226375 | - | see also 8421 line | | |
| 16941 | AMACR | NM_014324.3 | 439 | ccggttagctggccacgatatca | 226382 | - | - | alpha-methylacyl-CoA racemase | - |
| 16942 | C10orf33 | NM_032709.1 | 102 | cggcgtggagacgagataacacg | 226404 | - | - | - | - |
| 16943 | CED-6 | NM_016315.1 | 636 | gtggagttgcaaatatcaattta | 226435 | - | see also 6804 line | | |
| 16944 | CHDH | NM_018397.1 | 1489 | gagtgtgggctggctcaaactga | 226455 | - | see also 7439 line | | |
| 16945 | CRYL1 | NM_015974.1 | 449 | atgatcgagtgatcttaagcagt | 226471 | - | - | oxidoreductase、acting on CH-OH group of donors | - |
| 16946 | CYB5R1 | NM_016243.1 | 381 | aagggtgtgcaccccaaatttcc | 226481 | - | - | - | - |
| 16947 | CYBB | NM_000397.2 | 96 | tggtattaccgggtttatgatat | 226487 | - | see also 319 line | | |
| 16948 | DCXR | NM_016286.1 | 386 | caggggccatcgtgaatgtctcc | 226537 | - | - | - | - |
| 16949 | DECR2 | NM_020664.3 | 576 | gaggggtgatcgtgaacatcact | 226545 | - | - | - | - |
| 16950 | DHCR24 | NM_014762.1 | 1421 | gggagccgcgtgtgaaacacttt | 226564 | - | see also 5949 line | | |
| 16951 | DHCR7 | NM_001360.1 | 605 | aggggttgtgaacaagtatcaga | 226570 | - | - | oxidoreductase | Smith-Lemli-Opitz syndrome |
| 16952 | DHRS1 | NM_138452.1 | 531 | gtgctggtcaacaatgcttatgc | 226578 | - | - | - | - |
| 16953 | DHRS10 | NM_016246.1 | 636 | atggtgtccgagtcaactgtatc | 226596 | - | - | - | - |
| 16954 | DHRS7 | NM_016029.1 | 163 | acggcgacctgacgctactatgg | 226600 | - | - | - | - |
| 16955 | DHRS8 | NM_016245.1 | 553 | gtgtagtctatacatcagatttg | 226632 | - | - | - | - |
| 16956 | DHRS9 | NM_005771.3 | 2098 | gtgcacgtctcatgcattgaacc | 226660 | - | see also 4026 line | | |
| 16957 | DHRSX | NM_145177.1 | 134 | ctgaccgtgtcgctatagtgacg | 226666 | - | - | - | - |
| 16958 | DKFZp566 O084 | NM_015510.3 | 426 | tggctatgtcgacatacttgtca | 226678 | - | - | - | - |
| 16959 | ERO1LB | NM_019891.2 | 944 | aagcttatatcgggacttcatgc | 226709 | - | see also 7747 line | | |
| 16960 | FADS2 | NM_004265.2 | 1041 | ctgggccgtcagctactacatcc | 226739 | - | see also 2865 line | | |
| 16961 | FADS3 | NM_021727.3 | 973 | ctgctcaccctggtgaactttga | 226747 | - | - | - | - |
| 16962 | FLJ11042 | NM_018308.1 | 220 | ctgacccctcgagattagctgt | 226754 | - | - | - | - |
| 16963 | FLJ11577 | NM_025159.1 | 1092 | tagccgcctattgcaattgatgt | 226786 | - | - | - | - |
| 16964 | FLJ12661 | NM_025138.2 | 1471 | tggtaccattagcgaatgtcata | 226800 | - | - | - | - |
| 16965 | FLJ20643 | NM_017916.1 | 359 | cagaccagaatcgacacacaatcc | 226853 | - | - | - | - |
| 16966 | FVT1 | NM_002035.1 | 804 | ttggagactcgacttatttcaga | 226878 | - | - | oxidoreductase | follicular lymphoma |
| 16967 | FZD7 | NM_003507.1 | 1346 | ctgttcgtctacctcttcatagg | 226892 | - | receptor_acitivity、gpcr、signal_transduction | frizzled receptor | - |

Figure 46

| ID | Gene | Accession | No. | Sequence | No. 2 | | Annotation | Function | Disease |
|---|---|---|---|---|---|---|---|---|---|
| 16968 | GMIP | NM_016573.1 | 1789 | gtgcacggctgagatagaacacc | 226904 | - | - | | |
| 16969 | H17 | NM_017547.2 | 337 | tggaacggaccacacgtattca | 226914 | - | see also 7028 line | | |
| 16970 | HSD17B12 | NM_016142.1 | 473 | gtgggaatgtgtatgagtatcc | 226943 | - | - | | |
| 16971 | KIAA0769 | NM_014824.1 | 1843 | gagcaataatccgtatcctgaac | 227028 | - | see also 6026 line | | |
| 16972 | KREMEN2 | NM_024507.2 | 926 | gcggctggggcgtctatgaagtgt | 227037 | - | signal_transduction | | |
| 16973 | LISCH7 | NM_015925.3 | 1690 | aggtctcccacgagtaatggttgg | 227041 | - | see also 6573 line | | |
| 16974 | LOC283985 | NM_178128.2 | 1111 | ccgtcgctatgaggaattcatcg | 227053 | - | - | | |
| 16975 | MAWBP | NM_022129.1 | 677 | tggtgggcagacccaagcatttg | 227062 | - | - | oxidoreductase | |
| 16976 | MGC10204 | NM_153329.2 | 1616 | ctgccgctgggcctgaaatagg | 227073 | - | | | |
| 16977 | MGC11335 | NM_030819.2 | 651 | cagctgatttcggaacactatgt | 227079 | - | | | |
| 16978 | MGC15763 | NM_138381.1 | 496 | aggctgcactgagattgaca | 227085 | - | | | |
| 16979 | MGC23280 | NM_144683.2 | 504 | ccggacccgtgaggcgtttaacc | 227111 | - | | | |
| 16980 | MGC4172 | NM_024308.2 | 554 | atgtctgccaccgagtgttacc | 227134 | - | | | |
| 16981 | MRPS30 | NM_016640.2 | 537 | gaggagagcgaggtcatatcttt | 227143 | - | see also 6964 line | | |
| 16982 | NOX3 | NM_015718.1 | 1684 | cagcagtattggcgtgttcttct | 227201 | - | see also 6541 line | | |
| 16983 | NR1 | NM_014434.1 | 1773 | ccggacgcagccgcgtatctagc | 227216 | - | | | melanoma |
| 16984 | PECR | NM_018441.2 | 333 | ttggtcaaatctacctttagatac | 227222 | - | apoptosis | | |
| 16985 | PHYH | NM_006214.2 | 908 | cagtgccgattgccactacattg | 227276 | - | | oxidoreductase | Refsum disease |
| 16986 | RDH-E2 | NM_138969.2 | 985 | tcgggcgatgtttccatcctaatc | 227290 | - | | | |
| 16987 | RDH10 | NM_172037.1 | 978 | ttgtttgccctatcttgtaga | 227312 | - | see also 10033 line | | |
| 16988 | RDH11 | NM_016026.2 | 709 | tgggcgttacgacgtattctgtac | 227336 | - | see also 6626 line | | |
| 16989 | RDH12 | NM_152443.1 | 1088 | cagctgtgagcttctaggaatcc | 227352 | - | | | |
| 16990 | RDH13 | NM_138412.2 | 1117 | aagtacttcgatggactcaaaca | 227363 | - | | | |
| 16991 | RDH14 | NM_020905.1 | 804 | tggtattgtacggacaaatctgg | 227376 | - | | | |
| 16992 | RODH | NM_003725.2 | 933 | gtgcatccgcgaactcgatattc | 227396 | - | | | |
| 16993 | SCDR9 | NM_178135.2 | 340 | ggagatctatgcctcctaaatca | 227413 | - | | | |
| 16994 | SDR-O | NM_148897.1 | 696 | cagctacgcgaggagattatttcc | 227440 | - | | | |
| 16995 | SELO | NM_031454.1 | 737 | gtgccgcacgtgttctatgatgg | 227447 | - | see also 9033 line | | |
| 16996 | SEPW1 | NM_003009.1 | 212 | atggtagccggaagttgattca | 227458 | - | | selenium binding | |
| 16997 | SHREW1 | NM_018836.2 | 447 | aagccatgcctggatactgatag | 227461 | - | | | |
| 16998 | SLC35C2 | NM_015945.10 | 638 | ccgtctcgctgtacacaatgacc | 227473 | - | | | |
| 16999 | TM7SF2 | NM_003273.1 | 938 | tcgcctggctatcctattaacg | 227484 | - | | oxidoreductase | |

Figure 46

| 17000 | TSTA3 | NM_003313.2 | 59 | caggatccatgccggattctagt | 227488 | - | - | oxidoreductase | - |
|---|---|---|---|---|---|---|---|---|---|
| 17001 | ANAPC2 | NM_013366.2 | 1485 | gcggcgttcatcggacatcatca | 227506 | - | cell_cycle, kinase_rel ated | | - |
| 17002 | ANAPC4 | NM_013367.1 | 503 | tgggagacgtcaggcttaatatt | 227519 | - | see also 5410 line | | - |
| 17003 | ANAPC5 | NM_016237.3 | 2183 | gcgcatcaggagcgtcgtttact | 227626 | - | see also 6739 line | | - |
| 17004 | APC10 | NM_014885.1 | 245 | ctgtttggtcactctcatcttgc | 227629 | - | cell_cycle | | - |
| 17005 | C14orf130 | NM_018108.2 | 1092 | aggagcgatcccctaatggatac | 227657 | - | see also 7256 line | | - |
| 17006 | C6orf133 | NM_015255.1 | 801 | tgggcgtaggtcgttcgtgatatg | 227687 | - | - | | - |
| 17007 | CDC34 | NM_004359.1 | 294 | tggttgaacgagggcgatctatac | 227832 | - | cell_cycle | ubiquitin-protein ligase | - |
| 17008 | CEB1 | NM_016323.1 | 369 | cggacgccgaaatgcattaaatt | 227839 | - | cell_cycle, kinase_rel ated | | - |
| 17009 | DD5 | NM_015902.3 | 4528 | ctggctagtactagcatagatgc | 228044 | - | - | ubiquitin-protein ligase | - |
| 17010 | DKFZP564 G092 | NM_015601.2 | 3172 | caggtagtgatcgcattcctatt | 228245 | - | see also 6502 line | | - |
| 17011 | E2-230K | NM_022066.2 | 2002 | ctgacccacctgactttaggttc | 228273 | - | - | | - |
| 17012 | FBXL3A | NM_012158.1 | 817 | ctgtcttcgcttaagatagatga | 228320 | - | see also 5154 line | | - |
| 17013 | FBXL3B | NM_012159.1 | 1029 | ctgtcctcgacttgattgagttag | 228376 | - | - | | - |
| 17014 | FBXL5 | NM_012161.2 | 1438 | ctgtttgcacgatttaactaaca | 228413 | - | see also 5162 line | | - |
| 17015 | FBXL6 | NM_012162.1 | 946 | cggcatcaaccgtaatagccattc | 228426 | - | - | | - |
| 17016 | FBXL7 | NM_012304.3 | 1707 | tcggcaaatgcccttttgtatcc | 228436 | - | - | | - |
| 17017 | FBXO11 | NM_012167.1 | 1539 | atggattaccctcaaataagtgacc | 228479 | - | - | | - |
| 17018 | FBXO2 | NM_012168.2 | 824 | acgatgagacgtcaagaagtac | 228529 | - | - | ubiquitin-protein ligase | - |
| 17019 | FBXO21 | NM_015002.1 | 841 | ggggaatcgaatggattactata | 228553 | - | see also 6184 line | | - |
| 17020 | FBXO24 | NM_012172.2 | 774 | gtgacacagtttaccgtaaatac | 228576 | - | - | | - |
| 17021 | FBXO3 | NM_012175.2 | 1101 | cagtcggtatatgaataacaca | 228624 | - | see also 5178 line | | - |
| 17022 | FBXO4 | NM_012176.1 | 828 | atgttcagtcgacaacaatgaagg | 228650 | - | - | | - |
| 17023 | FBXO5 | NM_012177.2 | 171 | tcgaccctcgatagttgtaaag | 228662 | - | - | | - |
| 17024 | FBXO7 | NM_012179.2 | 519 | atgacattccagcgcctaatata | 228713 | - | see also 5180 line | | - |
| 17025 | FBXO9 | NM_012347.2 | 832 | cggcctcgtttcgtttgatgg | 228769 | - | - | | - |
| 17026 | FBXW1B | NM_012300.1 | 1373 | aggggatcgcctggttgttagtgg | 228817 | - | see also 5267 line | | - |
| 17027 | FLJ11011 | NM_018299.1 | 367 | aagagacgaccaccggataattc | 228863 | - | transcription_regulator | | - |
| 17028 | FLJ13855 | NM_023079.2 | 943 | ttgatgcgctgggactgatacg | 228881 | - | see also 8548 line | | - |
| 17029 | FLJ25157 | NM_152653.1 | 681 | cagcatcgccacacagtactga | 228892 | - | - | | - |
| 17030 | FTS | NM_022476.1 | 855 | aagaccctatgcaattagcttt | 228917 | - | - | | - |

Figure 46

| 17031 | HACE1 | NM_020771.2 | 2456 | gagtacgtccagcttgttactga | 228993 | - | see also 7947 line | | |
|---|---|---|---|---|---|---|---|---|---|
| 17032 | HECTD1 | NM_015382.1 | 2829 | atggctcgagatttatacgatga | 229083 | - | receptor_acitivity | - | - |
| 17033 | HERC1 | NM_003922.1 | 11203 | tggcgcattcctcaagatactac | 229527 | - | - | - | cowden disease、leukemia |
| 17034 | HERC2 | NM_004667.2 | 13250 | gaggcggctttccggaaagtagt | 229707 | - | see also 3188 line | | |
| 17035 | HERC3 | NM_014606.1 | 2550 | gtggactagctatctacaactcc | 229792 | - | see also 5769 line | | |
| 17036 | HIP2 | NM_005339.3 | 387 | atgactctccgcacggtattatt | 229824 | - | see also 3728 line | | |
| 17037 | HSPC150 | NM_014176.1 | 226 | ctgcgagctcaaatattaggtgg | 229842 | - | see also 5525 line | | |
| 17038 | ITCH | NM_031483.3 | 1059 | cagcacgggcgagtttactatgt | 229881 | - | - | - | - |
| 17039 | KIAA0010 | NM_014671.1 | 3151 | gagtggctccgaatgtttgatca | 230017 | - | see also 5849 line | | |
| 17040 | KIAA0317 | NM_014821.1 | 1814 | gagagcttcggcaggtacatatg | 230065 | - | see also 6017 line | | |
| 17041 | KIAA0322 | NM_015052.1 | 4507 | ctgcggtggagcgcttcaataat | 230151 | - | see also 6210 line | | |
| 17042 | LOC51619 | NM_015983.2 | 468 | aggccgacagagagaagtacaac | 230161 | - | - | - | - |
| 17043 | LOC92912 | NM_173469.1 | 1257 | gagcccaacaatcctataattcc | 230173 | - | - | - | - |
| 17044 | MGC35130 | NM_152489.1 | 381 | cagtttggcagggtttagtcttc | 230180 | - | - | - | - |
| 17045 | NCE2 | NM_080678.1 | 348 | aagttcccgatgcgtacaacatg | 230202 | - | - | - | - |
| 17046 | NEDD4 | NM_006154.1 | 798 | aagcacaacgtgcatttaccacc | 230234 | - | see also 4260 line | | |
| 17047 | RBAF600 | NM_020765.1 | 13901 | tggtggagcgattcaaaccatac | 230724 | - | - | - | - |
| 17048 | SMURF1 | NM_020429.1 | 1549 | aggagtcttaccgccagataatg | 230786 | - | see also 7871 line | | |
| 17049 | SMURF2 | NM_022739.2 | 2267 | aggcccgagactctttaccatac | 230863 | - | transcription_regulator | ubiquitin-protein ligase | - |
| 17050 | TRIP12 | NM_004238.1 | 4552 | cagtcgatactggtattacttgt | 230994 | - | see also 2828 line | | |
| 17051 | UBE2A | NM_003336.2 | 591 | aagcgtgtttctgcaatagtaga | 231075 | - | - | - | - |
| 17052 | UBE2B | NM_003337.2 | 448 | cgggatttcaagcggttacaaga | 231078 | - | see also 2257 line | | |
| 17053 | UBE2D1 | NM_003338.3 | 585 | cagatattgcacaaatctataaa | 231113 | - | see also 2258 line | | |
| 17054 | UBE2D2 | NM_003339.2 | 903 | tggtctccagcactaactatttc | 231119 | - | - | ubiquitin-protein ligase | - |
| 17055 | UBE2E1 | NM_003341.3 | 509 | aggttacatttcggacaagaatc | 231134 | - | - | ubiquitin-protein ligase | - |
| 17056 | UBE2G1 | NM_003342.3 | 470 | ccgatgggaagtccttattattg | 231144 | - | see also 2259 line | | |
| 17057 | UBE2G2 | NM_003343.4 | 329 | atgggagagtctgcatttccatc | 231160 | - | - | - | - |
| 17058 | UBE2H | NM_003344.2 | 454 | gagtaaacatgaggttacgatcc | 231162 | - | - | - | - |
| 17059 | UBE2I | NM_003345.3 | 540 | cagaggcctacacgatttactgc | 231193 | - | - | - | - |
| 17060 | UBE2J1 | NM_016021.2 | 353 | aagagtccggctgttaaacgttt | 231195 | - | see also 6622 line | | |
| 17061 | UBE2J2 | NM_058167.2 | 671 | ctgaagtcgtggaggagattaaa | 231242 | - | - | - | - |
| 17062 | UBE2L6 | NM_004223.3 | 331 | gtggacgagaacggacagatttg | 231249 | - | - | ubiquitin-protein ligase | - |
| 17063 | UBE2R2 | NM_017811.2 | 804 | ttgctttacgacgacttgtatga | 231308 | - | see also 7156 line | | |

EP 1 752 536 A1

Figure 46

| ID | Gene | Accession | No. | Sequence | | Notes | Function | | Disease |
|---|---|---|---|---|---|---|---|---|---|
| 17064 | UBE2V2 | NM_003350.2 | 242 | cagaagctcctccgtcagttaga | - | cell cycle | | - | |
| 17065 | UBE3A | NM_000462.2 | 1577 | aagttagacgtgaccatatcata | - | see also 364 line | | - | |
| 17066 | UBE3B | NM_130466.2 | 3303 | tagccgattccgttccattatca | - | see also 9545 line | | - | |
| 17067 | UBR1 | NM_174916.1 | 3834 | tggcgttgagtcttcgattaaat | - | see also 10125 line | | - | |
| 17068 | C6orf104 | NM_031949.2 | 611 | tcgtcatgcccaatgactatacc | - | | | - | |
| 17069 | FLJ35808 | NM_173623.1 | 593 | gttggaaggttccgactgattat | - | | | - | |
| 17070 | FLJ36119 | NM_153254.1 | 869 | ttgatgaaacccagatatggatc | - | | | - | |
| 17071 | KIAA0153 | NM_015140.1 | 1449 | acggttgttcgtgtatgatgtgt | - | | tubulinyl-tyrosine ligase | - | |
| 17072 | KIAA0173 | NM_014640.1 | 1342 | tggaaacctcctgcggtaaatca | - | | | - | |
| 17073 | KIAA0998 | NM_015072.2 | 111 | atgatcggcgaggtggatttatt | - | | | - | |
| 17074 | LOC158135 | NM_194252.1 | 1357 | atgagcacgaagggtgtttgaaa | - | | | - | |
| 17075 | TTL | NM_153712.2 | 808 | tggttggatcatcagtataatatc | - | see also 10017 line | tubulinyl-tyrosine ligase | - | |
| 17076 | TTLL1 | NM_012263.2 | 1152 | atgctatggctacgacatcatca | - | | tubulinyl-tyrosine ligase | - | |
| 17077 | GCLC | NM_001498.1 | 1824 | agggagtttatcgcaaaccatcc | - | see also 1027 line | | - | |
| 17078 | GCLM | NM_002061.1 | 372 | gcgaggagcttcatgattgtatc | - | | translation regulator | - | malignant mesothelioma |
| 17079 | PAICS | NM_006452.2 | 264 | aagtctacgaattgttagacagt | - | | | - | |
| 17080 | MCCC2 | NM_022132.3 | 1189 | gggtaccagtaggtatcgttgg | - | | methylcrotonyl-CoA carboxylase | - | methylcrotonylglycinuria |
| 17081 | PCCB | NM_000532.2 | 403 | aggccgaatcaatggaagattgg | - | | propionyl-CoA carboxylase | - | hyperglycinemia, propionic acidemia |
| 17082 | CTPS | NM_001905.1 | 457 | caggcgttaatacctgtagatga | - | see also 1218 line | | - | |
| 17083 | CTPS2 | NM_019857.3 | 1688 | cagacatcggttcgaggtaaacc | - | see also 7740 line | | - | |
| 17084 | HLCS | NM_000411.3 | 1617 | cagaacttccctacgattatagc | - | see also 325 line | | - | |
| 17085 | ASNS | NM_001673.2 | 814 | ctgcacgccctctatgacaatgt | - | | | - | |
| 17086 | LIAS | NM_006859.2 | 842 | cagtccctacgtgractgaaaca | - | | | - | |
| 17087 | GLUL | NM_002065.3 | 885 | cagatgggcacccctttggttgg | - | | glutamate-ammonia ligase | - | Alzheimer disease |
| 17088 | GLULD1 | NM_016571.1 | 1126 | ctgccgaaagcgttattccaagg | - | see also 6919 line | | - | |
| 17089 | HAL | NM_002108.2 | 1134 | ctgggctgatgctaaatacgtgc | - | see also 1359 line | | - | |
| 17090 | BCL7C | NM_004765.2 | 248 | gggcgacacttcccttcgtatct | - | | | - | |
| 17091 | RCL1 | NM_005772.2 | 302 | cagcttcataaggctattggaca | - | see also 4030 line | | - | |
| 17092 | SLC27A2 | NM_003645.2 | 669 | ctggttgtcgcagaactacaagc | - | | long-chain-fatty-acid-CoA-ligase | - | |
| 17093 | SLC27A5 | NM_012254.1 | 1639 | gcggcaatcgggcgacgttact | - | | | - | |
| 17094 | SLC27A6 | NM_014031.1 | 1693 | gttggctatcaggttatgaagg | - | | | - | |

Figure 46

| | Gene | Accession | | Sequence | | | see also | Function | Disease |
|---|---|---|---|---|---|---|---|---|---|
| 17095 | SUCLA2 | NM_003850.1 | 1107 | tggtgctacagtccatcaagtaa | 232516 | - | | succinate-CoA ligase (ADP-forming) | - |
| 17096 | ACAS2L | NM_032501.2 | 1879 | tggccgacatcggttggattaca | 232532 | - | see also 9222 line | | |
| 17097 | AACS | NM_023928.2 | 281 | tggtccgttgagtcatattcaga | 232546 | - | | | - |
| 17098 | BIRC6 | NM_016252.1 | 3629 | gtggtatgcgtcctgtagtaagg | 232678 | - | see also 6755 line | | - |
| 17099 | BTRC | NM_003939.2 | 1527 | ttggtgcgttgattcgattga | 233052 | - | see also 2696 line | | |
| 17100 | C20orf18 | NM_006462.2 | 1543 | gaggattaccagcgatttctaga | 233063 | - | | | Alzheimer disease |
| 17101 | GGCX | NM_000821.2 | 1297 | tgcggaactgggctaccttaacc | 233162 | - | see also 520 line | | |
| 17102 | LIPT1 | NM_015929.2 | 1176 | tggttgccattggaaatacgtga | 233218 | - | | | - |
| 17103 | MOCS3 | NM_014484.3 | 257 | ggggaccgcgtgcgtgctaatcg | 233257 | - | | ligase | - |
| 17104 | PARC | NM_015089.1 | 6871 | aagcggtttcaggactataatga | 233344 | - | | | - |
| 17105 | RNF25 | NM_022453.2 | 855 | ctgagcgaaacgatacttcatc | 233359 | - | see also 8361 line | | - |
| 17106 | UBE1 | NM_003334.2 | 378 | ggggcgtggagatcgctaagaaca | 233369 | - | | | |
| 17107 | UBE1L | NM_003335.2 | 913 | ttgcgtggtggggctatcactga | 233413 | - | | ubiquitin conjugating enzyme | |
| 17108 | C18B11 | NM_152260.1 | 960 | ctgtaaagaaaccatcttagtgg | 233438 | - | | | small cell lung cancer, non-small-cell lung cancer |
| 17109 | CLONE24922 | NM_015679.1 | 349 | caggctcctcacgatatgtaca | 233451 | - | | | - |
| 17110 | FLJ14494 | NM_032795.1 | 216 | cagcgggagagtgcaagaaatagt | 233463 | - | | | - |
| 17111 | PUS1 | NM_025215.3 | 534 | atgtcgggtcctcacaattcaaa | 233472 | - | | pseudouridylate synthase | |
| 17112 | TRUB1 | NM_139169.3 | 901 | gggaccatttacgctagaagaac | 233500 | - | | | |
| 17113 | ECH1 | NM_001398.1 | 208 | acgtctgcgcagaaaacatgttct | 233509 | - | | isomerase | - |
| 17114 | ECHS1 | NM_004092.2 | 555 | cagccggagatcttaataggaac | 233522 | - | see also 2760 line | | |
| 17115 | UROS | NM_000375.1 | 832 | tggtgacaatatcgatcaaatta | 233537 | - | | uroporphyringonen-III synthase | |
| 17116 | ALAD | NM_000031.3 | 731 | atgagctacagtgccaaattgc | 233541 | - | | porphobilinogen synthase | porphyria, diabetes |
| 17117 | FH | NM_000143.2 | 720 | aagattggacgtactcatactca | 233567 | - | see also 128 line | | |
| 17118 | CBS | NM_000071.1 | 1093 | agggatcggctacgaacttcatcc | 233582 | - | see also 45 line | | |
| 17119 | TGDS | NM_014305.1 | 625 | cagtcttactgggaacaatataa | 233602 | - | | | - |
| 17120 | GMDS | NM_001500.1 | 465 | tggcactctacgacttctagatg | 233624 | - | see also 1030 line | | |
| 17121 | FLJ31300 | NM_144639.1 | 1311 | gagttcgctacccttcctatgt | 233666 | - | | urocanate hydratase | |
| 17122 | PTS | NM_000317.1 | 130 | gcgcgagccacgattgtacagt | 233671 | - | | magnesium binding | hyperphenylalaninemia |
| 17123 | ODC-p | NM_052998.2 | 1550 | ggggcgtgatgggatcttcaact | 233698 | - | | | |
| 17124 | ODC1 | NM_002539.1 | 1535 | cagaggccgacgatctactatgt | 233735 | - | | translation regulator | esophageal cancer |
| 17125 | GAD1 | NM_000817.1 | 1118 | cagctctccactggattggatat | 233749 | - | see also 515 line | | |
| 17126 | GAD2 | NM_000818.1 | 1678 | ttgcgtactcggaagacaatga | 233816 | - | see also 516 line | | |

Figure 46

| | | | | | | | | | porphyria cutanea |
|---|---|---|---|---|---|---|---|---|---|
| 17127 | UROD | NM_000374.2 | 790 | atgatcatctttgctaaggatgg | 233828 | - | - | uroporphyrinogen decarboxylase | tarda、diabetes、porphyria、porphyria hepatoerythropoietic |
| 17128 | HDC | NM_002112.1 | 1200 | tcggtccttcggggtgaagaatc | 233836 | - | see also 1361 line | | |
| 17129 | DDC | NM_000790.1 | 1250 | aggatccccgctttgaaatctgt | 233858 | - | - | aromatic-L-amino acid decarboxylase | - |
| 17130 | AMD1 | NM_001634.3 | 623 | cagtgggtttgactcaattcaaa | 233870 | - | - | adenosylmethionine decarboxylase | - |
| 17131 | KIAA0251 | NM_015027.1 | 1640 | caggtatgaacatgctaatgatg | 233885 | - | - | - | - |
| 17132 | SGPL1 | NM_003901.2 | 1687 | cggaaacgagtagctatacaatt | 233937 | - | see also 2657 line | | |
| 17133 | ALDOA | NM_000034.2 | 1975 | ctgcgcaggaggagtatgtcaag | 233944 | - | - | USF43/USF44 complex | anemia hemolytic |
| 17134 | ALDOB | NM_000035.2 | 668 | tggactggtacctattgttgaac | 233950 | - | - | fructose-bisphosphate aldolase | fructosemia |
| 17135 | ALDOC | NM_005165.1 | 640 | gagaccacgacctcaaacgttgt | 233968 | - | - | fructose-bisphosphate aldolase | - |
| 17136 | CGI-26 | NM_015954.1 | 917 | aagccagaactctttcgaatagg | 233996 | - | see also 6588 line | | |
| 17137 | HMGCL | NM_000191.1 | 481 | cagcgcagtcagccaatatttct | 234037 | - | see also 158 line | | |
| 17138 | GUCY1A2 | NM_000855.1 | 2273 | gggtgcgaatgccacgttattgc | 234073 | - | - | receptor guanylate cyclase | - |
| 17139 | GUCY1A3 | NM_000856.1 | 714 | cagtcggagccgagtctatcttc | 234082 | - | receptor_acitivity、signal_transduction | receptor guanylate cyclase | - |
| 17140 | GUCY1B2 | NM_004129.1 | 1087 | aagtcggactacactcaaacttc | 234141 | - | see also 2786 line | | |
| 17141 | GUCY1B3 | NM_000857.1 | 1211 | aggctacagctcacgttaagagc | 234196 | - | see also 560 line | | |
| 17142 | SAC | NM_018417.2 | 881 | gtgtacgaccttcatgcattatt | 234230 | - | - | - | - |
| 17143 | CGI-150 | NM_016080.2 | 506 | ttgaactactggtgtaatctact | 234347 | - | - | - | - |
| 17144 | GLO1 | NM_006708.1 | 455 | gaggattcggtcatattggaatt | 234369 | - | - | lactoylglutathione lyase | - |
| 17145 | HCCS | NM_005333.1 | 826 | taggcacgattggatcataaacc | 234383 | - | - | holocytochrome c synthase | - |
| 17146 | CTH | NM_001902.4 | 808 | atgggcctggtgtctgttaattg | 234417 | - | - | - | cystathioninuria |
| 17147 | SDS | NM_006843.2 | 937 | cagccctggccgctgtctatagc | 234442 | - | - | L-serine ammonia-lyase | - |
| 17148 | ASL | NM_000048.2 | 1107 | tggccactggcgtcatctctacg | 234452 | - | - | argininosuccinate lyase | argininosuccinicaciduria |
| 17149 | ADSL | NM_000026.1 | 1087 | tggtcgtgtaccccaaagtaatt | 234473 | - | see also 2 line | | |
| 17150 | C20orf3 | NM_020531.1 | 329 | ccggagtccatagcacatattgg | 234485 | - | - | - | - |

Figure 46

| No. | Symbol | Accession | Length | Sequence | | GI | Note | | Description |
|---|---|---|---|---|---|---|---|---|---|
| 17151 | FLJ10916 | NM_018271.2 | 381 | ctgtggcatggcgtcacatatgc | | 234489 | - | - | |
| 17152 | LSS | NM_002340.2 | 316 | gggcactgacgggtgattatgg | | 234508 | - | - | lyase |
| 17153 | SDS-RS1 | NM_138432.2 | 631 | ccgtttgaccacccctaatag | | 234536 | - | - | |
| 17154 | SRR | NM_021947.1 | 430 | acggagcgtcaattgtatactgt | | 234548 | see also 8210 line | - | |
| 17155 | FBXO18 | NM_032807.3 | 2128 | atgacgctacttgaaactctgg | | 234600 | see also 9288 line | - | |
| 17156 | LOC150678 | NM_138336.1 | 452 | ggggcggaggcgctaatgttacc | | 234626 | - | - | |
| 17157 | PEO1 | NM_021830.3 | 1843 | cagacctcaatcgtatcttgaag | | 234663 | see also 8181 line | - | |
| 17158 | FKBP11 | NM_016594.1 | 391 | gagaagcggaaggcaatcattcc | | 234673 | - | - | |
| 17159 | KIAA0073 | NM_015342.1 | 1742 | tggctaacgcggatcaaatact | | 234722 | see also 6383 line | - | |
| 17160 | PIN4 | NM_006223.2 | 78 | gagcggttcagcgttcaacaaca | | 234733 | | | cyclophilin-type peptidy-prolyl cis-trans isomerase |
| 17161 | PPIB | NM_000942.3 | 410 | cagcaaattccatcgtgtaatca | | 234739 | | | cyclophilin-type peptidy-prolyl cis-trans isomerase |
| 17162 | PPIF | NM_005729.2 | 324 | gagaaagggcttcggctacaaagg | | 234740 | | | cyclophilin-type peptidy-prolyl cis-trans isomerase |
| 17163 | PPIL1 | NM_016059.3 | 346 | tggctcgtcgaggttactacaat | | 234745 | | | cyclophilin-type peptidy-prolyl cis-trans isomerase |
| 17164 | PPIL2 | NM_014337.2 | 1127 | aggcacgggtggggagtcatact | | 234773 | - | - | |
| 17165 | PPIL3 | NM_032472.3 | 498 | atgttagaggtfgtatctatg | | 234778 | - | - | |
| 17166 | SDCCAG10 | NM_005869.1 | 602 | atgttcgactgtcagaagtaga | | 234800 | - | - | |
| 17167 | EBP | NM_006579.1 | 351 | gggctgttcgttcttctactacg | | 234818 | receptor_acitivity | - | transmembrane receptor |
| 17168 | DCI | NM_001919.1 | 273 | aagagcttccgccggtgtcattct | | 234824 | - | - | dodecenoyl-CoA delta-isomerase |
| 17169 | JPH2 | NM_020433.3 | 1173 | aagcagccggtgccaagctatgagg | | 234828 | - | - | |
| 17170 | RPIA | NM_144563.1 | 715 | gggcgtggttgaacttcgaatgg | | 234869 | - | - | ribose-5-phosphate isomerase |
| 17171 | PGDS | NM_014485.1 | 117 | aagctgactggcctgaaatcaaa | | 234881 | signal_transduction | - | prostaglandin-D synthase |
| 17172 | PTGIS | NM_000961.2 | 986 | gagagctcgagagtatcctttgg | | 234899 | - | - | human cytochromes P450 family |
| 17173 | PMM1 | NM_002676.1 | 244 | cgggacggtcagtataagcacg | | 234912 | - | - | phosphomannomutase |

Figure 46

| ID | Symbol | Accession | Length | Sequence | Code | | Note | Function | Disease |
|---|---|---|---|---|---|---|---|---|---|
| 17174 | PMM2 | NM_000303.1 | 180 | aggcgatcggactttgagaaag | 234919 | - | | phosphomannomutase | carbohydrate-deficient glycoprotein syndrome(-) |
| 17175 | PGM3 | NM_015599.1 | 1036 | aagttcaacacggtatcttgaag | 234975 | - | see also 6499 line | | |
| 17176 | FLJ32029 | NM_173582.2 | 1053 | tggggtcggacatgactatgtgc | 235012 | - | | | |
| 17177 | PGM2 | NM_018290.1 | 635 | tgggacgattctttaattgatag | 235049 | - | | | |
| 17178 | PGM5 | NM_021965.2 | 1542 | tggatcccaagacgacacatattat | 235091 | - | | structural molecule | |
| 17179 | MUT | NM_000255.1 | 921 | cagatgattggagtacctctaga | 235126 | - | see also 199 line | | |
| 17180 | GALE | NM_000403.2 | 165 | tggtcatcgataacttccataat | 235169 | | | UDP-glucose 4-epimerase | galactosemia |
| 17181 | MCEE | NM_032601.2 | 386 | gaggtggataatattaatgcagc | 235189 | - | | | |
| 17182 | ISYNA1 | NM_016368.3 | 1231 | gcgcgctggatgagtataccccg | 235196 | - | | | |
| 17183 | EBRP | NM_032565.1 | 249 | tagtaggaaacgttgcaaattcc | 235201 | - | | isomerase | |
| 17184 | RENBP | NM_029910.4 | 549 | aggtgcagcgaaccatcttcagt | 235206 | - | | isomerase | |
| 17185 | TOPORS | NM_005802.2 | 649 | atgtcctaagccttcgtataat | 235224 | - | see also 4046 line | | |
| 17186 | TXNDC5 | NM_022085.2 | 1291 | aggctacccacgttattgcttt | 235327 | - | | | |
| 17187 | 101F6 | NM_007022.3 | 349 | tggcaaccggtgctctatgtcttt | 235330 | - | | | |
| 17188 | BRE | NM_004899.3 | 955 | aaggtgcagtacgttgattcaagg | 235360 | - | see also 3403 line | | |
| 17189 | HSPC155 | NM_016406.1 | 487 | gagtttgacattcctatcacata | 235379 | - | | | |
| 17190 | NEDD8 | NM_006156.1 | 130 | aaggagattgagattgacattga | 235385 | - | | ubiquitin conjugating enzyme | |
| 17191 | UBE1C | NM_003968.3 | 1266 | cagtcgtaacctcttattgaaga | 235433 | - | see also 2703 line | | |
| 17192 | UBE4A | NM_004788.2 | 2382 | ccgcgctcccatgtatcctatcc | 235515 | - | see also 3309 line | | |
| 17193 | SAE1 | NM_005500.1 | 296 | ctgggtctgttggccgaaatagg | 235544 | - | | | |
| 17194 | APG7L | NM_006395.1 | 1846 | cagtgacgatcggatgaatgagc | 235609 | - | | | |
| 17195 | UBA2 | NM_005499.1 | 384 | ctgcccgaaaccatgttaataga | 235631 | - | see also 3839 line | | |
| 17196 | ABHD2 | NM_007011.4 | 755 | acgtgatcgtccggtgtttgaac | 235698 | - | | | |
| 17197 | ABHD3 | NM_138340.3 | 1007 | gagtttgataagcgattcacttc | 235748 | - | see also 9602 line | | |
| 17198 | ACP33 | NM_016630.2 | 850 | ttgatcagagtgcgctttcaact | 235777 | - | | | |
| 17199 | APPBP1 | NM_003905.2 | 1349 | atgttaccggctgttgatagatt | 235816 | - | see also 2661 line | | |
| 17200 | BAT5 | NM_021160.1 | 1126 | atgtcctacccagatgttagtgc | 235835 | - | see also 8113 line | | |
| 17201 | BG1 | NM_015162.3 | 641 | ctgccagtacatcgcttatgact | 235845 | - | | | |
| 17202 | BGR | NM_030924.2 | 548 | gcgtttccacggagttggtatcc | 235871 | - | | | |
| 17203 | BUCS1 | NM_052956.1 | 1173 | cagtcggaaacgggactaattg | 235930 | - | | | |
| 17204 | C12orf5 | NM_020375.1 | 90 | ttgtccggcatggagaaacaaga | 235935 | - | | | |
| 17205 | C20orf135 | NM_080622.1 | 29 | tggtgcgcgttcaagatctac | 235952 | - | | | |
| 17206 | C20orf22 | NM_015600.2 | 442 | aagagttccctatttcattgatt | 235961 | - | see also 6501 line | | |
| 17207 | C21orf106 | NM_015151.1 | 2153 | ctgtcgatgaacggtctaagtta | 236006 | - | | | |

Figure 46

| | | | | | | | | | |
|---|---|---|---|---|---|---|---|---|---|
| 17208 | C5orf4 | NM_016348.1 | 1297 | tcggccacacgccctaatgatgg | 236028 | - | | | Alzheimer disease |
| 17209 | CGI-111 | NM_016048.1 | 428 | tagcgtgggacagagattgttgc | 236046 | - | | | - |
| 17210 | CGI-67 | NM_016014.1 | 635 | aggactaggatcacggattaatt | 236070 | - | see also 6615 line | | - |
| 17211 | CTMP | NM_053055.2 | 262 | tggaaacgtttgccttcatataaa | 236084 | - | | | - |
| 17212 | DKFZP566J2046 | NM_031208.1 | 586 | aagggagttggaccggttaaaga | 236098 | - | | | |
| 17213 | ECHDC1 | NM_018479.1 | 491 | aggtgttatgatgctacaacttc | 236109 | - | | | - |
| 17214 | ECHDC3 | NM_024693.2 | 547 | gtgattaccatgccgaagtattt | 236134 | - | | | - |
| 17215 | FA2H | NM_024306.2 | 988 | gggctacgtcctctatgacatga | 236143 | - | | | - |
| 17216 | FHL5 | NM_020482.3 | 796 | gagaattgccgacaacctatagg | 236158 | - | | hydrolase | melanoma |
| 17217 | FLJ10948 | NM_018281.1 | 807 | gagaagcggactcccaatttgt | 236179 | - | | | - |
| 17218 | FLJ12377 | NM_024989.1 | 1136 | ctgacttaacaggacatctatg | 236229 | - | | | - |
| 17219 | FLJ14906 | NM_032859.1 | 422 | ccgatgcgttaccttccttatg | 236266 | - | | | - |
| 17220 | FLJ20581 | NM_017888.2 | 556 | cggacagggactgtgatgattcc | 236281 | - | | | - |
| 17221 | FLJ20920 | NM_025149.2 | 422 | gggagactaactcctatgcatgg | 236288 | - | | | - |
| 17222 | FLJ21820 | NM_021925.1 | 581 | ttgtgctggtttcgatatgttct | 236322 | - | | | - |
| 17223 | FLJ21963 | NM_024560.2 | 620 | atgatccacagcegatgtatac | 236355 | - | see also 8643 line | | - |
| 17224 | FLJ25449 | NM_144714.1 | 447 | tggccacgctggtttccaatgg | 236405 | - | | | - |
| 17225 | FLJ32332 | NM_144641.1 | 639 | ttggtgcgagaagatgagatacg | 236426 | - | phosphatase | | - |
| 17226 | FLJ37964 | NM_182578.1 | 667 | gaggggcgttcacactaagtct | 236431 | - | | | - |
| 17227 | FLJ40125 | NM_178494.2 | 558 | gagcgttgggcgactttacctac | 236436 | - | | | - |
| 17228 | FLJ40773 | NM_152666.1 | 1628 | ctggcacgggccttgttatcaac | 236484 | - | | | - |
| 17229 | GAF1 | NM_015470.1 | 2012 | tggaccagtcggccaagtactac | 236494 | - | | | - |
| 17230 | GENX-3414 | NM_003943.1 | 1116 | gagttaccgctgggaagaatgc | 236512 | - | | | - |
| 17231 | GPHN | NM_020806.3 | 154 | ttgttgggtggactataatcagc | 236522 | - | see also 7978 line | | - |
| 17232 | GPR56 | NM_005682.3 | 1255 | gtgagacgtcaggaggagaaac | 236577 | - | gpcr, receptor acitivity, signal transduction | | melanoma |
| 17233 | HSRTSBETA | NM_017512.1 | 854 | gtgccacaatagagtgatattta | 236592 | - | | | - |
| 17234 | KIAA1463 | NM_020849.1 | 736 | caggcgatcgatgcattcatca | 236610 | - | see also 7993 line | | - |
| 17235 | KIAA1959 | NM_032873.2 | 1601 | tggttgtttccgagattacgaga | 236675 | - | see also 9329 line | | - |
| 17236 | LIPH | NM_139248.1 | 190 | gtggttggtacgggactaaatgt | 236692 | - | | | - |
| 17237 | LOC122618 | NM_138790.1 | 1339 | tcgtgccggtgggaaccattcc | 236730 | - | | | - |
| 17238 | LOC197322 | NM_174917.1 | 1784 | ttggagttccggatatgacatgg | 236743 | - | | | - |

Figure 46

| | | | | | | | | | |
|---|---|---|---|---|---|---|---|---|---|
| 17239 | LOC93081 | NM_138779.2 | 762 | aggacggtgacaatgatgtt | 236763 | - | | | |
| 17240 | LYPLAL1 | NM_138794.1 | 193 | cagctcctccagatcatatact | 236769 | - | | | |
| 17241 | MCOLN1 | NM_020533.1 | 868 | atgagatcccggactgctatacc | 236804 | - | channel | | mucolipidosis |
| 17242 | MGC15429 | NM_032750.1 | 723 | aagactccagctctgattgtata | 236815 | - | | | |
| 17243 | MGC19531 | NM_005167.4 | 2222 | ctgggtctcccaacaacaagc | 236829 | - | see also 3635 line | | |
| 17244 | MGC5352 | NM_138575.1 | 431 | tggccaagcttgggttgaagtt | 236832 | - | | | |
| 17245 | MOCS2 | NM_004531.3 | 863 | ctgtgagctatgccattgatact | 236849 | - | see also 3087 line | | |
| 17246 | NDRG1 | NM_006096.2 | 243 | caggacaatcagagacttacatgg | 236859 | - | | enzyme | breast cancer |
| 17247 | NDRG3 | NM_022477.2 | 163 | tggtgtggtccacgtcactataa | 236867 | - | see also 8401 line | | |
| 17248 | OAZIN | NM_015878.3 | 1428 | aggattcacggaactgaatttc | 236970 | - | see also 6548 line | | |
| 17249 | PGS1 | NM_024419.2 | 1425 | acgctgattggctctcctaattt | 236999 | - | | | |
| 17250 | PIGB | NM_004855.3 | 1442 | ttgttcctcgccctttatactgg | 237037 | - | | | |
| 17251 | PLEKHF1 | NM_194449.1 | 1613 | ctgaacgcctctgcgaacaaact | 237101 | - | see also 10309 line | | |
| 17252 | PME-1 | NM_016147.1 | 379 | gtgttcacggcagccgattattag | 237135 | - | see also 6695 line | | |
| 17253 | PNPLA1 | NM_173676.1 | 599 | tggtgatcctgcacgattactac | 237164 | - | | | |
| 17254 | PPM1L | NM_139245.1 | 713 | aagaagcagttcgattcatcaag | 237189 | - | see also 9682 line | | |
| 17255 | SAH | NM_005622.1 | 678 | tagacgctgttgcatccaaatgt | 237214 | - | see also 3901 line | | |
| 17256 | SERAC1 | NM_032861.2 | 2011 | caggctactttacaattcattcg | 237291 | - | | | |
| 17257 | SLC27A3 | NM_024330.1 | 1332 | ggggagctgtgccgatacttgt | 237299 | - | | | |
| 17258 | SLC27A4 | NM_005094.2 | 2096 | gggcttgacccggctattgtga | 237324 | - | | | |
| 17259 | TA-PP2C | NM_139283.1 | 520 | atgcggacgtgtgaacgtttagt | 237330 | - | see also 9687 line | | |
| 17260 | TA-WDRP | NM_139281.1 | 1748 | ggtctcgcagtggatggattaaac | 237398 | - | see also 9686 line | | |
| 17261 | TTS-2.2 | NM_020376.2 | 567 | cagacggcgagaatgtcattata | 237435 | - | | | |
| 17262 | UBASH3A | NM_018961.1 | 1348 | acggagtcgtgggatcaaagact | 237452 | - | | enzyme | |
| 17263 | UPP2 | NM_173355.2 | 1036 | tcgactgtgatcagatcaacttg | 237492 | - | see also 10065 line | | |
| 17264 | UXS1 | NM_025076.1 | 905 | tggggccacgtgaatccaatagg | 237498 | - | | | |
| 17265 | HTR1B | NM_000863.1 | 55 | tgggttcctcaagccaacttatc | 237510 | - | receptor_acitivity, gpcr | serotonin receptor | alcoholism |
| 17266 | HTR1D | NM_000864.3 | 1366 | ctggaacgcaaggagagatttctgc | 237531 | - | receptor_acitivity, gpcr | serotonin receptor | |
| 17267 | HTR1E | NM_000865.1 | 733 | ctgccaactacctaatctgttct | 237535 | - | receptor_acitivity, gpcr | serotonin receptor | |
| 17268 | HTR1F | NM_000866.1 | 995 | tggcatggcttgggtatctcaat | 237587 | - | see also 561 line | | |

Figure 46

| | | | | | | | | | |
|---|---|---|---|---|---|---|---|---|---|
| 17269 | HTR2A | NM_000621.1 | 823 | ttgcttactcgccgatgataact | 237615 | - | receptor_acitivity、gpcr、signal_transduction | serotonin receptor | schizophrenia、alcoholism、Alzheimer disease |
| 17270 | HTR2C | NM_000868.1 | 1348 | gcgtgctcaacgacccaaattc | 237655 | - | see also 563 line | | |
| 17271 | HTR4 | NM_000870.2 | 575 | ctgggtcatccccacgtttattt | 237692 | - | see also 565 line | | |
| 17272 | HTR5A | NM_024012.1 | 333 | gaggctgtgccagctttggatcg | 237710 | - | receptor_acitivity、gpcr、signal_transduction | serotonin receptor | - |
| 17273 | HTR7 | NM_000872.3 | 953 | ttggctatacgatttactctacc | 237719 | - | receptor_acitivity、gpcr | - | - |
| 17274 | ADRA2A | NM_000681.2 | 2086 | gtgccacgcacgctcttcaaatt | 237728 | - | gpcr、receptor_acitivity、kinase_related、signal_transduction | potassium channel regulator | - |
| 17275 | ADRA2B | NM_000682.3 | 1039 | aggggcgtgggtgctataggtgg | 237734 | - | gpcr、receptor_acitivity、signal_transduction | alpha2-adrenergic receptor | - |
| 17276 | ADRA1A | NM_000680.1 | 489 | cggcaccggtgaacatttccaag | 237736 | - | gpcr、receptor_acitivity、apoptosis、signal_transduction、kinase_related | alpha1-adrenergic receptor | - |
| 17277 | ADRA1B | NM_000679.2 | 1059 | gggcattgtggtcggtatgttca | 237756 | - | see also 426 line | | |
| 17278 | ADRA1D | NM_000678.2 | 1266 | ctgttccagccgcgagttcaagc | 237762 | - | gpcr、receptor_acitivity、signal_transduction | alpha1-adrenergic receptor | - |
| 17279 | ADRB2 | NM_000024.3 | 1426 | gggaggaattgtagtacaaatga | 237779 | - | receptor_acitivity、gpcr、kinase_related、signal_transduction | translation regulator | obesity |
| 17280 | HRH1 | NM_000861.2 | 1364 | tcgcattcaagacagtatgtatc | 237799 | - | receptor_acitivity、gpcr | histamine receptor | - |
| 17281 | HRH2 | NM_022304.1 | 672 | ccgcaacctgaccaattgtttca | 237804 | - | receptor_acitivity、gpcr | histamine receptor | - |
| 17282 | HRH3 | NM_007232.1 | 859 | ctgctatgccgagttcttctaca | 237815 | - | receptor_acitivity、gpcr | histamine receptor | - |
| 17283 | HRH4 | NM_021624.2 | 657 | cagtcatcttagtcgcttatttc | 237840 | - | receptor_acitivity、gpcr、signal_transduction | histamine receptor | - |

Figure 46

| | | | | | | | | | |
|---|---|---|---|---|---|---|---|---|---|
| 17284 | HTR6 | NM_000871.1 | 638 | gcgcaacacgtccaacttcttcc | 237851 | - | receptor_acitivity、gpcr | histamine receptor | - |
| 17285 | DRD1 | NM_000794.2 | 1783 | tagagacggtgagtatcaataac | 237878 | - | receptor_acitivity、gpcr、signal_transduction | Transcription factor AP-2 alpha | schizophrenia |
| 17286 | DRD2 | NM_000795.2 | 1295 | ttgttctcggcgtgttcatcatc | 237896 | - | see also 490 line | | |
| 17287 | DRD3 | NM_000796.2 | 626 | gtgtacagccagcatccttaatc | 237900 | - | see also 491 line | | |
| 17288 | DRD5 | NM_000798.3 | 87 | ggggcagttcgctctataccagc | 237914 | - | receptor_acitivity、gpcr、signal_transduction | dopamine receptor | blepharospasm、schizophrenia |
| 17289 | OR1A1 | NM_014565.1 | 310 | atgatagccttgggtaacacaga | 237921 | - | receptor_acitivity、signal_transduction、gpcr | olfactory receptor | - |
| 17290 | OR1A2 | NM_012352.1 | 835 | gtgaccccagcattaaatccttt | 237944 | - | receptor_acitivity、signal_transduction、gpcr | olfactory receptor | - |
| 17291 | OR1D2 | NM_002548.1 | 755 | atgggacactttgtatggtatac | 237955 | - | receptor_acitivity、signal_transduction、gpcr | olfactory receptor | - |
| 17292 | OR1D5 | NM_014566.1 | 564 | atgttccaacacccacatcattc | 331771 | - | - | olfactory receptor | - |
| 17293 | OR1G1 | NM_003555.1 | 286 | gggtgtctactacagttgtattt | 237969 | - | receptor_acitivity、signal_transduction、gpcr | olfactory receptor | - |
| 17294 | OR2C1 | NM_012368.1 | 697 | gggaggcgaaaggcgttcaatac | 237983 | - | receptor_acitivity、signal_transduction、gpcr | olfactory receptor | - |
| 17295 | OR3A1 | NM_002550.1 | 231 | cagcgtcactgttccatcaatgt | 237986 | - | receptor_acitivity、signal_transduction、gpcr | olfactory receptor | - |
| 17296 | OR51B4 | NM_033179.1 | 555 | ctgtgctgatatcacgtttaatc | 238003 | - | receptor_acitivity、signal_transduction、gpcr | olfactory receptor | - |
| 17297 | OR51E2 | NM_030774.2 | 858 | tgggcgtggacgtaatgttcatc | 238021 | - | receptor_acitivity、signal_transduction、gpcr | olfactory receptor | - |

Figure 46

| 17298 | OR7A5 | NM_017506.1 | 426 | ttgctgacatttgtgttacttcc | 238028 | - | receptor_acitivity、sig nal_transduction、gpcr | olfactory receptor | - |
| 17299 | OR7C1 | NM_198944.1 | 53 | cagcaacgtcagagattcagttc | 238033 | - | - | - | - |
| 17300 | OR8B8 | NM_012378.1 | 504 | ctgtgccaataaccttgtcaacc | 238048 | - | receptor_acitivity、sig nal_transduction、gpcr | olfactory receptor | - |
| 17301 | TAS2R1 | NM_019599.1 | 391 | ctggggtctctgctatatgtatc | 238063 | - | receptor_acitivity、gpc r、signal_transduction | | - |
| 17302 | TAS2R10 | NM_023921.1 | 379 | atggttcttcccttcatgatagt | 238093 | - | receptor_acitivity、gpc r、signal_transduction | | - |
| 17303 | TAS2R13 | NM_023920.1 | 128 | cagtcgataaactcctcattatc | 238118 | - | receptor_acitivity、gpc r、signal_transduction | | - |
| 17304 | TAS2R14 | NM_023922.1 | 404 | ttgtgacttcggtcttcttgttt | 238147 | - | receptor_acitivity、gpc r、signal_transduction | | - |
| 17305 | TAS2R16 | NM_016945.1 | 288 | cagcttgcttaccgtgttctact | 238164 | - | receptor_acitivity、gpc r、signal_transduction | | - |
| 17306 | TAS2R3 | NM_016943.1 | 374 | gagtttctagggtgatggtatgg | 238191 | - | receptor_acitivity、gpc r、signal_transduction | | - |
| 17307 | TAS2R4 | NM_016944.1 | 478 | gtgactacgagaaataacacatc | 238211 | - | receptor_acitivity、gpc r、signal_transduction | | - |
| 17308 | TAS2R5 | NM_018980.1 | 228 | ttggcttcgctatcttagtatct | 238229 | - | receptor_acitivity、gpc r、signal_transduction | | - |
| 17309 | TAS2R7 | NM_023919.1 | 175 | ttgtgcgtaatactattagattg | 238253 | - | receptor_acitivity、sig nal_transduction、gpcr | | - |
| 17310 | TAS2R8 | NM_023918.1 | 168 | ttgtttgatcagtgtaatggttg | 238274 | - | receptor_acitivity、sig nal_transduction、gpcr | | - |

Figure 46

| | | | | | | | | | |
|---|---|---|---|---|---|---|---|---|---|
| 17311 | TAS2R9 | NM_023917.1 | 227 | atggcaatagcgtgctagtaagc | 238305 | - | receptor_acitivity、sig nal_transduction、gpcr | - | - |
| 17312 | ELOVL4 | NM_022726.2 | 248 | ccgggtagtgtcctaaacgtagt | 238320 | - | gpcr、receptor_acitivit y | G-protein coupled photoreceptor | - |
| 17313 | OPN1SW | NM_001708.1 | 306 | tcgtcttcggtcgccatgtttgt | 238367 | - | receptor_acitivity、gpc r、signal_transduction | opsin | color blindness tritanopic |
| 17314 | OPN3 | NM_014322.1 | 945 | ttgctaaatcgaacactgtatac | 238391 | - | gpcr、receptor_acitivit y、signal_transduction | G-protein coupled photoreceptor | - |
| 17315 | OPN4 | NM_033282.1 | 401 | gggcaacctgacggtcatctata | 238396 | - | gpcr、receptor_acitivit y、signal_transduction | G-protein coupled photoreceptor | - |
| 17316 | RDS | NM_000322.2 | 532 | aagccgtacctggctatctgtgt | 238407 | - | gpcr、receptor_acitivit y | G-protein coupled photoreceptor | retinal degeneration、retinitis pigmentosa |
| 17317 | RHO | NM_000539.2 | 898 | acgccagcgtggcattctacatc | 238427 | - | see also 406 line | | |
| 17318 | ROM1 | NM_000327.2 | 389 | aagtctgaatgcagctctatacc | 238430 | - | gpcr、receptor_acitivit y | G-protein coupled photoreceptor | retinal degeneration |
| 17319 | PTGER1 | NM_000955.2 | 1006 | gggccagcttgtcggtatcatgg | 238443 | - | gpcr、receptor_acitivit y、signal_transduction | prostaglandin E receptor | - |
| 17320 | PTGER2 | NM_000956.2 | 156 | tgggcaatgcctccaatgactcc | 238444 | - | receptor_acitivity、gpc r、signal_transduction | thromboxane receptor | - |
| 17321 | PTGER3 | NM_000957.2 | 720 | ctggtatgcgagccacatgaaga | 238454 | - | receptor_acitivity、gpc r、transcription_regula tor、signal_transductio n | prostaglandin E receptor | - |
| 17322 | PTGER4 | NM_000958.2 | 1885 | gaggactttgcgaatatcagaga | 238487 | - | gpcr、receptor_acitivit y、signal_transduction | prostaglandin E receptor | - |
| 17323 | PTGFR | NM_000959.2 | 651 | atggccattgagcggtgtattgg | 238503 | - | receptor_acitivity、gpc r、signal_transduction | thromboxane receptor | - |

Figure 46

| | | | | | | | | | |
|---|---|---|---|---|---|---|---|---|---|
| 17324 | PTGDR | NM_000953.2 | 930 | gtgttctctgcccgtaatttatc | 238532 | - | receptor_acitivity、gpcr、signal_transduction | thromboxane receptor | - |
| 17325 | TBXA2R | NM_001060.2 | 446 | tgggcgtcgtcatgatcttcttc | 238539 | - | gpcr、receptor_acitivity、signal_transduction | Smad3/Sp-1 heterodimer | bleeding disorder |
| 17326 | EDG1 | NM_001400.2 | 293 | ctgactacgtcaactatgatatc | 238541 | - | receptor_acitivity、signal_transduction、gpcr | lysosphingolipid and lysophosphatidic acid receptor | - |
| 17327 | EDG2 | NM_001401.2 | 861 | ctggactatggccatcgttatgg | 238565 | - | see also 974 line | | |
| 17328 | EDG4 | NM_004720.3 | 441 | ccgcgtggtcatgctcattgtgg | 238576 | - | receptor_acitivity、signal_transduction、gpcr | - | - |
| 17329 | TSHR | NM_000369.1 | 1326 | aagacataatgggctacaagttc | 238619 | - | gpcr、receptor_acitivity | thyroid-stimulating hormone receptor | TSH resistance |
| 17330 | GNRHR | NM_000406.1 | 2257 | ttgcaggaccacagttatacatc | 238654 | - | - | gonadotropin-releasing hormone receptor | Hypogonadotropic hypogonadism |
| 17331 | FSHR | NM_000145.2 | 1219 | tagtgctagtgatcctaactacc | 238708 | - | receptor_acitivity、signal_transduction、gpcr | - | ovarian dysgenesis、ovarian sex cord tumors、premature ovarian failure、ovarian hyperstimulation syndrome |
| 17332 | GPR24 | NM_005297.2 | 519 | ggggagcatctcctacatcaaca | 238723 | - | gpcr、receptor_acitivity、signal_transduction | neuropeptide receptor | - |
| 17333 | GPR105 | NM_014879.1 | 901 | aagaaatctagccgcaacatatt | 238741 | - | gpcr、receptor_acitivity、signal_transduction | UDP-activated nucleotide receptor | - |
| 17334 | P2RY4 | NM_002565.2 | 820 | ctggtcactcttgtttgctatgg | 238764 | - | gpcr、receptor_acitivity | uridine nucleotide receptor | - |
| 17335 | GPR86 | NM_023914.2 | 221 | cagagacactcggatagtacagc | 238770 | - | - | - | - |
| 17336 | GPR87 | NM_023915.2 | 1396 | aagatcggaagttcgcatatatt | 238831 | - | gpcr、receptor_acitivity、signal_transduction | - | - |
| 17337 | P2RY12 | NM_022788.3 | 587 | ctgggactgataactatcgatcg | 238842 | - | gpcr、receptor_acitivity、signal_transduction | - | - |

Figure 46

| | | | | | | | | | |
|---|---|---|---|---|---|---|---|---|---|
| 17338 | GPR135 | NM_022571.3 | 1275 | aagccgcagtcgccttcgaaacc | 238869 | - | receptor_acitivity、gpcr、signal_transduction | - | - |
| 17339 | PTAFR | NM_000952.2 | 416 | cagtaactcggcccatcaagact | 238880 | - | receptor_acitivity、signal_transduction、gpcr | platelet activating factor receptor | - |
| 17340 | CYSLTR1 | NM_006639.2 | 1340 | ttgtccagccgtgacttatgtacc | 238929 | - | see also 4687 line | | |
| 17341 | CYSLTR2 | NM_020377.2 | 283 | agggctgactattatcttagagg | 238940 | - | see also 7856 line | | |
| 17342 | CNR1 | NM_016083.3 | 831 | ctgcgagaaactgcaatctgttt | 238968 | - | see also 6654 line | | |
| 17343 | CNR2 | NM_001841.1 | 1171 | tggccagattccagagatctaga | 238981 | - | receptor_acitivity、gpcr | cannabinoid receptor | - |
| 17344 | MTNR1A | NM_005958.2 | 1055 | acgtggccgatagggttaaatgg | 238998 | - | receptor_acitivity、gpcr | melatonin receptor | Alzheimer disease |
| 17345 | MTNR1B | NM_005959.2 | 647 | gagtacgacccacgcatctattc | 239007 | - | see also 4166 line | | |
| 17346 | TRHR | NM_003301.1 | 523 | tagcacctacaaagatgctattg | 239030 | - | see also 2232 line | | |
| 17347 | ADMR | NM_007264.2 | 975 | tggtccacctgctctacttcttc | 239054 | - | gpcr、receptor_acitivity、signal_transduction | rhodopsin-like receptor | - |
| 17348 | ADORA1 | NM_000674.1 | 1067 | cggcgacccgcagaagtactatg | 239057 | - | gpcr、receptor_acitivity、signal_transduction、apoptosis | A1 adenosine receptor、G-protein coupled | - |
| 17349 | ADORA2A | NM_000675.3 | 1102 | tcgtcctctcccacaccaattcg | 239065 | - | receptor_acitivity、gpcr、apoptosis、signal_transduction | A2A adenosine receptor、G-protein coupled | Parkinson disease |
| 17350 | ADORA2B | NM_000676.2 | 1164 | ctgtcacatgccaattcagttgt | 239078 | - | gpcr、receptor_acitivity、kinase_related、signal_transduction | A2B adenosine receptor、G-protein coupled | - |
| 17351 | ADORA3 | NM_000677.2 | 1346 | gtgcgccatctatcttgacatct | 239091 | - | gpcr、receptor_acitivity、signal_transduction | A3 adenosine receptor、G-protein coupled | - |
| 17352 | AGTRL1 | NM_005161.2 | 490 | gaggcgctcagctgatatcttca | 239102 | - | receptor_acitivity、gpcr、signal_transduction | rhodopsin-like receptor | Alzheimer disease |
| 17353 | BLR1 | NM_001716.2 | 150 | cagattggacaactataacgaca | 239111 | - | gpcr、receptor_acitivity、signal_transduction | - | - |
| 17354 | CMKOR1 | NM_020311.1 | 381 | atgacacgcactgctacatcttg | 239117 | - | see also 7831 line | | |

700

Figure 46

| 17355 | CXCR6 | NM_006564.1 | 950 | ttgtcagcctgaagtttcgaaag | 239142 | - | receptor_acitivity、gpcr、signal_transduction | - | - |
|---|---|---|---|---|---|---|---|---|---|
| 17356 | EBI2 | NM_004951.2 | 1005 | acggcaagtcagtgtatcgattt | 239200 | - | gpcr、receptor_acitivity、signal_transduction | purinergic nucleotide receptor、G-protein coupled | - |
| 17357 | ET(B)R-LP-2 | NM_004767.2 | 362 | aggaactgaggggcaatctgaca | 239204 | - | gpcr、receptor_acitivity、signal_transduction | rhodopsin-like receptor | - |
| 17358 | FKSG79 | NM_032553.1 | 743 | caggggtattcctaatttgcttt | 239239 | - | gpcr、receptor_acitivity、signal_transduction | purinergic nucleotide receptor、G-protein coupled | - |
| 17359 | FY | NM_002036.1 | 1075 | gtggactctgcaccctgatatac | 239260 | - | gpcr、receptor_acitivity、signal_transduction | - | - |
| 17360 | GHSR | NM_004122.1 | 345 | ctgcaaactcttccaattcgtca | 239265 | - | gpcr、receptor_acitivity、signal_transduction | rhodopsin-like receptor | - |
| 17361 | GNRHR2 | NM_057163.1 | 72 | ttgggatgccacctggaatatca | 239272 | - | gpcr、receptor_acitivity、signal_transduction | | - |
| 17362 | GPR | NM_007223.1 | 1886 | ccggtacagtcgccgtaatgtgg | 239308 | - | receptor_acitivity、gpcr、signal_transduction | - | - |
| 17363 | GPR1 | NM_005279.2 | 676 | ctgtggagttcaataatcatact | 239328 | - | gpcr、receptor_acitivity、signal_transduction | rhodopsin-like receptor | - |
| 17364 | GPR100 | NM_181885.1 | 83 | atgctccgatgcctgtcaaattc | 239341 | - | gpcr、receptor_acitivity、signal_transduction | | - |
| 17365 | GPR101 | NM_054021.1 | 99 | ccgctcaaccgtgctggttatct | 239349 | - | gpcr、receptor_acitivity、signal_transduction | - | - |
| 17366 | GPR102 | NM_053278.1 | 808 | tggttaccgtatacagttgatat | 239385 | - | gpcr、receptor_acitivity、signal_transduction | - | - |

Figure 46

| | | | | | | | | | |
|---|---|---|---|---|---|---|---|---|---|
| 17367 | GPR119 | NM_178471.1 | 646 | cggactcccagcgacttcaaagc | 239396 | - | receptor_acitivity、gpcr、signal_transduction | - | - |
| 17368 | GPR12 | NM_005288.1 | 713 | tggccacgtcgcactatgtgacc | 239408 | - | see also 3704 line | | |
| 17369 | GPR120 | NM_181745.1 | 557 | ccgaccaggaaatttcgatttgc | 239410 | - | see also 10252 line | | |
| 17370 | GPR132 | NM_013345.2 | 1481 | caggattgccgggtactactacg | 239419 | - | gpcr、receptor_acitivity、signal_transduction | - | atherosclerosis |
| 17371 | GPR145 | NM_032503.1 | 683 | ctgacgatgtactctggtataca | 239437 | - | gpcr、receptor_acitivity、signal_transduction | - | |
| 17372 | GPR146 | NM_138445.1 | 755 | ctggacgccacactatctgatcc | 239458 | - | receptor_acitivity、gpcr、signal_transduction | - | |
| 17373 | GPR15 | NM_005290.1 | 805 | ttgcggcaagaacactatttacc | 239482 | - | gpcr、receptor_acitivity、signal_transduction | purinergic nucleotide receptor、G-protein coupled | - |
| 17374 | GPR151 | NM_194251.1 | 398 | aagtgagtatccacaactacacc | 239495 | - | see also 10305 line | | |
| 17375 | GPR160 | NM_014373.1 | 941 | tggtacaggctatcaggataact | 239524 | - | gpcr、receptor_acitivity、signal_transduction | - | - |
| 17376 | GPR161 | NM_007369.1 | 1086 | ccggtattatcgggaaccatttg | 239551 | - | gpcr、receptor_acitivity、signal_transduction | - | - |
| 17377 | GPR18 | NM_005292.2 | 1397 | ctggtagtctacggtcactaagc | 239596 | - | gpcr、receptor_acitivity、signal_transduction | purinergic nucleotide receptor、G-protein coupled | - |
| 17378 | GPR19 | NM_006143.1 | 1227 | ttgcttggcccattaactcaaat | 239631 | - | gpcr、receptor_acitivity、signal_transduction | rhodopsin-like receptor | - |
| 17379 | GPR22 | NM_005295.1 | 88 | atgtaccaaccactatcatatcc | 239642 | - | see also 3705 line | | |
| 17380 | GPR23 | NM_005296.1 | 891 | gcgctcccaagctattactaatt | 239708 | - | see also 3710 line | | |
| 17381 | GPR26 | NM_153442.1 | 912 | ccgcatccgacccctttgtgtac | 239721 | - | gpcr、receptor_acitivity、signal_transduction | - | - |

702

Figure 46

| | | | | | | | | | |
|---|---|---|---|---|---|---|---|---|---|
| 17382 | GPR27 | NM_018971.1 | 1103 | gcgacctgaaaggcattggttta | 239725 | - | gpcr、receptor_acitivity、signal_transduction | C-C chemokine receptor | - |
| 17383 | GPR3 | NM_005281.2 | 494 | ccgctacctttctctgtacaatg | 239727 | - | gpcr、receptor_acitivity、signal_transduction | rhodopsin-like receptor | - |
| 17384 | GPR30 | NM_001505.1 | 1293 | ccgacgaggcctgcttctgtttc | 239735 | - | gpcr、receptor_acitivity、signal_transduction | purinergic nucleotide receptor、G-protein coupled | - |
| 17385 | GPR32 | NM_001506.1 | 947 | acgtcttcgttggcagagatttc | 239739 | - | gpcr、receptor_acitivity、signal_transduction | N-formyl peptide receptor | - |
| 17386 | GPR34 | NM_005300.2 | 726 | gtggattcctaactatgattatt | 239770 | - | see also 3711 line | | |
| 17387 | GPR35 | NM_005301.2 | 1067 | gagcacccggcacaatttcaact | 239793 | - | gpcr、receptor_acitivity、signal_transduction | rhodopsin-like receptor | - |
| 17388 | GPR37 | NM_005302.2 | 1267 | gggcacgaagggtggacaattgc | 239805 | - | see also 3712 line | | |
| 17389 | GPR38 | NM_001507.1 | 971 | cggaagattcgcggatgatgtac | 239850 | - | gpcr、receptor_acitivity、signal_transduction | rhodopsin-like receptor | - |
| 17390 | GPR39 | NM_001508.1 | 51 | cagtcatgtccccgagtttgagg | 239854 | - | gpcr、receptor_acitivity、signal_transduction | rhodopsin-like receptor | - |
| 17391 | GPR43 | NM_005306.1 | 428 | tcgtgatcatcgttcaatacttg | 239870 | - | gpcr、receptor_acitivity、signal_transduction | rhodopsin-like receptor | - |
| 17392 | GPR44 | NM_004778.1 | 428 | ctgcactcctccatcttctttct | 239874 | - | gpcr、receptor_acitivity、signal_transduction | N-formyl peptide receptor | - |
| 17393 | GPR48 | NM_018490.1 | 3259 | ttggtgcgctatgcttacaatct | 239969 | - | - | protein-hormone receptor | - |
| 17394 | GPR49 | NM_003667.2 | 1242 | tagcctccgatcgctgaatttgg | 239997 | - | see also 2492 line | | |
| 17395 | GPR50 | NM_004224.1 | 1005 | gcggcaccctatcatattcttcc | 240053 | - | receptor_acitivity、signal_transduction、gpcr | melatonin receptor | - |
| 17396 | GPR52 | NM_005684.1 | 826 | atgctgtggctcccctatataat | 240086 | - | gpcr、receptor_acitivity、signal_transduction | rhodopsin-like receptor | - |

Figure 46

| | | | | | | | | | |
|---|---|---|---|---|---|---|---|---|---|
| 17397 | GPR54 | NM_032551.2 | 126 | ctggctcgtgccgctcttcttcg | 240090 | - | receptor_acitivity、gpcr、signal_transduction | - | - |
| 17398 | GPR55 | NM_005683.2 | 341 | aagaacaggtggcccgattatgc | 240094 | - | - | - | - |
| 17399 | GPR58 | NM_014626.1 | 489 | atgggggaccaccttgtttatgg | 240122 | - | gpcr、receptor_acitivity、signal_transduction | | - |
| 17400 | GPR61 | NM_031936.2 | 731 | gtggagcgctactattacgtagt | 240150 | - | gpcr、receptor_acitivity、signal_transduction | rhodopsin-like receptor | - |
| 17401 | GPR62 | NM_080865.2 | 1005 | ctggccgtgggccaatttgcagc | 240153 | - | gpcr、receptor_acitivity、signal_transduction | rhodopsin-like receptor | - |
| 17402 | GPR63 | NM_030784.1 | 617 | ttgccctggtaactattcttact | 240174 | - | see also 8947 line | | |
| 17403 | GPR73 | NM_138964.1 | 111 | cagctacagcgactatgatatgc | 240204 | - | receptor_acitivity、gpcr、signal_transduction | | - |
| 17404 | GPR73L1 | NM_144773.2 | 198 | ctgcggcatcggtaactttgtct | 240212 | - | receptor_acitivity、gpcr、signal_transduction | - | - |
| 17405 | GPR75 | NM_006794.1 | 1007 | gtgccatgccggctctgtatagg | 240231 | - | receptor_acitivity、gpcr、signal_transduction | | - |
| 17406 | GPR78 | NM_080819.2 | 1175 | ctgctttgccccgtatgtcatga | 240253 | - | gpcr、receptor_acitivity、signal_transduction | | - |
| 17407 | GPR80 | NM_080818.2 | 665 | ttgctataccacgattatccaca | 240280 | - | gpcr、receptor_acitivity、signal_transduction | - | - |
| 17408 | GPR82 | NM_080817.1 | 647 | tagtactaacatcatactactct | 240318 | - | see also 9525 line | | |
| 17409 | GPR83 | NM_016540.2 | 616 | atgctatctgccagaaattattt | 240346 | - | receptor_acitivity、gpcr、signal_transduction | neuropeptide Y receptor | - |
| 17410 | GPR84 | NM_020370.1 | 1212 | ccgccaattccgccaagcatatg | 240371 | - | receptor_acitivity、gpcr、signal_transduction | - | - |
| 17411 | GPR85 | NM_018970.3 | 1177 | ttgtctacctcaagctgatattt | 240388 | - | see also 7615 line | | |

Figure 46

| | | | | | | | | | |
|---|---|---|---|---|---|---|---|---|---|
| 17412 | GPR88 | NM_022049.1 | 1109 | gcgggtcagcgtgctcaacttcc | 240405 | - | receptor_acitivity、 gpcr、 signal_transduction | - | - |
| 17413 | GPR91 | NM_033050.1 | 390 | cagcatagatcgatacttgataa | 240414 | - | gpcr、 receptor_acitivity、 signal_transduction | - | - |
| 17414 | GRCA | NM_014449.1 | 614 | gagggccgagtttgcaaagttcg | 240429 | - | gpcr、 receptor_acitivity、 signal_transduction | - | - |
| 17415 | GREAT | NM_130806.2 | 355 | tagtggatgggcgaccatatttg | 240442 | - | see also 9550 line | | |
| 17416 | H963 | NM_013308.2 | 1054 | gtgtcgaacctgtgctttgatcc | 240525 | - | gpcr、 receptor_acitivity、 signal_transduction | purinergic nucleotide receptor、 G-protein coupled | |
| 17417 | JCG1 | NM_153445.1 | 633 | gtgtgtcatagccatatcctaca | 240540 | - | receptor_acitivity、 signal_transduction、 gpcr | rhodopsin-like receptor | - |
| 17418 | JCG3 | NM_153444.1 | 629 | tagccgtctgctacatctatatc | 240556 | - | receptor_acitivity、 sig nal_transduction、 gpcr | rhodopsin-like receptor | - |
| 17419 | LGR6 | NM_021636.1 | 2171 | ccggtgcctacatcaaactgtac | 240580 | - | - | - | - |
| 17420 | LGR7 | NM_021634.1 | 764 | aaggcaaccatatccataattta | 240607 | - | gpcr、 receptor_acitivity、 signal_transduction | - | - |
| 17421 | LHCGR | NM_000233.1 | 1166 | aagtcgttacaaacttacagtgc | 240682 | - | receptor_acitivity、 gpcr | lutropin-choriogonadotropic hormone receptor | gonadotropin unresponsiveness、 Leydig cell hypoplasia、 micropenis、 Hypogonadotropic hypogonadism |
| 17422 | MAS1L | NM_052967.1 | 844 | gtggcacccctcataacagattt | 240734 | - | gpcr、 receptor_acitivity、 signal_transduction | rhodopsin-like receptor | |
| 17423 | MBC2 | NM_015292.1 | 1031 | caggggcattattcgaattcacc | 240757 | - | - | - | - |
| 17424 | MGC21621 | NM_145015.2 | 567 | gggcacgtttgccgactacatcc | 240794 | - | receptor_acitivity、 gpcr、 signal_transduction | - | - |
| 17425 | MGC26856 | NM_152779.1 | 176 | acgaatggcgtggcaaagtcaac | 240811 | - | - | - | - |
| 17426 | MGC40047 | NM_175911.2 | 910 | ttgggtctatgaatatatggttt | 240838 | - | receptor_acitivity、 sig nal_transduction、 gpcr | - | - |

Figure 46

| 17427 | MRGX1 | NM_147199.1 | 503 | ctgattctgcttggtgtcaaaca | 240851 | - | gpcr、receptor_acitivity、signal_transduction | - | - |
|---|---|---|---|---|---|---|---|---|---|
| 17428 | OPRL1 | NM_000913.3 | 452 | ggggaactgccttgtcatgtacg | 240862 | - | receptor_acitivity、gpcr、signal_transduction | - | - |
| 17429 | OR10H1 | NM_013940.1 | 907 | aagaagaccttcttcagtaaact | 240866 | - | - | olfactory receptor | - |
| 17430 | OR10H3 | NM_013938.1 | 10 | cagaactacagaaccatatctga | 240869 | - | - | olfactory receptor | - |
| 17431 | OR10J1 | NM_012351.1 | 117 | tggcgtgttccttgcactataca | 240873 | - | receptor_acitivity、signal_transduction、gpcr | olfactory receptor | - |
| 17432 | OR11A1 | NM_013937.2 | 737 | ctgacatcttatgccagaattgt | 240895 | - | receptor_acitivity、signal_transduction、gpcr | olfactory receptor | - |
| 17433 | OR12D3 | NM_030959.2 | 537 | atgtcaagccgctcttagaattg | 240911 | - | receptor_acitivity、signal_transduction、gpcr | olfactory receptor | - |
| 17434 | OR1F1 | NM_012360.1 | 817 | atggctactgtgttgtatacagt | 240924 | - | receptor_acitivity、signal_transduction、gpcr | olfactory receptor | - |
| 17435 | OR2H3 | NM_007160.2 | 900 | gagattgctggggaaggaaatgg | 240928 | - | receptor_acitivity、signal_transduction、gpcr | olfactory receptor | - |
| 17436 | OR2J2 | NM_030905.1 | 839 | ctgttgtcacaccgagtcttaat | 240946 | - | receptor_acitivity、signal_transduction、gpcr | - | - |
| 17437 | OR2W1 | NM_030903.1 | 89 | ttgtcgccatcttctacttaatt | 240950 | - | receptor_acitivity、signal_transduction、gpcr | olfactory receptor | - |
| 17438 | OR3A2 | NM_002551.2 | 1068 | atggcaggcacacctttggttct | 240970 | - | receptor_acitivity、signal_transduction、gpcr | olfactory receptor | - |
| 17439 | OR52A1 | NM_012375.1 | 218 | ttgcacttgctagcagcattatg | 240981 | - | receptor_acitivity、signal_transduction、gpcr | olfactory receptor | - |

Figure 46

| | | | | | | | | | | |
|---|---|---|---|---|---|---|---|---|---|---|
| 17440 | OR5I1 | NM_006637.1 | 426 | ctgtatgcggttgattgtcttgt | 241022 | - | receptor_acitivity、sig nal_transduction、gpcr | olfactory receptor | - | |
| 17441 | OR5V1 | NM_030876.3 | 861 | atgtacggcccatctcaacttac | 241048 | - | receptor_acitivity、sig nal_transduction、gpcr | olfactory receptor | - | |
| 17442 | OR6A1 | NM_003696.2 | 262 | ctgcgccactacaggtactattg | 241052 | - | receptor_acitivity、sig nal_transduction、gpcr | olfactory receptor | - | |
| 17443 | P2RY1 | NM_002563.2 | 1538 | gggctgttacggattaattgtga | 241083 | - | see also 1650 line | | | |
| 17444 | P2RY10 | NM_014499.2 | 947 | ttgcgttggtcgggatgattaca | 241110 | - | gpcr、receptor_acitivit y、signal_transduction | purinergic nucleotide receptor、G-protein coupled | - | |
| 17445 | P2RY11 | NM_002566.3 | 50 | cagctgccgacgacaaactcagt | 241121 | - | gpcr、receptor_acitivit y、signal_transduction | rhodopsin-like receptor | - | |
| 17446 | P2RY2 | NM_002564.2 | 526 | tggaatgcgtccaccacatatat | 241128 | - | receptor_acitivity、gpc r | - | - | |
| 17447 | P2RY5 | NM_005767.3 | 1148 | tggcaattgtctacccatttaag | 241139 | - | gpcr、receptor_acitivit y、signal_transduction | purinergic nucleotide receptor、G-protein coupled | - | |
| 17448 | P2RY6 | NM_004154.3 | 350 | ctgtgtctaccgcgagaacttca | 241160 | - | gpcr、receptor_acitivit y | - | - | |
| 17449 | P2RY8 | NM_178129.3 | 373 | cagatccccgtcggtcatcttca | 241167 | - | receptor_acitivity、gpc r、signal_transduction | - | - | |
| 17450 | RRH | NM_006583.1 | 562 | ctggtgctacgtgtaccataaac | 241200 | - | see also 4613 line | | | |
| 17451 | SALPR | NM_016568.1 | 731 | tggcgctgacggactttcagttt | 241227 | - | gpcr、receptor_acitivit y、signal_transduction | N-formyl peptide receptor | - | |
| 17452 | sdolf | NM_033519.1 | 318 | ctgccaacttcctgatactcatg | 241236 | - | - | olfactory receptor | - | |
| 17453 | TA4 | NM_175067.1 | 567 | ttgtcagaccgttgtaaatcaaa | 241261 | - | gpcr、receptor_acitivit y、signal_transduction | - | - | |
| 17454 | TAR1 | NM_138327.1 | 571 | ggggtactgacctttatgacttc | 241279 | - | gpcr、receptor_acitivit y、signal_transduction | - | - | |

Figure 46

| | | | | | | | |
|---|---|---|---|---|---|---|---|
| 17455 | TAR3 | NM_175057.1 | 412 | ctgacctatccaccaagtttac | 241297 | - | gpcr, receptor_acitivity, signal_transduction |
| 17456 | TG1019 | NM_148962.3 | 94 | ccgtggtggccagctgatagtgc | 241311 | - | receptor_acitivity, gpcr, signal_transduction |
| 17457 | VN1R1 | NM_020633.2 | 240 | gagaccacggacttgattctca | 241328 | - | receptor_acitivity, gpcr, signal_transduction |
| 17458 | VN1R2 | NM_173856.1 | 264 | gggggtggacctacactattcc | 241348 | - | receptor_acitivity, gpcr, signal_transduction |
| 17459 | VN1R3 | NM_174980.1 | 108 | ttgtttcaccagaggtatgttac | 241374 | - | receptor_acitivity, gpcr, signal_transduction |
| 17460 | VN1R4 | NM_173857.1 | 145 | ctgattgtagccaacttcttagc | 241392 | - | - |
| 17461 | CASR | NM_000388.2 | 1505 | aagaaagtggcgacaggtttagc | 241435 | - | see also 309 line |
| 17462 | GPRC5B | NM_016235.1 | 341 | tggtgcggctgcccttcatcaag | 241474 | - | - |
| 17463 | GPRC6A | NM_148963.1 | 1416 | atgctcacggggatttaaatact | 241518 | - | receptor_acitivity, gpcr |
| 17464 | GRM1 | NM_000838.2 | 949 | gagcggaatggacgcgtttcaaag | 241566 | - | see also 550 line |
| 17465 | GRM2 | NM_000839.1 | 2189 | gggctgctgcctacaatgtgc | 241622 | - | receptor_acitivity, gpcr, signal_transduction, metabotropic glutamate, GABA-B-like receptor |
| 17466 | GRM3 | NM_000840.1 | 1642 | gggcgatacaacgtgttcaatt | 241647 | - | see also 552 line |
| 17467 | GRM4 | NM_000841.1 | 288 | atgaattccatccgcatagatgg | 241684 | - | gpcr, receptor_acitivity, signal_transduction, metabotropic glutamate, GABA-B-like receptor |
| 17468 | GRM5 | NM_000842.1 | 1500 | tggagatacgatcctattcgatg | 241709 | - | gpcr, receptor_acitivity, signal_transduction, metabotropic glutamate, GABA-B-like receptor |
| 17469 | GRM6 | NM_000843.2 | 1309 | atgattccaccgcaaatgcaca | 241740 | - | gpcr, receptor_acitivity, signal_transduction, metabotropic glutamate, GABA-B-like receptor |
| 17470 | GRM7 | NM_000844.2 | 2529 | atgtacacgacatgtatagtatg | 241801 | - | receptor_acitivity, gpcr, signal_transduction |

Figure 46

| | | | | | | | | | |
|---|---|---|---|---|---|---|---|---|---|
| 17471 | GRM8 | NM_000845.1 | 1292 | tggcttacgccctgcacaatatg | 241834 | - | see also 559 line | | |
| 17472 | RAI3 | NM_003979.2 | 686 | caggatgttatcgctattgaata | 241877 | - | - | - | - |
| 17473 | TAS1R1 | NM_138697.2 | 2249 | aggacttgccagagaactacaac | 241924 | - | receptor_acitivity、gpcr | - | - |
| 17474 | TAS1R2 | NM_152232.1 | 1939 | ctgtatcgccgtgcgttctttcc | 241942 | - | receptor_acitivity、gpcr | - | - |
| 17475 | PTHR1 | NM_000316.2 | 1564 | ttgtcgcaatcatatactgtttc | 241957 | - | gpcr、receptor_acitivity | parathyroid hormone receptor | chondrosarcoma、Ollier disease、metaphyseal chondrodysplasia、chondrodysplasia |
| 17476 | PTHR2 | NM_005048.2 | 1213 | ctgaatacggttagagttctagc | 241987 | - | gpcr、receptor_acitivity、signal_transduction | parathyroid hormone receptor | - |
| 17477 | CALCR | NM_001742.1 | 297 | ctggagtattgtcctatcagttc | 241999 | - | see also 1153 line | | |
| 17478 | RCP9 | NM_014478.3 | 94 | cagtaactacgaggtatttcagt | 242037 | - | receptor_acitivity | calcitonin receptor | - |
| 17479 | CRHR1 | NM_004382.2 | 1018 | tggggtgtacaccgactacatct | 242053 | - | see also 2940 line | | |
| 17480 | CRHR2 | NM_001883.2 | 823 | gtgctcctgatcaatttcgtatt | 242071 | - | see also 1207 line | | |
| 17481 | VIPR1 | NM_004624.2 | 1159 | ttggagtacactacatcatgttc | 242088 | - | gpcr、receptor_acitivity | vasoactive intestinal polypeptide receptor | small cell lung cancer |
| 17482 | GCGR | NM_000160.1 | 798 | atgccatccacgcgaatctgttt | 242093 | - | receptor_acitivity、gpcr、signal_transduction | glucagon receptor | diabetes mellitus、diabetes |
| 17483 | GHRHR | NM_000823.1 | 454 | gtgggccatagcatctctattgt | 242104 | - | receptor_acitivity、gpcr、signal_transduction | G-protein coupled receptor | - |
| 17484 | SCTR | NM_002980.1 | 470 | tggcctgtggcgttaatgtgaac | 242116 | - | receptor_acitivity、gpcr、signal_transduction | secretin receptor | - |
| 17485 | FKSG83 | NM_032030.1 | 794 | atgggactaacgtgtgtctttcc | 242150 | - | receptor_acitivity、signal_transduction、gpcr | - | - |
| 17486 | VN1R5 | NM_173858.1 | 556 | ctgcggctacccccagtgaaacg | 242166 | - | receptor_acitivity、signal_transduction、gpcr | - | - |
| 17487 | ADCYAP1R1 | NM_001118.3 | 652 | tggggaccaggattattactacc | 242188 | - | receptor_acitivity、gpcr、signal_transduction | vasoactive intestinal polypeptide receptor | - |
| 17488 | BAI2 | NM_001703.1 | 4081 | gtggatctggcttgtcaaacagt | 242229 | - | see also 1115 line | | |
| 17489 | BAI3 | NM_001704.1 | 3395 | cagataaacgctccatattgttt | 242331 | - | see also 1116 line | | |

Figure 46

| 17490 | C17orf35 | NM_003876.1 | 664 | gtggagtacgacgcctataaact | 242376 | - | gpcr、receptor_acitivity、signal_transduction | G-protein coupled receptor | - |
| 17491 | C7orf9 | NM_022150.2 | 264 | atgagtacacctgcagtcaataa | 242381 | - | gpcr、receptor_acitivity、signal_transduction | - | - |
| 17492 | CALCRL | NM_005795.2 | 1295 | ctgattcctgcttgtatacatgc | 242418 | - | receptor_acitivity、gpcr | calcitonin receptor | - |
| 17493 | FZD1 | NM_003505.1 | 1324 | aggtgtatgggctcatgtacttc | 242450 | - | gpcr、receptor_acitivity、signal_transduction | frizzled receptor | - |
| 17494 | FZD10 | NM_007197.2 | 2054 | aggtgtgcagccgtaggttaaag | 242460 | - | receptor_acitivity、gpcr、signal_transduction | frizzled receptor | - |
| 17495 | FZD2 | NM_001466.2 | 454 | ccgccgtgccgctctatctgtga | 242465 | - | gpcr、receptor_acitivity、signal_transduction | frizzled receptor | - |
| 17496 | FZD3 | NM_017412.2 | 2314 | cagcatgtcacgactaacagatc | 242533 | - | see also 6984 line | | |
| 17497 | FZD4 | NM_012193.2 | 907 | gtgctcaagtgtggctatgatgc | 242542 | - | see also 5185 line | | |
| 17498 | FZD5 | NM_003468.2 | 813 | ccgaggtcctctgcatggattac | 242561 | - | gpcr、receptor_acitivity、signal_transduction | frizzled receptor | - |
| 17499 | FZD6 | NM_003506.2 | 547 | gtggttccaccttgtcgtaaact | 242568 | - | see also 2363 line | | |
| 17500 | FZD8 | NM_031866.1 | 2040 | acggcgagctccgtgtcttatcc | 242631 | - | receptor_acitivity、gpcr、signal_transduction | frizzled receptor | - |
| 17501 | FZD9 | NM_003508.1 | 1714 | tggcacgcactgccactataagg | 242644 | - | receptor_acitivity、gpcr、signal_transduction | frizzled receptor | - |
| 17502 | GIPR | NM_000164.1 | 279 | ctgtaacgggtccttcgatatgt | 242646 | - | gpcr、receptor_acitivity、signal_transduction | gastric inhibitory peptide receptor | - |
| 17503 | GLP1R | NM_002062.2 | 567 | gcgagcattgtccgtcttcatca | 242662 | - | receptor_acitivity、gpcr、signal_transduction | peptide receptor、G-protein coupled | - |
| 17504 | GLP2R | NM_004246.1 | 285 | ttgtaacgggacatttgatcagt | 242672 | - | see also 2850 line | | |
| 17505 | GPR110 | NM_153840.1 | 1565 | ctgtcacgcaacctagcaatacc | 242739 | - | see also 10021 line | | |

Figure 46

| | | | | | | | | | |
|---|---|---|---|---|---|---|---|---|---|
| 17506 | GPR111 | NM_153839.1 | 536 | ctgagaccataacagatatgttg | 242770 | - | receptor_acitivity、gpcr、signal_transduction | - | - |
| 17507 | GPR112 | NM_153834.1 | 2482 | aagctaagtaccccattggataa | 242864 | - | receptor_acitivity、gpcr、signal_transduction | - | - |
| 17508 | GPR113 | NM_153835.1 | 367 | ctggtctccgtctatgtacatct | 243036 | - | receptor_acitivity、gpcr、signal_transduction | - | - |
| 17509 | GPR115 | NM_153838.1 | 1460 | tggttgtgacggagatatcatac | 243090 | - | receptor_acitivity、gpcr、signal_transduction | - | - |
| 17510 | GPR116 | NM_015234.1 | 72 | ctggaattacgagtctactattc | 243112 | - | gpcr、receptor_acitivity、signal_transduction | - | - |
| 17511 | GPR123 | NM_032422.1 | 951 | aagccgtgttctgattatcaaca | 243201 | - | gpcr、receptor_acitivity、signal_transduction | - | - |
| 17512 | GPR124 | NM_032777.6 | 2690 | gggattccactcattatctgtgg | 243224 | - | gpcr、receptor_acitivity、signal_transduction | - | - |
| 17513 | GPR128 | NM_032787.1 | 2313 | tagcatacctgatgctagttaat | 243302 | - | gpcr、receptor_acitivity、signal_transduction | - | - |
| 17514 | GPR133 | NM_198827.1 | 564 | cagcaagcaccgttactactatg | 243322 | - | - | - | - |
| 17515 | GPR143 | NM_000273.1 | 825 | ttgttggttgtcgaatatcatca | 243335 | - | gpcr、receptor_acitivity、signal_transduction | molecular_function unknown | albinism |
| 17516 | GPR157 | NM_024980.2 | 568 | tggccattgcgctctacttgtac | 243343 | - | receptor_acitivity、gpcr | - | - |
| 17517 | GPR97 | NM_170776.3 | 1713 | caggcccactccgcatctcaaga | 243454 | - | gpcr、receptor_acitivity、signal_transduction | - | - |
| 17518 | LANCL1 | NM_006055.1 | 451 | tggccgctgtgctatatcacaag | 243468 | - | see also 4214 line | | |
| 17519 | TAS2R38 | NM_176817.1 | 797 | ctgtgcccctactgattctgtgg | 243503 | - | receptor_acitivity、gpcr、signal_transduction | - | - |

Figure 46

| 17520 | TAS2R39 | NM_176881.1 | 915 | cagccactcaattctactgattc | 243522 | - | - | - | - |
|---|---|---|---|---|---|---|---|---|---|
| 17521 | TAS2R40 | NM_176882.1 | 164 | ctggtgaccgcattatgttgatg | 243530 | - | receptor_acitivity、gpcr、signal_transduction | - | - |
| 17522 | TAS2R41 | NM_176883.1 | 131 | tggatatgatcctcattagcttg | 243545 | - | receptor_acitivity、gpcr、signal_transduction | - | - |
| 17523 | TAS2R49 | NM_176889.1 | 536 | tagccatgctagcaaacttgata | 243571 | - | receptor_acitivity、gpcr、signal_transduction | - | - |
| 17524 | TAS2R50 | NM_176890.1 | 547 | tggagcttcataccctttactct | 243579 | - | receptor_acitivity、gpcr、signal_transduction | - | - |
| 17525 | TAS2R60 | NM_177437.1 | 137 | gggtgctacggagaatgttgttg | 243583 | - | receptor_acitivity、gpcr、signal_transduction | - | - |
| 17526 | VIPR2 | NM_003382.3 | 344 | gtggcgtctgggacaacatcacg | 243602 | - | gpcr、receptor_acitivity、signal_transduction | vasoactive intestinal polypeptide receptor | - |
| 17527 | XPR1 | NM_004736.2 | 1549 | cagtgcctgcgccgatatcgaga | 243655 | - | see also 3254 line | | |
| 17528 | LIFR | NM_002310.2 | 1401 | gggtcgatcacaatcaacaattt | 243739 | - | see also 1475 line | | |
| 17529 | GHR | NM_000163.1 | 776 | gaggtgctctatgtaacacttcc | 243848 | - | receptor_acitivity | peptide hormone | pituitary dwarfism |
| 17530 | EPOR | NM_000121.2 | 1398 | ctgccagctttgagtacactatc | 243895 | - | receptor_acitivity、signal_transduction | Smad3/Sp-1 heterodimer | erythroleukemia |
| 17531 | GFRA2 | NM_001495.3 | 1094 | cagctgccctgcggacaattacc | 243905 | - | receptor_acitivity、signal_transduction、kinase_related | glial cell line-derived neurotrophic factor receptor | - |
| 17532 | CRLF2 | NM_022148.2 | 1063 | aggcggtgatgtggtcacaatcg | 243914 | - | - | - | - |
| 17533 | CSF3R | NM_000760.2 | 1270 | cagcggacggatccaaggttatg | 243923 | - | receptor_acitivity、signal_transduction | - | agranulocytosis |
| 17534 | F3 | NM_001993.2 | 602 | aagatgaacggactttagtcaga | 243958 | - | receptor_acitivity | Proto-oncogene C-JUN | - |
| 17535 | FAD104 | NM_022763.2 | 1934 | gtgggtcggctaccgaatacacc | 244023 | - | see also 8458 line | | |
| 17536 | IL11RA | NM_004512.3 | 627 | tggagccagtaccggattaatgt | 244076 | - | receptor_acitivity | - | - |
| 17537 | IL13RA1 | NM_001560.2 | 281 | ccggaaactcgtcgttcaataga | 244089 | - | receptor_acitivity、signal_transduction | hematopoietin/interferon-class (D200-domain) cytokine receptor | |

Figure 46

| | | | | | | | | | |
|---|---|---|---|---|---|---|---|---|---|
| 17538 | IL20RA | NM_014432.2 | 738 | ctgactacgaacaccagtattat | 244124 | - | receptor_acitivity | - | - |
| 17539 | IL22RA2 | NM_052962.2 | 628 | ctgttggggtactcaagaactct | 244163 | - | receptor_acitivity | - | - |
| 17540 | MGC34923 | NM_144717.2 | 428 | ctggagaaacagtgtactattct | 244183 | - | receptor_acitivity | - | - |
| 17541 | MPL | NM_005373.1 | 1404 | gcgatctcgctaccgtttacagc | 244209 | - | see also 3743 line | | |
| 17542 | GRIA1 | NM_000827.2 | 692 | gggataccggatgctctttcagg | 244224 | - | see also 528 line | | |
| 17543 | GRIA3 | NM_000828.2 | 384 | ccgctttgccgtgcagttataca | 244314 | - | see also 529 line | | |
| 17544 | GRIK1 | NM_000830.2 | 1245 | ccgggtttcggctgcttaacatt | 244388 | - | see also 540 line | | |
| 17545 | GRIK2 | NM_021956.2 | 1696 | atggaatggttcgtgaactaatt | 244489 | - | see also 8216 line | | |
| 17546 | GRIK3 | NM_000831.2 | 1251 | aggattaactggacgaattgttt | 244536 | - | receptor_acitivity、cha nnel、signal_transduct ion | potassium channel | - |
| 17547 | GRIK4 | NM_014619.2 | 1590 | gggacgcaaacccggctatttct | 244575 | - | see also 5794 line | | |
| 17548 | GRIK5 | NM_002088.3 | 2497 | tggcggtcatggaattcatatgg | 244607 | - | see also 1353 line | | |
| 17549 | GRIA4 | NM_000829.1 | 1097 | atggacgtagagtcaattacaca | 244643 | - | see also 536 line | | |
| 17550 | GRID2 | NM_001510.1 | 61 | gcgaatgcggattcgatcattca | 244676 | - | see also 1034 line | | |
| 17551 | GRIN3A | NM_133445.1 | 3625 | ccgtcagcttaccgtatggaata | 244831 | - | receptor_acitivity、cha nnel | - | - |
| 17552 | CD163 | NM_004244.2 | 1805 | tggagtagtctgctcaagataca | 244864 | - | receptor_acitivity | - | - |
| 17553 | CD5 | NM_014207.2 | 224 | aggacggatggcacatggtttgc | 244896 | - | receptor_acitivity | scavenger receptor | - |
| 17554 | CD5L | NM_005894.1 | 760 | acgtgggtcgaatgtgaagatcc | 244920 | - | receptor_acitivity、apo ptosis | scavenger receptor | - |
| 17555 | CD6 | NM_006725.1 | 1017 | ctgctcctggcggttcaacaact | 244926 | - | receptor_acitivity | scavenger receptor | - |
| 17556 | DMBT1 | NM_004406.1 | 3910 | cgggcgtgtagaaatttaccatg | 244955 | | receptor_acitivity | - | esophageal cancer、lung cancer、malignant brain tumors、CNS Tumours |
| 17557 | M160 | NM_174941.2 | 3601 | cagcctcgcccctttatctaaga | 245061 | - | receptor_acitivity | - | - |
| 17558 | MARCO | NM_006770.2 | 1408 | caggattgtcggcagtagtaacc | 245084 | - | receptor_acitivity | scavenger receptor | - |
| 17559 | MSR1 | NM_002445.2 | 768 | gaggagcgtgtttacaatgtatc | 245109 | - | receptor_acitivity | - | prostate cancer |
| 17560 | SRCRB4D | NM_080744.1 | 771 | ctggggcgtccacaattgctttc | 245139 | - | receptor_acitivity | - | - |
| 17561 | B2M | NM_004048.2 | 379 | ttgtcacagcccaagatagttaa | 245153 | - | see also 2730 line | | |
| 17562 | HLA-E | NM_005516.3 | 416 | acgacggcaaggattatctcacc | 245175 | - | receptor_acitivity | MHC class I receptor | - |
| 17563 | HLA-F | NM_018950.1 | 1061 | gggtgtctctcacagctaataaa | 245180 | - | receptor_acitivity | MHC class I receptor | - |
| 17564 | MR1 | NM_001531.1 | 71 | ccggacgcactctctgagatatt | 245186 | - | receptor_acitivity | - | - |
| 17565 | ULBP3 | NM_024518.1 | 387 | gtgtgaagccgatggatacatcc | 245220 | - | receptor_acitivity | - | - |
| 17566 | CD74 | NM_004355.1 | 615 | ctgacgctccaccgaaagagtca | 245226 | - | receptor_acitivity | chaperone | - |

Figure 46

| | | | | | | | | | |
|---|---|---|---|---|---|---|---|---|---|
| 17567 | HLA-DMB | NM_002118.3 | 501 | ttgcgcaatgggcttcagaattg | 245231 | - | receptor_acitivity | chaperone | - |
| 17568 | HLA-DOA | NM_002119.2 | 226 | gtgtggcgtctgcctgagtttgg | 245238 | - | receptor_acitivity | MHC class II receptor | - |
| 17569 | HLA-DOB | NM_002120.2 | 505 | caggggatatcaagatcaagtgg | 245249 | - | receptor_acitivity | MHC class II receptor | - |
| 17570 | HLA-DPA1 | NM_033554.2 | 299 | gggctggctaacattgctatatt | 245265 | - | receptor_acitivity | RNA polymerase II transcription factor、 enhancer binding | - |
| 17571 | HLA-DPB1 | NM_002121.4 | 721 | ctgcccggagtaagacattgacg | 245275 | - | receptor_acitivity | transcription factor | - |
| 17572 | HLA-DQA2 | NM_020056.1 | 244 | ccgcagagtgcactgagaaatat | 245280 | - | - | MHC class II receptor | - |
| 17573 | HLA-DQB1 | NM_002123.2 | 164 | aggatttcgtgtaccagtttaag | 245282 | - | receptor_acitivity | MHC class II receptor | diabetes |
| 17574 | HLA-DQB2 | NM_182549.1 | 181 | gtgtggccagatacatctataac | 245285 | - | receptor_acitivity | - | - |
| 17575 | HLA-DRA | NM_019111.2 | 186 | ctgaccaatcaggcgagtttatg | 245295 | - | receptor_acitivity | transcription factor | - |
| 17576 | TNFRSF1A | NM_001065.2 | 666 | aagaaccagtaccggcattattg | 245302 | - | receptor_acitivity、 apo ptosis、 signal_transdu ction、 kinase_related | tumor necrosis factor receptor、 type I | - |
| 17577 | TNFRSF19 | NM_018647.2 | 437 | acgaaacttgtcggctttcaaga | 245330 | - | receptor_acitivity、 apo ptosis、 kinase_related | - | - |
| 17578 | TNFRSF1B | NM_001066.2 | 895 | cagccttgggtctactaataata | 245358 | - | receptor_acitivity、 apo ptosis、 signal_transdu ction | tumor necrosis factor receptor | - |
| 17579 | XEDAR | NM_021783.2 | 751 | gggtccacagccccatcgaatgc | 245371 | - | receptor_acitivity、 tra nscription_regulator | tumor necrosis factor receptor | - |
| 17580 | TNFRSF14 | NM_003820.2 | 465 | aagtgcagtccaggttatcgtgt | 245373 | - | receptor_acitivity、 apo ptosis、 signal_transdu ction | NGF/TNF (6 C- domain) receptor | - |
| 17581 | FLJ30296 | NM_173495.1 | 690 | cagccgcgtatcagaacgttacc | 245401 | - | - | - | - |
| 17582 | NPC1 | NM_000271.1 | 3934 | gagcgcgaacggcttctaaattt | 245583 | - | receptor_acitivity | sterol transporter | atherosclerosis、 Niemann-Pick disease |
| 17583 | NPC1L1 | NM_013389.1 | 1222 | aagcccggagtgagaaagctttc | 245597 | - | receptor_acitivity | - | - |
| 17584 | PTCH | NM_000264.2 | 587 | aggacgagtaagtcgtgaattaa | 245632 | - | see also 207 line | | |
| 17585 | PTCH2 | NM_003738.2 | 1410 | cggcgtggatgacgtattcctgc | 245692 | - | see also 2551 line | | |

Figure 46

| | | | | | | | | | |
|---|---|---|---|---|---|---|---|---|---|
| 17586 | XPO7 | NM_015024.2 | 3233 | aggcatcgagcgaatcttctta | 245788 | - | see also 6190 line | | |
| 17587 | CD14 | NM_000591.1 | 483 | acgctcgaggacctaaagataac | 245798 | - | receptor_acitivity, apoptosis, signal_transduction | Smad3/Sp-1 heterodimer | alcoholism |
| 17588 | UNC5C | NM_003728.2 | 1213 | cggcctcgtctgcaatccaaga | 245833 | - | receptor_acitivity, signal_transduction | - | |
| 17589 | ROBO1 | NM_002941.2 | 1255 | cagcgatctgatgttggttatta | 245896 | - | see also 1975 line | | |
| 17590 | RRBP1 | NM_004587.1 | 2732 | tcggcatgtcggttacaagaaga | 246007 | - | receptor_acitivity, signal_transduction | - | |
| 17591 | AGER | NM_001136.3 | 1207 | gaggcaggcgagagtagtactgg | 246018 | - | receptor_acitivity, signal_transduction | | Alzheimer disease |
| 17592 | CD3D | NM_000732.1 | 194 | tggacccacgaggaatatatagg | 246029 | - | receptor_acitivity, signal_transduction | receptor signaling protein | leukemia, diabetes |
| 17593 | CD3E | NM_000733.1 | 439 | tcggtggccacaattgtcatagt | 246049 | - | see also 478 line | | |
| 17594 | CD3G | NM_000073.1 | 218 | atgatcggcttcctaactgaaga | 246056 | - | receptor_acitivity, apoptosis, signal_transduction | receptor signaling protein | leukemia |
| 17595 | CLDN3 | NM_001306.2 | 605 | ctgtcggccaacaccattatcc | 246079 | - | receptor_acitivity | transmembrane receptor | |
| 17596 | CLDN4 | NM_001305.3 | 910 | aagccttactcgccaagrattc | 246087 | - | receptor_acitivity | transmembrane receptor | |
| 17597 | CR2 | NM_001877.1 | 2537 | atggtagtcgcgtgattaggrgt | 246181 | - | receptor_acitivity | transmembrane receptor | |
| 17598 | DCC | NM_005215.1 | 3754 | ccggtgccaacgctagaaagtgc | 246278 | - | receptor_acitivity, apoptosis, cell_cycle | transmembrane receptor | colorectal cancer, neuroblastoma, osteosarcoma, pancreatic cancer, colorectal cancers, testicular cancer, head and neck squamous cell carcinoma, gastric cancer |
| 17599 | EDAR | NM_022336.1 | 685 | cagatatgcaggcgtcacaaaga | 246292 | - | receptor_acitivity, apoptosis, signal_transduction | transmembrane receptor | ectodermal dysplasia |
| 17600 | EVI2A | NM_014210.1 | 404 | ctgaagtagattataaaggtaat | 246311 | - | receptor_acitivity | transmembrane receptor | |
| 17601 | GPC1 | NM_002081.1 | 956 | gtgctcgagagctgtcatgaagc | 246327 | - | receptor_acitivity | - | |
| 17602 | GPC5 | NM_004466.3 | 2042 | cagtgcgactgaatctatgaca | 246404 | - | - | | |
| 17603 | GPC6 | NM_005708.2 | 766 | gagatcgcaggggaacacttaag | 246408 | - | see also 3961 line | | |
| 17604 | ICAM1 | NM_000201.1 | 1390 | gtgactgtcactcgagatcttga | 246435 | - | receptor_acitivity | Rel A homodimer | |
| 17605 | IGSF6 | NM_005849.1 | 177 | gaggccgtcaccataaagtgtac | 246442 | - | receptor_acitivity, signal_transduction | - | |

Figure 46

| 17606 | IL17RD | NM_017563.1 | 652 | ttgttacagccggacaatctagc | 246460 | - | see also 7033 line | | |
|---|---|---|---|---|---|---|---|---|---|
| 17607 | KLRA1 | NM_006611.1 | 558 | cgggtcctgttgtggagtaaact | 246503 | - | receptor_acitivity、signal_transduction | - | - |
| 17608 | LRRC21 | NM_015613.1 | 634 | atgtgactgccgactctatgacc | 246513 | - | - | - | - |
| 17609 | LTBR | NM_002342.1 | 407 | gtgccacatgtgccgagaattcc | 246528 | - | receptor_acitivity、apoptosis、signal_transduction、kinase_related | receptor | - |
| 17610 | LU | NM_005581.2 | 172 | cgggaacccacgaccattatatg | 246549 | - | receptor_acitivity、signal_transduction | transmembrane receptor | - |
| 17611 | M6PR | NM_002355.2 | 195 | aggactggactgctactactact | 246556 | - | receptor_acitivity | - | - |
| 17612 | MFRP | NM_031433.1 | 1199 | aggacgagtgcaagtttgactac | 246579 | - | receptor_acitivity | - | - |
| 17613 | NCR2 | NM_004828.2 | 723 | tcgtctggtgggggacatatgg | 246586 | - | - | - | - |
| 17614 | NGFR | NM_002507.1 | 681 | gagatccctggccgttggattac | 246590 | - | receptor_acitivity、apoptosis、signal_transduction | transmembrane receptor | - |
| 17615 | PLXN3 | NM_017514.1 | 5543 | tgggtggagaggtattatcgaga | 246618 | - | receptor_acitivity | - | - |
| 17616 | SARM1 | NM_015077.1 | 549 | tggacgcggtgctgtattggtgc | 246626 | - | see also 6225 line | | |
| 17617 | SFRP1 | NM_003012.2 | 750 | tggccccgagatgcttaagtgtga | 246639 | - | receptor_acitivity、apoptosis、signal_transduction | | - |
| 17618 | SFRP4 | NM_003014.2 | 1153 | gggcgcaccagtcgtagtaatcc | 246664 | - | see also 2018 line | | |
| 17619 | SFRP5 | NM_003015.2 | 1059 | ttgcagtcaaattcatgttctcc | 246668 | - | see also 2020 line | | |
| 17620 | SORL1 | NM_003105.3 | 3593 | gtgccggagtgacgagtacaact | 246742 | - | receptor_acitivity | transmembrane receptor | - |
| 17621 | TIRAP | NM_052887.2 | 998 | ctgagctccgattcatgtactac | 246819 | - | receptor_acitivity | - | - |
| 17622 | TLR5 | NM_003268.3 | 1244 | tagcctcgcagctaatagcttgt | 246845 | - | receptor_acitivity | transmembrane receptor | - |
| 17623 | TLR7 | NM_016562.2 | 2770 | aaggggtatcagcgtctaatatc | 246973 | - | see also 6915 line | | |
| 17624 | TLR9 | NM_017442.2 | 3421 | cagccgcaagacgctgtttgtgc | 247004 | - | receptor_acitivity | - | rheumatoid arthritis |
| 17625 | TNFRSF10C | NM_003841.2 | 621 | ttgtacgtcctgggatgatatcc | 247009 | - | receptor_acitivity、apoptosis、signal_transduction | transmembrane receptor | - |
| 17626 | TNFRSF5 | NM_001250.3 | 260 | ttgcggtgaaagcgaattcctag | 247015 | - | receptor_acitivity、apoptosis、signal_transduction、kinase_related | - | atherosclerosis、Alzheimer disease、arthritis、Kaposi's Sarcoma |

Figure 46

| | | | | | | | | | |
|---|---|---|---|---|---|---|---|---|---|
| 17627 | TNFRSF6 | NM_000043.3 | 1212 | aagcgtatgacacattgattaaa | 247043 | - | receptor_acitivity、apoptosis、signal_transduction | - | lupus erythematosus、bladder transitional cell carcinoma、leukemia、autoimmune lymphoproliferative syndrome、systemic lupus erythematosis、Alzheimer disease、arthritis |
| 17628 | TNFRSF7 | NM_001242.3 | 618 | cagcccacccacttaccttatgt | 247049 | - | receptor_acitivity、apoptosis、signal_transduction | receptor | - |
| 17629 | TNFRSF8 | NM_001243.2 | 1595 | cggaagagcgagggttaatgagc | 247062 | - | receptor_acitivity、signal_transduction | - | leukemia |
| 17630 | WSX1 | NM_004843.2 | 1574 | ctgtcggggtcccctatcgaatc | 247077 | - | receptor_acitivity、signal_transduction | - | - |
| 17631 | LOC51036 | NM_015854.1 | 132 | gagcaaacgagtgcagtcaattc | 247083 | - | - | - | - |
| 17632 | LY96 | NM_015364.2 | 253 | aggatccaaaggattattgcaca | 247095 | - | receptor_acitivity、signal_transduction | coreceptor | - |
| 17633 | PVRL1 | NM_002855.2 | 91 | caggtgaacgactccatgtatgg | 247104 | - | see also 1887 line | | |
| 17634 | PVRL2 | NM_002856.1 | 1015 | cagagcccacgggctatgactgg | 247116 | - | receptor_acitivity | coreceptor | - |
| 17635 | ZP2 | NM_003460.1 | 1081 | gagtcacccgtttctatagttac | 247145 | - | see also 2347 line | | |
| 17636 | CR1 | NM_000573.2 | 4326 | cagtcctacgatcccaattaatg | 247182 | - | receptor_acitivity | - | Alzheimer disease |
| 17637 | ITPR1 | NM_002222.1 | 7136 | gggttacaacccacgttgtttct | 247353 | - | see also 1411 line | | |
| 17638 | ITPR2 | NM_002223.1 | 6428 | cagacctgtatcgctacacatga | 247528 | - | channel、receptor_acitivity、signal_transduction | inositol-1、4、5-triphosphate receptor | - |
| 17639 | PLAUR | NM_002659.1 | 759 | aagccgttacctcgaatgcattt | 247584 | - | kinase_related、receptor_acitivity、signal_transduction | translation regulator | atherosclerosis |
| 17640 | AHSA2 | NM_152392.1 | 1980 | cagctgacccccctaaatggttg | 247609 | - | - | - | - |
| 17641 | AIPL1 | NM_014336.2 | 489 | aggttgatgccccgagtgattac | 247618 | - | receptor_acitivity | chaperone | - |
| 17642 | ANTXR1 | NM_018153.2 | 792 | aagaagtcctgcatcgaaattct | 247634 | - | receptor_acitivity | - | - |
| 17643 | BTN1A1 | NM_001732.1 | 522 | cagtatgcaagttcaagagaatg | 247657 | - | receptor_acitivity | receptor | - |
| 17644 | BTN3A2 | NM_007047.2 | 1225 | gagtcttcgtccgataccaataa | 247669 | - | receptor_acitivity | - | - |
| 17645 | BTNL3 | NM_006707.2 | 568 | acggaggtatccagttactctgc | 247677 | - | receptor_acitivity | - | - |
| 17646 | C6orf33 | NM_133367.1 | 610 | ttggcgtgagcgtttaccaatat | 247696 | - | - | - | - |
| 17647 | C6orf80 | NM_015439.2 | 395 | ctggccgtggtgcaataaggaac | 247711 | - | receptor_acitivity | - | - |
| 17648 | CD160 | NM_007053.1 | 319 | aggaaggaacgcgactaaactta | 247721 | - | receptor_acitivity、signal_transduction | receptor | - |
| 17649 | CD200R | NM_138806.2 | 553 | tggatatcacctccaagtgttag | 247754 | - | receptor_acitivity | - | - |
| 17650 | CD36 | NM_000072.1 | 795 | aagtaaagttgccataatcgaca | 247796 | - | see also 48 line | | |

Figure 46

| 17651 | CD7 | NM_006137.5 | 705 | gtgctcgtggcgggataagaatt | 247826 | - | receptor_acitivity、signal_transduction、kinase_related | receptor | - |
|---|---|---|---|---|---|---|---|---|---|
| 17652 | CD86 | NM_006889.2 | 949 | aagcggcctcgcaactcttataa | 247860 | - | receptor_acitivity、transcrition_regulator | - | rheumatoid arthritis |
| 17653 | CNGB1 | NM_001297.1 | 2947 | ctgtgaccggcagatgatctttg | 247887 | - | channel | potassium channel | - |
| 17654 | CNGB3 | NM_019098.2 | 1475 | aagcgagttcggacttggtatga | 247950 | - | see also 7704 line | | |
| 17655 | CNTNAP1 | NM_003632.1 | 2545 | gcgctgctatggcgatcgaaatt | 248005 | - | receptor_acitivity、signal_transduction | receptor | - |
| 17656 | CRL3 | NM_139017.3 | 1256 | ctggacgatccagcaagataaat | 248068 | - | receptor_acitivity、kinase_related、transcription_regulator、apoptosis、signal_transduction | - | - |
| 17657 | CRLF3 | NM_015986.2 | 656 | aggctccagtttcgtaaatgtac | 248108 | - | see also 6596 line | | |
| 17658 | CRSP3 | NM_004830.2 | 3390 | tggcagactagtcgatacgatgg | 248233 | - | receptor_acitivity、transcription_regulator | - | - |
| 17659 | CRSP6 | NM_004268.3 | 1579 | cagcttagcaagccgaattgagg | 248302 | - | see also 2866 line | | |
| 17660 | DKFZP434N1817 | NM_032137.2 | 1234 | gagttgtgtcgccacatagaagc | 248322 | - | - | - | - |
| 17661 | DNAJ | NM_032364.4 | 1283 | cagcggctggtaagaatagttca | 248386 | - | - | - | - |
| 17662 | EBAG9 | NM_004215.3 | 983 | aagcacaacggctaatgaagaag | 248424 | - | see also 2822 line | | |
| 17663 | EPS8 | NM_004447.3 | 304 | atgaatggctacggatcatcacc | 248427 | - | see also 2981 line | | |
| 17664 | EPS8L1 | NM_017729.2 | 1164 | atgggtcctgtgtaattatgact | 248504 | - | kinase_related、receptor_acitivity | - | - |
| 17665 | EPS8L2 | NM_022772.2 | 1623 | gtgagccgacagtccataagaaa | 248517 | - | - | - | - |
| 17666 | EPS8L3 | NM_024526.2 | 444 | atgtggcgctcaacacatgttcc | 248520 | - | receptor_acitivity | - | - |
| 17667 | FCRH1 | NM_052938.3 | 1149 | cagggcagctacagcctatatat | 248548 | - | receptor_acitivity | - | - |
| 17668 | FEM1B | NM_015322.2 | 805 | gtggtacgcttgctcttagaaca | 248560 | - | see also 6354 line | | |
| 17669 | FKSG87 | NM_032029.1 | 643 | gagctcaccatgagatcctatgg | 248611 | - | receptor_acitivity | - | - |
| 17670 | FLJ10587 | NM_018149.2 | 30 | gagcttgcgagaccttctaatgg | 248622 | - | see also 7284 line | | |
| 17671 | FLVCR | NM_014053.1 | 1843 | aagcagtcagaatcagcaatttg | 248721 | - | receptor_acitivity | - | - |
| 17672 | FRAG1 | NM_014489.1 | 917 | tcggcacaacatgtattgtgagg | 248730 | - | receptor_acitivity | - | - |
| 17673 | FREB | NM_032738.2 | 803 | aagatcactccgggtcatactgg | 248739 | - | receptor_acitivity | - | - |
| 17674 | GABARAPL1 | NM_031412.1 | 432 | gtgccctctgaccttactgttgg | 248744 | - | - | - | - |
| 17675 | GAC1 | NM_006338.1 | 811 | cagcctacaggaactctatctca | 248747 | - | receptor_acitivity、signal_transduction | receptor | CNS Tumours |

Figure 46

| | | | | | | | | | |
|---|---|---|---|---|---|---|---|---|---|
| 17676 | GFRA1 | NM_005264.2 | 1289 | aggactcctgcaagacgaattac | 248766 | - | see also 3701 line | | |
| 17677 | GFRA4 | NM_022139.2 | 120 | gcgtttgcgctccgagtatgtgg | 248782 | - | receptor_acitivity | | |
| 17678 | GOSR1 | NM_004871.1 | 143 | atggaagacgcgacaggtatagt | 248787 | - | | v-SNARE | |
| 17679 | GPA33 | NM_005814.1 | 952 | cagacacatcggttactacacatc | 248825 | - | receptor_acitivity | | |
| 17680 | GYPB | NM_002100.2 | 258 | ttgtgtgtgatgctgtattat | 248827 | - | | Smad3/Sp-1 heterodimer | |
| 17681 | HAVCR1 | NM_012206.1 | 898 | ctgacggccaataccactaaagg | 248844 | - | | | |
| 17682 | HAVCR2 | NM_032782.2 | 668 | atgagttacggactctagattg | 248867 | - | | | |
| 17683 | IL23R | NM_144701.2 | 1484 | tcgctattcgacaatactacagt | 248920 | - | receptor_acitivity | | |
| 17684 | IMPG1 | NM_001563.2 | 1799 | cagtatatcaccacctagttctat | 248964 | - | receptor_acitivity | | |
| 17685 | ITGA2B | NM_000419.2 | 1045 | tggttgggcgctccactgtatatg | 249009 | - | receptor_acitivity, signal_transduction | specific RNA polymerase II transcription factor | Glantzmann thrombasthenia |
| 17686 | ITGA3 | NM_002204.1 | 767 | tgggacttatctgagtatagtta | 249026 | - | receptor_acitivity, signal_transduction | | |
| 17687 | ITGA4 | NM_000885.2 | 1669 | gagtgcccctgatttacgaaca | 249052 | - | see also 576 line | | |
| 17688 | ITGA5 | NM_002205.1 | 256 | tgggagcacccaaggctaatacc | 249142 | - | receptor_acitivity, signal_transduction | Proto-oncogene C-JUN | |
| 17689 | ITGA6 | NM_000210.1 | 425 | atgcacggatcgagtttgata | 249179 | - | receptor_acitivity, signal_transduction | cell adhesion receptor | epidermolysis bullosa |
| 17690 | ITGA7 | NM_002206.1 | 3390 | cagtaccatcgcggtgaagattcc | 249297 | - | see also 1407 line | | |
| 17691 | ITGA9 | NM_002207.1 | 867 | aggcatcggcaaggttatattt | 249310 | - | receptor_acitivity, signal_transduction | cell adhesion receptor | |
| 17692 | ITGB2 | NM_000211.1 | 1269 | ctgtgatgggtgcagatcaatg | 249350 | - | receptor_acitivity, kinase_related, apoptosis, signal_transduction | hematopoietic-associated factor 1A complex | leukocyte adhesion deficiency, juvenile rheumatoid arthritis |
| 17693 | ITGB3 | NM_000212.1 | 268 | gagtgagcccgagtactagagg | 249362 | - | see also 161 line | | |
| 17694 | ITGB4 | NM_000213.2 | 5307 | gcgagaacgtgcctacaagttc | 249424 | - | receptor_acitivity, signal_transduction | AP-1(JunB/Fra-1 heterodimers) | epidermolysis bullosa simplex, epidermolysis bullosa |
| 17695 | ITGB5 | NM_002213.3 | 603 | aggctgggacgtcattcagatga | 249433 | - | receptor_acitivity, signal_transduction | cell adhesion receptor | |
| 17696 | ITGB6 | NM_000888.3 | 594 | ttgccaacccttgcagtagatt | 249476 | - | see also 578 line | | |
| 17697 | ITGB7 | NM_000889.1 | 1358 | ggggtccacatttcttacgaatc | 249532 | - | receptor_acitivity, signal_transduction | cell adhesion receptor | |
| 17698 | ITGB8 | NM_002214.1 | 1264 | cagtttcaccatacattagcatc | 249569 | - | receptor_acitivity, signal_transduction | cell adhesion receptor | |

Figure 46

| | | | | | | | | | |
|---|---|---|---|---|---|---|---|---|---|
| 17699 | ITPR3 | NM_002224.1 | 2121 | gtggactgacaagaataacgagc | 249635 | - | receptor_acitivity、cha nnel | inositol-1、4、5- triphosphate receptor | - |
| 17700 | KDELR3 | NM_006855.2 | 581 | gagaggctgagaccataactact | 249678 | - | receptor_acitivity | - | - |
| 17701 | KIAA1985 | NM_024577.2 | 2800 | ctgcaggctgtacgactctattg | 249716 | - | - | - | - |
| 17702 | LIMR | NM_018113.1 | 1079 | ctgacccgcaggatctgtaatcc | 249744 | - | see also 7259 line | | |
| 17703 | LOC143458 | NM_174902.2 | 522 | cagagaaccaacttgtgtattac | 249756 | - | receptor_acitivity | - | - |
| 17704 | LOC158435 | NM_138497.1 | 597 | gggaaggtggacccacaaactcg | 249761 | - | - | - | - |
| 17705 | LRP3 | NM_002333.1 | 457 | gggcgaccgtggtgacatgatta | 249765 | - | receptor_acitivity | - | - |
| 17706 | LRP5 | NM_002335.1 | 527 | ccgctcacgggtacatgtactgg | 249777 | - | receptor_acitivity、sig nal_transduction | - | atherosclerosis、diabetes |
| 17707 | MAML2 | NM_032427.1 | 2794 | cagcggccagtcgaaagtaatgg | 249826 | - | receptor_acitivity | - | - |
| 17708 | MCC | NM_002387.1 | 1888 | ttgcgacaccgagttcactaaag | 249881 | - | receptor_acitivity、sig nal_transduction、cell _cycle | receptor | colorectal cancer、mutated in colorectal cancers、colorectal cancers、squamous cell cancer |
| 17709 | MCP | NM_002389.3 | 326 | gtgaacgagtagattataagtgt | 249899 | - | receptor_acitivity | - | - |
| 17710 | MED6 | NM_005466.1 | 127 | gtggtagtgtcctggattacttt | 249942 | - | see also 3809 line | | |
| 17711 | MGC33974 | NM_153350.2 | 1421 | gtgctgccaggtgcaagacttcg | 249960 | - | - | - | - |
| 17712 | MGC4766 | NM_031451.3 | 791 | ttgccaggaaaccatactaataa | 249972 | - | - | - | - |
| 17713 | MIP-T3 | NM_015650.2 | 1430 | tagtctgcggtgtgagaatattc | 250000 | - | receptor_acitivity | - | - |
| 17714 | MMD | NM_012329.1 | 375 | gtgtgatagaatggttatctatt | 250021 | - | see also 5280 line | | |
| 17715 | MPRA | NM_178422.2 | 1102 | ctggtacagcgcaaacttgatca | 250054 | - | receptor_acitivity、gpc r、signal_transduction | - | - |
| 17716 | MS4A4A | NM_024021.2 | 213 | atggctgtcatacattcacatct | 250055 | - | - | - | - |
| 17717 | MS4A5 | NM_023945.2 | 415 | cagaaactctgataatattgagc | 250078 | - | receptor_acitivity、sig nal_transduction | - | - |
| 17718 | MS4A6A | NM_022349.2 | 645 | ctgtcaaacaggccaccttaaat | 250088 | - | receptor_acitivity、sig nal_transduction | - | - |
| 17719 | MS4A7 | NM_021201.3 | 570 | ctgaggactgcctctcaacattg | 250106 | - | receptor_acitivity、sig nal_transduction | - | - |
| 17720 | MS4A8B | NM_031457.1 | 252 | gtggcaccccacaatggttatcc | 250117 | - | receptor_acitivity、sig nal_transduction | - | - |
| 17721 | MTVR1 | NM_152832.1 | 639 | gagtcaatccaggactacaaaca | 250137 | - | receptor_acitivity | - | - |

Figure 46

| | | | | | | | | | |
|---|---|---|---|---|---|---|---|---|---|
| 17722 | NCR1 | NM_004829.3 | 319 | gggcaatacagctgcatctatcg | 250142 | - | receptor_acitivity、 signal_transduction | - | - |
| 17723 | NCR3 | NM_147130.1 | 728 | ccgtgggcagcaccgtctattac | 250151 | - | receptor_acitivity | - | - |
| 17724 | NECL1 | NM_021189.2 | 465 | gagcccttcgagataatcgaatt | 250153 | - | receptor_acitivity | - | - |
| 17725 | NGRH2 | NM_178568.1 | 243 | gtggatctactcgaacaacatca | 250169 | - | receptor_acitivity | - | - |
| 17726 | NISCH | NM_007184.1 | 651 | ctgccgttcgacctatcaatatt | 250184 | - | - | - | - |
| 17727 | OGFRL1 | NM_024576.2 | 1021 | gcggttcgcccagaaacactaca | 250245 | - | - | - | - |
| 17728 | OSCAR | NM_130771.1 | 343 | gggtcaacgtgaccttgagatgc | 250260 | - | receptor_acitivity | - | - |
| 17729 | PILRA | NM_013439.2 | 1003 | atgacggcatcgtctatgcttcc | 250269 | - | receptor_acitivity、 kinase_related | - | - |
| 17730 | PKHD1 | NM_138694.2 | 2085 | gggctatcggctagatcagtata | 250301 | - | receptor_acitivity | - | - |
| 17731 | PLXDC1 | NM_020405.3 | 338 | gaggacaaccacagctattatgt | 250546 | - | see also 7865 line | | |
| 17732 | PLXDC2 | NM_032812.7 | 1197 | aagtcgtacatcgaatgctaaca | 250572 | - | receptor_acitivity | - | - |
| 17733 | PLXNB3 | NM_005393.1 | 297 | tggccgaggcacactctatgtcg | 250597 | - | receptor_acitivity | - | - |
| 17734 | PLXND1 | NM_015103.1 | 5750 | tggccgaggagtcgaggaaatac | 250642 | - | receptor_acitivity | - | - |
| 17735 | PORIMIN | NM_052932.1 | 747 | tcggtatcgaaccatagatgaac | 250659 | - | receptor_acitivity | - | - |
| 17736 | PPFIA1 | NM_003626.2 | 461 | aggagttcgcagcacttactaaa | 250666 | - | - | - | - |
| 17737 | PROCR | NM_006404.3 | 825 | gcgtcctggtgggcagtttcatc | 250723 | - | receptor_acitivity | receptor | - |
| 17738 | PRV1 | NM_020406.1 | 152 | ccggcaatggacccctaagaaca | 250724 | - | receptor_acitivity | - | - |
| 17739 | PSK-1 | NM_012410.1 | 2613 | tggggatacgcgggagtatgaag | 250752 | - | receptor_acitivity | - | - |
| 17740 | PVR | NM_006505.2 | 1248 | ggggatcgggatttatttctatt | 250790 | - | receptor_acitivity | receptor | poliomyelitis infection |
| 17741 | RAMP2 | NM_005854.1 | 230 | agggggacggtgaagaactatg | 250794 | - | receptor_acitivity、 signal_transduction、 gpcr | receptor | - |
| 17742 | RAMP3 | NM_005856.1 | 217 | tcgtgtactatgagagtttcacc | 250803 | - | receptor_acitivity、 signal_transduction、 gpcr | receptor | - |
| 17743 | RAP80 | NM_016290.2 | 1971 | gtgaaggccgactccttagtttc | 250836 | - | - | - | - |
| 17744 | RAPSN | NM_005055.3 | 662 | ctgcgctatgcccacaacaatga | 250843 | - | - | - | - |
| 17745 | RHBDF1 | NM_022450.2 | 2400 | ctgccgtggattgacaactttgc | 250861 | - | - | - | - |
| 17746 | RIF1 | NM_018372.2 | 2189 | atgcttataacgcaatcataaat | 250901 | - | - | - | - |
| 17747 | RILP | NM_031430.1 | 1021 | caggagcggaatgaactcaaagc | 250912 | - | - | - | - |
| 17748 | RNF139 | NM_007218.3 | 2017 | tcgtattacaccgtgtaatcatt | 250978 | - | see also 5026 line | | |
| 17749 | RTN4R | NM_023004.4 | 1635 | tggcccacggcacatcaatgact | 250995 | - | receptor_acitivity | - | - |
| 17750 | RYR1 | NM_000540.1 | 2244 | gggtcggcgatgacctctattcc | 251016 | - | receptor_acitivity、 channel | receptor | central core disease of muscle、 malignant hyperthermia |
| 17751 | SCARB1 | NM_005505.3 | 906 | ctgccgatccatgaagctaatgt | 251126 | - | receptor_acitivity | - | atherosclerosis |

Figure 46

| | | | | | | | | |
|---|---|---|---|---|---|---|---|---|
| 17752 | SCARB2 | NM_005506.2 | 1601 | cggcgagtcgactgaagtctatg | 251177 | - | see also 3847 line | |
| 17753 | SCARF2 | NM_153334.3 | 512 | acgtgacaggccagtgacttgt | 251183 | - | receptor_acitivity | receptor |
| 17754 | SDFR1 | NM_012428.1 | 709 | ctgagtgccactcgtaagaatgc | 251205 | - | receptor_acitivity | |
| 17755 | SEMA4B | NM_020210.2 | 2340 | gttggcagtgtaccgtcattatc | 251239 | - | receptor_acitivity | |
| 17756 | SEMA4C | NM_017789.3 | 758 | tggctcaacgaacctcacttgt | 251247 | - | receptor_acitivity | |
| 17757 | SEMA4D | NM_006378.2 | 1184 | ttggctgaagagcctagtttcg | 251272 | - | receptor_acitivity, apoptosis | receptor |
| 17758 | SEMA4F | NM_004263.2 | 744 | cttgattcggacagatacctgc | 251304 | - | see also 2862 line | |
| 17759 | SEMA4G | NM_017893.2 | 718 | cagacggagtgcttaaccatgt | 251328 | - | receptor_acitivity | receptor |
| 17760 | SEMA5A | NM_003966.1 | 2813 | gagcaacgattccgatacacatg | 251387 | - | receptor_acitivity | receptor |
| 17761 | SLAMF6 | NM_052931.3 | 815 | gagattcctatctttgtctact | 251439 | - | receptor_acitivity | |
| 17762 | SLAMF7 | NM_021181.3 | 212 | aaggggggcactatcatagtgacc | 251456 | - | receptor_acitivity | |
| 17763 | SLC1A5 | NM_005628.1 | 1988 | ctgtaccgtcctcaatgtagaag | 251483 | - | receptor_acitivity | sodium:dicarboxylate/tricarboxylate cotransporter |
| 17764 | SLC20A1 | NM_005415.3 | 1593 | aaggattccggcctgtacaaaga | 251512 | - | see also 3781 line | |
| 17765 | SLC20A2 | NM_006749.3 | 547 | gtgttactaggcgccaaagtagg | 251539 | - | receptor_acitivity | |
| 17766 | SPAP1 | NM_030764.2 | 1206 | caggacctgatggctatagaaga | 251588 | - | - | |
| 17767 | TGFBRAP1 | NM_004257.3 | 847 | tagcgctgatgacgaattcatc | 251602 | - | receptor_acitivity, signal transduction | |
| 17768 | TIP47 | NM_005817.2 | 543 | gagcgcggtggacaagacacaaagt | 251632 | - | receptor_acitivity | |
| 17769 | TNFRSF10D | NM_003840.2 | 542 | atgtgccggacgtgtagaacagg | 251636 | - | receptor_acitivity, apoptosis | transmembrane receptor |
| 17770 | TNFRSF11A | NM_003839.2 | 661 | atgtttacttgcccgtttaata | 251656 | - | receptor_acitivity, signal transduction | receptor |
| 17771 | TNFRSF13B | NM_012452.2 | 89 | tggacggggtggctatgagatc | 251670 | - | receptor_acitivity, signal transduction | receptor |
| 17772 | TNFRSF17 | NM_001192.2 | 666 | gaggaaggcgcaaccattcttgt | 251687 | - | receptor_acitivity, signal transduction | receptor |
| 17773 | TNFRSF19L | NM_032871.3 | 1272 | aggttccgcgtggctcgaattcc | 251693 | - | receptor_acitivity | |
| 17774 | TNFRSF21 | NM_014452.3 | 586 | tggcacataccgccatgttgacc | 251699 | - | - | |
| 17775 | TNFRSF9 | NM_001561.4 | 671 | aggacactctcgcgagatcatct | 251738 | - | receptor_acitivity, apoptosis | receptor |
| 17776 | TNPO3 | NM_012470.1 | 1706 | tagcccgattacctttggataag | 251803 | - | receptor_acitivity | |
| 17777 | TRAP240 | NM_005121.1 | 2532 | ctgaccccgttatcttgcataag | 251891 | - | receptor_acitivity, transcription_regulator, signal_transduction | |

722

Figure 46

| | | | | | | | | | | |
|---|---|---|---|---|---|---|---|---|---|---|
| 17778 | TREM1 | NM_018643.2 | 184 | atgtgactacacgctagagaagt | 252010 | - | receptor_acitivity | | - | |
| 17779 | TREM2 | NM_018965.1 | 282 | tggtcagcacgcacaacttgtgg | 252025 | - | receptor_acitivity | | - | |
| 17780 | TREM5 | NM_174892.1 | 202 | aggggtccctgacggttcaatgc | 252030 | - | receptor_acitivity | | - | |
| 17781 | TRIP3 | NM_004773.1 | 454 | ttgcagactgctgtttaggaatt | 252049 | - | transcription_regulator | ligand-dependent thyroid hormone receptor interactor | - | |
| 17782 | TRPC4 | NM_016179.1 | 1500 | tggcggacttcaggactacatcc | 252091 | - | see also 6708 line | | - | |
| 17783 | TRPC7 | NM_020389.1 | 927 | ccggatcaaactcgccattaaat | 252167 | - | see also 7861 line | | - | |
| 17784 | TRPM8 | NM_024080.3 | 2796 | tggatgtaccacgtatgacttt | 252256 | - | see also 8586 line | | - | |
| 17785 | TRPV2 | NM_016113.3 | 509 | cggaaattcgccctcagataag | 252267 | - | - | | - | |
| 17786 | TRPV3 | NM_145068.2 | 1197 | gggactcacgaggcaacaacatc | 252297 | - | see also 9774 line | | - | |
| 17787 | TRPV4 | NM_021625.3 | 826 | ttgagcgtcgtgcaaacactac | 252322 | - | channel | | - | |
| 17788 | TRPV5 | NM_019841.3 | 1860 | ttgcctccgcgttctatatcatt | 252340 | - | receptor_acitivity, channel | | - | |
| 17789 | UNC13B | NM_006377.2 | 3416 | ttgagcagttcgtgctacaatgg | 252411 | - | see also 4469 line | | - | |
| 17790 | UNC5B | NM_170744.2 | 2625 | agggagccgaaaccgctaatgttc | 252445 | - | receptor_acitivity, signal_transduction | | - | |
| 17791 | WDR31 | NM_145241.1 | 1124 | ttgctacctcatcacatgattgc | 252467 | - | - | | - | |
| 17792 | ZYX | NM_003461.3 | 980 | tggatctgggtcacaaccaaatc | 252483 | - | signal_transduction | | - | |
| 17793 | APS | NM_020979.1 | 960 | tagagaatggagccgaatacatc | 252491 | - | signal_transduction | | - | |
| 17794 | ARHGAP4 | NM_001666.1 | 1076 | tggctgagatctgcgttgaaatg | 252497 | - | signal_transduction | SH3/SH2 adaptor protein | - | |
| 17795 | ARHGAP6 | NM_001174.2 | 2835 | atgtcgtggactatttactcaga | 252523 | - | see also 764 line | | - | |
| 17796 | BLNK | NM_013314.2 | 521 | gcgagtatatagacaatcgatca | 252560 | - | see also 5395 line | | - | |
| 17797 | CRK | NM_005206.2 | 430 | tgggacactacaacgttgataga | 252585 | - | see also 3656 line | | - | |
| 17798 | GRAP2 | NM_004810.2 | 589 | gagacaacaagggtaattacttt | 252602 | - | signal_transduction | SH3/SH2 adaptor protein | - | |
| 17799 | GRB10 | NM_005311.2 | 793 | cggcccctcgttcgtagtaagg | 252609 | - | | SH3/SH2 adaptor protein | - | |
| 17800 | GRB14 | NM_004490.1 | 1436 | gagcaccgactaactatggattc | 252663 | - | | SH3/SH2 adaptor protein | - | |
| 17801 | GRB7 | NM_005310.1 | 935 | ggggttcaggacgaagctttgg | 252697 | - | signal_transduction | SH3/SH2 adaptor protein | - | |
| 17802 | LASP1 | NM_006148.1 | 125 | aggtgaactgtctgataagttc | 252708 | - | | SH3/SH2 adaptor protein | - | breast cancer |
| 17803 | RUSC1 | NM_014328.1 | 187 | ccgtcagcgtctccgttgataaa | 252719 | - | - | | - | |
| 17804 | SCAP2 | NM_003930.3 | 556 | tagcctcagaacgatatgataaa | 252739 | - | see also 2689 line | | - | |

Figure 46

| 17805 | SH2D1A | NM_002351.1 | 432 | gtgctgtatcacggttacattta | 252765 | - | | SH3/SH2 adaptor protein | Epstein-Barr virus infection、lymphoproliferative Duncan's disease、infectious mononucleosis、lymphoproliferative syndrome (Duncan's disease) |
|---|---|---|---|---|---|---|---|---|---|
| 17806 | SH2D3C | NM_005489.1 | 114 | aggctggcagcgactatgtgaag | 252780 | - | signal_transduction | - | - |
| 17807 | SH3BGR | NM_007341.1 | 238 | ctgggtccatagcgattaggaag | 252788 | - | | SH3/SH2 adaptor protein | |
| 17808 | SH3BGRL | NM_003022.1 | 110 | ctggctctacagcgattaagaag | 252796 | - | see also 2021 line | | |
| 17809 | SH3BP2 | NM_003023.2 | 1762 | tgggacgaaacctctaacaaagt | 252814 | - | | SH3/SH2 adaptor protein | cherubism |
| 17810 | SH3BP5 | NM_004844.1 | 153 | cagacgggagactgaacttgagg | 252821 | - | signal_transduction | SH3/SH2 adaptor protein | - |
| 17811 | SHB | NM_003028.1 | 1708 | gggctttaagcctatcaaacatg | 252842 | - | - | - | - |
| 17812 | SLA | NM_006748.1 | 266 | atgtgtggccagagtttaccatg | 252856 | - | - | - | - |
| 17813 | SNX9 | NM_016224.3 | 1642 | tggaatgtaatcacgagtataaa | 252895 | - | see also 6729 line | | |
| 17814 | STAM | NM_003473.2 | 1339 | atgaacgaagatccgatgtattc | 252935 | - | see also 2349 line | | |
| 17815 | TP53BP2 | NM_005426.1 | 1745 | aagtgcgtccgttctcaatgttt | 252963 | - | apoptosis、signal_transduction、cell_cycle | SH3/SH2 adaptor protein | - |
| 17816 | PAG | NM_018440.2 | 955 | cagacacgaccagtgaaactaac | 253016 | - | kinase_related、signal_transduction | - | - |
| 17817 | SHC1 | NM_003029.3 | 521 | tggccgcagactgcaaacagatc | 253029 | - | see also 2031 line | | |
| 17818 | TRIM | NM_016388.1 | 467 | atgtgctacgcctcacttgatca | 253059 | - | kinase_related、signal_transduction | - | - |
| 17819 | AKAP12 | NM_005100.2 | 3072 | gggcgacacggtcgttagtgagg | 253106 | - | kinase_related、signal_transduction、gpcr | - | - |
| 17820 | AKAP3 | NM_006422.2 | 1053 | atgactttacggcttctgttagt | 253160 | - | see also 4498 line | | |
| 17821 | AKAP4 | NM_003886.2 | 2192 | aagcaagcgaacgggcaatttat | 253244 | - | kinase_related、signal_transduction | - | - |
| 17822 | AKAP6 | NM_004274.3 | 374 | gggtccgagacctaacctattca | 253261 | - | kinase_related | protein kinase A anchor protein | - |
| 17823 | AKAP7 | NM_004842.2 | 271 | gagcccgatgacgctgaactagt | 253435 | - | kinase_related | - | - |
| 17824 | PDCL | NM_005388.2 | 1012 | ctgacatctgtgcgtaactctgc | 253469 | - | see also 3759 line | | |
| 17825 | CD19 | NM_001770.3 | 805 | aagacgctggaaagtattattgt | 253480 | - | signal_transduction | DNA binding | multiple myeloma |
| 17826 | FLRT2 | NM_013231.2 | 1176 | gggatggccgtcagggaattaaa | 253520 | - | | receptor signaling protein | - |
| 17827 | MADH1 | NM_005900.1 | 1793 | tgggttcacctcataatcctatt | 253568 | - | see also 4110 line | | |
| 17828 | MADH5 | NM_005903.4 | 605 | aaggcttacccatgttatatat | 253575 | - | see also 4113 line | | |

EP 1 752 536 A1

Figure 46

| | | | | | | | | | |
|---|---|---|---|---|---|---|---|---|---|
| 17829 | MS4A1 | NM_021950.2 | 882 | gtggttgggctaactgaaacatc | 253630 | - | receptor_acitivity、sig nal_transduction | receptor signaling protein | leukemia |
| 17830 | NSMAF | NM_003580.2 | 988 | gtgttccgacatctacctaaagt | 253655 | - | see also 2376 line | | |
| 17831 | RGS14 | NM_006480.3 | 1299 | gagcgcgtggtacgaatctcagc | 253781 | - | signal_transduction、g pcr | GTPase activator | - |
| 17832 | SIVA | NM_006427.2 | 511 | cagtgacatgtacgagaaagtgc | 253785 | - | apoptosis | - | - |
| 17833 | TBL3 | NM_006453.2 | 1298 | gcgtccgtatctggagaatgaac | 253795 | - | see also 4524 line | | |
| 17834 | TIAM1 | NM_003253.1 | 5196 | tgggttaggcgtgaagactttgc | 253874 | | - | guanyl-nucleotide release factor | non-hodgkin lymphoma |
| 17835 | FST | NM_006350.2 | 706 | ctgggcagatctattggattagc | 253878 | - | - | - | - |
| 17836 | ARHGEF1 | NM_004706.3 | 2258 | gaggtggccaccgatcacaaagc | 253892 | - | cell_cycle、signal_tran sduction | - | |
| 17837 | ARHGEF11 | NM_014784.2 | 1927 | aagactatgacccgggttatttc | 253909 | - | signal_transduction、tr anscription_regulator 、gpcr | - | |
| 17838 | ARHGEF12 | NM_015313.1 | 210 | ttgttcagcgttgcgtaatcatc | 253957 | - | see also 6323 line | | |
| 17839 | AXIN1 | NM_003502.2 | 882 | tggatacctgccgaccttaaatg | 254087 | - | apoptosis、signal_tran sduction、cell_cycle | - | hepatocellular carcinoma |
| 17840 | AXIN2 | NM_004655.1 | 1720 | ctgggggcagcgagtattactgc | 254118 | - | signal_transduction | signal transducer | colorectal cancer、colorectal cancers |
| 17841 | BCAR1 | NM_014567.1 | 775 | ggggcagggctatgtatacgagg | 254126 | - | - | signal transducer | breast cancer |
| 17842 | BSG | NM_001728.2 | 1231 | acgtccgccagaggaactcttcc | 254134 | - | signal_transduction | signal transducer | - |
| 17843 | CLTCL1 | NM_001835.1 | 3531 | aagggccgtgagtcctatataga | 254204 | - | - | - | Di George syndrome |
| 17844 | DIXDC1 | NM_033425.1 | 1700 | aggaaatcaccggtatcacttca | 254244 | - | - | - | - |
| 17845 | DKFZP434I 092 | NM_015668.1 | 3313 | aaggagttaggaccatatgtatt | 254347 | - | - | - | - |
| 17846 | DKKL1- pending | NM_014419.1 | 458 | ggggagcttcgagggtgatttga | 254365 | - | - | signal transducer | - |
| 17847 | DRCTNNB 1A | NM_032581.2 | 1310 | cagccagtcgggtttatcaaaca | 254405 | - | see also 9252 line | | |
| 17848 | DVL1 | NM_004421.2 | 2024 | ggggaacccctgcgagttcttcg | 254432 | - | signal_transduction | - | schizophrenia |
| 17849 | DVL2 | NM_004422.1 | 1539 | ctgtgagagctacctagtcaacc | 254437 | - | signal_transduction | signal transducer | - |
| 17850 | GASZ | NM_130768.1 | 571 | atggttacactgctttaacgtgg | 254463 | - | - | - | - |
| 17851 | KCNH7 | NM_033272.2 | 759 | gtggtcatcgattcatctaaaca | 254515 | - | see also 9392 line | | |
| 17852 | LOC139135 | NM_173493.1 | 351 | gtgacgctacagttattagatgg | 254617 | - | signal_transduction | - | - |
| 17853 | LY86 | NM_004271.1 | 150 | cagagttgcgatccattacaaga | 254657 | - | apoptosis、signal_tran sduction | signal transducer | - |

Figure 46

| No. | Symbol | Accession | # | Sequence | ID | — | Annotation | Category | Disease/phenotype |
|---|---|---|---|---|---|---|---|---|---|
| 17854 | NPAS3 | NM_022123.1 | 973 | atgttcgtcactcgagtaaat | 254683 | - | see also 8284 line | | |
| 17855 | NXF | NM_178864.2 | 1088 | gggaccattactgccaataact | 254714 | - | see also 10227 line | | |
| 17856 | PDCD1 | NM_005018.1 | 125 | gcggccaggatgttcttagact | 254729 | - | apoptosis | signal transducer | systemic lupus erythematosis |
| 17857 | PER1 | NM_002616.1 | 3304 | cagtgacctgctcgaacttctgc | 254755 | - | see also 1691 line | | |
| 17858 | PER2 | NM_003894.3 | 829 | tgggcgttccacagtttcacc | 254764 | - | signal_transduction, transcription_regulator | | advanced sleep phase syndrome |
| 17859 | PER3 | NM_016831.1 | 1084 | tggaacatcgatcctaagctacc | 254815 | - | see also 6970 line | | |
| 17860 | PLEKHB1 | NM_021200.1 | 1027 | tggcgtgacgcacgtgatagtgc | 254872 | - | - | signal transducer | |
| 17861 | PPP1R12A | NM_002480.1 | 2299 | cagcgttatggccagtatcaac | 254911 | - | - | signal transducer | |
| 17862 | PWP2H | NM_005049.1 | 121 | cagtccgtgggcaaatagagtca | 254939 | - | signal_transduction | | |
| 17863 | RGS13 | NM_002927.3 | 444 | gagcacagtgacgagaatattca | 254974 | - | see also 1970 line | | |
| 17864 | RGS17 | NM_012419.3 | 545 | aaggctaggatgatatatgaaga | 254988 | - | signal_transduction | signal transducer | |
| 17865 | RGS18 | NM_130782.1 | 805 | aggagacgatcacgctcatttac | 254999 | - | signal_transduction | signal transducer | |
| 17866 | RGS5 | NM_003617.1 | 452 | aggctcctaaagagtgaatatt | 255022 | - | signal_transduction, gpcr | GTPase activator | |
| 17867 | RGS8 | NM_033345.1 | 211 | aagtcagactcgtgtagtgattt | 255025 | - | signal_transduction | signal transducer | |
| 17868 | RGSL1 | NM_181572.2 | 1049 | caggtcaaccgggcatataatga | 255068 | - | - | | |
| 17869 | RGSL2 | NM_032267.1 | 765 | tcgcttatacagcgttgctcaca | 255107 | - | - | | |
| 17870 | RTN1 | NM_021136.1 | 589 | tagagtcgtggcttatttagt | 255126 | - | see also 8098 line | | |
| 17871 | RTN2 | NM_005619.2 | 1000 | tagcacggaccaattagaattca | 255157 | - | signal_transduction | signal transducer | |
| 17872 | SH3BP4 | NM_014521.1 | 692 | gagtgacagccggtatgattgata | 255178 | - | cell_cycle | | |
| 17873 | SHC3 | NM_016848.2 | 1212 | gagctccggtttaagcaatattt | 255214 | - | - | | |
| 17874 | SNX13 | NM_015132.2 | 2618 | gagctacatatggcgatactatt | 255298 | - | - | signal transducer | |
| 17875 | SNX25 | NM_031953.2 | 1330 | gagaccctaaagatgaggtatta | 255331 | - | - | signal transducer | |
| 17876 | TM4SF3 | NM_004616.2 | 474 | caggtggcgacaggtatcctagg | 255382 | - | - | signal transducer | |
| 17877 | WNT1 | NM_005430.2 | 812 | caggccgtacgaccgtattctcc | 255405 | - | - | signal transducer | |
| 17878 | WNT10A | NM_025216.2 | 1170 | gagacatccacgcgagaaatgagg | 255412 | - | - | signal transducer | |
| 17879 | WNT10B | NM_003394.2 | 677 | aagcgcggtttccgagaaagtgc | 255415 | - | see also 2293 line | | |
| 17880 | WNT11 | NM_004626.2 | 1038 | aagcgacagcgtcgcgacccttatgt | 255421 | - | see also 3143 line | | |
| 17881 | WNT16 | NM_016087.2 | 651 | aggctgtcgccaagttgatgtca | 255423 | - | - | signal transducer | |
| 17882 | WNT2 | NM_003391.1 | 405 | caggttgatgtcggataatgtgc | 255433 | - | - | signal transducer | |
| 17883 | WNT2B | NM_004185.2 | 1183 | gtggaagtccatacttgcaaage | 255470 | - | see also 2806 line | | |
| 17884 | WNT3 | NM_030753.3 | 604 | ctgacttcggcgttagtgtcc | 255480 | - | see also 8927 line | | |
| 17885 | WNT3A | NM_033131.2 | 80 | tggcccactcgatacttctta | 255486 | - | - | signal transducer | |
| 17886 | WNT5B | NM_030775.2 | 756 | ccggtcgcagggctgtgtataag | 255498 | - | - | signal transducer | |
| 17887 | WNT6 | NM_006522.2 | 579 | ccgtcacgcaggccgttctatg | 255503 | - | - | signal transducer | |

Figure 46

| | | | | | | | | | |
|---|---|---|---|---|---|---|---|---|---|
| 17888 | WNT7B | NM_058238.1 | 888 | gagacagacctggtgtacattga | 255510 | - | see also 9505 line | | |
| 17889 | WNT8A | NM_031933.1 | 192 | caggtcccaaggcctatctgacc | 255512 | - | - | - | - |
| 17890 | WNT8B | NM_003393.2 | 1110 | ccggagggtcaccaagtacttct | 255536 | - | see also 2290 line | | |
| 17891 | WNT9A | NM_003395.1 | 508 | ggggctgcggagacaaccttaag | 255537 | - | - | signal transducer | - |
| 17892 | C3orf1 | NM_016589.1 | 451 | tgggtgtatgggggaataccagc | 255546 | - | - | - | - |
| 17893 | SEC61A1 | NM_013336.2 | 464 | ttggctggcgccaagataattga | 255572 | - | - | protein translocase | - |
| 17894 | SEC61G | NM_014302.2 | 120 | aagtcggcagtttgtaaaggact | 255632 | - | - | protein translocase | - |
| 17895 | TIMM17A | NM_006335.1 | 499 | tggagactatcgacaatatcagt | 255652 | - | - | protein translocase | - |
| 17896 | TIMM17B | NM_005834.1 | 197 | ttgcggtggagccttcactatgg | 255653 | - | - | protein translocase | - |
| 17897 | TIMM22 | NM_013337.1 | 233 | ctgcgtgggaggatttgtcttag | 255656 | - | - | - | - |
| 17898 | TIMM23 | NM_006327.1 | 406 | tggtcttcggctaggattgaagg | 255674 | - | - | protein translocase | - |
| 17899 | TIMM8A | NM_004085.2 | 235 | ttgagcgcttcattgatacaagc | 255682 | - | - | protein translocase | deafness |
| 17900 | SLC6A6 | NM_003043.1 | 388 | cggctatgcctccgttgtaattg | 255686 | - | see also 2038 line | | |
| 17901 | SLC6A4 | NM_001045.2 | 408 | tggagtccgggcaaatatccaat | 255715 | - | - | serotonin:sodium symporter | anxiety-related traits、alcoholism、Alzheimer disease |
| 17902 | SLC6A2 | NM_001043.1 | 718 | gagcagcgggattcatgacatcg | 255753 | - | - | noradrenaline:sodium symporter | alcoholism |
| 17903 | SLC6A8 | NM_005629.1 | 652 | gcgccgagaacggcatctatagc | 255773 | - | - | creatine:sodium symporter | - |
| 17904 | SLC6A9 | NM_006934.1 | 566 | aggagtgggctatggtatgatgg | 255780 | - | see also 4861 line | | |
| 17905 | FLJ31236 | NM_182632.1 | 1056 | ggggttcaaagcaactaatgact | 255795 | - | - | - | - |
| 17906 | NTT5 | NM_014037.1 | 1575 | cagcgcaatagggattatgcagg | 255833 | - | - | neurotransmitter:sodium symporter | |
| 17907 | SLC6A11 | NM_014229.1 | 1552 | ccggccaccgtcgctcattaagt | 255928 | - | see also 5547 line | | |
| 17908 | SLC6A12 | NM_003044.2 | 951 | ctgtggtcatcgagtcatatttg | 255938 | - | see also 2046 line | | |
| 17909 | SLC6A13 | NM_016615.2 | 322 | cagcactaggccagtacactagc | 255955 | - | see also 6952 line | | |
| 17910 | SLC6A14 | NM_007231.1 | 1194 | tgggacacatggcccatatatct | 256006 | - | see also 5043 line | | |
| 17911 | SLC6A3 | NM_001044.2 | 481 | tggccctcggccagttcaacagg | 256027 | - | see also 640 line | | |
| 17912 | SLC6A5 | NM_004211.1 | 2395 | atggctcttaccgctatcctaac | 256077 | - | see also 2820 line | | |
| 17913 | SLC6A7 | NM_014228.2 | 2124 | gagaaccggacgggcatgtatgt | 256094 | - | - | neurotransmitter:sodium symporter | |
| 17914 | XT3 | NM_020208.1 | 72 | gtgggccaactcgctacagttcg | 256096 | - | - | - | - |
| 17915 | SLC10A1 | NM_003049.1 | 533 | aaggacaaggtgccctataaagg | 256118 | - | - | bile acid:sodium symporter | - |
| 17916 | SLC10A2 | NM_000452.1 | 885 | aggccgtagtggtgctcattata | 256128 | - | - | bile acid:sodium symporter | bile acid malabsorption |
| 17917 | SLC10A3 | NM_019848.2 | 555 | tggcacaggtcccttaagcatgc | 256156 | - | - | bile acid:sodium symporter | |

Figure 46

| | | | | | | | | | |
|---|---|---|---|---|---|---|---|---|---|
| 17918 | SLC10A4 | NM_152679.2 | 1130 | ctggaaacaggtagtcagaatgt | 256177 | - | | | |
| 17919 | SLC5A1 | NM_000343.1 | 1494 | tgggatttcacgtatgattact | 256210 | - | | sugar porter | congenital glucose-galactose malabsorbtion syndrome |
| 17920 | SLC23A2 | NM_005116.4 | 2162 | acggcatggagtcgtacaattg | 256255 | - | see also 3589 line | | |
| 17921 | SLC38A1 | NM_030674.2 | 1707 | ttggagtcgtaggagttacatct | 256313 | - | | | |
| 17922 | SLC13A2 | NM_003984.1 | 297 | gaggttgccgtcgagtatcttaa | 256319 | | | low affinity sodium:dicarboxylate cotransporter | |
| 17923 | SLC28A1 | NM_004213.2 | 1888 | gagcggacttcccagatagtgc | 256342 | - | | nucleoside:sodium symporter | |
| 17924 | SLC28A2 | NM_004212.1 | 1979 | cagtgctatggcccttactaact | 256379 | - | | purine nucleoside transporter | |
| 17925 | SLC28A3 | NM_022127.1 | 782 | atgggaatcggctacagtttc | 256396 | - | | | |
| 17926 | SLC15A1 | NM_005073.1 | 2053 | tggctcggttctatacttacatc | 256461 | - | see also 3545 line | | |
| 17927 | SLC15A2 | NM_021082.2 | 476 | ttgatcggcctgagtctaatagc | 256480 | - | see also 8081 line | | |
| 17928 | SLC17A5 | NM_012434.3 | 814 | ttgtggatctggttagttagtga | 256552 | - | | | |
| 17929 | SLC12A1 | NM_000338.1 | 2169 | ttgtgggaccggcaaaactgtgt | 256631 | - | see also 284 line | | |
| 17930 | SLC12A2 | NM_001046.2 | 1501 | ttggtgatttcgtcataggaaca | 256712 | - | see also 641 line | | |
| 17931 | SLC12A5 | NM_020708.3 | 1907 | gaggccacgctttcgatattacc | 256800 | - | see also 7927 line | | |
| 17932 | SLC12A7 | NM_006598.1 | 1440 | gcgtggtctacgagataagttc | 256829 | - | see also 4631 line | | |
| 17933 | SLC12A3 | NM_000339.1 | 1311 | ctgccactacggctcatcaact | 256845 | - | | symporter | Gitelman syndrome (hypomagnesemia-hypokalemia) |
| 17934 | SLC12A6 | NM_005135.1 | 2958 | ccgaaactcaatgctacgattga | 256949 | - | see also 3611 line | | |
| 17935 | SLC13A4 | NM_012450.2 | 2445 | ctgggagtcaacgttattggact | 256973 | - | | | |
| 17936 | SLC4A4 | NM_003759.1 | 2523 | atggcttccgtggtatgtagc | 257056 | - | see also 2569 line | | |
| 17937 | SLC4A5 | NM_021196.2 | 2362 | ctgatgttctgtggaatcgatgc | 257103 | - | | | |
| 17938 | SLC4A7 | NM_003615.2 | 2631 | aaggtgcgatcgacaatcagtga | 257169 | - | | | |
| 17939 | SLC12A4 | NM_005072.3 | 948 | tgggggcataaagtctatatttg | 257212 | - | see also 3544 line | | |
| 17940 | SLC13A1 | NM_022444.3 | 1111 | tagtcgagaccccggatttgttc | 257264 | - | see also 8358 line | | |
| 17941 | SLC13A3 | NM_022829.3 | 709 | atgaatacgtcggaacatctgg | 257296 | - | see also 8485 line | | |
| 17942 | SLC16A1 | NM_003051.2 | 735 | ttggggcttgctactaaactgc | 257327 | - | | symporter | mevalonate uptake facilitator |
| 17943 | SLC16A2 | NM_006517.1 | 1184 | ctgagtaccttcatgtttgttct | 257368 | - | | symporter | |
| 17944 | SLC16A3 | NM_004207.1 | 333 | atgcttgtggggggtctctttgc | 257379 | - | | symporter | |
| 17945 | SLC16A4 | NM_004696.1 | 725 | tggagctatcgcattgaatttgg | 257398 | - | see also 3219 line | | |
| 17946 | SLC16A5 | NM_004695.2 | 1673 | acgtccagctggcgtcaataagc | 257425 | - | | | |
| 17947 | SLC16A6 | NM_004694.2 | 452 | cggacaccgtccttaaccataat | 257433 | - | | symporter | |
| 17948 | SLC16A7 | NM_004731.3 | 538 | ctgcaaccgccttaaccataat | 257453 | - | see also 3249 line | | |
| 17949 | SLC17A1 | NM_005074.1 | 512 | gaggccgacttacttctatgagt | 257506 | - | | symporter | |
| 17950 | SLC17A2 | NM_005835.1 | 1318 | cagtactctgctccatgttaaca | 257571 | - | | symporter | |

Figure 46

EP 1 752 536 A1

| 17951 | SLC17A3 | NM_006632.1 | 436 | tggaatagccctcgtcttacatt | 257581 | - | - | symporter | - |
|---|---|---|---|---|---|---|---|---|---|
| 17952 | SLC1A1 | NM_004170.3 | 478 | ctgcactggattccaacgtatcc | 257615 | - | - | sodium:dicarboxylate/ tricarboxylate cotransporter | - |
| 17953 | SLC1A3 | NM_004172.3 | 908 | gagactcttacccgaatcacaga | 257665 | - | see also 2801 line | | |
| 17954 | SLC1A4 | NM_003038.2 | 1221 | cagcgacccttccctctatgatg | 257689 | - | - | sodium:dicarboxylate/ tricarboxylate cotransporter | - |
| 17955 | SLC1A6 | NM_005071.1 | 1507 | ttgaccggcttcgcacaatgacc | 257704 | - | see also 3541 line | | |
| 17956 | SLC1A7 | NM_006671.3 | 1493 | ccgtttccgcaccatgattaacg | 257717 | - | - | sodium:dicarboxylate/ tricarboxylate cotransporter | - |
| 17957 | SLC23A1 | NM_005847.2 | 1590 | aggagcgtggtctgatacagtgg | 257731 | - | - | - | - |
| 17958 | SLC24A1 | NM_004727.1 | 378 | atgttgatcatcggttctactta | 257737 | - | - | symporter | - |
| 17959 | SLC24A2 | NM_020344.1 | 379 | aaggcgagtctgagaatagtaca | 257782 | - | see also 7842 line | | |
| 17960 | SLC24A3 | NM_020689.3 | 1234 | tggccagtcgcatgttgatcaat | 257833 | - | - | - | - |
| 17961 | SLC34A1 | NM_003052.2 | 923 | tggacgagtctgtgataaccagc | 257847 | - | - | - | - |
| 17962 | SLC5A4 | NM_014227.1 | 298 | aggagtcgccaccgtaacatttg | 257853 | - | - | symporter | - |
| 17963 | SLC5A5 | NM_000453.1 | 470 | gcgcagcgctgaggacttcttca | 257895 | - | - | symporter | thyroid cancer、 hypothyroidism、 thyroid hormonogenesis |
| 17964 | TSCOT | NM_033051.2 | 1296 | tggaaaggtgttcgtcatactgc | 257920 | - | - | - | - |
| 17965 | FLJ14753 | NM_032558.1 | 482 | tggctcagacggcctttcttagc | 257940 | - | see also 9247 line | | |
| 17966 | HIAT1 | NM_033055.2 | 1125 | aggaattcgaggattatgcaatg | 257975 | - | see also 9355 line | | |
| 17967 | MGC11332 | NM_032718.2 | 341 | gggctgctggagcgatgtagtgg | 257982 | - | see also 9277 line | | |
| 17968 | LOC133308 | NM_178833.2 | 781 | ctgtccatgggtccctgtattgt | 258024 | - | - | - | - |
| 17969 | MGC43026 | NM_178527.2 | 3223 | ctgtctgcttaagcgtgaaattg | 258154 | - | channel | - | - |
| 17970 | SLC9A1 | NM_003047.2 | 1218 | tgggcggtgagcagatcaacaac | 258167 | - | - | solute:hydrogen antiporter | - |
| 17971 | SLC9A2 | NM_003048.3 | 1651 | ttgtcggttgtttgatcatgtga | 258211 | - | - | solute:hydrogen antiporter | - |
| 17972 | SLC9A3 | NM_004174.1 | 1494 | ccggacagatcgggcacaattat | 258235 | - | - | solute:hydrogen antiporter | - |
| 17973 | SLC9A5 | NM_004594.1 | 265 | ctgtctcggaaagtaacatctct | 258245 | - | - | solute:hydrogen antiporter | - |
| 17974 | SLC9A6 | NM_006359.1 | 1453 | atgcacggcaaatgatgttcagc | 258292 | - | see also 4449 line | | |
| 17975 | SLC9A7 | NM_032591.1 | 936 | ctgaacactcacgcctttgatgc | 258315 | - | - | - | - |

Figure 46

| | | | | | | | | | |
|---|---|---|---|---|---|---|---|---|---|
| 17976 | SLC9A9 | NM_173653.1 | 644 | ctgcatcgtcatagggttaatta | 258332 | - | see also 10103 line | | |
| 17977 | SLC4A1 | NM_000342.1 | 985 | gagagggtgttccgcatagatgc | 258369 | - | - | inorganic anion exchanger | chorea-acanthocytosis、elliptocytosis、erythrocytosis、ovaloc ytosis |
| 17978 | SLC4A11 | NM_032034.1 | 173 | tcgaggattcaagctactacaag | 258380 | - | - | - | - |
| 17979 | SLC4A2 | NM_003040.2 | 2765 | acggcggtgagaacatgacatgg | 258430 | - | - | inorganic anion exchanger | - |
| 17980 | SLC4A3 | NM_005070.1 | 847 | gtgtgcggcgacacttagtgaaa | 258447 | - | see also 3540 line | | |
| 17981 | SLC4A8 | NM_004858.1 | 91 | cagggtgggaccagtacaattct | 258460 | - | see also 3376 line | | |
| 17982 | SLC7A11 | NM_014331.2 | 1215 | tgggctgatttatcttcgataca | 258548 | - | see also 5626 line | | |
| 17983 | FLJ10847 | NM_018242.2 | 312 | ctggcaatcgcggttatcaatgt | 258562 | - | see also 7341 line | | |
| 17984 | FLJ31196 | NM_152908.2 | 1286 | ttgccggtttatagtgtctttca | 258613 | - | - | - | - |
| 17985 | CHDC1 | NM_015116.1 | 960 | aagtgctcgtgattccaatttgt | 258644 | - | - | - | - |
| 17986 | FLJ10560 | NM_018138.1 | 316 | tggcgtggctttacttcgaaata | 258693 | - | - | - | - |
| 17987 | FLJ37307 | NM_178515.2 | 536 | tggaggcacctcccttcatcaga | 258740 | - | - | - | - |
| 17988 | HT008 | NM_018469.3 | 3296 | ccgactgaccgagtatggtatgg | 258810 | - | - | - | - |
| 17989 | KST1 | NM_052944.2 | 851 | cagacgctgatcatgcttatagg | 258823 | - | - | - | - |
| 17990 | MGC34837 | NM_152377.1 | 108 | aagactccccgtggatcagatgc | 258835 | - | - | - | - |
| 17991 | NALP13 | NM_176810.1 | 1162 | ggggacgacctacgggtatattt | 258898 | - | - | - | - |
| 17992 | PTD015 | NM_024684.2 | 148 | aaggctctaatacaacctataag | 258945 | - | - | - | - |
| 17993 | SLC2A1 | NM_006516.1 | 1547 | aggccggaccttcgatgagatcg | 258962 | - | - | sugar porter | glucose transport defect、 blood-brain barrier、 bladder cancer、 diabetes mellitus、 endometrial cancer、 lung cancer、 pancreatic cancer、 diabetes、 head and neck cancer、 gastric cancer |
| 17994 | SLC2A10 | NM_030777.3 | 151 | tggcctgacctttggttatgaac | 258964 | - | - | sugar porter | diabetes mellitus、diabetes |
| 17995 | SLC2A11 | NM_030807.2 | 216 | acggcctcggacgcagattcagg | 258975 | - | - | sugar porter | - |
| 17996 | SLC2A12 | NM_145176.1 | 956 | atgcggacccgaataatgatagg | 259010 | - | see also 9778 line | | |
| 17997 | SLC2A13 | NM_052885.1 | 666 | aggccgattagtcaccattaata | 259047 | - | - | sugar porter | - |
| 17998 | SLC2A14 | NM_153449.2 | 1121 | cgggtgtggttaatactatcttc | 259159 | - | - | - | - |
| 17999 | SLC2A2 | NM_000340.1 | 1338 | tggagttggcgctgtaaacatgg | 259134 | - | see also 287 line | | |
| 18000 | SLC2A4 | NM_001042.1 | 149 | ccgtcgggcttccaacagatagg | 259165 | - | - | sugar porter | diabetes mellitus、diabetes |
| 18001 | SLC2A5 | NM_003039.1 | 239 | atggtaggaccggtgaattcatg | 259176 | - | - | sugar porter | - |
| 18002 | SLC2A6 | NM_017585.2 | 497 | ccggtgtacgtgtctgagattgc | 259195 | - | - | sugar porter | - |
| 18003 | SLC2A8 | NM_014580.2 | 864 | gggggtcaacgccgtcatgttct | 259199 | - | - | sugar porter | - |
| 18004 | SLC2A9 | NM_020041.1 | 339 | aaggcatggacgtccaatagacc | 259206 | - | - | sugar porter | - |

Figure 46

| ID | Gene | Accession | Pos | Sequence | | Num | | Note | | Description | Disease |
|---|---|---|---|---|---|---|---|---|---|---|---|
| 18005 | SLC35A2 | NM_005660.1 | 867 | ttgtcaagtacgctgacaatatc | - | 259230 | - | - | - | sugar porter | - |
| 18006 | SLC35A3 | NM_012243.1 | 267 | atgcgttattccagaactttaaa | - | 259236 | - | see also 5220 line | - | | - |
| 18007 | SLC35C1 | NM_018389.3 | 890 | cagtccgctgacccacaatgtgt | - | 259276 | - | - | - | sugar porter | - |
| 18008 | SLC35D1 | NM_015139.1 | 850 | cagtactctgcacgcagtataat | - | 259294 | - | see also 6258 line | - | | - |
| 18009 | SLC37A1 | NM_018964.2 | 1352 | ctgccgattaggtattacctaac | - | 259317 | - | see also 7611 line | - | | - |
| 18010 | SLC37A4 | NM_001467.2 | 1461 | ttgtaggtagctctcacatgagt | - | 259343 | - | - | - | sugar porter | glycogen storage disease |
| 18011 | SLC7A7 | NM_003982.2 | 1620 | atggtatcatgcattgatctac | - | 259360 | | | | basic amino acid permease | lysinuric protein intolerance |
| 18012 | SLC7A2 | NM_003046.1 | 558 | tcgtatgtgataggtaccatcaag | - | 259372 | | | | basic amino acid transporter | |
| 18013 | SLC7A3 | NM_032803.3 | 923 | ttggtttcgactgattgctacc | - | 259418 | - | | - | | |
| 18014 | SLC7A5 | NM_003486.4 | 1495 | cagcgggctgccgtctacttct | - | 259451 | - | | - | neutral amino acid transporter | |
| 18015 | SLC7A8 | NM_012244.2 | 1467 | ctggaactttcgaattacgtga | - | 259462 | - | see also 5221 line | | | |
| 18016 | FLJ10815 | NM_018231.1 | 1054 | aagtacatctggccagataaaga | - | 259476 | - | - | | | |
| 18017 | FLJ39822 | NM_173512.1 | 850 | gtggtccgccttatccatatgt | - | 259494 | - | - | | | |
| 18018 | FLJ90709 | NM_173514.1 | 1783 | gggcgtggctaacctgattgttc | - | 259574 | - | see also 10078 line | | | |
| 18019 | MGC15523 | NM_138570.1 | 1291 | atgccccctccgtttaaagc | - | 259582 | - | - | | | |
| 18020 | SLC12A8 | NM_024628.3 | 1269 | acgttgcagtggactactcttac | - | 259606 | - | - | | | |
| 18021 | SLC36A1 | NM_078483.1 | 308 | cagcgctttggtcaaagcaatag | - | 259623 | - | see also 9509 line | | | |
| 18022 | SLC36A4 | NM_152313.2 | 136 | gaggcgcgaggagctagatatgg | - | 259647 | - | see also 9840 line | | | |
| 18023 | SLC38A3 | NM_006841.3 | 1363 | ttggcctgtcacttgtatcaac | - | 259712 | - | - | | | non-small-cell lung cancer |
| 18024 | SLC38A4 | NM_018018.2 | 1355 | cggacggcctatgcaattcctat | - | 259749 | - | see also 7212 line | | | |
| 18025 | SLC38A5 | NM_033518.1 | 982 | aagacatcgtttggaatgtcagt | - | 259777 | - | - | - | | |
| 18026 | SLC7A10 | NM_019849.1 | 1443 | ctgtggggtcgggtcatcatca | - | 259798 | - | - | - | | |
| 18027 | SLC7A13 | NM_138817.1 | 1248 | caggaaccaatctatctatacc | - | 259834 | - | - | - | | |
| 18028 | SLC7A4 | NM_004173.1 | 440 | atgttcctctgaatacatcatc | - | 259852 | - | - | | basic amino acid transporter | |
| 18029 | SLC7A6 | NM_003983.1 | 591 | cagctacgcttatattcttagagg | - | 259864 | - | see also 2721 line | - | | |
| 18030 | SLC7A9 | NM_014270.1 | 614 | ctgcaagcctcccaaatcgttg | - | 259894 | - | - | - | cystine transporter | cystinuria |
| 18031 | VIAAT | NM_080552.2 | 496 | ccgtcgagggagagcaatccattat | - | 259920 | - | - | - | | |
| 18032 | SLC17A4 | NM_005495.1 | 786 | aggatggccttacgtcttctata | - | 259950 | - | - | - | symporter | |
| 18033 | SLC17A7 | NM_020309.1 | 1422 | gtggcagtacgttcctaattg | - | 259972 | - | - | - | | |
| 18034 | SLC34A2 | NM_006424.1 | 1395 | tggaatcggcgtgataaccattg | - | 260001 | - | - | - | | |
| 18035 | ANKH | NM_054027.3 | 1073 | gagaatcagtcggcctattgtca | - | 260032 | - | see also 9499 line | - | | |
| 18036 | SLC26A11 | NM_173626.1 | 1520 | caggacgctctggcgtgttaaga | - | 260050 | - | - | - | | |
| 18037 | SLC26A2 | NM_000112.1 | 1015 | aaggcacgcattcctattgaact | - | 260077 | - | see also 95 line | - | | |

731

Figure 46

| | | | | | | | | | |
|---|---|---|---|---|---|---|---|---|---|
| 18038 | SLC26A4 | NM_000441.1 | 2382 | ttgacggtccatgatgctatact | 260180 | - | see also 349 line | | |
| 18039 | SLC26A6 | NM_022911.2 | 1578 | tggtggttgcggtcatcttctcc | 260200 | - | - | - | - |
| 18040 | SLC26A7 | NM_052832.2 | 2083 | aagcaatgacgtattatggaaac | 260247 | - | see also 9433 line | | |
| 18041 | SLC26A8 | NM_052961.2 | 170 | atgttaagcgtgaagtatacaat | 260259 | - | - | - | - |
| 18042 | SLC22A11 | NM_018484.2 | 547 | ctgcggtttccacaaacatgacc | 260375 | - | - | - | - |
| 18043 | SLC22A7 | NM_006672.2 | 445 | gggagtacgaccactcagaattc | 260384 | - | - | - | - |
| 18044 | SLCO1B1 | NM_006446.2 | 1954 | gtggcacacgtgggtcatgtagg | 260422 | - | - | sodium-independent organic anion transporter | - |
| 18045 | AFM | NM_001133.2 | 1414 | ctgtacgctaagtgaagagtttg | 260479 | - | - | carrier | - |
| 18046 | ECM1 | NM_004425.2 | 1519 | gtgtggtccccgacgtaacatct | 260508 | - | see also 2958 line | | |
| 18047 | LOC90139 | NM_130783.1 | 272 | gggccgtccgtgagaacaagtgt | 260512 | - | - | - | - |
| 18048 | KCNJ3 | NM_002239.2 | 536 | aagcccacgtcggtaactacacg | 260524 | - | see also 1434 line | | |
| 18049 | KCNJ6 | NM_002240.2 | 1355 | tggtgtcaccgctgatcattagc | 260581 | - | channel、gpcr | G-protein activated inward rectifier potassium channel | diabetes mellitus、diabetes |
| 18050 | KCNJ5 | NM_000890.3 | 794 | gtgtccagaggggattatactcc | 260589 | - | see also 581 line | | |
| 18051 | KCNJ12 | NM_021012.2 | 516 | gtgcccggtggccgtcttcatgg | 260596 | - | - | inward rectifier potassium channel | - |
| 18052 | KCNJ13 | NM_002242.2 | 861 | acgtactatcactccattacacc | 260621 | - | channel | inward rectifier potassium channel | - |
| 18053 | KCNJ14 | NM_013348.2 | 1185 | tggaggcaccgatcgtatcttcc | 260633 | - | channel | - | - |
| 18054 | KCNJ15 | NM_002243.3 | 750 | ttggtcatcacgaccttgattga | 260642 | - | channel | - | - |
| 18055 | KCNJ16 | NM_018658.1 | 1591 | gcgagacgaaggtcatttagtgc | 260688 | - | channel | - | - |
| 18056 | KCNJ2 | NM_000891.2 | 1148 | cagtggaatcgatcgtatatttc | 260706 | - | channel | inward rectifier potassium channel | - |
| 18057 | KCNJ4 | NM_004981.1 | 259 | ctggcgctacatgctcatgatct | 260724 | - | channel | - | - |
| 18058 | KCNJ8 | NM_004982.2 | 464 | ctgcaacctggcgcataagaaca | 260730 | - | channel | ATP-activated inward rectifier potassium channel | - |
| 18059 | KCNJ9 | NM_004983.2 | 964 | tcgtctcgccgctggttatcagc | 260753 | - | channel、gpcr | G-protein activated inward rectifier potassium channel | - |
| 18060 | KCNK1 | NM_002245.2 | 1044 | gaggatcaggtgcacatcataga | 260758 | - | channel | inward rectifier potassium channel | - |
| 18061 | KCNK6 | NM_004823.1 | 930 | ccgtgccctgccagtttcaatgc | 260763 | - | channel | inward rectifier potassium channel | - |
| 18062 | KCNQ5 | NM_019842.2 | 628 | cgggttgctgttgtcgatataga | 260768 | - | channel | inward rectifier potassium channel | - |

Figure 46

| 18063 | KCNE1 | NM_000219.1 | 38 | cagcggtgacgccctttctgacc | 260835 | - | channel | potassium channel regulator | Jervell and Lange-Nielsen syndrome、 long QT syndrome |
|---|---|---|---|---|---|---|---|---|---|
| 18064 | KCNQ1 | NM_000218.2 | 1878 | ccgcctgaaccgagtagaagaca | 260849 | - | channel | - | Jervell and Lange-Nielsen syndrome、 Romano-Ward syndrome |
| 18065 | KCNK2 | NM_014217.1 | 747 | gtggttatcactctaacaactat | 260893 | - | channel | outward rectifier potassium channel | - |
| 18066 | KCNAB1 | NM_003471.2 | 1047 | taggtgttggcgcaatgacatgg | 260927 | - | channel | - | - |
| 18067 | KCNAB2 | NM_003636.2 | 414 | atggcatcaacctcttcgataca | 260944 | - | channel | - | - |
| 18068 | KCNAB3 | NM_004732.2 | 497 | cagaaaccgagcgaggtttaagc | 260959 | - | channel | voltage-gated potassium channel | - |
| 18069 | KCNE2 | NM_172201.1 | 429 | aagagccaaatcttgaatctaga | 260976 | - | channel | voltage-gated potassium channel | long QT syndrome |
| 18070 | KCNE3 | NM_005472.2 | 450 | aagcgtagtgacccctatcatgt | 260985 | - | see also 3810 line | | |
| 18071 | KCNG3 | NM_133329.4 | 1299 | gagtgcatcgtgaggttcattgt | 261000 | - | see also 9564 line | | |
| 18072 | KCNH6 | NM_030779.2 | 1248 | ctggaccgctactctgagtatgg | 261030 | - | channel、 signal_transd uction、 transcription_r egulator | - | - |
| 18073 | KCNQ2 | NM_172106.1 | 942 | gggggagaacgaccactttgaca | 261043 | - | channel | - | benign familial neonatal convulsions(-)、 epilepsy |
| 18074 | KCNQ2 | NM_004518.2 | 1820 | tggacatgctgtcccgaattaag | 261047 | - | channel | - | benign familial neonatal convulsions(-)、 epilepsy |
| 18075 | KCNQ4 | NM_004700.2 | 1375 | cagccggatgggcatcaaagacc | 261106 | - | channel | - | deafness |
| 18076 | KCNV2 | NM_133497.2 | 775 | ctggggcgtgcggctcaagtaca | 261113 | - | channel | - | - |
| 18077 | KCNMB1 | NM_004137.2 | 764 | aaggtcagagccaaattccaaga | 261119 | - | channel | potassium channel regulator | - |
| 18078 | KCNMB2 | NM_005832.3 | 562 | atgcgtccatcacggaaacattt | 261125 | - | channel | - | - |
| 18079 | KCNMB3 | NM_014407.3 | 735 | ttgctcggaacaaccattctaaa | 261148 | - | channel | - | - |
| 18080 | KCNMB4 | NM_014505.4 | 536 | gcgtcgtgtcgctcttcatcttc | 261167 | - | channel | - | - |
| 18081 | HCN1 | NM_021072.1 | 821 | ttgcgtttattacgactttcaag | 261187 | - | see also 8077 line | | |
| 18082 | KCNK10 | NM_021161.3 | 2029 | gggagccggagaacaactcatta | 261265 | - | see also 8114 line | | |
| 18083 | KCNK12 | NM_022055.1 | 867 | ctgcatttactcgctcttcaacg | 261271 | - | channel | - | - |
| 18084 | KCNK13 | NM_022054.2 | 1177 | cagccagaacgcccactatgaga | 261275 | - | see also 8253 line | | |
| 18085 | KCNK17 | NM_031460.2 | 774 | cggcgactacgtgattggaatga | 261287 | - | - | - | - |
| 18086 | KCNK4 | NM_016611.2 | 623 | acgtgccaccggagctagtaaga | 261303 | - | channel | - | - |
| 18087 | KCNK5 | NM_003740.2 | 863 | tggtgatccccacccttcgtattc | 261318 | - | see also 2553 line | | |
| 18088 | KCNK7 | NM_005714.1 | 896 | cagcctgcaccccgtgatttacc | 261324 | - | channel | - | - |
| 18089 | KCNK9 | NM_016601.2 | 726 | tggcctttagctttatgtatatc | 261331 | - | channel | voltage-gated ion channel | - |
| 18090 | CACNA2D1 | NM_000722.1 | 119 | tcggccgtcactatcaaatcatg | 261338 | - | see also 471 line | | |

Figure 46

| | | | | | | | | | |
|---|---|---|---|---|---|---|---|---|---|
| 18091 | CACNG1 | NM_000727.2 | 482 | gcgacccgcgtccatgttctatg | 261466 | - | channel | dihydropyridine-sensitive calcium channel | - |
| 18092 | CACNB1 | NM_000723.3 | 1923 | ctggggacgaggcgtctacattc | 261482 | - | channel | voltage-gated calcium channel | - |
| 18093 | CACNB2 | NM_000724.1 | 808 | tggtggatagggcgattggtaaa | 261492 | - | see also 475 line | | |
| 18094 | CACNB4 | NM_000726.1 | 1417 | ttgaaagacgaagtctaatgacc | 261558 | - | channel | voltage-gated calcium channel | idiopathic generalized epilepsy、myoclonic epilepsy |
| 18095 | CACNG2 | NM_006078.2 | 865 | gtgctggcggtgcacatgtttat | 261566 | - | see also 4221 line | | |
| 18096 | CACNG3 | NM_006539.2 | 1780 | gtggttgccgtgcacatctatat | 261592 | - | see also 4572 line | | |
| 18097 | CACNG4 | NM_014405.2 | 252 | ctgcttccggatcaatcacttcc | 261601 | - | see also 5663 line | | |
| 18098 | CACNG5 | NM_014404.1 | 208 | gggcgttgcttcaccatagaata | 261607 | - | channel | - | - |
| 18099 | CACNG7 | NM_031896.3 | 758 | gcgacgtgtccatccaaatgacg | 261634 | - | see also 9048 line | | |
| 18100 | CACNG8 | NM_031895.4 | 396 | gaggcgtctgcgtgaagatcaat | 261637 | - | see also 9047 line | | |
| 18101 | TRPC3 | NM_003305.1 | 1325 | aggcacaaagcttcattaagtcg | 261729 | - | see also 2243 line | | |
| 18102 | TRPC5 | NM_012471.1 | 2994 | aaggcgcaacttgagaagtttca | 261810 | - | see also 5345 line | | |
| 18103 | TRPC6 | NM_004621.3 | 653 | gggagaaggttagctaatcgagg | 261830 | - | see also 3141 line | | |
| 18104 | TRPM1 | NM_002420.3 | 1823 | gaggcctaaagctcttaaacttc | 261923 | - | channel | - | melanoma |
| 18105 | TRPM3 | NM_020952.1 | 274 | aagatatgcaccataggtattgc | 261991 | - | see also 8046 line | | |
| 18106 | SCN11A | NM_014139.1 | 2879 | caggaggaggcttggtatactaac | 262160 | - | see also 5511 line | | |
| 18107 | SCN1B | NM_001037.2 | 329 | aggatctgtctatcttcatcacc | 262229 | - | see also 639 line | | |
| 18108 | SCN2B | NM_004588.2 | 362 | ccgcatgaagatcattaacctga | 262241 | - | channel | voltage-gated sodium channel | - |
| 18109 | SCN3B | NM_018400.2 | 996 | aggcccgagggcggtaaagattt | 262246 | - | see also 7447 line | | |
| 18110 | SCN5A | NM_000335.3 | 737 | ctgcctgcacgcgttcactttcc | 262260 | - | channel | - | sick sinus syndrome、heart block、long QT syndrome、Brugada syndrome、cardiac conduction defect |
| 18111 | SCN7A | NM_002976.1 | 3834 | cagtacctcgcccattaaacaag | 262391 | - | channel | voltage-gated sodium channel | - |
| 18112 | ACCN2 | NM_001095.2 | 1788 | gaggcacgttcgaggactttacc | 262450 | - | channel | - | - |
| 18113 | ACCN3 | NM_004769.1 | 1011 | cggctgccgaatggtgtacatgc | 262455 | - | channel | - | - |
| 18114 | APXL | NM_001649.2 | 993 | ctggtatgttcccgataagaaga | 262467 | - | see also 1092 line | | |
| 18115 | SCNN1B | NM_000336.1 | 1602 | ttgtcaagctcaacatctacttc | 262508 | - | channel | amiloride-sensitive sodium channel | pseudoaldosteronism、pseudohypoaldosteronism |
| 18116 | SCNN1D | NM_002978.2 | 860 | ctggtaccacttccactatgtgg | 262511 | - | channel | amiloride-sensitive sodium channel | - |
| 18117 | SCNN1G | NM_001039.1 | 684 | gtggtataagctacactacatga | 262524 | - | channel | amiloride-sensitive sodium channel | pseudoaldosteronism、pseudohypoaldosteronism |

Figure 46

| | | | | | | | | | |
|---|---|---|---|---|---|---|---|---|---|
| 18118 | ACCN4 | NM_018674.3 | 1773 | gagatcctcgactacatctatga | 262547 | - | see also 7548 line | | |
| 18119 | ACCN5 | NM_017419.1 | 327 | acgtccattgaggttcaatatgt | 262558 | - | channel | - | - |
| 18120 | CATSPER1 | NM_053054.2 | 903 | cagcgtgggatatctgactatca | 262595 | - | see also 9488 line | | |
| 18121 | CATSPER2 | NM_054020.2 | 402 | cagctagtgcgtttctctataaa | 262619 | - | see also 9497 line | | |
| 18122 | CATSPER3 | NM_178019.1 | 87 | cagcgccactcgagagtcatttc | 262634 | - | channel | - | - |
| 18123 | MCOLN3 | NM_018298.9 | 1186 | aagagtctaactagttatgatgt | 262700 | - | channel | - | - |
| 18124 | MGC15619 | NM_032369.1 | 1304 | cagaacggcaactcttaaggtta | 262727 | - | channel | - | - |
| 18125 | NOLA1 | NM_018983.2 | 550 | aagatgacatagtttgtaaatgt | 262735 | - | channel | - | - |
| 18126 | TPCN1 | NM_017901.3 | 1411 | gtgccagggagcgctatcttacc | 262745 | - | channel | - | - |
| 18127 | TPCN2 | NM_139075.1 | 1520 | ctgcgtcttcattgtgtactacc | 262774 | - | channel | - | - |
| 18128 | TRPM4 | NM_017636.2 | 129 | ctgcacgacgttcatagttgact | 262793 | - | receptor_acitivity、channel | - | - |
| 18129 | TRPM5 | NM_014555.2 | 2216 | tggggaacgtggtcatgtacttc | 262817 | - | channel | - | - |
| 18130 | VGCNL1 | NM_052867.1 | 3468 | gggccgatccatggaatctatat | 262956 | - | channel | - | - |
| 18131 | CLCA1 | NM_001285.1 | 1511 | gcgggcttcgatcggcatttact | 263015 | - | channel | - | - |
| 18132 | CLCA2 | NM_006536.3 | 1212 | cagcaatgatgatcgaaagttgc | 263079 | - | see also 4569 line | | |
| 18133 | CLCA4 | NM_012128.2 | 994 | gggggtaaggaccgcctaaatcg | 263157 | - | channel | - | - |
| 18134 | CLCN1 | NM_000083.1 | 2755 | acgacttcaactcgaaagagtac | 263238 | - | see also 55 line | | |
| 18135 | CLCN2 | NM_004366.2 | 2481 | tggaatcgttactctaaaggagc | 263262 | - | see also 2927 line | | |
| 18136 | CLCN4 | NM_001830.2 | 894 | gtgcgtgtatttgcaccatatcg | 263333 | - | see also 1188 line | | |
| 18137 | CLCN6 | NM_001286.1 | 178 | tggattatgatcgctgtatcaat | 263414 | - | see also 847 line | | |
| 18138 | CLCN6 | NM_021736.1 | 967 | ttggcgagtttaagagtcttaga | 263444 | - | channel、signal_transduction | - | - |
| 18139 | CLCN7 | NM_001287.3 | 1992 | gcgacacggcgtccaatcacaac | 263463 | - | channel | voltage-gated chloride channel | - |
| 18140 | CLCNKA | NM_004070.2 | 1896 | gtgaccctgacgctattctcaga | 263466 | - | channel | voltage-gated chloride channel | - |
| 18141 | CLIC2 | NM_001289.3 | 762 | ctggctgattgtagcttgttacc | 263496 | - | channel | voltage-gated chloride channel | - |
| 18142 | CLIC4 | NM_013943.1 | 910 | aggaggttgaaatagcatatagt | 263513 | - | channel | voltage-gated chloride channel | - |
| 18143 | CLIC5 | NM_016929.1 | 431 | aaggagtcgtgttcaatgtcacc | 263517 | - | channel | voltage-gated chloride channel | - |
| 18144 | CLIC6 | NM_053277.1 | 2017 | gagattgaacacgcatattcaga | 263550 | - | see also 9489 line | | |

Figure 46

| | | | | | | | | | |
|---|---|---|---|---|---|---|---|---|---|
| 18145 | LGICZ | NM_180990.2 | 258 | tggacatcctgcgatacacaatg | 263552 | - | channel | | |
| 18146 | FXYD1 | NM_005031.3 | 176 | aggaacacgaccgttcacttac | 263565 | - | channel | | |
| 18147 | FLJ12770 | NM_032174.3 | 825 | ctgggtagctacattgaatgtgg | 263579 | - | receptor_acitivity, channel | | |
| 18148 | VDAC1 | NM_003374.1 | 295 | gagtacggcctgacgtttacaga | 263589 | - | channel | voltage-dependent ion-selective channel | voltage-dependent anion channel deficiency |
| 18149 | VDAC3 | NM_005662.3 | 230 | ctggtcatgcttacactgctaac | 263591 | - | channel | voltage-dependent ion-selective channel | |
| 18150 | AQP7 | NM_001170.1 | 453 | acgcagctgtgacctttgctaac | 263611 | - | | water channel | |
| 18151 | FXYD6 | NM_022003.1 | 218 | ttgggatcctcttatcctaagt | 263613 | - | channel | ion channel | |
| 18152 | FXYD7 | NM_022006.1 | 257 | ctgagagcccaactgcaaatcc | 263616 | - | channel | ion channel | |
| 18153 | MLC1 | NM_015166.2 | 1294 | gagagccgtcgtgggtgcaaatgg | 263636 | - | see also 6260 line | | |
| 18154 | TM4SF11 | NM_015993.1 | 133 | ccgagttccgtcgaaagttagc | 263638 | - | | ion channel | |
| 18155 | CX36 | NM_020660.1 | 634 | ggggttcctggttggccaatattt | 263653 | - | | | |
| 18156 | CX40.1 | NM_153368.1 | 330 | cagccgggatgcgccaatgtttg | 263662 | - | | | |
| 18157 | CX62 | NM_032602.1 | 450 | aggatgtctgtcgctacttatg | 263678 | - | | | |
| 18158 | GJA1 | NM_000165.2 | 607 | aagttcaagtacggtattgaaga | 263714 | - | kinase_related | ion transporter | visceroatrial heterotaxia |
| 18159 | GJA12 | NM_020435.1 | 165 | acgagcaggccaagttcaacttgc | 263735 | - | | | |
| 18160 | GJA5 | NM_005266.4 | 1050 | aggtttcatccaggttcgttatg | 263746 | - | | | |
| 18161 | GJB1 | NM_000166.2 | 158 | gtgggtgaacggcattctact | 263774 | - | see also 136 line | | |
| 18162 | GJB2 | NM_004004.2 | 335 | atgagcaggcgactttgtctgc | 263788 | - | | connexon channel | deafness |
| 18163 | GJB4 | NM_153212.1 | 857 | tggatgcaaggtggtatccataa | 263803 | - | | connexon channel | erythrokeratodermia variabilis |
| 18164 | GJB5 | NM_005268.2 | 245 | aaggacttcgactgcaatactcg | 263804 | - | | connexon channel | |
| 18165 | GJE1 | NM_181538.1 | 254 | ctgtacccagcgccctctatatg | 263811 | - | | | |
| 18166 | AQP4 | NM_001650.3 | 599 | atgtcactggctcaatagcttta | 263824 | - | see also 1093 line | | |
| 18167 | AQP5 | NM_001651.1 | 1218 | gagcgtgtggccatcatcaaagg | 263845 | - | | water channel | |
| 18168 | AQP6 | NM_001652.2 | 381 | ttgggcgtgcaggctttggaaagc | 263846 | - | | | |
| 18169 | AQP8 | NM_001169.1 | 803 | tgtttgactgctcattaggtgc | 263859 | - | | water channel | |
| 18170 | AQP9 | NM_020980.2 | 377 | ttgggcacgttcatcttgattgt | 263862 | - | | water channel | |
| 18171 | SLC14A1 | NM_015865.1 | 772 | ctggtcatacctataactacagc | 263887 | - | | urea transporter | urea transport defect, diabetes |
| 18172 | AQP1 | NM_000385.3 | 821 | tggatgccgacgacatcaactcc | 263908 | - | | | |
| 18173 | AQP10 | NM_080429.2 | 223 | tggctctctggccgttacgatag | 263909 | - | | transporter | |
| 18174 | FLJ20130 | NM_017681.1 | 445 | ttgggaccatacattgattgaga | 263930 | - | | | |
| 18175 | LOC129401 | NM_138285.2 | 721 | atgcatattcgttatcaatctaa | 263958 | - | | | |
| 18176 | NUP214 | NM_005085.2 | 1198 | atgggagttgctgtagactatac | 264002 | - | | | leukemia |
| 18177 | NUP50 | NM_007172.2 | 1256 | gagcgacaagtgcctcatttaat | 264090 | - | | | |

Figure 46

| | | | | | | | | |
|---|---|---|---|---|---|---|---|---|
| 18178 | NUP54 | NM_017426.2 | 662 | tagttgcatgcccagtaataaag | 264101 | - | | |
| 18179 | PCNT1 | NM_024844.2 | 1494 | atgaaagccgtccgcaacaatcg | 264189 | - | | |
| 18180 | SYNPR | NM_144642.3 | 509 | aggactgtctgacgtcaaagttg | 264205 | - | | |
| 18181 | MAL | NM_002371.2 | 484 | atgcggttctctttaatcaga | 264222 | - | signal_transduction | leukemia |
| 18182 | STEAP | NM_012449.2 | 992 | gggcacaatacacgcattgattt | 264226 | - | channel/pore class transporter | Ewing's sarcoma |
| 18183 | ATP6V0A2 | NM_012463.1 | 187 | tagagcggaatattggtgtattg | 264237 | | hydrogen-transporting two-sector ATPase | |
| 18184 | C10orf26 | NM_017787.2 | 885 | aggttgactcgggcattgaagtgt | 264291 | - | | |
| 18185 | C14orf4 | NM_024496.2 | 3230 | gggcgaaatcgcgactatcttag | 264310 | - | | |
| 18186 | C14orf52 | NM_145165.1 | 389 | ctgatgaccccgacaaatgact | 264328 | - | transcription_regulator | |
| 18187 | C5orf12 | NM_178276.2 | 728 | ctggtgacgctcatcatgtattc | 264338 | - | | |
| 18188 | CYC1 | NM_001916.2 | 335 | cggaggggtttccaggtatataa | 264400 | - | electron transporter | |
| 18189 | DEGS | NM_003676.2 | 120 | ccgcgtctcgcgggaagacttcg | 264408 | - | | |
| 18190 | DISP1 | NM_032890.2 | 4362 | gtgttgcgaccccgagaataaac | 264553 | - | see also 9337 line | |
| 18191 | DKFZp434F054 | NM_032259.1 | 965 | gcgcaagccttcgcttaacctga | 264565 | - | | |
| 18192 | DKFZP564D0478 | NM_032125.1 | 444 | cagcaccaactggaatggtga | 264583 | - | see also 9078 line | |
| 18193 | EHZF | NM_015461.1 | 316 | ttgaatcgctgagcgatatcaca | 264590 | - | see also 6424 line | |
| 18194 | ERdj5 | NM_018981.1 | 2635 | cagggcctatccaacttaaat | 264753 | - | see also 7636 line | |
| 18195 | ETFA | NM_000126.1 | 58 | cagagtaccctggtaatagctga | 264763 | - | see also 99 line | |
| 18196 | FBLP-1 | NM_017556.1 | 951 | ccggcagaacctgttgagaaagg | 264785 | - | | |
| 18197 | FLJ10900 | NM_018264.1 | 2073 | atgtgaacacgaacactctaatt | 264794 | - | | |
| 18198 | FLJ12519 | NM_032168.1 | 95 | gttgccgcagcgagttgaacttt | 264797 | - | | |
| 18199 | FLJ14627 | NM_032814.1 | 1268 | cggggtctgatcgttctctaca | 264874 | - | | |
| 18200 | FLJ14768 | NM_032836.1 | 783 | gcgctgcgagcgcgcgacttcaacg | 264877 | - | | |
| 18201 | FLJ20095 | NM_017666.1 | 2230 | tagatcgtatggctaaacactta | 264937 | - | | |
| 18202 | FLJ20511 | NM_017853.1 | 401 | ctgcagctagatgatattcttc | 264947 | - | | |
| 18203 | FLJ22625 | NM_024715.2 | 1726 | gagagtattcggtggctaattcc | 264976 | - | | |
| 18204 | FLJ23537 | NM_024889.2 | 443 | ctgttgtgcaattgtctatatta | 264988 | - | | |
| 18205 | FLJ25369 | NM_152670.1 | 630 | tagtcaaggtcccatcttgaac | 265003 | - | | |
| 18206 | FLJ31384 | NM_152472.1 | 1138 | tggtaaggctcacaatcactga | 265009 | - | | |
| 18207 | FLJ40417 | NM_173650.1 | 593 | cggtatcatcccgacaagaatcc | 265016 | - | | |
| 18208 | GLRX | NM_002064.1 | 255 | aggcggatgcagtgatctagtct | 265033 | - | redox-active disulfide bond electron carrier | |

Figure 46

| 18209 | GLRX2 | NM_016066.3 | 654 | cagatccaagaaacaatttctga | 265038 | - | apoptosis、transcriptio n_regulator、signal_tr ansduction | - | - |
|---|---|---|---|---|---|---|---|---|---|
| 18210 | HCA127 | NM_018684.1 | 214 | aaggcacctgaaggaatacaagc | 265043 | - | - | - | - |
| 18211 | HECA | NM_016217.1 | 1545 | aagcccgtcgagacatgatgaga | 265060 | - | cell_cycle | - | - |
| 18212 | KIAA0528 | NM_014802.1 | 949 | acggcgtgtactctggataaatt | 265093 | - | see also 5996 line | | |
| 18213 | KIAA1441 | NM_020832.1 | 3123 | aggcgccatgtcagagttaatca | 265189 | - | see also 7985 line | | |
| 18214 | KIAA1573 | NM_020925.1 | 3443 | aagtccctacgttgatgacatgg | 265247 | - | see also 8033 line | | |
| 18215 | LOC152485 | NM_178835.2 | 453 | gtggagtcccccgtaaacgttgg | 265264 | - | - | - | - |
| 18216 | LOC204474 | NM_174924.1 | 1201 | ccgggtcacagaggtcgatatcc | 265320 | - | - | - | - |
| 18217 | LOC51061 | NM_015914.2 | 766 | gagtactcgggtactttgagttc | 265353 | - | see also 6568 line | | |
| 18218 | LOC51066 | NM_015931.1 | 722 | atgggcgtgggcggtacaagtgc | 265392 | - | - | - | - |
| 18219 | LOC51257 | NM_016496.3 | 869 | gtgctgcgacatggtgtgtttcc | 265400 | - | - | - | - |
| 18220 | LOC90637 | NM_182491.1 | 653 | gggagtcgccccaccatcaaagc | 265409 | - | - | - | - |
| 18221 | MB | NM_005368.1 | 288 | tgggtggcatccttaagaagaag | 265413 | - | - | globin | - |
| 18222 | MGC14353 | NM_032731.2 | 351 | cagtgcctacactacttaagtat | 265424 | - | - | - | - |
| 18223 | MGC15482 | NM_032875.1 | 1358 | tagccttgagcggatagaactct | 265463 | - | - | - | - |
| 18224 | MGC33414 | NM_173574.1 | 1184 | cagaagagcttcactcaacttgc | 265473 | - | - | - | - |
| 18225 | MGC34648 | NM_152660.1 | 382 | aagtatggaccaccctattcttg | 265479 | - | see also 9910 line | | |
| 18226 | MGC48332 | NM_178450.2 | 803 | ctggaagccgtcggactgattgc | 265496 | - | see also 10189 line | | |
| 18227 | N33 | NM_006765.2 | 1216 | aagtaccacggctatccttatag | 265527 | - | see also 4771 line | | |
| 18228 | NCF2 | NM_000433.1 | 794 | gaggctcaccgtgtgctatttgg | 265543 | - | - | electron transporter | chronic granulomatous disease |
| 18229 | NCF4 | NM_000631.2 | 303 | aggatccaagtacctcatctacc | 265554 | - | - | - | - |
| 18230 | NSE1 | NM_145080.2 | 541 | acgcggtgaagatctgcaatatc | 265572 | - | see also 9775 line | | |
| 18231 | OSR1 | NM_145260.1 | 959 | gagaccacagatatattcactcc | 265580 | - | - | - | - |
| 18232 | OSR2 | NM_053001.1 | 1025 | ttgcggcagacactttaccaaat | 265585 | - | - | - | - |
| 18233 | PDC | NM_002597.3 | 552 | cagaataccctatagttaagttt | 265607 | - | signal_transduction、g pcr | - | - |
| 18234 | PGBD3 | NM_170753.1 | 1722 | ctgtttcgaactggtcttacaaa | 265673 | - | - | - | - |
| 18235 | PHTF2 | NM_020432.2 | 1961 | cagcgatcagttgatgtaatagt | 265728 | - | see also 7872 line | | |
| 18236 | QSCN6 | NM_002826.2 | 1786 | ctggaaagccggaattcaactct | 265774 | - | cell_cycle | - | - |

Figure 46

| | | | | | | | | | |
|---|---|---|---|---|---|---|---|---|---|
| 18237 | RNF34 | NM_025126.2 | 1206 | gcgagccgtgcacgtgttcaagt | 265808 | - | see also 8866 line | | |
| 18238 | SLAMF8 | NM_020125.1 | 466 | caggcccgtggtacaagtgttca | 265815 | - | - | - | - |
| 18239 | SYTL5 | NM_138780.1 | 1493 | atgatcgatttggacgtaatagc | 265862 | - | see also 9631 line | | |
| 18240 | TLP19 | NM_015913.2 | 496 | aagcccaaatttgcagaatctac | 265876 | - | see also 6567 line | | |
| 18241 | TXNDC | NM_030755.3 | 346 | ctgagtggacggtttatcataaa | 265890 | - | see also 8932 line | | |
| 18242 | TXNL2 | NM_006541.1 | 156 | aagttatggcagagttagctaaa | 265911 | - | see also 4587 line | | |
| 18243 | ZNF330 | NM_014487.2 | 454 | ttgcaatggtgggtgcaatatgt | 265946 | - | - | - | - |
| 18244 | ZNF363 | NM_015436.1 | 143 | ttgccgcttgtgtcatgataaca | 265957 | - | - | - | - |
| 18245 | ZNF451 | NM_015555.1 | 2967 | tggccgtctagatgaacaactac | 266068 | - | see also 6463 line | | |
| 18246 | ZNF507 | NM_014910.1 | 435 | aagaaacgcccacgttcaagtgc | 266078 | - | - | - | - |
| 18247 | ATP5F1 | NM_001688.2 | 334 | cggaactgggcttatcttgtacg | 266151 | - | - | hydrogen-transporting two-sector ATPase | - |
| 18248 | ATP5I | NM_007100.1 | 251 | aagatgacagcatattaaagtga | 266176 | - | - | hydrogen-transporting two-sector ATPase | - |
| 18249 | ATP5J2 | NM_004889.1 | 164 | gaggttactaccggtactacaac | 266182 | - | - | hydrogen-transporting two-sector ATPase | - |
| 18250 | ATP5S | NM_015684.1 | 957 | tcgacgccaccgactcttgtatc | 266193 | - | - | hydrogen ion transporter | - |
| 18251 | ATP6V0A1 | NM_005177.2 | 348 | atggatcgaaagcttcgatttgt | 266207 | - | see also 3641 line | | |
| 18252 | TCIRG1 | NM_006019.2 | 2149 | ctgcctgacgcatctgtgaatgg | 266265 | - | - | - | - |
| 18253 | TMEM1 | NM_003274.2 | 2765 | ctgcgtaccacgtgatcgaattt | 266319 | - | - | sodium transporter | - |
| 18254 | SLC11A2 | NM_000617.1 | 829 | ctgtcgcactccacagattgaac | 266364 | - | see also 415 line | | |
| 18255 | TTYH1 | NM_020659.1 | 345 | tggcattggcatcggtttctatg | 266388 | - | - | - | - |
| 18256 | SLC31A2 | NM_001860.1 | 398 | tggccgtaatgtcctacaacacc | 266395 | - | - | copper ion transporter | - |
| 18257 | SLC30A3 | NM_003459.3 | 1143 | gtgggcccttacgctcacttacc | 266397 | - | - | zinc porter | - |
| 18258 | SLC30A4 | NM_013309.3 | 520 | caggccgacgatgattccttact | 266406 | - | see also 5391 line | | |
| 18259 | SLC39A2 | NM_014579.1 | 770 | atgcggctagtgcatttaggtac | 266444 | - | - | - | - |
| 18260 | RHAG | NM_000324.1 | 882 | ttgtgcggatatggcaattcacc | 266468 | - | - | ammonium transporter | Rh deficiency syndrome |
| 18261 | RHBG | NM_020407.1 | 782 | tcggacggccctcaacacatact | 266485 | - | - | - | - |
| 18262 | RHCG | NM_016321.1 | 343 | cagggctggttccacttcttaca | 266494 | - | - | - | - |
| 18263 | SLC22A1 | NM_003057.2 | 804 | aagaacggtggcgatcatgtacc | 266519 | - | - | - | - |
| 18264 | SLC22A13 | NM_004256.1 | 868 | aggatggacgaggcgatacaact | 266549 | - | - | - | - |

Figure 46

| | | | | | | | | | |
|---|---|---|---|---|---|---|---|---|---|
| 18265 | SLC22A14 | NM_004803.2 | 1106 | acgccgcaagtgtgaacaagaag | 266561 | - | - | - | - |
| 18266 | SLC22A2 | NM_003058.2 | 1621 | tggtctaccggctcactaacatc | 266593 | - | - | - | - |
| 18267 | SLC22A3 | NM_021977.2 | 1152 | agggggcaacctctatatagact | 266617 | - | see also 8237 line | | |
| 18268 | BA108L7.2 | NM_030971.2 | 1011 | cagcgttgaagtggtctactaca | 266642 | - | - | - | - |
| 18269 | DKFZP434 K0427 | NM_032148.2 | 1729 | aggaaaggacccggatagtttct | 266685 | - | see also 9084 line | | |
| 18270 | DKFZp547 M236 | NM_018713.1 | 1205 | ctgagtgaccacggacaaagtct | 266698 | - | - | - | - |
| 18271 | SFXN1 | NM_022754.4 | 1006 | ttgcgacgcgtgtacttcaataa | 266729 | - | - | cation transporter | - |
| 18272 | SFXN2 | NM_178858.3 | 720 | ctgcggctaactgtgtcaatatc | 266737 | - | - | - | - |
| 18273 | SFXN4 | NM_178867.2 | 409 | gcgggagccgttgcttcttcaac | 266750 | - | - | - | - |
| 18274 | SLC30A1 | NM_021194.1 | 1262 | ttgctagtgtaggctctaaatca | 266800 | - | see also 8132 line | | |
| 18275 | SLC30A2 | NM_032513.2 | 475 | cggagaagtcgttgagatcttgg | 266809 | - | - | - | - |
| 18276 | SLC30A5 | NM_022902.2 | 269 | acgtacccagcgctcgattaaca | 266814 | - | see also 8527 line | | |
| 18277 | SLC30A6 | NM_017964.2 | 185 | atgtggtgcagttctactaatag | 266896 | - | - | - | - |
| 18278 | SLC30A8 | NM_173851.1 | 559 | gcggccaacattgtactaactgt | 266932 | - | - | - | - |
| 18279 | SLC41A1 | NM_173854.3 | 1922 | ctgtcttcacgcctgtgattaat | 266958 | - | - | - | - |
| 18280 | MRS2L | NM_020662.1 | 611 | gagagggtcaactcgttacatac | 266973 | - | - | - | - |
| 18281 | SLC39A11 | NM_139177.2 | 171 | cagctctcgtgttcgtattctct | 266995 | - | - | - | - |
| 18282 | SLC39A12 | NM_152725.1 | 1714 | aagccattagcttgttagcaatc | 267036 | - | - | - | - |
| 18283 | SLC39A13 | NM_152264.2 | 1108 | ctgtgcgggcatcgtggtaatgg | 267050 | - | - | - | - |
| 18284 | SLC39A3 | NM_144564.3 | 567 | gggcagcgactcggagtatgaga | 267056 | - | - | - | - |
| 18285 | SLC39A4 | NM_017767.1 | 1736 | gggcgacgccgtgcacaacttcg | 267058 | - | - | - | - |
| 18286 | SLC39A5 | NM_173596.1 | 876 | tggggatgcactgctacatctgc | 267061 | - | - | - | - |
| 18287 | SLC39A6 | NM_012319.2 | 2273 | atgtagccgctgggggtatttct | 267118 | - | - | - | - |
| 18288 | SLC39A7 | NM_006979.1 | 815 | ccggcatcgctctctacttcaga | 267129 | - | see also 4873 line | | |
| 18289 | SLC39A8 | NM_022154.4 | 1378 | gggacgattgcctggatgataac | 267156 | - | - | - | - |
| 18290 | SLC39A9 | NM_018375.2 | 747 | ctggcttagagcggaatcgaatc | 267182 | - | - | - | - |
| 18291 | SLC22A6 | NM_004790.3 | 1634 | ctgtatactggggaactgtatcc | 267199 | - | see also 3325 line | | |
| 18292 | SLC22A8 | NM_004254.2 | 839 | tggcgttggctgcagttaactgt | 267206 | - | - | - | - |
| 18293 | SLCO1A2 | NM_005075.2 | 457 | ctgcatagacctataatgattgg | 267224 | - | - | - | - |
| 18294 | SLCO1B3 | NM_019844.1 | 107 | aagacgctgcaatggattcaaga | 267287 | - | - | - | - |
| 18295 | DKFZp761 H039 | NM_018711.1 | 1637 | ctgactctggcagtttacagtgg | 267339 | - | - | - | - |
| 18296 | PLP2 | NM_002668.1 | 157 | ctgtttgctgagattatattatg | 267341 | - | - | ion transporter | - |
| 18297 | SLC22A5 | NM_003060.2 | 1476 | ctggtacccccagacttgtatta | 267371 | - | - | ion transporter | carnitine deficiency |
| 18298 | AP1B1 | NM_001127.2 | 517 | ctgcatccgcgttgacaagatca | 267379 | - | see also 715 line | | |

Figure 46

| | | | | | | | | | | |
|---|---|---|---|---|---|---|---|---|---|---|
| 18299 | AP1G1 | NM_001128.4 | 2703 | atgcggatcaagcttacatataa | 267467 | - | see also 719 line | - | | - |
| 18300 | AP1G2 | NM_003917.2 | 1297 | cagtgacaggaacattaggtatg | 267476 | - | | - | | - |
| 18301 | AP1M1 | NM_032493.2 | 585 | cggtccgaaggcatcaagtatcg | 267494 | - | see also 9221 line | - | | - |
| 18302 | AP1M2 | NM_005498.3 | 1100 | acgtcgtgatttggagtattaag | 267525 | - | | - | protein transporter | - |
| 18303 | AP1S1 | NM_001283.2 | 30 | atgatgcggttcatgctattatt | 267532 | - | | - | | - |
| 18304 | AP1S3 | NM_178814.2 | 325 | gagctcttgacgctagagattgt | 267544 | - | | - | protein transporter | - |
| 18305 | AP2A1 | NM_014203.2 | 1594 | gttggtaccgtgtcacagatcg | 267554 | - | see also 5535 line | - | | - |
| 18306 | AP3B1 | NM_003664.3 | 1143 | gtgcgtttacttcgtagcaatag | 267596 | - | see also 2480 line | - | | - |
| 18307 | AP3B2 | NM_004644.3 | 1568 | cagagtcagtggtcgtcattaag | 267695 | - | | - | protein transporter | - |
| 18308 | AP3D1 | NM_003938.4 | 2373 | ctgcctatgtcagatcagtatgt | 267732 | - | see also 2695 line | - | | - |
| 18309 | AP3M1 | NM_012095.2 | 570 | gcgtccggcagggttaaagtaca | 267768 | - | | - | protein transporter | - |
| 18310 | AP3M2 | NM_006803.2 | 159 | gtggtcagccgttcgttcgttgtga | 267794 | - | see also 4783 line | - | | - |
| 18311 | AP3S1 | NM_001284.1 | 491 | gagattgttacacaaattgatgc | 267839 | - | signal_transduction | - | | - |
| 18312 | AP3S2 | NM_005829.3 | 533 | cagagattcctcggaacatcaac | 267856 | - | | - | | - |
| 18313 | APBA1 | NM_001163.2 | 2574 | tgggcaccggatcattgaaatc | 267880 | - | see also 742 line | - | | - |
| 18314 | ARCN1 | NM_001655.3 | 1222 | gtgactaggggtgtcaaagtgg | 267908 | - | | - | protein transporter | - |
| 18315 | BCAP29 | NM_018844.1 | 407 | ctggttacgcttattactcaact | 267922 | - | see also 7600 line | - | | - |
| 18316 | BCAP31 | NM_005745.5 | 335 | gtgcgcgaaattcggaagtatga | 267932 | - | see also 3995 line | - | | - |
| 18317 | C14orf163 | NM_016106.2 | 1151 | ctgacaataccgctaagctaaca | 267976 | - | | - | | - |
| 18318 | C20orf161 | NM_033421.2 | 596 | tcgccgttactcggactttgagc | 268006 | - | | - | | - |
| 18319 | CHS1 | NM_000081.1 | 8421 | tgggagactactagtgcatattt | 268252 | - | see also 51 line | - | | - |
| 18320 | COG1 | NM_018714.1 | 1500 | gtggcaaaccgggtcagtttgc | 268385 | - | see also 7575 line | - | | - |
| 18321 | COG2 | NM_007357.1 | 753 | cagacgtctgacgtcgatataat | 268428 | - | | - | protein transporter | - |
| 18322 | COG3 | NM_031431.2 | 1557 | ctggcatatcccgataagttagt | 268515 | - | see also 9018 line | - | | - |
| 18323 | COG4 | NM_015386.1 | 2163 | ctggaccatccgagacaagtttg | 268583 | - | see also 6403 line | - | | - |
| 18324 | COG5 | NM_006348.2 | 1807 | aagttgcaccaatcagtaacaaa | 268648 | - | | - | | - |
| 18325 | COG7 | NM_153603.1 | 931 | cagcttaccgactctatgatgc | 268779 | - | | - | | - |
| 18326 | COG8 | NM_032382.2 | 1459 | atgcccttgccaaggtaactaaa | 268813 | - | | - | | - |
| 18327 | COPB | NM_016451.3 | 3063 | ctgctgttaccggccatataaga | 268902 | - | see also 6862 line | - | | - |
| 18328 | COPE | NM_007263.3 | 152 | aggcgcagcgcgggtgaagctatca | 268910 | - | | - | protein transporter | - |
| 18329 | COPG | NM_016128.3 | 717 | atgaaccggctagccgtcaattag | 268930 | - | | - | protein transporter | - |
| 18330 | COPG2 | NM_012133.1 | 1190 | tggttggttacaggcaattagt | 268968 | - | | - | | - |
| 18331 | COPZ1 | NM_016057.1 | 462 | tggtacaccggtggccattaagg | 268999 | - | | - | protein transporter | - |
| 18332 | COPZ2 | NM_016429.1 | 352 | gggtcatcctacgagaatgagc | 269004 | - | see also 7733 line | - | protein transporter | - |
| 18333 | EIF4ENIF1 | NM_019843.2 | 1181 | cagaactcggggaattactttgc | 269030 | - | signal_transduction | - | | - |
| 18334 | EPIM | NM_001980.2 | 493 | cagtgatcttcggatacgaaga | 269075 | - | | - | protein transporter | - |
| 18335 | EXO70 | NM_015219.1 | 1383 | gcgcggctgcaagcactcatatct | 269110 | - | | - | | - |

Figure 46

| | | | | | | | | | |
|---|---|---|---|---|---|---|---|---|---|
| 18336 | GGA1 | NM_013365.2 | 43 | ctggaggcgcgaatcaatagagc | 269121 | - | - | protein transporter | - |
| 18337 | GOSR2 | NM_004287.2 | 406 | tggacgaatcactgcagtttaac | 269138 | - | - | - | - |
| 18338 | IPO11 | NM_016338.3 | 1096 | aggcgttacatttgaacgattca | 269166 | - | see also 6816 line | | |
| 18339 | IPO13 | NM_014652.1 | 1662 | ctggcactcgtcaacatgattat | 269238 | - | see also 5824 line | | |
| 18340 | IPO9 | NM_018085.3 | 1759 | ctgtgcatcgtttgtacagtaga | 269294 | - | - | - | - |
| 18341 | KPNA4 | NM_002268.3 | 1595 | tagtactcgatggactaagtaat | 269352 | - | see also 1456 line | | |
| 18342 | KPNA5 | NM_002269.2 | 207 | gagatgcgtagacgaagagaaga | 269364 | - | - | protein transporter | - |
| 18343 | KPNA6 | NM_012316.3 | 675 | ttgccgagattacgtcttgaact | 269415 | - | see also 5272 line | | |
| 18344 | MTX1 | NM_002455.2 | 632 | ctgacctatgccagatttactgg | 269435 | - | - | protein transporter | - |
| 18345 | MTX2 | NM_006554.1 | 435 | atgtgggaaatcaagtagtatca | 269451 | - | see also 4592 line | | |
| 18346 | NAPG | NM_003826.1 | 428 | gagcgagctggaaagcttataga | 269493 | - | - | lyase | - |
| 18347 | Nup37 | NM_024057.2 | 427 | agggccataccgatttcattaat | 269528 | - | see also 8580 line | | |
| 18348 | NUP98 | NM_005387.3 | 1270 | ttggcaataacaagcttactaca | 269566 | - | see also 3753 line | | |
| 18349 | NUTF2 | NM_005796.1 | 457 | aggctcgccctgcacaactttgg | 269693 | - | - | protein transporter | - |
| 18350 | NXT2 | NM_018698.3 | 389 | tggacaaggccaccttaatatgg | 269700 | - | - | protein transporter | - |
| 18351 | PEX13 | NM_002618.1 | 624 | cagcggatgttaggtttaagaag | 269731 | - | see also 1696 line | | |
| 18352 | PIGR | NM_002644.2 | 2183 | acgtcgaccgagtttcaatcaga | 269785 | - | - | protein transporter | - |
| 18353 | SCAMP1 | NM_004866.3 | 448 | tcgtcgggaacgagaaatgcaaa | 269803 | - | - | - | - |
| 18354 | SCAMP2 | NM_005697.3 | 752 | ttgtcaaatagggatctacatca | 269844 | - | - | protein transporter | - |
| 18355 | SCAMP3 | NM_005698.2 | 713 | atggagatcccccaagaatttca | 269852 | - | - | - | - |
| 18356 | SEC10L1 | NM_006544.2 | 1146 | aagtccgatgcagagcaatatct | 269880 | - | - | - | - |
| 18357 | SEC13L1 | NM_030673.2 | 890 | atgatgcctcaagcaatacgtgg | 269912 | - | - | - | - |
| 18358 | SEC15L1 | NM_019053.2 | 181 | ttgtatccgtaatcatgacaagg | 269918 | - | see also 7677 line | | |
| 18359 | SEC23A | NM_006364.2 | 2143 | tagcattcttgcagatcgtattc | 270035 | - | see also 4461 line | | |
| 18360 | SEC23B | NM_006363.3 | 1901 | ctgatgagtcgtcatattacaga | 270091 | - | see also 4458 line | | |
| 18361 | SEC24B | NM_006323.1 | 3434 | acggctggatgatcgtgtgtatatg | 270221 | - | - | protein transporter | - |
| 18362 | SEC24D | NM_014822.1 | 219 | gtggctacacctccgtattctca | 270236 | - | - | protein transporter | - |
| 18363 | SEC3 | NM_018261.2 | 2699 | atgaatttatacgccagtataag | 270363 | - | - | - | - |
| 18364 | SEC6 | NM_007277.3 | 1759 | atgacgaagaagtggctattagg | 270463 | - | - | - | - |
| 18365 | SEC63 | NM_007214.2 | 927 | aagcagaccaacggataatattc | 270494 | - | see also 5021 line | | |
| 18366 | SNAG1 | NM_052870.1 | 1823 | tagggaatgagtactctttctcg | 270516 | - | - | protein transporter | - |
| 18367 | SNAP23 | NM_003825.2 | 122 | cagagagctcaccagattactga | 270519 | - | - | - | - |
| 18368 | SNAP29 | NM_004782.2 | 692 | tcgagcctatcaccagaagatcg | 270536 | - | receptor_acitivity | protein transporter | - |
| 18369 | SNX1 | NM_003099.2 | 1381 | gagtctcgggtgactcaatatga | 270560 | - | - | - | - |
| 18370 | SNX10 | NM_013322.2 | 317 | atgtattcatactaatagcatgt | 270570 | - | - | protein transporter | - |
| 18371 | SNX11 | NM_013323.2 | 713 | atgccattcttcgatatgctatg | 270576 | - | - | - | - |
| 18372 | SNX12 | NM_013346.2 | 324 | gagagatagcaagattgtagtac | 270587 | - | - | protein transporter | - |

Figure 46

| | | | | | | | | |
|---|---|---|---|---|---|---|---|---|
| 18373 | SNX15 | NM_013306.2 | 657 | gggctacaccgagtacaagtaa | 270591 | - | - | - |
| 18374 | SNX16 | NM_022133.2 | 1020 | caggggcattctgtgaaactttag | 270618 | - | see also 8298 line | - |
| 18375 | SNX19 | NM_014758.1 | 1221 | tcgtaccggacgccatgtagtgg | 270638 | - | - | protein transporter |
| 18376 | SNX2 | NM_003100.2 | 401 | atgtctgctcccgtgatctttga | 270685 | - | see also 2081 line | - |
| 18377 | SNX24 | NM_014035.1 | 106 | gcgcgggatacacggtgtttaaga | 270714 | - | see also 5466 line | - |
| 18378 | SNX26 | NM_052948.2 | 368 | ccggttctccggagttacgatga | 270727 | - | - | protein transporter |
| 18379 | SNX27 | NM_030918.4 | 456 | acgactcgttgggacaatcatt | 270746 | - | - | protein transporter |
| 18380 | SNX4 | NM_003794.2 | 494 | tggagaggcgacggattggttta | 270808 | - | - | protein transporter |
| 18381 | SNX5 | NM_014426.2 | 554 | cagtaaagatgcaacttcatg | 270848 | - | see also 5673 line | - |
| 18382 | SNX8 | NM_013321.1 | 356 | tcggtatacagacggtacaatga | 270856 | - | - | protein transporter |
| 18383 | STX10 | NM_003765.1 | 348 | gaggaataacagagagatactcg | 270875 | - | - | protein transporter |
| 18384 | STX11 | NM_003764.2 | 202 | ctggactgtccaagcaatatga | 270877 | - | receptor activity | protein transporter |
| 18385 | STX12 | NM_177424.1 | 689 | gggtgatctgattgatagacatag | 270890 | - | see also 10162 line | - |
| 18386 | STX16 | NM_003763.2 | 909 | aaggacttaggggcgatgattgt | 270907 | - | - | t-SNARE |
| 18387 | STX18 | NM_016930.2 | 930 | cagcattcaccagttagttgtgg | 270931 | - | - | protein transporter |
| 18388 | STX1A | NM_004603.1 | 710 | gttggaacacgcggtagactagt | 270936 | - | - | protein transporter |
| 18389 | STX3A | NM_004177.3 | 638 | cagcggcagctcgaaattactgg | 270948 | - | see also 2804 line | - |
| 18390 | STX4A | NM_004604.3 | 858 | cagcttgaacgcgattcgtga | 270970 | - | - | protein transporter |
| 18391 | STX5A | NM_003164.2 | 394 | ctgtccgacaacgcagtgaattc | 270974 | - | see also 2138 line | - |
| 18392 | STX7 | NM_003569.1 | 428 | gagtttgttgctcgagtaagagc | 270990 | - | - | protein transporter |
| 18393 | STXBP1 | NM_003165.1 | 271 | atgaccgagggcataacgattgt | 270998 | - | - | - |
| 18394 | STXBP3 | NM_007269.1 | 921 | tagaattcgacatcgacatattg | 271058 | - | see also 5067 line | - |
| 18395 | SYBL1 | NM_005638.3 | 667 | aagaacctcaagctcactattat | 271113 | - | see also 3909 line | - |
| 18396 | TLOC1 | NM_003262.2 | 834 | cagggagtctgtgttgactact | 271125 | - | see also 2210 line | - |
| 18397 | TOM1 | NM_005488.1 | 947 | ctgcgccatgaaacggtttgaaacg | 271145 | - | - | - |
| 18398 | TOM1L1 | NM_005486.1 | 678 | gcgagtgatgtccgcccatattga | 271173 | - | - | - |
| 18399 | VDP | NM_003715.1 | 1258 | aagaccggcaattgtagtacttc | 271256 | - | see also 2538 line | - |
| 18400 | VPS18 | NM_080432.1 | 884 | ccgcccattacgttgctaaatg | 271311 | - | see also 9514 line | - |
| 18401 | VPS33A | NM_022916.2 | 1674 | gggcgtaaccttcgctgaaattg | 271372 | - | - | - |
| 18402 | VPS39 | NM_015289.1 | 2726 | tggagttggtcgtccattacttct | 271471 | - | - | - |
| 18403 | VPS45A | NM_007259.2 | 501 | gggatccagccagctatctaga | 271488 | - | see also 5062 line | - |
| 18404 | VTI1A | NM_145206.1 | 458 | gagggcacatctgctcgataaaca | 271526 | - | - | - |
| 18405 | XPO1 | NM_003400.2 | 2466 | aagttaggggggacatataacagc | 271622 | - | see also 2294 line | - |
| 18406 | XPO4 | NM_022459.3 | 2272 | cagcgattcttaagagtgataaa | 271721 | - | see also 8382 line | - |
| 18407 | CTNS | NM_004937.1 | 699 | cagcgccattagcatcatcataaacc | 271784 | - | see also 3433 line | - |
| 18408 | XK | NM_021083.2 | 536 | ctgacctacagctgtacatag | 271797 | - | see also 8082 line | - |
| 18409 | PITPNC1 | NM_012417.2 | 1320 | aagccatgtcggcccaaatctga | 271841 | - | signal_transduction | - |

743

Figure 46

| 18410 | APOA2 | NM_001643.1 | 294 | ctggaacggaactggttaacttc | 271852 | - | - | Hepatocyte nuclear factor 1 | familial hypercholesterolemia、apolipoprotein A-II deficiency |
|---|---|---|---|---|---|---|---|---|---|
| 18411 | APOC3 | NM_000040.1 | 268 | gagcaccgttaaggacaagttct | 271857 | - | - | Nuclear factor NF-kappa-B p50 subunit | - |
| 18412 | APOC4 | NM_001646.1 | 235 | cagagacgggtggcaatggtct | 271859 | - | - | - | Alzheimer disease |
| 18413 | APOL1 | NM_003661.2 | 1207 | tagtctacctcgtgtacgaatca | 271879 | - | - | - | schizophrenia |
| 18414 | APOL3 | NM_014349.2 | 746 | aagaagcttagaacatatgcagc | 271881 | - | kinase_related | - | schizophrenia |
| 18415 | APOL6 | NM_030641.2 | 1198 | ggggtggggaaggatttaactgg | 271905 | - | - | C-ets-1 protein | schizophrenia |
| 18416 | FHR-4 | NM_006684.1 | 565 | gagtaatggcatgcggtttaagc | 271918 | - | - | - | - |
| 18417 | SAA4 | NM_006512.1 | 185 | gagcctattgggacataatgata | 271923 | - | - | lipid transporter | amyloidosis |
| 18418 | SLCO2A1 | NM_005630.1 | 1424 | ctgctcgtgcccagattctatct | 271951 | - | - | lipid transporter | - |
| 18419 | SLC18A1 | NM_003053.1 | 828 | tgggacctatactctactctttg | 271972 | - | see also 2047 line | | |
| 18420 | SLC18A2 | NM_003054.1 | 1514 | tggatcacaactgccctattaaa | 272008 | - | see also 2048 line | | |
| 18421 | CTL1 | NM_022109.2 | 1292 | cagtaaatcgccttattcgttac | 272051 | - | see also 8271 line | | |
| 18422 | SLC5A7 | NM_021815.2 | 385 | gagcgcagcgaagccatcatagt | 272074 | - | see also 8176 line | | |
| 18423 | SLC35B1 | NM_005827.1 | 594 | cggctatggagagctactcttgc | 272132 | - | - | - | - |
| 18424 | SLC35A5 | NM_017945.2 | 1103 | gaggagtaaccgtgatcagatta | 272166 | - | - | - | - |
| 18425 | SLC35B4 | NM_032826.2 | 489 | aaggtactatgccataatggtga | 272175 | - | see also 9294 line | | |
| 18426 | NUP107 | NM_020401.1 | 2340 | atgctatccgagaacatttgtgc | 272254 | - | - | nucleocytoplasmic transporter | - |
| 18427 | NUP155 | NM_004298.2 | 1666 | gggctactcgggctttctttagg | 272315 | - | - | - | - |
| 18428 | UPF3A | NM_023011.2 | 870 | aagtaaggattaagcttcttaag | 272412 | - | - | - | - |
| 18429 | UPF3B | NM_023010.2 | 234 | gtggtaattcgaagattacctcc | 272419 | - | - | - | - |
| 18430 | CHAC | NM_015186.1 | 6547 | aagatggttgggatttaccatac | 272653 | - | see also 6271 line | | |
| 18431 | KIAA1012 | NM_014939.1 | 1753 | ttggcaggccatcgatttagtaa | 272806 | - | see also 6121 line | | |
| 18432 | VPS41 | NM_014396.2 | 573 | ctgattgcttgggccaataatat | 272895 | - | see also 5661 line | | |
| 18433 | DEFA4 | NM_001925.1 | 161 | cagaagaccaggacatatctatt | 272938 | - | - | calcium channel regulator | - |
| 18434 | PLN | NM_002667.2 | 301 | atgtctcttgctgatctgtatca | 272945 | - | phosphatase | calcium channel regulator | - |
| 18435 | CLNS1A | NM_001293.1 | 746 | gtggataccacaccaacagttgc | 272953 | - | - | auxiliary transport protein | - |
| 18436 | CYGB | NM_134268.3 | 514 | tggaaccggtgtacttcaagatc | 272962 | - | - | globin | - |
| 18437 | HBB | NM_000518.4 | 386 | ctgtgtgctggcccatcactttg | 272964 | - | - | specific RNA polymerase II transcription factor | - |
| 18438 | HBE1 | NM_005330.3 | 573 | tgggtaacgtgatggtgattatt | 272969 | - | - | zinc binding | - |
| 18439 | HBQ1 | NM_005331.3 | 264 | ccgccacgaagacctacttctcc | 272971 | - | - | globin | - |

744

Figure 46

| | | | | | | | | | |
|---|---|---|---|---|---|---|---|---|---|
| 18440 | HBZ | NM_005332.2 | 68 | aagactgagagaccatcattgt | 272972 | - | - | - | Smad3/Sp-1 heterodimer |
| 18441 | NGB | NM_021257.2 | 574 | aaggtgatgctcgtgattgatgc | 272973 | - | - | - | |
| 18442 | SORT1 | NM_002959.3 | 1308 | aaggcgtgctccgaagataatt | 273007 | - | see also 1996 line | - | |
| 18443 | TETRAN | NM_001120.2 | 493 | atgggtgtggccacctcatatgc | 273039 | - | - | - | |
| 18444 | SLC29A1 | NM_004955.1 | 614 | aagatgcgtcattaattcatt | 273048 | - | see also 3445 line | - | |
| 18445 | SLC29A2 | NM_001532.2 | 862 | gagcctgcctcacctgaagtttg | 273057 | - | - | - | nucleoside transporter |
| 18446 | SLC33A1 | NM_004733.2 | 770 | atgcggtctacgttaagaacttc | 273068 | - | see also 3250 line | - | |
| 18447 | AP2B1 | NM_001282.1 | 674 | gggatccatagcagattcaaat | 273113 | - | - | - | |
| 18448 | AP2M1 | NM_004068.2 | 618 | gcggcgagagggtatcaagtatc | 273148 | - | - | - | |
| 18449 | AP2S1 | NM_004069.2 | 189 | ccgtccgagacgccaaacacacc | 273162 | - | - | - | |
| 18450 | AP4B1 | NM_006594.1 | 588 | ctgcgggataaggcttcatatgt | 273180 | - | see also 4625 line | - | |
| 18451 | AP4M1 | NM_004722.2 | 897 | aggaatccgggtcgatgaagtct | 273247 | - | - | - | |
| 18452 | AP4S1 | NM_007077.2 | 457 | cagccgagtgagtgaattagatg | 273267 | - | - | - | |
| 18453 | AQP3 | NM_004925.3 | 560 | aggcacagcctccttatcgtgt | 273280 | - | - | - | transporter |
| 18454 | C16orf7 | NM_004913.1 | 139 | ctggggtatctcagagataagc | 273282 | - | transcription_regulator | - | |
| 18455 | C20orf121 | NM_024331.2 | 379 | ccgagccgcaagtttgattacg | 273289 | - | see also 8611 line | - | |
| 18456 | C20orf26 | NM_015585.2 | 2000 | aaggatccgatgagttatgcttt | 273355 | - | - | - | |
| 18457 | C20orf59 | NM_022082.2 | 1347 | gtgtctagcggctacttgatgt | 273408 | - | - | - | |
| 18458 | CLCA3 | NM_004921.1 | 576 | atgttccactcgtattactgtt | 273428 | - | - | - | |
| 18459 | CPNE7 | NM_014427.3 | 439 | ctgtcaggaggacgatttcctgg | 273435 | - | - | - | |
| 18460 | EVER1 | NM_007267.5 | 1841 | gggaactggtgtggaggattatc | 273500 | - | - | - | |
| 18461 | FLJ14153 | NM_022736.1 | 609 | atggctgtattctaagattgaag | 273523 | - | see also 8442 line | - | |
| 18462 | FLJ25217 | NM_152351.2 | 1020 | gtgtccgtctctcgaacagatga | 273538 | - | - | - | |
| 18463 | FLJ40121 | NM_175904.2 | 1203 | ctgagggctcccacaagtcatcc | 273573 | - | - | - | |
| 18464 | GASP | NM_014710.1 | 2037 | gagcgtattgtgattccttatt | 273619 | - | - | - | |
| 18465 | GOLGA3 | NM_005895.2 | 689 | cagtgtgccgaggtaaacagagc | 273653 | - | see also 4098 line | - | |
| 18466 | HIATL2 | NM_032318.1 | 410 | atgttgactgttcacatgaaac | 273689 | - | - | - | |
| 18467 | JUB | NM_032876.4 | 1829 | atggaccgggattatcactttga | 273703 | - | see also 9330 line | - | |
| 18468 | LCN2 | NM_005564.2 | 254 | tggcagggaatgcaattctcaga | 273705 | - | - | - | transporter |
| 18469 | LGI3 | NM_139278.1 | 1574 | gggcaagccacggctgattgtgt | 273730 | - | - | - | |
| 18470 | LOC127262 | NM_182752.2 | 363 | gagttgatcactggaacaatga | 273736 | - | - | - | |
| 18471 | LOC136306 | NM_174959.1 | 724 | aagacgctgagcctttctatta | 273759 | - | - | - | |

Figure 46

| | | | | | | | | | |
|---|---|---|---|---|---|---|---|---|---|
| 18472 | LOC138307 | NM_178469.2 | 536 | aagatcgtgggctcagaaataga | 273772 | - | - | - | - |
| 18473 | LOC143425 | NM_175733.2 | 1171 | aggttctctcgtcatgacttaat | 273794 | - | - | - | - |
| 18474 | MGC11308 | NM_032889.2 | 629 | cagccttcgaggcctggtatatc | 273815 | - | - | - | - |
| 18475 | MGC34646 | NM_173519.1 | 811 | aagatccggagcttcagataaat | 273884 | - | - | - | - |
| 18476 | MGC34821 | NM_173586.1 | 948 | gggttggacggaagatcatatgc | 273906 | - | - | - | - |
| 18477 | MGC52019 | NM_178498.2 | 941 | atggatctcgactacatatattt | 273936 | - | - | - | - |
| 18478 | MGC9564 | NM_080669.2 | 751 | ctgatagccatgactctctatgc | 273957 | - | see also 9520 line | | |
| 18479 | MIP | NM_012064.1 | 720 | ccggctcaagagtatttctgaga | 273970 | - | see also 5103 line | | |
| 18480 | MTAC2D1 | NM_152332.2 | 1058 | aaggttccaacgctattgaattt | 274002 | - | - | - | - |
| 18481 | NOD3 | NM_178844.1 | 1600 | gtggcagccgcgtattactatgg | 274022 | - | - | - | - |
| 18482 | NUP88 | NM_002532.3 | 708 | agggcgtataccgcatctctagg | 274058 | - | see also 1630 line | | |
| 18483 | ORM1 | NM_000607.1 | 338 | aggaccagtgcatctataacacc | 274098 | - | - | transporter | - |
| 18484 | PACSIN2 | NM_007229.1 | 1399 | ctggtcagacgatgagtctaaca | 274101 | - | - | - | - |
| 18485 | PDZK1 | NM_002614.3 | 157 | tggcttcttcctgcgaattgaga | 274104 | - | see also 1690 line | | |
| 18486 | PPP1R9B | NM_032595.1 | 1764 | gagcgaagtggcccagctaattc | 274126 | - | phosphatase、cell_cycle | | |
| 18487 | SLC13A5 | NM_177550.2 | 1652 | ggggacgggccatatttgacttg | 274179 | - | - | - | - |
| 18488 | SLC15A3 | NM_016582.1 | 1839 | ttgccggatggacctctacttct | 274185 | - | - | - | - |
| 18489 | SLC17A6 | NM_020346.1 | 1615 | tggtcgttggctattctcatact | 274218 | - | - | - | - |
| 18490 | SLC17A8 | NM_139319.1 | 890 | tggggtgttggtgcagtacattg | 274247 | - | - | - | - |
| 18491 | SLC22A12 | NM_144585.2 | 953 | ctgaggccctcctggctatttcc | 274276 | - | - | - | - |
| 18492 | SLC22A15 | NM_018420.1 | 1570 | ttgccggagaccccttaacagtcc | 274312 | - | - | - | - |
| 18493 | SLC22A16 | NM_033125.2 | 1012 | cagggcaagctcctgtaaactgt | 274332 | - | - | - | - |
| 18494 | SLC22A17 | NM_016609.2 | 191 | gcgattcctacagcgaatgatca | 274352 | - | see also 6950 line | | |
| 18495 | SLC22A9 | NM_080866.1 | 745 | aggcatcctaggcggtcatttat | 274361 | - | - | - | - |
| 18496 | SLC23A3 | NM_144712.2 | 1008 | tgggcgaaatatcttcattgtgg | 274380 | - | - | - | - |
| 18497 | SLC37A3 | NM_032295.1 | 1218 | gtgtttatctcgccattaatagt | 274428 | - | see also 9147 line | | |
| 18498 | SLC5A8 | NM_145913.2 | 1559 | tggctgcgctggcgtcacttatg | 274462 | - | see also 9804 line | | |
| 18499 | SLCO1C1 | NM_017435.2 | 1189 | tggtggcttggctatctaatagc | 274508 | - | - | - | - |
| 18500 | SLCO2B1 | NM_007256.1 | 2173 | gagctgtctgtcgctactacaat | 274558 | - | - | transporter | - |
| 18501 | SLCO3A1 | NM_013272.2 | 700 | ccggacggctaccaacatgatgt | 274568 | - | see also 5383 line | | |
| 18502 | SLCO4A1 | NM_016354.3 | 979 | cagctgctcgcccgtctacattg | 274599 | - | - | - | - |

Figure 46

| 18503 | SLCO4C1 | NM_180991.4 | 360 | gcgtcgccctcccaaatcgaagt | 274621 | - | - | - | - |
|---|---|---|---|---|---|---|---|---|---|
| 18504 | SLCO5A1 | NM_030958.1 | 2073 | ttgtcctcggaggctacattata | 274722 | - | see also 8975 line | | |
| 18505 | SLCO6A1 | NM_173488.2 | 990 | gtgctaggagcaccactagttaa | 274785 | - | - | - | - |
| 18506 | SMBP | NM_020123.2 | 1915 | gtgtggagcgattggttacatgg | 274876 | - | see also 7758 line | | |
| 18507 | SPINL | NM_032038.1 | 489 | cggccgttcggctctcatagtgg | 274881 | - | - | - | - |
| 18508 | SV2A | NM_014849.1 | 1902 | gagcgcgtagagcatgtaacttt | 274906 | - | - | - | - |
| 18509 | SV2B | NM_014848.1 | 605 | aggaatgctagggatgatagtct | 274925 | - | - | - | - |
| 18510 | SYPL | NM_006754.2 | 493 | tggctacacgagtctgtatctgg | 274960 | - | - | - | - |
| 18511 | SYT11 | NM_152280.2 | 642 | aagaactaaggagccctattaca | 274988 | - | see also 9827 line | | |
| 18512 | SYT12 | NM_177963.2 | 662 | gagcagatcgtgggcatttctcg | 275002 | - | see also 10173 line | | |
| 18513 | SYT13 | NM_020826.1 | 913 | tggagaggtcctactatccatca | 275013 | - | see also 7984 line | | |
| 18514 | SYT14 | NM_153262.1 | 906 | tggcaagtacaccttgttcttct | 275044 | - | see also 9980 line | | |
| 18515 | SYT14L | NM_031914.1 | 750 | cagccaacggcgttattctgaga | 275088 | - | - | - | - |
| 18516 | SYT2 | NM_177402.3 | 1280 | acgaagccataggcaagatcttc | 275117 | - | - | - | - |
| 18517 | SYT3 | NM_032298.1 | 803 | tcggtcatcgtgacattctgtgg | 275118 | - | see also 9149 line | | |
| 18518 | SYT4 | NM_020783.2 | 1255 | aggtaatcgggcagttagtcttg | 275149 | - | see also 7967 line | | |
| 18519 | SYT7 | NM_004200.2 | 716 | aggtgtccatcccccttaacaag | 275156 | - | see also 2811 line | | |
| 18520 | SYTL4 | NM_080737.1 | 435 | aggattaggcgactaaagaatga | 275159 | - | see also 9521 line | | |
| 18521 | TCOF1 | NM_000356.1 | 2977 | gagtccagtcggatatcagatgg | 275206 | - | - | transporter | - |
| 18522 | TM9SF1 | NM_006405.3 | 131 | cggcgaccctgttattctgtatg | 275214 | - | see also 4487 line | | |
| 18523 | TM9SF2 | NM_004800.1 | 981 | gggcgtctagatgggactatatt | 275277 | - | see also 3332 line | | |
| 18524 | TM9SF4 | NM_014742.1 | 369 | ctggggtcgcgcctatcaacttc | 275326 | - | see also 5939 line | | |
| 18525 | CNOT2 | NM_014515.1 | 1536 | aagaacgagtatggattaccagg | 275420 | - | see also 5729 line | | |
| 18526 | GTF2B | NM_001514.2 | 580 | gtgccgtatcacgaatttctaag | 275439 | - | see also 1042 line | | |
| 18527 | GTF2E1 | NM_005513.1 | 94 | ttgaagcggttagccaagtatgt | 275452 | - | - | general RNA polymerase II transcription factor | - |
| 18528 | GTF2E2 | NM_002095.3 | 1051 | acgctttaagactcataacgaac | 275512 | - | see also 1358 line | | |
| 18529 | TAF1C | NM_005679.2 | 1175 | gtgccgtgtggaagtttggtaaa | 275525 | - | transcription_regulator | - | - |
| 18530 | BLZF1 | NM_003666.2 | 1051 | tagaacgtatgtcaatacagtgt | 275552 | - | see also 2491 line | | |
| 18531 | CEP1 | NM_007018.3 | 2154 | tagaggctcggctaaacctaagg | 275636 | - | see also 4897 line | | |
| 18532 | EAP30 | NM_007241.2 | 335 | gtgggggacttctattacgaact | 275737 | - | transcription_regulator | - | - |
| 18533 | ELL2 | NM_012081.3 | 1113 | aggacctctcatataccttaaag | 275768 | - | transcription_regulator | RNA polymerase II transcription factor | - |
| 18534 | LITAF | NM_004862.1 | 399 | atgaatcctccttcgtattatac | 275781 | - | transcription_regulator , kinase_related | RNA polymerase II transcription factor | - |

Figure 46

| 18535 | SUPT3H | NM_003599.1 | 372 | atgtttatccgagactacaaatc | 275804 | - | transcription_regulator, cell_cycle | translation regulator | - |
| 18536 | SURB7 | NM_004264.2 | 340 | ctggaggatgttgtttatcgagg | 275825 | - | transcription_regulator | RNA polymerase II transcription factor | - |
| 18537 | BTF | NM_014739.1 | 417 | gagattatcgtcgcgattacaga | 275912 | - | see also 5928 line | | |
| 18538 | NAB1 | NM_005966.2 | 1089 | aagaacttgcagctcttagttca | 276045 | - | transcription_regulator | transcriptional repressor | - |
| 18539 | REST | NM_005612.3 | 1004 | ccgctgcggctacaatactaatc | 276074 | - | see also 3900 line | | |
| 18540 | ELL | NM_006532.1 | 438 | gagccgaagtgccattgtcatca | 276140 | - | transcription_regulator | positive transcription elongation factor | - |
| 18541 | GTF3C3 | NM_012086.1 | 1235 | ctgatggcgtgccaatagatatc | 276182 | - | transcription_regulator | - | - |
| 18542 | GTF3C5 | NM_012087.1 | 1180 | gcgcagcctatggattcgatttg | 276257 | - | - | - | - |
| 18543 | 8D6A | NM_016579.1 | 555 | gagctcggctgtggaaccaatga | 276268 | - | - | - | - |
| 18544 | A1BG | NM_130786.2 | 825 | cagcccagatcgcatcttctttc | 276274 | - | - | molecular_function unknown | - |
| 18545 | A2LP | NM_007245.2 | 758 | atgttgacttcaactatgctact | 276293 | - | - | - | - |
| 18546 | AAAS | NM_015665.3 | 198 | ctggtgacgggcagtagctatga | 276322 | - | see also 6536 line | | |
| 18547 | ACRBP | NM_032489.2 | 872 | tagagtctactcctatgataatg | 276345 | - | - | - | - |
| 18548 | ACTL6 | NM_016188.3 | 1186 | gagcatgcgactgaaactcattg | 276369 | - | see also 6714 line | | |
| 18549 | AF1Q | NM_006818.2 | 363 | ctgtgagtagccagtacagttcc | 276371 | - | - | molecular_function unknown | - |
| 18550 | AMMECR1 | NM_015365.1 | 956 | aaggatgggaccatatacagacc | 276384 | - | see also 6397 line | | |
| 18551 | ANGPT4 | NM_015985.2 | 1576 | gcgtgcctctcgcatgatgatac | 276406 | - | kinase_related | transmembrane receptor protein tyrosine kinase activator | - |
| 18552 | ANK3 | NM_001149.2 | 1908 | ggggttagccacgcaactgtaca | 276445 | - | signal_transduction | - | - |
| 18553 | ANKRD2 | NM_020349.2 | 1236 | ctggaggggcctaatgatagtgg | 276778 | - | - | structural constituent of muscle | - |
| 18554 | ANUBL1 | NM_174890.1 | 514 | atgcgtggcggacctataaatac | 276790 | - | - | - | - |
| 18555 | AP2A2 | NM_012305.1 | 669 | caggacgtcccccgatcttgtcc | 276849 | - | - | structural molecule | - |
| 18556 | APOA1BP | NM_144772.1 | 261 | cagcgtggaccaacttatggaac | 276873 | - | - | - | - |
| 18557 | ARFIP1 | NM_014447.1 | 192 | ttgatgactctcgtgaacatagc | 276887 | - | - | molecular_function unknown | - |
| 18558 | ARPP-21 | NM_016300.3 | 448 | gagaacggcattgttaaatcaga | 276916 | - | see also 6792 line | | |
| 18559 | ARS2 | NM_015908.3 | 377 | gcgccgtagtcggggtgaatatc | 276970 | - | see also 6566 line | | |

# Figure 46

EP 1 752 536 A1

| | | | | | | | | | |
|---|---|---|---|---|---|---|---|---|---|
| 18560 | ASB1 | NM_016114.3 | 730 | gcgcgaaccctgacttcaactgc | 277008 | - | - | molecular_function unknown | |
| 18561 | ASPSCR1 | NM_024083.2 | 265 | gagcgtgctcgacctttctctcc | 277015 | - | - | - | renal cell carcinoma、 alveolar soft part sarcoma、 renal tumour、 soft tissue sarcoma |
| 18562 | AUTS2 | NM_015570.1 | 1114 | gggccgattgtccccaagatatc | 277030 | - | - | - | - |
| 18563 | B1 | NM_014451.2 | 2717 | ttgcctgtcgaagagttctaagg | 277130 | - | - | - | - |
| 18564 | B7 | NM_006992.1 | 485 | ctgcgaagtgcccagatgaatga | 277141 | - | - | - | - |
| 18565 | B7H3 | NM_025240.1 | 807 | gtgctggagaaagatcaaacaga | 277148 | - | - | - | - |
| 18566 | BAL | NM_031458.1 | 995 | tagagactatccgggttagtttg | 277167 | - | see also 9034 line | | |
| 18567 | BBS2 | NM_031885.2 | 2344 | gagtcgttatatggaactctatg | 277273 | - | - | molecular_function unknown | diabetes |
| 18568 | BC-2 | NM_014453.2 | 246 | ccgcgagcgacagaaactagaga | 277288 | - | - | - | - |
| 18569 | BCLP | NM_033504.2 | 554 | tcgaccccacacgcatttatagc | 277298 | - | kinase_related | - | - |
| 18570 | BENE | NM_005434.3 | 413 | tagccgccacccacatagtatac | 277300 | - | - | molecular_function unknown | - |
| 18571 | BICD1 | NM_001714.1 | 2524 | aagatcggcagccctaaagtaag | 277347 | - | see also 1141 line | | |
| 18572 | BK65A6.2 | NM_019601.2 | 1279 | atgtcctcagcttctattactgc | 277359 | - | - | - | - |
| 18573 | BM-002 | NM_016617.1 | 259 | ctgcggattattcctagagatcg | 277374 | - | - | molecular_function unknown | - |
| 18574 | BPESC1 | NM_021812.1 | 615 | cagtccttgggttgatttctttg | 277375 | - | - | molecular_function unknown | - |
| 18575 | BRD3 | NM_007371.2 | 324 | ccgtggacgcaatcaaattgaac | 277379 | - | - | molecular_function unknown | - |
| 18576 | BRMS1 | NM_015399.2 | 748 | tggcccatacatcgtgtacatgc | 277398 | - | cell_cycle | - | - |
| 18577 | BRP44L | NM_016098.1 | 368 | atgccacgcaacaaatgaagtag | 277404 | - | - | - | - |
| 18578 | BTN2A1 | NM_007049.2 | 1060 | aggagtttgaacgggaaacaaga | 277409 | - | - | - | - |
| 18579 | C11orf1 | NM_022761.1 | 182 | atggcgatgcaccactaatgaga | 277413 | - | see also 8456 line | | |
| 18580 | C11orf2 | NM_013265.2 | 2078 | aagctattctctgaacgtattga | 277428 | - | - | - | - |
| 18581 | C11ORF4 | NM_020155.2 | 1252 | ttgcctctcggtcctacttcttt | 277431 | - | - | - | - |
| 18582 | C14orf1 | NM_007176.1 | 442 | gggctccggtatctagaagtaga | 277449 | - | see also 4982 line | | |
| 18583 | C14orf123 | NM_014169.2 | 1057 | ttggcccaggagttgttaaatgt | 277464 | - | - | - | - |
| 18584 | C15orf2 | NM_018958.1 | 2884 | gagccagtcgagggtcacaatgc | 277501 | - | - | - | - |
| 18585 | C16orf35 | NM_012075.1 | 927 | gggcgtagttcggcttcacatca | 277521 | - | - | molecular_function unknown | - |
| 18586 | C16orf5 | NM_013399.1 | 676 | gtgctgcagggagagatctttga | 277533 | - | - | - | - |
| 18587 | C17orf1A | NM_031456.2 | 1384 | aagagcatgcgtgtgaaacatcc | 277564 | - | - | - | - |
| 18588 | C18orf1 | NM_004338.1 | 256 | tggagttcgcccaaatcatcatc | 277603 | - | - | molecular_function unknown | - |

Figure 46

| | | | | | | | | | |
|---|---|---|---|---|---|---|---|---|---|
| 18589 | C18orf2 | NM_031416.1 | 195 | atgacgcaagagacaattctaag | 277615 | - | - | - | - |
| 18590 | C1orf2 | NM_006589.2 | 1167 | ctgtacactctctgctattgtct | 277626 | - | - | molecular_function unknown | - |
| 18591 | C1orf24 | NM_022083.1 | 1579 | aaggttaaactccgagtcttaaa | 277645 | - | - | - | - |
| 18592 | C20orf10 | NM_014477.2 | 643 | aagccctaccgcaatagaactcc | 277695 | - | - | - | - |
| 18593 | C21orf45 | NM_018944.1 | 482 | atgcacgcccaagaatcttgatt | 277715 | - | - | molecular_function unknown | - |
| 18594 | C21orf5 | NM_005128.1 | 4833 | tagaaggtctaacgaccattagt | 277788 | - | - | molecular_function unknown | - |
| 18595 | C21orf62 | NM_019596.1 | 733 | gggactcagccttaaatcatata | 277846 | - | - | molecular_function unknown | - |
| 18596 | C22orf23 | NM_032561.2 | 471 | gagtcttaccttccaagcaaata | 277852 | - | - | molecular_function unknown | - |
| 18597 | C2F | NM_006331.3 | 269 | gtgacaagcacaagtctatattg | 277860 | - | - | molecular_function unknown | - |
| 18598 | C40 | NM_017546.3 | 723 | tggccgaacgccaatctgaattg | 277875 | - | see also 7025 line | | |
| 18599 | C6orf11 | NM_005452.4 | 375 | aagttctgtcgcattgacaaatc | 277918 | - | - | molecular_function unknown | - |
| 18600 | C6orf15 | NM_014070.1 | 525 | cagacgactgccccgttctaatt | 277942 | - | - | - | - |
| 18601 | C6orf53 | NM_016462.2 | 388 | tggtaccttggctggcattatgg | 277988 | - | - | - | - |
| 18602 | C6orf54 | NM_014354.1 | 1018 | gcggctgtacgcgtcatgatagc | 277990 | - | - | - | - |
| 18603 | C6orf67 | NM_018247.1 | 372 | ccgcgagatcgagattgattata | 277995 | - | see also 7344 line | | |
| 18604 | C7orf30 | NM_138446.1 | 611 | atggaccctacgttcttatgatg | 278056 | - | - | - | - |
| 18605 | C8orf1 | NM_004337.1 | 869 | tagctcgctactataaacattat | 278084 | - | see also 2901 line | | |
| 18606 | C8orf2 | NM_007175.4 | 1051 | aggggctagctgacaagctaagc | 278143 | - | - | - | Alzheimer disease |
| 18607 | C9orf16 | NM_024112.2 | 129 | cagaatacgctgccatcaactcc | 278145 | - | - | molecular_function unknown | - |
| 18608 | C9orf27 | NM_021208.1 | 333 | cagggatgtgacctatgtaatga | 278147 | - | - | - | - |
| 18609 | C9orf5 | NM_032012.1 | 2565 | gtgagtcccacgaattcagttcc | 278208 | - | - | - | - |
| 18610 | CDCA1 | NM_031423.2 | 547 | aagcatgccgtgaaacgtatatg | 278226 | - | see also 9017 line | | |
| 18611 | CDCA3 | NM_031299.3 | 639 | caggttctatgcgcaatagatgg | 278263 | - | - | - | - |
| 18612 | CDKAL1 | NM_017774.1 | 316 | tggggttgttctcataataattc | 278273 | - | - | - | - |
| 18613 | CENPH | NM_022909.3 | 456 | tagtgtgctcatggataacatga | 278313 | - | - | - | - |
| 18614 | CENPJ | NM_018451.2 | 4164 | cagatccggtcggataagagtta | 278395 | - | - | - | - |
| 18615 | CER1 | NM_005454.1 | 493 | gtgcccttcagccagactataac | 278401 | - | - | zinc binding | - |
| 18616 | CFDP1 | NM_006324.1 | 167 | ccgtcgggtggagagtatagtga | 278408 | - | - | - | - |
| 18617 | CG005 | NM_014887.1 | 476 | aagcacgtcctccattagtatcc | 278436 | - | - | - | - |
| 18618 | CGI-51 | NM_015380.3 | 535 | gagattaacgggcagttataaca | 278486 | - | see also 6402 line | | |

Figure 46

| | | | | | | | | | |
|---|---|---|---|---|---|---|---|---|---|
| 18619 | ch-TOG | NM_014756.1 | 1776 | gagcctgagctctcgatagaagt | 278551 | - | - | molecular_function unknown | |
| 18620 | CHIC2 | NM_012110.2 | 323 | tggcggatttcgacgaaatctat | 278673 | - | see also 5137 line | | |
| 18621 | CHORDC1 | NM_012124.1 | 175 | ccggtctttcacgatgcattaaa | 278687 | - | see also 5146 line | | |
| 18622 | CHRD | NM_003741.2 | 2177 | aggtgcacatagccaaccaatgt | 278698 | - | - | - | - |
| 18623 | COL6A1 | NM_001848.1 | 831 | aagtgtgctgctccttcgaatgc | 278712 | - | - | molecular_function unknown | Bethlem myopathy |
| 18624 | CP110 | NM_014711.3 | 2960 | ttgtacggtattcatgacatatt | 278816 | - | see also 5904 line | | |
| 18625 | CRISP3 | NM_006061.1 | 637 | aagtacgaagatctctatagtaa | 278839 | - | - | molecular_function unknown | - |
| 18626 | CRYPTIC | NM_032545.2 | 286 | ttgggaaacagctatcaaagaga | 278848 | - | - | - | double-outlet right ventricle、heterotaxy、transposition of great arteries |
| 18627 | CSDUFD1 | NM_031919.1 | 293 | gagtcagattagtcaatgtaata | 278859 | - | see also 9052 line | | |
| 18628 | CSN3 | NM_005212.1 | 264 | acgtagaccagctatagcaatta | 278872 | - | - | molecular_function unknown | - |
| 18629 | CSRP2 | NM_001321.1 | 218 | tagcacaacagtggcaattcacg | 278880 | - | - | molecular_function unknown | - |
| 18630 | CTAG1 | NM_001327.1 | 340 | gagccgcctgcttgagttctacc | 278883 | - | - | - | melanoma |
| 18631 | CTAG2 | NM_020994.1 | 652 | cagaggtgtcgcctttaatgtga | 278888 | - | - | molecular_function unknown | melanoma |
| 18632 | CTDSP2 | NM_005730.2 | 401 | tcgtggacgtaacatcttcaagg | 278899 | - | phosphatase | - | - |
| 18633 | CTDSPL | NM_005808.1 | 516 | ctgcttccgtgattacaatgtgg | 278912 | - | - | molecular_function unknown | - |
| 18634 | CTNND1 | NM_001331.1 | 1169 | tggttatagtcgccactatgaag | 278927 | - | see also 884 line | | |
| 18635 | CUL4B | NM_003588.2 | 2452 | atgggctattggccgacatatgt | 279054 | - | see also 2381 line | | |
| 18636 | CXorf1 | NM_004709.1 | 369 | tagcatccccactgactatattc | 279069 | - | - | molecular_function unknown | - |
| 18637 | CXorf2 | NM_001586.1 | 974 | aagagagaatggcctatctatcc | 279089 | - | - | molecular_function unknown | - |
| 18638 | CXorf6 | NM_005491.1 | 2085 | acgtgtcaccgtctaacattact | 279132 | - | - | molecular_function unknown | - |
| 18639 | CXX1 | NM_003928.1 | 394 | tggggagggtccgctgtgttact | 279137 | - | - | molecular_function unknown | - |
| 18640 | CYYR1 | NM_052954.1 | 191 | atggaaccacgccctactgttgc | 279139 | - | - | - | - |
| 18641 | DIPA | NM_006848.1 | 615 | tggcctgttcccccgatgattga | 279148 | - | - | molecular_function unknown | - |
| 18642 | DISC1 | NM_018662.1 | 1173 | gagacgttacaacaaagattaga | 279157 | - | - | protein binding | schizophrenia |
| 18643 | DKFZP434F091 | NM_015453.1 | 1767 | cagtttacgttctgatacgtaca | 279211 | - | - | - | - |

Figure 46

| ID | Symbol | Accession | Length | Sequence | Number | | Notes | | Function | | Disease | |
|---|---|---|---|---|---|---|---|---|---|---|---|---|
| 18644 | DKFZP434G1415 | | | ttgatgcttcatgctatgctaac | 279266 | - | | - | | - | | - |
| 18645 | DKFZP564B167 | NM_031292.1 | 2082 | tggagtcgtgttgcttgataatt | 279276 | - | | - | | - | | - |
| 18646 | DKFZP564C186 | NM_015415.1 | 437 | aagggggaagaagaattcgttcc | 279280 | - | | - | molecular_function unknown | - | | - |
| 18647 | DKK4 | NM_015658.1 | 436 | cggcagcagcgctcgattaagagt | 279293 | - | signal_transduction | - | molecular_function unknown | - | | - |
| 18648 | DLG7 | NM_014420.1 | 745 | aagttgtctcaggtatagcaagt | 279339 | - | see also 5943 line | - | | - | | - |
| 18649 | DLGAP1 | NM_014750.3 | 1875 | gggtacacccacgaggtaaaga | 279371 | - | | - | protein binding | - | | - |
| 18650 | DOC1 | NM_004746.2 | 1046 | gagacaaacaggtctttacgaaa | 279406 | - | | - | | - | | - |
| 18651 | DONSON | NM_014890.1 | 429 | ttgttcataaggagcttactaac | 279502 | - | see also 7054 line | - | | - | | - |
| 18652 | DP1 | NM_017613.2 | 1635 | ctgacctactgggtagttgtatgg | 279506 | - | | - | molecular_function unknown | - | | - |
| 18653 | DSCR3 | NM_005669.3 | 284 | gtggtcatatcgagtaaggattc | 279512 | - | see also 4213 line | - | | - | | - |
| 18654 | DSCR4 | NM_006052.1 | 315 | atgatgaaccccggatatttacc | 279532 | - | | - | molecular_function unknown | - | | - |
| 18655 | DSCR6 | NM_005867.1 | 131 | cagtgcaagccacgattgacttc | 279541 | - | | - | molecular_function unknown | - | | - |
| 18656 | DSCR8 | NM_018962.1 | 521 | gtgagaaagggtcttcattcatt | 279542 | - | | - | | - | | - |
| 18657 | DSCR9 | NM_032589.1 | 177 | gagcccatgtcgcgatgtaact | 279545 | - | | - | | - | | - |
| 18658 | E46L | NM_148675.1 | 919 | cagaatggccgttcttgattatt | 279577 | - | see also 5355 line | - | | - | | - |
| 18659 | EAT2 | NM_013236.1 | 836 | cagggattagttggatgtcttgc | 279608 | - | see also 9494 line | - | | - | | - |
| 18660 | EML4 | NM_053282.1 | 470 | caggaatcgatcggattgtaagg | 279685 | - | | - | molecular_function unknown | - | | - |
| 18661 | EPB41L4A | NM_019063.2 | 2507 | cagtcggagcttggagattatga | 279723 | - | see also 8303 line | - | | - | | - |
| 18662 | ERH | NM_022140.2 | 704 | ccgagtcgatacccagacatacc | 279755 | - | cell_cycle | - | molecular_function unknown | - | | - |
| 18663 | ET | NM_004450.1 | 287 | cggctctgactaccgaagtatct | 279797 | - | see also 8609 line | - | | - | | - |
| 18664 | FAM3A | NM_024311.2 | 1580 | atgtcaacgaccgtttgaagttt | 279844 | - | | - | | - | | - |
| 18665 | FANCD2 | NM_021806.1 | 663 | gaggagagattgatggtctactaga | 279913 | - | | - | | | aplastic anemia, Fanconi anemia, Fanconi pancytopenia | - |
| 18666 | FANCE | NM_033084.2 | 2311 | gagagaccgaacattaagtcact | 279966 | - | | - | | | aplastic anemia, Fanconi anemia, Fanconi pancytopenia | - |
| 18667 | FANCF | NM_021922.1 | 879 | tggaaccggcatccacacaaatca | 279978 | - | | - | | | Fanconi anemia | - |
| 18668 | FETUB | NM_022725.1 | 678 | ccggatctcttgcgaaatacaac | 280006 | - | | - | molecular_function unknown | - | | - |
| 18669 | FFZ2 | NM_014375.1 | 616 | cagggagggacgagatttggaatgc | 280015 | - | signal_transduction | - | molecular_function unknown | - | | - |

Figure 46

| 18670 | FHL1 | NM_001449.3 | 832 | ctgcgtggattgctacaagaact | 280042 | - | - | molecular_function unknown | - |
| 18671 | FKBPL | NM_022110.3 | 272 | tggagacgccaccagtcaataca | 280052 | - | - | - | - |
| 18672 | FKSG2 | NM_021631.1 | 442 | ctgctgaactaccgtaaggatgg | 280069 | - | apoptosis | molecular_function unknown | - |
| 18673 | FLJ10525 | NM_018126.1 | 369 | gggcgctgtgcaattcatgatga | 280073 | - | see also 7269 line | | |
| 18674 | FLJ10769 | NM_018210.1 | 936 | tggggtcctgagattgaaacagg | 280124 | - | - | - | - |
| 18675 | FLJ11294 | NM_018383.2 | 3234 | tggccaccggttacgtgaatttg | 280191 | - | see also 7429 line | | |
| 18676 | FLJ11749 | NM_024591.2 | 305 | ttgagttcacccagatcgaaatg | 280203 | - | - | - | - |
| 18677 | FLJ13448 | NM_025147.2 | 455 | gagcgatatacatcagtagtaac | 280226 | - | - | - | - |
| 18678 | FLJ14007 | NM_024699.1 | 682 | aagccttacccttggatcatact | 280249 | - | - | - | - |
| 18679 | FLJ20485 | NM_019042.2 | 1950 | ctgggaagtcgttgcatatgatg | 280279 | - | - | - | - |
| 18680 | FLJ21128 | NM_025083.2 | 912 | gagtccgagcccattgtctatcg | 280302 | - | - | - | - |
| 18681 | FLJ21827 | NM_020153.2 | 965 | cggtgctctcactctggttaaca | 280323 | - | see also 7771 line | | |
| 18682 | FLJ32452 | NM_144576.2 | 697 | tggcgattcagccctggtattcc | 280340 | - | - | - | - |
| 18683 | FSD1 | NM_024333.1 | 436 | ctgccttccggctatcattgaaa | 280349 | - | - | - | - |
| 18684 | GAGEC1 | NM_007003.2 | 236 | ccgatcgaagaacgtaaagtaga | 280365 | - | - | molecular_function unknown | - |
| 18685 | GAS1 | NM_002048.1 | 734 | ctgcatctcggccctcattcagc | 280370 | - | cell_cycle | - | - |
| 18686 | GAS8 | NM_001481.1 | 251 | ccgcgagcgggaggaacgaaact | 280371 | - | see also 1020 line | | |
| 18687 | GKN1 | NM_019617.2 | 466 | gtgtcgtgggattccaacataca | 280401 | - | cell_cycle | molecular_function unknown | - |
| 18688 | GLTSCR2 | NM_015710.2 | 780 | ccggagcttcctacaatccatcc | 280410 | - | - | molecular_function unknown | - |
| 18689 | GPM6B | NM_005278.2 | 542 | cagcggtgcccgtgtttatgttc | 280420 | - | see also 3703 line | | |
| 18690 | GPR155 | NM_152529.3 | 1057 | aagtcggcatttgtagtactaat | 280474 | - | - | - | - |
| 18691 | GRCC9 | NM_032641.1 | 374 | cggggtaagaggggctattcaag | 280529 | - | - | - | - |
| 18692 | GREB1 | NM_014668.2 | 1183 | ccgcgaccatcggctttaggtat | 280543 | - | see also 5843 line | | |
| 18693 | GTSE1 | NM_016426.3 | 1177 | aggccaagcgggtcgatgtttct | 280625 | - | cell_cycle、 signal_transduction | molecular_function unknown | - |
| 18694 | H-plk | NM_015852.1 | 1304 | tagctaaacatttagttatgttt | 280639 | - | - | - | - |
| 18695 | H41 | NM_017548.2 | 810 | gcgatgactagtggtgtgtatag | 280645 | - | - | - | - |
| 18696 | HBLD1 | NM_194279.1 | 244 | atgctccggattccaatacaaat | 280648 | - | - | - | - |
| 18697 | HCG9 | NM_005844.2 | 127 | tagtccccacagcaataggaagc | 280654 | - | - | - | - |
| 18698 | HERPUD1 | NM_014685.1 | 1025 | tagaccgaggccggttcagaact | 280674 | - | - | molecular_function unknown | Alzheimer disease |
| 18699 | HHLA2 | NM_007072.2 | 577 | aggggatccgaagtcgtaataca | 280684 | - | - | - | - |

Figure 46

| | | | | | | | | | |
|---|---|---|---|---|---|---|---|---|---|
| 18700 | HRASLS3 | NM_007069.1 | 630 | caggtcaacaacaaacatgatga | 280720 | - | - | molecular_function unknown | - |
| 18701 | HSA272196 | NM_018405.2 | 245 | gggtgagggcacccattctgaag | 280723 | - | - | - | - |
| 18702 | HSHIN1 | NM_017493.4 | 328 | atgtgtcagtctctcctttatga | 280732 | - | - | molecular_function unknown | - |
| 18703 | HSPC117 | NM_014306.2 | 438 | tggtgtcgggtttgacatcaact | 280739 | - | see also 5612 line | | |
| 18704 | HSPC121 | NM_016395.1 | 189 | cagcgacaccgcgagctatatct | 280773 | - | kinase_related、signal transduction | - | - |
| 18705 | HSPC148 | NM_016403.1 | 161 | gagacctaccctctcatacaaag | 280801 | - | - | - | - |
| 18706 | HSPC177 | NM_016410.2 | 345 | tggaacaagccaattataccatc | 280818 | - | - | - | - |
| 18707 | HSRG1 | NM_014940.2 | 1877 | aggaccggctcttcattcgttac | 280833 | - | - | - | - |
| 18708 | IFI27 | NM_005532.1 | 142 | ctggccaggattgctacagttgt | 280835 | - | - | molecular_function unknown | - |
| 18709 | IFIT1 | NM_001548.1 | 130 | atgggagttatccattgatgacg | 280840 | - | - | molecular_function unknown | - |
| 18710 | IFIT4 | NM_001549.2 | 1016 | atgctatggactattcgaataaa | 280865 | - | - | molecular_function unknown | - |
| 18711 | IFIT5 | NM_012420.1 | 859 | cagccttacgtccttcgttatgc | 280898 | - | - | molecular_function unknown | - |
| 18712 | IFRD2 | NM_006764.2 | 185 | gggacgcttcggagttaaccacc | 280916 | - | - | molecular_function unknown | - |
| 18713 | IMPACT | NM_018439.1 | 156 | tagaattagcgacgatatagatg | 280926 | - | see also 7479 line | | |
| 18714 | JPH1 | NM_020647.1 | 610 | cggcggtttcgtgctcaacttcc | 280946 | - | see also 7905 line | | |
| 18715 | KERA | NM_007035.2 | 904 | cagctaagatggataaatctaaa | 280971 | - | - | molecular_function unknown | - |
| 18716 | KIAA0174 | NM_014761.1 | 630 | tggggtagagacagatcttattg | 281001 | - | - | - | - |
| 18717 | KIAA0186 | NM_021067.1 | 310 | tcgacgctgcactgtagcatacc | 281013 | - | - | molecular_function unknown | - |
| 18718 | KIAA0285 | NM_014807.2 | 1626 | ccgtatagacggcaaattagact | 281031 | - | - | - | - |
| 18719 | KIAA0905 | NM_014933.1 | 1253 | tggaggcaaactggttacgtttg | 281079 | - | - | - | - |
| 18720 | KIAA1173 | NM_020707.2 | 604 | aagatggaccccctaatctcttg | 281134 | - | - | - | - |
| 18721 | KREMEN1 | NM_032045.3 | 120 | cagccaatggtgcggattatagg | 281150 | - | see also 9071 line | | |
| 18722 | KTN1 | NM_004986.1 | 3924 | ttgcttcaggcggtaaaccaaca | 281269 | - | see also 3481 line | | |
| 18723 | LENG1 | NM_024316.1 | 143 | gaggcccgtacagaattcctacg | 281274 | - | - | - | - |
| 18724 | LENG4 | NM_024298.2 | 1456 | gtggtggctggcgcagtatatct | 281280 | - | - | - | - |
| 18725 | LGP1 | NM_032484.3 | 1550 | tggttggtgcctacaatcagtgt | 281290 | - | - | - | - |
| 18726 | Lin10 | NM_025187.3 | 342 | ctgactcaggacgggatcaaact | 281299 | - | see also 8875 line | | |

754

Figure 46

| | | | | | | | | | |
|---|---|---|---|---|---|---|---|---|---|
| 18727 | LLGL1 | NM_004140.2 | 889 | ctgtggcggaactgtgaatctgg | 281333 | - | kinase_related | - | - |
| 18728 | LMCD1 | NM_014583.2 | 1041 | cggctgcgatgagataatattcg | 281361 | - | see also 5763 line | | |
| 18729 | LMNB2 | NM_032737.2 | 1559 | aggagatcgcctacaagttcacg | 281365 | - | - | molecular_function unknown | - |
| 18730 | LMO6 | NM_006150.3 | 1639 | cagggtcagactcggaatcttgc | 281378 | - | - | molecular_function unknown | - |
| 18731 | LOC130617 | NM_138802.1 | 484 | ctgccggcagcgagaaatgatga | 281390 | - | see also 9640 line | | |
| 18732 | LOC55974 | NM_018845.1 | 290 | ctgcgcttcagaccctgtatatc | 281395 | - | - | - | - |
| 18733 | LOC91614 | NM_139160.1 | 1206 | cagaccgaacagacttagtgaaa | 281430 | - | - | - | - |
| 18734 | LPIN1 | NM_145693.1 | 1243 | tggtgctgacggcgtctacttgg | 281479 | - | - | molecular_function unknown | - |
| 18735 | LPP | NM_005578.1 | 1638 | ctgcgagccctgctacattaata | 281522 | - | see also 3879 line | | |
| 18736 | LRBA | NM_006726.1 | 2401 | tgggttacgtgttatctacaaac | 281612 | - | see also 4735 line | | |
| 18737 | LRG | NM_052972.1 | 636 | aggggtccgctgcaattagaacg | 281797 | - | - | molecular_function unknown | - |
| 18738 | LRPPRC | NM_133259.2 | 1748 | ttgtacaaggatggacgttattg | 281856 | - | see also 9557 line | | |
| 18739 | LY6G5B | NM_021221.1 | 935 | ctgtggacagtacatttcctacc | 281920 | - | - | - | - |
| 18740 | LY9 | NM_002348.1 | 866 | ttgtctggttgtttaacacatcc | 281929 | - | - | molecular_function unknown | - |
| 18741 | MAC30 | NM_014573.1 | 643 | gagcccctactacaagtatgaag | 281945 | - | - | - | - |
| 18742 | MAD1L1 | NM_003550.1 | 835 | cagaccacgagcagcagattaag | 281953 | - | - | - | prostate cancer |
| 18743 | MAGEA4 | NM_002362.3 | 834 | tcgtcctgggcacaattgcaatg | 281959 | - | - | molecular_function unknown | melanoma |
| 18744 | MAGEA5 | NM_021049.2 | 423 | ctgccatcgatttcactctatgg | 281961 | - | - | molecular_function unknown | - |
| 18745 | MAGEA9 | NM_005365.4 | 823 | tggcctctcgtgcgatagcatgc | 281963 | - | - | molecular_function unknown | - |
| 18746 | MAGEL2 | NM_019066.2 | 1623 | ctgagcccgcagagtatgagttc | 281987 | - | - | molecular_function unknown | - |
| 18747 | Magmas | NM_016069.8 | 285 | cagaagaactatgaacacttatt | 281992 | - | - | - | - |
| 18748 | MCSP | NM_030663.2 | 378 | ctgaagtctcacccccttaacatg | 281994 | - | - | molecular_function unknown | - |
| 18749 | MFAP4 | NM_002404.1 | 140 | ctggactgtgacgacatctatgc | 281998 | - | - | extracellular matrix structural constituent | Smith-Magenis syndrome chromosome region |
| 18750 | MGC23947 | NM_152278.1 | 281 | acgcccgtatggagaatttgaac | 282020 | - | - | - | - |
| 18751 | MGC26979 | NM_153704.2 | 21 | tggcggtttggtccctcttatcc | 282021 | - | see also 10013 line | | |

Figure 46

| 18752 | MGC29816 | NM_152272.1 | 531 | gaggaggtgtatcgtctgtatca | 282110 | - | see also 9825 line | | |
|---|---|---|---|---|---|---|---|---|---|
| 18753 | MLF2 | NM_005439.1 | 239 | ctgatggatccctttgctattca | 282125 | - | - | molecular_function unknown | - |
| 18754 | MLN51 | NM_007359.3 | 2107 | caggcgtcatgaactttggtaat | 282170 | - | see also 5090 line | | |
| 18755 | MN1 | NM_002430.1 | 4045 | gggccaagtcggacagtagttcg | 282197 | - | cell_cycle | molecular_function unknown | meningioma |
| 18756 | MORF4L2 | NM_012286.1 | 639 | gaggaggcgtttaagaatagaat | 282207 | - | - | molecular_function unknown | - |
| 18757 | MPV17 | NM_002437.3 | 510 | ttggccgttgtccaatgtgttgc | 282227 | - | - | molecular_function unknown | - |
| 18758 | MRPL39 | NM_017446.2 | 141 | atgcggaatgatctctttaataa | 282236 | - | see also 7013 line | | |
| 18759 | MRPL40 | NM_003776.2 | 138 | gcgagcgtcattgttgtctttct | 282278 | - | - | - | - |
| 18760 | MRPS24 | NM_032014.1 | 475 | gtgccctcaaaggttgtgtataa | 282333 | - | - | - | - |
| 18761 | MSLN | NM_005823.2 | 1016 | tagacgagagcctcatcttctac | 282337 | - | - | - | - |
| 18762 | MSMB | NM_002443.2 | 83 | atgcaatgcatcatgctatttca | 282346 | - | - | - | prostate cancer |
| 18763 | MUM-1 | NM_032853.2 | 256 | tggcccgaaccgcgacttcaaca | 282358 | - | - | - | - |
| 18764 | NAG | NM_015909.1 | 1960 | cagctggcagcgtattactatag | 282435 | - | - | - | - |
| 18765 | NAP1 | NM_016481.2 | 524 | tggcacacccgtactagacatca | 282498 | - | - | - | - |
| 18766 | NBR2 | NM_005821.2 | 598 | ttggttcctgacacaaggattcc | 282512 | - | - | molecular_function unknown | - |
| 18767 | NCSTN | NM_015331.1 | 2003 | gtgcgttctactgcacgattagc | 282555 | - | see also 6367 line | | |
| 18768 | NDRG2 | NM_016250.1 | 265 | gtgggactcaactataaatcttg | 282561 | - | see also 6753 line | | |
| 18769 | NEDF | NM_016079.1 | 62 | tggtcaatgagtggtcattgaag | 282574 | - | - | - | - |
| 18770 | NEUGRIN | NM_016645.1 | 677 | atggagcagatacggtatttaca | 282581 | - | transcription_regulator | - | - |
| 18771 | NOC4 | NM_006067.3 | 740 | gagacgctcgtggatttcgataa | 282596 | - | - | molecular_function unknown | - |
| 18772 | NOLA3 | NM_018648.2 | 122 | gagcagggagatcgagtctatac | 282600 | - | see also 7535 line | | |
| 18773 | NPC2 | NM_006432.3 | 372 | gagcctgatggttgtaagagtgg | 282611 | - | - | molecular_function unknown | Niemann-Pick disease |
| 18774 | NRM | NM_007243.1 | 86 | tggagttcgtgcgctttacctcc | 282615 | - | - | - | - |
| 18775 | NTN2L | NM_006181.1 | 1559 | ccgcatcagcctaaagaagttct | 282619 | - | - | - | - |
| 18776 | NTNG1 | NM_014917.1 | 1438 | tggacctcgcctacgcaatatgg | 282643 | - | see also 6088 line | | |
| 18777 | NTNG2 | NM_032536.1 | 731 | cgggaccgcttcgccatctttgc | 282651 | - | - | - | - |
| 18778 | NXPH3 | NM_007225.1 | 493 | cagcgtccacttccaacacaatg | 282658 | - | signal_transduction | molecular_function unknown | - |
| 18779 | NXPH4 | NM_007224.1 | 843 | aagagtcacgcgctttcaattgc | 282662 | - | - | - | - |

Figure 46

| 18780 | NYREN18 | NM_016118.3 | 1114 | aagggatccgaaactatcacagt | 282690 | - | see also 6672 line | | |
|---|---|---|---|---|---|---|---|---|---|
| 18781 | OFD1 | NM_003611.1 | 316 | tggcgcagtccaacatgtttacc | 282703 | - | see also 2425 line | | |
| 18782 | OTOF | NM_004802.2 | 1778 | aagctggacgcgacttctgaagc | 282795 | - | - | - | deafness |
| 18783 | P8 | NM_012385.1 | 299 | tggtgaccaagctgcagaattca | 282919 | - | apoptosis | molecular_function unknown | - |
| 18784 | PART1 | NM_016590.2 | 147 | tcgtacgctgggctataatctat | 282924 | - | see also 6935 line | | |
| 18785 | PDCD1LG2 | NM_025239.2 | 542 | gggacgaaggacagtaccaatgc | 282929 | - | - | - | - |
| 18786 | PHEMX | NM_005705.3 | 838 | gggtggacccacacattgtctcc | 282941 | - | - | - | - |
| 18787 | PKP2 | NM_004572.1 | 2451 | atgcacgcgaccttctaaacacc | 282977 | - | - | structural molecule | - |
| 18788 | PLAC1 | NM_021796.1 | 600 | gggcacgccatctaagtttgtga | 282991 | - | - | - | - |
| 18789 | POLA2 | NM_002689.2 | 1821 | cagattcagccgaatactcaagc | 283033 | - | see also 1751 line | | |
| 18790 | POPDC2 | NM_022135.2 | 175 | agggttcagcgtgcattaggtgg | 283038 | - | - | - | - |
| 18791 | POPDC3 | NM_022361.3 | 1195 | cggctacccaacttctatcaaat | 283072 | - | see also 8345 line | | |
| 18792 | PP3111 | NM_022156.3 | 765 | cagcgtcctgggagcatatcaag | 283083 | - | - | - | - |
| 18793 | PP35 | NM_181581.1 | 423 | ctgtgccccaatggttcgatatt | 283088 | - | - | - | - |
| 18794 | PRCC | NM_005973.4 | 1557 | cagcaatggatgactaagtcatt | 283135 | - | - | molecular_function unknown | renal cell carcinoma |
| 18795 | PRKAG2 | NM_016203.2 | 646 | cggttagagaatcgcatctatgc | 283144 | - | see also 6719 line | | |
| 18796 | PRM3 | NM_021247.1 | 111 | ctgtgtgagtcaggataacttct | 283171 | - | - | - | - |
| 18797 | PROK2 | NM_021935.2 | 274 | ctgccaggcttggcctgtttacg | 283176 | - | gpcr、apoptosis、kinase_related、signal_transduction | molecular_function unknown | - |
| 18798 | PROL1 | NM_021225.1 | 606 | cagctcctcaacagtacctatct | 283196 | - | - | - | - |
| 18799 | PROSC | NM_007198.2 | 1172 | gggcatgtccgcggatttccagc | 283211 | - | - | molecular_function unknown | - |
| 18800 | PRX | NM_020956.1 | 524 | atgcgccgagccttacaaagtct | 283216 | - | - | - | hereditary motor and sensory neuropathy |
| 18801 | PSG9 | NM_002784.2 | 492 | gagaagaaattcgacatttcacc | 283231 | - | - | - | - |
| 18802 | PTD008 | NM_016145.1 | 371 | gtgcggcctcatgcttaagctga | 283237 | - | - | molecular_function unknown | - |
| 18803 | PTTG1IP | NM_004339.2 | 464 | ctgcacgctggggagtttgttgg | 283241 | - | - | molecular_function unknown | - |
| 18804 | RAI2 | NM_021785.2 | 1284 | cagccgccacacggtcattaaga | 283252 | - | - | molecular_function unknown | - |
| 18805 | RARRES2 | NM_002889.2 | 351 | atgcctggcctgcatcaaactgg | 283263 | - | - | molecular_function unknown | - |
| 18806 | RCD-8 | NM_014329.2 | 745 | gtggcgcttggctctggttaatg | 283267 | - | see also 5623 line | | |

EP 1 752 536 A1

Figure 46

EP 1 752 536 A1

| | | | | | | | | | |
|---|---|---|---|---|---|---|---|---|---|
| 18807 | RER1 | NM_007033.2 | 623 | ctggccgattctggtgatgtact | 283304 | - | - | molecular_function unknown | - |
| 18808 | RIOK3 | NM_003831.2 | 1322 | ttgatgcggcagttatatcatga | 283374 | - | see also 2611 line | | |
| 18809 | RP1 | NM_006269.1 | 444 | aggacggcgagtcctacctatgt | 283393 | - | - | molecular_function unknown | - |
| 18810 | RP42 | NM_020640.2 | 81 | aagttcgtcagtttatgatcttc | 283578 | - | - | - | - |
| 18811 | SACS | NM_014363.3 | 11512 | ttgacgatgcgccacattataaa | 283999 | - | see also 5631 line | | |
| 18812 | SCA7 | NM_000333.1 | 2153 | gaggctattacgtgtttgactcc | 284090 | - | see also 283 line | | |
| 18813 | SCHIP1 | NM_014575.1 | 76 | aggtccgggcagcgtgttacaac | 284102 | - | see also 5762 line | | |
| 18814 | SEL1L | NM_005065.3 | 501 | gtgtgctacaacctatgactaca | 284122 | - | - | molecular_function unknown | diabetes |
| 18815 | SEPX1 | NM_016332.1 | 364 | gggcagtcccgattctgaatatt | 284185 | - | - | selenium binding | - |
| 18816 | Shax3 | NM_152284.2 | 369 | ctgcattacaggcactaaagaga | 284189 | - | - | - | - |
| 18817 | SHFM1 | NM_006304.1 | 147 | ccggtagacttaggtctgttaga | 284195 | - | see also 4396 line | | |
| 18818 | SHFM3 | NM_022039.2 | 1164 | atgggaagattggcattcataag | 284199 | - | signal_transduction | molecular_function unknown | split-hand and split-foot |
| 18819 | SIPA1L1 | NM_015556.1 | 3025 | aagagcgtggtcttcaattgttc | 284318 | - | see also 6465 line | | |
| 18820 | SKI | NM_003036.1 | 498 | gtgcgacgagctccacatctact | 284361 | - | - | molecular_function unknown | - |
| 18821 | SLC22A1LS | NM_007105.1 | 781 | gtgatgcctcagccagtcttaaa | 284367 | - | - | - | - |
| 18822 | SLC35F2 | NM_017515.3 | 440 | tcgatcaggcagtgataaccttt | 284378 | - | - | - | - |
| 18823 | SNAP25 | NM_003081.2 | 713 | gggcaatgagatcgatacacaga | 284427 | - | - | - | - |
| 18824 | SOST | NM_025237.1 | 120 | gggtggcaggcgttcaagaatga | 284432 | - | - | molecular_function unknown | - |
| 18825 | SPAG8 | NM_012436.2 | 152 | cggtcgcgatctttagacataca | 284440 | - | - | - | - |
| 18826 | SPATA2 | NM_006038.2 | 1700 | ctgcctcagcgcttaccattatg | 284482 | - | see also 4207 line | | |
| 18827 | SPRY3 | NM_005840.1 | 1021 | ctgtgtcaagggcctcttctacc | 284492 | - | signal_transduction | - | - |
| 18828 | SQSTM1 | NM_003900.2 | 444 | tggtaggaacccgctacaagtgc | 284495 | - | - | - | - |
| 18829 | ST7 | NM_013437.2 | 293 | caggcgaaatcattactataagt | 284515 | - | see also 5439 line | | |
| 18830 | STAF65(gamma) | NM_014860.1 | 523 | tagtctcaccatccatacgattc | 284582 | - | - | - | - |
| 18831 | SUMF2 | NM_015411.1 | 102 | tggctcaagctaggaaatggaca | 284595 | - | - | - | - |
| 18832 | SURF2 | NM_017503.2 | 360 | cagcgagctctgtgtaaatatga | 284603 | - | - | molecular_function unknown | - |
| 18833 | SURF4 | NM_033161.2 | 796 | ctggaccattccagtctacaagc | 284616 | - | - | molecular_function unknown | - |
| 18834 | SYMPK | NM_004819.1 | 1805 | cagcccaggtccgcataaagatc | 284634 | - | - | molecular_function unknown | - |

Figure 46

| No. | Gene | Accession | Length | Sequence | ID | Annotation | Molecular function | Disease |
|---|---|---|---|---|---|---|---|---|
| 18835 | TBL2 | NM_012453.1 | 900 | tggtgcggagccttcgaactaaag | 284651 | - | - | - |
| 18836 | TCTE1L | NM_006520.1 | 340 | atggaacctgtaccgtaagatgg | 284686 | - | molecular_function unknown | - |
| 18837 | TEX27 | NM_021943.1 | 303 | tcgataaccacgccaactcttag | 284692 | see also 8205 line | - | - |
| 18838 | TFG | NM_006070.3 | 1265 | cagtaccaggcgagcaatatcc | 284731 | see also 4215 line | - | - |
| 18839 | THSD3 | NM_182509.2 | 367 | tggccaacacaaccttgagtacc | 284739 | - | - | - |
| 18840 | THY1 | NM_006288.2 | 212 | cagcctgaccgtgagacaaaga | 284744 | see also 4378 line | - | - |
| 18841 | TIMP3 | NM_000362.3 | 1599 | tcggtatcacctgggttgtaact | 284750 | see also 296 line | - | - |
| 18842 | TLE4 | NM_007005.3 | 1684 | aagaaagatgccccgattagtcc | 284766 | see also 4888 line | - | - |
| 18843 | TM4SF1 | NM_014220.1 | 364 | gtgcgatgctttcttcgtgattg | 284792 | - | molecular_function unknown | - |
| 18844 | TM6SF1 | NM_023003.1 | 1103 | tggcctatcgttgatctacaaa | 284835 | see also 8537 line | - | - |
| 18845 | TMEFF2 | NM_016192.2 | 1058 | atgtctttggtcgatgtcaaga | 284854 | see also 6717 line | - | - |
| 18846 | TMEM14A | NM_014051.2 | 224 | aggtggtgttcgtcgtcttgattg | 284877 | - | - | - |
| 18847 | TMEM8 | NM_021259.1 | 644 | gcgggtggtcgagatttccatca | 284885 | - | - | - |
| 18848 | TMEPAI | NM_020182.3 | 940 | gtgcgcgcaccccaaacagaac | 284896 | signal_transduction | molecular_function unknown | - |
| 18849 | TPARL | NM_018475.2 | 749 | gtgatcgtctcaactaactaca | 284914 | - | - | - |
| 18850 | TPD52L1 | NM_003287.1 | 313 | cagctagaagacgaaattacaac | 284926 | - | - | breast cancer |
| 18851 | TSGA10 | NM_025244.1 | 1608 | cagaatgcgtcggcaattggatg | 284969 | - | - | - |
| 18852 | TU3A | NM_007177.1 | 719 | gttggaatccggagctcatcaagc | 285008 | - | molecular_function unknown | - |
| 18853 | TXNIP | NM_006472.1 | 1172 | gagttgggttagatctgaacatcc | 285026 | - | - | - |
| 18854 | UACA | NM_018003.1 | 662 | atgctaggttgcgaatatggttg | 285052 | - | - | - |
| 18855 | USP6NL | NM_014688.1 | 2624 | tagatagtcccgtgagatataaa | 285190 | - | - | - |
| 18856 | UXT | NM_004182.2 | 238 | aagctaagcactcggagttatat | 285196 | - | - | - |
| 18857 | VMD2 | NM_004183.1 | 762 | atgcctacgactggattagtatc | 285206 | channel | molecular_function unknown | Best disease |
| 18858 | VMD2L1 | NM_017682.1 | 598 | tggaatgctctttcactatgact | 285228 | channel | - | - |
| 18859 | VMD2L2 | NM_153274.1 | 586 | ccggaggacgggcgaatacgtga | 285234 | channel | - | - |
| 18860 | VMD2L3 | NM_152439.1 | 610 | ctgctgcccaccatacacattg | 285246 | channel | - | - |
| 18861 | WDR6 | NM_018031.2 | 2587 | cggcatcggatggttaaggtaga | 285283 | - | - | - |
| 18862 | WFDC1 | NM_021197.2 | 260 | ccggctctgccaagaatatctgg | 285286 | - | molecular_function unknown | - |
| 18863 | WIT-1 | NM_015855.2 | 1021 | cggcctcgaaggtgcataattat | 285295 | - | molecular_function unknown | - |
| 18864 | WSB1 | NM_015626.7 | 1250 | cggtggttacgatctgtatcttt | 285328 | - | - | - |

Figure 46

| | | | | | | | | | |
|---|---|---|---|---|---|---|---|---|---|
| 18865 | XG | NM_175569.1 | 450 | tggaggttacttcaatgatgtgg | 285345 | - | - | molecular_function unknown | - |
| 18866 | YIF1 | NM_020470.1 | 195 | atgacacaagcggtggttattcc | 285347 | - | - | - | - |
| 18867 | ZNF200 | NM_003454.2 | 1968 | aagatttggtcggctgtcaaact | 285371 | - | transcription_regulator | - | - |
| 18868 | ZP4 | NM_021186.2 | 747 | tggagaccgagcagtatatgaaa | 285389 | - | receptor_acitivity | - | - |
| 18869 | AGS3 | NM_015597.2 | 365 | aggcgagggccctctacaacatc | 285403 | - | - | - | - |
| 18870 | C1orf33 | NM_016183.2 | 260 | ttgggtcggagcccatctgatga | 285417 | - | - | - | - |
| 18871 | DAP3 | NM_004632.2 | 450 | ttgcttatccagctatacgatat | 285436 | - | see also 3147 line | | |
| 18872 | FLJ12331 | NM_024986.1 | 541 | aagctttcatacaagaataatag | 285460 | - | - | - | - |
| 18873 | GADD45A | NM_001924.2 | 701 | aaggatcctgccttaagtcaact | 285462 | - | see also 1227 line | | |
| 18874 | GADD45B | NM_015675.1 | 224 | gagtcggccaagttgatgaatgt | 285469 | - | apoptosis、kinase_related | structural constituent of ribosome | - |
| 18875 | GADD45G | NM_006705.2 | 175 | cgggaaagcgctgcatgagttgc | 285473 | - | apoptosis、kinase_related | structural constituent of ribosome | - |
| 18876 | LACTB | NM_032857.2 | 508 | gagacagttatgcgaattgctag | 285480 | - | see also 9314 line | | |
| 18877 | MGC9651 | NM_152301.3 | 307 | gaggacatcgcgtctcttatacg | 285508 | - | - | - | - |
| 18878 | MRPL11 | NM_016050.2 | 413 | gtgaccttgaagcatgtgtatga | 285515 | - | - | - | - |
| 18879 | MRPL13 | NM_014078.4 | 820 | gtggactccacctgaagattatc | 285535 | - | - | structural constituent of ribosome | - |
| 18880 | MRPL14 | NM_032111.1 | 296 | aaggtgggcgaccagatactact | 285536 | - | - | - | - |
| 18881 | MRPL15 | NM_014175.2 | 307 | gggtttaacgaaggacatagttt | 285546 | - | - | - | - |
| 18882 | MRPL16 | NM_017840.2 | 897 | aagtactgagcccatatgacttg | 285579 | - | - | - | - |
| 18883 | MRPL17 | NM_022061.2 | 324 | ctggcccctcggtacaaagatca | 285580 | - | - | - | - |
| 18884 | MRPL18 | NM_014161.2 | 354 | tggcacaggttgcgagttataag | 285591 | - | - | - | - |
| 18885 | MRPL19 | NM_014763.2 | 570 | tggatgatagcttgctatactta | 285614 | - | - | structural constituent of ribosome | - |
| 18886 | MRPL2 | NM_015950.2 | 775 | gagtatccaacgttgatcataac | 285643 | - | - | - | - |
| 18887 | MRPL22 | NM_014180.2 | 50 | ttgggtgcgttatggatacataa | 285650 | - | - | - | - |
| 18888 | MRPL24 | NM_024540.2 | 533 | gagatcgagtggagatttactga | 285674 | - | - | - | - |
| 18889 | MRPL27 | NM_016504.2 | 313 | ggggatagtccgctacactaagg | 285685 | - | - | - | - |
| 18890 | MRPL30 | NM_016503.2 | 224 | aaggtgtggagtctcttatttgt | 285691 | - | - | - | - |
| 18891 | MRPL32 | NM_031903.1 | 295 | tagacgcaccattgaagttaacc | 285712 | - | - | structural constituent of ribosome | - |
| 18892 | MRPL35 | NM_016622.2 | 299 | ctgccagtcagatctctaacata | 285733 | - | - | - | - |
| 18893 | MRPL36 | NM_032479.2 | 124 | gagccctctccacatttctattt | 285744 | - | - | - | - |
| 18894 | MRPL37 | NM_016491.2 | 410 | ccgccttctcgagggtgtaaagc | 285753 | - | - | - | - |
| 18895 | MRPL4 | NM_015956.2 | 1113 | tggcaggactcacgttacagacc | 285782 | - | - | - | - |

# Figure 46

| | | | | | | | | | |
|---|---|---|---|---|---|---|---|---|---|
| 18896 | MRPL41 | NM_032477.1 | 236 | ctggaggttcgtgcagatcaagg | 285783 | - | - | - | - |
| 18897 | MRPL42 | NM_014050.2 | 383 | tcggccagatcctgtgcataata | 285790 | - | - | - | - |
| 18898 | MRPL43 | NM_032112.2 | 293 | gagtagtggccgaataccttaac | 285798 | - | - | - | - |
| 18899 | MRPL46 | NM_022163.2 | 640 | gtgggcactacacattcaagttc | 285815 | - | - | - | - |
| 18900 | MRPL47 | NM_020409.2 | 662 | gagaaacgagcccgcatcaaagc | 285842 | - | - | - | - |
| 18901 | MRPL48 | NM_016055.3 | 397 | ctgtaggtggaattctactaagt | 285849 | - | see also 6645 line | | |
| 18902 | MRPL50 | NM_019051.1 | 301 | tagtcgtctaaagttcaatcttc | 285874 | - | - | - | - |
| 18903 | MRPL9 | NM_031420.2 | 307 | ccggcgacatcgcgtctataagc | 285890 | - | - | structural constituent of ribosome | - |
| 18904 | MRPS14 | NM_022100.1 | 326 | ctggaggcttagtcgtatagtct | 285928 | - | - | - | - |
| 18905 | MRPS15 | NM_031280.2 | 109 | tggaggacgctgagtttgattcg | 285930 | - | - | structural constituent of ribosome | - |
| 18906 | MRPS17 | NM_015969.2 | 134 | tggatccctatttattaaagtat | 285950 | - | - | structural constituent of ribosome | - |
| 18907 | MRPS18A | NM_018135.2 | 303 | aggcatgctgccccgaaagatca | 285957 | - | - | - | - |
| 18908 | MRPS18B | NM_014046.2 | 65 | ctgaggcggcttcctatgctatc | 285964 | - | - | - | - |
| 18909 | MRPS2 | NM_016034.2 | 129 | aagcgcgacggcccttatgatcc | 285978 | - | - | - | - |
| 18910 | MRPS21 | NM_018997.1 | 721 | tggctcgcaagatcaacttcttg | 285990 | - | - | - | - |
| 18911 | MRPS23 | NM_016070.2 | 257 | gtgtatgggtctggtcaaagagc | 286000 | - | - | - | - |
| 18912 | MRPS25 | NM_022497.3 | 194 | gtgaaggtcatgacagtgaatta | 286004 | - | - | structural constituent of ribosome | - |
| 18913 | MRPS26 | NM_030811.3 | 132 | acgacccgctggccaaatccaag | 286012 | - | - | - | - |
| 18914 | MRPS27 | NM_015084.1 | 1148 | gtggcatctagaccttgtacagt | 286035 | - | - | structural constituent of ribosome | - |
| 18915 | MRPS31 | NM_005830.2 | 688 | gagcttcggattcagtttgatga | 286043 | - | - | - | - |
| 18916 | MRPS33 | NM_016071.2 | 322 | atgcagacgctccgatttcttgg | 286059 | - | - | - | - |
| 18917 | MRPS34 | NM_023936.1 | 363 | tggggcatcctgaccttcaaagg | 286060 | - | - | - | - |
| 18918 | MRPS35 | NM_021821.2 | 546 | cagagcacgagtagtagtcttaa | 286073 | - | - | - | - |
| 18919 | MRPS5 | NM_031902.3 | 263 | tagcgggacggcaggtcatttat | 286096 | - | - | - | - |
| 18920 | MRPS6 | NM_032476.2 | 184 | ccgctacgagctggctttaatcc | 286126 | - | - | structural constituent of ribosome | - |
| 18921 | MRPS7 | NM_015971.2 | 294 | gagccgctatagtcctgaattca | 286128 | - | see also 6590 line | | |
| 18922 | NOLA2 | NM_017838.2 | 400 | atgtctatatcccctctaagacg | 286139 | - | - | - | - |
| 18923 | RPL10L | NM_080746.2 | 329 | tggcagagatggctttcacatgc | 286142 | - | - | - | - |
| 18924 | RPL27 | NM_000988.2 | 256 | ttgtgaaagtgtataactacaat | 286143 | - | - | structural constituent of ribosome | - |
| 18925 | RPS27L | NM_015920.2 | 79 | ttggctagagatttactacatcc | 286144 | - | - | - | - |

Figure 46

| 18926 | UBA52 | NM_003333.2 | 151 | ctgaccagcagcgtctgatattt | 286152 | - | - | structural constituent of ribosome | - |
| 18927 | 76P | NM_014444.2 | 378 | ccggctcggcacagactatattc | 286160 | - | see also 5676 line | | |
| 18928 | ACTA2 | NM_001613.1 | 640 | gtggctattccttcgttactact | 286207 | - | - | structural constituent of muscle | - |
| 18929 | ACTC | NM_005159.2 | 995 | ttgctccccctgagcgtaaatac | 286215 | - | see also 3626 line | | |
| 18930 | ACTG2 | NM_001615.2 | 943 | ttgacatccgtaaggacttatat | 286223 | - | - | structural constituent of muscle | - |
| 18931 | ACTL7A | NM_006687.2 | 1245 | gtgggtccaccgctttgagtacg | 286239 | - | see also 4721 line | | |
| 18932 | ACTL7B | NM_006686.2 | 851 | cggacggcaaactcatcactatt | 286245 | - | - | - | - |
| 18933 | ACTR10 | NM_018477.1 | 334 | gagaccgccgagttgtgattatc | 286255 | - | see also 7492 line | | |
| 18934 | ACTR1A | NM_005736.2 | 716 | gagcctgttacctatccataaac | 286307 | - | see also 3980 line | | |
| 18935 | ACTR1B | NM_005735.2 | 662 | ttgaggttgtccggacaatcaaa | 286323 | - | - | structural constituent of cytoskeleton | - |
| 18936 | ACTR3 | NM_005721.3 | 545 | gtggccaatccgccatggtatag | 286343 | - | - | structural constituent of cytoskeleton | - |
| 18937 | ACTR6 | NM_022496.2 | 124 | gtgtcggttattcctaattgtca | 286382 | - | see also 8419 line | | |
| 18938 | ACTR8 | NM_022899.3 | 948 | ggggtgtctcatcggaatactcg | 286443 | - | see also 8520 line | | |
| 18939 | ACTRT1 | NM_138289.1 | 821 | aggccgtggtaaataacatcaaa | 286487 | - | - | - | - |
| 18940 | ANK2 | NM_001148.2 | 4287 | ttgcggacgactatcatttatga | 286633 | - | see also 730 line | | |
| 18941 | ARPC1B | NM_005720.2 | 274 | ccgagagtaaccgtattgtgacc | 286813 | | - | structural constituent of cytoskeleton | Alzheimer disease |
| 18942 | ARPC2 | NM_005731.2 | 842 | gtgctctaaggcctatattcaca | 286842 | - | - | - | - |
| 18943 | ARPC3 | NM_005719.2 | 289 | aggaccttgatatatataactct | 286844 | - | - | structural constituent of cytoskeleton | - |
| 18944 | ARPC4 | NM_005718.2 | 358 | atgcgagcagagaacttctttat | 286847 | - | see also 3969 line | | |
| 18945 | ARPC5 | NM_005717.2 | 532 | ttgagagcccgtctgacaatagc | 286856 | - | - | structural constituent of cytoskeleton | - |
| 18946 | ARPM1 | NM_032487.2 | 1298 | aagcggttagttaaggatatagc | 286877 | - | - | - | - |
| 18947 | BFSP1 | NM_001195.2 | 1026 | tggatccggtgggaaagatctta | 286893 | - | - | structural constituent of eye lens | - |
| 18948 | BFSP2 | NM_003571.2 | 683 | aggccggagcagatgactttaaa | 286917 | - | - | structural constituent of eye lens | cataract |
| 18949 | CD2AP | NM_012120.1 | 975 | tggaggggcgaacttaatggtaa | 286952 | - | see also 5143 line | | |
| 18950 | CTNNA2 | NM_004389.1 | 736 | acgagagccaaccgagattatgt | 286990 | - | see also 2951 line | | |
| 18951 | CYLC2 | NM_001340.1 | 165 | acgggacaacacggtttctataa | 287045 | - | - | structural constituent of cytoskeleton | - |
| 18952 | D10S170 | NM_005436.1 | 1569 | gaggttatctaggcttcactatc | 287081 | - | - | - | - |

Figure 46

| 18953 | DSP | NM_004415.1 | 7362 | gggccacggtattcgcttattag | 287269 | - | - | structural constituent of cytoskeleton | - |
|-------|-----|-------------|------|--------------------------|--------|---|---|----------------------------------------|---|
| 18954 | FLJ12785 | NM_024855.2 | 991 | gcgtctggaccgactgctatatg | 287325 | - | see also 8793 line | | |
| 18955 | GFAP | NM_002055.2 | 383 | gcggctgcggctcgatcaactca | 287337 | - | - | TBP-associated factor 125 | alexander disease |
| 18956 | INA | NM_032727.2 | 1270 | tagcaccagtgggttaagcattt | 287344 | - | - | structural constituent of cytoskeleton | - |
| 18957 | KRT1 | NM_006121.2 | 787 | gtgatcaatctcggttggattcg | 287361 | - | - | structural constituent of cytoskeleton | epidermolytic hyperkeratosis |
| 18958 | KRT13 | NM_002274.2 | 712 | gagctcactctgtctaagactga | 287377 | - | - | - | white sponge nevus |
| 18959 | KRT20 | NM_019010.1 | 130 | gggacgacacccagcgtttatgg | 287382 | - | - | structural constituent of cytoskeleton | - |
| 18960 | KRT2A | NM_000423.1 | 532 | cagcctctcaacgtgaaagttga | 287413 | - | - | structural molecule | epidermal ichthyosis bullosa of Siemens |
| 18961 | KRT5 | NM_000424.2 | 1876 | cagctccagcgtcaaatttgtct | 287428 | - | - | structural constituent of cytoskeleton | breast cancer、epidermolysis bullosa simplex |
| 18962 | KRT9 | NM_000226.1 | 1078 | aaggacatcgagaatcaatatga | 287442 | - | - | structural constituent of cytoskeleton | epidermolytic palmoplantar keratoderma |
| 18963 | LOC81569 | NM_030812.1 | 813 | gtgctacgtgccgcagaatctgg | 287456 | - | - | - | - |
| 18964 | LOR | NM_000427.1 | 284 | ccggtgggagcgtcaagtactcc | 287464 | - | - | structural constituent of cytoskeleton | progressive symmetrical erythrokeratoderma、Vohwinkel syndrome |
| 18965 | MSN | NM_002444.1 | 979 | ccgtcgcaagcctgataccattg | 287498 | - | see also 1580 line | | |
| 18966 | NEF3 | NM_005382.1 | 1134 | tcgtcatttgcgcgaataccagg | 287510 | - | - | structural constituent of cytoskeleton | - |
| 18967 | NEFL | NM_006158.1 | 1190 | aggagacccgactcagtttcacc | 287533 | - | - | structural constituent of cytoskeleton | - |
| 18968 | PPL | NM_002705.2 | 2477 | aagctgtaaaggactatgagtta | 287539 | - | - | structural constituent of cytoskeleton | - |
| 18969 | SCAM-1 | NM_005775.2 | 1004 | aggacctgcgggcaattgagacc | 287562 | - | - | structural constituent of cytoskeleton | - |
| 18970 | VIM | NM_003380.1 | 1469 | tagagatggacaggttatcaacg | 287584 | - | - | structural constituent of cytoskeleton | - |
| 18971 | COL11A2 | NM_080679.1 | 755 | aagtgctcgtccagtattggaca | 287591 | - | - | - | - |
| 18972 | AMELY | NM_001143.1 | 189 | tggtaccagagcatgataagacc | 287615 | - | - | structural constituent of tooth enamel | - |
| 18973 | CHAD | NM_001267.1 | 437 | cggcctcaagcaacttatctact | 287622 | - | - | extracellular matrix structural constituent | - |
| 18974 | COL10A1 | NM_000493.2 | 2066 | ccgagtcaaatggcctatactcc | 287644 | - | see also 386 line | | |
| 18975 | COL11A1 | NM_001854.2 | 1798 | atgttaccgttccgttatggtgg | 287678 | - | see also 1198 line | | |
| 18976 | COL12A1 | NM_004370.4 | 6093 | ctgattccggacacactctattc | 287845 | - | see also 2929 line | | |

EP 1 752 536 A1

763

Figure 46

| | | | | | | | | | |
|---|---|---|---|---|---|---|---|---|---|
| 18977 | COL15A1 | NM_001855.2 | 4085 | ttgatggtcgagacataatgaca | 287955 | - | - | extracellular matrix structural constituent | - |
| 18978 | COL16A1 | NM_001856.2 | 3069 | gggatccttaccaattgaacagc | 287979 | - | see also 1199 line | | |
| 18979 | COL18A1 | NM_030582.2 | 3122 | ctgtttacgacagcaatgtgttt | 287998 | - | - | - | - |
| 18980 | COL19A1 | NM_001858.2 | 737 | atggacggacagttattgctacg | 288017 | - | see also 1201 line | | |
| 18981 | COL1A1 | NM_000088.2 | 624 | gagaaatcaaccggaggaatttc | 288048 | - | - | Smad3/Sp-1 heterodimer | Ehlers-Danlos syndrome、osteogenesis imperfecta、dermatofibrosarcoma protuberans |
| 18982 | COL1A2 | NM_000089.2 | 148 | ttgtggatacgcggactttgttg | 288053 | - | see also 67 line | | |
| 18983 | COL27A1 | NM_032888.2 | 661 | gtgacgcaggtcgctcacaatta | 288086 | - | see also 9334 line | | |
| 18984 | COL2A1 | NM_001844.3 | 1517 | cagacgggtgaacctggtattgc | 288115 | - | - | - | achondrogenesis、arthroophthalmopathy(-)、hypochondrogenesis、Kniest dysplasia、osteoarthritis with mild chondrodysplasia、primary osteoarthritis、spondyloepimetaphyseal dysplasia、spondyloepiphyseal dysplasia |
| 18985 | COL3A1 | NM_000090.2 | 315 | atgtgacgatcaagaattagact | 288126 | - | - | ATP-dependent DNA helicase II、70 kDa subunit | Ehlers-Danlos syndrome、aneurysm |
| 18986 | COL4A1 | NM_001845.2 | 4175 | caggtccttacgacatcatcaaa | 288165 | - | - | Smad3/Sp-1 heterodimer | - |
| 18987 | COL4A2 | NM_001846.1 | 2022 | ccgcgatgggctcgatggattcc | 288191 | - | - | Smad3/Sp-1 heterodimer | - |
| 18988 | COL4A4 | NM_000092.2 | 5115 | tggactgggtatagtctgttata | 288232 | - | - | extracellular matrix structural constituent | Alport syndrome、hematuria |
| 18989 | COL4A5 | NM_000495.3 | 667 | gggtgaaccaggtagtataatta | 288248 | - | see also 388 line | | |
| 18990 | COL4A6 | NM_001847.1 | 4718 | caggcgcaatgataaatcttact | 288315 | - | see also 1195 line | | |
| 18991 | COL5A2 | NM_000393.2 | 4027 | ccgcacgtgtgatgacctaaagc | 288343 | - | see also 315 line | | |
| 18992 | COL5A3 | NM_015719.2 | 4838 | ctgcctctatcccgacaagaagt | 288368 | - | - | extracellular matrix structural constituent | - |
| 18993 | COL6A2 | NM_001849.2 | 1241 | aagggaagcaaggggtatcaagg | 288385 | - | - | - | Bethlem myopathy |
| 18994 | COL6A3 | NM_004369.1 | 7757 | gtgtgaggaacggattcctaatg | 288518 | - | - | - | Bethlem myopathy、Ullrich congenital muscular dystrophy |
| 18995 | COL7A1 | NM_000094.2 | 594 | caggggtcaagctatttgctgt | 288548 | - | - | serine protease inhibitor | bullous dermolysis、epidermolysis bullosa、epidermolysis bullosa dystrophica、epidermolysis bullosa simplex、toenail dystrophy、pruriginosa epidermolysis bullosa |

Figure 46

| 18996 | COL8A1 | NM_001850.3 | 345 | aagccgctgccacctcaaattcc | 288569 | - | - | - | - |
|---|---|---|---|---|---|---|---|---|---|
| 18997 | COL9A1 | NM_001851.2 | 2876 | tggtcagcgagcatttaacaaag | 288624 | - | see also 1197 line | | |
| 18998 | COL9A2 | NM_001852.3 | 1187 | gggcctcgaggagaaattggtcc | 288631 | - | - | extracellular matrix structural constituent | epiphyseal dysplasia |
| 18999 | COL9A3 | NM_001853.2 | 1620 | ggggatgatcagcgaacaaattg | 288633 | - | - | extracellular matrix structural constituent | - |
| 19000 | KAL1 | NM_000216.1 | 1069 | gggagtgtgaccgtcactatagt | 288650 | - | - | serine protease inhibitor | Kallmann syndrome |
| 19001 | LAMA4 | NM_002290.2 | 628 | tggttatatcggagattccatca | 288691 | - | - | structural molecule | - |
| 19002 | LUM | NM_002345.2 | 1098 | cagtcttccaccggatatgtatg | 288863 | - | - | translation regulator | osteosarcoma |
| 19003 | MAGP2 | NM_003480.1 | 546 | cagtttacgacgtatgtacatcg | 288873 | - | - | extracellular matrix structural constituent | - |
| 19004 | MATN1 | NM_002379.2 | 1505 | aggcggctgtgcggaatatgtcc | 288885 | - | see also 1532 line | | |
| 19005 | MATN3 | NM_002381.2 | 1437 | cagctcgtatcttcaaagactga | 288917 | - | - | extracellular matrix structural constituent | epiphyseal dysplasia |
| 19006 | MEPE | NM_020203.1 | 763 | cagcggttatacagatcttcaag | 288953 | - | - | - | - |
| 19007 | MFAP1 | NM_005926.1 | 174 | ccgtcccagttcgcaatgagaaa | 288978 | - | - | extracellular matrix structural constituent | - |
| 19008 | MFAP2 | NM_002403.2 | 477 | ccgccgtgtgtacgtcattaaca | 289012 | - | - | - | - |
| 19009 | MFAP3 | NM_005927.3 | 671 | gggaactagcgtttcaattgagt | 289023 | - | - | extracellular matrix structural constituent | - |
| 19010 | OPTC | NM_014359.2 | 227 | aggcgattcctttgaagttctgc | 289053 | - | - | extracellular matrix structural constituent | - |
| 19011 | PRELP | NM_002725.2 | 496 | aggcctgcgatggattaacctgg | 289070 | - | - | extracellular matrix structural constituent | - |
| 19012 | TECTA | NM_005422.1 | 509 | tagtgtccgatggctcctataca | 289093 | - | see also 3787 line | | |
| 19013 | TFPI2 | NM_006528.2 | 180 | ctgtctcctgcccctagactacg | 289204 | - | - | serine protease inhibitor | - |
| 19014 | TNR | NM_003285.1 | 2855 | cagctaccgaatacgaaatcagc | 289268 | - | see also 2220 line | | |
| 19015 | MYBPH | NM_004997.1 | 1327 | aggcgtctgcaccctagagatcc | 289294 | - | see also 3492 line | | |
| 19016 | MYOM1 | NM_003803.1 | 761 | gggcctcggcgatgaatgttaaa | 289310 | - | see also 2600 line | | |
| 19017 | MYOM2 | NM_003970.1 | 1829 | gcgaaccttcggagataacgtcc | 289420 | - | - | structural constituent of muscle | - |
| 19018 | TCAP | NM_003673.2 | 200 | gcgctcgccctggctgatgatgc | 289467 | - | - | structural constituent of muscle | - |
| 19019 | TTID | NM_006790.1 | 1318 | aagagcaccaatgtttatctaca | 289492 | - | see also 4781 line | | |
| 19020 | CRYAA | NM_000394.2 | 261 | tcgatccgaccgggacaagttcg | 289512 | - | see also 316 line | | |
| 19021 | CRYAB | NM_001885.1 | 405 | atgtagaccctctcaccattact | 289516 | - | see also 1208 line | | |
| 19022 | CRYBA1 | NM_005208.3 | 529 | tggatatcgtgggtatcagtata | 289537 | - | see also 3659 line | | |

Figure 46

| | | | | | | | | | |
|---|---|---|---|---|---|---|---|---|---|
| 19023 | CRYBA2 | NM_005209.1 | 539 | cagcggagagttctgtacttacg | 289546 | - | - | - | - |
| 19024 | CRYBA4 | NM_001886.1 | 501 | gggctaccgaggatttcagtatg | 289553 | - | - | structural constituent of eye lens | - |
| 19025 | CRYBB1 | NM_001887.3 | 279 | agggccgtcgagcagaattctcg | 289557 | - | - | structural constituent of eye lens | - |
| 19026 | CRYBB3 | NM_004076.3 | 346 | tggtccaacagccgtgatagtga | 289566 | - | - | structural constituent of eye lens | - |
| 19027 | CRYGA | NM_014617.2 | 609 | gagacgggtcaccgatttgtact | 289585 | - | - | structural constituent of eye lens | - |
| 19028 | CRYGB | NM_005210.2 | 540 | tagacgagtcatggatttgtact | 289595 | - | - | structural constituent of eye lens | - |
| 19029 | CRYGD | NM_006891.2 | 358 | ccgcttccgcttcaatgaaatcc | 289600 | - | - | structural constituent of eye lens | cataract |
| 19030 | CRYGS | NM_017541.2 | 324 | gaggccagtataagattcagatc | 289612 | - | - | structural constituent of eye lens | - |
| 19031 | LIM2 | NM_030657.2 | 633 | gcgcctaccgggtgcatgaatgc | 289616 | - | - | structural constituent of eye lens | - |
| 19032 | AMBN | NM_016519.2 | 892 | atggcctatggagccatgtttcc | 289629 | - | - | - | - |
| 19033 | ENAM | NM_031889.1 | 906 | gggggtcgccctccttattattc | 289659 | - | see also 9042 line | | |
| 19034 | TUFT1 | NM_020127.1 | 959 | gcggaaagtccggcaaatgatag | 289742 | - | - | - | - |
| 19035 | KRTHA6 | NM_003771.3 | 1236 | atgcaagcctgttattagagttc | 289754 | - | - | structural constituent of epidermis | - |
| 19036 | KRTHB2 | NM_033033.3 | 755 | ctgcgtccctgtgttgagaatga | 289757 | - | - | - | - |
| 19037 | KRTHB4 | NM_033045.2 | 1030 | ctgaaccttgatgggatcattgc | 289765 | - | - | - | - |
| 19038 | AF053356_CDS3 | NM_023948.3 | 521 | ttgacgcagagggatatgtgaag | 289766 | - | - | - | - |
| 19039 | ARVCF | NM_001670.1 | 1673 | ctgtggaacctgtcatcctatga | 289771 | - | - | structural molecule | - |
| 19040 | BFAR | NM_016561.1 | 623 | tggtcctgctcgtctatcactgg | 289778 | - | see also 6914 line | | |
| 19041 | C21orf29 | NM_144991.2 | 201 | aagcgggatcaggatagttcagg | 289795 | - | - | - | - |
| 19042 | CAV1 | NM_001753.3 | 702 | cagtgcatcagccgtgtctattc | 289819 | - | see also 1158 line | | |
| 19043 | CLDN1 | NM_021101.3 | 640 | tggcaatagaatcgttcaagaat | 289825 | - | - | structural molecule | - |
| 19044 | CLDN10 | NM_006984.3 | 649 | atgtcttctcggacaaagtatca | 289846 | - | - | - | - |
| 19045 | CLDN12 | NM_012129.2 | 400 | acgacactacttggtactcatca | 289867 | - | - | structural molecule | - |
| 19046 | CLDN14 | NM_144492.1 | 1321 | ctgcccagcggcatgaagtttga | 289876 | - | - | structural molecule | - |
| 19047 | CLDN15 | NM_014343.1 | 258 | tcgatggctgtggaaacctttgg | 289879 | - | - | - | - |
| 19048 | CLDN17 | NM_012131.1 | 620 | cagataagcgaagaaatacgaca | 289899 | - | - | structural molecule | - |
| 19049 | CLDN18 | NM_016369.1 | 749 | cagaggacgaggtacaatcttat | 289912 | - | - | structural molecule | Alzheimer disease |
| 19050 | CLDN19 | NM_148960.1 | 808 | ctgcccgagaaccagttgttaaa | 289917 | - | - | - | - |

Figure 46

| | | | | | | | | | | |
|---|---|---|---|---|---|---|---|---|---|---|
| 19051 | CLDN2 | NM_020384.2 | 890 | gagaaatcgtccaactactacg | - | 289920 | - | | structural molecule | - |
| 19052 | CLDN5 | NM_003277.2 | 878 | ctgctcgtttcgccaacattgtcg | - | 289923 | - | | structural molecule | Alzheimer disease |
| 19053 | CLDN6 | NM_021195.3 | 669 | ctgagtaccctaccaagaattac | - | 289926 | - | | structural molecule | - |
| 19054 | CLDN7 | NM_001307.3 | 530 | atgcgggtgacaacatcatcacg | - | 289927 | - | | structural molecule | - |
| 19055 | CLDN8 | NM_012132.3 | 197 | tggagagtgtcggccttcattga | - | 289936 | - | | structural molecule | - |
| 19056 | CLTC | NM_004859.1 | 2875 | cagtccgttgttggaaagtatt | - | 290042 | - | | structural molecule | inflammatory myofibroblastic tumors, inflammatory myofibroblastic tumor |
| 19057 | COL21A1 | NM_030820.2 | 396 | ttgacatagggccgaagtttatt | - | 290115 | - | | - | - |
| 19058 | CTNNA1 | NM_001903.1 | 1339 | aggtgtaaagcttgttcgaatgt | - | 290190 | - | see also 1216 line | - | - |
| 19059 | CTNND2 | NM_001332.2 | 2668 | gttggcacccatcaatagtcaaac | - | 290235 | - | | structural molecule | - |
| 19060 | DCTN3 | NM_007234.3 | 487 | tggaggaatacaacaagactaca | - | 290257 | - | | - | - |
| 19061 | DJ473B4 | NM_019556.1 | 419 | gtgattcgtcatcgagatgttcg | - | 290267 | - | | - | - |
| 19062 | DRP2 | NM_001939.1 | 1264 | cagatcaacgtccgatggaaaca | - | 290290 | - | | structural molecule | - |
| 19063 | EVPL | NM_001988.1 | 1861 | aggagtgcgaggcgtttctgtcc | - | 290316 | - | | structural molecule | - |
| 19064 | FLJ13646 | NM_024584.2 | 185 | acgatctgaacaagcatataaa | - | 290324 | - | | - | - |
| 19065 | FLJ32214 | NM_152473.1 | 965 | ggggcgatagaggaggagataaaa | - | 290358 | - | signal_transduction | - | - |
| 19066 | HSPG2 | NM_005529.2 | 12335 | cagggcatcgggcaatgctatga | - | 290415 | - | | structural molecule | Schwartz-Jampel-Aberfeld syndrome, Silverman-Handmaker type dyssegmental dysplasia |
| 19067 | HUMCYT2A | NM_015848.1 | 758 | aagcagctagattcacttctagg | - | 290420 | - | | structural molecule | - |
| 19068 | ITGBL1 | NM_004791.1 | 1049 | gagctgtcatgaccgatattct | - | 290442 | - | see also 3327 line | - | - |
| 19069 | IVL | NM_005547.1 | 65 | ctgttcctccagtcaataac | - | 290456 | - | | - | - |
| 19070 | K6HF | NM_004693.1 | 1611 | cagtggttctagcgtcaagtttg | - | 290471 | - | | - | - |
| 19071 | KRT12 | NM_000223.1 | 1145 | ccgaagccgaggcgattactgc | - | 290491 | - | | structural molecule | Meesmann corneal dystrophy |
| 19072 | KRT4 | NM_002272.1 | 1324 | cagtcttaatgacgagatcaact | - | 290499 | - | | structural molecule | - |
| 19073 | KRT7 | NM_005556.2 | 1347 | gagtgggagccgtgaatatctct | - | 290505 | - | | structural molecule | breast cancer |
| 19074 | KRTHA7 | NM_003770.3 | 1672 | aggcccgagttaggagattgagg | - | 290510 | - | | structural molecule | - |
| 19075 | KRTHB5 | NM_002283.2 | 935 | cagacacctcggtcatagtcaag | - | 290513 | - | | - | - |
| 19076 | KRTHB6 | NM_002284.2 | 1309 | aggcgtcggctcggtgaatgtct | - | 290514 | - | | - | - |
| 19077 | LAD1 | NM_005558.3 | 1552 | cagcaagcgccacctcttgaga | - | 290521 | - | | structural molecule | monilethrix |
| 19078 | LAMA1 | NM_005559.2 | 4352 | ctgaactgtggcgataacacagc | - | 290591 | - | see also 3865 line | | - |
| 19079 | LAMA2 | NM_000426.2 | 763 | tcgctatattggcctgagattc | - | 290700 | - | see also 327 line | | - |
| 19080 | LAMA3 | NM_000227.2 | 185 | ctggatactatgggatcataaa | - | 290937 | - | signal_transduction | structural molecule | epidermolysis bullosa |
| 19081 | LAMB2 | NM_002292.2 | 4326 | cagaacgtcgtgccaatacctca | - | 291296 | - | see also 1470 line | - | - |
| 19082 | LAMB3 | NM_000228.1 | 2736 | cagacgcaacgcttcctaatcc | - | 291333 | - | see also 170 line | - | - |
| 19083 | LAMC1 | NM_002293.2 | 1357 | tcgaccctgaactctatcgttcc | - | 291361 | - | see also 1471 line | - | - |
| 19084 | LAMC3 | NM_006059.2 | 3993 | gcgacctacggcaaaacagaacc | - | 291449 | - | | structural molecule | - |

Figure 46

| No. | Gene | Accession | Length | Sequence | | ID | | See also | | Function | | Disease | |
|---|---|---|---|---|---|---|---|---|---|---|---|---|---|
| 19085 | LCMR1 | NM_153450.1 | 433 | cagtagctctttcaatcctatca | - | 291456 | - | - | - | - | - | - | - |
| 19086 | LMNB1 | NM_005573.2 | 762 | cagatcaagcttcgagaatatga | - | 291460 | - | see also 3873 line | - | - | - | - | - |
| 19087 | LOC126147 | NM_145807.1 | 1263 | gcgcgtgctggccgtttacaagc | - | 291500 | - | - | - | - | - | - | - |
| 19088 | MAP1A | NM_002373.3 | 7003 | cagaaccccgcatgatgaataacc | - | 291570 | - | - | - | structural molecule | - | - | - |
| 19089 | MAP1B | NM_005909.2 | 728 | aagagatcggggttactaagc | - | 291596 | - | see also 4125 line | - | - | - | - | - |
| 19090 | MAP4 | NM_002375.2 | 3274 | tgggtctaaggctaacatccaagc | - | 291792 | - | - | - | - | - | - | - |
| 19091 | MAP7 | NM_003980.2 | 885 | ctgcacactcagaaattgatg | - | 291805 | - | - | - | - | - | - | - |
| 19092 | MBP | NM_002385.1 | 441 | gaggcagagcgtccgactataaa | - | 291833 | - | - | - | structural molecule | - | - | - |
| 19093 | MEGF10 | NM_032446.1 | 2901 | aagggtcgtcaatgcagatattac | - | 291878 | - | - | - | - | - | - | - |
| 19094 | MGC26706 | NM_152581.1 | 579 | ttgtacgctttatcatcaactgc | - | 291919 | - | - | - | - | - | - | - |
| 19095 | MPZ | NM_000530.3 | 376 | tggtccattgtcatcacaaac | - | 291955 | - | - | - | structural molecule | - | Charcot-Marie-Tooth disease, Roussy-Levy syndrome, Charcot-Marie-Tooth neuropathy, hereditary motor and sensory neuropathy | - |
| 19096 | MYOC | NM_000261.1 | 1359 | atgctaccgtcaactttgcttat | - | 291973 | - | receptor acitivity | - | structural molecule | - | glaucoma | - |
| 19097 | NF2 | NM_000268.2 | 512 | acgccgagatggagttcaattgc | - | 291979 | - | see also 225 line | - | - | - | - | - |
| 19098 | NMP200 | NM_014502.3 | 666 | gtgcgggtgagccaatggattg | - | 292022 | - | - | - | - | - | - | - |
| 19099 | NTN1 | NM_004822.1 | 1116 | acgagtgcgtggcctgtaactgc | - | 292044 | - | - | - | - | - | - | - |
| 19100 | NTN4 | NM_021229.2 | 1369 | ctgtgccccgttatacaatgacc | - | 292067 | - | - | - | - | - | - | - |
| 19101 | NUMA1 | NM_006185.1 | 5705 | gcgccaactcatcgttctacagc | - | 292107 | - | see also 4275 line | - | - | - | - | - |
| 19102 | ODF1 | NM_024410.2 | 169 | ctgagatgcatcgacgaatttag | - | 292112 | - | - | - | structural molecule | - | - | - |
| 19103 | ODF2 | NM_002540.3 | 2200 | cagctagaacgctgtgacaaaga | - | 292156 | - | see also 1635 line | - | - | - | - | - |
| 19104 | OSF-2 | NM_006475.1 | 769 | ctgccatcacatcggacatattg | - | 292193 | - | see also 4545 line | - | - | - | - | - |
| 19105 | PKP3 | NM_007183.1 | 958 | ceggacgtgcatgggttcaacagc | - | 292243 | - | see also 4985 line | - | - | - | - | - |
| 19106 | PKP4 | NM_003628.2 | 3125 | gggacctccggagcatttataaa | - | 292326 | - | - | - | structural molecule | - | - | - |
| 19107 | PLP1 | NM_000533.2 | 524 | ttggagcgggtgtgtcattgttt | - | 292355 | - | see also 403 line | - | - | - | - | - |
| 19108 | PRPH | NM_006262.2 | 1299 | cagcccgaagacggttctgatca | - | 292404 | - | - | - | structural molecule | - | - | - |
| 19109 | SPON2 | NM_012445.1 | 895 | aggacacggtgaccgagataacg | - | 292405 | - | - | - | - | - | - | - |
| 19110 | TEKT1 | NM_053285.1 | 1094 | gtgtcgtgatgtcgcacaatata | - | 292420 | - | see also 9495 line | - | - | - | - | - |
| 19111 | TEKT2 | NM_014466.2 | 848 | gaggccactgctctaactattgc | - | 292437 | - | see also 5697 line | - | - | - | - | - |
| 19112 | TEKT3 | NM_031898.1 | 1311 | aagacggccgaacattgagttgt | - | 292483 | - | - | - | - | - | - | - |
| 19113 | THOC1 | NM_005131.1 | 49 | aagcggacgacgcggtttacgaag | - | 292488 | - | see also 3606 line | - | - | - | - | - |
| 19114 | UPK1B | NM_006952.2 | 573 | ctgcttccgactgagaataat | - | 292537 | - | - | - | structural molecule | - | - | - |
| 19115 | USH2A | NM_007123.3 | 854 | ctgagcaacaaggtgaatgtgt | - | 292560 | - | see also 4957 line | - | - | - | - | - |
| 19116 | VIL2 | NM_003379.3 | 458 | aggaatccttagcgatgagatct | - | 292759 | - | - | - | structural molecule | - | - | - |

Figure 46

| 19117 | PI15 | NM_015886.1 | 1030 | tcggataggatgcgcaattcata | 292792 | - | see also 6561 line | | |
|---|---|---|---|---|---|---|---|---|---|
| 19118 | ECG2 | NM_032566.1 | 273 | aagtaatggaagagttcagtttc | 292813 | - | - | serine protease inhibitor | - |
| 19119 | FKSG28 | NM_030929.3 | 843 | cagatcgtgtcacatccatatga | 292814 | - | - | - | - |
| 19120 | ITIH2 | NM_002216.1 | 2634 | cggactaataggccagttcatgc | 292903 | - | see also 1410 line | | |
| 19121 | ITIH3 | NM_002217.1 | 1258 | ggggcaagttccccttgtataac | 292926 | - | - | serine protease inhibitor | - |
| 19122 | ITIH4 | NM_002218.3 | 205 | gtggtcaatagggccaatactgt | 292943 | - | - | serine protease inhibitor | - |
| 19123 | KRTHB3 | NM_002282.2 | 82 | tgggttccgccctggaaacttca | 292979 | - | - | - | - |
| 19124 | OL-64 | NM_173850.2 | 1198 | tgggaacacgctgttcattgacc | 292985 | - | - | - | - |
| 19125 | PZP | NM_002864.1 | 1437 | gagactatcacggcacactatac | 293038 | - | - | wide-spectrum protease inhibitor | - |
| 19126 | RECK | NM_021111.1 | 2423 | tcgcgtggcagtcgattactatg | 293155 | - | see also 8086 line | | |
| 19127 | SERPINA1 | NM_000295.2 | 1148 | aggtctgccagcttacatttacc | 293169 | - | - | Hepatocyte nuclear factor 1 | rheumatoid arthritis、 atherosclerosis、 alpha-1-antitrypsin deficiency、 emphysema、 hepatocellular carcinoma/cirrhosis |
| 19128 | SERPINA10 | NM_016186.1 | 1713 | tggaccggccatttcatttcatg | 293191 | - | - | serpin | - |
| 19129 | SERPINA11 | NM_175739.2 | 614 | aagtgccctcttcgtcaagaagg | 293195 | - | - | - | - |
| 19130 | SERPINA3 | NM_001085.2 | 761 | ttgcatcacctgactatacctta | 293208 | - | - | serpin | atherosclerosis、 Alzheimer disease、 Parkinson disease |
| 19131 | SERPINB1 | NM_030666.2 | 487 | tggcttcgggcatggttgataac | 293225 | - | - | serpin | - |
| 19132 | SERPINB11 | NM_080475.1 | 787 | ctgaattcggggacgtttcatga | 293272 | - | - | serpin | - |
| 19133 | SERPINB13 | NM_012397.2 | 1071 | cgggttgtacgcccagaagttcc | 293297 | - | - | serpin | - |
| 19134 | SERPINB3 | NM_006919.1 | 509 | tgggtggaaagtcaaacgaatga | 293317 | - | - | serpin | - |
| 19135 | SERPINB5 | NM_002639.1 | 343 | aagcggctctacgtagacaaatc | 293337 | - | see also 1713 line | | |
| 19136 | SERPINB7 | NM_003784.1 | 743 | gagcgagttgactttacgaatca | 293373 | - | - | serpin | - |
| 19137 | SERPINF1 | NM_002615.3 | 449 | cagcgaacagaatccatcattca | 293400 | - | - | serpin | - |
| 19138 | SERPINF2 | NM_000934.1 | 1030 | ctgcctaagctgtatctgaaaca | 293422 | - | - | serpin | plasmin inhibitor deficiency |
| 19139 | SERPING1 | NM_000062.1 | 109 | ggggatagagcctcctcaaatcc | 293424 | - | - | serpin | hereditary angioneurotic edema、 partial deficiency of complement component 4 |
| 19140 | SERPINI1 | NM_005025.1 | 1096 | aagtccttcctagaggttaatga | 293455 | - | - | serpin | encephalopathy |
| 19141 | SERPINI2 | NM_006217.2 | 946 | ttgaacataaccgagatatttag | 293476 | - | - | serpin | - |

Figure 46

| | | | | | | | | |
|---|---|---|---|---|---|---|---|---|
| 19142 | SLPI | NM_003064.2 | 291 | cagtgacttatggccaatgtttg | 293489 | - | | serine protease inhibitor | - |
| 19143 | SPINK1 | NM_003122.1 | 103 | ttgagtctatctggtaacactgg | 293494 | - | | translation regulator | - |
| 19144 | SPINK2 | NM_021114.1 | 275 | caggaaggtggtcataatatta | 293505 | - | | serine protease inhibitor | - |
| 19145 | SPINK4 | NM_014471.1 | 178 | ctgatggctcacatatacgaat | 293507 | - | | serine protease inhibitor | - |
| 19146 | SPINK5 | NM_006846.1 | 2235 | ctgatggcaaatgtacaacaat | 293543 | - | | serine protease inhibitor | |
| 19147 | SPINLW1 | NM_020398.2 | 185 | cagacaatgccaggacaacaaga | 293567 | - | | | |
| 19148 | SPINT1 | NM_003710.2 | 1025 | cagatctgcaagagtttcgttta | 293577 | - | see also 2534 line | | |
| 19149 | SPINT2 | NM_021102.1 | 669 | cagcgatatgttcaactatgaag | 293593 | - | | serine protease inhibitor | - |
| 19150 | UNC5D | NM_080872.1 | 1538 | gagtaccacggcaagaatcattc | 293625 | - | see also 9533 line | | - |
| 19151 | WFDC10A | NM_080753.2 | 239 | ccgagattgtcaagcaaataaca | 293647 | - | | | - |
| 19152 | WFDC12 | NM_080869.1 | 84 | aggagttaaagagggtatagaga | 293648 | - | | | - |
| 19153 | WFDC3 | NM_080614.1 | 742 | gtgaggtctgattccgaattaga | 293664 | - | | | - |
| 19154 | WFDC8 | NM_130896.1 | 80 | atgtggactgtccgaactgaagg | 293667 | - | | | - |
| 19155 | AHSG | NM_001622.1 | 175 | gttggctatagactacatcaatca | 293687 | - | kinase_related, signal transduction | cysteine protease inhibitor | - |
| 19156 | BIRC4 | NM_001167.2 | 301 | gagataccgtccggtgctttagt | 293703 | - | apoptosis | cysteine protease inhibitor | - |
| 19157 | BIRC5 | NM_001168.1 | 290 | tcgtccggttgcgtcttcttc | 293740 | - | apoptosis, cell cycle | cysteine protease inhibitor | hepatocellular carcinoma, colorectal cancer, non-hodgkin lymphoma, breast cancer, lung cancer, neuroblastoma, melanoma, colorectal cancers |
| 19158 | CAST | NM_001750.4 | 1029 | gagctgacctcgctcaattaa | 293760 | - | | cysteine protease inhibitor | Alzheimer disease |
| 19159 | CST11 | NM_080830.2 | 230 | aggatcttccgagtccttaaagt | 293794 | - | | | - |
| 19160 | CST5 | NM_001900.2 | 245 | ggggtgaactactacttcaatgt | 293801 | - | | cysteine protease inhibitor | - |
| 19161 | CST7 | NM_003650.2 | 506 | gggccctagttcagatagtgaaa | 293812 | - | | cysteine protease inhibitor | - |
| 19162 | CST8 | NM_005492.2 | 534 | gagcgaggacaagtatgtcttcc | 293815 | - | | cysteine protease inhibitor | - |
| 19163 | CST9L | NM_080610.1 | 446 | cagactggggcacatcttgaatt | 293823 | - | | cysteine protease inhibitor | - |
| 19164 | CSTA | NM_005213.2 | 203 | ctggaacaaattactacattaag | 293826 | - | | cysteine protease inhibitor | - |

Figure 46

| | | | | | | | | | |
|---|---|---|---|---|---|---|---|---|---|
| 19165 | CSTB | NM_000100.2 | 255 | cggggacaaactacttcatcaag | 293831 | - | - | cysteine protease inhibitor | myoclonic epilepsy |
| 19166 | KNG | NM_000893.2 | 1258 | tagattgcaacgctgaagtttat | 293871 | - | apoptosis | microtubule motor | total kininogen deficiency |
| 19167 | TIMP2 | NM_003255.2 | 503 | gaggatccagtatgagatcaagc | 293879 | - | see also 2198 line | | |
| 19168 | TIMP4 | NM_003256.1 | 267 | atgctccggtatgaaatcaaaca | 293884 | - | see also 2200 line | | |
| 19169 | C4A | NM_007293.1 | 2792 | tggctcgagggtccttcgaattc | 329787 | - | - | endopeptidase inhibitor | systemic lupus erythematosis |
| 19170 | CD109 | NM_133493.1 | 894 | aaggagacgtaacgcttacattt | 293916 | - | see also 9588 line | | |
| 19171 | FLJ25179 | NM_144670.1 | 314 | atggagggcaccaatcagttact | 294036 | - | - | - | - |
| 19172 | SPP2 | NM_006944.1 | 53 | ttgctcttggaatgaactactgg | 294039 | - | - | endopeptidase inhibitor | - |
| 19173 | VIP | NM_015692.1 | 4835 | tggacgccgagtgctcttctact | 294077 | - | - | - | - |
| 19174 | DEFB126 | NM_030931.2 | 96 | gtgtctaaacgacgttggaattt | 294088 | - | - | - | - |
| 19175 | CDKN1C | NM_000076.1 | 314 | tgggaccttcccagtactagtgc | 294096 | - | kinase_related、cell_cycle | nutrient reservoir | hepatocellular carcinoma、Beckwith-Wiedemann syndrome、Ewing's sarcoma |
| 19176 | CDKN2C | NM_001262.2 | 1392 | gaggtgctaatcccgatttgaaa | 294107 | - | see also 822 line | | |
| 19177 | PKIA | NM_006823.2 | 685 | aagatgcacaacgaagttctaca | 294118 | - | kinase_related | - | - |
| 19178 | DNAJC3 | NM_006260.2 | 487 | atgcagcgtttgcgttcacaagc | 294136 | - | kinase_related | - | - |
| 19179 | MBIP | NM_016586.1 | 135 | ccgagaggtgctctacgaaatct | 294174 | - | see also 6926 line | | |
| 19180 | PPP1R1B | NM_032192.2 | 1011 | gtggaagacccagcactaagtga | 294199 | - | kinase_related、phosphatase、signal_transduction | - | - |
| 19181 | SOCS1 | NM_003745.1 | 375 | tggacgcctgcggattctactgg | 294200 | - | kinase_related | protein kinase inhibitor | - |
| 19182 | PPP1R1A | NM_006741.2 | 396 | aaggatcacacccacaatgaaag | 294205 | - | see also 4763 line | | |
| 19183 | C7orf16 | NM_006658.2 | 176 | tcgttgcagccacttagatgatc | 294208 | - | phosphatase、signal_transduction、kinase_related | protein phosphatase inhibitor | - |
| 19184 | CABIN1 | NM_012295.2 | 4191 | ttgaccacgattacgtcaaatgt | 294266 | - | phosphatase、signal_transduction | protein phosphatase inhibitor | - |
| 19185 | I-4 | NM_025210.1 | 324 | ccgcgcaacgtacagagattacg | 294291 | - | phosphatase | - | - |
| 19186 | ANP32E | NM_030920.2 | 865 | tggtccaccggaaggatatgagg | 294315 | - | see also 8963 line | | |
| 19187 | SNCB | NM_003085.2 | 355 | agggaggaattccctactgatct | 294319 | - | - | phospholipase inhibitor | Parkinson disease |
| 19188 | GPS1 | NM_004127.3 | 1189 | acgtgtcagccgacatgcatagg | 294329 | - | cell_cycle、kinase_related | GTPase inhibitor | - |

EP 1 752 536 A1

Figure 46

| | | | | | | | | | |
|---|---|---|---|---|---|---|---|---|---|
| 19189 | OAZ1 | NM_004152.2 | 447 | cagacgccaaacgcattaactgg | 294402 | - | - | ornithine decarboxylase inhibitor | |
| 19190 | PSMF1 | NM_006814.2 | 366 | atgtcctccggtatgagtataag | 294411 | - | - | | |
| 19191 | RNH | NM_002939.2 | 2703 | tggtcctgtacgacatttactgg | 294418 | - | - | ribonuclease inhibitor | - |
| 19192 | ARRB1 | NM_004041.2 | 1105 | gagacgccagtagataccaatct | 294431 | - | gpcr、 signal_transduction | | - |
| 19193 | ATPIF1 | NM_016311.3 | 238 | caggctgaagaggaacgatattt | 294435 | - | - | enzyme inhibitor | - |
| 19194 | NF1 | NM_000267.1 | 5708 | ctgctcaatatcgcattacttaa | 294536 | - | see also 211 line | | |
| 19195 | ALS2 | NM_020919.2 | 4717 | atgtgtccttcgactaaataagc | 294766 | - | see also 8023 line | | |
| 19196 | ARHGEF3 | NM_019555.1 | 256 | ccgagtcacgtcgctagcaaacc | 294784 | - | signal_transduction | - | - |
| 19197 | ARHGEF4 | NM_015320.2 | 2131 | ccgcgacgtgttgtactacaagg | 294826 | - | - | - | - |
| 19198 | ARHGEF6 | NM_004840.1 | 1427 | gaggagcggtaccttatgttatt | 294862 | - | see also 3355 line | | |
| 19199 | FARP1 | NM_005766.1 | 903 | tagtgcgtaccaggataccttgg | 294911 | - | - | - | - |
| 19200 | FBXO8 | NM_012180.1 | 367 | ctgcgagcaacatttctaacacc | 294945 | - | see also 5182 line | | |
| 19201 | GBF1 | NM_004193.1 | 2913 | aggcttggttcgggagaactatg | 295043 | - | - | guanyl-nucleotide release factor | - |
| 19202 | PSCD1 | NM_004762.1 | 757 | gagctcctccggaatctctatga | 295086 | - | - | - | - |
| 19203 | PSCD2 | NM_004228.3 | 1280 | gagtgtggaccccttctatgaga | 295098 | - | - | - | - |
| 19204 | PSCD4 | NM_013385.2 | 1079 | gagccacggggaattatacctct | 295108 | - | - | guanyl-nucleotide release factor | - |
| 19205 | RAB2L | NM_004761.2 | 1696 | acgggtgcttcggccaacattgg | 295129 | - | signal_transduction | guanyl-nucleotide release factor | |
| 19206 | RASGRF1 | NM_002891.3 | 2667 | ctgtcgtgaactggacaataacc | 295167 | - | see also 1929 line | | |
| 19207 | DELGEF | NM_012139.2 | 612 | gggactggtttggcatcatgtgg | 295191 | - | - | - | - |
| 19208 | WBSCR16 | NM_030798.2 | 438 | ctgcggatgttacgaaagtctgg | 295201 | - | - | - | - |
| 19209 | RIN2 | NM_018993.2 | 2658 | acgcatcaagaacgatccttatg | 295249 | - | see also 7644 line | | |
| 19210 | RGL | NM_015149.2 | 2434 | ctgtctcggtgacgtccattacc | 295292 | - | signal_transduction | guanyl-nucleotide release factor | - |
| 19211 | ABR | NM_001092.3 | 459 | aagcgtttgtcgataactataaa | 295303 | - | signal_transduction | - | medulloblastoma |
| 19212 | ARFGEF2 | NM_006420.1 | 2309 | atgtcctagcgtattcaattatt | 295363 | - | - | - | - |
| 19213 | ARHGEF7 | NM_003899.2 | 1589 | gagcatgattgagcggatattag | 295461 | - | see also 2656 line | | |
| 19214 | BCAR3 | NM_003567.1 | 1926 | ccgagcggccactctgagtaaga | 295508 | - | cell_cycle、 signal_transduction | | breast cancer |
| 19215 | BIG1 | NM_006421.2 | 3327 | tggagtgaaacctcgatacattt | 295600 | - | - | guanyl-nucleotide release factor | - |
| 19216 | ECT2 | NM_018098.4 | 626 | gtgagtctaggtactccaattat | 295694 | - | see also 7254 line | | |
| 19217 | FLJ10244 | NM_018037.1 | 420 | atgcgggtcagataacattaatg | 295773 | - | see also 7215 line | | |

Figure 46

| | | | | | | | | | |
|---|---|---|---|---|---|---|---|---|---|
| 19218 | GPIG4 | NM_152545.1 | 293 | tagtacctaatgtggattactat | 295832 | - | - | - | - |
| 19219 | HAPIP | NM_003947.1 | 322 | gcggaaactcgtgacgtatttgg | 295878 | - | signal_transduction | guanyl-nucleotide release factor | - |
| 19220 | KIAA1010 | NM_015221.1 | 741 | tggggtcgccctgtacagattcc | 295945 | - | - | - | - |
| 19221 | Link-GEFII | NM_016339.1 | 1132 | aagaacttgtttcgcaaatttga | 296053 | - | signal_transduction、g pcr | - | - |
| 19222 | LOC221002 | NM_145313.1 | 639 | ccggccaccggctgtagacaagg | 296062 | - | - | - | - |
| 19223 | MCF2 | NM_005369.2 | 1068 | gaggtgcaatgagctacgttacc | 296102 | - | see also 3742 line | | |
| 19224 | MCF2L | NM_024979.2 | 327 | aggacggaagcccggttatcacc | 296157 | - | - | guanyl-nucleotide release factor | - |
| 19225 | NET1 | NM_005863.2 | 917 | tcgaagtcgcctagtcaaatacc | 296217 | - | - | - | - |
| 19226 | PDZGEF1 | NM_014247.1 | 3762 | ggggatcgcgcgtcacttgatgc | 296329 | - | - | - | - |
| 19227 | PDZGEF2 | NM_016340.1 | 667 | gagcgccaaaccattactgatga | 296370 | - | see also 6823 line | | |
| 19228 | RALGDS | NM_006266.1 | 315 | gagtcggccctgaacctttatga | 296458 | - | see also 4357 line | | |
| 19229 | RALGPS1A | NM_014636.1 | 1837 | cagactggttcccgatttcatgc | 296493 | - | see also 5810 line | | |
| 19230 | RASGRF2 | NM_006909.1 | 2782 | aagcacgcacaggatttcgaact | 296592 | - | see also 4847 line | | |
| 19231 | Rgr | NM_153615.1 | 1559 | ctgggcaacacgcattaacaatg | 296620 | - | signal_transduction | - | - |
| 19232 | SOS2 | NM_006939.1 | 3805 | agggtaccgcgtcgatgctatgt | 296727 | - | - | - | - |
| 19233 | GRAF | NM_015071.2 | 2368 | cagcaggcacggtcttcgataat | 296770 | - | - | - | - |
| 19234 | MIG-6 | NM_018948.2 | 1566 | aagcgtaaacatttatcctatgt | 296820 | - | - | - | - |
| 19235 | OPHN1 | NM_002547.1 | 760 | aagacggcaacgcgcttatcagc | 296822 | - | see also 1641 line | | |
| 19236 | PARG1 | NM_004815.2 | 3309 | ttgcctgtagatagactacttct | 296965 | - | see also 3345 line | | |
| 19237 | IQGAP3 | NM_178229.3 | 4399 | cggaacctacgccgacttgaagc | 297030 | - | - | - | - |
| 19238 | RASA1 | NM_002890.1 | 2238 | gtgttcgagcacgatactctatg | 297093 | - | see also 1924 line | | |
| 19239 | RASA2 | NM_006506.2 | 1360 | gagaatctgcgctactatgtgaa | 297178 | - | see also 4550 line | | |
| 19240 | RASAL2 | NM_004841.2 | 587 | caggacagttcaacctaataagg | 297227 | - | signal_transduction | - | - |
| 19241 | ARHGDIB | NM_001175.1 | 556 | aagtggataaagcaacatttatg | 297304 | - | signal_transduction | Rho GDP-dissociation inhibitor | - |
| 19242 | ARHGDIG | NM_001176.1 | 467 | aggtccacagggagattgtcagc | 297310 | - | signal_transduction | Rho GDP-dissociation inhibitor | - |
| 19243 | GDI2 | NM_001494.2 | 681 | ttgggtcaagacgttatagattt | 297321 | - | - | RAB GTPase activator | - |
| 19244 | GDI1 | NM_001493.1 | 694 | ccgtcaaccgcatcaagttgtac | 297360 | - | signal_transduction | RAB GTPase activator | mental retardation |
| 19245 | RIN3 | NM_024832.3 | 2612 | gggggagggttcctactatctga | 297442 | - | see also 8771 line | | |

Figure 46

| 19246 | RANGAP1 | NM_002883.2 | 829 | cagaaaccgtctggagaatgatg | 297449 | - | signal_transduction | RAN GTPase activator | - |
| 19247 | SIPA1 | NM_006747.2 | 797 | ctggctactaccgcaaatacttc | 297461 | - | apoptosis、cell_cycle | - | - |
| 19248 | BART1 | NM_012106.2 | 419 | atgaagtggctggtgacatattc | 297468 | - | signal_transduction | - | - |
| 19249 | CCM1 | NM_004912.3 | 1893 | ttgaggccactcgcatattgtta | 297500 | - | see also 3413 line | | |
| 19250 | DKFZp761J1523 | NM_032293.3 | 2388 | gtggcctccaggtcggatatata | 297602 | - | see also 9145 line | | |
| 19251 | MINK | NM_015716.2 | 2164 | ctgggtccgcccagataacgagg | 297630 | - | see also 6540 line | | |
| 19252 | RPIP8 | NM_006695.3 | 685 | cggaccaccagacggttctatga | 297644 | - | signal_transduction | - | - |
| 19253 | WAS | NM_000377.1 | 728 | aagatcagcaaagctgatattgg | 297651 | - | - | small GTPase regulatory/interacting protein | rheumatoid arthritis、eczema-thrombocytopenia-immunodeficiency syndrome、Wiskott-Aldrich syndrome、thrombocytopenia |
| 19254 | BNIP2 | NM_004330.1 | 589 | tggacgacgctggcgtatgttca | 297687 | - | apoptosis | apoptosis inhibitor | - |
| 19255 | DOCK1 | NM_001380.1 | 5202 | gtgatcctttcggaaacgataag | 297921 | - | signal_transduction、apoptosis | - | - |
| 19256 | LGN | NM_013296.3 | 1080 | ctgttggcccgacagcttaaaga | 297956 | - | see also 5389 line | | |
| 19257 | RAB3-GAP150 | NM_012414.2 | 1343 | gggtaccgcgacgcacaaattgg | 298038 | - | - | - | - |
| 19258 | RALBP1 | NM_006788.2 | 1323 | ctgcagggtgggataaaggattt | 298119 | - | signal_transduction | - | - |
| 19259 | RAP1GA1 | NM_002885.1 | 603 | gtgccggacataccatgatgtca | 298138 | - | signal_transduction | GTPase activator | - |
| 19260 | RAP1GDS1 | NM_021159.3 | 1502 | ctgctgtctgcccttatacgaca | 298194 | - | see also 8111 line | | |
| 19261 | RASA3 | NM_007368.2 | 1146 | gtgcggctcttcctacactatgg | 298217 | - | see also 5094 line | | |
| 19262 | SH3BP1 | NM_018957.2 | 563 | aggactcctatgccaactacttc | 298247 | - | - | GTPase activator | - |
| 19263 | SRGAP2 | NM_014850.1 | 2654 | tgggccgagtgcggttacgatct | 298277 | - | see also 6050 line | | |
| 19264 | STARD13 | NM_052851.1 | 2584 | ggggctagcgcacatgatcatgg | 298319 | - | - | - | - |
| 19265 | STARD8 | NM_014725.2 | 1408 | aggccacttcatcagtagaaata | 298333 | - | see also 5915 line | | |
| 19266 | TBC1D2 | NM_018421.1 | 1903 | gagccccatcagtaagtatgatg | 298346 | - | - | - | - |
| 19267 | TSC2 | NM_000548.2 | 582 | ctgttacctcgacgagtacatcg | 298360 | - | cell_cycle | - | leiomyoma、tuberous sclerosis、renal cell carcinoma、familial nervous system tumor syndromes |
| 19268 | BCL2L13 | NM_015367.1 | 669 | gagaacaactcctctaattctga | 298407 | - | apoptosis | protein translocase | - |
| 19269 | PSME1 | NM_006263.2 | 665 | cagccccatgtgggtgattatcg | 298422 | - | - | - | - |
| 19270 | PSME3 | NM_005789.2 | 335 | aagttattagaacttgatagttt | 298428 | - | - | - | - |
| 19271 | PPP2R4 | NM_021131.3 | 863 | gggcagttcgcagctgatagacc | 298457 | - | phosphatase | - | - |
| 19272 | PLAA | NM_004253.2 | 718 | tggtgcgagtgatggcattatta | 298485 | - | see also 2857 line | | |
| 19273 | GM2A | NM_000405.3 | 573 | ctgcccaagagcgaattcgttgt | 298552 | - | see also 322 line | | |
| 19274 | CLPS | NM_001832.2 | 147 | gtgcccagtgtaagagcaattgc | 298554 | - | - | enzyme activator | - |

Figure 46

| | | | | | | | | | |
|---|---|---|---|---|---|---|---|---|---|
| 19275 | PPP1R12B | NM_002481.2 | 2914 | aagacctctgaccgatcatcagt | 298586 | - | signal_transduction | - | - |
| 19276 | CABYR | NM_012189.2 | 583 | cagccacccctaagactactacc | 298598 | - | see also 5184 line | | |
| 19277 | SPA17 | NM_017425.2 | 326 | tagaagaccgcttctataacaat | 298651 | - | see also 7001 line | | |
| 19278 | IGBP1 | NM_001551.1 | 315 | cagcgggctcgagaacactttat | 298667 | - | phosphatase、 signal_transduction | protein phosphatase type 2A、 regulator | - |
| 19279 | PPP2R2B | NM_004576.2 | 1811 | tggcggctacaaataacctatat | 298713 | - | see also 3114 line | | |
| 19280 | PPP2R2C | NM_020416.2 | 602 | gtgactgcgagacctacatgtcg | 298732 | - | see also 7868 line | | |
| 19281 | PPP2R5A | NM_006243.2 | 890 | gagtatgtttcaactaatcgtgg | 298747 | - | phosphatase、 signal_transduction | protein phosphatase type 2A、 regulator | - |
| 19282 | PPP2R5B | NM_006244.2 | 1305 | ccggttcatctatgaattcgagc | 298783 | - | see also 4326 line | | |
| 19283 | PPP2R5D | NM_006245.2 | 630 | acgctgccaccttcatcgaatcc | 298805 | - | see also 4327 line | | |
| 19284 | PPP1R7 | NM_002712.1 | 73 | aggcgggtcgagtctgaagaatc | 298858 | - | phosphatase | - | - |
| 19285 | PPP4R1 | NM_005134.1 | 2071 | tggaaagttcgacgaactctagc | 298925 | - | phosphatase | - | - |
| 19286 | CAPON | NM_014697.1 | 555 | ccggatacggtatgagtttaaag | 298952 | - | - | - | - |
| 19287 | SLN | NM_003063.1 | 183 | ccgggagctgtttctcaacttca | 298966 | - | - | - | - |
| 19288 | DCPS | NM_014026.3 | 842 | cagcatccagtgggtgtataaca | 298973 | - | - | - | - |
| 19289 | DNAJA1 | NM_001539.1 | 1033 | gggtcgcctaatcatcgaattta | 298998 | - | - | heat shock protein | - |
| 19290 | DNAJB1 | NM_006145.1 | 878 | acgatacccgtcgtattcaaaga | 299014 | - | - | Heat shock factor protein 1 | - |
| 19291 | DNAJB2 | NM_006736.4 | 777 | ctgaagtcagtcacaatcaatgg | 299026 | - | - | heat shock protein | - |
| 19292 | DNAJB4 | NM_007034.3 | 607 | gggccatcccgcctcaaacaaga | 299041 | - | see also 4923 line | | |
| 19293 | DNAJB6 | NM_005494.2 | 193 | atgcctcacccgaggatattaaa | 299061 | - | - | - | - |
| 19294 | HSPB3 | NM_006308.1 | 606 | aggatccagttgggactaagtga | 299084 | - | - | heat shock protein | - |
| 19295 | HSPE1 | NM_002157.1 | 188 | tggatcgggttctaaaggaaagg | 299087 | - | - | heat shock protein | - |
| 19296 | TBCC | NM_003192.1 | 931 | tcgagagctctggtttagatagg | 299100 | - | see also 2158 line | | |
| 19297 | TBCD | NM_005993.2 | 279 | tggagcggttccgcgtaataatg | 299108 | - | - | structural constituent of cytoskeleton | - |
| 19298 | TBCE | NM_003193.2 | 906 | ctgcccaggttagaacaattaat | 299189 | - | - | - | Kenny-Caffey syndrome、 hypoparathyroidism syndrome |
| 19299 | C19orf2 | NM_003796.2 | 1285 | gtgtgtagcgacactagtgaaag | 299246 | - | transcription_regulator | - | - |
| 19300 | CABC1 | NM_020247.3 | 1647 | gggcaacgcgggaatatgacaga | 299257 | - | - | - | - |
| 19301 | CIA30 | NM_016013.1 | 1176 | atgagcttccgcttgataagatc | 299284 | - | see also 6614 line | | |
| 19302 | CKAP1 | NM_001281.1 | 691 | atgggaaacgctacttcgaatgc | 299297 | - | - | chaperone | - |
| 19303 | CLN3 | NM_000086.1 | 275 | ttgcaacaacttctcttatgtgg | 299299 | - | - | chaperone | Batten disease、 ceroid-lipofuscinosis、 juvenile amaurotic family idiocy、 melanoma |
| 19304 | DNAJA2 | NM_005880.2 | 998 | gggatgccgcagtatcgtaatcc | 299329 | - | see also 4088 line | | |

EP 1 752 536 A1

775

Figure 46

| | | | | | | | | |
|---|---|---|---|---|---|---|---|---|
| 19305 | DNAJA3 | NM_005147.3 | 772 | cggctccggcatggaaaccatca | apoptosis | - | - | - |
| 19306 | DNAJA4 | NM_018602.2 | 866 | aagattatcgaggtacatgttga | see also 7531 line | - | - | - |
| 19307 | DNAJB11 | NM_016306.3 | 744 | atgcctaatgtcaaactagtga | see also 6801 line | - | - | - |
| 19308 | DNAJB12 | NM_017626.1 | 338 | agggtcaagcaatgtaaagatta | - | - | chaperone | - |
| 19309 | DNAJB8 | NM_153330.1 | 338 | tcgttggcacccgacaagaaac | - | - | - | - |
| 19310 | DNAJB9 | NM_012328.1 | 832 | aggaaatatggttactacataca | - | - | chaperone | - |
| 19311 | DNAJC4 | NM_005528.1 | 1135 | gggcatggcctgcactacattg | - | - | heat shock protein | - |
| 19312 | DNAJC8 | NM_014280.1 | 265 | gagtttcggcagttatccatct | - | - | - | - |
| 19313 | HKE2 | NM_014260.2 | 295 | gggtccggtgctagtcaaacagg | - | - | chaperone | - |
| 19314 | JDP1 | NM_021800.1 | 494 | aagaatctgacaagactcatacc | - | - | - | - |
| 19315 | MGC26226 | NM_033105.3 | 516 | gagaagcatatacgacaagtacg | - | - | chaperone | - |
| 19316 | PFDN2 | NM_012394.2 | 192 | cagcctagtgatgatacactga | - | - | chaperone | - |
| 19317 | SCAP | NM_012235.1 | 1361 | tggagacgtcacgctgtacaagg | transcription_regulator | - | chaperone | - |
| 19318 | SCO1 | NM_004589.1 | 343 | tggaatgaagcacgtcaagaaag | - | - | chaperone | - |
| 19319 | SIL1 | NM_022464.3 | 641 | aagattgctgcctctttgatct | - | - | - | - |
| 19320 | WBSCR18 | NM_032317.2 | 166 | gcgcacggcgctgtatgatctgc | - | - | - | - |
| 19321 | APPBP2 | NM_006380.2 | 1924 | ttgcgagatcggcaatattcagt | see also 4473 line | - | - | - |
| 19322 | Dlc2 | NM_080677.1 | 343 | aggccatgagaagtacaatata | - | - | - | - |
| 19323 | DNAL4 | NM_005740.1 | 337 | gtgtcacagcctgtgagaaattc | - | - | microtubule motor | - |
| 19324 | DNCI2 | NM_001378.1 | 1042 | gagttactcgtgcttcctataa | see also 947 line | - | - | - |
| 19325 | FLJ32752 | NM_144666.1 | 2757 | tgggcaactgttggttcgttact | - | - | - | - |
| 19326 | FLJ40427 | NM_178504.3 | 342 | tagataacggagtgatcgttaac | - | - | - | - |
| 19327 | KLC2 | NM_022822.1 | 1761 | ttgaggcgcagcggttcctttgg | - | - | - | - |
| 19328 | KNS2 | NM_005552.3 | 1158 | gcggcgacttgaataaccttgc | - | - | - | - |
| 19329 | KNSL8 | NM_138343.1 | 840 | tggtgtatcgtgaccagaataag | - | - | - | - |
| 19330 | DCTN1 | NM_004082.2 | 2628 | ttgctcatgcctcgtccattg | - | - | - | lower motor neuron disease |
| 19331 | DCTN2 | NM_006400.3 | 850 | gagacagctgtacgttgtgatca | - | - | motor | - |
| 19332 | DNAI1 | NM_012144.2 | 554 | cagcattaccgcgatgaattagt | - | - | motor | - |
| 19333 | DNAI2 | NM_023036.1 | 882 | gggcatgagcagcgattcataca | - | - | - | - |
| 19334 | DNALI1 | NM_003462.3 | 156 | aggctcggctactgaaagtcagc | - | - | axonemal motor | - |
| 19335 | CIAO1 | NM_004804.2 | 450 | atgctaccacttgcatttggaag | transcription_regulator | - | - | - |
| 19336 | ID1 | NM_002165.2 | 299 | acgacatgaacggctgttactca | transcription_regulator | - | - | melanoma |
| 19337 | RBBP8 | NM_002894.1 | 1559 | aggactgagtacgttaaagattc | - | - | - | retinoblastoma |

Figure 46

| 19338 | SMARCB1 | NM_003073.2 | 243 | gggaaactacctccgtatgttcc | 300020 | - | see also 2060 line | | |
|---|---|---|---|---|---|---|---|---|---|
| 19339 | THRSP | NM_003251.1 | 415 | caggaaatgacgggacaagtttg | 300032 | - | transcription_regulator | - | - |
| 19340 | RPC32 | NM_006467.1 | 554 | aaggcacaccactcactaatact | 300042 | - | transcription_regulator | - | - |
| 19341 | RPC62 | NM_006468.4 | 412 | gggtattgcgaatgcttagatat | 300054 | - | transcription_regulator | - | - |
| 19342 | AES | NM_001130.5 | 517 | ccgctcccgagctgaactctatc | 300099 | - | signal_transduction、tr anscription_regulator | - | - |
| 19343 | ASXL1 | NM_015338.3 | 3855 | tcgctacgcatgggatctttaca | 300164 | - | transcription_regulator | - | - |
| 19344 | BCL3 | NM_005178.2 | 919 | acgtgaacgcgcaaatgtactcc | 300187 | - | cell_cycle、transcripti on_regulator | translation regulator | leukemia、chronic lymphocytic leukaemia |
| 19345 | BMI1 | NM_005180.5 | 689 | aagaccagaccactactgaatat | 300193 | - | transcription_regulator | - | non-hodgkin lymphoma |
| 19346 | CCNC | NM_005190.2 | 279 | ctgctacggtatatttcaagaga | 300220 | - | see also 3648 line | | |
| 19347 | CCNH | NM_001239.2 | 468 | ttgtgggtacggcttgtatgtat | 300242 | - | cell_cycle、transcripti on_regulator、kinase_r elated | cyclin-dependent protein kinase、 regulator | - |
| 19348 | CCNT1 | NM_001240.2 | 2095 | ttgccaagagtactaaatcctct | 300309 | - | see also 811 line | | |
| 19349 | CCNT2 | NM_001241.2 | 961 | ctgcgccagtacctctaaattca | 300351 | - | cell_cycle、transcripti on_regulator、kinase_r elated | - | - |
| 19350 | DAT1 | NM_018640.2 | 827 | cggactacgaggaaggtttaatg | 300386 | - | transcription_regulator | - | - |
| 19351 | DAXX | NM_001350.2 | 1090 | gcgacgtcacctcgatctcatct | 300393 | - | apoptosis、transcriptio n_regulator | - | - |
| 19352 | FLJ10201 | NM_018023.3 | 1664 | ctgccggctctgttattaataat | 300451 | - | transcription_regulator | - | - |
| 19353 | GTF2H2 | NM_001515.2 | 432 | tagactgacgtgtactttaaagt | 300501 | - | transcription_regulator | translation factor、 nucleic acid binding | - |
| 19354 | JAZF1 | NM_175061.2 | 693 | ctgcggcaccacacaatcaattt | 300511 | - | transcription_regulator | - | - |
| 19355 | JJAZ1 | NM_015355.1 | 1736 | tagtcgcaacggaccagttaaga | 300541 | - | see also 6386 line | | |
| 19356 | MADH9 | NM_005905.2 | 846 | tggtgctcggtcgcctactatga | 300560 | - | transcription_regulator | - | - |
| 19357 | MJD | NM_004993.2 | 358 | gaggctcaggatcgatcctataa | 300588 | - | see also 3484 line | | |
| 19358 | MLLT3 | NM_004529.1 | 1246 | acgttaccgccatttgatgatat | 300641 | - | see also 3082 line | | |

Figure 46

| | | | | | | | | | |
|---|---|---|---|---|---|---|---|---|---|
| 19359 | NCOA5 | NM_020967.1 | 704 | atgccgaaaggcccgttgattgt | 300683 | - | transcription_regulator | - | - |
| 19360 | PCQAP | NM_015889.2 | 701 | cagcatctaattaaattgcatca | 300708 | - | transcription_regulator | translation regulator | - |
| 19361 | RBL1 | NM_002895.2 | 2478 | gtgtacgcttacgtgatctatgt | 300785 | - | see also 1930 line | | |
| 19362 | SEI1 | NM_013376.1 | 643 | gggcctgtttgaggatattgaca | 300809 | - | transcription_regulator、cell_cycle、kinase_related | - | - |
| 19363 | SIN3A | NM_015477.1 | 2492 | aggtctaagagcttactcaatga | 300868 | - | see also 6425 line | | |
| 19364 | SNAPC1 | NM_003082.2 | 852 | aaggcatcgtcaagtcaaactcg | 300912 | - | see also 2075 line | | |
| 19365 | TLE1 | NM_005077.3 | 1170 | aagcgatgacaacttagttgtgg | 300918 | - | signal_transduction、transcription_regulator | - | - |
| 19366 | TLE2 | NM_003260.3 | 316 | atgtcgtacgggctcaacattga | 300934 | - | signal_transduction、transcription_regulator | - | - |
| 19367 | TRIP-Br2 | NM_014755.1 | 827 | aggagctctgtcccacatctacc | 300971 | - | see also 5946 line | | |
| 19368 | TRIP8 | NM_004241.1 | 6018 | tggacggactatgcctaacattc | 301140 | - | transcription_regulator | - | - |
| 19369 | HSF2BP | NM_007031.1 | 592 | ccgtgcaggccgacaacataaga | 301183 | - | transcription_regulator | - | - |
| 19370 | FLJ25348 | NM_144569.2 | 946 | tggtgggaccgcagatgttcatg | 301210 | - | transcription_regulator | - | - |
| 19371 | PWP1 | NM_007062.1 | 1500 | aagacgagagaggcttgttcttg | 301250 | - | transcription_regulator | signal transducer | - |
| 19372 | NASP | NM_002482.2 | 2030 | aggaactgctacccgaaattaga | 301281 | - | see also 1588 line | | |
| 19373 | XRCC4 | NM_003401.2 | 800 | ccgagatccagtctatgatgaga | 301299 | - | - | - | - |
| 19374 | ALKBH | NM_006020.1 | 509 | gtgaccgtaggctaccattataa | 301309 | - | - | - | synovial sarcoma |
| 19375 | FANCA | NM_000135.1 | 281 | gaggcctatgctaatcattctag | 301321 | - | see also 120 line | | |
| 19376 | FANCC | NM_000136.1 | 669 | tgggtatgcacctatagattact | 301401 | - | see also 121 line | | |
| 19377 | FLJ10858 | NM_018248.1 | 951 | aagctaccgactagaaatactat | 301448 | - | - | - | - |
| 19378 | HUS1 | NM_004507.2 | 933 | aagacgtgtcccttcagtatttc | 301508 | - | signal_transduction、cell_cycle | - | - |
| 19379 | NEIL2 | NM_145043.1 | 1007 | cagcgtttgggtgaacgatttct | 301518 | - | - | - | - |
| 19380 | POLK | NM_016218.1 | 1697 | atgggtgttcggatatctagttt | 301577 | - | - | - | - |
| 19381 | RAD9A | NM_004584.2 | 992 | atgacgacattgactcttacatg | 301624 | - | cell_cycle、signal_transduction | - | - |

EP 1 752 536 A1

Figure 46

| 19382 | RBBP4 | NM_005610.1 | 1097 | ctgatcgcagactgaatgtctgg | 301635 | - | cell_cycle、 transcription_regulator | - | - |
|---|---|---|---|---|---|---|---|---|---|
| 19383 | CDC45L | NM_003504.3 | 384 | cagtcaatgtcgtcaatgtatac | 301647 | - | see also 2361 line | | |
| 19384 | GMNN | NM_015895.2 | 562 | aggagtcatttgatcttatgatt | 301676 | - | cell_cycle | - | - |
| 19385 | SPHAR | NM_006542.1 | 387 | atgtctcatttatgatagatttg | 301689 | - | cell_cycle | - | - |
| 19386 | REC8L1 | NM_005132.1 | 656 | gtgatccgcgtctattctcaaca | 301692 | - | - | - | - |
| 19387 | CIDEA | NM_001279.2 | 394 | gagagtcaccttcgacttgtaca | 301708 | - | apoptosis、 signal_transduction | apoptosis regulator | - |
| 19388 | CDC40 | NM_015891.2 | 809 | gtgttaatctacggtcaactatg | 301742 | - | - | protein tyrosine kinase | - |
| 19389 | CRNKL1 | NM_016652.3 | 809 | aagggctcgatccatatacgagc | 301783 | - | - | - | - |
| 19390 | GEMIN4 | NM_015721.1 | 2294 | ctggctccaccgcaagttagaac | 301868 | - | - | - | - |
| 19391 | ARL6IP4 | NM_016638.1 | 621 | gaggaaatcgtaaccaaagaacg | 301876 | - | - | - | - |
| 19392 | LUC7A | NM_016424.2 | 1215 | aagccgggatcgaaagtcatata | 301910 | - | see also 6850 line | | |
| 19393 | FLJ21172 | NM_024805.1 | 664 | gtgccaccgatagtgtttgttca | 301924 | - | - | - | - |
| 19394 | RNU3IP2 | NM_004704.2 | 579 | tggacggaagctgcatgtgattc | 301938 | - | - | - | - |
| 19395 | HCA66 | NM_018428.1 | 1169 | gtgtttcgttgctgtgttataac | 301978 | - | - | - | - |
| 19396 | PRPF39 | NM_017922.2 | 1849 | atggttcgtttacgaagagtaag | 302027 | - | see also 7177 line | | |
| 19397 | GLE1L | NM_001499.1 | 1809 | ccgtctctacgctgctatcatcc | 302073 | - | - | - | - |
| 19398 | PHLDA2 | NM_003311.2 | 337 | tcgcgctggcgctcatcgatttc | 302080 | - | apoptosis | - | - |
| 19399 | CD37 | NM_001774.1 | 772 | tggctgcacaacaaccttatttc | 302083 | - | - | - | - |
| 19400 | GYPC | NM_002101.3 | 389 | tcgtcatgctgcgctacatgtac | 302087 | - | - | - | - |
| 19401 | TM4SF2 | NM_004615.2 | 587 | atgaacgaaactgattgtaatcc | 302100 | - | - | - | mental retardation |
| 19402 | TM4SF4 | NM_004617.2 | 533 | tgggagctggatactcgtttatc | 302112 | - | - | - | - |
| 19403 | TM4SF5 | NM_003963.2 | 57 | gtgtggggctctccctcattacc | 302127 | - | - | - | - |
| 19404 | DAD1 | NM_001344.1 | 146 | tggacgcgtacctgctgtatata | 302134 | - | - | apoptosis inhibitor | - |
| 19405 | SERP1 | NM_014445.2 | 435 | ctggttattggctctcttcattt | 302142 | - | - | - | - |
| 19406 | UBQLN3 | NM_017481.2 | 1097 | gggtattataaggctctatgact | 302160 | - | - | - | - |
| 19407 | APG12L | NM_004707.2 | 432 | ctgtgggagacactcctattatg | 302182 | - | apoptosis | protein degradation tagging | - |
| 19408 | MGC47816 | NM_173642.1 | 1136 | ttgcagagagcgtctataccatc | 302195 | - | see also 10102 line | | |
| 19409 | UBD | NM_006398.1 | 119 | aagaacatgtccggtctaagacc | 302207 | - | - | - | - |
| 19410 | CDC23 | NM_004661.2 | 966 | gagttatctggctcataacctct | 302250 | - | see also 3180 line | | |
| 19411 | PSMD14 | NM_005805.1 | 1106 | atgttggatactgtcgtatttaa | 302303 | - | see also 4051 line | | |
| 19412 | PSMD8 | NM_002812.3 | 369 | gggtcgactcaagctagttcttc | 302309 | - | see also 1830 line | | |
| 19413 | UK114 | NM_005836.1 | 171 | tagtcgacaggaccatttacatt | 302321 | - | - | - | - |

EP 1 752 536 A1

Figure 46

| | | | | | | | | | |
|---|---|---|---|---|---|---|---|---|---|
| 19414 | EEF1E1 | NM_004280.2 | 69 | gggactgagtaaggggaataaat | 302327 | - | see also 2868 line | | |
| 19415 | MRRF | NM_138777.2 | 486 | ctgacgggaagcttgctttaaac | 302338 | - | - | - | - |
| 19416 | PELO | NM_015946.4 | 2001 | atgcaggcaccgttaggatattc | 302357 | - | - | - | - |
| 19417 | TPR | NM_003292.1 | 6676 | gtggtgccacatcgtactgatgg | 302505 | - | see also 2227 line | | |
| 19418 | MGC14421 | NM_032907.2 | 564 | tcgctaggggggctatagtatttc | 302560 | - | - | - | - |
| 19419 | TRAM2 | NM_012288.1 | 478 | gtggtgacggaaggatacttaac | 302586 | - | see also 5256 line | | |
| 19420 | THEG | NM_016585.3 | 837 | gcggatcctccagttgtcaaagc | 302613 | - | - | - | - |
| 19421 | CD81 | NM_004356.2 | 545 | gtgctcaagaacaatttgtgtcc | 302616 | - | - | - | - |
| 19422 | LPXN | NM_004811.1 | 263 | cggcgcagctcgtgtatactacc | 302623 | - | see also 3344 line | | |
| 19423 | OCLN | NM_002538.2 | 1800 | aagaactctcccgtttggataaa | 302656 | - | - | - | - |
| 19424 | OXA1L | NM_005015.1 | 1 | atggtaacgtggctttacagatt | 302661 | - | signal_transduction | - | - |
| 19425 | PICALM | NM_007166.1 | 483 | aagtggattgcaaggatatgaca | 302704 | - | see also 4966 line | | |
| 19426 | TRAF1 | NM_005658.2 | 254 | gggaagacctccagtctataagc | 302751 | - | apoptosis | astacin | - |
| 19427 | VAMP3 | NM_004781.2 | 258 | gtgggcaatcgggattactgttc | 302762 | - | see also 3293 line | | |
| 19428 | VAMP4 | NM_003762.1 | 197 | aagcgccacctcaatgatgatga | 302766 | - | see also 2574 line | | |
| 19429 | VAMP8 | NM_003761.2 | 109 | aggtggagggagttaagaatatt | 302774 | - | - | - | - |
| 19430 | DKFZp434 O0515 | NM_178123.2 | 1483 | atggcttattagggatgttgtgc | 302812 | - | - | - | - |
| 19431 | FKBP8 | NM_012181.2 | 445 | aggcagcaggagcccatacatcc | 302832 | - | - | - | - |
| 19432 | KIAA0372 | NM_014639.1 | 1995 | ctggcttaggcgaggactatact | 302886 | - | - | - | - |
| 19433 | LOC57795 | NM_021165.1 | 323 | cagtgtggcactcggtttagagg | 302981 | - | - | - | - |
| 19434 | PPIL6 | NM_173672.1 | 793 | cagcaacgggtcacaattctata | 303024 | - | - | - | - |
| 19435 | STUB1 | NM_005861.1 | 919 | aggttattgacgcattcatctct | 303038 | - | - | - | Parkinson disease |
| 19436 | LECT1 | NM_007015.1 | 555 | gagttctaaggtgttagaactct | 303050 | - | - | - | - |
| 19437 | PTDSS2 | NM_030783.1 | 1262 | gcgtgagatctacgacttcatgg | 303092 | - | see also 8946 line | | |
| 19438 | ELOVL1 | NM_022821.1 | 868 | ctggtatcactcttataccaagg | 303109 | - | - | - | - |
| 19439 | ELOVL3 | NM_152310.1 | 617 | gtgctcgtgtacacaagctttgg | 303121 | - | - | - | - |
| 19440 | PRKAB1 | NM_006253.4 | 547 | gggcggaaaggaagtttacttat | 303132 | - | signal_transduction | hydrolase、 hydrolyzing O-glycosyl compounds | - |
| 19441 | C1orf17 | NM_015101.1 | 745 | gagtcttaccctgatgaaattgg | 303142 | - | see also 6241 line | | |
| 19442 | CEECAM1 | NM_016174.3 | 957 | atgtgactcggccctctaagagg | 303171 | - | - | - | - |
| 19443 | FLJ22329 | NM_024656.2 | 596 | gggctgcgtactccaacttctgg | 303178 | - | see also 8679 line | | |
| 19444 | COQ7 | NM_016138.3 | 195 | tcgggcagctgtggatcgaataa | 303193 | - | - | - | - |
| 19445 | COQ4 | NM_016035.1 | 621 | aggagcgtccccggatttcgaca | 303208 | - | - | - | - |
| 19446 | MATP | NM_016180.2 | 497 | atgataggtgtcgttctctttga | 303217 | - | - | - | - |

Figure 46

| | | | | | | | | | |
|---|---|---|---|---|---|---|---|---|---|
| 19447 | SILV | NM_006928.2 | 1333 | atgtctacggaaagtattacagg | 303251 | - | see also 4859 line | | |
| 19448 | FLJ31978 | NM_144669.1 | 416 | cagtgggtcgatccagtgtttac | 303266 | - | - | - | - |
| 19449 | MGC35274 | NM_153374.1 | 1121 | atgcaacttccctctatcacagt | 303282 | - | - | - | - |
| 19450 | NCOA7 | NM_181782.1 | 396 | gagatcaggcgtacagaactaaa | 303288 | - | see also 10253 line | | |
| 19451 | OSBP2 | NM_030758.1 | 527 | aggatcgctgccagcgttaaagc | 303360 | - | - | - | - |
| 19452 | OSBPL10 | NM_017784.3 | 1561 | gaggatcagcgtagtataattct | 303400 | - | see also 7152 line | | |
| 19453 | OSBPL11 | NM_022776.3 | 1952 | tggagaaggtatccttagtctgt | 303464 | - | see also 8466 line | | |
| 19454 | OSBPL2 | NM_014835.2 | 252 | aagtcacgggaatgattgactta | 303481 | - | see also 6043 line | | |
| 19455 | OSBPL3 | NM_015550.2 | 949 | aggaagcgtagcagtatatcaaa | 303512 | - | - | - | - |
| 19456 | OSBPL6 | NM_032523.2 | 904 | ctgcgacatcatcggttgtatcg | 303583 | - | see also 9237 line | | |
| 19457 | OSBPL7 | NM_017731.3 | 2707 | gtgggtgaccaacaatacctact | 303669 | - | - | - | - |
| 19458 | OSBPL8 | NM_020841.3 | 2544 | acgggtaactcgagccataaatg | 303741 | - | see also 7988 line | | |
| 19459 | ASAH2 | NM_019893.1 | 1652 | caggtctatgcaacgtctataca | 303868 | - | see also 7751 line | | |
| 19460 | SFTPB | NM_000542.2 | 649 | aagcggatccaagccatgattcc | 303878 | - | - | translation regulator | congenital alveolar proteinosis、pulmonary alveolar proteinosis |
| 19461 | BTN3A1 | NM_007048.3 | 1319 | gagacattcagcctataatgaat | 303879 | - | - | - | - |
| 19462 | CLU | NM_001831.1 | 161 | aagtaagtacgtcaataaggaaa | 303887 | - | apoptosis | - | atherosclerosis、Alzheimer disease |
| 19463 | CYB5-M | NM_030579.1 | 384 | aagttgctgggcatattggattt | 303908 | - | see also 8892 line | | |
| 19464 | CYBRD1 | NM_024843.2 | 196 | tgggagcgcactagagtttaact | 303912 | - | - | - | - |
| 19465 | FLJ20296 | NM_017750.2 | 332 | gggggctgctgtcatacctttgg | 303930 | - | - | - | - |
| 19466 | FLJ23153 | NM_024636.1 | 754 | ttgtgttataagagacgtaatct | 303963 | - | - | - | - |
| 19467 | FLJ39035 | NM_182580.1 | 267 | ctgaacactccctgttcttcttc | 303974 | - | - | - | - |
| 19468 | KIAA0601 | NM_015013.1 | 1891 | ccgctccacgagtcaaaccttta | 304018 | - | transcription_regulator | - | - |
| 19469 | MGC20446 | NM_153611.2 | 474 | tggtgtctggacggttctacttg | 304042 | - | - | - | - |
| 19470 | MGC25181 | NM_152783.2 | 559 | tggcagcgtccccgtctttgacg | 304051 | - | - | - | - |
| 19471 | PAOX | NM_152911.1 | 760 | gggctatcaaggactcacaaact | 304057 | - | - | - | - |
| 19472 | PCM1 | NM_006197.2 | 5799 | aggtcctacaacgtgactttaaa | 304251 | - | see also 4290 line | | |
| 19473 | SRPUL | NM_014467.1 | 1467 | ctgatccttccaaccgatattat | 304291 | - | see also 5698 line | | |
| 19474 | STEAP2 | NM_152999.2 | 526 | ttgaccattcgacttattagatg | 304302 | - | - | - | - |
| 19475 | TSAP6 | NM_018234.1 | 1393 | aggagagccgctacaagttctac | 304347 | - | - | - | - |
| 19476 | FLJ10986 | NM_018291.2 | 1394 | ttggggactcgcttcattataga | 304367 | - | - | - | - |
| 19477 | KIAA1838 | NM_032448.1 | 2123 | caggggaacgccctagtttgaaa | 304414 | - | - | - | - |
| 19478 | MGC40579 | NM_152776.1 | 804 | gtgggatgattacctctctaatt | 304446 | - | - | - | - |

Figure 46

| | | | | | | | | | |
|---|---|---|---|---|---|---|---|---|---|
| 19479 | KIAA0992 | NM_016081.2 | 2794 | ttgtactgacggctaatggtac | 304516 | - | | | deafness, diabetes mellitus, diabetes, Wolfram syndrome |
| 19480 | WFS1 | NM_006005.2 | 234 | caggcgcgttccgactcaatgc | 304537 | - | | | |
| 19481 | CDT6 | NM_021146.2 | 1018 | acgacgccctccagtatcataac | 304560 | - | | | |
| 19482 | MTL5 | NM_004923.2 | 1129 | ttgcaacaacttgcatcatgata | 304573 | - | apoptosis | heavy metal binding | |
| 19483 | PDLIM1 | NM_020992.2 | 846 | aagtggctgcgtcgaattggaaat | 304591 | - | | | |
| 19484 | CAMP | NM_004345.3 | 593 | aagaattgtccagagaatcaagg | 304602 | - | | antibacterial peptide | |
| 19485 | DEFA1 | NM_004084.2 | 304 | caggagaacgtgctatggaacc | 304606 | - | | antifungal peptide | |
| 19486 | DEFA5 | NM_021010.1 | 186 | atggactctgctcttagaacc | 304607 | - | | antifungal peptide | |
| 19487 | DEFA6 | NM_001926.2 | 302 | ctgcactgtcatgggtattaacc | 304614 | - | | antifungal peptide | |
| 19488 | DEFB1 | NM_005218.2 | 155 | tggccacagatctgatcattaca | 304617 | - | signal_transduction, g pcr | antimicrobial peptide | |
| 19489 | DEFB103 | NM_018661.2 | 221 | gaggatccattatcttcgtttg | 304618 | - | | Gram-positive antibacterial peptide | |
| 19490 | DEFB104 | NM_080389.1 | 202 | atgagagcttactgaatcgtaca | 304634 | - | | | |
| 19491 | DEFB106 | NM_152251.2 | 177 | cagccatgtggagcattataga | 304638 | - | | | |
| 19492 | DEFB118 | NM_054112.1 | 188 | ctgcattccatccaatgaagacc | 304642 | - | | | |
| 19493 | DEFB123 | NM_153324.2 | 213 | gagtctatgtttactgcataaat | 304655 | - | | | |
| 19494 | DEFB125 | NM_153325.1 | 34 | cgggtgaccaaaggtagctttga | 304657 | - | | | |
| 19495 | DEFB127 | NM_139074.2 | 47 | cagtaacgaacaacttaagaag | 304672 | - | | | |
| 19496 | DEFB129 | NM_080831.2 | 71 | tggcttgagacgctgtttaatgg | 304678 | - | | | |
| 19497 | GNLY | NM_006433.2 | 435 | tggcgcgacgtctgcagaaattt | 304690 | - | | | |
| 19498 | HTN1 | NM_002159.2 | 113 | atgattagcgctgattcacatga | 304693 | - | | antifungal peptide | |
| 19499 | HTN3 | NM_000200.1 | 191 | tcgaggctatagatcaaattatc | 304701 | - | | antifungal peptide | |
| 19500 | LEAP-2 | NM_052971.1 | 1537 | gggatgattctgagtgtatcaca | 304703 | - | | | |
| 19501 | C7orf10 | NM_024728.1 | 1089 | aggatcagcaaaggtattacaca | 304743 | - | see also 8717 line | | |
| 19502 | INSIG1 | NM_005542.3 | 932 | gggaacataggacgacgcagttagc | 304765 | - | | | |
| 19503 | PRNP | NM_000311.2 | 624 | ttgtgcacgactgcgtcaatatc | 304777 | - | | | Creutzfeldt-Jakob disease, Creutzfeld-Jakob disease, fatal familial insomnia, Gerstmann-Straussler-Scheinker syndrome, scrapie, schizophrenia, Lewy body dementia, Gerstmann-Straussler disease, familial fatal insomnia |
| 19504 | ATP6V0A4 | NM_020632.1 | 1257 | cagatgccacacgtatcaagagg | 304798 | - | | | |
| 19505 | ATP6V0D2 | NM_152565.1 | 532 | ctgatcgaaacgccattagctcc | 304834 | - | see also 9882 line | | |
| 19506 | BAT3 | NM_004639.2 | 3582 | cagcagctccggtcgtatataca | 304897 | - | see also 3153 line | | |

Figure 46

| | | | | | | | | |
|---|---|---|---|---|---|---|---|---|
| 19507 | BRDG1 | NM_012108.1 | 707 | tagaaactactccatcactattc | 304985 | - | - | |
| 19508 | STAM2 | NM_005843.3 | 1604 | tagcctaggaccgatcaaatg | 305031 | - | - | |
| 19509 | STN2 | NM_033104.2 | 1539 | gggaccgatcttcgtcaaactga | 305059 | - | see also 9358 line | |
| 19510 | SEC22L1 | NM_004892.3 | 503 | cagtcgtgtcgaagaaatctag | 305096 | - | see also 3394 line | |
| 19511 | SEC22L3 | NM_004206.2 | 217 | gaggagacgcgtcaagagtttag | 305104 | - | - | |
| 19512 | RAB9P40 | NM_005833.2 | 1012 | tggacgattgaaccattccatgt | 305133 | - | - | |
| 19513 | ELMO2 | NM_022086.6 | 1705 | ttgctagcctgagttactctga | 305158 | - | apoptosis | |
| 19514 | ELMO3 | NM_024712.2 | 1440 | aaggtgaatgcgctcacttatgg | 305178 | - | apoptosis | |
| 19515 | AMPH | NM_001635.2 | 304 | gagtcgctgcatgaagtctatga | 305186 | - | | breast cancer |
| 19516 | AP4E1 | NM_007347.2 | 3162 | ctggttatccttcgcaaatgatg | 305305 | - | - | |
| 19517 | BIN1 | NM_004305.2 | 942 | gtggaacagccgcgtaggtttct | 305310 | - | cell_cycle | breast cancer, neuroblastoma, prostate cancer, melanoma |
| 19518 | BIN2 | NM_016187.1 | 773 | cagcaggcgctctttagtcattt | 305327 | - | - | |
| 19519 | BIN3 | NM_018688.3 | 637 | ccgagctcaggttgttgtactact | 305343 | - | - | |
| 19520 | EPN1 | NM_013333.1 | 2432 | gagcgccaccacgtacatctct | 305348 | - | - | |
| 19521 | HOOK2 | NM_013312.1 | 2154 | cagcggctggcaaccaattctcg | 305361 | - | - | |
| 19522 | CPLX2 | NM_006650.2 | 535 | cagcagatccgagataagtatgg | 305367 | - | - | |
| 19523 | YKT6 | NM_006555.3 | 209 | ctgctcaaagccgcatacgatgt | 305369 | - | - | |
| 19524 | LRMP | NM_006152.1 | 855 | cagacggtactataacttcaagt | 305389 | - | - | |
| 19525 | CADPS | NM_003716.2 | 3892 | tggttgcaaagttggttactatc | 305489 | - | see also 2539 line | |
| 19526 | CPLX1 | NM_006651.3 | 391 | gagaacaagtacggcatcaagaag | 305503 | - | - | |
| 19527 | GOLGA4 | NM_002078.2 | 3923 | tacggattcagctagatatttgc | 305605 | - | see also 1344 line | |
| 19528 | GLTP | NM_016433.2 | 654 | ccgcctcttcctagtcaactaca | 305707 | - | see also 6858 line | |
| 19529 | FLJ23790 | NM_144963.1 | 1451 | gagagttcactcgtgtcaatatg | 305718 | - | - | |
| 19530 | LAPTM4A | NM_014713.2 | 251 | tggtacatggtagtaaacctatt | 305721 | - | - | |
| 19531 | NPTX1 | NM_002522.1 | 832 | ctgcggaccaactatatgtatgc | 305748 | - | see also 1619 line | |
| 19532 | NUP62 | NM_012346.3 | 1051 | ccggcacctccacaacaacatcc | 305763 | - | - | |
| 19533 | STX17 | NM_017919.1 | 280 | ccgatccaatatccgagaaattg | 305775 | - | see also 7174 line | |
| 19534 | STX6 | NM_005819.3 | 413 | atgcaactgaattgagtataaga | 305790 | - | - | |
| 19535 | STX8 | NM_004853.1 | 450 | gggtgccgaaccagatctaatca | 305815 | - | - | |
| 19536 | TRAPPC3 | NM_014408.3 | 593 | aggcggattgaggaccaatcctcc | 305834 | - | - | |
| 19537 | DLEU1 | NM_005887.1 | 268 | atgagacctgtatatggataca | 305838 | - | cell_cycle | |
| 19538 | PDAP2 | NM_007275.1 | 384 | gaggcgagtgcataagaatctga | 305850 | - | cell_cycle | |
| 19539 | SASH1 | NM_015278.2 | 3806 | gagcgtattctgataagcatgg | 305892 | - | see also 6310 line | |
| 19540 | TSC1 | NM_000368.2 | 3492 | aaggctcgagagttatttcgtaa | 305953 | - | see also 297 line | |
| 19541 | BTG4 | NM_017589.2 | 667 | aagtcagcaatcgaagagtatt | 305978 | - | cell_cycle | |
| 19542 | C10orf7 | NM_006023.1 | 1233 | acgctcacaagctaatagacttc | 306018 | - | see also 4202 line | |

Figure 46

| 19543 | CUL2 | NM_003591.2 | 2414 | aggcgtcggcagatgaatacagc | 306099 | - | see also 2398 line | | |
| 19544 | CUL3 | NM_003590.1 | 1837 | cgggtgctcacgacaggatattg | 306161 | - | see also 2383 line | | |
| 19545 | CUL4A | NM_003589.1 | 1950 | atgctgctatcgtcagaataatg | 306230 | - | cell_cycle、apoptosis | - | Alzheimer disease |
| 19546 | GAR17 | NM_139285.1 | 1815 | aggagcaagccatctactgtagc | 306243 | - | cell_cycle | - | - |
| 19547 | GAS2L1 | NM_006478.3 | 1149 | gagatgactcccgttagcttacg | 306283 | - | see also 4546 line | | |
| 19548 | LOC283431 | NM_174942.1 | 717 | ttggtcgaattgtgtcaagatac | 306302 | - | cell_cycle | - | - |
| 19549 | PPP1R15A | NM_014330.2 | 1460 | caggatcagccgaggatgaaaga | 306354 | - | apoptosis、cell_cycle | - | - |
| 19550 | REPRIMO | NM_019845.1 | 383 | acgagcgtagcctgtacataatg | 306359 | - | cell_cycle | - | - |
| 19551 | SESN1 | NM_014454.1 | 516 | gggccgttacccctacattatcg | 306377 | - | cell_cycle | - | - |
| 19552 | SESN2 | NM_031459.3 | 1466 | tagcctcacctacaataccatcg | 306423 | - | see also 9035 line | | |
| 19553 | SESN3 | NM_144665.2 | 1581 | tagttactggcggcagttcaaac | 306473 | - | cell_cycle | - | - |
| 19554 | BCCIP | NM_016567.2 | 423 | gggtacccagtgtgttgaacaaa | 306486 | - | cell_cycle、kinase_related | - | - |
| 19555 | CCNA1 | NM_003914.2 | 1049 | aagtagacgagtttgtctatatc | 306535 | - | cell_cycle、kinase_related | translation regulator | - |
| 19556 | CCNA2 | NM_001237.2 | 1222 | tggcctgaatcattaatacgaaa | 306588 | - | see also 809 line | | |
| 19557 | CCNE2 | NM_004702.2 | 738 | ctgtgtattctagccattgattc | 306600 | - | cell_cycle、kinase_related | - | - |
| 19558 | CCNG1 | NM_004060.3 | 595 | ttgatccgaataagtcaatatag | 306628 | - | see also 2736 line | | |
| 19559 | RGC32 | NM_014059.1 | 322 | gtgcagattcactttataggaac | 306655 | - | cell_cycle、kinase_related | - | - |
| 19560 | BUB3 | NM_004725.1 | 288 | gaggactagatcatcaattgaaa | 306663 | - | - | - | - |
| 19561 | MAD2L2 | NM_006341.2 | 718 | gtggaagagcgcgctcataaagg | 306694 | - | cell_cycle | - | - |
| 19562 | CHFR | NM_018223.1 | 1878 | aagctcaccacgccatgaaattc | 306715 | - | see also 7328 line | | |
| 19563 | MAD2L1 | NM_002358.2 | 130 | tcgtggccgagttcttctcattc | 306721 | - | cell_cycle | - | - |
| 19564 | GML | NM_002066.1 | 188 | ctgtgcggtcataaatgacttca | 306726 | - | apoptosis、cell_cycle、signal_transduction | GPI-anchored membrane-bound receptor | - |
| 19565 | CCNG2 | NM_004354.1 | 619 | ttgcacttataccatactattat | 306767 | - | see also 2913 line | | |
| 19566 | CDC16 | NM_003903.2 | 1402 | gaggtcggcgtggttgcatttca | 306829 | - | see also 2660 line | | |
| 19567 | CENPF | NM_016343.2 | 1126 | caggcgtcaaccaagtatactgc | 306865 | - | - | - | - |
| 19568 | ANAPC7 | NM_016238.1 | 1103 | tcgcaccttgtcgcttagattgt | 307046 | - | see also 6744 line | | |

Figure 46

| | | | | | | | | | |
|---|---|---|---|---|---|---|---|---|---|
| 19569 | BCL2 | NM_000633.1 | 72 | tagtgatgaagtacatccattat | 307067 | - | apoptosis、cell_cycle | - | osteochondroma、leukemia、chondrosarcoma、colorectal cancer、non-hodgkin lymphoma、lung cancer、neuroblastoma、ovarian cancer、colorectal cancers、squamous cell carcinomacytic、lymphoma、Hodgkin's disease |
| 19570 | BTG3 | NM_006806.3 | 866 | gtgaccggaatcactggattaat | 307092 | - | cell_cycle | - | - |
| 19571 | CCNB1 | NM_031966.2 | 866 | ttgatcggttcatgcagaataat | 307117 | - | cell_cycle | USF43/USF44 complex | - |
| 19572 | CCNB2 | NM_004701.2 | 543 | gtgactacgttaaggatatctat | 307151 | - | see also 3221 line | | |
| 19573 | CCNB3 | NM_033031.1 | 217 | gggggagaattgccaaacgaaga | 307171 | - | cell_cycle | - | - |
| 19574 | CCND1 | NM_053056.1 | 672 | atgaccccgcacgatttcattga | 307249 | - | - | USF43/USF44 complex | hepatocellular carcinoma、ependymoma、chondrosarcoma、laryngeal cancer、non-hodgkin lymphoma、liposarcoma、breast cancer、Li-Fraumeni syndrome、leukemia、osteosarcoma、parathyroid adenomatosis、Ewing's sarcoma、head and neck cancer、lymphoma、multiple myeloma、head and neck squamous cell carcinoma、squamous cell cancer |
| 19575 | CCND2 | NM_001759.2 | 1018 | ttgaggcggtgctcctcaatagc | 307260 | - | cell_cycle | cyclin-dependent protein kinase、regulator | nerve sheath tumors |
| 19576 | CCND3 | NM_001760.2 | 729 | ctgtgctacagattatacctttg | 307267 | - | cell_cycle | cyclin-dependent protein kinase、regulator | - |
| 19577 | CCNE1 | NM_001238.1 | 454 | acgtgcaagcctcggattattgc | 307274 | - | see also 810 line | | |
| 19578 | CCNF | NM_001761.1 | 2339 | aagcggataaacctatgcataca | 307330 | - | cell_cycle | cyclin-dependent protein kinase、regulator | - |
| 19579 | HARC | NM_017913.1 | 643 | ttgtctgaccatccataccttgt | 307337 | - | cell_cycle | - | - |
| 19580 | MPHOSPH6 | NM_005792.1 | 72 | ctgcgcatgaagtttatgcaaag | 307349 | - | cell_cycle | molecular_function unknown | |
| 19581 | MPHOSPH9 | NM_022782.2 | 975 | ctgactctagatcctacgttaca | 307372 | - | cell_cycle | - | - |
| 19582 | NEDD9 | NM_006403.2 | 173 | tggcaagggccttatatgacaat | 307443 | - | see also 4486 line | | |
| 19583 | NOL1 | NM_006170.1 | 1031 | ctggactagtggtgtatgattct | 307504 | - | cell_cycle | - | - |
| 19584 | NUDC | NM_006600.2 | 611 | cggggcagacctgcccaattacc | 307527 | - | see also 4651 line | | |

Figure 46

| ID | Gene | Accession | No. | Sequence | ID2 | | Notes | Disease |
|---|---|---|---|---|---|---|---|---|
| 19585 | SUGT1 | NM_006704.2 | 779 | aagaacctatatccatcatcatc | 307550 | - | see also 4730 line | |
| 19586 | TOB2 | NM_016272.2 | 639 | tggcttccgctgttcacattg | 307561 | - | cell_cycle | |
| 19587 | HEC | NM_006101.1 | 345 | aaggaacccgagaccacttaatga | 307569 | - | see also 4245 line | |
| 19588 | PRC1 | NM_003981.2 | 1654 | tagtagcattcggcctatcttg | 307654 | - | - | |
| 19589 | HCAP-G | NM_022346.2 | 2700 | ccgtgctgccagaaattcgagt | 307741 | - | cell_cycle | melanoma |
| 19590 | CDC27 | NM_001256.2 | 2014 | gagtcaatctagacattataat | 307827 | - | see also 812 line | |
| 19591 | INCENP | NM_020238.1 | 625 | cagggcatccgacatcagatga | 307860 | - | cell_cycle | |
| 19592 | PSEN1 | NM_000021.2 | 532 | tggtcgtggctaccattaagtca | 307985 | - | apoptosis | Alzheimer disease, disinhibition-dementia-Parkinsonism-amyotrophy complex |
| 19593 | PSEN2 | NM_000447.1 | 411 | atgagcggagtccctaatgtcg | 308019 | - | - | Alzheimer disease |
| 19594 | SC65 | NM_006455.2 | 334 | gggcgcagtacgaagtacagc | 308075 | - | see also 4525 line | |
| 19595 | TSGA2 | NM_080860.1 | 473 | atggcgtatactactacatcaat | 308091 | - | - | |
| 19596 | C10orf9 | NM_145012.3 | 403 | gtgtcgctttgcaatatattat | 308120 | - | see also 9764 line | |
| 19597 | CCNK | NM_003858.2 | 284 | gagggcgctcggttcatctttga | 308130 | - | see also 2622 line | |
| 19598 | CCNL2 | NM_030937.2 | 2121 | gagcgctcgaggtcgtatgaacg | 308159 | - | - | |
| 19599 | CEP2 | NM_007186.2 | 2865 | gaggccctagtacgagagaaagc | 308181 | - | kinase_related, cell_cycle | |
| 19600 | FLJ13265 | NM_024877.1 | 296 | ctggctggtgacacactttatct | 308203 | - | - | |
| 19601 | FLJ40137 | NM_173478.1 | 782 | ctgctcgacctggtctatcttct | 308220 | - | - | |
| 19602 | FLJ40432 | NM_152523.1 | 1105 | ttgtgtagagccgctatggagaag | 308241 | - | - | |
| 19603 | LOC91768 | NM_138375.1 | 93 | gggctgccatgccaagaatatatg | 308244 | - | see also 9604 line | |
| 19604 | PARD3 | NM_019619.2 | 3512 | aagatcgtcgcggaacctatagt | 308349 | - | see also 7724 line | |
| 19605 | PARD6A | NM_016948.1 | 852 | gggggcatccgggcgtttgacagg | 308357 | - | see also 6978 line | |
| 19606 | SPIN | NM_006717.1 | 514 | aagagtttctgcgctgaagtcc | 308360 | - | - | |
| 19607 | APEG1 | NM_005876.3 | 2821 | ttgcaaagcggtcaatgagtatg | 308370 | - | see also 4087 line | |
| 19608 | BTG1 | NM_001731.1 | 497 | gggatcgggttaccgttgtattc | 308376 | - | - | |
| 19609 | CD164 | NM_006016.3 | 296 | atgttagcgttgttaatactacc | 308386 | - | see also 4200 line | |
| 19610 | CGRRF1 | NM_006568.1 | 37 | gtgtttctgttaacgctttatga | 308398 | - | - | |
| 19611 | 1.Dec | NM_017418.1 | 582 | gggccttcttgccgtgttacaca | 308425 | - | - | |
| 19612 | DLEC1 | NM_005106.2 | 4290 | tggctacgccctgggttcatga | 308500 | - | | esophageal cancer, lung cancer, non-small-cell lung cancer |
| 19613 | DRIM | NM_014503.1 | 3417 | aggtcgtcagcacggtatcttaa | 308611 | - | | |
| 19614 | GPNMB | NM_002510.1 | 821 | cagaagaagatcgaaattcatc | 308772 | - | see also 1617 line | |
| 19615 | LDOC1 | NM_012317.2 | 307 | tcgtgcagacggcgtcttacatg | 308795 | - | | |
| 19616 | MDM4 | NM_002393.1 | 267 | gaggtcatgcactatttaggtca | 308804 | - | see also 1538 line | |
| 19617 | NCK2 | NM_003581.1 | 816 | ctgcgcacgcccaccagataagc | 308852 | - | receptor_acitivity | |

Figure 46

| | Symbol | Accession | No. | Sequence | ID | | Annotation | Note | Disease |
|---|---|---|---|---|---|---|---|---|---|
| 19618 | PMP22 | NM_000304.2 | 349 | aggaaatgtccaccactgttct | 308856 | - | | | Roussy-Levy syndrome, Charcot-Marie-Tooth neuropathy, hereditary motor and sensory neuropathy, neuropathy |
| 19619 | RARRES1 | NM_002888.1 | 360 | gcgctacaaccccagagtcttac | 308861 | - | | | - |
| 19620 | TP53I11 | NM_006034.2 | 604 | ctgagaaggtcatcattcgatgg | 308879 | - | | | - |
| 19621 | AMSH | NM_006463.2 | 380 | aagtattatcacgctctttattga | 308885 | - | | | - |
| 19622 | EDN1 | NM_001955.2 | 365 | ctgtctgtccctgatggataaag | 308897 | - | signal_transduction | Proto-oncogene C-JUN | melanoma |
| 19623 | FGA | NM_000508.2 | 1230 | tgggaacgcgaggcctaacaaac | 308924 | - | | | - |
| 19624 | FGB | NM_005141.1 | 793 | ctgtcaaaccgtatagagtatac | 308958 | - | | | - |
| 19625 | FGFR1OP | NM_007045.2 | 452 | gtgggtggaccctattattaga | 308979 | - | | | - |
| 19626 | FGG | NM_000509.3 | 428 | atgactcaagtattcgatattg | 309005 | - | | | afibrinogenemiadysfibrinogenemia-gamma |
| 19627 | SAS | NM_005981.2 | 566 | ctgtatcaacaagattatgaatt | 309039 | - | | | Well-differentiated liposarcoma, soft tissue sarcoma, liposarcoma, osteosarcoma, rhabdomyosarcoma, astrocytic tumors |
| 19628 | TBC1D8 | NM_007063.2 | 2653 | ttgtggagctgcctgatattatg | 309061 | - | see also 4943 line | | - |
| 19629 | BST2 | NM_004335.2 | 11 | tggcatctacttcgtatgactat | 309073 | - | kinase_related | | rheumatoid arthritis |
| 19630 | DAB2 | NM_001343.1 | 1123 | tcgtctactccgctgagtaatgg | 309095 | - | see also 887 line | | - |
| 19631 | EMP1 | NM_001423.1 | 246 | gtggtccacatcgctactgttat | 309118 | - | | | breast cancer |
| 19632 | EMP2 | NM_001424.2 | 447 | atgattgcggcctccatttatac | 309133 | - | | | - |
| 19633 | LAMP3 | NM_014398.2 | 1089 | cagtgaagtgggagcctatttga | 309156 | - | | | - |
| 19634 | LGI1 | NM_005097.1 | 1079 | atgcaagcctatagtcattgaaa | 309201 | - | see also 3574 line | | - |
| 19635 | PDCD1LG1 | NM_014143.2 | 411 | gtgccgactacaagcgaattact | 309249 | - | signal_transduction | | melanoma |
| 19636 | PRG4 | NM_005807.1 | 284 | gtgactgcgagcgccaagtaag | 309272 | - | see also 4055 line | | - |
| 19637 | RBBP7 | NM_002893.2 | 1165 | cggataagacgtagcttatgg | 309371 | - | | | - |
| 19638 | SIL | NM_003035.1 | 558 | aagaccatcgacttgcttatcg | 309386 | - | see also 2034 line | | - |
| 19639 | SPOCK | NM_004598.2 | 1426 | atgaggtcgggtacatatggtag | 309518 | - | | | - |
| 19640 | TM4SF8 | NM_005724.4 | 622 | ccgggctattgattagtcacga | 309521 | - | see also 3971 line | | - |
| 19641 | TM4SF9 | NM_005723.2 | 743 | cagagcatatcgggatgacattg | 309534 | - | see also 3970 line | | - |
| 19642 | TPD52L2 | NM_003288.2 | 632 | aggaccgagttggaccataaag | 309543 | - | | | - |
| 19643 | TSPAN-1 | NM_005727.2 | 740 | ttgtatgacatcgaactaatgc | 309552 | - | | | - |
| 19644 | TSPAN-2 | NM_005725.2 | 408 | gaggcttacaatgattaccttaa | 309562 | - | see also 3972 line | | - |
| 19645 | FLJ32871 | NM_144674.1 | 199 | gggtaccgctacctcaattcatg | 309572 | - | | | - |
| 19646 | EML1 | NM_004434.1 | 758 | gggtcgagactgccgtaacaacc | 309585 | - | | | - |

Figure 46

| | | | | | | molecular_function unknown | |
|---|---|---|---|---|---|---|---|
| 19647 | SPRY2 | NM_005842.2 | 1193 | cagccaagggttgccttaaatg | signal_transduction | - | 309636 |
| 19648 | TUBGCP2 | NM_006659.1 | 1325 | aggattacaacgacaagtactgg | see also 4704 line | - | 309658 |
| 19649 | TUBGCP3 | NM_006322.3 | 1563 | ctgtggcacgacaagtatactt | see also 4423 line | - | 309715 |
| 19650 | PLEK2 | NM_016445.1 | 1073 | gagcgagccgagtgattgaagc | see also 6860 line | - | 309773 |
| 19651 | CAP1 | NM_006367.2 | 307 | tggagcgagctctgttggttaca | signal_transduction, g pcr | - | 309778 |
| 19652 | CAP2 | NM_006366.1 | 1380 | gtgagcgccaagtcatcgaaat | signal_transduction, g pcr | - | 309819 |
| 19653 | KISS1 | NM_002256.2 | 469 | aggacctgccgaactacaactgg | breast cancer, melanoma | - | 309825 |
| 19654 | PEX11A | NM_003847.1 | 334 | tggtacctcgcttatgcttaaca | signal_transduction | - | 309831 |
| 19655 | PEX11B | NM_003846.1 | 301 | ttgtacttcgcctgtgacaatgt | see also 2614 line | - | 309853 |
| 19656 | PEX16 | NM_004813.1 | 650 | cagacttcaccccctatcgttcc | Zellweger syndrome | - | 309860 |
| 19657 | PEX3 | NM_003630.1 | 448 | ttgcgggtccagttaaacataat | see also 2437 line | - | 309878 |
| 19658 | PXF | NM_002857.1 | 81 | gtgctcttgatgatttcgataaa | see also 1890 line | - | 309909 |
| 19659 | PXMP3 | NM_000318.1 | 534 | cagaggtgaaagcgtgcttatgg | peroxisome biogenesis disorder, Zellweger syndrome | - | 309921 |
| 19660 | GOLGB1 | NM_004487.1 | 2206 | ttggagctcgagctaaacttca | rheumatoid arthritis | - | 309993 |
| 19661 | EBNA1BP2 | NM_006824.1 | 530 | acgaagcgacccactgattattt | - | - | 310174 |
| 19662 | RRS1 | NM_015169.2 | 452 | gggcatccgtcccaagaagaaga | - | - | 310185 |
| 19663 | TINF2 | NM_012461.1 | 1179 | aggaagaacatcgcgtatatacaca | - | - | 310201 |
| 19664 | PALM | NM_002579.1 | 570 | ccggtgggcacgcccaaagacaag | - | - | 310202 |
| 19665 | SOCS2 | NM_003877.3 | 806 | tagagatagctcgcattcagact | see also 2637 line | - | 310210 |
| 19666 | SOCS3 | NM_003955.2 | 589 | cagaagagcctattacatctact | protein kinase inhibitor | - | 310220 |
| 19667 | SOCS4 | NM_004232.2 | 1707 | ttgttatacgtcagtataccaga | see also 2824 line | - | 310263 |
| 19668 | SOCS7 | NM_080867.2 | 1648 | tggcatcgatgcccttccaattc | see also 9532 line | - | 310305 |
| 19669 | NDRG4 | NM_020465.1 | 473 | cagcatttcggttcaagtatgt | - | - | 310312 |
| 19670 | PLEKHC1 | NM_006832.1 | 1609 | gagcagatcaacgactgatataac | - | - | 310355 |
| 19671 | APG5L | NM_004849.1 | 364 | ttggacgaattccaacttgtttc | see also 3359 line | - | 310368 |
| 19672 | BCAS3 | NM_017679.2 | 1387 | atgtcaccacgagtagtgaatcg | see also 7109 line | - | 310423 |
| 19673 | BCAS4 | NM_017843.2 | 213 | ctgatcaggagtgatacttcaca | - | - | 310456 |
| 19674 | BCL9 | NM_004326.1 | 1814 | cgggagcgctcettacaaactct | signal_transduction | - | 310476 |
| 19675 | CDR1 | NM_004065.1 | 638 | tggatcttcaggaagaacatatat | - | - | 310531 |
| 19676 | CLN5 | NM_006493.1 | 1676 | tggctcacctatcccagttatgg | ceroid-lipofuscinosis | - | 310539 |
| 19677 | DACH | NM_004392.2 | 1756 | gagacgaaacgcgtgaacaagc | see also 2953 line | - | 310571 |
| 19678 | DACH2 | NM_053281.2 | 132 | tagtttcgtgttaataaccaca | see also 9490 line | - | 310585 |

Figure 46

| 19679 | FRAT1 | NM_005479.2 | 862 | ctgcagttacgtgcaaagcttcc | 310615 | - | signal_transduction | - | non-hodgkin lymphoma |
|---|---|---|---|---|---|---|---|---|---|
| 19680 | FSTL3 | NM_005860.1 | 190 | ctgtgcctccggcaacattgaca | 310618 | - | - | - | - |
| 19681 | GPC3 | NM_004484.2 | 1556 | gagccagtggtcagtcaaattat | 310655 | - | see also 3040 line | | |
| 19682 | HHCM | NM_006543.1 | 623 | gtgagcaccgtgacataatgagc | 310676 | - | - | - | - |
| 19683 | LMO2 | NM_005574.2 | 1268 | aagctctgccggagagactatct | 310693 | - | see also 3876 line | | |
| 19684 | OS-9 | NM_006812.1 | 617 | gggtccaagtgcgaccttaatgg | 310708 | - | - | - | - |
| 19685 | SEMA3C | NM_006379.2 | 1375 | tggactgcgtagccttgtcaaca | 310752 | - | see also 4472 line | | |
| 19686 | SS18 | NM_005637.1 | 85 | atgttggatgacaataaccatct | 310792 | - | - | - | synovial sarcoma |
| 19687 | TACC3 | NM_006342.1 | 301 | cagacgcacaggattctaagtcc | 310824 | - | - | - | - |
| 19688 | TCL1A | NM_021966.1 | 312 | cagtttctggcgcttagtgtacc | 310848 | - | - | - | leukemia、lymphoma |
| 19689 | CRADD | NM_003805.2 | 105 | gaggccagagacaaacaagtact | 310852 | - | signal_transduction、apoptosis | death receptor adaptor protein | - |
| 19690 | DIABLO | NM_019887.2 | 187 | ctgtgtgcggttcctattgcaca | 310866 | - | apoptosis | - | - |
| 19691 | FADD | NM_003824.2 | 590 | gtgcagcatttaacgtcatatgt | 310885 | - | apoptosis、signal_transduction、kinase_related | death receptor-associated factor | diabetes |
| 19692 | CIDEB | NM_014430.1 | 1209 | gtgacttactcaggtcagtatct | 310892 | - | apoptosis、signal_transduction | apoptosis regulator | - |
| 19693 | BAK1 | NM_001188.2 | 676 | ccgcttcgtggtcgacttcatgc | 310900 | - | apoptosis | apoptosis regulator | tongue squamous cell carcinomas |
| 19694 | BCL2L11 | NM_006538.1 | 361 | atggttatcttacgactgttacg | 310910 | - | apoptosis | - | - |
| 19695 | BIK | NM_001197.3 | 383 | tggacggtttcaccacacttaag | 310915 | - | apoptosis | apoptosis regulator | - |
| 19696 | BNIP3L | NM_004331.1 | 490 | aagtgcggactgggtatcagact | 310920 | - | apoptosis | - | - |
| 19697 | CIAS1 | NM_004895.3 | 901 | tggatctagccacgctaatgatc | 310926 | - | apoptosis、signal_transduction | - | - |
| 19698 | EI24 | NM_004879.2 | 734 | tggttagtctcctgcatatgtcc | 310988 | - | see also 3388 line | | |
| 19699 | TRADD | NM_003789.2 | 540 | aggatgcgctgcgaaatctgaag | 311002 | - | apoptosis、signal_transduction、kinase_related | - | - |
| 19700 | API5 | NM_006595.1 | 145 | gaggagctttaccgcaattatgg | 311013 | - | - | - | - |
| 19701 | BCL2A1 | NM_004049.2 | 232 | ctgcagtgcgtcctacagatacc | 311050 | - | apoptosis | apoptosis inhibitor | - |
| 19702 | BCL2L2 | NM_004050.2 | 217 | tggcagactttgtaggttataag | 311065 | - | apoptosis | molecular_function unknown | - |
| 19703 | BECN1 | NM_003766.2 | 1111 | gagataccgacttgttccttacg | 311087 | - | see also 2577 line | | |
| 19704 | BIRC2 | NM_001166.2 | 1675 | gaggactaacccctacagttatg | 311113 | - | see also 743 line | | |
| 19705 | BIRC3 | NM_001165.3 | 2870 | ctgtaccgaatgtctacgtattc | 311140 | - | apoptosis、signal_transduction | - | - |
| 19706 | BIRC8 | NM_033341.2 | 1916 | cagcgcggttattgatttcaagc | 311200 | - | apoptosis | apoptosis inhibitor | - |

789

EP 1 752 536 A1

Figure 46

| | | | | | | | | |
|---|---|---|---|---|---|---|---|---|
| 19707 | BNIP1 | NM_001205.1 | 27 | acgtccagtccggatcgtaac | - | apoptosis | - | |
| 19708 | FAIM2 | NM_012306.2 | 431 | cagaaagttcgtcgagtctttgt | - | - | - | |
| 19709 | GG2-1 | NM_014350.1 | 621 | aagtcacatggacgggttaataa | - | apoptosis | - | |
| 19710 | TAX1BP1 | NM_006024.4 | 259 | gagtactgtcgtgattattaca | - | - | - | |
| 19711 | TOSO | NM_005449.3 | 366 | ccgagttactctgaagcaatacc | - | apoptosis | - | |
| 19712 | MCL1 | NM_021960.3 | 567 | tggtcggggaatctggtaataac | - | apoptosis | apoptosis regulator | |
| 19713 | AXUD1 | NM_033027.2 | 628 | aagctccgccagcgcttgaaaga | - | see also 9349 line | - | |
| 19714 | BCL2L12 | NM_053842.2 | 748 | ctgggagcgtcacatgcaaattg | - | apoptosis | - | |
| 19715 | BCLG | NM_030766.1 | 540 | gggtcaaaggacgttgaataac | - | apoptosis | - | |
| 19716 | CARD6 | NM_032587.2 | 466 | ttgcgcttaaggcatgaagtt | - | apoptosis | apoptosis regulator | |
| 19717 | CARD8 | NM_014959.1 | 615 | cagtgacgattgcgtttggttcc | - | apoptosis | apoptosis regulator | |
| 19718 | CARD9 | NM_022352.2 | 444 | ccgcgtcttctccatgatcatcg | - | - | - | |
| 19719 | COP | NM_052889.1 | 272 | cagttccgatacctggaaattag | - | apoptosis | - | |
| 19720 | CTNNAL1 | NM_003798.1 | 516 | tggcagaacgagtagtcattaaa | - | see also 2597 line | - | |
| 19721 | MAEA | NM_005882.2 | 1180 | aagatccgtgagcaacgataaat | - | apoptosis | - | |
| 19722 | MGC13096 | NM_032346.1 | 657 | cagccttcgagggactatcagc | - | apoptosis | - | |
| 19723 | NCKAP1 | NM_013436.2 | 2510 | atgaccgtactccagtcaataga | - | see also 5432 line | - | |
| 19724 | PDCD6IP | NM_013374.2 | 1260 | aagccgatttggttaacagatca | - | signal_transduction, apoptosis | - | |
| 19725 | PPP1R13B | NM_015316.1 | 1295 | aagatccaaatccgctaatgatg | - | - | - | |
| 19726 | TAIP-2 | NM_024969.2 | 1667 | atgacccggagcaattcgttga | - | see also 8829 line | - | |
| 19727 | TEGT | NM_003217.1 | 623 | aaggccgaacatggagatcaaga | - | apoptosis | - | |
| 19728 | TRIAD3 | NM_019011.3 | 1248 | aagtaccgtacctcttattgaaga | - | - | - | |
| 19729 | C6 | NM_000065.1 | 1212 | ttgtacagccgaatattcgatga | - | - | complement activity | |
| 19730 | C7 | NM_000587.1 | 918 | tgggtcgttaggagggagaataca | - | - | complement activity | complement component 7 deficiency |
| 19731 | C8A | NM_000562.1 | 634 | gtgtgtacgatgccagttattat | - | see also 409 line | - | |
| 19732 | C8B | NM_000066.1 | 314 | atggggaaccgtgcaacttctct | - | - | complement activity | C8 beta deficiency, arthritis |
| 19733 | C9 | NM_001737.2 | 429 | aagatgcgacttcggtgtaatgg | - | - | hemolysin | complement component 9 deficiency |
| 19734 | PAQR10 | NM_198403.1 | 422 | tcgaccggatggtcatctatttc | - | see also 10321 line | - | |
| 19735 | CLUL1 | NM_014410.4 | 451 | aaggctatgccgggagtctttgg | - | - | - | |
| 19736 | OPTN | NM_021980.3 | 1784 | cagcggaatattccgattcattc | - | see also 8239 line | - | |
| 19737 | SMPX | NM_014332.1 | 374 | caggaacctgcagtcaatctatcg | - | - | - | |
| 19738 | TNNT2 | NM_000364.1 | 758 | gttggcagagcatctataacttgg | - | - | - | cardiomyopathy |
| 19739 | DYSF | NM_003494.2 | 5100 | gtgcttggtccgtatctacatg | - | see also 2358 line | - | |
| 19740 | EMD | NM_000117.1 | 60 | tggacaactacgcagatcttcg | - | - | - | muscular dystrophy |

Figure 46

| ID | Symbol | Accession | # | Sequence | ID2 | Function | Protein class | Disease |
|---|---|---|---|---|---|---|---|---|
| 19741 | FER1L3 | NM_013451.2 | 686 | cagatccgtccgagtgattga | 312307 | - | - | - |
| 19742 | SGCA | NM_000023.1 | 733 | gcgttgactggtgcaatgtgacc | 312451 | - | - | Duchenne-like muscular dystrophy, limb girdle muscular dystrophy |
| 19743 | SLMAP | NM_007159.1 | 481 | gaggttaacacatgatactgaga | 312464 | see also 4962 line | - | - |
| 19744 | SSPN | NM_005086.3 | 413 | aggttgacgaacggacatgtatt | 312491 | - | - | - |
| 19745 | FEZ1 | NM_005103.3 | 328 | tggaggacctcgtaaatgaattt | 312522 | see also 3578 line | - | - |
| 19746 | SEMA3B | NM_004636.1 | 561 | ttggtactgagtgcatgaacttc | 312536 | receptor_acitivity | - | non-small-cell lung cancer |
| 19747 | F11R | NM_016946.3 | 914 | cagcctagtgcccgaagtgaagg | 312560 | - | - | - |
| 19748 | NEO1 | NM_002499.1 | 2265 | ctgctcaacaatccaatggtaca | 312627 | see also 1595 line | - | - |
| 19749 | PECAM1 | NM_000442.2 | 913 | cagcccaccggaatgatcatgg | 312698 | signal_transduction | Nuclear factor NF-kappa-B p50 subunit | graft versus host disease |
| 19750 | SEMA3E | NM_012431.1 | 2132 | aggcgtttccgagacaagatgt | 312788 | see also 5320 line | - | - |
| 19751 | TM4SF6 | NM_003270.2 | 297 | ttgctactggtacgtcattatt | 312812 | see also 2218 line | - | - |
| 19752 | TPBG | NM_006670.3 | 1643 | atgcggacccagattaacgaac | 312854 | see also 4714 line | - | - |
| 19753 | GAGEB1 | NM_003785.2 | 140 | cgggcgtagaccaatgatctatgt | 312859 | - | - | - |
| 19754 | LY6H | NM_002347.1 | 340 | aggatcaactcggtgaacaagatg | 312862 | - | - | - |
| 19755 | PTX3 | NM_002852.2 | 163 | tggccgagaactcggatgattat | 312865 | - | - | - |
| 19756 | CD1B | NM_001764.1 | 324 | tggattcgtcgagaagtacaag | 312903 | - | - | - |
| 19757 | CD1C | NM_001765.1 | 330 | tggattaactcgggagattcaag | 312922 | - | - | - |
| 19758 | CD226 | NM_006566.1 | 900 | acgaaacttcgtgatgagatg | 312964 | receptor_acitivity, signal_transduction | - | - |
| 19759 | CD2BP2 | NM_006110.1 | 291 | tgggggtccagcaaatatgaca | 312974 | - | - | - |
| 19760 | CD53 | NM_000560.2 | 618 | agggttctatgcgaaagcaaga | 312994 | signal_transduction | - | - |
| 19761 | C1QA | NM_015991.1 | 473 | ctgtaccggctactactactc | 312999 | - | complement activity | systemic lupus erythematosis |
| 19762 | C1QB | NM_000491.2 | 393 | aggtgaatcgggagactacaagg | 313006 | - | complement activity | systemic lupus erythematosis |
| 19763 | C1QG | NM_172369.1 | 442 | aggtgaggaggcagagatacaagc | 313009 | - | defense/immunity protein | systemic lupus erythematosis |
| 19764 | C4BPA | NM_000715.2 | 1573 | tggtcggacaggcgaaactctcc | 313055 | - | - | - |
| 19765 | C4BPB | NM_000716.2 | 306 | atggttgcgtgcgagtttcgc | 313059 | - | - | - |
| 19766 | DAF | NM_000574.2 | 1304 | cagttcataccgtcttctatct | 313099 | - | - | paroxysmal nocturnal hemoglobinuria |
| 19767 | FHR5 | NM_030787.1 | 638 | ttggatcagactcagttcaatgt | 313101 | - | - | - |
| 19768 | HF1 | NM_000186.1 | 3671 | gtgtgtaaacggggatatcgtct | 313231 | - | complement activity | hemolytic-uremic syndrome |
| 19769 | PFC | NM_002621.1 | 1220 | ctgtatccgacggaacatgaagt | 313242 | - | complement activity | renal cell carcinoma, properdin P factor complement deficiency |
| 19770 | CD24 | NM_013230.1 | 192 | ttggcccaaatccaactaatgc | 313250 | - | - | - |
| 19771 | CD83 | NM_004233.2 | 646 | cggatgggcagagaaacctaagt | 313257 | signal_transduction | - | - |
| 19772 | HGRG8 | NM_016258.1 | 779 | ctgttggtagcgggtccattact | 313281 | - | - | - |

Figure 46

| 19773 | AIM2 | NM_004833.1 | 983 | tggtgaaaccccgaagatcaaca | 313325 | - | - | - | melanoma |
| 19774 | AZGP1 | NM_001185.2 | 374 | cagtaacgggtctcacgtattgc | 313344 | - | - | - | - |
| 19775 | C1QBP | NM_001212.3 | 298 | ctgcacaccgacggagacaaagc | 313351 | - | see also 794 line | | |
| 19776 | CD1A | NM_001763.1 | 830 | tagatacgcccatgaattgcagt | 313373 | - | - | - | - |
| 19777 | CD59 | NM_000611.3 | 346 | tggtgggacatccttatcagaga | 313389 | - | signal_transduction | - | - |
| 19778 | CD96 | NM_005816.3 | 673 | tggaacagcaaccatacgataga | 313398 | - | - | defense/immunity protein | - |
| 19779 | CEACAM8 | NM_001816.2 | 891 | atggcacattccagcaatacaca | 313436 | - | - | - | leukemia |
| 19780 | CNIH | NM_005776.1 | 291 | atgcccctcttggcatatcatat | 313442 | - | - | - | - |
| 19781 | CTLA4 | NM_005214.2 | 407 | ctgtgcggcaacctacatgatgg | 313449 | - | - | - | rheumatoid arthritis、diabetes |
| 19782 | G1P3 | NM_002038.2 | 416 | cagcagcgtcgtcataggtaata | 313457 | - | - | - | - |
| 19783 | ICOS | NM_012092.2 | 556 | acgaccctaacggtgaatacatg | 313481 | - | - | - | - |
| 19784 | KAAG1 | NM_181337.2 | 935 | gggatcgcctcctgaaacgaacg | 313485 | - | - | - | - |
| 19785 | NK4 | NM_004221.3 | 515 | gagacagtggcggcttattatga | 313491 | - | - | - | - |
| 19786 | RAET1E | NM_139165.1 | 750 | gggggcattcatcctgttagttt | 313510 | - | - | - | - |
| 19787 | SEMA7A | NM_003612.1 | 1599 | gtgctgcaatccattaatccagc | 313534 | - | see also 2426 line | | |
| 19788 | CAMLG | NM_001745.2 | 863 | ctgccgaagtgataaatcgatca | 313549 | - | signal_transduction | - | - |
| 19789 | CD84 | NM_003874.1 | 301 | atgccttaggtccgaactacaat | 313563 | - | see also 2636 line | | |
| 19790 | LY6E | NM_002346.1 | 255 | ctgctccgaccaggacaactact | 313573 | - | signal_transduction | - | - |
| 19791 | NOD27 | NM_032206.2 | 5070 | gagggtcctacacctaccattca | 313616 | - | - | - | - |
| 19792 | TFF2 | NM_005423.2 | 272 | aggtctcagaccgaagaaactgt | 313621 | - | - | - | - |
| 19793 | TFF3 | NM_003226.1 | 172 | tggagtgccttggtgtttcaagc | 313624 | - | - | - | - |
| 19794 | PSTPIP1 | NM_003978.2 | 745 | gagcaagctgtcgctctacaaga | 313626 | - | signal_transduction | - | pyogenic sterile arthritis |
| 19795 | LECT2 | NM_002302.1 | 254 | cagggccatgggctaatatatgt | 313636 | - | - | - | - |
| 19796 | DFNA5 | NM_004403.1 | 866 | atgctgcgcatgggatatcttcc | 313714 | - | - | - | deafness |
| 19797 | DSPP | NM_014208.1 | 1668 | tagcacatcagacactaataata | 313755 | - | - | extracellular matrix structural constituent | dentinogenesis imperfecta |
| 19798 | ITM2B | NM_021999.2 | 466 | acgtgtactactgtggaataaag | 313764 | - | - | structural molecule | familial british dementia |
| 19799 | KCNE1L | NM_012282.2 | 309 | gggcgacgacgcctatctctaca | 313779 | - | channel | - | - |
| 19800 | NOG | NM_005450.2 | 751 | gtggctgtggtcgcagacattct | 313783 | - | - | - | symphalangism |
| 19801 | OTOR | NM_020157.2 | 145 | atgatgagtgtgtctatactatt | 313788 | - | - | - | - |
| 19802 | TBL1X | NM_005647.2 | 2105 | gtgcgactgtgggacatagaacg | 313803 | - | signal_transduction、tr anscription_regulator | - | - |
| 19803 | TMC1 | NM_138691.2 | 1260 | tggtgtgttgtacgacttcaatg | 313815 | - | see also 9628 line | | |
| 19804 | USH3A | NM_052995.2 | 449 | gagcatccacgtcaatgtcattc | 313859 | - | see also 9474 line | | |

Figure 46

| 19805 | GCPHLP | NM_152401.1 | 699 | ttggagcaatacagactgatttg | 313890 | - | - | - | - |
|---|---|---|---|---|---|---|---|---|---|
| 19806 | UNC119 | NM_005148.2 | 740 | ccggctggtgatgcacaataaag | 313899 | - | - | - | - |
| 19807 | SNN | NM_003498.3 | 369 | gggaccgtgcgtggagagaaagg | 313901 | - | - | - | trimethyltin sensitivity |
| 19808 | ARR3 | NM_004312.1 | 538 | ctggttgtccggaaagtacaatt | 313906 | - | signal_transduction | - | - |
| 19809 | OPA3 | NM_025136.1 | 148 | ccgccgaagcgagttcttcaaga | 313918 | - | - | - | - |
| 19810 | PROM1 | NM_006017.1 | 1619 | ttggatacaccctacttactaaa | 313971 | - | - | - | retinal degeneration |
| 19811 | RS1 | NM_000330.1 | 525 | tggaaacaaccgggtcttctatg | 314020 | - | - | - | retinoschisis |
| 19812 | TULP1 | NM_003322.2 | 768 | aggaggcagctacggtgataaag | 314024 | - | see also 2254 line | | |
| 19813 | TULP2 | NM_003323.1 | 1460 | acggtgtctacacgctcaatttc | 314058 | - | - | - | - |
| 19814 | ARRB2 | NM_004313.3 | 643 | aaggcctgcggcgtagactttga | 314065 | - | see also 2886 line | | |
| 19815 | CLONE24945 | NM_015683.1 | 661 | ttgcccgatcctggtactgtaac | 314082 | - | - | - | - |
| 19816 | KIAA1376 | NM_020801.1 | 379 | aagtacgctggactgaatctaga | 314087 | - | see also 7975 line | | |
| 19817 | STIP1 | NM_006819.1 | 137 | gtgctactccgaagctattaagc | 314125 | - | see also 4787 line | | |
| 19818 | SYN2 | NM_003178.2 | 856 | ctggtcgctatctataagacact | 314154 | - | see also 2156 line | | |
| 19819 | CALCYON | NM_015722.2 | 382 | tgggctgcgtgctgatcatgtac | 314165 | - | signal_transduction | - | - |
| 19820 | CART | NM_004291.1 | 203 | aagaagctcaagagtaaacgtgt | 314167 | - | signal_transduction | - | - |
| 19821 | CBLN1 | NM_004352.1 | 545 | gagtaatcgcaccatgatcatct | 314168 | - | - | - | - |
| 19822 | HAP1 | NM_003949.2 | 320 | tggacccgcttcgtattccaagg | 314180 | - | - | - | - |
| 19823 | MOG | NM_002433.1 | 738 | caggatccggaatgtaaggttct | 314198 | - | see also 1567 line | | |
| 19824 | CNTNAP2 | NM_014141.3 | 647 | aggtcgcattggactcagaattg | 314220 | - | - | - | - |
| 19825 | F13B | NM_001994.1 | 1722 | agggaggcctattgtttagatgg | 314333 | - | - | translation regulator | Factor XIIIB deficiency(-) |
| 19826 | GP5 | NM_004488.1 | 1207 | ctgagccgcctgcggtacttagg | 314353 | - | - | - | rheumatoid arthritis |
| 19827 | MMRN | NM_007351.1 | 255 | tcggcggagatagctacaactcc | 314370 | - | - | - | - |
| 19828 | TRO | NM_016157.2 | 128 | cggatatagggtgcctctatttc | 314507 | - | - | - | - |
| 19829 | BYSL | NM_004053.2 | 632 | gggtccgggaggtattatctaag | 314549 | - | - | - | - |
| 19830 | CSN1S1 | NM_001890.1 | 358 | ttgtagactgaacgaatacaacc | 314569 | - | - | - | - |
| 19831 | NPHS1 | NM_004646.1 | 3567 | gggaccctctacgatgaagtgc | 314614 | - | - | - | - |
| 19832 | NPHS2 | NM_014625.1 | 837 | gggaatcaaagtggagagaatag | 314628 | - | see also 5796 line | | |
| 19833 | TG737 | NM_006531.2 | 369 | acggcagttactagacctatagc | 314639 | - | see also 4565 line | | |
| 19834 | IBSP | NM_004967.2 | 259 | caggccacgatattatctttaca | 314706 | - | - | - | - |
| 19835 | SFTPC | NM_003018.1 | 594 | tggcgaggtgccgctctactaca | 314721 | - | - | surfactant | - |
| 19836 | FMR2 | NM_002025.1 | 3953 | atgctaccgatgtttatcactcc | 314787 | - | see also 1314 line | | |
| 19837 | GLIPR1 | NM_006851.1 | 236 | ctgcgttcgaatccataacaagt | 314797 | - | - | - | glioma |
| 19838 | PSORS1C1 | NM_014068.1 | 666 | tggctccaactctattgtactcg | 314837 | - | - | - | - |
| 19839 | TTC3 | NM_003316.2 | 5060 | aggggagtatgtacgagttaaac | 314840 | - | - | - | - |
| 19840 | APBB1IP | NM_019043.2 | 1259 | aggagtcccagtatatcaagtat | 314868 | - | see also 7676 line | | |

EP 1 752 536 A1

Figure 46

| | | | | | | | | | |
|---|---|---|---|---|---|---|---|---|---|
| 19841 | C12orf2 | NM_007211.2 | 518 | tgggggcagtatgctagtgatgt | 314878 | - | signal_transduction | - | - |
| 19842 | FLJ10324 | NM_018059.2 | 531 | cagtgagaagcccctcttgatcc | 314897 | - | signal_transduction | - | - |
| 19843 | NMB | NM_021077.2 | 370 | aggaggctgctggtacaaatact | 314909 | - | signal_transduction | peptide hormone | - |
| 19844 | PKD1L2 | NM_052892.2 | 3507 | cagccagccgaccgtgttgaacc | 314934 | - | channel、signal_transduction | - | - |
| 19845 | RASSF1 | NM_007182.4 | 811 | aggccctgctgcgaaagttcttg | 314974 | - | cell_cycle、signal_transduction | - | lung cancer、Ewing's sarcoma |
| 19846 | RASSF2 | NM_014737.1 | 617 | atgcgcacacgcagtgatgttgg | 314988 | - | see also 5925 line | | |
| 19847 | RASSF3 | NM_178169.2 | 149 | aggacgccgaggacttcttcttc | 315002 | - | signal_transduction | - | - |
| 19848 | RASSF4 | NM_032023.3 | 620 | ccggcgacaccggttctctatca | 315018 | - | see also 9061 line | | |
| 19849 | RASSF5 | NM_031437.1 | 756 | cggcacctacacgggtttcatca | 315030 | - | see also 9028 line | | |
| 19850 | RASSF6 | NM_177532.2 | 439 | ttgacgatctctatcgtattagt | 315054 | - | signal_transduction | - | - |
| 19851 | HOMER2 | NM_004839.1 | 152 | acggagccaaggtgatcataaac | 315077 | - | - | - | - |
| 19852 | CD47 | NM_001777.2 | 928 | gcggcgtgtataccaatgcatgg | 315120 | - | signal_transduction | - | - |
| 19853 | SIRPB1 | NM_006065.1 | 930 | cagcttcgaccctcatagagaac | 315129 | - | signal_transduction | - | - |
| 19854 | PARK7 | NM_007262.3 | 136 | gagacggtcatccctgtagatgt | 315134 | - | - | - | Parkinson disease |
| 19855 | EDARADD | NM_080738.2 | 373 | ctgcccacgaaattcagatatga | 315150 | - | signal_transduction、kinase_related | | - |
| 19856 | AIP1 | NM_012301.2 | 4121 | acgggaacacgacgttaggaaac | 315222 | - | - | - | - |
| 19857 | ALP | NM_014476.1 | 258 | ctgatgcgcaggacaggattaaa | 315226 | - | - | - | - |
| 19858 | ARHGAP21 | NM_020824.1 | 1643 | aggttgtcggacaatacaagaaa | 315265 | - | - | - | - |
| 19859 | ASB10 | NM_080871.2 | 546 | gagggatttccgcttcaacatcc | 315382 | - | - | - | - |
| 19860 | ASB11 | NM_080873.1 | 538 | gagatcctgctggcaaataatgt | 315393 | - | - | - | - |
| 19861 | ASB13 | NM_024701.2 | 405 | gagcggggagttccgaatgtgtga | 315403 | - | - | - | - |
| 19862 | ASB14 | NM_130387.4 | 650 | ttgatcaagttcggatctgttca | 315428 | - | - | - | - |
| 19863 | ASB15 | NM_080928.2 | 603 | aagcgatggtcagcaatgcatga | 315455 | - | see also 9543 line | | |
| 19864 | ASB17 | NM_080868.1 | 263 | ctgttacgaaccaaggatttaca | 315460 | - | - | - | - |
| 19865 | ASB2 | NM_016150.3 | 2142 | ttgccgactgcgggttcgaaagg | 315511 | - | - | - | - |
| 19866 | ASB3 | NM_016115.3 | 866 | aggacagttggcagttacctatt | 315533 | - | see also 6671 line | | |
| 19867 | ASB4 | NM_016116.1 | 192 | ctggttgcctagctataaattga | 315572 | - | - | - | - |
| 19868 | ASB5 | NM_080874.2 | 421 | tggcgtgactccgttattcaacg | 315606 | - | - | - | - |
| 19869 | ASB6 | NM_017873.2 | 714 | acggggtgcagatccacaatact | 315628 | - | - | - | - |
| 19870 | ASB8 | NM_024095.2 | 120 | gagcgcttaatccgaacaattgc | 315639 | - | - | - | - |
| 19871 | ASB9 | NM_024087.1 | 655 | tgggggcaacattgaccataaga | 315663 | - | - | - | - |
| 19872 | C20orf163 | NM_080749.2 | 1031 | ctgtgccgcctagtgatacaaag | 315670 | - | - | - | - |
| 19873 | CTEN | NM_032865.3 | 959 | cagtgtctgatgtcagctatatg | 315677 | - | - | - | - |

Figure 46

| | | | | | | | | | |
|---|---|---|---|---|---|---|---|---|---|
| 19874 | DCDC2 | NM_016356.1 | 1066 | atgatcgccactctaagtcaaca | 315702 | - | see also 6840 line | | |
| 19875 | EFS | NM_005864.2 | 1737 | atggccgaggagtatgactatgt | 315711 | - | - | - | - |
| 19876 | ENIGMA | NM_005451.2 | 1227 | ttgcgagcgagactatgagaaga | 315719 | - | - | - | - |
| 19877 | ERBB2IP | NM_018695.1 | 2197 | aagtcgtagcacttagtaataac | 315781 | - | cell_cycle、 signal_tran sduction | | |
| 19878 | FLJ12428 | NM_022783.1 | 1304 | gggcccacggacgattgtcatgg | 315881 | - | see also 8477 line | | |
| 19879 | FLJ12987 | NM_025170.2 | 649 | gtgcacttgggacaagcattatt | 315903 | - | - | - | - |
| 19880 | FLJ14249 | NM_022460.2 | 120 | aggtacggggcaagatgatgtct | 315949 | - | - | - | - |
| 19881 | FLJ20967 | NM_022071.2 | 352 | ctgtcggagatgtacatagatcc | 315961 | - | - | - | - |
| 19882 | FLJ21687 | NM_024859.1 | 139 | ctgcctcagggcaaactatgtac | 315988 | - | - | - | - |
| 19883 | FLJ23209 | NM_024895.3 | 407 | gggacgcgtcatcctcatcaact | 315997 | - | - | - | - |
| 19884 | FLJ30934 | NM_152760.1 | 540 | gcgtcgagaccacaacttctttg | 316009 | - | - | - | - |
| 19885 | FLJ32798 | NM_173496.2 | 1430 | ccgtatcggcgacaaaactaatga | 316036 | - | - | - | - |
| 19886 | FLJ33505 | NM_152317.1 | 287 | atgctgtcgatgtggtcttaagt | 316046 | - | - | - | - |
| 19887 | GIPC2 | NM_017655.3 | 419 | tcgccgtcggagatcttatattg | 316071 | - | - | - | - |
| 19888 | GIPC3 | NM_133261.1 | 856 | caggagtttgcacgctgtttaga | 316100 | - | - | - | - |
| 19889 | GOPC | NM_020399.1 | 1237 | tggacatcgttaccgtttgtacc | 316127 | - | see also 7864 line | | |
| 19890 | GORASP2 | NM_015530.3 | 853 | cagttaatcccccgtctttgtca | 316154 | - | - | - | - |
| 19891 | HSH2 | NM_032855.2 | 1449 | caggagttggcaccaaatggtag | 316167 | - | - | - | - |
| 19892 | HSPC163 | NM_014184.1 | 316 | gtgtttgatccaacagaaataca | 316182 | - | - | - | - |
| 19893 | KIAA1365 | NM_020794.1 | 2700 | aggcgtaccatgggagtatcatg | 316361 | - | - | - | - |
| 19894 | KIAA1444 | NM_032512.1 | 713 | ggggagctgcgtgctcgtaaact | 316418 | - | - | - | - |
| 19895 | LMO7 | NM_005358.3 | 2132 | aagatgatatgtcgtatcgaagg | 316455 | - | - | - | - |
| 19896 | LNK | NM_005475.1 | 2007 | ccgggactcggactacgaaatgg | 316528 | - | - | - | - |
| 19897 | LNX | NM_032622.1 | 1566 | ttgagcccggaggagtcataagc | 316566 | - | see also 9261 line | | |
| 19898 | LOC118987 | NM_173791.2 | 1162 | aggccgtcttaaagttacgttgt | 316601 | - | - | - | - |
| 19899 | LOC51248 | NM_016484.3 | 182 | cagccggattccttatgatgact | 316685 | - | see also 6875 line | | |
| 19900 | MAGI1 | NM_173515.1 | 1949 | gcggattcctccgatcattgaag | 316724 | - | kinase_related | - | - |
| 19901 | MGC2793 | NM_145064.1 | 676 | cagtacgcttgtgtcaaagatct | 316741 | - | see also 9773 line | | |
| 19902 | MGC32065 | NM_153271.1 | 1220 | aagatcgcgtggacactttcaag | 316751 | - | - | - | - |
| 19903 | MGC39715 | NM_152628.2 | 942 | gggaggtacggcactatggatac | 316774 | - | - | - | - |
| 19904 | MGC5395 | NM_024060.1 | 323 | gagggacgacggcgtctttgtgc | 316793 | - | - | - | - |
| 19905 | MPP4 | NM_033066.1 | 1312 | tggctacggtcagaagttcttta | 316817 | - | - | - | - |
| 19906 | MPP5 | NM_022474.2 | 2151 | ctgtacggcaagtgatcaactct | 316872 | - | see also 8392 line | | |

Figure 46

| | | | | | | | |
|---|---|---|---|---|---|---|---|
| 19907 | NCK1 | NM_006153.3 | 534 | tggtggcgtggtagctacaatgg | 316906 | - | see also 4255 line |
| 19908 | NOXO1 | NM_144603.2 | 374 | ggcgcaggagttggacgaattca | 316933 | - | |
| 19909 | PDLIM2 | NM_021630.4 | 529 | ccggcctggagacataatcgtgg | 316936 | - | |
| 19910 | PDZK2 | NM_024791.2 | 295 | ctggccgtgaacaatgatgttgt | 316949 | - | signal_transduction |
| 19911 | PDZRN1 | NM_153371.2 | 1835 | aggagcagccgagtacttcagc | 316998 | - | |
| 19912 | PSCDBP | NM_004288.2 | 734 | ttgtggaccggaatcgattatcc | 317030 | - | see also 2873 line |
| 19913 | RACGAP1 | NM_013277.2 | 647 | aagactatcaaccattgatgaat | 317035 | - | |
| 19914 | RHPN1 | NM_052924.1 | 1408 | cagccgctcactgccaagtatgc | 317043 | - | signal_transduction |
| 19915 | RHPN2 | NM_033103.3 | 1885 | atgatctgcttagccattgatga | 317055 | - | signal_transduction |
| 19916 | RIMS2 | NM_014677.1 | 3256 | gtgttcgcttggcctctgatagc | 317136 | - | see also 5872 line |
| 19917 | RP1L1 | NM_178857.4 | 2323 | atggctcagtgccacgatattct | 317160 | - | |
| 19918 | SCRIB | NM_015356.1 | 269 | tggacgtgcccggaacgatatc | 317180 | - | |
| 19919 | SDCBP2 | NM_015685.3 | 436 | aaggcatcaggcgataagattgt | 317189 | - | |
| 19920 | SDP35 | NM_017779.2 | 751 | atggccaatacaagtaaacgtgg | 317207 | - | |
| 19921 | SH2B | NM_015503.1 | 3149 | aggtccatcgagtatatcatgg | 317230 | - | see also 6441 line |
| 19922 | SH2D2A | NM_003975.2 | 745 | cagtacagcccaatcatcaaaca | 317237 | - | SH3/SH2 adaptor protein |
| 19923 | SHANK1 | NM_016148.1 | 6281 | aagccgtttggcgctaaacctct | 317267 | - | |
| 19924 | SHD | NM_020209.1 | 523 | ctggaagccgacactgagtatt | 317270 | - | |
| 19925 | ShrmL | NM_020859.1 | 6170 | cagtgacatgttggttaactgc | 317313 | - | |
| 19926 | SLA2 | NM_032214.2 | 583 | aagtctcaggcagagagtataac | 317320 | - | signal_transduction, transcription_regulator |
| 19927 | SSB1 | NM_025106.2 | 470 | ccgatcgtcaatgctttgtga | 317327 | - | |
| 19928 | SSB3 | NM_080861.3 | 379 | cggagaggagacgagtatttcg | 317337 | - | |
| 19929 | STAC | NM_003149.1 | 533 | aggggtttcggcgttactacagc | 317352 | - | |
| 19930 | STMN4 | NM_030795.2 | 196 | ctgaataagtcgtcctacaaata | 317378 | - | |
| 19931 | SYNJ2BP | NM_018373.1 | 497 | atgagataccggcaacaacttg | 317395 | - | |
| 19932 | TENC1 | NM_015319.2 | 3952 | ctgcgcattccagccaaagatcc | 317417 | - | |
| 19933 | TJP2 | NM_004817.1 | 2391 | ctgagtggtccggttaaataacc | 317470 | - | see also 3347 line |
| 19934 | WSB2 | NM_018639.3 | 859 | ttgtcacggcttcttacgatacc | 317495 | - | |
| 19935 | XTP1 | NM_018369.1 | 115 | aagatgccgttacggaaacatcg | 317506 | - | see also 7423 line |
| 19936 | CHL1 | NM_006614.2 | 1723 | cagcggtatcatatctatgaaa | 317613 | - | see also 4676 line |
| 19937 | FLJ38991 | NM_173827.1 | 823 | cagacgtattaacgtactttgt | 317711 | - | signal_transduction |
| 19938 | HAN11 | NM_005828.1 | 666 | tggctcggtcggatgtttgacc | 317746 | - | signal_transduction |
| 19939 | INPP4A | NM_001566.1 | 2611 | aagtttggcgatacgtctttaca | 317787 | - | see also 1065 line |
| 19940 | LRDD | NM_018494.2 | 2658 | atgccgagaccggctttctgacg | 317816 | - | signal_transduction |

Figure 46

| No. | Gene | Accession | Number | Sequence | | ID | | Note | Function | Disease |
|---|---|---|---|---|---|---|---|---|---|---|
| 19941 | RSU1 | NM_012425.3 | 389 | cagtagccttcagaaactcaaac | - | 317823 | - | signal_transduction | | |
| 19942 | SH3GL2 | NM_003026.1 | 932 | ccgagctcgtacgactttgaac | - | 317845 | - | signal_transduction | | |
| 19943 | SH3GL3 | NM_003027.2 | 1345 | aagacgacaggttctaacattcc | - | 317864 | - | see also 2029 line | | |
| 19944 | UNC5CL | NM_173561.1 | 617 | ttgcactctcacgttcaaacact | - | 317877 | - | signal_transduction | | |
| 19945 | NCAM2 | NM_004540.2 | 2252 | ctgccaatagattgggatattct | - | 317961 | - | see also 3097 line | | |
| 19946 | NINJ2 | NM_016533.4 | 397 | gggaccatcctctactactaca | - | 317970 | - | - | | |
| 19947 | EVA1 | NM_005797.2 | 517 | aagaacccaacctgatgttgatcg | - | 317979 | - | - | | |
| 19948 | JAM2 | NM_021219.2 | 1053 | aaggatggcatccgtttgctaga | - | 317999 | - | - | | |
| 19949 | VCAM1 | NM_001078.2 | 2296 | aagggtcatatagtcttgtaga | - | 318071 | - | see also 672 line | | |
| 19950 | ZAN | NM_003386.1 | 1516 | gagttcgcataccacatgtatgg | - | 318089 | - | - | | |
| 19951 | CDA08 | NM_030790.2 | 1189 | gaggcgcgtcgaatgtttaaagt | - | 318167 | - | - | | |
| 19952 | COL17A1 | NM_000494.2 | 3762 | tggaggacctctgtcttactta | - | 318239 | - | - | | epidermolysis bullosa |
| 19953 | DKFZP761D0211 | NM_032039.1 | 424 | gtgccgctgttatctatgactt | | 318255 | | | | |
| 19954 | MDS028 | NM_018463.2 | 673 | aggttgtgcgtatgcaattctac | | 318276 | | - | | |
| 19955 | PPFIA2 | NM_003625.2 | 1557 | tcggatacggttgatagacttct | - | 318317 | - | see also 2432 line | | |
| 19956 | SGCE | NM_003919.1 | 484 | ttgagactgcaaggcataattg | - | 318377 | - | see also 2678 line | | |
| 19957 | HNT | NM_016522.2 | 1206 | gtgaagacgaatacttggaaatt | - | 318402 | - | - | | |
| 19958 | CASPR3 | NM_033655.1 | 768 | aagcagtatgccgagaagaaag | - | 318411 | - | - | cell adhesion molecule | |
| 19959 | ADRM1 | NM_007002.2 | 231 | cagacgacgactcgcttattca | - | 318414 | - | - | | |
| 19960 | C1orf38 | NM_004848.1 | 395 | ccggcacgtccacttatccaaac | - | 318427 | - | - | | |
| 19961 | CASPR4 | NM_033401.2 | 2051 | cagcacaacggctctgacttaac | - | 318480 | - | see also 9420 line | | |
| 19962 | caspr5 | NM_130773.2 | 3148 | atgcattcgtccttacacttga | - | 318615 | - | - | | |
| 19963 | CD34 | NM_001773.1 | 135 | gggcttgaccgcgcttgcttgc | - | 318648 | - | - | transcription factor | leukemia |
| 19964 | CD99 | NM_002414.3 | 239 | cggatggtggtttcgatttatcc | - | 318666 | - | - | | |
| 19965 | CDON | NM_016952.1 | 1229 | ccgtcattcgttggtatgacagc | - | 318709 | - | - | | |
| 19966 | CNTN2 | NM_005076.2 | 1941 | gggcactaccggagaactaatgt | - | 318774 | - | - | | |
| 19967 | DCBLD1 | NM_173674.1 | 601 | tggaaacgagctagccattattg | - | 318809 | - | see also 10108 line | | |
| 19968 | DSCAM | NM_001389.2 | 1066 | ctgcatcacgcggcatcgataca | - | 318865 | - | see also 955 line | | |
| 19969 | E48 | NM_003695.1 | 98 | cagcaactgcaagcattctgtgg | - | 318982 | - | - | | |
| 19970 | EFNB1 | NM_004429.3 | 1521 | cggttgccggttgcgtcatcttcc | | 318987 | | - | transmembrane ephrin | |
| 19971 | ESDN | NM_080927.2 | 1267 | gtgatcggggatcctcaaataac | - | 319014 | - | see also 9542 line | | |
| 19972 | IGSF1 | NM_001555.1 | 4069 | tggaactgcccgttccaatataa | - | 319145 | - | see also 1062 line | | |
| 19973 | L1CAM | NM_000425.2 | 3510 | gaggcccgaccgatgaaagatga | | 319169 | - | - | | Gareis-Mason syndrome, hydrocephalus, MASA syndrome, spastic paraplegia |

Figure 46

| | | | | | | | | | |
|---|---|---|---|---|---|---|---|---|---|
| 19974 | LSAMP | NM_002338.1 | 564 | cagggagcagtcaggcaaatatg | 319186 | - | see also 1513 line | | |
| 19975 | MCAM | NM_006500.1 | 968 | cagtgggcgctatgaatgtcagg | 319209 | - | - | - | lung squamous cell carcinoma、melanoma |
| 19976 | MFGE8 | NM_005928.1 | 554 | atggacacgaattcgatttcatc | 319221 | - | - | structural molecule | - |
| 19977 | NINJ1 | NM_004148.2 | 373 | ttgtcaagtacgaccttaacaac | 319231 | - | - | - | - |
| 19978 | NRXN1 | NM_004801.2 | 3908 | cagggcgtcagctcacaatcttc | 319329 | - | see also 3336 line | | |
| 19979 | NRXN2 | NM_015080.2 | 4053 | acgtgggcacggacgacattacc | 319368 | - | - | - | - |
| 19980 | OMD | NM_005014.1 | 645 | tggatgggctagtaaacttgacc | 319403 | - | see also 3510 line | | |
| 19981 | OMG | NM_002544.2 | 1648 | aagaagtctcgaggttctcaacc | 319439 | - | see also 1639 line | | |
| 19982 | PUNC | NM_004884.1 | 742 | cggacaatgagaggtacacattg | 319475 | - | - | - | - |
| 19983 | SRPX | NM_006307.2 | 551 | gggagcggaccgtcacatgtatg | 319498 | - | - | - | - |
| 19984 | TROAP | NM_005480.2 | 2247 | gagcgcctcaaatcgtgtttaac | 319533 | - | - | - | - |
| 19985 | DAP4 | NM_014902.2 | 2091 | atgcctagtggcgtataagaaga | 319543 | - | - | - | - |
| 19986 | EFNA5 | NM_001962.1 | 412 | cagaggggtgactaccatattga | 319556 | - | see also 1241 line | | |
| 19987 | EFNB2 | NM_004093.2 | 663 | ccggacattcggggaacaacatc | 319602 | - | see also 2762 line | | |
| 19988 | IFRD1 | NM_001550.1 | 1292 | ttggtcctgaacgcatgtatatt | 319624 | - | see also 1057 line | | |
| 19989 | SPAG6 | NM_012443.2 | 254 | cagaacgcgggtgtaatgtcttt | 319637 | - | - | - | - |
| 19990 | MLF1 | NM_022443.2 | 249 | gagggagagctcataatcgtaga | 319679 | - | - | - | leukemia、myelodysplastic syndrome |
| 19991 | CRISP1 | NM_001131.2 | 469 | atggacaacaacggatgatgaca | 319715 | - | - | - | - |
| 19992 | NP25 | NM_013259.1 | 364 | cagaaccaccgacatctttcaga | 319735 | - | - | - | - |
| 19993 | PNMA1 | NM_006029.3 | 1001 | gggtggaccgtgcaagatgttgc | 319746 | - | - | - | - |
| 19994 | CLN8 | NM_018941.2 | 840 | ctgccgcatggttctaacctacc | 319853 | - | - | - | ceroid-lipofuscinosis、epilepsy |
| 19995 | DAB1 | NM_021080.3 | 839 | tcggggagacaagttatgtcaag | 319867 | - | see also 8080 line | | |
| 19996 | FCMD | NM_006731.1 | 1024 | tggtatcgacaatgcaacattat | 319923 | - | see also 4750 line | | |
| 19997 | MAB21L2 | NM_006439.3 | 1674 | cggccagccgctcaacaactacc | 319945 | - | see also 4504 line | | |
| 19998 | MOBP | NM_006501.1 | 64 | ccgaacacttcagcatacactgc | 319947 | - | - | - | - |
| 19999 | NEURL | NM_004210.3 | 1227 | tggtggaccgcaaggaattctgg | 319952 | - | - | - | - |
| 20000 | NUMBL | NM_004756.2 | 324 | gcgggcaccatgaacaagttacg | 319953 | - | - | - | - |
| 20001 | SEMA3A | NM_006080.1 | 600 | ggggcgagactttgctatcttcc | 319992 | - | see also 4222 line | | |
| 20002 | SEMA6B | NM_020241.2 | 413 | gtgtcgaaacttcgtaaaggtgc | 320050 | - | receptor_acitivity | - | - |
| 20003 | SEMA6C | NM_030913.2 | 953 | ccgcgtagcccgagtatgtaaac | 320078 | - | - | - | - |
| 20004 | EVC | NM_014556.2 | 2545 | ccgcctggtgcaggcgtattacc | 320102 | - | - | - | Weyers acrodental dysostosis、Ellis-van Creveld syndrome |
| 20005 | CSRP3 | NM_003476.1 | 223 | gtgtgctatgggcgcagatatgg | 320113 | - | - | - | - |
| 20006 | VAMP5 | NM_006634.2 | 133 | cggaaattatgcgtaacaacttc | 320119 | - | - | - | - |
| 20007 | CAV3 | NM_001234.3 | 368 | tgggcggtggtgccatgcattaa | 320124 | - | - | - | muscular dystrophy、Rippling muscle disease、elevated serum creatine phosphokinase |
| 20008 | SGCB | NM_000232.2 | 333 | aagtggcctgcttcgatttaagc | 320139 | - | see also 171 line | | |

Figure 46

| | | | | | | | | | |
|---|---|---|---|---|---|---|---|---|---|
| 20009 | SGCD | NM_000337.3 | 693 | gtgctaactcagcttataacagg | 320168 | - | - | | limb girdle muscular dystrophy |
| 20010 | SGCG | NM_000231.1 | 566 | aagccactatttactgtagatga | 320197 | - | - | | Duchenne-like muscular dystrophy, limb girdle muscular dystrophy |
| 20011 | KRTHA2 | NM_002278.2 | 906 | acgtgggaggaatggttcaatatg | 320218 | - | - | | - |
| 20012 | SCEL | NM_003843.2 | 993 | aagtccgattaaagttaatcaaa | 320238 | - | - | | - |
| 20013 | TNFAIP2 | NM_006291.2 | 1911 | aagattgaggtggccacttatgc | 320270 | - | - | | - |
| 20014 | ANKRD7 | NM_019644.1 | 417 | tagttgccgttattaacaataat | 320279 | - | - | | - |
| 20015 | MEA | NM_014623.1 | 501 | ctgcctgggctcgggagatatct | 320289 | - | - | | - |
| 20016 | CCNI | NM_006835.2 | 1549 | tggaatcaaacggtctataatg | 320309 | - | - | cyclin-dependent protein kinase, regulator | - |
| 20017 | FSHPRH1 | NM_006733.1 | 1137 | ttgatgttaggtccagctaatgt | 320335 | - | see also 4753 line | | - |
| 20018 | SPAG9 | NM_003971.3 | 1993 | aagtacaaacaggtaaccaatgg | 320424 | - | see also 2714 line | | - |
| 20019 | D8S2298E | NM_005671.1 | 87 | tgggatccggatataggaatca | 320482 | - | see also 3942 line | | - |
| 20020 | SPAG1 | NM_003114.3 | 1029 | ctgcgtcgtgctactacatataa | 320521 | - | - | | - |
| 20021 | VCY2 | NM_004678.1 | 528 | gtgggcttgctgacatattatgt | 320563 | - | - | | - |
| 20022 | XKRY | NM_004677.1 | 1101 | gaggtgggacatacaatcctaca | 320574 | - | - | | - |
| 20023 | RQCD1 | NM_005444.1 | 168 | gttggcattcatttgtactattg | 320581 | - | see also 3792 line | | - |
| 20024 | FLJ10292 | NM_018048.2 | 140 | ccggacggaaagcttagatatgc | 320601 | - | - | | - |
| 20025 | SEMG1 | NM_003007.2 | 1385 | acgaccgaaaccattatttaca | 320614 | - | - | | - |
| 20026 | ACRV1 | NM_001612.4 | 1008 | tggaacgaggatgcaaattatat | 320630 | - | see also 1069 line | | - |
| 20027 | AMN | NM_030943.1 | 433 | ccgccacgacgacgtcttcttc | 320638 | - | - | | - |
| 20028 | CLPTM1 | NM_001294.1 | 1408 | gagtcctgaccaaagtgtatga | 320652 | - | - | | - |
| 20029 | DKK2 | NM_014421.1 | 1345 | caggggaagtcgttaccaaaca | 320668 | - | signal_transduction | | - |
| 20030 | DKK3 | NM_013253.3 | 1128 | cagagaggtcccgatgagtatg | 320681 | - | - | | - |
| 20031 | GTL3 | NM_013242.1 | 679 | gcgagcatacggcaccaattaca | 320691 | - | see also 5357 line | | - |
| 20032 | LOC56920 | NM_020163.1 | 1775 | acgcgcacggagcacaatagcacc | 320714 | - | receptor_activity | | - |
| 20033 | NRLN1 | NM_145234.2 | 1237 | gaggtccacgtttggactattcg | 320742 | - | - | | - |
| 20034 | NUMB | NM_003744.3 | 1552 | gaggccgaccgatggttagaaga | 320769 | - | see also 2564 line | | - |
| 20035 | SEMA3F | NM_004186.2 | 289 | cgggtccggtctcattcaaaga | 320785 | - | receptor_acitivity | receptor | - |
| 20036 | SEMA6D | NM_020858.1 | 1013 | ttggccagcgatgccgttattta | 320830 | - | see also 8000 line | | - |
| 20037 | TCP11 | NM_018679.2 | 959 | aagcagccttagtctcttaatca | 320920 | - | - | transmembrane receptor | AAAD deficiency |
| 20038 | UPK2 | NM_006760.1 | 104 | aggggctcagagcttcaacatct | 320931 | - | - | | - |
| 20039 | WHSC2 | NM_005663.2 | 1393 | atgttcaagacggccaacaagt | 320945 | - | transcription_regulator | | Wolf-Hirschhorn syndrome |
| 20040 | DOCK9 | NM_015296.1 | 979 | tggttccggtttagatagctacc | 320976 | - | - | translation regulator | - |

Figure 46

| | | | | | | | | |
|---|---|---|---|---|---|---|---|---|
| 20041 | MGC10471 | NM_030818.2 | 406 | cggcatgggtgttcgttacaatg | 321129 | - | | |
| 20042 | NDP52 | NM_005831.3 | 266 | tggaagacaaccgtgagtatta | 321141 | - | | |
| 20043 | ALS2CR12 | NM_139163.1 | 1305 | ctgggagattggcctcattact | 321198 | - | | atherosclerosis |
| 20044 | ANGPTL3 | NM_014495.1 | 857 | cagttagtccatggacattaatt | 321220 | - | signal transduction | |
| 20045 | ANGPTL4 | NM_016109.2 | 534 | cagcaggatccagcaactcttcc | 321241 | - | apoptosis | |
| 20046 | BICD2 | NM_015250.1 | 2314 | aggtgtgacggtacattacaca | 321258 | - | see also 6291 line | |
| 20047 | C14orf102 | NM_017970.2 | 1385 | aaggacggcagcatcttatctca | 321283 | - | see also 7185 line | |
| 20048 | C14orf44 | NM_152445.1 | 146 | gaggggagccgtcagatatttcc | 321320 | - | see also 9868 line | |
| 20049 | C20orf11 | NM_017896.2 | 691 | gtgtggagtgaagttaaccaagc | 321351 | - | | |
| 20050 | C20orf96 | NM_153269.1 | 1122 | gggaacccgagaggtcatattt | 321366 | - | | |
| 20051 | C8orf13 | NM_053279.1 | 868 | cagagcatgatgaagctatagc | 321369 | - | | |
| 20052 | DACT1 | NM_016651.4 | 2050 | aggcggtggccaaacctaag | 321400 | - | | |
| 20053 | FLJ20366 | NM_017786.1 | 1937 | ggggaacggatcctgttataac | 321433 | - | | |
| 20054 | GCC2 | NM_014635.3 | 3224 | ctggctcgtattgaagacattaca | 321520 | - | | |
| 20055 | GOLGA1 | NM_002077.1 | 2364 | tggcgccttccgtcacgaataac | 321587 | - | see also 1341 line | |
| 20056 | GOLGA2 | NM_004486.3 | 1678 | ttgtaaagctgactaatgagaac | 321611 | - | | |
| 20057 | GOLGA5 | NM_005113.2 | 916 | tggcctcgttactccaaagatcc | 321640 | - | see also 3587 line | |
| 20058 | GOLGIN-67 | NM_015003.3 | 1064 | aggacacgtgacacagttgaagg | 321697 | - | | |
| 20059 | K6IRS2 | NM_080747.1 | 1456 | ttggcgcctcaagcagttatagc | 321701 | - | | |
| 20060 | KIAA0373 | NM_014684.1 | 2162 | cagtctctacgacgacagtttag | 321737 | - | | |
| 20061 | KRT23 | NM_015515.3 | 1569 | aggacatgcaagagatcatctcc | 321845 | - | | |
| 20062 | KRT24 | NM_019016.1 | 976 | gggaccgacctgaccaaattact | 321859 | - | | |
| 20063 | KRT6IRS | NM_033448.1 | 1536 | gggcagtgccaacgattaccaaag | 321877 | - | | |
| 20064 | KRTHB1 | NM_002281.2 | 51 | atgactgccggatcaggatttgg | 321880 | - | | monilethrix |
| 20065 | MCRS1 | NM_006337.3 | 1412 | gggtcgcacggccatctcacatcg | 321885 | - | | |
| 20066 | MGC33295 | NM_152505.2 | 1209 | gagactcggaagacttagcagc | 321908 | - | | |
| 20067 | MLPH | NM_024101.4 | 1047 | ctgcactcgggtcgaatgtcatc | 321928 | - | | |
| 20068 | OLFM2 | NM_058164.1 | 883 | gagcaacgtggtgttcaaataac | 321945 | - | receptor_acitivity | latrotoxin receptor |
| 20069 | PCNT2 | NM_006031.3 | 10075 | ctgccaccgatgattaaacagt | 322044 | - | | |
| 20070 | PES1 | NM_014303.2 | 1713 | ctgtaccagaagatcatgtttgg | 322061 | - | | |
| 20071 | PPP1R16A | NM_032902.4 | 751 | tggaggctggggccaacatcaat | 322063 | - | | |
| 20072 | ProSAPiP1 | NM_014731.2 | 1891 | aagcctgtctacccaagaattt | 322066 | - | | |
| 20073 | PSTPIP2 | NM_024430.2 | 505 | cagaaatgccgggacaaagatga | 322077 | - | | |
| 20074 | SH3KBP1 | NM_031892.1 | 1688 | ttgactccgtgtatcatctact | 322122 | - | see also 9046 line | |

Figure 46

| | Gene | Accession | Length | Sequence | Code | | Notes | Disease |
|---|---|---|---|---|---|---|---|---|
| 20075 | SPAG4 | NM_003116.1 | 539 | aggggctgagcgtgttgttatcc | 322134 | - | - | |
| 20076 | SPAG4L | NM_080675.3 | 241 | ctgttgcctgtccgcaacaatga | 322139 | - | - | breast cancer |
| 20077 | TACC1 | NM_006283.1 | 1689 | aaggcaaagtcgcgttaataac | 322177 | - | see also 4375 line | |
| 20078 | TACC2 | NM_006997.1 | 1549 | tggacgggatgggctaaacaag | 322225 | - | see also 4883 line | |
| 20079 | TCP10 | NM_004610.1 | 1195 | atgcctccggagggttaagatc | 322250 | - | - | |
| 20080 | TCP10L | NM_144659.1 | 312 | ccggagtcatatagatgctttga | 322252 | - | - | |
| 20081 | TPD52 | NM_005079.1 | 575 | ttgaattcggctcaaatgctag | 322262 | - | - | breast cancer |
| 20082 | UVRAG | NM_003369.2 | 1345 | ggggtctagatcaacaatcaaag | 322301 | - | see also 2273 line | |
| 20083 | VAMP1 | NM_014231.3 | 460 | gtgccatcgtggtagttatt | 322316 | - | - | |
| 20084 | VAMP2 | NM_014232.1 | 380 | atgatcatcttgggagtgattg | 322321 | - | - | |
| 20085 | VEZATIN | NM_017599.2 | 296 | tgattccgactcgactcattac | 322328 | - | - | |
| 20086 | ZCWCC1 | NM_014941.1 | 3915 | cagatcctccgaattgtttacg | 322422 | - | - | |
| 20087 | DCTN4 | NM_016221.2 | 521 | tggcacgacgtagaaactatatg | 322449 | - | see also 6725 line | |
| 20088 | EPLIN | NM_016357.1 | 518 | ggggtcgatatcccacatcaagg | 322488 | - | see also 6841 line | |
| 20089 | KRTAP11-1 | NM_175858.2 | 90 | ttgttccagtggcccagttacc | 322535 | - | - | |
| 20090 | KRTAP13-1 | NM_181599.1 | 560 | gttggatcaggcttctactattga | 322539 | - | - | |
| 20091 | KRTAP8-1 | NM_175857.2 | 88 | atggctaccgctgggatatagc | 322542 | - | - | |
| 20092 | MGC45562 | NM_152349.1 | 508 | aagcctaccacaagtagagttgg | 322547 | - | - | |
| 20093 | C14orf31 | NM_152330.2 | 497 | cagcaagggtatcgaccaatttg | 322566 | - | - | |
| 20094 | C20orf42 | NM_017671.2 | 649 | gcgccttgtctgccgaatttga | 322618 | - | see also 7090 line | |
| 20095 | CDC42EP4 | NM_012121.3 | 417 | tggacgaacagccctcttcttca | 322670 | - | - | |
| 20096 | DOCK2 | NM_004946.1 | 2278 | ttgttcggtcgaggacattattt | 322714 | - | see also 3444 line | |
| 20097 | EMS1 | NM_005231.2 | 798 | gagcgccgttggcttgagtatc | 322789 | - | | breast cancer, head and neck squamous cell carcinoma, squamous cell cancer |
| 20098 | FARP2 | NM_014808.1 | 915 | ctggtccaaggtcgttaaactaa | 322820 | - | see also 6000 line | |
| 20099 | FLJ10210 | NM_018027.2 | 966 | aggtcaagcaatcgtaaactaca | 322877 | - | see also 7213 line | |
| 20100 | FRMD1 | NM_024919.2 | 535 | gggcacggcacctgctactactgc | 322913 | - | - | |
| 20101 | NICAL | NM_022765.2 | 1892 | gagtccacgtctccgatttgtct | 322929 | - | signal_transduction | |
| 20102 | URP2 | NM_031471.4 | 1960 | ccgaattgtacacgagtatatcg | 322964 | - | - | |
| 20103 | C14orf2 | NM_004894.1 | 100 | aagccctactacacaaagttta | 322965 | - | - | |
| 20104 | HAX1 | NM_006118.2 | 762 | cagcccagcccaaatcctattt | 322974 | - | - | diabetes |
| 20105 | TOMM70A | NM_014820.1 | 767 | ctgttagccgataaagttcttaa | 322991 | - | see also 6014 line | |
| 20106 | MRVI1 | NM_006069.2 | 2676 | ctggggctctacaattcctataa | 323044 | - | - | |

Figure 46

| 20107 | PSMD1 | NM_002807.2 | 1531 | acgccagcaatgatatcgttaga | 323096 | - | cell_cycle | - | - |
|---|---|---|---|---|---|---|---|---|---|
| 20108 | PSMD10 | NM_002814.2 | 102 | gaggggtgtgtgtctaacctaat | 323132 | - | - | - | - |
| 20109 | PSMD12 | NM_002816.2 | 460 | ttgatactctacgaatggttacc | 323150 | - | see also 1833 line | | |
| 20110 | ADFP | NM_001122.2 | 180 | atggcatccgttgcagttgatcc | 323240 | - | - | - | - |
| 20111 | JPH3 | NM_020655.2 | 2465 | cggagtcgccattctgtttatta | 323265 | - | - | - | - |
| 20112 | PIGS | NM_033198.2 | 438 | ctgactgtgtacgtgatatctga | 323270 | - | - | - | - |
| 20113 | SART2 | NM_013352.1 | 2579 | aagggcaggcaaacgctataaat | 323355 | - | see also 5405 line | | |
| 20114 | SDF2L1 | NM_022044.1 | 598 | gtgccaacacgcacaatacgtgg | 323367 | - | - | - | - |
| 20115 | VPREB3 | NM_013378.1 | 365 | aagacgacgcggattactactgc | 323371 | - | - | - | - |
| 20116 | PSMD11 | NM_002815.2 | 255 | atgtacgacccttcttgaattcc | 323372 | - | see also 1832 line | | |
| 20117 | PSMD13 | NM_002817.2 | 293 | ctgtccctcgtggaaatcattct | 323401 | - | see also 1834 line | | |
| 20118 | PSMD3 | NM_002809.2 | 1330 | atgggacctacaccctaattatc | 323434 | - | see also 1827 line | | |
| 20119 | PSMD4 | NM_002810.1 | 966 | caggcggaatcagcagacattga | 323455 | - | - | - | - |
| 20120 | PSMD5 | NM_005047.2 | 1476 | gtgaagggccatactatgtgaaa | 323491 | - | - | - | - |
| 20121 | SART1 | NM_005146.3 | 1938 | acgaggaaccgatcgtgaatagg | 323497 | - | - | - | - |
| 20122 | TGOLN2 | NM_006464.1 | 1154 | tagcgagaaggatgacctttatc | 323505 | - | see also 4536 line | | |
| 20123 | GOLPH4 | NM_014498.2 | 1437 | gaggtgtctcgaaataatgatgt | 323526 | - | see also 5728 line | | |
| 20124 | D4S234E | NM_014392.2 | 257 | atgacgcccctcgatgtcaatca | 323543 | - | see also 5655 line | | |
| 20125 | HMP19 | NM_015980.3 | 281 | gtgaagacaagaacggaatatca | 323564 | - | signal_transduction | - | - |
| 20126 | ZDHHC3 | NM_016598.1 | 722 | aagtacttcgtcctgtttacaat | 323574 | - | see also 6937 line | | |
| 20127 | CD63 | NM_001780.3 | 517 | gtgctgtggggctgctaactaca | 323586 | - | - | - | melanoma |
| 20128 | LAMP2 | NM_002294.1 | 207 | gtgcggtcttatgcattggaact | 323594 | - | - | - | diabetes |
| 20129 | CD68 | NM_001251.1 | 778 | tggacattctcggctcagaatgc | 323646 | - | - | - | leukemia |
| 20130 | LAMP1 | NM_005561.2 | 1179 | cagtcggcaattcctacaagtgc | 323670 | - | - | - | - |
| 20131 | LAPTM5 | NM_006762.1 | 698 | gcgtgtggcggtgctacagattg | 323679 | - | - | - | - |
| 20132 | PXMP2 | NM_018663.1 | 289 | atgccgtttacgggttcttcttc | 323694 | - | - | - | - |
| 20133 | SRP72 | NM_006947.2 | 881 | atgcggaaggagtagagtttaag | 323719 | - | kinase_related、 signal _transduction | | - |
| 20134 | MGC4293 | NM_031304.2 | 983 | gaggtggctctggacatgtatga | 323749 | - | - | - | - |
| 20135 | BRD7 | NM_013263.1 | 1706 | cagacccctccgaacatgatct | 323771 | - | see also 5381 line | | |
| 20136 | ICA1 | NM_004968.1 | 207 | aggatcgatatgctcaagataag | 323777 | - | see also 3450 line | | |
| 20137 | MVP | NM_005115.3 | 948 | cggaccggatggcaagaatcagc | 323823 | - | - | - | - |
| 20138 | NFKBIE | NM_004556.1 | 230 | cggttcggctgcccacagtaacc | 323834 | - | transcription_regulator | transcription factor、 cytoplasmic sequestering | - |
| 20139 | SNCG | NM_003087.1 | 175 | aagaccaaggagaatgttgtaca | 323838 | - | - | - | breast cancer |
| 20140 | TUB | NM_003320.3 | 643 | cagcaccagctaccaagttcaag | 323845 | - | - | - | obesity |

Figure 46

| 20141 | FLJ39369 | NM_152363.2 | 1206 | ctgcggacgggctgtattccaga | 323859 | - | kinase_related | - | - |
|---|---|---|---|---|---|---|---|---|---|
| 20142 | LEMD2 | NM_181336.2 | 1295 | aggacatggagcgctatccatat | 323876 | - | - | - | - |
| 20143 | ACRC | NM_052957.2 | 248 | caggcgagttgcatccttaatgt | 323878 | - | - | - | - |
| 20144 | AKIP | NM_017900.1 | 567 | gagacgcaagcagatcaagttcg | 323924 | - | - | - | - |
| 20145 | BARD1 | NM_000465.1 | 1655 | ctgcggcctgtcgattatacaga | 323979 | - | see also 373 line | | |
| 20146 | BOP1 | NM_015201.3 | 2225 | gggagtgctggacgtcatcttcc | 324006 | - | see also 6277 line | | |
| 20147 | BRD4 | NM_014299.1 | 795 | tggcgtttccacggtaccaaaca | 324017 | - | see also 5607 line | | |
| 20148 | C5orf6 | NM_016605.1 | 1069 | gcggccttcgttggactttgaca | 324039 | - | - | - | - |
| 20149 | COIL | NM_004645.1 | 1121 | tggaggcgttctggctcaaatgg | 324062 | - | see also 3164 line | | |
| 20150 | CTNNBL1 | NM_030877.3 | 536 | ttgctcggacacgataatacaga | 324080 | - | see also 8958 line | | |
| 20151 | D21S2056E | NM_003683.3 | 1000 | cagaaagcgtctctacaaagtga | 324116 | - | - | - | - |
| 20152 | DKFZp761B128 | NM_152437.1 | 1942 | aagggtttctttcatatatcaga | 324121 | - | - | - | - |
| 20153 | DNTTIP1 | NM_052951.2 | 691 | atgaatctaccacctttgtgttg | 324136 | - | - | - | - |
| 20154 | DXS9928E | NM_004699.1 | 900 | gagtggaccactcttcaactttg | 324142 | - | - | - | - |
| 20155 | FAM9A | NM_174951.2 | 1039 | aaggctgccaaggacactaaaga | 324148 | - | - | - | - |
| 20156 | FBXL4 | NM_012160.3 | 1628 | aagttatgcagccttaaacgact | 324206 | - | see also 5158 line | | |
| 20157 | FHL2 | NM_001450.2 | 725 | gtgactgcaaggacttgtcttac | 324229 | - | - | - | Alzheimer disease |
| 20158 | FJX1 | NM_014344.2 | 987 | tagtacaatggaccgacttaatc | 324241 | - | - | - | - |
| 20159 | FLJ13479 | NM_024706.3 | 620 | ctgtcctgtaccaagacatttcc | 324244 | - | - | - | - |
| 20160 | FLJ14011 | NM_022103.2 | 1340 | aggtctgcaatcggcattcatcc | 324255 | - | transcription_regulator | - | |
| 20161 | FLJ21628 | NM_030613.1 | 604 | aagcatgcatcttattgtacatc | 324265 | - | - | - | - |
| 20162 | FLJ30726 | NM_153018.1 | 1331 | caggcggacctctcaccttattg | 324292 | - | - | - | - |
| 20163 | FLJ32053 | NM_152484.2 | 2239 | cagtgctcactgcttaatttaca | 324313 | - | transcription_regulator | - | |
| 20164 | FLJ36870 | NM_173658.1 | 768 | gagttcgggccttatatcacatc | 324325 | - | - | - | - |
| 20165 | FLJ38002 | NM_152412.1 | 1068 | ttggttgtaattctactctaata | 324344 | - | - | - | - |
| 20166 | FLJ38451 | NM_175872.3 | 1618 | agggcgctgtcaactacaagttg | 324376 | - | - | - | - |
| 20167 | FLJ38705 | NM_173832.2 | 631 | gtgtgggcggatctttaagcaca | 324386 | - | - | - | - |
| 20168 | FLJ90764 | NM_173656.1 | 2023 | cagagcccgaatcctttgtctca | 324401 | - | - | - | - |
| 20169 | HIRIP3 | NM_003609.2 | 1768 | gtggtgcccatcgaaactacaag | 324409 | - | - | - | - |
| 20170 | HSPC111 | NM_016391.2 | 578 | atgcctgggaccacgctaaatcg | 324416 | - | - | - | - |
| 20171 | KIAA0103 | NM_014673.2 | 739 | atgtcggcaagtcatattgcttc | 324439 | - | see also 5854 line | | |
| 20172 | KIAA0924 | NM_014897.1 | 1124 | aggcgcatgcagatttgtgataa | 324460 | - | - | - | - |
| 20173 | KIAA1190 | NM_145166.2 | 480 | ttggtccctccatgagcataaca | 324478 | - | - | - | - |
| 20174 | KIAA1615 | NM_020951.1 | 1589 | ttggagtaggtagtgaacttact | 324497 | - | - | - | - |

Figure 46

| | | | | | | | | | |
|---|---|---|---|---|---|---|---|---|---|
| 20175 | LOC148213 | NM_138286.1 | 733 | ctgtcgtcacacattacaacaca | 324503 | - | - | - | - |
| 20176 | LOC84661 | NM_032574.1 | 373 | cagacagttgtgcctatcttatt | 324512 | - | kinase_related、 signal _transduction | - | - |
| 20177 | LOC90589 | NM_145233.2 | 586 | gtgatctcccctactttcgaaca | 324516 | - | - | - | - |
| 20178 | LPIN2 | NM_014646.2 | 948 | cagacagcgtgtcctaagagtga | 324529 | - | - | - | - |
| 20179 | MDM1 | NM_017440.2 | 197 | ctggacttagatcagatcaatta | 324564 | - | see also 7012 line | | |
| 20180 | MGC13071 | NM_032689.2 | 1386 | tagtagccatgaacttagtatac | 324626 | - | - | - | - |
| 20181 | MGC27466 | NM_152373.2 | 612 | atgagcacaagtcttaatccaat | 324637 | - | transcription_regulator | - | |
| 20182 | MGC45408 | NM_152289.1 | 1079 | atgccttaatgatcacattcaaa | 324651 | - | - | - | - |
| 20183 | NPAT | NM_002519.1 | 1365 | ttgagtccgtgcctaatttgaat | 324692 | - | - | - | - |
| 20184 | NUCKS | NM_022731.1 | 459 | cagcgatgaagatttcctaatgg | 324801 | - | - | - | - |
| 20185 | NUSAP1 | NM_016359.1 | 1157 | ttgtctcgtcccctcaactatga | 324808 | - | - | - | - |
| 20186 | PCNP | NM_020357.1 | 242 | taggtagtcagacgacaaagaaa | 324823 | - | - | - | - |
| 20187 | PELP1 | NM_014389.1 | 700 | cagttggcctgtgagtgttattc | 324828 | - | see also 5649 line | | |
| 20188 | PHLDA1 | NM_007350.1 | 1016 | aggccatcctggcggtcaaatcc | 324844 | - | - | - | - |
| 20189 | PLINP-1 | NM_052850.2 | 229 | ggggtggtccccggttcgttatg | 324846 | - | - | - | - |
| 20190 | PRAC | NM_032391.2 | 129 | atgttgtgcgcccatttctcaga | 324849 | - | - | - | - |
| 20191 | RBBP5 | NM_005057.1 | 241 | ctgtaatgatggccgaattgtca | 324854 | - | see also 3526 line | | |
| 20192 | RNF144 | NM_014746.2 | 1086 | tggagtctctggacgatgatttc | 324891 | - | - | - | - |
| 20193 | SH3BGRL2 | NM_031469.1 | 398 | ccgatactgtggagattatgaca | 324900 | | - | - | - |
| 20194 | SH3BGRL3 | NM_031286.1 | 106 | ccggctcccgcgaaatcaagtcc | 324903 | - | - | - | - |
| 20195 | UBCE7IP5 | NM_014948.2 | 716 | cggcggtatcccttgtatcaagc | 324909 | - | see also 6146 line | | |
| 20196 | UTX | NM_021140.1 | 1461 | cagctccgcgcaaatagaaataa | 324931 | - | see also 8100 line | | |
| 20197 | UTY | NM_007125.3 | 2626 | ttgctaggcagtaattgtatagc | 324977 | - | - | - | - |
| 20198 | WDR13 | NM_017883.3 | 1013 | agggtgcgcttcgccaatgatga | 325009 | - | - | - | - |
| 20199 | WDR3 | NM_006784.1 | 1294 | gtgatgtgcggactttgtcattc | 325046 | - | - | - | - |
| 20200 | WRB | NM_004627.2 | 423 | gtggctgtcgtgccgagtaaatg | 325100 | - | - | - | congenital heart disease |
| 20201 | ZFP1 | NM_153688.1 | 636 | tggagtagaatattctgaataca | 325166 | - | - | - | - |
| 20202 | ZNF285 | NM_152354.2 | 264 | aagttacctttcgcaagaagtgc | 325176 | - | transcription_regulator | - | |
| 20203 | ZNF491 | NM_152356.2 | 784 | ttggcacagttctgttcgaatcc | 325190 | - | transcription_regulator | - | |

Figure 46

| | | | | | | | | | |
|---|---|---|---|---|---|---|---|---|---|
| 20204 | ZNF493 | NM_175910.4 | 691 | gtggcacatcttctaccaattc | 325194 | - | - | | |
| 20205 | ZNF501 | NM_145044.1 | 573 | ttgtttccgtaaacagtcaaatc | 325199 | - | transcription_regulator | | |
| 20206 | ZNF502 | NM_033210.3 | 531 | aagcacccctgaaattattagt | 325210 | - | transcription_regulator | | |
| 20207 | ATF7IP | NM_018179.3 | 1854 | cagtctaaacgtcgtcgatatat | 325249 | - | see also 7304 line | | |
| 20208 | C14orf18 | NM_021178.2 | 966 | atggagcgcaatcgtcagtatca | 325317 | - | | | |
| 20209 | LOC112840 | NM_080666.1 | 1262 | acgagtacgagttcaatagtaatg | 325365 | - | | | |
| 20210 | LRP10 | NM_014045.2 | 1610 | ccgctgcaactaccagacttct | 325376 | - | | | |
| 20211 | WDR23 | NM_025230.3 | 947 | tggttcgttggggatcgataca | 325394 | - | | | |
| 20212 | FLJ12847 | NM_024596.1 | 933 | ctgtgggagttcatatgatg | 325439 | - | | | |
| 20213 | MDC1 | NM_014641.1 | 6094 | cagcatgctcggtcctataagc | 325518 | - | | | |
| 20214 | PAXIP1L | NM_007349.2 | 534 | tgggctttggttacgttctatgg | 325521 | - | see also 5082 line | | |
| 20215 | CD9 | NM_001769.2 | 425 | ctgcggccatctggggatattc | 325596 | - | | | esophageal carcinoma, breast cancer, lung cancer, pancreatic cancer |
| 20216 | CDW52 | NM_001803.1 | 64 | ctggttatggtacagatacaaac | 325601 | - | | | |
| 20217 | DSCR2 | NM_003720.1 | 661 | tcggcgtgttgtccattgctaga | 325627 | - | | | |
| 20218 | GARP | NM_005512.1 | 1636 | ctgcctcaagcggctcaatcttg | 325639 | - | | | |
| 20219 | GPM6A | NM_005277.2 | 724 | ttgctatggttcactacctatg | 325658 | - | see also 3702 line | | |
| 20220 | GYPE | NM_002102.2 | 247 | ttgttgttgttggaatgatcatc | 325671 | - | | | |
| 20221 | HEM1 | NM_005337.2 | 822 | aagtaatgagcgctggatatc | 325699 | - | see also 3724 line | | |
| 20222 | JTB | NM_006694.2 | 898 | gagtgctccatgctctaattt | 325744 | - | | | |
| 20223 | KAI1 | NM_002231.2 | 184 | gggctcagcctgtatcaaagtca | 325750 | - | | | hepatocellular carcinoma, pancreatic cancer, prostate cancer |
| 20224 | M11S1 | NM_005898.2 | 2178 | tggctatcaacgggatggatatc | 325804 | - | see also 4104 line | | |
| 20225 | MLANA | NM_005511.1 | 174 | ctgctcatcggctgttggtattg | 325810 | - | | | melanoma |
| 20226 | MOX2 | NM_005944.3 | 462 | ctgcctcaccgtctatgttacagc | 325824 | - | | | arthritis |
| 20227 | NET-5 | NM_066675.2 | 299 | ttgccatagccaccattgtcatg | 325832 | - | | | |
| 20228 | NET-7 | NM_012339.2 | 682 | caggaacaacagacagaagttgtca | 325844 | - | | | |
| 20229 | NKG7 | NM_005601.2 | 162 | ttgctttgagcaccgatttctgg | 325849 | - | | | |
| 20230 | PODXL | NM_005397.2 | 1289 | ctgatatgccgagccagtcaaagc | 325859 | - | | | |
| 20231 | PTPRCAP | NM_005608.1 | 349 | gagctggggtccacagacaatga | 325865 | - | | antimicrobial peptide | |
| 20232 | SYNGR3 | NM_004209.4 | 402 | atgtgcgttccagcaaatcagc | 325867 | - | | | |
| 20233 | T1A-2 | NM_006474.3 | 543 | cagtccagcgcgcaagaacaaagt | 325874 | - | | | |
| 20234 | TM4SF13 | NM_014399.2 | 247 | gcgccctcaactgctttacacc | 325879 | - | | | |
| 20235 | TM7SF1 | NM_003272.1 | 843 | gagcgtcattccttgattatg | 325897 | - | | | |

Figure 46

| 20236 | TMEM4 | NM_014255.2 | 575 | atgggatctttccggatcaatcc | 325914 | - | - | - | - |
|---|---|---|---|---|---|---|---|---|---|
| 20237 | TMEM5 | NM_014254.1 | 504 | aagtcaatcgtaggaagaacaca | 325930 | - | - | - | - |
| 20238 | ADIPOR1 | NM_015999.2 | 653 | ttgaccatgctcagaccaaatat | 325957 | - | see also 6600 line | | |
| 20239 | ADIPOR2 | NM_024551.2 | 504 | tggcgagtgatccctcatgatgt | 325976 | - | - | - | - |
| 20240 | AMIGO2 | NM_181847.2 | 1784 | atgcccattcatcgattctcagt | 326031 | - | see also 10258 line | | |
| 20241 | ANTXR2 | NM_058172.1 | 187 | aagctagcgaatgaacaaattca | 326040 | - | - | - | - |
| 20242 | ARL6IP | NM_015161.1 | 556 | ctgatagtgacttccttactatt | 326070 | - | - | - | - |
| 20243 | BM88 | NM_016564.2 | 524 | ctggtcctgtgagaacttcaacc | 326073 | - | - | - | - |
| 20244 | C11orf15 | NM_020644.1 | 258 | atgtatctgccctccctataaag | 326077 | - | see also 7901 line | | |
| 20245 | C19orf4 | NM_012109.1 | 375 | ctgcgtggaagcctatgtgaagg | 326089 | - | - | - | - |
| 20246 | C1orf8 | NM_004872.3 | 987 | ttgtcctctcggtgatggtattg | 326110 | - | see also 3385 line | | |
| 20247 | C20orf103 | NM_012261.2 | 557 | tgggtggatcgcgcatatgcact | 326120 | - | - | - | - |
| 20248 | C20orf165 | NM_080608.3 | 267 | cagccgaaactgccacaaattcc | 326130 | - | - | - | - |
| 20249 | C20orf46 | NM_018354.1 | 957 | ccgatcccagcgggatgatctgt | 326138 | - | - | - | - |
| 20250 | C20orf52 | NM_080748.1 | 354 | gcggcacctttggcacattcatg | 326146 | - | - | - | - |
| 20251 | C20orf54 | NM_033409.2 | 941 | ctgcctcgtggcgttctttgtcc | 326153 | - | - | - | - |
| 20252 | C20orf75 | NM_152611.2 | 1600 | ttgtccttgagcctgatatctca | 326161 | - | - | - | - |
| 20253 | C21orf61 | NM_178817.2 | 275 | aagctgaaagcccacaaacattc | 326165 | - | - | - | - |
| 20254 | C22orf20 | NM_025225.2 | 879 | gagatatgccttcgaggatattt | 326175 | - | - | - | - |
| 20255 | C22orf5 | NM_012264.2 | 645 | ctggggaaagacttattccatcg | 326189 | - | - | - | - |
| 20256 | C3orf4 | NM_019895.1 | 1500 | atgtatccggtgaatttggatgg | 326211 | - | see also 7752 line | | |
| 20257 | C6orf31 | NM_030651.2 | 663 | ccggtgggcacgccatatgcagg | 326217 | - | - | - | - |
| 20258 | CAV2 | NM_001233.3 | 832 | gcgtaggacgatgcttctcttct | 326227 | - | - | - | - |
| 20259 | CDC91L1 | NM_080476.3 | 702 | gggaagcctagtggtaatcattt | 326247 | - | - | - | - |
| 20260 | CEACAM7 | NM_006890.1 | 614 | cagcgccacaaagaatgacatag | 326272 | - | - | - | colorectal cancer、colorectal cancers |
| 20261 | CGI-119 | NM_016056.1 | 437 | aagctttcatactgactactaca | 326283 | - | - | - | - |
| 20262 | CGI-147 | NM_016077.1 | 218 | tggagccttcgagtatgctttgg | 326291 | - | apoptosis | - | - |
| 20263 | CKAP4 | NM_006825.2 | 399 | cagggcgctcaactttctcttct | 326313 | - | - | - | - |
| 20264 | CLN6 | NM_017882.1 | 549 | gtgagaaccccatcatcaagaat | 326323 | - | - | - | ceroid-lipofuscinosis |
| 20265 | CUZD1 | NM_022034.3 | 2705 | tggtgactgtagcgacaatcaca | 326381 | - | see also 8242 line | | |
| 20266 | DC-TM4F2 | NM_030927.1 | 561 | ctgggacctcaacgtctacttca | 326386 | - | see also 8965 line | | |
| 20267 | DCAL1 | NM_172004.2 | 273 | tcggacttccccgttagaactcg | 326400 | - | - | - | - |
| 20268 | ELOVL5 | NM_021814.2 | 599 | aggagtatgggaaggcaaataca | 326415 | - | - | - | - |
| 20269 | ELOVL6 | NM_024090.1 | 795 | gcgtgcacgccgtgatgtactct | 326446 | - | see also 8588 line | | |
| 20270 | FAM14A | NM_032036.1 | 290 | ttggggaattcaccttcttcttc | 326466 | - | - | - | - |

Figure 46

| | | | | | | | |
|---|---|---|---|---|---|---|---|
| 20271 | FAM14B | NM_145249.1 | 449 | tggactctctgtgacatctaaag | 326471 | - | |
| 20272 | FLJ10134 | NM_018004.1 | 630 | gtgtggcagatatcttatgtttc | 326486 | - | |
| 20273 | FLJ22233 | NM_024959.2 | 1031 | aggaggaccggtatcttctaat | 326507 | - | |
| 20274 | FLJ22800 | NM_024795.1 | 55 | atggacatctgcaatggattca | 326518 | - | |
| 20275 | FLJ30002 | NM_152341.2 | 728 | ctgcttggggactggtaaatgt | 326537 | - | see also 9841 line |
| 20276 | FLOT1 | NM_005803.2 | 590 | gggcatcagtgtggttagctaca | 326541 | - | |
| 20277 | GHITM | NM_014394.1 | 961 | gttgccaatgtacggtggattagt | 326572 | - | |
| 20278 | GPSN2 | NM_004868.1 | 74 | ccgcgtggatggcctattacatc | 326578 | - | |
| 20279 | GYPA | NM_002099.2 | 447 | ctgacacagacgtgccttaagt | 326598 | - | |
| 20280 | HCST | NM_014266.3 | 212 | ctggcacttcaggctcttgttcc | 326602 | - | |
| 20281 | HSPC039 | NM_016097.2 | 223 | atgaaccttattcgatcgtgtaag | 326608 | - | |
| 20282 | HTGN29 | NM_020199.1 | 341 | gtgttgtatcacggactgattca | 326615 | - | |
| 20283 | HUCEP11 | NM_014858.1 | 8 | aggttgttcagcccttaatgagc | 326632 | - | |
| 20284 | IFRG28 | NM_022147.1 | 337 | aagtgctcctggtcccaatatga | 326638 | - | |
| 20285 | IGSF8 | NM_052868.1 | 1947 | ctgtccttggtaccatcacttgc | 326652 | - | receptor acitivity |
| 20286 | ITM2A | NM_004867.2 | 833 | tagacacttcccacgaattta | 326673 | - | |
| 20287 | ITM2C | NM_030926.3 | 343 | ctgtcacatctacagatactc | 326675 | - | see also 8964 line |
| 20288 | KIAA0133 | NM_014777.1 | 4388 | ctgcctacggtcctaaagrgtgc | 326765 | - | |
| 20289 | KIAA0152 | NM_014730.1 | 708 | gagtttgtcaaggggtactatga | 326788 | - | |
| 20290 | KIAA0247 | NM_014734.1 | 623 | ctgtctatagtggcttctactgc | 326800 | - | |
| 20291 | KIAA0779 | NM_015008.1 | 983 | caggaggagcggatatagatgtga | 326823 | - | |
| 20292 | LAPTM4B | NM_018407.3 | 1033 | gtgctgctaccccgtatgatga | 326878 | - | |
| 20293 | LEPROTL1 | NM_015344.1 | 268 | atgagtaacgcttgtaaggaact | 326895 | - | |
| 20294 | LOC116211 | NM_138461.1 | 351 | gagatacggctgcttcagtaaga | 326902 | - | |
| 20295 | LOC116441 | NM_138786.1 | 188 | ccgcttgcactttgagtataat | 326909 | - | |
| 20296 | LRRTM1 | NM_178839.3 | 995 | gttggctctatctgatcacaatc | 326937 | - | |
| 20297 | LRRTM2 | NM_015564.1 | 991 | aagtttggctgccaatggatttg | 326959 | - | see also 6475 line |
| 20298 | MAL2 | NM_052886.1 | 551 | atgacgaacagcttgttatggttg | 327006 | - | |
| 20299 | MGC35450 | NM_153708.1 | 233 | ttggaagcagtaactggaattgc | 327007 | - | |
| 20300 | MPDU1 | NM_004870.1 | 26 | gaggcggacgaccgcttaaacg | 327008 | - | |
| 20301 | MPRG | NM_017705.2 | 1258 | cagatagctggagccatacttct | 327025 | - | |
| 20302 | NIFIE14 | NM_032635.2 | 557 | tggatgataacacgctatgatct | 327036 | - | |
| 20303 | NMA | NM_012342.1 | 987 | ggggcagttgcaaagttagact | 327047 | - | |

Figure 46

| No. | Gene | Accession | Length | Sequence | ID | Note | Extra |
|---|---|---|---|---|---|---|---|
| 20304 | NPDC1 | NM_015392.2 | 989 | ctgtgcctgagcggcataaga | 327050 | - | |
| 20305 | PANX1 | NM_015368.3 | 1460 | cagggatcgacccaatgctact | 327076 | - | |
| 20306 | PANX2 | NM_052839.2 | 1404 | ccgctcgcccaccactacaagg | 327084 | - | |
| 20307 | PANX3 | NM_052959.2 | 501 | gagttccgacccctatgtgtct | 327092 | see also 9472 line | |
| 20308 | PAQR3 | NM_177453.2 | 686 | ctggcgtcaggtgtacttgatca | 327127 | see also 10163 line | |
| 20309 | PAQR6 | NM_024897.2 | 856 | aggagccttcgcctatccattcc | 327137 | - | |
| 20310 | PKD1L1 | NM_138295.2 | 2441 | gggcgactctcaggtatcactgg | 327200 | - | |
| 20311 | RFT1 | NM_052859.2 | 169 | aggaaatcgttgccgtagtaaat | 327307 | see also 9439 line | |
| 20312 | SECTM1 | NM_003004.1 | 474 | tcgtgggaccacagagaaataac | 327342 | kinase_related | |
| 20313 | SEZ6L | NM_021115.2 | 2940 | aagatgtcgctactattccaacc | 327384 | - | non-small-cell lung cancer |
| 20314 | SLC24A4 | NM_153646.1 | 1396 | gtgactattatcgatacacact | 327413 | see also 10003 line | |
| 20315 | SMP1 | NM_014313.2 | 380 | aagtccgaggtgatagttacagt | 327431 | - | |
| 20316 | STARD3NL | NM_032016.2 | 330 | ttgacctcttattcgtaacatta | 327518 | | |
| 20317 | SYNGR4 | NM_012451.2 | 523 | ttgccggcaccgcttcaagaca | 327533 | - | |
| 20318 | TACSTD1 | NM_002354.1 | 474 | gcgatgagagcgggctctttaag | 327543 | see also 1522 line | |
| 20319 | TDE1 | NM_006811.2 | 875 | ttgcgttggcttcattatatat | 327583 | see also 4786 line | |
| 20320 | TDE2 | NM_020755.2 | 792 | ttgttgcttcgtaatgtctata | 327621 | see also 7945 line | |
| 20321 | TED | NM_015686.1 | 766 | gggaggcgtgtagcaactgtatc | 327644 | receptor_acitivity | |
| 20322 | TM4-B | NM_012466.2 | 554 | acgagccagacgactattctaca | 327666 | - | |
| 20323 | TM4SF12 | NM_012338.2 | 249 | gtggttcatccggtcatgattgc | 327674 | see also 5298 line | |
| 20324 | TM7SF3 | NM_016551.1 | 1610 | gagggcggagtgacaaaacattct | 327738 | - | |
| 20325 | TMC2 | NM_080751.1 | 2027 | aagcctccgatccaacaacttc | 327783 | see also 9522 line | |
| 20326 | TMEFF1 | NM_003692.2 | 318 | gggagtctgacgtaagagtttgt | 327792 | - | |
| 20327 | TMEM10 | NM_033207.2 | 760 | ccgttacgtcctactatagaaa | 327826 | - | |
| 20328 | TMEM15 | NM_014908.1 | 748 | gggcttggctggagggttatca | 327832 | - | |
| 20329 | TMEM2 | NM_013390.1 | 1448 | gtggtgcaagcaacagactattc | 327887 | see also 5419 line | |
| 20330 | TMEM7 | NM_031440.1 | 353 | gtgccccaacctcgtttgagg | 327983 | - | |
| 20331 | TNMD | NM_022144.1 | 1017 | ccgcgtcgtgaacctttactag | 328011 | see also 8313 line | |
| 20332 | UPK1A | NM_007000.2 | 570 | ctgtcgccggacgggaaacttca | 328020 | - | |
| 20333 | UPK3A | NM_006953.1 | 649 | gaggcatgatcgtcatcacttcc | 328028 | - | |
| 20334 | VANGL1 | NM_138959.1 | 1431 | tggattacgggatggaattgtgt | 328058 | see also 9642 line | |
| 20335 | ZDHHC1 | NM_013304.1 | 1505 | gaggaagaggcgcgtgtataaag | 328073 | - | |
| 20336 | ZDHHC11 | NM_024786.1 | 869 | tgggaagccggaattattggttc | 328086 | - | |
| 20337 | ZDHHC12 | NM_032799.3 | 419 | gagcgcaaccaccacctctttgt | 328091 | - | |
| 20338 | ZDHHC2 | NM_016353.2 | 1270 | gagcaatcctgcattaaccattg | 328119 | see also 6833 line | |
| 20339 | ZDHHC4 | NM_018106.2 | 1159 | aagtccaccgaacattcactcc | 328136 | - | |

Figure 46

| | | | | | | | | | |
|---|---|---|---|---|---|---|---|---|---|
| 20340 | ZDHHC5 | NM_015457.1 | 652 | atgaacaggttacgggtaaattc | 328151 | - | - | - | - |
| 20341 | ZDHHC6 | NM_022494.1 | 1506 | gaggtttacgatactggttatat | 328201 | - | - | - | - |
| 20342 | ZDHHC7 | NM_017740.1 | 592 | tggtcaacggggtcatctttaac | 328210 | - | - | - | - |
| 20343 | ZDHHC8 | NM_013373.1 | 524 | ctgcatcgggcgtcgaaactatc | 328226 | - | - | - | - |
| 20344 | ZDHHC9 | NM_016032.1 | 690 | gcgaccaccgcctcgtatcaaga | 328239 | - | - | - | - |
| 20345 | SNAP91 | NM_014841.1 | 2927 | aggacccattagcggatcttaac | 328291 | - | see also 6046 line | | |
| 20346 | MAGEA1 | NM_004988.3 | 445 | ggggccaagcacctcttgtatcc | 328296 | - | - | - | neuroblastoma、melanoma |
| 20347 | SSFA2 | NM_006751.3 | 3265 | tggcagtagaagcgctgataact | 328362 | - | - | - | - |
| 20348 | CACNA2D2 | NM_006030.1 | 1435 | cggacggtgcgcgtgtttacttt | 328386 | - | - | - | - |
| 20349 | CACNA2D3 | NM_018398.1 | 2778 | tggtggcactccgatatgacagc | 328486 | - | see also 7440 line | | |
| 20350 | CACNA2D4 | NM_172364.2 | 2274 | tggccggtgactggatctactgc | 328521 | - | | - | - |
| 20351 | CKLFSF2 | NM_144673.2 | 667 | aagtcggcgatccatgaatctcc | 328569 | - | - | - | - |
| 20352 | CKLFSF3 | NM_144601.2 | 734 | ttgctaccatcgtgtttgcaact | 328576 | - | - | - | - |
| 20353 | CKLFSF4 | NM_178818.2 | 657 | ttggcgactgcggcatatgcagt | 328585 | - | see also 10209 line | | |
| 20354 | CKLFSF5 | NM_138460.2 | 629 | cggcgctactggagttcttcatc | 328592 | - | - | - | - |
| 20355 | CKLFSF6 | NM_017801.2 | 336 | aagttgtatcacaatgtacttta | 328597 | - | - | - | - |
| 20356 | CKLFSF7 | NM_138410.2 | 704 | aagcatatggctgtcctataaga | 328621 | - | see also 9617 line | | |
| 20357 | DOCK4 | NM_014705.2 | 4884 | tagacgcagcccgttaagttacc | 328752 | - | - | - | - |
| 20358 | FLJ30532 | NM_144724.1 | 899 | atgtattaccggaccattcttct | 328783 | - | - | - | - |
| 20359 | FLJ33990 | NM_153226.1 | 1116 | ttggagcggccattcgtaaatgg | 328843 | - | see also 9978 line | | |
| 20360 | GP2 | NM_001502.1 | 687 | caggacctcaatagttctgatgt | 328851 | - | - | - | - |
| 20361 | KIAA0792 | NM_014698.1 | 761 | ctggacaaagacccgtatagttt | 328869 | - | - | - | - |
| 20362 | MAMDC2 | NM_153267.3 | 2460 | ctggctacgagcactgattgaat | 328946 | - | see also 9985 line | | |
| 20363 | MGC3295 | NM_025246.1 | 1545 | ttggttagcacagtacaacattt | 328982 | - | - | - | - |
| 20364 | STOML3 | NM_145286.1 | 956 | tggcgtcagctatgataaccaca | 329011 | - | - | - | - |
| 20365 | TDE2L | NM_178865.2 | 750 | ctggtacgcaggcctcttcttct | 329014 | - | - | - | - |
| 20366 | TERE1 | NM_013319.1 | 372 | ctgtcgggagagactgtcaaagc | 329019 | - | - | - | - |
| 20367 | CSPG5 | NM_006574.2 | 758 | ttgacttatacgatgatttcacc | 329044 | - | - | - | - |
| 20368 | NESG1 | NM_012337.1 | 991 | gagattaagcgcatcaatgatga | 329067 | - | - | - | - |
| 20369 | FGL1 | NM_004467.2 | 1010 | tagccgttatgcacaatataaga | 329078 | - | - | - | - |
| 20370 | CHGB | NM_001819.1 | 1660 | gagattaggggaactgttcaacc | 329103 | - | - | peptide hormone | - |
| 20371 | CRISP2 | NM_003296.1 | 702 | ctgtcccaatcaagatagtctaa | 329121 | - | - | - | - |
| 20372 | FHR-3 | NM_021023.1 | 339 | cagggtaactctacagaagttgc | 329132 | - | - | - | - |
| 20373 | HFL3 | NM_005666.1 | 770 | aagaacaggtgacatagttgaat | 329137 | - | - | - | - |

Figure 46

| | | | | | | | | | |
|---|---|---|---|---|---|---|---|---|---|
| 20374 | PRH1 | NM_006250.1 | 124 | aagatgttcccctcgtaatatca | 329140 | - | gpcr、receptor_acitivity、signal_transduction | - | dental caries in children |
| 20375 | PRH2 | NM_005042.1 | 124 | aagacgttcccttggtaatatca | 329141 | - | - | rhodopsin-like receptor | - |
| 20376 | SCGB1D1 | NM_006552.1 | 244 | tggatacgatggcctatgagaaa | 329144 | - | - | - | - |
| 20377 | SCGB1D2 | NM_006551.2 | 154 | ctgttagacttcttcttcattag | 329146 | - | - | - | - |
| 20378 | SEMG2 | NM_003008.2 | 1745 | aagacagaaatccaatatctaca | 329155 | - | - | - | - |
| 20379 | ASPN | NM_017680.2 | 470 | gtgctattcacgagttgtacatt | 329159 | - | - | - | - |
| 20380 | CRTAP | NM_006371.2 | 644 | ccgagcagtgcgggcatacaacg | 329197 | - | - | - | - |
| 20381 | DCN | NM_001920.2 | 241 | ctgggctggaccgtttcaacaga | 329208 | - | - | - | Alzheimer disease |
| 20382 | GPC2 | NM_152742.1 | 291 | ccgggatatagcttaaacctaa | 329231 | - | receptor_acitivity | - | - |
| 20383 | C9orf19 | NM_022343.2 | 143 | cgggaggctcaacagtattctga | 329247 | - | - | - | - |
| 20384 | DKFZP434B044 | NM_031476.1 | 771 | ttgggagaacgcggtctactttg | 329258 | - | - | - | - |
| 20385 | FGL2 | NM_006682.1 | 289 | aaggaaatcgtaaatagtctaaa | 329274 | - | see also 4716 line | | |
| 20386 | LOC57151 | NM_020426.1 | 396 | gtgcaacgattataagagttact | 329324 | - | - | - | - |
| 20387 | LOC83690 | NM_031461.2 | 883 | caggtcgtgtgggcaactagtaa | 329348 | - | - | - | - |
| 20388 | MGC26768 | NM_144634.2 | 514 | aggaaccgctgccatatgtcatg | 329393 | - | - | - | - |
| 20389 | MGC39497 | NM_152436.1 | 151 | aggaggacgtagactttatcaac | 329400 | - | - | - | - |
| 20390 | R3HDML | NM_178491.1 | 642 | tggcgagtccccgtacaagatgg | 329426 | - | - | - | - |
| 20391 | SPACA3 | NM_173847.2 | 337 | aagctctacggtcgttgtgaact | 329432 | - | - | - | - |
| 20392 | C6orf135 | NM_024839.1 | 366 | cgggagccaagcagattccaaac | 329440 | - | - | - | - |
| 20393 | 182-FIP | NM_020772.1 | 670 | aggaaagctaggcgcaatagtgc | 378684 | - | - | - | - |
| 20394 | 24432 | NM_022914.1 | 992 | tcgcaaccaagacttagatgttc | 384854 | - | - | - | - |
| 20395 | 24b2/STAC2 | NM_198993.1 | 628 | agggcttgcgatgtaagatgtgc | 440387 | - | see also 10353 line | | |
| 20396 | 384D8-2 | NM_014551.3 | 283 | ctgcctgcgtctacagtaagaag | 340830 | - | - | - | - |
| 20397 | 7A5 | NM_182762.1 | 764 | ttgatccgccacacatgcttaac | 435165 | - | - | - | - |
| 20398 | 7h3 | NM_033025.3 | 1105 | gtgggactgtaccgtctttgtgg | 402608 | - | - | - | - |
| 20399 | AASDHPPT | NM_015423.2 | 246 | ctgctggcagtgcgatcgattca | 352598 | - | see also 6406 line | | |
| 20400 | AAT1 | NM_033364.2 | 1073 | cagcgcacgcatgtatcaacaat | 403727 | - | see also 9414 line | | |
| 20401 | ABC1 | NM_022070.3 | 1834 | ctggactcggtaatagcaattca | 382108 | - | - | - | - |
| 20402 | ACMSD | NM_138326.1 | 337 | gggatccagaagttcgtattaga | 407495 | - | - | - | - |
| 20403 | ACN9 | NM_020186.1 | 110 | gggcgaccagtacgtgaaagacg | 375862 | - | - | - | - |

Figure 46

| 20404 | ACY-3 | NM_080658.1 | 1001 | cggtgtaccccgtgttcattaac | 406564 | - | - | - | - |
|---|---|---|---|---|---|---|---|---|---|
| 20405 | AD024 | NM_020675.3 | 525 | cagaaatctgcagacttgtataa | 377913 | - | - | - | - |
| 20406 | AD031 | NM_032021.2 | 464 | ctgttgattcgcagatgtaatat | 397613 | - | - | - | - |
| 20407 | AD158 | NM_032270.2 | 236 | ttggcgtgtttggatgtacttta | 399248 | - | see also 9130 line | | |
| 20408 | AD24 | NM_022451.9 | 464 | aagcatgaacgcattatagataa | 382794 | - | see also 8360 line | | |
| 20409 | ADAMTS20 | NM_025003.2 | 277 | ctgaccgccgatgcatcctttct | 392899 | - | see also 8832 line | | |
| 20410 | ADAMTSL1 | NM_052866.2 | 624 | tggtccgagggcagtataaatcc | 404547 | - | - | - | - |
| 20411 | ADCK2 | NM_052853.2 | 1265 | ctgcgttacgaagctcagaatct | 404429 | - | - | - | - |
| 20412 | ADCK4 | NM_024876.2 | 1033 | ccgggtcccagccgtggttaagg | 391630 | - | kinase_related | - | - |
| 20413 | ADCK5 | NM_174922.2 | 1040 | ggggctggcagtgcatgacatag | 428685 | - | - | - | - |
| 20414 | ADMP | NM_145035.2 | 384 | gggcacgatcctttcatctattt | 413020 | - | - | - | - |
| 20415 | AEBP2 | NM_153207.2 | 731 | aagacatcgagccatatgctta | 422134 | - | see also 9975 line | | |
| 20416 | AF15Q14 | NM_020380.2 | 5148 | ttgtagtgtcactggtattgatg | 377309 | - | - | - | - |
| 20417 | AFAP | NM_021638.3 | 1700 | cagcgggacggcacttcattatg | 380970 | - | - | - | - |
| 20418 | AGR2 | NM_006408.2 | 195 | aggttggggtgaccaactcatct | 335402 | - | - | - | - |
| 20419 | AGTRAP | NM_020350.3 | 447 | gtgcctaccagacgattgactca | 376822 | - | - | - | - |
| 20420 | AHI1 | NM_017651.3 | 3637 | aaggagcgatcccctcctttaag | 360244 | - | see also 7078 line | | |
| 20421 | AHSA1 | NM_012111.1 | 373 | atgaatgggagcgtcaaactaaac | 337500 | - | see also 5140 line | | |
| 20422 | AIG1 | NM_016108.2 | 476 | aggacatcgcaccatcagtatcc | 356283 | - | - | - | - |
| 20423 | AIM1L | NM_017977.1 | 194 | tggtaagtgccacattgacatgg | 365016 | - | - | - | - |
| 20424 | AK7 | NM_152327.1 | 1275 | gcgattgttgcccctaacgatgt | 415654 | - | kinase_related | - | - |
| 20425 | AKNA | NM_030767.2 | 1007 | gagaaacgaccagattcttctgc | 394897 | - | - | - | - |
| 20426 | AKR1CL2 | NM_031436.2 | 235 | agggatccgttgcaagatcaagg | 396958 | - | - | - | - |
| 20427 | ALEX1 | NM_016608.1 | 1291 | ctgggctaagactgttaaccaac | 358402 | - | - | - | - |
| 20428 | ALEX2 | NM_014782.3 | 826 | tgggcctaaggccgaatcagtag | 343402 | - | see also 5963 line | | |
| 20429 | ALEX3 | NM_016607.2 | 1055 | tcgggatcccatagttaaggaaa | 358370 | - | - | - | - |
| 20430 | ALG10 | NM_032834.1 | 671 | ttgttagttccagcctatatatt | 401877 | - | - | - | - |
| 20431 | ALLC | NM_018436.2 | 653 | tggaattgcacgacttagagtat | 371493 | - | - | - | - |
| 20432 | ALMS1 | NM_015120.2 | 11387 | ctgtcgaatcagatatattgacc | 348470 | - | - | - | Alstrom syndrome、diabetes mellitus、Alzheimer disease、diabetes |
| 20433 | ALS2CR17 | NM_198945.1 | 2071 | atgttgaccaatccaactacttt | 440074 | - | see also 10350 line | | |
| 20434 | ALS2CR19 | NM_057177.3 | 1994 | tgggattgggcctcgaagattac | 406158 | - | cell_cycle | - | - |
| 20435 | ALS2CR4 | NM_152388.1 | 1389 | gtggctcttctggttggattatc | 416160 | - | - | - | - |
| 20436 | AMACO | NM_198496.1 | 706 | gagacggaacttgctctgaaata | 438601 | - | - | - | - |
| 20437 | AMICA | NM_153206.1 | 599 | gagcacgccaaggacgaatatgt | 422103 | - | - | - | - |

Figure 46

| | | | | | | | | |
|---|---|---|---|---|---|---|---|---|
| 20438 | AMOT | NM_133265.1 | 979 | gcgcgtctcggaggcatatgaga | 407172 | - | | |
| 20439 | AMOTL1 | NM_130847.1 | 2455 | ctgcagcagcgatctcgtaaaga | 407089 | - | | |
| 20440 | AMOTL2 | NM_016201.2 | 918 | cagtaccctcatgttgtactagc | 356549 | - | | |
| 20441 | AMSH-LP | NM_020799.1 | 117 | gcgagtccgtgcctaagcaagc | 379047 | - | | |
| 20442 | ANAPC1 | NM_022662.1 | 5960 | ctgccacctataggactagaagg | 383684 | - | see also 8427 line | |
| 20443 | ANC_2H01 | NM_016331.1 | 1479 | tagagttaagtgataagtataac | 357068 | - | see also 6814 line | |
| 20444 | ANGPTL5 | NM_178127.2 | 1416 | atgcacttaggacgttattcagg | 430517 | - | | |
| 20445 | ANGPTL6 | NM_031917.2 | 1179 | gggccttgaacccgtgtatacagc | 397298 | - | | |
| 20446 | ANKMY1 | NM_016552.1 | 246 | ttggcccacaggcgagtcatacc | 358081 | - | | |
| 20447 | ANKRD10 | NM_017664.1 | 936 | tagctccgtatcgaatacattga | 360472 | - | | |
| 20448 | ANKRD13 | NM_033121.1 | 801 | aagacaactgggcgggagttaatg | 403388 | - | | |
| 20449 | ANKRD15 | NM_015158.1 | 3809 | cagtcacgaacggatagacatgg | 348694 | - | cell_cycle | |
| 20450 | ANKRD6 | NM_014942.1 | 1296 | ttgtcgatgtgaacctctaatca | 346242 | - | see also 6130 line | |
| 20451 | ANP32B | NM_006401.1 | 275 | ttgtcttgacaattgcaaatca | 335384 | - | | Alzheimer disease |
| 20452 | ANP32C | NM_012403.1 | 173 | cagacttaccaaagttaaagttg | 338134 | - | | |
| 20453 | APBB2 | NM_173075.1 | 1721 | aagtagtgttcggtcaacaact | 424483 | - | signal_transduction | protein binding |
| 20454 | APCDD1 | NM_153000.3 | 693 | cggtgcacaaatcccacttatac | 421773 | - | | |
| 20455 | APG10L | NM_031482.3 | 527 | cagcagcgtccgaagtgattaaa | 397182 | - | see also 9038 line | |
| 20456 | APG16L | NM_017974.2 | 1368 | tggacaatgcgcggattgtctca | 364949 | - | see also 7186 line | |
| 20457 | APG4B | NM_013325.3 | 369 | cagcgtcctcaacgcattcatcg | 338711 | - | see also 5399 line | |
| 20458 | APH-1A | NM_016022.1 | 181 | gggacccgcttcgcgttatcatc | 355900 | - | | |
| 20459 | APOBEC3D | NM_152426.1 | 238 | aggtgtatttccggtttgagaac | 416625 | - | | |
| 20460 | APOBEC3F | NM_145298.3 | 919 | aagcctatggtcggaacgaaagc | 414264 | - | | |
| 20461 | 3.Apr | NM_016085.2 | 408 | gaggaccctggtccaaactttca | 356248 | - | | |
| 20462 | APTX | NM_017692.2 | 842 | ccgagtatgagccatgttacatct | 360905 | - | | |
| 20463 | APXL2 | NM_133456.1 | 1085 | gagcgcttcaggcgaagtcttgg | 407219 | - | | |
| 20464 | AQR | NM_014691.1 | 790 | cagctggcacgattggaattaga | 342226 | - | see also 5891 line | |
| 20465 | ARC | NM_015193.2 | 826 | cgggcgtggcacgcacgcagatcttcg | 349548 | - | | |
| 20466 | ARG99 | NM_031920.2 | 813 | aagcgatctaccactacagaaca | 397322 | - | | |
| 20467 | ARH | NM_015627.1 | 296 | aggtgtcgccacgggggaattatc | 354623 | - | | hypercholesterolemia |
| 20468 | ARHGAP11A | NM_014783.2 | 1402 | tagtacagactcttatcgattat | 343419 | - | see also 5965 line | |
| 20469 | ARHGAP12 | NM_018287.4 | 1104 | cagggcgttgctattactataac | 369716 | - | see also 7363 line | |

Figure 46

EP 1 752 536 A1

| | | | | | | | | | |
|---|---|---|---|---|---|---|---|---|---|
| 20470 | ARHGAP15 | NM_018460.2 | 237 | gggaaggtcactgaacctatatc | 371822 | - | see also 7485 line | | |
| 20471 | ARHGAP18 | NM_033515.2 | 436 | aggccggtttatccaatctcttc | 404064 | - | - | - | - |
| 20472 | ARHGAP19 | NM_032900.2 | 803 | ctgtttcaacacgttcatgatat | 402487 | - | - | - | - |
| 20473 | ARHGAP9 | NM_032496.1 | 1086 | ctggacgctgcttctacataaat | 400411 | - | - | - | - |
| 20474 | ARHGEF15 | NM_173728.2 | 2033 | cagggagagctgactgagttagg | 427964 | - | - | - | Alzheimer disease |
| 20475 | ARHGEF16 | NM_014448.2 | 1037 | aagcgcggagagctgttcttagt | 340659 | - | - | - | - |
| 20476 | ARHGEF17 | NM_014786.2 | 1217 | acgacgacctatggtctagtagg | 343526 | - | - | - | - |
| 20477 | ARHGEF9 | NM_015185.1 | 1659 | acgcaagcgacgtttagagaata | 349460 | - | - | - | - |
| 20478 | ARLTS1 | NM_138450.3 | 404 | gggcaagaccacgctcctttaca | 408240 | - | - | - | - |
| 20479 | ARMC2 | NM_032131.3 | 526 | gtggaccctgcgaagattagaag | 397976 | - | - | - | - |
| 20480 | ARMET | NM_006010.1 | 369 | gggcgactgcgaagtttgtattt | 334573 | - | - | - | - |
| 20481 | ARP10 | NM_181773.2 | 187 | acgcccacgagaggctactttga | 433116 | - | - | - | - |
| 20482 | ARPC5L | NM_030978.1 | 499 | gaggactaggctccattataaga | 395922 | - | see also 8987 line | | |
| 20483 | ARV1 | NM_022786.1 | 154 | aagagttgtaccgagactataac | 384224 | - | - | - | - |
| 20484 | ASAH3 | NM_133492.1 | 162 | gcgctcccgctacatttacgttg | 407249 | - | - | - | - |
| 20485 | ASAM | NM_024769.1 | 491 | atggctgctcaccgataatgaag | 390326 | - | - | - | - |
| 20486 | ASB12 | NM_130388.2 | 104 | atgacaacgactcctatactttg | 406890 | - | see also 9544 line | | |
| 20487 | ASB7 | NM_024708.2 | 976 | ttgcacgcttgatgttagaatct | 389463 | - | - | - | - |
| 20488 | ASCL3 | NM_020646.1 | 467 | gagctgcgatcaagtacattaac | 377848 | - | - | DNA binding | - |
| 20489 | ASF1B | NM_018154.1 | 597 | gggtgacccgcttccatatcaac | 367777 | - | - | - | - |
| 20490 | ASMTL | NM_004192.1 | 1094 | tggcatcggatggcgaatactct | 332465 | - | - | - | - |
| 20491 | ASP | NM_031916.2 | 530 | aagcggtatgtggaattaacaga | 397284 | - | kinase_related、signal_transduction | | |
| 20492 | ASPM | NM_018136.2 | 3416 | cagacggccgtgtgttatgttac | 367296 | - | see also 7271 line | | |
| 20493 | ASTN2 | NM_014010.2 | 3323 | aggcatggtgtggtatagcatcc | 339050 | - | see also 5454 line | | |
| 20494 | ASXL2 | NM_018263.2 | 4264 | gagggcacgccttcgaaatgtta | 369508 | - | see also 7348 line | | |
| 20495 | ATCAY | NM_033064.1 | 764 | ccggggacagcgcggatctattt | 402761 | - | - | - | ataxia |
| 20496 | ATF7IP2 | NM_024997.2 | 632 | atgaggggcttgtagtctaaag | 392835 | - | - | - | - |
| 20497 | ATP9B | NM_198531.2 | 679 | gaggtaaagtgcaagttaagagt | 439247 | - | see also 10338 line | | |
| 20498 | ATPAF1 | NM_022745.1 | 852 | gtgcatcgccaaccaagttcagc | 383867 | - | - | - | - |
| 20499 | ATPAF2 | NM_145691.2 | 583 | gtggaggagcccgagacattagt | 414694 | - | - | - | - |
| 20500 | AUTL1 | NM_032852.2 | 389 | ctgcagagtcgggatgtacaata | 402034 | - | - | - | - |

Figure 46

| | Symbol | Accession | | Sequence | ID | | Note | Disease |
|---|---|---|---|---|---|---|---|---|
| 20501 | AUTL2 | NM_052936.2 | 272 | gtgctcgtctatggttacatac | 405416 | - | | |
| 20502 | AUTL4 | NM_032885.4 | 369 | aagccggaccagctttagccaaga | 402431 | - | | |
| 20503 | AYP1 | NM_032193.2 | 608 | aagtgctgsggccttaacttgg | 398642 | - | | |
| 20504 | AZ2 | NM_022461.1 | 650 | aaggcctatcatcgatatcgaga | 382958 | - | | |
| 20505 | B29 | NM_031939.2 | 542 | aagtcatccatgagagtatgaag | 397407 | - | see also 9059 line | |
| 20506 | B3GTL | NM_194318.1 | 625 | ttgaaatccgactttacaataga | 436810 | - | | |
| 20507 | B4GalNac-T3 | NM_173593.2 | 2015 | aggtcacgcggagtcttcttgaag | 426399 | - | | |
| 20508 | B7-H4 | NM_024626.1 | 717 | atgttacgatcaacaacacatac | 388231 | - | | |
| 20509 | BAALC | NM_024812.1 | 581 | tggacagaagtcgaagaatcaca | 390961 | - | | |
| 20510 | BAIAP3 | NM_003933.3 | 2602 | gtgggcgacatccgcaagtagt | 332289 | - | | Alzheimer disease |
| 20511 | BANP | NM_017869.2 | 1427 | cagcgacatccaggttcagtacg | 363320 | - | | |
| 20512 | BARHL2 | NM_020063.1 | 680 | aaggagaccggagattacgagt | 375570 | - | | |
| 20513 | BASE | NM_173859.1 | 415 | atgaagccctcacagctaatat | 428521 | - | | |
| 20514 | BAT2 | NM_004638.2 | 2274 | gggtcgtccccctagacttct | 332675 | - | see also 3150 line | |
| 20515 | BBC3 | NM_014417.2 | 782 | ctggagggtcctgtacaatctca | 340559 | - | | |
| 20516 | BBP | NM_032027.2 | 554 | cagcacccaacataactgtaag | 397646 | - | | |
| 20517 | BBS1 | NM_024649.4 | 1296 | ccgaaagaccccggcttacgtgg | 388514 | - | | diabetes |
| 20518 | BBS4 | NM_033028.2 | 48 | gagtcgcgacgagaactcaattt | 402618 | - | | diabetes |
| 20519 | BBS7 | NM_018190.2 | 287 | gagatcatgatgggtagttatg | 368240 | - | see also 7309 line | |
| 20520 | BC002942 | NM_033200.1 | 1525 | ctggacggagatcgagttcatgt | 403570 | - | | |
| 20521 | BC008967 | NM_033201.1 | 519 | gagagaggatgagctaatccaga | 403574 | - | see also 9378 line | |
| 20522 | BCAS1 | NM_003657.1 | 273 | aagtgtcaagacggataatgtgg | 331914 | - | see also 2477 line | |
| 20523 | BCL11A | NM_018014.2 | 550 | gaggatgacgattgtttatcaac | 365306 | - | see also 7199 line | |
| 20524 | BCL11B | NM_022898.1 | 2201 | cggcgtcgcggcacttcatgaag | 384705 | - | see also 8519 line | |
| 20525 | BCL9L | NM_182557.1 | 1485 | ggggccacccacaattgtaatgt | 434424 | - | | |
| 20526 | BCMP11 | NM_176813.3 | 508 | tcggatttaccctattgatag | 429708 | - | | |
| 20527 | BCNP1 | NM_173544.2 | 1386 | gcgcgtgcgcaggggaggttact | 425772 | - | | |
| 20528 | BCOR | NM_017745.4 | 2837 | cagtgacttccacgaaaacttata | 361530 | - | see also 7135 line | |
| 20529 | BGALT15 | NM_198540.1 | 1615 | gtggctaccagcctatgcaagc | 439313 | - | | |
| 20530 | BHD | NM_144606.3 | 777 | cggatatatcagccatgataaa | 410247 | - | see also 9715 line | |
| 20531 | BHLHB5 | NM_152414.2 | 965 | aggcgctgcggttaacatcaat | 416440 | - | | |
| 20532 | BIA2 | NM_015431.2 | 1055 | tcgggtttgcagagcttctcatca | 352752 | - | | |
| 20533 | BICC1 | NM_025044.2 | 1150 | gaggtcagagaacgcccacaatac | 393226 | - | | |
| 20534 | BITE | NM_024491.1 | 894 | tggatgcctcaccaacttataaa | 386388 | - | | |
| 20535 | BIVM | NM_017693.2 | 1021 | tagtacatcggaaatatctaca | 360923 | - | see also 7114 line | |
| 20536 | BLP1 | NM_031940.2 | 55 | aggtggttgcccgttagttact | 397417 | - | | |

Figure 46

| | | | | | | | | | |
|---|---|---|---|---|---|---|---|---|---|
| 20537 | BLP2 | NM_025141.2 | 608 | gggaatatggacgctgatagacg | 393971 | - | - | - | - |
| 20538 | BM039 | NM_018455.3 | 927 | gtgatgctgccctgttagacatc | 371783 | - | - | - | - |
| 20539 | BNF1 | NM_015424.3 | 1150 | ctgccgtcaccccgagaaagtgg | 352623 | - | - | - | - |
| 20540 | BNIPL | NM_138278.1 | 851 | aaggctgggccgaggttgtatgt | 407354 | - | - | - | - |
| 20541 | BOMB | NM_024949.3 | 1201 | cagataggactcagatacaatgc | 392576 | - | - | coreceptor | - |
| 20542 | BP75 | NM_012107.1 | 244 | tggaccctggagttcttatgtac | 337494 | - | - | - | - |
| 20543 | BRI3BP | NM_080626.5 | 715 | tggtggccgtctacttcatgacc | 406511 | - | - | - | - |
| 20544 | BRIX | NM_018321.2 | 790 | atgcatcggcgtgtcataagatc | 370321 | - | see also 7404 line | | |
| 20545 | BRMS1L | NM_032352.2 | 717 | ttgttgtttcaggtccatatata | 399901 | - | - | - | - |
| 20546 | BRRN1 | NM_015341.2 | 1419 | aggcctcgacgcaaacaagatgc | 351736 | - | cell_cycle | - | - |
| 20547 | BSND | NM_057176.2 | 759 | aggggaaagacgcctaactcaga | 406119 | - | - | - | Bartter syndrome |
| 20548 | BTLA | NM_181780.1 | 152 | gagatccctttgaactagaatgc | 433178 | - | - | - | - |
| 20549 | BTN2A2 | NM_006995.3 | 534 | gggtctaagcccctcattgaaat | 336710 | - | - | - | - |
| 20550 | BTN2A3 | NM_024018.1 | 434 | ctgagcccgtcattgaaatgaga | 385464 | - | - | - | - |
| 20551 | BTN3A3 | NM_006994.3 | 1751 | ctgacctagtgcctgatcattcc | 336704 | - | - | - | - |
| 20552 | BTNL2 | NM_019602.1 | 966 | cagactgaccctgcagatactca | 375443 | - | - | - | - |
| 20553 | BVES | NM_007073.2 | 467 | atgtaccggcgattgtttgaacc | 336905 | - | - | - | - |
| 20554 | BXMAS2-10 | NM_145016.2 | 446 | aagagcatccctgaatccataaa | 412648 | - | - | - | - |
| 20555 | C10orf12 | NM_015652.2 | 3209 | aagtcgtaagagcgtatgcatca | 354920 | - | - | - | - |
| 20556 | C10orf23 | NM_152643.3 | 1487 | gagaagcactcacggaagattca | 419457 | - | - | - | - |
| 20557 | C10orf24 | NM_152709.2 | 2041 | cagtcaaccactcacatctaatt | 419991 | - | - | - | - |
| 20558 | C10orf25 | NM_145022.1 | 248 | acgaagtcaccttaattatcacc | 412802 | - | - | - | - |
| 20559 | C10orf27 | NM_152710.1 | 1292 | aagagccaccctgcagtcaatcc | 419998 | - | - | - | - |
| 20560 | C10orf28 | NM_014472.2 | 2031 | ctgggagtctatgtttaacgatg | 340797 | - | - | - | - |
| 20561 | C10orf29 | NM_152586.2 | 2188 | atgagtcacactcatcactatct | 418273 | - | - | - | - |
| 20562 | C10orf3 | NM_018131.3 | 323 | gtggggatcgaagcctagtaact | 367099 | - | - | - | - |
| 20563 | C10orf30 | NM_152751.1 | 345 | cagcactgggcggatctatcagc | 420577 | - | - | - | - |
| 20564 | C10orf32 | NM_144591.1 | 219 | ttgcactcagaagatagtttaag | 410090 | - | - | - | - |
| 20565 | C10orf36 | NM_194298.1 | 1398 | atgcctatgggatattaatgttc | 436546 | - | - | - | - |
| 20566 | C10orf39 | NM_194303.1 | 230 | caggcactgtaccggagaaatca | 436675 | - | - | - | - |
| 20567 | C10orf4 | NM_145246.2 | 404 | tagctatggatgcttatcaaaga | 413836 | - | - | - | - |
| 20568 | C10orf42 | NM_138357.1 | 677 | aggtacgaattgagattagcaga | 407591 | - | - | - | - |
| 20569 | C10orf45 | NM_031453.2 | 760 | atggccgagccagactacataga | 397071 | - | - | - | - |
| 20570 | C10orf46 | NM_153810.2 | 1004 | cagcactcggaacagatgtatag | 423912 | - | - | - | - |
| 20571 | C10orf5 | NM_178816.2 | 151 | aaggtatcattatagtctttaga | 431374 | - | - | - | - |
| 20572 | C10orf6 | NM_018121.2 | 2130 | aaggccgattctaatgtatcttc | 366895 | - | see also 7265 line | | |

Figure 46

| 20573 | C10orf8 | NM_012071.1 | 131 | gcggacgaggccgtgttagatca | 337469 | - | see also 5109 line | | |
|---|---|---|---|---|---|---|---|---|---|
| 20574 | C11orf10 | NM_014206.1 | 188 | aagtacactcgtgatatctataa | 339970 | - | - | - | - |
| 20575 | C11orf16 | NM_020643.1 | 1379 | aggagctggtctcgaaagcaacc | 377840 | - | - | - | - |
| 20576 | C11orf23 | NM_018312.2 | 779 | aggctcctgacgtgtatcgaacc | 370109 | - | see also 7389 line | | |
| 20577 | C11orf24 | NM_022338.1 | 611 | cggcagcccacctcaactctatg | 382596 | - | - | - | - |
| 20578 | C11orf30 | NM_020193.2 | 2263 | gagactagccctaccataattta | 376006 | - | see also 7794 line | | |
| 20579 | C11orf33 | NM_017678.1 | 914 | aggatcaatgacctattcagtca | 360577 | - | see also 7108 line | | |
| 20580 | C11orf9 | NM_013279.1 | 76 | tggagccctccaacatagacacc | 338640 | - | - | - | - |
| 20581 | C12orf10 | NM_021640.1 | 613 | gagttgctcgacttaatcctacc | 381009 | - | - | - | - |
| 20582 | C12orf22 | NM_030809.1 | 1087 | ccgggtccggactcattacctcc | 395269 | - | - | - | - |
| 20583 | C12orf4 | NM_020374.2 | 385 | gagtacatgggccaaagcttatg | 377101 | - | see also 7854 line | | |
| 20584 | C13orf1 | NM_020456.1 | 344 | gtgttgcaactcagaaggttaac | 377665 | - | - | - | - |
| 20585 | C13orf12 | NM_015932.2 | 337 | caggttcagcgtcttccatttct | 355504 | - | - | - | - |
| 20586 | C13orf17 | NM_018185.1 | 330 | tggtcatagcgtggaagttcagg | 368156 | - | - | - | - |
| 20587 | C13orf18 | NM_025113.1 | 1348 | tggctcgatagtcgtaaatgaag | 393701 | - | - | - | - |
| 20588 | C13orf3 | NM_145061.2 | 834 | aagtgatgccgaatataccaact | 413384 | - | see also 9770 line | | |
| 20589 | C13orf7 | NM_024546.2 | 746 | cagtatgagcgtgaaaccaatcg | 387033 | - | see also 8637 line | | |
| 20590 | C13orf9 | NM_016075.1 | 922 | ttgacagtggcgtcatggtaatt | 356203 | - | see also 6652 line | | |
| 20591 | C14orf100 | NM_016475.2 | 529 | aagccgtctacccactatatacc | 357680 | - | see also 6872 line | | |
| 20592 | C14orf101 | NM_017799.2 | 966 | gaggaccgaactctcattcaaca | 362626 | - | - | - | - |
| 20593 | C14orf103 | NM_018036.4 | 1174 | ctgacgagagtgggaatgtatcc | 365624 | - | - | - | - |
| 20594 | C14orf104 | NM_018139.1 | 706 | atggtgtaaacaacgattctttg | 367564 | - | - | - | - |
| 20595 | C14orf105 | NM_018168.1 | 457 | ttgacttgccacgagtaattact | 367886 | - | - | - | - |
| 20596 | C14orf108 | NM_018229.2 | 1789 | aagtgcacaccggaaactaattt | 368768 | - | - | - | - |
| 20597 | C14orf11 | NM_018453.2 | 389 | aggtactacgatgatatatattt | 371775 | - | - | - | - |
| 20598 | C14orf111 | NM_015962.1 | 31 | gcgaccatgaagcgaatgcttag | 355638 | - | - | - | - |
| 20599 | C14orf112 | NM_016468.3 | 384 | cagaaagccttaagactaagaca | 357623 | - | - | - | - |
| 20600 | C14orf115 | NM_018228.1 | 2187 | ctgcccgctccacatactacatg | 368708 | - | - | - | - |
| 20601 | C14orf116 | NM_018589.2 | 304 | tagatctgcctacctttgtgaat | 372270 | - | - | - | - |
| 20602 | C14orf118 | NM_017926.2 | 990 | gggtaccatactcgcttgaatcg | 364255 | - | - | - | - |
| 20603 | C14orf119 | NM_017924.2 | 192 | aggtccccagcgtgaacgtttcc | 364054 | - | see also 7178 line | | |
| 20604 | C14orf129 | NM_016472.2 | 316 | cggatgatgtggcctatatcaat | 357638 | - | - | - | - |
| 20605 | C14orf132 | NM_020215.1 | 480 | aagcatcatctgccttcattatt | 376242 | - | - | - | - |
| 20606 | C14orf133 | NM_022067.2 | 1489 | atgatgtagatgccctattcacc | 382007 | - | see also 8260 line | | |
| 20607 | C14orf135 | NM_022495.2 | 1274 | ctgggatatccgatttattcttc | 383544 | - | - | - | - |
| 20608 | C14orf138 | NM_024558.1 | 329 | aagacttgctgaagatgaatatt | 387261 | - | - | - | - |
| 20609 | C14orf139 | NM_024633.2 | 565 | cagccgagagacagcaatcataa | 388340 | - | - | - | - |

Figure 46

| | | | | | | | |
|---|---|---|---|---|---|---|---|
| 20610 | C14orf140 | NM_024643.1 | 755 | tgggtgcgagatccgaagactaga | 388472 | - | |
| 20611 | C14orf141 | NM_032035.1 | 822 | ctgccaaccttgagatatgtat | 397673 | - | |
| 20612 | C14orf142 | NM_032490.2 | 392 | tggctgtttgtatccttattgtca | 400386 | - | |
| 20613 | C14orf147 | NM_138288.1 | 218 | atggcgatattgcactactttga | 407450 | - | see also 9592 line |
| 20614 | C14orf160 | NM_024884.1 | 616 | tagggggtctaatggctattgatt | 391666 | - | |
| 20615 | C14orf161 | NM_024764.1 | 1879 | cggtagtgctaggcggattaatt | 390276 | - | see also 8741 line |
| 20616 | C14orf162 | NM_020181.1 | 346 | tagacagtcatctgcagataatg | 375834 | - | |
| 20617 | C14orf166 | NM_016039.1 | 272 | caggcactacaagattgaagaca | 356075 | - | |
| 20618 | C14orf168 | NM_031427.1 | 181 | ttgctaattgcgagaagcttca | 396939 | - | |
| 20619 | C14orf169 | NM_024644.1 | 1918 | ttggcaaccacgttgtatgataa | 388499 | - | see also 8676 line |
| 20620 | C14orf24 | NM_173607.2 | 445 | gggtatcagcaccccaaagtacc | 426487 | - | |
| 20621 | C14orf29 | NM_181533.3 | 634 | gtggttgcaagtatcaattatc | 432396 | - | |
| 20622 | C14orf32 | NM_144578.2 | 230 | aggctccaaccttggaataatc | 409911 | - | |
| 20623 | C14orf34 | NM_017949.1 | 1499 | gagagatcgattgaaatacgaat | 364661 | - | |
| 20624 | C14orf39 | NM_174978.1 | 1711 | cagacacttcaactcatacattt | 428884 | - | see also 10136 line |
| 20625 | C14orf45 | NM_025057.1 | 93 | ctgcagcaacacgcaatgatgaga | 393345 | - | |
| 20626 | C14orf48 | NM_152777.2 | 761 | gggctccatagcctcaagatcc | 421069 | - | |
| 20627 | C14orf49 | NM_152592.2 | 2382 | tcgtcgacgcgcaaatcttctac | 418518 | - | |
| 20628 | C14orf50 | NM_172365.1 | 886 | gggagactcttcgtaccaatat | 424427 | - | |
| 20629 | C14orf54 | NM_173526.1 | 686 | gagtggtcgagcctactacttac | 425485 | - | |
| 20630 | C14orf58 | NM_017791.1 | 1490 | tggtggtgtacacgtttaccttg | 362435 | - | see also 7154 line |
| 20631 | C14orf59 | NM_174976.1 | 389 | gggcaattacgtccttgtcatcc | 428825 | - | |
| 20632 | C14orf8 | NM_173846.3 | 557 | gggttctggcacctacgataaga | 428480 | - | |
| 20633 | C14orf9 | NM_144568.1 | 377 | cagtgggagtgccectatgatca | 409750 | - | |
| 20634 | C14orf93 | NM_021944.1 | 1314 | aggcgcaagcgggatcttgtact | 381516 | - | see also 8207 line |
| 20635 | C14orf94 | NM_017815.1 | 1023 | gtgcggtgtatgatccttaagc | 362681 | - | |
| 20636 | C15orf16 | NM_130901.1 | 1038 | aagaaggcccatcgttgttgtg | 407142 | - | |
| 20637 | C15orf5 | NM_030944.1 | 448 | aaggacctagaaggcaaagacatta | 395651 | - | |
| 20638 | C16orf34 | NM_144570.1 | 240 | gtggtatcttgacgaatcaacc | 409767 | - | |
| 20639 | C17orf28 | NM_030630.1 | 1238 | gagcagcgacgtcctagacatcc | 394726 | - | |
| 20640 | C17orf31 | NM_017575.3 | 817 | ctggcctgacggataatggatgt | 359243 | - | |
| 20641 | C19orf10 | NM_019107.1 | 218 | gggacaaatacgtgtatgttc | 375235 | - | |
| 20642 | C19orf6 | NM_033420.2 | 1271 | gtggctcgcggaccagtatgacg | 403876 | - | |
| 20643 | C1orf14 | NM_030933.1 | 1223 | acgtgtcgagctcattgagtatc | 395556 | - | |
| 20644 | C1orf16 | NM_014837.2 | 190 | atgctagtttaccgatttggaata | 344382 | - | |
| 20645 | C1orf19 | NM_052965.1 | 170 | gggcactcacctaagtatctag | 405566 | - | |
| 20646 | C1orf21 | NM_030806.2 | 700 | gaggttccggggattagttcatca | 395221 | - | see also 8952 line |

Figure 46

| | | | | | | | | | |
|---|---|---|---|---|---|---|---|---|---|
| 20647 | C1orf26 | NM_017673.4 | 2845 | aagacatgctcaactataggata | 360569 | - | see also 7104 line | | |
| 20648 | C1orf27 | NM_017847.2 | 891 | atgtagcggttctgtaaacctta | 363050 | - | see also 7161 line | | |
| 20649 | C1orf28 | NM_024529.3 | 474 | gcgtcaacatcggcaagtataga | 386724 | - | see also 8627 line | | |
| 20650 | C1orf29 | NM_006820.1 | 807 | aagccgtagtggggtctgatacc | 336388 | - | - | - | - |
| 20651 | C1orf35 | NM_024319.2 | 340 | tggctacaagaacgtgaagaagc | 386251 | - | - | - | - |
| 20652 | C1orf36 | NM_183059.1 | 1423 | aagatccacccatcctattgtgg | 435578 | - | - | - | - |
| 20653 | C1orf37 | NM_138391.3 | 670 | gcgtctgcgaccagagtcaatgg | 407937 | - | see also 9609 line | | |
| 20654 | C1orf9 | NM_014283.2 | 3307 | atgcagcgttgtcgaaatacttc | 340107 | - | see also 5590 line | | |
| 20655 | C1QDC1 | NM_023925.2 | 2725 | aggtagccccgtagcatctaaag | 385361 | - | - | - | - |
| 20656 | C1QTNF1 | NM_030968.2 | 600 | ccgccgtgccccagatcaacatc | 395895 | - | - | - | - |
| 20657 | C1QTNF2 | NM_031908.3 | 687 | cggccgtgcctgggatctactact | 397250 | - | - | - | - |
| 20658 | C1QTNF5 | NM_015645.1 | 607 | acgccgtcaccggcaagttcacc | 354858 | - | - | - | - |
| 20659 | C1QTNF6 | NM_031910.3 | 690 | acgtacgtgcacattatgcataa | 397260 | - | - | - | - |
| 20660 | C1QTNF7 | NM_031911.3 | 866 | atgggcaataccggataaagacc | 397270 | - | see also 9051 line | | |
| 20661 | C20orf108 | NM_080821.1 | 554 | gtgcccttgattgtcagatattt | 406785 | - | - | - | - |
| 20662 | C20orf110 | NM_021202.1 | 974 | gtgccagcgccagttcaactact | 380753 | - | see also 8137 line | | |
| 20663 | C20orf112 | NM_080616.1 | 912 | gcggatgcgtctagagatctacc | 406449 | - | - | - | - |
| 20664 | C20orf116 | NM_023935.1 | 788 | gggactataacaggtgtgattga | 385438 | - | - | - | - |
| 20665 | C20orf12 | NM_018152.2 | 1920 | aagacaatcgacccgtcataacc | 367767 | - | - | - | - |
| 20666 | C20orf126 | NM_030815.2 | 292 | tggtttgcttcgggaacatgttt | 395289 | - | - | - | - |
| 20667 | C20orf128 | NM_178468.1 | 1280 | ctgccctcaaccataatagtacc | 430822 | - | - | - | - |
| 20668 | C20orf129 | NM_030919.1 | 788 | atgacagttcggactatcacagg | 395444 | - | - | - | - |
| 20669 | C20orf132 | NM_152503.1 | 1325 | ctgccgctaaatgcttctacaac | 417267 | - | - | - | - |
| 20670 | C20orf133 | NM_080676.2 | 164 | gagagacgcaaagaataacctaag | 406666 | - | - | - | - |
| 20671 | C20orf140 | NM_144628.1 | 795 | aggcacgtcgtgcggttatatga | 410537 | - | see also 9720 line | | |
| 20672 | C20orf141 | NM_080739.1 | 339 | ggggcactaggactgacaatcc | 406680 | - | - | - | - |
| 20673 | C20orf152 | NM_080834.1 | 567 | tggtcgcaggcgtgtgatcatca | 406830 | - | - | - | - |
| 20674 | C20orf158 | NM_152302.1 | 1407 | gagaatatcgcttctaacgatgg | 415434 | - | - | - | - |
| 20675 | C20orf160 | NM_080625.2 | 120 | ctgcactcgatgcccctttatcc | 406500 | - | - | - | - |
| 20676 | C20orf172 | NM_024918.2 | 634 | cagagctcagccggtctatcagt | 392139 | - | - | - | - |
| 20677 | C20orf173 | NM_080828.1 | 573 | tggcaacgacatcgaccaatacc | 406799 | - | - | - | - |
| 20678 | C20orf175 | NM_080829.1 | 1255 | cagtggaacccgtttgatactga | 406809 | - | - | - | - |
| 20679 | C20orf185 | NM_182658.1 | 1259 | acggatcgcgtttagaagaatgg | 434867 | - | - | - | - |
| 20680 | C20orf186 | NM_182519.1 | 181 | gggggcaaataccgatatggtga | 434074 | - | - | - | - |
| 20681 | C20orf19 | NM_018474.2 | 1549 | tggccgtaggtcagctattcaca | 371993 | - | - | - | - |
| 20682 | C20orf20 | NM_018270.3 | 306 | ctgcatgagtctgagattcttcc | 369603 | - | - | - | - |
| 20683 | C20orf21 | NM_017798.2 | 528 | aggggaccctccgattccatacc | 362590 | - | see also 7155 line | | |

Figure 46

| 20684 | C20orf24 | NM_018840.1 | 419 | aggagtcctgtacctctacttca | 372965 | - | - | - | - |
|---|---|---|---|---|---|---|---|---|---|
| 20685 | C20orf28 | NM_015417.2 | 275 | atggtggagatgcacaattatgt | 352362 | - | - | - | - |
| 20686 | C20orf29 | NM_018347.1 | 394 | aggcaggtagagtcaatgtgtcg | 370561 | - | - | - | - |
| 20687 | C20orf32 | NM_020356.1 | 2221 | gagaggaaaccccgcttatctga | 376891 | - | signal_transduction | - | - |
| 20688 | C20orf38 | NM_018327.1 | 236 | aggaagcacccccttcatgttatg | 370413 | - | - | - | - |
| 20689 | C20orf4 | NM_015511.2 | 519 | aggccctcgtatgggtttcttcc | 353648 | - | - | - | - |
| 20690 | C20orf40 | NM_144703.1 | 679 | ctgctgacaacctgaatgctaaa | 411496 | - | see also 9731 line | | |
| 20691 | C20orf43 | NM_016407.1 | 73 | gggttgcgacgggggaacaatcc | 357394 | - | - | - | - |
| 20692 | C20orf44 | NM_018244.2 | 684 | aagtacatgtgtcgtatcatagt | 368962 | - | - | - | - |
| 20693 | C20orf45 | NM_016045.1 | 548 | aaggccgagaagcaatggaatgg | 356096 | - | - | - | - |
| 20694 | C20orf55 | NM_031424.3 | 835 | acgggccagctcggatatcgtgt | 396926 | - | - | - | - |
| 20695 | C20orf6 | NM_016649.3 | 1149 | acgtcgattagcagtttgtaaca | 358727 | - | - | - | - |
| 20696 | C20orf65 | NM_176791.2 | 454 | ccgagaaccctctgaaagtcagt | 429611 | - | - | - | - |
| 20697 | C20orf67 | NM_022104.2 | 753 | gggtacgtcccctgaagataaac | 382308 | - | - | - | - |
| 20698 | C20orf7 | NM_024120.2 | 251 | tcggatcgcagaccgtgtatatg | 386112 | - | see also 8603 line | | |
| 20699 | C20orf71 | NM_178466.1 | 561 | gagaccgtccttcgataacaaca | 430809 | - | - | - | - |
| 20700 | C20orf72 | NM_052865.2 | 319 | tggacccgtgagcaagcataagc | 404516 | - | - | - | - |
| 20701 | C20orf81 | NM_022760.3 | 1710 | cggggggatgccacgctatgttcc | 383991 | - | see also 8455 line | | |
| 20702 | C20orf86 | NM_080674.1 | 631 | agggcgtttgtggcgttgtatgt | 406662 | - | - | - | - |
| 20703 | C20orf9 | NM_016004.1 | 1319 | atgacatcgatacaagtgaaaca | 355815 | - | see also 6603 line | | |
| 20704 | C20orf92 | NM_053041.1 | 449 | caggtccaggcggatttcataac | 405822 | - | - | - | - |
| 20705 | C21orf100 | NM_145033.1 | 91 | tggtgtctcccaagaagtcatat | 412983 | - | - | - | - |
| 20706 | C21orf121 | NM_198078.1 | 339 | ctgtgtcccaaacgagtttgtgc | 436985 | - | - | - | - |
| 20707 | C21orf125 | NM_194309.1 | 369 | acggctttgacaagaatcgattt | 436684 | - | - | - | - |
| 20708 | C21orf128 | NM_152507.1 | 249 | gagacacgatctgcattctcagc | 417313 | - | - | - | - |
| 20709 | C21orf129 | NM_152506.1 | 544 | gggacccagggttcgtttgtttg | 417310 | - | - | - | - |
| 20710 | C21orf18 | NM_017438.1 | 885 | ctgttacggccctcacgataatc | 358842 | - | - | - | - |
| 20711 | C21orf42 | NM_058184.1 | 474 | ttggaactttaccaaggattaag | 406209 | - | - | - | - |
| 20712 | C21orf56 | NM_032261.3 | 364 | gaggagctactacctcaatgaga | 399103 | - | - | molecular_function unknown | - |
| 20713 | C21orf69 | NM_058189.1 | 432 | gtgggttcctaggcatgaagacc | 406216 | - | - | - | - |
| 20714 | C21orf7 | NM_020152.2 | 596 | ctgaagccaaagtctattactgt | 375727 | - | - | - | - |
| 20715 | C21orf70 | NM_058190.1 | 155 | aggactgggcgttcatcaacacc | 406217 | - | - | - | - |
| 20716 | C21orf77 | NM_018277.1 | 221 | acgacctctgcaggaacctatct | 369638 | - | - | - | - |
| 20717 | C21orf80 | NM_015227.2 | 1106 | cggaggcgttgcgattattgacc | 350122 | - | - | - | - |
| 20718 | C21orf84 | NM_153752.1 | 270 | aagcccgatgggaaagaatgtct | 423881 | - | - | - | - |
| 20719 | C21orf86 | NM_153454.1 | 685 | ccgcagagagcggatgtttgtga | 423283 | - | - | - | - |

Figure 46

| | | | | | | | | | |
|---|---|---|---|---|---|---|---|---|---|
| 20720 | C21orf88 | NM_153754.1 | 365 | gtgtggggttgtaggaatgaagt | 423887 | - | - | - | - |
| 20721 | C21orf90 | NM_153204.1 | 749 | cagctcattcgttagatgattgg | 422098 | - | - | - | - |
| 20722 | C22orf18 | NM_024053.2 | 390 | aagctggcccacacctatcaaag | 385653 | - | - | - | - |
| 20723 | C22orf19 | NM_003678.2 | 1337 | ctgactttgagcgactatgtact | 331990 | - | - | - | - |
| 20724 | C22orf4 | NM_014346.1 | 1082 | gtggatacgttcagggtataaat | 340253 | - | - | - | - |
| 20725 | C2orf11 | NM_144629.1 | 700 | gtggtgctattgcgcttgaaatt | 410555 | - | - | - | - |
| 20726 | C2orf3 | NM_003203.2 | 2097 | ctgctaaatcgttaccttattat | 331294 | - | transcription_regulator | RNA polymerase II transcription factor | - |
| 20727 | C2orf5 | NM_152516.1 | 109 | ctgctacggagccagctatatcc | 417450 | - | - | - | - |
| 20728 | C2orf6 | NM_018221.1 | 246 | aggatctcatcagtatgaactct | 368566 | - | - | - | - |
| 20729 | C2orf9 | NM_032309.2 | 282 | gcgggactgtcactaccttaaga | 399642 | - | - | - | - |
| 20730 | C3F | NM_005768.4 | 175 | ctggctggatactattacactgc | 334224 | - | - | - | - |
| 20731 | C3orf6 | NM_174908.2 | 1003 | gtgcttatgcagatagttactat | 428608 | - | - | - | - |
| 20732 | C4.4A | NM_014400.1 | 452 | gcgctcgacccggcaggtaatga | 340532 | - | - | - | - |
| 20733 | C4orf7 | NM_152997.1 | 143 | gtgacagcgatgaattagcttca | 421760 | - | - | - | - |
| 20734 | C4orf8 | NM_003704.1 | 2959 | aagcgagcaaggcataagcaaag | 332032 | - | - | - | - |
| 20735 | C5orf7 | NM_016604.2 | 4321 | atgtgctagatccccatacttct | 358326 | - | see also 6947 line | | |
| 20736 | C6orf10 | NM_006781.2 | 504 | ctgtctcgatcaagtattggtag | 336330 | - | - | - | - |
| 20737 | C6orf102 | NM_145027.2 | 222 | cagcgttaccatcgatgacaaca | 412860 | - | - | - | - |
| 20738 | C6orf105 | NM_032744.1 | 119 | ctggtatactttcctcaattatt | 401308 | - | - | - | - |
| 20739 | C6orf107 | NM_017754.2 | 1313 | ccgcaagatggcgtttgactatt | 361724 | - | - | - | - |
| 20740 | C6orf109 | NM_015388.1 | 248 | aggctctagcttcgaggatatgg | 352176 | - | - | - | - |
| 20741 | C6orf111 | NM_032870.1 | 2034 | gagaggagtcgaagtatagataa | 402372 | - | - | - | - |
| 20742 | C6orf113 | NM_145062.1 | 1712 | aaggtcacagtcgaactgttatt | 413456 | - | see also 9771 line | | |
| 20743 | C6orf117 | NM_138409.1 | 191 | atgtccgcccagagagttaatttc | 407968 | - | - | - | - |
| 20744 | C6orf118 | NM_144980.1 | 234 | cagcctccggagacgatcttaca | 412270 | - | - | - | - |
| 20745 | C6orf125 | NM_032340.1 | 220 | tagcgcgactccattcaaactac | 399819 | - | - | - | - |
| 20746 | C6orf128 | NM_145316.2 | 111 | tggccgtagacatgtatctcatc | 414412 | - | - | - | - |
| 20747 | C6orf130 | NM_145063.2 | 393 | atgggcgatatatatattacttg | 413466 | - | - | - | - |
| 20748 | C6orf134 | NM_024909.1 | 409 | tggccatgggcgagaactcttcc | 391966 | - | see also 8803 line | | |
| 20749 | C6orf136 | NM_145029.1 | 1157 | ttgtcgccatcgtctagataaac | 412887 | - | - | - | - |
| 20750 | C6orf141 | NM_153344.1 | 824 | gggacttggctcgaggaaattaa | 422844 | - | - | - | - |
| 20751 | C6orf142 | NM_138569.1 | 161 | aagactaccaacccatactcagt | 408450 | - | - | - | - |
| 20752 | C6orf146 | NM_173563.1 | 1110 | gtgctctagacttgcacaattta | 426153 | - | - | - | - |
| 20753 | C6orf148 | NM_030568.2 | 686 | gggagccaggactcctatcatca | 394629 | - | - | - | - |
| 20754 | C6orf150 | NM_138441.1 | 720 | ctgaacaccgggagctactatga | 408194 | - | - | - | - |
| 20755 | C6orf151 | NM_152551.2 | 776 | aggtgattcgagatgtgataaat | 418022 | - | - | - | - |

Figure 46

| 20756 | C6orf152 | NM_181714.1 | 1438 | aaggaggtacagcgactatatca | 432933 | - | - | | - | - |
| 20757 | C6orf153 | NM_033112.1 | 195 | gtggggactaagacgtacaaaca | 403353 | - | - | | - | - |
| 20758 | C6orf155 | NM_024882.1 | 566 | gcggtcatcgcgggtgactttct | 391646 | - | - | | - | - |
| 20759 | C6orf162 | NM_020425.3 | 114 | ggggtgcgcacaacaaccttatt | 377415 | - | - | | - | - |
| 20760 | C6orf165 | NM_178823.2 | 277 | ttgtatgactcggctattggata | 431670 | - | - | | - | - |
| 20761 | C6orf166 | NM_018064.2 | 1074 | aagtttacgcatgatcaaataat | 366036 | - | - | | - | - |
| 20762 | C6orf167 | NM_198468.1 | 416 | cggagcccttaagcgattgattt | 438281 | - | - | | - | - |
| 20763 | C6orf168 | NM_032511.1 | 1201 | aagacccgaacggctgatcaaag | 400500 | - | see also 9229 line | | | |
| 20764 | C6orf170 | NM_152730.2 | 626 | gtgtgtcgtcacatatatagtac | 420193 | - | - | | - | - |
| 20765 | C6orf18 | NM_019052.2 | 1157 | gtgaccctggttgagaatctaag | 374772 | - | - | | - | - |
| 20766 | C6orf182 | NM_173830.3 | 1418 | aagacagctaccctaaaggatca | 428444 | - | - | | - | - |
| 20767 | C6orf188 | NM_153711.1 | 681 | ctgccgatctaaagttagctacc | 423837 | - | see also 10016 line | | | |
| 20768 | C6orf192 | NM_052831.1 | 210 | tcggcagcttcggtgaacttagg | 404270 | - | - | | - | - |
| 20769 | C6orf195 | NM_152554.1 | 879 | cagagggagattagcaatgaagc | 418072 | - | - | | - | - |
| 20770 | C6orf203 | NM_016487.2 | 213 | aagttacgtgcaccaaattataa | 357818 | - | - | | - | - |
| 20771 | C6orf206 | NM_152732.2 | 132 | aagcgcgactaccgctatgatcg | 420245 | - | - | | - | - |
| 20772 | C6orf210 | NM_020381.2 | 755 | ttgtacatcgtgggatagtaaat | 377326 | - | - | | - | - |
| 20773 | C6orf211 | NM_024573.1 | 1298 | caggttgcacctgacttatatgc | 387406 | - | - | | - | - |
| 20774 | C6orf25 | NM_025260.2 | 368 | tggggacaggacctattgcaag | 394621 | - | - | | - | - |
| 20775 | C6orf27 | NM_025258.1 | 1361 | gagctcggcgtgagatcttgtcc | 394615 | - | - | | - | - |
| 20776 | C6orf29 | NM_025257.1 | 609 | ggggatcagcggtcttattgaca | 394585 | - | - | | - | - |
| 20777 | C6orf32 | NM_015864.2 | 839 | aagtaaggtagatgaactctatg | 355269 | - | see also 6546 line | | | |
| 20778 | C6orf37 | NM_017633.1 | 1168 | aggggggaggcctgcttaagtact | 359787 | - | - | molecular_function unknown | Alzheimer disease |
| 20779 | C6orf55 | NM_016485.3 | 710 | caggtgtagcaagtaatactatc | 357798 | - | see also 6879 line | | | |
| 20780 | C6orf56 | NM_014721.1 | 1767 | ctgcgatttaacgagtatgtaga | 342868 | - | - | | - | - |
| 20781 | C6orf60 | NM_024581.3 | 2851 | ttgttacggcataatcatcatca | 387522 | - | - | | - | - |
| 20782 | C6orf62 | NM_030939.2 | 809 | cagctccatgctcctcgatatca | 395634 | - | see also 8972 line | | | |
| 20783 | C6orf64 | NM_018322.1 | 447 | gagcgcctactctgtgttcaatc | 370330 | - | - | | - | - |
| 20784 | C6orf65 | NM_152731.1 | 535 | cagccgacttcgacagtctttgg | 420235 | - | - | | - | - |
| 20785 | C6orf69 | NM_173562.3 | 894 | aaggcatcagtgcaactgtattt | 426107 | - | channel | | - | - |
| 20786 | C6orf74 | NM_016063.1 | 340 | gagtacgagacccaatctagtgc | 356173 | - | - | | - | - |
| 20787 | C6orf75 | NM_021820.1 | 122 | gagttccgcctgccggaaataaa | 381041 | - | - | | - | - |
| 20788 | C6orf76 | NM_024807.1 | 1073 | atgtgcagcgatccttctacacg | 390835 | - | - | | - | - |
| 20789 | C6orf78 | NM_153036.1 | 429 | cgggagtgaagacgtcaaacaca | 422016 | - | - | | - | - |
| 20790 | C6orf81 | NM_145028.3 | 413 | ctgaggcctctgcttgtactacg | 412873 | - | - | | - | - |
| 20791 | C6orf82 | NM_015921.1 | 619 | aagtggccgaggtaattgcattg | 355492 | - | see also 6571 line | | | |

Figure 46

| | | | | | | | |
|---|---|---|---|---|---|---|---|
| 20792 | Cborf83 | NM_145169.1 | 470 | gggatgcagttattaaatgctgt | 413549 | - | - |
| 20793 | Cborf84 | NM_014895.1 | 1901 | aggggtgcgcaagccctaattga | 345330 | - | - |
| 20794 | Cborf89 | NM_152734.1 | 1015 | gagcggttgttcccatttcctta | 420264 | - | - |
| 20795 | Cborf93 | NM_032860.3 | 1221 | aagcccaaacaaattcgaatatc | 402156 | - | see also 9322 line |
| 20796 | Cborf96 | NM_017909.1 | 192 | aagaatcggtcatctaatgttaa | 363843 | - | see also 7171 line |
| 20797 | Cborf97 | NM_025059.2 | 123 | cggtcacgcgggagcagttaaac | 393362 | - | - |
| 20798 | C7orf11 | NM_138701.1 | 511 | cagtatctgtagtgatataagc | 408606 | - | - |
| 20799 | C7orf13 | NM_032625.1 | 797 | gggttgtggcctggatactctgg | 401147 | - | - |
| 20800 | C7orf19 | NM_032831.1 | 156 | cggccataaggggcatggattacc | 401806 | - | - |
| 20801 | C7orf2 | NM_022458.2 | 206 | gtgcgggagtccacgatatgttt | 382906 | - | - |
| 20802 | C7orf20 | NM_015949.2 | 451 | ctgagcgcgtgacctttgtgtcc | 355597 | - | - |
| 20803 | C7orf22 | NM_031443.2 | 113 | ctggaattgtctcgccatttaaa | 397026 | - | - |
| 20804 | C7orf23 | NM_024315.2 | 453 | tggtatcgacaaggagactaga | 386241 | - | - |
| 20805 | C7orf24 | NM_024051.2 | 433 | tggaatgtatgttgtaatagaag | 385630 | - | - |
| 20806 | C7orf25 | NM_024054.1 | 1148 | ctgtgttaattaagcgaattaat | 385675 | - | - |
| 20807 | C7orf26 | NM_024067.2 | 476 | tggtgccggatccattcagacg | 385718 | - | see also 8581 line |
| 20808 | C7orf27 | NM_152743.1 | 482 | gtgcggtcgacaccatcttctcc | 420309 | - | - |
| 20809 | C7orf28B | NM_198097.1 | 510 | aggcggtcaagcttctgaaagaaa | 437026 | - | - |
| 20810 | C7orf31 | NM_138811.3 | 1557 | aggacgaattgcccgattaattc | 409167 | - | see also 9641 line |
| 20811 | C7orf33 | NM_145304.2 | 517 | gggcggtccaggtcaatttaact | 414270 | - | - |
| 20812 | C7orf34 | NM_178829.2 | 442 | gaggcggacattcccaaatatcc | 431978 | - | - |
| 20813 | C7orf35 | NM_032936.2 | 397 | atgctggcggtgtaagtctgg | 402559 | - | see also 9346 line |
| 20814 | C8orf14 | NM_054029.1 | 1564 | gagcccgagatcggttcactttg | 406010 | - | - |
| 20815 | C8orf15 | NM_175076.1 | 1087 | tagagattgcttgggatttgatg | 429006 | - | - |
| 20816 | C8orf17 | NM_020237.1 | 1252 | ccgtcataagtacaagatgttgg | 376406 | - | - |
| 20817 | C9orf10 | NM_014612.2 | 1428 | gcggaggggcgacaaaccatatc | 341108 | - | - |
| 20818 | C9orf23 | NM_148178.1 | 227 | cagtgctcggcatgtagtgttct | 415121 | - | - |
| 20819 | C9orf24 | NM_032596.3 | 952 | atgcagtgtgcttgttacaactcc | 400990 | - | - |
| 20820 | C9orf25 | NM_147202.1 | 689 | aaggacggctaccggttagatga | 415118 | - | - |
| 20821 | C9orf26 | NM_033439.1 | 536 | aggttgacggtgttgatggttaaga | 404018 | - | - |
| 20822 | C9orf34 | NM_017948.3 | 1903 | aaggccgaattggccaaactatat | 364611 | - | - |
| 20823 | C9orf37 | NM_032937.2 | 837 | caggatgcccctttgagaaatgc | 402570 | - | - |
| 20824 | C9orf41 | NM_152420.1 | 690 | ctgttaccagccaatcattaaag | 416517 | - | - |
| 20825 | C9orf45 | NM_030814.3 | 644 | atgttactgggagccaaattgt | 395283 | - | - |
| 20826 | C9orf46 | NM_018465.1 | 447 | tggtcccgattgttccattaagc | 371892 | - | see also 7490 line |
| 20827 | C9orf48 | NM_194313.1 | 1323 | caggtagatttcatatatagaca | 436711 | - | - |
| 20828 | C9orf54 | NM_032809.2 | 544 | ctgagttggcatctcttccattga | 401598 | - | - |

Figure 46

| ID | Gene | Accession | Length | Sequence | Number | | Note | | Description |
|---|---|---|---|---|---|---|---|---|---|
| 20829 | C9orf7 | NM_017586.1 | 237 | ccggcgtgtggatgatcatgaat | 359387 | - | | - | |
| 20830 | CAB2 | NM_033419.2 | 205 | gggacgactgtaagtatgagtgt | 403862 | - | | - | |
| 20831 | CACNB3 | NM_000725.2 | 874 | aaggcgatctgtgctcaacaatcc | 329864 | | channel | - | voltage-gated calcium channel |
| 20832 | CaMKIINalpha | NM_018584.4 | 556 | gagcaagcgggttgttattgaag | 372264 | - | kinase_related | - | |
| 20833 | CAMTA2 | NM_015099.2 | 2660 | ccgtcacgtcagcctattctagt | 348025 | - | see also 6236 line | - | |
| 20834 | CANP | NM_198947.1 | 1301 | tgggtaggcggtatgctattaat | 440177 | - | | - | |
| 20835 | CAP350 | NM_014810.2 | 7102 | aagacctagttggattagctatt | 344091 | - | | - | Alzheimer disease |
| 20836 | CAPN12 | NM_144691.2 | 554 | gtgctgagccgaagttcatctgt | 411329 | - | | - | |
| 20837 | CASKIN1 | NM_020764.1 | 816 | ctgctggatagcgggatcaatgc | 378602 | - | | - | |
| 20838 | CASKIN2 | NM_020753.1 | 1179 | gtgaacatccggaatacgtataa | 378541 | - | | - | |
| 20839 | CASP8AP2 | NM_012115.2 | 3534 | atggcatagttgatcgtttattt | 337612 | - | | - | |
| 20840 | CAST | NM_015576.1 | 3062 | gggaggtcgcaacattccaatca | 354236 | - | see also 6496 line | - | |
| 20841 | CBLL1 | NM_024814.1 | 1475 | aagatatagaccgtattaccaat | 391041 | - | see also 8757 line | - | |
| 20842 | CBLN2 | NM_182511.2 | 913 | tcgaccaggtattagtaaatatt | 434039 | - | | - | |
| 20843 | CBLNL1 | NM_080617.3 | 491 | gtgtcctgctcacctagataaa | 406457 | - | see also 9516 line | - | |
| 20844 | CCNDBP1 | NM_012142.2 | 1061 | ctgaccgtgcgaatcaattctgc | 337706 | - | | - | |
| 20845 | CD1E | NM_030893.1 | 183 | gggcaccatccgctttctgaagc | 395356 | - | | - | defense/immunity protein |
| 20846 | CDA017 | NM_032024.2 | 741 | ggggaagtgtcgctacgtttact | 397621 | - | | - | |
| 20847 | CDA11 | NM_032026.1 | 28 | gagtcgttcaagtttatcgata | 397624 | - | see also 9062 line | - | |
| 20848 | CDAN1 | NM_138477.1 | 1424 | ctgacaatcgtgccaacttctc | 408371 | - | | - | |
| 20849 | CDCA4 | NM_017955.2 | 641 | aagtcacttgatcagatatttga | 364861 | - | | - | |
| 20850 | CDCA5 | NM_080668.2 | 235 | cagaaagcccatcgtcttaaaga | 406615 | - | | - | |
| 20851 | CDCA7 | NM_031942.3 | 852 | gagaccgcgaaggcgtacattcc | 397446 | - | | - | |
| 20852 | CDCA8 | NM_018101.1 | 600 | aggtcaagccgtgctaaacactgt | 366712 | - | | - | |
| 20853 | CDCP1 | NM_022842.3 | 968 | aggagcgggttgaatactacatc | 384653 | - | | - | |
| 20854 | CDK5RAP1 | NM_016082.3 | 325 | gagatagcctgtccatcttaca | 356220 | - | see also 6653 line | - | |
| 20855 | CDK5RAP2 | NM_018249.3 | 625 | aggttggaagatctcctaactaaa | 368985 | - | kinase_related | - | |
| 20856 | CDT1 | NM_030928.2 | 1502 | tgggcagctgttgtactatcatg | 395533 | - | | - | |
| 20857 | CDV-1 | NM_014055.2 | 1538 | taggcaacaggcatctatcattt | 339432 | - | see also 5472 line | - | |
| 20858 | CEAL1 | NM_020219.1 | 275 | acggaggcacgagggctatttacc | 376244 | - | | - | |
| 20859 | CECR5 | NM_017829.4 | 1034 | gtgtgtacaggcgtctacaatcc | 362837 | - | | - | |
| 20860 | CECR6 | NM_031890.1 | 1815 | gggtgtctcatggctatgtcaaca | 397229 | - | | - | |
| 20861 | CEI | NM_178569.2 | 512 | caggctccgccaaagaatttag | 431336 | - | | - | |

Figure 46

| | | | | | | | | | |
|---|---|---|---|---|---|---|---|---|---|
| 20862 | CG018 | NM_052818.1 | 633 | cggtatgaacacgatgttacttt | 404261 | - | see also 9432 line | | |
| 20863 | CGEF2 | NM_007023.1 | 1490 | aggcgaatacagtcagacttaaa | 336821 | - | see also 4903 line | | |
| 20864 | CGI-115 | NM_016052.2 | 743 | tggacgatcctacgtgatgattt | 356113 | - | - | - | - |
| 20865 | CGI-12 | NM_015942.3 | 289 | cagtctactagctcaagtagtca | 355534 | - | - | - | - |
| 20866 | CGI-125 | NM_016060.1 | 347 | atgctcagtgtgcgaaatttatt | 356143 | - | see also 6646 line | | |
| 20867 | CGI-127 | NM_016061.1 | 173 | ttgtgcaaactgtgatacgatcc | 356152 | - | - | - | - |
| 20868 | CGI-128 | NM_016062.1 | 381 | aagatggacgtgcacattactcc | 356170 | - | - | - | - |
| 20869 | CGI-32 | NM_015960.1 | 239 | ctgatcggattgaattatgttct | 355632 | - | - | - | - |
| 20870 | CGI-41 | NM_015997.2 | 714 | tggatgcctacatcatcgaattt | 355705 | - | see also 6597 line | | |
| 20871 | CGI-48 | NM_016001.1 | 1470 | ctgaccttcaatcctactacaga | 355737 | - | - | - | - |
| 20872 | CGI-49 | NM_016002.1 | 768 | gaggactcaacgttacttgtatg | 355759 | - | - | - | - |
| 20873 | CGI-57 | NM_015680.3 | 876 | gtggcatcggtggccgtaataca | 354987 | - | - | - | - |
| 20874 | CGI-62 | NM_016010.1 | 668 | caggtaaagtgtcttcaagtagc | 355869 | - | see also 6610 line | | |
| 20875 | CGI-83 | NM_016027.1 | 436 | gagccactctaagagttctatat | 355968 | - | see also 6627 line | | |
| 20876 | CGI-85 | NM_016028.3 | 351 | aggtcgaacagatgtaatggtaa | 355987 | - | see also 6628 line | | |
| 20877 | CGI-90 | NM_016033.1 | 499 | gtgtatgaagccctagagtatgc | 356042 | - | see also 6632 line | | |
| 20878 | CGI-94 | NM_016037.1 | 360 | gagaactcaggacgtcaaatata | 356060 | - | - | nucleic acid binding | - |
| 20879 | CHPF | NM_024536.4 | 2200 | cagcgaggcctgcttctacaact | 386910 | - | - | - | - |
| 20880 | CHPPR | NM_014637.2 | 722 | tggggctccaccaaagtacatct | 341579 | - | see also 5813 line | | |
| 20881 | CHRM4 | NM_000741.1 | 23 | atggcagctcgggcaatcagtcc | 329871 | - | gpcr、receptor_acitivity | muscarinic acetylcholine receptor | - |
| 20882 | CHRNG | NM_005199.3 | 1369 | cagcagagtcactttgacaatgg | 333565 | - | channel、receptor_acitivity、signal_transduction | nicotinic acetylcholine-activated cation-selective channel | - |
| 20883 | CHSY1 | NM_014918.3 | 2375 | acgtcgacctcgtgtttactaca | 345660 | - | - | - | - |
| 20884 | cig5 | NM_080657.3 | 938 | atgaatatatgcgctttctgaac | 406556 | - | - | - | - |
| 20885 | CIRH1A | NM_032830.1 | 1258 | tggatagcctattctacagtttc | 401782 | - | - | - | - |
| 20886 | CITED4 | NM_133467.2 | 609 | cggcatggacgccgaactcatcg | 407233 | - | transcription_regulator | - | - |
| 20887 | CKAP2 | NM_018204.2 | 1468 | aagcttgttaagtattggatatg | 368463 | - | - | - | - |
| 20888 | CKIP-1 | NM_016274.3 | 1062 | aagttgacgcccacagagaaagg | 356663 | - | - | - | - |
| 20889 | CKLFSF8 | NM_178868.3 | 454 | ctggtatggacgcttattgctgg | 432048 | - | - | - | - |
| 20890 | CL25022 | NM_015702.1 | 898 | acgctaccgacatttaggattct | 355202 | - | - | - | - |
| 20891 | CLASP1 | NM_015282.1 | 2580 | aagtccaaaccgaataggattag | 350965 | - | - | - | - |
| 20892 | CLIP-2 | NM_153221.1 | 2891 | gggtccccaggagtatatggtcc | 422268 | - | - | - | - |
| 20893 | CLIPR-59 | NM_015526.1 | 1028 | aaggtcacgctacccaactatga | 353710 | - | - | - | - |

Figure 46

| | CLONE250 | | | | | | | | | |
|---|---|---|---|---|---|---|---|---|---|---|
| 20894 | 03 | NM_015381.1 | 429 | aaggctgcgacttgttaatcaac | 351952 | - | - | | - | |
| 20895 | CLST11240 | NM_016438.2 | 475 | taggtgctgtgtacacaatgtac | 357477 | - | - | | - | |
| 20896 | CMIP | NM_030629.1 | 1877 | ctgcgccgagcacctcatcaaac | 394716 | - | - | | - | |
| 20897 | CML66 | NM_032869.2 | 683 | ctggagtgggtcactatcagtaa | 402295 | - | see also 9325 line | | | |
| 20898 | CMRF-35H | NM_007261.1 | 892 | cggtggtgtttgattctaacacc | 337364 | - | - | | - | |
| 20899 | CMT4B2 | NM_030962.1 | 3451 | gagactatcagcgtttaggttta | 395807 | - | see also 8980 line | | | |
| 20900 | CMYA1 | NM_194293.2 | 2657 | ggggcaccctatatacgaaagg | 436469 | - | - | | - | |
| 20901 | CMYA3 | NM_152381.2 | 3729 | aagagagctacttatgttcataa | 416006 | - | - | | - | |
| 20902 | CMYA4 | NM_173167.1 | 661 | gtgcggatagaccgaatctgtag | 424782 | - | - | | - | |
| 20903 | CMYA5 | NM_153610.2 | 1610 | gtggagaccgttggtaacgtagc | 423343 | - | - | | - | |
| 20904 | CNAP1 | NM_014865.1 | 1482 | gtgtagccttgacccgttataac | 344973 | - | see also 6064 line | | | |
| 20905 | CNNM1 | NM_020348.1 | 516 | gggtgaccagcggcacaacattg | 376801 | - | - | | - | |
| 20906 | CNNM4 | NM_020184.1 | 848 | aggagattcgcactgtttacaac | 375845 | - | see also 7789 line | | | |
| 20907 | CNO | NM_018366.2 | 628 | acgtgccctcgctctttagcaag | 370888 | - | - | | - | |
| 20908 | CNOT10 | NM_015442.1 | 1291 | atgtgtacgttactaaccaataa | 352885 | - | see also 6418 line | | | |
| 20909 | CNOT3 | NM_014516.1 | 2208 | aaggacgacgttgagtactatgt | 340819 | - | see also 5745 line | | | |
| 20910 | CNOT6L | NM_144571.1 | 193 | gtgcggagcctaagtacatcact | 409775 | - | see also 9693 line | | | |
| 20911 | CNTN4 | NM_175607.1 | 2711 | aggacgaatacaaggttatgagg | 429132 | - | see also 10145 line | | | |
| 20912 | CNTN5 | NM_014361.2 | 1314 | tggcaaccccgttccaacaatca | 340290 | - | - | | - | |
| 20913 | COBL | NM_015198.2 | 627 | ctgtgcgtttggtcgtgaattac | 349656 | - | - | | - | |
| 20914 | COBRA1 | NM_015456.2 | 1157 | tggtggatgactacactttcaat | 353234 | - | transcription_regulator | - | | |
| 20915 | COH1 | NM_015243.2 | 222 | cagcaagctcgagttaaagttgg | 350163 | - | - | | - | |
| 20916 | COL13A1 | NM_005203.3 | 2031 | aagggacctcgcggtaaaccagg | 333576 | - | see also 3655 line | | | |
| 20917 | COL23A1 | NM_173465.2 | 851 | aagggtgcaccaggagactttgg | 424842 | - | - | | - | |
| 20918 | COL24A1 | NM_152890.4 | 369 | cagtcacatcgggtgaacaatgc | 421436 | - | - | | - | |
| 20919 | COL25A1 | NM_032518.2 | 1681 | aaggggaaacaaggtgaatcagg | 400519 | - | - | | - | |
| 20920 | COL26A1 | NM_133457.1 | 264 | ctgcgccaacctcgtaagttaca | 407230 | - | - | | - | |
| 20921 | COL8A2 | NM_005202.1 | 1771 | gggcatgcccgtgaaatttgacc | 333569 | - | - | | - | Alzheimer disease |
| 20922 | COP1 | NM_022457.4 | 1186 | ttgtccaagtttactcgatataa | 382882 | - | see also 8376 line | | | |
| 20923 | COPS3 | NM_003653.2 | 732 | tggcggtcagtcatatcatgttg | 331897 | - | see also 2472 line | | | |
| 20924 | COPS4 | NM_016129.2 | 410 | aagatccagcctagagtcatttc | 356378 | - | see also 6680 line | | | |
| 20925 | COPS7A | NM_016319.1 | 935 | gagggagcgccaagatttggtcc | 356951 | - | - | | - | |
| 20926 | COPS7B | NM_022730.1 | 470 | cggaatctccgggaactagaaga | 383742 | - | see also 8434 line | | | |

Figure 46

| | | | | | | | | | |
|---|---|---|---|---|---|---|---|---|---|
| 20927 | CORTBP2 | NM_033427.1 | 3785 | tggactgtccgaatgttattact | 403965 | - | - | - | - |
| 20928 | COX7B2 | NM_130902.1 | 277 | gtgttgctacatgggtgtttaca | 407157 | - | - | - | - |
| 20929 | CPEB2 | NM_182485.1 | 1421 | gaggtgttcctaggccattaagg | 433869 | - | see also 10259 line | | |
| 20930 | CPLX4 | NM_181654.1 | 225 | agggcatgcctcagagttcatct | 432797 | - | - | - | - |
| 20931 | CPNE2 | NM_152727.4 | 642 | ctgcggggaagggcttgattacg | 420108 | - | see also 9933 line | | |
| 20932 | CPNE4 | NM_130808.1 | 697 | gtgcagcgcctgcggtttgaagt | 406939 | - | - | - | - |
| 20933 | CPNE5 | NM_020939.1 | 892 | ccgtgggcagagccaattcaaca | 380207 | - | - | - | - |
| 20934 | CPNE8 | NM_153634.2 | 1204 | aggatatctcacgaatttgcttt | 423440 | - | see also 10002 line | | |
| 20935 | CPR8 | NM_004748.2 | 2435 | tggacccaggtcggtttacataa | 332906 | - | - | - | - |
| 20936 | CPXCR1 | NM_033048.3 | 907 | atgtagattccgtgctattgtga | 402692 | - | - | - | - |
| 20937 | CRA | NM_006697.1 | 545 | cagggtccctgcataaatttatc | 336046 | - | - | - | - |
| 20938 | CREB3L2 | NM_194071.1 | 1371 | aggttctagagaacactaatagg | 436051 | - | - | - | - |
| 20939 | CREG2 | NM_153836.1 | 877 | aggaagtggcctcgtcaatatga | 423948 | - | - | - | - |
| 20940 | CRG-L2 | NM_181789.1 | 1002 | gtggacggctcgagcattcttgt | 433493 | - | - | - | - |
| 20941 | CRIPT | NM_014171.2 | 134 | aagatggtgctaggaataccaca | 339831 | - | - | - | - |
| 20942 | CROC4 | NM_006365.1 | 479 | aagatctatatggctatagcacc | 335351 | - | - | - | - |
| 20943 | CRR9 | NM_030782.2 | 622 | ccgatgtgcatcggtacatgaag | 394970 | - | see also 8945 line | | |
| 20944 | CRTAM | NM_019604.1 | 737 | cagtaacggaagattctagtaca | 375468 | - | - | - | - |
| 20945 | CRYGN | NM_144727.1 | 294 | cggcgactaccccgacttcttcc | 411862 | - | - | - | - |
| 20946 | CSE-C | NM_018978.1 | 1700 | ctggcgatcgattcttgtcatgg | 373986 | - | - | - | - |
| 20947 | CSMD2 | NM_052896.2 | 6973 | gtgacatcgtacgctacagatgc | 404702 | - | - | - | - |
| 20948 | CSMD3 | NM_052900.2 | 6521 | ctgatccacgcccgtttcgaaat | 405007 | - | - | - | - |
| 20949 | CSTL1 | NM_138283.1 | 411 | cagcaacgacacctacttatatc | 407372 | - | - | cysteine protease inhibitor | - |
| 20950 | CTAG3 | NM_175745.2 | 473 | atggcacactagacaatttgtta | 429218 | - | - | - | - |
| 20951 | CTAGE-2 | NM_172241.1 | 115 | gagacccgattctcatccttatg | 424366 | - | - | - | - |
| 20952 | CTCFL | NM_080618.2 | 1402 | aagcgacctacgtgtgcatatgc | 406488 | - | - | cell_cycle、transcription_regulator | - |
| 20953 | CTHRC1 | NM_138455.2 | 238 | gtggtggacctgtataatggaat | 408270 | - | - | - | - |
| 20954 | CTL2 | NM_020428.2 | 1624 | gtgcagatcatccgtgtgatact | 377456 | - | kinase_related | - | - |
| 20955 | CTXL | NM_014312.3 | 402 | ctgacgtccacccctcagatact | 340145 | - | - | - | - |
| 20956 | CXorf15 | NM_018360.1 | 689 | gagaacttcagcgtcacaataag | 370749 | - | - | - | - |
| 20957 | CYFIP1 | NM_014608.1 | 2227 | acggttacgatcagaatgcaaga | 341068 | - | see also 5772 line | | |
| 20958 | CYFIP2 | NM_014376.1 | 2197 | ctggattctaacggaccatatcc | 340448 | - | see also 5638 line | | |
| 20959 | CYLN2 | NM_003388.3 | 2007 | tggggtgctgcgggataaatacg | 331652 | - | - | - | - |
| 20960 | CYorf15B | NM_032576.1 | 608 | aagctgtacaaggctcttcaaat | 400826 | - | - | - | - |
| 20961 | CYP26C1 | NM_183374.1 | 789 | gggtgatgccctcgacctaatca | 435688 | - | - | - | - |

Figure 46

| 20962 | D2LIC | NM_015522.2 | 219 | tggggaaagactactattattc | 353682 | - | |
|---|---|---|---|---|---|---|---|
| 20963 | D6S2654E | NM_012135.1 | 253 | gaggttctcggcgcgattacgacg | 337691 | - | |
| 20964 | DAB2IP | NM_032552.1 | 1894 | ctgcccgcagctcgagtctactcg | 400676 | - | |
| 20965 | DARP | NM_144994.5 | 777 | gtgcggcacggacggagctacaaagc | 412505 | - | |
| 20966 | DATF1 | NM_022105.2 | 2017 | gtgggcgagcgatcacaattaca | 382358 | - | apoptosis, transcription regulator |
| 20967 | DBC-1 | NM_021174.4 | 1628 | gcgctttgccgagtttcagtacc | 380571 | - | |
| 20968 | DBC1 | NM_014618.1 | 2508 | tggatattcggacccgaattaat | 341363 | - | see also 5789 line |
| 20969 | DBCCR1L | NM_199051.1 | 2508 | atgacacgaccaaattatgtagt | 440719 | - | see also 10360 line |
| 20970 | DC-UbP | NM_152277.1 | 342 | tggcaccgccaatcaacatgata | 415300 | - | see also 9826 line |
| 20971 | DC12 | NM_020187.1 | 392 | tcgtagtgataccgtaatggaga | 375876 | - | |
| 20972 | DC13 | NM_020188.1 | 214 | atgcaacgtcttgattaacttgc | 375884 | - | |
| 20973 | DC6 | NM_020189.4 | 137 | aagagaacgcctcaaagagttgc | 375898 | - | |
| 20974 | DCLRE1A | NM_014881.1 | 2453 | gtgtgccagttgttaaagcttaca | 345177 | - | see also 6075 line |
| 20975 | DCLRE1B | NM_022836.2 | 234 | tggcaccgcacgtctcttctct | 384491 | - | |
| 20976 | DCLRE1C | NM_022487.1 | 1863 | gtgcaatagttcctagctactgg | 383424 | - | |
| 20977 | DCNP1 | NM_130848.1 | 847 | tggtcttcgcacttgatatcact | 407097 | - | |
| 20978 | DCOHM | NM_032151.2 | 88 | tagggctagcggccatgtcatca | 398382 | - | |
| 20979 | DCP1B | NM_152640.3 | 395 | cagattgtccatcatcatggaattt | 419427 | - | |
| 20980 | DCP2 | NM_152624.3 | 1353 | gggacagcccgtggcatgtaatg | 419138 | - | |
| 20981 | DDA3 | NM_032636.4 | 635 | agggagtcacccacttgcaatct | 401159 | - | |
| 20982 | DDX27 | NM_017895.6 | 342 | gaggcagccactacattagatg | 363676 | - | |
| 20983 | DEADC1 | NM_182503.1 | 752 | ctgtggaccgtgcattatgtgt | 433980 | - | |
| 20984 | DEPC-1 | NM_139178.1 | 1220 | ctgacctttcggacagtctatcc | 409447 | - | |
| 20985 | DERP6 | NM_015362.2 | 153 | gtgtcggggagcaagtgcatatcc | 351823 | - | |
| 20986 | DEXI | NM_014015.3 | 648 | tcggagctcgacgtctttgatgc | 339145 | - | |
| 20987 | DIBD1 | NM_024740.1 | 217 | gagcaccggacgagttatctgg | 389772 | - | see also 8718 line |
| 20988 | DIP13B | NM_018171.2 | 1556 | tggaaacgccgattcaattcgata | 367954 | - | |
| 20989 | DIRC1 | NM_052952.1 | 503 | taggtctgaattgaatactgtga | 405502 | - | |
| 20990 | DIRC2 | NM_032839.1 | 787 | gagcagcagggcgcatattaaag | 401896 | - | see also 9304 line |
| 20991 | DISP2 | NM_033510.1 | 2822 | ccggaacagtccggactaccaacc | 404046 | - | |
| 20992 | DJ1042K10.2 | NM_015705.3 | 202 | cagccctgactccgagcatatgg | 355213 | - | see also 6539 line |
| 20993 | DJ122O8.2 | NM_020466.3 | 206 | gtgccaccgaagaggatacaatc | 377728 | - | |
| 20994 | DJ167A19.1 | NM_018982.3 | 778 | ggggaagaagacgtactaccattatg | 374001 | - | see also 7641 line |

Figure 46

| | | | | | | | | | |
|---|---|---|---|---|---|---|---|---|---|
| 20995 | DJ465N24.2.1 | NM_020317.2 | 494 | acgggttcaccaggagatactac | 376483 | - | - | - | - |
| 20996 | DJ667H12.2 | NM_019605.2 | 922 | taggtagtgcatctatttacaag | 375499 | - | - | - | - |
| 20997 | DJ79P11.1 | NM_032621.1 | 113 | gaggaacgagcgttaaacaatct | 401124 | - | - | - | - |
| 20998 | DKFZp313N0621 | NM_173826.1 | 1101 | cagccagtattgacacttgaaga | 428428 | - | - | - | - |
| 20999 | DKFZP434A1022 | NM_021185.3 | 2001 | tggtgggcgaacaacaaacaaga | 380688 | - | - | - | - |
| 21000 | DKFZP434A1315 | NM_032132.2 | 256 | atgcgcttatggaacaagatatc | 398050 | - | - | - | - |
| 21001 | DKFZP434A1319 | NM_032140.1 | 878 | gtggacttcattcgtcacaatgc | 398323 | - | - | - | - |
| 21002 | DKFZp434A1721 | NM_017609.2 | 1602 | gtgttcgatgaagggacaatttc | 359421 | - | - | - | - |
| 21003 | DKFZP434B0335 | NM_015395.1 | 3472 | ctgggtgatcgccaacaaagtgc | 352217 | - | - | - | - |
| 21004 | DKFZp434B0417 | NM_013377.2 | 2267 | gcgccgtcgccgtgagttcatga | 338897 | - | - | - | - |
| 21005 | DKFZp434B1231 | NM_178275.3 | 2045 | gtgcggcagggctgtcagtatga | 430691 | - | - | - | - |
| 21006 | DKFZP434B168 | NM_015434.1 | 830 | cagcgtcatctttggttgataca | 352788 | - | - | - | - |
| 21007 | DKFZp434B227 | NM_032263.2 | 1616 | tggtggcgaggcactatgatacg | 399134 | - | - | - | - |
| 21008 | DKFZp434C0328 | NM_017577.1 | 2142 | aagatcaggcccatcgtttaaag | 359337 | - | - | - | - |
| 21009 | DKFZp434C171 | NM_015621.1 | 1111 | ctggccatttgacataaaggaga | 354618 | - | - | - | - |
| 21010 | DKFZp434C1714 | NM_152834.2 | 303 | atgaggcggctgcgatgaactgg | 421377 | - | - | - | - |
| 21011 | DKFZP434C245 | NM_015426.2 | 563 | gggaatgtgtccactcgtattgt | 352631 | - | - | - | - |
| 21012 | DKFZP434D1335 | NM_015578.1 | 225 | ccgacagatcgtccaataccacc | 354295 | - | - | - | - |
| 21013 | DKFZP434D146 | NM_015595.2 | 289 | gagccccaacgggttactaatta | 354361 | - | - | - | - |
| 21014 | DKFZp434E2220 | NM_017612.2 | 381 | ttggtgaacgatactaagttaga | 359537 | - | see also 7049 line | | |

Figure 46

| 21015 | DKFZP434F0318 | NM_030817.1 | 325 | ccgatctctcgctgatcttctgc | 395345 | - | - | - | - |
|---|---|---|---|---|---|---|---|---|---|
| 21016 | DKFZP434F1017 | NM_032135.2 | 345 | aagctacccatcgtattggtaat | 398196 | - | - | - | - |
| 21017 | DKFZp434F1719 | NM_032248.1 | 366 | tggatgtctaggtggtagaaacc | 399010 | - | - | - | - |
| 21018 | DKFZP434F2021 | NM_015412.1 | 1216 | aagcaaccggcttaaacatctgg | 352324 | - | - | - | - |
| 21019 | DKFZp434G0522 | NM_017566.2 | 637 | aagtacacgggattacatctact | 359124 | - | - | - | - |
| 21020 | DKFZp434G0625 | NM_181775.2 | 574 | gggccgtcaatcggatttacaag | 433142 | - | - | - | - |
| 21021 | DKFZp434G072 | NM_032149.1 | 988 | ttgccgagataaacctgttaact | 398374 | - | - | - | - |
| 21022 | DKFZp434G118 | NM_032266.1 | 538 | gagtcctacttccacaatagatt | 399173 | - | - | - | - |
| 21023 | DKFZP434G156 | NM_022742.2 | 2633 | gagaactgtcgcaagacttatga | 383818 | - | - | - | - |
| 21024 | DKFZP434H0115 | NM_031421.1 | 170 | gagcttcagcaacgctctttacc | 396894 | - | - | - | - |
| 21025 | DKFZP434H0820 | NM_022833.1 | 510 | atgcgcgtcactactacttctgc | 384480 | - | - | - | - |
| 21026 | DKFZp434H2010 | NM_032129.1 | 1366 | ctgaccacacttcggaaacatca | 397953 | - | - | - | - |
| 21027 | DKFZp434H2215 | NM_017559.1 | 313 | ttgctgaaccccatcaaattagc | 359080 | - | - | - | - |
| 21028 | DKFZp434I099 | NM_032269.3 | 1476 | aggacttgcagtgtaccaatatt | 399240 | - | - | - | - |
| 21029 | DKFZp434I1020 | NM_194295.1 | 211 | aggagtgagaaatgttagtgttg | 436501 | - | - | - | - |
| 21030 | DKFZp434I1916 | NM_032245.1 | 403 | aggaaactgatccttcatgtttc | 399008 | - | - | - | - |
| 21031 | DKFZP434J0113 | NM_032130.1 | 2271 | aagcgatcaaccatgtacaaatc | 397968 | - | - | - | - |
| 21032 | DKFZP434J046 | NM_015671.2 | 1045 | aagctgaagaccacttcttcaac | 354962 | - | - | - | - |
| 21033 | DKFZP434J154 | NM_015610.1 | 700 | gtgtgcgctgtcaatcaacaacg | 354597 | - | see also 6509 line | | |
| 21034 | DKFZP434K1172 | NM_031290.1 | 369 | cagtattcgccagaagaaactaa | 396524 | - | - | - | - |

Figure 46

| 21035 | DKFZP434K1421 | NM_032141.1 | 1601 | aagcggaacaatgaagaaactgt | 398351 | - | - | - | - |
|---|---|---|---|---|---|---|---|---|---|
| 21036 | DKFZp434K1815 | NM_152892.1 | 512 | aggatgcgtcctcaacttactct | 421501 | - | - | - | - |
| 21037 | DKFZp434K2435 | NM_032256.1 | 691 | ctgacgtctgtggttgtacttgt | 399042 | - | - | - | - |
| 21038 | DKFZP434L0117 | NM_022778.2 | 767 | cagcacaagtaaggagttgtaca | 384143 | - | see also 8469 line | | |
| 21039 | DKFZP434L0718 | NM_032139.1 | 770 | gagatatacgtccatcatgaaat | 398280 | - | - | - | - |
| 21040 | DKFZp434L0850 | NM_017558.1 | 2926 | gggacgtcggatccaacagttgt | 359068 | - | see also 7032 line | | |
| 21041 | DKFZp434L142 | NM_016613.4 | 1801 | atgcacacggggtcaaagtatta | 358430 | - | - | - | - |
| 21042 | DKFZP434L1717 | NM_032136.3 | 1596 | gtggtccgccacggtgtcaatga | 398257 | - | - | - | - |
| 21043 | DKFZp434M202 | NM_182499.1 | 503 | gagtaccacctgggggattatgg | 433967 | - | - | - | - |
| 21044 | DKFZp434N035 | NM_032262.1 | 288 | ctgcagctagcttcgattgttcc | 399105 | - | - | - | - |
| 21045 | DKFZp434N1415 | NM_032133.2 | 101 | aggattcccgcctcagaataact | 398088 | - | - | - | - |
| 21046 | DKFZP434O047 | NM_015594.1 | 2877 | aagggaatcagtgtctcattatc | 354353 | - | - | - | - |
| 21047 | DKFZp434O0527 | NM_194302.1 | 2109 | tgggctacaacccccatatgatg | 436658 | - | - | - | - |
| 21048 | DKFZP434P0316 | NM_032134.1 | 1231 | ttgcctgtcgtcgatcaacatgg | 398149 | - | - | - | - |
| 21049 | DKFZp434P055 | NM_173466.1 | 1321 | ttgtcagctaaggcgaataatgc | 424866 | - | - | - | - |
| 21050 | DKFZp451M2119 | NM_182585.1 | 3329 | aagttctgtaggcttataacata | 434523 | - | - | - | - |
| 21051 | DKFZp547A023 | NM_018704.1 | 1020 | tagccctaatgagcaattgaaga | 372706 | - | see also 7571 line | | |
| 21052 | DKFZp547B1713 | NM_152379.1 | 284 | gagcgcttcgagcttcttcaagg | 415992 | - | - | - | - |
| 21053 | DKFZp547C176 | NM_018712.2 | 511 | ctgcgtagagaggcctatgattc | 372789 | - | see also 7574 line | | |
| 21054 | DKFZp547E052 | NM_032276.2 | 804 | caggacggcaatactactttaat | 399335 | - | - | - | - |

Figure 46

| | | | | | | | |
|---|---|---|---|---|---|---|---|
| 21055 | DKFZP547E1010 | NM_015607.2 | 403 | cagccgacgccagtgaatattcg | 354469 | - | see also 6506 line |
| 21056 | DKFZp547G0215 | NM_173643.1 | 1052 | cagcaagtaatgatagtgaaa | 426937 | - | - |
| 21057 | DKFZP564A022 | NM_030954.2 | 687 | tgatctaccacttactgagg | 395683 | - | - |
| 21058 | DKFZP564B1162 | NM_031305.1 | 790 | ctggaggatactgttcgttatga | 396622 | - | - |
| 21059 | DKFZP564D116 | NM_015631.1 | 852 | tggggacccattcttacttact | 354715 | - | see also 6524 line |
| 21060 | DKFZP564D172 | NM_032042.3 | 1121 | ccgaccgtcacgagctaacttcc | 397728 | - | - |
| 21061 | DKFZP564G2022 | NM_015497.2 | 139 | ctgctgccgaccggtccaaatgg | 353576 | - | see also 6438 line |
| 21062 | DKFZp564I1922 | NM_015419.1 | 5740 | gtgatacggaggttcaagtaca | 352488 | - | receptor_acitivity |
| 21063 | DKFZP564J047 | NM_032126.1 | 715 | cagaggcctcaggcctttacaaa | 397918 | - | - |
| 21064 | DKFZP564K0322 | NM_032040.1 | 790 | cggggaggacgtcgactatttgc | 397679 | - | - |
| 21065 | DKFZP564K0822 | NM_030796.2 | 220 | aggactctatccaacctattata | 395096 | - | kinase_related |
| 21066 | DKFZP564K1964 | NM_015544.1 | 864 | ctgcaggagcagtctgcaattta | 353845 | - | - |
| 21067 | DKFZP564K2062 | NM_015421.1 | 471 | tggcggcaggacacatactgtcc | 352595 | - | - |
| 21068 | DKFZP564M082 | NM_014042.1 | 352 | aagcatgcgcggagaaagacaaca | 339372 | - | - |
| 21069 | DKFZP564O043 | NM_020319.1 | 398 | gcgacatggagccgatgtaaatt | 376506 | - | see also 7832 line |
| 21070 | DKFZP564O0463 | NM_015420.4 | 1626 | gtgtctgctagtttcgataaatc | 352566 | - | see also 6405 line |
| 21071 | DKFZP564O0523 | NM_032120.1 | 213 | gagcgattcgctttatatggtgc | 397810 | - | - |
| 21072 | DKFZP564O0823 | NM_015393.1 | 508 | gcggtggagtccacttaacaacc | 352197 | - | - |
| 21073 | DKFZP564O1664 | NM_030800.1 | 1300 | atggtagcggattgttcaattagg | 395174 | - | - |
| 21074 | DKFZP564O1863 | NM_015633.1 | 520 | cggccacggtccacgttagttat | 354744 | - | - |

Figure 46

| | | | | | | | | | |
|---|---|---|---|---|---|---|---|---|---|
| 21075 | DKFZP566A1524 | NM_030797.1 | 1137 | aagcacttgaacgatgaatcaac | 395118 | - | - | - | - |
| 21076 | DKFZP566B183 | NM_015509.2 | 399 | atgccttcgactttaatgtctcc | 353632 | - | see also 6445 line | | |
| 21077 | DKFZP566D1346 | NM_030816.1 | 422 | ctgccctcggcggtacctttacc | 395298 | - | - | - | - |
| 21078 | DKFZP566D193 | NM_015391.1 | 209 | ctgtcaaagaacaagaaatgaag | 352187 | - | - | - | - |
| 21079 | DKFZP566F0546 | NM_015653.1 | 443 | ctgtagggctatcaatgacttcc | 354926 | - | - | - | - |
| 21080 | DKFZP566F084 | NM_015448.1 | 129 | gagaagacgagtgaactacttgt | 353062 | - | - | - | - |
| 21081 | DKFZP566F2124 | NM_015630.2 | 2115 | cagttggccacaccactacaaac | 354698 | - | see also 6510 line | | |
| 21082 | DKFZP566K1924 | NM_015463.1 | 483 | caggtcgagaatatttccattgg | 353313 | - | - | - | - |
| 21083 | DKFZP566M1046 | NM_032127.1 | 1045 | tcgcggatcactcttacttctgc | 397926 | - | see also 9079 line | | |
| 21084 | DKFZP566M114 | NM_032128.1 | 234 | ctggcgatcgctggagctaaact | 397941 | - | - | - | - |
| 21085 | DKFZP566N034 | NM_030923.2 | 841 | ctgatcggcctcaccatatttgc | 395503 | - | - | - | - |
| 21086 | DKFZp586C1924 | NM_032273.2 | 418 | ctgactggtcttgttattggtgg | 399319 | - | - | - | - |
| 21087 | DKFZP586D0919 | NM_015433.1 | 548 | ctgggggattgaccatcatgtct | 352768 | - | - | - | - |
| 21088 | DKFZp586I1420 | NM_152747.1 | 2130 | tagggactgtgtacctttattca | 420446 | - | - | - | - |
| 21089 | DKFZP586L0724 | NM_015462.2 | 383 | aggtgctgttcgtggtttagagg | 353304 | - | - | - | - |
| 21090 | DKFZP586M1019 | NM_015603.1 | 151 | cagccacactcgatttgaaatca | 354455 | - | - | - | - |
| 21091 | DKFZP586M1120 | NM_031294.2 | 1206 | aagcacttgtcgagtttaagtgc | 396573 | - | - | - | - |
| 21092 | DKFZP586M1523 | NM_015476.1 | 920 | aagagaccgactcctttgtgaat | 353429 | - | - | - | - |
| 21093 | DKFZP586N0721 | NM_015400.1 | 1085 | aggtaaccgtcttcacaatgtat | 352301 | - | - | - | - |
| 21094 | DKFZp667B1218 | NM_177966.3 | 1087 | tcgactaccgccagaaccttatc | 430319 | - | - | - | - |

Figure 46

| 21095 | DKFZp686 A17109 | NM_198465.1 | 1574 | cagtgcacctaagcctctaaagg | 438124 | - | - | - | - |
|---|---|---|---|---|---|---|---|---|---|
| 21096 | DKFZp686 B0797 | NM_198539.1 | 527 | gggaggcactgtccagaagtttg | 439300 | - | - | - | - |
| 21097 | DKFZp686 B2197 | NM_194319.1 | 968 | ccgtgtctcagctcttatagatc | 436837 | - | - | - | - |
| 21098 | DKFZp686 G0786 | NM_198460.1 | 658 | ttgaacggtcaccctatcacaga | 437902 | - | - | - | - |
| 21099 | DKFZp686L 1814 | NM_194282.1 | 907 | aagttaccaccgcagcaaattaa | 436247 | - | see also 10306 line | | |
| 21100 | DKFZp686L 20145 | NM_019045.2 | 1288 | aagtacggttaaggattctcagc | 374688 | - | - | - | - |
| 21101 | DKFZp686 N19164 | NM_194325.1 | 1468 | cagcgaattcatgcagagataaa | 436867 | - | - | - | - |
| 21102 | DKFZp686 O1689 | NM_182608.2 | 823 | gggggctcacggcgttaatgaag | 434633 | - | - | - | - |
| 21103 | DKFZP727 C091 | NM_015443.1 | 3493 | cgggacactccgcgacacttagc | 352987 | - | - | - | - |
| 21104 | DKFZp727 G131 | NM_145111.1 | 557 | ctgcagtccggtatagactttgc | 413505 | - | - | - | - |
| 21105 | DKFZP727 M111 | NM_015540.2 | 1944 | cagggcctacccctagtctatac | 353772 | - | - | - | - |
| 21106 | DKFZp761 A052 | NM_017602.2 | 570 | aggtcggcgcaggctacaacagt | 359391 | - | see also 7046 line | | |
| 21107 | DKFZp761 A078 | NM_194292.1 | 905 | ctgagttagaagcggctaataaa | 436422 | - | - | - | - |
| 21108 | DKFZp761 B107 | NM_173463.2 | 686 | gggcacgagaaccgcatcattga | 424838 | - | - | - | - |
| 21109 | DKFZp761 B1514 | NM_032288.3 | 580 | atgaatcgtccacctctaagtga | 399369 | - | - | - | - |
| 21110 | DKFZp761 C121 | NM_032290.1 | 597 | ttgccaggaatagacatcaatgt | 399419 | - | see also 9136 line | | |
| 21111 | DKFZp761 C169 | NM_022913.1 | 1581 | gggaatatcctccgaatcctaaa | 384820 | - | see also 8531 line | | |
| 21112 | DKFZp761 D221 | NM_032291.1 | 2070 | caggctacatattataacgttga | 399494 | - | see also 9139 line | | |
| 21113 | DKFZP761 E1824 | NM_022486.1 | 1618 | acgtccgggtaccaactacaatg | 383376 | - | - | - | - |
| 21114 | DKFZP761F 241 | NM_031455.2 | 475 | tggtccaggactactcctatttc | 397080 | - | - | - | - |

Figure 46

| | | | | | | | | | |
|---|---|---|---|---|---|---|---|---|---|
| 21115 | DKFZp761H0421 | NM_173079.1 | 742 | gagcttcgtcagcgtgtagatgc | 424581 | - | - | - | - |
| 21116 | DKFZp761H079 | NM_144996.2 | 966 | cagaccggccatcattggaatca | 412524 | - | - | - | - |
| 21117 | DKFZP761H1710 | NM_031297.2 | 231 | gtgggcactcctacaatgtcacc | 396590 | - | - | - | - |
| 21118 | DKFZP761K1423 | NM_018422.1 | 1423 | tcggtcgccataaataagtcttc | 371347 | - | - | - | - |
| 21119 | DKFZP761L0424 | NM_019590.1 | 2781 | ccgggctgacagtcacgttaaag | 375361 | - | see also 7715 line | | |
| 21120 | DKFZp761L1417 | NM_152913.1 | 727 | aagactccgtggtctattataac | 421684 | - | - | - | - |
| 21121 | DKFZp761L1518 | NM_198075.1 | 1114 | aggtgcccaggggctacaactac | 436971 | - | - | - | - |
| 21122 | DKFZp761N1114 | NM_181644.2 | 1411 | caggctcagggcagctatagttt | 432644 | - | - | - | - |
| 21123 | DKFZp761O0113 | NM_018409.2 | 1340 | aagtatgctcgggctgtattact | 371189 | - | - | - | - |
| 21124 | DKFZp761O17121 | NM_032287.1 | 250 | atgaatgggaggtgtacagatca | 399356 | - | - | - | - |
| 21125 | DKFZp761P1121 | NM_152906.2 | 435 | ccgagggcgaggtgaacttgtca | 421676 | - | - | - | - |
| 21126 | DKFZp762C2414 | NM_178542.2 | 1111 | gtgtcctccatgagaatcataca | 431156 | - | - | - | - |
| 21127 | DKFZp762E1312 | NM_018410.2 | 1719 | aaggaatcgttacgatgaaatta | 371234 | - | - | - | - |
| 21128 | DKFZp762G199 | NM_197956.1 | 871 | aggtcctccggcctatgatcaaa | 436915 | - | - | - | - |
| 21129 | DKFZp762I194 | NM_152384.1 | 422 | atggcagtacacagagcttatga | 416041 | - | see also 9843 line | | |
| 21130 | DKFZp762O076 | NM_018710.1 | 728 | aggcgatttcgagcaacctatgt | 372773 | - | see also 7572 line | | |
| 21131 | DLNB14 | NM_198489.1 | 433 | atgcgcgattcaagaaatccatg | 438537 | - | - | - | - |
| 21132 | DNAJC9 | NM_015190.3 | 373 | gcgtattggcggctactctttaa | 349535 | - | - | - | - |
| 21133 | DNAJD1 | NM_013238.1 | 525 | gggactggtaagaagtttgatag | 338361 | - | - | - | - |
| 21134 | DNAPTP6 | NM_015535.1 | 1735 | tagtgtcggtagggtttcacagt | 353750 | - | see also 6460 line | | |
| 21135 | DOCK5 | NM_024940.3 | 1529 | ctgctaaagcgactaatagcatt | 392343 | - | - | - | - |
| 21136 | DOLPP1 | NM_020438.2 | 166 | cggtttcgtgaccctcatcatat | 377468 | - | see also 7876 line | | |
| 21137 | DOM3Z | NM_005510.2 | 1119 | ctgtgccgccttccttagctttg | 334047 | - | - | - | - |

Figure 46

| | | | | | | | |
|---|---|---|---|---|---|---|---|
| 21138 | DP1L1 | NM_138393.1 | 520 | tcgtgcgtccgctgttcctaagg | 407943 | - | |
| 21139 | DPCR1 | NM_080870.2 | 559 | aggaaacgagagccatccatacc | 406871 | - | |
| 21140 | DPH2L2 | NM_001384.2 | 1101 | ctggcaagcgtagctatgtgttg | 330198 | - | |
| 21141 | DPPA4 | NM_018189.1 | 458 | gggaaacgtccctgcaaagttct | 368231 | - | |
| 21142 | DREV1 | NM_016025.2 | 1036 | aaggcggacatgtataatgactac | 355956 | - | see also 6625 line |
| 21143 | DT1P1A10 | NM_058163.1 | 441 | gaggtcacagctacgaatgatgg | 406187 | - | |
| 21144 | DTNBP1 | NM_032122.3 | 308 | ctgctggattagaattacttagc | 397879 | - | schizophrenia |
| 21145 | DUFD1 | NM_138419.2 | 630 | gagtgacgagcgcattagtttgg | 408027 | - | |
| 21146 | DULLARD | NM_015343.3 | 501 | aagctctggagcttcttcattta | 351752 | - | |
| 21147 | DUSP15 | NM_080611.3 | 369 | tggactctacctcggaaacttca | 406405 | - | phosphatase |
| 21148 | DYX1C1 | NM_130810.1 | 1171 | aggcacgaagagcaatgaatact | 407022 | - | see also 9554 line |
| 21149 | DZIP1 | NM_014934.2 | 1550 | ccgagtgagatcgtcgtattga | 346050 | - | |
| 21150 | E2IG2 | NM_016565.1 | 188 | ctggacccaacgggtgaagaaag | 358147 | - | |
| 21151 | E2IG4 | NM_015516.1 | 798 | cgggcaacccaagcttaactgg | 353655 | - | |
| 21152 | E2IG5 | NM_014367.2 | 611 | atgtggtatgaggatccatttca | 340417 | - | |
| 21153 | EAF1 | NM_033083.5 | 956 | gagggctgaagttgaacattattg | 403204 | - | |
| 21154 | EAF2 | NM_018456.3 | 102 | tcgcgagcgggttctcaagttag | 371790 | - | |
| 21155 | EB-1 | NM_152788.2 | 2174 | aagccttgatcgacatgcatttg | 421341 | - | |
| 21156 | EB-1 | NM_020140.1 | 958 | gagcttaggcgggggaaatcaac | 375659 | - | see also 7764 line |
| 21157 | ECRG4 | NM_032411.1 | 443 | atgaatactatggcgattactac | 400203 | - | |
| 21158 | EDRF1 | NM_015608.2 | 3090 | ttgtccactacttacttactat | 354571 | - | |
| 21159 | EEG1 | NM_017611.2 | 367 | tggctggccttcactagtgtttg | 359513 | - | |
| 21160 | EFA6R | NM_015310.1 | 1349 | ctggccgagcacgcgtccatatcc | 351422 | - | see also 6322 line |
| 21161 | EG1 | NM_025205.1 | 189 | gtgagtcaggataggttgatatg | 394414 | - | |
| 21162 | EGFL11 | NM_198283.1 | 2039 | caggaatatcggtatctatgttt | 437643 | - | |
| 21163 | EIF2C3 | NM_024852.2 | 2808 | aagatacccttacgccacaatgtac | 391509 | - | see also 8784 line |
| 21164 | EIF2C4 | NM_017629.2 | 483 | ttggacggataggggttgatatg | 359742 | - | see also 7058 line |
| 21165 | ELAC1 | NM_018696.1 | 333 | ctgtaggcttcgggacttatc | 372648 | - | see also 7565 line |
| 21166 | ELAC2 | NM_018127.4 | 1360 | cagaggatgccattattacttg | 366996 | - | prostate cancer |
| 21167 | ELKS | NM_015064.2 | 2776 | gcgcacgacggaggacaatctca | 347576 | - | see also 6218 line |
| 21168 | Elis1 | NM_152793.1 | 120 | ctgttaggagcagatctgatga | 421370 | - | |
| 21169 | ELP4 | NM_019040.2 | 17 | gttggcaactgcgcggtagtgttgc | 374586 | - | see also 7673 line |
| 21170 | ELSPBP1 | NM_022142.3 | 361 | aagattacccagcgctgtatcttc | 382467 | - | |
| 21171 | EMCN | NM_016242.2 | 823 | aagctcttacgttaagacaat | 356602 | - | |
| 21172 | EMILIN3 | NM_024756.1 | 2832 | gagcgagtatggtttgagttaac | 390105 | - | |
| 21173 | EML5 | NM_183387.1 | 1874 | cagctcacgtaactaatgtcaga | 435903 | - | see also 10298 line |
| 21174 | EMU1 | NM_133455.1 | 119 | tggtgacccgcaccatctcatgc | 407210 | - | see also 9579 line |

Figure 46

| # | Symbol | Accession | Length | Sequence | ID | | Note | | | | | Description |
|---|--------|-----------|--------|----------|-----|---|------|---|---|---|---|-------------|
| 21175 | ENAH | NM_018212.2 | 518 | aggaccatcaggtcgtgataaac | 368483 | - | see also 7322 line | | - | | - | |
| 21176 | ENTH | NM_014666.2 | 377 | atgctttggtcacgaatgttaaa | 341964 | - | | | - | | - | |
| 21177 | EPM2AIP1 | NM_014805.2 | 290 | gttggaccagcgttatttggtgg | 343788 | - | | | - | | - | |
| 21178 | EPN2 | NM_014964.2 | 414 | atgacgacttcgtctcatcagacg | 346400 | - | | | - | | - | |
| 21179 | EPN3 | NM_017957.1 | 2016 | gggcaggccgacgctaaaccaga | 364870 | - | | | - | | - | |
| 21180 | EPPB9 | NM_015681.2 | 168 | gcgagtcctagccgctttctact | 354994 | - | | | - | | - | |
| 21181 | EPSTI1 | NM_033255.1 | 967 | gcggtaacagctggggtatatga | 403643 | - | | | - | | - | |
| 21182 | ERAS | NM_181532.2 | 506 | cagtgggactgcattctgaatg | 432385 | - | signal_transduction | | - | | - | bladder transitional cell carcinoma, bladder cancer, breast cancer, non-small-cell lung cancer |
| 21183 | ERMAP | NM_018538.2 | 1383 | cagcacctcagtcattggttca | 372178 | - | | | - | | - | |
| 21184 | ESAM | NM_138961.1 | 826 | gtgggcactgccaatgtaatgt | 409280 | - | | | - | | - | |
| 21185 | ESPN | NM_031475.1 | 455 | gagtgcaggacaaagacaattct | 397167 | - | | | - | | - | |
| 21186 | ESRRBL1 | NM_018010.2 | 810 | acgtgtactaccgcaactgaaag | 365265 | - | see also 7196 line | | - | | - | |
| 21187 | ESSPL | NM_183375.1 | 277 | gtgtggctaggatcgattacagt | 435698 | - | | | - | | - | |
| 21188 | EST1B | NM_015327.1 | 707 | atgaattagctggcgtagataacc | 351598 | - | | | - | | - | |
| 21189 | ETAA16 | NM_019002.2 | 1015 | cagaaatgtagcggacagttaag | 374300 | - | see also 7655 line | | - | | - | |
| 21190 | ETEA | NM_014613.1 | 259 | caggatctacagctatgttgtct | 341145 | - | see also 5781 line | | - | | - | |
| 21191 | ETR101 | NM_004907.1 | 137 | ctgtcggtgtggaagatgtatca | 333242 | - | | | - | | - | |
| 21192 | EVC2 | NM_147127.2 | 1672 | ctgagggcttgtcgaacacaaat | 414977 | - | | | - | | - | |
| 21193 | EVER2 | NM_152468.3 | 1517 | tggccagcttgggatgttctcc | 416988 | - | | | - | | - | |
| 21194 | EVI2B | NM_006495.2 | 1380 | cagtgtcaggagttctctattcc | 335690 | - | | | - | | - | |
| 21195 | F25965 | NM_019104.1 | 379 | ccgcaggatccaacacatatgt | 375232 | - | | | - | | - | |
| 21196 | F8A | NM_012151.2 | 129 | ccgctaccggctgtatcgaaca | 337712 | - | | ETS | - | | - | |
| 21197 | FAIM | NM_018147.1 | 134 | cggagtccacaagatcgaattg | 367676 | - | | | - | | - | |
| 21198 | FAM11A | NM_032508.1 | 970 | tcgtcgtgcccctttcacattt | 400464 | - | | | - | | - | |
| 21199 | FAM13A1 | NM_014883.1 | 1756 | caggtaccggctggtcaaacaga | 345288 | - | see also 6077 line | | - | | - | |
| 21200 | FAM13C1 | NM_198215.1 | 1359 | ccgctttatgaccgatacagaat | 437272 | - | see also 103314 line | | - | | - | |
| 21201 | FAM18B | NM_016078.1 | 791 | aggtgccaactgtatggttaca | 356217 | - | | | - | | - | |
| 21202 | FAM20A | NM_017565.1 | 1354 | tggaccggcaccattatgagatg | 359104 | - | | | - | | - | |
| 21203 | FAM20B | NM_014864.1 | 452 | gggcctttcaccgaatgatgact | 344941 | - | | | - | | - | |
| 21204 | FAM20C | NM_020223.1 | 1731 | gcggctccgccgtcgtgctaaagg | 376255 | - | | | - | | - | |
| 21205 | FAM8A1 | NM_016255.1 | 969 | tggttggcacttatatacaga | 356643 | - | | | - | | - | |
| 21206 | FANCL | NM_018062.1 | 222 | tagtggataccatcgaatagtac | 365984 | - | | | - | | - | |
| 21207 | FANK1 | NM_145235.2 | 935 | tggaaagacgccccttatggtgg | 413797 | - | | | - | | - | |
| 21208 | FAPP2 | NM_032639.2 | 1379 | cagacagccctaagaaatccaac | 401161 | - | | | - | | - | |
| 21209 | FATE | NM_033085.1 | 402 | aggcatacgtttccattatgatc | 403215 | - | | | - | | - | |
| 21210 | FBS1 | NM_022452.1 | 234 | acgtgcgtggcttacatgatc | 382832 | - | | | - | | - | |

Figure 46

| 21211 | FBX30 | NM_033182.4 | 795 | gcggcgtggacactcattactgg | 403565 | - | - | - | - |
|-------|-------|-------------|-----|-------------------------|--------|---|---|---|---|
| 21212 | FBXL12 | NM_017703.1 | 271 | cggtcctgctcgagatcttctct | 361079 | - | - | - | - |
| 21213 | FBXL8 | NM_018378.2 | 318 | ctgcctcgaccacattcacaacc | 370924 | - | - | - | - |
| 21214 | FBXO15 | NM_152676.1 | 887 | ttgaccatatctaccatgattgg | 419602 | - | - | - | - |
| 21215 | FBXO16 | NM_172366.2 | 634 | ctgacgttcaactagttacaagc | 424465 | - | - | - | - |
| 21216 | FBXO22 | NM_012170.2 | 449 | aagagtgtcgtggccataagaga | 337721 | - | see also 5175 line | | |
| 21217 | FBXO25 | NM_012173.3 | 816 | ttgccgattagaaactattctcg | 337744 | - | - | - | - |
| 21218 | FBXO27 | NM_178820.3 | 669 | acgacagcggctgtatgtacaga | 431380 | - | - | - | - |
| 21219 | FBXO32 | NM_058229.2 | 1021 | cggcagatccgcaaacgattaat | 406259 | - | - | - | - |
| 21220 | FBXO34 | NM_017943.2 | 303 | aagctagccacataacatcaagt | 364479 | - | - | - | - |
| 21221 | FBXO6 | NM_018438.2 | 533 | ttgtcacatcctacgaaatgtgc | 371546 | - | see also 7478 line | | |
| 21222 | FBXW5 | NM_018998.2 | 1334 | ctggaccacgtcatagacataca | 374101 | - | see also 7651 line | | |
| 21223 | FBXW7 | NM_018315.2 | 1471 | gggcatactaatagagtctattc | 370198 | - | see also 7394 line | | |
| 21224 | FBXW8 | NM_012174.1 | 548 | gtggtcattgcgggatatacatc | 337757 | - | - | - | - |
| 21225 | FCGBP | NM_003890.1 | 2604 | gggtccggggacccacactatgt | 332255 | - | - | - | - |
| 21226 | FEM1A | NM_018708.1 | 1009 | gggagctacgtatgtggataaga | 372734 | - | - | - | - |
| 21227 | FEZL | NM_018008.2 | 979 | gtgtttaacgctcactataatct | 365233 | - | see also 7195 line | | |
| 21228 | FGIF | NM_017704.1 | 412 | aggccactcacgtgaacactagg | 361092 | - | - | - | - |
| 21229 | FILIP1 | NM_015687.1 | 537 | gcgcaccgtatacgagttagaga | 355019 | - | - | - | - |
| 21230 | FIS | NM_175616.2 | 535 | ctgctgctccatcgcatgaattc | 429153 | - | - | - | - |
| 21231 | FKRP | NM_024301.2 | 1658 | tggcgcaggcgcctaacaactac | 386214 | - | - | - | - |
| 21232 | FKSG14 | NM_022145.2 | 1034 | aagatccaacccgaataaggatta | 382524 | - | - | - | - |
| 21233 | FKSG32 | NM_031307.2 | 273 | aagacacgtagccctaagaatag | 396661 | - | see also 9012 line | | |
| 21234 | FKSG42 | NM_032032.1 | 1671 | ctggagtcagttctcaatagtgg | 397665 | - | - | - | - |
| 21235 | FLJ00005 | NM_020447.2 | 348 | aagtttaacaagggctatactgc | 377482 | - | - | - | - |
| 21236 | FLJ00007 | NM_033449.1 | 707 | atgcccacctcgaccattactac | 404030 | - | see also 9425 line | | |
| 21237 | FLJ10006 | NM_017969.1 | 2308 | atgagtggtctaggcctatattt | 364932 | - | - | - | - |
| 21238 | FLJ10036 | NM_017975.2 | 914 | ctgcgatcgctttggatatttcc | 364981 | - | see also 7188 line | | |
| 21239 | FLJ10055 | NM_017983.3 | 140 | tggaactaaagccgggtataagc | 365034 | - | - | - | - |
| 21240 | FLJ10081 | NM_017991.3 | 458 | aaggcacgcagcgtgatgaatga | 365107 | - | - | - | - |
| 21241 | FLJ10099 | NM_017994.3 | 623 | ccgcaacgtcacccatctgtact | 365143 | - | - | - | - |
| 21242 | FLJ10103 | NM_017996.2 | 805 | gtgatacacgcacgttcaagtgt | 365175 | - | - | - | - |
| 21243 | FLJ10116 | NM_018000.1 | 463 | acgacagaaccctgtacaatttg | 365207 | - | - | - | - |
| 21244 | FLJ10154 | NM_018011.1 | 354 | gtgcggaagcgttccaaatctcg | 365280 | - | see also 7198 line | | |
| 21245 | FLJ10156 | NM_019013.1 | 328 | gggtgccgtgtcccagagaatcc | 374469 | - | - | - | - |
| 21246 | FLJ10159 | NM_018013.1 | 772 | aggaggtgcctccgaattagaaa | 365289 | - | - | - | - |
| 21247 | FLJ10178 | NM_018015.2 | 838 | tagaggccagccgtatacgtatg | 365365 | - | see also 7211 line | | |

Figure 46

| | | | | | | | | |
|---|---|---|---|---|---|---|---|---|
| 21248 | FLJ10188 | NM_018017.2 | 2170 | atgtctagaagatgaacgattaa | - | 365420 | - | |
| 21249 | FLJ10193 | NM_018019.2 | 423 | gagcaagtcaggaccaagaatga | - | 365453 | - | |
| 21250 | FLJ10199 | NM_018022.1 | 1013 | ccgctgaaagttcgaaggattaa | - | 365460 | - | |
| 21251 | FLJ10204 | NM_018024.1 | 519 | gtgatccgtgcagattcatatt | - | 365473 | - | |
| 21252 | FLJ10213 | NM_018029.3 | 895 | gaggaaaggggtactccttatgc | - | 365491 | - | |
| 21253 | FLJ10232 | NM_018033.1 | 353 | gtgcttcttggtcacttatga | - | 365524 | - | |
| 21254 | FLJ10233 | NM_018034.2 | 1271 | tgggacatccgacaatttaataa | - | 365556 | - | |
| 21255 | FLJ10241 | NM_018035.1 | 319 | tggatcaggccagataagtatgg | - | 365588 | - | |
| 21256 | FLJ10246 | NM_018038.1 | 441 | ctgtagagggctcatttcctga | - | 365682 | - | |
| 21257 | FLJ10251 | NM_018039.2 | 2156 | cagattatccaccgtcaaatg | - | 365697 | - | |
| 21258 | FLJ10260 | NM_018042.2 | 504 | agggtctcacgagctatgtgtgc | - | 365700 | - | |
| 21259 | FLJ10287 | NM_019083.1 | 52 | gagggtagatgcggttactatgt | - | 375032 | - | |
| 21260 | FLJ10298 | NM_018050.2 | 415 | gagcccgtatatacttcaactca | - | 365852 | - | |
| 21261 | FLJ10300 | NM_018051.2 | 3076 | aggacgacatccaacatctac | - | 365916 | - | |
| 21262 | FLJ10305 | NM_018052.3 | 2275 | gagtgcccatcttcacatatct | - | 365932 | - | see also 7217 line |
| 21263 | FLJ10307 | NM_018053.2 | 1191 | aggcgcatgacccatttagcaca | - | 365934 | - | |
| 21264 | FLJ10315 | NM_018056.1 | 1031 | ctgtctggttcgtgaagaacaca | - | 365935 | - | |
| 21265 | FLJ10350 | NM_018067.2 | 1427 | ccggtgacccctagccaaaccaatg | - | 366045 | - | |
| 21266 | FLJ10359 | NM_018072.3 | 421 | atgtcatgcgccctagaatgatact | - | 366123 | - | |
| 21267 | FLJ10374 | NM_018074.3 | 204 | gtgtaagacgtgcggagaatacca | - | 366187 | - | |
| 21268 | FLJ10375 | NM_018075.2 | 797 | tggccactctctaattacctcc | - | 366203 | - | see also 7232 line |
| 21269 | FLJ10385 | NM_018081.1 | 919 | ccgggagaaccgattcatatct | - | 366380 | - | |
| 21270 | FLJ10404 | NM_019057.2 | 4157 | ctgtgaactgaccaacttcagc | - | 374800 | - | |
| 21271 | FLJ10407 | NM_018087.3 | 271 | ccgcaggtgtcggagacatactgt | - | 366498 | - | see also 7249 line |
| 21272 | FLJ10415 | NM_018089.1 | 393 | ctggcatcgtttaacctaaagc | - | 366565 | - | |
| 21273 | FLJ10420 | NM_018090.3 | 322 | ggggcgacgggcgtttattggaat | - | 366578 | - | |
| 21274 | FLJ10460 | NM_018097.1 | 336 | gagatccctaggcaaagactgt | - | 366648 | - | |
| 21275 | FLJ10462 | NM_018099.3 | 1536 | ttgacgtgcgccagttgaactgg | - | 366690 | - | |
| 21276 | FLJ10490 | NM_018111.1 | 157 | ccggaaagacgcgctgtgtttgg | - | 366811 | - | |
| 21277 | FLJ10493 | NM_018112.1 | 576 | aggtggtaccattataacgaatt | - | 366830 | - | |
| 21278 | FLJ10498 | NM_018115.1 | 1770 | cagttcgacaaataaagaggaga | - | 366851 | - | |
| 21279 | FLJ10520 | NM_018124.2 | 1078 | gtggtccagacttataatgctgg | - | 366955 | - | |
| 21280 | FLJ10521 | NM_018125.2 | 2394 | ctgggtcaacagttacatttgg | - | 366979 | - | see also 7268 line |
| 21281 | FLJ10534 | NM_018128.4 | 2435 | atgtggctgctcgaattcgattt | - | 367028 | - | |
| 21282 | FLJ10539 | NM_018130.1 | 1781 | ctgctaaaccgcagcaatatt | - | 367096 | - | |
| 21283 | FLJ10546 | NM_018133.2 | 1548 | aagtactccgcagcttagaaact | - | 367177 | - | |
| 21284 | FLJ10547 | NM_018134.1 | 512 | cagccacttggccatggaattgc | - | 367206 | - | |

Figure 46

| | | | | | | | | | |
|---|---|---|---|---|---|---|---|---|---|
| 21285 | FLJ10565 | NM_018140.1 | 723 | gagcccgcagttggtacagtacc | 367592 | - | - | - | - |
| 21286 | FLJ10569 | NM_018142.1 | 1224 | aagaaatcgtcacattgtagtca | 367634 | - | see also 7273 line | | |
| 21287 | FLJ10579 | NM_018145.1 | 1065 | gtgagcgagaagaagtcatatgc | 367665 | - | - | - | - |
| 21288 | FLJ10597 | NM_016501.2 | 474 | tagctcatggggctctttataag | 357899 | - | - | - | - |
| 21289 | FLJ10652 | NM_018169.1 | 199 | aagcgagttcatctaaatttagt | 367900 | - | - | - | - |
| 21290 | FLJ10661 | NM_152563.1 | 262 | gcgggctcctcaattctacatgg | 367981 | - | - | - | - |
| 21291 | FLJ10665 | NM_018173.1 | 193 | tcgtggcctcccgcattgagact | 367982 | - | - | - | - |
| 21292 | FLJ10697 | NM_018181.3 | 1072 | cagcgttatcgtcaagaatgttc | 368066 | - | - | - | - |
| 21293 | FLJ10700 | NM_018182.1 | 1829 | cagagagtcgaagtcttcatatt | 368145 | - | see also 7307 line | | |
| 21294 | FLJ10724 | NM_018194.1 | 354 | aggatgcgaccgactttgagtgg | 368304 | - | - | - | - |
| 21295 | FLJ10737 | NM_018198.1 | 605 | ttgcgctcagacgagtaacttcg | 368347 | - | - | - | - |
| 21296 | FLJ10743 | NM_018201.2 | 98 | ctgcgggaactcagctttagtgg | 368373 | - | - | - | - |
| 21297 | FLJ10747 | NM_018202.2 | 1970 | atgcccagcataacatacagtgc | 368415 | - | - | - | - |
| 21298 | FLJ10748 | NM_018203.1 | 1172 | tggaccaagtttcccaacattcc | 368421 | - | - | - | - |
| 21299 | FLJ10781 | NM_018215.2 | 500 | gagcaccatcccccgtgaattcc | 368523 | - | - | - | - |
| 21300 | FLJ10786 | NM_018219.1 | 1379 | ctgccgtacgggtctctgataga | 368542 | - | - | - | - |
| 21301 | FLJ10817 | NM_018076.2 | 2587 | atgataattgatcgcttagatgg | 366279 | - | - | - | - |
| 21302 | FLJ10846 | NM_018241.1 | 546 | cagtggattcgacctttagtagc | 368895 | - | - | - | - |
| 21303 | FLJ10871 | NM_018250.1 | 1966 | aagaagactcgacccatatcatc | 369133 | - | - | - | - |
| 21304 | FLJ10874 | NM_018252.1 | 1169 | aggcaacgagccacataagctga | 369147 | - | - | - | - |
| 21305 | FLJ10884 | NM_019079.2 | 365 | aaggacatagctccggtattaga | 374973 | - | - | - | - |
| 21306 | FLJ10890 | NM_018259.2 | 3226 | ctggaatgacgccgtcatagtag | 369347 | - | - | - | - |
| 21307 | FLJ10901 | NM_018265.1 | 504 | caggatcggagcggcttacaaac | 369514 | - | - | - | - |
| 21308 | FLJ10902 | NM_018266.1 | 930 | tggctacccgtttggtgtttatg | 369532 | - | see also 7356 line | | |
| 21309 | FLJ10904 | NM_018268.1 | 1300 | gtgtacgcatttgggagttaaga | 369590 | - | - | - | - |
| 21310 | FLJ10922 | NM_018273.2 | 161 | tgggggagtatcgcaagatgtgg | 369618 | - | see also 7360 line | | |
| 21311 | FLJ10936 | NM_018279.2 | 823 | aggagggctagtcgttggattta | 369666 | - | - | - | - |
| 21312 | FLJ10970 | NM_018286.1 | 577 | aagagacgggagagtcaaacagc | 369698 | - | - | - | - |
| 21313 | FLJ10979 | NM_018289.2 | 222 | gagcaatctctggcgaacataga | 369778 | - | - | - | - |
| 21314 | FLJ10996 | NM_019044.2 | 1608 | tagccgtgccgagctaatacagt | 374653 | - | - | - | - |
| 21315 | FLJ10997 | NM_018293.1 | 597 | ttggttgtgtccggatatttaaa | 369817 | - | - | - | - |
| 21316 | FLJ10998 | NM_018294.3 | 1203 | ctgagacggttctttaagagtcg | 369855 | - | - | - | - |
| 21317 | FLJ11000 | NM_018295.1 | 257 | ctgctgttcatgagcatcatagt | 369860 | - | - | - | - |
| 21318 | FLJ11004 | NM_018296.2 | 1262 | ttgtcaatgtagagcaacaatta | 369883 | - | - | - | - |
| 21319 | FLJ11017 | NM_018302.1 | 763 | tggagacaacgttgatcataatg | 369979 | - | - | - | - |
| 21320 | FLJ11021 | NM_023012.3 | 1081 | tagctgttcctagctactataac | 384972 | - | - | - | - |
| 21321 | FLJ11029 | NM_018304.1 | 826 | aagcacggaatccactagttacc | 370007 | - | - | - | - |

Figure 46

| | | | | | | | |
|---|---|---|---|---|---|---|---|
| 21322 | FLJ11036 | NM_018306.2 | 717 | tggaaacggtggcatctactc | 370028 | - | - |
| 21323 | FLJ11046 | NM_018309.1 | 1790 | ctgttggttactgcaacacatat | 370082 | - | see also 7384 line |
| 21324 | FLJ11082 | NM_018317.1 | 1449 | tggatggttcgagctttctgg | 370250 | - | see also 7399 line |
| 21325 | FLJ11088 | NM_018318.2 | 391 | tggagccaatgtatctaacatac | 370261 | - | - |
| 21326 | FLJ11127 | NM_019018.1 | 957 | tggactagagcagattgatatgt | 374485 | - | - |
| 21327 | FLJ11171 | NM_018348.4 | 842 | tgggtagcgaatactctgaatcc | 370580 | - | - |
| 21328 | FLJ11184 | NM_018352.1 | 345 | ttgaaagtacttaaatcgattc | 370644 | - | - |
| 21329 | FLJ11193 | NM_018356.1 | 1184 | ctgtttaacctgcgattattca | 370682 | - | - |
| 21330 | FLJ11200 | NM_018359.1 | 110 | cagctagctactcctaatgaaat | 370689 | - | see also 7413 line |
| 21331 | FLJ11218 | NM_018363.1 | 624 | gtgagctactcctcgatatgc | 370787 | - | - |
| 21332 | FLJ11220 | NM_018364.2 | 143 | gggcgtgggccatttaaatgt | 370798 | - | see also 7417 line |
| 21333 | FLJ11235 | NM_019033.1 | 301 | cggcggaatacggcttcttcagg | 374584 | - | - |
| 21334 | FLJ11259 | NM_018370.1 | 365 | tagtgagtgcgatctgtgaatgg | 370892 | - | see also 7424 line |
| 21335 | FLJ11267 | NM_019607.1 | 659 | cagatgtaccgagtttgaaatcc | 375511 | - | - |
| 21336 | FLJ11280 | NM_018379.2 | 1358 | ctgctaaggaggggtgaacttagc | 370937 | - | - |
| 21337 | FLJ11286 | NM_018381.1 | 383 | acgtgcccaggacgacettatcc | 370961 | - | - |
| 21338 | FLJ11292 | NM_018382.1 | 190 | ctggccctttgcagtgatataaa | 370967 | - | - |
| 21339 | FLJ11301 | NM_018385.1 | 1388 | cggaatcctcccaattgatcaga | 370996 | - | - |
| 21340 | FLJ11305 | NM_018386.1 | 909 | gagcattgtcaccgttctagtca | 371023 | - | - |
| 21341 | FLJ11331 | NM_018392.3 | 891 | ctgaagtccgaatcatctagttc | 371043 | - | - |
| 21342 | FLJ11336 | NM_018393.2 | 1667 | ttgctcgctgtcaactataac | 371136 | - | see also 7437 line |
| 21343 | FLJ11362 | NM_021946.2 | 1447 | ccgagccagccgctggtatatat | 381540 | - | see also 8209 line |
| 21344 | FLJ11383 | NM_014801.2 | 1026 | ttgaccatcgtcttgtaagtcc | 343696 | - | - |
| 21345 | FLJ11457 | NM_024753.2 | 2091 | aagtgcggttaccattgctaat | 390014 | - | - |
| 21346 | FLJ11467 | NM_024963.2 | 1716 | ggggtccgggctggtcaatatcg | 392754 | - | - |
| 21347 | FLJ11506 | NM_024666.2 | 683 | ggggtggtgcatctaacacaaca | 388800 | - | - |
| 21348 | FLJ11526 | NM_024632.1 | 846 | gagcgtaaggcaactatatatct | 388326 | - | - |
| 21349 | FLJ11535 | NM_024888.1 | 1075 | gggccacgactcggtttatcagc | 391715 | - | - |
| 21350 | FLJ11560 | NM_025182.2 | 1432 | ttggagctagtggggtcatactgc | 394292 | - | see also 8874 line |
| 21351 | FLJ11712 | NM_024570.1 | 638 | gttggataacgtgtttccaaattg | 387343 | - | - |
| 21352 | FLJ11730 | NM_022756.3 | 553 | ccgtctggccatgtttgattatga | 383982 | - | - |
| 21353 | FLJ11752 | NM_152281.1 | 751 | acgctcggaagcggtttgacagg | 415315 | - | see also 9829 line |
| 21354 | FLJ11753 | NM_024659.2 | 595 | gagggatttccaatatggatdaca | 388671 | - | see also 8689 line |
| 21355 | FLJ11773 | NM_021934.3 | 945 | aggtgataacgtgtttgacaca | 381385 | - | - |
| 21356 | FLJ11785 | NM_021930.2 | 1488 | tgggtatcgcaatataaggatat | 381336 | - | see also 8200 line |
| 21357 | FLJ11795 | NM_024669.1 | 621 | aagtcgaagtgagcaggatttat | 388815 | - | - |
| 21358 | FLJ11800 | NM_024974.1 | 252 | tagcctactgtctgatgtcatca | 392760 | - | - |

Figure 46

| | | | | | | | |
|---|---|---|---|---|---|---|---|
| 21359 | FLJ11807 | NM_024954.3 | 334 | gcgggcaacggatgagttct | 392708 | - | - |
| 21360 | FLJ11848 | NM_025155.1 | 867 | ttggctcagacgctttcaactgc | 394130 | - | - |
| 21361 | FLJ11856 | NM_024531.3 | 640 | cagtggcaggacagttgcattct | 386782 | - | receptor_acitivity |
| 21362 | FLJ12057 | NM_024768.1 | 850 | aggctccggacctgaacatatc | 390317 | - | - |
| 21363 | FLJ12085 | NM_022771.3 | 1680 | tggacgaactaccatgtacaaa | 384085 | - | see also 8462 line |
| 21364 | FLJ12150 | NM_024736.4 | 888 | cagcacctcaatgaatgtgtact | 389758 | - | - |
| 21365 | FLJ12168 | NM_024682.1 | 1488 | gagctcgtgcaagggaactttga | 389127 | - | - |
| 21366 | FLJ12355 | NM_024988.1 | 471 | aggggaggagacaaacttaactct | 392776 | - | - |
| 21367 | FLJ12363 | NM_032167.1 | 65 | gagccggatcacagaaccaatgaca | 398405 | - | transcription_regulator |
| 21368 | FLJ12436 | NM_024661.2 | 260 | aagacttgrtgggacagatatga | 388708 | - | - |
| 21369 | FLJ12439 | NM_023077.1 | 134 | aaggaccggacggttgctatcg | 385243 | - | - |
| 21370 | FLJ12442 | NM_022908.1 | 935 | atggcaaggtccctccattaag | 384785 | - | - |
| 21371 | FLJ12448 | NM_022895.1 | 47 | gagcgattcgaaagtagtaaca | 384691 | - | - |
| 21372 | FLJ12505 | NM_024749.2 | 1224 | tgggctatcaaatccgaatttag | 389913 | - | - |
| 21373 | FLJ12517 | NM_023007.1 | 1122 | ctgctcgggcatcaactttgaag | 384936 | - | - |
| 21374 | FLJ12525 | NM_031206.2 | 226 | tgggaccaggtgacggtttatct | 395923 | - | see also 8988 line |
| 21375 | FLJ12541 | NM_022369.2 | 599 | ctgggactgttctatatgctgc | 382726 | - | - |
| 21376 | FLJ12571 | NM_024926.1 | 216 | ttggagttcaaagtcatgttgg | 392269 | - | see also 8808 line |
| 21377 | FLJ12572 | NM_022905.2 | 649 | ttgcgtagcactgacaagaatt | 384756 | - | - |
| 21378 | FLJ12577 | NM_030925.1 | 568 | gtgttggtagcagacttcttaga | 395522 | - | - |
| 21379 | FLJ12584 | NM_025139.2 | 1622 | gagatacagccgtatgtgaatgg | 393945 | - | see also 8868 line |
| 21380 | FLJ12598 | NM_024754.2 | 581 | aagttcaccaaagatacctatgt | 390079 | - | - |
| 21381 | FLJ12604 | NM_024621.1 | 1379 | cagcttcaccgccattgctaaac | 388093 | - | see also 8669 line |
| 21382 | FLJ12610 | NM_024782.1 | 533 | agggagctagcaaacgttacttca | 390648 | - | - |
| 21383 | FLJ12650 | NM_024522.1 | 327 | ctgcttgactacccctacattga | 386632 | - | - |
| 21384 | FLJ12666 | NM_024595.1 | 381 | aagaatatagtcgttatcagagg | 387712 | - | - |
| 21385 | FLJ12681 | NM_022773.1 | 402 | ctgggcatctcgtctttgtact | 384094 | - | - |
| 21386 | FLJ12684 | NM_024534.2 | 1140 | tggacccttcctaggtaatatac | 386852 | - | - |
| 21387 | FLJ12700 | NM_024910.1 | 469 | aagggggagtccctaagactac | 391975 | - | - |
| 21388 | FLJ12716 | NM_021942.4 | 3103 | gagtagcaacggcattatatt | 381491 | - | - |
| 21389 | FLJ12750 | NM_024667.1 | 312 | aagcacgcttgacccagaaatac | 388802 | - | - |
| 21390 | FLJ12787 | NM_032175.1 | 442 | aaggcactgatagagttcttgg | 398501 | - | see also 9086 line |
| 21391 | FLJ12788 | NM_022492.1 | 692 | aggactcactggatctatctagc | 383487 | - | - |
| 21392 | FLJ12806 | NM_022831.1 | 246 | aggcgatagacgagatcagata | 384465 | - | - |
| 21393 | FLJ12875 | NM_024544.1 | 326 | atgcgtgccttatgcttgttatag | 386941 | - | kinase_related |
| 21394 | FLJ12886 | NM_019108.1 | 728 | gttggcatcgacttcttattacc | 375250 | - | - |

Figure 46

| | | | | | | | |
|---|---|---|---|---|---|---|---|
| 21395 | FLJ12903 | NM_022753.1 | 290 | gtgagaacctactgtctgtattc | 383956 | - | |
| 21396 | FLJ12949 | NM_023008.2 | 466 | gagacatcgtcgcaaagttatgt | 384948 | - | |
| 21397 | FLJ12973 | NM_024908.1 | 494 | cgggattgtcacctacgaaagg | 391909 | - | |
| 21398 | FLJ12975 | NM_024809.2 | 2038 | gtgttggccgcagcttctatatc | 390925 | - | |
| 21399 | FLJ12994 | NM_022841.2 | 1048 | cagcggactaggcaattggtaac | 384562 | - | |
| 21400 | FLJ13031 | NM_024688.1 | 1689 | gagtcaaccacgacacacattaaa | 389281 | - | |
| 21401 | FLJ13081 | NM_024834.1 | 1351 | gtggttgcccacggaatagtacc | 391261 | - | |
| 21402 | FLJ13089 | NM_024953.2 | 294 | atgcaccgaccggagttagttac | 392605 | - | see also 8822 line |
| 21403 | FLJ13096 | NM_025000.1 | 1260 | gagtcaatgggattgtgacatatag | 392892 | - | |
| 21404 | FLJ13114 | NM_024541.1 | 183 | gtgtcggttacgcctgcattaca | 386919 | - | see also 8635 line |
| 21405 | FLJ13150 | NM_024813.1 | 843 | cagttaggcgattgcaaattaga | 390981 | - | |
| 21406 | FLJ13188 | NM_022063.1 | 597 | tagtgaacgtcctcaaatgaaa | 381780 | - | see also 8259 line |
| 21407 | FLJ13197 | NM_024614.1 | 443 | ggggtcggggagaataaactgt | 387972 | - | |
| 21408 | FLJ13215 | NM_025004.1 | 981 | ctgtaccttaccagaattatatg | 393093 | - | |
| 21409 | FLJ13231 | NM_023073.2 | 1540 | aggacgtgagggttcctttaaga | 385107 | - | |
| 21410 | FLJ13236 | NM_024902.2 | 1025 | cagctatgtgcagaaaacctttg | 391884 | - | |
| 21411 | FLJ13263 | NM_025125.2 | 349 | gcgtctcaccatcttgattgc | 393823 | - | |
| 21412 | FLJ13273 | NM_024751.1 | 804 | gagcctgttagagtgcataatga | 389926 | - | |
| 21413 | FLJ13291 | NM_032178.1 | 692 | gagctggtgaatgatgatgatcaaga | 398534 | - | |
| 21414 | FLJ13310 | NM_025118.1 | 820 | tgggtttgcatgcccaaagttta | 393810 | - | |
| 21415 | FLJ13330 | NM_025091.2 | 68 | aggaaacacactggaaatcgtgc | 393564 | - | |
| 21416 | FLJ13352 | NM_024592.1 | 758 | atgttattctcggcaatctcagg | 387689 | - | |
| 21417 | FLJ13386 | NM_025180.2 | 2173 | aggacaagcgtcggatagtataa | 394238 | - | see also 8873 line |
| 21418 | FLJ13391 | NM_032181.1 | 874 | aggagcctgaatcgctactatta | 398544 | - | |
| 21419 | FLJ13441 | NM_023924.1 | 655 | cagatacgtgtactacacaagttg | 385317 | - | |
| 21420 | FLJ13544 | NM_025008.1 | 519 | cagctcggatggattatgtgt | 393109 | - | |
| 21421 | FLJ13576 | NM_022484.2 | 1837 | tggaaccagacatgatacgtata | 383321 | - | |
| 21422 | FLJ13611 | NM_024941.1 | 754 | tggatacacgccagtacttatac | 392381 | - | see also 8813 line |
| 21423 | FLJ13614 | NM_139076.1 | 1086 | ctggtagtagtaaccaagataaa | 409355 | - | |
| 21424 | FLJ13615 | NM_025114.1 | 1696 | atgaatcgggagtatatggttta | 393765 | - | |
| 21425 | FLJ13621 | NM_025009.1 | 951 | ttgcatagtagggattggagatag | 393138 | - | |
| 21426 | FLJ13639 | NM_024705.1 | 339 | atgcaggttgcatggtcaataaa | 389444 | - | |
| 21427 | FLJ13657 | NM_024828.1 | 110 | gggagaaacgggcaaacgtagc | 391150 | - | |
| 21428 | FLJ13710 | NM_024817.1 | 1320 | gtgttggacccctctgaatgttc | 391068 | - | |
| 21429 | FLJ13725 | NM_024519.2 | 741 | gtgtgtaggcgatcagtatgaga | 386579 | - | |
| 21430 | FLJ13744 | NM_025011.1 | 532 | ctgaactccgtattcattgata | 393142 | - | |
| 21431 | FLJ13798 | NM_024773.1 | 677 | aagggcggaccatggttgttgattc | 390450 | - | |

Figure 46

| | | | | | | | | | |
|---|---|---|---|---|---|---|---|---|---|
| 21432 | FLJ13840 | NM_024746.2 | 1245 | gagtcccctcggacaatccattt | 389878 | - | - | - | - |
| 21433 | FLJ13841 | NM_024702.1 | 1406 | gagttcgaaagtccaattcctga | 389435 | - | - | - | - |
| 21434 | FLJ13868 | NM_022744.1 | 264 | tagcgtcagcccggactacttgc | 383849 | - | see also 8452 line | | |
| 21435 | FLJ13909 | NM_025108.1 | 1369 | aggagcacgtgtccttaccatcc | 393676 | - | - | - | - |
| 21436 | FLJ13910 | NM_022780.2 | 970 | tggcgctgccagctttaattaac | 384180 | - | see also 8471 line | | |
| 21437 | FLJ13912 | NM_022770.2 | 617 | agggcagaaaggactgaatgact | 384028 | - | - | - | - |
| 21438 | FLJ13941 | NM_024848.1 | 96 | cggttatggtgtctacgtatacc | 391417 | - | - | - | - |
| 21439 | FLJ13946 | NM_152275.2 | 530 | ctgtacaaggcctgcctttatcc | 415287 | - | - | - | - |
| 21440 | FLJ13955 | NM_024759.1 | 936 | aggccgtctcaggcatgatcact | 390154 | - | see also 8739 line | | |
| 21441 | FLJ13984 | NM_024770.1 | 616 | gtgctacaaatcgtttctcaaga | 390350 | - | - | - | - |
| 21442 | FLJ14001 | NM_024677.3 | 1578 | ttgctgctacctcgttgttcagg | 389043 | - | - | | |
| 21443 | FLJ14009 | NM_024760.1 | 1367 | tggtcaagggctacaacatctgg | 390168 | - | signal_transduction、tr anscription_regulator | - | |
| 21444 | FLJ14050 | NM_024869.1 | 452 | cagcaaggcttcggtgaacaaag | 391583 | - | - | - | - |
| 21445 | FLJ14054 | NM_024563.2 | 635 | tggttctacagaccctactataa | 387317 | - | - | - | - |
| 21446 | FLJ14075 | NM_024894.1 | 1172 | tggatataagactctatcacaag | 391747 | - | - | - | - |
| 21447 | FLJ14084 | NM_021637.1 | 327 | aagcattggtgcccttgaagtgg | 380946 | - | - | - | - |
| 21448 | FLJ14100 | NM_025025.1 | 1020 | ggggtgcaggacgctttctcagg | 393154 | - | - | - | - |
| 21449 | FLJ14103 | NM_024689.1 | 229 | aggactttcctcggtcttgataa | 389305 | - | see also 8696 line | | |
| 21450 | FLJ14146 | NM_024709.2 | 394 | atgtcgggaaggtcatcatcaaa | 389487 | - | - | - | - |
| 21451 | FLJ14166 | NM_024565.4 | 1105 | gagcacctcagcacgtgtattga | 387325 | - | - | - | - |
| 21452 | FLJ14299 | NM_025069.1 | 1322 | atgccgcgacccctattgcttgg | 393466 | - | - | - | - |
| 21453 | FLJ14351 | NM_024732.1 | 229 | gtgtgtaacctggcatggtttgg | 389726 | - | - | - | - |
| 21454 | FLJ14397 | NM_032779.2 | 1773 | tagtgagtatgctggtttagtgg | 401453 | - | - | - | - |
| 21455 | FLJ14399 | NM_032780.2 | 132 | gagttggagccacaaatagatgg | 401458 | - | - | - | - |
| 21456 | FLJ14464 | NM_032789.1 | 1012 | cagggtagagggattatgacaac | 401516 | - | - | - | - |
| 21457 | FLJ14503 | NM_152780.2 | 902 | aagacagctactaaagcttatcc | 421086 | - | - | - | - |
| 21458 | FLJ14525 | NM_032800.1 | 93 | gggagcgaaagcgcttcacttac | 401544 | - | - | - | - |
| 21459 | FLJ14564 | NM_032550.1 | 1155 | cggatgcccagcgctttaactgc | 400654 | - | - | - | - |
| 21460 | FLJ14566 | NM_032806.4 | 1769 | tggacagtcggcctatatccagg | 401588 | - | - | - | - |
| 21461 | FLJ14594 | NM_032808.2 | 1237 | gtgcgccacgcccgagtttgtcc | 401595 | - | - | - | - |
| 21462 | FLJ14624 | NM_032813.1 | 274 | tggtgagggccgtaaactacaat | 401603 | - | - | - | - |
| 21463 | FLJ14639 | NM_032815.2 | 267 | cggtcgcgtcccgggataacagc | 401644 | - | - | - | - |
| 21464 | FLJ14640 | NM_032816.2 | 1117 | aggtaacgtttccgattgtgaaa | 401675 | - | - | - | - |
| 21465 | FLJ14641 | NM_032817.1 | 938 | gggccttcctattatgtaaatcc | 401679 | - | - | - | - |
| 21466 | FLJ14642 | NM_032818.1 | 324 | gagctctataaccaatttgctgc | 401681 | - | - | - | - |

Figure 46

| | | | | | | | | | |
|---|---|---|---|---|---|---|---|---|---|
| 21467 | FLJ14668 | NM_032822.1 | 446 | aaggaggctctcttatcaattgg | 401684 | - | - | - | - |
| 21468 | FLJ14681 | NM_032824.1 | 962 | ttgggcctcatggatatatctct | 401696 | - | - | - | - |
| 21469 | FLJ14721 | NM_032829.1 | 255 | cggtggccagcgccatgcattcc | 401741 | - | - | - | - |
| 21470 | FLJ14775 | NM_032837.1 | 204 | aaggaatggcaacgaagaagaca | 401885 | - | - | - | - |
| 21471 | FLJ14803 | NM_032842.1 | 583 | tcgcccgtcagtggttataataa | 401942 | - | see also 9305 line | | |
| 21472 | FLJ14816 | NM_032845.1 | 324 | aggaaatggcagagtacgatttc | 401982 | - | - | - | - |
| 21473 | FLJ14825 | NM_032847.1 | 226 | cagacgacaagagtatcagaaag | 401989 | - | - | - | - |
| 21474 | FLJ14827 | NM_032848.1 | 808 | ctgtttggctcccgatctgaagg | 402003 | - | - | - | - |
| 21475 | FLJ14834 | NM_032849.2 | 715 | aaggacgtacgcgtttcttgtaa | 402012 | - | see also 9311 line | | |
| 21476 | FLJ14871 | NM_032854.1 | 822 | acgccgaacccgtgctcatttcg | 402080 | - | - | - | - |
| 21477 | FLJ14888 | NM_032856.1 | 951 | tggaacacggagccaagtagaac | 402093 | - | - | - | - |
| 21478 | FLJ14957 | NM_032866.2 | 708 | ctggtgtgacagctattcgttta | 402194 | - | - | - | - |
| 21479 | FLJ14966 | NM_032867.1 | 1426 | atgagtcggagctcctaatcatg | 402263 | - | - | - | - |
| 21480 | FLJ14981 | NM_032868.2 | 457 | tgggcctctgtcaagtacaaagg | 402270 | - | transcription_regulator | - | |
| 21481 | FLJ16030 | NM_198494.1 | 1302 | cagacatcgctcatcacttaatc | 438587 | - | - | - | - |
| 21482 | FLJ20003 | NM_017615.1 | 276 | ccgccagatccgccatcagtacc | 359587 | - | - | - | - |
| 21483 | FLJ20006 | NM_017618.1 | 900 | aagagacccggtcgcatttcatc | 359613 | - | - | - | - |
| 21484 | FLJ20010 | NM_019021.1 | 502 | cagcgtttgattgcattaggagg | 374506 | - | - | - | - |
| 21485 | FLJ20013 | NM_017621.2 | 325 | aggactatggccccaaagtcaac | 359659 | - | - | - | - |
| 21486 | FLJ20032 | NM_017628.2 | 2787 | aagcgagttcgagactcataatg | 359708 | - | - | - | - |
| 21487 | FLJ20045 | NM_017638.1 | 244 | ctggggcaccattaacatgatg | 359861 | - | see also 7062 line | | |
| 21488 | FLJ20051 | NM_019087.1 | 387 | aaggatctcaaggggtaatattt | 375124 | - | see also 7698 line | | |
| 21489 | FLJ20054 | NM_019049.1 | 293 | aagattatgggacctgttctagt | 374742 | - | - | - | - |
| 21490 | FLJ20071 | NM_017653.2 | 1889 | aggttcgctgagttctaatgatg | 360298 | - | see also 7082 line | | |
| 21491 | FLJ20073 | NM_017654.2 | 3192 | gtggagtacgcatcattcactct | 360388 | - | - | - | - |
| 21492 | FLJ20084 | NM_017659.1 | 301 | cagcgtctctggagcacttatct | 360448 | - | see also 7086 line | | |
| 21493 | FLJ20097 | NM_024553.2 | 385 | tagaggcgtatagagacaaattg | 387178 | - | see also 8641 line | | |
| 21494 | FLJ20136 | NM_017684.2 | 2029 | tggacggcgattgtataatgtac | 360640 | - | - | - | - |
| 21495 | FLJ20151 | NM_017689.1 | 3 | atgacgaagcaacatgaattagg | 360821 | - | - | - | - |
| 21496 | FLJ20152 | NM_019000.2 | 721 | gaggtatcctggactgataatgg | 374117 | - | see also 7652 line | | |
| 21497 | FLJ20156 | NM_017691.1 | 301 | ctgcgggcttcatctggttattc | 360832 | - | see also 7113 line | | |
| 21498 | FLJ20160 | NM_017694.2 | 1611 | ctgcaatacggctcgctatattt | 360997 | - | see also 7116 line | | |
| 21499 | FLJ20174 | NM_017699.1 | 2324 | atgggtcgtttcaagatagattt | 361055 | - | - | - | - |
| 21500 | FLJ20184 | NM_017700.1 | 544 | atgtggatagcttgtttagcaac | 361070 | - | - | - | - |
| 21501 | FLJ20186 | NM_017702.1 | 697 | ccgctgggctcacattcagatgt | 361077 | - | - | - | - |
| 21502 | FLJ20195 | NM_017706.3 | 160 | ccgggacactccggaagacatcg | 361096 | - | - | - | - |

Figure 46

| | | | | | | | | |
|---|---|---|---|---|---|---|---|---|
| 21503 | FLJ20200 | NM_017708.1 | 1234 | cggcactcagtgacattcttaagg | 361105 | - | | |
| 21504 | FLJ20202 | NM_017709.2 | 460 | ctgcaaagacctgacctaatct | 361116 | - | | |
| 21505 | FLJ20211 | NM_017713.1 | 430 | aggctaatgctgggaacttgaaa | 361128 | - | | |
| 21506 | FLJ20232 | NM_019008.3 | 1261 | agggtccctctggactatgtga | 374454 | | | molecular_function unknown |
| 21507 | FLJ20241 | NM_017721.2 | 1135 | gagccgccagcgcgactacaagc | 361149 | - | kinase_related | |
| 21508 | FLJ20259 | NM_017730.2 | 1832 | atgacacgggaagctgaaatgg | 361192 | - | see also 7124 line | |
| 21509 | FLJ20265 | NM_017733.2 | 1542 | cggccgttcacgtcattgtgtgc | 361239 | - | | |
| 21510 | FLJ20272 | NM_017735.2 | 2318 | gaggctgtatgcccacgtatatg | 361373 | - | see also 7125 line | |
| 21511 | FLJ20274 | NM_017736.1 | 894 | aggatccgtcacagcttaactca | 361403 | - | | |
| 21512 | FLJ20275 | NM_017737.1 | 602 | ttgaccgcctacgaatggaaatc | 361422 | - | see also 7126 line | |
| 21513 | FLJ20280 | NM_017741.1 | 634 | cagctaagccgaacattgagtag | 361434 | - | | |
| 21514 | FLJ20287 | NM_017746.1 | 1754 | cagatatcgtagtaaagtgttat | 361628 | - | see also 7139 line | |
| 21515 | FLJ20291 | NM_017748.3 | 1359 | tcgggtgaagcggaatatctact | 361667 | - | | |
| 21516 | FLJ20297 | NM_017751.1 | 917 | gttgcttcatcactattccttg | 361673 | - | | |
| 21517 | FLJ20300 | NM_017753.1 | 1219 | ctgcgtccatgaccgaagttacc | 361702 | - | see also 7140 line | |
| 21518 | FLJ20303 | NM_017755.4 | 587 | gcggcctcatcataagatcttag | 361787 | - | see also 7144 line | |
| 21519 | FLJ20308 | NM_017758.2 | 318 | atgacgtcccggacacaactataa | 361889 | - | | |
| 21520 | FLJ20309 | NM_017759.2 | 1762 | ggggtgaacagtgcgctaacaaag | 361923 | - | | |
| 21521 | FLJ20311 | NM_017760.4 | 673 | tggcgtatccatcaagcttata | 361958 | - | see also 7147 line | |
| 21522 | FLJ20313 | NM_017762.1 | 570 | aaggtatgccttgcaatagctca | 362033 | - | | |
| 21523 | FLJ20315 | NM_017763.1 | 1016 | aggagctacgggtcatttcctgc | 362074 | - | | |
| 21524 | FLJ20323 | NM_019005.1 | 791 | atgcatcgtaacctagctatatt | 374381 | - | see also 7656 line | |
| 21525 | FLJ20343 | NM_017775.2 | 805 | atggccaacttagcattatacg | 362198 | - | | |
| 21526 | FLJ20345 | NM_017777.1 | 579 | gcgggaacggcgagtgttcaagg | 362219 | - | | |
| 21527 | FLJ20356 | NM_018986.2 | 484 | ccggatcgtggtgacgtttaaga | 374071 | - | | |
| 21528 | FLJ20360 | NM_017782.2 | 704 | ttgtgctttcggtattggaagt | 362235 | - | | |
| 21529 | FLJ20364 | NM_017785.2 | 581 | gaggaactgcgcgtaatgtctga | 362381 | - | | |
| 21530 | FLJ20373 | NM_017792.1 | 558 | ctgctccacccacctaaacaca | 362450 | - | | |
| 21531 | FLJ20397 | NM_017802.2 | 1578 | ttggacgtgctgctgacaatagt | 362635 | - | | |
| 21532 | FLJ20420 | NM_017812.1 | 276 | cagcggtattctggtgcttatgg | 362659 | - | | |
| 21533 | FLJ20422 | NM_017814.1 | 1089 | ccgcatcgaagcccgtgaaatcc | 362672 | - | | |
| 21534 | FLJ20432 | NM_017819.1 | 1147 | ctgaatgccttccattagattaaa | 362742 | - | | |
| 21535 | FLJ20436 | NM_017822.1 | 577 | gagaagaagccgcgatacttaca | 362752 | - | | |
| 21536 | FLJ20445 | NM_017824.2 | 797 | tggcgcaaatactcgaataaact | 362769 | - | | |
| 21537 | FLJ20449 | NM_017826.1 | 561 | atggcaatgggcttgaattaaat | 362798 | - | | |
| 21538 | FLJ20452 | NM_017828.2 | 386 | cagcctacgcatgaatagtttgg | 362824 | - | | |

Figure 46

| | | | | | | | | | |
|---|---|---|---|---|---|---|---|---|---|
| 21539 | FLJ20457 | NM_017832.2 | 424 | cagcactctaaccgaatatgtgt | 362899 | - | - | - | - |
| 21540 | FLJ20464 | NM_017834.1 | 679 | ctgagcgcttccattaagagaag | 362943 | - | - | - | - |
| 21541 | FLJ20477 | NM_017837.2 | 1222 | ctgggatgttccactaatataca | 362958 | - | - | - | - |
| 21542 | FLJ20487 | NM_017841.1 | 481 | gcgtgccccagatcttgagtacc | 363011 | - | see also 7160 line | | |
| 21543 | FLJ20489 | NM_017842.1 | 1138 | tagcacgcagtgaggaatctttg | 363014 | - | - | - | - |
| 21544 | FLJ20502 | NM_017845.2 | 122 | ctgtgtaccaagattatcacact | 363021 | - | - | - | - |
| 21545 | FLJ20507 | NM_017849.1 | 751 | atggatcccaggtctatgtcacc | 363126 | - | - | - | - |
| 21546 | FLJ20508 | NM_017850.1 | 756 | ttgtacaagatatcctattgaat | 363141 | - | - | - | - |
| 21547 | FLJ20509 | NM_017851.1 | 868 | aagtacctcctggtgtattcaca | 363149 | - | - | - | - |
| 21548 | FLJ20512 | NM_017854.1 | 290 | atgggtcgggaagcagcatatgg | 363152 | - | - | - | - |
| 21549 | FLJ20513 | NM_017855.2 | 391 | ctgctgtagaccaaactattacc | 363165 | - | - | - | - |
| 21550 | FLJ20516 | NM_017858.1 | 49 | gtgattgacctaccagattatga | 363167 | - | see also 7162 line | | |
| 21551 | FLJ20519 | NM_017860.3 | 350 | ccggtcgtccgccattaacgagg | 363200 | - | - | - | - |
| 21552 | FLJ20522 | NM_017861.1 | 571 | aagcctcgattgtggtcaataac | 363217 | - | - | - | - |
| 21553 | FLJ20530 | NM_017864.1 | 592 | gagaccgccatccgaccttataa | 363253 | - | see also 7164 line | | |
| 21554 | FLJ20533 | NM_017866.3 | 416 | gagtctgattggccttacatttc | 363303 | - | - | - | - |
| 21555 | FLJ20534 | NM_017867.1 | 358 | ctgagttccagaccattattatc | 363318 | - | - | - | - |
| 21556 | FLJ20542 | NM_017871.3 | 558 | gtggtctacacgggtgattataa | 363331 | - | - | - | - |
| 21557 | FLJ20546 | NM_017872.2 | 314 | gtgatgagtacagctttgtgttc | 363343 | - | - | - | - |
| 21558 | FLJ20555 | NM_017877.2 | 430 | gcggggtgcagtgatcatattca | 363361 | - | - | - | - |
| 21559 | FLJ20580 | NM_017887.1 | 475 | caggagtctgtgggaatctatga | 363380 | - | - | - | - |
| 21560 | FLJ20605 | NM_017898.3 | 722 | aagcgtatagattggttcaattt | 363703 | - | - | - | - |
| 21561 | FLJ20618 | NM_017903.2 | 829 | atgattcctaccaccttactact | 363753 | - | - | - | - |
| 21562 | FLJ20625 | NM_017907.1 | 274 | gggcatggagcagcatgagtaca | 363832 | - | - | - | - |
| 21563 | FLJ20635 | NM_017911.1 | 1294 | acgcacacaatcagatactgatg | 363893 | - | - | - | - |
| 21564 | FLJ20641 | NM_017915.1 | 1677 | ttgtacggcaaactagctaaagt | 363929 | - | - | - | - |
| 21565 | FLJ20647 | NM_017918.3 | 880 | tagtcactcgacaggattatact | 363942 | - | - | - | - |
| 21566 | FLJ20668 | NM_017923.1 | 89 | aagatgccaaattatctaacttg | 364036 | - | - | - | - |
| 21567 | FLJ20694 | NM_017928.1 | 546 | tggttgggagcttgtgcataaat | 364268 | - | - | - | - |
| 21568 | FLJ20699 | NM_017931.1 | 777 | atgttggcttgtcataactattg | 364290 | - | - | - | - |
| 21569 | FLJ20701 | NM_017933.3 | 740 | ctggaaatccggccattccaagt | 364298 | - | - | - | - |
| 21570 | FLJ20716 | NM_017938.2 | 723 | ctggtgggtactacgaatacatc | 364364 | - | see also 7179 line | | |
| 21571 | FLJ20718 | NM_017939.1 | 1619 | tagtaatgtcggggttagagtga | 364405 | - | - | - | - |
| 21572 | FLJ20729 | NM_017953.2 | 1067 | tcgtgcccggaggcaaggtatta | 364741 | - | - | - | - |
| 21573 | FLJ20758 | NM_017952.4 | 829 | atggtgaagcaccgagcttatga | 364694 | - | - | - | - |
| 21574 | FLJ20772 | NM_017956.1 | 191 | tggtttacccagcgatacagaga | 364862 | - | - | - | - |
| 21575 | FLJ20811 | NM_019007.2 | 649 | cagcggccattcccctatgaaca | 374426 | - | - | - | - |

Figure 46

| 21576 | FLJ20859 | NM_022734.1 | 344 | aagacgggctaggcatcttgaga | 383757 | - | - | - | - |
|-------|----------|-------------|-----|-------------------------|--------|---|---|---|---|
| 21577 | FLJ20896 | NM_024605.2 | 906 | gtggccgtctacaatctttgtct | 387812 | - | see also 8665 line | | |
| 21578 | FLJ20898 | NM_024600.1 | 1072 | tcgggctcgtgactttctacaga | 387739 | - | - | - | - |
| 21579 | FLJ20972 | NM_025030.1 | 160 | aagaaggctcaactagaaattga | 393162 | - | - | - | - |
| 21580 | FLJ20989 | NM_023080.1 | 714 | atgaagagtttaggttcaatttc | 385262 | - | - | - | - |
| 21581 | FLJ21062 | NM_024788.2 | 2714 | aagcactcctgcccgattagtag | 390723 | - | - | - | - |
| 21582 | FLJ21069 | NM_024692.3 | 918 | cagtagatatgccgttagagatg | 389330 | - | see also 8697 line | | |
| 21583 | FLJ21075 | NM_025031.1 | 163 | tgggtgctggttcttaactctga | 393166 | - | - | - | - |
| 21584 | FLJ21103 | NM_024556.1 | 552 | atggagccctcgtattaactaca | 387231 | - | - | - | - |
| 21585 | FLJ21106 | NM_025097.1 | 1357 | ttgtgaaatccgatacttagaag | 393606 | - | see also 8860 line | | |
| 21586 | FLJ21125 | NM_024627.4 | 1387 | ctgtcggagtcagctaatcctcc | 388243 | - | - | - | - |
| 21587 | FLJ21127 | NM_024549.3 | 1280 | ggggtccggaccccagtattatt | 387168 | - | - | - | - |
| 21588 | FLJ21144 | NM_022774.1 | 1229 | aagtgccggacgtgtacctatgc | 384119 | - | - | - | - |
| 21589 | FLJ21148 | NM_024860.1 | 805 | atggctaactggcaactgattca | 391518 | - | - | - | - |
| 21590 | FLJ21159 | NM_024826.1 | 910 | acgccgaatggcatacaattaga | 391136 | - | - | - | - |
| 21591 | FLJ21168 | NM_025073.1 | 603 | aggaacttcgagaattattgtcc | 393491 | - | - | - | - |
| 21592 | FLJ21174 | NM_024863.3 | 379 | tggagcgggacataacaacttcg | 391573 | - | - | - | - |
| 21593 | FLJ21308 | NM_024615.2 | 973 | ttgcgcaggtcgccaagttatcc | 387987 | - | see also 8668 line | | |
| 21594 | FLJ21415 | NM_024738.1 | 395 | cggatgaactcggctacgtttgc | 389764 | - | transcription_regulator | - | - |
| 21595 | FLJ21477 | NM_025153.1 | 251 | gtgggcattgcggttctgaattt | 394101 | - | - | - | - |
| 21596 | FLJ21511 | NM_025087.1 | 256 | tgggaccgatgatctattacttt | 393516 | - | see also 8859 line | | |
| 21597 | FLJ21603 | NM_024762.1 | 703 | gtgagtccagtctcattaaatat | 390181 | - | - | - | - |
| 21598 | FLJ21610 | NM_022751.1 | 2259 | aaggtcgcctccgaaacatctcc | 383943 | - | - | - | - |
| 21599 | FLJ21657 | NM_022483.2 | 383 | ttgtagcactgaatctcttattt | 383257 | - | - | - | - |
| 21600 | FLJ21742 | NM_032207.1 | 1744 | ccgacacccatcgccaatcatgt | 398692 | - | see also 9098 line | | |
| 21601 | FLJ21749 | NM_025124.1 | 602 | tggagtctatcacgtgatcttca | 393818 | - | - | - | - |
| 21602 | FLJ21777 | NM_032209.1 | 1062 | acgccctgcaccctacgtaaatg | 398703 | - | - | - | - |
| 21603 | FLJ21816 | NM_024675.2 | 487 | caggaggattacctatacaaaga | 388935 | - | - | - | - |
| 21604 | FLJ21868 | NM_022769.1 | 1124 | ctggacggactcaacatgttaag | 384020 | - | - | - | - |
| 21605 | FLJ21870 | NM_023016.1 | 1007 | tggtcaagcgggacttcattacc | 385044 | - | - | - | - |
| 21606 | FLJ21901 | NM_024622.2 | 2017 | tcgtccattcagcgtattgaact | 388184 | - | - | - | - |
| 21607 | FLJ21908 | NM_024604.1 | 524 | atggatgccgatccatataatcc | 387761 | - | see also 8664 line | | |
| 21608 | FLJ21919 | NM_023015.3 | 1294 | tggtcgggatctcgtaagactac | 384995 | - | see also 8538 line | | |
| 21609 | FLJ21940 | NM_022828.2 | 1062 | tggggtattgcttcgtacattga | 384358 | - | - | - | - |
| 21610 | FLJ21941 | NM_025040.2 | 634 | aagagcgtgcagcaatgcaatgg | 393170 | - | - | - | - |
| 21611 | FLJ21945 | NM_025203.1 | 822 | gtgaagtacgctctatggataaa | 394372 | - | - | - | - |

Figure 46

| | | | | | | | |
|---|---|---|---|---|---|---|---|
| 21612 | FLJ21986 | NM_024913.3 | 687 | tggccgaagggccatactctaca | 392012 | - | |
| 21613 | FLJ22021 | NM_024535.1 | 1320 | acgcaagcgagggtttcttcttcc | 386895 | - | |
| 21614 | FLJ22054 | NM_018527.2 | 640 | aggttaccgagagacaagatacc | 372125 | - | see also 7506 line |
| 21615 | FLJ22104 | NM_022918.2 | 1344 | aggttggatccaagatacacaac | 384925 | - | see also 8536 line |
| 21616 | FLJ22160 | NM_024585.2 | 593 | aggaggtgtcccactcatcatca | 387602 | - | |
| 21617 | FLJ22169 | NM_024085.2 | 136 | tagaggctcctatagtgattca | 385784 | - | |
| 21618 | FLJ22170 | NM_025099.1 | 697 | aggatggttcatcctgcatatct | 393618 | - | |
| 21619 | FLJ22175 | NM_025161.2 | 2451 | cagtggctccttgctgagaatgc | 394194 | - | |
| 21620 | FLJ22202 | NM_024883.1 | 140 | atggccgttggaggtatagctgg | 391650 | - | |
| 21621 | FLJ22246 | NM_025232.2 | 557 | atgtgcggtggatgatgtactgg | 394488 | - | |
| 21622 | FLJ22269 | NM_032219.2 | 634 | ccgttaatgctcggtgtcttatac | 398706 | - | |
| 21623 | FLJ22301 | NM_024836.1 | 941 | cggccgtcttgacctacacttgg | 391350 | - | |
| 21624 | FLJ22318 | NM_022762.3 | 1135 | gagttaccgattgagattgaact | 384001 | - | see also 8457 line |
| 21625 | FLJ22341 | NM_024599.2 | 2220 | aggtcccagatcagttctacagg | 387734 | - | |
| 21626 | FLJ22344 | NM_024717.2 | 832 | gagcggatccctacgtgaagttcc | 389517 | - | |
| 21627 | FLJ22353 | NM_024587.1 | 792 | ccgtgactacccctacttactaca | 387611 | - | |
| 21628 | FLJ22374 | NM_032222.1 | 566 | cagacgtccgggtgaaactcc | 398720 | - | |
| 21629 | FLJ22378 | NM_025078.3 | 370 | gtggcagagcgccatcatgatcc | 393505 | - | |
| 21630 | FLJ22386 | NM_024589.1 | 866 | acgacgccctggtctacttcacc | 387617 | - | see also 8656 line |
| 21631 | FLJ22390 | NM_022746.2 | 581 | ccgagacgtcctcatcaaatagc | 383876 | - | |
| 21632 | FLJ22457 | NM_024901.3 | 867 | ctggactccacctagaacatgt | 391861 | - | |
| 21633 | FLJ22471 | NM_025140.1 | 908 | aggtccacctcatcaaagagagg | 393954 | - | |
| 21634 | FLJ22490 | NM_024790.2 | 735 | ggggcttacggtgagacatatcc | 390744 | - | |
| 21635 | FLJ22531 | NM_024650.2 | 830 | ttggtcgactccagcattatgt | 388523 | - | |
| 21636 | FLJ22555 | NM_024520.1 | 682 | ctgggttaggactcgaattaagg | 386611 | - | |
| 21637 | FLJ22557 | NM_024713.1 | 828 | aggtggttcagagaactatcatc | 389502 | - | |
| 21638 | FLJ22573 | NM_024660.1 | 287 | atgaccacggcgatttcgtaacg | 388702 | - | |
| 21639 | FLJ22609 | NM_022072.2 | 886 | gagggatactigtatacttctaca | 382157 | - | see also 8265 line |
| 21640 | FLJ22624 | NM_024808.2 | 368 | aagtcattctcgaattagataaag | 390844 | - | see also 8748 line |
| 21641 | FLJ22635 | NM_025092.1 | 387 | tgggagccgtgaggaatgctact | 393568 | - | |
| 21642 | FLJ22649 | NM_021928.1 | 555 | tggaacgtcgtaccaaatgctgg | 381277 | - | |
| 21643 | FLJ22662 | NM_024829.1 | 599 | gggacattgctccgtccttatca | 391185 | - | |
| 21644 | FLJ22684 | NM_025048.1 | 273 | aggcccagtcgaagaatatcagc | 393242 | - | |
| 21645 | FLJ22688 | NM_025129.3 | 1086 | gcgcctcctccgaaaactctata | 393827 | - | |
| 21646 | FLJ22692 | NM_025049.1 | 797 | cagggagacctgtctaacttgc | 393254 | - | |
| 21647 | FLJ22709 | NM_024578.1 | 773 | cagcgaggggctccgtgtacttct | 387440 | - | |
| 21648 | FLJ22729 | NM_024683.1 | 811 | aggtgctgagcatgaatcgaaat | 389139 | - | |

Figure 46

| 21649 | FLJ22746 | NM_024785.2 | 366 | gggccagtcctgtgaaatactgt | 390654 | - | - | - | - |
|---|---|---|---|---|---|---|---|---|---|
| 21650 | FLJ22757 | NM_024898.1 | 1895 | aagatggcaaccagacgataaga | 391781 | - | - | - | - |
| 21651 | FLJ22789 | NM_032230.1 | 1627 | aagtataagcctcgaatgaatga | 398900 | - | - | - | - |
| 21652 | FLJ22794 | NM_022074.2 | 1285 | gtgactcagttgggtacttattc | 382191 | - | - | - | - |
| 21653 | FLJ22833 | NM_022837.1 | 485 | tagggtcaacgacccacttattt | 384526 | - | see also 8490 line | | |
| 21654 | FLJ22875 | NM_032231.3 | 363 | aagaagaatatctggttattatc | 398913 | - | see also 9127 line | | |
| 21655 | FLJ22938 | NM_024676.2 | 1020 | cagagatgacattcaattccatc | 389008 | - | - | - | - |
| 21656 | FLJ22944 | NM_025145.3 | 1978 | aagaacataccggtatctacatt | 394027 | - | - | - | - |
| 21657 | FLJ22965 | NM_022101.2 | 203 | cgggaccggtcccgtgtgattga | 382288 | - | - | - | - |
| 21658 | FLJ23018 | NM_024810.1 | 833 | cagcggctaatcaagctattagt | 390942 | - | - | - | - |
| 21659 | FLJ23047 | NM_024548.2 | 262 | ctgacgttgcttcgtgtattaaa | 387080 | - | see also 8640 line | | |
| 21660 | FLJ23056 | NM_024582.1 | 83 | aggagcaagcagtttgacttacc | 387560 | - | - | - | - |
| 21661 | FLJ23058 | NM_024696.1 | 388 | ctgaatcatattccaacctatat | 389382 | - | - | - | - |
| 21662 | FLJ23059 | NM_032234.2 | 1312 | tcgtctacacggactgtagtaat | 398997 | - | - | - | - |
| 21663 | FLJ23091 | NM_024911.3 | 741 | gagggccgttactatgaatgtga | 391989 | - | see also 8804 line | | |
| 21664 | FLJ23129 | NM_024763.2 | 1019 | ctgtggttgagtcaagtagtaaa | 390211 | - | - | - | - |
| 21665 | FLJ23151 | NM_024772.2 | 2329 | atgatagcaccactaatttgaag | 390420 | - | - | - | - |
| 21666 | FLJ23186 | NM_024616.1 | 255 | gtgatcatcataggcttatgtct | 388054 | - | - | - | - |
| 21667 | FLJ23191 | NM_024574.2 | 2076 | ggggcactctgtaaagtatcaga | 387433 | - | - | - | - |
| 21668 | FLJ23221 | NM_024579.1 | 246 | gagactaccattagtcttgaaac | 387452 | - | - | - | - |
| 21669 | FLJ23231 | NM_025079.1 | 1058 | tgggatcaagtgccgattcttcc | 393508 | - | - | - | - |
| 21670 | FLJ23235 | NM_024943.1 | 671 | tggaatacccgaatgattgtata | 392405 | - | - | - | - |
| 21671 | FLJ23259 | NM_024727.1 | 1400 | gagctaatcgagctggatattag | 389717 | - | - | - | - |
| 21672 | FLJ23263 | NM_025115.1 | 1025 | agggagactttcggtgatactag | 393781 | - | see also 8865 line | | |
| 21673 | FLJ23306 | NM_024530.1 | 778 | ctgccacctgctggatcaagtgc | 386781 | - | - | - | - |
| 21674 | FLJ23311 | NM_024680.2 | 1357 | gtgtcaacgcctcagatagtaag | 389078 | - | transcription_regulator, cell_cycle | - | - |
| 21675 | FLJ23342 | NM_024631.1 | 841 | aagaggatatccgttaccattat | 388303 | - | see also 8673 line | | |
| 21676 | FLJ23403 | NM_022068.1 | 825 | gggacaccggtgctatgcaattt | 382033 | - | see also 8262 line | | |
| 21677 | FLJ23451 | NM_024766.1 | 205 | atgattgcctgcgacatgtatct | 390284 | - | - | - | - |
| 21678 | FLJ23467 | NM_024575.1 | 525 | ccgcatccgccacgtgtttgatc | 387435 | - | - | - | - |
| 21679 | FLJ23471 | NM_024723.1 | 195 | gggctaccgcgacgtgaatatct | 389559 | - | - | - | - |
| 21680 | FLJ23476 | NM_024640.2 | 640 | cagttgtccttggttattgatgg | 388428 | - | - | - | - |
| 21681 | FLJ23514 | NM_021827.1 | 1109 | gaggcgggatttgcaaatgcttc | 381207 | - | - | - | - |
| 21682 | FLJ23518 | NM_024725.2 | 789 | atgacagcgatatcctagtttaga | 389594 | - | - | - | - |
| 21683 | FLJ23550 | NM_025063.1 | 697 | ttgcacggtgtcagaacgatatt | 393411 | - | - | - | - |
| 21684 | FLJ23560 | NM_024685.2 | 502 | atggaccagtacctaagtagaca | 389154 | - | - | - | - |

Figure 46

| | | | | | | |
|---|---|---|---|---|---|---|
| 21685 | FLJ23584 | NM_024588.2 | 718 | aggggacaggcctcaagaagagc | 387615 | - |
| 21686 | FLJ23594 | NM_024781.1 | 708 | cagacaataccaggcaaatattg | 390634 | - |
| 21687 | FLJ23654 | NM_152550.1 | 1273 | gtgggacaggtcagcaacttatca | 417992 | - see also 9880 line |
| 21688 | FLJ23657 | NM_178497.2 | 359 | ccgttacttacttataggtatt | 430895 | - |
| 21689 | FLJ23703 | NM_182534.1 | 83 | ttgtaacatgcgggcatttctcc | 434210 | - |
| 21690 | FLJ23825 | NM_173620.1 | 1067 | ttgcttctacgcggaggatttga | 426589 | - |
| 21691 | FLJ23878 | NM_144990.1 | 503 | gggaaggaactgctattgagtga | 412464 | - |
| 21692 | FLJ25045 | NM_178537.3 | 2102 | acgacctgcgatgcaacgtttcg | 431141 | - |
| 21693 | FLJ25056 | NM_182530.1 | 236 | acgatagttgccctctatatact | 434181 | - |
| 21694 | FLJ25067 | NM_152504.2 | 156 | gtgttgacataagagattacgtcc | 417285 | - |
| 21695 | FLJ25102 | NM_182626.1 | 729 | caggtgacccaccattctcaga | 434771 | - |
| 21696 | FLJ25124 | NM_144698.2 | 232 | tggccagtcccgtttcatctgg | 411465 | - |
| 21697 | FLJ25143 | NM_182500.1 | 556 | tgagtttcctgaaagactatga | 433969 | - |
| 21698 | FLJ25161 | NM_182522.2 | 508 | ctgtcgcactggctctttctagc | 434149 | - see also 10267 line |
| 21699 | FLJ25168 | NM_152466.1 | 473 | tgggttatcagggctcacatact | 416981 | - |
| 21700 | FLJ25224 | NM_182495.2 | 762 | ctgtgtgaggcctcaacatrgc | 433918 | - |
| 21701 | FLJ25286 | NM_152546.1 | 598 | ttggcgaagaaaccaatacataa | 417890 | - see also 9879 line |
| 21702 | FLJ25323 | NM_198521.1 | 164 | cagaaatcccttgctatattcc | 439083 | - |
| 21703 | FLJ25328 | NM_152483.1 | 780 | tggagcgactgcgggaacttcact | 417053 | - |
| 21704 | FLJ25333 | NM_152548.1 | 903 | gggattatcgccacgaaatgaac | 417924 | - |
| 21705 | FLJ25351 | NM_152525.3 | 529 | aagttcgatgaagtgaagtattt | 417626 | - |
| 21706 | FLJ25359 | NM_144587.1 | 1414 | aggagaatacaacttattcgaaa | 410054 | - |
| 21707 | FLJ25371 | NM_152543.1 | 555 | ttgttaggcagtgtaagtcatca | 417855 | - |
| 21708 | FLJ25378 | NM_152768.1 | 460 | gagctgccggaagacatctacc | 420892 | - |
| 21709 | FLJ25393 | NM_181645.1 | 799 | gagttacagtcacgtgatgatct | 432668 | - |
| 21710 | FLJ25409 | NM_144652.1 | 1069 | aagcccaagccgattgagataac | 410856 | - |
| 21711 | FLJ25414 | NM_152343.1 | 157 | gagatgcgagatgacttaagtca | 415722 | - |
| 21712 | FLJ25416 | NM_145018.2 | 1908 | gagagtgaccattctagtctaaa | 412719 | - |
| 21713 | FLJ25421 | NM_152512.3 | 1919 | acgtgcgatcgctagattacatg | 417382 | - |
| 21714 | FLJ25422 | NM_145000.2 | 697 | aagcgttgtacggtcttcatcttt | 412548 | - |
| 21715 | FLJ25429 | NM_152376.2 | 253 | tgggaggtgtgcgctcatcatata | 415964 | - |
| 21716 | FLJ25436 | NM_144581.1 | 876 | ccgaattgccttacagttacaca | 409951 | - |
| 21717 | FLJ25444 | NM_152761.1 | 1098 | aagcaccagctacggattgtttc | 420805 | - |
| 21718 | FLJ25476 | NM_152493.2 | 843 | aagacgtacagtgttaaggtatg | 417104 | - |
| 21719 | FLJ25477 | NM_152704.2 | 1388 | gagtggacgacacctatctaca | 419904 | - |
| 21720 | FLJ25530 | NM_152722.3 | 463 | ctgtagatgtgcccatttcgagg | 420084 | - see also 9931 line |
| 21721 | FLJ25534 | NM_153234.3 | 579 | cagtgttggggcctatcactaca | 422431 | - |

Figure 46

| | | | | | | | | | |
|---|---|---|---|---|---|---|---|---|---|
| 21722 | FLJ25555 | NM_152345.3 | 1399 | tggcttcccagttaagattgaaa | 415740 | - | - | - | - |
| 21723 | FLJ25621 | NM_198514.1 | 344 | gagaacaacaacccattagttcc | 438889 | - | see also 10334 line | | |
| 21724 | FLJ25660 | NM_152481.1 | 380 | atgtcaccaccctgcatataaca | 417034 | - | - | - | - |
| 21725 | FLJ25694 | NM_173604.1 | 821 | cggacccgtccgatagtaaatc | 426480 | - | - | - | - |
| 21726 | FLJ25735 | NM_152577.1 | 570 | cagggaccatctaagctatattc | 418186 | - | - | - | - |
| 21727 | FLJ25736 | NM_152578.1 | 740 | cagagttgtaacgggtttgaaga | 418230 | - | - | - | - |
| 21728 | FLJ25770 | NM_178555.2 | 1576 | cagtctccgccaatagaaacaac | 431261 | - | see also 10196 line | | |
| 21729 | FLJ25773 | NM_182560.1 | 734 | ttgtgctacaccaagatttaagc | 434451 | - | - | - | - |
| 21730 | FLJ25791 | NM_173559.1 | 684 | gggaaccgatacttcttactagt | 426079 | - | - | - | - |
| 21731 | FLJ25801 | NM_173553.1 | 1013 | gcgaacacgggcagatatcattc | 426003 | - | - | - | - |
| 21732 | FLJ25851 | NM_174927.1 | 405 | atggagccacactcgtatcttcc | 428717 | - | - | - | - |
| 21733 | FLJ25954 | NM_173572.1 | 729 | ctgttatggttcgtcatgaatta | 426275 | - | - | - | - |
| 21734 | FLJ25955 | NM_178821.1 | 104 | cggtattacccgccaggaattat | 431387 | - | - | - | - |
| 21735 | FLJ25972 | NM_178822.3 | 2751 | aagtagacccccaataacagtaa | 431499 | - | receptor_acitivity | | - |
| 21736 | FLJ25976 | NM_174943.2 | 664 | gagaacatgatcttaaccatact | 428771 | - | - | - | - |
| 21737 | FLJ25989 | NM_198512.1 | 152 | ttgcaatggatcccagtctatat | 438858 | - | - | - | - |
| 21738 | FLJ26016 | NM_198472.1 | 101 | tcgttcttgcggacttgaacttc | 438404 | - | - | - | - |
| 21739 | FLJ30046 | NM_144595.2 | 657 | ccggtcaccgtgcgaaatagtca | 410119 | - | - | - | - |
| 21740 | FLJ30213 | NM_145008.1 | 602 | atggccgagcctacctgtttaac | 412590 | - | transcription_regulator | | - |
| 21741 | FLJ30277 | NM_153008.3 | 967 | ctgttgcataggcatttatttat | 421814 | - | - | - | - |
| 21742 | FLJ30294 | NM_144632.2 | 149 | gagactaaatatcgctatcttct | 410593 | - | - | - | - |
| 21743 | FLJ30373 | NM_152479.1 | 223 | ctgctatcgagagaagaagttcc | 417017 | - | - | - | - |
| 21744 | FLJ30430 | NM_153009.1 | 441 | ccggctgtcacggccgtttatca | 421818 | - | - | - | - |
| 21745 | FLJ30469 | NM_182573.1 | 333 | gcggctgcacaactgacaaatgc | 434483 | - | kinase_related、recept or_acitivity | | - |
| 21746 | FLJ30507 | NM_153010.3 | 475 | cagcgtcttcttcaaggtatttc | 421821 | - | - | - | - |
| 21747 | FLJ30525 | NM_144584.1 | 485 | gagacggcaattcagtttaaacc | 409977 | - | - | - | - |
| 21748 | FLJ30634 | NM_153014.1 | 796 | tgggtaaggatgaagtgttaaag | 421856 | - | - | - | - |
| 21749 | FLJ30655 | NM_144643.1 | 1616 | ttgtacaagatgctaccataaga | 410667 | - | - | - | - |
| 21750 | FLJ30668 | NM_153015.1 | 877 | gagctctatcgtcgaaacagatt | 421878 | - | - | - | - |
| 21751 | FLJ30672 | NM_153016.2 | 479 | aagctttggcagcttacacatgg | 421883 | - | - | - | - |
| 21752 | FLJ30794 | NM_152527.1 | 1187 | cagccgtgcattcgaattataga | 417683 | - | - | - | - |
| 21753 | FLJ30834 | NM_152399.1 | 550 | acggggcatgccagttcaaatcc | 416272 | - | - | - | - |
| 21754 | FLJ30851 | NM_198553.1 | 616 | aagaccaggatgggtcttgttct | 439475 | - | see also 10339 line | | |
| 21755 | FLJ30973 | NM_152451.1 | 440 | aagcgaatgtatggagactatga | 416918 | - | - | - | - |
| 21756 | FLJ30990 | NM_152517.1 | 1739 | atgcttcgtgatagtgttattca | 417461 | - | - | - | - |

Figure 46

| | | | | | | | | | |
|---|---|---|---|---|---|---|---|---|---|
| 21757 | FLJ31031 | NM_182533.1 | 550 | cagaagacgtgacgtgtctttca | 434207 | - | - | - | - |
| 21758 | FLJ31051 | NM_153687.1 | 678 | ttggaagatagcacactaagaaa | 423512 | - | - | - | - |
| 21759 | FLJ31052 | NM_152378.1 | 509 | aggcgcagcgtctcctattctag | 415985 | - | - | - | - |
| 21760 | FLJ31153 | NM_144600.1 | 484 | aagtactaacattgaagatcttc | 410218 | - | - | - | - |
| 21761 | FLJ31164 | NM_145003.1 | 213 | atgttggaccgagtatggaatcc | 412579 | - | - | - | - |
| 21762 | FLJ31166 | NM_153022.1 | 755 | gtgaccgtcattgctttcgatca | 421886 | - | - | - | - |
| 21763 | FLJ31265 | NM_152395.1 | 361 | tggtgcgagtgcccctgtatacc | 416255 | - | - | - | - |
| 21764 | FLJ31338 | NM_152469.1 | 1135 | tggactgtgcaggcatctgttct | 416997 | - | - | - | - |
| 21765 | FLJ31340 | NM_152748.2 | 1634 | caggaccattcggtttgctatag | 420478 | - | see also 9942 line | | |
| 21766 | FLJ31434 | NM_152496.1 | 804 | gaggcccgagatcgtttccatta | 417135 | - | - | - | - |
| 21767 | FLJ31438 | NM_152385.1 | 521 | aaggtaatagagcatatcactgc | 416069 | - | - | - | - |
| 21768 | FLJ31461 | NM_152454.1 | 209 | ttgtgatgctttgctacatcatt | 416955 | - | - | - | - |
| 21769 | FLJ31528 | NM_144679.1 | 112 | gtgtccgggctacctgtttgaag | 411284 | - | - | - | - |
| 21770 | FLJ31564 | NM_144720.1 | 1802 | cagaaacgttgtccgaaacatcc | 411784 | - | see also 9739 line | | |
| 21771 | FLJ31568 | NM_152509.1 | 873 | ctgtgctacagctctacagatga | 417317 | - | - | - | - |
| 21772 | FLJ31606 | NM_153025.1 | 46 | aagaagggatccacctattcagt | 421890 | - | - | - | - |
| 21773 | FLJ31737 | NM_144984.1 | 196 | ctggccgtgcgctggttctttgc | 412434 | - | - | - | - |
| 21774 | FLJ31751 | NM_152652.1 | 225 | ttggctccagatcatgaagtagg | 419511 | - | - | - | - |
| 21775 | FLJ31795 | NM_144609.1 | 112 | gtggaccgagccgtttatggagc | 410283 | - | - | - | - |
| 21776 | FLJ31810 | NM_152570.1 | 651 | atgcttcacacggccatatcatg | 418112 | - | see also 9884 line | | |
| 21777 | FLJ31818 | NM_152556.1 | 398 | gagggcgaaggtcgtattgaatg | 418075 | - | see also 9881 line | | |
| 21778 | FLJ31842 | NM_152487.1 | 713 | aagctatcgttatcaatggaata | 417074 | - | see also 9878 line | | |
| 21779 | FLJ31846 | NM_144974.1 | 792 | gggaaccgaataacacaagtaca | 412165 | - | - | - | - |
| 21780 | FLJ31951 | NM_144726.1 | 1672 | tcgttacccattgctacgaaaga | 411850 | - | receptor_acitivity | - | - |
| 21781 | FLJ31952 | NM_144682.2 | 1243 | aagagttgtatggctatctctgt | 411295 | - | - | - | - |
| 21782 | FLJ32000 | NM_152498.1 | 409 | aagcgcaaagttcttaataattt | 417166 | - | kinase_related | - | - |
| 21783 | FLJ32001 | NM_152609.1 | 851 | cagctagaacgattgtatcatga | 418774 | - | see also 9899 line | | |
| 21784 | FLJ32011 | NM_182516.1 | 572 | ggggtccaaggccctgactatga | 434053 | - | - | - | - |
| 21785 | FLJ32028 | NM_152680.1 | 452 | cagagtgctttacagacatatga | 419693 | - | - | - | - |
| 21786 | FLJ32063 | NM_153031.1 | 779 | caggtaatgcgcttcattattct | 421979 | - | - | - | - |
| 21787 | FLJ32110 | NM_181646.2 | 1082 | cggtccaccatgccaaatcgaca | 432685 | - | - | - | - |
| 21788 | FLJ32112 | NM_153035.1 | 276 | aagcaggccacgattgaatctct | 421994 | - | transcription_regulator | - | - |
| 21789 | FLJ32206 | NM_152497.1 | 855 | aagaccttctccgcatagattct | 417145 | - | - | - | - |
| 21790 | FLJ32252 | NM_182510.1 | 1131 | gaggatgcgctgccctaaactgc | 434032 | - | - | - | - |
| 21791 | FLJ32312 | NM_144709.1 | 1449 | gcgagctcgcgtcattcacttca | 411549 | - | - | - | - |
| 21792 | FLJ32334 | NM_144565.1 | 220 | tcgggtggtgaccagcttattca | 409679 | - | - | - | - |

Figure 46

| | | | | | | | |
|---|---|---|---|---|---|---|---|
| 21793 | FLJ32343 | NM_152434.1 | 437 | aggacacatgtagccgaaagatat | 416770 | - | |
| 21794 | FLJ32363 | NM_198566.1 | 2053 | gtggtatcctaaaccagatttct | 439663 | - | see also 10343 line |
| 21795 | FLJ32384 | NM_144608.1 | 614 | ccgggtccgcgaagagatgttcg | 410280 | - | |
| 21796 | FLJ32416 | NM_144616.2 | 394 | gagacctgcgtcaacaagtgc | 410342 | - | |
| 21797 | FLJ32421 | NM_144695.1 | 638 | aagcaagttacgatatattcaa | 411347 | - | |
| 21798 | FLJ32440 | NM_173685.1 | 438 | ttggatcggcaactaaaccatta | 427402 | - | see also 10110 line |
| 21799 | FLJ32447 | NM_153038.1 | 535 | cagccaactaaacggagtaaacg | 422027 | - | |
| 21800 | FLJ32549 | NM_152440.1 | 1128 | atgacggaccgcacatctgatct | 416829 | - | see also 9866 line |
| 21801 | FLJ32642 | NM_152415.1 | 397 | cagtatagccgaaatacagaaag | 416445 | - | |
| 21802 | FLJ32670 | NM_144612.3 | 1387 | tgggtccttacggcaagtctaca | 410320 | - | |
| 21803 | FLJ32675 | NM_173811.2 | 540 | gcgtctatgagtcgttgttgc | 428228 | - | |
| 21804 | FLJ32682 | NM_182542.1 | 1055 | tggctccggaagagcatgttaac | 434294 | - | |
| 21805 | FLJ32702 | NM_152337.1 | 332 | cagtgacattacgcttgaacaag | 415702 | - | |
| 21806 | FLJ32734 | NM_144681.1 | 736 | aggtgattgcacgctacaagacg | 411288 | - | |
| 21807 | FLJ32742 | NM_152580.1 | 370 | ttggtttcccaatctcttgtaaa | 418246 | - | |
| 21808 | FLJ32743 | NM_145020.2 | 855 | aggcacgccttgtggaaagtaac | 412770 | - | |
| 21809 | FLJ32745 | NM_144978.1 | 1657 | caggctgttccagtaatattaag | 412255 | - | |
| 21810 | FLJ32762 | NM_145023.3 | 1789 | atgcaagtgggtaatagtcaaac | 412836 | - | |
| 21811 | FLJ32771 | NM_145017.1 | 146 | gggggtcgtctcccttatgtga | 412666 | - | |
| 21812 | FLJ32779 | NM_173521.1 | 1183 | cagctaatgaatactacatgatg | 425420 | - | |
| 21813 | FLJ32783 | NM_144968.1 | 568 | gggcttccaacctatcttagtta | 412005 | - | |
| 21814 | FLJ32784 | NM_144623.1 | 1121 | cagacgatgcttggagagaagattc | 410444 | - | |
| 21815 | FLJ32785 | NM_152494.1 | 399 | ctgtgccagcctcctactagtac | 417109 | - | |
| 21816 | FLJ32786 | NM_144648.1 | 91 | tggagcccgatcgttacagttc | 410728 | - | kinase_related |
| 21817 | FLJ32796 | NM_153606.1 | 508 | gggggagtacgatatattcaagt | 423292 | - | |
| 21818 | FLJ32800 | NM_152647.1 | 1400 | aagtcgcactatagtagtactcgg | 419494 | - | |
| 21819 | FLJ32825 | NM_152492.1 | 660 | gagcatctgcgagttcgattact | 417087 | - | |
| 21820 | FLJ32827 | NM_173081.1 | 1608 | gagctgacggccaatgaattatg | 424665 | - | |
| 21821 | FLJ32830 | NM_152781.1 | 771 | ctgggttgcctgaataagcatgt | 421110 | - | |
| 21822 | FLJ32831 | NM_153040.1 | 538 | gggaaggggtcccacctaatagg | 422033 | - | |
| 21823 | FLJ32844 | NM_173543.1 | 1210 | aagcagaactctacactagaaga | 425756 | - | |
| 21824 | FLJ32855 | NM_182791.1 | 169 | aaggagaggaaccgcatactgagg | 435289 | - | |
| 21825 | FLJ32859 | NM_152539.1 | 473 | cagactgaacgaagattacctac | 417775 | - | kinase_related |
| 21826 | FLJ32880 | NM_152762.1 | 1105 | atgcctcggatacgatgaaact | 420822 | - | |
| 21827 | FLJ32884 | NM_144702.1 | 1482 | tcgccacggtgcagtatcagatg | 411488 | - | |
| 21828 | FLJ32915 | NM_145014.1 | 868 | atgagtcgattatcagtgaatca | 412630 | - | |
| 21829 | FLJ32926 | NM_144577.1 | 1273 | gcggaggccacgcgtctgattcg | 409908 | - | |

Figure 46

| | | | | | | |
|---|---|---|---|---|---|---|
| 21830 | FLJ32934 | NM_144622.1 | 1568 | cagcactggctacatagtcattg | 410430 | - |
| 21831 | FLJ32940 | NM_144696.2 | 2032 | ttggagctagatgcgattgaact | 411437 | - |
| 21832 | FLJ32942 | NM_144594.1 | 225 | aggttcctcgagctgaaattagt | 410104 | - |
| 21833 | FLJ32949 | NM_173812.3 | 273 | gaggatccaaagtctgaaagagc | 428241 | - |
| 21834 | FLJ32954 | NM_144713.1 | 1303 | tggcgatttgctcgtgcttatgg | 411658 | - |
| 21835 | FLJ32955 | NM_153041.1 | 340 | aagttgcatactatatatacatc | 422048 | - |
| 21836 | FLJ32965 | NM_182506.1 | 1243 | gttgcacgttccaaggttaagtcc | 434012 | - |
| 21837 | FLJ33008 | NM_152449.2 | 1011 | tggttgagaccctaatagcttga | 416889 | - |
| 21838 | FLJ33069 | NM_144649.1 | 251 | ctgtgattcctgatggttacc | 410813 | see also 9725 line |
| 21839 | FLJ33084 | NM_152500.1 | 1821 | tggctaacggccagccttattg | 417229 | - |
| 21840 | FLJ33167 | NM_152683.1 | 935 | aggaaagctggacatcgaattca | 419766 | - |
| 21841 | FLJ33207 | NM_178554.2 | 472 | gggatcgatctagcctgaaatcc | 431210 | - |
| 21842 | FLJ33215 | NM_148894.1 | 1391 | aggaagttgtaacgtctgatagc | 415149 | - |
| 21843 | FLJ33298 | NM_173636.1 | 1248 | gggatgtcaaggacatcaataga | 426928 | - |
| 21844 | FLJ33387 | NM_182526.1 | 367 | gagcgtgtgggccctcttcatct | 434167 | - |
| 21845 | FLJ33496 | NM_182587.1 | 837 | ceggacctgaatcaccaaatca | 434540 | - |
| 21846 | FLJ33516 | NM_152423.2 | 1832 | ttgactggtgcatctcactaatt | 416578 | - |
| 21847 | FLJ33534 | NM_182586.1 | 398 | tgggtggccagccgaatgcatta | 434526 | - |
| 21848 | FLJ33590 | NM_173821.1 | 1522 | ctgcttctcccaaggctattacc | 428336 | - |
| 21849 | FLJ33600 | NM_198478.1 | 973 | gagccgcgacgacctgttgtacc | 438452 | - |
| 21850 | FLJ33620 | NM_178824.3 | 1729 | gaggaccgaataagttccttaga | 431761 | - |
| 21851 | FLJ33641 | NM_152687.1 | 604 | aggcatgaaatctcagttgaaac | 419808 | - |
| 21852 | FLJ33651 | NM_182590.1 | 107 | gcggagaaactccctcaacatcc | 434557 | - |
| 21853 | FLJ33706 | NM_182584.1 | 677 | aagttgcctgcaccttttaattag | 434518 | - |
| 21854 | FLJ33708 | NM_173675.1 | 300 | caggtgatgtgtccttgttcttt | 427313 | - |
| 21855 | FLJ33710 | NM_198480.1 | 626 | aagagtactctcagtatacatca | 438466 | - |
| 21856 | FLJ33718 | NM_173660.2 | 1079 | ctggcagccactcctcttactcc | 427140 | - |
| 21857 | FLJ33761 | NM_153211.1 | 1074 | acgactagagtgtcaaatcaaca | 422167 | see also 9976 line |
| 21858 | FLJ33768 | NM_173610.1 | 328 | atgaaggaggttactaacttt | 426499 | - |
| 21859 | FLJ33790 | NM_173583.1 | 465 | cggctacactcgctcagaattg | 426299 | - |
| 21860 | FLJ33810 | NM_182558.1 | 542 | gagttggacagacggctttaattg | 434448 | - |
| 21861 | FLJ33814 | NM_173510.1 | 437 | ttgtgcacatcaggattgtagagg | 425232 | see also 10077 line |
| 21862 | FLJ33817 | NM_152348.1 | 717 | cagaagatcctccttgatacagc | 415756 | - |
| 21863 | FLJ33860 | NM_173644.1 | 373 | aagttcaacacttcaaacaaca | 426940 | - |
| 21864 | FLJ33915 | NM_182613.1 | 1565 | cagcgacctaacaatacatatg | 434682 | - |
| 21865 | FLJ33962 | NM_153213.2 | 1800 | caggagtgcaatgctagttgtaca | 422203 | - |
| 21866 | FLJ33977 | NM_173666.1 | 671 | cggatgcagccgactaatagatg | 427234 | - |

854

Figure 46

| | | | | | | | |
|---|---|---|---|---|---|---|---|
| 21867 | FLJ34047 | NM_173669.1 | 543 | ggggggacacttggtttagcaac | 427263 | - | |
| 21868 | FLJ34064 | NM_152633.1 | 1583 | aagatatgtatcgtgtaataga | 419365 | - | |
| 21869 | FLJ34154 | NM_173813.2 | 2072 | tgggcgtgctagaccttcaattta | 428303 | - | |
| 21870 | FLJ34278 | NM_173602.1 | 440 | atgaacgatatcgatcagatatc | 426459 | - | |
| 21871 | FLJ34443 | NM_175918.2 | 1711 | cagacgctgttaccgtacattct | 429566 | - | |
| 21872 | FLJ34503 | NM_173673.1 | 303 | atgctctcaagcctgtttcaga | 427304 | - | |
| 21873 | FLJ34512 | NM_173476.2 | 507 | tggtcgacattcagatcacaacc | 424944 | - | |
| 21874 | FLJ34583 | NM_198276.1 | 634 | aagcgatacatatcatcttcact | 437433 | - | |
| 21875 | FLJ34633 | NM_152365.1 | 985 | gcggcatcattcgcattagtacc | 415822 | - | |
| 21876 | FLJ34690 | NM_182567.1 | 481 | aggtgaaattcggacatttgtct | 434480 | - | |
| 21877 | FLJ34766 | NM_178502.2 | 867 | tggagaagtgccggcattcattc | 430919 | - | |
| 21878 | FLJ34780 | NM_173651.1 | 80 | aagaacaagcctattcctaatg | 427065 | - | |
| 21879 | FLJ34790 | NM_173621.1 | 934 | ttgattgacacgcgtcaatctat | 426602 | - | |
| 21880 | FLJ34836 | NM_173668.1 | 460 | cagtccgtaggcacttgctttaa | 427252 | - | |
| 21881 | FLJ34922 | NM_152270.1 | 2592 | gtgttcaggggaaacttacgaatt | 415251 | - | |
| 21882 | FLJ34969 | NM_152678.1 | 285 | cagccgtggagtaatttatcc | 419631 | - | see also 9911 line |
| 21883 | FLJ35093 | NM_198549.1 | 1752 | gagaagtgcgctattcaagtac | 439409 | - | |
| 21884 | FLJ35119 | NM_175871.2 | 623 | tgggtgacttccgatcagatgg | 429380 | - | |
| 21885 | FLJ35220 | NM_173627.2 | 634 | cagcaccaggcccctacatct | 426638 | - | |
| 21886 | FLJ35283 | NM_152702.1 | 506 | atcgttggtcagccaattgtcact | 419895 | - | |
| 21887 | FLJ35424 | NM_173661.1 | 705 | atgcaattactgatacatctaag | 427150 | - | |
| 21888 | FLJ35453 | NM_152600.1 | 1530 | ccgctgccctcgcaaacattaa | 418730 | - | |
| 21889 | FLJ35487 | NM_173820.1 | 168 | cagccaggaccgaccacatttaga | 428325 | - | |
| 21890 | FLJ35709 | NM_173589.1 | 1311 | ccgcctggttgactacatgattt | 426343 | - | |
| 21891 | FLJ35721 | NM_173684.1 | 178 | atggacatcctccttaccttca | 427389 | - | |
| 21892 | FLJ35725 | NM_152544.1 | 232 | atgcaatcacacccaatgataag | 417860 | - | |
| 21893 | FLJ35728 | NM_152610.1 | 692 | cagcgaccaggttgagtcattag | 418809 | - | |
| 21894 | FLJ35757 | NM_152598.1 | 2247 | ttgtgtcatctgtcagatagc | 418691 | - | |
| 21895 | FLJ35773 | NM_152599.1 | 491 | tcgggtgcacttccttgtaatg | 418704 | - | |
| 21896 | FLJ35775 | NM_152418.1 | 576 | cagcgtcgctgttcataggtagc | 416499 | - | |
| 21897 | FLJ35779 | NM_152408.1 | 1371 | atgtatgtgccaagagttgtaac | 416412 | - | see also 9859 line |
| 21898 | FLJ35782 | NM_152631.1 | 1642 | cagcgtgggaggataaggtatgg | 419226 | - | |
| 21899 | FLJ35794 | NM_152617.2 | 628 | gtgtaaccaccgctgtgtaaac | 418898 | - | see also 9903 line |
| 21900 | FLJ35801 | NM_153044.1 | 975 | caggttagttgtacaaagtcaca | 422057 | - | |
| 21901 | FLJ35802 | NM_173538.1 | 207 | cagccggagtatgagcaatatct | 425705 | - | |
| 21902 | FLJ35821 | NM_152589.1 | 1120 | atgagttatcaccgcaataataa | 418445 | - | |
| 21903 | FLJ35827 | NM_153265.1 | 2661 | ctggactgacttcgagacattcc | 422757 | - | see also 9983 line |

Figure 46

| 21904 | FLJ35834 | NM_178827.3 | 2289 | agggatatgaacgctcatttatt | 431943 | - | - | - | - |
|---|---|---|---|---|---|---|---|---|---|
| 21905 | FLJ35838 | NM_173532.1 | 752 | gtgcactggcgatccactaatga | 425580 | - | - | - | - |
| 21906 | FLJ35843 | NM_152591.1 | 1196 | ttgctcggttcacgtatgtcacg | 418505 | - | - | - | - |
| 21907 | FLJ35866 | NM_178828.3 | 2676 | tgggtatctcgggttgaatctgt | 431967 | - | - | - | - |
| 21908 | FLJ35880 | NM_153264.2 | 651 | ctgggctctacacgtaaggatga | 422714 | - | - | - | - |
| 21909 | FLJ35894 | NM_173616.1 | 992 | tgggcggactggatgtattgtct | 426551 | - | see also 10089 line | | |
| 21910 | FLJ35961 | NM_152372.2 | 2321 | cagccgctgagctacatctaatc | 415928 | - | - | - | - |
| 21911 | FLJ35976 | NM_173639.1 | 330 | gagcagccgtcagcttgtcaact | 426929 | - | - | - | - |
| 21912 | FLJ35989 | NM_152597.3 | 1330 | gagcaacgcgatctgcataatcg | 418651 | - | see also 9898 line | | |
| 21913 | FLJ36004 | NM_152590.1 | 1249 | cggtcaaccagacctaatcgagc | 418487 | - | - | - | - |
| 21914 | FLJ36046 | NM_152612.2 | 894 | ccgcaagcgagagttcaataagg | 418821 | - | - | - | - |
| 21915 | FLJ36090 | NM_153223.1 | 732 | gagcctcgtgaaacgttagaata | 422286 | - | - | - | - |
| 21916 | FLJ36102 | NM_173590.1 | 711 | tggagcatctctattaacttagt | 426351 | - | - | - | - |
| 21917 | FLJ36144 | NM_182561.1 | 1713 | gagaggatacgagatcagaaaga | 434454 | - | - | - | - |
| 21918 | FLJ36155 | NM_147193.1 | 2198 | cggggagacccacggtttcaacc | 415091 | - | - | - | - |
| 21919 | FLJ36175 | NM_152528.1 | 255 | ctgtactcgttacgtgactttac | 417688 | - | kinase_related | - | - |
| 21920 | FLJ36180 | NM_178556.3 | 826 | aagctcggctttcgaatctcttg | 431274 | - | - | - | - |
| 21921 | FLJ36198 | NM_173801.2 | 331 | ctgtactttaccccaaggaatat | 428041 | - | - | - | - |
| 21922 | FLJ36335 | NM_173568.1 | 1052 | cggttatgtggtccttattgtgg | 426233 | - | - | - | - |
| 21923 | FLJ36445 | NM_153233.1 | 1139 | aagccccatctcaaattcactcc | 422423 | - | - | - | - |
| 21924 | FLJ36525 | NM_147129.2 | 1963 | gcgtaggtctcaggattacctgt | 415030 | - | - | - | - |
| 21925 | FLJ36561 | NM_182520.1 | 821 | ccgctatgagtccctattggaga | 434088 | - | - | - | - |
| 21926 | FLJ36576 | NM_173522.1 | 335 | cagttgtggtcgcattctcaaga | 425430 | - | - | - | - |
| 21927 | FLJ36600 | NM_182497.1 | 668 | tggccactgatgactttaagtca | 433959 | - | - | - | - |
| 21928 | FLJ36601 | NM_173695.1 | 1058 | tagtgccatactgcagcaataat | 427666 | - | - | - | - |
| 21929 | FLJ36664 | NM_173690.1 | 835 | aggcggatcctgtaatggtatta | 427467 | - | see also 10111 line | | |
| 21930 | FLJ36674 | NM_173622.1 | 438 | ttgcccgcaagcctatgatgagg | 426617 | - | - | - | - |
| 21931 | FLJ36760 | NM_198550.1 | 920 | tagtgatacgacggcagaaatat | 439440 | - | - | - | - |
| 21932 | FLJ36779 | NM_152571.1 | 531 | ttgcctttcaggagtcagattcc | 418162 | - | - | - | - |
| 21933 | FLJ36812 | NM_153260.1 | 786 | aagaaccagattcgaagtatacc | 422684 | - | see also 9979 line | | |
| 21934 | FLJ36874 | NM_152716.1 | 1263 | gaggactatgaaagacgttatct | 420059 | - | - | - | - |
| 21935 | FLJ36878 | NM_178518.2 | 1037 | gggaagcgtggcccacattatgt | 431057 | - | - | - | - |
| 21936 | FLJ36888 | NM_178830.2 | 277 | tggatctcaataaggagataatg | 431979 | - | - | - | - |
| 21937 | FLJ36980 | NM_182598.1 | 364 | atgagaaggaccgcactatttca | 434596 | - | - | - | - |
| 21938 | FLJ37099 | NM_198459.1 | 1052 | gtgttacgccgtataaagaaaga | 437893 | - | - | - | - |
| 21939 | FLJ37118 | NM_173507.1 | 1486 | cagcgagggatgccacattctcc | 425204 | - | - | - | - |
| 21940 | FLJ37306 | NM_173497.1 | 670 | ctgtgtatgataccttacttaat | 425164 | - | - | - | - |

Figure 46

| 21941 | FLJ37357 | NM_173645.1 | 656 | aggctgacgtgctttattcgtct | 426946 | - | - | - | - |
|---|---|---|---|---|---|---|---|---|---|
| 21942 | FLJ37396 | NM_173671.1 | 578 | ccgacgcccgaggtttgatctca | 427282 | - | - | - | - |
| 21943 | FLJ37538 | NM_173564.2 | 1520 | aggacaaggccgtgtcttacacc | 426185 | - | - | - | - |
| 21944 | FLJ37543 | NM_173667.1 | 80 | tggcaccactcttcttatgctgt | 427240 | - | - | - | - |
| 21945 | FLJ37549 | NM_152605.1 | 1291 | aggcagagggcgcatcttactca | 418745 | - | - | - | - |
| 21946 | FLJ37587 | NM_173597.1 | 460 | cagcggcaactcattcttactca | 426425 | - | - | - | - |
| 21947 | FLJ37659 | NM_173698.1 | 742 | aagctctacgcatgaatcagaat | 427707 | - | - | - | - |
| 21948 | FLJ37673 | NM_182591.1 | 228 | ttgggtggtctgacctgatattt | 434558 | - | - | - | - |
| 21949 | FLJ37794 | NM_173588.1 | 1334 | ctgacgcacacgcttaagattaa | 426315 | - | - | - | - |
| 21950 | FLJ37818 | NM_175916.2 | 334 | aggcgactgataacgttcaatga | 429553 | - | - | - | - |
| 21951 | FLJ37927 | NM_152623.1 | 378 | ttgcgagtagccccattaccaca | 419079 | - | - | - | - |
| 21952 | FLJ37953 | NM_152382.1 | 769 | acgacgaatggtcctacacattc | 416023 | - | - | - | - |
| 21953 | FLJ38101 | NM_153261.3 | 130 | gagaggagacttactgaatatat | 422690 | - | - | - | - |
| 21954 | FLJ38159 | NM_152723.1 | 340 | atgcttcgctcccgcattgaaga | 420095 | - | - | - | - |
| 21955 | FLJ38190 | NM_152335.1 | 678 | agggtgaagagggtcattgatgc | 415695 | - | - | - | - |
| 21956 | FLJ38288 | NM_173632.2 | 1064 | taggagcaactcccacctaaagg | 426907 | - | - | - | - |
| 21957 | FLJ38335 | NM_153228.1 | 1072 | aagtctcggcttacaatatgaaa | 422342 | - | - | - | - |
| 21958 | FLJ38377 | NM_152698.1 | 2010 | cagccacttcgacaacagatact | 419889 | - | - | - | - |
| 21959 | FLJ38379 | NM_178530.2 | 720 | acgtctgcacacagtgtctttgg | 431073 | - | - | - | - |
| 21960 | FLJ38426 | NM_173611.1 | 503 | ctgacattccgcatatgctaaac | 426511 | - | - | - | - |
| 21961 | FLJ38482 | NM_152681.1 | 582 | gagacttgcgttgatgatacagt | 419713 | - | - | - | - |
| 21962 | FLJ38564 | NM_152579.1 | 322 | tggctccaatgtaaagtacatag | 418235 | - | - | - | - |
| 21963 | FLJ38568 | NM_153252.1 | 1757 | gtgggaggtacgctatattgaac | 422656 | - | - | - | - |
| 21964 | FLJ38607 | NM_152654.1 | 327 | aagtgatccaggggatgtgtaag | 419515 | - | - | - | - |
| 21965 | FLJ38615 | NM_173528.1 | 443 | gggacctgagcctgtgtatgact | 425507 | - | - | - | - |
| 21966 | FLJ38628 | NM_152267.2 | 333 | ctgttggccgtgtttacatcagt | 415226 | - | see also 9823 line | - | - |
| 21967 | FLJ38716 | NM_152367.1 | 656 | gtgaacatcgacggagacattgt | 415838 | - | - | - | - |
| 21968 | FLJ38723 | NM_173805.2 | 75 | cagcttcaagttgtactgattga | 428051 | - | - | - | - |
| 21969 | FLJ38725 | NM_153218.1 | 575 | ctgccactttgtataccattaaa | 422232 | - | - | - | - |
| 21970 | FLJ38736 | NM_182758.1 | 2261 | aggcactagccaagcctattact | 435080 | - | - | - | - |
| 21971 | FLJ38753 | NM_152375.1 | 1628 | tggcggtcaacggctttctctac | 415957 | - | - | - | - |
| 21972 | FLJ38771 | NM_173511.1 | 1282 | gagtctctcgaggaacaagtaca | 425264 | - | - | - | - |
| 21973 | FLJ38773 | NM_178528.2 | 648 | gtgcaagctcggagatgaattgg | 431070 | - | - | - | - |
| 21974 | FLJ38792 | NM_178520.2 | 626 | gtgatcgggcagctgatttatct | 431067 | - | - | - | - |
| 21975 | FLJ38819 | NM_145865.1 | 98 | gaggctaccaagcgagatctaaa | 414935 | - | - | - | - |
| 21976 | FLJ38944 | NM_152361.1 | 376 | cagattactacgggatttcgagg | 415808 | - | - | - | - |
| 21977 | FLJ38968 | NM_152316.1 | 187 | gagtccagcttcgtactacaaat | 415551 | - | - | - | - |

Figure 46

| 21978 | FLJ38973 | NM_153689.3 | 559 | cggttccccaatagttatatttg | 423528 | - | - | - | - |
|---|---|---|---|---|---|---|---|---|---|
| 21979 | FLJ38984 | NM_152374.1 | 534 | ggggacccacctcctggattatg | 415948 | - | - | - | - |
| 21980 | FLJ39058 | NM_173580.1 | 200 | ctggagtctggcttatttccagc | 426297 | - | - | - | - |
| 21981 | FLJ39106 | NM_173629.1 | 654 | ctgttgcacctaccgatcattta | 426693 | - | - | - | - |
| 21982 | FLJ39237 | NM_198571.1 | 1111 | acggcacttggcgctatctcaac | 439707 | - | - | - | - |
| 21983 | FLJ39267 | NM_173550.2 | 3628 | ctgcgtcagctcaatagacatct | 425937 | - | - | - | - |
| 21984 | FLJ39370 | NM_152400.1 | 399 | ctgcaagcccttggactagttgc | 416283 | - | see also 9851 line | | |
| 21985 | FLJ39378 | NM_178314.2 | 1169 | tggtagcagcgggactgataata | 430700 | - | - | - | - |
| 21986 | FLJ39415 | NM_173681.1 | 775 | tcgatgcgtggattacaatgttc | 427337 | - | - | - | - |
| 21987 | FLJ39426 | NM_173609.1 | 693 | aagtgttagttgcaaagatatgc | 426490 | - | - | - | - |
| 21988 | FLJ39441 | NM_194285.1 | 1622 | ctggacggactgtcagtaattca | 436341 | - | see also 10307 line | | |
| 21989 | FLJ39553 | NM_173549.1 | 938 | gagaatgtaacaccagaagtatt | 425850 | - | - | - | - |
| 21990 | FLJ39575 | NM_182597.1 | 419 | tggtggattccataaatgactta | 434584 | - | - | - | - |
| 21991 | FLJ39576 | NM_182592.1 | 603 | gtgtcaccttacatagtgaattt | 434583 | - | - | - | - |
| 21992 | FLJ39582 | NM_178315.2 | 387 | aagacagtatcgcaatacagaaa | 430707 | - | - | - | - |
| 21993 | FLJ39630 | NM_173688.1 | 404 | aggacaccgatctaatgacattc | 427430 | - | - | - | - |
| 21994 | FLJ39647 | NM_173625.2 | 853 | ctgttatccaccagacatacttc | 426637 | - | - | - | - |
| 21995 | FLJ39653 | NM_152684.1 | 823 | gggacagtggtacctcgatttcc | 419796 | - | - | - | - |
| 21996 | FLJ39660 | NM_173646.1 | 592 | ttgcatctcgaagttatgaattt | 426988 | - | - | - | - |
| 21997 | FLJ39743 | NM_182562.1 | 541 | gcgggacttctgtgagacattcc | 434457 | - | - | - | - |
| 21998 | FLJ39821 | NM_173700.1 | 751 | aggccctccaccgaagaataagg | 427732 | - | - | - | - |
| 21999 | FLJ39827 | NM_152424.1 | 2215 | gagaagcccgtcttagagtatca | 416613 | - | - | - | - |
| 22000 | FLJ39873 | NM_173799.2 | 510 | tggtggtcgcgttgactagaaag | 427987 | - | - | - | - |
| 22001 | FLJ40089 | NM_182496.1 | 595 | ctgctcgagtatgctttgtcaac | 433935 | - | - | - | - |
| 22002 | FLJ40126 | NM_173599.1 | 230 | atgcccctaagaaagcataattt | 426429 | - | - | - | - |
| 22003 | FLJ40160 | NM_173484.2 | 491 | aggcccttcggtgggaatctaag | 424997 | - | - | - | - |
| 22004 | FLJ40172 | NM_173649.1 | 159 | gtggagaatagcattaacagata | 427055 | - | - | - | - |
| 22005 | FLJ40182 | NM_173696.1 | 778 | ctgctacgtgaccaattcgtaaa | 427693 | - | - | - | - |
| 22006 | FLJ40201 | NM_152607.1 | 599 | atgcctggaaccggtctcattcc | 418747 | - | - | - | - |
| 22007 | FLJ40224 | NM_173579.1 | 837 | gagtccggaggccgaatccatca | 426296 | - | - | - | - |
| 22008 | FLJ40235 | NM_173635.1 | 724 | ctgatccagggtgctatctatgc | 426917 | - | - | - | - |
| 22009 | FLJ40243 | NM_173489.2 | 4585 | ccgagacgtgagcttctacttca | 425116 | - | - | - | - |
| 22010 | FLJ40288 | NM_173682.1 | 971 | aggctataggaatacttacaacc | 427357 | - | - | - | - |
| 22011 | FLJ40296 | NM_198441.1 | 263 | tcgctccatggccccctaatcaag | 437768 | - | - | - | - |
| 22012 | FLJ40321 | NM_198479.1 | 916 | aaggcccaggtccatcttaagt | 438456 | - | - | - | - |
| 22013 | FLJ40365 | NM_173482.1 | 958 | gtgcgccttggcgctaaacgaag | 424984 | - | - | - | - |
| 22014 | FLJ40411 | NM_198852.1 | 507 | ctgatacacccgttcaattcata | 439950 | - | - | - | - |

Figure 46

| | | | | | | | | | |
|---|---|---|---|---|---|---|---|---|---|
| 22015 | FLJ40457 | NM_173628.1 | 1492 | tggcgaaggtgtgtttcagtaca | 426670 | - | - | - | - |
| 22016 | FLJ40504 | NM_173624.1 | 509 | gggtcttagccagttagatttac | 426620 | - | - | - | - |
| 22017 | FLJ40542 | NM_178503.2 | 333 | cagccgtgcccacgcatttatct | 430924 | - | - | - | - |
| 22018 | FLJ40597 | NM_152615.1 | 1210 | agggacgtgcaggattagtcacc | 418889 | - | - | - | - |
| 22019 | FLJ40629 | NM_152515.2 | 860 | tcggacccttagtaaagttcagt | 417412 | - | - | - | - |
| 22020 | FLJ40852 | NM_173677.1 | 437 | tggatcatgttctgatgatatag | 427326 | - | - | - | - |
| 22021 | FLJ40869 | NM_182625.1 | 1435 | gtggtaaagacctgcaatgttag | 434753 | - | - | - | - |
| 22022 | FLJ40873 | NM_152665.1 | 233 | atgggcgcatcaaagattctatg | 419536 | - | - | - | - |
| 22023 | FLJ40906 | NM_173640.1 | 560 | cagtagtcctgcgcaatgtgaag | 426933 | - | - | - | - |
| 22024 | FLJ40919 | NM_182508.1 | 315 | atgctcttgtacttagagattca | 434026 | - | see also 10266 line | | |
| 22025 | FLJ41131 | NM_198476.1 | 886 | gggcaagagttggcactatcagc | 438438 | - | - | - | - |
| 22026 | FLJ41238 | NM_198485.1 | 305 | gtgtgaccgaatcaactctttac | 438511 | - | - | - | - |
| 22027 | FLJ41410 | NM_198581.1 | 899 | caggtcctggacctaacatgtct | 439766 | - | see also 10347 line | | |
| 22028 | FLJ42117 | NM_198463.1 | 1909 | atgttgagcccaccaatcgttcc | 438056 | - | - | - | - |
| 22029 | FLJ42461 | NM_198501.1 | 486 | cagcaagcaaacacagcaatagc | 438741 | - | - | - | - |
| 22030 | FLJ42654 | NM_198447.1 | 544 | gcgctgttccggagacttcaagg | 437824 | - | - | - | - |
| 22031 | FLJ42925 | NM_198511.1 | 926 | tgggaagcagtatgccataaaga | 438840 | - | - | - | - |
| 22032 | FLJ43070 | NM_152286.2 | 2240 | gtggtgactcggctgattcatct | 415349 | - | - | - | - |
| 22033 | FLJ43374 | NM_198582.1 | 905 | cagacttccccgactatcacaag | 439813 | - | - | - | - |
| 22034 | FLJ43654 | NM_198562.1 | 664 | aaggatctgcttgatatgattga | 439525 | - | - | - | - |
| 22035 | FLJ43842 | NM_198440.1 | 753 | ttgcggtgggccatatctactac | 437765 | - | - | - | - |
| 22036 | FLJ44112 | NM_198520.1 | 1990 | gtggctgcgacaataaattgtgt | 439036 | - | - | - | - |
| 22037 | FLJ44186 | NM_198508.1 | 1196 | cggcgaggacaatctggatatca | 438796 | - | - | - | - |
| 22038 | FLJ44216 | NM_198567.1 | 2262 | tggctggtcaaagcagtaactga | 439672 | - | see also 10346 line | | |
| 22039 | FLJ44299 | NM_198491.1 | 740 | aagcggtggaggtgtattctagc | 438550 | - | - | - | - |
| 22040 | FLJ44691 | NM_198506.1 | 1348 | ctgtatctagcgcatcatcatat | 438770 | - | - | - | - |
| 22041 | FLJ45235 | NM_198493.1 | 321 | ctgggcaaagtgacgtgattaga | 438558 | - | - | - | - |
| 22042 | FLJ45246 | NM_198484.1 | 1427 | ctgcctacatctggaataccttc | 438507 | - | - | - | - |
| 22043 | FLJ45273 | NM_198461.1 | 1062 | aagcggtttggcatgtgtttatc | 437949 | - | - | - | - |
| 22044 | FLJ45645 | NM_198557.1 | 292 | atgtgatatatccgacaagaacc | 439482 | - | - | - | - |
| 22045 | FLJ45651 | NM_198442.1 | 1190 | cagggtcatggctgtccaattct | 437778 | - | - | - | - |
| 22046 | FLJ45778 | NM_198532.1 | 524 | cagggcctccgtaccatctatgc | 439256 | - | - | - | - |
| 22047 | FLJ45909 | NM_198445.1 | 893 | gtgcgcatcgcctcagattctgg | 437815 | - | - | - | - |
| 22048 | FLJ46072 | NM_198488.1 | 1853 | aagtccaactacaggatttatcg | 438532 | - | - | - | - |
| 22049 | FLJ46154 | NM_198462.1 | 2941 | gggtatcgtaatgggaagttaat | 438000 | - | - | - | - |
| 22050 | FLJ46156 | NM_198499.1 | 2765 | aggagagaccacggatacttagt | 438701 | - | - | - | - |
| 22051 | FLJ46247 | NM_198529.1 | 1237 | gggtcacgcagagagtctattgc | 439168 | - | - | - | - |

Figure 46

| 22052 | FLJ46909 | NM_198469.1 | 247 | tggccataaagggcacatatacg | 438397 | - | - | - | - |
|--------|----------|-------------|------|---------------------------|--------|---|---------------------|--------------------------------|-----------------|
| 22053 | FLJ90005 | NM_153341.1 | 888 | aaggcctgactagtgtattgagc | 422827 | - | - | - | - |
| 22054 | FLJ90013 | NM_153365.1 | 1441 | caggcaagccgtccagtaaatca | 423103 | - | see also 9993 line | | |
| 22055 | FLJ90022 | NM_153690.3 | 1195 | ctgagctgtcgcaacttattagc | 423548 | - | - | - | - |
| 22056 | FLJ90024 | NM_153342.1 | 684 | tggctgtcatcgcctttatcacc | 422839 | - | - | - | - |
| 22057 | FLJ90119 | NM_153347.1 | 363 | gtgcatggtctgctgatgtttgc | 422925 | - | - | - | - |
| 22058 | FLJ90166 | NM_153360.1 | 1110 | ccgctcctgggaagggtattacc | 423052 | - | - | - | - |
| 22059 | FLJ90406 | NM_173670.1 | 1326 | gggcctggccactcatagataat | 427268 | - | - | - | - |
| 22060 | FLJ90492 | NM_181783.1 | 395 | aagtcttaccgtcccttaacagt | 433226 | - | - | - | - |
| 22061 | FLJ90575 | NM_153376.1 | 1459 | ggggctgcggacggacaacatca | 423166 | - | - | - | - |
| 22062 | FLJ90579 | NM_173591.1 | 395 | ttgtcctatgccattactcaact | 426356 | - | - | - | - |
| 22063 | FLJ90583 | NM_173828.3 | 1317 | ctgccacgtgctcaagaagaagg | 428439 | - | - | - | - |
| 22064 | FLJ90586 | NM_153345.1 | 1031 | ttggcggcagtccccacattaga | 422856 | - | - | - | - |
| 22065 | FLJ90650 | NM_173800.2 | 446 | gtgtctactggcgtcatgaaaca | 427996 | - | - | - | - |
| 22066 | FLJ90652 | NM_173618.1 | 235 | aggtgtcccgggacaagagtttc | 426580 | - | see also 10091 line | | |
| 22067 | FLJ90805 | NM_173633.1 | 958 | aggtactgtacacgtatgagtcg | 426913 | - | see also 10101 line | | |
| 22068 | FLJ90806 | NM_182513.1 | 413 | ccgtgtacgtggctcaactttac | 434046 | - | - | - | - |
| 22069 | FLJ90811 | NM_153339.1 | 464 | cggtgtttgaacgcaacctatgc | 422792 | - | - | - | - |
| 22070 | FLN29 | NM_006700.1 | 1407 | cagccgacccattaacaatatga | 336143 | - | - | - | - |
| 22071 | FLNC | NM_001458.1 | 5135 | gagaaccatgacggtacctttga | 330271 | - | - | - | - |
| 22072 | FNBP1 | NM_015033.1 | 590 | gcgatttgaacgcgattgcaaag | 346999 | - | - | - | - |
| 22073 | FNBP4 | NM_015308.1 | 1839 | cagggatcatagacggtatttct | 351381 | - | see also 6321 line | | |
| 22074 | FRBZ1 | NM_194314.1 | 1614 | atgtggtcgccagtttaacgaca | 436777 | - | see also 10308 line | | |
| 22075 | FRCP1 | NM_022823.1 | 788 | ctgaccggctaccttcaaacagt | 384249 | - | - | - | - |
| 22076 | FRCP2 | NM_153756.1 | 286 | gaggaggatacggagtacatagt | 423889 | - | - | - | - |
| 22077 | FS | NM_021996.3 | 634 | tggggaagtacactcatttcatc | 381671 | - | - | - | - |
| 22078 | FTSJ3 | NM_017647.2 | 979 | aggagctcaggtcgctactaaac | 360079 | - | see also 7077 line | | |
| 22079 | FXYD4 | NM_173160.1 | 498 | cagttctgagtggcaaatgcaaa | 424775 | - | channel | - | - |
| 22080 | FZR1 | NM_016263.2 | 220 | caggattaacgagaatgagaagt | 356658 | - | cell_cycle | - | - |
| 22081 | G30 | NM_172368.1 | 400 | cagttggccaggtcttgtgaaag | 424479 | - | - | - | - |
| 22082 | G6PC3 | NM_138387.1 | 687 | tggcttgtcgcgaatcttcatct | 407875 | - | - | - | - |
| 22083 | GA17 | NM_006360.2 | 670 | gtgtattgtacgagcattgaaag | 335330 | - | - | - | - |
| 22084 | GAB2 | NM_012296.2 | 803 | aagccgagccggcacaatacaga | 337961 | - | - | - | - |
| 22085 | GAB3 | NM_080612.1 | 53 | gtgcaccggctggctcgttaagt | 406413 | - | - | - | - |
| 22086 | GAGED2 | NM_020411.1 | 412 | cagtcaaacaccggggataaatc | 377374 | - | - | - | Ewing's sarcoma |
| 22087 | GAJ | NM_032117.2 | 276 | gagaggatcggaacttctaatta | 397763 | - | - | molecular_function unknown | |

Figure 46

| | | | | | | | | |
|---|---|---|---|---|---|---|---|---|
| 22088 | GALNT10 | NM_017540.3 | 648 | ttggggggagatccgaaatgtgg | 358989 | - | see also 7020 line | |
| 22089 | GALNT11 | NM_022087.1 | 1592 | gggaggtctcgtggtgcttaagg | 382255 | - | - | |
| 22090 | GALNT15 | NM_145292.2 | 695 | acgaacctcacgccacactatt | 414220 | - | - | |
| 22091 | GALNT7 | NM_054110.2 | 1639 | ctggagcgtatgactctcttatg | 406047 | - | - | |
| 22092 | GBAS | NM_001483.1 | 548 | aagatccggacctaatatatatg | 330297 | - | - | |
| 22093 | GBDR1 | NM_016172.1 | 441 | caggaccaagatgtcctattatt | 356520 | - | - | |
| 22094 | GBL | NM_022372.2 | 450 | aggtgaacgcacccattaactgc | 382751 | - | - | |
| 22095 | GBP | NM_017870.2 | 3038 | cggccggaagcgagtagagtttgt | 363325 | - | - | |
| 22096 | GCET2 | NM_152785.2 | 202 | cagatgctgggatcaccatatcg | 421213 | - | - | |
| 22097 | GCP16 | NM_016099.1 | 391 | gagcgaggactgcgagttattga | 356276 | - | - | |
| 22098 | GDAP1L1 | NM_024034.3 | 600 | cggccgagatccgcagacattta | 385534 | - | see also 8566 line | |
| 22099 | GDAP2 | NM_017686.1 | 1421 | aagaaactcacgatgttgttga | 360808 | - | - | |
| 22100 | GDDR | NM_182536.1 | 327 | atgttacatctatgagaaacagg | 434222 | - | - | |
| 22101 | GDF7 | NM_182828.1 | 833 | tggtgccgcaccacttcatgatg | 435295 | - | - | |
| 22102 | GEFT | NM_133483.1 | 611 | gaggccgtgacaggattgtgttt | 407236 | - | - | |
| 22103 | GEMIN5 | NM_015465.1 | 2694 | cagggctaccctgtatagaatga | 353379 | - | - | |
| 22104 | GEMIN6 | NM_024775.8 | 454 | ctgactagaccccaccatatga | 390577 | - | - | |
| 22105 | GEMIN7 | NM_024707.1 | 573 | ctgctccgatgtagtgacattat | 389453 | - | - | |
| 22106 | GJA8 | NM_005267.2 | 1334 | gagccaggtcaggacgatctaacc | 333584 | - | connexon channel | cataract |
| 22107 | GJB7 | NM_198568.1 | 700 | gggcctatggtacgcttatctta | 439695 | - | - | |
| 22108 | GK001 | NM_020198.1 | 950 | atggatacctacgtatttgctgt | 376120 | - | - | |
| 22109 | GK003 | NM_020192.1 | 647 | tgggcctatcagtagatgtatta | 375945 | - | - | |
| 22110 | GL004 | NM_020194.4 | 619 | gtgtacttacgctgagtgaaaga | 376045 | - | see also 7800 line | |
| 22111 | GL009 | NM_032492.2 | 611 | atgcctgcagttgactactacagc | 400402 | - | see also 9220 line | |
| 22112 | GLIS2 | NM_032575.1 | 564 | gtgtcgctgggccaagtgtaacc | 400819 | - | - | |
| 22113 | GLTDC1 | NM_182974.1 | 1022 | tggactcaatagacacttatgaaa | 435547 | - | - | |
| 22114 | GLTSCR1 | NM_015711.1 | 3730 | atgatcgaccgaatgttcattca | 355233 | - | - | |
| 22115 | gm117 | NM_198077.1 | 179 | cgggacctgacagagctgtttagg | 436975 | - | - | |
| 22116 | GMH1 | NM_014044.4 | 1451 | tggctctgtgtgtccactatagg | 339392 | - | see also 5471 line | |
| 22117 | GNPNAT1 | NM_198066.1 | 419 | atgttgttgttagtgatgaatgc | 436950 | - | - | |
| 22118 | GnT-IX | NM_144677.2 | 517 | gcggcgtcctgcgcaagatgagc | 411274 | - | - | |
| 22119 | GORASP1 | NM_031899.2 | 529 | aggagtccgaggacttctttacg | 397234 | - | see also 9049 line | |
| 22120 | GPP34R | NM_018178.3 | 464 | atgctaagaagcgactactaga | 368043 | - | see also 7302 line | |
| 22121 | GPR103 | NM_198179.1 | 538 | atgttggcacgtgcaacaacttga | 437167 | - | receptor_acitivity, gpcr, signal_transduction | |
| 22122 | GPR107 | NM_020960.1 | 276 | tagcctagacctacaagaatg | 380225 | - | - | |

Figure 46

| | | | | | | | | |
|---|---|---|---|---|---|---|---|---|
| 22123 | GPR141 | NM_181791.1 | 628 | aagctacgccactctttactatc | 433518 | - | receptor_acitivity, gpcr, signal_transduction | |
| 22124 | GPR144 | NM_182611.1 | 2459 | tggcccgtgtgtaatgatcacc | 434674 | - | receptor_acitivity, gpcr, signal_transduction | |
| 22125 | GPR8 | NM_005286.2 | 365 | ccgtcgaccactacacactctc | 333589 | - | receptor_acitivity, gpcr, signal_transduction | somatostatin receptor |
| 22126 | GPT | NM_005309.1 | 969 | tcgtgcgctgtgtcatcaacc | 333626 | - | | alanine aminotransferase |
| 22127 | GPX6 | NM_182701.1 | 196 | acggcgaggagtacatccaattc | 434873 | - | | |
| 22128 | GRASP | NM_181711.1 | 524 | cagcgtcaatggcctgaatgtgg | 432875 | - | receptor_acitivity | |
| 22129 | GRCC10 | NM_138425.2 | 223 | gggtaagatgctgcaattcgtgc | 408102 | - | | |
| 22130 | GRLF1 | NM_004491.2 | 2222 | ttggttacggacgcaacattaat | 332562 | - | transcription_regulator, cell_cycle, signal_transduction | |
| 22131 | GRPEL2 | NM_152407.2 | 587 | ctggcaccgtggcattagtaaga | 416373 | - | see also 9857 line | |
| 22132 | GRWD1 | NM_031485.2 | 257 | ccgagtccggcgacaacaagttcc | 397203 | - | | nutrient reservoir |
| 22133 | GSDML | NM_018530.1 | 1340 | ctgtgcgctgtatgttgttgtct | 372164 | - | | |
| 22134 | GSG1 | NM_031289.1 | 475 | gaggtgccgaagtttcattgaac | 396507 | - | see also 9003 line | |
| 22135 | GTF2IRD2 | NM_173537.1 | 1349 | tggaggcagctgagtttatcaaa | 425698 | - | | |
| 22136 | GW112 | NM_006418.3 | 1024 | atgtacaacaccgggaatattgc | 335517 | - | | |
| 22137 | H2AV | NM_012412.2 | 367 | gtgctgagctggcaggtaatgc | 338142 | - | | |
| 22138 | H326 | NM_015726.2 | 1169 | ggggctgtatacgatctatgtga | 355247 | - | see also 6542 line | |
| 22139 | H63 | NM_138423.2 | 1253 | cagtcctcttcagcgtttaatc | 408091 | - | see also 9618 line | |
| 22140 | HABP4 | NM_014282.1 | 1113 | ccggaaaccgccaatgacatca | 340023 | - | | |
| 22141 | HAGHL | NM_032304.1 | 677 | gggcagcgccagcagatgtacc | 399603 | - | | |
| 22142 | HATH6 | NM_032827.3 | 1061 | cagtgccgtgctactcatatgg | 401738 | - | see also 9297 line | |
| 22143 | HB-1 | NM_021182.1 | 162 | aagtcggaattggttgaagttga | 380666 | - | | |
| 22144 | HBXIP | NM_006402.2 | 163 | cggacgttgcaccgtgatcaatt | 335386 | - | | antiviral response protein |
| 22145 | HCA3 | NM_138703.2 | 900 | tggccgtgtatgcactaatcc | 408637 | - | | |
| 22146 | HCCA2 | NM_053005.2 | 181 | ccgcgagattgaccttaacgagt | 405761 | - | see also 9480 line | |
| 22147 | HCCR1 | NM_015416.2 | 942 | ctgaattctacgcatattggtga | 352361 | - | | |
| 22148 | HCD1 | NM_020195.1 | 855 | cggctcaggccaccaattcttcc | 376068 | - | | |
| 22149 | HCG4 | NM_018985.1 | 871 | gggtgcgtgggaacttagaaacc | 374065 | - | | |

Figure 46

| | Gene | Accession | Pos | Sequence | ID | | Notes | | |
|---|---|---|---|---|---|---|---|---|---|
| 22150 | HCMOGT-1 | NM_152904.1 | 710 | gtgacacggaacctatgataaga | 421574 | - | | | |
| 22151 | HCNGP | NM_013260.3 | 47 | tcgcagtttacgcggaagattca | 338482 | - | | | |
| 22152 | HDCMA18P | NM_016648.1 | 1002 | tggtgatcacggaacaaaggtatt | 358698 | - | | | |
| 22153 | HE9 | NM_172000.1 | 737 | ctgctggcatgtgatgatcaatg | 424179 | - | | | |
| 22154 | HEBP2 | NM_014320.1 | 684 | gtgtttgtacggtctttcgatgg | 340180 | - | | | |
| 22155 | HEIL1 | NM_032362.1 | 4 | gagagaatagtgcattgtgaagg | 399995 | - | | | |
| 22156 | HELB | NM_033647.2 | 202 | cgggtgctccgcgtttctattt | 404157 | - | | | |
| 22157 | HEMGN | NM_018437.3 | 733 | aagacaactctcctaacacatgc | 371524 | - | | | |
| 22158 | HFL-EDDG1 | NM_006553.2 | 473 | aagtttaatggccaatacgatat | 335806 | - | | | |
| 22159 | HHIP | NM_022475.1 | 1505 | ctgctaagcctcgcattccatcc | 383220 | - | see also 8397 line | | |
| 22160 | HHL | NM_014857.2 | 1981 | atggtcaagaatgctctataag | 344895 | - | see also 6054 line | | |
| 22161 | HHLA1 | NM_005712.1 | 1578 | tggcgcctgctgctgagataatg | 334205 | - | | | |
| 22162 | hIAN2 | NM_024711.2 | 338 | agggacgtcttcgagtctaaact | 389491 | - | | | |
| 22163 | hIAN6 | NM_175571.1 | 734 | ttgagtctttggtgaatacgaac | 429024 | - | | | |
| 22164 | hIAN7 | NM_153236.2 | 813 | tagtagaagaggataagcataaa | 422456 | - | | | |
| 22165 | HIC1 | NM_006497.1 | 2136 | ggggaccatcgaccgtttctctc | 335696 | - | transcription_regulator, cell_cycle | transcription factor | leukemia |
| 22166 | HIF1AN | NM_017902.1 | 851 | gttggcatcacatagagtcattac | 363736 | - | | | |
| 22167 | HILS1 | NM_194072.1 | 549 | tggcccgaaatgcctatcacttc | 436063 | - | | | |
| 22168 | HIMAP2 | NM_015660.1 | 566 | atgcacgactcagataacaaagc | 354947 | - | | | |
| 22169 | HIMAP4 | NM_018326.2 | 87 | cagcccaatacggcagtatgagc | 370388 | - | | | |
| 22170 | HINT2 | NM_032593.1 | 417 | tggataccgacttgatcaacg | 400965 | - | | | |
| 22171 | HIP14 | NM_015336.1 | 1424 | ggggaatcagcgcttgatttgg | 351654 | - | see also 6369 line | | |
| 22172 | HIP14L | NM_019028.1 | 1444 | ctgtgggctcgatatgatcaac | 374558 | - | see also 7667 line | | |
| 22173 | HIRIP5 | NM_015700.1 | 792 | aggcgtagaacaggttatggatg | 355152 | - | | | |
| 22174 | HIS1 | NM_006460.1 | 1250 | aaggatccgagcgcgagatgttcg | 335590 | - | | | |
| 22175 | HIST1H4B | NM_003544.2 | 133 | aagcgaatttccgtttgattta | 331765 | - | | | |
| 22176 | HIST1H4L | NM_003546.2 | 161 | gagacacgcggagttcttaaagt | 331768 | - | | | |
| 22177 | HIST4H4 | NM_175054.2 | 212 | ccggggagtcctcaaagtcttcc | 428901 | - | | | |
| 22178 | HIVEP2 | NM_006734.2 | 2811 | caggctagttcggcaacacaaca | 336224 | - | see also 4756 line | | |
| 22179 | HIWI2 | NM_152431.1 | 2059 | tgatgcgttggccagtgttaacc | 416739 | - | | | |
| 22180 | HKR2 | NM_181846.1 | 818 | gtgcctcgaggaacagttctagt | 433810 | - | transcription_regulator | | |
| 22181 | HLC-8 | NM_017941.3 | 1192 | gagacgacttaccagtttcattc | 364456 | - | | | |
| 22182 | HMGA1L4 | NM_031288.1 | 362 | tggctggatgaagacagtaatct | 396502 | - | | | |

Figure 46

| | | | | | | | | |
|---|---|---|---|---|---|---|---|---|
| 22183 | HNLF | NM_182547.2 | 174 | tggtcatcggcgaactatcgtacc | 434325 | - | | |
| 22184 | HNOEL-iso | NM_020190.1 | 153 | acgccgactagctgctttagagg | 375903 | - | | Hermansky-Pudlak syndrome |
| 22185 | HOM-TES-85 | NM_016383.1 | 535 | cagatctcaaggcgcagctaaga | 357270 | - | see also 6562 line | |
| 22186 | HOOK1 | NM_015888.3 | 1431 | cagacacactagcgtttgaaatg | 355338 | - | | |
| 22187 | HOXA13 | NM_000522.2 | 1014 | acgggaatacgccacgaataaat | 329696 | - | see also 400 line | |
| 22188 | HOXD12 | NM_021193.2 | 683 | acgaattcctgccaacgaattc | 380748 | - | transcription_regulator | transcription factor |
| 22189 | HPS3 | NM_032383.3 | 2831 | tcgtacacgcttgaaagagtatg | 400175 | - | | |
| 22190 | HPS5 | NM_007216.3 | 1688 | ccgttcggcattccattaccatt | 337155 | - | | |
| 22191 | HPS6 | NM_024747.4 | 715 | tggcgtggcccagttctactca | 389892 | - | | |
| 22192 | HRASLS | NM_020386.2 | 809 | aggccaacgagcgataagtaac | 377365 | - | | |
| 22193 | HRASLS2 | NM_017878.1 | 358 | tggggcaggaagttgcctattcg | 363371 | - | | |
| 22194 | HRD1 | NM_032431.1 | 1724 | gagactgccactacagttgttgc | 400230 | - | see also 9187 line | |
| 22195 | HRLP5 | NM_054108.1 | 297 | gtgccccactcaggtctaaatag | 406026 | - | | |
| 22196 | HSA275986 | NM_018403.1 | 389 | atgcaagctgtcgtatatatagt | 371149 | - | see also 7448 line | |
| 22197 | HSCARG | NM_020677.2 | 523 | aggctgcaaggtgcagaagtagt | 377922 | - | | |
| 22198 | HSPBAP1 | NM_024610.3 | 976 | gaggcaatcaccgtatgcttgt | 387856 | - | see also 8667 line | |
| 22199 | HSPC043 | NM_021218.1 | 318 | gagaccctcttaataaggact | 380785 | - | | |
| 22200 | HSPC056 | NM_014154.1 | 905 | gagcaattcgacagatgataac | 339684 | - | see also 5519 line | |
| 22201 | HSPC063 | NM_014155.1 | 385 | gagccactctatcaattaacaca | 339689 | - | see also 5523 line | |
| 22202 | HSPC065 | NM_014157.2 | 435 | cagcatcacgagggcgatcattga | 339714 | - | | |
| 22203 | HSPC072 | NM_014162.1 | 368 | agggcaagacatcctacacaagc | 339766 | - | | |
| 22204 | HSPC109 | NM_016390.2 | 1033 | ttgacctgcacgtcaattacctgt | 357278 | - | | |
| 22205 | HSPC128 | NM_014167.1 | 402 | tagcattgacgagcctttatttg | 339801 | - | | |
| 22206 | HSPC129 | NM_016396.1 | 382 | gagtagaattgaacgtgatatag | 557283 | - | | |
| 22207 | HSPC132 | NM_016399.2 | 80 | gagtacgaccagtgcttcaatcg | 557371 | - | | |
| 22208 | HSPC135 | NM_014170.1 | 78 | gagcgactgagccgctataatag | 339808 | - | | |
| 22209 | HSPC138 | NM_016401.2 | 570 | cagcagactcctgtaggtaatgc | 557384 | - | | |
| 22210 | HSPC142 | NM_014173.1 | 945 | aagggtaccagctacaagtatga | 339843 | - | | |
| 22211 | HSPC154 | NM_014177.1 | 353 | ctgcgacctagatgtattcttgg | 339871 | - | | |
| 22212 | HSPC157 | NM_014179.1 | 565 | cagaatcactggctaagtcttgc | 339901 | - | | |
| 22213 | HSPC166 | NM_014186.1 | 395 | tggactggagaggtggatatcaaa | 339966 | - | | |
| 22214 | HSPC171 | NM_014187.1 | 178 | aagttctacctgcgggatcatact | 339968 | - | | |
| 22215 | HSPC196 | NM_016464.2 | 385 | tggtcaacctcctattccataag | 357586 | - | | |

Figure 46

| | | | | | | | | |
|---|---|---|---|---|---|---|---|---|
| 22216 | HSPC242 | NM_016498.2 | 610 | ctgttgaccatcccatcattat | 357886 | - | | |
| 22217 | HSPC268 | NM_197964.1 | 264 | ctgcgtagcatccggaaacatgt | 436923 | - | | |
| 22218 | HSU15552 | NM_014597.1 | 65 | aagccgcgtcggctgaaagttcc | 340913 | - | | |
| 22219 | HSU79274 | NM_013300.1 | 637 | tagctaagctgcaagagttatg | 338686 | - | | |
| 22220 | hSyn | NM_018157.1 | 309 | ctgcgactagccaagctaaatga | 367783 | - | see also 7288 line | |
| 22221 | HT007 | NM_018480.2 | 751 | atggcgattcctctagtctttca | 372031 | - | | |
| 22222 | HT009 | NM_018470.1 | 549 | atgaccacactatttaacgaact | 371928 | - | | |
| 22223 | HT011 | NM_018472.1 | 526 | ctgcttcactaggatattgtgt | 371937 | - | | |
| 22224 | HT014 | NM_020362.2 | 676 | gtgaccatcgcaattacgaagc | 376904 | - | see also 7847 line | |
| 22225 | HT017 | NM_020678.2 | 285 | cagactcgaacgctgcatttaca | 377929 | - | | |
| 22226 | HT021 | NM_020685.3 | 212 | gagaataaattgggtgatcaaca | 379979 | - | | |
| 22227 | HT036 | NM_031207.2 | 411 | cagggagctgatcgaatagcagt | 395961 | - | | |
| 22228 | hT2R55 | NM_181429.1 | 151 | ttggctatccacaattggaca | 432096 | - | | |
| 22229 | HTPAP | NM_032483.2 | 298 | tggcgtctttaccaacacaataa | 400365 | - | | |
| 22230 | HTR1A | NM_000524.1 | 548 | ccgacggcatgcaccattagcaag | 329706 | - | receptor_acitivity、gpc r、signal transduction receptor_acitivity | gpc Smad3/Sp-1 heterodimer |
| 22231 | HTR3D | NM_182537.1 | 394 | cagccctacgtggtaaacttc | 434230 | - | receptor_acitivity | |
| 22232 | HTR3E | NM_182589.1 | 676 | atgaaggtcgcatcaggtataag | 434554 | - | | |
| 22233 | HUMAGCGB | NM_013286.2 | 862 | gggagcattcggaccatgatca | 338669 | - | | |
| 22234 | HUMPPA | NM_014603.1 | 1715 | gagtacaaggcgctcttcaaaga | 340990 | - | | |
| 22235 | HYPB | NM_012271.1 | 607 | gtgcttccgttataaatctacc | 337801 | - | | |
| 22236 | HYPC | NM_012272.1 | 2181 | gagttgaaggcacgattccatga | 337936 | - | see also 5247 line | |
| 22237 | IAN4L1 | NM_018384.2 | 318 | gagggcaaatatggaactatgg | 370970 | - | | |
| 22238 | IBRDC1 | NM_152553.2 | 994 | tagggccatagcggttgtaatc | 418064 | - | | |
| 22239 | IDN3 | NM_015384.2 | 1865 | aggtagtatgatcaatcagtgt | 351997 | - | cell_cycle | |
| 22240 | IER5 | NM_016545.2 | 893 | cggctgacccgctcaagaagc | 358079 | - | | |
| 22241 | IFI30 | NM_006332.3 | 577 | agggctgtcgccagacactatca | 335217 | - | | oxidoreductase |
| 22242 | IFI44 | NM_006417.2 | 905 | cggtaacattcgtgatgatacc | 335479 | - | | RNA polymerase II transcription factor |
| 22243 | IFIT2 | NM_001547.3 | 503 | aaggtggacacgttaaagtgt | 330351 | - | | RNA polymerase II transcription factor |
| 22244 | IFIX | NM_152501.2 | 884 | tcgcgatgtactaaatgcaaca | 417236 | - | | |
| 22245 | IFRG15 | NM_022347.1 | 110 | aggtattcttagaccgtattaac | 382633 | - | see also 8337 line | |
| 22246 | IGJ | NM_144646.2 | 460 | gtggtcccactcgtatatggtgg | 410726 | - | | antigen binding |
| 22247 | IGRP | NM_021176.1 | 50 | aggaactacgagcttactacact | 380580 | - | see also 8116 line | |

Figure 46

| | | | | | | | | |
|---|---|---|---|---|---|---|---|---|
| 22248 | IGSF11 | NM_152538.1 | 1262 | tggtcccggtcaacattaagact | 417763 | - | | - |
| 22249 | IGSF3 | NM_001542.1 | 3479 | tcggcatccgttctagataca | 330323 | - | | - |
| 22250 | IGSF4 | NM_014333.2 | 782 | gggacgcgcttgagttaacatgt | 340201 | - | see also 5628 line | |
| 22251 | IGSF9 | NM_020789.2 | 1896 | cagcgtgctagctcagaacaagc | 379034 | - | see also 7968 line | |
| 22252 | IL17RC | NM_032732.2 | 727 | aggtacgaatcgtgtcctatact | 401299 | - | receptor_acitivity | |
| 22253 | IL17RE | NM_144640.2 | 700 | gaggtcagcgtgcgtctttgtca | 410623 | - | | - |
| 22254 | IL23A | NM_016584.2 | 317 | gttgggaacacatgatctaagaga | 358227 | - | | - |
| 22255 | ILT10 | NM_024317.2 | 535 | ctggatacccaggttactatct | 386248 | - | | - |
| 22256 | ILT7 | NM_012276.2 | 1038 | atgggaccccgatgttcactttcc | 337953 | - | receptor_acitivity | - |
| 22257 | IMAGE3451454 | NM_053052.2 | 1347 | tagcatccgcagcgcggttattg | 405900 | | | |
| 22258 | IMAGE3455200 | NM_024006.2 | 252 | caggacagcatcctcaatcaatc | 385459 | - | | - |
| 22259 | IMAP1 | NM_130759.2 | 564 | gggctccctgcacgattacgtga | 406923 | - | | - |
| 22260 | IMMT | NM_006839.1 | 1045 | ctgsggccaagcctcatataact | 336440 | - | see also 4794 line | - |
| 22261 | IMP-2 | NM_006548.3 | 1728 | aggaagtgatcgtcagaattatc | 335777 | - | see also 4590 line | - |
| 22262 | IMP4 | NM_033416.1 | 357 | atgaaccgaggtgcacatgaagt | 403831 | - | | - |
| 22263 | INM01 | NM_175075.2 | 748 | cagcatcctggaaagatgtaa | 428999 | - | | - |
| 22264 | INSIG2 | NM_016133.2 | 824 | tggtcgacaactgcaatgtacg | 356431 | - | | - |
| 22265 | INSM2 | NM_032594.2 | 1826 | gggctgacccggccatcaataa | 400976 | - | | - |
| 22266 | INVS | NM_014425.2 | 1600 | cagcatggaggctatatcaac | 340616 | - | see also 5671 line | - |
| 22267 | IRA1 | NM_024665.2 | 440 | ttgtttgatgtcgaccaataga | 388741 | - | see also 8693 line | - |
| 22268 | IREM2 | NM_181449.1 | 139 | cagtgtggtcgtcagtatgaagagc | 432135 | - | receptor_acitivity | - |
| 22269 | IRF2BP1 | NM_015649.1 | 440 | gtgtcgcggctgcgtgaacttcg | 354860 | - | | - |
| 22270 | IRF2BP2 | NM_182972.1 | 1058 | ggggccaacgggtctaaagcagt | 435504 | - | | - |
| 22271 | IRTA1 | NM_031282.1 | 1478 | cagtcgttgtatgttgatgtaca | 396484 | - | | - |
| 22272 | IRTA2 | NM_031281.1 | 2880 | ctgcaaccagtgtacactaatgc | 396466 | - | | - |
| 22273 | IRX2 | NM_033267.1 | 1224 | agggtctcctggctacaactct | 403647 | - | | - |
| 22274 | ITGA1 | NM_181501.1 | 443 | gggcccttatatgctctatagatg | 432204 | - | receptor_acitivity, signal_transduction | magnesium binding |
| 22275 | ITIH5 | NM_030569.2 | 2669 | gaggccgtcctaacagtgaaagg | 394669 | - | see also 8889 line | - |
| 22276 | ITR | NM_180989.3 | 335 | atgaaccagaccgaacataatct | 432057 | - | receptor_acitivity | - |
| 22277 | JAM3 | NM_032801.3 | 541 | gagattgatcgagttaactgt | 401567 | - | | - |
| 22278 | JM1 | NM_014008.1 | 1741 | ctgttcggaaggcctataagtat | 339016 | - | see also 5453 line | - |
| 22279 | JM11 | NM_033626.2 | 1789 | aggccctgaggtgcatccaaacc | 404144 | - | | - |
| 22280 | JM4 | NM_007213.1 | 194 | gggtacgtgcggccacttcatac | 337117 | - | | - |
| 22281 | JM5 | NM_007075.1 | 331 | gtggtagtagtcccaagttctca | 336931 | - | see also 4945 line | - |

Figure 46

| | | | | | | | | | |
|---|---|---|---|---|---|---|---|---|---|
| 22282 | JMJD1 | NM_018433.2 | 2695 | cagtctccatacgtttaacagc | 371433 | - | - | | - |
| 22283 | JMY | NM_152405.1 | 868 | cagcgcactcggattgaagatga | 416338 | - | see also 9855 line | | - |
| 22284 | JPHL1 | NM_032452.1 | 2117 | cagctcgaatgccaaactgata | 400349 | - | - | | - |
| 22285 | JWA | NM_006407.2 | 409 | tgcgcgagctatttccttatctcc | 335397 | - | - | | - |
| 22286 | K5B | NM_173352.1 | 682 | caggcgtgccacacttgagaacg | 424812 | - | - | | - |
| 22287 | K6IRS3 | NM_175068.2 | 1579 | tggggagtgcaagtgaattcagg | 428984 | - | - | | - |
| 22288 | K6IRS4 | NM_175053.2 | 209 | gagggaatcggcgtatttcttc | 428895 | - | - | | - |
| 22289 | KAB | NM_014812.1 | 266 | aaggattccggaacagacttata | 344203 | - | - | | - |
| 22290 | KCNJ11 | NM_000525.2 | 1484 | gaggaggacggacgttactctgt | 329721 | - | channel | ATP-activated inward rectifier potassium channel | diabetes mellitus , diabetes |
| 22291 | KCTD8 | NM_198353.1 | 602 | caggtacgtgctggattatctgc | 437681 | - | see also 10316 line | | - |
| 22292 | KCTD9 | NM_017634.2 | 856 | tggacttcgatacattaacttc | 359808 | - | - | | - |
| 22293 | KDELC1 | NM_024089.1 | 1131 | tcgtgatgcctacgtacgatttg | 385858 | - | - | | - |
| 22294 | KENAE | NM_176816.2 | 1121 | atgaccaggcactacaaattgaca | 429796 | - | see also 10160 line | | - |
| 22295 | KEO4 | NM_006459.2 | 161 | tggaccaggctatcatcatcatgt | 335556 | - | see also 4531 line | | - |
| 22296 | KIAA0036 | NM_014642.1 | 680 | ctgacaatgtccaaatagggatct | 341599 | - | - | | - |
| 22297 | KIAA0040 | NM_014656.1 | 1004 | gggaccacgtggacaattcttgg | 341794 | - | - | nucleic acid binding | - |
| 22298 | KIAA0053 | NM_014882.1 | 1663 | acggacttccacctacgataacg | 345240 | - | see also 6076 line | | - |
| 22299 | KIAA0063 | NM_014876.3 | 797 | gagagcgagctcaggaagtttct | 345100 | - | - | | - |
| 22300 | KIAA0076 | NM_014780.2 | 4916 | caggtcgagatgggacattgtgc | 343263 | - | cell_cycle | | - |
| 22301 | KIAA0087 | NM_014769.1 | 621 | aggccagtcgagacaattcaata | 343191 | - | - | | - |
| 22302 | KIAA0090 | NM_015047.1 | 2332 | tggcgtcactgggcgtatcatc | 347506 | - | see also 6205 line | | - |
| 22303 | KIAA0092 | NM_014679.1 | 722 | aaggaaaccgcatgcaagctaagg | 342129 | - | see also 5881 line | | - |
| 22304 | KIAA0095 | NM_014669.1 | 1016 | ctgtgtgggcgctaattactac | 342010 | - | see also 5846 line | | - |
| 22305 | KIAA0101 | NM_014736.3 | 360 | ttgcaactgatcacacaaatga | 342890 | - | - | | - |
| 22306 | KIAA0140 | NM_014661.1 | 662 | cagcgtccagcgtcattccaacg | 341886 | - | - | | - |
| 22307 | KIAA0141 | NM_014773.1 | 976 | aggcggtccttattactcagttg | 343220 | - | - | | - |
| 22308 | KIAA0196 | NM_014846.1 | 3098 | ctgcgtatctcgaggctataatg | 344744 | - | see also 6049 line | | - |
| 22309 | KIAA0233 | NM_014745.1 | 1130 | ctgcgtcatcctgtgttgtaaga | 342981 | - | - | | - |
| 22310 | KIAA0252 | NM_015138.2 | 1048 | tcgattatcacggcataagctag | 348575 | - | - | | - |
| 22311 | KIAA0258 | NM_014785.1 | 954 | aggaatcctgcctacatacaact | 343517 | - | see also 5968 line | | - |
| 22312 | KIAA0266 | NM_021645.1 | 2234 | cagaggtcagagagagtacaaac | 381017 | - | - | | - |
| 22313 | KIAA0274 | NM_014845.4 | 611 | aagctaggtatctacgaatattt | 344579 | - | see also 6047 line | | - |
| 22314 | KIAA0276 | NM_015115.1 | 758 | tggtgcaatgtcctagagtttag | 348207 | - | see also 6254 line | | - |
| 22315 | KIAA0319 | NM_014809.1 | 2034 | cggacggagccactaacctctaca | 343864 | - | - | | - |
| 22316 | KIAA0329 | NM_014844.1 | 260 | gagttctgcccgtgtactatct | 344510 | - | - | | - |

Figure 46

| | | | | | | | |
|---|---|---|---|---|---|---|---|
| 22317 | KIAA0355 | NM_014686.1 | 1539 | ctgaggcgacatctagactaaga | 342160 | - | - |
| 22318 | KIAA0391 | NM_014672.1 | 372 | ttgtttggtattcgaagcttcc | 342045 | - | - |
| 22319 | KIAA0399 | NM_015113.2 | 8637 | cagactggccatcaacgattaaa | 348193 | - | see also 5826 line |
| 22320 | KIAA0406 | NM_014657.1 | 2191 | gagaccaaacctactcattagt | 341847 | - | - |
| 22321 | KIAA0408 | NM_014702.1 | 1980 | gagcagtagcccacttagaaatt | 342409 | - | - |
| 22322 | KIAA0409 | NM_015324.2 | 1036 | ttgccgcttggcttcaagtatcc | 351579 | - | - |
| 22323 | KIAA0431 | NM_015251.1 | 996 | tagtaagtcagcaattcgtacg | 350275 | - | - |
| 22324 | KIAA0476 | NM_014856.1 | 1048 | ctggtgtacgaggcagttctacg | 344821 | - | - |
| 22325 | KIAA0483 | NM_015176.1 | 345 | tggagaggtaccataatctatgt | 348924 | - | see also 6268 line |
| 22326 | KIAA0513 | NM_014732.1 | 776 | gagtctgcggacagtgagaatga | 342881 | - | - |
| 22327 | KIAA0555 | NM_014790.1 | 1957 | gagacgatccctccattaatc | 343601 | - | - |
| 22328 | KIAA0562 | NM_014704.1 | 2188 | ccgcgagacgcggttcgaatta | 342617 | - | see also 5897 line |
| 22329 | KIAA0582 | NM_015147.1 | 1371 | gcgaaggaaccggctgactatagg | 348614 | - | - |
| 22330 | KIAA0586 | NM_014749.1 | 2187 | agggccatcgaagcactcttaaa | 343047 | - | - |
| 22331 | KIAA0590 | NM_014714.1 | 1551 | gagtcaaaccgagttcaagttcg | 342657 | - | - |
| 22332 | KIAA0605 | NM_014694.1 | 2034 | gagagcttcttcgttggattatga | 342371 | - | - |
| 22333 | KIAA0625 | NM_015046.4 | 1640 | ggggtggaagccgtcgtcaaatgt | 347261 | - | see also 6202 line |
| 22334 | KIAA0632 | NM_015545.2 | 2086 | acggcttccgagcctattacaag | 353864 | - | - |
| 22335 | KIAA0635 | NM_014645.1 | 1191 | cagttacagaacgaacaacttact | 341621 | - | see also 5819 line |
| 22336 | KIAA0638 | NM_015157.1 | 1017 | ctgtcaccgatggctaatggtg | 348634 | - | - |
| 22337 | KIAA0643 | NM_024793.1 | 348 | atgacacttatctgagaaattt | 390757 | - | - |
| 22338 | KIAA0644 | NM_014817.1 | 1615 | cagcagcagggcgcgatttctagc | 344308 | - | - |
| 22339 | KIAA0648 | NM_015200.1 | 2764 | atgtctgcttgcgattagctgc | 349787 | - | - |
| 22340 | KIAA0649 | NM_014811.1 | 2562 | aaggcgcctagacgagaaagaga | 344185 | - | - |
| 22341 | KIAA0652 | NM_014741.1 | 1461 | gtgacctgacgagtttggatat | 342912 | - | - |
| 22342 | KIAA0672 | NM_014859.1 | 1778 | gcgtcgccacggataatatgatg | 344932 | - | see also 6061 line |
| 22343 | KIAA0683 | NM_016111.1 | 1350 | gcgggacagccgggatgaactgc | 356292 | - | - |
| 22344 | KIAA0685 | NM_014678.2 | 2436 | ttgatgagccagcggaactcaacg | 342115 | - | - |
| 22345 | KIAA0690 | NM_015179.2 | 1191 | acggccctgacgacttatgttcc | 348956 | - | - |
| 22346 | KIAA0720 | NM_020631.2 | 2173 | ctgggtggacaccatttacaatg | 377820 | - | kinase_related |
| 22347 | KIAA0738 | NM_014719.1 | 694 | aggggaacacgagtttctttaaag | 342827 | - | - |
| 22348 | KIAA0746 | NM_015187.1 | 2161 | gaggatctgcgttaatctatga | 349515 | - | see also 6272 line |
| 22349 | KIAA0748 | NM_014796.1 | 1660 | cagaactgcgtcggtctttaagc | 343665 | - | see also 5991 line |
| 22350 | KIAA0753 | NM_014804.1 | 2469 | caggagtgtgttcgtcaaagata | 343767 | - | see also 5998 line |
| 22351 | KIAA0759 | NM_015305.2 | 1348 | ttgtgcgggacctaaattctgt | 351331 | - | see also 6320 line |
| 22352 | KIAA0763 | NM_014869.1 | 2053 | cggactacaccagcgagaaatct | 345059 | - | - |
| 22353 | KIAA0831 | NM_014924.2 | 563 | tcgacgatccatatattagagc | 345771 | - | - |

Figure 46

| 22354 | KIAA0853 | NM_015070.2 | 3576 | gagacgcctcacgtgactataga | 347802 | - | - | - | - |
| 22355 | KIAA0861 | NM_015078.2 | 2305 | cagcgccaactggatcacaatct | 347919 | - | - | - | - |
| 22356 | KIAA0874 | NM_015208.2 | 1947 | ctgatcttgttcggtatgataat | 349881 | - | see also 6278 line | | |
| 22357 | KIAA0889 | NM_015377.1 | 537 | cgggcatccgagtctactatagt | 351938 | - | - | - | - |
| 22358 | KIAA0893 | NM_014969.3 | 1416 | gaggcctatcggcagtgaaatct | 346520 | - | see also 6158 line | | |
| 22359 | KIAA0903 | NM_015252.1 | 2762 | tagtccagcatcgattgttatct | 350361 | - | see also 6292 line | | |
| 22360 | KIAA0907 | NM_014949.1 | 1204 | gagtcttgccggcattacctact | 346278 | - | see also 6147 line | | |
| 22361 | KIAA0922 | NM_015196.2 | 947 | caggatatacgccatttctcaca | 349567 | - | see also 6276 line | | |
| 22362 | KIAA0963 | NM_014963.1 | 1368 | gtgttcgacgagtgtcacaaagc | 346384 | - | - | - | - |
| 22363 | KIAA0970 | NM_014923.2 | 1980 | atggcggagcaaccatcaataaa | 345720 | - | - | - | - |
| 22364 | KIAA0971 | NM_014929.1 | 1121 | atgaatcaccgatctcttatact | 345927 | - | - | - | - |
| 22365 | KIAA0977 | NM_014900.1 | 2302 | aggagtcaaggcaccctaattat | 345416 | - | - | - | - |
| 22366 | KIAA0982 | NM_014023.1 | 1571 | ttgaccggcaagccattggttgg | 339315 | - | see also 5462 line | | |
| 22367 | KIAA1007 | NM_016284.2 | 4507 | gtggatcgatcaattaagattgc | 356799 | - | see also 6780 line | | |
| 22368 | KIAA1018 | NM_014967.1 | 2364 | gggatcgactggcccttaattta | 346499 | - | see also 6156 line | | |
| 22369 | KIAA1023 | NM_152558.1 | 1921 | atgattccgacgatattgtcatt | 418108 | - | - | - | - |
| 22370 | KIAA1033 | NM_015275.1 | 1744 | aagcaacggcgacttattgtttc | 350784 | - | - | - | - |
| 22371 | KIAA1043 | NM_015281.1 | 736 | tcgccgcaataacctgtttaaca | 350867 | - | see also 6311 line | | |
| 22372 | KIAA1049 | NM_014972.1 | 276 | ccgcttcgagctgataaacattg | 346557 | - | - | - | - |
| 22373 | KIAA1052 | NM_014956.1 | 582 | tcgccaacgggcagtctatgtgg | 346326 | - | see also 6151 line | | |
| 22374 | KIAA1068 | NM_015332.2 | 961 | tggacaggcttacctttgactac | 351627 | - | - | - | - |
| 22375 | KIAA1074 | NM_014915.1 | 3874 | gaggttacgtcacgttatcgtat | 345611 | - | - | - | - |
| 22376 | KIAA1115 | NM_014931.1 | 2201 | acgccaacctacttgagatatgc | 345969 | - | - | - | - |
| 22377 | KIAA1117 | NM_015018.2 | 1039 | aggccactcgaccggatatgatc | 346802 | - | - | - | - |
| 22378 | KIAA1143 | NM_020696.1 | 141 | gggacccaccgtagagactaaga | 378154 | - | - | - | - |
| 22379 | KIAA1160 | NM_020701.1 | 236 | atgacggccttagcaagatttcg | 378166 | - | - | - | - |
| 22380 | KIAA1181 | NM_020462.1 | 233 | gcgtgtgaacgagctctatgtcg | 377674 | - | - | - | - |
| 22381 | KIAA1185 | NM_020710.1 | 1389 | cagacggtgatgtgatttccttc | 378263 | - | - | - | - |
| 22382 | KIAA1191 | NM_020444.2 | 502 | gtgattccagagcgcaagtatca | 377475 | - | - | - | - |
| 22383 | KIAA1194 | NM_015455.2 | 544 | aagaagcttaagcgcatctttgt | 353187 | - | - | - | - |
| 22384 | KIAA1199 | NM_018689.1 | 3936 | tggaacgacttcgcttacattga | 372635 | - | see also 7562 line | | |
| 22385 | KIAA1219 | NM_020336.1 | 995 | cagattgctacgctttacatatg | 376552 | - | - | - | - |
| 22386 | KIAA1221 | NM_032186.2 | 3685 | atgaggatagtccgttgatgtat | 398630 | - | - | - | - |
| 22387 | KIAA1244 | NM_020340.2 | 3582 | ccgacctttcgagcgcattatgc | 376726 | - | - | - | - |
| 22388 | KIAA1279 | NM_015634.1 | 1332 | gagacggtgcaagatgcataaac | 354808 | - | - | - | - |
| 22389 | KIAA1280 | NM_015691.2 | 3213 | aagacgaacccttcgctataagc | 355132 | - | - | - | - |
| 22390 | KIAA1287 | NM_020748.1 | 3155 | ctgacggtcgcaggtattccatc | 378454 | - | see also 7930 line | | |

Figure 46

| | | | | | | | |
|---|---|---|---|---|---|---|---|
| 22391 | KIAA1295 | NM_017963.2 | 330 | atgccagttacggtagtcatt | 364890 | - | - |
| 22392 | KIAA1318 | NM_020769.1 | 697 | atgtccgcaacgctaatggtagc | 378619 | - | - |
| 22393 | KIAA1322 | NM_020773.1 | 523 | aggctatgactcggtaaagctct | 378742 | - | see also 7950 line |
| 22394 | KIAA1324 | NM_020775.2 | 2506 | gggaataccggacgtgatcttct | 378910 | - | - |
| 22395 | KIAA1336 | NM_020779.2 | 1739 | tagccgtcttgctatcatagaca | 378981 | - | see also 7964 line |
| 22396 | KIAA1383 | NM_019090.1 | 2303 | tagtagtgacaggagttctatcc | 375202 | - | - |
| 22397 | KIAA1404 | NM_021035.1 | 2221 | aagggctacatgatgtatcatga | 380419 | - | transcription_regulator |
| 22398 | KIAA1407 | NM_020817.1 | 2889 | aaggtaccacgagctatatcagc | 379186 | - | - |
| 22399 | KIAA1446 | NM_020836.2 | 416 | aggacaacgagctctataggaag | 379383 | - | kinase_related |
| 22400 | KIAA1449 | NM_020839.2 | 150 | cagccggtcgagactctatcata | 379391 | - | see also 7986 line |
| 22401 | KIAA1465 | NM_020851.1 | 240 | ccggactgcgcttacaaagagttg | 379612 | - | - |
| 22402 | KIAA1468 | NM_020854.2 | 2051 | caggagtccttacgtgttatatt | 379686 | - | see also 7995 line |
| 22403 | KIAA1508 | NM_020880.2 | 485 | aagagtcacccctgtgagatttg | 379857 | - | - |
| 22404 | KIAA1518 | NM_015493.3 | 1971 | cggcaggcgtgcgatctatcatg | 553568 | - | - |
| 22405 | KIAA1524 | NM_020890.1 | 1093 | tggctctactgcgctggttaagc | 380015 | - | see also 8016 line |
| 22406 | KIAA1533 | NM_020895.2 | 1692 | gttggagcggcattgaagagactatt | 380081 | - | - |
| 22407 | KIAA1536 | NM_020898.1 | 1893 | gggggtgaggaggccaacttact | 380098 | - | - |
| 22408 | KIAA1587 | NM_020932.1 | 2106 | atggcaagcttacttagactaca | 380171 | - | see also 8037 line |
| 22409 | KIAA1598 | NM_018330.2 | 369 | tgggttctgtatccagtgtaaca | 370435 | - | - |
| 22410 | KIAA1608 | NM_024820.1 | 1317 | cagtttattgatggtcgattaga | 391098 | - | see also 8765 line |
| 22411 | KIAA1609 | NM_020947.2 | 1049 | gagggtgaagccccagtttcaagg | 380218 | - | - |
| 22412 | KIAA1627 | NM_020961.2 | 618 | atgtacttacagccgatataga | 380262 | - | - |
| 22413 | KIAA1701 | NM_030639.1 | 1247 | aggcaagccctccttcatatatt | 394784 | - | see also 8914 line |
| 22414 | KIAA1712 | NM_030633.1 | 349 | aggtgctccgtcgctaaattat | 394734 | - | - |
| 22415 | KIAA1715 | NM_030650.1 | 604 | aaggtttgatccggactcaaaga | 394838 | - | see also 8922 line |
| 22416 | KIAA1720 | NM_030645.1 | 832 | gagtcggcgaggaaaactgaatac | 394821 | - | - |
| 22417 | KIAA1737 | NM_033426.2 | 1209 | caggcaactcaggtagaaactaga | 403896 | - | see also 9424 line |
| 22418 | KIAA1754 | NM_033397.1 | 1696 | cagcgctcattagcgagtattcc | 403777 | - | - |
| 22419 | KIAA1754L | NM_178495.3 | 1288 | cggcagtcttgggggaagttgtagt | 430840 | - | - |
| 22420 | KIAA1797 | NM_017794.2 | 1583 | ctgtaaccagtatgtatggtaca | 362482 | - | - |
| 22421 | KIAA1827 | NM_032423.1 | 2507 | cagaaatcagaccttatacgaca | 400214 | - | transcription_regulator |
| 22422 | KIAA1892 | NM_015397.1 | 907 | tggctcacgtgatggttctatgg | 352275 | - | see also 6404 line |
| 22423 | KIAA1892L | NM_178470.2 | 387 | tcgctcgatgcgccactatctga | 430830 | - | - |

870

Figure 46

| 22424 | KIAA1893 | NM_052899.1 | 776 | atggatcccatgactgtaagaaa | 404774 | - | - | - | - |
|---|---|---|---|---|---|---|---|---|---|
| 22425 | KIAA1913 | NM_052913.2 | 2987 | gagattggcagcaaatacgatcg | 405288 | - | - | - | - |
| 22426 | KIAA1919 | NM_153369.1 | 1621 | tagctccgggctaaatgaatatg | 423150 | - | - | - | - |
| 22427 | KIAA1924 | NM_153239.2 | 913 | ccggtgctacggccatctcaaga | 422469 | - | - | - | - |
| 22428 | KIAA2010 | NM_017936.2 | 1285 | gtgcgaagtgctgctactgatat | 364328 | - | - | - | - |
| 22429 | KIAA2024 | NM_172070.2 | 2178 | ttgatcaatgcatcggtaattat | 424253 | - | - | - | - |
| 22430 | KIBRA | NM_015238.1 | 2037 | gggtgcgacccgaattcagattg | 350150 | - | - | - | - |
| 22431 | KIF24 | NM_018278.1 | 151 | cagcctgcgcatcaaatctcagg | 369642 | - | - | - | - |
| 22432 | KIRREL | NM_018240.3 | 1733 | gagagccacttacgatgcattct | 368887 | - | - | - | - |
| 22433 | KIRREL2 | NM_032123.4 | 2042 | cagactcacgtgtgacatctttc | 397897 | - | - | - | - |
| 22434 | KIRREL3 | NM_032531.1 | 978 | ccgcacggttgacgtctactttg | 400559 | - | see also 9238 line | | |
| 22435 | KLF8 | NM_007250.2 | 745 | atgctacaagctccaatacgtcc | 337329 | - | - | DNA binding | - |
| 22436 | KLHDC1 | NM_172193.1 | 666 | gcggacgtgttctgcaaactagg | 424288 | - | see also 10039 line | | |
| 22437 | KLHDC2 | NM_014315.1 | 570 | gtgatgttcctccttctatgtca | 340157 | - | see also 5618 line | | |
| 22438 | KLHDC3 | NM_057161.2 | 231 | ctgtcgggcatcgggtatactcc | 406097 | - | - | - | - |
| 22439 | KLIP1 | NM_024629.2 | 1273 | aagccatctgcgaaatatcaacc | 388280 | - | - | - | - |
| 22440 | KRT25A | NM_181534.2 | 593 | ctgatgtcaatgggttacgaaga | 432404 | - | - | - | - |
| 22441 | KRT25B | NM_181539.2 | 889 | gagtgcaacgctgcaacaacaga | 432582 | - | - | - | - |
| 22442 | KRT25C | NM_181537.2 | 996 | cagtccctcttagcaacgaaaca | 432565 | - | - | - | - |
| 22443 | KRT25D | NM_181535.2 | 450 | tagcagatatcacctaacaattg | 432416 | - | - | - | - |
| 22444 | KRT3 | NM_057088.1 | 1223 | gggatgacctaagaaataccaag | 406096 | - | - | structural molecule | - |
| 22445 | KRT6L | NM_175834.2 | 44 | ccgtctctcggcaaacatactcc | 429273 | - | - | - | - |
| 22446 | KRTAP12-3 | NM_198697.1 | 222 | gagctgcaggcccatcatatatg | 439879 | - | - | - | - |
| 22447 | KRTAP13-3 | NM_181622.1 | 264 | ctgcaattcttgcctgactatgc | 432615 | - | - | - | - |
| 22448 | KRTAP13-4 | NM_181600.1 | 453 | tggatctcgcttctatcaattca | 432592 | - | receptor_acitivity | - | - |
| 22449 | KRTAP15-1 | NM_181623.1 | 58 | aggtatccagtttccacttataa | 432624 | - | - | - | - |
| 22450 | KRTAP18-8 | NM_198695.1 | 37 | ctgcaccaggacgtatgtgattg | 439877 | - | - | - | - |
| 22451 | KRTAP19-3 | NM_181609.1 | 195 | ctgctaccgcccatcatactatg | 432596 | - | - | - | - |
| 22452 | KRTAP19-4 | NM_181610.1 | 165 | aggatatggattctcaattctac | 432597 | - | - | - | - |
| 22453 | KRTAP19-6 | NM_181612.1 | 141 | atgccgtgaaggatatggattct | 432603 | - | - | - | - |

Figure 46

| | | | | | | | |
|---|---|---|---|---|---|---|---|
| 22454 | KRTAP20-1 | NM_181615.1 | 1 | atgatttactacagccaacatta | 432605 | - | |
| 22455 | KRTAP21-2 | NM_181617.1 | 55 | tggagttcggctgtggatatgg | 432606 | - | |
| 22456 | KRTAP3-1 | NM_031958.1 | 202 | ctgctgcaagcctgatacctatg | 397546 | - | |
| 22457 | KRTAP6-2 | NM_181604.1 | 165 | atgcggctctggctactactatt | 432594 | - | |
| 22458 | KSP37 | NM_031950.2 | 327 | ttgctgctgacccaaaccttac | 397466 | - | |
| 22459 | LACE1 | NM_145315.2 | 1550 | ttgcatttcagccgcacaatttcc | 414410 | - | see also 9789 line |
| 22460 | LACRT | NM_033277.1 | 143 | tgggacctcaagcctaatgaag | 403649 | - | |
| 22461 | LAIR1 | NM_002287.2 | 242 | gggagagtagatccacatacaat | 330746 | - | |
| 22462 | LALP1 | NM_020354.1 | 1176 | ctgagtcccgacaatccattct | 376847 | - | see also 7844 line |
| 22463 | LANCL2 | NM_018697.2 | 881 | ttgaatcggtaagactttgtca | 372682 | - | see also 7568 line |
| 22464 | LAP1B | NM_015602.2 | 797 | acgtcgaccccctcctaagatacc | 354424 | - | |
| 22465 | LARP | NM_015315.2 | 2472 | tcgtaggcgtgccttaatgagc | 351462 | - | |
| 22466 | LAS1 | NM_018272.1 | 264 | ctgataccactatgatcatgt | 369609 | - | |
| 22467 | LAX | NM_017773.1 | 535 | aggattcgcatgattatgtcaat | 362176 | - | |
| 22468 | LCMT2 | NM_014793.3 | 1183 | gaggcatttcctcgtaaatcc | 343633 | - | |
| 22469 | LCN6 | NM_198946.1 | 295 | gagtgtcatgacctgataaagc | 440142 | - | |
| 22470 | LENG8 | NM_052925.1 | 2242 | tggccaccgccttggcgttaagg | 405332 | - | see also 9453 line |
| 22471 | LGI2 | NM_018176.2 | 914 | acgacgagagttgaccaaatt | 368013 | - | see also 7299 line |
| 22472 | LGI4 | NM_139284.1 | 787 | gtgattgaggacgatgcaattgc | 409614 | - | |
| 22473 | LHFPL1 | NM_178175.2 | 644 | gtgccttataccctttaggatgg | 430617 | - | |
| 22474 | LHFPL2 | NM_005779.1 | 480 | ctgtgggctgttgcaaggaattg | 334247 | - | |
| 22475 | LHFPL3 | NM_199000.1 | 525 | cggccactgtgtacaagatatgt | 440583 | - | |
| 22476 | LIMS2 | NM_017980.2 | 459 | tgatctgcggtgagttcatca | 365027 | - | |
| 22477 | LIP8 | NM_053051.1 | 2871 | gggccttccatccgatcatagg | 405869 | - | |
| 22478 | LIPE | NM_005357.2 | 2413 | tggctcaacagggaacggatct | 333649 | - | hydrolase |
| 22479 | LIR9 | NM_021250.2 | 616 | ctgagattcgacaggttcattct | 380904 | - | |
| 22480 | LL5beta | NM_145753.1 | 765 | tggcgcttcaaccaagttaact | 414785 | - | see also 9799 line |
| 22481 | LLGL2 | NM_004524.1 | 181 | gcgggacctgttccagtttaaca | 332613 | - | |
| 22482 | LMOD3 | NM_198271.1 | 882 | cagaatggtggtcactaatctgc | 437394 | - | |
| 22483 | LNIR | NM_030916.1 | 1361 | gtgctcagtccgataccatcg | 395392 | - | receptor_acitivity |
| 22484 | LOC112476 | NM_145239.1 | 459 | aagcccaggagggaatcaaagg | 413806 | - | |
| 22485 | LOC112817 | NM_138413.2 | 746 | gtggtgacccttgctactatcg | 407984 | - | |
| 22486 | LOC112869 | NM_138414.1 | 266 | gagcgaacaacttagtgaaca | 407992 | - | |

Figure 46

| 22487 | LOC113174 | NM_138421.1 | 254 | cagagtatgggatatgtcaatgg | 408052 | - | - | - | |
| 22488 | LOC113444 | NM_138428.2 | 633 | atgaacattggctgtcaatctct | 408109 | - | - | - | |
| 22489 | LOC113612 | NM_183075.1 | 1327 | gtgctccaagggtataccattcc | 435619 | - | - | - | |
| 22490 | LOC113655 | NM_138431.1 | 285 | ggggctggccaaggttctgtacg | 408114 | - | - | - | |
| 22491 | LOC113730 | NM_138433.2 | 1651 | ccgccgtgatgcgctacaacaca | 408116 | - | - | - | |
| 22492 | LOC113828 | NM_138435.1 | 706 | aagtggccactggatcttacagg | 408122 | - | - | - | |
| 22493 | LOC114659 | NM_052888.1 | 1123 | gaggcggaaccttcttctacagc | 404607 | - | - | - | |
| 22494 | LOC114926 | NM_138436.2 | 123 | gtgacgatggttctattgattat | 408128 | - | - | - | |
| 22495 | LOC114928 | NM_138437.1 | 962 | cagggctaaacatcagactaatc | 408153 | - | - | - | |
| 22496 | LOC114990 | NM_138440.1 | 283 | tggggctgtacgtctttgagaac | 408189 | - | - | - | |
| 22497 | LOC115098 | NM_138442.2 | 221 | ctggaaggacgacgacaaacacg | 408211 | - | - | - | |
| 22498 | LOC115106 | NM_138443.2 | 765 | ttggagtcctatttagacttaat | 408234 | - | cell_cycle | - | |
| 22499 | LOC115265 | NM_145244.2 | 505 | gcggcttgcgaggttgtgttatg | 413823 | - | - | - | |
| 22500 | LOC115811 | NM_138451.1 | 788 | agggataaggcccagttcttaca | 408260 | - | kinase_related | - | |
| 22501 | LOC116236 | NM_198147.1 | 479 | tgggtggtaggaccttgtgttcg | 437110 | - | - | - | |
| 22502 | LOC116238 | NM_138463.2 | 752 | tggacgtgatgatcataatctac | 408308 | - | - | - | |
| 22503 | LOC118430 | NM_058173.1 | 285 | ttggggatctcccgaatggtaga | 406199 | - | - | - | |
| 22504 | LOC119395 | NM_182494.1 | 247 | aagctcggagtcggtgatgaatg | 433907 | - | - | - | |
| 22505 | LOC119504 | NM_173473.1 | 314 | acgcttaaacaggtgaaacatga | 424922 | - | - | - | |
| 22506 | LOC119710 | NM_138787.1 | 434 | atgctaacctccaagtttagtcc | 408758 | - | - | - | |

Figure 46

| 22507 | LOC120224 | NM_138788.2 | 202 | tcgtcgaagccgcaattaggact | 408764 | - | - | - | |
| 22508 | LOC120379 | NM_138789.2 | 78 | tggaacctcctagatgatctagc | 408782 | - | - | - | |
| 22509 | LOC120526 | NM_181706.2 | 240 | cagaagttcatcgaaattgatca | 432845 | - | - | - | |
| 22510 | LOC122258 | NM_145248.2 | 405 | atggcgatccttctgagaattat | 413886 | - | - | - | |
| 22511 | LOC123169 | NM_138792.2 | 1724 | ctggaacctgatcgatacgatga | 408846 | - | - | - | |
| 22512 | LOC124220 | NM_145252.1 | 216 | ctgaagactacgaccatgaaatc | 413894 | - | - | - | |
| 22513 | LOC124402 | NM_145253.1 | 299 | ctgacgctctcaccatgttctcc | 413909 | - | - | - | |
| 22514 | LOC124446 | NM_194280.1 | 532 | caggacaactggtccttatcaca | 436236 | - | - | - | |
| 22515 | LOC124773 | NM_181707.1 | 681 | aagatcccaggaccatgtcaaga | 432854 | - | - | - | |
| 22516 | LOC125476 | NM_194281.1 | 164 | gggctatggcgccagtaagaaga | 436239 | - | - | - | |
| 22517 | LOC126248 | NM_173479.1 | 560 | ttgccgcatcctatgataagaca | 424956 | - | - | - | |
| 22518 | LOC126353 | NM_173481.2 | 2048 | acgggtgacccgtcacaagaacg | 424975 | - | - | - | |
| 22519 | LOC126731 | NM_145257.1 | 678 | ctgtgcacgagattcatgaatca | 413934 | - | - | - | |
| 22520 | LOC128153 | NM_138796.2 | 54 | aggtcgtcgactgtaggaaatca | 408853 | - | - | - | |
| 22521 | LOC128344 | NM_181643.1 | 164 | atggcttggcatacgacttatct | 432633 | - | - | - | |
| 22522 | LOC129026 | NM_080842.1 | 163 | gagatgcgacacccacttaaact | 406861 | - | - | - | |
| 22523 | LOC129285 | NM_152994.2 | 1424 | tggactccttcggacaaactaca | 421726 | - | see also 9970 line | | |
| 22524 | LOC129531 | NM_138798.1 | 595 | ctgttggaagttcaatactcttc | 408901 | - | - | - | |
| 22525 | LOC129642 | NM_138799.2 | 254 | tggtttcgaacttatctacattc | 408913 | - | see also 9636 line | | |
| 22526 | LOC130026 | NM_138468.3 | 1381 | atgtggctggagcgttcaacaac | 408347 | - | see also 9624 line | | |

EP 1 752 536 A1

Figure 46

| | | | | | | | | | |
|---|---|---|---|---|---|---|---|---|---|
| 22527 | LOC130589 | NM_138801.1 | 523 | atggcggagagctcatagtcaac | 408986 | - | - | | |
| 22528 | LOC130888 | NM_174899.3 | 240 | ttggtgcaagaatattagactat | 428545 | - | - | | |
| 22529 | LOC130940 | NM_138803.2 | 870 | aagactaacccgcttagtgaact | 409018 | - | - | | |
| 22530 | LOC130951 | NM_138804.2 | 1065 | tagcgggctctgcgagtcattga | 409070 | - | - | | - |
| 22531 | LOC131118 | NM_145261.1 | 360 | gagatgctcatcgacgaattatg | 413968 | - | - | | - |
| 22532 | LOC131368 | NM_175056.1 | 279 | gtggagtgcaggctattacgatg | 428910 | - | see also 10141 line | | |
| 22533 | LOC132200 | NM_138808.1 | 515 | gagaatgcgactatacaacttga | 409125 | - | - | | - |
| 22534 | LOC132321 | NM_173487.1 | 335 | gtgaagcatgagcccgtatttat | 425001 | - | - | | - |
| 22535 | LOC132671 | NM_145263.1 | 1761 | tggaattcggtgcgatctgtaag | 414019 | - | - | | - |
| 22536 | LOC133619 | NM_130809.2 | 883 | atggctccctatatcaaatctgg | 406993 | - | - | | - |
| 22537 | LOC133957 | NM_145265.2 | 717 | cagccggtctcaccgacatattt | 414033 | - | - | | - |
| 22538 | LOC134218 | NM_194283.1 | 266 | ccgtcagctggtagctttcattc | 436295 | - | - | | - |
| 22539 | LOC134285 | NM_173490.3 | 683 | taggtgactcggtaataatattt | 425132 | - | receptor_acitivity | | - |
| 22540 | LOC134548 | NM_175873.2 | 716 | tggcccgaggcttgaagaagtcg | 429381 | - | - | | - |
| 22541 | LOC136263 | NM_145268.2 | 421 | aggcgccagtctcagttcaatga | 414062 | - | - | | - |
| 22542 | LOC142678 | NM_080875.1 | 2061 | aggtgccaaacatcgatgttacc | 406884 | - | kinase_related | | - |
| 22543 | LOC143241 | NM_138812.1 | 247 | cagtggatccgatagaatattta | 409201 | - | kinase_related、signal _transduction | | - |
| 22544 | LOC144100 | NM_175058.2 | 1831 | tggagctgcggtcgtatgtcagt | 428968 | - | - | | - |
| 22545 | LOC144233 | NM_181708.1 | 247 | gtgtggctctatacaaacacttc | 432858 | - | see also 10249 line | | |
| 22546 | LOC144501 | NM_182507.1 | 494 | cagatatcctactctcctttatg | 434015 | - | | | |

Figure 46

| 22547 | LOC144747 | NM_194286.1 | 666 | ctgctcgtcgtgaggatttatct | 436361 | - | - | | - | |
|---|---|---|---|---|---|---|---|---|---|---|
| 22548 | LOC145497 | NM_194287.1 | 696 | ggggtaccaaggctattgctata | 436365 | - | - | | - | |
| 22549 | LOC146174 | NM_173501.1 | 322 | tcgtgcgacagtgtcaaacaatc | 425173 | - | - | | - | |
| 22550 | LOC146325 | NM_145270.1 | 369 | ctggggcaaaccctacaactaca | 414072 | - | - | | - | |
| 22551 | LOC146562 | NM_139170.1 | 888 | ttggaagcctgtctttcaacacc | 409414 | - | - | | - | |
| 22552 | LOC146845 | NM_145054.2 | 1430 | caggccgactgatgtatgtcatt | 413288 | - | - | | - | |
| 22553 | LOC146853 | NM_145272.2 | 754 | gtgcagagcccagcataacctag | 414075 | - | - | | - | |
| 22554 | LOC147111 | NM_178493.3 | 1435 | ctgcctctcccatgagatcatca | 430834 | - | - | | - | |
| 22555 | LOC147965 | NM_174905.2 | 853 | gagccgacctgtcgtgcttatgg | 428574 | - | - | | - | |
| 22556 | LOC148066 | NM_181710.2 | 70 | ctgccgtccggagcacttaatgc | 432870 | - | - | | - | |
| 22557 | LOC148137 | NM_144692.1 | 331 | ccgtggtggccaagtatataaac | 411339 | - | - | | - | |
| 22558 | LOC148523 | NM_144697.2 | 963 | cagatgggggaacgttacctagg | 411454 | - | - | | - | |
| 22559 | LOC148738 | NM_145277.2 | 882 | ctgcctacattggcacaactata | 414097 | - | - | | - | |
| 22560 | LOC148823 | NM_145278.1 | 248 | gtgctacactgtcattaatcaca | 414106 | - | - | | - | |
| 22561 | LOC149469 | NM_182518.1 | 278 | ttggcagtatcggtttgtcattg | 434061 | - | - | | - | |
| 22562 | LOC150159 | NM_139173.1 | 304 | gaggagtattgaatgtaattatt | 409421 | - | - | | - | |
| 22563 | LOC150379 | NM_138814.1 | 667 | ctggggctcatatgtctcatacc | 409220 | - | - | | - | |
| 22564 | LOC150759 | NM_175853.2 | 172 | cagaaactactgcaaacacttct | 429279 | - | - | | - | |
| 22565 | LOC151194 | NM_145280.3 | 701 | atgaacgggataacaacttctta | 414126 | - | see also 9786 line | | | |
| 22566 | LOC151534 | NM_138482.1 | 781 | cggcgtctgcagcctcttcatcc | 408399 | - | - | | - | |

EP 1 752 536 A1

Figure 46

| | | | | | | | | | |
|---|---|---|---|---|---|---|---|---|---|
| 22567 | LOC151636 | NM_138287.2 | 1144 | aggttgatagtgcccactataaa | 407415 | - | - | - | |
| 22568 | LOC151648 | NM_138484.1 | 742 | cagtggctctgcctaaacgtagg | 408421 | - | - | - | |
| 22569 | LOC151835 | NM_153635.1 | 330 | cggacggaccgaggtgattgata | 423455 | - | - | - | |
| 22570 | LOC151963 | NM_178496.2 | 810 | ctgctgcaccattgtagatcaca | 430869 | - | see also 10193 line | | |
| 22571 | LOC152185 | NM_144718.2 | 405 | ctggtacgccgtcatgaaataca | 411686 | - | see also 9734 line | | |
| 22572 | LOC152687 | NM_182524.1 | 551 | gagtttaccagtgcttgtcaact | 434160 | - | transcription_regulator | - | - |
| 22573 | LOC153684 | NM_194290.1 | 551 | acgactagcagttgacacaaaca | 436383 | - | - | - | - |
| 22574 | LOC154807 | NM_173517.1 | 417 | aagcgctgtggcggctttgatcc | 425318 | - | - | - | - |
| 22575 | LOC157378 | NM_194291.1 | 1134 | tggcaaacacgtcttagtacaca | 436396 | - | - | - | - |
| 22576 | LOC157657 | NM_177965.2 | 202 | atgatcttgacagtcttattaat | 430301 | - | - | - | - |
| 22577 | LOC158427 | NM_139246.3 | 1403 | gagcccgctgggaccagtataaa | 409600 | - | see also 9683 line | | |
| 22578 | LOC159091 | NM_138819.1 | 627 | cagccgccttcatcaaatcaaac | 409260 | - | - | - | - |
| 22579 | LOC161577 | NM_198524.1 | 716 | gagacagcagcgaacaattaata | 439117 | - | - | - | - |
| 22580 | LOC162427 | NM_178126.2 | 411 | aagacccgacgcattagacaatg | 430477 | - | see also 10179 line | | |
| 22581 | LOC165257 | NM_182528.1 | 1425 | aggctcacggaggcaataataac | 434173 | - | - | - | - |
| 22582 | LOC165324 | NM_181713.3 | 375 | ccgtcaacgacgatttcagaagt | 432887 | - | - | - | - |
| 22583 | LOC168850 | NM_176814.3 | 2296 | tcgtcggaaacgtgatgtgatac | 429763 | - | - | - | - |
| 22584 | LOC169355 | NM_194294.1 | 605 | aaggaatcttgcccttccatttg | 436496 | - | - | - | - |
| 22585 | LOC169611 | NM_182487.1 | 1077 | gcgcggacactcggacattgact | 433886 | - | receptor_acitivity | - | |
| 22586 | LOC170393 | NM_173541.1 | 247 | aagcacgtcgcccatttctcatc | 425742 | - | - | - | - |

Figure 46

| 22587 | LOC196264 | NM_198275.1 | 644 | gtgtatggcgaggctttgtgtcc | 437419 | - | - | - | |
| 22588 | LOC196463 | NM_173542.2 | 1159 | gtggatgatcgtggactacaagg | 425747 | - | - | - | |
| 22589 | LOC199675 | NM_174918.1 | 900 | tggtcaggagcaagattgataga | 428659 | - | - | - | |
| 22590 | LOC200030 | NM_183372.1 | 3569 | caggactcactggatgttattca | 435666 | - | - | - | |
| 22591 | LOC200186 | NM_181715.1 | 664 | cagccgacgtgggggtattctgg | 432976 | - | - | - | |
| 22592 | LOC201191 | NM_174920.2 | 1458 | cagctggacggaagcaaactaaa | 428668 | - | - | - | |
| 22593 | LOC201243 | NM_175734.2 | 618 | tgggctggatgctgtatgactcc | 429169 | - | - | - | |
| 22594 | LOC201292 | NM_173547.2 | 1050 | cagcgccaaccgtcacttctatc | 425836 | - | see also 10082 line | | |
| 22595 | LOC201895 | NM_174921.1 | 405 | aagccaaccagtcctcataatgg | 428678 | - | - | | |
| 22596 | LOC203522 | NM_182540.1 | 1265 | gtgaggttctcctgtgtagattg | 434265 | - | see also 10268 line | | |
| 22597 | LOC205251 | NM_174925.1 | 1067 | gcgagacagatgggactattagt | 428698 | - | - | | |
| 22598 | LOC220032 | NM_182833.1 | 1401 | gtgggccgtttagtatccattga | 435424 | - | - | - | |
| 22599 | LOC220070 | NM_145308.2 | 350 | gtgaaggagaggtgtgttgtaag | 414340 | - | - | - | |
| 22600 | LOC220074 | NM_145309.1 | 413 | tggatcgacctgtcctttaatga | 414350 | - | - | - | |
| 22601 | LOC220929 | NM_182755.1 | 1413 | ttgtccacccaccaaacttgatc | 434948 | - | - | - | |
| 22602 | LOC221044 | NM_145314.1 | 427 | gtggcgccagtggcactatgacg | 414359 | - | - | - | |
| 22603 | LOC221711 | NM_194299.1 | 488 | ccgtcttgacagatcaataaata | 436565 | - | - | - | |
| 22604 | LOC222967 | NM_173565.1 | 719 | tggccggacggcagcatgtatga | 426194 | - | - | - | |
| 22605 | LOC223075 | NM_194300.1 | 2255 | tggtaacccacgtcacagaaatg | 436610 | - | - | | |
| 22606 | LOC223082 | NM_147128.3 | 1595 | aagccacgaataacctataatga | 415010 | - | see also 9806 line | | |

Figure 46

| | | | | | | | |
|---|---|---|---|---|---|---|---|
| 22607 | LOC253012 | NM_198151.1 | 879 | gaggactgacaatactacatata | 437134 | - | see also 10311 line |
| 22608 | LOC253559 | NM_153184.1 | 1424 | gagtggtagggagctaaacattc | 422080 | - | - |
| 22609 | LOC253982 | NM_181718.3 | 969 | ctgagggggcttcgaagctttat | 433002 | - | - |
| 22610 | LOC254531 | NM_153613.1 | 652 | tggagcccttcttcgattcaacc | 423395 | - | - |
| 22611 | LOC255104 | NM_181719.1 | 956 | gtggctcgcttctggcaaatacc | 433016 | - | see also 10250 line |
| 22612 | LOC255743 | NM_198278.1 | 544 | ctgtctcaacggatatatgctca | 437464 | - | see also 10315 line |
| 22613 | LOC256643 | NM_198279.1 | 1528 | gagggaaaccaccgaaacacaag | 437523 | - | - |
| 22614 | LOC257106 | NM_181720.1 | 904 | aagtggaggtctatcttcaattt | 433027 | - | - |
| 22615 | LOC282966 | NM_182554.1 | 376 | cagattggatcatgtatccattt | 434417 | - | - |
| 22616 | LOC283241 | NM_175893.2 | 409 | ttgcctgtgtttcgctttattcc | 429515 | - | - |
| 22617 | LOC283385 | NM_173855.2 | 154 | cggcctgcggagccaggtatacg | 428508 | - | - |
| 22618 | LOC283476 | NM_175896.2 | 284 | gtgctcacaactccctattaagg | 429525 | - | - |
| 22619 | LOC283487 | NM_178514.2 | 742 | atggagacggaccgagttgaagg | 430969 | - | - |
| 22620 | LOC283537 | NM_181785.1 | 1476 | gtggttcgttcgactgaacaagg | 433363 | - | - |
| 22621 | LOC283849 | NM_178516.2 | 2210 | tggctggcctgcggcaacaattt | 430982 | - | - |
| 22622 | LOC283932 | NM_175901.2 | 323 | acgaggagactaccgtaaagatg | 429534 | - | - |
| 22623 | LOC284001 | NM_198082.1 | 626 | gaggtgagcgagctcaagataga | 436997 | - | - |
| 22624 | LOC284033 | NM_175903.2 | 552 | atgaaagggctgccaatcactcg | 429543 | - | - |
| 22625 | LOC284296 | NM_175908.3 | 338 | aagatgcctctgtcctatgatgt | 429550 | - | - |
| 22626 | LOC284345 | NM_198850.1 | 287 | aggctacgcctccgatagctatg | 439928 | - | - |

Figure 46

| | | | | | | | | | |
|---|---|---|---|---|---|---|---|---|---|
| 22627 | LOC284361 | NM_175063.2 | 1394 | aggcctgatggatgtacatcatt | 428978 | - | - | - | |
| 22628 | LOC284612 | NM_198280.1 | 307 | gggcaggccgatgctattggtgc | 437534 | - | - | - | |
| 22629 | LOC284680 | NM_182581.1 | 326 | gaggcggaactgcactatctacc | 434509 | - | - | - | |
| 22630 | LOC284697 | NM_183242.1 | 1345 | atgttgattcagtccttagtaaa | 435665 | - | - | - | |
| 22631 | LOC284837 | NM_194310.1 | 709 | caggcacaatcctctcactttct | 436690 | - | - | - | |
| 22632 | LOC285498 | NM_194439.1 | 564 | ctggagtcgatggaagttgatct | 436891 | - | - | - | |
| 22633 | LOC285513 | NM_198281.1 | 1662 | aagctcgcaggtactaattctag | 437552 | - | - | - | |
| 22634 | LOC285533 | NM_173662.1 | 832 | tgggttacttggcgatcatgttt | 427165 | - | - | - | |
| 22635 | LOC285636 | NM_175921.2 | 752 | cagtggctgacaaggcatatagg | 429575 | - | - | - | |
| 22636 | LOC285671 | NM_178532.2 | 1063 | cagtgtattctgaccatactaat | 431103 | - | - | - | |
| 22637 | LOC285908 | NM_181722.1 | 698 | atgcccgaacaaccatagcttct | 433032 | - | - | - | |
| 22638 | LOC286046 | NM_173683.2 | 962 | cagagccgtaacggaacaacagg | 427380 | - | see also 10109 line | | |
| 22639 | LOC286148 | NM_181787.1 | 666 | ttgcgtggttcgttattaacagg | 433395 | - | - | - | |
| 22640 | LOC338773 | NM_181724.1 | 810 | ctggaagggtctctcttgttagc | 433033 | - | - | - | |
| 22641 | LOC339451 | NM_198317.1 | 925 | atgtcccacggctcatgaagtgt | 437657 | - | - | - | |
| 22642 | LOC339768 | NM_194312.1 | 3022 | aagaagctgagatgttcaacttt | 436695 | - | - | - | |
| 22643 | LOC339834 | NM_178173.2 | 1503 | cagcgtcccaggccataagattc | 430594 | - | - | - | |
| 22644 | LOC340061 | NM_198282.1 | 831 | gtgagccagcggctgtatattct | 437578 | - | - | - | |
| 22645 | LOC340069 | NM_182761.1 | 371 | atgtctccttgtcgtcctattca | 435135 | - | - | - | |
| 22646 | LOC341019 | NM_181807.1 | 486 | aagattgctccacacatcaaaca | 433646 | - | - | - | |

Figure 46

| | | | | | | | | | |
|---|---|---|---|---|---|---|---|---|---|
| 22647 | LOC341567 | NM_181788.1 | 508 | caggccatctccactcacaaagg | 433471 | - | - | - | - |
| 22648 | LOC344901 | NM_198184.1 | 71 | aagtcctctcagtagatgtaaca | 437181 | - | - | - | - |
| 22649 | LOC345667 | NM_197941.1 | 366 | gtggttcattggggattggttgg | 436907 | - | - | - | - |
| 22650 | LOC346673 | NM_182489.1 | 481 | ctgactggcagcgggatcattac | 433899 | - | - | - | - |
| 22651 | LOC348094 | NM_182703.2 | 24 | ctggttcggccacttacgaatcc | 434901 | - | signal_transduction | - | - |
| 22652 | LOC348645 | NM_198851.1 | 946 | gggcgtgacaatgcatttgattc | 439935 | - | - | - | - |
| 22653 | LOC348840 | NM_182631.1 | 533 | caggaagagacgtgttccattat | 434806 | - | - | - | - |
| 22654 | LOC349236 | NM_182635.1 | 705 | ctgcagacattcgctttgtctgg | 434822 | - | - | - | - |
| 22655 | LOC352909 | NM_178837.2 | 332 | cagcaaccccgagctagatgtgc | 432001 | - | - | - | - |
| 22656 | LOC360200 | NM_182973.1 | 1077 | cagcttgtacggccattcactca | 435517 | - | - | - | - |
| 22657 | LOC374654 | NM_198525.1 | 2236 | tgggccgttacatgtggataaac | 439133 | - | - | - | - |
| 22658 | LOC374946 | NM_198545.2 | 829 | ttgttcgactggaccgattatga | 439327 | - | - | - | - |
| 22659 | LOC375108 | NM_198996.1 | 327 | cagtaggctccatcccattatgg | 440469 | - | - | - | - |
| 22660 | LOC375323 | NM_198560.1 | 648 | cggctacggtctacaagatctgc | 439516 | - | - | - | - |
| 22661 | LOC375449 | NM_198828.1 | 634 | gaggagcttgaccacatattatc | 439888 | - | - | - | - |
| 22662 | LOC51035 | NM_015853.3 | 272 | atgtggacgagcctttagagact | 355256 | - | - | - | - |
| 22663 | LOC51054 | NM_015899.1 | 1691 | tggccatactggacgctttataa | 355383 | - | - | - | - |
| 22664 | LOC51057 | NM_015910.2 | 551 | gtggagcactccgtaagtgtaga | 355411 | - | - | - | - |
| 22665 | LOC51059 | NM_015912.2 | 801 | cggttcaccttgcattatggatg | 355459 | - | - | - | - |
| 22666 | LOC51063 | NM_015916.3 | 1069 | atgagtcccagctctttggatgg | 355474 | - | - | - | - |
| 22667 | LOC51122 | NM_016094.1 | 252 | gagagctcaaagctcatgatttc | 356262 | - | see also 6659 line | | |
| 22668 | LOC51136 | NM_016125.2 | 554 | gagcgtcaaacttgttatgcagc | 356334 | - | see also 6677 line | | |
| 22669 | LOC51159 | NM_016206.1 | 640 | aagtctccaagcatcttatttga | 356562 | - | - | - | - |
| 22670 | LOC51161 | NM_016210.2 | 932 | aggccatgcagggcaagactact | 356573 | - | - | - | - |

EP 1 752 536 A1

Figure 46

| 22671 | LOC51186 | NM_016303.1 | 530 | atgcgttggaaggttaatcgaaa | 356926 | - | see also 6800 line | | |
| 22672 | LOC51198 | NM_016349.1 | 125 | gagccgcgtgcctgcaacaatcc | 357205 | - | - | - | - |
| 22673 | LOC51204 | NM_016360.2 | 642 | ctgctcatcgaggcattatctaa | 357217 | - | - | - | - |
| 22674 | LOC51212 | NM_016380.1 | 779 | gtggctagacggacactatttgg | 357250 | - | - | - | - |
| 22675 | LOC51233 | NM_016449.1 | 334 | aggacgcaccctgttatgaatct | 357563 | - | - | - | - |
| 22676 | LOC51234 | NM_016454.2 | 502 | tggcattggctgtttacaagtgc | 357573 | - | see also 6865 line | | |
| 22677 | LOC51236 | NM_016458.2 | 729 | tggggacgctgcggaattgctgc | 357576 | - | - | - | - |
| 22678 | LOC51240 | NM_016467.3 | 540 | tggtgttcggatctttggaatta | 357613 | - | - | - | - |
| 22679 | LOC51244 | NM_016474.3 | 592 | cggcgctgtatgagaaaggattt | 357645 | - | see also 6870 line | | |
| 22680 | LOC51315 | NM_016618.1 | 909 | aggaggagcggtctaagcataag | 358452 | - | - | - | - |
| 22681 | LOC51320 | NM_016626.2 | 1373 | aggagcgacccacctagtacagg | 358616 | - | - | - | - |
| 22682 | LOC51333 | NM_016643.1 | 706 | ccgcctaagctcgcacttcattc | 358677 | - | - | - | - |
| 22683 | LOC51334 | NM_016644.1 | 580 | caggctaatgcaccgcttattaa | 358680 | - | - | - | - |
| 22684 | LOC51668 | NM_016126.1 | 379 | atgatggctccgctacttacttg | 356339 | - | - | - | - |
| 22685 | LOC51693 | NM_016209.1 | 449 | atggtgacgtcgatgatgataca | 356570 | - | - | - | - |
| 22686 | LOC51759 | NM_016482.2 | 490 | gaggactgtctttatgaacttcc | 357746 | - | - | - | - |
| 22687 | LOC54499 | NM_019026.1 | 434 | ctgccctaatgggaatgttcaat | 374513 | - | - | - | - |
| 22688 | LOC55565 | NM_017530.1 | 770 | gaggaccagcgatctaactgtgc | 358976 | - | - | - | - |
| 22689 | LOC55580 | NM_017571.1 | 996 | gcggttgccgcacatattcaaga | 359183 | - | - | - | - |
| 22690 | LOC55831 | NM_018447.1 | 709 | ttgggcttcggagcatttactct | 371571 | - | see also 7484 line | | |
| 22691 | LOC55908 | NM_018687.3 | 338 | aagttgaggctgttataccttag | 372544 | - | - | - | - |
| 22692 | LOC55924 | NM_019099.3 | 1005 | gagcactcaccctcaggatttga | 375229 | - | - | - | - |
| 22693 | LOC56181 | NM_019557.3 | 365 | ggggctgctcgaagtgtagtaag | 375307 | - | see also 7713 line | | |
| 22694 | LOC56270 | NM_019613.2 | 342 | atgttatttcgctgcaactattt | 375514 | - | see also 7723 line | | |
| 22695 | LOC56901 | NM_020142.3 | 357 | gggcagcgctgcgctttacttgc | 375675 | - | - | - | - |
| 22696 | LOC56965 | NM_020213.1 | 938 | tggattcctcgttcagatcatga | 376219 | - | - | - | - |
| 22697 | LOC57146 | NM_020422.3 | 585 | tggtttgggcttcgtatcactcg | 377402 | - | - | - | - |
| 22698 | LOC57149 | NM_020424.2 | 694 | gggacttcgaagccaagagaaac | 377414 | - | - | - | - |
| 22699 | LOC57228 | NM_020467.2 | 453 | aagaggaatatttcatctaatga | 377733 | - | - | - | - |
| 22700 | LOC57821 | NM_021179.1 | 1020 | aggagagccgctggcttatatcg | 380631 | - | - | - | - |
| 22701 | LOC63920 | NM_022090.2 | 2226 | atgatatccgtgtggctatttca | 382279 | - | - | - | - |
| 22702 | LOC65122 | NM_023014.1 | 1242 | cagcaattgcatgtctattgacg | 384985 | - | - | - | - |
| 22703 | LOC80298 | NM_025198.2 | 658 | ctgtagtccaaccgctgttaaca | 394322 | - | - | - | - |
| 22704 | LOC81558 | NM_030802.2 | 1114 | gagaaagtgcgtgtgtttgaaga | 395208 | - | - | - | - |
| 22705 | LOC85865 | NM_033107.1 | 666 | tagctgatcttccgggtttaata | 403272 | - | - | - | - |
| 22706 | LOC88523 | NM_033111.2 | 1543 | taggcataccatagcattagaca | 403306 | - | - | - | - |
| 22707 | LOC89894 | NM_138341.1 | 815 | cagtcagagccacaagtgtatct | 407535 | - | - | - | - |

Figure 46

EP 1 752 536 A1

| 22708 | LOC90050 | NM_138344.2 | 1337 | gtgcccccacccatcttcaatgt | 407553 | - | - | - | - |
|-------|----------|-------------|------|-------------------------|--------|---|---|---|---|
| 22709 | LOC90167 | NM_194277.1 | 1373 | gaggcggatcccacattgctaca | 436206 | - | - | - | - |
| 22710 | LOC90268 | NM_138348.3 | 729 | aggacgatcggcactataacatc | 407559 | - | - | - | - |
| 22711 | LOC90313 | NM_138349.2 | 871 | cagggggctgtgtctgttcaagt | 407562 | - | - | - | - |
| 22712 | LOC90355 | NM_033211.2 | 270 | atggaagtcgatattaatggaga | 403578 | - | see also 9380 line | | |
| 22713 | LOC90378 | NM_138352.1 | 1432 | gagcaggcgacagctttccaaga | 407566 | - | - | - | - |
| 22714 | LOC90522 | NM_033557.1 | 859 | ctgcgtagccatctttgtgttca | 404137 | - | - | - | - |
| 22715 | LOC90529 | NM_178122.2 | 813 | gtgcatcattcgtgtccaatacc | 430462 | - | - | - | - |
| 22716 | LOC90580 | NM_138358.2 | 705 | gcggctcttcgatgagaagtaca | 407604 | - | - | - | - |
| 22717 | LOC90624 | NM_181705.1 | 330 | atgcaccaactcagaagcaatga | 432842 | - | - | - | - |
| 22718 | LOC90736 | NM_138362.1 | 268 | ttgcatgagggattgtctaaacc | 407613 | - | - | - | - |
| 22719 | LOC90768 | NM_178838.2 | 839 | ctgtactcggccgttaggaaatc | 432009 | - | - | - | - |
| 22720 | LOC90799 | NM_138363.1 | 821 | gagcctatccgagcagctattcc | 407637 | - | - | - | - |
| 22721 | LOC90806 | NM_144567.2 | 1118 | ctgacgcaattggcaatgctact | 409723 | - | - | - | - |
| 22722 | LOC90850 | NM_178167.2 | 327 | tggaaaggtgtacgcattgtaca | 430550 | - | - | - | - |
| 22723 | LOC91056 | NM_138368.2 | 1236 | cagagtgctaccctcaactttct | 407754 | - | - | - | - |
| 22724 | LOC91526 | NM_153697.1 | 763 | tggttaacgagttgattgactac | 423595 | - | see also 10007 line | | |
| 22725 | LOC91689 | NM_033318.3 | 85 | gagcggctcgctggctagtattg | 403698 | - | - | - | - |
| 22726 | LOC91752 | NM_194250.1 | 3562 | gagtggctgcgttataattcagg | 436150 | - | see also 10304 line | | |
| 22727 | LOC91937 | NM_138379.1 | 411 | ctggttcaacgatgtaaagataa | 407813 | - | - | - | - |
| 22728 | LOC91947 | NM_183376.1 | 621 | cgggaactccaggttgttagtca | 435728 | - | - | - | - |
| 22729 | LOC91966 | NM_178124.3 | 819 | ttgcaatgccccgaagacttaac | 430468 | - | - | - | - |
| 22730 | LOC92170 | NM_138384.1 | 278 | atggacttggcggatcttacaga | 407822 | - | - | - | - |
| 22731 | LOC92345 | NM_138386.1 | 1292 | aggatatcgtaacagagaattca | 407857 | - | - | - | - |
| 22732 | LOC92346 | NM_139240.2 | 707 | ttgcccagaaaggaaccaatagg | 409463 | - | - | - | - |
| 22733 | LOC92689 | NM_138389.1 | 1533 | gaggtaacagcgcgctgtattga | 407908 | - | see also 9605 line | | |
| 22734 | LOC92691 | NM_138390.2 | 1004 | ttgaatccgacaatatctcaagc | 407933 | - | - | - | - |
| 22735 | LOC92715 | NM_138778.1 | 834 | cagggtacccggcaaatttctct | 408747 | - | - | - | - |
| 22736 | LOC92979 | NM_138396.3 | 1184 | cagtggaaggtcctaaattatga | 407948 | - | - | - | - |
| 22737 | LOC93109 | NM_138399.2 | 833 | cggcactggacctcgctattatt | 407962 | - | - | - | - |
| 22738 | LOC93343 | NM_138401.1 | 305 | ccgtctgcgaccccatggattcc | 407965 | - | - | - | - |
| 22739 | LOC93380 | NM_173470.1 | 468 | ctgttacggtatagttcatattg | 424896 | - | see also 10066 line | | |
| 22740 | LOC93426 | NM_130784.1 | 419 | caggagtgcaaggagagaatttc | 406929 | - | - | - | - |
| 22741 | LOH11CR2A | NM_014622.3 | 1264 | cagacacgtttagtgtaattaaa | 341401 | - | - | - | - |
| 22742 | LOH12CR1 | NM_058169.2 | 472 | cagcaacgatcccgatgtcatca | 406191 | - | - | - | - |

Figure 46

| | | | | | | | |
|---|---|---|---|---|---|---|---|
| 22743 | LOH3CR2A | NM_013343.1 | 711 | tggtaccattagaaccttaag | 338782 | - | |
| 22744 | LOST1 | NM_172367.1 | 350 | ccggtgcagtccgagtttccttca | 424476 | - | |
| 22745 | LPPR2 | NM_022737.1 | 873 | caggtccgaccgctttgtcact | 383787 | - | |
| 22746 | LR8 | NM_014020.2 | 963 | gggagtaggtcttcgaaacttgt | 339213 | - | |
| 22747 | LRIG1 | NM_015541.1 | 1974 | gagtttcgtgtgcgatgacttcc | 353812 | - | |
| 22748 | LRP11 | NM_032832.3 | 1791 | aggaatcggactacctcataaat | 401815 | - | molecular_function unknown |
| 22749 | LRP15 | NM_052953.2 | 947 | ttgtccgacaatcggattcaaag | 405519 | - | |
| 22750 | LRP16 | NM_014067.2 | 339 | aggaggcgaaatcctttctgaag | 339513 | - | |
| 22751 | Lrp2bp | NM_181726.1 | 428 | aggagaagctccactacacaagg | 433036 | - | receptor_acitivity |
| 22752 | LRRC1 | NM_025168.1 | 828 | aagcttggacttagtgataatga | 368510 | - | |
| 22753 | LRRC16 | NM_017640.2 | 553 | gcggtcatgcgtgatgctaaga | 359884 | - | see also 7063 line |
| 22754 | LRRC17 | NM_005824.1 | 282 | atgcgtgtggttaccattgtaat | 334274 | - | |
| 22755 | LRRC19 | NM_022901.1 | 966 | atgcccaatatgtacaatattc | 384748 | - | |
| 22756 | LRRC2 | NM_024512.2 | 862 | gtgtcctgccgatgtcgaatttg | 386548 | - | |
| 22757 | LRRC5 | NM_018103.3 | 846 | tggcccttccgtggtattctaag | 366728 | - | |
| 22758 | LRRC8 | NM_019594.1 | 319 | acggcgctccctcaagaagtact | 375432 | - | |
| 22759 | LRRN1 | NM_020873.3 | 665 | aggtcgggctgcaaacctaacc | 379807 | - | see also 8011 line |
| 22760 | LRRN3 | NM_018334.3 | 815 | gttgattgtccacggttatgtac | 370441 | - | see also 7407 line |
| 22761 | LRRN4 | NM_002319.2 | 1180 | gagcagcgaccaccgaattaag | 330868 | - | |
| 22762 | LSR7 | NM_018559.2 | 562 | aggaccagttaactataatgtaa | 372221 | - | |
| 22763 | LXN | NM_020169.2 | 612 | atgacgctcgttctacattagc | 375742 | - | |
| 22764 | LY6G6C | NM_025261.1 | 294 | ccgtaagctgggtctgacatata | 394626 | - | |
| 22765 | LY6G6D | NM_021246.1 | 582 | gagccgaagaccaagaatcatcc | 380898 | - | |
| 22766 | LYAR | NM_017816.1 | 1008 | aggcactcggaagttgaaacaga | 362704 | - | |
| 22767 | LYRIC | NM_178812.2 | 1014 | cagttaccaccgagcaacttaca | 431346 | - | see also 10201 line |
| 22768 | LZIC | NM_032368.3 | 212 | tggatagactcatgcaacaatta | 400005 | - | |
| 22769 | LZK1 | NM_024835.1 | 2075 | ctgccggttaaatgtcacaaga | 391345 | - | see also 8772 line |
| 22770 | LZTFL1 | NM_020347.1 | 927 | cagcagccttatcgaagcatgaaa | 376796 | - | |
| 22771 | LZTR1 | NM_006767.1 | 1789 | ctgcctgaacttcgtggtaaagg | 336313 | - | see also 4772 line |
| 22772 | M-RIP | NM_015134.1 | 2997 | acggtgtccggatatgatataat | 348565 | - | |
| 22773 | M17S2 | NM_005899.2 | 1494 | cgggtctggtgcagtatcatagt | 334467 | - | see also 4109 line |
| 22774 | MAB21L1 | NM_005584.2 | 926 | tggctcaagcggtagacaaatgt | 334096 | - | |
| 22775 | MAD2L1BP | NM_014628.1 | 771 | ttgggaagactacatttgttcc | 341437 | - | |
| 22776 | MAFI | NM_032272.2 | 513 | gagacgacaaacacattgtcaag | 399301 | - | see also 9132 line |

Figure 46

| | | | | | | | | | |
|---|---|---|---|---|---|---|---|---|---|
| 22777 | MAGEA10 | NM_021048.3 | 989 | tggcattctcatacttatcctaa | 380494 | - | - | - | - |
| 22778 | MAGEA8 | NM_005364.3 | 925 | gggagcacagtgtctattggaag | 333664 | - | - | molecular_function unknown | - |
| 22779 | MAGEB1 | NM_002363.3 | 1013 | tggagaggagcacttaatctatg | 330879 | - | - | - | melanoma |
| 22780 | MAGEB2 | NM_002364.3 | 590 | tggccttgagctgaataaagtca | 330883 | - | - | - | melanoma |
| 22781 | MAGEB3 | NM_002365.2 | 545 | gtggtatttggtgttgatttaaa | 330893 | - | - | - | - |
| 22782 | MAGEB4 | NM_002367.2 | 754 | ctgaatatgctggggatctatga | 330902 | - | - | - | - |
| 22783 | MAGEB6 | NM_173523.2 | 746 | gtgcacgttggcgcaattcctgc | 425438 | - | - | - | - |
| 22784 | MAGEC1 | NM_005462.2 | 3651 | atgattcgactgccacagaaagt | 333855 | - | - | - | melanoma |
| 22785 | MAGEC3 | NM_138702.1 | 1526 | ttggcattgccctgactgatatg | 408621 | - | - | - | - |
| 22786 | MAGED1 | NM_006986.2 | 779 | gagaccgacccaggtatctctga | 336682 | - | - | - | - |
| 22787 | MAGED2 | NM_014599.4 | 1579 | aaggctagggccgagattagagc | 340987 | - | see also 5768 line | | |
| 22788 | MAGED4 | NM_030801.2 | 192 | cagcgtgcaatcggaaagctaca | 395186 | - | - | - | - |
| 22789 | MAGEE1 | NM_016249.2 | 566 | ctggtgtgataccaaatcttacc | 356618 | - | - | - | - |
| 22790 | MAGEF1 | NM_022149.3 | 803 | gggtatccgaagaggcttattat | 382542 | - | - | - | - |
| 22791 | MAGEH1 | NM_014061.3 | 732 | cagtccggtggagtatgagttct | 339498 | - | - | - | - |
| 22792 | MAIL | NM_031419.1 | 1815 | gagtctggttgataccattaagt | 396887 | - | - | - | - |
| 22793 | MAMDC1 | NM_182830.2 | 1675 | aggatcgaagtgtcactatgagt | 435334 | - | - | - | - |
| 22794 | MAML1 | NM_014757.2 | 570 | ccggccacgcatcttcatgatac | 343125 | - | - | - | - |
| 22795 | MAML3 | NM_018717.2 | 1311 | ccgactagcaaacgaattcgaaa | 372845 | - | transcription_regulator | - | - |
| 22796 | MAN1A1 | NM_005907.2 | 1793 | aagtctagcagcggactaactta | 334519 | - | see also 4122 line | | |
| 22797 | MANEA | NM_024641.1 | 857 | cagacacgccccagcttaattc | 388455 | - | - | - | - |
| 22798 | MAP1LC3A | NM_032514.2 | 465 | acgaggacggcttcctctatatg | 400512 | - | - | - | - |
| 22799 | MAP4K4 | NM_004834.2 | 2550 | gtgacaccccggagattcgtaaa | 333075 | - | see also 3349 line | | |
| 22800 | MBD3L1 | NM_145208.1 | 132 | aggccagtaacgagaattacacc | 413769 | - | transcription_regulator | - | - |
| 22801 | MBD6 | NM_052897.1 | 2844 | aggggacttaggggcattaatg | 404763 | - | - | - | - |
| 22802 | MBTD1 | NM_017643.1 | 615 | aagattaagactagtgtatgaag | 359951 | - | see also 7066 line | | |
| 22803 | MCC2 | NM_031941.2 | 1411 | aggagcgccgttctctaatgaag | 397428 | - | - | - | - |
| 22804 | MCLC | NM_015127.2 | 1139 | gtgatgccgatttccattatagg | 348523 | - | see also 6257 line | | |
| 22805 | MCM10 | NM_018518.3 | 2620 | gggaacgggacggaatgctaaag | 372106 | - | - | - | - |
| 22806 | MCM3APAS | NM_018118.1 | 709 | aagtaacagtacccatacacagg | 366856 | - | - | - | - |
| 22807 | MDS006 | NM_020233.3 | 90 | tggcgtcatcgcagatgttcaat | 376343 | - | - | - | - |
| 22808 | MDS009 | NM_020234.3 | 990 | tggtagactaccatactgatata | 376395 | - | - | - | - |
| 22809 | MDS010 | NM_020231.3 | 200 | gagaattacgaaccatgttcaag | 376308 | - | see also 7808 line | | |

EP 1 752 536 A1

Figure 46

| | | | | | | | | | |
|---|---|---|---|---|---|---|---|---|---|
| 22810 | MDS025 | NM_021825.3 | 616 | cagcgttaactgctttatcaaat | 381107 | - | see also 8178 line | | |
| 22811 | MDS027 | NM_018462.3 | 155 | aagacttgcaacactaaacgaga | 371874 | - | see also 7487 line | | |
| 22812 | MDS029 | NM_018464.1 | 148 | tggatcgcagcagttaccattgc | 371881 | - | - | - | - |
| 22813 | MDS031 | NM_018466.2 | 130 | tggttacaaccgacttatcctgc | 371897 | - | - | - | - |
| 22814 | MDS032 | NM_018467.1 | 188 | ccggcctctgaggtgatcaatga | 371917 | - | - | - | - |
| 22815 | MDS2 | NM_148895.1 | 677 | atggtgtctgcttaatgttaatc | 415164 | - | - | - | - |
| 22816 | MECT1 | NM_015321.1 | 665 | ctgtgaggtccccggaatcaaca | 351499 | - | - | - | - |
| 22817 | MEP50 | NM_024102.2 | 306 | tggggagagaggtattctagtgg | 386031 | - | - | - | - |
| 22818 | MESDC1 | NM_022566.1 | 1691 | aggactttaccgccagtgaattc | 383579 | - | - | molecular_function unknown | - |
| 22819 | MFN2 | NM_014874.1 | 1560 | aagactataagctgcgaattaag | 345081 | - | see also 6074 line | | |
| 22820 | MGC10084 | NM_153702.1 | 186 | cagcgaatatttgatacctatgt | 423679 | - | see also 10010 line | | |
| 22821 | MGC10120 | NM_173809.1 | 94 | cggccaccagtgaagactataag | 428196 | - | - | - | - |
| 22822 | MGC10198 | NM_152682.1 | 477 | ttggaaccgctatgacctataca | 419736 | - | - | - | - |
| 22823 | MGC10200 | NM_145060.1 | 913 | gaggacttactcgttatgttata | 413360 | - | - | - | - |
| 22824 | MGC10233 | NM_152715.1 | 1254 | aagcaacatgcttctctactatt | 420039 | - | - | - | - |
| 22825 | MGC10540 | NM_032353.2 | 252 | tggagtcgatccagattgtatta | 399918 | - | - | - | - |
| 22826 | MGC10744 | NM_032354.2 | 445 | gggagtgcactacgtattggtac | 399927 | - | - | - | - |
| 22827 | MGC10765 | NM_024345.1 | 439 | cagccccgatggacgaatgattt | 386352 | - | see also 8613 line | | |
| 22828 | MGC10870 | NM_032301.1 | 1306 | ttgcacccgaaggcatgacaatg | 399557 | - | - | - | - |
| 22829 | MGC10911 | NM_032302.1 | 238 | aagacacgccgttggtgatatcg | 399560 | - | - | - | - |
| 22830 | MGC10955 | NM_032676.2 | 265 | ccgtggctggagcccaaatcaca | 401212 | - | - | - | - |
| 22831 | MGC10992 | NM_033212.2 | 515 | ctgccaattggcgcgagaaatgg | 403594 | - | - | - | - |
| 22832 | MGC10993 | NM_030577.1 | 960 | cagacacttgttccgaatcaaac | 394697 | - | - | - | - |
| 22833 | MGC11061 | NM_032312.2 | 608 | atggattataaccatttggatat | 399661 | - | see also 9153 line | | |

886

Figure 46

| | | | | | | | |
|---|---|---|---|---|---|---|---|
| 22834 | MGC11082 | NM_032691.1 | 843 | cagctcagaaacacctattgttc | 401228 | - | |
| 22835 | MGC11115 | NM_032310.2 | 420 | ccgccagctctgcgtaagttcc | 399645 | - | |
| 22836 | MGC11242 | NM_024320.2 | 251 | gaggccccaacagcgactttaac | 386252 | - | |
| 22837 | MGC11266 | NM_024322.1 | 564 | cagaaccactccggatacatca | 386271 | - | |
| 22838 | MGC1203 | NM_024296.2 | 627 | tggaagacctcagtaattctatg | 386205 | - | |
| 22839 | MGC12197 | NM_016625.2 | 113 | tgggacgtcggtcatcagatact | 358562 | - | see also 6961 line |
| 22840 | MGC12909 | NM_145055.1 | 473 | aggcggtccgatctgaaagtga | 413317 | - | see also 9769 line |
| 22841 | MGC12935 | NM_032694.1 | 170 | gggtgacgcgggtcttcttgtct | 401232 | - | |
| 22842 | MGC12965 | NM_198519.1 | 1232 | cagtgaacacatgccacttaatg | 438955 | - | |
| 22843 | MGC12966 | NM_032706.1 | 386 | aggttcatattgagatccattga | 401246 | - | |
| 22844 | MGC12972 | NM_032683.1 | 220 | cggcgctccgcgagcatgtttgc | 401214 | - | |
| 22845 | MGC12981 | NM_032357.2 | 299 | ggggaaacgaacgggtgttgaacg | 399931 | - | |
| 22846 | MGC12992 | NM_032342.1 | 443 | ctggcctgtcaccgacttctaca | 399824 | - | |
| 22847 | MGC13010 | NM_032687.2 | 701 | ctgtcttcacggccaacacttat | 401221 | - | |
| 22848 | MGC13016 | NM_032343.1 | 535 | atgctgagatgtataaactgtct | 399846 | - | |
| 22849 | MGC13024 | NM_152288.1 | 697 | ctggttggttgggtcaagtttgt | 415376 | - | |
| 22850 | MGC13034 | NM_153217.1 | 681 | caggcccaatcctgatgttgaac | 422222 | - | |
| 22851 | MGC13040 | NM_032930.1 | 838 | ctggtgagtgttcgaaagaatcc | 402540 | - | |
| 22852 | MGC13045 | NM_032344.1 | 807 | ccgagccagtgccgagttctatg | 399849 | - | |
| 22853 | MGC13053 | NM_032710.1 | 471 | cagaacaagattccgttgttaca | 401247 | - | |

Figure 46

| | | | | | | | | | |
|---|---|---|---|---|---|---|---|---|---|
| 22854 | MGC13057 | NM_032321.1 | 566 | atgggcatgcaataacatcaagt | 399711 | - | - | - | - |
| 22855 | MGC13102 | NM_032323.1 | 772 | tgggagccgcactcattctcttc | 399729 | - | - | - | - |
| 22856 | MGC13125 | NM_032725.2 | 670 | aggagagtccgtcatgattcacc | 401267 | - | - | - | - |
| 22857 | MGC13159 | NM_032927.2 | 860 | cagccttgatacccattaccact | 402521 | - | - | - | - |
| 22858 | MGC13183 | NM_032358.2 | 1452 | tagacgggcacataagatacaag | 399964 | - | - | - | - |
| 22859 | MGC13204 | NM_031465.1 | 572 | gagcttaccttatgtgttcattc | 397092 | - | - | - | - |
| 22860 | MGC13251 | NM_032714.1 | 568 | tggctgccctgtgcatctactct | 401248 | - | - | - | - |
| 22861 | MGC14126 | NM_032898.2 | 458 | gcgcattatgccagttcgaaact | 402461 | - | - | - | - |
| 22862 | MGC14141 | NM_032928.2 | 164 | atgttcattcagaggaagtttcc | 402525 | - | - | - | - |
| 22863 | MGC14156 | NM_032906.2 | 316 | gggaatagcttatccaatcattg | 402499 | - | see also 9344 line | | |
| 22864 | MGC14161 | NM_032892.1 | 1471 | gggagccaccggaaatacacaga | 402449 | - | see also 9338 line | | |
| 22865 | MGC14288 | NM_032901.2 | 285 | gtgtccgagtctaccactatttc | 402492 | - | - | - | - |
| 22866 | MGC14289 | NM_080660.2 | 637 | cggtcccatcagcttatccatgc | 406577 | - | - | - | - |
| 22867 | MGC14407 | NM_032908.1 | 434 | atgcacacgatgttatgagaaca | 402502 | - | - | - | - |
| 22868 | MGC14595 | NM_032334.1 | 788 | aagaattcggaacagatctaacc | 399786 | - | - | - | - |
| 22869 | MGC14697 | NM_032747.1 | 371 | aagcattgcattgattgtcttat | 401318 | - | - | - | - |
| 22870 | MGC14799 | NM_032336.1 | 471 | gtgtcgcctcatgaagatagaga | 399792 | - | see also 9166 line | | |
| 22871 | MGC14801 | NM_032705.2 | 231 | cagcaccgatcccgacatagatc | 401240 | - | - | - | - |
| 22872 | MGC14816 | NM_144620.1 | 330 | aagaacgagtaagcttaaccaag | 410378 | - | - | - | - |
| 22873 | MGC14817 | NM_032338.2 | 326 | tggacagtacccaatatggatga | 399809 | - | - | - | - |

EP 1 752 536 A1

Figure 46

| | | | | | | |
|---|---|---|---|---|---|---|
| 22874 | MGC15396 | NM_052855.2 | 1181 | aagttaccaatactctgttaag | 404451 | - |
| 22875 | MGC15397 | NM_080652.2 | 625 | tcggttgatcccatataattc | 406523 | - |
| 22876 | MGC15407 | NM_080667.2 | 878 | aaggaccaatagcaaacttatca | 406610 | - |
| 22877 | MGC15419 | NM_024735.2 | 565 | acgtcgatgaccctatgagattc | 389746 | - |
| 22878 | MGC15606 | NM_145037.1 | 2083 | cagcggactcggtacaactttcagc | 413103 | - |
| 22879 | MGC15668 | NM_032756.1 | 1021 | ggggctgtatacgcctaacatg | 401326 | - |
| 22880 | MGC15677 | NM_033878.2 | 72 | gtgcaaggagttggtgttgatgg | 402390 | - |
| 22881 | MGC15716 | NM_033370.1 | 350 | ctggcctgaccagcatagtctcc | 400015 | - |
| 22882 | MGC15730 | NM_033880.2 | 1185 | acggtcgtgagccgtgagttcc | 402410 | - see also 9331 line |
| 22883 | MGC15827 | NM_032882.2 | 416 | gcgtttcgagagaggagataatgc | 402414 | - |
| 22884 | MGC15882 | NM_032884.1 | 959 | cagccgagacttcgaaacaaagt | 402421 | - |
| 22885 | MGC15912 | NM_032886.1 | 414 | tggggaggtgtacgtgttagtca | 402441 | - |
| 22886 | MGC15937 | NM_080661.2 | 182 | gtggctgaggataatagagtttc | 406583 | - |
| 22887 | MGC16028 | NM_052873.1 | 246 | ctggatctgaacgcatgctatca | 404566 | - |
| 22888 | MGC16044 | NM_138371.1 | 1573 | aggtttcttcgtcgaagacaatt | 407777 | - |
| 22889 | MGC16075 | NM_032761.2 | 468 | tgggcaccattggacctatagc | 401337 | - |
| 22890 | MGC16121 | NM_032762.2 | 443 | gcgttgcctcccggaatccaagc | 401338 | - |
| 22891 | MGC16202 | NM_032373.2 | 294 | ctgtaaagggtatctgatcaagc | 400022 | - see also 9175 line |
| 22892 | MGC16309 | NM_033413.2 | 414 | gggagggattactactatcagg | 403819 | - |
| 22893 | MGC16353 | NM_138774.2 | 175 | caggccgaaacagcacttcatca | 408729 | - |

Figure 46

| 22894 | MGC16372 | NM_145038.1 | 908 | ctgggattgcgaagaatacaaca | 413122 | - | - | - | - |
|---|---|---|---|---|---|---|---|---|---|
| 22895 | MGC16491 | NM_052943.2 | 785 | tcggtgagacgccagtttgaatt | 405461 | - | - | - | - |
| 22896 | MGC16733 | NM_033547.2 | 576 | atgttgcagctccatgaaagagg | 404130 | - | - | - | - |
| 22897 | MGC16824 | NM_020314.3 | 1492 | gagtgaccgacttcagattctca | 376457 | - | - | - | - |
| 22898 | MGC17330 | NM_052880.2 | 766 | agggagatgcagcgaatcactct | 404589 | - | - | - | - |
| 22899 | MGC17839 | NM_174926.1 | 428 | tggctctcgctctatatttcttt | 428701 | - | - | - | - |
| 22900 | MGC17943 | NM_152261.1 | 323 | gtgtgactgggggtatcttcagt | 415205 | - | - | - | - |
| 22901 | MGC18079 | NM_144675.1 | 479 | cgggaagagccgaccttcataga | 411269 | - | - | - | - |
| 22902 | MGC18216 | NM_152452.1 | 2342 | atgatgattacagcatacacagt | 416940 | - | - | - | - |
| 22903 | MGC19595 | NM_033415.2 | 846 | gcgtcacgcttgcctgaaacatg | 403822 | - | - | - | - |
| 22904 | MGC19780 | NM_144988.1 | 308 | tagagaccctagtaacatgtata | 412450 | - | - | - | - |
| 22905 | MGC20255 | NM_052848.1 | 1086 | gaggaggagaggtactttgatga | 404409 | - | - | - | - |
| 22906 | MGC20262 | NM_152421.2 | 492 | gggagctggtactgtttgacaag | 416541 | - | - | - | - |
| 22907 | MGC20398 | NM_052857.1 | 595 | ctgccaaacgatttctttagtac | 404461 | - | see also 9437 line | | |
| 22908 | MGC20460 | NM_053043.1 | 3301 | gggctccggcataatacaacttc | 405829 | - | - | - | - |
| 22909 | MGC20470 | NM_145053.2 | 1451 | ctgcaacccgagtcatctataat | 413246 | - | - | - | - |
| 22910 | MGC20481 | NM_052849.2 | 695 | gaggtggctccagccagataaag | 404416 | - | see also 9435 line | | |
| 22911 | MGC20486 | NM_052844.2 | 618 | gggactggagcacgcttaagtcc | 404405 | - | - | - | - |
| 22912 | MGC20579 | NM_182614.2 | 281 | tggtggcagcgatcgtgtttatc | 434687 | - | - | - | - |
| 22913 | MGC20700 | NM_174983.2 | 969 | aggtgggcatactgtacatgacc | 428890 | - | - | - | - |

Figure 46

| | | | | | | | |
|---|---|---|---|---|---|---|---|
| 22914 | MGC20781 | NM_052935.2 | 325 | gagaccggttacaggtgatttct | 405394 | - | |
| 22915 | MGC20806 | NM_144999.2 | 1115 | atgagaggcactccatcatcaac | 412543 | - | |
| 22916 | MGC21416 | NM_173834.2 | 314 | ttgtgtgacactcgcattaatgc | 428462 | - | |
| 22917 | MGC21518 | NM_145274.1 | 819 | ctgaggtgttagagtggaataaa | 414087 | - | |
| 22918 | MGC21636 | NM_145032.2 | 1933 | gcgctgccctaatttaaactact | 412954 | - | |
| 22919 | MGC21644 | NM_138492.3 | 749 | agggagcccagaaaagggaattaga | 408432 | - | |
| 22920 | MGC21654 | NM_145647.1 | 153 | cggccacgcgggacggaattata | 414481 | - | see also 9790 line |
| 22921 | MGC21658 | NM_198451.1 | 1167 | tgggaggagactcgtgtcttagc | 437869 | - | |
| 22922 | MGC21675 | NM_052861.1 | 650 | tcgcgtgggacaacctagtattg | 404478 | - | |
| 22923 | MGC21830 | NM_182563.2 | 777 | gagacagcggctgatttatctct | 434463 | - | |
| 22924 | MGC21854 | NM_052862.2 | 806 | gtgcggacgaggggctcaataaa | 404487 | - | |
| 22925 | MGC2217 | NM_024300.2 | 205 | ctgagagatctgacataaaatcc | 386210 | - | |
| 22926 | MGC22773 | NM_145258.1 | 399 | cagataaagagtctaccaaatac | 413951 | - | |
| 22927 | MGC22776 | NM_144962.1 | 335 | ccgtggacggcgcaacctatatc | 411945 | - | |
| 22928 | MGC22805 | NM_144590.1 | 498 | gaggctcttgtacgaatgctact | 410077 | - | |
| 22929 | MGC22960 | NM_199044.2 | 327 | atggtgcactggtcaataacttt | 440595 | - | |
| 22930 | MGC23244 | NM_144615.1 | 201 | gggaacggctccgtgttaagtgg | 410338 | - | |
| 22931 | MGC23401 | NM_144982.3 | 5256 | cagaatcgaggcgataatcttct | 412402 | - | |
| 22932 | MGC23918 | NM_144716.1 | 56 | gaggcggttgcgcgcgaaaggaacg | 411678 | - | |
| 22933 | MGC23937 | NM_145052.1 | 722 | ctgctgtcgatccatacgaattg | 413209 | - | |

Figure 46

| | | | | | | | | | |
|---|---|---|---|---|---|---|---|---|---|
| 22934 | MGC24039 | NM_144973.2 | 2266 | cagaccaactgtaaattcgtaga | 412123 | - | see also 9754 line | | |
| 22935 | MGC24103 | NM_152576.1 | 490 | gtgtttgatcaggttagtgaatc | 418167 | - | - | - | - |
| 22936 | MGC24133 | NM_174896.2 | 371 | cagatcctccagccaagctttca | 428527 | - | - | - | - |
| 22937 | MGC24180 | NM_152352.1 | 622 | aggagtccttcgccataagcaag | 415772 | - | - | - | - |
| 22938 | MGC24665 | NM_152308.1 | 300 | ctgtctagtcccaggaaagtatg | 415446 | - | - | - | - |
| 22939 | MGC2477 | NM_024099.1 | 2810 | caggctcccgtgtttgtagaagt | 386024 | - | - | - | - |
| 22940 | MGC2491 | NM_024040.1 | 156 | tggcttggatgaggtcatcttct | 385601 | - | - | - | - |
| 22941 | MGC24975 | NM_153359.1 | 619 | tgggccagtcagccctgtataag | 423046 | - | - | - | - |
| 22942 | MGC24976 | NM_144598.2 | 752 | aggtcgatctcgagaactacagt | 410189 | - | see also 9711 line | | |
| 22943 | MGC25062 | NM_019020.2 | 1547 | cagagcgttctggcgtaatgtgc | 374497 | - | - | - | - |
| 22944 | MGC2508 | NM_024327.1 | 677 | agggctgcatcaacactacattc | 386296 | - | - | - | - |
| 22945 | MGC2562 | NM_032374.2 | 170 | ttggataggaccccccagataaat | 400048 | - | see also 9176 line | | |
| 22946 | MGC2574 | NM_024098.1 | 417 | gtgaggaggcaccaaagtgttct | 386014 | - | - | - | - |
| 22947 | MGC2601 | NM_024042.2 | 666 | atgcaccagcgacttcgtaattc | 385619 | - | - | - | - |
| 22948 | MGC2603 | NM_024037.1 | 944 | gggacttagggccgtttcctaac | 385571 | - | - | - | - |
| 22949 | MGC2641 | NM_032631.2 | 1663 | aagaagattcgccgttacaaagc | 401152 | - | - | - | - |
| 22950 | MGC2650 | NM_024108.1 | 14 | cggatactgtgttgtttgagttt | 386049 | - | - | - | - |
| 22951 | MGC2654 | NM_024109.1 | 741 | aggcagcccgcacgatatcatca | 386057 | - | - | - | - |
| 22952 | MGC2655 | NM_024339.2 | 903 | ttggatgtttggcaactgattcc | 386326 | - | kinase_related | - | - |
| 22953 | MGC2656 | NM_024509.1 | 1934 | gtgcgtgctcgccgtgtatgagg | 386475 | - | - | - | - |
| 22954 | MGC26568 | NM_152402.1 | 1189 | aagaaacggtcgagatcttctaa | 416311 | - | - | - | - |
| 22955 | MGC26598 | NM_152638.2 | 562 | cgggtgctccagcgtacaataga | 419411 | - | - | - | - |
| 22956 | MGC26647 | NM_152706.2 | 852 | atggacccggcaacatttataaa | 419938 | - | - | - | - |
| 22957 | MGC26690 | NM_152450.1 | 332 | gagcacgcctttcggattaaaga | 416892 | - | - | - | - |
| 22958 | MGC26710 | NM_152510.1 | 584 | gagccacttcctaataatgttgt | 417333 | - | - | - | - |

EP 1 752 536 A1

Figure 46

| 22959 | MGC26733 | NM_144992.2 | 1219 | tggcttggtcgccaagaaactca | 412484 | - | - | - | - |
|---|---|---|---|---|---|---|---|---|---|
| 22960 | MGC26744 | NM_144645.1 | 335 | cggggcagttgttataatgtaca | 410711 | - | - | - | - |
| 22961 | MGC26778 | NM_145010.2 | 799 | cagtccctctcggtctttataga | 412610 | - | - | - | - |
| 22962 | MGC26816 | NM_152613.1 | 134 | cagcgatcagaaggctcaaatgt | 418829 | - | - | - | - |
| 22963 | MGC26818 | NM_152371.1 | 471 | ttggcatccaggggaacttgtct | 415908 | - | - | - | - |
| 22964 | MGC26847 | NM_145006.2 | 505 | acggtgcaggccgcataccttgg | 412585 | - | - | - | - |
| 22965 | MGC26885 | NM_152339.2 | 856 | gggagcagagtgccaaactgtgg | 415718 | - | - | - | - |
| 22966 | MGC26963 | NM_152621.3 | 1435 | tagcacacgaacactacactatc | 419027 | - | kinase_related | - | - |
| 22967 | MGC26988 | NM_130899.1 | 1228 | gcggcgtttgcaggcagtattac | 407122 | - | - | - | - |
| 22968 | MGC26989 | NM_152763.2 | 338 | aagataggcaatgattacagttt | 420830 | - | - | - | - |
| 22969 | MGC26999 | NM_152630.2 | 430 | atggttccgtagcaagttacata | 419177 | - | - | - | - |
| 22970 | MGC27034 | NM_152292.2 | 1209 | aaggtggatcggacagtgattcc | 415431 | - | - | - | - |
| 22971 | MGC27044 | NM_144977.1 | 835 | tcgtgattatctcgagcaaatta | 412185 | - | see also 9761 line | | |
| 22972 | MGC2705 | NM_032701.2 | 285 | tcggtttccgcacccataagatg | 401233 | - | - | - | - |
| 22973 | MGC27085 | NM_153353.2 | 888 | atgcaggcgcaaactatctcagt | 422967 | - | - | - | - |
| 22974 | MGC2714 | NM_032299.2 | 650 | ttgcgctcacagttgaatgatat | 399521 | - | see also 9150 line | | |
| 22975 | MGC27169 | NM_176782.1 | 171 | gggtgtttgccggcattacctgt | 429592 | - | - | - | - |
| 22976 | MGC2731 | NM_024068.2 | 212 | aggccgagtgaccaagacaaagg | 385732 | - | - | - | - |
| 22977 | MGC27434 | NM_145050.1 | 471 | aggttcactggaaatgatagaca | 413201 | - | - | - | - |
| 22978 | MGC2747 | NM_024104.2 | 142 | ccgacaaccttcccagaaagtcc | 386042 | - | - | - | - |
| 22979 | MGC2749 | NM_024069.2 | 343 | gaggaccctagtagagatgaaac | 385741 | - | - | - | - |
| 22980 | MGC2803 | NM_024038.2 | 467 | aagacggaggatgaggtattaac | 385580 | - | see also 8567 line | | |
| 22981 | MGC2865 | NM_032375.2 | 1033 | cagctgggcattagtgataatgg | 400060 | - | - | - | - |

Figure 46

| | | | | | | | | | | |
|---|---|---|---|---|---|---|---|---|---|---|
| 22982 | MGC29649 | NM_173810.3 | 502 | ctgccctctccgttacctaatct | 428205 | - | - | | - | |
| 22983 | MGC29761 | NM_144654.1 | 396 | gtggaatgttccggaacaatact | 410872 | - | - | | - | |
| 22984 | MGC29762 | NM_144706.2 | 602 | tggaaccagcgactcagttattt | 411513 | - | - | | - | |
| 22985 | MGC29784 | NM_173659.1 | 301 | caggagagctgacgttgttctca | 427134 | - | - | | - | |
| 22986 | MGC29814 | NM_182565.2 | 445 | ctgctccccacctgcaaacttca | 434470 | - | - | | - | |
| 22987 | MGC29875 | NM_014388.2 | 1355 | cagagaagcatccgactctatgc | 340501 | - | see also 5646 line | | | |
| 22988 | MGC29891 | NM_144618.1 | 521 | agggatgcccggactaaagtaga | 410346 | - | see also 9716 line | | | |
| 22989 | MGC29898 | NM_145048.2 | 669 | aggattgaatgctctagttcagc | 413186 | - | - | | - | |
| 22990 | MGC29937 | NM_144597.1 | 301 | ctgagctctgtcggtatctttcc | 410167 | - | - | | - | |
| 22991 | MGC29956 | NM_144638.1 | 216 | atgcgtcttaggcattattgtga | 410616 | - | - | | - | |
| 22992 | MGC3035 | NM_024293.2 | 780 | tggcccctggtggtttatcatga | 386151 | - | - | | - | |
| 22993 | MGC3047 | NM_032348.2 | 1313 | aagtacatcgacctagacaaagg | 399866 | - | see also 9169 line | | | |
| 22994 | MGC3048 | NM_024052.3 | 540 | aggagtatccaacccttacaacc | 385639 | - | see also 8579 line | | | |
| 22995 | MGC3067 | NM_024295.3 | 214 | cggcgatcacgcgctattggttc | 386174 | - | see also 8605 line | | | |
| 22996 | MGC3103 | NM_024036.3 | 1318 | gaggccacagcccgagtagaact | 385548 | - | - | | - | |
| 22997 | MGC3121 | NM_024031.2 | 1653 | tcgaccaatcaggttgaacaaga | 385503 | - | - | | - | |
| 22998 | MGC3130 | NM_024032.2 | 638 | ggggcctgaacaagacgaatttg | 385510 | - | - | | - | |
| 22999 | MGC3162 | NM_024078.1 | 971 | cgggctgttcatcttgattcaca | 385758 | - | - | | - | |
| 23000 | MGC3169 | NM_024074.1 | 312 | gggagccactgatcgattacttc | 385747 | - | - | | - | |
| 23001 | MGC31963 | NM_144580.1 | 1039 | tggctattgggaccaacactacc | 409937 | - | - | | - | |
| 23002 | MGC31967 | NM_174923.1 | 552 | tagcaagccggacaaggatatgg | 428694 | - | - | | - | |
| 23003 | MGC3200 | NM_032305.1 | 393 | atgtcaggtccgattgacaatgc | 399608 | - | see also 9151 line | | | |
| 23004 | MGC32020 | NM_152266.1 | 313 | gaggcttgttcgggttagaaatt | 415212 | - | - | | - | |
| 23005 | MGC32124 | NM_144611.2 | 774 | gtgaccggggactgttctgaagc | 410295 | - | - | | - | |
| 23006 | MGC3222 | NM_024334.1 | 1032 | gaggtgtttcatagagaactaag | 386312 | - | see also 8612 line | | | |

Figure 46

| | | | | | | | | | |
|---|---|---|---|---|---|---|---|---|---|
| 23007 | MGC3232 | NM_032313.2 | 1694 | gtgctataggccgcatagatttc | 399685 | - | - | - | - |
| 23008 | MGC3248 | NM_032486.2 | 147 | aagccagaacatcgttctcaatg | 400373 | - | see also 9218 line | | |
| 23009 | MGC3262 | NM_024029.3 | 945 | tggccatgggctgtaagttgtac | 385491 | - | - | - | - |
| 23010 | MGC3265 | NM_024028.2 | 624 | acgcagcgctttattgatgatgt | 385482 | - | - | - | - |
| 23011 | MGC32871 | NM_152311.1 | 716 | ctgctcgtcattcttctaaatat | 415510 | - | - | - | - |
| 23012 | MGC33190 | NM_152749.2 | 195 | ctgctccgacaatgtagatttag | 420516 | - | see also 9946 line | | |
| 23013 | MGC33212 | NM_152773.2 | 190 | gggagcccgagaacacctatatt | 420976 | - | - | - | - |
| 23014 | MGC33214 | NM_153354.2 | 863 | tggattacgactggctcaaatgc | 422999 | - | see also 9990 line | | |
| 23015 | MGC3329 | NM_024086.2 | 923 | aggggttcccaaagtaacgtaca | 385830 | - | see also 8587 line | | |
| 23016 | MGC33329 | NM_152782.2 | 1091 | tggggacacccgaagtatacttg | 421149 | - | - | - | - |
| 23017 | MGC33338 | NM_152366.2 | 355 | taggggccaggccgtacgattgg | 415827 | - | - | - | - |
| 23018 | MGC33365 | NM_173552.2 | 1044 | ttgacctcgcttggcaattaatg | 425971 | - | - | - | - |
| 23019 | MGC33367 | NM_144602.2 | 594 | tggcttacgaacgcaagctaaag | 410239 | - | - | - | - |
| 23020 | MGC33370 | NM_173807.2 | 584 | ctggcgtattgtagtaccaatga | 428091 | - | - | - | - |
| 23021 | MGC33371 | NM_144664.2 | 696 | ctggctctgtactttatcgtaca | 411141 | - | - | - | - |
| 23022 | MGC33382 | NM_173529.3 | 479 | gagcctaacaactgatgatctat | 425532 | - | - | - | - |
| 23023 | MGC33424 | NM_153705.2 | 1205 | aagcaggactcgccatattatga | 423752 | - | - | - | - |
| 23024 | MGC33530 | NM_182546.1 | 966 | cagaaaggtcagtcgtatagagt | 434322 | - | - | - | - |
| 23025 | MGC33547 | NM_144661.2 | 204 | aagaacagctgcgggaattgtgc | 411112 | - | - | - | - |
| 23026 | MGC33600 | NM_182539.1 | 695 | cagggtccacgtcgatcagttcc | 434239 | - | - | - | - |
| 23027 | MGC33602 | NM_152391.2 | 480 | tggccatgaatctatgtactttc | 416200 | - | - | - | - |
| 23028 | MGC33607 | NM_152775.1 | 97 | cagcgtccaagatcctaaattct | 420998 | - | - | - | - |

Figure 46

| | | | | | | | | | |
|---|---|---|---|---|---|---|---|---|---|
| 23029 | MGC33630 | NM_144668.2 | 1044 | agggccacgccaatattatctcc | 411165 | - | - | - | - |
| 23030 | MGC33648 | NM_153706.2 | 813 | aaggcgcagtcgtatctatgtat | 423763 | - | - | - | - |
| 23031 | MGC33653 | NM_153346.2 | 2196 | ctgtcggctagataccttattca | 422916 | - | - | - | - |
| 23032 | MGC33835 | NM_182548.2 | 411 | aagatctaccataccaactatgt | 434332 | - | - | - | - |
| 23033 | MGC33887 | NM_145036.1 | 1013 | cagcggtatgaacaacagatagt | 413044 | - | see also 9767 line | | |
| 23034 | MGC33889 | NM_173699.1 | 1047 | ttgtagccaagatacatgatacc | 427731 | - | - | - | - |
| 23035 | MGC33894 | NM_152914.1 | 419 | ctgctggtacatccagaagtacg | 421690 | - | - | - | - |
| 23036 | MGC33926 | NM_152390.1 | 847 | ctgcatcgcttatccgtttatta | 416190 | - | - | - | - |
| 23037 | MGC33947 | NM_152358.1 | 582 | tcgtggtctcggcttgtacatac | 415797 | - | - | - | - |
| 23038 | MGC33951 | NM_152448.1 | 495 | atgtacttccctctacagaatta | 416871 | - | - | - | - |
| 23039 | MGC33993 | NM_152737.1 | 657 | tagtagcctgcccgatgacaaca | 420284 | - | see also 9937 line | | |
| 23040 | MGC34032 | NM_152697.2 | 1632 | tggacgagccatacgatatcaca | 419865 | - | - | - | - |
| 23041 | MGC34034 | NM_153224.2 | 645 | aagcactcacgcaatctttgtcc | 422321 | - | - | - | - |
| 23042 | MGC34132 | NM_173654.1 | 495 | aggcgaacctctgtataactatg | 427088 | - | see also 10104 line | | |
| 23043 | MGC34290 | NM_152315.1 | 978 | ttgcaaccaggttcagttagaca | 415531 | - | - | - | - |
| 23044 | MGC34647 | NM_152456.1 | 524 | caggagccgacttcagtacatga | 416959 | - | - | - | - |
| 23045 | MGC34680 | NM_152346.1 | 607 | gcgaccttcggtccacgtttatt | 415751 | - | - | - | - |
| 23046 | MGC34713 | NM_173665.1 | 727 | ctgcatcttcgacgcttcttagt | 427196 | - | - | - | - |
| 23047 | MGC34741 | NM_153356.1 | 366 | aaggccttatgccaaatgtatga | 423036 | - | - | - | - |
| 23048 | MGC34799 | NM_182575.1 | 631 | ctgtcgtgctggcgctaaagtcc | 434489 | - | - | - | - |

Figure 46

| 23049 | MGC34805 | NM_173525.1 | 408 | ctgcaagatccgcctacatatgg | 425471 | - | - | - | - |
|-------|----------|-------------|-----|-------------------------|--------|---|--------------------|---|---|
| 23050 | MGC34824 | NM_173652.1 | 749 | tggaagttcaagcgactaactag | 427076 | - | - | - | - |
| 23051 | MGC34830 | NM_152314.1 | 367 | aaggcccagttagcacaatgtct | 415516 | - | - | - | - |
| 23052 | MGC34831 | NM_152632.1 | 2112 | gagcgaatcgattgttaaccacc | 419319 | - | - | - | - |
| 23053 | MGC34919 | NM_182582.1 | 1364 | gtgtatattacacacacaatata | 434514 | - | - | - | - |
| 23054 | MGC35010 | NM_178505.2 | 632 | ggggacagcggctgacatactgg | 430938 | - | - | - | - |
| 23055 | MGC35043 | NM_152770.1 | 377 | ctgagttccgtgatctttattcg | 420901 | - | - | - | - |
| 23056 | MGC35048 | NM_153208.1 | 1043 | aagataaatcggaaacaatcaac | 422146 | - | - | - | - |
| 23057 | MGC35062 | NM_198515.1 | 328 | aagtaaggtcggaaataaccaga | 438916 | - | - | - | - |
| 23058 | MGC35097 | NM_173546.1 | 699 | ctgcacgggaacaagatctatgt | 425824 | - | - | - | - |
| 23059 | MGC35118 | NM_152453.2 | 585 | aagatcaagccctctacataaag | 416951 | - | - | - | - |
| 23060 | MGC35138 | NM_173573.1 | 1806 | ctgtcggctccagaaagaacacc | 426292 | - | signal_transduction | - | - |
| 23061 | MGC35154 | NM_152614.1 | 197 | tagatcaggcctccttcaagacg | 418847 | - | - | - | - |
| 23062 | MGC35169 | NM_152324.1 | 503 | gtgctgggccctcgatgaagagt | 415591 | - | - | - | - |
| 23063 | MGC35179 | NM_153230.1 | 1075 | gagacccactctgatagatctcc | 422409 | - | - | - | - |
| 23064 | MGC35182 | NM_153707.1 | 579 | ttgctcacgacgtataagaaaga | 423793 | - | - | - | - |
| 23065 | MGC35194 | NM_152290.1 | 501 | cagcacctatgacgaccattaca | 415383 | - | - | - | - |
| 23066 | MGC35206 | NM_178552.2 | 812 | tggcaggaagacggatgatctgg | 431195 | - | - | - | - |
| 23067 | MGC35212 | NM_152764.1 | 928 | tgggctgcacgttcgttctatca | 420871 | - | - | - | - |
| 23068 | MGC35261 | NM_173494.1 | 470 | aggttgttgcagtgaactagtgg | 425148 | - | - | - | - |

Figure 46

| 23069 | MGC35295 | NM_152717.1 | 315 | tggccgtgcttactatcttcact | 420071 | - | receptor_acitivity、sig nal_transduction | - | - |
|---|---|---|---|---|---|---|---|---|---|
| 23070 | MGC35392 | NM_153336.1 | 590 | cggtgttgtcgcgtatgatgacg | 422770 | - | - | - | - |
| 23071 | MGC35468 | NM_153244.1 | 347 | atgcctatgggcaataatgcagg | 422575 | - | - | - | - |
| 23072 | MGC35521 | NM_145065.1 | 501 | ccggagccactcggtcatagtgg | 413477 | - | - | - | - |
| 23073 | MGC35555 | NM_178565.3 | 395 | cagtaagcgagctagttatgtat | 431297 | - | - | - | - |
| 23074 | MGC35558 | NM_145013.1 | 394 | aggatgtgcatgccatagtaacc | 412617 | - | - | - | - |
| 23075 | MGC3731 | NM_024313.1 | 218 | aggagcgccaccaggaatacttg | 386226 | - | - | - | - |
| 23076 | MGC3794 | NM_152902.2 | 214 | tggagaaattagccgatgaatta | 421535 | - | see also 9960 line | | |
| 23077 | MGC39325 | NM_147189.1 | 1350 | cagtctcaggacagtaacagtga | 415086 | - | - | - | - |
| 23078 | MGC39338 | NM_152480.1 | 361 | acgcctacccaagatttacgagg | 417025 | - | - | - | - |
| 23079 | MGC39350 | NM_144970.1 | 451 | tggttgatcgaaagaaagtcaca | 412037 | - | see also 9751 line | | |
| 23080 | MGC39520 | NM_153364.1 | 431 | ctgctgtatgctaccgtattaca | 423057 | - | - | - | - |
| 23081 | MGC39571 | NM_152728.1 | 438 | tggttagagtgagattctacatg | 420135 | - | - | - | - |
| 23082 | MGC39581 | NM_152784.2 | 359 | tggttcgttattgctgttagtag | 421156 | - | - | - | - |
| 23083 | MGC39633 | NM_152549.1 | 1408 | aagaacgccagcgccagtttaag | 417965 | - | - | - | - |
| 23084 | MGC39655 | NM_182541.1 | 544 | aagatcggactgcccatcatact | 434283 | - | - | - | - |
| 23085 | MGC39662 | NM_152394.2 | 933 | agggcatgcctcctgttgtattg | 416221 | - | - | - | - |
| 23086 | MGC39681 | NM_174939.2 | 182 | atgtatgccatatggatgaattt | 428759 | - | - | - | - |
| 23087 | MGC39696 | NM_152771.1 | 717 | gtggacacatctgcatcaaatcc | 420915 | - | - | - | - |
| 23088 | MGC39724 | NM_174906.1 | 386 | gaggctgcctccaccatattaga | 428576 | - | - | - | - |
| 23089 | MGC39725 | NM_152397.1 | 639 | ttgcattgtgacagagtgtattc | 416260 | - | | | |

Figure 46

| | | | | | | | | | | |
|---|---|---|---|---|---|---|---|---|---|---|
| 23090 | MGC39827 | NM_178499.2 | 652 | gtgaagataggccgtggatattt | 430897 | - | - | - | - | |
| 23091 | MGC39830 | NM_152756.1 | 63 | gagtacgagggcggaatgacagc | 420659 | - | - | - | - | |
| 23092 | MGC40053 | NM_152583.1 | 445 | gagtgcgctctaccataccttgt | 418257 | - | - | - | - | |
| 23093 | MGC40069 | NM_182615.1 | 401 | ttggctcacgctataatgtatta | 434701 | - | - | - | - | |
| 23094 | MGC40157 | NM_152350.1 | 410 | gggtagccacgaggttgcaaatt | 415765 | - | - | - | - | |
| 23095 | MGC40178 | NM_152325.1 | 237 | gtgatgagtacacttggaaatca | 415600 | - | - | - | - | |
| 23096 | MGC40179 | NM_173472.1 | 455 | cagagttgcgcacgatgaacaac | 424909 | - | - | - | - | |
| 23097 | MGC40195 | NM_152441.1 | 1090 | gggctgcgcacgctcaacattgg | 416840 | - | - | - | - | |
| 23098 | MGC40214 | NM_152416.1 | 208 | ctgcggaaacgggattatgaagg | 416484 | - | - | - | - | |
| 23099 | MGC40222 | NM_152738.1 | 498 | aagacacatggaccttatgtaat | 420304 | - | - | - | - | |
| 23100 | MGC40368 | NM_152772.1 | 741 | gtgtgctcccgtgcgagataatg | 420935 | - | - | - | - | |
| 23101 | MGC40397 | NM_152318.1 | 139 | gggacaggttgctcatcaactcc | 415572 | - | - | - | - | |
| 23102 | MGC40499 | NM_152755.1 | 386 | gagcggatcctggactatagtgt | 420653 | - | - | - | - | |
| 23103 | MGC4093 | NM_030578.2 | 527 | gggcagttggcgagaacagttgg | 394700 | - | - | - | - | |
| 23104 | MGC4126 | NM_032773.2 | 432 | ttgccggtacacttgtgtaattt | 401366 | - | - | - | - | |
| 23105 | MGC41816 | NM_016466.4 | 156 | gggccgagtgaagcatttaatcc | 357593 | - | - | - | - | |
| 23106 | MGC4189 | NM_032308.1 | 118 | cagccgggacaggctcctaaaca | 399634 | - | - | - | - | |
| 23107 | MGC41906 | NM_152474.2 | 715 | aagaatgcgtcacataatggaaa | 417015 | - | - | - | - | |
| 23108 | MGC42090 | NM_152774.1 | 260 | ttgtcgactagccagttatgaac | 420993 | - | - | - | - | |
| 23109 | MGC42157 | NM_153241.1 | 333 | atgttgtacagaactacttctca | 422570 | - | - | - | - | |
| 23110 | MGC4238 | NM_032332.2 | 103 | gtgcgagccgtacgatttaatgt | 399766 | - | - | - | - | |
| 23111 | MGC4248 | NM_032333.2 | 716 | gagacaaagtaaacctactttct | 399777 | - | - | - | - | |

Figure 46

| 23112 | MGC42493 | NM_178549.2 | 496 | gtgacactgacaagacatagaag | 431167 | - | - | | - | - |
| 23113 | MGC4251 | NM_032376.2 | 210 | ccgtgctgtgcgctagtttcatg | 400063 | - | kinase_related | - | | - |
| 23114 | MGC42530 | NM_173570.1 | 824 | ctgcgggttatttctgatactct | 426249 | - | - | | - | - |
| 23115 | MGC4268 | NM_031445.1 | 512 | cggtcacggtcgcctcatgaaag | 397041 | - | - | | - | - |
| 23116 | MGC43033 | NM_152711.1 | 168 | gtgttcaacggaaattcacaaat | 420007 | - | - | | - | - |
| 23117 | MGC4308 | NM_032359.2 | 772 | taggaactccggggagaattaaa | 399974 | - | - | | - | - |
| 23118 | MGC43122 | NM_173513.2 | 552 | aagataaagaatccttctgtagg | 425268 | - | - | | - | - |
| 23119 | MGC43690 | NM_182552.2 | 2528 | cagacacgcttacgtgtatgaaa | 434404 | - | - | | - | - |
| 23120 | MGC44287 | NM_182607.2 | 356 | ctgtagccatcgggcaatttaaa | 434603 | - | - | | - | - |
| 23121 | MGC44294 | NM_173499.1 | 278 | tagaacctcgtcgcgacatttct | 425166 | - | - | | - | - |
| 23122 | MGC44505 | NM_178553.2 | 1260 | cagtcgtggagtatcctatatgc | 431200 | - | - | | - | - |
| 23123 | MGC44593 | NM_173554.1 | 906 | gaggcctttaatgcacgaataga | 426023 | - | - | | - | - |
| 23124 | MGC4473 | NM_080719.1 | 637 | gagcgaaagggcccaactctacc | 406671 | - | - | | - | - |
| 23125 | MGC4504 | NM_024111.2 | 335 | gtgacgctccttgaagatcatga | 386064 | - | - | | - | - |
| 23126 | MGC45386 | NM_198527.1 | 109 | ccgaggggacccctacatcaac | 439136 | - | - | | - | - |
| 23127 | MGC45416 | NM_152398.1 | 431 | cagctaattctagatttggatca | 416266 | - | - | | - | - |
| 23128 | MGC45474 | NM_152369.2 | 1103 | atgtggtcgtaccatttaattgg | 415872 | - | - | | - | - |
| 23129 | MGC45491 | NM_153246.1 | 985 | acggctaatgcgctctaattacc | 422593 | - | - | | - | - |
| 23130 | MGC45714 | NM_152464.1 | 538 | gcgggtgctagctgcattgatcg | 416979 | - | - | | - | - |
| 23131 | MGC45780 | NM_173833.2 | 658 | aggccctgactcgcaatgtgaac | 428449 | - | receptor_acitivity | - | | - |
| 23132 | MGC45866 | NM_152259.2 | 1518 | aagacaactccagacataattaa | 415196 | - | - | | - | - |
| 23133 | MGC45871 | NM_182705.1 | 684 | gcgacgacctctctgatgaatgc | 434924 | - | - | | - | - |

Figure 46

| | | | | | | | | | |
|---|---|---|---|---|---|---|---|---|---|
| 23134 | MGC45873 | NM_152486.1 | 1400 | acgcccttgccggtcaaacttca | 417055 | - | - | - | - |
| 23135 | MGC4614 | NM_024294.1 | 980 | cagcagtcacgcaaacaacttat | 386171 | - | - | - | - |
| 23136 | MGC4618 | NM_032326.2 | 288 | cagcagttcgacagaagtgtaca | 399740 | - | - | - | - |
| 23137 | MGC4645 | NM_024515.2 | 317 | tggtcgaagtgactttagaatca | 386563 | - | - | - | - |
| 23138 | MGC46496 | NM_174952.1 | 187 | cagcaggcgacaggtagtaatgc | 428779 | - | - | - | - |
| 23139 | MGC46534 | NM_153340.2 | 739 | cagtgtggggtaataaatccaga | 422801 | - | see also 9989 line | | |
| 23140 | MGC46719 | NM_153713.1 | 436 | gggctctagtggtttatgagatg | 423847 | - | - | - | - |
| 23141 | MGC46732 | NM_153714.1 | 263 | gcgggtccacgagacttaacatt | 423865 | - | - | - | - |
| 23142 | MGC4701 | NM_024511.3 | 1722 | aagcatcgggacatatatcgtca | 386505 | - | - | - | - |
| 23143 | MGC4707 | NM_024113.2 | 847 | atgcacgacccagcaatggatga | 386080 | - | - | - | - |
| 23144 | MGC4728 | NM_198542.1 | 166 | ccgctcagcagggctatgtgacc | 439315 | - | - | - | - |
| 23145 | MGC4734 | NM_145051.2 | 633 | gtgtcactctgttgctcatattc | 413204 | - | - | - | - |
| 23146 | MGC47799 | NM_173545.1 | 266 | aggtggtcagctgcgaatcaaac | 425781 | - | see also 10081 line | | |
| 23147 | MGC47869 | NM_175874.2 | 850 | aaggctatgggaccaatcttaga | 429405 | - | - | - | - |
| 23148 | MGC4825 | NM_024122.2 | 723 | tggggtttacgaggatatatagt | 386144 | - | - | - | - |
| 23149 | MGC48935 | NM_178536.2 | 273 | ctgtgacacatggtcttatgtgc | 431119 | - | - | - | - |
| 23150 | MGC48972 | NM_173475.1 | 778 | aaggatctctaccggtttacatt | 424934 | - | see also 10069 line | | |
| 23151 | MGC48986 | NM_175881.2 | 372 | ctgcacgggctacatagatcatg | 429488 | - | - | - | - |
| 23152 | MGC48998 | NM_178550.2 | 135 | tggtcaggggacaattgtataag | 431177 | - | - | - | - |
| 23153 | MGC50104 | NM_198498.1 | 836 | atgaatgccgcagagcttattga | 438611 | - | - | - | - |
| 23154 | MGC50372 | NM_173566.1 | 693 | caggcatggaagctacatataac | 426207 | - | - | - | - |
| 23155 | MGC50721 | NM_173806.2 | 411 | ttgcaacatatcactattggaac | 428058 | - | - | - | - |
| 23156 | MGC50811 | NM_198559.1 | 913 | aggatatggagctctattcgaag | 439513 | - | - | - | - |

Figure 46

| 23157 | MGC50844 | NM_178562.2 | 360 | gtgtctacgctcggctaatgaag | 431287 | - | see also 10198 line | | |
| 23158 | MGC50896 | NM_182553.1 | 603 | atgctgacattctcaactactgc | 434413 | - | - | - | - |
| 23159 | MGC51025 | NM_178571.2 | 896 | aggcactccctgtggtattctac | 431337 | - | - | - | - |
| 23160 | MGC51029 | NM_173794.2 | 200 | tggatttaactgagtatgcaaga | 427970 | - | see also 10114 line | | |
| 23161 | MGC52022 | NM_198563.1 | 490 | ctgcgcttcggcgaatatggaga | 439538 | - | see also 10340 line | | |
| 23162 | MGC52057 | NM_194317.1 | 802 | ttgtggttagggctcatgttata | 436800 | - | - | - | - |
| 23163 | MGC5242 | NM_024033.1 | 472 | ccgtgtcgcctcacacaagttct | 385531 | - | - | - | - |
| 23164 | MGC52423 | NM_182517.1 | 483 | atggcttcatcgaggacaattac | 434056 | - | - | - | - |
| 23165 | MGC52498 | NM_182621.1 | 202 | cggcttccgcgaccacaagtact | 434717 | - | - | - | - |
| 23166 | MGC5306 | NM_024116.1 | 262 | ttgcaaatcgaagtgataactct | 386081 | - | - | - | - |
| 23167 | MGC5309 | NM_032286.1 | 322 | ttgagaatgctggtcaagattta | 399352 | - | - | - | - |
| 23168 | MGC5356 | NM_024059.2 | 909 | ctgctgcccaaccgatcctataa | 385707 | - | - | - | - |
| 23169 | MGC5384 | NM_030972.2 | 1551 | cagctggctcgacatagaagaat | 395902 | - | transcription_regulator | - | - |
| 23170 | MGC54289 | NM_178454.1 | 470 | atgaacgaacacggctactttcc | 430785 | - | see also 10190 line | | |
| 23171 | MGC5508 | NM_024092.1 | 208 | aagagagaagccccagttgatgt | 385954 | - | - | - | - |
| 23172 | MGC5509 | NM_024093.1 | 326 | aagacccctcatcgtatttgatg | 385961 | - | see also 8589 line | | |
| 23173 | MGC5528 | NM_024094.1 | 501 | aagacgtagacccaagttaaaga | 385976 | - | - | - | - |
| 23174 | MGC5560 | NM_019054.1 | 1914 | aagagttgttgatttcactatac | 374777 | - | - | - | - |
| 23175 | MGC5576 | NM_024056.2 | 150 | tggaattcaccgggagagatagc | 385683 | - | - | - | - |
| 23176 | MGC5590 | NM_024058.1 | 273 | cggcttctggacgagtttcatct | 385702 | - | - | - | - |
| 23177 | MGC57211 | NM_175878.2 | 1022 | ttgatcacactgctatatgctgc | 429485 | - | - | - | - |
| 23178 | MGC59868 | NM_198584.1 | 619 | tgggactactggacatatcctgg | 439833 | - | - | - | - |
| 23179 | MGC5987 | NM_138476.2 | 151 | acgtagaccctccgttccataag | 408355 | - | - | - | - |
| 23180 | MGC61550 | NM_182616.1 | 263 | gcgctactacgtgctgtatatcc | 434708 | - | - | - | - |
| 23181 | MGC61571 | NM_182523.1 | 246 | gagaggtgttctgaacaagttca | 434158 | - | - | - | - |

902

Figure 46

| | | | | | | | |
|---|---|---|---|---|---|---|---|
| 23182 | MGC61599 | NM_182505.3 | 460 | gtgaaggattcttatcacataat | 433995 | - | |
| 23183 | MGC61716 | NM_182501.1 | 828 | acggtaccaaaccctgataaga | 433976 | - | |
| 23184 | MGC64882 | NM_182627.1 | 1061 | ctgcctagcccattctatctct | 434783 | - | |
| 23185 | MGC7036 | NM_145058.1 | 767 | aagagctgcagtgctacaagagt | 413339 | - | |
| 23186 | MGC71806 | NM_198516.1 | 1232 | cggatcatctcgccatcctttga | 438935 | - | see also 10337 line |
| 23187 | MGC8721 | NM_016127.3 | 304 | atgtaagacggaacttagatattg | 356349 | - | see also 6679 line |
| 23188 | MGC874 | NM_016500.3 | 290 | tggcaacgatcctgacattgaga | 357889 | - | |
| 23189 | MGC8974 | NM_052940.3 | 671 | caggtatctgtgatttcagaaa | 405442 | - | see also 9463 line |
| 23190 | MGC9084 | NM_033418.1 | 866 | tggatcaggtttactaggttataa | 403846 | - | |
| 23191 | MGC955 | NM_024097.1 | 356 | ctgcaccatgtagccttgtaatat | 386008 | - | |
| 23192 | MGC9712 | NM_152689.2 | 2042 | tgggaatcgctcttatttatatt | 419819 | - | |
| 23193 | MGC9850 | NM_152705.1 | 442 | gcggagcagccaggacaagtacg | 419920 | - | |
| 23194 | MGC:13379 | NM_016499.2 | 339 | ctgccatgatggcctcctattac | 357887 | - | |
| 23195 | MIA2 | NM_054024.2 | 603 | aagtatggaacaggatcgtattc | 405986 | - | |
| 23196 | MIB | NM_020774.1 | 2314 | aagggtcaatgccacttgatct | 378841 | - | see also 7952 line |
| 23197 | MINA53 | NM_032778.3 | 1339 | ctgccgatcaagatcaatctga | 401439 | - | see also 9281 line |
| 23198 | MIR | NM_145021.2 | 292 | ctgctagagtctacagaagtaag | 412786 | - | |
| 23199 | MIS12 | NM_024039.1 | 662 | caggccgttgaacaggttattct | 385590 | - | cell_cycle |
| 23200 | MIST1 | NM_177455.2 | 274 | cacgcggatgcacaagctaaataa | 429883 | - | |
| 23201 | MIZF | NM_015517.3 | 1177 | aagtcccattaccgcaaagtaca | 353670 | - | transcription_regulator |
| 23202 | MKLN1 | NM_013255.2 | 513 | trgtctcaactggtatagcaagt | 338424 | - | see also 5368 line |
| 23203 | MLR1 | NM_153686.4 | 567 | trggaccaagtatagttatgtaaa | 423489 | - | see also 10004 line |
| 23204 | MLZE | NM_031415.1 | 973 | ttgagtgccaccaaattacgtca | 396714 | - | |
| 23205 | MMAA | NM_172250.1 | 761 | aggagcgggatatgaacataattc | 424389 | - | |
| 23206 | MMRP19 | NM_015957.1 | 515 | atgtacttccgagggtattata | 355614 | - | see also 6589 line |
| 23207 | MNS1 | NM_018365.1 | 312 | ccgtgttcagcgcaagcaatttc | 370862 | - | |
| 23208 | MO25 | NM_016289.2 | 1022 | tcggtgaactactactagataga | 356912 | - | see also 6789 line |
| 23209 | MOAP1 | NM_022151.3 | 1153 | aagttgtcggcttatgtactaag | 382568 | - | apoptosis |
| 23210 | MOB3B | NM_024761.2 | 772 | cagatcaacaacgcaggaaatatt | 390175 | - | kinase_related |
| 23211 | MOB4A | NM_173468.1 | 479 | ctgcaccaaagtatattgattac | 424877 | - | |
| 23212 | moblak | NM_130807.2 | 690 | ctggatcgaggcgcagatcaaca | 406933 | - | |
| 23213 | MOCOS | NM_017947.1 | 441 | cagtgggagtcgcttctgttacc | 364522 | - | |

Figure 46

| | | | | | | | | | |
|---|---|---|---|---|---|---|---|---|---|
| 23214 | MOCS1 | NM_005942.1 | 1394 | tggccactttatggaaaggatgc | 334547 | - | - | - | molybdenum cofactor deficiency |
| 23215 | MONDOA | NM_014938.2 | 650 | ttgtgatccgggagtatcacaag | 346215 | - | - | - | - |
| 23216 | MOPT | NM_194270.1 | 351 | atgaaggacaatttaaggataat | 436161 | - | - | - | - |
| 23217 | MPN2 | NM_183062.2 | 858 | atggatatgtgataacatagaaa | 435591 | - | - | - | - |
| 23218 | MR-1 | NM_015488.3 | 420 | ctgtcctctcggacaactacagc | 353509 | - | see also 6437 line | | |
| 23219 | MRGD | NM_198923.1 | 110 | tggagtcagccctaaactattcc | 439996 | - | - | - | - |
| 23220 | MRPL10 | NM_145255.2 | 739 | gagagcaacgcgagaaggattct | 413927 | - | - | - | - |
| 23221 | MRPL21 | NM_181512.1 | 357 | aagacctgatcttaattggaaat | 432376 | - | see also 10235 line | | |
| 23222 | MRPL28 | NM_006428.3 | 302 | tggatcctgggccaaatatatgc | 335544 | - | - | - | melanoma |
| 23223 | MRPL38 | NM_032478.2 | 729 | tggcggacctaccgagagtattt | 400351 | - | see also 9212 line | | |
| 23224 | MRPL45 | NM_032351.3 | 739 | aagttcgctgttcaagtatgatg | 399877 | - | - | - | - |
| 23225 | MRPL52 | NM_178336.1 | 159 | gagctcccagactggtcatatgc | 430711 | - | - | - | - |
| 23226 | MRPL53 | NM_053050.2 | 346 | gagacgggcatcgcctgattatg | 405855 | - | - | - | - |
| 23227 | MRPL54 | NM_172251.1 | 239 | atgccatgggcgtcaacatctac | 424403 | - | - | - | - |
| 23228 | MRPL55 | NM_181441.1 | 433 | atgctggcgatgcccatagatct | 432134 | - | - | - | - |
| 23229 | MRPS9 | NM_182640.1 | 769 | cagaggtttcgaagaagtgtaac | 434845 | - | - | - | - |
| 23230 | MS4A12 | NM_017716.1 | 568 | ttggccttcattggagtgattct | 361140 | - | receptor_acitivity、 signal_transduction | - | - |
| 23231 | MS4A3 | NM_006138.3 | 570 | gagtcaccggacctatgcaatta | 334868 | - | receptor_acitivity、 signal_transduction | - | - |
| 23232 | MTBP | NM_022045.2 | 980 | cagatctaccctcctgctatatg | 381703 | - | see also 8246 line | | |
| 23233 | MTMR9 | NM_015458.3 | 1898 | ttggaatagatcctctaagtatt | 353284 | - | see also 6422 line | | |
| 23234 | MUC13 | NM_033049.1 | 127 | gcgactagtggtcctacagtagc | 402700 | - | - | - | - |
| 23235 | MUC15 | NM_145650.2 | 1208 | gggaattctagctactacaatcc | 414622 | - | see also 9792 line | | |
| 23236 | MUC20 | NM_152673.1 | 122 | gagcagacactgcgatgacaacg | 419563 | - | - | - | - |
| 23237 | MUC7 | NM_152291.1 | 305 | aagcttccaccttcacctaataa | 415391 | - | - | - | - |
| 23238 | MUF1 | NM_006369.3 | 647 | atgttggcgagccaagtttatgg | 335357 | - | - | - | - |
| 23239 | MXD3 | NM_031300.2 | 224 | gcggtcagtgcacaatgaactgg | 396592 | - | - | - | - |
| 23240 | my048 | NM_080390.2 | 320 | gagagttgaggaaccgttaaagg | 406371 | - | - | - | - |
| 23241 | MYCT1 | NM_025107.1 | 614 | ctgactactggtccagtaacagt | 393674 | - | - | - | - |
| 23242 | MYH14 | NM_024729.2 | 2904 | gagtgcagccgtcaaatgcaaac | 389723 | - | - | - | - |
| 23243 | MYOZ1 | NM_021245.2 | 1199 | tggggagcctgtagactacaacg | 380892 | - | - | - | - |
| 23244 | MYOZ3 | NM_133371.2 | 769 | acgactaccgaaatttcaacaag | 407196 | - | - | - | - |
| 23245 | N4BP1 | NM_014664.1 | 2229 | gagaacaaggcgtgatcctaatg | 341933 | - | - | - | - |
| 23246 | NAG73 | NM_032570.1 | 783 | gtgattgggcacctcacttaacg | 400764 | - | - | - | - |
| 23247 | NALP10 | NM_176821.2 | 1347 | gagtagtaacgactaccaattgg | 429838 | - | - | - | - |
| 23248 | NALP4 | NM_134444.1 | 979 | gagagtgataggttagtgtattt | 407312 | - | - | - | - |

Figure 46

| | | | | | | | | | |
|---|---|---|---|---|---|---|---|---|---|
| 23249 | NALP8 | NM_176811.2 | 1883 | tggggtcccgcagttattctact | 429656 | - | - | - | - |
| 23250 | NAPE-PLD | NM_198990.1 | 478 | atgggtccaaagcgatttcgtcg | 440321 | - | see also 10351 line | | |
| 23251 | NARFL | NM_022493.1 | 397 | cagctgaatcctacagatactgc | 383497 | - | - | - | - |
| 23252 | NARG2 | NM_024611.2 | 920 | aaggacgatatagctaccattga | 387891 | - | - | - | - |
| 23253 | NCDN | NM_014284.1 | 1989 | acgcctgcttcacatctctaatg | 340130 | - | - | - | - |
| 23254 | NDE1 | NM_017668.1 | 1015 | cagcgtgcctttgggtgataagg | 360493 | - | - | - | - |
| 23255 | NDEL1 | NM_030808.2 | 966 | ggggatctcttacggaaagtagg | 395248 | - | see also 8953 line | | |
| 23256 | NDFIP1 | NM_030571.2 | 668 | caggatttggtctctctctaatt | 394680 | - | kinase_related | - | - |
| 23257 | NDNL2 | NM_138704.2 | 830 | ccgaccccgtcgactacgaattc | 408668 | - | - | - | - |
| 23258 | NDUFS7 | NM_024407.3 | 487 | gaggaggctactaccactattcc | 386363 | - | - | NADH dehydrogenase (ubiquinone) | Leigh syndrome |
| 23259 | NDUFV3 | NM_021075.2 | 1855 | cagcacgtacaccttcttagacc | 380520 | - | - | NADH dehydrogenase (ubiquinone) | - |
| 23260 | NECAB2 | NM_019065.2 | 461 | gggttataccaagaaggtatatg | 374890 | - | - | - | - |
| 23261 | NEDD1 | NM_152905.2 | 442 | tggtaagcggaggcctaaataac | 421617 | - | - | - | - |
| 23262 | NEFH | NM_021076.2 | 2355 | aaggtcccctgcagacaaattcc | 380524 | - | - | molecular_function unknown | - |
| 23263 | NEGR1 | NM_173808.1 | 218 | tggctgaaccggtcaagtattat | 428166 | - | see also 10116 line | | |
| 23264 | NELF | NM_015537.2 | 129 | ctgacgacatccctattcgtacc | 353754 | - | - | - | - |
| 23265 | NES | NM_006617.1 | 148 | gggggaggagtcgtttcagatgt | 335943 | - | - | - | - |
| 23266 | NETO1 | NM_138966.2 | 480 | cgggaatgcatctacatcataga | 409288 | - | see also 9647 line | | |
| 23267 | NETO2 | NM_018092.3 | 988 | aggccaagccgttgattgcatct | 366600 | - | - | - | - |
| 23268 | NEUROD2 | NM_006160.2 | 685 | ctgcgcctagccaagaactatat | 334900 | - | see also 4263 line | | |
| 23269 | NEUROG2 | NM_024019.2 | 567 | gggtctggtacacgattgcaaac | 385475 | - | - | - | - |
| 23270 | NFAM1 | NM_145912.3 | 787 | gagagaccgcatagattcgaaga | 414962 | - | - | - | - |
| 23271 | NFKBIB | NM_002503.2 | 990 | gcggagacgagggcgatgaatac | 330989 | - | transcription_regulator, signal_transduction | transcription co-activator | - |
| 23272 | NFKBIL1 | NM_005007.2 | 429 | cagatgcctacaccgatttcttc | 333363 | - | - | - | rheumatoid arthritis |
| 23273 | NFKBIL2 | NM_013432.3 | 2080 | cagaacccccctctaatagcact | 338916 | - | transcription_regulator | - | - |
| 23274 | NFRKB | NM_006165.2 | 3104 | cagacggttctgcgaatcactcc | 334962 | - | see also 4268 line | | |
| 23275 | NGEF | NM_019850.1 | 774 | aggaataccgagataaatcgact | 375541 | - | - | - | - |
| 23276 | NGLY1 | NM_018297.2 | 896 | ctgccagttcagcaatcgattcc | 369921 | - | see also 7372 line | | |
| 23277 | NGX6 | NM_016446.2 | 1809 | ctggccattcggagtcgatatgt | 357487 | - | - | - | - |

Figure 46

| | | | | | | | |
|---|---|---|---|---|---|---|---|
| 23278 | NHLRC1 | NM_198586.2 | 485 | gggaggatgcgcgcatcagtttg | 439850 | - | |
| 23279 | NHS | NM_198270.2 | 1617 | gagtgcccaaaccgaggatattct | 437297 | - | |
| 23280 | NICE-3 | NM_015449.1 | 518 | tggatctgcgaaacactagtacg | 353088 | - | |
| 23281 | NICN1 | NM_032316.2 | 320 | gagggagcccaggagtatgtatc | 399698 | - | |
| 23282 | NIF3L1 | NM_021824.1 | 1117 | gagatgtcccatcatgatacttt | 381094 | - | |
| 23283 | NIPA | NM_016478.2 | 212 | ctgccacatccagtcagttaat | 357703 | - | |
| 23284 | NIPA2 | NM_030922.2 | 552 | gagctctcagccgtgctagtaagt | 395464 | - | |
| 23285 | NIPSNAP1 | NM_003634.1 | 621 | gtgcacctgtggccgattcctcagg | 331852 | - | see also 2452 line |
| 23286 | NIPSNAP3B | NM_018376.2 | 391 | aggaatggcaagaacaatctatc | 370919 | - | |
| 23287 | NJMU-R1 | NM_022344.1 | 628 | atgttgtatgccatccaaagg | 382616 | - | kinase_related |
| 23288 | NKAP | NM_024528.1 | 1015 | aggctgaagaaccatcagattta | 386709 | - | |
| 23289 | NKD2 | NM_033120.2 | 380 | gttggacgttcacgctctatgact | 403370 | - | |
| 23290 | NLGN1 | NM_014932.2 | 2566 | cagcgcactactaccaatgatct | 346035 | - | see also 6115 line |
| 23291 | NLGN3 | NM_018977.1 | 675 | tggatatcgtgctacttacatc | 373933 | - | see also 7624 line |
| 23292 | NOB1P | NM_014062.1 | 383 | ctgcacatttctggtttccatct | 339509 | - | |
| 23293 | NOD9 | NM_024618.2 | 1908 | aaggtggttcacgagtgtttgg | 388063 | - | |
| 23294 | NOL6 | NM_022917.4 | 1264 | cagtcaacggatcagtttatgt | 384872 | - | |
| 23295 | NOPE | NM_020962.1 | 2952 | gagcgacactcggtacttcttca | 380310 | - | |
| 23296 | NOR1 | NM_145047.2 | 995 | gagcccggattccggttgaatct | 413163 | - | |
| 23297 | NOSIP | NM_015953.3 | 590 | cagtgtgggtctccaagtaagg | 355600 | - | |
| 23298 | NOSTRIN | NM_052946.2 | 1043 | aaggggaactcctacaaactgtca | 405488 | - | see also 9464 line |
| 23299 | 1.Nov | NM_144663.1 | 597 | ctgtatggatcgttctacttc | 411134 | - | |
| 23300 | NPD007 | NM_020684.2 | 802 | gaggaggatatccgtgttcaagt | 377973 | - | |
| 23301 | NPHP3 | NM_153240.3 | 3133 | atggtgcggaccatccatatact | 422547 | - | |
| 23302 | NPL | NM_030769.1 | 685 | gagcagtgggcagtaccctataac | 394930 | - | |
| 23303 | NPL4 | NM_017921.1 | 1720 | gagccgtgcggaccagaaatga | 364027 | - | |
| 23304 | NPR2L | NM_006545.3 | 161 | ctgccgcatcgaatgcatatct | 335734 | - | non-small-cell lung cancer |
| 23305 | NRAP | NM_006175.2 | 1783 | gggagcttagcaatattaa | 335019 | - | |
| 23306 | NRCAM | NM_005010.1 | 1241 | aaggactgctaccccaattatt | 333400 | - | see also 3503 line |
| 23307 | NRPS998 | NM_181806.1 | 437 | cagggataccgaagattgtcaga | 433538 | - | see also 10254 line |
| 23308 | NS | NM_014366.3 | 1384 | cagtcttccgtctgacaaatgg | 340402 | - | |
| 23309 | NS3TP2 | NM_023927.1 | 1033 | gagaactctgagattccatgc | 385417 | - | see also 8553 line |
| 23310 | NSFL1C | NM_016143.3 | 1966 | atgactactttcccgaacaaaga | 356454 | - | see also 6693 line |
| 23311 | NSPC1 | NM_032673.1 | 206 | tgtgaagtacctccaaactagc | 401202 | - | |
| 23312 | NT5C2L1 | NM_152729.2 | 230 | aaggtacgataaaggaatrgctca | 420141 | - | |
| 23313 | NUDT12 | NM_031438.2 | 1343 | aggatgcccgctggttcactaga | 397004 | - | |

906

Figure 46

| | | | | | | | | | |
|---|---|---|---|---|---|---|---|---|---|
| 23314 | NUDT13 | NM_015901.3 | 611 | gtgtgcccttccaataatataat | 355400 | - | - | - | - |
| 23315 | NUDT14 | NM_177533.2 | 199 | cagcgtgaccgttctcttattca | 430274 | - | phosphatase | - | - |
| 23316 | NUDT15 | NM_018283.1 | 481 | gtggatgtgactcatgattcaga | 369692 | - | - | - | - |
| 23317 | NUDT8 | NM_181843.1 | 323 | ctgctgcggcctgtgtatgatcc | 433806 | - | - | - | - |
| 23318 | NUP133 | NM_018230.2 | 2407 | gtggcatccgaacggtaataata | 368841 | - | - | - | - |
| 23319 | NUP210 | NM_024923.2 | 199 | gggccacgcgcgttaacttcacg | 392209 | - | see also 8807 line | | |
| 23320 | NUPL1 | NM_014089.2 | 507 | cagctagcggtctgactctttcg | 339539 | - | see also 5488 line | | |
| 23321 | NURIT | NM_152719.1 | 153 | ttgcactcaaggccaaattatac | 420074 | - | - | - | - |
| 23322 | NX17 | NM_020665.2 | 558 | gaggtgcaatcagccataagaat | 377888 | - | - | - | - |
| 23323 | NXN | NM_022463.3 | 1228 | ctgactccctgcgagattacacc | 382998 | - | - | - | - |
| 23324 | NXPH1 | NM_152745.1 | 1343 | tggcaatgggacatttagtgttt | 420427 | - | - | - | - |
| 23325 | NY-REN-58 | NM_016122.1 | 1416 | aaggatcgtgaattaatacgtaa | 356313 | - | see also 6676 line | | |
| 23326 | NYD-SP12 | NM_031955.2 | 167 | gtgaataggatctatcatgatca | 397494 | - | see also 9060 line | | |
| 23327 | NYD-SP14 | NM_031956.1 | 724 | aagccgaggcacacatgcatatg | 397532 | - | - | - | - |
| 23328 | NYD-SP17 | NM_032600.2 | 604 | atgcacaacgactaaagatatcc | 401103 | - | - | - | - |
| 23329 | NYD-SP18 | NM_032599.1 | 621 | tggatccgactggttcaaattct | 401084 | - | - | - | - |
| 23330 | NYD-SP20 | NM_032598.2 | 1056 | ctgttacacctggcccatattat | 401066 | - | - | - | - |
| 23331 | NYD-SP21 | NM_032597.2 | 1214 | atgcaatccctagatatgctatc | 401025 | - | - | - | - |
| 23332 | NYD-SP26 | NM_033122.2 | 284 | atgatatggggaccgactttatt | 403433 | - | - | - | - |
| 23333 | NYD-SP28 | NM_033124.2 | 1591 | cagcccttgtccatacgtatagc | 403494 | - | - | - | - |
| 23334 | NYD-SP29 | NM_145172.1 | 1247 | tcggtagccgtgcgactttcttt | 413668 | - | - | - | - |
| 23335 | NYD-TSP1 | NM_032567.2 | 1407 | ttgcaatacccagttcaatattc | 400757 | - | - | - | - |
| 23336 | OAZ | NM_015069.1 | 1279 | gagatgttcgccgacatcaatag | 347730 | - | see also 6220 line | | |
| 23337 | OCIA | NM_017830.1 | 263 | gagagtcttcgcagaatgcaatg | 362844 | - | see also 7158 line | | |
| 23338 | OCSP | NM_031945.2 | 295 | gagcagctgcgtcaagtatctga | 397464 | - | - | - | - |
| 23339 | ODF3 | NM_053280.3 | 860 | atgtgcgggtgaccaagttcaag | 405967 | - | - | - | - |
| 23340 | ODF4 | NM_153007.3 | 190 | cagagtactctgggaatgagttc | 421797 | - | - | - | - |
| 23341 | OFCC1 | NM_153003.1 | 557 | gagtcacgaaatccacaaagaag | 421792 | - | - | - | - |
| 23342 | OKL38 | NM_013370.2 | 1148 | cggggacatcgcccactactaca | 338849 | - | - | - | - |
| 23343 | OLIG3 | NM_175747.2 | 660 | aggctggttggcgagatctatgg | 429270 | - | - | - | - |
| 23344 | OR12D2 | NM_013936.2 | 120 | tggagccgttctgatgattgtca | 338970 | - | receptor_acitivity、sig nal_transduction、gpcr | olfactory receptor | - |
| 23345 | OR2B2 | NM_033057.1 | 452 | gtggctttagcaattcagtatta | 402746 | - | receptor_acitivity、sig nal_transduction、gpcr | rhodopsin-like receptor | - |

EP 1 752 536 A1

Figure 46

| 23346 | OR2C3 | NM_198074.1 | 60 | acgaccctcactagaaactgtcc | 436957 | - | - | - | - |
|-------|-------|-------------|-----|-------------------------|--------|---|---|---|---|
| 23347 | OR2F1 | NM_012369.1 | 800 | ctgtccttcaggagaagttgttc | 338104 | - | receptor_acitivity、sig nal_transduction、gpcr | olfactory receptor | - |
| 23348 | OR2S2 | NM_019897.1 | 719 | tcgtcttctacgggaccttattc | 375556 | - | receptor_acitivity、sig nal_transduction、gpcr | olfactory receptor | - |
| 23349 | OR3A3 | NM_012373.1 | 650 | tggcacccttggtcttcatcagt | 338105 | - | receptor_acitivity、sig nal_transduction、gpcr | olfactory receptor | - |
| 23350 | OR51B2 | NM_033180.1 | 217 | ttgaccacgatgcctactgtaat | 403543 | - | receptor_acitivity、sig nal_transduction、gpcr | olfactory receptor | - |
| 23351 | OR7C2 | NM_012377.1 | 244 | ctggtgaatatccaaacacaaag | 338108 | - | - | olfactory receptor | - |
| 23352 | ORAOV1 | NM_153451.1 | 114 | tggcagtcaggacatattcgatg | 423262 | - | - | - | oral cancer |
| 23353 | ORAOV2 | NM_018043.4 | 805 | gagcacgattgtctatgagatct | 365743 | - | - | - | - |
| 23354 | ORMDL2 | NM_014182.3 | 13 | gggtggcacacagcgaagtaaac | 339938 | - | see also 5530 line | | |
| 23355 | ORMDL3 | NM_139280.1 | 427 | cggcctctcggaagttcttgacc | 409610 | - | - | - | - |
| 23356 | OSAP | NM_032623.2 | 659 | aagaacgaaacctctgatgaata | 401143 | - | see also 9262 line | | |
| 23357 | OSRF | NM_012382.1 | 313 | aagatatcgggaggcaattcaga | 338117 | - | - | - | - |
| 23358 | OSTbeta | NM_178859.2 | 161 | cagctgtggtggtcattataagc | 432019 | - | - | neurohypophyseal hormone | |
| 23359 | OSTM1 | NM_014028.2 | 766 | tagtctgtacagtgaaatgcaaa | 339330 | - | - | - | - |
| 23360 | OTOA | NM_144672.2 | 1612 | aaggaggcaacctggattcaact | 411258 | - | see also 9728 line | | |
| 23361 | OTOP1 | NM_177998.1 | 1722 | ctgtcgacctgagtatgacaatg | 430364 | - | see also 10176 line | | |
| 23362 | OTOP2 | NM_178160.1 | 1220 | gggtcagtacgccatctcttact | 430538 | - | - | - | - |
| 23363 | OTOP3 | NM_178233.1 | 726 | gtgggttctggccgttaccaatg | 430627 | - | - | - | - |
| 23364 | OVCA2 | NM_080822.1 | 461 | ggggcattgggttcaaggaatcc | 406787 | - | - | - | - |
| 23365 | OVCH1 | NM_183378.1 | 1258 | gggagtctggctatattggtaga | 435769 | - | - | - | - |
| 23366 | OVTN | NM_198185.1 | 987 | aagcctccacctatattatgaga | 437214 | - | - | - | - |
| 23367 | P114-RHO-GEF | NM_015318.1 | 165 | acgcaactcggaccaatcacagg | 351466 | - | - | - | - |
| 23368 | p25 | NM_007030.1 | 620 | tggacgagtcaggctatgtgtcc | 336881 | - | - | - | - |
| 23369 | p30 | NM_181716.1 | 656 | ctgagcacataacgacttacacg | 432991 | - | - | - | - |
| 23370 | P38IP | NM_017569.2 | 2237 | cagcagcccacaacttgtagtcc | 359173 | - | see also 7036 line | | |
| 23371 | P518 | NM_198180.1 | 6 | aaggccttaccccctgatctact | 437178 | - | - | - | - |
| 23372 | P5326 | NM_031450.2 | 252 | atggaccctggctagtgtttga | 397056 | - | - | - | - |

Figure 46

| | | | | | | | | |
|---|---|---|---|---|---|---|---|---|
| 23373 | PACAP | NM_016459.2 | 560 | caggacatgttgcactacttgg | 357579 | - | | - |
| 23374 | PACRG | NM_152410.1 | 528 | aagctgattaccatcatcatct | 416432 | - | | - |
| 23375 | PAF53 | NM_022490.1 | 1097 | aaggcggaagattactgcatatgt | 383480 | - | transcription_regulator | - |
| 23376 | PAGE-5 | NM_130467.2 | 306 | atgagtgagcatgtaacaagatc | 406917 | - | | - |
| 23377 | PAK1IP1 | NM_017906.1 | 783 | tagtgtgcctcgcgaatttaaa | 363818 | - | | - |
| 23378 | PALM2 | NM_053016.1 | 1116 | gtgacggacgtgtccactattga | 405795 | - | see also 9481 line | - |
| 23379 | PALMD | NM_017734.2 | 1873 | gggactgaggatccatccttaac | 361305 | - | | - |
| 23380 | PAN3 | NM_175854.4 | 1277 | ctgcattgcgtaccattcataca | 429329 | - | see also 10154 line | - |
| 23381 | PAQR9 | NM_198504.1 | 501 | cagcacggtggcctactactact | 438743 | - | see also 10333 line | - |
| 23382 | PBF | NM_018660.1 | 1665 | ccggagccggtgctgctaagcttca | 372387 | - | | - |
| 23383 | PBOV1 | NM_021635.1 | 288 | aaggcaacagagttcattgatta | 380936 | - | | - |
| 23384 | PCNX | NM_014982.1 | 3237 | cagattcgattgaatagacatt | 346627 | - | see also 6164 line | - |
| 23385 | PD2 | NM_019088.1 | 1179 | gggtgacgggtttactacaatg | 375149 | - | see also 7702 line | - |
| 23386 | PDCD10 | NM_007217.3 | 740 | cagtaagatcccagatgagatca | 337216 | - | | - |
| 23387 | PDCD4 | NM_014456.3 | 739 | aagactttaacaccaatcataca | 340708 | - | see also 5683 line | - |
| 23388 | PDCD7 | NM_005707.1 | 942 | cagccgctgatggcgtactatct | 334181 | - | see also 3960 line | - |
| 23389 | PDIP38 | NM_015584.2 | 558 | gtgacttcttggctaaccatga | 354334 | - | see also 6497 line | - |
| 23390 | PEGASUS | NM_022466.2 | 514 | atgttagtagacggggtttgaaag | 383004 | - | | - |
| 23391 | PELI1 | NM_020651.2 | 4891 | gggttcaacacactagcatttcc | 377871 | - | see also 7907 line | - |
| 23392 | PELI2 | NM_021255.1 | 1253 | aggacgcgccaactcatgcttca | 380924 | - | | - |
| 23393 | PEPP3 | NM_014935.2 | 1601 | ccggcagccagtcgtcattatgatg | 346103 | - | see also 6118 line | - |
| 23394 | PERQ1 | NM_022574.2 | 704 | cggcgcaagttgaatttgattt | 383583 | - | | - |
| 23395 | PEX11G | NM_080662.2 | 187 | aggaccatcttgcgactctttga | 406588 | - | | - |
| 23396 | PEX26 | NM_017929.2 | 774 | gagcgggcgactggatgtacttca | 364272 | - | | - |
| 23397 | PEX5R | NM_016559.1 | 658 | atgggacgatgttaagtttcatg | 358118 | - | | - |
| 23398 | PF20 | NM_024532.2 | 715 | ccgactataagggtgttacatga | 386800 | - | | - |
| 23399 | Pfs2 | NM_016095.1 | 220 | ctggcgattaacctgaaacaaag | 356263 | - | | - |
| 23400 | PGA5 | NM_014224.1 | 86 | aggtcccctcatcagaaagaaag | 339972 | - | pepsin A | - |
| 23401 | PGBD2 | NM_170725.1 | 662 | gagtcttacggctcaggaattga | 424125 | - | | - |
| 23402 | PGBD4 | NM_152595.2 | 698 | atgattccacgtcaaagtatga | 418579 | - | | - |
| 23403 | PGBD5 | NM_024554.2 | 20 | gagtctcttcgtgcaaagtact | 387200 | - | | - |
| 23404 | PGSF1 | NM_174947.2 | 458 | aagtctgtattgacattctcata | 428778 | - | | - |
| 23405 | PHAX | NM_032177.1 | 55 | ttggaggtcgcgatatggaaga | 398509 | - | | - |
| 23406 | PHC3 | NM_024947.2 | 1703 | cagactgttgcggtaaacctaca | 392514 | - | see also 8814 line | - |
| 23407 | PHF7 | NM_016483.4 | 986 | ctgtcttatcttatctagtaagc | 357757 | - | transcription_regulator | - |

909

Figure 46

| | | | | | | | |
|---|---|---|---|---|---|---|---|
| 23408 | PHGDHL1 | NM_177967.2 | 944 | tagccataagtggacttagtcc | 430334 | - | - |
| 23409 | PHOSPHO1 | NM_178500.2 | 696 | gaggcgacctgctgcagtttgtgg | 430916 | - | phosphatase |
| 23410 | PHYHIP | NM_014759.1 | 495 | tggagaggtcacccattacttc | 343160 | - | - |
| 23411 | PIB5PA | NM_014422.1 | 1080 | tgggtaccaacaaatacgtaacc | 340568 | - | - |
| 23412 | PIBF1 | NM_006346.1 | 83 | cagttctacggatgatatttcc | 335244 | - | see also 4443 line |
| 23413 | PIGN | NM_012327.3 | 2007 | atgcttaccgctggacttactgc | 338076 | - | see also 5279 line |
| 23414 | PIGT | NM_015937.2 | 433 | ctgcgcctctcaacttcatcg | 355517 | - | - |
| 23415 | PIGW | NM_178517.2 | 630 | aagggtccaaattgcattacttt | 431002 | - | see also 10195 line |
| 23416 | PIK3AP1 | NM_152309.1 | 1413 | aggcccagtcgagtatatatga | 415481 | - | kinase_related |
| 23417 | PIP3AP | NM_019061.2 | 2052 | cagcgctacctacgttgattcc | 374844 | - | see also 7685 line |
| 23418 | PISD | NM_014338.3 | 870 | gttggctgcacagcgtgattagc | 340216 | - | - |
| 23419 | PIWIL1 | NM_004764.2 | 1285 | tagccgttcatacaagactaact | 332957 | - | see also 3275 line |
| 23420 | PIWIL2 | NM_018068.2 | 1379 | tcgtacctatcgtattgatgatg | 366076 | - | - |
| 23421 | PJA1 | NM_022368.2 | 335 | caggagggtatcagtccaataca | 382707 | - | - |
| 23422 | PKD1L3 | NM_181536.1 | 1468 | gtgttactaagcgctaatcgtaa | 432470 | - | see also 10237 line |
| 23423 | PKHD1L1 | NM_177531.2 | 1531 | ttggactaccagtatcgaaat | 429935 | - | see also 10166 line |
| 23424 | PLAC4 | NM_182832.1 | 295 | tggcctaaacatgttctatata | 435379 | - | - |
| 23425 | PLAC8 | NM_016619.1 | 425 | aaggagagccatgcgtactttct | 358466 | - | - |
| 23426 | PLEKHB2 | NM_017958.1 | 522 | gcgtaccgccaggaactcaagt | 364880 | - | - |
| 23427 | PLVAP | NM_031310.1 | 417 | cgggtcatctacacgcaacaatca | 396710 | - | - |
| 23428 | PMAIP1 | NM_021127.1 | 260 | caggagatttggagacaaactga | 380528 | - | - |
| 23429 | PMFBP1 | NM_031293.1 | 699 | gagcaacatcgagttactagaat | 396535 | - | transcription_regulator |
| 23430 | PNAS-131 | NM_031446.2 | 449 | aaggactgcaaaccaaatcatg | 397048 | - | - |
| 23431 | PNAS-4 | NM_016076.2 | 609 | gagattcctcgctggatcaatcg | 356213 | - | - |
| 23432 | PNLDC1 | NM_173516.1 | 283 | ttgccatcggagtggtatctaa | 425274 | - | transcription_regulator |
| 23433 | PNMA2 | NM_007257.3 | 736 | caggccacggagatagtcaaaga | 337354 | - | - |
| 23434 | PNMA5 | NM_052926.1 | 754 | aagatgagggccgaagtatgaca | 405342 | - | - |
| 23435 | PODN | NM_153703.2 | 918 | gagcagcctgcgcgagctatacc | 423709 | - | - |
| 23436 | POF1B | NM_024921.2 | 931 | ctgatctgagccgtaagaatacg | 392172 | - | - |
| 23437 | POLN | NM_181808.1 | 1418 | gagaacggtttcttataaagagc | 433706 | - | - |
| 23438 | POMT1 | NM_007171.2 | 1587 | gtgaacacttccgctgtcttaaa | 337001 | - | see also 4973 line |
| 23439 | POP5 | NM_015918.3 | 408 | ttgcagaactgcactgatgaagg | 355487 | - | - |
| 23440 | POT1 | NM_015450.1 | 1201 | atgagggcgatttgatataatt | 353155 | - | - |
| 23441 | PP1057 | NM_031285.1 | 709 | gggttgtttatgcctatcaatg | 396491 | - | - |

Figure 46

| | | | | | | | |
|---|---|---|---|---|---|---|---|
| 23442 | PP1201 | NM_022152.3 | 459 | gagaaatgtggctgtctactacg | 382578 | - | |
| 23443 | PP2447 | NM_025204.2 | 879 | gcgcgacgtcacctaacctaca | 394411 | - | |
| 23444 | pp9099 | NM_025201.3 | 1293 | cagcccagggaacggctatatcg | 394356 | - | |
| 23445 | PPAN | NM_020230.2 | 1254 | aagatgatgacatcgagtatttc | 376304 | - | |
| 23446 | PPFIA3 | NM_003660.1 | 3267 | gttgaacgtcgaatgttagatca | 331953 | - | |
| 23447 | PPIL5 | NM_152329.2 | 645 | aggaactactaccatgaatctgc | 415689 | - | |
| 23448 | PPL13 | NM_020129.1 | 504 | atgtgcgtcacaaggaatacaag | 375617 | - | |
| 23449 | PPN | NM_022825.1 | 1182 | ggggtgcgagcctaaaacttgc | 384267 | - | see also 8480 line |
| 23450 | PPP1R12C | NM_017607.1 | 1060 | ctgagcagtcgcgagaagattc | 359413 | - | |
| 23451 | PPP1R14C | NM_030949.1 | 532 | gagctgctttctcggataagagg | 395669 | - | |
| 23452 | PPP1R14D | NM_017726.6 | 261 | ccgcctgacagtgaagtatgacc | 361162 | - | |
| 23453 | PPP1R15B | NM_032833.1 | 1598 | gaggcgcgaataagttagttga | 401836 | - | see also 9300 line |
| 23454 | PPP1R3B | NM_024607.1 | 683 | cagtacgtgaaggacacttatgc | 387827 | - | |
| 23455 | PR1 | NM_183240.1 | 118 | ctgtcctcggtctccatttgtga | 435635 | - | |
| 23456 | PRAM-1 | NM_032152.3 | 636 | cagcctgagttgagttacctttcc | 398400 | - | melanoma |
| 23457 | PRAME | NM_006115.2 | 1396 | gtgtgggatcacggatgatcagc | 334837 | - | |
| 23458 | PRDM11 | NM_020229.1 | 706 | gagagtgacgtgcgatgtgtaaa | 376275 | - | |
| 23459 | PRDM8 | NM_020226.1 | 1505 | tggtccaatcggccagagataag | 376260 | - | see also 7806 line |
| 23460 | PREI3 | NM_015387.1 | 573 | gtgtcatcggtttactaagtttg | 352173 | - | |
| 23461 | PRES | NM_198999.1 | 2237 | tggagacgtcggtatatatgtat | 440570 | - | see also 10354 line |
| 23462 | PRG1 | NM_002727.1 | 183 | aggaccaatgttcgaactacttc | 331026 | - | |
| 23463 | PRG1 | NM_014839.2 | 1044 | cgggctaacacaggataaactcagt | 344476 | - | see also 6044 line |
| 23464 | PRIC285 | NM_033405.2 | 2210 | gtggccgcaaggcttctactacc | 403788 | - | transcription_regulator |
| 23465 | PRICKLE1 | NM_153026.1 | 1855 | gtgttcgggattcgatggattct | 421941 | - | |
| 23466 | PRICKLE2 | NM_198859.1 | 1373 | aggtatcgaccaaggtcaaatga | 439969 | - | |
| 23467 | PRIMA1 | NM_178004.1 | 309 | gtggtcggggctcggtgatcatca | 430372 | - | |
| 23468 | PRKRIP1 | NM_024653.1 | 109 | acgctcgtcatccccaagaatgc | 388542 | - | |
| 23469 | PRND | NM_012409.2 | 151 | ggggcatcaagcacagaatcaag | 338138 | - | |
| 23470 | PRO0149 | NM_014117.2 | 719 | tggctatcagcgacgcaataagg | 339598 | - | |
| 23471 | PRO0628 | NM_014134.1 | 583 | ctgttaacacggcatttggtact | 339621 | - | |
| 23472 | PRO0971 | NM_018569.2 | 723 | aagatgacttcgaatcttgtttg | 372248 | - | |
| 23473 | PRO1580 | NM_018502.2 | 1119 | ggggcctttggctcctcaacaacc | 372047 | - | |
| 23474 | PRO1853 | NM_018607.4 | 589 | cagttacttcaaggatatgatat | 372317 | - | |
| 23475 | PRO1914 | NM_014106.1 | 1286 | cagatcacccccacctactatcc | 339585 | - | |
| 23476 | PRO2268 | NM_018520.2 | 214 | aagccaaacaggattagactta | 372110 | - | |
| 23477 | PRO2730 | NM_025222.2 | 1641 | ctgcatccctgctaatagtgttt | 394458 | - | |

Figure 46

| ID | Name | Accession | Length | Sequence | Num2 | | Notes |
|---|---|---|---|---|---|---|---|
| 23478 | PRO2964 | NM_018547.1 | 646 | ttggagaatactgttaattcagt | 372184 | - | - |
| 23479 | Prostein | NM_033102.1 | 1872 | tgggtctggtcgccatttacttt | 403255 | - | see also 9357 line |
| 23480 | PSARL | NM_018622.3 | 594 | cagcggacaatgatcagatatt | 372341 | - | - |
| 23481 | PSD | NM_002779.2 | 2288 | ctggatcactcgtcatcaatgtag | 331070 | - | signal_transduction |
| 23482 | PSFL | NM_031301.1 | 100 | ccgagccgttcgtatcatcttc | 396598 | - | - |
| 23483 | PSIP2 | NM_021144.2 | 364 | aggcagcaactaaacaatcaaat | 380534 | - | - |
| 23484 | PTD002 | NM_016144.2 | 265 | aggcagttgatcacaatgtgaag | 356467 | - | see also 6694 line |
| 23485 | PTD012 | NM_014039.2 | 658 | gttgtcattactggctaatcttt | 339360 | - | see also 5469 line |
| 23486 | PTF1A | NM_178161.1 | 32 | ggggcctagacgccttccttct | 430546 | - | - |
| 23487 | PTPNS1 | NM_080792.1 | 1255 | ttgcatgagcccgagaagaatgc | 406776 | - | - |
| 23488 | PTPNS1L2 | NM_178460.1 | 175 | gggagtcaatcatcttgagttgc | 430792 | - | - |
| 23489 | PTRF | NM_012232.1 | 424 | gaggacccacgctctatattgt | 337793 | - | - |
| 23490 | PTX1 | NM_016570.1 | 354 | ttgagcggatgtattggattta | 358162 | - | see also 6917 line |
| 23491 | PURB | NM_033224.2 | 363 | gcgcggtcaagagcgaattcttg | 403614 | - | see also 9382 line |
| 23492 | PVRL3 | NM_015480.1 | 764 | aggacatccgatactctttcata | 353456 | - | see also 6429 line |
| 23493 | PWDMP | NM_025132.2 | 3863 | atggtcaggagacccgatatatc | 393923 | - | see also 8867 line |
| 23494 | PYC1 | NM_152901.1 | 179 | gcgcggtcgggcagctagatatc | 421532 | - | - |
| 23495 | PYM | NM_032345.1 | 479 | gagcaggactcttgataaggtgt | 399857 | - | - |
| 23496 | R29124_1 | NM_033543.1 | 229 | cagctaatcgcagcatatgtaat | 404116 | - | - |
| 23497 | Rab11-FIP2 | NM_014904.1 | 738 | caggtgcaatcaatctcaatga | 345456 | - | - |
| 23498 | RAB15 | NM_198686.1 | 563 | atggcattgacttctatgaaaca | 439873 | - | see also 10348 line |
| 23499 | RAB3IL1 | NM_013401.2 | 1219 | aggacgcagagcccatgttctgg | 338905 | - | - |
| 23500 | RAB3IP | NM_022456.2 | 425 | gtgcggaaatatctagtatcagc | 382842 | - | - |
| 23501 | RABAC1 | NM_006423.1 | 244 | gagtactaccagagcaactatgt | 335539 | - | - |
| 23502 | RAD9B | NM_152442.1 | 980 | tggctttgagtattgatgatatg | 416851 | - | cell_cycle |
| 23503 | RAI14 | NM_015577.1 | 737 | cagacctaaaccttgtagattct | 354250 | - | - |
| 23504 | RAI16 | NM_022749.4 | 1575 | ctgattccgctttcaaactcc | 383896 | - | - |
| 23505 | RAM2 | NM_018719.2 | 588 | tagcgcaacgcttacagaatgaga | 372940 | - | - |
| 23506 | RAMP | NM_016448.1 | 828 | atgggatttacgtaagaattata | 357517 | - | see also 6861 line |
| 23507 | RANBP9 | NM_005493.2 | 1928 | tggacgagagctgcaagcaatga | 333905 | - | see also 3817 line |
| 23508 | RANGNRF | NM_014185.1 | 194 | ccgaccggtcccgacaatcaag | 339947 | - | - |
| 23509 | RAP140 | NM_015224.1 | 2323 | gtgggcactgaccataagctaca | 350039 | - | - |
| 23510 | raptor | NM_020761.1 | 3393 | acgagattggcgaccaaatatt | 378589 | - | see also 7946 line |
| 23511 | RARG-1 | NM_016167.3 | 403 | cagcttcacagactaacatcaag | 356507 | - | - |
| 23512 | RARRES3 | NM_004585.2 | 263 | gtggtgggaggctgttgctatcg | 332626 | - | - |

Figure 46

| 23513 | RB1CC1 | NM_014781.1 | 1377 | cagcaagatgaaactacgattga | 343291 | - | see also 5953 line | | |
|---|---|---|---|---|---|---|---|---|---|
| 23514 | RBPMS2 | NM_194272.1 | 406 | cggccgttcaaggggtatgaagg | 436173 | - | - | - | - |
| 23515 | RBT1 | NM_013368.2 | 450 | caggcatgtcctcatccataaca | 338846 | - | - | - | - |
| 23516 | RC3 | NM_015263.1 | 4452 | tggtactcgagattatactgaga | 350535 | - | - | - | - |
| 23517 | RCP | NM_025151.2 | 931 | ttggtggccttcggtctaagaat | 394080 | - | - | - | - |
| 23518 | REC14 | NM_025234.1 | 86 | cagtacggtattctcttcaaaca | 394493 | - | - | - | - |
| 23519 | RFPL1 | NM_021026.1 | 1086 | aagagtgtcttgagtatctgtcc | 380368 | - | - | - | - |
| 23520 | RGMA | NM_020211.1 | 831 | tggccgctcatcgacaataatta | 376157 | - | - | - | - |
| 23521 | RGPR | NM_033127.1 | 1714 | ctgtatcagcgtgcgattgttgc | 403524 | - | see also 9373 line | | |
| 23522 | RhoGAP2 | NM_021226.2 | 2196 | acgaatgtcccggttagaagaag | 380860 | - | - | - | - |
| 23523 | RIC3 | NM_024557.2 | 1044 | gtggtcccaagactttaaagatg | 387259 | - | - | - | - |
| 23524 | RIMS4 | NM_182970.2 | 523 | aagtcgctggacccactgtataa | 435499 | - | - | - | - |
| 23525 | RIPX | NM_014961.1 | 1450 | caggatcattaccttacaagaag | 346369 | - | see also 6152 line | | |
| 23526 | RIS1 | NM_015444.1 | 321 | tcggggtgccctccaatgcttca | 352988 | - | - | - | - |
| 23527 | RNF10 | NM_014868.3 | 1320 | ttgttggtgataccattacgatg | 345031 | - | - | - | - |
| 23528 | RNF111 | NM_017610.6 | 1636 | aagctactagcgcttccattaac | 359472 | - | - | - | - |
| 23529 | RNF121 | NM_018320.3 | 662 | gagtcgggcatgcctaccaaaca | 370297 | - | see also 7403 line | | |
| 23530 | RNF122 | NM_024787.2 | 606 | gagtgagcgatacggatataagg | 390669 | - | see also 8747 line | | |
| 23531 | RNF123 | NM_022064.2 | 719 | ctgcctgaacggtgtatcactgg | 381791 | - | - | - | - |
| 23532 | RNF125 | NM_017831.2 | 901 | ccgtttaatacccgatgagaatc | 362880 | - | - | - | - |
| 23533 | RNF135 | NM_032322.3 | 1160 | aggactatggactcttgttgtgt | 399720 | - | - | - | - |
| 23534 | RNF141 | NM_016422.3 | 238 | gagagtggctccttaacttatga | 357416 | - | see also 6848 line | | |
| 23535 | RNF146 | NM_030963.2 | 989 | ctgacggagcggacagtgtatca | 395888 | - | see also 8983 line | | |
| 23536 | RNF148 | NM_198085.1 | 666 | tagtcgcggtgatgataagcaac | 437020 | - | - | - | - |
| 23537 | RNF152 | NM_173557.1 | 961 | cggcatcgtgcttcacaacatgt | 426065 | - | - | - | - |
| 23538 | RNF17 | NM_031277.1 | 999 | aaggaactttcttgttacgatac | 396406 | - | - | - | - |
| 23539 | RNF2 | NM_007212.2 | 380 | atgtgcccaatttgtttggatat | 337082 | - | see also 5019 line | | |
| 23540 | RNF20 | NM_019592.5 | 1648 | ctgcagtcccagtctagtactga | 375413 | - | see also 7717 line | | |
| 23541 | RNF24 | NM_007219.2 | 255 | gagctcggatttcccacattaca | 337232 | - | see also 5032 line | | |
| 23542 | RNF3 | NM_006315.3 | 1029 | ttgtcactaggtggagattcaag | 335209 | - | see also 4420 line | | |
| 23543 | RNF31 | NM_017999.3 | 485 | tggcgtggtgtcaagtttaataa | 365193 | - | - | - | - |
| 23544 | RNF32 | NM_030936.2 | 696 | cagtatcaaacccgagtgataca | 395590 | - | - | - | - |
| 23545 | RNF36 | NM_080745.2 | 735 | gcgtctggcatggtgacattaag | 406734 | - | - | - | - |
| 23546 | RNF40 | NM_014771.2 | 1570 | atgcgccacctgattagtagtct | 343198 | - | - | - | - |
| 23547 | RNF41 | NM_005785.2 | 749 | atgagctgcccaaccataactgc | 334259 | - | see also 4044 line | | |
| 23548 | RNF44 | NM_014901.3 | 749 | gagcccgttcatggttgatctcc | 345443 | - | see also 6084 line | | |
| 23549 | RNF8 | NM_003958.2 | 416 | gagcgcgtctggaacctttaagg | 332304 | - | - | - | - |

Figure 46

| | | | | | | | |
|---|---|---|---|---|---|---|---|
| 23550 | ROBO4 | NM_019055.4 | 1014 | ggggccaagactacgagttcaaag | 374785 | - | see also 7684 line |
| 23551 | RP4-622L5 | NM_019118.2 | 372 | gtgctcaccgtgataggaatttc | 375303 | - | - |
| 23552 | RPC2 | NM_018082.2 | 2350 | ttgggcgttgccttgtatataa | 366462 | - | transcription_regulator |
| 23553 | RPGRIP1 | NM_020366.2 | 1675 | gagaggttgactgactactaga | 376932 | - | see also 7849 line |
| 23554 | Rpp25 | NM_017793.1 | 1222 | tagtctcagcgtacttaagaacg | 362455 | - | - |
| 23555 | RSHL2 | NM_031924.3 | 419 | gggagcgagcccagatagctcc | 397353 | - | - |
| 23556 | RTBDN | NM_031429.1 | 660 | gagccctgaattgcgaatccttcc | 396948 | - | RAS small monomeric GTPase |
| 23557 | RTKN2 | NM_145307.2 | 1031 | ttgcgaggagtaaactctattg | 414311 | - | signal transduction |
| 23558 | RTP801 | NM_019058.1 | 569 | aagccaggtgggcaaagaactac | 374801 | - | - |
| 23559 | RTTN | NM_173630.2 | 3757 | atgctcggaacttctttacgttt | 426813 | - | see also 10092 line |
| 23560 | RWDD3 | NM_015485.2 | 141 | cagatggaccgtgttcagaatt | 353491 | - | - |
| 23561 | RYD5 | NM_145651.1 | 161 | ggggaccttggcaagtacaatg | 414630 | - | - |
| 23562 | SACM1L | NM_014016.1 | 1214 | aaggcgtttccgaagcaattgc | 339184 | - | see also 5456 line |
| 23563 | SAGE | NM_018666.1 | 1543 | atgttctatccgggcttattaat | 372421 | - | - |
| 23564 | SAMD3 | NM_152552.1 | 552 | caggccgacagactaagtatct | 418045 | - | - |
| 23565 | SAMD4 | NM_015589.2 | 947 | cagttggactgtgtttaacagacc | 354350 | - | - |
| 23566 | SAMD6 | NM_173551.1 | 735 | atgaactgactggaatccttaag | 425960 | - | - |
| 23567 | SAMD7 | NM_182610.1 | 534 | atgattcaacggcatcatactgc | 434637 | - | - |
| 23568 | SAMD8 | NM_144660.1 | 447 | ccggaccataactgacttgaatt | 411090 | - | see also 9727 line |
| 23569 | SAMSN1 | NM_022136.2 | 678 | atgccaccccatatagaaaacagt | 382440 | - | see also 8300 line |
| 23570 | SAP130 | NM_024545.2 | 1661 | ggtgtccactatccgacagtatcc | 386974 | - | see also 8636 line |
| 23571 | SAS10 | NM_020368.1 | 1509 | caggttcgtgaagttcgtaaaga | 377034 | - | - |
| 23572 | SCAMP-4 | NM_079834.1 | 480 | ctggctgcggcaattggattct | 406367 | - | see also 9510 line |
| 23573 | SCAMP5 | NM_138967.1 | 419 | gcgtctgcggctggattgctacc | 409322 | - | - |
| 23574 | SCAPIN1 | NM_080672.2 | 1283 | aggcgctgaacgactccattatt | 406635 | - | phosphatase |
| 23575 | SCDR10 | NM_198533.1 | 1042 | gtggccacagcccaagatgaagt | 439262 | - | - |
| 23576 | SCG3 | NM_013243.2 | 442 | gtgttagttgctccgattcaagc | 338373 | - | see also 5359 line |
| 23577 | SCGB3A2 | NM_054023.2 | 125 | gtgaccatcagcctttgtagtta | 405969 | - | - |
| 23578 | SCML4 | NM_198081.1 | 499 | gtgtcagtctcggcttcctttga | 436994 | - | - |
| 23579 | SCN4B | NM_174934.1 | 258 | ggggaaaggccaccgacatctacg | 428723 | - | channel |
| 23580 | SCNM1 | NM_024041.1 | 633 | aagacgagccctggaccattatc | 385615 | - | - |
| 23581 | SCOC | NM_032547.1 | 326 | aagacttaatacaagtgttgg | 400644 | - | - |
| 23582 | SDBCAG84 | NM_015966.2 | 309 | ttgtgcctactgagtattgatg | 355658 | | - |
| 23583 | SDCCAG1 | NM_004713.2 | 683 | tggaattctcgggtaaftgtcaaag | 332769 | - | see also 3231 line |

914

Figure 46

| 23584 | SDCCAG16 | NM_006649.2 | 1397 | ctgtctgaattgagagtactatc | 336038 | - | - | - | - |
|--------|----------|-------------|------|-------------------------|--------|---|---|---|---|
| 23585 | SDCCAG8 | NM_006642.1 | 739 | aggaacgacttagctgaatatca | 335998 | - | - | - | - |
| 23586 | SDK1 | NM_152744.2 | 2336 | ctgactccggctcgtacctatca | 420357 | - | see also 9941 line | | |
| 23587 | SDK2 | NM_019064.2 | 5016 | caggcggctctccgtcaagaact | 374876 | - | - | - | - |
| 23588 | SE57-1 | NM_025214.1 | 243 | gagtctacgtccgctcacattat | 394424 | - | - | - | - |
| 23589 | SEC13L | NM_031216.2 | 647 | atgatcgccgtaggaagtgatga | 395990 | - | see also 8992 line | | |
| 23590 | SEC31L2 | NM_015490.3 | 375 | cggggacaatggcatgcttattc | 353514 | - | - | - | - |
| 23591 | SELH | NM_170746.1 | 334 | tggaggaggcgaccgttgttatc | 424154 | - | - | - | - |
| 23592 | SELM | NM_080430.1 | 274 | aggacattccattctatcacaac | 406378 | - | - | - | - |
| 23593 | SELV | NM_182704.1 | 1108 | gagcgttatcgatgaggaaatca | 434921 | - | - | - | - |
| 23594 | SEMA3D | NM_152754.1 | 508 | atggccagtacgatgtaatgttt | 420613 | - | see also 9948 line | | |
| 23595 | SES1 | NM_014766.2 | 1335 | gtgttgacacggagattaagttc | 343182 | - | - | DNA binding | - |
| 23596 | Ses2 | NM_138355.1 | 503 | ctgcgccattctcctaccatagc | 407569 | - | - | - | - |
| 23597 | SES3 | NM_024583.2 | 234 | gagaacgtcttaagtgtacatat | 387580 | - | - | - | - |
| 23598 | SETMAR | NM_006515.1 | 138 | cagtacactcctgatcatgtagt | 335698 | - | see also 4558 line | | |
| 23599 | SEZ6 | NM_178860.2 | 1409 | ggggtagtgcccgcttccattgt | 432033 | - | see also 10223 line | | |
| 23600 | SFMBT1 | NM_016329.1 | 2280 | gtgggcatagtaacttagcttgt | 357020 | - | see also 6811 line | | |
| 23601 | SH2BP1 | NM_014633.1 | 884 | tagcaaccctatggtattgaacc | 341485 | - | see also 5801 line | | |
| 23602 | SH3GLB1 | NM_016009.2 | 338 | aagctccaagtcgtataaacaac | 355829 | - | see also 6608 line | | |
| 23603 | SH3MD1 | NM_014631.1 | 1673 | gagggcttccggccatatgcaga | 341445 | - | - | - | - |
| 23604 | SH3MD2 | NM_020870.1 | 288 | gggatcgtaggttctcgaaatga | 379721 | - | - | - | - |
| 23605 | SH3YL1 | NM_015677.1 | 825 | aagatatccgagcttatgacatt | 354966 | - | - | - | - |
| 23606 | SIP | NM_014412.1 | 733 | atgaagcgaaccattaataaagc | 340557 | - | see also 5666 line | | |
| 23607 | SIPL | NM_018269.1 | 548 | cgggcagtacgtgaaatttctgg | 369600 | - | - | - | - |
| 23608 | SITPEC | NM_016581.2 | 1097 | ctgggacaactacgagtttgaca | 358225 | - | - | - | - |
| 23609 | SLAC2-B | NM_015065.1 | 5625 | atgggtacagtcgaagattcaga | 347715 | - | see also 6219 line | | |
| 23610 | SLAMF9 | NM_033438.1 | 743 | atgcagatcctaactatgcttct | 404002 | - | - | - | - |
| 23611 | SLC16A10 | NM_018593.2 | 1213 | tggccggattgcagattatgtgc | 372294 | - | - | - | - |
| 23612 | SLC16A11 | NM_153357.1 | 791 | aggcaccctctcgcgttacttct | 423043 | - | - | - | - |
| 23613 | SLC27A1 | NM_198580.1 | 979 | cagtcgtcctccgcaagaaattc | 439733 | - | - | - | - |
| 23614 | SLC35A4 | NM_080670.2 | 1553 | cagcatcacacgcctctttgtgg | 406627 | - | - | - | - |
| 23615 | SLC35B3 | NM_015948.2 | 1186 | ttgacatgcactagtggattagg | 355583 | - | see also 6587 line | | |
| 23616 | SLC35E1 | NM_024881.3 | 998 | tgggggtcttcctctataacaag | 391640 | - | - | - | - |
| 23617 | SLC35E3 | NM_018656.1 | 585 | cagctcacgctgattcctataac | 372360 | - | - | - | - |
| 23618 | SLC35F3 | NM_173508.1 | 533 | gggcgcaactcaagaagatcttc | 425207 | - | see also 10075 line | | |
| 23619 | SLC35F5 | NM_025181.2 | 1351 | aggcgttgtactggtaaacctgg | 394277 | - | - | - | - |

Figure 46

| | | | | | | | | |
|---|---|---|---|---|---|---|---|---|
| 23620 | SLC36A2 | NM_181776.1 | 131 | gggagccgttgccatcaaattgg | 433163 | - | | |
| 23621 | SLC36A3 | NM_181774.1 | 356 | ctggactatcgatgatgcaaact | 433126 | - | | |
| 23622 | SLC37A2 | NM_198277.1 | 1463 | ttgccggttagtatacaaagaga | 437454 | - | | |
| 23623 | SLC43A1 | NM_003627.3 | 1311 | gggacgggttgctaccaaatcc | 331806 | - | | prostate cancer |
| 23624 | SLC4A9 | NM_031467.1 | 1155 | gtgcttctcggccgtactctaca | 397152 | - | | |
| 23625 | SLIC1 | NM_153337.1 | 233 | aggacctgacgggcacttagaca | 422784 | - | | |
| 23626 | SMC4L1 | NM_005496.2 | 1006 | tgttgtggacggctaaatgaac | 333942 | - | see also 3826 line | |
| 23627 | SMCR5 | NM_144774.1 | 453 | atgcgaaggtgtttaagagtaaa | 411900 | - | | |
| 23628 | SMCR7 | NM_139162.2 | 323 | ctggccgtgaagcggttcattga | 409359 | - | | |
| 23629 | SMCR8 | NM_144775.1 | 2361 | tggctagccgggacatcagtaag | 411934 | - | | |
| 23630 | SMT3H1 | NM_006936.1 | 116 | gagggtgtgaagacagagaatga | 336618 | - | | |
| 23631 | SMU-1 | NM_018225.1 | 71 | ccgccttattatgcagttacttga | 368586 | - | | |
| 23632 | SMYD2 | NM_020197.1 | 774 | atgaccgttaagagattcttat | 376085 | - | | |
| 23633 | SMYD3 | NM_022743.1 | 382 | caggcaactcgttaatgacatttc | 383834 | - | see also 8451 line | |
| 23634 | SNIP1 | NM_024700.2 | 977 | cagcatgcggtctttcaatatcg | 389407 | - | | |
| 23635 | SOAT | NM_197965.1 | 917 | cagcatatcagacgtacaagagg | 436940 | - | | |
| 23636 | SOC | NM_145345.1 | 150 | aggcgaggaatccgcatctatgg | 41431 | - | | |
| 23637 | SOM | NM_021180.2 | 1109 | gtgttcatcgggtaaactgtct | 380654 | - | | |
| 23638 | SOSTDC1 | NM_015464.1 | 316 | cagcaacagcacgttgaatcaag | 353320 | - | | |
| 23639 | SP192 | NM_021639.3 | 1830 | cagtccaatctcgttaccaaac | 380991 | - | see also 8159 line | |
| 23640 | SPACA1 | NM_030960.2 | 282 | caggcgagccgcggagaactacg | 395691 | - | | |
| 23641 | SPAG5 | NM_006461.1 | 2594 | aggacactgtagagaacctaacg | 335635 | - | cell_cycle | |
| 23642 | SPATA1 | NM_022354.1 | 619 | tagatgagcggcagactaataat | 382653 | - | | |
| 23643 | SPATA11 | NM_032306.2 | 892 | gggctcagccgttatgacttct | 399614 | - | | |
| 23644 | SPATA12 | NM_181727.1 | 1150 | gggtgcagccgttcaaaccaact | 433058 | - | see also 7694 line | |
| 23645 | SPATA6 | NM_019073.1 | 355 | gtgttcccgacgcagtagatcc | 374929 | - | | |
| 23646 | SPATA7 | NM_018418.1 | 1325 | acgactgttgagcggacatataa | 371327 | - | | |
| 23647 | SPATA9 | NM_031952.1 | 454 | gcgccgaggcaacccctatataa | 397478 | - | | |
| 23648 | SPATS1 | NM_145026.2 | 959 | aggcgacaatccatatatgtacc | 412855 | - | | |
| 23649 | SPATS2 | NM_023071.1 | 1147 | acggtatcgagttagttaag | 385060 | - | | |
| 23650 | SPG20 | NM_015087.3 | 806 | gaggttagtgcaccttcgtatcc | 347963 | - | see also 6226 line | |
| 23651 | SPG6 | NM_144599.2 | 1488 | caggctccgtcgtgctgattatc | 410209 | - | | |
| 23652 | SPON1 | NM_006108.1 | 1133 | gttggacagaacggccatttaat | 334801 | - | | |
| 23653 | SPRED1 | NM_152594.1 | 1300 | aagttctgcttgcgatggttagc | 418562 | - | see also 9890 line | |
| 23654 | SPRED2 | NM_181784.1 | 310 | cagtccgcgtcgggtctgtaagg | 433300 | - | signal_transduction | |
| 23655 | SPRR3 | NM_005416.1 | 527 | gagccaggtgccatcaaagttcc | 333755 | - | | structural molecule |
| 23656 | SPRR4 | NM_173080.1 | 145 | cagccacccctgttaatgtca | 424599 | - | | |

Figure 46

| | | | | | | | |
|---|---|---|---|---|---|---|---|
| 23657 | SPUF | NM_013349.3 | 182 | aggaagatcagcccatcctactg | 338809 | - | - |
| 23658 | SPY1 | NM_182756.1 | 987 | ctgtcaatggacataataggtga | 435006 | - | - |
| 23659 | SR-A1 | NM_021228.1 | 2588 | gggctccattggcgtcaaattca | 380871 | - | - |
| 23660 | SRRM2 | NM_016333.2 | 6354 | aagtcgtcaccacttgctatcc | 357192 | see also 6815 line | - |
| 23661 | SS18L2 | NM_016305.1 | 131 | gggcaaggcggaagtcaatcaag | 356932 | - | renal cell carcinoma |
| 23662 | SSTK-IP | NM_144627.1 | 366 | atggagcggcacactagtcatcc | 410510 | - | - |
| 23663 | SSTR4 | NM_001052.1 | 1021 | gaggagccctggactactatgc | 330140 | gpcr, receptor_acitivit y | somatostatin receptor |
| 23664 | SSX2IP | NM_014021.1 | 828 | aagtcgagctactcagtataatc | 339230 | - | - |
| 23665 | SSX6 | NM_173357.2 | 352 | cagactctcagggaatgattct | 424819 | - | - |
| 23666 | ST5 | NM_005418.2 | 2763 | gcgccagctcatccgaatctttg | 333778 | see also 3786 line | - |
| 23667 | ST7 | NM_018412.2 | 663 | aggacgagagcctcttacttact | 371258 | see also 7450 line | - |
| 23668 | STAG2 | NM_006603.3 | 756 | gtgttagttacggcaatgtcaata | 335845 | see also 4654 line | - |
| 23669 | STARD10 | NM_006645.1 | 895 | atgggcgtgattacatcattat | 336031 | see also 4698 line | - |
| 23670 | STARD7 | NM_020151.2 | 1477 | tggcctgctcgattgagtatg | 375722 | see also 7769 line | - |
| 23671 | STARS | NM_139166.2 | 879 | atgtcaccgcgcctacacaaagg | 409375 | - | - |
| 23672 | STATIP1 | NM_018255.1 | 1911 | gagatcgaactggtcattgtgg | 369219 | - | - |
| 23673 | STEPP | NM_152990.2 | 70 | atggttcgagaggatcttcaaca | 421691 | - | - |
| 23674 | ST2 | NM_145755.1 | 2437 | cagaattgggcacgcttagtga | 414897 | - | - |
| 23675 | STK11IP | NM_052902.1 | 447 | atggcctccgaggcatctactcc | 405143 | - | - |
| 23676 | STK22B | NM_053006.3 | 368 | ctgacggacggatctacatcatc | 405774 | kinase_related | - |
| 23677 | STRAIT114 99 | NM_021242.3 | 895 | tcgctctttaacgccatgaatcg | 380880 | see also 8143 line | - |
| 23678 | STRC | NM_153700.2 | 243 | ccgctcacggttcttacattcc | 423664 | see also 10008 line | - |
| 23679 | STX1B2 | NM_052874.1 | 682 | ccgcgagctgcacgatatgtttg | 404573 | - | - |
| 23680 | STXBP4 | NM_178509.3 | 599 | cagtactgtgggtttgtctaata | 430961 | - | - |
| 23681 | STXBP5 | NM_139244.2 | 723 | ttggtcgtccaggagtagaatgt | 409465 | - | - |
| 23682 | STXBP6 | NM_014178.5 | 458 | gaggtcaaggcgaatatttaact | 339891 | - | - |
| 23683 | SUHW1 | NM_080740.1 | 319 | gagggggatcgacagagtagtcc | 406692 | transcription_regulator | - |
| 23684 | SULT1C2 | NM_006588.2 | 1103 | atggcaaactattcatcgattcc | 335833 | - | sulfotransferase |
| 23685 | SUMF1 | NM_182760.2 | 274 | gcggcaactcgcgcactccaaaga | 435118 | - | - |
| 23686 | SVH | NM_031905.2 | 630 | cagcctgaagacttaactgatgg | 397245 | - | - |
| 23687 | SYAP1 | NM_032796.2 | 561 | tgccgtgcaatttaatttcgact | 401532 | - | - |
| 23688 | SYCP3 | NM_153694.2 | 127 | ctgtggaagatcagtttacgaga | 423552 | - | - |
| 23689 | SYN1 | NM_006950.2 | 604 | tggtggattctcgtcgtggatatgg | 336624 | see also 4865 line | - |

917

Figure 46

| 23690 | SYNCOILIN | NM_030786.1 | 428 | aagaacgccagaggcagttaaga | 394995 | - | - | - | - |
|---|---|---|---|---|---|---|---|---|---|
| 23691 | SYNE1 | NM_015293.1 | 7894 | agggagaaccgcactgattcagc | 351285 | - | - | - | - |
| 23692 | SYNE2 | NM_015180.3 | 20312 | gtgtcggagggaactaatgcaac | 349444 | - | see also 6270 line | | |
| 23693 | SYNPO | NM_007286.3 | 2778 | ctggcgcgaaacatcatcaatgc | 337410 | - | - | - | - |
| 23694 | SYNPO2L | NM_024875.1 | 382 | cagcctcaccaatcaatctgact | 391613 | - | see also 8800 line | | |
| 23695 | SYT10 | NM_198992.1 | 1520 | atgtgtgaaggtcgaagattaaa | 440375 | - | see also 10352 line | | |
| 23696 | SYT5 | NM_003180.1 | 1007 | cagacgctgaaccctcactttgg | 331241 | - | - | transporter | - |
| 23697 | SYTL2 | NM_032379.2 | 1431 | ctgctaaggggaagtcatctaaa | 400089 | - | see also 9177 line | | |
| 23698 | T1 | NM_031466.3 | 2955 | tagaagtcgagccgtctgtattt | 397140 | - | - | - | - |
| 23699 | T2BP | NM_052864.2 | 628 | atggtcagattcggagagtatca | 404505 | - | - | - | - |
| 23700 | T3JAM | NM_025228.1 | 1384 | gagacctgtgcagcttggatacc | 394478 | - | see also 8885 line | | |
| 23701 | TA-LRRP | NM_015350.1 | 633 | gtgggacgtcttctggtattaca | 351757 | - | - | - | - |
| 23702 | TA-NFKBH | NM_032721.2 | 696 | gtgtaccggcgtcttgacattcg | 401254 | - | - | - | - |
| 23703 | TAB3 | NM_152787.2 | 1100 | atgcacatacctcggtatagtac | 421238 | - | - | - | - |
| 23704 | TAF1L | NM_153809.1 | 601 | aggaccaagatgctattacctgt | 423896 | - | transcription_regulator | - | |
| 23705 | TAF7L | NM_024885.1 | 832 | aagtacccgttgggaagtcattg | 391705 | - | transcription_regulator | - | |
| 23706 | TAFA2 | NM_178539.2 | 1166 | acgcgagctgctccatcatgtgt | 431152 | - | - | - | - |
| 23707 | TAGAP | NM_054114.3 | 401 | atgtcaatcagagggtgatatca | 406065 | - | - | - | - |
| 23708 | TAPBP-R | NM_018009.3 | 428 | aagatgacccacctattatcttt | 365242 | - | receptor_acitivity | - | - |
| 23709 | TARSH | NM_015429.2 | 1743 | aaggtgccccagcgtgttactgc | 352696 | - | see also 6413 line | | |
| 23710 | TBC1D4 | NM_014832.1 | 2239 | ccgactccccgcagtttcgaaga | 344344 | - | see also 6041 line | | |
| 23711 | TBC1D5 | NM_014744.1 | 2274 | cagggcatgcacgaactgttagc | 342936 | - | see also 5940 line | | |
| 23712 | TBC1D7 | NM_016495.2 | 530 | aagatagtgtcgactgttactgg | 357869 | - | - | - | - |
| 23713 | TBL1Y | NM_033284.1 | 1332 | gggcacgacgtcccaagtaataa | 403658 | - | transcription_regulator | - | |
| 23714 | TBN | NM_138572.1 | 330 | gtggacactctccctgcttatgc | 408488 | - | - | - | - |
| 23715 | TCBA1 | NM_153355.2 | 504 | cagaagaccatcgctacatcacg | 423027 | - | - | - | - |
| 23716 | TCEB3L | NM_016427.1 | 840 | ttggcactcccctactttgatca | 357436 | - | transcription_regulator | - | |
| 23717 | TCERG1L | NM_174937.1 | 605 | ctggactcaacgtggatacatcc | 428735 | - | - | - | - |
| 23718 | TCTE3 | NM_174910.1 | 606 | cagctgggtcgcagctaaacacg | 428646 | - | - | - | - |
| 23719 | TD-60 | NM_018715.1 | 1123 | ccgggcacagcggatagagtacg | 372826 | - | - | - | - |
| 23720 | TEAD1 | NM_021961.2 | 1221 | gtggacattcgtcagatttatga | 381654 | - | see also 8217 line | | |
| 23721 | TEB4 | NM_005885.2 | 2835 | gggtcaacgactcgtgaactacg | 334438 | - | see also 4093 line | | |

EP 1 752 536 A1

Figure 46

| | | | | | | | | | |
|---|---|---|---|---|---|---|---|---|---|
| 23722 | TECTB | NM_058222.1 | 968 | tggggatttgtgccgtgttatag | 406244 | - | see also 9502 line | | |
| 23723 | TES | NM_015641.2 | 1298 | cagcgggtgacctataacaattt | 354846 | - | - | - | - |
| 23724 | TESSP1 | NM_183379.1 | 135 | ctgcggccaccgggaaattcacg | 435824 | - | - | - | - |
| 23725 | TESSP2 | NM_182702.1 | 395 | tgggagatcggagtgtctataat | 434883 | - | - | - | - |
| 23726 | TEX11 | NM_031276.1 | 1249 | atgccctacaatggtactattat | 396324 | - | - | - | - |
| 23727 | TEX13A | NM_031274.2 | 918 | cggtcgaaacagccacatcttat | 396264 | - | - | - | - |
| 23728 | TEX13B | NM_031273.1 | 760 | cagcccctcggagctattgtagc | 396259 | - | - | - | - |
| 23729 | TEX15 | NM_031271.2 | 550 | gagtccggtaatgcttatacaaa | 396025 | - | see also 8996 line | | |
| 23730 | TEX261 | NM_144582.1 | 345 | atgtcgtctccaattatttcacc | 409973 | - | - | - | - |
| 23731 | TFB2M | NM_022366.1 | 210 | aagcggcgacgcgaaagcatttg | 382668 | - | - | - | - |
| 23732 | TFPT | NM_013342.1 | 358 | gcgttgcctcccctatttggtgg | 338776 | - | - | - | - |
| 23733 | TGM6 | NM_198994.1 | 937 | acgggtcgtgtccaacttcaact | 440394 | - | - | - | - |
| 23734 | TGS | NM_173083.2 | 1683 | ttgttcggcatgcaaattcctca | 424763 | - | see also 10061 line | | |
| 23735 | TH1L | NM_016397.2 | 1537 | ctgagtcgaggttatgtacttcc | 357361 | - | see also 6847 line | | |
| 23736 | THAP10 | NM_020147.2 | 558 | ctgcagcttcacagattacgtgt | 375692 | - | - | - | - |
| 23737 | THAP4 | NM_015963.3 | 717 | aggatctggggcgtgcaaattta | 355642 | - | - | - | - |
| 23738 | THAP9 | NM_024672.2 | 162 | tggatcagggctgttaatcgtgt | 388830 | - | - | - | - |
| 23739 | THOC3 | NM_032361.1 | 913 | gcgtcagcatcggaagatcattt | 399991 | - | see also 9174 line | | |
| 23740 | THSD1 | NM_018676.2 | 523 | ctggtgactactggttcacaatg | 372496 | - | - | - | - |
| 23741 | THSD2 | NM_032784.2 | 379 | atgcatcctaacgttagtcaagg | 401491 | - | see also 9282 line | | |
| 23742 | THY28 | NM_014174.2 | 978 | ttgttcggatgatgaaacgtttc | 339857 | - | see also 5524 line | | |
| 23743 | TIC | NM_012455.1 | 982 | ggggacggcgctgctatcagtgg | 338222 | - | - | - | - |
| 23744 | TIGA1 | NM_053000.1 | 560 | cggggagcttcgtcgatttgtgt | 405592 | - | - | - | - |
| 23745 | TIGD2 | NM_145715.1 | 1677 | aggaggcttcggaccataataag | 414757 | - | - | - | - |
| 23746 | TIGD3 | NM_145719.1 | 530 | ccgctggaaacgccgaaacaacg | 414763 | - | - | - | - |
| 23747 | TIP120A | NM_018448.2 | 3113 | gtggtgggccttaaaccatatgt | 371671 | - | transcription_regulator | - | - |
| 23748 | TJP1 | NM_003257.2 | 1263 | atgaacgggctacgctattgaat | 331326 | - | see also 2201 line | | |
| 23749 | TJP3 | NM_014428.1 | 2785 | aagtttatgcgagtacatgatgc | 340658 | - | - | - | - |
| 23750 | TJP4 | NM_080604.1 | 1518 | cagcccagcacccctaacactca | 406399 | - | - | - | - |
| 23751 | TLK2 | NM_006852.1 | 1985 | aggtgctggtacttattggtatt | 336481 | - | see also 4796 line | | |
| 23752 | TM4SF10 | NM_031442.2 | 574 | gggatctcgaaggcgtttctata | 397013 | - | see also 9030 line | | |
| 23753 | TMC3 | NM_181841.1 | 365 | ttgctcgtctcgcctgtaacttt | 433744 | - | - | - | - |
| 23754 | TMC4 | NM_144686.1 | 495 | ccggcacggagtcctacttctcc | 411309 | - | - | - | - |
| 23755 | TMC5 | NM_024780.3 | 2160 | tggatcgacaccctaagtacacg | 390615 | - | see also 8744 line | | |
| 23756 | TMC7 | NM_024847.2 | 498 | gaggacatccgcagcatagaagg | 391370 | - | see also 8783 line | | |
| 23757 | TMEM13 | NM_181744.1 | 382 | ctgtcagcctggatcgatatttg | 433073 | - | see also 10251 line | | |

Figure 46

| 23758 | TMEM16C | NM_031418.1 | 1214 | atgaccgatctcgtctcattaat | 396771 | - | - | - | |
| 23759 | TMEM16D | NM_178826.2 | 1870 | gtgggcgttaatcaggaataact | 431824 | - | see also 10214 line | | |
| 23760 | TMPIT | NM_031925.1 | 247 | tggccctcgccctgaagaaatgc | 397386 | - | - | - | |
| 23761 | TNIP2 | NM_024309.2 | 331 | aggctgactgagcgactagaaga | 386216 | - | - | - | |
| 23762 | TNIP3 | NM_024873.2 | 850 | tcggatcgagagagacttaatca | 391603 | - | - | - | |
| 23763 | TNRC5 | NM_006586.2 | 661 | tcggacttgcggttaatcgaagt | 335814 | - | - | - | |
| 23764 | TNT | NM_182831.1 | 482 | cagagcagcgagagtcatgtttc | 435373 | - | - | - | |
| 23765 | TOR2A | NM_130459.1 | 559 | gggtggtatacgggaccaattac | 406915 | - | - | - | |
| 23766 | TOR3A | NM_022371.2 | 866 | caggggcgatataatcaatgagg | 382742 | - | - | - | |
| 23767 | TP53INP1 | NM_033285.2 | 674 | aggtggattaaccactatcaagg | 403670 | - | see also 9396 line | | |
| 23768 | TP53TG3 | NM_016212.1 | 318 | atgttcgaagtttgataccttgc | 351860 | - | - | - | |
| 23769 | TRAG3 | NM_004909.1 | 218 | aggaattctaccacaagactaac | 333243 | - | - | - | |
| 23770 | TRALPUSH | NM_053002.2 | 2211 | cagcgcacaatccttctctatgg | 405665 | - | see also 9475 line | | |
| 23771 | TRAP25 | NM_080651.1 | 415 | cagtcgagcaacttattccatat | 406516 | - | receptor_acitivity、transcription_regulator、signal_transduction | - | |
| 23772 | TRAPPC6B | NM_177452.2 | 435 | ttggactacggtattcaagaaac | 429873 | - | - | receptor | - |
| 23773 | TREM4 | NM_145273.2 | 298 | aggagctctcgctcattgtgacc | 414076 | - | receptor_acitivity | - | |
| 23774 | TREML1 | NM_178174.2 | 262 | cggggcaggcgtacgtttctcaca | 430605 | - | - | - | |
| 23775 | TRF3 | NM_199047.1 | 191 | ttgtaccttatgatatgtacata | 440613 | - | - | - | |
| 23776 | TRIF | NM_014261.1 | 579 | cagacgccactccaactttctgt | 339992 | - | receptor_acitivity、kinase_related | - | |
| 23777 | TRIK | NM_181840.1 | 266 | aggtgaagcctcagtggtttaac | 433725 | - | channel | - | |
| 23778 | TRIP | NM_005879.2 | 1096 | cagctcccagcatggttactacg | 334338 | - | - | - | |
| 23779 | TRIPIN | NM_152524.1 | 549 | ttgatgcgtcttccatttgcaag | 417578 | - | - | - | |
| 23780 | TSARG1 | NM_139073.2 | 400 | aggaaagcagggcctctgattcg | 409327 | - | - | - | |
| 23781 | TSARG2 | NM_144644.2 | 716 | atgctgaccaatgaacttaaagc | 410698 | - | - | - | |
| 23782 | TSARG6 | NM_153614.2 | 778 | cgggatcactcgcaattcagagg | 423401 | - | - | - | |
| 23783 | TSGA13 | NM_052933.2 | 1128 | cagcgagtgaaaggccaatttcc | 405389 | - | - | - | |
| 23784 | TSGA14 | NM_018718.1 | 570 | ctgctagatgtgcgtgatagaga | 372906 | - | - | - | |
| 23785 | TSLL2 | NM_145296.1 | 919 | ttgcgaggcgtccaataagcacg | 414262 | - | - | - | |
| 23786 | TSLP | NM_033035.2 | 468 | gcgcgtcgctcgccaaagaaatg | 402665 | - | - | - | |
| 23787 | TSLRP | NM_012472.3 | 1144 | aagtgaaacccgatagtagttct | 338269 | - | see also 5348 line | | |
| 23788 | TSNAXIP1 | NM_018430.1 | 1501 | aagcggagtgagaatgtgtatgt | 371357 | - | - | - | |

Figure 46

| | | | | | | | |
|---|---|---|---|---|---|---|---|
| 23789 | TSP-NY | NM_032573.2 | 884 | aagttgcctgcacgatgaattgc | 400787 | - | |
| 23790 | TSRC1 | NM_019032.2 | 1902 | cgggaccgtcttcgatataac | 374570 | - | - |
| 23791 | TSSC1 | NM_003310.1 | 886 | cagctgcggagacgactgtaagg | 331505 | - | - |
| 23792 | TSSC4 | NM_005706.2 | 1116 | gagaccacttccggaacaagagc | 334180 | - | - |
| 23793 | TTC11 | NM_016068.1 | 515 | ttgctgtgtccaagtccaaatcc | 356180 | - | - |
| 23794 | TTC13 | NM_024525.2 | 1969 | aagaccttctccgattatgaag | 386690 | - | receptor acitivity |
| 23795 | TTC15 | NM_016030.5 | 1903 | aggcgtatcattcggtttatcaag | 356029 | - | |
| 23796 | TTC4 | NM_004623.2 | 167 | gagagcgccatcagaaattgatc | 332629 | - | |
| 23797 | TTC5 | NM_138376.1 | 529 | cagtgtccgacaggctaagttgg | 407799 | - | |
| 23798 | TTC8 | NM_144596.1 | 341 | caggccgttaggccaatcacaca | 410133 | - | see also 9709 line |
| 23799 | TTF1 | NM_007344.2 | 1907 | aggtatagcgaaggagatactga | 337440 | - | see also 5081 line |
| 23800 | TTY7 | NM_031926.1 | 732 | cgggtttctcctccacttacaga | 397394 | - | |
| 23801 | TTYH2 | NM_032646.4 | 1586 | aggcactacgggaatcagtttcc | 401178 | - | |
| 23802 | TU12B1-TY | NM_016575.1 | 309 | atgtacagcgggcagtattaatg | 358193 | - | |
| 23803 | TUWD12 | NM_172240.1 | 128 | ccgttctggacgttatttcaaa | 424306 | - | |
| 23804 | UBAP1 | NM_016525.3 | 888 | tggaacgggcaaccctagattc | 358029 | - | see also 6900 line |
| 23805 | UBAP2 | NM_018449.2 | 2344 | cagcgacctccgtctcaagttcc | 371754 | - | |
| 23806 | UBE4B | NM_006048.2 | 1595 | gtgtttgaccgagttggaatag | 334702 | - | see also 4208 line |
| 23807 | UBN1 | NM_016936.2 | 2157 | gagcagagggaccggattgttc | 358787 | - | transcription_regulator |
| 23808 | UBPH | NM_019116.1 | 1324 | ctgtccgcatgtacaataaatc | 375287 | - | |
| 23809 | UBQLN1 | NM_013438.2 | 1756 | tggaggctcttcgggaactaatg | 338944 | - | see also 5443 line |
| 23810 | UBQLN2 | NM_013444.2 | 1466 | ctgaatagccgcgtgttactgc | 338962 | - | see also 5446 line |
| 23811 | UBXD1 | NM_025241.1 | 446 | ctgtgcctggccgtgtacttcacc | 394514 | - | |
| 23812 | UBXD2 | NM_014607.2 | 1304 | aagcgcttcggtggtactgttgc | 341013 | - | |
| 23813 | UNC93A | NM_018974.2 | 537 | ttgatctcatcgctggtatttgg | 373917 | - | |
| 23814 | UNC93B1 | NM_030930.1 | 789 | ctgaaccacacgctgtacaatgt | 395542 | - | see also 8966 line |
| 23815 | UNQ1912 | NM_198507.1 | 661 | acgttgtttgatgccaatcatcc | 438794 | - | |
| 23816 | UNQ2446 | NM_198443.1 | 146 | ccgatgtgacaccatataccagg | 437792 | - | |
| 23817 | UNQ2492 | NM_198585.1 | 244 | aggcctcacggcactcattctcc | 439836 | - | |
| 23818 | UNQ3030 | NM_198565.1 | 1710 | gggaatcagggacttagatct | 439604 | - | |
| 23819 | UNQ3033 | NM_198481.1 | 730 | aggctgccgaggcagatttatcc | 438476 | - | |
| 23820 | UNQ470 | NM_198573.1 | 271 | acgttgactctctctgaatcc | 439717 | - | |
| 23821 | UNQ473 | NM_198477.1 | 299 | atgtgagtgcaaagattggttcc | 438439 | - | |
| 23822 | UNQ564 | NM_198474.1 | 873 | gggttaggccgagcattggtttac | 438426 | - | |
| 23823 | UNQ6369 | NM_198510.1 | 3910 | ctggtgaagcgtctcatgtaga | 438835 | - | |

Figure 46

| | | | | | | | |
|---|---|---|---|---|---|---|---|
| 23824 | UNQ698 | NM_198538.1 | 750 | cagtcaacacgccttcatcaac | 439299 | - | - |
| 23825 | UNQ8193 | NM_198450.1 | 127 | cagagcagctcccatatatact | 437835 | - | see also 10332 line |
| 23826 | UNQ9366 | NM_198444.1 | 1502 | gggaacacgacgggatctacaac | 437808 | - | - |
| 23827 | UNQ9391 | NM_198464.1 | 719 | gtgccggatacaagaatgagagc | 438081 | - | - |
| 23828 | UNRIP | NM_007178.2 | 485 | gggtgcaacactgaataaggatg | 337013 | - | - |
| 23829 | UPK3B | NM_030570.2 | 481 | gtgcctacacaccacagataac | 394673 | - | - |
| 23830 | URB | NM_178274.2 | 310 | tgggaggtctcgcggaattgaga | 430643 | - | see also 10183 line |
| 23831 | URG4 | NM_017920.2 | 1958 | ctgcctcggagctaatcgatgg | 363975 | - | - |
| 23832 | URP | NM_198152.1 | 332 | gagacgtcctatgctgtagatgg | 437154 | - | - |
| 23833 | USH1G | NM_173477.2 | 1085 | caggccacgactccctgtttacc | 424946 | - | - |
| 23834 | VCIP135 | NM_025054.3 | 3306 | ctgagcaacttcataacgtaact | 393328 | - | see also 8835 line |
| 23835 | VEST1 | NM_052958.1 | 1871 | aaggtcagtagagcatccaaagt | 405538 | - | - |
| 23836 | VGLL2 | NM_153453.1 | 209 | gagctgtcgatgttattgtacc | 423272 | - | transcription_regulator |
| 23837 | VIT | NM_053276.2 | 442 | atggcactgacgtgtatgcatcc | 405941 | - | - |
| 23838 | VMP1 | NM_030938.2 | 375 | gtgcttatgctacgtattatgt | 395601 | - | see also 8971 line |
| 23839 | VprBP | NM_014703.1 | 4054 | cagtgtcgcgtgggttcaatca | 342556 | - | - |
| 23840 | VPS52 | NM_022553.3 | 947 | gtgacgaagatccgagagagtttat | 383559 | - | - |
| 23841 | VR22 | NM_013266.1 | 1301 | cagctccgcaaggctattataga | 338521 | - | - |
| 23842 | WAC | NM_016628.2 | 247 | atgttgcggagatctgatagtcc | 358622 | - | - |
| 23843 | WBP2 | NM_012478.2 | 68 | gcggagtgatcgtcaataacacc | 338281 | - | see also 5354 line |
| 23844 | WBSCR20A | NM_018044.2 | 244 | ctggccaagtgtcagtgtatga | 365766 | - | - |
| 23845 | WBSCR28 | NM_182504.2 | 435 | atgtctgcacggcctgatgttgg | 433992 | - | - |
| 23846 | WDR10 | NM_018262.2 | 2796 | atgcagtgcctcgatatagctca | 369397 | - | see also 7347 line |
| 23847 | WDR12 | NM_018256.2 | 874 | atggaggagtccacacaaatcgacc | 369264 | - | - |
| 23848 | WDR17 | NM_170710.2 | 349 | cagttggatcacgttataatga | 423970 | - | see also 10024 line |
| 23849 | WDR18 | NM_024100.2 | 63 | ctgcatcgtgtgggaacttcact | 386028 | - | - |
| 23850 | WDR20 | NM_144574.2 | 1011 | aaggtgaccctatggagtttagt | 409878 | - | see also 9702 line |
| 23851 | WDR7 | NM_015285.1 | 477 | ctggattagctccatgagtatta | 351018 | - | - |
| 23852 | WDR8 | NM_017818.2 | 806 | tggaaaggtgcgcatccttaatc | 362726 | - | - |
| 23853 | WFDC10B | NM_172131.2 | 185 | aaggacccagcatagatctatg | 424206 | - | - |
| 23854 | WFDC11 | NM_147197.2 | 437 | tcgaaaccagtggagattactaa | 415102 | - | - |
| 23855 | WFDC13 | NM_172005.1 | 181 | aagcagcgtgttctgaagtatat | 424201 | - | - |
| 23856 | WFDC6 | NM_080827.1 | 304 | cagaaaggtcagccttacttat | 406794 | - | - |
| 23857 | WFDC9 | NM_147198.2 | 459 | gagcccttaaatcaatgctaaa | 415111 | - | - |
| 23858 | WINS1 | NM_018148.3 | 1249 | tagagcagcaagcttagttataa | 367709 | - | - |

Figure 46

| | | | | | | | |
|---|---|---|---|---|---|---|---|
| 23859 | WIRE | NM_133264.2 | 394 | gggcgtcctcaggatgatacaga | 407162 | - | see also 9562 line |
| 23860 | WTAP | NM_004906.3 | 250 | aaggttcgattgagtgaaacaga | 333213 | - | - |
| 23861 | WWP2 | NM_007014.3 | 1020 | ctgcccaacgacgtgtctatta | 336753 | - | see also 4892 line |
| 23862 | XAGE-5 | NM_130775.1 | 69 | gagttggcgaggaaggagatata | 406925 | - | - |
| 23863 | XPO5 | NM_020750.1 | 1213 | ctgtcccgtgatcctttgctatt | 378485 | - | see also 7939 line |
| 23864 | XTP2 | NM_015172.2 | 4920 | cagaatcgacgtggcaacaatgg | 348823 | - | - |
| 23865 | XTP3TPB | NM_015701.2 | 1482 | gagcctcactcctgtcaatat | 355185 | - | see also 6537 line |
| 23866 | XTP7 | NM_138568.2 | 342 | cagcgtgtgagcgattccatga | 408447 | - | - |
| 23867 | YPEL1 | NM_013313.3 | 547 | atgcggttgccgacatctactgc | 338701 | - | - |
| 23868 | Z39IG | NM_007268.1 | 574 | tggtataagcaacagactaataa | 337371 | - | - |
| 23869 | ZA20D1 | NM_020205.1 | 2508 | gagtggctgattcctatagcaat | 376154 | - | - |
| 23870 | ZAR1 | NM_175619.1 | 1043 | agggaactaacaaggtttacttc | 429163 | - | - |
| 23871 | ZBTB1 | NM_014950.1 | 2120 | cagttcgactgcacaatgatat | 346324 | - | - |
| 23872 | ZCWCC2 | NM_024657.2 | 637 | gagtaccggctaacaataaatgc | 388629 | - | see also 8681 line |
| 23873 | ZCWPW1 | NM_017984.2 | 1526 | gagccgattcaacggatctaaca | 365086 | - | - |
| 23874 | ZD52F10 | NM_033317.2 | 1353 | cagccgactctggagggatttca | 403695 | - | - |
| 23875 | ZDHHC14 | NM_024630.2 | 1055 | ctgcgtagaacggtttgatcacc | 388287 | - | see also 8672 line |
| 23876 | ZDHHC16 | NM_032327.2 | 1077 | tagcatcgaaaggcacatcaaca | 399761 | - | see also 9163 line |
| 23877 | ZDHHC21 | NM_178566.2 | 1028 | atgggcattactatgttagttgg | 431327 | - | see also 10200 line |
| 23878 | ZFP106 | NM_022473.1 | 3135 | aggggacatagtgcacaattatc | 383119 | - | - |
| 23879 | ZFP276 | NM_152287.2 | 1385 | aggggctgacggcatggaagaagc | 415370 | - | see also 9830 line |
| 23880 | ZFP42 | NM_174900.1 | 375 | gcggtgtggccttatgtgatcg | 428556 | - | - |
| 23881 | ZFP91 | NM_053023.2 | 619 | tcggtccaagacgttcattgc | 405801 | - | - |
| 23882 | ZFPM1 | NM_153813.1 | 2412 | ccgtgcacaagcggtactactgc | 423929 | - | transcription_regulator |
| 23883 | ZG16 | NM_152338.1 | 162 | ccgggtccgagtcaaacatact | 415714 | - | - |
| 23884 | ZIC1 | NM_003412.2 | 1788 | acgagcgacaagcccatatctttg | 331663 | - | see also 2306 line |
| 23885 | ZMYND10 | NM_015896.2 | 866 | ggggcagccgttggcatactgtgg | 355368 | - | - |
| 23886 | ZMYND12 | NM_032257.3 | 1261 | cagtctagccaaagaacaacagc | 399099 | - | - |
| 23887 | ZMYND15 | NM_032265.1 | 1627 | cggcctgttccggcatatcagc | 399157 | - | - |
| 23888 | ZMYND17 | NM_178451.2 | 356 | aggatggaagacacacatttcgatt | 430731 | - | - |
| 23889 | ZMYND19 | NM_138462.2 | 588 | aagcttgattggcttgcaattc | 408292 | - | - |
| 23890 | ZNF169 | NM_194320.1 | 945 | ggggcgacacttcaggtatacatc | 436851 | - | - |
| 23891 | ZNF233 | NM_181756.1 | 478 | cagtaatcaagacctaataataa | 433096 | - | transcription_regulator |
| 23892 | ZNF237 | NM_014242.1 | 666 | tagtggtatcactacagaattga | 339988 | - | - |
| 23893 | ZNF291 | NM_020843.1 | 257 | tcgtctacgactggagataaaca | 379470 | - | see also 7989 line |

Figure 46

| | | | | | | | | | | |
|---|---|---|---|---|---|---|---|---|---|---|
| 23894 | ZNF294 | NM_015565.1 | 2301 | tggctcaaaggcgatatcctgg | 353970 | - | | | | |
| 23895 | ZNF313 | NM_018683.3 | 393 | aggtgtgaaggccaccattaagg | 372535 | - | see also 7554 line | | | |
| 23896 | ZNF364 | NM_014455.1 | 376 | gtggcggcagtcgatagacaat | 340678 | - | see also 5679 line | | | |
| 23897 | ZNF367 | NM_153695.1 | 449 | cagatactgtccgcgatttaata | 423569 | - | | | | |
| 23898 | ZNF407 | NM_017757.1 | 823 | ttgcatgcatgcgattactacg | 361849 | - | transcription_regulator | | | |
| 23899 | ZNF410 | NM_021188.1 | 325 | cagaatgcagtcggctaatgtta | 380712 | - | see also 8120 line | | | |
| 23900 | ZNF503 | NM_032772.3 | 2110 | ccgccaccgaccgtactactcc | 401356 | - | | | | |
| 23901 | ZNF509 | NM_145291.2 | 1572 | gtggtcgagggtttagtaacttc | 414186 | - | | | | |
| 23902 | ZNF511 | NM_145806.2 | 686 | aggccggatctacagacatagaat | 414926 | - | | | | |
| 23903 | ZNF515 | NM_152629.2 | 2364 | tcgactcactcgggcattacagt | 419165 | - | transcription_regulator | | | |
| 23904 | ZNHIT1 | NM_006349.2 | 649 | aagagactgcctcagtttgatga | 335303 | - | | | | |
| 23905 | ZNRF1 | NM_032268.3 | 1066 | cagctgcatagtgtttcaagt | 399223 | - | see also 9129 line | | | |
| 23906 | ZPBP | NM_007009.1 | 271 | aagagtccacacgtgttatgtgt | 336717 | - | | | | |
| 23907 | ZRANB1 | NM_017580.1 | 1340 | gaggcaggatatgctagcaatat | 359360 | - | | | | |
| 23908 | ZSWIM2 | NM_182521.1 | 783 | gtgtaccgaatgcatagaaatatc | 434108 | - | | | | |
| 23909 | ATBF1 | NM_006885.2 | 1573 | cgcggtgcatgacatcgaatga | 54 | - | see also 4813 line | | | |
| 23910 | ATBF1 | NM_006885.2 | 8662 | ttccaatccgactcgaaagatgt | 162 | - | see also 4813 line | | | |
| 23911 | GTF2IRD1 | NM_005685.2 | 2496 | ggccggtcgtgtccccttataaa | 217 | - | see also 10367 line | | | |
| 23912 | GTF2IRD1 | NM_005685.2 | 2497 | gccggtggtccccttataaac | 218 | - | see also 10367 line | | | |
| 23913 | GTF2IRD1 | NM_005685.2 | 1196 | cccgtgccctaccggaagattgc | 209 | - | see also 10367 line | | | |
| 23914 | HIF1A | NM_001530.2 | 809 | aactagccgaggaagaactatga | 237 | - | see also 1050 line | | | |
| 23915 | HIF1A | NM_001530.2 | 1362 | gtccttaaaccggttgaatcttc | 262 | - | see also 1050 line | | | |
| 23916 | ICSBP1 | NM_002163.1 | 1254 | ttccggagtttcagatatttg | 326 | - | see also 1387 line | | | |
| 23917 | ICSBP1 | NM_002163.1 | 458 | gtgcggtcgtctgaaatcgacg | 319 | - | see also 1387 line | | | |
| 23918 | MYEF2 | NM_016132.2 | 1276 | accgtggtgcgatgactagtagc | 358 | - | see also 6688 line | | | |
| 23919 | MYEF2 | NM_016132.2 | 1557 | atcgatatggatcgaggattttt | 372 | - | see also 6688 line | | | |
| 23920 | MYF5 | NM_005593.1 | 533 | ctgatgcatgcccgaatgtaac | 385 | - | see also 10371 line | | | |
| 23921 | MYF5 | NM_005593.1 | 105 | accgtccccgagggtgaatttg | 381 | - | see also 10371 line | | | |
| 23922 | MYOD1 | NM_002478.3 | 241 | gccacaacggacgacttctatga | 394 | - | see also 10372 line | | | |
| 23923 | MYOD1 | NM_002478.3 | 240 | tgccacaacgacgacttctatg | 393 | - | see also 10372 line | | | |
| 23924 | NFIX | NM_002501.1 | 900 | ccctgttgatgacgtttctatc | 404 | - | see also 1607 line | | | |
| 23925 | NR5A2 | NM_003822.2 | 903 | cagctcacccgagtccataatgg | 426 | - | see also 2604 line | | | |
| 23926 | PURA | NM_005859.2 | 725 | ctccttgactgtggcaacaaagc | 457 | | transcription_regulator | translation regulator | diabetes | |
| 23927 | RFX1 | NM_002918.2 | 95 | ggcaacaacaggggtatactgagc | 459 | - | see also 10374 line | | | |

Figure 46

| 23928 | RFX1 | NM_002918.2 | 2037 | gcgccaccgctggctgtacatga | 465 | - | see also 10374 line |
| 23929 | RFX3 | NM_002919.2 | 527 | tccgaacaacaacgtatccttac | 475 | - | see also 1964 line |
| 23930 | RFX3 | NM_002919.2 | 2190 | ccgtctactctacgacgaatata | 519 | - | see also 1964 line |
| 23931 | TFAP2A | NM_003220.1 | 978 | tacgtgtgcgaaaccgaatttcc | 546 | - | see also 2177 line |
| 23932 | TFAP2A | NM_003220.1 | 819 | ctccggagggcgaagtctaaaaa | 539 | - | see also 2177 line |
| 23933 | TFAP2A | NM_003220.1 | 901 | gacgtaaagctgccaacgttacc | 545 | - | see also 2177 line |
| 23934 | TFAP4 | NM_003223.1 | 1044 | ttccacatcccggcaaaatctgg | 551 | - | see also 10376 line |
| 23935 | TFAP4 | NM_003223.1 | 527 | agcagacagccgagtacatcttc | 550 | - | see also 10376 line |
| 23936 | AATF | NM_012138.2 | 327 | ttcctagtagtgggtagcattag | 553 | - | see also 10377 line |
| 23937 | AATF | NM_012138.2 | 1239 | aagcgctttgccgactttacagt | 569 | - | see also 10377 line |
| 23938 | ADNP | NM_015339.2 | 519 | gtgggacccatcacttacgaaaa | 591 | - | see also 6378 line |
| 23939 | ADNP | NM_015339.2 | 1255 | gtgaatcgactctcaataccaaa | 609 | - | see also 6378 line |
| 23940 | AEBP1 | NM_001129.2 | 1267 | gtggacgcctacggagaaagtca | 679 | - | see also 10379 line |
| 23941 | AEBP1 | NM_001129.2 | 2701 | gaccgggactatcaatgacttca | 688 | - | see also 10379 line |
| 23942 | AF5Q31 | NM_014423.1 | 786 | ggcacgaccgtgagtcatataac | 709 | - | see also 5667 line |
| 23943 | AF5Q31 | NM_014423.1 | 791 | gaccgtgagtcatataacaatag | 710 | - | see also 5667 line |
| 23944 | AF5Q31 | NM_014423.1 | 1322 | ccccctctaacggctattcatac | 718 | - | see also 5667 line |
| 23945 | AHR | NM_001621.2 | 1735 | gtctaatgcacgcctgctttata | 823 | - | see also 1076 line |
| 23946 | AHR | NM_001621.2 | 2943 | gggccgtgtcgatgtatcagtgc | 860 | - | see also 1076 line |
| 23947 | ALX3 | NM_006492.1 | 942 | ttcctcagatggtgactataagt | 870 | - | see also 10382 line |
| 23948 | ALX3 | NM_006492.1 | 803 | ccccatgcatgtctccatattcc | 868 | - | see also 10382 line |
| 23949 | ALX4 | NM_021926.1 | 559 | ttccctgctacgctaaagagagc | 876 | - | see also 8197 line |
| 23950 | ALX4 | NM_021926.1 | 455 | cgcgcaaccgcatctttacttgc | 875 | - | see also 8197 line |
| 23951 | ALX4 | NM_021926.1 | 187 | gggagggctcgtcgcctttagg | 873 | - | see also 8197 line |
| 23952 | AR | NM_000044.2 | 3628 | aactcgatcgtatcattgcatgc | 911 | - | see also 16 line |
| 23953 | AR | NM_000044.2 | 3624 | aaggaactcgatcgtatcattgc | 910 | - | see also 16 line |
| 23954 | ARNT | NM_001668.2 | 913 | tgggctcaaggagatcgtttatt | 930 | - | see also 10385 line |
| 23955 | ARNT | NM_001668.2 | 914 | gggctcaaggagatcgtttattt | 931 | - | see also 10385 line |
| 23956 | ARNT2 | NM_014862.1 | 1218 | tcctgtcggtcatgtatcgattt | 968 | - | see also 6062 line |
| 23957 | ARNT2 | NM_014862.1 | 501 | cagggcgagtgatttatgtgtct | 956 | - | see also 6062 line |
| 23958 | ARNTL | NM_001178.2 | 1399 | cacgcgatagatggaaagtttgt | 1011 | - | see also 767 line |
| 23959 | ARNTL | NM_001178.2 | 1091 | ctggagcacgacgttctttcttc | 1002 | - | see also 767 line |
| 23960 | ARNTL2 | NM_020183.3 | 440 | cgcgtaaactggacaaaacttaca | 1041 | - | see also 10388 line |
| 23961 | ARNTL2 | NM_020183.3 | 1813 | ttcgatgccctatgtgacaatga | 1082 | - | see also 10388 line |
| 23962 | ARX | NM_139058.1 | 1576 | gacagacgcgcctctagcatagc | 1087 | - | see also 9655 line |
| 23963 | ARX | NM_139058.1 | 38 | ggcccgagtgcaaaagtaaatct | 1083 | - | see also 9655 line |
| 23964 | ASCL1 | NM_004316.1 | 708 | ctcgtcttcgcccgaactgatgc | 1088 | - | see also 10389 line |

Figure 46

| 23965 | ASCL1 | NM_004316.1 | 884 | cggccaacaagaagatgagtaag | 1089 | - | see also 10389 line | | |
|---|---|---|---|---|---|---|---|---|---|
| 23966 | ATF1 | NM_005171.2 | 659 | tactcagcaaggtacaactattc | 1097 | - | see also 10391 line | | |
| 23967 | ATF1 | NM_005171.2 | 862 | cagatgacccccaattgaaaaga | 1101 | - | see also 10391 line | | |
| 23968 | ATF1 | NM_005171.2 | 320 | tgctcaacaggtatcatctttat | 1093 | - | see also 10391 line | | |
| 23969 | ATF3 | NM_001674.1 | 376 | ccctcggggtgtccatcacaaaa | 1103 | - | see also 10392 line | | |
| 23970 | ATF3 | NM_001674.1 | 378 | ctcggggtgtccatcacaaaagc | 1104 | - | see also 10392 line | | |
| 23971 | ATF6 | NM_007348.1 | 354 | tcctcggtcagtggactcttatt | 1115 | - | see also 10393 line | | |
| 23972 | ATF6 | NM_007348.1 | 1038 | agggttagaggcgagattaaagg | 1129 | - | see also 10393 line | | |
| 23973 | ATF7 | NM_006856.1 | 1319 | aagactcaaggctatttagaaag | 1167 | - | see also 4802 line | | |
| 23974 | ATOH1 | NM_005172.1 | 908 | cagcgatgatggcgcaaaagaat | 1176 | - | see also 10395 line | | |
| 23975 | ATOH1 | NM_005172.1 | 1003 | cacagaagcgacggggaattttc | 1178 | - | see also 10395 line | | |
| 23976 | ATRX | NM_000489.2 | 6444 | tggcttcgaaacattgactatta | 1275 | - | see also 379 line | | |
| 23977 | ATRX | NM_000489.2 | 6443 | gtggcttcgaaacattgactatt | 1274 | - | see also 379 line | | |
| 23978 | BACH1 | NM_001186.1 | 1416 | ctgagtgtccgtggttaggtatc | 1339 | - | see also 10397 line | | |
| 23979 | BACH1 | NM_001186.1 | 833 | gtccgtactggggaatctagtgt | 1324 | - | see also 10397 line | | |
| 23980 | BAPX1 | NM_001189.2 | 857 | tgcgcgacgaccagagacaatac | 1364 | - | see also 771 line | | |
| 23981 | BARHL1 | NM_020064.2 | 636 | ctcatcggactctgagtataaag | 1368 | - | see also 10399 line | | |
| 23982 | BARHL1 | NM_020064.2 | 639 | atcggactctgagtataaagtga | 1369 | - | see also 10399 line | | |
| 23983 | BARX1 | NM_021570.2 | 841 | aacgagcgagcagcttactgagc | 1374 | - | see also 10400 line | | |
| 23984 | BARX1 | NM_021570.2 | 673 | ttccacgccggacagaatagatc | 1372 | - | see also 10400 line | | |
| 23985 | BARX1 | NM_021570.2 | 674 | tccacgccggacagaatagatct | 1373 | - | see also 10400 line | | |
| 23986 | BARX2 | NM_003658.3 | 849 | acgtgatgtccccttagagatgg | 1378 | - | see also 10401 line | | |
| 23987 | BARX2 | NM_003658.3 | 848 | cacgtgatgtccccttagagatg | 1377 | - | see also 10401 line | | |
| 23988 | BATF | NM_006399.2 | 439 | ggctctacgcaaggagatcaagc | 1382 | - | transcription_regulator | - | - |
| 23989 | BATF | NM_006399.2 | 334 | gaggagggagaaaaatcgtattg | 1381 | - | see also 23988 line | | |
| 23990 | BATF | NM_006399.2 | 332 | cagaggagggagaaaaatcgtat | 1380 | - | see also 23988 line | | |
| 23991 | BAZ1B | NM_023005.2 | 934 | acgtagatcgccacgaaaacttc | 1390 | - | see also 10402 line | | |
| 23992 | BAZ1B | NM_023005.2 | 3041 | gtcatgcgcaggactcctattgg | 1445 | - | see also 10402 line | | |
| 23993 | BCL6 | NM_001706.2 | 684 | cacttgccggaagtttattaagg | 1491 | - | see also 1132 line | | |
| 23994 | BCL6 | NM_001706.2 | 2042 | aaccctatcgttgcaacatctgt | 1501 | - | see also 1132 line | | |
| 23995 | BCL6 | NM_001706.2 | 529 | aaccttagtgtgatcaatctaga | 1487 | - | see also 1132 line | | |
| 23996 | BHLHB2 | NM_003670.1 | 1015 | aaggatcggcgcaattaagcaag | 1512 | - | see also 2497 line | | |
| 23997 | BHLHB3 | NM_030762.1 | 521 | tgcgttccactcgggatttcaaa | 1530 | - | see also 8938 line | | |
| 23998 | BHLHB3 | NM_030762.1 | 244 | aacgagacgacaccaaggatacc | 1522 | - | see also 8938 line | | |
| 23999 | BNC | NM_001717.2 | 282 | cccacgctctaagtaagctaagg | 1538 | - | see also 1144 line | | |
| 24000 | BNC | NM_001717.2 | 2201 | cacgtgggtcagcatgcattagc | 1567 | - | see also 1144 line | | |

Figure 46

| 24001 | BRD8 | NM_006696.3 | 602 | gtgatggttcgctctcctataga | 1585 | - | see also 4727 line | | |
| 24002 | BRD8 | NM_006696.3 | 606 | tggttcgctctcctatagattct | 1587 | - | see also 4727 line | | |
| 24003 | BRF1 | NM_001519.2 | 907 | gacccgtgcctgtatattccacg | 1643 | - | see also 10408 line | | |
| 24004 | BRF1 | NM_001519.2 | 1188 | gaccattgatgagttcatgaaga | 1645 | - | see also 10408 line | | |
| 24005 | BTAF1 | NM_003972.1 | 2370 | gccgcgtttacttgatatccttt | 1716 | - | see also 10409 line | | |
| 24006 | BTAF1 | NM_003972.1 | 129 | caggctagatcgcctttttattt | 1650 | - | see also 10409 line | | |
| 24007 | BTAF1 | NM_003972.1 | 537 | gaggatagcacgccaacgaaaat | 1663 | - | see also 10409 line | | |
| 24008 | BTEB1 | NM_001206.1 | 1794 | ggccagactgccttaaaaagttc | 1820 | - | transcription_regulator | transcription factor | - |
| 24009 | BTF3L1 | NM_001208.1 | 293 | tgctactcagcatcgatcataaa | 1822 | - | transcription_regulator | transcription factor | - |
| 24010 | BTF3L1 | NM_001208.1 | 9 | aacaatcatgaaccaaaaactca | 1821 | - | see also 24009 line | | |
| 24011 | BTG2 | NM_006763.2 | 419 | cggctccatctgcgtcttgtacg | 1824 | - | see also 10410 line | | |
| 24012 | BTG2 | NM_006763.2 | 506 | gagcagcccctccaagaactacg | 1825 | - | see also 10410 line | | |
| 24013 | C10orf1 | NM_020527.1 | 806 | gccgacctaattcaattagctgg | 1843 | - | see also 10411 line | | |
| 24014 | C10orf1 | NM_020527.1 | 571 | gccgcagtcccattaatcacagt | 1837 | - | see also 10411 line | | |
| 24015 | C10orf48 | NM_173576.1 | 559 | atgcaagacgtcggcttaagaat | 1856 | - | see also 10085 line | | |
| 24016 | C10orf48 | NM_173576.1 | 1097 | cacgtattggaaggagatcaacg | 1873 | - | see also 10085 line | | |
| 24017 | C20orf17 | NM_173485.2 | 3529 | agcgtatgcaaatctctaagttt | 1922 | - | see also 10413 line | | |
| 24018 | C20orf17 | NM_173485.2 | 1844 | ggctaagaaacgcgtttttgatg | 1895 | - | see also 10413 line | | |
| 24019 | CART1 | NM_006982.1 | 189 | agccctccgagtaaaaacagtga | 1934 | - | see also 4878 line | | |
| 24020 | CART1 | NM_006982.1 | 723 | agggaacgttatggccaaataca | 1947 | - | see also 4878 line | | |
| 24021 | CBFA2T1 | NM_004349.2 | 1445 | ggcgatctctcaccgtactaagg | 1986 | - | see also 2910 line | | |
| 24022 | CBFA2T1 | NM_004349.2 | 543 | tacaccgacaacgttaactaatg | 1965 | - | see also 2910 line | | |
| 24023 | CBFA2T1 | NM_004349.2 | 1161 | tccaaataacggcttatcctacc | 1980 | - | see also 2910 line | | |
| 24024 | CBFA2T2 | NM_005093.2 | 2083 | tgcaatatcgcgcgatactgtgg | 2028 | - | see also 10416 line | | |
| 24025 | CBFA2T2 | NM_005093.2 | 1581 | tgcgctgaattgcattatggaaa | 2017 | - | see also 10416 line | | |
| 24026 | CBFA2T2 | NM_005093.2 | 1577 | accatgcgctgaattgcattatg | 2016 | - | see also 10416 line | | |
| 24027 | CBFA2T3 | NM_005187.4 | 1070 | cgcccgacaggaccaaagagaac | 2033 | - | see also 10417 line | | |
| 24028 | CBFB | NM_001755.1 | 76 | gccgcgagtgtgagattaagtac | 2035 | - | see also 10418 line | | |
| 24029 | CBFB | NM_001755.1 | 248 | aacacctagccgagagtatgtcg | 2039 | - | see also 10418 line | | |
| 24030 | CBL | NM_005188.1 | 2291 | cagagttcacgagcatgtgattg | 2096 | - | see also 3644 line | | |
| 24031 | CBL | NM_005188.1 | 385 | tagcccaccttatatcttagacc | 2054 | - | see also 3644 line | | |
| 24032 | CCRN4L | NM_012118.2 | 613 | cccacctatcagggttatgcaat | 2110 | - | see also 10420 line | | |
| 24033 | CCRN4L | NM_012118.2 | 1037 | atcgctgttacccatctaaaagc | 2124 | - | see also 10420 line | | |
| 24034 | CCRN4L | NM_012118.2 | 398 | accggtacaagcagactctatag | 2107 | - | see also 10420 line | | |

EP 1 752 536 A1

927

Figure 46

| 24035 | CDX1 | NM_001804.1 | 514 | ggcggtggcagcggtaagactcg | 2136 | - | transcription_regulator | transcription factor | - |
|---|---|---|---|---|---|---|---|---|---|
| 24036 | CDX1 | NM_001804.1 | 843 | ctctccaatgcctgtgaaagagg | 2137 | - | see also 24035 line | | |
| 24037 | CDX2 | NM_001265.2 | 704 | tcggcagccaagtgaaaaccagg | 2138 | - | see also 10421 line | | |
| 24038 | CDX4 | NM_005193.1 | 141 | accggctttctcgcactatatgg | 2142 | - | see also 10422 line | | |
| 24039 | CDX4 | NM_005193.1 | 140 | caccggctttctcgcactatatg | 2141 | - | see also 10422 line | | |
| 24040 | CDX4 | NM_005193.1 | 257 | atcctgggccgtctagtacaatg | 2143 | - | see also 10422 line | | |
| 24041 | CEBPB | NM_005194.2 | 1001 | agcacagcgacgagtacaagatc | 2153 | - | see also 3653 line | | |
| 24042 | CHD4 | NM_001273.1 | 5295 | tggctgctagccggcattataaa | 2227 | - | see also 838 line | | |
| 24043 | CHD4 | NM_001273.1 | 2487 | gacatgtatgtcgtaacctatgt | 2185 | - | see also 838 line | | |
| 24044 | CHES1 | NM_005197.1 | 1214 | cagccgacgaccactatgagttt | 2254 | - | see also 10424 line | | |
| 24045 | CHES1 | NM_005197.1 | 1525 | gggatccggtcctgtttgaataa | 2257 | - | see also 10424 line | | |
| 24046 | CHX10 | NM_182894.1 | 482 | gcgatcgaaaaatgtccaaatct | 2260 | - | see also 10280 line | | |
| 24047 | CITED1 | NM_004143.2 | 623 | tccaaccttgcggtgaaagatcg | 2266 | - | see also 10426 line | | |
| 24048 | CITED1 | NM_004143.2 | 622 | ctccaaccttgcggtgaaagatc | 2265 | - | see also 10426 line | | |
| 24049 | CITED2 | NM_006079.2 | 942 | tggggcaaaacgagtttgatttt | 2275 | - | see also 10427 line | | |
| 24050 | CITED2 | NM_006079.2 | 941 | ctggggcaaaacgagtttgattt | 2274 | - | see also 10427 line | | |
| 24051 | CLOCK | NM_004898.2 | 2676 | cagccaccgcaacaatttttaca | 2344 | - | see also 3398 line | | |
| 24052 | CLOCK | NM_004898.2 | 1209 | cacagggcaccacccataatagg | 2298 | - | see also 3398 line | | |
| 24053 | CNOT7 | NM_013354.4 | 844 | ttgagatccttcgattgtttttt | 2347 | - | see also 10429 line | | |
| 24054 | CNOT8 | NM_004779.4 | 294 | gtgtgggccagtaatctagaaga | 2352 | - | see also 10430 line | | |
| 24055 | CNOT8 | NM_004779.4 | 393 | gtgcgaccaattggtgaatttcg | 2361 | - | see also 10430 line | | |
| 24056 | CREB1 | NM_004379.2 | 331 | tcccaccgtaactctagtacagc | 2386 | - | see also 2936 line | | |
| 24057 | CREB1 | NM_004379.2 | 787 | gccgggtactaccattctacagt | 2400 | - | see also 2936 line | | |
| 24058 | CREB3L1 | NM_052854.1 | 1366 | ctcccgaagcctcctattctacg | 2419 | - | see also 9436 line | | |
| 24059 | CREB3L1 | NM_052854.1 | 182 | gagtcggacttcctcaacaatgc | 2410 | - | see also 9436 line | | |
| 24060 | CREBBP | NM_004380.1 | 4531 | ctccgcacagccgtttaccatga | 2511 | - | see also 10432 line | | |
| 24061 | CREBBP | NM_004380.1 | 2202 | acggaggtcgcgtttacataaac | 2465 | - | see also 10432 line | | |
| 24062 | CREBL2 | NM_001310.2 | 399 | agcccgaaaaaagctgagatatc | 2529 | - | see also 10433 line | | |
| 24063 | CREBL2 | NM_001310.2 | 389 | gagaatgccgagcccgaaaaaag | 2527 | - | see also 10433 line | | |
| 24064 | CRX | NM_000554.2 | 924 | gccccgtggatagcttggaattc | 2570 | - | see also 10434 line | | |
| 24065 | CRX | NM_000554.2 | 925 | ccccgtggatagcttggaattca | 2571 | - | see also 10434 line | | |
| 24066 | CSDA | NM_003651.3 | 1030 | gaccggttcatcgaaatccaact | 2580 | - | see also 10435 line | | |
| 24067 | CSDA | NM_003651.3 | 486 | ttcaacgtcagaaatggatatgg | 2576 | - | see also 10435 line | | |
| 24068 | CTCF | NM_006565.1 | 317 | ttgtggaggagtccgaaactttt | 2581 | - | see also 4603 line | | |
| 24069 | CTCF | NM_006565.1 | 1307 | ggcatcgtcgttacaaacacacc | 2604 | - | see also 4603 line | | |
| 24070 | CUTL1 | NM_001913.2 | 139 | gtccagaaagcggcttatcgaac | 2624 | - | see also 10437 line | | |

928

Figure 46

| | | | | | | | |
|---|---|---|---|---|---|---|---|
| 24071 | CUTL1 | NM_001913.2 | 819 | agctctcatggccaatcactcc | 2636 | - | see also 10437 line |
| 24072 | DLX1 | NM_178120.2 | 510 | ggcggtgaagtgcgcttcaatgg | 2672 | - | see also 10438 line |
| 24073 | DLX1 | NM_178120.2 | 804 | cccggctggaacctaactctc | 2673 | - | see also 10438 line |
| 24074 | DLX1 | NM_178120.2 | 279 | cactactccatgcactgtttaca | 2670 | - | see also 10438 line |
| 24075 | DLX2 | NM_004405.2 | 497 | ctcttcagccgcgtttccaaaag | 2676 | - | see also 10439 line |
| 24076 | DLX2 | NM_004405.2 | 500 | ttcagcgccgtttccaaaagact | 2677 | - | see also 10439 line |
| 24077 | DLX3 | NM_005220.2 | 358 | cgctcccagcacgattactact | 2680 | - | see also 3662 line |
| 24078 | DLX5 | NM_005221.3 | 511 | gccgcctcgctgggattgacaca | 2693 | - | see also 10441 line |
| 24079 | DLX5 | NM_005221.3 | 270 | agcttatgccgactatagctacg | 2691 | - | see also 10441 line |
| 24080 | DMRT1 | NM_021951.1 | 793 | agccgtctcgtttccttattac | 2698 | - | see also 10442 line |
| 24081 | DMRT1 | NM_021951.1 | 1166 | cagctctccattagttaacaaga | 2701 | - | see also 10442 line |
| 24082 | DMRT2 | NM_066557.3 | 534 | gggcgtttccgggaaacagaata | 2702 | - | see also 10443 line |
| 24083 | DMRT2 | NM_066557.3 | 536 | ggctttccgggaaacagaataat | 2704 | - | see also 10443 line |
| 24084 | DMRT3 | NM_021240.2 | 1172 | aagccagtcgagcccctttgc | 2743 | - | see also 8142 line |
| 24085 | DMRT3 | NM_021240.2 | 478 | agctcgccaagccagatttgact | 2732 | - | see also 8142 line |
| 24086 | DMRT3 | NM_021240.2 | 516 | gacggcaagtcggcagacaatac | 2733 | - | see also 8142 line |
| 24087 | DMRTB1 | NM_033067.1 | 613 | cccactgttcaccgactttgtgc | 2748 | - | see also 10445 line |
| 24088 | DMRTB1 | NM_033067.1 | 978 | cactgaccgtctgtttgatact | 2750 | - | see also 10445 line |
| 24089 | DMRTB1 | NM_033067.1 | 184 | ctccgagcgcagaagatcatgg | 2747 | - | see also 10445 line |
| 24090 | DMRTC2 | NM_033052.1 | 316 | agcaggaggcgcagctaaagaag | 2754 | - | see also 9353 line |
| 24091 | DMTF1 | NM_021145.1 | 787 | atcttaaggcacgcggaataaaa | 2767 | - | see also 8108 line |
| 24092 | DMTF1 | NM_021145.1 | 2530 | atgtcgaagatttgtaaactgt | 2831 | - | see also 8108 line |
| 24093 | DRIL1 | NM_005224.1 | 1136 | caccctgcggaccaatacatga | 2836 | - | see also 10447 line |
| 24094 | DRIL1 | NM_005224.1 | 1138 | cctgcggaccaatacatgaag | 2837 | - | see also 10447 line |
| 24095 | DSCR1 | NM_004414.4 | 435 | gagcttcaaacgagtcagaataa | 2843 | - | see also 10448 line |
| 24096 | DSCR1 | NM_004414.4 | 650 | aagatgcgaccccagtcataaac | 2846 | - | see also 10448 line |
| 24097 | DSIP1 | NM_004089.2 | 525 | gtgagaacaccctgttgaagacc | 2853 | - | see also 10449 line |
| 24098 | DUX4 | NM_033178.1 | 572 | ctctgcggcacccggaaaacatgc | 2855 | - | transcription_regulator |
| 24099 | E2F1 | NM_005225.1 | 989 | aacaaggcccgatcgatgtttc | 2861 | - | see also 10450 line |
| 24100 | E2F1 | NM_005225.1 | 993 | aggcccgatcgatgttttcctgt | 2862 | - | see also 10450 line |
| 24101 | E2F2 | NM_004091.2 | 632 | cggacccgagggccaagtgtgc | 2866 | - | see also 10451 line |
| 24102 | E2F2 | NM_004091.2 | 1191 | atccgtcgtttggcaactttaa | 2869 | - | see also 10451 line |
| 24103 | E2F3 | NM_001949.2 | 654 | gtcaccgatggggtattggatt | 2875 | - | see also 10452 line |
| 24104 | E2F3 | NM_001949.2 | 656 | caccgatggggtattggatttg | 2876 | - | see also 10452 line |
| 24105 | E2F4 | NM_001950.2 | 1220 | cccgggaccaccgattatatct | 2910 | - | see also 10453 line |
| 24106 | E2F4 | NM_001950.2 | 101 | cactccaagccggcacgcaaaaga | 2902 | - | see also 10453 line |

Figure 46

| 24107 | E2F4 | NM_001950.2 | 229 | ggcggatttacgacattaccaat | 2904 | - | see also 10453 line |
|---|---|---|---|---|---|---|---|
| 24108 | E2F5 | NM_001951.2 | 922 | atctattagtggagatatcattg | 2938 | - | see also 1240 line |
| 24109 | E2F5 | NM_001951.2 | 736 | ttcatctaagcccgtggttttc | 2932 | - | see also 1240 line |
| 24110 | E2F5 | NM_001951.2 | 544 | ttcctatgtaactcatgaagaca | 2926 | - | see also 1240 line |
| 24111 | E2F6 | NM_001952.3 | 470 | aagagacctcgttttgatgtatc | 2947 | - | see also 10455 line |
| 24112 | E2F6 | NM_001952.3 | 486 | atgtatcgctggtttatttaact | 2949 | - | see also 10455 line |
| 24113 | E4F1 | NM_004424.2 | 223 | ttcaccgccttggaggattttgt | 2953 | - | see also 10456 line |
| 24114 | E4F1 | NM_004424.2 | 1742 | agccgttcaagtgctacaagtgc | 2957 | - | see also 10456 line |
| 24115 | EGR1 | NM_001964.2 | 565 | cacctgaccgcagagtcttttcc | 2958 | - | transcription_regulator Egr1/Egr2 - |
| 24116 | EGR1 | NM_001964.2 | 1877 | ctcccaggacaattgaaatttgc | 2959 | - | see also 24115 line |
| 24117 | EGR3 | NM_004430.2 | 481 | ctcggtagtccattacaatcaga | 2964 | - | see also 2963 line |
| 24118 | EGR3 | NM_004430.2 | 475 | cagcgactcggtagtccattaca | 2961 | - | see also 2963 line |
| 24119 | EGR4 | NM_001965.1 | 854 | gaggctcgttttcccgtaatagg | 2972 | - | see also 10458 line |
| 24120 | EGR4 | NM_001965.1 | 866 | cccgtaatagggaccaagattga | 2973 | - | see also 10458 line |
| 24121 | EHF | NM_012153.2 | 945 | ctcagccgagctatgagatatta | 2995 | - | see also 5151 line |
| 24122 | EHF | NM_012153.2 | 765 | aacccgagagggactcacttatg | 2987 | - | see also 5151 line |
| 24123 | EHF | NM_012153.2 | 943 | agctcagccgagctatgagatat | 2994 | - | see also 5151 line |
| 24124 | ELF1 | NM_172373.2 | 1204 | accagccggtcgagagtatcttc | 3032 | - | see also 10046 line |
| 24125 | ELF1 | NM_172373.2 | 1375 | ttccggactgttcatgtagtaca | 3043 | - | see also 10046 line |
| 24126 | ELF1 | NM_172373.2 | 1207 | agccggtcgagagtatcttcaag | 3033 | - | see also 10046 line |
| 24127 | ELF2 | NM_006874.1 | 728 | accatgggacgagctttgagata | 3074 | - | see also 4810 line |
| 24128 | ELF2 | NM_006874.1 | 482 | aagaccagcaatcaccaatttc | 3063 | - | see also 4810 line |
| 24129 | ELF3 | NM_004433.3 | 4232 | cggcgactcgtctacaagtttgg | 3092 | - | see also 10462 line |
| 24130 | ELF3 | NM_004433.3 | 4081 | tcggcatgaaggcgtcttcaagt | 3087 | - | see also 10462 line |
| 24131 | ELF4 | NM_001421.1 | 662 | accgcggaagtcttactcaatat | 3098 | - | see also 10463 line |
| 24132 | ELF4 | NM_001421.1 | 658 | gtcaaccgcggaagtcttactca | 3096 | - | see also 10463 line |
| 24133 | ELF5 | NM_001422.2 | 817 | gaccgaaggttagtgtacaaatt | 3136 | - | see also 994 line |
| 24134 | ELF5 | NM_001422.2 | 812 | gggttgaccgaaggttagtgtac | 3135 | - | see also 994 line |
| 24135 | ELK1 | NM_005229.2 | 316 | cacgggatggtggtgaattcaag | 3141 | - | see also 3667 line |
| 24136 | ELK3 | NM_005230.1 | 357 | gacctcgaacgatggtgaattca | 3145 | - | see also 10466 line |
| 24137 | ELK3 | NM_005230.1 | 672 | cacgagccgcaacgaatacatcc | 3150 | - | see also 10466 line |
| 24138 | ELK4 | NM_001973.1 | 551 | gacctctagccgcaatgactaca | 3158 | - | see also 10467 line |
| 24139 | ELK4 | NM_001973.1 | 559 | gccgcaatgactacatacactct | 3161 | - | see also 10467 line |
| 24140 | EMX2 | NM_004098.2 | 1473 | aaggctcagattcgcaacaaaag | 3175 | - | see also 2765 line |
| 24141 | EN1 | NM_001426.2 | 1748 | cacggcaacccggctatcctact | 3185 | - | see also 10468 line |
| 24142 | EN1 | NM_001426.2 | 1720 | cggagcgcagggcaccaaatacc | 3184 | - | see also 10468 line |

Figure 46

| 24143 | EN2 | NM_001427.2 | 2354 | gtcccaggtctcgaaaaccaaag | 3188 | - | transcription_regulator | transcription factor | - |
|---|---|---|---|---|---|---|---|---|---|
| 24144 | EN2 | NM_001427.2 | 2355 | tcccaggtctcgaaaaccaaaga | 3189 | - | see also 24143 line | | |
| 24145 | EN2 | NM_001427.2 | 2358 | caggtctcgaaaaccaaagaaga | 3190 | - | see also 24143 line | | |
| 24146 | EOMES | NM_005442.2 | 1404 | tcggtacggcgttcaatccttct | 3216 | - | see also 10469 line | | |
| 24147 | EOMES | NM_005442.2 | 1451 | tacctcaagcccgctattataat | 3219 | - | see also 10469 line | | |
| 24148 | EP300 | NM_001429.1 | 3447 | ggctatgggcctcgtatgcaaca | 3297 | - | see also 995 line | | |
| 24149 | EP300 | NM_001429.1 | 2548 | cccccaacctaagcactgttagt | 3265 | - | see also 995 line | | |
| 24150 | ERF | NM_006494.1 | 1556 | gcccctcaagctacgctttaagc | 3389 | - | see also 4547 line | | |
| 24151 | ERF | NM_006494.1 | 373 | ccctgcgctattactataacaag | 3384 | - | see also 4547 line | | |
| 24152 | ERG | NM_004449.2 | 378 | cacgaacgagcgcagagttatcg | 3394 | - | see also 2984 line | | |
| 24153 | ERG | NM_004449.2 | 843 | aacaagtagccgccttgcaaatc | 3404 | - | see also 2984 line | | |
| 24154 | ESR1 | NM_000125.1 | 1659 | tcggttccgcatgatgaatctgc | 3428 | - | see also 10473 line | | |
| 24155 | ESR1 | NM_000125.1 | 811 | aggccaaattcagataatcgacg | 3419 | - | see also 10473 line | | |
| 24156 | ESR2 | NM_001437.1 | 881 | tacgcatcgggatatcactatgg | 3444 | - | see also 1005 line | | |
| 24157 | ESR2 | NM_001437.1 | 1809 | acgtcaggcatgcgagtaacaag | 3457 | - | see also 1005 line | | |
| 24158 | ESRRA | NM_004451.3 | 1029 | ggccttcgctgaggacttagtcc | 3459 | - | transcription_regulator, receptor_acitivity | steroid hormone receptor | - |
| 24159 | ESRRB | NM_004452.2 | 1312 | agctggtacgcaggtacaagaag | 3473 | - | see also 10475 line | | |
| 24160 | ESRRB | NM_004452.2 | 868 | tgcgtggaggccgtcagaaatac | 3466 | - | see also 10475 line | | |
| 24161 | ESRRG | NM_001438.1 | 1146 | gacgaagaccagtccaaattagc | 3494 | - | see also 1006 line | | |
| 24162 | ESRRG | NM_001438.1 | 696 | tgccgcttcatgaagtgtttaaa | 3485 | - | see also 1006 line | | |
| 24163 | ESX1L | NM_153448.2 | 487 | caccgcgttcacgcagttcagc | 3501 | - | see also 10477 line | | |
| 24164 | ESX1L | NM_153448.2 | 515 | gagctagagaactttttcgatga | 3502 | - | see also 10477 line | | |
| 24165 | ESX1L | NM_153448.2 | 570 | gacttgcagcacgcctgaatttg | 3505 | - | see also 10477 line | | |
| 24166 | ETS1 | NM_005238.1 | 733 | cccgctatacctcggattacttc | 3528 | - | see also 3672 line | | |
| 24167 | ETS1 | NM_005238.1 | 1044 | gagagctacgatagttgtgatcg | 3532 | - | see also 3672 line | | |
| 24168 | ETS2 | NM_005239.2 | 717 | ctccgttcctcattggattaaca | 3543 | - | see also 10479 line | | |
| 24169 | ETS2 | NM_005239.2 | 1189 | gagcaaggcaaaccagttatacc | 3548 | - | see also 10479 line | | |
| 24170 | ETV1 | NM_004956.2 | 1033 | aagggaaggacgtcctatgtacc | 3558 | - | see also 10480 line | | |
| 24171 | ETV1 | NM_004956.2 | 623 | gagcagtttgtaccagactatca | 3554 | - | see also 10480 line | | |
| 24172 | ETV3 | NM_005240.1 | 259 | cagggagagtacggggaatttgt | 3575 | - | see also 10481 line | | |
| 24173 | ETV3 | NM_005240.1 | 452 | tgcccaactacccattcatcaac | 3576 | - | see also 10481 line | | |
| 24174 | ETV4 | NM_001986.1 | 1257 | gccgctcgctccgatactattat | 3594 | - | see also 10482 line | | |
| 24175 | ETV4 | NM_001986.1 | 1256 | agccgctcgctccgatactatta | 3593 | - | see also 10482 line | | |
| 24176 | ETV4 | NM_001986.1 | 886 | caccgggtgcgcatcaatgtacc | 3585 | - | see also 10482 line | | |
| 24177 | ETV5 | NM_004454.1 | 1544 | gagcgatacgtctacaaatttgt | 3612 | - | see also 10483 line | | |

Figure 46

| | | | | | | | |
|---|---|---|---|---|---|---|---|
| 24178 | ETV5 | NM_004454.1 | 1223 | tactttgacgacacttgtgttgt | 3609 | - | see also 10483 line |
| 24179 | ETV6 | NM_001987.2 | 331 | cgctatcgatctcctcattcagg | 3622 | - | see also 1267 line |
| 24180 | ETV7 | NM_016135.2 | 997 | tgcgccactattataagcttaat | 3649 | - | see also 10485 line |
| 24181 | ETV7 | NM_016135.2 | 859 | cccgatatgagccctacatcaag | 3646 | - | see also 10485 line |
| 24182 | EVX1 | NM_001989.2 | 572 | accgagtcggatttctatgaaga | 3656 | - | see also 10486 line |
| 24183 | EVX1 | NM_001989.2 | 571 | caccgagtcggatttctatgaag | 3655 | - | see also 10486 line |
| 24184 | FHX | NM_018416.2 | 1928 | cccaccctcgaacaactactaca | 3673 | - | see also 7459 line |
| 24185 | FHX | NM_018416.2 | 2318 | ttctgactggtgctctaatattg | 3675 | - | see also 7459 line |
| 24186 | FKHL18 | NM_004118.3 | 136 | tacagctacatcgcccttattgc | 3685 | - | see also 10488 line |
| 24187 | FKHL18 | NM_004118.3 | 360 | ccctgactgccacgacatgtttg | 3686 | - | see also 10488 line |
| 24188 | FLI1 | NM_002017.2 | 1291 | gaccgagtcgtccatgtacaagt | 3719 | - | see also 1296 line |
| 24189 | FLI1 | NM_002017.2 | 909 | gggcacaaacgatcagtaagaat | 3712 | - | see also 1296 line |
| 24190 | FLJ12287 | NM_022367.2 | 911 | ttcgacccaggtcgtctacttct | 3731 | - | see also 8351 line |
| 24191 | FLJ12287 | NM_022367.2 | 772 | tgctctattctggtactatgaac | 3730 | - | see also 8351 line |
| 24192 | FLJ12895 | NM_023926.3 | 1196 | ctccggttggtggaaagagatcc | 3748 | - | see also 10491 line |
| 24193 | FLJ12895 | NM_023926.3 | 545 | cgcctgcgtttccgggaatttgt | 3744 | - | see also 10491 line |
| 24194 | FLJ12895 | NM_023926.3 | 823 | tgcgggctcatcctcaattctta | 3746 | - | see also 10491 line |
| 24195 | FLJ20626 | NM_017908.1 | 911 | gcctgcgctcgggaaatgagagt | 3749 | - | transcription_regulator |
| 24196 | FLJ21616 | NM_024567.1 | 524 | ccctggcgctacactaagtatga | 3763 | - | see also 8645 line |
| 24197 | FLJ21616 | NM_024567.1 | 631 | gacgagggagtcgatttacctgg | 3769 | - | see also 8645 line |
| 24198 | FLJ23614 | NM_152695.2 | 1305 | cactcggcggtcacatcttatag | 3795 | - | see also 10493 line |
| 24199 | FLJ23614 | NM_152695.2 | 642 | agggccaccggagctgaactatg | 3785 | - | see also 10493 line |
| 24200 | FLJ25680 | NM_153216.1 | 397 | ctcgggcatactgaaagagttgc | 3805 | - | see also 10495 line |
| 24201 | FLJ25680 | NM_153216.1 | 797 | gtggttcgagtttggttctataa | 3810 | - | see also 10495 line |
| 24202 | FLJ30678 | NM_144657.1 | 1242 | accaatccattacggagtaattt | 3849 | - | see also 10496 line |
| 24203 | FLJ30678 | NM_144657.1 | 1100 | gcccggaccaggaagaaatatgc | 3842 | - | see also 10496 line |
| 24204 | FLJ30678 | NM_144657.1 | 1027 | cacagataccaagctattcgaga | 3836 | - | see also 10496 line |
| 24205 | FLJ33779 | NM_182572.2 | 751 | agcactccccgaggaaagtgagt | 3867 | - | see also 10497 line |
| 24206 | FLJ35867 | NM_152455.2 | 2060 | gtcggagtgcacgactcattaga | 3890 | - | see also 10498 line |
| 24207 | FLJ35867 | NM_152455.2 | 2407 | gacttacgtgcacatcatagaac | 3896 | - | see also 10498 line |
| 24208 | FLJ35936 | NM_173464.1 | 477 | cccgacatccatcggtattctgt | 3899 | - | see also 10499 line |
| 24209 | FLJ35936 | NM_173464.1 | 826 | ggcgaccatagcagatattgttg | 3909 | - | see also 10499 line |
| 24210 | FMNL2 | NM_052905.1 | 560 | ttgcacagtacgcggtaactttt | 3942 | - | see also 10500 line |
| 24211 | FMNL2 | NM_052905.1 | 2435 | atcggttcatgatgcagtttagt | 3979 | - | see also 10500 line |
| 24212 | FOSL2 | NM_005253.2 | 1422 | cacggcccagtgtgcaagattag | 4000 | - | see also 3697 line |
| 24213 | FOXA1 | NM_004496.2 | 1705 | tccagacccgtcctaaacacttc | 4009 | - | see also 10502 line |

Figure 46

| | | | | | | | transcription_regulator | transcription factor | see also | |
|---|---|---|---|---|---|---|---|---|---|---|
| 24214 | FOXA1 | NM_004496.2 | 1571 | acgaacaggcactgcaatactcg | 4008 | - | | | see also 10502 line | |
| 24215 | FOXA2 | NM_021784.3 | 278 | ctcctccgtgagcaacatgaacg | 4010 | - | | | see also 8169 line | |
| 24216 | FOXA3 | NM_004497.2 | 650 | cccttactaccggaggaatcagc | 4015 | - | | | see also 3046 line | |
| 24217 | FOXA3 | NM_004497.2 | 543 | gccaagccacgtattcctatat | 4014 | - | | | see also 3046 line | |
| 24218 | FOXA3 | NM_004497.2 | 1225 | cccgctctttgcttaatgcatcc | 4020 | - | | | see also 3046 line | |
| 24219 | FOXB1 | NM_012182.1 | 620 | cgctcccaaccagttgactacc | 4022 | - | transcription_regulator | transcription factor | | |
| 24220 | FOXB1 | NM_012182.1 | 795 | cgccatccccgtgccattaagc | 4023 | - | | | see also 24219 line | |
| 24221 | FOXB1 | NM_012182.1 | 297 | cccaagctgcggggacatgttcg | 4021 | - | | | see also 24219 line | |
| 24222 | FOXC1 | NM_001453.1 | 1562 | ctccagtgaacgggaatagtagc | 4024 | - | | | see also 10504 line | |
| 24223 | FOXC1 | NM_001453.1 | 1639 | gtctacgactgtagcaagttttg | 4027 | - | | | see also 10504 line | |
| 24224 | FOXC2 | NM_005251.1 | 1482 | ctcctacgactgcacgaaatact | 4029 | - | transcription_regulator | transcription factor | | |
| 24225 | FOXC2 | NM_005251.1 | 1367 | cccaccggctgggggattgagaac | 4028 | - | | | see also 24224 line | |
| 24226 | FOXD1 | NM_004472.1 | 1769 | tccagtgtcgagaactttactgc | 4031 | - | | | see also 10505 line | |
| 24227 | FOXD2 | NM_004474.1 | 2188 | tcgaggccgacgcagacataga | 4032 | - | | | see also 10506 line | |
| 24228 | FOXD3 | NM_012183.1 | 392 | ccccgagcaagcccaagaacagc | 4034 | - | | | see also 10507 line | |
| 24229 | FOXD3 | NM_012183.1 | 1086 | caccaccggtcgctcatcaagt | 4035 | - | | | see also 10507 line | |
| 24230 | FOXE1 | NM_004473.3 | 929 | accgcgacaaccccaaaaagtgg | 4037 | - | transcription_regulator | RNA polymerase II transcription factor | | thyroid agenesis |
| 24231 | FOXE1 | NM_004473.3 | 1050 | cccaacgcgaggacatgttcg | 4038 | - | | | see also 24230 line | |
| 24232 | FOXF2 | NM_001452.1 | 1377 | ctcagctagcgggtcgtattatc | 4041 | - | | | see also 10508 line | |
| 24233 | FOXF2 | NM_001452.1 | 1380 | agctagcgggtcgtattatcacc | 4042 | - | | | see also 10508 line | |
| 24234 | FOXH1 | NM_003923.1 | 1413 | cacctctacttgcctatctaca | 4061 | - | | | see also 10510 line | |
| 24235 | FOXH1 | NM_003923.1 | 1577 | gggtgcaccccaacaaaaagcatc | 4063 | - | | | see also 10510 line | |
| 24236 | FOXJ1 | NM_001454.1 | 698 | atcccacctggccagaattcaatc | 4066 | - | | | see also 10511 line | |
| 24237 | FOXJ1 | NM_001454.1 | 639 | cacccctgtcggccatctacaagt | 4064 | - | | | see also 10511 line | |
| 24238 | FOXL1 | NM_005250.1 | 42 | ctcgcccatgctgtatctgtacg | 4068 | - | | | see also 10512 line | |
| 24239 | FOXL1 | NM_005250.1 | 39 | cgcctcgcccatgctgtatctgt | 4067 | - | | | see also 10512 line | |
| 24240 | FOXL2 | NM_023067.1 | 1042 | gggctagtgaactgctacaatgg | 4071 | - | transcription_regulator | transcription factor | | blepharophimosis |
| 24241 | FOXM1 | NM_021953.1 | 1483 | cacttagcgagaccccatcaaagt | 4103 | - | | | see also 10513 line | |
| 24242 | FOXM1 | NM_021953.1 | 786 | ctcttacatggccatgattacaat | 4093 | - | | | see also 10513 line | |
| 24243 | FOXM1 | NM_021953.1 | 587 | caggctgcactatcaaacaatagc | 4089 | - | | | see also 10513 line | |
| 24244 | FOXN1 | NM_003593.2 | 906 | ccccgtcagcgagatctacacattt | 4121 | - | | | see also 10514 line | |
| 24245 | FOXN1 | NM_003593.2 | 767 | gggctcctcacactatcagtacc | 4117 | - | | | see also 10514 line | |
| 24246 | FOXO1A | NM_002015.2 | 2316 | gtgtcaagacaacgacgacacatagc | 4162 | - | | | see also 1293 line | |

Figure 46

| 24247 | FOXO1A | NM_002015.2 | 1429 | cccgccatctgccgcaaagatgg | 4137 | - | see also 1293 line | | |
|---|---|---|---|---|---|---|---|---|---|
| 24248 | FOXO3A | NM_001455.1 | 2446 | aacgtgatgcttcgcaatgatcc | 4175 | - | see also 1014 line | | |
| 24249 | FOXO3A | NM_001455.1 | 2456 | ttcgcaatgatccgatgatgtcc | 4176 | - | see also 1014 line | | |
| 24250 | FOXP1 | NM_032682.3 | 1968 | gtgcgtcataatcttagtcttca | 4212 | - | see also 9272 line | | |
| 24251 | FOXP2 | NM_014491.1 | 1011 | agggctagacctcactactaaca | 4226 | - | see also 5707 line | | |
| 24252 | FOXP3 | NM_014009.2 | 1162 | tccacaacatggactacttcaag | 4248 | - | see also 10519 line | | |
| 24253 | FOXP3 | NM_014009.2 | 475 | tccaggacaggccacatttcatg | 4246 | - | see also 10519 line | | |
| 24254 | FOXP4 | NM_138457.1 | 1923 | gcctatttccgcagaaacactgc | 4254 | - | see also 9622 line | | |
| 24255 | FOXP4 | NM_138457.1 | 1910 | caccaggatgttcgcctatttcc | 4252 | - | see also 9622 line | | |
| 24256 | FUBP1 | NM_003902.2 | 1335 | gactatgctcggcaactcataga | 4307 | - | see also 2659 line | | |
| 24257 | FUBP1 | NM_003902.2 | 1640 | tgcttattacgctcactattatc | 4315 | - | see also 2659 line | | |
| 24258 | G10 | NM_003910.2 | 627 | gccatcagcagagaactctatga | 4332 | - | see also 10523 line | | |
| 24259 | G10 | NM_003910.2 | 626 | agccatcagcagagaactctatg | 4331 | - | see also 10523 line | | |
| 24260 | G10 | NM_003910.2 | 703 | agcaaggatatgagaacttgtgc | 4335 | - | see also 10523 line | | |
| 24261 | GABPB2 | NM_002041.2 | 972 | ggggtcagcaagtcatcacaata | 4356 | - | see also 1317 line | | |
| 24262 | GABPB2 | NM_002041.2 | 552 | ctgatgtacacacgcaaagtaaa | 4344 | - | see also 1317 line | | |
| 24263 | GAS41 | NM_006530.2 | 491 | aacaggatggggtgaattcgaaa | 4380 | - | see also 4564 line | | |
| 24264 | GAS41 | NM_006530.2 | 494 | aggatggggtgaattcgaaataa | 4382 | - | see also 4564 line | | |
| 24265 | GAS7 | NM_003644.1 | 592 | ggcggacgaagcagaagttcacc | 4402 | - | see also 10526 line | | |
| 24266 | GAS7 | NM_003644.1 | 226 | atcctcatcgccaagcaaaaagc | 4396 | - | see also 10526 line | | |
| 24267 | GATA1 | NM_002049.2 | 997 | gaccatgcggaaggatggtattc | 4415 | - | see also 1322 line | | |
| 24268 | GATA1 | NM_002049.2 | 1130 | ggcagcggtagcgggaattgtgg | 4419 | - | see also 1322 line | | |
| 24269 | GATA1 | NM_002049.2 | 1002 | tgcggaaggatggtattcagact | 4417 | - | see also 1322 line | | |
| 24270 | GATA2 | NM_032638.3 | 1522 | accggaagatgtccaacaagtcc | 4424 | - | see also 9271 line | | |
| 24271 | GATA2 | NM_032638.3 | 1365 | gccggcacctgttgtgcaaattg | 4421 | - | see also 9271 line | | |
| 24272 | GATA3 | NM_002051.1 | 1305 | tccccaagaacagctcgtttaac | 4429 | - | see also 1324 line | | |
| 24273 | GATA3 | NM_002051.1 | 284 | gaggaggtggatgtgctttttaa | 4426 | - | see also 1324 line | | |
| 24274 | GATA4 | NM_002052.2 | 1447 | tgcggaaagaggggatccaaacc | 4433 | - | see also 1325 line | | |
| 24275 | GATA5 | NM_080473.3 | 578 | caccttcgtgtccgacttcttgg | 4435 | - | see also 10529 line | | |
| 24276 | GATA6 | NM_005257.3 | 1666 | ttgtggactctacatgaaactcc | 4439 | - | see also 3699 line | | |
| 24277 | GLI2 | NM_005270.2 | 723 | gaggctgaggtggtcatctatga | 4447 | - | see also 10531 line | | |
| 24278 | GLI2 | NM_005270.2 | 1619 | ccggttcgagcagctcaagaagg | 4452 | - | see also 10531 line | | |
| 24279 | GLI3 | NM_000168.2 | 118 | aagtgctccactcgaacagatgt | 4455 | - | see also 138 line | | |
| 24280 | GLI3 | NM_000168.2 | 2248 | ctgaccgatggaggtagtatagg | 4489 | - | see also 138 line | | |
| 24281 | GSC | NM_173849.2 | 372 | ctcccgcctcggctacaacaact | 4518 | - | transcription_regulator | transcription factor | - |

Figure 46

| | | | | | | | | | |
|---|---|---|---|---|---|---|---|---|---|
| 24282 | GSCL | NM_005315.1 | 417 | ggcgctcgaggcgcttttcgtgc | 4519 | - | transcription_regulator | transcription factor | - |
| 24283 | GSH-2 | NM_133267.1 | 997 | ctcgactccggaggattgaaatc | 4522 | - | see also 9563 line | | |
| 24284 | GSH1 | NM_145657.1 | 536 | agcgcgagttcgcttctaatatg | 4526 | - | see also 10534 line | | |
| 24285 | GSH1 | NM_145657.1 | 537 | gcgcgagttcgcttctaatatgt | 4527 | - | see also 10534 line | | |
| 24286 | GTF2A2 | NM_004492.1 | 331 | acgtacagattctgcgataatgt | 4534 | - | see also 3043 line | | |
| 24287 | GTF2A2 | NM_004492.1 | 355 | tggacttttgtactgaatgatgt | 4535 | - | see also 3043 line | | |
| 24288 | GTF2H3 | NM_001516.3 | 794 | ctgtcatcgaaatctcattgaaa | 4564 | - | see also 10536 line | | |
| 24289 | GTF2H3 | NM_001516.3 | 875 | tacgtgcgagacagcctttaaaa | 4566 | - | see also 10536 line | | |
| 24290 | GTF2H4 | NM_001517.3 | 263 | tggggtattggaccgattgtatg | 4568 | - | see also 10537 line | | |
| 24291 | GTF2H4 | NM_001517.3 | 1004 | gaggaaatctcggcgttactacc | 4576 | - | see also 10537 line | | |
| 24292 | GTF2I | NM_001518.2 | 954 | taggtggtcgtgtgatggtaaca | 4584 | - | see also 1043 line | | |
| 24293 | HAND1 | NM_004821.1 | 831 | gcccagtcgagaagaggattaaa | 4595 | - | see also 10539 line | | |
| 24294 | HAND1 | NM_004821.1 | 800 | cagcagcacgaaggttttcctcc | 4594 | - | see also 10539 line | | |
| 24295 | HCFC1 | NM_005334.1 | 5558 | gacctccggcaagattatcgagt | 4631 | - | see also 3716 line | | |
| 24296 | HCFC1 | NM_005334.1 | 5554 | ctgtgacctccggcaagattatc | 4630 | - | see also 3716 line | | |
| 24297 | HCLS1 | NM_005335.3 | 1395 | ttgatccggacgacgtaatcact | 4653 | - | see also 10541 line | | |
| 24298 | HCLS1 | NM_005335.3 | 716 | aagaagacgacgcccatagaagc | 4641 | - | see also 10541 line | | |
| 24299 | HDAC1 | NM_004964.2 | 742 | ccgctccgagacgggattgatga | 4668 | - | see also 10542 line | | |
| 24300 | HDAC1 | NM_004964.2 | 787 | aagccggtcatgtccaaagtaat | 4669 | - | see also 10542 line | | |
| 24301 | HDAC2 | NM_001527.1 | 779 | atcgtgtaatgacggtatcattc | 4698 | - | see also 1047 line | | |
| 24302 | HDAC2 | NM_001527.1 | 1018 | ctgggttgtttcaatctaacagt | 4710 | - | see also 1047 line | | |
| 24303 | HES6 | NM_018645.3 | 752 | agcctgaccacagcccaaattgc | 4724 | - | transcription_regulator | - | - |
| 24304 | HESX1 | NM_003865.1 | 725 | tgctatcctggtatcgatattag | 4730 | - | see also 10544 line | | |
| 24305 | HESX1 | NM_003865.1 | 722 | aactgctatcctggtatcgatat | 4728 | - | see also 10544 line | | |
| 24306 | HESX1 | NM_003865.1 | 750 | aagacttagctcaaaaattgaat | 4732 | - | see also 10544 line | | |
| 24307 | HEY1 | NM_012258.2 | 284 | cgccgacgagaccggatcaataa | 4741 | - | see also 5239 line | | |
| 24308 | HEY1 | NM_012258.2 | 998 | gggacggagatcggagctttta | 4752 | - | see also 5239 line | | |
| 24309 | HEY1 | NM_012258.2 | 285 | gccgacgagaccggatcaataac | 4742 | - | see also 5239 line | | |
| 24310 | HEY2 | NM_012259.1 | 226 | ggcgtcgggatcggataaataac | 4759 | - | see also 5240 line | | |
| 24311 | HEY2 | NM_012259.1 | 227 | gcgtcgggatcggataaataaca | 4760 | - | see also 5240 line | | |
| 24312 | HEYL | NM_014571.2 | 362 | ttgacttccggagcattggtttt | 4769 | - | see also 10547 line | | |
| 24313 | HEYL | NM_014571.2 | 364 | gacttccggagcattggttttcg | 4770 | - | see also 10547 line | | |
| 24314 | HHEX | NM_002729.2 | 112 | cacacccgacgccctttacatc | 4772 | - | see also 10548 line | | |
| 24315 | HHEX | NM_002729.2 | 110 | cgcacacccgacgccctttaca | 4771 | - | see also 10548 line | | |
| 24316 | HHEX | NM_002729.2 | 811 | ttgagggcgataaaagctatttt | 4778 | - | see also 10548 line | | |

935

Figure 46

| | | | | | | | |
|---|---|---|---|---|---|---|---|
| 24317 | HIF3A | NM_022462.2 | 1083 | gagtatcgtctgtgtccatttt | 4787 | - | see also 10549 line |
| 24318 | HIF3A | NM_022462.2 | 1497 | ggcagtgggagacagatttagata | 4790 | - | see also 10549 line |
| 24319 | HIRA | NM_003325.3 | 3046 | cgcacggtacctcgtaaacgaag | 4844 | - | see also 2255 line |
| 24320 | HIRA | NM_003325.3 | 256 | tggcaagccgattttcagttg | 4805 | - | see also 2255 line |
| 24321 | HIVEP3 | NM_024503.1 | 2829 | atgtggtgctcggtacaagaaaa | 4862 | - | see also 8620 line |
| 24322 | HIVEP3 | NM_024503.1 | 2657 | agccggcaatcgaattacctttg | 4855 | - | see also 8620 line |
| 24323 | HKR3 | NM_005341.1 | 1026 | gtcgaatgccccacatgtcataa | 4918 | - | see also 10552 line |
| 24324 | HKR3 | NM_005341.1 | 1954 | acgaccgggtaggagaactacaac | 4926 | - | see also 10552 line |
| 24325 | HLX1 | NM_021958.2 | 1245 | tgccgcagacgctacaaaaggaag | 4934 | - | see also 10553 line |
| 24326 | HLX1 | NM_021958.2 | 1117 | atctctagatccattaacgagg | 4930 | - | see also 10553 line |
| 24327 | HLXB9 | NM_005515.2 | 856 | gcctaagatgccgacttcaact | 4941 | - | see also 10554 line |
| 24328 | HMG20A | NM_018200.2 | 253 | gacggttccaggagagtaatga | 4942 | - | see also 7315 line |
| 24329 | HMG20A | NM_018200.2 | 1024 | cggagccgcaacacagtcttaca | 4963 | - | see also 7315 line |
| 24330 | HMG20B | NM_006339.1 | 876 | tgcggatacacccagaagaacc | 4973 | - | see also 10555 line |
| 24331 | HMG20B | NM_006339.1 | 850 | cccctgaaccggaaaaagcact | 4972 | - | see also 10555 line |
| 24332 | HMGB2 | NM_002129.2 | 282 | acccggactcttccgtccatttc | 4974 | - | see also 10556 line |
| 24333 | HMGB2 | NM_002129.2 | 283 | cccggacttccgtccattcg | 4975 | - | see also 10556 line |
| 24334 | HMGB2 | NM_002129.2 | 458 | aaggaccccaatgctcctaaaag | 4979 | - | see also 10556 line |
| 24335 | HNF4A | NM_000457.3 | 612 | gacattcgggcggaagaagattgc | 4986 | - | see also 10557 line |
| 24336 | HNF4A | NM_000457.3 | 352 | tgcggaagaaccacatgttactcc | 4983 | - | see also 10557 line |
| 24337 | HOXA1 | NM_005522.3 | 146 | acccctcgaccataggattaca | 4993 | - | see also 3856 line |
| 24338 | HOXA1 | NM_005522.3 | 387 | cagccctacgttaaatcagg | 5001 | - | see also 3856 line |
| 24339 | HOXA10 | NM_018951.2 | 1200 | gggagctcacagccaacttaat | 5021 | - | see also 10559 line |
| 24340 | HOXA10 | NM_018951.2 | 1199 | cggagctcacagccaactttaa | 5020 | - | see also 10559 line |
| 24341 | HOXA11 | NM_005523.4 | 967 | cagagacgtttacagtactact | 5036 | - | see also 3857 line |
| 24342 | HOXA11 | NM_005523.4 | 424 | ccccgcagtctgtccaatttct | 5027 | - | see also 3857 line |
| 24343 | HOXA2 | NM_006735.3 | 1315 | ccctcgacagtcccgtagatatt | 5049 | - | see also 4758 line |
| 24344 | HOXA2 | NM_006735.3 | 1317 | ctcgacagtcccgtagatatttc | 5050 | - | see also 4758 line |
| 24345 | HOXA3 | NM_030661.3 | 1012 | ccggagccggttgctatctgaac | 5057 | - | see also 10560 line |
| 24346 | HOXA3 | NM_030661.3 | 1208 | agggggcacccccgactatgacc | 5060 | - | see also 10560 line |
| 24347 | HOXA4 | NM_002141.2 | 657 | agcgccgttaacccagttataa | 5062 | - | see also 10561 line |
| 24348 | HOXA4 | NM_002141.2 | 659 | cgccgttaacccagttataacg | 5064 | - | see also 10561 line |
| 24349 | HOXA5 | NM_019102.2 | 611 | cacataagtcatgacaacacatagg | 5074 | - | see also 7708 line |
| 24350 | HOXA6 | NM_024014.2 | 150 | gacgtacacctcacttgtttct | 5079 | - | transcription_regulator | transcription factor |
| 24351 | HOXA7 | NM_006896.2 | 279 | gaccgttccggcttatacaatg | 5083 | - | see also 4827 line |
| 24352 | HOXA7 | NM_006896.2 | 375 | ctgcgcctcctacgaccaaaaca | 5085 | - | see also 4827 line |

Figure 46

| | | | | | | | | | |
|---|---|---|---|---|---|---|---|---|---|
| 24353 | HOXB1 | NM_002144.2 | 18 | taggatgaactccttcttagagt | 5093 | - | see also 10563 line | | |
| 24354 | HOXB1 | NM_002144.2 | 482 | tcctctccgaggacaaggaaaca | 5095 | - | see also 10563 line | | |
| 24355 | HOXB13 | NM_006361.2 | 355 | agctcccgtgccttatggttact | 5099 | - | see also 10564 line | | |
| 24356 | HOXB13 | NM_006361.2 | 828 | gggagtatgcggctaacaagttc | 5102 | - | see also 10564 line | | |
| 24357 | HOXB2 | NM_002145.2 | 397 | cccgagttcccttggatgaaaga | 5110 | - | see also 10565 line | | |
| 24358 | HOXB2 | NM_002145.2 | 229 | atcaaggagtcgacattaattcc | 5107 | - | see also 10565 line | | |
| 24359 | HOXB3 | NM_002146.3 | 2123 | atccaagaagcgcccaaattaac | 5117 | - | see also 10566 line | | |
| 24360 | HOXB3 | NM_002146.3 | 2124 | tccaagaagcgcccaaattaaca | 5118 | - | see also 10566 line | | |
| 24361 | HOXB3 | NM_002146.3 | 897 | ctctcttcggaggctattcctcg | 5113 | - | see also 10566 line | | |
| 24362 | HOXB4 | NM_024015.2 | 75 | ggctatgagttcttttttgatca | 5119 | - | see also 8557 line | | |
| 24363 | HOXB6 | NM_018952.3 | 393 | gccagctaccgctctattcgtcg | 5130 | - | see also 10568 line | | |
| 24364 | HOXB6 | NM_018952.3 | 421 | tgcggacccgctgagacattacc | 5132 | - | see also 10568 line | | |
| 24365 | HOXB8 | NM_024016.2 | 388 | gtcgcaaatccaggagttctacc | 5134 | - | transcription_regulator | transcription factor | - |
| 24366 | HOXB9 | NM_024017.3 | 542 | acccggctacggggacaataaaa | 5149 | - | see also 8558 line | | |
| 24367 | HOXB9 | NM_024017.3 | 541 | gacccggctacggggacaataaa | 5148 | - | see also 8558 line | | |
| 24368 | HOXC10 | NM_017409.2 | 167 | gccggagcgcaggcatgtatatg | 5155 | - | see also 10570 line | | |
| 24369 | HOXC10 | NM_017409.2 | 1085 | tccgggaactgacctccaatttt | 5161 | - | see also 10570 line | | |
| 24370 | HOXC11 | NM_014212.2 | 801 | agcgctgcccttattcgaaattc | 5169 | - | see also 10571 line | | |
| 24371 | HOXC11 | NM_014212.2 | 802 | gcgctgcccttattcgaaattcc | 5170 | - | see also 10571 line | | |
| 24372 | HOXC12 | NM_173860.1 | 614 | cgccctggtacccgatcaacagc | 5174 | - | see also 10123 line | | |
| 24373 | HOXC12 | NM_173860.1 | 739 | ctctcagaccgcttgaatcttag | 5175 | - | see also 10123 line | | |
| 24374 | HOXC13 | NM_017410.2 | 154 | gagccttatgtacgtctatgagg | 5178 | - | see also 6983 line | | |
| 24375 | HOXC13 | NM_017410.2 | 1012 | ctctgagcgccaggtaaccatct | 5179 | - | see also 6983 line | | |
| 24376 | HOXC6 | NM_004503.2 | 328 | tggatctaattccttttaccagg | 5185 | - | see also 3054 line | | |
| 24377 | HOXC8 | NM_022658.2 | 629 | ggcgcagtggacggcaaacttac | 5194 | - | see also 8424 line | | |
| 24378 | HOXC8 | NM_022658.2 | 336 | cgcttcgcaccacgttcaagact | 5188 | - | see also 8424 line | | |
| 24379 | HOXC9 | NM_006897.1 | 306 | ctctcagtcgtccgtggtatatc | 5199 | - | see also 4829 line | | |
| 24380 | HOXC9 | NM_006897.1 | 111 | gcccatcagtaactattacgtgg | 5196 | - | see also 4829 line | | |
| 24381 | HOXD1 | NM_024501.1 | 910 | cagcgcgatccgcacgaatttca | 5204 | - | see also 10573 line | | |
| 24382 | HOXD1 | NM_024501.1 | 1150 | aacccccactaagtttatcaaga | 5209 | - | see also 10573 line | | |
| 24383 | HOXD10 | NM_002148.2 | 278 | tccttatatacctcaagtagaca | 5218 | - | see also 1378 line | | |
| 24384 | HOXD13 | NM_000523.2 | 964 | aacgagtatgccattaacaaatt | 5234 | - | see also 10574 line | | |
| 24385 | HOXD13 | NM_000523.2 | 836 | aggggtcccacttttggaaatct | 5232 | - | see also 10574 line | | |
| 24386 | HOXD3 | NM_006898.4 | 228 | cagaaggctgcttactatgaaaa | 5236 | - | see also 10575 line | | |
| 24387 | HOXD3 | NM_006898.4 | 1444 | tgccggaggctcccaaactgacg | 5238 | - | see also 10575 line | | |
| 24388 | HOXD3 | NM_006898.4 | 267 | ggctatggctacagcaaaactac | 5237 | - | see also 10575 line | | |

Figure 46

| | | | | | | | transcription_regulator | transcription factor | |
|---|---|---|---|---|---|---|---|---|---|
| 24389 | HOXD4 | NM_014621.2 | 300 | ccctcctgcgaggagtattgc | 5239 | - | see also 24389 line | | - |
| 24390 | HOXD4 | NM_014621.2 | 303 | tccgtgcgaggagtatttgcagg | 5240 | - | see also 10576 line | | - |
| 24391 | HOXD8 | NM_019558.2 | 1018 | gcgaagttttacggatacgataa | 5242 | - | see also 10576 line | | - |
| 24392 | HOXD8 | NM_019558.2 | 1078 | gccgagctgtacaatatcctga | 5246 | - | see also 5537 line | | - |
| 24393 | HOXD9 | NM_014213.2 | 467 | tactacgtggactcgcttatagg | 5255 | - | see also 10577 line | | - |
| 24394 | HOZFP | NM_152995.3 | 1329 | accgaggtccctgtgaaacatgt | 5288 | - | see also 10577 line | | - |
| 24395 | HOZFP | NM_152995.3 | 1521 | gacggactttaggatgtagaaac | 5300 | - | see also 10578 line | | - |
| 24396 | HR | NM_005144.2 | 3767 | ggcttctaccgcggtcaaactct | 5326 | - | see also 10578 line | | - |
| 24397 | HR | NM_005144.2 | 2087 | cagcaagggcttttactacaagg | 5317 | - | see also 10579 line | | - |
| 24398 | HSF1 | NM_005526.1 | 1137 | cccgacgccctcattgactcc | 5332 | - | see also 10579 line | | - |
| 24399 | HSF1 | NM_005526.1 | 1312 | cagtgaccacttgatgctatgg | 5333 | - | see also 3063 line | | - |
| 24400 | HSF2 | NM_004506.2 | 1470 | ctgttgaacaggcgagtacaaca | 5363 | - | see also 3063 line | | - |
| 24401 | HSF2 | NM_004506.2 | 563 | aaggaggtgtcagaattacgagc | 5347 | - | see also 10581 line | | - |
| 24402 | HSFY | NM_033108.1 | 131 | tccccaaagatgaattaactgc | 5365 | - | see also 10582 line | | - |
| 24403 | HSRNAFEV | NM_017521.1 | 607 | agccctgctgatcaacactgtac | 5383 | - | | | - |
| 24404 | HSRNAFEV | NM_017521.1 | 608 | gccccctgctcaacatgtacc | 5384 | - | see also 10582 line | | - |
| 24405 | HTLF | NM_002158.2 | 574 | aagccccatactccttagtct | 5399 | - | see also 10583 line | | - |
| 24406 | HTLF | NM_002158.2 | 1081 | cccagtgctacgattacaaga | 5412 | - | see also 10583 line | | - |
| 24407 | ILF1 | NM_004514.2 | 519 | gggcaagaacggggtattcgtgg | 5423 | - | see also 10584 line | | - |
| 24408 | ILF1 | NM_004514.2 | 1006 | ctcaccctgaacggatttatac | 5433 | - | see also 10584 line | | - |
| 24409 | IPF1 | NM_000209.1 | 192 | ccccctgcgtgcctgtacatgg | 5447 | - | see also 10585 line | | - |
| 24410 | IRF1 | NM_002198.1 | 926 | gtcggagtggcagccaacaaacg | 5457 | - | see also 1402 line | | - |
| 24411 | IRF1 | NM_002198.1 | 229 | ggctagaatgcagattaattcc | 5451 | - | see also 1402 line | | - |
| 24412 | IRF2 | NM_002199.2 | 650 | atgcggtcctgacttcaactata | 5466 | - | see also 10587 line | | - |
| 24413 | IRF2 | NM_002199.2 | 420 | cagatgcgccatgaattccttgc | 5462 | - | see also 10587 line | | - |
| 24414 | IRF3 | NM_001571.1 | 345 | ctcacgacccacataaaaatctac | 5478 | - | see also 10588 line | | - |
| 24415 | IRF3 | NM_001571.1 | 342 | accctcacgaccccacataaaatc | 5477 | - | see also 10588 line | | - |
| 24416 | IRF3 | NM_001571.1 | 341 | gaccctcacgaccccacataaaat | 5476 | - | see also 10588 line | | - |
| 24417 | IRF4 | NM_002460.1 | 1435 | atccgccattcctctattcaaga | 5496 | - | see also 10589 line | | - |
| 24418 | IRF4 | NM_002460.1 | 1069 | gacgggctattgcgaaaagact | 5492 | - | see also 10589 line | | - |
| 24419 | IRF4 | NM_002460.1 | 459 | ctcagaccgtacaaagtgtaca | 5483 | - | see also 10589 line | | - |
| 24420 | IRF5 | NM_002200.3 | 1306 | cccaccacccttcgagatcttct | 5507 | - | see also 10590 line | | - |
| 24421 | IRF5 | NM_002200.3 | 1441 | atcttggtcagctgatagtatcc | 5508 | - | see also 10590 line | | - |
| 24422 | IRF5 | NM_002200.3 | 1269 | atgagctcatcctgttccaaaag | 5506 | - | see also 10590 line | | - |

Figure 46

| | | | | | | | | | |
|---|---|---|---|---|---|---|---|---|---|
| 24423 | IRF6 | NM_006147.2 | 1491 | ttcacacgatcctttgatagtgg | 5527 | - | see also 10591 line | | |
| 24424 | IRF6 | NM_006147.2 | 1363 | caccgtttgagatctacttatgc | 5523 | - | see also 10591 line | | |
| 24425 | IRF7 | NM_001572.2 | 1213 | cccgagctgcacgttcctatacg | 5531 | - | see also 10592 line | | |
| 24426 | IRF7 | NM_001572.2 | 1212 | acccgagctgcacgttcctatac | 5530 | - | see also 10592 line | | |
| 24427 | IRX1 | NM_024337.1 | 830 | ggcactgaatccacagtatgaat | 5535 | - | see also 10593 line | | |
| 24428 | IRX1 | NM_024337.1 | 887 | ggcacaaccaggagcctattatc | 5536 | - | see also 10593 line | | |
| 24429 | IRX3 | NM_024336.1 | 425 | cccccagctgggataccaataca | 5538 | - | see also 10594 line | | |
| 24430 | IRX3 | NM_024336.1 | 427 | cccagctgggataccaatacatc | 5539 | - | see also 10594 line | | |
| 24431 | IRX4 | NM_016358.1 | 122 | gtcctacccgcagtttggatacc | 5542 | - | transcription_regulator | transcription factor | - |
| 24432 | IRX4 | NM_016358.1 | 514 | acccctatgacaggtatggaacc | 5543 | - | see also 24431 line | | |
| 24433 | IRX4 | NM_016358.1 | 1409 | cccggtaaccagtctcagaaact | 5544 | - | see also 24431 line | | |
| 24434 | ISGF3G | NM_006084.3 | 49 | ggcacgctgcacccgaaaactcc | 5559 | - | see also 10596 line | | |
| 24435 | ISGF3G | NM_006084.3 | 1106 | tccagccatactccacagaatct | 5562 | - | see also 10596 line | | |
| 24436 | ISL1 | NM_002202.1 | 1186 | aaggaggaccgggctctaattcc | 5586 | - | see also 1403 line | | |
| 24437 | ISL1 | NM_002202.1 | 304 | atcagattcacgatcagtatatt | 5567 | - | see also 1403 line | | |
| 24438 | ISL2 | NM_145805.1 | 114 | tgctatgggtgatcattccaaga | 5592 | - | see also 9801 line | | |
| 24439 | JUN | NM_002228.2 | 1927 | gtgggtgccaactcatgctaacg | 5607 | - | see also 1424 line | | |
| 24440 | JUN | NM_002228.2 | 1042 | gcggaccttatggctacagtaac | 5601 | - | see also 1424 line | | |
| 24441 | JUNB | NM_002229.1 | 1020 | cgccggtgtcccccatcaacatg | 5610 | - | transcription_regulator | AP-1(JunB/Fra-1 heterodimers) | - |
| 24442 | JUNB | NM_002229.1 | 1021 | gccggtgtcccccatcaacatgg | 5611 | - | see also 24441 line | | |
| 24443 | JUNB | NM_002229.1 | 441 | gcggtggcggcagctactttct | 5609 | - | see also 24441 line | | |
| 24444 | JUND | NM_005354.2 | 1053 | ctcgcgcctggaagagaaagtga | 5612 | - | transcription_regulator | AP-1(jun-D/c-fos heterodimer) | - |
| 24445 | KIAA0669 | NM_014779.1 | 1493 | ttcccgttttcgcgtgatcaagc | 5620 | - | see also 5951 line | | |
| 24446 | KIAA0669 | NM_014779.1 | 2781 | gggcaagtcgacgatactagaag | 5638 | - | see also 5951 line | | |
| 24447 | KIAA0863 | NM_014913.1 | 1313 | gccagtcggacctgtcaataagt | 5682 | - | see also 10601 line | | |
| 24448 | KIAA0863 | NM_014913.1 | 772 | agcaaccgaaaactaacgatact | 5666 | - | see also 10601 line | | |
| 24449 | KIAA1041 | NM_014947.1 | 1683 | tgcgacacttgatatgctaaaag | 5778 | - | see also 6138 line | | |
| 24450 | KIAA1041 | NM_014947.1 | 1677 | ttggtatgcgacacttgatatgc | 5776 | - | see also 6138 line | | |
| 24451 | KIAA1443 | NM_020834.1 | 593 | agcctacccagacgaaaggattg | 5794 | - | see also 10603 line | | |
| 24452 | KIAA1443 | NM_020834.1 | 163 | gccagcggtctcagtagttcacc | 5792 | - | see also 10603 line | | |
| 24453 | KIAA1474 | NM_020856.1 | 2479 | tccggctacgggacttatccaaa | 5843 | - | see also 7998 line | | |
| 24454 | KIAA1474 | NM_020856.1 | 1955 | gacatctgccgtcgtatcattca | 5828 | - | see also 7998 line | | |
| 24455 | KIAA1618 | NM_020954.1 | 1567 | gtcaaccgctgtctgttcataaa | 5869 | - | see also 10605 line | | |
| 24456 | KIAA1618 | NM_020954.1 | 2745 | agccttatctgccgagattgtct | 5895 | - | see also 10605 line | | |

Figure 46

| | | | | | | | |
|---|---|---|---|---|---|---|---|
| 24457 | KIAA1618 | NM_020954.1 | 1937 | taccattgccggacggaaaaagc | 5878 | - | see also 10605 line |
| 24458 | KLF12 | NM_007249.3 | 334 | cacaactatcccgatatggaagc | 5915 | - | see also 5050 line |
| 24459 | KLF3 | NM_016531.2 | 547 | gccctctcgcggcatggaatacg | 5956 | - | see also 6907 line |
| 24460 | KLF3 | NM_016531.2 | 1064 | ctcggtctgatgaactaacaaga | 5968 | - | see also 6907 line |
| 24461 | KLF4 | NM_004235.3 | 1114 | cgcgcacgtgccccaagatcaag | 5974 | - | see also 10608 line |
| 24462 | KLF4 | NM_004235.3 | 1481 | cgccacccacacttgtgattacg | 5978 | - | see also 10608 line |
| 24463 | KLF7 | NM_003709.1 | 524 | cccgtgcattgaggaaagcttcc | 5989 | - | see also 2529 line |
| 24464 | KLF7 | NM_003709.1 | 973 | gggttcaccgctgtcagtttaac | 5999 | - | see also 2529 line |
| 24465 | L3MBTL | NM_015478.4 | 1970 | gacgccctccgaagtatcgaaag | 6013 | - | see also 10609 line |
| 24466 | L3MBTL | NM_015478.4 | 1951 | cgccatcacggccgaattggacg | 6012 | - | see also 10609 line |
| 24467 | LASS2 | NM_013384.3 | 739 | ccgagctgggactctaatcatgg | 6031 | - | see also 5416 line |
| 24468 | LASS2 | NM_013384.3 | 585 | cccagtattggtactacatgatt | 6026 | - | see also 5416 line |
| 24469 | LASS3 | NM_178842.2 | 638 | aagagacagttcgaaaggttaca | 6054 | - | see also 10611 line |
| 24470 | LASS3 | NM_178842.2 | 508 | cacgatggactcgtctttgtaaa | 6051 | - | see also 10611 line |
| 24471 | LASS4 | NM_024552.1 | 1024 | ctgtaagatggtcaactacatgc | 6092 | - | see also 10612 line |
| 24472 | LASS4 | NM_024552.1 | 1257 | tgctctatagcttcatgaagaag | 6094 | - | see also 10612 line |
| 24473 | LASS5 | NM_147190.1 | 235 | ggctgctcttcgagcgatttatt | 6098 | - | see also 10613 line |
| 24474 | LASS5 | NM_147190.1 | 901 | cacgactaggaatctatccattc | 6124 | - | see also 10613 line |
| 24475 | LBX1 | NM_006562.3 | 271 | gccgtctgtgcggagaagttact | 6131 | - | see also 10614 line |
| 24476 | LBX1 | NM_006562.3 | 466 | cgcgacggtatgaccatctttgg | 6133 | - | see also 10614 line |
| 24477 | LBX1 | NM_006562.3 | 407 | agctcgccagcaagacgtttaag | 6132 | - | see also 10614 line |
| 24478 | LHX1 | NM_005568.2 | 1163 | tccggtgtttcggtaccaaatgc | 6137 | - | see also 10615 line |
| 24479 | LHX1 | NM_005568.2 | 1162 | ttccggtgtttcggtaccaaatg | 6136 | - | see also 10615 line |
| 24480 | LHX2 | NM_004789.3 | 1464 | ctgcgctaagctgcaacgaaaac | 6148 | - | see also 3321 line |
| 24481 | LHX2 | NM_004789.3 | 1162 | cacgtgcaccacgtgtaacaaga | 6145 | - | see also 3321 line |
| 24482 | LHX3 | NM_014564.2 | 644 | gacgctgaagagcgcttacaaca | 6151 | - | transcription_regulator - pituitary dwarfism |
| 24483 | LHX3 | NM_014564.2 | 646 | cgctgaagagcgcttacaacacc | 6152 | - | see also 24482 line |
| 24484 | LHX3 | NM_014564.2 | 346 | gccgaggggagagcgtttactgc | 6150 | - | see also 24482 line |
| 24485 | LHX4 | NM_033343.2 | 1329 | taggctatcccgactttccaact | 6170 | - | see also 9409 line |
| 24486 | LHX5 | NM_022363.2 | 1588 | ccccaggttcaccgacatgatct | 6178 | - | see also 10616 line |
| 24487 | LHX5 | NM_022363.2 | 822 | aagtctttcacctcaactgtttc | 6176 | - | see also 10616 line |
| 24488 | LHX6 | NM_014368.2 | 599 | atccactacgacaccatgattga | 6180 | - | see also 10617 line |
| 24489 | LHX6 | NM_014368.2 | 1076 | cacctcaaagccgatatggatgg | 6183 | - | see also 10617 line |
| 24490 | LHX9 | NM_020204.2 | 1503 | cccggcactgcgaccactttaac | 6193 | - | see also 10618 line |
| 24491 | LHX9 | NM_020204.2 | 1240 | agcgcatgcgaacctctttcaag | 6190 | - | see also 10618 line |

Figure 46

| | | | | | | | | | |
|---|---|---|---|---|---|---|---|---|---|
| 24492 | LMO1 | NM_002315.1 | 934 | agctcaatggcacctttgaatcc | 6203 | - | transcription_regulator | transcription factor | leukemia、lymphoma |
| 24493 | LMO4 | NM_006769.2 | 1148 | tcccgggagatcggtttcactac | 6211 | - | see also 4775 line | | |
| 24494 | LMX1A | NM_177398.1 | 808 | aacgtccgagaaccatcttgaca | 6220 | - | see also 10620 line | | |
| 24495 | LMX1A | NM_177398.1 | 1191 | gaccacatgcacccttatggtgc | 6223 | - | see also 10620 line | | |
| 24496 | LMX1B | NM_002316.1 | 986 | tcgacagcgatacctccttaacc | 6227 | - | see also 10621 line | | |
| 24497 | LOC51270 | NM_016521.2 | 900 | atctccgacgacaaatcagaata | 6230 | - | see also 10622 line | | |
| 24498 | LOC51270 | NM_016521.2 | 902 | ctccgacgacaaatcagaatatc | 6231 | - | see also 10622 line | | |
| 24499 | LOC51270 | NM_016521.2 | 903 | tccgacgacaaatcagaatatct | 6232 | - | see also 10622 line | | |
| 24500 | MAD | NM_002357.1 | 615 | ctccgacagggaagaaatcgacg | 6246 | - | see also 1523 line | | |
| 24501 | MAD | NM_002357.1 | 446 | aagccaaattgcacataaagaaa | 6243 | - | see also 1523 line | | |
| 24502 | MADH3 | NM_005902.1 | 986 | gcctcagtgacagcgctattttt | 6261 | - | see also 10624 line | | |
| 24503 | MADH3 | NM_005902.1 | 939 | aagaggcgtgcggctctactaca | 6260 | - | see also 10624 line | | |
| 24504 | MADH4 | NM_005359.3 | 1717 | ttcgtcgcttatgcatactcagg | 6305 | - | see also 3736 line | | |
| 24505 | MADH4 | NM_005359.3 | 235 | tggacaatatgtctattacgaat | 6264 | - | see also 3736 line | | |
| 24506 | MAX | NM_197957.1 | 210 | gagcaaccgaggtttcaatctgc | 6314 | - | see also 10627 line | | |
| 24507 | MBD1 | NM_002384.1 | 1542 | cacccgtgatcacggagattttc | 6340 | - | see also 10628 line | | |
| 24508 | MBD1 | NM_002384.1 | 1543 | acccgtgatcacggagattttca | 6341 | - | see also 10628 line | | |
| 24509 | MDS1 | NM_004991.1 | 656 | ctgaaagaccccagttatggatg | 6353 | - | see also 10629 line | | |
| 24510 | MDS1 | NM_004991.1 | 421 | tccctccctcaatattcaagagc | 6350 | - | see also 10629 line | | |
| 24511 | MEF2A | NM_005587.1 | 1618 | atcaagtccgaaccgatttcacc | 6385 | - | see also 10630 line | | |
| 24512 | MEF2A | NM_005587.1 | 1635 | ttcacctcctcgggatcgtatga | 6386 | - | see also 10630 line | | |
| 24513 | MEF2A | NM_005587.1 | 922 | gccagctcaacgttaacagattc | 6373 | - | see also 10630 line | | |
| 24514 | MEF2B | NM_005919.1 | 853 | gcccagcccagatgtggtatacg | 6390 | - | transcription_regulator | translation regulator | - |
| 24515 | MEF2B | NM_005919.1 | 630 | accgtgtgctgctgaagtacaca | 6389 | - | see also 24514 line | | |
| 24516 | MEF2C | NM_002397.2 | 521 | ctgtgagattgcgctgatcatct | 6392 | - | see also 1542 line | | |
| 24517 | MEF2C | NM_002397.2 | 1785 | gtcaagcgcatgcgactttctga | 6408 | - | see also 1542 line | | |
| 24518 | MEIS1 | NM_002398.1 | 1124 | gcccatgggaggtttcgtaatgg | 6433 | - | see also 1545 line | | |
| 24519 | MEIS1 | NM_002398.1 | 429 | gccgtgttcgccaaacagattcg | 6418 | - | see also 1545 line | | |
| 24520 | MEIS1 | NM_002398.1 | 428 | agccgtgttcgccaaacagattc | 6417 | - | see also 1545 line | | |
| 24521 | MEIS2 | NM_002399.2 | 949 | atcgacctcgtcattgatgaaag | 6440 | - | see also 1556 line | | |
| 24522 | MEIS2 | NM_002399.2 | 619 | cgggacaaggacgcgatctatgg | 6436 | - | see also 1556 line | | |
| 24523 | MEOX1 | NM_004527.2 | 439 | ttgggagcactgccaatgagaca | 6464 | - | see also 10634 line | | |
| 24524 | MEOX1 | NM_004527.2 | 355 | cggcaggggttccaaggaaatg | 6462 | - | see also 10634 line | | |
| 24525 | MEOX2 | NM_005924.2 | 1024 | aacaggggactctatagcaaatg | 6473 | - | see also 10635 line | | |
| 24526 | MEOX2 | NM_005924.2 | 453 | ggcacctcccgcagatgtcttcc | 6470 | - | see also 10635 line | | |

Figure 46

| 24527 | MIXL1 | NM_031944.1 | 425 | ggcgtcagagtgggaaatccttc | 6475 | - | transcription_regulator | - | | - |
|---|---|---|---|---|---|---|---|---|---|---|
| 24528 | MIXL1 | NM_031944.1 | 626 | ctctctctgaagacattggttca | 6476 | - | see also 24527 line | | | |
| 24529 | MLL | NM_005933.1 | 5924 | aacgacatcgggatttgatcaaa | 6604 | - | see also 10636 line | | | |
| 24530 | MLL | NM_005933.1 | 6182 | ctcgcaagcgctgtgtatataca | 6615 | - | see also 10636 line | | | |
| 24531 | MLL2 | NM_003482.1 | 5446 | ggcgacatagcccgtaagactga | 6796 | - | see also 10637 line | | | |
| 24532 | MLL2 | NM_003482.1 | 2785 | atcgttaacgagatctctaatct | 6775 | - | see also 10637 line | | | |
| 24533 | MLL4 | NM_014727.1 | 3225 | agctcggcgctgcgtcaaacagc | 6910 | - | see also 10638 line | | | |
| 24534 | MLL4 | NM_014727.1 | 3506 | accgcgtccgtgtggattttaag | 6913 | - | see also 10638 line | | | |
| 24535 | MLLT10 | NM_004641.1 | 563 | ctgttccgcatgatcgttataat | 6960 | - | see also 3155 line | | | |
| 24536 | MLLT10 | NM_004641.1 | 3034 | cacacaaccgtaccacctaatgc | 7015 | - | see also 3155 line | | | |
| 24537 | MLLT2 | NM_005935.1 | 3598 | atggcgatgtttcgttgtaaaaa | 7065 | - | see also 10640 line | | | |
| 24538 | MLLT2 | NM_005935.1 | 575 | agctgtctagtcgaatacagaac | 7026 | - | see also 10640 line | | | |
| 24539 | MLLT2 | NM_005935.1 | 1370 | atcgacagcagacctttgaaaaa | 7041 | - | see also 10640 line | | | |
| 24540 | MLLT7 | NM_005938.1 | 565 | tccgtactgtaccctacttcaag | 7079 | - | see also 4146 line | | | |
| 24541 | MNT | NM_020310.1 | 590 | ccccggactcagcattaaggagc | 7087 | - | see also 10642 line | | | |
| 24542 | MORF4 | NM_006792.2 | 463 | gccgaaattcttgcagattgtcc | 7090 | - | transcription_regulator | - | | - |
| 24543 | MORF4 | NM_006792.2 | 424 | ggcacccagctactcaacaaatt | 7089 | - | see also 24542 line | | | |
| 24544 | MSC | NM_005098.2 | 797 | tccgcagccaacagactatgtgg | 7092 | - | see also 10643 line | | | |
| 24545 | MSC | NM_005098.2 | 847 | cacttgatgggattattcgttaa | 7095 | - | see also 10643 line | | | |
| 24546 | MSX1 | NM_002448.1 | 589 | ctcctcaagctgccagaagatgc | 7096 | - | see also 10644 line | | | |
| 24547 | MSX2 | NM_002449.3 | 368 | tcggtcaagtcggaaaattcaga | 7101 | - | see also 10645 line | | | |
| 24548 | MSX2 | NM_002449.3 | 374 | aagtcggaaaattcagaagatgg | 7102 | - | see also 10645 line | | | |
| 24549 | MTA1 | NM_004689.2 | 1330 | aggaccaaaccgcagtaacatga | 7123 | - | see also 3218 line | | | |
| 24550 | MTA1 | NM_004689.2 | 29 | gtcggagactacgtctactttga | 7106 | - | see also 3218 line | | | |
| 24551 | MTA2 | NM_004739.2 | 1822 | cggccttatgctcctatcaatgc | 7157 | - | see also 3269 line | | | |
| 24552 | MTF1 | NM_005955.1 | 495 | aagcggtaccaatgtacctttga | 7181 | - | see also 4156 line | | | |
| 24553 | MTF1 | NM_005955.1 | 487 | aagaagtaaagcggtaccaatgt | 7179 | - | see also 4156 line | | | |
| 24554 | MYBL2 | NM_002466.2 | 900 | ccctccgtccctcctaccataaa | 7219 | - | see also 10648 line | | | |
| 24555 | MYBL2 | NM_002466.2 | 1766 | cacgccaaccccgttcaagaacg | 7225 | - | see also 10648 line | | | |
| 24556 | MYC | NM_002467.2 | 432 | ctcggtgcagccgtatttctact | 7229 | - | see also 1586 line | | | |
| 24557 | MYCL2 | NM_005377.1 | 967 | gagcgcaagagacggaatgatca | 7246 | - | see also 10650 line | | | |
| 24558 | MYCL2 | NM_005377.1 | 1077 | cacggaatacttacatgaactgg | 7252 | - | see also 10650 line | | | |
| 24559 | MYCN | NM_005378.3 | 1358 | accacgtgccggagttggtaaag | 7261 | - | see also 10651 line | | | |
| 24560 | MYCN | NM_005378.3 | 188 | acccagacctcgagtttgactcg | 7256 | - | see also 10651 line | | | |
| 24561 | MYCN | NM_005378.3 | 228 | cccggacgaagatgacttctact | 7257 | - | see also 10651 line | | | |

Figure 46

| | | | | | | | |
|---|---|---|---|---|---|---|---|
| 24562 | MYF6 | NM_002469.1 | 454 | tgcggagcgccatcagctatatt | 7264 | - | see also 10652 line |
| 24563 | MYF6 | NM_002469.1 | 642 | ctcgtgataacggctaaggaagg | 7269 | - | see also 10652 line |
| 24564 | MYNN | NM_018657.2 | 642 | ggcgcgttagcgaaaaagtcatc | 7285 | - | see also 7538 line |
| 24565 | MYNN | NM_018657.2 | 573 | ctgcgagattataataatcgaga | 7283 | - | see also 7538 line |
| 24566 | MYOG | NM_002479.2 | 418 | gcgcagtgccatccagtacatcg | 7326 | - | see also 10654 line |
| 24567 | MYOG | NM_002479.2 | 253 | gtgggcgtgtaaggtgtgtaaga | 7325 | - | see also 10654 line |
| 24568 | MYST2 | NM_007067.3 | 638 | agcgccgtcgcttccatgaaagc | 7333 | - | see also 10655 line |
| 24569 | MYST2 | NM_007067.3 | 801 | gagccgggataagcagatagaag | 7342 | - | see also 10655 line |
| 24570 | MYT1 | NM_004535.1 | 1759 | ctgtccccacaaggataggatcc | 7363 | - | see also 3089 line |
| 24571 | MYT1 | NM_004535.1 | 1627 | cactgtgcggaagagttactaca | 7358 | - | see also 3089 line |
| 24572 | NEUROG1 | NM_006161.2 | 862 | cccagccgcctccgaagacttca | 7385 | - | transcription_regulator translation regulator |- |
| 24573 | NEUROG1 | NM_006161.2 | 941 | cacacaacgccctgtttcattcc | 7387 | - | see also 24572 line |
| 24574 | NEUROG1 | NM_006161.2 | 939 | tccacacaacgccctgtttcatt | 7386 | - | see also 24572 line |
| 24575 | NFAT5 | NM_006599.2 | 3678 | gagcagcttagtgcagatatttt | 7471 | - | see also 4634 line |
| 24576 | NFATC1 | NM_006162.3 | 2463 | ccccgttcacgtcagtttctacg | 7534 | - | see also 10658 line |
| 24577 | NFATC1 | NM_006162.3 | 2432 | tcccgccatttcggaatcagagg | 7531 | - | see also 10658 line |
| 24578 | NFATC2 | NM_012340.2 | 1837 | cacgcgggtgagactggttttcc | 7561 | - | see also 10659 line |
| 24579 | NFATC2 | NM_012340.2 | 391 | acccgcatacccgatgatgtcc | 7548 | - | see also 10659 line |
| 24580 | NFATC3 | NM_004555.2 | 2928 | ttcgtctcagttacaacctatta | 7645 | - | see also 10660 line |
| 24581 | NFATC3 | NM_004555.2 | 235 | cacgacgagctcgacttcaaact | 7582 | - | see also 10660 line |
| 24582 | NFATC4 | NM_004554.3 | 1627 | tggcggtcaccccgtagtcaagc | 7670 | - | see also 3100 line |
| 24583 | NFATC4 | NM_004554.3 | 705 | gggacggctctcctagagattac | 7668 | - | see also 3100 line |
| 24584 | NFE2 | NM_006163.1 | 983 | ttcctacggacaagattgtcaac | 7696 | - | see also 4264 line |
| 24585 | NFE2 | NM_006163.1 | 1082 | tccgacgacggggcaaaaacaag | 7699 | - | see also 4264 line |
| 24586 | NFE2L1 | NM_003204.1 | 2786 | ccctactcgcccagtcagtatgc | 7729 | - | see also 2167 line |
| 24587 | NFE2L1 | NM_003204.1 | 1155 | ggcgtgaggtttttgactatagt | 7715 | - | see also 2167 line |
| 24588 | NFE2L2 | NM_006164.2 | 324 | ttcgctcagttacaactagatga | 7735 | - | see also 4266 line |
| 24589 | NFE2L2 | NM_006164.2 | 1615 | tacgtaggaggggtaagaataaa | 7762 | - | see also 4266 line |
| 24590 | NFE2L3 | NM_004289.4 | 77 | tccgcgtagacctagatctttac | 7768 | - | see also 10664 line |
| 24591 | NFE2L3 | NM_004289.4 | 76 | ctccgcgtagacctagatcttta | 7767 | - | see also 10664 line |
| 24592 | NFIA | NM_005595.1 | 272 | aggcccgaaaacgaaaatacttc | 7827 | - | see also 3883 line |
| 24593 | NFIA | NM_005595.1 | 423 | acccgaatatcgagaggattttg | 7835 | - | see also 3883 line |
| 24594 | NFIB | NM_005596.1 | 944 | aagccaaccatactatcatgaca | 7881 | - | see also 3886 line |
| 24595 | NFIB | NM_005596.1 | 280 | tccgtgcaattgcctatacttgg | 7858 | - | see also 3886 line |
| 24596 | NFIC | NM_005597.1 | 1231 | caggacccgctcaaagatcttgt | 7894 | - | see also 10667 line |
| 24597 | NFIC | NM_005597.1 | 1128 | cccgtcctccgctctgcatttcc | 7893 | - | see also 10667 line |

943

Figure 46

| | | | | | | | |
|---|---|---|---|---|---|---|---|
| 24598 | NFIL3 | NM_005384.1 | 715 | ccctcgatggtcaagtagttg | 7906 | - | see also 10668 line |
| 24599 | NFIL3 | NM_005384.1 | 932 | cagcgtccatctatcaaaactat | 7917 | - | see also 10668 line |
| 24600 | NFIL3 | NM_005384.1 | 766 | agctcgctgtccgatgtttcaga | 7912 | - | see also 10668 line |
| 24601 | NFKB1 | NM_003998.2 | 947 | ggcgtgtaaagggctataatc | 7951 | - | see also 2724 line |
| 24602 | NFKB1 | NM_003998.2 | 2409 | cccaacgggacggtctgaatgc | 7981 | - | see also 2724 line |
| 24603 | NFKB2 | NM_002502.2 | 1055 | aagattgagcggcctgaacagt | 8024 | - | see also 10670 line |
| 24604 | NFKB2 | NM_002502.2 | 305 | cagagaggcttccgatttcgata | 8009 | - | see also 10670 line |
| 24605 | NFX1 | NM_002504.3 | 2018 | tgctctcgcacatcagttattc | 8064 | - | see also 1613 line |
| 24606 | NFX1 | NM_002504.3 | 1667 | acctcccgagatgtgttatgtgg | 8056 | - | see also 1613 line |
| 24607 | NFYA | NM_002505.3 | 669 | gggcagaccatcgtctatcaacc | 8111 | - | see also 10672 line |
| 24608 | NFYA | NM_002505.3 | 203 | tagttcgacagagcagattgttg | 8107 | - | see also 10672 line |
| 24609 | NFYB | NM_006166.2 | 673 | cacaacatcatatcaacagatt | 8123 | - | see also 4269 line |
| 24610 | NFYB | NM_006166.2 | 433 | aacaatcaatggagaagatattc | 8121 | - | see also 4269 line |
| 24611 | NFYC | NM_014223.2 | 313 | tcccactggctcgtattaagaag | 8128 | - | see also 10674 line |
| 24612 | NFYC | NM_014223.2 | 600 | gccgagccagtccagtactattt | 8141 | - | see also 10674 line |
| 24613 | NKX2-2 | NM_002509.2 | 980 | tggtcaggacggcaaaccatgt | 8150 | - | see also 10675 line |
| 24614 | NKX2-2 | NM_002509.2 | 741 | gcgaaagcggcgagtgctttct | 8149 | - | see also 10675 line |
| 24615 | NKX2-3 | NM_145285.1 | 324 | gcgctgaggggaacgcaatttc | 8151 | - | see also 10676 line |
| 24616 | NKX2-3 | NM_145285.1 | 325 | ccgctgaggggacgcaatttct | 8152 | - | see also 10676 line |
| 24617 | NKX2-5 | NM_004387.1 | 907 | gcctcaatcctacggttataac | 8158 | - | transcription_regulator / transcription factor / atrial septal defect, congenital heart disease |
| 24618 | NKX2-5 | NM_004387.1 | 487 | aggaccctagagccgaaaagaaa | 8156 | - | see also 24617 line |
| 24619 | NKX2-5 | NM_004387.1 | 491 | ccctagagccgaaaagaaagagc | 8157 | - | see also 24617 line |
| 24620 | NKX2-8 | NM_014360.2 | 220 | accgtgccgagcctctagatt | 8159 | - | see also 10677 line |
| 24621 | NKX2-8 | NM_014360.2 | 426 | ctcggacgccgagaaaaggaaga | 8161 | - | see also 10677 line |
| 24622 | NKX3-1 | NM_006167.2 | 674 | ccctggtctccgtgtataacagc | 8167 | - | see also 10678 line |
| 24623 | NKX3-1 | NM_006167.2 | 672 | ctccctggtccgtgtataaca | 8166 | - | see also 10678 line |
| 24624 | NKX6-1 | NM_006168.1 | 804 | ggctcgtttggcctattcgttgg | 8174 | - | see also 10679 line |
| 24625 | NKX6-1 | NM_006168.1 | 479 | ccggactgccacgctttagcagc | 8168 | - | see also 10679 line |
| 24626 | NKX6-2 | NM_177400.1 | 693 | cgcggaggacgacgacgaataca | 8175 | - | transcription_regulator / - |
| 24627 | NME2 | NM_002512.1 | 485 | aactggttgactacaagtctgt | 8178 | - | see also 10680 line |
| 24628 | NME2 | NM_002512.1 | 509 | ctcatgactggctatgaataa | 8179 | - | see also 10680 line |
| 24629 | NPAS1 | NM_002517.2 | 1851 | cgcgctcccggaggcctttacc | 8186 | - | see also 10681 line |
| 24630 | NPAS1 | NM_002517.2 | 700 | agcagcgtcttcgactacattca | 8183 | - | see also 10681 line |
| 24631 | NPAS2 | NM_002518.2 | 2380 | gacggacggcaagtcaagtacg | 8211 | - | see also 1618 line |
| 24632 | NPAS2 | NM_002518.2 | 1010 | tgcatagttgaacgaacctttaga | 8196 | - | see also 1618 line |

Figure 46

| | | | | | | | |
|---|---|---|---|---|---|---|---|
| 24633 | NR0B1 | NM_000475.2 | 47 | tgcttatgagcgcgaagcaaacg | 8213 | - | see also 10683 line |
| 24634 | NR0B1 | NM_000475.2 | 794 | cgctgcgcttcgtcaagtacttg | 8221 | - | see also 10683 line |
| 24635 | NR0B2 | NM_021969.1 | 133 | cccgtagccgctgcctatgtagg | 8227 | - | see also 10684 line |
| 24636 | NR0B2 | NM_021969.1 | 365 | cccggtgcccagccatatccaaga | 8229 | - | see also 10684 line |
| 24637 | NR1D1 | NM_021724.1 | 1844 | cccggcaggcaactcaaagaat | 8235 | - | see also 10685 line |
| 24638 | NR1D1 | NM_021724.1 | 2063 | tgctgatggtgctttgcttcg | 8237 | - | see also 10685 line |
| 24639 | NR1D1 | NM_021724.1 | 711 | ccctgaatcctctatagtgaca | 8232 | - | see also 10685 line |
| 24640 | NR1H2 | NM_007121.1 | 907 | agcggctcaagaactaatgatcc | 8241 | - | see also 10686 line |
| 24641 | NR1H2 | NM_007121.1 | 1088 | atcgtggacttcgctaagcaagt | 8243 | - | see also 10686 line |
| 24642 | NR1H2 | NM_007121.1 | 1159 | ggcatccactatcgatcatgc | 8244 | - | see also 10686 line |
| 24643 | NR1H3 | NM_005693.1 | 1226 | tcccatgaccgactgatgttcc | 8251 | - | see also 3959 line |
| 24644 | NR1H3 | NM_005693.1 | 263 | cacagagatcgtccacaaagc | 8246 | - | see also 3959 line |
| 24645 | NR1H4 | NM_005123.1 | 1653 | ctgggtcgctgactgaattacg | 8276 | - | see also 10687 line |
| 24646 | NR1H4 | NM_005123.1 | 892 | aagagtgtcgactaaggaaatgc | 8262 | - | see also 10687 line |
| 24647 | NR1I2 | NM_003889.2 | 1940 | aagtcggaggtccccaaatctgc | 8280 | - | see also 10688 line |
| 24648 | NR1I2 | NM_003889.2 | 2959 | aagtcctacattgaatgcaatcg | 8289 | - | see also 10688 line |
| 24649 | NR1I3 | NM_005122.2 | 816 | gggcctcttgctacacacattga | 8302 | - | see also 10689 line |
| 24650 | NR1I3 | NM_005122.2 | 632 | cacacacttgcagacatcaaca | 8299 | - | see also 10689 line |
| 24651 | NR2C1 | NM_003297.1 | 887 | atcattagcgaatcttggaaaaa | 8337 | - | see also 2229 line |
| 24652 | NR2C1 | NM_003297.1 | 1771 | gtctcattggcaatatacgaatt | 8364 | - | see also 2229 line |
| 24653 | NR2C2 | NM_003298.2 | 1366 | cacacgtcacatttaagctaaca | 8399 | - | see also 10691 line |
| 24654 | NR2C2 | NM_003298.2 | 1194 | gagataactcggccatttgatac | 8396 | - | see also 10691 line |
| 24655 | NR2E1 | NM_003269.2 | 829 | aacggagcatccgaaggaatagg | 8426 | - | see also 2215 line |
| 24656 | NR2E1 | NM_003269.2 | 1806 | atcggcaatgtgccaattacaag | 8452 | - | see also 2215 line |
| 24657 | NR2E3 | NM_014249.2 | 859 | ctggctgcctactcttcatgg | 8455 | - | see also 10693 line |
| 24658 | NR2E3 | NM_014249.2 | 707 | ttcatggccagccttataacagc | 8454 | - | see also 10693 line |
| 24659 | NR2F1 | NM_005654.3 | 425 | agcgtccgcaggaacttaactta | 8464 | - | see also 10694 line |
| 24660 | NR2F1 | NM_005654.3 | 550 | agcggttcagcggaggaagaatgc | 8465 | - | see also 10694 line |
| 24661 | NR2F2 | NM_021005.2 | 1793 | cagttttaacttggccgtatatgg | 8477 | - | see also 8063 line |
| 24662 | NR3C1 | NM_000176.1 | 1558 | tgccgctatcgaaaatgtcttca | 8516 | - | see also 144 line |
| 24663 | NR3C2 | NM_000901.1 | 2664 | ggatagtacaaacatacgaaca | 8603 | - | see also 10697 line |
| 24664 | NR3C2 | NM_000901.1 | 1147 | tccagccttcgaacactaattaa | 8563 | - | see also 10697 line |
| 24665 | NR4A1 | NM_002135.3 | 1131 | aacgcttcatgccagccattatgg | 8615 | - | see also 10698 line |
| 24666 | NR4A1 | NM_002135.3 | 486 | gacggctacacaggagagtttga | 8613 | - | see also 10698 line |
| 24667 | NR4A2 | NM_006186.2 | 683 | ctccgggtcgtttactacaagc | 8630 | - | see also 4276 line |
| 24668 | NR4A2 | NM_006186.2 | 770 | cccgggatctctcacacacttcc | 8631 | - | see also 4276 line |
| 24669 | NR4A3 | NM_006981.2 | 924 | ctcgagcaactacgaactcaagc | 8655 | - | see also 4874 line |

Figure 46

| | | | | | | | |
|---|---|---|---|---|---|---|---|
| 24670 | NR4A3 | NM_006981.2 | 1907 | ctcctccaatctgcatgatgaat | 8660 | - | see also 4874 line |
| 24671 | NR5A1 | NM_004959.3 | 1414 | ccgcactgcgggacaaattcc | 8698 | - | see also 3449 line |
| 24672 | NR5A1 | NM_004959.3 | 1413 | accgcactgcgggacaaattc | 8697 | - | see also 3449 line |
| 24673 | NR5A1 | NM_004959.3 | 300 | agcgcacggtgcagaacaacaag | 8692 | - | see also 3449 line |
| 24674 | NR6A1 | NM_001489.2 | 439 | tgcaacaaacggtatatcgatg | 8700 | - | see also 1026 line |
| 24675 | NR6A1 | NM_001489.2 | 1137 | ctctacgtggcaggagctaatcc | 8712 | - | see also 1026 line |
| 24676 | NT5C | NM_014595.1 | 439 | cccagttcgtagaacgaattat | 8726 | - | see also 10702 line |
| 24677 | NT5C | NM_014595.1 | 440 | cccagttcgtagaacgaattatc | 8727 | - | see also 10702 line |
| 24678 | NT5C | NM_014595.1 | 237 | gcccgacctggcggataaagtgg | 8723 | - | see also 10702 line |
| 24679 | NY-BR-1 | NM_052997.1 | 312 | ttctggtgccgatataaatctcg | 8730 | - | see also 10703 line |
| 24680 | NY-BR-1 | NM_052997.1 | 455 | cactttactatccataacgaaa | 8731 | - | see also 10703 line |
| 24681 | NY-BR-1 | NM_052997.1 | 461 | tactatccataacgaaaagaagt | 8732 | - | see also 10703 line |
| 24682 | ODAG | NM_021167.2 | 432 | tgctcggctcagaacactaaat | 8745 | - | see also 10704 line |
| 24683 | ODAG | NM_021167.2 | 914 | ttgggctactcctgcaataaca | 8756 | - | see also 10704 line |
| 24684 | ONECUT1 | NM_004498.1 | 1222 | tccgcttagcagcatgcaaaagg | 8763 | - | see also 3047 line |
| 24685 | ONECUT2 | NM_004852.1 | 1137 | ccgacctgctccggaatccaaaa | 8772 | - | see also 3372 line |
| 24686 | OTEX | NM_139282.1 | 92 | tccacgacacgtgttctactgc | 8777 | - | transcription_regulator , signal_transduction |
| 24687 | OTEX | NM_139282.1 | 115 | ctgagtgtataccaggtaaaaat | 8778 | - | see also 24686 line |
| 24688 | OTEX | NM_139282.1 | 117 | gagtgtataccaggtaaaaataa | 8779 | - | see also 24686 line |
| 24689 | OTX1 | NM_014562.2 | 891 | gccacgccagcagcctcttatcc | 8784 | - | see also 5754 line |
| 24690 | OTX1 | NM_014562.2 | 1229 | ttcctccgctgaaaatcaact | 8786 | - | see also 5754 line |
| 24691 | OTX2 | NM_021728.2 | 568 | agctaagtgccgccaacaacagc | 8789 | - | see also 8166 line |
| 24692 | OTX3 | NM_147192.2 | 132 | ttcagtgcatggcttacattgg | 8802 | - | see also 9815 line |
| 24693 | OTX3 | NM_147192.2 | 921 | gccctacctgcgggtcaacatgg | 8804 | - | see also 9815 line |
| 24694 | P1P373C6 | NM_019110.3 | 1686 | ctgaggctccgtgttcttataaa | 8811 | - | see also 10707 line |
| 24695 | P1P373C6 | NM_019110.3 | 245 | atggctagagaaccgagaaaaaa | 8806 | - | see also 10707 line |
| 24696 | p66alpha | NM_017660.2 | 912 | ctcatccgcgtcgccaatgttcc | 8817 | - | see also 10708 line |
| 24697 | p66alpha | NM_017660.2 | 508 | aagcaaaactgtgttgttgaaa | 8814 | - | see also 10708 line |
| 24698 | P66beta | NM_020699.1 | 798 | atgtctcaacgtgttattgcacc | 8835 | - | see also 7924 line |
| 24699 | P66beta | NM_020699.1 | 1726 | accgacagcgtgaataccttta | 8855 | - | see also 7924 line |
| 24700 | PAX3 | NM_000438.3 | 698 | cgcctgacgtgagaagaaaatt | 8870 | - | see also 345 line |
| 24701 | PAX3 | NM_000438.3 | 830 | gccgatcctgagaagtaaattc | 8872 | - | see also 345 line |
| 24702 | PAX4 | NM_006193.1 | 313 | cacggatccttaaggtatctaat | 8893 | - | see also 10710 line |
| 24703 | PAX4 | NM_006193.1 | 694 | accggaatcggactatccttctcc | 8896 | - | see also 10710 line |
| 24704 | PAX4 | NM_006193.1 | 309 | atctcacggatccttaaggtatc | 8892 | - | see also 10710 line |

Figure 46

| 24705 | PAX6 | NM_000280.1 | 746 | gggtctgtaccaacgataacata | 8912 | - | see also 234 line | | |
|---|---|---|---|---|---|---|---|---|---|
| 24706 | PAX7 | NM_002584.1 | 613 | ccggcacggtaccgagaatgatg | 8938 | - | see also 10711 line | | |
| 24707 | PAX7 | NM_002584.1 | 1252 | gacgcagtcggaccacattcacg | 8945 | - | see also 10711 line | | |
| 24708 | PAX8 | NM_003466.2 | 868 | gccgctcgagtgcccatttgagc | 8956 | - | see also 2348 line | | |
| 24709 | PAX8 | NM_003466.2 | 168 | tgcctcacaactccatcagatct | 8952 | - | see also 2348 line | | |
| 24710 | PBX1 | NM_002585.1 | 646 | ttcaccacccacgtgatgaatct | 8963 | - | see also 1663 line | | |
| 24711 | PBX2 | NM_002586.3 | 1353 | ggctcaacggagattcctattct | 8972 | - | see also 1664 line | | |
| 24712 | PBX2 | NM_002586.3 | 1355 | ctcaacggagattcctattctgc | 8973 | - | see also 1664 line | | |
| 24713 | PBX4 | NM_025245.1 | 788 | aacaaaagaatccggtataaaaa | 9007 | - | see also 10715 line | | |
| 24714 | PBX4 | NM_025245.1 | 394 | gtcccagatccgacagatttacc | 9004 | - | see also 10715 line | | |
| 24715 | PEG3 | NM_006210.1 | 1219 | cagtcgaggtctaaaaactatgc | 9030 | - | see also 10716 line | | |
| 24716 | PEG3 | NM_006210.1 | 4292 | gagtacgggtcctcctatactca | 9109 | - | see also 10716 line | | |
| 24717 | PGBD1 | NM_032507.2 | 2113 | ttggtttgaaccctatcaagaag | 9148 | - | see also 10717 line | | |
| 24718 | PGBD1 | NM_032507.2 | 2657 | gtgagctacatgattgatgtagc | 9169 | - | see also 10717 line | | |
| 24719 | PGR | NM_000926.2 | 1696 | cggacgtggagggcgcatattcc | 9174 | - | see also 603 line | | |
| 24720 | PGR | NM_000926.2 | 4056 | cagcgtttctatcaacttacaaa | 9218 | - | see also 603 line | | |
| 24721 | PHF1 | NM_002636.3 | 596 | gagcaaccgcagcagagttact | 9229 | - | see also 1704 line | | |
| 24722 | PHF2 | NM_005392.2 | 1371 | gtccgaccggaagtgaatactgt | 9247 | - | see also 3763 line | | |
| 24723 | PHF2 | NM_005392.2 | 1373 | ccgaccggaagtgaatactgtcg | 9248 | - | see also 3763 line | | |
| 24724 | PHOX2B | NM_003924.2 | 588 | tccgtacgccgcagttccttaca | 9266 | - | see also 2683 line | | |
| 24725 | PHOX2B | NM_003924.2 | 592 | tacgccgcagttccttacaaact | 9267 | - | see also 2683 line | | |
| 24726 | PHTF1 | NM_006608.1 | 176 | gacttgattgacgttgacttaat | 9275 | - | see also 10720 line | | |
| 24727 | PHTF1 | NM_006608.1 | 738 | gacaatgtcgaccagagattaga | 9297 | - | see also 10720 line | | |
| 24728 | PILB | NM_012228.2 | 243 | cagttctacgtcacaagagaaaa | 9341 | - | see also 10721 line | | |
| 24729 | PILB | NM_012228.2 | 454 | gtctggatacctcgttaggatca | 9345 | - | see also 10721 line | | |
| 24730 | PITX1 | NM_002653.3 | 689 | ggcaacgtacgcacttcacaagc | 9346 | - | see also 1735 line | | |
| 24731 | PITX1 | NM_002653.3 | 1134 | ctcgggcctcaacaacatcaaca | 9348 | - | see also 1735 line | | |
| 24732 | PITX2 | NM_000325.4 | 1456 | gccgactcctccgtatgtttata | 9356 | - | see also 10722 line | | |
| 24733 | PITX2 | NM_000325.4 | 1455 | cgccgactcctccgtatgtttat | 9355 | - | see also 10722 line | | |
| 24734 | PITX3 | NM_005029.3 | 315 | aggacggttcgctgaaaaagaag | 9364 | - | transcription_regulator | transcription factor | - |
| 24735 | PITX3 | NM_005029.3 | 261 | aggagcacagcgactcagaaaag | 9363 | - | see also 24734 line | | |
| 24736 | PKNOX1 | NM_004571.3 | 875 | gtcgcctgggacaattaggatcc | 9384 | - | see also 3110 line | | |
| 24737 | PKNOX1 | NM_004571.3 | 1215 | cagaggttttggcctgattctat | 9394 | - | see also 3110 line | | |
| 24738 | PKNOX2 | NM_022062.1 | 685 | aaccgtcaactcacaagttgtgt | 9402 | - | see also 8258 line | | |
| 24739 | PKNOX2 | NM_022062.1 | 723 | accaaccggttaccatggtaacc | 9403 | - | see also 8258 line | | |
| 24740 | PLAG1 | NM_002655.1 | 901 | gccttgcatgccgcaacaagtgg | 9418 | - | see also 1736 line | | |

Figure 46

| 24741 | PLAG1 | NM_002655.1 | 778 | atgtttcaccggaaagatcatct | 9413 | - | see also 1736 line | | |
|---|---|---|---|---|---|---|---|---|---|
| 24742 | PLAGL2 | NM_002657.2 | 1243 | ctcatacttgcccgacaaattgc | 9461 | - | see also 1738 line | | |
| 24743 | PLAGL2 | NM_002657.2 | 489 | aaccccaccagtgtatgtactgt | 9452 | - | see also 1738 line | | |
| 24744 | PLU-1 | NM_006618.2 | 3021 | atcgcttgcttcatcgatattgt | 9503 | - | see also 4679 line | | |
| 24745 | PLU-1 | NM_006618.2 | 3893 | gagatgcgtatccgtttggaaca | 9526 | - | see also 4679 line | | |
| 24746 | PML | NM_002675.2 | 446 | ccgccctggataacgtcttttc | 9580 | - | transcription_regulator | - | leukemia |
| 24747 | PML | NM_002675.2 | 447 | cgccctggataacgtctttttcg | 9581 | - | see also 24746 line | | |
| 24748 | PML | NM_002675.2 | 681 | cacccgcaagaccaacaacatct | 9582 | - | see also 24746 line | | |
| 24749 | POLR2I | NM_006233.3 | 146 | cgcgtgccggaactgtgattacc | 9603 | - | see also 10728 line | | |
| 24750 | POLR2I | NM_006233.3 | 349 | aggacgccatgcgcctttactac | 9605 | - | see also 10728 line | | |
| 24751 | POLR3K | NM_016310.2 | 257 | aacatcctcgtgcttacttcatg | 9608 | - | see also 10729 line | | |
| 24752 | POLR3K | NM_016310.2 | 158 | atcggaagtacccaaaactgaaa | 9607 | - | see also 10729 line | | |
| 24753 | POU1F1 | NM_000306.1 | 626 | aacaacaatctgccgatttgaaa | 9619 | - | see also 10730 line | | |
| 24754 | POU1F1 | NM_000306.1 | 458 | caggcggaaaagtaaattggtgg | 9615 | - | see also 10730 line | | |
| 24755 | POU1F1 | NM_000306.1 | 454 | aactcaggcggaaaagtaaattg | 9614 | - | see also 10730 line | | |
| 24756 | POU2F1 | NM_002697.1 | 988 | ggctcgctatggggaaactatat | 9640 | - | see also 1755 line | | |
| 24757 | POU2F1 | NM_002697.1 | 990 | ctcgctatggggaaactatatgg | 9641 | - | see also 1755 line | | |
| 24758 | POU2F2 | NM_002698.1 | 463 | ggcctgctaccgacaccaaatct | 9666 | - | see also 1757 line | | |
| 24759 | POU2F2 | NM_002698.1 | 802 | aacgatgcagagactatgtctgt | 9667 | - | see also 1757 line | | |
| 24760 | POU2F3 | NM_014352.1 | 1253 | tccgcctccagttttaactcttc | 9679 | - | see also 10732 line | | |
| 24761 | POU2F3 | NM_014352.1 | 929 | tcggaggagatctccatgattgc | 9675 | - | see also 10732 line | | |
| 24762 | POU3F2 | NM_005604.1 | 1001 | ggcggatcaaactgggatttacc | 9681 | - | transcription_regulator | translation regulator | - |
| 24763 | POU3F4 | NM_000307.1 | 123 | ttccgcaaccctcagaaacttct | 9682 | - | see also 264 line | | |
| 24764 | POU4F1 | NM_006237.2 | 1463 | agcagaagcggatgaaattctct | 9694 | - | transcription_regulator | transcription factor | - |
| 24765 | POU4F2 | NM_004575.1 | 1287 | cgctcgctcgaagcctactttgc | 9700 | - | - | transcription factor | - |
| 24766 | POU4F2 | NM_004575.1 | 494 | aaccccaccgagcaatatattcg | 9697 | - | see also 24765 line | | |
| 24767 | POU4F2 | NM_004575.1 | 491 | tccaaccccaccgagcaatatat | 9696 | - | see also 24765 line | | |
| 24768 | POU5F1 | NM_002701.1 | 141 | ttcttgaatcccgaatggaaagg | 9716 | - | see also 10735 line | | |
| 24769 | POU5F1 | NM_002701.1 | 129 | gccacacgtaggttcttgaatcc | 9715 | - | see also 10735 line | | |
| 24770 | POU5F1 | NM_002701.1 | 128 | ggccacacgtaggttcttgaatc | 9714 | - | see also 10735 line | | |
| 24771 | PPARA | NM_005036.2 | 582 | ggcttctttcggcgaacgattcg | 9730 | - | see also 10736 line | | |
| 24772 | PPARA | NM_005036.2 | 714 | cacaacgcgattcgttttggacg | 9731 | - | see also 10736 line | | |
| 24773 | PPARD | NM_006238.2 | 772 | cacaacgctatccgttttggtcg | 9748 | - | see also 10737 line | | |
| 24774 | PPARD | NM_006238.2 | 992 | tccacgacatcgagacattgtgg | 9749 | - | see also 10737 line | | |

Figure 46

| 24775 | PPARG | NM_005037.3 | 680 | ggcggagatctccagtgatatcg | 9773 | - | see also 3519 line | | |
|---|---|---|---|---|---|---|---|---|---|
| 24776 | PPARG | NM_005037.3 | 823 | cagacaaatcaccattcgttatc | 9775 | - | see also 3519 line | | |
| 24777 | PRDM1 | NM_001198.2 | 1480 | cccccctacggcatgaattgtaa | 9829 | - | see also 774 line | | |
| 24778 | PRDM1 | NM_001198.2 | 1025 | aggacctctaccgttctaacatt | 9816 | - | see also 774 line | | |
| 24779 | PRDM1 | NM_001198.2 | 1371 | gtcctacggcacggaaggtttgg | 9828 | - | see also 774 line | | |
| 24780 | PRDM16 | NM_022114.1 | 3595 | gaccacgaaggcggtctgttagc | 9877 | - | see also 8274 line | | |
| 24781 | PRDM16 | NM_022114.1 | 3237 | gtcggacaaccacgcacttttag | 9874 | - | see also 8274 line | | |
| 24782 | PRDM16 | NM_022114.1 | 2990 | agcagccgtacaggtgtaagtac | 9871 | - | see also 8274 line | | |
| 24783 | PRDM2 | NM_012231.2 | 5083 | cagcaaaatgtcgtcgaataagc | 9967 | - | see also 10740 line | | |
| 24784 | PRDM2 | NM_012231.2 | 1982 | gagcgtcacatgcatatccatat | 9899 | - | see also 10740 line | | |
| 24785 | PRRX1 | NM_006902.2 | 1356 | caggctttggagcgtgtctttga | 10000 | - | see also 4838 line | | |
| 24786 | PRRX1 | NM_006902.2 | 1184 | ggcggcacaggcggatgagaacg | 9997 | - | see also 4838 line | | |
| 24787 | PRRX2 | NM_016307.3 | 804 | accgccctgagtccagattatct | 10010 | - | transcription_regulator | transcription factor | - |
| 24788 | PRRX2 | NM_016307.3 | 803 | caccgccctgagtccagattatc | 10009 | - | see also 24787 line | | |
| 24789 | RARA | NM_000964.1 | 245 | ttccagttagtggatatagcaca | 10013 | - | see also 10742 line | | |
| 24790 | RARA | NM_000964.1 | 242 | agcttccagttagtggatatagc | 10012 | - | see also 10742 line | | |
| 24791 | RARA | NM_000964.1 | 799 | accaagtgcatcattaagactgt | 10015 | - | see also 10742 line | | |
| 24792 | RARB | NM_000965.2 | 1309 | gacggccttaccctaaatcgaac | 10037 | - | see also 10743 line | | |
| 24793 | RARB | NM_000965.2 | 1624 | gagcgtgtaattaccttgaaaat | 10049 | - | see also 10743 line | | |
| 24794 | RARG | NM_000966.3 | 672 | tccgcctcctcgggtctacaagc | 10053 | - | see also 10744 line | | |
| 24795 | RARG | NM_000966.3 | 696 | atgcttcgtgtgcaatgacaagt | 10054 | - | see also 10744 line | | |
| 24796 | RB1 | NM_000321.1 | 2701 | agcgaccgtgtgctcaaaagaag | 10143 | - | see also 268 line | | |
| 24797 | RB1 | NM_000321.1 | 1986 | aggttcaactacgcgtgtaaatt | 10120 | - | see also 268 line | | |
| 24798 | RBBP1 | NM_002892.2 | 2108 | aacggggacgacctcctttaaaa | 10202 | - | see also 10747 line | | |
| 24799 | RBBP1 | NM_002892.2 | 3189 | aacgattgaagttgatagtattg | 10235 | - | see also 10747 line | | |
| 24800 | RBBP1 | NM_002892.2 | 1309 | tactgccgttcggcaaatattca | 10189 | - | see also 10747 line | | |
| 24801 | RBBP2 | NM_005056.1 | 1220 | agctgtacgagagtatacacttc | 10281 | - | see also 10748 line | | |
| 24802 | RBBP2 | NM_005056.1 | 933 | gaggtcacccgaagacgaaaagt | 10272 | - | see also 10748 line | | |
| 24803 | REL | NM_002908.1 | 231 | gaggggaatgcgttttagataca | 10372 | - | see also 1940 line | | |
| 24804 | REL | NM_002908.1 | 830 | ttcgttttgtgttgaacgattgg | 10390 | - | see also 1940 line | | |
| 24805 | RELA | NM_021975.1 | 588 | tgccgagtgaaccgaaactctgg | 10432 | - | see also 8236 line | | |
| 24806 | RELA | NM_021975.1 | 1036 | cccagccctatccctttacgtca | 10440 | - | see also 8236 line | | |
| 24807 | RELB | NM_006509.2 | 1034 | tgcggatttgccgaattaacaag | 10448 | - | see also 4552 line | | |
| 24808 | RELB | NM_006509.2 | 837 | tgccattgagcggaagattcaac | 10444 | - | see also 4552 line | | |
| 24809 | RERE | NM_012102.2 | 2282 | tggacccgccaccgtttatgttc | 10476 | - | see also 10752 line | | |
| 24810 | RERE | NM_012102.2 | 1741 | ctgcatgaaagcggttacgatgc | 10464 | - | see also 10752 line | | |

Figure 46

| | | | | | | | | |
|---|---|---|---|---|---|---|---|---|
| 24811 | RFX5 | NM_000449.2 | 926 | acctcgggaacggtcatctaaac | - | see also 353 line | | |
| 24812 | RFX5 | NM_000449.2 | 931 | gggaacggtcatctaaaccaaag | - | see also 353 line | | |
| 24813 | RFXAP | NM_000538.2 | 816 | tcctgcaagacctactcttttag | - | transcription_regulator | transcription factor | bare lymphocyte syndrome , MHC class II deficiency |
| 24814 | RFXAP | NM_000538.2 | 878 | gtccagaagtagtgcaatttta | - | see also 24813 line | | |
| 24815 | RFXAP | NM_000538.2 | 822 | aagacctactctttagaacaag | - | see also 24813 line | | |
| 24816 | RLF | NM_012421.1 | 4783 | tgcgttaagtacggtaccaaaat | - | see also 10754 line | | |
| 24817 | RLF | NM_012421.1 | 2309 | atgacgatctgcgttacaaatgt | - | see also 10754 line | | |
| 24818 | RNF110 | NM_007144.2 | 408 | gtgtgacgtgcaggtccataaaa | - | see also 10755 line | | |
| 24819 | RNF110 | NM_007144.2 | 410 | gtgacgtgcaggtccataaaaac | - | see also 10755 line | | |
| 24820 | RNF4 | NM_002938.2 | 776 | gtctcatcgtttccacagaatgc | - | see also 1972 line | | |
| 24821 | RORA | NM_002943.2 | 821 | ttcacgacgacctcagtaactac | - | see also 1984 line | | |
| 24822 | RORA | NM_002943.2 | 1396 | cccgacgtcttcaaatccttagg | - | see also 1984 line | | |
| 24823 | RORB | NM_006914.2 | 1392 | cactcacgccatccaatacgtgg | - | see also 4849 line | | |
| 24824 | RORB | NM_006914.2 | 653 | tccactacggagtcatcacatgt | - | see also 4849 line | | |
| 24825 | RORB | NM_006914.2 | 1017 | tgccaacgggcacgtcattgacc | - | see also 4849 line | | |
| 24826 | RORC | NM_005060.2 | 1186 | aaccgcacggtctttttgaagg | - | see also 3529 line | | |
| 24827 | RORC | NM_005060.2 | 1183 | gacaaccgcacggtctttttga | - | see also 3529 line | | |
| 24828 | RORC | NM_005060.2 | 1181 | ctgacaaccgcacggtctttt | - | see also 3529 line | | |
| 24829 | RPF-1 | NM_007252.1 | 1917 | gaccgagaagtagttagagtttg | - | see also 5055 line | | |
| 24830 | RPF-1 | NM_007252.1 | 1776 | atcggagtgaaccatccaaaaa | - | see also 5055 line | | |
| 24831 | RUNX1 | NM_001754.2 | 1358 | gacgtgccagcgcatgacaacc | - | see also 10757 line | | |
| 24832 | RUNX1 | NM_001754.2 | 1297 | taccaataacctggatccattgc | - | see also 10757 line | | |
| 24833 | RUNX2 | NM_004348.1 | 430 | gcgggttaacgatgaaaattattc | - | see also 2904 line | | |
| 24834 | RUNX2 | NM_004348.1 | 961 | gtgcctaggcgcgcatttcagatga | - | see also 2904 line | | |
| 24835 | RXRA | NM_002957.3 | 1342 | tgcgctccatcgggctccaaatgc | - | transcription_regulator , receptor_acitivity , s ignal_transduction | Retinoic acid receptor RXR-alpha | atherosclerosis |
| 24836 | RXRB | NM_021976.3 | 877 | tccgcaaagaccttacatactct | - | see also 10758 line | | |
| 24837 | RXRB | NM_021976.3 | 1331 | ctcacacgcatccattgatgttc | - | see also 10758 line | | |
| 24838 | RXRB | NM_021976.3 | 1666 | tccggtccattggccttaagtgt | - | see also 10758 line | | |
| 24839 | RXRG | NM_006917.2 | 702 | atctacacgtgtcgggataataa | - | see also 10759 line | | |
| 24840 | RXRG | NM_006917.2 | 707 | cacgtgtcgggataataaagact | - | see also 10759 line | | |
| 24841 | SATB1 | NM_002971.2 | 1294 | ctccccctgcagtagatctatg | - | see also 10760 line | | |
| 24842 | SATB1 | NM_002971.2 | 1295 | tccccctgcagtagatctatga | - | see also 10760 line | | |
| 24843 | SATB2 | NM_015265.1 | 1249 | accgcacacagggattgttgtct | - | see also 6294 line | | |
| 24844 | SBLF | NM_006873.2 | 339 | cagacagcccactgcaatatca | - | see also 10762 line | | |

950

Figure 46

| 24845 | SBLF | NM_006873.2 | 1399 | gccgaagcgagatgaatcctatt | 11041 | - | see also 10762 line |
|---|---|---|---|---|---|---|---|
| 24846 | SCAND2 | NM_022050.2 | 452 | gactccttcagtacaagatctcc | 11072 | - | see also 10763 line |
| 24847 | SCAND2 | NM_022050.2 | 463 | tacaagatctccaaatagttaaa | 11073 | - | see also 10763 line |
| 24848 | SCMH1 | NM_012236.1 | 1710 | accttcgtagtgacaatctgttt | 11101 | - | see also 10764 line |
| 24849 | SCMH1 | NM_012236.1 | 729 | ttcggctgaatgcgtcttcttgg | 11082 | - | see also 10764 line |
| 24850 | SCMH1 | NM_012236.1 | 499 | acctccaagcaacgagttcaaga | 11075 | - | see also 10764 line |
| 24851 | SCML1 | NM_006746.2 | 1179 | aacggctgtgaagctatgctact | 11113 | - | see also 10765 line |
| 24852 | SCML1 | NM_006746.2 | 626 | atgaggtttacgagacattctcc | 11103 | - | see also 10765 line |
| 24853 | SCML2 | NM_006089.1 | 780 | gtcgcctgacaggagatgtatta | 11133 | - | see also 10766 line |
| 24854 | SCML2 | NM_006089.1 | 1839 | caccactagttggggacatatcc | 11154 | - | see also 10766 line |
| 24855 | SCRT1 | NM_031309.2 | 691 | cggcgagtgcggcaaaacatacg | 11162 | - | see also 10767 line |
| 24856 | SDCCAG33 | NM_005786.3 | 3582 | atcgaccgctactattatgaaaa | 11178 | - | see also 10768 line |
| 24857 | SDCCAG33 | NM_005786.3 | 2351 | cccgtacgtcacgcccaataacc | 11167 | - | see also 10768 line |
| 24858 | SHOX2 | NM_003030.2 | 232 | ggcgaagtcggaccaatttcacc | 11200 | - | see also 2032 line |
| 24859 | SIM1 | NM_005068.2 | 2303 | ttggctctcaccggcagtatttt | 11239 | - | see also 3536 line |
| 24860 | SIM1 | NM_005068.2 | 2218 | ttgatcctagctaaagactatct | 11237 | - | see also 3536 line |
| 24861 | SIM2 | NM_005069.2 | 837 | atcgagaagaccctataccatca | 11265 | - | see also 3538 line |
| 24862 | SIM2 | NM_005069.2 | 1026 | tgcatcgtgagtgtcaattatgt | 11268 | - | see also 3538 line |
| 24863 | SIX1 | NM_005982.1 | 993 | cacgccaggagctcaaactattc | 11280 | - | see also 4179 line |
| 24864 | SIX2 | NM_016932.3 | 531 | gtggctcaaggcacactcatcg | 11284 | - | see also 10771 line |
| 24865 | SIX2 | NM_016932.3 | 395 | agcaccttcacaagaatgaaagc | 11283 | - | see also 10771 line |
| 24866 | SIX4 | NM_017420.1 | 1594 | gtggtatacacggttcctaatac | 11319 | - | see also 6993 line |
| 24867 | SIX6 | NM_007374.1 | 291 | aactaccgcgagctctatcatat | 11342 | - | see also 10773 line |
| 24868 | SIX6 | NM_007374.1 | 100 | tccagctgcccatcttgaatttc | 11341 | - | see also 10773 line |
| 24869 | SLC26A10 | NM_133489.1 | 2713 | ctccagatcagctgtttgtgagt | 11344 | - | - | | - |
| 24870 | SLC26A3 | NM_000111.1 | 2174 | gtgaggggccttaaatcgatttt | 11428 | - | see also 10774 line |
| 24871 | SLC26A3 | NM_000111.1 | 2172 | cagtgaggggccttaaatcgatt | 11427 | - | see also 10774 line |
| 24872 | SLC26A3 | NM_000111.1 | 434 | gggattgtggccgtactacaagg | 11359 | - | see also 10774 line |
| 24873 | SLC30A9 | NM_006345.2 | 1010 | ctcatccgtacggattttcaaat | 11471 | - | see also 4439 line |
| 24874 | SLC30A9 | NM_006345.2 | 900 | taccggttcagcaagtatgttct | 11466 | - | see also 4439 line |
| 24875 | SLC34A3 | NM_080877.1 | 1255 | gtcgtgaggacagtcatcaatgc | 11504 | - | - | | - |
| 24876 | SLC38A2 | NM_018976.3 | 352 | gccgaaatgggacgattcagtat | 11505 | - | see also 7623 line |
| 24877 | SLC38A2 | NM_018976.3 | 1295 | accgcagccgtagaagaatgatg | 11536 | - | see also 7623 line |
| 24878 | SLC38A2 | NM_018976.3 | 1377 | tggatacctaacattttacgaac | 11540 | - | see also 7623 line |
| 24879 | SLC38A6 | NM_153811.1 | 1233 | atcactctagcactcaatattat | 11587 | - | see also 10778 line |

EP 1 752 536 A1

Figure 46

| 24880 | SLC38A6 | NM_153811.1 | 234 | cagcgatccccaggtgtttcatt | 11556 | - | see also 10778 line | | |
|---|---|---|---|---|---|---|---|---|---|
| 24881 | SLC4A10 | NM_022058.2 | 2919 | aggcacgtaccgcttcgaaaagt | 11666 | - | see also 8254 line | | |
| 24882 | SLC4A10 | NM_022058.2 | 1216 | aagatcgtaatgacttggtatca | 11617 | - | see also 8254 line | | |
| 24883 | SMARCA4 | NM_003072.2 | 3624 | tgcgtatcgcggctttaaatacc | 11695 | - | see also 10780 line | | |
| 24884 | SMARCA4 | NM_003072.2 | 2727 | ctgggcgtacgagtttgacaagt | 11684 | - | see also 10780 line | | |
| 24885 | SNAPC4 | NM_003086.1 | 3201 | cccgggtcccaccgtcttaaatg | 11729 | - | see also 2079 line | | |
| 24886 | SNAPC4 | NM_003086.1 | 1480 | cacatcccctaccgcagaattgt | 11720 | - | see also 2079 line | | |
| 24887 | SNAPC5 | NM_006049.1 | 181 | atgttggtgcatgtagacaatga | 11742 | - | see also 10783 line | | |
| 24888 | SNAPC5 | NM_006049.1 | 164 | ctgaacagtcacatgatatgttg | 11741 | - | see also 10783 line | | |
| 24889 | SOLH | NM_005632.1 | 1401 | tgccaagtgcacgctcagaaacc | 11746 | - | transcription_regulator | - | - |
| 24890 | SOLH | NM_005632.1 | 1910 | tgcgaccccaggagatcaactgc | 11747 | - | see also 24889 line | | |
| 24891 | SOLH | NM_005632.1 | 3446 | ggcaggtcctggtgatcttgtcc | 11748 | - | see also 24889 line | | |
| 24892 | SOX13 | NM_005686.1 | 302 | agctggacttcaaccgaaatttg | 11749 | - | see also 10785 line | | |
| 24893 | SOX13 | NM_005686.1 | 662 | aggttaacatgccttatgtcatg | 11753 | - | see also 10785 line | | |
| 24894 | SOX14 | NM_004189.2 | 168 | acgcctaggtgccgaatggaagc | 11754 | - | see also 10786 line | | |
| 24895 | SOX14 | NM_004189.2 | 640 | tacgtggtgccctgtaactgtac | 11755 | - | see also 10786 line | | |
| 24896 | SOX4 | NM_003107.2 | 2081 | ccgggacctggattttaacttcg | 11760 | - | transcription_regulator | translation regulator | - |
| 24897 | SOX4 | NM_003107.2 | 953 | gaccccgagtgggcacatcaagc | 11758 | - | see also 24896 line | | |
| 24898 | SOX4 | NM_003107.2 | 2014 | acctgaaccccagctcaaacttt | 11759 | - | see also 24896 line | | |
| 24899 | SOX5 | NM_006940.4 | 1738 | tcccgagggcgtggtagcaatga | 11789 | - | see also 10788 line | | |
| 24900 | SOX5 | NM_006940.4 | 1426 | gccagagttagcacaataggtta | 11778 | - | see also 10788 line | | |
| 24901 | SOX6 | NM_033326.1 | 2112 | agcttcggattggggagtataag | 11831 | - | see also 9400 line | | |
| 24902 | SOX6 | NM_033326.1 | 705 | tggcagcgtcacaaattgagaaa | 11801 | - | see also 9400 line | | |
| 24903 | SP140 | NM_007237.2 | 1352 | gtcagtgtctagtgaactagaaa | 11863 | - | see also 10791 line | | |
| 24904 | SP140 | NM_007237.2 | 316 | tccgagaccgctccttcatctcc | 11842 | - | see also 10791 line | | |
| 24905 | SP140 | NM_007237.2 | 1350 | gggtcagtgtctagtgaactaga | 11862 | - | see also 10791 line | | |
| 24906 | SPBPBP | NM_006692.1 | 859 | gggcactttactaaagctttaca | 11877 | - | see also 10792 line | | |
| 24907 | SPBPBP | NM_006692.1 | 776 | ggctggagagtgccattataaca | 11872 | - | see also 10792 line | | |
| 24908 | SPDEF | NM_012391.1 | 1332 | gccgctccatccgccagtattac | 11878 | - | see also 5306 line | | |
| 24909 | SPI1 | NM_003120.1 | 279 | aacgccaaacgcacgagtattac | 11884 | - | see also 10793 line | | |
| 24910 | SPI1 | NM_003120.1 | 255 | tgccctatgacacggatctatac | 11882 | - | see also 10793 line | | |
| 24911 | SPIB | NM_003121.1 | 57 | tacccagatggcgtcttctatga | 11887 | - | transcription_regulator | translation regulator | - |
| 24912 | SPIB | NM_003121.1 | 314 | cgcataccccacggagaacttcg | 11890 | - | see also 24911 line | | |
| 24913 | SPIB | NM_003121.1 | 190 | acccggcagcagccgcttttagc | 11889 | - | see also 24911 line | | |

EP 1 752 536 A1

Figure 46

| | | | | | | | |
|---|---|---|---|---|---|---|---|
| 24914 | SPIC | NM_152323.1 | 239 | cagtgctgcggacttctatttg | 11899 | - | see also 10794 line |
| 24915 | SPIC | NM_152323.1 | 109 | ttcagtactcgccagattacaga | 11893 | - | see also 10794 line |
| 24916 | SPIC | NM_152323.1 | 357 | ctccgactgtttgaataccttca | 11900 | - | see also 10794 line |
| 24917 | SREBF1 | NM_004176.2 | 2305 | gacgctggccgagatctatgtgg | 11919 | - | see also 10795 line |
| 24918 | SREBF1 | NM_004176.2 | 2170 | cgcagccctggctcaccataagc | 11917 | - | see also 10795 line |
| 24919 | SRF | NM_003131.1 | 1589 | agcccgcatgcggtgatgtatgc | 11934 | - | see also 2113 line |
| 24920 | SRF | NM_003131.1 | 985 | gaccggcaaggcactgattcaga | 11928 | - | see also 2113 line |
| 24921 | ST18 | NM_014682.1 | 713 | tgcgtactcgctctaaaggaacc | 11937 | - | see also 10797 line |
| 24922 | ST18 | NM_014682.1 | 2218 | ttcggtaaagccctctcaataca | 11967 | - | see also 10797 line |
| 24923 | STAT1 | NM_007315.2 | 2177 | aaggggcatcacattcacatgg | 12048 | - | see also 10798 line |
| 24924 | STAT1 | NM_007315.2 | 354 | gtctcagttgtacgaacttcagc | 12001 | - | see also 10798 line |
| 24925 | STAT2 | NM_005419.2 | 734 | cactgctaggcgcgattaacttacc | 12066 | - | see also 10799 line |
| 24926 | STAT2 | NM_005419.2 | 744 | ccgattaactaccctaatcgagc | 12067 | - | see also 10799 line |
| 24927 | STAT3 | NM_003150.2 | 992 | ctgcctagatcgcgctagaaaact | 12091 | - | see also 10800 line |
| 24928 | STAT3 | NM_003150.2 | 994 | gcctagatcggctagaaaaactgg | 12092 | - | see also 10800 line |
| 24929 | STAT3 | NM_003150.2 | 1042 | ttcagaccgtcaacaaattaag | 12096 | - | see also 10800 line |
| 24930 | STAT4 | NM_003151.2 | 1358 | aactctaagcaaccgaagatttg | 12166 | - | see also 2128 line |
| 24931 | STAT4 | NM_003151.2 | 1239 | cagaggccgttggtacttaaaac | 12161 | - | see also 2128 line |
| 24932 | STAT5A | NM_003152.2 | 2470 | agcccgacggaccttcttgttg | 12215 | - | see also 10802 line |
| 24933 | STAT5A | NM_003152.2 | 2471 | gcccgacggacctctgttgc | 12216 | - | see also 10802 line |
| 24934 | STAT5B | NM_012448.2 | 551 | atccgccatatgtacaatga | 12232 | - | see also 5336 line |
| 24935 | STAT5B | NM_012448.2 | 2198 | gaccgcttgggagacttgaatta | 12247 | - | see also 5336 line |
| 24936 | STAT6 | NM_003153.3 | 1867 | ctggtctgaccggctgatcattg | 12265 | - | see also 10804 line |
| 24937 | STAT6 | NM_003153.3 | 764 | ttgatagaaactcctgctaatgg | 12263 | - | see also 10804 line |
| 24938 | SUPT4H1 | NM_003168.1 | 195 | agggtaaccgagagatggtatat | 12279 | - | see also 10805 line |
| 24939 | SUPT4H1 | NM_003168.1 | 124 | ttcgctgtcaagactatagacc | 12274 | - | see also 10805 line |
| 24940 | SUPT5H | NM_003169.2 | 2243 | gtcagcgtggcvggctttggtagc | 12304 | - | see also 2141 line |
| 24941 | SUPT5H | NM_003169.2 | 618 | gggtgagtattacatgaagaaat | 12291 | - | see also 2141 line |
| 24942 | SUPT6H | NM_003170.2 | 2327 | gtcgaaagctctacaattggttg | 12347 | - | see also 2142 line |
| 24943 | SUPT6H | NM_003170.2 | 2223 | ggccatcgaacgggcttacagc | 12345 | - | see also 2142 line |
| 24944 | SUPT6H | NM_003170.2 | 439 | aagtaccggcgtgtcaaaaaat | 12316 | - | see also 2142 line |
| 24945 | T | NM_003181.2 | 1370 | cctatgctcatcggaacaattc | 12403 | - | see also 10808 line |
| 24946 | T | NM_003181.2 | 969 | tgctgaactccttgcataagtat | 12400 | - | see also 10808 line |
| 24947 | TADA2L | NM_001488.2 | 827 | ctcccgacctacctttgactcc | 12412 | - | see also 10809 line |
| 24948 | TADA2L | NM_001488.2 | 882 | agctcgagcagatttcattgagg | 12413 | - | see also 10809 line |
| 24949 | TADA3L | NM_006354.2 | 1058 | ccgcacacttgaggagttactga | 12449 | - | see also 10810 line |
| 24950 | TADA3L | NM_006354.2 | 1436 | cactcccctgcaatcagaaca | 12452 | - | see also 10810 line |

953

Figure 46

| 24951 | TAF10 | NM_006284.2 | 461 | gcctcagacccacgcataattcg | 12455 | - | see also 10811 line |
|---|---|---|---|---|---|---|---|
| 24952 | TAF10 | NM_006284.2 | 591 | aggaccgcaagtacactctaacc | 12457 | - | see also 10811 line |
| 24953 | TAF13 | NM_005645.2 | 252 | aagacaaggtcgagtacaagttg | 12467 | - | see also 10812 line |
| 24954 | TAF13 | NM_005645.2 | 260 | gtcgagtacaagttgaagatatc | 12468 | - | see also 10812 line |
| 24955 | TAF1B | NM_005680.1 | 1410 | gtcgacaaaccagtagcatataa | 12511 | - | see also 10813 line |
| 24956 | TAF1B | NM_005680.1 | 911 | gcctgactacgaggacatctata | 12495 | - | see also 10813 line |
| 24957 | TAF4 | NM_003185.2 | 1360 | gtcctcgtccgaagtgagaatgg | 12525 | - | see also 10814 line |
| 24958 | TAF4 | NM_003185.2 | 3137 | gacagttcacgcgacaaagaatc | 12557 | - | see also 10814 line |
| 24959 | TAF4 | NM_003185.2 | 2813 | tccgggcacagctcaagtttttt | 12552 | - | see also 10814 line |
| 24960 | TAF5 | NM_006951.2 | 1053 | gggatgccgcgtagtaagcaaca | 12579 | - | see also 4867 line |
| 24961 | TAF5 | NM_006951.2 | 1058 | gccgcgtagtaagcaacagatag | 12580 | - | see also 4867 line |
| 24962 | TAF5L | NM_014409.2 | 1273 | cagtccatatagcctgtacttcg | 12653 | - | see also 5664 line |
| 24963 | TARDBP | NM_007375.2 | 1172 | gtcaggcccatcgggtaataacc | 12668 | - | see also 10817 line |
| 24964 | TARDBP | NM_007375.2 | 1175 | aggcccatcgggtaataaccaaa | 12669 | - | see also 10817 line |
| 24965 | TBR1 | NM_006593.2 | 1741 | cgccgcaacgctggtttgtgacg | 12698 | - | see also 4618 line |
| 24966 | TBR1 | NM_006593.2 | 1037 | tcccacggctcattacaatattt | 12691 | - | see also 4618 line |
| 24967 | TBX1 | NM_005992.1 | 797 | tggacgacaacggccacattatt | 12706 | - | see also 10819 line |
| 24968 | TBX1 | NM_005992.1 | 873 | acgcaaagatagcgagaaatatg | 12710 | - | see also 10819 line |
| 24969 | TBX10 | NM_005995.2 | 304 | agcctctgtgggaggaattcaac | 12717 | - | transcription_regulator | - | | - |
| 24970 | TBX10 | NM_005995.2 | 442 | tcccctggacgacaagagatac | 12718 | - | see also 24969 line |
| 24971 | TBX10 | NM_005995.2 | 443 | cccctggacgacaagagataca | 12719 | - | see also 24969 line |
| 24972 | TBX19 | NM_005149.1 | 579 | tgcccatcgaatggtaacaaact | 12733 | - | see also 3614 line |
| 24973 | TBX19 | NM_005149.1 | 581 | cccatcgaatggtaacaaactgc | 12734 | - | see also 3614 line |
| 24974 | TBX19 | NM_005149.1 | 80 | agcccagcgatggcactgtttct | 12720 | - | see also 3614 line |
| 24975 | TBX2 | NM_005994.2 | 495 | cgctgacgattgccgctataagt | 12740 | - | see also 10821 line |
| 24976 | TBX2 | NM_005994.2 | 1874 | tgcgtttcagcccctatcagatc | 12744 | - | see also 10821 line |
| 24977 | TBX20 | NM_020417.1 | 584 | taccggtgagcaactactcaaac | 12749 | - | see also 10822 line |
| 24978 | TBX20 | NM_020417.1 | 585 | accggtgagcaactactcaaaca | 12750 | - | see also 10822 line |
| 24979 | TBX20 | NM_020417.1 | 110 | ggcgacggagaacacaatcaaac | 12746 | - | see also 10822 line |
| 24980 | TBX21 | NM_013351.1 | 1070 | tccaacacgcatatctttacttt | 12760 | - | see also 10823 line |
| 24981 | TBX21 | NM_013351.1 | 642 | acctgttgtggtccaagtttaat | 12758 | - | see also 10823 line |
| 24982 | TBX22 | NM_016954.2 | 690 | accccgagtgcacgtgatagagc | 12778 | - | see also 6980 line |
| 24983 | TBX22 | NM_016954.2 | 189 | gagcgagccgcttgaaaaacaac | 12765 | - | see also 6980 line |
| 24984 | TBX3 | NM_005996.2 | 447 | gccgatcatggatcaattggtgg | 12812 | - | see also 10825 line |
| 24985 | TBX3 | NM_005996.2 | 996 | tagtacatttcggacatacttgt | 12821 | - | see also 10825 line |
| 24986 | TBX3 | NM_005996.2 | 1282 | gggacatcgaacctcaaagattt | 12827 | - | see also 10825 line |

Figure 46

| | | | | | | | |
|---|---|---|---|---|---|---|---|
| 24987 | TBX4 | NM_018488.2 | 1619 | cccgagaatcttccttacagtac | 12853 | - | see also 10826 line |
| 24988 | TBX4 | NM_018488.2 | 1561 | ccctcgccacatctaaatgctgc | 12849 | - | see also 10826 line |
| 24989 | TBX4 | NM_018488.2 | 1073 | ccgccaacctgacttaccttgc | 12841 | - | see also 10826 line |
| 24990 | TBX5 | NM_000192.3 | 991 | tgccgacgatcacagatacaaat | 12863 | - | see also 10827 line |
| 24991 | TBX5 | NM_000192.3 | 988 | acctgccgacgatcacagataca | 12861 | - | see also 10827 line |
| 24992 | TBX6 | NM_004608.2 | 1506 | tccggagaacggcagaaactgt | 12891 | - | see also 10828 line |
| 24993 | TBX6 | NM_004608.2 | 820 | caccctaaactggattgcttcc | 12887 | - | see also 10828 line |
| 24994 | TCEA1 | NM_006756.1 | 469 | ctcgagatacttatgtttcatcc | 12910 | - | see also 10829 line |
| 24995 | TCEA1 | NM_006756.1 | 461 | gacaaatgctgagatacttatg | 12909 | - | see also 10829 line |
| 24996 | TCEA1 | NM_006756.1 | 231 | tccacaagaatcggaatgtcagt | 12903 | - | see also 10829 line |
| 24997 | TCEA2 | NM_003195.4 | 1157 | ggctcagatcgaggaatgcatct | 12912 | - | see also 10830 line |
| 24998 | TCEAL1 | NM_004780.1 | 166 | tggacaaaccacgcaaagaaaat | 12913 | - | see also 10831 line |
| 24999 | TCEAL1 | NM_004780.1 | 168 | gacaaaccacgcaaagaaaatga | 12914 | - | see also 10831 line |
| 25000 | TCF1 | NM_000545.3 | 619 | gggcgaggaaccgtttcaagtgg | 12916 | - | see also 10832 line |
| 25001 | TCF1 | NM_000545.3 | 1568 | gggcctgctcccgcagcactatgc | 12918 | - | see also 10832 line |
| 25002 | TCF19 | NM_007109.1 | 386 | aggacttgctgccattaccatc | 12921 | - | see also 10833 line |
| 25003 | TCF19 | NM_007109.1 | 352 | tacttcatgttccaacaagtacg | 12920 | - | see also 10833 line |
| 25004 | TCF2 | NM_000458.1 | 937 | aggcctacgatcggcaaaagaaac | 12930 | - | see also 355 line |
| 25005 | TCF2 | NM_000458.1 | 914 | cggtcccagcaaatcttgtacc | 12929 | - | see also 355 line |
| 25006 | TCF3 | NM_003200.1 | 1053 | ccggatcactcaagcaataact | 12952 | - | see also 10835 line |
| 25007 | TCF3 | NM_003200.1 | 1052 | ccccggatcactcaagcaataac | 12951 | - | see also 10835 line |
| 25008 | TCF3 | NM_003200.1 | 1051 | tccccggatcactcaagcaataa | 12950 | - | see also 10835 line |
| 25009 | TCF7L1 | NM_031283.1 | 587 | tccagcacactgtctaataaag | 12960 | - | see also 10836 line |
| 25010 | TCF7L1 | NM_031283.1 | 1048 | gccctgcagtgagcgtgaaatca | 12962 | - | see also 10836 line |
| 25011 | TCF7L1 | NM_031283.1 | 1227 | cacaacctgtctcgagaagaaca | 12964 | - | see also 10836 line |
| 25012 | TCF8 | NM_030751.2 | 715 | gtgacgcagtctgggtgtaatcg | 12975 | - | see also 8924 line |
| 25013 | TCF8 | NM_030751.2 | 2505 | agcgctagctgccaataagcaaa | 13025 | - | see also 8924 line |
| 25014 | TCFL4 | NM_170607.2 | 906 | accctgcgggagattgtgattgg | 13053 | - | see also 10023 line |
| 25015 | TCFL4 | NM_170607.2 | 925 | ttggcgtcctgcaccaattgaaa | 13054 | - | see also 10023 line |
| 25016 | TEAD2 | NM_003598.1 | 1269 | tgcctgagcgatacatgatgaac | 13069 | - | see also 10839 line |
| 25017 | TEAD2 | NM_003598.1 | 404 | caggtttggcccgaaggaaatc | 13058 | - | see also 10839 line |
| 25018 | TEAD3 | NM_003214.1 | 401 | gagttgattgcacgctatattaa | 13075 | - | see also 10840 line |
| 25019 | TEAD3 | NM_003214.1 | 830 | gtgttggcaggaccgtaccattgc | 13081 | - | see also 10840 line |
| 25020 | TEAD4 | NM_003213.1 | 1386 | tgctgtgcattgcctatgtcttt | 13093 | - | see also 10841 line |
| 25021 | TEAD4 | NM_003213.1 | 340 | gccctgtcgcagggcgcaaaatca | 13085 | - | see also 10841 line |
| 25022 | TFAP2B | NM_003221.2 | 533 | ctcggccatcccggaattgaaga | 13101 | - | see also 2182 line |
| 25023 | TFAP2B | NM_003221.2 | 635 | cccaaatcggtgacttcctaat | 13104 | - | see also 2182 line |

Figure 46

| | | | | | | | |
|---|---|---|---|---|---|---|---|
| 25024 | TFAP2BL1 | NM_172238.1 | 1525 | tcctgcgcgaccaaacaaatcgt | 13137 | - | see also 10042 line |
| 25025 | TFAP2C | NM_003222.3 | 385 | cacactggagtcgccgaatatca | 13151 | - | see also 10844 line |
| 25026 | TFAP2C | NM_003222.3 | 940 | ctcctcagctctacgtctaaata | 13153 | - | see also 10844 line |
| 25027 | TFCP2 | NM_005653.3 | 1462 | cagtggttgcatcgaaatcgtt | 13193 | - | see also 10845 line |
| 25028 | TFCP2 | NM_005653.3 | 485 | tggccgacgaagtgattgaatcc | 13166 | - | see also 10845 line |
| 25029 | TFDP1 | NM_007111.3 | 215 | ggcaaaagatgccggtctaattg | 13216 | - | see also 10846 line |
| 25030 | TFDP1 | NM_007111.3 | 1232 | aactgttgaggcgtgttcatca | 13218 | - | see also 10846 line |
| 25031 | TFDP2 | NM_006286.1 | 492 | aagaacattagcgaagagtta | 13232 | - | see also 4376 line |
| 25032 | TFDP2 | NM_006286.1 | 1233 | gagacccctgttcgttcaatga | 13244 | - | see also 4376 line |
| 25033 | TFE3 | NM_006521.3 | 1303 | gcgtcgcaggcgattcaacatta | 13258 | - | see also 4560 line |
| 25034 | TFE3 | NM_006521.3 | 1302 | agcgtcgcaggcgattcaacatt | 13257 | - | see also 4560 line |
| 25035 | TFEB | NM_007162.1 | 1295 | ctcccgtccgcatgaacatgg | 13266 | - | see also 10848 line |
| 25036 | TFEB | NM_007162.1 | 379 | agcaacagcgctgtcatgcattac | 13262 | - | see also 10848 line |
| 25037 | TFEC | NM_012252.1 | 312 | ctcaccaagttactagctattgg | 13269 | - | see also 10849 line |
| 25038 | TFEC | NM_012252.1 | 890 | ttcacttggcacggttgatttag | 13284 | - | see also 10849 line |
| 25039 | TGIF | NM_003244.2 | 918 | agcagatagcggccaaaaacttc | 13303 | - | see also 10850 line |
| 25040 | TGIF | NM_003244.2 | 886 | gtcggtcggtgtgggacaaaaca | 13301 | - | see also 10850 line |
| 25041 | TGIF2 | NM_021809.4 | 635 | acccctgagcacacttactct | 13316 | - | see also 10851 line |
| 25042 | TGIF2 | NM_021809.4 | 413 | ggcaaagaccctaatcagttac | 13314 | - | see also 10851 line |
| 25043 | TGIF2 | NM_021809.4 | 637 | cctggtagcacacttacctgc | 13317 | - | see also 10851 line |
| 25044 | THG-1 | NM_030935.3 | 1663 | acctagacagcgacgatgatagt | 13322 | - | see also 10852 line |
| 25045 | THG-1 | NM_030935.3 | 1694 | aagcctggttggcattgacaaca | 13323 | - | see also 10852 line |
| 25046 | THRA | NM_003250.4 | 600 | gtcagggtatatccctagttac | 13325 | - | see also 2190 line |
| 25047 | THRB | NM_000461.2 | 418 | aggcgcagcacgttgaaaaatga | 13338 | - | see also 361 line |
| 25048 | THRB | NM_000461.2 | 1517 | aacaccacgtgacacactttttg | 13358 | - | see also 361 line |
| 25049 | THRB | NM_000461.2 | 417 | gaggcgcagcaacgttgaaaaatg | 13337 | - | see also 361 line |
| 25050 | TIEG | NM_005655.1 | 1060 | cccaaaggcgctgtcatgtttgt | 13374 | - | see also 10854 line |
| 25051 | TIEG | NM_005655.1 | 1059 | ccccaaaggcgctgtcatgtttg | 13373 | - | see also 10854 line |
| 25052 | TIEG | NM_005655.1 | 1091 | agcccgttgtgcagagttcaaag | 13375 | - | see also 10854 line |
| 25053 | TIX1 | NM_015035.2 | 1679 | atgggttgcaagcaacaagttcc | 13401 | - | see also 10855 line |
| 25054 | TIX1 | NM_015035.2 | 577 | gggcatcggagcactttagatgg | 13384 | - | see also 10855 line |
| 25055 | TLX1 | NM_005521.2 | 1167 | tggtcggagcgcctacacttac | 13425 | - | see also 3854 line |
| 25056 | TLX1 | NM_005521.2 | 1053 | gtcacgcagagcccattagcttc | 13423 | - | see also 3854 line |
| 25057 | TLX2 | NM_001534.2 | 524 | ccgacgcacagttcaaaacgtgg | 13434 | - | transcription_regulator |
| 25058 | TNRC4 | NM_007185.2 | 1113 | atgaggacgtccggaagatgttt | 13436 | - | see also 10857 line |
| 25059 | TNRC4 | NM_007185.2 | 2009 | atctcagccaagtcttgttga | 13440 | - | see also 10857 line |

Figure 46

| | | | | | | | |
|---|---|---|---|---|---|---|---|
| 25060 | TP53 | NM_000546.2 | 386 | gtccccgacgatattgaacaat | 13448 | - | see also 10858 line |
| 25061 | TP53 | NM_000546.2 | 388 | cccggacgatattgaacaatgg | 13450 | - | see also 10858 line |
| 25062 | TP73 | NM_005427.1 | 536 | gacgtactcccgctcttgaaga | 13465 | - | see also 3789 line |
| 25063 | TP73 | NM_005427.1 | 753 | atccgcgtggaaggcaataatct | 13467 | - | see also 3789 line |
| 25064 | TP73L | NM_003722.3 | 1039 | accgccgtccaattttaatcatt | 13495 | - | see also 10860 line |
| 25065 | TP73L | NM_003722.3 | 1035 | atgaaccgcgtccaattttaat | 13494 | - | see also 10860 line |
| 25066 | TP73L | NM_003722.3 | 1032 | gggatgaaccgcgtccaattt | 13493 | - | see also 10860 line |
| 25067 | TRIM16 | NM_006470.2 | 545 | gccgcatcaggtgaacatcaaac | 13514 | - | see also 10861 line |
| 25068 | TRIM16 | NM_006470.2 | 490 | aggcagtgaagtcctgtctaacc | 13512 | - | see also 10861 line |
| 25069 | TRIM22 | NM_006074.2 | 1216 | tgggcgtacacagtaaaataagt | 13526 | - | see also 10862 line |
| 25070 | TRIM22 | NM_006074.2 | 1215 | ctgggcgtacacagtaaaataag | 13525 | - | see also 10862 line |
| 25071 | TRIM22 | NM_006074.2 | 1213 | tcctgggcgtacacagtaaaata | 13524 | - | see also 10862 line |
| 25072 | TRIM28 | NM_005762.2 | 1521 | agcgtcctggcactaactcaaca | 13540 | - | see also 10863 line |
| 25073 | TRIM28 | NM_005762.2 | 1518 | cagagcgtcctggcactaactca | 13538 | - | see also 10863 line |
| 25074 | TRIM29 | NM_012101.2 | 1558 | aggtggggtcggacatcatacc | 13570 | - | see also 10864 line |
| 25075 | TRIM29 | NM_012101.2 | 1411 | ggcgacctgagccgtaacttca | 13560 | - | see also 10864 line |
| 25076 | TRPS1 | NM_014112.1 | 2462 | aagctcgcgagtcaaacatcagt | 13635 | - | see also 5496 line |
| 25077 | TRPS1 | NM_014112.1 | 2459 | tggaagctcgcgagtcaaacatc | 13634 | - | see also 5496 line |
| 25078 | TSC22 | NM_006022.2 | 350 | tgcaagtgtgtagctattgaca | 13692 | - | see also 10866 line |
| 25079 | TSC22 | NM_006022.2 | 448 | aagagcaaatcaaagaactaata | 13693 | - | see also 10866 line |
| 25080 | TULP4 | NM_020245.2 | 3144 | ttgctcaggtcacgtctaatatc | 13795 | - | see also 7820 line |
| 25081 | UBP1 | NM_014517.2 | 1810 | accccgtttaaccatctatgtct | 13854 | - | see also 5748 line |
| 25082 | UBP1 | NM_014517.2 | 2026 | gggtccaccgtattccacttc | 13863 | - | see also 5748 line |
| 25083 | UHRF1 | NM_013282.2 | 471 | gacggaattgggcgtgtacaagg | 13870 | - | see also 10869 line |
| 25084 | UHRF1 | NM_013282.2 | 665 | acggcgtggtccagatgaactcc | 13871 | - | see also 10869 line |
| 25085 | USF2 | NM_003367.1 | 760 | tggatcgtcagcttcgaaaat | 13888 | - | see also 2272 line |
| 25086 | USF2 | NM_003367.1 | 171 | ggcgttcggcgaccaacaacatcc | 13880 | - | see also 2272 line |
| 25087 | VAV1 | NM_005428.2 | 1630 | caccgccaacggcatgaacttcc | 13903 | - | see also 10871 line |
| 25088 | VAV1 | NM_005428.2 | 598 | ggcggaaggcgacgagatcttatg | 13894 | - | see also 10871 line |
| 25089 | VDR | NM_000376.1 | 1360 | gcccttgtctcgaagtgtttg | 13929 | - | see also 300 line |
| 25090 | VDR | NM_000376.1 | 817 | cagttacagcatccaaaaggtca | 13924 | - | see also 300 line |
| 25091 | VDR | NM_000376.1 | 1361 | ccccttgtctcgaagtgtttgg | 13930 | - | see also 300 line |
| 25092 | VENTX2 | NM_014468.2 | 764 | gccacagacgggggatgcatttt | 13933 | - | see also 10873 line |
| 25093 | VSX1 | NM_014588.4 | 616 | gagaaatgctggctgtgaaaact | 13934 | - | see also 10874 line |
| 25094 | WT1 | NM_000378.2 | 1180 | ctctgcggagcccaatacagaat | 13942 | - | see also 301 line |
| 25095 | WT1 | NM_000378.2 | 1412 | aggactgtgaacgaaggtttct | 13947 | - | see also 301 line |
| 25096 | XBP1 | NM_005080.2 | 78 | ggccgacgggggaccctaaagttc | 13951 | - | see also 3549 line |

Figure 46

| 25097 | XBP1 | NM_005080.2 | 621 | ggcggtattgactcttcagattc | 13958 | - | see also 3549 line |
|---|---|---|---|---|---|---|---|
| 25098 | YY1 | NM_003403.3 | 904 | gacgacgactacattgaacaaac | 13964 | - | see also 2304 line |
| 25099 | YY1 | NM_003403.3 | 1634 | gccccttcgatggttgtaataag | 13972 | - | see also 2304 line |
| 25100 | ZFH4 | NM_024721.2 | 1513 | aactcaccgatagtattggtaac | 14021 | - | see also 8698 line |
| 25101 | ZFHX1B | NM_014795.2 | 4006 | cccgaaacgatacgagatgaaga | 14333 | - | see also 5974 line |
| 25102 | ZFHX1B | NM_014795.2 | 2015 | gtcttccggtagtgagtcataat | 14277 | - | see also 5974 line |
| 25103 | ZFP36L1 | NM_004926.2 | 977 | tccgagtcccctcacatgtttga | 14349 | - | see also 10880 line |
| 25104 | ZFP36L1 | NM_004926.2 | 976 | gtccgagtcccctcacatgtttg | 14348 | - | see also 10880 line |
| 25105 | ZFP36L1 | NM_004926.2 | 161 | ttcgacttgagcgaagttttatg | 14342 | - | see also 10880 line |
| 25106 | ZFP36L2 | NM_006887.3 | 849 | gactcgccatccgaagtacaaga | 14352 | - | see also 10881 line |
| 25107 | ZFP36L2 | NM_006887.3 | 579 | ctcctacggcacccttaaggagc | 14351 | - | see also 10881 line |
| 25108 | ZFP37 | NM_003408.1 | 37 | ctccagcggcgtccagattctga | 14354 | - | see also 10882 line |
| 25109 | ZFP37 | NM_003408.1 | 656 | aagtcattcaaccatagcttatc | 14357 | - | see also 10882 line |
| 25110 | ZFP95 | NM_014569.2 | 230 | accgaatcccgagaagttataga | 14371 | - | see also 10883 line |
| 25111 | ZFP95 | NM_014569.2 | 229 | gaccgaatcccgagaagttatag | 14370 | - | see also 10883 line |
| 25112 | ZHX1 | NM_007222.2 | 1679 | gtggcaggcgttccaagtcaaaa | 14445 | - | see also 5034 line |
| 25113 | ZHX1 | NM_007222.2 | 735 | atgtttgtgtcgaatgcaatttt | 14412 | - | see also 5034 line |
| 25114 | ZHX2 | NM_014943.2 | 1945 | accaccgatatcggtgtcaaagg | 14490 | - | see also 6132 line |
| 25115 | ZHX2 | NM_014943.2 | 2840 | gtcgagcgttgtggattacgtgg | 14498 | - | see also 6132 line |
| 25116 | ZHX2 | NM_014943.2 | 510 | tgcatggttcggacatcacaagt | 14472 | - | see also 6132 line |
| 25117 | ZNF11B | NM_006955.1 | 494 | aacatggacgtaagttcttttcc | 14503 | - | see also 10885 line |
| 25118 | ZNF133 | NM_003434.3 | 1359 | accggaagtcaacgctaatcata | 14509 | - | see also 10887 line |
| 25119 | ZNF133 | NM_003434.3 | 2457 | aaccttcggattccttatactct | 14516 | - | see also 10887 line |
| 25120 | ZNF133 | NM_003434.3 | 2454 | ggcaaccttcggattccttatac | 14515 | - | see also 10887 line |
| 25121 | ZNF134 | NM_003435.1 | 1300 | ctgagtcaaagccgtttgagtgc | 14523 | - | see also 10888 line |
| 25122 | ZNF134 | NM_003435.1 | 614 | aagtgaggcctcaattgtgaaga | 14517 | - | see also 10888 line |
| 25123 | ZNF135 | NM_003436.2 | 774 | cacagctcagcacttatcgaaca | 14532 | - | see also 10889 line |
| 25124 | ZNF135 | NM_003436.2 | 624 | ctcccacatcaaccaatgactcc | 14528 | - | see also 10889 line |
| 25125 | ZNF135 | NM_003436.2 | 690 | gagaagccagacctaaatgtttt | 14530 | - | see also 10889 line |
| 25126 | ZNF137 | NM_003438.1 | 299 | aagtgggaagtcgtcacttttt | 14537 | - | see also 10890 line |
| 25127 | ZNF137 | NM_003438.1 | 302 | tgggaagtcgtcacttttttatc | 14539 | - | see also 10890 line |
| 25128 | ZNF137 | NM_003438.1 | 301 | gtgggaagtcgtcacttttttat | 14538 | - | see also 10890 line |
| 25129 | ZNF140 | NM_003440.2 | 1682 | ttccgttgtcactcattccttat | 14561 | - | see also 10891 line |
| 25130 | ZNF140 | NM_003440.2 | 1683 | tccgttgtcactcattccttatt | 14562 | - | see also 10891 line |
| 25131 | ZNF142 | NM_005081.1 | 1991 | ttcgcctgcaagcggaagtatga | 14583 | - | see also 10892 line |
| 25132 | ZNF142 | NM_005081.1 | 1686 | tccgacacatgcttctacatacg | 14579 | - | see also 10892 line |
| 25133 | ZNF145 | NM_006006.3 | 436 | caccgcaatagtcaacactatac | 14610 | - | see also 10893 line |

Figure 46

| 25134 | ZNF145 | NM_006006.3 | 437 | accgcaatagtcaacactatact | 14611 | - | see also 10893 line | | |
|---|---|---|---|---|---|---|---|---|---|
| 25135 | ZNF147 | NM_005082.2 | 1080 | caccatagacctcaaaaacgagc | 14636 | - | see also 10894 line | | |
| 25136 | ZNF147 | NM_005082.2 | 1532 | atcccagaggttcacatactgc | 14643 | - | see also 10894 line | | |
| 25137 | ZNF154 | NM_003444.1 | 1409 | ctcaaaattccggcctcattaag | 14655 | - | see also 10895 line | | |
| 25138 | ZNF154 | NM_003444.1 | 860 | ctgcagtacgacctcatgaatgt | 14653 | - | see also 10895 line | | |
| 25139 | ZNF155 | NM_003445.2 | 541 | caggtctcaggactctatcataa | 14656 | - | see also 10896 line | | |
| 25140 | ZNF155 | NM_003445.2 | 1083 | gtggtaagaccttctattttagg | 14659 | - | see also 10896 line | | |
| 25141 | ZNF157 | NM_003446.2 | 425 | tgccctcaggcatgataatgacc | 14668 | - | see also 10897 line | | |
| 25142 | ZNF157 | NM_003446.2 | 391 | cactgctgtgtggcaatggttct | 14667 | - | see also 10897 line | | |
| 25143 | ZNF165 | NM_003447.2 | 2406 | cacatcttattcgacactttaga | 14692 | - | see also 10898 line | | |
| 25144 | ZNF165 | NM_003447.2 | 1179 | ggcaggacacttgcttacagaga | 14677 | - | see also 10898 line | | |
| 25145 | ZNF167 | NM_018651.1 | 661 | ggggacttcgctcaatgtacttc | 14701 | - | see also 10899 line | | |
| 25146 | ZNF167 | NM_018651.1 | 662 | gggacttcgctcaatgtacttct | 14702 | - | see also 10899 line | | |
| 25147 | ZNF174 | NM_003450.1 | 1418 | gacattgcagggtggaatacatc | 14712 | - | see also 10900 line | | |
| 25148 | ZNF174 | NM_003450.1 | 996 | ttgtatgcaggggcaaaaggtgc | 14710 | - | see also 10900 line | | |
| 25149 | ZNF175 | NM_007147.2 | 568 | ttcgcagtggggtatcacattcc | 14718 | - | see also 10901 line | | |
| 25150 | ZNF175 | NM_007147.2 | 1426 | ttgcttacgcaccagaaaacaca | 14735 | - | see also 10901 line | | |
| 25151 | ZNF187 | NM_152736.2 | 218 | ttcggcatgagtgtgaagttaca | 14741 | - | see also 10902 line | | |
| 25152 | ZNF187 | NM_152736.2 | 220 | cggcatgagtgtgaagttacaaa | 14742 | - | see also 10902 line | | |
| 25153 | ZNF189 | NM_003452.2 | 1250 | ttcgattaagcacataccttata | 14767 | - | see also 2316 line | | |
| 25154 | ZNF189 | NM_003452.2 | 685 | atccgtaaaagaccaaactcaga | 14758 | - | see also 2316 line | | |
| 25155 | ZNF19 | NM_006961.2 | 1021 | gagtagttccgagtttgttatac | 14794 | - | see also 10904 line | | |
| 25156 | ZNF19 | NM_006961.2 | 923 | gggcgagcctttaatgataatgc | 14789 | - | see also 10904 line | | |
| 25157 | ZNF192 | NM_006298.1 | 553 | gaccctattagaggatttggaaa | 14802 | - | see also 10905 line | | |
| 25158 | ZNF192 | NM_006298.1 | 632 | ctctgggaggaggtagtacattc | 14804 | - | see also 10905 line | | |
| 25159 | ZNF193 | NM_006299.2 | 1047 | caccgaggaatccacaatataca | 14817 | - | see also 10906 line | | |
| 25160 | ZNF193 | NM_006299.2 | 1190 | cagtcggcgttcatttctcattg | 14821 | - | see also 10906 line | | |
| 25161 | ZNF197 | NM_006991.2 | 1227 | ttcagcgttcgagccttctaatg | 14838 | - | see also 10907 line | | |
| 25162 | ZNF197 | NM_006991.2 | 2053 | tggtaaggtcttcggttcaaaca | 14850 | - | see also 10907 line | | |
| 25163 | ZNF202 | NM_003455.1 | 1352 | aagatgaacgcgccttacaaata | 14871 | - | see also 10908 line | | |
| 25164 | ZNF202 | NM_003455.1 | 1086 | aggacaatccaggtagacttaat | 14868 | - | see also 10908 line | | |
| 25165 | ZNF206 | NM_032805.1 | 948 | atccggagttgcagttcatctgc | 14879 | - | transcription_regulator | - | - |
| 25166 | ZNF206 | NM_032805.1 | 1926 | gccaccgtttccgcaatagctcc | 14880 | - | see also 25165 line | | |
| 25167 | ZNF206 | NM_032805.1 | 312 | ggccgctggattttagttgtaat | 14878 | - | see also 25165 line | | |
| 25168 | ZNF207 | NM_003457.1 | 382 | aacaatagatgccgtaccaaatg | 14889 | - | see also 2338 line | | |
| 25169 | ZNF207 | NM_003457.1 | 357 | ttcattgcatgcaggtacataaa | 14887 | - | see also 2338 line | | |

Figure 46

| 25170 | ZNF211 | NM_006385.2 | 962 | agcatgtacgcgaagatgtaacc | 14927 | - | see also 10910 line |
|---|---|---|---|---|---|---|---|
| 25171 | ZNF211 | NM_006385.2 | 493 | atgccgactcctgtgaaatatgt | 14919 | - | see also 10910 line |
| 25172 | ZNF215 | NM_013250.1 | 1838 | accgacgtacaaaccttactaag | 14963 | - | see also 10912 line |
| 25173 | ZNF215 | NM_013250.1 | 717 | ctctgaggcatctcgtcaaaagt | 14938 | - | see also 10912 line |
| 25174 | ZNF215 | NM_013250.1 | 1834 | ttcaaccgacgtacaaaccttac | 14962 | - | see also 10912 line |
| 25175 | ZNF217 | NM_006526.2 | 2815 | ccctgcaccggataagacaaaaa | 15026 | - | see also 10913 line |
| 25176 | ZNF217 | NM_006526.2 | 2814 | cccctgcaccggataagacaaaa | 15025 | - | see also 10913 line |
| 25177 | ZNF219 | NM_016423.1 | 2022 | gtccggctcgctcaagtatcacc | 15052 | - | see also 10914 line |
| 25178 | ZNF219 | NM_016423.1 | 602 | agcgcttccgcttcaactctatc | 15045 | - | see also 10914 line |
| 25179 | ZNF232 | NM_014519.2 | 734 | aggatggctgtatcactaacagc | 15054 | - | see also 10915 line |
| 25180 | ZNF232 | NM_014519.2 | 1931 | tggtgctcagagctcattagaca | 15066 | - | see also 10915 line |
| 25181 | ZNF236 | NM_007345.1 | 826 | agccagttaacgcgacacattag | 15089 | - | see also 10916 line |
| 25182 | ZNF236 | NM_007345.1 | 827 | gccagttaacgcgacacattagg | 15090 | - | see also 10916 line |
| 25183 | ZNF236 | NM_007345.1 | 750 | gtcttataaccggaatatcgaca | 15088 | - | see also 10916 line |
| 25184 | ZNF24 | NM_006965.1 | 1026 | ttcagccgaagttccattcttgt | 15168 | - | see also 10917 line |
| 25185 | ZNF24 | NM_006965.1 | 263 | tggcgaagagggatcaagtatcc | 15160 | - | see also 10917 line |
| 25186 | ZNF256 | NM_005773.2 | 1907 | aaccactctagcctcgttaaaca | 15184 | - | see also 10918 line |
| 25187 | ZNF256 | NM_005773.2 | 1354 | cactggtacaaggccttatatgt | 15183 | - | see also 10918 line |
| 25188 | ZNF256 | NM_005773.2 | 569 | caccatggtcagaaactgtatac | 15178 | - | see also 10918 line |
| 25189 | ZNF263 | NM_005741.3 | 1927 | ctcgtcattcacgaaagaactca | 15204 | - | see also 3982 line |
| 25190 | ZNF263 | NM_005741.3 | 1489 | ttctctaacaactcaaacctaat | 15195 | - | see also 3982 line |
| 25191 | ZNF265 | NM_005455.2 | 768 | ctcgtgggtcgaaatcaagatcc | 15230 | - | see also 10920 line |
| 25192 | ZNF265 | NM_005455.2 | 818 | tccccacgaaaaagatcttattc | 15231 | - | see also 10920 line |
| 25193 | ZNF274 | NM_016324.2 | 1622 | cagtcggagtactaaacagatta | 15247 | - | see also 10921 line |
| 25194 | ZNF274 | NM_016324.2 | 1617 | accttcagtcggagtactaaaca | 15246 | - | see also 10921 line |
| 25195 | ZNF277 | NM_021994.1 | 195 | cacagtcggggtgtaggttatg | 15261 | - | see also 10922 line |
| 25196 | ZNF277 | NM_021994.1 | 532 | ttgttatgtgacgttttaccaga | 15266 | - | see also 10922 line |
| 25197 | ZNF287 | NM_020653.1 | 533 | gagacctgtcgacagaattttag | 15294 | - | see also 10923 line |
| 25198 | ZNF287 | NM_020653.1 | 987 | ctgtgacgttacagaactattgg | 15300 | - | see also 10923 line |
| 25199 | ZNF3 | NM_032924.2 | 1572 | cagagttacgaccgagttaaata | 15333 | - | see also 10924 line |
| 25200 | ZNF3 | NM_032924.2 | 1579 | acgaccgagttaaatatcagaga | 15335 | - | see also 10924 line |
| 25201 | ZNF3 | NM_017715.1 | 607 | ctgtgaattccaaccttatctca | 15340 | - | see also 10925 line |
| 25202 | ZNF3 | NM_017715.1 | 609 | gtgaattccaaccttatctcaca | 15341 | - | see also 10925 line |
| 25203 | ZNF305 | NM_014724.1 | 589 | gacggtacgtctacgaaaagaag | 15349 | - | see also 10926 line |
| 25204 | ZNF305 | NM_014724.1 | 234 | aggattgggacctgcgtaaaaac | 15344 | - | see also 10926 line |
| 25205 | ZNF305 | NM_014724.1 | 587 | gagacggtacgtctacgaaaaga | 15348 | - | see also 10926 line |
| 25206 | ZNF306 | NM_024493.1 | 1083 | ggcggcacatctgccatgaatgt | 15374 | - | see also 10927 line |

EP 1 752 536 A1

Figure 46

| 25207 | ZNF306 | NM_024493.1 | 1566 | atgttgaaactcccatgtcttat | 15377 | - | see also 10927 line |
|---|---|---|---|---|---|---|---|
| 25208 | ZNF31 | NM_145238.2 | 2854 | gaccgttctaacctcattactca | 15397 | - | see also 10928 line |
| 25209 | ZNF31 | NM_145238.2 | 1446 | gtccccattctcggaaaagcttc | 15386 | - | see also 10928 line |
| 25210 | ZNF323 | NM_030899.2 | 873 | gacgccacaggtgcaatgaatgt | 15401 | - | see also 10929 line |
| 25211 | ZNF323 | NM_030899.2 | 789 | atgtatctttggattctaagtac | 15400 | - | see also 10929 line |
| 25212 | ZNF33A | NM_006974.1 | 801 | tccgtcaagggacaatcactatg | 15407 | - | see also 10930 line |
| 25213 | ZNF33A | NM_006974.1 | 1353 | gacttcgcaccttaaagtacacc | 15411 | - | see also 10930 line |
| 25214 | ZNF35 | NM_003420.2 | 1181 | cacattacggaaaaatgctatga | 15424 | - | see also 10931 line |
| 25215 | ZNF35 | NM_003420.2 | 457 | aacccttggcagctactcattac | 15414 | - | see also 10931 line |
| 25216 | ZNF38 | NM_145914.2 | 290 | ggcagtttgggtaccatgatacc | 15428 | - | see also 10932 line |
| 25217 | ZNF38 | NM_145914.2 | 700 | gagcaggagttcgctcaagatcc | 15431 | - | see also 10932 line |
| 25218 | ZNF394 | NM_032164.2 | 251 | gtcccaacgcgacggactttttgc | 15444 | - | see also 10933 line |
| 25219 | ZNF394 | NM_032164.2 | 733 | cagttccgccgagtttggaaagc | 15446 | - | see also 10933 line |
| 25220 | ZNF396 | NM_145756.1 | 1022 | atcgacgaacccatactgattca | 15480 | - | see also 10934 line |
| 25221 | ZNF396 | NM_145756.1 | 751 | ggcatagaactgcattgcaatgt | 15474 | - | see also 10934 line |
| 25222 | ZNF397 | NM_032347.1 | 183 | tccggaacaagaactaatactag | 15487 | - | see also 10935 line |
| 25223 | ZNF397 | NM_032347.1 | 765 | ttcatttcgaggaattaagttgt | 15493 | - | see also 10935 line |
| 25224 | ZNF397 | NM_032347.1 | 180 | tcctccggaacaagaactaatac | 15486 | - | see also 10935 line |
| 25225 | ZNF41 | NM_007130.1 | 2265 | cgcacttcattgcgcatcataga | 15520 | - | see also 10936 line |
| 25226 | ZNF41 | NM_007130.1 | 1132 | ctgtgaacatgaccactatgaaa | 15512 | - | see also 10936 line |
| 25227 | ZNF42 | NM_003422.2 | 1042 | tccgcaggtccaggtagtgtaag | 15527 | - | see also 10937 line |
| 25228 | ZNF434 | NM_017810.2 | 1757 | agccgaagttcttatcttgttcg | 15545 | - | see also 10938 line |
| 25229 | ZNF434 | NM_017810.2 | 1751 | acctttagccgaagttcttatct | 15543 | - | see also 10938 line |
| 25230 | ZNF435 | NM_025231.1 | 499 | ttcagaaagacgggacatactca | 15549 | - | see also 10939 line |
| 25231 | ZNF435 | NM_025231.1 | 174 | aggattccagctcacaaaagtgc | 15546 | - | see also 10939 line |
| 25232 | ZNF45 | NM_003425.2 | 954 | atgccttccgtcggttttcaagt | 15571 | - | see also 10940 line |
| 25233 | ZNF45 | NM_003425.2 | 759 | aacgagatgatttgcactataaa | 15568 | - | see also 10940 line |
| 25234 | ZNF494 | NM_152677.1 | 1293 | ttcgaaaactactcgagtaaatg | 15598 | - | see also 10941 line |
| 25235 | ZNF494 | NM_152677.1 | 1757 | acctacgggtgcatcagataatt | 15617 | - | see also 10941 line |
| 25236 | ZNF494 | NM_152677.1 | 1581 | ttccacatgtgaggtacatcaga | 15606 | - | see also 10941 line |
| 25237 | ZNF495 | NM_024303.1 | 1338 | agcgcttcatgcagcttattagc | 15623 | - | see also 10942 line |
| 25238 | ZNF495 | NM_024303.1 | 1595 | ttcagtcggctgaaattgttaag | 15625 | - | see also 10942 line |
| 25239 | ZNF496 | NM_032752.1 | 1474 | ggcggcaacccgcgatctctaga | 15628 | - | see also 10943 line |
| 25240 | ZNF496 | NM_032752.1 | 488 | gagtcttggctccgaaggaaagt | 15626 | - | see also 10943 line |
| 25241 | ZNF70 | NM_021916.2 | 510 | aacaggttagagtcacaacaagg | 15633 | - | see also 10944 line |
| 25242 | ZNF70 | NM_021916.2 | 643 | atgattacgaggggaatttcagt | 15634 | - | see also 10944 line |
| 25243 | ZNF75 | NM_007131.1 | 664 | atcaggatgcgaagtatcaaaaa | 15642 | - | see also 10945 line |

Figure 46

| | | | | | | | | | |
|---|---|---|---|---|---|---|---|---|---|
| 25244 | ZNF75 | NM_007131.1 | 1197 | agccttacgtgtagcttatgc | 15646 | - | see also 10945 line | | |
| 25245 | ZNF80 | NM_007136.1 | 671 | atgtgggagcgtgtttaacaaaa | 15650 | - | see also 10946 line | | |
| 25246 | ZNF80 | NM_007136.1 | 1034 | ttccttaacccgcacatgaaga | 15653 | - | see also 10946 line | | |
| 25247 | ZNF80 | NM_007136.1 | 1117 | ctgttttctccgacatagtatg | 15654 | - | see also 10946 line | | |
| 25248 | ZNF83 | NM_018300.2 | 1926 | ttccgcgacaattcatatcttgt | 15667 | - | see also 10947 line | | |
| 25249 | ZNF83 | NM_018300.2 | 1923 | ctcttccgacaattcatatct | 15666 | - | see also 10947 line | | |
| 25250 | ZNF83 | NM_018300.2 | 1921 | aactcttccgcgacaattcatat | 15665 | - | see also 10947 line | | |
| 25251 | ZNF85 | NM_003429.1 | 1015 | aaaccttacctacccatagaaaaa | 15672 | - | transcription_regulator | transcription factor | - |
| 25252 | ZNF91 | NM_003430.1 | 686 | acccaacataaatgcgtttatat | 15683 | - | transcription_regulator | zinc binding | - |
| 25253 | ZNF91 | NM_003430.1 | 3551 | aagaaaattcattactatccacacc | 15684 | - | see also 25252 line | | |
| 25254 | ZNFN1A3 | NM_012481.3 | 1055 | ctggagatggttccagttatca | 15709 | - | see also 10949 line | | |
| 25255 | ZNFN1A3 | NM_012481.3 | 128 | tgcagcggtttgaatgactaca | 15688 | - | see also 10949 line | | |
| 25256 | ZNFN1A4 | NM_022465.2 | 623 | tgcggcatggtctgtattggac | 15723 | - | see also 10950 line | | |
| 25257 | ZNFN1A4 | NM_022465.2 | 620 | gtctgcggcatggtctgtattgg | 15722 | - | see also 10950 line | | |
| 25258 | ZNRD1 | NM_014596.4 | 462 | agccgatgaagggcaaactgtct | 15731 | - | see also 10951 line | | |
| 25259 | ZNRD1 | NM_014596.4 | 409 | tgccctcgatgtggtcatgaagg | 15730 | - | see also 10951 line | | |
| 25260 | ANKRD17 | NM_032217.3 | 1289 | ctggcattaatacgcattctaat | 15762 | - | see also 9099 line | | |
| 25261 | ANKRD17 | NM_032217.3 | 5423 | gagaccggataatcactataagg | 15856 | - | see also 9099 line | | |
| 25262 | BRCA1 | NM_007294.1 | 4423 | agctacccttccatcataagtga | 16043 | - | see also 5077 line | | |
| 25263 | BRCA1 | NM_007294.1 | 5544 | cacaggtgtccacccaattgtgg | 16066 | - | see also 5077 line | | |
| 25264 | DDB1 | NM_001923.2 | 538 | tccactagatcgcgataataaag | 16080 | - | see also 1223 line | | |
| 25265 | DDB1 | NM_001923.2 | 537 | ttccactagatcgcgataataaa | 16079 | - | see also 1223 line | | |
| 25266 | DDB2 | NM_000107.1 | 206 | aagacctccgagattgtattacg | 16134 | - | see also 10954 line | | |
| 25267 | DDB2 | NM_000107.1 | 1292 | gacgtgttcgatggaaactcagg | 16155 | - | see also 10954 line | | |
| 25268 | DMC1 | NM_007068.2 | 694 | tgctgatcgtttaagttagacc | 16179 | - | see also 10955 line | | |
| 25269 | DMC1 | NM_007068.2 | 398 | gaggcagcgaacaactaattga | 16167 | - | see also 10955 line | | |
| 25270 | ERCC1 | NM_001983.1 | 932 | atcgccgatcaagaagaagatct | 16206 | - | see also 10956 line | | |
| 25271 | ERCC1 | NM_001983.1 | 926 | cagctcatcgccgatcaagaga | 16204 | - | see also 10956 line | | |
| 25272 | ERCC3 | NM_000122.1 | 2017 | ggcgggtgcttcgagctaaaaaa | 16253 | - | see also 10957 line | | |
| 25273 | ERCC3 | NM_000122.1 | 1091 | tgcacgttcggggtcattgttc | 16232 | - | see also 10957 line | | |
| 25274 | ERCC3 | NM_000122.1 | 1499 | gaccctcgtccgcgaagatgaca | 16235 | - | see also 10957 line | | |
| 25275 | FANCG | NM_004629.1 | 2124 | cccaggtaatcgagacaacttact | 16284 | - | see also 3144 line | | |
| 25276 | FANCG | NM_004629.1 | 2116 | cagatgtgccagttaatcgaga | 16283 | - | see also 3144 line | | |
| 25277 | FEN1 | NM_004111.4 | 454 | ggccgtaaggtggccattgatgc | 16293 | - | see also 10959 line | | |
| 25278 | FEN1 | NM_004111.4 | 1449 | acccaagggatccactaagaaga | 16300 | - | see also 10959 line | | |

Figure 46

| | | | | | | | | |
|---|---|---|---|---|---|---|---|---|
| 25279 | FEN1 | NM_004111.4 | 1448 | aacccaagggatccactaagaag | 16299 | - | see also 10959 line | |
| 25280 | JTV1 | NM_006303.2 | 795 | gtcaacgcaacccttatagatag | 16320 | - | see also 4395 line | |
| 25281 | JTV1 | NM_006303.2 | 400 | atgagcccacgactttaaccacc | 16308 | - | see also 4395 line | |
| 25282 | MPG | NM_002434.1 | 352 | gcccataccgcagcatctatttc | 16327 | - | see also 10961 line | |
| 25283 | MPG | NM_002434.1 | 577 | cccgcaaccgaggcatgttcatg | 16331 | - | see also 10961 line | |
| 25284 | MPG | NM_002434.1 | 351 | ggcccataccgcagcatctattt | 16326 | - | see also 10961 line | |
| 25285 | MSH2 | NM_000251.1 | 1282 | accgactctatcagggtataaat | 16366 | - | see also 183 line | |
| 25286 | MSH2 | NM_000251.1 | 2645 | atcgcaaggatatgatatcatgg | 16419 | - | see also 183 line | |
| 25287 | MSH2 | NM_000251.1 | 876 | ctgtctgcggtaatcaagttttt | 16354 | - | see also 183 line | |
| 25288 | MSH3 | NM_002439.1 | 671 | atctatacgccgctagaattaca | 16433 | - | see also 10963 line | |
| 25289 | MSH3 | NM_002439.1 | 1988 | ttgctccggaccgttattttaga | 16490 | - | see also 10963 line | |
| 25290 | MSH4 | NM_002440.2 | 1087 | gacgacttcgttctaatatatta | 16567 | - | see also 10964 line | |
| 25291 | MSH4 | NM_002440.2 | 2713 | tagccactaggcttgttcaaact | 16626 | - | see also 10964 line | |
| 25292 | MSH5 | NM_002441.2 | 1089 | ttctcgtctggacgtcattcagt | 16645 | - | see also 1574 line | |
| 25293 | MSH5 | NM_002441.2 | 1091 | ctcgtctggacgtcattcagttt | 16646 | - | see also 1574 line | |
| 25294 | MSH6 | NM_000179.1 | 3185 | tgcggcgactgttctataacttt | 16779 | - | see also 153 line | |
| 25295 | MSH6 | NM_000179.1 | 3502 | ggcaagtctacgcttatgagaca | 16795 | - | see also 153 line | |
| 25296 | MSH6 | NM_000179.1 | 712 | gaggtaggcacaacttacgtaac | 16685 | - | see also 153 line | |
| 25297 | N4BP2 | NM_018177.2 | 1437 | atcttacgttggactagttcttg | 16851 | - | see also 10966 line | |
| 25298 | N4BP2 | NM_018177.2 | 3770 | accatgaatcgatgacaagtata | 16920 | - | see also 10966 line | |
| 25299 | NBS1 | NM_002485.3 | 608 | ttgtggacgtccaattgtaaagc | 16978 | - | see also 1591 line | |
| 25300 | NBS1 | NM_002485.3 | 225 | cagtcgatcagccgaaatcatgc | 16960 | - | see also 1591 line | |
| 25301 | OGG1 | NM_002542.4 | 1664 | tgcgacaagaccccatcgaatgc | 17029 | - | see also 10968 line | |
| 25302 | OGG1 | NM_002542.4 | 1434 | tggtgtactagcggatcaagtat | 17027 | - | see also 10968 line | |
| 25303 | PMS2L9 | NM_005395.1 | 423 | ttctgtggggtatgattatcacc | 17049 | - | transcription_regulator | - | - |
| 25304 | PNKP | NM_007254.2 | 927 | acgacggcacgcccatatccatc | 17053 | - | see also 10969 line | |
| 25305 | PNKP | NM_007254.2 | 1359 | gggtcgccatcgacaacacaaac | 17055 | - | see also 10969 line | |
| 25306 | POLH | NM_006502.1 | 1135 | gccatgtgccgagggattgaaca | 17075 | - | see also 4548 line | |
| 25307 | POLH | NM_006502.1 | 642 | gcctatctcggcagacttgttgc | 17062 | - | see also 4548 line | |
| 25308 | POLQ | NM_006596.3 | 1761 | acctgcacgtcgtgtgattattc | 17149 | - | see also 4627 line | |
| 25309 | POLQ | NM_006596.3 | 7794 | cgctgagcgtcaagctatcaaca | 17357 | - | see also 4627 line | |
| 25310 | POLQ | NM_006596.3 | 2970 | acgtcgcaatatgcgaactatct | 17197 | - | see also 4627 line | |
| 25311 | RAD1 | NM_002853.2 | 446 | tgccacgtgtttcgcaactaaaa | 17370 | - | see also 1883 line | |
| 25312 | RAD1 | NM_002853.2 | 1033 | tagtcctatcttgtaaggtatct | 17394 | - | see also 1883 line | |
| 25313 | RAD18 | NM_020165.2 | 743 | ctgtttatcacgcgaagagaaga | 17423 | - | see also 10973 line | |
| 25314 | RAD18 | NM_020165.2 | 941 | atgcgatgctttgcatcctaaat | 17434 | - | see also 10973 line | |

| 25315 | RAD51 | NM_002875.2 | 896 | atctaggtatgcactgcttattg | 17471 | - | see also 1917 line |
|---|---|---|---|---|---|---|---|
| 25316 | RAD51 | NM_002875.2 | 813 | ctggataatgtagcatatgctcg | 17469 | - | see also 1917 line |
| 25317 | RAD51C | NM_002876.2 | 171 | ctccgagcttagcaaagaagttg | 17480 | - | see also 10975 line |
| 25318 | RAD51C | NM_002876.2 | 375 | tgggggtggagtgcccttaatga | 17485 | - | see also 10975 line |
| 25319 | RAD51L1 | NM_002877.3 | 932 | gtgatagccgcactaggaaatac | 17536 | - | see also 10976 line |
| 25320 | RAD51L1 | NM_002877.3 | 813 | cagttatcttgacgaatcagatt | 17532 | - | see also 10976 line |
| 25321 | RAD51L3 | NM_002878.2 | 1130 | atggcgtgtctggccaaatcttc | 17549 | - | see also 10977 line |
| 25322 | RAD51L3 | NM_002878.2 | 1131 | tggcgtgtctggccaaatcttcc | 17550 | - | see also 10977 line |
| 25323 | REV1L | NM_016316.1 | 2906 | gccgaccagtcctgatactaaca | 17629 | - | see also 6805 line |
| 25324 | REV1L | NM_016316.1 | 1852 | aggaagtcgcacaaacattgtat | 17599 | - | see also 6805 line |
| 25325 | TIAF1 | NM_004740.2 | 182 | gccgggtccaggttcttaagaat | 17661 | - | see also 10979 line |
| 25326 | TIAF1 | NM_004740.2 | 328 | tgcaatgacctatttaaggtaaa | 17665 | - | see also 10979 line |
| 25327 | XPA | NM_000380.2 | 835 | accgtaagacttgtactatgtgt | 17742 | - | see also 10980 line |
| 25328 | XPA | NM_000380.2 | 854 | gtgtggccatgaactgacatatg | 17743 | - | see also 10980 line |
| 25329 | XPC | NM_004628.2 | 584 | ttcggagggcgatgaaacgtttc | 17754 | - | see also 10981 line |
| 25330 | XPC | NM_004628.2 | 589 | agggcgatgaaacgtttcaataa | 17757 | - | see also 10981 line |
| 25331 | XRCC1 | NM_006297.1 | 1925 | ttcgtccccgatggatctacagt | 17806 | - | see also 10982 line |
| 25332 | XRCC1 | NM_006297.1 | 418 | cccaaccgcgttcgcatgtttgg | 17794 | - | see also 10982 line |
| 25333 | XRCC1 | NM_006297.1 | 478 | tgggaccgggtcaaaattgtttg | 17798 | - | see also 10982 line |
| 25334 | XRCC2 | NM_005431.1 | 351 | atgctccggctagttacaattct | 17811 | - | see also 10983 line |
| 25335 | XRCC2 | NM_005431.1 | 661 | ttgcaacgacacaaactataatg | 17821 | - | see also 10983 line |
| 25336 | XRCC3 | NM_005432.2 | 369 | ctggacctgaatcccagaattat | 17831 | - | see also 10984 line |
| 25337 | XRCC3 | NM_005432.2 | 1001 | agccccattccgctgtgaatttg | 17840 | - | see also 10984 line |
| 25338 | ASF1A | NM_014034.1 | 475 | gtgctaattacttgtacctatcg | 17847 | - | see also 5464 line |
| 25339 | ASF1A | NM_014034.1 | 388 | cccgcaggaaggcatatgtttgt | 17845 | - | see also 5464 line |
| 25340 | BAF53A | NM_004301.2 | 661 | gtcgttctactgggctgattttg | 17870 | - | see also 10986 line |
| 25341 | BAF53A | NM_004301.2 | 363 | ggcggtcccacctactacataga | 17860 | - | see also 10986 line |
| 25342 | CBX3 | NM_007276.3 | 112 | atggcctccaacaaaactacatt | 17898 | - | transcription_regulator - | - |
| 25343 | CBX3 | NM_007276.3 | 113 | tggcctccaacaaaactacattg | 17899 | - | see also 25342 line |
| 25344 | CBX4 | NM_003655.1 | 262 | gagaggctggtcgcccaaatata | 17900 | - | see also 2476 line |
| 25345 | CBX4 | NM_003655.1 | 1359 | acgccgtcggcctgaatctctcc | 17912 | - | see also 2476 line |
| 25346 | CBX5 | NM_012117.1 | 416 | ctcaaacagtgccgatgacatca | 17919 | - | see also 10988 line |
| 25347 | CBX5 | NM_012117.1 | 589 | ttgcaaaagaagctaatgtgaaa | 17922 | - | see also 10988 line |
| 25348 | CBX6 | NM_014292.2 | 630 | ccctcgcggaaccgcgttatagg | 17925 | - | see also 5600 line |
| 25349 | CBX7 | NM_175709.1 | 535 | tgcggctctcgcgcaagaagttc | 17928 | - | see also 10989 line |
| 25350 | CBX7 | NM_175709.1 | 512 | gccccgaaaggcccacaagtacc | 17927 | - | see also 10989 line |

EP 1 752 536 A1

Figure 46

| 25351 | CBX8 | NM_020649.1 | 155 | tacggaaaggacgcatggaatac | 17930 | - | see also 10990 line |
|---|---|---|---|---|---|---|---|
| 25352 | CBX8 | NM_020649.1 | 1230 | cacggaccaaggctttttaaag | 17938 | - | see also 10990 line |
| 25353 | CDYL | NM_004824.2 | 1590 | accgttatgtttcccaagataat | 17964 | - | see also 10991 line |
| 25354 | CDYL | NM_004824.2 | 1312 | ccgttggcagcgtcttctgttgt | 17954 | - | see also 10991 line |
| 25355 | CDYL2 | NM_152342.1 | 903 | cagtcagacctcggataacaatg | 17981 | - | see also 10992 line |
| 25356 | CDYL2 | NM_152342.1 | 175 | gagtatcttatccgatggaaagg | 17969 | - | see also 10992 line |
| 25357 | CENPA | NM_001809.2 | 380 | ctcgtggtgtggacttcaattgg | 17990 | - | see also 10993 line |
| 25358 | CENPA | NM_001809.2 | 378 | cactcgtggtgtggacttcaatt | 17989 | - | see also 10993 line |
| 25359 | CENPA | NM_001809.2 | 411 | ggccctattggccctacaagagg | 17991 | - | see also 10993 line |
| 25360 | CENPB | NM_001810.4 | 582 | caccgagaccagtctatggtacg | 17994 | - | see also 10994 line |
| 25361 | CHAF1A | NM_005483.1 | 263 | aacgggaagggtcccttagataa | 18002 | - | see also 10995 line |
| 25362 | CHAF1A | NM_005483.1 | 98 | ccgtttaagcgcctgaatcttgt | 17997 | - | see also 10995 line |
| 25363 | CHAF1B | NM_005441.1 | 642 | gtgctgcgagtatacagtataca | 18044 | - | see also 10996 line |
| 25364 | CHAF1B | NM_005441.1 | 640 | gggtgctgcgagtatacagtata | 18043 | - | see also 10996 line |
| 25365 | CHD1 | NM_001270.1 | 2241 | ttctgttacgccgagttaagaaa | 18129 | - | see also 832 line |
| 25366 | CHD1 | NM_001270.1 | 2514 | ttcgtagtagcggaaaattgatt | 18144 | - | see also 832 line |
| 25367 | CHD1 | NM_001270.1 | 859 | tcgtcgccaagcaactgttaatg | 18085 | - | see also 832 line |
| 25368 | CHD2 | NM_001271.1 | 1382 | agcggctaaaaacgttagttaca | 18239 | - | see also 10998 line |
| 25369 | CHD2 | NM_001271.1 | 5732 | tggggaccggcgacatatggatg | 18343 | - | see also 10998 line |
| 25370 | CHD5 | NM_015557.1 | 5943 | ggccctatgtgaccgatatctag | 18377 | - | see also 10999 line |
| 25371 | CHD5 | NM_015557.1 | 370 | cagtgactactccccgaataaaa | 18351 | - | see also 10999 line |
| 25372 | CHD6 | NM_032221.3 | 6451 | gccaaaggaccgcgtgataatta | 18498 | - | see also 9120 line |
| 25373 | CHD6 | NM_032221.3 | 6450 | ggccaaaggaccgcgtgataatt | 18497 | - | see also 9120 line |
| 25374 | CHD6 | NM_032221.3 | 1533 | ctcgcgagtataagaacagtaac | 18401 | - | see also 9120 line |
| 25375 | EZH1 | NM_001991.2 | 2083 | gacgcggaaaggtctatgacaaa | 18558 | - | see also 1278 line |
| 25376 | EZH1 | NM_001991.2 | 2081 | tcgacgcggaaaggtctatgaca | 18557 | - | see also 1278 line |
| 25377 | HTATIP | NM_006388.2 | 801 | caggacggaagcgaaaatcgaat | 18568 | - | see also 4476 line |
| 25378 | HTATIP | NM_006388.2 | 802 | aggacggaagcgaaaatcgaatt | 18569 | - | see also 4476 line |
| 25379 | HTATIP | NM_006388.2 | 804 | gacggaagcgaaaatcgaattgt | 18570 | - | see also 4476 line |
| 25380 | ING1L | NM_001564.1 | 559 | gaccagtgaaagccgtgatttat | 18597 | - | see also 11003 line |
| 25381 | ING1L | NM_001564.1 | 236 | ctgcgagagctggacaacaaata | 18584 | - | see also 11003 line |
| 25382 | MGC10561 | NM_032647.2 | 142 | cgcggctggtcctccaaacataa | 18605 | - | see also 11004 line |
| 25383 | MPO | NM_000250.1 | 1606 | cccacgtaccgttcctacaatga | 18612 | - | see also 11006 line |
| 25384 | MPO | NM_000250.1 | 1608 | cacgtaccgttcctacaatgact | 18613 | - | see also 11006 line |
| 25385 | MYST1 | NM_032188.1 | 563 | cacatcgggaactacgaaattga | 18627 | - | see also 11007 line |
| 25386 | MYST1 | NM_032188.1 | 561 | tccacatcgggaactacgaaatt | 18626 | - | see also 11007 line |

Figure 46

| | | | | | | | | |
|---|---|---|---|---|---|---|---|---|
| 25387 | MYST1 | NM_032188.1 | 852 | acgtggagccgttcgtcttttac | 18635 | - | see also 11007 line | |
| 25388 | NCOA6 | NM_014071.2 | 6056 | accagtgtacgctcgatagtaac | 18757 | - | see also 5477 line | |
| 25389 | NCOA6 | NM_014071.2 | 6105 | ccgccgtaccgaccacaaaaagc | 18759 | - | see also 5477 line | |
| 25390 | NCOA6 | NM_014071.2 | 6104 | tccgccgtaccgaccacaaaaag | 18758 | - | see also 5477 line | |
| 25391 | NSBP1 | NM_030763.1 | 129 | gccaggttgtctgctatgcttgt | 18769 | - | see also 11009 line | |
| 25392 | NSBP1 | NM_030763.1 | 165 | gaggtgaagcctaaaagaacatc | 18772 | - | see also 11009 line | |
| 25393 | PB1 | NM_018165.2 | 2404 | cgctaccgtcggcttgatttatt | 18839 | - | see also 7297 line | |
| 25394 | PB1 | NM_018165.2 | 2407 | taccgtcggcttgatttatttca | 18840 | - | see also 7297 line | |
| 25395 | RBP1L1 | NM_016374.4 | 4150 | aagtgccgaacgcatcacaattc | 18985 | - | see also 6844 line | |
| 25396 | RBP1L1 | NM_016374.4 | 4153 | tgccgaacgcatcacaattcttc | 18986 | - | see also 6844 line | |
| 25397 | SMARCC1 | NM_003074.2 | 545 | atctcgaatggatcgtaatgtgg | 19002 | - | see also 2062 line | |
| 25398 | SMARCC1 | NM_003074.2 | 1713 | tggggactcgttaattaccaagt | 19042 | - | see also 2062 line | |
| 25399 | SMARCC2 | NM_003075.2 | 1927 | aggacgagtgcatcttgcatttt | 19098 | - | see also 2063 line | |
| 25400 | SMARCE1 | NM_003079.3 | 283 | ctctggtatcacgattccaaaac | 19106 | - | see also 11014 line | |
| 25401 | SMARCE1 | NM_003079.3 | 904 | aagcacagattcatttaacaatg | 19108 | - | see also 11014 line | |
| 25402 | SUV39H1 | NM_003173.1 | 1227 | gcgggtccgtattgaatgcaagt | 19131 | - | see also 11015 line | |
| 25403 | SUV39H1 | NM_003173.1 | 229 | gtgaaatggcgtggatatccaga | 19118 | - | see also 11015 line | |
| 25404 | SUV39H2 | NM_024670.3 | 1097 | ctcgtcttccccgaatagcattg | 19157 | - | see also 8695 line | |
| 25405 | SUV39H2 | NM_024670.3 | 309 | cccgttactgcttcagcaattct | 19133 | - | see also 8695 line | |
| 25406 | BRCA2 | NM_000059.1 | 7852 | acgtatggcgtttctaaacattg | 19383 | - | see also 34 line | |
| 25407 | BRCA2 | NM_000059.1 | 9432 | ttgttctgaggtggacctaatag | 19430 | - | see also 34 line | |
| 25408 | ERCC5 | NM_000123.1 | 3438 | aagaatacatgcggtggattttt | 19552 | - | see also 98 line | |
| 25409 | ERCC5 | NM_000123.1 | 2733 | ttggaccggaataagttaataaa | 19530 | - | see also 98 line | |
| 25410 | HNRPDL | NM_005463.2 | 919 | tgccgacagctccgtcactgg | 19559 | - | see also 3802 line | |
| 25411 | IGHMBP2 | NM_002180.1 | 282 | ttcccagtaacagctttacttct | 19583 | - | see also 11020 line | |
| 25412 | IGHMBP2 | NM_002180.1 | 712 | cacgactgtggttgagatcattc | 19593 | - | see also 11020 line | |
| 25413 | KHDRBS1 | NM_006559.1 | 1415 | gagcacccatatggacgttatta | 19631 | - | see also 11021 line | |
| 25414 | KHDRBS1 | NM_006559.1 | 1416 | agcacccatatggacgttattaa | 19632 | - | see also 11021 line | |
| 25415 | PC4 | NM_006713.2 | 267 | tacgttagtgttcgcgatttttaa | 19633 | - | transcription_regulator | translation regulator | - |
| 25416 | PC4 | NM_006713.2 | 268 | acgttagtgttcgcgatttttaaa | 19634 | - | see also 25415 line | |
| 25417 | PCBP1 | NM_006196.2 | 939 | gaccggattcgccggaattgact | 19642 | - | see also 4288 line | |
| 25418 | PCBP1 | NM_006196.2 | 1064 | ggcgccaaggcgccaacattaat | 19648 | - | see also 4288 line | |
| 25419 | PIR51 | NM_006479.2 | 276 | aagctctaccagagagacttaga | 19658 | - | see also 11022 line | |
| 25420 | PIR51 | NM_006479.2 | 415 | ctcctcatatctctaattgcagt | 19660 | - | see also 11022 line | |
| 25421 | POLG2 | NM_007215.1 | 531 | cagaaactctacgcgaaatcttg | 19673 | - | see also 5024 line | |
| 25422 | POLG2 | NM_007215.1 | 782 | aagactgaagcttcgttagtatg | 19684 | - | see also 5024 line | |

# Figure 46

| 25423 | RAD23A | NM_005053.2 | 1125 | aggcctatttcgcgtgtgaaaaa | 19713 | - | - | single-stranded DNA binding | | - |
|---|---|---|---|---|---|---|---|---|---|---|
| 25424 | RAD23A | NM_005053.2 | 1126 | ggcctatttcgcgtgtgaaaaaa | 19714 | - | see also 25423 line | | | |
| 25425 | RAD23A | NM_005053.2 | 1127 | gcctatttcgcgtgtgaaaaaaa | 19715 | - | see also 25423 line | | | |
| 25426 | RAD23B | NM_002874.2 | 913 | cagtcttacgagaatatggtaac | 19726 | - | see also 1914 line | | | |
| 25427 | RAD23B | NM_002874.2 | 864 | ttctcggtcaaacctttttgaag | 19722 | - | see also 1914 line | | | |
| 25428 | RBMS1 | NM_002897.2 | 1214 | ggctgtcgagacgtctaatgacc | 19759 | - | see also 1936 line | | | |
| 25429 | RBMS1 | NM_002897.2 | 1034 | gtcactacagcccgcatcaatga | 19754 | - | see also 1936 line | | | |
| 25430 | RPA1 | NM_002945.2 | 1784 | tacaacgacgagtctcgaattaa | 19815 | - | see also 1989 line | | | |
| 25431 | RPA1 | NM_002945.2 | 137 | cccattactacggggaatagtcc | 19764 | - | see also 1989 line | | | |
| 25432 | RPA2 | NM_002946.3 | 289 | aacagtggattcgaaagctatgg | 19817 | - | see also 1990 line | | | |
| 25433 | RPA2 | NM_002946.3 | 283 | atgtggaacagtggattcgaaag | 19816 | - | see also 1990 line | | | |
| 25434 | RPA3 | NM_002947.2 | 1344 | ttgatggaaccccttgatgaaga | 19838 | - | see also 11028 line | | | |
| 25435 | RPA3 | NM_002947.2 | 1287 | cccaccggaaaaatgtttattct | 19837 | - | see also 11028 line | | | |
| 25436 | SMUG1 | NM_014311.1 | 601 | cactgttttgtccacaatctatg | 19845 | - | - | | - | - |
| 25437 | SMUG1 | NM_014311.1 | 477 | ccctccccaagagcatcctaaac | 19843 | - | see also 25436 line | | | |
| 25438 | SMUG1 | NM_014311.1 | 481 | ccccaagagcatcctaaacgacc | 19844 | - | see also 25436 line | | | |
| 25439 | SSBP1 | NM_003143.1 | 166 | ctgaatcgtgtgcacttacttgg | 19851 | - | see also 11029 line | | | |
| 25440 | SSBP1 | NM_003143.1 | 249 | ttctctagcaactaatgagatgt | 19852 | - | see also 11029 line | | | |
| 25441 | SSBP2 | NM_012446.2 | 767 | cccttaggaccacagaactatgg | 19873 | - | see also 5334 line | | | |
| 25442 | SSBP2 | NM_012446.2 | 620 | cccccattgaggatacctaatca | 19869 | - | see also 5334 line | | | |
| 25443 | SSBP2 | NM_012446.2 | 622 | cccattgaggatacctaatcagg | 19870 | - | see also 5334 line | | | |
| 25444 | SSBP4 | NM_032627.1 | 278 | aagttggcgctgtacgtttatga | 19881 | - | see also 9263 line | | | |
| 25445 | TAF15 | NM_003487.2 | 870 | aagaccggaaaaccaatgataaa | 19913 | - | see also 2352 line | | | |
| 25446 | TAF15 | NM_003487.2 | 117 | gggggtgagcagcaaagttattc | 19889 | - | see also 2352 line | | | |
| 25447 | WBP11 | NM_016312.2 | 1789 | gacccaaggcggatgatacaagt | 19928 | - | see also 11033 line | | | |
| 25448 | WBP11 | NM_016312.2 | 1786 | agcgacccaaggcggatgataca | 19927 | - | see also 11033 line | | | |
| 25449 | WBP11 | NM_016312.2 | 1791 | cccaaggcggatgatacaagtgc | 19929 | - | see also 11033 line | | | |
| 25450 | CGGBP1 | NM_003663.2 | 383 | acctgctcgaaaccgttctaaga | 19931 | - | see also 11034 line | | | |
| 25451 | CGGBP1 | NM_003663.2 | 426 | ctggatcgagtcactgagtttgg | 19932 | - | see also 11034 line | | | |
| 25452 | G22P1 | NM_001469.2 | 2058 | agcttcgcttcacatacagaagt | 19942 | - | see also 11035 line | | | |
| 25453 | HLF | NM_002126.3 | 585 | cacgaagacgcatttagtaaaga | 19945 | - | see also 1365 line | | | |
| 25454 | IFI16 | NM_005531.1 | 2074 | gagatcccaaaaggattgattag | 19971 | - | see also 11036 line | | | |
| 25455 | IFI16 | NM_005531.1 | 2104 | agcgtaactcctaaaatcaatca | 19973 | - | see also 11036 line | | | |
| 25456 | IFI16 | NM_005531.1 | 1264 | tacgaaattcaggatgatagagg | 19962 | - | see also 11036 line | | | |
| 25457 | MRE11A | NM_005590.2 | 1320 | tggatcgggtagctaatccaaaa | 20007 | - | see also 11037 line | | | |
| 25458 | MRE11A | NM_005590.2 | 1658 | gaggaggtacgtcgtttcagaga | 20013 | - | see also 11037 line | | | |

Figure 46

| | | | | | | | |
|---|---|---|---|---|---|---|---|
| 25459 | MTERF | NM_006980.2 | 475 | cacgtactcccgagaatctttca | 20052 | - | see also 11038 line |
| 25460 | MTERF | NM_006980.2 | 473 | aacacgtactcccgagaatcttt | 20051 | - | see also 11038 line |
| 25461 | MTERF | NM_006980.2 | 676 | cccctcgtaccttctccaatagt | 20058 | - | see also 11038 line |
| 25462 | NP220 | NM_014497.2 | 2774 | aacggtagctgttaaaggtaata | 20181 | - | see also 5721 line |
| 25463 | NP220 | NM_014497.2 | 1929 | tcccgttcaccatatcgaattag | 20149 | - | see also 5721 line |
| 25464 | SAFB | NM_002967.2 | 1696 | agcgatctcgagccacaaagtca | 20292 | - | see also 11040 line |
| 25465 | SAFB | NM_002967.2 | 1353 | ggcgccaaggttgtgacaaatgc | 20283 | - | see also 11040 line |
| 25466 | XRCC5 | NM_021141.2 | 1202 | tggccatagttcgatatgcttat | 20327 | - | see also 8105 line |
| 25467 | XRCC5 | NM_021141.2 | 970 | gggttccgctatggaagtgatat | 20320 | - | see also 8105 line |
| 25468 | MBD2 | NM_003927.3 | 745 | tggcaagagcgatgtctactact | 20348 | - | see also 2688 line |
| 25469 | MBD4 | NM_003925.1 | 1557 | atcgctactatatttctcaatcg | 20396 | - | see also 11043 line |
| 25470 | MBD4 | NM_003925.1 | 1463 | cccacgacgtaaagcctttaaga | 20389 | - | see also 11043 line |
| 25471 | MBD4 | NM_003925.1 | 1702 | acgatcttcgggcaaaaaccatt | 20398 | - | see also 11043 line |
| 25472 | MLH3 | NM_014381.1 | 730 | gacgtatgttcccgattttgtca | 20417 | - | see also 11044 line |
| 25473 | MLH3 | NM_014381.1 | 2268 | ctggtatagacacgtttccaatg | 20460 | - | see also 11044 line |
| 25474 | ORC2L | NM_006190.3 | 637 | atgatcctgagattacgataaac | 20518 | - | see also 4277 line |
| 25475 | ORC2L | NM_006190.3 | 641 | tcctgagattacgataaacgttc | 20519 | - | see also 4277 line |
| 25476 | ORC3L | NM_012381.2 | 899 | gactacggtactcgataagctac | 20606 | - | see also 11046 line |
| 25477 | ORC3L | NM_012381.2 | 159 | gacagtaagcttcgattcgaaac | 20574 | - | see also 11046 line |
| 25478 | ORC4L | NM_002552.2 | 1007 | ttgatgcttgctttaaatcgagt | 20664 | - | see also 1642 line |
| 25479 | ORC5L | NM_002553.2 | 234 | tggaaagacctatgtaacacaaa | 20678 | - | see also 1646 line |
| 25480 | TERF2 | NM_005652.2 | 1182 | aagaacaagcgcatgacaataag | 20732 | - | see also 3921 line |
| 25481 | TERF2 | NM_005652.2 | 568 | cagtggtcgaatccagtagaaaa | 20724 | - | see also 3921 line |
| 25482 | TERT | NM_003219.1 | 2960 | aacatgcgtcgcaaactctttgg | 20757 | - | see also 11050 line |
| 25483 | TERT | NM_003219.1 | 1981 | cagaacgttccgcagagaaaaga | 20748 | - | see also 11050 line |
| 25484 | KIF2C | NM_006845.2 | 90 | tcccggtctcgctatcaagatcc | 20762 | - | see also 11051 line |
| 25485 | KIF2C | NM_006845.2 | 89 | ttcccggtctcgctatcaagatc | 20761 | - | see also 11051 line |
| 25486 | KIF2C | NM_006845.2 | 1463 | cccacgcgtgcttccaaattatt | 20789 | - | see also 11051 line |
| 25487 | ZW10 | NM_004724.2 | 1208 | tacgctcgtaacatcaattctca | 20831 | - | see also 11052 line |
| 25488 | ZW10 | NM_004724.2 | 293 | cacgtatcaaccggtgaatttac | 20813 | - | see also 11052 line |
| 25489 | ZW10 | NM_004724.2 | 282 | gccgggatcttcacgtatcaacc | 20812 | - | see also 11052 line |
| 25490 | TP53BP1 | NM_005657.1 | 396 | gacggtaatagtgggttcaatga | 20875 | - | see also 3924 line |
| 25491 | TP53BP1 | NM_005657.1 | 2977 | gacacagtactcctattggtatt | 20931 | - | see also 3924 line |
| 25492 | ZBP1 | NM_030776.1 | 594 | atgtgaaccgagacttgtacagg | 20991 | - | see also 11055 line |
| 25493 | ZBP1 | NM_030776.1 | 1275 | gtcgtcccgcagacacacaatcc | 21002 | - | see also 11055 line |
| 25494 | CGBP | NM_014593.1 | 329 | gccgcaaaccggacatcaactgc | 21007 | - | see also 5766 line |
| 25495 | 3'HEXO | NM_153332.2 | 1030 | cgcccgaatagcagttcgaatgc | 21050 | - | see also 11057 line |

Figure 46

| 25496 | 3'HEXO | NM_153332.2 | 604 | tacgcatactttagaaatagaag | 21032 | - | see also 11057 line |
|---|---|---|---|---|---|---|---|
| 25497 | ABI-2 | NM_005759.3 | 489 | ttgaacgaccagttcgttatatt | 21067 | - | see also 4011 line |
| 25498 | ABI-2 | NM_005759.3 | 492 | aacgaccagttcgttatattaga | 21068 | - | see also 4011 line |
| 25499 | ABL1 | NM_005157.2 | 848 | tccatctcgctgagatacgaagg | 21104 | - | see also 11058 line |
| 25500 | ABL1 | NM_005157.2 | 626 | ctccgggtcttaggctataatca | 21097 | - | see also 11058 line |
| 25501 | ABL1 | NM_005157.2 | 949 | ggccgagttggttcatcatcatt | 21109 | - | see also 11058 line |
| 25502 | ACINUS | NM_014977.1 | 3253 | accttaactcgacgttccattag | 21157 | - | see also 11059 line |
| 25503 | ACINUS | NM_014977.1 | 3626 | tggaccgtccctctgaaactaag | 21171 | - | see also 11059 line |
| 25504 | ADPRT | NM_001618.2 | 2029 | aacgcttggcactccaaaaattt | 21203 | - | see also 11060 line |
| 25505 | ADPRT | NM_001618.2 | 274 | gtgcagtcgcccatgtttgatgg | 21179 | - | see also 11060 line |
| 25506 | ADPRTL2 | NM_005484.2 | 1701 | gtccgtatgcggtacctttaaa | 21279 | - | see also 3814 line |
| 25507 | ADPRTL2 | NM_005484.2 | 1699 | aggtccgtatgcggtaccttta | 21278 | - | see also 3814 line |
| 25508 | AKAP8 | NM_005858.2 | 447 | agccgttcgagtcctatgactcc | 21285 | - | see also 4073 line |
| 25509 | AKAP8 | NM_005858.2 | 1505 | ctggcctgcgacatgctaattcc | 21297 | - | see also 4073 line |
| 25510 | ALF | NM_006872.2 | 928 | caccagcacgtgactgatattca | 21335 | - | see also 11063 line |
| 25511 | ALF | NM_006872.2 | 1351 | gacctgtttgacacggataatgt | 21343 | - | see also 11063 line |
| 25512 | ALG1 | NM_019109.2 | 340 | ttgtacttcaggctatgtacttg | 21349 | - | see also 11064 line |
| 25513 | ALG1 | NM_019109.2 | 591 | ctgaacctgtgtgttaccaatgc | 21354 | - | see also 11064 line |
| 25514 | ALTE | NM_004729.1 | 1584 | gacgcccgagatcgacatgtttc | 21359 | - | see also 11065 line |
| 25515 | ALTE | NM_004729.1 | 1587 | gcccgagatcgacatgtttctca | 21361 | - | see also 11065 line |
| 25516 | ANKRD1 | NM_014391.2 | 783 | atcgaattccgtgatatgcttga | 21371 | - | see also 5654 line |
| 25517 | ANKRD1 | NM_014391.2 | 1028 | gagactgaaccgctataagatga | 21373 | - | see also 5654 line |
| 25518 | APEX1 | NM_001641.2 | 1166 | tggttggcgccttgattactttt | 21397 | - | see also 1084 line |
| 25519 | APEX1 | NM_001641.2 | 542 | tgggcttcgagcctggattaaga | 21377 | - | see also 1084 line |
| 25520 | APLP2 | NM_001642.1 | 602 | agggaatgaccttatatagctac | 21415 | - | see also 1087 line |
| 25521 | APLP2 | NM_001642.1 | 1075 | tgcgtgcgctttatatatggtgg | 21425 | - | see also 1087 line |
| 25522 | APOB48R | NM_018690.1 | 1976 | gaccgagagtctgaactatcaga | 21449 | - | see also 11068 line |
| 25523 | APOB48R | NM_018690.1 | 2347 | aggctgggactcgaaagaaaagg | 21450 | - | see also 11068 line |
| 25524 | ARAP3 | NM_022481.4 | 1995 | gacacgagtgtctggagtaatga | 21468 | - | see also 8403 line |
| 25525 | ARFGAP1 | NM_018209.2 | 1053 | gcggtctggaccacttccaaaac | 21509 | - | see also 7321 line |
| 25526 | ARFGAP1 | NM_018209.2 | 587 | tcctcggacaaggcttttgaaga | 21505 | - | see also 7321 line |
| 25527 | ARFGAP3 | NM_014570.3 | 159 | tccgctcggtgcccactaacaag | 21515 | - | see also 5756 line |
| 25528 | ARFGAP3 | NM_014570.3 | 931 | atcattacgattagcctataagg | 21539 | - | see also 5756 line |
| 25529 | ASH1L | NM_018489.1 | 8365 | cagtcctatcgactgttatctca | 21793 | - | see also 7495 line |
| 25530 | ASH1L | NM_018489.1 | 8506 | tcccgtcggttctatcataatga | 21801 | - | see also 7495 line |
| 25531 | ATF2 | NM_001880.2 | 1189 | atctcgaccgcagtcattacaac | 21841 | - | see also 1204 line |
| 25532 | ATF2 | NM_001880.2 | 1152 | gtcatggtagcggattggttagg | 21840 | - | see also 1204 line |

Figure 46

| 25533 | ATF5 | NM_012068.2 | 601 | cccacctgacctggaagctatgg | 21858 | - | see also 5104 line | | |
|---|---|---|---|---|---|---|---|---|---|
| 25534 | ATM | NM_000051.2 | 4583 | ttcgagacgttatttatactttg | 21999 | - | see also 18 line | | |
| 25535 | ATM | NM_000051.2 | 4580 | ttcttcgagacgttatttatact | 21998 | - | see also 18 line | | |
| 25536 | AWP1 | NM_019006.2 | 474 | gtcagtagtctgtctgaatcttt | 22126 | - | see also 11076 line | | |
| 25537 | BACH2 | NM_021813.1 | 2032 | agcacctcggtgcattcttattc | 22150 | - | see also 11077 line | | |
| 25538 | BACH2 | NM_021813.1 | 900 | ggcgctggttggacagacaaaaa | 22131 | - | see also 11077 line | | |
| 25539 | BANF1 | NM_003860.2 | 683 | tccgggaatggctgaaagacact | 22171 | - | - | DNA binding | - |
| 25540 | BAZ1A | NM_013448.2 | 858 | atcgttcgcgcttacatgaaatt | 22183 | - | see also 5447 line | | |
| 25541 | BAZ1A | NM_013448.2 | 4007 | ctgaatgcgcgttgcaagatatg | 22276 | - | see also 5447 line | | |
| 25542 | BAZ2A | NM_013449.2 | 4952 | ctgcttagcacacctaatggtgc | 22357 | - | see also 5449 line | | |
| 25543 | BAZ2A | NM_013449.2 | 5493 | atctcacattttgcgagattatc | 22367 | - | see also 5449 line | | |
| 25544 | BAZ2B | NM_013450.1 | 4493 | cacttactcatgccgatatgtca | 22463 | - | see also 11080 line | | |
| 25545 | BAZ2B | NM_013450.1 | 4406 | tccccaatgaccagttactaaaa | 22459 | - | see also 11080 line | | |
| 25546 | BBX | NM_020235.2 | 1773 | agcgtcatatataccattgaagc | 22536 | - | see also 7810 line | | |
| 25547 | BBX | NM_020235.2 | 1633 | ttgaggatcccgcagcattaaac | 22530 | - | see also 7810 line | | |
| 25548 | BDP1 | NM_018429.1 | 2462 | aagatcaatcgcgtaaagatttt | 22617 | - | see also 11082 line | | |
| 25549 | BDP1 | NM_018429.1 | 1052 | ttgagcgcggttctacaactaca | 22575 | - | see also 11082 line | | |
| 25550 | BHC80 | NM_016621.2 | 781 | gacactacctctcgtcttgaaag | 22713 | - | see also 6953 line | | |
| 25551 | BHC80 | NM_016621.2 | 837 | gccagagacctaccattgctatg | 22714 | - | see also 6953 line | | |
| 25552 | BLM | NM_000057.1 | 3568 | accaggcgatcgcttatgtgatg | 22843 | - | see also 30 line | | |
| 25553 | BLM | NM_000057.1 | 4273 | ctccaccgaagcctataaataga | 22863 | - | see also 30 line | | |
| 25554 | BOC | NM_033254.2 | 820 | gtccggtacagcgtcaaacaaga | 22867 | - | see also 9387 line | | |
| 25555 | BOC | NM_033254.2 | 1845 | ggcgccaatcctctactatgtgg | 22876 | - | see also 9387 line | | |
| 25556 | BRD1 | NM_014577.1 | 1823 | accgtgtgcgctccttatattcc | 22928 | - | see also 11086 line | | |
| 25557 | BRD1 | NM_014577.1 | 3074 | ctctgcgactcgagctttaatgc | 22938 | - | see also 11086 line | | |
| 25558 | BRD1 | NM_014577.1 | 3577 | ggccgtgcggatcgcttttgacc | 22943 | - | see also 11086 line | | |
| 25559 | BRIP1 | NM_032043.1 | 998 | ctgaggtagtcggtaacttcaac | 22976 | - | see also 9068 line | | |
| 25560 | BRIP1 | NM_032043.1 | 1001 | aggtagtcggtaacttcaacaga | 22977 | - | see also 9068 line | | |
| 25561 | BRPF1 | NM_004634.1 | 111 | gccgaaaggtctacaagagttac | 23076 | - | see also 3148 line | | |
| 25562 | BS69 | NM_006624.1 | 1426 | ctccgtgtcaactcagacaaaga | 23137 | - | see also 4684 line | | |
| 25563 | BS69 | NM_006624.1 | 844 | tgcgaggatgctatataaagaca | 23125 | - | see also 4684 line | | |
| 25564 | C11orf13 | NM_003475.2 | 146 | atcgcactagcccaagcaatagg | 23143 | - | see also 11090 line | | |
| 25565 | C11orf13 | NM_003475.2 | 251 | caggccacctgcggacagtttgc | 23144 | - | see also 11090 line | | |
| 25566 | C14orf106 | NM_018353.2 | 1744 | gactatatcaggcaacgtttata | 23188 | - | see also 11091 line | | |
| 25567 | C14orf106 | NM_018353.2 | 1100 | ctgcacaatttaacttacgaact | 23153 | - | see also 11091 line | | |
| 25568 | C14orf146 | NM_152447.2 | 1434 | ggccgctacttttggtcaattcc | 23270 | - | see also 9869 line | | |
| 25569 | C14orf146 | NM_152447.2 | 1485 | ctcattactcgtcatacacatga | 23271 | - | see also 9869 line | | |

Figure 46

| | | | | | | | |
|---|---|---|---|---|---|---|---|
| 25570 | C20orf100 | NM_032883.1 | 887 | cggtgacgtccaaatcgtgg | 23318 | - | see also 11093 line |
| 25571 | C20orf100 | NM_032883.1 | 818 | gtcggcctacgcactcttctca | 23316 | - | see also 11093 line |
| 25572 | C20orf104 | NM_016436.3 | 232 | gcgttggaaccatcgttatgatg | 23324 | - | see also 11094 line |
| 25573 | C20orf104 | NM_016436.3 | 985 | acgtcctggccttgacaaaaatt | 23345 | - | see also 11094 line |
| 25574 | C20orf179 | NM_178477.1 | 249 | gccatgagctcgcgatcaatttg | 23385 | - | - |
| 25575 | C20orf179 | NM_178477.1 | 248 | agccatgagctcgcgatcaattt | 23384 | - | see also 25574 line |
| 25576 | C20orf179 | NM_178477.1 | 202 | ctccgaaccattggagaaaaag | 23383 | - | see also 25574 line |
| 25577 | C21orf127 | NM_013240.2 | 290 | gacagcacgctgaaacaaagttc | 23389 | - | see also 11095 line |
| 25578 | C21orf127 | NM_013240.2 | 293 | agcacgctgtaacaaagttcaca | 23390 | - | see also 11095 line |
| 25579 | C21orf66 | NM_013329.2 | 824 | gagaaacgcggatagtttttc | 23410 | - | see also 5401 line |
| 25580 | C21orf66 | NM_013329.2 | 1168 | gactccgttactattgatttgg | 23419 | - | see also 5401 line |
| 25581 | CACNA1A | NM_000068.2 | 4326 | ccgggtgctacgacctcttaaaa | 23544 | - | see also 40 line |
| 25582 | CACNA1A | NM_000068.2 | 4112 | tgcgatacttgactacgttttt | 23541 | - | see also 40 line |
| 25583 | CARHSP1 | NM_014316.1 | 325 | acgaggtcacctataaaatgtgc | 23581 | - | - |
| 25584 | CARHSP1 | NM_014316.1 | 347 | ctccatcccaccaagaatgaga | 23582 | - | see also 25583 line |
| 25585 | CARP-1 | NM_018237.1 | 3468 | aacttatctacggtaatggatga | 23675 | - | see also 7330 line |
| 25586 | CBF2 | NM_005760.1 | 1625 | gacaatatcggatcgatattaca | 23721 | - | see also 4017 line |
| 25587 | CBF2 | NM_005760.1 | 1288 | ggctacttctccgctcaaatatc | 23710 | - | see also 4017 line |
| 25588 | CBF2 | NM_005760.1 | 288 | ttggcgaagtatacaaaagcttc | 23682 | - | see also 4017 line |
| 25589 | CDC5L | NM_001253.2 | 1945 | accgcctttaacagatttacaga | 23840 | - | see also 11101 line |
| 25590 | CDC5L | NM_001253.2 | 292 | gaggaataccgaggatgaaattc | 23783 | - | see also 11101 line |
| 25591 | CEBPG | NM_001806.2 | 489 | aacgccgagagaggaacaacatg | 23857 | - | see also 11102 line |
| 25592 | CEBPG | NM_001806.2 | 678 | cagacaacgtacagtccattagc | 23863 | - | see also 11102 line |
| 25593 | CENPC1 | NM_001812.1 | 2452 | ttcgtgattagtggagtactatc | 23930 | - | see also 11103 line |
| 25594 | CENPC1 | NM_001812.1 | 2587 | atggtaaatctaggtatacctct | 23938 | - | see also 11103 line |
| 25595 | CENTA1 | NM_006869.1 | 1164 | caegcccgaccgcaagtttctgt | 23970 | - | see also 4808 line |
| 25596 | CENTA1 | NM_006869.1 | 1279 | gaggcgcacttcaagcataaacc | 23976 | - | see also 4808 line |
| 25597 | CENTA2 | NM_018404.1 | 1202 | aaccggcttactgcatcaacaga | 23995 | - | see also 7449 line |
| 25598 | CENTA2 | NM_018404.1 | 791 | gcccgtcgtcagtacctaaaaat | 23988 | - | see also 7449 line |
| 25599 | CENTB1 | NM_014716.1 | 687 | cgggacgggcactggatttatgc | 24003 | - | see also 5909 line |
| 25600 | CENTB1 | NM_014716.1 | 1708 | aggcctggattcacgctaaatac | 24009 | - | see also 5909 line |
| 25601 | CENTB2 | NM_012287.3 | 1908 | tccacggtgtcagccaatagttt | 24068 | - | see also 11107 line |
| 25602 | CENTB2 | NM_012287.3 | 2343 | gtcacttacgtttagcaag | 24081 | - | see also 11107 line |
| 25603 | CENTB2 | NM_012287.3 | 1170 | gtggtctcgcaacaaaaagtttg | 24052 | - | see also 11107 line |
| 25604 | CENTB5 | NM_030649.1 | 1303 | agcgctgaatacagatctatga | 24089 | - | see also 11108 line |
| 25605 | CENTB5 | NM_030649.1 | 1953 | agcggacgtgaaccaaagagaca | 24093 | - | see also 11108 line |
| 25606 | CENTD1 | NM_015230.2 | 5193 | aaggacgataaacttcgaaatcg | 24239 | - | see also 11109 line |

Figure 46

| | | | | | | | |
|---|---|---|---|---|---|---|---|
| 25607 | CENTD1 | NM_015230.2 | 4347 | aaggaaccgactgtagtattat | 24214 | - | see also 11109 line |
| 25608 | CENTD2 | NM_015242.2 | 851 | accgcaggatcttacatctatc | 24256 | - | see also 6286 line |
| 25609 | CENTD2 | NM_015242.2 | 2430 | ctgggacgcctaccctacaaagc | 24274 | - | see also 6286 line |
| 25610 | CENTG1 | NM_014770.1 | 1208 | taccacccagtattaacgatta | 24297 | - | see also 11111 line |
| 25611 | CENTG1 | NM_014770.1 | 914 | ctcccgtcctcaccgaatgttgg | 24293 | - | see also 11111 line |
| 25612 | CENTG1 | NM_014770.1 | 1209 | accacccagtattaacgattac | 24298 | - | see also 11111 line |
| 25613 | CENTG2 | NM_014914.1 | 1046 | tacagtcgaattggccaactatcg | 24309 | - | see also 11112 line |
| 25614 | CENTG2 | NM_014914.1 | 1295 | cctgcaagtcgctacctaattc | 24312 | - | see also 11112 line |
| 25615 | CENTG3 | NM_031946.3 | 264 | ctccgtaccggagcttaaagtgg | 24321 | - | see also 11113 line |
| 25616 | CENTG3 | NM_031946.3 | 262 | cgctccgtaccggagcttaaagt | 24320 | - | see also 11113 line |
| 25617 | CHC1 | NM_001269.2 | 725 | cgggacaataacggtgtgattgg | 24339 | - | see also 831 line |
| 25618 | CHD1L | NM_024568.1 | 1817 | aggggccgatcactccgaaataaa | 24379 | - | see also 11115 line |
| 25619 | CHD1L | NM_024568.1 | 1819 | ggccgatcactccgaaataaagg | 24381 | - | see also 11115 line |
| 25620 | CHRAC1 | NM_017444.3 | 353 | ttcaatgcctagccacctattcc | 24384 | - | see also 11116 line |
| 25621 | CHRAC1 | NM_017444.3 | 400 | aagaaagtactgacttacagtga | 24387 | - | see also 11116 line |
| 25622 | CHTF18 | NM_022092.1 | 1916 | ctccgcctcacagccgattctacc | 24395 | - | see also 11117 line |
| 25623 | CHTF18 | NM_022092.1 | 2462 | gcccgatggccagtacatctaca | 24399 | - | see also 11117 line |
| 25624 | CHTF18 | NM_022092.1 | 2379 | tacagcaaccctgaaaagcaaca | 24397 | - | see also 11117 line |
| 25625 | CIC | NM_015125.2 | 2130 | ctgaggcgtccaaatgacaca | 24409 | - | see also 11118 line |
| 25626 | CIC | NM_015125.2 | 4385 | gtgcttggggagctagagtatga | 24415 | - | see also 11118 line |
| 25627 | CIP29 | NM_033082.1 | 50 | agctaaagcttgccgaactaaag | 24418 | - | see also 11119 line |
| 25628 | CIP29 | NM_033082.1 | 337 | agggctgaacgattcaatgtacc | 24423 | - | see also 11119 line |
| 25629 | CNTN1 | NM_001843.2 | 1616 | cacagatcctagcgaattatat | 24478 | - | see also 1191 line |
| 25630 | CNTN1 | NM_001843.2 | 2402 | tactggccgatatgtccataaag | 24507 | - | see also 1191 line |
| 25631 | COPEB | NM_001300.3 | 379 | cagagccgacctgctatgtttc | 24532 | - | see also 11121 line |
| 25632 | COPEB | NM_001300.3 | 650 | tggtcagctcgggaaaattgagc | 24542 | - | see also 11121 line |
| 25633 | CREB3 | NM_006368.4 | 585 | accgagccgactgggaagtagatg | 24546 | - | see also 11122 line |
| 25634 | CREB3 | NM_006368.4 | 633 | cccagcgtgtttgaacattctca | 24551 | - | see also 11122 line |
| 25635 | CREB3L3 | NM_032607.1 | 832 | acgaggagcgagtgctgaaaaaa | 24566 | - | see also 11123 line |
| 25636 | CREB3L3 | NM_032607.1 | 357 | tccccgaaggcagtgatagtgg | 24562 | - | see also 11123 line |
| 25637 | CREB3L4 | NM_130898.2 | 369 | ctccagaggttcggttaactagg | 24569 | - | see also 11124 line |
| 25638 | CREB3L4 | NM_130898.2 | 1109 | cagacgctaattgctcaaactc | 24574 | - | see also 11124 line |
| 25639 | CREB5 | NM_004904.1 | 359 | agcggaatatctcgatgcataat | 24586 | - | see also 3409 line |
| 25640 | CREB5 | NM_004904.1 | 360 | gcggaatatctcgatgcataatg | 24587 | - | see also 3409 line |
| 25641 | CREBL1 | NM_004381.3 | 63 | tgctgacccgacgcgtttcttca | 24600 | - | see also 2939 line |
| 25642 | CREBL1 | NM_004381.3 | 468 | aaccgtccagatcaatgttatcc | 24605 | - | see also 2939 line |
| 25643 | CRY1 | NM_004075.2 | 1014 | gtggacacaaccgcctctaacttat | 24632 | - | see also 2746 line |

Figure 46

| 25644 | CRY1 | NM_004075.2 | 2007 | ctgaggcaagccgtttgaatatc | 24663 | - | see also 2746 line |
|---|---|---|---|---|---|---|---|
| 25645 | CSEN | NM_013434.3 | 377 | ctctacaggggctttaagaatga | 24676 | - | see also 11128 line |
| 25646 | CSEN | NM_013434.3 | 373 | gtctctctacaggggctttaaga | 24675 | - | see also 11128 line |
| 25647 | D1S155E | NM_007158.2 | 1390 | aacagaccgacgtgacaaattag | 24705 | - | see also 4961 line |
| 25648 | D1S155E | NM_007158.2 | 1946 | gtgcgacttttaggtcgtaattc | 24726 | - | see also 4961 line |
| 25649 | DC8 | NM_015471.2 | 244 | atgcgcagtggacttttgaatca | 24738 | - | see also 11130 line |
| 25650 | DC8 | NM_015471.2 | 108 | gccactccccgagaagactttcg | 24736 | - | see also 11130 line |
| 25651 | DC8 | NM_015471.2 | 587 | gtccttgcctgcattaattgaac | 24745 | - | see also 11130 line |
| 25652 | DDEF2 | NM_003887.1 | 2835 | cccctcttcgcgtgacatctacc | 24793 | - | see also 11131 line |
| 25653 | DDEF2 | NM_003887.1 | 2386 | ttctcacgttcacgttgaatatg | 24787 | - | see also 11131 line |
| 25654 | DDEF2 | NM_003887.1 | 1996 | ggccgtcaaaacgagagatattt | 24777 | - | see also 11131 line |
| 25655 | DDX20 | NM_007204.3 | 2354 | tacgcctgtggatgatcgtattt | 24861 | - | see also 5006 line |
| 25656 | DDX3X | NM_001356.2 | 1930 | gcctcagattcgtagaatagtcg | 24890 | - | see also 901 line |
| 25657 | DDX3X | NM_001356.2 | 2027 | atgctggctcgtgatttcttaga | 24895 | - | see also 901 line |
| 25658 | DDX3Y | NM_004660.2 | 1139 | acctcagatacgtcgtatagttg | 24915 | - | see also 3179 line |
| 25659 | DDX3Y | NM_004660.2 | 1373 | tgcaacagggagtgattcactta | 24924 | - | see also 3179 line |
| 25660 | DDX48 | NM_014740.1 | 595 | tgctctcggtgactacatgaatg | 24939 | - | see also 11134 line |
| 25661 | DDX48 | NM_014740.1 | 702 | ctccagggcgtgtttttgatatg | 24941 | - | see also 11134 line |
| 25662 | DEAF1 | NM_021008.2 | 1546 | tgcgacgacatgaccttaagtgg | 24974 | - | see also 11135 line |
| 25663 | DEAF1 | NM_021008.2 | 1929 | aacgtcacatcccaaaatagtgt | 24979 | - | see also 11135 line |
| 25664 | DEDD | NM_004216.2 | 728 | tgcgggttcgggctgaatactgc | 24989 | - | see also 11136 line |
| 25665 | DEDD | NM_004216.2 | 1002 | agccatcaagctgctggtaaatg | 24993 | - | see also 11136 line |
| 25666 | DEDD2 | NM_133328.2 | 398 | gtctccagaacgctatagctatg | 24994 | - | see also 11137 line |
| 25667 | DEK | NM_003472.2 | 841 | ctgtcagatgaatctagtagtga | 25011 | - | see also 11138 line |
| 25668 | DEK | NM_003472.2 | 471 | aaccgatgaacttagaaatctac | 24997 | - | see also 11138 line |
| 25669 | DHX9 | NM_001357.2 | 2820 | cacgtagcccttttatcagtatt | 25082 | - | see also 902 line |
| 25670 | DHX9 | NM_001357.2 | 2815 | ctgatcacgtagcccttttatca | 25081 | - | see also 902 line |
| 25671 | DHX9 | NM_001357.2 | 3441 | agccagttggaccccgtaaatga | 25107 | - | see also 902 line |
| 25672 | DKFZp434I1930 | NM_032255.1 | 305 | gacgcaaaggggggactgaaagt | 25114 | - | see also 11140 line |
| 25673 | DKFZp434I1930 | NM_032255.1 | 1963 | gtgcgttgagtattattacatct | 25129 | - | see also 11140 line |
| 25674 | DMAP1 | NM_019100.3 | 763 | ttgacctgcgttttgttgttatc | 25247 | - | see also 11143 line |
| 25675 | DMAP1 | NM_019100.3 | 633 | tacccctttgccaggttcaataa | 25242 | - | see also 11143 line |
| 25676 | DNAJC1 | NM_022365.2 | 361 | atcgtaagctttcactaacttta | 25256 | - | see also 8346 line |
| 25677 | DNAJC1 | NM_022365.2 | 531 | tactacaggcgggtgagaaaaat | 25262 | - | see also 8346 line |

Figure 46

| 25678 | DNASE1L1 | NM_006730.1 | 1000 | tgcttcgagaactcaatcgattt | 25294 | - | see also 11145 line |
|---|---|---|---|---|---|---|---|
| 25679 | DNASE1L1 | NM_006730.1 | 1241 | cactcctaaggccgtagagaagg | 25300 | - | see also 11145 line |
| 25680 | DNASE1L1 | NM_006730.1 | 1269 | aacgccctctacgatgtgtttct | 25301 | - | see also 11145 line |
| 25681 | DNASE1L2 | NM_001374.1 | 157 | ttcgcatcggagccttcaacatt | 25303 | - | see also 11146 line |
| 25682 | DNASE1L2 | NM_001374.1 | 159 | cgcatcggagccttcaacattca | 25305 | - | see also 11146 line |
| 25683 | DNASE1L3 | NM_004944.1 | 215 | atcaaacgctgtgacatcatact | 25307 | - | see also 11147 line |
| 25684 | DNASE1L3 | NM_004944.1 | 600 | accgctggaaggcggagaatttc | 25313 | - | see also 11147 line |
| 25685 | DNASE2 | NM_001375.1 | 621 | cacctacccctgggtctataact | 25319 | - | see also 942 line |
| 25686 | DNMT1 | NM_001379.1 | 1713 | tcccgagtatgcgcccatatttg | 25352 | - | see also 949 line |
| 25687 | DNMT1 | NM_001379.1 | 1714 | cccgagtatgcgcccatatttgg | 25353 | - | see also 949 line |
| 25688 | DNMT2 | NM_004412.3 | 1183 | gtgcatgtagtagctaaactaat | 25433 | - | see also 2956 line |
| 25689 | DNMT2 | NM_004412.3 | 1180 | aacgtgcatgtagtagctaaact | 25432 | - | see also 2956 line |
| 25690 | DNMT3B | NM_006892.3 | 1201 | aagctcgtctcctatcgaaaagc | 25465 | - | see also 4823 line |
| 25691 | DNMT3B | NM_006892.3 | 1507 | cccaagcgcctcaagacaaattg | 25472 | - | see also 4823 line |
| 25692 | DNTT | NM_004088.2 | 355 | ctcctggctgatcgaatgcatag | 25501 | - | see also 11152 line |
| 25693 | DNTT | NM_004088.2 | 1327 | ggccatccgtgtggatttagttc | 25521 | - | see also 11152 line |
| 25694 | DOT1L | NM_032482.1 | 101 | ggccgctgccggtctacgataaa | 25532 | - | see also 9213 line |
| 25695 | DOT1L | NM_032482.1 | 102 | gccgctgccggtctacgataaac | 25533 | - | see also 9213 line |
| 25696 | DPF1 | NM_004647.1 | 585 | gccgtatgtctgtgataagtttt | 25567 | - | see also 11154 line |
| 25697 | DPF1 | NM_004647.1 | 776 | acccctcgtgtttacaattcacg | 25571 | - | see also 11154 line |
| 25698 | DPF2 | NM_006268.3 | 411 | cccggatccccgagttgatgatg | 25577 | - | see also 4362 line |
| 25699 | DPF2 | NM_006268.3 | 985 | atcgagtgcaaatgttgcaatat | 25584 | - | see also 4362 line |
| 25700 | DR1 | NM_001938.1 | 854 | aaggccagttctcgtttggaaaa | 25594 | - | see also 1236 line |
| 25701 | DRAP1 | NM_006442.2 | 606 | aacggtgggatgggaacgaaaag | 25602 | - | see also 11156 line |
| 25702 | DRF1 | NM_025104.2 | 570 | atcggccatggttgatccaaaag | 25605 | - | see also 11157 line |
| 25703 | DRF1 | NM_025104.2 | 571 | tcggccatggttgatccaaaagg | 25606 | - | see also 11157 line |
| 25704 | DRIL2 | NM_006465.1 | 760 | aagagagatctctcgagattttg | 25629 | - | see also 11158 line |
| 25705 | DRIL2 | NM_006465.1 | 800 | gacggtgatcctgaaaggaaaga | 25630 | - | see also 11158 line |
| 25706 | DRLM | NM_153757.1 | 337 | cagaagcgatgcgataagataga | 25635 | - | see also 11159 line |
| 25707 | DRLM | NM_153757.1 | 267 | gccgaaaaatgactttatcgaga | 25633 | - | see also 11159 line |
| 25708 | EBF | NM_024007.2 | 1603 | tggacaactggccgtgaatgtct | 25693 | - | see also 8555 line |

Figure 46

| | | | | | | | |
|---|---|---|---|---|---|---|---|
| 25709 | EBF | NM_024007.2 | 1325 | aacgaaccaccatcgattatgg | 25689 | - | see also 8555 line |
| 25710 | EBF | NM_024007.2 | 1329 | aacccaccatcgattatggtttc | 25690 | - | see also 8555 line |
| 25711 | EBF2 | NM_022659.1 | 697 | tccacgcctcaacagtctaatta | 25705 | - | see also 11161 line |
| 25712 | EBF2 | NM_022659.1 | 699 | cacgcctcaacagtctaattaca | 25706 | - | see also 11161 line |
| 25713 | EBF2 | NM_022659.1 | 1000 | gccatgaccggacttgttgtacc | 25714 | - | see also 11161 line |
| 25714 | ECE2 | NM_014693.1 | 913 | ccctctcggattactacttaaaa | 25720 | - | see also 11162 line |
| 25715 | ECE2 | NM_014693.1 | 915 | ctctcggattactacttaaaca | 25721 | - | see also 11162 line |
| 25716 | EDF1 | NM_003792.2 | 306 | aggacctggccacgaaaatcaat | 25746 | - | see also 11163 line |
| 25717 | EDF1 | NM_003792.2 | 165 | ctggccagaacaaacaacattct | 25744 | - | see also 11163 line |
| 25718 | EGR2 | NM_000399.2 | 345 | accgccaaggccgtagacaaaat | 25751 | - | see also 11164 line |
| 25719 | EGR2 | NM_000399.2 | 344 | gaccgccaaggccgtagacaaaa | 25750 | - | see also 11164 line |
| 25720 | EGR2 | NM_000399.2 | 435 | gccacgtcggtgaccatcttcc | 25753 | - | see also 11164 line |
| 25721 | EIF4A1 | NM_001416.1 | 494 | ggccgtgtttgatatgcttaa | 25764 | - | see also 11165 line |
| 25722 | EIF4A1 | NM_001416.1 | 520 | gagatacctgtccccaaataca | 25765 | - | see also 11165 line |
| 25723 | EIF4A2 | NM_001967.2 | 21 | tggttggctccgcggattataaca | 25769 | - | see also 1245 line |
| 25724 | EIF4A2 | NM_001967.2 | 23 | gttggctccgcggattataacaga | 25770 | - | see also 1245 line |
| 25725 | ELYS | NM_015446.1 | 5350 | aggtcaacgtatccaaaacgtga | 25917 | - | see also 6421 line |
| 25726 | ELYS | NM_015446.1 | 4563 | aggaagtagcgctgaacttaaaa | 25890 | - | see also 6421 line |
| 25727 | ENDOG | NM_004435.1 | 724 | gacccgcagctaccaaaagtct | 25938 | - | see also 11168 line |
| 25728 | ENDOG | NM_004435.1 | 730 | cagctaccaaaagtctatgtct | 25939 | - | see also 11168 line |
| 25729 | EP400 | NM_015409.2 | 5669 | gacgtcgggtgctgattttatca | 26010 | - | see also 11169 line |
| 25730 | EP400 | NM_015409.2 | 5848 | cacagccgtaccacaggttataaa | 26015 | - | see also 11169 line |
| 25731 | EP400 | NM_015409.2 | 7255 | ctccgtacgagccgagatctatgc | 26043 | - | see also 11169 line |
| 25732 | ERCC4 | NM_005236.1 | 2421 | tgcaacgcggagttgtttgaag | 26135 | - | see also 3671 line |
| 25733 | ERCC4 | NM_005236.1 | 248 | acctccctcgccgtgtaacaaat | 26067 | - | see also 3671 line |
| 25734 | ERN1 | NM_001433.1 | 2753 | tgcgtaaattcaggacctataaa | 26192 | - | see also 1002 line |
| 25735 | ERN1 | NM_001433.1 | 1401 | cccgtggactccatgcttaagg | 26180 | - | see also 1002 line |
| 25736 | EVI1 | NM_005241.1 | 777 | cacttcgtcgggcctcaaacaac | 26200 | - | see also 3673 line |
| 25737 | EVI1 | NM_005241.1 | 1055 | tggataaaacgtccatggttaat | 26209 | - | see also 3673 line |
| 25738 | EVI1 | NM_005241.1 | 780 | ttcgtcgggcctcaaacaacaca | 26201 | - | see also 3673 line |
| 25739 | EXO1 | NM_003686.3 | 1631 | tggaacgagtgattagttactaaa | 26320 | - | see also 2505 line |
| 25740 | EXO1 | NM_003686.3 | 793 | acccggtctatcaatatcacaca | 26280 | - | see also 2505 line |
| 25741 | EZH2 | NM_004456.3 | 2388 | atgtcggcatcgaaagagaaatg | 26409 | - | see also 2990 line |
| 25742 | FALZ | NM_004459.5 | 7549 | aacctcacaaccgattccaattc | 26577 | - | see also 11175 line |
| 25743 | FALZ | NM_004459.5 | 1680 | aggctcggggcagtaacaaatcc | 26446 | - | see also 11175 line |
| 25744 | FBXL11 | NM_012308.1 | 3265 | tgccacgcttcgcctcataattc | 26668 | - | see also 11176 line |
| 25745 | FBXL11 | NM_012308.1 | 1806 | gacgagtgcgatgtcgaaaatgc | 26651 | - | see also 11176 line |

Figure 46

| 25746 | FCRH3 | NM_052939.2 | 2257 | tggagatagcaacccgatttatt | 26706 | - | see also 11177 line |
|---|---|---|---|---|---|---|---|
| 25747 | FCRH3 | NM_052939.2 | 2259 | gagatagcaacccgatttattcc | 26707 | - | see also 11177 line |
| 25748 | FLJ10876 | NM_018254.2 | 557 | aggctcgtaaactagctaataga | 26726 | - | see also 7346 line |
| 25749 | FLJ10876 | NM_018254.2 | 1122 | caggcgtcggtttaacttagagg | 26737 | - | see also 7346 line |
| 25750 | FLJ12178 | NM_025134.2 | 592 | tacgtggactgatattaacgttg | 26764 | - | see also 11179 line |
| 25751 | FLJ12178 | NM_025134.2 | 1956 | tactacgtcgtacaaaaactatt | 26805 | - | see also 11179 line |
| 25752 | FLJ12178 | NM_025134.2 | 588 | ttcgtacgtggactgatattaac | 26762 | - | see also 11179 line |
| 25753 | FLJ12800 | NM_022903.2 | 900 | cccagtgtccggcgaatgaaagg | 26931 | - | see also 11180 line |
| 25754 | FLJ12800 | NM_022903.2 | 1384 | tccgtgccataaaggtagatagg | 26935 | - | see also 11180 line |
| 25755 | FLJ13111 | NM_025082.1 | 306 | ctgaagaacatcctactaactgc | 26942 | - | see also 11181 line |
| 25756 | FLJ13111 | NM_025082.1 | 915 | ctggtggcctggagccaaattta | 26948 | - | see also 11181 line |
| 25757 | FLJ13213 | NM_024755.1 | 748 | gcccacgtgatgtagatcatagg | 26967 | - | see also 8732 line |
| 25758 | FLJ14260 | NM_025027.2 | 1889 | ttctataagcctgatacacatgc | 26996 | - | see also 11182 line |
| 25759 | FLJ14260 | NM_025027.2 | 1924 | atgtgactcagccataatgtatg | 26997 | - | see also 11182 line |
| 25760 | FLJ14281 | NM_024920.3 | 2342 | atgtaaaaattactatgaagtac | 27008 | - | see also 11183 line |
| 25761 | FLJ14281 | NM_024920.3 | 2594 | tagaggttgtgaagctgatataa | 27010 | - | see also 11183 line |
| 25762 | FLJ20125 | NM_017676.1 | 942 | atgtttagtcgaaatccttatat | 27041 | - | see also 7107 line |
| 25763 | FLJ20125 | NM_017676.1 | 997 | atggtgataatacaagtatgttt | 27043 | - | see also 7107 line |
| 25764 | FLJ20203 | NM_032292.3 | 130 | gacctagaatcagccgttaaacc | 27049 | - | see also 11185 line |
| 25765 | FLJ20203 | NM_032292.3 | 1524 | tgcatttcgaacgcgttctaaga | 27077 | - | see also 11185 line |
| 25766 | FLJ21439 | NM_025137.2 | 5683 | aagtagagtatgccggtattttc | 27247 | - | see also 11186 line |
| 25767 | FLJ21439 | NM_025137.2 | 925 | aggacctaagggcgtagatgaag | 27118 | - | see also 11186 line |
| 25768 | FLJ23017 | NM_022840.2 | 1884 | aagccctacgaaggtcttatact | 27316 | - | see also 8492 line |
| 25769 | FLJ23017 | NM_022840.2 | 1886 | gccctacgaaggtcttatactgg | 27317 | - | see also 8492 line |
| 25770 | FLJ23119 | NM_024652.2 | 3889 | gccgagtcattgccgtcttaaaa | 27375 | - | see also 11188 line |
| 25771 | FLJ23119 | NM_024652.2 | 327 | gtcgggacgcacctggatttaat | 27328 | - | see also 11188 line |
| 25772 | FLJ23436 | NM_024671.2 | 848 | gtctacaccgcagtttgagatgc | 27387 | - | see also 11189 line |
| 25773 | FLJ23436 | NM_024671.2 | 322 | atgaagtcgaagagataccattt | 27380 | - | see also 11189 line |
| 25774 | FLJ36070 | NM_182574.1 | 875 | cagtgtcggggaccaagtctatg | 27390 | - | see also 10271 line |
| 25775 | FLJ38964 | NM_173527.1 | 163 | cagatgccacgctactaaagaag | 27393 | - | see also 10080 line |
| 25776 | FLJ38964 | NM_173527.1 | 166 | atgccacgctactaaagaagtca | 27394 | - | see also 10080 line |
| 25777 | FOS | NM_005252.2 | 224 | cggggatagcctctcttactacc | 27402 | - | see also 11192 line |
| 25778 | FOS | NM_005252.2 | 653 | agcggagacagaccaactagaag | 27407 | - | see also 11192 line |
| 25779 | FOSB | NM_006732.1 | 647 | accctctgccgagtctcaatatc | 27410 | - | see also 11193 line |
| 25780 | FOSB | NM_006732.1 | 646 | caccctctgccgagtctcaatat | 27409 | - | see also 11193 line |
| 25781 | FUS | NM_004960.1 | 1461 | ctctcacatgggggggtaactacg | 27434 | - | see also 11194 line |
| 25782 | FUS | NM_004960.1 | 1026 | aacgggacagcccatgattaatt | 27427 | - | see also 11194 line |

Figure 46

| 25783 | GASC1 | NM_015061.1 | 3263 | ctgtataccgcactttttttgaag | 27506 | - | see also 6215 line |
|---|---|---|---|---|---|---|---|
| 25784 | GASC1 | NM_015061.1 | 2727 | tacatgctttcgacataaggtca | 27483 | - | see also 6215 line |
| 25785 | GASC1 | NM_015061.1 | 2988 | gtggcccgatggcaaactctatg | 27490 | - | see also 6215 line |
| 25786 | GCM1 | NM_003643.1 | 1065 | tccggagtctactctgatcatgg | 27519 | - | see also 11196 line |
| 25787 | GCM1 | NM_003643.1 | 627 | cacgacggacgctttatattttt | 27511 | - | see also 11196 line |
| 25788 | GCM1 | NM_003643.1 | 628 | acgacggacgctttatatttttc | 27512 | - | see also 11196 line |
| 25789 | GCM2 | NM_004752.1 | 507 | tggcaacgcgatcttttttcagg | 27537 | - | see also 11197 line |
| 25790 | GCM2 | NM_004752.1 | 1526 | tgggctccgcagtcagttactca | 27564 | - | see also 11197 line |
| 25791 | GFI1 | NM_005263.1 | 1193 | aacggagctttgactgtaagatc | 27576 | - | see also 11198 line |
| 25792 | GFI1 | NM_005263.1 | 617 | tgcctttcaaaccgtactcatgg | 27574 | - | see also 11198 line |
| 25793 | GIOT-1 | NM_153257.1 | 1625 | ggcgtttaatcatagattacaac | 27588 | - | see also 11199 line |
| 25794 | GIOT-1 | NM_153257.1 | 1137 | aggcatcgctcacatcttactat | 27584 | - | see also 11199 line |
| 25795 | GIT1 | NM_014030.1 | 1632 | gaggacgacgccatctattcagt | 27603 | - | see also 11200 line |
| 25796 | GIT1 | NM_014030.1 | 1756 | gccgccacacgagcaagctttcc | 27604 | - | see also 11200 line |
| 25797 | GIT2 | NM_014776.2 | 315 | gaccctgcgtctattatgagtgg | 27610 | - | see also 11201 line |
| 25798 | GIT2 | NM_014776.2 | 332 | gagtggaagacgtaaagctaatc | 27612 | - | see also 11201 line |
| 25799 | GLI | NM_005269.1 | 3161 | ctcccgaaggacaggtatgtaac | 27686 | - | see also 11202 line |
| 25800 | GLI | NM_005269.1 | 1008 | gtcatactcacgcctcgaaaacc | 27660 | - | see also 11202 line |
| 25801 | GLI | NM_005269.1 | 3162 | tcccgaaggacaggtatgtaacc | 27687 | - | see also 11202 line |
| 25802 | GLI4 | NM_138465.2 | 54 | ggcagccctaggggacattcagg | 27690 | - | see also 11203 line |
| 25803 | GLI4 | NM_138465.2 | 537 | tgccgagctgggagtcaacttcg | 27691 | - | see also 11203 line |
| 25804 | GMEB1 | NM_006582.2 | 617 | tggactccggacagattgatttt | 27702 | - | see also 11204 line |
| 25805 | GMEB1 | NM_006582.2 | 616 | atggactccggacagattgattt | 27701 | - | see also 11204 line |
| 25806 | GMEB2 | NM_012384.2 | 611 | gggcgacctgacggaagataaca | 27716 | - | see also 11205 line |
| 25807 | GMEB2 | NM_012384.2 | 1053 | cccacgtcggccgagtacattcc | 27726 | - | see also 11205 line |
| 25808 | GTF2F1 | NM_002096.1 | 476 | ctccgggtcaacggcaaatcagg | 27737 | - | see also 11206 line |
| 25809 | GTF2F1 | NM_002096.1 | 221 | gtcgttcgagttcctaagaatac | 27733 | - | see also 11206 line |
| 25810 | GTF2F1 | NM_002096.1 | 225 | ttcgagttcctaagaatacaacc | 27735 | - | see also 11206 line |
| 25811 | GTF2F2 | NM_004128.1 | 639 | aagcgagctcgagctgataaaca | 27755 | - | see also 11207 line |
| 25812 | GTF2F2 | NM_004128.1 | 580 | aacctgttgctaatcatcaatac | 27750 | - | see also 11207 line |
| 25813 | GTF3A | NM_002097.1 | 749 | cacgagggctatgtatgtcaaaa | 27772 | - | see also 11208 line |
| 25814 | GTF3A | NM_002097.1 | 746 | gcccacgagggctatgtatgtca | 27771 | - | see also 11208 line |
| 25815 | GTF3C1 | NM_001520.1 | 1935 | caccaatcttcgcttaatcgaga | 27827 | - | see also 11209 line |
| 25816 | GTF3C1 | NM_001520.1 | 911 | ggctgtgcgaaaggacgtttaag | 27811 | - | see also 11209 line |
| 25817 | GTF3C1 | NM_001520.1 | 784 | ctcctactgaaccggtttcatgt | 27808 | - | see also 11209 line |
| 25818 | H1FOO | NM_153833.1 | 218 | cacgtcggtggcagctatcaagc | 27881 | - | see also 11211 line |
| 25819 | H1FOO | NM_153833.1 | 227 | ggcagctatcaagctctacatcc | 27882 | - | see also 11211 line |

Figure 46

| | | | | | | | | | |
|---|---|---|---|---|---|---|---|---|---|
| 25820 | H1FX | NM_006026.2 | 456 | ggcgtgtcccatctaagaaga | 27890 | - | | | see also 11212 line |
| 25821 | H2AFY | NM_004893.2 | 1211 | aacggtgtacttcgtgcttttg | 27905 | - | | | see also 11213 line |
| 25822 | H2AFY | NM_004893.2 | 1236 | agcgagagtataggcatctatgt | 27907 | - | | | see also 11213 line |
| 25823 | H2AFY2 | NM_018649.1 | 1049 | ctcagtgggctccgacaaatgt | 27923 | - | | | see also 11214 line |
| 25824 | H2AFY2 | NM_018649.1 | 1011 | tggactcgcagccaaatttgtca | 27922 | - | | | see also 11214 line |
| 25825 | HBP1 | NM_012257.2 | 535 | atcggcgaccagtcacacaaagtcc | 27933 | - | | | see also 5233 line |
| 25826 | HBP1 | NM_012257.2 | 1310 | gccacgcgtgtgcatctttgtct | 27959 | - | | | see also 5233 line |
| 25827 | HBXAP | NM_016578.3 | 1795 | accgagtcgtgtaccatgaaagg | 28006 | - | | | see also 11217 line |
| 25828 | HBXAP | NM_016578.3 | 910 | aaccgttctacagccaatgttct | 27978 | - | | | see also 11217 line |
| 25829 | HBXAP | NM_016578.3 | 3944 | gccgaagcacagacgagtattca | 28048 | - | | | see also 11217 line |
| 25830 | HELLS | NM_018063.3 | 1196 | gccgtctaatcaggagttaaaa | 28092 | - | | | see also 7231 line |
| 25831 | HELLS | NM_018063.3 | 701 | ttgaccagtcggaagtgtaat | 28074 | - | | | see also 7231 line |
| 25832 | HEMK | NM_016173.1 | 844 | acctcgacatgacctcagaaagg | 28139 | - | | | see also 11219 line |
| 25833 | HEMK | NM_016173.1 | 814 | ttcggttgcaggacaggatttgg | 28138 | - | | | see also 11219 line |
| 25834 | HES1 | NM_005524.2 | 302 | cacgcaccggataaaaccaaaga | 28146 | - | transcription_regulator | DNA binding | see also 25834 line |
| 25835 | HES1 | NM_005524.2 | 300 | aaacgacaccggataaaccaaa | 28145 | - | | | see also 25834 line |
| 25836 | HES1 | NM_005524.2 | 297 | gtcaacagcacacgcgataaacc | 28144 | - | | | see also 25834 line |
| 25837 | HES7 | NM_032580.1 | 8 | cccgggatgagctgagaattagg | 28151 | - | transcription_regulator | | see also 25837 line |
| 25838 | HES7 | NM_032580.1 | 260 | cgctcgccagtctgctacttgtcc | 28153 | - | | | see also 25837 line |
| 25839 | HES7 | NM_032580.1 | 155 | agctggagaaagcgcgagatattg | 28152 | - | | | see also 25837 line |
| 25840 | HIC2 | NM_015094.1 | 1536 | tcctacggggacaacttgtatgt | 28157 | - | transcription_regulator | | see also 25840 line |
| 25841 | HIC2 | NM_015094.1 | 1535 | gtcctacggggacaacttgtatg | 28156 | - | | | see also 25840 line |
| 25842 | HIC2 | NM_015094.1 | 1431 | aacggcaaggatgcaagtgaaga | 28155 | - | | | see also 25840 line |
| 25843 | HIPK1 | NM_152696.3 | 3101 | tggcaccgcactatcattgtgc | 28227 | - | | | see also 9919 line |
| 25844 | HIST1H1A | NM_005325.2 | 447 | aaggcaacgggtgcatctaaaaa | 28236 | - | | | see also 11222 line |
| 25845 | HIST1H1A | NM_005325.2 | 448 | aggcaacgggtgcatctaaaaag | 28237 | - | | | see also 11222 line |
| 25846 | HIST1H1A | NM_005325.2 | 500 | gagcgtcaagactccgaaaaagg | 28240 | - | | | see also 11222 line |
| 25847 | HIST1H1B | NM_005322.2 | 541 | ggcggctggcgtcaaaaaggtgg | 28246 | - | | | see also 11223 line |
| 25848 | HIST1H1B | NM_005322.2 | 586 | ggccgcgtcaaaccgaaaaagg | 28247 | - | | | see also 11223 line |
| 25849 | HIST1H1C | NM_005319.3 | 314 | ggcactctggttgcaaacgaaagg | 28251 | - | | | see also 11224 line |
| 25850 | HIST1H1C | NM_005319.3 | 412 | ggggcggaaccaaacctaagaagc | 28252 | - | | | see also 11224 line |
| 25851 | HIST1H1D | NM_005320.2 | 169 | accccagtatctgagcttatca | 28256 | - | | | see also 11225 line |
| 25852 | HIST1H1D | NM_005320.2 | 545 | accgctgctgggaccaagaaagt | 28259 | - | | | see also 11225 line |
| 25853 | HIST1H1E | NM_005321.2 | 358 | ggcgcgtcgggttccttcaaact | 28265 | - | | | see also 11226 line |

Figure 46

| | | | | | | | | | |
|---|---|---|---|---|---|---|---|---|---|
| 25854 | HIST1H1E | NM_005321.2 | 176 | cggtgtccgagctcattactaaa | 28262 | - | see also 11226 line | | |
| 25855 | HIST1H1T | NM_005323.3 | 376 | tagtaagaaggtgattcctaaat | 28272 | - | see also 11227 line | | |
| 25856 | HIST1H1T | NM_005323.3 | 600 | gggcttcgaagtcaaaattgacc | 28277 | - | see also 11227 line | | |
| 25857 | HIST1H2AA | NM_170745.2 | 206 | atgcgtctcgcgataacaaaaaa | 28284 | - | see also 11228 line | | |
| 25858 | HIST1H2AA | NM_170745.2 | 100 | ctgcttcgtaagggaaactatgc | 28280 | - | see also 11228 line | | |
| 25859 | HIST1H2AE | NM_021052.2 | 84 | aagctcgggcaaaagctaaaacg | 28290 | - | - | DNA binding | - |
| 25860 | HIST1H2AM | NM_003514.2 | 93 | ctctagagctgggctccaatttc | 28292 | - | - | E2F-1/DP-1complex | - |
| 25861 | HIST1H2BA | NM_170610.2 | 1 | atgccggaggtgtcatctaaagg | 28293 | - | see also 11230 line | | |
| 25862 | HIST1H2BA | NM_170610.2 | 96 | gaggacccgtaaggagagttatt | 28294 | - | see also 11230 line | | |
| 25863 | HIST1H2BB | NM_021062.2 | 42 | ttctaagaaggctatcactaagg | 28299 | - | - | DNA binding | - |
| 25864 | HIST1H2BJ | NM_021058.3 | 238 | ttcgtttgtgaacgacattttcg | 28300 | - | - | DNA binding | - |
| 25865 | HIST1H3A | NM_003529.2 | 174 | cactgaactgcttattcgtaaac | 28304 | - | - | DNA binding | - |
| 25866 | HIST1H3A | NM_003529.2 | 172 | tccactgaactgcttattcgtaa | 28303 | - | see also 25865 line | | |
| 25867 | HIST1H3C | NM_003531.2 | 52 | cgcaagcagcttgctactaaagc | 28305 | - | see also 11233 line | | |
| 25868 | HIST1H4A | NM_003538.3 | 167 | gcggggtgctcaaggtgtttttg | 28308 | - | see also 11234 line | | |
| 25869 | HIST1H4A | NM_003538.3 | 168 | cggggtgctcaaggtgtttttgg | 28309 | - | see also 11234 line | | |
| 25870 | HIST1H4D | NM_003539.3 | 164 | ctgcggagtgctgaaagttttc | 28310 | - | - | - | - |
| 25871 | HIST2H2AB | NM_175065.2 | 271 | gacgaagagctcaacaagttact | 28312 | - | - | - | - |
| 25872 | HIST2H2AB | NM_175065.2 | 368 | gtcacaagcctggcaagaacaag | 28313 | - | see also 25871 line | | |
| 25873 | HIST2H2AC | NM_003517.2 | 335 | tccaggccgttctgttaccaaag | 28315 | - | - | DNA binding | - |
| 25874 | HIST2H2AC | NM_003517.2 | 331 | aacatccaggccgttctgttacc | 28314 | - | see also 25873 line | | |
| 25875 | HIST3H2BB | NM_175055.2 | 51 | gcgcccaagaagggttctaaaaa | 28316 | - | - | - | - |
| 25876 | HIVEP1 | NM_002114.1 | 5860 | gtcgttatagaacctataagtga | 28455 | - | see also 1362 line | | |
| 25877 | HIVEP1 | NM_002114.1 | 3182 | ggcgagggacaatgtttgagtgt | 28387 | - | see also 1362 line | | |
| 25878 | HKR1 | NM_181786.2 | 1290 | aagtcgaacctctttacacatca | 28513 | - | see also 11237 line | | |
| 25879 | HKR1 | NM_181786.2 | 1609 | ggcgtcactttagctggaaatca | 28518 | - | see also 11237 line | | |

Figure 46

| 25880 | HMG2L1 | NM_005487.2 | 1090 | ttcagggcttgaacctattctgg | 28540 | - | see also 3815 line |
|---|---|---|---|---|---|---|---|
| 25881 | HMG2L1 | NM_005487.2 | 1098 | ttgaacctattctggatagaatca | 28541 | - | see also 3815 line |
| 25882 | HMG4L | NM_178467.1 | 457 | aagttggacggcacaaaaggtcc | 28560 | - | see also 11239 line |
| 25883 | HMGN3 | NM_004242.2 | 220 | tggatccaagtaactaaacagg | 28562 | - | see also 11240 line |
| 25884 | HNRPD | NM_002138.2 | 1081 | cagcaacagtgggatctagagg | 28583 | - | see also 1368 line |
| 25885 | HNRPK | NM_002140.2 | 458 | ccccgagcgcatattgagtatca | 28586 | - | see also 11242 line |
| 25886 | HNRPK | NM_002140.2 | 457 | gccccgagcgcatattgagtatc | 28585 | - | see also 11242 line |
| 25887 | HNRPU | NM_004501.2 | 1237 | ttcgtattggctggtcactaact | 28600 | - | see also 3053 line |
| 25888 | HNRPU | NM_004501.2 | 2399 | tggttggaatcggctatccatacc | 28633 | - | see also 3053 line |
| 25889 | HP1-BP74 | NM_016287.1 | 188 | ccgattccgaactgtgaattc | 28644 | - | see also 6786 line |
| 25890 | HRB | NM_004504.3 | 1272 | tgcgtatacttccacaagtaatg | 28694 | - | see also 3058 line |
| 25891 | HRB | NM_004504.3 | 203 | gccccacctacgttaacatgacg | 28669 | - | see also 3058 line |
| 25892 | HRBL | NM_006076.3 | 324 | ccctcatcgtgtcaagtcaatc | 28705 | - | see also 11246 line |
| 25893 | HRBL | NM_006076.3 | 327 | ctcatcgtgtcaagtcaatctcc | 28706 | - | see also 11246 line |
| 25894 | HSPC133 | NM_014168.1 | 429 | gtccaagtcattcgatacagtat | 28720 | - | see also 11247 line |
| 25895 | HSPC133 | NM_014168.1 | 178 | gccgcaattgcagcatgtatgc | 28712 | - | see also 11247 line |
| 25896 | HSPC133 | NM_014168.1 | 278 | agcaatcggaactgcaatgttagg | 28716 | - | see also 11247 line |
| 25897 | HT001 | NM_014065.2 | 1246 | ccctaccgaagcactagataatg | 28746 | - | see also 11249 line |
| 25898 | HT001 | NM_014065.2 | 1245 | accctaccgaagcactacagataat | 28745 | - | see also 11249 line |
| 25899 | HTR3A | NM_000869.1 | 687 | ctgtagcctcgacatctacaact | 28784 | - | see also 11250 line |
| 25900 | HTR3A | NM_000869.1 | 644 | aaggcgaagttcagaactacaag | 28781 | - | see also 11250 line |
| 25901 | IDAX | NM_025212.1 | 196 | ccgagccggtggggacttttc | 28794 | - | see also 11251 line |
| 25902 | IDAX | NM_025212.1 | 197 | ccgagccgcgtgggggacttttcc | 28795 | - | see also 11251 line |
| 25903 | ILF3 | NM_004516.2 | 267 | atgcgtccaatgcgaattttgt | 28812 | - | see also 11252 line |
| 25904 | ILF3 | NM_004516.2 | 269 | gcgtccaatgcgaattttgtga | 28813 | - | see also 11252 line |
| 25905 | ING1 | NM_005537.3 | 1579 | tgcgtgggctcaatcataaaac | 28850 | - | see also 11253 line |
| 25906 | ING1 | NM_005537.3 | 1576 | tcgtgcgtggggctcaatcataa | 28848 | - | see also 11253 line |
| 25907 | ING3 | NM_019071.2 | 236 | tgcaatggatcaactagaacaaa | 28856 | - | see also 7687 line |
| 25908 | ING4 | NM_016162.2 | 170 | ttggccactgagtatatgagtag | 28894 | - | see also 11255 line |
| 25909 | ING4 | NM_016162.2 | 384 | ttgagtcaagtgactatgacagc | 28898 | - | see also 11255 line |
| 25910 | INSM1 | NM_002196.1 | 473 | agcacgagaagcacaagtacttc | 28902 | - | see also 11256 line |
| 25911 | INSM1 | NM_002196.1 | 1595 | gccttacgcggcacatcaacaag | 28904 | - | see also 11256 line |
| 25912 | IRLB | NM_005848.1 | 2309 | agcggtgaagtacgacttataga | 28984 | - | see also 11257 line |
| 25913 | IRLB | NM_005848.1 | 4694 | tgctctcggtgtagaactgtga | 29062 | - | see also 11257 line |
| 25914 | JADE1 | NM_024900.2 | 396 | tccggatgagtactatgtgttgg | 29112 | - | see also 11258 line |
| 25915 | JADE1 | NM_024900.2 | 395 | atccggatgagtactatgtgttg | 29111 | - | see also 11258 line |
| 25916 | JDP2 | NM_130469.2 | 484 | agcccgatgccggaacaagaaga | 29141 | - | see also 9546 line |

Figure 46

| 25917 | JDP2 | NM_130469.2 | 486 | cccgatgccggaacaagaagaag | 29142 | - | see also 9546 line |
| 25918 | JMJ | NM_004973.2 | 2773 | agccgaaatcgagcaagagtact | 29174 | - | see also 3457 line |
| 25919 | JMJ | NM_004973.2 | 1799 | agcgcctcctgtgaaaatcgttc | 29164 | - | see also 3457 line |
| 25920 | JMJD2 | NM_014663.1 | 443 | ctgtacccacgctatagtgagt | 29202 | - | see also 5842 line |
| 25921 | JMJD2 | NM_014663.1 | 447 | accccacgctatagtgagtttga | 29203 | - | see also 5842 line |
| 25922 | JRK | NM_003724.1 | 1251 | ttccgattggttccatcatatct | 29262 | - | see also 11262 line |
| 25923 | JRK | NM_003724.1 | 984 | gtccgccgagcaggtttacaacg | 29260 | - | see also 11262 line |
| 25924 | JRKL | NM_003772.2 | 812 | tgccgcaagttcgagagtattta | 29292 | - | see also 11263 line |
| 25925 | JRKL | NM_003772.2 | 70 | gacggaggttcttccaaacaact | 29268 | - | see also 11263 line |
| 25926 | KCNMA1 | NM_002247.2 | 859 | tacttcggcttgcggtttattgc | 29331 | - | see also 1439 line |
| 25927 | KCNMA1 | NM_002247.2 | 3142 | atgagcgcgacgtacttcaatga | 29389 | - | see also 1439 line |
| 25928 | KIAA0211 | NM_014630.1 | 3941 | ttcagtgcgcgaaatgtagtttt | 29430 | - | see also 5798 line |
| 25929 | KIAA0211 | NM_014630.1 | 3943 | cagtgcgcgaaatgtagttttgc | 29431 | - | see also 5798 line |
| 25930 | KIAA0211 | NM_014630.1 | 1263 | tccggatgccactcgattcttcg | 29404 | - | see also 5798 line |
| 25931 | KIAA0222 | NM_014643.1 | 2504 | acctcatgccgctagatttaagt | 29442 | - | see also 5815 line |
| 25932 | KIAA0222 | NM_014643.1 | 1333 | aagtgcgggaacctgtttacaaa | 29436 | - | see also 5815 line |
| 25933 | KIAA0296 | NM_014699.1 | 3694 | ctggctgcctaccgtaatcatct | 29478 | - | see also 5895 line |
| 25934 | KIAA0296 | NM_014699.1 | 900 | ttcgggtacgtgggaagatctgc | 29461 | - | see also 5895 line |
| 25935 | KIAA0352 | NM_014830.1 | 1336 | ttcaccgacgggatggaatattt | 29501 | - | see also 6040 line |
| 25936 | KIAA0352 | NM_014830.1 | 1538 | ctgcgaccagcgtcaccttaacc | 29505 | - | see also 6040 line |
| 25937 | KIAA0478 | NM_014870.2 | 3522 | agccattccggtgcttgtactgt | 29545 | - | see also 11269 line |
| 25938 | KIAA0478 | NM_014870.2 | 3254 | aaggcctaccagcaattgtctgg | 29538 | - | see also 11269 line |
| 25939 | KIAA0628 | NM_014789.1 | 743 | gactctacgtctgtaatgtgtgt | 29550 | - | see also 11270 line |
| 25940 | KIAA0628 | NM_014789.1 | 1680 | tagtcagtatgggagagatttta | 29558 | - | see also 11270 line |
| 25941 | KIAA0945 | NM_014952.1 | 1654 | ctgtggcggctgcccatacaaaa | 29561 | - | see also 11271 line |
| 25942 | KIAA0945 | NM_014952.1 | 2144 | gacaccgtccttctcaaatcagg | 29569 | - | see also 11271 line |
| 25943 | KIAA1111 | NM_015107.1 | 1480 | tgcgaaccgtacagctcattaaa | 29604 | - | see also 6248 line |
| 25944 | KIAA1111 | NM_015107.1 | 2094 | aaggcgaccctgataataagacc | 29619 | - | see also 6248 line |
| 25945 | KIAA1196 | NM_020713.1 | 1005 | cacaccgccctgcaaaatggtgc | 29637 | - | see also 11273 line |
| 25946 | KIAA1196 | NM_020713.1 | 974 | cagtcagcaggcccattgctatc | 29636 | - | see also 11273 line |
| 25947 | KIAA1259 | NM_017553.1 | 4645 | tacgggtacaacgtgtctaaagg | 29739 | - | see also 11274 line |
| 25948 | KIAA1259 | NM_017553.1 | 2658 | gtgtaatcacccggagttatttg | 29713 | - | see also 11274 line |
| 25949 | KIAA1333 | NM_017769.2 | 391 | accccgatgtaaacgaagttatc | 29746 | - | see also 11275 line |
| 25950 | KIAA1333 | NM_017769.2 | 392 | ccccgatgtaaacgaagttatca | 29747 | - | see also 11275 line |
| 25951 | KIAA1706 | NM_030636.1 | 1796 | gtcacgacctgaccttgttaac | 29808 | - | see also 11276 line |
| 25952 | KIAA1706 | NM_030636.1 | 2216 | tagccgagttctacactgaaaag | 29814 | - | see also 11276 line |
| 25953 | KIAA1706 | NM_030636.1 | 1007 | ccctcaccccacgtgttaacatc | 29804 | - | see also 11276 line |

Figure 46

| 25954 | KIAA1750 | NM_033512.1 | 1000 | atcgcaacccgtatttccaaaat | 29825 | - | see also 11277 line | | |
|--------|----------|-------------|------|------------------------|-------|---|--------------------|---|---|
| 25955 | KIAA1750 | NM_033512.1 | 486 | accgtgggaaggccgaaaaatgc | 29819 | - | see also 11277 line | | |
| 25956 | KIAA1946 | NM_177454.2 | 755 | aacgttactggctatcttacagt | 29845 | - | see also 10164 line | | |
| 25957 | KIAA1946 | NM_177454.2 | 346 | gacggttccagtgtctgtattta | 29833 | - | see also 10164 line | | |
| 25958 | KIF22 | NM_007317.1 | 1124 | aggtgatcaatcggcctttacc | 29911 | - | see also 11279 line | | |
| 25959 | KIF22 | NM_007317.1 | 103 | cgctgtcggctaagcaagattgg | 29903 | - | see also 11279 line | | |
| 25960 | KIF4A | NM_012310.2 | 270 | gccactcataaaaggtgtattta | 29919 | - | - | microtubule motor | - |
| 25961 | KLF14 | NM_138693.1 | 686 | ggctcgactgcgacaagaagttt | 29920 | - | transcription_regulator | - | - |
| 25962 | KLF14 | NM_138693.1 | 831 | cccaacctatcatccagatatga | 29921 | - | see also 25961 line | | |
| 25963 | KLF15 | NM_014079.2 | 300 | ccctcacccgtctctgaagatga | 29922 | - | see also 11280 line | | |
| 25964 | KLF15 | NM_014079.2 | 1343 | tcctgtgtgcgagaagaagttcg | 29926 | - | see also 11280 line | | |
| 25965 | KLF15 | NM_014079.2 | 1133 | agccgcagaactcatcaaaatgc | 29925 | - | see also 11280 line | | |
| 25966 | KLF5 | NM_001730.2 | 1310 | ttctaaactggcaattcacaatc | 29951 | - | see also 11282 line | | |
| 25967 | KLF5 | NM_001730.2 | 694 | aagggttaccttacagtatcaac | 29935 | - | see also 11282 line | | |
| 25968 | KLHL10 | NM_152467.1 | 1291 | ggctacgtgcgtctaaacactgc | 29991 | - | see also 9876 line | | |
| 25969 | KLHL10 | NM_152467.1 | 187 | atcttcaacgagcttagactaga | 29959 | - | see also 9876 line | | |
| 25970 | KNSL7 | NM_020242.1 | 358 | gtcttgcatgagcggttataatg | 30018 | - | see also 7817 line | | |
| 25971 | KNSL7 | NM_020242.1 | 95 | gagttacgcagcgtgacaaatgg | 30011 | - | see also 7817 line | | |
| 25972 | LAF4 | NM_002285.1 | 2973 | gcctcgcagtgccgattatttta | 30155 | - | see also 11285 line | | |
| 25973 | LAF4 | NM_002285.1 | 1427 | ctgaaccggcatcctctaacaag | 30133 | - | see also 11285 line | | |
| 25974 | LBR | NM_002296.2 | 1088 | gggcgtagagtttcattacgtgt | 30196 | - | see also 1473 line | | |
| 25975 | LBR | NM_002296.2 | 399 | tcccctggtcgaccacctaaaag | 30179 | - | see also 1473 line | | |
| 25976 | LCX | NM_030625.1 | 4476 | agctgtcttgatcgagttataca | 30373 | - | see also 11287 line | | |
| 25977 | LCX | NM_030625.1 | 6309 | gagcaccccaaccgtaatcatcc | 30423 | - | see also 11287 line | | |
| 25978 | LDB1 | NM_003893.3 | 920 | tgctacggagctgtactatgttc | 30434 | - | see also 2647 line | | |
| 25979 | LDB1 | NM_003893.3 | 650 | tcccatgtatccgcctacatacc | 30430 | - | see also 2647 line | | |
| 25980 | LEF1 | NM_016269.2 | 981 | gagttattccgggtacataatga | 30489 | - | see also 11290 line | | |
| 25981 | LEF1 | NM_016269.2 | 784 | cccgaagaggaaggcgatttagc | 30484 | - | see also 11290 line | | |
| 25982 | LENEP | NM_018655.1 | 96 | tgcgggtgcctgtcattaagatg | 30516 | - | - | DNA binding | - |
| 25983 | LHFP | NM_005780.1 | 492 | accttccggaggtgctcatatcc | 30518 | - | see also 11291 line | | |
| 25984 | LHFP | NM_005780.1 | 422 | ctcctgcgtggggttctttatgc | 30517 | - | see also 11291 line | | |
| 25985 | LIG1 | NM_000234.1 | 2581 | cgcccttacgtgcggatagatgg | 30536 | - | see also 172 line | | |
| 25986 | LIG1 | NM_000234.1 | 1650 | tcccgacctggaccgaattatcc | 30527 | - | see also 172 line | | |
| 25987 | LIG1 | NM_000234.1 | 124 | cagcgaagtatcatgtcattttt | 30521 | - | see also 172 line | | |
| 25988 | LIG3 | NM_002311.2 | 850 | tacgggagtttcgaaagttatgc | 30547 | - | see also 1480 line | | |
| 25989 | LIG3 | NM_002311.2 | 1933 | ttccaaaccggatcatgttctca | 30575 | - | see also 1480 line | | |

Figure 46

| 25990 | LIG4 | NM_002312.2 | 2711 | ttcgacgccacaccgtttatttg | 30686 | - | see also 1484 line | | |
|---|---|---|---|---|---|---|---|---|---|
| 25991 | LIG4 | NM_002312.2 | 2210 | cacctaaccttactaacgttaac | 30665 | - | see also 1484 line | | |
| 25992 | LIN28 | NM_024674.3 | 722 | tgctcccggaggcacagaattga | 30706 | - | see also 11294 line | | |
| 25993 | LIN28 | NM_024674.3 | 502 | cagaagcgcgatcaaaaggaga | 30703 | - | see also 11294 line | | |
| 25994 | LOC112885 | NM_138415.1 | 1008 | gcctggttaccacggaacatttg | 30708 | - | see also 11295 line | | |
| 25995 | LOC112885 | NM_138415.1 | 1512 | tggcgtcagcagtgcagaaatgc | 30710 | - | see also 11295 line | | |
| 25996 | LOC115950 | NM_138783.1 | 1604 | ggcggcacatgatcatccattca | 30719 | - | see also 11296 line | | |
| 25997 | LOC115950 | NM_138783.1 | 1002 | cggctcacaggtgatcatcattg | 30714 | - | see also 11296 line | | |
| 25998 | LOC116071 | NM_138456.2 | 803 | agcgcctgtgccccatggattgt | 30720 | - | - | | - |
| 25999 | LOC117584 | NM_057178.2 | 1067 | cagtggtgccgaagaccaaaacg | 30729 | - | see also 9500 line | | |
| 26000 | LOC117584 | NM_057178.2 | 362 | gacctgcttggactgtaagaaaa | 30722 | - | see also 9500 line | | |
| 26001 | LOC127540 | NM_145205.2 | 150 | gtctcttcttacgttcacttttt | 30731 | - | transcription_regulator | - | - |
| 26002 | LOC151871 | NM_138815.2 | 563 | aacaactcggtttgagtactaat | 30742 | - | see also 11298 line | | |
| 26003 | LOC151871 | NM_138815.2 | 560 | gtcaacaactcggtttgagtact | 30741 | - | see also 11298 line | | |
| 26004 | LOC153222 | NM_153607.1 | 412 | ttcggatccagatttcatgtatg | 30755 | - | see also 9997 line | | |
| 26005 | LOC153222 | NM_153607.1 | 1906 | ttccagagcttgtcggttaaaga | 30793 | - | see also 9997 line | | |
| 26006 | LOC221143 | NM_174928.1 | 173 | ttgagccaggcgaggatgataaa | 30806 | - | see also 11300 line | | |
| 26007 | LOC221143 | NM_174928.1 | 372 | ttcgatatacatctttgaatatg | 30815 | - | see also 11300 line | | |
| 26008 | LOC283248 | NM_173587.2 | 521 | atgatccgcgttggaaccaatta | 30826 | - | see also 11301 line | | |
| 26009 | LOC283248 | NM_173587.2 | 1419 | tccgtaggtatggcaaagacttt | 30837 | - | see also 11301 line | | |
| 26010 | LOC56959 | NM_020207.1 | 270 | ggcagcacatgacgtatttgagt | 30846 | - | see also 11302 line | | |
| 26011 | LOC56959 | NM_020207.1 | 272 | cagcacatgacgtatttgagttg | 30847 | - | see also 11302 line | | |
| 26012 | LOC57117 | NM_020395.2 | 1320 | ggccttagtcgctcagttagttg | 30919 | - | see also 11303 line | | |

Figure 46

| | | | | | | | | | |
|---|---|---|---|---|---|---|---|---|---|
| 26013 | LOC57117 | NM_020395.2 | 1047 | agcgtttcctcgtcagtaactag | 30913 | - | see also 11303 line | | |
| 26014 | LOC57117 | NM_020395.2 | 1321 | gccttagtcgctcagttagttgt | 30920 | - | see also 11303 line | | |
| 26015 | LOC58486 | NM_021211.1 | 1206 | tactgttgcacgtcgaattaagg | 30952 | - | see also 11304 line | | |
| 26016 | LOC58486 | NM_021211.1 | 1808 | ttcgtcgtgaacttttggttttc | 30981 | - | see also 11304 line | | |
| 26017 | LOC58486 | NM_021211.1 | 2471 | tgcgaatccgacttagcaatatt | 31005 | - | see also 11304 line | | |
| 26018 | LOC64744 | NM_022733.1 | 1064 | ggccatgcctgcaggctatatgg | 31031 | - | see also 8436 line | | |
| 26019 | LOC90668 | NM_138360.1 | 2276 | gacaagctggaacctgatagaag | 31039 | - | see also 11306 line | | |
| 26020 | LOC90668 | NM_138360.1 | 1642 | cccccccaagcctcggcaataact | 31038 | - | see also 11306 line | | |
| 26021 | LOC93349 | NM_138402.2 | 156 | cgggaacgagaacgcagaaaaac | 31040 | - | see also 11307 line | | |
| 26022 | LOC93349 | NM_138402.2 | 454 | aaccttggcaaagtgtatacaga | 31042 | - | see also 11307 line | | |
| 26023 | LRP1B | NM_018557.1 | 6172 | gagccagttggtctatcgataga | 31222 | - | see also 7512 line | | |
| 26024 | LRP1B | NM_018557.1 | 7679 | aaggtaccaaccgaatcttttac | 31274 | - | see also 7512 line | | |
| 26025 | LYL1 | NM_005583.3 | 708 | ggccagcaggacctttagcatc | 31492 | - | transcription_regulator | DNA binding | leukemia |
| 26026 | M96 | NM_007358.1 | 280 | cacaagcggtctcctttacgtcg | 31495 | - | see also 5083 line | | |
| 26027 | M96 | NM_007358.1 | 277 | gtccacaagcggtctcctttacg | 31494 | - | see also 5083 line | | |
| 26028 | MAF | NM_005360.2 | 1994 | tacttaccagtgtgttcacaaaa | 31548 | - | transcription_regulator | RNA polymerase II transcription factor | - |
| 26029 | MAFB | NM_005461.3 | 623 | gacacacctcgaggatctgtact | 31549 | - | - | - | - |
| 26030 | MAZ | NM_002383.1 | 828 | tcccctcgggtgctatgaagatg | 31551 | - | see also 11309 line | | |
| 26031 | MAZ | NM_002383.1 | 829 | cccctcgggtgctatgaagatgc | 31552 | - | see also 11309 line | | |
| 26032 | MBD3 | NM_003926.5 | 348 | agcagccggtgaccaagattacc | 31559 | - | see also 2687 line | | |
| 26033 | MBD3 | NM_003926.5 | 352 | gccggtgaccaagattaccaacc | 31560 | - | see also 2687 line | | |
| 26034 | MBD5 | NM_018328.3 | 3189 | cagtcgctgttaaacaacaatca | 31645 | - | see also 11311 line | | |
| 26035 | MBD5 | NM_018328.3 | 2397 | cagttactacagtctatgagttg | 31620 | - | see also 11311 line | | |
| 26036 | MCM2 | NM_004526.2 | 60 | ggcggaatcatcggaatccttca | 31690 | - | see also 11312 line | | |
| 26037 | MCM2 | NM_004526.2 | 1638 | agcgaagtcgcagtttctcaagt | 31702 | - | see also 11312 line | | |
| 26038 | MCM3 | NM_002388.3 | 570 | accatagagcgacgttattctga | 31724 | - | see also 11313 line | | |
| 26039 | MCM3 | NM_002388.3 | 2219 | ttcatacgacccctatgacttca | 31758 | - | see also 11313 line | | |
| 26040 | MCM3AP | NM_003906.3 | 5261 | atgatcttatcgccttgtgtatc | 31841 | - | see also 11314 line | | |
| 26041 | MCM3AP | NM_003906.3 | 5868 | gtgtctaggcgaacgactaaagc | 31850 | - | see also 11314 line | | |
| 26042 | MCM4 | NM_005914.2 | 1838 | ctcactgcgtacgtaatgaaaga | 31875 | - | see also 11315 line | | |
| 26043 | MCM4 | NM_005914.2 | 2579 | atgagtgccacctctcgtaaacg | 31896 | - | see also 11315 line | | |
| 26044 | MCM5 | NM_006739.2 | 1615 | gtcgcgcttcgacatgatcttca | 31920 | - | see also 4762 line | | |
| 26045 | MCM5 | NM_006739.2 | 1396 | tggtggggtcgtctgtattgacg | 31917 | - | see also 4762 line | | |
| 26046 | MCM6 | NM_005915.4 | 1469 | atggacgtgcgggatcaagttgc | 31952 | - | see also 4136 line | | |
| 26047 | MCM6 | NM_005915.4 | 524 | gagcttgtgagcggaactttct | 31931 | - | see also 4136 line | | |

Figure 46

| 26048 | MCM7 | NM_005916.3 | 1318 | cacgtcagcgtcactggtatttt | 32004 | - | see also 11317 line |
|---|---|---|---|---|---|---|---|
| 26049 | MCM7 | NM_005916.3 | 687 | ccccgagttggtggactcaattt | 31991 | - | see also 11317 line |
| 26050 | MCM8 | NM_032485.4 | 635 | cagcgatagctctcctttgattg | 32031 | - | see also 11318 line |
| 26051 | MCM8 | NM_032485.4 | 582 | ttggatcgattcataccatataa | 32028 | - | see also 11318 line |
| 26052 | MCMDC1 | NM_153255.2 | 621 | gggacagtgattcgaacaagtct | 32114 | - | see also 11319 line |
| 26053 | MCMDC1 | NM_153255.2 | 951 | ctcactatttacgggattgtaat | 32131 | - | see also 11319 line |
| 26054 | MECP2 | NM_004992.2 | 608 | cacatccctggaccctaatgatt | 32152 | - | see also 3482 line |
| 26055 | MGC23143 | NM_153219.1 | 654 | atctgccggctgcgctttacaga | 32162 | - | see also 11321 line |
| 26056 | MGC23143 | NM_153219.1 | 102 | gagaaacctctggccttatctcc | 32161 | - | see also 11321 line |
| 26057 | MGC23920 | NM_173825.1 | 286 | ttcgtacacgacttaacaaataa | 32168 | - | see also 11322 line |
| 26058 | MGC23920 | NM_173825.1 | 475 | aagcgccatgaagttttaactac | 32176 | - | see also 11322 line |
| 26059 | MGC2941 | NM_024297.1 | 822 | gagcaagaagcggaagttaaaaa | 32185 | - | see also 11323 line |
| 26060 | MGC2941 | NM_024297.1 | 823 | agcaagaagcggaagttaaaaaa | 32186 | - | see also 11323 line |
| 26061 | MGC29463 | NM_152686.1 | 41 | gcgctggacggaagcttacattg | 32187 | - | see also 9917 line |
| 26062 | MGC33407 | NM_178525.2 | 1114 | cgcggacttggcccaaaacgtgc | 32212 | - | see also 11325 line |
| 26063 | MGC33407 | NM_178525.2 | 864 | tggacctagtggagaacattaag | 32210 | - | see also 11325 line |
| 26064 | MGMT | NM_002412.1 | 118 | gggtctgcacgaaataaagctcc | 32213 | - | see also 11326 line |
| 26065 | MHC2TA | NM_000246.1 | 1810 | agcaaggccgacgccctatttga | 32226 | - | see also 11327 line |
| 26066 | MHC2TA | NM_000246.1 | 3279 | caggctaagcttgtacaataact | 32235 | - | see also 11327 line |
| 26067 | MI-ER1 | NM_020948.1 | 748 | tccgcccacgtcgatgtaaatat | 32245 | - | see also 11328 line |
| 26068 | MI-ER1 | NM_020948.1 | 1364 | tcctggtgtaacggattacatgg | 32261 | - | see also 11328 line |
| 26069 | MITF | NM_000248.2 | 474 | atgtaatcgatgacatcattagc | 32283 | - | see also 180 line |
| 26070 | MITF | NM_000248.2 | 1198 | ggccaaccaagcctatagtgtcc | 32296 | - | see also 180 line |
| 26071 | MIZ1 | NM_004671.1 | 881 | atcttgtacggcagcttacatca | 32352 | - | see also 3193 line |
| 26072 | MIZ1 | NM_004671.1 | 178 | ttgtatagacgccgatatccacg | 32323 | - | see also 3193 line |
| 26073 | MKL1 | NM_020831.2 | 624 | atctcaaacggaagattcgttcc | 32375 | - | see also 11330 line |
| 26074 | MKL1 | NM_020831.2 | 1935 | tgctcagcacgggcgatgaaaac | 32383 | - | see also 11330 line |
| 26075 | MLL3 | NM_021230.1 | 11941 | ctgaacgcaccttatagtaaaca | 32659 | - | see also 11331 line |
| 26076 | MLL3 | NM_021230.1 | 11782 | ttccgatacggccgaaatcctct | 32653 | - | see also 11331 line |
| 26077 | MLL5 | NM_018682.2 | 4451 | ctcagacacacgttcgtaattca | 32821 | - | see also 11332 line |
| 26078 | MLL5 | NM_018682.2 | 3599 | tcggacggactgttaatgacaat | 32805 | - | see also 11332 line |

Figure 46

| | | | | | | | | | | |
|---|---|---|---|---|---|---|---|---|---|---|
| 26079 | MLLT1 | NM_005934.2 | 392 | ggggtacgctggcttcatcatgc | 32864 | - | see also 11333 line | | | |
| 26080 | MLLT1 | NM_005934.2 | 414 | cccatcgaggtgcacttcaaaaa | 32865 | - | see also 11333 line | | | |
| 26081 | MLLT6 | NM_005937.1 | 1888 | acccccatgggtgccgttaatcc | 32880 | - | see also 4144 line | | | |
| 26082 | MLLT6 | NM_005937.1 | 1640 | tactaggggcaggcatctacacc | 32877 | - | see also 4144 line | | | |
| 26083 | MNDA | NM_002432.1 | 857 | aacagcgccatttaaatacgagt | 32897 | - | see also 11335 line | | | |
| 26084 | MNDA | NM_002432.1 | 377 | tgcctgtctagacaaactaatag | 32891 | - | see also 11335 line | | | |
| 26085 | MRTF-B | NM_014048.3 | 2235 | gtcgtctcgcagttttatacttc | 32956 | - | see also 11336 line | | | |
| 26086 | MRTF-B | NM_014048.3 | 2233 | gtgtcgtctcgcagttttatact | 32955 | - | see also 11336 line | | | |
| 26087 | MUS81 | NM_025128.3 | 385 | caccgcacgcgcttcgtatttca | 32973 | - | see also 11337 line | | | |
| 26088 | MUS81 | NM_025128.3 | 390 | cacgcgcttcgtatttcagaagg | 32975 | - | see also 11337 line | | | |
| 26089 | MUTYH | NM_012222.1 | 1463 | ctctcacatcaagctgacatatc | 32995 | - | see also 11338 line | | | |
| 26090 | MUTYH | NM_012222.1 | 160 | gtcgtctgtgggccatcatgagg | 32988 | - | see also 11338 line | | | |
| 26091 | MUTYH | NM_012222.1 | 1584 | tccaccgccatgaaaaaggtttt | 33000 | - | see also 11338 line | | | |
| 26092 | MXD4 | NM_006454.2 | 136 | gcgacttcgccagggagaaaaca | 33003 | - | transcription_regulator | transcription co-repressor | - | |
| 26093 | MXD4 | NM_006454.2 | 138 | gacttcgccagggagaaacaaa | 33004 | - | see also 26092 line | | | |
| 26094 | MXI1 | NM_005962.2 | 920 | cacagcagcctgccgagtattgg | 33013 | - | see also 11339 line | | | |
| 26095 | MXI1 | NM_005962.2 | 605 | tgcacccggcacacaacacttgg | 33008 | - | see also 11339 line | | | |
| 26096 | MXI1 | NM_005962.2 | 387 | aacatggctacgcctcttcattc | 33005 | - | see also 11339 line | | | |
| 26097 | MYB | NM_005375.1 | 1990 | ctcgtaaatacgtgaatgcattc | 33063 | - | see also 11340 line | | | |
| 26098 | MYB | NM_005375.1 | 446 | tccgaaacgttggtctgttattg | 33019 | - | see also 11340 line | | | |
| 26099 | MYB | NM_005375.1 | 1979 | ttccagtcaagctcgtaaatacg | 33062 | - | see also 11340 line | | | |
| 26100 | MYCD | NM_153604.1 | 222 | tgccgacttggttaatatgcaca | 33074 | - | see also 9996 line | | | |
| 26101 | MYCD | NM_153604.1 | 217 | aacagtgccgacttggttaatat | 33072 | - | see also 9996 line | | | |
| 26102 | MYST3 | NM_006766.1 | 1376 | aacggcgctatactaatccaata | 33138 | - | see also 11342 line | | | |
| 26103 | MYST3 | NM_006766.1 | 1369 | cagataaaacggcgctatactaa | 33137 | - | see also 11342 line | | | |
| 26104 | MYST4 | NM_012330.1 | 2026 | atgcgtcgtaaaactgaattatc | 33287 | - | see also 5283 line | | | |
| 26105 | MYST4 | NM_012330.1 | 6177 | tcctgcacggactttaacgatgc | 33361 | - | see also 5283 line | | | |
| 26106 | MYST4 | NM_012330.1 | 822 | accgcagtacagggtcaattatg | 33248 | - | see also 5283 line | | | |
| 26107 | N-PAC | NM_032569.2 | 122 | aacctcgcggaaagaaatgcttc | 33381 | - | see also 9250 line | | | |
| 26108 | NAP1L1 | NM_004537.3 | 975 | cagttcgtactgtgactaaaaca | 33417 | - | see also 3090 line | | | |
| 26109 | NAP1L2 | NM_021963.2 | 1543 | tacgtacttacataattccaaga | 33452 | - | see also 11346 line | | | |
| 26110 | NAP1L2 | NM_021963.2 | 713 | aactagagcggccaatttagaat | 33424 | - | see also 11346 line | | | |
| 26111 | NAP1L2 | NM_021963.2 | 719 | agcggccaatttagaatccaaat | 33425 | - | see also 11346 line | | | |
| 26112 | NAP1L3 | NM_004538.2 | 473 | gccgcagccgcttgtatagaaag | 33456 | - | see also 3094 line | | | |
| 26113 | NAP1L3 | NM_004538.2 | 581 | atcgtgtgcaagcgcttagaaac | 33461 | - | see also 3094 line | | | |
| 26114 | NAP1L4 | NM_005969.2 | 735 | tggacagcctatgtcttttgtgt | 33511 | - | see also 11348 line | | | |

Figure 46

| 26115 | NAP1L4 | NM_005969.2 | 737 | gacagcctatgtcttttgtgtta | 33512 | - | see also 11348 line |
|---|---|---|---|---|---|---|---|
| 26116 | NCL | NM_005381.1 | 1276 | gagcgagatgcgagaacacttt | 33529 | - | see also 11349 line |
| 26117 | NCL | NM_005381.1 | 1017 | accgactacggctttcaatctct | 33521 | - | see also 11349 line |
| 26118 | NCL | NM_005381.1 | 1894 | tccgttcgggcaaggatagttac | 33546 | - | see also 11349 line |
| 26119 | NCOR1 | NM_006311.2 | 6785 | cccgctcaccagggagtataagc | 33678 | - | see also 4410 line |
| 26120 | NCOR1 | NM_006311.2 | 4040 | caccgttagaggggctgatatgc | 33624 | - | see also 4410 line |
| 26121 | NCOR2 | NM_006312.1 | 2122 | gtcgggcgtgagcggaaatgagg | 33709 | - | see also 11351 line |
| 26122 | NCOR2 | NM_006312.1 | 3655 | ctcggacagcgccatcacatacc | 33713 | - | see also 11351 line |
| 26123 | NCOR2 | NM_006312.1 | 3347 | agcgtcctcgagaggcaaatagg | 33711 | - | see also 11351 line |
| 26124 | NDN | NM_002487.2 | 850 | cgcccgaatacgagttctttgg | 33735 | - | see also 11352 line |
| 26125 | NDN | NM_002487.2 | 796 | aggagttcgtccaaatgaattac | 33734 | - | see also 11352 line |
| 26126 | NEUROD1 | NM_002500.1 | 1112 | tccttcgatagccattcacatca | 33746 | - | see also 1606 line |
| 26127 | NEUROD1 | NM_002500.1 | 1111 | gtccttcgatagccattcacatc | 33745 | - | see also 1606 line |
| 26128 | NEUROD1 | NM_002500.1 | 1041 | cccaaagccacggatcaatcttc | 33742 | - | see also 1606 line |
| 26129 | NEUROD4 | NM_021191.1 | 1041 | cacgaggataaatctcctatttg | 33759 | - | see also 11354 line |
| 26130 | NEUROD4 | NM_021191.1 | 1391 | cccccgttatgatgttcctatag | 33765 | - | see also 11354 line |
| 26131 | NEUROD4 | NM_021191.1 | 588 | gagagcagaccaggtacttatgg | 33747 | - | see also 11354 line |
| 26132 | NEUROD6 | NM_022728.2 | 684 | aacgacgctctggacaacttaag | 33771 | - | see also 8429 line |
| 26133 | NEUROD6 | NM_022728.2 | 685 | acgacgctctggacaacttaaga | 33772 | - | see also 8429 line |
| 26134 | NEUROG3 | NM_020999.1 | 398 | gcgacggagtcggcgaaagaagg | 33798 | - | see also 11355 line |
| 26135 | NIRF | NM_152306.2 | 1641 | gtcgtacgagagaatgtactatt | 33848 | - | see also 9835 line |
| 26136 | NIRF | NM_152306.2 | 1032 | cacgagctagaaccattttgaaa | 33826 | - | see also 9835 line |
| 26137 | NONO | NM_007363.3 | 1019 | accaagtggaccgcaacatcaag | 33887 | - | see also 11357 line |
| 26138 | NONO | NM_007363.3 | 1260 | gcggcgacagcaggaaggattca | 33888 | - | see also 11357 line |
| 26139 | NRBF-2 | NM_030759.1 | 258 | tacgaagaggctatttcttgtca | 33891 | - | see also 11358 line |
| 26140 | NRBF-2 | NM_030759.1 | 264 | gaggctatttcttgtcacaaaaa | 33892 | - | see also 11358 line |
| 26141 | NRF | NM_017544.1 | 1275 | caggcgtgtaagacaaatcctga | 33919 | - | see also 11359 line |
| 26142 | NRF | NM_017544.1 | 1030 | agcctgtaacagccaacatgtat | 33910 | - | see also 11359 line |
| 26143 | NRF1 | NM_005011.2 | 857 | aggcactacggaccatagttaaa | 33960 | - | see also 11360 line |
| 26144 | NRF1 | NM_005011.2 | 422 | ttcgagccacgttagatgaatat | 33950 | - | see also 11360 line |
| 26145 | NSD1 | NM_022455.3 | 3551 | aacctcgtaagcgcatgaacaga | 34083 | - | see also 8362 line |
| 26146 | NSD1 | NM_022455.3 | 3535 | aaccaaaggcgcactaaacctcg | 34082 | - | see also 8362 line |
| 26147 | NSD1 | NM_022455.3 | 3554 | ctcgtaagcgcatgaacagattt | 34084 | - | see also 8362 line |
| 26148 | NTHL1 | NM_002528.4 | 529 | cacgctgggcaagctcatctacc | 34175 | - | see also 11362 line |
| 26149 | NTHL1 | NM_002528.4 | 565 | gaggagcaaggtgaaatacatca | 34176 | - | see also 11362 line |
| 26150 | NUCB1 | NM_006184.3 | 655 | accgcgagcaccctaaagtcaac | 34184 | - | see also 11363 line |
| 26151 | NUCB1 | NM_006184.3 | 378 | gccgagcaggatcccaatgtaca | 34179 | - | see also 11363 line |

Figure 46

| 26152 | NUCB1 | NM_006184.3 | 519 | tacgacgcagcccatcatgaaga | 34182 | - | see also 11363 line |
|---|---|---|---|---|---|---|---|
| 26153 | NUCB2 | NM_005013.1 | 694 | atcaaagcggcaacaagtgatct | 34201 | - | see also 3509 line |
| 26154 | NUCB2 | NM_005013.1 | 705 | aacaagtgatctggaacactatg | 34202 | - | see also 3509 line |
| 26155 | NUP153 | NM_005124.2 | 2049 | ttcgcatcgccgaagatagattc | 34281 | - | see also 3601 line |
| 26156 | NUP153 | NM_005124.2 | 1118 | aacagctcggcgaatattgcagt | 34243 | - | see also 3601 line |
| 26157 | OASL | NM_003733.2 | 1126 | gagcccataaggaaggttaaaga | 34347 | - | see also 11366 line |
| 26158 | OASL | NM_003733.2 | 1462 | tggttgggtctggggatctatgg | 34350 | - | see also 11366 line |
| 26159 | OLIG2 | NM_005806.1 | 869 | tccaccgcacggcctactcaagt | 34353 | - | see also 4054 line |
| 26160 | OLIG2 | NM_005806.1 | 146 | gccagagcccgatgaccttttc | 34351 | - | see also 4054 line |
| 26161 | ORC1L | NM_004153.2 | 1152 | acctgaacatcgcataatcctga | 34379 | - | see also 11367 line |
| 26162 | ORC1L | NM_004153.2 | 1400 | agcttcggtttctaggtaatagt | 34389 | - | see also 11367 line |
| 26163 | ORC6L | NM_014321.2 | 552 | atccggtgtaaaaaaagctatat | 34420 | - | see also 5619 line |
| 26164 | ORC6L | NM_014321.2 | 242 | gccccttggacagggcttattta | 34413 | - | see also 5619 line |
| 26165 | OSMR | NM_003999.1 | 3240 | ctcccccgaccgagaatagcagc | 34501 | - | see also 11368 line |
| 26166 | OSMR | NM_003999.1 | 2863 | gactaagcctaactacctttatc | 34497 | - | see also 11368 line |
| 26167 | OSMR | NM_003999.1 | 3237 | tgcctcccccgaccgagaatagc | 34500 | - | see also 11368 line |
| 26168 | PAX1 | NM_006192.1 | 606 | gggcatccggacgtttatggagc | 34506 | - | see also 4280 line |
| 26169 | PAX1 | NM_006192.1 | 1029 | ccggacgccctcagtagcttaca | 34510 | - | see also 4280 line |
| 26170 | PAX5 | NM_016734.1 | 680 | atcaagcctggggtaattggagg | 34513 | - | see also 11370 line |
| 26171 | PAX5 | NM_016734.1 | 857 | aggatcatccggacaaaagtaca | 34516 | - | see also 11370 line |
| 26172 | PAX9 | NM_006194.1 | 802 | gggtcattacgactcatacaagc | 34524 | - | see also 11371 line |
| 26173 | PAX9 | NM_006194.1 | 1231 | agcccaagtgtcgccttacatga | 34529 | - | see also 11371 line |
| 26174 | PC326 | NM_018442.1 | 1828 | aaggccgctagtaaaaatggttt | 34561 | - | see also 11372 line |
| 26175 | PC326 | NM_018442.1 | 1829 | aggccgctagtaaaaatggttta | 34562 | - | see also 11372 line |
| 26176 | PC326 | NM_018442.1 | 1825 | tagaaggccgctagtaaaaatgg | 34560 | - | see also 11372 line |
| 26177 | PCBP3 | NM_020528.1 | 1020 | accattcccaatgatctaatagg | 34584 | - | see also 7889 line |
| 26178 | PCDH1 | NM_002587.3 | 967 | tcctatgaggccgaactatctga | 34594 | - | see also 11374 line |
| 26179 | PCDH1 | NM_002587.3 | 344 | gcctcgcagccgactatggtttt | 34589 | - | see also 11374 line |
| 26180 | PCDH1 | NM_002587.3 | 346 | ctcgcagccgactatggttttcc | 34590 | - | see also 11374 line |
| 26181 | PCNA | NM_002592.2 | 530 | taccttggcgctagtatttgaag | 34626 | - | see also 11375 line |
| 26182 | PCNA | NM_002592.2 | 503 | tacactaagggccgaagataacg | 34624 | - | see also 11375 line |
| 26183 | PDCD2 | NM_002598.2 | 601 | accagatcatctggaccatataa | 34642 | - | see also 11376 line |
| 26184 | PHF10 | NM_018288.2 | 1074 | aaggtcattccaaatgctatatg | 34669 | - | see also 7369 line |
| 26185 | PHF10 | NM_018288.2 | 1068 | aagccaaaggtcattccaaatgc | 34668 | - | see also 7369 line |
| 26186 | PHF11 | NM_016119.1 | 362 | agccaccgtgggatgtgatttaa | 34688 | - | see also 11377 line |
| 26187 | PHF11 | NM_016119.1 | 495 | ttcccgatcatcgctcaaagtgc | 34693 | - | see also 11377 line |
| 26188 | PHF12 | NM_020889.1 | 2481 | taggacctccgttgacagattca | 34732 | - | see also 8012 line |

Figure 46

| | | | | | | | |
|---|---|---|---|---|---|---|---|
| 26189 | PHF13 | NM_153812.1 | 1161 | gtgcgaaatccggaaatccaat | 34744 | - | see also 11379 line |
| 26190 | PHF13 | NM_153812.1 | 1114 | cccatgatcgagtgtaatgagt | 34743 | - | see also 11379 line |
| 26191 | PHF13 | NM_153812.1 | 1162 | tgcgaaaatccggaaatccaatg | 34745 | - | see also 11379 line |
| 26192 | PHF14 | NM_014660.1 | 1743 | gaccctcgctttgctagaactgg | 34776 | - | see also 5831 line |
| 26193 | PHF14 | NM_014660.1 | 1568 | gtgtcctaatcaggatggaatt | 34766 | - | see also 5831 line |
| 26194 | PHF15 | NM_015288.4 | 232 | ctgacagtcatgcgacatctaca | 34814 | - | see also 6314 line |
| 26195 | PHF16 | NM_014735.2 | 617 | atccgacccggaagtatattca | 34833 | - | see also 5924 line |
| 26196 | PHF16 | NM_014735.2 | 619 | ccgacccggaagtatattcact | 34834 | - | see also 5924 line |
| 26197 | PHF3 | NM_015153.1 | 4944 | agcgaggtccacagtttatca | 35003 | - | see also 11383 line |
| 26198 | PHF3 | NM_015153.1 | 3164 | aacgacggccaatcaccaaaata | 34960 | - | see also 11383 line |
| 26199 | PHF6 | NM_032335.1 | 598 | gagccctcatcacctaaaagtaa | 35046 | - | see also 9165 line |
| 26200 | PHF6 | NM_032335.1 | 813 | agcacgaggaaaactgcatatat | 35049 | - | see also 9165 line |
| 26201 | PIAS1 | NM_016166.1 | 1704 | gccttacgacttacaaggattag | 35123 | - | see also 6696 line |
| 26202 | PIAS1 | NM_016166.1 | 862 | gtccgactgtccacaacagtacc | 35096 | - | see also 6696 line |
| 26203 | PIAS3 | NM_006099.2 | 836 | atctgagttcggacggaattact | 35133 | - | see also 11385 line |
| 26204 | PIAS3 | NM_006099.2 | 1617 | gccgacatccaagtttagattt | 35151 | - | see also 11385 line |
| 26205 | PIAS3 | NM_006099.2 | 1616 | agccgacatccaagtttagatt | 35150 | - | see also 11385 line |
| 26206 | PIASY | NM_015897.2 | 681 | cgggctacctaccctccaataag | 35166 | - | see also 11386 line |
| 26207 | PIASY | NM_015897.2 | 334 | cccgtgtccacggaaagtact | 35158 | - | see also 11386 line |
| 26208 | PIK4CA | NM_002650.1 | 1275 | ggcgagtacgggtgaaagtcc | 35177 | - | see also 11387 line |
| 26209 | PIK4CA | NM_002650.1 | 1370 | tgggctatgtgcgggatatatt | 35179 | - | see also 11387 line |
| 26210 | PJA2 | NM_014819.1 | 1247 | atcaccatactcaagagttatt | 35289 | - | see also 6012 line |
| 26211 | PJA2 | NM_014819.1 | 493 | gaggaaggcaggggataccttagg | 35264 | - | see also 6012 line |
| 26212 | PJA2 | NM_014819.1 | 1994 | ctgctattggtcaggaacaatgc | 35303 | - | see also 6012 line |
| 26213 | PLAC3 | NM_020318.1 | 1597 | gacgcttatctggccatcaaag | 35316 | - | see also 11389 line |
| 26214 | PLAC3 | NM_020318.1 | 1934 | aagtaggactgccatcttatac | 35323 | - | see also 11389 line |
| 26215 | PLAGL1 | NM_002656.2 | 2951 | ctcctgctaccaaaataccttt | 35406 | - | see also 11390 line |
| 26216 | PLAGL1 | NM_002656.2 | 2220 | ccttagcggaaccaaggaaaaga | 35398 | - | see also 11390 line |
| 26217 | PMS1 | NM_000534.2 | 2512 | ctcgtcttacagcgaatggtttc | 35493 | - | see also 404 line |
| 26218 | PMS1 | NM_000534.2 | 1959 | gacttggaacgatacaatagtca | 35477 | - | see also 404 line |
| 26219 | PMS2 | NM_000535.2 | 944 | atgaggtctaccacatgtataat | 35513 | - | see also 11392 line |
| 26220 | PMS2 | NM_000535.2 | 1008 | ttcagaatgcgttgatatcaatg | 35514 | - | see also 11392 line |
| 26221 | POGK | NM_017542.3 | 1722 | cccaaccgggaatgctaagaagc | 35545 | - | see also 7022 line |
| 26222 | POGK | NM_017542.3 | 911 | agcgtgtggccgaatatgtcaga | 35533 | - | see also 7022 line |
| 26223 | POGZ | NM_015100.1 | 386 | aacggttagaccaattacactag | 35560 | - | see also 6238 line |
| 26224 | POGZ | NM_015100.1 | 1420 | aggtagatggtcaactatctgc | 35580 | - | see also 6238 line |
| 26225 | POLA | NM_016937.1 | 3676 | ggctcggatctgtgaaccaatag | 35707 | - | see also 6972 line |

Figure 46

| | | | | | | | |
|---|---|---|---|---|---|---|---|
| 26226 | POLA | NM_016937.1 | 4065 | agccaacctgtcgcaatgcgaact | 35721 | - | see also 6972 line |
| 26227 | POLA | NM_016937.1 | 3939 | atcgttgcagtaacatcgattgt | 35714 | - | see also 6972 line |
| 26228 | POLB | NM_002690.1 | 563 | tcctcgtgaagagatgttacaaa | 35743 | - | see also 1752 line |
| 26229 | POLB | NM_002690.1 | 519 | cagcgaattgggctgaaatatt | 35740 | - | see also 1752 line |
| 26230 | POLD1 | NM_002691.1 | 630 | tgctcccgagagagcatgtttgg | 35758 | - | see also 11397 line |
| 26231 | POLD1 | NM_002691.1 | 1225 | acgtgatcaccgttacaacatc | 35761 | - | see also 11397 line |
| 26232 | POLD3 | NM_006591.1 | 127 | aacaagatcgtgacattacaaatg | 35769 | - | see also 4617 line |
| 26233 | POLD3 | NM_006591.1 | 1226 | aacgcgtactaaaatctaaaact | 35790 | - | see also 4617 line |
| 26234 | POLE | NM_006231.1 | 4177 | gtgcttcgtatcgcaaggtaaat | 35850 | - | see also 4320 line |
| 26235 | POLE2 | NM_002692.2 | 546 | atcggagatgcgattgttcttgg | 35898 | - | see also 11399 line |
| 26236 | POLE2 | NM_002692.2 | 261 | gagcacgtttcaatatcatagg | 35888 | - | see also 11399 line |
| 26237 | POLE3 | NM_017443.3 | 239 | tcccgaacggtgtcaacatctcc | 35929 | - | see also 11400 line |
| 26238 | POLE3 | NM_017443.3 | 324 | tgctaacaacttgcaatgaaag | 35931 | - | see also 11400 line |
| 26239 | POLE4 | NM_019896.2 | 265 | ttgcaaaagatgcctactgttgc | 35933 | - | see also 11401 line |
| 26240 | POLE4 | NM_019896.2 | 318 | aggagaactggataatgcaat | 35934 | - | see also 11401 line |
| 26241 | POLG | NM_002693.1 | 2065 | cagatgcgggtcacacctaaact | 35951 | - | see also 1754 line |
| 26242 | POLG | NM_002693.1 | 2683 | tggaggaacgcccataaaacgtat | 35959 | - | see also 1754 line |
| 26243 | POLL | NM_013274.2 | 1497 | ggccatcgcctgaagcattaca | 35978 | - | see also 11403 line |
| 26244 | POLL | NM_013274.2 | 1501 | atcggcctgaagcattacagtga | 35979 | - | see also 11403 line |
| 26245 | POLM | NM_013284.1 | 913 | gtcctgcggtcgatgtagatgc | 35990 | - | see also 11404 line |
| 26246 | POLM | NM_013284.1 | 234 | ctccgaagcgacacatgttgtga | 35986 | - | see also 11404 line |
| 26247 | POLR1A | NM_015425.1 | 473 | gtctacgagcttgagagaattct | 36008 | - | see also 6408 line |
| 26248 | POLR1A | NM_015425.1 | 1173 | ttgctattgaccgatccttttg | 36024 | - | see also 6408 line |
| 26249 | POLR1B | NM_019014.2 | 2160 | atcaagaaccgatcggataacaaa | 36132 | - | see also 11406 line |
| 26250 | POLR1B | NM_019014.2 | 2166 | accgatcggataacaaactgtat | 36133 | - | see also 11406 line |
| 26251 | POLR1B | NM_019014.2 | 218 | ctgcggtcgaggctatacctcc | 36083 | - | see also 11406 line |
| 26252 | POLR1C | NM_004875.1 | 921 | cccggttcgagatcattatatc | 36193 | - | see also 11407 line |
| 26253 | POLR1C | NM_004875.1 | 372 | ctgatcccgtcttttgagtat | 36186 | - | see also 11407 line |
| 26254 | POLR1C | NM_004875.1 | 239 | gccaatgcttttcgacgaattct | 36182 | - | see also 11407 line |
| 26255 | POLR2A | NM_000937.2 | 2425 | tcctcatcgaggtcatactatt | 36223 | - | see also 11408 line |
| 26256 | POLR2A | NM_000937.2 | 2602 | atcaggtgaaccgcattcttaac | 36229 | - | see also 11408 line |
| 26257 | POLR2A | NM_000937.2 | 4156 | tccgtattcgcatcatgaacagc | 36255 | - | see also 11408 line |
| 26258 | POLR2B | NM_000938.1 | 2960 | ccctctcgtatgactattggtca | 36387 | - | see also 616 line |
| 26259 | POLR2C | NM_002694.2 | 153 | ttcgattcgagggtcttcatcg | 36408 | - | see also 11410 line |
| 26260 | POLR2C | NM_002694.2 | 447 | gacatcccggaaccgagataatg | 36414 | - | see also 11410 line |
| 26261 | POLR2E | NM_002695.2 | 40 | gacgtaccggctctgaaaatcc | 36425 | - | transcription_regulator / protein binding - |

Figure 46

| | | | | | | | | |
|---|---|---|---|---|---|---|---|---|
| 26262 | POLR2F | NM_021974.2 | 374 | ccccgaaagatcccatcatcatt | 36429 | - | | see also 11411 line |
| 26263 | POLR2F | NM_021974.2 | 228 | gcgaatcaccacaccacatacatga | 36428 | - | | see also 11411 line |
| 26264 | POLR2J | NM_006234.3 | 359 | gcgctttcgggtggccataaaag | 36430 | - | transcription_regulator | see also 26264 line |
| 26265 | POLR2J | NM_006234.3 | 360 | cgctttcgggtggccataaaaga | 36431 | - | | see also 26264 line |
| 26266 | POLR2J | NM_006234.3 | 364 | ttcgggtggccataaaagacaag | 36432 | - | | see also 26264 line |
| 26267 | POLR2K | NM_005034.2 | 210 | gactaaaagattcgtcgttttg | 36436 | - | | see also 11412 line |
| 26268 | POLR2K | NM_005034.2 | 90 | acctccaaagcagcaaccaatga | 36434 | - | | see also 11412 line |
| 26269 | POLRMT | NM_005035.2 | 1846 | tgctctaccacgtgattccttc | 36440 | - | | see also 11413 line |
| 26270 | POLRMT | NM_005035.2 | 865 | aggagctgtatatgtgttattc | 36438 | - | | see also 11413 line |
| 26271 | POLS | NM_006999.2 | 284 | gacaaggctacggtaccaataat | 36448 | - | | see also 4887 line |
| 26272 | POLS | NM_006999.2 | 287 | aaggctacgtaccaataataaa | 36449 | - | | see also 4887 line |
| 26273 | PPARBP | NM_004774.2 | 3171 | gcgcagtcggaccccttctaatg | 36562 | - | | see also 3284 line |
| 26274 | PPARBP | NM_004774.2 | 1393 | ggccgagttcctttatcctaaa | 36519 | - | | see also 3284 line |
| 26275 | PPARGC1 | NM_013261.2 | 1948 | caccgcacgcaccgaaattctcc | 36636 | - | | see also 5378 line |
| 26276 | PPFIBP1 | NM_003622.2 | 538 | acctgcgttggattgttagagatg | 36647 | - | | see also 11417 line |
| 26277 | PPFIBP1 | NM_003622.2 | 542 | gcgttgattgttagagatgatgg | 36648 | - | | see also 11417 line |
| 26278 | PQBP1 | NM_057710.1 | 497 | cccaactccgtggttaccaaat | 36724 | - | | see also 11418 line |
| 26279 | PQBP1 | NM_057710.1 | 498 | cccaactccgtggttaccaaatc | 36725 | - | | see also 11418 line |
| 26280 | PRDM10 | NM_020228.1 | 3186 | ggcggttacttcgggtcattatg | 36772 | - | | see also 11419 line |
| 26281 | PRDM10 | NM_020228.1 | 2277 | ttgtcagttattgcgataaggttt | 36760 | - | | see also 11419 line |
| 26282 | PRDM12 | NM_021619.2 | 655 | ctggtgttggtacggaaaactcaca | 36784 | - | | see also 11420 line |
| 26283 | PRDM12 | NM_021619.2 | 488 | atggcacggtgcgctacttcatc | 36780 | - | | see also 11420 line |
| 26284 | PRDM13 | NM_021620.1 | 1158 | cgggtgcgctcagcttcaagc | 36790 | - | | see also 11421 line |
| 26285 | PRDM13 | NM_021620.1 | 1721 | ccgcttattaccgctcaattg | 36793 | - | | see also 11421 line |
| 26286 | PRDM14 | NM_024504.1 | 854 | agcctgcaccatgcgatttcagg | 36801 | - | | see also 11422 line |
| 26287 | PRDM14 | NM_024504.1 | 1084 | gacctacggagacaattctgtga | 36807 | - | | see also 11422 line |
| 26288 | PRDM4 | NM_012406.2 | 556 | cagcactagatccctatcagtcc | 36834 | - | | see also 11423 line |
| 26289 | PRDM4 | NM_012406.2 | 718 | gggttaccagcccaatttcgaca | 36839 | - | | see also 11423 line |
| 26290 | PRDM5 | NM_018699.1 | 1094 | atcagctaaaagtcatatgatc | 36885 | - | | see also 7569 line |
| 26291 | PRDM5 | NM_018699.1 | 1144 | tgccgagatttgtaataagtcttt | 36890 | - | | see also 7569 line |
| 26292 | PRDM7 | NM_052996.1 | 529 | cacaggcagatcttctatagaac | 36911 | - | transcription_regulator, DNA binding | |
| 26293 | PRDM7 | NM_052996.1 | 852 | tccacgctgctaatgacaaaa | 36908 | - | | see also 26292 line |
| 26294 | PRDM7 | NM_052996.1 | 854 | cacgctgctaatgacaaaacc | 36909 | - | | see also 26292 line |
| 26295 | PRDM9 | NM_020227.1 | 723 | tccacacacagccgtaatgacaaaa | 36913 | - | transcription_regulator, DNA binding | |

Figure 46

| | | | | | | | |
|---|---|---|---|---|---|---|---|
| 26296 | PRDM9 | NM_020227.1 | 722 | atccacacagccgtaatgacaaa | 36912 | - | see also 26295 line |
| 26297 | PREB | NM_013388.3 | 1375 | gtgtgtcggcttattattgtga | 36928 | - | see also 11425 line |
| 26298 | PREB | NM_013388.3 | 1377 | gtgtcggcttattattgtgacc | 36929 | - | see also 11425 line |
| 26299 | PRIM2A | NM_000947.1 | 460 | atggatctcctcgatttagatt | 36938 | - | see also 629 line |
| 26300 | PRIM2A | NM_000947.1 | 1438 | agccaacgtattctaaatggtgg | 36962 | - | see also 629 line |
| 26301 | PRKCBP1 | NM_012408.3 | 3459 | atcgacacagttccgatcacaaca | 37030 | - | see also 11427 line |
| 26302 | PRKCBP1 | NM_012408.3 | 442 | cgggtttatcacgcttaagtgtct | 36974 | - | see also 11427 line |
| 26303 | PRKRIR | NM_004705.2 | 2099 | gacgaaagcgtcttaaagcatat | 37087 | - | see also 3228 line |
| 26304 | PRKRIR | NM_004705.2 | 1389 | agctggccgagcatttgtactct | 37072 | - | see also 3228 line |
| 26305 | PTPRF | NM_002840.2 | 1952 | gagcggatcatcatgtatgaact | 37146 | - | see also 11429 line |
| 26306 | PTPRF | NM_002840.2 | 1673 | cggggataccgcgtctactatac | 37143 | - | see also 11429 line |
| 26307 | PYGO1 | NM_015617.1 | 589 | aaacccgatttggcatctaattt | 37200 | - | see also 11430 line |
| 26308 | PYGO1 | NM_015617.1 | 1210 | atgcgtactagagaaacttttgg | 37218 | - | see also 11430 line |
| 26309 | PYGO1 | NM_015617.1 | 20 | cagctccgcttacaaagtttcc | 37180 | - | see also 11430 line |
| 26310 | PYGO2 | NM_138300.2 | 678 | agcctctgggtcaaaactttagt | 37220 | - | see also 11431 line |
| 26311 | RAB10 | NM_016131.2 | 996 | tcctcacgttagctgaagatatc | 37242 | - | see also 6684 line |
| 26312 | RAB25 | NM_020387.1 | 302 | ccgattcacgcgcaatgagttc | 37246 | - | see also 11433 line |
| 26313 | RAB25 | NM_020387.1 | 632 | ccgaatgttcgctgaaaacaat | 37250 | - | see also 11433 line |
| 26314 | RAB25 | NM_020387.1 | 630 | ggcccgaatgttcgctgaaaaca | 37249 | - | see also 11433 line |
| 26315 | RABGEF1 | NM_014504.1 | 1180 | agctagacgcccagtcttgaat | 37269 | - | see also 11434 line |
| 26316 | RABGEF1 | NM_014504.1 | 459 | aaccggcaaaccagcattgaaac | 37253 | - | see also 11434 line |
| 26317 | RAD17 | NM_002873.1 | 984 | accaaaacaggggtggatctattt | 37291 | - | see also 1910 line |
| 26318 | RAD17 | NM_002873.1 | 941 | aagaagtcgaaacctggttaaaa | 37288 | - | see also 1910 line |
| 26319 | RAD52 | NM_002879.2 | 308 | ttgagggtcatcgggtaattaat | 37340 | - | see also 1919 line |
| 26320 | RAD54B | NM_012415.2 | 2061 | cacgaaacttgacctactgaaaa | 37413 | - | see also 11437 line |
| 26321 | RAD54B | NM_012415.2 | 872 | taagcccaaattccctcgttatgc | 37370 | - | see also 11437 line |
| 26322 | RAD54L | NM_003579.2 | 2263 | tgcccgaaggtacttatacgtcc | 37476 | - | see also 2374 line |
| 26323 | RAD54L | NM_003579.2 | 2259 | gccgtgccgaaggtacttatac | 37474 | - | see also 2374 line |
| 26324 | RAG1 | NM_000448.1 | 221 | ttccgggtgagatcctttgaaaa | 37487 | - | see also 11439 line |
| 26325 | RAG1 | NM_000448.1 | 218 | ctgttccgggtgagatcctttga | 37486 | - | see also 11439 line |
| 26326 | RAG2 | NM_000536.1 | 729 | tgggtgtgctacatcatacattc | 37566 | - | see also 11440 line |
| 26327 | RAG2 | NM_000536.1 | 1444 | aacacttgggtaccattctattc | 37588 | - | see also 11440 line |
| 26328 | RAI1 | NM_030665.3 | 5864 | gccgacaagggtcgcaaacatga | 37626 | - | see also 11441 line |
| 26329 | RAI1 | NM_030665.3 | 6021 | tggccgtggacatgatgtgttcc | 37628 | - | see also 11441 line |
| 26330 | RBL2 | NM_005611.2 | 2054 | ctccaaccacattatacgatagg | 37699 | - | see also 3898 line |
| 26331 | RBL2 | NM_005611.2 | 2052 | atctccaaccacattatacgata | 37698 | - | see also 3898 line |
| 26332 | RBM5 | NM_005778.1 | 908 | tacgcgtctttagctgtcaataa | 37755 | - | see also 4039 line |

Figure 46

| 26333 | RBM5 | NM_005778.1 | 1592 | cccaactcgcaatactactataa | 37772 | - | see also 4039 line |
|---|---|---|---|---|---|---|---|
| 26334 | RBM5 | NM_005778.1 | 1566 | atccgacaacagggctctattat | 37767 | - | see also 4039 line |
| 26335 | RBM6 | NM_005777.1 | 2109 | tgctaaagcgtatctatcgttcc | 37846 | - | see also 4031 line |
| 26336 | RBM6 | NM_005777.1 | 1967 | gagtcacgcttaggacatcaaaa | 37840 | - | see also 4031 line |
| 26337 | RBM6 | NM_005777.1 | 1317 | ggcggcaagacaccgattacaga | 37822 | - | see also 4031 line |
| 26338 | RBPSUH | NM_005349.1 | 1407 | gccaggtgcccctaacgaatca | 37889 | - | see also 3734 line |
| 26339 | RBPSUH | NM_005349.1 | 1406 | agccaggtgcccctaacgaatc | 37888 | - | see also 3734 line |
| 26340 | RBPSUHL | NM_014276.2 | 1158 | cggcaccgagtcggtggaatttt | 37909 | - | see also 11446 line |
| 26341 | RBPSUHL | NM_014276.2 | 934 | cccatgatcatccgtaaagtagc | 37907 | - | see also 11446 line |
| 26342 | RBPSUHL | NM_014276.2 | 423 | gcccacggtctgcggttacatgg | 37904 | - | see also 11446 line |
| 26343 | RCOR | NM_015156.1 | 703 | aagacgaggactaaaactagtgt | 37919 | - | see also 6259 line |
| 26344 | RCOR | NM_015156.1 | 402 | ctggtcacccaatcaaaatctgt | 37914 | - | see also 6259 line |
| 26345 | RECQL | NM_002907.2 | 1410 | agggcaatcaggaatcatatatt | 37966 | - | see also 11447 line |
| 26346 | RECQL | NM_002907.2 | 1720 | tactacggctttggagatatatt | 37978 | - | see also 11447 line |
| 26347 | REPIN1 | NM_013400.1 | 864 | ccggcacaagcccaacttgatcg | 37997 | - | see also 11448 line |
| 26348 | REPIN1 | NM_013400.1 | 863 | tccggcacaagcccaacttgatc | 37996 | - | see also 11448 line |
| 26349 | REV3L | NM_002912.1 | 8742 | tgcgcgtcagttgggacttaagc | 38245 | - | see also 1946 line |
| 26350 | REV3L | NM_002912.1 | 2669 | accgtcaccctaacgagaataca | 38037 | - | see also 1946 line |
| 26351 | RFC1 | NM_002913.3 | 2267 | cacgaaacatgctctcatcatgg | 38358 | - | see also 1958 line |
| 26352 | RFC1 | NM_002913.3 | 3029 | ctcgtctacaggcaaacatgatc | 38380 | - | see also 1958 line |
| 26353 | RFC2 | NM_002914.3 | 418 | tcgcttcgcccttgcttgtaatg | 38402 | - | see also 11451 line |
| 26354 | RFC2 | NM_002914.3 | 523 | caggctgatgaatgttatcgaga | 38406 | - | see also 11451 line |
| 26355 | RFC3 | NM_002915.2 | 241 | gtgtattctacgtgaactttatg | 38423 | - | see also 11452 line |
| 26356 | RFC3 | NM_002915.2 | 239 | atgtgtattctacgtgaacttta | 38422 | - | see also 11452 line |
| 26357 | RFC4 | NM_002916.3 | 957 | ttcaaagcgctactcgattaaca | 38466 | - | see also 1963 line |
| 26358 | RFC4 | NM_002916.3 | 640 | gccgtgtccgccttttaagattg | 38451 | - | see also 1963 line |
| 26359 | RFC4 | NM_002916.3 | 734 | tcgaaaaccacccgattctgtct | 38456 | - | see also 1963 line |
| 26360 | RFC5 | NM_007370.3 | 844 | cacccgctcaagtcagacattgc | 38485 | - | see also 11454 line |
| 26361 | RFC5 | NM_007370.3 | 431 | gaccgatcctgagctttgctagc | 38478 | - | see also 11454 line |
| 26362 | RFP | NM_006510.3 | 441 | tgctcgactgcggccataacatc | 38492 | - | see also 11455 line |
| 26363 | RFP | NM_006510.3 | 1826 | tgccccatgagtgggatagatgg | 38503 | - | see also 11455 line |
| 26364 | RFX2 | NM_000635.2 | 887 | aactgatccgttctgtgtttatg | 38507 | - | see also 11456 line |
| 26365 | RFX2 | NM_000635.2 | 2199 | gacgctgctcgacaaagatgaca | 38513 | - | see also 11456 line |
| 26366 | RFX4 | NM_002920.3 | 1046 | tgcgaaacatcaagttcgaattg | 38533 | - | see also 11457 line |
| 26367 | RFX4 | NM_002920.3 | 1482 | cgggtcttttcacctaattcact | 38544 | - | see also 11457 line |
| 26368 | RFXDC1 | NM_173560.1 | 162 | gggcaagggcttgctagtctatc | 38562 | - | see also 10083 line |
| 26369 | RFXDC1 | NM_173560.1 | 163 | ggcaagggcttgctagtctatcc | 38563 | - | see also 10083 line |

Figure 46

| 26370 | RNF11 | NM_014372.3 | 476 | atccccacctcggatgacatct | 38635 | - | see also 5636 line | | |
|---|---|---|---|---|---|---|---|---|---|
| 26371 | RPC155 | NM_007055.2 | 2767 | aagctctactggcgatattatcc | 38711 | - | see also 4940 line | | |
| 26372 | RPC155 | NM_007055.2 | 334 | aggccactatgggtatatcgacc | 38643 | - | see also 4940 line | | |
| 26373 | RREB1 | NM_002955.3 | 1429 | cagcgagcaacaccgttttgtct | 38755 | - | see also 11460 line | | |
| 26374 | RREB1 | NM_002955.3 | 1259 | aacgcgcttgtccacaaacaact | 38750 | - | see also 11460 line | | |
| 26375 | RUNX3 | NM_004350.1 | 378 | tgcctcggccgtcatgaagaacc | 38776 | - | see also 11461 line | | |
| 26376 | SAFB2 | NM_014649.1 | 1742 | agcggacggtcgtgatggataaa | 38797 | - | see also 11462 line | | |
| 26377 | SAFB2 | NM_014649.1 | 1287 | aacacgcgctacggatctcaaga | 38787 | - | see also 11462 line | | |
| 26378 | SAFB2 | NM_014649.1 | 1775 | agcccgtcattagcgtgaaaacc | 38801 | - | see also 11462 line | | |
| 26379 | SALL1 | NM_002968.1 | 95 | aaccgagtcgccctactaagagc | 38809 | - | see also 1999 line | | |
| 26380 | SALL1 | NM_002968.1 | 3513 | cacgactaaaggcaatcttaagg | 38854 | - | see also 1999 line | | |
| 26381 | SALL3 | NM_171999.1 | 3863 | cgcggtttatcgaggataacaag | 38875 | - | see also 11464 line | | |
| 26382 | SALL3 | NM_171999.1 | 3855 | cccattcacgcggtttatcgagg | 38873 | - | see also 11464 line | | |
| 26383 | SALL3 | NM_171999.1 | 3861 | cacgcggtttatcgaggataaca | 38874 | - | see also 11464 line | | |
| 26384 | SALL4 | NM_020436.2 | 2893 | aggtacggacggaaaaagagtct | 38911 | - | see also 7875 line | | |
| 26385 | SALL4 | NM_020436.2 | 3119 | ggctcccagtcgggtatcagtgc | 38913 | - | see also 7875 line | | |
| 26386 | SCOTIN | NM_016479.3 | 209 | tgcacgtggtgaggtgtgtatgg | 38914 | - | see also 11466 line | | |
| 26387 | SCOTIN | NM_016479.3 | 308 | ctctgacgtgctgaagaaatttg | 38918 | - | see also 11466 line | | |
| 26388 | SE20-4 | NM_022117.1 | 964 | ttgatcaaccgacgtgatgaaga | 38930 | - | see also 8281 line | | |
| 26389 | SE20-4 | NM_022117.1 | 1304 | tacgctactacctgagagaaagg | 38935 | - | see also 8281 line | | |
| 26390 | SET | NM_003011.1 | 59 | gggccgacgagacctcagaaaaa | 38949 | - | phosphatase | protein phosphatase inhibitor | Wilms tumor |
| 26391 | SET | NM_003011.1 | 60 | ggccgacgagacctcagaaaag | 38950 | - | see also 26390 line | | |
| 26392 | SET | NM_003011.1 | 61 | gccgacgagacctcagaaaaga | 38951 | - | see also 26390 line | | |
| 26393 | SETBP1 | NM_015559.1 | 1629 | ctcacagtcgagacgattcatga | 38985 | - | see also 11468 line | | |
| 26394 | SETBP1 | NM_015559.1 | 1631 | cacagtcgagacgattcatgagg | 38986 | - | see also 11468 line | | |
| 26395 | SETDB1 | NM_012432.1 | 869 | cagtcgggtggtcgccaaataca | 39041 | - | see also 5321 line | | |
| 26396 | SETDB1 | NM_012432.1 | 3655 | gcgcacctgttcgtaagaacaca | 39100 | - | see also 5321 line | | |
| 26397 | SETDB2 | NM_031915.1 | 2764 | ggccgcttccttaatcatagttg | 39171 | - | see also 11470 line | | |
| 26398 | SETDB2 | NM_031915.1 | 1377 | ttcagcactccacgtgagttata | 39119 | - | see also 11470 line | | |
| 26399 | SFPQ | NM_005066.1 | 2174 | gagtacgaaggcccaaacaaaaa | 39220 | - | see also 3531 line | | |
| 26400 | SFPQ | NM_005066.1 | 1924 | tggcggaggagcaatgaacatgg | 39214 | - | see also 3531 line | | |
| 26401 | SHPRH | NM_173082.1 | 3903 | cagcgagtgcgaaatgaaataac | 39317 | - | see also 10050 line | | |
| 26402 | SIRT1 | NM_012238.3 | 467 | ggcgattgggtaccgagataacc | 39378 | - | see also 5214 line | | |
| 26403 | SIRT1 | NM_012238.3 | 589 | ggccacggataggtccatatact | 39381 | - | see also 5214 line | | |
| 26404 | SIRT1 | NM_012238.3 | 2043 | gacgtcttatcctctagttcttg | 39439 | - | see also 5214 line | | |
| 26405 | SIRT2 | NM_012237.2 | 791 | cgccagctgccggcacgaatacc | 39452 | - | see also 11474 line | | |

Figure 46

| 26406 | SIRT2 | NM_012237.2 | 812 | cccgctaagctggatgaaagaga | 39453 | - | see also 11474 line |
|---|---|---|---|---|---|---|---|
| 26407 | SIRT2 | NM_012237.2 | 494 | tccatccaccggcctctatgaca | 39447 | - | see also 11474 line |
| 26408 | SIRT3 | NM_012239.3 | 621 | cccgtaccccgaggccattttg | 39461 | - | see also 11475 line |
| 26409 | SIRT3 | NM_012239.3 | 959 | ttgtgaagcccgacattgtgttc | 39464 | - | see also 11475 line |
| 26410 | SIRT4 | NM_012240.1 | 328 | agcggtactgggcgagaaacttc | 39474 | - | see also 11476 line |
| 26411 | SIRT4 | NM_012240.1 | 860 | cccgattgcaatactgaacattg | 39480 | - | see also 11476 line |
| 26412 | SIRT5 | NM_012241.2 | 760 | tggagatccatggtagcttattt | 39486 | - | see also 11477 line |
| 26413 | SIRT5 | NM_012241.2 | 1008 | gagctcgcccactgtgatttatg | 39490 | - | see also 11477 line |
| 26414 | SIRT7 | NM_016538.1 | 1039 | tggcaggatcccattttctcact | 39497 | - | - - - |
| 26415 | SIRT7 | NM_016538.1 | 837 | gtccagcctgaaggttctaaaga | 39496 | - | see also 26414 line |
| 26416 | SLK | NM_014720.1 | 3449 | ctcgaagaacgacacttacaaga | 39572 | - | see also 5913 line |
| 26417 | SLK | NM_014720.1 | 1614 | aacgtagtaactctgaagataaa | 39531 | - | see also 5913 line |
| 26418 | SMAP1 | NM_021940.2 | 774 | gacatctttggaccgatgatttc | 39604 | - | see also 8204 line |
| 26419 | SMAP1 | NM_021940.2 | 1154 | gcccatgcccaatgggtttatgg | 39610 | - | see also 8204 line |
| 26420 | SMAP1 | NM_021940.2 | 762 | aacgatgatctggacatctttgg | 39603 | - | see also 8204 line |
| 26421 | SMARCA1 | NM_003069.2 | 1907 | atggatcgagcacatcgtattgg | 39680 | - | see also 2053 line |
| 26422 | SMARCA1 | NM_003069.2 | 1277 | ttgttacgccgtataaaaactga | 39650 | - | see also 2053 line |
| 26423 | SMARCA2 | NM_003070.2 | 4068 | ggccatcgaagacggcaatttgg | 39773 | - | see also 11482 line |
| 26424 | SMARCA2 | NM_003070.2 | 1141 | gcccgcatagctcataggataca | 39710 | - | see also 11482 line |
| 26425 | SMARCA3 | NM_003071.2 | 2320 | tgcttagactgcggcaaatttgt | 39842 | - | see also 11483 line |
| 26426 | SMARCA3 | NM_003071.2 | 2712 | cacgcattgactgacttaagaaa | 39855 | - | see also 11483 line |
| 26427 | SMARCA5 | NM_003601.2 | 1010 | atcaggtccgaggattaaactgg | 39878 | - | see also 11484 line |
| 26428 | SMARCA5 | NM_003601.2 | 2166 | tgcgactgctgatgtagtaattt | 39913 | - | see also 11484 line |
| 26429 | SMARCA5 | NM_003601.2 | 3037 | aagtggggtcgtgatgatattga | 39937 | - | see also 11484 line |
| 26430 | SMARCAD1 | NM_020159.1 | 1928 | ttcgacggctgaaacttaattac | 40000 | - | see also 11485 line |
| 26431 | SMARCAD1 | NM_020159.1 | 1929 | tcgacggctgaaacttaattacg | 40001 | - | see also 11485 line |
| 26432 | SMARCAL1 | NM_014140.2 | 1385 | gaccccaagctcgtgtctaatct | 40074 | - | see also 5513 line |
| 26433 | SMARCAL1 | NM_014140.2 | 697 | ttcggggcagaacatttcttaca | 40057 | - | see also 5513 line |
| 26434 | SMARCF1 | NM_006015.3 | 3103 | gccgccaggctaccccaatatga | 40111 | - | see also 4188 line |
| 26435 | SMARCF1 | NM_006015.3 | 7153 | ctcggtatcaccgttgatgaact | 40177 | - | see also 4188 line |
| 26436 | SMCX | NM_004187.1 | 1690 | caggctacccgggaatacactct | 40192 | - | see also 2809 line |
| 26437 | SMCX | NM_004187.1 | 997 | cactacgaacgcattgtttatcc | 40184 | - | see also 2809 line |
| 26438 | SMCY | NM_004653.2 | 2223 | gagacgttggatctcaatctagc | 40221 | - | see also 11488 line |

Figure 46

| | | | | | | |
|---|---|---|---|---|---|---|
| 26439 | SMCY | NM_004653.2 | 3027 | caggcgcaatggctagatgaagt | 40225 | - | see also 11488 line |
| 26440 | SMYD1 | NM_198274.1 | 957 | ggctcgttccgagggtttgtatc | 40250 | - | see also 11489 line |
| 26441 | SMYD1 | NM_198274.1 | 963 | ttccgagggtttgtatcatgagg | 40252 | - | see also 11489 line |
| 26442 | SNAI2 | NM_003068.3 | 697 | ttcggacccacacattacctgt | 40271 | - | see also 11491 line |
| 26443 | SNAI2 | NM_003068.3 | 473 | aagccccattagtgatgaagagg | 40266 | - | see also 11491 line |
| 26444 | SNAPC3 | NM_003084.1 | 314 | ggcggtgtgcggccttgataaac | 40275 | - | see also 2076 line |
| 26445 | SNAPC3 | NM_003084.1 | 1161 | ttccgaatgctgcactatgattc | 40297 | - | see also 2076 line |
| 26446 | SNX7 | NM_015976.2 | 640 | ttgaaccgaattgctgatcatcc | 40316 | - | see also 11493 line |
| 26447 | SNX7 | NM_015976.2 | 614 | cacgcaggaaggctttacataaa | 40314 | - | see also 11493 line |
| 26448 | SON | NM_003103.5 | 5400 | ctcgaagtaagcgttccaaatct | 40443 | - | see also 2086 line |
| 26449 | SON | NM_003103.5 | 5436 | cacgcaagcgtaccagtgaatct | 40446 | - | see also 2086 line |
| 26450 | SOS1 | NM_005633.2 | 3569 | aagcctattcaccacagatattca | 40566 | - | see also 3904 line |
| 26451 | SOS1 | NM_005633.2 | 1833 | gaggcttacgttaccatatgtacg | 40520 | - | see also 3904 line |
| 26452 | SOX10 | NM_006941.3 | 1494 | cagccctcaggaccctattatgg | 40572 | - | see also 11496 line |
| 26453 | SOX11 | NM_003108.3 | 700 | gacggtcaagtgcgtgtttctgg | 40576 | - | see also 2091 line |
| 26454 | SOX17 | NM_022454.2 | 1405 | gacgccagctccggtatatta | 40587 | - | see also 11499 line |
| 26455 | SOX17 | NM_022454.2 | 1345 | gggcatgactccggtgtgaatct | 40586 | - | see also 11499 line |
| 26456 | SOX18 | NM_018419.2 | 941 | gccgctcgctggcctgtactacg | 40589 | - | see also 11500 line |
| 26457 | SOX18 | NM_018419.2 | 1223 | cgccagcagcgcgggtcattaca | 40591 | - | see also 11500 line |
| 26458 | SOX21 | NM_007084.1 | 515 | tgctcgacctggcctccaaatg | 40592 | transcription_regulator | see also 11501 line |
| 26459 | SOX3 | NM_005634.2 | 1104 | cccatgggctcggtagtgaagt | 40594 | - | see also 11501 line |
| 26460 | SOX30 | NM_007017.2 | 1397 | gaccagcactagccaaagctaac | 40604 | - | see also 4893 line |
| 26461 | SOX30 | NM_007017.2 | 1241 | ccctactggagccttctgtaaaa | 40600 | - | see also 4893 line |
| 26462 | SOX7 | NM_031439.2 | 1052 | ggggacatggatcgcaatgaatt | 40636 | - | see also 9029 line |
| 26463 | SOX7 | NM_031439.2 | 1206 | atgccacgccacgtactacaac | 40639 | - | see also 9029 line |
| 26464 | SOX9 | NM_000346.2 | 1547 | cccagcgaacgcacatcaagacg | 40643 | - | see also 288 line |
| 26465 | SP1 | NM_138473.2 | 1743 | gcctgccttcgtactagcaaat | 40670 | - | see also 9626 line |
| 26466 | SP1 | NM_138473.2 | 1742 | ggcctgccgttggctatagcaaa | 40669 | - | see also 9626 line |
| 26467 | SP100 | NM_003113.2 | 121 | cagccacgatttgcaaaggatgt | 40680 | - | see also 11505 line |
| 26468 | SP100 | NM_003113.2 | 1974 | accgcgggagcatcccaagaactg | 40713 | - | see also 11505 line |
| 26469 | SP110 | NM_004509.2 | 379 | atcccaatcggtgacgatttac | 40721 | - | see also 11506 line |
| 26470 | SP110 | NM_004509.2 | 1066 | aagatgactcaacttgtaactcc | 40732 | - | see also 11506 line |
| 26471 | SP2 | NM_003110.3 | 2130 | cccacctggtcacgaagaacttg | 40758 | - | see also 2092 line |
| 26472 | SP3 | NM_003111.1 | 2324 | agctgcgagatgatactttga | 40777 | - | see also 11508 line |
| 26473 | SP3 | NM_003111.1 | 2326 | ctgcgcgaatgatagttgatt | 40778 | - | see also 11508 line |
| 26474 | SP3 | NM_003111.1 | 1180 | tgcctgttacgatagatagtaca | 40768 | - | see also 11508 line |

RNA polymerase II
transcription factor

Figure 46

| | | | | | | | |
|---|---|---|---|---|---|---|---|
| 26475 | SP4 | NM_003112.2 | 1530 | atccgactcaggtgcttatcagg | 40806 | - | see also 2093 line |
| 26476 | SP4 | NM_003112.2 | 2324 | aggtttatgcggagtgatcatct | 40821 | - | see also 2093 line |
| 26477 | SP7 | NM_152860.1 | 167 | gacggcagcgtgcagcaaatttg | 40825 | - | see also 11509 line |
| 26478 | SP7 | NM_152860.1 | 572 | tactccatggtgggatatgcacc | 40830 | - | see also 11509 line |
| 26479 | SP8 | NM_182700.2 | 691 | cccgtacgagtcgtggtttaagc | 40843 | - | see also 11510 line |
| 26480 | SP8 | NM_182700.2 | 149 | gccgctacctgtaataagatagg | 40839 | - | see also 11510 line |
| 26481 | SP8 | NM_182700.2 | 879 | cgccgctcggaggctacaactcg | 40844 | - | see also 11510 line |
| 26482 | SPATA13 | NM_153023.1 | 1005 | ttcgtcagattgcgagtgaatca | 40847 | - | see also 11511 line |
| 26483 | SPATA13 | NM_153023.1 | 1243 | ttgcgcagctagccactattttt | 40848 | - | see also 11511 line |
| 26484 | SPATA13 | NM_153023.1 | 1244 | tgcgcagctagccactattttg | 40849 | - | see also 11511 line |
| 26485 | SPG3A | NM_015915.2 | 1586 | atcatagctagcctatgcaatat | 40907 | - | see also 6570 line |
| 26486 | SPG3A | NM_015915.2 | 1089 | gtcccgagagcctagatattaaa | 40892 | - | see also 6570 line |
| 26487 | SPO11 | NM_012444.2 | 538 | gtcgtcgacaatattatcaatga | 40929 | - | see also 11513 line |
| 26488 | SPO11 | NM_012444.2 | 534 | gactgtcgtcgacaatattatca | 40927 | - | see also 11513 line |
| 26489 | SRCAP | NM_006662.1 | 5905 | acccgggatgtccacatatatag | 41027 | - | see also 11514 line |
| 26490 | SRCAP | NM_006662.1 | 2475 | aggtcgtgtctctcgatatgagg | 40997 | - | see also 11514 line |
| 26491 | SREBF2 | NM_004599.2 | 1161 | ggcggacaacccataatatcatt | 41060 | - | see also 11515 line |
| 26492 | SREBF2 | NM_004599.2 | 2296 | accgagcacactggttgagatcc | 41082 | - | see also 11515 line |
| 26493 | SREBF2 | NM_004599.2 | 2224 | atccgcctgttccgatgtacaca | 41080 | - | see also 11515 line |
| 26494 | SRISNF2L | NM_015106.1 | 2665 | cccatcgtatgcgcagtattacc | 41159 | - | see also 6244 line |
| 26495 | SRISNF2L | NM_015106.1 | 708 | ttgccattgtgccggttaatact | 41109 | - | see also 6244 line |
| 26496 | SRY | NM_003140.1 | 166 | tgctatgttaagcgtattcaaca | 41178 | - | see also 11517 line |
| 26497 | SRY | NM_003140.1 | 638 | tacagggatgactgtacgaaagc | 41190 | - | see also 11517 line |
| 26498 | SSA1 | NM_003141.2 | 990 | gccaatccgtggctgatactttc | 41200 | - | see also 11518 line |
| 26499 | SSA1 | NM_003141.2 | 112 | cagcagcacgcttgacaatgatg | 41192 | - | see also 11518 line |
| 26500 | SSRP1 | NM_003146.2 | 889 | ctgactcctcgtggtcgttatga | 41216 | - | see also 2127 line |
| 26501 | SSRP1 | NM_003146.2 | 891 | gactcctcgtggtcgttatgaca | 41217 | - | see also 2127 line |
| 26502 | SUHW2 | NM_080764.2 | 1024 | ctcgcaaattgcagcctaaaagt | 41246 | - | see also 11520 line |
| 26503 | SUHW2 | NM_080764.2 | 1379 | ttcgttcccttcagatacctttc | 41259 | - | see also 11520 line |
| 26504 | SYCP1 | NM_003176.2 | 1764 | cactgcaacaagctttcactaga | 41299 | - | see also 11521 line |
| 26505 | SYCP1 | NM_003176.2 | 3070 | accgttgggctgtaattgctaaa | 41323 | - | see also 11521 line |
| 26506 | SYCP2 | NM_014258.2 | 1352 | gccaaaacgtcgctgcatatact | 41356 | - | see also 11522 line |
| 26507 | SYCP2 | NM_014258.2 | 1253 | ttgtattttgacgcatcactaga | 41349 | - | see also 11522 line |
| 26508 | TAF1 | NM_004606.2 | 2323 | ggccaattgctgcaagcatttga | 41476 | - | see also 11523 line |
| 26509 | TAF1 | NM_004606.2 | 819 | tggaaaggtgttacgttttctac | 41475 | - | see also 11523 line |
| 26510 | TAF11 | NM_005643.2 | 172 | ggctaccgacaccgatggaatcc | 41479 | - | see also 3916 line |
| 26511 | TAF11 | NM_005643.2 | 627 | cactacaacccaaacatatgagg | 41491 | - | see also 3916 line |

Figure 46

| 26512 | TAF12 | NM_005644.2 | 251 | ggctccatggccaatagtactgc | 41498 | - | see also 3917 line | | |
|-------|-------|-------------|-----|--------------------------|-------|---|--------------------|---|---|
| 26513 | TAF12 | NM_005644.2 | 572 | atccgaccctacaaaaaagcttg | 41506 | - | see also 3917 line | | |
| 26514 | TAF6 | NM_005641.2 | 768 | aagaatgtcgagccactctatgg | 41515 | - | see also 11526 line | | |
| 26515 | TAF6 | NM_005641.2 | 1302 | ctgccacggttcagtacctttat | 41521 | - | see also 11526 line | | |
| 26516 | TAF6L | NM_006473.2 | 1874 | ctcgcgctacgtgcagaaactgc | 41561 | - | see also 11527 line | | |
| 26517 | TAF6L | NM_006473.2 | 758 | ggcacggagcctatttcgtaatc | 41556 | - | see also 11527 line | | |
| 26518 | TAF9L | NM_015975.3 | 471 | taggctgaagtccttaattaaaa | 41584 | - | see also 11528 line | | |
| 26519 | TAL1 | NM_003189.1 | 1039 | gccggatgccttccctatgttca | 41586 | - | see also 11529 line | | |
| 26520 | TAL1 | NM_003189.1 | 1084 | gagaccttccccctatgagatgg | 41587 | - | see also 11529 line | | |
| 26521 | TBP | NM_003194.2 | 816 | agcggtttgctgcggtaatcatg | 41601 | - | see also 11531 line | | |
| 26522 | TBP | NM_003194.2 | 791 | gcccgaaacgccgaatataatcc | 41600 | - | see also 11531 line | | |
| 26523 | TBPL1 | NM_004865.2 | 721 | gtgctatcggataaaatctctaa | 41627 | - | see also 3383 line | | |
| 26524 | TBPL1 | NM_004865.2 | 722 | tgctatcggataaaatctctaag | 41628 | - | see also 3383 line | | |
| 26525 | TBRG1 | NM_032811.1 | 670 | accgaccgacctggctttcatga | 41632 | - | see also 11533 line | | |
| 26526 | TBRG1 | NM_032811.1 | 1275 | atcacagggtagcccaattcagt | 41650 | - | see also 11533 line | | |
| 26527 | TCF12 | NM_003205.2 | 1972 | cgcttacgcgtgcgggatattaa | 41688 | - | see also 2171 line | | |
| 26528 | TCF12 | NM_003205.2 | 1404 | gtctcgaatggaggatcgtttag | 41674 | - | see also 2171 line | | |
| 26529 | TCF12 | NM_003205.2 | 1117 | accagcagttcaccttacgttgc | 41669 | - | see also 2171 line | | |
| 26530 | TCF20 | NM_005650.1 | 3258 | agggctaaccggagttctttaca | 41756 | - | see also 3919 line | | |
| 26531 | TCF20 | NM_005650.1 | 200 | cggctcatcccggctagaagagt | 41696 | - | see also 3919 line | | |
| 26532 | TCF21 | NM_003206.2 | 318 | gtgacgggttgaaaatggattcg | 41816 | - | see also 11536 line | | |
| 26533 | TCF21 | NM_003206.2 | 316 | atgtgacgggttgaaaatggatt | 41815 | - | see also 11536 line | | |
| 26534 | TCF4 | NM_003199.1 | 1097 | gccaacgggacagacagtataat | 41833 | - | see also 2161 line | | |
| 26535 | TCF7 | NM_003202.1 | 92 | accgtctactccgccttcaatct | 41852 | - | see also 11538 line | | |
| 26536 | TCF7 | NM_003202.1 | 810 | accctggctcgcctaagaaatgc | 41856 | - | see also 11538 line | | |
| 26537 | TCF7 | NM_003202.1 | 214 | ctctctctacgaacatttcaaca | 41854 | - | see also 11538 line | | |
| 26538 | TCFL1 | NM_005997.1 | 421 | ctctgacagtcggaagtctatgc | 41862 | - | see also 11539 line | | |
| 26539 | TCFL1 | NM_005997.1 | 1104 | cccgagccttgcgccagaaaatt | 41878 | - | see also 11539 line | | |
| 26540 | TCFL5 | NM_006602.2 | 1483 | agcgtattttgcggtaaaactgg | 41897 | - | see also 4652 line | | |
| 26541 | TEF | NM_003216.2 | 346 | tcccatatgatggcgaatctttc | 41901 | - | transcription_regulator | translation regulator | - |
| 26542 | TEF | NM_003216.2 | 347 | cccatatgatggcgaatctttcc | 41902 | - | see also 26541 line | | |
| 26543 | TEF | NM_003216.2 | 336 | gacaagaccatcccatatgatgg | 41900 | - | see also 26541 line | | |
| 26544 | THAP11 | NM_020457.2 | 419 | ccgtctctgcagcgttcacttcc | 41911 | - | see also 11540 line | | |
| 26545 | THAP11 | NM_020457.2 | 962 | tactggctccgaccattcgtact | 41912 | - | see also 11540 line | | |
| 26546 | THAP6 | NM_144721.2 | 57 | ctcgctgcttgccaaattcgaag | 41914 | - | see also 11541 line | | |
| 26547 | THAP6 | NM_144721.2 | 355 | aaccgttccagccactaactaca | 41921 | - | see also 11541 line | | |

Figure 46

| | | | | | | | | |
|---|---|---|---|---|---|---|---|---|
| 26548 | THAP7 | NM_030573.1 | 802 | ctcccctcagcgtatatgtgc | 41944 | - | see also 8890 line | |
| 26549 | THAP7 | NM_030573.1 | 398 | ctggtgggaatcagrtggatatca | 41938 | - | see also 8890 line | |
| 26550 | THAP8 | NM_152658.1 | 864 | gccgcctccctacagaagaata | 41948 | - | - | |
| 26551 | THAP8 | NM_152658.1 | 1239 | acctgaggaatcccaaacttca | 41949 | - | see also 26550 line | |
| 26552 | THAP8 | NM_152658.1 | 611 | gccctgtgagcttcacaagttc | 41946 | - | see also 26550 line | |
| 26553 | TIAL1 | NM_003252.2 | 539 | gtcgtcaaatcgaaccaattgg | 41954 | - | see also 2195 line | |
| 26554 | TIAL1 | NM_003252.2 | 553 | accaattgggcactcgtaaacc | 41955 | - | see also 2195 line | |
| 26555 | TIF1 | NM_003852.2 | 1776 | tccagcccacgattactagtgc | 42005 | - | see also 2616 line | |
| 26556 | TIF1 | NM_003852.2 | 2044 | gtgtacaccccaattacgttca | 42021 | - | see also 2616 line | |
| 26557 | TIGD4 | NM_145720.2 | 1100 | tggtccgatgttacgtctaaaag | 42061 | - | see also 11545 line | |
| 26558 | TIGD4 | NM_145720.2 | 1066 | atcgaattgctcagtgtctaaat | 42059 | - | see also 11545 line | |
| 26559 | TIGD4 | NM_145720.2 | 1107 | atgttacgtctaaaagctaatga | 42063 | - | see also 11545 line | |
| 26560 | TIGD5 | NM_032862.2 | 545 | acggggacgagcagaattacagc | 42106 | - | see also 11546 line | |
| 26561 | TIGD5 | NM_032862.2 | 1122 | cgccttcaaacagcgtacaagc | 42108 | - | see also 11546 line | |
| 26562 | TIGD6 | NM_030953.2 | 1386 | caggcatcgtccctatggaattt | 42144 | - | see also 11547 line | |
| 26563 | TIGD6 | NM_030953.2 | 1388 | ggcatcgtccctatggaattgc | 42146 | - | see also 11547 line | |
| 26564 | TIGD7 | NM_033208.2 | 1603 | gaggtccgacattttcaacttaa | 42178 | - | see also 11548 line | |
| 26565 | TIGD7 | NM_033208.2 | 1795 | agctgcaaacggctgtatagatg | 42193 | - | see also 11548 line | |
| 26566 | TIMELESS | NM_003920.1 | 2914 | ggcccgaatagtggataaactct | 42257 | - | see also 2682 line | |
| 26567 | TIMELESS | NM_003920.1 | 1206 | accggctcatggatcagtaaag | 42225 | - | see also 2682 line | |
| 26568 | TMF1 | NM_007114.1 | 2304 | aagaccattgcttcgacaaatag | 42327 | - | see also 11550 line | |
| 26569 | TMF1 | NM_007114.1 | 3207 | tcgattagatctcgaagatgtaa | 42350 | - | see also 11550 line | |
| 26570 | TMX2 | NM_015959.1 | 636 | ttggacgctatactgatgttagt | 42367 | - | see also 11551 line | |
| 26571 | TMX2 | NM_015959.1 | 629 | gtggatgttgagcgctatactga | 42366 | - | see also 11551 line | |
| 26572 | TNFAIP3 | NM_006290.2 | 338 | agcttgtgcgctgaaaacgaac | 42377 | - | see also 4384 line | |
| 26573 | TNFAIP3 | NM_006290.2 | 815 | tcggctatgacagccatcatttt | 42387 | - | see also 4384 line | |
| 26574 | TNP1 | NM_003284.2 | 32 | gtcgaccagccgcaaattaaaga | 42403 | - | see also 11553 line | |
| 26575 | TNP2 | NM_005425.2 | 346 | cacccgccattgccaaaccttca | 42404 | - | - | DNA binding |
| 26576 | TNP2 | NM_005425.2 | 501 | caccaaagcgccacaaaaagact | 42407 | - | see also 26575 line | |
| 26577 | TNP2 | NM_005425.2 | 498 | caccaccaaagcgccacaaaaag | 42405 | - | see also 26575 line | |
| 26578 | TNXB | NM_019105.4 | 11614 | ccccgtgacctccaattcagtga | 42439 | - | see also 7710 line | |
| 26579 | TNXB | NM_019105.4 | 12598 | gaggactatgcccatggttttgg | 42441 | - | see also 7710 line | |
| 26580 | TOP1 | NM_003286.2 | 292 | ttccgattgaatgattctcataa | 42445 | - | see also 2222 line | |
| 26581 | TOP1 | NM_003286.2 | 1525 | atcatgcttaaccctagttcacg | 42474 | - | see also 2222 line | |
| 26582 | TOP1MT | NM_052963.1 | 229 | tcccgacggagtgcgtttcttct | 42478 | - | see also 11555 line | |
| 26583 | TOP1MT | NM_052963.1 | 772 | ttggaccgagagcgttcagaact | 42496 | - | see also 11555 line | |
| 26584 | TOP2A | NM_001067.2 | 1482 | cactgagtgtacgcttatcctga | 42551 | - | see also 653 line | |

**Figure 46**

| | | | | | | | |
|---|---|---|---|---|---|---|---|
| 26585 | TOP2A | NM_001067.2 | 2531 | gtccacgatacatcttacaatg | 42583 | - | see also 653 line |
| 26586 | TOP2B | NM_001068.2 | 3492 | agggcgagagtcaatgatctta | 42747 | - | see also 656 line |
| 26587 | TOP2B | NM_001068.2 | 3129 | aagttattacggtttacgtaagg | 42738 | - | see also 656 line |
| 26588 | TOP3A | NM_004618.2 | 1437 | cccaagcaatggaacaagtct | 42795 | - | see also 3135 line |
| 26589 | TOP3A | NM_004618.2 | 1520 | cagcgactgtacgagtttattgt | 42799 | - | see also 3135 line |
| 26590 | TOP3B | NM_003935.3 | 1528 | gacacggtgaagcggttgttagc | 42832 | - | see also 11559 line |
| 26591 | TOP3B | NM_003935.3 | 2063 | tcctggtgcacggctactataag | 42842 | - | see also 11559 line |
| 26592 | TOX | NM_014729.1 | 1623 | ctcttcaacccgactatcagact | 42878 | - | see also 5919 line |
| 26593 | TRERF1 | NM_018415.1 | 3709 | ttcaggggtgacgcagaactataa | 42913 | - | see also 7454 line |
| 26594 | TRERF1 | NM_018415.1 | 2915 | ccggaacacgcctgccagaaatca | 42904 | - | see also 7454 line |
| 26595 | TRIM26 | NM_003449.2 | 1029 | accattgctcgagtggttaaaa | 42921 | - | see also 11562 line |
| 26596 | TRIM26 | NM_003449.2 | 1030 | cccattgcgagtggttaaaaa | 42922 | - | see also 11562 line |
| 26597 | TRIM33 | NM_015906.2 | 1974 | tcccgtagtaatcggtaacacaca | 42975 | - | see also 11563 line |
| 26598 | TRIM33 | NM_015906.2 | 3159 | ggccgatgtccgtttgatcttca | 43005 | - | see also 11563 line |
| 26599 | TRIM33 | NM_015906.2 | 3045 | tgcttcgataccaaactactata | 42999 | - | see also 11563 line |
| 26600 | TRPV6 | NM_018646.2 | 2330 | gagacctgcgtgggataatcaac | 43023 | - | see also 11564 line |
| 26601 | TRPV6 | NM_018646.2 | 2328 | gagagacctgcgtgggataatca | 43022 | - | see also 11564 line |
| 26602 | TSG101 | NM_006292.2 | 819 | ctctgcggtcagtgacaaactga | 43046 | - | see also 4386 line |
| 26603 | TSG101 | NM_006292.2 | 308 | tgccttatagaggtaatacatac | 43030 | - | see also 4386 line |
| 26604 | TSN | NM_004622.2 | 813 | agcgctacgacggattgaaatat | 43073 | - | see also 11566 line |
| 26605 | TSN | NM_004622.2 | 814 | gcgctacgacggattgaaatatg | 43074 | - | see also 11566 line |
| 26606 | TSNAX | NM_005999.2 | 572 | aacacgatcattaattagtatgg | 43094 | - | see also 4185 line |
| 26607 | TSPYL3 | NM_178465.1 | 415 | ctgaaccttggtgccctaattgt | 43112 | - | see also 11568 line |
| 26608 | TSPYL3 | NM_178465.1 | 545 | tcgcccgtaggagagcttcatcatc | 43113 | - | see also 11568 line |
| 26609 | TSPYL4 | NM_021648.3 | 183 | tgcagcgcagaagtttcattatc | 43119 | - | see also 11569 line |
| 26610 | TSPYL4 | NM_021648.3 | 387 | atgagggcttgtcaaggaatat | 43121 | - | see also 11569 line |
| 26611 | TTF2 | NM_003594.3 | 75 | gtccgcgatggcccgaataaagg | 43125 | - | see also 11570 line |
| 26612 | TTF2 | NM_003594.3 | 78 | cgcgatggcccgaataaaggaa | 43126 | - | see also 11570 line |
| 26613 | TWIST2 | NM_057179.1 | 502 | acgagatgacaataagatgacc | 43202 | - | see also 9501 line |
| 26614 | UBE2V1 | NM_021988.3 | 447 | ttgaacctagaaaatttcactgc | 43203 | - | transcription_regulator |
| 26615 | UBTF | NM_014233.1 | 385 | aggaagttccgtacattgacaga | 43204 | - | see also 5550 line |
| 26616 | UBTF | NM_014233.1 | 2086 | gagtacatccaataaaacgtaa | 43215 | - | see also 5550 line |
| 26617 | UPLC1 | NM_017707.2 | 457 | gacggtcgacaggattccaaaaa | 43220 | - | see also 11572 line |
| 26618 | UPLC1 | NM_017707.2 | 458 | acggtcgacaggattccaaaaaa | 43221 | - | see also 11572 line |
| 26619 | UPLC1 | NM_017707.2 | 976 | aagaaaagtgacggaattcgaag | 43226 | - | see also 11572 line |
| 26620 | USF1 | NM_007122.2 | 455 | ctgagacgcactacttacttc | 43237 | - | see also 4956 line |

Figure 46

| | | | | | | |
|---|---|---|---|---|---|---|
| 26621 | USF1 | NM_007122.2 | 265 | tagtcttccgaactgagaatgg | - | see also 4956 line |
| 26622 | VAT1 | NM_006373.2 | 554 | tgctgccttgctcgtcaattaca | - | see also 11574 line |
| 26623 | VAT1 | NM_006373.2 | 558 | gccttgctcgtcaattacattac | - | see also 11574 line |
| 26624 | WBSCR14 | NM_032951.1 | 590 | accacaagtggcgcatctactac | - | see also 11575 line |
| 26625 | WBSCR14 | NM_032951.1 | 1153 | agcgccttcctgagttctgattt | - | see also 11575 line |
| 26626 | WDHD1 | NM_007086.1 | 2879 | ctgaacttagtcgaactacaaat | - | see also 4947 line |
| 26627 | WDHD1 | NM_007086.1 | 2880 | tgcacttagtcgaactacaaata | - | see also 4947 line |
| 26628 | WDTC1 | NM_015023.2 | 2246 | aggccgagtcgtggaagatatgg | - | see also 6189 line |
| 26629 | WDTC1 | NM_015023.2 | 1957 | agcgaagctacgactatcagttc | - | see also 6189 line |
| 26630 | WHSC1 | NM_007331.1 | 2318 | ttcagctcttgggtttagcaaaa | - | see also 11578 line |
| 26631 | WHSC1 | NM_007331.1 | 2323 | ctcttgggtttagcaaaagttca | - | see also 11578 line |
| 26632 | WHSC1L1 | NM_017778.2 | 1472 | gaggcgtgggttcatgaaaaac | - | see also 11579 line |
| 26633 | WHSC1L1 | NM_017778.2 | 2057 | aggggatgggaaatttatcgatc | - | see also 11579 line |
| 26634 | WRN | NM_000553.2 | 2706 | tacgtaactccagaatactgttc | - | see also 11580 line |
| 26635 | WRN | NM_000553.2 | 4758 | cccgtcaactcagatatgagtaa | - | see also 11580 line |
| 26636 | WRN | NM_000553.2 | 4358 | gagaccaactacggttgaaaacg | - | see also 11580 line |
| 26637 | WRNIP1 | NM_020135.2 | 1299 | agccgctgcgagtgattgttct | - | see also 11581 line |
| 26638 | WRNIP1 | NM_020135.2 | 1296 | ctgagccgctgcgagtgattgt | - | see also 11581 line |
| 26639 | YBX2 | NM_015982.1 | 515 | ggcgcagaagccactaatgtaac | - | see also 11582 line |
| 26640 | ZBED2 | NM_024508.1 | 215 | cactccaatgccccaacaacagg | - | - |
| 26641 | ZBED2 | NM_024508.1 | 246 | ttctctgaggcatggaatattt | - | see also 26640 line |
| 26642 | ZBED4 | NM_014838.1 | 1085 | tcccgtccccgatcgaataaca | - | see also 11584 line |
| 26643 | ZBED4 | NM_014838.1 | 1089 | gtccccgatcgaataaacagagg | - | see also 11584 line |
| 26644 | ZBTB2 | NM_020861.1 | 400 | ctcatcgaccggttcgattaga | - | see also 11585 line |
| 26645 | ZBTB2 | NM_020861.1 | 403 | atgacccggttgattagaaaca | - | see also 11585 line |
| 26646 | ZBTB2 | NM_020861.1 | 219 | tgcaatcggcgatgtatacttca | - | see also 11585 line |
| 26647 | ZBTB3 | NM_024784.2 | 135 | gagcgaaaagatctctacttg | - | see also 8746 line |
| 26648 | ZF | NM_021212.1 | 1004 | cgctacctacgggcagtcttagc | - | see also 11587 line |
| 26649 | ZF | NM_021212.1 | 1250 | aaggccgtcttctcttaaaat | - | see also 11587 line |
| 26650 | ZF | NM_021212.1 | 1252 | ggcgtcgtcttccttaaaatgt | - | see also 11587 line |
| 26651 | ZFD25 | NM_016220.2 | 1385 | accaacaccaaacctaattaac | - | see also 11588 line |
| 26652 | ZFP161 | NM_003409.2 | 285 | ttcttcggtcatagaaatagatt | - | see also 11589 line |
| 26653 | ZFP161 | NM_003409.2 | 692 | aggaagttcggaaggtcaattgc | - | see also 11589 line |
| 26654 | ZFP28 | NM_020828.1 | 968 | aggccagccgatctgtacatgaga | - | see also 11590 line |
| 26655 | ZFP28 | NM_020828.1 | 1186 | gagaacgtacatataacaaatct | - | see also 11590 line |
| 26656 | ZFP36 | NM_003407.1 | 488 | gacggaactcgtgtcacaagttct | - | see also 11591 line |
| 26657 | ZFP36 | NM_003407.1 | 464 | ggccaatcgccaccccaaataca | - | see also 11591 line |

Figure 46

| 26658 | ZFP64 | NM_018197.2 | 830 | gacatggagcggcatttaaaaat | 43966 | - | see also 7313 line |
|---|---|---|---|---|---|---|---|
| 26659 | ZFP64 | NM_018197.2 | 828 | aggacatggagcggcatttaaaa | 43965 | - | see also 7313 line |
| 26660 | ZFP64 | NM_018197.2 | 539 | cccagcacggtccagtttgtatc | 43957 | - | see also 7313 line |
| 26661 | ZFX | NM_003410.1 | 771 | gactaccgacgtagtttcagaag | 43988 | - | see also 11593 line |
| 26662 | ZFX | NM_003410.1 | 767 | ctctgactaccgacgtagtttca | 43986 | - | see also 11593 line |
| 26663 | ZFY | NM_003411.2 | 788 | tcctctgacgagtgacatagttt | 43994 | - | see also 11594 line |
| 26664 | ZFY | NM_003411.2 | 969 | gtgaccatcgatgcagaatcaga | 43997 | - | see also 11594 line |
| 26665 | ZIC2 | NM_007129.2 | 501 | gccgcacggctcgcagaatgtgc | 44001 | - | see also 11595 line |
| 26666 | ZIC2 | NM_007129.2 | 720 | cggtgccttttccgctatatgc | 44002 | - | see also 11595 line |
| 26667 | ZIC3 | NM_003413.2 | 1766 | gccagttcaggctatgaatcttc | 44008 | - | see also 2308 line |
| 26668 | ZIC4 | NM_032153.2 | 770 | cggaacaggccaaccacatttgc | 44016 | - | see also 11597 line |
| 26669 | ZIC4 | NM_032153.2 | 773 | aacaggccaaccacatttgcttc | 44017 | - | see also 11597 line |
| 26670 | ZIC5 | NM_033132.2 | 1774 | aagatccacaagcgtactcatac | 44028 | - | see also 9376 line |
| 26671 | ZIM3 | NM_052882.1 | 771 | ctgggcatagatgatgtatcttc | 44040 | - | see also 11599 line |
| 26672 | ZIM3 | NM_052882.1 | 743 | atgtgacggatctaagaaaatac | 44038 | - | see also 11599 line |
| 26673 | ZNF10 | NM_015394.4 | 1835 | gtgggacagcgcttgttaatacc | 44091 | - | see also 11600 line |
| 26674 | ZNF10 | NM_015394.4 | 1682 | tccggaagaatgacctcattaag | 44082 | - | see also 11600 line |
| 26675 | ZNF124 | NM_003431.1 | 830 | cccgttctcttaatagacataaa | 44101 | - | see also 11601 line |
| 26676 | ZNF124 | NM_003431.1 | 829 | tcccgttctcttaatagacataa | 44100 | - | see also 11601 line |
| 26677 | ZNF124 | NM_003431.1 | 701 | atggacattatggttgtacaata | 44097 | - | see also 11601 line |
| 26678 | ZNF136 | NM_003437.2 | 976 | cccaaccttacgaatacatgaaa | 44122 | - | see also 11602 line |
| 26679 | ZNF136 | NM_003437.2 | 1058 | ctcccctcccttcgactacatga | 44123 | - | see also 11602 line |
| 26680 | ZNF14 | NM_021030.2 | 1776 | ttcgttccagtcaaattcgattg | 44140 | - | see also 11603 line |
| 26681 | ZNF14 | NM_021030.2 | 1785 | gtcaaattcgattgcatgaaagg | 44141 | - | see also 11603 line |
| 26682 | ZNF141 | NM_003441.1 | 1377 | cacagatcggagtcaacataaga | 44144 | - | see also 11604 line |
| 26683 | ZNF141 | NM_003441.1 | 1463 | cgctcctgagtcaacataagaaa | 44146 | - | see also 11604 line |
| 26684 | ZNF143 | NM_003442.3 | 1118 | tgcggtcggtcctttacaacatc | 44176 | - | see also 11605 line |
| 26685 | ZNF143 | NM_003442.3 | 1753 | aggaacgcactctgttgctatgg | 44197 | - | see also 11605 line |
| 26686 | ZNF146 | NM_007145.1 | 1015 | tagccaaaagcagtatgtcatta | 44203 | - | see also 11606 line |
| 26687 | ZNF146 | NM_007145.1 | 1056 | ctggcgagaagcttttcgaatgt | 44207 | - | see also 11606 line |
| 26688 | ZNF148 | NM_021964.1 | 925 | tgcctttagaacgaactatcact | 44224 | - | see also 8219 line |
| 26689 | ZNF151 | NM_003443.1 | 2370 | gacagacgcggacttctatcagc | 44287 | - | see also 11607 line |
| 26690 | ZNF151 | NM_003443.1 | 1470 | cccacactgcgacaagaagttca | 44283 | - | see also 11607 line |
| 26691 | ZNF16 | NM_006958.2 | 240 | accccctgctgtagtgatactga | 44288 | - | see also 11608 line |
| 26692 | ZNF16 | NM_006958.2 | 1345 | gtcgggtctccaacctcattaag | 44299 | - | see also 11608 line |
| 26693 | ZNF160 | NM_033288.2 | 522 | accaagaacgccggaatgtgtca | 44305 | - | see also 11609 line |
| 26694 | ZNF160 | NM_033288.2 | 563 | tccctcctaaatgtacaatcaag | 44306 | - | see also 11609 line |

Figure 46

| | | | | | | | | | |
|---|---|---|---|---|---|---|---|---|---|
| 26695 | ZNF177 | NM_003451.1 | 1360 | agcacaagcactaacctataat | 44329 | - | see also 11610 line | | |
| 26696 | ZNF177 | NM_003451.1 | 1359 | tagcacaagcactaaccttataa | 44328 | - | see also 11610 line | | |
| 26697 | ZNF180 | NM_013256.1 | 1437 | gccgagctcgcaccttgtttcc | 44347 | - | see also 11611 line | | |
| 26698 | ZNF180 | NM_013256.1 | 1113 | gccatgtacaccctacatata | 44342 | - | see also 11611 line | | |
| 26699 | ZNF184 | NM_007149.1 | 301 | ctccaaggggacataatctact | 44353 | - | see also 4960 line | | |
| 26700 | ZNF184 | NM_007149.1 | 1483 | atccgtcatcatatgattcatac | 44366 | - | see also 4960 line | | |
| 26701 | ZNF195 | NM_007152.1 | 471 | atgcatcttctactagctatgt | 44375 | - | see also 11613 line | | |
| 26702 | ZNF195 | NM_007152.1 | 1200 | aacatggtacaaaggttttaacc | 44383 | - | see also 11613 line | | |
| 26703 | ZNF20 | NM_021143.1 | 920 | tagtgaaatccgtagacataaaa | 44389 | - | see also 11614 line | | |
| 26704 | ZNF20 | NM_021143.1 | 587 | ctcctttcaatcacatgataaag | 44387 | - | see also 11614 line | | |
| 26705 | ZNF208 | NM_007153.1 | 643 | accottacttattaataagagtgc | 44391 | - | transcription_regulator | zinc binding | - |
| 26706 | ZNF212 | NM_012256.2 | 952 | ggctgcccgaagcagaaatctca | 44392 | - | transcription_regulator | zinc binding | - |
| 26707 | ZNF212 | NM_012256.2 | 956 | gcccgaagcagaaatctcatagg | 44393 | - | see also 26706 line | | |
| 26708 | ZNF214 | NM_013249.1 | 1075 | aagctgtacggatgtgatgaagt | 44414 | - | see also 11615 line | | |
| 26709 | ZNF214 | NM_013249.1 | 1151 | tagggaggtacctttatagctgt | 44419 | - | see also 11615 line | | |
| 26710 | ZNF216 | NM_006007.1 | 572 | tgcctgtaactcagcaaatgaca | 44431 | - | see also 11616 line | | |
| 26711 | ZNF22 | NM_006963.1 | 185 | gtgggcatgactattcgatttg | 44435 | - | see also 11617 line | | |
| 26712 | ZNF22 | NM_006963.1 | 746 | gtggctggtctccgttaagtata | 44440 | - | see also 11617 line | | |
| 26713 | ZNF221 | NM_013359.1 | 816 | gtcacgtgcaacagaaaccttac | 44441 | - | see also 11618 line | | |
| 26714 | ZNF221 | NM_013359.1 | 1106 | gggaaaggcttccatagtagatc | 44443 | - | see also 11618 line | | |
| 26715 | ZNF222 | NM_013360.1 | 511 | ttccaggtgaaaattgtaaaca | 44448 | - | see also 11619 line | | |
| 26716 | ZNF222 | NM_013360.1 | 1296 | tagacatgcttctagtattttga | 44451 | - | see also 11619 line | | |
| 26717 | ZNF223 | NM_013361.2 | 1230 | ttcgtaggctggatttgtgtaag | 44456 | - | see also 11620 line | | |
| 26718 | ZNF223 | NM_013361.2 | 591 | ggcctcaagactctaccataaag | 44453 | - | see also 11620 line | | |
| 26719 | ZNF224 | NM_013398.1 | 1530 | tacacaaattcacaatgctattc | 44462 | - | see also 11621 line | | |
| 26720 | ZNF224 | NM_013398.1 | 2200 | ttgtacagaattcattcttcctaa | 44463 | - | see also 11621 line | | |
| 26721 | ZNF225 | NM_013362.1 | 1136 | agcttccgtagtagagcaaatct | 44468 | - | see also 11622 line | | |
| 26722 | ZNF225 | NM_013362.1 | 696 | agggtaggacgtgtaaaaagtcc | 44466 | - | see also 11622 line | | |
| 26723 | ZNF225 | NM_013362.1 | 1140 | tccgtagtagagcaaatcttaat | 44469 | - | see also 11622 line | | |
| 26724 | ZNF226 | NM_015919.2 | 349 | aacccgaagacagggaaatttag | 44476 | - | see also 11623 line | | |
| 26725 | ZNF228 | NM_013380.2 | 807 | atccatgtactsggtatagcaaaa | 44511 | - | see also 11624 line | | |
| 26726 | ZNF228 | NM_013380.2 | 309 | aagtaacagtaagctactttttcc | 44498 | - | see also 11624 line | | |
| 26727 | ZNF23 | NM_145911.1 | 1759 | agcgttcaatgttaatgcaaaat | 44554 | - | see also 11625 line | | |
| 26728 | ZNF23 | NM_145911.1 | 1758 | aagcgttcaatgttaatgcaaaa | 44553 | - | see also 11625 line | | |
| 26729 | ZNF230 | NM_006300.1 | 1503 | ggctgtacaccggtctttctgt | 44567 | - | see also 11626 line | | |

Figure 46

| | | | | | | | |
|---|---|---|---|---|---|---|---|
| 26730 | ZNF230 | NM_006300.1 | 637 | ttcacagaatgggaagtgtaaac | 44564 | - | see also 11626 line |
| 26731 | ZNF234 | NM_006630.1 | 1122 | aagaacttccgtcgtagatcagc | 44578 | - | see also 11627 line |
| 26732 | ZNF234 | NM_006630.1 | 2223 | agctggcgatcaaatcttgtaag | 44583 | - | see also 11627 line |
| 26733 | ZNF235 | NM_004234.3 | 1045 | aacgctattggtgtcatgaatgt | 44605 | - | see also 11628 line |
| 26734 | ZNF235 | NM_004234.3 | 561 | aactgtctagtgaatcacatagg | 44591 | - | see also 11628 line |
| 26735 | ZNF238 | NM_006352.2 | 714 | atctgaacagcgacattgttaca | 44609 | - | see also 4445 line |
| 26736 | ZNF238 | NM_006352.2 | 1416 | aagaaagtgtgagcactaataac | 44621 | - | see also 4445 line |
| 26737 | ZNF239 | NM_005674.1 | 1032 | ctggcctcgccattgttaaatgg | 44636 | - | see also 11629 line |
| 26738 | ZNF239 | NM_005674.1 | 2001 | cagcgggttcacaagaaagatcc | 44641 | - | see also 11629 line |
| 26739 | ZNF25 | NM_145011.2 | 346 | gacctatggagcattcatgatct | 44648 | - | see also 11630 line |
| 26740 | ZNF25 | NM_145011.2 | 115 | ttccaggacccgtgacattaaa | 44642 | - | see also 11630 line |
| 26741 | ZNF258 | NM_007167.1 | 394 | aagggccaaactgcatatcataa | 44655 | - | see also 11631 line |
| 26742 | ZNF258 | NM_007167.1 | 396 | gggccaaactgcatatcataaga | 44657 | - | see also 11631 line |
| 26743 | ZNF26 | NM_019591.2 | 519 | ctgatatcagtggggtatcatgg | 44696 | - | see also 11632 line |
| 26744 | ZNF26 | NM_019591.2 | 1932 | tggaattcagggcttcgtataca | 44711 | - | see also 11632 line |
| 26745 | ZNF261 | NM_005096.1 | 3258 | tagttgtaccgggctcaacatt | 44751 | - | see also 3559 line |
| 26746 | ZNF261 | NM_005096.1 | 4023 | gactcgcaacgatgttctacc | 44781 | - | see also 3559 line |
| 26747 | ZNF262 | NM_005095.1 | 1955 | atcatcggtgatgcaagtactca | 44814 | - | see also 11634 line |
| 26748 | ZNF262 | NM_005095.1 | 745 | atcgtgtatcacggcatacaagc | 44793 | - | see also 11634 line |
| 26749 | ZNF264 | NM_003417.1 | 1348 | ggctttctccgcactatgtgt | 44867 | - | see also 11635 line |
| 26750 | ZNF264 | NM_003417.1 | 933 | aggtaaagatcccatgattcagg | 44865 | - | see also 11635 line |
| 26751 | ZNF266 | NM_006631.2 | 1864 | accttgctgtcgtatacaaact | 44879 | - | see also 11636 line |
| 26752 | ZNF266 | NM_006631.2 | 2606 | taagcattcctccagtcttaata | 44887 | - | see also 11636 line |
| 26753 | ZNF267 | NM_003414.3 | 724 | taggcaggtctctactctaaata | 44898 | - | see also 11637 line |
| 26754 | ZNF267 | NM_003414.3 | 1629 | aagtctttagccgtagttcttgc | 44914 | - | see also 11637 line |
| 26755 | ZNF271 | NM_006629.3 | 1657 | tcgggttctgatctcattaacc | 44932 | - | see also 4686 line |
| 26756 | ZNF271 | NM_006629.3 | 1233 | tagtcgccgttcagatcttgtta | 44926 | - | see also 4686 line |
| 26757 | ZNF278 | NM_014323.2 | 1938 | atcacttgaacggacatatcaag | 44951 | - | see also 11639 line |
| 26758 | ZNF278 | NM_014323.2 | 1532 | atccttccatgcggtctatgtgg | 44946 | - | see also 11639 line |
| 26759 | ZNF282 | NM_003575.1 | 1056 | gccatccctacggaatccattac | 44963 | - | see also 11640 line |
| 26760 | ZNF282 | NM_003575.1 | 1101 | gacctcttgtccggattaaaca | 44964 | - | see also 11640 line |
| 26761 | ZNF286 | NM_020652.1 | 735 | cagactgacacggaatagtgtct | 44971 | - | see also 11641 line |
| 26762 | ZNF286 | NM_020652.1 | 741 | gacacggaatagtgtctatgact | 44973 | - | see also 11641 line |
| 26763 | ZNF288 | NM_015642.1 | 1036 | gcgcggagcgctcttttacagc | 44999 | - | see also 6532 line |
| 26764 | ZNF288 | NM_015642.1 | 1902 | cccggtctgtcgacctttactgc | 45004 | - | see also 6532 line |
| 26765 | ZNF289 | NM_032389.2 | 135 | cagcatcacgtacggtgttttct | 45014 | - | see also 11643 line |
| 26766 | ZNF289 | NM_032389.2 | 279 | tgccaatgcgacggcttttttc | 45017 | - | see also 11643 line |

Figure 46

| | | | | | | | |
|---|---|---|---|---|---|---|---|
| 26767 | ZNF289 | NM_032389.2 | 278 | atgccaatgcgaggctttttt | 45016 | - | see also 11643 line |
| 26768 | ZNF297 | NM_005453.3 | 1029 | tacgtgaagcgaggtgtaattg | 45046 | - | see also 3800 line |
| 26769 | ZNF297 | NM_005453.3 | 300 | ctctgcgatgtatctatcagagt | 45043 | - | see also 3800 line |
| 26770 | ZNF297B | NM_014007.1 | 1415 | atgcccgctccagaaattgttag | 45070 | - | see also 5452 line |
| 26771 | ZNF297B | NM_014007.1 | 2463 | accggcatgtgacttcttgtact | 45089 | - | see also 5452 line |
| 26772 | ZNF300 | NM_052860.1 | 863 | acctaccgagttgttattaagagc | 45102 | - | see also 11646 line |
| 26773 | ZNF300 | NM_052860.1 | 702 | caggcaggtcacatttgttaaca | 45098 | - | see also 11646 line |
| 26774 | ZNF302 | NM_018443.1 | 518 | ccccaaccagtaacaatggaaaa | 45112 | - | see also 11647 line |
| 26775 | ZNF302 | NM_018443.1 | 1430 | aagtttagaaatgcaggaaatcc | 45114 | - | see also 11647 line |
| 26776 | ZNF304 | NM_020657.1 | 2208 | gagctcacgagtgcaacagtttt | 45133 | - | see also 11648 line |
| 26777 | ZNF304 | NM_020657.1 | 836 | cagcaccagaccacttacaatag | 45128 | - | see also 11648 line |
| 26778 | ZNF317 | NM_020933.2 | 934 | tacgacggagaaaatgcatga | 45142 | - | see also 11649 line |
| 26779 | ZNF317 | NM_020933.2 | 1399 | ttccgctggaagtccaacttaa | 45144 | - | see also 11649 line |
| 26780 | ZNF32 | NM_006973.1 | 630 | ttcaggaatcagagtaaccttgc | 45164 | - | see also 11650 line |
| 26781 | ZNF32 | NM_006973.1 | 178 | atgtagccgttctttcaatgtgatg | 45158 | - | see also 11650 line |
| 26782 | ZNF325 | NM_016265.1 | 995 | aacgtctgttcagaatatatta | 45171 | - | see also 11651 line |
| 26783 | ZNF325 | NM_016265.1 | 1109 | aggagatcaagcttatgaattta | 45173 | - | see also 11651 line |
| 26784 | ZNF331 | NM_018555.3 | 1575 | aaggcctttactcgagtcaatta | 45197 | - | see also 11652 line |
| 26785 | ZNF331 | NM_018555.3 | 1577 | ggcctttactcgagtcaattacc | 45199 | - | see also 11652 line |
| 26786 | ZNF333 | NM_032433.1 | 1167 | tccgtagtgcggataaatcctat | 45212 | - | see also 11653 line |
| 26787 | ZNF333 | NM_032433.1 | 1636 | ccggacgagcactcatctgaacg | 45218 | - | see also 11653 line |
| 26788 | ZNF334 | NM_018102.3 | 1752 | ggcatgccgtcagcaaaaactca | 45249 | - | see also 11654 line |
| 26789 | ZNF334 | NM_018102.3 | 797 | ttgaaacaacgtttgactataa | 45234 | - | see also 11654 line |
| 26790 | ZNF335 | NM_022095.3 | 1078 | gaggataggcgactataatccagc | 45260 | - | see also 8267 line |
| 26791 | ZNF335 | NM_022095.3 | 1073 | tgaaggaaggcgactataat | 45258 | - | see also 8267 line |
| 26792 | ZNF336 | NM_022482.3 | 212 | atggtactaggactaatgtctac | 45282 | - | see also 8409 line |
| 26793 | ZNF336 | NM_022482.3 | 1733 | atgcgtgcggacggacattcacc | 45309 | - | see also 8409 line |
| 26794 | ZNF337 | NM_015655.2 | 362 | ggcccctgtgcaggaatatatgc | 45312 | - | see also 11657 line |
| 26795 | ZNF337 | NM_015655.2 | 1098 | gtgggcgaggctatactaataag | 45321 | - | see also 11657 line |
| 26796 | ZNF339 | NM_021220.2 | 540 | cccggtcgccagatcgaaaatca | 45347 | - | see also 11658 line |
| 26797 | ZNF339 | NM_021220.2 | 539 | gcccggtcgccagatcgaaaatc | 45346 | - | see also 11658 line |
| 26798 | ZNF339 | NM_021220.2 | 545 | tcgccagatcgaaaatcaagttc | 45349 | - | see also 11658 line |
| 26799 | ZNF341 | NM_032819.3 | 915 | ctgaagaaccgacgagctaaagc | 45363 | - | see also 11659 line |
| 26800 | ZNF341 | NM_032819.3 | 1143 | ggcaccgtgtctcgaaaactgt | 45365 | - | see also 11659 line |
| 26801 | ZNF342 | NM_145288.1 | 1117 | gccatcaccacggaacaaagaac | 45377 | - | transcription_regulator |
| 26802 | ZNF342 | NM_145288.1 | 1120 | atcaccacggaacaaagaactga | 45378 | - | see also 26801 line |

Figure 46

| 26803 | ZNF342 | NM_145288.1 | 1222 | gagttctgcgggaagcattttac | 45380 | - | see also 26801 line | | |
|---|---|---|---|---|---|---|---|---|---|
| 26804 | ZNF345 | NM_003419.2 | 1097 | agcagtggttcggctcttactcg | 45383 | - | see also 11660 line | | |
| 26805 | ZNF345 | NM_003419.2 | 1818 | aactctgcgaattggaaactata | 45387 | - | see also 11660 line | | |
| 26806 | ZNF345 | NM_003419.2 | 1820 | ctctgcgaattggaaactataaa | 45388 | - | see also 11660 line | | |
| 26807 | ZNF347 | NM_032584.1 | 277 | gagagccaagtacaaatagcagg | 45393 | - | see also 11661 line | | |
| 26808 | ZNF347 | NM_032584.1 | 482 | cacatggccttgagtatcaatgc | 45395 | - | see also 11661 line | | |
| 26809 | ZNF354A | NM_005649.2 | 1120 | agccgaaggtcaggcctttttat | 45414 | - | see also 11662 line | | |
| 26810 | ZNF354A | NM_005649.2 | 1037 | ctctcagtacatccctttataaa | 45413 | - | see also 11662 line | | |
| 26811 | ZNF358 | NM_018083.1 | 254 | acccctgattctcgatcctaaca | 45416 | - | transcription_regulator | - | - |
| 26812 | ZNF358 | NM_018083.1 | 225 | gacctcgatccagatgtgattgg | 45415 | - | see also 26811 line | | |
| 26813 | ZNF358 | NM_018083.1 | 603 | cgctccacgctgctgaaacatcg | 45417 | - | see also 26811 line | | |
| 26814 | ZNF366 | NM_152625.1 | 1830 | gacacgccacatgaaagtcaagc | 45421 | - | see also 11663 line | | |
| 26815 | ZNF366 | NM_152625.1 | 2328 | ttctgattacttatacttcaagc | 45422 | - | see also 11663 line | | |
| 26816 | ZNF382 | NM_032825.2 | 400 | ggcattctagacccctcatattc | 45426 | - | see also 11664 line | | |
| 26817 | ZNF382 | NM_032825.2 | 1156 | aaccctttatttgtatcgattgt | 45446 | - | see also 11664 line | | |
| 26818 | ZNF384 | NM_133476.2 | 642 | ggggtccgggtttggtaatcacg | 45453 | - | see also 11665 line | | |
| 26819 | ZNF384 | NM_133476.2 | 535 | agcgtccgttacccagaatatca | 45452 | - | see also 11665 line | | |
| 26820 | ZNF398 | NM_020781.2 | 1089 | gacgtgtacaaaagcacttatgc | 45473 | - | see also 7966 line | | |
| 26821 | ZNF398 | NM_020781.2 | 1984 | tccgcaagcaccacctaatgaaa | 45476 | - | see also 7966 line | | |
| 26822 | ZNF408 | NM_024741.1 | 373 | cccgagacatcctcgctttaaag | 45488 | - | see also 11667 line | | |
| 26823 | ZNF408 | NM_024741.1 | 376 | gagacatcctcgctttaaagagc | 45490 | - | see also 11667 line | | |
| 26824 | ZNF409 | NM_014894.1 | 1843 | ctcgtggagagagctacaactgt | 45507 | - | see also 11668 line | | |
| 26825 | ZNF409 | NM_014894.1 | 1208 | ctgcaaggccctcataagctttc | 45506 | - | see also 11668 line | | |
| 26826 | ZNF43 | NM_003423.1 | 956 | tgcccttcaatcatcactaaaca | 45520 | - | see also 11669 line | | |
| 26827 | ZNF43 | NM_003423.1 | 955 | ctgcccttcaatcatcactaaac | 45519 | - | see also 11669 line | | |
| 26828 | ZNF431 | NM_133473.1 | 540 | ctccgcaagtgtagatgagtata | 45525 | - | see also 11671 line | | |
| 26829 | ZNF431 | NM_133473.1 | 541 | tccgcaagtgtagatgagtataa | 45526 | - | see also 11671 line | | |
| 26830 | ZNF432 | NM_014650.1 | 1369 | agccgtatgatcgaacatcaacg | 45544 | - | see also 11672 line | | |
| 26831 | ZNF432 | NM_014650.1 | 1110 | aggcaagcgtaatctcattgtac | 45540 | - | see also 11672 line | | |
| 26832 | ZNF433 | NM_152602.1 | 324 | accccagaacatatatgtagagt | 45562 | - | see also 11673 line | | |
| 26833 | ZNF433 | NM_152602.1 | 338 | atgtagagtacgaaaatctaagg | 45563 | - | see also 11673 line | | |
| 26834 | ZNF436 | NM_030634.1 | 777 | cacaggcatccgctatcatatat | 45575 | - | see also 11674 line | | |
| 26835 | ZNF436 | NM_030634.1 | 781 | ggcatccgctatcatatatgttc | 45577 | - | see also 11674 line | | |
| 26836 | ZNF439 | NM_152262.1 | 1393 | gccccaaatcttcaattgcatgg | 45586 | - | transcription_regulator | DNA binding | - |
| 26837 | ZNF439 | NM_152262.1 | 1389 | atctgccccaaatcttcaattgc | 45585 | - | see also 26836 line | | |

Figure 46

| | | | | | | | |
|---|---|---|---|---|---|---|---|
| 26838 | ZNF44 | NM_016264.1 | 387 | accttaagccagattcgaatag | 45588 | - | see also 11675 line |
| 26839 | ZNF44 | NM_016264.1 | 691 | ttcgaagacatatggtagtaaaa | 45591 | - | see also 11675 line |
| 26840 | ZNF441 | NM_152355.1 | 1449 | ttcatttgttgcacttaccttca | 45600 | - | see also 11676 line |
| 26841 | ZNF441 | NM_152355.1 | 1200 | ttctctgattgcacaggtttg | 45599 | - | see also 11676 line |
| 26842 | ZNF442 | NM_030824.1 | 838 | gaggagcctaagatgtcatatca | 45605 | - | see also 11677 line |
| 26843 | ZNF442 | NM_030824.1 | 948 | gagaaatcatgggttgttcattc | 45608 | - | see also 11677 line |
| 26844 | ZNF443 | NM_005815.2 | 1723 | ttcggtcattatgataacttaaa | 45611 | - | apoptosis, transcription regulator |
| 26845 | ZNF450 | NM_014797.1 | 945 | gagatccgtcaaacttaaagatt | 45628 | - | see also 5992 line |
| 26846 | ZNF450 | NM_014797.1 | 1831 | ctcgtaaccgattctgtacataa | 45643 | - | see also 5992 line |
| 26847 | ZNF481 | NM_020924.2 | 1024 | tgcgtgtgcatgccggaattaaa | 45675 | - | see also 8031 line |
| 26848 | ZNF481 | NM_020924.2 | 1029 | gtgcatgccggaattaaacctt | 45677 | - | see also 8031 line |
| 26849 | ZNF482 | NM_006626.3 | 1135 | tgggcatacggcccctttcagtgt | 45711 | - | see also 11680 line |
| 26850 | ZNF482 | NM_006626.3 | 764 | cagtacagctggtgtcgaaatg | 45702 | - | see also 11680 line |
| 26851 | ZNF490 | NM_020714.1 | 1181 | accggagttcaaccttatacatg | 45728 | - | see also 11681 line |
| 26852 | ZNF490 | NM_020714.1 | 723 | ctcgcagctcagtattcgaacc | 45720 | - | see also 11681 line |
| 26853 | ZNF510 | NM_014930.1 | 1360 | cacagagttcgccgaagaagtca | 45761 | - | see also 11682 line |
| 26854 | ZNF510 | NM_014930.1 | 1268 | aagagaacccattggtaagtaat | 45757 | - | see also 11682 line |
| 26855 | ZNF6 | NM_021998.2 | 961 | gtcgactctccacaatggtatc | 45777 | - | see also 8241 line |
| 26856 | ZNF6 | NM_021998.2 | 2407 | gaccttaagccggccacatcatatc | 45836 | - | see also 8241 line |
| 26857 | ZNF7 | NM_003416.1 | 2102 | gtgagccgtaaaaggttaatac | 45871 | - | see also 11684 line |
| 26858 | ZNF7 | NM_003416.1 | 476 | tccaagacagattctacgattag | 45854 | - | see also 11684 line |
| 26859 | ZNF71 | NM_021216.3 | 629 | gccgaagctcgtccctgataaag | 45885 | - | see also 11685 line |
| 26860 | ZNF71 | NM_021216.3 | 485 | ggctggacgacccacagaaaag | 45882 | - | see also 11685 line |
| 26861 | ZNF74 | NM_003426.1 | 1784 | cacgccaagtgagccagaaaagt | 45887 | - | see also 11686 line |
| 26862 | ZNF76 | NM_003427.3 | 1184 | accgagtactgagctgtataa | 45902 | - | see also 2314 line |
| 26863 | ZNF76 | NM_003427.3 | 736 | caccgctcatcacttaaaggtgc | 45895 | - | see also 2314 line |
| 26864 | ZNF79 | NM_007135.1 | 527 | agcagtgtgacggtagctttgc | 45912 | - | see also 11688 line |
| 26865 | ZNF79 | NM_007135.1 | 727 | ctctccaggctggaagattatat | 45913 | - | see also 11688 line |
| 26866 | ZNF8 | NM_021089.1 | 842 | tccagtcaggccattccaattac | 45921 | - | see also 11689 line |
| 26867 | ZNF8 | NM_021089.1 | 1267 | tcggaccacttgcctttcctgc | 45924 | - | see also 11689 line |
| 26868 | ZNF84 | NM_003428.1 | 863 | aggatactgataccttggttaaaa | 45934 | - | see also 11690 line |
| 26869 | ZNF84 | NM_003428.1 | 950 | atcgtttaggggagaaactctat | 45938 | - | see also 11690 line |
| 26870 | ZNFN1A1 | NM_006060.2 | 744 | gaggacgcactccgttggtaaac | 45958 | - | see also 11691 line |
| 26871 | ZNFN1A1 | NM_006060.2 | 972 | aagtaacgtcgccaaacgtaaga | 45963 | - | see also 11691 line |
| 26872 | ZNFN1A2 | NM_016260.1 | 1695 | aggaccgttatgagtttcatca | 45998 | - | see also 6775 line |
| 26873 | ZNFN1A2 | NM_016260.1 | 482 | atccgcttccgaatggtaaact | 45968 | - | see also 6775 line |

Figure 46

| 26874 | ZT86 | NM_015871.2 | 252 | ggcctgcgcgaggtacttcatcg | 46002 | - | see also 6547 line | | |
|---|---|---|---|---|---|---|---|---|---|
| 26875 | ZT86 | NM_015871.2 | 251 | tggcctgcgcgaggtacttcatc | 46001 | - | see also 6547 line | | |
| 26876 | BCAS2 | NM_005872.1 | 85 | gtggatgcgctgccgtattttga | 46008 | - | see also 11695 line | | |
| 26877 | BCAS2 | NM_005872.1 | 90 | tgcgctgccgtattttgatcaag | 46009 | - | see also 11695 line | | |
| 26878 | BCAS2 | NM_005872.1 | 270 | tgctcgacaaccaattgaattgc | 46016 | - | see also 11695 line | | |
| 26879 | C20orf14 | NM_012469.2 | 1116 | tacaagtagctcggaaccttatc | 46054 | - | see also 11696 line | | |
| 26880 | C20orf14 | NM_012469.2 | 2419 | tactcgagcacgggccattttgg | 46063 | - | see also 11696 line | | |
| 26881 | DDX23 | NM_004818.1 | 344 | accgtaaacgatccagcttatct | 46074 | - | see also 11697 line | | |
| 26882 | DDX23 | NM_004818.1 | 281 | aggagcgggatcggaataagaag | 46072 | - | see also 11697 line | | |
| 26883 | DHX38 | NM_014003.2 | 158 | gcctcgatccatcgattggaagg | 46106 | - | see also 11698 line | | |
| 26884 | DHX38 | NM_014003.2 | 844 | gccttcctatcgggattctgagc | 46116 | - | see also 11698 line | | |
| 26885 | DHX38 | NM_014003.2 | 1101 | ccgatcgggattggtacatgatg | 46121 | - | see also 11698 line | | |
| 26886 | DHX8 | NM_004941.1 | 2691 | agctcgtggtgacgcctatttct | 46215 | - | see also 11699 line | | |
| 26887 | DHX8 | NM_004941.1 | 2101 | cactcagtacgcgatcatcatgt | 46197 | - | see also 11699 line | | |
| 26888 | DIM1 | NM_006701.2 | 279 | agcatcgccgagaaggttaaaaa | 46240 | - | - | pre-mRNA splicing factor | - |
| 26889 | DIM1 | NM_006701.2 | 278 | cagcatcgccgagaaggttaaaa | 46239 | - | see also 26888 line | | |
| 26890 | DIM1 | NM_006701.2 | 282 | atcgccgagaaggttaaaaattt | 46241 | - | see also 26888 line | | |
| 26891 | FUSIP1 | NM_054016.1 | 575 | caccgaaatcgatctttttcaag | 46244 | - | see also 11700 line | | |
| 26892 | FUSIP1 | NM_054016.1 | 553 | ttccgacaatgatagattcaaac | 46242 | - | see also 11700 line | | |
| 26893 | FUSIP1 | NM_054016.1 | 554 | tccgacaatgatagattcaaaca | 46243 | - | see also 11700 line | | |
| 26894 | HPRP8BP | NM_004814.1 | 529 | gacgatggcacagttaagctttg | 46260 | - | see also 11701 line | | |
| 26895 | HPRP8BP | NM_004814.1 | 152 | aagcgggacctccaagatgttcc | 46251 | - | see also 11701 line | | |
| 26896 | KHSRP | NM_003685.1 | 1146 | tgctggcgtgcggatacagttca | 46284 | - | see also 11702 line | | |
| 26897 | KHSRP | NM_003685.1 | 1931 | ccggccagtcggactacactaag | 46297 | - | see also 11702 line | | |
| 26898 | LSM1 | NM_014462.1 | 253 | ttcgagatggaaggacacttata | 46301 | - | see also 11703 line | | |
| 26899 | LSM1 | NM_014462.1 | 345 | ggcaaaaaatacggtgatattcc | 46306 | - | see also 11703 line | | |
| 26900 | LSM10 | NM_032881.1 | 265 | gcccacggacgcatagacaatgt | 46313 | - | see also 11704 line | | |
| 26901 | LSM10 | NM_032881.1 | 264 | ggcccacggacgcatagacaatg | 46312 | - | see also 11704 line | | |
| 26902 | LSM11 | NM_173491.1 | 1016 | aggtattcactcgacacataaat | 46328 | - | see also 10071 line | | |
| 26903 | LSM11 | NM_173491.1 | 968 | gaggccagtcccgtaagaaaaag | 46326 | - | see also 10071 line | | |
| 26904 | LSM3 | NM_014463.1 | 141 | gagcttcgaggcagattacatgc | 46338 | - | see also 5694 line | | |
| 26905 | LSM3 | NM_014463.1 | 99 | agcctagatgagcgaatttatgt | 46334 | - | see also 5694 line | | |
| 26906 | LSM4 | NM_012321.2 | 450 | ggcgctggccgaggtgtgtttgg | 46345 | - | - | pre-mRNA splicing factor | - |
| 26907 | LSM5 | NM_012322.1 | 55 | gacaaatgtataggatcaagaat | 46346 | - | see also 11708 line | | |
| 26908 | LSM5 | NM_012322.1 | 72 | aagaattcacatcgtgatgaaga | 46348 | - | see also 11708 line | | |

Figure 46

| 26909 | LSM6 | NM_007080.1 | 131 | gacgaccagttgtggtaaaatta | 46350 | - | see also 11709 line |
|---|---|---|---|---|---|---|---|
| 26910 | LSM6 | NM_007080.1 | 99 | aacccctagtgacttcttaaagc | 46349 | - | see also 11709 line |
| 26911 | LSM6 | NM_007080.1 | 132 | acgaccagttgtggtaaaattaa | 46351 | - | see also 11709 line |
| 26912 | LSM8 | NM_016200.2 | 91 | accgaactgttgccgttattaca | 46357 | - | see also 6718 line |
| 26913 | LSM8 | NM_016200.2 | 90 | aaccgaactgttgccgttattac | 46356 | - | see also 6718 line |
| 26914 | MGC14151 | NM_032356.3 | 713 | ggcgctgctcaacaagactatgc | 46370 | - | see also 11712 line |
| 26915 | MGC14151 | NM_032356.3 | 712 | aggcgctgctcaacaagactatg | 46369 | - | see also 11712 line |
| 26916 | MPHOSPH 10 | NM_005791.1 | 1906 | accgcctgtaccagagattaaag | 46396 | - | see also 11713 line |
| 26917 | MPHOSPH 10 | NM_005791.1 | 1962 | aagtagccccagtgagtgttagt | 46399 | - | see also 11713 line |
| 26918 | PPIG | NM_004792.1 | 1164 | gaggaccaaggcgttatcgaact | 46434 | - | see also 11714 line |
| 26919 | PPIG | NM_004792.1 | 446 | gacgagagtttcgctgttaaaca | 46417 | - | see also 11714 line |
| 26920 | PRPF18 | NM_003675.2 | 660 | tggcgtttgggctaaagaattga | 46474 | - | see also 11715 line |
| 26921 | PRPF18 | NM_003675.2 | 994 | gacgaaactcagcggaaatatat | 46483 | - | see also 11715 line |
| 26922 | PRPF3 | NM_004698.1 | 212 | cagccgatcatctgaaacctttt | 46488 | - | see also 3220 line |
| 26923 | PRPF3 | NM_004698.1 | 1034 | agcgccagagacgcacttttaaa | 46501 | - | see also 3220 line |
| 26924 | PRPF4 | NM_004697.3 | 468 | gtcggtactgatgccttgaaaaa | 46538 | - | see also 11717 line |
| 26925 | PRPF4 | NM_004697.3 | 1237 | caggacgttgtatcatgttctta | 46555 | - | see also 11717 line |
| 26926 | PRPF8 | NM_006445.2 | 3099 | gacatgaaccatacgaattcata | 46623 | - | see also 4507 line |
| 26927 | PRPF8 | NM_006445.2 | 3763 | accgcgtgcgtcagattctcatg | 46639 | - | see also 4507 line |
| 26928 | SCA2 | NM_002973.1 | 3787 | aacacgcagtcgccacagaatag | 46768 | - | see also 2005 line |
| 26929 | SF3A1 | NM_005877.3 | 937 | ctcagatcgactggcatgatttt | 46789 | - | see also 11720 line |
| 26930 | SF3A1 | NM_005877.3 | 825 | aacccccgagaagttttggatca | 46787 | - | see also 11720 line |
| 26931 | SF3A1 | NM_005877.3 | 939 | cagatcgactggcatgattttgt | 46790 | - | see also 11720 line |
| 26932 | SF3A3 | NM_006802.1 | 1018 | agcgacatctcactcatgaaaat | 46827 | - | see also 4782 line |
| 26933 | SF3A3 | NM_006802.1 | 105 | ctccgggaccagatcaattctga | 46810 | - | see also 4782 line |
| 26934 | SF3B1 | NM_012433.1 | 2489 | accgacagttagttgatactact | 46911 | - | see also 5325 line |
| 26935 | SF3B1 | NM_012433.1 | 3501 | tgccgtaacaccgttacttgaag | 46937 | - | see also 5325 line |
| 26936 | SF3B1 | NM_012433.1 | 3502 | gccgtaacaccgttacttgaaga | 46938 | - | see also 5325 line |
| 26937 | SF3B4 | NM_005850.3 | 1149 | tcctatgcctccgcatggtatgc | 46975 | - | see also 11723 line |
| 26938 | SF3B4 | NM_005850.3 | 557 | accgtcctatcaccgtatcttat | 46973 | - | see also 11723 line |
| 26939 | SF3B4 | NM_005850.3 | 558 | ccgtcctatcaccgtatcttatg | 46974 | - | see also 11723 line |
| 26940 | SFRS1 | NM_006924.3 | 306 | tggtcgcgacggctatgattacg | 46979 | - | see also 4853 line |
| 26941 | SFRS10 | NM_004593.1 | 813 | atgatcgggactactatagcaga | 47010 | - | see also 11725 line |

Figure 46

| 26942 | SFRS10 | NM_004593.1 | 771 | gccgtcgggattactatgacaga | 47008 | - | see also 11725 line | | |
|---|---|---|---|---|---|---|---|---|---|
| 26943 | SFRS11 | NM_004768.2 | 948 | ggcggtcacattctaagtctagg | 47031 | - | see also 11726 line | | |
| 26944 | SFRS11 | NM_004768.2 | 941 | tcgagacggcggtcacattctaa | 47027 | - | see also 11726 line | | |
| 26945 | SFRS11 | NM_004768.2 | 945 | gacggcggtcacattctaagtct | 47029 | - | see also 11726 line | | |
| 26946 | SFRS2IP | NM_004719.1 | 3495 | accgtggcagaggcacttacaga | 47094 | - | see also 11727 line | | |
| 26947 | SFRS2IP | NM_004719.1 | 2327 | ctgaccgaacatcctagatctac | 47075 | - | see also 11727 line | | |
| 26948 | SFRS4 | NM_005626.3 | 1542 | cgccctcccgatctagatctagg | 47157 | - | see also 11728 line | | |
| 26949 | SFRS4 | NM_005626.3 | 1500 | cccgatctcggtccaagtctaga | 47155 | - | see also 11728 line | | |
| 26950 | SFRS6 | NM_006275.4 | 239 | tcctcgaagtagacctcaaaaat | 47158 | - | - | pre-mRNA splicing factor | - |
| 26951 | SFRS7 | NM_006276.36 | 275 | ctcccgagtgagggttgaactat | 47166 | - | see also 11729 line | | |
| 26952 | SFRS7 | NM_006276.36 | 308 | gcctcggagatcacgttttgata | 47168 | - | see also 11729 line | | |
| 26953 | SFRS7 | NM_006276.36 | 435 | agcaggtttcttcgtttgagtca | 47175 | - | see also 11729 line | | |
| 26954 | SFRS8 | NM_004592.2 | 593 | cacttacggtagcgactattacg | 47180 | - | see also 11730 line | | |
| 26955 | SFRS8 | NM_004592.2 | 2446 | cacgttctcccaagtaccattcg | 47213 | - | see also 11730 line | | |
| 26956 | SFRS9 | NM_003769.2 | 674 | aacttcctacatccgagtttatc | 47228 | - | see also 11731 line | | |
| 26957 | SFRS9 | NM_003769.2 | 748 | gccgtgactctccataccaaagc | 47229 | - | see also 11731 line | | |
| 26958 | SIP1 | NM_003616.1 | 218 | cccgaggacgcctcaggaatacc | 47235 | - | see also 2429 line | | |
| 26959 | SLU7 | NM_006425.3 | 1070 | ttgcatgggaagcctatgacaag | 47273 | - | see also 11733 line | | |
| 26960 | SLU7 | NM_006425.3 | 877 | aggattcgagaagatattgcaaa | 47270 | - | see also 11733 line | | |
| 26961 | SMNDC1 | NM_005871.2 | 736 | aacaacagagcctattctaaaaa | 47292 | - | see also 4086 line | | |
| 26962 | SMNDC1 | NM_005871.2 | 828 | aacctgtggaattgctgataaac | 47295 | - | see also 4086 line | | |
| 26963 | SNRP70 | NM_003089.3 | 544 | gtgtacggacctatcaaaagaat | 47305 | - | see also 11735 line | | |
| 26964 | SNRP70 | NM_003089.3 | 547 | tacggacctatcaaaagaataca | 47306 | - | see also 11735 line | | |
| 26965 | SNRP70 | NM_003089.3 | 487 | ttcgtggcgagagtgaattatga | 47302 | - | see also 11735 line | | |
| 26966 | SNRPB | NM_003091.3 | 386 | gagaatctggtctcaatgacagt | 47310 | - | see also 11736 line | | |
| 26967 | SNRPB | NM_003091.3 | 410 | gagggacctcctcccaaagatac | 47311 | - | see also 11736 line | | |
| 26968 | SNRPB2 | NM_003092.3 | 648 | ccctgattaccctccaaactata | 47323 | - | see also 11737 line | | |
| 26969 | SNRPB2 | NM_003092.3 | 854 | caccgtcccatgctatgaagatc | 47331 | - | see also 11737 line | | |
| 26970 | SNRPD1 | NM_006938.2 | 300 | acgctgagtattcgaggaaataa | 47338 | - | see also 11738 line | | |
| 26971 | SNRPD1 | NM_006938.2 | 291 | cagctggaaacgctgagtattcg | 47336 | - | see also 11738 line | | |
| 26972 | SNRPD2 | NM_004597.4 | 457 | gccgcgtgaaggccttcgatagg | 47343 | - | see also 11739 line | | |
| 26973 | SNRPD3 | NM_004175.3 | 503 | gacgaacaccggtgaggtatatc | 47346 | - | see also 2803 line | | |
| 26974 | SNRPF | NM_003095.1 | 123 | tacccctcaatcccaaacctttc | 47351 | - | see also 11741 line | | |

Figure 46

| | | | | | | | |
|---|---|---|---|---|---|---|---|
| 26975 | SNRPF | NM_003095.1 | 310 | gtgtaataatgtcctttatatca | 47353 | - | see also 11741 line |
| 26976 | SNRPN | NM_003097.3 | 493 | ctgcagcacattgactactagaat | 47355 | - | see also 11742 line |
| 26977 | SNRPN | NM_003097.3 | 715 | gggccacccccaaagatactgg | 47356 | - | see also 11742 line |
| 26978 | SRRM1 | NM_005839.1 | 1147 | ctcggaaaactcgtaggttatct | 47359 | - | see also 4067 line |
| 26979 | SRRM1 | NM_005839.1 | 1435 | tgcagcagagcgccaatacaga | 47369 | - | see also 4067 line |
| 26980 | TRA2A | NM_013293.2 | 847 | gttggcagcgtcgagattcttac | 47389 | - | see also 5388 line |
| 26981 | TRA2A | NM_013293.2 | 851 | cagacgtcgagattcttactatg | 47391 | - | see also 5388 line |
| 26982 | U2AF1 | NM_006758.1 | 369 | gggaacgtgtacgtcaagtttcg | 47407 | - | see also 11744 line |
| 26983 | U2AF1 | NM_006758.1 | 426 | ttgaataaccgttggtttaatgg | 47409 | - | see also 11744 line |
| 26984 | U2AF2 | NM_007279.1 | 213 | ggcgacgacgcagcaaacctttg | 47412 | - | see also 11745 line |
| 26985 | U2AF2 | NM_007279.1 | 255 | agcacggtggactgattcgttcc | 47414 | - | see also 11745 line |
| 26986 | U5-116KD | NM_004247.1 | 184 | gacgatgacgtaggagatcatga | 47426 | - | see also 2851 line |
| 26987 | U5-116KD | NM_004247.1 | 1790 | tccgaccctgaagttcaatacc | 47463 | - | see also 2851 line |
| 26988 | USP39 | NM_006590.1 | 663 | cacttacctgccgggtattgtgg | 47488 | - | see also 11747 line |
| 26989 | USP39 | NM_006590.1 | 1339 | accaagttgcctccatatctaat | 47509 | - | see also 11747 line |
| 26990 | USP39 | NM_006590.1 | 800 | gtcctccagggatatcatgttc | 47495 | - | see also 11747 line |
| 26991 | AUH | NM_001698.1 | 890 | gggatggaggtcgatttagtaac | 47529 | - | see also 11748 line |
| 26992 | AUH | NM_001698.1 | 487 | aagagcagtgattaacgatattg | 47518 | - | see also 11748 line |
| 26993 | CPSF1 | NM_013291.1 | 894 | tgccgtcaactcgcgttgtacc | 47535 | - | see also 11749 line |
| 26994 | CPSF1 | NM_013291.1 | 2842 | ttccgctacttcgaggatattta | 47543 | - | see also 11749 line |
| 26995 | CPSF1 | NM_013291.1 | 1885 | atcgggacaaccgctacattgt | 47541 | - | see also 11749 line |
| 26996 | ELAVL1 | NM_001419.2 | 224 | aacgaatttgatcgtcaactacc | 47554 | - | see also 993 line |
| 26997 | ELAVL1 | NM_001419.2 | 602 | gtccagagggttgcgtttatcc | 47558 | - | see also 993 line |
| 26998 | ELAVL2 | NM_004432.1 | 967 | atgtcagcggacttccaaaaaca | 47569 | - | see also 2964 line |
| 26999 | ELAVL3 | NM_001420.2 | 850 | agcgtttccggctggacaatttg | 47598 | - | see also 11751 line |
| 27000 | ELAVL3 | NM_001420.2 | 441 | accctcaacggcctcaaattaca | 47596 | - | see also 11751 line |
| 27001 | ELAVL3 | NM_001420.2 | 905 | gccgatcgccatcgatggtatga | 47599 | - | see also 11751 line |
| 27002 | ELAVL4 | NM_021952.2 | 680 | ctggcaataggcggccgtatcatca | 47608 | - | see also 8211 line |
| 27003 | ELAVL4 | NM_021952.2 | 824 | tgctacggaaccgattactgtga | 47612 | - | see also 8211 line |
| 27004 | ELAVL4 | NM_021952.2 | 704 | ctcacgaatcctggttgatcaag | 47609 | - | see also 8211 line |
| 27005 | PARN | NM_002582.1 | 1706 | atcactattaccgcaacaatagt | 47683 | - | see also 11753 line |
| 27006 | PARN | NM_002582.1 | 1564 | agctatccggatccaaacctatgc | 47681 | - | see also 11753 line |
| 27007 | PABPC3 | NM_030979.1 | 176 | ctccaactacggtatgtgaact | 47686 | - | see also 11754 line |
| 27008 | PABPC3 | NM_030979.1 | 1368 | cgcccaggtgctcctagagtacc | 47689 | - | see also 11754 line |
| 27009 | PABPC4 | NM_003819.2 | 913 | tccgggtcgccgcgatatgatc | 47691 | - | see also 11755 line |
| 27010 | PABPC4 | NM_003819.2 | 916 | gggtctgccgcgatatgatcacc | 47693 | - | see also 11755 line |
| 27011 | TIA1 | NM_022037.1 | 1110 | ggccagtatatgcctaatggttg | 47742 | - | see also 8243 line |

Figure 46

| 27012 | TIA1 | NM_022037.1 | 629 | tgctgaaaacgccattcaacaga | 47716 | - | see also 8243 line |
|---|---|---|---|---|---|---|---|
| 27013 | PCF11 | NM_015885.2 | 4412 | tagacgttggtactacagtttaa | 47863 | - | see also 11757 line |
| 27014 | PCF11 | NM_015885.2 | 4408 | ctcatagacgttggtactacagt | 47862 | - | see also 11757 line |
| 27015 | FMR1 | NM_002024.1 | 785 | ttcggagtctgcgcactaagttg | 47899 | - | see also 1297 line |
| 27016 | FMR1 | NM_002024.1 | 545 | ttgaacgtctaagatctgttaat | 47887 | - | see also 1297 line |
| 27017 | GRSF1 | NM_002092.1 | 1272 | atcggtcccacgttcatcatagg | 47974 | - | see also 1356 line |
| 27018 | GRSF1 | NM_002092.1 | 1278 | cccacgttcatcataggtatatt | 47977 | - | see also 1356 line |
| 27019 | RBM8A | NM_005105.2 | 345 | ctcgacaggcgaacaggatatct | 47986 | - | see also 11759 line |
| 27020 | RBM8A | NM_005105.2 | 366 | ctgaaggggtatactctagttga | 47988 | - | see also 11759 line |
| 27021 | SLBP | NM_006527.2 | 562 | tgcctacgatcgttatattaaag | 48004 | - | see also 11760 line |
| 27022 | SLBP | NM_006527.2 | 563 | gcctacgatcgttatattaaaga | 48005 | - | see also 11760 line |
| 27023 | SSB | NM_003142.2 | 979 | atggtaacctacaattaaggaac | 48037 | - | see also 2124 line |
| 27024 | SSB | NM_003142.2 | 282 | aaccgtctaacaacagactttaa | 48021 | - | see also 2124 line |
| 27025 | ADAR | NM_001111.2 | 1700 | atcagcgggctgttagaatatgc | 48072 | - | see also 695 line |
| 27026 | ADARB1 | NM_001112.1 | 931 | ctgacacgctcttcaatggtttt | 48119 | - | see also 11763 line |
| 27027 | ADARB1 | NM_001112.1 | 724 | tgcacgcgcctttgtttgtcatg | 48114 | - | see also 11763 line |
| 27028 | ADARB1 | NM_001112.1 | 2231 | ctccgctattgaggtcatcaacg | 48147 | - | see also 11763 line |
| 27029 | ADARB2 | NM_018702.1 | 1720 | atcgatattcgtgcggttaaaag | 48161 | - | see also 11764 line |
| 27030 | ADARB2 | NM_018702.1 | 1711 | ctcagagcgatcgatattcgtgc | 48160 | - | see also 11764 line |
| 27031 | ADARB2 | NM_018702.1 | 1707 | aggactcagagcgatcgatattc | 48159 | - | see also 11764 line |
| 27032 | CARF | NM_017632.1 | 427 | gggtgccgataccctcaaaaagt | 48170 | - | see also 11765 line |
| 27033 | CARF | NM_017632.1 | 699 | agggtcggccatcaaatcagaga | 48183 | - | see also 11765 line |
| 27034 | DHX30 | NM_014966.2 | 426 | gtctacgtgcacacaaatggacc | 48187 | - | see also 6154 line |
| 27035 | DHX30 | NM_014966.2 | 705 | ctgaatccagagagtattcgacc | 48193 | - | see also 6154 line |
| 27036 | DICER1 | NM_030621.2 | 1095 | gactgtcgtgccgtattggtagt | 48252 | - | see also 8898 line |
| 27037 | DICER1 | NM_030621.2 | 1810 | atcgatcctatgttcaatctaaa | 48283 | - | see also 8898 line |
| 27038 | DICER1 | NM_030621.2 | 3968 | tggtacgacagacactattcaag | 48369 | - | see also 8898 line |
| 27039 | FLJ20399 | NM_017803.3 | 1256 | ccctaagatgtgcctactagagt | 48447 | - | see also 11768 line |
| 27040 | FLJ20399 | NM_017803.3 | 250 | atggagcggacattgtttactgt | 48425 | - | see also 11768 line |
| 27041 | JAZ | NM_012279.2 | 270 | aggtttgctgcgccttgcttatt | 48454 | - | see also 5254 line |
| 27042 | JAZ | NM_012279.2 | 888 | ggcaacccattcagaaagactca | 48470 | - | see also 5254 line |
| 27043 | LOC161931 | NM_139174.2 | 1212 | ggccctgctgaggttcttgttcc | 48475 | - | see also 11769 line |
| 27044 | LOC161931 | NM_139174.2 | 780 | tgcgggcactgcgaatagcaaga | 48473 | - | see also 11769 line |
| 27045 | LRRFIP1 | NM_004735.1 | 1504 | aagcacagggcatagtttagaga | 48488 | - | see also 11770 line |
| 27046 | LRRFIP1 | NM_004735.1 | 1328 | tccaactgttggagtgacttaga | 48484 | - | see also 11770 line |

Figure 46

| 27047 | MRPL44 | NM_022915.2 | 988 | gacggccgtggaactattccaag | 48521 | - | see also 11771 line |
|---|---|---|---|---|---|---|---|
| 27048 | MRPL44 | NM_022915.2 | 59 | ggcgtccgggctggtaagattgc | 48496 | - | see also 11771 line |
| 27049 | PRKR | NM_002759.1 | 903 | ggccgctaaacttgcatatcttc | 48543 | - | see also 11772 line |
| 27050 | PRKR | NM_002759.1 | 1306 | aagacttacgttattaaacgtgt | 48558 | - | see also 11772 line |
| 27051 | PRKRA | NM_003690.3 | 223 | tacacgaatacggcatgaagacc | 48580 | - | see also 11773 line |
| 27052 | PRKRA | NM_003690.3 | 900 | agcgccaatggacaatatcaatg | 48600 | - | see also 11773 line |
| 27053 | RNASE3L | NM_013235.2 | 3116 | aactccctcgaggattaaccaca | 48687 | - | see also 11774 line |
| 27054 | RNASE3L | NM_013235.2 | 2495 | ctctgtccgtatcgatcaactgg | 48664 | - | see also 11774 line |
| 27055 | SLC4A1AP | NM_018158.1 | 647 | agccggactgcggtgattttagg | 48732 | - | see also 11775 line |
| 27056 | SLC4A1AP | NM_018158.1 | 646 | cagccggactgcggtgattttag | 48731 | - | see also 11775 line |
| 27057 | STAU | NM_004602.1 | 1596 | gccacggtaactgccatgatagc | 48774 | - | see also 3130 line |
| 27058 | STAU | NM_004602.1 | 1027 | aacgagtaaagcctagaatcaaa | 48768 | - | see also 3130 line |
| 27059 | STAU2 | NM_014393.1 | 1109 | ctcgacgtcgagaatttgtgatg | 48803 | - | see also 5657 line |
| 27060 | STAU2 | NM_014393.1 | 1107 | gcctcgacgtcgagaatttgtga | 48802 | - | see also 5657 line |
| 27061 | STAU2 | NM_014393.1 | 1033 | agccgcctggcgcaaattcaaca | 48798 | - | see also 5657 line |
| 27062 | STRBP | NM_018387.2 | 1752 | ttcacgtagcggtgaaggtattg | 48853 | - | see also 7432 line |
| 27063 | STRBP | NM_018387.2 | 2088 | gaggcgcaggtccaaataagaaa | 48865 | - | see also 7432 line |
| 27064 | TARBP2 | NM_004178.3 | 913 | cacgtcagctacctggatattga | 48887 | - | see also 11779 line |
| 27065 | TARBP2 | NM_004178.3 | 97 | caggagtatgggaccagaatagg | 48877 | - | see also 11779 line |
| 27066 | Tenr | NM_139243.1 | 345 | tacgcaagtaacgggtaattttc | 48893 | - | see also 9680 line |
| 27067 | Tenr | NM_139243.1 | 849 | ttctaatcgttcagaatacctga | 48907 | - | see also 9680 line |
| 27068 | TLR3 | NM_003265.2 | 925 | atctcactatgctcgatctttcc | 48966 | - | see also 11781 line |
| 27069 | TLR3 | NM_003265.2 | 1858 | ttgaactaaagatcatcgattta | 48999 | - | see also 11781 line |
| 27070 | HNRPH1 | NM_005520.1 | 1069 | gtcgagttcgcaactcatgaaga | 49038 | - | see also 3850 line |
| 27071 | AARS | NM_001605.1 | 139 | tccggcagcgatttatagatttc | 49043 | - | see also 11783 line |
| 27072 | AARS | NM_001605.1 | 1503 | ctggacatttacgctatcgaaga | 49064 | - | see also 11783 line |
| 27073 | CARS | NM_001751.3 | 499 | cacggatcccgataaaaagcaga | 49101 | - | see also 11784 line |
| 27074 | CARS | NM_001751.3 | 494 | gagaccacggatcccgataaaaa | 49100 | - | see also 11784 line |
| 27075 | CARS | NM_001751.3 | 784 | aacccgggttagtgagtatgtgc | 49103 | - | see also 11784 line |
| 27076 | FARS1 | NM_006567.1 | 714 | ggccgtgaagcttgtagagtttg | 49136 | - | see also 11785 line |
| 27077 | FARS1 | NM_006567.1 | 675 | ttctcgctctgcgcataaacaag | 49134 | - | see also 11785 line |
| 27078 | FARS1 | NM_006567.1 | 1159 | gacttagtccgaacaattggagg | 49156 | - | see also 11785 line |
| 27079 | MARS | NM_004990.2 | 209 | tagtgcaatctgccgatattttt | 49157 | - | see also 11786 line |
| 27080 | MARS | NM_004990.2 | 446 | atctctagccgacattgttttgt | 49161 | - | see also 11786 line |
| 27081 | MARS | NM_004990.2 | 211 | gtgcaatctgccgatattttttt | 49158 | - | see also 11786 line |
| 27082 | SCYE1 | NM_004757.1 | 506 | tcccgtctggatcttcgaattgg | 49210 | - | see also 11787 line |
| 27083 | SCYE1 | NM_004757.1 | 306 | tgcacgctaattctatggtttct | 49203 | - | see also 11787 line |

Figure 46

| 27084 | SLA/LP | NM_016955.1 | 567 | gggggctcgagttggtagaatag | 49234 | - | see also 6981 line | | |
|--------|--------|-------------|------|-------------------------|-------|---|--------------------|---|---|
| 27085 | SLA/LP | NM_016955.1 | 568 | ggggctcgagttggtagaataga | 49235 | - | see also 6981 line | | |
| 27086 | YARS | NM_003680.2 | 1384 | tccctgcacttggctattcaaaa | 49268 | - | see also 11789 line | | |
| 27087 | YARS | NM_003680.2 | 1400 | ttcaaaacgggtccatctgatga | 49269 | - | see also 11789 line | | |
| 27088 | RPL11 | NM_000975.2 | 195 | tcctttggcatccggagaaatga | 49287 | - | - | structural constituent of ribosome | - |
| 27089 | RPL11 | NM_000975.2 | 151 | cagggcagacccctgtgttttcc | 49286 | - | see also 27088 line | | |
| 27090 | RPL11 | NM_000975.2 | 23 | ggcgcaggatcaaggtgaaaagg | 49285 | - | see also 27088 line | | |
| 27091 | RPL5 | NM_000969.2 | 220 | ttcgtgtgacaaacagagatatc | 49288 | - | see also 11790 line | | |
| 27092 | RPL5 | NM_000969.2 | 225 | gtgacaaacagagatatcatttg | 49290 | - | see also 11790 line | | |
| 27093 | EIF4EL3 | NM_004846.1 | 608 | cacacttcggcgagtgcttaacc | 49309 | - | see also 3358 line | | |
| 27094 | EIF4G3 | NM_003760.2 | 3094 | gccacgtatggaccagtacttta | 49372 | - | see also 2573 line | | |
| 27095 | EIF4G3 | NM_003760.2 | 4794 | cactacaagcatcgatagtaaaa | 49424 | - | see also 2573 line | | |
| 27096 | NCBP2 | NM_007362.2 | 312 | aacgccatgcggtacataaatgg | 49431 | - | see also 11794 line | | |
| 27097 | NCBP2 | NM_007362.2 | 419 | gggccaggttcgggatgagtatc | 49434 | - | see also 11794 line | | |
| 27098 | RNUT1 | NM_005701.2 | 153 | cccgcctatcccagtacaagtcc | 49435 | - | see also 11795 line | | |
| 27099 | RNUT1 | NM_005701.2 | 684 | tccgattctactggatgcattca | 49448 | - | see also 11795 line | | |
| 27100 | RRP4 | NM_014285.4 | 249 | atcgtagtgggacgaatcacaga | 49457 | - | see also 5592 line | | |
| 27101 | RRP4 | NM_014285.4 | 101 | cactacggacacaggattcatgc | 49454 | - | see also 5592 line | | |
| 27102 | SRP19 | NM_003135.1 | 177 | aaggcgaatccccataagtaagg | 49477 | - | see also 2115 line | | |
| 27103 | SRP19 | NM_003135.1 | 375 | cccatcacgtaagtcagtaatgt | 49491 | - | see also 2115 line | | |
| 27104 | PTBP1 | NM_002819.3 | 1261 | cgcgtgaagatcctgttcaataa | 49503 | - | see also 11798 line | | |
| 27105 | PTBP1 | NM_002819.3 | 1260 | gcgcgtgaagatcctgttcaata | 49502 | - | see also 11798 line | | |
| 27106 | A2BP1 | NM_018723.2 | 1154 | cacggttcccgagcacacattaa | 49508 | - | see also 7576 line | | |
| 27107 | ACF | NM_014576.2 | 1500 | ctcgctccccagatattagaaga | 49571 | - | see also 11800 line | | |
| 27108 | ACF | NM_014576.2 | 259 | tccagcgcacaggatatagcttg | 49545 | - | see also 11800 line | | |
| 27109 | ACO1 | NM_002197.1 | 979 | ctgaccgagctacgattgctaac | 49599 | - | see also 1400 line | | |
| 27110 | ACO1 | NM_002197.1 | 203 | tgggcgcttaccattttcgatca | 49579 | - | see also 1400 line | | |
| 27111 | AD023 | NM_020679.2 | 858 | gccagctgcccacaagtattact | 49663 | - | see also 11802 line | | |
| 27112 | AD023 | NM_020679.2 | 856 | acgccagctgcccacaagtatta | 49661 | - | see also 11802 line | | |
| 27113 | ADAT1 | NM_012091.2 | 479 | aacttagacgagacctcattttt | 49671 | - | see also 11803 line | | |
| 27114 | ADAT1 | NM_012091.2 | 1587 | accggattatcaccagttcaagt | 49688 | - | see also 11803 line | | |
| 27115 | AKAP1 | NM_003488.2 | 2012 | cacttagtcggtcggctaattgg | 49706 | - | see also 11804 line | | |
| 27116 | AKAP1 | NM_003488.2 | 2541 | acgtggactacggcggatataag | 49711 | - | see also 11804 line | | |
| 27117 | ALK | NM_004304.3 | 4736 | atctcacgggcgagctactatag | 49749 | - | see also 2882 line | | |
| 27118 | ALK | NM_004304.3 | 4741 | cagggcgagctactatagaaagg | 49750 | - | see also 2882 line | | |
| 27119 | APOBEC1 | NM_001644.2 | 374 | atctacgtagctcggctttttg | 49773 | - | see also 11806 line | | |

Figure 46

| | | | | | | | |
|---|---|---|---|---|---|---|---|
| 27120 | APOBEC1 | NM_001644.2 | 315 | gggaatgctcccaggctattaga | 49771 | - | see also 11806 line |
| 27121 | APOBEC2 | NM_006789.1 | 442 | agcgtgtgtgaccgcattatca | 49783 | - | - |
| 27122 | APOBEC2 | NM_006789.1 | 391 | cccagccctgcggtacaatgtca | 49782 | - | see also 27121 line |
| 27123 | BAT1 | NM_004640.3 | 857 | atcctagccctggctcgaaataa | 49786 | - | see also 3154 line |
| 27124 | BAT1 | NM_004640.3 | 1385 | gacatcgagcgggtgaacattgc | 49801 | - | see also 3154 line |
| 27125 | BRUNOL4 | NM_020180.1 | 1209 | tcctcagccgcctccaatgatcc | 49812 | - | see also 7788 line |
| 27126 | BRUNOL4 | NM_020180.1 | 1178 | ctgctgcctatggtcagataagc | 49811 | - | see also 7788 line |
| 27127 | BRUNOL5 | NM_021938.2 | 1296 | tcctacccttcggcaatatcatt | 49814 | - | - |
| 27128 | BRUNOL5 | NM_021938.2 | 1299 | tacccttcggcaatatcatttcc | 49815 | - | see also 27127 line |
| 27129 | BRUNOL5 | NM_021938.2 | 320 | gccagggattccgccatcaaagc | 49813 | - | see also 27127 line |
| 27130 | C14orf21 | NM_174913.1 | 479 | accgatgcggggtccatgtatta | 49820 | - | see also 11809 line |
| 27131 | C14orf21 | NM_174913.1 | 1865 | ctcgaaatgtggccttgactacc | 49837 | - | see also 11809 line |
| 27132 | C15orf12 | NM_018285.1 | 549 | cggcacgtgctagagtacaatga | 49839 | - | see also 11810 line |
| 27133 | C1orf25 | NM_030934.3 | 1480 | gggtcactacggatgtaacatt | 49883 | - | see also 11811 line |
| 27134 | C1orf25 | NM_030934.3 | 1478 | ccggcgtcactacggatgttaaca | 49882 | - | see also 11811 line |
| 27135 | CGI-37 | NM_016101.2 | 276 | cacaacgaccgggtgtactatgt | 49906 | - | see also 11812 line |
| 27136 | CGI-37 | NM_016101.2 | 424 | ttgcaccttatgccaagtataaa | 49909 | - | see also 11812 line |
| 27137 | CGI-79 | NM_016024.1 | 850 | ctgaagggcggtagttatagaagt | 49918 | - | see also 11813 line |
| 27138 | CGI-79 | NM_016024.1 | 853 | aagggcgtagttatagaagtaga | 49919 | - | see also 11813 line |
| 27139 | CHERP | NM_006387.4 | 196 | ttcttttcggagggcgaattctac | 49930 | - | see also 11814 line |
| 27140 | CHERP | NM_006387.4 | 206 | aggcgaattctacagttactaca | 49934 | - | see also 11814 line |
| 27141 | CIRBP | NM_001280.1 | 246 | aacattgacgacgctaaggatgc | 49945 | - | see also 11815 line |
| 27142 | CIRBP | NM_001280.1 | 551 | cgggtcctacagagacagttacg | 49947 | - | see also 11815 line |
| 27143 | CIZ1 | NM_012127.1 | 2667 | gggcgtcaaccgcctccaaaacc | 49971 | - | see also 11816 line |
| 27144 | CIZ1 | NM_012127.1 | 566 | accaccccaatcgaaaggattc | 49953 | - | see also 11816 line |
| 27145 | CPSF2 | NM_017437.1 | 1197 | acctagcctaaagttgtacttg | 49989 | - | see also 7011 line |
| 27146 | CPSF2 | NM_017437.1 | 2419 | ctcttacgggaggggattcaagc | 50027 | - | see also 7011 line |
| 27147 | CPSF3 | NM_016207.2 | 113 | atgtatttctgagttcaaag | 50034 | - | see also 11818 line |
| 27148 | CPSF3 | NM_016207.2 | 1522 | ctccttgcgacctgtccaattat | 50088 | - | see also 11818 line |
| 27149 | CPSF5 | NM_007006.1 | 265 | caccggctaccccatgtgttact | 50107 | - | see also 11819 line |
| 27150 | CPSF5 | NM_007006.1 | 356 | aaggactaaaacgcttaatgaca | 50110 | - | see also 11819 line |
| 27151 | CPSF6 | NM_007007.1 | 1661 | gaccgagagcgcgaatatcgtca | 50146 | - | see also 11820 line |
| 27152 | CPSF6 | NM_007007.1 | 67 | cgcgggatgtcggcgaagagttca | 50119 | - | see also 11820 line |
| 27153 | CSL4 | NM_016046.2 | 566 | cccgagtacaaccgcgaattctg | 50163 | - | see also 6638 line |
| 27154 | CSL4 | NM_016046.2 | 326 | aagatgtccgagcaactgaaaaa | 50158 | - | see also 6638 line |
| 27155 | CSTF1 | NM_001324.1 | 1357 | gtcgttgggcgcacaacaatattg | 50206 | - | see also 11821 line |
| 27156 | CSTF1 | NM_001324.1 | 562 | tgctgatgcttcggataaagatac | 50178 | - | see also 11821 line |

Figure 46

| 27157 | CSTF2 | NM_001325.1 | 132 | ctgaggttggacctgttgttagt | 50208 | - | see also 874 line |
|---|---|---|---|---|---|---|---|
| 27158 | CSTF3 | NM_001326.1 | 2187 | aggcccaacgaggattcagatga | 50302 | - | see also 876 line |
| 27159 | CSTF3 | NM_001326.1 | 658 | gtgttaatccgatgatcaacatt | 50248 | - | see also 876 line |
| 27160 | CUGBP1 | NM_006560.2 | 1230 | tactcgggtatccagcaatatgc | 50326 | - | see also 4596 line |
| 27161 | CUGBP1 | NM_006560.2 | 801 | aaccttgctggtctaaatactct | 50319 | - | see also 4596 line |
| 27162 | CUGBP2 | NM_006561.1 | 625 | ttcacctatcgtggtgaagtttg | 50348 | - | see also 4600 line |
| 27163 | CUGBP2 | NM_006561.1 | 54 | tgctcgttcctgacagaattaac | 50335 | - | see also 4600 line |
| 27164 | DAZAP1 | NM_018959.2 | 408 | cacgctagatggccgaaacatcg | 50365 | - | see also 7608 line |
| 27165 | DAZAP1 | NM_018959.2 | 606 | cacggaggtagtcatgatctatg | 50372 | - | see also 7608 line |
| 27166 | DAZAP1 | NM_018959.2 | 293 | aagtcgtagattgtgttatcatg | 50363 | - | see also 7608 line |
| 27167 | DAZL | NM_001351.2 | 366 | tgcaaccagccaaggctatattt | 50381 | - | see also 11827 line |
| 27168 | DAZL | NM_001351.2 | 756 | cacaaccacgatgaatcctataa | 50386 | - | see also 11827 line |
| 27169 | DDX1 | NM_004939.1 | 1562 | ttcctacatgggttgacttaaaa | 50439 | - | see also 3438 line |
| 27170 | DDX1 | NM_004939.1 | 1563 | tcctacatgggttgacttaaaag | 50440 | - | see also 3438 line |
| 27171 | DDX10 | NM_004398.2 | 1643 | gagccaagccgataaagtaattg | 50517 | - | see also 11829 line |
| 27172 | DDX10 | NM_004398.2 | 1579 | ctgtggcaccacgcgtaagattt | 50512 | - | see also 11829 line |
| 27173 | DDX17 | NM_006386.3 | 1875 | agctatcgggatcgtagtgaaac | 50576 | - | see also 11830 line |
| 27174 | DDX17 | NM_006386.3 | 1768 | ctcgttaccggaccacttcttca | 50574 | - | see also 11830 line |
| 27175 | DDX18 | NM_006773.3 | 196 | ctgaccctatcggaaactcaaaa | 50595 | - | see also 11831 line |
| 27176 | DDX18 | NM_006773.3 | 924 | gactcaccacgtgcatacctatg | 50612 | - | see also 11831 line |
| 27177 | DDX19 | NM_007242.3 | 488 | gagcccccacagaacttaattgc | 50658 | - | see also 11832 line |
| 27178 | DDX19 | NM_007242.3 | 553 | ggccatgcttagccaagtagaac | 50659 | - | see also 11832 line |
| 27179 | DDX21 | NM_004728.1 | 2382 | agcggagtttcagtaaagcattt | 50709 | - | see also 3240 line |
| 27180 | DDX21 | NM_004728.1 | 1549 | tgctgcacgtgggttagacatcc | 50682 | - | see also 3240 line |
| 27181 | DDX21 | NM_004728.1 | 1938 | agcgctccttgatcaactcaaat | 50694 | - | see also 3240 line |
| 27182 | DDX24 | NM_020414.3 | 2284 | atccgtttagctcgacagattga | 50751 | - | see also 7867 line |
| 27183 | DDX24 | NM_020414.3 | 2091 | cccacgtacctcggagatttatg | 50746 | - | see also 7867 line |
| 27184 | DDX25 | NM_013264.2 | 782 | ttctcagatcatagtattcgtat | 50768 | - | see also 5382 line |
| 27185 | DDX25 | NM_013264.2 | 894 | ctgaccctaatgttatcaagtta | 50773 | - | see also 5382 line |
| 27186 | DDX41 | NM_016222.2 | 311 | aggctgaagcgcgcaaagagtct | 50789 | - | see also 6727 line |
| 27187 | DDX41 | NM_016222.2 | 915 | tgggggcatgtccgtgaaagagc | 50798 | - | see also 6727 line |
| 27188 | DDX43 | NM_018665.1 | 1276 | tcccggaagattgaatgatctgc | 50838 | - | see also 11837 line |
| 27189 | DDX43 | NM_018665.1 | 657 | gagatcggccattgatagattgg | 50821 | - | see also 11837 line |
| 27190 | DDX5 | NM_004396.2 | 1967 | agctgcacctatgattggttatc | 50923 | - | see also 11838 line |
| 27191 | DDX5 | NM_004396.2 | 1644 | gaccgtcgggacagatactctgc | 50906 | - | see also 11838 line |
| 27192 | DDX56 | NM_019082.1 | 709 | cccggttacccttaagttacagg | 50933 | - | see also 11839 line |
| 27193 | DDX56 | NM_019082.1 | 707 | aacccggttacccttaagttaca | 50932 | - | see also 11839 line |

Figure 46

| 27194 | DDX56 | NM_019082.1 | 1387 | ggccattcgggaggcaagattga | 50943 | - | see also 11839 line |
|---|---|---|---|---|---|---|---|
| 27195 | DDX6 | NM_004397.3 | 1261 | acccagtactacgcatatgtaac | 50968 | - | see also 11840 line |
| 27196 | DDX6 | NM_004397.3 | 1264 | cagtactacgcatatgtaactga | 50970 | - | see also 11840 line |
| 27197 | DHX34 | NM_014681.3 | 1189 | agcggcatctccacaacgatttc | 50998 | - | see also 11841 line |
| 27198 | DHX34 | NM_014681.3 | 3350 | agggctagaagtccagaacatgt | 51009 | - | see also 11841 line |
| 27199 | DIS3 | NM_014953.2 | 1174 | atccctcgaattcgcatagaaac | 51045 | - | see also 11842 line |
| 27200 | DIS3 | NM_014953.2 | 357 | acgcatccgagatgtgactaata | 51018 | - | see also 11842 line |
| 27201 | DKC1 | NM_001363.2 | 95 | ggcggatgcggaagtaattattt | 51106 | - | see also 11843 line |
| 27202 | DKC1 | NM_001363.2 | 974 | atctcataaacggctggttatga | 51135 | - | see also 11843 line |
| 27203 | DKC1 | NM_001363.2 | 1157 | ctctacctgcgaccatggtatag | 51142 | - | see also 11843 line |
| 27204 | DSCR1L2 | NM_013441.2 | 613 | agcaagagcgcgaatagaactcc | 51152 | - | see also 11844 line |
| 27205 | DSCR1L2 | NM_013441.2 | 610 | ggcagcaagagcgcgaatagaac | 51151 | - | see also 11844 line |
| 27206 | DUSP11 | NM_003584.1 | 158 | ggccggagacgcgactttcagg | 51158 | - | see also 11845 line |
| 27207 | DUSP11 | NM_003584.1 | 217 | ccccgaaaggtggaaagactatc | 51159 | - | see also 11845 line |
| 27208 | DUSP11 | NM_003584.1 | 403 | aacatatactcaacgctattata | 51166 | - | see also 11845 line |
| 27209 | EIF2S1 | NM_004094.3 | 246 | cagaaggcgtatccgttctatca | 51188 | - | see also 11846 line |
| 27210 | EIF2S1 | NM_004094.3 | 780 | agctcctcctcggtatgtaatga | 51207 | - | see also 11846 line |
| 27211 | EIF2S2 | NM_003908.2 | 278 | agccaactgaggacaaggatttg | 51215 | - | see also 2667 line |
| 27212 | EIF3S4 | NM_003755.1 | 181 | gaggtcatcaacggaaacataaa | 51219 | - | see also 11847 line |
| 27213 | EIF3S4 | NM_003755.1 | 509 | cccgctgcccctacaaggatacg | 51225 | - | see also 11847 line |
| 27214 | EIF3S9 | NM_003751.2 | 1383 | ctggatacgcttagcatctatga | 51248 | - | see also 11848 line |
| 27215 | EIF3S9 | NM_003751.2 | 991 | gtggagaccgcacttccatattc | 51236 | - | see also 11848 line |
| 27216 | EIF4G1 | NM_004953.2 | 2693 | gtcatcccgcatccgctttatgc | 51291 | - | see also 11850 line |
| 27217 | EIF4G1 | NM_004953.2 | 4022 | tgcattcgtcgctgaacagaagg | 51310 | - | see also 11850 line |
| 27218 | EIF4G1 | NM_004953.2 | 3118 | ctcgcccagctactagtactttg | 51299 | - | see also 11850 line |
| 27219 | EIF4G2 | NM_001418.1 | 1439 | tgccaggtagcggaattggtact | 51366 | - | see also 979 line |
| 27220 | EIF4G2 | NM_001418.1 | 1731 | gcctgctcagtcgttcctaatga | 51380 | - | see also 979 line |
| 27221 | ERAL1 | NM_005702.1 | 191 | gcctcggcttctcgcagtaatgg | 51423 | - | see also 11851 line |
| 27222 | ERAL1 | NM_005702.1 | 48 | ggcttgttcaatcggtgttaaga | 51422 | - | see also 11851 line |
| 27223 | ETF1 | NM_004730.1 | 925 | ttcaaccaagctattgagttatc | 51467 | - | see also 3242 line |
| 27224 | EWSR1 | NM_005243.1 | 631 | ctctacacagccgactagttatg | 51496 | - | see also 3682 line |
| 27225 | EWSR1 | NM_005243.1 | 499 | atctagcacagggggttacaacc | 51491 | - | see also 3682 line |
| 27226 | FBL | NM_001436.2 | 740 | cgcaatggtggatgtgatctttg | 51514 | - | see also 11853 line |
| 27227 | FBL | NM_001436.2 | 434 | ttcggaaggagatgacaaaattg | 51512 | - | see also 11853 line |
| 27228 | FLJ10379 | NM_018079.3 | 1082 | ctcagtctgctatcgtacattag | 51539 | - | see also 11854 line |
| 27229 | FLJ10379 | NM_018079.3 | 2992 | cccccgatctaggattactctgg | 51611 | - | see also 11854 line |
| 27230 | FLJ10379 | NM_018079.3 | 2354 | caggattatatccgaacgttttg | 51592 | - | see also 11854 line |

Figure 46

| 27231 | FLJ10581 | NM_018146.2 | 887 | ttccggatgcccattatcaataa | 51632 | - | see also 7281 line |
|-------|----------|-------------|-----|--------------------------|-------|---|---------------------|
| 27232 | FLJ10581 | NM_018146.2 | 373 | gtctgggcttcgctacgataaag | 51613 | - | see also 7281 line |
| 27233 | FLJ13842 | NM_024645.1 | 408 | gtggtggccgattcccattatca | 51674 | - | see also 8677 line |
| 27234 | FLJ13842 | NM_024645.1 | 546 | gtcgcatctccctatcaaagaag | 51680 | - | see also 8677 line |
| 27235 | FLJ20244 | NM_017722.2 | 173 | ctctagagcccggtttttcgagt | 51692 | - | see also 11856 line |
| 27236 | FLJ20244 | NM_017722.2 | 1436 | cccggacgtgcctctgtactaca | 51701 | - | see also 11856 line |
| 27237 | FLJ22419 | NM_024697.1 | 876 | aacgacgacaactgtggaaatcc | 51711 | - | see also 11857 line |
| 27238 | FLJ22419 | NM_024697.1 | 1401 | ggctccacgaattctaccaaacc | 51723 | - | see also 11857 line |
| 27239 | FLJ22419 | NM_024697.1 | 1395 | gccattggctccacgaattctac | 51722 | - | see also 11857 line |
| 27240 | FLJ25270 | NM_152520.2 | 490 | aagcggttattaaccatacattt | 51724 | - | see also 11858 line |
| 27241 | FLJ25270 | NM_152520.2 | 634 | aagcactagacgcaacgaaaaat | 51726 | - | see also 11858 line |
| 27242 | FLJ31121 | NM_144723.1 | 140 | gcctgtcaagcgagagcttttac | 51747 | - | see also 9744 line |
| 27243 | FXR1 | NM_005087.1 | 582 | aagtattcgtacgaagttgatgc | 51783 | - | see also 3550 line |
| 27244 | FXR1 | NM_005087.1 | 1141 | cagattggttctaggtcttatag | 51806 | - | see also 3550 line |
| 27245 | FXR2 | NM_004860.2 | 1482 | ctccatgcgactcgaacctatgg | 51849 | - | see also 3377 line |
| 27246 | FXR2 | NM_004860.2 | 1081 | cccgaagctaccttgagttttct | 51843 | - | see also 3377 line |
| 27247 | G3BP | NM_005754.2 | 975 | tgggataccacctcatgttgtta | 51866 | - | see also 11862 line |
| 27248 | G3BP | NM_005754.2 | 486 | gaggagattcatgcaaacgtttg | 51862 | - | see also 11862 line |
| 27249 | G3BP2 | NM_012297.2 | 1420 | cccggtggtccacgtggaattgt | 51896 | - | see also 5264 line |
| 27250 | G3BP2 | NM_012297.2 | 1124 | ttggtaacttgccacatgatatt | 51888 | - | see also 5264 line |
| 27251 | HDLBP | NM_005336.1 | 860 | ttcatcgctgggccgtataatag | 51907 | - | see also 3717 line |
| 27252 | HDLBP | NM_005336.1 | 863 | atcgctgggccgtataatagact | 51908 | - | see also 3717 line |
| 27253 | HNRPA0 | NM_006805.2 | 804 | tccccaaggaggatatctactcc | 51993 | - | see also 11865 line |
| 27254 | HNRPA0 | NM_006805.2 | 556 | cggtgcccacgccaaggttaaga | 51985 | - | see also 11865 line |
| 27255 | HNRPA2B1 | NM_002137.2 | 1063 | ttctaactacggtccaatgaaga | 52009 | - | see also 11866 line |
| 27256 | HNRPA2B1 | NM_002137.2 | 638 | atcgtattgcagaaataccatac | 51999 | - | see also 11866 line |
| 27257 | HNRPAB | NM_004499.2 | 501 | gaggtcgttgactgtacaataaa | 52016 | - | see also 3050 line |
| 27258 | HNRPAB | NM_004499.2 | 802 | gacgaggttttgtgtttatcacc | 52027 | - | see also 3050 line |
| 27259 | HNRPF | NM_004966.2 | 1220 | agcgaccgagaacgacatttaca | 52048 | - | see also 11868 line |
| 27260 | HNRPF | NM_004966.2 | 552 | agcatgggacaccggtacattga | 52043 | - | see also 11868 line |
| 27261 | HNRPH2 | NM_019597.2 | 141 | ctgctcagccgatgaagtgatgc | 52053 | - | see also 11869 line |
| 27262 | HNRPH2 | NM_019597.2 | 191 | atggcacatcaggtattcgtttc | 52054 | - | see also 11869 line |
| 27263 | HNRPH3 | NM_012207.1 | 444 | agcgaccgggaccatatgataga | 52066 | - | see also 11870 line |
| 27264 | HNRPH3 | NM_012207.1 | 713 | atgagagggttgccttttcgtgc | 52069 | - | see also 11870 line |
| 27265 | HNRPL | NM_001533.1 | 547 | ttcgatcaccacggatgttcttt | 52091 | - | see also 11871 line |

Figure 46

| 27266 | HNRPL | NM_001533.1 | 401 | tacgcagccgacaaccaaatata | 52081 | - | see also 11871 line |
|---|---|---|---|---|---|---|---|
| 27267 | HNRPL | NM_001533.1 | 382 | ggcttgcaacgcagtgaactacg | 52080 | - | see also 11871 line |
| 27268 | HNRPM | NM_005968.2 | 2395 | ttgacgttcgaattgatagaaac | 52136 | - | see also 4169 line |
| 27269 | HNRPM | NM_005968.2 | 2387 | ccgagagattgacgttcgaattg | 52134 | - | see also 4169 line |
| 27270 | HNRPR | NM_005826.2 | 686 | atccactgtccggtcagaataga | 52156 | - | see also 4062 line |
| 27271 | ILF2 | NM_004515.2 | 817 | gacctactaggccattatgctgt | 52199 | - | see also 11874 line |
| 27272 | ILF2 | NM_004515.2 | 619 | gtgccacccaatcttcgaaaact | 52191 | - | see also 11874 line |
| 27273 | ILF2 | NM_004515.2 | 998 | agcaggacatggtctgctataca | 52203 | - | see also 11874 line |
| 27274 | IMP-3 | NM_006547.2 | 1028 | tgcggcttgtaagtctattctgg | 52235 | - | see also 4588 line |
| 27275 | IMP-3 | NM_006547.2 | 1025 | ctctgcggcttgtaagtctattc | 52234 | - | see also 4588 line |
| 27276 | KHDRBS3 | NM_006558.1 | 1172 | cccccgacacaagagacttatgg | 52266 | - | see also 4595 line |
| 27277 | KHDRBS3 | NM_006558.1 | 1171 | accccccgacacaagagacttatg | 52265 | - | see also 4595 line |
| 27278 | KIAA0116 | NM_015004.2 | 329 | ccctctatcggatatttaacaat | 52279 | - | see also 11877 line |
| 27279 | KIAA0116 | NM_015004.2 | 325 | aacaccctctatcggatatttaa | 52277 | - | see also 11877 line |
| 27280 | KIAA0117 | NM_015014.1 | 699 | gggaacgctatccaaaaagttgg | 52298 | - | see also 11878 line |
| 27281 | KIAA0117 | NM_015014.1 | 693 | agcagagggaacgctatccaaaa | 52295 | - | see also 11878 line |
| 27282 | KIAA0427 | NM_014772.1 | 1333 | ctgcggcgaaggctaaaggaaaa | 52305 | - | see also 11879 line |
| 27283 | KIAA0427 | NM_014772.1 | 841 | ctgttccgcaggaggagaaatga | 52300 | - | see also 11879 line |
| 27284 | KIAA0682 | NM_014852.1 | 82 | gtcgcgactgatcgtgaagaatc | 52312 | - | see also 6053 line |
| 27285 | KIAA0682 | NM_014852.1 | 1552 | gggatcgtcgtcctacaagaaga | 52323 | - | see also 6053 line |
| 27286 | KIS | NM_144624.1 | 631 | acctcaaaccacgtaacatattg | 52343 | - | see also 9717 line |
| 27287 | KIS | NM_144624.1 | 982 | atcacctaagagaccttatcaaa | 52351 | - | see also 9717 line |
| 27288 | LGTN | NM_006893.1 | 1351 | aggtccgaacgatcgtcattaac | 52386 | - | see also 11881 line |
| 27289 | LGTN | NM_006893.1 | 1354 | tccgaacgatcgtcattaactac | 52387 | - | see also 11881 line |
| 27290 | LOC91801 | NM_138775.1 | 509 | accaggactcatggtagtagaag | 52406 | - | see also 11882 line |
| 27291 | LOC91801 | NM_138775.1 | 289 | tgctcccggttttagagaaatgt | 52404 | - | see also 11882 line |
| 27292 | MATR3 | NM_018834.3 | 1682 | aaccacaccagcgttagtatttg | 52450 | - | see also 7583 line |
| 27293 | MBNL1 | NM_021038.1 | 1497 | cacggccagacacggaatgtaaa | 52456 | - | see also 8071 line |
| 27294 | MBNL1 | NM_021038.1 | 2495 | ctgactagccacaagtatgttac | 52479 | - | see also 8071 line |
| 27295 | MCTS1 | NM_014060.1 | 414 | gtccgatgccatgaacatataga | 52488 | - | see also 5475 line |
| 27296 | MCTS1 | NM_014060.1 | 367 | aaccatggcttaatcaaatcatg | 52485 | - | see also 5475 line |
| 27297 | METTL3 | NM_019852.2 | 1664 | tggcactcgcaagattgagttat | 52529 | - | see also 7738 line |
| 27298 | MGC42174 | NM_152383.2 | 1471 | aagcgtctacttggttcaaaagg | 52558 | - | see also 9842 line |
| 27299 | MGC4562 | NM_133375.2 | 1705 | aacttaagcgttgttgatgatat | 52614 | - | see also 11887 line |
| 27300 | MGC4562 | NM_133375.2 | 2512 | gccaccgaggagcgttgcatatc | 52634 | - | see also 11887 line |
| 27301 | MOV10L1 | NM_018995.1 | 3348 | gaccgtacggtcaaatgaagata | 52728 | - | see also 11888 line |

Figure 46

| | | | | | | | |
|---|---|---|---|---|---|---|---|
| 27302 | MOV10L1 | NM_018995.1 | 3346 | tcgaccgtacggtcaaatgaaga | 52727 | - | see also 11888 line |
| 27303 | MRPL1 | NM_020236.2 | 906 | tgccaatctgcaagcagttatta | 52743 | - | see also 11889 line |
| 27304 | MRPL1 | NM_020236.2 | 872 | tagcaacattggatatgtcaagt | 52742 | - | see also 11889 line |
| 27305 | MRPL12 | NM_002949.2 | 533 | ggcgaagcccgtggacaaagtga | 52750 | - | see also 11890 line |
| 27306 | MRPL12 | NM_002949.2 | 494 | agcgaaagaacggacacatttca | 52749 | - | see also 11890 line |
| 27307 | MRPL23 | NM_021134.2 | 202 | aagggtggacctcaggaattacc | 52753 | - | see also 11891 line |
| 27308 | MRPL23 | NM_021134.2 | 267 | gggtgcagcatggctctaacaag | 52754 | - | see also 11891 line |
| 27309 | MRPL3 | NM_007208.2 | 848 | tgctacgcatggtcaaacgaaaa | 52773 | - | see also 11892 line |
| 27310 | MRPL3 | NM_007208.2 | 628 | ttcgtaaagctacatccatattg | 52765 | - | see also 11892 line |
| 27311 | MRPL3 | NM_007208.2 | 1086 | aaggatctcggtaaaaatctacc | 52780 | - | see also 11892 line |
| 27312 | MRPS28 | NM_014018.2 | 441 | gtccggttgcggctattagatct | 52794 | - | see also 11893 line |
| 27313 | MRPS28 | NM_014018.2 | 447 | ttgcggctattagatcttgaact | 52797 | - | see also 11893 line |
| 27314 | MSI1 | NM_002442.2 | 740 | acctacgccagccggagttatac | 52814 | - | see also 1576 line |
| 27315 | NHP2L1 | NM_005008.1 | 165 | acctcgttcagcagtcatgtaac | 52817 | - | see also 11894 line |
| 27316 | NHP2L1 | NM_005008.1 | 171 | ttcagcagtcatgtaactataag | 52818 | - | see also 11894 line |
| 27317 | NOL4 | NM_003787.1 | 1665 | gagcgtgccagaaaacgtatacg | 52835 | - | see also 11895 line |
| 27318 | NOL4 | NM_003787.1 | 1669 | gtgccagaaaacgtatacgtact | 52836 | - | see also 11895 line |
| 27319 | NOL4 | NM_003787.1 | 1673 | cagaaaacgtatacgtacttacc | 52837 | - | see also 11895 line |
| 27320 | NOL5A | NM_006392.2 | 1187 | tgcctcacgaatcgattgcttct | 52883 | - | see also 4485 line |
| 27321 | NOL5A | NM_006392.2 | 622 | gggagtggtacgggtatcacttt | 52870 | - | see also 4485 line |
| 27322 | NOPD1 | NM_182543.1 | 1308 | ggcggttaaacttgatatggtgg | 52917 | - | see also 11897 line |
| 27323 | NOPD1 | NM_182543.1 | 1047 | cagtgtactgccccgttacttat | 52904 | - | see also 11897 line |
| 27324 | NOVA1 | NM_002515.1 | 481 | ccccagaccaccgttaatccaga | 52933 | - | see also 11898 line |
| 27325 | NOVA1 | NM_002515.1 | 320 | agctgtctaagtccaaagatttt | 52932 | - | see also 11898 line |
| 27326 | NOVA2 | NM_002516.1 | 1443 | cacgcaagccgctcaatacctca | 52960 | - | - | - | small cell lung cancer |
| 27327 | NOVA2 | NM_002516.1 | 1440 | ggccacgcaagccgctcaatacc | 52959 | - | see also 27326 line |
| 27328 | NOVA2 | NM_002516.1 | 345 | tgccgagaaggtccgagaaatcc | 52958 | - | see also 27326 line |
| 27329 | NUFIP1 | NM_012345.1 | 1313 | atcacaactatcaaacgttattc | 52978 | - | see also 11899 line |
| 27330 | NUFIP1 | NM_012345.1 | 1084 | tgctcactaatgagtagctatgg | 52973 | - | see also 11899 line |
| 27331 | NUFIP1 | NM_012345.1 | 1318 | aactatcaaacgttattcgaacc | 52979 | - | see also 11899 line |
| 27332 | NXF2 | NM_017809.2 | 1078 | gtgaagacgaaatccgtattacc | 53030 | - | see also 11900 line |
| 27333 | NXF2 | NM_017809.2 | 1081 | aagacgaaatccgtattaccacg | 53031 | - | see also 11900 line |
| 27334 | NXF3 | NM_022052.1 | 263 | aagatacactccctatactattt | 53035 | - | see also 11901 line |
| 27335 | NXF3 | NM_022052.1 | 489 | cccttcgtcccagttgagtttca | 53041 | - | see also 11901 line |
| 27336 | OAS1 | NM_002534.1 | 862 | acggtcttggaattagtcataaa | 53066 | - | see also 11902 line |
| 27337 | OAS1 | NM_002534.1 | 863 | cggtcttggaattagtcataaac | 53067 | - | see also 11902 line |
| 27338 | OAS2 | NM_002535.1 | 1248 | atctcgtcgtgttccataactca | 53088 | - | see also 11903 line |

Figure 46

| 27339 | OAS2 | NM_002535.1 | 1250 | ctcgtcgtgttccataactcact | 53089 | - | see also 11903 line |
|---|---|---|---|---|---|---|---|
| 27340 | OAS2 | NM_002535.1 | 1761 | gtggagtgccggattttgacact | 53097 | - | see also 11903 line |
| 27341 | OAS3 | NM_006187.1 | 2349 | aggtgaacaaggccgttgatacc | 53119 | - | see also 11904 line |
| 27342 | OAS3 | NM_006187.1 | 3048 | accgccagctctgtatctactgg | 53126 | - | see also 11904 line |
| 27343 | OIP2 | NM_181503.1 | 131 | aactgtcaacatcggttcaatta | 53128 | - | see also 10233 line |
| 27344 | OIP2 | NM_181503.1 | 135 | gtcaacatcggttcaattagtac | 53130 | - | see also 10233 line |
| 27345 | OIP2 | NM_181503.1 | 450 | ctcgactacgatggaaacatttt | 53136 | - | see also 10233 line |
| 27346 | P14 | NM_016047.2 | 437 | cacctatcgggattcaatgtttg | 53159 | - | see also 6639 line |
| 27347 | P14 | NM_016047.2 | 438 | acctatcgggattcaatgtttgt | 53160 | - | see also 6639 line |
| 27348 | PABPC5 | NM_080832.1 | 1336 | ccccaggggtgcctatctatatt | 53183 | - | see also 9528 line |
| 27349 | PABPC5 | NM_080832.1 | 1335 | cccccaggggtgcctatctatat | 53182 | - | see also 9528 line |
| 27350 | PABPC5 | NM_080832.1 | 951 | aacaaccgccaggtgtatgttgg | 53172 | - | see also 9528 line |
| 27351 | PABPN1 | NM_004643.1 | 1870 | ttcagtcaaccgtgttaccatac | 53189 | - | see also 3163 line |
| 27352 | PABPN1 | NM_004643.1 | 1915 | tcccaaagggtttgcgtatatag | 53191 | - | see also 3163 line |
| 27353 | PAPOLA | NM_032632.2 | 318 | tgcgtacttacacagaaactaat | 53202 | - | see also 9264 line |
| 27354 | PAPOLA | NM_032632.2 | 311 | gactgactgcgtacttacacaga | 53200 | - | see also 9264 line |
| 27355 | PAPOLB | NM_020144.3 | 1499 | gtgattgggttagggctaaaaaa | 53246 | - | see also 11909 line |
| 27356 | PAPOLB | NM_020144.3 | 1230 | cacacgagattttgctaagtaag | 53245 | - | see also 11909 line |
| 27357 | PAPOLB | NM_020144.3 | 1159 | ctccacatacaacgtgtctattt | 53240 | - | see also 11909 line |
| 27358 | PAPOLG | NM_022894.2 | 1126 | tgggatcctcgggtaaatccatc | 53298 | - | see also 8496 line |
| 27359 | PAPOLG | NM_022894.2 | 2052 | taccgtagtaggacgaaatgtca | 53332 | - | see also 8496 line |
| 27360 | PMSCL1 | NM_005033.1 | 703 | agcgtgatcctgtaccattaagt | 53353 | - | see also 3518 line |
| 27361 | PMSCL2 | NM_002685.1 | 1031 | ttctactcggacggaagacttca | 53398 | - | see also 1748 line |
| 27362 | PMSCL2 | NM_002685.1 | 76 | cggcgaccagcgcaaccaaatcc | 53373 | - | see also 1748 line |
| 27363 | PNPT1 | NM_033109.2 | 1076 | tgcgatggtcgggatttgacttc | 53454 | - | see also 11913 line |
| 27364 | PNPT1 | NM_033109.2 | 2109 | ctgcggtactgcttcataacaca | 53503 | - | see also 11913 line |
| 27365 | POLR2G | NM_002696.1 | 121 | ctccctagagcacgaaatcctgc | 53508 | - | see also 11914 line |
| 27366 | POLR2G | NM_002696.1 | 402 | cagaaattgggcccatgtcttgc | 53512 | - | see also 11914 line |
| 27367 | POP4 | NM_006627.1 | 423 | cagatcttcacggggctattatt | 53530 | - | see also 11915 line |
| 27368 | POP4 | NM_006627.1 | 642 | aacggtctgcgaagaagttcaaa | 53541 | - | see also 11915 line |
| 27369 | PPIE | NM_006112.1 | 232 | gacgtacaattcgtgtcaatttg | 53549 | - | see also 4248 line |
| 27370 | PPIE | NM_006112.1 | 815 | caccgaaggcctagatgtcttgc | 53555 | - | see also 4248 line |
| 27371 | PPP1R8 | NM_002713.2 | 1059 | agcaggcgcatgcagaactttgc | 53557 | - | see also 11917 line |
| 27372 | PPP1R8 | NM_002713.2 | 1229 | gccgtcagcagtgaacatgaacc | 53558 | - | see also 11917 line |
| 27373 | PS1D | NM_016505.2 | 663 | ctgaccctacaaggaatccttct | 53597 | - | see also 11918 line |
| 27374 | PS1D | NM_016505.2 | 582 | aaccaggtgggactaaatactct | 53593 | - | see also 11918 line |
| 27375 | PSMA1 | NM_002786.2 | 595 | ttcccaatcagctcgtacttact | 53620 | - | see also 1814 line |

Figure 46

| | | | | | | | |
|---|---|---|---|---|---|---|---|
| 27376 | PSMA1 | NM_002786.2 | 366 | tggctcaggagtgtttggattcc | 53612 | - | see also 1814 line |
| 27377 | PSMA6 | NM_002791.1 | 129 | ccggtttgacgccacattacc | 53630 | - | see also 11920 line |
| 27378 | PSMA6 | NM_002791.1 | 168 | gtcggctcaccaagtagaatat | 53632 | - | see also 11920 line |
| 27379 | PUM1 | NM_014676.1 | 3502 | agcggaagatcgtcatgcataag | 53715 | - | see also 5858 line |
| 27380 | PUM1 | NM_014676.1 | 3288 | atccgaggcaatgtacttgtatt | 53712 | - | see also 5858 line |
| 27381 | PUM1 | NM_014676.1 | 2610 | aaccggtacccaatttacaact | 53690 | - | see also 5858 line |
| 27382 | PUM2 | NM_015317.1 | 2101 | cccggcttcgtattaataggtct | 53777 | - | see also 6328 line |
| 27383 | RAE1 | NM_003610.2 | 517 | cagtaaccaagcgatacagatcg | 53837 | - | see also 2422 line |
| 27384 | RAE1 | NM_003610.2 | 875 | atcgagggagagttgctattca | 53857 | - | see also 2422 line |
| 27385 | RALY | NM_007367.2 | 711 | tggtccggcgtgtcaaaactaac | 53878 | - | see also 11924 line |
| 27386 | RALY | NM_007367.2 | 710 | ttggtccgcgtgtcaaaactaa | 53877 | - | see also 11924 line |
| 27387 | RALY | NM_007367.2 | 716 | cggcgtgtcaaaactaaagtacc | 53879 | - | see also 11924 line |
| 27388 | RBM10 | NM_005676.3 | 1950 | gccagtcgtatccatcatgtca | 53891 | - | see also 3943 line |
| 27389 | RBM11 | NM_144770.1 | 814 | agccaaagttcgaaagtcaa | 53915 | - | see also 11926 line |
| 27390 | RBM11 | NM_144770.1 | 184 | caaccagaatcggtgtcttatgc | 53906 | - | see also 11926 line |
| 27391 | RBM11 | NM_144770.1 | 113 | cactaaccaaagtgactatatgc | 53904 | - | see also 11926 line |
| 27392 | RBM15 | NM_022768.3 | 1661 | tgccgaccgaacatcgttacc | 53950 | - | see also 11927 line |
| 27393 | RBM15 | NM_022768.3 | 136 | gtgcggttcgctgtggaaacg | 53916 | - | see also 11927 line |
| 27394 | RBM16 | NM_014892.1 | 1513 | cacggtcgtattcaagtgaaagg | 54007 | - | see also 6078 line |
| 27395 | RBM16 | NM_014892.1 | 3553 | ttcggtctggaaactatcgattt | 54052 | - | see also 6078 line |
| 27396 | RBM3 | NM_006743.2 | 474 | cagaaaccagggtgtttatgacc | 54068 | - | see also 11929 line |
| 27397 | RBM3 | NM_006743.2 | 89 | tcgtgggaggctcaactttaac | 54063 | - | see also 11929 line |
| 27398 | RBM7 | NM_016090.2 | 599 | caggtaaagtcagaatgaattc | 54079 | - | see also 6657 line |
| 27399 | RBM7 | NM_016090.2 | 626 | ccctacctagcagatagacatta | 54082 | - | see also 6657 line |
| 27400 | RBM9 | NM_014309.1 | 1209 | ttggcgctgtggcgagtttatac | 54100 | - | see also 11931 line |
| 27401 | RBM9 | NM_014309.1 | 1245 | gccgatttgcccctactgaagt | 54102 | - | see also 11931 line |
| 27402 | RBMS2 | NM_002898.1 | 433 | ctcaaacctcccactgtcaatgg | 54103 | - | see also 11932 line |
| 27403 | RBMS2 | NM_002898.1 | 870 | ctcctgrtcttcgtatcagaga | 54111 | - | see also 11932 line |
| 27404 | RBMS3 | NM_014483.1 | 1056 | gcctcatccgccatacgttatgc | 54136 | - | see also 5701 line |
| 27405 | RBMS3 | NM_014483.1 | 181 | cccagtacacttactactatcc | 54114 | - | see also 5701 line |
| 27406 | RBPMS | NM_006867.1 | 816 | atccgcttcgatcctgaaattcc | 54150 | - | see also 11934 line |
| 27407 | RBPMS | NM_006867.1 | 844 | cactacgactagagtttgctaag | 54151 | - | see also 11934 line |
| 27408 | RDBP | NM_002904.4 | 1216 | gacgttcacaaggaaaaaccttgt | 54156 | - | see also 11935 line |
| 27409 | RDBP | NM_002904.4 | 431 | agccgttcagaggagagcatatct | 54152 | - | see also 11935 line |
| 27410 | RENT1 | NM_002911.2 | 2453 | aaaccgataaaccgatgttcttc | 54186 | - | see also 1941 line |
| 27411 | RENT1 | NM_002911.2 | 2826 | agcaaggtatggcgtcatcattg | 54193 | - | see also 1941 line |
| 27412 | RENT1 | NM_002911.2 | 328 | ttcgagttcaccgactttactct | 54158 | - | see also 1941 line |

Figure 46

| | | | | | | | | | |
|---|---|---|---|---|---|---|---|---|---|
| 27413 | RNASE1 | NM_002933.3 | 517 | ctgtgaagggagcccatatgtgc | 54205 | - | see also 11937 line | | |
| 27414 | RNASE1 | NM_002933.3 | 256 | ctccacctactgtaaccaaatga | 54204 | - | see also 11937 line | | |
| 27415 | RNASEH1 | NM_002936.3 | 426 | gccgtatgcaaagcacatgaagc | 54207 | - | see also 11938 line | | |
| 27416 | RNASEH1 | NM_002936.3 | 582 | ggggccaggccatcctttaaatg | 54208 | - | see also 11938 line | | |
| 27417 | RNASEH2A | NM_006397.2 | 902 | cccgttcttcccaccgatatttc | 54218 | - | see also 11939 line | | |
| 27418 | RNASEH2A | NM_006397.2 | 425 | ctgggcttatacagtatgcattg | 54213 | - | see also 11939 line | | |
| 27419 | RNASEL | NM_021133.2 | 2055 | gacaagtggacgactaagattaa | 54257 | - | see also 11940 line | | |
| 27420 | RNASEL | NM_021133.2 | 1085 | gacagcgaggcggaattatgacc | 54241 | - | see also 11940 line | | |
| 27421 | RNASET2 | NM_003730.3 | 601 | atggactatggcccgataaaagt | 54269 | - | see also 11941 line | | |
| 27422 | RNASET2 | NM_003730.3 | 581 | tccggattactggacaatacatg | 54268 | - | see also 11941 line | | |
| 27423 | RNASET2 | NM_003730.3 | 689 | ggcatactggcctgacgtaattc | 54270 | - | see also 11941 line | | |
| 27424 | RNMT | NM_003799.1 | 923 | atgaaaaatcgtcgtgatagtga | 54297 | - | see also 11942 line | | |
| 27425 | RNMT | NM_003799.1 | 246 | aggcaaaagcgtcagtgaattct | 54278 | - | see also 11942 line | | |
| 27426 | RNMT | NM_003799.1 | 930 | atcgtcgtgatagtgaatatatt | 54298 | - | see also 11942 line | | |
| 27427 | RNPC1 | NM_017495.4 | 670 | gtcctcgccctacattgagtaca | 54311 | - | see also 11943 line | | |
| 27428 | RNPC2 | NM_004902.2 | 709 | gacgacgttcccgaagcaaaagt | 54324 | - | see also 3405 line | | |
| 27429 | RNPC2 | NM_004902.2 | 963 | gtgcctctagcaataggattaac | 54336 | - | see also 3405 line | | |
| 27430 | ROD1 | NM_005156.3 | 168 | atcttataacagtcggtttaaag | 54355 | - | see also 3623 line | | |
| 27431 | ROD1 | NM_005156.3 | 557 | aagactgacaatctacctaatca | 54368 | - | see also 3623 line | | |
| 27432 | RPL18 | NM_000979.2 | 282 | ggggaccataactgatgatgtgc | 54397 | - | see also 11945 line | | |
| 27433 | RPL18 | NM_000979.2 | 83 | agcccaagagccaggatatctac | 54395 | - | see also 11945 line | | |
| 27434 | RPL24 | NM_000986.2 | 57 | cagttttagcgggtacaagatct | 54399 | - | - | structural constituent of ribosome | - |
| 27435 | RPL24 | NM_000986.2 | 43 | aaggtcgagctgtgcagttttag | 54398 | - | see also 27434 line | | |
| 27436 | RPL24 | NM_000986.2 | 443 | aggcagcacctaagcaaaagatt | 54400 | - | see also 27434 line | | |
| 27437 | RPL28 | NM_000991.3 | 45 | gtctgcgcatctgcaatggatgg | 54401 | - | - | structural constituent of ribosome | - |
| 27438 | RPL30 | NM_000989.2 | 122 | ctccaactcgttatgaaaagtgg | 54402 | - | - | structural constituent of ribosome | - |
| 27439 | RPL37A | NM_000998.2 | 38 | ggccaaacgtaccaagaaagtcg | 54404 | - | see also 11946 line | | |
| 27440 | RPL37A | NM_000998.2 | 55 | aagtcgggatcgtcggtaaatac | 54405 | - | see also 11946 line | | |
| 27441 | RPL3L | NM_005061.2 | 1114 | gtcgccaagccgtggagaatatt | 54413 | - | see also 11948 line | | |
| 27442 | RPL3L | NM_005061.2 | 1115 | tcgccaagccgtggagaatattg | 54414 | - | see also 11948 line | | |
| 27443 | RPL8 | NM_000973.2 | 218 | ccgggatccgtatcggtttaaga | 54418 | - | see also 11950 line | | |
| 27444 | RPL8 | NM_000973.2 | 240 | aagcggacggagctgttcattgc | 54420 | - | see also 11950 line | | |

Figure 46

| | | | | | | | | | | |
|---|---|---|---|---|---|---|---|---|---|---|
| 27445 | RPLP2 | NM_001004.2 | 402 | tgggatttggcctttttgattaa | 54426 | - | - | structural constituent of ribosome | - |
| 27446 | RPN1 | NM_002950.2 | 741 | aagtctctcactggggtaatatt | 54440 | - | see also 11951 line | | |
| 27447 | RPN1 | NM_002950.2 | 1049 | agctatgagtacctctataattt | 54450 | - | see also 11951 line | | |
| 27448 | RPP14 | NM_007042.1 | 436 | gaccctgttaggatcctataaag | 54467 | - | see also 11952 line | | |
| 27449 | RPP14 | NM_007042.1 | 346 | acctttggacatcctaacctatg | 54463 | - | see also 11952 line | | |
| 27450 | RPS19 | NM_001022.2 | 72 | gcctggagttactgtaaaagacg | 54474 | - | - | structural constituent of ribosome | Diamond-Blackfan anemia、Blackfan-Diamond anemia |
| 27451 | RPS29 | NM_001032.2 | 112 | cacggtctgatccggaaatatgg | 54477 | - | see also 11954 line | | |
| 27452 | RPS29 | NM_001032.2 | 122 | tccggaaatatggcctcaatatg | 54478 | - | see also 11954 line | | |
| 27453 | RPS4Y | NM_001008.2 | 204 | atgtatgcaacgtttcatcaaaa | 54484 | - | see also 11956 line | | |
| 27454 | RPS4Y | NM_001008.2 | 208 | atgcaacgtttcatcaaaattga | 54485 | - | see also 11956 line | | |
| 27455 | RPS4Y2 | NM_138963.1 | 609 | ttcttgcgatgtggtacatgtga | 54491 | - | see also 11957 line | | |
| 27456 | RPS4Y2 | NM_138963.1 | 498 | tgggaagataaccagctttatca | 54490 | - | see also 11957 line | | |
| 27457 | RPS5 | NM_001009.2 | 333 | aagcatgccttcgagatcataca | 54495 | - | see also 11958 line | | |
| 27458 | RPS5 | NM_001009.2 | 605 | ctcgaactcctatgccattaaga | 54496 | - | see also 11958 line | | |
| 27459 | RPS6 | NM_001010.2 | 351 | tgcaaatctgagcgttctcaact | 54497 | - | - | structural constituent of ribosome | - |
| 27460 | RRP40 | NM_016042.1 | 293 | aagcggtatgttccagtaaaagg | 54499 | - | see also 11960 line | | |
| 27461 | RRP40 | NM_016042.1 | 644 | ctctatccactggagatagtatt | 54515 | - | see also 11960 line | | |
| 27462 | RRP41 | NM_019037.1 | 451 | cccacgctcccagattgatatct | 54522 | - | see also 11961 line | | |
| 27463 | RRP41 | NM_019037.1 | 310 | ggccctagtgaactgtcaatata | 54518 | - | see also 11961 line | | |
| 27464 | RRP46 | NM_020158.1 | 35 | gacgcatactgacgccaaaatcc | 54529 | - | see also 11962 line | | |
| 27465 | RRP46 | NM_020158.1 | 221 | cagcaaagagattttcaacaagg | 54530 | - | see also 11962 line | | |
| 27466 | RTCD1 | NM_003729.1 | 1206 | gacgccgctaaagatacttatat | 54569 | - | see also 11963 line | | |
| 27467 | RTCD1 | NM_003729.1 | 1131 | acgcaaaccgcgatacattttgc | 54565 | - | see also 11963 line | | |
| 27468 | SARS | NM_006513.2 | 425 | gtgacgcggagcggataaagttg | 54583 | - | see also 4553 line | | |
| 27469 | SARS | NM_006513.2 | 197 | gcgagtggcgacgatgtagattt | 54575 | - | see also 4553 line | | |
| 27470 | SBP2 | NM_024077.2 | 1986 | ttgtgttaccgacctactcaaag | 54629 | - | see also 11965 line | | |
| 27471 | SBP2 | NM_024077.2 | 2061 | gactaaacgtcgacttgtgttgg | 54634 | - | see also 11965 line | | |
| 27472 | SCA1 | NM_000332.1 | 1796 | ctcccaggtcgtcatgcaatacg | 54651 | - | see also 281 line | | |
| 27473 | SCA1 | NM_000332.1 | 2222 | ctcaccccacacggtcattcaga | 54652 | - | see also 281 line | | |
| 27474 | SF1 | NM_004630.1 | 777 | cagcaacacgtgtgagtgataaa | 54679 | - | see also 3145 line | | |
| 27475 | SF1 | NM_004630.1 | 732 | ttgcactcaatccggatttcaag | 54678 | - | see also 3145 line | | |
| 27476 | SF3A2 | NM_007165.3 | 529 | tgccacgtcaccgcttcatgtct | 54699 | - | see also 11968 line | | |
| 27477 | SF3A2 | NM_007165.3 | 824 | gccaccgctgcctgagtctttgc | 54700 | - | see also 11968 line | | |
| 27478 | SFRS14 | NM_014884.1 | 3101 | ggggtcgtcccatgtccaaaaag | 54776 | - | see also 11970 line | | |

Figure 46

| | | | | | | | |
|---|---|---|---|---|---|---|---|
| 27479 | SFRS14 | NM_014884.1 | 901 | cccaccgtgaatcgtattactcc | 54736 | - | see also 11970 line |
| 27480 | SFRS3 | NM_003017.3 | 289 | agctagatggaagaacactatgt | 54783 | - | see also 11971 line |
| 27481 | SFRS3 | NM_003017.3 | 107 | ttcctgtccattggactgtaagg | 54781 | - | see also 11971 line |
| 27482 | SKIV2L | NM_006929.3 | 610 | tggagcactcagcccgaaaatgg | 54785 | - | see also 4860 line |
| 27483 | SKIV2L | NM_006929.3 | 1926 | ttccatacaaaagggtactatgc | 54816 | - | see also 4860 line |
| 27484 | SKIV2L | NM_006929.3 | 1498 | tccgggacttccgaaacacattc | 54801 | - | see also 4860 line |
| 27485 | SNRPA | NM_004596.3 | 1147 | tccaccgaatcacatcttgttcc | 54839 | - | see also 11973 line |
| 27486 | SNRPA | NM_004596.3 | 1140 | ctgagaatccaccgaatcacatc | 54837 | - | see also 11973 line |
| 27487 | SNRPA1 | NM_003090.1 | 557 | tgcacagcttgcaaaggatattg | 54843 | - | see also 2080 line |
| 27488 | SNRPC | NM_003093.1 | 171 | aacaacggctgcatttcaacaag | 54845 | - | -                    RNA binding                    - |
| 27489 | SNRPC | NM_003093.1 | 180 | tgcatttcaacaaggaaagatac | 54846 | - | see also 27488 line |
| 27490 | SRP54 | NM_003136.2 | 1205 | tacgttgcgagacatgtatgagc | 54871 | - | see also 2116 line |
| 27491 | SRP54 | NM_003136.2 | 1016 | tgccacaaaaagtccgattattt | 54864 | - | see also 2116 line |
| 27492 | SRP68 | NM_014230.2 | 1303 | acctgatccgactctatgacatc | 54921 | - | see also 5548 line |
| 27493 | SRP68 | NM_014230.2 | 766 | tccgctattgtgcatataatatt | 54909 | - | see also 5548 line |
| 27494 | SSA2 | NM_004600.3 | 304 | atcgactacaccggttcttatgt | 54930 | - | see also 3127 line |
| 27495 | SSA2 | NM_004600.3 | 1038 | atgccgcttactgcattactaag | 54948 | - | see also 3127 line |
| 27496 | SUPV3L1 | NM_003171.2 | 1873 | atggttacgccgatacatcaaat | 55024 | - | see also 2149 line |
| 27497 | SUPV3L1 | NM_003171.2 | 1870 | tgcatggttacgccgatacatca | 55023 | - | see also 2149 line |
| 27498 | SURF6 | NM_006753.3 | 713 | aggcgcagcgcagaaaagagaag | 55048 | - | see also 11978 line |
| 27499 | SURF6 | NM_006753.3 | 426 | gcgacagcgactgcatgagaaga | 55045 | - | see also 11978 line |
| 27500 | SYNCRIP | NM_006372.3 | 1424 | cccaggcaaggcgtaggttaatg | 55073 | - | see also 4463 line |
| 27501 | SYNCRIP | NM_006372.3 | 1423 | gcccaggcaaggcgtaggttaat | 55072 | - | see also 4463 line |
| 27502 | SYNJ1 | NM_003895.1 | 1518 | aagctcgagcacttttaactact | 55137 | - | see also 11980 line |
| 27503 | SYNJ1 | NM_003895.1 | 1416 | ctgttacccgaacaattcagaat | 55131 | - | see also 11980 line |
| 27504 | SYNJ2 | NM_003898.1 | 1194 | gagaacgtcagtccacgttttca | 55232 | - | see also 2655 line |
| 27505 | SYNJ2 | NM_003898.1 | 2265 | aactaccgcattgatcttactta | 55252 | - | see also 2655 line |
| 27506 | TARBP1 | NM_005646.2 | 4314 | cagtcgtgtttccgacttagacc | 55385 | - | see also 11982 line |
| 27507 | TARBP1 | NM_005646.2 | 4411 | atcgacaaaccgaccaatttagg | 55387 | - | see also 11982 line |
| 27508 | TEP1 | NM_007110.3 | 1816 | cactgatgaggcggatactaact | 55418 | - | see also 4953 line |
| 27509 | TEP1 | NM_007110.3 | 7425 | aggaccctaatatcgataactca | 55503 | - | see also 4953 line |
| 27510 | TRNT1 | NM_016000.2 | 1130 | tactcgtgtatgtgaactactga | 55552 | - | see also 6602 line |
| 27511 | TRNT1 | NM_016000.2 | 499 | aacgcagagatctcactataaat | 55526 | - | see also 6602 line |
| 27512 | TTC14 | NM_133462.1 | 1650 | atctagggcatcctcaaatcaga | 55604 | - | see also 9581 line |
| 27513 | TTC14 | NM_133462.1 | 549 | gagagatatagcccacttagaaa | 55567 | - | see also 9581 line |
| 27514 | UPF2 | NM_015542.2 | 558 | gccgcctcgactcaagtttgaag | 55632 | - | see also 11986 line |
| 27515 | UPF2 | NM_015542.2 | 2297 | gcgagatacgtcacaatggtaga | 55676 | - | see also 11986 line |

Figure 46

| 27516 | WBP4 | NM_007187.3 | 611 | gaccgtttgggtagaaggtttaa | 55738 | - | see also 4986 line |
|---|---|---|---|---|---|---|---|
| 27517 | WBP4 | NM_007187.3 | 612 | accgtttgggtagaaggtttaag | 55739 | - | see also 4986 line |
| 27518 | WBP4 | NM_007187.3 | 177 | gacaataggcctagtgttgaatt | 55725 | - | see also 4986 line |
| 27519 | WIG1 | NM_022470.2 | 934 | tccccgctctcggcagagaattc | 55772 | - | see also 8391 line |
| 27520 | WIG1 | NM_022470.2 | 642 | ctccgcagatgggctcctttaag | 55762 | - | see also 8391 line |
| 27521 | XRN2 | NM_012255.2 | 1855 | accgtttaaaccactagaacaac | 55838 | - | see also 5224 line |
| 27522 | XRN2 | NM_012255.2 | 816 | atgaaccgaactttaccattatt | 55802 | - | see also 5224 line |
| 27523 | ZFR | NM_016107.3 | 3185 | accgcaaatgagccaacgtttta | 55921 | - | see also 6664 line |
| 27524 | ZFR | NM_016107.3 | 3147 | ttccgccagatacacaaagttct | 55919 | - | see also 6664 line |
| 27525 | ZNF318 | NM_014345.1 | 2629 | gagtctctgcgaggctcaattcc | 55973 | - | see also 5630 line |
| 27526 | ZNF318 | NM_014345.1 | 3676 | gagctaaagcgcaaacttagtga | 55994 | - | see also 5630 line |
| 27527 | ZNF385 | NM_015481.1 | 412 | cgccacgcccgacgagtcaaagg | 56072 | - | see also 6435 line |
| 27528 | ZNF385 | NM_015481.1 | 355 | atctgtcaaatccgcttcaattc | 56071 | - | see also 6435 line |
| 27529 | ZPR9 | NM_033414.2 | 867 | ccctatcacggactgcttatttt | 56084 | - | see also 9422 line |
| 27530 | BRF2 | NM_018310.2 | 845 | ttcccttgcccgattttgtaaat | 56114 | - | see also 11992 line |
| 27531 | BRF2 | NM_018310.2 | 846 | tcccttgcccgattttgtaaatt | 56115 | - | see also 11992 line |
| 27532 | BZW1 | NM_014670.1 | 947 | gccagttgtcatcggaatagtct | 56135 | - | see also 5848 line |
| 27533 | BZW1 | NM_014670.1 | 267 | taccgtcgatatgcagaaacact | 56128 | - | see also 5848 line |
| 27534 | BZW2 | NM_014038.1 | 328 | ggctcaagattagattatcgtcg | 56140 | - | see also 5468 line |
| 27535 | BZW2 | NM_014038.1 | 682 | accgacagcttagtcaaagaagg | 56145 | - | see also 5468 line |
| 27536 | CGI-09 | NM_015939.3 | 666 | cacatgagatacgatacactagc | 56172 | - | see also 11995 line |
| 27537 | CGI-09 | NM_015939.3 | 389 | agcgggcactgataatcgaaata | 56161 | - | see also 11995 line |
| 27538 | COPS5 | NM_006837.2 | 463 | ctggactaaggatcaccattact | 56192 | - | see also 4791 line |
| 27539 | COPS5 | NM_006837.2 | 1068 | gggtgaatacgttgagttcttct | 56213 | - | see also 4791 line |
| 27540 | COPS6 | NM_006833.4 | 632 | gaccacgtagcccgaatgacagc | 56232 | - | see also 4788 line |
| 27541 | COPS6 | NM_006833.4 | 769 | agcgggagaggtcccctttaatc | 56233 | - | see also 4788 line |
| 27542 | DENR | NM_003677.2 | 435 | aagaccgtaccacaaaaggttac | 56244 | - | see also 2498 line |
| 27543 | DENR | NM_003677.2 | 189 | tacccacttcgagtcctttattg | 56239 | - | see also 2498 line |
| 27544 | DENR | NM_003677.2 | 226 | taccaacagagtactgtgaatat | 56240 | - | see also 2498 line |
| 27545 | eIF2A | NM_032025.1 | 1021 | ctcgtaatgcagcctactatagc | 56279 | - | see also 11999 line |
| 27546 | eIF2A | NM_032025.1 | 1171 | gcccggatggtgagcatatttta | 56286 | - | see also 11999 line |
| 27547 | eIF2A | NM_032025.1 | 1172 | cccggatggtgagcatattttaa | 56287 | - | see also 11999 line |
| 27548 | EIF2B1 | NM_001414.1 | 249 | ccctggaatactccgattactcc | 56303 | - | see also 977 line |
| 27549 | EIF2B2 | NM_014239.1 | 847 | ctgtgcacctatgttcaaacttt | 56331 | - | see also 12001 line |
| 27550 | EIF2B2 | NM_014239.1 | 1057 | ccgcctgatgagtgaactctacc | 56333 | - | see also 12001 line |
| 27551 | EIF2B3 | NM_020365.1 | 1207 | tccattaagcgctcagtcattgg | 56358 | - | see also 12002 line |
| 27552 | EIF2B3 | NM_020365.1 | 457 | gacctgtttagagcttatgatgc | 56346 | - | see also 12002 line |

Figure 46

| | | | | | | | |
|---|---|---|---|---|---|---|---|
| 27553 | EIF2B4 | NM_015636.2 | 512 | caggttcctacacgaaaggatta | 56364 | - | see also 6525 line |
| 27554 | EIF2B5 | NM_003907.1 | 865 | ttgtgcgaggtctcttagtgaat | 56413 | - | see also 12004 line |
| 27555 | EIF2B5 | NM_003907.1 | 706 | agggtctccggcgttttgcattt | 56405 | - | see also 12004 line |
| 27556 | EIF2C1 | NM_012199.2 | 1951 | cagcgctctgccgttttcaaca | 56457 | - | see also 5187 line |
| 27557 | EIF2C1 | NM_012199.2 | 1282 | aaccagacctcgaccatgataaa | 56449 | - | see also 5187 line |
| 27558 | EIF2C2 | NM_012154.2 | 2340 | tcctctgggacgacaatcgtttc | 56501 | - | see also 12006 line |
| 27559 | EIF2C2 | NM_012154.2 | 1156 | gcccgatcggcaagaagagatta | 56483 | - | see also 12006 line |
| 27560 | EIF2C2 | NM_012154.2 | 2239 | cacgactgtggacacgaaaatca | 56498 | - | see also 12006 line |
| 27561 | eIF3k | NM_013234.1 | 462 | gccaatccgacagattttgtacc | 56503 | - | - | | - |
| 27562 | eIF3k | NM_013234.1 | 461 | ggccaatccgacagattttgtac | 56502 | - | see also 27561 line |
| 27563 | EIF3S1 | NM_003758.1 | 442 | gacctcgaattagcaaaggaaac | 56505 | - | see also 12007 line |
| 27564 | EIF3S1 | NM_003758.1 | 801 | tggaggatatgtacaagactatg | 56507 | - | see also 12007 line |
| 27565 | EIF3S10 | NM_003750.1 | 145 | ccctcaaacgcgccaacgaattt | 56509 | - | see also 12008 line |
| 27566 | EIF3S10 | NM_003750.1 | 3477 | aacgccgatgatgacagaattcc | 56601 | - | see also 12008 line |
| 27567 | EIF3S10 | NM_003750.1 | 624 | gagcgcctgtaccatgatattgc | 56524 | - | see also 12008 line |
| 27568 | EIF3S2 | NM_003757.1 | 46 | agcggtccattacgcagattaag | 56617 | - | see also 12009 line |
| 27569 | EIF3S2 | NM_003757.1 | 106 | aggaccctatcgtcaatgtatgg | 56623 | - | see also 12009 line |
| 27570 | EIF3S3 | NM_003756.1 | 316 | tgcggagccttcgccatgtaaac | 56640 | - | see also 12010 line |
| 27571 | EIF3S3 | NM_003756.1 | 841 | agcgtcgccagcaggagaatatg | 56661 | - | see also 12010 line |
| 27572 | EIF3S6 | NM_001568.1 | 697 | ccccaaaggtcgcgataatatta | 56684 | - | see also 12011 line |
| 27573 | EIF3S6 | NM_001568.1 | 696 | accccaaaggtcgcgataatatt | 56683 | - | see also 12011 line |
| 27574 | EIF3S6IP | NM_016091.1 | 84 | cgcttatcccagcgactatgata | 56711 | - | see also 6658 line |
| 27575 | EIF3S6IP | NM_016091.1 | 582 | ctgggatattatcgatgagttca | 56721 | - | see also 6658 line |
| 27576 | EIF3S7 | NM_003753.2 | 1729 | ctcggtaccacgtgaaagactcc | 56765 | - | see also 12013 line |
| 27577 | EIF3S7 | NM_003753.2 | 651 | cagcggaatcgaatgagatttgc | 56736 | - | see also 12013 line |
| 27578 | EIF3S8 | NM_003752.2 | 415 | ggctgacctagaggactatctta | 56769 | - | see also 2568 line |
| 27579 | EIF4EBP1 | NM_004095.2 | 357 | tgcgcaatagcccagaagataag | 56783 | - | - | | - |
| 27580 | EIF4EBP1 | NM_004095.2 | 386 | ggcggtgaagagtcacagtttga | 56784 | - | see also 27579 line |
| 27581 | EIF4EBP2 | NM_004096.3 | 374 | accgggaggaactcgaatcattt | 56788 | - | see also 12015 line |
| 27582 | EIF4EBP2 | NM_004096.3 | 317 | cgcgcagctacctcatgactatt | 56785 | - | see also 12015 line |
| 27583 | EIF4EBP3 | NM_003732.1 | 342 | acccgatgacgcacaatttgaaa | 56792 | - | - | | - |
| 27584 | EIF4EBP3 | NM_003732.1 | 343 | cccgatgacgcacaatttgaaat | 56793 | - | see also 27583 line |
| 27585 | EIF5A2 | NM_020390.5 | 193 | gtgctcggccttgcgcaaaaacg | 56832 | - | see also 7862 line |
| 27586 | EIF5A2 | NM_020390.5 | 232 | aggacgaccatgcaaaatagtgg | 56833 | - | see also 7862 line |
| 27587 | FLJ20288 | NM_024668.1 | 1015 | aacactgcgctaacttatgcatg | 378002 | - | see also 12018 line |
| 27588 | FLJ20288 | NM_024668.1 | 858 | agcacaagtattgcttgctatgc | 377997 | - | see also 12018 line |
| 27589 | GC20 | NM_005875.1 | 289 | aactaagggtgacgacttactcc | 56853 | - | see also 12019 line |

Figure 46

| | | | | | | | | | |
|---|---|---|---|---|---|---|---|---|---|
| 27590 | GC20 | NM_005875.1 | 530 | ctcttggaggttggcattgtaaa | 56858 | - | see also 12019 line | | |
| 27591 | GC20 | NM_005875.1 | 469 | tcctgaatacggagaggttattc | 56857 | - | see also 12019 line | | |
| 27592 | IF2 | NM_015904.2 | 3450 | ttctcgagatccgatagtgatgg | 56933 | - | see also 12020 line | | |
| 27593 | IF2 | NM_015904.2 | 3536 | ttgttgacatcggaatagtaaca | 56934 | - | see also 12020 line | | |
| 27594 | ITGB4BP | NM_002212.2 | 82 | gagcttcgttcgagaacaactgt | 56942 | - | see also 12021 line | | |
| 27595 | ITGB4BP | NM_002212.2 | 80 | ccgagcttcgttcgagaacaact | 56941 | - | see also 12021 line | | |
| 27596 | KIAA0664 | NM_015229.2 | 1236 | tccgagaaagggccatattcaag | 56952 | - | see also 12022 line | | |
| 27597 | KIAA0664 | NM_015229.2 | 2240 | gccttcgacattcgcttcaatcc | 56955 | - | see also 12022 line | | |
| 27598 | KIAA0664 | NM_015229.2 | 871 | acggggcttttacctgaatcagt | 56950 | - | see also 12022 line | | |
| 27599 | LAMA5 | NM_005560.3 | 10884 | accggctagcggtgatgaaaagc | 56988 | - | see also 12023 line | | |
| 27600 | LAMA5 | NM_005560.3 | 8395 | taccgaggatcgctttgtgatgt | 56972 | - | see also 12023 line | | |
| 27601 | LRIG2 | NM_014813.1 | 446 | gtcataatcggttgtctaactgg | 56992 | - | see also 12024 line | | |
| 27602 | LRIG2 | NM_014813.1 | 2891 | cccgggtgatttgctcagattgt | 57056 | - | see also 12024 line | | |
| 27603 | MFHAS1 | NM_004225.1 | 3055 | aacggcagcgagcgagtaaatgt | 57096 | - | see also 12025 line | | |
| 27604 | MFHAS1 | NM_004225.1 | 2128 | agcggcaagctactctactttga | 57081 | - | see also 12025 line | | |
| 27605 | MFHAS1 | NM_004225.1 | 3057 | cggcagcgagcgagtaaatgttg | 57097 | - | see also 12025 line | | |
| 27606 | MGC11102 | NM_032325.2 | 721 | aacacaaaccgcagacagtatca | 57101 | - | see also 12026 line | | |
| 27607 | MGC11102 | NM_032325.2 | 723 | cacaaaccgcagacagtatcatg | 57102 | - | see also 12026 line | | |
| 27608 | MGC3207 | NM_032285.1 | 787 | accggcaaggagatcattattga | 57104 | - | see also 12027 line | | |
| 27609 | MGC3207 | NM_032285.1 | 823 | caggagctgaccgatgttaatgg | 57107 | - | see also 12027 line | | |
| 27610 | MRPL49 | NM_004927.2 | 268 | tcccccaaagcatgaacattatc | 57109 | - | see also 12028 line | | |
| 27611 | MRPL49 | NM_004927.2 | 329 | cccaacctgccttactttgtacg | 57111 | - | see also 12028 line | | |
| 27612 | MTIF2 | NM_002453.1 | 1240 | tccgcacttacgggcgataatct | 57143 | - | see also 12029 line | | |
| 27613 | MTIF2 | NM_002453.1 | 1242 | cgcacttacgggcgataatctga | 57144 | - | see also 12029 line | | |
| 27614 | MTIF2 | NM_002453.1 | 1244 | cacttacgggcgataatctgatg | 57145 | - | see also 12029 line | | |
| 27615 | MTIF3 | NM_152912.3 | 693 | aacacctagtccagattaccata | 57191 | - | see also 12030 line | | |
| 27616 | MTIF3 | NM_152912.3 | 386 | cagcgagttattcacttatttga | 57184 | - | see also 12030 line | | |
| 27617 | PSMD7 | NM_002811.3 | 486 | ttccgtattggtcatcattgatg | 57204 | - | see also 1828 line | | |
| 27618 | PSMD7 | NM_002811.3 | 487 | tccgtattggtcatcattgatgt | 57205 | - | see also 1828 line | | |
| 27619 | RRN3 | NM_018427.2 | 1047 | atctatatcgcgacctgataaac | 57217 | - | see also 12032 line | | |
| 27620 | RRN3 | NM_018427.2 | 1053 | atcgcgacctgataaacatcttt | 57218 | - | see also 12032 line | | |
| 27621 | EEF1A2 | NM_001958.2 | 248 | atctacaaatgcggaggtattga | 57224 | - | see also 12033 line | | |
| 27622 | EEF1A2 | NM_001958.2 | 1465 | ggccgtaggcgtcatcaagaacg | 57229 | - | see also 12033 line | | |
| 27623 | EEF1G | NM_001404.3 | 456 | ggctgctggatgcttacttgaag | 57236 | - | - | translation elongation factor | - |

1028

Figure 46

| | | | | | | | |
|---|---|---|---|---|---|---|---|
| 27624 | EEF2 | NM_001961.2 | 966 | gtgtttgatgcgatcatgaattt | 57240 | - | see also 12034 line |
| 27625 | EEF2 | NM_001961.2 | 973 | atgcgatcatgaatttcaagaaa | 57242 | - | see also 12034 line |
| 27626 | EFG1 | NM_024996.5 | 597 | cgctacaacgttccgtttctaac | 57252 | - | see also 8830 line |
| 27627 | EFG1 | NM_024996.5 | 1677 | gcctttgagagaccattactgc | 57296 | - | see also 8830 line |
| 27628 | EFG1 | NM_024996.5 | 2113 | accgacgccatgggtaatcact | 57309 | - | see also 8830 line |
| 27629 | EFG2 | NM_032380.3 | 1354 | ttcaactgaagagcgtaactatg | 57343 | - | see also 9178 line |
| 27630 | EFG2 | NM_032380.3 | 1357 | acctgaagagcgtaactatgaat | 57344 | - | see also 9178 line |
| 27631 | ELL3 | NM_025165.1 | 815 | ctgaccaatcaggatttacaaga | 57402 | - | see also 12037 line |
| 27632 | ELL3 | NM_025165.1 | 863 | gaggacatggacccagattaga | 57404 | - | see also 12037 line |
| 27633 | FLJ13119 | NM_024580.3 | 198 | agccgcctagcagcaagttaag | 57414 | - | see also 8648 line |
| 27634 | FLJ13119 | NM_024580.3 | 369 | accgtcgttgcatttgtgatgg | 57426 | - | see also 8648 line |
| 27635 | FLJ13119 | NM_024580.3 | 471 | atccgtccgttttagttgattaa | 57427 | - | see also 8648 line |
| 27636 | FLJ13220 | NM_021927.1 | 1147 | agccgcaaataggagataacattat | 57499 | - | see also 8199 line |
| 27637 | FLJ13220 | NM_021927.1 | 1139 | gtcactgaagccgcaaataggaga | 57498 | - | see also 8199 line |
| 27638 | GSPT1 | NM_002094.1 | 843 | accgccaggtgctcctaagaaag | 57527 | - | see also 12040 line |
| 27639 | GSPT1 | NM_002094.1 | 1286 | aacacctaattgtgctaattaat | 57529 | - | see also 12040 line |
| 27640 | GSPT2 | NM_018094.1 | 1896 | accccgcttcgtgaaacaagatc | 57552 | - | see also 12041 line |
| 27641 | GSPT2 | NM_018094.1 | 1958 | gcctcgagacgttcaaagatttt | 57559 | - | see also 12041 line |
| 27642 | GTPBP1 | NM_004286.3 | 721 | gtccacgaaagtcattaccttca | 57570 | - | see also 2870 line |
| 27643 | GTPBP1 | NM_004286.3 | 355 | gagcatgcggaacagatagagg | 57566 | - | see also 2870 line |
| 27644 | GTPBP2 | NM_019096.2 | 395 | caccgaggtgctagttacgaaagg | 57586 | - | see also 12042 line |
| 27645 | GTPBP2 | NM_019096.2 | 533 | ccgggctcggctcaacctttcc | 57587 | - | see also 12042 line |
| 27646 | HBS1L | NM_006620.2 | 2023 | cgccgttataaacgattgatta | 57655 | - | see also 4680 line |
| 27647 | HBS1L | NM_006620.2 | 2024 | gccgttattaaacgattgattag | 57656 | - | see also 4680 line |
| 27648 | HTATSF1 | NM_014500.3 | 408 | aacgatggcgcatctagttctac | 57673 | - | see also 12044 line |
| 27649 | HTATSF1 | NM_014500.3 | 422 | tagttctaccgcaaagttgaag | 57676 | - | see also 12044 line |
| 27650 | SELB | NM_021937.2 | 455 | ttccgaggtgcaccgattatacc | 57728 | - | see also 12045 line |
| 27651 | SELB | NM_021937.2 | 1666 | gactttcaagcgttatgtcttcg | 57738 | - | see also 12045 line |
| 27652 | SUPT16H | NM_007192.2 | 1809 | gaggcgattgacttgaacacaaagg | 57766 | - | see also 4990 line |
| 27653 | SUPT16H | NM_007192.2 | 2306 | accggagtaatccgaaactgaaa | 57782 | - | see also 4990 line |
| 27654 | SUPT16H | NM_007192.2 | 843 | ggcatataccatcgtctgtaaagg | 57750 | - | see also 4990 line |
| 27655 | TUFM | NM_003321.3 | 542 | gacccgagagcacttattactgg | 57811 | - | see also 2253 line |
| 27656 | TUFM | NM_003321.3 | 311 | gggaggtgggctaagttcaaga | 57800 | - | see also 2253 line |
| 27657 | FLJ38663 | NM_152269.1 | 903 | tcccctcaggcatcgttgtaaag | 57827 | - | see also 12048 line |
| 27658 | FLJ38663 | NM_152269.1 | 647 | agcaccgtgggtttatttcattt | 57821 | - | see also 12048 line |
| 27659 | ICT1 | NM_001545.1 | 217 | tccctctagatcgcttgacaata | 57834 | - | see also 12049 line |
| 27660 | ICT1 | NM_001545.1 | 220 | ctctagatcgcttgacaatatct | 57836 | - | see also 12049 line |

Figure 46

| | | | | | | | |
|---|---|---|---|---|---|---|---|
| 27661 | ICT1 | NM_001545.1 | 218 | ccctctagatcgttgacaatat | 57835 | - | see also 12049 line |
| 27662 | MTRF1 | NM_004294.2 | 827 | ttccggtgacggtgtcataagc | 57861 | - | see also 12050 line |
| 27663 | MTRF1 | NM_004294.2 | 1248 | gagcgaattcggacatataatt | 57871 | - | see also 12050 line |
| 27664 | ABCF1 | NM_001090.1 | 857 | tcccgccaagccatgttagaaaa | 57885 | - | see also 12051 line |
| 27665 | ABCF1 | NM_001090.1 | 782 | gagcgccaagtggcttcattaaa | 57883 | - | see also 12051 line |
| 27666 | EEF2K | NM_013302.2 | 1614 | agctctggacgggtatgtgtaga | 57922 | - | see also 12052 line |
| 27667 | EEF2K | NM_013302.2 | 2029 | tagtggcgcgagctttgactct | 57927 | - | see also 12052 line |
| 27668 | PET112L | NM_004564.1 | 327 | tggaacttgccggttctcaaca | 57933 | - | see also 3103 line |
| 27669 | PET112L | NM_004564.1 | 831 | gggcgttcgaacggaagtgaaga | 57949 | - | see also 3103 line |
| 27670 | AF020591 | NM_014480.1 | 1923 | aactctaacctgtcatacatca | 57984 | - | see also 12054 line |
| 27671 | AF020591 | NM_014480.1 | 763 | ttctacctacaacattacctaca | 57970 | - | see also 12054 line |
| 27672 | AF020591 | NM_014480.1 | 905 | agctttctgtcagagtatttact | 57972 | - | see also 12054 line |
| 27673 | AFG3L2 | NM_006796.1 | 221 | ccggacgctttaccgatttgtta | 57987 | - | see also 12055 line |
| 27674 | AFG3L2 | NM_006796.1 | 1469 | caccaatcgaccagatatcctgg | 58018 | - | see also 12055 line |
| 27675 | ARIH2 | NM_006321.1 | 503 | ctcgagttcagcctaatccatca | 58055 | - | see also 12056 line |
| 27676 | ARIH2 | NM_006321.1 | 1420 | tacacctacccatatgcatatta | 58073 | - | see also 12056 line |
| 27677 | ARIH2 | NM_006321.1 | 916 | ttcaagtcgtcagatgtatca | 58062 | - | see also 12056 line |
| 27678 | ASK | NM_006716.2 | 1243 | aagtgagcaacacagaaacttg | 58075 | - | see also 4734 line |
| 27679 | ASK | NM_006716.2 | 1270 | gagtaaccagtatcaagttgttg | 58076 | - | see also 4734 line |
| 27680 | BANK1 | NM_017935.2 | 935 | gtctactgtgatggaatcgttaa | 58103 | - | see also 12058 line |
| 27681 | BANK1 | NM_017935.2 | 2301 | gacaactacgagactgcattatt | 58144 | - | see also 12058 line |
| 27682 | BANK1 | NM_017935.2 | 2445 | ctgctcgacccaagttgaaaag | 58147 | - | see also 12058 line |
| 27683 | BAT4 | NM_033177.2 | 756 | ggctgcagcccgagctttctag | 58152 | - | see also 12059 line |
| 27684 | BAT4 | NM_033177.2 | 1307 | gtgacaccacttccaagattcc | 58154 | - | see also 12059 line |
| 27685 | BDG29 | NM_015144.1 | 518 | tggtccgacgcatttttatgtgg | 58164 | - | see also 12060 line |
| 27686 | BDG29 | NM_015144.1 | 396 | ttgtggtctgattcttcaataac | 58159 | - | see also 12060 line |
| 27687 | BDG29 | NM_015144.1 | 2026 | gtgtgcccctgcaataataaac | 58184 | - | see also 12060 line |
| 27688 | BOLL | NM_033030.3 | 653 | gggatccctgttctagtataat | 58205 | - | see also 9351 line |
| 27689 | BOLL | NM_033030.3 | 652 | agggatccctgttctagtataa | 58204 | - | see also 9351 line |
| 27690 | BRUNOL6 | NM_052840.2 | 1003 | ctccgacacgctctacaataacg | 58226 | - | see also 9434 line |
| 27691 | BRUNOL6 | NM_052840.2 | 1273 | agccgttgtctctgctaaagtct | 58228 | - | see also 9434 line |
| 27692 | C13orf10 | NM_022118.3 | 1448 | aagcagtatgaggatagtagtgg | 58271 | - | see also 8282 line |
| 27693 | C13orf10 | NM_022118.3 | 717 | gtctcttcggtacctagtatttc | 58249 | - | see also 8282 line |
| 27694 | C14orf114 | NM_018199.1 | 114 | tggttcgcctgccaagctaatc | 58299 | - | see also 7314 line |
| 27695 | C14orf114 | NM_018199.1 | 240 | aggattatggcctcgttgttagg | 58307 | - | see also 7314 line |
| 27696 | C14orf6 | NM_145250.1 | 612 | caggtgccaataccatagtgtta | 58340 | - | see also 12065 line |
| 27697 | C14orf6 | NM_145250.1 | 268 | atgaaccgatcctgttagttaa | 58330 | - | see also 12065 line |

Figure 46

| | | | | | | | |
|---|---|---|---|---|---|---|---|
| 27698 | C20orf99 | NM_033089.4 | 891 | atctgaccggggagtacaaatg | 58346 | - | see also 12066 line |
| 27699 | C20orf99 | NM_033089.4 | 1009 | agcccaagacatgctttaatgt | 58348 | - | see also 12066 line |
| 27700 | C21orf107 | NM_018963.3 | 2793 | gtcgacgaattactcgattttgt | 58439 | - | see also 7610 line |
| 27701 | C21orf107 | NM_018963.3 | 3752 | accgatctttaacacaattcgaat | 58472 | - | see also 7610 line |
| 27702 | C21orf107 | NM_018963.3 | 5315 | ttgcgggtagcccggaaaaatg | 58511 | - | see also 7610 line |
| 27703 | CGI-18 | NM_015947.1 | 1202 | aggcatctggactaatagtgaaa | 58588 | - | see also 12068 line |
| 27704 | CGI-18 | NM_015947.1 | 619 | agcttgctctataagcatatagt | 58571 | - | see also 12068 line |
| 27705 | CGI-72 | NM_016018.3 | 1149 | gagctcgacttaacaagattact | 58627 | - | see also 12069 line |
| 27706 | CGI-72 | NM_016018.3 | 831 | aggattggatagctttagtcaaa | 58613 | - | see also 12069 line |
| 27707 | CNOT4 | NM_013316.1 | 1726 | accgagcggtttataattcattc | 58686 | - | see also 5396 line |
| 27708 | CNOT4 | NM_013316.1 | 1723 | agcaccgagcgtttataattca | 58684 | - | see also 5396 line |
| 27709 | COL4A3 | NM_000091.2 | 3841 | caggtctgcccggtgcaattatc | 58722 | - | see also 12071 line |
| 27710 | COL4A3 | NM_000091.2 | 2993 | ttcagagatatccacgtaatag | 58714 | - | see also 12071 line |
| 27711 | COVA1 | NM_006375.2 | 1177 | gtcgtagcgccaataacttctac | 58760 | - | see also 4465 line |
| 27712 | COVA1 | NM_006375.2 | 1174 | accgtcgtagcgccaataacttc | 58758 | - | see also 4465 line |
| 27713 | CPEB1 | NM_030594.2 | 1549 | gggtattagccgacagraacttt | 58809 | - | see also 8893 line |
| 27714 | CPEB1 | NM_030594.2 | 1693 | atgccggattgacacagataag | 58812 | - | see also 8893 line |
| 27715 | CPEB3 | NM_014912.1 | 1096 | accggagtaggccctatgatact | 58828 | - | see also 6085 line |
| 27716 | CPEB3 | NM_014912.1 | 1095 | gaccggagtaggccctatgatac | 58827 | - | see also 6085 line |
| 27717 | CPSF4 | NM_006693.1 | 250 | ctgcgtggcctatgcaaggaaagg | 58901 | - | see also 4726 line |
| 27718 | CSTF2T | NM_015235.1 | 357 | gcctgcagccgccattatgact | 58909 | - | see also 12077 line |
| 27719 | CSTF2T | NM_015235.1 | 58 | gtgagagaccccggcaatggatcg | 58906 | - | see also 12077 line |
| 27720 | DARS | NM_001349.1 | 916 | gactctaataccccatagacatct | 58942 | - | see also 896 line |
| 27721 | DC50 | NM_031210.3 | 50 | gggcgctgcgtagaagtatcaatc | 58963 | - | see also 12079 line |
| 27722 | DC50 | NM_031210.3 | 51 | gcgctgcgtagaagtatcaatca | 58964 | - | see also 12079 line |
| 27723 | DDX26 | NM_012141.1 | 1521 | caggtgracgtgagcaatagtgc | 58991 | - | see also 5148 line |
| 27724 | DDX26 | NM_012141.1 | 1935 | cggagccgatcacatggtttatc | 59000 | - | see also 5148 line |
| 27725 | DDX31 | NM_022779.7 | 1441 | cgtcggctagcttgatatcagtttg | 59078 | - | see also 12081 line |
| 27726 | DDX31 | NM_022779.7 | 1246 | gacgcctggtgatcatatataaaa | 59073 | - | see also 12081 line |
| 27727 | DDX39 | NM_005804.2 | 116 | aacgatcttttggattacgatga | 59104 | - | see also 12082 line |
| 27728 | DDX39 | NM_005804.2 | 1301 | caggaacggtttgaagttaatgt | 59115 | - | see also 12082 line |
| 27729 | DDX4 | NM_019039.1 | 1435 | aacagcgccaaaccccttatgttc | 59166 | - | see also 7670 line |
| 27730 | DDX4 | NM_019039.1 | 1951 | atcttgaatcggataaccattta | 59176 | - | see also 7670 line |
| 27731 | DDX42 | NM_007372.1 | 1014 | atcctgatcgacccattcgagt | 59212 | - | see also 5095 line |
| 27732 | DDX42 | NM_007372.1 | 807 | acccccaggtcgactgatagatca | 59201 | - | see also 5095 line |
| 27733 | DDX42 | NM_007372.1 | 1195 | aagagctagcgaataaccttaaa | 59218 | - | see also 5095 line |
| 27734 | DDX46 | NM_014829.1 | 158 | cacgccactatcgaaaacgatcg | 59241 | - | see also 6032 line |

Figure 46

| | | | | | | | |
|---|---|---|---|---|---|---|---|
| 27735 | DDX46 | NM_014829.1 | 152 | gggagtcacgcactatgaaaa | 59239 | - | see also 6032 line |
| 27736 | DDX47 | NM_016355.2 | 928 | agcgcctaggatccttaataag | 59342 | - | see also 6838 line |
| 27737 | DDX47 | NM_016355.2 | 197 | ggccttacaagtcgtgatatca | 59319 | - | see also 6838 line |
| 27738 | DDX49 | NM_019070.3 | 460 | ctgcgcagtccaacactttag | 59364 | - | see also 12087 line |
| 27739 | DDX49 | NM_019070.3 | 1259 | cggcccactttgacgaaaagaag | 59371 | - | see also 12087 line |
| 27740 | DDX50 | NM_024045.1 | 432 | aagcgagtatcatctttagatac | 59379 | - | see also 8570 line |
| 27741 | DDX51 | NM_175066.2 | 658 | cacggcatctgtcctactttcc | 59425 | - | see also 12088 line |
| 27742 | DDX51 | NM_175066.2 | 1411 | gacgcggattcggggaagtatgc | 59428 | - | see also 12088 line |
| 27743 | DDX51 | NM_175066.2 | 986 | aggagagcctcgtccagaaaaca | 59427 | - | see also 12088 line |
| 27744 | DDX52 | NM_007010.2 | 908 | gactactccaaatcgactaatct | 59444 | - | see also 4890 line |
| 27745 | DDX52 | NM_007010.2 | 599 | gcctacgccaatccaaatgcaag | 59436 | - | see also 4890 line |
| 27746 | DDX53 | NM_182699.1 | 1622 | agcgagcagtagagggacttaaa | 59512 | - | see also 12090 line |
| 27747 | DDX53 | NM_182699.1 | 1684 | tcccgaggcttgatcttaatga | 59518 | - | see also 12090 line |
| 27748 | DDX54 | NM_024072.2 | 666 | tgcacgaaaatcccgacataatt | 59535 | - | see also 12091 line |
| 27749 | DDX54 | NM_024072.2 | 1744 | ggcgactatctttgagatcaacg | 59543 | - | see also 12091 line |
| 27750 | DHX15 | NM_001358.1 | 912 | ctggagcgttatggttaataat | 59558 | - | see also 911 line |
| 27751 | DHX16 | NM_003587.3 | 273 | agcgcctacgagacactgatacc | 59617 | - | see also 2380 line |
| 27752 | DHX16 | NM_003587.3 | 3168 | aggtggctcccattattaag | 59653 | - | see also 2380 line |
| 27753 | DHX29 | NM_019030.1 | 3509 | gacgatctacaatgcatatctag | 59736 | - | see also 12094 line |
| 27754 | DHX29 | NM_019030.1 | 2901 | gagacgggtatcactattcctga | 59719 | - | see also 12094 line |
| 27755 | DHX33 | NM_020162.2 | 2040 | cgggacctcgtcatagatgc | 59786 | - | see also 7773 line |
| 27756 | DHX33 | NM_020162.2 | 920 | gacgtgccgagacaattgcaagc | 59770 | - | see also 7773 line |
| 27757 | DHX35 | NM_021931.2 | 1195 | gtcgtagtcgctcgggaaaatgt | 59825 | - | see also 12095 line |
| 27758 | DHX35 | NM_021931.2 | 609 | gggatcttcgattgattgtagc | 59805 | - | see also 12095 line |
| 27759 | DHX36 | NM_020865.1 | 981 | aacagggttcatctttatactgt | 59874 | - | see also 8005 line |
| 27760 | DHX36 | NM_020865.1 | 260 | ggcatgttgtacgcgaaaaaaaca | 59855 | - | see also 8005 line |
| 27761 | DHX36 | NM_020865.1 | 2385 | gactgtttcagatacgaaaag | 59931 | - | see also 8005 line |
| 27762 | DHX37 | NM_032656.2 | 1965 | ggccgagacgtcgcttaccatcc | 59970 | - | see also 12097 line |
| 27763 | DHX37 | NM_032656.2 | 1087 | tcctaccagatccggtatgaagg | 59963 | - | see also 12097 line |
| 27764 | DHX40 | NM_024612.3 | 2012 | gtctttgtgcgggctatttcaaa | 59993 | - | see also 12098 line |
| 27765 | DHX40 | NM_024612.3 | 537 | aggataccaagttcgtttgatg | 59986 | - | see also 12098 line |
| 27766 | DHX40 | NM_024612.3 | 2176 | accaaagtctacgcaagaattgt | 59997 | - | see also 12098 line |
| 27767 | DIAPH3 | NM_030932.2 | 220 | ctctctgcggtatgcattgtagg | 60008 | - | see also 8967 line |
| 27768 | DIAPH3 | NM_030932.2 | 1584 | aacacggttggcagagrtcatga | 60043 | - | see also 8967 line |
| 27769 | DKFZP434B1727 | NM_032143.1 | 781 | accggattcacatcctatactaag | 60100 | - | see also 12100 line |

Figure 46

| 27770 | DKFZP434B1727 | NM_032143.1 | 444 | ctctgcacctatatcaataattc | 60085 | - | see also 12100 line |
|---|---|---|---|---|---|---|---|
| 27771 | DKFZP434B195 | NM_031284.3 | 206 | agcctgggacgcgcttatcgtgc | 60138 | - | see also 9002 line |
| 27772 | DKFZP434B195 | NM_031284.3 | 919 | agctggccagtatgactaacagg | 60153 | - | see also 9002 line |
| 27773 | DKFZP434B195 | NM_031284.3 | 700 | cccatgccaaccgattcatcttc | 60148 | - | see also 9002 line |
| 27774 | DKFZP434I116 | NM_015496.3 | 1349 | cgccaacctatcgccttaaatgt | 60213 | - | see also 12102 line |
| 27775 | DKFZP434I116 | NM_015496.3 | 1348 | tcgccaacctatcgccttaaatg | 60212 | - | see also 12102 line |
| 27776 | DKFZp547B0714 | NM_152606.2 | 1521 | cagcttaatcggcataaaacaat | 60337 | - | see also 12103 line |
| 27777 | DKFZp547B0714 | NM_152606.2 | 2098 | gtcgtagtgtagaccttagaata | 60346 | - | see also 12103 line |
| 27778 | DKFZP564B1023 | NM_031306.1 | 1397 | gtgccaatgtacgtcagattatt | 60386 | - | see also 9009 line |
| 27779 | DKFZP564B1023 | NM_031306.1 | 1398 | tgccaatgtacgtcagattattt | 60387 | - | see also 9009 line |
| 27780 | DKFZp761J139 | NM_032280.1 | 3168 | cccgagttagtaccacatataac | 60401 | - | see also 12105 line |
| 27781 | DKFZp761J139 | NM_032280.1 | 3167 | gcccgagttagtaccacatataa | 60400 | - | see also 12105 line |
| 27782 | DKFZp762I137 | NM_152411.1 | 1351 | gggcttcgccaagcagtgtaaac | 60443 | - | see also 12106 line |
| 27783 | DKFZp762I137 | NM_152411.1 | 859 | ccggaacgctgacggtgaaatgt | 60442 | - | see also 12106 line |
| 27784 | DND1 | NM_194249.1 | 1082 | ggcaggtaccatggttaaacagt | 60453 | - | see also 12107 line |
| 27785 | DND1 | NM_194249.1 | 1080 | gaggcaggtaccatggttaaaca | 60452 | - | see also 12107 line |
| 27786 | DRB1 | NM_152945.1 | 612 | gggctacgtacgatacttaaaac | 60463 | - | see also 9967 line |
| 27787 | DRB1 | NM_152945.1 | 606 | aggtttgggctacgtacgatact | 60460 | - | see also 9967 line |
| 27788 | ECGP | NM_018025.2 | 1854 | aagcgatacgacgagttcttagt | 60514 | - | see also 12109 line |
| 27789 | ECGP | NM_018025.2 | 2328 | caccgaggtcccgacaaatcaag | 60522 | - | see also 12109 line |
| 27790 | EHD2 | NM_014601.2 | 1072 | tgcgagttcacgcttacatcatc | 60537 | - | see also 12110 line |
| 27791 | EHD2 | NM_014601.2 | 1499 | gaccaaggacaagtccaaatacg | 60539 | - | see also 12110 line |
| 27792 | EHD3 | NM_014600.1 | 1282 | aacaacctggccgagatctatgg | 60543 | - | see also 12111 line |
| 27793 | EHD3 | NM_014600.1 | 925 | atgcgagtggtgctgaacaaagc | 60541 | - | see also 12111 line |
| 27794 | ENDOGL1 | NM_005107.1 | 258 | cactaatcacgctttgtcttatg | 60549 | - | see also 12112 line |

Figure 46

| | | | | | | | |
|---|---|---|---|---|---|---|---|
| 27795 | ENDOGL1 | NM_005107.1 | 59 | ggggttcccgtcgttttctgagc | 60545 | - | see also 12112 line |
| 27796 | ENDOGL1 | NM_005107.1 | 885 | aactagtgatatccggaatatct | 60565 | - | see also 12112 line |
| 27797 | ENPP1 | NM_006208.1 | 755 | aacatgagaccggtatatccaac | 60583 | - | see also 4303 line |
| 27798 | ENPP1 | NM_006208.1 | 2348 | gacaccctactgcgaaagtatgc | 60631 | - | see also 4303 line |
| 27799 | ENPP2 | NM_006209.2 | 655 | gaggccggtgtacccaactaaaa | 60655 | - | see also 4304 line |
| 27800 | ENPP2 | NM_006209.2 | 531 | ttgttcgccctccattaatcatc | 60653 | - | see also 4304 line |
| 27801 | ENPP3 | NM_005021.2 | 1260 | cccgcatccgagctcataatata | 60767 | - | see also 12115 line |
| 27802 | ENPP3 | NM_005021.2 | 1262 | cgcatccgagctcataatatacc | 60769 | - | see also 12115 line |
| 27803 | ENPP3 | NM_005021.2 | 2179 | tactagcaatttggtacctatgt | 60800 | - | see also 12115 line |
| 27804 | FAM12A | NM_006683.3 | 461 | ggcgtagatggatatgttgataa | 60828 | - | see also 12116 line |
| 27805 | FAM12A | NM_006683.3 | 330 | aggggagcgaccgatatagaaat | 60819 | - | see also 12116 line |
| 27806 | FAM12A | NM_006683.3 | 331 | ggggagcgaccgatatagaaatg | 60820 | - | see also 12116 line |
| 27807 | FAM12B | NM_022360.2 | 275 | atggatcgcttccgaaatgcata | 60835 | - | see also 12117 line |
| 27808 | FAM12B | NM_022360.2 | 235 | atggtacaaaatcgagcatatat | 60834 | - | see also 12117 line |
| 27809 | FLJ10094 | NM_017993.1 | 697 | accgtgtttgtcggaggattacc | 60853 | - | see also 7191 line |
| 27810 | FLJ10094 | NM_017993.1 | 1786 | tcccatacacaagcgttactaaa | 60871 | - | see also 7191 line |
| 27811 | FLJ10252 | NM_018040.1 | 308 | gagaaaacggaggtcgtataatg | 60891 | - | see also 12119 line |
| 27812 | FLJ10252 | NM_018040.1 | 1397 | atccttatgcacgggagatatca | 60921 | - | see also 12119 line |
| 27813 | FLJ10290 | NM_018047.1 | 256 | gagtccgcatgcgtttcaagaag | 60932 | - | see also 12120 line |
| 27814 | FLJ10290 | NM_018047.1 | 715 | ttctaaagcgggcttcaacaatg | 60948 | - | see also 12120 line |
| 27815 | FLJ10377 | NM_018077.1 | 566 | gggaagatgcgcggttttggttt | 60969 | - | see also 7234 line |
| 27816 | FLJ10377 | NM_018077.1 | 468 | ttcggaacctgagctttaagtgt | 60965 | - | see also 7234 line |
| 27817 | FLJ10377 | NM_018077.1 | 2306 | aaccagctggtcgaacaatataa | 61001 | - | see also 7234 line |
| 27818 | FLJ10486 | NM_018109.2 | 523 | gtcacgcgtacggtcaagtaatc | 61013 | - | see also 7257 line |
| 27819 | FLJ10486 | NM_018109.2 | 267 | agcggactgttttaatacattgc | 61005 | - | see also 7257 line |
| 27820 | FLJ10634 | NM_018163.1 | 212 | agccagggctgcatatgacaagg | 61055 | - | see also 12123 line |
| 27821 | FLJ10634 | NM_018163.1 | 261 | gagaggacccagaaacttgatga | 61056 | - | see also 12123 line |
| 27822 | FLJ10891 | NM_018260.1 | 896 | taccttgcatgccatagatgtca | 61062 | - | - | - | - |
| 27823 | FLJ10891 | NM_018260.1 | 674 | aggccaaaaactcgtatttctaa | 61061 | - | see also 27822 line |
| 27824 | FLJ11016 | NM_018301.2 | 416 | tcggagcgtcagcgcattctttg | 61071 | - | see also 7373 line |
| 27825 | FLJ11016 | NM_018301.2 | 1215 | aagcgacttctctcatatcatgt | 61094 | - | see also 7373 line |
| 27826 | FLJ11126 | NM_018332.3 | 449 | tgctgaaccccacagaatctga | 61097 | - | see also 12125 line |
| 27827 | FLJ11142 | NM_018338.2 | 1686 | atccaaaagggctcacgattttt | 61148 | - | see also 12126 line |
| 27828 | FLJ11142 | NM_018338.2 | 479 | aagcgagccaacctacaacttct | 61107 | - | see also 12126 line |
| 27829 | FLJ11175 | NM_018349.1 | 916 | agcattcgcggtattttgatca | 61204 | - | see also 12127 line |
| 27830 | FLJ11175 | NM_018349.1 | 915 | tagcattcgcggtattttgatc | 61203 | - | see also 12127 line |
| 27831 | FLJ11637 | NM_024967.1 | 1084 | cacgcgagaacgcatgctaaaaa | 61233 | - | see also 12128 line |

Figure 46

| 27832 | FLJ11637 | NM_024967.1 | 485 | atcgtcatctgcgcaagaattgt | 61218 | - | see also 12128 line | | |
|---|---|---|---|---|---|---|---|---|---|
| 27833 | FLJ11637 | NM_024967.1 | 494 | tgcgcaagaattgttgtactagt | 61221 | - | see also 12128 line | | |
| 27834 | FLJ11806 | NM_024824.2 | 461 | tcctagcgcgagacctgaaaaaa | 61249 | - | see also 12129 line | | |
| 27835 | FLJ11806 | NM_024824.2 | 296 | atggcttcatggtgtattagata | 61242 | - | see also 12129 line | | |
| 27836 | FLJ12455 | NM_022078.1 | 101 | cggcccatttacggagctatttt | 61315 | - | see also 12130 line | | |
| 27837 | FLJ12455 | NM_022078.1 | 653 | gcctacccctcggatcatcacc | 61322 | - | see also 12130 line | | |
| 27838 | FLJ12529 | NM_024811.2 | 395 | atccaggttattcgctctatagg | 61338 | - | see also 8750 line | | |
| 27839 | FLJ12529 | NM_024811.2 | 665 | gcccattcccgagattctagtga | 61343 | - | see also 8750 line | | |
| 27840 | FLJ12606 | NM_024804.1 | 1242 | gtcgtttgagttccctttgtaac | 61361 | - | see also 12132 line | | |
| 27841 | FLJ12606 | NM_024804.1 | 654 | ttgatctgaacgagaacatttct | 61359 | - | see also 12132 line | | |
| 27842 | FLJ12644 | NM_023074.2 | 568 | gacgggacaacgctatgaacacg | 61365 | - | see also 12133 line | | |
| 27843 | FLJ12644 | NM_023074.2 | 1682 | agcttaagtcctagtgaacatgt | 61382 | - | see also 12133 line | | |
| 27844 | FLJ12998 | NM_022764.1 | 932 | cccgcttgcagccgatgtttacg | 61415 | - | see also 12134 line | | |
| 27845 | FLJ12998 | NM_022764.1 | 931 | ccccgcttgcagccgatgtttac | 61414 | - | see also 12134 line | | |
| 27846 | FLJ13590 | NM_024840.2 | 2100 | cagacctcattaactaacagtgc | 61439 | - | see also 12135 line | | |
| 27847 | FLJ13590 | NM_024840.2 | 1999 | atccttgctcagagagtcatagc | 61431 | - | see also 12135 line | | |
| 27848 | FLJ14451 | NM_032786.1 | 743 | gacgtcatgatctctatgatatc | 61446 | - | see also 12136 line | | |
| 27849 | FLJ14451 | NM_032786.1 | 741 | acgacgtcatgatctctatgata | 61445 | - | see also 12136 line | | |
| 27850 | FLJ14779 | NM_032838.2 | 486 | agggggggacatttcaatcaattg | 61461 | - | see also 12137 line | | |
| 27851 | FLJ14779 | NM_032838.2 | 485 | cagggggggacatttcaatcaatt | 61460 | - | see also 12137 line | | |
| 27852 | FLJ20035 | NM_017631.3 | 3952 | aaggtatttggtcgagtaaaatt | 61531 | - | see also 12138 line | | |
| 27853 | FLJ20035 | NM_017631.3 | 4942 | gactctcaactcgtatctcattt | 61554 | - | see also 12138 line | | |
| 27854 | FLJ20070 | NM_017652.1 | 806 | gggaagtcctttgcttatacatc | 61570 | - | see also 12139 line | | |
| 27855 | FLJ20070 | NM_017652.1 | 375 | aagcagagcacgtgtatacatat | 61568 | - | see also 12139 line | | |
| 27856 | FLJ20094 | NM_017665.1 | 149 | ctcaaggacagcagaactaaaga | 61572 | - | - | - | - |
| 27857 | FLJ20171 | NM_017697.1 | 973 | ggggagttcgccacagatattcg | 61597 | - | see also 12140 line | | |
| 27858 | FLJ20171 | NM_017697.1 | 450 | gacccggtatattgaggtttaca | 61584 | - | see also 12140 line | | |
| 27859 | FLJ20249 | NM_015590.2 | 308 | tacagataaggagcctttctaag | 61605 | - | see also 12141 line | | |
| 27860 | FLJ20249 | NM_015590.2 | 872 | ggcttggggaattgaatagcaga | 61612 | - | see also 12141 line | | |
| 27861 | FLJ20273 | NM_019027.1 | 619 | cagtgcgtgagctcaacaactac | 61616 | - | see also 12142 line | | |
| 27862 | FLJ20273 | NM_019027.1 | 1334 | tgggcccaacagggactactttg | 61617 | - | see also 12142 line | | |
| 27863 | FLJ20344 | NM_017776.1 | 297 | gtgtccgtggggtatctagttgc | 61625 | - | see also 12143 line | | |
| 27864 | FLJ20344 | NM_017776.1 | 294 | ctggtgtccgtggggtatctagt | 61624 | - | see also 12143 line | | |
| 27865 | FLJ20433 | NM_017820.2 | 1451 | tcctctcggacccctctatcacc | 61627 | - | see also 12144 line | | |
| 27866 | FLJ20433 | NM_017820.2 | 2270 | gccgctgccaggcctgtaactgt | 61632 | - | see also 12144 line | | |
| 27867 | FLJ20557 | NM_017879.1 | 208 | cccgtgactgcagaagctaaact | 61634 | - | see also 12145 line | | |
| 27868 | FLJ20557 | NM_017879.1 | 500 | tcccattcctgaccgacattttg | 61635 | - | see also 12145 line | | |

Figure 46

| 27869 | FLJ21839 | NM_021831.3 | 822 | gccccgtctagagcagctatttc | 61657 | - | see also 8182 line | | |
|---|---|---|---|---|---|---|---|---|---|
| 27870 | FLJ21839 | NM_021831.3 | 2069 | gtcgggcacgaagttttagcacc | 61676 | - | see also 8182 line | | |
| 27871 | FLJ21918 | NM_024939.1 | 1048 | cacatgggcgtccgctatattga | 61693 | - | see also 8811 line | | |
| 27872 | FLJ21918 | NM_024939.1 | 753 | ttcgaagcccgaggtgataaagc | 61691 | - | see also 8811 line | | |
| 27873 | FLJ21977 | NM_032213.3 | 951 | tgccgatggcgcaggagattttc | 61718 | - | see also 12148 line | | |
| 27874 | FLJ21977 | NM_032213.3 | 952 | gccgatggcgcaggagattttcc | 61719 | - | see also 12148 line | | |
| 27875 | FLJ22347 | NM_022830.1 | 114 | tgcctctgccacgttactacagc | 61723 | - | see also 12149 line | | |
| 27876 | FLJ22347 | NM_022830.1 | 1946 | agcactggggtgccatatagaac | 61735 | - | see also 12149 line | | |
| 27877 | FLJ22347 | NM_022830.1 | 1216 | accggctggccctgcataactcc | 61730 | - | see also 12149 line | | |
| 27878 | FLJ22559 | NM_024928.3 | 915 | ctccgaccaagtgaattttaaga | 61753 | - | see also 8810 line | | |
| 27879 | FLJ22559 | NM_024928.3 | 575 | gaggcagtatccgcacatacaga | 61747 | - | see also 8810 line | | |
| 27880 | FLJ22611 | NM_032226.1 | 474 | ttcggaatcttcgagtagtaaac | 61767 | - | see also 9123 line | | |
| 27881 | FLJ22611 | NM_032226.1 | 310 | gagactatagaagcatacgaaga | 61764 | - | see also 9123 line | | |
| 27882 | FLJ23233 | NM_024691.2 | 537 | aggactgctcagttcaaacattc | 61793 | - | see also 12152 line | | |
| 27883 | FLJ23233 | NM_024691.2 | 852 | gagactacatgctgggaaaaaga | 61794 | - | see also 12152 line | | |
| 27884 | FLJ23506 | NM_024833.1 | 571 | gagtctcacccatgtgacatatg | 61798 | - | see also 12153 line | | |
| 27885 | FLJ23506 | NM_024833.1 | 1221 | tagcagtaagtctaatctcattc | 61802 | - | see also 12153 line | | |
| 27886 | FLJ25078 | NM_152485.2 | 929 | caggcctgagggacattctaaac | 61814 | - | see also 12154 line | | |
| 27887 | FLJ25078 | NM_152485.2 | 973 | acccgatttaggactcagaatga | 61815 | - | see also 12154 line | | |
| 27888 | FLJ25344 | NM_182512.1 | 130 | cccttactcaggggcaattgtca | 61816 | - | - | - | - |
| 27889 | FLJ30594 | NM_153011.1 | 568 | gggggttacctcctaaatctaag | 61820 | - | see also 12155 line | | |
| 27890 | FLJ30594 | NM_153011.1 | 567 | tgggggttacctcctaaatctaa | 61819 | - | see also 12155 line | | |
| 27891 | FLJ30791 | NM_144694.1 | 1382 | cagaatgcacacctagttcaaca | 61835 | - | see also 12156 line | | |
| 27892 | FLJ30791 | NM_144694.1 | 636 | gacatctgtacagtaatgtgatg | 61827 | - | see also 12156 line | | |
| 27893 | FLJ30921 | NM_152360.2 | 623 | tacatagaagtacccacttatga | 61837 | - | see also 12157 line | | |
| 27894 | FLJ30921 | NM_152360.2 | 596 | ttggatttacagtgtgagataat | 61836 | - | see also 12157 line | | |
| 27895 | FLJ30927 | NM_144690.1 | 1626 | cagcagttaccccttactaatca | 61866 | - | see also 12158 line | | |
| 27896 | FLJ30927 | NM_144690.1 | 706 | aaggaactccttattaatcatca | 61852 | - | see also 12158 line | | |
| 27897 | FLJ30932 | NM_144693.1 | 472 | cactagggtgtcgtgttaataaa | 61868 | - | see also 12159 line | | |
| 27898 | FLJ30932 | NM_144693.1 | 470 | ctcactagggtgtcgtgttaata | 61867 | - | see also 12159 line | | |
| 27899 | FLJ30932 | NM_144693.1 | 958 | acgttttccgcaccagttgtaac | 61876 | - | see also 12159 line | | |
| 27900 | FLJ31030 | NM_152478.1 | 1276 | tagccatcgtggatacctaattg | 61903 | - | see also 12160 line | | |
| 27901 | FLJ31030 | NM_152478.1 | 419 | tgccctgattgggagtatgtatt | 61882 | - | see also 12160 line | | |
| 27902 | FLJ31100 | NM_173631.2 | 607 | aggaagaccgacatttgtgaaga | 61909 | - | see also 12161 line | | |
| 27903 | FLJ31100 | NM_173631.2 | 610 | aagaccgacatttgtgaagaacc | 61910 | - | see also 12161 line | | |
| 27904 | FLJ31139 | NM_173657.1 | 384 | acgattacgccgaataactgaga | 61923 | - | see also 12162 line | | |
| 27905 | FLJ31139 | NM_173657.1 | 336 | ttcggatattccagtagaattta | 61920 | - | see also 12162 line | | |

Figure 46

| 27906 | FLJ31295 | NM_152320.1 | 790 | tccagcgtgtctgaagatactgt | 61949 | - | see also 12163 line |
|---|---|---|---|---|---|---|---|
| 27907 | FLJ31295 | NM_152320.1 | 745 | gagggggatacccctgaactaga | 61945 | - | see also 12163 line |
| 27908 | FLJ31875 | NM_182531.1 | 569 | ctcagtagaatggcgacttaaaa | 61962 | - | see also 12164 line |
| 27909 | FLJ31875 | NM_182531.1 | 1539 | ctgtgcgctgtggccttactaga | 61984 | - | see also 12164 line |
| 27910 | FLJ31986 | NM_152476.1 | 923 | gaggcaacacgtctgaatgtatt | 61999 | - | see also 12165 line |
| 27911 | FLJ31986 | NM_152476.1 | 898 | aagaccaacatgagtactcaaaa | 61996 | - | see also 12165 line |
| 27912 | FLJ32130 | NM_152458.4 | 1259 | tccggcactttccagatatattt | 62034 | - | see also 12166 line |
| 27913 | FLJ32130 | NM_152458.4 | 1258 | ttccggcactttccagatatatt | 62033 | - | see also 12166 line |
| 27914 | FLJ32191 | NM_144689.3 | 1830 | gcctttgcgcgtggcttactact | 62051 | - | see also 12167 line |
| 27915 | FLJ32191 | NM_144689.3 | 1835 | tgcgcgtggcttactacttatac | 62052 | - | see also 12167 line |
| 27916 | FLJ32191 | NM_144689.3 | 573 | ttcaaccagcatacttacttatc | 62045 | - | see also 12167 line |
| 27917 | FLJ32932 | NM_152909.2 | 1126 | cagctccacatttgttagacatc | 62062 | - | see also 12168 line |
| 27918 | FLJ32932 | NM_152909.2 | 640 | ttccagaggggatgattggatac | 62058 | - | see also 12168 line |
| 27919 | FLJ33071 | NM_152457.1 | 861 | atctatcccgacacatgaatagc | 62076 | - | see also 12169 line |
| 27920 | FLJ33071 | NM_152457.1 | 677 | ttcagcgatcattcatacctagt | 62073 | - | see also 12169 line |
| 27921 | FLJ33071 | NM_152457.1 | 846 | ttcgccagcactctcatctatcc | 62075 | - | see also 12169 line |
| 27922 | FLJ33979 | NM_152636.1 | 869 | tgccagcatcgttgcagaatact | 62090 | - | see also 12170 line |
| 27923 | FLJ33979 | NM_152636.1 | 905 | cccagtggcccaaactctttatt | 62094 | - | see also 12170 line |
| 27924 | FLJ34817 | NM_152303.1 | 1015 | atccgattcagtcttaaaccacc | 62116 | - | see also 12171 line |
| 27925 | FLJ34817 | NM_152303.1 | 587 | tcctcatggacggggtatttact | 62098 | - | see also 12171 line |
| 27926 | FLJ34917 | NM_153263.1 | 1738 | ttctaccttgaggttaaacttct | 62137 | - | see also 12172 line |
| 27927 | FLJ34917 | NM_153263.1 | 2101 | caccagaaggtacatataacaga | 62142 | - | see also 12172 line |
| 27928 | FLJ34917 | NM_153263.1 | 1912 | caccgcaccagcctcattcaaca | 62139 | - | see also 12172 line |
| 27929 | FLJ35863 | NM_152604.1 | 893 | atgttactcggcatctgaaaatt | 62148 | - | see also 12173 line |
| 27930 | FLJ35863 | NM_152604.1 | 562 | agggagataatgggacttacaaa | 62146 | - | see also 12173 line |
| 27931 | FLJ36040 | NM_173530.1 | 1742 | tgcgtaccttacagatggaatga | 62160 | - | see also 12174 line |
| 27932 | FLJ36040 | NM_173530.1 | 1732 | gagaattcactgcgtaccttaca | 62158 | - | see also 12174 line |
| 27933 | FLJ36754 | NM_173829.2 | 244 | ttctccgagtagaccctaaaagg | 62161 | - | see also 12175 line |
| 27934 | FLJ36754 | NM_173829.2 | 577 | agcattcttctacacctaatagt | 62163 | - | see also 12175 line |
| 27935 | FLJ36991 | NM_152477.1 | 955 | tggtcgtacatcagaacttattc | 62176 | - | see also 12176 line |
| 27936 | FLJ36991 | NM_152477.1 | 502 | caggtgtgagaaatttctacaaa | 62168 | - | see also 12176 line |
| 27937 | FLJ38499 | NM_173797.2 | 1239 | tgggcaagtcaccatcagataaa | 62207 | - | see also 12177 line |
| 27938 | FLJ38499 | NM_173797.2 | 1214 | tccgttagtgctggtgattaaga | 62204 | - | see also 12177 line |
| 27939 | FLJ90396 | NM_153358.1 | 418 | aagactgccggagtaaaaccata | 62227 | - | see also 12178 line |
| 27940 | FLJ90396 | NM_153358.1 | 505 | cacactggatacgagctatttga | 62228 | - | see also 12178 line |
| 27941 | FLJ90396 | NM_153358.1 | 1499 | gtgcgttacgaacacatgaaaga | 62238 | - | see also 12178 line |
| 27942 | FLJ90430 | NM_178558.2 | 1723 | atgcgacgataattttgataaca | 62246 | - | see also 12179 line |

Figure 46

| | | | | | | | |
|---|---|---|---|---|---|---|---|
| 27943 | FLJ90430 | NM_178558.2 | 1574 | tggcctgcaactcttgctaatca | 62243 | - | see also 12179 line |
| 27944 | FLJ90798 | NM_153367.1 | 681 | gcgctgcttcggcgagtacaagt | 62248 | - | see also 12180 line |
| 27945 | FLJ90798 | NM_153367.1 | 660 | gactccataccagggcaaaaagc | 62247 | - | see also 12180 line |
| 27946 | GPR65 | NM_003608.2 | 1350 | atcacggttgcattaacaagttt | 62271 | - | see also 12181 line |
| 27947 | GPR65 | NM_003608.2 | 1008 | tgcgatgccgaaaagtctaattt | 62261 | - | see also 12181 line |
| 27948 | HEL308 | NM_133636.1 | 3214 | gggtcgagcaaaacagttataca | 62366 | - | see also 12182 line |
| 27949 | HEL308 | NM_133636.1 | 3213 | agggtcgagcaaaacagttatac | 62365 | - | see also 12182 line |
| 27950 | HEL308 | NM_133636.1 | 2939 | aacgttgtcaacaggctatatct | 62354 | - | see also 12182 line |
| 27951 | HELIC1 | NM_006828.1 | 5316 | ttccgacgtcttatcatgaatcc | 62530 | - | see also 12183 line |
| 27952 | HELIC1 | NM_006828.1 | 6168 | ggctcgtgggatgacttagttga | 62551 | - | see also 12183 line |
| 27953 | HELZ | NM_014877.1 | 5365 | ttcgctccctagcttagaagagt | 62716 | - | see also 12184 line |
| 27954 | HELZ | NM_014877.1 | 3843 | tggcctataacatgaacctatta | 62680 | - | see also 12184 line |
| 27955 | HELZ | NM_014877.1 | 4622 | ggggaggctttagatcgtataca | 62700 | - | see also 12184 line |
| 27956 | HNRNPG-T | NM_014469.2 | 303 | cggcaagtccctggatggtaagg | 62726 | - | see also 12185 line |
| 27957 | HNRNPG-T | NM_014469.2 | 1063 | gacagttacagcagcagttatgg | 62728 | - | see also 12185 line |
| 27958 | HRB2 | NM_007043.5 | 48 | aggcgctggaaaaagtgaatttc | 62729 | - | see also 12186 line |
| 27959 | HRB2 | NM_007043.5 | 626 | aagtagtccttgatactatgaag | 62744 | - | see also 12186 line |
| 27960 | HSA9947 | NM_022089.1 | 91 | gacgatagggacatcaatagatc | 62751 | - | see also 12187 line |
| 27961 | HSA9947 | NM_022089.1 | 287 | gccgaaacactcgttatcgaaat | 62755 | - | see also 12187 line |
| 27962 | HSPC055 | NM_014153.2 | 352 | gacgtttatcgggaacatgattg | 62787 | - | see also 5514 line |
| 27963 | HSPC055 | NM_014153.2 | 2111 | aagttcggtatggctgtttaagg | 62840 | - | see also 5514 line |
| 27964 | HTF9C | NM_022727.3 | 1040 | cccagcagactgagtatcgtaat | 62864 | - | see also 12189 line |
| 27965 | HTF9C | NM_022727.3 | 1042 | cagcagactgagtatcgtaataa | 62865 | - | see also 12189 line |
| 27966 | HZF12 | NM_033204.2 | 229 | tggcctcggtcggaatccaatgg | 62877 | - | see also 12190 line |
| 27967 | HZF12 | NM_033204.2 | 574 | gtggtgcacggcgcacagtaaca | 62883 | - | see also 12190 line |
| 27968 | IMP-1 | NM_006546.2 | 1061 | ctgctcctccgcttgtaagatga | 62902 | - | see also 12191 line |
| 27969 | IMP-1 | NM_006546.2 | 1316 | cagggccgagcaggaaataatga | 62910 | - | see also 12191 line |
| 27970 | KARS | NM_005548.1 | 970 | tggcatcgaccgggtttatgaaa | 62947 | - | see also 3862 line |
| 27971 | KARS | NM_005548.1 | 971 | ggcatcgaccgggtttatgaaat | 62948 | - | see also 3862 line |
| 27972 | KIAA0052 | NM_015360.2 | 1162 | gcccactccattgcaacactaca | 63005 | - | see also 6389 line |
| 27973 | KIAA0052 | NM_015360.2 | 1886 | ggctccgctgatcctctaaatag | 63022 | - | see also 6389 line |
| 27974 | KIAA0082 | NM_015050.1 | 1797 | ttcctccgaccctaaatcgaagt | 63093 | - | see also 6209 line |
| 27975 | KIAA0082 | NM_015050.1 | 390 | ttctcgctattccatgtataata | 63072 | - | see also 6209 line |
| 27976 | KIAA0082 | NM_015050.1 | 1563 | cacggattccgacgtcaacttgg | 63091 | - | see also 6209 line |
| 27977 | KIAA0150 | NM_015117.1 | 1314 | cccgctctcggcttacaaagtga | 63100 | - | see also 12195 line |

Figure 46

| | | | | | | | |
|---|---|---|---|---|---|---|---|
| 27978 | KIAA0150 | NM_015117.1 | 1513 | gtcaccacgcaccgactatgtcg | 63102 | - | see also 12195 line |
| 27979 | KIAA0663 | NM_014827.1 | 1328 | aacgtggagaattgcaaactaaa | 63108 | - | see also 12196 line |
| 27980 | KIAA1055 | NM_015079.2 | 779 | gacataattgggtcgtacaaaaa | 63116 | - | see also 12197 line |
| 27981 | KIAA1055 | NM_015079.2 | 2531 | tacttcactcgcactatccttga | 63146 | - | see also 12197 line |
| 27982 | KIAA1285 | NM_015694.1 | 348 | cgcacgatgggatcgtgattaag | 63147 | - | see also 12198 line |
| 27983 | KIAA1285 | NM_015694.1 | 547 | cagcgagttcagcgaactgaagc | 63148 | - | see also 12198 line |
| 27984 | KIAA1582 | NM_018996.2 | 2295 | accgtccattcgccgcaaaatgg | 63198 | - | see also 12199 line |
| 27985 | KIAA1582 | NM_018996.2 | 3707 | agcaagttgcgcgcacaatcact | 63223 | - | see also 12199 line |
| 27986 | KIAA1582 | NM_018996.2 | 4176 | ctcctatggccggtacgatttaa | 63227 | - | see also 12199 line |
| 27987 | KIAA1847 | NM_032527.2 | 604 | gggcactctggagtatcacaacg | 63241 | - | see also 12200 line |
| 27988 | KIAA1847 | NM_032527.2 | 897 | atgtggacaacggctactacaca | 63243 | - | see also 12200 line |
| 27989 | LEREPO4 | NM_018471.1 | 1095 | gtggtcgtgaagtgtttgaattt | 63265 | - | see also 12201 line |
| 27990 | LEREPO4 | NM_018471.1 | 1091 | atcagtggtcgtgaagtgtttga | 63263 | - | see also 12201 line |
| 27991 | LGALS12 | NM_033101.2 | 451 | tccttatgtcacgacgatttttg | 63276 | - | see also 12202 line |
| 27992 | LGALS12 | NM_033101.2 | 450 | ttccttatgtcacgacgattttt | 63275 | - | see also 12202 line |
| 27993 | LGALS12 | NM_033101.2 | 336 | ctcctggaacgaggatctacagt | 63273 | - | see also 12202 line |
| 27994 | LGP2 | NM_024119.1 | 1658 | tacgcgtttgtagcaactgaagg | 63300 | - | see also 12203 line |
| 27995 | LGP2 | NM_024119.1 | 1321 | cagccctcggggtatcatcttca | 63296 | - | see also 12203 line |
| 27996 | LKAP | NM_014647.1 | 3222 | agggcaccgtgcctttattgagc | 63391 | - | see also 5821 line |
| 27997 | LKAP | NM_014647.1 | 3027 | acgacggctcctccacgaattgc | 63384 | - | see also 5821 line |
| 27998 | LOC113251 | NM_052879.3 | 2260 | cagggagtgactcgacgtaatgg | 63513 | - | see also 12205 line |
| 27999 | LOC113251 | NM_052879.3 | 2155 | agggctagtaaggattattctgg | 63509 | - | see also 12205 line |
| 28000 | LOC115509 | NM_138447.1 | 921 | ggcgagtgcccctatgtttgtga | 63523 | - | see also 12206 line |
| 28001 | LOC115509 | NM_138447.1 | 822 | aagtgcgcccagaatctaaaaaa | 63522 | - | see also 12206 line |
| 28002 | LOC115509 | NM_138447.1 | 723 | cccagcaaaccccgactgattcc | 63518 | - | see also 12206 line |
| 28003 | LOC124245 | NM_144604.2 | 1154 | aagacgaacgggaatttgacaaa | 63539 | - | see also 12208 line |
| 28004 | LOC124245 | NM_144604.2 | 1242 | gccttacgcagacccttattatg | 63542 | - | see also 12208 line |
| 28005 | LOC126295 | NM_173480.1 | 1670 | atcccacgaatgtcagtcatact | 63575 | - | see also 12209 line |
| 28006 | LOC126295 | NM_173480.1 | 1674 | cacgaatgtcagtcatactcaaa | 63577 | - | see also 12209 line |

Figure 46

| | | | | | | | |
|---|---|---|---|---|---|---|---|
| 28007 | LOC146542 | NM_145271.2 | 504 | acgggcacagccacccaaaaatg | 63581 | - | see also 12210 line |
| 28008 | LOC147837 | NM_145276.1 | 1535 | tacgggagatgggccgaataaat | 63586 | - | see also 12211 line |
| 28009 | LOC147837 | NM_145276.1 | 458 | tagcctcattcgagatagtattg | 63582 | - | see also 12211 line |
| 28010 | LOC162655 | NM_145287.1 | 567 | aaccatgtattcctatgaataaa | 63593 | - | see also 12212 line |
| 28011 | LOC162655 | NM_145287.1 | 883 | atgtataatagtctttagtcaat | 63597 | - | see also 12212 line |
| 28012 | LOC163227 | NM_173531.1 | 1665 | tccctaagccttattaaacaaaa | 63600 | - | transcription_regulator |- | - |
| 28013 | LOC169270 | NM_173539.1 | 553 | ttcagtgactggttatcctttaa | 63609 | - | see also 12213 line |
| 28014 | LOC169270 | NM_173539.1 | 508 | tacatgagaacgaaacactttgt | 63607 | - | see also 12213 line |
| 28015 | LOC169270 | NM_173539.1 | 561 | ctggttatcctttaatcaacaca | 63611 | - | see also 12213 line |
| 28016 | LOC170261 | NM_173798.2 | 1500 | cacacaatccgctgttcgtattg | 63643 | - | see also 12214 line |
| 28017 | LOC170261 | NM_173798.2 | 777 | caggcgaccaaccctaacctaag | 63629 | - | see also 12214 line |
| 28018 | LOC201514 | NM_173548.1 | 511 | aggatgtgacggtatatttctcc | 63648 | - | see also 12215 line |
| 28019 | LOC201514 | NM_173548.1 | 1460 | gacccgttcaggcctctatcagc | 63653 | - | see also 12215 line |
| 28020 | LOC220992 | NM_145312.2 | 429 | cacacatgcagtcgaggattact | 63654 | - | see also 12216 line |
| 28021 | LOC220992 | NM_145312.2 | 678 | atgtgggatcgcctttatgaaca | 63658 | - | see also 12216 line |
| 28022 | LOC286075 | NM_173831.2 | 594 | acccacgtgcggcgagaaagagc | 63667 | - | see also 12217 line |
| 28023 | LOC286075 | NM_173831.2 | 908 | gaccttccggtgggcttcaaacc | 63669 | - | see also 12217 line |
| 28024 | LOC286075 | NM_173831.2 | 406 | tggctgctctgggattttgcagc | 63666 | - | see also 12217 line |
| 28025 | LOC55954 | NM_019103.1 | 417 | agccagggagtggctactagatg | 63670 | - | - | - | - |
| 28026 | LOC56902 | NM_020143.2 | 651 | gtgacacggacaaggatagtttt | 63679 | - | see also 7765 line |
| 28027 | LOC56902 | NM_020143.2 | 653 | gacacggacaaggatagttttgg | 63680 | - | see also 7765 line |
| 28028 | LOC56931 | NM_020175.1 | 132 | gcccattaagcgtcaatacctca | 63682 | - | see also 12219 line |

Figure 46

| | | | | | | | |
|---|---|---|---|---|---|---|---|
| 28029 | LOC56931 | NM_020175.1 | 1089 | gtccgagtgggccctactcaaac | 63687 | - | see also 12219 line |
| 28030 | LOC65243 | NM_023070.1 | 1474 | agcgtttccagattagacacc | 63714 | - | see also 12220 line |
| 28031 | LOC65243 | NM_023070.1 | 943 | aggccaagagtctaatagatttg | 63702 | - | see also 12220 line |
| 28032 | LOC81691 | NM_030941.1 | 1782 | ggcccagtccagtcaatgactt | 63764 | - | see also 12221 line |
| 28033 | LOC81691 | NM_030941.1 | 1069 | aagatgtacagaggcagttaaaa | 63743 | - | see also 12221 line |
| 28034 | LOC81691 | NM_030941.1 | 1022 | ttcgggaatcacgaagaagattc | 63737 | - | see also 12221 line |
| 28035 | LOC84524 | NM_032494.1 | 149 | cacggactctgacgaaagaatcg | 63770 | - | see also 12222 line |
| 28036 | LOC84524 | NM_032494.1 | 153 | gactctgacgaagaatcgatga | 63771 | - | see also 12222 line |
| 28037 | LOC84524 | NM_032494.1 | 159 | gacgaaagaatcgatgatgaaat | 63772 | - | see also 12222 line |
| 28038 | LOC84549 | NM_032509.2 | 787 | aggcccttagtgcgaaacacaaa | 63817 | - | see also 9223 line |
| 28039 | LOC90233 | NM_138347.2 | 1337 | ttcaatccattcgacatagaag | 63833 | - | see also 12223 line |
| 28040 | LOC90233 | NM_138347.2 | 427 | aactcacctcagtgagattaaga | 63820 | - | see also 12223 line |
| 28041 | LOC90462 | NM_183238.1 | 1112 | cagtaggaagtcgcagcttaaaa | 63855 | - | see also 12224 line |
| 28042 | LOC90462 | NM_183238.1 | 1119 | aagtcgcagcttaaaagacatca | 63857 | - | see also 12224 line |
| 28043 | LOC91120 | NM_033196.1 | 1341 | tactgcgtcaaacgctataaat | 63884 | - | see also 12225 line |
| 28044 | LOC91120 | NM_033196.1 | 804 | gtgttcaccctttgttagacata | 63882 | - | see also 12225 line |
| 28045 | LOC92906 | NM_138394.2 | 979 | tgcacggccaactcgtctaaatg | 63908 | - | see also 9611 line |
| 28046 | LOC92906 | NM_138394.2 | 1791 | ccttacggcactgaatcactat | 63942 | - | see also 9611 line |
| 28047 | MADP-1 | NM_033114.2 | 255 | atgactgtacggatatttttcc | 63950 | - | see also 9361 line |
| 28048 | MADP-1 | NM_033114.2 | 755 | gtcccctcaacatcagatgattc | 63963 | - | see also 9361 line |
| 28049 | MAN1 | NM_014319.3 | 1077 | ccgtgttaavgctaagaaaactga | 63980 | - | see also 12228 line |
| 28050 | MAN1 | NM_014319.3 | 1289 | atcatacggcctccaatcatacc | 63988 | - | see also 12228 line |
| 28051 | MBNL2 | NM_005757.3 | 1138 | gtcccgcgatagggacacaaatacg | 64052 | - | see also 4006 line |
| 28052 | MBNL2 | NM_005757.3 | 1151 | gacaaatacggctattagctttg | 64054 | - | see also 4006 line |
| 28053 | MBNL3 | NM_018388.2 | 329 | ggggcggaacaatctgattcaaca | 64076 | - | see also 12230 line |
| 28054 | MBNL3 | NM_018388.2 | 1018 | ggcacccgcttcaaatattgtgc | 64097 | - | see also 12230 line |
| 28055 | MDA5 | NM_022168.2 | 3070 | aagtgtgccgactatcaaataaa | 64164 | - | see also 8322 line |
| 28056 | MDA5 | NM_022168.2 | 3067 | aagaagtgtgccgactatcaaat | 64163 | - | see also 8322 line |
| 28057 | MGC10433 | NM_024321.3 | 1385 | cggcgccatgcgtgagatgaatgg | 64175 | - | see also 12232 line |
| 28058 | MGC10433 | NM_024321.3 | 1282 | ccgcttcccatccttccttaagg | 64174 | - | see also 12232 line |
| 28059 | MGC10520 | NM_030580.2 | 849 | aagcgtgtacacggtcaaaaaa | 64181 | - | see also 12233 line |
| 28060 | MGC10520 | NM_030580.2 | 850 | agcgtgtacacaggtcaaaaaaa | 64182 | - | see also 12233 line |
| 28061 | MGC10715 | NM_024325.4 | 1152 | aaccctgtgcagctagacacaaagg | 64198 | - | see also 12234 line |

Figure 46

| | | | | | | | |
|---|---|---|---|---|---|---|---|
| 28062 | MGC10715 | NM_024325.4 | 1361 | ctgatggagaagccttatgtgt | 64202 | - | see also 12234 line |
| 28063 | MGC10871 | NM_031492.2 | 698 | tggagcaactcgactactataagc | 64213 | - | see also 12235 line |
| 28064 | MGC10871 | NM_031492.2 | 592 | cgggtcgtgtggcagacctttact | 64208 | - | see also 12235 line |
| 28065 | MGC12466 | NM_033213.1 | 1309 | agcatgaaagagcttatatgtgg | 64225 | - | see also 12236 line |
| 28066 | MGC12466 | NM_033213.1 | 959 | cacttacttgggaatacatgaaa | 64221 | - | see also 12236 line |
| 28067 | MGC13105 | NM_032497.1 | 2153 | tactcggtccacatatcttattc | 64256 | - | see also 12237 line |
| 28068 | MGC13105 | NM_032497.1 | 1432 | ctcatagtagagtgttaacctata | 64246 | - | see also 12237 line |
| 28069 | MGC13105 | NM_032497.1 | 1989 | tactcctcgcaccttagtcaaca | 64253 | - | see also 12237 line |
| 28070 | MGC17986 | NM_153608.1 | 707 | tcctgtcttaaacgatagtcaaa | 64267 | - | see also 12238 line |
| 28071 | MGC17986 | NM_153608.1 | 1329 | gtcattcgggagtcctcaaaata | 64280 | - | see also 12238 line |
| 28072 | MGC17986 | NM_153608.1 | 1333 | ttcgggagtcctcaaaatataca | 64281 | - | see also 12238 line |
| 28073 | MGC26914 | NM_144976.1 | 769 | cgcccaagtttatttcagataca | 64291 | - | see also 12240 line |
| 28074 | MGC26914 | NM_144976.1 | 1449 | aaggattagaaaacatatgatac | 64293 | - | see also 12240 line |
| 28075 | MGC26914 | NM_144976.1 | 767 | atcgcccaagtttatttcagata | 64289 | - | see also 12240 line |
| 28076 | MGC27016 | NM_144979.2 | 790 | aaccatgcagagagttaaagttc | 64310 | - | see also 12241 line |
| 28077 | MGC27016 | NM_144979.2 | 1656 | atgttggacagcggctatgtatc | 64353 | - | see also 12241 line |
| 28078 | MGC2734 | NM_033117.2 | 175 | tgcaggaaggacaccgattatgg | 64356 | - | see also 9368 line |
| 28079 | MGC2734 | NM_033117.2 | 505 | agcctactcagtctaacctaagt | 64365 | - | see also 9368 line |
| 28080 | MGC32104 | NM_144684.1 | 1597 | ggcctttagtcgcatttcatacc | 64378 | - | see also 12242 line |
| 28081 | MGC32104 | NM_144684.1 | 1439 | cacaagttatcactaaccaatca | 64376 | - | see also 12242 line |
| 28082 | MGC33637 | NM_152596.2 | 1695 | aagatcgattcctggataagtcc | 64416 | - | see also 12243 line |

Figure 46

| | | | | | | | |
|---|---|---|---|---|---|---|---|
| 28083 | MGC33637 | NM_152596.2 | 563 | ttgccgagtttacttatttgaca | 64389 | - | see also 12243 line |
| 28084 | MGC4054 | NM_024341.1 | 1093 | tacgggggagggtcattatgtat | 64429 | - | see also 12244 line |
| 28085 | MGC4054 | NM_024341.1 | 679 | agcaacactcaacgaatgtaatc | 64425 | - | see also 12244 line |
| 28086 | MGC41917 | NM_153231.2 | 1044 | acccaccggtctactttattcg | 64443 | - | see also 12245 line |
| 28087 | MGC41917 | NM_153231.2 | 726 | gggaaagacgccttgattcatgc | 64442 | - | see also 12245 line |
| 28088 | MGC42415 | NM_153363.1 | 561 | tccgccagagctaggcataaaag | 64450 | - | see also 12246 line |
| 28089 | MGC42415 | NM_153363.1 | 1357 | ttctcctcaactcttaatactca | 64452 | - | see also 12246 line |
| 28090 | MGC43537 | NM_178544.2 | 2186 | gacgtttagtcggcactatcatc | 64475 | - | see also 12247 line |
| 28091 | MGC43537 | NM_178544.2 | 735 | cagatgggatgcgttagtcaaat | 64465 | - | see also 12247 line |
| 28092 | MGC4400 | NM_032679.1 | 1114 | gccggaagtgccggctcaataga | 64495 | - | see also 12248 line |
| 28093 | MGC4400 | NM_032679.1 | 1116 | cggaagtgccggctcaatagaca | 64496 | - | see also 12248 line |
| 28094 | MGC45380 | NM_133466.1 | 774 | gagtgcgccaacagcttactttt | 64513 | - | see also 12249 line |
| 28095 | MGC45380 | NM_133466.1 | 776 | gtgcgccaacagcttactttca | 64514 | - | see also 12249 line |
| 28096 | MGC45586 | NM_152603.2 | 752 | aaggggaggcctgattacacata | 64540 | - | see also 12250 line |
| 28097 | MGC45586 | NM_152603.2 | 355 | aacaccaagagaagtattctaga | 64530 | - | see also 12250 line |
| 28098 | MGC48625 | NM_182609.1 | 473 | agcctgtgggactatgatgtaaa | 64557 | - | see also 12251 line |
| 28099 | MGC48625 | NM_182609.1 | 383 | taccatctggtgatattagaaag | 64552 | - | see also 12251 line |
| 28100 | MKI67IP | NM_032390.3 | 67 | gggccaatcctgtcgcttaatcc | 64565 | - | see also 12252 line |
| 28101 | MKI67IP | NM_032390.3 | 134 | agcgcataacccagcgaaaaaaa | 64567 | - | see also 12252 line |
| 28102 | MKRN1 | NM_013446.2 | 401 | ctctgacagtccgtatagtgtag | 64584 | - | - nucleic acid binding - |
| 28103 | MKRN1 | NM_013446.2 | 399 | ctctctgacagtccgtatagtgt | 64583 | - | see also 28102 line |
| 28104 | MKRN2 | NM_014160.3 | 423 | cagccgagcatggtgagtaatcc | 64591 | - | see also 12253 line |
| 28105 | MKRN2 | NM_014160.3 | 197 | tggaactcggtgcagatatgacc | 64586 | - | see also 12253 line |
| 28106 | MKRN3 | NM_005664.1 | 1424 | agcgcatactggcatcaacttgt | 64615 | - | see also 12254 line |
| 28107 | MKRN3 | NM_005664.1 | 831 | tgcggttttgctattatgcttcc | 64607 | - | see also 12254 line |
| 28108 | MNAB | NM_018835.1 | 1085 | aaccacagccggaaatgtcattt | 64656 | - | see also 12255 line |

Figure 46

| | | | | | | | |
|---|---|---|---|---|---|---|---|
| 28109 | MNAB | NM_018835.1 | 1188 | cagctaatctcacgtagtactga | 64659 | - | see also 12255 line |
| 28110 | MSI2 | NM_138962.2 | 269 | ctcaccagatagccttagagact | 64709 | - | see also 9643 line |
| 28111 | MSI2 | NM_138962.2 | 274 | cagatagccttagagactatttt | 64710 | - | see also 9643 line |
| 28112 | MYEOV | NM_138768.1 | 1304 | atcgagctgccctgactatgaaa | 64722 | - | see also 12257 line |
| 28113 | MYEOV | NM_138768.1 | 1199 | tgggctctcatggaaattatgt | 64721 | - | see also 12257 line |
| 28114 | NARS | NM_004539.2 | 423 | ttggtgcgttagaaggattataga | 64736 | - | see also 3095 line |
| 28115 | NARS | NM_004539.2 | 518 | ttgcgagatggtacaggttatct | 64740 | - | see also 3095 line |
| 28116 | NPM2 | NM_182795.1 | 754 | caggcgagccgtggctaagaaaaa | 64773 | - | see also 12259 line |
| 28117 | NPM2 | NM_182795.1 | 583 | ctcagtggccaggaaacgttatga | 64771 | - | see also 12259 line |
| 28118 | NPM2 | NM_182795.1 | 756 | ggcgagcgtggctaagaaaaaaa | 64774 | - | see also 12259 line |
| 28119 | NPM3 | NM_006993.1 | 101 | cccggtcactatggacagtttt | 64777 | - | see also 12260 line |
| 28120 | NPM3 | NM_006993.1 | 152 | ccgctcccttcaccttaagttag | 64779 | - | see also 12260 line |
| 28121 | NUPL2 | NM_007342.1 | 1350 | cagcatcattgcaacagataatg | 64802 | - | see also 12261 line |
| 28122 | NUPL2 | NM_007342.1 | 1384 | cccagagataaaactaacagtaga | 64805 | - | see also 12261 line |
| 28123 | PCTAIRE2 BP | NM_014290.1 | 2615 | ttgctcggactgtagcattaagg | 64876 | - | see also 5597 line |
| 28124 | PCTAIRE2 BP | NM_014290.1 | 515 | ccgggcgtcaagttaattgtcaga | 64816 | - | see also 5597 line |
| 28125 | PDIP46 | NM_032311.3 | 1076 | atgccatcaccgcatataagaag | 64901 | - | see also 9152 line |
| 28126 | PDIP46 | NM_032311.3 | 1073 | acgatgccatcaccgcatataag | 64900 | - | see also 9152 line |
| 28127 | PERC | NM_133263.2 | 3007 | gactacgattccaattcagaaga | 64930 | - | see also 12264 line |
| 28128 | PERC | NM_133263.2 | 895 | caggcgatggtgcaactcatacg | 64915 | - | see also 12264 line |
| 28129 | PINX1 | NM_017884.3 | 489 | ctccaaaaccgtgttcactata | 64943 | - | see also 7170 line |
| 28130 | PINX1 | NM_017884.3 | 195 | ctggagtaatgacgattccaagt | 64932 | - | see also 7170 line |
| 28131 | PNMA3 | NM_013364.1 | 397 | ctcgggtcggacaccaattgttc | 64962 | - | see also 12266 line |
| 28132 | PNMA3 | NM_013364.1 | 395 | tcctcgggtcggacaccaattgt | 64961 | - | see also 12266 line |
| 28133 | PNMA3 | NM_013364.1 | 368 | ggcggaccgtgtcagatatgaac | 64959 | - | see also 12266 line |
| 28134 | PPIL4 | NM_139126.2 | 1375 | aggatggccattatagttaatag | 65012 | - | see also 9660 line |
| 28135 | PPIL4 | NM_139126.2 | 473 | ttgtaccatatcaggatatcagg | 64993 | - | see also 9660 line |
| 28136 | PPP1R10 | NM_002714.2 | 1989 | tagtcaggagggatatatccagg | 65041 | - | see also 12268 line |
| 28137 | PPP1R10 | NM_002714.2 | 1614 | cccggttccccgtttgaagtcc | 65031 | - | see also 12268 line |
| 28138 | PRC | NM_015062.3 | 4585 | cggccggtacagtcttatcgttc | 65087 | - | see also 12269 line |
| 28139 | PRC | NM_015062.3 | 4586 | ggcggtacagtcttatcgttca | 65088 | - | see also 12269 line |
| 28140 | PRR3 | NM_025263.1 | 861 | aagctgtctcttatcaagaata | 65102 | - | see also 12270 line |
| 28141 | PRR3 | NM_025263.1 | 845 | accctgaaggctcaaaagctgg | 65101 | - | see also 12270 line |
| 28142 | PSPC1 | NM_018282.1 | 368 | ggggttcactatcgacatcaaga | 65105 | - | see also 7362 line |
| 28143 | PSPC1 | NM_018282.1 | 759 | cgcggtagagctacaggaaaagg | 65115 | - | see also 7362 line |

Figure 46

| 28144 | PTBP2 | NM_021190.1 | 414 | ttcgtaaccaaccaatatatatc | 65146 | - | see also 8123 line | | |
|---|---|---|---|---|---|---|---|---|---|
| 28145 | PTBP2 | NM_021190.1 | 225 | ctcgtgtacttcatattcgaaaa | 65143 | - | see also 8123 line | | |
| 28146 | PTBP2 | NM_021190.1 | 125 | ctcagtagtccgaactctaatat | 65136 | - | see also 8123 line | | |
| 28147 | R3HDM | NM_015361.2 | 2535 | gagtggtcatcccctatacttca | 65250 | - | see also 6393 line | | |
| 28148 | R3HDM | NM_015361.2 | 1686 | gcgcacctgtcgtctatccaact | 65230 | - | see also 6393 line | | |
| 28149 | RAD52B | NM_145654.2 | 320 | ttcgtcttggcaccagacataag | 65279 | - | see also 12273 line | | |
| 28150 | RAD52B | NM_145654.2 | 122 | cggccgaggctttgcatcattct | 65270 | - | see also 12273 line | | |
| 28151 | RAVER1 | NM_133452.1 | 29 | ggcggacgtgtccgttactcacc | 65293 | - | see also 12274 line | | |
| 28152 | RAVER1 | NM_133452.1 | 260 | cagtgactatgagctcaaatact | 65295 | - | see also 12274 line | | |
| 28153 | RBAK | NM_021163.2 | 1836 | tcgggtgtcatacctcactatac | 65316 | - | see also 12275 line | | |
| 28154 | RBAK | NM_021163.2 | 816 | aaggcaagctgcttgtatcaata | 65300 | - | see also 12275 line | | |
| 28155 | RBM14 | NM_006328.1 | 2017 | cacgctattcgggctcctataat | 65347 | - | see also 4430 line | | |
| 28156 | RBM14 | NM_006328.1 | 2024 | ttcgggctcctataatgattacc | 65350 | - | see also 4430 line | | |
| 28157 | RECQL4 | NM_004260.1 | 2071 | ctgctgcaaggcaaacgttttca | 65357 | - | see also 12277 line | | |
| 28158 | RECQL4 | NM_004260.1 | 2198 | gaggtcgtgcccccaaaaccaca | 65359 | - | see also 12277 line | | |
| 28159 | RECQL5 | NM_004259.3 | 2828 | agcgaacagggcaccttgaatcc | 65386 | - | see also 12278 line | | |
| 28160 | RECQL5 | NM_004259.3 | 268 | tgcgaccatggctgtagtaaaag | 65365 | - | see also 12278 line | | |
| 28161 | RIG-I | NM_014314.2 | 1550 | atcacggattagcgacaaattta | 65427 | - | see also 5617 line | | |
| 28162 | RIG-I | NM_014314.2 | 1297 | aacacccgtacaatatgatcatg | 65417 | - | see also 5617 line | | |
| 28163 | RIG-I | NM_014314.2 | 1294 | aacaacacccgtacaatatgatc | 65416 | - | see also 5617 line | | |
| 28164 | RNASE2 | NM_002934.1 | 330 | atgacctgtcctagtaacaaaac | 65473 | - | see also 12280 line | | |
| 28165 | RNASE2 | NM_002934.1 | 452 | gacaccagcaaacatgttctata | 65475 | - | see also 12280 line | | |
| 28166 | RNASE3 | NM_002935.1 | 226 | cgggcaattaacaattatcgatg | 65481 | - | see also 12281 line | | |
| 28167 | RNASE3 | NM_002935.1 | 223 | atgcgggcaattaacaattatcg | 65480 | - | see also 12281 line | | |
| 28168 | RNASE4 | NM_002937.3 | 712 | atcgctactgcaacttgatgatg | 65492 | - | see also 12282 line | | |
| 28169 | RNASE4 | NM_002937.3 | 713 | tcgctactgcaacttgatgatgc | 65493 | - | see also 12282 line | | |
| 28170 | RNASE6 | NM_005615.2 | 718 | ttcctgtacacttagatagtatt | 65506 | - | see also 12283 line | | |
| 28171 | RNASE6 | NM_005615.2 | 719 | tcctgtacacttagatagtattc | 65507 | - | see also 12283 line | | |
| 28172 | RNASE7 | NM_032572.2 | 630 | ctgtaagcctccccagaaaaagg | 65508 | - | - | pancreatic ribonuclease | - |
| 28173 | RNASE8 | NM_138331.1 | 156 | agccatgagcatcatcaataagt | 65511 | - | see also 12284 line | | |
| 28174 | RNASE8 | NM_138331.1 | 153 | ctcagccatgagcatcatcaata | 65509 | - | see also 12284 line | | |
| 28175 | RNPC4 | NM_018107.3 | 1030 | ggccgctctaaaggttatggttt | 65538 | - | see also 12285 line | | |
| 28176 | RNPC4 | NM_018107.3 | 374 | atcggtatagacggagaaatagt | 65517 | - | see also 12285 line | | |
| 28177 | RNPC6 | NM_153020.1 | 625 | tgcagacagaccgaatgcaatag | 65548 | - | see also 12286 line | | |
| 28178 | RNPC6 | NM_153020.1 | 144 | aaggatcccaatcccatcattga | 65542 | - | see also 12286 line | | |
| 28179 | RoXaN | NM_017590.4 | 577 | cgcaaggcacgcgctctcaatga | 65553 | - | see also 12287 line | | |

Figure 46

| 28180 | RoXaN | NM_017590.4 | 713 | gagttcgcaaggcgtataagagg | 65558 | - | see also 12287 line | | |
|---|---|---|---|---|---|---|---|---|---|
| 28181 | RSN | NM_002956.2 | 1142 | gcccagtcggacaggactattga | 65586 | - | see also 1994 line | | |
| 28182 | RSN | NM_002956.2 | 703 | ctccgatcagcaaccttacaaaa | 65581 | - | see also 1994 line | | |
| 28183 | RY1 | NM_006857.1 | 442 | cagtacatgaatcgaaaaggtgg | 65647 | - | see also 4804 line | | |
| 28184 | SART3 | NM_014706.2 | 1718 | acccgattagctcgtgtcaatga | 65677 | - | see also 12290 line | | |
| 28185 | SART3 | NM_014706.2 | 1410 | gtgatccaagctgcgtgattatg | 65669 | - | see also 12290 line | | |
| 28186 | SBNO1 | NM_018183.2 | 2924 | aacagttcggacgtactcataga | 65788 | - | see also 12291 line | | |
| 28187 | SBNO1 | NM_018183.2 | 2904 | gagcgctgatagagcaattcaac | 65786 | - | see also 12291 line | | |
| 28188 | SBZF3 | NM_020394.2 | 251 | tggtgaggatagcttcaatatgc | 65845 | - | see also 12292 line | | |
| 28189 | SBZF3 | NM_020394.2 | 478 | ttgagaaattacgcttaaggaat | 65853 | - | see also 12292 line | | |
| 28190 | SEC8 | NM_021807.2 | 739 | ctcctcgaaaattccttgatacc | 65864 | - | see also 8173 line | | |
| 28191 | SEC8 | NM_021807.2 | 1889 | tgcagcttacaggggtattgtcc | 65891 | - | see also 8173 line | | |
| 28192 | SECP43 | NM_017846.3 | 102 | gaccgtaatgagcgtcaaaatta | 65902 | - | see also 12293 line | | |
| 28193 | SECP43 | NM_017846.3 | 418 | ggcgtgtctaagggttatggttt | 65914 | - | see also 12293 line | | |
| 28194 | SECP43 | NM_017846.3 | 237 | cacacctgcgaaacgttttaaac | 65909 | - | see also 12293 line | | |
| 28195 | SFRS12 | NM_139168.1 | 1639 | aacgttcaacgtctatgagaaag | 65948 | - | see also 9672 line | | |
| 28196 | SFRS12 | NM_139168.1 | 993 | aggagtagatcccataatagatc | 65939 | - | see also 9672 line | | |
| 28197 | SHARP | NM_015001.2 | 5912 | atgtaaggagcgtctatgcaacc | 66090 | - | see also 6175 line | | |
| 28198 | SHARP | NM_015001.2 | 9754 | taccgactgcacccctatactgt | 66133 | - | see also 6175 line | | |
| 28199 | SIAHBP1 | NM_014281.3 | 1658 | caggagcgttttgataacagtga | 66154 | - | see also 5589 line | | |
| 28200 | SIAHBP1 | NM_014281.3 | 407 | tacgtgggctctatctactatga | 66148 | - | see also 5589 line | | |
| 28201 | SIAHBP1 | NM_014281.3 | 1652 | tacgaccaggagcgttttgataa | 66152 | - | see also 5589 line | | |
| 28202 | SLM1 | NM_152688.1 | 1102 | ggctacgggggtgaatatgatga | 66158 | - | see also 12297 line | | |
| 28203 | SLM1 | NM_152688.1 | 1123 | gaccagacctatgagacttatga | 66160 | - | see also 12297 line | | |
| 28204 | SND1 | NM_014390.1 | 1360 | ctgcgtcccctgtatgacattcc | 66191 | - | see also 5650 line | | |
| 28205 | SND1 | NM_014390.1 | 274 | ctctcagggtgcgccatcattgt | 66162 | - | see also 5650 line | | |
| 28206 | SPAG7 | NM_004890.1 | 844 | gtcaccgttggagcttggatatg | 66227 | - | see also 3392 line | | |
| 28207 | SPAG7 | NM_004890.1 | 239 | gagaggagcatactacatgatgt | 66218 | - | see also 3392 line | | |
| 28208 | SPF45 | NM_032905.3 | 354 | ctcagatgaccggcaaattgtgg | 66232 | - | see also 9342 line | | |
| 28209 | SPF45 | NM_032905.3 | 460 | gacgaatatgaccctatgtttcc | 66234 | - | see also 9342 line | | |
| 28210 | SPF45 | NM_032905.3 | 727 | gagttaccccgagattttcctta | 66236 | - | see also 9342 line | | |
| 28211 | SRP46 | NM_032102.1 | 875 | ctcgctacagccgatatcacagc | 66248 | - | see also 12300 line | | |
| 28212 | SRrp35 | NM_080743.2 | 884 | atctcgaagttatcgtcataaaa | 66271 | - | see also 12301 line | | |
| 28213 | SRrp35 | NM_080743.2 | 886 | ctcgaagttatcgtcataaaaac | 66272 | - | see also 12301 line | | |
| 28214 | SSX1 | NM_005635.2 | 506 | ctgcacccccaggaaaagcaaa | 66274 | - | transcription_regulator | transcription co-repressor | soft tissue sarcoma、osteosarcoma、synovial sarcoma |
| 28215 | SSX1 | NM_005635.2 | 507 | tgcacccccaggaaaagcaaat | 66275 | - | see also 28214 line | | |

Figure 46

| | | | | | | | | | |
|---|---|---|---|---|---|---|---|---|---|
| 28216 | SSX2 | NM_003147.4 | 643 | cacaacattggtcgattcagttt | 66276 | - | gpcr、receptor_acitivity、transcription_regulator、signal_transduction | - | soft tissue sarcoma、synovial sarcoma |
| 28217 | SSX2 | NM_003147.4 | 653 | gtcgattcagtttgtcaacttct | 66277 | - | see also 28216 line | | |
| 28218 | SSX4 | NM_005636.3 | 299 | gggaatgattttggtaacgatcg | 66280 | - | see also 12302 line | | |
| 28219 | STK31 | NM_031414.2 | 2233 | agcagcaagcgtcctttggtacg | 66341 | - | see also 9015 line | | |
| 28220 | STK31 | NM_031414.2 | 383 | atcgatctgatatagttgaaatt | 66293 | - | see also 9015 line | | |
| 28221 | SZF1 | NM_016089.1 | 815 | gaggcgtcagacccttgttttgg | 66373 | - | see also 12304 line | | |
| 28222 | SZF1 | NM_016089.1 | 1209 | gtgtgggcgaggctttatagttg | 66381 | - | see also 12304 line | | |
| 28223 | SZFP41 | NM_152279.2 | 680 | gtcacagttcaaggtacatctga | 66390 | - | transcription_regulator | - | - |
| 28224 | SZFP41 | NM_152279.2 | 1092 | atccagaaaacacaattgattgc | 66392 | - | see also 28223 line | | |
| 28225 | SZFP41 | NM_152279.2 | 841 | ttcaagtatcgtctcttttcagg | 66391 | - | see also 28223 line | | |
| 28226 | T54 | NM_015698.3 | 557 | ggccgcaccttcaatcaagtagt | 66396 | - | see also 12305 line | | |
| 28227 | T54 | NM_015698.3 | 559 | ccgcaccttcaatcaagtagtga | 66397 | - | see also 12305 line | | |
| 28228 | TDRD1 | NM_198795.1 | 1576 | aacattgtcgtagacaaaagtga | 66452 | - | see also 12306 line | | |
| 28229 | TDRD1 | NM_198795.1 | 954 | gggtacggttaccgaattcaaac | 66428 | - | see also 12306 line | | |
| 28230 | TDRD3 | NM_030794.1 | 887 | agcatcgaggcaagctcttatgg | 66545 | - | see also 8950 line | | |
| 28231 | TDRD3 | NM_030794.1 | 1391 | tagaccgtattctagatatgaca | 66559 | - | see also 8950 line | | |
| 28232 | TDRD4 | NM_019038.2 | 2518 | ttcatatgccgttggtagaaatg | 66655 | - | see also 12308 line | | |
| 28233 | TDRD4 | NM_019038.2 | 1473 | ggcgctgtcagagtacaatattt | 66626 | - | see also 12308 line | | |
| 28234 | TDRKH | NM_006862.2 | 603 | gagacaattcgttctatctgtaa | 66671 | - | see also 12309 line | | |
| 28235 | TDRKH | NM_006862.2 | 1956 | tccgtagggaacctttactcttt | 66710 | - | see also 12309 line | | |
| 28236 | TFIP11 | NM_012143.1 | 2314 | tgggttggaagtcgatgttctca | 66743 | - | see also 12310 line | | |
| 28237 | TFIP11 | NM_012143.1 | 1598 | cacttatggcaccgagatcatct | 66724 | - | see also 12310 line | | |
| 28238 | THOC4 | NM_005782.1 | 54 | gtctctggacgacatcattaaac | 66749 | - | see also 12311 line | | |
| 28239 | THOC4 | NM_005782.1 | 285 | gtggcagcacgatcttttcgaca | 66752 | - | see also 12311 line | | |
| 28240 | TIPARP | NM_015508.2 | 1329 | tcccgagtctgtcattcgattga | 66792 | - | see also 6442 line | | |
| 28241 | TIPARP | NM_015508.2 | 1247 | caccaccctctagcaatgtcaac | 66786 | - | see also 6442 line | | |
| 28242 | TIPARP | NM_015508.2 | 1807 | ggccgtgacaggataataaatga | 66807 | - | see also 6442 line | | |
| 28243 | TIZ | NM_138330.1 | 439 | ttcagttaaaaggctgtaaaagt | 66815 | - | transcription_regulator、kinase_related、signal_transduction | - | - |
| 28244 | TNRC6 | NM_014494.1 | 5326 | gccggaccgatctcaatcactgg | 66925 | - | see also 5711 line | | |
| 28245 | TNRC6 | NM_014494.1 | 5106 | ctggtccgctacagttcaaaaga | 66918 | - | see also 5711 line | | |
| 28246 | TNRC6 | NM_014494.1 | 5322 | tccagccggaccgatctcaatca | 66923 | - | see also 5711 line | | |

1047

Figure 46

| 28247 | TOE1 | NM_025077.2 | 1728 | aggtggctgccgatgaaactagg | 66970 | - | see also 12314 line |
|---|---|---|---|---|---|---|---|
| 28248 | TOE1 | NM_025077.2 | 1142 | gtgctacacaatggccttataga | 66956 | - | see also 12314 line |
| 28249 | TRAF6 | NM_004620.2 | 493 | aacgccatgcggccataggttct | 66988 | - | see also 3136 line |
| 28250 | TRAF6 | NM_004620.2 | 1233 | agctcaaacgaaccattcgaacc | 67019 | - | see also 3136 line |
| 28251 | TRAF6 | NM_004620.2 | 320 | agctcctgtagcgctgtaacaaa | 66979 | - | see also 3136 line |
| 28252 | TRF4-2 | NM_022447.1 | 432 | tggacgacacttcaattatttaa | 67040 | - | see also 12316 line |
| 28253 | TRF4-2 | NM_022447.1 | 623 | ttgattatgcctacgttgttttg | 67044 | - | see also 12316 line |
| 28254 | TRIM56 | NM_030961.1 | 1926 | cgctagcgcacggctctatctca | 67057 | - | see also 12317 line |
| 28255 | TRIM56 | NM_030961.1 | 891 | ggccggtgtggacaataacctgg | 67050 | - | see also 12317 line |
| 28256 | U1SNRNPBP | NM_007020.2 | 1125 | gacgggaccggccttttcgaaaa | 67073 | - | see also 12318 line |
| 28257 | U1SNRNPBP | NM_007020.2 | 1164 | ttgttaaaaacgacctctataga | 67076 | - | see also 12318 line |
| 28258 | U2AF1L3 | NM_144987.1 | 278 | ggccgtggctgaactcagtaacc | 67084 | - | see also 9762 line |
| 28259 | U2AF1L3 | NM_144987.1 | 233 | gggcaacgtctatgtcaagttcc | 67083 | - | see also 9762 line |
| 28260 | UNKL | NM_024023.1 | 493 | ggctcgcagtctcgagaatgtgt | 67086 | - | see also 12319 line |
| 28261 | UNKL | NM_024023.1 | 1273 | cacagccgcaccataaatcctga | 67096 | - | see also 12319 line |
| 28262 | USP6 | NM_004505.1 | 2656 | ggcacgtctgcggaaccaattct | 67112 | - | see also 12320 line |
| 28263 | USP6 | NM_004505.1 | 1737 | agcggaaggacatacttatgaag | 67105 | - | see also 12320 line |
| 28264 | ZC3HAV1 | NM_020119.3 | 2278 | ttcaaacgtcgactcttcatacc | 67202 | - | see also 12321 line |
| 28265 | ZC3HAV1 | NM_020119.3 | 683 | tcgcagtccgagcggaatttatg | 67151 | - | see also 12321 line |
| 28266 | ZC3HDC1 | NM_022750.1 | 1387 | gccgtatcgatggcaattcttgg | 67249 | - | see also 12322 line |
| 28267 | ZC3HDC1 | NM_022750.1 | 1386 | tgccgtatcgatggcaattcttg | 67248 | - | see also 12322 line |
| 28268 | ZCCHC2 | NM_017742.2 | 574 | gactccctacacagtatcaataa | 67278 | - | see also 12323 line |
| 28269 | ZCCHC2 | NM_017742.2 | 2681 | ccctcccccttctaatgatacg | 67322 | - | see also 12323 line |
| 28270 | ZCCHC5 | NM_152694.1 | 1056 | ttcctggttcagctgtatagtta | 67338 | - | see also 12324 line |
| 28271 | ZCCHC5 | NM_152694.1 | 1391 | tgccagttctatacaagatgaac | 67350 | - | see also 12324 line |
| 28272 | ZNF183 | NM_006978.1 | 664 | atctatcggggaatcaacaatta | 67369 | - | see also 4871 line |
| 28273 | ZNF183 | NM_006978.1 | 417 | gacccgtgacagtggtaaacaga | 67362 | - | see also 4871 line |
| 28274 | ZNF183L1 | NM_178861.3 | 723 | aagagggtcgctactgtatctgc | 67379 | - | see also 12325 line |
| 28275 | ZNF183L1 | NM_178861.3 | 692 | aagctcgggtgggagattgaacg | 67377 | - | see also 12325 line |
| 28276 | ZNF2 | NM_021088.1 | 1355 | tactcgccatcagctaatccaca | 67383 | - | see also 12326 line |
| 28277 | ZNF2 | NM_021088.1 | 935 | agccctgtccagggaaattctca | 67380 | - | see also 12326 line |
| 28278 | ZNF227 | NM_182490.1 | 1367 | ggcttcggttggagtgttaatct | 67409 | - | see also 12327 line |
| 28279 | ZNF227 | NM_182490.1 | 1366 | gggcttcggttggagtgttaatc | 67408 | - | see also 12327 line |
| 28280 | ZNF248 | NM_021045.1 | 949 | ttcgggcttaattattagtaaaa | 67426 | - | see also 12328 line |
| 28281 | ZNF248 | NM_021045.1 | 1467 | ttctccgtgaatataaagtgagt | 67436 | - | see also 12328 line |

Figure 46

| | | | | | | | |
|---|---|---|---|---|---|---|---|
| 28282 | ZNF350 | NM_021632.2 | 1459 | gtcgtgtctggttaagcataaga | 67455 | - | see also 12329 line |
| 28283 | ZNF350 | NM_021632.2 | 726 | aacgacttcatactgcaattaaa | 67448 | - | see also 12329 line |
| 28284 | ZNF354B | NM_058230.1 | 611 | ttcagttgcccatacaaaaatcc | 67462 | - | see also 12330 line |
| 28285 | ZNF354B | NM_058230.1 | 1734 | gagaaaccatatgatgtttaga | 67469 | - | see also 12330 line |
| 28286 | ZNF354C | NM_014594.1 | 349 | ttcagataccgtctagtttca | 67474 | - | see also 12331 line |
| 28287 | ZNF354C | NM_014594.1 | 357 | ccgtctaggtttcaagaacttgg | 67475 | - | see also 12331 line |
| 28288 | ZNF415 | NM_018355.2 | 797 | gactgtacgtcaggtaagtcatt | 67502 | - | see also 12332 line |
| 28289 | ZNF415 | NM_018355.2 | 245 | gtctcgtaactgtgtaatcaagg | 67495 | - | see also 12332 line |
| 28290 | ZNF415 | NM_018355.2 | 765 | tgcgacaaagccttgaatcatgg | 67501 | - | see also 12332 line |
| 28291 | ZNF426 | NM_024106.1 | 1551 | tgggtatccctccatgtcttaata | 67527 | - | see also 12333 line |
| 28292 | ZNF426 | NM_024106.1 | 649 | cagacatccattggatacaatt | 67510 | - | see also 12333 line |
| 28293 | ZNF454 | NM_182594.1 | 1042 | ctcacttacgtaccatcagaaaa | 67538 | - | see also 12334 line |
| 28294 | ZNF454 | NM_182594.1 | 514 | ctgtcttgactggatgactatgc | 67531 | - | see also 12334 line |
| 28295 | ZNF471 | NM_020813.1 | 1112 | ttcagtgatggctcgtctttgc | 67548 | - | see also 12335 line |
| 28296 | ZNF471 | NM_020813.1 | 228 | accctgctcagaagcgtttatac | 67539 | - | see also 12335 line |
| 28297 | ZNF514 | NM_032788.1 | 725 | cggttgtgatggccagttagaga | 67564 | - | see also 12336 line |
| 28298 | ZNF514 | NM_032788.1 | 891 | ctcatgggagaaggatcttataa | 67571 | - | see also 12336 line |
| 28299 | ZNF75A | NM_153028.1 | 660 | aaggatagtgccggaaaatctcc | 67573 | - | see also 12337 line |
| 28300 | ZNF75A | NM_153028.1 | 757 | cagcaggcgtcatatcaaaaaag | 67577 | - | see also 12337 line |
| 28301 | ZNF77 | NM_021217.1 | 211 | ttgcctccttgattgttacatt | 67584 | - | see also 12338 line |
| 28302 | ZNF77 | NM_021217.1 | 1257 | ttcggatgtcccacttactttag | 67596 | - | see also 12338 line |
| 28303 | ZNF92 | NM_007139.2 | 900 | accagttctgattcttaataaa | 67604 | - | see also 12339 line |
| 28304 | ZNF92 | NM_007139.2 | 430 | aactactgacagcaagatatttc | 67603 | - | see also 12339 line |
| 28305 | 13CDNA73 | NM_023037.1 | 7508 | atcatcgggaggcggtgatgatga | 67794 | - | see also 8545 line |
| 28306 | 13CDNA73 | NM_023037.1 | 732 | aacgcaagattcgtatcattag | 67614 | - | see also 8545 line |
| 28307 | 13CDNA73 | NM_023037.1 | 3653 | gccctagagcgggatactttagc | 67706 | - | see also 8545 line |
| 28308 | AAK1 | NM_014911.2 | 714 | aacgtgagtagcgggtgatgtatg | 67852 | - | see also 12341 line |
| 28309 | AAK1 | NM_014911.2 | 533 | ggcggaaggtgatttgctattg | 67845 | - | see also 12341 line |
| 28310 | AARSL | NM_020745.1 | 1561 | ctgcgaccagcggaagttatga | 67907 | - | see also 12342 line |
| 28311 | AARSL | NM_020745.1 | 1563 | gcgaccagcggaagttatgagt | 67908 | - | see also 12342 line |
| 28312 | ABCA1 | NM_005502.2 | 5676 | tggagctgttcacgacaataag | 68015 | - | see also 3842 line |
| 28313 | ABCA1 | NM_005502.2 | 4844 | aagacgtatgtgcagatcatagc | 67995 | - | see also 3842 line |
| 28314 | ABCA10 | NM_080282.2 | 1870 | gagcgagtttacctgataaaga | 68067 | - | see also 12344 line |
| 28315 | ABCA10 | NM_080282.2 | 1011 | ctcatatcgcctgaagtttaatt | 68046 | - | see also 12344 line |

Figure 46

| 28316 | ABCA10 | NM_080282.2 | 2606 | accgacttatcctcttcagtacc | 68077 | - | see also 12344 line |
|---|---|---|---|---|---|---|---|
| 28317 | ABCA12 | NM_015657.3 | 4144 | atcgggtgctacggcatttcaga | 68239 | - | see also 6534 line |
| 28318 | ABCA12 | NM_015657.3 | 5813 | tgcgactcttaatcaacgaatcc | 68295 | - | see also 6534 line |
| 28319 | ABCA13 | NM_152701.2 | 13711 | cagttcggacgtggctttcattt | 68717 | - | see also 12346 line |
| 28320 | ABCA13 | NM_152701.2 | 10696 | ctgccataccagcgacctattcc | 68658 | - | see also 12346 line |
| 28321 | ABCA2 | NM_001606.2 | 6277 | gggagacgccgacaatgacatgg | 68795 | - | see also 1067 line |
| 28322 | ABCA3 | NM_001089.1 | 2314 | caccagtggacgggcatacatca | 68814 | - | see also 12348 line |
| 28323 | ABCA3 | NM_001089.1 | 1014 | atcacctacggttcagttacaca | 68806 | - | see also 12348 line |
| 28324 | ABCA3 | NM_001089.1 | 5536 | ggcgaaggttttcggtattctgg | 68830 | - | see also 12348 line |
| 28325 | ABCA4 | NM_000350.1 | 2500 | gagtacctggttcgctttgaaga | 68886 | - | see also 291 line |
| 28326 | ABCA4 | NM_000350.1 | 4627 | cagcgcagcacggaaattctaca | 68905 | - | see also 291 line |
| 28327 | ABCA5 | NM_018672.2 | 3340 | taccgcctgagcatgtacataga | 69019 | - | see also 7544 line |
| 28328 | ABCA5 | NM_018672.2 | 4072 | ccccatagtgcggctttaaatgt | 69042 | - | see also 7544 line |
| 28329 | ABCA5 | NM_018672.2 | 4071 | tccccatagtgcggctttaaatg | 69041 | - | see also 7544 line |
| 28330 | ABCA6 | NM_080284.2 | 2551 | aactatcgaacaagatttcgaac | 69158 | - | see also 12351 line |
| 28331 | ABCA6 | NM_080284.2 | 4343 | aggctcgccatcgcaagattagt | 69200 | - | see also 12351 line |
| 28332 | ABCA7 | NM_019112.2 | 2811 | gacgagcacgtctggttctatgg | 69217 | - | see also 12352 line |
| 28333 | ABCA7 | NM_019112.2 | 6355 | agcacggcgtggaggactttttcc | 69223 | - | see also 12352 line |
| 28334 | ABCA8 | NM_007168.1 | 1308 | tccatcggacggctcaaatctca | 69253 | - | see also 4971 line |
| 28335 | ABCA8 | NM_007168.1 | 1807 | agcaagctgaccggagtttgtcc | 69269 | - | see also 4971 line |
| 28336 | ABCA9 | NM_080283.2 | 3588 | ttctcgatagttgctactgatct | 69416 | - | see also 9513 line |
| 28337 | ABCA9 | NM_080283.2 | 2262 | tggggcataggctaccatttaag | 69382 | - | see also 9513 line |
| 28338 | ABCB1 | NM_000927.2 | 2207 | gacgtcatcgctggtttcgatga | 69485 | - | see also 604 line |
| 28339 | ABCB1 | NM_000927.2 | 755 | atgaccaggtatgcctattatta | 69441 | - | see also 604 line |
| 28340 | ABCB10 | NM_012089.1 | 1475 | aacgaggggggtcatcttaaatga | 69528 | - | see also 12356 line |
| 28341 | ABCB10 | NM_012089.1 | 2112 | atcgtctgtccaccattaagaat | 69541 | - | see also 12356 line |
| 28342 | ABCB11 | NM_003742.2 | 3964 | atccagaacgcggatatcattgc | 69659 | - | see also 2554 line |
| 28343 | ABCB11 | NM_003742.2 | 135 | ctcagtaattcttcgaagtataa | 69543 | - | see also 2554 line |
| 28344 | ABCB4 | NM_000443.2 | 3086 | ctcgagctaacgtcaagatcttg | 69695 | - | see also 350 line |
| 28345 | ABCB4 | NM_000443.2 | 3084 | ttctcgagctaacgtcaagatct | 69694 | - | see also 350 line |
| 28346 | ABCB4 | NM_000443.2 | 2389 | ctcaatacgcggctaacagatga | 69675 | - | see also 350 line |
| 28347 | ABCB5 | NM_178559.3 | 737 | tgctcgtgccttagttcgaaacc | 69763 | - | see also 12359 line |
| 28348 | ABCB5 | NM_178559.3 | 874 | accgactttctactattcgaagt | 69767 | - | see also 12359 line |
| 28349 | ABCB6 | NM_005689.1 | 1673 | tacaacgccgagagttacgaagt | 69831 | - | see also 3949 line |
| 28350 | ABCB6 | NM_005689.1 | 2037 | agggccgtattgagtttgagaac | 69840 | - | see also 3949 line |
| 28351 | ABCB7 | NM_004299.2 | 1979 | atcgttagattcgattactgaag | 69907 | - | see also 12361 line |
| 28352 | ABCB7 | NM_004299.2 | 2150 | tgctaaccctcatagtatctatt | 69914 | - | see also 12361 line |

Figure 46

| 28353 | ABCB7 | NM_004299.2 | 73 | cgctgctcgcgatgcattcttgg | 69848 | - | see also 12361 line |
|---|---|---|---|---|---|---|---|
| 28354 | ABCB8 | NM_007188.2 | 1993 | ggccgcacggtgctggtaattgc | 69944 | - | see also 12362 line |
| 28355 | ABCB8 | NM_007188.2 | 1417 | ctgcgtggctccgttacatttca | 69937 | - | see also 12362 line |
| 28356 | ABCB9 | NM_019624.1 | 2191 | tgccgagagcgagtatctgatcc | 69960 | - | see also 7732 line |
| 28357 | ABCB9 | NM_019624.1 | 358 | gaccacggccatctatgtcttca | 69946 | - | see also 7732 line |
| 28358 | ABCC1 | NM_004996.2 | 4336 | ttcgggttccctccgaatgaacc | 70003 | - | see also 3489 line |
| 28359 | ABCC1 | NM_004996.2 | 3814 | gggcaactgcatcgttctgtttg | 69994 | - | see also 3489 line |
| 28360 | ABCC10 | NM_033450.2 | 1711 | ggcgactccgggtcatcaaatac | 70018 | - | see also 9427 line |
| 28361 | ABCC10 | NM_033450.2 | 1524 | ctgctggtacccgtcaacaaagt | 70012 | - | see also 9427 line |
| 28362 | ABCC11 | NM_032583.2 | 397 | agcttacggagtcgcttagatga | 70044 | - | see also 12366 line |
| 28363 | ABCC11 | NM_032583.2 | 613 | ctcgggccaatattgattatacc | 70052 | - | see also 12366 line |
| 28364 | ABCC11 | NM_032583.2 | 3052 | aactatagccggtctcctttatt | 70088 | - | see also 12366 line |
| 28365 | ABCC12 | NM_033226.1 | 1096 | ttgtgcggcgtacgcctttttca | 70125 | - | see also 9384 line |
| 28366 | ABCC12 | NM_033226.1 | 2422 | agggcgctatgcaaaactgattc | 70159 | - | see also 9384 line |
| 28367 | ABCC13 | NM_138726.2 | 601 | aaccatctctactctatgcattg | 70208 | - | see also 12368 line |
| 28368 | ABCC13 | NM_138726.2 | 687 | tccttcactagcccactcataat | 70212 | - | see also 12368 line |
| 28369 | ABCC2 | NM_000392.1 | 4095 | ctgccggtggtcagattatcatt | 70307 | - | see also 311 line |
| 28370 | ABCC2 | NM_000392.1 | 4096 | tgccggtggtcagattatcattg | 70308 | - | see also 311 line |
| 28371 | ABCC3 | NM_003786.2 | 1159 | gtcgctgatcttacaacactatt | 70328 | - | see also 12370 line |
| 28372 | ABCC3 | NM_003786.2 | 4508 | atcgcacaccggcttaacactat | 70364 | - | see also 12370 line |
| 28373 | ABCC4 | NM_005845.2 | 2622 | tgccgctgacgtttttagatttc | 70447 | - | see also 12371 line |
| 28374 | ABCC4 | NM_005845.2 | 4056 | agccctcgaccttaactattttc | 70492 | - | see also 12371 line |
| 28375 | ABCC4 | NM_005845.2 | 4060 | ctcgaccttaactattttcgaga | 70493 | - | see also 12371 line |
| 28376 | ABCC5 | NM_005688.1 | 4297 | ttctaggctccgataggattatg | 70574 | - | see also 3945 line |
| 28377 | ABCC5 | NM_005688.1 | 1284 | gaggagcgtcggatattggaaaa | 70513 | - | see also 3945 line |
| 28378 | ABCC5 | NM_005688.1 | 3464 | ggggctgatgatcgttcttatgc | 70549 | - | see also 3945 line |
| 28379 | ABCC6 | NM_001171.2 | 3471 | aacaatgctcgcgtagatgaaag | 70600 | - | see also 12373 line |
| 28380 | ABCC6 | NM_001171.2 | 2680 | gacgcgagaggtccatcaagtca | 70596 | - | see also 12373 line |
| 28381 | ABCC8 | NM_000352.2 | 4788 | tgccatccttgagttcgataagc | 70640 | - | see also 12374 line |
| 28382 | ABCC8 | NM_000352.2 | 4787 | gtgccatccttgagttcgataag | 70639 | - | see also 12374 line |
| 28383 | ABCC9 | NM_005691.1 | 3087 | agctgatcagacctactatgtgg | 70730 | - | see also 3953 line |
| 28384 | ABCC9 | NM_005691.1 | 1189 | aggctctctacgtctaacttatc | 70670 | - | see also 3953 line |
| 28385 | ABCD1 | NM_000033.2 | 698 | caccttcctgtcggtgtatgtgg | 70782 | - | see also 5 line |
| 28386 | ABCD1 | NM_000033.2 | 1576 | atgccattgagcggatcatgtcg | 70787 | - | see also 5 line |
| 28387 | ABCD2 | NM_005164.2 | 1814 | cactgctcgagtgtacaatatgt | 70844 | - | see also 3628 line |
| 28388 | ABCD2 | NM_005164.2 | 1812 | tacactgctcgagtgtacaatat | 70843 | - | see also 3628 line |
| 28389 | ABCD3 | NM_002858.2 | 682 | aagttaacgagtgcaattggagc | 70904 | - | see also 1891 line |

Figure 46

| 28390 | ABCD4 | NM_005050.1 | 552 | ccggttcacctcgtctactaca | 70958 | - | see also 3525 line |
|---|---|---|---|---|---|---|---|
| 28391 | ABCD4 | NM_005050.1 | 1647 | ctcctttgcccgactcttctacc | 70968 | - | see also 3525 line |
| 28392 | ABCD4 | NM_005050.1 | 90 | gcccaggttagatctgcaatttc | 70947 | - | see also 3525 line |
| 28393 | ABCE1 | NM_002940.1 | 318 | tgggcgccttatcaattgtcaatc | 70988 | - | see also 1974 line |
| 28394 | ABCE1 | NM_002940.1 | 311 | gcccctttggcgccttatcaatt | 70984 | - | see also 1974 line |
| 28395 | ABCF2 | NM_005692.3 | 1334 | atgggccttgcatctacaataat | 71030 | - | see also 3956 line |
| 28396 | ABCF2 | NM_005692.3 | 1740 | caccaatcaactgatatcgaga | 71043 | - | see also 3956 line |
| 28397 | ABCF3 | NM_018358.1 | 113 | ggggacttgcgccgaaatcctgc | 71045 | - | see also 7412 line |
| 28398 | ABCF3 | NM_018358.1 | 2099 | gtcccacgatgagcgcttatca | 71072 | - | see also 7412 line |
| 28399 | ABCG1 | NM_004915.2 | 1796 | tacagttgcatgtcctacatctcc | 71087 | - | see also 12382 line |
| 28400 | ABCG1 | NM_004915.2 | 883 | gtcctgagtcaaggacaatgtgt | 71080 | - | see also 12382 line |
| 28401 | ABCG2 | NM_004827.1 | 1411 | gtcgtactgggactggttatagg | 71121 | - | see also 12383 line |
| 28402 | ABCG2 | NM_004827.1 | 641 | ttcggcttgcaacaactatgacg | 71100 | - | see also 12383 line |
| 28403 | ABCG2 | NM_004827.1 | 1912 | tacttcagcattccacgatatgg | 71136 | - | see also 12383 line |
| 28404 | ABCG4 | NM_022169.2 | 227 | gggcaaaaggggggttataagacc | 71150 | - | see also 8326 line |
| 28405 | ABCG4 | NM_022169.2 | 654 | ccgcctgcatgttctttgatg | 71156 | - | see also 8326 line |
| 28406 | ABCG5 | NM_022436.2 | 685 | accgactgattggcaactacagc | 71162 | - | see also 12385 line |
| 28407 | ABCG5 | NM_022436.2 | 1623 | gaggttgcccgatttggatattt | 71179 | - | see also 12385 line |
| 28408 | ABCG8 | NM_022437.2 | 1873 | ttcctgcggtggtttttgaagg | 71221 | - | see also 8356 line |
| 28409 | ABCG8 | NM_022437.2 | 1954 | atgccggtctcaggagataaaat | 71222 | - | see also 8356 line |
| 28410 | ABL2 | NM_005158.2 | 2815 | gaccgaccccgacgggtaaaacc | 71286 | - | see also 12387 line |
| 28411 | ABL2 | NM_005158.2 | 2819 | gaccccgacggtaaaaccaaag | 71287 | - | see also 12387 line |
| 28412 | ACACB | NM_001093.1 | 3523 | cactacgacaagtgtgtgataaa | 71499 | - | see also 12388 line |
| 28413 | ACACB | NM_001093.1 | 5843 | aagagatcgtcaccattagcttg | 71527 | - | see also 12388 line |
| 28414 | ACK1 | NM_005781.2 | 830 | aacgcaagtcgtggagtgagtaag | 71554 | - | see also 12389 line |
| 28415 | ACK1 | NM_005781.2 | 1345 | ctggagtccaagcgcttattca | 71556 | - | see also 12389 line |
| 28416 | ACLY | NM_001096.2 | 1229 | ggcatcgtgagagcaattcgaga | 71580 | - | see also 682 line |
| 28417 | ACLY | NM_001096.2 | 1237 | gagagcaattcgagattaccagg | 71581 | - | see also 682 line |
| 28418 | ACVR1 | NM_001105.2 | 890 | ttggatcattcgtgtacatcagg | 71613 | - | see also 686 line |
| 28419 | ACVR1 | NM_001105.2 | 1602 | atggtatagtggaggattacaag | 71625 | - | see also 686 line |
| 28420 | ACVR1B | NM_004302.3 | 651 | ggccgaaccatcgtttacaag | 71641 | - | see also 12392 line |
| 28421 | ACVR1B | NM_004302.3 | 652 | gccgaaccatcgtttacaaga | 71642 | - | see also 12392 line |
| 28422 | ACVR1C | NM_145259.1 | 1146 | tggtctggcacacttcatatgg | 71676 | - | see also 12393 line |
| 28423 | ACVR1C | NM_145259.1 | 1656 | ccgcctaactgctcttcgtatta | 71683 | - | see also 12393 line |
| 28424 | ACVR2 | NM_001616.2 | 405 | atgatatcaactgctatgacagg | 71699 | - | see also 1070 line |
| 28425 | ACVR2 | NM_001616.2 | 555 | cacctaagccaccctattacaac | 71708 | - | see also 1070 line |
| 28426 | ACVR2B | NM_001106.2 | 1512 | tggacctgcccctaaagagtca | 71753 | - | see also 687 line |

Figure 46

| ID | Gene | Accession | | Sequence | | | Notes |
|---|---|---|---|---|---|---|---|
| 28427 | ACVR2B | NM_001106.2 | 1307 | aagatgaggcccaccattaaga | 71750 | - | see also 687 line |
| 28428 | ACVRL1 | NM_000020.1 | 1722 | caccgcgctcggatcaagaaga | 71763 | - | see also 12396 line |
| 28429 | ACVRL1 | NM_000020.1 | 1665 | ctcaggcctagctcagatgatgc | 71762 | - | see also 12396 line |
| 28430 | ADRBK1 | NM_001619.2 | 656 | ttcagcgtgcatcgtcatcattgg | 71768 | - | see also 12397 line |
| 28431 | ADRBK1 | NM_001619.2 | 402 | gggagatcttcgactcatacatc | 71766 | - | see also 12397 line |
| 28432 | ADRBK2 | NM_005160.2 | 1787 | gttggcagcgtcgctattttacc | 71818 | - | see also 3627 line |
| 28433 | ADRBK2 | NM_005160.2 | 1785 | cagttgcagcgtcgctatttta | 71817 | - | see also 3627 line |
| 28434 | ADRBK2 | NM_005160.2 | 1841 | gggagagtcccgccaaaatttac | 71820 | - | see also 3627 line |
| 28435 | AFG3L1 | NM_001132.1 | 623 | ctggaagtcgtgaacaaacaatc | 71827 | - | see also 12399 line |
| 28436 | AFG3L1 | NM_001132.1 | 472 | ccctgggatgacgaggatttcc | 71824 | - | see also 12399 line |
| 28437 | AFURS1 | NM_024524.2 | 3347 | ggctattccgttaaattggtcc | 71877 | - | see also 12400 line |
| 28438 | AFURS1 | NM_024524.2 | 2663 | aacagcacttcataatcgaatta | 71856 | - | see also 12400 line |
| 28439 | AK1 | NM_000476.1 | 517 | agcggctggagacctattacaag | 71895 | - | see also 12401 line |
| 28440 | AK1 | NM_000476.1 | 312 | atgtgtggccaaagtcaatacttc | 71892 | - | see also 12401 line |
| 28441 | AK3L1 | NM_016282.2 | 534 | ccgccagttggccgagtctataa | 71912 | - | see also 6779 line |
| 28442 | AK3L1 | NM_016282.2 | 440 | agccctagatagagcttatcaga | 71907 | - | see also 6779 line |
| 28443 | AK5 | NM_012093.2 | 2533 | atcgcctacagagacaaaaac | 71954 | - | see also 12403 line |
| 28444 | AK5 | NM_012093.2 | 1733 | gccgaccagaacgacaaatgtaaaa | 71937 | - | see also 12403 line |
| | | | | | | | kinase_related, apoptosis, signal_transduction, gpcr — receptor signaling protein serine/threonine kinase — schizophrenia |
| 28445 | AKT1 | NM_005163.1 | 239 | aacgagggagtacatcaagacc | 71961 | - | |
| 28446 | AKT1 | NM_005163.1 | 1402 | atcatgcagcatcgtcttttgc | 71963 | - | see also 28445 line |
| 28447 | AKT1 | NM_005163.1 | 949 | cgcttctatgcgctgagattgt | 71962 | - | see also 28445 line |
| 28448 | AKT3 | NM_005465.3 | 310 | ctccagtggactactgttataga | 71981 | - | see also 3804 line |
| 28449 | AKT3 | NM_005465.3 | 176 | atggctcattcataggattataaa | 71978 | - | see also 3804 line |
| 28450 | ALS2CR2 | NM_018571.4 | 1216 | ctccttatagcccattggatatc | 72019 | - | see also 7527 line |
| 28451 | ALS2CR2 | NM_018571.4 | 395 | aagaacacaagttgaatcactca | 72014 | - | see also 7527 line |
| 28452 | ALS2CR7 | NM_139158.1 | 934 | ctccaagctacctaactacaatc | 72042 | - | see also 12406 line |
| 28453 | ALS2CR7 | NM_139158.1 | 976 | tacgcctgaagccttcatgttg | 72044 | - | see also 12406 line |
| 28454 | AMHR2 | NM_020547.1 | 1287 | ggccctcgacgacgagctgatattt | 72063 | - | see also 7900 line |
| 28455 | AMHR2 | NM_020547.1 | 584 | tgctacagcgaaagaactacaga | 72055 | - | see also 7900 line |
| 28456 | AMHR2 | NM_020547.1 | 1288 | gccctccgacgagctgatattta | 72064 | - | see also 7900 line |
| 28457 | ANKK1 | NM_178510.1 | 1123 | ctccgaccgtaagaatttggtcc | 72076 | - | see also 12408 line |
| 28458 | ANKK1 | NM_178510.1 | 1119 | agctctccgaccgtaagaatttg | 72075 | - | see also 12408 line |
| 28459 | ANKRD3 | NM_020639.1 | 506 | cccactaccacgtcaagatttct | 72086 | - | see also 12409 line |
| 28460 | ANKRD3 | NM_020639.1 | 1011 | ctctgccccaacttcgataacg | 72090 | - | see also 12409 line |

1053

Figure 46

| | | | | | | | |
|---|---|---|---|---|---|---|---|
| 28461 | ANKRD3 | NM_020639.1 | 894 | aaccgaggacctgtgtgaaaagc | 72088 | - | see also 12409 line |
| 28462 | APAF1 | NM_001160.2 | 775 | ttcctacgtatcattctacaatg | 72103 | - | see also 734 line |
| 28463 | APAF1 | NM_001160.2 | 771 | atgattcctacgtatcattctac | 72102 | - | see also 734 line |
| 28464 | APG-1 | NM_014278.2 | 1729 | ggctagcgcatcagtaattgaga | 72268 | - | see also 5577 line |
| 28465 | APG-1 | NM_014278.2 | 1033 | tcgggccttgttgcgtttatatc | 72239 | - | see also 5577 line |
| 28466 | ARAF1 | NM_001654.1 | 483 | tcccgctgaccatgcacaatttt | 72295 | - | see also 1094 line |
| 28467 | ARAF1 | NM_001654.1 | 347 | ctgaaggtgcggggtctaaatca | 72294 | - | see also 1094 line |
| 28468 | ARK5 | NM_014840.1 | 1610 | ctggccgagtggttgctataaaa | 72308 | - | see also 12413 line |
| 28469 | ARK5 | NM_014840.1 | 2142 | tggtttcgatcacaaaaacctca | 72325 | - | see also 12413 line |
| 28470 | ASNA1 | NM_004317.1 | 825 | gtgtgaggcccgtcacaagatcc | 72350 | - | see also 12414 line |
| 28471 | ASNA1 | NM_004317.1 | 17 | agccgctggagcctacacttagc | 72339 | - | see also 12414 line |
| 28472 | ASS | NM_000050.2 | 877 | atcgtggagaaccgcttcattgg | 72353 | - | see also 12415 line |
| 28473 | ASS | NM_000050.2 | 1004 | aacaaggcctgggcttgaaattt | 72355 | - | see also 12415 line |
| 28474 | ATP10A | NM_024490.2 | 1017 | acgcagtcgtcggcattgtcatc | 72361 | - | see also 8619 line |
| 28475 | ATP10A | NM_024490.2 | 1187 | ctgtggatatggcggtatcaaga | 72364 | - | see also 8619 line |
| 28476 | ATP10D | NM_020453.2 | 385 | atcgaatacgaacaacaaagtac | 72424 | - | see also 12417 line |
| 28477 | ATP10D | NM_020453.2 | 2349 | gacgaagcggccttagtgtatgc | 72470 | - | see also 12417 line |
| 28478 | ATP10D | NM_020453.2 | 1085 | acggtataagcgcagcaaattag | 72442 | - | see also 12417 line |
| 28479 | ATP12A | NM_001676.2 | 2782 | gccgctcgctgtgtactcatacc | 72560 | - | see also 12418 line |
| 28480 | ATP12A | NM_001676.2 | 1496 | gcctccttatccaagataataac | 72536 | - | see also 12418 line |
| 28481 | ATP1A1 | NM_000701.5 | 1008 | caccgcacgtggtattgttgtct | 72597 | - | see also 12419 line |
| 28482 | ATP1A1 | NM_000701.5 | 1011 | cgcacgtggtattgttgtctaca | 72598 | - | see also 12419 line |
| 28483 | ATP1A2 | NM_000702.1 | 1944 | accggggatcaccctatcacagc | 72638 | - | see also 431 line |
| 28484 | ATP1A2 | NM_000702.1 | 935 | tgggcggacacccatagcaatgg | 72630 | - | see also 431 line |
| 28485 | ATP1A3 | NM_152296.2 | 1496 | gcgtgaacgcaacaagaaagtgg | 72648 | - | see also 9834 line |
| 28486 | ATP1A3 | NM_152296.2 | 3053 | ctgtgccttcccctacagtttcc | 72651 | - | see also 9834 line |
| 28487 | ATP1A4 | NM_144699.1 | 3208 | acctatgagcaacgaaaagttgt | 72676 | - | see also 12422 line |
| 28488 | ATP1A4 | NM_144699.1 | 2188 | aagggattcccatttaatacaga | 72666 | - | see also 12422 line |
| 28489 | ATP2A1 | NM_004320.2 | 1528 | ctcggtgatccgccagctaatga | 72698 | - | see also 2888 line |
| 28490 | ATP2A1 | NM_004320.2 | 2153 | ccctcgcacaagtccaagattgt | 72703 | - | see also 2888 line |
| 28491 | ATP2A2 | NM_001681.2 | 2836 | atccccatacccgatgacaatgg | 72769 | - | see also 1102 line |
| 28492 | ATP2A2 | NM_001681.2 | 639 | ttcctgctgatataaggttaact | 72720 | - | see also 1102 line |
| 28493 | ATP2A3 | NM_005173.2 | 2855 | tccgtgctcgtgaccattgaaat | 72795 | - | see also 12424 line |
| 28494 | ATP2A3 | NM_005173.2 | 1548 | agcgagctggcgcctgtaacacg | 72787 | - | see also 12424 line |
| 28495 | ATP2A3 | NM_005173.2 | 683 | tccatcctgacgggtgaatctgt | 72781 | - | see also 12424 line |
| 28496 | ATP2B1 | NM_001682.1 | 1792 | acctcgtcacgttggtaataaaa | 72840 | - | see also 12425 line |
| 28497 | ATP2B1 | NM_001682.1 | 1791 | tacctcgtcacgttggtaataaa | 72839 | - | see also 12425 line |

Figure 46

| 28498 | ATP2B1 | NM_001682.1 | 852 | ttccagctgacggcatacttatt | 72813 | - | see also 12425 line |
|---|---|---|---|---|---|---|---|
| 28499 | ATP2B2 | NM_001683.1 | 3490 | cacggcgagcgcaatgtctttga | 72930 | - | see also 1104 line |
| 28500 | ATP2B3 | NM_021949.1 | 1342 | tgcacgccggtctatgtacaata | 72948 | - | see also 12426 line |
| 28501 | ATP2B3 | NM_021949.1 | 1344 | cacgccggtctatgtacaatact | 72949 | - | see also 12426 line |
| 28502 | ATP2C1 | NM_014382.1 | 553 | cactgtggcaatacttatcgttg | 72976 | - | see also 5642 line |
| 28503 | ATP2C1 | NM_014382.1 | 2625 | agctacgagacaatgtgattaca | 73039 | - | see also 5642 line |
| 28504 | ATP4A | NM_000704.1 | 574 | gtcatccgcgatggagacaaatt | 73059 | - | see also 433 line |
| 28505 | ATP4A | NM_000704.1 | 500 | ttggctactaccaggaattcaag | 73057 | - | see also 433 line |
| 28506 | ATP5A1 | NM_004046.3 | 1268 | atccgccctgcaattaacgttgg | 73109 | - | see also 12429 line |
| 28507 | ATP5A1 | NM_004046.3 | 1264 | aggtatccgccctgcaattaacg | 73108 | - | see also 12429 line |
| 28508 | ATP5B | NM_001686.2 | 1370 | gtgtcccgtgcacggaaaataca | 73141 | - | see also 12430 line |
| 28509 | ATP5B | NM_001686.2 | 796 | ctctaaggtagcgctggtatatg | 73126 | - | see also 12430 line |
| 28510 | ATP6AP1 | NM_001183.3 | 652 | tccgaagatgtcccatacacagc | 73148 | - | see also 12431 line |
| 28511 | ATP6AP1 | NM_001183.3 | 1369 | aagaccatggatcgctttgatga | 73158 | - | see also 12431 line |
| 28512 | ATP6V1B1 | NM_001692.2 | 146 | ggcggtcacccgaaactacatca | 73220 | - | see also 12433 line |
| 28513 | ATP6V1B1 | NM_001692.2 | 571 | ctcctattgacgtcatgaacagc | 73230 | - | see also 12433 line |
| 28514 | ATP6V1B2 | NM_001693.2 | 1476 | tgcgtgctatgctattggaaagg | 73279 | - | see also 1110 line |
| 28515 | ATP6V1B2 | NM_001693.2 | 1236 | gagaaacggctcgattactcaaa | 73270 | - | see also 1110 line |
| 28516 | ATP6V1C1 | NM_001695.2 | 595 | ctcgagcatctgcatacaataac | 73304 | - | see also 1111 line |
| 28517 | ATP6V1C1 | NM_001695.2 | 466 | gagtggacttggttacttatata | 73298 | - | see also 1111 line |
| 28518 | ATP6V1C2 | NM_144583.1 | 1090 | tcccgggactgcaactcaataac | 73352 | - | see also 9706 line |
| 28519 | ATP6V1C2 | NM_144583.1 | 504 | acccggacactgagtgatattgt | 73337 | - | see also 9706 line |
| 28520 | ATP6V1H | NM_015941.1 | 1569 | cagcaggtccgctataatgctct | 73387 | - | see also 6582 line |
| 28521 | ATP6V1H | NM_015941.1 | 1566 | gaccagcaggtccgctataatgc | 73386 | - | see also 6582 line |
| 28522 | ATP7A | NM_000052.1 | 4502 | ctggaccggattgttaattatag | 73515 | - | see also 21 line |
| 28523 | ATP7A | NM_000052.1 | 3080 | tcccgaacagaaacgataatacg | 73480 | - | see also 21 line |
| 28524 | ATP7B | NM_000053.1 | 4041 | ggcagccgacgtcgtccttatca | 73594 | - | see also 26 line |
| 28525 | ATP7B | NM_000053.1 | 4045 | gccgacgtcgtccttatcagaaa | 73596 | - | see also 26 line |
| 28526 | ATP8A1 | NM_006095.1 | 1575 | aacacccgactcggtgattatag | 73646 | - | see also 4236 line |
| 28527 | ATP8A1 | NM_006095.1 | 1272 | caccatagcgggagttgcttatg | 73638 | - | see also 4236 line |
| 28528 | ATP8A1 | NM_006095.1 | 1579 | cccgactcggtgattatagattc | 73648 | - | see also 4236 line |
| 28529 | ATP8A2 | NM_016529.3 | 2923 | gcgtcccgcatgggtatgctttt | 73775 | - | see also 6904 line |
| 28530 | ATP8A2 | NM_016529.3 | 552 | gagacgaaccttaaaatacgtca | 73714 | - | see also 6904 line |
| 28531 | ATP8B1 | NM_005603.1 | 484 | gacgatgtggctcgccataaaat | 73796 | - | see also 12441 line |
| 28532 | ATP8B1 | NM_005603.1 | 2931 | gactgcatacgaggattggttca | 73876 | - | see also 12441 line |
| 28533 | ATP8B1 | NM_005603.1 | 491 | tggctcgccataaaatggataag | 73799 | - | see also 12441 line |
| 28534 | ATSV | NM_004321.3 | 4855 | tccgagtcagcccgatcgtttcc | 73926 | - | see also 2893 line |

Figure 46

| | | | | | | | |
|---|---|---|---|---|---|---|---|
| 28535 | ATSV | NM_004321.3 | 3844 | atgaccggacctttaccaattt | 73916 | - | see also 2893 line |
| 28536 | ATSV | NM_004321.3 | 3944 | gacactctccgcttatatgaga | 73924 | - | see also 2893 line |
| 28537 | AURKB | NM_004217.1 | 261 | aacgcggcacttcacaattgatg | 73930 | - | see also 12443 line |
| 28538 | AURKB | NM_004217.1 | 242 | gtgggacacccgacatcttaacg | 73929 | - | see also 12443 line |
| 28539 | AURKC | NM_003160.1 | 557 | tcctgcgcctgtataactattc | 73942 | - | see also 12444 line |
| 28540 | AURKC | NM_003160.1 | 672 | gcgcacagccacgataatagaag | 73947 | - | see also 12444 line |
| 28541 | AXL | NM_001699.3 | 791 | cacgggacagtaccagtgtttg | 73953 | - | see also 1112 line |
| 28542 | BCKDK | NM_005881.1 | 640 | ctgcacgtgcatgagctatatat | 73977 | - | see also 4092 line |
| 28543 | BCS1L | NM_004328.3 | 532 | ggccgcatttccactaagtttga | 73988 | - | see also 2896 line |
| 28544 | BCS1L | NM_004328.3 | 819 | ccggcgaccactgaattctgtgg | 73996 | - | see also 2896 line |
| 28545 | BLK | NM_001715.2 | 1726 | ctcgaatcatcgacagtgaatac | 74017 | - | see also 12448 line |
| 28546 | BLK | NM_001715.2 | 783 | gactacaccgctatgaattgatcg | 74005 | - | see also 12448 line |
| 28547 | BLK | NM_001715.2 | 1806 | ttcggggtcttcaccatcaaagc | 74019 | - | see also 12448 line |
| 28548 | BMP2K | NM_017593.3 | 1015 | ttctcgttactccgtaacatac | 74050 | - | see also 7045 line |
| 28549 | BMP2K | NM_017593.3 | 1069 | tccggaacatagacctgatatat | 74057 | - | see also 7045 line |
| 28550 | BMPR1A | NM_004329.1 | 662 | tacgccggacaatagaatgttgt | 74104 | - | see also 2897 line |
| 28551 | BMPR1A | NM_004329.1 | 659 | agctacgccggacaatagaatgt | 74103 | - | see also 2897 line |
| 28552 | BMPR1B | NM_001203.1 | 1155 | caccctagacgctaaatcaatgc | 74137 | - | see also 12451 line |
| 28553 | BMPR1B | NM_001203.1 | 1099 | tacctaatcacagactatcatga | 74135 | - | see also 12451 line |
| 28554 | BMPR2 | NM_001204.4 | 1179 | tccgaaccctcttcttgatctaga | 74164 | - | see also 788 line |
| 28555 | BMPR2 | NM_001204.4 | 1722 | agcgagcttggcactatcagata | 74195 | - | see also 788 line |
| 28556 | BMS1L | NM_014753.1 | 2085 | tccaaacctccgaaagcttattt | 74255 | - | see also 12453 line |
| 28557 | BMS1L | NM_014753.1 | 2084 | ctccaaacctccgaaagcttatt | 74254 | - | see also 12453 line |
| 28558 | BMX | NM_001721.3 | 1097 | ttcgagccaagtgggaatgtaca | 74278 | - | see also 12454 line |
| 28559 | BMX | NM_001721.3 | 1829 | cagttcagtcgggaacaaagtttc | 74296 | - | see also 12454 line |
| 28560 | BRAF | NM_004333.2 | 1389 | gacgggactcgagtgatgattgg | 74340 | - | see also 12455 line |
| 28561 | BRAF | NM_004333.2 | 586 | tacagtccgagacagtctaaaga | 74312 | - | see also 12455 line |
| 28562 | BTK | NM_000061.1 | 1857 | caccggaagtcctgatgtatagc | 74399 | - | see also 36 line |
| 28563 | BTK | NM_000061.1 | 1855 | cccacggaagtcctgatgtata | 74398 | - | see also 36 line |
| 28564 | BUB1 | NM_004336.1 | 1793 | tggggtattcgtgcaacaaaac | 74435 | - | see also 2898 line |
| 28565 | BUB1 | NM_004336.1 | 672 | aacgaagagtgatcacgatttct | 74411 | - | see also 2898 line |
| 28566 | BUB1 | NM_004336.1 | 3251 | gccctacgtaataggctaattgt | 74477 | - | see also 2898 line |
| 28567 | BUB1B | NM_001211.3 | 2815 | tccacgatccctatgattgtaac | 74538 | - | see also 12458 line |
| 28568 | BUB1B | NM_001211.3 | 508 | ggcttaaattagggcgtttatgc | 74489 | - | see also 12458 line |
| 28569 | C14orf127 | NM_025152.1 | 487 | ttggggtcaactggactacttag | 74570 | - | see also 12459 line |
| 28570 | C14orf127 | NM_025152.1 | 488 | tggggtcaactggactacttagt | 74571 | - | see also 12459 line |
| 28571 | C14orf131 | NM_018335.1 | 908 | gcccacatggatcacattgact | 74595 | - | see also 12460 line |

Figure 46

| 28572 | C14orf131 | NM_018335.1 | 1107 | gacttccgatgggcttatcttgt | 74598 | - | see also 12460 line |
|---|---|---|---|---|---|---|---|
| 28573 | C14orf20 | NM_174944.1 | 1159 | accaatccttgcaaattacgacc | 74610 | - | see also 12461 line |
| 28574 | C14orf20 | NM_174944.1 | 1156 | agcaccaatccttgcaaattacg | 74609 | - | see also 12461 line |
| 28575 | C20orf23 | NM_017683.1 | 622 | aacgaacgtgtgagagatctact | 74623 | - | see also 12462 line |
| 28576 | C20orf23 | NM_017683.1 | 951 | caccggggttaggctaaaggaag | 74634 | - | see also 12462 line |
| 28577 | C20orf23 | NM_017683.1 | 644 | ttcggcggaagtcatctaaaacc | 74626 | - | see also 12462 line |
| 28578 | C20orf64 | NM_033550.2 | 695 | ggctcgaatgcacgatgaagacc | 74715 | - | see also 12463 line |
| 28579 | C20orf64 | NM_033550.2 | 662 | ctccaacttagccaagacaattg | 74714 | - | see also 12463 line |
| 28580 | C20orf64 | NM_033550.2 | 938 | ggccaggccagtgctaaaaaaat | 74719 | - | see also 12463 line |
| 28581 | C20orf97 | NM_021158.2 | 1210 | gcctacgtgggacctgagatact | 74723 | - | see also 12464 line |
| 28582 | C20orf97 | NM_021158.2 | 803 | gccctacaggcactgagtatacc | 74722 | - | see also 12464 line |
| 28583 | C6orf199 | NM_145025.2 | 211 | aacaacattagcccgttacataa | 74725 | - | see also 12465 line |
| 28584 | C6orf199 | NM_145025.2 | 952 | aagagcagctattctaaccaaac | 74742 | - | see also 12465 line |
| 28585 | C8FW | NM_025195.2 | 1615 | gggtacatcgactcagaaatagg | 74758 | - | see also 12466 line |
| 28586 | C8FW | NM_025195.2 | 1618 | tacatcgactcagaaataggaac | 74759 | - | see also 12466 line |
| 28587 | CACNA1B | NM_000718.1 | 3362 | gacccgaacactattgtacatat | 74777 | - | see also 435 line |
| 28588 | CACNA1B | NM_000718.1 | 1671 | ggcttaccacgaccctgtatttt | 74769 | - | see also 435 line |
| 28589 | CAD | NM_004341.2 | 5394 | ggggaggttgcctatatcgatgg | 74903 | - | see also 12468 line |
| 28590 | CAD | NM_004341.2 | 5226 | cacaatcctcggcgcatctttca | 74899 | - | see also 12468 line |
| 28591 | CAMK1 | NM_003656.3 | 1042 | gtcggtgagtgagcagatcaaga | 74925 | - | see also 12469 line |
| 28592 | CAMK1 | NM_003656.3 | 826 | tcccttctatgacgagaatgatg | 74919 | - | see also 12469 line |
| 28593 | CAMK1 | NM_003656.3 | 827 | cccttctatgacgagaatgatgc | 74920 | - | see also 12469 line |
| 28594 | CAMK1D | NM_020397.1 | 697 | tgctggtccatcggagtgattgc | 74931 | - | see also 12470 line |
| 28595 | CAMK1D | NM_020397.1 | 453 | cactctgatccgccaagtcttgg | 74929 | - | see also 12470 line |
| 28596 | CAMK1D | NM_020397.1 | 785 | tcctcaaggcggaatatgagttt | 74936 | - | see also 12470 line |
| 28597 | CAMK1G | NM_020439.1 | 695 | aacggagtctaagcttttcgaga | 74962 | - | see also 12471 line |
| 28598 | CAMK1G | NM_020439.1 | 701 | gtctaagcttttcgagaagatca | 74963 | - | see also 12471 line |
| 28599 | CAMK1G | NM_020439.1 | 691 | aagaaacggagtctaagcttttc | 74961 | - | see also 12471 line |
| 28600 | CAMK2A | NM_015981.2 | 356 | cccaacatcgtccgactacatga | 74969 | - | see also 6591 line |
| 28601 | CAMK2A | NM_015981.2 | 1351 | gggcctggacttccatcgattct | 74979 | - | see also 6591 line |
| 28602 | CAMK2B | NM_001220.3 | 2062 | atccggctcacgcagtacattga | 74996 | - | see also 800 line |
| 28603 | CAMK2B | NM_001220.3 | 1846 | gccgtcaacaacggtgactttga | 74992 | - | see also 800 line |
| 28604 | CAMK2B | NM_001220.3 | 244 | taccagctctacgaggatattgg | 74983 | - | see also 800 line |
| 28605 | CAMK2D | NM_001221.2 | 534 | cacggacgagtatcagcttttcg | 74997 | - | see also 801 line |
| 28606 | CAMK2D | NM_001221.2 | 1779 | tgccgcctgcatagcatatatta | 75021 | - | see also 801 line |
| 28607 | CAMK2G | NM_001222.2 | 1125 | gtggtacacaacgctacagatgg | 75042 | - | see also 803 line |
| 28608 | CAMK2G | NM_001222.2 | 1269 | gaggcctacacgaagatttgtga | 75044 | - | see also 803 line |

Figure 46

| 28609 | CAMK4 | NM_001744.3 | 834 | gaggcgatcagttcagttcagg | 75063 | - | see also 1154 line |
| 28610 | CAMK4 | NM_001744.3 | 893 | tggtgggatgaagtatctctaaa | 75070 | - | see also 1154 line |
| 28611 | CAMKK1 | NM_032294.2 | 1198 | gtgccattcatcgacgatttca | 75086 | - | see also 9146 line |
| 28612 | CAMKK1 | NM_032294.2 | 1322 | aagaatcccgagcgagagaattgg | 75091 | - | see also 9146 line |
| 28613 | CAMKK2 | NM_006549.3 | 2001 | tggacgagcggatcatgtgttta | 75108 | - | see also 12478 line |
| 28614 | CAMKK2 | NM_006549.3 | 1951 | ggccatgggtgacactatact | 75106 | - | see also 12478 line |
| 28615 | CARD12 | NM_021209.3 | 2036 | tcccgattacttagactic | 75156 | - | see also 12479 line |
| 28616 | CARD12 | NM_021209.3 | 280 | gacaatagccgagccctattca | 75113 | - | see also 12479 line |
| 28617 | CARD15 | NM_022162.1 | 2561 | ctctgtatttgcgcgataacaat | 75206 | - | see also 8318 line |
| 28618 | CARD15 | NM_022162.1 | 586 | ttgtcagccagtatgaagttga | 75188 | - | see also 8318 line |
| 28619 | CARD15 | NM_022162.1 | 2619 | tgctcttcactgcgagcaattgc | 75209 | - | see also 8318 line |
| 28620 | CARD4 | NM_006092.1 | 2740 | agccaggtacgtcaccaaaatcc | 75236 | - | see also 12481 line |
| 28621 | CARD4 | NM_006092.1 | 2668 | aagcgaagagctgaccaaataca | 75233 | - | see also 12481 line |
| 28622 | CARK | NM_015978.1 | 1520 | aacgttatcgagccaatacctac | 75294 | - | see also 12482 line |
| 28623 | CARK | NM_015978.1 | 1510 | gtggctataaaacgttatcgagc | 75293 | - | see also 12482 line |
| 28624 | CASK | NM_003688.1 | 863 | gggatcgttacgcctacaagatt | 75353 | - | see also 2506 line |
| 28625 | CASK | NM_003688.1 | 96 | cagtgttgracgacgatgtatca | 75316 | - | see also 2506 line |
| 28626 | CCRK | NM_012119.2 | 894 | tgccgactacacaacagatctcc | 75405 | - | see also 12484 line |
| 28627 | CCRK | NM_012119.2 | 888 | ctgagctgccggactacaacaag | 75404 | - | see also 12484 line |
| 28628 | CCT2 | NM_006431.1 | 125 | cagctcgtctgacttcttttatt | 75409 | - | see also 12485 line |
| 28629 | CCT2 | NM_006431.1 | 221 | gtggacgaatgcctctcttatg | 75410 | - | see also 12485 line |
| 28630 | CCT3 | NM_005998.2 | 1256 | atgcaagtgtgtcgcaatgttct | 75451 | - | see also 12486 line |
| 28631 | CCT3 | NM_005998.2 | 1026 | cagacaataatcgccattgctaga | 75447 | - | see also 12486 line |
| 28632 | CCT4 | NM_006430.2 | 1443 | ttggccctacgattaactgaata | 75505 | - | see also 4500 line |
| 28633 | CCT4 | NM_006430.2 | 1438 | tagagttggcctacgattaact | 75503 | - | see also 4500 line |
| 28634 | CCT5 | NM_012073.2 | 98 | accctgccttcgatgaatatgg | 75521 | - | see also 12488 line |
| 28635 | CCT5 | NM_012073.2 | 97 | gaccctcgccttcgatgaatatg | 75520 | - | see also 12488 line |
| 28636 | CCT6A | NM_001762.2 | 1530 | taggcgtatcggattaactatrgt | 75575 | - | see also 12489 line |
| 28637 | CCT6A | NM_001762.2 | 1532 | ggcgtatcggataaactattgtgt | 75576 | - | see also 12489 line |
| 28638 | CCT6B | NM_006584.1 | 1465 | aacttgtggccgtagatttgaat | 75607 | - | see also 12490 line |
| 28639 | CCT6B | NM_006584.1 | 1462 | aacaacttgtggccgtagatttg | 75606 | - | see also 12490 line |
| 28640 | CCT7 | NM_006429.1 | 1473 | gcccaggggggtacatggtatgg | 75652 | - | see also 12491 line |
| 28641 | CCT7 | NM_006429.1 | 193 | cccgttgcatggacaagcttatt | 75616 | - | see also 12491 line |
| 28642 | CCT8 | NM_006585.1 | 1546 | ggctatcaaactcgctactaatg | 75703 | - | see also 12492 line |
| 28643 | CCT8 | NM_006585.1 | 513 | ctctacttcgtacctccataatg | 75661 | - | see also 12492 line |
| 28644 | CCT8 | NM_006585.1 | 577 | tgctcaggcatgcgtatctattt | 75668 | - | see also 12492 line |
| 28645 | CDC2 | NM_001786.2 | 655 | ctggggtcagctcgttactcaac | 75722 | - | see also 12493 line |

Figure 46

| | | | | | | | |
|---|---|---|---|---|---|---|---|
| 28646 | CDC2 | NM_001786.2 | 968 | tggcactgaatcatccatatttt | 75737 | - | see also 12493 line |
| 28647 | CDC2L5 | NM_003718.2 | 2726 | aagaaagtcggccgtatactaac | 75778 | - | see also 12494 line |
| 28648 | CDC2L5 | NM_003718.2 | 2736 | gccgtatactaacaaggtaatta | 75781 | - | see also 12494 line |
| 28649 | CDC42BPA | NM_003607.2 | 4600 | agccgcaatcatagatcatgaaa | 75940 | - | see also 12495 line |
| 28650 | CDC42BPA | NM_003607.2 | 5213 | cagggccgaagatctagacaaca | 75961 | - | see also 12495 line |
| 28651 | CDC42BPB | NM_006035.2 | 4845 | ctccacgcttgatctacttcaag | 76022 | - | see also 4206 line |
| 28652 | CDC42BPB | NM_006035.2 | 5313 | aacactcaactccatcgaatagc | 76026 | - | see also 4206 line |
| 28653 | CDC42BPB | NM_006035.2 | 3566 | ggcatcggaacagcctacaaagg | 76003 | - | see also 4206 line |
| 28654 | CDC6 | NM_001254.2 | 383 | gtctgggcgatgacaacctatgc | 76037 | - | see also 12497 line |
| 28655 | CDC6 | NM_001254.2 | 477 | tacacttaagggacgaagattgg | 76041 | - | see also 12497 line |
| 28656 | CDC6 | NM_001254.2 | 387 | gggcgatgacaacctatgcaaca | 76038 | - | see also 12497 line |
| 28657 | CDC7 | NM_003503.2 | 728 | aggaacccatgatacgaaaatag | 76099 | - | see also 2360 line |
| 28658 | CDC7 | NM_003503.2 | 622 | aacgcattcatcagtttggtatt | 76092 | - | see also 2360 line |
| 28659 | CDC7 | NM_003503.2 | 731 | aacccatgatacgaaaatagagc | 76100 | - | see also 2360 line |
| 28660 | CDK10 | NM_003674.2 | 979 | tccggaagcagccctacaacaac | 76127 | - | see also 12499 line |
| 28661 | CDK10 | NM_003674.2 | 707 | ggcctatggtgtcccagtaaagc | 76126 | - | see also 12499 line |
| 28662 | CDK10 | NM_003674.2 | 1099 | gcctggagagctcctatttcaag | 76129 | - | see also 12499 line |
| 28663 | CDK2 | NM_001798.2 | 691 | gagtccctgttcgtacttacacc | 76135 | - | see also 1175 line |
| 28664 | CDK3 | NM_001258.1 | 647 | tggagcattggttgcatctttgc | 76144 | - | see also 12501 line |
| 28665 | CDK4 | NM_000075.2 | 388 | ttcgtgaggtggctttactgagg | 76146 | - | see also 12502 line |
| 28666 | CDK4 | NM_000075.2 | 407 | gaggcgactggaggcttttgagc | 76147 | - | see also 12502 line |
| 28667 | CDK5 | NM_004935.2 | 809 | aacgtcgtgcccaaactcaatgc | 76170 | - | see also 12503 line |
| 28668 | CDK5 | NM_004935.2 | 351 | acctcgatcctgagattgtaaag | 76165 | - | see also 12503 line |
| 28669 | CDK6 | NM_001259.2 | 534 | gagtagtgcatcgcgatctaaaa | 76177 | - | see also 819 line |
| 28670 | CDK6 | NM_001259.2 | 338 | aacgtggtcaggttgtttgatgt | 76172 | - | see also 819 line |
| 28671 | CDK7 | NM_001799.2 | 797 | tggccggacatgtgtagtcttcc | 76211 | - | see also 12505 line |
| 28672 | CDK7 | NM_001799.2 | 884 | gacgacttactagatctcataca | 76217 | - | see also 12505 line |
| 28673 | CDK8 | NM_001260.1 | 706 | tactaacgtcagaaccaatattt | 76240 | - | see also 12506 line |
| 28674 | CDK8 | NM_001260.1 | 710 | aacgtcagaaccaatatttcact | 76241 | - | see also 12506 line |
| 28675 | CDK8 | NM_001260.1 | 1057 | acgtttttgccggttgtcaaatc | 76256 | - | see also 12506 line |
| 28676 | CDK9 | NM_001261.2 | 375 | aaccgctgcaagggtagtatata | 76264 | - | see also 12507 line |
| 28677 | CDK9 | NM_001261.2 | 376 | accgctgcaagggtagtatatac | 76265 | - | see also 12507 line |
| 28678 | CDK9 | NM_001261.2 | 496 | tgcttaacggcctctactacatc | 76270 | - | see also 12507 line |

Figure 46

| | | | | | | | |
|---|---|---|---|---|---|---|---|
| 28679 | CDKL1 | NM_004196.3 | 438 | aacattccgtgattaagctttgt | 76283 | - | see also 12508 line |
| 28680 | CDKL1 | NM_004196.3 | 442 | ttccgtgattaagctttgtgact | 76284 | - | see also 12508 line |
| 28681 | CDKL2 | NM_003948.2 | 1178 | tacatattgacccgacacaaaga | 76335 | - | see also 12509 line |
| 28682 | CDKL2 | NM_003948.2 | 1576 | agcgtggaccacacaaggaatcc | 76344 | - | see also 12509 line |
| 28683 | CDKL3 | NM_016508.2 | 325 | atccttgagcaattgactatct | 76362 | - | see also 12510 line |
| 28684 | CDKL3 | NM_016508.2 | 493 | tggtatagagctcccgaattagt | 76366 | - | see also 12510 line |
| 28685 | CDKL5 | NM_003159.1 | 1611 | cagagtcggcatagctatattga | 76432 | - | see also 12511 line |
| 28686 | CDKL5 | NM_003159.1 | 682 | ttggtttgtcgtaatcgtca | 76412 | - | see also 12511 line |
| 28687 | CENPE | NM_001813.1 | 1595 | cacttcgtcgactatgataat | 76494 | - | see also 12512 line |
| 28688 | CENPE | NM_001813.1 | 287 | tagcagcaccaatcatcgattct | 76466 | - | see also 12512 line |
| 28689 | CFTR | NM_000492.2 | 3334 | ttcggacggcagccttactttga | 76721 | - | see also 384 line |
| 28690 | CFTR | NM_000492.2 | 2856 | cagcaccagttcgtattatgtgt | 76706 | - | see also 384 line |
| 28691 | CGI-04 | NM_015936.1 | 1270 | cccgagggtatcgaatgataaca | 76796 | - | see also 12514 line |
| 28692 | CGI-04 | NM_015936.1 | 158 | cccggagcagggggacgaaaatag | 76766 | - | see also 12514 line |
| 28693 | CGI-152 | NM_020410.1 | 2474 | acgagagtacgcccattgtgaaa | 76822 | - | see also 7866 line |
| 28694 | CGI-152 | NM_020410.1 | 2476 | gagagtacgcccattgtgaaact | 76823 | - | see also 7866 line |
| 28695 | CHEK1 | NM_001274.2 | 1266 | tggcagcggttggtcaaaagaat | 76872 | - | see also 840 line |
| 28696 | CHEK1 | NM_001274.2 | 1120 | cccgcacaggtctttcctatgg | 76864 | - | see also 840 line |
| 28697 | CHEK2 | NM_007194.2 | 1297 | aacgccgtcctttgaataacaat | 76893 | - | see also 4996 line |
| 28698 | CHUK | NM_001278.2 | 1100 | ttcactacgtctcgtattgagc | 76945 | - | see also 844 line |
| 28699 | CHUK | NM_001278.2 | 1777 | aacagcgtgccattgatctatat | 76966 | - | see also 844 line |
| 28700 | CIT | NM_007174.1 | 4544 | tcccgacgggatgtatctattc | 77074 | - | see also 4975 line |
| 28701 | CIT | NM_007174.1 | 1792 | gacggagtgatctctacgaatct | 77029 | - | see also 4975 line |
| 28702 | CIT | NM_007174.1 | 4545 | cccgacgggatgtatctattca | 77075 | - | see also 4975 line |
| 28703 | CLK1 | NM_004071.1 | 362 | tcgacgctacaattgagtgagtaca | 77111 | - | see also 12519 line |
| 28704 | CLK1 | NM_004071.1 | 1390 | ttcaccacgatcgattagactcg | 77141 | - | see also 12519 line |
| 28705 | CLK2 | NM_001291.1 | 354 | cagctacagacgcaacgattata | 77153 | - | see also 12520 line |
| 28706 | CLK2 | NM_001291.1 | 405 | cacagactatcggcattcctatg | 77157 | - | see also 12520 line |
| 28707 | CLK3 | NM_001292.1 | 177 | gagcctccccacgaagatcctcg | 77163 | - | see also 12521 line |
| 28708 | CLK3 | NM_001292.1 | 244 | agcgccgtgacagcggatacatac | 77164 | - | see also 12521 line |
| 28709 | CLK4 | NM_020666.1 | 602 | ctcgttcagaaatccaagtatta | 77190 | - | see also 7912 line |
| 28710 | CLK4 | NM_020666.1 | 1351 | ctggttcgaagaatgtttagaata | 77207 | - | see also 7912 line |
| 28711 | CLPX | NM_066660.3 | 136 | gcctccgcgcagagaggtatttc | 77211 | - | see also 4706 line |
| 28712 | CLPX | NM_066660.3 | 1725 | cagattggcactagaacgaaaaa | 77264 | - | see also 4706 line |
| 28713 | CNK | NM_004073.1 | 275 | accgccaactgtgcgtttttcg | 77278 | - | see also 2743 line |
| 28714 | CNTN6 | NM_014461.2 | 2405 | gtcggtcgaactcgtcattacg | 77355 | - | see also 5690 line |
| 28715 | CNTN6 | NM_014461.2 | 1028 | atccagtccccgatattagttgg | 77315 | - | see also 5690 line |

Figure 46

| | | | | | | | |
|---|---|---|---|---|---|---|---|
| 28716 | CPS1 | NM_001875.2 | 2378 | acgtcgtatccgggaagacatca | 77423 | - | see also 12526 line |
| 28717 | CPS1 | NM_001875.2 | 2518 | atggctattggtcgtacctttga | 77428 | - | see also 12526 line |
| 28718 | CRK7 | NM_016507.1 | 382 | aggcgttcccgggacttactaaa | 77495 | - | see also 6883 line |
| 28719 | CRK7 | NM_016507.1 | 4272 | cccggtggtcgggcaaccattcc | 77574 | - | see also 6883 line |
| 28720 | CRK7 | NM_016507.1 | 852 | gtcggtcagtccccctacaagg | 77510 | - | see also 6883 line |
| 28721 | CSF1R | NM_005211.2 | 2370 | ccgagggaggcgtcgactataag | 77603 | - | see also 3661 line |
| 28722 | CSF1R | NM_005211.2 | 2376 | gaggcgtcgactataagaacatc | 77606 | - | see also 3661 line |
| 28723 | CSK | NM_004383.1 | 1132 | gacgcaactgcggcatagcaacc | 77629 | - | see also 2941 line |
| 28724 | CSK | NM_004383.1 | 436 | gccatccggtacagaatgtattg | 77620 | - | see also 2941 line |
| 28725 | CSK | NM_004383.1 | 899 | ctctgtacgcgcctcattaaacc | 77626 | - | see also 2941 line |
| 28726 | CSNK1A1 | NM_001892.3 | 897 | ttgcgatgtacttaaactattgt | 77642 | - | see also 12529 line |
| 28727 | CSNK1A1 | NM_001892.3 | 142 | cagcggctccaaggctgaattca | 77634 | - | see also 12529 line |
| 28728 | CSNK1D | NM_001893.3 | 657 | gtcgcatcgaatacattcattca | 77650 | - | see also 12530 line |
| 28729 | CSNK1D | NM_001893.3 | 1021 | ccccatcgaagtgttgtgtaaag | 77659 | - | see also 12530 line |
| 28730 | CSNK1D | NM_001893.3 | 653 | atcagtcgcatcgaatacattca | 77648 | - | see also 12530 line |
| 28731 | CSNK1E | NM_001894.4 | 1178 | tccagccggctggcaatacttct | 77674 | - | see also 1209 line |
| 28732 | CSNK1E | NM_001894.4 | 836 | aaggctatccctccgaattctca | 77670 | - | see also 1209 line |
| 28733 | CSNK1G1 | NM_022048.1 | 1233 | cagagccttaggactgttacagc | 77701 | - | see also 8248 line |
| 28734 | CSNK1G2 | NM_001319.5 | 233 | ccgcctaggaaagaatctctata | 77704 | - | see also 12533 line |
| 28735 | CSNK1G3 | NM_004384.1 | 1252 | cccaccgcaggacgttcaaatgc | 77737 | - | see also 2944 line |
| 28736 | CSNK1G3 | NM_004384.1 | 1201 | ggctcggtacaggttgtaagttc | 77733 | - | see also 2944 line |
| 28737 | CSNK2A2 | NM_001896.2 | 953 | aacgatatcctgggacaacattc | 77762 | - | see also 1210 line |
| 28738 | CSNK2A2 | NM_001896.2 | 542 | atccggttttatatgtatgaact | 77748 | - | see also 1210 line |
| 28739 | CSPG4 | NM_001897.2 | 902 | accagtaccctacgcatacttcg | 77764 | - | see also 12536 line |
| 28740 | CSPG4 | NM_001897.2 | 4076 | cactagaggcgcaaaacttcagc | 77770 | - | see also 12536 line |
| 28741 | CSPG6 | NM_005445.2 | 2566 | tactcgagtagagacttatctca | 77858 | - | see also 3793 line |
| 28742 | CSPG6 | NM_005445.2 | 350 | aaccggttaccaatcgataaaga | 77786 | - | see also 3793 line |
| 28743 | DAPK1 | NM_004938.1 | 1604 | gccacgtcgataccttgaaattt | 77925 | - | see also 3434 line |
| 28744 | DAPK1 | NM_004938.1 | 2037 | agggtgtttcgtcgattatcaag | 77933 | - | see also 3434 line |
| 28745 | DAPK2 | NM_014326.2 | 838 | gacccggaaacggctcacaatcc | 77973 | - | see also 5621 line |
| 28746 | DAPK2 | NM_014326.2 | 677 | atcacctacatcctcttaagtgg | 77972 | - | see also 5621 line |
| 28747 | DAPK3 | NM_001348.1 | 866 | aagatcccaagcggagaatgacc | 77978 | - | see also 12539 line |
| 28748 | DAPK3 | NM_001348.1 | 603 | ggggaacgagttcaagaacatct | 77977 | - | see also 12539 line |
| 28749 | DCAMKL1 | NM_004734.1 | 2275 | accacgggtttaccatcaagaga | 78014 | - | see also 3253 line |
| 28750 | DCAMKL1 | NM_004734.1 | 443 | cccagaccggttccgatctttttg | 77982 | - | see also 3253 line |

Figure 46

| | | | | | | | |
|---|---|---|---|---|---|---|---|
| 28751 | DCAMKL1 | NM_004734.1 | 493 | ctctgtcggataacgtgaatttg | 77984 | - | see also 3253 line |
| 28752 | DCK | NM_000788.1 | 425 | ctgaacgatggtcttttaccttc | 78023 | - | see also 12541 line |
| 28753 | DCK | NM_000788.1 | 842 | ttcaagaggtgcctatcttaaca | 78036 | - | see also 12541 line |
| 28754 | DDR1 | NM_001954.3 | 1176 | ggctatgcaggtccactgtaaca | 78045 | - | see also 12542 line |
| 28755 | DDR1 | NM_001954.3 | 2491 | ctggccacactcaactttgtaca | 78054 | - | see also 12542 line |
| 28756 | DDR2 | NM_006182.1 | 1749 | gggtccaactcgacttacgatcg | 78097 | - | see also 12543 line |
| 28757 | DDR2 | NM_006182.1 | 1180 | accgcatcaggaatttcactacc | 78082 | - | see also 12543 line |
| 28758 | DDX32 | NM_018180.2 | 1581 | aaggtgtacaacccgagaataag | 78143 | - | see also 12544 line |
| 28759 | DDX32 | NM_018180.2 | 2347 | ctcttctgtccggttactttatg | 78160 | - | see also 12544 line |
| 28760 | DGKE | NM_003647.1 | 1020 | aacaggcaacgatctatccaata | 78196 | - | see also 2471 line |
| 28761 | DGKE | NM_003647.1 | 138 | tgcggacgggcacctgatcttgt | 78170 | - | see also 2471 line |
| 28762 | DGKZ | NM_003646.1 | 545 | aagccgtccttccgtgaatcagg | 78226 | - | see also 2467 line |
| 28763 | DGUOK | NM_001929.2 | 228 | gcctgcactgcccaaagtcttgg | 78239 | - | see also 1231 line |
| 28764 | DKFZP434 C131 | NM_015518.1 | 93 | cacgtacgccacggtgtacaagg | 78255 | - | - |
| 28765 | DKFZP434 C131 | NM_015518.1 | 317 | acctgtctcgcttcatccatacc | 78258 | - | see also 28764 line |
| 28766 | DKFZP434 C131 | NM_015518.1 | 197 | acctcctcacggagattgagatc | 78257 | - | see also 28764 line |
| 28767 | DKFZp434 C1418 | NM_173655.1 | 1192 | gacctagcggctcggaatatact | 78280 | - | see also 10106 line |
| 28768 | DKFZp434 C1418 | NM_173655.1 | 681 | tccctcaagaattcgtattgaga | 78271 | - | see also 10106 line |
| 28769 | DKFZp434 C1418 | NM_173655.1 | 599 | tcccgggaattaaaacttacatt | 78266 | - | see also 10106 line |
| 28770 | DKFZP434 G2226 | NM_031217.2 | 894 | aaggggacccgatttgtagaagg | 78312 | - | see also 8994 line |
| 28771 | DKFZP434 G2226 | NM_031217.2 | 130 | ttcgtgtacgtccggaaaacact | 78288 | - | see also 8994 line |
| 28772 | DKFZP434 G2226 | NM_031217.2 | 133 | gtgtacgtccggaaaacactaaa | 78289 | - | see also 8994 line |
| 28773 | DKFZp727 A071 | NM_152268.2 | 679 | tccgtggccgagagttttacatg | 78376 | - | see also 12548 line |
| 28774 | DKFZp727 A071 | NM_152268.2 | 682 | gtggccgagagttttacatgaag | 78378 | - | see also 12548 line |
| 28775 | DKFZp761P 1010 | NM_018423.1 | 1434 | gacgaggctgtgttacaagtacc | 78412 | - | see also 12549 line |

Figure 46

| | DKFZp761P1010 | | | | | | |
|---|---|---|---|---|---|---|---|
| 28776 | DKFZp761P1010 | NM_018423.1 | 1309 | gaccagragctgcacacatacc | 78409 | - | see also 12549 line |
| 28777 | DLAT | NM_001931.2 | 1307 | aacattcgtcgggttattgcaca | 78447 | - | see also 1233 line |
| 28778 | DLAT | NM_001931.2 | 1304 | agcaacaattcgtcgggttattgc | 78446 | - | see also 1233 line |
| 28779 | DLAT | NM_001931.2 | 498 | ttcccatcggagcgatcatcgt | 78424 | - | see also 1233 line |
| 28780 | DMPK | NM_004409.1 | 1832 | cccctttacaccggatttcgaag | 78480 | - | see also 12551 line |
| 28781 | DMPK | NM_004409.1 | 2265 | cagaacttcgccagtcaactacg | 78483 | - | see also 12551 line |
| 28782 | DNAH11 | NM_003777.1 | 7380 | ttccgtcgcaggggaacaatctt | 78656 | - | see also 2579 line |
| 28783 | DNAH11 | NM_003777.1 | 6178 | agcccgaaagttcattacgttgt | 78623 | - | see also 2579 line |
| 28784 | DNAH3 | NM_017539.1 | 8570 | agcggatccggaaaaggtttatc | 79032 | - | see also 7017 line |
| 28785 | DNAH3 | NM_017539.1 | 5172 | tgcagctctcggcgatttcacag | 78949 | - | see also 7017 line |
| 28786 | DNAH5 | NM_001369.1 | 746 | aggaacctacggactacttgact | 79129 | - | see also 930 line |
| 28787 | DNAH5 | NM_001369.1 | 13814 | ttcgaacggacttgaactacatt | 79507 | - | see also 930 line |
| 28788 | DNAH5 | NM_001369.1 | 9554 | cccacgtgacgcccaaatcatac | 79402 | - | see also 930 line |
| 28789 | DNAH7 | NM_018897.1 | 12134 | gacgaggttgagcactgttatgt | 79886 | - | see also 12555 line |
| 28790 | DNAH7 | NM_018897.1 | 5744 | atcgaactcgaaatacgtttaaa | 79696 | - | see also 12555 line |
| 28791 | DNAH8 | NM_001371.1 | 9129 | ttccgtgccgttcttgaaatt | 80144 | - | see also 937 line |
| 28792 | DNAH9 | NM_001372.2 | 12842 | gtgttggccggatgaatatcctca | 80506 | - | see also 12557 line |
| 28793 | DNAH9 | NM_001372.2 | 12757 | agcgggttgacagacgagttaac | 80499 | - | see also 12557 line |
| 28794 | DNCH1 | NM_001376.2 | 1839 | tggggccattcgcgaataccaga | 80564 | - | see also 943 line |
| 28795 | DNCH1 | NM_001376.2 | 9698 | tgcgtgtgacttgaggataaag | 80741 | - | see also 943 line |
| 28796 | DNCL12 | NM_006141.2 | 1271 | aagccggacccaaacatcaaaa | 80834 | - | see also 12559 line |
| 28797 | DNCL12 | NM_006141.2 | 1249 | ctcccaggcacgtcagtaaaaa | 80832 | - | see also 12559 line |
| 28798 | DNCL12 | NM_006141.2 | 236 | accctcatgactaaactacaagg | 80816 | - | see also 12559 line |
| 28799 | DPCK | NM_025233.3 | 204 | ctgtgaatcacacactctatgt | 80839 | - | see also 8886 line |
| 28800 | DPCK | NM_025233.3 | 1112 | cagcttccgccagcgaatgttgg | 80847 | - | see also 8886 line |
| 28801 | DQX1 | NM_133637.1 | 592 | gagcggtcggttggcatcagattc | 80861 | - | see also 9590 line |
| 28802 | DQX1 | NM_133637.1 | 562 | tggggcgttcgtactagatga | 80859 | - | see also 9590 line |
| 28803 | DTYMK | NM_012145.1 | 297 | gaccctcgtcgtggacagatacg | 80898 | - | see also 12562 line |
| 28804 | DTYMK | NM_012145.1 | 140 | tgctccgttcccggaaagatca | 80887 | - | see also 12562 line |
| 28805 | DTYMK | NM_012145.1 | 263 | tgccgttaattaaggaaaagttg | 80896 | - | see also 12562 line |
| 28806 | DYRK1A | NM_001396.2 | 1407 | tacggtcgtgactacttgaagt | 80949 | - | see also 960 line |
| 28807 | DYRK1A | NM_001396.2 | 902 | ttgcgactccagaacttagtatc | 80931 | - | see also 960 line |
| 28808 | DYRK1B | NM_004714.1 | 1728 | accgctacagcaaccgatattgt | 80986 | - | see also 3235 line |
| 28809 | DYRK1B | NM_004714.1 | 598 | ctggctgagcgctacgaaattg | 80976 | - | see also 3235 line |
| 28810 | DYRK2 | NM_003583.2 | 1680 | accggttgctatcacatctatc | 81012 | - | see also 12564 line |
| 28811 | DYRK2 | NM_003583.2 | 720 | gtcaaggctacgactcacaaagt | 80995 | - | see also 12564 line |

Figure 46

| 28812 | DYRK3 | NM_003582.1 | 747 | acctcgagaccatctagcttatc | 81029 | - | see also 12565 line |
|---|---|---|---|---|---|---|---|
| 28813 | DYRK3 | NM_003582.1 | 1547 | gtcgctcacgtaggggtaaaaag | 81050 | - | see also 12565 line |
| 28814 | DYRK4 | NM_003845.1 | 1700 | aacgttttaccccctattgtatg | 81096 | - | see also 12566 line |
| 28815 | DYRK4 | NM_003845.1 | 377 | ctcgacacggctcctgagaaatt | 81071 | - | see also 12566 line |
| 28816 | DYT1 | NM_000113.1 | 1002 | aacggtgttcaccaagttagatt | 81118 | - | see also 96 line |
| 28817 | DYT1 | NM_000113.1 | 354 | aggcaccggcaaaaatttcgtca | 81100 | - | see also 96 line |
| 28818 | EGFR | NM_005228.2 | 2387 | cccgtcgctatcaaggaattaag | 81171 | - | see also 12568 line |
| 28819 | EGFR | NM_005228.2 | 1128 | acggcgtccgcaagtgtaagaag | 81140 | - | see also 12568 line |
| 28820 | EHD4 | NM_139265.2 | 1416 | tacactctgtcgcccatcaatgg | 81209 | - | see also 12569 line |
| 28821 | EHD4 | NM_139265.2 | 1059 | aggctaccggaaatctacattca | 81206 | - | see also 12569 line |
| 28822 | EIF2AK3 | NM_004836.2 | 600 | cgcggcaggtcattagtaattat | 81211 | - | see also 3353 line |
| 28823 | EIF2AK3 | NM_004836.2 | 959 | ttgtcgccaatgggatagtgacg | 81220 | - | see also 3353 line |
| 28824 | EPB42 | NM_000119.1 | 1441 | accggtgggcgtcttctcataga | 81299 | - | see also 12571 line |
| 28825 | EPB42 | NM_000119.1 | 2028 | ctcagtgacgctggttaatcaca | 81306 | - | see also 12571 line |
| 28826 | EPHA1 | NM_005232.2 | 2168 | agcgaaagccgatcatgatcatc | 81329 | - | see also 3668 line |
| 28827 | EPHA2 | NM_004431.2 | 401 | aggagaggctgagcgtatcttca | 81337 | - | see also 12573 line |
| 28828 | EPHA2 | NM_004431.2 | 1844 | gtccccggaggacgtttacttct | 81352 | - | see also 12573 line |
| 28829 | EPHA3 | NM_005233.3 | 1538 | cacctgtcctgacgattaagaaa | 81401 | - | see also 12574 line |
| 28830 | EPHA3 | NM_005233.3 | 1503 | tgcggtcagcatcacaactaatc | 81398 | - | see also 12574 line |
| 28831 | EPHA3 | NM_005233.3 | 1539 | acctgtcctgacgattaagaaag | 81402 | - | see also 12574 line |
| 28832 | EPHA4 | NM_004438.2 | 121 | cccgcgaatgaagttaccttatt | 81447 | - | see also 2970 line |
| 28833 | EPHA4 | NM_004438.2 | 551 | tccgggatgtagggccattaagc | 81458 | - | see also 2970 line |
| 28834 | EPHA5 | NM_004439.3 | 1585 | gaccagctacacgdattatcaaat | 81527 | - | see also 2972 line |
| 28835 | EPHA5 | NM_004439.3 | 1338 | atctactcgctcacacaaactat | 81520 | - | see also 2972 line |
| 28836 | EPHA7 | NM_004440.2 | 1430 | tagcccacgctaattatactttt | 81594 | - | see also 2974 line |
| 28837 | EPHA7 | NM_004440.2 | 1049 | gtggccgtgggttctacaagtct | 81580 | - | see also 2974 line |
| 28838 | EPHA8 | NM_020526.2 | 699 | ctctccgcatctactataagaag | 81644 | - | see also 7887 line |
| 28839 | EPHA8 | NM_020526.2 | 697 | ctctctccgcatctactataaga | 81643 | - | see also 7887 line |
| 28840 | EPHB1 | NM_004441.2 | 1117 | cacctgtcggaccggttattacc | 81667 | - | see also 2980 line |
| 28841 | EPHB1 | NM_004441.2 | 1183 | atcaggtccccgcaatgttatct | 81668 | - | see also 2980 line |
| 28842 | EPHB1 | NM_004441.2 | 712 | gcctcttactcggaatggttttt | 81660 | - | see also 2980 line |
| 28843 | EPHB2 | NM_004442.4 | 479 | gccgcgtcatgaaaatcaacacc | 81696 | - | see also 12580 line |
| 28844 | EPHB2 | NM_004442.4 | 1135 | acccgctgcggggacaatgtaca | 81701 | - | see also 12580 line |
| 28845 | EPHB3 | NM_004443.3 | 709 | ggcttcgcacggggttcatctgg | 81723 | - | see also 12581 line |
| 28846 | EPHB3 | NM_004443.3 | 3278 | cagtgcggggtttgcatcttttg | 81738 | - | see also 12581 line |
| 28847 | EPHB4 | NM_004444.3 | 2209 | gtctcctacgtcaagattgaaga | 81749 | - | see also 12582 line |
| 28848 | EPHB4 | NM_004444.3 | 1239 | cccagccaatagccactctaaca | 81741 | - | see also 12582 line |

Figure 46

| 28849 | EPHB4 | NM_004444.3 | 2146 | atcgacccettcacttatgaaga | 81747 | - | see also 12582 line |
|---|---|---|---|---|---|---|---|
| 28850 | EPHB6 | NM_004445.1 | 2667 | aggactcggggtgaagtattaca | 81772 | - | see also 12583 line |
| 28851 | EPHB6 | NM_004445.1 | 2666 | caggactcggggtgaagtattac | 81771 | - | see also 12583 line |
| 28852 | EPRS | NM_004446.1 | 3323 | ttcctacgtactcgtgaatttct | 81878 | - | see also 12584 line |
| 28853 | EPRS | NM_004446.1 | 2343 | gggaggtggttcgtaagctaaaa | 81844 | - | see also 12584 line |
| 28854 | ERBB2 | NM_004448.1 | 3684 | tggggtcgtcaaagacgtttttg | 81943 | - | see also 12585 line |
| 28855 | ERBB2 | NM_004448.1 | 506 | atggagacccgctgaacaatacc | 81923 | - | see also 12585 line |
| 28856 | ERBB3 | NM_001982.1 | 1629 | tacggaagagcgactagacatca | 81971 | - | see also 12586 line |
| 28857 | ERBB3 | NM_001982.1 | 2220 | aagggctatgaggcgatacttgg | 81983 | - | see also 12586 line |
| 28858 | ERBB3 | NM_001982.1 | 2928 | gccctatgcagggctacgattgg | 82002 | - | see also 12586 line |
| 28859 | ERBB4 | NM_005235.1 | 2567 | cagcccgtaatgtcttagtgaaa | 82083 | - | see also 12587 line |
| 28860 | ERBB4 | NM_005235.1 | 331 | gagaatttacgcattattcgtgg | 82029 | - | see also 12587 line |
| 28861 | ERK8 | NM_139021.1 | 60 | cgcattgtccggagatacctact | 82121 | - | see also 12588 line |
| 28862 | ERK8 | NM_139021.1 | 238 | ttggggaccatcccaacatcatc | 82122 | - | see also 12588 line |
| 28863 | FER | NM_005246.1 | 2565 | agcaccggaagctcttaattatg | 82188 | - | see also 3686 line |
| 28864 | FER | NM_005246.1 | 1314 | aacgttagcggaagaacttatgc | 82152 | - | see also 3686 line |
| 28865 | FES | NM_002005.2 | 1782 | ggggaactttggcgaagtgttca | 82201 | - | see also 12590 line |
| 28866 | FES | NM_002005.2 | 149 | gtctactggagggcatgagaaag | 82199 | - | see also 12590 line |
| 28867 | FGFR1 | NM_000604.2 | 1282 | cccacactgcgctggttgaaaaa | 82207 | - | see also 414 line |
| 28868 | FGFR1 | NM_000604.2 | 1284 | cacactgcgctggttgaaaaatg | 82208 | - | see also 414 line |
| 28869 | FGFR2 | NM_000141.2 | 1153 | aaccatgcggtggctgaaaaacg | 82235 | - | see also 127 line |
| 28870 | FGFR2 | NM_000141.2 | 1318 | cacgtaccacctggatgttgtgg | 82237 | - | see also 127 line |
| 28871 | FGFR2 | NM_000141.2 | 658 | gccctccttcagtttagttgagg | 82228 | - | see also 127 line |
| 28872 | FGFR3 | NM_000142.2 | 504 | gcccgagcggatggacaagaagc | 82276 | - | see also 12591 line |
| 28873 | FGFR3 | NM_000142.2 | 2198 | cacacgacctgtacatgatcatg | 82281 | - | see also 12591 line |
| 28874 | FGFR4 | NM_002011.2 | 1498 | ggcctcgtgagtctagatctacc | 82287 | - | see also 12592 line |
| 28875 | FGFR4 | NM_002011.2 | 645 | tgcggggaacaccgtcaagttcc | 82283 | - | see also 12592 line |
| 28876 | FGR | NM_005248.1 | 720 | aggcgatcatgtgaagcattaca | 82293 | - | see also 12593 line |
| 28877 | FGR | NM_005248.1 | 777 | caccacacgggttcagttcaact | 82295 | - | see also 12593 line |
| 28878 | FIGN | NM_018086.1 | 1963 | ttgaggcccgttacatatcaaga | 82358 | - | see also 7237 line |
| 28879 | FIGNL1 | NM_022116.2 | 2231 | atcaagttcgacccatagcttac | 82424 | - | see also 8278 line |
| 28880 | FIGNL1 | NM_022116.2 | 1306 | aagtctctaggagctagtagatc | 82392 | - | see also 8278 line |
| 28881 | FLJ10074 | NM_017988.2 | 99 | cccttgcggttcgtgtaaattca | 82431 | - | see also 12596 line |
| 28882 | FLJ10074 | NM_017988.2 | 98 | tcccttgcggttcgtgtaaattc | 82430 | - | see also 12596 line |
| 28883 | FLJ10326 | NM_018060.2 | 825 | tacgtactggcggcagataaagt | 82497 | - | see also 7221 line |
| 28884 | FLJ10326 | NM_018060.2 | 2413 | tccgtactgggtggattagtact | 82555 | - | see also 7221 line |
| 28885 | FLJ10496 | NM_018114.3 | 847 | ggcggctgcctggttgtacatgt | 82576 | - | see also 12598 line |

Figure 46

| | | | | | | | |
|---|---|---|---|---|---|---|---|
| 28886 | FLJI0496 | NM_018114.3 | 1218 | gagtggcacctttgtcatgtata | 82580 | - | see also 12598 line |
| 28887 | FLJI0514 | NM_018122.3 | 1971 | aaggcccgtagccaacactatga | 82623 | - | see also 7266 line |
| 28888 | FLJI0514 | NM_018122.3 | 2018 | aggaggtggttcaattgaattc | 82629 | - | see also 7266 line |
| 28889 | FLJI0709 | NM_018188.1 | 375 | aacagtccaagtccaaagagtat | 82641 | - | see also 12600 line |
| 28890 | FLJI0774 | NM_024662.1 | 3145 | ggccgaacgcctcgatcatcagc | 82688 | - | see also 8690 line |
| 28891 | FLJI0774 | NM_024662.1 | 2309 | gcctgccgaacgcctggattacc | 82675 | - | see also 8690 line |
| 28892 | FLJI2118 | NM_024537.1 | 565 | ctcgttggaacgcttattcaacg | 82704 | - | see also 12602 line |
| 28893 | FLJI2118 | NM_024537.1 | 796 | ctgccatcgctagtatggtattt | 82706 | - | see also 12602 line |
| 28894 | FLJI2528 | NM_025150.3 | 2051 | aacattcggactcgagataatcg | 82748 | - | see also 12603 line |
| 28895 | FLJI2528 | NM_025150.3 | 177 | aacccggactattaagatatca | 82722 | - | see also 12603 line |
| 28896 | FLJI2735 | NM_024857.3 | 3664 | ctcgggttgcatagacacttaaag | 82837 | - | see also 12604 line |
| 28897 | FLJI2735 | NM_024857.3 | 401 | tgcgagattgagccatgcaaaaa | 82749 | - | see also 12604 line |
| 28898 | FLJI2969 | NM_022838.1 | 921 | cccctggcagaacgcagtatagt | 82915 | - | see also 12605 line |
| 28899 | FLJI2969 | NM_022838.1 | 1582 | ttgccctccttaagttaactaag | 82928 | - | see also 12605 line |
| 28900 | FLJI3490 | NM_024942.1 | 340 | atgcttctattggcatttaag | 82954 | - | see also 12606 line |
| 28901 | FLJI3490 | NM_024942.1 | 1357 | cccactggatacctctaagaca | 82962 | - | see also 12606 line |
| 28902 | FLJI4600 | NM_032810.2 | 1104 | accttgactggctataatgaga | 82985 | - | see also 9292 line |
| 28903 | FLJI4600 | NM_032810.2 | 969 | ttctacgaaacgttcaagttct | 82982 | - | see also 9292 line |
| 28904 | FLJI4813 | NM_032844.1 | 710 | agctcgttggatttaacacacc | 83008 | - | see also 9309 line |
| 28905 | FLJI4813 | NM_032844.1 | 1123 | tgccattgagacgaaaggttca | 83023 | - | see also 9309 line |
| 28906 | FLJI4813 | NM_032844.1 | 836 | aaggacactagccttattctag | 83013 | - | see also 9309 line |
| 28907 | FLJ20245 | NM_017723.1 | 775 | cgcgctcgtgctgcaataccatg | 83065 | - | see also 12609 line |
| 28908 | FLJ20245 | NM_017723.1 | 772 | caggcgctcgtgctgcaatacc | 83064 | - | see also 12609 line |
| 28909 | FLJ20559 | NM_017881.1 | 594 | gaggagtacaagggtcatcagc | 83078 | - | see also 12610 line |
| 28910 | FLJ20559 | NM_017881.1 | 618 | tccagactctccggatactttg | 83079 | - | see also 12610 line |
| 28911 | FLJ20574 | NM_017886.1 | 252 | tcvgtctcaccgtgaataaaa | 83094 | - | see also 12611 line |
| 28912 | FLJ20574 | NM_017886.1 | 174 | ggcgacggaagggaacaatcaat | 83088 | - | see also 12611 line |
| 28913 | FLJ20604 | NM_017897.1 | 668 | gccagtcagcattcgatataaa | 83137 | - | see also 12612 line |
| 28914 | FLJ20604 | NM_017897.1 | 880 | aagttggcatgtcgaccatttca | 83148 | - | see also 12612 line |
| 28915 | FLJ21019 | NM_024927.3 | 1554 | tgccctcgctgcgggagatttcc | 83165 | - | see also 12613 line |
| 28916 | FLJ21019 | NM_024927.3 | 1849 | ctccgggtggagactatgtctgc | 83168 | - | see also 12613 line |
| 28917 | FLJ22002 | NM_024838.3 | 1702 | ttccgcatacttgatcttgtta | 83217 | - | see also 8780 line |
| 28918 | FLJ22002 | NM_024838.3 | 1817 | atgggaatccgattcgaaaatt | 83225 | - | see also 8780 line |
| 28919 | FLJ22002 | NM_024838.3 | 1539 | cagtgggttgtgagtcagattt | 83213 | - | see also 8780 line |
| 28920 | FLJ22761 | NM_025130.2 | 1785 | cagtgcgaatgtacaacaagatc | 83265 | - | see also 12615 line |
| 28921 | FLJ22761 | NM_025130.2 | 464 | ttctacccaacgccaatgaaat | 83250 | - | see also 12615 line |
| 28922 | FLJ22865 | NM_025109.3 | 934 | atgcgtgtacactgcgggaataac | 83279 | - | see also 8864 line |

Figure 46

| | | | | | | | |
|---|---|---|---|---|---|---|---|
| 28923 | FLJ22865 | NM_025109.3 | 935 | tgcgtgtacactgaggaataaca | 83280 | - | see also 8864 line |
| 28924 | FLJ22955 | NM_024819.3 | 628 | agcacaccgtggtagtatactgc | 83305 | - | see also 12617 line |
| 28925 | FLJ22955 | NM_024819.3 | 452 | gggggacctgatctttaaccagc | 83303 | - | see also 12617 line |
| 28926 | FLJ23074 | NM_025052.1 | 454 | gtgtggtacatcgcgatatcaaa | 83311 | - | see also 12618 line |
| 28927 | FLJ23074 | NM_025052.1 | 463 | atcgcgatatcaaaggaaataat | 83314 | - | see also 12618 line |
| 28928 | FLJ23356 | NM_032237.2 | 711 | ccctctaggttccttgagtaacc | 83328 | - | see also 9128 line |
| 28929 | FLJ23356 | NM_032237.2 | 1074 | gccctcatatgatgagaagattg | 83331 | - | see also 9128 line |
| 28930 | FLJ23441 | NM_024678.3 | 300 | cgcgagtggggagcgcattaaga | 83340 | - | see also 12620 line |
| 28931 | FLJ23441 | NM_024678.3 | 630 | gactaacgttctgggttctatat | 83350 | - | see also 12620 line |
| 28932 | FLJ23441 | NM_024678.3 | 903 | tccgaccttccgagctgaaaatt | 83357 | - | see also 12620 line |
| 28933 | FLJ25005 | NM_152334.2 | 1048 | caggtgcggtccattaattgacc | 83391 | - | see also 12621 line |
| 28934 | FLJ25005 | NM_152334.2 | 629 | ctggctgaaagcacggtaatagc | 83381 | - | see also 12621 line |
| 28935 | FLJ25006 | NM_144610.1 | 562 | aacggcaccttttcattatgtgt | 83433 | - | see also 12622 line |
| 28936 | FLJ25006 | NM_144610.1 | 802 | gagctcaagcctacactatctgt | 83436 | - | see also 12622 line |
| 28937 | FLJ31364 | NM_152463.1 | 1700 | cgccgcattggaccagaactatc | 83459 | - | see also 12623 line |
| 28938 | FLJ31364 | NM_152463.1 | 1701 | gccgcattggaccagaactatcc | 83460 | - | see also 12623 line |
| 28939 | FLJ32685 | NM_152534.1 | 592 | aagacgcacacaaaggtatttta | 83476 | - | see also 12624 line |
| 28940 | FLJ32685 | NM_152534.1 | 594 | gacgcacacaaaggtattttatg | 83477 | - | see also 12624 line |
| 28941 | FLJ32704 | NM_152572.2 | 1288 | gccatgtgtggacgtcttctacc | 83490 | - | see also 12625 line |
| 28942 | FLJ32704 | NM_152572.2 | 1952 | atcgagagtgggatcattaatcc | 83499 | - | see also 12625 line |
| 28943 | FLJ32704 | NM_152572.2 | 1929 | acccatacacagtcttcgaatac | 83497 | - | see also 12625 line |
| 28944 | FLJ32818 | NM_144685.3 | 1429 | cccgtcgtggccgcagaagatgg | 83502 | - | kinase_related |- |- |
| 28945 | FLJ32818 | NM_144685.3 | 521 | tccatgacgccctcaagttctac | 83500 | - | see also 28944 line |
| 28946 | FLJ32818 | NM_144685.3 | 1150 | gaccagattgagacagtgaatgg | 83501 | - | see also 28944 line |
| 28947 | FLJ34389 | NM_152649.1 | 1149 | ggctggcagcattgcaatagtga | 83526 | - | see also 12626 line |
| 28948 | FLJ34389 | NM_152649.1 | 1669 | atcccgtttcaaggctgtaattc | 83538 | - | see also 12626 line |
| 28949 | FLJ37300 | NM_153209.1 | 859 | cgcgcctcgcagacacagaatcg | 83546 | - | see also 12627 line |
| 28950 | FLJ37300 | NM_153209.1 | 646 | accgaagtctccaccatcaatgc | 83543 | - | see also 12627 line |
| 28951 | FLT1 | NM_002019.2 | 1338 | ctcgccggaagttgtatggttaa | 83585 | - | see also 12628 line |
| 28952 | FLT1 | NM_002019.2 | 1077 | ttccgtaaggcgacgaattgacc | 83572 | - | see also 12628 line |
| 28953 | FLT1 | NM_002019.2 | 1334 | ttccctcgccggaagttgtatgg | 83584 | - | see also 12628 line |
| 28954 | FLT3 | NM_004119.1 | 593 | aggacgccctggtctgcatatct | 83665 | - | see also 2784 line |
| 28955 | FLT4 | NM_002020.1 | 781 | gtgtgggctgagtttaactcagg | 83752 | - | see also 12630 line |
| 28956 | FLT4 | NM_002020.1 | 342 | cagctacgtctgctactacaagt | 83748 | - | see also 12630 line |
| 28957 | FPGS | NM_004957.2 | 386 | tggcctgaagacgggattcttta | 83768 | - | see also 12631 line |
| 28958 | FPGS | NM_004957.2 | 387 | ggcctgaagacgggattctttag | 83769 | - | see also 12631 line |
| 28959 | FRK | NM_002031.2 | 1866 | gcctaaggaacgacctacatttg | 83821 | - | see also 12632 line |

Figure 46

| 28960 | FRK | NM_002031.2 | 1526 | tcctcgttggtgaacataatatc | 83805 | - | see also 12632 line | | |
|---|---|---|---|---|---|---|---|---|---|
| 28961 | FRK | NM_002031.2 | 1650 | gcccgaagccattcgtagtaata | 83810 | - | see also 12632 line | | |
| 28962 | FTHFSDC1 | NM_015440.3 | 559 | gccggttcttgcaattatccagg | 83824 | - | see also 6417 line | | |
| 28963 | FTHFSDC1 | NM_015440.3 | 580 | ggcaggtgacgacaacttgatgc | 83826 | - | see also 6417 line | | |
| 28964 | FUK | NM_145059.1 | 2940 | cagcctggtacggcaaactgagg | 83869 | - | see also 12634 line | | |
| 28965 | FUK | NM_145059.1 | 936 | tgcagatgtagcgggttatctgc | 83865 | - | see also 12634 line | | |
| 28966 | FYN | NM_002037.3 | 1804 | ttcggattggcccgattgataga | 83898 | - | see also 1316 line | | |
| 28967 | GABARAP L2 | NM_007285.6 | 190 | cgcgaagattcgagcgaaatatc | 83909 | - | see also 12636 line | | |
| 28968 | GABARAP L2 | NM_007285.6 | 403 | aactatgggacagctttacgaga | 83914 | - | see also 12636 line | | |
| 28969 | GAK | NM_005255.1 | 799 | gcgaaacttcgaatagtcaatgg | 83941 | - | see also 12637 line | | |
| 28970 | GAK | NM_005255.1 | 795 | tggagcgaaacttcgaatagtca | 83938 | - | see also 12637 line | | |
| 28971 | GALK1 | NM_000154.1 | 372 | tcctcggtgggccaactatgtca | 83971 | - | see also 12638 line | | |
| 28972 | GALK1 | NM_000154.1 | 617 | tggaccagttcatctcacttatg | 83974 | - | see also 12638 line | | |
| 28973 | GALK2 | NM_002044.1 | 646 | ctctgagggcaaccgatgtaaaa | 84002 | - | see also 1319 line | | |
| 28974 | GALK2 | NM_002044.1 | 1037 | tgcgcgagtgctccagtttaaga | 84014 | - | see also 1319 line | | |
| 28975 | GARS | NM_002047.1 | 1505 | cggacagtccgctcggaaaatgc | 84042 | - | see also 1320 line | | |
| 28976 | GARS | NM_002047.1 | 1710 | tcctacggttggattgagattgt | 84046 | - | see also 1320 line | | |
| 28977 | GCK | NM_000162.2 | 1671 | gagccgcagcgaggacgtaatgc | 84071 | - | see also 12641 line | | |
| 28978 | GCK | NM_000162.2 | 1407 | caggctcgtggacgaaaacctgc | 84068 | - | see also 12641 line | | |
| 28979 | GK | NM_000167.1 | 1486 | ctcgaacccgaggatttgtctgc | 84072 | - | kinase_related | glycerol kinase | hyperglycerolemia |
| 28980 | GK2 | NM_033214.1 | 242 | ttgacgaactgaatattgatata | 84074 | - | see also 12642 line | | |
| 28981 | GK2 | NM_033214.1 | 1643 | tgctaattggagcaagatatatc | 84091 | - | see also 12642 line | | |
| 28982 | GMPS | NM_003875.1 | 824 | aggcacgtcaaaagttttggttt | 84117 | - | see also 12643 line | | |
| 28983 | GMPS | NM_003875.1 | 816 | gagagagtaggcacgtcaaaagt | 84115 | - | see also 12643 line | | |
| 28984 | GMPS | NM_003875.1 | 818 | gagagtaggcacgtcaaaagttt | 84116 | - | see also 12643 line | | |
| 28985 | GNE | NM_005476.3 | 1374 | gacgaacctccgagttgcaatag | 84184 | - | see also 12644 line | | |
| 28986 | GNE | NM_005476.3 | 1240 | aagtttctcaaatctatcgatct | 84181 | - | see also 12644 line | | |
| 28987 | GPRK2L | NM_005307.1 | 1189 | aggaggtcgatcaaagaatcaag | 84242 | - | see also 12645 line | | |
| 28988 | GPRK2L | NM_005307.1 | 536 | aaggcaacccgtaacaaagaaca | 84221 | - | see also 12645 line | | |
| 28989 | GPRK5 | NM_005308.1 | 1138 | caccgtctaccgagatctgaaac | 84269 | - | see also 12646 line | | |
| 28990 | GPRK5 | NM_005308.1 | 1100 | gagatcctctgcggcttagaaga | 84268 | - | see also 12646 line | | |
| 28991 | GPRK6 | NM_002082.1 | 201 | ctcagcctcgagcgtgactatca | 84284 | - | see also 1347 line | | |
| 28992 | GPRK7 | NM_139209.1 | 1063 | ctcggcaactgcaggttatctga | 84298 | - | see also 12648 line | | |

Figure 46

| | | | | | | | |
|---|---|---|---|---|---|---|---|
| 28993 | GPRK7 | NM_139209.1 | 1061 | acctcggcaactgcaggttatct | 84297 | - | see also 12648 line |
| 28994 | GSG2 | NM_031965.1 | 2217 | aacaaccgctggggtgaatatca | 84353 | - | see also 12649 line |
| 28995 | GSG2 | NM_031965.1 | 57 | agccggcttttccgcacatatgg | 84313 | - | see also 12649 line |
| 28996 | GSK3A | NM_019884.1 | 1477 | accccgtcctcacaagctttaac | 84369 | - | see also 12650 line |
| 28997 | GSK3A | NM_019884.1 | 463 | gtggcttacacggacatcaaagt | 84359 | - | see also 12650 line |
| 28998 | GSK3B | NM_002093.2 | 1223 | agccgtctgctggagtatacacc | 84391 | - | see also 1357 line |
| 28999 | GSK3B | NM_002093.2 | 683 | cagacgctccctgtgatttatgt | 84380 | - | see also 1357 line |
| 29000 | GSS | NM_000178.2 | 1330 | cccgagtggtccagtgcatttca | 84411 | - | see also 12652 line |
| 29001 | GSS | NM_000178.2 | 1151 | ccctagccggtttgtgctaaagc | 84410 | - | see also 12652 line |
| 29002 | GUCY2C | NM_004963.1 | 2342 | tccgacgtctacagctatattct | 84476 | - | see also 12653 line |
| 29003 | GUCY2C | NM_004963.1 | 1233 | gacctatgatacccacgtaaata | 84442 | - | see also 12653 line |
| 29004 | GUCY2C | NM_004963.1 | 2341 | atccgacgtctacagctatattc | 84475 | - | see also 12653 line |
| 29005 | GUCY2D | NM_000180.1 | 1414 | ctcgtgctggttcgatccaaaca | 84503 | - | see also 12654 line |
| 29006 | GUCY2D | NM_000180.1 | 2848 | gacaataggggacgcctatatgg | 84517 | - | see also 12654 line |
| 29007 | GUCY2F | NM_001522.1 | 3222 | gtgccggtccgaattcgaattgg | 84594 | - | see also 12655 line |
| 29008 | GUCY2F | NM_001522.1 | 1374 | atcgcacaagccatgaataatgc | 84547 | - | see also 12655 line |
| 29009 | GUK1 | NM_000858.3 | 360 | aacggcaaagattactactttgt | 84601 | - | see also 12656 line |
| 29010 | GUK1 | NM_000858.3 | 763 | aggcgctctctgaggaaatcaag | 84604 | - | see also 12656 line |
| 29011 | HAK | NM_052947.1 | 4331 | tccatcggcctgagaacaatatc | 84673 | - | see also 12657 line |
| 29012 | HAK | NM_052947.1 | 3929 | gtcgccaggatactaaaggatgt | 84668 | - | see also 12657 line |
| 29013 | HAK | NM_052947.1 | 4330 | atccatcggcctgagaacaatat | 84672 | - | see also 12657 line |
| 29014 | HARS | NM_002109.3 | 716 | aacgatcgacgcattctagatgg | 84696 | - | see also 12658 line |
| 29015 | HARS | NM_002109.3 | 1054 | tgctcgagggctggattactaca | 84702 | - | see also 12658 line |
| 29016 | HARSL | NM_012208.2 | 756 | tggtcagtttgaccctatgatcc | 84713 | - | see also 12659 line |
| 29017 | HARSL | NM_012208.2 | 688 | cggcgagagagcccaaccatagt | 84712 | - | see also 12659 line |
| 29018 | HCK | NM_002110.2 | 259 | ctccaggtcggaggcaatacatt | 84732 | - | see also 1360 line |
| 29019 | HCK | NM_002110.2 | 265 | gtcggaggcaatacattctcaaa | 84734 | - | see also 1360 line |
| 29020 | HCK | NM_002110.2 | 1341 | tgcatccctggtgtgtaagattg | 84741 | - | see also 1360 line |
| 29021 | HEAB | NM_006831.1 | 1618 | gagcgtatccgtgagtattttta | 84760 | - | see also 12660 line |
| 29022 | HEAB | NM_006831.1 | 1616 | atgagcgtatccgtgagtatttt | 84759 | - | see also 12660 line |
| 29023 | HIPK2 | NM_022740.1 | 1102 | ctggtggatccatctagacaacc | 84780 | - | see also 8444 line |
| 29024 | HIPK2 | NM_022740.1 | 432 | cggcggaccacacaacctaatgc | 84769 | - | see also 8444 line |
| 29025 | HIPK3 | NM_005734.2 | 3119 | tgggtactcgtcagcaaaaattg | 84902 | - | see also 3977 line |
| 29026 | HIPK3 | NM_005734.2 | 2803 | aagagaccgaatagtatgtcaga | 84892 | - | see also 3977 line |
| 29027 | HK1 | NM_000188.1 | 1310 | ttggtgtcgacggatctctttac | 84935 | - | see also 157 line |
| 29028 | HK1 | NM_000188.1 | 2449 | atcgagagtgaccgattagcact | 84957 | - | see also 157 line |
| 29029 | HK2 | NM_000189.4 | 4187 | tccgtaacattctcatcgatttc | 84979 | - | see also 12664 line |

Figure 46

| | | | | | | | |
|---|---|---|---|---|---|---|---|
| 29030 | HK2 | NM_000189.4 | 3134 | aggagcggctgcgctctactatt | 84971 | - | see also 12664 line |
| 29031 | HK3 | NM_002115.1 | 2349 | atgtccgccacatcctttaca | 84999 | - | see also 12665 line |
| 29032 | HK3 | NM_002115.1 | 1310 | cagccgggagccaacaaacactcc | 84994 | - | see also 12665 line |
| 29033 | HRI | NM_014413.2 | 167 | ccccgaatatgacgaatctgatg | 85004 | - | see also 12666 line |
| 29034 | HRI | NM_014413.2 | 1696 | tgccggaatccctccgtaaaagg | 85052 | - | see also 12666 line |
| 29035 | HRI | NM_014413.2 | 168 | cccgaatatgacgaatctgatgt | 85005 | - | see also 12666 line |
| 29036 | HSA250839 | NM_018401.1 | 754 | cacgcccatcgatgaaatcctca | 85068 | - | see also 12667 line |
| 29037 | HSA250839 | NM_018401.1 | 560 | aacatagccgacgggtagtgaaagg | 85063 | - | see also 12667 line |
| 29038 | HSP70-4 | NM_016299.1 | 309 | tacatcgcggaaagtaaatgtt | 85081 | - | see also 6790 line |
| 29039 | HSP70-4 | NM_016299.1 | 823 | atgcgcgagccatgatgaaatta | 85095 | - | see also 6790 line |
| 29040 | HSPA12B | NM_052970.3 | 2135 | cgcgcgtccatcgactttcttc | 85124 | - | see also 9473 line |
| 29041 | HSPA12B | NM_052970.3 | 2080 | tgggcgacaccgaaattaaggtca | 85123 | - | see also 9473 line |
| 29042 | HSPA1L | NM_005527.2 | 521 | ttcgatggtattgactaagttga | 85137 | - | see also 12670 line |
| 29043 | HSPA1L | NM_005527.2 | 373 | aacgtctgatcggcaggaaattt | 85128 | - | see also 12670 line |
| 29044 | HSPA2 | NM_021979.2 | 1220 | atcctcatcggcgacaaatcaga | 85161 | - | see also 12671 line |
| 29045 | HSPA2 | NM_021979.2 | 492 | aggtgcacagcgcggtcataacg | 85160 | - | see also 12671 line |
| 29046 | HSPA4 | NM_002154.3 | 2759 | gccttcggattcagacaagaagc | 85216 | - | see also 1384 line |
| 29047 | HSPA5 | NM_005347.2 | 1293 | tggctcgactcgaattccaaaga | 85235 | - | see also 3732 line |
| 29048 | HSPA5 | NM_005347.2 | 928 | ttcgaagttgttggccactaatgg | 85226 | - | see also 3732 line |
| 29049 | HSPA6 | NM_002155.2 | 415 | gcccaaggtgcgcgtatgctaacc | 85245 | - | see also 12674 line |
| 29050 | HSPA6 | NM_002155.2 | 746 | tccattgacgctggtgtctttga | 85246 | - | see also 12674 line |
| 29051 | HSPA8 | NM_006597.3 | 970 | acccgtgcccgatttgaagaact | 85263 | - | see also 12675 line |
| 29052 | HSPA8 | NM_006597.3 | 620 | ttgcttacgcttagacacaaaag | 85254 | - | see also 12675 line |
| 29053 | HSPA9B | NM_004134.4 | 948 | ggccttgctacggcacacattgtga | 85288 | - | see also 2791 line |
| 29054 | HSPA9B | NM_004134.4 | 192 | tagctggaatggccttagtcatg | 85272 | - | see also 2791 line |
| 29055 | HSPCB | NM_007355.2 | 2117 | accgcatcatccatgatcaag | 85320 | - | see also 12677 line |
| 29056 | HSPCB | NM_007355.2 | 200 | tccttcgggagttgatctctaat | 85293 | - | see also 12677 line |
| 29057 | HSPD1 | NM_002156.3 | 283 | ctgttgcaaagtcaattgactta | 85325 | - | see also 12678 line |
| 29058 | HSPD1 | NM_002156.3 | 279 | gtgactgttgcaaagtcaattga | 85324 | - | see also 12678 line |
| 29059 | HSPH1 | NM_006644.1 | 490 | tgcaaacaatacggtgtctaact | 85332 | - | see also 4696 line |
| 29060 | HSPH1 | NM_006644.1 | 410 | cggtgcaccccgtcagtcatatc | 85327 | - | see also 4696 line |
| 29061 | HUNK | NM_014586.1 | 1305 | ggcgaatcgctggcttaatgaga | 85389 | - | see also 12680 line |
| 29062 | HUNK | NM_014586.1 | 1303 | ctggcgaatcgctggcttaatga | 85388 | - | see also 12680 line |
| 29063 | HYOU1 | NM_006389.2 | 427 | tccaaaggctacgctacgttact | 85421 | - | see also 12681 line |
| 29064 | HYOU1 | NM_006389.2 | 1083 | gtgaggctaatcgtctcaaaacc | 85434 | - | see also 12681 line |

Figure 46

| 29065 | IARS | NM_002161.2 | 1080 | atcgggtctgtatggactttaac | 85490 | - | see also 1386 line |
|---|---|---|---|---|---|---|---|
| 29066 | IARS | NM_002161.2 | 3050 | gtcatcaatcgcatacagaaact | 85556 | - | see also 1386 line |
| 29067 | ICK | NM_014920.2 | 785 | ggcccgagaaatacgatcaaaac | 85594 | - | see also 6094 line |
| 29068 | ICK | NM_014920.2 | 786 | gcccgagaaatacgatcaaaacc | 85595 | - | see also 6094 line |
| 29069 | ICK | NM_014920.2 | 705 | ggcttctttcatcgagacttaaa | 85592 | - | see also 6094 line |
| 29070 | IDH3G | NM_004135.2 | 556 | ctgccaccgtcgcacaaatctcg | 85633 | - | see also 2792 line |
| 29071 | IDH3G | NM_004135.2 | 448 | gaggtgcacgtgagttccaatgc | 85630 | - | see also 2792 line |
| 29072 | IGF1R | NM_000875.2 | 279 | cacggtcattaccgagtacttgc | 85645 | - | see also 566 line |
| 29073 | IKBKE | NM_014002.1 | 2433 | atcatcgaacggctaaatagagt | 85709 | - | see also 12686 line |
| 29074 | IKBKE | NM_014002.1 | 1546 | acctgcaggcggattacaacact | 85706 | - | see also 12686 line |
| 29075 | ILK | NM_004517.1 | 1112 | cactcaatagccgtagtgtaatg | 85729 | - | see also 3065 line |
| 29076 | ILK | NM_004517.1 | 972 | gccgtatggatccctctacaatg | 85724 | - | see also 3065 line |
| 29077 | ILK | NM_004517.1 | 744 | gacgaagctcaacgagaatcact | 85720 | - | see also 3065 line |
| 29078 | IMMP2L | NM_032549.1 | 655 | tacaccgtggtgacattgtatca | 85738 | - | see also 12688 line |
| 29079 | IMMP2L | NM_032549.1 | 540 | gtcgcctgtgtggcaagagtaga | 85736 | - | see also 12688 line |
| 29080 | IMMP2L | NM_032549.1 | 461 | gtgggtgaaaagatacatcaagg | 85735 | - | see also 12688 line |
| 29081 | INSR | NM_000208.1 | 2952 | acgtcccgtcaaatattgcaaaa | 85788 | - | see also 12690 line |
| 29082 | INSR | NM_000208.1 | 3602 | atggtcgcccatgattttactgt | 85796 | - | see also 12690 line |
| 29083 | INSR | NM_000208.1 | 439 | atcacgactgttctttaactacg | 85748 | - | see also 12690 line |
| 29084 | INSRR | NM_014215.1 | 1180 | cccgtaatagcagcagcatattc | 85805 | - | see also 5539 line |
| 29085 | INSRR | NM_014215.1 | 3179 | tcgggagcagatctcgataatcc | 85819 | - | see also 5539 line |
| 29086 | IPT | NM_017646.3 | 1237 | gcgaatgggcagcgcacataaaa | 85859 | - | see also 7075 line |
| 29087 | IPT | NM_017646.3 | 1202 | gtgtgacctctgtgatcgaatca | 85858 | - | see also 7075 line |
| 29088 | IRAK1 | NM_001569.2 | 2046 | agccaccgcagattatcatcaac | 85869 | - | see also 12693 line |
| 29089 | IRAK1 | NM_001569.2 | 2041 | gtcagagccaccgcagattatca | 85868 | - | see also 12693 line |
| 29090 | IRAK1 | NM_001569.2 | 1358 | cacggtgccaggaccaagtatct | 85866 | - | see also 12693 line |
| 29091 | IRAK2 | NM_001570.2 | 1009 | agcaacgtcaagagctctaatgt | 85888 | - | see also 12694 line |
| 29092 | IRAK2 | NM_001570.2 | 746 | gtccaggatcaatcgaaagattc | 85882 | - | see also 12694 line |
| 29093 | IRAK3 | NM_007199.1 | 856 | cacattcgaatcggtatattaat | 85922 | - | see also 5001 line |
| 29094 | IRAK3 | NM_007199.1 | 347 | atcgtcgagctattcatttaatt | 85905 | - | see also 5001 line |
| 29095 | IRAK3 | NM_007199.1 | 1720 | gacttaaggccctataaggtaaa | 85950 | - | see also 5001 line |
| 29096 | IRAK4 | NM_016123.1 | 264 | ttgactggggcaccacaaattgc | 85957 | - | see also 12696 line |
| 29097 | IRAK4 | NM_016123.1 | 1215 | aacaccgtgaacctcagttattg | 85982 | - | see also 12696 line |
| 29098 | ITGB1BP3 | NM_014446.1 | 211 | tggggaagacggcttcaaacagt | 85992 | - | see also 12697 line |
| 29099 | ITGB1BP3 | NM_014446.1 | 290 | ctgagcagcccgcagaagtttgc | 85993 | - | see also 12697 line |
| 29100 | ITK | NM_005546.3 | 1798 | taccggatttcggttgtacaagc | 86034 | - | see also 12698 line |
| 29101 | ITK | NM_005546.3 | 980 | gacaatcctaagcgatactatgt | 86022 | - | see also 12698 line |

Figure 46

| | | | | | | | |
|---|---|---|---|---|---|---|---|
| 29102 | ITK | NM_005546.3 | 1836 | cacacgtctaccagattatgaat | 86035 | - | see also 12698 line |
| 29103 | JAK1 | NM_002227.1 | 355 | cactaccgatgaggttctattt | 86042 | - | see also 1417 line |
| 29104 | JAK1 | NM_002227.1 | 2509 | atgacccgctgcatgaactatga | 86091 | - | see also 1417 line |
| 29105 | JAK2 | NM_004972.2 | 3251 | tgctggtcggcgtaatctaaaat | 86196 | - | see also 3454 line |
| 29106 | JAK2 | NM_004972.2 | 3250 | gtgctggtcggcgtaatctaaaa | 86195 | - | see also 3454 line |
| 29107 | JAK2 | NM_004972.2 | 3253 | ctggtcggcgtaatctaaaatta | 86197 | - | see also 3454 line |
| 29108 | JAK3 | NM_000215.1 | 3017 | gccgcttgacaaagactactacg | 86247 | - | see also 12700 line |
| 29109 | JAK3 | NM_000215.1 | 1036 | agcaccgcctggtcactgttacc | 86236 | - | see also 12700 line |
| 29110 | JFC1 | NM_032872.1 | 1453 | cctggacacttacgtacaatgc | 86253 | - | - |
| 29111 | JFC1 | NM_032872.1 | 575 | gccaggctcaccattgatgagg | 86251 | - | see also 29110 line |
| 29112 | JFC1 | NM_032872.1 | 1215 | atctttctggcgtgaagttgaagt | 86252 | - | see also 29110 line |
| 29113 | JIK | NM_016281.2 | 1004 | tccccaagttacagtcaatga | 86275 | - | see also 12701 line |
| 29114 | JIK | NM_016281.2 | 1466 | tctccgtcgtgcataagaaaga | 86285 | - | see also 12701 line |
| 29115 | KATNA1 | NM_007044.2 | 1154 | gacgacgcttgagaaacgaatc | 86344 | - | see also 4933 line |
| 29116 | KATNA1 | NM_007044.2 | 594 | tagtaccggatatgataaagact | 86332 | - | see also 4933 line |
| 29117 | KCNJ1 | NM_000220.2 | 611 | aacgtgccaagaccattacgttc | 86371 | - | see also 165 line |
| 29118 | KCNJ1 | NM_000220.2 | 973 | tgccaagtccggacatcctatgt | 86377 | - | see also 165 line |
| 29119 | KCNJ10 | NM_002241.2 | 748 | agcgggctgagaccattcgtttc | 86388 | - | see also 12704 line |
| 29120 | KCNJ10 | NM_002241.2 | 305 | acggcggagagtcctgacacaaag | 86386 | - | see also 12704 line |
| 29121 | KDR | NM_002253.1 | 952 | gtcgttgagggtataggatttta | 86407 | - | see also 12705 line |
| 29122 | KDR | NM_002253.1 | 990 | tccgtctcatggaattgaactat | 86411 | - | see also 12705 line |
| 29123 | KIAA0195 | NM_014738.1 | 4179 | atcgtgaagctaacatgagattcg | 86481 | - | see also 5927 line |
| 29124 | KIAA0195 | NM_014738.1 | 778 | ctggccaggaatcgtttgcttct | 86468 | - | see also 5927 line |
| 29125 | KIAA0703 | NM_014861.1 | 3234 | ttggcctcatccgtcttcatttt | 86507 | - | see also 12707 line |
| 29126 | KIAA0703 | NM_014861.1 | 1292 | ctctcagttcggagaagtgttta | 86486 | - | see also 12707 line |
| 29127 | KIAA0999 | NM_025164.3 | 2512 | aactacgaccaggcgcatttaca | 86553 | - | see also 12708 line |
| 29128 | KIAA0999 | NM_025164.3 | 1422 | aaccgggcaacttgagtacaagg | 86545 | - | see also 12708 line |
| 29129 | KIAA1164 | NM_019092.1 | 823 | ttcccggacaatctgtgtatca | 86572 | - | see also 12709 line |
| 29130 | KIAA1164 | NM_019092.1 | 825 | ccgggacaatctgtgtatcaca | 86573 | - | see also 12709 line |
| 29131 | KIAA1164 | NM_019092.1 | 462 | ggccggagtgggtcatgagttgg | 86569 | - | see also 12709 line |
| 29132 | KIAA1804 | NM_032435.1 | 2758 | atgctcactccgattttgtcc | 86613 | - | see also 12710 line |
| 29133 | KIAA1804 | NM_032435.1 | 1285 | gtccctatcggggcattgatgg | 86584 | - | see also 12710 line |
| 29134 | KIAA1811 | NM_032430.1 | 845 | agctgcgggtcccccattatgc | 86627 | - | see also 12711 line |
| 29135 | KIAA1811 | NM_032430.1 | 349 | acccagcacgcccaatatgtgg | 86623 | - | see also 12711 line |
| 29136 | KIF11 | NM_004523.2 | 242 | agtagcgccattcaatagtgg | 86639 | - | see also 12712 line |
| 29137 | KIF11 | NM_004523.2 | 1116 | ggggtacaagaaacatctataat | 86673 | - | see also 12712 line |
| 29138 | KIF12 | NM_138424.1 | 293 | gtcacccttgcgcctctatct | 86732 | - | see also 12713 line |

Figure 46

| 29139 | KIF12 | NM_138424.1 | 295 | cacccttcgcgcctcttatctgg | 86733 | - | see also 12713 line |
|---|---|---|---|---|---|---|---|
| 29140 | KIF12 | NM_138424.1 | 386 | aacaagactcggggcttctatgt | 86735 | - | see also 12713 line |
| 29141 | KIF13A | NM_022113.3 | 4930 | accatcgcttgtgcatgatttca | 86861 | - | see also 12714 line |
| 29142 | KIF13A | NM_022113.3 | 4929 | caccatcgcttgtgcatgatttc | 86860 | - | see also 12714 line |
| 29143 | KIF13B | NM_015254.1 | 2024 | agcgctcagcaacgcttaagaca | 86917 | - | see also 12715 line |
| 29144 | KIF13B | NM_015254.1 | 3281 | agctaccaggatcgagatttaga | 86941 | - | see also 12715 line |
| 29145 | KIF13B | NM_015254.1 | 2361 | aagataacccagtaatacgatca | 86927 | - | see also 12715 line |
| 29146 | KIF14 | NM_014875.1 | 2526 | accaagcccgtttaatagtcaac | 87044 | - | see also 12716 line |
| 29147 | KIF14 | NM_014875.1 | 2521 | tgctaaccaagcccgtttaatag | 87043 | - | see also 12716 line |
| 29148 | KIF1B | NM_015074.1 | 2292 | tacttcattacgggacttactct | 87174 | - | see also 6222 line |
| 29149 | KIF1B | NM_015074.1 | 5442 | atgcccgagccagtcgaaatact | 87238 | - | see also 6222 line |
| 29150 | KIF2 | NM_004520.1 | 284 | cacgtgcacggcccagtcaattt | 87246 | - | see also 3072 line |
| 29151 | KIF2 | NM_004520.1 | 1903 | gtcgattcatatgctacacaact | 87287 | - | see also 3072 line |
| 29152 | KIF20A | NM_005733.1 | 2649 | ctcacggcgttcccctttactca | 87333 | - | see also 3976 line |
| 29153 | KIF20A | NM_005733.1 | 2042 | tggccctacggcggtcacaaagg | 87324 | - | see also 3976 line |
| 29154 | KIF21A | NM_017641.2 | 2211 | aacttaggctcggtagaatctta | 87402 | - | see also 12720 line |
| 29155 | KIF21A | NM_017641.2 | 1236 | tacgccaatcgagctagaaatat | 87371 | - | see also 12720 line |
| 29156 | KIF21A | NM_017641.2 | 4427 | taccagtcgaacagtagctattc | 87468 | - | see also 12720 line |
| 29157 | KIF25 | NM_005355.2 | 466 | cagcggaaagagctataccatgc | 87499 | - | see also 12721 line |
| 29158 | KIF25 | NM_005355.2 | 968 | aggagccgcagagcttctcaagg | 87504 | - | see also 12721 line |
| 29159 | KIF27 | NM_017576.1 | 1195 | cccgagtcagaccgtatagatga | 87542 | - | see also 12722 line |
| 29160 | KIF27 | NM_017576.1 | 840 | ccctcggcatattgtctcaaagt | 87513 | - | see also 12722 line |
| 29161 | KIF3A | NM_007054.3 | 481 | atggaaggtgttcgagctattcc | 87565 | - | see also 12723 line |
| 29162 | KIF3A | NM_007054.3 | 2110 | cacgtgtatcttgcctatactga | 87613 | - | see also 12723 line |
| 29163 | KIF3B | NM_004798.2 | 795 | gagcacagctcgcgttctcatgc | 87629 | - | see also 12724 line |
| 29164 | KIF3B | NM_004798.2 | 392 | gacgttccgaccacttgttgact | 87619 | - | see also 12724 line |
| 29165 | KIF3C | NM_002254.4 | 756 | acctttgacgccgtgtatgatgc | 87654 | - | see also 1449 line |
| 29166 | KIF3C | NM_002254.4 | 809 | aaccgtgaggcccctgatagact | 87655 | - | see also 1449 line |
| 29167 | KIF5A | NM_004984.1 | 2067 | tccgctcgcttacggaatacatg | 87707 | - | see also 3474 line |
| 29168 | KIF5A | NM_004984.1 | 1574 | gagaacgatgccgctaaggatga | 87697 | - | see also 3474 line |
| 29169 | KIF5B | NM_004521.1 | 1892 | tcggcaactttagcgagtataga | 87773 | - | see also 3075 line |
| 29170 | KIF5B | NM_004521.1 | 1526 | accgcaattggagttataggaaa | 87766 | - | see also 3075 line |
| 29171 | KIF5C | NM_004522.1 | 2523 | tccagactccgagacgaaattga | 87825 | - | see also 3081 line |
| 29172 | KIF5C | NM_004522.1 | 695 | cccacgaattgcccatgatatct | 87803 | - | see also 3081 line |
| 29173 | KIF9 | NM_022342.2 | 207 | aacagacagactggtcgtttaag | 87838 | - | see also 8335 line |
| 29174 | KIF9 | NM_022342.2 | 1633 | tagtgagatcaaccgaattttca | 87864 | - | see also 8335 line |
| 29175 | KIFC2 | NM_145754.2 | 80 | ctcgttgctcatctacatcttct | 87879 | - | see also 12730 line |

Figure 46

| 29176 | KIFC2 | NM_145754.2 | 2293 | gacagtctgctccctcaagttcg | 87881 | - | see also 12730 line |
|---|---|---|---|---|---|---|---|
| 29177 | KIFC3 | NM_005550.2 | 1883 | agcgctgcggagatctacaatga | 87888 | - | see also 12731 line |
| 29178 | KIFC3 | NM_005550.2 | 1562 | accaatgctgtgactttcgatgc | 87884 | - | see also 12731 line |
| 29179 | KIT | NM_000222.1 | 164 | tccgcgtgggcgacgagattagg | 87894 | - | see also 166 line |
| 29180 | KIT | NM_000222.1 | 2157 | ttcctgcagcgatagtactaatg | 87961 | - | see also 166 line |
| 29181 | KPI2 | NM_014916.2 | 2005 | tgctcacaaccgacatggataat | 88036 | - | see also 12733 line |
| 29182 | KPI2 | NM_014916.2 | 4353 | aaggcagtcacgtttttcgatga | 88074 | - | see also 12733 line |
| 29183 | KSR2 | NM_173598.2 | 1419 | tccggtgacctcgaatccaatct | 88096 | - | see also 12734 line |
| 29184 | KSR2 | NM_173598.2 | 554 | ccgcgctgcatcggagcaaatcc | 88084 | - | see also 12734 line |
| 29185 | LAK | NM_025144.2 | 785 | ctctccacgtcgctaggtatact | 88115 | - | see also 12735 line |
| 29186 | LAK | NM_025144.2 | 787 | ctccacgtcgctaggtatactgg | 88116 | - | see also 12735 line |
| 29187 | LARS | NM_020117.8 | 2801 | gactacgactcaagaactatatg | 88257 | - | see also 12736 line |
| 29188 | LARS | NM_020117.8 | 163 | gaggtcaatgcatctaatttaga | 88187 | - | see also 12736 line |
| 29189 | LARS2 | NM_015340.2 | 604 | gccgcagtcgagaggaatctaca | 88287 | - | see also 6381 line |
| 29190 | LARS2 | NM_015340.2 | 1882 | gccgtcatgcacttgttctatgc | 88322 | - | see also 6381 line |
| 29191 | LARS2 | NM_015340.2 | 714 | aactacgtgtttgccagattact | 88289 | - | see also 6381 line |
| 29192 | LATS1 | NM_004690.2 | 1395 | tacgtaatctcccgaatctctcc | 88366 | - | see also 12738 line |
| 29193 | LATS1 | NM_004690.2 | 2512 | agcagcgtctacatcgtaaaaaa | 88412 | - | see also 12738 line |
| 29194 | LATS2 | NM_014572.1 | 2165 | ctcgccatacgcctttaagttct | 88471 | - | see also 12739 line |
| 29195 | LATS2 | NM_014572.1 | 3472 | ctgagcacgcattttacgaattc | 88488 | - | see also 12739 line |
| 29196 | LATS2 | NM_014572.1 | 1001 | gccgtacgtggactacctttcc | 88467 | - | see also 12739 line |
| 29197 | LCK | NM_005356.2 | 668 | tccgccattacaccaatgcttca | 88497 | - | see also 12740 line |
| 29198 | LCK | NM_005356.2 | 1383 | gaccaacccggaggtgattcaga | 88501 | - | see also 12740 line |
| 29199 | LCK | NM_005356.2 | 420 | gcccgaaccctggttcttcaaga | 88493 | - | see also 12740 line |
| 29200 | LIMK1 | NM_002314.2 | 247 | tgcggccagaggatctatgatgg | 88505 | - | see also 1489 line |
| 29201 | LIMK1 | NM_002314.2 | 246 | ctgcggccagaggatctatgatg | 88504 | - | see also 1489 line |
| 29202 | LIMK2 | NM_005569.2 | 992 | tccctaaggcgcagtaacagtat | 88529 | - | see also 12742 line |
| 29203 | LIMK2 | NM_005569.2 | 993 | ccctaaggcgcagtaacagtatc | 88530 | - | see also 12742 line |
| 29204 | LIMK2 | NM_005569.2 | 1217 | gtcatgaaagagttaattcgatg | 88533 | - | see also 12742 line |
| 29205 | LOC115704 | NM_145245.2 | 1630 | gcctcgacgcgagagcttaaact | 88557 | - | see also 9783 line |
| 29206 | LOC115704 | NM_145245.2 | 2118 | ctcggactcatcgcagtacatcc | 88559 | - | see also 9783 line |
| 29207 | LOC115704 | NM_145245.2 | 1632 | ctcgacgcgagagcttaaactgc | 88558 | - | see also 9783 line |
| 29208 | LOC116143 | NM_138458.1 | 435 | tggcataggtggactaggaattg | 88566 | - | see also 9623 line |

Figure 46

| 29209 | LOC116143 | NM_138458.1 | 436 | ggcataggtggactaggaattgg | 88567 | - | see also 9623 line |
|---|---|---|---|---|---|---|---|
| 29210 | LOC200383 | NM_145299.1 | 684 | agcaccgggaaacaaacgaattg | 88627 | - | see also 12745 line |
| 29211 | LOC200383 | NM_145299.1 | 452 | cagtgcgctatgggtatctaatg | 88615 | - | see also 12745 line |
| 29212 | LOC84643 | NM_032559.2 | 842 | caccgtcccctcggacaatgtgg | 88669 | - | see also 12747 line |
| 29213 | LOC84643 | NM_032559.2 | 1091 | aacggcccaagattgttctaagg | 88672 | - | see also 12747 line |
| 29214 | LOC84643 | NM_032559.2 | 851 | ctcggacaatgtggttatggtgc | 88670 | - | see also 12747 line |
| 29215 | LOC91807 | NM_182493.1 | 2413 | aacacgagtggctgaataatttg | 88704 | - | see also 12748 line |
| 29216 | LOC91807 | NM_182493.1 | 2154 | gccccagaagtcgtcaattatga | 88697 | - | see also 12748 line |
| 29217 | LOC91807 | NM_182493.1 | 2156 | cccagaagtcgtcaattatgagt | 88698 | - | see also 12748 line |
| 29218 | LOC91862 | NM_052858.1 | 768 | cagttcggaggggcttacagtgg | 88713 | - | see also 12749 line |
| 29219 | LOC91862 | NM_052858.1 | 524 | accccttcggagagatatctgc | 88708 | - | see also 12749 line |
| 29220 | LOC92935 | NM_138395.1 | 524 | ggcgtctatgaaggttggtattg | 88721 | - | see also 9615 line |
| 29221 | LOC92935 | NM_138395.1 | 1473 | gtgtccggcaaactaatggtttt | 88742 | - | see also 9615 line |
| 29222 | LONP | NM_031490.2 | 2526 | ggcaacgtacgacaggatttaag | 88806 | - | see also 9040 line |
| 29223 | LONP | NM_031490.2 | 2529 | aacgtacgacaggatttaagttt | 88807 | - | see also 9040 line |
| 29224 | LTK | NM_002344.1 | 2236 | ggcctgtgtaccgcatcatgacc | 88824 | - | see also 12752 line |
| 29225 | LTK | NM_002344.1 | 1498 | aggtttccccagccaatgttact | 88818 | - | see also 12752 line |
| 29226 | LYK5 | NM_153335.3 | 454 | gactgtacggaggattaacctag | 88832 | - | see also 9988 line |
| 29227 | LYK5 | NM_153335.3 | 545 | cccaatatcgtgccatatcgagc | 88835 | - | see also 9988 line |
| 29228 | LYN | NM_002350.1 | 530 | acccggacgacttgtctttcaag | 88856 | - | see also 12754 line |
| 29229 | LYN | NM_002350.1 | 531 | cccggacgacttgtctttcaaga | 88857 | - | see also 12754 line |
| 29230 | MAK | NM_005906.3 | 1195 | gcctctgccggatataatcgatc | 88916 | - | see also 12755 line |
| 29231 | MAK | NM_005906.3 | 1410 | ggcgttggggtcagactatcttc | 88918 | - | see also 12755 line |
| 29232 | MAP2K1 | NM_002755.2 | 114 | ccccgacggctctgcagttaacg | 88938 | - | see also 1798 line |
| 29233 | MAP2K1 | NM_002755.2 | 983 | gccgacctcccatggcaattttt | 88949 | - | see also 1798 line |
| 29234 | MAP2K2 | NM_030662.2 | 965 | gcggttgcagggcacacattact | 88963 | - | see also 12757 line |
| 29235 | MAP2K2 | NM_030662.2 | 964 | agcggttgcagggcacacattac | 88962 | - | see also 12757 line |
| 29236 | MAP2K3 | NM_002756.2 | 626 | ttctacggggcactattcagaga | 88970 | - | see also 1799 line |
| 29237 | MAP2K3 | NM_002756.2 | 594 | tgcgcacggtcgactgtttctac | 88967 | - | see also 1799 line |
| 29238 | MAP2K4 | NM_003010.2 | 901 | agcgcatcacgacaaggatatga | 88975 | - | see also 2016 line |
| 29239 | MAP2K4 | NM_003010.2 | 1195 | gaggtcgcatgctatgtttgtaa | 88976 | - | see also 2016 line |
| 29240 | MAP2K4 | NM_003010.2 | 900 | aagcgcatcacgacaaggatatg | 88974 | - | see also 2016 line |
| 29241 | MAP2K5 | NM_002757.2 | 1785 | ttctcggagccatttgtacattt | 89009 | - | see also 1801 line |
| 29242 | MAP2K5 | NM_002757.2 | 1297 | ttctttataagtgcgattcatca | 88994 | - | see also 1801 line |

Figure 46

| | | | | | | | |
|---|---|---|---|---|---|---|---|
| 29243 | MAP2K6 | NM_002758.2 | 875 | gacgtcaagccttctaatgtact | 89037 | - | see also 12760 line |
| 29244 | MAP2K6 | NM_002758.2 | 1234 | ttccaaagaacggcctacatacc | 89049 | - | see also 12760 line |
| 29245 | MAP2K7 | NM_145185.2 | 495 | cacgtcattgccgttaagcaaat | 89052 | - | see also 12761 line |
| 29246 | MAP2K7 | NM_145185.2 | 626 | caccaacacggacgtcttcatcg | 89054 | - | see also 12761 line |
| 29247 | MAP3K10 | NM_002446.2 | 1222 | gcgtatggcgtggctatgaataa | 89061 | - | see also 12762 line |
| 29248 | MAP3K10 | NM_002446.2 | 789 | cccacacctctgcctagtgatgg | 89058 | - | see also 12762 line |
| 29249 | MAP3K11 | NM_002419.2 | 1452 | tgccataccgtggcattgactgc | 89067 | - | see also 12763 line |
| 29250 | MAP3K11 | NM_002419.2 | 2804 | ccccttcgcagccgcattgatcc | 89072 | - | see also 12763 line |
| 29251 | MAP3K12 | NM_006301.2 | 1304 | ctgtctgcaccgcctagaagagg | 89082 | - | see also 4392 line |
| 29252 | MAP3K13 | NM_004721.3 | 2007 | agcagcaagagccgatatcgaag | 89134 | - | see also 12765 line |
| 29253 | MAP3K13 | NM_004721.3 | 1656 | ctcaggcatgcgctggatattcg | 89127 | - | see also 12765 line |
| 29254 | MAP3K14 | NM_003954.1 | 557 | agccagtccgagagtcttgatca | 89161 | - | see also 2701 line |
| 29255 | MAP3K14 | NM_003954.1 | 1201 | gtctcgtggtgcccatgagaagt | 89166 | - | see also 2701 line |
| 29256 | MAP3K2 | NM_006609.2 | 329 | tggacagtctatggatctacatt | 89186 | - | see also 4667 line |
| 29257 | MAP3K3 | NM_002401.2 | 1618 | aacgcctgcagacgatctgtatg | 89275 | - | see also 12767 line |
| 29258 | MAP3K3 | NM_002401.2 | 969 | ccccgaatacggcgtcatcaagg | 89263 | - | see also 12767 line |
| 29259 | MAP3K4 | NM_005922.1 | 908 | tggcttaaccgatctaacgaact | 89306 | - | see also 4139 line |
| 29260 | MAP3K4 | NM_005922.1 | 2287 | acctcaagcatcgcatagtttaa | 89348 | - | see also 4139 line |
| 29261 | MAP3K5 | NM_005923.3 | 3319 | acccgacacggagttgaaagtgg | 89500 | - | see also 4141 line |
| 29262 | MAP3K5 | NM_005923.3 | 3316 | ttcacccgacacggagttgaaag | 89499 | - | see also 4141 line |
| 29263 | MAP3K6 | NM_004672.2 | 2620 | cccgaagcgctgcctcagttatg | 89547 | - | see also 3207 line |
| 29264 | MAP3K6 | NM_004672.2 | 886 | ctggtatcgcaaggcttttgacg | 89537 | - | see also 3207 line |
| 29265 | MAP3K6 | NM_004672.2 | 980 | tccaaagagctccggctaatagg | 89538 | - | see also 3207 line |
| 29266 | MAP3K7 | NM_003188.2 | 748 | aacccaaagcgctaattcacagg | 89569 | - | see also 12771 line |
| 29267 | MAP3K7 | NM_003188.2 | 1918 | ctcgcctggtacaggaacataaa | 89596 | - | see also 12771 line |
| 29268 | MAP3K8 | NM_005204.2 | 862 | aacgatgagcgttctaagtctct | 89604 | - | see also 12772 line |
| 29269 | MAP3K8 | NM_005204.2 | 1182 | aagaatggcgtgtaaactgatcc | 89613 | - | see also 12772 line |
| 29270 | MAP4K1 | NM_007181.2 | 1123 | gagtcgtctgacgatgactatga | 89642 | - | see also 12773 line |
| 29271 | MAP4K1 | NM_007181.2 | 223 | gtggcctaccatgggagttatct | 89635 | - | see also 12773 line |
| 29272 | MAP4K2 | NM_004579.2 | 2255 | ctgtgtgaggattgtcaacatgc | 89664 | - | see also 12774 line |
| 29273 | MAP4K2 | NM_004579.2 | 848 | ggccctgaccaagaatcctaaga | 89660 | - | see also 12774 line |
| 29274 | MAP4K3 | NM_003618.2 | 1309 | aggacacgtcgcacatttagaag | 89702 | - | see also 12775 line |
| 29275 | MAP4K3 | NM_003618.2 | 185 | aaggcacggaatgttaacactgg | 89666 | - | see also 12775 line |
| 29276 | MAP4K5 | NM_006575.3 | 2629 | ttcgcattgaatctgtagtatgc | 89803 | - | see also 4608 line |
| 29277 | MAP4K5 | NM_006575.3 | 2411 | atcgaatcaggtagttcagtttg | 89794 | - | see also 4608 line |
| 29278 | MAPK1 | NM_002745.2 | 352 | atggtgtgctctgcttatgataa | 89812 | - | see also 1787 line |
| 29279 | MAPK1 | NM_002745.2 | 515 | caccaaccatcgagcaaatgaaa | 89818 | - | see also 1787 line |

Figure 46

| | | | | | | | |
|---|---|---|---|---|---|---|---|
| 29280 | MAPK10 | NM_002753.2 | 1186 | ctccgagcacaataaactcaaag | 89861 | - | see also 12778 line |
| 29281 | MAPK10 | NM_002753.2 | 1180 | agcggactccgagcacaataaac | 89859 | - | see also 12778 line |
| 29282 | MAPK11 | NM_002751.4 | 813 | gccctgaggttctggcaaaatc | 89868 | - | see also 12779 line |
| 29283 | MAPK12 | NM_002969.2 | 571 | gggctgctggacgtattcactcc | 89869 | - | see also 12780 line |
| 29284 | MAPK12 | NM_002969.2 | 1327 | tggaagcgtgttacttaccaaaga | 89874 | - | see also 12780 line |
| 29285 | MAPK13 | NM_002754.3 | 381 | tcctccctgcgcaacttcatga | 89878 | - | see also 12781 line |
| 29286 | MAPK13 | NM_002754.3 | 1076 | ttccttagaacacgagaaaactca | 89882 | - | see also 12781 line |
| 29287 | MAPK14 | NM_001315.1 | 806 | cagggacctaaaacctagtaatc | 89902 | - | see also 12782 line |
| 29288 | MAPK14 | NM_001315.1 | 574 | aactgcggttacttaaacatatg | 89886 | - | see also 12782 line |
| 29289 | MAPK14 | NM_001315.1 | 543 | tccatcattcatgcgaaaagaac | 89884 | - | see also 12782 line |
| 29290 | MAPK3 | NM_002746.1 | 991 | ctggaccgatgttaacctttaa | 89927 | - | see also 12783 line |
| 29291 | MAPK3 | NM_002746.1 | 553 | aacgtgctccacgagatctaaa | 89921 | - | see also 12783 line |
| 29292 | MAPK4 | NM_002747.2 | 673 | acgcgctccgagagatcaagatc | 89933 | - | see also 1788 line |
| 29293 | MAPK4 | NM_002747.2 | 539 | cggtgggcgctttgttgacttcc | 89932 | - | see also 1788 line |
| 29294 | MAPK6 | NM_002748.2 | 2069 | tggagttgagccatacttgtaac | 89979 | - | see also 1789 line |
| 29295 | MAPK6 | NM_002748.2 | 2813 | ttggtcaccacttaagtcaata | 89996 | - | see also 1789 line |
| 29296 | MAPK7 | NM_002749.2 | 1114 | gggtcgcatgctgcgttttgagc | 90019 | - | see also 1790 line |
| 29297 | MAPK7 | NM_002749.2 | 772 | gcccgctgaacatcagtacttca | 90007 | - | see also 1790 line |
| 29298 | MAPK7 | NM_002749.2 | 1474 | tgcaactgacaccattgatctga | 90021 | - | see also 1790 line |
| 29299 | MAPK8 | NM_002750.2 | 781 | cagtaaggacttacgttgaaaac | 90049 | - | see also 1791 line |
| 29300 | MAPK8 | NM_002750.2 | 474 | aagcccagtaatatagtagtaaa | 90043 | - | see also 1791 line |
| 29301 | MAPK9 | NM_002752.3 | 261 | atcgtgaacttgtctcttaaaa | 90069 | - | see also 1795 line |
| 29302 | MAPK9 | NM_002752.3 | 567 | cccggacagcgtgcactaactc | 90081 | - | see also 1795 line |
| 29303 | MAPKAPK 2 | NM_004759.3 | 1122 | tccgaatgggccagtatgaattt | 90111 | - | see also 12789 line |
| 29304 | MAPKAPK 2 | NM_004759.3 | 708 | tggacggtgggagaactctttagc | 90102 | - | see also 12789 line |
| 29305 | MAPKAPK 3 | NM_004635.3 | 653 | gtgcttaagctcaacgattttgg | 90123 | - | see also 3149 line |
| 29306 | MAPKAPK 3 | NM_004635.3 | 1012 | cccctggatcaaccaattcgatgg | 90127 | - | see also 3149 line |
| 29307 | MAPKAPK 5 | NM_003668.2 | 998 | agccggactatcccaaaggatat | 90158 | - | see also 2496 line |
| 29308 | MAPKAPK 5 | NM_003668.2 | 892 | ctcaccgacgccctacacttaca | 90152 | - | see also 2496 line |
| 29309 | MAPKAPK 5 | NM_003668.2 | 898 | gacgccctacacttaccaacaaga | 90153 | - | see also 2496 line |
| 29310 | MARK1 | NM_018650.2 | 2393 | agcgacgcagcgttgcttataat | 90222 | - | see also 7536 line |

Figure 46

| | | | | | | | |
|---|---|---|---|---|---|---|---|
| 29311 | MARK1 | NM_018650.2 | 2061 | cacagatcgatacgtagcattgc | 90218 | - | see also 7536 line |
| 29312 | MARK1 | NM_018650.2 | 2576 | cggcgttctttgcggttcacatgg | 90225 | - | see also 7536 line |
| 29313 | MARK2 | NM_004954.2 | 2390 | ctctctcaacgggttcgattta | 90255 | - | see also 12793 line |
| 29314 | MARK2 | NM_004954.2 | 1883 | agccccagaccgaactaacttcc | 90253 | - | see also 12793 line |
| 29315 | MARK2 | NM_004954.2 | 2392 | ctctcaacgggttcgatttaag | 90256 | - | see also 12793 line |
| 29316 | MARK3 | NM_002376.3 | 2282 | gccgaggctccactaatctctt | 90307 | - | see also 1529 line |
| 29317 | MARK3 | NM_002376.3 | 2469 | cgccaataactgcgactatgagc | 90315 | - | see also 1529 line |
| 29318 | MARK4 | NM_031417.2 | 483 | gccaagttccgacagatgtttc | 90321 | - | see also 9016 line |
| 29319 | MARK4 | NM_031417.2 | 1261 | agcgcaggcatagcgatttctgt | 90330 | - | see also 9016 line |
| 29320 | MARK4 | NM_031417.2 | 316 | tccgagaagtccgcatcatgaag | 90318 | - | see also 9016 line |
| 29321 | MAST205 | NM_015112.1 | 2111 | gacatggtcggtctatactttgc | 90363 | - | see also 6250 line |
| 29322 | MAT1A | NM_000429.1 | 850 | gtgtcactgccgtaagattatt | 90391 | - | see also 12796 line |
| 29323 | MAT1A | NM_000429.1 | 1042 | tggccgacccgtgtccatttcc | 90395 | - | see also 12796 line |
| 29324 | MAT2A | NM_005911.3 | 630 | tggttacgccctgattctaaaac | 90407 | - | see also 12797 line |
| 29325 | MAT2A | NM_005911.3 | 629 | ttggttacgccctgattctaaaa | 90406 | - | see also 12797 line |
| 29326 | MAT2A | NM_005911.3 | 975 | gtcgaccgttcagctgcttatgc | 90414 | - | see also 12797 line |
| 29327 | MATK | NM_002378.2 | 1603 | gacgggtccgtaccctaaaatg | 90430 | - | see also 1531 line |
| 29328 | MATK | NM_002378.2 | 1604 | acgggctccgtaccctaaaatgt | 90431 | - | see also 1531 line |
| 29329 | MATK | NM_002378.2 | 864 | gacggccacctcacaaatcgatga | 90424 | - | see also 1531 line |
| 29330 | MCCC1 | NM_020166.2 | 2256 | cactcctttagtcgagtttgagg | 90493 | - | see also 7778 line |
| 29331 | MCCC1 | NM_020166.2 | 1402 | gacgaagtttccgtgcattatga | 90468 | - | see also 7778 line |
| 29332 | MDN1 | NM_014611.1 | 11108 | atcgctgtgaacacttctacc | 90748 | - | see also 5779 line |
| 29333 | MDN1 | NM_014611.1 | 2202 | caccgtttgaggggttgtcaatat | 90541 | - | see also 5779 line |
| 29334 | MELK | NM_014791.1 | 1540 | tactcactacgccaaatcgttac | 90900 | - | see also 12801 line |
| 29335 | MELK | NM_014791.1 | 1272 | gtggcgggattaatagactatga | 90890 | - | see also 12801 line |
| 29336 | MERTK | NM_006343.1 | 1804 | gtcggcgagccattgaacttacc | 90959 | - | see also 4437 line |
| 29337 | MERTK | NM_006343.1 | 699 | cccatctacatcgaagtacaagg | 90932 | - | see also 4437 line |
| 29338 | MET | NM_000245.1 | 815 | tccattgcattcgatatcagtga | 91016 | - | see also 175 line |
| 29339 | MET | NM_000245.1 | 3246 | gactaccgagctacttttccaga | 91090 | - | see also 175 line |
| 29340 | MET | NM_000245.1 | 1454 | agctttgcagcgcgttgacttat | 91038 | - | see also 175 line |
| 29341 | MGC13186 | NM_032324.1 | 329 | atgtggtcgacctgacttcttt | 91132 | - | see also 12803 line |
| 29342 | MGC13186 | NM_032324.1 | 331 | gtggtcgacctgacttctttga | 91133 | - | see also 12803 line |
| 29343 | MGC14560 | NM_016301.2 | 147 | aaccggctgtccaagttgtaaa | 91137 | - | see also 12804 line |

Figure 46

| | | | | | | | |
|---|---|---|---|---|---|---|---|
| 29344 | MGC14560 | NM_016301.2 | 148 | accggtctgtccaagttgtaaac | 91138 | - | see also 12804 line |
| 29345 | MGC14560 | NM_016301.2 | 704 | agctatatgtggactgattgatg | 91160 | - | see also 12804 line |
| 29346 | MGC16169 | NM_033115.2 | 2314 | ggcagaagttcggcaccttattt | 91222 | - | see also 12805 line |
| 29347 | MGC16169 | NM_033115.2 | 1676 | atcagtacgatgaactgttatca | 91198 | - | see also 12805 line |
| 29348 | MGC22688 | NM_145001.1 | 367 | gtgcgtggagcgcaatgaagtga | 91234 | - | see also 12806 line |
| 29349 | MGC22688 | NM_145001.1 | 505 | gggtggagacctgcgttatcacc | 91236 | - | see also 12806 line |
| 29350 | MGC2599 | NM_032116.2 | 472 | ggcgtcccaatcgagaagtaaga | 91243 | - | see also 9075 line |
| 29351 | MGC2599 | NM_032116.2 | 1431 | cggcgtatcaatggcttaagtcc | 91263 | - | see also 9075 line |
| 29352 | MGC2744 | NM_025267.2 | 759 | aggttctggtccgggtagattgg | 91282 | - | see also 12808 line |
| 29353 | MGC2744 | NM_025267.2 | 1063 | tgggcccattcgggttgttaaca | 91287 | - | see also 12808 line |
| 29354 | MGC33182 | NM_145203.2 | 1025 | atccttaggctacgttttcatgt | 91299 | - | see also 12809 line |
| 29355 | MGC33182 | NM_145203.2 | 1021 | tggaatccttaggctacgttttc | 91298 | - | see also 12809 line |
| 29356 | MGC42105 | NM_153361.2 | 2028 | atcacaggggtctatagaattat | 91318 | - | see also 12810 line |
| 29357 | MGC42105 | NM_153361.2 | 1699 | cagaaaccgtggccaaactaaaa | 91311 | - | see also 12810 line |
| 29358 | MGC45428 | NM_152619.1 | 978 | ccctctcgacgcagcaaatcacc | 91349 | - | see also 9905 line |
| 29359 | MGC4796 | NM_032017.1 | 750 | gaccgcacctgtgaaatcgttga | 91382 | - | see also 12812 line |
| 29360 | MGC4796 | NM_032017.1 | 778 | cagaatccagccggatggttaag | 91383 | - | see also 12812 line |
| 29361 | MGC5347 | NM_024063.1 | 2216 | ctgtaaaaccgtcgttaagttgc | 91425 | - | see also 12813 line |
| 29362 | MGC5347 | NM_024063.1 | 1388 | ttcgaagcgtcattggattaatg | 91402 | - | see also 12813 line |
| 29363 | MGC5347 | NM_024063.1 | 1373 | ttcagccctcatcgtttcgaagc | 91401 | - | see also 12813 line |
| 29364 | MGC8407 | NM_024046.2 | 869 | ctggctggtgactatgagtttga | 91436 | - | see also 8578 line |
| 29365 | MGC8407 | NM_024046.2 | 793 | aggcaatccacctttctatgagg | 91434 | - | see also 8578 line |
| 29366 | MIDORI | NM_020778.1 | 2507 | ttccggaagtcaaggtatcattg | 91467 | - | see also 7962 line |
| 29367 | MIDORI | NM_020778.1 | 4785 | cccgtagactgcggtgtgtatcg | 91474 | - | see also 7962 line |
| 29368 | MIDORI | NM_020778.1 | 5204 | gagtcgggagtactgcaaaatct | 91478 | - | see also 7962 line |
| 29369 | MKI67 | NM_002417.2 | 558 | cacgtcgtgtctcaagatctagc | 91497 | - | see also 12816 line |
| 29370 | MKI67 | NM_002417.2 | 1977 | aacgccgtaggtcctgcaaaaca | 91512 | - | see also 12816 line |
| 29371 | MKKS | NM_018848.2 | 1347 | gtgcgtagtatattaacaagtaa | 91603 | - | see also 7603 line |

Figure 46

| 29372 | MKKS | NM_018848.2 | 1348 | tgcgtagtatattaacaagtaaa | 91604 | - | see also 7603 line |
|---|---|---|---|---|---|---|---|
| 29373 | MKNK1 | NM_003684.3 | 977 | cacggaccaggccacattctacg | 91659 | - | see also 12818 line |
| 29374 | MKNK1 | NM_003684.3 | 543 | ttgcaaggaggttccatcttagc | 91652 | - | see also 12818 line |
| 29375 | MKNK2 | NM_017572.2 | 888 | aggtctcccccgtgaagatctgt | 91663 | - | see also 12819 line |
| 29376 | MKNK2 | NM_017572.2 | 657 | acgtcctagagctgattgagttc | 91661 | - | see also 12819 line |
| 29377 | MLH1 | NM_000249.2 | 847 | aaccatcgtctggtagaatcaac | 91691 | - | see also 182 line |
| 29378 | MLH1 | NM_000249.2 | 848 | accatcgtctggtagaatcaact | 91692 | - | see also 182 line |
| 29379 | MORC | NM_014429.1 | 1711 | aacttagagagtcggtcataaag | 91765 | - | see also 5675 line |
| 29380 | MORC | NM_014429.1 | 1719 | gagtcggtcataaagtatcaaaa | 91767 | - | see also 5675 line |
| 29381 | MOS | NM_005372.1 | 421 | ttcggtggcaacgtcactttaca | 91798 | - | see also 12822 line |
| 29382 | MOS | NM_005372.1 | 327 | agcaaggctgcgccacgataaca | 91796 | - | see also 12822 line |
| 29383 | MOS | NM_005372.1 | 768 | gacgcctaaagccgacatttatt | 91803 | - | see also 12822 line |
| 29384 | MOV10 | NM_020963.1 | 2405 | cacggcgtaatgggcaaagatga | 91838 | - | see also 8052 line |
| 29385 | MOV10 | NM_020963.1 | 1911 | aggaataccgggtcttaattacc | 91831 | - | see also 8052 line |
| 29386 | MPHOSPH1 | NM_016195.1 | 5359 | aacgacgacttcgaacaaaaaca | 91989 | - | see also 12824 line |
| 29387 | MPHOSPH1 | NM_016195.1 | 4626 | atcaagcccaaacgtattagttc | 91963 | - | see also 12824 line |
| 29388 | MPHOSPH1 | NM_016195.1 | 333 | tgcatccttggtcggttaagtga | 91851 | - | see also 12824 line |
| 29389 | MST1R | NM_002447.1 | 1327 | ctcacgtgtggacctattcaatg | 92002 | - | see also 12825 line |
| 29390 | MST1R | NM_002447.1 | 3312 | ttgtctaccacggagaatacata | 92020 | - | see also 12825 line |
| 29391 | MST4 | NM_016542.2 | 868 | gccacctaactccgatatgcatc | 92056 | - | see also 6908 line |
| 29392 | MST4 | NM_016542.2 | 357 | cccagcaagtcgttgctattaaa | 92047 | - | see also 6908 line |
| 29393 | MT | NM_014507.1 | 389 | gccgctgtcgagaaactacatca | 92075 | - | see also 12827 line |
| 29394 | MT | NM_014507.1 | 391 | cgctgtcgagaaactacatcacc | 92076 | - | see also 12827 line |
| 29395 | MTHFD1 | NM_005956.2 | 1721 | acggacggcccagtttgatatct | 92127 | - | see also 4162 line |
| 29396 | MTHFD1 | NM_005956.2 | 1720 | cacggacggcccagtttgatatc | 92126 | - | see also 4162 line |
| 29397 | MUSK | NM_005592.1 | 2540 | tacaatctcatgcgtctatgttg | 92183 | - | see also 3882 line |
| 29398 | MUSK | NM_005592.1 | 2593 | caccagtattcaccgaattctgg | 92185 | - | see also 3882 line |
| 29399 | MVD | NM_002461.1 | 26 | ctggcggcagtcacttgtacagc | 92187 | - | see also 12830 line |
| 29400 | MVD | NM_002461.1 | 24 | cgctggcggcagtcacttgtaca | 92186 | - | see also 12830 line |
| 29401 | MVK | NM_000431.1 | 229 | cagcttacccaacattggtatca | 92196 | - | see also 12831 line |
| 29402 | MVK | NM_000431.1 | 612 | aggaggatttggagctaattaac | 92202 | - | see also 12831 line |
| 29403 | MYH1 | NM_005963.2 | 2245 | ctcctggggtccattgacattga | 92205 | - | see also 12832 line |
| 29404 | MYH1 | NM_005963.2 | 4435 | cgctcactcagcacagaactatt | 92207 | - | see also 12832 line |
| 29405 | MYH11 | NM_002474.1 | 4590 | aactcgagcggaccaacaaaatg | 92238 | - | see also 12833 line |

Figure 46

| | | | | | | | |
|---|---|---|---|---|---|---|---|
| 29406 | MYH11 | NM_002474.1 | 418 | ctcagggctaatatatacgtact | 92213 | - | see also 12833 line |
| 29407 | MYH13 | NM_003802.1 | 1048 | gtcacggtagccagtatcgatga | 92257 | - | see also 12834 line |
| 29408 | MYH13 | NM_003802.1 | 3848 | gccgacggtagaagatcaattt | 92282 | - | see also 12834 line |
| 29409 | MYH13 | NM_003802.1 | 3847 | tgccgacggtagaagatcaatt | 92281 | - | see also 12834 line |
| 29410 | MYH2 | NM_017534.1 | 68 | aacgcattgaggcccagaattagg | 92289 | - | see also 12835 line |
| 29411 | MYH2 | NM_017534.1 | 1758 | tggttgttggactacacaacatta | 92295 | - | see also 12835 line |
| 29412 | MYH3 | NM_002470.1 | 5814 | tgcggatatcgcagaatctcaag | 92333 | - | see also 12836 line |
| 29413 | MYH3 | NM_002470.1 | 5839 | aacaagctccgcctaagactcg | 92335 | - | see also 12836 line |
| 29414 | MYH4 | NM_017533.1 | 4434 | tgcctgcatagcttctcgataaga | 92357 | - | see also 12837 line |
| 29415 | MYH4 | NM_017533.1 | 4437 | ctgcatagctctcgataagaagc | 92358 | - | see also 12837 line |
| 29416 | MYH6 | NM_002471.1 | 5848 | gtgacattggtgccaagcaaaaa | 92372 | - | see also 12838 line |
| 29417 | MYH6 | NM_002471.1 | 2466 | cgcattgagttcaagaagatagt | 92370 | - | see also 12838 line |
| 29418 | MYH6 | NM_002471.1 | 2016 | tcggctctccacggggaaaatct | 92368 | - | see also 12838 line |
| 29419 | MYH7 | NM_000257.1 | 1963 | gggctgatcgcctattgagaag | 92383 | - | see also 12839 line |
| 29420 | MYH7 | NM_000257.1 | 1004 | cccctacgattatgcattcatct | 92378 | - | see also 12839 line |
| 29421 | MYH8 | NM_002472.1 | 1938 | gtctctttccaagtatgctagt | 92401 | - | see also 12840 line |
| 29422 | MYH8 | NM_002472.1 | 2084 | cacttcgtacggtgtatcattcc | 92402 | - | see also 12840 line |
| 29423 | MYH8 | NM_002472.1 | 4560 | atcaactgaaacgctaagaaga | 92415 | - | see also 12840 line |
| 29424 | MYH9 | NM_002473.2 | 465 | caccgcctacaggagtatgatgc | 92428 | - | see also 12841 line |
| 29425 | MYH9 | NM_002473.2 | 2213 | aggcgtgcgtcatcatgataaaa | 92440 | - | see also 12841 line |
| 29426 | MYLK | NM_005965.2 | 3193 | gaggacgggaactgctctttaat | 92485 | - | see also 4168 line |
| 29427 | MYLK | NM_005965.2 | 3165 | gtccgccacttccagatagact | 92483 | - | see also 4168 line |
| 29428 | MYLK2 | NM_033118.2 | 669 | cacctcgagggggattgagttcc | 92502 | - | see also 12843 line |
| 29429 | MYLK2 | NM_033118.2 | 1512 | gacctttgaggccgtatcagacg | 92512 | - | see also 12843 line |
| 29430 | MYLK2 | NM_033118.2 | 1468 | accctaaacaacgttctatctgg | 92510 | - | see also 12843 line |
| 29431 | MYO10 | NM_012334.1 | 6234 | acccctggcgaatacgtataaga | 92628 | - | see also 5295 line |
| 29432 | MYO10 | NM_012334.1 | 6233 | cacccctggcgaatacgtataag | 92627 | - | see also 5295 line |
| 29433 | MYO10 | NM_012334.1 | 6236 | cctggcgaatacgtataagatc | 92629 | - | see also 5295 line |
| 29434 | MYO15A | NM_016239.2 | 1257 | tacggctacgagcgattacgaacc | 92638 | - | see also 6746 line |
| 29435 | MYO15A | NM_016239.2 | 4116 | gtgtcggtgaaccataccaaat | 92654 | - | see also 6746 line |
| 29436 | MYO18B | NM_032608.4 | 3206 | tccaactcacagcgatatcaaga | 92726 | - | see also 12846 line |
| 29437 | MYO18B | NM_032608.4 | 3205 | ctccacgctacagcgatatcaag | 92725 | - | see also 12846 line |
| 29438 | MYO1A | NM_005379.2 | 3318 | cagcaagcaagctacgctacaaaaaa | 92826 | - | see also 12847 line |
| 29439 | MYO1A | NM_005379.2 | 3316 | aacagcaagctacgctacacaaaa | 92825 | - | see also 12847 line |
| 29440 | MYO1C | NM_033375.1 | 1947 | gcctatcgccgcaaatacgaagc | 92844 | - | see also 12848 line |
| 29441 | MYO1C | NM_033375.1 | 166 | ggcgatttcgggagaatctcatc | 92829 | - | see also 12848 line |
| 29442 | MYO1E | NM_004998.1 | 1678 | tggacaccattgagtacttaa | 92886 | - | see also 3493 line |

Figure 46

| | | | | | | | |
|---|---|---|---|---|---|---|---|
| 29443 | MYO1F | NM_004998.1 | 642 | tgccctgcagataatatgtaca | 92868 | - | see also 3493 line |
| 29444 | MYO1F | NM_012335.2 | 798 | gacaccggactacttactacc | 92937 | - | see also 12850 line |
| 29445 | MYO1F | NM_012335.2 | 2589 | cacgcgacaggacgacttcttca | 92944 | - | see also 12850 line |
| 29446 | MYO1F | NM_012335.2 | 793 | ctcatgacaccggactactatta | 92936 | - | see also 12850 line |
| 29447 | MYO3A | NM_017433.3 | 699 | cccggcaccgtcggaacacatcc | 92963 | - | see also 7004 line |
| 29448 | MYO3B | NM_138995.1 | 2447 | tgctgtaccgtggaatatgagg | 93115 | - | see also 12852 line |
| 29449 | MYO3B | NM_138995.1 | 1880 | aagacaattcgaagcaattcagc | 93105 | - | see also 12852 line |
| 29450 | MYO5A | NM_000259.1 | 5644 | gtcgttcattcgtactatacaga | 93257 | - | see also 201 line |
| 29451 | MYO5A | NM_000259.1 | 5752 | ttccctgcactagaaaccatcc | 93259 | - | see also 201 line |
| 29452 | MYO5C | NM_018728.1 | 2651 | atccgacgattcgtgcttaatat | 93323 | - | see also 12854 line |
| 29453 | MYO5C | NM_018728.1 | 2652 | tccgacgattcgtgcttaatatt | 93324 | - | see also 12854 line |
| 29454 | MYO6 | NM_004999.1 | 214 | ccccgacagcttaacaattgaac | 93375 | - | see also 3494 line |
| 29455 | MYO6 | NM_004999.1 | 3948 | aggctcggcccacctatgcaaca | 93483 | - | see also 3494 line |
| 29456 | MYO7A | NM_000260.1 | 6688 | gcctcatgatcccaaaacgaag | 93523 | - | see also 205 line |
| 29457 | MYO7A | NM_000260.1 | 5068 | aggggctccggaagagatctaag | 93512 | - | see also 205 line |
| 29458 | MYO7A | NM_000260.1 | 5211 | ggctgggccaacggcatcaatga | 93517 | - | see also 205 line |
| 29459 | MYO9A | NM_006901.1 | 3889 | atcgaattagccgtgaaagttca | 93651 | - | see also 4834 line |
| 29460 | MYO9A | NM_006901.1 | 2848 | cactacaagatcgcattaccaag | 93618 | - | see also 4834 line |
| 29461 | MYO9B | NM_004145.2 | 4219 | ggctctcaggtcgacctaaatc | 93777 | - | see also 2793 line |
| 29462 | NADSYN1 | NM_018161.3 | 2054 | cgcgtaccacgccgagaactaca | 93811 | - | see also 12859 line |
| 29463 | NADSYN1 | NM_018161.3 | 364 | accgaaacgtccgctacaactgc | 93797 | - | see also 12859 line |
| 29464 | NADSYN1 | NM_018161.3 | 1978 | gacaggtcgctgacaaagtgaag | 93810 | - | see also 12859 line |
| 29465 | NAGK | NM_017567.1 | 56 | acgatccgaggtcctttagtct | 93819 | - | see also 7035 line |
| 29466 | NAGK | NM_017567.1 | 53 | caacacgatccgaggtcctttag | 93818 | - | see also 7035 line |
| 29467 | NALP1 | NM_014922.2 | 2686 | ttgtacgagactcggaacaaaac | 93859 | - | see also 12861 line |
| 29468 | NALP1 | NM_014922.2 | 4569 | gacatcggtggaggttgcttgg | 93883 | - | see also 12861 line |
| 29469 | NALP1 | NM_014922.2 | 2706 | aacgttcctgacacaagtgatcg | 93861 | - | see also 12861 line |
| 29470 | NALP11 | NM_145007.1 | 2376 | gagcgacgggatgaacatactgt | 93960 | - | see also 12862 line |
| 29471 | NALP11 | NM_145007.1 | 1419 | cactcataaagacccgttacaagt | 93919 | - | see also 12862 line |
| 29472 | NALP12 | NM_033297.1 | 2127 | ggcagcgcttgagtaacaaaac | 93997 | - | see also 12863 line |
| 29473 | NALP12 | NM_033297.1 | 2126 | tggcagcgcttcgagtaacaaaa | 93996 | - | see also 12863 line |
| 29474 | NALP2 | NM_017852.1 | 1844 | tgcgatgcgacataagttgtaag | 94031 | - | see also 12864 line |
| 29475 | NALP2 | NM_017852.1 | 1843 | ctgcgatgcgacataagttgtaa | 94030 | - | see also 12864 line |
| 29476 | NALP5 | NM_153447.2 | 1270 | ccccgttacctgttagttagagg | 94074 | - | see also 12865 line |
| 29477 | NALP5 | NM_153447.2 | 1358 | aagggttgcgtcgatcatgaac | 94075 | - | see also 12865 line |
| 29478 | NALP6 | NM_138329.1 | 528 | ctcggacacgcacactttcaacc | 94126 | - | see also 12866 line |
| 29479 | NALP6 | NM_138329.1 | 2404 | cagtacctggtgggtatgcttcg | 94129 | - | see also 12866 line |

Figure 46

| 29480 | NALP7 | NM_139176.1 | 2853 | tacggttgaagacctatgaaact | 94158 | - | see also 12867 line |
|---|---|---|---|---|---|---|---|
| 29481 | NALP7 | NM_139176.1 | 1812 | ccccgttcaaggaaatttctatt | 94143 | - | see also 12867 line |
| 29482 | NALP7 | NM_139176.1 | 2707 | aaccaagcttggctgtagatatc | 94154 | - | see also 12867 line |
| 29483 | NAV1 | NM_020443.2 | 2277 | gggcgcaagactagcttagatgt | 94175 | - | see also 7877 line |
| 29484 | NAV1 | NM_020443.2 | 4861 | gccgtcgaggtgtcaataacata | 94204 | - | see also 7877 line |
| 29485 | NAV2 | NM_145117.2 | 2951 | tccggaccgatgacattacaagc | 94246 | - | see also 12869 line |
| 29486 | NAV2 | NM_145117.2 | 2950 | atccggaccgatgacattacaag | 94245 | - | see also 12869 line |
| 29487 | NAV3 | NM_014903.2 | 4877 | gccgattgcaaagtctaactatg | 94409 | - | see also 12870 line |
| 29488 | NAV3 | NM_014903.2 | 6753 | taccattggtccccgactattcc | 94465 | - | see also 12870 line |
| 29489 | NDUFA10 | NM_004544.2 | 148 | agcgcgtgagaggaattcatagc | 94475 | - | see also 12871 line |
| 29490 | NDUFA10 | NM_004544.2 | 432 | tacgatgatccgagaagcaatga | 94482 | - | see also 12871 line |
| 29491 | NEK11 | NM_024800.2 | 1115 | aagaccatctgctatcgaaattt | 94518 | - | see also 12872 line |
| 29492 | NEK11 | NM_024800.2 | 631 | gccgagatctggacgataaaatt | 94502 | - | see also 12872 line |
| 29493 | NEK2 | NM_002497.1 | 633 | aaccatgacacgagttttgcaaa | 94553 | - | see also 12873 line |
| 29494 | NEK2 | NM_002497.1 | 847 | accgttactctgatgaattgaat | 94564 | - | see also 12873 line |
| 29495 | NEK2 | NM_002497.1 | 609 | gactttgggctagctagaatatt | 94551 | - | see also 12873 line |
| 29496 | NEK3 | NM_002498.1 | 1257 | tagaaagggtagccatactgatt | 94598 | - | see also 12874 line |
| 29497 | NEK3 | NM_002498.1 | 897 | ctgtacccttaagcatccatttc | 94590 | - | see also 12874 line |
| 29498 | NEK4 | NM_003157.1 | 1256 | gccacaatcagtagcgtaaatat | 94633 | - | see also 12875 line |
| 29499 | NEK4 | NM_003157.1 | 2661 | aagctcgccagttgaaatttttt | 94669 | - | see also 12875 line |
| 29500 | NEK4 | NM_003157.1 | 1362 | atgagttaggaggtatatgcagt | 94639 | - | see also 12875 line |
| 29501 | NEK6 | NM_014397.3 | 270 | tggcggacttccagatcgaaaag | 94671 | - | see also 12876 line |
| 29502 | NEK6 | NM_014397.3 | 271 | ggcggacttccagatcgaaaaga | 94672 | - | see also 12876 line |
| 29503 | NEK7 | NM_133494.1 | 843 | aactccgacagttagttaatatg | 94714 | - | see also 9589 line |
| 29504 | NEK7 | NM_133494.1 | 114 | gaccggatatgggctataataca | 94688 | - | see also 9589 line |
| 29505 | NEK8 | NM_178170.2 | 1372 | ctgtccactgagcgagaactatt | 94726 | - | see also 12878 line |
| 29506 | NEK8 | NM_178170.2 | 487 | gtggtgggtaccccatgctatat | 94722 | - | see also 12878 line |
| 29507 | NEK9 | NM_033116.3 | 1757 | ttccctacacaacgtcctttacc | 94786 | - | see also 9363 line |
| 29508 | NEK9 | NM_033116.3 | 756 | gaggacgtttgatgctacaaacc | 94756 | - | see also 9363 line |
| 29509 | NLK | NM_016231.1 | 627 | gagccggatagacctattggata | 94804 | - | see also 6733 line |
| 29510 | NLK | NM_016231.1 | 1210 | aactactaggacgaagaatattg | 94822 | - | see also 6733 line |
| 29511 | NME1 | NM_000269.2 | 185 | agcgttttgagcagaaaggattc | 94844 | - | see also 12881 line |
| 29512 | NME1 | NM_000269.2 | 543 | tgctcagaactggatctatgaat | 94849 | - | see also 12881 line |
| 29513 | NME4 | NM_005009.2 | 422 | gaggctgccccaggaaccataag | 94854 | - | see also 12883 line |
| 29514 | NME5 | NM_003551.2 | 319 | gccatgatattagctagacataa | 94860 | - | see also 2364 line |
| 29515 | NME5 | NM_003551.2 | 479 | ctgcggaaagagaaatacgtttt | 94871 | - | see also 2364 line |

Figure 46

| ID | Gene | Accession | Pos | Sequence | ID2 | | Annotation | | |
|---|---|---|---|---|---|---|---|---|---|
| 29516 | NME6 | NM_005793.3 | 261 | aaggggctttttctatcagagg | 94883 | - | kinase_related, apoptosis; nucleoside-diphosphate kinase | - | |
| 29517 | NME6 | NM_005793.3 | 393 | gccatgtgccccagattctatc | 94884 | - | see also 29516 line | | |
| 29518 | NME6 | NM_005793.3 | 182 | agcaacaagttcctgattgtacg | 94881 | - | see also 29516 line | | |
| 29519 | NME7 | NM_013330.3 | 445 | ttctcgacaactggtattaattg | 94891 | - | see also 12885 line | | |
| 29520 | NME7 | NM_013330.3 | 925 | gccggcaaacactgctaaattta | 94907 | - | see also 12885 line | | |
| 29521 | NME7 | NM_013330.3 | 300 | ttcgacgttatgagctttattt | 94887 | - | see also 12885 line | | |
| 29522 | NOLC1 | NM_004741.1 | 2056 | aagtcctttcggcatgagaaaac | 94932 | - | see also 12886 line | | |
| 29523 | NOLC1 | NM_004741.1 | 660 | agctcgagcagcacctaaaatag | 94923 | - | see also 12886 line | | |
| 29524 | NPR2 | NM_000907.1 | 2790 | tgctcgtatggcccctagcattac | 94979 | - | see also 588 line | | |
| 29525 | NRBP | NM_013392.1 | 320 | gaggtgaatcaacggaatgtacc | 94986 | - | see also 5425 line | | |
| 29526 | NRBP | NM_013392.1 | 123 | gggagtccagacagtacttagc | 94984 | - | see also 5425 line | | |
| 29527 | NSF | NM_006178.1 | 1596 | cccagttactcgagttctagatg | 95050 | - | see also 4271 line | | |
| 29528 | NSF | NM_006178.1 | 1992 | gaccactagccgcaaagatgtcc | 95059 | - | see also 4271 line | | |
| 29529 | NSF | NM_006178.1 | 955 | gaggctaacattgcaaacttt | 95028 | - | see also 4271 line | | |
| 29530 | NTRK1 | NM_002529.2 | 2006 | ttggcatgagcagggatatctac | 95072 | - | see also 12890 line | | |
| 29531 | NTRK1 | NM_002529.2 | 1978 | caggactgtggtcaagattgg | 95071 | - | see also 12890 line | | |
| 29532 | NTRK2 | NM_006180.2 | 1274 | ctcttgtgtggcggaaaatcttg | 95097 | - | see also 4274 line | | |
| 29533 | NTRK2 | NM_006180.2 | 1277 | ttgtgtgcggaaaatcttgtag | 95098 | - | see also 4274 line | | |
| 29534 | NTRK3 | NM_002530.1 | 454 | agtctacaccggacttcaaaag | 95135 | - | see also 12892 line | | |
| 29535 | NTRK3 | NM_002530.1 | 531 | aaccccatttgcgttatataaa | 95136 | - | see also 12892 line | | |
| 29536 | NUBP1 | NM_002484.1 | 807 | ccctggatccgctcataggtaag | 95166 | - | see also 1590 line | | |
| 29537 | NUBP1 | NM_002484.1 | 267 | ctcttctagacatcggatatgt | 95161 | - | see also 1590 line | | |
| 29538 | NUBP2 | NM_012225.1 | 575 | ggcgcgggagctgaccttcgtagg | 95175 | - | molecular_function unknown | - | |
| 29539 | NUBP2 | NM_012225.1 | 373 | gtgtggagaggcgcccaagaaaaa | 95174 | - | see also 29538 line | | |
| 29540 | NVL | NM_002533.1 | 2060 | aagtgtccgagtggtgaatcagc | 95210 | - | see also 1631 line | | |
| 29541 | NVL | NM_002533.1 | 2224 | cagatgccttgccatcttaaaa | 95215 | - | see also 1631 line | | |
| 29542 | OSR1 | NM_005109.1 | 1759 | ctggtcgacgaagggatttagt | 95265 | - | see also 12895 line | | |
| 29543 | OSR1 | NM_005109.1 | 1637 | tcccaatcagtctagtactaaga | 95259 | - | see also 12895 line | | |
| 29544 | P2RX1 | NM_002558.2 | 1243 | cggcactgcagaccatcctatga | 95279 | - | see also 1648 line | | |
| 29545 | P2RX2 | NM_012226.3 | 665 | ctgcctgtcaggctacaacttc | 95293 | - | see also 12897 line | | |
| 29546 | P2RX2 | NM_012226.3 | 860 | gcgactggatcttgctaacattc | 95294 | - | see also 12897 line | | |
| 29547 | P2RX3 | NM_002559.2 | 985 | ccgcttcgacgtgctggtatacg | 95313 | - | see also 12898 line | | |
| 29548 | P2RX3 | NM_002559.2 | 475 | cactggccgctgccgtgaactaca | 95305 | - | see also 12898 line | | |
| 29549 | P2RX4 | NM_002560.2 | 666 | atgcgaccactgtgtgtaaatca | 95321 | - | see also 12899 line | | |
| 29550 | P2RX4 | NM_002560.2 | 1254 | tccgcttcgacatcattgtgttt | 95331 | - | see also 12899 line | | |

Figure 46

| 29551 | P2RX5 | NM_002561.2 | 327 | gactacaagaccgagaagtatgt | 95337 | - | see also 12900 line |
|---|---|---|---|---|---|---|---|
| 29552 | P2RX5 | NM_002561.2 | 346 | atgtcatcgccaagaacaagaag | 95338 | - | see also 12900 line |
| 29553 | P2RX7 | NM_002562.4 | 1280 | agccgacattaaagtatgtgtcc | 95390 | - | see also 12901 line |
| 29554 | P2RX7 | NM_002562.4 | 1220 | agccctgtgtggtcaacgaatac | 95384 | - | see also 12901 line |
| 29555 | P2RXL1 | NM_005446.2 | 698 | gccgctatgaaccacaattcagc | 95403 | - | see also 12902 line |
| 29556 | P2RXL1 | NM_005446.2 | 1055 | ggctgggcgtggtcacctttttc | 95405 | - | see also 12902 line |
| 29557 | PACE-1 | NM_020423.4 | 1304 | tgctgggcctgcgtgatactagt | 95426 | - | see also 7870 line |
| 29558 | PACE-1 | NM_020423.4 | 1845 | atcaagcttaccccaaaagatta | 95442 | - | see also 7870 line |
| 29559 | PAK1 | NM_002576.2 | 547 | gaccgattttaccgatccatttt | 95450 | - | see also 1657 line |
| 29560 | PAK1 | NM_002576.2 | 1240 | accgtgtacacagcaatggatgt | 95466 | - | see also 1657 line |
| 29561 | PAK1 | NM_002576.2 | 642 | cacaattcatgtcggttttgatg | 95455 | - | see also 1657 line |
| 29562 | PAK2 | NM_002577.2 | 689 | cgcgaccggatcatacgaaatca | 95478 | - | see also 12904 line |
| 29563 | PAK2 | NM_002577.2 | 693 | accggatcatacgaaatcaattt | 95480 | - | see also 12904 line |
| 29564 | PAK3 | NM_002578.1 | 1300 | gtgactcgaaaagcttatggtcc | 95517 | - | see also 1658 line |
| 29565 | PAK3 | NM_002578.1 | 457 | gcccatccttcgagtacaaaaac | 95503 | - | see also 1658 line |
| 29566 | PAK6 | NM_020168.3 | 786 | ggccaccgacccagacatgtacc | 95527 | - | see also 12907 line |
| 29567 | PAK6 | NM_020168.3 | 2044 | ctctcggacttcggattctgtgc | 95530 | - | see also 12907 line |
| 29568 | PAK7 | NM_020341.2 | 817 | tccagcgaatccgatactactgc | 95538 | - | see also 7836 line |
| 29569 | PAK7 | NM_020341.2 | 923 | agcaaaatgggcacgtaatgaaa | 95541 | - | see also 7836 line |
| 29570 | PAK7 | NM_020341.2 | 950 | agcacggggaggcctactattct | 95543 | - | see also 7836 line |
| 29571 | PANK1 | NM_138316.2 | 248 | ttcgagccgaaggatattacagc | 95586 | - | see also 9596 line |
| 29572 | PANK1 | NM_138316.2 | 911 | tgcgcgttgaatgagaacataga | 95594 | - | see also 9596 line |
| 29573 | PANK2 | NM_024960.3 | 1029 | atgcggcttttggcatatgcttt | 95619 | - | see also 8826 line |
| 29574 | PANK3 | NM_024594.2 | 480 | caccggcattcgggatgtacacc | 95633 | - | see also 8657 line |
| 29575 | PANK3 | NM_024594.2 | 764 | atggacaagccgagtgctattat | 95641 | - | see also 8657 line |
| 29576 | PANK4 | NM_018216.1 | 1328 | atgacgctctggcccgaaaatac | 95675 | - | see also 7323 line |
| 29577 | PANK4 | NM_018216.1 | 846 | ggccaccgccgaccaagagttct | 95668 | - | see also 7323 line |
| 29578 | PANK4 | NM_018216.1 | 2189 | gccgtgctgtccacacaaactac | 95685 | - | see also 7323 line |
| 29579 | PAPSS1 | NM_005443.3 | 1745 | agcgtatggactactatgactct | 95721 | - | see also 12912 line |
| 29580 | PAPSS1 | NM_005443.3 | 477 | aggtgcaagtttaccgtttttg | 95694 | - | see also 12912 line |
| 29581 | PAPSS2 | NM_004670.2 | 484 | ttcgcaaaggatcgtgagaatgc | 95735 | - | see also 3192 line |
| 29582 | PAPSS2 | NM_004670.2 | 584 | gagacgtaaaaggcctctataaa | 95740 | - | see also 3192 line |
| 29583 | PAPSS2 | NM_004670.2 | 586 | gacgtaaaaggcctctataaaag | 95741 | - | see also 3192 line |
| 29584 | PASK | NM_015148.2 | 3067 | agggcgagtactcccaaaagtac | 95808 | - | see also 12914 line |
| 29585 | PASK | NM_015148.2 | 1274 | tgcgtacaacagctcattacagc | 95794 | - | see also 12914 line |
| 29586 | PC | NM_000920.2 | 2770 | ggcaactcggacgtgtatgaaaa | 95837 | - | see also 597 line |
| 29587 | PC | NM_000920.2 | 1946 | tgcgcacccacgatctcaaaaag | 95831 | - | see also 597 line |

Figure 46

| | | | | | | | |
|---|---|---|---|---|---|---|---|
| 29588 | PC | NM_000920.2 | 383 | tccgcacgtagccatctactct | 95821 | - | see also 597 line |
| 29589 | PCCA | NM_000282.1 | 1397 | ttcgaggtgttacacataatatt | 95887 | - | see also 246 line |
| 29590 | PCCA | NM_000282.1 | 733 | ggcgatagatagactactaataga | 95866 | - | see also 246 line |
| 29591 | PCOLCE2 | NM_013363.2 | 1347 | aagatgggcgaggcaaaatcatg | 95942 | - | see also 5409 line |
| 29592 | PCOLCE2 | NM_013363.2 | 1047 | tccctgtaaccacgggtttaaaa | 95932 | - | see also 5409 line |
| 29593 | PCTK1 | NM_006201.2 | 1625 | gccacgcacccgacttgatagc | 95954 | - | see also 4297 line |
| 29594 | PCTK1 | NM_006201.2 | 1063 | atcgttacgctacatgacattat | 95948 | - | see also 4297 line |
| 29595 | PCTK2 | NM_002595.2 | 1984 | aaggaaccgggtttcgaaatc | 96003 | - | see also 1676 line |
| 29596 | PCTK2 | NM_002595.2 | 1985 | aggaaccgggtttcgaaattct | 96004 | - | see also 1676 line |
| 29597 | PCTK3 | NM_002596.1 | 353 | cccgcactgagaccattgaaga | 96006 | - | see also 1679 line |
| 29598 | PCTK3 | NM_002596.1 | 1017 | tgcacaacgtcaagatttcatg | 96008 | - | see also 1679 line |
| 29599 | PDGFRA | NM_006206.2 | 1711 | cacgccgcttcctgatattgagt | 96029 | - | see also 12921 line |
| 29600 | PDGFRA | NM_006206.2 | 1050 | aagtcttaaaaccgtgtataag | 96019 | - | see also 12921 line |
| 29601 | PDGFRB | NM_002609.2 | 2911 | tgcgggactcgaattacatctcc | 96091 | - | see also 1687 line |
| 29602 | PDGFRB | NM_002609.2 | 691 | agcggaaacggctacatcttt | 96063 | - | see also 1687 line |
| 29603 | PDK1 | NM_002610.3 | 804 | atgctaggcgtctgtgtgattg | 96127 | - | see also 1688 line |
| 29604 | PDK1 | NM_002610.3 | 992 | taccccctattcaagttcatgt | 96133 | - | see also 1688 line |
| 29605 | PDK2 | NM_002611.3 | 1102 | ccgcctcacgccaagtacttc | 96145 | - | see also 1689 line |
| 29606 | PDK2 | NM_002611.3 | 1006 | agggactcttcagctacagtac | 96144 | - | see also 1689 line |
| 29607 | PDK3 | NM_005391.1 | 219 | ttctgccggataatttacttaac | 96157 | - | see also 3760 line |
| 29608 | PDK4 | NM_002612.2 | 421 | tctcccgacccaattagtaaat | 96197 | - | see also 12926 line |
| 29609 | PDK4 | NM_002612.2 | 1110 | gaccgctctttagtttatacata | 96223 | - | see also 12926 line |
| 29610 | PDPK1 | NM_002613.1 | 1631 | cacgcctaacaggacgtattatc | 96259 | - | see also 12927 line |
| 29611 | PDPK1 | NM_002613.1 | 1468 | tggataagcggaagggtttattt | 96253 | - | see also 12927 line |
| 29612 | PDZK3 | NM_015022.2 | 1454 | cgtcacctcacgctatcgtgt | 96273 | - | see also 12928 line |
| 29613 | PDZK3 | NM_015022.2 | 2792 | gacccgaggcgtgctcaatttc | 96295 | - | see also 12928 line |
| 29614 | PDZK3 | NM_015022.2 | 1597 | ctcgatcattggttgtacaaag | 96274 | - | see also 12928 line |
| 29615 | PEX1 | NM_000466.1 | 3006 | tactagtcgccctgacttgattg | 96462 | - | see also 374 line |
| 29616 | PEX1 | NM_000466.1 | 2442 | tcgactctctgtcagagtatat | 96443 | - | see also 374 line |
| 29617 | PEX6 | NM_000287.2 | 2690 | gcctggtaaacgtgctagattgc | 96504 | - | see also 12930 line |
| 29618 | PEX6 | NM_000287.2 | 744 | ctgggacctctctgatagactgg | 96487 | - | see also 12930 line |
| 29619 | PFAS | NM_012393.1 | 759 | ttgacttggcgcagtccaatagc | 96516 | - | see also 12931 line |
| 29620 | PFAS | NM_012393.1 | 787 | cagccgacactggttcttcaagg | 96517 | - | see also 12931 line |
| 29621 | PFKFB1 | NM_002625.1 | 1197 | agcgtctggagccagtgataatg | 96572 | - | see also 12932 line |
| 29622 | PFKFB1 | NM_002625.1 | 256 | cacaaagctcacacgatatctca | 96555 | - | see also 12932 line |
| 29623 | PFKFB2 | NM_006212.1 | 194 | ctcccgactctgatcgttatga | 96577 | - | see also 12933 line |
| 29624 | PFKFB2 | NM_006212.1 | 418 | aagatgttaaggcgtatctcact | 96587 | - | see also 12933 line |

Figure 46

| 29625 | PFKFB3 | NM_004566.1 | 1477 | ccgctcatgagacgcaatagtgt | 96622 | - | see also 12934 line | | |
|---|---|---|---|---|---|---|---|---|---|
| 29626 | PFKFB3 | NM_004566.1 | 735 | cagggacttgtcgctgatcaagg | 96617 | - | see also 12934 line | | |
| 29627 | PFKFB4 | NM_004567.2 | 424 | aacggagagcgaccatctttaat | 96635 | - | see also 3106 line | | |
| 29628 | PFKFB4 | NM_004567.2 | 617 | ctcctacgagtcgctagatgagg | 96642 | - | see also 3106 line | | |
| 29629 | PFTK1 | NM_012395.2 | 1325 | ctgccttgagccacgagtatttt | 96673 | - | see also 5309 line | | |
| 29630 | PFTK1 | NM_012395.2 | 933 | atccgtccctagccacacatact | 96665 | - | see also 5309 line | | |
| 29631 | PHKG1 | NM_006213.2 | 547 | ctgcaccttgcacaaactcaaca | 96680 | - | see also 12937 line | | |
| 29632 | PHKG1 | NM_006213.2 | 414 | atgagaccaacactttcttcttc | 96679 | - | see also 12937 line | | |
| 29633 | PHKG2 | NM_000294.1 | 809 | tcctgatgttacgcatgatcatg | 96689 | - | see also 12938 line | | |
| 29634 | PHKG2 | NM_000294.1 | 958 | cccttctttgagcgttgtgaagg | 96692 | - | see also 12938 line | | |
| 29635 | PIK3R4 | NM_014602.1 | 4256 | tcctcatagcgtccatggtatct | 96813 | - | see also 12939 line | | |
| 29636 | PIK3R4 | NM_014602.1 | 1884 | gtcaaagaggttcctcgtaatga | 96743 | - | see also 12939 line | | |
| 29637 | PIM1 | NM_002648.2 | 1197 | gccaaccttcgaagaaatccaga | 96830 | - | see also 12940 line | | |
| 29638 | PIM1 | NM_002648.2 | 964 | ttcgatgggacccgagtgtatag | 96827 | - | see also 12940 line | | |
| 29639 | PIM2 | NM_006875.1 | 548 | cgcccaggatctctttgactata | 96831 | - | kinase_related | protein kinase CK2 | - |
| 29640 | PIM2 | NM_006875.1 | 1027 | tgctggacccctggatgcaaaca | 96833 | - | see also 29639 line | | |
| 29641 | PIM2 | NM_006875.1 | 549 | gcccaggatctctttgactatat | 96832 | - | see also 29639 line | | |
| 29642 | PINK1 | NM_032409.1 | 1167 | tcgcgcacagagacctgaaatcc | 96840 | - | see also 9184 line | | |
| 29643 | PINK1 | NM_032409.1 | 1030 | gacgctgttcctcgttatgaaga | 96839 | - | see also 9184 line | | |
| 29644 | PKE | NM_173575.2 | 1382 | ctcccagtccgagaatgactatc | 96857 | - | see also 12942 line | | |
| 29645 | PKE | NM_173575.2 | 827 | tgcacacctgaccgacttcaaca | 96853 | - | see also 12942 line | | |
| 29646 | PKMYT1 | NM_004203.3 | 800 | ctcctacggagaggtcttcaagg | 96863 | - | see also 12943 line | | |
| 29647 | PKMYT1 | NM_004203.3 | 1439 | gcgttctgtccttgtcatgatgc | 96866 | - | see also 12943 line | | |
| 29648 | PLK | NM_005030.3 | 555 | caccgaaaccgagttattcatcg | 96871 | - | see also 12944 line | | |
| 29649 | PLK | NM_005030.3 | 758 | gtccattgggtgtatcatgtata | 96873 | - | see also 12944 line | | |
| 29650 | PP13 | NM_013268.2 | 309 | agctgtgcatctacgtacattac | 96886 | - | see also 12945 line | | |
| 29651 | PP13 | NM_013268.2 | 320 | tacgtacattacaatgagtatga | 96889 | - | see also 12945 line | | |
| 29652 | PP13 | NM_013268.2 | 46 | ttctttacccgtgccatacaaac | 96883 | - | see also 12945 line | | |
| 29653 | PRKAA2 | NM_006252.1 | 711 | atgagcatgtacctacgttattt | 96907 | - | see also 12946 line | | |
| 29654 | PRKAA2 | NM_006252.1 | 820 | cccactgaaacgagcaactatca | 96917 | - | see also 12946 line | | |
| 29655 | PRKACA | NM_002730.2 | 531 | aggacaactcaaacttatacatg | 96951 | - | see also 12947 line | | |
| 29656 | PRKACA | NM_002730.2 | 869 | ctgggggttcttatctatgaaat | 96954 | - | see also 12947 line | | |
| 29657 | PRKACB | NM_002731.1 | 944 | cacgacagattggattgctattt | 96985 | - | see also 12948 line | | |
| 29658 | PRKACB | NM_002731.1 | 361 | ttcgactggagtatgcttttaag | 96963 | - | see also 12948 line | | |
| 29659 | PRKACG | NM_002732.2 | 111 | caccgccagctcggatcagttcg | 97001 | - | see also 12949 line | | |
| 29660 | PRKACG | NM_002732.2 | 398 | tacagcgcgtcggaaggtttagc | 97003 | - | see also 12949 line | | |
| 29661 | PRKACG | NM_002732.2 | 901 | accagctggatcgccatctatga | 97007 | - | see also 12949 line | | |

Figure 46

| | | | | | | | |
|---|---|---|---|---|---|---|---|
| 29662 | PRKCA | NM_002737.1 | 740 | gacgactgtctgtagaaatctgg | 97020 | - | see also 1774 line |
| 29663 | PRKCA | NM_002737.1 | 445 | cacaagcaatgcgtcatcaatgt | 97012 | - | see also 1774 line |
| 29664 | PRKCB1 | NM_002738.4 | 557 | aagcgctgcgtgatgaatgttcc | 97046 | - | see also 1775 line |
| 29665 | PRKCB1 | NM_002738.4 | 1897 | acctgaaggcgaacgtgatatca | 97062 | - | see also 1775 line |
| 29666 | PRKCD | NM_006254.2 | 1711 | ctctaccgtgccacgtttatgc | 97087 | - | see also 12952 line |
| 29667 | PRKCD | NM_006254.2 | 1008 | ggccaacagccggacactatat | 97077 | - | see also 12952 line |
| 29668 | PRKCE | NM_005400.1 | 1403 | cacggaacaacccgtaccttacc | 97113 | - | see also 12953 line |
| 29669 | PRKCE | NM_005400.1 | 903 | cagaggaatcgccaaagtactgg | 97105 | - | see also 12953 line |
| 29670 | PRKCG | NM_002739.2 | 723 | ggcccgtaacctaattcctatgg | 97135 | - | see also 12954 line |
| 29671 | PRKCG | NM_002739.2 | 1464 | cccggaccgcctgatttcgtga | 97144 | - | see also 12954 line |
| 29672 | PRKCH | NM_006255.2 | 1423 | ttcttccaacgacttggtatcg | 97176 | - | see also 4337 line |
| 29673 | PRKCH | NM_006255.2 | 1315 | gggtctccaaccggaaaatattt | 97173 | - | see also 4337 line |
| 29674 | PRKCI | NM_002740.3 | 457 | gagacccgtgtacagtatcatct | 97211 | - | see also 1776 line |
| 29675 | PRKCI | NM_002740.3 | 1058 | aacagatcgtatttatgcaatga | 97231 | - | see also 1776 line |
| 29676 | PRKCL1 | NM_002741.1 | 1708 | gcccgaccacgggtgacatatc | 97254 | - | see also 12957 line |
| 29677 | PRKCL1 | NM_002741.1 | 1592 | agcgtgctaggcagatgaacatc | 97252 | - | see also 12957 line |
| 29678 | PRKCL1 | NM_002741.1 | 2331 | caccgagggctacgtcaagatcg | 97260 | - | see also 12957 line |
| 29679 | PRKCL2 | NM_006256.1 | 389 | atgaccctgtgttcttcctagc | 97278 | - | see also 4341 line |
| 29680 | PRKCL2 | NM_006256.1 | 1138 | agcggaagtagtcgaaatcttct | 97314 | - | see also 4341 line |
| 29681 | PRKCM | NM_002742.1 | 1548 | tgcggaaacggcactattggaga | 97382 | - | see also 1780 line |
| 29682 | PRKCM | NM_002742.1 | 2712 | agcgctacagtgtggataagacc | 97416 | - | see also 1780 line |
| 29683 | PRKCM | NM_002742.1 | 1173 | caccgaaagtaccaaacaactgc | 97374 | - | see also 1780 line |
| 29684 | PRKCN | NM_005813.2 | 1749 | tccgctaatgaggttgtacaat | 97453 | - | see also 4056 line |
| 29685 | PRKCN | NM_005813.2 | 755 | gcctcacacgggagagtgttacc | 97424 | - | see also 4056 line |
| 29686 | PRKCQ | NM_006257.1 | 1335 | tccgtttctgacgcacatgttt | 97522 | - | see also 4343 line |
| 29687 | PRKCQ | NM_006257.1 | 353 | ctcaaggccgaatgctaatgaat | 97504 | - | see also 4343 line |
| 29688 | PRKCQ | NM_006257.1 | 1400 | acctcaacggagggacttaatg | 97527 | - | see also 4343 line |
| 29689 | PRKD2 | NM_016457.2 | 1166 | tggggtacatccccctaatgaeg | 97548 | - | see also 12961 line |
| 29690 | PRKD2 | NM_016457.2 | 1295 | ggcgcctggactgcaagtgtatc | 97551 | - | see also 12961 line |
| 29691 | PRKG1 | NM_006258.1 | 641 | accgtcaagactcttgtaaatgt | 97577 | - | see also 4344 line |
| 29692 | PRKG1 | NM_006258.1 | 672 | gggcattgatcgacacaatgtttt | 97579 | - | see also 4344 line |
| 29693 | PRKG2 | NM_006259.1 | 1262 | tgcgaagcggtccatgtctaact | 97642 | - | see also 4348 line |
| 29694 | PRKG2 | NM_006259.1 | 779 | ggcactagatcgagaggtattcc | 97628 | - | see also 4348 line |
| 29695 | PRKWNK1 | NM_018979.1 | 631 | gccgtgggaatgtctaacgatgg | 97669 | - | see also 7630 line |
| 29696 | PRKWNK1 | NM_018979.1 | 3759 | tgccgggaaggcggtttatagtga | 97738 | - | see also 7630 line |

EP 1 752 536 A1

Figure 46

| 29697 | PRKWNK2 | NM_006648.3 | 3662 | acggggacgcacccgatgaaatt | 97825 | - | see also 12965 line | | |
|---|---|---|---|---|---|---|---|---|---|
| 29698 | PRKWNK2 | NM_006648.3 | 3663 | cggggacgcacccgatgaaattg | 97826 | - | see also 12965 line | | |
| 29699 | PRKWNK3 | NM_020922.1 | 4001 | ggggtcggttccaggtgattaca | 97951 | - | see also 12966 line | | |
| 29700 | PRKWNK3 | NM_020922.1 | 1454 | gagaccgggtgacgccaataaag | 97879 | - | see also 12966 line | | |
| 29701 | PRKWNK4 | NM_032387.2 | 1881 | ctcggcttttgccctatccattc | 98003 | - | see also 9179 line | | |
| 29702 | PRKX | NM_005044.1 | 1215 | aacaaggcgattaggaaacatga | 98022 | - | kinase_related | protein tyrosine kinase | - |
| 29703 | PRKX | NM_005044.1 | 1220 | ggcgattaggaaacatgaagaac | 98023 | - | see also 29702 line | | |
| 29704 | PRO2000 | NM_014109.2 | 3101 | agcgattccgagtgtttactaag | 98107 | - | see also 5494 line | | |
| 29705 | PRO2000 | NM_014109.2 | 1226 | gtcgtaatagggctatcaatagg | 98055 | - | see also 5494 line | | |
| 29706 | PRPF4B | NM_003913.3 | 916 | aagtcgcgatcgaggtaaaaaat | 98173 | - | see also 2669 line | | |
| 29707 | PRPF4B | NM_003913.3 | 918 | gtcgcgatcgaggtaaaaaatcc | 98174 | - | see also 2669 line | | |
| 29708 | PRSS15 | NM_004793.2 | 523 | gtcggcgtctttctaaagagaga | 98245 | - | see also 12970 line | | |
| 29709 | PRSS15 | NM_004793.2 | 504 | tcgtctcgcccagccttatgtcg | 98242 | - | see also 12970 line | | |
| 29710 | PSK | NM_016151.1 | 2657 | gcggcagcgccagtactttgagc | 333015 | - | see also 12971 line | | |
| 29711 | PSK | NM_016151.1 | 1055 | cacggcttggctggtaatggagt | 333001 | - | see also 12971 line | | |
| 29712 | PSKH1 | NM_006742.1 | 403 | cccacgtgttacagctaagtatg | 98269 | - | see also 4764 line | | |
| 29713 | PSKH1 | NM_006742.1 | 1319 | tccacacgctccaataagtcacg | 98282 | - | see also 4764 line | | |
| 29714 | PSKH2 | NM_033126.1 | 430 | gagctctttgatcgactcattgc | 98290 | - | see also 12973 line | | |
| 29715 | PSKH2 | NM_033126.1 | 340 | cgggttagccatcgttacattgt | 98286 | - | see also 12973 line | | |
| 29716 | PSMC1 | NM_002802.2 | 879 | tgcaccgtccatcgtgtttattg | 98309 | - | see also 12974 line | | |
| 29717 | PSMC1 | NM_002802.2 | 882 | accgtccatcgtgtttattgatg | 98310 | - | see also 12974 line | | |
| 29718 | PSMC1 | NM_002802.2 | 1036 | gccacaaaccgaatagaaacttt | 98313 | - | see also 12974 line | | |
| 29719 | PSMC2 | NM_002803.2 | 772 | tgcgtgcttcattcgagttattg | 98335 | - | see also 1819 line | | |
| 29720 | PSMC2 | NM_002803.2 | 1263 | gagcacggcgaaaaattgctacc | 98351 | - | see also 1819 line | | |
| 29721 | PSMC2 | NM_002803.2 | 1347 | ctcctcgttacatgacatacaac | 98358 | - | see also 1819 line | | |
| 29722 | PSMC3 | NM_002804.3 | 1118 | tccagcccaacacccaagttaag | 98369 | - | see also 1820 line | | |
| 29723 | PSMC4 | NM_006503.2 | 1008 | caggacggctggaccgtaaaatt | 98389 | - | see also 12977 line | | |
| 29724 | PSMC4 | NM_006503.2 | 632 | ccccgaggcgtcctcatgtatgg | 98382 | - | see also 12977 line | | |
| 29725 | PSMC4 | NM_006503.2 | 1009 | aggacggctggaccgtaaaattg | 98390 | - | see also 12977 line | | |
| 29726 | PSMC5 | NM_002805.4 | 93 | agcggactccgccaatattatct | 98401 | - | see also 1821 line | | |
| 29727 | PSMC5 | NM_002805.4 | 92 | cagcggactccgccaatattatc | 98400 | - | see also 1821 line | | |
| 29728 | PSMC6 | NM_002806.2 | 266 | ttgtcgtcgacagcttgacaaaa | 98420 | - | see also 1822 line | | |

Figure 46

| 29729 | PSMC6 | NM_002806.2 | 268 | gtcgtcgacagcttgacaaaagt | 98421 | - | see also 1822 line |
|---|---|---|---|---|---|---|---|
| 29730 | PTK2 | NM_005607.3 | 2966 | acctggaccggtcgaatgataag | 98524 | - | see also 3893 line |
| 29731 | PTK2 | NM_005607.3 | 2108 | atgtaatcggtcgaattgaaaat | 98508 | - | see also 3893 line |
| 29732 | PTK2 | NM_005607.3 | 2385 | tccgaggtccagcgaaggatttt | 98513 | - | see also 3893 line |
| 29733 | PTK2B | NM_004103.3 | 2795 | agcccagccgacctaagtacaga | 98553 | - | see also 2772 line |
| 29734 | PTK2B | NM_004103.3 | 1379 | aggagtgcgtcatgaagttcttc | 98535 | - | see also 2772 line |
| 29735 | PTK6 | NM_005975.2 | 1122 | gaggccattactccaccaaatcc | 98569 | - | see also 12981 line |
| 29736 | PTK6 | NM_005975.2 | 406 | ggctgtgcggcactacaagatct | 98561 | - | see also 12981 line |
| 29737 | PTK7 | NM_002821.3 | 636 | cacacttcgttgccacattgatg | 98572 | - | see also 12982 line |
| 29738 | PTK7 | NM_002821.3 | 2051 | accccaagccgctgattcagtgg | 98585 | - | see also 12982 line |
| 29739 | PTK7 | NM_002821.3 | 1849 | aacgctgggaccctgcattttgc | 98584 | - | see also 12982 line |
| 29740 | PTOV1 | NM_017432.2 | 1148 | cggctgcgtgcacttttcctaca | 98589 | - | see also 12983 line |
| 29741 | PTOV1 | NM_017432.2 | 848 | cccagtccagatcgtcaacaaca | 98588 | - | see also 12983 line |
| 29742 | PXK | NM_017771.2 | 759 | ctcagcgttgctaattaggatgt | 98616 | - | see also 7151 line |
| 29743 | PXK | NM_017771.2 | 612 | gccaaaggaacggctagtgttaa | 98610 | - | see also 7151 line |
| 29744 | PXK | NM_017771.2 | 192 | gtcccacacggaatatattattc | 98595 | - | see also 7151 line |
| 29745 | QARS | NM_005051.1 | 1254 | gcctatcgagtcaagtatacacc | 98663 | - | see also 12985 line |
| 29746 | QARS | NM_005051.1 | 266 | aagcgctgcccttgagtatgtgc | 98641 | - | see also 12985 line |
| 29747 | RAD50 | NM_005732.2 | 4264 | atcgatcagtgctcagagattgt | 98825 | - | see also 3973 line |
| 29748 | RAD50 | NM_005732.2 | 1051 | gagattcgtgatcagattacaag | 98714 | - | see also 3973 line |
| 29749 | RAF1 | NM_002880.1 | 897 | gtcgacatccacacctaatgtcc | 98850 | - | see also 1921 line |
| 29750 | RAF1 | NM_002880.1 | 1725 | ctcctatggcatcgtattgtatg | 98860 | - | see also 1921 line |
| 29751 | RAGE | NM_014226.1 | 693 | agccgtacacggaatacatctcc | 98889 | - | see also 5541 line |
| 29752 | RAGE | NM_014226.1 | 400 | tccgcacccaaacattcttatgt | 98876 | - | see also 5541 line |
| 29753 | RARSL | NM_020320.2 | 480 | tgcgttctaccatcataggaaat | 98916 | - | see also 12989 line |
| 29754 | RARSL | NM_020320.2 | 1601 | aggcatatcgtcagttaccttct | 98954 | - | see also 12989 line |
| 29755 | RET | NM_000323.2 | 1238 | gggcgaccgtacatgactatagg | 98968 | - | see also 273 line |
| 29756 | RET | NM_000323.2 | 2872 | ttcggcttgtcccgagatgttta | 98981 | - | see also 273 line |
| 29757 | RHOK | NM_002929.1 | 1341 | gcccgtgaagtaccctgataagt | 99008 | - | see also 12991 line |
| 29758 | RHOK | NM_002929.1 | 491 | aggggatagtggcgaagtttaag | 99005 | - | see also 12991 line |
| 29759 | RICH1 | NM_018054.3 | 1447 | cccgagttctaatcactcattcc | 99050 | - | see also 7218 line |
| 29760 | RICH1 | NM_018054.3 | 1441 | caccaccccgagttctaatcact | 99049 | - | see also 7218 line |
| 29761 | RIOK1 | NM_031480.2 | 1169 | accacggtggaggcgtgtatatc | 99085 | - | see also 12993 line |
| 29762 | RIOK1 | NM_031480.2 | 1444 | gtgtttaagcgagcatatattcc | 99096 | - | see also 12993 line |
| 29763 | RIPK1 | NM_003804.2 | 538 | gacggcaccgctaagaagaatgg | 99124 | - | see also 12994 line |
| 29764 | RIPK1 | NM_003804.2 | 1737 | cactagtctgacggataaacacc | 99156 | - | see also 12994 line |
| 29765 | RIPK2 | NM_003821.4 | 1028 | acctcaccgagcacgtatgatct | 99178 | - | see also 2603 line |

Figure 46

| 29766 | ROCK1 | NM_005406.1 | 1201 | agcaatcgtagatacttatcttc | 99228 | - | see also 3772 line |
|---|---|---|---|---|---|---|---|
| 29767 | ROCK1 | NM_005406.1 | 2079 | aaccaaagctcgtttaactgaca | 99252 | - | see also 3772 line |
| 29768 | ROCK2 | NM_004850.2 | 1685 | atcggatttacctactatagaga | 99310 | - | see also 3364 line |
| 29769 | ROCK2 | NM_004850.2 | 1682 | ttcatcggatttacctactatag | 99309 | - | see also 3364 line |
| 29770 | ROR1 | NM_005012.1 | 1070 | tcccgtactgcgatgaaacttca | 99413 | - | see also 3506 line |
| 29771 | ROR1 | NM_005012.1 | 1650 | ttgcgtctgtcggaataaccaga | 99430 | - | see also 3506 line |
| 29772 | ROR2 | NM_004560.2 | 473 | aacgtgcggtggctaaagaatga | 99468 | - | see also 12999 line |
| 29773 | ROR2 | NM_004560.2 | 1132 | ccgctaccatcagtgctataacg | 99474 | - | see also 12999 line |
| 29774 | ROS1 | NM_002944.2 | 2362 | cacgaaaggcgacgtttttgtgt | 99552 | - | see also 1986 line |
| 29775 | ROS1 | NM_002944.2 | 1142 | tgccttcggttggatgctatata | 99516 | - | see also 1986 line |
| 29776 | ROS1 | NM_002944.2 | 297 | gcctaaagtcgtgtgtaactaat | 99487 | - | see also 1986 line |
| 29777 | RP2 | NM_006915.1 | 522 | ttcgtgtgcgagattgtagaaag | 99713 | - | see also 4852 line |
| 29778 | RP2 | NM_006915.1 | 337 | gacggtagcaggacaacagtttc | 99704 | - | see also 4852 line |
| 29779 | RP2 | NM_006915.1 | 759 | aggactatgttcctatacctact | 99720 | - | see also 4852 line |
| 29780 | RPS6KA1 | NM_002953.2 | 891 | gtggtcctatggggtgttgatgt | 99740 | - | see also 13002 line |
| 29781 | RPS6KA1 | NM_002953.2 | 324 | ggctgatccatcccatttcgagc | 99735 | - | see also 13002 line |
| 29782 | RPS6KA2 | NM_021135.3 | 1320 | aacgctcatcacctgtttagagg | 99753 | - | see also 13003 line |
| 29783 | RPS6KA2 | NM_021135.3 | 875 | ctgcgggacgatcgagtacatgg | 99750 | - | see also 13003 line |
| 29784 | RPS6KA3 | NM_004586.1 | 881 | aagcgcagagtctttacgaatg | 99778 | - | see also 3120 line |
| 29785 | RPS6KA3 | NM_004586.1 | 1658 | atccggaatctattcgaatttgt | 99800 | - | see also 3120 line |
| 29786 | RPS6KA3 | NM_004586.1 | 882 | agcgcagagtctttacgaatgc | 99779 | - | see also 3120 line |
| 29787 | RPS6KA4 | NM_003942.1 | 520 | tgcacaagctcggcatcatttac | 99825 | - | see also 13005 line |
| 29788 | RPS6KA4 | NM_003942.1 | 519 | ctgcacaagctcggcatcattta | 99824 | - | see also 13005 line |
| 29789 | RPS6KA5 | NM_004755.2 | 303 | cacgagctgcggactgctaattt | 99830 | - | see also 3273 line |
| 29790 | RPS6KA5 | NM_004755.2 | 1359 | atcctattcaagcgtaatgcagc | 99863 | - | see also 3273 line |
| 29791 | RPS6KA6 | NM_014496.1 | 2089 | agcggtatactgctgaacaaata | 99940 | - | see also 5720 line |
| 29792 | RPS6KA6 | NM_014496.1 | 2079 | gacccacatcagcggtatactgc | 99938 | - | see also 5720 line |
| 29793 | RPS6KB1 | NM_003161.1 | 738 | tggactatgcaaagaatctattc | 99953 | - | see also 2134 line |
| 29794 | RPS6KB1 | NM_003161.1 | 899 | ccccattcactggggagaataga | 99956 | - | see also 2134 line |
| 29795 | RPS6KB2 | NM_003952.1 | 1111 | gaggacgtgagccagtttgatac | 99967 | - | see also 13009 line |
| 29796 | RPS6KB2 | NM_003952.1 | 402 | acgggctgagcggaacattctag | 99960 | - | see also 13009 line |
| 29797 | RPS6KC1 | NM_012424.2 | 1042 | tgcgggcagaaagtatctctagt | 99993 | - | see also 13010 line |
| 29798 | RPS6KC1 | NM_012424.2 | 217 | acccgaggggctacacagtatat | 99970 | - | see also 13010 line |
| 29799 | RPS6KL1 | NM_031464.2 | 344 | gacatgacaaaacgtgactatct | 100073 | - | see also 13011 line |
| 29800 | RPS6KL1 | NM_031464.2 | 1393 | aggtcacatccggctcacatatt | 100078 | - | see also 13011 line |
| 29801 | RUVBL1 | NM_003707.1 | 222 | gtggcgtcatagtagaattaatc | 100086 | - | see also 13012 line |
| 29802 | RUVBL1 | NM_003707.1 | 220 | atgtggcgtcatagtagaattaa | 100085 | - | see also 13012 line |

Figure 46

| 29803 | RUVBL2 | NM_006666.1 | 218 | gccggtcgggcagtccttattgc | 100117 | - | see also 13013 line |
|---|---|---|---|---|---|---|---|
| 29804 | RUVBL2 | NM_006666.1 | 217 | tgccggtcgggcagtccttattg | 100116 | - | see also 13013 line |
| 29805 | RUVBL2 | NM_006666.1 | 50 | atccgtgatgtaacaaggattga | 100114 | - | see also 13013 line |
| 29806 | RYK | NM_002958.1 | 951 | cagacacgcccaacaatgcaact | 100154 | - | see also 13014 line |
| 29807 | RYK | NM_002958.1 | 525 | gggaagttccacgcactttatca | 100138 | - | see also 13014 line |
| 29808 | SARS2 | NM_017827.2 | 1021 | ccctgcccgcttcaaagatctca | 100180 | - | see also 7157 line |
| 29809 | SCN8A | NM_014191.1 | 5789 | gagccgttcgtcgcatccaatcc | 100237 | - | see also 5532 line |
| 29810 | SCYL1 | NM_020680.2 | 327 | gaccccgttgggaatatacctca | 100244 | - | see also 13017 line |
| 29811 | SCYL1 | NM_020680.2 | 232 | ttcaagcgcttcaaaactctacg | 100242 | - | see also 13017 line |
| 29812 | SEPHS2 | NM_012248.2 | 1428 | gacaagccgcgagttattgaagt | 100282 | - | see also 5222 line |
| 29813 | SEPHS2 | NM_012248.2 | 660 | ctctacgccatgggattactga | 100257 | - | see also 5222 line |
| 29814 | SEPHS2 | NM_012248.2 | 708 | agcgtcagccagagtatgagtga | 100258 | - | see also 5222 line |
| 29815 | SGK | NM_005627.2 | 78 | tgctaaggcgaccctcacttact | 100283 | - | see also 3903 line |
| 29816 | SGK | NM_005627.2 | 154 | atgggtctgaacgacttattca | 100288 | - | see also 3903 line |
| 29817 | SGK2 | NM_016276.3 | 981 | ctgtggtaccccctgagttactgg | 100320 | - | see also 13020 line |
| 29818 | SGK2 | NM_016276.3 | 1250 | ttgagattaagaaccatgtattc | 100323 | - | see also 13020 line |
| 29819 | SGKL | NM_013257.3 | 1505 | aagattccaccaccatttaatcc | 100364 | - | see also 5376 line |
| 29820 | SGKL | NM_013257.3 | 247 | aagctgcccaagtgtaagcattc | 100328 | - | see also 5376 line |
| 29821 | SIK2 | NM_015191.1 | 988 | gccatccatcggggagtttaatg | 100399 | - | see also 13022 line |
| 29822 | SIK2 | NM_015191.1 | 991 | atccatcggggagtttaatgagc | 100400 | - | see also 13022 line |
| 29823 | SKD3 | NM_030813.3 | 274 | gaggacgcttcgataccaaatgc | 100424 | - | see also 8957 line |
| 29824 | SKD3 | NM_030813.3 | 272 | gggaggacgcttcgataccaaat | 100423 | - | see also 8957 line |
| 29825 | SMC1L1 | NM_006306.2 | 1469 | agccggtattgatgaaatcaat | 100466 | - | see also 4397 line |
| 29826 | SMC1L2 | NM_148674.1 | 2759 | aacgattagagaagcataactg | 100578 | - | see also 9820 line |
| 29827 | SMC1L2 | NM_148674.1 | 3586 | gacgcgctgatcggcatctatcc | 100610 | - | see also 9820 line |
| 29828 | SMC2L1 | NM_006444.1 | 1917 | aactgaaacgtcgatacactata | 100677 | - | see also 4505 line |
| 29829 | SMC2L1 | NM_006444.1 | 1723 | tcccaatcttcgatttgcataca | 100669 | - | see also 4505 line |
| 29830 | SMC5L1 | NM_015110.1 | 1425 | ttgtacgttttgacaatcttatg | 100773 | - | see also 6249 line |
| 29831 | SMC5L1 | NM_015110.1 | 1038 | tggaaaaacgagcgtcacaattg | 100758 | - | see also 6249 line |
| 29832 | SMC6L1 | NM_024624.2 | 1866 | cacggccaccgataatagtttct | 100878 | - | see also 8671 line |
| 29833 | SMC6L1 | NM_024624.2 | 1868 | cggccaccgataatagtttctga | 100879 | - | see also 8671 line |
| 29834 | SNARK | NM_030952.1 | 1234 | gcggcagcattcgctcaagaagt | 100928 | - | see also 13028 line |
| 29835 | SNARK | NM_030952.1 | 923 | gcctaccgggagccacctaaacc | 100927 | - | see also 13028 line |
| 29836 | SNF1LK | NM_173354.1 | 228 | cgcggcatcgagtcaccaaaacg | 100933 | - | see also 10064 line |
| 29837 | SNF1LK | NM_173354.1 | 279 | cacgattagattcaagcaattg | 100936 | - | see also 10064 line |
| 29838 | SNF1LK | NM_173354.1 | 398 | atcgtcactgaatttgctaaaaa | 100939 | - | see also 10064 line |
| 29839 | SNK | NM_006622.1 | 617 | aagtctacgccgcaaaaattatt | 100946 | - | see also 4683 line |

Figure 46

| 29840 | SNK | NM_006622.1 | 498 | gccggagatctcgcggattatcg | 100944 | - | see also 4683 line | | | |
|---|---|---|---|---|---|---|---|---|---|---|
| 29841 | SNRK | NM_017719.3 | 312 | aggccattttgccgtggttaaac | 100996 | - | see also 13031 line | | | |
| 29842 | SNRK | NM_017719.3 | 464 | tcgtccgcctttatgaagttatt | 101000 | - | see also 13031 line | | | |
| 29843 | SPATA5 | NM_145207.1 | 207 | gacggtgaccaacttattagaaa | 101044 | - | see also 9780 line | | | |
| 29844 | SPATA5 | NM_145207.1 | 2382 | tactaaccgtccagataggatag | 101114 | - | see also 9780 line | | | |
| 29845 | SPATA5 | NM_145207.1 | 101 | accaaagcgcggaaaatggttcg | 101041 | - | see also 9780 line | | | |
| 29846 | SPG4 | NM_014946.3 | 839 | gtcacaaacggacgtctataatg | 101139 | - | see also 6136 line | | | |
| 29847 | SPG4 | NM_014946.3 | 1592 | tagtagacgcctaaaaactgaat | 101167 | - | see also 6136 line | | | |
| 29848 | SPG4 | NM_014946.3 | 1089 | aagggtactccgaaaacaaatag | 101148 | - | see also 6136 line | | | |
| 29849 | SPG7 | NM_003119.2 | 982 | gtccgcgagtttgtggattatct | 101196 | - | see also 13034 line | | | |
| 29850 | SPG7 | NM_003119.2 | 1381 | tccacgaaccgagctgacatttt | 101199 | - | see also 13034 line | | | |
| 29851 | SPG7 | NM_003119.2 | 1383 | cacgaaccgagctgacattttgg | 101200 | - | see also 13034 line | | | |
| 29852 | SPHK1 | NM_021972.2 | 1346 | gggcaggcatatggagtatgaat | 101211 | - | see also 13035 line | | | |
| 29853 | SPHK1 | NM_021972.2 | 1268 | tggcgtcatgcatctgttctacg | 101210 | - | see also 13035 line | | | |
| 29854 | SPHK2 | NM_020126.3 | 1962 | gacgctggaggggggactttgtgc | 101222 | - | see also 13036 line | | | |
| 29855 | SPHK2 | NM_020126.3 | 951 | ggcctggcagtggtgtaagaacc | 101221 | - | see also 13036 line | | | |
| 29856 | SRC | NM_005417.3 | 1679 | ggctcggctcattgaagacaatg | 101227 | - | see also 13037 line | | | |
| 29857 | SRC | NM_005417.3 | 886 | tccaggctgaggagtggtatttt | 101225 | - | see also 13037 line | | | |
| 29858 | SRMS | NM_080823.2 | 805 | tggcgatcaaggtcatcaagtca | 101230 | - | see also 13038 line | | | |
| 29859 | SRMS | NM_080823.2 | 744 | aagctgggtgaaggctactttgg | 101229 | - | see also 13038 line | | | |
| 29860 | SRPK1 | NM_003137.2 | 1949 | tccggcacccttggcttaactcc | 101272 | - | see also 2117 line | | | |
| 29861 | SRPK2 | NM_182691.1 | 1835 | taccgctccatagagggttttaat | 101306 | - | see also 10272 line | | | |
| 29862 | SRPK2 | NM_182691.1 | 422 | ggccggtatcatgttattagaaa | 101273 | - | see also 10272 line | | | |
| 29863 | SRPK2 | NM_182691.1 | 1894 | cacggcgtgtatggcatttgagc | 101310 | - | see also 10272 line | | | |
| 29864 | SSTK | NM_032037.1 | 139 | ttctgagcgaactcggttataag | 101320 | - | see also 9064 line | | | |
| 29865 | STCH | NM_006948.3 | 1264 | tcctcggatccgtcaagtcattc | 101356 | - | see also 13042 line | | | |
| 29866 | STCH | NM_006948.3 | 709 | gggcggaggaactctagatgtgt | 101346 | - | see also 13042 line | | | |
| 29867 | STK10 | NM_005990.1 | 1629 | ctcaagcggacacgcaaatttgt | 101377 | - | see also 4184 line | | | |
| 29868 | STK10 | NM_005990.1 | 1287 | tcccgacccgtgtcaatggatgc | 101373 | - | see also 4184 line | | | |
| 29869 | STK11 | NM_000455.3 | 826 | caccggtggcaccctcaaaatct | 101391 | - | kinase_related | protein serine/threonine kinase | breast cancer、 Peutz-Jeghers syndrome、 pancreatic cancer、 melanoma、 colorectal cancers |
| 29870 | STK11 | NM_000455.3 | 577 | gaggaggttacggcacaaaaatg | 101389 | - | see also 29869 line | | | |
| 29871 | STK11 | NM_000455.3 | 578 | aggaggttacggcacaaaatgt | 101390 | - | see also 29869 line | | | |
| 29872 | STK16 | NM_003691.1 | 775 | gtccctaggctgcgtgctatatg | 101405 | - | see also 2525 line | | | |
| 29873 | STK16 | NM_003691.1 | 776 | tccctaggctgcgtgctatatgc | 101406 | - | see also 2525 line | | | |
| 29874 | STK17A | NM_004760.1 | 439 | gagattgctgtacttgaactagc | 101415 | - | see also 13045 line | | | |

Figure 46

| | | | | | | | |
|---|---|---|---|---|---|---|---|
| 29875 | STK17A | NM_004760.1 | 710 | ctccattgggtgacattaagatt | 101425 | - | see also 13045 line |
| 29876 | STK17B | NM_004226.2 | 661 | atgatgttatcagactcattaaa | 101457 | - | see also 2823 line |
| 29877 | STK17B | NM_004226.2 | 527 | gtcttgtcccgtgttattaatc | 101452 | - | see also 2823 line |
| 29878 | STK18 | NM_014264.2 | 2501 | tgctaatgaggtcatcgtattt | 101534 | - | see also 5571 line |
| 29879 | STK18 | NM_014264.2 | 613 | tcctactgactcgtaatatgaac | 101485 | - | see also 5571 line |
| 29880 | STK18 | NM_014264.2 | 609 | aacctcctactgactcgtaatat | 101483 | - | see also 5571 line |
| 29881 | STK19 | NM_004197.1 | 485 | cccggagacctttggagttaaga | 101558 | - | see also 2810 line |
| 29882 | STK19 | NM_004197.1 | 429 | ccettcccggtaccataaaatc | 101557 | - | see also 2810 line |
| 29883 | STK19 | NM_004197.1 | 428 | cccettcccggtaccataaaat | 101556 | - | see also 2810 line |
| 29884 | STK22C | NM_052841.2 | 268 | ccctcgggagctccaaatcgtcc | 101568 | - | see also 13049 line |
| 29885 | STK22C | NM_052841.2 | 649 | gggcattcccacgatgcaaaa | 101570 | - | see also 13049 line |
| 29886 | STK23 | NM_014370.1 | 1345 | gaggacacatcgctcacatagt | 101584 | - | see also 13050 line |
| 29887 | STK23 | NM_014370.1 | 684 | gtgtgtggggacgcttacatca | 101578 | - | see also 13050 line |
| 29888 | STK24 | NM_003576.2 | 494 | ggctccgcactagatctattaga | 101605 | - | see also 2373 line |
| 29889 | STK24 | NM_003576.2 | 496 | ctccgcactagatctattagaac | 101606 | - | see also 2373 line |
| 29890 | STK25 | NM_006374.3 | 1443 | gagcgagtgcagaggttttcaca | 101635 | - | see also 13052 line |
| 29891 | STK25 | NM_006374.3 | 1441 | tggagcgagtgcagaggtttca | 101634 | - | see also 13052 line |
| 29892 | STK29 | NM_003957.1 | 2325 | gacacactaactgtatggaaat | 101645 | - | see also 13053 line |
| 29893 | STK29 | NM_003957.1 | 2330 | cactaactgtatggaaatgatga | 101646 | - | see also 13053 line |
| 29894 | STK3 | NM_006281.1 | 668 | aacgcaatactgtaataggaact | 101662 | - | see also 4373 line |
| 29895 | STK3 | NM_006281.1 | 869 | cagaacttggtccgatgatttc | 101670 | - | see also 4373 line |
| 29896 | STK33 | NM_030906.1 | 1847 | ggcgtgtaatgtacatgttatt | 101724 | - | see also 13055 line |
| 29897 | STK33 | NM_030906.1 | 1845 | taggcgtgtaatgtacatgtta | 101722 | - | see also 13055 line |
| 29898 | STK36 | NM_015690.2 | 1964 | tgcggaggacagccttatgtgc | 101778 | - | see also 13056 line |
| 29899 | STK36 | NM_015690.2 | 2573 | gtgacgttgctactctcttacc | 101789 | - | see also 13056 line |
| 29900 | STK38 | NM_007271.2 | 380 | agcaaccttatcgctcaacatga | 101805 | - | see also 5071 line |
| 29901 | STK38L | NM_015000.1 | 868 | ttggtttatgtacggattaaag | 101842 | - | see also 6174 line |
| 29902 | STK38L | NM_015000.1 | 1448 | taccacagaaccggactacaaat | 101858 | - | see also 6174 line |
| 29903 | STK39 | NM_013233.1 | 1528 | gtgccgtgaacctcgttttgaga | 101897 | - | see also 13059 line |
| 29904 | STK39 | NM_013233.1 | 1658 | tggtcacgatgtagttatagtgg | 101907 | - | see also 13059 line |
| 29905 | STK4 | NM_006282.2 | 951 | gtcaatactgcgagacttaatta | 101942 | - | see also 4374 line |
| 29906 | STK4 | NM_006282.2 | 593 | ttggggtagcaggtcaacttaca | 101931 | - | see also 4374 line |
| 29907 | STK6 | NM_003600.2 | 556 | cagtcacaagccggttcagaatc | 101952 | - | see also 13061 line |
| 29908 | STK6 | NM_003600.2 | 915 | cccaccttcggcatcctaatatt | 101953 | - | see also 13061 line |
| 29909 | SYK | NM_003177.3 | 858 | ctggcagctagtcgagcattatt | 101966 | - | see also 13062 line |
| 29910 | SYK | NM_003177.3 | 862 | cagctagtcgagcattattctta | 101969 | - | see also 13062 line |
| 29911 | SYN3 | NM_003490.2 | 704 | tcccgcttgtggagcaaacattt | 102008 | - | see also 13063 line |

1094

Figure 46

| | | | | | | | |
|---|---|---|---|---|---|---|---|
| 29912 | SYN3 | NM_003490.2 | 286 | tccacgcccattgttcaaagacc | 102003 | - | see also 13063 line |
| 29913 | SYN3 | NM_003490.2 | 914 | tccgcatccagaaaattggatcc | 102012 | - | see also 13063 line |
| 29914 | TAP1 | NM_000593.4 | 759 | gccttcgttgtcagttatgcagc | 102021 | - | see also 13064 line |
| 29915 | TAP1 | NM_000593.4 | 2297 | ggcgttggccgagcattgatcc | 102041 | - | see also 13064 line |
| 29916 | TAP1 | NM_000593.4 | 755 | taccgccttcgttgtcagttatg | 102019 | - | see also 13064 line |
| 29917 | TAP2 | NM_000544.2 | 1111 | ctgcagaacgttcgcagttttgg | 102052 | - | see also 13065 line |
| 29918 | TAP2 | NM_000544.2 | 744 | caccatgtctcgaatcaacttgc | 102047 | - | see also 13065 line |
| 29919 | TARS | NM_152295.3 | 2379 | tccaacgggaacgcaccatttct | 102127 | - | see also 9832 line |
| 29920 | TARS | NM_152295.3 | 567 | aacaggttgatgcggaatcttgg | 102071 | - | see also 9832 line |
| 29921 | TARS | NM_152295.3 | 423 | gtcgagctgagttgaatccttgg | 102067 | - | see also 9832 line |
| 29922 | TBK1 | NM_013254.2 | 1127 | agggcgacgcttagtcttagaac | 102158 | - | see also 13067 line |
| 29923 | TBK1 | NM_013254.2 | 2230 | ttgcaacgttgactgtctttag | 102196 | - | see also 13067 line |
| 29924 | TCEB3L2 | NM_145653.1 | 1331 | gccgacagacaaagatgatctgt | 102200 | - | see also 13068 line |
| 29925 | TCEB3L2 | NM_145653.1 | 1281 | gcgagtagtgaccacgaaaatcc | 102198 | - | see also 13068 line |
| 29926 | TCP1 | NM_030752.1 | 1127 | ctcgtacgtctgcatcgattatc | 102212 | - | see also 8925 line |
| 29927 | TCP1 | NM_030752.1 | 1581 | tgcaatcaccattcttcgaattg | 102236 | - | see also 8925 line |
| 29928 | TCP1 | NM_030752.1 | 67 | acgatccgctcccaaaacgttat | 102201 | - | see also 8925 line |
| 29929 | TEC | NM_003215.1 | 1135 | aggcttcgtacccagttagtgt | 102265 | - | see also 13070 line |
| 29930 | TEC | NM_003215.1 | 1281 | atggcgagccagttacaaagtcg | 102267 | - | see also 13070 line |
| 29931 | TEK | NM_000459.1 | 3441 | agcgaaagacctacgttgaataac | 102371 | - | see also 358 line |
| 29932 | TEK | NM_000459.1 | 1273 | gccgctacctaattgaagaaa | 102313 | - | see also 358 line |
| 29933 | TESK1 | NM_006285.1 | 425 | ggcgcgtggacgattttcact | 102374 | - | see also 13072 line |
| 29934 | TESK1 | NM_006285.1 | 423 | ctggcgcgtgacgatttca | 102373 | - | see also 13072 line |
| 29935 | TESK2 | NM_007170.1 | 546 | tccagactgacgcgttggatga | 102392 | - | see also 13073 line |
| 29936 | TESK2 | NM_007170.1 | 1799 | acgcctaagtagtctcaagtaca | 102413 | - | see also 13073 line |
| 29937 | TEX14 | NM_031272.2 | 3052 | cacggattggcagcgagttattg | 102480 | - | see also 8999 line |
| 29938 | TEX14 | NM_031272.2 | 1499 | gccgtagtctcgcggaattattt | 102442 | - | see also 8999 line |
| 29939 | TGFBR1 | NM_004612.1 | 166 | ggcgacgcgttacagttgtttct | 102513 | - | see also 3133 line |
| 29940 | TGFBR1 | NM_004612.1 | 312 | ctcgagataggccgtttgatgt | 102517 | - | see also 3133 line |
| 29941 | TGFBR2 | NM_003242.3 | 414 | cacgttcagaagtcggttaataa | 102559 | - | see also 13076 line |
| 29942 | TGFBR2 | NM_003242.3 | 425 | gtcggttaataacgacatgatag | 102561 | - | see also 13076 line |
| 29943 | TGFBR2 | NM_003242.3 | 456 | aacaacggtgcagtcaagtttcc | 102562 | - | see also 13076 line |
| 29944 | TIE | NM_005424.2 | 794 | gaccatgacgcgcgaatgtgtatg | 102589 | - | see also 13077 line |
| 29945 | TIE | NM_005424.2 | 795 | accatgacgcgcgaatgtgtatgc | 102590 | - | see also 13077 line |
| 29946 | TIMM44 | NM_006351.2 | 94 | ccatggctgacctatcagatg | 102616 | - | see also 4444 line |
| 29947 | TIMM44 | NM_006351.2 | 476 | gtcggccgagtcggtatccaaag | 102625 | - | see also 4444 line |
| 29948 | TK1 | NM_003258.1 | 527 | tccgggaagccgcctataccaag | 102641 | - | see also 13079 line |

Figure 46

| 29949 | TK1 | NM_003258.1 | 133 | gggccgatgttctcaggaaaaag | 102640 | - | see also 13079 line |
|---|---|---|---|---|---|---|---|
| 29950 | TK2 | NM_004614.2 | 753 | gtcgattcacagcgcaagataca | 102648 | - | see also 13080 line |
| 29951 | TK2 | NM_004614.2 | 1097 | aacaaaatcgggatcgaatatta | 102655 | - | see also 13080 line |
| 29952 | TLK1 | NM_012290.3 | 2168 | gacgctgtttggcatatcgaaaa | 102718 | - | see also 5257 line |
| 29953 | TLK1 | NM_012290.3 | 1709 | aagaagctcggtctattgtaatg | 102701 | - | see also 5257 line |
| 29954 | TNK1 | NM_003985.1 | 619 | aggtatcggtcatgatgaacttg | 102730 | - | see also 13082 line |
| 29955 | TNK1 | NM_003985.1 | 1023 | gcctcggacgtgtggatgtttgg | 102731 | - | see also 13082 line |
| 29956 | TOM1L2 | NM_144678.2 | 172 | gtgaggattggacgttgaatatg | 102734 | - | see also 9730 line |
| 29957 | TOM1L2 | NM_144678.2 | 648 | gtcgttcgaggaaacacaaaagt | 102738 | - | see also 9730 line |
| 29958 | TOPK | NM_018492.2 | 491 | ttgttggttatcgtgcttttact | 102757 | - | see also 13084 line |
| 29959 | TOPK | NM_018492.2 | 326 | ctggggtaaatgtgtacctaatg | 102751 | - | see also 13084 line |
| 29960 | TOR1B | NM_014506.1 | 622 | gacgcaatcaagccgtttctaga | 102782 | - | see also 13085 line |
| 29961 | TOR1B | NM_014506.1 | 299 | atctagccacggaagtgattttc | 102774 | - | see also 13085 line |
| 29962 | TPX2 | NM_012112.4 | 1980 | accgagcctattggctttgattt | 102821 | - | see also 13086 line |
| 29963 | TPX2 | NM_012112.4 | 2212 | aggacgaaccggtagtgataaaa | 102829 | - | see also 13086 line |
| 29964 | TRA1 | NM_003299.1 | 2270 | cgcttcggtcagggtatctttta | 102864 | - | see also 2230 line |
| 29965 | TRA1 | NM_003299.1 | 333 | tgccttccaagccgaagttaaca | 102839 | - | see also 2230 line |
| 29966 | TRAD | NM_007064.1 | 3883 | tcccgcctagcatgcttcataga | 102934 | - | see also 4944 line |
| 29967 | TRAD | NM_007064.1 | 2700 | aacgtctgcaacagtcaaagtgc | 102914 | - | see also 4944 line |
| 29968 | TRAP1 | NM_016292.1 | 978 | cccgctacaccctgcactataag | 102958 | - | see also 13089 line |
| 29969 | TRAP1 | NM_016292.1 | 1238 | aggaaactccgggacgttttaca | 102963 | - | see also 13089 line |
| 29970 | TRAP100 | NM_014815.1 | 2575 | gagacaccgcgaagacattgagg | 103002 | - | see also 6010 line |
| 29971 | TRAP100 | NM_014815.1 | 2645 | atgcgactgctgagctctaatga | 103003 | - | see also 6010 line |
| 29972 | TRAP150 | NM_005119.1 | 561 | ctcgtcgaggccgttcaagatcc | 103014 | - | see also 3590 line |
| 29973 | TRAP150 | NM_005119.1 | 463 | tgggcgtaacagaggcttttatc | 103010 | - | see also 3590 line |
| 29974 | TRAP150 | NM_005119.1 | 267 | ctcgttctcgttcattttcgaag | 103007 | - | see also 3590 line |
| 29975 | TRB2 | NM_021643.1 | 2162 | agcgtctcgaattcagcatatgg | 103083 | - | see also 8162 line |
| 29976 | TRB2 | NM_021643.1 | 1299 | aagagttgtcgtctataaggtcc | 103062 | - | see also 8162 line |
| 29977 | TRIO | NM_007118.1 | 5608 | gccgagctcgtcagtgcaattga | 103173 | - | see also 13093 line |
| 29978 | TRIO | NM_007118.1 | 8594 | ttcgacctatccgtagcattaaa | 103218 | - | see also 13093 line |
| 29979 | TRIO | NM_007118.1 | 6345 | aggattcgacgggaaaatcgttg | 103189 | - | see also 13093 line |
| 29980 | TRIP13 | NM_004237.2 | 720 | taccgatatggccaattaattga | 103235 | - | see also 13094 line |
| 29981 | TRIP13 | NM_004237.2 | 906 | gcgggcaccgagccatcagatgc | 103239 | - | see also 13094 line |
| 29982 | TRPC4AP | NM_015638.1 | 976 | tggtacacatggctagacaatgc | 103276 | - | see also 6526 line |
| 29983 | TRPC4AP | NM_015638.1 | 1961 | gccgcctgctcgcctacatatcc | 103296 | - | see also 6526 line |
| 29984 | TRPM6 | NM_017662.3 | 4841 | cccttcgctaggagtcatagttt | 103418 | - | see also 7087 line |
| 29985 | TRPM6 | NM_017662.3 | 4839 | ggcccttcgctaggagtcatagt | 103417 | - | see also 7087 line |

Figure 46

| 29986 | TRPM7 | NM_017672.2 | 1991 | cagccctaccgaccaaagattga | 103518 | - | see also 7091 line |
|---|---|---|---|---|---|---|---|
| 29987 | TRPM7 | NM_017672.2 | 2688 | aagtacggattttggatagtaat | 103555 | - | see also 7091 line |
| 29988 | TRPM7 | NM_017672.2 | 2199 | ttgcctgtaagatctatcgttca | 103530 | - | see also 7091 line |
| 29989 | TTBK2 | NM_173500.2 | 2889 | ttcgtgtccgctcagagattact | 103696 | - | see also 10073 line |
| 29990 | TTBK2 | NM_173500.2 | 789 | tgggtcgctttcctagtacatgt | 103668 | - | see also 10073 line |
| 29991 | TTC12 | NM_017868.2 | 1785 | agctatctgcacgaatagttatc | 103779 | - | see also 13099 line |
| 29992 | TTC12 | NM_017868.2 | 1143 | cggacggagcctgatcatcaacc | 103762 | - | see also 13099 line |
| 29993 | TTK | NM_003318.3 | 1776 | taccggaacgaaatagcttattt | 103837 | - | see also 13100 line |
| 29994 | TTK | NM_003318.3 | 1305 | gagttagcccgaaaagttaatac | 103823 | - | see also 13100 line |
| 29995 | TTK | NM_003318.3 | 1777 | accggaacgaaatagcttatttg | 103838 | - | see also 13100 line |
| 29996 | TTN | NM_003319.2 | 6441 | aacggtctgaccggatctactgg | 104007 | - | see also 2250 line |
| 29997 | TXK | NM_003328.1 | 853 | cagctgggtttagctacgaaaag | 107034 | - | see also 13102 line |
| 29998 | TXK | NM_003328.1 | 226 | ggccgtggctcagccaattgtca | 107014 | - | see also 13102 line |
| 29999 | TXK | NM_003328.1 | 270 | ggccgtgtgcagccgtcaaaacg | 107019 | - | see also 13102 line |
| 30000 | TXL-2 | NM_178130.2 | 651 | aggcagaagtgcgacttttctac | 107071 | - | see also 13103 line |
| 30001 | TXL-2 | NM_178130.2 | 242 | ctcagttccaaaggactaactgt | 107059 | - | see also 13103 line |
| 30002 | TXNDC3 | NM_016616.2 | 964 | gtcaacttctatagtcgaatagc | 107092 | - | see also 13104 line |
| 30003 | TXNDC3 | NM_016616.2 | 2076 | atctaacgcctatgaagcaaaag | 107123 | - | see also 13104 line |
| 30004 | TYK2 | NM_003331.3 | 3485 | gcccgaaggccacgagtactacc | 107142 | - | see also 2256 line |
| 30005 | TYK2 | NM_003331.3 | 628 | atgcaagcctgatgctatatttc | 107127 | - | see also 2256 line |
| 30006 | TYRO3 | NM_006293.2 | 2756 | cagtgactgtcggtacatactca | 107175 | - | see also 13106 line |
| 30007 | TYRO3 | NM_006293.2 | 2336 | ggccgacaacctgtatactgtgc | 107167 | - | see also 13106 line |
| 30008 | UCK1 | NM_031432.1 | 423 | tggaggtgccgacctatgatttt | 107182 | - | see also 13107 line |
| 30009 | UCK1 | NM_031432.1 | 425 | gaggtgccgacctatgattttgt | 107183 | - | see also 13107 line |
| 30010 | UCK1 | NM_031432.1 | 202 | gtcgaccgtgtgtgagaagatca | 107178 | - | see also 13107 line |
| 30011 | ULK1 | NM_003565.1 | 2328 | gcccctttggccggtctttcagc | 107191 | - | see also 13108 line |
| 30012 | ULK1 | NM_003565.1 | 1772 | tacacgccatctcctcaagttgg | 107190 | - | see also 13108 line |
| 30013 | ULK1 | NM_003565.1 | 1064 | cgcatggacttcgatgagtttt | 107188 | - | see also 13108 line |
| 30014 | ULK2 | NM_014683.2 | 983 | tgctcgttacctacatagtaaca | 107209 | - | see also 5884 line |
| 30015 | ULK2 | NM_014683.2 | 1022 | gtgtggatccccgatgtacatgg | 107212 | - | see also 5884 line |
| 30016 | UMP-CMPK | NM_016308.1 | 187 | gcccgcatcgtcgagaaatatgg | 107257 | - | see also 13110 line |
| 30017 | UMP-CMPK | NM_016308.1 | 604 | aagccaattattgacttatatga | 107266 | - | see also 13110 line |
| 30018 | UMPK | NM_012474.3 | 663 | cccggctctcacgcagagtatta | 107274 | - | see also 13111 line |
| 30019 | UMPK | NM_012474.3 | 664 | ccggctctcacgcagagtattaa | 107275 | - | see also 13111 line |
| 30020 | URKL1 | NM_017859.1 | 1076 | gaccagtcgcgacgagttcatct | 107287 | - | see also 13112 line |

Figure 46

| 30021 | URKL1 | NM_017859.1 | 1614 | aactttggcgaccgctactttgg | 107292 | - | see also 13112 line |
|---|---|---|---|---|---|---|---|
| 30022 | VARS2 | NM_006295.1 | 525 | tacgccgacacggagttaatacc | 107293 | - | see also 4390 line |
| 30023 | VARS2 | NM_006295.1 | 528 | gccgacacggagttaataccagc | 107294 | - | see also 4390 line |
| 30024 | VARS2 | NM_006295.1 | 1823 | aacaactcggatcgagacaatgc | 107303 | - | see also 4390 line |
| 30025 | VCP | NM_007126.2 | 273 | aaccgtcccaatcggttaattgt | 107315 | - | see also 13114 line |
| 30026 | VCP | NM_007126.2 | 275 | ccgtcccaatcggttaattgttg | 107316 | - | see also 13114 line |
| 30027 | VPS11 | NM_021729.3 | 2633 | accggaaggtcatggatatgatc | 107386 | - | see also 13115 line |
| 30028 | VPS11 | NM_021729.3 | 592 | aagggcaactatcctgtaactgg | 107360 | - | see also 13115 line |
| 30029 | VPS4A | NM_013245.2 | 1178 | gtcggccacacacttcaaaaagg | 107410 | - | see also 5363 line |
| 30030 | VPS4A | NM_013245.2 | 243 | ttcctccacgctatcaagtatga | 107391 | - | see also 5363 line |
| 30031 | VPS4A | NM_013245.2 | 427 | aaggggataatccggagaaaaag | 107397 | - | see also 5363 line |
| 30032 | VPS4B | NM_004869.2 | 1436 | cggatatgttgcggtcactatct | 107448 | - | see also 13117 line |
| 30033 | VPS4B | NM_004869.2 | 1444 | ttgcggtcactatctaacacaaa | 107449 | - | see also 13117 line |
| 30034 | VRK1 | NM_003384.1 | 757 | acgagcatcgatgcacacaatgg | 107474 | - | see also 2280 line |
| 30035 | VRK1 | NM_003384.1 | 815 | ttggttattgcatgatccaatgg | 107477 | - | see also 2280 line |
| 30036 | VRK2 | NM_006296.2 | 1247 | cacacaataggttaatcgaaaaa | 107514 | - | see also 4391 line |
| 30037 | VRK2 | NM_006296.2 | 1245 | ggcacacaataggttaatcgaaa | 107513 | - | see also 4391 line |
| 30038 | VRK3 | NM_016440.2 | 208 | aagtatccaagcggcattcaaat | 107527 | - | see also 13120 line |
| 30039 | VRK3 | NM_016440.2 | 213 | tccaagcggcattcaaattctgc | 107528 | - | see also 13120 line |
| 30040 | WARS | NM_004184.2 | 772 | gacggatgacgagaagtatctgt | 107560 | - | see also 13121 line |
| 30041 | WARS | NM_004184.2 | 498 | ttgttcggtttggaagtagtaaa | 107544 | - | see also 13121 line |
| 30042 | WARS2 | NM_015836.1 | 712 | ctggccaccgtccgaataacaga | 107594 | - | see also 13122 line |
| 30043 | WARS2 | NM_015836.1 | 814 | cgcgctggcgtgtccaacatagt | 107595 | - | see also 13122 line |
| 30044 | WEE1 | NM_003390.2 | 3002 | aggtccaccacccagagtaatag | 107653 | - | see also 2287 line |
| 30045 | WEE1 | NM_003390.2 | 1701 | ccccgcacacgcccaagagtttg | 107602 | - | see also 2287 line |
| 30046 | XAB1 | NM_007266.1 | 165 | cactccaccgtatgtgatcaacc | 107659 | - | see also 13124 line |
| 30047 | XAB1 | NM_007266.1 | 823 | agggagtatcgtcctgaatatga | 107672 | - | see also 13124 line |
| 30048 | YES1 | NM_005433.2 | 1343 | ctgtatggtcggtttacaataaa | 107706 | - | see also 13125 line |
| 30049 | YES1 | NM_005433.2 | 1347 | atggtcggtttacaataaagtct | 107707 | - | see also 13125 line |
| 30050 | YME1L1 | NM_014263.2 | 957 | gtccgcttccggacaacaacagg | 107731 | - | see also 5570 line |
| 30051 | YME1L1 | NM_014263.2 | 1293 | aaggcgaatgctccttgtgttat | 107742 | - | see also 5570 line |
| 30052 | ZAK | NM_016653.1 | 481 | aagtcaagaaacgttgttatagc | 107776 | - | see also 6967 line |
| 30053 | ZAK | NM_016653.1 | 534 | tggtgcctctcggttccataacc | 107778 | - | see also 6967 line |
| 30054 | ZAP70 | NM_001079.2 | 726 | gccgagcgcaaactttactctgg | 107847 | - | see also 13128 line |
| 30055 | ZAP70 | NM_001079.2 | 724 | aggccgagcgcaaactttactct | 107846 | - | see also 13128 line |
| 30056 | ZCWCC3 | NM_015358.1 | 1946 | cccgatgcgaccagggaaatact | 107919 | - | see also 13129 line |
| 30057 | ZCWCC3 | NM_015358.1 | 388 | ctgggtaaagacgcaatcgtttt | 107870 | - | see also 13129 line |

Figure 46

| 30058 | CNGA3 | NM_001298.1 | 525 | cccgtccagcaacctgtactacc | 107948 | - | see also 858 line | | |
|---|---|---|---|---|---|---|---|---|---|
| 30059 | CNGA3 | NM_001298.1 | 858 | gaggttcaaccgcctactgaagt | 107952 | - | see also 858 line | | |
| 30060 | EPAC | NM_006105.2 | 915 | aactcggtgaagcgagaattagc | 107958 | - | see also 13131 line | | |
| 30061 | EPAC | NM_006105.2 | 1307 | aggcaggaaccggtatacagtga | 107965 | - | see also 13131 line | | |
| 30062 | HCN2 | NM_001194.2 | 2701 | cgcgcctctcgtccaacttgtga | 107976 | - | channel | - | - |
| 30063 | HCN3 | NM_020897.1 | 393 | ttcaacgtattgtctgatacttt | 107980 | - | see also 8020 line | | |
| 30064 | HCN3 | NM_020897.1 | 169 | agcctacggtcaacaagttctcc | 107977 | - | see also 8020 line | | |
| 30065 | HCN3 | NM_020897.1 | 1234 | cgcttcgcgaggagatcattaac | 107986 | - | see also 8020 line | | |
| 30066 | HCN4 | NM_005477.1 | 1740 | tcccgcctcattcgatatattca | 108002 | - | see also 13133 line | | |
| 30067 | HCN4 | NM_005477.1 | 1739 | ctcccgcctcattcgatatattc | 108001 | - | see also 13133 line | | |
| 30068 | HCN4 | NM_005477.1 | 1112 | ctcggtggacaccgctatcaaag | 107993 | - | see also 13133 line | | |
| 30069 | PRKAR1A | NM_002734.2 | 455 | tgcggcatcctatgttagaaagg | 108017 | - | see also 1773 line | | |
| 30070 | PRKAR1A | NM_002734.2 | 1157 | cccctt gaagtgcgttaagctgg | 108039 | - | see also 1773 line | | |
| 30071 | PRKAR2A | NM_004157.1 | 725 | tagaacggggaacttatgacatt | 108047 | - | see also 2797 line | | |
| 30072 | PRKAR2A | NM_004157.1 | 721 | gtcatagaacggggaacttatga | 108046 | - | see also 2797 line | | |
| 30073 | PRKAR2B | NM_002736.1 | 1012 | ttctgaacgcctgaaagtagtag | 108085 | - | see also 13136 line | | |
| 30074 | PRKAR2B | NM_002736.1 | 828 | tcggcgaactggccttaatgtac | 108079 | - | see also 13136 line | | |
| 30075 | ACAS2 | NM_018677.2 | 304 | gcccattccttcggtacaacttt | 108100 | - | see also 7550 line | | |
| 30076 | ADSS | NM_001126.2 | 1359 | tactgcgttggcacttaccaagt | 108153 | - | see also 714 line | | |
| 30077 | ADSS | NM_001126.2 | 1311 | ctggttggacctcgttttgctca | 108150 | - | see also 714 line | | |
| 30078 | ADSSL1 | NM_152328.3 | 620 | ctgcgacctcctgtcagattttg | 108166 | - | see also 13139 line | | |
| 30079 | ADSSL1 | NM_152328.3 | 1176 | gacgtactgggtgaggttaaagt | 108179 | - | see also 13139 line | | |
| 30080 | ADSSL1 | NM_152328.3 | 1125 | cacatggtcaacggattcactgc | 108178 | - | see also 13139 line | | |
| 30081 | APMCF1 | NM_021203.2 | 232 | ttgtgattccgggaaaacgttgc | 108189 | - | see also 8138 line | | |
| 30082 | APMCF1 | NM_021203.2 | 252 | tgctctttgtcaggttgttaaca | 108191 | - | see also 8138 line | | |
| 30083 | ARF1 | NM_001658.2 | 559 | agcggcgacgggctctatgaagg | 108210 | - | see also 13141 line | | |
| 30084 | ARF1 | NM_001658.2 | 460 | gacctccccaacgccatgaatgc | 108209 | - | see also 13141 line | | |
| 30085 | ARF3 | NM_001659.1 | 563 | cacccaagggttgatatttgtgg | 108211 | - | signal_transduction | small monomeric GTPase | - |
| 30086 | ARF4 | NM_001660.2 | 229 | tccctcttctcccgactatttgg | 108213 | - | see also 13142 line | | |
| 30087 | ARF4 | NM_001660.2 | 228 | ctccctcttctcccgactatttg | 108212 | - | see also 13142 line | | |
| 30088 | ARF4L | NM_001661.1 | 435 | gacagacggtctagtgtttgtgg | 108223 | - | see also 13143 line | | |
| 30089 | ARF4L | NM_001661.1 | 432 | ccggacagacggtctagtgtttg | 108221 | - | see also 13143 line | | |
| 30090 | ARF5 | NM_001662.2 | 172 | atcccaaccataggcttcaatgt | 108226 | - | signal_transduction | protein transporter | - |
| 30091 | ARF5 | NM_001662.2 | 345 | tgctgatgaactccagaagatgc | 108227 | - | see also 30090 line | | |
| 30092 | ARF5 | NM_001662.2 | 126 | gaccacaatcctgtacaaactga | 108225 | - | see also 30090 line | | |
| 30093 | ARFD1 | NM_001656.2 | 1236 | ttcgggtcgttacgttaggattg | 108278 | - | see also 1095 line | | |

Figure 46

| | | | | | | | |
|---|---|---|---|---|---|---|---|
| 30094 | ARFD1 | NM_001656.2 | 866 | gagaatgcccggtcatgtattcg | - | see also 1095 line | |
| 30095 | ARFIP2 | NM_012402.2 | 431 | atggggatcaacaacctataagt | - | see also 5310 line | |
| 30096 | ARFIP2 | NM_012402.2 | 935 | ggcccatcggacaagtatgaga | - | see also 5310 line | |
| 30097 | ARFRP1 | NM_003224.2 | 262 | aacccgatttaacaagaactaca | - | see also 2183 line | |
| 30098 | ARFRP1 | NM_003224.2 | 159 | tgctgtcgggcttgtacaagtac | - | see also 2183 line | |
| 30099 | ARHA | NM_001664.1 | 371 | tacccagataccgatgttatact | - | see also 13148 line | |
| 30100 | ARHA | NM_001664.1 | 373 | cccagataccgatgttatactga | - | see also 13148 line | |
| 30101 | ARHA | NM_001664.1 | 410 | gacagccctgatagtttagaaaa | - | see also 13148 line | |
| 30102 | ARHB | NM_004040.1 | 554 | cccagaacggctgcatcaactgc | - | see also 13149 line | |
| 30103 | ARHC | NM_175744.3 | 194 | gtctacgtccctactgtcttttga | - | signal_transduction, kinase_related; see also 30103 line | Rho small monomeric GTPase; breast cancer, pancreatic cancer, melanoma |
| 30104 | ARHC | NM_175744.3 | 630 | gcctccaggtcgcaagaacaag | - | see also 30103 line | |
| 30105 | ARHD | NM_014578.2 | 566 | ctcggctggctccatgcatgacaacg | - | see also 13150 line | |
| 30106 | ARHD | NM_014578.2 | 383 | gtggtacccagaagtgaatcatt | - | see also 13150 line | |
| 30107 | ARHE | NM_005168.2 | 556 | tgcggacagatgttagtacatta | - | see also 3637 line | |
| 30108 | ARHF | NM_019034.2 | 202 | ccatcgtgttcgagagaagtaca | - | see also 13152 line | |
| 30109 | ARHF | NM_019034.2 | 341 | atctgctatgacgtcatgaatcc | - | see also 13152 line | |
| 30110 | ARHG | NM_001665.1 | 345 | ccctcagaccaacgttttcgtca | - | see also 13153 line | |
| 30111 | ARHG | NM_001665.1 | 185 | tgctcatcgtacacaactaac | - | see also 13153 line | |
| 30112 | ARHGAP1 | NM_004308.2 | 244 | aagtggatgaccatactatga | - | see also 13154 line | |
| 30113 | ARHGAP1 | NM_004308.2 | 859 | agggagactgttgcctacttaca | - | see also 13154 line | |
| 30114 | ARHGEF5 | NM_005435.2 | 1303 | ttccgcacggagactcaaagtga | - | see also 13155 line | |
| 30115 | ARHGEF5 | NM_005435.2 | 1420 | tacaagccccgagagaatgatga | - | see also 13155 line | |
| 30116 | ARHGEF5 | NM_005435.2 | 1225 | aagctacatggacctcacaaaaa | - | see also 13155 line | |
| 30117 | ARHH | NM_004310.1 | 1121 | ggctcttctccatcaatgagtgc | - | see also 13156 line | |
| 30118 | ARHI | NM_004675.1 | 341 | gagtacctgccgaccattgaaaa | - | see also 13157 line | |
| 30119 | ARHI | NM_004675.1 | 344 | tacctgccgaccattgaaaatac | - | see also 13157 line | |
| 30120 | ARHJ | NM_020663.2 | 833 | ctcataggqaccaccagattgatct | - | see also 13158 line | |
| 30121 | ARHJ | NM_020663.2 | 877 | ggcccgtttgctgtatatgaaag | - | see also 13158 line | |
| 30122 | ARHN | NM_005440.2 | 88 | gacgcctatcccgggagttatgt | - | see also 13159 line | |
| 30123 | ARHN | NM_005440.2 | 90 | cgcctatcccggagttatgtcc | - | see also 13159 line | |
| 30124 | ARHT1 | NM_018307.2 | 1426 | tcctactatgcgattaacactgt | - | see also 7378 line | |
| 30125 | ARHT1 | NM_018307.2 | 1161 | gacgctcacgacttatttagatg | - | see also 7378 line | |
| 30126 | ARHT2 | NM_138769.1 | 1582 | cagcgtcacaagcaccattaca | - | see also 13161 line | |
| 30127 | ARHT2 | NM_138769.1 | 627 | aggcgctgacgcgcatcttcagg | - | see also 13161 line | |
| 30128 | ARHU | NM_021205.3 | 1274 | gccatcgtgctggccattcaata | - | see also 13162 line | |
| 30129 | ARHU | NM_021205.3 | 1106 | cagtcggatccagagaagatgt | - | see also 13162 line | |

Figure 46

| 30130 | ARHV | NM_133639.2 | 547 | gacgatgtcaacgtactaattca | 108495 | - | see also 13163 line | | |
|---|---|---|---|---|---|---|---|---|---|
| 30131 | ARHV | NM_133639.2 | 662 | cagccttgacgcagaagaacttg | 108496 | - | see also 13163 line | | |
| 30132 | ARL1 | NM_001177.3 | 439 | gagaccgaattggcatttccaaa | 108507 | - | see also 13164 line | | |
| 30133 | ARL1 | NM_001177.3 | 586 | ctgccttgaaggaccgaaaatgg | 108508 | - | see also 13164 line | | |
| 30134 | ARL3 | NM_004311.2 | 293 | cacacctacacagggtttcaaca | 108514 | - | see also 13165 line | | |
| 30135 | ARL3 | NM_004311.2 | 320 | aagtgtacaatcacaaggtttta | 108517 | - | see also 13165 line | | |
| 30136 | ARL3 | NM_004311.2 | 295 | cacctacacagggtttcaacatc | 108515 | - | see also 13165 line | | |
| 30137 | ARL5 | NM_012097.2 | 634 | tagcgcatgaggacctaagaaaa | 108535 | - | see also 5127 line | | |
| 30138 | ARL5 | NM_012097.2 | 819 | atgatgtcacgacttaagattag | 108541 | - | see also 5127 line | | |
| 30139 | ARL6 | NM_032146.3 | 712 | accgtcgaattccaatcttattc | 108555 | - | see also 9083 line | | |
| 30140 | ARL6 | NM_032146.3 | 711 | caccgtcgaattccaatcttatt | 108554 | - | see also 9083 line | | |
| 30141 | ARL6 | NM_032146.3 | 436 | gtggcaaaacgacgatcattaac | 108545 | - | see also 9083 line | | |
| 30142 | ARL6IP2 | NM_022374.1 | 1283 | gtggcgataaaacaatttcgttc | 108604 | - | see also 8355 line | | |
| 30143 | ARL6IP2 | NM_022374.1 | 121 | gacctccctaggtgagaattatg | 108564 | - | see also 8355 line | | |
| 30144 | ARL6IP2 | NM_022374.1 | 1284 | tggcgataaaacaatttcgttca | 108605 | - | see also 8355 line | | |
| 30145 | ARL8 | NM_178815.2 | 517 | gacagggaacgactagctattac | 108627 | - | see also 13169 line | | |
| 30146 | ARL8 | NM_178815.2 | 521 | gggaacgactagctattacaaaa | 108629 | - | see also 13169 line | | |
| 30147 | C14orf159 | NM_024952.3 | 2167 | ctcccgtcggtcattaaggaaga | 108660 | - | see also 13170 line | | |
| 30148 | C14orf159 | NM_024952.3 | 1588 | aaggaggtcgccataatcgttga | 108643 | - | see also 13170 line | | |
| 30149 | CDC10 | NM_001788.2 | 91 | gccaatctcccaaatcaagtata | 108661 | - | cell_cycle | structural molecule | neuroblastoma |
| 30150 | CDC10 | NM_001788.2 | 95 | atctcccaaatcaagtatacaga | 108662 | - | see also 30149 line | | |
| 30151 | CDC10 | NM_001788.2 | 97 | ctcccaaatcaagtatacagaaa | 108663 | - | see also 30149 line | | |
| 30152 | CDC42 | NM_001791.2 | 160 | tcctgatatcctacacaacaaac | 108667 | - | see also 13171 line | | |
| 30153 | CDC42 | NM_001791.2 | 157 | gtctcctgatatcctacacaaca | 108666 | - | see also 13171 line | | |
| 30154 | CDC42 | NM_001791.2 | 136 | atggtgctgttggtaaaacatgt | 108665 | - | see also 13171 line | | |
| 30155 | DIRAS2 | NM_017594.2 | 323 | agcccatctacgaacaaatctgc | 108673 | - | see also 13172 line | | |
| 30156 | DIRAS2 | NM_017594.2 | 483 | agccaagctcaaccataacgtga | 108675 | - | see also 13172 line | | |
| 30157 | DIRAS2 | NM_017594.2 | 557 | tccagatcgacgggaaaaagagc | 108676 | - | see also 13172 line | | |
| 30158 | DKFZP564J0863 | NM_015459.2 | 909 | tacggtcgtctggcaatggatga | 108683 | - | see also 13173 line | | |
| 30159 | DKFZP564J0863 | NM_015459.2 | 1288 | gtcggggactactggagtatttt | 108695 | - | see also 13173 line | | |
| 30160 | DNCLI1 | NM_016141.1 | 755 | acctctgggtgcggatacactta | 108723 | - | see also 6690 line | | |
| 30161 | DNCLI1 | NM_016141.1 | 1139 | aactaaaccacctgttcgaaagt | 108734 | - | see also 6690 line | | |
| 30162 | DNCLI1 | NM_016141.1 | 786 | ttgggcattccagtactagtagt | 108726 | - | see also 6690 line | | |
| 30163 | DNM1 | NM_004408.1 | 1143 | agcccgcattaaccgaatcttcc | 108754 | - | see also 13175 line | | |
| 30164 | DNM1 | NM_004408.1 | 1176 | ccctttcgagctggtcaagatgg | 108755 | - | see also 13175 line | | |

Figure 46

| 30165 | DNM1L | NM_005690.2 | 2015 | agctagtaggccagctgtataaa | 108834 | - | see also 3950 line |
|---|---|---|---|---|---|---|---|
| 30166 | DNM1L | NM_005690.2 | 1894 | gaggttattgaacgactcattaa | 108827 | - | see also 3950 line |
| 30167 | DNM2 | NM_004945.1 | 1026 | ggccctacgtagcaaactacaga | 108852 | - | see also 3443 line |
| 30168 | DNM2 | NM_004945.1 | 1514 | agcgaatcgtcaccacttacatc | 108860 | - | see also 3443 line |
| 30169 | DRG2 | NM_001388.3 | 666 | agctcatcctgcacgaatacaag | 108873 | - | see also 13178 line |
| 30170 | DRG2 | NM_001388.3 | 338 | accactctgacgtgtattcctgg | 108869 | - | see also 13178 line |
| 30171 | FLJ10101 | NM_024718.2 | 692 | ctgcaacggggtggtcatgatgt | 108881 | - | see also 13179 line |
| 30172 | FLJ10101 | NM_024718.2 | 729 | cagtggacttcaattacattct | 108882 | - | see also 13179 line |
| 30173 | FLJ10702 | NM_018184.1 | 292 | atcgcgtcaggtcaattcagtga | 108885 | - | see also 13180 line |
| 30174 | FLJ10702 | NM_018184.1 | 354 | aactaaagttaacgtcacaataa | 108888 | - | see also 13180 line |
| 30175 | FLJ10849 | NM_018243.2 | 498 | cagctataagccgatagtagaat | 108919 | - | see also 13181 line |
| 30176 | FLJ10849 | NM_018243.2 | 499 | agctataagccgatagtagaata | 108920 | - | see also 13181 line |
| 30177 | FLJ21665 | NM_024803.1 | 702 | atcgtaaactcggtgttgaatgc | 108941 | - | see also 13182 line |
| 30178 | FLJ21665 | NM_024803.1 | 368 | tacgcgcgaggccgttactctgt | 108934 | - | see also 13182 line |
| 30179 | FLJ22595 | NM_025047.1 | 432 | accgatgggctggtgtatgttgt | 108962 | - | see also 13183 line |
| 30180 | FLJ22595 | NM_025047.1 | 189 | atgggttcgctgggttctaaaaa | 108956 | - | see also 13183 line |
| 30181 | FLJ22655 | NM_024730.2 | 498 | atccgggagccacaaactagtca | 108969 | - | see also 13184 line |
| 30182 | FLJ22655 | NM_024730.2 | 556 | ttggcaacaaacgagatcttgt | 108972 | - | see also 13184 line |
| 30183 | FLJ25410 | NM_144605.1 | 1018 | taccggtcatcagactcaatga | 108983 | - | see also 13185 line |
| 30184 | FLJ25410 | NM_144605.1 | 1021 | cgcgtcatcagactcaatgaaag | 108985 | - | see also 13185 line |
| 30185 | FLJ39249 | NM_173664.2 | 666 | ctgggtctacaggctatcgataa | 108989 | - | see also 13186 line |
| 30186 | FLJ39249 | NM_173664.2 | 668 | gggtctacaggctatcgataacc | 108991 | - | see also 13186 line |
| 30187 | GBP1 | NM_002053.1 | 1822 | gacgaaaggcatgtaccataagc | 108993 | - | see also 13187 line |
| 30188 | GBP2 | NM_004120.3 | 869 | ttggagctttcgctaaagctaag | 108995 | - | see also 13188 line |
| 30189 | GBP2 | NM_004120.3 | 873 | agctttcgctaaagctaagaaaa | 108997 | - | see also 13188 line |
| 30190 | GBP3 | NM_018284.1 | 1762 | aggaacaggcccgagtactaaag | 109016 | - | see also 13189 line |
| 30191 | GBP3 | NM_018284.1 | 1796 | aggtgaaagtaccaacttcaaa | 109018 | - | see also 13189 line |
| 30192 | GBP4 | NM_052941.2 | 1410 | aacaggttgagtgggacttataag | 109032 | - | see also 13190 line |
| 30193 | GBP4 | NM_052941.2 | 393 | tgggcgatgtagaaaagagtaac | 109025 | - | see also 13190 line |
| 30194 | GBP5 | NM_052942.2 | 1716 | ctggaagcatcctcggattattg | 109043 | - | see also 13191 line |
| 30195 | GBP5 | NM_052942.2 | 2238 | cacgcccagagagactgttaataa | 109050 | - | see also 13191 line |
| 30196 | GEM | NM_005261.2 | 841 | tgccgagaagtgtctgtatcaga | 109057 | - | see also 13192 line |
| 30197 | GEM | NM_005261.2 | 613 | ggggaaagtgcaacgattatact | 109054 | - | see also 13192 line |
| 30198 | GNA12 | NM_007353.1 | 560 | accggatcggccagctgaattac | 109065 | - | see also 13193 line |
| 30199 | GNA12 | NM_007353.1 | 561 | ccggatcggccagctgaattact | 109066 | - | see also 13193 line |
| 30200 | GNA13 | NM_006572.3 | 1130 | tccgttgacgtgaaggattacatt | 109093 | - | see also 4606 line |
| 30201 | GNA13 | NM_006572.3 | 357 | tgctcgagagaagcttcatattc | 109072 | - | see also 4606 line |

Figure 46

| | | | | | | | |
|---|---|---|---|---|---|---|---|
| 30202 | GNA14 | NM_004297.2 | 778 | agccgatggacacgctaaggatac | 109098 | - | see also 2880 line |
| 30203 | GNA14 | NM_004297.2 | 997 | ccgcatcgccacaccatcattcg | 109104 | - | see also 2880 line |
| 30204 | GNA15 | NM_002068.1 | 1004 | tggaactaccctgttcaaaagc | 109117 | - | see also 1335 line |
| 30205 | GNA15 | NM_002068.1 | 915 | ggcctcactgagtgaattacgacc | 109116 | - | see also 1335 line |
| 30206 | GNAI1 | NM_002069.4 | 1178 | agccctcactatatgctatcc | 109146 | - | see also 13197 line |
| 30207 | GNAI1 | NM_002069.4 | 763 | atcccgagagtaccagcttaatg | 109131 | - | see also 13197 line |
| 30208 | GNAI2 | NM_002070.1 | 159 | ggccgagcgctaagatgatcg | 109148 | - | see also 13198 line |
| 30209 | GNAI2 | NM_002070.1 | 647 | tgctacggacccgcgtaaagacc | 109152 | - | see also 13198 line |
| 30210 | GNAI3 | NM_006496.1 | 301 | gccatgggacggctaaagattga | 109162 | - | see also 13199 line |
| 30211 | GNAI3 | NM_006496.1 | 300 | agccatggacggctaaagattg | 109161 | - | see also 13199 line |
| 30212 | GNAL | NM_002071.1 | 1056 | gtggttacggaccatttctatca | 109198 | - | see also 1336 line |
| 30213 | GNAL | NM_002071.1 | 1054 | aggtggttacggaccatttctat | 109197 | - | see also 1336 line |
| 30214 | GNAO1 | NM_020988.1 | 273 | gggcatcgaatatggtgataagg | 109212 | - | see also 13201 line |
| 30215 | GNAO1 | NM_020988.1 | 614 | aggcgatctgaaccgcaagaagtgg | 109215 | - | see also 13201 line |
| 30216 | GNAS | NM_000516.3 | 339 | aaacctgaaagaggggattgaaac | 109224 | - | see also 13202 line |
| 30217 | GNAS | NM_000516.3 | 340 | acctgaaagagaggcgattgaaacc | 109225 | - | see also 13202 line |
| 30218 | GNAT1 | NM_000172.2 | 504 | tccggtatccaggcctgtttga | 109242 | - | see also 13203 line |
| 30219 | GNAT1 | NM_000172.2 | 506 | cggtatccaggcctgtttgagc | 109243 | - | see also 13203 line |
| 30220 | GNAT1 | NM_000172.2 | 944 | gggcacctcagccatcgtttcc | 109245 | - | see also 13203 line |
| 30221 | GNAT2 | NM_005272.2 | 1103 | atgatgcgggaattacatataag | 109259 | - | see also 13204 line |
| 30222 | GNAT2 | NM_005272.2 | 985 | ttctttgccggctacttccattgt | 109257 | - | see also 13204 line |
| 30223 | GNAZ | NM_002073.1 | 1036 | gacgtcatcatacagaacaatct | 109267 | - | see also 1338 line |
| 30224 | GNL1 | NM_005275.2 | 2504 | ggcggctcggaatgatgtgtaca | 109289 | - | see also 13206 line |
| 30225 | GNL1 | NM_005275.2 | 1249 | gaggctacgacccaaatcgatac | 109270 | - | see also 13206 line |
| 30226 | GTPBP4 | NM_012341.1 | 1844 | ggcggatagacacgtgtttgata | 109339 | - | see also 5299 line |
| 30227 | GTPBP4 | NM_012341.1 | 59 | gccgtccgccaaggacttcatag | 109290 | - | see also 5299 line |
| 30228 | GTPBP4 | NM_012341.1 | 251 | tccgttctatgctgatttgatga | 109299 | - | see also 5299 line |
| 30229 | GTPBP5 | NM_015666.2 | 1243 | acgcaatcgtgcaaacaagatt | 109350 | - | see also 13208 line |
| 30230 | GTPBP5 | NM_015666.2 | 1244 | cgcaatcgtcgcaaacaagattg | 109351 | - | see also 13208 line |
| 30231 | HRAS | NM_005343.1 | 88 | gacgaataacgaccccactataga | 109353 | - | cell_cycle, signal_transduction — bladder transitional cell carcinoma, congenital neutropenia, colorectal cancer, bladder cancer, breast cancer, lung cancer, ovarian cancer, colorectal cancers, non-small-cell lung cancer, head and neck squamous cell carcinoma |

Figure 46

| | | | | | | | | | |
|---|---|---|---|---|---|---|---|---|---|
| 30232 | HUMAUANTIG | NM_013285.1 | 447 | atcgaatccggcctcataacttg | 109359 | - | see also 5386 line | | |
| 30233 | HUMAUANTIG | NM_013285.1 | 531 | aacgaccaaacttatttgcaagt | 109363 | - | see also 5386 line | | |
| 30234 | KBRAS2 | NM_017595.4 | 309 | agcaggtgcgtttctatgacacc | 109411 | - | see also 13209 line | | |
| 30235 | KIAA0820 | NM_015569.1 | 2697 | caccaactcgtcccactataatc | 109468 | - | see also 6486 line | | |
| 30236 | KIAA0820 | NM_015569.1 | 2698 | accaactcgtcccactataatcc | 109469 | - | see also 6486 line | | |
| 30237 | KIAA0820 | NM_015569.1 | 1230 | gtggtgctaaaatcaatcgtatt | 109438 | - | see also 6486 line | | |
| 30238 | KRAS2 | NM_004985.3 | 269 | gacgaatatgatccaacaataga | 109471 | - | see also 3479 line | | |
| 30239 | KRAS2 | NM_004985.3 | 243 | tacagctaattcagaatcatttt | 109470 | - | see also 3479 line | | |
| 30240 | LOC127829 | NM_138795.2 | 235 | cacgctggtcgggcttcagtact | 109487 | - | signal_transduction | - | - |
| 30241 | LOC127829 | NM_138795.2 | 563 | gaccttccgggagcattggatga | 109488 | - | see also 30240 line | | |
| 30242 | LOC127829 | NM_138795.2 | 683 | accctacagtggcttattcaaca | 109489 | - | see also 30240 line | | |
| 30243 | LOC51277 | NM_016544.1 | 672 | tgccattcgcagaattcgaaata | 109514 | - | see also 6910 line | | |
| 30244 | LOC51277 | NM_016544.1 | 466 | accaaacatcgctgtgtagatga | 109504 | - | see also 6910 line | | |
| 30245 | LOC51762 | NM_016530.1 | 534 | tggagacaagcgcaaaatccagt | 109521 | - | see also 13212 line | | |
| 30246 | LOC51762 | NM_016530.1 | 582 | cacttgcacgagatataatgaca | 109522 | - | see also 13212 line | | |
| 30247 | MEL | NM_005370.4 | 374 | agctcgatggcaagagaattaaa | 109527 | - | see also 13213 line | | |
| 30248 | MEL | NM_005370.4 | 555 | tgcagacgtcgaaaagatgatac | 109533 | - | see also 13213 line | | |
| 30249 | MEL | NM_005370.4 | 552 | ctctgcagacgtcgaaaagatga | 109532 | - | see also 13213 line | | |
| 30250 | MGC10731 | NM_030907.2 | 523 | gccgtgtcgtcatgtttcgtttt | 109550 | - | see also 13214 line | | |
| 30251 | MGC10731 | NM_030907.2 | 571 | cactcaaaaagttcgatcatatg | 109552 | - | see also 13214 line | | |
| 30252 | MGC10731 | NM_030907.2 | 524 | ccgtgtcgtcatgtttcgttttg | 109551 | - | see also 13214 line | | |
| 30253 | MGC2827 | NM_023940.2 | 332 | tccggttcctcaccaaacgattc | 109557 | - | see also 13215 line | | |
| 30254 | MGC2827 | NM_023940.2 | 374 | atgcaggtaatctctatactaga | 109561 | - | see also 13215 line | | |
| 30255 | MGC4083 | NM_032525.1 | 204 | gtcatcgtctcagaaatatgtgc | 109567 | - | see also 13216 line | | |
| 30256 | MGC4083 | NM_032525.1 | 279 | tgggccttttgggcagcttttcc | 109568 | - | see also 13216 line | | |
| 30257 | MGC45806 | NM_152304.1 | 434 | tggagacctcggttaaaaacaac | 109569 | - | signal_transduction | - | - |
| 30258 | MGC9726 | NM_177403.3 | 534 | tggctgcatcctagcttttgatg | 109574 | - | see also 13217 line | | |
| 30259 | MGC9726 | NM_177403.3 | 381 | gacgttttatgaggaataccaga | 109570 | - | see also 13217 line | | |
| 30260 | MRAS | NM_012219.2 | 521 | ttccatgacctcgttagagtaat | 109585 | - | see also 13218 line | | |

Figure 46

| 30261 | MRAS | NM_012219.2 | 461 | cacaatattccgtacatagaaac | 109584 | - | see also 13218 line |
|---|---|---|---|---|---|---|---|
| 30262 | MRAS | NM_012219.2 | 522 | tccatgacctcgttagagtaatt | 109586 | - | see also 13218 line |
| 30263 | MSF | NM_006640.2 | 1232 | gtccatcacgcacgatattgagg | 109593 | - | see also 13219 line |
| 30264 | MSF | NM_006640.2 | 1760 | gtggggtaccatcgaagttgaaa | 109599 | - | see also 13219 line |
| 30265 | MX1 | NM_002462.2 | 856 | gtcccggatctgactctaataga | 109609 | - | see also 13220 line |
| 30266 | MX1 | NM_002462.2 | 858 | cccggatctgactctaatagacc | 109610 | - | see also 13220 line |
| 30267 | MX2 | NM_002463.1 | 1902 | ctggggacgccttcacagaatat | 109653 | - | see also 13221 line |
| 30268 | MX2 | NM_002463.1 | 1904 | ggggacgccttcacagaatatga | 109655 | - | see also 13221 line |
| 30269 | NEDD5 | NM_004404.1 | 971 | ctgtggttggatccaatcagttg | 109670 | - | see also 13222 line |
| 30270 | NEDD5 | NM_004404.1 | 690 | tagggtgcattgttgcttttact | 109667 | - | see also 13222 line |
| 30271 | NRAS | NM_002524.2 | 611 | ttgccaacaaggacagttgatac | 109678 | - | see also 13223 line |
| 30272 | NRAS | NM_002524.2 | 517 | gtcatttgcggatattaacctct | 109677 | - | see also 13223 line |
| 30273 | OPA1 | NM_015560.1 | 432 | taccggaccttagtgaatataaa | 109696 | - | see also 6473 line |
| 30274 | OPA1 | NM_015560.1 | 1084 | tagttctcgggagtttgatctta | 109716 | - | see also 6473 line |
| 30275 | OPA1 | NM_015560.1 | 2839 | tactggtaaacgcgttcaactgg | 109781 | - | see also 6473 line |
| 30276 | PCK1 | NM_002591.2 | 1784 | ttcaaccggatcgatggaaaagc | 109807 | - | see also 13225 line |
| 30277 | PCK1 | NM_002591.2 | 716 | ggcgatggggagtttgtcaaatg | 109798 | - | see also 13225 line |
| 30278 | PCK2 | NM_004563.1 | 1448 | tggtatacgaggccttcaactgg | 109830 | - | see also 13226 line |
| 30279 | PCK2 | NM_004563.1 | 617 | tggtggcaagcatgcgtattatg | 109818 | - | see also 13226 line |
| 30280 | PGPL | NM_012227.1 | 536 | tgccccgaccaagaaagaactgg | 109834 | - | see also 13227 line |
| 30281 | PGPL | NM_012227.1 | 670 | cgctgcacaggtcgaacttgaaa | 109835 | - | see also 13227 line |
| 30282 | PNUTL1 | NM_002688.2 | 339 | gacggtagagattctaaaacaca | 109841 | - | see also 13228 line |
| 30283 | PNUTL1 | NM_002688.2 | 337 | cagacggtagagattctaaaaca | 109840 | - | see also 13228 line |
| 30284 | PNUTL2 | NM_004574.2 | 234 | aagttcgtgaaggatttctcagg | 109845 | - | see also 3111 line |
| 30285 | PNUTL2 | NM_004574.2 | 456 | ggcaagcttgatccctatgattc | 109846 | - | see also 3111 line |
| 30286 | PTD004 | NM_013341.2 | 158 | ttcccgttctgcactattgatcc | 109865 | - | see also 13230 line |
| 30287 | PTD004 | NM_013341.2 | 119 | ttcaatgtgttaaccaatagtca | 109863 | - | see also 13230 line |
| 30288 | RAB11A | NM_004663.3 | 340 | agcgatatcgagctataacatca | 109889 | - | see also 3184 line |
| 30289 | RAB11B | NM_004218.1 | 469 | gccttggattccactaacgtaga | 109902 | - | see also 13232 line |
| 30290 | RAB11B | NM_004218.1 | 480 | cactaacgtagaggaagcattca | 109903 | - | see also 13232 line |
| 30291 | RAB11B | NM_004218.1 | 425 | gcgccttcgcagaaaagaacaac | 109900 | - | see also 13232 line |
| 30292 | RAB14 | NM_016322.2 | 432 | agctgcgggagctcttatggtct | 109915 | - | see also 6810 line |
| 30293 | RAB14 | NM_016322.2 | 631 | ttcctcgaagcgagtgcaaaaac | 109924 | - | see also 6810 line |
| 30294 | RAB17 | NM_022449.1 | 1153 | gtcggaggtgttcaatacagtgg | 109933 | - | see also 13234 line |
| 30295 | RAB17 | NM_022449.1 | 751 | ggctcttcggtacgtgaagaacg | 109927 | - | see also 13234 line |
| 30296 | RAB17 | NM_022449.1 | 1149 | aggtgtcggaggtgttcaataca | 109932 | - | see also 13234 line |
| 30297 | RAB18 | NM_021252.3 | 287 | aggtgcacagggtgttatattag | 109938 | - | see also 8145 line |

Figure 46

| | | | | | | | |
|---|---|---|---|---|---|---|---|
| 30298 | RAB18 | NM_021252.3 | 252 | gagaggtttagaacattaactcc | 109936 | - | see also 8145 line |
| 30299 | RAB1A | NM_004161.2 | 424 | aggagcccatggcatcatagttg | 109959 | - | see also 2798 line |
| 30300 | RAB2 | NM_002865.1 | 335 | ttcggtgctcgaatgataactat | 109978 | - | see also 13237 line |
| 30301 | RAB2 | NM_002865.1 | 337 | cggtgctcgaatgataactattg | 109980 | - | see also 13237 line |
| 30302 | RAB2 | NM_002865.1 | 336 | tcggtgctcgaatgataactatt | 109979 | - | see also 13237 line |
| 30303 | RAB20 | NM_017817.1 | 752 | agcgccaagaccggctacaatgt | 109996 | - | see also 13238 line |
| 30304 | RAB20 | NM_017817.1 | 754 | cgccaagaccggctacaatgtgg | 109997 | - | see also 13238 line |
| 30305 | RAB21 | NM_014999.1 | 365 | tgcgctactgcgagaacaagttt | 109999 | - | see also 13239 line |
| 30306 | RAB21 | NM_014999.1 | 496 | ttccatgcattgggtccaattta | 110003 | - | see also 13239 line |
| 30307 | RAB22A | NM_020673.2 | 665 | gactacggcgactcattcatgc | 110035 | - | see also 7914 line |
| 30308 | RAB22A | NM_020673.2 | 703 | cagcgcaaaaacgcgataaaaca | 110040 | - | see also 7914 line |
| 30309 | RAB22A | NM_020673.2 | 493 | gtcggctgcagctataatcgttt | 110026 | - | see also 7914 line |
| 30310 | RAB23 | NM_016277.3 | 1165 | aactaacgcattcaagtagtaac | 110058 | - | see also 13241 line |
| 30311 | RAB23 | NM_016277.3 | 945 | gccgaagtgggagatataccaac | 110051 | - | see also 13241 line |
| 30312 | RAB24 | NM_130781.1 | 647 | gaaccggactgtgacattaggtat | 110065 | - | see also 13242 line |
| 30313 | RAB24 | NM_130781.1 | 648 | accggactgtgacattaggtatt | 110066 | - | see also 13242 line |
| 30314 | RAB27A | NM_004580.3 | 858 | gcctctacggatcagttaagtga | 110086 | - | see also 3119 line |
| 30315 | RAB27A | NM_004580.3 | 860 | ctctacggatcagttaagtgaag | 110087 | - | see also 3119 line |
| 30316 | RAB27B | NM_004163.3 | 568 | gacaaatatggcataccatattt | 110093 | - | see also 13244 line |
| 30317 | RAB27B | NM_004163.3 | 384 | cgccatgggcttcttattaatgt | 110090 | - | see also 13244 line |
| 30318 | RAB28 | NM_004249.1 | 263 | gagtcctggtatatgatatt | 110099 | - | see also 13245 line |
| 30319 | RAB28 | NM_004249.1 | 257 | cacagggagtcctcttggtatat | 110096 | - | see also 13245 line |
| 30320 | RAB2B | NM_032846.2 | 190 | gtccacgacctcacaataggtgt | 110109 | - | see also 9310 line |
| 30321 | RAB2B | NM_032846.2 | 222 | agctcgtatgtcaacattgatg | 110110 | - | see also 9310 line |
| 30322 | RAB30 | NM_014488.3 | 327 | gacgtgcctcgtccgaagattca | 110118 | - | see also 5704 line |
| 30323 | RAB30 | NM_014488.3 | 331 | tgcctcgtccgaagattcactca | 110119 | - | see also 5704 line |
| 30324 | RAB31 | NM_006868.2 | 486 | aagtgcgacctctcagatattag | 110146 | - | see also 4807 line |
| 30325 | RAB31 | NM_006868.2 | 134 | ggcgatacggagctcaaagtgt | 110133 | - | see also 4807 line |
| 30326 | RAB32 | NM_006834.2 | 516 | tccacattgaggcagtcttaaa | 110150 | - | see also 13249 line |
| 30327 | RAB32 | NM_006834.2 | 593 | tgctgtcctcttggctaacaaat | 110151 | - | see also 13249 line |
| 30328 | RAB33A | NM_004794.2 | 827 | gccagaacgtgagtcgatttttc | 110164 | - | see also 13250 line |
| 30329 | RAB33A | NM_004794.2 | 863 | gccgattgaaggcccagaaatcc | 110165 | - | see also 13250 line |
| 30330 | RAB33B | NM_031296.1 | 412 | cgctcgaggcaagcttttcgtcc | 110170 | - | see also 9007 line |
| 30331 | RAB33B | NM_031296.1 | 1014 | cagcccctgataatggaattat | 110195 | - | see also 9007 line |
| 30332 | RAB34 | NM_031934.3 | 948 | tgcattgcatcaacctactattag | 110202 | - | see also 9056 line |
| 30333 | RAB34 | NM_031934.3 | 674 | cgctttgcacggcacaaagact | 110198 | - | see also 9056 line |
| 30334 | RAB35 | NM_006861.4 | 444 | agcggtggcttcacgaaatcaac | 110213 | - | see also 4806 line |

Figure 46

| | | | | | | | | | |
|---|---|---|---|---|---|---|---|---|---|
| 30335 | RAB35 | NM_006861.4 | 710 | aagctcacgaagaacagtaaacg | 110219 | - | see also 4806 line | | |
| 30336 | RAB36 | NM_004914.2 | 463 | caggttttgcaagaatgtttttg | 110224 | - | see also 13254 line | | |
| 30337 | RAB37 | NM_175738.2 | 1045 | ttccagatccgagactatgtaga | 110235 | - | see also 13255 line | | |
| 30338 | RAB37 | NM_175738.2 | 714 | aagcgtcacccatgcttattaca | 110227 | - | see also 13255 line | | |
| 30339 | RAB38 | NM_022337.1 | 495 | cacggtttcgtaggatggtttga | 110241 | - | see also 8331 line | | |
| 30340 | RAB38 | NM_022337.1 | 581 | tgcaaatgagtgtgacctaatgg | 110246 | - | see also 8331 line | | |
| 30341 | RAB39 | NM_017516.1 | 303 | tactaaccgacgatcttttgaac | 110256 | - | see also 13257 line | | |
| 30342 | RAB39 | NM_017516.1 | 307 | aaccgacgatcttttgaacatgt | 110257 | - | see also 13257 line | | |
| 30343 | RAB39B | NM_171998.1 | 496 | ttcagccctaccaaattgtattt | 110267 | - | see also 10029 line | | |
| 30344 | RAB39B | NM_171998.1 | 593 | tgcatacggcatgaagtacattg | 110271 | - | see also 10029 line | | |
| 30345 | RAB3A | NM_002866.3 | 189 | ttcgactacatgttcaagattct | 110277 | - | signal_transduction | protein transporter | - |
| 30346 | RAB3B | NM_002867.2 | 128 | ggcttcagtgacagatggtaaaa | 110278 | - | signal_transduction | protein transporter | - |
| 30347 | RAB3B | NM_002867.2 | 407 | ggccatgggcttcattctgatgt | 110279 | - | see also 30346 line | | |
| 30348 | RAB3C | NM_138453.2 | 459 | tacaagattggtcaactcaaatc | 110289 | - | see also 13258 line | | |
| 30349 | RAB3C | NM_138453.2 | 399 | gtggagccatgggctttatttta | 110287 | - | see also 13258 line | | |
| 30350 | RAB3D | NM_004283.2 | 424 | gaccgtctaccgccatgacaaga | 110294 | - | see also 13259 line | | |
| 30351 | RAB3D | NM_004283.2 | 302 | atgttcaaactgctactgatagg | 110293 | - | see also 13259 line | | |
| 30352 | RAB40B | NM_006822.1 | 352 | gacggcattgatcgatggattaa | 110296 | - | signal_transduction | - | - |
| 30353 | RAB40C | NM_021168.1 | 124 | gtccccgtacgcctacagtaacg | 110297 | - | see also 13260 line | | |
| 30354 | RAB40C | NM_021168.1 | 825 | cgcggagtaactgcaagatctcc | 110300 | - | see also 13260 line | | |
| 30355 | RAB4A | NM_004578.2 | 217 | gacggccatgtccgaaacctacg | 110301 | - | see also 3117 line | | |
| 30356 | RAB4A | NM_004578.2 | 433 | caggtccgtgacgagaagttatt | 110306 | - | see also 3117 line | | |
| 30357 | RAB4B | NM_016154.2 | 1174 | gtcagtgacgcggagttattacc | 110325 | - | see also 13262 line | | |
| 30358 | RAB4B | NM_016154.2 | 1458 | cccgcactatcctcaacaagatt | 110326 | - | see also 13262 line | | |
| 30359 | RAB5A | NM_004162.3 | 478 | aagcctagtgcttcgttttgtga | 110331 | - | see also 2800 line | | |
| 30360 | RAB5A | NM_004162.3 | 733 | gaggcaagcaagtcctaacattg | 110339 | - | see also 2800 line | | |
| 30361 | RAB5B | NM_002868.2 | 444 | atcgtggtttacgacattactaa | 110357 | - | see also 13264 line | | |
| 30362 | RAB5B | NM_002868.2 | 517 | aggccagtcctagcatcgttatt | 110359 | - | see also 13264 line | | |
| 30363 | RAB5C | NM_004583.1 | 635 | ctgcaatgaacgtgaacgaaatc | 110369 | - | see also 13265 line | | |
| 30364 | RAB5C | NM_004583.1 | 636 | tgcaatgaacgtgaacgaaatct | 110370 | - | see also 13265 line | | |
| 30365 | RAB6B | NM_016577.1 | 420 | ctctgattacgaggttcatgtac | 110376 | - | see also 6921 line | | |
| 30366 | RAB6B | NM_016577.1 | 407 | gtcgggaagacgtctctgattac | 110375 | - | see also 6921 line | | |
| 30367 | RAB6B | NM_016577.1 | 338 | atgtccgcaggggggagattttgg | 110374 | - | see also 6921 line | | |
| 30368 | RAB7 | NM_004637.5 | 565 | tccccgagatcctgaaaacttcc | 110387 | - | see also 13266 line | | |
| 30369 | RAB7 | NM_004637.5 | 478 | ctgctgcgttctggtatttgatg | 110383 | - | see also 13266 line | | |
| 30370 | RAB7L1 | NM_003929.1 | 242 | gagcgcttcacctctatgacacg | 110397 | - | see also 13267 line | | |
| 30371 | RAB7L1 | NM_003929.1 | 532 | ggctatgagagtcctcattgaaa | 110402 | - | see also 13267 line | | |

Figure 46

| # | Gene | Accession | Length | Sequence | ID | | Note | Category |
|---|------|-----------|--------|----------|-----|---|------|----------|
| 30372 | RAB9A | NM_004251.3 | 612 | gacaacggcgactatccttatt | 110408 | - | see also 2856 line | |
| 30373 | RAB9A | NM_004251.3 | 245 | ttcacttatgaacagatatgtaa | 110405 | - | see also 2856 line | |
| 30374 | RAB9B | NM_016370.1 | 358 | aaccgttacgttaaccaacaatt | 110411 | - | see also 6842 line | |
| 30375 | RABL4 | NM_006860.2 | 596 | gggagagtcccaatgcttatgt | 110422 | - | see also 13269 line | |
| 30376 | RABL4 | NM_006860.2 | 809 | ttgaaacatccgtgaaagagatg | 110426 | - | see also 13269 line | |
| 30377 | RAC2 | NM_002872.3 | 367 | ctgtcagccagcctccttatga | 110430 | - | see also 13270 line | Rho small monomeric GTPase |
| 30378 | RAC3 | NM_005052.2 | 322 | cccccaaactgacgtctttctga | 110431 | - | see also 13270 line | |
| 30379 | RALA | NM_005402.2 | 771 | tgctaaaacacgagctaatgttg | 110453 | - | see also 3770 line | |
| 30380 | RALA | NM_005402.2 | 484 | gtccagatcgatatcttagatac | 110438 | - | see also 3770 line | |
| 30381 | RALB | NM_002881.1 | 398 | agccattgagataactactttc | 110463 | - | see also 1923 line | |
| 30382 | RALB | NM_002881.1 | 260 | gacgcttcagttcatgtgatgacg | 110458 | - | see also 1923 line | |
| 30383 | RAN | NM_006325.2 | 190 | ttcgtgaaacgtcatttgactgg | 110472 | - | see also 13272 line | |
| 30384 | RANBP17 | NM_022897.2 | 1397 | gtgcagttattcgaccaaaatgc | 110514 | - | see also 8509 line | |
| 30385 | RAP1A | NM_002884.1 | 529 | aagaacggccaaggtttgcact | 110570 | - | see also 13273 line | |
| 30386 | RAP1A | NM_002884.1 | 788 | atgactggtcagacagataaat | 110578 | - | see also 13273 line | |
| 30387 | RAP1B | NM_015646.3 | 229 | tacgatcctacgatagaagattc | 110581 | - | see also 13274 line | |
| 30388 | RAP1B | NM_015646.3 | 656 | aggctcgcaaaaagtcatcatgt | 110582 | - | see also 13274 line | |
| 30389 | RAP2A | NM_021033.3 | 691 | ttccgctaagagtaaaacaatgg | 110587 | - | see also 8070 line | |
| 30390 | RAP2A | NM_021033.3 | 560 | cgcgtgaagcggtatgagaaagt | 110584 | - | see also 8070 line | |
| 30391 | RAP2B | NM_002886.2 | 904 | agctctggtagacgagctattg | 110593 | - | see also 30391 line | RAS small monomeric GTPase |
| 30392 | RAP2B | NM_002886.2 | 552 | accgaccatcgaagacttttac | 110589 | - | see also 30391 line | |
| 30393 | RAP2B | NM_002886.2 | 553 | ccgaccatcgaagactttacc | 110590 | - | see also 30391 line | |
| 30394 | RASD1 | NM_016084.3 | 851 | ctgcgacgtgctgcacaagaagg | 110607 | - | see also 13277 line | |
| 30395 | RASD1 | NM_016084.3 | 490 | tcctcacaggacgtttcatc | 110606 | - | see also 13277 line | |
| 30396 | RASD2 | NM_014310.3 | 363 | aggaacttccacgtaaggtatac | 110609 | - | see also 13278 line | |
| 30397 | RASD2 | NM_014310.3 | 362 | gaggacttccacgtaaggtata | 110608 | - | see also 13278 line | |
| 30398 | REM | NM_014012.4 | 923 | cgctgtggttcgactgtaaat | 110614 | - | see also 13279 line | |
| 30399 | REM | NM_014012.4 | 925 | ctgtggttcgactgtaaattc | 110615 | - | see also 13279 line | |
| 30400 | RERG | NM_032918.1 | 394 | atgatccaccctcgaatcaacc | 110623 | - | see also 9345 line | |
| 30401 | RERG | NM_032918.1 | 830 | cagctccaccacgcatgtcaagc | 110628 | - | see also 9345 line | |
| 30402 | RHEBL1 | NM_144593.1 | 430 | atcattgggtccatggttatgt | 110635 | - | see also 9707 line | |
| 30403 | RHEBL1 | NM_144593.1 | 624 | gtcctggggtcgacatttatgg | 110638 | - | see also 9707 line | |
| 30404 | RHO6 | NM_014470.2 | 310 | aggatctccctactaccgataatg | 110651 | - | see also 13282 line | |
| 30405 | RHO6 | NM_014470.2 | 349 | cagcgactcggatgcagtattac | 110653 | - | see also 13282 line | |
| 30406 | RHO6 | NM_014470.2 | 350 | agcgactcggatgcagtattact | 110654 | - | see also 13282 line | |

Figure 46

| 30407 | RHOBTB1 | NM_014836.3 | 1768 | aacgttctcggacgtgacattta | 110689 | - | see also 13283 line |
|---|---|---|---|---|---|---|---|
| 30408 | RHOBTB1 | NM_014836.3 | 793 | cccgttagcaaggcccataaaga | 110672 | - | see also 13283 line |
| 30409 | RHOBTB1 | NM_014836.3 | 764 | gacctggaagctgttaatcgagc | 110671 | - | see also 13283 line |
| 30410 | RHOBTB2 | NM_015178.1 | 396 | tcccgagacgtggtagatgatgt | 110708 | - | see also 6269 line |
| 30411 | RHOBTB2 | NM_015178.1 | 395 | ctcccgagacgtggtagatgatg | 110707 | - | see also 6269 line |
| 30412 | RIS | NM_016563.2 | 216 | atgtcctcggtgtttggaaaacc | 110721 | - | see also 13285 line |
| 30413 | RIS | NM_016563.2 | 317 | tgccctgaccgtgaagtttctga | 110722 | - | see also 13285 line |
| 30414 | RIT1 | NM_006912.2 | 452 | acggatcgtcgaagtttccatga | 110740 | - | see also 13286 line |
| 30415 | RIT1 | NM_006912.2 | 320 | aagatcaggatccgtattgatga | 110732 | - | see also 13286 line |
| 30416 | RIT2 | NM_002930.1 | 589 | cagttccgccaggtttctacaga | 110758 | - | see also 1971 line |
| 30417 | RRAD | NM_004165.1 | 684 | gcggcgtgcacggcaaacagatg | 110763 | - | see also 13288 line |
| 30418 | RRAD | NM_004165.1 | 822 | agcggcattgcaccacaatgtcc | 110767 | - | see also 13288 line |
| 30419 | RRAGA | NM_006570.3 | 328 | gtcgataatcttcgccaattaca | 110769 | - | see also 13289 line |
| 30420 | RRAGA | NM_006570.3 | 501 | tccgtaacgtggaagttttgatt | 110772 | - | see also 13289 line |
| 30421 | RRAGB | NM_006064.2 | 766 | tagccaacgggacaacatcttcc | 110787 | - | see also 13290 line |
| 30422 | RRAGB | NM_006064.2 | 1052 | aaggcttggtccagcatagttta | 110791 | - | see also 13290 line |
| 30423 | RRAS2 | NM_012250.3 | 803 | atgaacttgtccgggttatcagg | 110825 | - | see also 13291 line |
| 30424 | RRAS2 | NM_012250.3 | 482 | cccggctagatattttggataca | 110814 | - | see also 13291 line |
| 30425 | SARA1 | NM_020150.3 | 439 | ttctcgcctcgtggaatccaaag | 110833 | - | see also 13292 line |
| 30426 | SARA1 | NM_020150.3 | 699 | ggctctcccagtatattgactga | 110838 | - | see also 13292 line |
| 30427 | SARA1 | NM_020150.3 | 212 | ttcttaggtttggataatgcagg | 110830 | - | see also 13292 line |
| 30428 | SARA2 | NM_016103.2 | 634 | atgcccgacccttagaagttttc | 110849 | - | see also 13293 line |
| 30429 | SARA2 | NM_016103.2 | 635 | tgcccgacccttagaagttttca | 110850 | - | see also 13293 line |
| 30430 | 1.Sep | NM_052838.2 | 657 | gggcagtacacgagaaagtcaac | 110861 | - | see also 13294 line |
| 30431 | 1.Sep | NM_052838.2 | 658 | ggcagtacacgagaaagtcaaca | 110862 | - | see also 13294 line |
| 30432 | 10.Sep | NM_144710.2 | 1716 | aggaccgtaagaactccaatttt | 110880 | - | see also 13295 line |
| 30433 | 10.Sep | NM_144710.2 | 940 | gacacttgatctcttaaccatga | 110866 | - | see also 13295 line |
| 30434 | 3.Sep | NM_019106.4 | 899 | agggtcctcggccgaaaaactcc | 110888 | - | see also 13296 line |
| 30435 | 3.Sep | NM_019106.4 | 966 | ccctgcttcgagactttgtcatc | 110890 | - | see also 13296 line |
| 30436 | 3.Sep | NM_019106.4 | 494 | cccattgagaagtacatcaatga | 110884 | - | see also 13296 line |
| 30437 | 6.Sep | NM_015129.4 | 546 | ggctaaagctcacgatcgttagc | 110895 | - | see also 13297 line |
| 30438 | 6.Sep | NM_015129.4 | 832 | ttcgaagagtgagctaacaaagt | 110898 | - | see also 13297 line |
| 30439 | SGNE1 | NM_003020.1 | 322 | gactggagacaacattcctaagg | 110905 | - | see also 13298 line |
| 30440 | SGNE1 | NM_003020.1 | 402 | cagatgatggatgtctagaaaac | 110906 | - | see also 13298 line |
| 30441 | SRPR | NM_003139.2 | 1245 | gccacagcgtcgtgtagacatgc | 110924 | - | see also 2120 line |
| 30442 | SRPR | NM_003139.2 | 1315 | accttctgcggcgttaatggagt | 110925 | - | see also 2120 line |
| 30443 | SUCLG1 | NM_003849.1 | 127 | ttctcggcaacatctctatgttg | 110935 | - | see also 13300 line |

Figure 46

| | | | | | | | | | |
|---|---|---|---|---|---|---|---|---|---|
| 30444 | SUCLG1 | NM_003849.1 | 124 | agcttctcggcaacatctctatg | 110934 | - | see also 13300 line | | |
| 30445 | SUCLG1 | NM_003849.1 | 331 | aacggcttctgtcatttatgttc | 110941 | - | see also 13300 line | | |
| 30446 | TUBA1 | NM_006000.1 | 367 | tgcccgtggtcactataccattg | 110951 | - | see also 13301 line | | |
| 30447 | TUBA1 | NM_006000.1 | 362 | aactatgcccgtggtcactatac | 110949 | - | see also 13301 line | | |
| 30448 | TUBA4 | NM_025019.1 | 445 | gagtggctttctgttaactatgg | 110955 | - | see also 13302 line | | |
| 30449 | TUBA8 | NM_018943.1 | 725 | gacattgagcgccctacctatac | 110960 | - | see also 13303 line | | |
| 30450 | TUBA8 | NM_018943.1 | 184 | tgctcaagctagcaagatcaacg | 110958 | - | see also 13303 line | | |
| 30451 | TUBA8 | NM_018943.1 | 991 | cccgagacatggcaagtacatgg | 110962 | - | see also 13303 line | | |
| 30452 | TUBB1 | NM_030773.1 | 154 | aacgaagcctacggtaggaaata | 110965 | - | see also 13304 line | | |
| 30453 | TUBB1 | NM_030773.1 | 476 | acccggaccggatcatgaattcc | 110974 | - | see also 13304 line | | |
| 30454 | TUBB4 | NM_006086.1 | 248 | atctcttcaggcctgacaatttc | 110982 | - | - | structural constituent of cytoskeleton | - |
| 30455 | TUBD1 | NM_016261.1 | 443 | caggattaggagctttcgttaca | 110991 | - | see also 13306 line | | |
| 30456 | TUBD1 | NM_016261.1 | 971 | ttcctcctcttagtaaaatgtct | 111019 | - | see also 13306 line | | |
| 30457 | TUBE1 | NM_016262.3 | 223 | agcacgagcagtcttgattgata | 111030 | - | see also 13307 line | | |
| 30458 | TUBE1 | NM_016262.3 | 225 | cacgagcagtcttgattgatatg | 111031 | - | see also 13307 line | | |
| 30459 | TUBE1 | NM_016262.3 | 572 | gtcataacctcaccttataatag | 111044 | - | see also 13307 line | | |
| 30460 | TUBG1 | NM_001070.3 | 507 | ctgggttcctacctcttagaacg | 111061 | - | see also 13308 line | | |
| 30461 | TUBG1 | NM_001070.3 | 552 | aagctggtgcagacatactcagt | 111062 | - | see also 13308 line | | |
| 30462 | TUBG2 | NM_016437.1 | 1419 | ttcctgccagcagtttgacaagc | 111066 | - | see also 13309 line | | |
| 30463 | TUBG2 | NM_016437.1 | 1515 | caggtctagggaggttgttcagg | 111067 | - | see also 13309 line | | |
| 30464 | VTS58635 | NM_033315.2 | 562 | acgacatctgctgctttgacagc | 111069 | - | see also 13310 line | | |
| 30465 | ZNF179 | NM_007148.2 | 744 | gcctcgccaggggcatatggatg | 111071 | - | see also 13311 line | | |
| 30466 | ZNF179 | NM_007148.2 | 1520 | gccaccaagcgcatattctctgc | 111074 | - | see also 13311 line | | |
| 30467 | CNGA1 | NM_000087.1 | 1700 | atcgaagaacggccaatattaaa | 111126 | - | see also 66 line | | |
| 30468 | CNGA1 | NM_000087.1 | 637 | tacgtatcagacatagtctattt | 111092 | - | see also 66 line | | |
| 30469 | PDE10A | NM_006661.1 | 1008 | tactcgacgtatcaaaaacatat | 111166 | - | see also 4709 line | | |
| 30470 | PDE10A | NM_006661.1 | 2170 | gaccggatcactaaaccttaata | 111195 | - | see also 4709 line | | |
| 30471 | PDE10A | NM_006661.1 | 442 | caggtaccaagatacgaatatgc | 111142 | - | see also 4709 line | | |
| 30472 | PDE5A | NM_001083.2 | 1608 | aaccgaaatgacgaacagtttct | 111247 | - | see also 677 line | | |
| 30473 | PDE5A | NM_001083.2 | 816 | atccgcttagaatggaacaaagg | 111223 | - | see also 677 line | | |
| 30474 | C5orf3 | NM_018691.1 | 1196 | aaccgaaatgctacactatctca | 111288 | - | see also 7564 line | | |
| 30475 | C5orf3 | NM_018691.1 | 1935 | atgtccttaacccattaatcact | 111302 | - | see also 7564 line | | |
| 30476 | LOC56851 | NM_020154.1 | 330 | atcccgttcgagtggatatcact | 111313 | - | see also 7772 line | | |
| 30477 | LOC56851 | NM_020154.1 | 332 | cccgttcgagtggatatcacttc | 111314 | - | see also 7772 line | | |
| 30478 | BIRC1 | NM_004536.1 | 712 | tacgacataagggtgaagaatct | 111337 | - | see also 13317 line | | |
| 30479 | BIRC1 | NM_004536.1 | 627 | cacgagactccccatagaagacc | 111335 | - | see also 13317 line | | |

Figure 46

| 30480 | DHRS3 | NM_004753.3 | 915 | tgcatgccctcgttatcttgaaa | 111356 | - | see also 13318 line |
|---|---|---|---|---|---|---|---|
| 30481 | DHRS3 | NM_004753.3 | 910 | gacaatgcatgccctcgttatct | 111355 | - | see also 13318 line |
| 30482 | LTB4R | NM_181657.1 | 1986 | gccgcctgtcactatgtctgc | 111362 | - | nucleotide binding |
| 30483 | LTB4R | NM_181657.1 | 1747 | ctcactaggtgtagagttcatct | 111360 | - | see also 30482 line |
| 30484 | LTB4R | NM_181657.1 | 1744 | cccctcactaggtgtagagttca | 111359 | - | see also 30482 line |
| 30485 | NOX4 | NM_016931.2 | 264 | ctcgccaacgaagggttaaaca | 111364 | - | see also 13319 line |
| 30486 | NOX4 | NM_016931.2 | 1533 | ggcattggagtaactccatttgc | 111382 | - | see also 13319 line |
| 30487 | SLC22A4 | NM_003059.2 | 1346 | ccctgcccaggcgttatatcata | 111402 | - | see also 13320 line |
| 30488 | SLC22A4 | NM_003059.2 | 1167 | gactcggaatattgccataatga | 111396 | - | see also 13320 line |
| 30489 | ABT1 | NM_013375.2 | 568 | gcgttgagaccgacttctatcttc | 111415 | - | see also 5413 line |
| 30490 | AIP | NM_003977.1 | 151 | gggatccaaaaacgtgtgataca | 111421 | - | see also 13322 line |
| 30491 | AIP | NM_003977.1 | 140 | tccgggaggacggatccaaaaaa | 111417 | - | see also 13322 line |
| 30492 | BRDT | NM_001726.1 | 1696 | ttggcggagtagttcacataata | 111472 | - | see also 13323 line |
| 30493 | BRDT | NM_001726.1 | 1432 | aagtaccttccgtaagctaaat | 111464 | - | see also 13323 line |
| 30494 | CRSP2 | NM_004229.2 | 405 | ttcgttcgattattagctttagt | 111521 | - | see also 13324 line |
| 30495 | CRSP2 | NM_004229.2 | 525 | ctggcctggctagctagagatgc | 111528 | - | see also 13324 line |
| 30496 | CRSP7 | NM_004831.2 | 1196 | ctcgagactatacggttaacttg | 111677 | - | see also 13325 line |
| 30497 | CRSP7 | NM_004831.2 | 1721 | atgactgaacgcagtgcatatcg | 111681 | - | see also 13325 line |
| 30498 | CRSP8 | NM_004269.2 | 24 | tggcggacgtgataaatgtcagt | 111682 | - | see also 13326 line |
| 30499 | CRSP8 | NM_004269.2 | 644 | ttcattgatcgaacaatagtaaa | 111697 | - | see also 13326 line |
| 30500 | CRSP9 | NM_004270.3 | 412 | tacacgtgcatcatcttataaat | 111717 | - | see also 13327 line |
| 30501 | CRSP9 | NM_004270.3 | 722 | tgccttgtgtcctaattgatg | 111726 | - | see also 13327 line |
| 30502 | DTX1 | NM_004416.1 | 851 | cgcatggacggcctacgatatgg | 111730 | - | see also 13328 line |
| 30503 | DTX1 | NM_004416.1 | 1455 | ctcccgttgaagaacttgaatgg | 111731 | - | see also 13328 line |
| 30504 | GCN5L2 | NM_021078.1 | 1619 | cgcgcatgcctaaggagtatatc | 111742 | - | see also 13329 line |
| 30505 | GCN5L2 | NM_021078.1 | 780 | gacgatgttgagctctcaaaga | 111737 | - | see also 13329 line |
| 30506 | GCN5L2 | NM_021078.1 | 981 | aagccttctccggtccattttca | 111740 | - | see also 13329 line |
| 30507 | GTF2A1 | NM_015859.1 | 1202 | agctccattgtcttacaagttg | 111770 | - | see also 13330 line |
| 30508 | GTF2A1 | NM_015859.1 | 1338 | gagccctcaatagtgaagatga | 111772 | - | see also 13330 line |
| 30509 | HCF-2 | NM_013320.1 | 163 | gtctacaacacgctacgaatca | 111781 | - | see also 13331 line |
| 30510 | HCF-2 | NM_013320.1 | 975 | tgcaatcggcactcgattgtatt | 111808 | - | see also 13331 line |
| 30511 | HSGT1 | NM_007265.1 | 1922 | cccagactaccgataacaattca | 111894 | - | see also 5063 line |
| 30512 | HSGT1 | NM_007265.1 | 1926 | gactaccgataacaattcagatg | 111896 | - | see also 5063 line |
| 30513 | HTATIP2 | NM_006410.3 | 277 | caccttcgacgaggaagcttata | 111909 | - | see also 4496 line |
| 30514 | HTATIP2 | NM_006410.3 | 278 | accttcgacgaggaagcttataa | 111910 | - | see also 4496 line |
| 30515 | MYCBP | NM_012333.2 | 235 | gcctagaactggccgaaatgaaa | 111923 | - | see also 5294 line |
| 30516 | MYCBP | NM_012333.2 | 157 | aacctaacagtgctttggatttt | 111922 | - | see also 5294 line |

Figure 46

| | | | | | | | | | |
|---|---|---|---|---|---|---|---|---|---|
| 30517 | NCOA1 | NM_003743.3 | 1751 | ctcccgttggcatgacaagtagt | 111971 | - | see also 2559 line | | |
| 30518 | NCOA1 | NM_003743.3 | 1342 | atgtcaattccccgagtaaatcc | 111957 | - | see also 2559 line | | |
| 30519 | NCOA2 | NM_006540.1 | 595 | cagtatctaaggtataaccaaga | 112050 | - | see also 4581 line | | |
| 30520 | NCOA2 | NM_006540.1 | 735 | gaggcggaacagccataccttca | 112054 | - | see also 4581 line | | |
| 30521 | NCOA3 | NM_006534.2 | 948 | gtgtggcacgccgcattactaca | 112152 | - | see also 4567 line | | |
| 30522 | NCOA3 | NM_006534.2 | 591 | atcgagacggaaacattgtattt | 112145 | - | see also 4567 line | | |
| 30523 | NCOA4 | NM_005437.1 | 419 | ctcgttattgggccagttcaatt | 112246 | - | see also 3791 line | | |
| 30524 | NCOA4 | NM_005437.1 | 636 | ttgatggctcatgctagttcagc | 112256 | - | see also 3791 line | | |
| 30525 | NRIP1 | NM_003489.1 | 2269 | aaccgataggtatgattgataga | 112315 | - | see also 13337 line | | |
| 30526 | NRIP1 | NM_003489.1 | 1248 | atgtcaagccatcttaatggtca | 112289 | - | see also 13337 line | | |
| 30527 | PCBD | NM_000281.1 | 297 | gccagcttcatcgaacaagtagc | 112355 | - | see also 245 line | | |
| 30528 | PCBD | NM_000281.1 | 271 | gcctttcagaacgggacataaac | 112353 | - | see also 245 line | | |
| 30529 | PMF1 | NM_007221.1 | 246 | gacatctatccgggaggaaatct | 112360 | - | see also 13339 line | | |
| 30530 | PMF1 | NM_007221.1 | 148 | ggcagctaccagagattcactga | 112358 | - | see also 13339 line | | |
| 30531 | POU2AF1 | NM_006235.1 | 829 | gacaccttacaccgagtatgtgc | 112362 | - | transcription_regulator | transcription co-activator | leukemia |
| 30532 | POU2AF1 | NM_006235.1 | 871 | cccctactcagctgacatgtatg | 112363 | - | see also 30531 line | | |
| 30533 | POU2AF1 | NM_006235.1 | 872 | ccctactcagctgacatgtatgt | 112364 | - | see also 30531 line | | |
| 30534 | RNF14 | NM_004290.3 | 334 | gccctggcaagtatttacgatgg | 112367 | - | see also 2874 line | | |
| 30535 | RNF14 | NM_004290.3 | 333 | ggccctggcaagtatttacgatg | 112366 | - | see also 2874 line | | |
| 30536 | RNF14 | NM_004290.3 | 1388 | aagcggatgaggctaataaaaga | 112387 | - | see also 2874 line | | |
| 30537 | SMARCD2 | NM_003077.2 | 826 | agcgaaagcttcggatctacatt | 112397 | - | see also 2072 line | | |
| 30538 | SMARCD3 | NM_003078.2 | 1185 | gacggcgtgctatgacattgacg | 112414 | - | see also 2074 line | | |
| 30539 | SMARCD3 | NM_003078.2 | 583 | aagcggaagctgcgactctatat | 112407 | - | see also 2074 line | | |
| 30540 | SNW1 | NM_012245.2 | 172 | ggctggatacctcggttattaga | 112421 | - | see also 13343 line | | |
| 30541 | SNW1 | NM_012245.2 | 28 | atggcgctcaccagctttttacc | 112418 | - | see also 13343 line | | |
| 30542 | TAF7 | NM_005642.2 | 1131 | atctggaaccacggaattactct | 112458 | - | see also 3915 line | | |
| 30543 | TAF7 | NM_005642.2 | 1271 | tagtactcggtgggaaataattg | 112460 | - | see also 3915 line | | |
| 30544 | TAZ | NM_015472.3 | 1374 | ccccctcttcaatgatgtagagt | 112485 | - | see also 13345 line | | |
| 30545 | TAZ | NM_015472.3 | 875 | cacccgcagggctcatgagtatg | 112478 | - | see also 13345 line | | |
| 30546 | TCERG1 | NM_006706.2 | 2979 | atcctcgatgtattaagttctcc | 112552 | - | see also 4731 line | | |
| 30547 | TCERG1 | NM_006706.2 | 2782 | tcgtaggaccctccgaaaagatc | 112545 | - | see also 4731 line | | |
| 30548 | TGFB1I1 | NM_015927.3 | 532 | gacttccgcgttcaaaaccatct | 112567 | - | see also 13347 line | | |
| 30549 | TGFB1I1 | NM_015927.3 | 528 | ctctgacttccgcgttcaaaacc | 112566 | - | see also 13347 line | | |
| 30550 | TNRC11 | NM_005120.1 | 5717 | accgtcctgtgcgcttaccaatg | 112653 | - | see also 3592 line | | |
| 30551 | TNRC11 | NM_005120.1 | 1451 | aagttcgctggtctttcgataaa | 112584 | - | see also 3592 line | | |
| 30552 | TRAP95 | NM_005481.2 | 1912 | caccgacgtcgacattgacaagg | 112683 | - | see also 13349 line | | |

Figure 46

| | | | | | | | | |
|---|---|---|---|---|---|---|---|---|
| 30553 | TRAP95 | NM_005481.2 | 1158 | ttctcaaatgcggatcctatcg | 112680 | - | | see also 13349 line |
| 30554 | TRAP95 | NM_005481.2 | 859 | gtcgcccgtgcagttctacaagg | 112676 | - | | see also 13349 line |
| 30555 | TRIM32 | NM_012210.2 | 592 | aactcgtctgcgggaacttatgg | 112698 | - | | see also 13350 line |
| 30556 | TRIM32 | NM_012210.2 | 1298 | gaccgtggtaactatcgtataca | 112710 | - | | see also 13350 line |
| 30557 | TRIM32 | NM_012210.2 | 1303 | tggtaactatcgtatacaagtct | 112712 | - | | see also 13350 line |
| 30558 | TRIP11 | NM_004239.1 | 1621 | tgcgtatcgaagtttagaaaaa | 112757 | - | | see also 13351 line |
| 30559 | TRIP11 | NM_004239.1 | 5100 | gagagattgcgtaatcatctttt | 112827 | - | | see also 13351 line |
| 30560 | TRIP11 | NM_004239.1 | 1111 | gacgacatcgaagaagaattaagt | 112746 | - | | see also 13351 line |
| 30561 | TRIP4 | NM_016213.2 | 993 | gacacgcctctcgacttctaag | 112873 | - | | see also 6724 line |
| 30562 | TRIP4 | NM_016213.2 | 137 | atcattcagtacgtttgtcaat | 112856 | - | | see also 6724 line |
| 30563 | UTF1 | NM_003577.1 | 361 | cgccgctacaagttccttaaaga | 112893 | - | | see also 13353 line |
| 30564 | VGLL1 | NM_016267.2 | 788 | tcccggctcagttcactataaga | 112905 | - | | see also 13354 line |
| 30565 | VGLL1 | NM_016267.2 | 281 | cagcagcgtagtggatgaacact | 112895 | - | | see also 13354 line |
| 30566 | CALR | NM_004343.2 | 1099 | acgagacgtggggcgtaacaaag | 112928 | - | | see also 13355 line |
| 30567 | CALR | NM_004343.2 | 276 | caggatgcacgctttatgctct | 112917 | - | | see also 13355 line |
| 30568 | CREG | NM_003851.1 | 530 | ttgcaaagcattcgttattcatt | 112933 | - | | see also 13356 line |
| 30569 | CREG | NM_003851.1 | 554 | gacacctgagatgaaaacctgg | 112935 | - | | see also 13356 line |
| 30570 | HD | NM_002111.4 | 8600 | atgcccactgcgtgaacattca | 113102 | - | | see also 13357 line |
| 30571 | HD | NM_002111.4 | 5088 | tccgtccgtagacatgcttta | 113041 | - | | see also 13357 line |
| 30572 | HSBP1 | NM_001537.1 | 263 | tacctgccacgcaaaagagttga | 113120 | - | transcription_regulator, transcription co-repressor | |
| 30573 | ID3 | NM_002167.2 | 690 | ttgtcatccaacgacaacaaagg | 113121 | - | transcription_regulator, transcription co-repressor | |
| 30574 | L3MBTL2 | NM_031488.3 | 1604 | gccgctccatcgagactcttaa | 113139 | - | | see also 9039 line |
| 30575 | L3MBTL2 | NM_031488.3 | 2090 | tcgcccgacaaggcttcaagtcc | 113144 | - | | see also 9039 line |
| 30576 | L3MBTL2 | NM_031488.3 | 1608 | ctccatcgagactcttaacatg | 113141 | - | | see also 9039 line |
| 30577 | NAB2 | NM_005967.1 | 1209 | cccgagagagcacctacttgtcc | 113154 | - | | see also 13359 line |
| 30578 | NAB2 | NM_005967.1 | 982 | acggagcgttggcacatctttg | 113150 | - | | see also 13359 line |
| 30579 | PAWR | NM_002583.1 | 979 | tagaagtgcgttcctagatata | 113160 | - | | see also 13360 line |
| 30580 | PAWR | NM_002583.1 | 982 | aagtgggttcctagatataaca | 113161 | - | | see also 13360 line |
| 30581 | PBXIP1 | NM_020524.2 | 1907 | ctctctgttaagaacatacttgg | 113172 | - | | see also 7886 line |
| 30582 | PBXIP1 | NM_020524.2 | 2016 | cgcctccgcttccggatttgt | 113173 | - | | see also 7886 line |
| 30583 | PLRG1 | NM_002669.1 | 1223 | atcttccgtcataatgctatt | 113219 | - | | see also 1745 line |
| 30584 | PLRG1 | NM_002669.1 | 136 | atcaagcttcgtaatgagtatgg | 113183 | - | | see also 1745 line |
| 30585 | RAI | NM_006663.1 | 1120 | ccctacccaccaagaaacagtacc | 113228 | - | apoptosis, transcription_regulator | |
| 30586 | RAI | NM_006663.1 | 1756 | ggcagacgtcgagcagtatgg | 113229 | - | | see also 30585 line |

Figure 46

| | | | | | | | |
|---|---|---|---|---|---|---|---|
| 30587 | RAI | NM_006663.1 | 1977 | agcctcaaaggagtaaagtctag | 113230 | - | see also 30585 line |
| 30588 | RNF12 | NM_016120.2 | 1996 | tgcatcgatcgctggttatctga | 113284 | - | see also 6674 line |
| 30589 | RNF12 | NM_016120.2 | 743 | acccacgatctgaatcaacatct | 113246 | - | see also 6674 line |
| 30590 | RNF12 | NM_016120.2 | 1290 | aggaagtttaggcgtacattt | 113262 | - | see also 6674 line |
| 30591 | RYBP | NM_012234.3 | 474 | aaggaaattagtcctagtgttac | 113291 | - | see also 13364 line |
| 30592 | RYBP | NM_012234.3 | 210 | agcccgaccaggccaaaaagaca | 113288 | - | see also 13364 line |
| 30593 | SAP18 | NM_005870.3 | 424 | gaccctgcagtcgcagaagttcc | 113304 | - | see also 4085 line |
| 30594 | SAP18 | NM_005870.3 | 504 | cagggcgcatgagaccatattaa | 113305 | - | see also 4085 line |
| 30595 | SAP30 | NM_003864.1 | 790 | tgccacttaggtcattccagt | 113315 | - | see also 13366 line |
| 30596 | SAP30 | NM_003864.1 | 553 | agcgcaaggcatcttacatatg | 113307 | - | see also 13366 line |
| 30597 | SIAH2 | NM_005067.4 | 1208 | cagcgactgccttgttttcgaca | 113322 | - | see also 3535 line |
| 30598 | SIAH2 | NM_005067.4 | 895 | tgcacgcccacaagagcattacc | 113318 | - | see also 3535 line |
| 30599 | SUFU | NM_016169.2 | 1312 | tcctgcatggacggcactttaca | 113337 | - | see also 13368 line |
| 30600 | SUFU | NM_016169.2 | 1446 | ctgttgaccgaagagtttgtaga | 113346 | - | see also 13368 line |
| 30601 | TTRAP | NM_016614.2 | 718 | agggcatgctgccggaacgaatga | 113363 | - | see also 6951 line |
| 30602 | TTRAP | NM_016614.2 | 727 | tgcggaacgaatgaatcagttaa | 113366 | - | see also 6951 line |
| 30603 | TZFP | NM_014383.1 | 281 | tccggccagcactcgtgatact | 113380 | - | see also 13370 line |
| 30604 | TZFP | NM_014383.1 | 1090 | aggatcccacgtcctaaatgc | 113387 | - | see also 13370 line |
| 30605 | YAF2 | NM_005748.2 | 375 | tggagatcgacagtcattatta | 113390 | - | see also 13371 line |
| 30606 | YAF2 | NM_005748.2 | 221 | tgcctcctacacagtcaaagaaa | 113388 | - | see also 13371 line |
| 30607 | ZFPM2 | NM_012082.2 | 1661 | aggggctacttgtttgagtgt | 113421 | - | see also 5112 line |
| 30608 | ZFPM2 | NM_012082.2 | 1050 | cactgctgattccgtgatcaact | 113410 | - | see also 5112 line |
| 30609 | NMI | NM_004688.1 | 559 | ctggaagttccttatgagatac | 113481 | - | see also 13373 line |
| 30610 | NMI | NM_004688.1 | 556 | gagctgaaagttccttatgaga | 113480 | - | see also 13373 line |
| 30611 | PCAF | NM_003884.3 | 1197 | ttcctaaaccgcatcaactattg | 113513 | - | see also 13374 line |
| 30612 | PCAF | NM_003884.3 | 2525 | tggagttcgacagattcctatag | 113555 | - | see also 13374 line |
| 30613 | PIR | NM_003662.1 | 567 | ggcctacaactgtgggttaattt | 113572 | - | see also 13375 line |
| 30614 | PIR | NM_003662.1 | 317 | cagacccgagttaaaaaatctgg | 113565 | - | see also 13375 line |
| 30615 | PIR | NM_003662.1 | 645 | atccctaaaccagtaaggatgg | 113576 | - | see also 13375 line |
| 30616 | HDAC11 | NM_024827.1 | 249 | cacgaggcgctatccttaatgagc | 113584 | - | see also 13376 line |
| 30617 | HDAC11 | NM_024827.1 | 245 | tgcacacgaggcgtatcttaat | 113582 | - | see also 13376 line |
| 30618 | HDAC3 | NM_003883.2 | 1277 | gaggcacccaatgagttctatga | 113634 | - | see also 2640 line |
| 30619 | HDAC3 | NM_003883.2 | 847 | tcgattgggctgctttaacctca | 113616 | - | see also 2640 line |
| 30620 | HDAC4 | NM_006037.2 | 1449 | cggagtgtcgaccctcctataacc | 113640 | - | see also 13378 line |
| 30621 | HDAC4 | NM_006037.2 | 3739 | gacgcctcggaagcatgtgtttc | 113643 | - | see also 13378 line |
| 30622 | HDAC5 | NM_005474.3 | 1221 | ggggtgtcgtgtgttgtaacagc | 113652 | - | see also 3811 line |
| 30623 | HDAC5 | NM_005474.3 | 3045 | caggcgtggggttacaatgtgaac | 113659 | - | see also 3811 line |

Figure 46

| 30624 | HDAC7A | NM_015401.1 | 2176 | ctcgtgagctaaagaatggtttc | 113673 | - | see also 13380 line |
|---|---|---|---|---|---|---|---|
| 30625 | HDAC7A | NM_015401.1 | 2171 | ggcttctcgtgagctaaagaatg | 113672 | - | see also 13380 line |
| 30626 | HDAC7A | NM_015401.1 | 1361 | aactcacgtccaggtgatcaaga | 113665 | - | see also 13380 line |
| 30627 | HDAC8 | NM_018486.1 | 531 | acgattgcgacggaaatttgagc | 113697 | - | see also 13381 line |
| 30628 | HDAC8 | NM_018486.1 | 85 | gtcccggtttatatctatagtcc | 113686 | - | see also 13381 line |
| 30629 | HDAC9 | NM_014707.1 | 1066 | cagcaacgcattctaattcatga | 113738 | - | see also 5898 line |
| 30630 | HDAC9 | NM_014707.1 | 906 | ttcattcaagaagcgaatgtttg | 113731 | - | see also 5898 line |
| 30631 | MYBBP1A | NM_014520.1 | 240 | atgccctgaagcgtctaatcacg | 113787 | - | see also 13383 line |
| 30632 | MYBBP1A | NM_014520.1 | 720 | tggtgggatccgtgaacctattc | 113795 | - | see also 13383 line |
| 30633 | NFKBIA | NM_020529.1 | 641 | ggccacacgtgtctacacttagc | 113811 | - | see also 13384 line |
| 30634 | NFKBIA | NM_020529.1 | 510 | tccgagactttcgaggaaatacc | 113810 | - | see also 13384 line |
| 30635 | NFKBIA | NM_020529.1 | 905 | cagctgacactagaaaaccttca | 113817 | - | see also 13384 line |
| 30636 | RNF19 | NM_015435.3 | 1434 | gtcggaatcgctttaatagctgg | 113850 | - | see also 6415 line |
| 30637 | RNF19 | NM_015435.3 | 1141 | tacgtttgagaactatacgttct | 113841 | - | see also 6415 line |
| 30638 | RNF19 | NM_015435.3 | 783 | gtggattgcttacgacaatattt | 113825 | - | see also 6415 line |
| 30639 | VHL | NM_000551.2 | 820 | caggagcgcattgcacatcaacg | 113900 | - | see also 13386 line |
| 30640 | VHL | NM_000551.2 | 715 | agcctagtcaagcctgagaatta | 113897 | - | see also 13386 line |
| 30641 | GSN | NM_000177.3 | 285 | cccgtgcccaccaacctttatgg | 113906 | - | see also 151 line |
| 30642 | GSN | NM_000177.3 | 738 | aacagcaatcggtatgaaagact | 113909 | - | see also 151 line |
| 30643 | CAPG | NM_001747.1 | 790 | ggccgcagctctgtataaggtct | 113927 | - | see also 13388 line |
| 30644 | CAPG | NM_001747.1 | 939 | aggggcgaaaagcgaatgagaag | 113931 | - | see also 13388 line |
| 30645 | CAPPA3 | NM_033328.2 | 392 | gggcgactaccgatttttgacc | 113940 | - | see also 9406 line |
| 30646 | CAPPA3 | NM_033328.2 | 811 | aagctcaactggctctaagtttt | 113960 | - | see also 9406 line |
| 30647 | CAPPA3 | NM_033328.2 | 522 | ctgtgcgccttaaaactgtatgt | 113944 | - | see also 9406 line |
| 30648 | CAPZA1 | NM_006135.1 | 562 | gtggttggcgtgcttaagattca | 113992 | - | see also 13390 line |
| 30649 | CAPZA1 | NM_006135.1 | 103 | gacgttcggctactacttaataa | 113978 | - | see also 13390 line |
| 30650 | CAPZA2 | NM_006136.1 | 762 | gacaatgtcggacactactttca | 114011 | - | see also 13391 line |
| 30651 | CAPZA2 | NM_006136.1 | 804 | gccagttacacgcactaagattg | 114013 | - | see also 13391 line |
| 30652 | CAPZA2 | NM_006136.1 | 55 | aaggtgcgtatagcagcaaaatt | 114002 | - | see also 13391 line |
| 30653 | CAPZB | NM_004930.1 | 483 | cggtcgcaccgcccattacaagt | 114029 | - | see also 3429 line |
| 30654 | CAPZB | NM_004930.1 | 675 | aagtacgctgaacgagatctact | 114030 | - | see also 3429 line |
| 30655 | EVL | NM_016337.1 | 1105 | ggcaaagagccccgaagctaaga | 114042 | - | see also 13393 line |
| 30656 | EVL | NM_016337.1 | 208 | cagagtcgttggagtcaagttgc | 114034 | - | see also 13393 line |
| 30657 | HRIHFB2122 | NM_007032.3 | 400 | tacgcggtgcagcgcaactatgg | 114047 | - | see also 4921 line |
| 30658 | HRIHFB2122 | NM_007032.3 | 433 | cacaccaaggatgctgtctatac | 114048 | - | see also 4921 line |

Figure 46

| | | | | | | | | | |
|---|---|---|---|---|---|---|---|---|---|
| 30659 | ARP3BETA | NM_020445.1 | 235 | atcgataaacctacatatgctac | 114059 | - | see also 13395 line | | |
| 30660 | ARP3BETA | NM_020445.1 | 231 | agccatcgataaacctacatatg | 114058 | - | see also 13395 line | | |
| 30661 | FLNA | NM_001456.1 | 4967 | aaccatgacggcacgtatacagt | 114101 | - | see also 13396 line | | |
| 30662 | FLNA | NM_001456.1 | 4104 | accgtggcgatggcatgtacaaa | 114091 | - | see also 13396 line | | |
| 30663 | FLNA | NM_001456.1 | 824 | gccagcaagcccgttaccaatgc | 114065 | - | see also 13396 line | | |
| 30664 | FSCN3 | NM_020369.1 | 1459 | cccgaccgcattcatctactacc | 114131 | - | see also 7853 line | | |
| 30665 | FSCN3 | NM_020369.1 | 1479 | accctgccgaccgggtatctacc | 114132 | - | see also 7853 line | | |
| 30666 | MACF1 | NM_012090.3 | 15894 | gccgagcacgaggtagaactaac | 114462 | - | see also 13398 line | | |
| 30667 | MACF1 | NM_012090.3 | 9394 | ctgccgggtccgagagatgttct | 114314 | - | see also 13398 line | | |
| 30668 | MARCKS | NM_002356.4 | 807 | cagcaacgagaccccgaaaaaaa | 114618 | - | - | calmodulin binding | - |
| 30669 | PLS1 | NM_002670.1 | 1878 | tcggtgcccggatatatgcatta | 114668 | - | see also 13399 line | | |
| 30670 | PLS1 | NM_002670.1 | 1908 | acctcgtagaagtgaaaccaaag | 114671 | - | see also 13399 line | | |
| 30671 | SPTA1 | NM_003126.1 | 277 | gtgttgactcggtatcaaagttt | 114677 | - | see also 2099 line | | |
| 30672 | SPTA1 | NM_003126.1 | 6803 | aggcgatgaagcgtcaactaacc | 114802 | - | see also 2099 line | | |
| 30673 | AVIL | NM_006576.2 | 706 | cccttggccgacgctccattatc | 114833 | - | see also 13401 line | | |
| 30674 | AVIL | NM_006576.2 | 709 | ttggccgacgctccattatcaag | 114834 | - | see also 13401 line | | |
| 30675 | CFL1 | NM_005507.1 | 298 | gccctctatgatgcaacctatga | 114863 | - | see also 13402 line | | |
| 30676 | CFL1 | NM_005507.1 | 438 | agggatcaagcatgaattgcaag | 114864 | - | see also 13402 line | | |
| 30677 | SDCBP | NM_005625.1 | 259 | tcctatccctcacgatggaaatc | 114865 | - | - | syndecan binding | melanoma |
| 30678 | ACTN4 | NM_004924.3 | 593 | cccgtataagaacgtcaatgtgc | 114870 | - | see also 3427 line | | |
| 30679 | ACTN4 | NM_004924.3 | 332 | atcaggggagcggttacctaagc | 114866 | - | see also 3427 line | | |
| 30680 | ACTN4 | NM_004924.3 | 2737 | ccggtgccctcgactacaagtcc | 114881 | - | see also 3427 line | | |
| 30681 | EPB49 | NM_001978.1 | 1289 | tgcaccagggaacgtctaaatct | 114893 | - | see also 13404 line | | |
| 30682 | EPB49 | NM_001978.1 | 1288 | ttgcaccagggaacgtctaaatc | 114892 | - | see also 13404 line | | |
| 30683 | FSCN1 | NM_003088.1 | 1006 | gaccgcgacaccaaaaagtgtgc | 114897 | - | see also 13405 line | | |
| 30684 | FSCN1 | NM_003088.1 | 1004 | tcgaccgcgacaccaaaaagtgt | 114896 | - | see also 13405 line | | |
| 30685 | FSCN2 | NM_012418.1 | 1217 | ctggcggctatcagcgatttgt | 114910 | - | see also 13406 line | | |
| 30686 | FSCN2 | NM_012418.1 | 1219 | ggcggctatcagcgattttgtcg | 114911 | - | see also 13406 line | | |
| 30687 | FSCN2 | NM_012418.1 | 1185 | ggcgctacgtgtgcatgaagaag | 114908 | - | see also 13406 line | | |
| 30688 | VIL1 | NM_007127.1 | 2266 | cccaaagtggacgtgttcaatgc | 114930 | - | see also 4958 line | | |
| 30689 | VIL1 | NM_007127.1 | 2270 | aagtggacgtgttcaatgctaac | 114931 | - | see also 4958 line | | |
| 30690 | DSTN | NM_006870.2 | 245 | gagatgttggtgtaaccataact | 114935 | - | see also 13408 line | | |
| 30691 | DSTN | NM_006870.2 | 119 | atgacatgaaagttcgtaaatgc | 114933 | - | see also 13408 line | | |
| 30692 | WASF1 | NM_003931.1 | 451 | aagaacgtgtggaccgtttatct | 114946 | - | see also 2692 line | | |
| 30693 | WASF1 | NM_003931.1 | 459 | gtggaccgtttatctgttagtgt | 114949 | - | see also 2692 line | | |

Figure 46

| 30694 | WASF3 | NM_006646.3 | 905 | tcggacgttacggattactctta | 114991 | - | see also 13410 line |
|---|---|---|---|---|---|---|---|
| 30695 | WASF3 | NM_006646.3 | 1167 | tcccagcgcagataattgagtat | 114995 | - | see also 13410 line |
| 30696 | ABLIM1 | NM_002313.4 | 1097 | atcccgactgtaagcaatctacg | 115036 | - | see also 1485 line |
| 30697 | ABLIM1 | NM_002313.4 | 2106 | taccgacttcgctcagtataaca | 115069 | - | see also 1485 line |
| 30698 | ABLIM1 | NM_002313.4 | 1202 | caggtcatactatctatgcaaaa | 115042 | - | see also 1485 line |
| 30699 | ABLIM2 | NM_032432.2 | 1001 | gtctccgagccgtgtgatttatg | 115086 | - | see also 13412 line |
| 30700 | ABLIM2 | NM_032432.2 | 818 | cccttcctgcgcgctatgtgtca | 115082 | - | see also 13412 line |
| 30701 | ABLIM2 | NM_032432.2 | 313 | tgcggcagggcgagtacatctgc | 115075 | - | see also 13412 line |
| 30702 | ACTN1 | NM_001102.2 | 198 | ttctcagcaaaccaacgattaca | 115090 | - | see also 13413 line |
| 30703 | ACTN1 | NM_001102.2 | 1919 | tccagacctaccacgtcaatatg | 115103 | - | see also 13413 line |
| 30704 | ACTN1 | NM_001102.2 | 870 | aactgcccgaccggatgagaaag | 115098 | - | see also 13413 line |
| 30705 | ACTN2 | NM_001103.1 | 447 | atgcggttccacaaaattgctaa | 115108 | - | see also 13414 line |
| 30706 | ACTN2 | NM_001103.1 | 2809 | tggattacgctgcgttctcttcc | 115143 | - | see also 13414 line |
| 30707 | ACTN3 | NM_001104.1 | 1321 | gactacgattcggctttgctaca | 115148 | - | see also 13415 line |
| 30708 | ACTN3 | NM_001104.1 | 320 | tccacaaaatcgccaacgttaac | 115146 | - | see also 13415 line |
| 30709 | ADD1 | NM_001119.3 | 1326 | ctcatgcggatgctcgataatct | 115182 | - | see also 707 line |
| 30710 | ADD2 | NM_001617.1 | 507 | cccgcaggggcagccttactttg | 115195 | - | see also 13417 line |
| 30711 | ANLN | NM_018685.2 | 1182 | aacggggtatcgaaaccaattg | 115240 | - | see also 7555 line |
| 30712 | ANLN | NM_018685.2 | 1177 | ctgaaaacgggggtatcgaaacc | 115239 | - | see also 7555 line |
| 30713 | ARPC1A | NM_006409.2 | 991 | ttcgtctccaagttagatattcc | 115315 | - | see also 4495 line |
| 30714 | ARPC1A | NM_006409.2 | 988 | accttcgtctccaagttagatat | 115314 | - | see also 4495 line |
| 30715 | ARPC1A | NM_006409.2 | 233 | atcacgaagtgcacatctataag | 115308 | - | see also 4495 line |
| 30716 | BCL7B | NM_001707.2 | 234 | gacacgtccctgaggatatttaa | 115324 | - | see also 13419 line |
| 30717 | BCL7B | NM_001707.2 | 401 | ttccgtgtctgacgtctatcagc | 115327 | - | see also 13419 line |
| 30718 | BPAG1 | NM_001723.3 | 2557 | accctacgatcagagcttaatgt | 115398 | - | see also 1145 line |
| 30719 | C6orf209 | NM_018368.2 | 147 | tggtgatcggctggtgcatattc | 116007 | - | see also 13421 line |
| 30720 | C6orf209 | NM_018368.2 | 160 | gtgcatattcggcctcttactac | 116008 | - | see also 13421 line |
| 30721 | CALD1 | NM_004342.4 | 590 | ttcgacccaacaataacagatgc | 116075 | - | see also 13423 line |
| 30722 | CALD1 | NM_004342.4 | 1584 | aaggtgtacgcaacatcaagagt | 116094 | - | see also 13423 line |
| 30723 | CEACAM1 | NM_001712.2 | 1303 | atgctgaacgtaaactataatgc | 116109 | | see also 13424 line |
| 30724 | CEACAM1 | NM_001712.2 | 1238 | aggatgctgggacgtattggtgt | 116107 | - | see also 13424 line |
| 30725 | CEACAM5 | NM_004363.1 | 2092 | ttggctactggccgcaataattc | 116130 | - | see also 13425 line |
| 30726 | CEACAM5 | NM_004363.1 | 1984 | cagtattcttggcgtatcaatgg | 116125 | - | see also 13425 line |

Figure 46

| | | | | | | | |
|---|---|---|---|---|---|---|---|
| 30727 | CFL2 | NM_021914.5 | 264 | ttctgtttaagcgatgacaaaag | 116138 | - | see also 8196 line |
| 30728 | CFL2 | NM_021914.5 | 406 | acgatgccacatacgaaacaaaa | 116142 | - | see also 8196 line |
| 30729 | CFL2 | NM_021914.5 | 410 | tgccacatacgaaacaaaagagt | 116143 | - | see also 8196 line |
| 30730 | CGN | NM_020770.1 | 3362 | ctgcagtctaccaatcgaaaact | 116174 | - | see also 13427 line |
| 30731 | CGN | NM_020770.1 | 1234 | gggtgagcttacccgaaaagtgg | 116162 | - | see also 13427 line |
| 30732 | CLMN | NM_024734.2 | 328 | atcctctcgcatcgtattttc | 116184 | - | see also 13428 line |
| 30733 | CLMN | NM_024734.2 | 329 | tcctcgtcgcatcgtattttcg | 116185 | - | see also 13428 line |
| 30734 | CNN1 | NM_001299.3 | 292 | gccgtcgcatggcaacaacttc | 116242 | - | see also 13429 line |
| 30735 | CNN1 | NM_001299.3 | 344 | ttgcgaattcatcaataagctgc | 116246 | - | see also 13429 line |
| 30736 | CNN2 | NM_004368.1 | 187 | ctgaaggatggaactatcttatg | 116254 | - | see also 13430 line |
| 30737 | CNN2 | NM_004368.1 | 446 | tggacattggcgtcaagtactg | 116255 | - | see also 13430 line |
| 30738 | CNN3 | NM_001839.1 | 959 | ttcgaaatcagtgatagtgatt | 116272 | - | see also 13431 line |
| 30739 | CNN3 | NM_001839.1 | 793 | cggtggacaactcgacaatttcc | 116268 | - | see also 13431 line |
| 30740 | CORO1A | NM_007074.1 | 364 | ctggtgcccgcacaatgacaacg | 116283 | - | see also 13432 line |
| 30741 | CORO1A | NM_007074.1 | 158 | aaggccgaccagtgctatgaaga | 116280 | - | see also 13432 line |
| 30742 | CORO1B | NM_020441.1 | 334 | tcctcacaacgacgaagtcatag | 116286 | - | see also 13433 line |
| 30743 | CORO1C | NM_014325.2 | 355 | gactggtgcccacataacgatca | 116298 | - | see also 5620 line |
| 30744 | CORO1C | NM_014325.2 | 283 | ctgcacaagactggtcgaattga | 116294 | - | see also 5620 line |
| 30745 | CORO2A | NM_003389.2 | 1196 | gactgagtaccgctcctataacc | 116334 | - | see also 13435 line |
| 30746 | CORO2A | NM_003389.2 | 867 | gaccgcaagattcgggttattga | 116332 | - | see also 13435 line |
| 30747 | CORO2A | NM_003389.2 | 1666 | atggctttgacgtttcgaatgc | 116339 | - | see also 13435 line |
| 30748 | CORO2B | NM_006091.1 | 1179 | gagatcccgtgctgatgtctttg | 116349 | - | see also 13436 line |
| 30749 | CORO2B | NM_006091.1 | 790 | gcccctgatcgaagaggaaattg | 116346 | - | see also 13436 line |
| 30750 | COTL1 | NM_021149.2 | 216 | gccgtcatctgggtgactttaaa | 116355 | - | see also 13437 line |
| 30751 | COTL1 | NM_021149.2 | 217 | ccgtcatctgggtgactttaaa | 116356 | - | see also 13437 line |
| 30752 | DAAM1 | NM_014992.1 | 146 | tggacgaggtatttcattcatct | 116362 | - | see also 13438 line |
| 30753 | DAAM1 | NM_014992.1 | 203 | cacgtatcggctgcgaaatgata | 116365 | - | see also 13438 line |
| 30754 | DAAM2 | NM_015345.2 | 520 | caagagtctgacgcgtttgatga | 116442 | - | see also 13439 line |
| 30755 | DAAM2 | NM_015345.2 | 3361 | cgggcaataaaccggctaaatta | 116481 | - | see also 13439 line |
| 30756 | DBN1 | NM_004395.2 | 2017 | ctccagagatcgacatcacatgc | 116493 | - | see also 13440 line |
| 30757 | DBN1 | NM_004395.2 | 365 | agctgctctgccaaaatacgtgc | 116484 | - | see also 13440 line |
| 30758 | DIAPH1 | NM_005219.2 | 609 | gagactgctgggagtttacgatag | 116499 | - | see also 13441 line |
| 30759 | DIAPH1 | NM_005219.2 | 1251 | ctcttggtccgaaatgactatga | 116510 | - | see also 13441 line |
| 30760 | DIAPH2 | NM_006729.2 | 929 | tccggtcagctgggttaacaact | 116581 | - | see also 4745 line |
| 30761 | DIAPH2 | NM_006729.2 | 853 | atgaattacgatcgggtatatca | 116576 | - | see also 4745 line |
| 30762 | DMD | NM_000109.2 | 10429 | gagacttgccaaggtactaaaa | 116898 | - | see also 70 line |
| 30763 | DMD | NM_000109.2 | 9599 | gtctttagctgacctgaataatg | 116881 | - | see also 70 line |

Figure 46

| | | | | | | | |
|---|---|---|---|---|---|---|---|
| 30764 | DNASE1 | NM_005223.2 | 1552 | gccgagatcgacgctctatga | 116925 | - | see also 13444 line |
| 30765 | DNASE1 | NM_005223.2 | 1185 | caccctcgtcagctacattgtgc | 116919 | - | see also 13444 line |
| 30766 | ENC1 | NM_003633.1 | 883 | accaagcgtacgaactatcttg | 116938 | - | see also 2451 line |
| 30767 | EPB41 | NM_004437.1 | 1762 | ttcgagcgtacagcaagtaaacg | 116996 | - | see also 13446 line |
| 30768 | EPB41 | NM_004437.1 | 1670 | tcttggctaggatccaaattt | 116993 | - | see also 13446 line |
| 30769 | EPB41L1 | NM_012156.2 | 2330 | gagtctccgctattggataacacc | 117027 | - | see also 5152 line |
| 30770 | EPB41L2 | NM_001431.1 | 1343 | cagactgcgaatcaatcgtttg | 117054 | - | see also 1000 line |
| 30771 | EPB41L2 | NM_001431.1 | 1348 | tgcgaatcaatcgtttgcttgg | 117056 | - | see also 1000 line |
| 30772 | EPB41L3 | NM_012307.2 | 1195 | atcgagctgccaagcgttatgg | 117132 | - | see also 5269 line |
| 30773 | EPB41L3 | NM_012307.2 | 2328 | gacactgccgtaacgaatgaatg | 117155 | - | see also 5269 line |
| 30774 | FHOD1 | NM_013241.1 | 2858 | accgccgtgtcgcaataggttc | 117180 | - | see also 13450 line |
| 30775 | FHOD1 | NM_013241.1 | 2855 | tgcaacgcgtgtctgcaatagg | 117179 | - | see also 13450 line |
| 30776 | FLII | NM_002018.2 | 1206 | tgccgctgagtcgtaccaacatcg | 117193 | - | see also 13451 line |
| 30777 | FLII | NM_002018.2 | 2362 | gacacgcgctgcgtgtacattct | 117201 | - | see also 13451 line |
| 30778 | FLNB | NM_001457.1 | 6867 | gggtctgcggtgtatcttatat | 117330 | - | see also 13452 line |
| 30779 | FLNB | NM_001457.1 | 6834 | aaggccgagattacattcgatga | 117326 | - | see also 13452 line |
| 30780 | FMNL1 | NM_005892.3 | 898 | gcctacggccatcatgaactac | 117346 | - | see also 13453 line |
| 30781 | FMNL1 | NM_005892.3 | 2119 | tccgaatgccactcttgaactgg | 117351 | - | see also 13453 line |
| 30782 | FXYD5 | NM_014164.3 | 651 | cccggttatgccggaatcgttgc | 117371 | - | see also 13454 line |
| 30783 | FXYD5 | NM_014164.3 | 235 | ttcagcagactcaactatcatgg | 117368 | - | see also 13454 line |
| 30784 | GC | NM_000583.2 | 1311 | tgctaagggccctctactaaaga | 117401 | - | see also 13455 line |
| 30785 | GC | NM_000583.2 | 1175 | aagtcatgataagtatacattt | 117395 | - | see also 13455 line |
| 30786 | GMFB | NM_004124.2 | 290 | gacaacctcgcttcattgtgat | 117415 | - | see also 13456 line |
| 30787 | GMFB | NM_004124.2 | 440 | cagctgaactaaccaaggtattt | 117421 | - | see also 13456 line |
| 30788 | GMFG | NM_004877.1 | 308 | atgatgtatgcaggggagtaaaaa | 117427 | - | see also 13457 line |
| 30789 | GMFG | NM_004877.1 | 71 | ttccgaaaagacagacagacaatgc | 117424 | - | see also 13457 line |
| 30790 | HDAC6 | NM_006044.2 | 823 | atccggaaggtccttatcgtaga | 117444 | - | see also 13458 line |
| 30791 | HDAC6 | NM_006044.2 | 176 | gtgtcacttcgaagcgaaatatt | 117428 | - | see also 13458 line |
| 30792 | HIP-55 | NM_014063.4 | 785 | ccgtgcaccgagggagagatttc | 117484 | - | - |
| 30793 | HIP-55 | NM_014063.4 | 518 | atgccgtgtcgagattaaaagg | 117483 | - | see also 30792 line |
| 30794 | HIP-55 | NM_014063.4 | 514 | accaatgccgtgtctgagattaa | 117482 | - | see also 30792 line |
| 30795 | HIP1 | NM_005338.4 | 566 | aagtgacgtgaacaacttttcc | 117487 | - | see also 3727 line |
| 30796 | HIP1 | NM_005338.4 | 2426 | aactgccacggccagaatagagg | 117502 | - | see also 3727 line |
| 30797 | HPCAL1 | NM_002149.2 | 814 | agcgcacagacaagatcttcagg | 117510 | - | - |
| 30798 | IPP | NM_005897.1 | 1707 | ttggactcagttgaagtttataa | 117511 | - | see also 13460 line |
| 30799 | IQGAP2 | NM_006633.1 | 2356 | cacaggcggcaaacgttcattga | 117581 | - | see also 13461 line |
| 30800 | IQGAP2 | NM_006633.1 | 2360 | ggcggcaaacgttcattgataat | 117584 | - | see also 13461 line |

Figure 46

| | | | | | | | |
|---|---|---|---|---|---|---|---|
| 30801 | KIAA0843 | NM_014945.1 | 1389 | gccggagctctcataccatagc | - | 117677 | see also 6134 line |
| 30802 | KLHL1 | NM_020866.1 | 2617 | ttcccggctactggattatgtag | - | 117730 | see also 8009 line |
| 30803 | KLHL1 | NM_020866.1 | 2618 | tcccggctactggattatgtaga | - | 117731 | see also 8009 line |
| 30804 | KLHL2 | NM_007246.2 | 1092 | tgccaacagagcagcgtatatta | - | 117772 | see also 5045 line |
| 30805 | KLHL2 | NM_007246.2 | 560 | ctgaggatgctaattgattatgt | - | 117750 | see also 5045 line |
| 30806 | KLHL3 | NM_017415.1 | 1377 | aagaccaacgagtggttcttgt | - | 117818 | see also 13465 line |
| 30807 | KLHL3 | NM_017415.1 | 1292 | cgcagcggtgtctcaatgacttgc | - | 117817 | see also 13465 line |
| 30808 | KLHL4 | NM_019117.3 | 2116 | gaggatatgacggacatacttat | - | 117869 | see also 7711 line |
| 30809 | KLHL4 | NM_019117.3 | 2122 | atgacggacatactatttgaac | - | 117871 | see also 7711 line |
| 30810 | KPTN | NM_007059.1 | 363 | cagcccctcctgaacattact | - | 117890 | see also 4942 line |
| 30811 | LCP1 | NM_002298.2 | 1797 | aaggaccgaagattagtacaag | - | 117931 | see also 1474 line |
| 30812 | LCP1 | NM_002298.2 | 1550 | ttgtaactacgcggtagaattgg | - | 117923 | see also 1474 line |
| 30813 | LSP1 | NM_002339.1 | 719 | ccctaaacgctcctagagaag | - | 117936 | see also 13469 line |
| 30814 | LSP1 | NM_002339.1 | 1072 | gccacgggcatgggaagtatga | - | 117939 | see also 13469 line |
| 30815 | LSP1 | NM_002339.1 | 1074 | caccgggcatgggaagtatgaga | - | 117940 | see also 13469 line |
| 30816 | MEFV | NM_000243.1 | 2155 | ccccgacccgcctgctaataaag | - | 117958 | see also 13470 line |
| 30817 | MEFV | NM_000243.1 | 2154 | ccccgaccccgcctgctaataaa | - | 117957 | see also 13470 line |
| 30818 | MEFV | NM_000243.1 | 2156 | ccccgaccccgcctgctaataaagg | - | 117959 | see also 13470 line |
| 30819 | MGC35136 | NM_152427.1 | 680 | gcctctgagtctgcatccattaa | - | 117968 | see also 13471 line |
| 30820 | MGC35136 | NM_152427.1 | 684 | ctgagtctgcatccattaagagc | - | 117969 | see also 13471 line |
| 30821 | MTSS1 | NM_014751.2 | 2667 | tccgaagggcgtgaaactgaag | - | 117998 | see also 13472 line |
| 30822 | MTSS1 | NM_014751.2 | 1623 | ttccagactacgctcattattac | - | 117990 | see also 13472 line |
| 30823 | MYBPC1 | NM_002465.1 | 2984 | atcgaaccagtgccaccattact | - | 118089 | see also 1581 line |
| 30824 | MYBPC1 | NM_002465.1 | 1087 | ttcgtaagagagcctccaattat | - | 118030 | see also 1581 line |
| 30825 | MYBPC2 | NM_004533.1 | 2157 | gtgtcttcgccgtcaatgctata | - | 118111 | see also 13474 line |
| 30826 | MYBPC2 | NM_004533.1 | 2992 | ctgtactgtgtccgaccttatcg | - | 118118 | see also 13474 line |
| 30827 | MYBPC3 | NM_000256.2 | 1787 | gcccgacagccgcataaaggtgt | - | 118135 | see also 13475 line |
| 30828 | MYBPC3 | NM_000256.2 | 868 | gtggtcgggcgatcagtgatagc | - | 118128 | see also 13475 line |
| 30829 | MYRIP | NM_015460.1 | 1233 | ccgactacgtggccaaacctaag | - | 118161 | see also 13476 line |
| 30830 | MYRIP | NM_015460.1 | 1891 | caggctgtacgagttagcaatga | - | 118166 | see also 13476 line |
| 30831 | NCALD | NM_032041.1 | 376 | atcatcgcctttgagtgtaacttc | - | 118187 | see also 9066 line |
| 30832 | NCALD | NM_032041.1 | 196 | gagatccaggaaatggttataaagg | - | 118180 | see also 9066 line |
| 30833 | NEB | NM_004543.2 | 15420 | cagtccggagagtcaacaatgt | - | 118489 | see also 13478 line |
| 30834 | NEB | NM_004543.2 | 4027 | gtctgacgtcgaaaaagttaaa | - | 118281 | see also 13478 line |
| 30835 | NEB | NM_004543.2 | 3134 | caggcacgttgattataagcaca | - | 118254 | see also 13478 line |

Figure 46

| 30836 | NEBL | NM_006393.1 | 3098 | tccgagatttaccctagcttttc | 118684 | - | see also 13479 line | | |
|---|---|---|---|---|---|---|---|---|---|
| 30837 | NEBL | NM_006393.1 | 2441 | cagctgagtgcggtaaaatataa | 118668 | - | see also 13479 line | | |
| 30838 | NEBL | NM_006393.1 | 3273 | accgagccatgtacgattacagt | 118687 | - | see also 13479 line | | |
| 30839 | PARVA | NM_018222.2 | 1157 | atctacactacgagtgttgtaca | 118700 | - | see also 13480 line | | |
| 30840 | PARVA | NM_018222.2 | 1160 | tacactacgagtgttgtacaacc | 118701 | - | see also 13480 line | | |
| 30841 | PARVB | NM_013327.2 | 418 | gaggtgacacagtccgaaatagg | 118703 | - | see also 13481 line | | |
| 30842 | PARVB | NM_013327.2 | 511 | cggtggagcgtggactcaattca | 118706 | - | see also 13481 line | | |
| 30843 | PARVG | NM_022141.3 | 989 | aggacgtctttgatgaattattt | 118720 | - | see also 13482 line | | |
| 30844 | PARVG | NM_022141.3 | 981 | gcctccaaaggacgtctttgatg | 118718 | - | see also 13482 line | | |
| 30845 | PFN2 | NM_002628.2 | 134 | cagagcattacgccaatagaaat | 118726 | - | see also 1699 line | | |
| 30846 | PFN2 | NM_002628.2 | 393 | aagcatatgaactcgctttatac | 118741 | - | see also 1699 line | | |
| 30847 | PIP | NM_002652.1 | 127 | aacactcggaagatcataataaa | 118747 | - | see also 13483 line | | |
| 30848 | PIP | NM_002652.1 | 439 | aaccggttttatactattgaaat | 118761 | - | see also 13483 line | | |
| 30849 | PIP | NM_002652.1 | 302 | gcctatgtgacgacaatccaaaa | 118756 | - | see also 13483 line | | |
| 30850 | PLEC1 | NM_000445.1 | 1814 | tgcaccagtccatcgaagaattc | 118773 | - | see also 13484 line | | |
| 30851 | PLEC1 | NM_000445.1 | 12440 | agctgatggagcgttgtatcact | 118794 | - | see also 13484 line | | |
| 30852 | PLS3 | NM_005032.3 | 1326 | tcctcacgtaaaccatctctatg | 118832 | - | see also 3513 line | | |
| 30853 | PSG3 | NM_021016.1 | 1343 | atcttcctggccttaatccatta | 118851 | - | - | defense/immunity protein | - |
| 30854 | RDX | NM_002906.2 | 43 | atcaacgtaagagtaactacaat | 118854 | - | see also 13486 line | | |
| 30855 | RDX | NM_002906.2 | 38 | aaccaatcaacgtaagagtaact | 118853 | - | see also 13486 line | | |
| 30856 | RDX | NM_002906.2 | 32 | tgccgaaaccaatcaacgtaaga | 118852 | - | see also 13486 line | | |
| 30857 | SCIN | NM_033128.1 | 394 | cacggggctgccaaacaaatttt | 118875 | - | see also 13487 line | | |
| 30858 | SCIN | NM_033128.1 | 1384 | tacggctgctctaacaaaactgg | 118893 | - | see also 13487 line | | |
| 30859 | SEC24C | NM_004922.2 | 1291 | cccgatacatccgatgtacatcc | 118907 | - | see also 3422 line | | |
| 30860 | SEC24C | NM_004922.2 | 2635 | agcgtcggctccgcatccataat | 118940 | - | see also 3422 line | | |
| 30861 | SEC24C | NM_004922.2 | 1290 | ccccgatacatccgatgtacatc | 118906 | - | see also 3422 line | | |
| 30862 | SMTN | NM_006932.3 | 515 | gcgaagcgctggtgagtatgagg | 118958 | - | see also 13489 line | | |
| 30863 | SMTN | NM_006932.3 | 1208 | gcgggatcgtgtccacaagttca | 118959 | - | see also 13489 line | | |
| 30864 | SNTA1 | NM_003098.2 | 784 | acgtctcaccgtatttcaagaac | 118974 | - | see also 13490 line | | |
| 30865 | SNTA1 | NM_003098.2 | 790 | caccgtatttcaagaactctact | 118975 | - | see also 13490 line | | |
| 30866 | SNTB1 | NM_021021.2 | 1646 | tgccgtttgaccatacattatga | 118990 | - | see also 13491 line | | |
| 30867 | SNTB1 | NM_021021.2 | 1162 | tgccatccattccaacgttaatg | 118979 | - | see also 13491 line | | |
| 30868 | SNTB2 | NM_006750.2 | 1571 | tccgaagccgattgtatttgtgt | 119027 | - | see also 4765 line | | |
| 30869 | SNTB2 | NM_006750.2 | 1458 | ttgtaccgctacccctttgaaag | 119021 | - | see also 4765 line | | |
| 30870 | SNTG1 | NM_018967.1 | 1605 | tgctggaccgacggaaacagtgc | 119053 | - | see also 7613 line | | |
| 30871 | SNTG1 | NM_018967.1 | 989 | ttcctcaaactcccattgaatga | 119042 | - | see also 7613 line | | |

Figure 46

| 30872 | SNTG2 | NM_018968.1 | 198 | gcgcggacgaaaaccactattgc | 119065 | - | see also 13494 line |
|---|---|---|---|---|---|---|---|
| 30873 | SNTG2 | NM_018968.1 | 829 | aagccggaacggaaaaattaagg | 119081 | - | see also 13494 line |
| 30874 | SORBS1 | NM_006434.1 | 1930 | ctccgcgatggagatatcgttga | 119111 | - | see also 13495 line |
| 30875 | SORBS1 | NM_006434.1 | 180 | aacccggcaacacgaaagcaaac | 119082 | - | see also 13495 line |
| 30876 | SORBS1 | NM_006434.1 | 406 | gcctatccctccacaacgattgt | 119087 | - | see also 13495 line |
| 30877 | SPTAN1 | NM_003127.1 | 1800 | ggcccagctagccgattctttcc | 119149 | - | see also 13496 line |
| 30878 | SPTAN1 | NM_003127.1 | 1166 | cacggctcaatgattcatacagg | 119139 | - | see also 13496 line |
| 30879 | SPTB | NM_000347.3 | 2999 | gtggatcacggacaagacaaagg | 119242 | - | see also 289 line |
| 30880 | SPTBN1 | NM_003128.1 | 5509 | cacgatgttacaagaacgattcc | 119315 | - | see also 2101 line |
| 30881 | SPTBN1 | NM_003128.1 | 2677 | ggcggaagagatcgccaattaca | 119292 | - | see also 2101 line |
| 30882 | SPTBN1 | NM_003128.1 | 2454 | tccgtgagaggatcatttacatc | 119285 | - | see also 2101 line |
| 30883 | SPTBN2 | NM_006946.1 | 5892 | ctccgcggatctagtcatcaaga | 119389 | - | see also 13499 line |
| 30884 | SPTBN2 | NM_006946.1 | 703 | ttcaacgccatcgtgcataaaca | 119364 | - | see also 13499 line |
| 30885 | SPTBN2 | NM_006946.1 | 5889 | gtcctccgcggatctagtcatca | 119388 | - | see also 13499 line |
| 30886 | SPTBN4 | NM_025213.1 | 1666 | agccgaaggctactacgatatcc | 380329 | - | see also 13500 line |
| 30887 | SPTBN4 | NM_025213.1 | 1224 | aggctttcacggcctattgcacg | 380325 | - | see also 13500 line |
| 30888 | SPTBN5 | NM_016642.1 | 1020 | gccagatgagcgctctatcatga | 119399 | - | see also 13501 line |
| 30889 | SPTBN5 | NM_016642.1 | 6073 | cgcacggcggtgcgtgactatgc | 119413 | - | see also 13501 line |
| 30890 | SVIL | NM_003174.2 | 1708 | atgaacgctcgctatcaaactca | 119436 | - | see also 2155 line |
| 30891 | SVIL | NM_003174.2 | 601 | ccccacatcggccgatcaaatga | 119427 | - | see also 2155 line |
| 30892 | TAGLN | NM_003186.2 | 107 | gccgcgaagtgcagtccaaaatc | 119489 | - | see also 13503 line |
| 30893 | TAGLN | NM_003186.2 | 108 | ccgcgaagtgcagtccaaaatcg | 119490 | - | see also 13503 line |
| 30894 | TLN1 | NM_006289.2 | 993 | agcccgttctctcaagacttacg | 119503 | - | see also 4379 line |
| 30895 | TLN1 | NM_006289.2 | 3702 | tggccagcgcgatgtggataatg | 119526 | - | see also 4379 line |
| 30896 | TLN2 | NM_015059.1 | 82 | tacgatgcgtgtcgagtcattcg | 119566 | - | see also 13505 line |
| 30897 | TLN2 | NM_015059.1 | 3135 | agcttgtggtccgatggaaatcg | 119602 | - | see also 13505 line |
| 30898 | TMOD1 | NM_003275.1 | 552 | tgcccgacgaagaaccaaattca | 119666 | - | see also 13506 line |
| 30899 | TMOD1 | NM_003275.1 | 551 | gtgcccgacgaagaaccaaattc | 119665 | - | see also 13506 line |
| 30900 | TMOD2 | NM_014548.2 | 931 | gaccttgacaagtctaaacatag | 119689 | - | see also 5750 line |
| 30901 | TMOD2 | NM_014548.2 | 545 | gcctctgacaccgaactctatga | 119683 | - | see also 5750 line |
| 30902 | TMOD2 | NM_014548.2 | 544 | tgcctctgacaccgaactctatg | 119682 | - | see also 5750 line |
| 30903 | TMOD3 | NM_014547.3 | 1208 | gagcagctaatgctataacaaaa | 119717 | - | see also 13508 line |
| 30904 | TMOD3 | NM_014547.3 | 1042 | ctgattgatgcgttaagagataa | 119712 | - | see also 13508 line |
| 30905 | TMOD4 | NM_013353.1 | 1061 | atgacccgaaacaatgaactacg | 119734 | - | see also 5406 line |
| 30906 | TMOD4 | NM_013353.1 | 631 | gagggtccgaagcaatgacaagg | 119725 | - | see also 5406 line |
| 30907 | TNNI3 | NM_000363.3 | 212 | ctccgcctcgagaaaattgcagc | 119742 | - | see also 13512 line |
| 30908 | TNNI3 | NM_000363.3 | 186 | ccgcacgccaagaaaaaatctaa | 119740 | - | see also 13512 line |

EP 1 752 536 A1

Figure 46

| ID | Gene | Accession | Num | Sequence | Num2 | | Note | Function |
|---|---|---|---|---|---|---|---|---|
| 30909 | TPM1 | NM_000366.4 | 913 | gacgagcgtacgctcagaaact | 119757 | - | see also 13513 line | |
| 30910 | TPM1 | NM_000366.4 | 967 | cacgctctcaacgatatgacttc | 119761 | - | see also 13513 line | structural constituent of muscle |
| 30911 | TPM2 | NM_003289.1 | 279 | aggatgagtggaaaagtattct | 119762 | - | - | |
| 30912 | UTRN | NM_007124.1 | 5119 | gtccggcatcaagccctatttt | 119871 | - | see also 13514 line | |
| 30913 | UTRN | NM_007124.1 | 1706 | gtgttcgacgtctgctattttg | 119800 | - | see also 13514 line | |
| 30914 | UTRN | NM_007124.1 | 3334 | gagatcgctactcaaaaagtag | 119836 | - | see also 13514 line | |
| 30915 | VASP | NM_003370.1 | 450 | atcgtccgggtgtcaagtataa | 120005 | - | see also 13515 line | |
| 30916 | VASP | NM_003370.1 | 455 | ccggggtgtcaagtataaccagg | 120007 | - | see also 13515 line | |
| 30917 | VCL | NM_003373.2 | 2111 | gtctcggctcgtgtatcttact | 120054 | - | see also 13516 line | |
| 30918 | VCL | NM_003373.2 | 2113 | ctcggctcgtgtatcttactta | 120055 | - | see also 13516 line | |
| 30919 | VCL | NM_003373.2 | 3042 | agcgaatcccaaccataagcacc | 120071 | - | see also 13516 line | |
| 30920 | VILL | NM_015873.1 | 564 | ggcgggagggataaatcgattca | 120085 | - | see also 13517 line | |
| 30921 | VILL | NM_015873.1 | 1321 | tccagccacgactgtttgagtgc | 120098 | - | see also 13517 line | |
| 30922 | WASF2 | NM_006990.1 | 214 | gtcgaccgactacaggttaaagt | 120105 | - | receptor_acitivity, signal transduction, gpcr | actin binding |
| 30923 | WASL | NM_003941.2 | 1034 | ttcgatatgtggaatctcaga | 120122 | - | see also 13518 line | |
| 30924 | WASL | NM_003941.2 | 475 | tgcagcagatcggaactgtatgt | 120108 | - | see also 13518 line | |
| 30925 | WASL | NM_003941.2 | 1650 | tacgacagggtatccaactaaaa | 120126 | - | see also 13518 line | |
| 30926 | WASPIP | NM_003387.3 | 264 | caccaatactggacaaacctaaa | 120131 | - | see also 2286 line | |
| 30927 | WDR1 | NM_005112.3 | 906 | gtgggattacggcaattagttgg | 120156 | - | see also 3585 line | |
| 30928 | WDR1 | NM_005112.3 | 275 | ggcgaccctaagggcaacaattt | 120143 | - | see also 3585 line | |
| 30929 | DCX | NM_000555.2 | 1185 | ttcgctatgctcaggatgatttt | 120202 | - | see also 13521 line | |
| 30930 | DCX | NM_000555.2 | 706 | gcctcaggsagtgcgttacatt | 120190 | - | see also 13521 line | |
| 30931 | HOOK3 | NM_032410.2 | 1242 | aggcggcaggttaaactcttaga | 120229 | - | see also 13522 line | |
| 30932 | HOOK3 | NM_032410.2 | 1241 | aaggcggcaggttaaactcttag | 120228 | - | see also 13522 line | |
| 30933 | MAPRE1 | NM_012325.1 | 65 | atggcagtgaacgtatctcaac | 120264 | - | see also 13523 line | |
| 30934 | MAPRE1 | NM_012325.1 | 185 | ggggctgcgtattgtcagtttat | 120270 | - | see also 13523 line | |
| 30935 | MAPRE2 | NM_014268.1 | 364 | agccggctattgccaattcatgg | 120298 | - | see also 5573 line | |
| 30936 | MAPRE2 | NM_014268.1 | 494 | atgaaacgttgataaggtaattcc | 120302 | - | see also 5573 line | |
| 30937 | MAPRE3 | NM_012326.2 | 238 | cacctcaactataccaagataga | 120311 | - | see also 5278 line | |
| 30938 | MAPRE3 | NM_012326.2 | 815 | aggaaacgtgacttctacttcagc | 120315 | - | see also 5278 line | |
| 30939 | TUBGCP5 | NM_052903.1 | 1590 | cacgttatatagcgtatcagaaa | 120352 | - | see also 13525 line | |
| 30940 | TUBGCP5 | NM_052903.1 | 2476 | cccgttgacattgttataagttt | 120376 | - | see also 13525 line | |
| 30941 | TUBGCP6 | NM_020461.2 | 533 | ttccgtacagcggctatgattgc | 120396 | - | see also 13526 line | |
| 30942 | TUBGCP6 | NM_020461.2 | 1746 | cgcttattggcgcgagttcatgattc | 120411 | - | see also 13526 line | |

Figure 46

| 30943 | UNC84B | NM_015374.1 | 893 | ctgacgtatggtgcttggtattt | 120439 | - | see also 6401 line |
|---|---|---|---|---|---|---|---|
| 30944 | UNC84B | NM_015374.1 | 1006 | cagagactcatcgccacatttcc | 120440 | - | see also 6401 line |
| 30945 | GABARAP | NM_007278.1 | 183 | gaccgggtgccggtgatagtaga | 120451 | - | see also 13528 line |
| 30946 | GABARAP | NM_007278.1 | 185 | ccgggtgccggtgatagtagaaa | 120452 | - | see also 13528 line |
| 30947 | GAPCENA | NM_012197.2 | 1926 | ccgggatattaaccgaacattcc | 120518 | - | see also 5186 line |
| 30948 | GAPCENA | NM_012197.2 | 1432 | ttcagcaaacgtagtactactga | 120501 | - | see also 5186 line |
| 30949 | LMOD1 | NM_012134.1 | 1124 | gactgaggtgaacgtcaacaact | 120553 | - | see also 13530 line |
| 30950 | LMOD1 | NM_012134.1 | 1067 | agctcccagcatatttgatgagc | 120552 | - | see also 13530 line |
| 30951 | TNNT1 | NM_003283.3 | 294 | gacactcatcgatgtacatttcg | 120561 | - | see also 13531 line |
| 30952 | TNNT1 | NM_003283.3 | 234 | gcgcgttgacttcgatgacatcc | 120559 | - | see also 13531 line |
| 30953 | TNNT3 | NM_006757.1 | 198 | gcgtcagaacaaagacctaatgg | 120562 | - | see also 13532 line |
| 30954 | KIFAP3 | NM_014970.2 | 563 | aaccgccgtgattcattgtcagg | 120573 | - | see also 6160 line |
| 30955 | KIFAP3 | NM_014970.2 | 1358 | atcaccctccgacttttactaaa | 120596 | - | see also 6160 line |
| 30956 | MAPK8IP2 | NM_012324.2 | 2406 | tccccgcaacagctgctatttcg | 120664 | - | see also 5275 line |
| 30957 | MAPK8IP2 | NM_012324.2 | 2062 | acgggggagcgcggtgtgtttcc | 120662 | - | see also 5275 line |
| 30958 | MAPK8IP3 | NM_015133.1 | 3978 | ggcagagcgcagtcacatcatcg | 120688 | - | see also 13536 line |
| 30959 | MAPK8IP3 | NM_015133.1 | 3568 | gggctccgagccaataagacatc | 120683 | - | see also 13536 line |
| 30960 | TRIM2 | NM_015271.2 | 1087 | acctcgggacgatcttaaccacc | 120701 | - | see also 6301 line |
| 30961 | TRIM2 | NM_015271.2 | 1083 | cacaacctcgggacgatcttaac | 120700 | - | see also 6301 line |
| 30962 | TNKS1BP1 | NM_033396.1 | 5351 | tccgcggtccagcgttcgaaatc | 120752 | - | see also 13538 line |
| 30963 | TNKS1BP1 | NM_033396.1 | 3558 | gaggctgggtcggtgagtttagc | 120742 | - | see also 13538 line |
| 30964 | ARGBP2 | NM_003603.2 | 2171 | cccgatctcatacgtagagaaac | 120786 | - | see also 13539 line |
| 30965 | ARGBP2 | NM_003603.2 | 2567 | accgtttcaggctctgtataact | 120798 | - | see also 13539 line |
| 30966 | ARGBP2 | NM_003603.2 | 1939 | accgtgagtcccctagaagttac | 120779 | - | see also 13539 line |
| 30967 | BAIAP2 | NM_006340.1 | 122 | accggctcacggaaaatgtctat | 120837 | - | see also 4436 line |
| 30968 | BAIAP2 | NM_006340.1 | 168 | ccctagcctccggaacttcatcg | 120840 | - | see also 4436 line |
| 30969 | CDC42EP3 | NM_006449.3 | 1174 | gggaactacgagcttttacctgg | 120847 | - | see also 4523 line |
| 30970 | CDC42EP3 | NM_006449.3 | 1617 | caccccatgcgagctcatcaagg | 120855 | - | see also 4523 line |
| 30971 | AF3P21 | NM_016453.2 | 1288 | gcgcagttgggcactatatgagg | 120864 | - | see also 13542 line |
| 30972 | AF3P21 | NM_016453.2 | 1065 | ctggcctgagccacgaattatgc | 120861 | - | see also 13542 line |

Figure 46

| | | | | | | | |
|---|---|---|---|---|---|---|---|
| 30973 | DLG1 | NM_004087.1 | 997 | tgcgggtcaatgactgtatatta | 120893 | - | see also 2754 line |
| 30974 | JUP | NM_002230.1 | 1133 | atccgtgtgtcccagccaataagc | 120947 | - | see also 13544 line |
| 30975 | JUP | NM_002230.1 | 1539 | aactctgtcgtctcaactatgg | 120948 | - | see also 13544 line |
| 30976 | JUP | NM_002230.1 | 916 | tggccgacgggctgcaaagatg | 120945 | - | see also 13544 line |
| 30977 | MYLIP | NM_013262.3 | 1238 | gcgttgtgacctgtttcaaga | 120977 | - | see also 13545 line |
| 30978 | MYLIP | NM_013262.3 | 887 | gcccaattaataggatagcttat | 120971 | - | see also 13545 line |
| 30979 | PKD2 | NM_000297.2 | 1009 | ctgttagggttccacgaattacg | 120992 | - | see also 262 line |
| 30980 | PKD2 | NM_000297.2 | 1966 | ttcactcaattccgtatcatttt | 121022 | - | see also 262 line |
| 30981 | RPH3AL | NM_006987.2 | 529 | gaccatgagcgcgaaatgtgattgg | 121038 | - | see also 13547 line |
| 30982 | RPH3AL | NM_006987.2 | 685 | gcccctgggctgtgtaagatct | 121040 | - | see also 13547 line |
| 30983 | SDC1 | NM_002997.3 | 311 | gccgcaaattgtggctactaatt | 121043 | - | see also 13548 line |
| 30984 | SDC1 | NM_002997.3 | 312 | ccgcaaattgtggctactaattt | 121044 | - | see also 13548 line |
| 30985 | SDC2 | NM_002998.2 | 1182 | aggcacctactaaggagtttat | 121066 | - | see also 13549 line |
| 30986 | SDC2 | NM_002998.2 | 721 | agcttcaggagtgtatcctattg | 121050 | - | see also 13549 line |
| 30987 | SDC3 | NM_014654.1 | 2479 | gaggtgctgtagctgrgattgt | 121069 | - | see also 13550 line |
| 30988 | SDC3 | NM_014654.1 | 1592 | agcaggagtcgggcattgagaca | 121067 | - | see also 13550 line |
| 30989 | SDC4 | NM_002999.2 | 338 | cccacgaaccaagaaactaga | 121072 | - | see also 2014 line |
| 30990 | SDC4 | NM_002999.2 | 382 | gagaatctcaccgttgaagaga | 121075 | - | see also 2014 line |
| 30991 | SSH3BP1 | NM_005470.1 | 491 | ttcggaaacctcatcgattacaca | 121088 | - | see also 13551 line |
| 30992 | SSH3BP1 | NM_005470.1 | 657 | gggacggaatactcctttataaaa | 121094 | - | see also 13551 line |
| 30993 | PPP3R1 | NM_000945.2 | 544 | tcctttagtacagcgagtaatag | 121126 | - | see also 625 line |
| 30994 | PPP3R1 | NM_000945.2 | 552 | tacagcgagtaatagatatattc | 121128 | - | see also 625 line |
| 30995 | ADCY1 | NM_021116.1 | 1016 | ttcggcaagttgatgaattagc | 121136 | - | see also 13553 line |
| 30996 | ADCY1 | NM_021116.1 | 2015 | gggtgcctgacgattcagattg | 121166 | - | see also 13553 line |
| 30997 | ADD3 | NM_016824.1 | 1732 | gacaatcgaacgtaaacaacaag | 121236 | - | see also 6969 line |
| 30998 | ADD3 | NM_016824.1 | 1727 | aagaggacaatcgaacgtaaaca | 121233 | - | see also 6969 line |
| 30999 | AKAP5 | NM_004857.2 | 1819 | gtcaacccagatctaagtgaag | 121252 | - | see also 13555 line |
| 31000 | AKAP5 | NM_004857.2 | 1539 | gtcttgtaacacgcaggaaaagg | 121244 | - | see also 13555 line |
| 31001 | GAP43 | NM_002045.2 | 177 | ctcataaggccgcaaccaaaatt | 121270 | - | see also 13556 line |
| 31002 | GAP43 | NM_002045.2 | 131 | gacgaccaaaagattgaacaaga | 121269 | - | see also 13556 line |
| 31003 | IQGAP1 | NM_003870.2 | 2262 | ctggggtgactgccgcatataac | 121322 | - | see also 2627 line |
| 31004 | IQGAP1 | NM_003870.2 | 2336 | cgctgccgtggatactagttcg | 121325 | - | see also 2627 line |
| 31005 | ITPKA | NM_002220.1 | 1328 | ccgcgaggacggctatttgctgg | 121395 | - | see also 13558 line |
| 31006 | ITPKA | NM_002220.1 | 539 | gccggtcataagccctttcaaga | 121390 | - | see also 13558 line |
| 31007 | ITPKB | NM_002221.2 | 781 | cagggtccatccgctttgtaag | 121400 | - | see also 13559 line |
| 31008 | ITPKB | NM_002221.2 | 782 | agggtccatccgctttgtaagg | 121401 | - | see also 13559 line |
| 31009 | KCNH1 | NM_002238.2 | 235 | ttcggcggtccaatgatactaat | 121421 | - | see also 13560 line |

Figure 46

| | | | | | | | |
|---|---|---|---|---|---|---|---|
| 31010 | KCNH1 | NM_002238.2 | 238 | ggcggtccaatgatactattt | 121424 | - | see also 13560 line |
| 31011 | KCNH5 | NM_139318.3 | 1737 | ggcggtagagttccaaaccattc | 121547 | - | see also 9688 line |
| 31012 | KCNH5 | NM_139318.3 | 1244 | ctccatatcgctacaataccagt | 121538 | - | see also 9688 line |
| 31013 | KCNN1 | NM_002248.3 | 554 | gcctcgggaaaccctcaaatgt | 121573 | - | see also 13562 line |
| 31014 | KCNN1 | NM_002248.3 | 1507 | cagggagacgtgctcatctaca | 121575 | - | see also 13562 line |
| 31015 | KCNN2 | NM_021614.2 | 1494 | ttggagcgatgtgttgatatca | 121599 | - | see also 13563 line |
| 31016 | KCNN2 | NM_021614.2 | 1498 | agcgatgtgttgatatcaataa | 121601 | - | see also 13563 line |
| 31017 | KCNN3 | NM_002249.3 | 2491 | tcccgacccgtacacacaagttca | 121633 | - | see also 1447 line |
| 31018 | KCNN3 | NM_002249.3 | 1783 | atgaccagcaggacgtaactagt | 121620 | - | see also 1447 line |
| 31019 | KCNN4 | NM_002250.2 | 937 | agcatcggcgtctcaatcaagt | 121640 | - | see also 13565 line |
| 31020 | KCNN4 | NM_002250.2 | 769 | ccgccgtgcgtgcaggatttagg | 121637 | - | see also 13565 line |
| 31021 | MAP2 | NM_002374.2 | 5402 | aggtggcggacgtgtgaaaattg | 121759 | - | see also 1526 line |
| 31022 | NOS1 | NM_000620.1 | 3902 | gagcggaacacggctttaggtgt | 121807 | - | see also 417 line |
| 31023 | NOS1 | NM_000620.1 | 4853 | agggatgaacaacgataccatga | 121819 | - | see also 417 line |
| 31024 | NOS2A | NM_000625.3 | 535 | ggcagcggatgacttccaaga | 121828 | - | see also 419 line |
| 31025 | NOS2A | NM_000625.3 | 680 | ttctacctcaagctatcgaattt | 121833 | - | see also 419 line |
| 31026 | NRGN | NM_006176.1 | 269 | gccggacgacgacattctagaca | 121854 | - | see also 13568 line |
| 31027 | PDE1A | NM_005019.2 | 1075 | cacgtgagtgcagcttatcgact | 121887 | - | see also 13569 line |
| 31028 | PDE1A | NM_005019.2 | 1157 | ttcggaacctagtgattgaaatg | 121888 | - | see also 13569 line |
| 31029 | PDE1B | NM_000924.2 | 1057 | cagctctgtttccgattgatgc | 121913 | - | see also 13570 line |
| 31030 | PDE1B | NM_000924.2 | 574 | acggatgttccggagaaacataca | 121904 | - | see also 13570 line |
| 31031 | PHKA1 | NM_002637.1 | 3075 | agcgttcgtcccactgattcaaa | 121973 | - | see also 1708 line |
| 31032 | PHKA2 | NM_000292.1 | 3151 | cagatgactaggccgtttagtgc | 122078 | - | see also 257 line |
| 31033 | PHKA2 | NM_000292.1 | 2278 | ttgccgactaaagttctaagtgc | 122060 | - | see also 257 line |
| 31034 | PHKB | NM_000293.1 | 2369 | tgcgctacggggctgcatttacc | 122168 | - | see also 261 line |
| 31035 | PHKB | NM_000293.1 | 1746 | tgttacccgattattttcgacc | 122143 | - | see also 261 line |
| 31036 | PPP3CA | NM_000944.2 | 911 | aagtattcagaacgcgtatatga | 122206 | - | see also 13574 line |
| 31037 | PPP3CA | NM_000944.2 | 1733 | accctgcaaagcgctactgtttga | 122221 | - | see also 13574 line |
| 31038 | PPP3CB | NM_021132.1 | 1660 | accacgggacgggcaaccatact | 122270 | - | see also 13575 line |
| 31039 | PPP3CB | NM_021132.1 | 466 | ctgctaatacacgataccttttt | 122237 | - | see also 13575 line |
| 31040 | PPP3CB | NM_021132.1 | 467 | tgctaatacacgatacctttttc | 122238 | - | see also 13575 line |
| 31041 | PPP3CC | NM_005605.3 | 1476 | tacagttcgtaaggagatcatca | 122297 | - | see also 13576 line |
| 31042 | PPP3CC | NM_005605.3 | 355 | accacccgaccgcgtcatcaaagc | 122272 | - | see also 13576 line |
| 31043 | RGS1 | NM_002922.2 | 606 | tgcaggctaatagcctaaagtga | 122326 | - | see also 13577 line |
| 31044 | RGS1 | NM_002922.2 | 452 | gacttccgcactcgagaatctac | 122321 | - | see also 13577 line |
| 31045 | RGS1 | NM_002922.2 | 91 | ttcacttctagacgacaaaatgc | 122312 | - | see also 13577 line |
| 31046 | RGS16 | NM_002928.2 | 485 | cagggcacacagatctttgagg | 122330 | - | see also 13578 line |

Figure 46

| | | | | | | | |
|---|---|---|---|---|---|---|---|
| 31047 | RGS16 | NM_002928.2 | 515 | ttgcagtgaggccctaaagagg | 122331 | - | see also 13578 line |
| 31048 | RGS2 | NM_002923.1 | 158 | gaccccgtttgagctacttcttac | 122334 | - | see also 13579 line |
| 31049 | RGS2 | NM_002923.1 | 159 | accgtttgagctacttcttaca | 122335 | - | see also 13579 line |
| 31050 | RGS4 | NM_005613.3 | 425 | atcaaatcaccatctaaactaag | 122348 | - | see also 13580 line |
| 31051 | RGS4 | NM_005613.3 | 157 | ttgcttgaggagtgcaaaagata | 122342 | - | see also 13580 line |
| 31052 | RGS4 | NM_005613.3 | 164 | aggagtgcaaaagatatgaaaca | 122344 | - | see also 13580 line |
| 31053 | RYR2 | NM_001035.1 | 12549 | aacgcatcgagagggtctatttt | 122637 | - | see also 634 line |
| 31054 | RYR2 | NM_001035.1 | 3413 | gccggacggtggtatttgaatt | 122421 | - | see also 634 line |
| 31055 | RYR2 | NM_001035.1 | 12550 | acgcatcgagaggtctatttg | 122638 | - | see also 634 line |
| 31056 | SLC8A1 | NM_021097.1 | 84 | tacaaacgcggcgattaagtct | 122715 | - | see also 8084 line |
| 31057 | SLC8A1 | NM_021097.1 | 87 | aacatgcggcgattaagtcttc | 122716 | - | see also 8084 line |
| 31058 | SLC8A3 | NM_033262.3 | 3496 | caacactagaggcctattgctaca | 122814 | - | see also 9389 line |
| 31059 | SLC8A3 | NM_033262.3 | 3370 | ctgcatcagcgtgctcttgtacc | 122813 | - | see also 9389 line |
| 31060 | STRN | NM_003162.1 | 1654 | aacatcgaccctatgattctta | 122856 | - | see also 2135 line |
| 31061 | STRN | NM_003162.1 | 1291 | ggccttacggtggccaatgaagc | 122842 | - | see also 2135 line |
| 31062 | STRN3 | NM_014574.2 | 790 | cagcgggtaagtcattacttgg | 122898 | - | see also 5757 line |
| 31063 | STRN3 | NM_014574.2 | 1277 | ttgcagacttgacggtaacaaat | 122913 | - | see also 5757 line |
| 31064 | STRN4 | NM_013403.1 | 1307 | cgcactagacgcgcattcgttcc | 122967 | - | see also 5431 line |
| 31065 | STRN4 | NM_013403.1 | 1833 | ctctggcgacaccgtcttgtatg | 122974 | - | see also 5431 line |
| 31066 | IL18R1 | NM_003855.2 | 1329 | aagccgaagactaatcattgtcc | 123005 | - | see also 13586 line |
| 31067 | IL18R1 | NM_003855.2 | 792 | atcggatcctaatatacatgaag | 122998 | - | see also 13586 line |
| 31068 | IL18RAP | NM_003853.2 | 706 | gggtagtaacgacctatctgatg | 123025 | - | see also 13587 line |
| 31069 | IL18RAP | NM_003853.2 | 734 | tggtaccaacaaccttgaatgg | 123026 | - | see also 13588 line |
| 31070 | IL1R1 | NM_000877.2 | 1231 | gacctatgacgcatatatactgt | 123119 | - | see also 13588 line |
| 31071 | IL1R1 | NM_000877.2 | 422 | gtggagaatgatgcctaacttatg | 123075 | - | see also 13588 line |
| 31072 | IL1RAP | NM_002182.2 | 467 | cccgagaaccgcattagtaagg | 123150 | - | see also 1392 line |
| 31073 | IL1RAP | NM_002182.2 | 466 | tccccgagaaccgcattagtaag | 123149 | - | see also 1392 line |
| 31074 | IL1RAPL1 | NM_014271.2 | 1676 | ttcataaacgagagctaargtac | 123240 | - | see also 13590 line |
| 31075 | IL1RAPL1 | NM_014271.2 | 1666 | agcgttctccttcataaacgaga | 123239 | - | see also 13590 line |
| 31076 | IL1RAPL2 | NM_017416.1 | 1185 | cagcaggatccgctatttagaaa | 123282 | - | see also 6987 line |
| 31077 | IL1RAPL2 | NM_017416.1 | 1724 | aagctgaactggcgaattatacc | 123299 | - | see also 6987 line |
| 31078 | IL1RL1 | NM_003856.2 | 1168 | ggcacaccgtaagactaagtagg | 123370 | - | see also 13592 line |
| 31079 | IL1RL1 | NM_003856.2 | 363 | accgtggattggtattacttcaca | 123340 | - | see also 13592 line |
| 31080 | IL1RL2 | NM_003854.2 | 704 | acgcgtgtcaagccatactgaca | 123427 | - | see also 13593 line |
| 31081 | IL1RL2 | NM_003854.2 | 1677 | cacggagcagtcacagtgtatga | 123469 | - | see also 13593 line |
| 31082 | MYD88 | NM_002468.2 | 788 | agcgactgatcccatcaagtac | 123478 | - | see also 13594 line |
| 31083 | MYD88 | NM_002468.2 | 367 | gaggaggattgccaaaagtatat | 123472 | - | see also 13594 line |

Figure 46

| 31084 | SIGIRR | NM_021805.1 | 1392 | atctggctcgcgaaactacagt | 123485 | - | see also 13595 line |
|---|---|---|---|---|---|---|---|
| 31085 | SIGIRR | NM_021805.1 | 756 | accgcaagttcgtgaacttcatc | 123481 | - | see also 13595 line |
| 31086 | SIGIRR | NM_021805.1 | 1103 | gtgactccttcctccgattttg | 123483 | - | see also 13595 line |
| 31087 | TLR1 | NM_003263.2 | 1208 | ttcggtttccgcaaagttatat | 123525 | - | see also 2214 line |
| 31088 | TLR1 | NM_003263.2 | 1281 | cacgcatggtccacatgctttgc | 123527 | - | see also 2214 line |
| 31089 | TLR10 | NM_030956.1 | 2182 | gacgttcatctcacgaattatc | 123606 | - | see also 13597 line |
| 31090 | TLR10 | NM_030956.1 | 2758 | cccaagataggcgtaaatgtgg | 123624 | - | see also 13597 line |
| 31091 | TLR2 | NM_003264.2 | 1474 | aacttatccagcacacgaataca | 123665 | - | see also 13598 line |
| 31092 | TLR2 | NM_003264.2 | 471 | atcctataattacttatctaatt | 123640 | - | see also 13598 line |
| 31093 | TLR6 | NM_006068.2 | 148 | ttctccgacggaaatgaatttgc | 123687 | - | see also 13599 line |
| 31094 | TLR6 | NM_006068.2 | 2326 | ctcatgacgcagcggacttattt | 123736 | - | see also 13599 line |
| 31095 | TLR6 | NM_006068.2 | 691 | ttgctatccaagtgaacatatc | 123707 | - | see also 13599 line |
| 31096 | IL1R2 | NM_004633.3 | 234 | tgcgcttgtacgtgttggtaatg | 123742 | - | see also 13600 line |
| 31097 | IL1R2 | NM_004633.3 | 542 | acctacgtctgcactactagaaa | 123745 | - | see also 13600 line |
| 31098 | IL1R2 | NM_004633.3 | 545 | tacgtctgcactactagaaatgc | 123746 | - | see also 13600 line |
| 31099 | IL2RA | NM_000417.1 | 796 | tgcctgtcacaacaacagatt | 123783 | - | see also 13601 line |
| 31100 | IL2RA | NM_000417.1 | 589 | gtggggcagatggtttattatca | 123779 | - | see also 13601 line |
| 31101 | IL2RB | NM_000878.2 | 1159 | tgcctgagcccgcatccttaagc | 123799 | - | see also 13602 line |
| 31102 | IL2RB | NM_000878.2 | 1158 | gtgcctgagcccgcatccttaag | 123798 | - | see also 13602 line |
| 31103 | IL2RG | NM_000206.1 | 288 | aagaactcggataatgataaagt | 123818 | - | see also 13603 line |
| 31104 | IL2RG | NM_000206.1 | 599 | cagctggactgaacaatcagtgg | 123823 | - | see also 13603 line |
| 31105 | IL10RA | NM_001558.2 | 587 | tcgcaaggtgccgggaaacttca | 123841 | - | see also 13604 line |
| 31106 | IL10RA | NM_001558.2 | 695 | atctgccttccgagtaaca | 123844 | - | see also 13604 line |
| 31107 | IL10RB | NM_000628.3 | 303 | tactaccttgacggaatgtgatt | 123861 | - | see also 13605 line |
| 31108 | IL10RB | NM_000628.3 | 157 | atggtaccacctccgaaaatgt | 123854 | - | see also 13605 line |
| 31109 | IL10RB | NM_000628.3 | 830 | tgctgtggtgtttacaagaag | 123876 | - | see also 13605 line |
| 31110 | IL8RA | NM_000634.2 | 1032 | taegccttcatcggccaaaattt | 123892 | - | see also 13606 line |
| 31111 | IL8RA | NM_000634.2 | 1134 | tacacttcttgtctgtcaatgt | 123897 | - | see also 13606 line |
| 31112 | IL8RA | NM_000634.2 | 1068 | ctcaagatcctggctatgccatgg | 123895 | - | see also 13606 line |
| 31113 | IL8RB | NM_001557.2 | 1381 | ctcctccaagattctagcttataca | 123902 | - | see also 13607 line |
| 31114 | IL8RB | NM_001557.2 | 451 | tggaaaggtgaagatcttagtaa | 123899 | - | see also 13607 line |
| 31115 | IL7R | NM_002185.2 | 451 | tagaacctaaccactatagttaaa | 123913 | - | see also 1396 line |
| 31116 | IL7R | NM_002185.2 | 1214 | tgcatgtgacggcccctattctct | 123941 | - | see also 1396 line |
| 31117 | IL6R | NM_000565.2 | 1207 | atcgggctgaacggtcaaagaca | 123957 | - | see also 13608 line |
| 31118 | IL6R | NM_000565.2 | 1084 | ctgatccgcctgccaacatcaca | 123955 | - | see also 13608 line |
| 31119 | IL6ST | NM_002184.2 | 1681 | accgtgcatcgcacctatttaag | 124021 | - | see also 1395 line |
| 31120 | IL6ST | NM_002184.2 | 918 | gcccaatccgccacataattat | 123992 | - | see also 1395 line |

Figure 46

| 31121 | IL6ST | NM_002184.2 | 1680 | taccgtgcatcgcacctatttaa | 124020 | - | see also 1395 line |
|---|---|---|---|---|---|---|---|
| 31122 | CSF2RB | NM_000395.1 | 2525 | ctctcgctccggagtaaaccttc | 124072 | - | see also 13610 line |
| 31123 | CSF2RB | NM_000395.1 | 967 | cgcgacccacggccaatacatcg | 124063 | - | see also 13610 line |
| 31124 | IL5RA | NM_000564.2 | 620 | atcatcgtggcgcatgtattact | 124075 | - | see also 13611 line |
| 31125 | IL5RA | NM_000564.2 | 1243 | ctggcttgcggtgcttgttaacg | 124093 | - | see also 13611 line |
| 31126 | IL4R | NM_000418.1 | 990 | gccgcctcgtggctataataatc | 124119 | - | see also 13612 line |
| 31127 | IL4R | NM_000418.1 | 609 | acccgtatccccctgacaattac | 124111 | - | see also 13612 line |
| 31128 | IL3RA | NM_002183.2 | 490 | gaccgatatcgagtgtgttaaag | 124130 | - | see also 13613 line |
| 31129 | IL3RA | NM_002183.2 | 491 | accgatatcgagtgtgttaaaga | 124131 | - | see also 13613 line |
| 31130 | IL3RA | NM_002183.2 | 775 | tgccaacaggcgtcaacagtacg | 124136 | - | see also 13613 line |
| 31131 | IL21R | NM_021798.2 | 528 | ctcctggcgctcagattacgaag | 124155 | - | see also 13615 line |
| 31132 | IL21R | NM_021798.2 | 529 | tcctggcgctcagattacgaaga | 124156 | - | see also 13615 line |
| 31133 | IL21R | NM_021798.2 | 385 | atggccgacgacattttcagtgt | 124153 | - | see also 13615 line |
| 31134 | IL17R | NM_014339.3 | 1876 | tcccgactggttcgaatgtgaga | 124174 | - | see also 13616 line |
| 31135 | IL17R | NM_014339.3 | 550 | caggcggtggcgttttaccttca | 124170 | - | see also 13616 line |
| 31136 | IL17R | NM_014339.3 | 546 | accacaggcggtggcgttttacc | 124169 | - | see also 13616 line |
| 31137 | IL12RB2 | NM_001559.2 | 1860 | tgcccactcgtattaacataatg | 124209 | - | see also 13617 line |
| 31138 | IL12RB2 | NM_001559.2 | 1317 | ctccgtgggacattagaatcaaa | 124196 | - | see also 13617 line |
| 31139 | IL13RA2 | NM_000640.2 | 141 | ttggctatcggatgcttatatac | 124229 | - | see also 13618 line |
| 31140 | IL13RA2 | NM_000640.2 | 251 | tcccggatacttaggttatctct | 124233 | - | see also 13618 line |
| 31141 | IL15RA | NM_002189.2 | 784 | cccgctggccagcgttgaaatgg | 124291 | - | see also 13619 line |
| 31142 | IL15RA | NM_002189.2 | 298 | gacggagtgcgtgttgaacaagg | 124284 | - | see also 13619 line |
| 31143 | IL17RB | NM_018725.2 | 94 | cccgagagccgaccgttcaatgt | 124293 | - | see also 7582 line |
| 31144 | IL17RB | NM_018725.2 | 819 | agcgactgcatccgacataaagg | 124317 | - | see also 7582 line |
| 31145 | IL17RB | NM_018725.2 | 684 | agcactatcatcgggttttctca | 124311 | - | see also 7582 line |
| 31146 | IL18BP | NM_005699.2 | 1106 | aagcagtgtccagcattggaagt | 124336 | - | see also 13621 line |
| 31147 | CCR5 | NM_000579.1 | 662 | aactcttgacagggctctatttt | 124345 | - | see also 13622 line |
| 31148 | CCR5 | NM_000579.1 | 1355 | agcgagcaagctcagtttacacc | 124351 | - | see also 13622 line |
| 31149 | CCR6 | NM_004367.3 | 500 | gtctatgacagacgtctatctct | 124355 | - | see also 13623 line |
| 31150 | CCR6 | NM_004367.3 | 743 | aacactaccgcgcagcaaaatca | 124362 | - | see also 13623 line |
| 31151 | CCR8 | NM_005201.2 | 147 | gacaacagtgaccgactactact | 124378 | - | see also 13624 line |
| 31152 | CCR8 | NM_005201.2 | 189 | cccctgtgatgcggaacttattc | 124380 | - | see also 13624 line |
| 31153 | CCR8 | NM_005201.2 | 188 | gcccctgtgatgcggaacttatt | 124379 | - | see also 13624 line |
| 31154 | CCR9 | NM_006641.2 | 153 | aagactacgttaacttcaacttc | 124403 | - | see also 4695 line |
| 31155 | CCR9 | NM_006641.2 | 323 | accgacatgttccttttgaattt | 124406 | - | see also 4695 line |
| 31156 | CCRL1 | NM_016557.2 | 649 | cccgctacctaggaacatcaatg | 124435 | - | see also 13626 line |
| 31157 | CCRL1 | NM_016557.2 | 648 | ccccgctacctaggaacatcaat | 124434 | - | see also 13626 line |

Figure 46

| | | | | | | | |
|---|---|---|---|---|---|---|---|
| 31158 | CCRL1 | NM_016557.2 | 260 | ttccatggtagtggcaatttatg | 124422 | - | see also 13626 line |
| 31159 | GPR2 | NM_016602.1 | 79 | gccactgccggagctttgctaca | 124453 | - | see also 13627 line |
| 31160 | CXCR3 | NM_001504.1 | 510 | gaccgctacctgaacatagttca | 124457 | - | see also 13628 line |
| 31161 | CXCR3 | NM_001504.1 | 512 | ccgctacctgaacatagttcatg | 124458 | - | see also 13628 line |
| 31162 | CCBP2 | NM_001296.3 | 743 | gtggaactgccacgcagatttcg | 124468 | - | see also 13629 line |
| 31163 | CCBP2 | NM_001296.3 | 523 | tggttgagcactctttatactatt | 124465 | - | see also 13629 line |
| 31164 | CCL13 | NM_005408.2 | 168 | atctacttgcttcacattta | 124476 | - | see also 13630 line |
| 31165 | CCL13 | NM_005408.2 | 183 | cacatttagcagtaagaagatct | 124477 | - | see also 13630 line |
| 31166 | CCR1 | NM_001295.1 | 818 | gacccctacaatttgactatac | 124500 | - | see also 13631 line |
| 31167 | CCR1 | NM_001295.1 | 819 | acccctacaatttgactatact | 124501 | - | see also 13631 line |
| 31168 | CCR2 | NM_000647.3 | 89 | cacatctgttctggtttatca | 124503 | - | see also 13632 line |
| 31169 | CCR2 | NM_000647.3 | 162 | tacggtgtccctgtcataaatt | 124508 | - | see also 13632 line |
| 31170 | CCR3 | NM_001837.2 | 626 | tccgaattatgaccaacatctac | 124521 | - | see also 13633 line |
| 31171 | CCR3 | NM_001837.2 | 1476 | gccggaactctcattgtgtttt | 124533 | - | see also 13633 line |
| 31172 | CCR3 | NM_001837.2 | 1470 | agcagagccggaactctctattg | 124531 | - | see also 13633 line |
| 31173 | CCR4 | NM_005508.2 | 82 | ccctcgatgaaagcatatacagc | 124538 | - | see also 3848 line |
| 31174 | CCR4 | NM_005508.2 | 590 | tactgagcgcaaccatacctact | 124547 | - | see also 3848 line |
| 31175 | CCR7 | NM_001838.2 | 214 | aaggacgtgcggaactttaaagc | 124555 | - | see also 13635 line |
| 31176 | CCR7 | NM_001838.2 | 298 | gtcgtttgacctatactattt | 124557 | - | see also 13635 line |
| 31177 | CMKLR1 | NM_004072.1 | 1547 | accccagccataagaagctttacc | 124572 | - | see also 13636 line |
| 31178 | CMKLR1 | NM_004072.1 | 1507 | ctggtcaatgtctaagtgaaga | 124570 | - | see also 13636 line |
| 31179 | CX3CR1 | NM_001337.2 | 821 | caccctacaacgttatgatttc | 124585 | - | see also 13637 line |
| 31180 | CX3CR1 | NM_001337.2 | 1037 | ggcgtcagtccacgttgatttc | 124588 | - | see also 13637 line |
| 31181 | CX3CR1 | NM_001337.2 | 692 | tcccctgtcatttatgagttat | 124581 | - | see also 13637 line |
| 31182 | CXCR4 | NM_003467.1 | 946 | caccgaggccctagctttcttcc | 124602 | - | see also 13638 line |
| 31183 | CXCR4 | NM_003467.1 | 240 | ctggcattgtggcaatggattg | 124595 | - | see also 13638 line |
| 31184 | XCR1 | NM_005283.1 | 24 | gagcaccacctttttactatg | 124609 | - | see also 13640 line |
| 31185 | IFNGR1 | NM_000416.1 | 220 | tacgtagaggtaaagaactatg | 124614 | - | see also 13641 line |
| 31186 | IFNGR1 | NM_000416.1 | 1207 | acctggcagcatgctttaaact | 124643 | - | see also 13641 line |
| 31187 | IFNGR1 | NM_000416.1 | 530 | ccgaaaactaccgttacattag | 124628 | - | see also 13641 line |
| 31188 | IFNGR2 | NM_005534.1 | 1043 | ttcaacactatcggaatgtgact | 124663 | - | see also 13642 line |
| 31189 | IFNGR2 | NM_005534.1 | 1324 | gtcgggcatttaagcaacacatatc | 124671 | - | see also 13642 line |
| 31190 | IFNGR2 | NM_005534.1 | 1233 | ctccatttcattggataacttaa | 124666 | - | see also 13642 line |
| 31191 | IFNAR1 | NM_000629.1 | 991 | ctccgcgtacaagcatctgatgg | 124704 | - | see also 13643 line |
| 31192 | IFNAR1 | NM_000629.1 | 1090 | aacattagatccttagttgattc | 124707 | - | see also 13643 line |
| 31193 | IFNAR1 | NM_000629.1 | 1152 | cacgcctgtgatccaggattatc | 124712 | - | see also 13643 line |
| 31194 | IFNAR2 | NM_000874.2 | 933 | aaacacgaactactgtgtatctgt | 124754 | - | see also 13644 line |

Figure 46

| 31195 | IFNAR2 | NM_000874.2 | 861 | aagaagcataaacccgaaataaa | 124748 | - | see also 13644 line | | |
|---|---|---|---|---|---|---|---|---|---|
| 31196 | IL22RA1 | NM_021258.2 | 1239 | tccctcctatggggtatgcatgg | 124774 | - | see also 13645 line | | |
| 31197 | IL22RA1 | NM_021258.2 | 1497 | agggacaccacagtacctaaagg | 124779 | - | see also 13645 line | | |
| 31198 | CRIM1 | NM_016441.1 | 2272 | cgcaataacagcgtacctaatta | 124817 | - | see also 13646 line | | |
| 31199 | CRIM1 | NM_016441.1 | 2742 | gtggcccacgcctagtgaaaatg | 124829 | - | see also 13646 line | | |
| 31200 | CTGF | NM_001901.1 | 422 | ggcgtgtgcaccgccaaagatgg | 124848 | - | see also 13647 line | | |
| 31201 | CTGF | NM_001901.1 | 711 | gcccagacccaactatgattaga | 124852 | - | see also 13647 line | | |
| 31202 | CTGF | NM_001901.1 | 1124 | tacaactgtcccggagacaatga | 124859 | - | see also 13647 line | | |
| 31203 | CYR61 | NM_001554.3 | 1083 | cccccgaaccagtcaggtttact | 124872 | - | see also 13648 line | | |
| 31204 | CYR61 | NM_001554.3 | 694 | ctgtgacgaggatagtatcaagg | 124868 | - | see also 13648 line | | |
| 31205 | ESM1 | NM_007036.2 | 120 | gccgcctggagcaataattatgc | 124880 | - | see also 13649 line | | |
| 31206 | ESM1 | NM_007036.2 | 589 | ctcccgtaatgaggaaatggtta | 124889 | - | see also 13649 line | | |
| 31207 | HTRA3 | NM_053044.2 | 657 | accagccgtggtccacatagagc | 124892 | - | - | trypsin | - |
| 31208 | IGFALS | NM_004970.1 | 577 | acctcaacctcggctggaatagc | 124893 | - | see also 13650 line | | |
| 31209 | IGFBP1 | NM_000596.1 | 622 | gacgccatcagtacctatgatgg | 124899 | - | see also 13651 line | | |
| 31210 | IGFBP1 | NM_000596.1 | 619 | tgggacgccatcagtacctatga | 124898 | - | see also 13651 line | | |
| 31211 | IGFBP2 | NM_000597.1 | 663 | gcccctcaagtcgggtatgaagg | 124906 | - | - | insulin-like growth factor binding | - |
| 31212 | IGFBP2 | NM_000597.1 | 1035 | ccccgagtgtcatctcttctaca | 124907 | - | see also 31211 line | | |
| 31213 | IGFBP3 | NM_000598.2 | 716 | atggtccctgccgtagagaaatg | 124910 | - | see also 13652 line | | |
| 31214 | IGFBP3 | NM_000598.2 | 697 | tccaagcgggagacagaatatgg | 124909 | - | see also 13652 line | | |
| 31215 | IGFBP4 | NM_001552.1 | 706 | aagcacttcgccaaaattcgaga | 124916 | - | see also 13653 line | | |
| 31216 | IGFBP4 | NM_001552.1 | 703 | cagaagcacttcgccaaaattcg | 124915 | - | see also 13653 line | | |
| 31217 | IGFBP6 | NM_002178.1 | 585 | gtctaccgaggggctcaaacact | 124924 | - | see also 13654 line | | |
| 31218 | NOV | NM_002514.2 | 1014 | gtgctccccagggcaaatagtca | 124937 | - | see also 13656 line | | |
| 31219 | NOV | NM_002514.2 | 421 | taccgcagtggagagaaatttca | 124933 | - | see also 13656 line | | |
| 31220 | PRSS11 | NM_002775.2 | 1027 | aactcgggaggcccgttagtaaa | 124957 | - | see also 1812 line | | |
| 31221 | PRSS11 | NM_002775.2 | 591 | tgccgtggttcatatcgaattgt | 124946 | - | see also 1812 line | | |
| 31222 | WISP1 | NM_003882.2 | 159 | cccctacgaccatggactttacc | 124965 | - | see also 2639 line | | |
| 31223 | WISP1 | NM_003882.2 | 1068 | cccgccaggtcctatggattaat | 124981 | - | see also 2639 line | | |
| 31224 | WISP2 | NM_003881.2 | 869 | ggggtcgcagtccacaaaacagt | 124985 | - | signal_transduction | - | - |
| 31225 | WISP2 | NM_003881.2 | 867 | caggggtcgcagtccacaaaaca | 124984 | - | see also 31224 line | | |
| 31226 | WISP3 | NM_003880.2 | 1022 | ctgtatccctaataagtctaaaa | 125010 | - | see also 13659 line | | |
| 31227 | WISP3 | NM_003880.2 | 475 | gcgagttcaaccaggtacattat | 124990 | - | see also 13659 line | | |
| 31228 | FIBP | NM_004214.4 | 280 | gagcgacaccatggaccattacc | 125020 | - | see also 13660 line | | |
| 31229 | FIBP | NM_004214.4 | 181 | tcgcctctggctcgatggttact | 125019 | - | see also 13660 line | | |
| 31230 | LTBP1 | NM_000627.1 | 2039 | gggccttccggtgtgaatactgt | 125074 | - | see also 420 line | | |

Figure 46

| 31231 | LTBP1 | NM_000627.1 | 132 | gcctccgctcctagtgagtaacc | 125032 | - | see also 420 line | | |
| 31232 | AVPR1A | NM_000706.3 | 2139 | cgccgtgctggcggtgactttcg | 125133 | - | see also 13662 line | | |
| 31233 | AVPR1A | NM_000706.3 | 2756 | aagcgggtgtggccttccaaaag | 125134 | - | see also 13662 line | | |
| 31234 | AVPR1B | NM_000707.2 | 1416 | gacaagaatgcccctgatgaaga | 125145 | - | see also 13663 line | | |
| 31235 | AVPR1B | NM_000707.2 | 1161 | atctgccatgagatctgtaaaaa | 125143 | - | see also 13663 line | | |
| 31236 | COPB2 | NM_004766.1 | 1653 | tgggtaggcgattgcttcattta | 125197 | - | see also 3276 line | | |
| 31237 | COPB2 | NM_004766.1 | 2698 | tactcgaactagaagtagatttg | 125234 | - | see also 3276 line | | |
| 31238 | GNB2L1 | NM_006098.2 | 380 | caccaccacgaggcgatttgtgg | 125242 | - | see also 4244 line | | |
| 31239 | GNB2L1 | NM_006098.2 | 461 | tggatctcgagataaaaccatca | 125246 | - | see also 4244 line | | |
| 31240 | HINT1 | NM_005340.2 | 143 | cacgatctttgggaagatcatcc | 125254 | - | kinase_related、signal transduction | zinc binding | - |
| 31241 | LIM | NM_006457.1 | 1333 | ctccgatgtgcgcccattgtaac | 125275 | - | see also 4529 line | | |
| 31242 | LIM | NM_006457.1 | 1334 | tccgatgtgcgcccattgtaacc | 125276 | - | see also 4529 line | | |
| 31243 | LIM | NM_006457.1 | 816 | gagcgctatacagagtttatca | 125270 | - | see also 4529 line | | |
| 31244 | CGI-121 | NM_016058.1 | 115 | aacacatcagctggacctatttc | 125289 | - | see also 13668 line | | |
| 31245 | CGI-121 | NM_016058.1 | 583 | gacattattggatgctatcattt | 125297 | - | see also 13668 line | | |
| 31246 | NBEA | NM_015678.2 | 3777 | accggtagcgtagacttaacttg | 125391 | - | see also 13669 line | | |
| 31247 | NBEA | NM_015678.2 | 3774 | atgaccggtagcgtagacttaac | 125390 | - | see also 13669 line | | |
| 31248 | PTPRR | NM_002849.2 | 1569 | gacgtcgtggcaagttcacattt | 125591 | - | see also 13670 line | | |
| 31249 | PTPRR | NM_002849.2 | 1566 | agggacgtcgtggcaagttcaca | 125590 | - | see also 13670 line | | |
| 31250 | DSCR1L1 | NM_005822.1 | 344 | gacgtgtccgtataaacttcagc | 125617 | - | see also 4060 line | | |
| 31251 | DSCR1L1 | NM_005822.1 | 676 | cacgtgtgcgacagtgacataga | 125623 | - | see also 4060 line | | |
| 31252 | MYOZ2 | NM_016599.2 | 836 | tcctaagggatggatatctgaga | 125645 | - | see also 6939 line | | |
| 31253 | MYOZ2 | NM_016599.2 | 599 | tagcaatgatccggagctttag | 125636 | - | see also 6939 line | | |
| 31254 | MYOZ2 | NM_016599.2 | 809 | ttctggcagacggtcctttaata | 125642 | - | see also 6939 line | | |
| 31255 | AKAP11 | NM_016248.2 | 1305 | atcccgtcatagggaagtcatcg | 125672 | - | see also 13673 line | | |
| 31256 | AKAP11 | NM_016248.2 | 1695 | accatactaataccctatcaaat | 125684 | - | see also 13673 line | | |
| 31257 | LILRB1 | NM_006669.2 | 1443 | gtctaagatcaacgtaccaatct | 125844 | - | see also 13674 line | | |
| 31258 | LILRB1 | NM_006669.2 | 1450 | atcaacgtaccaatctcaaaaat | 125846 | - | see also 13674 line | | |
| 31259 | PPP1R16B | NM_015568.2 | 1405 | gtgctactctccgaatttcctac | 125853 | - | see also 13675 line | | |
| 31260 | PPP1R16B | NM_015568.2 | 1406 | tgctactctccgaatttcctacc | 125854 | - | see also 13675 line | | |
| 31261 | NAKAP95 | NM_014371.1 | 511 | ggcctatgagggccaatacgatg | 125867 | - | see also 5634 line | | |
| 31262 | NAKAP95 | NM_014371.1 | 214 | caccaactatgggtatggtatgg | 125865 | - | see also 5634 line | | |
| 31263 | ARIH1 | NM_005744.2 | 1395 | gtctgtcgtaaccagaattgtaa | 125912 | - | see also 3984 line | | |
| 31264 | BIRC7 | NM_022161.2 | 627 | cagttcctgctccggtcaaaagg | 125929 | - | see also 13678 line | | |
| 31265 | BIRC7 | NM_022161.2 | 630 | ttcctgctccggtcaaaaggaag | 125930 | - | see also 13678 line | | |
| 31266 | CASP2 | NM_001224.3 | 694 | gggctatgacgtccatgttctat | 125945 | - | see also 13679 line | | |

Figure 46

| 31267 | CASP2 | NM_001224.3 | 695 | ggctatgacgtccatgttctatg | 125946 | - | see also 13679 line |
|---|---|---|---|---|---|---|---|
| 31268 | DNMT3L | NM_013369.2 | 1189 | gggacccttcgatcttgtgtacg | 125970 | - | see also 13680 line |
| 31269 | DNMT3L | NM_013369.2 | 703 | cgccccatgtaaggacaagttcc | 125964 | - | see also 13680 line |
| 31270 | HDAC10 | NM_032019.3 | 1605 | acgccggatatcacattggttct | 125983 | - | see also 13681 line |
| 31271 | HDAC10 | NM_032019.3 | 1601 | gacaacgccggatatcacattgg | 125982 | - | see also 13681 line |
| 31272 | PREX1 | NM_020820.2 | 4592 | caccacggcggtaaagatagacc | 126022 | - | see also 13682 line |
| 31273 | PREX1 | NM_020820.2 | 2580 | cacggcgtggtgtatgagtatgt | 126006 | - | see also 13682 line |
| 31274 | SERPINB12 | NM_080474.1 | 949 | atgggcattacggatatctttga | 126052 | - | see also 13683 line |
| 31275 | SERPINB12 | NM_080474.1 | 971 | atgaaacgagggctgatcttact | 126054 | - | see also 13683 line |
| 31276 | APBB1 | NM_001164.2 | 1495 | tgcctacgtagctcgtgataagc | 126077 | - | see also 13684 line |
| 31277 | APBB1 | NM_001164.2 | 1379 | gaggatgagacactaaagctagt | 126075 | - | see also 13684 line |
| 31278 | BAIAP1 | NM_004742.1 | 2082 | gcggacgtggcttcaaatagttc | 126117 | - | see also 13685 line |
| 31279 | BAIAP1 | NM_004742.1 | 900 | aagcgaaccaagtcctacaatga | 126092 | - | see also 13685 line |
| 31280 | CTBP1 | NM_001328.1 | 335 | gtccggattggcagtggttttga | 126149 | - | see also 13686 line |
| 31281 | CTBP1 | NM_001328.1 | 337 | ccggattggcagtggttttgaca | 126150 | - | see also 13686 line |
| 31282 | DLG4 | NM_001365.1 | 1411 | ctccatcgttcgcctctatgtca | 126161 | - | see also 927 line |
| 31283 | DLG4 | NM_001365.1 | 1250 | atcggtgacgacccatccatttt | 126157 | - | see also 927 line |
| 31284 | HSPB7 | NM_014424.3 | 947 | cggcgtcacccgcatacagaaca | 126188 | - | see also 13688 line |
| 31285 | HSPB7 | NM_014424.3 | 723 | ccctaggagacgcctatgagttt | 126186 | - | see also 13688 line |
| 31286 | ITGB1BP1 | NM_004763.2 | 515 | aagtatggcataaaagtatcaac | 126201 | - | see also 3274 line |
| 31287 | ITGB1BP1 | NM_004763.2 | 209 | tcccaaagtagcgaaatcagtac | 126192 | - | see also 3274 line |
| 31288 | ITGB3BP | NM_014288.3 | 192 | agcacagaaatggactatcaaat | 126212 | - | see also 13690 line |
| 31289 | ITGB3BP | NM_014288.3 | 50 | ctgaagttggatggtctgttaga | 126206 | - | see also 13690 line |
| 31290 | MLLT4 | NM_005936.1 | 220 | acgctcgcggagaaatttcgacc | 126223 | - | see also 13691 line |
| 31291 | MLLT4 | NM_005936.1 | 1424 | ttggggcgtcccatgtatttaag | 126242 | - | see also 13691 line |
| 31292 | PRKCABP | NM_012407.1 | 885 | ttcggcattcggcttctgaaaac | 126311 | - | see also 5311 line |
| 31293 | PRKCABP | NM_012407.1 | 741 | aacctcctacgggccttttatga | 126308 | - | see also 5311 line |
| 31294 | SNX17 | NM_014748.2 | 793 | agccttcggagtcaagagtataa | 126325 | - | see also 5941 line |
| 31295 | SNX17 | NM_014748.2 | 949 | cagcaccggcaactcaaatctct | 126332 | - | see also 5941 line |
| 31296 | TOPBP1 | NM_007027.2 | 2411 | accgtcgttacacctttagatat | 126402 | - | see also 4919 line |
| 31297 | TOPBP1 | NM_007027.2 | 2949 | atcgggagtataaatctgtaaaa | 126421 | - | see also 4919 line |
| 31298 | TRIM3 | NM_006458.2 | 2135 | tagaccggaatggacatatcatt | 126468 | - | see also 4530 line |
| 31299 | TRIM3 | NM_006458.2 | 1302 | gcggcgatcggtgctcaatctgg | 126465 | - | see also 4530 line |
| 31300 | ELMO1 | NM_014800.8 | 2005 | gtcctgcattacggagacttaga | 126521 | - | see also 5994 line |
| 31301 | ELMO1 | NM_014800.8 | 2008 | ctgcattacggagacttagaaga | 126522 | - | see also 5994 line |

Figure 46

| | | | | | | | | | |
|---|---|---|---|---|---|---|---|---|---|
| 31302 | LRRFIP2 | NM_006309.1 | 2119 | cggaagctacaacgagagttacg | 126583 | - | see also 13697 line | | |
| 31303 | LRRFIP2 | NM_006309.1 | 2123 | agctacaacgagagttacgaaca | 126584 | - | see also 13697 line | | |
| 31304 | KRT14 | NM_000526.3 | 523 | gcggcctgctgagatcaaagact | 126587 | - | see also 13698 line | | |
| 31305 | YWHAB | NM_003404.3 | 900 | tacgctgaatgaagagtcttata | 126598 | - | see also 13699 line | | |
| 31306 | YWHAB | NM_003404.3 | 786 | tcgtcttggtctggcactaaatt | 126596 | - | see also 13699 line | | |
| 31307 | YWHAG | NM_012479.2 | 704 | ccgattaggcctggctcttaact | 126604 | - | see also 13700 line | | |
| 31308 | YWHAG | NM_012479.2 | 502 | tggataactacctgatcaagaat | 126602 | - | see also 13700 line | | |
| 31309 | YWHAH | NM_003405.2 | 824 | cacactaaacgaggattcctata | 126608 | - | see also 13701 line | | |
| 31310 | YWHAH | NM_003405.2 | 826 | cactaaacgaggattcctataag | 126609 | - | see also 13701 line | | |
| 31311 | YWHAQ | NM_006826.2 | 516 | gcgtgtggtgatgatcgaaaaca | 126610 | - | kinase_related、 cell_cycle | - | - |
| 31312 | FCGR1A | NM_000566.2 | 935 | ttctctgggtgacaatacgtaaa | 126623 | - | see also 13702 line | | |
| 31313 | FCGR1A | NM_000566.2 | 207 | ctcgaccccagctacagaatca | 126611 | - | see also 13702 line | | |
| 31314 | FCGR2B | NM_004001.2 | 583 | cccgttcggatcccaacttctcc | 126628 | - | see also 13703 line | | |
| 31315 | FCGR2B | NM_004001.2 | 143 | cccccagccttggggtcatatgc | 126627 | - | see also 13703 line | | |
| 31316 | FCGR3A | NM_000569.4 | 628 | ggcccctcggtgggtgttcaagg | 126633 | - | see also 13704 line | | |
| 31317 | FCGR3A | NM_000569.4 | 502 | ggcctcgagctacttcattgacg | 126632 | - | see also 13704 line | | |
| 31318 | FCGRT | NM_004107.3 | 938 | cacgcctcgtcgtcactaacagt | 126644 | - | receptor_acitivity | receptor | - |
| 31319 | FCGRT | NM_004107.3 | 936 | tccacgcctcgtcgtcactaaca | 126643 | - | see also 31318 line | | |
| 31320 | FCGRT | NM_004107.3 | 944 | tcgtcgtcactaacagtcaaaag | 126646 | - | see also 31318 line | | |
| 31321 | FCER1A | NM_002001.2 | 661 | gagcccctcaacattactgtaat | 126660 | - | see also 13705 line | | |
| 31322 | FCER1A | NM_002001.2 | 571 | aaccacaacatctccattacaaa | 126657 | - | see also 13705 line | | |
| 31323 | FCER1G | NM_004106.1 | 174 | tgcgaaaggcagctataaccagc | 126671 | - | see also 13706 line | | |
| 31324 | FCER1G | NM_004106.1 | 171 | aagtgcgaaaggcagctataacc | 126670 | - | see also 13706 line | | |
| 31325 | FCER2 | NM_002002.3 | 360 | cccggaacgtctctcaagtttcc | 126675 | - | see also 13707 line | | |
| 31326 | FCER2 | NM_002002.3 | 318 | gggacaccacacagagtctaaaa | 126673 | - | see also 13707 line | | |
| 31327 | FCER2 | NM_002002.3 | 655 | gtgcaacacgtgccctgaaaagt | 126677 | - | see also 13707 line | | |
| 31328 | LGALS3 | NM_002306.1 | 676 | cagtacaatcatcgggttaaaaa | 126694 | - | see also 13708 line | | |
| 31329 | LGALS3 | NM_002306.1 | 398 | tgcctcgcatgctgataacaatt | 126682 | - | see also 13708 line | | |
| 31330 | MS4A2 | NM_000139.2 | 518 | tagctgggggaacgggaattacc | 126705 | - | see also 13709 line | | |
| 31331 | MS4A2 | NM_000139.2 | 197 | aagtatcttcaggcagactattg | 126699 | - | see also 13709 line | | |
| 31332 | CD44 | NM_000610.2 | 597 | atggaccaattaccataactatt | 126735 | - | see also 13710 line | | |
| 31333 | CD44 | NM_000610.2 | 229 | gagcctggcgcagatcgatttga | 126720 | - | see also 13710 line | | |
| 31334 | FN1 | NM_002026.1 | 473 | acctaggcaatgcgttggtttgt | 126771 | - | see also 13711 line | | |
| 31335 | FN1 | NM_002026.1 | 478 | ggcaatgcgttggtttgtacttg | 126773 | - | see also 13711 line | | |
| 31336 | GP6 | NM_016363.3 | 851 | ctcggggctgtgatcctaataat | 126906 | - | see also 13712 line | | |
| 31337 | GP6 | NM_016363.3 | 341 | ctcgttgccacgggagtttttgc | 126897 | - | see also 13712 line | | |

Figure 46

| | | | | | | | | |
|---|---|---|---|---|---|---|---|---|
| 31338 | GP6 | NM_016363.3 | 709 | cgctgaactgacgtctcattca | | 126901 | - | see also 13712 line |
| 31339 | ITGA10 | NM_003637.3 | 278 | gaccgggagggggacgtttatcg | | 126909 | - | see also 13713 line |
| 31340 | ITGA10 | NM_003637.3 | 808 | gactgcccaagcaataatggtgg | | 126914 | - | see also 13713 line |
| 31341 | ITGA11 | NM_012211.2 | 2161 | atggatgagaggcggtatacacc | | 126979 | - | see also 5198 line |
| 31342 | ITGA11 | NM_012211.2 | 3161 | tagcgaacagtcctgtaacatc | | 126986 | - | see also 5198 line |
| 31343 | ITGA2 | NM_002203.2 | 266 | acegaatgggagatgtgtataaa | | 126998 | - | see also 13715 line |
| 31344 | ITGA2 | NM_002203.2 | 1036 | atcgctagtattccaacagaaag | | 127027 | - | see also 13715 line |
| 31345 | NID2 | NM_007361.2 | 1657 | accgaggtcttcacgtataatgc | | 127116 | - | see also 5092 line |
| 31346 | NID2 | NM_007361.2 | 3657 | ctccaataatgggaattgatt | | 127154 | - | see also 5092 line |
| 31347 | PCOLCE | NM_002593.2 | 406 | cagcggctcggacgctttgtgg | | 127178 | - | see also 13717 line |
| 31348 | PCOLCE | NM_002593.2 | 1152 | tgccgtgactgcagtcttattg | | 127184 | - | see also 13717 line |
| 31349 | SERPINH1 | NM_001235.2 | 1284 | tccaacgccacgcctttgagttg | | 127199 | - | see also 13718 line |
| 31350 | SERPINH1 | NM_001235.2 | 1418 | agcggctccctgctattcattgg | | 127202 | - | see also 13718 line |
| 31351 | SPARC | NM_003118.1 | 487 | gactacatcgggcctttgcaaata | | 127205 | - | see also 2097 line |
| 31352 | A2M | NM_000014.3 | 1882 | ctgggtcctcggtttacaacc | | 127240 | - | see also 1 line |
| 31353 | A2M | NM_000014.3 | 1652 | gtcgctcggttgctcatctatgc | | 127239 | - | see also 1 line |
| 31354 | CGI-100 | NM_016040.2 | 655 | agctcgtgatcgaaacataacaag | | 127286 | - | see also 6635 line |
| 31355 | CGI-100 | NM_016040.2 | 290 | atcgagtaccaagtttagatgg | | 127281 | - | see also 6635 line |
| 31356 | CGI-100 | NM_016040.2 | 701 | ttctggtctatggttaatttagt | | 127287 | - | see also 6635 line |
| 31357 | CGI-109 | NM_181836.3 | 679 | ctgaagtcgtcatcgattatca | | 127290 | - | see also 13721 line |
| 31358 | CGI-109 | NM_181836.3 | 698 | atcagactcatttccgtttaaga | | 127291 | - | see also 13721 line |
| 31359 | HSGP25L2G | NM_017510.3 | 102 | gcgcggaagcggcgctcactttc | | 127298 | - | see also 7016 line |
| 31360 | MGC23911 | NM_144676.1 | 139 | ttccgtggagctgatcgatatga | | 127305 | - | see also 13724 line |
| 31361 | MGC23911 | NM_144676.1 | 559 | atgtggcgatactacaactttgc | | 127319 | - | see also 13724 line |
| 31362 | P24B | NM_007364.1 | 425 | ctctcacaagacgctcactttg | | 127335 | - | see also 13725 line |
| 31363 | P24B | NM_007364.1 | 378 | gtcaagggcgtttatcagtttg | | 127333 | - | see also 13725 line |
| 31364 | RNP24 | NM_006815.2 | 71 | caeggtctcggctatttcgtta | | 127340 | - | see also 13726 line |
| 31365 | RNP24 | NM_006815.2 | 72 | acggtctgggctatttcgttag | | 127341 | - | see also 13726 line |
| 31366 | TMP21 | NM_006827.4 | 399 | gtggaggcgaaaaattacgaaga | | 127354 | - | see also 13727 line |
| 31367 | TMP21 | NM_006827.4 | 554 | aacaaacactcgggtcctatact | | 127359 | - | see also 13727 line |
| 31368 | TMP21 | NM_006827.4 | 404 | ggcgaaaaattacgaagagattg | | 127355 | - | see also 13727 line |
| 31369 | HPS4 | NM_022081.4 | 1106 | caccagtgatctgcataagatt | | 127369 | - | see also 8266 line |
| 31370 | HPS4 | NM_022081.4 | 841 | tccgctgtttttgacatttct | | 127365 | - | see also 8266 line |
| 31371 | HPS4 | NM_022081.4 | 1030 | ccctagcttatgagaactgttct | | 127367 | - | see also 8266 line |

Figure 46

| 31372 | INHBB | NM_002193.1 | 1230 | ccgggtccgcctatacttcttc | 127395 | - | see also 13729 line |
|---|---|---|---|---|---|---|---|
| 31373 | INHBB | NM_002193.1 | 1228 | ctcccgggtccgcctatacttct | 127394 | - | see also 13729 line |
| 31374 | S100B | NM_006272.1 | 106 | atcgacgttttccaccaatattc | 127404 | - | see also 13730 line |
| 31375 | S100B | NM_006272.1 | 107 | tcgacgttttccaccaatattct | 127405 | - | see also 13730 line |
| 31376 | S100B | NM_006272.1 | 325 | tgccacgagttctttgaacatga | 127409 | - | see also 13730 line |
| 31377 | IPO7 | NM_006391.1 | 856 | atgatcgacctgagttaccatgg | 127429 | - | see also 4478 line |
| 31378 | IPO7 | NM_006391.1 | 331 | caggggatatatcccttatact | 127410 | - | see also 4478 line |
| 31379 | IPO8 | NM_006390.1 | 977 | agctcggctctttgaacgatatg | 127526 | - | see also 13732 line |
| 31380 | IPO8 | NM_006390.1 | 2073 | atctgtctacggatcattgatct | 127558 | - | see also 13732 line |
| 31381 | KPNB3 | NM_002271.3 | 749 | acccgtcgatcaggacgttatct | 127601 | - | see also 1466 line |
| 31382 | KPNB3 | NM_002271.3 | 2659 | caggtcgaagagtcactacaaga | 127637 | - | see also 1466 line |
| 31383 | RANBP2 | NM_006267.3 | 9357 | agggcggataactatggaattat | 127748 | - | see also 4359 line |
| 31384 | RANBP2 | NM_006267.3 | 8980 | gagcgcggtgttggagatataaa | 127734 | - | see also 4359 line |
| 31385 | RANBP2 | NM_006267.3 | 3500 | ttcggcgaagtgatgatatgttt | 127672 | - | see also 4359 line |
| 31386 | RANBP3 | NM_003624.1 | 880 | cgccaaccgcaacgaactatttc | 127764 | - | see also 13735 line |
| 31387 | RANBP3 | NM_003624.1 | 884 | aaccgcaacgaactatttcctcc | 127765 | - | see also 13735 line |
| 31388 | PLDN | NM_012388.2 | 293 | gggagccgacgcctggtttaagt | 127781 | - | see also 13737 line |
| 31389 | PLDN | NM_012388.2 | 297 | gccgacgcctggtttaagtgaca | 127783 | - | see also 13737 line |
| 31390 | STXBP2 | NM_006949.1 | 270 | gtcggttcaggccctgatcaaag | 127795 | - | see also 13738 line |
| 31391 | STXBP2 | NM_006949.1 | 304 | acccgactttcacctacaaagc | 127797 | - | see also 13738 line |
| 31392 | SNAPAP | NM_012437.3 | 286 | ggcgacgcgttgtcttggttaac | 127816 | - | see also 13739 line |
| 31393 | SNAPAP | NM_012437.3 | 247 | tggatcttgacccctatgttaag | 127814 | - | see also 13739 line |
| 31394 | NARF | NM_012336.2 | 126 | cacgcgcaaggaatgtagtaaga | 127821 | - | see also 5297 line |
| 31395 | NARF | NM_012336.2 | 124 | tgcacgcgcaaggaatgtagtaa | 127820 | - | see also 5297 line |
| 31396 | NARF | NM_012336.2 | 128 | cgcgcaaggaatgtagtaagaaa | 127822 | - | see also 5297 line |
| 31397 | TMPO | NM_003276.1 | 1609 | ctcactaccttaggtctagaagt | 127873 | - | see also 13741 line |
| 31398 | TMPO | NM_003276.1 | 223 | gacccctcggtcctgacaaaaga | 127841 | - | see also 13741 line |
| 31399 | NLGN2 | NM_020795.2 | 1831 | gccacgcgtgcgtgacaactacc | 127892 | - | see also 13742 line |
| 31400 | NLGN2 | NM_020795.2 | 525 | aggacggtccgctcacaaaaaaa | 127882 | - | see also 13742 line |
| 31401 | NLGN2 | NM_020795.2 | 524 | gaggacggtccgctcacaaaaaa | 127881 | - | see also 13742 line |
| 31402 | TNFRSF10A | NM_003844.2 | 1287 | ttgtatgcaatgctgatgaaatg | 127904 | - | see also 13743 line |
| 31403 | TNFRSF10A | NM_003844.2 | 640 | caggaactttccggaatgacaat | 127896 | - | see also 13743 line |
| 31404 | TNFRSF10B | NM_003842.3 | 945 | tgcagccgtagtcttgattgtgg | 127913 | - | see also 13744 line |

Figure 46

| | | | | | | | |
|---|---|---|---|---|---|---|---|
| 31405 | TNFRSF10B | NM_003842.3 | 778 | gtcaaggtcggtgattgtacacc | 127911 | - | see also 13744 line |
| 31406 | TNFRSF10B | NM_003842.3 | 939 | cacagttgcagccgtagtcttga | 127912 | - | see also 13744 line |
| 31407 | DCTN6 | NM_006571.2 | 462 | tggggcttgttgcaacctaaata | 127931 | - | see also 13745 line |
| 31408 | DCTN6 | NM_006571.2 | 238 | ggcgaagggaacctaatagaaga | 127920 | - | see also 13745 line |
| 31409 | GGA2 | NM_015044.2 | 378 | agcgaggtggccaaatttcgttt | 127940 | - | see also 13746 line |
| 31410 | GGA2 | NM_015044.2 | 709 | ctgcaaaccggttaatcaagaat | 127949 | - | see also 13746 line |
| 31411 | GGA3 | NM_014001.2 | 554 | tgcacacgttagaggaagttaac | 127970 | - | see also 13747 line |
| 31412 | GGA3 | NM_014001.2 | 1700 | ttcctgtgacagcctacgataaa | 127985 | - | see also 13747 line |
| 31413 | GGA3 | NM_014001.2 | 1703 | ctgtgacagcctacgataaaaac | 127987 | - | see also 13747 line |
| 31414 | APC | NM_000038.2 | 2442 | gacaccaatcgacatgatgataa | 128062 | - | see also 7 line |
| 31415 | APC | NM_000038.2 | 8175 | atgcgtaccgtgggtttggaaaa | 128227 | - | see also 7 line |
| 31416 | APCL | NM_005883.1 | 4690 | atcaagctgtcgcctacctatca | 128247 | - | see also 13749 line |
| 31417 | APCL | NM_005883.1 | 7077 | ccggggacgaacagtgatctacg | 128252 | - | see also 13749 line |
| 31418 | PKP1 | NM_000299.1 | 326 | tgccgtcggaccaaaagatgaaa | 128259 | - | see also 263 line |
| 31419 | PKP1 | NM_000299.1 | 445 | tccaatcgaggttccatgtatga | 128262 | - | see also 263 line |
| 31420 | PKP1 | NM_000299.1 | 276 | cgccttggcgtacgaatgcttcc | 128257 | - | see also 263 line |
| 31421 | FRZB | NM_001463.2 | 1053 | aaggatcgactcggtaaaaaagt | 128287 | - | see also 13751 line |
| 31422 | FRZB | NM_001463.2 | 1023 | ttggtggaaggctctatagctga | 128286 | - | see also 13751 line |
| 31423 | GFR | NM_012294.1 | 662 | caccgtcgtcacactgtagatga | 128306 | - | see also 5262 line |
| 31424 | GFR | NM_012294.1 | 666 | gtcgtcacactgtagatgaatat | 128308 | - | see also 5262 line |
| 31425 | CSE1L | NM_001316.2 | 393 | gccgatcgagtggccattaaagc | 128329 | - | see also 869 line |
| 31426 | CSE1L | NM_001316.2 | 582 | ctccgtacagcacattcattatt | 128336 | - | see also 869 line |
| 31427 | RBX1 | NM_014248.2 | 69 | gggcaagaagcgctttgaagtga | 128419 | - | see also 13754 line |
| 31428 | RBX1 | NM_014248.2 | 73 | aagaagcgctttgaagtgaaaaa | 128420 | - | see also 13754 line |
| 31429 | AB026190 | NM_014458.3 | 834 | tccagtgatgagctaaacgttcg | 128452 | - | see also 5686 line |
| 31430 | AB026190 | NM_014458.3 | 195 | atgcgcaggtgtaccaacattcg | 128426 | - | see also 5686 line |
| 31431 | ABTB1 | NM_032548.2 | 1134 | ccccgaggcagcctatgatgtgc | 128491 | - | see also 9242 line |
| 31432 | ABTB1 | NM_032548.2 | 969 | ctgtggccgcagtgactacttcc | 128488 | - | see also 9242 line |
| 31433 | ABTB2 | NM_145804.1 | 1235 | gggtatgacgccactgatgtacg | 128506 | - | see also 13757 line |
| 31434 | ABTB2 | NM_145804.1 | 820 | ccctccacacgctctactactttt | 128500 | - | see also 13757 line |
| 31435 | AKAP10 | NM_007202.2 | 429 | atggaggccgctcattttggtga | 128524 | - | see also 5002 line |
| 31436 | AKAP10 | NM_007202.2 | 392 | tagcaggacagccacacttaaga | 128521 | - | see also 5002 line |
| 31437 | AKAP9 | NM_005751.3 | 1456 | agccagttcgaaactgatatagt | 128589 | - | see also 13759 line |
| 31438 | AKAP9 | NM_005751.3 | 4376 | ctgttgattcggtggtaattaca | 128644 | - | see also 13759 line |
| 31439 | AMFR | NM_001144.3 | 975 | gaggtgcaacgtcgaattcgtcg | 128832 | - | see also 13760 line |

Figure 46

| 31440 | AMFR | NM_001144.3 | 1834 | tccagcaagctcgcaaacgtttc | 128851 | - | see also 13760 line |
|---|---|---|---|---|---|---|---|
| 31441 | ANKFY1 | NM_016376.2 | 807 | ctcacgacgactggagagtattg | 128870 | - | see also 13761 line |
| 31442 | ANKFY1 | NM_016376.2 | 630 | gagcgctcagttgttatacaaaa | 128864 | - | see also 13761 line |
| 31443 | ANKFY1 | NM_016376.2 | 631 | agcgctcagttgttatacaaaat | 128865 | - | see also 13761 line |
| 31444 | ANXA11 | NM_001157.2 | 1685 | cacgacatctcgggagatacttc | 128933 | - | see also 13762 line |
| 31445 | ANXA11 | NM_001157.2 | 967 | gacggcttacggcaaggatttga | 128925 | - | see also 13762 line |
| 31446 | APBA2 | NM_005503.2 | 1288 | acctcatcgacgggatcatcttt | 128942 | - | see also 3845 line |
| 31447 | APBA2 | NM_005503.2 | 1290 | ctcatcgacgggatcatctttgc | 128943 | - | see also 3845 line |
| 31448 | APBA3 | NM_004886.2 | 1811 | ggcctatggcgaggtgcatatca | 128956 | - | see also 13764 line |
| 31449 | APBA3 | NM_004886.2 | 1287 | ggcgcctgccacctccataatgg | 128954 | - | see also 13764 line |
| 31450 | APBA3 | NM_004886.2 | 1728 | cgcgtcggccaccgcatcattga | 128955 | - | see also 13764 line |
| 31451 | APBB3 | NM_006051.2 | 662 | ctggtatggtggggaatcctaca | 128959 | - | see also 13765 line |
| 31452 | APBB3 | NM_006051.2 | 1306 | tgctgaacgaggccattggtacc | 128963 | - | see also 13765 line |
| 31453 | APP | NM_000484.1 | 1604 | ggcctcgtcacgtgttcaatatg | 128991 | - | see also 377 line |
| 31454 | APP | NM_000484.1 | 1603 | cggcctcgtcacgtgttcaatat | 128990 | - | see also 377 line |
| 31455 | ARHGDIA | NM_004309.3 | 438 | ttccgggttaaccgagagatagt | 129087 | - | see also 2884 line |
| 31456 | ARHGDIA | NM_004309.3 | 410 | gggtgtggagtaccggataaaaa | 129084 | - | see also 2884 line |
| 31457 | ASE-1 | NM_012099.1 | 1225 | tcccgtccaccaccaagaagagg | 129093 | - | see also 13769 line |
| 31458 | ASE-1 | NM_012099.1 | 1738 | ctcccacatccaccaagaagaag | 129095 | - | see also 13769 line |
| 31459 | AVEN | NM_020371.1 | 403 | ctgggatcgatatcaagatattg | 129100 | - | see also 13770 line |
| 31460 | AVEN | NM_020371.1 | 397 | ctctaactgggatcgatatcaag | 129098 | - | see also 13770 line |
| 31461 | BAG1 | NM_004323.2 | 698 | tggttgccgggtcatgttaattg | 129119 | - | see also 2894 line |
| 31462 | BAG2 | NM_004282.2 | 436 | accgttgaagtgtcagtagaaac | 129126 | - | see also 13772 line |
| 31463 | BAG2 | NM_004282.2 | 616 | cagaagtttcaatccatagtaat | 129132 | - | see also 13772 line |
| 31464 | BAG3 | NM_004281.2 | 1219 | cccatgacccatcgagaaactgc | 129136 | - | see also 2869 line |
| 31465 | BAG3 | NM_004281.2 | 1542 | agccgaggctcccccaaaacatc | 129138 | - | see also 2869 line |
| 31466 | BAG4 | NM_004874.2 | 729 | ctcatactcaggggcttattatg | 129150 | - | see also 3387 line |
| 31467 | BAG4 | NM_004874.2 | 1076 | cacccggcaatctctacatgact | 129157 | - | see also 3387 line |
| 31468 | BAG5 | NM_004873.1 | 694 | tgcggtgcaagagataatcgaag | 129194 | - | see also 13774 line |
| 31469 | BAG5 | NM_004873.1 | 1389 | atcgaaccgataagaactacatc | 129207 | - | see also 13774 line |
| 31470 | BAI1 | NM_001702.1 | 3408 | agggtacagcaccatgaactact | 129223 | - | see also 1114 line |
| 31471 | BAI1 | NM_001702.1 | 3407 | aagggtacagcaccatgaactac | 129222 | - | see also 1114 line |
| 31472 | BCL10 | NM_003921.2 | 1375 | cccttaagatcacgtactgtttc | 129245 | - | see also 13776 line |
| 31473 | BCL10 | NM_003921.2 | 760 | gacgccttagaaaatttacgtgt | 129226 | - | see also 13776 line |
| 31474 | BCL2L10 | NM_020396.2 | 219 | ggcagattcaccggtccttttc | 129246 | - | see also 13777 line |
| 31475 | BCL2L10 | NM_020396.2 | 620 | ttgttaacaacagccttcattta | 129249 | - | see also 13777 line |
| 31476 | BCL6B | NM_181844.2 | 1227 | acgtgcggctcgcgctttgtaca | 129259 | - | see also 10257 line |

Figure 46

| | | | | | | | |
|---|---|---|---|---|---|---|---|
| 31477 | BCL6B | NM_181844.2 | 654 | aagtacatcgtgctaaactctca | - | 129254 | see also 10257 line |
| 31478 | BTBD1 | NM_025238.1 | 1121 | gacgagtgatcgaatcagattca | - | 129284 | see also 13779 line |
| 31479 | BTBD1 | NM_025238.1 | 1013 | cctaaacccgagttgaataca | - | 129280 | see also 13779 line |
| 31480 | BTBD11 | NM_152322.1 | 751 | gacgttggcatcagaattcagt | - | 129306 | see also 13780 line |
| 31481 | BTBD11 | NM_152322.1 | 350 | ctctccagcaagccgacaaatga | - | 129296 | see also 13780 line |
| 31482 | BTBD12 | NM_032444.1 | 2660 | tgctaatcgaagaagaacttgc | - | 129337 | see also 13781 line |
| 31483 | BTBD12 | NM_032444.1 | 1108 | cgcacccgtccgcttcctattg | - | 129321 | see also 13781 line |
| 31484 | BTBD14A | NM_144653.2 | 1671 | aactgacgccgtgaatgttgacc | - | 129346 | see also 9726 line |
| 31485 | BTBD14A | NM_144653.2 | 486 | cggcaccgacctcatgttcaagg | - | 129341 | see also 9726 line |
| 31486 | BTBD14B | NM_052876.2 | 1191 | gtcttcccggctgaacttatca | - | 129349 | see also 13783 line |
| 31487 | BTBD14B | NM_052876.2 | 1475 | tgctccacgtgtcaagtactac | - | 129351 | see also 13783 line |
| 31488 | BTBD14B | NM_052876.2 | 1058 | accggcagatcgcaacatgtac | - | 129347 | see also 13783 line |
| 31489 | BTBD2 | NM_017797.2 | 1245 | caccgactaccaagtgaacatcc | - | 129362 | see also 13784 line |
| 31490 | BTBD2 | NM_017797.2 | 1062 | gccacgagtggagttcattgaac | - | 129360 | see also 13784 line |
| 31491 | BTBD3 | NM_014962.2 | 1368 | tccggggtattaactctcaatga | - | 129390 | see also 13785 line |
| 31492 | BTBD3 | NM_014962.2 | 1328 | cacaatggccctgatgatttg | - | 129385 | see also 13785 line |
| 31493 | BTBD5 | NM_017658.1 | 738 | ttcgttgatcgcacftgtaaa | - | 129428 | see also 7085 line |
| 31494 | BTBD5 | NM_017658.1 | 166 | tgccagcgtcagccgtatttca | - | 129405 | see also 7085 line |
| 31495 | BTBD6 | NM_033271.1 | 397 | gtcggcagctccgtcttctatgc | - | 129434 | see also 13787 line |
| 31496 | BTBD6 | NM_033271.1 | 1336 | caccggtccaggttgaacaaga | - | 129438 | see also 13787 line |
| 31497 | BTBD6 | NM_033271.1 | 433 | gacctggcggaagtcaaatctga | - | 129435 | see also 13787 line |
| 31498 | BTBD9 | NM_152733.1 | 1135 | accggatagccgtcttactca | - | 129470 | see also 9935 line |
| 31499 | BTBD9 | NM_152733.1 | 1066 | gccgttccggcatcgagattaag | - | 129466 | see also 9935 line |
| 31500 | C16orf44 | NM_024731.2 | 181 | tacaagatcagcgaatcatcaaa | - | 129505 | see also 13789 line |
| 31501 | C16orf44 | NM_024731.2 | 165 | gcgagtgtctcggccatacaaga | - | 129503 | see also 13789 line |
| 31502 | C3IP1 | NM_021633.2 | 862 | atgtgcggatgcccctactaacc | - | 129529 | see also 8156 line |
| 31503 | C3IP1 | NM_021633.2 | 1143 | atcactcgttaagagacgttatgt | - | 129537 | see also 8156 line |
| 31504 | C6orf46 | NM_181842.1 | 227 | cgtcaacgctacggaacatgaacc | - | 129547 | see also 10256 line |
| 31505 | C6orf46 | NM_181842.1 | 226 | ccgcaacgctacggaacatgaac | - | 129546 | see also 10256 line |
| 31506 | CALB1 | NM_004929.2 | 611 | agccgagtatacagacctaatgc | - | 129564 | see also 3428 line |
| 31507 | CALB1 | NM_004929.2 | 622 | cagacctaatgctgaaaactattt | - | 129565 | see also 3428 line |
| 31508 | CALM1 | NM_006888.2 | 519 | tacgtcacgtcatgacaaacta | - | 129581 | see also 13793 line |
| 31509 | CALM1 | NM_006888.2 | 516 | aactacgtcacgtcatgacaaac | - | 129580 | see also 13793 line |
| 31510 | CARD10 | NM_014550.2 | 1854 | accagatggactgtcgttttatg | - | 129593 | see also 5752 line |
| 31511 | CARD10 | NM_014550.2 | 1853 | gaccagatggactgtcgttttat | - | 129592 | see also 5752 line |
| 31512 | CARD11 | NM_032415.2 | 3197 | gcctggtacggcgccttctactgc | - | 129625 | see also 9185 line |
| 31513 | CARD11 | NM_032415.2 | 3396 | ccccaacgcgttcgaatgcatcg | - | 129627 | see also 9185 line |

Figure 46

| 31514 | CARD11 | NM_032415.2 | 2086 | ggcgggtttgacgccttagatct | 129617 | - | see also 9185 line | | |
|--------|--------|-------------|------|-------------------------|--------|---|--------------------|---|---|
| 31515 | CARD14 | NM_024110.2 | 1911 | ggcgttccagggggatgcattgc | 129637 | - | see also 13796 line | | |
| 31516 | CARD14 | NM_024110.2 | 1750 | ggcgacccacacctggattatga | 129636 | - | see also 13796 line | | |
| 31517 | CBLB | NM_170662.2 | 2654 | gacgccctctgattatgatcttc | 129708 | - | see also 13797 line | | |
| 31518 | CBLB | NM_170662.2 | 2456 | tcctgttcggtcttgtgataatg | 129699 | - | see also 13797 line | | |
| 31519 | CBLC | NM_012116.2 | 1223 | gggctgggaggccgtgagtatct | 129725 | - | see also 13798 line | | |
| 31520 | CBLC | NM_012116.2 | 701 | atggccaacactcctcaagaact | 129723 | - | see also 13798 line | | |
| 31521 | CCIN | NM_005893.1 | 1078 | ctctgctggtcgctacatctaca | 129757 | - | see also 13799 line | | |
| 31522 | CCIN | NM_005893.1 | 651 | gccgcctgctccgtgatgaaaac | 129742 | - | see also 13799 line | | |
| 31523 | CD2 | NM_001767.2 | 473 | cccgaattaaacctgtatcaaga | 129793 | - | see also 13800 line | | |
| 31524 | CD2 | NM_001767.2 | 307 | gaccgatgatcaggatatctaca | 129786 | - | see also 13800 line | | |
| 31525 | CD244 | NM_016382.1 | 968 | gacaatttacgaagatgtcaagg | 129821 | - | see also 6846 line | | |
| 31526 | CD244 | NM_016382.1 | 498 | ctggaggtcaccagtatatctgg | 129810 | - | see also 6846 line | | |
| 31527 | CD28 | NM_006139.1 | 715 | tgctatagcttgctagtaacagt | 129840 | - | see also 13802 line | | |
| 31528 | CD28 | NM_006139.1 | 339 | ctgcaagtattcctacaatctct | 129833 | - | see also 13802 line | | |
| 31529 | CD3Z | NM_000734.2 | 163 | ggcacagttgccgattacagagg | 129844 | - | see also 13803 line | | |
| 31530 | CD3Z | NM_000734.2 | 321 | agggccagaaccagctctataac | 129845 | - | see also 13803 line | | |
| 31531 | CD48 | NM_001778.2 | 305 | tggcgcactgtacatctctaagg | 129857 | - | see also 13804 line | | |
| 31532 | CD48 | NM_001778.2 | 685 | cccggtcctttggagtagaatgg | 129865 | - | see also 13804 line | | |
| 31533 | CD58 | NM_001779.1 | 458 | gccatcgaggacttataatgtac | 129885 | - | see also 13805 line | | |
| 31534 | CD58 | NM_001779.1 | 500 | agcaatgtaaacgtaactcaacc | 129889 | - | see also 13805 line | | |
| 31535 | CD8A | NM_001768.4 | 747 | aaccgaagacgtgtttgcaaatg | 129898 | - | receptor_acitivity、signal_transduction、kinase_related | - | diabetes |
| 31536 | CD8A | NM_001768.4 | 748 | accgaagacgtgtttgcaaatgt | 129899 | - | see also 31535 line | | |
| 31537 | CD8B1 | NM_004931.2 | 338 | aagccggttcattctcaatctca | 129904 | - | see also 13806 line | | |
| 31538 | CD8B1 | NM_004931.2 | 334 | atgcaagccggttcattctcaat | 129903 | - | see also 13806 line | | |
| 31539 | CDC37 | NM_007065.3 | 748 | accagacaatcgtcatgcaattt | 129918 | - | see also 13807 line | | |
| 31540 | CDC37 | NM_007065.3 | 1217 | tcccaagacgggcgatgagaagg | 129925 | - | see also 13807 line | | |
| 31541 | CHC1L | NM_001268.2 | 1631 | ttcgtcattggaagataacgagg | 129950 | - | see also 828 line | | |
| 31542 | CHC1L | NM_001268.2 | 615 | gacgtccagagcaccattgaacc | 129926 | - | see also 828 line | | |
| 31543 | CIB1 | NM_006384.2 | 368 | cagccacgccagacatcaagtcc | 129968 | - | see also 13808 line | | |
| 31544 | CIB1 | NM_006384.2 | 153 | agcccacaggcggttttgtgagc | 129965 | - | see also 13808 line | | |
| 31545 | CKN1 | NM_000082.1 | 158 | tccacggcggtggaattaacacc | 129972 | - | see also 13809 line | | |
| 31546 | CKN1 | NM_000082.1 | 618 | gtctccacgttatgactatatct | 129988 | - | see also 13809 line | | |
| 31547 | CKTSF1B1 | NM_013372.4 | 589 | gtcgttgcatatccatcgatttg | 130016 | - | see also 13810 line | | |
| 31548 | CKTSF1B1 | NM_013372.4 | 590 | tcgttgcatatccatcgatttgg | 130017 | - | see also 13810 line | | |

Figure 46

| No | Symbol | Accession | Length | Sequence | ID | Function | Sign | Note |
|---|---|---|---|---|---|---|---|---|
| 31549 | CLSPN | NM_022111.2 | 3744 | gccagtcgcctatggttattca | 130088 | | - | see also 8273 line |
| 31550 | CLSPN | NM_022111.2 | 954 | gagactcagcgccttattcgaga | 130030 | | - | see also 8273 line |
| 31551 | CNK1 | NM_006314.1 | 1145 | gtcctgttggtcggaagaaatca | 130102 | | - | see also 13812 line |
| 31552 | CNK1 | NM_006314.1 | 348 | tgccaagacccctattgatgtcc | 130101 | | - | see also 13812 line |
| 31553 | CRHBP | NM_001882.2 | 94 | atgtcgcccaacttcaaacttca | 130106 | | - | see also 13813 line |
| 31554 | CRHBP | NM_001882.2 | 763 | cacgtaaatggtcttcagttaaa | 130126 | | - | see also 13813 line |
| 31555 | CRI1 | NM_014335.1 | 493 | atggcgggtttcagatgcattat | 130135 | | - | see also 13814 line |
| 31556 | CRI1 | NM_014335.1 | 523 | cccgtttgatcagttagctttt | 130138 | | - | see also 13814 line |
| 31557 | CTNNBIP1 | NM_020248.1 | 303 | tcggaggagatgtacattcagc | 130143 | signal_transduction, transcription_regulator | - | see also 13815 line |
| 31558 | DAG1 | NM_004393.1 | 1509 | ctgaggcgccattattcaaacc | 130157 | | - | see also 13815 line |
| 31559 | DAG1 | NM_004393.1 | 1507 | gactcgaggcggccattattcaaa | 130156 | | - | see also 13816 line |
| 31560 | DBT | NM_001918.1 | 1295 | ggccattcccgatttaaccaga | 130197 | | - | see also 13816 line |
| 31561 | DBT | NM_001918.1 | 1324 | aagtatataaggcacagataatg | 130198 | | - | see also 13817 line |
| 31562 | DLG5 | NM_004747.2 | 1969 | gacaggatcgttgcgatcaatgg | 130340 | | - | see also 13817 line |
| 31563 | DLG5 | NM_004747.2 | 572 | acgggtcatcgagattctcaac | 130326 | | - | see also 13818 line |
| 31564 | DLGAP2 | NM_004745.3 | 2300 | tacagcgcggtgagaactgtacg | 130392 | | - | see also 13818 line |
| 31565 | DLGAP2 | NM_004745.3 | 2138 | gagaagcggacacggacgttttaa | 130388 | | - | see also 13818 line |
| 31566 | DLGAP2 | NM_004745.3 | 2543 | aacgacctctcggaggaaattct | 130395 | | - | see also 13819 line |
| 31567 | DMN | NM_015286.4 | 2278 | aagtcagccgagcagatgatagg | 130431 | | - | see also 13819 line |
| 31568 | DMN | NM_015286.4 | 2553 | cacgactcacgtggagagaagtga | 130435 | | - | see also 13820 line |
| 31569 | DMXL1 | NM_005509.3 | 2478 | aacatcgtcagtcaacaatcaac | 130533 | | - | see also 13820 line |
| 31570 | DMXL1 | NM_005509.3 | 157 | aggcgcttcacggcttatgcatct | 130467 | | - | see also 13820 line |
| 31571 | DMXL1 | NM_005509.3 | 4914 | gtccggaataccccgccatcttacg | 130619 | | - | see also 9555 line |
| 31572 | DNCL2B | NM_130897.1 | 220 | ttggacaacttcaacaactgttca | 130775 | | - | see also 9555 line |
| 31573 | DNCL2B | NM_130897.1 | 323 | ttcttaggatcagatcaaagaaa | 130778 | | - | see also 9555 line |
| 31574 | DOK1 | NM_001381.2 | 1406 | gactgtgggctctctagagtagg | 130780 | signal_transduction, kinase_related / protein binding | - | see also 2720 line |
| 31575 | DOK2 | NM_003974.1 | 1014 | ccctcgacctgaccacatacg | 130791 | | - | see also 2720 line |
| 31576 | DPT | NM_001937.3 | 387 | tggatcgggagtggcagtttac | 130806 | | - | see also 13824 line |
| 31577 | DPT | NM_001937.3 | 65 | tgsggccagtatggcgattatgg | 130800 | | - | see also 13824 line |
| 31578 | DRE1 | NM_017644.2 | 471 | ttcgtgatagccgcttattcaca | 130830 | | - | see also 7070 line |
| 31579 | DRE1 | NM_017644.2 | 1156 | agcaagacgttaccacatacttg | 130853 | | - | see also 7070 line |
| 31580 | DRPLA | NM_001940.2 | 2115 | cccccacgtacggaaagagagc | 130898 | | - | see also 1238 line |
| 31581 | DRPLA | NM_001940.2 | 2372 | gagcgccacgcagatcaaacagg | 130901 | | - | see also 1238 line |
| 31582 | DTNA | NM_001390.2 | 1151 | agctgactaatgcattaagcaag | 130928 | | - | see also 13827 line |

Figure 46

| | | | | | | | |
|---|---|---|---|---|---|---|---|
| 31583 | DTNB | NM_021907.3 | 361 | agcctgcaaattacgattttgtac | 130961 | - | see also 8186 line |
| 31584 | DTNB | NM_021907.3 | 1255 | tcctcgccctctgactaatatga | 130994 | - | see also 8186 line |
| 31585 | EMILIN1 | NM_007046.1 | 1588 | gccacgctggaggattacaaga | 130999 | - | see also 13829 line |
| 31586 | EMILIN1 | NM_007046.1 | 2037 | ggcaaccaaggaccgtatcatt | 131002 | - | see also 13829 line |
| 31587 | EMILIN2 | NM_032048.1 | 1749 | aggagatgaacgggtcatgatgg | 131031 | - | see also 9073 line |
| 31588 | EMILIN2 | NM_032048.1 | 1662 | ggggagcgttctcctacagatga | 131030 | - | see also 9073 line |
| 31589 | ENG | NM_000118.1 | 1447 | acgccatgacccctggtactaaag | 131054 | - | see also 13831 line |
| 31590 | ENG | NM_000118.1 | 2173 | ctgcactcggtacatctactcg | 131058 | - | see also 13831 line |
| 31591 | ESPL1 | NM_012291.3 | 1050 | caccaagagggctatagacttg | 131076 | - | see also 13832 line |
| 31592 | ESPL1 | NM_012291.3 | 6296 | ctcccgactcaagtatcttatt | 131142 | - | see also 13832 line |
| 31593 | EVI5 | NM_005665.2 | 1632 | cgccacttagctcgtactactgg | 131183 | - | see also 3930 line |
| 31594 | EVI5 | NM_005665.2 | 1707 | ctgatgaccattcgacttagaga | 131184 | - | see also 3930 line |
| 31595 | FBI1 | NM_015898.2 | 579 | ctggaccttgtagatcaaattga | 131202 | - | see also 13834 line |
| 31596 | FBI1 | NM_015898.2 | 1198 | tggactactacctgaagtacttc | 131203 | - | see also 13834 line |
| 31597 | FBXL2 | NM_012157.2 | 848 | tccgcgactgcaaattttgagg | 131228 | - | see also 13835 line |
| 31598 | FBXL2 | NM_012157.2 | 843 | aactgtccgcgactgcaaattt | 131226 | - | see also 13835 line |
| 31599 | FBXL2 | NM_012157.2 | 914 | agctcggaattgccacgaattgg | 131234 | - | see also 13835 line |
| 31600 | FLJ10572 | NM_018143.1 | 471 | agccgtaatcgagtacatgtaca | 131244 | - | see also 7277 line |
| 31601 | FLJ10572 | NM_018143.1 | 544 | gccgacaggttcctactcattcg | 131247 | - | see also 7277 line |
| 31602 | FLJ11078 | NM_018316.1 | 214 | ctgatgttgtgctgactattaa | 131286 | - | see also 13837 line |
| 31603 | FLJ11078 | NM_018316.1 | 1752 | gggcatcgtacaggtgtacaaca | 131291 | - | see also 13837 line |
| 31604 | FLJ12587 | NM_022480.2 | 310 | cgcctctagccgctattttgagg | 131294 | - | see also 8402 line |
| 31605 | FLJ12587 | NM_022480.2 | 308 | gccgcctctagccgctattttga | 131293 | - | see also 8402 line |
| 31606 | FLJ12587 | NM_022480.2 | 1720 | ttccggcaacaagtctatgtgg | 131295 | - | see also 8402 line |
| 31607 | FLJ13057 | NM_178439.3 | 864 | gcagggacctatggattagatt | 131300 | - | see also 13839 line |
| 31608 | FLJ13057 | NM_178439.3 | 861 | atcagcagggacctatggattag | 131299 | - | see also 13839 line |
| 31609 | FLJ13063 | NM_024742.1 | 1679 | agccgatggcaccctgtattgt | 131305 | - | see also 8721 line |
| 31610 | FLJ13063 | NM_024742.1 | 1681 | cgcatggcaccctcgtattgtgg | 131306 | - | see also 8721 line |
| 31611 | FLJ14360 | NM_032775.2 | 1146 | tgctcaacaacttgtatacttg | 131328 | - | see also 13841 line |
| 31612 | FLJ14360 | NM_032775.2 | 390 | acggtgtcctacacaatgctatg | 131315 | - | see also 13841 line |
| 31613 | FLJ31322 | NM_152387.1 | 310 | accgtgatggacgtcatttaaa | 131343 | - | see also 9847 line |
| 31614 | FLJ31322 | NM_152387.1 | 309 | gaccgtgatggacgtctatttaa | 131342 | - | see also 9847 line |
| 31615 | FMOD | NM_002023.2 | 325 | tcgttcctccgcatgaagtat | 131362 | - | see also 13843 line |
| 31616 | FMOD | NM_002023.2 | 282 | acggccatgtactgtgacaatcg | 131361 | - | see also 13843 line |
| 31617 | FYB | NM_001465.2 | 2430 | gagttacctagcggacaatgatg | 131398 | - | see also 13844 line |
| 31618 | FYB | NM_001465.2 | 1815 | aaggggttcatatggctatatta | 131381 | - | see also 13844 line |
| 31619 | G1P2 | NM_005101.1 | 506 | ccctgagcaccgtgttcatgaat | 131402 | - | see also 13845 line |

Figure 46

| | | | | | | | |
|---|---|---|---|---|---|---|---|
| 31620 | G1P2 | NM_005101.1 | 504 | gcccctgagcaccgtgttcatga | 131401 | - | see also 13845 line |
| 31621 | GAB1 | NM_002039.1 | 2000 | tacttagatctcgacttagattc | 131445 | - | see also 13846 line |
| 31622 | GAB1 | NM_002039.1 | 281 | aagaagcctattcgtattattga | 131404 | - | see also 13846 line |
| 31623 | GAN | NM_022041.2 | 1768 | gactacgtgcgtgagtttaaaag | 131503 | - | see also 13847 line |
| 31624 | GAN | NM_022041.2 | 1644 | aaggtggatctatcttaacgacc | 131497 | - | see also 13847 line |
| 31625 | GDF5 | NM_000557.2 | 327 | tccccaaactcctcactttcttg | 131506 | - | see also 408 line |
| 31626 | GMCL | NM_022471.2 | 767 | tgcggttattacacatcagtaga | 131515 | - | see also 13849 line |
| 31627 | GMCL | NM_022471.2 | 241 | ggcgagctacggcttctgttact | 131514 | - | see also 13849 line |
| 31628 | GMRP-1 | NM_032320.3 | 980 | ttcgagcgattctggattactat | 131523 | - | see also 9154 line |
| 31629 | GRF2 | NM_005312.2 | 3225 | tgcggaggaacgacgacattata | 131590 | - | see also 13850 line |
| 31630 | GRF2 | NM_005312.2 | 1567 | gcggcatccctcgcagtatgaca | 131559 | - | see also 13850 line |
| 31631 | GTF3C2 | NM_001521.1 | 1963 | gaccttcgacgtccttacgaacc | 131621 | - | see also 1045 line |
| 31632 | GTF3C2 | NM_001521.1 | 2179 | tccgactggcttgggacaatagc | 131627 | - | see also 1045 line |
| 31633 | HAT1 | NM_003642.1 | 1015 | cacgctagaagggtttatgaaat | 131677 | - | see also 2459 line |
| 31634 | HLA-DMA | NM_006120.2 | 649 | tgcattgtgactcacgaaattga | 131697 | - | see also 13853 line |
| 31635 | HLA-DMA | NM_006120.2 | 648 | ctgcattgtgactcacgaaattg | 131696 | - | see also 13853 line |
| 31636 | HOMER1 | NM_004272.2 | 520 | ggcaaacaccgtttatggattgg | 131707 | - | see also 13854 line |
| 31637 | HOMER1 | NM_004272.2 | 690 | ctcctttaacaccggaaagtatc | 131708 | - | see also 13854 line |
| 31638 | HOMER3 | NM_004838.2 | 230 | ggcgcacgtgttccaaattgacc | 131717 | - | see also 13855 line |
| 31639 | HOMER3 | NM_004838.2 | 688 | cagagcgcgagcggctaaagaag | 131719 | - | see also 13855 line |
| 31640 | HSPB2 | NM_001541.2 | 89 | gccaccgccgagtacgaatttgc | 131725 | - | see also 13856 line |
| 31641 | HSPB2 | NM_001541.2 | 88 | ggccaccgccgagtacgaatttg | 131724 | - | see also 13856 line |
| 31642 | HUMGT198A | NM_013290.3 | 345 | tacggcaagcagaagatctattt | 131732 | - | see also 13857 line |
| 31643 | HUMGT198A | NM_013290.3 | 552 | aacattaaagcagctaccaatca | 131735 | - | see also 13857 line |
| 31644 | HUMGT198A | NM_013290.3 | 314 | ggcgcaacaaggcaagatcaaag | 131731 | - | see also 13857 line |
| 31645 | HYPE | NM_007076.1 | 1329 | ggcgacgtgaggcctttcattcg | 131743 | - | see also 13858 line |
| 31646 | HYPE | NM_007076.1 | 1348 | ttcgcttcatcgccaagtgtact | 131744 | - | see also 13858 line |
| 31647 | IFI35 | NM_005533.2 | 839 | ctcggtactggtgctcaacattc | 131748 | - | see also 13859 line |
| 31648 | IFI35 | NM_005533.2 | 601 | tggacaagctagagatcttcttt | 131746 | - | see also 13859 line |
| 31649 | INADL | NM_005799.2 | 4153 | agcggcaccgaacctattagtag | 131849 | - | see also 13860 line |
| 31650 | INADL | NM_005799.2 | 3887 | accgatcacgcatgagcatattt | 131843 | - | see also 13860 line |
| 31651 | INHA | NM_002191.2 | 1171 | acctcggatgggaggttactcttt | 131886 | - | see also 1398 line |
| 31652 | INHA | NM_002191.2 | 1173 | ctcggatgggaggttactcttca | 131887 | - | see also 1398 line |

Figure 46

| | | | | | | | |
|---|---|---|---|---|---|---|---|
| 31653 | INHBA | NM_002192.1 | 1142 | gagtgccggccgagccatatagcagg | 131901 | - | see also 13862 line |
| 31654 | INHBA | NM_002192.1 | 330 | cgcttctgaacgcgatcagaaag | 131894 | - | see also 13862 line |
| 31655 | IRS1 | NM_005544.1 | 2239 | ctcttcccacgggatctagtgc | 131910 | - | see also 13863 line |
| 31656 | IRS1 | NM_005544.1 | 2453 | ccccaacgtcactacattttg | 131916 | - | see also 13863 line |
| 31657 | IRS1 | NM_005544.1 | 1659 | ctgtggccactcggaaaacttct | 131908 | - | see also 13863 line |
| 31658 | ISLR | NM_005545.2 | 1203 | tgccaatgcagcctgcttatcc | 131935 | - | see also 3861 line |
| 31659 | ITGB1 | NM_002211.2 | 1585 | ggcgcgttgcaggtgcaatgaaag | 131955 | - | see also 13865 line |
| 31660 | ITGB1 | NM_002211.2 | 476 | agcagttgttttgcgattaaga | 131942 | - | see also 13865 line |
| 31661 | ITGB1BP2 | NM_012278.1 | 156 | tgccgaaagcgaactgtagattt | 131992 | - | see also 5253 line |
| 31662 | ITGB1BP2 | NM_012278.1 | 157 | gccgaaagcgaactgtagattttc | 131993 | - | see also 5253 line |
| 31663 | IVNS1ABP | NM_006469.2 | 993 | ctcccgtttgttgaataaggttg | 132027 | - | see also 4537 line |
| 31664 | IVNS1ABP | NM_006469.2 | 1700 | ttgaacagtcgaatgctataat | 132040 | - | see also 4537 line |
| 31665 | KBTBD3 | NM_152433.2 | 1238 | ctgcatattgccgagtcatatca | 132096 | - | see also 9863 line |
| 31666 | KBTBD3 | NM_152433.2 | 1225 | tcgaacgttcgactgcatattg | 132095 | - | see also 9863 line |
| 31667 | KBTBD4 | NM_016506.3 | 1566 | ctgtaacgggagcatctargtct | 132149 | - | see also 6881 line |
| 31668 | KBTBD4 | NM_016506.3 | 1585 | gtcttccggacgcgatataaaaa | 132151 | - | see also 6881 line |
| 31669 | KBTBD5 | NM_152393.1 | 376 | ggcgagcgtgcaggattigttcg | 132154 | - | see also 13870 line |
| 31670 | KBTBD5 | NM_152393.1 | 375 | aggcgagcgtgcagagatttgttc | 132153 | - | see also 13870 line |
| 31671 | KBTBD6 | NM_152903.1 | 1699 | cagtaagccatgttctgttatg | 132162 | - | see also 13871 line |
| 31672 | KBTBD6 | NM_152903.1 | 1703 | aagccatgttcgttatgatcc | 132163 | - | see also 13871 line |
| 31673 | KBTBD7 | NM_032138.3 | 1942 | aggcggattagtaatattccttt | 132185 | - | see also 13872 line |
| 31674 | KBTBD7 | NM_032138.3 | 1939 | tggaggcggattagttaattcc | 132184 | - | see also 13872 line |
| 31675 | KCNA1 | NM_000217.1 | 1325 | gtcgcgcagttcctctactatg | 132208 | - | see also 13873 line |
| 31676 | KCNA1 | NM_000217.1 | 1285 | cacgtcagttccctaacttagc | 132207 | - | see also 13873 line |
| 31677 | KCNA1 | NM_000217.1 | 1180 | atcgctggtgtgtaacaattgc | 132205 | - | see also 13873 line |
| 31678 | KCNA10 | NM_005549.2 | 1372 | gccgagtgcccaacagaacatgt | 132227 | - | see also 13874 line |
| 31679 | KCNA10 | NM_005549.2 | 578 | tccaagcttccgggagactatgc | 132215 | - | see also 13874 line |
| 31680 | KCNA2 | NM_004974.2 | 1374 | tccgtgtcatccggttggtaaga | 132238 | - | see also 3464 line |
| 31681 | KCNA2 | NM_004974.2 | 1383 | tccgttggtaagagtcttagg | 132240 | - | see also 3464 line |
| 31682 | KCNA2 | NM_004974.2 | 1631 | gacatggttccgactaccattgg | 132245 | - | see also 3464 line |
| 31683 | KCNA3 | NM_002232.2 | 1025 | ctcgacgtgcgaggagactcattcg | 132256 | - | see also 1425 line |
| 31684 | KCNA3 | NM_002232.2 | 1887 | acctgcaccacgaacaataatcc | 132266 | - | see also 1425 line |
| 31685 | KCNA4 | NM_002233.2 | 2309 | ttgtggttcgctgctttgcttgt | 132280 | - | see also 1427 line |
| 31686 | KCNA4 | NM_002233.2 | 2000 | ccctccccgagaatgaatttaaa | 132277 | - | see also 1427 line |
| 31687 | KCNA5 | NM_002234.2 | 588 | aggcgtcacatcaacatctcc | 132289 | - | see also 13877 line |
| 31688 | KCNA5 | NM_002234.2 | 585 | accagcgcggtccacatcaacatc | 132288 | - | see also 13877 line |
| 31689 | KCNA6 | NM_002235.2 | 2064 | gacgatgacgattcgcttttcc | 132295 | - | see also 1433 line |

1144

Figure 46

| 31690 | KCNA6 | NM_002235.2 | 988 | agcggctggtgatcaatatctcc | 132291 | - | see also 1433 line |
|---|---|---|---|---|---|---|---|
| 31691 | KCNA7 | NM_031886.2 | 1363 | accgggtggactcccatttcact | 132307 | - | see also 13879 line |
| 31692 | KCNA7 | NM_031886.2 | 1046 | ctgtccaagcaaggctatcttct | 132300 | - | see also 13879 line |
| 31693 | KCNB1 | NM_004975.2 | 1069 | tgcgaattctccgcatccttaag | 132321 | - | see also 3468 line |
| 31694 | KCNB1 | NM_004975.2 | 2167 | agctccgagcacttaaagtcaac | 132336 | - | see also 3468 line |
| 31695 | KCNB2 | NM_004770.2 | 1957 | gccttcgctcgaagtatggaact | 132384 | - | see also 13881 line |
| 31696 | KCNB2 | NM_004770.2 | 2004 | cggagagtccgccaacacaaagg | 132388 | - | see also 13881 line |
| 31697 | KCNC1 | NM_004976.2 | 213 | cccgcgtgctgacgagttcttct | 132403 | - | see also 3470 line |
| 31698 | KCNC1 | NM_004976.2 | 864 | ctgccccaacaaggtagagttca | 132407 | - | see also 3470 line |
| 31699 | KCNC2 | NM_139136.2 | 1663 | tactcttcgagctagtactaatg | 132431 | - | see also 13883 line |
| 31700 | KCNC2 | NM_139136.2 | 1668 | ttcgagctagtactaatgaattt | 132432 | - | see also 13883 line |
| 31701 | KCNC2 | NM_139136.2 | 1622 | aagctcacccgccattttgtagg | 132428 | - | see also 13883 line |
| 31702 | KCNC3 | NM_004977.2 | 553 | cagcggcaagatcgtgatcaacg | 132461 | - | see also 13884 line |
| 31703 | KCNC3 | NM_004977.2 | 1287 | gggcacctccggagaacatcacc | 132464 | - | see also 13884 line |
| 31704 | KCNC4 | NM_004978.2 | 924 | tcctccgcgtagggaacatcacc | 132471 | - | see also 3471 line |
| 31705 | KCND1 | NM_004979.3 | 1844 | caccgcagccctcgttttctact | 132483 | - | see also 13886 line |
| 31706 | KCND1 | NM_004979.3 | 1846 | ccgcagccctcgttttctactat | 132484 | - | see also 13886 line |
| 31707 | KCND1 | NM_004979.3 | 1841 | gagcaccgcagccctcgttttct | 132482 | - | see also 13886 line |
| 31708 | KCND2 | NM_012281.2 | 2426 | aaccacgaatcacgagtttgtgg | 132515 | - | see also 5255 line |
| 31709 | KCND2 | NM_012281.2 | 1718 | gcctagtcgttaccgttttgtgc | 132495 | - | see also 5255 line |
| 31710 | KCND3 | NM_004980.3 | 2115 | ctgctgctcccgtcgtagtaaga | 132535 | - | see also 3473 line |
| 31711 | KCND3 | NM_004980.3 | 1777 | gccaaaacaggcagttcgaatgc | 132529 | - | see also 3473 line |
| 31712 | KCNF1 | NM_002236.4 | 706 | cggcaagcgcgagttctactttg | 132544 | - | see also 13889 line |
| 31713 | KCNF1 | NM_002236.4 | 705 | ccggcaagcgcgagttctacttt | 132543 | - | see also 13889 line |
| 31714 | KCNG1 | NM_002237.2 | 4 | accctcttaccgggagacaattc | 132548 | - | channel - - |
| 31715 | KCNG1 | NM_002237.2 | 330 | cacctgcaacgagttcttcttcg | 132549 | - | see also 31714 line |
| 31716 | KCNG2 | NM_012283.1 | 746 | cgccactcaacatcattgacatc | 132552 | - | channel - - |
| 31717 | KCNRG | NM_173605.1 | 585 | aagccggaacactcaagctttt | 132563 | - | see also 13891 line |
| 31718 | KCNRG | NM_173605.1 | 280 | aagatattcacgacaaggtttc | 132556 | - | see also 13891 line |
| 31719 | KCNRG | NM_173605.1 | 327 | tcgtttggcacgcatgttagatg | 132559 | - | see also 13891 line |
| 31720 | KCNS1 | NM_002251.3 | 1279 | gcccagtacgcgcaacttcttct | 132582 | - | see also 13892 line |
| 31721 | KCNS1 | NM_002251.3 | 1278 | cgcccagtacgcgcaacttcttc | 132581 | - | see also 13892 line |
| 31722 | KCNS3 | NM_002252.3 | 1717 | agcggatgcacaccttcattacc | 132611 | - | see also 1448 line |
| 31723 | KCNS3 | NM_002252.3 | 1252 | tcctacggcttatgaggattttc | 132601 | - | see also 1448 line |
| 31724 | KCNV1 | NM_014379.2 | 1397 | ttctatccgtgggaatctctata | 132630 | - | see also 13894 line |
| 31725 | KCNV1 | NM_014379.2 | 1296 | ggctctgcgcatgctaaagctgg | 132624 | - | see also 13894 line |
| 31726 | KCTD10 | NM_031954.2 | 624 | aacggaagggtcctgttcataaa | 132657 | - | see also 13895 line |

Figure 46

| | | | | | | | |
|---|---|---|---|---|---|---|---|
| 31727 | KCTD10 | NM_031954.2 | 767 | tcccgaagcccggatttatgagg | 132661 | - | see also 13895 line |
| 31728 | KCTD12 | NM_138444.2 | 988 | ttggggacaccctgaacgaaagc | 132663 | - | see also 13896 line |
| 31729 | KCTD12 | NM_138444.2 | 252 | gcggacagcaacgtggattacc | 132662 | - | see also 13896 line |
| 31730 | KCTD13 | NM_178863.2 | 1150 | ctcatggccaacaaattgtcttc | 132674 | - | see also 10225 line |
| 31731 | KCTD14 | NM_023930.2 | 691 | ctgacgtacccaccaaaagaaa | 132684 | - | see also 13898 line |
| 31732 | KCTD14 | NM_023930.2 | 318 | ggctcagttctacgaaatcaagc | 132676 | - | see also 13898 line |
| 31733 | KCTD15 | NM_024076.1 | 492 | caggataagccgcctcttcaatg | 132686 | - | see also 13899 line |
| 31734 | KCTD15 | NM_024076.1 | 560 | ttgaccggatggggagatttc | 132688 | - | see also 13899 line |
| 31735 | KCTD3 | NM_016121.2 | 1407 | aagtcccgtaacaaaaatcatgc | 132726 | - | see also 13900 line |
| 31736 | KCTD3 | NM_016121.2 | 1329 | agcagtacgagtgattgtacaac | 132721 | - | see also 13900 line |
| 31737 | KCTD5 | NM_018992.1 | 711 | cccagcgagaaggccaagatttt | 132770 | - | channel |
| 31738 | KCTD5 | NM_018992.1 | 427 | aagcagaatttacaatatcacc | 132767 | - | see also 31737 line |
| 31739 | KCTD5 | NM_018992.1 | 444 | atcacctcattaataaaactgt | 132768 | - | see also 31737 line |
| 31740 | KCTD6 | NM_153331.2 | 585 | tccgatatgtcctcaacttctta | 132780 | - | see also 9986 line |
| 31741 | KCTD7 | NM_153033.1 | 916 | acctcgtgaaccactactactgc | 132808 | - | see also 9974 line |
| 31742 | KCTD7 | NM_153033.1 | 256 | ctgaggttgtcccttaacatc | 132802 | - | see also 9974 line |
| 31743 | KEAP1 | NM_012289.2 | 1547 | gacgggacaaaccgccttaattc | 132812 | - | see also 13902 line |
| 31744 | KEAP1 | NM_012289.2 | 1652 | gtcctgcacaactgtatctatgc | 132816 | - | see also 13902 line |
| 31745 | KIAA0469 | NM_014851.1 | 1584 | ctctaaacggactcatgtacttt | 132823 | - | see also 13903 line |
| 31746 | KIAA0469 | NM_014851.1 | 1648 | gaggaacgaatgggacaagatcc | 132825 | - | see also 13903 line |
| 31747 | KIAA0711 | NM_014867.1 | 2451 | ggctcctgacggcttcatctacc | 132831 | - | see also 13904 line |
| 31748 | KIAA0711 | NM_014867.1 | 2322 | cacctgccacggcgagatctacg | 132828 | - | see also 13904 line |
| 31749 | KIAA0795 | NM_025010.1 | 1156 | cacgtgcagcccgaacatacaca | 132840 | - | see also 13905 line |
| 31750 | KIAA0795 | NM_025010.1 | 633 | tgcattggcctggtcagatacg | 132832 | - | see also 13905 line |
| 31751 | KIAA1309 | NM_033495.1 | 477 | gagcaaagtcggtctaaggaaaa | 132846 | - | see also 9428 line |
| 31752 | KIAA1309 | NM_033495.1 | 1152 | ggcccatgagtggaaatcgttag | 132861 | - | see also 9428 line |
| 31753 | KIAA1354 | NM_018847.1 | 552 | tgcccattgcagccttgtaagg | 132876 | - | see also 13907 line |
| 31754 | KIAA1354 | NM_018847.1 | 607 | cacagttcggttgttgtcaagg | 132879 | - | see also 13907 line |
| 31755 | KIAA1354 | NM_018847.1 | 575 | caggaaccacacgctttttacc | 132877 | - | see also 13907 line |
| 31756 | KIAA1900 | NM_052904.1 | 1929 | accagtggacacgttgtaatttc | 132916 | - | see also 13908 line |
| 31757 | KIAA1900 | NM_052904.1 | 1889 | atccttgtgccatatagattc | 132911 | - | see also 13908 line |
| 31758 | KIP2 | NM_006383.1 | 249 | tggtgaggggaacctcacttca | 132923 | - | see also 13909 line |
| 31759 | KIP2 | NM_006383.1 | 30 | caccgaagagcagctagacaact | 132921 | - | see also 13909 line |
| 31760 | KLHL6 | NM_130446.1 | 121 | gacgcggactctccttaattct | 132930 | - | see also 13910 line |
| 31761 | KLHL6 | NM_130446.1 | 1476 | gcccgtgggggctaaatgcatca | 132955 | - | see also 13910 line |
| 31762 | KLHL8 | NM_020803.3 | 789 | gtcacaatcgaatagacttaatg | 132983 | - | see also 13911 line |
| 31763 | KLHL8 | NM_020803.3 | 1029 | tgccattgttgccggttgatttt | 132996 | - | see also 13911 line |

Figure 46

| | | | | | | | |
|---|---|---|---|---|---|---|---|
| 31764 | KNTC1 | NM_014708.2 | 348 | tagccgaccaatcagtgatatg | 133045 | - | see also 13912 line |
| 31765 | KNTC1 | NM_014708.2 | 3421 | atgcagcgacttgtttaagtatc | 133134 | - | see also 13912 line |
| 31766 | KPNA1 | NM_002264.1 | 520 | ttctcttcagaccgaattgtga | 133261 | - | see also 1450 line |
| 31767 | KPNA1 | NM_002264.1 | 65 | gagaactttcgcctgaaaagtta | 133247 | - | see also 1450 line |
| 31768 | KPNA1 | NM_002264.1 | 920 | gtcatcgatgcgggagtatgtag | 133269 | - | see also 1450 line |
| 31769 | LCP2 | NM_005565.2 | 389 | gggtgccgattctcagtaagtta | 133295 | - | see also 13914 line |
| 31770 | LCP2 | NM_005565.2 | 1068 | accaccgaccacgaaagacatg | 133302 | - | see also 13914 line |
| 31771 | LGALS3BP | NM_005567.2 | 810 | tacttctactccgaaggattga | 133318 | - | see also 13915 line |
| 31772 | LGALS3BP | NM_005567.2 | 604 | cccttggccagatctttgacagc | 133316 | - | see also 13915 line |
| 31773 | LIMD1 | NM_014240.1 | 1406 | ctcacccgaaggctgattacttt | 133334 | - | see also 13916 line |
| 31774 | LIMD1 | NM_014240.1 | 1408 | cacccgaaggctgattactttgg | 133335 | - | see also 13916 line |
| 31775 | LIN7A | NM_004664.1 | 718 | gtgcggatacaccccaaaagttct | 133356 | - | see also 13917 line |
| 31776 | LIN7A | NM_004664.1 | 356 | ttcgagaggtgatcaatatatg | 133348 | - | see also 13917 line |
| 31777 | LIN7A | NM_004664.1 | 751 | gaggctcgctttgaaaagctacg | 133357 | - | see also 13917 line |
| 31778 | LMNA | NM_005572.2 | 556 | agctgaaagcgcgcaataccaag | 133359 | - | see also 13918 line |
| 31779 | LMNA | NM_005572.2 | 1444 | gtggggcagcgtcaccaaaaag | 133360 | - | see also 13918 line |
| 31780 | LOC92799 | NM_138392.1 | 360 | gaggagttggatcgatcttcttg | 133368 | - | see also 13919 line |
| 31781 | LOC92799 | NM_138392.1 | 174 | cgcatctcgacgctgaaagatga | 133366 | - | see also 13919 line |
| 31782 | LRP6 | NM_002336.1 | 1223 | ggccatacgccgttcatttatag | 133424 | - | see also 1499 line |
| 31783 | LRP6 | NM_002336.1 | 1508 | tggttctgaccgtgtagtattgg | 133433 | - | see also 1499 line |
| 31784 | LRPAP1 | NM_002337.1 | 278 | gacgaactgcctggaagagaaact | 133555 | - | see also 13921 line |
| 31785 | LRPAP1 | NM_002337.1 | 248 | ctccacgctgatctgaagataca | 133554 | - | see also 13921 line |
| 31786 | LTBP2 | NM_000428.1 | 2285 | aggacgcggagtgtgtgaattacc | 133565 | - | see also 333 line |
| 31787 | LTBP2 | NM_000428.1 | 2176 | gcctcccggttgattgagaatgg | 133564 | - | see also 333 line |
| 31788 | LTBP4 | NM_003573.1 | 4153 | tacgagtacggcccagacttagg | 133604 | - | see also 13923 line |
| 31789 | LTBP4 | NM_003573.1 | 1630 | cagggcacccgatgcattgatgt | 133592 | - | see also 13923 line |
| 31790 | LY64 | NM_005582.1 | 184 | ttctgccggctgaaagtcatca | 133608 | - | see also 13924 line |
| 31791 | LY64 | NM_005582.1 | 1600 | cacgaagaccaacctacttcaga | 133646 | - | see also 13924 line |
| 31792 | MADH2 | NM_005901.2 | 1249 | gaggagtggcttatactacata | 133701 | - | see also 4111 line |
| 31793 | MADH2 | NM_005901.2 | 790 | ccgacacacgagatcctaaca | 133679 | - | see also 4111 line |
| 31794 | MADH6 | NM_005585.2 | 1782 | gtctcctcgcgacgagtacaagc | 133717 | - | see also 13926 line |
| 31795 | MADH6 | NM_005585.2 | 1815 | gtccgattccacattgtcttaca | 133720 | - | see also 13926 line |
| 31796 | MADH7 | NM_005904.1 | 1351 | caggacgcgttggtacacacagg | 133737 | - | see also 4119 line |
| 31797 | MADH7 | NM_005904.1 | 1431 | ggcccaatgaaccacgagtttatg | 133738 | - | see also 4119 line |
| 31798 | MALT1 | NM_006785.2 | 1354 | gtgtacgaattgacttaacttact | 133764 | - | see also 4779 line |

Figure 46

| 31799 | MALT1 | NM_006785.2 | 2120 | aaccaccggagataataatgtgt | 133790 | - | see also 4779 line |
|---|---|---|---|---|---|---|---|
| 31800 | MAP3K7IP 1 | NM_006116.1 | 569 | cggtacaaaccgtgcacttttat | 133814 | - | see also 13929 line |
| 31801 | MAP3K7IP 1 | NM_006116.1 | 576 | aaccgtgcacttttatgcaaatc | 133815 | - | see also 13929 line |
| 31802 | MEN1 | NM_000244.2 | 1055 | gacctactatcgggatgaacaca | 133828 | - | see also 13930 line |
| 31803 | MEN1 | NM_000244.2 | 1480 | ggcagaaggtgcgcatagtgagc | 133832 | - | see also 13930 line |
| 31804 | MGC10485 | NM_052875.2 | 948 | gaggactgtctgcacattgaatt | 133841 | - | see also 9440 line |
| 31805 | MGC10485 | NM_052875.2 | 949 | aggactgtctgcacattgaattt | 133842 | - | see also 9440 line |
| 31806 | MGC23166 | NM_152735.2 | 1655 | ctgcccactggacttacaagtga | 133862 | - | see also 13932 line |
| 31807 | MGC23166 | NM_152735.2 | 423 | ccgggaacttagggctcataaag | 133849 | - | see also 13932 line |
| 31808 | MGC2629 | NM_032522.2 | 719 | cccttagcccacgacataatacc | 133875 | - | see also 9234 line |
| 31809 | MGC2629 | NM_032522.2 | 717 | ctcccttagcccacgacataata | 133873 | - | see also 9234 line |
| 31810 | MICB | NM_005931.2 | 36 | gccgtcgccttcccttttgcacc | 133888 | - | see also 13934 line |
| 31811 | MICB | NM_005931.2 | 915 | cagagtcaacggacagactttcc | 133891 | - | see also 13934 line |
| 31812 | MID1 | NM_000381.1 | 2020 | aacggctctatcgccttttatga | 133929 | - | see also 303 line |
| 31813 | MID1 | NM_000381.1 | 1462 | aacgtcgttagtctgtgtaattc | 133920 | - | see also 303 line |
| 31814 | MPDZ | NM_003829.1 | 4600 | cacggatggacgactcaaagtcg | 134062 | - | see also 13936 line |
| 31815 | MPDZ | NM_003829.1 | 4598 | gccacggatggacgactcaaagt | 134061 | - | see also 13936 line |
| 31816 | MPDZ | NM_003829.1 | 4836 | aaccggagtccatccgaaataca | 134066 | - | see also 13936 line |
| 31817 | MPP2 | NM_005374.2 | 1354 | aacctgtatggcacacgtattga | 134112 | - | see also 13937 line |
| 31818 | MPP2 | NM_005374.2 | 1076 | accgtcatgagctgctcatttat | 134108 | - | see also 13937 line |
| 31819 | MPP6 | NM_016447.2 | 771 | ctccatgggggaatgatagatcg | 134127 | - | see also 13938 line |
| 31820 | MPP6 | NM_016447.2 | 979 | atccatacaatgacaacctaata | 134131 | - | see also 13938 line |
| 31821 | MPZL1 | NM_003953.3 | 811 | tccgacactgagggtcttgtaaa | 134157 | - | see also 13939 line |
| 31822 | MPZL1 | NM_003953.3 | 771 | gagtttgtcaccagttaagcagg | 134156 | - | see also 13939 line |
| 31823 | MTPN | NM_145808.1 | 334 | aggaaacctcttcattatgcagc | 134159 | - | see also 13940 line |
| 31824 | NMNAT1 | NM_022787.2 | 329 | tgcctatcaccgggtcatcatgg | 134163 | - | see also 13941 line |
| 31825 | NMNAT1 | NM_022787.2 | 207 | aacatgcacctcaggttgtttga | 134161 | - | see also 13941 line |
| 31826 | NPHP1 | NM_000272.1 | 840 | ttgctacctaatagttctgatgc | 134191 | - | see also 13942 line |
| 31827 | NPHP1 | NM_000272.1 | 590 | ctgatggttggtggatagctaag | 134184 | - | see also 13942 line |
| 31828 | NPHP4 | NM_015102.2 | 1517 | ctcgcactgtctggtctacaagg | 134243 | - | see also 6242 line |
| 31829 | NPHP4 | NM_015102.2 | 3552 | tccagcgcagtgcccactaaaca | 134265 | - | see also 6242 line |
| 31830 | NUDT5 | NM_014142.2 | 424 | gccaagaaccaacggaatcttct | 134274 | - | see also 13944 line |

Figure 46

| 31831 | NUDT5 | NM_014142.2 | 818 | aggcttgtcaaactgtactatac | 134288 | - | see also 13944 line | | |
|--------|-------|-------------|------|------------------------|--------|---|----------------------|--|--|
| 31832 | OGT | NM_003605.3 | 544 | acctgatttcatcgatggttata | 134300 | - | see also 2417 line | | |
| 31833 | OIP106 | NM_014965.1 | 1076 | tgctatcgcaaatagttgatttg | 134425 | - | see also 13946 line | | |
| 31834 | OIP106 | NM_014965.1 | 1072 | cacctgctatcgcaaatagttga | 134423 | - | see also 13946 line | | |
| 31835 | OIP5 | NM_007280.1 | 143 | ttgaccaagcttcttttacgacc | 134443 | - | see also 13947 line | | |
| 31836 | OIP5 | NM_007280.1 | 487 | cccgttggtttccatctgtattc | 134456 | - | see also 13947 line | | |
| 31837 | OIP5 | NM_007280.1 | 378 | ctccagagttacaaataacgtcg | 134446 | - | see also 13947 line | | |
| 31838 | OSTF1 | NM_012383.3 | 198 | ttcagagccctgtatacgtttga | 134465 | - | see also 5304 line | | |
| 31839 | OSTF1 | NM_012383.3 | 272 | tactgacatgagcgataccaatt | 134469 | - | see also 5304 line | | |
| 31840 | P29 | NM_015484.1 | 605 | agccggagacgtccttataatga | 134493 | - | see also 13949 line | | |
| 31841 | P29 | NM_015484.1 | 630 | atgcagatatcgactacattaat | 134497 | - | see also 13949 line | | |
| 31842 | PACSIN1 | NM_020804.1 | 453 | gcctggggtgccataatgacaga | 134501 | - | kinase_related | protein kinase | - |
| 31843 | PACSIN1 | NM_020804.1 | 417 | atcgagaaaggcccacagtatgg | 134500 | - | see also 31842 line | | |
| 31844 | PACSIN1 | NM_020804.1 | 681 | gcctaccatttggcttgcaaaga | 134502 | - | see also 31842 line | | |
| 31845 | PDHX | NM_003477.1 | 193 | ttcggggtgatcccattaagata | 134504 | - | see also 2351 line | | |
| 31846 | PDHX | NM_003477.1 | 1151 | aggcttacttactccaatcataa | 134533 | - | see also 2351 line | | |
| 31847 | PEA15 | NM_003768.2 | 316 | tggcagtgcctggtttagcttcc | 134550 | - | see also 13951 line | | |
| 31848 | PEA15 | NM_003768.2 | 315 | ctggcagtgcctggtttagcttc | 134549 | - | see also 13951 line | | |
| 31849 | PEX12 | NM_000286.1 | 996 | cccgctggaaacgattttacaga | 134563 | - | see also 250 line | | |
| 31850 | PEX12 | NM_000286.1 | 994 | ttcccgctggaaacgattttaca | 134562 | - | see also 250 line | | |
| 31851 | PFDN4 | NM_002623.2 | 367 | ttcgggagcaacataaaccttga | 134594 | - | see also 13953 line | | |
| 31852 | PFDN4 | NM_002623.2 | 80 | cacggaatacaagtagaatcaca | 134587 | - | see also 13953 line | | |
| 31853 | PGLYRPIalpha | NM_052891.1 | 498 | cacctgtcgcccaggtatattca | 134596 | - | see also 13954 line | | |
| 31854 | PGLYRPIalpha | NM_052891.1 | 496 | gtcacctgtcgcccaggtatatt | 134595 | - | see also 13954 line | | |
| 31855 | PGLYRPIbeta | NM_020393.1 | 875 | aagtcatgcgacattggttataa | 134620 | - | see also 13955 line | | |
| 31856 | PGLYRPIbeta | NM_020393.1 | 880 | atgcgacattggttataacttcc | 134622 | - | see also 13955 line | | |
| 31857 | PGRP-L | NM_052890.2 | 1901 | cacgtctattcaggaaaacctagg | 134633 | - | see also 13956 line | | |
| 31858 | PGRP-L | NM_052890.2 | 1569 | tggccatagtgggcaactacacc | 134632 | - | see also 13956 line | | |
| 31859 | PHC2 | NM_004427.2 | 668 | gggtgctccctcaaactcaagt | 134640 | - | see also 2959 line | | |
| 31860 | PHIP | NM_017934.4 | 1627 | cacccgttcgatcctagagttct | 134703 | - | see also 13958 line | | |
| 31861 | PHIP | NM_017934.4 | 3299 | aactgactggcggatcatttacc | 134740 | - | see also 13958 line | | |
| 31862 | PI3 | NM_002638.2 | 307 | atccggtgcgccatgttgaatcc | 134823 | - | see also 13959 line | | |
| 31863 | PI3 | NM_002638.2 | 136 | gtcacgggagttcctgttaaagg | 134820 | - | see also 13959 line | | |

Figure 46

| | | | | | | | |
|---|---|---|---|---|---|---|---|
| 31864 | P13 | NM_002638.2 | 391 | tgcgggatggcctgtttcgttcc | 134825 | - | see also 13959 line |
| 31865 | PMPCA | NM_015160.1 | 842 | gagcgcagaggccgtggatattg | 134843 | - | see also 13960 line |
| 31866 | PMPCA | NM_015160.1 | 947 | ccccgagctcacgcacatcatgg | 134847 | - | see also 13960 line |
| 31867 | PNRC1 | NM_006813.1 | 1051 | agctgatggcagtacacttaaaa | 134864 | - | see also 13961 line |
| 31868 | PNRC1 | NM_006813.1 | 1053 | ctgatgcagtacacttaaaaac | 134865 | - | see also 13961 line |
| 31869 | PNRC1 | NM_006813.1 | 695 | tccccatggccagcttgttcatg | 134857 | - | see also 13961 line |
| 31870 | PROZ | NM_003891.1 | 570 | tcccgtggcaggtaaagttaaca | 134874 | - | see also 2646 line |
| 31871 | PROZ | NM_003891.1 | 1174 | cagtactcactctgtttaaac | 134884 | - | see also 2646 line |
| 31872 | RAD21 | NM_006265.1 | 1929 | ttgagttatgtcgaaatacgaac | 134932 | - | see also 4356 line |
| 31873 | RAD21 | NM_006265.1 | 365 | ctgggagtagttcgaatctatca | 134893 | - | see also 4356 line |
| 31874 | RBBP6 | NM_006910.4 | 1264 | ttcctattggagtgttaaatct | 134946 | - | see also 4848 line |
| 31875 | RBBP6 | NM_006910.4 | 1065 | tcctctaaactcaactatgatac | 134941 | - | see also 4848 line |
| 31876 | RCBTB1 | NM_018191.2 | 953 | tgcggttacgcacatatctagc | 135103 | - | see also 13965 line |
| 31877 | RCBTB1 | NM_018191.2 | 505 | caccgaagatggagtggtttatg | 135091 | - | see also 13965 line |
| 31878 | RCN2 | NM_002902.1 | 564 | tcccggtttgagtcttgaagaat | 135124 | - | see also 13966 line |
| 31879 | RCN2 | NM_002902.1 | 824 | taccttgggtagtacctaataat | 135132 | - | see also 13966 line |
| 31880 | RGS19 | NM_005873.1 | 543 | gagctgggcgcagtctttgaca | 135137 | - | gpcr, signal_transduction protein binding |
| 31881 | RGS19IP1 | NM_005716.1 | 1000 | gacgaacggctgggtgactttgc | 135139 | - | see also 13967 line |
| 31882 | RGS20 | NM_003702.2 | 222 | tgctcgtgtctcactgttagaaa | 135140 | - | see also 13968 line |
| 31883 | RGS20 | NM_003702.2 | 659 | ctcatatcctcgattcatgaact | 135148 | - | see also 13968 line |
| 31884 | RGS20 | NM_003702.2 | 707 | gtccttatcggagaaatctattg | 135151 | - | see also 13968 line |
| 31885 | RHOBTB3 | NM_014899.1 | 2080 | agcacagatggccgtcgaatatg | 135204 | - | see also 6083 line |
| 31886 | RHOBTB3 | NM_014899.1 | 2142 | tcccggaaatgtcgttgcttagt | 135211 | - | see also 6083 line |
| 31887 | RIN1 | NM_004292.1 | 193 | tgggcacctggccgagagaaaagc | 135213 | - | see also 13970 line |
| 31888 | RIN1 | NM_004292.1 | 192 | ctgggcacctggccgagagaaaag | 135212 | - | see also 13970 line |
| 31889 | RIN1 | NM_004292.1 | 399 | tcctgtcggaaatctaacacc | 135215 | - | see also 13970 line |
| 31890 | RINZF | NM_023929.2 | 2165 | gtcgacgagggccaaatagaaaa | 135245 | - | see also 8554 line |
| 31891 | RINZF | NM_023929.2 | 1485 | ccgcagggaccccgcaactacaag | 135221 | - | see also 8554 line |
| 31892 | RINZF | NM_023929.2 | 2104 | ggcaacacgaatcttaccaatt | 135242 | - | see also 8554 line |
| 31893 | RPC8 | NM_138338.1 | 514 | gtggtggacgagagctttgttga | 135268 | - | see also 13972 line |
| 31894 | RPE65 | NM_000329.1 | 787 | ctgactcccaactatatcgtttt | 135293 | - | see also 276 line |
| 31895 | RPE65 | NM_000329.1 | 304 | atccgcactgatgcttacgtacg | 135271 | - | see also 276 line |
| 31896 | RPGR | NM_000328.1 | 1228 | ttctgccgtatagccagtttaacc | 135352 | - | see also 13974 line |
| 31897 | RPGR | NM_000328.1 | 467 | tacatccgagcataagattaagc | 135330 | - | see also 13974 line |
| 31898 | RTKN | NM_033046.1 | 1216 | gagccgctgcttactattgctgt | 135384 | - | see also 13975 line |
| 31899 | RTKN | NM_033046.1 | 1598 | caccctggctggcaatgtttaca | 135386 | - | see also 13975 line |

Figure 46

| 31900 | RTN4 | NM_007008.1 | 550 | ctcaggcgcctcttcttagttga | 135397 | - | see also 13976 line | | |
|---|---|---|---|---|---|---|---|---|---|
| 31901 | RTN4 | NM_007008.1 | 343 | gtgagcgtaacagcctacattgc | 135393 | - | see also 13976 line | | |
| 31902 | S100P | NM_005980.1 | 56 | tagacgtctttcccgatattcg | 135483 | - | - | protein binding | breast cancer |
| 31903 | S100P | NM_005980.1 | 264 | cacgtctgcctgtcacaagtact | 135485 | - | see also 31902 line | | |
| 31904 | S100P | NM_005980.1 | 169 | gacaaggatgccgtggataaatt | 135484 | - | see also 31902 line | | |
| 31905 | SAG | NM_000541.2 | 230 | gtccgaaccgaaccatgttatct | 135486 | - | see also 13977 line | | |
| 31906 | SAG | NM_000541.2 | 232 | ccgaaccgaaccatgttatcttc | 135487 | - | see also 13977 line | | |
| 31907 | SARCOSIN | NM_006063.1 | 1153 | tccagctcgatagcatagcatct | 135537 | - | see also 13978 line | | |
| 31908 | SARCOSIN | NM_006063.1 | 141 | ttcatcgattgcaccctaaaagc | 135504 | - | see also 13978 line | | |
| 31909 | SARCOSIN | NM_006063.1 | 144 | atcgattgcaccctaaaagcagg | 135505 | - | see also 13978 line | | |
| 31910 | SBBI26 | NM_018846.2 | 1984 | cagggtgttggtcgattaggaca | 135613 | - | see also 7601 line | | |
| 31911 | SBBI26 | NM_018846.2 | 1992 | tggtcgattaggacacattctcg | 135614 | - | see also 7601 line | | |
| 31912 | SCAP1 | NM_003726.2 | 623 | aggcgcagctatgagtttacagc | 135634 | - | see also 13980 line | | |
| 31913 | SCAP1 | NM_003726.2 | 378 | aagaacttgataacgtaatcaag | 135628 | - | see also 13980 line | | |
| 31914 | SEC23IP | NM_007190.2 | 2738 | cccgtgctcatacgtcttcaacc | 135717 | - | see also 13981 line | | |
| 31915 | SEC23IP | NM_007190.2 | 1017 | tggagggcgctacgatgtttacc | 135660 | - | see also 13981 line | | |
| 31916 | SEMA6A | NM_020796.1 | 1905 | cagataccgccttaccaaaattg | 135763 | - | see also 7972 line | | |
| 31917 | SEMA6A | NM_020796.1 | 1282 | accgtcaagcacgattcaaaatg | 135743 | - | see also 7972 line | | |
| 31918 | SENP2 | NM_021627.1 | 665 | gccgtcgccatagcaaaggtaat | 135796 | - | see also 8154 line | | |
| 31919 | SENP2 | NM_021627.1 | 761 | gtctgaggcgtccccattgtact | 135803 | - | see also 8154 line | | |
| 31920 | SENP2 | NM_021627.1 | 1261 | gacgatctccttgaacttacaga | 135817 | - | see also 8154 line | | |
| 31921 | SERPINA4 | NM_006215.2 | 1328 | tcgtcgaccccacgaaaccatag | 135860 | - | see also 13984 line | | |
| 31922 | SERPINA4 | NM_006215.2 | 1204 | gccaccagcttcgcgatcaaatt | 135856 | - | see also 13984 line | | |
| 31923 | SERPINA7 | NM_000354.2 | 1464 | cactttcctacaccctattatcc | 135880 | - | see also 13985 line | | |
| 31924 | SERPINA7 | NM_000354.2 | 1468 | ttcctacaccctattatccaaat | 135881 | - | see also 13985 line | | |
| 31925 | SERPINA7 | NM_000354.2 | 1043 | tgcaccagatggaacaatactat | 135869 | - | see also 13985 line | | |
| 31926 | SERPINB10 | NM_005024.1 | 548 | tggtgaacgccctatactttaaa | 135901 | - | see also 13986 line | | |
| 31927 | SERPINB10 | NM_005024.1 | 550 | gtgaacgccctatactttaaagg | 135902 | - | see also 13986 line | | |
| 31928 | SERPINB6 | NM_004568.3 | 618 | accgaggagagactgtttaaagt | 135921 | - | see also 13987 line | | |
| 31929 | SERPINB6 | NM_004568.3 | 531 | ggctcagtggatccattgacaag | 135920 | - | see also 13987 line | | |
| 31930 | SERPINB6 | NM_004568.3 | 527 | tccgggctcagtggatccattga | 135919 | - | see also 13987 line | | |
| 31931 | SERPINB8 | NM_002640.3 | 605 | gacagtcgatcccctgacaaagc | 135933 | - | see also 13988 line | | |
| 31932 | SERPINB8 | NM_002640.3 | 529 | aagagtgcaggaagcatataaat | 135929 | - | see also 13988 line | | |

Figure 46

| | | | | | | | |
|---|---|---|---|---|---|---|---|
| 31933 | SERPINB9 | NM_004155.3 | 115 | ttgccatacgcctttaaagata | 135942 | - | see also 13989 line |
| 31934 | SERPINB9 | NM_004155.3 | 116 | tgccatacgcctttaaagatac | 135943 | - | see also 13989 line |
| 31935 | SERPINB9 | NM_004155.3 | 598 | atgaaccgtttgacgaaacatac | 135951 | - | see also 13989 line |
| 31936 | SGTA | NM_003021.3 | 714 | ccccgacaacgagacatacaagt | 135966 | - | see also 13990 line |
| 31937 | SGTA | NM_003021.3 | 713 | accccgacaacgagacatacaag | 135965 | - | see also 13990 line |
| 31938 | SHANK2 | NM_012309.1 | 37 | gtgatgatgacgggctacaataa | 135972 | - | see also 13991 line |
| 31939 | SHANK2 | NM_012309.1 | 276 | gaccggggacttcttgattgagg | 135980 | - | see also 13991 line |
| 31940 | SLC12A9 | NM_020246.2 | 1670 | ttccaccaggtgcgtaagtatct | 136029 | - | see also 13992 line |
| 31941 | SLC12A9 | NM_020246.2 | 2066 | ctgacggaccggctttctctga | 136032 | - | see also 13992 line |
| 31942 | SLC9A3R2 | NM_004785.1 | 780 | cccgagacagatgaacacttca | 136038 | - | see also 13993 line |
| 31943 | SLC9A3R2 | NM_004785.1 | 666 | cgcccaggacacggctcattgagg | 136037 | - | see also 13993 line |
| 31944 | SLIT1 | NM_003061.1 | 4070 | aacagcgaggcgccactctatgt | 136070 | - | see also 13994 line |
| 31945 | SLIT1 | NM_003061.1 | 453 | ctcggatccataagaatgactt | 136041 | - | see also 13994 line |
| 31946 | SLIT2 | NM_004787.1 | 1964 | tacttcttacgagtaatcgtttg | 136137 | - | see also 3302 line |
| 31947 | SLIT2 | NM_004787.1 | 3737 | ttcggcctcagacgaacataaca | 136189 | - | see also 3302 line |
| 31948 | SLIT3 | NM_003062.1 | 2583 | ctccgacacctgacgcttattga | 136229 | - | see also 13996 line |
| 31949 | SLIT3 | NM_003062.1 | 3267 | gacaacgactgcgaaaacaatgc | 136236 | - | see also 13996 line |
| 31950 | SMO | NM_005631.3 | 1743 | tgctatgtcaggccaatgtgacc | 136263 | - | see also 13997 line |
| 31951 | SMO | NM_005631.3 | 1723 | gcgcagcttccgggactatgtgc | 136261 | - | see also 13997 line |
| 31952 | SNCAIP | NM_005460.1 | 192 | aagatgtgatacgcaaaacgaag | 136265 | - | see also 13998 line |
| 31953 | SNCAIP | NM_005460.1 | 325 | cgggtttcgccactgaaacatca | 136269 | - | see also 13998 line |
| 31954 | SNX6 | NM_021249.2 | 780 | gagcacgaacgaacatttcttt | 136317 | - | see also 13999 line |
| 31955 | SNX6 | NM_021249.2 | 744 | atcgtttcaggagtaaggatgt | 136315 | - | see also 13999 line |
| 31956 | SOCS5 | NM_014011.3 | 1614 | tgcaggtgcactacgtatgatgg | 136393 | - | see also 14000 line |
| 31957 | SOCS5 | NM_014011.3 | 600 | cgctacgggtaagttctgtaca | 136356 | - | see also 14000 line |
| 31958 | SOCS5 | NM_014011.3 | 649 | ctgtaggaagtcgctcctaaga | 136358 | - | see also 14000 line |
| 31959 | SORCS2 | NM_020777.1 | 1006 | ctcttatcgtcgaaatgaattg | 136406 | - | see also 14001 line |
| 31960 | SORCS2 | NM_020777.1 | 3229 | gaccgttacgcccaaatgcaca | 136426 | - | see also 14001 line |
| 31961 | SORCS2 | NM_020777.1 | 1300 | gacgccttaacctacaacaagg | 136412 | - | see also 14001 line |
| 31962 | SPEC1 | NM_020239.2 | 440 | gaccatgattggggaaccaatga | 136428 | - | see also 14002 line |
| 31963 | SPEC1 | NM_020239.2 | 444 | atgattggggaaccaatgaattt | 136429 | - | see also 14002 line |
| 31964 | SPEC2 | NM_020240.1 | 62 | gacgggcggattgacagaagtatg | 136433 | - | see also 14003 line |
| 31965 | SPEC2 | NM_020240.1 | 65 | gggcggattgacagaagtatgatt | 136435 | - | see also 14003 line |
| 31966 | SPOP | NM_003563.2 | 1403 | cacgcaaacgcctgaagcaatcc | 136453 | - | see also 14004 line |
| 31967 | SPOP | NM_003563.2 | 825 | taccatgaacatggtaaaggttc | 136448 | - | see also 14004 line |
| 31968 | STATH | NM_003154.1 | 210 | accactataccacaccaccatacc | 136454 | structural constituent of tooth enamel | - |

Figure 46

| 31969 | STATH | NM_003154.1 | 225 | accataccaaccacaataccaac | 136455 | - | see also 31968 line |
|---|---|---|---|---|---|---|---|
| 31970 | STIM1 | NM_003156.2 | 1494 | cggagccgccaaaaatatgctga | 136465 | - | see also 2129 line |
| 31971 | STIM1 | NM_003156.2 | 1491 | gagcggagccgccaaaaatatgc | 136464 | - | see also 2129 line |
| 31972 | STIM2 | NM_020860.1 | 2368 | gcctcgagatataccaaacatta | 136512 | - | see also 14006 line |
| 31973 | STIM2 | NM_020860.1 | 2359 | ctcctttaagcctcgagatatac | 136510 | - | see also 14006 line |
| 31974 | TA-KRP | NM_032505.1 | 1165 | tgcaatcggaggtcgtgtttatg | 136566 | - | see also 14007 line |
| 31975 | TA-KRP | NM_032505.1 | 486 | tcggagatcgatcaaaagaatac | 136541 | - | see also 14007 line |
| 31976 | TANK | NM_004180.2 | 168 | aacattggcgagcaactcaataa | 136592 | - | see also 14008 line |
| 31977 | TANK | NM_004180.2 | 264 | aactatgagcagagaatacgtga | 136597 | - | see also 14008 line |
| 31978 | TANK | NM_004180.2 | 356 | ttccactcaagataacaattatg | 136600 | - | see also 14008 line |
| 31979 | TCEB3 | NM_003198.1 | 1496 | acccgcgatacaggccaattacc | 136656 | - | see also 2160 line |
| 31980 | TCEB3 | NM_003198.1 | 1948 | agcggctacgagtactaacaaag | 136672 | - | see also 2160 line |
| 31981 | TGFA | NM_003236.1 | 144 | cagcagtggtgtcccattttaat | 136680 | - | see also 14010 line |
| 31982 | TGFA | NM_003236.1 | 397 | ctgctgccaggtccgaaaacact | 136683 | - | see also 14010 line |
| 31983 | TIP-1 | NM_014604.1 | 219 | gcgtcaaggtgagaaacttaatcc | 136688 | - | see also 14011 line |
| 31984 | TIP-1 | NM_014604.1 | 184 | accgccgtggtgcaaagagttga | 136685 | - | see also 14011 line |
| 31985 | TNFAIP1 | NM_021137.3 | 958 | gacactcaacgtcctactctatg | 136696 | - | see also 14012 line |
| 31986 | TNFAIP1 | NM_021137.3 | 932 | ttcccagaggcccgaatctatga | 136695 | - | see also 14012 line |
| 31987 | TNIP1 | NM_006058.2 | 2057 | accctgtggaggggttcgaaatc | 136707 | - | see also 14013 line |
| 31988 | TNIP1 | NM_006058.2 | 2058 | ccctgtggaggggttcgaaatcc | 136708 | - | see also 14013 line |
| 31989 | TNKS | NM_003747.1 | 1104 | gggcgaaagtcgactcctttaca | 136731 | - | see also 2565 line |
| 31990 | TNKS | NM_003747.1 | 607 | tggtggacgcggcaaacgtaaat | 136717 | - | see also 2565 line |
| 31991 | TOB1 | NM_005749.2 | 588 | accaagttcggctctaccaaaat | 136820 | - | see also 3996 line |
| 31992 | TOB1 | NM_005749.2 | 630 | aacaaggttgcacgtacttctcc | 136821 | - | see also 3996 line |
| 31993 | TOB1 | NM_005749.2 | 356 | accagtgaaggtgctttacgtgg | 136812 | - | see also 3996 line |
| 31994 | TOLLIP | NM_019009.2 | 438 | cccaggcgtggactctttctatc | 136834 | - | see also 14016 line |
| 31995 | TOLLIP | NM_019009.2 | 261 | caccgtgggccgactgaacatca | 136830 | - | see also 14016 line |
| 31996 | TRAPPC4 | NM_016146.1 | 290 | agctggcggcttgatttaccagt | 136838 | - | -      protein binding     - |
| 31997 | TRAPPC4 | NM_016146.1 | 531 | tacccggtgtccattcgatttgg | 136839 | - | see also 31996 line |
| 31998 | TRAPPC4 | NM_016146.1 | 758 | ttctcttctccgaaagatttatg | 136843 | - | see also 31996 line |
| 31999 | TRIM14 | NM_014788.2 | 661 | agccgtggagagtacattacaga | 136853 | - | see also 14017 line |
| 32000 | TRIM14 | NM_014788.2 | 353 | ttcactgaactcagattactact | 136849 | - | see also 14017 line |
| 32001 | TRIP10 | NM_004240.1 | 1443 | ccccatttacacggagtttgatg | 136869 | - | see also 2847 line |
| 32002 | TRIP10 | NM_004240.1 | 1655 | acctccgagtcacgctcaattga | 136874 | - | see also 2847 line |
| 32003 | TRIP15 | NM_004236.1 | 1261 | aggggtggtgcacgatatactgc | 136922 | - | see also 2826 line |
| 32004 | TRIP15 | NM_004236.1 | 941 | tagcaatgacgaatttagtaagt | 136907 | - | see also 2826 line |
| 32005 | TRIP6 | NM_003302.1 | 976 | cacgtgggctgctttgtatgttc | 136929 | - | see also 14020 line |

Figure 46

| | | | | | | | |
|---|---|---|---|---|---|---|---|
| 32006 | TRIP6 | NM_003302.1 | 1336 | ctggatcgaagtttcacattgg | 136935 | - | see also 14020 line |
| 32007 | TRIP6 | NM_003302.1 | 1332 | tgctctgatcgaagtttcaca | 136933 | - | see also 14020 line |
| 32008 | TTC1 | NM_003314.1 | 879 | accggctcgtactccatcaattt | 136959 | - | see also 2246 line |
| 32009 | TTC1 | NM_003314.1 | 884 | ctcgtactccatcaatttcgttc | 136962 | - | see also 2246 line |
| 32010 | TWSG1 | NM_020648.3 | 726 | atgtattgactatggtagtaaaa | 136980 | - | see also 14022 line |
| 32011 | TWSG1 | NM_020648.3 | 716 | ttggtccagaatgtattgactat | 136978 | - | see also 14022 line |
| 32012 | USH1C | NM_005709.1 | 1454 | tccggctcctacgcatcaagaag | 137000 | - | see also 14023 line |
| 32013 | USH1C | NM_005709.1 | 490 | gacgagatgtccggatcaatgg | 136987 | - | see also 14023 line |
| 32014 | VBP1 | NM_003372.3 | 531 | ggcagataacctgattgcaaag | 137013 | - | see also 2275 line |
| 32015 | VBP1 | NM_003372.3 | 530 | tggcagataacctgtattgcaaa | 137012 | - | see also 2275 line |
| 32016 | VPS26 | NM_004896.2 | 105 | gtccaatttgtgagatcgatatt | 137022 | - | see also 14025 line |
| 32017 | VPS26 | NM_004896.2 | 106 | tccaatttgtgagatcgatattg | 137023 | - | see also 14025 line |
| 32018 | VPS35 | NM_018206.2 | 197 | tggtgaactccggacttctatgt | 137047 | - | see also 7319 line |
| 32019 | VPS35 | NM_018206.2 | 196 | ttggtgaactccggacttctatg | 137046 | - | see also 7319 line |
| 32020 | WWP1 | NM_007013.3 | 1552 | atctcagcggaaccaattgcagg | 137075 | - | see also 14027 line |
| 32021 | WWP1 | NM_007013.3 | 561 | taggaaaagcaacgatagatttg | 137071 | - | see also 14027 line |
| 32022 | XAB2 | NM_020196.1 | 450 | atcacgcagcactctcgaatttg | 137084 | - | see also 14028 line |
| 32023 | XAB2 | NM_020196.1 | 355 | ctcgtctrggctagattactgc | 137083 | - | see also 14028 line |
| 32024 | YAP1 | NM_006106.2 | 1139 | ccctgggtagccagttaccaaca | 137103 | - | see also 14029 line |
| 32025 | YAP1 | NM_006106.2 | 821 | atcacatcgatcagacaacaaca | 137102 | - | see also 14029 line |
| 32026 | ZBTB4 | NM_020899.2 | 1788 | gcctcggttatcacttcactgc | 137122 | - | see also 14030 line |
| 32027 | ZBTB4 | NM_020899.2 | 1784 | agctgcctcggttatcacttaca | 137120 | - | see also 14030 line |
| 32028 | ZBTB5 | NM_014872.1 | 1393 | gagcataagtcacttttagtat | 137149 | - | see also 6068 line |
| 32029 | ZBTB5 | NM_014872.1 | 602 | ccggcatgcagcgctcctttatg | 137134 | - | see also 6068 line |
| 32030 | ZBTB8 | NM_144621.2 | 91 | atggagatgcaatcctattatgc | 137161 | - | see also 14032 line |
| 32031 | ZBTB8 | NM_144621.2 | 176 | ttgtggaagggcggatcttcaag | 137162 | - | see also 14032 line |
| 32032 | ZNF-kaiso | NM_006777.2 | 686 | ttgcacctagtgctagacaataac | 137198 | - | see also 4776 line |
| 32033 | ZNF-kaiso | NM_006777.2 | 1281 | atcgactggttgcaagttttatg | 137221 | - | see also 4776 line |
| 32034 | ZNF-U69274 | NM_014415.1 | 2938 | ctgaacactccgtaaaacatatg | 137332 | - | see also 14035 line |
| 32035 | ZNF-U69274 | NM_014415.1 | 1901 | acgatacgccttattaatgcaca | 137298 | - | see also 14035 line |
| 32036 | ZNF259 | NM_003904.1 | 172 | gtcgtatgcatgaactgttact | 137347 | - | see also 14036 line |
| 32037 | ZNF259 | NM_003904.1 | 473 | ttgatcacccgtgctatcctgg | 137352 | - | see also 14036 line |
| 32038 | ZNF499 | NM_032792.2 | 615 | tgcttcgcataacagacagtttatc | 137371 | - | see also 14037 line |
| 32039 | ZNF499 | NM_032792.2 | 1485 | ctgagccacctacgttatgagtgc | 137374 | - | see also 14037 line |
| 32040 | CALR3 | NM_145046.2 | 655 | taagaactggaacttaacatcact | 137389 | - | see also 14038 line |

Figure 46

| | | | | | | | |
|---|---|---|---|---|---|---|---|
| 32041 | CALR3 | NM_145046.2 | 217 | tcgtcggcgaagtttatggtca | 137379 | - | see also 14038 line |
| 32042 | CANX | NM_001746.2 | 650 | atgacaagacccctatacgatt | 137409 | - | see also 1156 line |
| 32043 | CANX | NM_001746.2 | 657 | gaccccttatacgattatgtttg | 137411 | - | see also 1156 line |
| 32044 | CASQ1 | NM_001231.2 | 561 | gagtacgatggcgagttttctgc | 137445 | - | see also 14040 line |
| 32045 | CASQ1 | NM_001231.2 | 559 | ttgagtacgatggcgagtttct | 137444 | - | see also 14040 line |
| 32046 | CASQ2 | NM_001232.1 | 200 | aaggaccgagtggtaagtcttc | 137462 | - | see also 14041 line |
| 32047 | CASQ2 | NM_001232.1 | 462 | agggtgatcgcacaatagagttt | 137469 | - | see also 14041 line |
| 32048 | CLGN | NM_004362.1 | 1222 | ctccactggtcgataatcctaac | 137516 | - | see also 2925 line |
| 32049 | CLGN | NM_004362.1 | 313 | tggctgggtgcttatcaaaa | 137487 | - | see also 2925 line |
| 32050 | CLGN | NM_004362.1 | 497 | tgctgataaaccctgatagttc | 137495 | - | see also 2925 line |
| 32051 | FLJ20793 | NM_019022.3 | 579 | gagacaccgtgtattttcgttt | 137558 | - | see also 7663 line |
| 32052 | FLJ20793 | NM_019022.3 | 169 | ctctgcgccacagttgttgtact | 137537 | - | see also 7663 line |
| 32053 | AGPAT2 | NM_006412.2 | 266 | tgcgaagcttcaagtactttac | 137589 | - | see also 14044 line |
| 32054 | AGPAT2 | NM_006412.2 | 662 | tcgtccccgtggtgtactcttcc | 137592 | - | see also 14044 line |
| 32055 | AKAP13 | NM_006738.4 | 6104 | gtcttggagtcggataatagaca | 137714 | - | see also 14046 line |
| 32056 | AKAP13 | NM_006738.4 | 6616 | ttgtaactcagcggattaccaag | 137737 | - | see also 14046 line |
| 32057 | ANKRD5 | NM_022096.4 | 1781 | ctgatagcagccggtttaataga | 137824 | - | see also 8268 line |
| 32058 | ANKRD5 | NM_022096.4 | 1120 | gacgtggggctgatttcgataaa | 137800 | - | see also 8268 line |
| 32059 | ANXA1 | NM_000700.1 | 946 | ctcgccataaggcattgatcagg | 137873 | - | see also 14048 line |
| 32060 | ANXA1 | NM_000700.1 | 226 | tgcattaaggccataatggttaaa | 137860 | - | see also 14048 line |
| 32061 | ANXA10 | NM_007193.2 | 975 | gaccataaggaaacgatacaaag | 137887 | - | see also 4995 line |
| 32062 | ANXA10 | NM_007193.2 | 888 | tagattatatgtgcaattcatg | 137883 | - | see also 4995 line |
| 32063 | ANXA13 | NM_004306.1 | 828 | ttgctgaacgtctgtacagtcg | 137906 | - | see also 14050 line |
| 32064 | ANXA13 | NM_004306.1 | 545 | ctgcaggctaatcgcaatgaagg | 137900 | - | see also 14050 line |
| 32065 | ANXA3 | NM_005139.1 | 856 | tactctgaaccgaataatggtgt | 137931 | - | see also 14051 line |
| 32066 | ANXA3 | NM_005139.1 | 428 | atctctcaagcctattatacagt | 137917 | - | see also 14051 line |
| 32067 | ANXA4 | NM_001153.2 | 163 | aggggtctcggcaaccgatgaagacg | 137934 | - | see also 14052 line |
| 32068 | ANXA4 | NM_001153.2 | 254 | accatcgcagggacttgataga | 137938 | - | see also 14052 line |
| 32069 | ANXA5 | NM_001154.2 | 558 | gagagccatcaaacaagtttag | 137962 | - | see also 14053 line |
| 32070 | ANXA5 | NM_001154.2 | 241 | gagcgggctgatgcagaaactct | 137955 | - | see also 14053 line |
| 32071 | ANXA6 | NM_001155.2 | 916 | tccggagcacccggaatattt | 137988 | - | see also 733 line |
| 32072 | ANXA6 | NM_001155.2 | 915 | atccggagcacccggaatatt | 137987 | - | see also 733 line |
| 32073 | ANXA6 | NM_001155.2 | 408 | ggcaagtttgaacggttgattgt | 137975 | - | see also 733 line |
| 32074 | ANXA7 | NM_001156.2 | 1150 | atggctaatcgagactgttaag | 138038 | - | see also 14055 line |
| 32075 | ANXA7 | NM_001156.2 | 625 | gtggtggccaacgttccaatga | 138016 | - | see also 14055 line |
| 32076 | ANXA8 | NM_001630.1 | 918 | cgcgtgatgggaccctgataaga | 138054 | - | see also 14056 line |
| 32077 | ANXA8 | NM_001630.1 | 952 | aaggagcgagagattgacttaatc | 138056 | - | see also 14056 line |

Figure 46

| 32078 | ANXA9 | NM_003568.1 | 1203 | gcctagcttcggtgatcaagaac | 138070 | - | see also 14057 line | | |
|---|---|---|---|---|---|---|---|---|---|
| 32079 | ANXA9 | NM_003568.1 | 1287 | aagtcctgattcgcatccttatc | 138071 | - | see also 14057 line | | |
| 32080 | APBA2BP | NM_031231.2 | 352 | atgggcatctcaccgacaattta | 138074 | - | see also 14058 line | | |
| 32081 | APBA2BP | NM_031231.2 | 218 | cacgctcttccaggacgttttcc | 138073 | - | see also 14058 line | | |
| 32082 | ASPH | NM_004318.2 | 267 | ctgggcgtctggacatctgtagc | 138083 | - | see also 2887 line | | |
| 32083 | ASPH | NM_004318.2 | 281 | atctgtagctgtcgtttggtttg | 138084 | - | see also 2887 line | | |
| 32084 | BGLAP | NM_000711.1 | 163 | cccaggcgctacctgtatcaatg | 138142 | - | - | VDR/RXR alpha heterodimer | - |
| 32085 | BMP1 | NM_001199.1 | 838 | tgcattacgctcggaacacattc | 138146 | - | see also 779 line | | |
| 32086 | BMP1 | NM_001199.1 | 731 | ttccatcgttcgtgagaacatcc | 138145 | - | see also 779 line | | |
| 32087 | BMPER | NM_133468.2 | 420 | gccgctcgcctgggattacgtgc | 138170 | - | see also 9585 line | | |
| 32088 | BMPER | NM_133468.2 | 2284 | ctgtggtccgggatgtatcaaga | 138219 | - | see also 9585 line | | |
| 32089 | C14orf10 | NM_017917.2 | 698 | aagcgatgatcaattacgaaaac | 138232 | - | see also 7172 line | | |
| 32090 | C14orf10 | NM_017917.2 | 699 | agcgatgatcaattacgaaaact | 138233 | - | see also 7172 line | | |
| 32091 | C14orf143 | NM_145231.1 | 298 | aacgatatcggaacgaagtaaga | 138268 | - | see also 14063 line | | |
| 32092 | C14orf143 | NM_145231.1 | 339 | gacacctactatcgtggattttt | 138269 | - | see also 14063 line | | |
| 32093 | C14orf143 | NM_145231.1 | 465 | cacgtcagctttagagactttga | 138276 | - | see also 14063 line | | |
| 32094 | C1orf10 | NM_016190.1 | 267 | aggaattcctggtcttagtgttt | 138280 | - | see also 14064 line | | |
| 32095 | C1orf10 | NM_016190.1 | 1366 | tggtgaggaatgggttgatgacc | 138285 | - | see also 14064 line | | |
| 32096 | C1orf22 | NM_025191.2 | 1542 | ttgtatgctcaaagtattcgtga | 138325 | - | see also 14065 line | | |
| 32097 | C1orf22 | NM_025191.2 | 732 | gtgggcgtgactataaatattca | 138303 | - | see also 14065 line | | |
| 32098 | C1QR1 | NM_012072.2 | 839 | gacgagactcagagtcattattt | 138353 | - | see also 14066 line | | |
| 32099 | C1QR1 | NM_012072.2 | 1680 | aggctacacccaccacaagtaga | 138354 | - | see also 14066 line | | |
| 32100 | C1R | NM_001733.2 | 1048 | tccgtgactacttcattgctacc | 138364 | - | see also 14067 line | | |
| 32101 | C1R | NM_001733.2 | 1446 | cgcatcatcggagggcaaaaagc | 138365 | - | see also 14067 line | | |
| 32102 | C1S | NM_001734.1 | 562 | tacggggtttgctgcatactatg | 138371 | - | see also 14068 line | | |
| 32103 | C1S | NM_001734.1 | 2195 | tacacacgggtaaagaactatgt | 138417 | - | see also 14068 line | | |
| 32104 | C20orf31 | NM_018217.1 | 1183 | cccacttcggccagaacttattg | 138443 | - | see also 7327 line | | |
| 32105 | C20orf31 | NM_018217.1 | 1304 | tgcggatttgcaacaatcaaaga | 138445 | - | see also 7327 line | | |
| 32106 | Cab45 | NM_016176.2 | 741 | gacgagtataaggtgaagttttt | 138453 | - | see also 14069 line | | |
| 32107 | Cab45 | NM_016176.2 | 734 | gtcttgggacgagtataaggtga | 138452 | - | see also 14069 line | | |
| 32108 | CABP1 | NM_004276.3 | 483 | accgagacatagaggaaattatc | 138459 | - | see also 14070 line | | |
| 32109 | CABP1 | NM_004276.3 | 121 | atcactgcgaccagaggaaattg | 138457 | - | see also 14070 line | | |
| 32110 | CABP2 | NM_016366.1 | 452 | ggcggaaaggtggactttgaaga | 138461 | - | see also 14071 line | | |
| 32111 | CABP5 | NM_019855.3 | 600 | gacggcacagttgactttgaaga | 138473 | - | see also 14073 line | | |
| 32112 | CABP5 | NM_019855.3 | 315 | cccacggagatggaactgattga | 138470 | - | see also 14073 line | | |
| 32113 | CABP7 | NM_182527.1 | 401 | ttccatggcaccgactttgatac | 138474 | - | see also 14074 line | | |

Figure 46

| | | | | | | | |
|---|---|---|---|---|---|---|---|
| 32114 | CABP7 | NM_182527.1 | 402 | tccatggcaccgactttgatact | 138475 | - | see also 14074 line |
| 32115 | CACNA1C | NM_000719.3 | 1681 | gtccgtcaacacggaaaacgtgg | 138498 | - | see also 442 line |
| 32116 | CACNA1C | NM_000719.3 | 1746 | cccacggatctccaagtcaaag | 138500 | - | see also 442 line |
| 32117 | CACNA1D | NM_000720.1 | 4751 | agggtagcgtgcaagagattagt | 138612 | - | see also 445 line |
| 32118 | CACNA1E | NM_000721.1 | 1517 | aggtagacggggtctctatttc | 138678 | - | see also 457 line |
| 32119 | CACNA1E | NM_000721.1 | 1371 | tggaacatccgcctagaagtgc | 138672 | - | see also 457 line |
| 32120 | CACNA1F | NM_005183.1 | 3794 | cacgtttgacgcttattgtgg | 138784 | - | see also 3643 line |
| 32121 | CACNA1F | NM_005183.1 | 2836 | gccgtagctgtttaatatgttg | 138768 | - | see also 3643 line |
| 32122 | CACNA1F | NM_005183.1 | 3458 | gggcgagcaggagtaccaaaact | 138780 | - | see also 3643 line |
| 32123 | CACNA1G | NM_018896.3 | 3074 | cagaccggaagaatttgactcc | 138807 | - | see also 7604 line |
| 32124 | CACNA1G | NM_018896.3 | 4602 | accatcgcaacattgtagtca | 138814 | - | see also 7604 line |
| 32125 | CACNA1H | NM_021098.1 | 6444 | agcgtccgcactcgtaagcatac | 138850 | - | see also 14080 line |
| 32126 | CACNA1H | NM_021098.1 | 2419 | gccgtggcgtcatgaattcaag | 138838 | - | see also 14080 line |
| 32127 | CACNA1I | NM_021096.2 | 813 | gtcgggcgacaaftgggataatgg | 138857 | - | see also 14081 line |
| 32128 | CACNA1I | NM_021096.2 | 3589 | ggcagcacggaacgcatcttct | 138869 | - | see also 14081 line |
| 32129 | CACNA1I | NM_021096.2 | 5742 | ctcgccgatccagagaacttcc | 138883 | - | see also 14081 line |
| 32130 | CACNA1S | NM_000069.1 | 4542 | cccacatgggtagcttgtaagc | 138925 | - | see also 14082 line |
| 32131 | CACNA1S | NM_000069.1 | 1597 | cactggctgaccgtttgcaaga | 138897 | - | see also 14082 line |
| 32132 | CALB2 | NM_001740.2 | 161 | gacggaaatggtatattgaagg | 138936 | - | see also 1152 line |
| 32133 | CALB3 | NM_004057.1 | 199 | aacaccctagatgatctctttca | 138948 | - | see also 14084 line |
| 32134 | CALML3 | NM_005185.2 | 263 | ggccgagctgcgggacatgatga | 138949 | - | calcium ion binding |
| 32135 | CALML5 | NM_017422.3 | 163 | cgcggttgacacggatggaaacg | 138950 | - | see also 14085 line |
| 32136 | CALN1 | NM_031468.1 | 569 | ttcagaaggtcgcgatggtttc | 138952 | - | see also 14086 line |
| 32137 | CALN1 | NM_031468.1 | 823 | gcctcatatgcgcctttgctatg | 138958 | - | see also 14086 line |
| 32138 | CALN1 | NM_031468.1 | 589 | ttcttgggaacacgatagacagc | 138953 | - | see also 14086 line |
| 32139 | CALU | NM_001219.2 | 983 | ggccttagtacggcatgatgagt | 138983 | - | see also 795 line |
| 32140 | CAPN1 | NM_005186.2 | 2143 | acctggcgtgacttgacaat | 138994 | - | see also 14088 line |
| 32141 | CAPN1 | NM_005186.2 | 404 | tcccacggaactgctgtcaaacc | 138984 | - | see also 14088 line |
| 32142 | CAPN11 | NM_007058.1 | 869 | gtgagaggcacgcttactctgt | 139009 | - | see also 14089 line |
| 32143 | CAPN11 | NM_007058.1 | 1066 | ctggatgtcctaccaagatttcc | 139010 | - | see also 14089 line |
| 32144 | CAPN2 | NM_001748.3 | 2142 | ttcggctggaaacgctattcaag | 139057 | - | see also 1157 line |
| 32145 | CAPN2 | NM_001748.3 | 698 | gccaagatcaacggatggctatga | 139030 | - | see also 1157 line |
| 32146 | CAPN3 | NM_000070.2 | 2258 | agcaacaattcggaacattttc | 139099 | - | see also 14091 line |
| 32147 | CAPN3 | NM_000070.2 | 2516 | accagtccggcaccatcaacagc | 139104 | - | see also 14091 line |
| 32148 | CAPN9 | NM_006615.1 | 848 | gacgcgtttggtcttattaagg | 139126 | - | see also 14092 line |
| 32149 | CAPN9 | NM_006615.1 | 485 | cctggttatgccgggatattcc | 139118 | - | see also 14092 line |
| 32150 | CAPNS1 | NM_001749.1 | 798 | atccgacgctactcagatgaaag | 139159 | - | see also 14093 line |

Figure 46

| | | | | | | | |
|---|---|---|---|---|---|---|---|
| 32151 | CAPNS1 | NM_001749.1 | 799 | tccgacgctactcagatgaaagt | 139160 | - | see also 14093 line |
| 32152 | CAPNS2 | NM_032330.1 | 320 | aggcgatttcggcaacaatttac | 139165 | - | see also 14094 line |
| 32153 | CAPNS2 | NM_032330.1 | 668 | cgccggtatgctaatgaagatgg | 139172 | - | see also 14094 line |
| 32154 | CAPS | NM_004058.2 | 205 | caggtttttccgccaactagacc | 139178 | - | see also 14095 line |
| 32155 | CAPS2 | NM_032606.2 | 1115 | aagatgacacctcttcgatttag | 139180 | - | see also 14096 line |
| 32156 | CAPS2 | NM_032606.2 | 1841 | ttcaaacgtgtattattggtga | 139192 | - | see also 14096 line |
| 32157 | CBARA1 | NM_006077.1 | 154 | tccagatccggcgaagactaatg | 139205 | - | see also 4219 line |
| 32158 | CBARA1 | NM_006077.1 | 567 | cacttggtctggatcaatatat | 139220 | - | see also 4219 line |
| 32159 | CBARA1 | NM_006077.1 | 153 | atccagatccgcgaagactaat | 139204 | - | see also 4219 line |
| 32160 | CD164L1 | NM_020404.1 | 1487 | tgcctaccaaccggtattctct | 139243 | - | see also 14098 line |
| 32161 | CD164L1 | NM_020404.1 | 725 | gggctgcgaacacgaatgtgtgg | 139239 | - | see also 14098 line |
| 32162 | CD97 | NM_001784.2 | 1390 | tgcgatccttatggctcattatg | 139256 | - | see also 14099 line |
| 32163 | CD97 | NM_001784.2 | 924 | gcgcacgcatgaagctgaattgg | 139251 | - | see also 14099 line |
| 32164 | CDH1 | NM_004360.2 | 874 | cacggtaaccgatcagaatgaca | 139278 | - | see also 14100 line |
| 32165 | CDH1 | NM_004360.2 | 872 | atcacggtaaccgatcagaatga | 139277 | - | see also 14100 line |
| 32166 | CDH10 | NM_006727.2 | 2388 | ctccggcgagatattattccaga | 139373 | - | see also 14101 line |
| 32167 | CDH10 | NM_006727.2 | 1261 | atgtgacggtactgatatgttt | 139340 | - | see also 14101 line |
| 32168 | CDH11 | NM_001797.2 | 1669 | ctgccaacagccgataaggtat | 139405 | - | see also 1173 line |
| 32169 | CDH11 | NM_001797.2 | 850 | tggagccaccgtcggaattcatt | 139385 | - | see also 1173 line |
| 32170 | CDH12 | NM_004061.2 | 2408 | atgccactaatgaattactaga | 139471 | - | see also 14103 line |
| 32171 | CDH12 | NM_004061.2 | 1834 | agccggaacaacaatagtcaaca | 139453 | - | see also 14103 line |
| 32172 | CDH13 | NM_001257.2 | 1167 | cacgatcatgatcgatgacaaaa | 139527 | - | see also 818 line |
| 32173 | CDH13 | NM_001257.2 | 957 | tgccctcgcggtataatatcc | 139523 | - | see also 818 line |
| 32174 | CDH15 | NM_004933.2 | 2208 | atgccgacttcatcaatgatgg | 139556 | - | see also 14105 line |
| 32175 | CDH15 | NM_004933.2 | 2007 | caggacgaccttcgagacaatgt | 139555 | - | see also 14105 line |
| 32176 | CDH16 | NM_004062.2 | 547 | agccaactcggatcttcgattcc | 139559 | - | see also 14106 line |
| 32177 | CDH16 | NM_004062.2 | 1382 | gtcgcagtcacagatatcaatga | 139568 | - | see also 14106 line |
| 32178 | CDH17 | NM_004063.2 | 1141 | ccgtcaccagtaaccgtatttga | 139609 | - | see also 2738 line |
| 32179 | CDH17 | NM_004063.2 | 2341 | gtcgtcttgatccgcatcaatga | 139647 | - | see also 2738 line |
| 32180 | CDH18 | NM_004934.2 | 764 | agctattgatagacgtacaaaca | 139669 | - | see also 14108 line |
| 32181 | CDH18 | NM_004934.2 | 1574 | gaccgtcgttggtacagtttttg | 139693 | - | see also 14108 line |
| 32182 | CDH18 | NM_004934.2 | 999 | ttctccgtcgaccctaaaacagg | 139674 | - | see also 14108 line |
| 32183 | CDH19 | NM_021153.2 | 664 | gacgatccctcaagtgttaataa | 139745 | - | see also 14109 line |
| 32184 | CDH19 | NM_021153.2 | 1126 | tacggtattagagcaaaagttaa | 139765 | - | see also 14109 line |
| 32185 | CDH2 | NM_001792.2 | 888 | agcagatagcccgtttcatttg | 139825 | - | see also 1160 line |
| 32186 | CDH2 | NM_001792.2 | 1734 | ttcatgccgtaccatgttgaca | 139853 | - | see also 1160 line |
| 32187 | CDH20 | NM_031891.2 | 2237 | tcgactccctccagacgtatatg | 139935 | - | see also 9045 line |

Figure 46

| | | | | | | | |
|---|---|---|---|---|---|---|---|
| 32188 | CDH20 | NM_031891.2 | 1946 | accggaaacaaccatacatcatc | 139933 | - | see also 9045 line |
| 32189 | CDH22 | NM_021248.1 | 1691 | cgccattgaccgcgaatcagatt | 139943 | - | see also 14112 line |
| 32190 | CDH22 | NM_021248.1 | 1716 | gaccagatcttcgatatcgatgc | 139945 | - | see also 14112 line |
| 32191 | CDH22 | NM_021248.1 | 1692 | gccattgaccgcgaatcagattt | 139944 | - | see also 14112 line |
| 32192 | CDH23 | NM_022124.2 | 1258 | agcggagtgctgaccttgaatgg | 139952 | - | see also 8289 line |
| 32193 | CDH23 | NM_022124.2 | 1607 | aggggaaggcggacattcgtatt | 139955 | - | see also 8289 line |
| 32194 | CDH24 | NM_022478.2 | 1649 | agccctacgatacttttgtgtgt | 140046 | - | see also 14114 line |
| 32195 | CDH24 | NM_022478.2 | 1644 | ggctgagccctacgatacttttg | 140045 | - | see also 14114 line |
| 32196 | CDH24 | NM_022478.2 | 1344 | tccggagcgttgcttctctatcc | 140042 | - | see also 14114 line |
| 32197 | CDH26 | NM_021810.3 | 275 | acccacgcagggagttaaggatc | 140049 | - | see also 14115 line |
| 32198 | CDH26 | NM_021810.3 | 276 | cccacgcagggagttaaggatct | 140050 | - | see also 14115 line |
| 32199 | CDH3 | NM_001793.2 | 2149 | cacccgtgacaacgtcttctact | 140139 | - | see also 14116 line |
| 32200 | CDH3 | NM_001793.2 | 2151 | cccgtgacaacgtcttctactat | 140140 | - | see also 14116 line |
| 32201 | CDH4 | NM_001794.2 | 2007 | acccgcctgaacggtgactatgc | 140170 | - | see also 1165 line |
| 32202 | CDH4 | NM_001794.2 | 1584 | accgtgctgaccacgttttcagc | 140162 | - | see also 1165 line |
| 32203 | CDH5 | NM_001795.2 | 737 | gacgtattatcacaataacgaaa | 140192 | - | see also 14118 line |
| 32204 | CDH5 | NM_001795.2 | 1641 | aacaccacgaaacgtgaagttca | 140204 | - | see also 14118 line |
| 32205 | CDH5 | NM_001795.2 | 724 | atcgataattctggacgtattat | 140189 | - | see also 14118 line |
| 32206 | CDH6 | NM_004932.2 | 700 | tccttcgagctcaagctataaac | 140221 | - | see also 3431 line |
| 32207 | CDH6 | NM_004932.2 | 1673 | aacactgctatggcacaacatta | 140238 | - | see also 3431 line |
| 32208 | CDH7 | NM_004361.2 | 688 | ggcgaaggggcaagttccatttt | 140267 | - | see also 2920 line |
| 32209 | CDH7 | NM_004361.2 | 2299 | ctccttatcgtcactatgagaag | 140305 | - | see also 2920 line |
| 32210 | CDH8 | NM_001796.2 | 1374 | cacggcgacagtcaaaatcgtgg | 140354 | - | see also 1166 line |
| 32211 | CDH8 | NM_001796.2 | 1370 | aagacacggcgacagtcaaaatc | 140353 | - | see also 1166 line |
| 32212 | CDH8 | NM_001796.2 | 1685 | cacgagtacctgttgctattaaa | 140366 | - | see also 1166 line |
| 32213 | CDH9 | NM_016279.2 | 1487 | gcccttgaccgggaatcatctcc | 140429 | - | see also 14122 line |
| 32214 | CDH9 | NM_016279.2 | 958 | tccccagagtacgtatcaattta | 140415 | - | see also 14122 line |
| 32215 | CDH9 | NM_016279.2 | 669 | ctggagtcggtacatctgttata | 140405 | - | see also 14122 line |
| 32216 | CEGF3 | NM_152753.2 | 1923 | cccgcagggaacgtattaccacg | 140488 | - | see also 14123 line |
| 32217 | CEGF3 | NM_152753.2 | 662 | cccggtgcggctgccatatcaag | 140461 | - | see also 14123 line |
| 32218 | CELSR1 | NM_014246.1 | 4870 | ctgtcagtcgacggcaaaaatgt | 140523 | - | see also 5554 line |
| 32219 | CELSR1 | NM_014246.1 | 4900 | gccggattcatcgccaacaatgg | 140525 | - | see also 5554 line |
| 32220 | CELSR2 | NM_001408.1 | 568 | ggcgtcggaaaaggaatgtaaat | 140551 | - | see also 14125 line |
| 32221 | CELSR2 | NM_001408.1 | 6831 | gggctactgcctcataactatga | 140595 | - | see also 14125 line |
| 32222 | CELSR3 | NM_001407.1 | 4844 | gcccagttcgttcgtcatgtttc | 140651 | - | see also 975 line |
| 32223 | CELSR3 | NM_001407.1 | 5398 | tggcggcttttgtcgcaaataat | 140660 | - | see also 975 line |
| 32224 | CELSR3 | NM_001407.1 | 9414 | gcctcttacggtcgcatctatgc | 140684 | - | see also 975 line |

Figure 46

| 32225 | CETN1 | NM_004066.1 | 71 | ctgcctccaccggccaaaagaga | 140688 | - | see also 14127 line | | |
|---|---|---|---|---|---|---|---|---|---|
| 32226 | CETN1 | NM_004066.1 | 312 | ggccgtgatgacgcagaagatgt | 140690 | - | see also 14127 line | | |
| 32227 | CETN2 | NM_004344.1 | 83 | aagttctcagcgaaaaagaatga | 140691 | - | see also 2903 line | | |
| 32228 | CETN2 | NM_004344.1 | 179 | aactggcaccatagatgttaaag | 140694 | - | see also 2903 line | | |
| 32229 | CETN3 | NM_004365.1 | 416 | ttcgagctatgatagaagaattt | 140707 | - | see also 14129 line | | |
| 32230 | CETN3 | NM_004365.1 | 368 | tgcgacgtgttgctagagaattg | 140706 | - | see also 14129 line | | |
| 32231 | CGREF1 | NM_006569.2 | 770 | aaggcagtccctattagctaaaa | 140710 | - | cell_cycle | - | - |
| 32232 | CGREF1 | NM_006569.2 | 281 | gacgatgacagtgttaatcctgc | 140709 | - | see also 32231 line | | |
| 32233 | CGREF1 | NM_006569.2 | 771 | aggcagtccctattagctaaaag | 140711 | - | see also 32231 line | | |
| 32234 | CHGA | NM_001275.2 | 300 | taccgaggtgatgaaatgcatcg | 140715 | - | see also 14130 line | | |
| 32235 | CHGA | NM_001275.2 | 496 | aagaaacacagcggttttgaaga | 140718 | - | see also 14130 line | | |
| 32236 | CHP | NM_007236.3 | 568 | atggtcggagtaaatatctcaga | 140725 | - | see also 14131 line | | |
| 32237 | CHP | NM_007236.3 | 534 | ctcccgtgatgagctgttacagg | 140724 | - | see also 14131 line | | |
| 32238 | CLSTN1 | NM_014944.1 | 2762 | ctgagtggcgtccaccattttgc | 140764 | - | see also 14132 line | | |
| 32239 | CLSTN1 | NM_014944.1 | 3189 | gccgctacatcagcaacgaattt | 140768 | - | see also 14132 line | | |
| 32240 | CLSTN2 | NM_022131.1 | 105 | tcctcgcggctaaagtcaataag | 140776 | - | see also 8296 line | | |
| 32241 | CLSTN2 | NM_022131.1 | 104 | ctcctcgcggctaaagtcaataa | 140775 | - | see also 8296 line | | |
| 32242 | CLSTN3 | NM_014718.1 | 2656 | cacacgcatgtcggatgagattg | 140823 | - | see also 5912 line | | |
| 32243 | CLSTN3 | NM_014718.1 | 695 | gacaacacggtcctactgaatcc | 140811 | - | see also 5912 line | | |
| 32244 | CLSTN3 | NM_014718.1 | 2554 | aaccaacggcgtccctttgttcc | 140822 | - | see also 5912 line | | |
| 32245 | CLTA | NM_001833.1 | 290 | gggggtccggatgctgttgatgg | 140834 | - | see also 14135 line | | |
| 32246 | CLTA | NM_001833.1 | 329 | tactaccaggaaagtaatggtcc | 140836 | - | see also 14135 line | | |
| 32247 | CLTB | NM_001834.2 | 648 | aggaggctttcgtgaaggaatcc | 140845 | - | see also 14136 line | | |
| 32248 | CPNE6 | NM_006032.2 | 540 | atcaggcacaaacgactatgtgc | 140851 | - | see also 14137 line | | |
| 32249 | CPNE6 | NM_006032.2 | 640 | accaacgaggaccaaagtgatca | 140854 | - | see also 14137 line | | |
| 32250 | CRB1 | NM_012076.1 | 997 | tgcacgggtagtggattcacagg | 140866 | - | see also 5111 line | | |
| 32251 | CRB1 | NM_012076.1 | 1147 | atcgacctcaatgaatgcaatag | 140871 | - | see also 5111 line | | |
| 32252 | CRELD1 | NM_015513.2 | 1214 | ggccaggtcgctgtaagaagtgt | 140962 | - | see also 6446 line | | |
| 32253 | CRELD1 | NM_015513.2 | 974 | acgccagccatctggtatgttcg | 140957 | - | see also 6446 line | | |
| 32254 | CRTAC1 | NM_018058.1 | 735 | caccgccgactttgacaatgacc | 140967 | - | see also 14140 line | | |
| 32255 | CRTAC1 | NM_018058.1 | 380 | aggctggggtcagcaaatataca | 140964 | - | see also 14140 line | | |
| 32256 | CSN2 | NM_001891.1 | 220 | ttgaacctatcccctatggtttt | 140974 | - | see also 14141 line | | |
| 32257 | CSN2 | NM_001891.1 | 313 | aagtccctaaagctaaagacact | 140976 | - | see also 14141 line | | |
| 32258 | CSPG2 | NM_004385.2 | 657 | gacgtcatgtacgggattgaaga | 140989 | - | see also 2948 line | | |
| 32259 | CSPG2 | NM_004385.2 | 3295 | ctcgccttgaagcgactatttct | 141060 | - | see also 2948 line | | |
| 32260 | CSPG3 | NM_004386.1 | 639 | accgcactgttcggtatcctatc | 141260 | - | see also 14143 line | | |
| 32261 | CSPG3 | NM_004386.1 | 211 | ccgagatgcccctcggataaagt | 141259 | - | see also 14143 line | | |

Figure 46

| 32262 | CUBN | NM_001081.2 | 3105 | gactccgacctcgcttatgaagg | 141373 | - | see also 14144 line |
|---|---|---|---|---|---|---|---|
| 32263 | CUBN | NM_001081.2 | 2881 | accccacggtatcaactgtact | 141367 | - | see also 14144 line |
| 32264 | DCSTAMP | NM_030788.2 | 417 | atggtatgacttgcaacctaagg | 141586 | - | see also 14145 line |
| 32265 | DCSTAMP | NM_030788.2 | 510 | ttgccactccactaagtgtattt | 141587 | - | see also 14145 line |
| 32266 | DEF6 | NM_022047.2 | 1681 | aaccggctgatgcatccaattga | 141618 | - | see also 8247 line |
| 32267 | DEF6 | NM_022047.2 | 987 | ggggaagacgtccctacacaagg | 141614 | - | see also 8247 line |
| 32268 | DGKA | NM_001345.3 | 1222 | ctggaccggatcgtaaaaatagc | 141632 | - | see also 14147 line |
| 32269 | DGKA | NM_001345.3 | 1225 | gaccggatcgtaaaaatagcaaa | 141633 | - | see also 14147 line |
| 32270 | DGKB | NM_004080.1 | 2045 | gccatcgacgaatagagaaaaaa | 141716 | - | see also 14148 line |
| 32271 | DGKB | NM_004080.1 | 2043 | aagccatcgacgaatagagaaaa | 141714 | - | see also 14148 line |
| 32272 | DGKG | NM_001346.1 | 650 | acccacatgagccgattagctat | 141727 | - | see also 891 line |
| 32273 | DGKG | NM_001346.1 | 651 | cccacatgagccgattagctatg | 141728 | - | see also 891 line |
| 32274 | DKFZp566D234 | NM_020116.2 | 937 | atcctaatggcgacgacatatct | 141775 | - | see also 14149 line |
| 32275 | DKFZp566D234 | NM_020116.2 | 2895 | atcctagatggacgactcaataa | 141827 | - | see also 14149 line |
| 32276 | DKFZp566D234 | NM_020116.2 | 2896 | tcctagatggacgactcaataaa | 141828 | - | see also 14149 line |
| 32277 | DKFZp586G0123 | NM_013386.2 | 1419 | tggcctctttcgacgaattattt | 141872 | - | see also 14150 line |
| 32278 | DKFZp586G0123 | NM_013386.2 | 803 | tgcaggttcacggttcaaaatca | 141844 | - | see also 14150 line |
| 32279 | DLK1 | NM_003836.3 | 470 | tggacgatggcctctatgaatgc | 141884 | - | see also 14151 line |
| 32280 | DLK1 | NM_003836.3 | 394 | gacggggagctctgtgatagaga | 141883 | - | see also 14151 line |
| 32281 | DLL1 | NM_005618.2 | 2429 | accagtcggtgtacgtcatatcc | 141903 | - | see also 14152 line |
| 32282 | DLL1 | NM_005618.2 | 1428 | tgcggacggcccttgctttaacg | 141894 | - | see also 14152 line |
| 32283 | DLL1 | NM_005618.2 | 1427 | gtgcggacggcccttgctttaac | 141893 | - | see also 14152 line |
| 32284 | DLL3 | NM_016941.1 | 1674 | gtccgagctcgtccgtagattgg | 141908 | - | see also 14153 line |
| 32285 | DLL3 | NM_016941.1 | 631 | accgctcgaggacgaatgtgagg | 141906 | - | see also 14153 line |
| 32286 | DLL4 | NM_019074.2 | 2268 | aagccagagtgtcggatatcagc | 141931 | - | see also 14154 line |
| 32287 | DLL4 | NM_019074.2 | 1380 | tactatggcctgcattgtgaaca | 141923 | - | see also 14154 line |
| 32288 | DMP1 | NM_004407.1 | 1201 | cccgaccccacaactagttatgt | 141948 | - | see also 14155 line |
| 32289 | DMP1 | NM_004407.1 | 1200 | ccccgaccccacaactagttatg | 141947 | - | see also 14155 line |
| 32290 | DNER | NM_139072.2 | 2122 | gccgcatcagccgcattgaatac | 141988 | - | see also 9657 line |
| 32291 | DNER | NM_139072.2 | 1702 | acctcgttaatggctatgagtgt | 141982 | - | see also 9657 line |
| 32292 | DSC1 | NM_004948.2 | 2302 | aagagacgttagaccaaatgtaa | 142051 | - | see also 14157 line |

Figure 46

| | | | | | | | |
|---|---|---|---|---|---|---|---|
| 32293 | DSC1 | NM_004948.2 | 1973 | aacgatcacgcacctcaaattga | 142041 | - | see also 14157 line |
| 32294 | DSC2 | NM_004949.2 | 503 | gaccctcgcgatcttaatatttg | 142074 | - | see also 14158 line |
| 32295 | DSC2 | NM_004949.2 | 925 | ctgacacggcccaaaactatacc | 142088 | - | see also 14158 line |
| 32296 | DSC2 | NM_004949.2 | 504 | accctcgcgatcttaatatttgc | 142075 | - | see also 14158 line |
| 32297 | DSC3 | NM_001941.2 | 1831 | gggtataccgacatttttagctgt | 142215 | - | see also 14159 line |
| 32298 | DSC3 | NM_001941.2 | 1828 | atggggtataccgacattttagc | 142214 | - | see also 14159 line |
| 32299 | DSG1 | NM_001942.1 | 1813 | aacggagccaaagatttgttatc | 142302 | - | see also 14160 line |
| 32300 | DSG1 | NM_001942.1 | 2870 | tgcgagatgggtcgaatgttata | 142335 | - | see also 14160 line |
| 32301 | DSG2 | NM_001943.1 | 874 | aagttacgcgcataaaagtgttc | 142376 | - | see also 14161 line |
| 32302 | DSG2 | NM_001943.1 | 431 | acccttagagctacgcattaagg | 142358 | - | see also 14161 line |
| 32303 | DSG3 | NM_001944.1 | 3025 | agctacgagggtcacatactatg | 142525 | - | see also 1239 line |
| 32304 | DSG3 | NM_001944.1 | 1511 | ttctacaggcacggtatatgtta | 142482 | - | see also 1239 line |
| 32305 | DSG3 | NM_001944.1 | 1518 | ggcacggtatatgttagagtacc | 142484 | - | see also 1239 line |
| 32306 | DSG4 | NM_177986.2 | 2573 | ctcccgtaggctctattggttgt | 142606 | - | see also 10174 line |
| 32307 | DSG4 | NM_177986.2 | 2519 | aaggtcgaccagccaatgactgc | 142605 | - | see also 10174 line |
| 32308 | DSG4 | NM_177986.2 | 994 | ctgatacgattacaagcaattga | 142560 | - | see also 10174 line |
| 32309 | DUOX1 | NM_017434.3 | 2089 | ctctgttgttcgtgactattttg | 142641 | - | see also 14164 line |
| 32310 | DUOX1 | NM_017434.3 | 2197 | ccggctccggatgagaaatttca | 142643 | - | see also 14164 line |
| 32311 | DUOX2 | NM_014080.3 | 310 | agcgctatgacggctggtttaac | 142653 | - | see also 14165 line |
| 32312 | DUOX2 | NM_014080.3 | 2773 | aggataagtcccgtctaatgttt | 142659 | - | see also 14165 line |
| 32313 | EDEM1 | NM_014674.1 | 1898 | aggccgaaacctcatgagttaaa | 142714 | - | see also 5857 line |
| 32314 | EDEM1 | NM_014674.1 | 1070 | aacctccggagcaatgatacagg | 142691 | - | see also 5857 line |
| 32315 | EDIL3 | NM_005711.3 | 702 | taccgaggggatacattcatagg | 142725 | - | see also 14167 line |
| 32316 | EDIL3 | NM_005711.3 | 990 | ccctactatgcacgtcttaataa | 142735 | - | see also 14167 line |
| 32317 | EDIL3 | NM_005711.3 | 698 | agcataccgaggggatacattca | 142724 | - | see also 14167 line |
| 32318 | EFCBP1 | NM_022351.2 | 367 | gtcgatcttcctcgacatactga | 142764 | - | see also 8342 line |
| 32319 | EFCBP1 | NM_022351.2 | 890 | agcggtccaggcttattagaaga | 142778 | - | see also 8342 line |
| 32320 | EFEMP1 | NM_004105.2 | 820 | gccaccaaagatgcgtgaataca | 142809 | - | see also 2773 line |
| 32321 | EFEMP1 | NM_004105.2 | 1218 | ttccgttgttatccacgaaatcc | 142822 | - | see also 2773 line |
| 32322 | EFEMP1 | NM_004105.2 | 1338 | tacatgagcatccgatctgatag | 142825 | - | see also 2773 line |
| 32323 | EFEMP2 | NM_016938.1 | 1189 | gtctaccccggtgcctacaatgc | 142839 | - | see also 14169 line |
| 32324 | EFEMP2 | NM_016938.1 | 676 | aacaaccgctcctgtgttgatgt | 142837 | - | see also 14169 line |
| 32325 | EFHC1 | NM_018100.1 | 1603 | accggttcatcatccttgataca | 142901 | - | see also 14170 line |
| 32326 | EFHC1 | NM_018100.1 | 1153 | ctcgacggtattacaaagagaag | 142889 | - | see also 14170 line |
| 32327 | EGF | NM_001963.2 | 2963 | agcatgtatgctcggtgtatttc | 142987 | - | see also 14171 line |
| 32328 | EGF | NM_001963.2 | 2734 | gtgttcgtgtcgtgaaggtttta | 142980 | - | see also 14171 line |
| 32329 | EGF | NM_001963.2 | 2737 | ttcgtgtcgtgaaggttttatga | 142981 | - | see also 14171 line |

Figure 46

| 32330 | EGFL6 | NM_015507.2 | 876 | tggacgatatgactgtatagata | 143020 | - | see also 14172 line |
|---|---|---|---|---|---|---|---|
| 32331 | EGFL6 | NM_015507.2 | 1663 | gtcgggaaacttcgagtgtttgt | 143036 | - | see also 14172 line |
| 32332 | EGFL7 | NM_016215.2 | 502 | agcacctaccgaaccatctatag | 143040 | - | see also 14173 line |
| 32333 | EGFL9 | NM_023932.1 | 545 | cacctgccttgacggcataaacc | 143042 | - | see also 14174 line |
| 32334 | ELA1 | NM_001971.3 | 394 | cccagagcgttaccctcaatagc | 143050 | - | see also 14175 line |
| 32335 | ELA1 | NM_001971.3 | 160 | ctggaggttcccggtatcacacc | 143045 | - | see also 14175 line |
| 32336 | EML2 | NM_012155.1 | 287 | ggcctccgtagccgtgctataca | 143061 | - | see also 14176 line |
| 32337 | EML2 | NM_012155.1 | 264 | accggggagatagtgtactttgt | 143060 | - | see also 14176 line |
| 32338 | EMR1 | NM_001974.3 | 1577 | tcgagtcgttgggggcataatga | 143117 | - | see also 1257 line |
| 32339 | EMR1 | NM_001974.3 | 1315 | cggctgttcggacggaatactta | 143105 | - | see also 1257 line |
| 32340 | EMR2 | NM_013447.2 | 2212 | ttcctcgtggcaattgatcaaac | 143147 | - | see also 14178 line |
| 32341 | EMR2 | NM_013447.2 | 2213 | tcctcgtggcaattgatcaaacc | 143148 | - | see also 14178 line |
| 32342 | EMR3 | NM_032571.2 | 815 | ctcaagcgattacagacaattgc | 143167 | - | see also 14179 line |
| 32343 | EMR3 | NM_032571.2 | 793 | aacgatagtgtagctattgaaac | 143166 | - | see also 14179 line |
| 32344 | EPS15 | NM_001981.1 | 2069 | caggcaatctactgatccttttg | 143234 | - | see also 1263 line |
| 32345 | EPS15 | NM_001981.1 | 2329 | ctgtcagcaacgtagtgattaca | 143239 | - | see also 1263 line |
| 32346 | EPS15R | NM_021235.1 | 294 | gagcatgccaccgcctaaatttc | 143267 | - | see also 14181 line |
| 32347 | EPS15R | NM_021235.1 | 995 | aagaccaattcgcgttagctatg | 143271 | - | see also 14181 line |
| 32348 | ERO1L | NM_014584.1 | 1190 | ttcgagcgcccagattttcaact | 143329 | - | see also 5764 line |
| 32349 | ERO1L | NM_014584.1 | 1353 | aggactttcgactgcattttaga | 143332 | - | see also 5764 line |
| 32350 | F10 | NM_000504.2 | 356 | acctgtttagaaggattcgaagg | 143344 | - | see also 14183 line |
| 32351 | F10 | NM_000504.2 | 1466 | gtcataacgtcctctccattaaa | 143346 | - | see also 14183 line |
| 32352 | F12 | NM_000505.2 | 213 | ggcagctgtaccacaaatgtacc | 143353 | - | see also 14184 line |
| 32353 | F12 | NM_000505.2 | 123 | cccccaaggagcataagtacaaa | 143350 | - | see also 14184 line |
| 32354 | F13A1 | NM_000129.2 | 1594 | ccctgatgtacggagctaaaaag | 143395 | - | see also 115 line |
| 32355 | F13A1 | NM_000129.2 | 986 | aagcgttgacattctattggaat | 143381 | - | see also 115 line |
| 32356 | F2 | NM_000506.2 | 1327 | aaggtacgagcgaaacattgaaa | 143413 | - | see also 14186 line |
| 32357 | F2 | NM_000506.2 | 311 | aggacgcctcgagataagcttgc | 143403 | - | see also 14186 line |
| 32358 | F7 | NM_000131.2 | 803 | cggcccactgtttcgacaaaatc | 143424 | - | see also 14187 line |
| 32359 | F7 | NM_000131.2 | 804 | ggcccactgtttcgacaaaatca | 143425 | - | see also 14187 line |
| 32360 | F9 | NM_000133.2 | 1011 | aacagctacgttacacctatttg | 143467 | - | see also 14188 line |
| 32361 | F9 | NM_000133.2 | 495 | tcctgtactgagggatatcgact | 143446 | - | see also 14188 line |
| 32362 | FAT | NM_005245.1 | 10906 | caggacgtgtatgatactctaac | 143803 | - | see also 14189 line |
| 32363 | FAT | NM_005245.1 | 10382 | ttctcgaccgagaaacgatttca | 143789 | - | see also 14189 line |
| 32364 | FAT2 | NM_001447.1 | 1742 | tggccagtagggaaatcgataat | 143914 | - | see also 14190 line |
| 32365 | FAT2 | NM_001447.1 | 2881 | cgcaggtgaagtgcgatatgttc | 143946 | - | see also 14190 line |
| 32366 | FBLN1 | NM_001996.2 | 303 | gccatatgctacggaatccaaag | 144139 | - | see also 1279 line |

Figure 46

| 32367 | FBLN1 | NM_001996.2 | 937 | gacgagtgtgagagtggtattca | 144145 | - | see also 1279 line |
| 32368 | FBLN1 | NM_001996.2 | 1052 | ctctaggcaactgtgattgatatc | 144150 | - | see also 1279 line |
| 32369 | FBLN2 | NM_001998.1 | 1668 | gtcgtgtgagtccaatcctaaac | 144173 | - | see also 14192 line |
| 32370 | FBLN2 | NM_001998.1 | 3229 | ttcgagtgtctcccaactatgt | 144183 | - | see also 14192 line |
| 32371 | FBLN5 | NM_006329.2 | 844 | gttggacgagtgtgcaacagattc | 144195 | - | see also 4433 line |
| 32372 | FBLN5 | NM_006329.2 | 845 | tggacgagtgtgcaacagattcc | 144196 | - | see also 4433 line |
| 32373 | FBN1 | NM_000138.2 | 5413 | taccggtttacccgttgatattg | 144342 | - | see also 122 line |
| 32374 | FBN1 | NM_000138.2 | 2355 | accttcgtgggacctataaagt | 144277 | - | see also 122 line |
| 32375 | FBN1 | NM_000138.2 | 5414 | accggtttacccgttgatattga | 144343 | - | see also 122 line |
| 32376 | FBN2 | NM_001999.2 | 7197 | tagtcgcaatctgtcactaagt | 144574 | - | see also 1282 line |
| 32377 | FBN2 | NM_001999.2 | 7201 | cgcaatctcgtcactaagtcaga | 144576 | - | see also 1282 line |
| 32378 | FBN3 | NM_032447.2 | 2446 | cacggatggtcgagacatcaacg | 144599 | - | see also 14195 line |
| 32379 | FBN3 | NM_032447.2 | 3912 | gtgacgtgcactgtattaacact | 144609 | - | see also 14195 line |
| 32380 | FCN1 | NM_002003.2 | 1013 | tgcgtcgaaggggtacaaatata | 144646 | - | see also 14196 line |
| 32381 | FCN1 | NM_002003.2 | 932 | cgccgactgtcatgcttcaaacc | 144643 | - | see also 14196 line |
| 32382 | FIBL-6 | NM_031935.1 | 11226 | cacctcgagtgcgaatcctatct | 144987 | - | see also 14197 line |
| 32383 | FIBL-6 | NM_031935.1 | 2157 | ttgcctgaccgttaacgatatg | 144714 | - | see also 14197 line |
| 32384 | FKBP10 | NM_021939.2 | 1166 | ctctgccgtgctaatcttcaacg | 145143 | - | see also 14198 line |
| 32385 | FKBP10 | NM_021939.2 | 1291 | ttcgataccattacaactgttct | 145148 | - | see also 14198 line |
| 32386 | FKBP14 | NM_017946.1 | 757 | tacatataaacacgatgagttat | 145165 | - | see also 14199 line |
| 32387 | FKBP14 | NM_017946.1 | 293 | gtccactatgaaggctacttaga | 145150 | - | see also 14199 line |
| 32388 | FKBP7 | NM_016105.2 | 594 | tccaccggatgctacattgattt | 145181 | - | see also 6663 line |
| 32389 | FKBP7 | NM_016105.2 | 287 | tggctaaagacggtcggaaattc | 145167 | - | see also 6663 line |
| 32390 | FKBP9 | NM_007270.2 | 1041 | cggcacgtttgacacgtacattgg | 145207 | - | see also 14201 line |
| 32391 | FKBP9 | NM_007270.2 | 878 | tgcattattggacctccataacc | 145202 | - | see also 14201 line |
| 32392 | FKBP9 | NM_007270.2 | 1033 | ctggaaccgcacgtttgacacg | 145206 | - | see also 14201 line |
| 32393 | FLJ11767 | NM_024593.2 | 218 | ttggactggatgtaatgcattt | 145218 | - | see also 14202 line |
| 32394 | FLJ11767 | NM_024593.2 | 106 | ctgacggaccacttaaccaaaaa | 145215 | - | see also 14202 line |
| 32395 | FLJ12443 | NM_024830.3 | 1593 | cccggatcagacacatttcgaaa | 145240 | - | see also 14203 line |
| 32396 | FLJ12443 | NM_024830.3 | 760 | acctgcctaattaccttcaaacc | 145232 | - | see also 14203 line |
| 32397 | FLJ13397 | NM_024948.1 | 277 | ccggtctctcggacaccatttc | 145243 | - | see also 8816 line |
| 32398 | FLJ13397 | NM_024948.1 | 1534 | cagatcgctcccttccactaaat | 145288 | - | see also 8816 line |
| 32399 | FLJ13612 | NM_025202.2 | 688 | gtcatcggccagtaagtttgaag | 145291 | - | see also 14204 line |
| 32400 | FLJ13612 | NM_025202.2 | 788 | ctcaaggccaacttcaatacata | 145293 | - | see also 14204 line |
| 32401 | FLJ20047 | NM_017639.1 | 620 | tggttggaagcacgcttgttaca | 145303 | - | see also 14205 line |
| 32402 | FLJ20047 | NM_017639.1 | 3002 | gtggtacatgccatgaacttaaa | 145359 | - | see also 14205 line |
| 32403 | FLJ20080 | NM_017657.2 | 453 | ttcgataccaccagattatactcg | 145366 | - | see also 7083 line |

Figure 46

| | | | | | | | |
|---|---|---|---|---|---|---|---|
| 32404 | FLJ20080 | NM_017657.2 | 2264 | ccgcctggagcgaattttcgaag | 145402 | - | see also 7083 line |
| 32405 | FLJ20254 | NM_017727.2 | 991 | cagctctacccccgactgaaaat | 145426 | - | see also 14207 line |
| 32406 | FLJ20254 | NM_017727.2 | 766 | aacctcaccgagggactgaaagt | 145423 | - | see also 14207 line |
| 32407 | FLJ20254 | NM_017727.2 | 1964 | ggctttgcctacaggacattaca | 145431 | - | see also 14207 line |
| 32408 | FLJ20298 | NM_017752.2 | 1686 | ggctactaatcctgactattata | 145480 | - | see also 14208 line |
| 32409 | FLJ20298 | NM_017752.2 | 1190 | cactacgagaggtcttagctata | 145457 | - | see also 14208 line |
| 32410 | FLJ22843 | NM_025184.2 | 478 | ccggcgtcatcggattactcttc | 145542 | - | see also 14209 line |
| 32411 | FLJ22843 | NM_025184.2 | 479 | cggcgtcatcggattactcttcc | 145543 | - | see also 14209 line |
| 32412 | FLJ23588 | NM_022785.2 | 1468 | aacccgatggaccgataacaaga | 145676 | - | see also 14210 line |
| 32413 | FLJ23588 | NM_022785.2 | 1778 | agggcgcattggccgaaataatt | 145688 | - | see also 14210 line |
| 32414 | FLJ25200 | NM_144715.2 | 1237 | agcaatcgacgagcaccattagg | 145780 | - | see also 9733 line |
| 32415 | FLJ25200 | NM_144715.2 | 893 | atcgcatcagacccatatactct | 145771 | - | see also 9733 line |
| 32416 | FLJ25818 | NM_173503.1 | 1040 | gtggccgatgcaactgctattaa | 145851 | - | see also 14211 line |
| 32417 | FLJ25818 | NM_173503.1 | 1105 | tgcccttgaacaccgaaaagaga | 145857 | - | see also 14211 line |
| 32418 | FLJ30681 | NM_133459.1 | 164 | agcaaaatcgcgacgactaaata | 145870 | - | see also 14212 line |
| 32419 | FLJ30681 | NM_133459.1 | 135 | aggacggcgacagagaaatctgc | 145869 | - | see also 14212 line |
| 32420 | FLJ31455 | NM_144964.1 | 717 | gactcagaacacgctcttgaaga | 145891 | - | see also 9747 line |
| 32421 | FLJ31455 | NM_144964.1 | 454 | gactatgtatcgatttgagtatg | 145886 | - | see also 9747 line |
| 32422 | FLJ31614 | NM_152573.2 | 2426 | atctaaccgggaccaattccaaa | 145954 | - | see also 14214 line |
| 32423 | FLJ31614 | NM_152573.2 | 1293 | agctaaccggaagctacatgaca | 145923 | - | see also 14214 line |
| 32424 | FLJ32009 | NM_152718.1 | 1748 | gacgacaacggggttgagtttcc | 145965 | - | see also 14215 line |
| 32425 | FLJ32009 | NM_152718.1 | 677 | aaccggcactcctgtgtagatgt | 145958 | - | see also 14215 line |
| 32426 | FLJ34588 | NM_152726.1 | 518 | ttgcttacaatcctcactaaacc | 145978 | - | see also 9932 line |
| 32427 | FLJ34588 | NM_152726.1 | 368 | gagcaaatggaacgtaaaacttc | 145974 | - | see also 9932 line |
| 32428 | FLJ37440 | NM_153214.1 | 530 | cccgcagacggcagaaagtttgg | 145993 | - | see also 14217 line |
| 32429 | FLJ37440 | NM_153214.1 | 567 | tggatcacgaagtccattttacc | 145995 | - | see also 14217 line |
| 32430 | FLJ37874 | NM_182603.1 | 724 | agcagtgcgacgcaagttttaaa | 146009 | - | see also 14218 line |
| 32431 | FLJ37874 | NM_182603.1 | 448 | gaccggggatgcactcctttaca | 146000 | - | see also 14218 line |
| 32432 | FLJ39116 | NM_152635.1 | 590 | ccctggaggctactatgtgtatc | 146027 | - | see also 14219 line |
| 32433 | FLJ39116 | NM_152635.1 | 286 | atccttgttctgcttacatcagc | 146025 | - | see also 14219 line |
| 32434 | FLJ39155 | NM_152403.2 | 2829 | cagttacctgacgtatgacaacc | 146086 | - | see also 9854 line |
| 32435 | FLJ39155 | NM_152403.2 | 2955 | gagacccaacagcgacttcattt | 146091 | - | see also 9854 line |
| 32436 | FLJ40342 | NM_152347.3 | 3091 | tccgatatattgacaattcctaa | 146186 | - | see also 14221 line |
| 32437 | FLJ40342 | NM_152347.3 | 796 | agcgttcacaagacttcatcacc | 146110 | - | see also 14221 line |
| 32438 | FLRT1 | NM_013280.3 | 2332 | gcggcatccccgacatagactac | 146198 | - | see also 14222 line |
| 32439 | FLRT1 | NM_013280.3 | 661 | acgtgcaggtcatctacctatac | 146195 | - | see also 14222 line |
| 32440 | FLRT3 | NM_013281.2 | 1078 | agctctatcgactggatatgtcc | 146224 | - | see also 5384 line |

Figure 46

| 32441 | FLRT3 | NM_013281.2 | 1763 | aactgcacccttcgaatgtaca | 146251 | - | see also 5384 line |
|---|---|---|---|---|---|---|---|
| 32442 | FLRT3 | NM_013281.2 | 1207 | gggtacgtgactggttacaatca | 146232 | - | see also 5384 line |
| 32443 | FRAS1 | NM_025074.2 | 11962 | gaggcggcttacacgttcaaagg | 146508 | - | see also 8843 line |
| 32444 | FRAS1 | NM_025074.2 | 8226 | tgccgcgagtcgtgtgatattcg | 146404 | - | see also 8843 line |
| 32445 | FREQ | NM_014286.2 | 405 | ctctacgacttggacaatgatgg | 146514 | - | see also 5593 line |
| 32446 | FREQ | NM_014286.2 | 229 | tccagaagatctacaagcaattc | 146513 | - | see also 5593 line |
| 32447 | FSTL1 | NM_007085.3 | 167 | aagatctgtgccaatgtgttttg | 146516 | - | see also 14225 line |
| 32448 | FSTL1 | NM_007085.3 | 781 | tgccctggaggatgaaacgtatg | 146522 | - | see also 14225 line |
| 32449 | GAS6 | NM_000820.1 | 431 | tgggtctccgtacaccaaaaact | 146528 | - | see also 14226 line |
| 32450 | GAS6 | NM_000820.1 | 375 | aacgaccccgagacggattattt | 146526 | - | see also 14226 line |
| 32451 | GAS6 | NM_000820.1 | 1392 | aacctgaccgtgggaggtattcc | 146534 | - | see also 14226 line |
| 32452 | GCA | NM_012198.2 | 202 | agctatactcctcgatggatact | 146540 | - | see also 14227 line |
| 32453 | GCA | NM_012198.2 | 656 | gtgaagcttcgagcattgacaga | 146554 | - | see also 14227 line |
| 32454 | GPD2 | NM_000408.1 | 2069 | agcgtgtattagagagtatcaat | 146615 | - | see also 323 line |
| 32455 | GRN | NM_002087.1 | 439 | gccctgatagtcagttcgaatgc | 146630 | - | see also 14229 line |
| 32456 | GRN | NM_002087.1 | 470 | ctccacgtgctgtgttatggtcg | 146631 | - | see also 14229 line |
| 32457 | GRN | NM_002087.1 | 818 | gtgtgacctgatccagagtaagt | 146632 | - | see also 14229 line |
| 32458 | GUCA1B | NM_002098.2 | 100 | agcggcacactctttatgcatga | 146642 | - | see also 14230 line |
| 32459 | GUCA1B | NM_002098.2 | 95 | gccccagcggcacactctttatg | 146640 | - | see also 14230 line |
| 32460 | HGF | NM_000601.3 | 1951 | tacgagtggcacatctctatata | 146718 | - | see also 14231 line |
| 32461 | HGF | NM_000601.3 | 923 | aacatgcgctgacaatactatga | 146675 | - | see also 14231 line |
| 32462 | HGF | NM_000601.3 | 1533 | acgaaacaattgcgagttgtaaa | 146705 | - | see also 14231 line |
| 32463 | HPCAL4 | NM_016257.2 | 419 | agctcaactgggcctttgagatg | 146728 | - | see also 14232 line |
| 32464 | HPCAL4 | NM_016257.2 | 563 | agcagcgtgtggacaagatcttc | 146729 | - | see also 14232 line |
| 32465 | HRC | NM_002152.1 | 987 | caccaaggccacgggattgaaga | 146732 | | see also 1383 line |
| 32466 | HUMMLC2B | NM_013292.2 | 503 | cggcaacgtcgactacaaaaaca | 146747 | - | see also 14234 line |
| 32467 | HUMMLC2B | NM_013292.2 | 502 | gcggcaacgtcgactacaaaaac | 146746 | - | see also 14234 line |
| 32468 | IPO4 | NM_024658.2 | 785 | tgcgatacgcatacgtattctct | 146755 | - | see also 8687 line |
| 32469 | IPO4 | NM_024658.2 | 990 | aagcatttcgctgtacaagttgt | 146758 | - | see also 8687 line |
| 32470 | ITIH1 | NM_002215.1 | 343 | aacgcatttatcggagacataaa | 146778 | - | see also 14236 line |
| 32471 | ITIH1 | NM_002215.1 | 213 | ctctcgcttcgcccactatgttg | 146776 | - | see also 14236 line |
| 32472 | ITSN1 | NM_003024.1 | 1038 | ttcgatctggcagtggtatatct | 146833 | - | see also 2024 line |
| 32473 | ITSN1 | NM_003024.1 | 4736 | cacattgaccgcgtctatactct | 146900 | - | see also 2024 line |
| 32474 | ITSN2 | NM_006277.1 | 1602 | aacgacaacgtcgcttagaatgg | 146937 | - | see also 4363 line |
| 32475 | ITSN2 | NM_006277.1 | 360 | caggtgatcaagcacgtaatttt | 146909 | - | see also 4363 line |

Figure 46

| | | | | | | | |
|---|---|---|---|---|---|---|---|
| 32476 | ITSN2 | NM_006277.1 | 2454 | aacgtaaggataaggatactttg | 146958 | - | see also 4363 line |
| 32477 | JAG1 | NM_000214.1 | 2692 | tggtcaacggcgagtcctttacg | 147078 | - | see also 162 line |
| 32478 | JAG1 | NM_000214.1 | 934 | cgggcgttgcccactttgagtat | 147029 | - | see also 162 line |
| 32479 | JAG2 | NM_002226.3 | 902 | gtcgcatgccggcatgatcaacc | 147112 | - | see also 14240 line |
| 32480 | JAG2 | NM_002226.3 | 991 | tgcgctgcgacgagaactactac | 147113 | - | see also 14240 line |
| 32481 | KCNIP1 | NM_014592.2 | 861 | ttgtaaccgctctgtcgatttta | 147139 | - | see also 14241 line |
| 32482 | KCNIP1 | NM_014592.2 | 865 | aaccgctctgtcgattttattga | 147140 | - | see also 14241 line |
| 32483 | KCNIP2 | NM_014591.3 | 602 | agcgtggacgatgaatttgaatt | 147156 | - | see also 14242 line |
| 32484 | KCNIP2 | NM_014591.3 | 601 | cagcgtggacgatgaatttgaat | 147155 | - | see also 14242 line |
| 32485 | KIAA0494 | NM_014774.1 | 1863 | aacgagacagtcagtaatcttac | 147188 | - | see also 14243 line |
| 32486 | KIAA0494 | NM_014774.1 | 985 | agcgcaaagagctcaatgcattg | 147168 | - | see also 14243 line |
| 32487 | KIAA0676 | NM_198868.1 | 1061 | gacgcttgccacctcatcatacc | 147211 | - | see also 14244 line |
| 32488 | KIAA0676 | NM_198868.1 | 2609 | ctggagcagtaccggattgatgc | 147222 | - | see also 14244 line |
| 32489 | KIAA0980 | NM_025176.3 | 2411 | cgcccagatgtgcgtatcgttgg | 147239 | - | see also 14245 line |
| 32490 | KIAA0980 | NM_025176.3 | 193 | accatttcgccagggttaacttt | 147228 | - | see also 14245 line |
| 32491 | KIP3 | NM_054113.1 | 119 | agctcgtgcccctcgactatacc | 147258 | - | see also 14246 line |
| 32492 | KIP3 | NM_054113.1 | 43 | gcgtatcaggactgcacattttt | 147256 | - | see also 14246 line |
| 32493 | LALBA | NM_002289.1 | 241 | tccagatcagtaataagctttgg | 147267 | - | see also 14247 line |
| 32494 | LALBA | NM_002289.1 | 240 | ttccagatcagtaataagctttg | 147266 | - | see also 14247 line |
| 32495 | LALBA | NM_002289.1 | 76 | tcctggccaagcaattcacaaaa | 147260 | - | see also 14247 line |
| 32496 | LDLR | NM_000527.2 | 2156 | accccactcgcccaagtttacc | 147295 | - | see also 14248 line |
| 32497 | LDLR | NM_000527.2 | 795 | cacctgtcgccctgacgaattcc | 147271 | - | see also 14248 line |
| 32498 | LETM1 | NM_012318.1 | 2147 | ggcagatcgatggcttgatctcg | 147311 | - | see also 5274 line |
| 32499 | LETM1 | NM_012318.1 | 2309 | tggccgcagcactggatgaaaac | 147312 | - | see also 5274 line |
| 32500 | LOC286097 | NM_181723.1 | 653 | ctcatcgaagctatttcgaaatc | 147328 | - | see also 14250 line |
| 32501 | LOC286097 | NM_181723.1 | 651 | ggctcatcgaagctatttcgaaa | 147327 | - | see also 14250 line |
| 32502 | LOC286097 | NM_181723.1 | 656 | atcgaagctatttcgaaatctta | 147329 | - | see also 14250 line |
| 32503 | LOC63928 | NM_022097.1 | 607 | accccctgggagaccgaattata | 147356 | - | see also 14251 line |
| 32504 | LOC63928 | NM_022097.1 | 608 | ccccctgggagaccgaattatag | 147357 | - | see also 14251 line |
| 32505 | LPA | NM_005577.1 | 11828 | atcggaggatcccattatactat | 147371 | - | see also 14252 line |
| 32506 | LPA | NM_005577.1 | 10642 | ttcgtccctccgaatgttattct | 147363 | - | see also 14252 line |
| 32507 | LRP1 | NM_002332.1 | 679 | atgccagccaaacgagcataact | 147379 | - | see also 1491 line |
| 32508 | LRP1 | NM_002332.1 | 10466 | tgcacggctagccagtttgtatg | 147480 | - | see also 1491 line |
| 32509 | LRP2 | NM_004525.1 | 6619 | aagttctgcggatcaatactact | 147731 | - | see also 14254 line |

Figure 46

| | | | | | | | |
|---|---|---|---|---|---|---|---|
| 32510 | LRP2 | NM_004525.1 | 3569 | ttgccccaatcatcgatgtattg | 147634 | - | see also 14254 line |
| 32511 | LRP2 | NM_004525.1 | 1131 | agccgtacctgtgttgagtttga | 147545 | - | see also 14254 line |
| 32512 | LRP8 | NM_004631.2 | 1881 | ccctgcgagggttcatgtattg | 147954 | - | see also 3146 line |
| 32513 | LRP8 | NM_004631.2 | 1885 | tgcgaggttcatgtattggtct | 147956 | - | see also 3146 line |
| 32514 | LTBP3 | NM_021070.2 | 2849 | ggggcgaccactgcgaaatctac | 147979 | - | see also 14256 line |
| 32515 | LTBP3 | NM_021070.2 | 2400 | cagcaacacgccaggatcttcc | 147976 | - | see also 14256 line |
| 32516 | MAN1B1 | NM_007230.1 | 119 | gccgcctcatcgggacttcatct | 147981 | - | see also 14257 line |
| 32517 | MAN1B1 | NM_007230.1 | 1216 | ccgtctcacagcggataagaag | 147994 | - | see also 14257 line |
| 32518 | MAN1C1 | NM_020379.1 | 920 | acgaactccgtccactaacaaaa | 148009 | - | see also 14258 line |
| 32519 | MAN1C1 | NM_020379.1 | 944 | atggctacgagggtaacatgttc | 148011 | - | see also 14258 line |
| 32520 | MASP1 | NM_001879.3 | 110 | caccgtggagctaaacaatatgt | 148027 | - | see also 14259 line |
| 32521 | MASP1 | NM_001879.3 | 108 | caacacgtggagctaaacaatat | 148026 | - | see also 14259 line |
| 32522 | MASP2 | NM_006610.2 | 494 | accacctggccgtttctactgc | 148081 | - | see also 14260 line |
| 32523 | MASP2 | NM_006610.2 | 320 | ccctggcaaggacacttctac | 148078 | - | see also 14260 line |
| 32524 | MATN2 | NM_002380.2 | 1196 | aacgtgcacaaagatagactact | 148136 | - | see also 1534 line |
| 32525 | MATN2 | NM_002380.2 | 677 | acgggacacaggcatcctaatct | 148130 | - | see also 1534 line |
| 32526 | MATN4 | NM_003833.2 | 1071 | gagcgtgcgtccacaaaacttcg | 148166 | - | see also 2613 line |
| 32527 | MCFD2 | NM_139279.2 | 226 | tgcacgaccaagagcatatcatg | 148169 | - | see also 14263 line |
| 32528 | MCFD2 | NM_139279.2 | 197 | cggcagcaatgggcctggataaga | 148168 | - | see also 14263 line |
| 32529 | MCSC | NM_052901.1 | 535 | aagcattccacgatctttgatgt | 148180 | - | see also 14264 line |
| 32530 | MCSC | NM_052901.1 | 103 | cagaccgagttccagtactttga | 148173 | - | see also 14264 line |
| 32531 | MGC11256 | NM_024324.2 | 987 | aacgaaaactgctacaatactcc | 148195 | - | see also 14265 line |
| 32532 | MGC11256 | NM_024324.2 | 401 | gagcgaatatctgacttattcg | 148187 | - | see also 14265 line |
| 32533 | MGC12458 | NM_032328.1 | 232 | ctggatacattcgattcgaaaaa | 148200 | - | see also 9164 line |
| 32534 | MGC12458 | NM_032328.1 | 233 | tggatacattcgattcgaaaaat | 148201 | - | see also 9164 line |
| 32535 | MGC2615 | NM_024103.1 | 1022 | ccccgagtcagctatcaagttc | 148221 | - | see also 14267 line |
| 32536 | MGC2615 | NM_024103.1 | 1023 | cccgagtcagctatcaagttca | 148222 | - | see also 14267 line |
| 32537 | MGC2615 | NM_024103.1 | 1400 | tcctgcctgcggtaccatatcc | 148225 | - | see also 14267 line |
| 32538 | MGC26610 | NM_144647.2 | 281 | tggatgacgataataatcgaacc | 148233 | - | see also 9724 line |
| 32539 | MGC26610 | NM_144647.2 | 379 | ttccggagtttgataaagatgg | 148235 | - | see also 9724 line |
| 32540 | MGC39650 | NM_152465.1 | 941 | gccagacgttgagccgatcattaa | 148252 | - | see also 14269 line |

Figure 46

EP 1 752 536 A1

1169

| 32541 | MGC39650 | NM_152465.1 | 203 | caggacgacgctcacaattgaaa | 148245 | - | see also 14269 line |
|---|---|---|---|---|---|---|---|
| 32542 | MGC39821 | NM_182576.1 | 628 | agcgatgatgaccgtaatgaaaa | 148259 | - | see also 14270 line |
| 32543 | MGC39821 | NM_182576.1 | 629 | gcgatgatgaccgtaatgaaaag | 148260 | - | see also 14270 line |
| 32544 | MGC4170 | NM_024312.3 | 1264 | atcctcgagtgacaatagtaaca | 148304 | - | see also 14271 line |
| 32545 | MGC4170 | NM_024312.3 | 397 | tgccgattgacgttgtttacacc | 148267 | - | see also 14271 line |
| 32546 | MGC4266 | NM_032680.2 | 791 | gagttcactactggatttagtca | 148385 | - | see also 14272 line |
| 32547 | MGC4266 | NM_032680.2 | 789 | aggagttcactactggatttagt | 148384 | - | see also 14272 line |
| 32548 | MGC4342 | NM_024329.3 | 732 | agctgcagtccacctttaagtag | 148398 | - | see also 14273 line |
| 32549 | MGC45840 | NM_173584.1 | 323 | ctctttcgaggatgttctgatac | 148399 | - | - |
| 32550 | MGC50831 | NM_175924.2 | 435 | agcccgtgctgggggtagattac | 148407 | - | see also 10159 line |
| 32551 | MGC50831 | NM_175924.2 | 484 | ccgagcagatctcgtgataaatg | 148409 | - | see also 10159 line |
| 32552 | MGP | NM_000900.1 | 77 | gccgccttagcggtagtaacttt | 148417 | - | see also 14274 line |
| 32553 | MGP | NM_000900.1 | 85 | agcggtagtaactttgtgttatg | 148418 | - | see also 14274 line |
| 32554 | MGP | NM_000900.1 | 234 | agcctgtccacgagctcaatagg | 148423 | - | see also 14274 line |
| 32555 | MLC1SA | NM_002475.2 | 490 | aggggtttcgtgtgtttgacaag | 148430 | - | see also 14275 line |
| 32556 | MMP1 | NM_002421.2 | 902 | gtgtgacagtaagctaacctttg | 148447 | - | see also 1563 line |
| 32557 | MMP1 | NM_002421.2 | 997 | acccggaagttgagctcaatttc | 148452 | - | see also 1563 line |
| 32558 | MMP11 | NM_005940.2 | 772 | caggggcgttcaacacctatatg | 148463 | - | see also 14277 line |
| 32559 | MMP11 | NM_005940.2 | 263 | ggcgtgcccgacccatctgatgg | 148460 | - | see also 14277 line |
| 32560 | MMP12 | NM_002426.1 | 610 | gacgaattctggactacacattc | 148490 | - | see also 14278 line |
| 32561 | MMP12 | NM_002426.1 | 330 | gcccgtatggaggaaacattata | 148483 | - | see also 14278 line |
| 32562 | MMP12 | NM_002426.1 | 329 | ggcccgtatggaggaaacattat | 148482 | - | see also 14278 line |
| 32563 | MMP13 | NM_002427.2 | 1388 | atctggagtaaccgtattgttcg | 148545 | - | see also 1564 line |
| 32564 | MMP13 | NM_002427.2 | 1407 | ttcgcgtcatgccagcaaattcc | 148546 | - | see also 1564 line |
| 32565 | MMP13 | NM_002427.2 | 1271 | gactatccgagactaatagaaga | 148539 | - | see also 1564 line |
| 32566 | MMP2 | NM_004530.1 | 378 | cgccgtcgcccatcatcaagttc | 148548 | - | see also 3086 line |
| 32567 | MMP2 | NM_004530.1 | 379 | gccgtcgcccatcatcaagttcc | 148549 | - | see also 3086 line |
| 32568 | MMP3 | NM_002422.2 | 1077 | aggcgtggatgccgcatatgaag | 148579 | - | see also 14281 line |
| 32569 | MMP3 | NM_002422.2 | 809 | tccgcctgtctcaagatgatata | 148575 | - | see also 14281 line |
| 32570 | MMP7 | NM_002423.2 | 272 | ctcccgcgtcatagaaataatgc | 148597 | - | see also 14282 line |
| 32571 | MMP7 | NM_002423.2 | 378 | tacaggatcgtatcatatactcg | 148601 | - | see also 14282 line |
| 32572 | MMP7 | NM_002423.2 | 397 | ctcgagacttaccgcatattaca | 148602 | - | see also 14282 line |

Figure 46

| | | | | | | | | | | |
|---|---|---|---|---|---|---|---|---|---|---|
| 32573 | MMP8 | NM_002424.1 | 686 | ctccgcaattacaacttgttc | 148622 | - | see also 14283 line | | | |
| 32574 | MMP8 | NM_002424.1 | 241 | atgtgatcgttgaaaagcttaaa | 148612 | - | see also 14283 line | | | |
| 32575 | MRC1 | NM_002438.1 | 1370 | tggatcggcttaaatgacattaa | 148669 | - | see also 1570 line | | | |
| 32576 | MRC1 | NM_002438.1 | 1204 | gccgtatgccgtcactgttaca | 148662 | - | see also 1570 line | | | |
| 32577 | MUCDHL | NM_017717.3 | 1509 | ctggccatcacatatcgaatta | 148762 | - | see also 14285 line | | | |
| 32578 | MUCDHL | NM_017717.3 | 1511 | cggccatcacatatcgaattacc | 148764 | - | see also 14285 line | | | |
| 32579 | MYL2 | NM_000432.1 | 248 | ctccggtccaattaacttact | 148780 | - | see also 14286 line | | | |
| 32580 | MYL2 | NM_000432.1 | 367 | gtgctgaaggctgattacgttcg | 148785 | - | see also 14286 line | | | |
| 32581 | MYL3 | NM_000258.1 | 242 | acccaagrtgagatgaagatca | 148787 | - | - | | | cardiomyopathy |
| 32582 | MYL4 | NM_002476.2 | 270 | gacccccgactgagagatgaaga | 148789 | - | - | translation regulator | structural constituent of muscle | |
| 32583 | MYL4 | NM_002476.2 | 217 | ttcactgccgaccagattgaaga | 148788 | - | see also 32582 line | | | |
| 32584 | MYL6 | NM_021019.2 | 432 | ggcatgagacagcaatggttgt | 148790 | - | - | | | |
| 32585 | MYL7 | NM_021223.1 | 71 | gttgttcttccaacgtctttc | 148791 | - | - | | | |
| 32586 | MYLC2PL | NM_138403.3 | 524 | aggccgatgtcatcaaagaaaaa | 148793 | - | see also 14287 line | | | |
| 32587 | MYLC2PL | NM_138403.3 | 525 | ggccgatgtcatcaaagaaaaac | 148794 | - | see also 14287 line | | | |
| 32588 | NELL1 | NM_006157.1 | 1087 | aaggtctgccgaccaaaatgtat | 148821 | - | see also 14288 line | | | |
| 32589 | NELL1 | NM_006157.1 | 1092 | ctgccgaccaaaatgtatctatg | 148823 | - | see also 14288 line | | | |
| 32590 | NELL2 | NM_006159.1 | 98 | tgagtctcgggtcttactgaga | 148863 | - | see also 4261 line | | | |
| 32591 | NID | NM_002508.1 | 1517 | ttccgagacgttggatattct | 148941 | - | see also 14290 line | | | |
| 32592 | NID | NM_002508.1 | 1460 | acctccactcttacgtagtaatg | 148939 | - | see also 14290 line | | | |
| 32593 | NIN | NM_016350.3 | 5604 | aacatcagagtgcgagcttaaag | 149088 | - | see also 6829 line | | | |
| 32594 | NIN | NM_016350.3 | 1052 | gacggtacaatgagtgragaaga | 149004 | - | see also 6829 line | | | |
| 32595 | NIN | NM_016350.3 | 5531 | gtcgataagttggcaaaatcaag | 149086 | - | see also 6829 line | | | |
| 32596 | NKD1 | NM_033119.3 | 807 | aagaggagcgtccttgtcaatca | 149107 | - | see also 9369 line | | | |
| 32597 | NKD1 | NM_033119.3 | 989 | gagaaaccactacttagatctg | 149110 | - | see also 9369 line | | | |
| 32598 | NOTCH1 | NM_017617.2 | 6898 | ggccggtccaccagtttgaatgg | 149139 | - | see also 14293 line | | | |
| 32599 | NOTCH1 | NM_017617.2 | 6148 | aagaacgggcgtaacaaagatat | 149137 | - | see also 14293 line | | | |
| 32600 | NOTCH2 | NM_024408.2 | 6047 | cgcaaccgagtaactgatctaga | 149219 | - | see also 8617 line | | | |
| 32601 | NOTCH2 | NM_024408.2 | 3148 | ctctgactacgtcaacagttaca | 149182 | - | see also 8617 line | | | |
| 32602 | NOTCH3 | NM_000435.1 | 3366 | ggggacctgccgtggctatatgg | 149255 | - | see also 344 line | | | |
| 32603 | NOTCH3 | NM_000435.1 | 4805 | aggtgatcggctcggtagtaatg | 149261 | - | see also 344 line | | | |
| 32604 | NOTCH4 | NM_004557.2 | 5917 | tgcgataatgcgaggaagatacg | 149286 | - | see also 3101 line | | | |
| 32605 | NOTCH4 | NM_004557.2 | 5773 | tcggacttggtccgtagacttgg | 149284 | - | see also 3101 line | | | |
| 32606 | NOX5 | NM_024505.2 | 447 | ccctatttgactccgatgaagt | 149291 | - | see also 14297 line | | | |
| 32607 | NOX5 | NM_024505.2 | 1827 | cactgggcgttggttctaagagg | 149298 | - | see also 14297 line | | | |
| 32608 | NOX5 | NM_024505.2 | 2136 | agctccataaggtggactttatc | 149302 | - | see also 14297 line | | | |

Figure 46

| | | | | | | | |
|---|---|---|---|---|---|---|---|
| 32609 | OAZ2 | NM_002537.1 | 712 | cccctctcggccagatgtgatgt | 149312 | - | see also 1632 line |
| 32610 | OAZ2 | NM_002537.1 | 717 | ctggccagatgtgatgttcatg | 149314 | - | see also 1632 line |
| 32611 | PADI1 | NM_013358.1 | 849 | ttccccgatgccgatttcctagg | 149326 | - | see also 5408 line |
| 32612 | PADI1 | NM_013358.1 | 243 | tacaaccgcacacgtgtgaaaga | 149316 | - | see also 5408 line |
| 32613 | PADI1 | NM_013358.1 | 414 | tacctcactggcctcgatatttc | 149321 | - | see also 5408 line |
| 32614 | PADI2 | NM_007365.1 | 649 | cgccggatacgagatagttctgt | 149337 | - | see also 14300 line |
| 32615 | PADI2 | NM_007365.1 | 1071 | aggtctgcttccagtaacctaaac | 149340 | - | see also 14300 line |
| 32616 | PADI3 | NM_016233.1 | 173 | gacgcctggcgtggacatctaca | 149345 | - | see also 14301 line |
| 32617 | PADI3 | NM_016233.1 | 980 | gaggaacaacacgtgttttgtgg | 149349 | - | see also 14301 line |
| 32618 | PADI4 | NM_012387.1 | 945 | gaggtgtacgcgtgcagtatttt | 149363 | - | see also 14302 line |
| 32619 | PADI4 | NM_012387.1 | 946 | aggtgtacgcgtgcagtatttt | 149364 | - | see also 14302 line |
| 32620 | PADI4 | NM_012387.1 | 951 | tacgcgtgcagtattttgaaaa | 149366 | - | see also 14302 line |
| 32621 | PATE | NM_138294.1 | 260 | tggtaatccctggttaaccttca | 149382 | - | see also 14303 line |
| 32622 | PATE | NM_138294.1 | 259 | atggtaatccctggttaaccttc | 149381 | - | see also 14303 line |
| 32623 | PC-LKC | NM_017675.2 | 1771 | tggttagccgctcctacaacatc | 149398 | - | see also 14304 line |
| 32624 | PC-LKC | NM_017675.2 | 2300 | cccggaacgtgagcctggattacg | 149404 | - | see also 14304 line |
| 32625 | PCDH10 | NM_020815.1 | 1762 | aagccggcgagttggactatgaag | 149433 | - | see also 7982 line |
| 32626 | PCDH12 | NM_016580.2 | 3678 | tacaggaacgctgcgtaatcaagg | 149508 | - | see also 6923 line |
| 32627 | PCDH12 | NM_016580.2 | 1984 | caactggtacgcttctcataaaa | 149482 | - | see also 6923 line |
| 32628 | PCDH15 | NM_033056.2 | 3362 | aacacagtcaatcttaatgaag | 149615 | - | see also 14307 line |
| 32629 | PCDH15 | NM_033056.2 | 2391 | accaccgggattctaaccttagg | 149584 | - | see also 14307 line |
| 32630 | PCDH16 | NM_003737.1 | 2333 | tgggcgactctcctaccatatcc | 149705 | - | see also 14308 line |
| 32631 | PCDH16 | NM_003737.1 | 8485 | gtgcgcgagtgcccttgactat | 149747 | - | see also 14308 line |
| 32632 | PCDH17 | NM_014459.2 | 3283 | ctcggatgtccataattcagaca | 149783 | - | see also 14309 line |
| 32633 | PCDH17 | NM_014459.2 | 1403 | acgttccgccaacgtacagatca | 149765 | - | see also 14309 line |
| 32634 | PCDH18 | NM_019035.2 | 2239 | tgcgccatagtagccaggtaatga | 149827 | - | see also 7669 line |
| 32635 | PCDH18 | NM_019035.2 | 3049 | gggcagattctccaatagatag | 149854 | - | see also 7669 line |
| 32636 | PCDH20 | NM_022843.2 | 2273 | ctccccggttacagaagtctatg | 149904 | - | see also 8494 line |
| 32637 | PCDH20 | NM_022843.2 | 2274 | tccccggttacagaagtctatgc | 149905 | - | see also 8494 line |
| 32638 | PCDH7 | NM_002589.2 | 4152 | ctgtcaaaccaattaacaagtaca | 149963 | - | see also 1669 line |
| 32639 | PCDH7 | NM_002589.2 | 2021 | gggtcaacgggcagatcgaatac | 149920 | - | see also 1669 line |
| 32640 | PCDH8 | NM_002590.2 | 3050 | ggcgtgcaccgctgagtgtaaga | 149980 | - | see also 1672 line |
| 32641 | PCDH8 | NM_002590.2 | 1894 | tgcgcagcttcgactatgagacg | 149977 | - | see also 1672 line |
| 32642 | PCDH9 | NM_020403.2 | 1562 | aaccgacgtgggttatacttaac | 150023 | - | see also 14313 line |
| 32643 | PCDH9 | NM_020403.2 | 3154 | gtcgcagcgccgttacgttc | 150082 | - | see also 14313 line |
| 32644 | PCDHA5 | NM_018908.2 | 463 | ggcgcgtcggatttggatattgg | 150092 | - | see also 14314 line |
| 32645 | PCDHA5 | NM_018908.2 | 655 | ccgaactaacagagttacagttca | 150101 | - | see also 14314 line |

Figure 46

| 32646 | PCDHB1 | NM_013340.2 | 79 | tggcgggtacgcgcagaaaatct | 150121 | - | see also 14315 line |
|---|---|---|---|---|---|---|---|
| 32647 | PCDHB1 | NM_013340.2 | 81 | gcgggtacgcgcagaaaatcttt | 150122 | - | see also 14315 line |
| 32648 | PCDHB10 | NM_018930.2 | 678 | tactgtacgcatcgttgtcttgg | 150197 | - | see also 14316 line |
| 32649 | PCDHB10 | NM_018930.2 | 1084 | gagacgccgctggctgtttttaa | 150205 | - | see also 14316 line |
| 32650 | PCDHB10 | NM_018930.2 | 863 | ttcgaacaacctttcaaatcaat | 150203 | - | see also 14316 line |
| 32651 | PCDHB11 | NM_018931.2 | 2304 | accggttatccctaatatccagg | 150218 | - | see also 14317 line |
| 32652 | PCDHB11 | NM_018931.2 | 1097 | tggttttttagtatccaagatata | 150215 | - | see also 14317 line |
| 32653 | PCDHB11 | NM_018931.2 | 771 | tggctcgctgattttgattgtct | 150212 | - | see also 14317 line |
| 32654 | PCDHB12 | NM_018932.2 | 816 | aacaaacagtgaactatcctata | 150222 | - | see also 14318 line |
| 32655 | PCDHB12 | NM_018932.2 | 2274 | ctccaggtcaaataagttcaaat | 150225 | - | see also 14318 line |
| 32656 | PCDHB12 | NM_018932.2 | 2336 | caggtagtgaagtcgaagaaaat | 150227 | - | see also 14318 line |
| 32657 | PCDHB13 | NM_018933.2 | 1365 | tcctgaaatccgcggaaaacttt | 150230 | - | see also 14319 line |
| 32658 | PCDHB13 | NM_018933.2 | 2545 | ttctaccttccccaataacttttg | 150234 | - | see also 14319 line |
| 32659 | PCDHB14 | NM_018934.2 | 1041 | gaccatatcgtcgattacaaaga | 150266 | - | see also 14320 line |
| 32660 | PCDHB14 | NM_018934.2 | 14 | gggcactcgatctgcgaaaaagg | 150235 | - | see also 14320 line |
| 32661 | PCDHB15 | NM_018935.2 | 235 | ctgcagaccgggcagttgatatt | 150278 | - | see also 14321 line |
| 32662 | PCDHB15 | NM_018935.2 | 934 | atgtcttcgtatgatctagatat | 150296 | - | see also 14321 line |
| 32663 | PCDHB16 | NM_020957.1 | 2003 | agccttcggaggatattagtaaa | 150328 | - | see also 14322 line |
| 32664 | PCDHB16 | NM_020957.1 | 2004 | gccttcggaggatattagtaaaa | 150329 | - | see also 14322 line |
| 32665 | PCDHB2 | NM_018936.2 | 1181 | aactaactatgtcgacacttatc | 150357 | - | see also 14323 line |
| 32666 | PCDHB2 | NM_018936.2 | 694 | gacagtctcgatggcataatatt | 150348 | - | see also 14323 line |
| 32667 | PCDHB2 | NM_018936.2 | 1168 | gacaatcctccggaactaactat | 150355 | - | see also 14323 line |
| 32668 | PCDHB3 | NM_018937.2 | 355 | aacgagctccgtatcatagatgt | 150364 | - | see also 14324 line |
| 32669 | PCDHB3 | NM_018937.2 | 82 | tccgagtcaagacgctattctgt | 150361 | - | see also 14324 line |
| 32670 | PCDHB3 | NM_018937.2 | 351 | tacaaacgagctccgtatcatag | 150363 | - | see also 14324 line |
| 32671 | PCDHB4 | NM_018938.2 | 77 | gcctcgagcctattcgttattct | 150393 | - | see also 14325 line |
| 32672 | PCDHB4 | NM_018938.2 | 79 | ctcgagcctattcgttattctgt | 150394 | - | see also 14325 line |
| 32673 | PCDHB5 | NM_015669.2 | 1007 | gtctccgctcgagatttagatgc | 150442 | - | see also 14326 line |
| 32674 | PCDHB5 | NM_015669.2 | 457 | gaccggcaatttgcttctatatg | 150427 | - | see also 14326 line |
| 32675 | PCDHB6 | NM_018939.2 | 184 | ggcgctcgggttgttttcaaagg | 150458 | - | see also 14327 line |
| 32676 | PCDHB6 | NM_018939.2 | 355 | tccttgcgagtcagagatataaa | 150460 | - | see also 14327 line |
| 32677 | PCDHB7 | NM_018940.2 | 255 | cgccgaaccgcttcggtattttg | 150477 | - | see also 14328 line |
| 32678 | PCDHB7 | NM_018940.2 | 1017 | ttcttacgccactgaaagaattc | 150498 | - | see also 14328 line |
| 32679 | PCDHB7 | NM_018940.2 | 256 | gccgaaccgcttcggtattttgt | 150478 | - | see also 14328 line |
| 32680 | PCDHB8 | NM_019120.2 | 1059 | aacttttaaggtcgatttcttga | 150511 | - | see also 14329 line |
| 32681 | PCDHB8 | NM_019120.2 | 2493 | accagtattacctaatattcagg | 150513 | - | see also 14329 line |
| 32682 | PCDHB9 | NM_019119.3 | 765 | tccagtagggtcccttattgtta | 150519 | - | see also 14330 line |

Figure 46

| | | | | | | | |
|---|---|---|---|---|---|---|---|
| 32683 | PCDHB9 | NM_019119.3 | 2358 | ttctactctcccaatagcttg | 150522 | - | see also 14330 line |
| 32684 | PDCD6 | NM_013232.2 | 486 | tccacgacatcctcattcgaaag | 150591 | - | see also 14331 line |
| 32685 | PDCD6 | NM_013232.2 | 570 | tgcagaggttgacggatatattc | 150593 | - | see also 14331 line |
| 32686 | PEF | NM_012392.1 | 600 | ccgtctgggtccattagctaca | 150608 | - | see also 5308 line |
| 32687 | PH-4 | NM_017732.2 | 1285 | gccgctgcaggttgttcgatatg | 150611 | - | see also 14333 line |
| 32688 | PH-4 | NM_017732.2 | 791 | cccggcttcctgactgatgaaga | 150609 | - | see also 14333 line |
| 32689 | PITPNM2 | NM_020845.1 | 160 | aagagccgtaacgagaacatatgg | 150623 | - | see also 7992 line |
| 32690 | PITPNM2 | NM_020845.1 | 633 | cccatcatgtgcgcatacaagc | 150630 | - | see also 7992 line |
| 32691 | PITPNM3 | NM_031220.1 | 1917 | gacaggtaatggctatgagagc | 150644 | - | see also 14335 line |
| 32692 | PITPNM3 | NM_031220.1 | 196 | ccctggcaccttcgaaatgtcc | 150642 | - | see also 14335 line |
| 32693 | PKD1-like | NM_024874.3 | 889 | gaggtccacaaggcggattacaat | 150665 | - | see also 8799 line |
| 32694 | PKD1-like | NM_024874.3 | 890 | aggtccacaaggcgattacaatt | 150666 | - | see also 8799 line |
| 32695 | PKD2L1 | NM_016112.2 | 2416 | aactaggccgatctatgtgagc | 150748 | - | see also 6669 line |
| 32696 | PKD2L1 | NM_016112.2 | 988 | agctaaagtccgcaatgactcc | 150728 | - | see also 6669 line |
| 32697 | PKD2L2 | NM_014386.1 | 369 | agcagtcgcatctactatgaaaa | 150766 | - | see also 14338 line |
| 32698 | PKD2L2 | NM_014386.1 | 839 | cagatatgttagctactatgact | 150786 | - | see also 14338 line |
| 32699 | PKDREJ | NM_006071.1 | 1153 | aactacgtgsggatcgaataat | 150825 | - | see also 14339 line |
| 32700 | PKDREJ | NM_006071.1 | 4804 | tacggctatgacaagtcaataga | 150940 | - | see also 14339 line |
| 32701 | PKDREJ | NM_006071.1 | 3911 | gtcgatcgcctagcctggtattta | 150911 | - | see also 14339 line |
| 32702 | PLA2G10 | NM_003561.1 | 792 | tgcggaccggcagaggaacaaatg | 150997 | - | see also 14340 line |
| 32703 | PLA2G10 | NM_003561.1 | 833 | gtgtgaccaggagattgctaact | 150999 | - | see also 14340 line |
| 32704 | PLA2G1B | NM_000928.2 | 302 | acccgtacaccacccattca | 151006 | - | see also 14341 line |
| 32705 | PLA2G1B | NM_000928.2 | 231 | gtgctgccagacacatgacaact | 151004 | - | see also 14341 line |
| 32706 | PLA2G2A | NM_000300.2 | 499 | aacgtggatgtggcaccaaattt | 151012 | - | see also 14342 line |
| 32707 | PLA2G2A | NM_000300.2 | 614 | tgccacctgtttgctagaaaca | 151016 | - | see also 14342 line |
| 32708 | PLA2G2E | NM_014589.1 | 428 | aaccgcaaatatgccattatcc | 151024 | - | see also 14343 line |
| 32709 | PLA2G2E | NM_014589.1 | 419 | ggcacctacaacgcaaatatgc | 151023 | - | see also 14343 line |
| 32710 | PLA2G2F | NM_022819.1 | 241 | ccctatgtggaccactatgatca | 151025 | - | see also 14344 line |
| 32711 | PLA2G2F | NM_022819.1 | 389 | accgtggcttcctcaatgtctac | 151028 | - | see also 14344 line |
| 32712 | PLA2G3 | NM_015715.1 | 807 | accgctgcccacagaacatctca | 151031 | - | see also 14345 line |
| 32713 | PLA2G3 | NM_015715.1 | 1696 | aggcccatgtcattcctacaacc | 151033 | - | see also 14345 line |
| 32714 | PLA2G5 | NM_000929.1 | 374 | ttcgcacacagtcctacaaatac | 151037 | - | see also 14346 line |
| 32715 | PLA2G5 | NM_000929.1 | 193 | ggcttgctggacctaaaatcaat | 151036 | - | see also 14346 line |
| 32716 | PLCD1 | NM_006225.1 | 1151 | atcatctaccacggctatacttt | 151047 | - | see also 14347 line |
| 32717 | PLCD1 | NM_006225.1 | 1158 | accacggctatactttcacttcc | 151049 | - | see also 14347 line |
| 32718 | PLCD3 | NM_133373.1 | 410 | tgcgccatcgcagcacatcttct | 151053 | - | see also 14348 line |
| 32719 | PLCD3 | NM_133373.1 | 2001 | cagtgtggctacgtcctaaaacc | 151059 | - | see also 14348 line |

Figure 46

| 32720 | PLCD4 | NM_032726.2 | 1828 | tggcagttaagccgtgtgtatcc | 151092 | - | see also 14349 line |
|---|---|---|---|---|---|---|---|
| 32721 | PLCD4 | NM_032726.2 | 1147 | agcagcgtcgagggatatatacg | 151079 | - | see also 14349 line |
| 32722 | PLCG1 | NM_002660.2 | 617 | cggaatcgtgaggatcgtatatc | 151113 | - | see also 14350 line |
| 32723 | PLCG1 | NM_002660.2 | 1897 | gggcgactacacgctctctttct | 151123 | - | see also 14350 line |
| 32724 | PLCG2 | NM_002661.1 | 3450 | aagacgacggttgtgaatgataa | 151202 | - | see also 1741 line |
| 32725 | PLCG2 | NM_002661.1 | 3452 | gacgacggttgtgaatgataatg | 151203 | - | see also 1741 line |
| 32726 | PLCZ1 | NM_033123.2 | 540 | tacgagcctatcgaagaagttag | 151221 | - | see also 9370 line |
| 32727 | PLCZ1 | NM_033123.2 | 1831 | cagaattggcattgatacgtttt | 151276 | - | see also 9370 line |
| 32728 | PLEK | NM_002664.1 | 528 | cacaggtaactgcgtcattgatt | 151286 | - | see also 1744 line |
| 32729 | PLEK | NM_002664.1 | 559 | tccaaccagtctgttaggaatcg | 151290 | - | see also 1744 line |
| 32730 | PLG | NM_000301.1 | 2138 | accggaccgaatgtttcatcact | 151314 | - | see also 14354 line |
| 32731 | PLG | NM_000301.1 | 2132 | tcgctgaccggaccgaatgtttc | 151311 | - | see also 14354 line |
| 32732 | PLSCR1 | NM_021105.1 | 564 | gtcagatagatcagatactgatt | 151319 | - | see also 14355 line |
| 32733 | PLSCR1 | NM_021105.1 | 1063 | ggcatttacagacgctgataact | 151322 | - | see also 14355 line |
| 32734 | PLSCR2 | NM_020359.1 | 358 | aactgtccgccaggattggaata | 151323 | - | see also 14356 line |
| 32735 | PLSCR2 | NM_020359.1 | 839 | ttgtggttggcaggatttctaag | 151336 | - | see also 14356 line |
| 32736 | PLSCR3 | NM_020360.2 | 510 | gagacctgtaatcggtatgaact | 151340 | - | see also 14357 line |
| 32737 | PLSCR3 | NM_020360.2 | 444 | ctggtgcagattgatcagatttt | 151338 | - | see also 14357 line |
| 32738 | PLSCR4 | NM_020353.1 | 834 | gggcggtgtacagcatccaaaat | 151356 | - | see also 7843 line |
| 32739 | PLSCR4 | NM_020353.1 | 690 | tgggccgagaaatcatgacaatg | 151354 | - | see also 7843 line |
| 32740 | PLSCR4 | NM_020353.1 | 952 | atccaacatcggcagtattatcc | 151357 | - | see also 7843 line |
| 32741 | PPEF1 | NM_006240.2 | 1292 | ctcccaatcggtacaatcgttga | 151386 | - | see also 14359 line |
| 32742 | PPEF1 | NM_006240.2 | 1298 | atcggtacaatcgttgacaatga | 151387 | - | see also 14359 line |
| 32743 | PPEF2 | NM_006239.2 | 785 | ctcgctacgtcttgaacctttg | 151432 | - | see also 4325 line |
| 32744 | PPEF2 | NM_006239.2 | 1856 | accgcaaggtattaacaatcttt | 151463 | - | see also 4325 line |
| 32745 | PPEF2 | NM_006239.2 | 1720 | tgcaaggccaacactattcgagg | 151457 | - | see also 4325 line |
| 32746 | PPHLN1 | NM_016488.3 | 162 | tggctacaatagactagttaata | 151496 | - | see also 14361 line |
| 32747 | PPHLN1 | NM_016488.3 | 156 | cagtgatggctacaatagactag | 151494 | - | see also 14361 line |
| 32748 | PPM2C | NM_018444.1 | 1822 | atgttgtaggggcgtatcaaaac | 151572 | - | see also 14362 line |
| 32749 | PPM2C | NM_018444.1 | 911 | tggtgagtcgactgatattgatg | 151543 | - | see also 14362 line |
| 32750 | PPP2R3A | NM_002718.3 | 3326 | gccgatctgtctcgatacaatga | 151673 | - | see also 14363 line |
| 32751 | PPP2R3A | NM_002718.3 | 3530 | gacggtgtactctccatgtatga | 151678 | - | see also 14363 line |
| 32752 | PPP2R3A | NM_002718.3 | 2687 | aacattccacggttctactttcc | 151650 | - | see also 14363 line |
| 32753 | PR48 | NM_013239.2 | 498 | tccgcagggacgagagtagttca | 151686 | - | see also 14364 line |
| 32754 | PR48 | NM_013239.2 | 1105 | accagctgaccgaattcttctcg | 151690 | - | see also 14364 line |
| 32755 | PR48 | NM_013239.2 | 787 | tcgtcgccatgtggagaaaaatc | 151689 | - | see also 14364 line |
| 32756 | PRKCSH | NM_002743.1 | 282 | atcaggtcaacgatgactattgc | 151693 | - | see also 14365 line |

Figure 46

| 32757 | PRKCSH | NM_002743.1 | 407 | cgggtcaacgatggtgtttgtga | 151697 | - | see also 14365 line |
|---|---|---|---|---|---|---|---|
| 32758 | PROC | NM_000312.1 | 1192 | tcccgtggtcccgcacaatgagt | 151706 | - | see also 14366 line |
| 32759 | PROC | NM_000312.1 | 498 | aggtgagcttcctcaattgctcg | 151703 | - | see also 14366 line |
| 32760 | PROM2 | NM_144707.1 | 1990 | ccccgacttcctcgttcagatcc | 151716 | - | see also 14367 line |
| 32761 | PROM2 | NM_144707.1 | 380 | ttggtaaaggccctactgaatga | 151709 | - | see also 14367 line |
| 32762 | PROS1 | NM_000313.1 | 1847 | atgttccgatcaacaatctcatc | 151775 | - | see also 14368 line |
| 32763 | PROS1 | NM_000313.1 | 1467 | ccgattaaccctcgtctagatgg | 151759 | - | see also 14368 line |
| 32764 | PRRG1 | NM_000950.1 | 561 | ccccccaccagatgaagtgtttg | 151790 | - | see also 14369 line |
| 32765 | PRRG1 | NM_000950.1 | 386 | gaggaagtgactggtttcagttt | 151784 | - | see also 14369 line |
| 32766 | PVALB | NM_002854.1 | 243 | gagctgggattcatcctaaaagg | 151793 | - | see also 14370 line |
| 32767 | Rab11-FIP3 | NM_014700.1 | 1138 | ctcggcgtgatcagctttgaaga | 151798 | - | see also 14371 line |
| 32768 | Rab11-FIP3 | NM_014700.1 | 1257 | cccggacgagttcgatgacttcg | 151800 | - | see also 14371 line |
| 32769 | RAB11-FIP4 | NM_032932.2 | 2011 | cgcctcgcgcgatgagctaatgg | 151812 | - | see also 14372 line |
| 32770 | RAB11-FIP4 | NM_032932.2 | 2014 | ctcgcgcgatgagctaatggaag | 151813 | - | see also 14372 line |
| 32771 | RAB11-FIP4 | NM_032932.2 | 1025 | aacagcgaattgctagatgttta | 151806 | - | see also 14372 line |
| 32772 | RASGRP1 | NM_005739.2 | 340 | gtgtcgaagtaaccaactgttgc | 151818 | - | see also 14373 line |
| 32773 | RASGRP1 | NM_005739.2 | 827 | cccaccatggagcgatctattgc | 151830 | - | see also 14373 line |
| 32774 | RASGRP2 | NM_005825.2 | 1197 | gtgacggcgacaggcaactatgg | 151871 | - | see also 4061 line |
| 32775 | RASGRP3 | NM_015376.1 | 1660 | atcaggatggcctaattagtaaa | 151916 | - | see also 14375 line |
| 32776 | RASGRP3 | NM_015376.1 | 393 | atgcaccgatggtatttatcttc | 151885 | - | see also 14375 line |
| 32777 | RCN1 | NM_002901.1 | 504 | accccgcagagtttcatgattct | 151931 | - | see also 14376 line |
| 32778 | RCN1 | NM_002901.1 | 503 | aaccccgcagagtttcatgattc | 151930 | - | see also 14376 line |
| 32779 | RCN3 | NM_020650.2 | 629 | gacgggaagtggccaaggaattc | 151933 | - | see also 14377 line |
| 32780 | RCN3 | NM_020650.2 | 1343 | aagcggaaatcctgggtaattgg | 151939 | - | see also 14377 line |
| 32781 | RCV1 | NM_002903.1 | 555 | tggagatcgtcatggctattttc | 151941 | - | see also 14378 line |
| 32782 | RCV1 | NM_002903.1 | 656 | aagtactttggaaagaatgatga | 151942 | - | see also 14378 line |
| 32783 | REPS1 | NM_031922.2 | 2821 | caggcatcaattagacgtaataa | 151999 | - | see also 9053 line |
| 32784 | REPS2 | NM_004726.1 | 1013 | aacagcgcgagtactatgtcaat | 152008 | - | see also 14380 line |
| 32785 | REPS2 | NM_004726.1 | 1853 | caccatcaaagcccattcgtagg | 152021 | - | see also 14380 line |
| 32786 | RGN | NM_004683.3 | 623 | ttggtcgctagaccacaaaatct | 152035 | - | see also 3211 line |
| 32787 | RGN | NM_004683.3 | 713 | ctccaaccgcagaagtgtttaca | 152036 | - | see also 3211 line |
| 32788 | RHBDL1 | NM_003961.1 | 704 | accaccccgagtacatgaagagc | 152046 | - | see also 14382 line |
| 32789 | RHBDL1 | NM_003961.1 | 699 | gacctaccaccccgagtacatga | 152045 | - | see also 14382 line |

Figure 46

| | | | | | | | | | |
|---|---|---|---|---|---|---|---|---|---|
| 32790 | RHBDL4 | NM_138328.1 | 755 | cacccgaattgggcttgtctacg | 152060 | - | see also 9598 line | | |
| 32791 | RHBDL4 | NM_138328.1 | 647 | ttggcgctacctgacatacatct | 152057 | - | see also 9598 line | | |
| 32792 | RYR3 | NM_001036.2 | 11263 | cacgcgtgatctctttagattcc | 152288 | - | see also 14384 line | | |
| 32793 | RYR3 | NM_001036.2 | 7297 | tgcgcttgcactaaataggtata | 152192 | - | see also 14384 line | | |
| 32794 | S100A10 | NM_002966.1 | 343 | ggcctcaccattgcatgcaatga | 152360 | - | see also 1998 line | | |
| 32795 | S100A10 | NM_002966.1 | 260 | tggctgtggacaaaataatgaag | 152359 | - | see also 1998 line | | |
| 32796 | S100A11 | NM_005620.1 | 192 | tgctggaaaggatggttataact | 152362 | - | see also 14385 line | | |
| 32797 | S100A12 | NM_005621.1 | 240 | ttccaaggcctggatgctaatca | 152369 | - | see also 14386 line | | |
| 32798 | S100A12 | NM_005621.1 | 127 | ttcggaaggggcattttgacacc | 152367 | - | see also 14386 line | | |
| 32799 | S100A12 | NM_005621.1 | 88 | atctggagggaattgtcaatatc | 152365 | - | see also 14386 line | | |
| 32800 | S100A4 | NM_002961.2 | 152 | agctcaacaagtcagaactaaag | 152374 | - | - | - | colorectal cancer、 breast cancer、 colorectal cancers、 osteosarcoma |
| 32801 | S100A5 | NM_002962.1 | 429 | gagcagcatcgatgacttgatga | 152376 | - | see also 14389 line | | |
| 32802 | S100A5 | NM_002962.1 | 317 | tggtgaccacgtttcacaaatat | 152375 | - | see also 14389 line | | |
| 32803 | S100A8 | NM_002964.3 | 172 | gaccgagtgtcctcagtatatca | 152379 | - | see also 14390 line | | |
| 32804 | S100A8 | NM_002964.3 | 238 | cactgatggtgcagttaacttcc | 152383 | - | see also 14390 line | | |
| 32805 | S100Z | NM_130772.1 | 464 | ttcctctcgtgccaaaaggaaac | 152387 | - | see also 14392 line | | |
| 32806 | S100Z | NM_130772.1 | 485 | acccagttggttgataagatagt | 152390 | - | see also 14392 line | | |
| 32807 | SCG2 | NM_003469.2 | 1478 | ctgccaaggctcccttatggtgc | 152412 | - | see also 14393 line | | |
| 32808 | SCG2 | NM_003469.2 | 1441 | gtcgtattttcccaatccatata | 152409 | - | see also 14393 line | | |
| 32809 | SCGN | NM_006998.2 | 882 | cagcggggtggaccttgataagt | 152441 | - | see also 14394 line | | |
| 32810 | SCGN | NM_006998.2 | 669 | tggtcggttggatctaaatgact | 152435 | - | see also 14394 line | | |
| 32811 | SCN10A | NM_006514.1 | 1744 | gtcgatgtctcggcattcgatgc | 152482 | - | see also 4556 line | | |
| 32812 | SCN10A | NM_006514.1 | 239 | aggatctagatccgttctacagc | 152448 | - | see also 4556 line | | |
| 32813 | SCN1A | NM_006920.2 | 5919 | ttcctatgaccgggtgacaaagc | 152618 | - | see also 14396 line | | |
| 32814 | SCN1A | NM_006920.2 | 3602 | tccgaatagttgaacataactgg | 152582 | - | see also 14396 line | | |
| 32815 | SCN2A2 | NM_021007.1 | 1003 | ttggatgggaatggtactacttt | 152633 | - | see also 14397 line | | |
| 32816 | SCN2A2 | NM_021007.1 | 2151 | gagacggtccagttcttatcatg | 152647 | - | see also 14397 line | | |
| 32817 | SCN3A | NM_006922.2 | 2327 | agcgacgcaacagtaacgttagt | 152709 | - | see also 14398 line | | |
| 32818 | SCN3A | NM_006922.2 | 3532 | aagatgcgggagtgtttccaaaa | 152715 | - | see also 14398 line | | |
| 32819 | SCN4A | NM_000334.3 | 1084 | ttgattgggacgcctacatcagt | 152763 | - | see also 14399 line | | |
| 32820 | SCN4A | NM_000334.3 | 2120 | gtcgtggccaacgctgaacatgc | 152772 | - | see also 14399 line | | |
| 32821 | SCN9A | NM_002977.1 | 1370 | cagcggcagcggctgaatataca | 152809 | - | see also 14400 line | | |
| 32822 | SCN9A | NM_002977.1 | 1376 | cagcggctgaatatacaagtatt | 152812 | - | see also 14400 line | | |
| 32823 | SCUBE1 | NM_173050.1 | 744 | atcgagacgtgcgcagtcaataa | 152891 | - | see also 10047 line | | |
| 32824 | SCUBE1 | NM_173050.1 | 1339 | tcgtgccagactcggaaaatagc | 152897 | - | see also 10047 line | | |
| 32825 | SCUBE2 | NM_020974.1 | 2826 | gtcccatacgtgacatatgatga | 152951 | - | see also 8059 line | | |

Figure 46

| 32826 | SCUBE2 | NM_020974.1 | 1656 | agcttccgctacgtaaaccttac | 152922 | - | see also 8059 line |
| 32827 | SEPN1 | NM_020451.1 | 295 | cactgacgggatatgtacatca | 152954 | - | see also 14403 line |
| 32828 | SEPN1 | NM_020451.1 | 758 | ctgtccaacaacgcttctatcc | 152956 | - | see also 14403 line |
| 32829 | SLC25A12 | NM_003705.2 | 1748 | ctgcagctcgagtgtttcgatcc | 153016 | - | see also 2527 line |
| 32830 | SLC25A12 | NM_003705.2 | 977 | ttgccgagtcgcttacagattc | 152997 | - | see also 2527 line |
| 32831 | SLC25A13 | NM_014251.1 | 1640 | tccgtgctatgctcatgtgaagg | 153061 | - | see also 5556 line |
| 32832 | SLC25A13 | NM_014251.1 | 1808 | cacttacagcggagtgatagact | 153063 | - | see also 5556 line |
| 32833 | SMOC1 | NM_022137.2 | 976 | atccccgtgaggtattgtcatc | 153083 | - | see also 8301 line |
| 32834 | SMOC1 | NM_022137.2 | 1135 | gcgacgccaggcgcaagactaca | 153087 | - | see also 8301 line |
| 32835 | SMOC2 | NM_022138.1 | 959 | aggttgtccgggtgccaaaaagc | 153104 | - | see also 14407 line |
| 32836 | SMOC2 | NM_022138.1 | 243 | cagctagagattgcatatcgagg | 153098 | - | see also 14407 line |
| 32837 | SPARCL1 | NM_004684.2 | 1162 | tgcatcgaacgtcaataagcaca | 153127 | - | see also 14408 line |
| 32838 | SPARCL1 | NM_004684.2 | 1770 | gtcatctattcgctactaaatgc | 153142 | - | see also 14408 line |
| 32839 | SPOCK2 | NM_014767.1 | 539 | gacaatcagcaaggagatgaagc | 153154 | - | - |
| 32840 | SPOCK3 | NM_016950.1 | 213 | ggcggtcggacgcgggtaatttt | 153155 | - | see also 6979 line |
| 32841 | SPOCK3 | NM_016950.1 | 214 | gcggtcggacgcgcggtaatttc | 153156 | - | see also 6979 line |
| 32842 | SPOCK3 | NM_016950.1 | 352 | acccttcgatcaggctttagatc | 153159 | - | see also 6979 line |
| 32843 | SRI | NM_003130.2 | 531 | tcgcctgcgtcaaactgagg | 153206 | - | see also 14410 line |
| 32844 | SRI | NM_003130.2 | 310 | gtctggcacaatgggtttcaatg | 153196 | - | see also 14410 line |
| 32845 | SSR1 | NM_003144.2 | 351 | accgagtcgagatacaactatac | 153213 | - | see also 2125 line |
| 32846 | SSR1 | NM_003144.2 | 603 | ggggcggacgaccatttggtttgg | 153222 | - | see also 2125 line |
| 32847 | SSR4 | NM_006280.1 | 508 | tgcgggatcggccttgtgatct | 153244 | - | see also 14412 line |
| 32848 | SSR4 | NM_006280.1 | 511 | gggatcggccttgtgatctact | 153245 | - | see also 14412 line |
| 32849 | SSR4 | NM_006280.1 | 252 | ctgacgtggtggaaaaacaattc | 153239 | - | see also 14412 line |
| 32850 | STAB1 | NM_015136.1 | 373 | ggcgctgagacccatgcaatgg | 153249 | - | see also 14413 line |
| 32851 | STAB1 | NM_015136.1 | 1383 | cacgtcaccttaaccaattca | 153255 | - | see also 14413 line |
| 32852 | STAB1 | NM_015136.1 | 5298 | atcccataggcccttcacaatgc | 153293 | - | see also 14413 line |
| 32853 | STAB2 | NM_017564.8 | 2566 | tgcgttcacggaacatgcaataa | 153373 | - | see also 14414 line |
| 32854 | STAB2 | NM_017564.8 | 6128 | cggccaccggagagtgtaaatgc | 153464 | - | see also 14414 line |
| 32855 | SWAP70 | NM_015055.1 | 271 | accagggctacatgccttattta | 153484 | - | see also 6214 line |
| 32856 | SWAP70 | NM_015055.1 | 1687 | tggccatcatgaaggattaatt | 153512 | - | see also 6214 line |
| 32857 | SYP | NM_003179.1 | 300 | ctcctcgtcagccgaattcttg | 153520 | - | see also 14416 line |
| 32858 | SYP | NM_003179.1 | 548 | gccgccagacagggaacacatgc | 153524 | - | see also 14416 line |
| 32859 | TGM1 | NM_000359.1 | 1542 | aagtacgacacgccttcatttt | 153538 | - | see also 14417 line |
| 32860 | TGM1 | NM_000359.1 | 1545 | tacgacacgcctttcattttgc | 153539 | - | see also 14417 line |
| 32861 | TGM1 | NM_000359.1 | 466 | accacacagacgagtatgagtac | 153526 | - | see also 14417 line |
| 32862 | TGM2 | NM_004613.2 | 902 | ggctgccagccgtcaagtatgg | 153558 | - | see also 14418 line |

Figure 46

| 32863 | TGM2 | NM_004613.2 | 984 | cccgcgtcgtgaccaactacaac | 153559 | - | see also 14418 line |
|---|---|---|---|---|---|---|---|
| 32864 | TGM3 | NM_003245.2 | 1700 | ctcgtacgctcagtatgagaagt | 153597 | - | see also 2187 line |
| 32865 | TGM3 | NM_003245.2 | 633 | cgccgtgacgctgctactgatgt | 153582 | - | see also 2187 line |
| 32866 | TGM4 | NM_003241.1 | 1464 | cacatgtcggtacaatcagatga | 153627 | - | see also 14420 line |
| 32867 | TGM4 | NM_003241.1 | 1459 | ttcttcacatgtcggtacaatca | 153626 | - | see also 14420 line |
| 32868 | TGM5 | NM_004245.1 | 935 | cacgcctttgtgttttcgatgg | 153655 | - | see also 2849 line |
| 32869 | TGM5 | NM_004245.1 | 1623 | agcatcacgattaatgttctagg | 153666 | - | see also 2849 line |
| 32870 | TGM7 | NM_052955.1 | 920 | cgcgcacaacgtggataggaact | 153690 | - | see also 14422 line |
| 32871 | TGM7 | NM_052955.1 | 942 | ttgaccatcgatacgtactatga | 153691 | - | see also 14422 line |
| 32872 | THBD | NM_000361.2 | 1293 | gccagcccctgaaccaaactagc | 153707 | - | see also 14423 line |
| 32873 | THBD | NM_000361.2 | 487 | gtgggttacgggagacaacaaca | 153704 | - | see also 14423 line |
| 32874 | THBS1 | NM_003246.2 | 3224 | caccgaaagggacgatgactatg | 153751 | - | see also 2188 line |
| 32875 | THBS1 | NM_003246.2 | 3225 | accgaaagggacgatgactatgc | 153752 | - | see also 2188 line |
| 32876 | THBS1 | NM_003246.2 | 2302 | atgcgacttaccactgcaaaaag | 153735 | - | see also 2188 line |
| 32877 | THBS2 | NM_003247.2 | 3514 | cggggcaggtgcgaaccttatgg | 153789 | - | see also 14425 line |
| 32878 | THBS2 | NM_003247.2 | 3278 | cagtggcacattctacgtaaaca | 153786 | - | see also 14425 line |
| 32879 | THBS3 | NM_007112.3 | 2181 | gaggtaacgcttacggattttcg | 153826 | - | see also 14426 line |
| 32880 | THBS3 | NM_007112.3 | 2726 | ggcggattctggggtgatcattg | 153833 | - | see also 14426 line |
| 32881 | THBS4 | NM_003248.3 | 1100 | ggcttgcggtcctctcaagtttc | 153844 | - | see also 14427 line |
| 32882 | THBS4 | NM_003248.3 | 1491 | cccaactcgatctgcgttaatac | 153852 | - | see also 14427 line |
| 32883 | TKT | NM_001064.1 | 51 | cacggccaaccgcctacgtatca | 153879 | - | see also 652 line |
| 32884 | TKT | NM_001064.1 | 1372 | gtggaactagccgccaatacaaa | 153894 | - | see also 652 line |
| 32885 | TKTL1 | NM_012253.1 | 1291 | acccgaccagaaactatggttat | 153912 | - | see also 14429 line |
| 32886 | TKTL1 | NM_012253.1 | 1292 | cccgaccagaaactatggttatt | 153913 | - | see also 14429 line |
| 32887 | TLL1 | NM_012464.3 | 1414 | atcgagataaccacgtaactatc | 153948 | - | see also 5339 line |
| 32888 | TLL2 | NM_012465.2 | 1854 | agccggaggatgtgaaatcgagc | 154040 | - | see also 5343 line |
| 32889 | TLL2 | NM_012465.2 | 2240 | gtctgtaagtacgactttgtaga | 154046 | - | see also 5343 line |
| 32890 | TMG3 | NM_024082.2 | 419 | cccattcggtcctgaaacgattc | 154058 | - | see also 14431 line |
| 32891 | TMG3 | NM_024082.2 | 1038 | cccaaagtacgaggagatagtgg | 154064 | - | see also 14431 line |
| 32892 | TNNC1 | NM_003280.1 | 232 | agcggcacggtggactttgatga | 154065 | - | see also 2219 line |
| 32893 | TNNC2 | NM_003279.2 | 93 | gtcctacctcagcgaagagatga | 154068 | - | see also 14433 line |
| 32894 | TPO | NM_000547.3 | 2075 | tgcgggacggtgactggttttgg | 154086 | - | see also 14434 line |
| 32895 | TPO | NM_000547.3 | 2675 | gtctcacctcgacggtgatttgc | 154092 | - | see also 14434 line |
| 32896 | TRPA1 | NM_007332.1 | 1534 | gccagttatgggcgtatcaatac | 154145 | - | see also 5080 line |
| 32897 | TRPA1 | NM_007332.1 | 1533 | agccagttatgggcgtatcaata | 154144 | - | see also 5080 line |
| 32898 | TSC | NM_017899.1 | 284 | agcagctccatcggagatttaag | 154218 | - | see also 14436 line |
| 32899 | TSC | NM_017899.1 | 590 | gccgcatcactctggaagaatat | 154223 | - | see also 14436 line |

Figure 46

| 32900 | TSC | NM_017899.1 | 375 | ccccatccgatccaaaattgttc | 154220 | - | see also 14436 line |
|---|---|---|---|---|---|---|---|
| 32901 | UCC1 | NM_017549.1 | 204 | ctcgcgcgtccggatctcaaaag | 154227 | - | see also 7030 line |
| 32902 | UCC1 | NM_017549.1 | 940 | gtctacgcggtttttgacatcc | 154238 | - | see also 7030 line |
| 32903 | UCC1 | NM_017549.1 | 205 | tcgcgcgtccggatctcaaaagc | 154228 | - | see also 7030 line |
| 32904 | UMOD | NM_003361.1 | 1585 | ggcgacctgtcccgatttgcact | 154251 | - | see also 14438 line |
| 32905 | UMOD | NM_003361.1 | 1197 | ccggtgctcgggcttcaatgaca | 154247 | - | see also 14438 line |
| 32906 | USP13 | NM_003940.1 | 2338 | ggcactacgagcaacgaataata | 154317 | - | see also 14439 line |
| 32907 | USP13 | NM_003940.1 | 2340 | cactacgagcaacgaataataac | 154318 | - | see also 14439 line |
| 32908 | USP13 | NM_003940.1 | 2337 | aggcactacgagcaacgaataat | 154316 | - | see also 14439 line |
| 32909 | USP32 | NM_032582.2 | 800 | tcccggactggacgatttgattt | 154340 | - | see also 14440 line |
| 32910 | USP32 | NM_032582.2 | 1644 | ttgctcgacaacataacacttct | 154366 | - | see also 14440 line |
| 32911 | VSNL1 | NM_003385.3 | 345 | aggctaaatctcgaggaatttca | 154387 | - | see also 2283 line |
| 32912 | ACE | NM_000789.2 | 2345 | gccacgtcccggaaatatgaaga | 154422 | - | see also 488 line |
| 32913 | ACE | NM_000789.2 | 2428 | cccgaaatacgtggaactcatca | 154424 | - | see also 488 line |
| 32914 | ACE2 | NM_021804.1 | 2021 | atgtacctgttccgatcatctgt | 154526 | - | see also 8170 line |
| 32915 | ACE2 | NM_021804.1 | 1097 | atgctaacggacccaggaaatgt | 154483 | - | see also 8170 line |
| 32916 | ADAM10 | NM_001110.1 | 2352 | ctggatccccttgcaacgatttt | 154608 | - | see also 688 line |
| 32917 | ADAM10 | NM_001110.1 | 2353 | tggatccccttgcaacgatttta | 154609 | - | see also 688 line |
| 32918 | ADAM12 | NM_003474.2 | 930 | gaccctcaaggcaactaagtatg | 154631 | - | see also 14443 line |
| 32919 | ADAM12 | NM_003474.2 | 2233 | atctgcctgaatcgtcaatgtca | 154650 | - | see also 14443 line |
| 32920 | ADAM15 | NM_003815.2 | 497 | cccattatttcgcgaatccaaga | 154667 | - | see also 14444 line |
| 32921 | ADAM15 | NM_003815.2 | 153 | ttcaggacgatctcccaattagc | 154662 | - | see also 14444 line |
| 32922 | ADAM15 | NM_003815.2 | 1643 | tgcctccagacagctaatactcg | 154681 | - | see also 14444 line |
| 32923 | ADAM17 | NM_003183.3 | 2327 | tgcatcggttcgcattatcaaac | 154745 | - | see also 14445 line |
| 32924 | ADAM17 | NM_003183.3 | 1761 | aagtgccaggaggcgattaatgc | 154736 | - | see also 14445 line |
| 32925 | ADAM19 | NM_023038.2 | 932 | atgacaacgcccaattaatcacg | 154771 | - | see also 14446 line |
| 32926 | ADAM19 | NM_023038.2 | 515 | agggccaacaccttatttacaga | 154758 | - | see also 14446 line |
| 32927 | ADAM20 | NM_003814.3 | 815 | gtggtcgtggataatattagata | 154801 | - | see also 14447 line |
| 32928 | ADAM20 | NM_003814.3 | 2050 | gtgtgccagtatggttcatctgt | 154841 | - | see also 14447 line |
| 32929 | ADAM21 | NM_003813.2 | 389 | ggggctttcgaggagtattaaaa | 154853 | - | see also 14448 line |
| 32930 | ADAM21 | NM_003813.2 | 539 | tagcacgccaacagttggaattt | 154856 | - | see also 14448 line |
| 32931 | ADAM28 | NM_014265.1 | 1021 | accacagcgataatcttcttaga | 154926 | - | see also 14449 line |
| 32932 | ADAM28 | NM_014265.1 | 1461 | tgcgacctgcctgaaatgtgtaa | 154941 | - | see also 14449 line |
| 32933 | ADAM30 | NM_021794.2 | 1953 | tacctgacctaggtatgataaat | 155049 | - | see also 14450 line |
| 32934 | ADAM30 | NM_021794.2 | 1556 | gagtactgcgacgggaattcaag | 155022 | - | see also 14450 line |
| 32935 | ADAM33 | NM_025220.2 | 568 | ttctcaacccacgagatctttcg | 155059 | - | see also 8882 line |
| 32936 | ADAM8 | NM_001109.1 | 1814 | gccaggacttacacgtttacaga | 155069 | - | see also 14452 line |

Figure 46

| 32937 | ADAM8 | NM_001109.1 | 1817 | aggacttacacgtttacagatcc | 155071 | - | see also 14452 line |
|---|---|---|---|---|---|---|---|
| 32938 | ADAM9 | NM_003816.1 | 1051 | agccacgcaggcgggattaatgt | 155099 | - | see also 14453 line |
| 32939 | ADAM9 | NM_003816.1 | 248 | aagcccctaggccctattcaaaa | 155073 | - | see also 14453 line |
| 32940 | ADAM9 | NM_003816.1 | 1056 | cgcaggcgggattaatgtgtttg | 155101 | - | see also 14453 line |
| 32941 | ADAMDEC 1 | NM_014479.2 | 893 | cgcaagcaccacgtttgacaact | 155162 | - | see also 5700 line |
| 32942 | ADAMDEC 1 | NM_014479.2 | 975 | agcgggattagcttcaacaatcg | 155165 | - | see also 5700 line |
| 32943 | ADAMTS1 | NM_006988.2 | 2875 | tggtagaatgccgagacattaat | 155238 | - | see also 4880 line |
| 32944 | ADAMTS1 | NM_006988.2 | 1127 | ccccagcattcgtaattcagtta | 155182 | - | see also 4880 line |
| 32945 | ADAMTS10 | NM_030957.1 | 1698 | gtcaaatcgcgtcagtgtaaata | 155248 | - | see also 14456 line |
| 32946 | ADAMTS10 | NM_030957.1 | 2176 | tagcggaaggcttcaacttctac | 155251 | - | see also 14456 line |
| 32947 | ADAMTS12 | NM_030955.1 | 892 | accacccgcacatcgtttacagg | 155271 | - | see also 14457 line |
| 32948 | ADAMTS12 | NM_030955.1 | 1561 | tgggcagacatccgtacatcatg | 155284 | - | see also 14457 line |
| 32949 | ADAMTS13 | NM_139025.1 | 2142 | agcgagagaatatgtcacatttc | 155339 | - | see also 14458 line |
| 32950 | ADAMTS13 | NM_139025.1 | 2111 | gcccacggaagggctctttcaca | 155337 | - | see also 14458 line |
| 32951 | ADAMTS13 | NM_139025.1 | 481 | ctctgtgtggccggaatccttgc | 155336 | - | see also 14458 line |
| 32952 | ADAMTS15 | NM_139055.1 | 627 | cgccaagcgtttcgtgtctatcc | 155357 | - | see also 14459 line |
| 32953 | ADAMTS15 | NM_139055.1 | 2377 | cgggtccgctactccttctatct | 155365 | - | see also 14459 line |
| 32954 | ADAMTS17 | NM_139057.1 | 974 | agcgcgatacctcggcaataacc | 155374 | - | see also 14460 line |
| 32955 | ADAMTS17 | NM_139057.1 | 2498 | ctcgtgtacacggattgtcaaca | 155397 | - | see also 14460 line |
| 32956 | ADAMTS17 | NM_139057.1 | 886 | gacaacgtcccgctaagttgtcc | 155373 | - | see also 14460 line |
| 32957 | ADAMTS19 | NM_133638.1 | 3167 | tacgtcatcggaccgttagatgt | 155483 | - | see also 14461 line |
| 32958 | ADAMTS19 | NM_133638.1 | 1960 | agcagtcgagagcgcaaatgtcc | 155457 | - | see also 14461 line |

Figure 46

| 32959 | ADAMTS3 | NM_014243.1 | 2351 | gccaagtcgcggaccttcataga | 155556 | - | see also 14462 line | | |
|---|---|---|---|---|---|---|---|---|---|
| 32960 | ADAMTS3 | NM_014243.1 | 2356 | gtcgcggaccttcatagatcttg | 155558 | - | see also 14462 line | | |
| 32961 | ADAMTS4 | NM_005099.2 | 2491 | caggtacggatacaacaatgtgg | 155600 | - | see also 3577 line | | |
| 32962 | ADAMTS4 | NM_005099.2 | 2489 | ttcaggtacggatacaacaatgt | 155599 | - | see also 3577 line | | |
| 32963 | ADAMTS5 | NM_007038.1 | 1346 | ggctcacgaaatcggacatttac | 155609 | - | see also 4924 line | | |
| 32964 | ADAMTS5 | NM_007038.1 | 1983 | tgcccacccaatggtaaatcatt | 155617 | - | see also 4924 line | | |
| 32965 | ADAMTS6 | NM_014273.2 | 1317 | ctcacattactgcgaataccaat | 155685 | - | see also 14465 line | | |
| 32966 | ADAMTS6 | NM_014273.2 | 1682 | aacggtatcgctcctgtaacaca | 155695 | - | see also 14465 line | | |
| 32967 | ADAMTS8 | NM_007037.2 | 3261 | ggctggcagaggcgaactgtaga | 155747 | - | see also 14466 line | | |
| 32968 | ADAMTS8 | NM_007037.2 | 2603 | gaggagcgagttcaaagtgttcg | 155742 | - | see also 14466 line | | |
| 32969 | ADAMTS9 | NM_020249.2 | 2613 | aaccccgatgtacgctattcttt | 155828 | - | see also 14467 line | | |
| 32970 | ADAMTS9 | NM_020249.2 | 2041 | atgtgcgctgggtccctaaatac | 155807 | - | see also 14467 line | | |
| 32971 | ADH1A | NM_000667.2 | 1177 | tccgtaccattctgatgttttga | 155905 | - | see also 14468 line | | |
| 32972 | ADH1A | NM_000667.2 | 1176 | atccgtaccattctgatgttttg | 155904 | - | see also 14468 line | | |
| 32973 | ADH1B | NM_000668.3 | 1021 | ggctgtttatggtggctttaaga | 155906 | - | - | zinc binding | - |
| 32974 | ADH1C | NM_000669.2 | 1010 | cacgtggaaaggagctatttttg | 155907 | - | see also 14469 line | | |
| 32975 | ADH1C | NM_000669.2 | 1156 | tgcttcgctctggaaagagtatc | 155909 | - | see also 14469 line | | |
| 32976 | ADH4 | NM_000670.2 | 1423 | atcggccgtactataaatggaac | 155934 | - | see also 14470 line | | |
| 32977 | ADH4 | NM_000670.2 | 1587 | aggaaaaagcgtccgaacaatcc | 155940 | - | see also 14470 line | | |
| 32978 | ADH4 | NM_000670.2 | 1238 | aaccgatccaggaagttatcatt | 155927 | - | see also 14470 line | | |
| 32979 | ADH5 | NM_000671.2 | 685 | ggcatttcaaccggttatggtgc | 155944 | - | - | zinc binding | - |
| 32980 | ADH6 | NM_000672.2 | 305 | ggctggaatcgttgagagtattg | 155951 | - | see also 14471 line | | |
| 32981 | ADH6 | NM_000672.2 | 582 | gtcgctcctctagagaaagtatg | 155954 | - | see also 14471 line | | |
| 32982 | ADH7 | NM_000673.2 | 434 | tgcattaggagcgatattactgg | 155973 | - | see also 14472 line | | |
| 32983 | ADH7 | NM_000673.2 | 881 | aacgtgggatacacctttgaagt | 155980 | - | see also 14472 line | | |
| 32984 | AICDA | NM_020661.1 | 638 | gacttacgagacgcatttcgtac | 155999 | - | see also 14473 line | | |
| 32985 | AICDA | NM_020661.1 | 643 | acgagacgcatttcgtactttgg | 156000 | - | see also 14473 line | | |

Figure 46

| | | | | | | | |
|---|---|---|---|---|---|---|---|
| 32986 | ANPEP | NM_001150.1 | 1589 | tcctgtccgaggacgtattcaag | 156014 | - | see also 14474 line |
| 32987 | ANPEP | NM_001150.1 | 2001 | gggctattaccgggtgaactacg | 156020 | - | see also 14474 line |
| 32988 | APOBEC3A | NM_145699.2 | 105 | cacttgatcgatccacacatatt | 156033 | - | - |
| 32989 | APOBEC3G | NM_021822.1 | 1165 | gcccgcatctatgatgatcaagg | 156042 | - | see also 14475 line |
| 32990 | APOBEC3G | NM_021822.1 | 554 | tggccgaggaaccgaaggttacc | 156036 | - | see also 14475 line |
| 32991 | ARTS-1 | NM_016442.2 | 1823 | atggtccatcgattttgctaaa | 156085 | - | see also 6859 line |
| 32992 | ARTS-1 | NM_016442.2 | 1824 | tggtccatcgattttgctaaaa | 156086 | - | see also 6859 line |
| 32993 | BSPRY | NM_017688.1 | 953 | ggccgatcagaccatttctatcg | 156121 | - | see also 14477 line |
| 32994 | BSPRY | NM_017688.1 | 364 | aggagtcggaaatgttaaacttt | 156115 | - | see also 14477 line |
| 32995 | CA1 | NM_001738.1 | 820 | cacaattccgcagccttctatca | 156135 | - | see also 14478 line |
| 32996 | CA1 | NM_001738.1 | 502 | ttcacgtagctcactggaattct | 156127 | - | see also 14478 line |
| 32997 | CA10 | NM_020178.2 | 861 | cggcagtcgccagtcaacataga | 156140 | - | see also 14479 line |
| 32998 | CA10 | NM_020178.2 | 860 | acggcagtcgccagtcaacatag | 156139 | - | see also 14479 line |
| 32999 | CA11 | NM_001217.2 | 702 | ccgacctgtggtcaatgtgtct | 156165 | - | see also 14480 line |
| 33000 | CA11 | NM_001217.2 | 466 | gtggagctacaaggataatctcc | 156164 | - | see also 14480 line |
| 33001 | CA12 | NM_001218.2 | 716 | ttcgtcccggattcaacattga | 156174 | - | see also 14481 line |
| 33002 | CA12 | NM_001218.2 | 831 | tccgaaacccgtgcaaatttcc | 156178 | - | see also 14481 line |
| 33003 | CA12 | NM_001218.2 | 712 | agcattcgtcccggattcaaca | 156173 | - | see also 14481 line |
| 33004 | CA14 | NM_012113.1 | 1158 | ctgtataccacaggtgaaatgc | 156188 | - | see also 14482 line |
| 33005 | CA14 | NM_012113.1 | 1145 | tccaagcaggatcctcgtatacc | 156186 | - | see also 14482 line |
| 33006 | CA2 | NM_000067.1 | 479 | tggactggcgttctaggtattt | 156195 | - | see also 14483 line |
| 33007 | CA2 | NM_000067.1 | 484 | tggccgttctaggtattttttg | 156198 | - | see also 14483 line |
| 33008 | CA3 | NM_005181.2 | 770 | gcccgttgagctgcatactaaag | 156204 | - | see also 14484 line |
| 33009 | CA3 | NM_005181.2 | 1092 | cgcgatgggatcgctgtgattgg | 156217 | - | see also 14484 line |
| 33010 | CA3 | NM_005181.2 | 1050 | tggaacccgaagtataaacactt | 156214 | - | see also 14484 line |
| 33011 | CA4 | NM_000717.2 | 876 | ggcagcgcacggtgataaagtcc | 156233 | - | see also 14485 line |
| 33012 | CA4 | NM_000717.2 | 721 | cacaccgacctgcgatgagaagg | 156230 | - | see also 14485 line |
| 33013 | CA5A | NM_001739.1 | 384 | accgaggcatcaggaattagtgg | 156236 | - | see also 14486 line |
| 33014 | CA5A | NM_001739.1 | 656 | gccggaaataaaacataaggacg | 156237 | - | see also 14486 line |
| 33015 | CA5B | NM_007220.2 | 704 | gccgtcaattaagcataaggacg | 156252 | - | see also 5033 line |
| 33016 | CA5B | NM_007220.2 | 698 | tactttgccgtcaattaagcata | 156251 | - | see also 5033 line |
| 33017 | CA5B | NM_007220.2 | 983 | ttcatccttccgcatgattatg | 156256 | - | see also 5033 line |
| 33018 | CA6 | NM_001215.1 | 401 | gacatgtgatcgaggattccacatt | 156259 | - | see also 14488 line |
| 33019 | CA6 | NM_001215.1 | 838 | ttcccgaatcaggaataactct | 156264 | - | see also 14488 line |

Figure 46

| | | | | | | | | zinc binding |
|---|---|---|---|---|---|---|---|---|
| 33020 | CA7 | NM_005182.1 | 683 | tccggagcctgctttacctcg | 156270 | - | - | - |
| 33021 | CA7 | NM_005182.1 | 460 | gacgagcacccagcatgaatcg | 156269 | - | see also 33020 line | |
| 33022 | CA7 | NM_005182.1 | 203 | tccaggtagacttcaatgacagc | 156268 | - | see also 33020 line | |
| 33023 | CA8 | NM_004056.3 | 630 | gagcacacgttaatttcaaagc | 156287 | - | see also 2733 line | |
| 33024 | CA9 | NM_001216.1 | 1003 | ttcagccgtacttccaatatga | 156303 | - | see also 14490 line | |
| 33025 | CA9 | NM_001216.1 | 1005 | cagccgctacttccaatatgaeg | 156304 | - | see also 14490 line | |
| 33026 | CDA | NM_001785.1 | 469 | accaagccgatggtacgtatat | 156312 | - | see also 14491 line | |
| 33027 | CDA | NM_001785.1 | 379 | gccagtgacatgcaagatgattt | 156311 | - | see also 14491 line | |
| 33028 | CDADC1 | NM_030911.1 | 146 | ggcagcatgaccggtcagataac | 156315 | - | see also 14492 line | |
| 33029 | CDADC1 | NM_030911.1 | 962 | ttcggattttaccgtagcaatcc | 156340 | - | see also 14492 line | |
| 33030 | CDADC1 | NM_030911.1 | 555 | tgcttacggaggcttctagttct | 156330 | - | see also 14492 line | |
| 33031 | CGI-63 | NM_016011.1 | 856 | cgcgtggaggaaccatggtaacc | 156362 | - | see also 14493 line | |
| 33032 | CGI-63 | NM_016011.1 | 645 | ctgggcctaagaaccatcaatgt | 156358 | - | see also 14493 line | |
| 33033 | CRIP2 | NM_001312.2 | 642 | tgcggtgggcagctacatcatg | 156366 | - | see also 14494 line | |
| 33034 | CRIP2 | NM_001312.2 | 132 | ggggaaggactggcacaagttct | 156365 | - | see also 14494 line | |
| 33035 | CRYZ | NM_001889.2 | 881 | ggcaaaggagtcgagtataattg | 156376 | - | see also 14495 line | |
| 33036 | CRYZ | NM_001889.2 | 880 | tggcaaaggagtcgagtataatt | 156375 | - | see also 14495 line | |
| 33037 | CRYZL1 | NM_005111.5 | 394 | tagagtacatgagcattacttgg | 156389 | - | see also 3582 line | |
| 33038 | CSRP1 | NM_004078.1 | 470 | ggcataaggcctgctttcgatgt | 156405 | - | see also 14496 line | |
| 33039 | CSRP1 | NM_004078.1 | 552 | ctgcaaagatgttatgctaaaa | 156407 | - | see also 14496 line | |
| 33040 | DCTD | NM_001921.1 | 112 | ggcgcctgcatcgtgaattcaga | 156410 | - | see also 14497 line | |
| 33041 | DCTD | NM_001921.1 | 23 | aacgggacgactatttggaatgg | 156409 | - | see also 14497 line | |
| 33042 | DKFZP547N043 | NM_032018.2 | 500 | agccaatataacggtataccata | 156438 | - | see also 14498 line | |
| 33043 | DKFZP547N043 | NM_032018.2 | 1282 | aacgacctaggctagaagataag | 156463 | - | see also 14498 line | |
| 33044 | DPF3 | NM_012074.1 | 631 | agcaacagtggcgtataggaaaa | 156472 | - | see also 14499 line | |
| 33045 | DPF3 | NM_012074.1 | 43 | agcgctcggggaccagttctaca | 156467 | - | see also 14499 line | |
| 33046 | DPF3 | NM_012074.1 | 290 | ctgcggctgctggagataaaacc | 156469 | - | see also 14499 line | |
| 33047 | DUSP12 | NM_007240.1 | 681 | tgcaggcgatcattatttcgaag | 156490 | - | see also 14500 line | |
| 33048 | DUSP12 | NM_007240.1 | 525 | gttggatacctctagtgcaattta | 156480 | - | see also 14500 line | |
| 33049 | ECE1 | NM_001397.1 | 781 | ggcttgccctcgagagacatta | 156502 | - | see also 14501 line | |
| 33050 | ECE1 | NM_001397.1 | 827 | aggtgctgaccggatatctgaac | 156504 | - | see also 14501 line | |
| 33051 | ECEL1 | NM_004826.1 | 226 | gacgcggcgactacgatgagttcc | 156525 | - | see also 3348 line | |
| 33052 | ECEL1 | NM_004826.1 | 1907 | caggcgctcaatgctactatct | 156532 | - | see also 3348 line | |
| 33053 | EEA1 | NM_003566.1 | 3953 | gacttacggcgtgaaattgcagt | 156632 | - | see also 14503 line | |
| 33054 | EEA1 | NM_003566.1 | 2042 | aaggtctccagcttgacataca | 156587 | - | see also 14503 line | |

Figure 46

| 33055 | ENPEP | NM_001977.2 | 1872 | gggttatcctgtgcttaacgtga | 156680 | - | see also 1259 line |
|---|---|---|---|---|---|---|---|
| 33056 | ENPEP | NM_001977.2 | 2801 | cagtcattcgatatatctcatat | 156708 | - | see also 1259 line |
| 33057 | FASN | NM_004104.3 | 6236 | tccgccatggagcgtatctgtga | 156731 | - | see also 14505 line |
| 33058 | FASN | NM_004104.3 | 2314 | gtcctccgccgagtacaatgtca | 156724 | - | see also 14505 line |
| 33059 | FASN | NM_004104.3 | 6310 | cggcgacgtgggcattttggtgg | 156733 | - | see also 14505 line |
| 33060 | FBXO30 | NM_032145.3 | 2049 | ctcgatggcttcagcttatgtca | 156788 | - | see also 14506 line |
| 33061 | FBXO30 | NM_032145.3 | 1703 | acccaagcagcgttcaatgttta | 156774 | - | see also 14506 line |
| 33062 | FBXO30 | NM_032145.3 | 447 | tggaatcgatggccagttagtta | 156742 | - | see also 14506 line |
| 33063 | FGD1 | NM_004463.2 | 1573 | accgggacagcggcattgatagc | 156802 | - | see also 3013 line |
| 33064 | FGD1 | NM_004463.2 | 2560 | accgatacctcatactattcaac | 156813 | - | see also 3013 line |
| 33065 | FGD2 | NM_173558.2 | 1891 | cccctcgtgctctatgtctatgc | 156835 | - | see also 14507 line |
| 33066 | FGD2 | NM_173558.2 | 1413 | agcagccattgaccaaatcgaga | 156833 | - | see also 14507 line |
| 33067 | FGD3 | NM_033086.1 | 2194 | gccgtgtctgcagagattgtttc | 156845 | - | see also 14508 line |
| 33068 | FGD3 | NM_033086.1 | 1133 | cacggaggagtgggacacaaacc | 156839 | - | see also 14508 line |
| 33069 | FGD4 | NM_139241.1 | 1091 | aaccgacttgacctcttagatca | 156858 | - | see also 9676 line |
| 33070 | FGD4 | NM_139241.1 | 2089 | atggatccgagataatgaagtga | 156882 | - | see also 9676 line |
| 33071 | FLJ10759 | NM_018207.1 | 858 | gaccgacatcgagcagaaagtcc | 156894 | - | see also 14510 line |
| 33072 | FLJ10759 | NM_018207.1 | 1256 | tgggttctgaagccttcagtagt | 156896 | - | see also 14510 line |
| 33073 | FLJ23049 | NM_024687.1 | 1021 | atcgtttagaatgcaacaattcc | 156926 | - | see also 14511 line |
| 33074 | FLJ23049 | NM_024687.1 | 514 | atcaacatcatgttttcgataag | 156905 | - | see also 14511 line |
| 33075 | FLJ32919 | NM_144588.3 | 1151 | aacgggttcctgagcaagaatga | 156948 | - | see also 14512 line |
| 33076 | FLJ32919 | NM_144588.3 | 480 | cagtaggtgccctgatgatttca | 156941 | - | see also 14512 line |
| 33077 | FLJ40722 | NM_173678.1 | 1307 | tccgcaaggcgctctctcaattc | 156954 | - | see also 14513 line |
| 33078 | FLJ40722 | NM_173678.1 | 1423 | gaggggggtccctgcttacatatc | 156959 | - | see also 14513 line |
| 33079 | FYCO1 | NM_024513.1 | 4607 | gtgatctacgatggaagtgattt | 156997 | - | see also 14514 line |
| 33080 | FYCO1 | NM_024513.1 | 3234 | tggactaccagagcagacttaag | 156978 | - | see also 14514 line |
| 33081 | GDA | NM_004293.1 | 786 | aacccgtgatttgcacattcaga | 157019 | - | see also 2875 line |
| 33082 | GDA | NM_004293.1 | 788 | cccgtgatttgcacattcagagc | 157020 | - | see also 2875 line |
| 33083 | GGT1 | NM_005265.1 | 638 | tacaacagcaccacacgaaaagc | 157039 | - | - | - | glutathionuria |
| 33084 | HGS | NM_004712.3 | 1883 | cccaatgcacggcgtgtacatga | 157053 | - | see also 14516 line |
| 33085 | HGS | NM_004712.3 | 246 | aacccacacgtcgccttgtatgc | 157041 | - | see also 14516 line |
| 33086 | HS3ST1 | NM_005114.1 | 565 | gtcggagcgcgtgctatctgact | 157057 | - | see also 14517 line |
| 33087 | HS3ST1 | NM_005114.1 | 947 | gtcgatcccaaactactcaataa | 157062 | - | see also 14517 line |
| 33088 | HSXIAPAF1 | NM_017523.2 | 1070 | gccgatcctaaatcaacatcagg | 157084 | - | see also 14518 line |
| 33089 | HSXIAPAF1 | NM_017523.2 | 1064 | tcccctgccgatcctaaatcaac | 157081 | - | see also 14518 line |

Figure 46

| 33090 | KEL | NM_000420.2 | 1749 | gtccctccgagctagaattgtcc | 157109 | - | see also 14519 line |
|---|---|---|---|---|---|---|---|
| 33091 | KEL | NM_000420.2 | 2038 | tgcctgaagcgccattatgctgc | 157117 | - | see also 14519 line |
| 33092 | KIAA1576 | NM_020927.1 | 317 | cgcgtcaaagcctgtggattaaa | 157122 | - | see also 14520 line |
| 33093 | KIAA1576 | NM_020927.1 | 357 | tgcgacaagggaatattgacaac | 157124 | - | see also 14520 line |
| 33094 | KPNB1 | NM_002265.4 | 2885 | ggcggagatcgaagactaacaaa | 157208 | - | see also 1451 line |
| 33095 | KPNB1 | NM_002265.4 | 2883 | agggcggagatcgaagactaaca | 157207 | - | see also 1451 line |
| 33096 | KUB3 | NM_033276.1 | 413 | gtggtcacacacgagcttattca | 157220 | - | see also 14522 line |
| 33097 | KUB3 | NM_033276.1 | 761 | aaccgtgatcggtattattcaaa | 157231 | - | see also 14522 line |
| 33098 | LAP3 | NM_015907.1 | 988 | ggcagccccaatgcaaacgaacc | 157236 | - | see also 14523 line |
| 33099 | LAP3 | NM_015907.1 | 851 | atctcaaaagtgctagtagtaaa | 157234 | - | see also 14523 line |
| 33100 | LMLN | NM_033029.1 | 125 | gtgtgggtccgaagcgttttact | 157241 | - | see also 14524 line |
| 33101 | LMLN | NM_033029.1 | 1049 | ctggtaacgcctcgtgttgttga | 157261 | - | see also 14524 line |
| 33102 | LNPEP | NM_005575.1 | 2933 | ctggggtcctataccatacaaaa | 157352 | - | see also 3877 line |
| 33103 | LNPEP | NM_005575.1 | 848 | tactcggcaaatatatctagttc | 157301 | - | see also 3877 line |
| 33104 | LNPEP | NM_005575.1 | 843 | tagagtactcggcaaatatatct | 157300 | - | see also 3877 line |
| 33105 | LOC51321 | NM_016627.1 | 691 | gacagatggtgtggggatattca | 157358 | - | see also 6962 line |
| 33106 | LOC51321 | NM_016627.1 | 806 | ttcgacaactattatattccaga | 157361 | - | see also 6962 line |
| 33107 | LOC51321 | NM_016627.1 | 846 | tacttcgatcctgtaagacttta | 157364 | - | see also 6962 line |
| 33108 | LOC51725 | NM_016298.1 | 1653 | ctcgcctacttgggatgtacatt | 157402 | - | see also 14527 line |
| 33109 | LOC51725 | NM_016298.1 | 2185 | aggcccgagagagcttagtctcc | 157411 | - | see also 14527 line |
| 33110 | LRAP | NM_022350.1 | 2784 | gacttgggctcatatgacataag | 157486 | - | see also 14528 line |
| 33111 | LRAP | NM_022350.1 | 1321 | aacttatcgctgttaatgctaca | 157447 | - | see also 14528 line |
| 33112 | LTA4H | NM_000895.1 | 808 | atctgggaggaccgtatgtatgg | 157514 | - | see also 582 line |
| 33113 | LTA4H | NM_000895.1 | 1050 | cacatttgcggacgattgtttgg | 157523 | - | see also 582 line |
| 33114 | LTB4DH | NM_012212.2 | 775 | cactgttatcggccagatgaaga | 157577 | - | see also 5200 line |
| 33115 | LTB4DH | NM_012212.2 | 141 | ttgttggctatcctactaatagt | 157551 | - | see also 5200 line |
| 33116 | MADHIP | NM_004799.1 | 581 | ttgtagccgtcatgcataactgt | 157585 | - | see also 14531 line |
| 33117 | MADHIP | NM_004799.1 | 901 | tccatgtctgcgattacaagttt | 157602 | - | see also 14531 line |
| 33118 | MBTPS2 | NM_015884.1 | 997 | atcgcctatgagccccaaattgg | 157712 | - | see also 6557 line |
| 33119 | MBTPS2 | NM_015884.1 | 344 | ttggcgtaattgccatgtttagc | 157693 | - | see also 6557 line |
| 33120 | MEP1A | NM_005588.1 | 1387 | tacaattcggagggatatggttt | 157755 | - | see also 14533 line |
| 33121 | MEP1A | NM_005588.1 | 762 | gaggctgaaccgaatgtacaatt | 157747 | - | see also 14533 line |
| 33122 | MEP1B | NM_005925.1 | 1583 | tccaatcagcggagtataactac | 157825 | - | see also 4142 line |
| 33123 | MEP1B | NM_005925.1 | 2102 | atgagctcaaatcgaccaaattt | 157841 | - | see also 4142 line |
| 33124 | MGC45594 | NM_175907.2 | 959 | tacgaaagggcgcttgatagtaa | 157862 | - | see also 10157 line |

Figure 46

| | | | | | | | |
|---|---|---|---|---|---|---|---|
| 33125 | MGC45594 | NM_175907.2 | 857 | acccgtaggtaccgtccttaagc | 157859 | - | see also 10157 line |
| 33126 | MID2 | NM_012216.2 | 2078 | ctctcgctgcaatagtaacttcg | 157922 | - | see also 5209 line |
| 33127 | MID2 | NM_012216.2 | 951 | aagcgcaacagcgaactagaaaa | 157887 | - | see also 5209 line |
| 33128 | MIPEP | NM_005932.1 | 783 | cacattcgtcgtaactttacatc | 157940 | - | see also 4143 line |
| 33129 | MIPEP | NM_005932.1 | 781 | aacacattcgtcgtaactttaca | 157939 | - | see also 4143 line |
| 33130 | MIPEP | NM_005932.1 | 958 | tggtggggtattccacgttttct | 157950 | - | see also 4143 line |
| 33131 | MIRAB13 | NM_033386.1 | 2433 | agcgcaacgctatcatcaactgc | 157991 | - | see also 14539 line |
| 33132 | MIRAB13 | NM_033386.1 | 2430 | agcagcgcaacgctatcatcaac | 157990 | - | see also 14539 line |
| 33133 | MIRAB13 | NM_033386.1 | 2334 | agctccggtgcctcctcaataag | 157989 | - | see also 14539 line |
| 33134 | MME | NM_000902.2 | 401 | ctcccgttacggcaactttgaca | 158003 | - | see also 586 line |
| 33135 | MME | NM_000902.2 | 399 | agctcccgttacggcaactttga | 158002 | - | see also 586 line |
| 33136 | MMEL2 | NM_033467.1 | 2220 | ggccccgagttcgccatccaatcc | 158086 | - | see also 14541 line |
| 33137 | MMEL2 | NM_033467.1 | 1245 | tggtgctggaccgcattggtagc | 158076 | - | see also 14541 line |
| 33138 | MMP10 | NM_002425.1 | 1275 | agcaaggcttccctagactaata | 158102 | - | see also 14542 line |
| 33139 | MMP10 | NM_002425.1 | 917 | agcactctgaggggagaatatct | 158092 | - | see also 14542 line |
| 33140 | MMP14 | NM_004995.2 | 1795 | gagacggaggtgatcatcattga | 158124 | - | see also 3488 line |
| 33141 | MMP14 | NM_004995.2 | 1462 | ctgcctaccgacaagattgatgc | 158119 | - | see also 3488 line |
| 33142 | MMP15 | NM_002428.1 | 1318 | gagcgccaagacggtcgttttgt | 158127 | - | see also 1565 line |
| 33143 | MMP15 | NM_002428.1 | 2007 | tgcgccacgtgtcctgctttact | 158132 | - | see also 1565 line |
| 33144 | MMP16 | NM_005941.2 | 457 | tcgtcgaaagcgatatgcattga | 158146 | - | see also 4147 line |
| 33145 | MMP16 | NM_005941.2 | 456 | ttcgtcgaaagcgatatgcattg | 158145 | - | see also 4147 line |
| 33146 | MMP17 | NM_016155.2 | 570 | cacggtgcgtgcactcatgtact | 158205 | - | see also 14546 line |
| 33147 | MMP17 | NM_016155.2 | 1565 | ccgacggtgcctcctacttcttc | 158212 | - | see also 14546 line |
| 33148 | MMP19 | NM_002429.2 | 1174 | aaggtgtggcgctacattaattt | 158232 | - | see also 14547 line |
| 33149 | MMP19 | NM_002429.2 | 1519 | cactgtcgtccccggactataga | 158243 | - | see also 14547 line |
| 33150 | MMP20 | NM_004771.2 | 133 | cgcacaggcgtatcttgacaaat | 158251 | - | see also 3283 line |
| 33151 | MMP20 | NM_004771.2 | 823 | cggacctcggaaagtattcctgg | 158272 | - | see also 3283 line |
| 33152 | MMP21 | NM_147191.1 | 1347 | tcccctagacacggcgttttatg | 158304 | - | see also 9814 line |
| 33153 | MMP21 | NM_147191.1 | 1348 | cccctagacacggcgttttatga | 158305 | - | see also 9814 line |
| 33154 | MMP24 | NM_006690.2 | 545 | agctagacacgcggaaagctatt | 158324 | - | see also 4723 line |
| 33155 | MMP24 | NM_006690.2 | 1285 | cgcatcgacgcagcctatgaaag | 158328 | - | see also 4723 line |
| 33156 | MMP25 | NM_022468.3 | 1815 | gtccgaaacctgcgattgtcagt | 158337 | - | see also 14551 line |
| 33157 | MMP25 | NM_022468.3 | 1082 | cgccccaaacccatatgacaag | 158336 | - | see also 14551 line |
| 33158 | MMP26 | NM_021801.2 | 741 | tgccgatgatatccaaaggatcc | 158357 | - | see also 14552 line |
| 33159 | MMP26 | NM_021801.2 | 25 | ctcgtcatcttaagagttactat | 158340 | - | see also 14552 line |
| 33160 | MMP27 | NM_022122.1 | 504 | tcggtgtcctcgctattttgatg | 158368 | - | see also 14553 line |

Figure 46

| | | | | | | | |
|---|---|---|---|---|---|---|---|
| 33161 | MMP27 | NM_022122.1 | 501 | tggtcggttcctgctatttg | 158367 | - | see also 14553 line |
| 33162 | MMP27 | NM_022122.1 | 505 | cggtgtcctgctattttgatgg | 158369 | - | see also 14553 line |
| 33163 | MMP28 | NM_024302.2 | 540 | cccgctgcgggttacagatac | 158402 | - | see also 8607 line |
| 33164 | MMP28 | NM_024302.2 | 1400 | tgcgcagtgtcattgaatgatg | 158410 | - | see also 8607 line |
| 33165 | MMP9 | NM_004994.1 | 1136 | tgcgctaccacctcgaacttga | 158419 | - | see also 14555 line |
| 33166 | MMP9 | NM_004994.1 | 1182 | gcccggaccaaggatacagtttg | 158421 | - | see also 14555 line |
| 33167 | MNAT1 | NM_002431.1 | 54 | ctcggttgaagaccaccaaatat | 158433 | - | see also 1566 line |
| 33168 | MPI | NM_002435.1 | 1235 | gccgaaggacctgctgatattcc | 158446 | - | see also 14557 line |
| 33169 | MPI | NM_002435.1 | 920 | gccaaccctgtgaaatgctca | 158444 | - | see also 14557 line |
| 33170 | MPRP-1 | NM_145243.2 | 1508 | cagaccctgattactattcaaa | 158492 | - | see also 14558 line |
| 33171 | MPRP-1 | NM_145243.2 | 961 | gtggttgattcccaattattaa | 158471 | - | see also 14558 line |
| 33172 | MT4 | NM_032935.1 | 63 | atgcacaacctgcaactgtaaaa | 158497 | - | zinc binding | - |
| 33173 | MTMR4 | NM_004687.3 | 236 | ctctaactaccggctgcatatca | 158499 | - | see also 3215 line |
| 33174 | MTMR4 | NM_004687.3 | 240 | aactaccggctgcatatcaaatt | 158500 | - | see also 3215 line |
| 33175 | NLN | NM_020726.1 | 1661 | tgcacgatttagccggaacaaatg | 158601 | - | see also 14560 line |
| 33176 | NLN | NM_020726.1 | 1062 | agccgcgtaacagcctttctaga | 158580 | - | see also 14560 line |
| 33177 | NPEPPS | NM_006310.2 | 2485 | gacactgtatcggtaattggtgg | 158657 | - | see also 4404 line |
| 33178 | PAM | NM_015057.1 | 255 | gaccgctaccgaggatttatac | 158664 | - | see also 14562 line |
| 33179 | PAM | NM_015057.1 | 4845 | atcttccgattggtatgatgg | 158773 | - | see also 14562 line |
| 33180 | PAM | NM_015057.1 | 2617 | aacgcaagtgttaatcagaagg | 158720 | - | see also 14562 line |
| 33181 | PAPPA | NM_002581.3 | 4806 | aggccagtgtcggttccaaacg | 159097 | - | see also 14563 line |
| 33182 | PAPPA | NM_002581.3 | 3254 | aacaatggcagcacatgaataag | 159071 | - | see also 14563 line |
| 33183 | PEX10 | NM_002617.3 | 111 | gggcgcagaagagacgagtactacc | 159102 | - | adrenoleukodystrophy, peroxisome biogenesis disorder, Zellweger syndrome | - |
| 33184 | PLEKHF1 | NM_024310.2 | 815 | acggagcgccaggaatggattag | 159105 | - | see also 8608 line |
| 33185 | PLEKHF2 | NM_024613.2 | 363 | aaggagtattgactaagttgtgc | 159115 | - | see also 14565 line |
| 33186 | PLEKHF2 | NM_024613.2 | 318 | cagctgtcaacctttaactata | 159110 | - | see also 14565 line |
| 33187 | POLR2H | NM_006232.2 | 262 | gtcatagctagtaccttgtatga | 159134 | - | see also 14566 line |
| 33188 | POLR2H | NM_006232.2 | 267 | agctagtaccttgtatgaagatg | 159135 | - | see also 14566 line |
| 33189 | POLR2H | NM_006232.2 | 339 | ggctgaccagtttgagtatgtaa | 159137 | - | see also 14566 line |
| 33190 | PTER | NM_030664.2 | 641 | agcttaccgatgtccttatgaat | 159155 | - | see also 14567 line |
| 33191 | PTER | NM_030664.2 | 1136 | gaggtcacggctattcctcatata | 159183 | - | see also 14567 line |
| 33192 | PTPRG | NM_002841.2 | 1378 | ttgaagggtgtcgtacatcatga | 159209 | - | see also 1862 line |
| 33193 | PTPRG | NM_002841.2 | 1306 | atcggagccatggccatatttt | 159205 | - | see also 1862 line |
| 33194 | PTPRZ1 | NM_002851.1 | 1148 | gacctcgagtcgtttatgtatacc | 159322 | - | see also 14569 line |
| 33195 | PTPRZ1 | NM_002851.1 | 2388 | gacaacccaacggtatacaatg | 159359 | - | see also 14569 line |
| 33196 | PTPRZ1 | NM_002851.1 | 620 | ttgatgcggacgattttcaagt | 159309 | - | see also 14569 line |

Figure 46

| 33197 | RABIF | NM_002871.3 | 51 | agcagccgagcgagttagtgtca | 159496 | - | see also 1907 line | | |
| 33198 | RAI17 | NM_020338.1 | 3217 | cccgacctgccgagcaatagtaa | 159526 | - | see also 7834 line | | |
| 33199 | RFP2 | NM_005798.2 | 1270 | gccgatttcatagaacaatcagt | 159560 | - | see also 14571 line | | |
| 33200 | RFP2 | NM_005798.2 | 347 | gtcctacatgccgtaaggaaact | 159533 | - | see also 14571 line | | |
| 33201 | RFP2 | NM_005798.2 | 1266 | aacagccgatttcatagaacaat | 159558 | - | see also 14571 line | | |
| 33202 | RFWD1 | NM_032271.1 | 1059 | ggcgggacgcatccatgttaaat | 159568 | - | see also 14572 line | | |
| 33203 | RFWD1 | NM_032271.1 | 1060 | gcgggacgcatccatgttaaatg | 159569 | - | see also 14572 line | | |
| 33204 | RIMS1 | NM_014989.1 | 3277 | cccgaagtccagttgatcataga | 159642 | - | see also 14573 line | | |
| 33205 | RIMS1 | NM_014989.1 | 3178 | agccgcgttcacgtttaccaaat | 159634 | - | see also 14573 line | | |
| 33206 | RIMS1 | NM_014989.1 | 716 | ctcctattgtcgcactaagttct | 159581 | - | see also 14573 line | | |
| 33207 | RING1 | NM_002931.2 | 321 | tcctagccgggaggaatacgagg | 159649 | - | see also 14574 line | | |
| 33208 | RING1 | NM_002931.2 | 822 | ctgccagacgaggtatgtgaaga | 159650 | - | see also 14574 line | | |
| 33209 | RNF129 | NM_152620.2 | 1328 | ggcaattgggcgatacatgaaga | 159665 | - | see also 14575 line | | |
| 33210 | RNF129 | NM_152620.2 | 610 | gccttgagcccttgaggaataat | 159659 | - | see also 14575 line | | |
| 33211 | RNF137 | NM_018073.5 | 773 | acccgaaaacagagtattgtatg | 159671 | - | see also 14576 line | | |
| 33212 | RNF137 | NM_018073.5 | 1605 | ggcgcctcctgccctattttagt | 159691 | - | see also 14576 line | | |
| 33213 | RNF26 | NM_032015.3 | 738 | cacacggtactgactagtcttct | 159693 | - | see also 14577 line | | |
| 33214 | RNF26 | NM_032015.3 | 1533 | gaccctatgagggtattctcagt | 159696 | - | see also 14577 line | | |
| 33215 | RNF28 | NM_032588.2 | 267 | gccggaagtgtgccaatgacatc | 159697 | - | see also 14578 line | | |
| 33216 | RNF28 | NM_032588.2 | 1043 | gagaacatggacttctttacttt | 159703 | - | see also 14578 line | | |
| 33217 | RNF29 | NM_033058.2 | 367 | taggaaatgtgccagtgatattt | 159707 | - | see also 14579 line | | |
| 33218 | RNF29 | NM_033058.2 | 261 | aagagcagcagaccatggataac | 159705 | - | see also 14579 line | | |
| 33219 | RNF30 | NM_032546.2 | 480 | ggccccactgcccaccatttaca | 159728 | - | see also 14580 line | | |
| 33220 | RNF30 | NM_032546.2 | 745 | cggaggcagaagcagttgttaaa | 159733 | - | see also 14580 line | | |
| 33221 | RNF39 | NM_025236.2 | 480 | cgccgcagcctgaggtctaatgt | 159737 | - | see also 14581 line | | |
| 33222 | RNF39 | NM_025236.2 | 85 | gactgagactctggttgaagaga | 159734 | - | see also 14581 line | | |
| 33223 | RNF6 | NM_005977.2 | 816 | acctttcggcgcacaggaaatgc | 159752 | - | see also 4172 line | | |
| 33224 | RNF6 | NM_005977.2 | 820 | ttcggcgcacaggaaatgcaact | 159753 | - | see also 4172 line | | |
| 33225 | RNF7 | NM_014245.2 | 202 | gtcgggaggcgacaagatgttct | 159821 | - | - | - | - |
| 33226 | RNPEP | NM_020216.3 | 1239 | gacccggacgacacctataatga | 159837 | - | see also 7802 line | | |
| 33227 | RNPEP | NM_020216.3 | 573 | ttcgacacgcctgctgttaaata | 159825 | - | see also 7802 line | | |
| 33228 | RPH3A | NM_014954.1 | 1655 | gtccaacaagcttcgtacaaaaa | 159870 | - | see also 14584 line | | |
| 33229 | RPH3A | NM_014954.1 | 1920 | gcccgaggcatggccctttatga | 159875 | - | see also 14584 line | | |
| 33230 | RPH3A | NM_014954.1 | 1055 | ctcatctagccgagattcagaga | 159866 | - | see also 14584 line | | |
| 33231 | RTN4IP1 | NM_032730.2 | 1009 | agcttggtgcagacgatgtaatt | 159912 | - | see also 9278 line | | |
| 33232 | RTN4IP1 | NM_032730.2 | 1353 | aagatccggccagttattgaaca | 159929 | - | see also 9278 line | | |
| 33233 | RUFY1 | NM_025158.2 | 552 | agcgactagtgtcagaaatcttc | 159938 | - | see also 8872 line | | |

Figure 46

| 33234 | RUFY1 | NM_025158.2 | 1362 | agcgattaacttacagatgtttc | 159950 | - | see also 8872 line | | |
|---|---|---|---|---|---|---|---|---|---|
| 33235 | RUFY2 | NM_017987.2 | 366 | gccgattacttacgttgcttaat | 159967 | - | see also 7189 line | | |
| 33236 | RUFY2 | NM_017987.2 | 963 | gagctagcagtacaagttagtat | 159994 | - | see also 7189 line | | |
| 33237 | SORD | NM_003104.3 | 189 | tgcgcctggagaactatcctatc | 160011 | - | - | zinc binding | - |
| 33238 | SORD | NM_003104.3 | 185 | gacttgcgcctggagaactatcc | 160010 | - | see also 33237 line | | |
| 33239 | THOP1 | NM_003249.3 | 1157 | ctgggacatgcgctactacatga | 160013 | - | see also 2189 line | | |
| 33240 | TIMM10 | NM_012456.1 | 182 | tggccgatatgtacaacagaatg | 160019 | - | see also 14589 line | | |
| 33241 | TIMM10 | NM_012456.1 | 225 | gtgtgtgcctcctcactacaagg | 160021 | - | see also 14589 line | | |
| 33242 | TIMM13 | NM_012458.2 | 477 | aagtgtttccggaagtgtatagg | 160025 | - | see also 5338 line | | |
| 33243 | TIMM8B | NM_012459.1 | 184 | agctgtgtagaccgcttcattga | 160033 | - | see also 14591 line | | |
| 33244 | TIMM8B | NM_012459.1 | 152 | gcctagactctcgcactgaaaat | 160029 | - | see also 14591 line | | |
| 33245 | TIMM9 | NM_012460.2 | 529 | gacttcacaacaagagaagtaaa | 160038 | - | see also 14592 line | | |
| 33246 | TIMM9 | NM_012460.2 | 579 | ttgcttacagaaatatttaaaaa | 160039 | - | see also 14592 line | | |
| 33247 | TP53I3 | NM_004881.2 | 1013 | acccggatggctggagctattcc | 160043 | - | see also 14593 line | | |
| 33248 | TP53I3 | NM_004881.2 | 1330 | gaggtctagggacaataagtaca | 160046 | - | see also 14593 line | | |
| 33249 | TRAF2 | NM_021138.2 | 1169 | gccgcatacccgccatcttctcc | 160055 | - | see also 14594 line | | |
| 33250 | TRAF2 | NM_021138.2 | 1431 | gccagtcaacgacatgaacatcg | 160059 | - | see also 14594 line | | |
| 33251 | TRAF3 | NM_003300.2 | 1618 | gacgcacttgtcgctgtttttg | 160077 | - | see also 2231 line | | |
| 33252 | TRAF3 | NM_003300.2 | 1009 | gtggagcaactcgctcgaaaaga | 160070 | - | see also 2231 line | | |
| 33253 | TRAF4 | NM_004295.2 | 495 | ggcgcctcaagtgcgagttttgt | 160082 | - | see also 14596 line | | |
| 33254 | TRAF4 | NM_004295.2 | 292 | cccggagctggaagtacaagtat | 160080 | - | see also 14596 line | | |
| 33255 | TRAF5 | NM_004619.2 | 1385 | gagggtacttgctataatggaaa | 160121 | - | see also 14597 line | | |
| 33256 | TRAF5 | NM_004619.2 | 1382 | ctggagggtacttgctataatgg | 160120 | - | see also 14597 line | | |
| 33257 | TRHDE | NM_013381.1 | 1141 | ggggttgtagtacgattatatgc | 160155 | - | see also 5414 line | | |
| 33258 | TRHDE | NM_013381.1 | 1137 | gagtggggttgtagtacgattat | 160152 | - | see also 5414 line | | |
| 33259 | TRIM10 | NM_006778.2 | 763 | ctgccggtttagtgctcttattg | 160239 | - | see also 4778 line | | |
| 33260 | TRIM10 | NM_006778.2 | 641 | ttcgcacacctgaggaagtttct | 160235 | - | see also 4778 line | | |
| 33261 | TRIM11 | NM_145214.2 | 1515 | caccgatgggtcactgctattca | 160250 | - | see also 14600 line | | |
| 33262 | TRIM11 | NM_145214.2 | 1598 | gcccgaccccgatgactatctgc | 160253 | - | see also 14600 line | | |
| 33263 | TRIM15 | NM_033229.1 | 720 | gcccgctgggagaaacttactgc | 160254 | - | - | - | - |
| 33264 | TRIM17 | NM_016102.1 | 1288 | tccaaggggaccaagtacttatc | 160271 | - | see also 14601 line | | |
| 33265 | TRIM17 | NM_016102.1 | 949 | tgcagagttcccggacagattga | 160268 | - | see also 14601 line | | |
| 33266 | TRIM31 | NM_007028.2 | 1272 | agcgagctctcaggatacgaaga | 160283 | - | see also 14602 line | | |
| 33267 | TRIM31 | NM_007028.2 | 1349 | cagcgatccgggcttggttttgt | 160285 | - | see also 14602 line | | |
| 33268 | TRIM31 | NM_007028.2 | 704 | tcctgctatcacggatttactgg | 160277 | - | see also 14602 line | | |
| 33269 | TRIM34 | NM_021616.3 | 684 | atcagcttagaagcatcctaaat | 160290 | - | see also 14603 line | | |
| 33270 | TRIM34 | NM_021616.3 | 295 | gtgtggtatcagttactcatttg | 160288 | - | see also 14603 line | | |

Figure 46

| 33271 | TRIM35 | NM_015066.1 | 1390 | gggcgagctgtctttctatgacg | 160312 | - | see also 14604 line |
|---|---|---|---|---|---|---|---|
| 33272 | TRIM35 | NM_015066.1 | 644 | cggcaggagtttgataagcttcg | 160310 | - | see also 14604 line |
| 33273 | TRIM36 | NM_018700.2 | 1898 | ttctagcgataaactacaagaat | 160373 | - | see also 14605 line |
| 33274 | TRIM36 | NM_018700.2 | 303 | ctgctgactctcgatgattcatt | 160317 | - | see also 14605 line |
| 33275 | TRIM37 | NM_015294.1 | 996 | tggagttgtgcgaggttactact | 160413 | - | see also 6316 line |
| 33276 | TRIM37 | NM_015294.1 | 1003 | gtgcgaggttactacttatctgt | 160416 | - | see also 6316 line |
| 33277 | TRIM38 | NM_006355.2 | 1337 | ctcatcacgaactaattctctct | 160486 | - | see also 14607 line |
| 33278 | TRIM38 | NM_006355.2 | 1296 | aaggagtcatcaagttagtgtga | 160484 | - | see also 14607 line |
| 33279 | TRIM39 | NM_021253.2 | 816 | taccgcagtctccgacctaatcg | 160496 | - | see also 8148 line |
| 33280 | TRIM4 | NM_033017.2 | 1325 | gaggacgtcatgggaattactga | 160528 | - | see also 14608 line |
| 33281 | TRIM4 | NM_033017.2 | 833 | aacacgttaaaactcaatcaaac | 160520 | - | see also 14608 line |
| 33282 | TRIM40 | NM_138700.2 | 781 | agcggccagaatccttgacatct | 160534 | - | see also 14609 line |
| 33283 | TRIM40 | NM_138700.2 | 495 | tgctagggacaggctatatctgc | 160533 | - | see also 14609 line |
| 33284 | TRIM41 | NM_033549.2 | 1286 | aacccatcataaggaaaaggtgg | 160546 | - | see also 14610 line |
| 33285 | TRIM41 | NM_033549.2 | 1266 | gtgggtgctgcccgtgaatcaac | 160544 | - | see also 14610 line |
| 33286 | TRIM42 | NM_152616.3 | 1332 | ctcaagttgcgcagcattcttca | 160564 | - | see also 9901 line |
| 33287 | TRIM42 | NM_152616.3 | 2119 | ctcctcctaacaacgtacaaatg | 160576 | - | see also 9901 line |
| 33288 | TRIM42 | NM_152616.3 | 1588 | agctccggctgcactcaataaac | 160566 | - | see also 9901 line |
| 33289 | TRIM44 | NM_017583.3 | 1000 | ggccgctatgatcgaattggtgg | 160588 | - | see also 7044 line |
| 33290 | TRIM44 | NM_017583.3 | 1059 | ctcgggaccaaatgaagatgttt | 160590 | - | see also 7044 line |
| 33291 | TRIM44 | NM_017583.3 | 848 | gaccatgggcttgatttgagtac | 160585 | - | see also 7044 line |
| 33292 | TRIM45 | NM_025188.1 | 2232 | agcgcaccgaggagtctacttag | 160626 | - | see also 14613 line |
| 33293 | TRIM45 | NM_025188.1 | 1613 | tagcacccgtcctggagtaaatg | 160608 | - | see also 14613 line |
| 33294 | TRIM46 | NM_025058.1 | 395 | ctggaccggctgcttaagtcagg | 160631 | - | see also 14614 line |
| 33295 | TRIM46 | NM_025058.1 | 811 | atgccaacgcctggtatgtcaac | 160633 | - | see also 14614 line |
| 33296 | TRIM47 | NM_033452.1 | 1306 | tgcctatattgtggatttggaca | 160648 | - | see also 14615 line |
| 33297 | TRIM47 | NM_033452.1 | 1380 | gggtgctgtgtcctatcaactac | 160650 | - | see also 14615 line |
| 33298 | TRIM5 | NM_033034.1 | 1006 | tggatggcgtcataaaaaggacg | 160659 | - | see also 14616 line |
| 33299 | TRIM5 | NM_033034.1 | 764 | ctggaagactcaaatacagtatg | 160657 | - | see also 14616 line |
| 33300 | TRIM52 | NM_032765.2 | 956 | gggaaaccggagtaatgatcagg | 160692 | - | see also 14618 line |
| 33301 | TRIM52 | NM_032765.2 | 954 | cggggaaaccggagtaatgatca | 160691 | - | see also 14618 line |
| 33302 | TRIM6 | NM_058166.2 | 751 | aagaaggggctacgaattataga | 160698 | - | see also 14619 line |
| 33303 | TRIM6 | NM_058166.2 | 1092 | gaggtttgtgggagctaaagtat | 160704 | - | see also 14619 line |
| 33304 | TRIM8 | NM_030912.1 | 1415 | ctgtggacaactgttactgttct | 160718 | - | see also 8960 line |
| 33305 | TRIM8 | NM_030912.1 | 1410 | ctgttctgtggacaactgttact | 160717 | - | see also 8960 line |
| 33306 | TRIM9 | NM_015163.3 | 608 | cgcgtactggaaggggtaattga | 160725 | - | see also 14620 line |
| 33307 | TRIM9 | NM_015163.3 | 706 | gtgcgaacagtgcgatgtcttct | 160726 | - | see also 14620 line |

Figure 46

| 33308 | TSBF1 | NM_173084.1 | 818 | gtgatgttggcaatctaattaat | 160756 | - | see also 14621 line |
|---|---|---|---|---|---|---|---|
| 33309 | TSBF1 | NM_173084.1 | 1028 | atcctcgagtaagcaaaatattg | 160766 | - | see also 14621 line |
| 33310 | WDFY1 | NM_020830.2 | 826 | tcctcggacggcggaattgcagt | 160789 | - | see also 14622 line |
| 33311 | WDFY1 | NM_020830.2 | 1196 | gtgggaccgaccgcattgtaaag | 160797 | - | see also 14622 line |
| 33312 | WDFY2 | NM_052950.2 | 935 | agcacacgcgacaattgatctcc | 160821 | - | see also 9467 line |
| 33313 | WDFY2 | NM_052950.2 | 360 | gccaagcgtataccatgcaatgc | 160806 | - | see also 9467 line |
| 33314 | WDFY2 | NM_052950.2 | 930 | tgcacagcacacgcgacaattga | 160819 | - | see also 9467 line |
| 33315 | ZADH1 | NM_152444.1 | 1001 | cccgctatcccctgctatagagg | 161164 | - | see also 14623 line |
| 33316 | ZADH1 | NM_152444.1 | 1102 | tacagctgagtcagtggtttaaa | 161166 | - | see also 14623 line |
| 33317 | ZADH1 | NM_152444.1 | 841 | cagaacagctccgtgaatcatgc | 161154 | - | see also 14623 line |
| 33318 | ZFPL1 | NM_006782.2 | 779 | cgcccctaggaaggtgtatgata | 161174 | - | see also 14624 line |
| 33319 | ZFPL1 | NM_006782.2 | 421 | cggcacctgccggctatcagtgc | 161171 | - | see also 14624 line |
| 33320 | ZFYVE1 | NM_021260.1 | 2030 | cagtatgacaaccgagtgtatac | 161203 | - | see also 14625 line |
| 33321 | ZFYVE1 | NM_021260.1 | 2458 | gtgtgcgacgtcctttaaagata | 161210 | - | see also 14625 line |
| 33322 | ZFYVE16 | NM_014733.1 | 4311 | ggcttgcggctagctttacgaga | 161356 | - | see also 5923 line |
| 33323 | ZFYVE16 | NM_014733.1 | 3440 | tgctaatctactcgtgaatgtca | 161326 | - | see also 5923 line |
| 33324 | ZFYVE16 | NM_014733.1 | 604 | tgggacgatgtagtaaacctatc | 161224 | - | see also 5923 line |
| 33325 | ZFYVE19 | NM_032850.3 | 299 | ctgcagtacggctgtaagaattg | 161372 | - | see also 9313 line |
| 33326 | ZFYVE19 | NM_032850.3 | 196 | gtcgctggagttagattaccaca | 161370 | - | see also 9313 line |
| 33327 | ZFYVE20 | NM_022340.1 | 980 | agctgatagacgctttaagtaag | 161401 | - | see also 8332 line |
| 33328 | ZFYVE20 | NM_022340.1 | 979 | gagctgatagacgctttaagtaa | 161400 | - | see also 8332 line |
| 33329 | ZFYVE21 | NM_024071.2 | 445 | ttgtcgtctttccaataaccaga | 161418 | - | see also 14629 line |
| 33330 | ZFYVE21 | NM_024071.2 | 491 | agccactatgaaatcgaaattgt | 161422 | - | see also 14629 line |
| 33331 | ZFYVE26 | NM_015346.2 | 3118 | gccgaacggcgtttgaatagtag | 161461 | - | see also 14630 line |
| 33332 | ZFYVE26 | NM_015346.2 | 3114 | gacagccgaacggcgtttgaata | 161459 | - | see also 14630 line |
| 33333 | ZNF185 | NM_007150.1 | 45 | aacgccaggcaccctacaatatc | 161524 | - | see also 14631 line |
| 33334 | ZNF185 | NM_007150.1 | 1235 | atccgagactgtccaaagattac | 161536 | - | see also 14631 line |
| 33335 | ZNF198 | NM_003453.2 | 914 | gacgggcgagatatgaacttaat | 161553 | - | see also 2319 line |
| 33336 | ZNF505 | NM_031218.2 | 506 | gacttaaccagtgtagtacaact | 161659 | - | see also 14633 line |
| 33337 | ZNF505 | NM_031218.2 | 325 | gccaacccctcagttatatgttc | 161656 | - | see also 14633 line |
| 33338 | AGMAT | NM_024758.3 | 451 | tgcttgggacagtcaatcctagc | 161661 | - | see also 14634 line |
| 33339 | AGMAT | NM_024758.3 | 516 | ggcgatgtgaatgtcaatcttta | 161663 | - | see also 14634 line |
| 33340 | ARG1 | NM_000045.2 | 159 | ctggtctgcttgagaaaacttaaa | 161678 | - | see also 14635 line |
| 33341 | ARG1 | NM_000045.2 | 356 | gaccacagtttggcaattggaag | 161680 | - | see also 14635 line |
| 33342 | ARG2 | NM_001172.2 | 400 | gcctggcaatcggtaccattagt | 161705 | - | see also 14636 line |
| 33343 | ARG2 | NM_001172.2 | 170 | tggtcccgctgccataagagaag | 161699 | - | see also 14636 line |
| 33344 | B3GAT1 | NM_018644.2 | 351 | acccctgctcgcggtacataagg | 161722 | - | see also 14637 line |

Figure 46

| | | | | | | | | |
|---|---|---|---|---|---|---|---|---|
| 33345 | B3GAT1 | NM_018644.2 | 350 | caccctgctcgcggtaacataag | | 161721 | - | see also 14637 line |
| 33346 | B3GAT2 | NM_080742.1 | 38 | tgccctggatcctaattgtcatc | | 161731 | - | see also 14638 line |
| 33347 | B3GAT2 | NM_080742.1 | 39 | gccctggatcctaattgtcatca | | 161732 | - | see also 14638 line |
| 33348 | B3GAT2 | NM_080742.1 | 721 | gccatcgacatggcaggatttgc | | 161734 | - | see also 14638 line |
| 33349 | B3GAT3 | NM_012200.2 | 250 | tgcctactatctatgttgttacc | | 161748 | - | see also 5189 line |
| 33350 | B3GAT3 | NM_012200.2 | 584 | gacccaagagtcgtctactttg | | 161749 | - | see also 5189 line |
| 33351 | B4GALT6 | NM_004775.2 | 455 | tacgctcaacgtacagattatc | | 161768 | - | see also 14639 line |
| 33352 | B4GALT6 | NM_004775.2 | 729 | ttccgtaatcgccatgaacatct | | 161781 | - | see also 14639 line |
| 33353 | B4GALT6 | NM_004775.2 | 376 | tacaagctcgaggtaatgttg | | 161765 | - | see also 14639 line |
| 33354 | CDIPT | NM_006319.2 | 716 | atccggtgctcggatctactac | | 161805 | - | see also 4421 line |
| 33355 | CDIPT | NM_006319.2 | 717 | tccggtgctcggatctactaca | | 161806 | - | see also 4421 line |
| 33356 | DCD | NM_053283.1 | 370 | gacgtccttgactcagtactata | | 161814 | - | see also 14641 line |
| 33357 | DCD | NM_053283.1 | 351 | agccgtccatgacgttaaagacg | | 161813 | - | see also 14641 line |
| 33358 | DKFZP566E144 | NM_015523.1 | 307 | aagtcctaacctgattattaaaa | | 161820 | - | see also 6447 line |
| 33359 | DKFZP566E144 | NM_015523.1 | 308 | aggtcctaacctgattattaaac | | 161821 | - | see also 6447 line |
| 33360 | GALNT1 | NM_020474.2 | 1491 | aacttaatggccagttacaatg | | 161860 | - | see also 7884 line |
| 33361 | GALNT1 | NM_020474.2 | 1345 | aacgaatcagtgtcagataaaca | | 161855 | - | see also 7884 line |
| 33362 | NPEPL1 | NM_024663.2 | 1021 | tgcgacaagccagatgacatcc | | 161868 | - | see also 14644 line |
| 33363 | NPEPL1 | NM_024663.2 | 949 | cgcaatcaagcaggtttcaaag | | 161867 | - | see also 14644 line |
| 33364 | PDE8A | NM_002605.1 | 2320 | accggacccaatcaaacgaatg | | 161925 | - | see also 1685 line |
| 33365 | PDE8A | NM_002605.1 | 989 | ggctgacttgctcgatactataa | | 161897 | - | see also 1685 line |
| 33366 | PDE8B | NM_003719.1 | 72 | ctgcagagcgcggtgatctact | | 161932 | - | see also 2547 line |
| 33367 | PDE8B | NM_003719.1 | 1298 | ttgacgtgaatcgatatcatct | | 161963 | - | see also 2547 line |
| 33368 | PDE9A | NM_002606.1 | 275 | aacgcactccgtacaaagtgaga | | 161995 | - | see also 14647 line |
| 33369 | PDE9A | NM_002606.1 | 1453 | aacgaggtcgtccaatggaagt | | 162012 | - | see also 14647 line |
| 33370 | PEPD | NM_000285.1 | 839 | ttcgacatgggcggtgagtatta | | 162021 | - | see also 248 line |
| 33371 | PEPD | NM_000285.1 | 840 | tcgacatgggcggtgagtattac | | 162022 | - | see also 248 line |
| 33372 | POFUT1 | NM_015352.1 | 493 | tggcccattctggatcagttc | | 162028 | - | see also 14648 line |
| 33373 | POFUT1 | NM_015352.1 | 524 | ttcaacagtcggagcttttac | | 162030 | - | see also 14648 line |
| 33374 | PPM1A | NM_021003.2 | 882 | tgcaggtggctcgtaatgattc | | 162060 | - | see also 14649 line |
| 33375 | PPM1A | NM_021003.2 | 497 | gacttgaatcgtgtcattcttt | | 162049 | - | see also 14649 line |
| 33376 | PPM1B | NM_002706.3 | 1419 | cccatrgtcatcgcaatctgtct | | 162106 | - | see also 14650 line |
| 33377 | PPM1B | NM_002706.3 | 1264 | tcgagataacatgagtattgtac | | 162103 | - | see also 14650 line |
| 33378 | PPM1D | NM_003620.2 | 692 | tggcggaatggccaaagaactatg | | 162121 | - | see also 14651 line |
| 33379 | PPM1D | NM_003620.2 | 286 | gaggacgttactcaaatcgttgt | | 162115 | - | see also 14651 line |

Figure 46

| | | | | | | | |
|---|---|---|---|---|---|---|---|
| 33380 | PPM1F | NM_014634.2 | 1025 | aagcattgggtggctttgtgtct | 162154 | - | see also 14652 line |
| 33381 | PPM1F | NM_014634.2 | 685 | cgcgcctactttgctgtgtttga | 162153 | - | see also 14652 line |
| 33382 | PPM1G | NM_002707.3 | 1222 | tagaactagcacgcatcaagaat | 162166 | - | see also 14653 line |
| 33383 | PPM1G | NM_002707.3 | 549 | gaggctaccatgactattgaaga | 162160 | - | see also 14653 line |
| 33384 | PPP1CA | NM_002708.2 | 239 | ctcaagatctgcgtgacataca | 162170 | - | see also 14654 line |
| 33385 | PPP1CA | NM_002708.2 | 272 | tacgaccttctgcgactatttga | 162171 | - | see also 14654 line |
| 33386 | PPP1CA | NM_002708.2 | 720 | acgaccgtgcgtctctttacc | 162180 | - | see also 14654 line |
| 33387 | PPP1CB | NM_002709.1 | 961 | ctgaatcgtcatgatttagattt | 162202 | - | see also 1763 line |
| 33388 | PPP1CC | NM_002710.1 | 1084 | aacgcctccaaggggtatgatca | 162234 | - | see also 14656 line |
| 33389 | PPP1CC | NM_002710.1 | 1086 | cgcctccaaggggtatgatcaca | 162235 | - | see also 14656 line |
| 33390 | PPP2CA | NM_002715.1 | 1036 | tggaacttgacgatactctaaaa | 162262 | - | see also 1767 line |
| 33391 | PPP2CA | NM_002715.1 | 966 | gaccggaatgtagtaacgatttt | 162253 | - | see also 1767 line |
| 33392 | PPP2CA | NM_002715.1 | 717 | ctctcgccatctatagatacact | 162246 | - | see also 1767 line |
| 33393 | PPP2CB | NM_004156.1 | 371 | atcacgaaagccgacaaattacc | 162273 | - | see also 14658 line |
| 33394 | PPP2CB | NM_004156.1 | 245 | caccggatacaaactacttattc | 162266 | - | see also 14658 line |
| 33395 | PPP4C | NM_002720.1 | 733 | ccgaggagccggctaacctattt | 162292 | - | see also 14659 line |
| 33396 | PPP4C | NM_002720.1 | 651 | aagcaagaggtgcctcatgatgg | 162291 | - | see also 14659 line |
| 33397 | PPP5C | NM_006247.2 | 840 | ctcggagaccaacccctatatat | 162302 | - | see also 14660 line |
| 33398 | PPP5C | NM_006247.2 | 736 | ctcgttggaaaccaactcaaaga | 162299 | - | see also 14660 line |
| 33399 | PPP6C | NM_002721.3 | 142 | agcgctatgtgactacgtttgt | 162308 | - | see also 1768 line |
| 33400 | PPP6C | NM_002721.3 | 962 | aacgacaagccatatttcctt | 162333 | - | see also 1768 line |
| 33401 | SOD2 | NM_000636.1 | 283 | tggtcatatcaatcatagcattt | 162337 | - | see also 421 line |
| 33402 | ABP1 | NM_001091.1 | 1286 | tgccgatgaccggtccattatc | 162360 | - | see also 14662 line |
| 33403 | ABP1 | NM_001091.1 | 1287 | gccgatgaccggtccattatcc | 162361 | - | see also 14662 line |
| 33404 | AOC2 | NM_001158.2 | 1482 | tccatgccacgggttatatcaac | 162388 | - | see also 14663 line |
| 33405 | AOC2 | NM_001158.2 | 809 | ttctaccttgggcactactatgc | 162369 | - | see also 14663 line |
| 33406 | AOC2 | NM_001158.2 | 1244 | atggtggacatccatatattagt | 162380 | - | see also 14663 line |
| 33407 | AOC3 | NM_003734.2 | 1563 | tgctcaactatgactatgtgtgg | 162401 | - | see also 14664 line |
| 33408 | AOC3 | NM_003734.2 | 937 | gactatccagaaggrgttctatc | 162400 | - | see also 14664 line |
| 33409 | ATOX1 | NM_004045.2 | 379 | ctgcttgcaacctgaagaaaac | 162407 | - | see also 14665 line |
| 33410 | ATOX1 | NM_004045.2 | 285 | ctctcgggtcctcaataagcttg | 162406 | - | see also 14665 line |
| 33411 | CCS | NM_005125.1 | 438 | atcagtacggggaccttacaaac | 162411 | - | see also 14666 line |
| 33412 | CCS | NM_005125.1 | 625 | tggccgagcctgattattgatg | 162412 | - | see also 14666 line |
| 33413 | CP | NM_000096.1 | 1920 | ttcggtcgtggtacttattca | 162491 | - | see also 14667 line |
| 33414 | CP | NM_000096.1 | 1427 | agccgattgggtgagattcaat | 162476 | - | see also 14667 line |
| 33415 | DBH | NM_000787.2 | 732 | ctctcggcaccacattatcaagt | 162511 | - | see also 487 line |
| 33416 | DBH | NM_000787.2 | 1047 | ctcaggccatccgctgtactaca | 162517 | - | see also 487 line |

Figure 46

| | | | | | | | |
|---|---|---|---|---|---|---|---|
| 33417 | DCT | NM_001922.2 | 1783 | atcgatctgccagtttcagttga | 162554 | - | see also 1222 line |
| 33418 | DCT | NM_001922.2 | 1216 | gacgatccgactctgattagtcg | 162537 | - | see also 1222 line |
| 33419 | F5 | NM_000130.2 | 6228 | ctccaactcgagcctataacaga | 162703 | - | see also 116 line |
| 33420 | F5 | NM_000130.2 | 6389 | cccttccgtgcccgtctgaatgc | 162707 | - | see also 116 line |
| 33421 | F8 | NM_000132.2 | 7134 | gactcgctaccttcgaattcacc | 162916 | - | see also 117 line |
| 33422 | F8 | NM_000132.2 | 7115 | ctctagacccaccgttactgact | 162914 | - | see also 117 line |
| 33423 | FLJ12892 | NM_022757.3 | 5890 | aggtgactcgactaagagaatta | 162961 | - | see also 14672 line |
| 33424 | FLJ12892 | NM_022757.3 | 5983 | ctgggaataaccttaccaaatca | 162968 | - | see also 14672 line |
| 33425 | HEPH | NM_014799.2 | 2553 | atggagtgctagaatctactact | 163018 | - | see also 14673 line |
| 33426 | HEPH | NM_014799.2 | 2229 | ctgcaagaatctactatatcatg | 163008 | - | see also 14673 line |
| 33427 | HYAL4 | NM_012269.1 | 2063 | aagttatcgaagcattcagttgt | 163072 | - | see also 14674 line |
| 33428 | HYAL4 | NM_012269.1 | 930 | tactatccgtggtatacatcaca | 163049 | - | see also 14674 line |
| 33429 | LOX | NM_002317.3 | 804 | ctctgacgacaacccttattaca | 163076 | - | see also 1490 line |
| 33430 | LOX | NM_002317.3 | 809 | acgacaacccttattacaactac | 163078 | - | see also 1490 line |
| 33431 | LOXL1 | NM_005576.1 | 1956 | aacgtggtgagatgcaacattca | 163097 | - | see also 14676 line |
| 33432 | LOXL1 | NM_005576.1 | 1911 | cacgtgaacccaaagtatattgt | 163095 | - | see also 14676 line |
| 33433 | LOXL2 | NM_002318.1 | 2529 | agcacttcagcgggctcttaaac | 163121 | - | see also 14677 line |
| 33434 | LOXL2 | NM_002318.1 | 359 | cagcaaccggctcctgagtatca | 163099 | - | see also 14677 line |
| 33435 | LOXL3 | NM_032603.2 | 1353 | ggggcgagtcgaggtgcaaatag | 163131 | - | see also 9258 line |
| 33436 | LOXL3 | NM_032603.2 | 568 | gtggaggaggtgcgaattcgacc | 163127 | - | see also 9258 line |
| 33437 | LOXL4 | NM_032211.5 | 1375 | caggtgcaatgtccctaacatgg | 163146 | - | see also 14678 line |
| 33438 | LOXL4 | NM_032211.5 | 2297 | cagtgccgctgcaagtatgatgg | 163152 | - | see also 14678 line |
| 33439 | MASS1 | NM_032119.1 | 15764 | ttgggccatccattgtttatatt | 163650 | - | see also 14679 line |
| 33440 | MASS1 | NM_032119.1 | 13823 | cagtgagcgggttgttctatttt | 163582 | - | see also 14679 line |
| 33441 | MOXD1 | NM_015529.1 | 909 | tccattagatccgcattatgtgc | 163745 | - | see also 6452 line |
| 33442 | MOXD1 | NM_015529.1 | 1352 | gtcgctacaacacgaaagataga | 163755 | - | see also 6452 line |
| 33443 | PAM | NM_000919.2 | 579 | aacagtccgatacatacttctgc | 163773 | - | see also 595 line |
| 33444 | PAM | NM_000919.2 | 573 | cacctaaacagtccgatacatac | 163771 | - | see also 595 line |
| 33445 | PAM | NM_000919.2 | 2807 | ttggaacatcgatcagttaaaaa | 163830 | - | see also 595 line |
| 33446 | SCO2 | NM_005138.1 | 679 | tgcccaggctagtcacagttacc | 163841 | - | see also 14682 line |
| 33447 | SCO2 | NM_005138.1 | 787 | cggcctcttcacggattactacg | 163844 | - | see also 14682 line |
| 33448 | SLC14A2 | NM_007163.2 | 909 | ctcgacatccccggatactcacc | 163845 | - | see also 4965 line |
| 33449 | SLC14A2 | NM_007163.2 | 3247 | aacagcaccctcgcatgcatagc | 163862 | - | see also 4965 line |
| 33450 | SLC15A4 | NM_145648.1 | 1217 | ctggtcgatcccattttgagaag | 163890 | - | see also 9791 line |
| 33451 | SLC15A4 | NM_145648.1 | 751 | ctgcgtcggccttgcttttgtgg | 163874 | - | see also 9791 line |
| 33452 | SLC15A4 | NM_145648.1 | 749 | gtctgcgtcggccttgcttttgt | 163873 | - | see also 9791 line |
| 33453 | SLC35B2 | NM_178148.1 | 721 | gccaatacgaagctcttaagttc | 163903 | - | see also 14685 line |

Figure 46

| 33454 | SLC35B2 | NM_178148.1 | 258 | ggctatgccagctttatggtacc | 163900 | - | see also 14685 line |
| 33455 | TRRAP | NM_003496.1 | 10189 | gagcggtgtccgatgctaaaatc | 164099 | - | see also 14686 line |
| 33456 | TRRAP | NM_003496.1 | 10190 | agcggtgtccgatgctaaaatca | 164100 | - | see also 14686 line |
| 33457 | TRRAP | NM_003496.1 | 5816 | ggcgcacattatcgccaaattcg | 164024 | - | see also 14686 line |
| 33458 | FTHL17 | NM_031894.1 | 273 | tgcgcctgtcggacgacaaaatg | 164128 | - | - | ferric iron binding | - |
| 33459 | FTHL17 | NM_031894.1 | 196 | ctcctacctgtacctgtctatgg | 164127 | - | see also 33458 line |
| 33460 | LTF | NM_002343.1 | 744 | gacacttcgtccattcttgaatt | 164135 | - | see also 14687 line |
| 33461 | LTF | NM_002343.1 | 611 | gacagccacgaactcactattat | 164133 | - | see also 14687 line |
| 33462 | LTF | NM_002343.1 | 393 | acccgaggccacaaaatgcttcc | 164130 | - | see also 14687 line |
| 33463 | MFI2 | NM_005929.3 | 345 | gccggtggtgggcgaagtgtacg | 164150 | - | see also 14688 line |
| 33464 | MFI2 | NM_005929.3 | 454 | tcctgccacacgggcatcaatcg | 164153 | - | see also 14688 line |
| 33465 | MTF | NM_177478.1 | 204 | ctctcgggtgcgccagaacttcc | 164173 | - | see also 14689 line |
| 33466 | MTF | NM_177478.1 | 683 | tggcggagtacctttttgacaca | 164180 | - | see also 14689 line |
| 33467 | RSHL1 | NM_030785.1 | 1399 | gccgctcaggcgccaacaagtac | 164184 | - | see also 14690 line |
| 33468 | RSHL1 | NM_030785.1 | 625 | cccagcacgggccttacataagg | 164183 | - | see also 14690 line |
| 33469 | RSHL1 | NM_030785.1 | 624 | gcccagcacgggccttacataag | 164182 | - | see also 14690 line |
| 33470 | TF | NM_001063.2 | 1893 | tgcgtccacaagatattacgtca | 164232 | - | see also 14691 line |
| 33471 | TF | NM_001063.2 | 724 | agcactcgactatatttgagaac | 164208 | - | see also 14691 line |
| 33472 | TF | NM_001063.2 | 854 | taccgtcgtggcccgaagtatgg | 164214 | - | see also 14691 line |
| 33473 | FECH | NM_000140.1 | 32 | gcgttcactcggcgcaaacatgg | 164238 | - | see also 126 line |
| 33474 | FECH | NM_000140.1 | 1018 | tcctcttggttccgatagcattt | 164270 | - | see also 126 line |
| 33475 | ACO2 | NM_001098.1 | 1150 | tccgagtgggtctaattggtagc | 164293 | - | see also 14693 line |
| 33476 | ACO2 | NM_001098.1 | 1149 | atccgagtgggtctaattggtag | 164292 | - | see also 14693 line |
| 33477 | ACO2 | NM_001098.1 | 1146 | gacatccgagtgggtctaattgg | 164291 | - | see also 14693 line |
| 33478 | ADHFE1 | NM_144650.1 | 237 | ctgtgcaagtagctatggattcc | 164301 | - | see also 14694 line |
| 33479 | ADHFE1 | NM_144650.1 | 830 | ctgcggatcgtggctaagtatct | 164313 | - | see also 14694 line |
| 33480 | ALOX12 | NM_000697.1 | 1477 | ttctaccaaagggatgacatagt | 164345 | - | see also 14695 line |
| 33481 | ALOX12 | NM_000697.1 | 1950 | agcccggaatgagcaacttgact | 164352 | - | see also 14695 line |
| 33482 | ALOX12B | NM_001139.1 | 1950 | tccgatatgtcactatagtcatc | 164370 | - | see also 726 line |
| 33483 | ALOX12B | NM_001139.1 | 2055 | tgcggaatccaccgattcagact | 164372 | - | see also 726 line |
| 33484 | ALOX12B | NM_001139.1 | 529 | tgcccccaacggccgtatctacc | 164355 | - | see also 726 line |
| 33485 | ALOX15 | NM_001140.3 | 1151 | gccgtcgatacatcctatcttca | 164381 | - | see also 14697 line |
| 33486 | ALOX15 | NM_001140.3 | 1191 | ctgcgatacaccctggaaattaa | 164384 | - | see also 14697 line |
| 33487 | ALOX15B | NM_001141.1 | 1007 | cccaatgaccctgctataccaga | 164393 | - | see also 14698 line |
| 33488 | ALOX15B | NM_001141.1 | 1479 | ggctactactaccgtgatgatgg | 164395 | - | see also 14698 line |
| 33489 | ALOX5 | NM_000698.1 | 429 | aagcaacaccgacgtaaagaact | 164403 | - | see also 14699 line |
| 33490 | ALOX5 | NM_000698.1 | 538 | cccgtgatatccagtttgatagt | 164405 | - | see also 14699 line |

Figure 46

| 33491 | ALOXE3 | NM_021628.1 | 767 | accgaaccggatggtagtgtatc | 164435 | - | see also 14700 line | | |
|---|---|---|---|---|---|---|---|---|---|
| 33492 | ALOXE3 | NM_021628.1 | 637 | ccctggatcggtacagaagtaca | 164432 | - | see also 14700 line | | |
| 33493 | FDX1 | NM_004109.3 | 469 | accgtgatggtgaaacattaaca | 164450 | - | - | Smad3/Sp-1 heterodimer | - |
| 33494 | FDX1 | NM_004109.3 | 770 | tgccagacaatccattgatgtgg | 164451 | - | see also 33493 line | | |
| 33495 | NIFU | NM_014301.1 | 363 | aagattgtggatgctaggtttaa | 164458 | - | see also 14701 line | | |
| 33496 | NIFU | NM_014301.1 | 317 | atgtggtgacgtaatgaaattac | 164456 | - | see also 14701 line | | |
| 33497 | PAH | NM_000277.1 | 1334 | ttgggacatgtgcccttgttttc | 164472 | - | see also 14702 line | | |
| 33498 | PAH | NM_000277.1 | 1670 | aggaactttgctgccacaatacc | 164479 | - | see also 14702 line | | |
| 33499 | SCD | NM_005063.3 | 775 | tgcccctacggctctttctgatc | 164482 | - | - | Sterol regulatory element binding protein-1a | - |
| 33500 | SCD4 | NM_024906.1 | 717 | ttcgcaagcatcgagatgttatt | 164485 | - | see also 14703 line | | |
| 33501 | SCD4 | NM_024906.1 | 871 | gtggcatcaagggcatatattga | 164490 | - | see also 14703 line | | |
| 33502 | TH | NM_000360.2 | 803 | agcggctaccgggaagacaatat | 164499 | - | see also 14704 line | | |
| 33503 | TH | NM_000360.2 | 802 | cagcggctaccgggaagacaata | 164498 | - | see also 14704 line | | |
| 33504 | TPH1 | NM_004179.1 | 421 | taccgtaaacgtcgaaagtattt | 164514 | - | see also 2805 line | | |
| 33505 | TPH1 | NM_004179.1 | 422 | accgtaaacgtcgaaagtatttt | 164515 | - | see also 2805 line | | |
| 33506 | TPH1 | NM_004179.1 | 412 | gacaatgtctaccgtaaacgtcg | 164513 | - | see also 2805 line | | |
| 33507 | TPH2 | NM_173353.2 | 1113 | tccactacttgcggatcctaagt | 164560 | - | see also 10062 line | | |
| 33508 | TPH2 | NM_173353.2 | 430 | aggaaatctcggcgaagaagttc | 164542 | - | see also 10062 line | | |
| 33509 | AOX1 | NM_001159.2 | 1670 | gagttatgcatctcatatggagg | 164610 | - | see also 14707 line | | |
| 33510 | AOX1 | NM_001159.2 | 1607 | gcgctagcgatagtcaattcagg | 164607 | - | see also 14707 line | | |
| 33511 | SUOX | NM_000456.1 | 1167 | gtcccatgtgcgtgagttactgg | 164678 | - | see also 14708 line | | |
| 33512 | SUOX | NM_000456.1 | 965 | ctcaggagtcaacacacatatac | 164676 | - | see also 14708 line | | |
| 33513 | XDH | NM_000379.2 | 3274 | agcgagacaagcactaacactgt | 164741 | - | see also 302 line | | |
| 33514 | XDH | NM_000379.2 | 435 | tggcatcgtcatgagtatgtaca | 164691 | - | see also 302 line | | |
| 33515 | AFP | NM_001134.1 | 969 | ctggaacgtggtcaatgtataat | 164774 | - | see also 14710 line | | |
| 33516 | AFP | NM_001134.1 | 967 | cgctggaacgtggtcaatgtata | 164773 | - | see also 14710 line | | |
| 33517 | AFP | NM_001134.1 | 1469 | gggagcggctgacattattatcg | 164795 | - | see also 14710 line | | |
| 33518 | ADCY2 | NM_020546.1 | 3231 | acgtgcacctgtcgaggaataat | 164873 | - | see also 7895 line | | |
| 33519 | ADCY2 | NM_020546.1 | 2322 | gtgttcctgcgggtaaactatga | 164861 | - | see also 7895 line | | |
| 33520 | ADCY3 | NM_004036.2 | 2567 | ttcgggagcacgacttacctatg | 164897 | - | see also 14712 line | | |
| 33521 | ADCY3 | NM_004036.2 | 2531 | ggcgtcccgtctttgatgaatac | 164894 | - | see also 14712 line | | |
| 33522 | ADCY4 | NM_139247.2 | 2424 | ggctcgccagaatgagtactact | 164934 | - | see also 14713 line | | |
| 33523 | ADCY4 | NM_139247.2 | 1835 | cagctctggccatcacgtatagc | 164930 | - | see also 14713 line | | |
| 33524 | ADCY5 | NM_183357.1 | 2643 | ggcggagtcggccgtcaactaca | 164951 | - | see also 14714 line | | |

Figure 46

| | | | | | | | | | |
|---|---|---|---|---|---|---|---|---|---|
| 33525 | ADCY5 | NM_183357.1 | 2886 | caccgccaacgccatagacttct | 164952 | - | see also 14714 line | | |
| 33526 | ADCY6 | NM_015270.2 | 2588 | atcgatgcccgcagcattgatca | 164961 | - | see also 6300 line | | |
| 33527 | ADCY6 | NM_015270.2 | 3986 | ggggctcggaagccacagtatga | 164971 | - | see also 6300 line | | |
| 33528 | ADCY7 | NM_001114.1 | 2217 | gggggacgctctgcactatctct | 164974 | - | see also 697 line | | |
| 33529 | ADCY7 | NM_001114.1 | 2912 | gcctccgtgccggacttcaaagt | 164981 | - | see also 697 line | | |
| 33530 | ADCY8 | NM_001115.1 | 5389 | ggcgttatcggcgctaagaaacc | 165043 | - | see also 698 line | | |
| 33531 | ADCY8 | NM_001115.1 | 2519 | ttcttaatgggggctatagctac | 164993 | - | see also 698 line | | |
| 33532 | ADCY9 | NM_001116.1 | 2728 | tgccgtcgtgcactactgtaact | 165088 | - | see also 704 line | | |
| 33533 | ADCY9 | NM_001116.1 | 1905 | gcccttcgtgcggaatcacattt | 165075 | - | see also 704 line | | |
| 33534 | ADCY9 | NM_001116.1 | 2891 | gagaggaacccgtgcaatagttc | 165097 | - | see also 704 line | | |
| 33535 | ADK | NM_001123.2 | 580 | caggtccctcatagctaatcttg | 165113 | - | see also 712 line | | |
| 33536 | ADK | NM_001123.2 | 985 | agggagagatgacactataatgg | 165128 | - | see also 712 line | | |
| 33537 | ADPRH | NM_001125.2 | 1174 | tgcccaccgagcctttttccatg | 165143 | - | see also 14720 line | | |
| 33538 | ADPRH | NM_001125.2 | 1271 | ccctccaactatgagaaactaga | 165149 | - | see also 14720 line | | |
| 33539 | ALPI | NM_001631.2 | 910 | gagcccggagacacgaaatatga | 165153 | - | see also 14721 line | | |
| 33540 | ALPI | NM_001631.2 | 912 | gcccggagacacgaaatatgaga | 165154 | - | see also 14721 line | | |
| 33541 | ALPL | NM_000478.2 | 247 | atgatttcaccattcttagtact | 165158 | - | see also 14722 line | | |
| 33542 | ALPL | NM_000478.2 | 342 | agcgcaagagacactgaaatatg | 165161 | - | see also 14722 line | | |
| 33543 | B3GALT3 | NM_003781.2 | 907 | gccccaatgccaagtacgtaatg | 165189 | - | see also 2586 line | | |
| 33544 | B3GALT3 | NM_003781.2 | 1010 | cacaggttatcctctaattgata | 165194 | - | see also 2586 line | | |
| 33545 | CDS1 | NM_001263.2 | 1024 | ttcgctaccatagatttatatca | 165222 | - | see also 827 line | | |
| 33546 | CDS1 | NM_001263.2 | 1018 | tcctcattcgctaccatagattt | 165221 | - | see also 827 line | | |
| 33547 | CERK | NM_022766.4 | 1540 | agcgctttgggcacatttgcagc | 165274 | - | see also 14725 line | | |
| 33548 | CLDN16 | NM_006580.2 | 417 | tggtgggaatgcgtcacacaatgc | 165294 | - | see also 14726 line | | |
| 33549 | CLDN16 | NM_006580.2 | 596 | ccctgatgagccgtacattaaag | 165303 | - | see also 14726 line | | |
| 33550 | COMT | NM_000754.2 | 918 | ttgagtgcacacactaccaatcg | 165317 | - | see also 14727 line | | |
| 33551 | COMT | NM_000754.2 | 700 | tggagcgtcccaggacatcatcc | 165316 | - | see also 14727 line | | |
| 33552 | DKFZp761G058 | NM_152542.2 | 825 | agccctattgcgagatggtattg | 165326 | | see also 14728 line | | |
| 33553 | DKFZp761G058 | NM_152542.2 | 826 | gccctattgcgagatggtattga | 165327 | - | see also 14728 line | | |
| 33554 | DUT | NM_001948.2 | 419 | tgcgaacggattttttatccaga | 165338 | - | phosphatase | pseudouridylate synthase | - |
| 33555 | DUT | NM_001948.2 | 424 | acggattttttatccagaaatag | 165339 | - | see also 33554 line | | |
| 33556 | ENO2 | NM_001975.2 | 1186 | gtgaccaacccaaaacgtattga | 165355 | - | see also 1258 line | | |
| 33557 | ENO2 | NM_001975.2 | 336 | agcctctacgggcatctatgagg | 165340 | - | see also 1258 line | | |
| 33558 | ENO3 | NM_001976.2 | 1308 | ctcggagcgtctggccaaataca | 165371 | - | see also 14730 line | | |

Figure 46

| | | | | | | | | |
|---|---|---|---|---|---|---|---|---|
| 33559 | ENO3 | NM_001976.2 | 395 | agctagatgggaccgagaataag | 165361 | - | see also 14730 line | |
| 33560 | ENTPD1 | NM_001776.2 | 218 | aggagtctaacgtgaagacattt | 165372 | - | see also 1159 line | |
| 33561 | ENTPD1 | NM_001776.2 | 729 | tgcctatggctgattactatca | 165389 | - | see also 1159 line | |
| 33562 | ENTPD2 | NM_001246.1 | 171 | cacgtccatgtttattctacaagt | 165412 | - | - | magnesium binding |
| 33563 | ENTPD2 | NM_001246.1 | 636 | ctctaccagatcacttttgaga | 165413 | - | see also 33562 line | |
| 33564 | ENTPD2 | NM_001246.1 | 994 | ttctccgatgctcttcaatgg | 165414 | - | see also 33562 line | |
| 33565 | ENTPD3 | NM_001248.1 | 284 | accacagtctacgtgatcaatg | 165421 | - | see also 14732 line | |
| 33566 | ENTPD3 | NM_001248.1 | 241 | aggactgaagtatggtattgtgc | 165418 | - | see also 14732 line | |
| 33567 | ENTPD5 | NM_001249.1 | 754 | tccgacgaaggcatattagcttg | 165455 | - | see also 14733 line | |
| 33568 | ENTPD5 | NM_001249.1 | 755 | ccgacgaaggcatattagcttgg | 165456 | - | see also 14733 line | |
| 33569 | ENTPD6 | NM_001247.1 | 978 | gacggcactgcggatgtttaaca | 165476 | - | see also 14734 line | |
| 33570 | ENTPD6 | NM_001247.1 | 1502 | agtcactcggaaaattgacaat | 165482 | - | see also 14734 line | |
| 33571 | FACL2 | NM_001995.2 | 1624 | cccgatgccgtgcaatttgataa | 165523 | - | see also 14735 line | |
| 33572 | FACL2 | NM_001995.2 | 1290 | acggatgtttgaccgaattttc | 165518 | - | see also 14735 line | |
| 33573 | FACL2 | NM_001995.2 | 1623 | ccccgatgccgtgcaatttgata | 165522 | - | see also 14735 line | |
| 33574 | FACL3 | NM_004457.2 | 1510 | tgcaaccacgcagcggattcatga | 165582 | - | see also 14736 line | |
| 33575 | FACL3 | NM_004457.2 | 1516 | cacgcagccgattcatgaacatct | 165584 | - | see also 14736 line | |
| 33576 | FACL4 | NM_004458.1 | 1734 | aaccccagagtgaaatcgtaat | 165666 | - | see also 3001 line | |
| 33577 | FACL5 | NM_016234.2 | 1342 | tccttaacaggatctcacgataag | 165712 | - | see also 14737 line | |
| 33578 | FACL5 | NM_016234.2 | 1509 | ggcggaagggttcgtgtaattgt | 165717 | - | see also 14737 line | |
| 33579 | FACL6 | NM_015256.1 | 670 | atccgctacatcatcaatacagc | 165738 | - | see also 14738 line | |
| 33580 | FACL6 | NM_015256.1 | 662 | ctggggctatccgctacatcatc | 165735 | - | see also 14738 line | |
| 33581 | FDFT1 | NM_004462.2 | 466 | cccaagatctcccttgagtta | 165770 | - | see also 3012 line | |
| 33582 | FDFT1 | NM_004462.2 | 285 | tgcgcaacgcagtgtgcatattt | 165765 | - | see also 3012 line | |
| 33583 | FDFT1 | NM_004462.2 | 1184 | accatccggacgcagagaatcttcc | 165788 | - | see also 3012 line | |
| 33584 | FMO2 | NM_001460.1 | 1369 | gacgagctgccttagagatagg | 165821 | - | see also 14740 line | |
| 33585 | FMO2 | NM_001460.1 | 715 | tgggtcatgagccgtatcctga | 165803 | - | see also 14740 line | |
| 33586 | GRIN1 | NM_000832.4 | 3507 | ctgcgaccttactttgagaac | 165845 | - | see also 14741 line | |
| 33587 | GRIN1 | NM_000832.4 | 2661 | gcgcgcagtacatcgagttttcc | 165834 | - | see also 14741 line | |
| 33588 | GRIN2A | NM_000833.2 | 2498 | tacgatgccgcagtcttgaatta | 165889 | - | see also 544 line | |
| 33589 | GRIN2A | NM_000833.2 | 3812 | aaccggaacccttgcataatga | 165913 | - | see also 544 line | |
| 33590 | GRIN2A | NM_000833.2 | 3368 | tccataccgccagattcactac | 165907 | - | see also 544 line | |
| 33591 | GRIN2B | NM_000834.2 | 4050 | tacccctcagtcgccgactaattc | 165979 | - | see also 546 line | |
| 33592 | GRIN2B | NM_000834.2 | 3356 | cagtggccacgacgacttgatcc | 165973 | - | see also 546 line | |
| 33593 | GRIN2C | NM_000835.2 | 2254 | agcggaacatccgcagtaactac | 165997 | - | see also 14744 line | |
| 33594 | GRIN2C | NM_000835.2 | 453 | cacgtccacgcaatgtgtcttga | 165988 | - | see also 14744 line | |
| 33595 | GRIN2C | NM_000835.2 | 1310 | ctgggagcatggcgtcctataca | 165990 | - | see also 14744 line | |

Figure 46

| 33596 | GRIN2D | NM_000836.1 | 1872 | ctgtggtcgccgtcactgttttc | 166006 | - | see also 14745 line |
|-------|--------|-------------|------|--------------------------|--------|---|---------------------|
| 33597 | GRIN2D | NM_000836.1 | 1091 | ctgcgtgattatggtttccttcc | 166002 | - | see also 14745 line |
| 33598 | HPCL2 | NM_012260.1 | 294 | tgctgcctccgcgattggatatc | 166021 | - | see also 5241 line |
| 33599 | HPRT1 | NM_000194.1 | 100 | ccctggcgtcgtgattagtgatg | 166070 | - | see also 14747 line |
| 33600 | HPRT1 | NM_000194.1 | 226 | acgtcttgctcgagatgtgatga | 166074 | - | see also 14747 line |
| 33601 | IDI1 | NM_004508.2 | 538 | tacgtgttgtagtcatccattaa | 166098 | - | see also 14748 line |
| 33602 | IDI1 | NM_004508.2 | 863 | aagataacgccatggtttaaaat | 166109 | - | see also 14748 line |
| 33603 | IDI1 | NM_004508.2 | 464 | ttcaacaccgaaaataagcttct | 166095 | - | see also 14748 line |
| 33604 | IDI2 | NM_033261.2 | 551 | cccggatcccagtgaaacgaaaa | 166122 | - | see also 14749 line |
| 33605 | IDI2 | NM_033261.2 | 640 | aagtcaccccctggctaagaacc | 166127 | - | see also 14749 line |
| 33606 | ILKAP | NM_030768.2 | 832 | cggagatagtcgggcaatcttgt | 166140 | - | see also 8943 line |
| 33607 | ILKAP | NM_030768.2 | 843 | gggcaatcttgtgtcgttataat | 166143 | - | see also 8943 line |
| 33608 | IMPA1 | NM_005536.2 | 795 | ggcgtgctaatggatgttacagg | 166162 | - | see also 14751 line |
| 33609 | IMPA1 | NM_005536.2 | 713 | tggcggagcagatgcatattatg | 166156 | - | see also 14751 line |
| 33610 | ITGAE | NM_002208.3 | 3147 | tgcgtcccaaccaaattacgagg | 166195 | - | see also 14752 line |
| 33611 | ITGAE | NM_002208.3 | 1055 | caccgatggtggcatattcgagg | 166173 | - | see also 14752 line |
| 33612 | ITGAL | NM_002209.1 | 1002 | aacccgcgagcgagtttgtgaaa | 166213 | - | see also 14753 line |
| 33613 | ITGAL | NM_002209.1 | 1319 | ctgccctcccggcaaaagacttc | 166221 | - | see also 14753 line |
| 33614 | ITGAM | NM_000632.2 | 776 | tggtacgagagctgtttaacatc | 166255 | - | see also 14754 line |
| 33615 | ITGAM | NM_000632.2 | 773 | aagtggtacgagagctgtttaac | 166254 | - | see also 14754 line |
| 33616 | ITGAX | NM_000887.3 | 905 | ggcatcatccgctatgcaattgg | 166303 | - | see also 14755 line |
| 33617 | ITGAX | NM_000887.3 | 3434 | atcacagcggtactgtacaaagt | 166316 | - | see also 14755 line |
| 33618 | LYSAL1 | NM_004901.1 | 261 | tacgccaaattatggtcattagt | 166323 | - | see also 3404 line |
| 33619 | LYSAL1 | NM_004901.1 | 382 | ggcacgagttaccgacattgaag | 166328 | - | see also 3404 line |
| 33620 | LYSAL1 | NM_004901.1 | 253 | taccaatttacgccaaattatgg | 166322 | - | see also 3404 line |
| 33621 | MTHFD2 | NM_006636.2 | 1065 | agcgagaagtgctgaagtctaaa | 166372 | - | see also 14757 line |
| 33622 | MTHFD2 | NM_006636.2 | 869 | gtcattgatgtgggaataaatag | 166371 | - | see also 14757 line |
| 33623 | MTHFD2 | NM_006636.2 | 787 | tacaattcttgcagatattgtaa | 166370 | - | see also 14757 line |
| 33624 | MTHFS | NM_006441.1 | 520 | caccctggcgttggctttcaaag | 166380 | - | see also 14758 line |
| 33625 | MTHFS | NM_006441.1 | 278 | cacatggatatggtgagaataga | 166377 | - | see also 14758 line |
| 33626 | PFKL | NM_002626.2 | 975 | gcctagtgggctccatcgataac | 166387 | - | see also 14759 line |
| 33627 | PFKL | NM_002626.2 | 2113 | gggctccgacactgctgtaaatg | 166395 | - | see also 14759 line |
| 33628 | PFKM | NM_000289.3 | 219 | cacctgggagagcgtttcgatga | 166405 | - | see also 255 line |
| 33629 | PFKM | NM_000289.3 | 125 | tggttcgagttggtatcttcacc | 166402 | - | see also 255 line |
| 33630 | PFKP | NM_002627.2 | 1399 | caggatgctcgccatctatgatg | 166450 | - | see also 1698 line |
| 33631 | PGM1 | NM_002633.2 | 456 | cgggatcggtcgcttggttatcg | 166469 | - | see also 14762 line |
| 33632 | PGM1 | NM_002633.2 | 944 | ctgcgaactcggcagttaactgc | 166485 | - | see also 14762 line |

Figure 46

| 33633 | PKLR | NM_000298.3 | 1738 | atcatgcgggtgctaagcatatc | 166518 | - | see also 14763 line | | | |
|---|---|---|---|---|---|---|---|---|---|---|
| 33634 | PKLR | NM_000298.3 | 640 | tgggtggactaccccaatattgt | 166507 | - | see also 14763 line | | | |
| 33635 | PKM2 | NM_002654.3 | 565 | tgctgtggctctagacactaaag | 166519 | - | kinase_related | - | | breast cancer、lung cancer、cancer |
| 33636 | PP | NM_021129.2 | 240 | cacgctggtctaatgcaaaaatg | 166525 | - | see also 14764 line | | | |
| 33637 | PP | NM_021129.2 | 915 | taccaacagacgtggataagtgg | 166548 | - | see also 14764 line | | | |
| 33638 | PPA2 | NM_006903.3 | 620 | acctggaagctactcttaattgg | 166567 | - | see also 4841 line | | | |
| 33639 | PPA2 | NM_006903.3 | 775 | aagtgtaatggaggagctataaa | 166570 | - | see also 4841 line | | | |
| 33640 | PPAT | NM_002703.3 | 747 | cagcatactccctgcttataatg | 166597 | - | see also 14766 line | | | |
| 33641 | PPAT | NM_002703.3 | 805 | tggaaatcgtcccttatgcattg | 166602 | - | see also 14766 line | | | |
| 33642 | PPM1E | NM_014906.3 | 2136 | tacgccaccactactcaaagaag | 166652 | - | see also 14767 line | | | |
| 33643 | PPM1E | NM_014906.3 | 1755 | atgggcgtgtggattcattcact | 166640 | - | see also 14767 line | | | |
| 33644 | PRPS1 | NM_002764.2 | 279 | gtggcgaaatcaatgacaattta | 166661 | - | see also 14768 line | | | |
| 33645 | PRPS1 | NM_002764.2 | 280 | tggcgaaatcaatgacaatttaa | 166662 | - | see also 14768 line | | | |
| 33646 | PRPS1L1 | NM_175886.2 | 930 | ttgctccaaaatacgagtaattg | 166669 | - | see also 14769 line | | | |
| 33647 | PRPS1L1 | NM_175886.2 | 933 | ctccaaaatacgagtaattgaca | 166671 | - | see also 14769 line | | | |
| 33648 | PRPS1L1 | NM_175886.2 | 931 | tgctccaaaatacgagtaattga | 166670 | - | see also 14769 line | | | |
| 33649 | PRPS2 | NM_002765.2 | 361 | tcccatacgcccgacaagataaa | 166680 | - | see also 1808 line | | | |
| 33650 | PRPS2 | NM_002765.2 | 362 | cccatacgcccgacaagataaaa | 166681 | - | see also 1808 line | | | |
| 33651 | PRPS2 | NM_002765.2 | 360 | ttcccatacgcccgacaagataa | 166679 | - | see also 1808 line | | | |
| 33652 | PSPH | NM_004577.2 | 635 | aaggataacgccaaatggtatat | 166710 | - | see also 14771 line | | | |
| 33653 | PSPH | NM_004577.2 | 428 | ttctactttaacggtgaatatgc | 166707 | - | see also 14771 line | | | |
| 33654 | SMPD2 | NM_003080.1 | 742 | tggtgctcaacgcctatgtgacc | 166716 | - | see also 14772 line | | | |
| 33655 | SMPD2 | NM_003080.1 | 1249 | cagagaggtcgccgttgatgtgt | 166723 | - | see also 14772 line | | | |
| 33656 | BSN | NM_003458.1 | 3878 | gccgtgtacgaagaaatccttca | 166756 | - | see also 2345 line | | | |
| 33657 | BSN | NM_003458.1 | 9796 | tcctcgaaactacgtaatgattg | 166789 | - | see also 2345 line | | | |
| 33658 | BSN | NM_003458.1 | 9795 | ttcctcgaaactacgtaatgatt | 166788 | - | see also 2345 line | | | |
| 33659 | DLD | NM_000108.2 | 185 | tacgcagatcagccgattgatgc | 166798 | - | see also 14774 line | | | |
| 33660 | DLD | NM_000108.2 | 1021 | tggccgacgaccctttactaaga | 166836 | - | see also 14774 line | | | |
| 33661 | FLJ10511 | NM_018120.3 | 786 | tggtgcgaatccgttcagatttg | 166866 | - | see also 14775 line | | | |
| 33662 | FLJ10511 | NM_018120.3 | 274 | cgctctatcggtagttaaccagt | 166845 | - | see also 14775 line | | | |
| 33663 | GSR | NM_000637.1 | 757 | tacggcatgataaggtacttaga | 166886 | - | see also 423 line | | | |
| 33664 | GSR | NM_000637.1 | 753 | atgatacggcatgataaggtact | 166885 | - | see also 423 line | | | |
| 33665 | KIAA0467 | NM_015284.1 | 1886 | gagtccattcacgcctcaaaatg | 166934 | - | see also 6313 line | | | |
| 33666 | KIAA0467 | NM_015284.1 | 2800 | gccccacggcttcgattggatgt | 166953 | - | see also 6313 line | | | |
| 33667 | MT3 | NM_005954.1 | 130 | tgcaagtgcgagggatgcaaatg | 166986 | - | - | | heavy metal binding | Alzheimer disease |
| 33668 | PITPNM1 | NM_004910.1 | 346 | gggggcagcgggcaatacacaca | 166987 | - | see also 3412 line | | | |
| 33669 | PKD1 | NM_000296.2 | 3818 | ggcggatgtgcgcgtctttgagg | 167003 | - | see also 14779 line | | | |

Figure 46

| | | | | | | | | | | |
|---|---|---|---|---|---|---|---|---|---|---|
| 33670 | PKD1 | NM_000296.2 | 4235 | cccgacggtgacacacaacttca | 167005 | - | see also 14779 line | | | |
| 33671 | SOD1 | NM_000454.3 | 412 | cacactggtggtccatgaaaaag | 167036 | - | see also 14780 line | | | |
| 33672 | SOD1 | NM_000454.3 | 155 | aaggtgtggggaagcattaaagg | 167032 | - | see also 14780 line | | | |
| 33673 | TXNRD1 | NM_003330.2 | 1777 | gacgattccgtcaagagataaca | 167072 | - | see also 14781 line | | | |
| 33674 | TXNRD1 | NM_003330.2 | 1180 | tggtttagacgtcactgttatgg | 167054 | - | see also 14781 line | | | |
| 33675 | C5 | NM_001735.2 | 2167 | cagcgagctgcacggattagttt | 167149 | - | see also 1149 line | | | |
| 33676 | C5 | NM_001735.2 | 4449 | ttgtgtacgattccggatatttg | 167229 | - | see also 1149 line | | | |
| 33677 | CCL1 | NM_002981.1 | 177 | ctcatttgcggagcaagagattc | 167254 | - | signal_transduction | chemokine | - | |
| 33678 | CCL1 | NM_002981.1 | 241 | tgctccaatgagggcttaatatt | 167255 | - | see also 33677 line | | | |
| 33679 | CCL1 | NM_002981.1 | 243 | ctccaatgagggcttaatattca | 167256 | - | see also 33677 line | | | |
| 33680 | CCL15 | NM_004167.3 | 663 | gtgttcactcatgaaaagttatt | 167262 | - | see also 14784 line | | | |
| 33681 | CCL15 | NM_004167.3 | 661 | ccgtgttcactcatgaaaagtta | 167261 | - | see also 14784 line | | | |
| 33682 | CCL16 | NM_004590.2 | 365 | aacttgtccacggttaaaattat | 167270 | - | see also 14785 line | | | |
| 33683 | CCL16 | NM_004590.2 | 368 | ttgtccacggttaaaattattac | 167271 | - | see also 14785 line | | | |
| 33684 | CCL16 | NM_004590.2 | 362 | aggaacttgtccacggttaaaat | 167269 | - | see also 14785 line | | | |
| 33685 | CCL17 | NM_002987.2 | 301 | tccagggatgccatcgtttttgt | 167274 | - | signal_transduction、g pcr | chemokine | - | |
| 33686 | CCL17 | NM_002987.2 | 300 | ctccagggatgccatcgtttttg | 167273 | - | see also 33685 line | | | |
| 33687 | CCL18 | NM_002988.2 | 143 | agctctgctgcctcgtctatacc | 167275 | - | see also 14786 line | | | |
| 33688 | CCL2 | NM_002982.2 | 205 | tgcagaggctcgcgagctataga | 167278 | - | see also 14788 line | | | |
| 33689 | CCL2 | NM_002982.2 | 211 | ggctcgcgagctatagaagaatc | 167281 | - | see also 14788 line | | | |
| 33690 | CCL20 | NM_004591.1 | 164 | tacacagaccgtattcttcatcc | 167283 | - | see also 14789 line | | | |
| 33691 | CCL20 | NM_004591.1 | 269 | ttgtctgtgtgcgcaaatccaaa | 167284 | - | see also 14789 line | | | |
| 33692 | CCL22 | NM_002990.3 | 152 | gcgcgtggtgaaacacttctact | 167289 | - | see also 14790 line | | | |
| 33693 | CCL22 | NM_002990.3 | 154 | gcgtggtgaaacacttctactgg | 167290 | - | see also 14790 line | | | |
| 33694 | CCL23 | NM_005064.3 | 317 | ttcactcctggagagttactttg | 167293 | - | see also 14791 line | | | |
| 33695 | CCL25 | NM_005624.2 | 356 | ctggaaactccaagttatcatcg | 167306 | - | see also 14793 line | | | |
| 33696 | CCL25 | NM_005624.2 | 335 | atgctgtaaagaagttgagttct | 167305 | - | see also 14793 line | | | |
| 33697 | CCL26 | NM_006072.3 | 218 | gggtgcgaagctatgaattcacc | 167313 | - | see also 14794 line | | | |
| 33698 | CCL26 | NM_006072.3 | 214 | acctgggtgcgaagctatgaatt | 167311 | - | see also 14794 line | | | |
| 33699 | CCL27 | NM_006664.2 | 117 | gaccctacagcagcattcctact | 167317 | - | - | chemokine | Peutz-Jeghers syndrome | |
| 33700 | CCL28 | NM_019846.3 | 406 | cacgaaacatacggccataaaac | 167328 | - | see also 14795 line | | | |
| 33701 | CCL28 | NM_019846.3 | 279 | cccgcacaaccatactgttaagc | 167326 | - | see also 14795 line | | | |
| 33702 | CCL28 | NM_019846.3 | 147 | cacggaggtttcacatcatattt | 167320 | - | see also 14795 line | | | |
| 33703 | CCL5 | NM_002985.2 | 244 | acccagcagtcgtctttgtcacc | 167336 | - | see also 14796 line | | | |
| 33704 | CCL5 | NM_002985.2 | 199 | gtgcccacatcaaggagtatttc | 167334 | - | see also 14796 line | | | |
| 33705 | CCL7 | NM_006273.2 | 249 | cccgggaagctgtaatcttcaag | 167342 | - | see also 14797 line | | | |

Figure 46

| ID | Gene | Accession | Pos | Sequence | Num | | Function | Notes |
|---|---|---|---|---|---|---|---|---|
| 33706 | CCL7 | NM_006273.2 | 141 | agccagttcggattaatacttca | 167339 | - | | see also 14797 line |
| 33707 | CCL8 | NM_005623.2 | 564 | aacgtgatcaataggaaaattcc | 167345 | - | | see also 14798 line |
| 33708 | CCL8 | NM_005623.2 | 555 | tgctgctttaacgtgatcaatag | 167344 | - | | see also 14798 line |
| 33709 | CX3CL1 | NM_002996.1 | 156 | accacggtgtgacgaaatgcaac | 167348 | - | | see also 14799 line |
| 33710 | CX3CL1 | NM_002996.1 | 252 | gcggcaaacgcgcaatcatcttg | 167352 | - | | see also 14799 line |
| 33711 | CXCL1 | NM_001511.1 | 340 | tcctgcatccccatagttaaga | 167358 | - | | see also 14800 line |
| 33712 | CXCL10 | NM_001565.1 | 239 | caegtgttgagtcattgctaca | 167365 | - | | see also 14801 line |
| 33713 | CXCL10 | NM_001565.1 | 157 | acctgcatcagcattagtaatca | 167362 | - | | see also 14801 line |
| 33714 | CXCL11 | NM_005409.3 | 332 | agcaagcaaggcttataatcaaa | 167371 | - | | see also 14802 line |
| 33715 | CXCL11 | NM_005409.3 | 237 | ctccataatgtaccagtaaca | 167370 | - | | see also 14802 line |
| 33716 | CXCL12 | NM_000609.3 | 176 | cagatgccatgccgattcttg | 167372 | - | | see also 14803 line |
| 33717 | CXCL12 | NM_000609.3 | 216 | gccaacgtcaagcatctcaaat | 167373 | - | | see also 14803 line |
| 33718 | CXCL13 | NM_006419.1 | 226 | cgcttcattgatcgaattcaaat | 167383 | - | | see also 14804 line |
| 33719 | CXCL13 | NM_006419.1 | 217 | atccctagacgcttcattgatcg | 167380 | - | | see also 14804 line |
| 33720 | CXCL14 | NM_004887.3 | 554 | cgcgtgtgacgggtccaaatgc | 167386 | - | | see also 3391 line |
| 33721 | CXCL14 | NM_004887.3 | 779 | agcgcaggcgtcacgaagaatag | 167391 | - | | see also 3391 line |
| 33722 | CXCL5 | NM_002994.2 | 239 | tgcgttgcgtttgtttcagacc | 167392 | - | | see also 14806 line |
| 33723 | CXCL5 | NM_002994.2 | 266 | aaggagttcatcccaaaatgatc | 167394 | - | | see also 14806 line |
| 33724 | CXCL6 | NM_002993.1 | 218 | taagcgttacgctgagtaaac | 167396 | - | | see also 14807 line |
| 33725 | CXCL6 | NM_002993.1 | 240 | ccccaaaacgattggtaaactgc | 167399 | - | | see also 14807 line |
| 33726 | CXCL9 | NM_002416.1 | 386 | ctcaacgttctcgtcaaagaag | 167410 | - | | see also 14808 line |
| 33727 | CXCL9 | NM_002416.1 | 202 | agcccttcctgcgagaaaattga | 167404 | - | | see also 14808 line |
| 33728 | CXCL9 | NM_002416.1 | 160 | atccacctacaatccttgaaaga | 167401 | - | translation regulator | see also 14808 line |
| 33729 | IL13 | NM_002188.2 | 433 | ttgcgaggacagttcaactga | 167414 | - | signal transduction | |
| 33730 | IL13 | NM_002188.2 | 226 | acctgacagctggcatgtactgt | 167412 | - | | see also 33729 line |
| 33731 | IL13 | NM_002188.2 | 361 | tgcatgtccgagacaccaaaatc | 167413 | - | | see also 33729 line |
| 33732 | IL8 | NM_000584.2 | 176 | gccaaggagtgctaaagaactta | 167415 | - | | see also 14809 line |
| 33733 | IL8 | NM_000584.2 | 193 | aacttagatgtcagtgcataaag | 167417 | - | | see also 14809 line |
| 33734 | PPBP | NM_002704.1 | 330 | ttgcaaccaagtcgaagtgatag | 167426 | - | | see also 14810 line |
| 33735 | PPBP | NM_002704.1 | 331 | tgcaaccaagtcgaagtgatagc | 167427 | - | | see also 14810 line |
| 33736 | IFNB1 | NM_002176.1 | 223 | gacgccgcattgaccatctatga | 167431 | - | | see also 14811 line |
| 33737 | IFNB1 | NM_002176.1 | 225 | cgccgcattgaccatctatgaga | 167432 | - | | see also 14811 line |
| 33738 | IFNW1 | NM_002177.1 | 604 | cctagtgatgaccagctatagc | 167442 | - | | see also 14812 line |
| 33739 | IFNW1 | NM_002177.1 | 655 | ctcagaacatggcctacttagc | 167443 | - | | see also 14812 line |
| 33740 | LIF | NM_002309.2 | 577 | cacctcgggtaaggatgtcttcc | 167449 | - | | see also 14813 line |
| 33741 | LIF | NM_002309.2 | 238 | tgccaatgccctcttttattctct | 167446 | - | | see also 14813 line |
| 33742 | LIF | NM_002309.2 | 248 | ctcttattctcttattcacacagc | 167448 | - | | see also 14813 line |

Figure 46

| | | | | | | | | | |
|---|---|---|---|---|---|---|---|---|---|
| 33743 | OSM | NM_020530.3 | 216 | tcctggaccctatatacgtatc | 167451 | - | see also 14814 line | | |
| 33744 | OSM | NM_020530.3 | 243 | gcctggatgttcctaaactgaga | 167453 | - | see also 14814 line | | |
| 33745 | CTF1 | NM_001330.2 | 136 | tgcgcacctcctcaccaaatacg | 167456 | - | see also 14815 line | | |
| 33746 | IL12A | NM_000882.2 | 928 | ttcgggcagtgactattgataga | 167473 | - | see also 14816 line | | |
| 33747 | IL12A | NM_000882.2 | 678 | gccctgtgccttagtagtattta | 167465 | - | see also 14816 line | | |
| 33748 | IL12B | NM_002187.2 | 977 | accgctactatagctcatcttgg | 167491 | - | see also 14817 line | | |
| 33749 | IL12B | NM_002187.2 | 968 | gggcccaggaccgctactatagc | 167490 | - | see also 14817 line | | |
| 33750 | IL5 | NM_000879.2 | 423 | aacaccgagtggataatagaaag | 167498 | - | see also 14818 line | | |
| 33751 | IL5 | NM_000879.2 | 426 | accgagtggataatagaaagttg | 167499 | - | see also 14818 line | | |
| 33752 | CSF2 | NM_000758.2 | 346 | gccctccaacccggaaacttcc | 167502 | - | see also 14819 line | | |
| 33753 | CSF2 | NM_000758.2 | 375 | acccagattatcacctttgaaag | 167504 | - | see also 14819 line | | |
| 33754 | CSF2 | NM_000758.2 | 365 | ttcctgtgcaacccagattatca | 167503 | - | see also 14819 line | | |
| 33755 | EPO | NM_000799.1 | 384 | tcccagacaccaaagttaatttc | 167507 | - | see also 14820 line | | |
| 33756 | EPO | NM_000799.1 | 430 | cgggcagcaggccgtagaagtct | 167508 | - | see also 14820 line | | |
| 33757 | IL10 | NM_000572.2 | 429 | cggcgctgtcatcgatttcttcc | 167516 | - | see also 14822 line | | |
| 33758 | IL10 | NM_000572.2 | 428 | acggcgctgtcatcgatttcttc | 167515 | - | see also 14822 line | | |
| 33759 | IL9 | NM_000590.1 | 362 | ggcaggcaacgcgctgacatttc | 167521 | - | see also 14823 line | | |
| 33760 | IL9 | NM_000590.1 | 245 | taccaccatgcaaacaagatacc | 167519 | - | see also 14823 line | | |
| 33761 | IL7 | NM_000880.2 | 805 | aagacatatctgtgatgctaata | 167529 | - | see also 573 line | | |
| 33762 | IL7 | NM_000880.2 | 993 | aagcccaaccaacaaagagtttg | 167536 | - | see also 573 line | | |
| 33763 | IL6 | NM_000600.1 | 637 | atctcattctgcgcagctttaag | 167543 | - | see also 14825 line | | |
| 33764 | IL6 | NM_000600.1 | 268 | aggagacatgtaacaagagtaac | 167540 | - | see also 14825 line | | |
| 33765 | IL4 | NM_000589.2 | 756 | accagagtacgttggaaaacttc | 167549 | - | see also 14826 line | | |
| 33766 | IL4 | NM_000589.2 | 415 | agcatgtgccggcaactttgtcc | 167544 | - | see also 14826 line | | |
| 33767 | IL3 | NM_000588.3 | 380 | cgcacccacgcgacatccaatcc | 167555 | - | see also 14827 line | | |
| 33768 | IL3 | NM_000588.3 | 388 | cgcgacatccaatccatatcaag | 167558 | - | see also 14827 line | | |
| 33769 | IL2 | NM_000586.2 | 613 | agcaatatcaacgtaatagttct | 167576 | - | see also 14828 line | | |
| 33770 | IL2 | NM_000586.2 | 644 | agggatctgaaacaacattcatg | 167577 | - | see also 14828 line | | |
| 33771 | IFNG | NM_000619.1 | 448 | gacttcgaaaagctgactaatta | 167586 | - | see also 14829 line | | |
| 33772 | IFNG | NM_000619.1 | 533 | aactgtcgccagcagctaaaaca | 167587 | - | see also 14829 line | | |
| 33773 | IL17E | NM_022789.2 | 571 | ctcccctggagatatgagttgg | 167590 | - | - | - | - |
| 33774 | IFNA5 | NM_002169.1 | 40 | ctcaactgcaagtcaatctgttc | 167591 | - | - | - | - |
| 33775 | IFNA8 | NM_002170.1 | 69 | tgctcagctacaagtcattcagc | 167593 | - | see also 14831 line | | |
| 33776 | IFNA8 | NM_002170.1 | 550 | atccttctctttatcaatcaact | 167596 | - | see also 14831 line | | |
| 33777 | IFNK | NM_020124.1 | 103 | ccctggactgtaacttactgaac | 167600 | - | see also 14832 line | | |
| 33778 | IFNK | NM_020124.1 | 29 | caccaaacctgatatgattcaaa | 167597 | - | see also 14832 line | | |
| 33779 | IFNT1 | NM_176891.2 | 524 | gccgatgtctgttctttgtgttc | 167613 | - | see also 14833 line | | |

Figure 46

| 33780 | IFNT1 | NM_176891.2 | 411 | gggtagtgataaccttagattac | 167609 | - | see also 14833 line | | |
|---|---|---|---|---|---|---|---|---|---|
| 33781 | IL15 | NM_000585.2 | 675 | gtccggagatgcaagtattcatg | 167624 | - | see also 14834 line | | |
| 33782 | IL15 | NM_000585.2 | 585 | tactttatatacggaaagtgatg | 167622 | - | see also 14834 line | | |
| 33783 | ED1 | NM_001399.3 | 696 | aggcgtgttcgccgcaataaaag | 167629 | - | see also 972 line | | |
| 33784 | LTA | NM_000595.2 | 416 | ctccttgagcaacaattctctcc | 167644 | - | see also 14836 line | | |
| 33785 | LTA | NM_000595.2 | 720 | agccctagtactgtcttctttgg | 167646 | - | see also 14836 line | | |
| 33786 | LTB | NM_002341.1 | 388 | tcccgcaggacggcctctattac | 167647 | - | signal_transduction | - | - |
| 33787 | LTB | NM_002341.1 | 389 | cccgcaggacggcctctattacc | 167648 | - | see also 33786 line | | |
| 33788 | TNF | NM_000594.2 | 358 | ccccagggacctctctctaatca | 167650 | - | see also 14837 line | | |
| 33789 | TNF | NM_000594.2 | 787 | ccgactcagcgctgagatcaatc | 167657 | - | see also 14837 line | | |
| 33790 | TNFSF10 | NM_003810.2 | 343 | aactccgtcagctcgttagaaag | 167666 | - | see also 14838 line | | |
| 33791 | TNFSF10 | NM_003810.2 | 743 | tcctgaccctatattgttgatga | 167681 | - | see also 14838 line | | |
| 33792 | TNFSF10 | NM_003810.2 | 742 | atcctgaccctatattgttgatg | 167680 | - | see also 14838 line | | |
| 33793 | TNFSF11 | NM_003701.2 | 1056 | tccggatcaggatgcaacatact | 167696 | - | see also 2526 line | | |
| 33794 | TNFSF11 | NM_003701.2 | 844 | gacctagctacagagtatcttca | 167691 | - | see also 2526 line | | |
| 33795 | TNFSF13 | NM_003808.2 | 1434 | ggcaagggcgaaacttaacctct | 167705 | - | see also 14840 line | | |
| 33796 | TNFSF13 | NM_003808.2 | 1378 | agcgcaggtgtcttccatttaca | 167703 | - | see also 14840 line | | |
| 33797 | TNFSF13B | NM_006573.3 | 883 | tacgccatgggacatctaattca | 167713 | - | see also 14841 line | | |
| 33798 | TNFSF13B | NM_006573.3 | 880 | acctacgccatgggacatctaat | 167712 | - | see also 14841 line | | |
| 33799 | TNFSF13B | NM_006573.3 | 290 | agcagtcacgccttacttcttgc | 167707 | - | see also 14841 line | | |
| 33800 | TNFSF15 | NM_005118.2 | 813 | atctctttggtggattacacaaa | 167720 | - | see also 14842 line | | |
| 33801 | TNFSF15 | NM_005118.2 | 497 | caccaagaaccgaatgaactata | 167717 | - | see also 14842 line | | |
| 33802 | TNFSF4 | NM_003326.2 | 654 | tggcggagaactgattcttatcc | 167732 | - | see also 14843 line | | |
| 33803 | TNFSF4 | NM_003326.2 | 323 | atcctcgaattcaaagtatcaaa | 167723 | - | see also 14843 line | | |
| 33804 | TNFSF5 | NM_000074.1 | 632 | ccggtagattcgagagaatctta | 167746 | - | see also 14844 line | | |
| 33805 | TNFSF5 | NM_000074.1 | 687 | accttgcgggcaacaatccattc | 167748 | - | see also 14844 line | | |
| 33806 | TNFSF6 | NM_000639.1 | 974 | cagacgtttttcggcttatataa | 167767 | - | see also 14845 line | | |
| 33807 | TNFSF6 | NM_000639.1 | 976 | gacgtttttcggcttatataagc | 167768 | - | see also 14845 line | | |
| 33808 | TNFSF8 | NM_001244.2 | 343 | cacggtggccactattatggtgt | 167772 | - | see also 14846 line | | |
| 33809 | TNFSF8 | NM_001244.2 | 286 | cacgagccgcagctatttctatt | 167770 | - | see also 14846 line | | |
| 33810 | TNFSF9 | NM_003811.2 | 422 | ggctggagtctactatgtcttct | 167793 | - | see also 14847 line | | |
| 33811 | TNFSF9 | NM_003811.2 | 291 | cagctggtggcccaaaatgttct | 167790 | - | see also 14847 line | | |
| 33812 | IL1F5 | NM_012275.2 | 590 | tgcccccatcacagacttctact | 167800 | - | - | - | - |
| 33813 | IL1F5 | NM_012275.2 | 200 | ctcggcattgaaggtgctttatc | 167797 | - | see also 33812 line | | |
| 33814 | IL1F5 | NM_012275.2 | 253 | tgcatgcagggaaggtcattaaa | 167798 | - | see also 33812 line | | |
| 33815 | IL1RN | NM_000577.3 | 564 | aggcgtcatggtcaccaaattct | 167808 | - | see also 14848 line | | |
| 33816 | IL1RN | NM_000577.3 | 220 | ctgaggaacaaccaactagttgc | 167803 | - | see also 14848 line | | |

Figure 46

| | | | | | | | | | |
|---|---|---|---|---|---|---|---|---|---|
| 33817 | IL18 | NM_001562.2 | 679 | ttcatcatacgaaggatactttc | 167828 | - | see also 14849 line | | |
| 33818 | IL18 | NM_001562.2 | 450 | atgcacccccggaccatatttatt | 167821 | - | see also 14849 line | | |
| 33819 | IL1A | NM_000575.3 | 1640 | ctcacggcactaagaactatttc | 167859 | - | see also 14850 line | | |
| 33820 | IL1A | NM_000575.3 | 1638 | aactcacggcactaagaactatt | 167858 | - | see also 14850 line | | |
| 33821 | IL1B | NM_000576.2 | 162 | agctgatggccctaaacagatga | 167866 | - | see also 14851 line | | |
| 33822 | IL1B | NM_000576.2 | 463 | ctccgggactcacagcaaaaaag | 167869 | - | see also 14851 line | | |
| 33823 | IL1B | NM_000576.2 | 495 | gtctggtccatatgaactgaaag | 167870 | - | see also 14851 line | | |
| 33824 | IL1F10 | NM_032556.4 | 843 | cagcccgtaccaagttttacttt | 167884 | - | see also 9244 line | | |
| 33825 | IL1F10 | NM_032556.4 | 841 | ctcagcccgtaccaagttttact | 167883 | - | see also 9244 line | | |
| 33826 | IL1F10 | NM_032556.4 | 844 | agcccgtaccaagttttactttg | 167885 | - | see also 9244 line | | |
| 33827 | IL1F6 | NM_014440.1 | 124 | gtcactattgccttaatctcatg | 167891 | - | see also 14853 line | | |
| 33828 | IL1F6 | NM_014440.1 | 120 | tccagtcactattgccttaatct | 167889 | - | see also 14853 line | | |
| 33829 | IL1F7 | NM_014439.3 | 398 | gtctctactgtgacaaggataaa | 167897 | - | see also 14854 line | | |
| 33830 | IL1F7 | NM_014439.3 | 379 | gtctctaaaggggagttttgtct | 167896 | - | see also 14854 line | | |
| 33831 | IL1F7 | NM_014439.3 | 576 | cacctcctgcaattgtaatgagc | 167902 | - | see also 14854 line | | |
| 33832 | IL1F8 | NM_014438.3 | 199 | ctcctcttagccgcagcattaag | 167913 | - | see also 14855 line | | |
| 33833 | IL1F8 | NM_014438.3 | 129 | cccaaatcctatgctattcgtga | 167911 | - | see also 14855 line | | |
| 33834 | IL1F8 | NM_014438.3 | 135 | tcctatgctattcgtgattctcg | 167912 | - | see also 14855 line | | |
| 33835 | IL1F9 | NM_019618.2 | 101 | gaggaagggccgtctatcaatca | 167925 | - | see also 14856 line | | |
| 33836 | IL1F9 | NM_019618.2 | 110 | ccgtctatcaatcaatgtgtaaa | 167929 | - | see also 14856 line | | |
| 33837 | INHBC | NM_005538.2 | 452 | atcaaccagactcgtcttgattt | 167940 | - | see also 14857 line | | |
| 33838 | INHBC | NM_005538.2 | 865 | gtgctgtcgacaagagtttttg | 167948 | - | see also 14857 line | | |
| 33839 | TGFB1 | NM_000660.1 | 1417 | ctcgccagagtggttatcttttg | 167962 | - | see also 14858 line | | |
| 33840 | TGFB1 | NM_000660.1 | 1186 | aacccacaacgaaatctatgaca | 167958 | - | see also 14858 line | | |
| 33841 | TGFB1 | NM_000660.1 | 1728 | tgcggcagctgtacattgacttc | 167967 | - | see also 14858 line | | |
| 33842 | TGFB2 | NM_003238.1 | 1133 | tgcctacgtccactttacattga | 167994 | - | see also 14859 line | | |
| 33843 | TGFB2 | NM_003238.1 | 457 | gaggagcgacgaagagtactacg | 167972 | - | see also 14859 line | | |
| 33844 | TGFB3 | NM_003239.1 | 803 | cgctatatcggtggcaagaatct | 168013 | - | see also 14860 line | | |
| 33845 | TGFB3 | NM_003239.1 | 642 | aggttttccgcttcaatgtgtcc | 168012 | - | see also 14860 line | | |
| 33846 | TGFB3 | NM_003239.1 | 842 | gccgagtggctgtcctttgatgt | 168014 | - | see also 14860 line | | |
| 33847 | BTC | NM_001729.1 | 728 | ttcggaaacgtcgtaaaagaaag | 168027 | - | cell_cycle | growth factor | - |
| 33848 | BTC | NM_001729.1 | 725 | ctcttcggaaacgtcgtaaaaga | 168026 | - | see also 33847 line | | |
| 33849 | BTC | NM_001729.1 | 734 | aacgtcgtaaaagaaagaagaaa | 168029 | - | see also 33847 line | | |
| 33850 | DTR | NM_001945.1 | 707 | cagtggaaaatcgcttatatacc | 168032 | - | see also 14861 line | | |
| 33851 | DTR | NM_001945.1 | 705 | cccagtggaaaatcgcttatata | 168031 | - | see also 14861 line | | |
| 33852 | EREG | NM_001432.1 | 563 | gtcgtcggttccacatattattt | 168052 | - | see also 14862 line | | |
| 33853 | EREG | NM_001432.1 | 523 | ggctttgaccgtgattcttatta | 168049 | - | see also 14862 line | | |

Figure 46

| 33854 | EREG | NM_001432.1 | 420 | tggacatgagtcaaaactactgc | 168043 | - | see also 14862 line |
|---|---|---|---|---|---|---|---|
| 33855 | ECGF1 | NM_001953.2 | 709 | tcctgcggacggaatcctatatg | 168058 | - | see also 14863 line |
| 33856 | ECGF1 | NM_001953.2 | 708 | ttcctgcggacggaatcctatat | 168057 | - | see also 14863 line |
| 33857 | FIGF | NM_004469.2 | 770 | taggtttgcggcaactttctatg | 168072 | - | see also 14864 line |
| 33858 | FIGF | NM_004469.2 | 775 | ttgcggcaactttctatgacatt | 168074 | - | see also 14864 line |
| 33859 | PDGFB | NM_002608.1 | 1130 | ctcgatccgctcctttgatgatc | 168098 | - | see also 14865 line |
| 33860 | PDGFB | NM_002608.1 | 1128 | cactcgatccgctcctttgatga | 168097 | - | see also 14865 line |
| 33861 | PDGFB | NM_002608.1 | 1097 | tcccgaggagctttatgagatgc | 168096 | - | see also 14865 line |
| 33862 | VEGF | NM_003376.3 | 1632 | gaggcagcttgagttaaacgaac | 168113 | - | see also 2277 line |
| 33863 | VEGF | NM_003376.3 | 1500 | gcgcaagaaatcccggtataagt | 168109 | - | see also 2277 line |
| 33864 | VEGFB | NM_003377.3 | 401 | cagtgtgaatgcagacctaaaaa | 168115 | - | see also 14866 line |
| 33865 | MUC4 | NM_004532.2 | 1513 | agggacgacgggacttacgattc | 168130 | - | see also 14867 line |
| 33866 | MUC4 | NM_004532.2 | 2585 | ggcgaacgccaccctcaatcagt | 168139 | - | see also 14867 line |
| 33867 | KITLG | NM_000899.1 | 268 | tgcaggaatcgtgtgactaataa | 168197 | - | see also 584 line |
| 33868 | KITLG | NM_000899.1 | 267 | ctgcaggaatcgtgtgactaata | 168196 | - | see also 584 line |
| 33869 | CSF1 | NM_000757.3 | 586 | gcctgcgtccgaactttctatga | 168235 | - | see also 14869 line |
| 33870 | CSF1 | NM_000757.3 | 585 | ggcctgcgtccgaactttctatg | 168234 | - | see also 14869 line |
| 33871 | IL22 | NM_020525.3 | 312 | gagtgagcgctgctatctgatga | 168251 | - | see also 14870 line |
| 33872 | IL22 | NM_020525.3 | 436 | aacaggctaagcacatgtcatat | 168254 | - | see also 14870 line |
| 33873 | DKFZp451J0118 | NM_175852.3 | 1024 | aggagcatatcgacaaagtcttc | 168263 | - | see also 14871 line |
| 33874 | DKFZp451J0118 | NM_175852.3 | 94 | ctgccaaacaatccaatccaaaa | 168259 | - | see also 14871 line |
| 33875 | AREG | NM_001657.2 | 438 | ctcgggagccgactatgactact | 168272 | - | see also 14872 line |
| 33876 | AREG | NM_001657.2 | 435 | gtcctcgggagccgactatgact | 168271 | - | see also 14872 line |
| 33877 | BMP10 | NM_014482.1 | 616 | agggatcgtatgatatacgatgg | 168293 | - | see also 14874 line |
| 33878 | BMP10 | NM_014482.1 | 296 | aagacggtgtcgactttaacaca | 168283 | - | see also 14874 line |
| 33879 | BMP15 | NM_005448.1 | 980 | tactacgcgatggtctcaattcc | 168329 | - | see also 14875 line |
| 33880 | BMP15 | NM_005448.1 | 1074 | cccgtataagtatgttccaatta | 168336 | - | see also 14875 line |
| 33881 | BMP2 | NM_001200.1 | 1276 | cggggtatcacgccttttactgc | 168356 | - | see also 780 line |
| 33882 | BMP2 | NM_001200.1 | 1183 | aacagcggaaacgccttaagtcc | 168354 | - | see also 780 line |
| 33883 | BMP3 | NM_001201.1 | 1027 | cagagccttatatcttggtatat | 168378 | - | see also 14877 line |
| 33884 | BMP3 | NM_001201.1 | 1356 | agccagacgctccaatttgatga | 168384 | - | see also 14877 line |
| 33885 | BMP4 | NM_001202.2 | 1100 | atgtgacacggtgggaaactttt | 168408 | - | see also 782 line |
| 33886 | BMP4 | NM_001202.2 | 1345 | gccaagcgtagccctaagcatca | 168411 | - | see also 782 line |
| 33887 | BMP5 | NM_021073.2 | 1543 | tggacgcagtatcaacgtaaaat | 168437 | - | see also 14878 line |
| 33888 | BMP5 | NM_021073.2 | 767 | tgggttctagtgggttatgcaaa | 168415 | - | see also 14878 line |

Figure 46

| 33889 | BMP6 | NM_001718.2 | 1317 | cgccgacaacagagtcgtaatcg | 168457 | - | see also 14879 line | | |
|---|---|---|---|---|---|---|---|---|---|
| 33890 | BMP6 | NM_001718.2 | 1610 | ctgtgcgccaactaagctaaatg | 168463 | - | see also 14879 line | | |
| 33891 | BMP7 | NM_001719.1 | 287 | gcgcgagatcctctccattttgg | 168465 | - | see also 14880 line | | |
| 33892 | BMP7 | NM_001719.1 | 667 | gggaacgcttcgacaatgagacg | 168469 | - | see also 14880 line | | |
| 33893 | CKLF | NM_016326.2 | 300 | cagcggtccttaccagaaaaagc | 168473 | - | - | - | - |
| 33894 | CKLF | NM_016326.2 | 154 | aacgtgcagccgaaaataaaaca | 168472 | - | see also 33893 line | | |
| 33895 | CLC | NM_013246.1 | 152 | ctggccctccatccagaaaacc | 168490 | - | see also 5365 line | | |
| 33896 | CXCL16 | NM_022059.1 | 669 | agcttaccatcggtgtctatact | 168493 | - | see also 14881 line | | |
| 33897 | CXCL16 | NM_022059.1 | 624 | cccgccatcggttcagttcatga | 168491 | - | see also 14881 line | | |
| 33898 | CXCL16 | NM_022059.1 | 674 | accatcggtgtctatactacacg | 168494 | - | see also 14881 line | | |
| 33899 | EBAF | NM_003240.2 | 246 | cacgtgagggcccagtatgtagt | 168503 | - | signal_transduction | transforming growth factor-beta receptor ligand | - |
| 33900 | FAM3B | NM_058186.2 | 147 | aggacgtttgttggttaagttaa | 168507 | - | see also 14882 line | | |
| 33901 | FAM3B | NM_058186.2 | 143 | ttcaaggacgtttgttggttaag | 168505 | - | see also 14882 line | | |
| 33902 | FAM3C | NM_014888.1 | 302 | cacagctgcacgttctacaaagc | 168520 | - | see also 14883 line | | |
| 33903 | FAM3C | NM_014888.1 | 615 | tacgatgatggagcaaccaaact | 168527 | - | see also 14883 line | | |
| 33904 | FAM3C | NM_014888.1 | 675 | agcacatctattactaatcttgg | 168529 | - | see also 14883 line | | |
| 33905 | FAM3D | NM_138805.2 | 793 | gggagttcctacgcaaaacaact | 168547 | - | see also 14884 line | | |
| 33906 | FAM3D | NM_138805.2 | 792 | ggggagttcctacgcaaaacaac | 168546 | - | see also 14884 line | | |
| 33907 | FLT3LG | NM_001459.2 | 519 | ctggatcactcgccagaacttct | 168553 | - | see also 14885 line | | |
| 33908 | FLT3LG | NM_001459.2 | 391 | gtgaacacggagatacactttgt | 168552 | - | see also 14885 line | | |
| 33909 | GDF10 | NM_004962.2 | 888 | agcgctcgaggtgctatgcaagc | 168556 | - | see also 14886 line | | |
| 33910 | GDF10 | NM_004962.2 | 1639 | atctcaccgaaatcttttgatgc | 168559 | - | see also 14886 line | | |
| 33911 | GDF11 | NM_005811.2 | 452 | caccgagaccgtcattagcatgg | 168563 | - | see also 14887 line | | |
| 33912 | GDF11 | NM_005811.2 | 1061 | ctccggccagtgcgagtacatgt | 168568 | - | see also 14887 line | | |
| 33913 | GDF2 | NM_016204.1 | 469 | tacgccagcgtccaacattgtgc | 168574 | - | see also 6723 line | | |
| 33914 | GDF2 | NM_016204.1 | 664 | aggaagcgtggtcatttatgatg | 168576 | - | see also 6723 line | | |
| 33915 | GDF3 | NM_020634.1 | 236 | cccgagacttatgctacgtaaag | 168588 | - | see also 14889 line | | |
| 33916 | GDF3 | NM_020634.1 | 513 | cacccctaagccaggtaaaatgt | 168591 | - | see also 14889 line | | |
| 33917 | GDF3 | NM_020634.1 | 1078 | gtcattctacgacattatgaaga | 168601 | - | see also 14889 line | | |
| 33918 | GDF8 | NM_005259.1 | 468 | acgctacaacggaaacaatcatt | 168615 | - | see also 3700 line | | |
| 33919 | GDF9 | NM_005260.2 | 1240 | gtgccaagaccgtcatgtgtacc | 168669 | - | see also 14891 line | | |
| 33920 | GDF9 | NM_005260.2 | 444 | taccgttgaacacttactcaagt | 168643 | - | see also 14891 line | | |
| 33921 | GPI | NM_000175.2 | 1567 | agccttggtcgccatgtatgagc | 168694 | - | see also 14892 line | | |
| 33922 | GPI | NM_000175.2 | 834 | ttgccctgtctactaacacaacc | 168682 | - | see also 14892 line | | |
| 33923 | IK | NM_006083.2 | 1697 | atcgccagtggaagaagattagt | 168697 | - | - | cytokine | - |

Figure 46

| | | | | | | | | | |
|---|---|---|---|---|---|---|---|---|---|
| 33924 | IL16 | NM_004513.3 | 1128 | gacgaagctacttgacgaaaaga | 168704 | - | see also 14893 line | | |
| 33925 | IL16 | NM_004513.3 | 575 | ttcgccaaagcttgaaaggtttg | 168701 | - | see also 14893 line | | |
| 33926 | IL17 | NM_002190.2 | 196 | atccataaccggaataccaatac | 168755 | - | see also 14894 line | | |
| 33927 | IL17 | NM_002190.2 | 197 | tccataaccggaataccaatacc | 168756 | - | see also 14894 line | | |
| 33928 | IL17 | NM_002190.2 | 202 | aaccggaataccaataccaatcc | 168757 | - | see also 14894 line | | |
| 33929 | IL17F | NM_052872.2 | 156 | aggtagtatgaagcttgacattg | 168767 | - | see also 14896 line | | |
| 33930 | IL17F | NM_052872.2 | 97 | atccccaaagtaggacatacttt | 168763 | - | see also 14896 line | | |
| 33931 | IL19 | NM_013371.2 | 1138 | cagatcattaagcccttagatgt | 168775 | - | see also 14897 line | | |
| 33932 | IL19 | NM_013371.2 | 1032 | cacagacatgcaccatatagaag | 168774 | - | see also 14897 line | | |
| 33933 | IL20 | NM_018724.2 | 309 | ctctatctggacagggtatttaa | 168790 | - | see also 14898 line | | |
| 33934 | IL20 | NM_018724.2 | 376 | gcctcgccaattcctttcttacc | 168793 | - | see also 14898 line | | |
| 33935 | IL21 | NM_021803.1 | 418 | atgtgattcttatgagaaaaaac | 168805 | - | see also 14899 line | | |
| 33936 | IL21 | NM_021803.1 | 235 | tccagaagatgtagagacaaact | 168803 | - | see also 14899 line | | |
| 33937 | IL24 | NM_006850.2 | 792 | tccggagagcattcaaacagttg | 168810 | - | apoptosis | - | melanoma |
| 33938 | IL24 | NM_006850.2 | 641 | aactaccacaatagaacagttga | 168807 | - | see also 33937 line | | |
| 33939 | IL24 | NM_006850.2 | 725 | ctgcaacccagtcaagaaaatga | 168809 | - | see also 33937 line | | |
| 33940 | IL26 | NM_018402.1 | 211 | accgcataaaaaatatacgatta | 168818 | - | see also 14900 line | | |
| 33941 | IL26 | NM_018402.1 | 210 | gaccgcataaaaaatatacgatt | 168817 | - | see also 14900 line | | |
| 33942 | MDK | NM_002391.2 | 264 | ttggagccgactgcaagtacaag | 168824 | - | see also 14901 line | | |
| 33943 | MDK | NM_002391.2 | 79 | ctccgcggtcgccaaaaagaaag | 168822 | - | see also 14901 line | | |
| 33944 | MIF | NM_002415.1 | 369 | gcccggacagggtctacatcaac | 168828 | - | see also 14902 line | | |
| 33945 | MIF | NM_002415.1 | 372 | cggacagggtctacatcaactat | 168829 | - | see also 14902 line | | |
| 33946 | NODAL | NM_018055.3 | 995 | tcctagatcaccataaagacatg | 168847 | - | see also 14903 line | | |
| 33947 | NODAL | NM_018055.3 | 564 | tgccaccaatgtgctccttatgc | 168836 | - | see also 14903 line | | |
| 33948 | PBEF | NM_005746.1 | 76 | tcctacaaggttactcactataa | 168848 | - | signal_transduction | - | - |
| 33949 | PGLYRP | NM_005091.1 | 521 | agccctgaggtccaactatgtgc | 168850 | - | see also 14904 line | | |
| 33950 | PLAB | NM_004864.1 | 918 | atgacttgttagccaaagactgc | 168853 | - | see also 14905 line | | |
| 33951 | PLAB | NM_004864.1 | 901 | gtcgctccagacctatgatgact | 168852 | - | see also 14905 line | | |
| 33952 | PTN | NM_002825.4 | 373 | cagcagcgtcgaaaatttgcagc | 168854 | - | see also 14906 line | | |
| 33953 | PTN | NM_002825.4 | 570 | agctgagtgcaagcaaaccatga | 168858 | - | see also 14906 line | | |
| 33954 | PTN | NM_002825.4 | 794 | aacctcaagcagaatctaagaag | 168860 | - | see also 14906 line | | |
| 33955 | SCGB1A1 | NM_003357.3 | 297 | agcccaaagctcactgtgtaatt | 168864 | - | see also 14907 line | | |
| 33956 | SCGB1A1 | NM_003357.3 | 261 | gcccagagaaagcatcattaagc | 168862 | - | see also 14907 line | | |
| 33957 | SCGB1A1 | NM_003357.3 | 274 | atcattaagctcatggaaaaaat | 168863 | - | see also 14907 line | | |
| 33958 | SPP1 | NM_000582.2 | 752 | acctgaacgcgccttctgattgg | 168881 | - | see also 14908 line | | |
| 33959 | SPP1 | NM_000582.2 | 433 | gactcgaacgactctgatgatgt | 168872 | - | see also 14908 line | | |
| 33960 | SPP1 | NM_000582.2 | 890 | agcattccgatgtgattgatagt | 168886 | - | see also 14908 line | | |

Figure 46

| | | | | | | | | transcription co-activator | persistent Mullerian duct syndrome |
|---|---|---|---|---|---|---|---|---|---|
| 33961 | THPO | NM_000460.2 | 964 | tacacgaactcttgaatggaact | 168896 | - | see also 14909 line | | |
| 33962 | THPO | NM_000460.2 | 1108 | atcctcctactggacagtatacg | 168897 | - | see also 14909 line | | |
| 33963 | TNFRSF11B | NM_002546.2 | 476 | caccagtgacgagtgtctatact | 168905 | | see also 14910 line | | |
| 33964 | TNFRSF11B | NM_002546.2 | 578 | agggcgctaccttgagatagagt | 168906 | - | see also 14910 line | | |
| 33965 | TNFSF18 | NM_005092.2 | 98 | gtggctctttgctccaatagtta | 168925 | - | see also 14911 line | | |
| 33966 | TNFSF18 | NM_005092.2 | 448 | atgtttgggaccaccatagacttg | 168940 | - | see also 14911 line | | |
| 33967 | AMH | NM_000479.2 | 1626 | cgccgagccgctccgtactcatcc | 168948 | - | | | |
| 33968 | BDNF | NM_001709.3 | 455 | gacatcattggctgacactttcg | 168955 | - | see also 1134 line | | |
| 33969 | CECR1 | NM_017424.2 | 1272 | ttcttccacgccggagaaacaga | 168998 | - | see also 14913 line | | |
| 33970 | CECR1 | NM_017424.2 | 1384 | accccgcagtcaggacttactcc | 168999 | - | see also 14913 line | | |
| 33971 | DKK1 | NM_012242.1 | 222 | gagtgagcgccaccttgaactcg | 169006 | - | see also 5219 line | | |
| 33972 | DKK1 | NM_012242.1 | 868 | gtcttgccggatacagaaagatc | 169011 | - | see also 5219 line | | |
| 33973 | FGF1 | NM_000800.2 | 541 | cgcgtctccggactcactatgg | 169026 | - | see also 14916 line | | |
| 33974 | FGF1 | NM_000800.2 | 172 | gccctgaccgagaagtttaatct | 169015 | - | see also 14916 line | | |
| 33975 | FGF10 | NM_004465.1 | 227 | atgtccgctggagaaagctattc | 169029 | - | see also 3024 line | | |
| 33976 | FGF10 | NM_004465.1 | 509 | agcataatgggaggcaaatgat | 169031 | - | see also 3024 line | | |
| 33977 | FGF11 | NM_004112.2 | 651 | tacagttcgccgcatttcacagc | 169032 | - | see also 14917 line | | |
| 33978 | FGF11 | NM_004112.2 | 671 | agctgagtgtcgctttaaggagt | 169034 | - | see also 14917 line | | |
| 33979 | FGF11 | NM_004112.2 | 668 | cacagctgagtgtcgctttaagg | 169033 | - | see also 14917 line | | |
| 33980 | FGF12 | NM_004113.3 | 631 | cacattttgtaccgaaacctatt | 169044 | - | see also 14918 line | | |
| 33981 | FGF12 | NM_004113.3 | 529 | cactgtaccgccagcaagaatca | 169043 | - | see also 14918 line | | |
| 33982 | FGF12 | NM_004113.3 | 348 | gactacaactccttcaatcttaat | 169039 | - | see also 14918 line | | |
| 33983 | FGF13 | NM_004114.2 | 984 | ctgcgagtggtggctatccaagg | 169058 | - | see also 2777 line | | |
| 33984 | FGF13 | NM_004114.2 | 906 | ctgcagccgatggaaccattga | 169056 | - | see also 2777 line | | |
| 33985 | FGF14 | NM_004115.2 | 682 | agcacaagtcgtctgcaataat | 169082 | - | see also 2780 line | | |
| 33986 | FGF14 | NM_004115.2 | 207 | gggtatagtgaccaggttatatt | 169069 | - | see also 2780 line | | |
| 33987 | FGF17 | NM_003867.1 | 518 | agcgcgaggcccactcatcaag | 169094 | - | see also 2624 line | | |
| 33988 | FGF18 | NM_003862.1 | 956 | ccctgatgtcggctaagtactcc | 169097 | - | see also 14921 line | | |
| 33989 | FGF18 | NM_003862.1 | 766 | ggccgaggatggggacaagtatgc | 169096 | - | see also 14921 line | | |
| 33990 | FGF2 | NM_002006.2 | 780 | aacgattggaatctaataactac | 169105 | - | see also 1285 line | | |
| 33991 | FGF2 | NM_002006.2 | 751 | atgtgttaccggatgagtgtttct | 169101 | - | see also 1285 line | | |
| 33992 | FGF20 | NM_019851.1 | 446 | gtgggactggtcagtattagagg | 169113 | - | see also 14924 line | | |
| 33993 | FGF20 | NM_019851.1 | 727 | aagagttccagaattgtacaagg | 169122 | - | see also 14924 line | | |
| 33994 | FGF21 | NM_019113.1 | 261 | gccggagttattcaaatcttgg | 169126 | - | see also 14925 line | | |

Figure 46

| 33995 | FGF21 | NM_019113.1 | 253 | gccttgaagccgggagttattca | 169124 | - | see also 14925 line |
|-------|-------|-------------|-----|-------------------------|--------|---|---------------------|
| 33996 | FGF23 | NM_020638.2 | 631 | acgagatcccctaattcacttc | 169134 | - | see also 14927 line |
| 33997 | FGF23 | NM_020638.2 | 419 | aagatacctctgcatggatttca | 169131 | - | see also 14927 line |
| 33998 | FGF3 | NM_005247.2 | 858 | gagctgggctataatacgtatgc | 169139 | - | see also 14928 line |
| 33999 | FGF3 | NM_005247.2 | 696 | gagaacagcgcctacagtatttt | 169137 | - | see also 14928 line |
| 34000 | FGF4 | NM_002007.1 | 820 | cgcctacgagtcctacaagtacc | 169141 | - | see also 14929 line |
| 34001 | FGF5 | NM_004464.2 | 480 | atcccacgaagccaatatgttaa | 169146 | - | see also 3021 line |
| 34002 | FGF5 | NM_004464.2 | 481 | tcccacgaagccaatatgttaag | 169147 | - | see also 3021 line |
| 34003 | FGF6 | NM_020996.1 | 551 | tgcctacgagtcagacttgtacc | 169166 | - | see also 14931 line |
| 34004 | FGF6 | NM_020996.1 | 451 | ccctcttcgttgccatgaacagt | 169163 | - | see also 14931 line |
| 34005 | FGF7 | NM_002009.2 | 671 | tacctgaggatcgataaaagagg | 169179 | - | see also 14932 line |
| 34006 | FGF7 | NM_002009.2 | 593 | gagcgacacacaagaagttatga | 169175 | - | see also 14932 line |
| 34007 | FGF8 | NM_006119.2 | 676 | agcgtgaggtccacttcatgaag | 169184 | - | see also 14933 line |
| 34008 | FGF8 | NM_006119.2 | 737 | gcgcttcgagttcctcaactacc | 169186 | - | see also 14933 line |
| 34009 | FGF9 | NM_002010.1 | 262 | cagcccggttttgttaagtgacc | 169195 | - | see also 1288 line |
| 34010 | FGF9 | NM_002010.1 | 255 | cggtggacagcccggttttgtta | 169191 | - | see also 1288 line |
| 34011 | GDNF | NM_000514.2 | 657 | gagacaacgtacgacaaaatatt | 169220 | - | see also 398 line |
| 34012 | GDNF | NM_000514.2 | 543 | aaccggggttgtgtcttaactgc | 169215 | - | see also 398 line |
| 34013 | GDNF | NM_000514.2 | 659 | gacaacgtacgacaaaatattga | 169221 | - | see also 398 line |
| 34014 | GRP | NM_002091.3 | 191 | gaccgtgctgaccaagatgtacc | 169228 | - | see also 14936 line |
| 34015 | GRP | NM_002091.3 | 342 | aggaatttgctgggtctcataga | 169230 | - | see also 14936 line |
| 34016 | HDGF | NM_004494.1 | 799 | ctggaggactctcctaaacgtcc | 169235 | - | see also 3044 line |
| 34017 | IGF1 | NM_000618.2 | 186 | ttccaacccaattatttaagtgc | 169238 | - | see also 14937 line |
| 34018 | IGF1 | NM_000618.2 | 247 | gtcctcctcgcatctcttctacc | 169239 | - | see also 14937 line |
| 34019 | IGF1 | NM_000618.2 | 179 | agcagtcttccaacccaattatt | 169236 | - | see also 14937 line |
| 34020 | INHBE | NM_031479.3 | 962 | atgttgcaggcgagaccattacg | 169250 | - | see also 14938 line |
| 34021 | INHBE | NM_031479.3 | 717 | aacctgggctggcataccttaac | 169243 | - | see also 14938 line |
| 34022 | LOC145957 | NM_138573.1 | 389 | tggcggtcctagtaacacttatc | 169262 | - | see also 14939 line |
| 34023 | LOC145957 | NM_138573.1 | 285 | ttgtaggtgcgttgaaaactata | 169257 | - | see also 14939 line |
| 34024 | MIA | NM_006533.1 | 330 | agcgttcagggagattactatgg | 169270 | - | see also 14940 line |
| 34025 | MIA | NM_006533.1 | 269 | gggccaagtggtgtatgtcttct | 169268 | - | see also 14940 line |
| 34026 | NDP | NM_000266.1 | 530 | ggcaccactatgtggattctatc | 169275 | - | see also 14941 line |
| 34027 | NDP | NM_000266.1 | 419 | atgtactagctgcatcctttct | 169272 | - | see also 14941 line |
| 34028 | NGFB | NM_002506.1 | 828 | ggcggtttatccggatagatacg | 169286 | - | see also 14942 line |
| 34029 | NGFB | NM_002506.1 | 360 | cggggcagacccgcaacattact | 169277 | - | see also 14942 line |

Figure 46

| 34030 | NRG1 | NM_013962.1 | 867 | cagcaggtacatcttcttcatgg | 169337 | - | see also 14943 line |
|---|---|---|---|---|---|---|---|
| 34031 | NRG2 | NM_004883.1 | 865 | ctcgataccaacggcaaaaatct | 169345 | - | see also 14944 line |
| 34032 | NRG2 | NM_004883.1 | 1289 | atggaggcgtctgctactacatc | 169351 | - | see also 14944 line |
| 34033 | NTF3 | NM_002527.2 | 525 | aacggtacgcggagcataagagt | 169375 | - | see also 1629 line |
| 34034 | NTF3 | NM_002527.2 | 825 | ggcggtggatacggatagacacg | 169386 | - | see also 1629 line |
| 34035 | NTF5 | NM_006179.3 | 710 | ggcgatggattcgaattgacact | 169393 | - | see also 14946 line |
| 34036 | NTF5 | NM_006179.3 | 706 | ggctggcgatggattcgaattga | 169391 | - | see also 14946 line |
| 34037 | NTF5 | NM_006179.3 | 555 | aacccgctgcaaggctgataacg | 169389 | - | see also 14946 line |
| 34038 | NUDT6 | NM_007083.3 | 514 | tacaagatcgaaataaattgaaa | 169399 | - | see also 4946 line |
| 34039 | NUDT6 | NM_007083.3 | 379 | accacgcagaatcggattcatca | 169394 | - | see also 4946 line |
| 34040 | NUDT6 | NM_007083.3 | 381 | cacgcagaatcggattcatcaac | 169395 | - | see also 4946 line |
| 34041 | OGN | NM_014057.2 | 508 | atctttacgcacgattcaacaaa | 169418 | - | see also 5474 line |
| 34042 | OGN | NM_014057.2 | 1015 | taccgatagggtcatacttttaa | 169434 | - | see also 5474 line |
| 34043 | PDGFC | NM_016205.1 | 1116 | cagttggacttagaagatctata | 169449 | - | see also 14948 line |
| 34044 | PDGFC | NM_016205.1 | 744 | tggatacaacttacgtttgatga | 169439 | - | see also 14948 line |
| 34045 | PGF | NM_002632.3 | 544 | cagcgaggtggagcacatgttca | 169461 | - | see also 14949 line |
| 34046 | PGF | NM_002632.3 | 633 | cggtggagacggccaatgtcacc | 169465 | - | see also 14949 line |
| 34047 | PROK1 | NM_032414.1 | 63 | tgccacgcgagtctcaatcatgc | 169469 | - | see also 14950 line |
| 34048 | PROK1 | NM_032414.1 | 60 | aggtgccacgcgagtctcaatca | 169467 | - | see also 14950 line |
| 34049 | RABEP1 | NM_004703.2 | 1429 | ctcgggctcattgcaatccaaag | 169493 | - | see also 3222 line |
| 34050 | RABEP1 | NM_004703.2 | 2215 | ggcactgcgggagttggtattaa | 169522 | - | see also 3222 line |
| 34051 | SCDGF-B | NM_025208.2 | 622 | cacattcaagtccgatgactact | 169545 | - | see also 14952 line |
| 34052 | SCDGF-B | NM_025208.2 | 1004 | tactcggtcaatataagagaaga | 169554 | - | see also 14952 line |
| 34053 | TFF1 | NM_003225.1 | 236 | ccctggtgcttctatcctaatac | 169562 | - | see also 14954 line |
| 34054 | TFF1 | NM_003225.1 | 230 | ggggtcccctggtgcttctatcc | 169561 | - | see also 14954 line |
| 34055 | VEGFC | NM_005429.2 | 1052 | tcctgccgatgcatgtctaaact | 169578 | - | see also 3790 line |
| 34056 | VEGFC | NM_005429.2 | 1051 | ttcctgccgatgcatgtctaaac | 169577 | - | see also 3790 line |
| 34057 | VEGFC | NM_005429.2 | 866 | gtcgcgacaaacaccttctttaa | 169570 | - | see also 3790 line |
| 34058 | VGF | NM_003378.2 | 787 | cacgctgacccgagtgaatctgg | 169595 | - | see also 14956 line |
| 34059 | VGF | NM_003378.2 | 785 | cacacgctgacccgagtgaatct | 169594 | - | see also 14956 line |
| 34060 | VGF | NM_003378.2 | 1567 | gacgatcgacagcctcattgagc | 169597 | - | see also 14956 line |
| 34061 | ADCYAP1 | NM_001117.2 | 510 | gacgccgaatagcttatttgtag | 169605 | - | see also 14957 line |
| 34062 | ADCYAP1 | NM_001117.2 | 250 | cgcccacgggatccttaacgagg | 169600 | - | see also 14957 line |
| 34063 | AGRP | NM_001138.1 | 622 | tgctactgccgcttcttcaatgc | 169606 | - | signal_transduction | - | obesity |
| 34064 | ASMT | NM_004043.1 | 603 | agcttttacggccatctacagg | 169613 | - | see also 14958 line |
| 34065 | ASMT | NM_004043.1 | 874 | atgtaagatcaccgttttgaca | 169615 | - | see also 14958 line |

Figure 46

| | | | | | | | | | |
|---|---|---|---|---|---|---|---|---|---|
| 34066 | CALCB | NM_000728.2 | 439 | ccaccaatggttccaaagc | 169617 | - | - | neuropeptide hormone | - |
| 34067 | CORT | NM_001302.1 | 231 | tcctgcttggtgtttgagtgg | 169619 | - | see also 14959 line | | |
| 34068 | CRH | NM_000756.1 | 499 | aacaggcgaccgccaacttttc | 169621 | - | see also 14960 line | | |
| 34069 | GAL | NM_015973.2 | 437 | gcgcacaatcattgagtttctgt | 169623 | - | see also 14961 line | | |
| 34070 | GALP | NM_033106.1 | 256 | gagggagacagcgccttgagatcc | 169624 | - | see also 14962 line | | |
| 34071 | GALP | NM_033106.1 | 328 | gccctccaagagagaatgtgatgg | 169625 | - | see also 14962 line | | |
| 34072 | NPY | NM_000905.2 | 233 | ctrggcgtcgcgacactacatca | 169627 | - | see also 14963 line | | |
| 34073 | NPY | NM_000905.2 | 236 | ggcgctgcgacactacatcaacc | 169629 | - | see also 14963 line | | |
| 34074 | NTS | NM_006183.3 | 575 | accacagaagacccttacatactca | 169636 | - | see also 14964 line | | |
| 34075 | NTS | NM_006183.3 | 553 | aacgcgcagctgtatgagaataaa | 169635 | - | see also 14964 line | | |
| 34076 | PMCH | NM_002674.1 | 233 | ctccttccctggaacaatataaa | 169644 | - | signal_transduction | neuropeptide hormone | - |
| 34077 | PMCH | NM_002674.1 | 234 | tccttccctggaacaatataaaa | 169645 | - | see also 34076 line | | |
| 34078 | PMCH | NM_002674.1 | 132 | atccaagtccataagaaatttag | 169641 | - | see also 34076 line | | |
| 34079 | PNOC | NM_006228.2 | 720 | tgcaccagaatggtaatgtgtag | 169650 | - | see also 4319 line | | |
| 34080 | PNOC | NM_006228.2 | 631 | caggaagttggccaatcagaagc | 169648 | - | see also 4319 line | | |
| 34081 | TAC1 | NM_003182.1 | 214 | agccaatgatgatctgaattact | 169653 | - | see also 2157 line | | |
| 34082 | TAC1 | NM_003182.1 | 500 | aggagtgcaatgcagaattatga | 169656 | - | see also 2157 line | | |
| 34083 | UCN | NM_003353.2 | 450 | gccgagcagaaccgcatcatatt | 169658 | - | see also 2260 line | | |
| 34084 | VIP | NM_003381.2 | 377 | gacacaccctattatgatgtatc | 169663 | - | signal_transduction, g pcr | translation regulator | rheumatoid arthritis |
| 34085 | VIP | NM_003381.2 | 569 | aactataccgcccttagataaaca | 169666 | - | see also 34084 line | | |
| 34086 | VIP | NM_003381.2 | 482 | gagtctcttatgggaaaaacgtgt | 169665 | - | see also 34084 line | | |
| 34087 | PTH | NM_000315.2 | 290 | ctgcaggatgtgcacaatttgt | 169682 | - | see also 14966 line | | |
| 34088 | PTH | NM_000315.2 | 230 | atgcataacctgggaaaaacatct | 169680 | - | see also 14966 line | | |
| 34089 | PTHLH | NM_002820.2 | 599 | gtccgatttgggtcgtgatgatga | 169686 | - | see also 14967 line | | |
| 34090 | PTHLH | NM_002820.2 | 463 | ggggaagtccatccaagatttac | 169685 | - | see also 14967 line | | |
| 34091 | GNRH1 | NM_000825.2 | 1179 | gagagatgccgaaaatttgattg | 169687 | - | see also 14968 line | | |
| 34092 | GNRH1 | NM_000825.2 | 1184 | atgccgaaaatttgattgattct | 169688 | - | see also 14968 line | | |
| 34093 | NPPB | NM_002521.1 | 360 | ccgtgggcaccgcaaaatggtcc | 169691 | - | signal_transduction | diuretic hormone | - |
| 34094 | NPPB | NM_002521.1 | 227 | tacaggacagcgcaaccattg | 169690 | - | see also 34093 line | | |
| 34095 | TRH | NM_007117.1 | 320 | tccgcgtcccagatctttcaatc | 169692 | - | see also 14969 line | | |
| 34096 | TRH | NM_007117.1 | 323 | ggcgtcccagatctttcaatctga | 169694 | - | see also 14969 line | | |
| 34097 | ADM | NM_001124.1 | 234 | cgcgtcggagtttcgaaagaagt | 169697 | - | see also 14970 line | | |
| 34098 | ADM | NM_001124.1 | 440 | accgcgcagcatgaacaaacttc | 169698 | - | see also 14970 line | | |
| 34099 | AGT | NM_000029.1 | 373 | ttcttgggcttccgtatatatgg | 169700 | - | see also 14971 line | | |

Figure 46

| | | | | | | | | | |
|---|---|---|---|---|---|---|---|---|---|
| 34100 | AGT | NM_000029.1 | 1110 | gggtctcactttccagcaaaact | 169701 | - | see also 14971 line | | |
| 34101 | APLN | NM_017413.2 | 731 | ttcccgttctctcacaagaatcc | 169704 | - | signal_transduction | hormone | - |
| 34102 | APLN | NM_017413.2 | 471 | ggcagggaggtcggaggaaattc | 169703 | - | see also 34101 line | | |
| 34103 | APM1 | NM_004797.1 | 375 | gtgggattggagacttacgttac | 169706 | - | see also 14972 line | | |
| 34104 | APM1 | NM_004797.1 | 371 | cagtgtgggattggagacttacg | 169705 | - | see also 14972 line | | |
| 34105 | CALCA | NM_001741.1 | 313 | atctaagcggtgcggtaatctga | 169713 | - | see also 14973 line | | |
| 34106 | CALCA | NM_001741.1 | 453 | gagaccatcgccctcatgttagc | 169715 | - | see also 14973 line | | |
| 34107 | CCK | NM_000729.2 | 286 | tggacgaatgtccatcgttaaga | 169717 | - | see also 14974 line | | |
| 34108 | CCK | NM_000729.2 | 379 | tcgcagtgccgaggagtatgagt | 169721 | - | see also 14974 line | | |
| 34109 | CGA | NM_000735.2 | 342 | tccacttgctgtgtagctaaatc | 169723 | - | see also 14975 line | | |
| 34110 | CGA | NM_000735.2 | 347 | ttgctgtgtagctaaatcatata | 169724 | - | see also 14975 line | | |
| 34111 | COPA | NM_004371.2 | 1718 | gccgtttgggtcgctcgaaatcg | 169750 | - | see also 2933 line | | |
| 34112 | COPA | NM_004371.2 | 941 | gtgcgcgtttgggatatttctgg | 169730 | - | see also 2933 line | | |
| 34113 | COPA | NM_004371.2 | 2540 | atgtgctatcagcgtaccaaaaa | 169774 | - | see also 2933 line | | |
| 34114 | FLJ14800 | NM_032840.1 | 1097 | tccggaacgtgcggaatgtcatg | 169815 | - | see also 14977 line | | |
| 34115 | FLJ14800 | NM_032840.1 | 709 | gggactctgctggagtacttagg | 169813 | - | see also 14977 line | | |
| 34116 | FSHB | NM_000510.1 | 319 | agcgacagcactgattgtactgt | 169823 | - | see also 14978 line | | |
| 34117 | FSHB | NM_000510.1 | 98 | aagaatgtcgtttctgcataagc | 169818 | - | see also 14978 line | | |
| 34118 | GAS | NM_000805.2 | 151 | agccagatgcacccttaggtaca | 169825 | - | signal_transduction | peptide hormone | - |
| 34119 | GCG | NM_002054.2 | 364 | gccaaacgtcacgatgaatttga | 169829 | - | see also 1328 line | | |
| 34120 | GCG | NM_002054.2 | 303 | caggcgtgcccaagattttgtgc | 169828 | - | see also 1328 line | | |
| 34121 | GIP | NM_004123.1 | 401 | ggccagtcaagctaataggaagg | 169845 | - | see also 14981 line | | |
| 34122 | GIP | NM_004123.1 | 397 | agctggccagtcaagctaatagg | 169843 | - | see also 14981 line | | |
| 34123 | GNRH2 | NM_001501.1 | 390 | cccgccatcctccaataaagtgt | 169846 | - | signal_transduction | - | - |
| 34124 | GNRH2 | NM_001501.1 | 392 | cgccatcctccaataaagtgtga | 169847 | - | see also 34123 line | | |
| 34125 | GPB5 | NM_145171.2 | 213 | ctggaacccccctatattgaagc | 169849 | - | see also 14982 line | | |
| 34126 | GPHA2 | NM_130769.2 | 282 | gtggttaccgacacaacatcacc | 169851 | - | see also 14983 line | | |
| 34127 | GPHA2 | NM_130769.2 | 332 | ggcctgaagaaggtcaaagtaca | 169853 | - | see also 14983 line | | |
| 34128 | GRTP1 | NM_024719.1 | 588 | ttccagacatttgcgataagttt | 169863 | - | see also 14984 line | | |
| 34129 | GRTP1 | NM_024719.1 | 591 | cagacatttgcgataagtttaag | 169865 | - | see also 14984 line | | |
| 34130 | GUCA2A | NM_033553.2 | 76 | gtcaccgtgcaggatggaaattt | 169866 | - | - | - | - |
| 34131 | GUCA2A | NM_033553.2 | 225 | cccgaactttccagaagaactca | 169869 | - | see also 34130 line | | |
| 34132 | GUCA2A | NM_033553.2 | 223 | aacccgaactttccagaagaact | 169868 | - | see also 34130 line | | |
| 34133 | HAMP | NM_021175.1 | 252 | tgctgtcatcgatcaaagtgtgg | 169871 | - | see also 14985 line | | |
| 34134 | IAPP | NM_000415.1 | 326 | aacgtgggatccaatacatatgg | 169874 | - | see also 14986 line | | |
| 34135 | IAPP | NM_000415.1 | 353 | aggaatgcagtagaggttttaaa | 169876 | - | see also 14986 line | | |
| 34136 | IAPP | NM_000415.1 | 352 | gaggaatgcagtagaggttttaa | 169875 | - | see also 14986 line | | |

EP 1 752 536 A1

Figure 46

| | | | | | | | | | | |
|---|---|---|---|---|---|---|---|---|---|---|
| 34137 | INS | NM_000207.1 | 307 | agcgtggcattgtggaacaatgc | 169880 | - | see also 14987 line | | | |
| 34138 | INS | NM_000207.1 | 172 | gcggggaacgaggcttcttctac | 169878 | - | see also 14987 line | | | |
| 34139 | INSL4 | NM_002195.1 | 337 | gccttaggtacgacatcagaatt | 169887 | - | see also 14988 line | | | |
| 34140 | INSL4 | NM_002195.1 | 288 | tggacgtcccaaagaaatggtgt | 169884 | - | see also 14988 line | | | |
| 34141 | INSL5 | NM_005478.3 | 117 | ctctgtgggctagaatacatacg | 169901 | - | see also 14989 line | | | |
| 34142 | INSL5 | NM_005478.3 | 136 | tacggacagtcatctatatctgt | 169902 | - | see also 14989 line | | | |
| 34143 | INSL6 | NM_007179.2 | 351 | cactacctgagtataaggataaa | 169917 | - | see also 14990 line | | | |
| 34144 | INSL6 | NM_007179.2 | 349 | gtcactacctgagtataaggata | 169916 | - | see also 14990 line | | | |
| 34145 | LEP | NM_000230.1 | 225 | gtcaccgtttggacttcattcc | 169926 | - | see also 14991 line | | | |
| 34146 | LEP | NM_000230.1 | 257 | cccatcctgaccttatccaaga | 169929 | - | see also 14991 line | | | |
| 34147 | LEP | NM_000230.1 | 253 | tccacccatcctgaccttatcc | 169927 | - | see also 14991 line | | | |
| 34148 | LRSAM1 | NM_138361.2 | 473 | gacattctgacatctctaaatg | 169934 | - | see also 14992 line | | | |
| 34149 | LRSAM1 | NM_138361.2 | 619 | ggcaaccatcaaggttctagatc | 169935 | - | see also 14992 line | | | |
| 34150 | MGC45438 | NM_152459.2 | 909 | cacagatggaccagtcatgttct | 169953 | - | see also 14993 line | | | |
| 34151 | MGC45438 | NM_152459.2 | 137 | ggctggacactgctgaaagtaaa | 169952 | - | see also 14993 line | | | |
| 34152 | NPPA | NM_006172.1 | 168 | cagagctaatccatgtacaatg | 169957 | - | see also 14994 line | | | |
| 34153 | NPPA | NM_006172.1 | 121 | accgtgagcttcctcctttact | 169956 | - | see also 14994 line | | | |
| 34154 | POMC | NM_000939.1 | 810 | agcgctacgcgccgtttcatgacc | 169959 | - | signal_transduction | | peptide hormone | obesity |
| 34155 | PPY | NM_002722.1 | 57 | acctgccgttggctctgttactaca | 169960 | - | see also 14995 line | | | |
| 34156 | PPY | NM_002722.1 | 198 | aggcctaggtatgggaaaagaca | 169962 | - | see also 14995 line | | | |
| 34157 | PYY | NM_004160.1 | 261 | cggcagcggtatgggaaaagaga | 169963 | - | signal_transduction, g pcr | | peptide hormone | diabetes |
| 34158 | RETNLB | NM_032579.1 | 173 | ttccttagactccgttatggata | 169964 | - | see also 14996 line | | | |
| 34159 | RETNLB | NM_032579.1 | 255 | ctctcgtgtgctagtgtcaaaag | 169969 | - | see also 14996 line | | | |
| 34160 | RETNLB | NM_032579.1 | 174 | tccttagactccgttatggataa | 169965 | - | see also 14996 line | | | |
| 34161 | RLN1 | NM_006911.2 | 464 | cagcagtatgtacctgcattaaa | 169975 | - | see also 14997 line | | | |
| 34162 | RLN1 | NM_006911.2 | 210 | aggacgatgttattaaattatgc | 169974 | - | see also 14997 line | | | |
| 34163 | RLN2 | NM_005059.2 | 36 | ctgtttactactgaaccaatttt | 169978 | - | - | | | |
| 34164 | RLN2 | NM_005059.2 | 41 | tactactgaaccaattttccaga | 169979 | - | see also 34163 line | | | |
| 34165 | RLN3 | NM_080864.1 | 385 | tgcaagtgggggtgtagcaaaag | 169984 | - | - | | | |
| 34166 | RLN3 | NM_080864.1 | 388 | aagtgggggtgtagcaaaagtga | 169985 | - | see also 34165 line | | | |
| 34167 | RLN3 | NM_080864.1 | 391 | tgggggtagcaaaagtgaaat | 169986 | - | see also 34165 line | | | |
| 34168 | STC1 | NM_003155.1 | 513 | ttcttgtacagcgctgctaaatt | 169994 | - | see also 15000 line | | | |
| 34169 | STC1 | NM_003155.1 | 377 | atcccgagttcggctcaaaact | 169992 | - | see also 15000 line | | | |
| 34170 | STC2 | NM_003714.1 | 478 | ggcacacggttcggctgcataagc | 170006 | - | see also 15001 line | | | |

Figure 46

| | | | | | | | |
|---|---|---|---|---|---|---|---|
| 34171 | STC2 | NM_003714.1 | 370 | ggggcttacatgggattgcatg | 170004 | - | see also 15001 line |
| 34172 | TG | NM_003235.3 | 886 | gacggttcctcgcagttcaatca | 170023 | - | see also 2185 line |
| 34173 | TG | NM_003235.3 | 7951 | cagcctacgagggggcagtttct | 170134 | - | see also 2185 line |
| 34174 | TSHB | NM_000549.1 | 42 | atgtgggcaagcgatgtctttt | 170140 | - | see also 407 line |
| 34175 | TSHB | NM_000549.1 | 105 | gtgtgcttattgcctaaccatca | 170143 | - | see also 407 line |
| 34176 | UCN3 | NM_053049.1 | 451 | gcccacctgattgcgcaaatgg | 170155 | - | see also 15003 line |
| 34177 | UCN3 | NM_053049.1 | 57 | gacaggcctcccccacacaagttct | 170153 | - | see also 15003 line |
| 34178 | UTS2 | NM_006786.2 | 302 | ctctggacaagatcctaacatt | 170164 | - | see also 15004 line |
| 34179 | UTS2 | NM_006786.2 | 305 | tggacaagatcctaacattttac | 170166 | - | see also 15004 line |
| 34180 | ADAM11 | NM_002390.2 | 2160 | cacgggggacggaggagatata | 170180 | - | see also 15005 line |
| 34181 | ADAM11 | NM_002390.2 | 712 | accaagtatgtggagctaattgt | 170176 | - | see also 15005 line |
| 34182 | ADAM2 | NM_001464.2 | 1182 | cacaatcagcctcgcttagatcc | 170232 | - | see also 15006 line |
| 34183 | ADAM2 | NM_001464.2 | 2078 | cctgaaggcgctacattgaga | 170258 | - | see also 15006 line |
| 34184 | ADAM22 | NM_004194.2 | 843 | atcggctttcgttgtacatacc | 170278 | - | see also 15007 line |
| 34185 | ADAM22 | NM_004194.2 | 863 | accaatacctatgcgaaatctgt | 170280 | - | see also 15007 line |
| 34186 | ADAM23 | NM_003812.1 | 1390 | ctcgccgggtgacatttcactata | 170374 | - | see also 15008 line |
| 34187 | ADAM23 | NM_003812.1 | 434 | aggcctccgcattggaatgaaac | 170339 | - | see also 15008 line |
| 34188 | ADAM23 | NM_003812.1 | 947 | agcacaggtcgaccacatataat | 170363 | - | see also 15008 line |
| 34189 | ECM2 | NM_001393.1 | 945 | acccggtaagaggagatagttc | 170425 | - | see also 15009 line |
| 34190 | ECM2 | NM_001393.1 | 1219 | aggcatagtccaaagcattca | 170429 | - | see also 15009 line |
| 34191 | ICAM2 | NM_000873.2 | 119 | atcggatgagaaggtattcgagg | 170464 | - | see also 15010 line |
| 34192 | ICAM2 | NM_000873.2 | 110 | ctgtccaggatcggatgagaagg | 170462 | - | see also 15010 line |
| 34193 | ICAM3 | NM_002162.2 | 1515 | gactatcgtactggccttaatgt | 170489 | - | see also 15011 line |
| 34194 | ICAM3 | NM_002162.2 | 1358 | tcccgttcttgtcaacgtaaca | 170481 | - | see also 15011 line |
| 34195 | ICAM4 | NM_001544.2 | 777 | ggcgctgcgtacctatgcaagt | 170495 | - | see also 15012 line |
| 34196 | ICAM4 | NM_001544.2 | 773 | ctgtgggcgctgcgtacctatgc | 170494 | - | see also 15012 line |
| 34197 | TGFBI | NM_000358.1 | 291 | tacgagtcgtgtcctggatatga | 170499 | - | see also 15013 line |
| 34198 | TGFBI | NM_000358.1 | 1128 | aacggggtgatccactaccattga | 170510 | - | see also 15013 line |
| 34199 | DOK4 | NM_018110.2 | 697 | gacagatcgcttcaatgtcttcc | 170534 | - | see also 15014 line |
| 34200 | DOK4 | NM_018110.2 | 903 | ctgggaaggactctatacettc | 170536 | - | see also 15014 line |
| 34201 | DOK5 | NM_018431.2 | 774 | ggcggtattggacgtgatactacg | 170558 | - | see also 7469 line |
| 34202 | DOK5 | NM_018431.2 | 474 | atgacgatacctccaagactttt | 170549 | - | see also 7469 line |
| 34203 | FLJ22570 | NM_024872.1 | 1044 | gccaccacgtccaggaaaatgc | 170562 | - | - |
| 34204 | FRS2 | NM_006654.2 | 1518 | cagacgccaagtttagaacaca | 170596 | - | see also 4699 line |
| 34205 | FRS2 | NM_006654.2 | 392 | cccgcaaacgtgactcagtaaaa | 170566 | - | see also 4699 line |
| 34206 | FRS3 | NM_006653.3 | 1477 | cccaaggcagcttaactacatcc | 170614 | - | see also 15016 line |
| 34207 | FRS3 | NM_006653.3 | 403 | ggccgtccgctggccttatctct | 170602 | - | see also 15016 line |

Figure 46

| 34208 | IRS2 | NM_003749.2 | 3433 | tccgctctccgactacatgaacc | 170618 | - | signal_transduction | - | diabetes mellitus、diabetes、obesity |
|---|---|---|---|---|---|---|---|---|---|
| 34209 | IRS2 | NM_003749.2 | 4468 | cacctacgccagcattgacttct | 170620 | - | see also 34208 line | | |
| 34210 | IRS2 | NM_003749.2 | 3432 | ctccgctctccgactacatgaac | 170617 | - | see also 34208 line | | |
| 34211 | IRS4 | NM_003604.1 | 753 | ggcggagccacccttctataaag | 170631 | - | see also 15017 line | | |
| 34212 | IRS4 | NM_003604.1 | 398 | ctcggctggaatactacgaaaat | 170627 | - | see also 15017 line | | |
| 34213 | MGC20785 | NM_152721.1 | 657 | ttgactgaaccaatgacattatc | 170699 | - | see also 15018 line | | |
| 34214 | MGC20785 | NM_152721.1 | 786 | atgtctcgtgcacagacatttcc | 170705 | - | see also 15018 line | | |
| 34215 | CD4 | NM_000616.2 | 769 | aggcctccagcatagtctataag | 170714 | - | see also 15019 line | | |
| 34216 | CD4 | NM_000616.2 | 823 | cactcgcctttacagttgaaaag | 170717 | - | see also 15019 line | | |
| 34217 | TLR8 | NM_016610.2 | 3177 | ggctacggcagcggatctgtaag | 170837 | - | see also 15020 line | | |
| 34218 | TLR8 | NM_016610.2 | 272 | gagttatgcgccgaagaaaattt | 170725 | - | see also 15020 line | | |
| 34219 | PDYN | NM_024411.2 | 750 | tgggggctttttgcgcaaatacc | 170847 | - | see also 15021 line | | |
| 34220 | PDYN | NM_024411.2 | 549 | aagcaagtttctcccaagtatct | 170844 | - | see also 15021 line | | |
| 34221 | REA | NM_007273.3 | 985 | cacagaatcgtatctatctcaca | 170866 | - | see also 15022 line | | |
| 34222 | REA | NM_007273.3 | 983 | atcacagaatcgtatctatctca | 170865 | - | see also 15022 line | | |
| 34223 | BID | NM_001196.2 | 678 | gtctttcacacaacagtgaattt | 170873 | - | see also 15023 line | | |
| 34224 | BID | NM_001196.2 | 366 | cactcccgcttgggaagaataga | 170870 | - | see also 15023 line | | |
| 34225 | GPRC5D | NM_018654.1 | 268 | cccgtacgctactttctctttgg | 170884 | - | see also 15024 line | | |
| 34226 | GPRC5D | NM_018654.1 | 244 | atcgagctcaatcaacaaactgc | 170881 | - | see also 15024 line | | |
| 34227 | GPRC5D | NM_018654.1 | 267 | ccccgtacgctactttctctttg | 170883 | - | see also 15024 line | | |
| 34228 | BZRAP1 | NM_004758.1 | 1161 | gacaacctacccgtgattctagg | 170895 | - | see also 15025 line | | |
| 34229 | BZRAP1 | NM_004758.1 | 5067 | cacctacccgtcaggatctttgt | 170910 | - | see also 15025 line | | |
| 34230 | BZRAP1 | NM_004758.1 | 5610 | gacgatgacggtttctactatgg | 170913 | - | see also 15025 line | | |
| 34231 | ALCAM | NM_001627.1 | 1181 | ttcgatctagcccgtcattttct | 170956 | - | see also 1080 line | | |
| 34232 | ALCAM | NM_001627.1 | 1114 | cccgtgtcatgcacaatatctgc | 170954 | - | see also 1080 line | | |
| 34233 | ALCAM | NM_001627.1 | 721 | tgctcggtgacatattatggacc | 170947 | - | see also 1080 line | | |
| 34234 | ALS2CR3 | NM_015049.1 | 1849 | agcttgcgtcgacaaaactattt | 171012 | - | see also 6208 line | | |
| 34235 | ALS2CR3 | NM_015049.1 | 1518 | ggggactatgcgtaaaaagctga | 171006 | - | see also 6208 line | | |
| 34236 | ANGPT1 | NM_001146.3 | 1129 | ggcttggttactcgtcaaacata | 171050 | - | see also 727 line | | |
| 34237 | ANGPT2 | NM_001147.1 | 1445 | cagcaacgctatgtgcttaaaat | 171101 | - | see also 15029 line | | |
| 34238 | ANGPT2 | NM_001147.1 | 1234 | tggcatctacacgttaacattcc | 171095 | - | see also 15029 line | | |
| 34239 | ANGPTL1 | NM_004673.3 | 1273 | taccaccggtaactttcatcaat | 171138 | - | see also 15030 line | | |
| 34240 | ANGPTL1 | NM_004673.3 | 1277 | accggtaactttcatcaatgaag | 171140 | - | see also 15030 line | | |
| 34241 | ANGPTL2 | NM_012098.2 | 597 | ggctcgccaagagagttcattta | 171166 | - | see also 5129 line | | |
| 34242 | ANGPTL2 | NM_012098.2 | 1657 | tggaacctgagagcgagtattat | 171171 | - | see also 5129 line | | |

Figure 46

| 34243 | APOB | NM_000384.1 | 9929 | gtcggcattcggctatgtgttcc | 171454 | - | see also 15032 line | | |
|---|---|---|---|---|---|---|---|---|---|
| 34244 | APOB | NM_000384.1 | 2027 | ctctcggaactatcaactctaca | 171211 | - | see also 15032 line | | |
| 34245 | APOE | NM_000041.1 | 319 | aaggagttgaaggcctacaaatc | 171584 | - | see also 15033 line | | |
| 34246 | APOE | NM_000041.1 | 992 | ctgtgcccagcgacaatcactga | 171586 | - | see also 15033 line | | |
| 34247 | APOF | NM_001638.1 | 767 | agcgatcagtgctgcacttaaac | 171597 | - | see also 15034 line | | |
| 34248 | APOF | NM_001638.1 | 384 | taccgccagggtggtgtgaatgc | 171592 | - | see also 15034 line | | |
| 34249 | APOF | NM_001638.1 | 984 | atcaagagctatgacttagatcc | 171605 | - | see also 15034 line | | |
| 34250 | ASIP | NM_001672.2 | 168 | tggcgctgaacaagaaatccaaa | 171609 | - | see also 15036 line | | |
| 34251 | C17 | NM_018659.1 | 340 | ctcgttctgcaggagagatttgg | 171611 | - | see also 15037 line | | |
| 34252 | C17 | NM_018659.1 | 344 | ttctgcaggagagatttggtatt | 171612 | - | see also 15037 line | | |
| 34253 | C3 | NM_000064.1 | 2736 | ctcgttgtccgttccatatgtca | 171639 | - | see also 15038 line | | |
| 34254 | C3 | NM_000064.1 | 2734 | tcctcgttgtccgttccatatgt | 171638 | - | see also 15038 line | | |
| 34255 | C3 | NM_000064.1 | 2733 | gtcctcgttgtccgttccatatg | 171637 | - | see also 15038 line | | |
| 34256 | CD1D | NM_001766.2 | 870 | ctgggaacgcctcaaataacttc | 171679 | - | see also 15039 line | | |
| 34257 | CD1D | NM_001766.2 | 867 | accctgggaacgcctcaaataac | 171677 | - | see also 15039 line | | |
| 34258 | CD72 | NM_001782.1 | 219 | cacctacgagaatgttcaagtgc | 171685 | - | see also 15040 line | | |
| 34259 | CD72 | NM_001782.1 | 217 | atcacctacgagaatgttcaagt | 171684 | - | see also 15040 line | | |
| 34260 | CD80 | NM_005191.2 | 629 | accggaccatctttgatatcact | 171706 | - | see also 15041 line | | |
| 34261 | CD80 | NM_005191.2 | 699 | cacatacgagtgtgttgttctga | 171707 | - | see also 15041 line | | |
| 34262 | CD80 | NM_005191.2 | 752 | acctggctgaagtgacgttatca | 171708 | - | see also 15041 line | | |
| 34263 | DBI | NM_020548.2 | 172 | agcaactgtgggcgacataaata | 171727 | - | see also 15042 line | | |
| 34264 | DBI | NM_020548.2 | 152 | atctatggccactacaaacaagc | 171725 | - | see also 15042 line | | |
| 34265 | EDN3 | NM_000114.1 | 503 | gacaaggagtgtgtctactattg | 171730 | - | signal_transduction | toxin | Waardenburg-Hirschsprung disease |
| 34266 | EDN3 | NM_000114.1 | 720 | ctctggacgtcagcagtaattca | 171731 | - | see also 34265 line | | |
| 34267 | EFNA1 | NM_004428.2 | 470 | aaggacacagctactactacatc | 171736 | - | see also 2962 line | | |
| 34268 | F2R | NM_001992.2 | 453 | accttagatccccggtcatttct | 171742 | - | see also 15045 line | | |
| 34269 | F2R | NM_001992.2 | 1436 | gtgcatcgacccccctaatttact | 171767 | - | see also 15045 line | | |
| 34270 | F2RL1 | NM_005242.3 | 823 | cacgacctgtcatgatgtttttgc | 171783 | - | see also 15046 line | | |
| 34271 | F2RL1 | NM_005242.3 | 1236 | gccgaagtgtccgcactgtaaag | 171791 | - | see also 15046 line | | |
| 34272 | GFRA3 | NM_001496.2 | 367 | gccccgcagccttggtaactatga | 171798 | - | see also 15047 line | | |
| 34273 | GFRA3 | NM_001496.2 | 366 | tgcccgcagccttggtaactatg | 171797 | - | see also 15047 line | | |
| 34274 | GFRA3 | NM_001496.2 | 1037 | cagctaagatgcgttttcacagc | 171802 | - | see also 15047 line | | |
| 34275 | GLG1 | NM_012201.1 | 2538 | ggcaacgctcagattatcgaatg | 171863 | - | see also 5191 line | | |
| 34276 | GLG1 | NM_012201.1 | 1376 | ttgttccggattacatcgaaaag | 171835 | - | see also 5191 line | | |
| 34277 | GLMN | NM_007070.2 | 618 | gtgttgcaaggccttaatagagt | 171914 | - | see also 15049 line | | |
| 34278 | GLMN | NM_007070.2 | 1248 | agctatgcttcagctgtatatta | 171929 | - | see also 15049 line | | |
| 34279 | ICOSL | NM_015259.2 | 845 | cagtcagtaccggcgagaaaaac | 171954 | - | see also 15050 line | | |

Figure 46

| | | | | | | | |
|---|---|---|---|---|---|---|---|
| 34280 | ICOSL | NM_015259.2 | 607 | cccaagtgtactgctgatcaataa | 171952 | - | see also 15050 line |
| 34281 | NMU | NM_006681.1 | 211 | tgccgaggtgctccaatattacc | 171956 | - | see also 15051 line |
| 34282 | NMU | NM_006681.1 | 531 | cagagtggacgaagaattccaaa | 171959 | - | see also 15051 line |
| 34283 | PCSK1N | NM_013271.2 | 615 | tacttgctgggacggattcttgc | 171960 | - | - |
| 34284 | PLXNC1 | NM_005761.1 | 3633 | cacggagtccgtcgtcgaaaaac | 172033 | - | see also 4018 line |
| 34285 | PLXNC1 | NM_005761.1 | 2655 | ggcgacttactgccgggttttag | 172013 | - | see also 4018 line |
| 34286 | SELPLG | NM_003006.2 | 179 | gagacaggccaccgaatatgagt | 172077 | - | see also 15053 line |
| 34287 | SELPLG | NM_003006.2 | 190 | ccgaatatgagtacctagattat | 172078 | - | see also 15053 line |
| 34288 | STOML2 | NM_013442.1 | 691 | acccgagagtcggccatcaatgt | 172095 | - | see also 15054 line |
| 34289 | STOML2 | NM_013442.1 | 337 | gtgactctgacaatgtaactct | 172087 | - | see also 15054 line |
| 34290 | TAC3 | NM_013251.1 | 414 | gactctcctacggatgtgaatca | 172103 | - | see also 15055 line |
| 34291 | TAC3 | NM_013251.1 | 422 | tacggatgtgaatcaagagaacg | 172104 | - | see also 15055 line |
| 34292 | WNT5A | NM_003392.3 | 713 | gccgcgagacggccttcacatac | 172115 | - | see also 15056 line |
| 34293 | WNT5A | NM_003392.3 | 454 | tggtggtcgctaggtatgaataa | 172109 | - | see also 15056 line |
| 34294 | WNT7A | NM_004625.3 | 400 | tggggcgcaagcatcatctgtaac | 172117 | - | see also 15057 line |
| 34295 | WNT7A | NM_004625.3 | 401 | ggggcgcaagcatcatctgtaaca | 172118 | - | see also 15057 line |
| 34296 | ZP3 | NM_007155.4 | 779 | cacgtgccactgcggttgttgt | 172124 | - | see also 15058 line |
| 34297 | ARHGEF2 | NM_004723.2 | 1591 | atgctgcagaatctaatcgtac | 172138 | - | see also 3238 line |
| 34298 | ARHGEF2 | NM_004723.2 | 1592 | tcgctgcagaatcaatcgtacg | 172139 | - | see also 3238 line |
| 34299 | CHN1 | NM_001822.2 | 746 | cacgtaggatacacaaccttaaa | 172172 | - | see also 1177 line |
| 34300 | CHN1 | NM_001822.2 | 724 | gacgataaacccaatttatgagc | 172170 | - | see also 1177 line |
| 34301 | CHN1 | NM_001822.2 | 1188 | ttgagtctagagtcttaattct | 172180 | - | see also 1177 line |
| 34302 | CHN2 | NM_004067.1 | 586 | aagagtttgagtcgattcatga | 172208 | - | see also 15061 line |
| 34303 | CHN2 | NM_004067.1 | 975 | ctgtggattgaacgtacacaaac | 172224 | - | see also 15061 line |
| 34304 | DGKD | NM_003648.2 | 3297 | tccgcctcgtgaccaagtttaaa | 172288 | - | see also 15062 line |
| 34305 | DGKD | NM_003648.2 | 2156 | gtcatcagtcgcctgttaattaa | 172273 | - | see also 15062 line |
| 34306 | DGKQ | NM_001347.2 | 334 | tcctgtgcgacgtctgcaatttc | 172292 | - | see also 15063 line |
| 34307 | DGKQ | NM_001347.2 | 338 | gtgcgacgtctgcaatttcatgt | 172293 | - | see also 15063 line |
| 34308 | VAV2 | NM_003371.2 | 1092 | gacttggcgatgtacatcaatga | 172317 | - | see also 15064 line |
| 34309 | VAV2 | NM_003371.2 | 1149 | agcgaatttcagagttctataga | 172320 | - | see also 15064 line |
| 34310 | VAV3 | NM_006113.3 | 2423 | tgccatcgctcggtatgacttct | 172402 | - | see also 4249 line |
| 34311 | VAV3 | NM_006113.3 | 1330 | cagtgatcgtatgtaagagaaaa | 172360 | - | see also 4249 line |
| 34312 | ACBD3 | NM_022735.3 | 400 | aagttcttatgggcccatataat | 172411 | - | see also 8441 line |
| 34313 | ACBD3 | NM_022735.3 | 428 | cacttgtcctgagtttggattct | 172412 | - | see also 8441 line |
| 34314 | ACBD4 | NM_024722.1 | 646 | acccccattggacgatataagtgg | 172441 | - | see also 15067 line |
| 34315 | ACBD4 | NM_024722.1 | 642 | tgggaccccattggacgatataa | 172439 | - | see also 15067 line |
| 34316 | ACBD5 | NM_145698.1 | 615 | aacgccaaaacgttaatggtaa | 172453 | - | see also 9795 line |

Figure 46

| 34317 | ACBD5 | NM_145698.1 | 1588 | tgctaacgtttgccatcatatgg | 172473 | - | see also 9795 line |
|---|---|---|---|---|---|---|---|
| 34318 | ACBD6 | NM_032360.2 | 672 | ggcctgtgatcgaggacataagg | 172491 | - | see also 9172 line |
| 34319 | ACBD6 | NM_032360.2 | 412 | caggaatatatcgcagtagttaa | 172480 | - | see also 9172 line |
| 34320 | PECI | NM_006117.1 | 601 | ctgactaacttcactgatattcc | 172513 | - | see also 4254 line |
| 34321 | PECI | NM_006117.1 | 243 | aggtgtatttgacttgatcaaca | 172501 | - | see also 4254 line |
| 34322 | NDUFAB1 | NM_005003.2 | 266 | caggaccgttctttacgtatt | 172526 | - | see also 15071 line |
| 34323 | NDUFAB1 | NM_005003.2 | 270 | accgtgtctttacgtattgaaa | 172528 | - | see also 15071 line |
| 34324 | CPNE1 | NM_003915.2 | 1308 | gtccaacagggtcaatgagtac | 172550 | - | see also 2673 line |
| 34325 | CPNE1 | NM_003915.2 | 1011 | tgcaatgctccgattatgacagt | 172543 | - | see also 2673 line |
| 34326 | CPNE3 | NM_003909.1 | 1064 | gccccaatggcgttaatgagtat | 172591 | - | see also 2668 line |
| 34327 | DOC2B | NM_003585.1 | 1058 | tcggaccctacgtgaaaacata | 172621 | - | see also 2379 line |
| 34328 | DOC2B | NM_003585.1 | 749 | tactacgggatcacagatgaaga | 172620 | - | see also 2379 line |
| 34329 | DOC2B | NM_003585.1 | 1233 | ttggaaaatccaacgatttcatt | 172625 | - | see also 2379 line |
| 34330 | PEPP2 | NM_019012.2 | 1205 | aggccgatcgagtcatcacagaga | 172662 | - | see also 7661 line |
| 34331 | PEPP2 | NM_019012.2 | 353 | aacggccaataagtatgataaat | 172637 | - | see also 7661 line |
| 34332 | PIK3R1 | NM_181504.2 | 468 | aaccactaccggaatgaatctct | 172724 | - | see also 15075 line |
| 34333 | PIK3R1 | NM_181504.2 | 1035 | aacgagtggttgggcaatgaaaa | 172743 | - | see also 15075 line |
| 34334 | PLEKHA4 | NM_020904.1 | 1336 | acccgttgagtcgcattgatgt | 172797 | - | see also 15076 line |
| 34335 | PLEKHA4 | NM_020904.1 | 1338 | ccgttggtcgcattgatgtcc | 172798 | - | see also 15076 line |
| 34336 | PLEKHA4 | NM_020904.1 | 2574 | ggcgtcgcagtggcacagaatga | 172804 | - | see also 15076 line |
| 34337 | PSCD3 | NM_004227.3 | 400 | aagacgtcgccagttccttat | 172812 | - | see also 15077 line |
| 34338 | PSCD3 | NM_004227.3 | 178 | tagacattcgtcgagaagaaaag | 172808 | - | see also 15077 line |
| 34339 | TULP3 | NM_003324.2 | 358 | ctgaaaccagacagagttcatgc | 172828 | - | see also 15078 line |
| 34340 | TULP3 | NM_003324.2 | 735 | cccacctactatatgtacttgg | 172835 | - | see also 15078 line |
| 34341 | DAPP1 | NM_014395.1 | 697 | cggatcctagacctaacagaatg | 172853 | - | see also 5658 line |
| 34342 | DAPP1 | NM_014395.1 | 303 | gaggccaaagattctgttaaac | 172846 | - | see also 5658 line |
| 34343 | LYPLA3 | NM_012320.2 | 1097 | ctgaccgtgaccctaaaatctgc | 172879 | - | see also 15080 line |
| 34344 | LYPLA3 | NM_012320.2 | 258 | gaccgaaagctacttcacaatct | 172860 | - | see also 15080 line |
| 34345 | OSBPL1A | NM_018030.3 | 1682 | tgccacgtttgacgcttacaaaa | 172908 | - | see also 15081 line |
| 34346 | OSBPL1A | NM_018030.3 | 1683 | gccacgtttgacgcttacaaaaa | 172909 | - | see also 15081 line |
| 34347 | OSBPL1A | NM_018030.3 | 1057 | tcctacagcgcctaactgaattac | 172885 | - | see also 15081 line |
| 34348 | PAFAH2 | NM_000437.2 | 388 | tggccgagtacctgccagtttaat | 172963 | - | see also 15082 line |
| 34349 | PAFAH2 | NM_000437.2 | 356 | gttggattccccgctatgagtact | 172962 | - | see also 15082 line |
| 34350 | PLA2G7 | NM_005084.2 | 406 | tccgttggttgtacagacttaat | 172992 | - | see also 15083 line |
| 34351 | PLA2G7 | NM_005084.2 | 1040 | gttggagcaacggttattcagact | 173013 | - | see also 15083 line |
| 34352 | PLEKHA1 | NM_021622.2 | 2250 | aacgtctgaactttcatgtgc | 173052 | - | see also 8151 line |
| 34353 | PLEKHA1 | NM_021622.2 | 2149 | aaggaaacctcttcgtgtaatacc | 173045 | - | see also 8151 line |

Figure 46

| | | | | | | | | | |
|---|---|---|---|---|---|---|---|---|---|
| 34354 | PLEKHA3 | NM_019091.2 | 803 | accatccggatcccttagtttct | 173069 | - | see also 15085 line | | |
| 34355 | PLEKHA3 | NM_019091.2 | 802 | caccatccggatcccttagtttc | 173068 | - | see also 15085 line | | |
| 34356 | PLEKHA3 | NM_019091.2 | 738 | cacaacgcttgaggaatgtgtga | 173066 | - | see also 15085 line | | |
| 34357 | RASAL1 | NM_004658.1 | 1335 | tccaagtcgatggaacagtttat | 173080 | - | see also 15086 line | | |
| 34358 | RASAL1 | NM_004658.1 | 756 | gacccatttgcacgtgtgttttg | 173075 | - | see also 15086 line | | |
| 34359 | SDPR | NM_004657.3 | 582 | agctcctccgacgcaaccatttc | 173089 | - | see also 15087 line | | |
| 34360 | SDPR | NM_004657.3 | 914 | gggacaaagatcgtatctgtaga | 173096 | - | see also 15087 line | | |
| 34361 | SEC14L2 | NM_012429.1 | 1006 | atgaacacagcgtgcagatttcc | 173115 | - | see also 15088 line | | |
| 34362 | SEC14L2 | NM_012429.1 | 607 | aggtggagaccatcaccataatt | 173106 | - | see also 15088 line | | |
| 34363 | SYT1 | NM_005639.1 | 220 | gccatagtcgcagtccttttagt | 173122 | - | see also 3914 line | | |
| 34364 | SYT1 | NM_005639.1 | 198 | accgtgggccttaattgcaatag | 173119 | - | see also 3914 line | | |
| 34365 | FABP2 | NM_000134.2 | 90 | accggagtgaaaactatgacaag | 173136 | - | see also 15090 line | | |
| 34366 | FABP2 | NM_000134.2 | 228 | ttcgaaacattgaagttgttttt | 173140 | - | see also 15090 line | | |
| 34367 | FABP3 | NM_004102.2 | 354 | cacacttgtgcgggagctaattg | 173150 | - | see also 15091 line | | |
| 34368 | FABP3 | NM_004102.2 | 74 | agctagtggacagcaagaatttc | 173145 | - | see also 15091 line | | |
| 34369 | FABP3 | NM_004102.2 | 162 | taccacaatcatcgaaaagaatg | 173148 | - | see also 15091 line | | |
| 34370 | FABP4 | NM_001442.1 | 414 | gtcacttccacgagagtttatga | 173160 | - | see also 15092 line | | |
| 34371 | FABP4 | NM_001442.1 | 416 | cacttccacgagagtttatgaga | 173161 | - | see also 15092 line | | |
| 34372 | FABP6 | NM_001445.1 | 198 | atctccagcgatgtaatcgaaaa | 173164 | - | see also 15093 line | | |
| 34373 | FABP6 | NM_001445.1 | 193 | ttgggatctccagcgatgtaatc | 173162 | - | see also 15093 line | | |
| 34374 | RBP3 | NM_002900.1 | 1446 | ctgcgcttcgatagttttgctga | 173168 | - | see also 1939 line | | |
| 34375 | RBP3 | NM_002900.1 | 3572 | cgcggaggagttcacctatatca | 173184 | - | see also 1939 line | | |
| 34376 | ALB | NM_000477.3 | 882 | ggcggaccttgccaagtatatct | 173203 | - | see also 15095 line | | |
| 34377 | ALB | NM_000477.3 | 1697 | ttgttgagctcgtgaaacacaag | 173221 | - | see also 15095 line | | |
| 34378 | APOA1 | NM_000039.1 | 212 | aggctccgccttgggaaaacagc | 173224 | - | - | Retinoic acid receptor RXR-alpha | atherosclerosis、amyloidosis、hypoalphalipoproteinemia、diabetes |
| 34379 | APOA1 | NM_000039.1 | 805 | ctctcgaggagtacactaagaag | 173228 | - | see also 34378 line | | |
| 34380 | APOA1 | NM_000039.1 | 192 | gactatgtgtcccagtttgaagg | 173223 | - | see also 34378 line | | |
| 34381 | APOA4 | NM_000482.2 | 720 | gccctacgctgacgaattcaaag | 173232 | - | see also 15096 line | | |
| 34382 | APOA4 | NM_000482.2 | 719 | cgccctacgctgacgaattcaaa | 173231 | - | see also 15096 line | | |
| 34383 | APOA4 | NM_000482.2 | 717 | tacgccctacgctgacgaattca | 173230 | - | see also 15096 line | | |
| 34384 | APOA5 | NM_052968.2 | 193 | aagacctcaacaatatgaacaag | 173237 | - | see also 15097 line | | |
| 34385 | APOA5 | NM_052968.2 | 981 | gccccagagtttcaacaaacaga | 173240 | - | see also 15097 line | | |
| 34386 | APOD | NM_001647.1 | 188 | ctcggaagatggtacgaaattga | 173245 | - | see also 1091 line | | |
| 34387 | APOD | NM_001647.1 | 272 | aacggaaagatcaaagtgttaaa | 173249 | - | see also 1091 line | | |
| 34388 | ATP5G1 | NM_005175.1 | 204 | ttcttgaatagcccagtgaattc | 173251 | - | see also 15098 line | | |
| 34389 | ATP5G1 | NM_005175.1 | 292 | ttgtctcccgggacattgacaca | 173252 | - | see also 15098 line | | |

## Figure 46

| 34390 | ATP5G2 | NM_005176.3 | 431 | ggccatggggctctttgtctga | 173254 | - | see also 15099 line |
|-------|--------|-------------|-----|------------------------|--------|---|---------------------|
| 34391 | ATP5G3 | NM_001689.1 | 447 | atcagcagagacattgatactgc | 173260 | - | see also 15100 line |
| 34392 | ATP5G3 | NM_001689.1 | 299 | agctggatccagagttgcataca | 173255 | - | see also 15100 line |
| 34393 | BPI | NM_001725.1 | 335 | tgcccaatgtgggccttaagttc | 173266 | - | see also 15101 line |
| 34394 | BPI | NM_001725.1 | 336 | gcccaatgtgggccttaagttct | 173267 | - | see also 15101 line |
| 34395 | BPI | NM_001725.1 | 612 | gtctgcgcttcgaaacaagatga | 173272 | - | see also 15101 line |
| 34396 | BPIL1 | NM_025227.1 | 266 | caccgtggtccgactcaacaagg | 173290 | - | see also 15102 line |
| 34397 | BPIL1 | NM_025227.1 | 1528 | agggctacgtggtgatatccagt | 173302 | - | see also 15102 line |
| 34398 | BPIL2 | NM_174932.1 | 840 | tggaatcgccgagtatttcttta | 173314 | - | see also 15103 line |
| 34399 | BPIL2 | NM_174932.1 | 412 | gtctactttaccggtatcattat | 173309 | - | see also 15103 line |
| 34400 | BPIL3 | NM_174897.1 | 1170 | atcgattggcaacttcaatgaga | 173351 | - | see also 10124 line |
| 34401 | BPIL3 | NM_174897.1 | 595 | cagatgggcaccgtcaaatatgt | 173343 | - | see also 10124 line |
| 34402 | C20orf114 | NM_033197.2 | 1115 | gtcaagcatcgggctgatcaatg | 173365 | - | see also 15105 line |
| 34403 | C20orf114 | NM_033197.2 | 1119 | agcatcgggctgatcaatgaaaa | 173367 | - | see also 15105 line |
| 34404 | CETP | NM_000078.1 | 1449 | ctcggctcgaggtagtgtttaca | 173395 | - | see also 15106 line |
| 34405 | CETP | NM_000078.1 | 1447 | gtctcggctcgaggtagtgttta | 173394 | - | see also 15106 line |
| 34406 | CRABP1 | NM_004378.1 | 412 | agctggccaacgatgaacttatc | 173403 | - | see also 2935 line |
| 34407 | CRABP1 | NM_004378.1 | 416 | ggccaacgatgaacttatcctga | 173404 | - | see also 2935 line |
| 34408 | CRABP2 | NM_001878.2 | 309 | accgtgcgcaccacagagattaa | 173406 | - | see also 15107 line |
| 34409 | CRABP2 | NM_001878.2 | 479 | gaccaacgatggggaactgatcc | 173408 | - | see also 15107 line |
| 34410 | CRABP2 | NM_001878.2 | 173 | atcggaaaacttcgaggaattgc | 173405 | - | see also 15107 line |
| 34411 | CRBPIV | NM_052960.1 | 122 | gactttgccactcgtaaaatagc | 173411 | - | see also 15108 line |
| 34412 | CRBPIV | NM_052960.1 | 171 | ttgagcagaatggggattctttt | 173412 | - | see also 15108 line |
| 34413 | EDG3 | NM_005226.2 | 664 | atcgcttacaaggtcaacattct | 173413 | - | see also 3663 line |
| 34414 | EDG3 | NM_005226.2 | 827 | ggccttacgacgccaacaagagg | 173419 | - | see also 3663 line |
| 34415 | EDG5 | NM_004230.1 | 549 | gcctctctacgccaagcattatg | 173434 | - | see also 15110 line |
| 34416 | EDG5 | NM_004230.1 | 548 | tgcctctctacgccaagcattat | 173433 | - | see also 15110 line |
| 34417 | EDG6 | NM_003775.1 | 250 | gtcgcgacgctgggtctactatt | 173438 | - | see also 15111 line |
| 34418 | EDG6 | NM_003775.1 | 252 | cgcgacgctgggtctactattgc | 173440 | - | see also 15111 line |
| 34419 | EDG7 | NM_012152.1 | 265 | gccgatttcttcgctggaattgc | 173442 | - | see also 15112 line |
| 34420 | EDG7 | NM_012152.1 | 697 | gtcttgtctccgcatacaagtgg | 173448 | - | see also 15112 line |
| 34421 | FABP1 | NM_001443.1 | 208 | tccaaagtgatccaaaacgaatt | 173456 | - | see also 15113 line |
| 34422 | FABP1 | NM_001443.1 | 356 | acggcgacataatcaccaatacc | 173459 | - | see also 15113 line |
| 34423 | FABP7 | NM_001446.3 | 402 | gaccaaaccaacggtaattatca | 173463 | - | see also 15114 line |
| 34424 | FABP7 | NM_001446.3 | 666 | tgctgttcgccactatgagaagg | 173471 | - | see also 15114 line |
| 34425 | LBP | NM_004139.2 | 644 | atcggtgtcctccgatctacagc | 173477 | - | see also 15115 line |
| 34426 | LBP | NM_004139.2 | 653 | ctccgatctacagccttatctcc | 173478 | - | see also 15115 line |

Figure 46

| 34427 | LIPF | NM_004190.1 | 862 | gtcgcttggatgtgtatctatca | 173521 | - | see also 15116 line |
|---|---|---|---|---|---|---|---|
| 34428 | LIPF | NM_004190.1 | 497 | agccacaatcgacttcattgtaa | 173515 | - | see also 15116 line |
| 34429 | LIPF | NM_004190.1 | 429 | tactattcaccagattcagttga | 173513 | - | see also 15116 line |
| 34430 | MTP | NM_000253.1 | 1960 | cccgttcggcatctacttacagc | 173581 | - | see also 194 line |
| 34431 | MTP | NM_000253.1 | 1312 | gagccctcattagtaagttcaaa | 173565 | - | see also 194 line |
| 34432 | PCTP | NM_021213.1 | 597 | tggagttcctaacttcttgaaag | 173620 | - | see also 15117 line |
| 34433 | PCTP | NM_021213.1 | 342 | gtccaacagagactatgtctacc | 173615 | - | see also 15117 line |
| 34434 | PITPN | NM_006224.2 | 632 | aacacgtggaagccgtatatata | 173630 | - | see also 4317 line |
| 34435 | PITPN | NM_006224.2 | 634 | cacgtggaagccgtatatataga | 173631 | - | see also 4317 line |
| 34436 | PITPNB | NM_012399.2 | 201 | gggacagtatacgcacaaaattt | 173646 | - | see also 15119 line |
| 34437 | PITPNB | NM_012399.2 | 203 | gacagtatacgcacaaaatttat | 173647 | - | see also 15119 line |
| 34438 | PITPNB | NM_012399.2 | 205 | cagtatacgcacaaaatttatca | 173648 | - | see also 15119 line |
| 34439 | PLIN | NM_002666.1 | 440 | cagtaccccctgaaaagattgc | 173660 | - | see also 15120 line |
| 34440 | PLIN | NM_002666.1 | 282 | ctgtgtgcaatgcctatgagaag | 173658 | - | see also 15120 line |
| 34441 | PLTP | NM_006227.2 | 1249 | tgcgcaggttccgaatctattcc | 173671 | - | see also 15121 line |
| 34442 | PLTP | NM_006227.2 | 1253 | caggttccgaatctattccaacc | 173672 | - | see also 15121 line |
| 34443 | PMP2 | NM_002677.2 | 179 | gtggggttagccaccagaaaact | 173676 | - | see also 15122 line |
| 34444 | PMP2 | NM_002677.2 | 296 | ttcaagctaggccaggaatttga | 173678 | - | see also 15122 line |
| 34445 | PSAP | NM_002778.1 | 1051 | tactcgacgcttttgacaaaatg | 173690 | - | see also 15123 line |
| 34446 | PSAP | NM_002778.1 | 1458 | ttcgtgtgcttgaaaattggagc | 173693 | - | see also 15123 line |
| 34447 | RBP1 | NM_002899.2 | 496 | gtgtggtctgcaagcaagtattc | 173698 | - | see also 15124 line |
| 34448 | RBP1 | NM_002899.2 | 256 | tgcaggacggtgaccatatgatc | 173694 | - | see also 15124 line |
| 34449 | RBP2 | NM_004164.2 | 429 | gtgccgtcaagtgttcaaaaaga | 173710 | - | see also 15125 line |
| 34450 | RBP2 | NM_004164.2 | 162 | cactcagacgaaggttattgatc | 173700 | - | see also 15125 line |
| 34451 | RBP5 | NM_031491.1 | 325 | aggagcgtggacggacgaaaatg | 173711 | - | - | retinol binding | - |
| 34452 | RRAGC | NM_022157.2 | 735 | cacaactgccgaccttggaaaac | 173724 | - | see also 8315 line |
| 34453 | RRAGC | NM_022157.2 | 676 | tagtatctatgaccattcaatat | 173722 | - | see also 8315 line |
| 34454 | SCP2 | NM_002979.2 | 240 | gagggctatctatcacagtttgg | 173747 | - | see also 15127 line |
| 34455 | SCP2 | NM_002979.2 | 998 | aaggtgcaacgctggttgataga | 173766 | - | see also 15127 line |
| 34456 | SEC14L3 | NM_174975.2 | 59 | ggccaagttccgagaaaacgtcc | 173785 | - | see also 15128 line |
| 34457 | SEC14L3 | NM_174975.2 | 136 | tccgagctcggaattttgacttg | 173787 | - | see also 15128 line |
| 34458 | SEC14L4 | NM_174977.2 | 528 | ggcgctgatggtgtttgacatgg | 173812 | - | see also 15129 line |
| 34459 | SEC14L4 | NM_174977.2 | 446 | tccggaagcgcatcaaagtctgt | 173811 | - | see also 15129 line |
| 34460 | STAR | NM_000349.1 | 258 | ccctagcacgtggattaaccagg | 173817 | - | see also 15130 line |
| 34461 | STAR | NM_000349.1 | 255 | cacccctagcacgtggattaacc | 173816 | - | see also 15130 line |
| 34462 | STARD3 | NM_006804.2 | 1188 | ggcgcagggaccgatacttgtca | 173832 | - | see also 4784 line |
| 34463 | STARD3 | NM_006804.2 | 731 | tccgagggacagttctattcacc | 173827 | - | see also 4784 line |

Figure 46

| 34464 | STARD3 | NM_006804.2 | 932 | accgtgtacaccattgaagttcc | 173830 | - | see also 4784 line |
|---|---|---|---|---|---|---|---|
| 34465 | STARD4 | NM_139164.1 | 670 | aacttctatggtgatttacgaaa | 173853 | - | see also 15132 line |
| 34466 | STARD4 | NM_139164.1 | 673 | ttctatggtgatttacgaaaagc | 173854 | - | see also 15132 line |
| 34467 | STARD5 | NM_030574.2 | 186 | accgaggagaaggcattgtatat | 173855 | - | see also 8891 line |
| 34468 | STARD6 | NM_139171.1 | 318 | ttcccctcgagactttatcgact | 173875 | - | see also 15133 line |
| 34469 | STARD6 | NM_139171.1 | 353 | agcgctacgaaggaaatatgaac | 173878 | - | see also 15133 line |
| 34470 | STARD6 | NM_139171.1 | 326 | gagactttatcgacttagtgtac | 173876 | - | see also 15133 line |
| 34471 | CD209 | NM_021155.2 | 1102 | aacgacgacaaatgtaatcttgc | 173898 | - | see also 15134 line |
| 34472 | CD209 | NM_021155.2 | 1096 | ggctggaacgacgacaaatgtaa | 173895 | - | see also 15134 line |
| 34473 | LMAN2L | NM_030805.1 | 534 | agcgggtattccctacatctca | 173907 | - | see also 15135 line |
| 34474 | LMAN2L | NM_030805.1 | 162 | gtcaaacgttcgagtacttgaaa | 173904 | - | see also 15135 line |
| 34475 | MBL2 | NM_000242.1 | 702 | cccaacaatgctggttctgatga | 173923 | - | see also 15136 line |
| 34476 | MBL2 | NM_000242.1 | 415 | cagaaatggcacgtatcaaaaag | 173922 | - | see also 15136 line |
| 34477 | APCS | NM_001639.2 | 256 | ttgtgttttcgagcctatagtga | 173933 | - | see also 15137 line |
| 34478 | APCS | NM_001639.2 | 351 | aagagttggagagtatagtctat | 173937 | - | see also 15137 line |
| 34479 | ASGR1 | NM_001671.2 | 703 | gggctgacgccgacaactactgc | 173945 | - | see also 15138 line |
| 34480 | ASGR2 | NM_001181.2 | 963 | cacagactataggcacaactaca | 173946 | - | receptor_acitivity、sig nal_transduction - - |
| 34481 | ATRN | NM_012070.2 | 3117 | agcactgcgagacctgcatatct | 174029 | - | see also 5106 line |
| 34482 | BCAN | NM_021948.3 | 1617 | ccctaggacgctcctagaatttg | 174054 | - | see also 15140 line |
| 34483 | BCAN | NM_021948.3 | 1616 | cccctaggacgctcctagaattt | 174053 | - | see also 15140 line |
| 34484 | C14orf27 | NM_175060.1 | 1406 | cacgatgtctacccttcaaatgt | 174065 | - | see also 15141 line |
| 34485 | C14orf27 | NM_175060.1 | 1019 | tacttgcatcgcggacgaaatcg | 174062 | - | see also 15141 line |
| 34486 | C21orf63 | NM_058187.3 | 644 | tcggcattctacgataagtgtcc | 174081 | - | see also 15142 line |
| 34487 | C21orf63 | NM_058187.3 | 639 | tgccctcggcattctacgataag | 174079 | - | see also 15142 line |
| 34488 | CD207 | NM_015717.1 | 219 | ctgctgcaggccgtcctttatcc | 174101 | - | see also 15143 line |
| 34489 | CD207 | NM_015717.1 | 165 | cccacagtccgtgctgcattaat | 174099 | - | see also 15143 line |
| 34490 | CD209L | NM_014257.2 | 1120 | atcgatgtgacgttgacaattac | 174118 | - | see also 15144 line |
| 34491 | CD209L | NM_014257.2 | 1121 | tcgatgtgacgttgacaattact | 174119 | - | see also 15144 line |
| 34492 | CD22 | NM_001771.1 | 2423 | ctgcgatgacacggtcacttatt | 174155 | - | see also 15145 line |
| 34493 | CD22 | NM_001771.1 | 445 | aacgaatacacctcaatgtctct | 174126 | - | see also 15145 line |
| 34494 | CD33 | NM_001772.1 | 231 | ggccacaaacaagctagatcaag | 174162 | - | see also 15146 line |
| 34495 | CD33 | NM_001772.1 | 232 | gccacaaacaagctagatcaaga | 174163 | - | see also 15146 line |
| 34496 | CD69 | NM_001781.1 | 284 | gtccaggccaatacacattctca | 174171 | - | see also 15147 line |
| 34497 | CD69 | NM_001781.1 | 198 | tcctgtcctgtgtgctgtaatga | 174170 | - | see also 15147 line |
| 34498 | CHODL | NM_024944.2 | 1060 | aacaatacccctgctcttactga | 174189 | - | see also 15148 line |
| 34499 | CHODL | NM_024944.2 | 659 | cccgggacagggatttctgatgg | 174179 | - | see also 15148 line |

Figure 46

| 34500 | CHST4 | NM_005769.1 | 1042 | ttcccgaatgtatgaattcgtgg | 174207 | - | see also 15149 line |
|---|---|---|---|---|---|---|---|
| 34501 | CHST4 | NM_005769.1 | 1256 | taccgccacgtcagatctgaaca | 174214 | - | see also 15149 line |
| 34502 | CLEC1 | NM_016511.2 | 443 | aactctgtcgtgagctgtataac | 174222 | - | see also 15150 line |
| 34503 | CLEC1 | NM_016511.2 | 428 | agcatgtggctgaaaaactctgt | 174221 | - | see also 15150 line |
| 34504 | CLEC2 | NM_016509.1 | 223 | gtcatgcagcgcaattacctaca | 174229 | - | see also 15151 line |
| 34505 | CLEC2 | NM_016509.1 | 219 | gtctgtcatgcagcgcaattacc | 174228 | - | see also 15151 line |
| 34506 | CLECSF1 | NM_005752.1 | 410 | atcaacgccctccaagactatgg | 174242 | - | see also 15152 line |
| 34507 | CLECSF1 | NM_005752.1 | 415 | cgccctccaagactatggtaaaa | 174243 | - | see also 15152 line |
| 34508 | CLECSF11 | NM_130441.1 | 654 | ctgtccaagttacgagagtatca | 174256 | - | see also 15153 line |
| 34509 | CLECSF11 | NM_130441.1 | 656 | gtccaagttacgagagtatcaac | 174257 | - | see also 15153 line |
| 34510 | CLECSF12 | NM_022570.3 | 210 | cacttcgactctcaaagcaatac | 174275 | - | see also 15154 line |
| 34511 | CLECSF12 | NM_022570.3 | 214 | tcgactctcaaagcaataccagg | 174276 | - | see also 15154 line |
| 34512 | CLECSF13 | NM_006344.1 | 490 | agcagcttggaagaaacgatagc | 174305 | - | see also 15155 line |
| 34513 | CLECSF13 | NM_006344.1 | 210 | gacaaggacgtatgaaaacttcc | 174301 | - | see also 15155 line |
| 34514 | CLECSF13 | NM_173535.1 | 368 | ggcagaaatgcgagagcttatcc | 174315 | - | see also 15156 line |
| 34515 | CLECSF13 | NM_173535.1 | 1343 | gaggttaagaggggatctagaga | 174342 | - | see also 15156 line |
| 34516 | CLECSF2 | NM_005127.2 | 858 | tgccgacctaactataattgaca | 174355 | - | see also 15157 line |
| 34517 | CLECSF2 | NM_005127.2 | 854 | aacatgccgacctaactataatt | 174354 | - | see also 15157 line |
| 34518 | CLECSF5 | NM_013252.2 | 680 | tgcgaccattggcctaacaaaga | 174375 | - | see also 15158 line |
| 34519 | CLECSF5 | NM_013252.2 | 503 | aggatccacattggcaattgtca | 174368 | - | see also 15158 line |
| 34520 | CLECSF6 | NM_016184.2 | 854 | tgcgttgtgctaaattttcgtaa | 174387 | - | see also 15159 line |
| 34521 | CLECSF6 | NM_016184.2 | 849 | agcgctgcgttgtgctaaatttt | 174385 | - | see also 15159 line |
| 34522 | CLECSF8 | NM_080387.4 | 597 | tggatagacggctttcctatttc | 174407 | - | see also 15160 line |
| 34523 | CLECSF8 | NM_080387.4 | 464 | ttccagtccaactgctattttcc | 174404 | - | see also 15160 line |
| 34524 | CLECSF9 | NM_014358.1 | 609 | tggacggcacacctttgacaaag | 174423 | - | see also 15161 line |
| 34525 | CLECSF9 | NM_014358.1 | 448 | ttcctgggcgttaagtttaaaga | 174419 | - | see also 15161 line |
| 34526 | CLL-1 | NM_138337.2 | 597 | tggcataaggacagctgttattt | 174433 | - | see also 15162 line |
| 34527 | CLL-1 | NM_138337.2 | 729 | cagagtagatcatatgactattg | 174440 | - | see also 15162 line |
| 34528 | COLEC10 | NM_006438.2 | 482 | cccggctcaagacatctatgaag | 174457 | - | see also 15163 line |
| 34529 | COLEC10 | NM_006438.2 | 852 | cacagagtgccatcttaccatgt | 174466 | - | see also 15163 line |
| 34530 | COLEC11 | NM_024027.3 | 165 | ccctggtgggcgttctaatcagc | 174469 | - | - |
| 34531 | COLEC11 | NM_024027.3 | 163 | ggccctggtgggcgttctaatca | 174468 | - | see also 34530 line |
| 34532 | COLEC11 | NM_024027.3 | 808 | ccccatgcggaccttcaacaagt | 174471 | - | see also 34530 line |
| 34533 | COLEC12 | NM_030781.2 | 393 | atctcgccaaacctatgatgaca | 174479 | - | see also 15164 line |
| 34534 | COLEC12 | NM_030781.2 | 212 | tcggttacaagcggtttggtatt | 174473 | - | see also 15164 line |
| 34535 | CSAD | NM_015989.2 | 567 | tggtcaccctcggttcttcaacc | 174525 | - | see also 15165 line |
| 34536 | CSAD | NM_015989.2 | 529 | gagcggtgtcgggctgtgattcg | 174524 | - | see also 15165 line |

Figure 46

| 34537 | DCL-1 | NM_014880.3 | 571 | ttgctagcacggtaattttgaca | 174542 | - | see also 15166 line |
|---|---|---|---|---|---|---|---|
| 34538 | DCL-1 | NM_014880.3 | 663 | accgcaccccaatcaccttataa | 174548 | - | see also 15166 line |
| 34539 | DGCR2 | NM_005137.1 | 1475 | acctccctacacggcatacaagt | 174562 | - | see also 3612 line |
| 34540 | DGCR2 | NM_005137.1 | 1478 | tccctacacggcatacaagtacc | 174564 | - | see also 3612 line |
| 34541 | DGCR2 | NM_005137.1 | 1477 | ctccctacacggcatacaagtac | 174563 | - | see also 3612 line |
| 34542 | DTX2 | NM_020892.1 | 2017 | tgcgggaccatcctcatagttta | 174571 | - | see also 15168 line |
| 34543 | DTX2 | NM_020892.1 | 2019 | cgggaccatcctcatagtttaca | 174573 | - | see also 15168 line |
| 34544 | FCN3 | NM_003665.2 | 774 | atcctgttaccgatcaaatctca | 174578 | - | see also 15169 line |
| 34545 | FCN3 | NM_003665.2 | 771 | tgcatcctgttaccgatcaaatc | 174577 | - | see also 15169 line |
| 34546 | GCKR | NM_001486.2 | 1257 | tccctctctcacggaaatcgata | 174596 | - | see also 15170 line |
| 34547 | GCKR | NM_001486.2 | 1258 | ccctctctcacggaaatcgatac | 174597 | - | see also 15170 line |
| 34548 | GCKR | NM_001486.2 | 1312 | ctcacggaggtgcagactatagt | 174598 | - | see also 15170 line |
| 34549 | GFPT1 | NM_002056.1 | 158 | tcctcgaacgagacgagaaatcc | 174602 | - | see also 1329 line |
| 34550 | GFPT1 | NM_002056.1 | 1406 | tactttgatgggtcttcgttact | 174636 | - | see also 1329 line |
| 34551 | GFPT1 | NM_002056.1 | 1410 | ttgatgggtcttcgttactgtaa | 174637 | - | see also 1329 line |
| 34552 | GFPT2 | NM_005110.1 | 1244 | ccgtggctacgcggcaagttttg | 174686 | - | see also 3581 line |
| 34553 | GFPT2 | NM_005110.1 | 1248 | ggctacgcggcaagttttggagg | 174687 | - | see also 3581 line |
| 34554 | ITLN1 | NM_017625.2 | 1008 | agcagcagccgtgagataactga | 174705 | - | see also 15173 line |
| 34555 | ITLN1 | NM_017625.2 | 879 | agggtcaccggatgtaacactga | 174704 | - | see also 15173 line |
| 34556 | ITLN2 | NM_080878.2 | 1008 | aggcggctgtactcttgttctat | 174719 | - | see also 15174 line |
| 34557 | ITLN2 | NM_080878.2 | 1003 | aacggaggcggctgtactcttgt | 174718 | - | see also 15174 line |
| 34558 | KLRB1 | NM_002258.1 | 392 | ctgtcaacccttggaataacagt | 174738 | - | see also 15175 line |
| 34559 | KLRB1 | NM_002258.1 | 556 | aagtggataaacggctctttttt | 174744 | - | see also 15175 line |
| 34560 | KLRC1 | NM_002259.2 | 497 | gcctctgtggtaacgatagttgt | 174757 | - | see also 15176 line |
| 34561 | KLRC1 | NM_002259.2 | 359 | acctatgcggaattaaaccttca | 174752 | - | see also 15176 line |
| 34562 | KLRD1 | NM_002262.2 | 718 | tagcgtataatccaaatggaaat | 174779 | - | see also 15178 line |
| 34563 | KLRD1 | NM_002262.2 | 711 | aactgcatagcgtataatccaaa | 174776 | - | see also 15178 line |
| 34564 | KLRF1 | NM_016523.1 | 158 | gtgacgttgcactggtataaaat | 174786 | - | see also 15179 line |
| 34565 | KLRF1 | NM_016523.1 | 160 | gacgttgcactggtataaaatct | 174787 | - | see also 15179 line |
| 34566 | KLRG1 | NM_005810.2 | 248 | gaccgctggatgaaatatggtaa | 174810 | - | see also 15180 line |
| 34567 | KLRG1 | NM_005810.2 | 575 | aagtgtccctttgcagatcaagc | 174817 | - | see also 15180 line |
| 34568 | KLRK1 | NM_007360.1 | 606 | cccttgaccgaaagttactgtgg | 174827 | - | see also 15181 line |
| 34569 | KLRK1 | NM_007360.1 | 604 | ttcccttgaccgaaagttactgt | 174826 | - | see also 15181 line |
| 34570 | KLRK1 | NM_007360.1 | 863 | acccaacctactaacaataattg | 174836 | - | see also 15181 line |
| 34571 | LGALS1 | NM_002305.2 | 367 | agctgccagatggatacgaattc | 174842 | - | see also 15182 line |
| 34572 | LGALS1 | NM_002305.2 | 434 | agctgacggtgacttcaagatca | 174845 | - | see also 15182 line |
| 34573 | LGALS2 | NM_006498.1 | 185 | caccattgtctgcaactcattgg | 174848 | - | see also 15183 line |

Figure 46

| 34574 | LGALS2 | NM_006498.1 | 401 | gtcctctttcaagttaaaagaat | 174849 | - | see also 15183 line | | |
|-------|--------|-------------|-----|-------------------------|--------|---|---------------------|--|--|
| 34575 | LGALS4 | NM_006149.2 | 475 | gggatctgcaacttcaatcaatc | 174856 | - | see also 15184 line | | |
| 34576 | LGALS4 | NM_006149.2 | 665 | agctcgaagaaccatcatcatca | 174859 | - | see also 15184 line | | |
| 34577 | LGALS7 | NM_002307.1 | 197 | cacgtcggaggtggtcttcaaca | 174864 | - | apoptosis | galactose binding lectin | - |
| 34578 | LGALS8 | NM_006499.2 | 653 | cacctatgacacgcctttcaaaa | 174870 | - | see also 15185 line | | |
| 34579 | LGALS8 | NM_006499.2 | 765 | aggatcggcccagagaaaataga | 174875 | - | see also 15185 line | | |
| 34580 | LGALS9 | NM_002308.2 | 832 | agcgaagtctgccccgaaaaatg | 174886 | - | see also 15186 line | | |
| 34581 | LGALS9 | NM_002308.2 | 928 | gtcagcacctgtttgaatactac | 174888 | - | see also 15186 line | | |
| 34582 | LLT1 | NM_013269.1 | 109 | taccttaatttggcgcttatttt | 174892 | - | see also 15187 line | | |
| 34583 | LLT1 | NM_013269.1 | 493 | aggagagtgtgcctatttgaatg | 174903 | - | see also 15187 line | | |
| 34584 | LMAN1 | NM_005570.2 | 1060 | agctgaaccggcagttagatatg | 174931 | - | see also 3869 line | | |
| 34585 | LMAN1 | NM_005570.2 | 621 | gtccgagcaaagattacctatta | 174917 | - | see also 3869 line | | |
| 34586 | LMAN1L | NM_021819.1 | 121 | ctcctctacgcaggtttgagtac | 174959 | - | see also 15189 line | | |
| 34587 | LMAN1L | NM_021819.1 | 122 | tcctctacgcaggtttgagtaca | 174960 | - | see also 15189 line | | |
| 34588 | LMAN2 | NM_006816.1 | 512 | agcgcgtgttcccgtacatctcg | 174975 | - | see also 15190 line | | |
| 34589 | LMAN2 | NM_006816.1 | 778 | ggcgacctgtctgacaatcatga | 174978 | - | see also 15190 line | | |
| 34590 | LOC143903 | NM_178834.2 | 484 | tacttctcgaagactgaactttg | 174984 | - | see also 15191 line | | |
| 34591 | LOC143903 | NM_178834.2 | 487 | ttctcgaagactgaactttgagg | 174985 | - | see also 15191 line | | |
| 34592 | LPHN1 | NM_014921.1 | 1439 | ctgtacgtctggaacaactattt | 175011 | - | see also 15192 line | | |
| 34593 | LPHN1 | NM_014921.1 | 3035 | gggatcgacaagactcagtatga | 175016 | - | see also 15192 line | | |
| 34594 | LPHN2 | NM_012302.1 | 744 | tccctatcgtaccgatactttaa | 175049 | - | see also 15193 line | | |
| 34595 | LPHN2 | NM_012302.1 | 2274 | gtccgcaaataccgtcaaacaga | 175104 | - | see also 15193 line | | |
| 34596 | LPHN2 | NM_012302.1 | 1135 | gacaaacgtgccgcatcaaatgc | 175067 | - | see also 15193 line | | |
| 34597 | LPHN3 | NM_015236.1 | 4043 | gtggaatgacacggttcgaaagc | 175282 | - | see also 15194 line | | |
| 34598 | LPHN3 | NM_015236.1 | 2975 | gagctactccaagcgtacaatga | 175244 | - | see also 15194 line | | |
| 34599 | LY6G5C | NM_025262.1 | 65 | ccctctacacggtcctcttaata | 175292 | - | see also 15195 line | | |
| 34600 | LY6G5C | NM_025262.1 | 67 | ctctacacggtcctcttaatagt | 175293 | - | see also 15195 line | | |
| 34601 | LY6G5C | NM_025262.1 | 24 | ggcgctggaccctgctatactgg | 175291 | - | see also 15195 line | | |
| 34602 | LY75 | NM_002349.1 | 4759 | ctgcaactatcgtttccataaaa | 175452 | - | see also 1518 line | | |
| 34603 | LY75 | NM_002349.1 | 1755 | gagtataactgggcaactgttgg | 175345 | - | see also 1518 line | | |
| 34604 | MAG | NM_002361.2 | 1168 | gggagacggtctctatcttgtgc | 175476 | - | see also 15197 line | | |
| 34605 | MAG | NM_002361.2 | 313 | tgcatggtgtctggtacttcaat | 175472 | - | see also 15197 line | | |
| 34606 | MRC2 | NM_006039.1 | 356 | ctcccgaaaccggctattcaacc | 175481 | - | see also 15198 line | | |
| 34607 | MRC2 | NM_006039.1 | 1436 | gtggatcggcctcaacgatttga | 175491 | - | see also 15198 line | | |

Figure 46

| 34608 | NALP9 | NM_176820.2 | 887 | ttgcggcatccaaaactcataaa | 175521 | - | see also 15199 line |
|---|---|---|---|---|---|---|---|
| 34609 | NALP9 | NM_176820.2 | 1494 | cccaggtgggatattcatgtt | 175538 | - | see also 15199 line |
| 34610 | NPTX2 | NM_002523.1 | 804 | gcgaggcaatagcgcctttaagt | 175560 | - | see also 1620 line |
| 34611 | NPTX2 | NM_002523.1 | 1306 | cgcgtccttcgcgcacaagaaat | 175570 | - | see also 1620 line |
| 34612 | NRIP3 | NM_020645.1 | 319 | ggcctctaagacgaacaaactca | 175580 | - | see also 15201 line |
| 34613 | NRIP3 | NM_020645.1 | 322 | ctctaagacgaacaaactcaatc | 175581 | - | see also 15201 line |
| 34614 | OLR1 | NM_002543.2 | 724 | tccttgatgcccacttattta | 175595 | - | see also 15202 line |
| 34615 | OLR1 | NM_002543.2 | 723 | ctcctttgatgcccacttattt | 175594 | - | see also 15202 line |
| 34616 | OLR1 | NM_002543.2 | 191 | gtccttgcctggattagtagt | 175586 | - | see also 15202 line |
| 34617 | PLA2R1 | NM_007366.2 | 825 | gccacgacaagccgttatgaaag | 175615 | - | see also 15203 line |
| 34618 | PLA2R1 | NM_007366.2 | 824 | tgccacgacaagccgttatgaaa | 175614 | - | see also 15203 line |
| 34619 | PRG2 | NM_002728.3 | 461 | aacttcaattaattatcgaat | 175727 | - | see also 15204 line |
| 34620 | PRG2 | NM_002728.3 | 455 | atccacaacttcaatattaatta | 175726 | - | see also 15204 line |
| 34621 | PRG2 | NM_002728.3 | 451 | ttccatccacaacttcaatatta | 175725 | - | see also 15204 line |
| 34622 | PRG3 | NM_006093.2 | 450 | ttggtgcggactcctaaaacttt | 175731 | - | see also 15205 line |
| 34623 | PRG3 | NM_006093.2 | 451 | tggtgcggactcctaaaacttt | 175732 | - | see also 15205 line |
| 34624 | PRG3 | NM_006093.2 | 521 | ctctatccatgacttcaacttca | 175735 | - | see also 15205 line |
| 34625 | REG4 | NM_032044.2 | 652 | gtgctgagatgagctccaataac | 175749 | - | see also 15206 line |
| 34626 | REG4 | NM_032044.2 | 585 | tggattgatggggccatgtatct | 175747 | - | see also 15206 line |
| 34627 | SELE | NM_000450.1 | 1233 | cccgagcgaggctacatggaattg | 175772 | - | see also 15207 line |
| 34628 | SELE | NM_000450.1 | 1232 | ccccgagcgaggctacatggaatt | 175771 | - | see also 15207 line |
| 34629 | SELL | NM_000655.2 | 254 | ttctgccgagacaattacacaga | 175787 | - | see also 15208 line |
| 34630 | SELL | NM_000655.2 | 841 | accaacctgtcaagtgattcagt | 175798 | - | see also 15208 line |
| 34631 | SELP | NM_003005.2 | 1369 | ccgatatagttcggtgtgataac | 175833 | - | see also 15209 line |
| 34632 | SELP | NM_003005.2 | 555 | aagcacgcattgttacacagc | 175820 | - | see also 15209 line |
| 34633 | SFTPD | NM_003019.2 | 820 | gtgtcgggagaagattttcaag | 175858 | - | see also 15210 line |
| 34634 | SFTPD | NM_003019.2 | 779 | ctctcagtataagaaagttgagc | 175857 | - | see also 15210 line |
| 34635 | SIGLEC10 | NM_033130.2 | 1783 | atcggcatcacggctcttcttt | 175862 | - | see also 15211 line |
| 34636 | SIGLEC10 | NM_033130.2 | 2181 | ggccgattatgcagaagtcaagt | 175867 | - | see also 15211 line |
| 34637 | SIGLEC10 | NM_033130.2 | 1784 | tcggcatcacggctctcttc | 175863 | - | see also 15211 line |
| 34638 | SIGLEC11 | NM_052884.1 | 518 | ttcctgagcaatgcgttcttct | 175868 | - | see also 15212 line |
| 34639 | SIGLEC11 | NM_052884.1 | 2137 | aggctttggcttcaattggaga | 175871 | - | see also 15212 line |
| 34640 | SIGLEC5 | NM_003830.1 | 893 | ctgcaaaacacctcatcaccttcc | 175872 | - | - |
| 34641 | SIGLEC5 | NM_003830.1 | 1097 | ccgctgggcttcctgcaaattt | 175873 | - | see also 34640 line |
| 34642 | SIGLEC5 | NM_003830.1 | 1370 | tgcaaggcctggaacatctatgg | 175874 | - | see also 34640 line |
| 34643 | SIGLEC6 | NM_001245.2 | 333 | tccagcctcgtactatggttatg | 175877 | - | see also 15213 line |
| 34644 | SIGLEC6 | NM_001245.2 | 332 | ttccagcctgctactatggttat | 175876 | - | see also 15213 line |

Figure 46

| 34645 | SIGLEC6 | NM_001245.2 | 328 | acccttccagcctcgtactatgg | 175875 | - | see also 15213 line | | |
|---|---|---|---|---|---|---|---|---|---|
| 34646 | SIGLEC7 | NM_014385.1 | 1296 | ctgagtcctgggcagatgataac | 175882 | - | see also 15214 line | | |
| 34647 | SIGLEC8 | NM_014442.1 | 120 | cagacaatatggggatggttact | 175883 | - | see also 15215 line | | |
| 34648 | SIGLECL1 | NM_033329.1 | 729 | agggctgtccgactcaacatatc | 175894 | - | see also 15216 line | | |
| 34649 | SIGLECL1 | NM_033329.1 | 730 | gggctgtccgactcaacatatcc | 175895 | - | see also 15216 line | | |
| 34650 | SN | NM_023068.2 | 837 | tggggtacgcctccaaaccaaga | 175910 | - | see also 15217 line | | |
| 34651 | SN | NM_023068.2 | 1206 | ccctgtcagcgtggtagtcaacc | 175911 | - | see also 15217 line | | |
| 34652 | TNA | NM_003278.1 | 542 | cccgcatcgcctacaagaactgg | 175937 | - | see also 15218 line | | |
| 34653 | ZDHHC19 | NM_144637.2 | 847 | agcaactggtatttaacaatttg | 175942 | - | see also 15219 line | | |
| 34654 | ZDHHC19 | NM_144637.2 | 804 | cagacaccttcagggatacaacc | 175940 | - | see also 15219 line | | |
| 34655 | CHIA | NM_021797.1 | 953 | agggcaagtttccctaatctcc | 175948 | - | see also 15220 line | | |
| 34656 | CHIA | NM_021797.1 | 1139 | gcctctaccccgtggcaaataac | 175950 | - | see also 15220 line | | |
| 34657 | CHIT1 | NM_003465.1 | 591 | tggatacgaggtggacaaaatcg | 175952 | - | see also 15221 line | | |
| 34658 | CHIT1 | NM_003465.1 | 907 | gggatgctggcctactatgaagt | 175955 | - | see also 15221 line | | |
| 34659 | TACR1 | NM_001058.2 | 856 | tacaccgtagtgggaatcacact | 175973 | - | see also 650 line | | |
| 34660 | TACR1 | NM_001058.2 | 501 | atggtactacggcctgttctact | 175963 | - | see also 650 line | | |
| 34661 | TACR2 | NM_001057.1 | 624 | gccgctcgcggtgatgtttgtag | 175982 | - | see also 649 line | | |
| 34662 | TACR2 | NM_001057.1 | 625 | ccgctcgcggtgatgtttgtagc | 175983 | - | see also 649 line | | |
| 34663 | TACR3 | NM_001059.1 | 1364 | gtccatgacagtcgtgtttgacc | 176008 | - | see also 15224 line | | |
| 34664 | TACR3 | NM_001059.1 | 1259 | ctggtgtcctttcatcaaagttt | 176006 | - | see also 15224 line | | |
| 34665 | NPY1R | NM_000909.3 | 846 | tccatcggactctcataggttgt | 176041 | - | see also 15225 line | | |
| 34666 | NPY1R | NM_000909.3 | 1358 | cggtctcgggatgatgattatga | 176048 | - | see also 15225 line | | |
| 34667 | NPY2R | NM_000910.2 | 1526 | agcggttggatgccattcactct | 176076 | - | see also 592 line | | |
| 34668 | NPY2R | NM_000910.2 | 1055 | gggagtattcgctgattgagatc | 176066 | - | see also 592 line | | |
| 34669 | NPY5R | NM_006174.2 | 193 | agctcgacgagtattataacaag | 176081 | - | see also 4270 line | | |
| 34670 | NPY5R | NM_006174.2 | 312 | ctgattgggctctatacatttgt | 176089 | - | see also 4270 line | | |
| 34671 | GPR66 | NM_006056.2 | 412 | gcgcacgcctaccaactactacc | 176106 | - | see also 15228 line | | |
| 34672 | GPR66 | NM_006056.2 | 200 | aggaaccccatggcttgcaatgg | 176105 | - | see also 15228 line | | |
| 34673 | NMU2R | NM_020167.3 | 1263 | gtggagctgaccgaagatatagg | 176125 | - | see also 15229 line | | |
| 34674 | NMU2R | NM_020167.3 | 1261 | ttgtggagctgaccgaagatata | 176124 | - | see also 15229 line | | |
| 34675 | GPR10 | NM_004248.1 | 422 | agccggtcaccgtctatgtgtcg | 176129 | - | - | neuropeptide Y receptor | - |
| 34676 | GPR10 | NM_004248.1 | 959 | gccactggctcgccatgagttcg | 176130 | - | see also 34675 line | | |
| 34677 | GPR74 | NM_004885.1 | 843 | gtggtctacccttttaaaccaaa | 176159 | - | see also 15230 line | | |
| 34678 | GPR74 | NM_004885.1 | 1299 | tacgctgacctttctccaaatga | 176169 | - | see also 15230 line | | |
| 34679 | HCRTR2 | NM_001526.2 | 651 | agccacactggtcgtggatatca | 176187 | - | see also 15231 line | | |
| 34680 | HCRTR2 | NM_001526.2 | 463 | tacctgcacccgaaagaatatga | 176182 | - | see also 15231 line | | |

Figure 46

| 34681 | OT7T022 | NM_022146.1 | 379 | gtgtcggcttccgttttcacact | 176219 | - | see also 15232 line | | |
|---|---|---|---|---|---|---|---|---|---|
| 34682 | OT7T022 | NM_022146.1 | 377 | ctgtgtcggcttccgttttcaca | 176218 | - | see also 15232 line | | |
| 34683 | SORCS3 | NM_014978.1 | 1256 | gtcggtggccggattggataagg | 176235 | - | see also 15233 line | | |
| 34684 | SORCS3 | NM_014978.1 | 1457 | agccagctactacgtgtcttatc | 176241 | - | see also 15233 line | | |
| 34685 | OPRM1 | NM_000914.1 | 1309 | ctcgaattcgtcagaacactaga | 176334 | - | see also 15234 line | | |
| 34686 | OPRM1 | NM_000914.1 | 253 | ctgatgccttggcgtactcaagt | 176303 | - | see also 15234 line | | |
| 34687 | OPRK1 | NM_000912.2 | 1492 | atcgatgggatgaataaaccagt | 176354 | - | see also 15235 line | | |
| 34688 | OPRK1 | NM_000912.2 | 720 | gagtacggtctacttgatgaatt | 176344 | - | see also 15235 line | | |
| 34689 | OPRD1 | NM_000911.2 | 448 | tcggcatcgtccggtacactaag | 176356 | - | see also 15236 line | | |
| 34690 | OPRD1 | NM_000911.2 | 606 | ctctccatcgactactacaatat | 176359 | - | see also 15236 line | | |
| 34691 | GPR7 | NM_005285.1 | 949 | aacctccgccagctgataacttg | 176364 | - | see also 15237 line | | |
| 34692 | OGFR | NM_007346.2 | 240 | acgagggacatgtgtaggtatcg | 176367 | - | see also 15238 line | | |
| 34693 | OGFR | NM_007346.2 | 1255 | tggagaagatcgctctgaatttg | 176377 | - | see also 15238 line | | |
| 34694 | OPCML | NM_002545.2 | 634 | accagtccggggagtacgaatgc | 176387 | - | see also 15239 line | | |
| 34695 | OPCML | NM_002545.2 | 207 | gccaccctcaggtgtaccataga | 176378 | - | see also 15239 line | | |
| 34696 | MC2R | NM_000529.1 | 7 | cacattatcaactcgtatgaaaa | 176392 | - | see also 15240 line | | |
| 34697 | MC2R | NM_000529.1 | 274 | ctcaagccacgtggcagtttga | 176398 | - | see also 15240 line | | |
| 34698 | MC1R | NM_002386.2 | 1454 | cacgtgcggctgcatcttcaaga | 176409 | - | see also 15241 line | | |
| 34699 | MC3R | NM_019888.2 | 357 | ggccgacatgctggtaagtgtgt | 176415 | - | see also 15242 line | | |
| 34700 | MC3R | NM_019888.2 | 429 | gaccttcgaggaccagtttatcc | 176416 | - | see also 15242 line | | |
| 34701 | MC4R | NM_005912.1 | 113 | gagggtgctacgagcaactttt | 176420 | - | see also 4133 line | | |
| 34702 | MC4R | NM_005912.1 | 491 | agcgggttgggatcatcataagt | 176436 | - | see also 4133 line | | |
| 34703 | MC5R | NM_005913.1 | 862 | tccgtgatggaccctctcatata | 176456 | - | gpcr、receptor_acitivity | melanocortin receptor | - |
| 34704 | MC5R | NM_005913.1 | 510 | cacgggctgcggcattgtcttca | 176454 | - | see also 34703 line | | |
| 34705 | MC5R | NM_005913.1 | 298 | aacaacaagcacctagtgatagc | 176451 | - | see also 34703 line | | |
| 34706 | SSTR1 | NM_001049.2 | 1021 | gcgtggacgcggtcaacatgttc | 176459 | - | see also 15244 line | | |
| 34707 | SSTR1 | NM_001049.2 | 1655 | cgccggaggagccggttgactatt | 176466 | - | see also 15244 line | | |
| 34708 | SSTR2 | NM_001050.1 | 467 | cccggacggccaagatgatcacc | 176478 | - | see also 15245 line | | |
| 34709 | SSTR2 | NM_001050.1 | 7 | atggcggatgagccactcaatgg | 176468 | - | see also 15245 line | | |
| 34710 | AVPR2 | NM_000054.2 | 914 | ctcatcttccgggagattcatgc | 176489 | - | see also 15247 line | | |
| 34711 | AVPR2 | NM_000054.2 | 358 | ggcgctgctctccatagtctttg | 176488 | - | see also 15247 line | | |
| 34712 | OXTR | NM_000916.3 | 1695 | gagagacgagtgccagcaaaaag | 176495 | - | see also 15248 line | | |
| 34713 | OXTR | NM_000916.3 | 1438 | cacggtcaagatgactttcatca | 176493 | - | see also 15248 line | | |
| 34714 | F2RL2 | NM_004101.2 | 1038 | atcatccggacacttaatgcata | 176523 | - | see also 15249 line | | |
| 34715 | F2RL2 | NM_004101.2 | 1035 | gccatcatccggacacttaatgc | 176522 | - | see also 15249 line | | |
| 34716 | F2RL2 | NM_004101.2 | 1048 | cacttaatgcatacgatcataga | 176525 | - | see also 15249 line | | |

1229

EP 1 752 536 A1

Figure 46

| | | | | | | | |
|---|---|---|---|---|---|---|---|
| 34717 | GP1BA | NM_000173.1 | 634 | aactcgctgtatacaataccaaa | 176540 | - | see also 15250 line |
| 34718 | GP1BA | NM_000173.1 | 626 | tccaagagaactgctgtataca | 176538 | - | see also 15250 line |
| 34719 | FPR1 | NM_002029.3 | 641 | ctggaccaacgaccctaaagaga | 176558 | - | see also 15251 line |
| 34720 | FPR1 | NM_002029.3 | 893 | cagaatccgtgagttattgcaag | 176565 | - | see also 15251 line |
| 34721 | FPRL1 | NM_001462.1 | 1208 | atcgtcggaccttggattcttgc | 176573 | - | see also 15252 line |
| 34722 | FPRL1 | NM_001462.1 | 1375 | agggattatccggtttgtcattg | 176578 | - | see also 15252 line |
| 34723 | FPRL2 | NM_002030.3 | 749 | gagagttgaacgtgttcattac | 176594 | - | see also 15253 line |
| 34724 | FPRL2 | NM_002030.3 | 475 | atgtaagttagttcatgttatga | 176587 | - | see also 15253 line |
| 34725 | GALR1 | NM_001480.2 | 1747 | ttcacaactgagtgatactaaag | 176613 | - | see also 15254 line |
| 34726 | GALR1 | NM_001480.2 | 1345 | gcccgaccctgccacaagaagg | 176601 | - | see also 15254 line |
| 34727 | AGTR1 | NM_000685.3 | 790 | cgccttcgacgcacaatgcttgt | 176633 | - | see also 15256 line |
| 34728 | AGTR1 | NM_000685.3 | 712 | aaactgtacgctagtgtgtttct | 176629 | - | see also 15256 line |
| 34729 | AGTR2 | NM_000686.3 | 459 | atgggcaacctattattcttata | 176661 | - | see also 429 line |
| 34730 | AGTR2 | NM_000686.3 | 453 | tcctctatggcaacctattatt | 176659 | - | see also 429 line |
| 34731 | MAS1 | NM_002377.2 | 275 | gtctatcgactatgctttagatt | 176691 | - | see also 15258 line |
| 34732 | MAS1 | NM_002377.2 | 27 | aacgtgacatcatttgttgttga | 176687 | - | see also 15258 line |
| 34733 | EDNRA | NM_001957.1 | 1499 | aaccgatgtgaattacttagttt | 176731 | - | see also 15259 line |
| 34734 | EDNRA | NM_001957.1 | 1154 | ttcgtcatgtaccctttgaata | 176720 | - | see also 15259 line |
| 34735 | EDNRB | NM_000115.1 | 632 | tgcgaaacggtcccaatatcttg | 176738 | - | see also 97 line |
| 34736 | EDNRB | NM_000115.1 | 1330 | ttggtattggactatatggtat | 176753 | - | see also 97 line |
| 34737 | CCKAR | NM_000730.1 | 674 | ctccgtacccatttatagcaac | 176777 | - | see also 15261 line |
| 34738 | CCKAR | NM_000730.1 | 675 | tccgtacccatttatagcaact | 176778 | - | see also 15261 line |
| 34739 | CCKBR | NM_176875.2 | 701 | cacgccggctcgcgtgattgtagc | 176794 | - | see also 15262 line |
| 34740 | CCKBR | NM_176875.2 | 914 | atctctcgcgagctctacttagg | 176796 | - | see also 15262 line |
| 34741 | CCKBR | NM_176875.2 | 1486 | ttcgctgtccaggcttagctaca | 176799 | - | see also 15262 line |
| 34742 | BDKRB1 | NM_000710.2 | 745 | agccgtcccagatcgaacatca | 176804 | - | see also 15263 line |
| 34743 | BDKRB1 | NM_000710.2 | 852 | tggctgcatcgtcttcttcaac | 176806 | - | see also 15263 line |
| 34744 | BDKRB2 | NM_000623.2 | 1263 | ctccgtggaacgccagattcaca | 176821 | - | see also 15264 line |
| 34745 | BDKRB2 | NM_000623.2 | 405 | ctgcaacggtgcagagatctacc | 176815 | - | see also 15264 line |
| 34746 | BRS3 | NM_001727.1 | 682 | ttgctcacctgaggctatatt | 176843 | - | see also 1148 line |
| 34747 | BRS3 | NM_001727.1 | 288 | ttgtgtgccatctatattactta | 176832 | - | see also 1148 line |
| 34748 | NMBR | NM_002511.1 | 663 | cagttccgaagcggtgtttca | 176862 | - | see also 15266 line |
| 34749 | NMBR | NM_002511.1 | 693 | ctcgcatcagtagcttggataat | 176865 | - | see also 15266 line |
| 34750 | C5R1 | NM_001736.1 | 668 | tactcacgctcacgatttgttac | 176897 | - | see also 15267 line |
| 34751 | C5R1 | NM_001736.1 | 665 | ctctactcacgctcacgatttgt | 176896 | - | see also 15267 line |
| 34752 | C3AR1 | NM_004054.2 | 982 | tccctagcgcttctagcaattcc | 176925 | - | see also 15268 line |
| 34753 | C3AR1 | NM_004054.2 | 981 | ttccctagcgcttctagcaattc | 176924 | - | see also 15268 line |

Figure 46

| 34754 | HCRTR1 | NM_001525.1 | 1089 | gcccatcagcgtcctcaatgtcc | 176940 | - | see also 15269 line |
|---|---|---|---|---|---|---|---|
| 34755 | NTSR1 | NM_002531.1 | 1065 | caccgtcaaggtcgtcatacagg | 176945 | - | see also 15270 line |
| 34756 | NTSR1 | NM_002531.1 | 1371 | gtggactccgttcctctatgact | 176948 | - | see also 15270 line |
| 34757 | NTSR2 | NM_012344.2 | 880 | cccaagtgccgtccacttctacc | 176954 | - | see also 15271 line |
| 34758 | NTSR2 | NM_012344.2 | 767 | caccgcgctccaagtctttatcc | 176952 | - | see also 15271 line |
| 34759 | GRPR | NM_005314.1 | 794 | ggcgctctcggcagacagataca | 176970 | - | see also 15272 line |
| 34760 | GRPR | NM_005314.1 | 871 | gcctcaaagccgcctttatctgg | 176975 | - | see also 15272 line |
| 34761 | GRPR | NM_005314.1 | 605 | gtccatgcgaaacgttccaaacc | 176966 | - | see also 15272 line |
| 34762 | NPR3 | NM_000908.1 | 1439 | accgaattgtagagcatacaaac | 177007 | - | see also 15273 line |
| 34763 | NPR3 | NM_000908.1 | 1387 | cggccgaatgtcaaatatccttg | 177005 | - | see also 15273 line |
| 34764 | NPR3 | NM_000908.1 | 1388 | ggccgaatgtcaaatatccttgg | 177006 | - | see also 15273 line |
| 34765 | BRAP | NM_006768.2 | 327 | atctcggccgtcgagaaatgaca | 177014 | - | see also 4773 line |
| 34766 | BRAP | NM_006768.2 | 768 | cacaggctgatgcggatagtttt | 177028 | - | see also 4773 line |
| 34767 | KPNA3 | NM_002267.2 | 1921 | aggaggtacctacaattttgatc | 177084 | - | see also 1454 line |
| 34768 | KPNA3 | NM_002267.2 | 1654 | accaccgttctgtaatttactgt | 177078 | - | see also 1454 line |
| 34769 | KPNA3 | NM_002267.2 | 719 | gacagaaatccaccgattgatga | 177060 | - | see also 1454 line |
| 34770 | KPNB2 | NM_002270.2 | 1064 | gacgaaacgattcctgatagtga | 177110 | - | see also 1464 line |
| 34771 | KPNB2 | NM_002270.2 | 1088 | caggatatacggccacgttttca | 177111 | - | see also 1464 line |
| 34772 | KPNB2 | NM_002270.2 | 2073 | agctggtagcccgaagtaacatc | 177131 | - | see also 1464 line |
| 34773 | TNPO2 | NM_013433.2 | 2391 | gccctgtatcgccgagttcatgc | 177156 | - | see also 15277 line |
| 34774 | TNPO2 | NM_013433.2 | 2760 | gggcgttgtgcaggactttattt | 177157 | - | see also 15277 line |
| 34775 | KDELR1 | NM_006801.2 | 409 | ttcaccacggtctggttgattta | 177163 | - | see also 15278 line |
| 34776 | KDELR1 | NM_006801.2 | 365 | cactctacaacacgtgtatgaag | 177162 | - | see also 15278 line |
| 34777 | KDELR2 | NM_006854.2 | 477 | gggcctctatcgtgctttgtatc | 177180 | - | see also 15279 line |
| 34778 | KDELR2 | NM_006854.2 | 259 | aaggcaacctacgatggaaatca | 177176 | - | see also 15279 line |
| 34779 | PEX7 | NM_000288.1 | 854 | tggcctcttgctcgtatgatttt | 177205 | - | see also 253 line |
| 34780 | PXR1 | NM_000319.3 | 579 | caccgatcgctggtatgatgaat | 177218 | - | see also 266 line |
| 34781 | PXR1 | NM_000319.3 | 577 | gccaccgatcgctggtatgatga | 177217 | - | see also 266 line |
| 34782 | PXR1 | NM_000319.3 | 580 | accgatcgctggtatgatgaata | 177219 | - | see also 266 line |
| 34783 | SSR2 | NM_003145.2 | 365 | cacctcggcaacaattacttacc | 177246 | - | see also 2126 line |
| 34784 | SSR2 | NM_003145.2 | 216 | gacgtggaactatctgatgattc | 177239 | - | see also 2126 line |
| 34785 | SSR3 | NM_007107.2 | 198 | ctggcgaatatggcatatggatc | 177252 | - | see also 4952 line |
| 34786 | SSR3 | NM_007107.2 | 110 | aggatttcagccgcaatctctcg | 177250 | - | see also 4952 line |
| 34787 | TOMM34 | NM_006809.4 | 255 | ctgcaggcgcaaggttcttcaga | 177265 | - | see also 15284 line |
| 34788 | TOMM34 | NM_006809.4 | 438 | aagtaccctatggcctatgttga | 177267 | - | see also 15284 line |
| 34789 | NKTR | NM_005385.2 | 242 | gccggttggtcgcattatgtttc | 177275 | - | see also 3746 line |
| 34790 | NKTR | NM_005385.2 | 241 | agccggttggtcgcattatgttt | 177274 | - | see also 3746 line |

Figure 46

| | | | | | | | | | |
|---|---|---|---|---|---|---|---|---|---|
| 34791 | NKTR | NM_005385.2 | 4392 | tacacctacgatagctactatag | 177382 | - | see also 3746 line | | |
| 34792 | PPIC | NM_000943.3 | 125 | ggctcggcgcacttgtgttttct | 177393 | - | signal_transduction | cyclosporin A binding | - |
| 34793 | PPIC | NM_000943.3 | 642 | caccaactgctcgatcatcaaca | 177398 | - | see also 34792 line | | |
| 34794 | PPIC | NM_000943.3 | 123 | ggggctcggcgcacttgtgtttt | 177391 | - | see also 34792 line | | |
| 34795 | PPIH | NM_006347.2 | 274 | tactggagtcgccagtatttacc | 177406 | - | see also 15286 line | | |
| 34796 | PPIH | NM_006347.2 | 216 | tccacagggtcataaaggatttc | 177405 | - | see also 15286 line | | |
| 34797 | PPIH | NM_006347.2 | 50 | aaccccgtggtgttctttgatgt | 177401 | - | see also 15286 line | | |
| 34798 | AAMP | NM_001087.1 | 136 | atcgaggtggtagaacttgatcc | 177410 | - | see also 15287 line | | |
| 34799 | AAMP | NM_001087.1 | 120 | tggtgatgaagagattatcgagg | 177409 | - | see also 15287 line | | |
| 34800 | APLP1 | NM_005166.2 | 1029 | taggatgcgccagattaatgagg | 177431 | - | see also 3633 line | | |
| 34801 | APOH | NM_000042.1 | 313 | gccgtacgctatacgactttga | 177447 | - | see also 15288 line | | |
| 34802 | APOH | NM_000042.1 | 131 | cagtggtcccgttaaaaacattc | 177436 | - | see also 15288 line | | |
| 34803 | AZU1 | NM_001700.3 | 288 | ggcagtcccgccagacgttttcc | 177470 | - | kinase_related | trypsin | - |
| 34804 | AZU1 | NM_001700.3 | 292 | gtcccgccagacgttttccatca | 177471 | - | see also 34803 line | | |
| 34805 | AZU1 | NM_001700.3 | 507 | cccgttttcccaggtttgtcaac | 177472 | - | see also 34803 line | | |
| 34806 | COL5A1 | NM_000093.2 | 1071 | accgggcagcttatgattactgt | 177484 | - | see also 68 line | | |
| 34807 | COL5A1 | NM_000093.2 | 5840 | ttcggtgaagcgtcacagaaatt | 177503 | - | see also 68 line | | |
| 34808 | HBP17 | NM_005130.1 | 543 | agctagtcagctccactctattt | 177518 | - | see also 15290 line | | |
| 34809 | HBP17 | NM_005130.1 | 394 | tccaacctcatgcctaaagctca | 177513 | - | see also 15290 line | | |
| 34810 | HRG | NM_000412.2 | 324 | gtgatcggacaatgtaaggtaat | 177527 | - | see also 15291 line | | |
| 34811 | HRG | NM_000412.2 | 145 | ggcgacgggatggctacctttc | 177522 | - | see also 15291 line | | |
| 34812 | LAMC2 | NM_005562.1 | 1125 | ctccgcatccgagctacatatgg | 177577 | - | see also 3866 line | | |
| 34813 | LAMC2 | NM_005562.1 | 2863 | cccgtgccaatcttgctaaaagc | 177605 | - | see also 3866 line | | |
| 34814 | LIPC | NM_000236.1 | 896 | agcgatcggtgcaccttttcatc | 177625 | - | see also 15293 line | | |
| 34815 | LIPC | NM_000236.1 | 332 | tggacggcgtgctagaaaactgg | 177620 | - | see also 15293 line | | |
| 34816 | LIPC | NM_000236.1 | 598 | atcggtggaacgcacaagattgg | 177622 | - | see also 15293 line | | |
| 34817 | LIPG | NM_006033.1 | 1569 | ccccggacgggagctgaatatca | 177666 | - | see also 15294 line | | |
| 34818 | LIPG | NM_006033.1 | 1568 | accccggacgggagctgaatatc | 177665 | - | see also 15294 line | | |
| 34819 | LPL | NM_000237.1 | 973 | tcccacgagcgctccattcatct | 177691 | - | see also 15295 line | | |
| 34820 | LPL | NM_000237.1 | 791 | tagacgtcttacacacattcacc | 177684 | - | see also 15295 line | | |
| 34821 | ODZ1 | NM_014253.1 | 3727 | ctcgggaaactccgttagtattt | 177784 | - | see also 5559 line | | |
| 34822 | ODZ1 | NM_014253.1 | 6825 | ggcgacgtgtcgcgagtaagtcc | 177891 | - | see also 5559 line | | |
| 34823 | SERPINC1 | NM_000488.1 | 1245 | tgcaagtaccgctgttgtgattg | 177944 | - | see also 15297 line | | |
| 34824 | SERPINC1 | NM_000488.1 | 831 | gtcgtgttcagcatctatgatgt | 177937 | - | see also 15297 line | | |
| 34825 | SERPINC1 | NM_000488.1 | 682 | ggccgaatcaccgatgtcattcc | 177933 | - | see also 15297 line | | |
| 34826 | SERPIND1 | NM_000185.2 | 1235 | aagtgcttctgccgaaattcaag | 177951 | - | see also 15298 line | | |

Figure 46

| 34827 | SERPIND1 | NM_000185.2 | 1133 | ttgtggtcccacacaagatgtct | 177950 | - | see also 15298 line |
|---|---|---|---|---|---|---|---|
| 34828 | SERPINE2 | NM_006216.2 | 745 | tcctcgtcaacgcagtgtatttc | 177971 | - | see also 15299 line |
| 34829 | SERPINE2 | NM_006216.2 | 744 | gtcctcgtcaacgcagtgtattt | 177970 | - | see also 15299 line |
| 34830 | VTN | NM_000638.2 | 1030 | ctgctatgaactggacgaaaagg | 177991 | - | see also 15300 line |
| 34831 | VTN | NM_000638.2 | 1817 | gacaagtactaccgagtcaatct | 178002 | - | see also 15300 line |
| 34832 | BRAL1 | NM_021817.1 | 877 | gccggtaccagttcaattactac | 178004 | - | - | - | - |
| 34833 | HAPLN3 | NM_178232.2 | 373 | ggcgtgtgcgtgtcaaatggtgg | 178005 | - | see also 15301 line |
| 34834 | HAPLN3 | NM_178232.2 | 1174 | gccgcttgtacggtgtttactgc | 178014 | - | see also 15301 line |
| 34835 | HMMR | NM_012484.1 | 2008 | ctccgctgtcagcttgctaaaaa | 178055 | - | see also 15302 line |
| 34836 | HMMR | NM_012484.1 | 1982 | gccaactcaaatcggaagtatca | 178053 | - | see also 15302 line |
| 34837 | IMPG2 | NM_016247.1 | 2902 | gcctccgagtgactaacatgatg | 178133 | - | see also 6751 line |
| 34838 | IMPG2 | NM_016247.1 | 3663 | aagtacaaccccgtgtatgaaag | 178151 | - | see also 6751 line |
| 34839 | TM6SF2 | NM_023002.1 | 523 | cccccgtggaggccgatacaagc | 178155 | - | see also 15304 line |
| 34840 | TNFAIP6 | NM_007115.2 | 414 | atggaatccgtctcaataggagt | 178165 | - | see also 15305 line |
| 34841 | TNFAIP6 | NM_007115.2 | 235 | agctaaggcggtgtgtgaatttg | 178160 | - | see also 15305 line |
| 34842 | XLKD1 | NM_006691.2 | 480 | ttcgtggtcatctctaggattag | 178187 | - | see also 15306 line |
| 34843 | XLKD1 | NM_006691.2 | 405 | gccggcaaggaccaagttgaaac | 178185 | - | see also 15306 line |
| 34844 | DSPG3 | NM_004950.2 | 540 | ttctattcccgctttaacagaat | 178209 | - | see also 15307 line |
| 34845 | DSPG3 | NM_004950.2 | 687 | gtcctgcgtgacaacaaaataag | 178214 | - | see also 15307 line |
| 34846 | HABP2 | NM_004132.2 | 226 | tacagctacgaggattataatca | 178223 | - | see also 15308 line |
| 34847 | HABP2 | NM_004132.2 | 624 | tgcctgtcccgaccagttcaagg | 178226 | - | see also 15308 line |
| 34848 | PODLX2 | NM_015720.1 | 618 | aagcctcaggtccgtgacttttc | 178252 | - | see also 15309 line |
| 34849 | PODLX2 | NM_015720.1 | 583 | agctgctccctgtgaatggatcc | 178251 | - | see also 15309 line |
| 34850 | PODLX2 | NM_015720.1 | 619 | agcctcaggtccgtgacttttct | 178253 | - | see also 15309 line |
| 34851 | TGFBR3 | NM_003243.1 | 2535 | ctcgctggacgcctcgataatct | 178299 | - | see also 15310 line |
| 34852 | TGFBR3 | NM_003243.1 | 1342 | gacaatggctatagtccaataac | 178271 | - | see also 15310 line |
| 34853 | TGFBR3 | NM_003243.1 | 2537 | cgctggacgcctcgataatctgg | 178300 | - | see also 15310 line |
| 34854 | GABRA1 | NM_000806.3 | 388 | agctatggacagccgtcattaca | 178305 | - | see also 493 line |
| 34855 | GABRA1 | NM_000806.3 | 644 | tcctccggttaaataacctaatg | 178315 | - | see also 493 line |
| 34856 | GABRA2 | NM_000807.1 | 356 | gacttctggatggttacgataat | 178345 | - | see also 15312 line |
| 34857 | GABRA2 | NM_000807.1 | 840 | tcctgatggctctaggttaaatc | 178365 | - | see also 15312 line |
| 34858 | GABRA3 | NM_000808.2 | 1256 | ctggttcatagccgtctgttatg | 178410 | - | see also 495 line |
| 34859 | GABRA4 | NM_000809.2 | 826 | aaccgtatcaagtgaaaccatca | 178434 | - | see also 499 line |
| 34860 | GABRA4 | NM_000809.2 | 1406 | ggcacacctcggtcttacttagc | 178449 | - | see also 499 line |
| 34861 | GABRA5 | NM_000810.2 | 1615 | gacaaaatgtcccgaatcgtatt | 178482 | - | see also 502 line |
| 34862 | GABRA5 | NM_000810.2 | 1648 | ttcggcactttcaacttagttta | 178485 | - | see also 502 line |
| 34863 | GABRA5 | NM_000810.2 | 916 | agctatgcgtaccctaattctga | 178466 | - | see also 502 line |

Figure 46

| | | | | | | | |
|---|---|---|---|---|---|---|---|
| 34864 | GABRA6 | NM_000811.1 | 689 | atctaacacaggtgaatacgtta | 178507 | - | see also 503 line |
| 34865 | GABRA6 | NM_000811.1 | 693 | aacacaggtgaatacgttataat | 178508 | - | see also 503 line |
| 34866 | GABRA6 | NM_000811.1 | 987 | ttcgcagctgtcaactactttac | 178516 | - | see also 503 line |
| 34867 | GABRB1 | NM_000812.2 | 1171 | gtcgacgcccacgtaacattct | 178552 | - | see also 504 line |
| 34868 | GABRB1 | NM_000812.2 | 780 | tccacgactgtcactaagtttc | 178541 | - | see also 504 line |
| 34869 | GABRB2 | NM_000813.1 | 1567 | atcggtggtcccgcatattcttc | 178597 | - | see also 508 line |
| 34870 | GABRB2 | NM_000813.1 | 1474 | aacgacatgtggcgcaaaagaaa | 178595 | - | see also 508 line |
| 34871 | GABRB3 | NM_000814.3 | 895 | gccctctatactgataaacgattc | 178608 | - | see also 511 line |
| 34872 | GABRB3 | NM_000814.3 | 894 | tgccctctatactgataacgatt | 178607 | - | see also 511 line |
| 34873 | GABRB3 | NM_000814.3 | 1249 | atcgctggaagttcacaatgaaa | 178612 | - | see also 511 line |
| 34874 | GABRD | NM_000815.2 | 563 | ctggagagctacggttactcatc | 178619 | - | see also 15318 line |
| 34875 | GABRD | NM_000815.2 | 77 | cgcggcaacgagcgatgaatga | 178617 | - | see also 15318 line |
| 34876 | GABRE | NM_004961.2 | 1184 | tccgccatcctgtatcaatagc | 178654 | - | see also 15319 line |
| 34877 | GABRE | NM_004961.2 | 1183 | ctcgccatcctgtatcaatag | 178653 | - | see also 15319 line |
| 34878 | GABRG1 | NM_173536.2 | 577 | tcctaatcgtcttcgaattt | 178683 | - | see also 15320 line |
| 34879 | GABRG1 | NM_173536.2 | 582 | atgtctgcttcgaatttggaat | 178684 | - | see also 15320 line |
| 34880 | GABRG2 | NM_000816.2 | 594 | gtccagtgaacgctatcaatatg | 178716 | - | see also 512 line |
| 34881 | GABRG2 | NM_000816.2 | 849 | atgctgagtgccaattacaattg | 178724 | - | see also 512 line |
| 34882 | GABRG3 | NM_033223.1 | 242 | gaccgtaattgacgttgacattt | 178754 | - | see also 9381 line |
| 34883 | GABRG3 | NM_033223.1 | 162 | gacaccgagtgactcttattct | 178752 | - | see also 9381 line |
| 34884 | GABRP | NM_014211.1 | 989 | gagtgacgacgtgttatcaatg | 178786 | - | see also 15323 line |
| 34885 | GABRP | NM_014211.1 | 794 | agcggtatttcaccttagtcacc | 178783 | - | see also 15323 line |
| 34886 | GABRQ | NM_018558.1 | 368 | ttcaacgcttagcatactatgaga | 178810 | - | see also 7524 line |
| 34887 | GABRQ | NM_018558.1 | 724 | tcctgggaaggacgattactagc | 178820 | - | see also 7524 line |
| 34888 | GABRR1 | NM_002042.2 | 1367 | atcgacaacccacgccattgataa | 178872 | - | see also 15325 line |
| 34889 | GABRR1 | NM_002042.2 | 114 | aagtccacagagatgtctaagaaa | 178856 | - | see also 15325 line |
| 34890 | GABRR2 | NM_002043.1 | 1359 | aaggccagaacgcggtttcgtat | 178877 | - | see also 15326 line |
| 34891 | GABRR2 | NM_002043.1 | 382 | ccctgtacctgcggcattactgg | 178874 | - | see also 15326 line |
| 34892 | GABRR2 | NM_002043.1 | 715 | atgggatgaatccctaaaaaaca | 178876 | - | see also 15326 line |
| 34893 | GABBR1 | NM_001470.1 | 1116 | cctacccgcgtgaaactctttg | 178887 | - | see also 1017 line |
| 34894 | GABBR1 | NM_001470.1 | 1743 | gaccattaccgaccaaatcctacc | 178904 | - | see also 1017 line |
| 34895 | GABBR1 | NM_001470.1 | 1119 | tacccgcgtgaaactctttgaaa | 178888 | - | see also 1017 line |
| 34896 | CHRM1 | NM_000738.2 | 1292 | ctccgaagtggtgatcaagatgc | 178924 | - | see also 479 line |
| 34897 | CHRM1 | NM_000738.2 | 1354 | cccacacggagctccccaataca | 178925 | - | see also 479 line |
| 34898 | CHRM2 | NM_000739.1 | 1003 | gactcatgtacccccaactaatac | 178947 | - | see also 15329 line |
| 34899 | CHRM2 | NM_000739.1 | 158 | acctccagaacgtcaacaattac | 178932 | - | see also 15329 line |
| 34900 | CHRM3 | NM_000740.1 | 90 | cccgggaaccgtcactcatttcg | 178956 | - | see also 15330 line |

Figure 46

| | | | | | | | |
|---|---|---|---|---|---|---|---|
| 34901 | CHRM3 | NM_000740.1 | 1721 | agcagagacagtcggtcattttt | 178977 | - | see also 15330 line |
| 34902 | CHRM3 | NM_000740.1 | 368 | atctgtttacgacctacatcatc | 178964 | - | see also 15330 line |
| 34903 | CHRM5 | NM_012125.2 | 1673 | cacatggacccccgtataacatca | 178990 | - | see also 15331 line |
| 34904 | CHRM5 | NM_012125.2 | 1455 | ttccgattggtggtaaaagctga | 178985 | - | see also 15331 line |
| 34905 | CHRM5 | NM_012125.2 | 1602 | aagagagtggtcctagtcaaaga | 178987 | - | see also 15331 line |
| 34906 | CHRNA1 | NM_000079.1 | 122 | cccgtctggtggcaaagctattt | 178993 | - | see also 15332 line |
| 34907 | CHRNA1 | NM_000079.1 | 317 | atgactatggcggtgtgaaaaaa | 178999 | - | see also 15332 line |
| 34908 | CHRNA1 | NM_000079.1 | 261 | aaccaatgtgcgtctgaaacagc | 178995 | - | see also 15332 line |
| 34909 | CHRNA2 | NM_000742.1 | 2120 | tccgttcctagctggaatgatct | 179011 | - | see also 15333 line |
| 34910 | CHRNA2 | NM_000742.1 | 1741 | agctcagcccctcttatcactgg | 179009 | - | see also 15333 line |
| 34911 | CHRNA3 | NM_000743.2 | 1303 | tacggtgccgagctctcaaatct | 179027 | - | see also 15334 line |
| 34912 | CHRNA3 | NM_000743.2 | 1439 | acctcacgagaagctctagttct | 179033 | - | see also 15334 line |
| 34913 | CHRNA3 | NM_000743.2 | 730 | tacgataaggcgaaaatcgatct | 179021 | - | see also 15334 line |
| 34914 | CHRNA4 | NM_000744.2 | 359 | aactcttctccggttacaacaag | 179040 | - | see also 15335 line |
| 34915 | CHRNA4 | NM_000744.2 | 1340 | ggctcatcgagtccatgcataag | 179042 | - | see also 15335 line |
| 34916 | CHRNA5 | NM_000745.2 | 1351 | ctcgattctattcgctacattac | 179085 | - | see also 15336 line |
| 34917 | CHRNA5 | NM_000745.2 | 1208 | cgcctttggtccgcaagatattt | 179079 | - | see also 15336 line |
| 34918 | CHRNA6 | NM_004198.2 | 762 | tgccgatgtttacacgattaat | 179110 | - | see also 15337 line |
| 34919 | CHRNA6 | NM_004198.2 | 763 | gccgatgtttacacgattaatc | 179111 | - | see also 15337 line |
| 34920 | CHRNA7 | NM_000746.2 | 315 | ttggacagatcactatttacagt | 179128 | - | see also 480 line |
| 34921 | CHRNB1 | NM_000747.1 | 1255 | ccctgatctgcggcgatttatcg | 179154 | - | see also 15339 line |
| 34922 | CHRNB1 | NM_000747.1 | 1254 | cccctgatctgcggcgatttatc | 179153 | - | see also 15339 line |
| 34923 | CHRNB1 | NM_000747.1 | 1191 | tccggaagccgccaagtgatttt | 179151 | - | see also 15339 line |
| 34924 | CHRNB2 | NM_000748.1 | 640 | atgtacgaggtgtccttctattc | 179160 | - | see also 15340 line |
| 34925 | CHRNB2 | NM_000748.1 | 721 | agcgcatgcaagattgaagtaaa | 179162 | - | see also 15340 line |
| 34926 | CHRNB4 | NM_000750.1 | 782 | acgtgacttacgacttcatcatc | 179171 | - | see also 15341 line |
| 34927 | CHRNB4 | NM_000750.1 | 456 | gcctgacatcgtgctttacaaca | 179166 | - | see also 15341 line |
| 34928 | CHRND | NM_000751.1 | 385 | ctcctgcaacgtgcttgtctacc | 179176 | - | see also 15342 line |
| 34929 | CHRND | NM_000751.1 | 1317 | ctgtggatggggcaaacttcatt | 179179 | - | see also 15342 line |
| 34930 | CHRNE | NM_000080.2 | 1124 | ggcgtcgtcggtgggcttattgc | 179199 | - | see also 49 line |
| 34931 | CHRNA10 | NM_020402.2 | 957 | gccgctcatcgggaagtactaca | 179208 | - | see also 15344 line |
| 34932 | CHRNA10 | NM_020402.2 | 415 | gacatcgtactctataacaaagc | 179206 | - | see also 15344 line |
| 34933 | CHRNA10 | NM_020402.2 | 959 | cgctcatcgggaagtactacatg | 179209 | - | see also 15344 line |
| 34934 | CHRNA9 | NM_017581.1 | 292 | cacgtgggaccgagatcagtacg | 179221 | - | see also 15345 line |
| 34935 | CHRNA9 | NM_017581.1 | 355 | gaggccagacatcgtcttatata | 179222 | - | see also 15345 line |
| 34936 | CHRNB3 | NM_000749.2 | 1180 | atcatgtggatcgctactcatcc | 179279 | - | see also 15346 line |
| 34937 | CHRNB3 | NM_000749.2 | 195 | aactcaatcgccgaaaatgaaga | 179256 | - | see also 15346 line |

Figure 46

| | | | | | | | |
|---|---|---|---|---|---|---|---|
| 34938 | GLRA1 | NM_000171.1 | 1039 | tgggttactacctgattcagatg | 179296 | - | see also 15347 line |
| 34939 | GLRA1 | NM_000171.1 | 1040 | gggttactacctgattcagatgt | 179297 | - | see also 15347 line |
| 34940 | GLRA3 | NM_006529.1 | 736 | ctcgcgtacagtgaatatcctga | 179318 | - | see also 4563 line |
| 34941 | GLRA3 | NM_006529.1 | 731 | cccgcctcgctacagtgaatat | 179315 | - | see also 4563 line |
| 34942 | GLRB | NM_000824.2 | 757 | tacacaactgatgatttacgatt | 179358 | - | see also 522 line |
| 34943 | GLRB | NM_000824.2 | 930 | gaggcaggtcggcttttacatga | 179363 | - | see also 522 line |
| 34944 | HTR3C | NM_130770.1 | 107 | ggcgacgcttttaccatcaattg | 179384 | - | see also 15350 line |
| 34945 | HTR3C | NM_130770.1 | 103 | aagaggcgacgcttttaccatca | 179383 | - | see also 15350 line |
| 34946 | HTR3C | NM_130770.1 | 445 | ttccggtctcactgcctatatca | 179394 | - | see also 15350 line |
| 34947 | C20orf79 | NM_178483.1 | 339 | gtggaccattgatctgaagaatg | 179409 | - | see also 15351 line |
| 34948 | C20orf79 | NM_178483.1 | 106 | gaccatcaaccaagatcaaagc | 179406 | - | see also 15351 line |
| 34949 | HSD17B4 | NM_000414.1 | 1268 | gagagcagtacttagagttatat | 179443 | - | see also 15352 line |
| 34950 | HSD17B4 | NM_000414.1 | 2028 | tggggctaagtggactattgacc | 179462 | - | see also 15352 line |
| 34951 | STOML1 | NM_004809.3 | 320 | cacctacgagcggatgattgtgt | 179468 | - | see also 3343 line |
| 34952 | STOML1 | NM_004809.3 | 318 | cccacctacgagcggatgattgt | 179467 | - | see also 3343 line |
| 34953 | STOML1 | NM_004809.3 | 95 | ggcgctgccctgggtgatttg | 179463 | - | see also 3343 line |
| 34954 | ALDH1A1 | NM_000689.3 | 272 | tggatcccgtggcgtactatgg | 179479 | - | see also 15354 line |
| 34955 | ALDH1A1 | NM_000689.3 | 476 | gggcgtacaataccaattgatg | 179493 | - | see also 15354 line |
| 34956 | SCGB2A1 | NM_002407.1 | 158 | accatcaattccgacatatctat | 179529 | - | see also 15355 line |
| 34957 | SCGB2A1 | NM_002407.1 | 161 | atcaattccgacatatctataac | 179530 | - | see also 15355 line |
| 34958 | SCGB2A1 | NM_002407.1 | 263 | ctcaaccagtcacatagaactct | 179534 | - | see also 15355 line |
| 34959 | SHBG | NM_001040.2 | 660 | atcaaatccgggatatttctcc | 179542 | - | see also 15356 line |
| 34960 | SHBG | NM_001040.2 | 233 | gggacccagagggagtgatttt | 179539 | - | see also 15356 line |
| 34961 | OSBP | NM_002556.2 | 1255 | aacgaacgagccacactctttag | 179550 | - | see also 15357 line |
| 34962 | OSBP | NM_002556.2 | 1259 | aacgagccacactctttaggata | 179552 | - | see also 15357 line |
| 34963 | OSBPL5 | NM_020896.2 | 995 | ttccctggagaacgatgcattct | 179587 | - | see also 8019 line |
| 34964 | PGRMC2 | NM_006320.1 | 423 | ggcgggtccatatggaatattg | 179595 | - | see also 15360 line |
| 34965 | PGRMC2 | NM_006320.1 | 524 | cagatttgaatgcagtacaaatg | 179596 | - | see also 15360 line |
| 34966 | SCGB2A2 | NM_002411.1 | 211 | gacgacaatgccactacacaatgc | 179601 | - | see also 15361 line |
| 34967 | SCGB2A2 | NM_002411.1 | 314 | atgacagcagtctttgtgattta | 179604 | - | see also 15361 line |
| 34968 | SERPINA6 | NM_001756.2 | 925 | cactgagccgggacacgattaac | 179610 | - | see also 15362 line |
| 34969 | SERPINA6 | NM_001756.2 | 1208 | gcctatcatcttgcgtttcaacc | 179615 | - | see also 15362 line |
| 34970 | SERPINA6 | NM_001756.2 | 1199 | gacgtccaagcctatcacttgc | 179614 | - | see also 15362 line |
| 34971 | STE | NM_005420.2 | 660 | aagagtccacgtgactatttct | 179629 | - | see also 15363 line |
| 34972 | STE | NM_005420.2 | 830 | cacaacactgccagacgaaatta | 179635 | - | see also 15363 line |
| 34973 | TTR | NM_000371.1 | 92 | tacgggcaccggtgaatccaagt | 179640 | - | see also 15364 line |
| 34974 | TTR | NM_000371.1 | 149 | aggcagtcctgccatcaatgtgg | 179641 | - | see also 15364 line |

Figure 46

| | | | | | | | |
|---|---|---|---|---|---|---|---|
| 34975 | DKFZp547 H025 | NM_020161.1 | 478 | gagaacaagaagccacaagattc | 179648 | - | see also 15365 line |
| 34976 | FOLR1 | NM_000802.2 | 259 | ttcctacctatagattccaact | 179653 | - | see also 15366 line |
| 34977 | FOLR1 | NM_000802.2 | 397 | gcgcaaagagcgggtactgaacg | 179654 | - | see also 15366 line |
| 34978 | FOLR2 | NM_000803.2 | 616 | ctggacctcaggagttaacagt | 179657 | - | see also 492 line |
| 34979 | FOLR3 | NM_000804.2 | 761 | cccgtctgtgggattattgatt | 179661 | - | see also 15367 line |
| 34980 | FOLR3 | NM_000804.2 | 759 | gccccgtctgtgggattattga | 179660 | - | see also 15367 line |
| 34981 | FTCD | NM_006657.1 | 58 | tggaatgcgtccccaactttcg | 179664 | - | see also 15368 line |
| 34982 | FTCD | NM_006657.1 | 57 | gtggaatgcgtccccaacttc | 179663 | - | see also 15368 line |
| 34983 | GNMT | NM_018960.2 | 93 | cgcgcgtgtggcagcctgtatatc | 179666 | - | see also 15369 line |
| 34984 | GNMT | NM_018960.2 | 708 | atggctcctgccttgagtaag | 179675 | - | see also 15369 line |
| 34985 | SLC19A1 | NM_003056.2 | 1742 | ttcgggtcaacacgttctttgc | 179682 | - | - |
| 34986 | SLC19A1 | NM_003056.2 | 1827 | tccgcaagcagttccagttatac | 179683 | - | see also 34985 line |
| 34987 | SLC19A1 | NM_003056.2 | 2287 | tccaagcgacgtgttcagaatg | 179684 | - | see also 34985 line |
| 34988 | SLC19A2 | NM_006996.1 | 514 | gactacctccgttataaacctgt | 179690 | - | see also 4881 line |
| 34989 | SLC19A2 | NM_006996.1 | 1238 | gttggcgtggaggccgtttcaacc | 179714 | - | see also 4881 line |
| 34990 | SLC19A3 | NM_025243.2 | 560 | ctggcgaacatgtcgtactttta | 179753 | - | see also 8887 line |
| 34991 | SLC19A3 | NM_025243.2 | 561 | tggcgaacatgtcgtactttac | 179754 | - | see also 8887 line |
| 34992 | RBP4 | NM_006744.2 | 310 | gggcgagtccgtctttgaata | 179779 | - | transporter |
| 34993 | RBP4 | NM_006744.2 | 311 | ggccgagtccgtctttgaataa | 179780 | - | see also 34992 line |
| 34994 | RBP4 | NM_006744.2 | 653 | tactgcgatgcagatcagaaag | 179784 | - | see also 34992 line |
| 34995 | RLBP1 | NM_000326.3 | 822 | ctctctagtcggacaagtatgg | 179785 | - | see also 275 line |
| 34996 | RLBP1 | NM_000326.3 | 1102 | tccaacagccatggtacttcacc | 179789 | - | see also 275 line |
| 34997 | SLC5A6 | NM_021095.1 | 669 | accgatttgggacccaatattgg | 179794 | - | see also 15373 line |
| 34998 | SLC5A6 | NM_021095.1 | 786 | acctggagcttcgattcaataaa | 179796 | - | see also 15373 line |
| 34999 | SLC5A6 | NM_021095.1 | 788 | ctggagcttgattcaataaaac | 179797 | - | see also 15373 line |
| 35000 | TTPA | NM_000370.1 | 213 | ggcctgccggttactaaaaaact | 179811 | - | see also 15374 line |
| 35001 | TTPA | NM_000370.1 | 432 | cacatccgagcttattgtacagg | 179823 | - | see also 15374 line |
| 35002 | TTPA | NM_000370.1 | 430 | atcacatccgagcttattgtaca | 179822 | - | see also 15374 line |
| 35003 | FKBP3 | NM_002013.2 | 727 | gagggtccacaaatatactaa | 179846 | - | see also 1291 line |
| 35004 | FKBP3 | NM_002013.2 | 904 | aaggtcggagtaggcaaagttat | 179852 | - | see also 1291 line |
| 35005 | FKBP4 | NM_002014.2 | 522 | cccaatgccacgcttgtatttg | 179860 | - | see also 15376 line |
| 35006 | FKBP4 | NM_002014.2 | 597 | cggaatcattcgcagaatacaga | 179863 | - | see also 15376 line |
| 35007 | FKBP5 | NM_004117.2 | 737 | accacgacattccaattggaatt | 179882 | - | see also 2782 line |
| 35008 | FKBP5 | NM_004117.2 | 1358 | acgagcgggaccgcaggatatac | 179894 | - | see also 2782 line |
| 35009 | FKBP6 | NM_003602.2 | 601 | accttttccgccagaatcgttc | 179901 | - | see also 15378 line |
| 35010 | FKBP6 | NM_003602.2 | 625 | atgatgccaaagtgagatataaa | 179902 | - | see also 15378 line |

Figure 46

| 35011 | PPID | NM_005038.1 | 434 | atgatcgggaggggtttactgagc | 179921 | - | see also 15379 line | | |
|-------|------|-------------|-----|--------------------------|--------|---|---------------------|---|---|
| 35012 | PPID | NM_005038.1 | 898 | aagctgcaacctatagctttaag | 179934 | - | see also 15379 line | | |
| 35013 | OPRS1 | NM_005866.2 | 663 | ctcaccctcttctatactcttcg | 179947 | - | receptor_acitivity | - | - |
| 35014 | APOL5 | NM_030642.1 | 387 | cactcacgagttgcttaccaaga | 179950 | - | see also 15380 line | | |
| 35015 | APOL5 | NM_030642.1 | 1098 | tcgaggatcccgtgtggttaaac | 179964 | - | see also 15380 line | | |
| 35016 | ANKRA2 | NM_023039.2 | 1142 | ggctactcgtatcgaacaagaaa | 179976 | - | see also 15381 line | | |
| 35017 | ANKRA2 | NM_023039.2 | 1145 | tactcgtatcgaacaagaaaatg | 179977 | - | see also 15381 line | | |
| 35018 | SCARF1 | NM_003693.2 | 1824 | ttcgcggaaggtaccaagtttgc | 179994 | - | see also 15382 line | | |
| 35019 | SCARF1 | NM_003693.2 | 2310 | cccaactcagccccaaaagctgg | 179998 | - | see also 15382 line | | |
| 35020 | PTGDS | NM_000954.5 | 534 | gaccccagggctgagttaaagg | 179999 | - | - | transporter | - |
| 35021 | PTGDS | NM_000954.5 | 611 | tcctgccccaaacgataagtgc | 180001 | - | see also 35020 line | | |
| 35022 | PTGDS | NM_000954.5 | 537 | ccccagggctgagttaaaggaga | 180000 | - | see also 35020 line | | |
| 35023 | LAG3 | NM_002286.4 | 1781 | tggcgaccaagacgattttctgc | 180012 | - | see also 15383 line | | |
| 35024 | LAG3 | NM_002286.4 | 1278 | ctggagacaatggcgactttacc | 180006 | - | see also 15383 line | | |
| 35025 | LILRA3 | NM_006865.2 | 1114 | gagggatgcacactttccttttg | 180014 | - | see also 15384 line | | |
| 35026 | SLAMF1 | NM_003037.1 | 405 | atctaggagatcgctacaagttt | 180022 | - | see also 15385 line | | |
| 35027 | SLAMF1 | NM_003037.1 | 922 | aggtaaaacgaaccattaccaga | 180032 | - | see also 15385 line | | |
| 35028 | VPREB1 | NM_007128.2 | 229 | tgctgagatatttctcacaatca | 180036 | - | - | - | - |
| 35029 | HSPC182 | NM_014188.1 | 209 | tcccgacaagcccaatgtttatg | 180038 | - | - | - | - |
| 35030 | HSPC182 | NM_014188.1 | 208 | ctcccgacaagcccaatgtttat | 180037 | - | see also 35029 line | | |
| 35031 | HSPC182 | NM_014188.1 | 210 | cccgacaagcccaatgtttatga | 180039 | - | see also 35029 line | | |
| 35032 | OTC | NM_000531.3 | 1260 | gccggatgctagtgtaaccaagt | 180065 | - | see also 402 line | | |
| 35033 | PP591 | NM_025207.2 | 1254 | gaggtataatctgcagatgttgg | 180083 | - | see also 15387 line | | |
| 35034 | PP591 | NM_025207.2 | 837 | ctggggcagcaactactatcagg | 180077 | - | see also 15387 line | | |
| 35035 | DIO1 | NM_000792.2 | 176 | gccacgacaactggataccaacc | 180086 | - | see also 15388 line | | |
| 35036 | DIO1 | NM_000792.2 | 676 | atcctctacaagggtaaatctgg | 180097 | - | see also 15388 line | | |
| 35037 | DIO1 | NM_000792.2 | 181 | gacaactggataccaaccttttt | 180087 | - | see also 15388 line | | |
| 35038 | DIO2 | NM_000793.2 | 1033 | ggctgaccgcatggacaataacg | 180104 | - | see also 15389 line | | |
| 35039 | DIO2 | NM_000793.2 | 403 | gggcatcctcagcgtagacttgc | 180098 | - | see also 15389 line | | |
| 35040 | SELENBP1 | NM_003944.2 | 595 | ctgcaccgttgggctatgacttc | 180110 | - | see also 15390 line | | |
| 35041 | SELENBP1 | NM_003944.2 | 717 | tacgggagccacttatatgtatg | 180117 | - | see also 15390 line | | |
| 35042 | 15.Sep | NM_004261.2 | 423 | aactgagcattctcaaatggaac | 180125 | - | see also 15391 line | | |
| 35043 | 15.Sep | NM_004261.2 | 298 | ccctcaagtccaagcttttgtta | 180122 | - | see also 15391 line | | |
| 35044 | SEPP1 | NM_005410.1 | 245 | aagacctgcgagtaaaactgaag | 180131 | - | see also 15392 line | | |
| 35045 | SEPP1 | NM_005410.1 | 450 | tagatgtggccgtcttgtatatc | 180138 | - | see also 15392 line | | |
| 35046 | ERAF | NM_016633.1 | 74 | ggctcttcttaaggccaataagg | 180167 | - | see also 15393 line | | |
| 35047 | ERAF | NM_016633.1 | 193 | tggaggactggatgaacttctac | 180169 | - | see also 15393 line | | |

Figure 46

| | | | | | | | |
|---|---|---|---|---|---|---|---|
| 35048 | HPR | NM_020995.2 | 37 | tggggacgacagcttttttgcact | 180172 | - | see also 15395 line |
| 35049 | HPR | NM_020995.2 | 731 | ctgtggctgaccaatacgattgc | 180175 | - | see also 15395 line |
| 35050 | OBP2A | NM_014582.1 | 360 | gacggacgactacgtcttttact | 180179 | - | -                transporter                - |
| 35051 | PLUNC | NM_016583.2 | 464 | caccatccctctcggcataaagc | 180182 | - | see also 15396 line |
| 35052 | PLUNC | NM_016583.2 | 777 | atcaccctggtgcatgacattgt | 180188 | - | see also 15396 line |
| 35053 | TLR4 | NM_003266.2 | 1114 | ctctagagggcctgtgcaatttg | 180215 | - | see also 15397 line |
| 35054 | TLR4 | NM_003266.2 | 1150 | tccgattagcatacttagactac | 180218 | - | see also 15397 line |
| 35055 | NOP5/NOP58 | NM_015934.3 | 588 | gtctcgatatagattgaagttta | 180303 | - | see also 6576 line |
| 35056 | NOP5/NOP58 | NM_015934.3 | 580 | cacagcctgtctcgatatagatt | 180300 | - | see also 6576 line |
| 35057 | AKR1C2 | NM_001354.3 | 131 | gccgtcaaattggcaatagaagc | 180328 | - | see also 15399 line |
| 35058 | AKR1C2 | NM_001354.3 | 168 | ttgattctgcacatgtttacaat | 180329 | - | see also 15399 line |
| 35059 | ALOX5AP | NM_001629.2 | 329 | tgctggactgatgtacttgtttg | 180332 | - | see also 15400 line |
| 35060 | ALOX5AP | NM_001629.2 | 198 | ttccagaggaccggaacacttgc | 180331 | - | see also 15400 line |
| 35061 | AMBP | NM_001633.2 | 329 | ttcaatatctctcggatctatgg | 180337 | - | see also 1082 line |
| 35062 | AMBP | NM_001633.2 | 505 | gacgtctggagcttatgagaaaa | 180339 | - | see also 1082 line |
| 35063 | CGI-69 | NM_016016.1 | 783 | agccctgtactggttcaactatg | 180356 | - | see also 15402 line |
| 35064 | CGI-69 | NM_016016.1 | 782 | cagccctgtactggttcaactat | 180355 | - | see also 15402 line |
| 35065 | CNK2 | NM_014927.1 | 2588 | cacgacctccctcgatgagttgc | 180419 | - | see also 6096 line |
| 35066 | CRP | NM_000567.1 | 205 | ccctcaaagcaccgttaacgaag | 180441 | - | see also 15403 line |
| 35067 | CRP | NM_000567.1 | 175 | ttcccaaagagtcggatacttcc | 180440 | - | see also 15403 line |
| 35068 | DKFZP434N1235 | NM_031291.1 | 577 | aacccgattaggtgtcgatattg | 180461 | - | see also 9004 line |
| 35069 | DKFZP434N1235 | NM_031291.1 | 578 | acccgattaggtgtcgatattgg | 180462 | - | see also 9004 line |
| 35070 | DKFZp547P234 | NM_153045.2 | 1223 | cacacgaactctccgagaattcc | 180500 | - | see also 15405 line |
| 35071 | DKFZp547P234 | NM_153045.2 | 417 | ttctccggattgacaatacctgt | 180490 | - | see also 15405 line |
| 35072 | DOC2A | NM_003586.1 | 521 | gcctgtaaggccaataagctaaa | 180503 | - | see also 15406 line |
| 35073 | DOC2A | NM_003586.1 | 523 | ctgtaaggccaataagctaaaaa | 180504 | - | see also 15406 line |
| 35074 | FLJ10618 | NM_018155.1 | 763 | ctggtatatcagagactgttatc | 180527 | - | see also 15407 line |
| 35075 | FLJ10618 | NM_018155.1 | 695 | atgtgttcgtaaagtgtatcaga | 180523 | - | see also 15407 line |
| 35076 | FLJ12687 | NM_024917.4 | 2045 | cccgtgccggactgcattacaag | 180568 | - | see also 8805 line |
| 35077 | FLJ12687 | NM_024917.4 | 1373 | tggcgcagtactatgaagtattc | 180550 | - | see also 8805 line |
| 35078 | FLJ20551 | NM_017875.1 | 607 | tggttcgcacggagagtcttttg | 180578 | - | see also 15409 line |
| 35079 | FLJ20551 | NM_017875.1 | 428 | cacggtggaaacgcttatgttac | 180577 | - | see also 15409 line |

Figure 46

| | | | | | | | |
|---|---|---|---|---|---|---|---|
| 35080 | GIF | NM_005142.2 | 1165 | atcaacaatatcgcggaaaatgt | 180634 | - | see also 3613 line |
| 35081 | GIF | NM_005142.2 | 795 | tacggatatgatactcaatgaga | 180613 | - | see also 3613 line |
| 35082 | HEBP1 | NM_015987.2 | 536 | cagtgacaaaagcgttaagattg | 180642 | - | see also 15411 line |
| 35083 | HEBP1 | NM_015987.2 | 293 | ggcctgtgaaggcggcaaatttg | 180639 | - | see also 15411 line |
| 35084 | HPX | NM_000613.1 | 241 | tgggaccgggagttaatctcaga | 180645 | - | see also 15412 line |
| 35085 | HPX | NM_000613.1 | 597 | ctgcttccagggtaaccaattcc | 180649 | - | see also 15412 line |
| 35086 | LOC153328 | NM_145282.1 | 280 | gagtatgttcggcttcttcaagg | 180657 | - | see also 15413 line |
| 35087 | LOC153328 | NM_145282.1 | 345 | tggtgtttggggtcttcagtaac | 180658 | - | see also 15413 line |
| 35088 | LOC203427 | NM_145305.1 | 606 | gccgcctaccgcaaatttgttgt | 180660 | - | - | - | - |
| 35089 | LOC203427 | NM_145305.1 | 608 | cgcctaccgcaaatttgttgtgc | 180661 | - | see also 35088 line |
| 35090 | LOC203427 | NM_145305.1 | 1154 | agccaatttactgaagatagttc | 180662 | - | see also 35088 line |
| 35091 | LOC283130 | NM_182556.1 | 485 | cccgtttgacactgtaaagctcc | 180666 | - | see also 15414 line |
| 35092 | LOC283130 | NM_182556.1 | 1177 | accttcctcagctacgaatatct | 180670 | - | see also 15414 line |
| 35093 | LOC91137 | NM_138773.1 | 438 | gccgccaatgtcaggttaattac | 180675 | - | see also 9630 line |
| 35094 | LOC91137 | NM_138773.1 | 1104 | cacgcacaataattgacaataca | 180689 | - | see also 9630 line |
| 35095 | LUZP1 | NM_033631.1 | 1879 | gacaacacgaacgtttagtgaca | 180724 | - | see also 9429 line |
| 35096 | LUZP1 | NM_033631.1 | 397 | cagccgccacttgcggtttaagc | 180697 | - | see also 9429 line |
| 35097 | MCFP | NM_018843.1 | 943 | tgggctcctactgttcttagaga | 180783 | - | see also 7599 line |
| 35098 | MCFP | NM_018843.1 | 563 | gaggcaacaaactatggtataag | 180772 | - | see also 7599 line |
| 35099 | MFTC | NM_030780.2 | 924 | aaggcgtcggtggattttacaag | 180825 | - | see also 8944 line |
| 35100 | MFTC | NM_030780.2 | 637 | aggtgtgcgtggattatataagg | 180815 | - | see also 8944 line |
| 35101 | MSCP | NM_016612.1 | 359 | aggcgtcaacgtcatgatcatgg | 180836 | - | see also 15419 line |
| 35102 | MSCP | NM_016612.1 | 550 | agcgcttgcagatgtacaactcg | 180840 | - | see also 15419 line |
| 35103 | MTCH1 | NM_014341.1 | 492 | gactcggggtagcatgaagaagg | 180850 | - | see also 15420 line |
| 35104 | MTCH1 | NM_014341.1 | 870 | ggccatccggagctataccaagt | 180856 | - | see also 15420 line |
| 35105 | MTCH1 | NM_014341.1 | 497 | ggggtagcatgaagaaggtttc | 180851 | - | see also 15420 line |
| 35106 | NALP14 | NM_176822.2 | 2163 | ccctgatggttgtcaggatatct | 180902 | - | see also 15421 line |
| 35107 | NALP14 | NM_176822.2 | 3221 | gagttagcaatccacacttaatc | 180932 | - | see also 15421 line |
| 35108 | SEC14L1 | NM_003003.1 | 610 | tccaatcgggtcatcattaatga | 180937 | - | see also 2015 line |
| 35109 | SEC14L1 | NM_003003.1 | 1639 | acgctggttagtccgttcattga | 180952 | - | see also 2015 line |
| 35110 | SFRS16 | NM_007056.1 | 2047 | cccgatacagtcgagaatacagc | 180988 | - | see also 15423 line |

Figure 46

| 35111 | SFRS16 | NM_007056.1 | 2045 | cccccgatacagtcgagaataca | 180987 | - | see also 15423 line | | |
|---|---|---|---|---|---|---|---|---|---|
| 35112 | SFRS16 | NM_007056.1 | 413 | gtcggacgaacggaagtgtaact | 180977 | - | see also 15423 line | | |
| 35113 | SLC25A1 | NM_005984.1 | 393 | ggccgtcaggtttggaatgttcg | 180989 | - | see also 15424 line | | |
| 35114 | SLC25A1 | NM_005984.1 | 811 | aacactcctctggatgtgattaa | 180990 | - | see also 15424 line | | |
| 35115 | SLC25A10 | NM_012140.3 | 402 | ggctccgtcagcggtttagctgg | 180993 | - | - | dicarboxylic acid transporter | - |
| 35116 | SLC25A10 | NM_012140.3 | 884 | caccgtgctcacttttgtgtttc | 180995 | - | see also 35115 line | | |
| 35117 | SLC25A10 | NM_012140.3 | 885 | accgtgctcacttttgtgtttct | 180996 | - | see also 35115 line | | |
| 35118 | SLC25A14 | NM_003951.2 | 600 | ttcgtagaacgtttagaagatga | 181025 | - | see also 2699 line | | |
| 35119 | SLC25A14 | NM_003951.2 | 979 | tggttcgaactcgcatgatgaac | 181042 | - | see also 2699 line | | |
| 35120 | SLC25A15 | NM_014252.1 | 616 | gtgtggtctgtcatcaaaagtat | 181057 | - | see also 15427 line | | |
| 35121 | SLC25A15 | NM_014252.1 | 618 | gtggtctgtcatcaaaagtattc | 181058 | - | see also 15427 line | | |
| 35122 | SLC25A15 | NM_014252.1 | 893 | aacaggcaggatttatcagaacc | 181062 | - | see also 15427 line | | |
| 35123 | SLC25A16 | NM_152707.2 | 1241 | gactcgtcggcgaatgcaattag | 181092 | - | see also 15428 line | | |
| 35124 | SLC25A16 | NM_152707.2 | 1239 | gtgactcgtcggcgaatgcaatt | 181091 | - | see also 15428 line | | |
| 35125 | SLC25A17 | NM_006358.2 | 807 | ctgcagacggtacagtcaattct | 181122 | - | see also 15429 line | | |
| 35126 | SLC25A17 | NM_006358.2 | 830 | gaggtttgggcgtcatagactaa | 181124 | - | see also 15429 line | | |
| 35127 | SLC25A18 | NM_031481.1 | 1384 | ttgctcaaggggtctattttatt | 181139 | - | see also 15430 line | | |
| 35128 | SLC25A18 | NM_031481.1 | 1414 | gagagcgcatcttaaagtgtttt | 181142 | - | see also 15430 line | | |
| 35129 | SLC25A19 | NM_021734.3 | 708 | cacgccgtggggaccatgtatag | 181151 | - | see also 15431 line | | |
| 35130 | SLC25A19 | NM_021734.3 | 329 | cagtcccttcgacgtcatcaaga | 181144 | - | see also 15431 line | | |
| 35131 | SLC25A2 | NM_031947.1 | 215 | tggcctacgtcgccgaaaactcg | 181157 | - | see also 15432 line | | |
| 35132 | SLC25A2 | NM_031947.1 | 466 | gtcgtgaagggtatccttaaaaa | 181159 | - | see also 15432 line | | |
| 35133 | SLC25A20 | NM_000387.2 | 422 | ctcctggagaacggatcaagtgc | 181172 | - | see also 15433 line | | |
| 35134 | SLC25A20 | NM_000387.2 | 524 | ttgggatccgaggcatctacaaa | 181174 | - | see also 15433 line | | |
| 35135 | SLC25A21 | NM_030631.1 | 452 | accgatccaaacagttataaaag | 181187 | - | see also 15434 line | | |
| 35136 | SLC25A21 | NM_030631.1 | 804 | tccagggcctcaacaaaggatta | 181194 | - | see also 15434 line | | |
| 35137 | SLC25A26 | NM_173471.1 | 329 | agggttgtatcgaggctataaaa | 181207 | - | see also 15435 line | | |
| 35138 | SLC25A26 | NM_173471.1 | 330 | gggttgtatcgaggctataaaag | 181208 | - | see also 15435 line | | |
| 35139 | SLC25A27 | NM_004277.2 | 302 | ccccgagcgagcaaattcctact | 181215 | - | see also 15436 line | | |
| 35140 | SLC25A27 | NM_004277.2 | 519 | gagtgacacccgccatttacaga | 181218 | - | see also 15436 line | | |
| 35141 | SLC25A28 | NM_031212.2 | 1404 | ttcaggacggtatatcaagtagg | 181228 | - | see also 8991 line | | |
| 35142 | SLC25A28 | NM_031212.2 | 1448 | aggggtgcaggccagagtaattt | 181230 | - | see also 8991 line | | |
| 35143 | SLC25A3 | NM_002635.1 | 520 | ctctggcgcacatcactatattt | 181242 | - | see also 15438 line | | |
| 35144 | SLC25A3 | NM_002635.1 | 599 | ctgctaaggttcgaattcaaacc | 181244 | - | see also 15438 line | | |
| 35145 | SLC25A4 | NM_001151.1 | 671 | tacttcggagtctatgatactgc | 181271 | - | see also 15439 line | | |
| 35146 | SLC25A4 | NM_001151.1 | 111 | atcacgcttggagcttcctaaag | 181265 | - | see also 15439 line | | |

Figure 46

| | | | | | | | |
|---|---|---|---|---|---|---|---|
| 35147 | SLC25A5 | NM_001152.1 | 670 | tgcttccggatcccaagaacact | 181277 | - | see also 15440 line |
| 35148 | SLC39A1 | NM_014437.3 | 1354 | ggccagttctgagcaaaggatcc | 181279 | - | - | tryptase | - |
| 35149 | SLC40A1 | NM_014585.3 | 1652 | atccgatcaaggttcattcaagg | 181311 | - | see also 5765 line |
| 35150 | SLC40A1 | NM_014585.3 | 2083 | tgcttgcggtcctgatgcaaaag | 181327 | - | see also 5765 line |
| 35151 | TCN1 | NM_001062.2 | 387 | atgtcgtaacgctgaggaaaact | 181341 | - | see also 15442 line |
| 35152 | TCN1 | NM_001062.2 | 558 | ctcaaccgccgaagttgtcaacc | 181352 | - | see also 15442 line |
| 35153 | TCN2 | NM_000355.2 | 1281 | tccttgtcaggcccctacttaac | 181384 | - | see also 15443 line |
| 35154 | TCN2 | NM_000355.2 | 772 | gtctgaagcgctcaaacttcaac | 181378 | - | see also 15443 line |
| 35155 | TNC | NM_002160.1 | 6408 | gacgcgagaacttctaccaaaac | 181459 | - | see also 1385 line |
| 35156 | TNC | NM_002160.1 | 5559 | gaggtacaccctccatggtaact | 181451 | - | see also 1385 line |
| 35157 | TNC | NM_002160.1 | 3348 | ttgaccgctaccgcctcaattac | 181415 | - | see also 1385 line |
| 35158 | UCP1 | NM_021833.3 | 578 | ctcgctacacggggacttataat | 181479 | - | see also 15445 line |
| 35159 | UCP1 | NM_021833.3 | 267 | gacgtccagtgttattaggtata | 181472 | - | see also 15445 line |
| 35160 | UCP2 | NM_003355.2 | 488 | tgctaaagtccggttacagatcc | 181502 | - | see also 2261 line |
| 35161 | UCP3 | NM_003356.2 | 966 | agcccctcgactgtatgataaa | 181511 | - | see also 15447 line |
| 35162 | UCP3 | NM_003356.2 | 967 | gcccctcgactgtatgataaag | 181512 | - | see also 15447 line |
| 35163 | UCP3 | NM_003356.2 | 968 | cccctcgactgtatgataaaga | 181513 | - | see also 15447 line |
| 35164 | VWF | NM_000552.2 | 2664 | ggctcgagtgtaccaaaacgtgc | 181532 | - | see also 15448 line |
| 35165 | VWF | NM_000552.2 | 2661 | aagggctcgagtgtaccaaaacg | 181531 | - | see also 15448 line |
| 35166 | TPSB2 | NM_024164.2 | 561 | accacatttgtgacgcaaaatac | 331465 | - | - | tryptase | - |
| 35167 | TPSB2 | NM_024164.2 | 778 | cacccgtgtcacctactacttgg | 331466 | - | see also 35166 line |
| 35168 | TPSB2 | NM_024164.2 | 539 | gtgaaggtccccataatggaaaa | 331464 | - | see also 35166 line |
| 35169 | ACR | NM_001097.1 | 280 | ctgcttcgtcggcaaaaataatg | 181596 | - | see also 15449 line |
| 35170 | ACR | NM_001097.1 | 421 | ctctgcgacagagggaaatgaca | 181597 | - | see also 15449 line |
| 35171 | BF | NM_001710.3 | 2424 | ctcacgcccgagactttcacatc | 181636 | - | see also 15450 line |
| 35172 | BF | NM_001710.3 | 1985 | cagactatcaggcccatttgtct | 181630 | - | see also 15450 line |
| 35173 | C1RL | NM_016546.1 | 1403 | tggcgaagggtatgacttctaca | 181659 | - | see also 6912 line |
| 35174 | C1RL | NM_016546.1 | 501 | accgtggctgtgaactatagtca | 181647 | - | see also 6912 line |
| 35175 | C2 | NM_000063.3 | 646 | tgccgccaaccctactcttatga | 181665 | - | see also 15452 line |
| 35176 | C2 | NM_000063.3 | 905 | atgtgagcgttgccattatcacc | 181676 | - | see also 15452 line |
| 35177 | C6orf158 | NM_153362.1 | 1373 | cacgggaacgatgccaattgtgc | 181726 | - | see also 9992 line |
| 35178 | C6orf158 | NM_153362.1 | 1142 | gtgtccgacgaatccaatgatct | 181718 | - | see also 9992 line |
| 35179 | CMA1 | NM_001836.2 | 159 | ttccaacggtccctcaaaatttt | 181731 | - | see also 15454 line |
| 35180 | CMA1 | NM_001836.2 | 190 | ttccttataagacggaactttgt | 181733 | - | see also 15454 line |
| 35181 | CMA1 | NM_001836.2 | 352 | caccacgatatcatgttactaaa | 181744 | - | see also 15454 line |
| 35182 | CRN | NM_006587.2 | 3162 | gtcatatttcgtcgaatggatta | 181823 | - | see also 4614 line |
| 35183 | CRN | NM_006587.2 | 2983 | atcaccactcggatgatatgtgc | 181814 | - | see also 4614 line |

Figure 46

| 35184 | CTRC | NM_007272.1 | 171 | tacgtgtggcgggactttgattg | 181825 | - | see also 15456 line | | |
|---|---|---|---|---|---|---|---|---|---|
| 35185 | CTRC | NM_007272.1 | 735 | ccgcaagaagccggtagtctaca | 181831 | - | see also 15456 line | | |
| 35186 | CTRL | NM_001907.1 | 272 | ggcgagtatgaccgatcatcaaa | 181833 | - | see also 15457 line | | |
| 35187 | CTRL | NM_001907.1 | 317 | tccgtctctcgggccattacaca | 181835 | - | see also 15457 line | | |
| 35188 | CTSG | NM_001911.2 | 385 | aagagtcagacggaatcgaaacg | 181848 | - | see also 15458 line | | |
| 35189 | CTSG | NM_001911.2 | 133 | cccctacatggcgtatcttcaga | 181843 | - | see also 15458 line | | |
| 35190 | DKFZP586H2123 | NM_015430.1 | 998 | aacggacgccatgctaaaattgg | 181878 | - | see also 6414 line | | |
| 35191 | DKFZP586H2123 | NM_015430.1 | 995 | atcaacggacgccatgctaaaat | 181877 | - | see also 6414 line | | |
| 35192 | DKFZp686L1818 | NM_182502.1 | 1343 | gacttcttatcgcaattggatta | 181937 | - | see also 15460 line | | |
| 35193 | DKFZp686L1818 | NM_182502.1 | 172 | cactatggactacgatctttatt | 181898 | - | see also 15460 line | | |
| 35194 | DKFZp686L1818 | NM_182502.1 | 881 | gacacggaaagtccaaaacatta | 181925 | - | see also 15460 line | | |
| 35195 | ELA2 | NM_001972.1 | 358 | acggctacgaccccgtaaacttg | 181942 | - | see also 15461 line | | |
| 35196 | ELA2 | NM_001972.1 | 248 | ctgcgtggcgaatgtaaacgtcc | 181941 | - | see also 15461 line | | |
| 35197 | ELA2A | NM_033440.1 | 82 | ccccacttacccaccttatgtga | 181945 | - | - | trypsin | - |
| 35198 | ELA2B | NM_015849.1 | 82 | ctccacttacgcgcctgatatgt | 181946 | - | see also 15462 line | | |
| 35199 | ELA2B | NM_015849.1 | 604 | cagcaccgtgaagacgaatatga | 181948 | - | see also 15462 line | | |
| 35200 | ELA3A | NM_005747.2 | 555 | gcccgtggtggactataagcact | 181952 | - | - | trypsin | - |
| 35201 | ELA3B | NM_007352.1 | 558 | gccggtggtggactatgaacact | 181954 | - | see also 15463 line | | |
| 35202 | F11 | NM_000128.2 | 379 | atcccacccgatggtttacttgt | 181959 | - | see also 15464 line | | |
| 35203 | F11 | NM_000128.2 | 701 | cagaataacgaagctcgataaag | 181972 | - | see also 15464 line | | |
| 35204 | FLJ90661 | NM_173502.1 | 2219 | ctccatcttgacacaacgaatct | 182008 | - | see also 15465 line | | |
| 35205 | FLJ90661 | NM_173502.1 | 1952 | gccttacattgaagtgtatctgg | 182007 | - | see also 15465 line | | |
| 35206 | FLJ90724 | NM_153692.2 | 1093 | atgtgattggcgtcaattcattg | 182026 | - | see also 15466 line | | |
| 35207 | FLJ90724 | NM_153692.2 | 714 | gacgggctcattattaccaatgc | 182014 | - | see also 15466 line | | |
| 35208 | GZMA | NM_006144.2 | 365 | cacacgcgaaggtgaccttaaac | 182050 | - | see also 15467 line | | |
| 35209 | GZMA | NM_006144.2 | 515 | tgcatcttggtccgatactctga | 182055 | - | see also 15467 line | | |
| 35210 | GZMA | NM_006144.2 | 157 | gaccctacatggtcctacttagt | 182043 | - | see also 15467 line | | |
| 35211 | GZMB | NM_004131.3 | 715 | gtctcctatggacgaaacaatgg | 182070 | - | see also 15468 line | | |
| 35212 | GZMB | NM_004131.3 | 588 | atctgacttacgccattattacg | 182069 | - | see also 15468 line | | |
| 35213 | GZMH | NM_033423.2 | 192 | tggcggcatcctagtgagaaagg | 182075 | - | see also 15469 line | | |
| 35214 | GZMH | NM_033423.2 | 237 | ctgccagggaagctccataaatg | 182076 | - | see also 15469 line | | |
| 35215 | GZMK | NM_002104.1 | 506 | gccaccgatccagattcattaag | 182100 | - | see also 15470 line | | |
| 35216 | GZMK | NM_002104.1 | 241 | ctgccaatatcggtttaccaaag | 182087 | - | see also 15470 line | | |

Figure 46

| | | | | | | | | | | |
|---|---|---|---|---|---|---|---|---|---|---|
| 35217 | HAT | NM_004262.1 | 584 | gccggtccagacctaataacatt | 182133 | - | see also 15472 line | | | |
| 35218 | HAT | NM_004262.1 | 75 | cacgtgtaacttcgacttcaaga | 182111 | - | see also 15472 line | | | |
| 35219 | HGFAC | NM_001528.2 | 1896 | cacccgcgtggccaactatgtgg | 182162 | - | see also 1049 line | | | |
| 35220 | HPN | NM_002151.1 | 999 | gaggagaacagcaacgatattgc | 182164 | - | see also 15474 line | | | |
| 35221 | HPN | NM_002151.1 | 1439 | gtggatcttccaggccataaaga | 182170 | - | see also 15474 line | | | |
| 35222 | IF | NM_000204.1 | 1218 | cccgaccttaaacgtatagtaat | 182207 | - | see also 15475 line | | | |
| 35223 | IF | NM_000204.1 | 1217 | ccccgaccttaaacgtatagtaa | 182206 | - | see also 15475 line | | | |
| 35224 | KLK1 | NM_002257.2 | 738 | cccttgtggcacccccaataagc | 182238 | - | see also 15476 line | | | |
| 35225 | KLK1 | NM_002257.2 | 769 | gccgtcagagtgctgtcttatgt | 182240 | - | see also 15476 line | | | |
| 35226 | KLK1 | NM_002257.2 | 768 | cgccgtcagagtgctgtcttatg | 182239 | - | see also 15476 line | | | |
| 35227 | KLK10 | NM_002776.3 | 318 | ggcgctgctcccccaaaacgaca | 182241 | - | cell_cycle | - | | - |
| 35228 | KLK10 | NM_002776.3 | 615 | aacggatgagcacgatctcatgt | 182242 | - | see also 35227 line | | | |
| 35229 | KLK11 | NM_006853.2 | 825 | cacgaaagtctgcaaatatgtgg | 182244 | - | - | - | | - |
| 35230 | KLK11 | NM_006853.2 | 823 | tacacgaaagtctgcaaatatgt | 182243 | - | see also 35229 line | | | |
| 35231 | KLK12 | NM_019598.2 | 168 | agccacaccgaagattttcaatg | 182249 | - | see also 15477 line | | | |
| 35232 | KLK12 | NM_019598.2 | 163 | caggcagccacaccgaagatttt | 182246 | - | see also 15477 line | | | |
| 35233 | KLK13 | NM_015596.1 | 262 | tgccgcacactgtctaaaggagg | 182254 | - | see also 15478 line | | | |
| 35234 | KLK13 | NM_015596.1 | 644 | atgttgtgtgccggcacaaaaga | 182258 | - | see also 15478 line | | | |
| 35235 | KLK13 | NM_015596.1 | 816 | tccgtgaaacaatccgaaaatat | 182259 | - | see also 15478 line | | | |
| 35236 | KLK14 | NM_022046.3 | 749 | ctctgcaatgcgtgaacatcaac | 182263 | - | see also 15479 line | | | |
| 35237 | KLK14 | NM_022046.3 | 752 | tgcaatgcgtgaacatcaacatc | 182264 | - | see also 15479 line | | | |
| 35238 | KLK15 | NM_017509.2 | 150 | acgagcgtggacgctttaactgt | 182266 | - | see also 15480 line | | | |
| 35239 | KLK2 | NM_005551.2 | 660 | gacacttgtgggggtgattctgg | 182268 | - | - | trypsin | prostate cancer | |
| 35240 | KLK4 | NM_004917.2 | 135 | ggcggcactggtcatggaaaacg | 182278 | - | see also 15481 line | | | |
| 35241 | KLK4 | NM_004917.2 | 308 | tacggcacccagagtacaacaga | 182281 | - | see also 15481 line | | | |
| 35242 | KLK5 | NM_012427.3 | 358 | gagcatgttctcgccaacaatga | 182283 | - | see also 15482 line | | | |
| 35243 | KLK5 | NM_012427.3 | 492 | tggatccgactgcgatatgcaca | 182285 | - | see also 15482 line | | | |
| 35244 | KLK6 | NM_002774.2 | 431 | ctgcaaaaaaccgaatcttcagg | 182300 | - | see also 15483 line | | | |
| 35245 | KLK6 | NM_002774.2 | 704 | caccatccagtgtgcatacatcc | 182304 | - | see also 15483 line | | | |
| 35246 | KLK7 | NM_005046.2 | 414 | ctccacacagacccatgttaatg | 182308 | - | see also 15484 line | | | |
| 35247 | KLK7 | NM_005046.2 | 332 | ccgtgcacctgggcagtgatacg | 182307 | - | see also 15484 line | | | |
| 35248 | KLK9 | NM_012315.1 | 397 | agcgccaatgaccacaatgatga | 182321 | - | see also 15486 line | | | |
| 35249 | KLK9 | NM_012315.1 | 396 | cagcgccaatgaccacaatgatg | 182320 | - | see also 15486 line | | | |
| 35250 | KLKB1 | NM_000892.2 | 593 | aacacctaccgctataaaggtgc | 182342 | - | see also 15487 line | | | |
| 35251 | KLKB1 | NM_000892.2 | 1396 | aggatgtttggcgcatctatagt | 182362 | - | see also 15487 line | | | |
| 35252 | LOC339967 | NM_182606.1 | 1135 | ttctgtgccggatatatggaagg | 182401 | - | see also 15488 line | | | |

Figure 46

| | | | | | | | |
|---|---|---|---|---|---|---|---|
| 35253 | LOC339967 | NM_182606.1 | 1102 | caggtgtatggcaatgatataaa | 182399 | - | see also 15488 line |
| 35254 | MGC34695 | NM_173555.1 | 777 | gccttctgcccgacatcttct | 182405 | - | see also 15489 line |
| 35255 | MGC52282 | NM_178453.2 | 259 | acctcagtgggcatgtatgtgg | 182406 | - | see also 15490 line |
| 35256 | MGC57341 | NM_182559.1 | 249 | ttgcaagggtctcggattatagg | 182410 | - | see also 10269 line |
| 35257 | MGC57341 | NM_182559.1 | 50 | gagcgtggcgctgttgtttgtgg | 182409 | | see also 10269 line |
| 35258 | MPN | NM_031948.2 | 652 | ttggctaccaaccaaaaccatc | 182440 | - | see also 15492 line |
| 35259 | MPN | NM_031948.2 | 341 | gccggaccacacgctatgtatg | 182436 | - | see also 15492 line |
| 35260 | MSP | NM_032046.1 | 1675 | agcgaggtgcgattcagaaaatc | 182454 | - | see also 15493 line |
| 35261 | MSP | NM_032046.1 | 1674 | gagcgaggtgcgattcagaaaat | 182453 | - | see also 15493 line |
| 35262 | PLAT | NM_000930.2 | 1625 | tccagccgctgcacatcacaaca | 182464 | - | see also 15494 line |
| 35263 | PLAT | NM_000930.2 | 768 | agccctggctacgtctttag | 182460 | - | see also 15494 line |
| 35264 | PLAU | NM_002658.1 | 792 | tgggtgctcaaggcttaactc | 182473 | - | see also 15495 line |
| 35265 | PLAU | NM_002658.1 | 945 | agccatccggactatacagacc | 182476 | - | see also 15495 line |
| 35266 | PRSS12 | NM_003619.2 | 1237 | agggtcgcttggaggtatattac | 182502 | - | see also 2431 line |
| 35267 | PRSS12 | NM_003619.2 | 164 | gggtccgcactacccctattacc | 182483 | - | see also 2431 line |
| 35268 | PRSS21 | NM_006799.2 | 455 | accgttacttcgtctcgaatat | 182537 | - | see also 15497 line |
| 35269 | PRSS21 | NM_006799.2 | 454 | caccgttacttcgtatcgaata | 182536 | - | see also 15497 line |
| 35270 | PRSS21 | NM_006799.2 | 456 | cccgttacttcgtatcgaatatc | 182538 | - | see also 15498 line |
| 35271 | PRSS22 | NM_022119.2 | 475 | gtctgagcgctccatacagttc | 182558 | - | see also 15498 line |
| 35272 | PRSS22 | NM_022119.2 | 228 | atcgtgagcatccagaagaatgg | 182555 | - | see also 15498 line |
| 35273 | PRSS25 | NM_013247.3 | 1027 | ctcccggagtcagtacaacttc | 182562 | - | see also 5366 line |
| 35274 | PRSS25 | NM_013247.3 | 1030 | cccggagtcagtacaacttcatc | 182564 | - | see also 5366 line |
| 35275 | PRSS25 | NM_013247.3 | 1028 | tccccggagtcagtacaacttca | 182563 | - | see also 5366 line |
| 35276 | PRSS7 | NM_002772.1 | 2783 | tggggacggttgtatatcaagg | 182674 | - | see also 15500 line |
| 35277 | PRSS7 | NM_002772.1 | 391 | cagcattatagtcgtatttgacc | 182603 | - | see also 15500 line |
| 35278 | PRSS8 | NM_002773.2 | 929 | gacgtgaactgcctgtacaaca | 182685 | - | see also 15501 line |
| 35279 | PRSS8 | NM_002773.2 | 962 | gcctgaggagccgcactttgtcc | 182686 | - | see also 15501 line |
| 35280 | PRTN3 | NM_002777.2 | 373 | aactacgacgcggagaacaaact | 182687 | - | see also 15502 line |
| 35281 | PRTN3 | NM_002777.2 | 377 | acgacgcggagaacaaactgaac | 182688 | - | see also 15502 line |
| 35282 | SPUVE | NM_007173.3 | 865 | atgggcatggattatgattatgc | 182698 | - | see also 15503 line |
| 35283 | SPUVE | NM_007173.3 | 781 | atgcccgagcagatgaaatttca | 182696 | - | see also 15503 line |
| 35284 | ST14 | NM_021978.2 | 1834 | aggtgaacgtgtcacttgtacc | 182721 | - | see also 15504 line |
| 35285 | ST14 | NM_021978.2 | 750 | cccacgactccaaaacagtaca | 182718 | - | see also 15504 line |

**EP 1 752 536 A1**

Figure 46

| | | | | | | | |
|---|---|---|---|---|---|---|---|
| 35286 | TMPRSS2 | NM_005656.2 | 772 | tgccggcaatgtcgatatctata | 182736 | - | see also 15505 line |
| 35287 | TMPRSS2 | NM_005656.2 | 774 | ccggcaatgtcgatatctataaa | 182738 | - | see also 15505 line |
| 35288 | TMPRSS3 | NM_024022.1 | 478 | gacggagtctggattgcaaaga | 182774 | - | see also 15506 line |
| 35289 | TMPRSS3 | NM_024022.1 | 437 | gtcgctcatctttaagtgtatc | 182772 | - | see also 15506 line |
| 35290 | TMPRSS4 | NM_019894.2 | 770 | agcttcgcatgcggaactcaagt | 182796 | - | see also 15507 line |
| 35291 | TMPRSS4 | NM_019894.2 | 1445 | ggcatgtgttgggcatcgttagc | 182805 | - | see also 15507 line |
| 35292 | TMPRSS5 | NM_030770.1 | 792 | cccctggcttcccggatagttgg | 182810 | - | see also 15508 line |
| 35293 | TMPRSS5 | NM_030770.1 | 1204 | cccaccctagccatacttacagc | 182812 | - | see also 15508 line |
| 35294 | TMPRSS5 | NM_030770.1 | 352 | ggctcctagtgctgtatctgtgt | 182807 | - | see also 15508 line |
| 35295 | TMPRSS6 | NM_153019.2 | 503 | ccgcctgggaacttactacaac | 182820 | - | see also 15509 line |
| 35296 | TMPRSS6 | NM_153019.2 | 501 | caccgcctgggaacttactaca | 182819 | - | see also 15509 line |
| 35297 | TPSD1 | NM_012217.1 | 649 | ctgtgtgcggggagcgaaaatca | 182835 | - | see also 15510 line |
| 35298 | TPSD1 | NM_012217.1 | 577 | cacctttcaacgcggaatatca | 182833 | - | see also 15510 line |
| 35299 | TPSG1 | NM_012467.1 | 389 | ctccaccgtgaggcagatcatcc | 182836 | - | trypsin |
| 35300 | TSP50 | NM_013270.2 | 511 | gcctgatcggcgtgatgttatc | 182837 | - | see also 15511 line |
| 35301 | TSP50 | NM_013270.2 | 691 | aggaactcaagtacagccaattac | 182838 | - | see also 15511 line |
| 35302 | C9orf43 | NM_152786.1 | 1465 | acgtctatagttacagaact | 182868 | - | see also 15512 line |
| 35303 | C9orf43 | NM_152786.1 | 614 | caccgttagacatcttagatt | 182842 | - | see also 15512 line |
| 35304 | C9orf43 | NM_152786.1 | 612 | ctcaccgttagaacatcttaga | 182841 | - | see also 15512 line |
| 35305 | FLJ10504 | NM_018116.2 | 170 | gccccgacgtcctgtatcgtacg | 182878 | - | see also 15513 line |
| 35306 | FLJ10504 | NM_018116.2 | 1280 | tggtgctgagggtatagacaga | 182883 | - | see also 15513 line |
| 35307 | FURIN | NM_002569.2 | 2081 | tcctgatacgcactatagcacc | 182906 | - | see also 1652 line |
| 35308 | FURIN | NM_002569.2 | 2559 | ggggcgagagagaccgcctttatca | 182907 | - | see also 1652 line |
| 35309 | MBTPS1 | NM_003791.1 | 2886 | cagaagatgccgtcgtgataaca | 182962 | - | see also 2590 line |
| 35310 | MBTPS1 | NM_003791.1 | 2025 | tgccgacagttgttaatgtcacc | 182943 | - | see also 2590 line |
| 35311 | PACE4 | NM_002570.2 | 1104 | atcgtgggcatagccgtacaatgc | 182991 | - | see also 15516 line |
| 35312 | PACE4 | NM_002570.2 | 990 | gacgtgaacggcaatgattatga | 182988 | - | see also 15516 line |
| 35313 | PACE4 | NM_002570.2 | 584 | ggcgcacgggtaccttcaacttgg | 182973 | - | see also 15516 line |
| 35314 | PCSK1 | NM_000439.3 | 1506 | atggtgaatagtcgatttggatt | 183046 | - | see also 346 line |
| 35315 | PCSK1 | NM_000439.3 | 442 | ctcgaaggagtgccttttcatatc | 183019 | - | see also 346 line |
| 35316 | PCSK2 | NM_002594.2 | 400 | gaccggtcttcacgaatcatttt | 183070 | - | see also 1675 line |
| 35317 | PCSK2 | NM_002594.2 | 1617 | gggggtcggcctggaatttaatca | 183094 | - | see also 1675 line |
| 35318 | PCSK4 | NM_017573.2 | 985 | gtccatggactccacttatacg | 183097 | - | - |
| 35319 | PCSK5 | NM_006200.2 | 2320 | ttccgctatagccgagttgaaga | 183149 | - | see also 15519 line |
| 35320 | PCSK5 | NM_006200.2 | 1557 | cagcggggagtcctacgataaga | 183126 | - | see also 15519 line |
| 35321 | PCSK7 | NM_004716.2 | 1666 | gtcagtcccgtgttaaaagaaaa | 183178 | - | see also 3237 line |
| 35322 | PCSK7 | NM_004716.2 | 1440 | tggcaatgagcctaatgctgc | 183175 | - | see also 3237 line |

Figure 46

| 35323 | PCSK9 | NM_174936.2 | 1640 | gggcaggttggcagctgttttgc | 183184 | - | see also 10133 line |
|---|---|---|---|---|---|---|---|
| 35324 | TNS | NM_022648.2 | 2209 | atgttccgctctcaatccttttc | 183200 | - | see also 15522 line |
| 35325 | TNS | NM_022648.2 | 1429 | cacacgcaggggccactagatgg | 183198 | - | see also 15522 line |
| 35326 | TNS | NM_022648.2 | 795 | tgcaatgaagcggttctatgagg | 183187 | - | see also 15522 line |
| 35327 | TPP2 | NM_003291.1 | 3741 | atcatgtatcctcccgattattg | 183335 | - | see also 2225 line |
| 35328 | TPP2 | NM_003291.1 | 1546 | atgactacctcgttcagaataca | 183269 | - | see also 2225 line |
| 35329 | APEH | NM_001640.3 | 1990 | tgcggtactactagtgaactatc | 183355 | - | see also 15524 line |
| 35330 | APEH | NM_001640.3 | 567 | aacgcatggagaacattcgattc | 183337 | - | see also 15524 line |
| 35331 | DPP4 | NM_001935.2 | 2078 | atcccggtgggagtactatgact | 183434 | - | see also 1234 line |
| 35332 | DPP4 | NM_001935.2 | 1460 | tccggaaaggtgtcagtactatt | 183410 | - | see also 1234 line |
| 35333 | DPP4 | NM_001935.2 | 226 | gtcgcaaaacttacactctaact | 183366 | - | see also 1234 line |
| 35334 | FAP | NM_004460.2 | 984 | atcccgttgttcggatatttatt | 183465 | - | see also 3010 line |
| 35335 | FAP | NM_004460.2 | 985 | tcccgttgttcggatatttatta | 183466 | - | see also 3010 line |
| 35336 | FAP | NM_004460.2 | 1574 | agcgactacgccaagtactatgc | 183485 | - | see also 3010 line |
| 35337 | PREP | NM_002726.2 | 924 | tcccaactatcgcgtgatcaaca | 183529 | - | see also 15527 line |
| 35338 | PREP | NM_002726.2 | 39 | gaccgccgtacaggattatcatg | 183501 | - | see also 15527 line |
| 35339 | SERPINB2 | NM_002575.1 | 481 | ttccgggaagaatatattcgact | 183572 | - | see also 1656 line |
| 35340 | SERPINB2 | NM_002575.1 | 759 | cacacctgtacagatgatgtact | 183579 | - | see also 1656 line |
| 35341 | SERPINE1 | NM_000602.1 | 786 | cacgcccgatggccattactacg | 183595 | - | see also 15529 line |
| 35342 | SERPINE1 | NM_000602.1 | 363 | cgccctccggcatctgtacaagg | 183591 | - | see also 15529 line |
| 35343 | SERPINE1 | NM_000602.1 | 227 | aggaccgcaacgtggttttctca | 183588 | - | see also 15529 line |
| 35344 | CLPP | NM_006012.1 | 529 | ccccaactcccgtatcatgatcc | 183603 | - | see also 15530 line |
| 35345 | CLPP | NM_006012.1 | 528 | tccccaactcccgtatcatgatc | 183602 | - | see also 15530 line |
| 35346 | WDR11 | NM_018117.10 | 1179 | tacgtgttcgaagatcttataat | 183628 | - | see also 7260 line |
| 35347 | WDR11 | NM_018117.10 | 2618 | gtgacctttcgtatattggatgt | 183679 | - | see also 7260 line |
| 35348 | CLN2 | NM_000391.2 | 643 | tccgtaagcgatacaacttgacc | 183713 | - | see also 15532 line |
| 35349 | CLN2 | NM_000391.2 | 1475 | ggggatcctatccttgatcaatg | 183717 | - | see also 15532 line |
| 35350 | DPP7 | NM_013379.1 | 114 | gcgtctggaccacttcaacttcg | 183720 | - | see also 15533 line |
| 35351 | DPP7 | NM_013379.1 | 113 | agcgtctggaccacttcaacttc | 183719 | - | see also 15533 line |
| 35352 | FOLH1 | NM_004476.1 | 768 | gccagagggcgatctagtgtatg | 183734 | - | see also 3028 line |
| 35353 | FOLH1 | NM_004476.1 | 371 | tcctcttcgggtggtttataaaa | 183725 | - | see also 3028 line |
| 35354 | NAALAD2 | NM_005467.2 | 1616 | gagcccgttacactaagaataag | 183789 | - | see also 15535 line |
| 35355 | NAALAD2 | NM_005467.2 | 164 | ctgtgcgctatcatcaaagtata | 183743 | - | see also 15535 line |
| 35356 | NAALADL1 | NM_005468.1 | 1219 | ctggcgtcctcgcagatcaatcg | 183817 | - | see also 15536 line |

Figure 46

| | | | | | | | |
|---|---|---|---|---|---|---|---|
| 35357 | NAALADL1 | NM_005468.1 | 1919 | ttgggccaacgcatatcaacact | 183827 | - | see also 15536 line |
| 35358 | P11 | NM_006025.2 | 1137 | caggcaaagtgtgccagttaagc | 183846 | - | see also 4204 line |
| 35359 | P11 | NM_006025.2 | 1037 | ttcaactgggacggctactataa | 183843 | - | see also 4204 line |
| 35360 | PRSS16 | NM_005865.2 | 1146 | taccgagttcggcttctatgtca | 183858 | - | see also 15538 line |
| 35361 | PRSS16 | NM_005865.2 | 656 | tgctggatttctccgagtataat | 183853 | - | see also 15538 line |
| 35362 | RELN | NM_005045.2 | 358 | tactacgttccgggacaagaata | 183864 | - | see also 3523 line |
| 35363 | RELN | NM_005045.2 | 6236 | acgttggctgttcgactgatagc | 184032 | - | see also 3523 line |
| 35364 | RHBDL2 | NM_017821.2 | 955 | aggttttggatagcaattgctgc | 184161 | - | see also 15540 line |
| 35365 | RHBDL2 | NM_017821.2 | 236 | aagatcgggccaagagtaaaaag | 184145 | - | see also 15540 line |
| 35366 | ADAMTS2 | NM_014244.1 | 1059 | cacgggccacgatgaataccacg | 184165 | - | see also 5553 line |
| 35367 | ADAMTS2 | NM_014244.1 | 1595 | cccccttggacgggactatgtgt | 184166 | - | see also 5553 line |
| 35368 | ADAMTS2 | NM_014244.1 | 1597 | cccttggacgggactatgtgtgc | 184167 | - | see also 5553 line |
| 35369 | PMPCB | NM_004279.1 | 102 | ctgcgggacggtcattatatttt | 184187 | - | see also 15542 line |
| 35370 | PMPCB | NM_004279.1 | 103 | tgcgggacggtcattatattttg | 184188 | - | see also 15542 line |
| 35371 | PMPCB | NM_004279.1 | 627 | ctgcacttggacggacaattttg | 184205 | - | see also 15542 line |
| 35372 | OSGEP | NM_017807.1 | 546 | tgcatactcggaacatcgttacc | 184235 | - | see also 15543 line |
| 35373 | OSGEP | NM_017807.1 | 321 | tggattaacctcccaggatatcg | 184228 | - | see also 15543 line |
| 35374 | OSGEPL1 | NM_022353.1 | 1067 | atctggtggtgtcgcaagtaact | 184276 | - | see also 8343 line |
| 35375 | OSGEPL1 | NM_022353.1 | 1073 | tggtgtcgcaagtaacttctata | 184280 | - | see also 8343 line |
| 35376 | IDE | NM_004969.1 | 2724 | agcaattcgtcgactagacaaac | 184359 | - | see also 3451 line |
| 35377 | IDE | NM_004969.1 | 296 | atcccaccacggataagtcatca | 184298 | - | see also 3451 line |
| 35378 | IDE | NM_004969.1 | 1346 | cacggggctatacatctaagatt | 184328 | - | see also 3451 line |
| 35379 | NRD1 | NM_002525.1 | 2810 | cccgttggtctatgattgacaag | 184450 | - | see also 1621 line |
| 35380 | NRD1 | NM_002525.1 | 209 | cggcgctctggggaatcgaaacg | 184376 | - | see also 1621 line |
| 35381 | RCE1 | NM_005133.1 | 707 | cagcgtggggaacatcttcttgt | 184482 | - | see also 15547 line |
| 35382 | RCE1 | NM_005133.1 | 435 | ctgatgcagctctctatggattg | 184478 | - | see also 15547 line |
| 35383 | ADAM18 | NM_014237.1 | 2174 | atcgtaattcatccgttgtatca | 184560 | - | see also 15548 line |
| 35384 | ADAM18 | NM_014237.1 | 1690 | gacgctcaatctacagtttattc | 184544 | - | see also 15548 line |
| 35385 | ADAM32 | NM_145004.4 | 1858 | atgcataactgtagactacaaat | 184625 | - | see also 15549 line |
| 35386 | ADAM32 | NM_145004.4 | 1455 | tacaatcaggcgttgaatgtagg | 184614 | - | see also 15549 line |
| 35387 | ADAM7 | NM_003817.1 | 2124 | gtcggtatcggagttcttatact | 184709 | - | see also 15550 line |
| 35388 | ADAM7 | NM_003817.1 | 2126 | cggtatcggagttcttatactat | 184710 | - | see also 15550 line |
| 35389 | ADAMTS14 | NM_080722.2 | 2500 | ccccttatcgggagcaacaatgt | 184730 | - | see also 15551 line |

Figure 46

| 35390 | ADAMTS14 | NM_080722.2 | 2594 | gagggatccagttcaccaaatac | 184731 | - | see also 15551 line | | |
|---|---|---|---|---|---|---|---|---|---|
| 35391 | ADAMTS16 | NM_139056.1 | 594 | ctcgccatcccacgtactgtaca | 184739 | - | see also 9650 line | | |
| 35392 | ADAMTS18 | NM_139054.2 | 1656 | tgctctaagtaccgaagttgtac | 184825 | - | see also 9648 line | | |
| 35393 | ADAMTS18 | NM_139054.2 | 2735 | cgcccgaagcatcgaaatccagg | 184858 | - | see also 9648 line | | |
| 35394 | UQCRC1 | NM_003365.1 | 1494 | ctgtgaccgaatgaaaatcgatg | 184887 | - | see also 15553 line | | |
| 35395 | UQCRC1 | NM_003365.1 | 953 | tgcgagatgtggtctttaactac | 184876 | - | see also 15553 line | | |
| 35396 | UQCRC2 | NM_003366.1 | 426 | tgcctgcggggtgatgttgatat | 184898 | - | see also 2271 line | | |
| 35397 | UQCRC2 | NM_003366.1 | 609 | ttggctaatcccttgtattgtcc | 184909 | - | see also 2271 line | | |
| 35398 | ZMPSTE24 | NM_005857.2 | 1306 | agccaacccactcttattggact | 184949 | - | see also 4072 line | | |
| 35399 | ZMPSTE24 | NM_005857.2 | 1314 | cactcttattggactattgatca | 184951 | - | see also 4072 line | | |
| 35400 | BACE | NM_012104.2 | 1573 | cccaagacgactgttacaagttt | 184973 | - | see also 5134 line | | |
| 35401 | BACE2 | NM_012105.3 | 825 | ccccgcactcctacatagacacg | 184980 | - | see also 15557 line | | |
| 35402 | BACE2 | NM_012105.3 | 833 | tcctacatagacacgtactttga | 184981 | - | see also 15557 line | | |
| 35403 | CTSD | NM_001909.3 | 481 | ttgctggatccaccacaagtaca | 185004 | - | see also 15558 line | | |
| 35404 | CTSD | NM_001909.3 | 515 | tccagcacctacgtgaagaatgg | 185005 | - | see also 15558 line | | |
| 35405 | NAP1 | NM_004851.1 | 401 | ctggttacaccaccgatttgatc | 185014 | - | see also 15559 line | | |
| 35406 | NAP1 | NM_004851.1 | 402 | tggttacaccaccgatttgatcc | 185015 | - | see also 15559 line | | |
| 35407 | PGC | NM_002630.1 | 561 | ttcgtctatgcgcagtttgatgg | 185020 | - | see also 15560 line | | |
| 35408 | PGC | NM_002630.1 | 1046 | ttcctcctatatcctcagtaaca | 185025 | - | see also 15560 line | | |
| 35409 | PGC | NM_002630.1 | 795 | tactggcagattggcattgaaga | 185021 | - | see also 15560 line | | |
| 35410 | REN | NM_000537.2 | 1077 | ctcaccagcgcggactatgtatt | 185044 | - | see also 15561 line | | |
| 35411 | REN | NM_000537.2 | 1079 | caccagcgcggactatgtatttc | 185045 | - | see also 15561 line | | |
| 35412 | FLJ13687 | NM_024935.2 | 1592 | gggttgaccggcagccaatttct | 185091 | - | see also 15562 line | | |
| 35413 | FLJ13687 | NM_024935.2 | 1188 | caccacgacctagctatgcatca | 185078 | - | see also 15562 line | | |
| 35414 | FLJ25084 | NM_152792.1 | 874 | ttcgtcccggaaccttttgatgg | 185111 | - | see also 15563 line | | |
| 35415 | FLJ25084 | NM_152792.1 | 871 | gccttcgtcccggaaccttttga | 185110 | - | see also 15563 line | | |
| 35416 | FLJ25084 | NM_152792.1 | 870 | tgccttcgtcccggaaccttttg | 185109 | - | see also 15563 line | | |
| 35417 | NRIP2 | NM_031474.1 | 609 | gccgcctggggttagagaaaagg | 185117 | - | - | | |
| 35418 | MGC26605 | NM_144662.1 | 186 | tccaccgcggtcggaattcgagg | 185121 | - | see also 15564 line | | |
| 35419 | MGC26605 | NM_144662.1 | 131 | cccagacggacacctttttcaag | 185118 | - | see also 15564 line | | |

Figure 46

| | | | | | | | |
|---|---|---|---|---|---|---|---|
| 35420 | MGC26605 | NM_144662.1 | 347 | tgctgatgctagagtagtaataa | 185128 | - | see also 15564 line |
| 35421 | PSMA2 | NM_002787.2 | 56 | ttcagccgtctggtaaactgt | 185145 | - | see also 15565 line |
| 35422 | PSMA2 | NM_002787.2 | 434 | tggaatgagggacgaccatattt | 185159 | - | see also 15565 line |
| 35423 | PSMA3 | NM_002788.2 | 480 | acggtgcgcaactctacatgatt | 185173 | - | see also 15566 line |
| 35424 | PSMA3 | NM_002788.2 | 601 | gacctgccgtgatatcgttaaag | 185179 | - | see also 15566 line |
| 35425 | PSMA4 | NM_002789.3 | 183 | gtcgcttataccaagttgaatat | 185191 | - | see also 1815 line |
| 35426 | PSMA5 | NM_002790.2 | 107 | taagacagggcgtgaatacttt | 185211 | - | see also 15568 line |
| 35427 | PSMA5 | NM_002790.2 | 108 | acgacaggggcgtgaatacttt | 185212 | - | see also 15568 line |
| 35428 | PSMA5 | NM_002790.2 | 314 | gccatgagtgggctaattgctga | 185222 | - | see also 15568 line |
| 35429 | PSMA7 | NM_002792.2 | 346 | cgccgatgcaaggatagtcatca | 185235 | - | see also 1818 line |
| 35430 | PSMA7 | NM_002792.2 | 341 | ctcaccgccgatgcaaggatagt | 185234 | - | see also 1818 line |
| 35431 | PSMA7 | NM_002792.2 | 753 | gagatcaatcctcaagatttta | 185239 | - | see also 1818 line |
| 35432 | PSMB1 | NM_002793.2 | 130 | tgcgatttttcgccctacgtttc | 185241 | - | see also 15570 line |
| 35433 | PSMB1 | NM_002793.2 | 131 | gcgatttttcgccctacgttttca | 185242 | - | see also 15570 line |
| 35434 | PSMB10 | NM_002801.2 | 405 | atggagctacacgcgttatctac | 185267 | - | see also 15571 line |
| 35435 | PSMB10 | NM_002801.2 | 637 | accggttccagccgaacatgacg | 185270 | - | see also 15571 line |
| 35436 | PSMB2 | NM_002794.3 | 644 | cagtgttcgaatcattgacacaaa | 185284 | - | see also 15572 line |
| 35437 | PSMB2 | NM_002794.3 | 114 | gagtacctcatcggtatccaagg | 185273 | - | see also 15572 line |
| 35438 | PSMB3 | NM_002795.2 | 498 | caactgcgcgaacaaatgtacg | 185296 | - | see also 15573 line |
| 35439 | PSMB3 | NM_002795.2 | 314 | gtcggcagatcaaacttatacc | 185291 | - | see also 15573 line |
| 35440 | PSMB4 | NM_002796.2 | 317 | ctctggcgactacgctgatttcc | 185302 | - | see also 15574 line |
| 35441 | PSMB4 | NM_002796.2 | 572 | cactggttatggtgcatacttgg | 185308 | - | see also 15574 line |
| 35442 | PSMB4 | NM_002796.2 | 185 | ctcagtcctcggcggttaagttcg | 185299 | - | see also 15574 line |
| 35443 | PSMB5 | NM_002797.2 | 373 | ggctcggcaatgtcgaatctatg | 185319 | - | see also 15575 line |
| 35444 | PSMB5 | NM_002797.2 | 253 | ggctacagcgggtgcttacattg | 185318 | - | see also 15575 line |
| 35445 | PSMB6 | NM_002798.1 | 683 | gggtagagcggcaagtactttg | 185333 | proteasome endopeptidase | - |
| 35446 | PSMB6 | NM_002798.1 | 240 | acctattcacgacgcgcatttct | 185325 | - | see also 35445 line |
| 35447 | PSMB6 | NM_002798.1 | 522 | ctccggagctcctacatctatg | 185328 | - | see also 35445 line |
| 35448 | PSMB7 | NM_002799.2 | 155 | cgctggggtgtctataaggatg | 185336 | - | see also 15576 line |
| 35449 | PSMB7 | NM_002799.2 | 645 | ctgggctccgaagcaacattga | 185343 | - | see also 15576 line |
| 35450 | PSMB8 | NM_004159.3 | 875 | cacgggtagtgggaacacttatg | 185354 | - | see also 15577 line |
| 35451 | PSMB8 | NM_004159.3 | 687 | ctgtactatcgtcgcgaaatggaga | 185349 | - | see also 15577 line |
| 35452 | PSMB8 | NM_004159.3 | 960 | ggccgcagggctattgcttatgc | 185359 | - | see also 15577 line |
| 35453 | PSMB9 | NM_002800.3 | 496 | tccggcagcacctttatctatgg | 185367 | - | see also 15578 line |
| 35454 | PSMB9 | NM_002800.3 | 495 | ctccggcagcctttatctatg | 185366 | - | see also 15578 line |

Figure 46

| | | | | | | | |
|---|---|---|---|---|---|---|---|
| 35455 | PSMB9 | NM_002800.3 | 628 | atctacctggtcactattacagc | 185368 | - | see also 15578 line |
| 35456 | SENP1 | NM_014554.1 | 652 | cacctgtctcgatgtcttagttc | 185390 | - | see also 15579 line |
| 35457 | SENP1 | NM_014554.1 | 486 | ttcatgtcgtcgaagtcttttga | 185386 | - | see also 15579 line |
| 35458 | AGTPBP1 | NM_015239.1 | 2926 | tccggatttacatcctacaattt | 185509 | - | see also 6284 line |
| 35459 | AGTPBP1 | NM_015239.1 | 2689 | cagaaatcgcccttacgttttct | 185503 | - | see also 6284 line |
| 35460 | AGTPBP1 | NM_015239.1 | 1563 | aggccttggaccgaattacattg | 185461 | - | see also 6284 line |
| 35461 | CPA1 | NM_001868.1 | 343 | ctccaccgacacttttaactacg | 185528 | - | see also 15581 line |
| 35462 | CPA1 | NM_001868.1 | 221 | atccaggcggtcaagatctttct | 185527 | - | see also 15581 line |
| 35463 | CPA1 | NM_001868.1 | 1084 | agccagtggaagcactattgact | 185533 | - | see also 15581 line |
| 35464 | CPA2 | NM_001869.1 | 535 | tgctcgagagtggggttacacaag | 185545 | - | see also 15582 line |
| 35465 | CPA2 | NM_001869.1 | 466 | gcctatgaacgtgctcaagttca | 185544 | - | see also 15582 line |
| 35466 | CPA3 | NM_001870.1 | 184 | accacgtagctgctaatatgatg | 185563 | - | see also 1202 line |
| 35467 | CPA3 | NM_001870.1 | 855 | gagacgaaagctgtcactaattt | 185585 | - | see also 1202 line |
| 35468 | CPA4 | NM_016352.1 | 359 | ttcaactacgggggcttaccattc | 185621 | - | see also 6832 line |
| 35469 | CPA4 | NM_016352.1 | 118 | cagcaaattgagtcaactagtga | 185611 | - | see also 6832 line |
| 35470 | CPA5 | NM_080385.2 | 1794 | gaccgtgtcctgacagacatact | 185650 | - | see also 15585 line |
| 35471 | CPA5 | NM_080385.2 | 1928 | ctgcatcggcgtggatctcaaca | 185655 | - | see also 15585 line |
| 35472 | CPA6 | NM_020361.2 | 1293 | gtcagtatacggggtacgataca | 185700 | - | see also 15586 line |
| 35473 | CPA6 | NM_020361.2 | 1300 | tacggggtacgatacagatatgg | 185701 | - | see also 15586 line |
| 35474 | CPA6 | NM_020361.2 | 754 | ggcagacgatcacgactcaaaag | 185684 | - | see also 15586 line |
| 35475 | CPB1 | NM_001871.1 | 1044 | cacggcaccaagtacacatatgg | 185731 | - | see also 15587 line |
| 35476 | CPB1 | NM_001871.1 | 928 | tccactcgtactcccaaatgatg | 185726 | - | see also 15587 line |
| 35477 | CPB2 | NM_001872.2 | 366 | agcctccgcatcgtactatgaac | 185740 | - | see also 15588 line |
| 35478 | CPB2 | NM_001872.2 | 367 | gcctccgcatcgtactatgaaca | 185741 | - | see also 15588 line |
| 35479 | CPB2 | NM_001872.2 | 369 | ctccgcatcgtactatgaacagt | 185742 | - | see also 15588 line |
| 35480 | CPD | NM_001304.3 | 447 | gtcgcgccaggtgttgatctact | 185789 | - | see also 859 line |
| 35481 | CPD | NM_001304.3 | 982 | aacggcgcacattggtatgatgt | 185803 | - | see also 859 line |
| 35482 | CPD | NM_001304.3 | 3771 | agcgccaggtgtccataacatta | 185911 | - | see also 859 line |
| 35483 | CPE | NM_001873.1 | 1477 | aaggaatagaccacgatgttaca | 185957 | - | see also 1203 line |
| 35484 | CPE | NM_001873.1 | 875 | taggatagtgtacgtgaatgaga | 185935 | - | see also 1203 line |
| 35485 | CPN1 | NM_001308.1 | 875 | tggccaattacccgtatgacaag | 185978 | - | see also 15591 line |
| 35486 | CPN1 | NM_001308.1 | 1427 | ctgcggaaccaacgttggttaac | 185987 | - | see also 15591 line |
| 35487 | CPN1 | NM_001308.1 | 1428 | tgcggaaccaacgttggttaact | 185988 | - | see also 15591 line |
| 35488 | CPO | NM_173077.1 | 537 | gacggatctcaatcgaaatttca | 186013 | - | see also 15592 line |
| 35489 | CPO | NM_173077.1 | 533 | ttgggacggatctcaatcgaaat | 186010 | - | see also 15592 line |
| 35490 | CPXM | NM_019609.2 | 239 | tccggattcgagtcatcaagaag | 186029 | - | see also 15593 line |
| 35491 | CPXM | NM_019609.2 | 505 | ctggaggacggcgatctatatga | 186039 | - | see also 15593 line |

Figure 46

| | | | | | | | |
|---|---|---|---|---|---|---|---|
| 35492 | CPZ | NM_003652.1 | 730 | aagctcatcggcaacattcatgg | 186059 | - | see also 15594 line |
| 35493 | CPZ | NM_003652.1 | 1367 | gaggcatgtccgatttcaactac | 186066 | - | see also 15594 line |
| 35494 | DSCAML1 | NM_020693.2 | 4661 | cacgcatgctcggcttaacctgc | 186114 | - | see also 7916 line |
| 35495 | DSCAML1 | NM_020693.2 | 1705 | tccgggctatgcggaacatcaca | 186088 | - | see also 7916 line |
| 35496 | FLJ21347 | NM_022827.2 | 1188 | ggcttctatagcgcagaagatgc | 186132 | - | see also 15596 line |
| 35497 | FLJ21347 | NM_022827.2 | 1177 | accggtccgagggcttctatagc | 186131 | - | see also 15596 line |
| 35498 | FLJ23598 | NM_024783.2 | 1932 | aaggaacagagacgagtgttatg | 186188 | - | see also 15597 line |
| 35499 | FLJ23598 | NM_024783.2 | 1775 | ggccacagtcgtaagaataatat | 186178 | - | see also 15597 line |
| 35500 | FLJ32310 | NM_152336.1 | 596 | ttccacgatccctatcttatat | 186216 | - | see also 15598 line |
| 35501 | FLJ32310 | NM_152336.1 | 597 | tccacgatccctatcttatatg | 186217 | - | see also 15598 line |
| 35502 | FLJ32310 | NM_152336.1 | 475 | ctgggacgtagatgcaatttct | 186212 | - | see also 15598 line |
| 35503 | LOC119587 | NM_198148.1 | 1655 | cgctgaccacgatggaattga | 186244 | - | see also 15599 line |
| 35504 | LOC119587 | NM_198148.1 | 2240 | caggttcatcgtggcattaaagg | 186254 | - | see also 15599 line |
| 35505 | MGC20452 | NM_153232.2 | 708 | cagggtggacccgatatttaa | 186271 | - | see also 15600 line |
| 35506 | MGC20452 | NM_153232.2 | 598 | ccgattgctccggcagaataca | 186267 | - | see also 15600 line |
| 35507 | TBC1D1 | NM_015173.1 | 1160 | caggacagctatcgctttacaac | 186298 | - | see also 6266 line |
| 35508 | TBC1D1 | NM_015173.1 | 817 | aagcgcctgaagctcgattatga | 186287 | - | see also 6266 line |
| 35509 | PGCP | NM_016134.2 | 617 | ctggtggtgacctcttcgatga | 186327 | - | see also 15602 line |
| 35510 | PGCP | NM_016134.2 | 1076 | ggcggtggagcctttatatcatg | 186340 | - | see also 15602 line |
| 35511 | FLJ14675 | NM_032823.3 | 1573 | ctcgggagcatcgttatcaagc | 186391 | - | see also 15603 line |
| 35512 | FLJ14675 | NM_032823.3 | 2313 | aggtgtggccgaaatgttattt | 186403 | - | see also 15603 line |
| 35513 | RNPEPL1 | NM_018226.2 | 636 | gtggggcaggtacgaccattgct | 186406 | - | see also 7329 line |
| 35514 | RNPEPL1 | NM_018226.2 | 405 | tgccacccggagtgcatacatgg | 186404 | - | see also 7329 line |
| 35515 | TAF2 | NM_003184.2 | 3088 | atgagtccccttatgcaatga | 186505 | - | see also 15605 line |
| 35516 | TAF2 | NM_003184.2 | 1978 | gagttagatgatcctcaatca | 186465 | - | see also 15605 line |
| 35517 | METAP1 | NM_015143.1 | 632 | gtcgggccatgtatttacaattg | 186543 | - | see also 15606 line |
| 35518 | METAP1 | NM_015143.1 | 127 | atgttgctgccggcatgattaaa | 186527 | - | see also 15606 line |
| 35519 | METAP2 | NM_006838.2 | 1399 | agcgcaatttgaacataccatcc | 186581 | - | see also 15607 line |
| 35520 | METAP2 | NM_006838.2 | 636 | aagactgttcacgcaagttaata | 186554 | - | see also 15607 line |
| 35521 | PA2G4 | NM_006191.1 | 1238 | cccacagtgcggaaacattaga | 186583 | - | see also 15608 line |
| 35522 | DPEP1 | NM_004413.1 | 746 | ggcgtcctgcgggcactctatca | 186589 | - | see also 15609 line |
| 35523 | DPEP1 | NM_004413.1 | 374 | atgaggagactccctgtcattga | 186584 | - | see also 15609 line |
| 35524 | DPEP2 | NM_022355.1 | 881 | ctggggcatgatggtagagacttatc | 186594 | - | see also 15610 line |

Figure 46

| 35525 | DPEP2 | NM_022355.1 | 704 | gacaatagcctctccatcttacg | 186592 | - | see also 15610 line |
|---|---|---|---|---|---|---|---|
| 35526 | DPEP3 | NM_022357.1 | 395 | gagacagcgttacaagaatgtgc | 186598 | - | see also 15611 line |
| 35527 | DPEP3 | NM_022357.1 | 813 | gtcagcggattgacaagctttgg | 186599 | - | see also 15611 line |
| 35528 | DNPEP | NM_012100.1 | 559 | ggctggacgcgtcattgtcaagt | 186613 | - | see also 5131 line |
| 35529 | DNPEP | NM_012100.1 | 1027 | gcgcatggtcacactctatgaca | 186620 | - | see also 5131 line |
| 35530 | LOC63929 | NM_022098.2 | 1493 | tcggggtcttggtgtacgaattg | 186663 | - | see also 8270 line |
| 35531 | LOC63929 | NM_022098.2 | 635 | agcctatacgctagaagaatttc | 186643 | - | see also 8270 line |
| 35532 | XPNPEP1 | NM_020383.2 | 1640 | tccttgcggcatcagttacaaaa | 186694 | - | see also 7859 line |
| 35533 | XPNPEP1 | NM_020383.2 | 1366 | atgaggtgtaccttattgactcg | 186686 | - | see also 7859 line |
| 35534 | XPNPEP2 | NM_003399.3 | 1160 | gtgtgcaaatcgaggattacagc | 186721 | - | see also 15615 line |
| 35535 | XPNPEP2 | NM_003399.3 | 1703 | cccgtgtgctgataggaaatatt | 186727 | - | see also 15615 line |
| 35536 | ACY1 | NM_000666.1 | 1214 | ctccgtggggtggacatatatac | 186744 | - | see also 15616 line |
| 35537 | ACY1 | NM_000666.1 | 1215 | tccgtggggtggacatatataca | 186745 | - | see also 15616 line |
| 35538 | CN1 | NM_032649.4 | 1131 | gtcatacctggccgagttatagg | 186765 | - | see also 15617 line |
| 35539 | CN1 | NM_032649.4 | 1206 | caggtgacacgacatcttgaaga | 186773 | - | see also 15617 line |
| 35540 | CN2 | NM_018235.1 | 382 | gagatcccgctccctcctattct | 186793 | - | see also 15618 line |
| 35541 | CN2 | NM_018235.1 | 1262 | aactacgcagccccaatgagttc | 186807 | - | see also 15618 line |
| 35542 | FLJ32569 | NM_152491.1 | 1009 | cacggcactcaccatattcaaag | 186838 | - | see also 15619 line |
| 35543 | FLJ32569 | NM_152491.1 | 978 | atcccttaaccaatgcaataatc | 186837 | - | see also 15619 line |
| 35544 | PITRM1 | NM_014889.1 | 1229 | agcctcatagacagaacgattga | 186869 | - | see also 15620 line |
| 35545 | PITRM1 | NM_014889.1 | 2593 | gcccttcccggtgaattacgtgg | 186894 | - | see also 15620 line |
| 35546 | PITRM1 | NM_014889.1 | 2867 | gacatcgacgaagccaaactttc | 186899 | - | see also 15620 line |
| 35547 | TIMP1 | NM_003254.1 | 364 | accgcagcgaggagtttctcatt | 186907 | - | see also 15621 line |
| 35548 | TIMP1 | NM_003254.1 | 238 | agcgttatgagatcaagatgacc | 186906 | - | see also 15621 line |
| 35549 | DPP10 | NM_020868.1 | 2297 | gagattcatcgaagattaggttc | 186967 | - | see also 8010 line |
| 35550 | DPP10 | NM_020868.1 | 1972 | tacggacaacccagcaaaatatt | 186953 | - | see also 8010 line |
| 35551 | DPP6 | NM_001936.2 | 630 | ctcgtatactggatattacgtcc | 187003 | - | see also 15623 line |
| 35552 | DPP6 | NM_001936.2 | 427 | cccgaggctaagtggataagtga | 186992 | - | see also 15623 line |
| 35553 | DPP8 | NM_017743.3 | 1839 | tacgtagtcagttacgtaaatcc | 187095 | - | see also 15624 line |
| 35554 | DPP8 | NM_017743.3 | 1836 | ctgtacgtagtcagttacgtaaa | 187094 | - | see also 15624 line |
| 35555 | DPP9 | NM_139159.2 | 2749 | atgagcccaaccgcttgcttatc | 187171 | - | see also 9662 line |
| 35556 | DPP9 | NM_139159.2 | 2903 | gtcgggcgagcactatgaagtca | 187177 | - | see also 9662 line |
| 35557 | PRCP | NM_005040.2 | 1530 | gcccgctccttggaagttagaca | 187222 | - | see also 15626 line |
| 35558 | PRCP | NM_005040.2 | 1229 | tactaatggtgtcgatgacatgt | 187213 | - | see also 15626 line |
| 35559 | PPGB | NM_000308.1 | 412 | tactccgatgacaagttttatgc | 187232 | - | see also 15627 line |
| 35560 | PPGB | NM_000308.1 | 262 | cccggctgcagctcactagatgg | 187227 | - | see also 15627 line |
| 35561 | PPGB | NM_000308.1 | 857 | gggtgcccagccattttaggtat | 187242 | - | see also 15627 line |

Figure 46

| 35562 | CPVL | NM_019029.1 | 1045 | tactagatggcgacttaacaagt | 187268 | - | see also 15628 line | | |
|---|---|---|---|---|---|---|---|---|---|
| 35563 | CPVL | NM_019029.1 | 401 | caccgtgaataagacttacaaca | 187251 | - | see also 15628 line | | |
| 35564 | CPVL | NM_019029.1 | 684 | gacgatgtagcacgggatttata | 187258 | - | see also 15628 line | | |
| 35565 | RISC | NM_021626.1 | 1113 | atcaacgtgacggtgtataatgg | 187306 | - | see also 8152 line | | |
| 35566 | RISC | NM_021626.1 | 1116 | aacgtgacggtgtataatggaca | 187307 | - | see also 8152 line | | |
| 35567 | FLJ25059 | NM_144981.1 | 225 | ttcgacttgttggctatactatt | 187313 | - | - | - | - |
| 35568 | FLJ25059 | NM_144981.1 | 478 | caccactagtccatcagatttct | 187319 | - | see also 35567 line | | |
| 35569 | FLJ25059 | NM_144981.1 | 313 | atcaatggagcctacaattcaaa | 187315 | - | see also 35567 line | | |
| 35570 | CASP10 | NM_001230.2 | 1180 | ttcccattcgggagatcatgtct | 187355 | - | see also 15630 line | | |
| 35571 | CASP10 | NM_001230.2 | 325 | taggattggtccccaacaagaag | 187340 | - | see also 15630 line | | |
| 35572 | CASP14 | NM_012114.1 | 52 | agcaatccgcggtctttggaaga | 187366 | - | see also 15631 line | | |
| 35573 | CASP14 | NM_012114.1 | 555 | ttccacggtagagggatacatcg | 187375 | - | see also 15631 line | | |
| 35574 | CASP14 | NM_012114.1 | 534 | cacagatgccttgcacgtttatt | 187373 | - | see also 15631 line | | |
| 35575 | CASP3 | NM_004346.2 | 668 | ggggatcgttgtagaagtctaac | 187380 | - | see also 15632 line | | |
| 35576 | CASP3 | NM_004346.2 | 912 | atgccgacaagcttgaatttatg | 187392 | - | see also 15632 line | | |
| 35577 | CASP4 | NM_001225.2 | 86 | atccctgggcaaagatttcctca | 187400 | - | see also 15633 line | | |
| 35578 | CASP4 | NM_001225.2 | 384 | ctgagactatgtaaagaaagagc | 187405 | - | see also 15633 line | | |
| 35579 | CASP5 | NM_004347.1 | 805 | ctgccggaactgcgcataaaaaga | 187427 | - | see also 15634 line | | |
| 35580 | CASP5 | NM_004347.1 | 144 | atcaaaagtcgaccagtgtaaaa | 187414 | - | see also 15634 line | | |
| 35581 | CASP6 | NM_001226.2 | 851 | agcgccgagtggactttttgcaaa | 187461 | - | see also 806 line | | |
| 35582 | CASP6 | NM_001226.2 | 400 | cacgcagatgccgattgctttgt | 187443 | - | see also 806 line | | |
| 35583 | CASP7 | NM_001227.2 | 466 | cgggaccgagtgcctacatatca | 187470 | - | see also 15636 line | | |
| 35584 | CASP7 | NM_001227.2 | 404 | ttgtaccgtccctcttcagtaag | 187469 | - | see also 15636 line | | |
| 35585 | CASP8 | NM_001228.2 | 1003 | tcggggatactgtctgatcatca | 187515 | - | see also 807 line | | |
| 35586 | CASP9 | NM_001229.2 | 1245 | ctgcttagggtcgctaatgctgt | 187545 | - | see also 15638 line | | |
| 35587 | CASP9 | NM_001229.2 | 420 | gtggtgctcagaccagagattcg | 187537 | - | see also 15638 line | | |
| 35588 | CFLAR | NM_003879.3 | 741 | ctcaccttgtttcggactataga | 187553 | - | see also 15639 line | | |
| 35589 | CFLAR | NM_003879.3 | 743 | caccttgtttcggactatagagt | 187554 | - | see also 15639 line | | |
| 35590 | CAPN10 | NM_021251.2 | 251 | cgctcttctgcgacttgtctacg | 187576 | - | - | - | diabetes mellitus、diabetes |
| 35591 | CAPN13 | NM_144575.1 | 1455 | tccactcgatttccaagtgattc | 187614 | - | see also 15640 line | | |
| 35592 | CAPN13 | NM_144575.1 | 1139 | cgcggaaaagccagctacataag | 187600 | - | see also 15640 line | | |
| 35593 | CAPN13 | NM_144575.1 | 763 | gccaagctgctcggatcctattc | 187596 | - | see also 15640 line | | |
| 35594 | CAPN5 | NM_004055.3 | 1995 | tccacctccgggaccgaaatagc | 187632 | - | see also 2732 line | | |
| 35595 | CAPN5 | NM_004055.3 | 803 | cagctctttgagcgcatgttaaa | 187621 | - | see also 2732 line | | |
| 35596 | CAPN6 | NM_014289.2 | 1702 | ggctacccgaaagtagttactca | 187663 | - | see also 5594 line | | |
| 35597 | CAPN6 | NM_014289.2 | 1698 | tcgtggctacccgaaagtagtta | 187661 | - | see also 5594 line | | |
| 35598 | CAPN7 | NM_014296.1 | 327 | tactccgaggcggtgttttatta | 187677 | - | see also 5603 line | | |

Figure 46

| 35599 | CAPN7 | NM_014296.1 | 2250 | tacacggttcgggtatattcagc | 187742 | - | see also 5603 line |
|---|---|---|---|---|---|---|---|
| 35600 | CTSL | NM_001912.2 | 443 | ggcgatgcacaacagattatacg | 187764 | - | see also 15644 line |
| 35601 | CTSL | NM_001912.2 | 906 | cagtatgttcaggataatggagg | 187767 | - | see also 15644 line |
| 35602 | CTSL2 | NM_001333.2 | 1118 | gggctcgaatggctatgtaaaaa | 187782 | - | see also 15645 line |
| 35603 | CTSL2 | NM_001333.2 | 1117 | ggggctcgaatggctatgtaaaa | 187781 | - | see also 15645 line |
| 35604 | LGMN | NM_005606.3 | 783 | gtcgtcctacgcctgttactatg | 187792 | - | see also 15646 line |
| 35605 | LGMN | NM_005606.3 | 787 | tcctacgcctgttactatgatga | 187795 | - | see also 15646 line |
| 35606 | PIGK | NM_005482.1 | 685 | atggctctagctagtagtcaagt | 187830 | - | see also 3813 line |
| 35607 | PIGK | NM_005482.1 | 455 | ctcggtcaaaacgtcttctttct | 187819 | - | see also 3813 line |
| 35608 | CTSS | NM_004079.3 | 457 | gtcaaaccctaatcggatattgc | 187857 | - | see also 2749 line |
| 35609 | CTSS | NM_004079.3 | 919 | cagaagtggtgtctactatgaac | 187873 | - | see also 2749 line |
| 35610 | CTSK | NM_000396.2 | 413 | ctgtctcattcccgcagtaatga | 187882 | - | see also 317 line |
| 35611 | CTSK | NM_000396.2 | 415 | gtctcattcccgcagtaatgaca | 187883 | - | see also 317 line |
| 35612 | CTSH | NM_004390.2 | 557 | cgccatcgcaaccggaaagatgc | 187903 | - | see also 15650 line |
| 35613 | CTSH | NM_004390.2 | 1039 | tcgagcgcggaaagaacatgtgt | 187916 | - | see also 15650 line |
| 35614 | CTSB | NM_001908.2 | 815 | gacaagcactacggatacaattc | 187921 | - | see also 15651 line |
| 35615 | CTSB | NM_001908.2 | 762 | cccccaagtgtagcaagatctgt | 187920 | - | see also 15651 line |
| 35616 | BLMH | NM_000386.2 | 887 | tccgagtggtgtgcatctgtttg | 187950 | - | see also 306 line |
| 35617 | BLMH | NM_000386.2 | 1436 | aagaccatggccacaaaggttac | 187963 | - | see also 306 line |
| 35618 | AQP11 | NM_173039.1 | 1017 | cactttcgctacatttcatgtgt | 187970 | - | see also 15653 line |
| 35619 | AQP11 | NM_173039.1 | 1140 | tgcataacaaccatacaattaat | 187976 | - | see also 15653 line |
| 35620 | ARHGEF10 | NM_014629.1 | 6183 | ggcgtcctaccagttagaagtct | 188038 | - | see also 15654 line |
| 35621 | ARHGEF10 | NM_014629.1 | 4972 | aagaacgccgagtcttcatgtta | 188010 | - | see also 15654 line |
| 35622 | CT120 | NM_024792.1 | 385 | gtcgaaaccgcctcatgatcaca | 188050 | - | see also 15655 line |
| 35623 | CT120 | NM_024792.1 | 198 | ggctcggggatcgtcatcattcg | 188046 | - | see also 15655 line |
| 35624 | CTSC | NM_001814.2 | 179 | tgccaactgcacctatcttgacc | 188059 | - | see also 15656 line |
| 35625 | CTSC | NM_001814.2 | 306 | aagctggatacagcatatgatga | 188062 | - | see also 15656 line |
| 35626 | CTSF | NM_003793.2 | 666 | tacctataaccggacatatgagt | 188104 | - | see also 15657 line |
| 35627 | CTSF | NM_003793.2 | 710 | gcctgtccgtctttgtcaataac | 188109 | - | see also 15657 line |
| 35628 | CTSO | NM_001334.2 | 187 | aagtcttaatagacatcgatact | 188126 | - | see also 15658 line |
| 35629 | CTSO | NM_001334.2 | 531 | ttgactgttcgtataataattat | 188139 | - | see also 15658 line |
| 35630 | CTSW | NM_001335.2 | 626 | tcgtctgggacgcgttcataact | 188168 | - | see also 15659 line |
| 35631 | CTSW | NM_001335.2 | 625 | ttcgtctgggacgcgttcataac | 188167 | - | see also 15659 line |
| 35632 | CTSZ | NM_001336.2 | 795 | aactacaccggaggcatctatgc | 188178 | - | see also 15660 line |
| 35633 | CTSZ | NM_001336.2 | 999 | atcgaggagcactgtacatttgg | 188185 | - | see also 15660 line |

Figure 46

| 35634 | CYLD | NM_015247.1 | 2766 | gagggcttgcaatgtatgagtgt | 188256 | - | see also 6290 line |
|---|---|---|---|---|---|---|---|
| 35635 | CYLD | NM_015247.1 | 856 | gaggacagtctccggaatattct | 188202 | - | see also 6290 line |
| 35636 | D13S106E | NM_005800.3 | 781 | gacgaaacctcgtcaaacttacc | 188283 | - | see also 15662 line |
| 35637 | D13S106E | NM_005800.3 | 2435 | aggacgctcagttaaaacaattc | 188336 | - | see also 15662 line |
| 35638 | D13S106E | NM_005800.3 | 1897 | gaccaacacgctctcagtaatga | 188317 | - | see also 15662 line |
| 35639 | FLJ20558 | NM_017880.1 | 1777 | aacctacggtcgtatagaaaaac | 188413 | - | see also 7168 line |
| 35640 | FLJ20558 | NM_017880.1 | 1931 | ctgcggatcaagtttgagtatgg | 188424 | - | see also 7168 line |
| 35641 | GPR114 | NM_153837.1 | 909 | tgcggctcatctgtatctacttc | 188427 | - | see also 15664 line |
| 35642 | GPR114 | NM_153837.1 | 903 | gggagctgcggctcatctgtatc | 188426 | - | see also 15664 line |
| 35643 | GRP58 | NM_005313.3 | 633 | taccgatttgcacatacgaatgt | 188450 | - | see also 15665 line |
| 35644 | GRP58 | NM_005313.3 | 559 | taggtttttcgatgattcattc | 188445 | - | see also 15665 line |
| 35645 | GRP58 | NM_005313.3 | 991 | tagctagccgcaaaacctttagc | 188468 | - | see also 15665 line |
| 35646 | KIAA1203 | NM_020718.1 | 303 | agccgcgacttcaagactattgt | 188479 | - | see also 15666 line |
| 35647 | KIAA1203 | NM_020718.1 | 1940 | ctgcacgcagacagcatacatcc | 188517 | - | see also 15666 line |
| 35648 | LCN7 | NM_022164.1 | 767 | tccgatcgtgtctcaatccattc | 188535 | - | see also 15667 line |
| 35649 | LCN7 | NM_022164.1 | 686 | aagtggcccaacctgattcatga | 188533 | - | see also 15667 line |
| 35650 | LOC83693 | NM_031463.3 | 476 | gacatagccgacacgtacaaagt | 188553 | - | see also 15668 line |
| 35651 | LOC83693 | NM_031463.3 | 236 | gcctggtatacggccagaaaaag | 188544 | - | see also 15668 line |
| 35652 | MGC20741 | NM_018561.3 | 1868 | tccggctgcaccttaaaagattc | 188596 | - | see also 7525 line |
| 35653 | MGC20741 | NM_018561.3 | 1783 | agcaaacgacgaaaatccaatcc | 188591 | - | see also 7525 line |
| 35654 | MGC26717 | NM_173824.2 | 943 | ctggaacattaccgaaaccatct | 188622 | - | see also 15670 line |
| 35655 | MGC26717 | NM_173824.2 | 422 | aacgccagagccagttacaaaga | 188612 | - | see also 15670 line |
| 35656 | PARK2 | NM_004562.1 | 864 | tcccgccacgtgatttgcttaga | 188645 | - | see also 15671 line |
| 35657 | PARK2 | NM_004562.1 | 778 | tgcacctgatcgcaacaaatagt | 188641 | - | see also 15671 line |
| 35658 | PARK2 | NM_004562.1 | 868 | gccacgtgatttgcttagactgt | 188646 | - | see also 15671 line |
| 35659 | USP1 | NM_003368.3 | 1773 | gacgcgttgcttggaatgtgaaa | 188688 | - | see also 15672 line |
| 35660 | USP1 | NM_003368.3 | 2175 | aactaacgacagctatggdattat | 188705 | - | see also 15672 line |
| 35661 | USP10 | NM_005153.1 | 1481 | tacgtcaacacccatgatagaca | 188772 | - | see also 3615 line |
| 35662 | USP11 | NM_004651.2 | 643 | ttcagccataccgattctattgg | 188785 | - | see also 3165 line |
| 35663 | USP11 | NM_004651.2 | 653 | ccgattctattggcctagtattg | 188786 | - | see also 3165 line |
| 35664 | USP12 | NM_182488.1 | 708 | tggtcgtttacctaatggtaata | 188842 | - | see also 10263 line |
| 35665 | USP12 | NM_182488.1 | 353 | ttccggtcaatgagcactatttt | 188828 | - | see also 10263 line |
| 35666 | USP14 | NM_005151.2 | 150 | ccgctctactccgttactgtaaa | 188868 | - | see also 15676 line |

Figure 46

| | | | | | | | |
|---|---|---|---|---|---|---|---|
| 35667 | USP14 | NM_005151.2 | 582 | atggcttcagcgcagtatattac | 188885 | - | see also 15676 line |
| 35668 | USP15 | NM_006313.1 | 2534 | gtccttgccgctataatctgatt | 189029 | - | see also 4416 line |
| 35669 | USP15 | NM_006313.1 | 106 | gtcgatagtcgctggttcaaaca | 188922 | - | see also 4416 line |
| 35670 | USP16 | NM_006447.1 | 492 | gaggtccagtattgtagttcaaa | 189049 | - | see also 4515 line |
| 35671 | USP16 | NM_006447.1 | 1204 | ttggtggtgaactaactagtatg | 189069 | - | see also 4515 line |
| 35672 | USP18 | NM_017414.2 | 1409 | ctggcaggaaactgcatatcttc | 189115 | - | see also 15679 line |
| 35673 | USP18 | NM_017414.2 | 1410 | tggcaggaaactgcatatcttct | 189116 | - | see also 15679 line |
| 35674 | USP2 | NM_004205.3 | 1265 | accgcgcttgttggctataatc | 189130 | - | see also 2815 line |
| 35675 | USP2 | NM_004205.3 | 1554 | cccattgctaagcgaggttatcc | 189138 | - | see also 2815 line |
| 35676 | USP20 | NM_006676.2 | 1565 | aacgttccaggacttatcactgc | 189161 | - | see also 15681 line |
| 35677 | USP20 | NM_006676.2 | 689 | cggcctgctgcgcacagataaga | 189152 | - | see also 15681 line |
| 35678 | USP21 | NM_012475.2 | 1139 | atgatgaccgagccaacctaatg | 189178 | - | see also 5349 line |
| 35679 | USP25 | NM_013396.2 | 2862 | aaccgattatcgttcacatcatg | 189272 | - | see also 5429 line |
| 35680 | USP25 | NM_013396.2 | 2863 | accgattatcgtttacatcatgt | 189273 | - | see also 5429 line |
| 35681 | USP26 | NM_031907.1 | 682 | ttcgaggtgttgaagctataatc | 189315 | - | see also 15684 line |
| 35682 | USP26 | NM_031907.1 | 549 | cacttacttgcggagagttatca | 189309 | - | see also 15684 line |
| 35683 | USP29 | NM_020903.1 | 2121 | aaccaatacattcgtagagttca | 189423 | - | see also 15685 line |
| 35684 | USP29 | NM_020903.1 | 2602 | aacgatctatgtgtatcagaaat | 189434 | - | see also 15685 line |
| 35685 | USP29 | NM_020903.1 | 2593 | ttcacatacaagatctatgtgt | 189432 | - | see also 15685 line |
| 35686 | USP3 | NM_006537.1 | 1595 | aggcgaaggcctacatcctttc | 189475 | - | see also 4570 line |
| 35687 | USP3 | NM_006537.1 | 181 | agcgtgtgccggtccaacaaaag | 189438 | - | see also 4570 line |
| 35688 | USP30 | NM_032663.2 | 777 | aagactcactagtaatatggtct | 189497 | - | see also 15687 line |
| 35689 | USP30 | NM_032663.2 | 827 | ttcgatttgatacctttgatagc | 189499 | - | see also 15687 line |
| 35690 | USP31 | NM_032236.3 | 2007 | atgaacggtagcaccttaaataa | 189580 | - | see also 15688 line |
| 35691 | USP31 | NM_032236.3 | 1130 | aggtgggtcctacgtgtatgaac | 189538 | - | see also 15688 line |
| 35692 | USP33 | NM_015017.2 | 821 | gactagctcgaacagataagaaa | 189627 | - | see also 6186 line |
| 35693 | USP33 | NM_015017.2 | 1758 | aggatcatgtggcgaagcatatg | 189655 | - | see also 6186 line |
| 35694 | USP36 | NM_025090.2 | 1273 | gtccgtatatgtcccagaataat | 189716 | - | see also 15690 line |
| 35695 | USP36 | NM_025090.2 | 1274 | tccgtatatgtcccagaataatg | 189717 | - | see also 15690 line |
| 35696 | USP37 | NM_020935.1 | 581 | aacaaagccgcctaatgttaact | 189739 | - | see also 8038 line |
| 35697 | USP37 | NM_020935.1 | 872 | atccgggtagagggatcgattaag | 189745 | - | see also 8038 line |
| 35698 | USP37 | NM_020935.1 | 962 | cagggttgctagaaaatcgtact | 189750 | - | see also 8038 line |
| 35699 | USP38 | NM_032557.4 | 2009 | agcatacgcacctcggatattct | 189835 | - | see also 9246 line |
| 35700 | USP38 | NM_032557.4 | 2284 | aactacgaactcacatacgttgt | 189841 | - | see also 9246 line |
| 35701 | USP4 | NM_003363.2 | 350 | accgagccgtggaataaactact | 189881 | - | see also 2268 line |
| 35702 | USP4 | NM_003363.2 | 231 | atgtgggtgaacataacctattt | 189877 | - | see also 2268 line |
| 35703 | USP44 | NM_032147.1 | 905 | ctcgctcagtcgaccataataga | 189960 | - | see also 15693 line |

Figure 46

| 35704 | USP44 | NM_032147.1 | 2354 | gacgctgatacctcgtctaatga | 190007 | - | see also 15693 line |
|---|---|---|---|---|---|---|---|
| 35705 | USP46 | NM_022832.2 | 1063 | atcgtgggcattatatcactatt | 190040 | - | see also 8487 line |
| 35706 | USP46 | NM_022832.2 | 594 | atgaacgaacctgcggaaaataa | 190024 | - | see also 8487 line |
| 35707 | USP47 | NM_017944.2 | 855 | gggctggcgaatcgacacatatc | 190061 | - | see also 7180 line |
| 35708 | USP47 | NM_017944.2 | 859 | tggcgaatcgacacatatcttga | 190064 | - | see also 7180 line |
| 35709 | USP5 | NM_003481.1 | 329 | cccacgcggctggctattggtgt | 190182 | - | see also 15696 line |
| 35710 | USP5 | NM_003481.1 | 808 | agctgacgtgtactcatatgatg | 190189 | - | see also 15696 line |
| 35711 | USP52 | NM_014871.1 | 1820 | gcctgtacgctgtctaattcaaa | 190231 | - | see also 6067 line |
| 35712 | USP52 | NM_014871.1 | 863 | gagagacctccgtacttttaagg | 190211 | - | see also 6067 line |
| 35713 | USP7 | NM_003470.1 | 1096 | ttgtcgagtgttgctcgataatg | 190309 | - | see also 15698 line |
| 35714 | USP7 | NM_003470.1 | 3095 | acgagccggacgtttcgaataga | 190372 | - | see also 15698 line |
| 35715 | USP7 | NM_003470.1 | 3094 | aacgagccggacgtttcgaatag | 190371 | - | see also 15698 line |
| 35716 | USP8 | NM_005154.1 | 3283 | gagctcgacgggattctctaaaa | 190459 | - | see also 3617 line |
| 35717 | USP8 | NM_005154.1 | 3284 | agctcgacgggattctctaaaaa | 190460 | - | see also 3617 line |
| 35718 | USP9X | NM_004652.2 | 3791 | tgggtcgttacagctagtattta | 190528 | - | see also 3166 line |
| 35719 | USP9X | NM_004652.2 | 2779 | cagtacgacgatgtattctcaat | 190510 | - | see also 3166 line |
| 35720 | USP9Y | NM_004654.2 | 6329 | ttgctatccaactcaaacgattt | 190646 | - | see also 15701 line |
| 35721 | USP9Y | NM_004654.2 | 3694 | cggcgatgtattgttaatcgtat | 190611 | - | see also 15701 line |
| 35722 | BAP1 | NM_004656.2 | 212 | tacgaccttcagagcaaatgtca | 190669 | - | see also 15702 line |
| 35723 | BAP1 | NM_004656.2 | 817 | gcccgaccgcaggatcaagtatg | 190676 | - | see also 15702 line |
| 35724 | BAP1 | NM_004656.2 | 319 | tacgtccgtgattgatgatgata | 190671 | - | see also 15702 line |
| 35725 | UCHL1 | NM_004181.3 | 332 | ctgtggcacaatcggacttattc | 190694 | - | see also 15703 line |
| 35726 | UCHL1 | NM_004181.3 | 354 | cacgcagtggccaataatcaaga | 190696 | - | see also 15703 line |
| 35727 | UCHL3 | NM_006002.3 | 671 | agcgcgaccctgatgaactaaga | 190716 | - | see also 4186 line |
| 35728 | UCHL3 | NM_006002.3 | 667 | atggagcgcgaccctgatgaact | 190714 | - | see also 4186 line |
| 35729 | UCHL5 | NM_015984.1 | 344 | ctcccgacttgacacgatatttt | 190724 | - | see also 6595 line |
| 35730 | UCHL5 | NM_015984.1 | 346 | cccgacttgacacgatatttttt | 190726 | - | see also 6595 line |
| 35731 | UCHL5 | NM_015984.1 | 444 | gtccatttaggcgagacattatc | 190731 | - | see also 6595 line |
| 35732 | UFD1L | NM_005659.3 | 238 | accaactcagccgacttaacatt | 190757 | - | see also 3929 line |
| 35733 | UFD1L | NM_005659.3 | 483 | accaaccccaaagccgtattaga | 190766 | - | see also 3929 line |
| 35734 | PGPEP1 | NM_017712.1 | 100 | accgtgaacgccagttggattgc | 190781 | - | see also 15707 line |
| 35735 | PGPEP1 | NM_017712.1 | 183 | tccggttgagtaccaaacagtcc | 190784 | - | see also 15707 line |
| 35736 | MGC27076 | NM_152699.2 | 1331 | cagaacatcgttctaataccatg | 190819 | - | see also 9927 line |
| 35737 | PRSC2 | NM_145204.2 | 588 | tgggatgtacgtgatatgtaaca | 190862 | - | see also 15709 line |
| 35738 | PRSC2 | NM_145204.2 | 104 | accccgtagtcttgagttacatg | 190843 | - | see also 15709 line |
| 35739 | SENP3 | NM_015670.2 | 1491 | ggcgacgcaccatcacctatttt | 190879 | - | see also 15710 line |

Figure 46

| | | | | | | | |
|---|---|---|---|---|---|---|---|
| 35740 | SENP3 | NM_015670.2 | 1535 | cgccgctgccctaagcatattgc | 190882 | - | see also 15710 line |
| 35741 | SENP7 | NM_020654.2 | 1332 | gaccgtgagacaactaatgaaaa | 190929 | - | see also 15711 line |
| 35742 | SENP7 | NM_020654.2 | 2686 | cccaaagtaccgagtcgaatatg | 190966 | - | see also 15711 line |
| 35743 | SENP7 | NM_020654.2 | 2994 | aagtggtttcctcgtcatgtaat | 190979 | - | see also 15711 line |
| 35744 | SUSP1 | NM_015571.1 | 2230 | tggacccggcacgtagatatttt | 191058 | - | see also 15712 line |
| 35745 | SUSP1 | NM_015571.1 | 2229 | atggacccggcacgtagatattt | 191057 | - | see also 15712 line |
| 35746 | TINAG | NM_014464.1 | 4 | gtggaccggatataagatcttaa | 191084 | - | see also 5696 line |
| 35747 | TINAG | NM_014464.1 | 836 | aagaaccgtcatggatgcaatag | 191112 | - | see also 5696 line |
| 35748 | ABHD5 | NM_016006.3 | 265 | aagaacctgttcgtatatctaat | 191132 | - | see also 15714 line |
| 35749 | ABHD5 | NM_016006.3 | 1038 | tccttacgaccacattcatatgt | 191156 | - | see also 15714 line |
| 35750 | GGH | NM_003878.1 | 185 | atgccgtaataaagtcatgaaaa | 191160 | - | see also 15715 line |
| 35751 | GGH | NM_003878.1 | 230 | tgcgtcctatgtaaagtacttgg | 191165 | - | see also 15715 line |
| 35752 | LOC51185 | NM_016302.2 | 290 | ctgattcccggacagacattacc | 191185 | - | see also 6798 line |
| 35753 | LOC51185 | NM_016302.2 | 741 | ctcgctggctgtattccttatat | 191194 | - | see also 6798 line |
| 35754 | RNF127 | NM_024778.3 | 418 | agccctcgaggtgtatagacagc | 191220 | - | see also 15717 line |
| 35755 | RNF127 | NM_024778.3 | 1003 | tagcaatcggtctcagatttatt | 191227 | - | see also 15717 line |
| 35756 | RNF127 | NM_024778.3 | 1439 | tgcgctctatgtatgagattatt | 191237 | - | see also 15717 line |
| 35757 | C14orf137 | NM_023112.2 | 802 | ttccgtttacctgctctataaaa | 191264 | - | see also 15718 line |
| 35758 | C14orf137 | NM_023112.2 | 839 | atcctttatgcagccgataaaca | 191266 | - | see also 15718 line |
| 35759 | C14orf137 | NM_023112.2 | 803 | tccgtttacctgctctataaaac | 191265 | - | see also 15718 line |
| 35760 | C2orf7 | NM_032319.1 | 135 | ggcttccgtatccatgattattt | 191270 | - | see also 15719 line |
| 35761 | C2orf7 | NM_032319.1 | 178 | ctggggacattcgatacatcttc | 191273 | - | see also 15719 line |
| 35762 | C9orf52 | NM_152574.1 | 1791 | acctagtgccgttcactctattt | 191313 | - | see also 15720 line |
| 35763 | C9orf52 | NM_152574.1 | 874 | aaggattatcagactactagaat | 191292 | - | see also 15720 line |
| 35764 | CGI-14 | NM_015944.2 | 369 | ttccccaccggaggtttatcaca | 191322 | - | see also 6586 line |
| 35765 | CGI-14 | NM_015944.2 | 803 | ccgcaggccgctgcatcttctat | 191324 | - | see also 6586 line |
| 35766 | DHH | NM_021044.2 | 738 | gccgtgctttggacatcactacg | 191327 | - | see also 8076 line |
| 35767 | DKFZP566H073 | NM_015528.1 | 770 | tgccgctgtagtacacaatgtga | 191336 | | see also 6451 line |
| 35768 | DKFZP566H073 | NM_015528.1 | 1059 | cagcggaatcgacttaccaaaga | 191348 | - | see also 6451 line |
| 35769 | FLJ10613 | NM_019067.3 | 315 | gccaatcgagaggctgaattaaa | 191354 | - | see also 15723 line |
| 35770 | FLJ10613 | NM_019067.3 | 1393 | atctcagtgctgagatcgttaag | 191364 | - | see also 15723 line |
| 35771 | FLJ25461 | NM_144966.1 | 425 | aactcggccactcctcaaatttt | 191387 | - | see also 15724 line |
| 35772 | FLJ25461 | NM_144966.1 | 553 | ctcggcctttgtgggtataaagg | 191393 | - | see also 15724 line |
| 35773 | IMP5 | NM_175882.1 | 1816 | gacgcccacttggatcctaatga | 191422 | - | see also 15725 line |
| 35774 | IMP5 | NM_175882.1 | 168 | cccactcctgccctgtatgatg | 191416 | - | see also 15725 line |

Figure 46

| 35775 | KYNU | NM_003937.1 | 554 | aagcctacgccaaacgatataa | 191435 | - | see also 15726 line |
|---|---|---|---|---|---|---|---|
| 35776 | KYNU | NM_003937.1 | 555 | agcctacgccaaaacgatataaa | 191436 | - | see also 15726 line |
| 35777 | LOC56926 | NM_020170.1 | 1190 | tgcgtcccgggtggattctaag | 191475 | - | see also 15727 line |
| 35778 | LOC56926 | NM_020170.1 | 1219 | accegtaacacgaggatcattgc | 191477 | - | see also 15727 line |
| 35779 | LOC90701 | NM_033280.1 | 93 | cgcgtccggcttggatatcttcg | 191480 | - | see also 9394 line |
| 35780 | LOC90701 | NM_033280.1 | 357 | aggacgagacattccaatagttc | 191486 | - | see also 9394 line |
| 35781 | MGC10067 | NM_145049.1 | 937 | gcgcacctaaatcgtgataaaga | 191521 | - | see also 15728 line |
| 35782 | MGC10067 | NM_145049.1 | 936 | agcgcacctaaatcgtgataaag | 191520 | - | see also 15728 line |
| 35783 | OTUB1 | NM_017670.1 | 830 | gcctggacactacgatatcctct | 191532 | - | see also 15729 line |
| 35784 | OTUB1 | NM_017670.1 | 299 | caggcctgacggcaactgtttct | 191526 | - | see also 15729 line |
| 35785 | PACS1 | NM_018026.2 | 2152 | tggggtcagtcgacagtaaatac | 191557 | - | see also 15730 line |
| 35786 | PACS1 | NM_018026.2 | 2158 | cagtcgacagtaaatacagtagt | 191559 | - | see also 15730 line |
| 35787 | RNF128 | NM_024539.3 | 779 | ctgaaggctacggaatgcaaga | 191586 | - | see also 8634 line |
| 35788 | RNF128 | NM_024539.3 | 1225 | cacgtgcagtcaacaaatgaaag | 191600 | - | see also 8634 line |
| 35789 | RNF13 | NM_007282.3 | 1024 | ctggcactttcatcgtgttaatt | 191619 | - | see also 5076 line |
| 35790 | RNF13 | NM_007282.3 | 1864 | atggtgaacgggattacaacata | 191630 | - | see also 5076 line |
| 35791 | RNF130 | NM_018434.4 | 1364 | atgtactgataacgtagcattcg | 191659 | - | see also 7470 line |
| 35792 | RNF130 | NM_018434.4 | 977 | cagccgtggctctctagtcttcg | 191649 | - | see also 7470 line |
| 35793 | RNF149 | NM_173647.2 | 556 | cacgcgggaacaggaaatatagt | 191670 | - | see also 15734 line |
| 35794 | RNF149 | NM_173647.2 | 1009 | caccgaacatgtccaatgtgtaa | 191677 | - | see also 15734 line |
| 35795 | RNF149 | NM_173647.2 | 1010 | accgaacacatgccaatgtgtaaa | 191678 | - | see also 15734 line |
| 35796 | SHH | NM_000193.2 | 356 | aactccgagcgatttaaggaact | 191682 | - | see also 15735 line |
| 35797 | SPC12 | NM_014041.1 | 268 | ttcggtggactgtctatatagt | 191687 | - | see also 5470 line |
| 35798 | SPC12 | NM_014041.1 | 265 | cagttcggttggactgtctatat | 191686 | - | see also 5470 line |
| 35799 | SPC18 | NM_014300.2 | 794 | cagggggatttgttccttatattg | 191694 | - | see also 15737 line |
| 35800 | SPC18 | NM_014300.2 | 791 | agccagggggatttgttccttata | 191693 | - | see also 15737 line |
| 35801 | SPPL2A | NM_032802.2 | 632 | atcgtggcctaactttgattata | 191709 | - | see also 9286 line |
| 35802 | SPPL2A | NM_032802.2 | 629 | tccatcgtggcctaactttgatt | 191708 | - | see also 9286 line |
| 35803 | SPPL2B | NM_020172.1 | 1280 | gggtttcggagacattttggtgc | 191759 | - | see also 15739 line |
| 35804 | SPPL2B | NM_020172.1 | 609 | gggagtcgggacgtgaagaaaag | 191756 | - | see also 15739 line |
| 35805 | SPPL2B | NM_020172.1 | 608 | cgggagtcgggacgtgaagaaaa | 191755 | - | see also 15739 line |
| 35806 | SPPL3 | NM_139015.3 | 789 | cagttcttgcaacgagagcttt | 191773 | - | see also 15740 line |
| 35807 | SPPL3 | NM_139015.3 | 1429 | ctgcaccccatcgatactttg | 191784 | - | see also 15740 line |
| 35808 | TFR2 | NM_003227.2 | 1770 | tgccgtcgagttctctcttatgg | 191790 | - | see also 15741 line |
| 35809 | TFR2 | NM_003227.2 | 2126 | agcgactgacacgcatgtacaac | 191792 | - | see also 15741 line |

Figure 46

| 35810 | TFRC | NM_003234.1 | 912 | gggggttatgtggcgtatagtaa | 191823 | - | see also 2184 line | | |
|---|---|---|---|---|---|---|---|---|---|
| 35811 | TFRC | NM_003234.1 | 613 | ctgcagcacgtcgcttatattgg | 191813 | - | see also 2184 line | | |
| 35812 | WFIKKNRP | NM_175575.4 | 1653 | cgcgcctcgctgggcttacaaca | 191873 | - | see also 15744 line | | |
| 35813 | WFIKKNRP | NM_175575.4 | 1655 | cgcctcgctgggcttacaacagc | 191874 | - | see also 15744 line | | |
| 35814 | OCA2 | NM_000275.1 | 1136 | ggcgtctacgcgctgatcatatt | 191889 | - | see also 15745 line | | |
| 35815 | OCA2 | NM_000275.1 | 2319 | tggcgtcgtccctgattgacaac | 191911 | - | see also 15745 line | | |
| 35816 | ATP6V1E1 | NM_001696.2 | 667 | ctggtggagttgagatctataat | 191926 | - | see also 15746 line | | |
| 35817 | ATP6V1E1 | NM_001696.2 | 516 | ctggagccccgaatgattgttcg | 191920 | - | see also 15746 line | | |
| 35818 | ATP6V1E2 | NM_080653.2 | 1568 | atccccgagtacatgacaatttc | 191931 | - | see also 15747 line | | |
| 35819 | ATP6V1E2 | NM_080653.2 | 1569 | tccccgagtacatgacaatttcc | 191932 | - | see also 15747 line | | |
| 35820 | ATP6V1E2 | NM_080653.2 | 1121 | ctgagtgatgtcgatgtgaaaaa | 191928 | - | see also 15747 line | | |
| 35821 | ATP6V1F | NM_004231.2 | 209 | gacactttccggcaatttctaaa | 191936 | - | - | hydrogen-translocating V-type ATPase | - |
| 35822 | ATP6V1F | NM_004231.2 | 211 | cactttccggcaatttctaaacc | 191937 | - | see also 35821 line | | |
| 35823 | ATP1B1 | NM_001677.2 | 941 | cagtgagaaagaccgtttcagg | 191964 | - | see also 15748 line | | |
| 35824 | ATP1B1 | NM_001677.2 | 595 | aagagggcaaaccgtgcattatt | 191947 | - | see also 15748 line | | |
| 35825 | ATP1B3 | NM_001679.1 | 258 | accagtgaccgcattggaatata | 191970 | - | see also 15749 line | | |
| 35826 | ATP1B3 | NM_001679.1 | 804 | gggacgagttatgttcaaaatca | 191981 | - | see also 15749 line | | |
| 35827 | ATP1B3 | NM_001679.1 | 803 | tgggacgagttatgttcaaaatc | 191980 | - | see also 15749 line | | |
| 35828 | ATP1B4 | NM_012069.2 | 932 | acccttactacggcaaactgact | 192003 | - | see also 5105 line | | |
| 35829 | ATP1B4 | NM_012069.2 | 947 | aactgactcacgttaactacaca | 192005 | - | see also 5105 line | | |
| 35830 | ATP1B4 | NM_012069.2 | 364 | gcccgaacaggcctgatcttact | 191987 | - | see also 5105 line | | |
| 35831 | ATP4B | NM_000705.1 | 300 | aaccttaaggccggatgtttacg | 192011 | - | see also 15751 line | | |
| 35832 | ATP4B | NM_000705.1 | 742 | ttcccttattacgggaagaaagc | 192018 | - | see also 15751 line | | |
| 35833 | FXYD2 | NM_001680.3 | 90 | gacccgttctactatgactatga | 192022 | - | see also 15752 line | | |
| 35834 | FXYD2 | NM_001680.3 | 84 | gacgtggacccgttctactatga | 192021 | - | see also 15752 line | | |
| 35835 | ATP6V0E | NM_003945.2 | 267 | tggaccgcaattgaaaaatgaaa | 192026 | - | - | hydrogen-transporting two-sector ATPase | - |
| 35836 | ATP6V0E | NM_003945.2 | 262 | ctctttggaccgcaattgaaaaa | 192025 | - | see also 35835 line | | |
| 35837 | KATNB1 | NM_005886.1 | 380 | cacccgtacggcgagtttgtagc | 192027 | - | see also 15753 line | | |
| 35838 | KATNB1 | NM_005886.1 | 878 | atctgcaatgaccagttgatagg | 192031 | - | see also 15753 line | | |
| 35839 | p44S10 | NM_014814.1 | 425 | ctgggtcaccgattggatattgt | 192048 | - | see also 6005 line | | |
| 35840 | p44S10 | NM_014814.1 | 553 | gagaaaccgcctaaaagtgtatc | 192054 | - | see also 6005 line | | |
| 35841 | NUDT9 | NM_024047.3 | 658 | atcctcagatcagtgaaagtaat | 192090 | - | see also 15755 line | | |

Figure 46

| | | | | | | | |
|---|---|---|---|---|---|---|---|
| 35842 | NUDT9 | NM_024047.3 | 1096 | gccaagacacctagtgatatat | 192103 | - | see also 15755 line |
| 35843 | HTCD37 | NM_021222.1 | 519 | gaccatcgagcgaaacactgc | 192118 | - | see also 15756 line |
| 35844 | HTCD37 | NM_021222.1 | 398 | gagattgacctccatgcattata | 192115 | - | see also 15756 line |
| 35845 | MGC20553 | NM_174938.3 | 809 | gagcccaccagttgctgaattta | 192140 | - | see also 10134 line |
| 35846 | MGC20553 | NM_174938.3 | 1903 | cagagtttgagcagtttcactat | 192159 | - | see also 10134 line |
| 35847 | SHAPY | NM_138793.2 | 930 | gagctatccgacctgattgtttt | 192167 | - | see also 15758 line |
| 35848 | SHAPY | NM_138793.2 | 931 | agctatccgacctgattgtttc | 192168 | - | see also 15758 line |
| 35849 | C21orf6 | NM_016940.1 | 570 | aacacgcctctggctatgtcagc | 192186 | - | see also 6977 line |
| 35850 | C21orf6 | NM_016940.1 | 374 | gacgaaaaatggcgatgttttc | 192181 | - | see also 6977 line |
| 35851 | RWDD2 | NM_033411.2 | 604 | tcctccgaatgtggatctacagt | 192202 | - | see also 9421 line |
| 35852 | RWDD2 | NM_033411.2 | 925 | atctgggccaattcttagaattt | 192213 | - | see also 9421 line |
| 35853 | NUDT3 | NM_006703.2 | 700 | aacccgtgcaggcatcatatttt | 192220 | - | see also 15761 line |
| 35854 | NUDT3 | NM_006703.2 | 763 | cagtcgtgccaccacatactcg | 192221 | - | see also 15761 line |
| 35855 | NUDT4 | NM_019094.3 | 865 | ccttccttccggataataatg | 192224 | - | phosphatase |
| 35856 | NUDT4 | NM_019094.3 | 864 | tcccttcccttccggatataat | 192223 | - | see also 35855 line |
| 35857 | NUDT4 | NM_019094.3 | 863 | gtccttcccttccggataataa | 192222 | - | see also 35855 line |
| 35858 | ITPA | NM_033453.2 | 291 | atgagatttccatacagaaatgt | 192227 | - | see also 15762 line |
| 35859 | ITPA | NM_033453.2 | 279 | aggggagccggatgagagatttcc | 192226 | - | see also 15762 line |
| 35860 | NUDT2 | NM_001161.3 | 610 | caggaacaagcctaaaacagtca | 192237 | - | see also 741 line |
| 35861 | NUDT2 | NM_001161.3 | 556 | agcaggccagctgaccattattg | 192235 | - | see also 741 line |
| 35862 | RRAGD | NM_021244.2 | 888 | tagtactccggtggatatgcaaa | 192251 | - | see also 8144 line |
| 35863 | RRAGD | NM_021244.2 | 289 | aagtcgtcattcagaaagttgt | 192239 | - | see also 8144 line |
| 35864 | FLJ10458 | NM_018096.2 | 1373 | ggccacgcggatgaggtatatgc | 192268 | - | see also 15765 line |
| 35865 | FLJ10458 | NM_018096.2 | 1054 | ctccgacacttcacctattcc | 192266 | - | see also 15765 line |
| 35866 | GNB1 | NM_002074.2 | 672 | tgcggtggctgataaacattg | 192277 | - | see also 15766 line |
| 35867 | GNB1 | NM_002074.2 | 551 | ctcgcaggatggtaaacttatca | 192273 | - | see also 15766 line |
| 35868 | GNB2 | NM_005273.2 | 311 | tccgggatgcccgaaaagcatgt | 192282 | - | see also 15767 line |
| 35869 | GNB2 | NM_005273.2 | 310 | atccgggatgcccgaaaagcatg | 192281 | - | see also 15767 line |
| 35870 | GNB3 | NM_002075.2 | 474 | agcctgctgacgttactctgg | 192287 | - | see also 1339 line |
| 35871 | GNB3 | NM_002075.2 | 822 | ttctgctcacacaggttatctct | 192290 | - | see also 1339 line |
| 35872 | GNB4 | NM_021629.2 | 392 | gactctgtgggtcgaatacaaat | 192299 | - | see also 15769 line |
| 35873 | GNB4 | NM_021629.2 | 360 | atgcaacgcttgttcagattaca | 192296 | - | see also 15769 line |
| 35874 | GNB5 | NM_006578.2 | 866 | cgctacgtgtcgcctctatgacc | 192340 | - | see also 15770 line |
| 35875 | GNB5 | NM_006578.2 | 480 | tacccttgacgtttgacaaaaa | 192336 | - | see also 15770 line |
| 35876 | GNB5 | NM_006578.2 | 481 | accccttgacgtttgacaaaaat | 192337 | - | see also 15770 line |

Figure 46

| | | | | | | | |
|---|---|---|---|---|---|---|---|
| 35877 | GNG13 | NM_016541.1 | 92 | gggacgtgccacagatgaagaaa | 192350 | - | see also 15772 line |
| 35878 | GNG3 | NM_012202.1 | 276 | ccggtgaacagcactatgatgat | 192351 | - | see also 15773 line |
| 35879 | GNG4 | NM_004485.2 | 364 | ccggaagatcctctcatcattcc | 192354 | - | see also 15774 line |
| 35880 | GNG5 | NM_005274.1 | 398 | gactcaacgcgtaaaagtttcc | 192358 | - | gpcr, signal_transduction signal transducer see also 15772 line |
| 35881 | GNG5 | NM_005274.1 | 396 | cggactcaacgcgtaaaagtt | 192357 | - | see also 35880 line |
| 35882 | GNG5 | NM_005274.1 | 394 | gcccggactcaaccgcgtaaaagt | 192356 | - | see also 35880 line |
| 35883 | GNG8 | NM_033258.1 | 181 | cgcgacaagcgcctctttgtgt | 192361 | - | gpcr, signal_transduction signal transducer see also 15772 line |
| 35884 | GNG8 | NM_033258.1 | 118 | tgcgagacgcatgccaaagatga | 192359 | - | see also 35883 line |
| 35885 | GNG8 | NM_033258.1 | 179 | tccgcgacaagcgcctctttgt | 192360 | - | see also 35883 line |
| 35886 | GNGT2 | NM_031498.1 | 438 | aggaaacgatccttttctcaaag | 192366 | - | see also 15776 line |
| 35887 | GNGT2 | NM_031498.1 | 490 | gagaaaggtggctgtctgataag | 192367 | - | see also 15776 line |
| 35888 | RGS11 | NM_003834.1 | 1300 | gacgcgttccgtttacgtgg | 192380 | - | see also 15777 line |
| 35889 | RGS11 | NM_003834.1 | 334 | cccgtacttctggacaagtacc | 192370 | - | see also 15777 line |
| 35890 | RGS11 | NM_003834.1 | 849 | gacgccactgggtcatgaatgc | 192378 | - | see also 15777 line |
| 35891 | RGS6 | NM_004296.3 | 1525 | cgcttcctccgtcaaatgctta | 192419 | - | see also 2877 line |
| 35892 | RGS6 | NM_004296.3 | 1068 | tgcctacacggaacaatatgtgg | 192404 | - | see also 2877 line |
| 35893 | RGS7 | NM_002924.2 | 457 | ttcctattcgtacggtcaaaagc | 192423 | - | see also 15779 line |
| 35894 | RGS7 | NM_002924.2 | 710 | cacagattatgccgtttacctct | 192432 | - | see also 15779 line |
| 35895 | RGS9 | NM_003835.1 | 1014 | gaggcggctgatcaacatagatg | 192468 | - | see also 15780 line |
| 35896 | RGS9 | NM_003835.1 | 1012 | gcgaggggctggatcaacataga | 192467 | - | see also 15780 line |
| 35897 | WDR5B | NM_019069.2 | 1116 | aacgctcgttgatgacgataacc | 192487 | - | see also 15781 line |
| 35898 | WDR5B | NM_019069.2 | 684 | ttctgtctgatagggctaatcataa | 192477 | - | see also 15781 line |
| 35899 | ARHGAP5 | NM_001173.1 | 616 | tgcctcatcggagtacgaatttg | 192507 | - | see also 744 line |
| 35900 | ARHGAP5 | NM_001173.1 | 617 | gcctcatggagtaacgaatttgc | 192508 | - | see also 744 line |
| 35901 | ARHGAP5 | NM_001173.1 | 325 | ctgtccccatcctataccatc | 192494 | - | see also 744 line |
| 35902 | GTPBP3 | NM_032620.1 | 643 | cacgtggaggcctatatcgattt | 192648 | - | see also 15783 line |
| 35903 | GTPBP3 | NM_032620.1 | 1377 | cggccactacaagcagtcaaaag | 192651 | - | see also 15783 line |
| 35904 | MFN1 | NM_017927.1 | 1092 | ttcgaacagcacactatcagagc | 192682 | - | see also 15784 line |
| 35905 | MFN1 | NM_017927.1 | 397 | ttggccatataaccaattgcttc | 192664 | - | see also 15784 line |
| 35906 | SAMHD1 | NM_015474.2 | 524 | ctgtccgaatcattgattacacc | 192732 | - | see also 15785 line |
| 35907 | SAMHD1 | NM_015474.2 | 521 | ctcctcgtccgaatcattgatac | 192730 | - | see also 15785 line |
| 35908 | ACP1 | NM_004300.2 | 158 | tgttaaccgatcaaaacatctca | 192792 | - | phosphatase |
| 35909 | ACP1 | NM_004300.2 | 155 | aactgtaaccgatcacaaacatc | 192791 | - | see also 35908 line |
| 35910 | ACP1 | NM_004300.2 | 420 | tgggagctatgatccacacaaaac | 192796 | - | see also 35908 line |
| 35911 | CDC25A | NM_001789.1 | 1184 | ctcctctcgtcatgagaactaca | 192812 | - | see also 15786 line |

Figure 46

| 35912 | CDC25A | NM_001789.1 | 1606 | gactgtcgatacccatatgaata | 192820 | - | see also 15786 line |
|---|---|---|---|---|---|---|---|
| 35913 | CDC25B | NM_004358.2 | 582 | agccagccggatcattcgaaacg | 192832 | - | see also 15787 line |
| 35914 | CDC25B | NM_004358.2 | 1664 | cccgcatgtgccgtttcatcagg | 192844 | - | see also 15787 line |
| 35915 | CDC25B | NM_004358.2 | 1283 | cccgcgtcctccgctcaaaatca | 192835 | - | see also 15787 line |
| 35916 | CDC25C | NM_001790.2 | 1480 | ttgtactacccagagctatatat | 192883 | - | see also 15788 line |
| 35917 | CDC25C | NM_001790.2 | 324 | acccagttttaggtctaatcaaa | 192846 | - | see also 15788 line |
| 35918 | DUSP18 | NM_152511.2 | 956 | tccactatgagttccaattgttt | 192891 | - | see also 15789 line |
| 35919 | DUSP18 | NM_152511.2 | 675 | caccttgtatgaggatatccagt | 192887 | - | see also 15789 line |
| 35920 | DUSP19 | NM_080876.2 | 399 | ttgctcctagggtcacaagatgc | 192898 | - | see also 9541 line |
| 35921 | DUSP19 | NM_080876.2 | 652 | ctgctgcaattgtaataggtttc | 192901 | - | see also 9541 line |
| 35922 | MTM1 | NM_000252.1 | 1737 | agccttacgcgacgaatacataa | 192973 | - | see also 188 line |
| 35923 | MTM1 | NM_000252.1 | 1738 | gccttacgcgacgaatacataaa | 192974 | - | see also 188 line |
| 35924 | MTMR3 | NM_021090.2 | 3373 | agcgcctgcgtcagattgagtca | 193028 | - | see also 8083 line |
| 35925 | MTMR3 | NM_021090.2 | 2117 | gactagatcatacgacaatctga | 193013 | - | see also 8083 line |
| 35926 | PTP4A1 | NM_003463.2 | 1094 | ttggagaagtatcgtcctaaaat | 193052 | - | see also 15793 line |
| 35927 | PTP4A1 | NM_003463.2 | 1135 | ttccaacggtcatagaaacaact | 193056 | - | see also 15793 line |
| 35928 | PTP4A3 | NM_007079.2 | 418 | cacgctcagcaccttcattgagg | 193057 | - | phosphatase | - | - |
| 35929 | PTPN1 | NM_002827.2 | 331 | gaccatagtcggattaaactaca | 193061 | - | see also 15794 line |
| 35930 | PTPN1 | NM_002827.2 | 373 | atcaacgctagtttgataaaaat | 193065 | - | see also 15794 line |
| 35931 | PTPN11 | NM_002834.3 | 1023 | ccccttaacacgactcgtataaa | 193105 | - | see also 1854 line |
| 35932 | PTPN11 | NM_002834.3 | 1904 | agcacagtaccgatttatctata | 193119 | - | see also 1854 line |
| 35933 | PTPN11 | NM_002834.3 | 1024 | cccttaacacgactcgtataaat | 193106 | - | see also 1854 line |
| 35934 | PTPN12 | NM_002835.2 | 2207 | tccagcgggaggtattcactatg | 193215 | - | see also 1855 line |
| 35935 | PTPN12 | NM_002835.2 | 246 | agccgagttaaattgacattaaa | 193142 | - | see also 1855 line |
| 35936 | PTPN12 | NM_002835.2 | 316 | gcgtctatgggccaaaagcatat | 193147 | - | see also 1855 line |
| 35937 | PTPN13 | NM_006264.1 | 7325 | tgcgctgcatgagactacaaaga | 193416 | - | see also 4351 line |
| 35938 | PTPN13 | NM_006264.1 | 4778 | gtgtgctaccggaaattgatact | 193344 | - | see also 4351 line |
| 35939 | PTPN14 | NM_005401.3 | 3562 | gtcggtccgtcgccataccaaca | 193483 | - | see also 3767 line |
| 35940 | PTPN14 | NM_005401.3 | 1155 | ttgccacacgacacaagttttac | 193447 | - | see also 3767 line |
| 35941 | PTPN18 | NM_014369.2 | 450 | gcctgtcgagagatagagaatgg | 193496 | - | see also 15799 line |
| 35942 | PTPN18 | NM_014369.2 | 581 | caggaccctcaaggtcacattcc | 193499 | - | see also 15799 line |
| 35943 | PTPN2 | NM_002828.2 | 955 | accgaatgggtcttattcagacc | 193525 | - | see also 15800 line |
| 35944 | PTPN2 | NM_002828.2 | 647 | gtcgtattatacagtacatctac | 193518 | - | see also 15800 line |
| 35945 | PTPN21 | NM_007039.2 | 2831 | cccgcctctaggggggaatgaaaa | 193567 | - | see also 4927 line |
| 35946 | PTPN21 | NM_007039.2 | 3587 | gtctgttcgacgccatacacaata | 193585 | - | see also 4927 line |
| 35947 | PTPN22 | NM_012411.2 | 672 | atctatagaccctattcttgagc | 193610 | - | see also 5312 line |
| 35948 | PTPN22 | NM_012411.2 | 700 | tgggatgtacgttgttaccaaga | 193611 | - | see also 5312 line |

Figure 46

| | | | | | | | |
|---|---|---|---|---|---|---|---|
| 35949 | PTPN3 | NM_002829.2 | 2068 | atgacaccacccgggtattattg | 193718 | - | see also 15803 line |
| 35950 | PTPN3 | NM_002829.2 | 1078 | cagccatgcggagatccttatca | 193700 | - | see also 15803 line |
| 35951 | PTPN4 | NM_002830.2 | 1996 | acccggttacgaccatcttcagt | 193780 | - | see also 1836 line |
| 35952 | PTPN4 | NM_002830.2 | 1947 | tccaccggaactcctaatcatc | 193777 | - | see also 1836 line |
| 35953 | PTPN5 | NM_032781.2 | 1546 | ggcgtacgacggtgttgagatca | 193850 | - | see also 15805 line |
| 35954 | PTPN5 | NM_032781.2 | 1271 | aaccggtacaaaaccatcacttcc | 193846 | - | see also 15805 line |
| 35955 | PTPN5 | NM_032781.2 | 1638 | agcgaggcctgaagcattactgg | 193852 | - | see also 15805 line |
| 35956 | PTPN6 | NM_002831.3 | 796 | tacctgcggcagccgtactatgc | 193858 | - | see also 1850 line |
| 35957 | PTPN6 | NM_002831.3 | 1063 | cccgggtccgactacatcaatgc | 193863 | - | see also 1850 line |
| 35958 | PTPN6 | NM_002831.3 | 556 | ggcgagccctggacgttcttgt | 193856 | - | see also 1850 line |
| 35959 | PTPN7 | NM_002832.2 | 488 | cgcctccaaggaacgatacaaga | 193877 | - | see also 15807 line |
| 35960 | PTPN7 | NM_002832.2 | 562 | aggaggacggagattacacatcaat | 193879 | - | see also 15807 line |
| 35961 | PTPN9 | NM_002833.2 | 1211 | ctgggtgggtacgtcaaaattga | 193898 | - | see also 1851 line |
| 35962 | PTPN9 | NM_002833.2 | 1216 | tgggtacgtcaaaattgatctcg | 193900 | - | see also 1851 line |
| 35963 | PTPRA | NM_002836.2 | 1934 | cccgatcggcatgctcaagttcc | 193936 | - | see also 1858 line |
| 35964 | PTPRA | NM_002836.2 | 898 | tgggaccccactatttaaccact | 193922 | - | see also 1858 line |
| 35965 | PTPRB | NM_002837.2 | 5006 | ttcgtagggatcgaccattatct | 194080 | - | see also 1860 line |
| 35966 | PTPRB | NM_002837.2 | 2636 | ccgcaaccgactcgatttact | 194024 | - | see also 1860 line |
| 35967 | PTPRD | NM_002839.1 | 4580 | cccacgcgactcgttcaagtaacg | 194329 | - | see also 15811 line |
| 35968 | PTPRD | NM_002839.1 | 5533 | gacggccagtccgaacagtaag | 194364 | - | see also 15811 line |
| 35969 | PTPRE | NM_006504.3 | 2031 | ccgagtagtgcggccagttcact | 194409 | - | see also 15812 line |
| 35970 | PTPRE | NM_006504.3 | 1966 | gccatcagtatacgagacttct | 194408 | - | see also 15812 line |
| 35971 | PTPRJ | NM_002843.2 | 2142 | tcgggcaccttatataaacatca | 194469 | - | see also 1868 line |
| 35972 | PTPRJ | NM_002843.2 | 2679 | tagaacggaagtcacgtatttga | 194486 | - | see also 1868 line |
| 35973 | PTPRK | NM_002844.2 | 2388 | cccagtgcgtacgcattgctaca | 194597 | - | see also 1870 line |
| 35974 | PTPRK | NM_002844.2 | 2392 | gtgcgtaagcattgctacacaaaag | 194598 | - | see also 1870 line |
| 35975 | PTPRM | NM_002845.2 | 2502 | atcggtgcctaattcctattacc | 194732 | - | see also 1875 line |
| 35976 | PTPRM | NM_002845.2 | 330 | tccgggttactcaatgtctacg | 194671 | - | see also 1875 line |
| 35977 | PTPRN2 | NM_002847.2 | 245 | aggttccggcaatggactttac | 194771 | - | see also 1880 line |
| 35978 | PTPRN2 | NM_002847.2 | 1201 | caggacgacgatgatagacttta | 194779 | - | see also 1880 line |
| 35979 | PTPRO | NM_002848.2 | 2594 | tagccgtccaacaatggttaca | 194884 | - | see also 1882 line |
| 35980 | PTPRO | NM_002848.2 | 2575 | cccagtgtccctacgttcatgc | 194883 | - | see also 1882 line |
| 35981 | PTPRS | NM_002850.2 | 4795 | tccaggtcacgttgctagatacc | 194936 | - | see also 15818 line |
| 35982 | PTPRS | NM_002850.2 | 3925 | agcagtatggcggctccgataac | 194931 | - | see also 15818 line |
| 35983 | PTPRT | NM_007050.3 | 2927 | aaccgcaataagaatcgatatgg | 194971 | - | see also 4934 line |
| 35984 | PTPRT | NM_007050.3 | 3029 | aactacattgacggatgaccatcg | 194973 | - | see also 4934 line |
| 35985 | PTPRU | NM_005704.2 | 488 | ggcgtcacgtgcgcgttaatgg | 194995 | - | see also 15820 line |

Figure 46

| | | | | | | | | | |
|---|---|---|---|---|---|---|---|---|---|
| 35986 | PTPRU | NM_005704.2 | 3206 | gagtatgtcgtgcgcacttttgc | 195017 | - | see also 15820 line | | |
| 35987 | PTPRU | NM_005704.2 | 3919 | ctggcggctggtctacgattacg | 195023 | - | see also 15820 line | | |
| 35988 | TPTE | NM_013315.2 | 1618 | aacacttcattatttattcgatt | 195030 | - | see also 15821 line | | |
| 35989 | TPTE | NM_013315.2 | 1642 | ctcgttatgtacgtgatctaaaa | 195031 | - | see also 15821 line | | |
| 35990 | PTPRH | NM_002842.1 | 1819 | cccactctcagttgtacgtatac | 195040 | - | see also 15822 line | | |
| 35991 | PTPRH | NM_002842.1 | 1954 | aacccgggacgttgtacaatttc | 195043 | - | see also 15822 line | | |
| 35992 | PTPRH | NM_002842.1 | 1384 | agcccggaaccttgtacacattc | 195038 | - | see also 15822 line | | |
| 35993 | PTPRN | NM_002846.2 | 2362 | gcccttctcggagcgattacatc | 195068 | - | see also 15823 line | | |
| 35994 | PTPRN | NM_002846.2 | 2363 | cccttctcggagcgattacatca | 195069 | - | see also 15823 line | | |
| 35995 | PTPRN | NM_002846.2 | 2865 | ctgaaccgcatggcaaaaggagt | 195074 | - | see also 15823 line | | |
| 35996 | DUSP1 | NM_004417.2 | 1062 | taccttatgaggactaatcgagt | 195083 | - | see also 2957 line | | |
| 35997 | DUSP1 | NM_004417.2 | 1315 | accttcagagccccattacgacc | 195085 | - | see also 2957 line | | |
| 35998 | DUSP1 | NM_004417.2 | 869 | agccaattgtcccaaccattttg | 195080 | - | see also 2957 line | | |
| 35999 | ACPP | NM_001099.2 | 665 | aagtctacgacccttttatattgt | 195110 | - | see also 685 line | | |
| 36000 | ACPP | NM_001099.2 | 980 | gccacttgacggaattgtacttt | 195120 | - | see also 685 line | | |
| 36001 | CDC14A | NM_003672.2 | 512 | aagatcggttatattttgctact | 195127 | - | see also 15826 line | | |
| 36002 | CDC14A | NM_003672.2 | 764 | gtgcctatgcagtaatctattta | 195131 | - | see also 15826 line | | |
| 36003 | CDC14A | NM_003672.2 | 762 | aggtgcctatgcagtaatctatt | 195130 | - | see also 15826 line | | |
| 36004 | CDC14B | NM_033331.1 | 1689 | cccgaaccgtacagtgatgatga | 331968 | - | phosphatase | - | - |
| 36005 | CDC14B | NM_033331.1 | 699 | gagaacttctacgcagattttgg | 331966 | - | see also 36004 line | | |
| 36006 | CDC14B | NM_033331.1 | 1331 | tgccattgtcaaagaattcctag | 331967 | - | see also 36004 line | | |
| 36007 | DKFZP566 K0524 | NM_015605.3 | 528 | tacgcgcaaaattgtttctgaag | 195169 | - | phosphatase | - | - |
| 36008 | DKFZP566 K0524 | NM_015605.3 | 811 | ttctggcatggttcaaacgaagg | 195170 | - | see also 36007 line | | |
| 36009 | DUSP2 | NM_004418.2 | 729 | gccttttccgctacaagagtatc | 195173 | - | see also 15827 line | | |
| 36010 | DUSP2 | NM_004418.2 | 726 | agggccttttccgctacaagagt | 195172 | - | see also 15827 line | | |
| 36011 | DUSP3 | NM_004090.2 | 340 | ttcaacctcagcgcttactttga | 195175 | - | see also 2758 line | | |
| 36012 | DUSP5 | NM_004419.2 | 598 | gagactttctactcggaatatcc | 195182 | - | see also 15828 line | | |
| 36013 | DUSP5 | NM_004419.2 | 1102 | agcatggtctcgcccaactttgg | 195186 | - | see also 15828 line | | |
| 36014 | DUSP6 | NM_001946.1 | 1332 | atccaacatatcccctaacttca | 195196 | - | see also 15829 line | | |
| 36015 | DUSP6 | NM_001946.1 | 1448 | ccccttccaaccagaatgtatac | 195198 | - | see also 15829 line | | |
| 36016 | DUSP7 | NM_001947.1 | 1032 | gtcgagtgagcagctctactttt | 195202 | - | see also 15830 line | | |
| 36017 | DUSP7 | NM_001947.1 | 359 | tccaaggtggtttcaacaagttt | 195199 | - | see also 15830 line | | |
| 36018 | DUSP8 | NM_004420.1 | 815 | gtccatcgagttcatcgataaag | 195204 | - | phosphatase、kinase_r elated | protein tyrosine/serine/threoni ne phosphatase | - |

Figure 46

| | | | | | | | | | | |
|---|---|---|---|---|---|---|---|---|---|---|
| 36019 | EPM2A | NM_005670.2 | 993 | gactccagacactatgattaaac | 195222 | - | see also 15831 line | | | |
| 36020 | EPM2A | NM_005670.2 | 866 | ctcgtcaggtggaacatgtaacc | 195215 | - | see also 15831 line | | | |
| 36021 | MTMR1 | NM_003828.1 | 789 | aacggacaggcactatttgcatt | 195248 | - | see also 15832 line | | | |
| 36022 | MTMR1 | NM_003828.1 | 1380 | gggacgcattggctggaatatat | 195263 | - | see also 15832 line | | | |
| 36023 | MTMR6 | NM_004685.2 | 1003 | tccagtcaatcgctatatgtacg | 195307 | - | see also 3213 line | | | |
| 36024 | MTMR6 | NM_004685.2 | 334 | gacgaccaaggtcgaacaagtaa | 195283 | - | see also 3213 line | | | |
| 36025 | PTEN | NM_000314.2 | 1647 | ttcagtggcggaacttatcctca | 195339 | - | - | prenylated protein tyrosine phosphatase | bladder transitional cell carcinoma、 cowden disease、 breast cancer、 sstrosytoma、 Bannayan-Riley-Ruvalcaba syndrome(-)、 Bannayan-Zonana syndrome(-)、 endometrial cancer、 Lhermitte-Duclos syndrome、 lung cancer、 multiple hamartoma、 polyposis、 prostate cancer、 CNS Tumours、 melanoma、 meningioma、 lhermitte-Duclos disease、 squamous cell carcinomacytic、 head and neck squamous cell carcinoma、 astrocytic tumors、 familial nervous system tumor syndromes |
| 36026 | PTP9Q22 | NM_152422.3 | 347 | gagcgtttacggcatgtcattcc | 195341 | - | see also 15834 line | | | |
| 36027 | PTP9Q22 | NM_152422.3 | 2357 | aaggcattcactaaggttaattt | 195392 | - | see also 15834 line | | | |
| 36028 | PTPLA | NM_014241.2 | 306 | agctattgccatggtacgttttt | 195399 | - | see also 5552 line | | | |
| 36029 | PTPLA | NM_014241.2 | 311 | ttgccatggtacgtttttatatg | 195400 | - | see also 5552 line | | | |
| 36030 | PTPN23 | NM_015466.1 | 392 | aagtacgagcaggcctgtattct | 195428 | - | see also 15836 line | | | |
| 36031 | PTPN23 | NM_015466.1 | 682 | tgcacaggtggtagattactaca | 195432 | - | see also 15836 line | | | |
| 36032 | TENS1 | NM_022748.6 | 306 | tacaacgaccacggtgtgattgt | 195454 | - | see also 15837 line | | | |
| 36033 | TENS1 | NM_022748.6 | 290 | agggtccgaacacttgtacaacg | 195453 | - | see also 15837 line | | | |
| 36034 | CDKN3 | NM_005192.2 | 131 | cagactccaattcatatatcatg | 195491 | - | see also 15838 line | | | |
| 36035 | CDKN3 | NM_005192.2 | 268 | ctgtggtatacaagacatatttg | 195495 | - | see also 15838 line | | | |
| 36036 | DUSP10 | NM_007207.3 | 1494 | gtcaaaggcaaacgaccaattat | 195532 | - | see also 5013 line | | | |
| 36037 | DUSP10 | NM_007207.3 | 1576 | gtgtgacaccgagaatccttaca | 195534 | - | see also 5013 line | | | |
| 36038 | DUSP13 | NM_016364.2 | 309 | tgccacactgaaccatatcgatg | 195536 | - | phosphatase | protein tyrosine/serine/threonine phosphatase | - |
| 36039 | DUSP13 | NM_016364.2 | 306 | ggctgccacactgaaccatatcg | 195535 | - | see also 36038 line | | | |
| 36040 | DUSP14 | NM_007026.1 | 596 | gtgtatcgcgtacctgatgaaat | 195548 | - | see also 15840 line | | | |
| 36041 | DUSP14 | NM_007026.1 | 772 | tagttcccgacgtctatgagaag | 195550 | - | see also 15840 line | | | |
| 36042 | DUSP16 | NM_030640.1 | 2067 | agccagagcaagcgattgcattc | 195584 | - | see also 8917 line | | | |

Figure 46

| 36043 | DUSP16 | NM_030640.1 | 2243 | gggctggcactcggatatcttgg | 195588 | - | see also 8917 line |
|-------|--------|-------------|------|-------------------------|--------|---|--------------------|
| 36044 | DUSP4 | NM_001394.4 | 1051 | ggcatcacggctctgttgaatgt | 195598 | - | see also 15842 line |
| 36045 | DUSP4 | NM_001394.4 | 844 | tccgagtacccagaattctgttc | 195597 | - | see also 15842 line |
| 36046 | DUSP9 | NM_001395.1 | 761 | ccgggattccgccaatttggaga | 195601 | - | see also 15843 line |
| 36047 | DUSP9 | NM_001395.1 | 802 | tccgctacatcctcaatgtcacc | 195602 | - | see also 15843 line |
| 36048 | PPP1R3A | NM_002711.2 | 1489 | tacgtcggcatgtctcaaagaat | 195651 | - | see also 1765 line |
| 36049 | PPP1R3A | NM_002711.2 | 27 | aagtacctagtcagattagcaaa | 195605 | - | see also 1765 line |
| 36050 | PPP1R3C | NM_005398.2 | 178 | ggcccgtacgatgaatttcaacg | 195713 | - | see also 15845 line |
| 36051 | PPP1R3C | NM_005398.2 | 908 | cagagttagagtcaacaatcttt | 195734 | - | see also 15845 line |
| 36052 | PPP2R1A | NM_014225.3 | 605 | ggcggaacttcgacagtacttcc | 195741 | - | see also 15846 line |
| 36053 | PPP2R1A | NM_014225.3 | 1255 | tacggctgaacatcatctctaac | 195744 | - | see also 15846 line |
| 36054 | PPP2R1B | NM_002716.3 | 1232 | gtcctgacgttcgtttgaatatc | 195775 | - | see also 15847 line |
| 36055 | PPP2R1B | NM_002716.3 | 135 | cccgatcgcggtttaatcgacg | 195749 | - | see also 15847 line |
| 36056 | PPP2R2A | NM_002717.2 | 1441 | aagcatcgcgggaaaacaataag | 195835 | - | see also 15848 line |
| 36057 | PPP2R2A | NM_002717.2 | 1183 | gccatagtggtcgatatatgatg | 195826 | - | see also 15848 line |
| 36058 | PPP2R2A | NM_002717.2 | 1590 | atcattgccgtagctactacaaa | 195846 | - | see also 15848 line |
| 36059 | PPP1R3D | NM_006242.3 | 1055 | agggcacggaggacgttttcacc | 195849 | - | see also 15849 line |
| 36060 | DUSP21 | NM_022076.2 | 363 | ctcgcgtctctacgacttttttg | 195855 | - | see also 15850 line |
| 36061 | DUSP21 | NM_022076.2 | 479 | gccttgcgtacctcatgaaatac | 195859 | - | see also 15850 line |
| 36062 | DUSP22 | NM_020185.3 | 729 | cactggtgatcgcatacatcatg | 195865 | - | see also 7790 line |
| 36063 | DUSP22 | NM_020185.3 | 727 | gacactggtgatcgcatacatca | 195864 | - | see also 7790 line |
| 36064 | MGC1136 | NM_024025.1 | 496 | ggcaagacagcctgtaaccatgc | 195869 | - | see also 15852 line |
| 36065 | MGC1136 | NM_024025.1 | 438 | gccaaccgttcaacatcctttcc | 195868 | - | see also 15852 line |
| 36066 | MK-STYX | NM_016086.2 | 969 | tgcacatccggatagaagattcc | 195884 | - | see also 15853 line |
| 36067 | MK-STYX | NM_016086.2 | 745 | ctcaggcacgtaccactttctcc | 195881 | - | see also 15853 line |
| 36068 | MTMR2 | NM_016156.2 | 1953 | gtgtcactgtggtcttacataaa | 195943 | - | see also 15854 line |
| 36069 | MTMR2 | NM_016156.2 | 1661 | aaccatccgaggatttgaagtcc | 195932 | - | see also 15854 line |
| 36070 | SBF1 | NM_002972.1 | 5699 | aacgcgtcgcgtttacaacttct | 195972 | - | see also 15855 line |
| 36071 | SBF1 | NM_002972.1 | 5701 | cgcgtcgcgtttacaacttctgt | 195973 | - | see also 15855 line |
| 36072 | SBF1 | NM_002972.1 | 5696 | gacaacgcgtcgcgtttacaact | 195971 | - | see also 15855 line |
| 36073 | SSH-3 | NM_017857.2 | 1677 | gccacggccacgtataaacctcc | 195983 | - | see also 15856 line |
| 36074 | SSH-3 | NM_017857.2 | 699 | ggccacactccaggtattgcacc | 195976 | - | see also 15856 line |
| 36075 | SSH2 | NM_033389.2 | 3543 | aagtagcgcagacctaagtttaa | 196062 | - | see also 9415 line |
| 36076 | SSH2 | NM_033389.2 | 566 | aagtcgccagagcgcataactac | 196001 | - | see also 9415 line |
| 36077 | STYX | NM_145251.2 | 557 | tccctatgactaaggaatttatt | 196080 | - | see also 15858 line |
| 36078 | STYX | NM_145251.2 | 741 | tgctggatttgtccatcaacttc | 196083 | - | see also 15858 line |
| 36079 | CILP | NM_003613.1 | 2883 | gggggatcgatatgactacaaca | 196114 | - | see also 2428 line |

Figure 46

| 36080 | CILP | NM_003613.1 | 367 | tactatggggaccgtgtatgtgc | 196092 | - | see also 2428 line |
|---|---|---|---|---|---|---|---|
| 36081 | CTDP1 | NM_004715.2 | 425 | cagccacccggttgtcatgaaag | 196125 | - | see also 15860 line |
| 36082 | CTDP1 | NM_004715.2 | 1008 | aagtttgcccccaatctgataac | 196134 | - | see also 15860 line |
| 36083 | DNAJC6 | NM_014787.1 | 728 | gccattcgattgctatatgcaaa | 196168 | - | see also 5971 line |
| 36084 | DNAJC6 | NM_014787.1 | 1861 | tgcgtctccaaccctaagagtgg | 196189 | - | see also 5971 line |
| 36085 | FLJ22405 | NM_022485.2 | 517 | atggacgctcaggctacaactat | 196206 | - | see also 15862 line |
| 36086 | FLJ22405 | NM_022485.2 | 620 | cacgcatcttttgataaggtca | 196212 | - | see also 15862 line |
| 36087 | ILVBL | NM_006844.3 | 336 | cgctggtcggtgggcacatttcc | 196221 | - | see also 15863 line |
| 36088 | ILVBL | NM_006844.3 | 506 | acggtgactgcggtgaagaatgc | 196223 | - | see also 15863 line |
| 36089 | MTMR8 | NM_017677.2 | 112 | tggatcgttatgtgagtaagaaa | 196246 | - | see also 15864 line |
| 36090 | MTMR8 | NM_017677.2 | 212 | atggattgcactccatcacattg | 196251 | - | see also 15864 line |
| 36091 | PPAP2A | NM_003711.2 | 1028 | tggtcttgttgccgtatccattt | 196333 | - | see also 2535 line |
| 36092 | PPAP2A | NM_003711.2 | 1195 | aagaggaggactctcatacaact | 196343 | - | see also 2535 line |
| 36093 | PPAP2B | NM_003713.3 | 929 | ccctacgtggcagcactctataa | 196354 | - | see also 2536 line |
| 36094 | PPAP2B | NM_003713.3 | 935 | gtggcagcactctataagcaagt | 196355 | - | see also 2536 line |
| 36095 | PPAP2C | NM_003712.2 | 187 | gccccgtacaagcgaggattta | 196363 | - | see also 15866 line |
| 36096 | PPAP2C | NM_003712.2 | 348 | ttctcgctcggacttcaacaact | 196368 | - | see also 15866 line |
| 36097 | PPAP2C | NM_003712.2 | 188 | ccccgtacaagcgaggattttac | 196364 | - | see also 15866 line |
| 36098 | RNGTT | NM_003800.1 | 1175 | atggtactcggtacatgatgttg | 196393 | - | see also 2598 line |
| 36099 | RNGTT | NM_003800.1 | 1455 | aagtcctcgacacgaaaaaatga | 196408 | - | see also 2598 line |
| 36100 | RSC1A1 | NM_006511.1 | 1353 | tggcctgtcaaccgataaggaag | 196459 | - | see also 15868 line |
| 36101 | RSC1A1 | NM_006511.1 | 132 | ctcagatcgcattgaacctaaag | 196430 | - | see also 15868 line |
| 36102 | INPP5A | NM_005539.2 | 693 | gagatctactcggatacccttaga | 196481 | - | see also 15870 line |
| 36103 | INPP5A | NM_005539.2 | 521 | gtcgagtgatgcgatgaaagaat | 196477 | - | see also 15870 line |
| 36104 | INPP5E | NM_019892.2 | 1204 | cccacggcgtgctctacatgtcg | 196499 | - | see also 15871 line |
| 36105 | INPP5E | NM_019892.2 | 850 | cccttcgctcggccaaatcctcc | 196497 | - | see also 15871 line |
| 36106 | SKIP | NM_016532.2 | 1267 | ggctgcgggacgttaatgactac | 196525 | - | see also 15872 line |
| 36107 | SKIP | NM_016532.2 | 276 | aacaaccggaacctcaatcttga | 196511 | - | see also 15872 line |
| 36108 | OCRL | NM_000276.3 | 1784 | aacgaaggtaccggaaagtcttt | 196565 | | see also 227 line |
| 36109 | OCRL | NM_000276.3 | 2384 | ttctagtagatcacctattcaaa | 196579 | | see also 227 line |
| 36110 | IMPA2 | NM_014214.1 | 1039 | ggccttacagacgattaactatg | 196600 | - | see also 15874 line |
| 36111 | IMPA2 | NM_014214.1 | 1040 | gccttacagacgattaactatgg | 196601 | - | see also 15874 line |
| 36112 | IMPA2 | NM_014214.1 | 553 | cccgactgtggcggttagcattg | 196592 | - | see also 15874 line |
| 36113 | INPP1 | NM_002194.2 | 1087 | gccgtaattagtacaagtgaaaa | 196625 | - | see also 15875 line |
| 36114 | INPP1 | NM_002194.2 | 1138 | gtgtgtggagatcgcatatttgg | 196628 | - | see also 15875 line |
| 36115 | INPP4B | NM_003866.1 | 2605 | aacgatttgccgcaaactgaatg | 196704 | - | see also 15876 line |
| 36116 | INPP4B | NM_003866.1 | 2611 | ttgccgcaaactgaatggtattc | 196706 | - | see also 15876 line |

Figure 46

| 36117 | BPNT1 | NM_006085.3 | 468 | atcgcagtacagtgctattaaag | 196721 | - | see also 15877 line |
|---|---|---|---|---|---|---|---|
| 36118 | BPNT1 | NM_006085.3 | 196 | ttggtagcctccgcatattctat | 196716 | - | see also 15877 line |
| 36119 | FLJ20421 | NM_017813.1 | 659 | cccagcgtccagattaatactga | 196737 | - | see also 15878 line |
| 36120 | FLJ20421 | NM_017813.1 | 841 | tcgaaagtacgtcactactatgg | 196748 | - | see also 15878 line |
| 36121 | INPPL1 | NM_001567.2 | 1699 | cccgattgccatgcaatcactgt | 196772 | - | see also 15879 line |
| 36122 | INPPL1 | NM_001567.2 | 2626 | accaattctggctgatatcgagt | 196790 | - | see also 15879 line |
| 36123 | INPPL1 | NM_001567.2 | 1909 | gacggctcggaggaaccaaaact | 196776 | - | see also 15879 line |
| 36124 | MINPP1 | NM_004897.2 | 644 | gccggacgtcgcagatatggagt | 196802 | - | see also 15880 line |
| 36125 | MINPP1 | NM_004897.2 | 140 | ctcgtcgctcagcccctatttcg | 196798 | - | see also 15880 line |
| 36126 | ACP2 | NM_001610.1 | 112 | cccggagtctgcgcttcgttacc | 196821 | - | see also 15881 line |
| 36127 | ACP2 | NM_001610.1 | 277 | agcgctatcacggcttcctaaac | 196827 | - | see also 15881 line |
| 36128 | ACP5 | NM_001611.2 | 402 | tacgtgctagccggaaaccatga | 196844 | - | see also 15882 line |
| 36129 | ACP5 | NM_001611.2 | 1016 | ctcgggcaagtccctctttaaga | 196850 | - | see also 15882 line |
| 36130 | ACP6 | NM_016361.2 | 732 | agctggtggtccgaaaccatatt | 196856 | - | see also 15883 line |
| 36131 | ACP6 | NM_016361.2 | 756 | tccttacgactctcaataccatg | 196859 | - | see also 15883 line |
| 36132 | FLJ20331 | NM_017768.3 | 641 | tccagtctggtgcgactcaatct | 196883 | - | see also 7149 line |
| 36133 | FLJ20331 | NM_017768.3 | 647 | ctggtgcgactcaatctttctag | 196886 | - | see also 7149 line |
| 36134 | FLJ23751 | NM_152282.1 | 1367 | aacattccgtccggattctttac | 196963 | - | see also 15886 line |
| 36135 | FLJ23751 | NM_152282.1 | 901 | cagtacctcctacgtttgaaaaa | 196954 | - | see also 15886 line |
| 36136 | KIAA0377 | NM_014659.1 | 2612 | ttcgaacgcgtctctatttcacc | 196996 | - | see also 5829 line |
| 36137 | KIAA0377 | NM_014659.1 | 2608 | cacgttcgaacgcgtctctattt | 196994 | - | see also 5829 line |
| 36138 | KIAA0433 | NM_015216.1 | 2950 | aacgtcatgttcgtactagatta | 197072 | - | see also 6279 line |
| 36139 | KIAA0433 | NM_015216.1 | 3980 | tccacggaagaccgctgaaattt | 197113 | - | see also 6279 line |
| 36140 | KIAA0433 | NM_015216.1 | 3751 | atgctcgtactcatcgtaaaaag | 197110 | - | see also 6279 line |
| 36141 | FBP1 | NM_000507.2 | 1039 | gagactgctgtacgaatgcaacc | 197131 | - | see also 15889 line |
| 36142 | FBP1 | NM_000507.2 | 404 | aacgtgacaggtgatcaagttaa | 197119 | - | see also 15889 line |
| 36143 | FBP2 | NM_003837.2 | 320 | atggtccaatcctcctatagtac | 197133 | - | see also 15890 line |
| 36144 | FBP2 | NM_003837.2 | 705 | atgagggctatgccaagtatttt | 197139 | - | see also 15890 line |
| 36145 | G6PC | NM_000151.1 | 959 | ttccgcctcagctctattgtagc | 197161 | - | see also 15891 line |
| 36146 | G6PC | NM_000151.1 | 730 | cagcttcgccatcggattttatc | 197158 | - | see also 15891 line |
| 36147 | G6PC | NM_000151.1 | 731 | agcttcgccatcggattttatct | 197159 | - | see also 15891 line |
| 36148 | NT5C1A | NM_032526.1 | 809 | ggctggagtgcccaattcgtacc | 197176 | - | see also 15892 line |
| 36149 | NT5C1A | NM_032526.1 | 404 | tccgcctcatcaacagtatcaac | 197170 | - | see also 15892 line |
| 36150 | NT5C1B | NM_033253.1 | 945 | atgctagactccgtgatctatat | 197183 | - | see also 15893 line |
| 36151 | NT5C1B | NM_033253.1 | 715 | gaggtcgttctacgagaagaacc | 197178 | - | see also 15893 line |
| 36152 | NT5C2 | NM_012229.2 | 234 | agcctatcatcgggtgtttgtga | 197205 | - | see also 15894 line |
| 36153 | NT5C2 | NM_012229.2 | 1709 | accagctgacacggtcaattagt | 197245 | - | see also 15894 line |

Figure 46

| 36154 | NT5E | NM_002526.1 | 560 | gtgggaatcgttggatacacttc | 197259 | - | see also 1628 line |
|-------|------|-------------|-----|------------------------|--------|---|-------------------|
| 36155 | NT5E | NM_002526.1 | 1313 | gacctagtccagttaaaaggttc | 197284 | - | see also 1628 line |
| 36156 | NT5M | NM_020201.2 | 563 | aggagatggccagcctacaaaac | 197297 | - | see also 15896 line |
| 36157 | NT5M | NM_020201.2 | 608 | gccccatcaagatgttcaagtac | 197299 | - | see also 15896 line |
| 36158 | NT5M | NM_020201.2 | 568 | atggccagcctacaaaacactga | 197298 | - | see also 15896 line |
| 36159 | GPLD1 | NM_001503.2 | 236 | ttcacaatgggcgtgttaactac | 197303 | - | see also 1032 line |
| 36160 | GPLD1 | NM_001503.2 | 579 | ggctcattcggctggtgattttg | 197314 | - | see also 1032 line |
| 36161 | PLD1 | NM_002662.1 | 2385 | gccgcttacgtccatgtgataga | 197437 | - | see also 15898 line |
| 36162 | PLD1 | NM_002662.1 | 2384 | cgccgcttacgtccatgtgatag | 197436 | - | see also 15898 line |
| 36163 | PLD2 | NM_002663.2 | 917 | ggcacggcgtgcggatcgatacc | 197478 | - | see also 1743 line |
| 36164 | PLD2 | NM_002663.2 | 2638 | gagagcaacgccaatatctatga | 197507 | - | see also 1743 line |
| 36165 | PLD3 | NM_012268.1 | 1050 | ctcgagacctgaccaagatcttt | 197514 | - | see also 5246 line |
| 36166 | BPGM | NM_001724.3 | 652 | gccacggtcggaaagcttaaagg | 197535 | - | see also 15901 line |
| 36167 | BPGM | NM_001724.3 | 605 | tacaacgaccggaggtataaagt | 197530 | - | see also 15901 line |
| 36168 | PGAM2 | NM_000290.1 | 668 | cacggggatccccattgtgtatg | 197552 | - | see also 15902 line |
| 36169 | PGAM2 | NM_000290.1 | 415 | acgagaagcacccctactacaac | 197551 | - | see also 15902 line |
| 36170 | PON1 | NM_000446.3 | 741 | ttcacccgatggcaagtatgtct | 197580 | - | see also 351 line |
| 36171 | PON1 | NM_000446.3 | 660 | gtggtcgtatgttgtctactata | 197577 | - | see also 351 line |
| 36172 | PON2 | NM_000305.1 | 903 | ttcgtgtatgacccgaacaatcc | 197609 | - | see also 15904 line |
| 36173 | PON2 | NM_000305.1 | 705 | atcaatatttcacctgatgataa | 197604 | - | see also 15904 line |
| 36174 | PON3 | NM_000940.1 | 589 | ttggatcttcgctggacttatgt | 197628 | - | see also 15905 line |
| 36175 | PON3 | NM_000940.1 | 58 | atgttcctggcgtttagagaaag | 197615 | - | see also 15905 line |
| 36176 | PHP14 | NM_014172.2 | 378 | ctccgacggcgtcttcaagtatg | 197643 | - | see also 15906 line |
| 36177 | PHP14 | NM_014172.2 | 379 | tccgacggcgtcttcaagtatgt | 197644 | - | see also 15906 line |
| 36178 | PDE6A | NM_000440.1 | 2235 | gacacggaaggaaatcgttatgg | 197685 | - | see also 348 line |
| 36179 | PDE6A | NM_000440.1 | 289 | gagagcgaaatcatctttgatct | 197652 | - | see also 348 line |
| 36180 | PDE6B | NM_000283.1 | 1102 | tccgctgacgaaatgttcaaatt | 197702 | - | see also 15908 line |
| 36181 | PDE6B | NM_000283.1 | 1101 | ttccgctgacgaaatgttcaaat | 197701 | - | see also 15908 line |
| 36182 | PDE6C | NM_006204.2 | 928 | gcgctctacacggttagatcata | 197730 | - | see also 4302 line |
| 36183 | PDE6C | NM_006204.2 | 926 | aagcgctctacacggttagatca | 197729 | - | see also 4302 line |
| 36184 | PDE6G | NM_002602.1 | 257 | gacgacatccctggaatggaagg | 197778 | - | see also 15910 line |
| 36185 | PDE6H | NM_006205.1 | 174 | gactcgccaattcaagagtaaac | 197782 | - | see also 15911 line |
| 36186 | PDE6H | NM_006205.1 | 170 | ggcagactcgccaattcaagagt | 197781 | - | see also 15911 line |
| 36187 | PDE4A | NM_006202.1 | 1974 | ctcgtcccgggaggaattcgtgg | 197809 | - | see also 15912 line |
| 36188 | PDE4A | NM_006202.1 | 1445 | agcgcgagcgtggcatggaaatc | 197799 | - | see also 15912 line |
| 36189 | PDE4B | NM_002600.2 | 1214 | ctcacaatagacccctaacatgc | 197836 | - | see also 1680 line |
| 36190 | PDE4B | NM_002600.2 | 378 | aacgcttccaagcattgctatta | 197818 | - | see also 1680 line |

Figure 46

| | | | | | | | |
|---|---|---|---|---|---|---|---|
| 36191 | PDE4C | NM_000923.1 | 1443 | ctcagacgtggcgcttatgtaca | 197862 | - | see also 15914 line |
| 36192 | PDE4C | NM_000923.1 | 1521 | ggcagagaactgcgatatcttcc | 197865 | - | see also 15914 line |
| 36193 | PDE4D | NM_006203.2 | 463 | aagatcgagcacctagcaaaaga | 197878 | - | see also 4299 line |
| 36194 | PDE4D | NM_006203.2 | 1425 | gacatcgtacttgcaacagatat | 197899 | - | see also 4299 line |
| 36195 | PDE7A | NM_002603.1 | 418 | ttccagcgatatcttagatcttc | 197928 | - | see also 1682 line |
| 36196 | PDE7A | NM_002603.1 | 1282 | tgcgatcgtcacactgaatctat | 197964 | - | see also 1682 line |
| 36197 | PDE7A | NM_002603.1 | 1283 | gcgatcgtcacactgaatctatt | 197965 | - | see also 1682 line |
| 36198 | PDE1C | NM_005020.1 | 1573 | ggcgttcgagtttgaatagcatc | 198014 | - | see also 3511 line |
| 36199 | PDE1C | NM_005020.1 | 1643 | gggaagtgccccgatcaacaatt | 198017 | - | see also 3511 line |
| 36200 | PDE3A | NM_000921.2 | 2556 | tgctcctcaggcggtgctatata | 198074 | - | see also 599 line |
| 36201 | PDE3A | NM_000921.2 | 535 | ggcgtcggggaggatcacttact | 198028 | - | see also 599 line |
| 36202 | PDE2A | NM_002599.1 | 2518 | tgggctacgaccgaaacaacaag | 198106 | - | see also 15919 line |
| 36203 | PDE2A | NM_002599.1 | 1094 | cacctccgaggatgtacaacagc | 198094 | - | see also 15919 line |
| 36204 | PDE11A | NM_016953.2 | 2722 | aaccatcgtgatatatttcgatc | 198160 | - | see also 15920 line |
| 36205 | PDE11A | NM_016953.2 | 2338 | cagctgatgttcgcgatgttaac | 198145 | - | see also 15920 line |
| 36206 | PDE6D | NM_002601.1 | 519 | aactgggaacgttatcatagaaa | 198178 | - | see also 15921 line |
| 36207 | PDE6D | NM_002601.1 | 181 | atcctgaggggcttcaaactaaa | 198171 | - | see also 15921 line |
| 36208 | CNP | NM_033133.3 | 941 | tacgctcaacaagatgtgttaaa | 198186 | - | see also 15922 line |
| 36209 | CNP | NM_033133.3 | 1168 | gacgggccttgacctcttagaga | 198187 | - | see also 15922 line |
| 36210 | PLCB1 | NM_015192.2 | 3290 | cagagatgatccggtcatatatc | 198263 | - | see also 6273 line |
| 36211 | PLCB2 | NM_004573.1 | 2244 | agcgtgcgcacctatgtagaagt | 198296 | - | see also 15924 line |
| 36212 | PLCB2 | NM_004573.1 | 549 | gacgtactggccctagtcaaaca | 198273 | - | see also 15924 line |
| 36213 | PLCB3 | NM_000932.1 | 1627 | ctgaaccgaggcccctatgttct | 198326 | - | see also 15925 line |
| 36214 | PLCB3 | NM_000932.1 | 2523 | caccgaagcctcggactacattc | 198330 | - | see also 15925 line |
| 36215 | PLCB4 | NM_000933.2 | 2015 | ggccgagtcgattccagtaatta | 198399 | - | see also 606 line |
| 36216 | PLCB4 | NM_000933.2 | 2349 | tccgaactcgcatggttatgaat | 198414 | - | see also 606 line |
| 36217 | PLCE1 | NM_016341.2 | 1194 | agcgatccactttgttagtcagg | 198477 | - | see also 6825 line |
| 36218 | PLCE1 | NM_016341.2 | 1190 | aaggagcgatccactttgttagt | 198476 | - | see also 6825 line |
| 36219 | PLCL1 | NM_006226.1 | 1230 | agcgttgaactcgatgtaagtga | 198643 | - | see also 15928 line |
| 36220 | PLCL1 | NM_006226.1 | 2182 | cagcggattgttcggaacaaaga | 198679 | - | see also 15928 line |
| 36221 | PLCL2 | NM_015184.2 | 520 | ttgtgcgttttccgtcatatatg | 198714 | - | see also 15929 line |
| 36222 | PLCL2 | NM_015184.2 | 1076 | tccatagacggggttcactaatta | 198726 | - | see also 15929 line |
| 36223 | FLJ11323 | NM_018390.1 | 794 | acctggtcgcctgtatcaagaac | 198783 | - | see also 15930 line |
| 36224 | FLJ11323 | NM_018390.1 | 530 | gccctgtcgtgctgaaatggtcc | 198780 | - | see also 15930 line |
| 36225 | FLJ31579 | NM_153268.1 | 1281 | cacaagtgtgcgcaaactaatcc | 198797 | - | see also 15931 line |
| 36226 | FLJ31579 | NM_153268.1 | 938 | atgccgaccaggagatctacttc | 198790 | - | see also 15931 line |
| 36227 | FLJ37451 | NM_182569.1 | 573 | cactcccattaacatcgatatca | 198814 | - | see also 15932 line |

Figure 46

| | | | | | | | |
|---|---|---|---|---|---|---|---|
| 36228 | FLJ37451 | NM_182569.1 | 141 | gtcgtccactgcggctttttacc | 198799 | - | see also 15932 line |
| 36229 | KIAA1434 | NM_019593.2 | 776 | atccttaataagcgacaatgagt | 198851 | - | see also 7721 line |
| 36230 | KIAA1434 | NM_019593.2 | 872 | gacgatggaaccagataacctgg | 198852 | - | see also 7721 line |
| 36231 | MGC4171 | NM_024307.1 | 559 | gccgccagtcgggcctaaacagg | 198905 | - | see also 15934 line |
| 36232 | MGC4171 | NM_024307.1 | 722 | acccatgagcgtagagatcaaag | 198908 | - | see also 15934 line |
| 36233 | MIR16 | NM_016641.3 | 933 | ctcgattggagcatgcataatat | 198945 | - | see also 6965 line |
| 36234 | MIR16 | NM_016641.3 | 489 | gtcttaatgcacgataacacagt | 198924 | - | see also 6965 line |
| 36235 | OBDPF | NM_017711.2 | 1250 | gacggtaccagcattagaagagc | 198969 | - | see also 7122 line |
| 36236 | PP1665 | NM_030792.4 | 847 | accagcgctcccatgatgatacc | 198982 | - | see also 15937 line |
| 36237 | PP1665 | NM_030792.4 | 846 | taccagcgctcccatgatgatac | 198981 | - | see also 15937 line |
| 36238 | SMPD3 | NM_018667.1 | 408 | ctcactcgccagggtcaacaacc | 198993 | - | see also 7539 line |
| 36239 | SMPD3 | NM_018667.1 | 829 | ggccagacgcccaaccataatca | 198996 | - | see also 7539 line |
| 36240 | TDP1 | NM_018319.2 | 869 | gacgtggactggctcgtaaaaca | 199014 | - | see also 7400 line |
| 36241 | TDP1 | NM_018319.2 | 873 | tggactggctcgtaaaacagtat | 199015 | - | see also 7400 line |
| 36242 | LENG5 | NM_024075.1 | 759 | cccacgagctgcgctacagtatc | 199058 | - | see also 15940 line |
| 36243 | LENG5 | NM_024075.1 | 717 | ggcgtgtccagtctaaagactgg | 199057 | - | see also 15940 line |
| 36244 | LENG5 | NM_024075.1 | 984 | ttctccgcagcctgatggtaagg | 199062 | - | see also 15940 line |
| 36245 | MGC2776 | NM_025265.2 | 1717 | ctgagtcgatgggtttcttcacg | 199103 | - | see also 8888 line |
| 36246 | MGC2776 | NM_025265.2 | 1328 | aggagcctttaacgatagtgaag | 199084 | - | see also 8888 line |
| 36247 | POP1 | NM_015029.1 | 1551 | cagttggggatcctcgaataaat | 199146 | - | see also 6198 line |
| 36248 | POP1 | NM_015029.1 | 584 | atgaaccggacgctagaatttaa | 199118 | - | see also 6198 line |
| 36249 | RNASEP1 | NM_006638.1 | 381 | ttcagaagaatcgacaatgatgt | 199181 | - | see also 15943 line |
| 36250 | RNASEP1 | NM_006638.1 | 8 | ctggaccctattactttgtgaag | 199174 | - | see also 15943 line |
| 36251 | RPP20 | NM_005837.1 | 363 | gtgctctgagatctacattcacg | 199195 | - | see also 15944 line |
| 36252 | RPP20 | NM_005837.1 | 474 | ctccaccgtggagcttgttgatg | 199197 | - | see also 15944 line |
| 36253 | RPP30 | NM_006413.2 | 891 | gccatatgacgtggcaaatctag | 199212 | - | see also 15945 line |
| 36254 | RPP30 | NM_006413.2 | 778 | gactccacaatgagaaggtatac | 199209 | - | see also 15945 line |
| 36255 | RPP38 | NM_006414.1 | 105 | gtcgttgaacaacccatacatca | 199216 | - | see also 15946 line |
| 36256 | RPP38 | NM_006414.1 | 542 | cccccgtcattggcttaaaatgt | 199228 | - | see also 15946 line |
| 36257 | DLAD | NM_021233.2 | 721 | cagtcggcccagggacaaaaatt | 199265 | - | see also 15947 line |
| 36258 | DLAD | NM_021233.2 | 486 | tccggaagaaggctatgattatc | 199260 | - | see also 15947 line |
| 36259 | APEX2 | NM_014481.2 | 567 | ggcctgagcggctagtctttaag | 199283 | - | see also 15948 line |
| 36260 | APEX2 | NM_014481.2 | 296 | cgcaaccgtagcggctattctgg | 199278 | - | see also 15948 line |
| 36261 | EloA-BP1 | NM_020695.2 | 3342 | cacggacgtgcacgtggtttatg | 199347 | - | see also 15949 line |
| 36262 | EloA-BP1 | NM_020695.2 | 550 | cccggcagccacggcctattaag | 199339 | - | see also 15949 line |
| 36263 | FLJ12484 | NM_022767.2 | 843 | gggcactcatcagtagaagatgc | 199353 | - | see also 15950 line |
| 36264 | FLJ12484 | NM_022767.2 | 616 | tggcccagaaagagatccttaag | 199349 | - | see also 15950 line |

Figure 46

| | | | | | | | |
|---|---|---|---|---|---|---|---|
| 36265 | FLJ12671 | NM_030980.1 | 402 | cctagcaaggcgcctaagttgc | 199358 | - | see also 15951 line |
| 36266 | FLJ12671 | NM_030980.1 | 855 | ggctcgatgtagcattgtcaact | 199366 | - | see also 15951 line |
| 36267 | ISG20 | NM_002201.4 | 764 | aacgatggagctctatcaaatct | 199379 | - | see also 15952 line |
| 36268 | ISG20 | NM_002201.4 | 761 | ggcaacgatggagctctatcaaa | 199378 | - | see also 15952 line |
| 36269 | MGC16943 | NM_080663.1 | 63 | cgcggcagcttggattaattagg | 199380 | - | see also 15953 line |
| 36270 | MGC16943 | NM_080663.1 | 89 | aagtcaattgcgccagcaaatgg | 199382 | - | see also 15953 line |
| 36271 | DFFA | NM_004401.1 | 289 | gtctaccttccaatactaagttt | 199409 | - | see also 15954 line |
| 36272 | DFFA | NM_004401.1 | 285 | ctgtgtcaccttccaatactaa | 199407 | - | see also 15954 line |
| 36273 | DFFB | NM_004402.1 | 287 | cacggagctgacgaagattact | 199422 | - | see also 15955 line |
| 36274 | DFFB | NM_004402.1 | 387 | aggcgcttcctcagtgcatttca | 199423 | - | see also 15955 line |
| 36275 | KIAA0218 | NM_014760.1 | 2586 | ttgcatacggtccgagagattgc | 199463 | - | see also 15956 line |
| 36276 | KIAA0218 | NM_014760.1 | 1635 | cggagccgcatgagtgattattc | 199447 | - | see also 15956 line |
| 36277 | PLA2G13 | NM_032562.1 | 397 | cacttgcggtgccaacaaatatc | 199473 | - | see also 15957 line |
| 36278 | PLA2G13 | NM_032562.1 | 171 | ccgtcaatagctacttcgattct | 199469 | - | see also 15957 line |
| 36279 | PLA2G4A | NM_024420.1 | 1805 | tgccgtatccttgatactgaga | 199523 | - | see also 8618 line |
| 36280 | PLA2G4A | NM_024420.1 | 1225 | atggctcccgacttatttggaag | 199503 | - | see also 8618 line |
| 36281 | PLA2G6 | NM_003560.1 | 1459 | cccacgatcagcctaaacaac | 199551 | - | see also 2367 line |
| 36282 | PLA2G6 | NM_003560.1 | 1458 | cccaccgatcagcctaaacaac | 199550 | - | see also 2367 line |
| 36283 | PLA2G6 | NM_003560.1 | 367 | ggctgacgccctagtgaatttcc | 199540 | - | see also 2367 line |
| 36284 | PRDX6 | NM_004905.2 | 352 | cccatcatcgatgatagagaatcg | 199566 | - | see also 15960 line |
| 36285 | PRDX6 | NM_004905.2 | 73 | ggggacgttgctcccaactttga | 199560 | - | see also 15960 line |
| 36286 | CLC | NM_001828.4 | 121 | tactggttctactgtgacaatca | 199570 | - | see also 15961 line |
| 36287 | CLC | NM_001828.4 | 124 | tggttctactgtgacaatcaaag | 199572 | - | see also 15961 line |
| 36288 | LYPLA1 | NM_006330.2 | 658 | gtcctatcggtggtgctaataga | 199588 | - | see also 15962 line |
| 36289 | LYPLA1 | NM_006330.2 | 663 | atcggtggtgctaatagagatat | 199589 | - | see also 15962 line |
| 36290 | LYPLA1 | NM_006330.2 | 737 | tggttctttacggtggaaaaac | 199593 | - | see also 15962 line |
| 36291 | MGLL | NM_007283.3 | 1257 | ctctgccgatcgcctatgtgaca | 199597 | - | - |
| 36292 | PLA1A | NM_015900.1 | 354 | ttctgcgtgcaacgaatgctaat | 199604 | - | see also 15963 line |
| 36293 | PLA1A | NM_015900.1 | 358 | gcgtgcaacgaatgctaatgtga | 199606 | - | see also 15963 line |
| 36294 | cPLA2delta | NM_178034.1 | 361 | agctgcaacaggaactacgtagc | 199623 | - | - |
| 36295 | cPLA2delta | NM_178034.1 | 103 | aagccatcggagcaacatttc | 199622 | - | see also 36294 line |
| 36296 | PLA2G4B | NM_005090.1 | 155 | gccccaacaggccgtgcattatc | 199626 | - | see also 15964 line |
| 36297 | PLA2G4B | NM_005090.1 | 1682 | tggattgcgtctctacatcacc | 199635 | - | see also 15964 line |
| 36298 | PLA2G4C | NM_003706.1 | 1377 | atggggaccactcacaacttcc | 199663 | - | see also 15965 line |
| 36299 | PLA2G4C | NM_003706.1 | 1413 | tgggcatccgggacaagataatga | 199665 | - | see also 15965 line |

Figure 46

| 36300 | C11orf11 | NM_006133.1 | 1864 | caccccagcgacctaactatagc | 199674 | - | see also 15966 line |
|---|---|---|---|---|---|---|---|
| 36301 | C11orf11 | NM_006133.1 | 1227 | ctgccatgatgcggtctatgaaa | 199671 | - | see also 15966 line |
| 36302 | LOC221955 | NM_139179.1 | 734 | gaccatactcgggttgctttttc | 199700 | - | see also 15967 line |
| 36303 | LOC221955 | NM_139179.1 | 735 | accatactcgggttgctttttcg | 199701 | - | see also 15967 line |
| 36304 | PNLIP | NM_000936.1 | 388 | ggctcccgaactggatacacaca | 199725 | - | see also 15968 line |
| 36305 | PNLIP | NM_000936.1 | 1303 | gtgggagcatccaagattatagt | 199747 | - | see also 15968 line |
| 36306 | PNLIPRP1 | NM_006229.1 | 1124 | gccactggtcagatcaaagttgc | 199764 | - | see also 15969 line |
| 36307 | PNLIPRP1 | NM_006229.1 | 473 | gacatcctcttgacagagtatag | 199757 | - | see also 15969 line |
| 36308 | PNLIPRP2 | NM_005396.3 | 431 | gggcggagacagctttcttaata | 199778 | - | see also 3766 line |
| 36309 | PNLIPRP2 | NM_005396.3 | 909 | ctgtgcctcctacgatgagtttc | 199786 | - | see also 3766 line |
| 36310 | AOAH | NM_001637.1 | 1355 | gactatcccgccatcgttatata | 199827 | - | see also 1083 line |
| 36311 | AOAH | NM_001637.1 | 1950 | atccgttcaaccccccagattaaa | 199838 | - | see also 1083 line |
| 36312 | APOC2 | NM_000483.3 | 295 | gcccgctgtagatgagaaactca | 199840 | - | see also 15972 line |
| 36313 | LIPA | NM_000235.1 | 1088 | gtctacgacgtcaatatcttact | 199866 | - | see also 15973 line |
| 36314 | LIPA | NM_000235.1 | 1083 | cagatgtctacgacgtcaatatc | 199864 | - | see also 15973 line |
| 36315 | ACHE | NM_000665.2 | 1181 | aaggatgagggctcgtattttct | 199877 | - | see also 15974 line |
| 36316 | ACHE | NM_000665.2 | 383 | cagagtgtctgctaccaatatgt | 199872 | - | see also 15974 line |
| 36317 | BCHE | NM_000055.1 | 1114 | tccgaccgtggatggtgattttc | 199920 | - | see also 29 line |
| 36318 | BCHE | NM_000055.1 | 639 | tggctcgggttgaaagagttatt | 199899 | - | see also 29 line |
| 36319 | CACH-1 | NM_130767.1 | 850 | gccgagggcgtcacatcaacagt | 199965 | - | see also 9547 line |
| 36320 | CACH-1 | NM_130767.1 | 1234 | atcgtctcttgtctgactttaca | 199973 | - | see also 9547 line |
| 36321 | CEL | NM_001807.2 | 369 | cccgggacctgcccgttatgatc | 199989 | - | see also 15977 line |
| 36322 | CEL | NM_001807.2 | 550 | cgccaatctgccaggtaactatg | 199992 | - | see also 15977 line |
| 36323 | CES1 | NM_001266.3 | 1284 | cagacgacactgtcaaaaagaaa | 200002 | - | see also 15978 line |
| 36324 | CES1 | NM_001266.3 | 1222 | tccccttgtttgcattgctaagg | 200001 | - | see also 15978 line |
| 36325 | CES2 | NM_003869.4 | 1318 | ggggagtcttgtccatgtgaagg | 200004 | - | see also 15979 line |
| 36326 | CES2 | NM_003869.4 | 2425 | ggcggactccatgtttgtgatcc | 200020 | - | see also 15979 line |
| 36327 | ESD | NM_001984.1 | 433 | agccctcgtggctgcaatattaa | 200031 | - | see also 15980 line |
| 36328 | ESD | NM_001984.1 | 434 | gccctcgtggctgcaatattaaa | 200032 | - | see also 15980 line |
| 36329 | NTE | NM_006702.2 | 2534 | gtccgacgctactccttaacagt | 200057 | - | see also 15981 line |
| 36330 | NTE | NM_006702.2 | 2084 | accgctccgactgcacttacatc | 200052 | - | see also 15981 line |
| 36331 | NTE | NM_006702.2 | 2643 | tgcacaccgtatcgtactctacc | 200061 | - | see also 15981 line |
| 36332 | PTE1 | NM_005469.2 | 547 | accctcattgaccagtatttaag | 200080 | - | see also 15982 line |
| 36333 | PTE1 | NM_005469.2 | 678 | gagaatggagcccaaacagatgt | 200083 | - | see also 15982 line |
| 36334 | THEA | NM_015547.2 | 1155 | tccgcctggacaggaagtacatc | 200089 | - | see also 6461 line |

Figure 46

| | | | | | | | | |
|---|---|---|---|---|---|---|---|---|
| 36335 | ZAP128 | NM_006821.3 | 1127 | gccaatgttgggggaacctacg | | 200105 | - | see also 15984 line |
| 36336 | ZAP128 | NM_006821.3 | 1342 | gagtgagttctatgctaatgagg | | 200110 | - | see also 15984 line |
| 36337 | PAFAH1B1 | NM_000430.2 | 1637 | ccctacgcgtatgggattacaag | | 200143 | - | see also 336 line |
| 36338 | PAFAH1B1 | NM_000430.2 | 1640 | tacgcgtatgggattacaagaac | | 200144 | - | see also 336 line |
| 36339 | PAFAH1B2 | NM_002572.1 | 454 | atcgaggccattgtacaacttat | | 200156 | - | see also 1654 line |
| 36340 | PAFAH1B2 | NM_002572.1 | 450 | tgggatcgagccattgtacaac | | 200154 | - | see also 1654 line |
| 36341 | H6PD | NM_004285.1 | 1592 | cagccctgtccgattactacg | | 200187 | - | see also 15988 line |
| 36342 | H6PD | NM_004285.1 | 1369 | tgggctacgctccgatcttgttc | | 200185 | - | see also 15988 line |
| 36343 | PGLS | NM_012088.1 | 644 | agctgttctgaagcgccattttgg | | 200196 | - | see also 15989 line |
| 36344 | HIBCH | NM_014362.2 | 108 | ctcattcgaagtttaatgcatt | | 200200 | - | see also 15990 line |
| 36345 | HIBCH | NM_014362.2 | 216 | tgcacgggagtcataacactaaa | | 200205 | - | see also 15990 line |
| 36346 | PPT1 | NM_000310.1 | 549 | gcctcgtgcaagccgaatactgg | | 200246 | - | see also 15991 line |
| 36347 | PPT1 | NM_000310.1 | 523 | gggtactccaaagttgttcagg | | 200245 | - | see also 15991 line |
| 36348 | PPT1 | NM_000310.1 | 403 | acctcccatgatcaatctgatct | | 200241 | - | see also 15991 line |
| 36349 | PPT2 | NM_005155.5 | 1047 | acctctatcggatctgctatagc | | 200257 | - | see also 3622 line |
| 36350 | PPT2 | NM_005155.5 | 999 | gagacacggacgtacttgaagtgg | | 200256 | - | see also 3622 line |
| 36351 | HAGH | NM_005326.3 | 541 | ccctgaccgacaactacatgtac | | 200266 | - | see also 15993 line |
| 36352 | HAGH | NM_005326.3 | 539 | tgccctgaccgacgaactacatgt | | 200265 | - | see also 15993 line |
| 36353 | ARSA | | 1516 | gacgaggtccgtcgggttttgc | cerebroside-sulfatase; cerebral sclerosis, metachromatic leukoencephalopathy, sulfatide lipidosis, metachromatic leukodystrophy | 200275 | - | - |
| 36354 | ARSA | NM_000487.3 | 1536 | tgctgtccggactggaagtaca | | 200276 | - | see also 36353 line |
| 36355 | ARSB | NM_000046.2 | 1593 | taccagatccgtacagtttaca | | 200278 | - | see also 15994 line |
| 36356 | ARSB | NM_000046.2 | 1814 | ttcccatgaacgctgtacattaa | | 200283 | - | see also 15994 line |
| 36357 | ARSD | NM_001669.1 | 161 | aagacgtgtgaacctaaaactgc | | 200309 | - | see also 15995 line |
| 36358 | ARSD | NM_001669.1 | 262 | tacactgagaacgccgaatattg | | 200316 | - | see also 15995 line |
| 36359 | ARSE | NM_000047.1 | 1127 | cacggcgttcctagagaatca | | 200356 | - | see also 15996 line |
| 36360 | ARSE | NM_000047.1 | 120 | tgctcgctactgctaagtttg | | 200339 | - | see also 15996 line |
| 36361 | ARSF | NM_004042.3 | 618 | ccctccgtaacacggaattagc | | 200378 | - | see also 15997 line |
| 36362 | ARSF | NM_004042.3 | 338 | cgcgttcttgacgggaagataac | | 200366 | - | see also 15997 line |
| 36363 | ARSF | NM_004042.3 | 868 | ctgaacgagctgatccattatg | | 200380 | - | see also 15997 line |
| 36364 | GALNS | NM_000512.2 | 821 | gacgccgtccgggagattgatga | | 200411 | - | see also 15998 line |
| 36365 | GALNS | NM_000512.2 | 214 | cagagagaccccgaatttggacc | | 200403 | - | see also 15998 line |

Figure 46

| | | | | | | | |
|---|---|---|---|---|---|---|---|
| 36366 | STS | NM_000351.2 | 1519 | aagccaacgtccgatcatgagt | 200446 | - | see also 15999 line |
| 36367 | STS | NM_000351.2 | 289 | agcatcaaggccgaacatcatcc | 200417 | - | see also 15999 line |
| 36368 | IDS | NM_000202.2 | 990 | tcctggccgttggtatcataag | 200476 | - | see also 160 line |
| 36369 | IDS | NM_000202.2 | 779 | ccgtatagctggtcttttccacc | 200469 | - | see also 160 line |
| 36370 | GNS | NM_002076.1 | 1230 | tgctggctacgacctaaataaga | 200528 | - | see also 1340 line |
| 36371 | GNS | NM_002076.1 | 1234 | ggctacgacctaaataagacaca | 200530 | - | see also 1340 line |
| 36372 | DKFZp313 G1735 | NM_198150.1 | 239 | tccttcgatggaaggttaacatt | 200548 | | see also 16002 line |
| 36373 | DKFZp313 G1735 | NM_198150.1 | 1119 | cacatgttccgctttgatgatg | 200581 | - | see also 16002 line |
| 36374 | KIAA1001 | NM_014960.1 | 1050 | gacgtggcctcctctctttatga | 200605 | - | see also 16003 line |
| 36375 | KIAA1001 | NM_014960.1 | 1916 | tgcagacgtcctccaagacattg | 200613 | - | see also 16003 line |
| 36376 | SGSH | NM_000199.2 | 399 | gaaccgtgtacccgtttgactttg | 200616 | - | see also 159 line |
| 36377 | SULF1 | NM_015170.1 | 1336 | tccccataggccgttatgatgg | 200641 | - | see also 6263 line |
| 36378 | SULF1 | NM_015170.1 | 2338 | ttccttgtccgtcgaatttgaag | 200664 | - | see also 6263 line |
| 36379 | SULF1 | NM_015170.1 | 1335 | atcccccataggcccgttatgatg | 200640 | - | see also 6263 line |
| 36380 | SULF2 | NM_018837.2 | 1877 | ccggacgccggaaaaaactcttc | 200705 | - | see also 16006 line |
| 36381 | SULF2 | NM_018837.2 | 1511 | ggccggtgaatcgtttcacttg | 200699 | - | see also 16006 line |
| 36382 | C14orf126 | NM_080664.1 | 501 | gacaccaacggaccattcacaca | 200725 | - | see also 16007 line |
| 36383 | C14orf126 | NM_080664.1 | 351 | cactctaactctggaaagaaga | 200722 | - | see also 16007 line |
| 36384 | FLJ11106 | NM_018324.1 | 1071 | atcctgaacgagagaaattaatc | 200760 | - | see also 16008 line |
| 36385 | FLJ11106 | NM_018324.1 | 916 | caactctaacgtaccatcctaagg | 200753 | - | see also 16008 line |
| 36386 | HARS2 | NM_080820.3 | 486 | aacatacaggccggagcttatca | 200775 | - | see also 16009 line |
| 36387 | HARS2 | NM_080820.3 | 271 | cacatggtcgaaagattctaaa | 200769 | - | see also 16009 line |
| 36388 | HARS2 | NM_080820.3 | 473 | agcagctgcgtaaaacatacagg | 200774 | - | see also 16009 line |
| 36389 | HYAL2 | NM_003773.2 | 357 | gcctgtatccacgcttcgattct | 200785 | - | see also 16010 line |
| 36390 | HYAL2 | NM_003773.2 | 891 | gccatggcgcaactttgtgagc | 200790 | - | see also 16010 line |
| 36391 | HYAL3 | NM_003549.2 | 505 | ctcggcctcatcctactttgg | 200795 | - | see also 16011 line |
| 36392 | HYAL3 | NM_003549.2 | 766 | cagtctacaaggcctatactgg | 200796 | - | see also 16011 line |
| 36393 | MGEA5 | NM_012215.1 | 1673 | aaccgtagtaacaacagtttatc | 200839 | - | see also 5203 line |
| 36394 | MGEA5 | NM_012215.1 | 848 | atccacattgaaagctaaattgg | 200811 | - | see also 5203 line |
| 36395 | SPAM1 | NM_003117.3 | 646 | accgggcaaggttacaatatt | 200876 | - | see also 16012 line |
| 36396 | SPAM1 | NM_003117.3 | 645 | caccgggcaaggttacaatat | 200875 | - | see also 16012 line |
| 36397 | HEXA | NM_000520.2 | 224 | gcgctatcgtgacctgctttcg | 200913 | - | see also 16013 line |
| 36398 | HEXA | NM_000520.2 | 433 | tccgaggtctggagactttagc | 200916 | - | see also 16013 line |
| 36399 | HEXB | NM_000521.2 | 358 | gaggaagcgtttgacgcgatatca | 200937 | - | see also 16014 line |
| 36400 | HEXB | NM_000521.2 | 1324 | ccgggcaacaatagttgaagtatg | 200964 | - | see also 16014 line |

Figure 46

| 36401 | NAGPA | NM_016256.1 | 604 | gtcgtgtggctgattcgtaatgg | 200977 | - | see also 16015 line |
|---|---|---|---|---|---|---|---|
| 36402 | NAGPA | NM_016256.1 | 607 | gtgtggctgattcgtaatggaag | 200978 | - | see also 16015 line |
| 36403 | NAGLU | NM_000263.2 | 1925 | atctggtacaaccgatctgatgt | 200995 | - | see also 206 line |
| 36404 | NAGLU | NM_000263.2 | 1270 | ggccgacactttcaatgagatgc | 200987 | - | see also 206 line |
| 36405 | NAGA | NM_000262.1 | 669 | ggcgggacatgggctacacatac | 201002 | - | see also 16017 line |
| 36406 | NAGA | NM_000262.1 | 670 | gcgggacatgggctacacatacc | 201003 | - | see also 16017 line |
| 36407 | MAN2A1 | NM_002372.1 | 1705 | agggctcggatgctactagatca | 201052 | - | see also 1525 line |
| 36408 | MAN2A1 | NM_002372.1 | 760 | gaggatggtccgaaaagttcaca | 201023 | - | see also 1525 line |
| 36409 | MAN2A1 | NM_002372.1 | 1707 | ggctcggatgctactagatcagt | 201053 | - | see also 1525 line |
| 36410 | MAN2A2 | NM_006122.1 | 1556 | tgcggaccgggaggatcattact | 201150 | - | see also 16019 line |
| 36411 | MAN2A2 | NM_006122.1 | 1555 | atgcggaccgggaggatcattac | 201149 | - | see also 16019 line |
| 36412 | MAN2B1 | NM_000528.1 | 2137 | cacatccgggcaacgtttgatcc | 201194 | - | see also 16020 line |
| 36413 | MAN2B1 | NM_000528.1 | 2230 | ttctggtacaacgccagtatagg | 201199 | - | see also 16020 line |
| 36414 | MAN2C1 | NM_006715.1 | 2557 | agcgacctacccactacaatacc | 201229 | - | see also 16021 line |
| 36415 | MAN2C1 | NM_006715.1 | 1016 | tgcgtgccgtgggcagtttgtgc | 201219 | - | see also 16021 line |
| 36416 | MANBA | NM_005908.2 | 1768 | tggccgtccttcagtacattaga | 201282 | - | see also 16022 line |
| 36417 | MANBA | NM_005908.2 | 1503 | cactgaccggccaatctacatca | 201272 | - | see also 16022 line |
| 36418 | GAA | NM_000152.2 | 1191 | ctcgctgccctcgcagtatatca | 201309 | - | see also 16023 line |
| 36419 | GAA | NM_000152.2 | 1862 | agccgctgattgggaaggtatgg | 201316 | - | see also 16023 line |
| 36420 | MGAM | NM_004668.1 | 1195 | cacctcagtcgttacgaatatgg | 201359 | - | see also 16024 line |
| 36421 | MGAM | NM_004668.1 | 1193 | ttcacctcagtcgttacgaatat | 201358 | - | see also 16024 line |
| 36422 | SI | NM_001041.1 | 529 | atcgtttccggttcaagattact | 201448 | - | see also 16025 line |
| 36423 | SI | NM_001041.1 | 3373 | ctcgcctgccatcagaatatata | 201543 | - | see also 16025 line |
| 36424 | SI | NM_001041.1 | 5023 | ctcggtggtttgactaccataca | 201592 | - | see also 16025 line |
| 36425 | GCS1 | NM_006302.1 | 959 | tggtaaagagtcgcctaaatagc | 201620 | - | see also 16026 line |
| 36426 | GCS1 | NM_006302.1 | 270 | ggccgtcgtggtcctgtctttgg | 201616 | - | see also 16026 line |
| 36427 | AGL | NM_000028.1 | 3465 | agcagatcccacgttaccttatc | 201733 | - | see also 16027 line |
| 36428 | AGL | NM_000028.1 | 575 | ttccgattaggcccaactttaca | 201629 | - | see also 16027 line |
| 36429 | KIAA1128 | NM_018999.1 | 1616 | gaggctctccctatagagaatct | 201814 | - | see also 16028 line |
| 36430 | KIAA1128 | NM_018999.1 | 662 | atggcccaggtgtaacttctact | 201781 | - | see also 16028 line |
| 36431 | NEU1 | NM_000434.2 | 627 | tggcctctaccatgttggtatgg | 201830 | - | see also 16029 line |
| 36432 | NEU1 | NM_000434.2 | 408 | ttgctgaggcgaggaaaatgtcc | 201825 | - | see also 16029 line |
| 36433 | NEU2 | NM_005383.1 | 1055 | gtctgtacgaagccaatgattac | 201842 | - | see also 16030 line |
| 36434 | NEU2 | NM_005383.1 | 532 | gcctacgcctaccggaaacttca | 201840 | - | see also 16030 line |
| 36435 | NEU3 | NM_006656.4 | 913 | tccgggcttcggcgaatgagacc | 201848 | - | see also 16031 line |
| 36436 | NEU3 | NM_006656.4 | 1553 | tccctgcgtatacctactacatc | 201854 | - | see also 16031 line |
| 36437 | FLJ90231 | NM_173581.1 | 877 | gacgtaaccttgggcgatattgg | 201887 | - | see also 16032 line |

EP 1 752 536 A1

Figure 46

| 36438 | FLJ90231 | NM_173581.1 | 330 | gacgcctatcctacttaaatga | 201878 | - | see also 16032 line |
|---|---|---|---|---|---|---|---|
| 36439 | GLB1 | NM_000404.1 | 1375 | gtccacgatcgagcatatgttgc | 201917 | - | see also 16033 line |
| 36440 | GLB1 | NM_000404.1 | 1576 | ctcacgactgacgatctttcc | 201925 | - | see also 16033 line |
| 36441 | GLB1 | NM_000404.1 | 1514 | atggtgcatatacaacgatttt | 201922 | - | see also 16033 line |
| 36442 | LOC112937 | NM_138416.1 | 500 | cccagcctaggtttaattggtct | 201933 | - | see also 16034 line |
| 36443 | LOC112937 | NM_138416.1 | 494 | atccgtcccagcctaggtttaat | 201932 | - | see also 16034 line |
| 36444 | LOC89944 | NM_138342.2 | 1428 | gtccttcggtacattctctatg | 201950 | - | see also 16035 line |
| 36445 | LOC89944 | NM_138342.2 | 1648 | caggcaaaggcttaattggaaa | 201954 | - | see also 16035 line |
| 36446 | GLA | NM_000169.1 | 716 | tccgacagtactgcaatcactgg | 201975 | - | see also 141 line |
| 36447 | GLA | NM_000169.1 | 1136 | ttggtggacctgctcttatacc | 201987 | - | see also 141 line |
| 36448 | GBE1 | NM_000158.1 | 173 | tggagatcgaccgtacttgaag | 201993 | - | see also 133 line |
| 36449 | GBE1 | NM_000158.1 | 527 | gcggagaagatcttgtatcgtatt | 202009 | - | see also 133 line |
| 36450 | SLC3A1 | NM_000341.2 | 873 | ttgacgaagtgcgaaaccaatgt | 202083 | - | see also 16038 line |
| 36451 | SLC3A1 | NM_000341.2 | 935 | ttccgcaatcctgatgttcaaga | 202086 | - | see also 16038 line |
| 36452 | SLC3A2 | NM_002394.3 | 1336 | agcctactcgaatccaacaaaga | 202135 | - | see also 16039 line |
| 36453 | SLC3A2 | NM_002394.3 | 759 | tgctggtgccgtgtcataatcg | 202127 | - | see also 16039 line |
| 36454 | LOC129530 | NM_174898.1 | 375 | gactcacggcctgaactactgt | 202140 | - | see also 16040 line |
| 36455 | LOC129530 | NM_174898.1 | 601 | tcccacatcctggattagttgagt | 202144 | - | see also 16040 line |
| 36456 | LYG2 | NM_175735.3 | 674 | caccccatcgacatagacacaatg | 202160 | - | see also 16041 line |
| 36457 | LYG2 | NM_175735.3 | 675 | acccatcggacatagacacaatga | 202161 | - | see also 16041 line |
| 36458 | LYZ | NM_000239.1 | 260 | agccgctactggtgttaatgatgg | 202168 | - | see also 16042 line |
| 36459 | LYZ | NM_000239.1 | 431 | aacagagagtcgtcgtatgt | 202175 | - | see also 16042 line |
| 36460 | PARG | NM_003631.1 | 2955 | agctgttgctacgatactacaat | 202242 | - | see also 2442 line |
| 36461 | PARG | NM_003631.1 | 2957 | ctgttgctacgatactacaatga | 202243 | - | see also 2442 line |
| 36462 | GUSB | NM_000181.1 | 1378 | agcctgcgtcccacctagaatct | 202253 | - | see also 16044 line |
| 36463 | GUSB | NM_000181.1 | 1820 | gacgagagtgctgggaataaaa | 202258 | - | see also 16044 line |
| 36464 | HPSE | NM_006665.2 | 1396 | ggcaagcgtgcaaggttcaaga | 202296 | - | see also 4713 line |
| 36465 | HPSE | NM_006665.2 | 1526 | ttgcggttaccctatcctttc | 202306 | - | see also 4713 line |
| 36466 | IDUA | NM_000203.2 | 810 | gccacgacgtaccaacttcttc | 202317 | - | see also 16046 line |
| 36467 | IDUA | NM_000203.2 | 813 | acgacgtaccaacttctcact | 202318 | - | see also 16046 line |
| 36468 | GBA | NM_000157.1 | 1409 | acccaattggtgcgtaacttg | 202321 | - | see also 16047 line |
| 36469 | GBA | NM_000157.1 | 1410 | cccaattggtgcgtaacttgt | 202322 | - | see also 16047 line |
| 36470 | GBA | NM_000157.1 | 1431 | gtcgacagtcccatcattgtaga | 202323 | - | see also 16047 line |

# EP 1 752 536 A1

Figure 46

| | | | | | | |
|---|---|---|---|---|---|---|
| 36471 | GALC | NM_000153.1 | 758 | cgccaagcgttaccatgattgg | - | see also 16048 line |
| 36472 | GALC | NM_000153.1 | 696 | ttcgactggccttatgtcaatct | - | see also 16048 line |
| 36473 | FUCA1 | NM_000147.2 | 644 | agctgtacgaccttgttaacagc | - | see also 16049 line |
| 36474 | FUCA1 | NM_000147.2 | 1170 | ctcaaagggatcggctgtttatg | - | see also 16049 line |
| 36475 | TREH | NM_007180.1 | 1240 | gaccggagcctggttcgattacg | - | see also 16050 line |
| 36476 | TREH | NM_007180.1 | 550 | tgggcggctgctttgagttct | - | see also 16050 line |
| 36477 | LCT | NM_002299.2 | 3325 | atcacacgtacgatgaggaaatac | - | see also 16051 line |
| 36478 | LCT | NM_002299.2 | 3915 | gcctacagctcgatggtataga | - | see also 16051 line |
| 36479 | LCT | NM_002299.2 | 102 | accgctggtcctctaaccaatga | - | see also 16051 line |
| 36480 | CSMD1 | NM_033225.1 | 474 | caegggcgagcgcaataggatac | - | see also 9383 line |
| 36481 | CSMD1 | NM_033225.1 | 6953 | ccctcgaaacggcgtatagctcc | - | see also 9383 line |
| 36482 | G2AN | NM_014610.3 | 1457 | ttgagcggcttggcttcaagagg | - | see also 5777 line |
| 36483 | G2AN | NM_014610.3 | 325 | atgactcggttcaggattgatga | - | see also 5777 line |
| 36484 | GBA3 | NM_020973.2 | 1207 | cactcaaacgctgggagtatttca | - | see also 16054 line |
| 36485 | GBA3 | NM_020973.2 | 976 | tacaactcgcttaatcaagtacc | - | see also 16054 line |
| 36486 | KL | NM_004795.2 | 2071 | atgagccgtatacaaggaatatg | - | see also 16055 line |
| 36487 | KL | NM_004795.2 | 266 | tgcgtccatctgggatacgttca | - | see also 16055 line |
| 36488 | LOC152831 | NM_175737.2 | 1513 | caccatccgcgaggattatttt | - | see also 10149 line |
| 36489 | RIMS3 | NM_014747.1 | 1051 | caactatatcaagttacctgc | - | see also 16057 line |
| 36490 | RIMS3 | NM_014747.1 | 823 | ctccgacgcacgttcatcttcc | - | see also 16057 line |
| 36491 | UNG | NM_003362.2 | 411 | gagttcgggaaaccgtatttat | - | see also 16058 line |
| 36492 | UNG | NM_003362.2 | 585 | cacgggctctgctttagtgttca | - | see also 16058 line |
| 36493 | UNG2 | NM_021147.1 | 1007 | cagatccttcgtttccttttagt | - | uracil DNA N-glycosylase - |
| 36494 | BST1 | NM_004334.1 | 791 | atcgagatctgggttatgcatga | - | see also 16059 line |
| 36495 | BST1 | NM_004334.1 | 687 | tccacgtcatcgtgaatggttca | - | see also 16059 line |
| 36496 | BST1 | NM_004334.1 | 572 | gactctgactcgattaccaatc | - | see also 16059 line |
| 36497 | CD38 | NM_001775.2 | 453 | accgtacctgcaacaagattct | - | see also 16060 line |
| 36498 | CD38 | NM_001775.2 | 305 | atgcttcaagtacactgaaattc | - | see also 16060 line |
| 36499 | ASML3B | NM_014474.1 | 1081 | agccatccgggttcgaatatg | - | see also 16061 line |
| 36500 | ASML3B | NM_014474.1 | 1082 | gccatccgggttcgaatatga | - | see also 16061 line |
| 36501 | ASML3B | NM_014474.1 | 997 | cccataagcgccatgttcatca | - | see also 16061 line |
| 36502 | CTBS | NM_004388.1 | 277 | tgctacgtcattcaaaaggagc | - | see also 16062 line |
| 36503 | CTBS | NM_004388.1 | 857 | gacgtcaggtgcctacaaaacg | - | see also 16062 line |
| 36504 | CTBS | NM_004388.1 | 866 | tgccctacaaacgatcatgaag | - | see also 16062 line |
| 36505 | FLJ21865 | NM_022759.1 | 210 | gaccccctgccagttagatatta | - | see also 16063 line |

Figure 46

| 36506 | FLJ21865 | NM_022759.1 | 896 | ctgcgacggcttcttcactaact | 203145 | - | see also 16063 line |
|---|---|---|---|---|---|---|---|
| 36507 | SMPDL3A | NM_006714.2 | 509 | tgcgctgggtaatcatgactatt | 203163 | - | see also 16064 line |
| 36508 | SMPDL3A | NM_006714.2 | 708 | tacggcccaaatataatgacact | 203176 | - | see also 16064 line |
| 36509 | SMPDL3A | NM_006714.2 | 1190 | gacccagacctacgacattgaag | 203197 | - | see also 16064 line |
| 36510 | TDG | NM_003211.2 | 845 | gccacgaatagcagtgtttaatg | 203222 | - | see also 16065 line |
| 36511 | TDG | NM_003211.2 | 844 | agccacgaatagcagtgtttaat | 203221 | - | see also 16065 line |
| 36512 | PIGL | NM_004278.2 | 725 | ctcccggtacatgagaatcaact | 203244 | - | see also 16066 line |
| 36513 | PIGL | NM_004278.2 | 727 | cccggtacatgagaatcaactca | 203245 | - | see also 16066 line |
| 36514 | PIGL | NM_004278.2 | 314 | ctccagtgtaatgattattgaca | 203237 | - | see also 16066 line |
| 36515 | ASAH1 | NM_004315.2 | 1317 | accgtatacacaaccttgataga | 203270 | - | see also 16067 line |
| 36516 | ASAH1 | NM_004315.2 | 566 | gggtattgccgctgttactgata | 203256 | - | see also 16067 line |
| 36517 | AGA | NM_000027.2 | 473 | aagtatggggtttatcaatgaag | 203281 | - | see also 16068 line |
| 36518 | AGA | NM_000027.2 | 187 | cagcgtggagggcattagcatct | 203276 | - | see also 16068 line |
| 36519 | ASRGL1 | NM_025080.2 | 646 | tccacaggcggtatcgttaataa | 203306 | - | see also 16069 line |
| 36520 | ASRGL1 | NM_025080.2 | 650 | caggcggtatcgttaataaaatg | 203308 | - | see also 16069 line |
| 36521 | C20orf13 | NM_017714.1 | 386 | caggaatgggatctaatctaaat | 203323 | - | see also 7123 line |
| 36522 | C20orf13 | NM_017714.1 | 387 | aggaatgggatctaatctaaatc | 203324 | - | see also 7123 line |
| 36523 | ASPA | NM_000049.1 | 1009 | atgaggccgcatattacgaaaag | 203370 | - | see also 17 line |
| 36524 | ASPA | NM_000049.1 | 1012 | aggccgcatattacgaaaagaaa | 203371 | - | see also 17 line |
| 36525 | ASPA | NM_000049.1 | 1013 | ggccgcatattacgaaaagaaag | 203372 | - | see also 17 line |
| 36526 | GA | NM_013267.2 | 1245 | tgccatcggctattatctcaagg | 203390 | - | see also 16072 line |
| 36527 | GA | NM_013267.2 | 534 | acgaatccctatccacaagttca | 203383 | - | see also 16072 line |
| 36528 | GLS | NM_014905.2 | 1969 | aacagcgggactatgattctaga | 203453 | - | see also 16073 line |
| 36529 | GLS | NM_014905.2 | 1972 | agcgggactatgattctagaaca | 203455 | - | see also 16073 line |
| 36530 | FLJ31204 | NM_174912.2 | 193 | ggccgagcagctttagtcttagg | 203460 | - | see also 16074 line |
| 36531 | FLJ31204 | NM_174912.2 | 629 | ctcttggcataaccaactgtagt | 203468 | - | see also 16074 line |
| 36532 | FLJ31204 | NM_174912.2 | 358 | gacgtgaacccaatgatcaatgg | 203463 | - | see also 16074 line |
| 36533 | QRSL1 | NM_018292.2 | 1506 | aaccaagggttgccaataggact | 203529 | - | see also 16075 line |
| 36534 | QRSL1 | NM_018292.2 | 385 | aaggttatataccaccttataat | 203497 | - | see also 16075 line |
| 36535 | UPB1 | NM_016327.1 | 342 | tgcccttcatagacgcataaagg | 203536 | - | see also 16076 line |
| 36536 | UPB1 | NM_016327.1 | 570 | cagcgagcatgggatgttttgt | 203540 | - | see also 16076 line |
| 36537 | VNN3 | NM_018399.2 | 159 | agcatgcggtgatattaccaaac | 203553 | - | see also 7446 line |
| 36538 | VNN3 | NM_018399.2 | 158 | gagcatgcggtgatattaccaaa | 203552 | - | see also 7446 line |
| 36539 | ADA | NM_000022.1 | 750 | ggctcggccgaagtagtaaaaga | 203583 | - | see also 16078 line |
| 36540 | ADA | NM_000022.1 | 749 | gggctcggccgaagtagtaaaag | 203582 | - | see also 16078 line |
| 36541 | ADA | NM_000022.1 | 748 | tgggctcggccgaagtagtaaaa | 203581 | - | see also 16078 line |
| 36542 | AMPD1 | NM_000036.1 | 842 | aaggacctgttaagacctatacc | 203615 | - | see also 16079 line |

EP 1 752 536 A1

Figure 46

| | | | | | | | |
|---|---|---|---|---|---|---|---|
| 36543 | AMPD1 | NM_000036.1 | 1884 | gagtcccgtgctacagtacttgt | 203639 | - | see also 16079 line |
| 36544 | AMPD2 | NM_004037.5 | 1643 | aagtgccacaccgagatttctac | 203663 | - | see also 2728 line |
| 36545 | AMPD3 | NM_000480.1 | 2593 | tccgatatgagaccttatgcaat | 203711 | - | see also 375 line |
| 36546 | AMPD3 | NM_000480.1 | 771 | accatcagcggagattactgtgc | 203682 | - | see also 375 line |
| 36547 | ATIC | NM_004044.4 | 251 | ctggaatcctagctcgtaatatt | 203720 | - | see also 16082 line |
| 36548 | ATIC | NM_004044.4 | 1208 | gtgtcgtcgacaagtcattattt | 203748 | - | see also 16082 line |
| 36549 | GCH1 | NM_000161.1 | 494 | gtcctaaacgatgctatatttga | 203769 | - | see also 16083 line |
| 36550 | GCH1 | NM_000161.1 | 668 | atctatagtagaagactacaagt | 203776 | - | see also 16083 line |
| 36551 | DDAH1 | NM_012137.2 | 651 | atcgcaattgggtctagtgaatc | 203787 | - | see also 5147 line |
| 36552 | DDAH1 | NM_012137.2 | 712 | accaccgctacgacaaactcact | 203790 | - | see also 5147 line |
| 36553 | DDAH2 | NM_013974.1 | 610 | ctccgaattgtggaaataggaga | 203799 | - | see also 16085 line |
| 36554 | DDAH2 | NM_013974.1 | 444 | ggggctacagctgctagaactgc | 203797 | - | see also 16085 line |
| 36555 | CRMP1 | NM_001313.2 | 1654 | agcgcgtcaaaatcaggaataag | 203816 | - | see also 16086 line |
| 36556 | CRMP1 | NM_001313.2 | 291 | aacgatgaccaatccctttatgc | 203800 | - | see also 16086 line |
| 36557 | CRMP1 | NM_001313.2 | 682 | tgcaggacaaaggcgtcaattcc | 203806 | - | see also 16086 line |
| 36558 | DPYS | NM_001385.1 | 1219 | gtgcatagtggtaaaatggatga | 203841 | - | see also 16087 line |
| 36559 | DPYS | NM_001385.1 | 664 | ttctctcggtgcaaggaaattgg | 203828 | - | see also 16087 line |
| 36560 | DPYSL2 | NM_001386.3 | 798 | agctaacggattgccagatttat | 203861 | - | see also 951 line |
| 36561 | DPYSL3 | NM_001387.1 | 1526 | cccgttctccgactatgtctaca | 203897 | - | see also 954 line |
| 36562 | DPYSL3 | NM_001387.1 | 1040 | cccaactactccggactacatca | 203890 | - | see also 954 line |
| 36563 | IGSF2 | NM_004258.1 | 2333 | gcgatcggggcaaatatcactgt | 203933 | - | see also 16089 line |
| 36564 | IGSF2 | NM_004258.1 | 2017 | aggatagccgtcactttaccaga | 203923 | - | see also 16089 line |
| 36565 | MGC35366 | NM_152435.1 | 1295 | tcccgatgggagcatttgattta | 203958 | - | see also 16090 line |
| 36566 | MGC35366 | NM_152435.1 | 1294 | atcccgatgggagcatttgattt | 203957 | - | see also 16090 line |
| 36567 | MGC35366 | NM_152435.1 | 1291 | ttcatcccgatgggagcatttga | 203956 | - | see also 16090 line |
| 36568 | NIT1 | NM_005600.1 | 955 | tgcccgaatagacctcaactatc | 203968 | - | see also 3892 line |
| 36569 | NIT1 | NM_005600.1 | 672 | gtctagctgtctgctatgacatg | 203966 | - | see also 3892 line |
| 36570 | NIT1 | NM_005600.1 | 244 | gacgccagacaagcaacagaact | 203962 | - | see also 3892 line |
| 36571 | BTD | NM_000060.1 | 627 | gtcgtgttcagcaataatggaac | 203978 | - | see also 35 line |
| 36572 | BTD | NM_000060.1 | 343 | aggatgtacagattatagtgttt | 203971 | - | see also 35 line |
| 36573 | NIT2 | NM_020202.2 | 115 | ttccatcaaatcagataacgtca | 204003 | - | see also 16093 line |
| 36574 | NIT2 | NM_020202.2 | 169 | aacgcaaggagccaaaatagttt | 204005 | - | see also 16093 line |
| 36575 | NIT2 | NM_020202.2 | 625 | accagcccattgggagttacttc | 204013 | - | see also 16093 line |
| 36576 | VNN1 | NM_004666.1 | 32 | agcttacgtggcaattttgcttt | 204017 | - | see also 16094 line |

Figure 46

| 36577 | VNN1 | NM_004666.1 | 1175 | cactgtggaagggcgctattatc | 204035 | - | see also 16094 line |
|---|---|---|---|---|---|---|---|
| 36578 | VNN2 | NM_004665.2 | 1421 | atctgggcctatactaacagtgt | 204084 | - | see also 16095 line |
| 36579 | VNN2 | NM_004665.2 | 1415 | tggatcatctgggcctatactaa | 204083 | - | see also 16095 line |
| 36580 | ABHD6 | NM_020676.3 | 639 | ctgcagtactcaactgacaatca | 204103 | - | see also 7915 line |
| 36581 | ABHD6 | NM_020676.3 | 575 | tggggtgtatgctgcttactacc | 204101 | - | see also 7915 line |
| 36582 | ABHD7 | NM_173567.2 | 483 | ctccattcatcgacagaattat | 204117 | - | see also 16097 line |
| 36583 | ABHD7 | NM_173567.2 | 484 | tcccattcatcgacagaattata | 204118 | - | see also 16097 line |
| 36584 | ABHD8 | NM_024527.2 | 1288 | caeggcatgcacgataagtttgt | 204149 | - | see also 16098 line |
| 36585 | ABHD8 | NM_024527.2 | 1286 | tccaeggcatgcacgataagttt | 204148 | - | see also 16098 line |
| 36586 | ABHD9 | NM_024794.1 | 460 | gcctgcgtcgcactatgtctcg | 204150 | - | see also 16099 line |
| 36587 | ABHD9 | NM_024794.1 | 1243 | aggagatgcaccagtacatgtgg | 204155 | - | see also 16099 line |
| 36588 | EPHX1 | NM_000120.2 | 557 | ctcctactgcggaatgaatttg | 204159 | - | see also 16100 line |
| 36589 | EPHX1 | NM_000120.2 | 555 | atctctactgcggaatgaatt | 204158 | - | see also 16100 line |
| 36590 | EPHX2 | NM_001979.3 | 1123 | gggcgggttgcccagtttgaatact | 204205 | - | see also 16101 line |
| 36591 | EPHX2 | NM_001979.3 | 210 | gccactaccccgcttatgaaagg | 204183 | - | see also 16101 line |
| 36592 | MEST | NM_002402.2 | 711 | ctggtcgcttaccataaagagt | 204228 | - | see also 16102 line |
| 36593 | MEST | NM_002402.2 | 709 | atctggtcggcttaccataaaga | 204227 | - | see also 16102 line |
| 36594 | AHCY | NM_000687.1 | 1023 | gaccggtatcggttgaagaatgg | 204244 | - | see also 16103 line |
| 36595 | AHCY | NM_000687.1 | 1163 | gtggaaccatccaggacaagtacc | 204245 | - | see also 16103 line |
| 36596 | AHCYL1 | NM_006621.3 | 1461 | cggcaagtcgatgtcgtaataac | 204270 | - | see also 4682 line |
| 36597 | AHCYL1 | NM_006621.3 | 1460 | ccggcaagtcgatgtcgtaataa | 204269 | - | see also 4682 line |
| 36598 | KIAA0828 | NM_015328.1 | 922 | tggtggtgtatcgatagatgtgt | 204302 | - | see also 6366 line |
| 36599 | KIAA0828 | NM_015328.1 | 1548 | gcgagtgagatctcaagttgacc | 204324 | - | see also 6366 line |
| 36600 | FAH | NM_000137.1 | 458 | taccaacgtcgaatcatgttca | 204333 | - | see also 16106 line |
| 36601 | FAH | NM_000137.1 | 606 | gactctaagcctccgtatatgg | 204334 | - | see also 16106 line |
| 36602 | FAH | NM_000137.1 | 455 | tgctaccaacgtcggaatcatgt | 204332 | - | see also 16106 line |
| 36603 | GPAA1 | NM_003801.2 | 446 | gacccacgagcgctatatgtgt | 204345 | - | see also 16107 line |
| 36604 | GPAA1 | NM_003801.2 | 223 | cgctgaccagcgcacttacatg | 204344 | - | see also 16107 line |
| 36605 | AADAC | NM_001086.1 | 501 | tcgtcgtatcaaccaactacaga | 204364 | - | see also 16108 line |
| 36606 | AADAC | NM_001086.1 | 492 | ttgatgctgctcgtatcaacc | 204363 | - | see also 16108 line |
| 36607 | ABHD1 | NM_032604.2 | 1006 | tacaatccgccagtttgatgagc | 204401 | - | see also 16109 line |
| 36608 | ABHD1 | NM_032604.2 | 710 | gtcgtgaaccacataaagcatcg | 204395 | - | see also 16109 line |
| 36609 | ABHD4 | NM_022060.1 | 661 | tcctaggacgttccaatccattg | 204412 | - | see also 16110 line |
| 36610 | ABHD4 | NM_022060.1 | 875 | ccctatgctgagcgaattcact | 204414 | - | see also 16110 line |
| 36611 | ACATE2 | NM_012332.1 | 156 | ggcaccctgcgttcgagataagt | 204423 | - | see also 16111 line |
| 36612 | ACATE2 | NM_012332.1 | 777 | cagtccacgtcgttactgaaaa | 204442 | - | see also 16111 line |
| 36613 | ADPRHL1 | NM_138430.3 | 627 | gtcgttcgccgcacaaggaaagc | 204469 | - | see also 16112 line |

Figure 46

| | | | | | | | |
|---|---|---|---|---|---|---|---|
| 36614 | ADPRHL1 | NM_138430.3 | 445 | cacacaccgttcaatgaaaaagg | 204468 | - | see also 16112 line |
| 36615 | ADPRHL1.2 | NM_017825.1 | 407 | cccaaatgtcgcgatgtctttg | 204475 | - | see also 16113 line |
| 36616 | ADPRHL1.2 | NM_017825.1 | 408 | cccaaatgtcgcgatgtctttga | 204476 | - | see also 16113 line |
| 36617 | ASAHL | NM_014435.1 | 607 | gcccacacaagtttacagttct | 204493 | - | see also 16114 line |
| 36618 | ASAHL | NM_014435.1 | 606 | agcccacacaagtttacagtttc | 204492 | - | see also 16114 line |
| 36619 | ASAHL | NM_014435.1 | 601 | gccagagcccacacaagttaca | 204490 | - | see also 16114 line |
| 36620 | ATP5C1 | NM_005174.1 | 179 | atggtagccgcagcaaaatatgc | 204494 | - | see also 16115 line |
| 36621 | ATP5C1 | NM_005174.1 | 661 | aagtgctgacagcagatgagtatct | 204503 | - | see also 16115 line |
| 36622 | ATP5D | NM_001687.3 | 455 | ggccgtgacggtggacatgttgg | 204518 | - | see also 16116 line |
| 36623 | ATP5D | NM_001687.3 | 232 | ctcccacgcaggttcttcaac | 204515 | - | see also 16116 line |
| 36624 | ATP5D | NM_001687.3 | 230 | ctctcccacgcagcgttcttca | 204514 | - | see also 16116 line |
| 36625 | ATP5O | NM_001697.1 | 656 | gggctatgcgggagattgtctaa | 204536 | - | see also 16117 line |
| 36626 | ATP5O | NM_001697.1 | 593 | ttgtgccattggcgagaaatat | 204530 | - | see also 16117 line |
| 36627 | ATP6AP2 | NM_005765.2 | 562 | ctccgtaatgcctgtttcaaga | 204551 | - | see also 4025 line |
| 36628 | ATP6AP2 | NM_005765.2 | 145 | ttggggaacgagtttagttatt | 204539 | - | see also 4025 line |
| 36629 | ATP6V0B | NM_004047.2 | 296 | cagcctggggcatctatattacc | 204576 | - | see also 16119 line |
| 36630 | ATP6V0B | NM_004047.2 | 669 | tggggtcatcgtcgcaattcttc | 204577 | - | see also 16119 line |
| 36631 | ATP6V0D1 | NM_004691.3 | 1084 | atcgtgtggatcgctgaatgtat | 204584 | - | see also 16120 line |
| 36632 | ATP6V0D1 | NM_004691.3 | 102 | gtcgttcttcccggagcttact | 204579 | - | see also 16120 line |
| 36633 | ATP6V1D | NM_015994.2 | 760 | agcgagaagagttctataggtta | 204601 | - | see also 16121 line |
| 36634 | ATP6V1D | NM_015994.2 | 708 | ccccggattgaacgtactcttgc | 204597 | - | see also 16121 line |
| 36635 | ATP6V1G1 | NM_004888.2 | 406 | gacattcggccagaaatccatga | 204608 | - | see also 16122 line |
| 36636 | ATP6V1G1 | NM_004888.2 | 410 | ttcggccagaaatccatgaaaac | 204609 | - | see also 16122 line |
| 36637 | ATP6V1G1 | NM_004888.2 | 411 | tcggccagaaatccatgaaaact | 204610 | - | see also 16122 line |
| 36638 | ATP6V1G2 | NM_130463.2 | 622 | caccccaactaccggattctgc | 204614 | - | see also 16123 line |
| 36639 | ATP6V1G3 | NM_133262.2 | 365 | cactacaataagtatatggaaag | 204624 | - | see also 16124 line |
| 36640 | ATP6V1G3 | NM_133262.2 | 344 | aagatacaagaacttaatggaca | 204620 | - | see also 16124 line |
| 36641 | BPHL | NM_004332.1 | 910 | ttcatgccgacttcattcataag | 204640 | - | see also 16125 line |
| 36642 | BPHL | NM_004332.1 | 302 | tgggttcagctgcattaccagc | 204626 | - | see also 16125 line |
| 36643 | C11orf8 | NM_001584.1 | 895 | atcatgaccgacggttacacaac | 204661 | - | see also 16126 line |

Figure 46

| | | | | | | | |
|---|---|---|---|---|---|---|---|
| 36644 | C11orf8 | NM_001584.1 | 906 | cggttacacaacgtacatcaatg | 204663 | - | see also 16126 line |
| 36645 | C20orf147 | NM_152667.1 | 404 | tcgcctacttcttattaacgaat | 204680 | - | see also 16127 line |
| 36646 | C20orf147 | NM_152667.1 | 333 | atctacacgtttacagcatatga | 204676 | - | see also 16127 line |
| 36647 | C22orf1 | NM_001585.2 | 993 | gtccgtatgcactgtgaactacc | 204696 | - | see also 16128 line |
| 36648 | C22orf1 | NM_001585.2 | 968 | atgggacgaccaacctatgtgaat | 204694 | - | see also 16128 line |
| 36649 | CHI3L1 | NM_001276.1 | 464 | cccagagtcgccggacttcatc | 204699 | - | see also 16129 line |
| 36650 | CHI3L1 | NM_001276.1 | 470 | gtcgccggactttcatcaagtca | 204701 | - | see also 16129 line |
| 36651 | CHI3L2 | NM_004000.1 | 371 | ttctacatcacgcttggaattca | 204718 | - | see also 16130 line |
| 36652 | CHI3L2 | NM_004000.1 | 203 | ttcattgccagcatcgaaaaca | 204716 | - | see also 16130 line |
| 36653 | DBR1 | NM_016216.1 | 138 | cggccgagctggataagatctatg | 204734 | - | see also 16131 line |
| 36654 | DBR1 | NM_016216.1 | 1180 | cagctgattcatagggatcaatcc | 204766 | - | see also 16131 line |
| 36655 | DIO3 | NM_001362.1 | 908 | gcctacttcgagcgtctctatgt | 204780 | - | see also 16132 line |
| 36656 | DIO3 | NM_001362.1 | 769 | cacggactctccctacatcatcc | 204779 | - | see also 16132 line |
| 36657 | DKFZP564 D1378 | NM_032124.3 | 541 | tggagcaactctgatagcaatcc | 204782 | - | see also 16133 line |
| 36658 | DKFZP564 D1378 | NM_032124.3 | 603 | tgggcctggaccatttgtgact | 204784 | - | see also 16133 line |
| 36659 | DKFZP564 O243 | NM_015407.3 | 295 | ctggcaactcgcccatctttac | 204791 | - | see also 16134 line |
| 36660 | DKFZP564 O243 | NM_015407.3 | 296 | tggcaactcgccatctttacc | 204792 | - | see also 16134 line |
| 36661 | DPYSL4 | NM_006426.1 | 1289 | gtcggtgaccagtacaaatgc | 204797 | - | see also 4499 line |
| 36662 | DPYSL4 | NM_006426.1 | 184 | cacgagtgacgccttctgatca | 204794 | - | see also 4499 line |
| 36663 | DPYSL5 | NM_020134.2 | 990 | cacgctgacaggcttacactact | 204819 | - | see also 16136 line |
| 36664 | DPYSL5 | NM_020134.2 | 485 | ccctgtggacgcttatgagaag | 204813 | - | see also 16136 line |
| 36665 | DPYSL5 | NM_020134.2 | 483 | ctccctgtggacgcttatgaga | 204812 | - | see also 16136 line |
| 36666 | ENPP4 | NM_014936.1 | 1000 | gccgatgaaggctggacaattgt | 204852 | - | see also 6119 line |
| 36667 | ENPP4 | NM_014936.1 | 111 | ctctagtttgccacctaagttac | 204826 | - | see also 6119 line |
| 36668 | ENPP5 | NM_021572.4 | 1217 | ggcaaccacggttacgataatgc | 204905 | - | see also 16138 line |
| 36669 | ENPP5 | NM_021572.4 | 422 | acctacctaaccattatacttt | 204874 | - | see also 16138 line |
| 36670 | ENPP6 | NM_153343.2 | 738 | gaccgcctgaacgtcattatttt | 204942 | - | see also 16139 line |
| 36671 | ENPP6 | NM_153343.2 | 251 | tagtctcgtatcccaattatt | 204928 | - | see also 16139 line |
| 36672 | EYA1 | NM_000503.3 | 809 | gccgacggtctttaaacaatt | 204962 | - | see also 390 line |
| 36673 | EYA1 | NM_000503.3 | 1664 | gggtcctacgccaacagatatgg | 204985 | - | see also 390 line |
| 36674 | EYA2 | NM_005244.3 | 871 | agtccgagccggtctaagagg | 205009 | - | see also 16141 line |
| 36675 | EYA2 | NM_005244.3 | 978 | tccttactcacgggacatttgc | 205011 | - | see also 16141 line |
| 36676 | EYA3 | NM_001990.2 | 489 | ccctcaggcaaccaccaaacgtatg | 205034 | - | see also 1273 line |

Figure 46

| 36677 | EYA3 | NM_001990.2 | 347 | ctgctcatccaatgattatacc | 205031 | - | see also 1273 line |
|---|---|---|---|---|---|---|---|
| 36678 | EYA4 | NM_004100.2 | 1301 | atcaacgtatggagcgtatatga | 205076 | - | see also 2769 line |
| 36679 | EYA4 | NM_004100.2 | 1313 | agcgtatatgacatcgaataaca | 205078 | - | see also 2769 line |
| 36680 | FAM16AX | NM_012080.1 | 341 | gtccgcgtcgttcgtatgaaga | 205121 | - | see also 16144 line |
| 36681 | FAM16AX | NM_012080.1 | 338 | ggggtccgcgtcgttcgtatga | 205120 | - | see also 16144 line |
| 36682 | FLJ11151 | NM_018340.1 | 941 | gtcaccgccgagaaaattgttca | 205129 | - | see also 16145 line |
| 36683 | FLJ11151 | NM_018340.1 | 962 | caccgatacacagtctagatga | 205131 | - | see also 16145 line |
| 36684 | FLJ11151 | NM_018340.1 | 943 | caccgccgagaaaatgttcacc | 205130 | - | see also 16145 line |
| 36685 | FLJ11342 | NM_018394.1 | 291 | tggcgattgaggagtttgcaaa | 205148 | - | see also 16146 line |
| 36686 | FLJ11342 | NM_018394.1 | 909 | agctctcaactatagttaactag | 205171 | - | see also 16146 line |
| 36687 | FLJ21736 | NM_024922.3 | 1241 | accgtcatagatgaataacctagg | 205183 | - | see also 16147 line |
| 36688 | FLJ21736 | NM_024922.3 | 1310 | atggtgacgtattcatcaatgt | 205185 | - | see also 16147 line |
| 36689 | FLJ31547 | NM_145024.1 | 1484 | tgctcgaaccggaatcctaatg | 205210 | - | see also 16148 line |
| 36690 | FLJ31547 | NM_145024.1 | 1483 | ttgctcgaaccgggaatcctaat | 205209 | - | see also 16148 line |
| 36691 | GNPDA1 | NM_005471.2 | 782 | tgcaggtgggatcgagctatttg | 205224 | - | see also 16149 line |
| 36692 | GNPDA1 | NM_005471.2 | 1198 | tggaccccttgtacagtatcaaa | 205227 | - | see also 16149 line |
| 36693 | KIAA1363 | NM_020792.1 | 871 | tgcaggcaatgatcgttaacaat | 205252 | - | see also 7970 line |
| 36694 | LHPP | NM_022126.2 | 332 | cggagtccgctcagaatttgatc | 205267 | - | see also 16151 line |
| 36695 | LHPP | NM_022126.2 | 330 | gacggagtccgctcagaatttga | 205266 | - | see also 16151 line |
| 36696 | LOC134147 | NM_138809.2 | 789 | ttccactcaaggacgtatcttg | 205286 | - | see also 16152 line |
| 36697 | LOC134147 | NM_138809.2 | 413 | cccaataccagatatatagctga | 205279 | - | see also 16152 line |
| 36698 | LOC339221 | NM_178543.2 | 262 | ccctggtcaccggcaaatatatc | 205291 | - | see also 16153 line |
| 36699 | LOC339221 | NM_178543.2 | 260 | caccctggtcaccggccaaatata | 205290 | - | see also 16153 line |
| 36700 | MASA | NM_021204.1 | 251 | caccgtgatcctgttagatatcg | 205301 | - | see also 16154 line |
| 36701 | MASA | NM_021204.1 | 653 | ggccggaatgaaggtgtacatct | 205308 | - | see also 16154 line |
| 36702 | MGC12904 | NM_031219.2 | 1069 | ccccatcgctgaacagcttata | 205317 | - | see also 16155 line |
| 36703 | MGC12904 | NM_031219.2 | 1345 | agcccatgttgggatattacc | 205322 | - | see also 16155 line |
| 36704 | MGC3234 | NM_023947.2 | 954 | aagtccaagtgggggaagcaaaat | 205332 | - | see also 16156 line |
| 36705 | MGC3234 | NM_023947.2 | 982 | tgsggctcaacttctatggtatg | 205334 | - | see also 16156 line |
| 36706 | MPPE1 | NM_023075.2 | 1964 | tgctcggaaagcgtaagacaaga | 205356 | - | see also 16157 line |
| 36707 | MPPE1 | NM_023075.2 | 1199 | atgattggagcgtgtttcagaaa | 205337 | - | see also 16157 line |
| 36708 | OVGP1 | NM_002557.2 | 628 | atgtccaaacatcctatgatgt | 205371 | - | see also 16158 line |

Figure 46

| | | | | | | | |
|---|---|---|---|---|---|---|---|
| 36709 | OVGP1 | NM_002557.2 | 1172 | tcccccttgtctacgtattgaat | 205379 | - | see also 16158 line |
| 36710 | OVGP1 | NM_002557.2 | 1205 | tgcgggctgagttcagttcaact | 205384 | - | see also 16158 line |
| 36711 | PHCA | NM_018367.3 | 531 | tacattagtacttcgatctattt | 205419 | - | see also 7420 line |
| 36712 | PHCA | NM_018367.3 | 344 | atgaactcccaatgatatacagc | 205410 | - | see also 7420 line |
| 36713 | PIGO | NM_032634.2 | 862 | cacgccatagtggaagacaatct | 205433 | - | see also 9265 line |
| 36714 | PLP | NM_020315.3 | 739 | ccccgcacgcacgcttatggtgg | 205467 | - | see also 16160 line |
| 36715 | PLP | NM_020315.3 | 200 | gccggcaaggcggctctgtttgt | 205465 | - | see also 16160 line |
| 36716 | SGPP2 | NM_152386.2 | 745 | ttccccgtgtgtgtcatagttgt | 205484 | - | see also 16161 line |
| 36717 | SGPP2 | NM_152386.2 | 742 | ctcttccccgtgtgtgtcatagt | 205483 | - | see also 16161 line |
| 36718 | THEM2 | NM_018473.2 | 454 | acccggagtcagtgtcgatatga | 205505 | - | see also 16162 line |
| 36719 | THEM2 | NM_018473.2 | 392 | ttgacagccacgttagtagataa | 205499 | - | see also 16162 line |
| 36720 | VPS29 | NM_016226.2 | 240 | aggagacttcgatgagaatctga | 205514 | - | see also 16163 line |
| 36721 | VPS29 | NM_016226.2 | 544 | acctatgtgtatcagctaattgg | 205524 | - | see also 16163 line |
| 36722 | COL4A3BP | NM_005713.1 | 1911 | ttctcagcgagacgtattatatc | 205563 | - | see also 3962 line |
| 36723 | COL4A3BP | NM_005713.1 | 2034 | ccgatgtgtccgtgccaaaataa | 205565 | - | see also 3962 line |
| 36724 | CSNK2B | NM_001320.5 | 382 | tgggctccgtggcaatgaattct | 205577 | - | see also 872 line |
| 36725 | CSNK2B | NM_001320.5 | 560 | caggcagccgagatgctttatgg | 205583 | - | see also 872 line |
| 36726 | PRKDC | NM_006904.6 | 11939 | ttcggctaactcgccagtttatc | 205898 | - | see also 4842 line |
| 36727 | PRKDC | NM_006904.6 | 3144 | tggtcggtgtattcgagaattcc | 205671 | - | see also 4842 line |
| 36728 | PRKAG1 | NM_002733.1 | 689 | ggcacctatgccaatattgctat | 205924 | - | see also 1772 line |
| 36729 | PRKAG1 | NM_002733.1 | 691 | cacctatgccaatattgctatgg | 205925 | - | see also 1772 line |
| 36730 | CKS2 | NM_001827.1 | 147 | gagtaccggcatgttatgttacc | 205935 | - | see also 16168 line |
| 36731 | CKS2 | NM_001827.1 | 302 | tagacgacctcttccaaaagatc | 205940 | - | see also 16168 line |
| 36732 | PRKAG3 | NM_017431.1 | 1209 | ttcccgctttgatgtgattcacc | 205956 | - | see also 16169 line |
| 36733 | PRKAG3 | NM_017431.1 | 1201 | ggcctctattcccgctttgatgt | 205955 | - | see also 16169 line |
| 36734 | GTF2H1 | NM_005316.2 | 1341 | atgcaacaagtcaggacattatt | 205961 | - | see also 16170 line |
| 36735 | GTF2H1 | NM_005316.2 | 1785 | ggcgtctgatgaagaaaacgtga | 205967 | - | see also 16170 line |
| 36736 | BCR | NM_004327.2 | 3001 | gccgcaacggcaagagttacacg | 205972 | - | see also 16171 line |
| 36737 | BCR | NM_004327.2 | 640 | aggagcgcttccgcatgatctac | 205968 | - | see also 16171 line |
| 36738 | BRD2 | NM_005104.2 | 3931 | agcggacagctcaattctactaa | 206011 | - | see also 16172 line |
| 36739 | BRD2 | NM_005104.2 | 2025 | gggtctaccggattatcacaaaa | 205979 | - | see also 16172 line |
| 36740 | FASTK | NM_006712.3 | 1276 | accgtgcccgctgcaagtacagt | 206020 | - | see also 16173 line |
| 36741 | FASTK | NM_006712.3 | 944 | gccctttgggcgactgaactacc | 206015 | - | see also 16173 line |
| 36742 | H11 | NM_014365.2 | 918 | gtggcattgtttctaagaacttc | 206023 | - | see also 16174 line |
| 36743 | H11 | NM_014365.2 | 1034 | gtccctccttactcaacatttgg | 206027 | - | see also 16174 line |

Figure 46

| | | | | | | | | | transmembrane-ephrin | |
|---|---|---|---|---|---|---|---|---|---|---|
| 36744 | EFNA3 | NM_004952.3 | 415 | cccgcacagccccatcaagttct | 206028 | - | receptor_acitivity | receptor | - |
| 36745 | EFNA3 | NM_004952.3 | 644 | atcaacgtgctggaagactttga | 206029 | - | see also 36744 line | | |
| 36746 | EFNA4 | NM_005227.2 | 294 | ccccgagacgtttgctttgtaca | 206033 | - | see also 3664 line | | |
| 36747 | EFNB3 | NM_001406.3 | 783 | tccgctcgcaccacgattactac | 206047 | - | see also 16176 line | | |
| 36748 | EFNB3 | NM_001406.3 | 791 | caccacgattactacatcattgc | 206049 | - | see also 16176 line | | |
| 36749 | NRP2 | NM_003872.1 | 78 | accgtgcggaggtcgtttgaatt | 206055 | - | see also 2633 line | | |
| 36750 | NRP2 | NM_003872.1 | 464 | gccccaacgggaccatcgaatct | 206065 | - | see also 2633 line | | |
| 36751 | FGFRL1 | NM_021923.2 | 1011 | aggacgatgcgggcatgtacatc | 206107 | - | see also 16178 line | | |
| 36752 | FGFRL1 | NM_021923.2 | 1413 | cagttgctggccctaagttgtac | 206109 | - | see also 16178 line | | |
| 36753 | PDGFRL | NM_006207.1 | 712 | agccaatggaacggacattgttt | 206123 | - | see also 16179 line | | |
| 36754 | PDGFRL | NM_006207.1 | 331 | aactgtagagcttcgatgtaaag | 206116 | - | see also 16179 line | | |
| 36755 | PDGFRL | NM_006207.1 | 243 | caccaaagacgcagtctatcatg | 206114 | - | see also 16179 line | | |
| 36756 | IGF2R | NM_000876.1 | 6205 | aacggagtctcgtactatataaa | 206284 | - | see also 572 line | | |
| 36757 | IGF2R | NM_000876.1 | 1185 | gagcggaggttcatcctatattt | 206151 | - | see also 572 line | | |
| 36758 | CRKL | NM_005207.2 | 1366 | tcccctttacgcacgtcaaaatc | 206328 | - | see also 16181 line | | |
| 36759 | CRKL | NM_005207.2 | 596 | gggccagcgccacggtatgttcc | 206307 | - | see also 16181 line | | |
| 36760 | WIF1 | NM_007191.2 | 1190 | cagcacacgccttcacttaaaaa | 206355 | - | see also 4987 line | | |
| 36761 | WIF1 | NM_007191.2 | 757 | ttgtaccccacgatgtatgaatg | 206346 | - | see also 4987 line | | |
| 36762 | C9orf39 | NM_017738.1 | 371 | ctccggcaaagtgttactaatct | 206362 | - | see also 16183 line | | |
| 36763 | C9orf39 | NM_017738.1 | 535 | aagcttagaaacgttaatggttt | 206367 | - | see also 16183 line | | |
| 36764 | KCNH2 | NM_000238.2 | 1953 | ttggctccctcatgtatgctagc | 206437 | - | see also 174 line | | |
| 36765 | KCNH2 | NM_000238.2 | 1949 | ctcattggctccctcatgtatgc | 206436 | - | see also 174 line | | |
| 36766 | KCNH3 | NM_012284.1 | 817 | agggcaagcacaagctcaataag | 206444 | - | see also 16185 line | | |
| 36767 | KCNH3 | NM_012284.1 | 1889 | ggccacctgggcggtgaacaatg | 206449 | - | see also 16185 line | | |
| 36768 | KCNH4 | NM_012285.1 | 1194 | gtcccagtccggccaggtaatct | 206461 | - | see also 16186 line | | |
| 36769 | KCNH4 | NM_012285.1 | 2031 | ggccctatcgctgcacatcaaga | 206469 | - | see also 16186 line | | |
| 36770 | KCNH8 | NM_144633.2 | 1440 | gtcgatccgaagtgcctatattg | 206515 | - | see also 16187 line | | |
| 36771 | KCNH8 | NM_144633.2 | 1637 | ccctctatcacactagaactaag | 206519 | - | see also 16187 line | | |
| 36772 | ATR | NM_001184.2 | 1943 | ttgccgctaatcttctaacatta | 206613 | - | see also 16188 line | | |
| 36773 | ATR | NM_001184.2 | 6097 | atccatggtcgagctatgctact | 206747 | - | see also 16188 line | | |
| 36774 | CDK5R1 | NM_003885.2 | 455 | cacgcacctcaacaatgagaacc | 206827 | - | see also 16189 line | | |
| 36775 | CDK5R1 | NM_003885.2 | 838 | tgctctgcagggatgttatctcc | 206828 | - | see also 16189 line | | |
| 36776 | CDKN1A | NM_000389.2 | 526 | gaccagcatgacagatttctacc | 206830 | - | see also 16190 line | | |
| 36777 | PIK3C2A | NM_002645.1 | 3069 | tagtacccgatacgaacatgttt | 206922 | - | see also 1719 line | | |
| 36778 | PIK3C2A | NM_002645.1 | 3072 | tacccgatacgaacatgttttgg | 206923 | - | see also 1719 line | | |
| 36779 | PIK3C2B | NM_002646.2 | 4563 | aagagctctggccgaatcagtga | 207035 | - | see also 16192 line | | |

Figure 46

| 36780 | PIK3C2B | NM_002646.2 | 2776 | cccgttggagtgcacctaatttc | 207011 | - | see also 16192 line |
|---|---|---|---|---|---|---|---|
| 36781 | PIK3C2G | NM_004570.2 | 4271 | atgtacggaccccacttacaatg | 207165 | - | see also 3107 line |
| 36782 | PIK3C2G | NM_004570.2 | 3243 | aagtcgggccacatgtttcatat | 207134 | - | see also 3107 line |
| 36783 | PIK3C3 | NM_002647.2 | 1737 | aacgcgaaagtggaaatcgtaag | 207236 | - | see also 1728 line |
| 36784 | PIK3C3 | NM_002647.2 | 1707 | atcggttggtgcatctaatgaag | 207234 | - | see also 1728 line |
| 36785 | PIK3CA | NM_006218.1 | 118 | tgcctccgtgaggctacattagt | 207266 | - | see also 4306 line |
| 36786 | PIK3CA | NM_006218.1 | 806 | atcctctgagtcagtataagtat | 207275 | - | see also 4306 line |
| 36787 | PIK3CB | NM_006219.1 | 1514 | accctcccttcgataagattatt | 207392 | - | see also 4313 line |
| 36788 | PIK3CB | NM_006219.1 | 1181 | taccaagaatggctcgattatgt | 207377 | - | see also 4313 line |
| 36789 | PIK3CD | NM_005026.2 | 759 | ggcccttctggtcaacgttaagt | 207455 | - | see also 3512 line |
| 36790 | PIK3CD | NM_005026.2 | 605 | acgactttcgcgccaagatgtgc | 207451 | - | see also 3512 line |
| 36791 | PIK3CG | NM_002649.2 | 1154 | gtgggcgaaacgcccatcaaaaa | 207472 | - | see also 1729 line |
| 36792 | PIK3CG | NM_002649.2 | 1155 | tgggcgaaacgcccatcaaaaac | 207473 | - | see also 1729 line |
| 36793 | PIK3R2 | NM_005027.1 | 745 | agccctggctgacggcattaaga | 207530 | - | see also 16197 line |
| 36794 | PIK3R2 | NM_005027.1 | 1612 | ccgcgagtatgaccagctttatg | 207535 | - | see also 16197 line |
| 36795 | MGC26597 | NM_152700.2 | 1436 | gtcaatcgacactcaaagactgg | 207547 | - | see also 16198 line |
| 36796 | MGC26597 | NM_152700.2 | 1370 | ctggctgtcaatccacaatatag | 207544 | - | see also 16198 line |
| 36797 | MGC46424 | NM_173492.1 | 243 | ctccgagaggtatgacatcaaag | 207548 | - | see also 16199 line |
| 36798 | MGC46424 | NM_173492.1 | 244 | tccgagaggtatgacatcaaagg | 207549 | - | see also 16199 line |
| 36799 | PIP5K1A | NM_003557.1 | 425 | tcgtcttcggtcggtttttcatc | 207551 | - | see also 16200 line |
| 36800 | PIP5K1A | NM_003557.1 | 1332 | gcctcttgatgtcaatccataat | 207554 | - | see also 16200 line |
| 36801 | PIP5K1B | NM_003558.1 | 1116 | gacatgcacgaagggttgtattt | 207572 | - | see also 2365 line |
| 36802 | PIP5K1B | NM_003558.1 | 1893 | cacgacaggcctacactctattc | 207592 | - | see also 2365 line |
| 36803 | PIP5K1C | NM_012398.1 | 755 | tccgcgtcgtggtcatgaacaac | 207605 | - | see also 16202 line |
| 36804 | PIP5K1C | NM_012398.1 | 1350 | tgccgagcgcttttcaagttca | 207607 | - | see also 16202 line |
| 36805 | PIP5K2A | NM_005028.3 | 1232 | aacatcgacgtctatggaattaa | 207636 | - | see also 16203 line |
| 36806 | PIP5K2A | NM_005028.3 | 767 | ggcatgtaccggcttaatgttga | 207625 | - | see also 16203 line |
| 36807 | PIP5K2B | NM_003559.3 | 1106 | ctcactgtgcatcgcaagtatga | 207650 | - | see also 2366 line |
| 36808 | PIP5K2B | NM_003559.3 | 1108 | cactgtgcatcgcaagtatgacc | 207651 | - | see also 2366 line |
| 36809 | PIP5K2B | NM_003559.3 | 744 | acctgcccagccgctttaagttt | 207645 | - | see also 2366 line |
| 36810 | PIP5K2C | NM_024779.3 | 176 | ggcgtagcccactcgatcaatga | 207660 | - | see also 8742 line |
| 36811 | PIP5K2C | NM_024779.3 | 1224 | tccatccggagcagtatgctaag | 207676 | - | see also 8742 line |
| 36812 | IHPK1 | NM_153273.1 | 275 | ccgggaacagcgcttttacgagt | 207681 | - | see also 16205 line |

Figure 46

| | | | | | | | |
|---|---|---|---|---|---|---|---|
| 36813 | IHPK1 | NM_153273.1 | 338 | aggcgtggtatctgtctgtttg | 207683 | - | see also 16205 line |
| 36814 | IHPK2 | NM_016291.1 | 736 | atcggaaccagtacaaatttatc | 207708 | - | see also 16206 line |
| 36815 | IHPK2 | NM_016291.1 | 351 | gtggtatctgtgcggtttgaaga | 207696 | - | see also 16206 line |
| 36816 | IHPK3 | NM_054111.1 | 1079 | tgcggcatgcaggtttatcaaac | 207714 | - | see also 16207 line |
| 36817 | IHPK3 | NM_054111.1 | 1409 | aaggttgacatccgcatgattga | 207721 | - | see also 16207 line |
| 36818 | IPMK | NM_152230.1 | 953 | atgccggtcacaggaaaatatat | 207755 | - | see also 16208 line |
| 36819 | IPMK | NM_152230.1 | 954 | tgcgcgtcacaggaaaaatatata | 207756 | - | see also 16208 line |
| 36820 | PIK4CB | NM_002651.1 | 1377 | gagtacgaggtccgtagaaaact | 207793 | - | see also 1733 line |
| 36821 | PIK4CB | NM_002651.1 | 214 | gtcggggaactatcagtgattga | 207770 | - | see also 1733 line |
| 36822 | PIK4CB | NM_002651.1 | 2486 | atggcagtatgcggtctatcacc | 207809 | - | see also 1733 line |
| 36823 | FRAP1 | NM_004958.2 | 3677 | gtgcgacaccgaatcaatcatca | 207869 | - | see also 3446 line |
| 36824 | FRAP1 | NM_004958.2 | 1887 | ttgccactgtgcggatcatttc | 207832 | - | see also 3446 line |
| 36825 | FRDA | NM_000144.3 | 962 | ttggccattccgcgaaaaagatgc | 207945 | - | kinase_related — Friedreich ataxia, diabetes, Friedreich's ataxia |
| 36826 | PI4K2B | NM_018323.2 | 770 | atcggattgaccgtgcaaaatca | 207961 | - | see also 16211 line |
| 36827 | PI4K2B | NM_018323.2 | 1038 | ttggttagtcagataagaaaagc | 207969 | - | see also 16211 line |
| 36828 | PI4K2B | NM_018323.2 | 1427 | tccagctagtacagataccttgt | 207975 | - | see also 16211 line |
| 36829 | PI4KII | NM_018425.2 | 808 | atcgggctaccaccaaaggttgg | 207983 | - | see also 7467 line |
| 36830 | PI4KII | NM_018425.2 | 680 | ttccccgtacaaggtagtatac | 207980 | - | see also 7467 line |
| 36831 | SMG1 | NM_014006.2 | 3832 | accatatcgcttagtagtgaatc | 208053 | - | see also 16212 line |
| 36832 | SMG1 | NM_014006.2 | 6816 | ttcgaatttccggcaaatcatat | 208148 | - | see also 16212 line |
| 36833 | DGKH | NM_152910.3 | 203 | tccggaccaagaccagtattaaa | 208235 | - | see also 16213 line |
| 36834 | DGKH | NM_152910.3 | 2221 | gggagttgattatcaacaaaat | 208315 | - | see also 16213 line |
| 36835 | DGKH | NM_152910.3 | 287 | ttcgaggccgcacccttactat | 208240 | - | see also 16213 line |
| 36836 | DGKI | NM_004717.2 | 1249 | tgcgcttgaattgtataggaaag | 208382 | - | see also 16214 line |
| 36837 | DGKI | NM_004717.2 | 2350 | gacttgccgtatgtacattagacc | 208410 | - | see also 16214 line |
| 36838 | DGKI | NM_004717.2 | 2944 | cggaccttccgagttattggata | 208430 | - | see also 16214 line |
| 36839 | FLJ10842 | NM_018238.2 | 993 | acccgacaagcaaagaagatttt | 208450 | - | see also 7339 line |
| 36840 | FLJ10842 | NM_018238.2 | 698 | tggtatcttgggcctctaaaaat | 208445 | - | see also 7339 line |
| 36841 | CHK | NM_001277.1 | 1339 | gactacgcccaagcaaggtttga | 208478 | - | see also 842 line |
| 36842 | CHK | NM_001277.1 | 1004 | tgctcattgatttcgaatacagc | 208470 | - | see also 842 line |
| 36843 | CHK | NM_001277.1 | 1342 | tacgcccaagcaaggtttgatgc | 208479 | - | see also 842 line |
| 36844 | FLJ10761 | NM_018208.1 | 829 | cacgtgcggttcattgactatga | 208487 | - | see also 16217 line |
| 36845 | FLJ10761 | NM_018208.1 | 556 | ttcaggttaatcgccttagaaat | 208480 | - | see also 16217 line |
| 36846 | XYLB | NM_005108.2 | 1339 | gaggttcgagcacttaattgaagg | 208514 | - | see also 16218 line |
| 36847 | XYLB | NM_005108.2 | 1493 | ccccgggtatgtttatagacact | 208519 | - | see also 16218 line |
| 36848 | RBSK | NM_022128.1 | 459 | gtctgccagctcgaaataactcc | 208540 | - | see also 8291 line |
| 36849 | RBSK | NM_022128.1 | 87 | accgacctggtcagtcttacttc | 208523 | - | see also 8291 line |

Figure 46

| | | | | | | | |
|---|---|---|---|---|---|---|---|
| 36850 | RBSK | NM_022128.1 | 697 | gccaggtggtaatcattacctta | 208546 | - | see also 8291 line |
| 36851 | FLJ13052 | NM_023018.2 | 1082 | atgccagcgatgaaagctttgg | 208556 | - | see also 16220 line |
| 36852 | FLJ13052 | NM_023018.2 | 1325 | gaggggaacgcagcgttgttct | 208561 | - | see also 16220 line |
| 36853 | KHK | NM_000221.1 | 562 | acggagcacgtggtgtttgtcagc | 208570 | - | see also 16221 line |
| 36854 | DKFZP586B1621 | NM_015533.2 | 744 | ggcaaccgccgatgaggattgta | 208580 | - | see also 6455 line |
| 36855 | DKFZP586B1621 | NM_015533.2 | 511 | ctgtgcggcacgtgcttataca | 208576 | - | see also 6455 line |
| 36856 | EKI1 | NM_018638.3 | 595 | atggcaataagactgagttatta | 208593 | - | see also 16223 line |
| 36857 | EKI1 | NM_018638.3 | 1092 | tacaactacctggcatatgatat | 208602 | - | see also 16223 line |
| 36858 | FLJI1149 | NM_018339.3 | 328 | gccccgagcggactgcattatga | 208622 | - | see also 16224 line |
| 36859 | FLJI1149 | NM_018339.3 | 327 | tgccccgagcggactgcattatg | 208621 | - | see also 16224 line |
| 36860 | FLJI1149 | NM_018339.3 | 549 | accatattacaagaatacgaag | 208628 | - | see also 16224 line |
| 36861 | PDXK | NM_003681.2 | 415 | gactacgtgctcacaggttatac | 208640 | - | see also 16225 line |
| 36862 | PDXK | NM_003681.2 | 601 | gtggtgccgcttgcagacattat | 208644 | - | see also 16225 line |
| 36863 | PRIM1 | NM_000946.1 | 602 | aagggtggtcaagacgttaaaaa | 208669 | - | see also 626 line |
| 36864 | PRIM1 | NM_000946.1 | 369 | acgacgatgtgaggagatgttgt | 208658 | - | see also 626 line |
| 36865 | POLR2D | NM_004805.2 | 76 | gacgcctcacagctcatcttcc | 208695 | - | transcription_regulator / DNA-directed RNA polymerase III |
| 36866 | POLR2D | NM_004805.2 | 125 | ttctaaattcagaagttcatatg | 208696 | - | see also 36865 line |
| 36867 | POLR2D | NM_004805.2 | 410 | tgcagcagattcttgatgatatc | 208699 | - | see also 36865 line |
| 36868 | POLR2L | NM_021128.2 | 187 | atgagaagctgctcaattatgc | 208701 | - | see also 16226 line |
| 36869 | POLR2L | NM_021128.2 | 29 | tccctgtacgctgcttcacttgt | 208700 | - | see also 16226 line |
| 36870 | POLR3D | NM_001722.1 | 547 | ctgaggaacgacactgaaatat | 208703 | - | see also 16227 line |
| 36871 | POLR3D | NM_001722.1 | 772 | ggcctcccgaaggatgtatctgt | 208707 | - | see also 16227 line |
| 36872 | POLR3F | NM_006466.2 | 154 | cgccgatccggtcgaaatagaaa | 208713 | - | see also 16228 line |
| 36873 | POLR3F | NM_006466.2 | 152 | gacgccggatcccgtcgaaattaga | 208712 | - | see also 16228 line |
| 36874 | RPC5 | NM_018119.2 | 2138 | acceggtacgccgaaacatgatc | 208764 | - | see also 7264 line |
| 36875 | RPC5 | NM_018119.2 | 651 | ggcggaagaacgagttaagcaga | 208750 | - | see also 7264 line |
| 36876 | USP49 | NM_004275.2 | 690 | tgccggtggctggcgattcgttag | 208776 | - | see also 16230 line |
| 36877 | USP49 | NM_004275.2 | 545 | ctcgagttcctacagagtttct | 208775 | - | see also 16230 line |
| 36878 | POLD2 | NM_006230.1 | 197 | ggctaggagagcgcagcttagc | 208777 | - | see also 16231 line |
| 36879 | POLD2 | NM_006230.1 | 437 | agcctcctcggagtaaatacata | 208782 | - | see also 16231 line |
| 36880 | POLI | NM_007195.1 | 519 | ctgtgtcgggtcatgtatacaat | 208817 | - | see also 4999 line |
| 36881 | POLI | NM_007195.1 | 926 | gaggataaccccctgtgatact | 208838 | - | see also 4999 line |
| 36882 | CDS2 | NM_003818.2 | 547 | tcgtgatgtgcgttcagattaag | 208876 | - | see also 16234 line |
| 36883 | CDS2 | NM_003818.2 | 682 | gtgagacagtgacggattacttc | 208885 | - | see also 16234 line |

Figure 46

| 36884 | GALT | NM_000155.2 | 885 | atgatctagcctccatcatgaag | 208916 | - | see also 16235 line |
| 36885 | GALT | NM_000155.2 | 772 | cagtgagcactggttagtactgg | 208915 | - | see also 16235 line |
| 36886 | TXNDC2 | NM_032243.3 | 1596 | gtgtcccaacctttcagttttat | 208926 | - | - | - | - |
| 36887 | TXNDC2 | NM_032243.3 | 1225 | cccacccaaggagattgacatcc | 208925 | - | see also 36886 line |
| 36888 | PCYT1A | NM_005017.2 | 647 | ggccgaacaccggattgattttg | 208935 | - | see also 16236 line |
| 36889 | PCYT1A | NM_005017.2 | 646 | tggccgaacaccggattgatttt | 208934 | - | see also 16236 line |
| 36890 | PCYT1A | NM_005017.2 | 648 | gccgaacaccggattgattttgt | 208936 | - | see also 16236 line |
| 36891 | PCYT1B | NM_004845.2 | 420 | gtcgctacgtagacgaagttatc | 208953 | - | see also 16237 line |
| 36892 | PCYT1B | NM_004845.2 | 455 | tggacactcacgccagagtttct | 208956 | - | see also 16237 line |
| 36893 | CMAS | NM_018686.3 | 791 | tacgaaatgcgagctgaacatag | 208978 | - | see also 7561 line |
| 36894 | CMAS | NM_018686.3 | 996 | aagatgctattgggataagttta | 208984 | - | see also 7561 line |
| 36895 | NANS | NM_018946.2 | 659 | ctgcgggtcatctcggaatatca | 209004 | - | see also 7607 line |
| 36896 | NANS | NM_018946.2 | 695 | gacattcccatagggtattctgg | 209007 | - | see also 7607 line |
| 36897 | NMNAT2 | NM_015039.2 | 1037 | agccgacacagaccgaatcatga | 209027 | - | see also 6200 line |
| 36898 | NMNAT2 | NM_015039.2 | 1064 | ctcctcaatactccgcaaataca | 209029 | - | see also 6200 line |
| 36899 | NMNAT2 | NM_015039.2 | 884 | cacggtgatgcggtatgaagaga | 209024 | - | see also 6200 line |
| 36900 | UGP2 | NM_006759.2 | 958 | ggcgggacactcactcaatatga | 209063 | - | see also 4768 line |
| 36901 | UGP2 | NM_006759.2 | 851 | gtgccacagtggatctgtatatt | 209056 | - | see also 4768 line |
| 36902 | UAP1 | NM_003115.3 | 641 | gacaagactcggcgttgcatatc | 209083 | - | see also 16241 line |
| 36903 | UAP1 | NM_003115.3 | 692 | atcccgtaagacactttttcaga | 209087 | - | see also 16241 line |
| 36904 | PCYT2 | NM_002861.1 | 1116 | caccaacaggttggagtatgagg | 209133 | - | see also 1904 line |
| 36905 | PCYT2 | NM_002861.1 | 1050 | gggcatcttccgtcagattgaca | 209132 | - | see also 1904 line |
| 36906 | FPGT | NM_003838.1 | 1170 | agctcggcttacagtccataact | 209168 | - | see also 16243 line |
| 36907 | FPGT | NM_003838.1 | 1172 | ctcggcttacagtccataacttt | 209169 | - | see also 16243 line |
| 36908 | GMPPA | NM_013335.2 | 170 | ccctatgatccaacaccatattg | 209192 | - | see also 5403 line |
| 36909 | GMPPA | NM_013335.2 | 761 | agggcagggccagatatacgtgc | 209203 | - | see also 5403 line |
| 36910 | GMPPB | NM_013334.2 | 1282 | ctgggtgaggacgtcatagttaa | 209210 | - | see also 5402 line |
| 36911 | GMPPB | NM_013334.2 | 1284 | gggtgaggacgtcatagttaatg | 209212 | - | see also 5402 line |
| 36912 | NMNAT3 | NM_178177.2 | 788 | taggtcacgacccaaaaggttac | 209219 | - | see also 16246 line |
| 36913 | NMNAT3 | NM_178177.2 | 789 | aggtcacgacccaaaaggttaca | 209220 | - | see also 16246 line |
| 36914 | NMNAT3 | NM_178177.2 | 433 | aacgacacctatgggaagaaaga | 209218 | - | see also 16246 line |
| 36915 | DLG2 | NM_001364.1 | 1791 | cgctcgatttgaggccaaaatcc | 209253 | - | see also 925 line |
| 36916 | DLG2 | NM_001364.1 | 818 | tgcgtagaagacgacctattttg | 209232 | - | see also 925 line |
| 36917 | DLG2 | NM_001364.1 | 2338 | cccatgaaggatcggatcaatga | 209265 | - | see also 925 line |
| 36918 | DLG3 | NM_021120.1 | 1982 | gacggggatgattgagtctaaca | 209294 | - | see also 8095 line |
| 36919 | MAGI-3 | NM_020965.2 | 1714 | tgcaccaggcgatgttattgtag | 209341 | - | see also 8053 line |
| 36920 | MPP1 | NM_002436.2 | 311 | gtgcggaaagtgcgactcataca | 209378 | - | see also 1568 line |

Figure 46

| | | | | | | | |
|---|---|---|---|---|---|---|---|
| 36921 | MPP1 | NM_002436.2 | 217 | ggctgtatcgcatccattgaata | 209374 | - | see also 1568 line |
| 36922 | MPP3 | NM_001932.2 | 1665 | ggccgacttacatcacaacaagt | 209424 | - | see also 16251 line |
| 36923 | MPP3 | NM_001932.2 | 957 | gggatccatcaccctaaaaatca | 209420 | - | see also 16251 line |
| 36924 | PMVK | NM_006556.2 | 754 | gacgatgctgagtcagaatgtgg | 209431 | - | see also 16252 line |
| 36925 | CKB | NM_001823.3 | 875 | gtctaaggactatgagttcatgt | 209434 | - | see also 1178 line |
| 36926 | CKM | NM_001824.2 | 1058 | tgccgtgggctcagtatttgacg | 209439 | - | see also 1179 line |
| 36927 | CKM | NM_001824.2 | 134 | ccccgacctcagcaaacataaca | 209435 | - | see also 1179 line |
| 36928 | CKM | NM_001824.2 | 834 | tgcgtagggctgcagaagattga | 209438 | - | see also 1179 line |
| 36929 | CKMT1 | NM_020990.2 | 406 | cactggttggacgctagatcagt | 209440 | - | see also 8062 line |
| 36930 | CKMT1 | NM_020990.2 | 765 | acctggctggacgttactatagg | 209444 | - | see also 8062 line |
| 36931 | CKMT2 | NM_001825.1 | 1116 | gtggaatgagcgcctaggataca | 209464 | - | see also 1180 line |
| 36932 | CKMT2 | NM_001825.1 | 1121 | atgagcgcctaggatacacattttg | 209465 | - | see also 1180 line |
| 36933 | CEPT1 | NM_006090.2 | 707 | gcgcactggcaaacgtatgtttc | 209490 | - | see also 4228 line |
| 36934 | CEPT1 | NM_006090.2 | 710 | cactggcaaacgtatgtttctgg | 209492 | - | see also 4228 line |
| 36935 | PIGF | NM_002643.2 | 275 | ctgtcaatctagtactatattta | 209519 | - | see also 1718 line |
| 36936 | PIGF | NM_002643.2 | 420 | tggagcaccactgatagagttgg | 209522 | - | see also 1718 line |
| 36937 | CHPT1 | NM_020244.1 | 617 | tgccgctcgcttaggaacttatc | 209528 | - | see also 7818 line |
| 36938 | CHPT1 | NM_020244.1 | 618 | gccgctcgcttaggaacttatcc | 209529 | - | see also 7818 line |
| 36939 | DPAGT1 | NM_001382.1 | 1188 | ctcgttcgactcttctatgatgt | 209579 | - | see also 950 line |
| 36940 | DPAGT1 | NM_001382.1 | 892 | accgcatacccagactcaatatc | 209573 | - | see also 950 line |
| 36941 | PRPSAP1 | NM_002766.1 | 875 | ggcgcctataagatctatgttat | 209598 | - | see also 16259 line |
| 36942 | PRPSAP1 | NM_002766.1 | 413 | ctggcgaaagcaggtttaactca | 209586 | - | see also 16259 line |
| 36943 | PRPSAP2 | NM_002767.2 | 528 | gcgtgataccctactttccttac | 209609 | - | see also 16260 line |
| 36944 | PRPSAP2 | NM_002767.2 | 1098 | tggcaactcatggcttgttgtct | 209620 | - | see also 16260 line |
| 36945 | TPK1 | NM_022445.2 | 233 | gtgccaaccgcttatatgatatc | 209628 | - | see also 16261 line |
| 36946 | TPK1 | NM_022445.2 | 234 | tgccaaccgcttatatgatatca | 209629 | - | see also 16261 line |
| 36947 | PGK1 | NM_000291.2 | 231 | agccaagtcggtagtccttatga | 209649 | - | see also 16262 line |
| 36948 | PGK1 | NM_000291.2 | 1053 | cactcgggctaagcagattgtgt | 209658 | - | see also 16262 line |
| 36949 | PGK2 | NM_138733.2 | 613 | ctgcacaccgcgctcatagttcc | 209667 | - | see also 16263 line |
| 36950 | PGK2 | NM_138733.2 | 1122 | gggccgttaggagtatttgaatg | 209681 | - | see also 16263 line |
| 36951 | PYCS | NM_002860.2 | 1428 | cacccgaatcgccaaaaacttgg | 209711 | - | see also 1903 line |
| 36952 | PYCS | NM_002860.2 | 1425 | ccgcacccgaatcgccaaaaact | 209710 | - | see also 1903 line |
| 36953 | PYCS | NM_002860.2 | 1692 | ttgccgcctagacaaaatgatag | 209719 | - | see also 1903 line |
| 36954 | MGC11134 | NM_031472.2 | 531 | atgcggtcccattgtgaaatagc | 209747 | - | see also 16265 line |
| 36955 | MGC11134 | NM_031472.2 | 434 | ctggccatccatcctactcaaag | 209742 | - | see also 16265 line |

Figure 46

| | | | | | | | |
|---|---|---|---|---|---|---|---|
| 36956 | B3GALT1 | NM_020981.2 | 1233 | gtgtaggtatcgccgagttatca | 209780 | - | see also 16266 line |
| 36957 | B3GALT1 | NM_020981.2 | 788 | ttgactcctaccataaccttacc | 209769 | - | see also 16266 line |
| 36958 | B3GALT2 | NM_003783.2 | 1536 | tacctaatgcgaggatatgcaacc | 209808 | - | see also 2587 line |
| 36959 | B3GALT2 | NM_003783.2 | 1942 | atgccaacgtaaactacattag | 209830 | - | see also 2587 line |
| 36960 | B3GALT4 | NM_003782.2 | 770 | gacggacgatgatgttgtatgtca | 209832 | - | see also 16268 line |
| 36961 | B3GALT4 | NM_003782.2 | 1350 | atcctgcggtgtcgagcaatagc | 209836 | - | see also 16268 line |
| 36962 | B3GALT5 | NM_006057.1 | 503 | agcatgtacagtctaaatccttt | 209839 | - | see also 16269 line |
| 36963 | B3GALT5 | NM_006057.1 | 1133 | aagagcgtcccatacattaaact | 209856 | - | see also 16269 line |
| 36964 | B3GNT1 | NM_006577.3 | 542 | gtcggtggttacgggttttaaca | 209871 | - | see also 4609 line |
| 36965 | B3GNT1 | NM_006577.3 | 1069 | ctggacctcatcgggataagaag | 209885 | - | see also 4609 line |
| 36966 | B4GALT1 | NM_011497.2 | 715 | aaccaggcgggagcacactatatt | 209910 | - | see also 16271 line |
| 36967 | B4GALT1 | NM_011497.2 | 716 | accaggcgggagcacactatattc | 209911 | - | see also 16271 line |
| 36968 | B4GALT2 | NM_003780.2 | 986 | gaccgcgacaagcataacgaacc | 209939 | - | see also 2585 line |
| 36969 | B4GALT2 | NM_003780.2 | 981 | agcacgacgacgacaagcataac | 209938 | - | see also 2585 line |
| 36970 | B4GALT3 | NM_003779.2 | 412 | ccctcgtgatgtctacagtaacc | 209944 | - | see also 2584 line |
| 36971 | B4GALT3 | NM_003779.2 | 406 | ttctcaccctgtgatgtctaca | 209943 | - | see also 2584 line |
| 36972 | B4GALT4 | NM_003778.2 | 973 | cactggtacggttacgttaca | 209979 | - | see also 16274 line |
| 36973 | B4GALT4 | NM_003778.2 | 897 | acctggtacccgagaatgacttt | 209973 | - | see also 16274 line |
| 36974 | ABO | NM_020469.1 | 16 | cggacgctggccggaaaaccaa | 209988 | - | see also 16275 line |
| 36975 | ABO | NM_020469.1 | 339 | caccaccattggttaactgtgt | 209994 | - | see also 16275 line |
| 36976 | ABO | NM_020469.1 | 47 | cacttcgacctatgatcctttc | 209990 | - | see also 16275 line |
| 36977 | B4GALT7 | NM_007255.1 | 845 | aaggcatcgcagctcaaaaaca | 210000 | - | see also 16276 line |
| 36978 | B4GALT7 | NM_007255.1 | 497 | agcaacacacggactacattgc | 209997 | - | see also 16276 line |
| 36979 | C1GALT1 | NM_020156.1 | 397 | cccagcgttgtaacaaagtgttg | 210013 | - | see also 16277 line |
| 36980 | C1GALT1 | NM_020156.1 | 1074 | accttacctgaacgtatactaaa | 210027 | - | see also 16277 line |
| 36981 | A4GALT | NM_017436.3 | 1252 | gacgcacgagggcatgaaaatgt | 210039 | - | see also 16278 line |
| 36982 | A4GALT | NM_017436.3 | 1161 | tccacgtgtgaacaagaagagc | 210037 | - | see also 16278 line |
| 36983 | B3GALT6 | NM_080605.2 | 389 | gcgcgacgcctacgaaaacctca | 210042 | - | see also 9515 line |
| 36984 | B3GALT6 | NM_080605.2 | 385 | cgctcgcgacgcctacgaaaac | 210041 | - | see also 9515 line |
| 36985 | B3GNT3 | NM_014256.2 | 345 | gccgcagcacgttcagaaacttcc | 210044 | - | see also 16279 line |
| 36986 | B3GNT3 | NM_014256.2 | 1144 | gtgcaccgctcctacctttatga | 210051 | - | see also 16279 line |
| 36987 | B3GNT4 | NM_030765.1 | 128 | ggcggtaggaagtggcctttacc | 210055 | - | see also 16280 line |
| 36988 | B3GNT4 | NM_030765.1 | 847 | caggaacactaaggtcaaatact | 210060 | - | see also 16280 line |
| 36989 | B3GNT5 | NM_032047.2 | 672 | ctgctaccaatacttgattaac | 210079 | - | see also 16281 line |
| 36990 | B3GNT5 | NM_032047.2 | 669 | ggcctcgctaccaatacttgatt | 210077 | - | see also 16281 line |
| 36991 | B3GNT7 | NM_145236.1 | 1244 | accgcaacagccgcatgaacaag | 210117 | - | see also 16282 line |
| 36992 | B3GNT7 | NM_145236.1 | 919 | cccaccaacctgctagaattct | 210115 | - | see also 16282 line |

Figure 46

| 36993 | B4GALT5 | NM_004776.2 | 1207 | cacatacgacgccttgtataaaa | 210143 | - | see also 16283 line |
|---|---|---|---|---|---|---|---|
| 36994 | B4GALT5 | NM_004776.2 | 270 | tccgggacaacgtgagaacaatc | 210120 | - | see also 16283 line |
| 36995 | B4GALT5 | NM_004776.2 | 1205 | atcacatacgacgccttgtataa | 210142 | - | see also 16283 line |
| 36996 | MGC39558 | NM_152490.1 | 1205 | gtcgacacttatcgtaatgttcc | 210170 | - | see also 16285 line |
| 36997 | MGC39558 | NM_152490.1 | 678 | gagttctctaccccatcgttatt | 210157 | - | see also 16285 line |
| 36998 | MGC4655 | NM_033309.1 | 896 | ggccttcgacgacaccttttta | 210190 | - | see also 16286 line |
| 36999 | MGC4655 | NM_033309.1 | 897 | gccttcgacgacacctttttaa | 210191 | - | see also 16286 line |
| 37000 | DSCR5 | NM_016430.2 | 354 | ctcgtgtgggcctttattcctga | 210199 | - | see also 6856 line |
| 37001 | DSCR5 | NM_016430.2 | 387 | aactctttaggtttaacctattg | 210201 | - | see also 6856 line |
| 37002 | DSCR5 | NM_016430.2 | 508 | ctccactcgactccatccataca | 210203 | - | see also 6856 line |
| 37003 | PIGA | NM_002641.1 | 1464 | cacggggtgcctggactaataac | 210250 | - | see also 1714 line |
| 37004 | PIGA | NM_002641.1 | 803 | atcgatttgcttagtggtataat | 210240 | - | see also 1714 line |
| 37005 | PIGC | NM_153747.1 | 833 | cagcacactatccttgaacatgg | 210268 | - | see also 16289 line |
| 37006 | PIGC | NM_153747.1 | 676 | gtgccctagtcttcattactttc | 210265 | - | see also 16289 line |
| 37007 | PIGQ | NM_004204.2 | 869 | acctcacgctaatcttcagtaca | 210281 | - | see also 2813 line |
| 37008 | PIGQ | NM_004204.2 | 1257 | gtccctcctctcggacattatcg | 210282 | - | see also 2813 line |
| 37009 | C2GNT3 | NM_016591.1 | 1075 | aactgttcgggtatctatgaaca | 210293 | - | see also 16291 line |
| 37010 | C2GNT3 | NM_016591.1 | 1950 | gagtaagactcgccttgtcaagt | 210318 | - | see also 16291 line |
| 37011 | GCNT2 | NM_001491.2 | 1744 | atctatggaaacggagacttaaa | 210353 | - | see also 16292 line |
| 37012 | GCNT2 | NM_001491.2 | 1759 | gacttaaagtggctggttaattc | 210354 | - | see also 16292 line |
| 37013 | GCNT3 | NM_004751.1 | 1139 | aacccgctggaaatatcactttg | 210411 | - | see also 16293 line |
| 37014 | GCNT3 | NM_004751.1 | 1470 | atcgataagggtgctccttatgc | 210422 | - | see also 16293 line |
| 37015 | FLJ20277 | NM_017739.1 | 1861 | acctacgtggcctttattcgaat | 210443 | - | see also 7128 line |
| 37016 | FLJ20277 | NM_017739.1 | 1648 | atcgtcggcctcaacatgaatgg | 210440 | - | see also 7128 line |
| 37017 | MGAT1 | NM_002406.2 | 1735 | agctggctaccggggtattgtca | 210448 | - | see also 16295 line |
| 37018 | MGAT1 | NM_002406.2 | 1799 | ccgacgtgggagggctatgatcc | 210450 | - | see also 16295 line |
| 37019 | MGAT4A | NM_012214.1 | 1401 | accgatagctggagactacatct | 210482 | - | see also 5202 line |
| 37020 | MGAT4B | NM_014275.2 | 1108 | aacctgccggatccgcttcaaacc | 210494 | - | see also 5576 line |
| 37021 | MGAT3 | NM_002409.3 | 1206 | ggcccgtgacggcgtcctttcc | 210504 | - | see also 1561 line |
| 37022 | MGAT3 | NM_002409.3 | 1666 | gaccccagcgagcacatgtatgc | 210508 | - | see also 1561 line |
| 37023 | GCNT1 | NM_001490.3 | 955 | tccgaggattcctatttagctgc | 210538 | - | see also 16298 line |
| 37024 | GCNT1 | NM_001490.3 | 747 | gacacctgacgactatataaaca | 210522 | - | see also 16298 line |
| 37025 | B3GNT6 | NM_006876.1 | 1262 | atcctatatcgccagttcaaaca | 210570 | - | see also 16299 line |
| 37026 | B3GNT6 | NM_006876.1 | 1250 | cagcacaataagatcctatatcg | 210569 | - | see also 16299 line |
| 37027 | MGAT2 | NM_002408.2 | 1135 | cccgactccttcggccattatag | 210584 | - | see also 1557 line |

Figure 46

| 37028 | MGAT2 | NM_002408.2 | 1136 | ccgactccttcggccattataga | 210585 | - | see also 1557 line |
|---|---|---|---|---|---|---|---|
| 37029 | MGAT5 | NM_002410.2 | 4376 | tggaagctatcgcaaatggatgt | 210650 | - | see also 16301 line |
| 37030 | MGAT5 | NM_002410.2 | 4490 | agcatccttacgctgaagttttc | 210652 | - | see also 16301 line |
| 37031 | alpha4GnT | NM_016161.1 | 1134 | gacttacagggacctgattaaag | 210677 | - | see also 16302 line |
| 37032 | alpha4GnT | NM_016161.1 | 874 | ttgagggtatggtgtaaacttga | 210672 | - | see also 16302 line |
| 37033 | XYLT1 | NM_022166.1 | 1402 | ctcccgataccgagatatgaatt | 210682 | - | see also 16303 line |
| 37034 | XYLT1 | NM_022166.1 | 1403 | tcccgataccgagatatgaattt | 210683 | - | see also 16303 line |
| 37035 | XYLT2 | NM_022167.1 | 2410 | gccaccccacaacgagtacatgg | 210712 | - | see also 8320 line |
| 37036 | XYLT2 | NM_022167.1 | 949 | ggcccagggctatgataacgtgc | 210702 | - | see also 8320 line |
| 37037 | FUT1 | NM_000148.1 | 963 | aggctacaccgtggaaagacttt | 210722 | - | see also 16305 line |
| 37038 | FUT1 | NM_000148.1 | 964 | ggctacaccgtggaaagactttg | 210723 | - | see also 16305 line |
| 37039 | FUT2 | NM_000511.1 | 626 | tccatgtcatgccaaaagtgtgg | 210728 | - | see also 16306 line |
| 37040 | FUT2 | NM_000511.1 | 750 | ctggtgtcgggagaacattgaca | 210730 | - | see also 16306 line |
| 37041 | FUT8 | NM_004480.3 | 1700 | agctggactgcacaatcgataca | 210763 | - | see also 3030 line |
| 37042 | FUT8 | NM_004480.3 | 2034 | ttgggaaggacgggcctatatcc | 210772 | - | see also 3030 line |
| 37043 | FUT10 | NM_032664.3 | 520 | cacctacgcatcttaattcattt | 210790 | - | see also 16308 line |
| 37044 | FUT10 | NM_032664.3 | 930 | ctgaagtcactccgatacctagt | 210800 | - | see also 16308 line |
| 37045 | FUT11 | NM_173540.1 | 1468 | gacgaaattttgggattacctac | 210836 | - | see also 16309 line |
| 37046 | FUT11 | NM_173540.1 | 1114 | gggcatcaccaaccaatttcttc | 210832 | - | see also 16309 line |
| 37047 | FUT4 | NM_002033.1 | 1042 | agcgctgggtttggatgaacttc | 210840 | - | see also 16310 line |
| 37048 | FUT4 | NM_002033.1 | 1418 | ctcgcagcacctggattatatca | 210842 | - | see also 16310 line |
| 37049 | FUT7 | NM_004479.1 | 1052 | caccgactggcgggaacgttct | 210848 | - | see also 16311 line |
| 37050 | FUT7 | NM_004479.1 | 999 | agccgataccaacgcttctttgc | 210847 | - | see also 16311 line |
| 37051 | FUT9 | NM_006581.2 | 785 | gacttaccgccgtgattcagata | 210863 | - | see also 4610 line |
| 37052 | FUT9 | NM_006581.2 | 599 | aacggaccgttcactgtacaaca | 210855 | - | see also 4610 line |
| 37053 | UGT1A10 | NM_019075.1 | 1095 | tgcgaacaacacgatacttgtta | 329678 | - | see also 16313 line |
| 37054 | UGT1A10 | NM_019075.1 | 1117 | aagtggctaccccaaaacgatct | 329680 | - | see also 16313 line |
| 37055 | UGT2A1 | NM_006798.1 | 527 | gtggcgatatagtagctttaaaa | 210904 | - | see also 16314 line |
| 37056 | UGT2A1 | NM_006798.1 | 659 | aactcaccgaccaaatgtcttc | 210909 | - | see also 16314 line |
| 37057 | UGT2B10 | NM_001075.2 | 25 | gactacagttctgctgatacaac | 210930 | - | see also 16315 line |
| 37058 | UGT2B10 | NM_001075.2 | 28 | tacagttctgctgatacaactca | 210931 | - | see also 16315 line |
| 37059 | UGT2B15 | NM_001076.1 | 1203 | tcccttgtttgcggatcaacatg | 210944 | - | see also 16317 line |
| 37060 | UGT2B15 | NM_001076.1 | 206 | ctcttgtcaatgccagtaaatca | 210937 | - | see also 16317 line |
| 37061 | UGT2B17 | NM_001077.1 | 236 | ttcttgtcaatgccagtaaatca | 210937 | - | see also 16318 line |
| 37062 | UGT2B4 | NM_021139.1 | 512 | ctgctggccgagttacttaaaat | 210955 | - | see also 16320 line |
| 37063 | UGT2B4 | NM_021139.1 | 566 | ggctacgcaattgaaaagcatag | 210959 | - | see also 16320 line |
| 37064 | UGT2B7 | NM_001074.1 | 221 | atccgctcttaaaattgaaattt | 210960 | - | see also 16321 line |

Figure 46

| | | | | | | | |
|---|---|---|---|---|---|---|---|
| 37065 | UGT2B7 | NM_001074.1 | 349 | tacaggaaatcatgtcaatatt | 210963 | - | see also 16321 line |
| 37066 | GALNT2 | NM_004481.2 | 1359 | aactaactgcctcgacaacttgg | 210985 | - | see also 3033 line |
| 37067 | GALNT2 | NM_004481.2 | 1502 | accgggcaacgggctccttata | 210987 | - | see also 3033 line |
| 37068 | GALNT3 | NM_004482.2 | 897 | ggctgtcgtaagtccagatattg | 211029 | - | see also 3035 line |
| 37069 | GALNT3 | NM_004482.2 | 587 | aagcgtggtccacgttgcttaga | 211019 | - | see also 3035 line |
| 37070 | GALNT5 | NM_014568.1 | 2557 | atcgaccaagggctagatgttgg | 211114 | - | see also 5755 line |
| 37071 | GALNT5 | NM_014568.1 | 2407 | tgctcccgagtgggccatatatt | 211109 | - | see also 5755 line |
| 37072 | GALNT6 | NM_007210.2 | 1102 | cacatcggtacctatgataatca | 211142 | - | see also 5014 line |
| 37073 | GALNT6 | NM_007210.2 | 897 | caccatcgaccttaatactttg | 211136 | - | see also 5014 line |
| 37074 | GALNT6 | NM_007210.2 | 898 | accatcgaccttaatactttga | 211137 | - | see also 5014 line |
| 37075 | GALNT7 | NM_017423.1 | 651 | ctcacttcgagcgttgtcattgt | 211165 | - | see also 6998 line |
| 37076 | GALNT7 | NM_017423.1 | 523 | aagcaattcaaggcagccattaaa | 211162 | - | see also 6998 line |
| 37077 | GALNT8 | NM_017417.1 | 685 | aaegggccatcaaccagatcatc | 211221 | - | see also 16326 line |
| 37078 | GALNT8 | NM_017417.1 | 583 | ccgacacgcgagactacagatgt | 211217 | - | see also 16326 line |
| 37079 | GALNT9 | NM_021808.2 | 536 | cccggagatggagggtctacaaca | 211245 | - | see also 8175 line |
| 37080 | GALNT9 | NM_021808.2 | 326 | gccctacaacgacgacattgact | 211244 | - | see also 8175 line |
| 37081 | GALGT | NM_001478.2 | 1726 | caccgacgcgtgttaacttct | 211261 | - | see also 16328 line |
| 37082 | GALGT | NM_001478.2 | 1399 | ccctacgtggaacactatctca | 211257 | - | see also 16328 line |
| 37083 | DDOST | NM_005216.3 | 1123 | tccgcattgatcctttgtgagg | 211280 | - | see also 16329 line |
| 37084 | DDOST | NM_005216.3 | 603 | accatcgttcggaaatcatctct | 211276 | - | see also 16329 line |
| 37085 | RPN2 | NM_002951.2 | 2040 | tgctatgctgggactcatgtatg | 211321 | - | see also 1993 line |
| 37086 | RPN2 | NM_002951.2 | 505 | acctacatcagatctaaccttga | 211288 | - | see also 1993 line |
| 37087 | ALG5 | NM_013338.3 | 89 | gccgcagccctcgtactgatttc | 211323 | - | see also 5404 line |
| 37088 | ALG5 | NM_013338.3 | 88 | ggccgcagccctcgtactgattt | 211322 | - | see also 5404 line |
| 37089 | ALG6 | NM_013339.2 | 352 | tgctggtaaaccgcctatgtttg | 211351 | - | see also 16332 line |
| 37090 | ALG6 | NM_013339.2 | 361 | acgcctatgtttggtgattatg | 211352 | - | see also 16332 line |
| 37091 | ALG6 | NM_013339.2 | 1143 | tgccacgtcacatccaattaata | 211387 | - | see also 16332 line |
| 37092 | ITM1 | NM_152713.2 | 1243 | atgcccggattttatcatcatg | 211434 | - | see also 9930 line |
| 37093 | ITM1 | NM_152713.2 | 576 | gttggctgctcctatgataatga | 211416 | - | see also 9930 line |
| 37094 | SIMP | NM_178862.1 | 308 | accgtgtttaactatagatca | 211465 | - | see also 16334 line |
| 37095 | SIMP | NM_178862.1 | 304 | ttgaccgtggtttaactatag | 211462 | - | see also 16334 line |
| 37096 | PYGB | NM_002862.3 | 1988 | atcggcgacgtcgtcaatcatga | 211567 | - | see also 16335 line |
| 37097 | PYGB | NM_002862.3 | 1497 | tcgtgaaacagtcggtctttaag | 211563 | - | see also 16335 line |
| 37098 | PYGL | NM_002863.1 | 1806 | cacgatgtacaaccgcattaaga | 211599 | - | see also 1905 line |
| 37099 | PYGL | NM_002863.1 | 523 | tacggcattcggtatgaatatgg | 211578 | - | see also 1905 line |
| 37100 | PYGL | NM_002863.1 | 2073 | ctcggggacaggcaatatgaagt | 211611 | - | see also 1905 line |
| 37101 | PYGM | NM_005609.1 | 1510 | gcctggctatcgcaacaatgttg | 211640 | - | see also 3897 line |

Figure 46

| | | | | | | | |
|---|---|---|---|---|---|---|---|
| 37102 | PYGM | NM_005609.1 | 1513 | tggctatcgcaacaatgttgtca | 211641 | - | see also 3897 line |
| 37103 | MTAP | NM_002451.2 | 143 | accgccgtgaagattggaataat | 211660 | - | see also 16338 line |
| 37104 | MTAP | NM_002451.2 | 764 | atcgccatggcgacagattatga | 211678 | - | see also 16338 line |
| 37105 | MTAP | NM_002451.2 | 636 | tcgagggacctcgttttagctcc | 211676 | - | see also 16338 line |
| 37106 | NP | NM_000270.1 | 176 | caccgacctcaagttgcaataat | 211687 | - | see also 16339 line |
| 37107 | NP | NM_000270.1 | 177 | accgacctcaagttgcaataatc | 211688 | - | see also 16339 line |
| 37108 | GYG | NM_004130.2 | 452 | ttccggagtcttcgtttatcagc | 211704 | - | see also 2787 line |
| 37109 | GYG | NM_004130.2 | 643 | acctcccggcatttaaagtgttt | 211712 | - | see also 2787 line |
| 37110 | GYG | NM_004130.2 | 321 | tggtcgcttacacagtattcaaa | 211700 | - | see also 2787 line |
| 37111 | GYG2 | NM_003918.1 | 757 | ccccggatggccggattgcttca | 211731 | - | see also 16341 line |
| 37112 | GYG2 | NM_003918.1 | 1002 | gtgcaaaggtcgtccactttttg | 211738 | - | see also 16341 line |
| 37113 | UGCGL1 | NM_020120.1 | 307 | atcatgacggtaccgattattcc | 211764 | - | see also 16342 line |
| 37114 | UGCGL1 | NM_020120.1 | 2357 | tggacggcagttactgtatgatg | 211840 | - | see also 16342 line |
| 37115 | GYS1 | NM_002103.3 | 1561 | cgccgaatcggcctcttcaatag | 211913 | - | see also 16343 line |
| 37116 | GYS1 | NM_002103.3 | 1283 | tgccagcgcggaccaacaatttc | 211902 | - | see also 16343 line |
| 37117 | UGCG | NM_003358.1 | 1354 | tccgcgaatccatgacaatatac | 211955 | - | see also 2263 line |
| 37118 | UGCG | NM_003358.1 | 1353 | atccgcgaatccatgacaatata | 211954 | - | see also 2263 line |
| 37119 | POMT2 | NM_013382.3 | 1608 | gtcgaattcgcttcatccatttg | 211979 | - | see also 5415 line |
| 37120 | POMT2 | NM_013382.3 | 1511 | accggctatggcataaatggaac | 211971 | - | see also 5415 line |
| 37121 | SDF2 | NM_006923.2 | 347 | tccatagtcaccacttcacttca | 211993 | - | see also 16346 line |
| 37122 | DPM1 | NM_003859.1 | 666 | tactattggcgaggttccaatat | 212012 | - | see also 2623 line |
| 37123 | DPM1 | NM_003859.1 | 57 | gcgcagtccacgacagaacaaat | 211994 | - | see also 2623 line |
| 37124 | AD-017 | NM_018446.1 | 386 | aacactcgctccaatgtgatttt | 212022 | - | see also 7481 line |
| 37125 | AD-017 | NM_018446.1 | 1190 | ttcaacctaatccgaagatatac | 212060 | - | see also 7481 line |
| 37126 | FLJ21934 | NM_024743.1 | 1124 | gtcattaccgattcctcttataa | 212077 | - | see also 16349 line |
| 37127 | FLJ21934 | NM_024743.1 | 257 | accaactacgatgtaatgcttat | 212069 | - | see also 16349 line |
| 37128 | FLJ21934 | NM_024743.1 | 260 | aactacgatgtaatgcttataga | 212070 | - | see also 16349 line |
| 37129 | FLJ34658 | NM_152404.2 | 1406 | gccgacacactgacacttacaat | 212089 | - | see also 16350 line |
| 37130 | FLJ34658 | NM_152404.2 | 297 | aagagcatggtcataatgtgact | 212078 | - | see also 16350 line |
| 37131 | FLJ35155 | NM_152531.3 | 486 | ggcgcacgaggtgcttaaccttc | 212091 | - | see also 16351 line |
| 37132 | FLJ35155 | NM_152531.3 | 711 | ctcggtcgccatgcatcagatca | 212094 | - | see also 16351 line |
| 37133 | GYLTL1B | NM_152312.2 | 638 | ccggcctctatgggctaatgaag | 212104 | - | see also 9839 line |
| 37134 | GYLTL1B | NM_152312.2 | 568 | gtccgtgtcagcttttatcatgc | 212103 | - | see also 9839 line |
| 37135 | HGNT-IV-H | NM_013244.2 | 876 | aagcggtatcttacaattggact | 212115 | - | see also 5360 line |
| 37136 | HGNT-IV-H | NM_013244.2 | 1830 | aacgttatgcctagcaaacaaag | 212148 | - | see also 5360 line |

Figure 46

| 37137 | LARGE | NM_004737.2 | 2534 | gccgattttgagccgtatgttgt | 212171 | - | see also 3263 line |
|---|---|---|---|---|---|---|---|
| 37138 | LARGE | NM_004737.2 | 979 | gacaatccacgttgctattgtct | 212158 | - | see also 3263 line |
| 37139 | LOC167127 | NM_174914.2 | 1062 | accccaaggggtgatatggaagt | 212183 | - | see also 16354 line |
| 37140 | LOC167127 | NM_174914.2 | 253 | accatgcttaaccacaaaagagg | 212176 | - | see also 16354 line |
| 37141 | LOC83468 | NM_031302.2 | 676 | ctctgactcgaatacgaaaatgg | 212195 | - | see also 16355 line |
| 37142 | LOC83468 | NM_031302.2 | 850 | aacacgagaaagtcatctatttg | 212201 | - | see also 16355 line |
| 37143 | UGCGL2 | NM_020121.2 | 1193 | aggcgatgctcgtctatttataa | 212248 | - | see also 16356 line |
| 37144 | UGCGL2 | NM_020121.2 | 1192 | caggcgatgctcgtctatttata | 212247 | - | see also 16356 line |
| 37145 | ADPRTL1 | NM_006437.2 | 3258 | gccactctgtctccgtcaaatgg | 212447 | - | see also 16357 line |
| 37146 | ADPRTL1 | NM_006437.2 | 1542 | gtgattcgctcagtacaagtatc | 212387 | - | see also 16357 line |
| 37147 | ADPRTL3 | NM_005485.2 | 558 | gggagaggtcggccagtcaaaga | 212477 | - | see also 16358 line |
| 37148 | ADPRTL3 | NM_005485.2 | 363 | acccgcagagaagcgcataatcc | 212475 | - | see also 16358 line |
| 37149 | ART1 | NM_004314.1 | 963 | gggccttgccatctggataattc | 212494 | - | see also 16359 line |
| 37150 | ART3 | NM_001179.2 | 631 | tacatttggagggctaaaccaag | 212509 | - | see also 16360 line |
| 37151 | ART3 | NM_001179.2 | 832 | ggctggcaataaccttatccttc | 212514 | - | see also 16360 line |
| 37152 | ART3 | NM_001179.2 | 1196 | cagtttgggatggtcatcatttt | 212516 | - | see also 16360 line |
| 37153 | TNKS2 | NM_025235.2 | 583 | tgggcggaaagacgtagttgaat | 212519 | - | see also 16361 line |
| 37154 | TNKS2 | NM_025235.2 | 3448 | tgccaatgaacgaatgctatttc | 212605 | - | see also 16361 line |
| 37155 | TNKS2 | NM_025235.2 | 3447 | atgccaatgaacgaatgctattt | 212604 | - | see also 16361 line |
| 37156 | ART5 | NM_053017.2 | 342 | tggcgctggcggctttgatgatc | 212615 | - | see also 16362 line |
| 37157 | ART5 | NM_053017.2 | 902 | aaggcagtggcccacagatttgg | 212618 | - | see also 16362 line |
| 37158 | DO | NM_021071.1 | 690 | cacaggggccaccattcgatttg | 212648 | - | see also 16363 line |
| 37159 | DO | NM_021071.1 | 970 | atccctgatcctatagctattgc | 212656 | - | see also 16363 line |
| 37160 | DO | NM_021071.1 | 303 | ctgtagcaaacaggttatggaga | 212632 | - | see also 16363 line |
| 37161 | PRTFDC1 | NM_020200.5 | 564 | gacggctttagacctgactatgc | 212683 | - | see also 16364 line |
| 37162 | PRTFDC1 | NM_020200.5 | 61 | acgggcgaggcgtcgtgattatg | 212657 | - | see also 16364 line |
| 37163 | QTRT1 | NM_031209.1 | 392 | ccccctacgacggcaatgagacc | 212694 | - | see also 8990 line |
| 37164 | QTRT1 | NM_031209.1 | 275 | acggtctccacggcttcatgaat | 212691 | - | see also 8990 line |
| 37165 | QTRTD1 | NM_024638.2 | 1351 | tggccggagtcctgcttatgatg | 212720 | - | see also 16366 line |
| 37166 | QTRTD1 | NM_024638.2 | 1006 | aggtgctcgagtgtattgaaaga | 212712 | - | see also 16366 line |
| 37167 | UPP1 | NM_003364.2 | 998 | tccggaaaacggaccttaacaag | 212739 | - | see also 16367 line |
| 37168 | UPP1 | NM_003364.2 | 512 | atgattgccccgtcagactttta | 212726 | - | see also 16367 line |
| 37169 | UPP1 | NM_003364.2 | 913 | cccggcactgtggtcataacaga | 212737 | - | see also 16367 line |
| 37170 | UMPS | NM_000373.1 | 723 | gtggcagcgaatcataatggttc | 212753 | - | see also 299 line |
| 37171 | UMPS | NM_000373.1 | 1427 | acgaggttccgatatcatcattg | 212771 | - | see also 299 line |

Figure 46

| | | | | | | | | transferase、transferring glycosyl groups | |
|---|---|---|---|---|---|---|---|---|---|
| 37172 | QPRT | NM_014298.2 | 227 | tgccatatttacccaactcaact | 212775 | - | - | | - |
| 37173 | SIAT4A | NM_003033.2 | 1989 | caccttggcctccatcaataaaa | 212778 | - | see also 16369 line | | |
| 37174 | SIAT4A | NM_003033.2 | 1326 | gcgggagaagaagcccaataact | 212776 | - | see also 16369 line | | |
| 37175 | SIAT4B | NM_006927.2 | 1382 | gtgcctccgactggtttgacagc | 212779 | - | see also 16370 line | | |
| 37176 | SIAT4B | NM_006927.2 | 1402 | agccactttgacggtaacatttc | 212781 | - | see also 16370 line | | |
| 37177 | SIAT4C | NM_006278.1 | 355 | ttcctgcggcttgaggattattt | 212787 | - | see also 4368 line | | |
| 37178 | SIAT7C | NM_152996.1 | 688 | tccgaatgcccaaatatacgtga | 212822 | - | see also 9971 line | | |
| 37179 | SIAT7D | NM_014403.3 | 663 | ctgctgctgcgcaactattcaca | 212837 | - | see also 16373 line | | |
| 37180 | SIAT7D | NM_014403.3 | 698 | ggcccgagacacgctctacatgg | 212839 | - | see also 16373 line | | |
| 37181 | SIAT1 | NM_003032.2 | 1118 | taccagaatccggattataattt | 212863 | - | see also 16374 line | | |
| 37182 | SIAT1 | NM_003032.2 | 1357 | gactgacgtgtgctactactacc | 212869 | - | see also 16374 line | | |
| 37183 | SIAT8A | NM_003034.2 | 798 | tggtatgacggggagtttttata | 212878 | - | see also 16375 line | | |
| 37184 | SIAT8A | NM_003034.2 | 933 | ggctgtggccgtcaaatagatga | 212884 | - | see also 16375 line | | |
| 37185 | SIAT9 | NM_003896.2 | 750 | accagttcgatgttgtgataagg | 212908 | - | see also 2653 line | | |
| 37186 | SIAT9 | NM_003896.2 | 682 | ctgtcggcgctgtgtggttattg | 212904 | - | see also 2653 line | | |
| 37187 | SIAT7A | NM_018414.2 | 1693 | ggccatgagcgcttttctgatca | 212940 | - | see also 16377 line | | |
| 37188 | SIAT7A | NM_018414.2 | 1590 | tggtgcccactggaggatatacc | 212937 | - | see also 16377 line | | |
| 37189 | SIAT6 | NM_006279.2 | 1271 | ctcgcgtcatcactgatctaagc | 212958 | - | see also 4370 line | | |
| 37190 | SIAT6 | NM_006279.2 | 1225 | cacaatatccagcgagagaaaga | 212957 | - | see also 4370 line | | |
| 37191 | SIAT6 | NM_006279.2 | 227 | ttctggtactgggattttttgtat | 212944 | - | see also 4370 line | | |
| 37192 | SIAT10 | NM_006100.2 | 873 | atcgactcctatgatgtaataat | 212980 | - | see also 16378 line | | |
| 37193 | SIAT10 | NM_006100.2 | 528 | tgcatattatggggaacgaatgt | 212965 | - | see also 16378 line | | |
| 37194 | SIAT7E | NM_030965.1 | 1048 | gggcagtcatcaccgctttatca | 213001 | - | see also 16379 line | | |
| 37195 | SIAT7E | NM_030965.1 | 1091 | aactgggcacggacattcaatat | 213002 | - | see also 16379 line | | |
| 37196 | SIAT8B | NM_006011.2 | 998 | acccagtcaagtaccactattat | 213019 | - | see also 4187 line | | |
| 37197 | SIAT8B | NM_006011.2 | 972 | gccctttccgctggatcagaacc | 213018 | - | see also 4187 line | | |
| 37198 | SIAT8C | NM_015879.1 | 2237 | ttcgtgcccattacgaattctct | 213029 | - | see also 6552 line | | |
| 37199 | SIAT8C | NM_015879.1 | 2191 | cgcgggattccggtcacaatttg | 213025 | - | see also 6552 line | | |
| 37200 | SIAT8C | NM_015879.1 | 2233 | gtcattcgtgcccattacgaatt | 213028 | - | see also 6552 line | | |
| 37201 | SIAT8D | NM_005668.3 | 445 | gtcggtcacttgtcaatagctct | 213068 | - | see also 3938 line | | |
| 37202 | SIAT8D | NM_005668.3 | 441 | ttgagtcggtcacttgtcaatag | 213067 | - | see also 3938 line | | |
| 37203 | SIAT8E | NM_013305.2 | 1327 | atcgccgcaagctgtctactact | 213111 | - | see also 16383 line | | |
| 37204 | SIAT8E | NM_013305.2 | 538 | ctcggccaaccgggatttgttgg | 213094 | - | see also 16383 line | | |
| 37205 | SIAT8E | NM_013305.2 | 990 | accggtcccagtttaagaagtgt | 213108 | - | see also 16383 line | | |
| 37206 | ST6GalII | NM_032528.1 | 204 | aacgaatgcttttcggaatattc | 213116 | - | see also 16384 line | | |

Figure 46

| | | | | | | | |
|---|---|---|---|---|---|---|---|
| 37207 | ST6GalII | NM_032528.1 | 1150 | tgctcctacacgtggttatgaga | 213131 | - | see also 16384 line |
| 37208 | ST6GALNAC6 | NM_013443.3 | 208 | gacgccggagagaaatgagtagc | 213143 | - | see also 16385 line |
| 37209 | ST6GALNAC6 | NM_013443.3 | 308 | tgccaatgaggtcttccattacg | 213145 | - | see also 16385 line |
| 37210 | STHM | NM_006456.1 | 811 | aggccgcacgcctattttggacc | 213167 | - | see also 16386 line |
| 37211 | STHM | NM_006456.1 | 808 | gacaggccgcacgcctattttgg | 213166 | - | see also 16386 line |
| 37212 | STHM | NM_006456.1 | 732 | ctcagacatccgcgactatgtga | 213162 | - | see also 16386 line |
| 37213 | ALG12 | NM_024105.2 | 473 | tacgtgctttcgctgttagaaat | 213173 | - | see also 16387 line |
| 37214 | ALG12 | NM_024105.2 | 895 | gacggttgctgtggactcttatt | 213181 | - | see also 16387 line |
| 37215 | ALG12 | NM_024105.2 | 552 | ttggactctggacgttacaaaag | 213177 | - | see also 16387 line |
| 37216 | ALG3 | NM_005787.3 | 325 | atctttatggggttgtactatgc | 213192 | - | see also 4045 line |
| 37217 | ALG3 | NM_005787.3 | 239 | tagaaggcgtcatcaatggtacc | 213187 | - | see also 4045 line |
| 37218 | ALG8 | NM_024079.2 | 716 | aaccagatgggtctattcgatgg | 213222 | - | see also 8582 line |
| 37219 | ALG8 | NM_024079.2 | 1250 | gagacgcttcgattttctgatt | 213237 | - | see also 8582 line |
| 37220 | ALG8 | NM_024079.2 | 69 | cacgggtactggcaattggtttt | 213197 | - | see also 8582 line |
| 37221 | C1GALT2 | NM_152692.2 | 445 | cagtaagagcttcgagtatact | 213255 | - | see also 16390 line |
| 37222 | C1GALT2 | NM_152692.2 | 638 | tacgcctttgataagtatagaa | 213261 | - | see also 16390 line |
| 37223 | DPM3 | NM_018973.3 | 339 | ggctatcgtgtggccactttca | 213284 | - | - | - | - |
| 37224 | DPM3 | NM_018973.3 | 344 | tcgtgtggccacttttcatgact | 213285 | - | see also 37223 line |
| 37225 | EXT1 | NM_000127.1 | 1615 | tgccttatatcacgtccataacg | 213304 | - | see also 100 line |
| 37226 | EXT1 | NM_000127.1 | 2580 | aggagcggtggggatacacatca | 213340 | - | see also 100 line |
| 37227 | EXT2 | NM_000401.1 | 490 | gtgtgcgtcggtcaagtataata | 213347 | - | see also 321 line |
| 37228 | EXT2 | NM_000401.1 | 1677 | aagcgtacttccagtcaattaaa | 213383 | - | see also 321 line |
| 37229 | EXTL1 | NM_004455.1 | 1142 | gggcgatggccttaaggtattcg | 213409 | - | see also 16394 line |
| 37230 | EXTL1 | NM_004455.1 | 2302 | gtgatcgcttctacccatatagc | 213417 | - | see also 16394 line |
| 37231 | EXTL2 | NM_001439.1 | 1152 | cacgctctgcagaggtcttattg | 213456 | - | see also 16395 line |
| 37232 | EXTL2 | NM_001439.1 | 432 | ctcatgttgcgtagggaaataaa | 213438 | - | see also 16395 line |
| 37233 | EXTL3 | NM_001440.1 | 744 | cccgctcatcgcccactattacc | 213462 | - | see also 1009 line |
| 37234 | EXTL3 | NM_001440.1 | 743 | tcccgctcatcgcccactattac | 213461 | - | see also 1009 line |
| 37235 | HAS1 | NM_001523.1 | 951 | agcggtcctctaggcctatatag | 213500 | - | see also 16397 line |
| 37236 | HAS1 | NM_001523.1 | 827 | tggggacgtgcggatccttaacc | 213499 | - | see also 16397 line |
| 37237 | HAS2 | NM_005328.1 | 1664 | acctacgaagcgattatcactgg | 213544 | - | see also 3713 line |
| 37238 | HAS2 | NM_005328.1 | 1779 | tagtaggtctcataaaatcatct | 213551 | - | see also 3713 line |
| 37239 | MFNG | NM_002405.2 | 869 | caccatgggctatatcattgagt | 213590 | - | see also 16399 line |
| 37240 | MFNG | NM_002405.2 | 1009 | aggggaaactcaacgtcattaag | 213591 | - | see also 16399 line |
| 37241 | SMP3 | NM_025163.2 | 1337 | tccccctggtcctgctttgtagt | 213596 | - | see also 16400 line |

Figure 46

| | | | | | | | |
|---|---|---|---|---|---|---|---|
| 37242 | SMP3 | NM_025163.2 | 882 | agccacaaaccctggtttgaagt | 213594 | - | see also 16400 line |
| 37243 | BAT8 | NM_006709.2 | 2598 | accatgactgcgtgctgttattc | 213615 | - | see also 16401 line |
| 37244 | BAT8 | NM_006709.2 | 2507 | atcgccgagtccttcgaatgc | 213614 | - | see also 16401 line |
| 37245 | SET7 | NM_030648.1 | 1198 | cccgttttgggccatcaaatg | 213649 | - | see also 8921 line |
| 37246 | SET7 | NM_030648.1 | 971 | ctgtgggacctaatactgttatg | 213642 | - | see also 8921 line |
| 37247 | C20orf36 | NM_018257.1 | 233 | atcgatcgtcagacattatct | 213653 | - | see also 16403 line |
| 37248 | C20orf36 | NM_018257.1 | 600 | ctcagtatgatcgtgtatactgt | 213666 | - | see also 16403 line |
| 37249 | C20orf36 | NM_018257.1 | 981 | gtcgaatggaaacgatttgtcttt | 213674 | - | see also 16403 line |
| 37250 | LOC115294 | NM_052937.1 | 663 | tgcctatagaggatcagttaaca | 213696 | - | see also 9460 line |
| 37251 | LOC115294 | NM_052937.1 | 845 | tacattcgacgcacacttagaaa | 213704 | - | see also 9460 line |
| 37252 | PCMT1 | NM_005389.1 | 180 | accgctcccactatgcaaaatgt | 213716 | - | see also 16405 line |
| 37253 | PCMT1 | NM_005389.1 | 573 | aggcgctaatagatcagttaaag | 213728 | - | see also 16405 line |
| 37254 | BHMT | NM_001713.1 | 58 | aggggcatcctagaaacgtttaaat | 213739 | - | see also 16406 line |
| 37255 | BHMT | NM_001713.1 | 57 | aaagggcatcctagaaacgtttaaa | 213738 | - | see also 16406 line |
| 37256 | BHMT2 | NM_017614.3 | 185 | cgcagttcgtcaacttcacatgg | 213759 | - | see also 16407 line |
| 37257 | BHMT2 | NM_017614.3 | 215 | gagagcaggatcaaargtcatgc | 213762 | - | see also 16407 line |
| 37258 | MTR | NM_000254.1 | 720 | ctctggtccgactaataagaca | 213781 | - | see also 16408 line |
| 37259 | MTR | NM_000254.1 | 960 | cagggacgatcggttgataaaagt | 213790 | - | see also 16408 line |
| 37260 | MTR | NM_000254.1 | 2297 | gtcgaagaacgccttgagtatgc | 213817 | - | see also 16408 line |
| 37261 | HSA9761 | NM_014473.1 | 499 | ctgttgctacatcgaactttt | 213868 | - | see also 5699 line |
| 37262 | HSA9761 | NM_014473.1 | 556 | gccctccgactggttgcaaaacc | 213872 | - | see also 5699 line |
| 37263 | TFB1M | NM_016020.1 | 120 | cacgattcgagaaatcattaagt | 213894 | - | see also 6621 line |
| 37264 | TFB1M | NM_016020.1 | 647 | agcgtagtcgcctctctgttatg | 213915 | - | see also 6621 line |
| 37265 | FLJ10140 | NM_018006.2 | 765 | gcctcgactggtcactttattt | 213948 | - | see also 16412 line |
| 37266 | FLJ10140 | NM_018006.2 | 626 | tggggggattaacgaaagagttt | 213940 | - | see also 16412 line |
| 37267 | ICMT | NM_012405.2 | 788 | ttcttccgcgatcgaacgagaga | 213968 | - | see also 16413 line |
| 37268 | ICMT | NM_012405.2 | 694 | ttcttacgtcgggttgttttact | 213967 | - | see also 16413 line |
| 37269 | ICMT | NM_012405.2 | 295 | ctgcggcacgctgctaagttta | 213955 | - | see also 16413 line |
| 37270 | PEMT | NM_007169.2 | 682 | ctggtrggccctcacctacatagt | 213974 | - | see also 16414 line |
| 37271 | PEMT | NM_007169.2 | 525 | aacttcctaggtgattacttcg | 213973 | - | see also 16414 line |
| 37272 | PNMT | NM_002686.2 | 972 | ccgggacctccgcacctatatca | 213985 | - | see also 16415 line |
| 37273 | PNMT | NM_002686.2 | 971 | tccgggacctccgcacctatatc | 213984 | - | see also 16415 line |
| 37274 | COQ3 | NM_017421.2 | 519 | ggcggcttggggcttcagttatt | 214006 | - | see also 6997 line |
| 37275 | COQ3 | NM_017421.2 | 299 | gtcgacagcgggtgaggtaaaaac | 213998 | - | see also 6997 line |
| 37276 | COQ3 | NM_017421.2 | 121 | ttcctccgcgttttatgtgaaga | 213990 | - | see also 6997 line |

Figure 46

| | | | | | | | |
|---|---|---|---|---|---|---|---|
| 37277 | CGI-30 | NM_015958.1 | 831 | gccgacgaccagaaaattgcagc | 214049 | - | see also 16417 line |
| 37278 | CGI-30 | NM_015958.1 | 704 | tccacggtatatgagtgtaaacc | 214041 | - | see also 16417 line |
| 37279 | CGI-30 | NM_015958.1 | 216 | tacacctcagtcctaactgtagg | 214024 | - | see also 16417 line |
| 37280 | INMT | NM_006774.3 | 182 | ggggacacgctgattgacattgg | 214055 | - | see also 16418 line |
| 37281 | INMT | NM_006774.3 | 633 | tggggaagcgtgaattttcctgc | 214056 | - | see also 16418 line |
| 37282 | GAMT | NM_000156.4 | 206 | gcgctgggagaccccctatatgc | 214057 | - | see also 132 line |
| 37283 | GAMT | NM_000156.4 | 444 | accctgcctgacggtcactttga | 214059 | - | see also 132 line |
| 37284 | TPMT | NM_000367.1 | 753 | tggggaattgactgtcttttga | 214066 | - | see also 16419 line |
| 37285 | NNMT | NM_006169.1 | 826 | gtgatctcgcaaagttattcttc | 214079 | - | see also 16420 line |
| 37286 | NNMT | NM_006169.1 | 797 | aggctggctacacaatcgaatgg | 214074 | - | see also 16420 line |
| 37287 | AD-003 | NM_014064.1 | 775 | atgagatctaccatgtctatagc | 214084 | - | see also 16421 line |
| 37288 | AD-003 | NM_014064.1 | 703 | acgtggtccgcaggatcatctgc | 214083 | - | see also 16421 line |
| 37289 | C2orf8 | NM_025264.2 | 1198 | tggcggaaccattgtattgttgc | 214116 | - | see also 16422 line |
| 37290 | C2orf8 | NM_025264.2 | 113 | gcggccacgcaggttgaatatat | 214086 | - | see also 16422 line |
| 37291 | CGI-01 | NM_015935.2 | 1501 | ggccttgctacgatgtcataatg | 214143 | - | see also 16423 line |
| 37292 | CGI-01 | NM_015935.2 | 478 | tgggctatcgggatatagtgaac | 214127 | - | see also 16423 line |
| 37293 | CYT19 | NM_020682.2 | 816 | atcggtgactgtcgttttgtttc | 214172 | - | see also 16424 line |
| 37294 | CYT19 | NM_020682.2 | 694 | gtctgggtggtgctttatactgg | 214168 | - | see also 16424 line |
| 37295 | CYT19 | NM_020682.2 | 88 | ttcgtgacgctgagatacagaag | 214154 | - | see also 16424 line |
| 37296 | FLJ10640 | NM_019023.1 | 1060 | tgccatagcaggcggtttgaacc | 214190 | - | see also 7664 line |
| 37297 | FLJ10640 | NM_019023.1 | 973 | gtgtcaccagccgactttacagt | 214188 | - | see also 7664 line |
| 37298 | FLJ20628 | NM_017910.2 | 831 | cacaaggacgagtcataagtttt | 214225 | - | see also 16426 line |
| 37299 | FLJ20628 | NM_017910.2 | 542 | ttgaacaacttcggactcttaaa | 214211 | - | see also 16426 line |
| 37300 | FLJ23841 | NM_144589.2 | 769 | gccgagtgtgtgcgaaacctaaa | 214258 | - | see also 16427 line |
| 37301 | FLJ23841 | NM_144589.2 | 320 | agccgcagggggattctatgatg | 214255 | - | see also 16427 line |
| 37302 | HNMT | NM_006895.1 | 567 | atcgaacctcgagaacgtaaagt | 214272 | - | see also 4826 line |
| 37303 | HNMT | NM_006895.1 | 881 | acctagggcttaagtatgagtgc | 214278 | - | see also 4826 line |
| 37304 | HRMT1L1 | NM_001535.1 | 1063 | ctctctgaaccgtgcactatatt | 214296 | - | see also 16429 line |
| 37305 | HRMT1L1 | NM_001535.1 | 1102 | accgtgcaaatttctgatctaga | 214299 | - | see also 16429 line |
| 37306 | HRMT1L2 | NM_001536.2 | 816 | tgcaagtgaagcggaatgactac | 214309 | - | see also 16430 line |
| 37307 | HRMT1L2 | NM_001536.2 | 225 | accgcaactccatgtttcataac | 214302 | - | see also 16430 line |
| 37308 | HRMT1L2 | NM_001536.2 | 805 | cccgttctgcctgcaagtgaagc | 214308 | - | see also 16430 line |
| 37309 | HRMT1L3 | NM_019854.2 | 1132 | atgtgcgagacctcgatttcaca | 214326 | - | see also 16431 line |
| 37310 | HRMT1L3 | NM_019854.2 | 240 | gagatgacctcgagagattatta | 214311 | - | see also 16431 line |
| 37311 | LCMT1 | NM_016015.2 | 1090 | agcatcggccgtcgacatgatgg | 214349 | - | see also 16432 line |
| 37312 | LCMT1 | NM_016015.2 | 623 | gaggttgactttccaatgattgt | 214338 | - | see also 16432 line |

Figure 46

| | | | | | | | |
|---|---|---|---|---|---|---|---|
| 37313 | MGC17301 | NM_152637.1 | 353 | tgcggaaccggagccaactttca | 214354 | - | see also 16433 line |
| 37314 | MGC17301 | NM_152637.1 | 612 | gaccgggaggtgtgctcttttc | 214358 | - | see also 16433 line |
| 37315 | MGC2408 | NM_032331.2 | 537 | gaggccggtttatctcaatgact | 214368 | - | see also 16434 line |
| 37316 | MGC2408 | NM_032331.2 | 534 | ctggaggccggtttatctcaatg | 214366 | - | see also 16434 line |
| 37317 | MGC24132 | NM_152396.1 | 792 | gactacggactgtatgatcatgc | 214392 | - | see also 16435 line |
| 37318 | MGC24132 | NM_152396.1 | 709 | cagctgttcatcctgataagatg | 214387 | - | see also 16435 line |
| 37319 | MGC50559 | NM_173802.2 | 837 | ttgttgttcttggcgatatgttt | 214414 | - | see also 16436 line |
| 37320 | MGC50559 | NM_173802.2 | 558 | atccttactgggcaatctactgg | 214401 | - | see also 16436 line |
| 37321 | NCOA6IP | NM_024831.5 | 1868 | gtcatcggcaggtcaggtattta | 214463 | - | see also 16437 line |
| 37322 | NCOA6IP | NM_024831.5 | 577 | gaggatcgaaaattgtacaattt | 214427 | - | see also 16437 line |
| 37323 | NCOA6IP | NM_024831.5 | 496 | agccgcgtggcggaaatgtttct | 214421 | - | see also 16437 line |
| 37324 | SRM | NM_003132.1 | 361 | cccgcgaaaggtgctgatcatcg | 214500 | - | see also 16438 line |
| 37325 | SRM | NM_003132.1 | 234 | tcctcgtcttccgcagtaagacc | 214499 | - | see also 16438 line |
| 37326 | WBSCR22 | NM_017528.2 | 118 | gacagaagcccggaaatacgttc | 214507 | - | see also 16439 line |
| 37327 | WBSCR22 | NM_017528.2 | 673 | ttctgggccttcgacctttatc | 214517 | - | see also 16439 line |
| 37328 | GART | NM_000819.3 | 1083 | caccgccctaactgttgtcatgg | 214546 | - | see also 16440 line |
| 37329 | GART | NM_000819.3 | 1102 | atggcaagtaaaggttatcctgg | 214548 | - | see also 16440 line |
| 37330 | MtFMT | NM_139242.2 | 725 | ggcgacttacgcccctaagattt | 214620 | - | see also 16441 line |
| 37331 | MtFMT | NM_139242.2 | 386 | ttcgtttggccgacttttgaatg | 214612 | - | see also 16441 line |
| 37332 | FTHFD | NM_012190.2 | 339 | gagtgccggtattcaagtactcc | 214639 | - | see also 16442 line |
| 37333 | FTHFD | NM_012190.2 | 336 | atggagtgccggtattcaagtac | 214637 | - | see also 16442 line |
| 37334 | SHMT1 | NM_004169.3 | 975 | gccgagctggcatgatcttctac | 214683 | - | see also 16443 line |
| 37335 | SHMT1 | NM_004169.3 | 379 | ggccctaggctcttgcttaaata | 214677 | - | see also 16443 line |
| 37336 | SHMT1 | NM_004169.3 | 336 | agctgattgcctcggagaatttc | 214675 | - | see also 16443 line |
| 37337 | SHMT2 | NM_005412.3 | 930 | gccgggagatcccttacacattt | 214697 | - | see also 16444 line |
| 37338 | SHMT2 | NM_005412.3 | 665 | ttccggccacggctcatcatagc | 214694 | - | see also 16444 line |
| 37339 | SHMT2 | NM_005412.3 | 697 | cgcctatgctcgcctcattgact | 214695 | - | see also 16444 line |
| 37340 | AMT | NM_000481.1 | 206 | gccttgtgtcgtccacttagttg | 214707 | - | see also 16445 line |
| 37341 | AMT | NM_000481.1 | 247 | caggacaccgctctatgacttcc | 214708 | - | see also 16445 line |
| 37342 | DMGDH | NM_013391.2 | 2449 | ttcgcatatgtccctgtacaact | 214798 | - | see also 5421 line |
| 37343 | DMGDH | NM_013391.2 | 759 | ggctcgtgaagtaggtaaaatga | 214744 | - | see also 5421 line |
| 37344 | DMGDH | NM_013391.2 | 2082 | ggcgctgtatgacgctatcatga | 214785 | - | see also 5421 line |

Figure 46

| | | | | | | | | | |
|---|---|---|---|---|---|---|---|---|---|
| 37345 | GCSH | NM_004483.2 | 326 | aagttgggacaaaattgaacaaa | 214802 | - | - | aminomethyltransferase | - |
| 37346 | PDPR | NM_017990.3 | 3086 | ttcgcagtctccgaattgagaag | 214808 | - | see also 7190 line | | |
| 37347 | PDPR | NM_017990.3 | 3268 | atcctggacgaccatgattcaga | 214816 | - | see also 7190 line | | |
| 37348 | PDPR | NM_017990.3 | 3463 | aaccggggagagtatgagattga | 214819 | - | see also 7190 line | | |
| 37349 | SARDH | NM_007101.2 | 2886 | gccggtctcgctggactttgtga | 214838 | - | see also 16448 line | | |
| 37350 | SARDH | NM_007101.2 | 1154 | agcgcatcgaggggattcagaac | 214824 | - | see also 16448 line | | |
| 37351 | SARDH | NM_007101.2 | 1913 | cccaccacgacacgatcaagaag | 214832 | - | see also 16448 line | | |
| 37352 | TYMS | NM_001071.1 | 1006 | tacaatccgcatccaactattaa | 214857 | - | see also 16449 line | | |
| 37353 | TYMS | NM_001071.1 | 643 | tgcgcttggaatccaagagatct | 214848 | - | see also 16449 line | | |
| 37354 | TYMS | NM_001071.1 | 1010 | atccgcatccaactattaaaatg | 214858 | - | see also 16449 line | | |
| 37355 | FLJ20257 | NM_019606.3 | 2048 | gtcttcgtcacgggtaattatgt | 214865 | - | see also 16450 line | | |
| 37356 | FLJ20257 | NM_019606.3 | 1924 | agggaccaccaccgttcgaaaga | 214863 | - | see also 16450 line | | |
| 37357 | FTSJ1 | NM_012280.2 | 1001 | tggtcccacccgcatcattgtgc | 214879 | - | see also 16451 line | | |
| 37358 | FTSJ1 | NM_012280.2 | 389 | tgctcgcagcgccttcaaactgc | 214873 | - | see also 16451 line | | |
| 37359 | FTSJ2 | NM_013393.1 | 625 | gtcaaagccgtcggttacagagg | 214890 | - | see also 16452 line | | |
| 37360 | FTSJ2 | NM_013393.1 | 425 | cccgagaacctcacagagaatcc | 214887 | - | see also 16452 line | | |
| 37361 | LOC57019 | NM_020313.1 | 757 | accacgactgtatcactcattgc | 214901 | - | see also 16453 line | | |
| 37362 | LOC57019 | NM_020313.1 | 555 | gaccctctcagccaacgatatgg | 214893 | - | see also 16453 line | | |
| 37363 | LOC57019 | NM_020313.1 | 598 | ttctgtggatgtagtttaactca | 214895 | - | see also 16453 line | | |
| 37364 | SKB1 | NM_006109.2 | 665 | ttccggactttgtgtgactatag | 214908 | - | see also 4246 line | | |
| 37365 | SKB1 | NM_006109.2 | 666 | tccggactttgtgtgactatagt | 214909 | - | see also 4246 line | | |
| 37366 | GATM | NM_001482.1 | 646 | taccgagcgtacaggtcaattat | 214946 | - | see also 1021 line | | |
| 37367 | GATM | NM_001482.1 | 647 | accgagcgtacaggtcaattatc | 214947 | - | see also 1021 line | | |
| 37368 | NAT1 | NM_000662.3 | 594 | ttccgtttgacggaagagaatgg | 214970 | - | see also 16455 line | | |
| 37369 | NAT1 | NM_000662.3 | 722 | tactcttaagcctcgaacaattg | 214975 | - | see also 16455 line | | |
| 37370 | NAT1 | NM_000662.3 | 863 | cacccataggagattcaattata | 214977 | - | see also 16455 line | | |
| 37371 | NAT2 | NM_000015.1 | 683 | tacgcttgaacctcgaacaattg | 214985 | - | see also 16456 line | | |
| 37372 | NAT2 | NM_000015.1 | 856 | cagatctggtcgagtttaaaact | 214992 | - | see also 16456 line | | |
| 37373 | SAT | NM_002970.1 | 411 | cccgtggattggcaagttattgt | 215001 | - | see also 16457 line | | |
| 37374 | SAT | NM_002970.1 | 445 | ttcttcgtgatgagtgattatag | 215004 | - | see also 16457 line | | |
| 37375 | SAT | NM_002970.1 | 469 | ggctttggcataggatcagaaat | 215005 | - | see also 16457 line | | |
| 37376 | AANAT | NM_001088.1 | 718 | gacgcgctggtacccttctatga | 215013 | - | - | arylalkylamine N-acetyltransferase | - |
| 37377 | NAGS | NM_153006.1 | 893 | gcctgcgcgacagcagtcataag | 215016 | - | see also 16458 line | | |
| 37378 | NAGS | NM_153006.1 | 1445 | atccctggtacttcaaacacagt | 215020 | - | see also 16458 line | | |
| 37379 | ARD1 | NM_003491.2 | 126 | tgcgaggccagaggacctaatga | 215021 | - | see also 2356 line | | |

Figure 46

| 37380 | CML2 | NM_016347.1 | 275 | accctggacgcggtatgtagaca | 215028 | - | see also 16459 line |
|---|---|---|---|---|---|---|---|
| 37381 | CML2 | NM_016347.1 | 283 | cgcggtatgtagacatagcattg | 215030 | - | see also 16459 line |
| 37382 | CSRP2BP | NM_020536.2 | 2521 | gcccgacgcacctctcgattact | 215059 | - | see also 7892 line |
| 37383 | CSRP2BP | NM_020536.2 | 2523 | ccgacgcacctctcgattactgt | 215060 | - | see also 7892 line |
| 37384 | DKFZP564C103 | NM_015654.3 | 156 | taccacgagtggatgaaatcaga | 215075 | - | see also 16461 line |
| 37385 | DKFZP564C103 | NM_015654.3 | 562 | aggtggctacgagcagtgttttt | 215081 | - | see also 16461 line |
| 37386 | ELP3 | NM_018091.3 | 1250 | tacggccataccaggttgaattg | 215117 | - | see also 7252 line |
| 37387 | ELP3 | NM_018091.3 | 1355 | tcctacgattacgcaagtgttca | 215124 | - | see also 7252 line |
| 37388 | ELP3 | NM_018091.3 | 940 | cccgatgggctgaaactctatcc | 215109 | - | see also 7252 line |
| 37389 | FLJ13848 | NM_024771.1 | 316 | ttcgacctgaccaaaacgaatat | 215138 | - | see also 16463 line |
| 37390 | FLJ13848 | NM_024771.1 | 419 | acctcatcgcgtgggaaaacagc | 215140 | - | see also 16463 line |
| 37391 | FLJ14154 | NM_024845.1 | 388 | cacaagtcgcgtacatcctaagt | 215149 | - | see also 8781 line |
| 37392 | FLJ14154 | NM_024845.1 | 784 | tgccatggtcgggcatctcttcc | 215155 | - | see also 8781 line |
| 37393 | FLJ37478 | NM_178557.2 | 159 | ggcggacatcgagcagtactaca | 215156 | - | - |
| 37394 | MAK3P | NM_025146.1 | 543 | accttaccgaaggctaggaatag | 215167 | - | see also 8871 line |
| 37395 | MAK3P | NM_025146.1 | 547 | taccgaaggctaggaataggaac | 215168 | - | see also 8871 line |
| 37396 | NAT5 | NM_016100.2 | 210 | ctcgcccactggccagagtattt | 215177 | - | see also 6660 line |
| 37397 | SAT2 | NM_133491.2 | 276 | gggagctagccgaattcgaaaaa | 215198 | - | see also 16467 line |
| 37398 | SAT2 | NM_133491.2 | 282 | tagccgaattcgaaaaactctcg | 215200 | - | see also 16467 line |
| 37399 | SAT2 | NM_133491.2 | 275 | cgggagctagccgaattcgaaaa | 215197 | - | see also 16467 line |
| 37400 | GTF3C4 | NM_012204.1 | 2379 | ccgcggactctgtaactttttaa | 215243 | - | see also 16468 line |
| 37401 | GTF3C4 | NM_012204.1 | 762 | tcccatgggttgcgatgctaatg | 215217 | - | see also 16468 line |
| 37402 | ACAT1 | NM_000019.2 | 707 | caggacgcttatgctattaattc | 215271 | - | see also 16469 line |
| 37403 | ACAT1 | NM_000019.2 | 702 | atgaacaggacgcttatgctatt | 215269 | - | see also 16469 line |
| 37404 | HADHA | NM_000182.3 | 80 | ctccgctcccgaggttatatatg | 215294 | - | see also 155 line |
| 37405 | HADHA | NM_000182.3 | 1899 | agggcttcctaggtcgtaaatct | 215340 | - | see also 155 line |
| 37406 | GCAT | NM_014291.2 | 998 | gacccagaggttccgtagtaaga | 215360 | - | see also 16471 line |
| 37407 | GCAT | NM_014291.2 | 1000 | cccagaggttccgtagtaagatg | 215361 | - | see also 16471 line |
| 37408 | CHAT | NM_020549.2 | 1498 | tgcgaacactccccattcgatgg | 215371 | - | see also 16472 line |
| 37409 | CHAT | NM_020549.2 | 1210 | gacgagcgtttgcctccaattgg | 215366 | - | see also 16472 line |
| 37410 | CRAT | NM_000755.2 | 2011 | cggctacggtgtctgctataacc | 215390 | - | see also 16473 line |
| 37411 | CRAT | NM_000755.2 | 909 | tggtacacaactaccagtttttt | 215385 | - | see also 16473 line |
| 37412 | GNPAT | NM_014236.1 | 841 | aactatgttacggaatggttatg | 215416 | - | see also 16474 line |
| 37413 | GNPAT | NM_014236.1 | 1698 | ttcgcttcctacgtgatgttttt | 215435 | - | see also 16474 line |
| 37414 | NMT1 | NM_021079.3 | 924 | cccacggaagctgattgaagtga | 215451 | - | see also 8079 line |

Figure 46

| 37415 | NMT1 | NM_021079.3 | 667 | gtctcaagtcggaaattggttgg | 215446 | - | see also 8079 line |
|---|---|---|---|---|---|---|---|
| 37416 | NMT2 | NM_004808.1 | 486 | tacgccaagaaccgtattctttg | 215468 | - | see also 3342 line |
| 37417 | NMT2 | NM_004808.1 | 823 | acgggtagcccagtgctaatcc | 215480 | - | see also 3342 line |
| 37418 | AGPAT1 | NM_006411.2 | 497 | gacgcaacgtcgagaacatgaag | 215506 | - | see also 16477 line |
| 37419 | AGPAT1 | NM_006411.2 | 985 | cgcttcacctcgggacaatgtca | 215513 | - | see also 16477 line |
| 37420 | AGPAT4 | NM_020133.1 | 645 | gggactacccgagaagtatttt | 215524 | - | see also 16478 line |
| 37421 | AGPAT4 | NM_020133.1 | 307 | ctgcagactgtcctattgcatct | 215518 | - | see also 16478 line |
| 37422 | LPAAT-e | NM_018361.1 | 814 | tggctgcatgaacgtttcgaaat | 215549 | - | see also 7414 line |
| 37423 | LPAAT-e | NM_018361.1 | 708 | accgaccatgacggaatttctct | 215545 | - | see also 7414 line |
| 37424 | CPT1A | NM_001876.1 | 1388 | agctacgccaaatctcactaca | 215587 | - | see also 16480 line |
| 37425 | CPT1A | NM_001876.1 | 1086 | accatcgagacgctacttcaag | 215580 | - | see also 16480 line |
| 37426 | CPT1A | NM_001876.1 | 707 | gtcggtcttggaccaagattaca | 215571 | - | see also 16480 line |
| 37427 | CPT1B | NM_004377.2 | 2264 | aggcgagaacacgatcttctcc | 215626 | - | see also 16481 line |
| 37428 | CPT1B | NM_004377.2 | 1703 | ggcagacgacgtggagttgtact | 215620 | - | see also 16481 line |
| 37429 | CPT2 | NM_000098.1 | 2247 | tgcatacgggcagataaaaccaca | 215654 | - | see also 16482 line |
| 37430 | CPT2 | NM_000098.1 | 1200 | accagtcgggatgaactcttca | 215638 | - | see also 16482 line |
| 37431 | CROT | NM_021151.2 | 449 | tgcctatctggattgttcgtatac | 215662 | - | see also 16483 line |
| 37432 | CROT | NM_021151.2 | 1423 | tgcttcaccggatacgttatt | 215718 | - | see also 16483 line |
| 37433 | SOAT1 | NM_003101.3 | 2216 | aacgtgcctcgggttactaaattc | 215759 | - | see also 16484 line |
| 37434 | SOAT1 | NM_003101.3 | 1515 | gtcgaattgacataaaacagttg | 215737 | - | see also 16484 line |
| 37435 | SOAT1 | NM_003101.3 | 2168 | ttcgagcagattcgttttgtaat | 215756 | - | see also 16484 line |
| 37436 | SOAT2 | NM_003578.2 | 250 | gccatgcgggaggctatacaatc | 215788 | - | see also 16485 line |
| 37437 | SOAT2 | NM_003578.2 | 918 | gccctatgtcaggtggaattatg | 215791 | - | see also 16485 line |
| 37438 | SOAT2 | NM_003578.2 | 249 | ggccatgcgggaggctatacaat | 215787 | - | see also 16485 line |
| 37439 | LCAT | NM_000229.1 | 298 | tagactgctgatcgataacacc | 215805 | - | see also 16486 line |
| 37440 | LCAT | NM_000229.1 | 671 | ggcctggaaggaccgctttattg | 215808 | - | see also 16486 line |
| 37441 | KIAA1560 | NM_020918.2 | 1023 | tacgacggaagctcgatgaaaca | 215842 | - | see also 8021 line |
| 37442 | KIAA1560 | NM_020918.2 | 1840 | cactacgaggaacgatgagtttt | 215873 | - | see also 8021 line |
| 37443 | KIAA1560 | NM_020918.2 | 1571 | tccctacgaaggagttgattgc | 215867 | - | see also 8021 line |
| 37444 | ACAA1 | NM_001607.2 | 389 | ggcccgaatcgcccagtttctga | 215893 | - | see also 16488 line |
| 37445 | ACAA1 | NM_001607.2 | 391 | ccgaatcgcccagtttctgagt | 215894 | - | see also 16488 line |
| 37446 | ACAA2 | NM_006111.1 | 102 | agcttacggaggccttctgaaag | 215908 | - | see also 16489 line |
| 37447 | ACAA2 | NM_006111.1 | 195 | aacagttgacagtgtgattatgg | 215909 | - | see also 16489 line |
| 37448 | HADHB | NM_000183.1 | 178 | gacgttagccaaacccaatataa | 215914 | - | see also 16490 line |
| 37449 | HADHB | NM_000183.1 | 1101 | tacttgaccaacatatgctact | 215936 | - | see also 16490 line |
| 37450 | HADHB | NM_000183.1 | 179 | acgttagccaaacccaatataag | 215915 | - | see also 16490 line |
| 37451 | SPTLC2 | NM_004863.2 | 1845 | gacgagaacgacgtatgaagaaac | 216027 | - | see also 3378 line |

Figure 46

| | | | | | | | |
|---|---|---|---|---|---|---|---|
| 37452 | SPTLC2 | NM_004863.2 | 1848 | gagacgacgtatgaagaaacaga | 216028 | - | see also 3378 line |
| 37453 | DLST | NM_001933.3 | 770 | atgtgcaatgctgacaacttta | 216030 | - | see also 16492 line |
| 37454 | ALAS1 | NM_000688.4 | 351 | ttcgccgctgccattcttatcc | 216032 | - | see also 16493 line |
| 37455 | ALAS1 | NM_000688.4 | 975 | accacacctgagtttttaaa | 216042 | - | see also 16493 line |
| 37456 | ALAS2 | NM_000032.1 | 917 | atccaaggtatccgtaacagtgg | 216065 | - | see also 4 line |
| 37457 | ALAS2 | NM_000032.1 | 626 | tccgtctggtagtaatgatta | 216063 | - | see also 4 line |
| 37458 | ATE1 | NM_007041.1 | 1195 | tacgatcctgattattcgtttt | 216106 | - | see also 4930 line |
| 37459 | ATE1 | NM_007041.1 | 1493 | accgattgcagtgtttcacaag | 216111 | - | see also 4930 line |
| 37460 | AUP1 | NM_012103.2 | 306 | agcgtccttgccagattcgtagt | 216117 | - | see also 5132 line |
| 37461 | AUP1 | NM_012103.2 | 1491 | gagcgcaagcaagcactatatga | 216134 | - | see also 5132 line |
| 37462 | BAAT | NM_001701.1 | 1303 | tgcctcaacgaccacgatttga | 216178 | - | see also 16497 line |
| 37463 | BAAT | NM_001701.1 | 1043 | accaatgccttgggttactaga | 216172 | - | see also 16497 line |
| 37464 | BAAT | NM_001701.1 | 585 | caactggtgtcacacgaattaag | 216151 | - | see also 16497 line |
| 37465 | CPT1C | NM_152359.1 | 878 | tcctggtggcgtccaattatgt | 216186 | - | see also 16498 line |
| 37466 | CPT1C | NM_152359.1 | 1966 | gtcggccatgactcgcttattcc | 216195 | - | see also 16498 line |
| 37467 | CPT1C | NM_152359.1 | 2294 | cacaattacccggactacgtttc | 216199 | - | see also 16498 line |
| 37468 | DGAT2 | NM_032564.2 | 914 | tgggagtggcaatgctatcatca | 216214 | - | see also 9248 line |
| 37469 | DGAT2 | NM_032564.2 | 880 | gccggacaccatagactactatttg | 216213 | - | see also 9248 line |
| 37470 | DGAT2L1 | NM_058165.1 | 494 | ctgtctttcgagaatatgtgatg | 216228 | - | see also 16500 line |
| 37471 | DGAT2L1 | NM_058165.1 | 166 | atgctgatcatacacaactattt | 216221 | - | see also 16500 line |
| 37472 | DKFZp586 M1819 | NM_178819.2 | 2034 | gacgtacctgctgctgcggaatgatga | 216272 | - | see also 10210 line |
| 37473 | DKFZp586 M1819 | NM_178819.2 | 1674 | ctcacgatcgatgtgatcatct | 216263 | - | see also 10210 line |
| 37474 | DKFZp586 M1819 | NM_178819.2 | 1676 | caccgatcgatgtgatcatcttg | 216264 | - | see also 10210 line |
| 37475 | FLJ32370 | NM_152417.1 | 740 | ggccacttgttagctactctcacc | 216286 | - | see also 9860 line |
| 37476 | FLJ32370 | NM_152417.1 | 481 | tgcagccgtttgcatgtgttatg | 216279 | - | see also 9860 line |
| 37477 | FLJ37965 | NM_182551.1 | 926 | gtggtagaccgctctaagagaagg | 216310 | - | see also 16503 line |
| 37478 | FLJ37965 | NM_182551.1 | 430 | ttggtatcgctggatcaacaacc | 216299 | - | see also 16503 line |
| 37479 | GGTLA1 | NM_004121.1 | 1231 | acgtgctaagagggttcaacttc | 216313 | - | see also 16504 line |
| 37480 | GGTLA1 | NM_004121.1 | 1208 | tgccattctcagctttatcctca | 216312 | - | see also 16504 line |
| 37481 | GLYAT | NM_005838.1 | 468 | aacacaacgcattctctatatgg | 216330 | - | see also 16505 line |
| 37482 | GLYAT | NM_005838.1 | 411 | tagcctgaatgaggctatacaaa | 216326 | - | see also 16505 line |
| 37483 | KIAA0205 | NM_014873.1 | 381 | aagcggttctgtatatcgaagg | 216336 | - | see also 6069 line |
| 37484 | KIAA0205 | NM_014873.1 | 389 | ctggtatatcgaaggaatcatgt | 216337 | - | see also 6069 line |
| 37485 | LRAT | NM_004744.1 | 694 | tgggattggcgtctatagtctgt | 216372 | - | see also 16507 line |

Figure 46

| ID | Gene | Accession | Pos | Sequence | ID2 | | Note |
|---|---|---|---|---|---|---|---|
| 37486 | LRAT | NM_004744.1 | 384 | atcctgggcgttattgtcaaagt | 216368 | - | see also 16507 line |
| 37487 | MGAT2 | NM_025098.2 | 720 | ctcctggttacgctatatccaga | 216388 | - | see also 16508 line |
| 37488 | MGAT2 | NM_025098.2 | 910 | ctgcaccagcgttatatcaaaga | 216391 | - | see also 16508 line |
| 37489 | MGAT3 | NM_178176.2 | 629 | cccggtctatcgcgactacatca | 216402 | - | see also 16509 line |
| 37490 | MGAT3 | NM_178176.2 | 745 | gtgcgcacagagcccgtattca | 216404 | - | see also 16509 line |
| 37491 | MGC11324 | NM_032717.3 | 565 | ggcgtcatagtgcgctattgtgt | 216422 | - | see also 16510 line |
| 37492 | MGC11324 | NM_032717.3 | 262 | gagtgggccacaatacgaattga | 216412 | - | see also 16510 line |
| 37493 | QPCT | NM_012413.2 | 521 | tgctcgtcgcttagcacaagaaac | 216456 | - | see also 16511 line |
| 37494 | QPCT | NM_012413.2 | 648 | ctctatgggtctcgacacttagc | 216459 | - | see also 16511 line |
| 37495 | TAZ | NM_000116.2 | 939 | gtggcatgtcggaatgaatgacg | 216477 | - | see also 16512 line |
| 37496 | TAZ | NM_000116.2 | 989 | cccgctttggacagaaaatcact | 216478 | - | see also 16512 line |
| 37497 | FLJ90165 | NM_153338.1 | 153 | atcagaggcgctggttctaaacc | 216479 | - | - |
| 37498 | GGTL3 | NM_052830.3 | 495 | gggcatcgaggtgctcagtaaac | 216481 | - | see also 16513 line |
| 37499 | GGTL3 | NM_052830.3 | 600 | gggcgtgatgctggtacatgaca | 216483 | - | see also 16513 line |
| 37500 | HMGCS1 | NM_002130.3 | 492 | gacacaactaatgcatgctatgg | 216505 | - | see also 1367 line |
| 37501 | HMGCS1 | NM_002130.3 | 1300 | caccgggtctgctcttgataaa | 216526 | - | see also 1367 line |
| 37502 | HMGCS2 | NM_005518.1 | 842 | gggccttgatcgatgttacaca | 216560 | - | see also 3849 line |
| 37503 | HMGCS2 | NM_005518.1 | 1540 | gagcagcatcgccgaagatgc | 216578 | - | see also 3849 line |
| 37504 | CS | NM_004077.2 | 914 | aacatgttaggctatactgatca | 216587 | - | see also 16516 line |
| 37505 | CS | NM_004077.2 | 820 | ttgtgttgcagcaaagatctacc | 216583 | - | see also 16516 line |
| 37506 | CLYBL | NM_138280.2 | 139 | cccggaggcagtgctttatgt | 216595 | - | see also 9591 line |
| 37507 | CLYBL | NM_138280.2 | 94 | atcccagacttggatatagttc | 216590 | - | see also 9591 line |
| 37508 | CLYBL | NM_138280.2 | 264 | agctcgactgagaattgtaaaaa | 216597 | - | see also 9591 line |
| 37509 | GSTA3 | NM_000847.3 | 698 | cccgcagatgcaaaagctttaga | 216614 | - | glutathione transferase |
| 37510 | GSTA3 | NM_000847.3 | 696 | ctcccgcagatgcaaaagcttta | 216612 | - | see also 37509 line |
| 37511 | GSTA3 | NM_000847.3 | 697 | tcccgcagatgcaaaagcttag | 216613 | - | see also 37509 line |
| 37512 | GSTA4 | NM_001512.2 | 164 | tccgtgatggtttagctgc | 216616 | - | see also 16518 line |
| 37513 | GSTA4 | NM_001512.2 | 187 | cgccggagtcgagtttgatgaag | 216617 | - | see also 16518 line |
| 37514 | GSTA4 | NM_001512.2 | 188 | gccggagtcgagtttgatgaaga | 216618 | - | see also 16518 line |
| 37515 | GSTM1 | NM_000561.2 | 363 | gaacatggacaacatatgcagc | 216625 | - | see also 16519 line |
| 37516 | GSTM1 | NM_000561.2 | 669 | aagacctgttctcaaagatgg | 216628 | - | see also 16519 line |
| 37517 | GSTM2 | NM_000848.2 | 535 | tccttgagagaaaccaagtattt | 216630 | - | see also 16520 line |
| 37518 | GSTM3 | NM_000849.3 | 427 | ttcgagtggacatcatagagaac | 216634 | - | see also 16521 line |
| 37519 | GSTM3 | NM_000849.3 | 633 | ttggatcgaaacgtatatttga | 216641 | - | see also 16521 line |

Figure 46

| 37520 | GSTM4 | NM_000850.3 | 692 | tggaggaacttcctacaatgatg | 216642 | - | see also 16522 line | | |
| 37521 | GSTM5 | NM_000851.2 | 681 | tccgaggtcttttgtttggaaag | 216644 | - | - | monodehydroascorbate reductase (NADH) | - |
| 37522 | GSTO1 | NM_004832.1 | 323 | atgacccctatgagaaagcttgc | 216649 | - | see also 16523 line | | |
| 37523 | GSTO1 | NM_004832.1 | 587 | tagaccacactccaaaactgaaa | 216655 | - | see also 16523 line | | |
| 37524 | GSTO2 | NM_183239.1 | 957 | gagctcgccaaaagatgttattg | 216667 | - | see also 10291 line | | |
| 37525 | GSTO2 | NM_183239.1 | 954 | aacgagctcgccaaaagatgtta | 216665 | - | see also 10291 line | | |
| 37526 | GSTP1 | NM_000852.2 | 624 | tacgtgaacctccccatcaatgg | 216683 | - | see also 16525 line | | |
| 37527 | GSTP1 | NM_000852.2 | 35 | gccctacaccgtggtctatttcc | 216679 | - | see also 16525 line | | |
| 37528 | GSTT1 | NM_000853.1 | 214 | ctctacctgacgcgcaaatataa | 216688 | - | see also 16526 line | | |
| 37529 | GSTT1 | NM_000853.1 | 89 | tgcgcatcgtggatctgattaaa | 216685 | - | see also 16526 line | | |
| 37530 | GSTT2 | NM_000854.2 | 265 | ctcggccatcctgatttacctga | 216693 | - | see also 16527 line | | |
| 37531 | GSTT2 | NM_000854.2 | 413 | caggtgttggggccactcattgg | 216696 | - | see also 16527 line | | |
| 37532 | GSTZ1 | NM_001513.2 | 814 | agcacagcgggcatatactgtgt | 216703 | - | see also 16528 line | | |
| 37533 | GSTZ1 | NM_001513.2 | 552 | aaccctgaagattgatggaatca | 216697 | - | see also 16528 line | | |
| 37534 | LOC51064 | NM_015917.1 | 220 | tccccgcaaaggactatacatgg | 216711 | - | see also 16529 line | | |
| 37535 | LOC51064 | NM_015917.1 | 59 | cgcaccgtggagctcttctatga | 216710 | - | see also 16529 line | | |
| 37536 | MGST1 | NM_020300.3 | 467 | cagccaaatagagctttgagttt | 216728 | - | see also 16530 line | | |
| 37537 | MGST1 | NM_020300.3 | 348 | ttggaattggcctcctgtattcc | 216724 | - | see also 16530 line | | |
| 37538 | MGST2 | NM_002413.3 | 395 | gagtatttcgggcacaacaaaac | 216732 | - | see also 16531 line | | |
| 37539 | MGST2 | NM_002413.3 | 294 | ctcggcctgtcagcaaagttatt | 216730 | - | see also 16531 line | | |
| 37540 | MGST3 | NM_004528.2 | 155 | gcccacctagccatcaatgtttc | 216748 | - | see also 16532 line | | |
| 37541 | MGST3 | NM_004528.2 | 133 | tgctgccagctttataatggtgg | 216746 | - | see also 16532 line | | |
| 37542 | CHM | NM_000390.1 | 1928 | aggctaactcggagactttcaag | 216796 | - | see also 310 line | | |
| 37543 | CHM | NM_000390.1 | 52 | gagtttgatgtgatcgtaatagg | 216759 | - | see also 310 line | | |
| 37544 | RABGGTA | NM_004581.2 | 1727 | gaccccatgcgggcaacgtatct | 216806 | - | see also 16534 line | | |
| 37545 | RABGGTA | NM_004581.2 | 1519 | cacgacagacgagcagctattca | 216804 | - | see also 16534 line | | |
| 37546 | PGGT1B | NM_005023.2 | 411 | gacgacttaagccgagtaaataa | 216831 | - | see also 16535 line | | |
| 37547 | PGGT1B | NM_005023.2 | 71 | tcggcacgtgcgattttttccagc | 216817 | - | see also 16535 line | | |
| 37548 | FNTA | NM_002027.1 | 511 | tggcatcataggcgagtattagt | 216866 | - | see also 1315 line | | |
| 37549 | FNTA | NM_002027.1 | 265 | gtggtccagatcatttatagtga | 216857 | - | see also 1315 line | | |
| 37550 | FNTB | NM_002028.2 | 1074 | acctggcaagtcgcgtgatttct | 216878 | - | see also 16537 line | | |
| 37551 | FNTB | NM_002028.2 | 850 | agccggcagatgcgatttgaagg | 216876 | - | see also 16537 line | | |
| 37552 | NS3TP1 | NM_019048.1 | 499 | agcggggacccaatagtagtaaa | 216881 | - | see also 16538 line | | |

Figure 46

| | | | | | | | |
|---|---|---|---|---|---|---|---|
| 37553 | NS3TP1 | NM_019048.1 | 503 | gggacccaatagtagtaaacaat | 216884 | - | see also 16538 line |
| 37554 | COX10 | NM_001303.2 | 836 | aggtgccatttgactcaaacatg | 216962 | - | see also 16539 line |
| 37555 | COX10 | NM_001303.2 | 293 | aggactccctcacaagttctta | 216953 | - | see also 16539 line |
| 37556 | GGPS1 | NM_004837.2 | 1007 | tacactcgtaataccccttaaaga | 216984 | - | see also 16540 line |
| 37557 | GGPS1 | NM_004837.2 | 1077 | ctgagctagagccttagtaaaa | 216987 | - | see also 16540 line |
| 37558 | GGPS1 | NM_004837.2 | 1083 | tagtagccttagtaaaacactta | 216988 | - | see also 16540 line |
| 37559 | FDPS | NM_002004.1 | 119 | ccctgtcccgctggttgagatct | 216990 | - | see also 16541 line |
| 37560 | FDPS | NM_002004.1 | 1015 | atggggagttctttcagattca | 216993 | - | see also 16541 line |
| 37561 | CL640 | NM_015697.3 | 1053 | aaccgaacactgggactaatagt | 217033 | - | see also 16542 line |
| 37562 | CL640 | NM_015697.3 | 419 | agcaggctgtactattaatgaca | 217009 | - | see also 16542 line |
| 37563 | RABGGTB | NM_004582.2 | 317 | tggttggaataagtgctagtatcg | 217044 | - | see also 16543 line |
| 37564 | RABGGTB | NM_004582.2 | 858 | ctgcgtaatttcatttagcatg | 217066 | - | see also 16543 line |
| 37565 | SMS | NM_004595.2 | 806 | aacgtgtggcgatgtcttagaca | 217078 | - | see also 16544 line |
| 37566 | SMS | NM_004595.2 | 525 | gacgaagattcaaccttatcaaaa | 217069 | - | see also 16544 line |
| 37567 | HMBS | NM_000190.2 | 1100 | ggcatcactgctcgtaacattcc | 217099 | - | see also 16545 line |
| 37568 | HMBS | NM_000190.2 | 946 | agccgtgcatacagctatgaagg | 217096 | - | see also 16545 line |
| 37569 | HMBS | NM_000190.2 | 234 | gccagcttgctcgcatacagacg | 217089 | - | see also 16545 line |
| 37570 | DHPS | NM_001930.2 | 573 | gggagaacgggatcaataggatc | 217104 | - | see also 16546 line |
| 37571 | DHPS | NM_001930.2 | 697 | gttggacgcttctaagatgggatcg | 217105 | - | see also 16546 line |
| 37572 | AGPS | NM_003659.1 | 941 | ctgtaggaggatgggtatctact | 217136 | - | see also 2478 line |
| 37573 | AGPS | NM_003659.1 | 623 | agcgaattcctgatatagtttta | 217128 | - | see also 2478 line |
| 37574 | SULT1A3 | NM_003166.2 | 443 | cccttcctgaggtcaatgatcc | 217182 | - | - |
| 37575 | HS3ST2 | NM_006043.1 | 859 | tacttccgctagctcagattca | 217193 | - | see also 16548 line |
| 37576 | HS3ST2 | NM_006043.1 | 630 | ggctcctgacgcatcttcaaca | 217187 | - | see also 16548 line |
| 37577 | HS3ST3A1 | NM_006042.1 | 897 | cacgtcccttacgtcttctact | 217205 | - | see also 16549 line |
| 37578 | HS3ST3B1 | NM_006041.1 | 341 | gcctgagtggcggcagatcttgc | 217206 | - | - |
| 37579 | NDST2 | NM_003635.2 | 874 | tggagtctagtcgttttcgttat | 217208 | - | see also 2453 line |
| 37580 | NDST2 | NM_003635.2 | 873 | ctggagtctagtcgttttcgtta | 217207 | - | see also 2453 line |
| 37581 | NDST3 | NM_004784.1 | 2123 | cagaatccttggcgatgacaaacg | 217268 | - | see also 3295 line |
| 37582 | CHST8 | NM_022467.3 | 1773 | ctcatcgactacgatttcgtagg | 217300 | - | see also 8390 line |
| 37583 | CHST8 | NM_022467.3 | 979 | gggtcactcgggacttatccagt | 217295 | - | see also 8390 line |
| 37584 | CHST9 | NM_031422.1 | 1062 | caccccaatagttattaccatcc | 217329 | - | see also 16553 line |
| 37585 | CHST9 | NM_031422.1 | 919 | ctgtccactacgggaagcatttg | 217324 | - | see also 16553 line |
| 37586 | TPST1 | NM_003596.2 | 437 | aacgtagccagccagtcaaattg | 217348 | - | see also 2414 line |
| 37587 | TPST1 | NM_003596.2 | 1211 | atgtaggagctctatcaaaatgg | 217374 | - | see also 2414 line |

Figure 46

| | | | | | | | |
|---|---|---|---|---|---|---|---|
| 37588 | TPST2 | NM_003595.2 | 1184 | ctggctatgaccctatgcaaa | 217381 | - | see also 16555 line |
| 37589 | TPST2 | NM_003595.2 | 1247 | aacacacagcgggtcttgaaagg | 217382 | - | see also 16555 line |
| 37590 | CHST3 | NM_004273.2 | 1782 | atgcgcctcttggctacaaact | 217391 | - | see also 2867 line |
| 37591 | CHST7 | NM_019886.2 | 717 | cccggacacggccaatcttacc | 217399 | - | see also 7744 line |
| 37592 | SULT2A1 | NM_003167.2 | 82 | gtcggacgatttcttatggtttg | 217402 | - | see also 16558 line |
| 37593 | SULT2A1 | NM_003167.2 | 691 | gacgttagaaccgaagaactga | 217417 | - | see also 16558 line |
| 37594 | CHST6 | NM_021615.2 | 1132 | gccgcggaagccttcaagacttcg | 217424 | - | see also 16559 line |
| 37595 | CHST6 | NM_021615.2 | 1354 | ccgcctcgcacccccgaaattag | 217426 | - | see also 16559 line |
| 37596 | CHST1 | NM_003654.2 | 1217 | ggcgcaagtgcgggctactcaacc | 217427 | - | see also 16560 line |
| 37597 | CHST1 | NM_003654.2 | 1585 | tggctcggaacctatgaagaag | 217429 | - | see also 16560 line |
| 37598 | CHST10 | NM_004854.2 | 1349 | ccgtttcgaaggggactttaag | 217451 | - | see also 3374 line |
| 37599 | CHST10 | NM_004854.2 | 1199 | aggacgatgccccatacatctta | 217447 | - | see also 3374 line |
| 37600 | CHST2 | NM_004267.2 | 779 | gtcgttctcggcgagctattca | 217455 | - | see also 16562 line |
| 37601 | CHST2 | NM_004267.2 | 1552 | cactacggagagtgtacgatttt | 217461 | - | see also 16562 line |
| 37602 | CHST5 | NM_012126.1 | 1287 | agccaatcgaggccttccatact | 217468 | - | see also 16563 line |
| 37603 | CHST5 | NM_012126.1 | 1350 | acgcgttgccctcactaagatc | 217470 | - | see also 16563 line |
| 37604 | GALNAC4S-6ST | NM_014863.1 | 1466 | tggaatcgtcgcgctaagagatg | 217491 | - | |
| 37605 | GALNAC4S-6ST | NM_014863.1 | 1629 | tccacgatgtgggataataatgc | 217495 | - | see also 6063 line |
| 37606 | GP3ST | NM_022134.1 | 971 | ggcgcctgacgagcatttcaac | 217513 | - | see also 16565 line |
| 37607 | GP3ST | NM_022134.1 | 278 | cgccggtcaccaacatcatgttc | 217506 | - | see also 16565 line |
| 37608 | HS3ST5 | NM_153612.1 | 512 | tactaaattgcgcaaattcttc | 217539 | - | see also 16566 line |
| 37609 | HS3ST5 | NM_153612.1 | 493 | aggrtggaccccctgtcattact | 217538 | - | see also 16566 line |
| 37610 | NCAG1 | NM_032160.1 | 3482 | aacgatggactcaatttgttaat | 217607 | - | see also 16567 line |
| 37611 | NCAG1 | NM_032160.1 | 3902 | aactaatgcgatggatattaata | 217629 | - | see also 16567 line |
| 37612 | NDST4 | NM_022569.1 | 3035 | ctgggagttacacctcgttataa | 217735 | - | see also 8422 line |
| 37613 | NDST4 | NM_022569.1 | 2256 | accgcctaggttatatacttt | 217716 | - | see also 8422 line |
| 37614 | SULT1B1 | NM_014465.2 | 626 | accgactgatcttcctctaaat | 217756 | - | see also 16569 line |
| 37615 | SULT1B1 | NM_014465.2 | 596 | tccatcacccgattgtgaaaa | 217754 | - | see also 16569 line |
| 37616 | SULT1C1 | NM_001056.2 | 1162 | cccatgacaaatgttcacagt | 217781 | - | see also 648 line |
| 37617 | SULT2B1 | NM_004605.2 | 307 | tgcgggacgaacgaacatctttatc | 217786 | - | see also 16571 line |
| 37618 | SULT2B1 | NM_004605.2 | 308 | gcgggacgacgacatctttatca | 217787 | - | see also 16571 line |
| 37619 | SULT4A1 | NM_014351.2 | 581 | ttctgcgggaggttatgaatga | 217807 | - | see also 16572 line |
| 37620 | SULT4A1 | NM_014351.2 | 557 | agctaccgaggcacctttcaaga | 217804 | - | see also 16572 line |
| 37621 | SULT4A1 | NM_014351.2 | 584 | tgccggagtttatgaatgataa | 217809 | - | see also 16572 line |
| 37622 | UST | NM_005715.1 | 672 | accccgtcaaccggttcatcc | 217827 | - | see also 3968 line |

**EP 1 752 536 A1**

Figure 46

| | | | | | | |
|---|---|---|---|---|---|---|
| 37623 | UST | NM_005715.1 | 1262 | gagccaatcgacgatgaagaaca | - | see also 3968 line |
| 37624 | MPST | NM_021126.3 | 426 | ggccaccacgtcgtgatctacg | - | see also 16574 line |
| 37625 | TST | NM_003312.4 | 273 | ggcgtcgccctacgagatgatgc | - | see also 16575 line |
| 37626 | TST | NM_003312.4 | 306 | ggctggcttcgccgagtatgtgg | - | see also 16575 line |
| 37627 | TST | NM_003312.4 | 351 | cacgcacgtggtggtgtatgatg | - | see also 16575 line |
| 37628 | OXCT | NM_000436.1 | 1275 | caegtcgatctgacaatgctagg | - | see also 16576 line |
| 37629 | OXCT | NM_000436.1 | 208 | atcgccataccaagttttataca | - | see also 16576 line |
| 37630 | OXCT2 | NM_022120.1 | 871 | caegttcctaacatttatgttaga | - | - |
| 37631 | OXCT2 | NM_022120.1 | 200 | cceggctccgtgccaagttctac | - | see also 37630 line |
| 37632 | OXCT2 | NM_022120.1 | 868 | atccacgttcctaacatttatgt | - | see also 37630 line |
| 37633 | AGXT | NM_000030.1 | 1141 | tagaccaacttcgacattgagatc | - | see also 16577 line |
| 37634 | AGXT | NM_000030.1 | 893 | caggatgtaccatcacacacaatcc | - | see also 16577 line |
| 37635 | AGXT2 | NM_031900.1 | 98 | ttcccggacatcagtaaccaage | - | see also 16578 line |
| 37636 | AGXT2 | NM_031900.1 | 545 | cagggcgcactcaaacaacatag | - | see also 16578 line |
| 37637 | AGXT2L1 | NM_031279.2 | 1356 | gccgatggacctcatagaaatgt | - | see also 16579 line |
| 37638 | AGXT2L1 | NM_031279.2 | 232 | ttgcatcggatccatcaaaata | - | see also 16579 line |
| 37639 | BCAT1 | NM_005504.3 | 742 | agggcaactctgccggtatttga | - | see also 3846 line |
| 37640 | BCAT1 | NM_005504.3 | 744 | ggcaactctgccggtatttgaca | - | see also 3846 line |
| 37641 | BCAT2 | NM_001190.1 | 670 | ggggtcggcaactacaagttagg | - | see also 16581 line |
| 37642 | BCAT2 | NM_001190.1 | 668 | gcgggttcggcaactacaagtta | - | see also 16581 line |
| 37643 | GOT1 | NM_002079.1 | 379 | ttcttagccgttggtacaatgg | - | see also 1346 line |
| 37644 | GOT1 | NM_002079.1 | 383 | tagcgcgttggtacaatggaaca | - | see also 1346 line |
| 37645 | GOT2 | NM_002080.1 | 865 | agcgtgtaggccttcactatg | - | see also 16583 line |
| 37646 | GOT2 | NM_002080.1 | 936 | atcttgatccgtcccatgtattc | - | see also 16583 line |
| 37647 | TAT | NM_000353.1 | 1267 | gagttcacggagcggttagttgc | - | see also 16584 line |
| 37648 | TAT | NM_000353.1 | 497 | aagctaaggagagtcattcgaaca | - | see also 16584 line |
| 37649 | PSAT1 | NM_021154.3 | 119 | gccgcaactcagtgttgttagaga | - | see also 16585 line |
| 37650 | PSAT1 | NM_021154.3 | 499 | cagatgcctcctacgtgtattat | - | see also 16585 line |
| 37651 | OAT | NM_000274.1 | 774 | tccggatccagttacctaatgg | - | see also 16586 line |
| 37652 | OAT | NM_000274.1 | 92 | tacttagtcgcgagttcattct | - | see also 16586 line |
| 37653 | OAT | NM_000274.1 | 1328 | ttcgagagtccattgaaattatt | - | see also 16586 line |
| 37654 | ABAT | NM_000663.2 | 836 | accgttcccacgcctgaaatacc | - | see also 425 line |
| 37655 | ABAT | NM_000663.2 | 676 | agctggagacgtgcatgattaac | - | see also 425 line |
| 37656 | CCBL1 | NM_004059.2 | 947 | gccgtgaccacatgatacgtagc | - | see also 2735 line |
| 37657 | CCBL1 | NM_004059.2 | 614 | agcatgacgtgtgttgtatcact | - | see also 2735 line |
| 37658 | CCBL1 | NM_004059.2 | 355 | atcatcatcgaaccctttttga | - | see also 2735 line |
| 37659 | AADAT | NM_016228.3 | 1462 | gccgttaagatgtggggtattaat | - | see also 16589 line |

Figure 46

| 37660 | AADAT | NM_016228.3 | 397 | atccggaccatgactgacatatt | 218164 | - | see also 16589 line |
|---|---|---|---|---|---|---|---|
| 37661 | AADAT | NM_016228.3 | 491 | ctgccgtaatcactgtagaaaat | 218169 | - | see also 16589 line |
| 37662 | GPT2 | NM_133443.1 | 327 | agctgcagcggggtatcaaaaag | 218204 | - | see also 9578 line |
| 37663 | GPT2 | NM_133443.1 | 873 | atcctaaggtgctctgcataatc | 218213 | - | see also 9578 line |
| 37664 | MGC15875 | NM_032921.1 | 726 | agccaccggcgttgagtacttca | 218221 | - | see also 16591 line |
| 37665 | MGC15875 | NM_032921.1 | 729 | caccggcgttgagtacttcaaca | 218222 | - | see also 16591 line |
| 37666 | MGC33309 | NM_152413.1 | 472 | aggatgctcgtatagtttacatc | 218251 | - | see also 16592 line |
| 37667 | MGC33309 | NM_152413.1 | 465 | tggcataaggatgctcgtatagt | 218250 | - | see also 16592 line |
| 37668 | PHACS | NM_032592.1 | 1728 | ggcaaggccttcgagtgtaaaga | 218293 | - | see also 9257 line |
| 37669 | PHACS | NM_032592.1 | 1521 | gtgtacctgccggaaaaccatgc | 218289 | - | see also 9257 line |
| 37670 | PHACS | NM_032592.1 | 1394 | cacgctgtacacagaaaaccagg | 218288 | - | see also 9257 line |
| 37671 | SCLY | NM_016510.3 | 96 | cgccggagaggaaagtttatatg | 218297 | - | see also 16594 line |
| 37672 | SCLY | NM_016510.3 | 94 | ctcgccggagaggaaagtttata | 218295 | - | see also 16594 line |
| 37673 | TALDO1 | NM_006755.1 | 351 | ggccgagtatccacagaagtaga | 218318 | - | see also 16595 line |
| 37674 | TALDO1 | NM_006755.1 | 695 | gagtgtcactaaaatctacaact | 218329 | - | see also 16595 line |
| 37675 | ACAT2 | NM_005891.1 | 833 | aagtcagaagctgataaacgtgg | 218344 | - | see also 16596 line |
| 37676 | ACAT2 | NM_005891.1 | 1108 | atctggctgtcgaattcttgtga | 218352 | - | see also 16596 line |
| 37677 | ALG2 | NM_033087.1 | 288 | gcgcctacgtgcgcatggtttc | 218354 | - | see also 16597 line |
| 37678 | ALG2 | NM_033087.1 | 768 | agctgcgtggaagattgacatcc | 218367 | - | see also 16597 line |
| 37679 | C20orf155 | NM_019095.3 | 857 | agctgcatcagcttatagttact | 218403 | - | see also 7703 line |
| 37680 | C20orf155 | NM_019095.3 | 329 | cccatggacaatcccgaatatgt | 218378 | - | see also 7703 line |
| 37681 | CARKL | NM_013276.1 | 493 | ccgatgtagcagcgaattcctgg | 218409 | - | see also 16599 line |
| 37682 | CARKL | NM_013276.1 | 1309 | gtgccgaggcattgttcagaacc | 218414 | - | see also 16599 line |
| 37683 | CAS1 | NM_022900.3 | 2013 | aggttagaccgttatgtagtttt | 218484 | - | see also 8521 line |
| 37684 | CDSN | NM_001264.2 | 557 | ctcttaccgcggaatactaaacc | 218518 | - | see also 16601 line |
| 37685 | CDSN | NM_001264.2 | 1549 | agctagctgaccctgaagttttc | 218526 | - | see also 16601 line |
| 37686 | CDSN | NM_001264.2 | 555 | aactcttaccgcggaatactaaa | 218517 | - | see also 16601 line |
| 37687 | ChGn | NM_018371.3 | 1434 | cacgcttatcaatgttatcgtgc | 218547 | - | see also 7426 line |
| 37688 | ChGn | NM_018371.3 | 1341 | aggggaccacaaacacgaattca | 218542 | - | see also 7426 line |
| 37689 | CHST11 | NM_018413.2 | 924 | agcggtacggcaccaagatcatc | 218561 | - | see also 7453 line |
| 37690 | CHST11 | NM_018413.2 | 1211 | aagtctacgagaactactgatga | 218566 | - | see also 7453 line |
| 37691 | CHST12 | NM_018641.2 | 179 | tgctggggtcggtgttcatgatc | 218570 | - | see also 7534 line |
| 37692 | D4ST-1 | NM_130468.2 | 959 | tgcaggcgacgatgtcacattcc | 218579 | - | see also 16604 line |

Figure 46

| | | | | | | | |
|---|---|---|---|---|---|---|---|
| 37693 | D4ST-1 | NM_130468.2 | 1064 | ttgtgccgtgcactagacttg | 218581 | - | see also 16604 line |
| 37694 | DHDDS | NM_024887.1 | 765 | cccgttctgtggccagagtatac | 218597 | - | see also 8801 line |
| 37695 | FLJ12171 | NM_024619.2 | 738 | ttctacggccactcggaatatga | 218608 | - | see also 16606 line |
| 37696 | FLJ12171 | NM_024619.2 | 338 | agcccagctggcgatttacacc | 218606 | - | see also 16606 line |
| 37697 | FN3K | NM_022158.2 | 702 | tacgacccggcttccttctatgg | 218620 | - | see also 8317 line |
| 37698 | FN3K | NM_022158.2 | 487 | aggatgactggcgcacctttc | 218616 | - | see also 8317 line |
| 37699 | FN3K | NM_022158.2 | 600 | aagatcccggatctgtttgtgg | 218619 | - | see also 8317 line |
| 37700 | GAL3ST-4 | NM_024637.3 | 846 | cactacgctgcaacttactatg | 218625 | - | see also 16608 line |
| 37701 | GAL3ST-4 | NM_024637.3 | 843 | gaccactacgctgcaacttact | 218624 | - | see also 16608 line |
| 37702 | GAL3ST2 | NM_033036.1 | 1356 | cagccgcaaggtggacattatgg | 218637 | - | see also 16609 line |
| 37703 | GAL3ST2 | NM_033036.1 | 966 | ccgccaggtgaggaggtttct | 218636 | - | see also 16609 line |
| 37704 | GALGT2 | NM_153446.1 | 1078 | tccggagtattcgagagtattac | 218663 | - | see also 16610 line |
| 37705 | GALGT2 | NM_153446.1 | 1071 | atcatgctccggagtattcgaga | 218661 | - | see also 16610 line |
| 37706 | GALGT2 | NM_153446.1 | 243 | cgccgtcagggctcgagatttct | 218639 | - | see also 16610 line |
| 37707 | GALNACT-2 | NM_018590.3 | 1056 | gaacttacggaatagacatgt | 218695 | - | see also 7528 line |
| 37708 | GALNACT-2 | NM_018590.3 | 984 | gacttcgtagaaggttattatcg | 218694 | - | see also 7528 line |
| 37709 | GALNT12 | NM_024642.2 | 651 | tgcgcgagggggcgatgttctga | 218716 | - | see also 8675 line |
| 37710 | GALNT12 | NM_024642.2 | 1669 | gacagtttcgttccactcttacg | 218735 | - | see also 8675 line |
| 37711 | GALNT12 | NM_024642.2 | 1704 | ctcggatcatcagaaatggttct | 218737 | - | see also 8675 line |
| 37712 | GALNT14 | NM_024572.1 | 1624 | ctgcctcgatacagatatgttcg | 218766 | - | see also 16613 line |
| 37713 | GALNT14 | NM_024572.1 | 749 | tgccctgtcgatatcattaa | 218749 | - | see also 16613 line |
| 37714 | HS2ST1 | NM_012262.2 | 1343 | gcccatgccgttcgagaaaaaga | 218797 | - | see also 5242 line |
| 37715 | HS2ST1 | NM_012262.2 | 1342 | agcccatgccgttcgagaaaaag | 218796 | - | see also 5242 line |
| 37716 | HS6ST2 | NM_147174.2 | 465 | gccgcgtgggtcagaagaaatgc | 218803 | - | see also 9811 line |
| 37717 | HS6ST2 | NM_147174.2 | 1051 | ttgccatttacccagtataata | 218810 | - | see also 9811 line |
| 37718 | HS6ST3 | NM_153456.1 | 1208 | aacgcattgaggatctaaactc | 218827 | - | see also 9995 line |
| 37719 | HS6ST3 | NM_153456.1 | 1240 | cagtttacgagtatgcaaaga | 218830 | - | see also 9995 line |
| 37720 | KIAA0290 | NM_015122.1 | 1628 | ctcctcgtgcccgaaaacgtgg | 218843 | - | see also 6256 line |
| 37721 | KIAA0290 | NM_015122.1 | 635 | gcccaagtccgcgagaactacc | 218833 | - | see also 6256 line |
| 37722 | KIAA0290 | NM_015122.1 | 1944 | tgccctccacgtagccaatgg | 218844 | - | see also 6256 line |
| 37723 | MAT2B | NM_013283.3 | 1025 | gccaacgaacaccatttcgaatt | 218868 | - | see also 16618 line |
| 37724 | MAT2B | NM_013283.3 | 1024 | ggccaacgaacaccatttcgaat | 218867 | - | see also 16618 line |
| 37725 | MMAB | NM_052845.1 | 687 | ttcaacgctagccagatgcagc | 218880 | - | see also 16619 line |
| 37726 | MMAB | NM_052845.1 | 643 | atgcgaacgtggccaagttctta | 218879 | - | see also 16619 line |
| 37727 | MTRR | NM_002454.1 | 650 | gtcgagcttcgagattcgatga | 218903 | - | see also 16620 line |

Figure 46

| | | | | | | | | |
|---|---|---|---|---|---|---|---|---|
| 37728 | MTRR | NM_002454.1 | 1310 | gccgattatgccgctttgtacg | 218929 | - | | see also 16620 line |
| 37729 | MTRR | NM_002454.1 | 1718 | accggcatagcccccgtttattgg | 218944 | - | | see also 16620 line |
| 37730 | PIGM | NM_145167.1 | 646 | cgccggtaatgcggactctattgt | 218962 | - | | see also 16621 line |
| 37731 | PIGM | NM_145167.1 | 830 | tccgtcaattccggtacacttc | 218976 | - | | see also 16621 line |
| 37732 | PNR | NM_003967.1 | 484 | cccgcagcatacacttcgttatt | 219002 | - | | see also 16622 line |
| 37733 | PNR | NM_003967.1 | 123 | tgcagcaggcatgctgattatcg | 218998 | - | | see also 16622 line |
| 37734 | PTDSS1 | NM_014754.1 | 469 | ttcgagcaggttaaatctctaat | 219012 | - | | see also 5944 line |
| 37735 | TBDN100 | NM_025085.2 | 1096 | atgttagaacggctaaaaattta | 219067 | - | | see also 8853 line |
| 37736 | TBDN100 | NM_025085.2 | 679 | agggaaacgagggtatcagttact | 219050 | - | | see also 8853 line |
| 37737 | TPT | NM_014317.2 | 1134 | gacagagctcgacagtatgtact | 219144 | - | | see also 16625 line |
| 37738 | TPT | NM_014317.2 | 925 | tcgcctcagtacggaaaaaat | 219136 | - | | see also 16625 line |
| 37739 | TPT | NM_014317.2 | 916 | tgcatgagatcgcctatcagtac | 219135 | - | | see also 16625 line |
| 37740 | WBSCR17 | NM_022479.1 | 391 | gaccgttccattccggattatcg | 219152 | - | | see also 16626 line |
| 37741 | WBSCR17 | NM_022479.1 | 1763 | gtcagaggtggaccattaagaac | 219167 | - | | see also 16626 line |
| 37742 | AKAP28 | NM_178813.3 | 588 | tggttcgctttcgagaaaactgg | 219177 | - | | see also 16627 line |
| 37743 | AKAP28 | NM_178813.3 | 255 | agggagcgaaaccctttgaaaaac | 219171 | - | | see also 16627 line |
| 37744 | ALS2CR8 | NM_024744.12 | 1030 | tggacaggtacttcgtgtaattc | 219195 | - | | see also 8723 line |
| 37745 | ALS2CR8 | NM_024744.12 | 2582 | gcctccaccatgggtaaccttcc | 219233 | - | | see also 8723 line |
| 37746 | C9orf12 | NM_022755.3 | 610 | tacgctatgtgccttcctaattt | 219245 | - | | see also 16629 line |
| 37747 | C9orf12 | NM_022755.3 | 317 | accacggagagggcaataagagc | 219242 | - | | see also 16629 line |
| 37748 | | NM_003936.2 | 1120 | ttcttcacgcgagtcttcaaga | 219256 | - | kinase_related, cell_cycle | cyclin-dependent protein kinase 5 activator |
| 37749 | CDK5R2 | NM_003936.2 | 845 | ggcaagaccaggccttcattacg | 219255 | - | | see also 37748 line |
| 37750 | CDKN1B | NM_004064.2 | 926 | gacctgcaacgacgacgattcttct | 219266 | - | | see also 2742 line |
| 37751 | CDKN1B | NM_004064.2 | 898 | gcggagcaatgcgcaggaataag | 219264 | - | | see also 2742 line |

Figure 46

| | | | | | | | | | |
|---|---|---|---|---|---|---|---|---|---|
| 37752 | CDKN2A | NM_000077.2 | 677 | ggcaccagaggcagtaaccatgc | 219272 | - | kinase_related、 cell_cycle | - | hepatocellular carcinoma、 bladder transitional cell carcinoma、 rhabdomyosarcoma、 ependymoma、 soft tissue sarcoma、 colorectal cancer、 bladder cancer、 breast cancer、 lung cancer、 melanoma、 osteosarcoma、 pancreatic cancer、 Ewing's sarcoma、 ocular melanoma、 dysplastic nevus syndrome、 head and neck cancer、 colorectal cancers、 non-small-cell lung cancer、 squamous cell carcinomacytic、 nerve sheath tumors、 head and neck squamous cell carcinoma、 mesothelioma、 astrocytic tumors、 squamous cell cancer、 Wilms tumor、 bladder squamous cell carcinoma、 skin cancer、 Hodgkin's disease |
| 37753 | CINP | NM_032630.2 | 142 | atgcggctgattggcacaattta | 219277 | - | see also 16631 line | | |
| 37754 | CINP | NM_032630.2 | 143 | tgcggctgattggcacaatttaa | 219278 | - | see also 16631 line | | |
| 37755 | DKFZP434N1923 | NM_030974.2 | 655 | gcccctggagtcagtttcctaca | 219287 | - | see also 16632 line | | |
| 37756 | DVL3 | NM_004423.2 | 1622 | gtgacatggctgccatcgtaaaa | 219305 | - | see also 16633 line | | |
| 37757 | DVL3 | NM_004423.2 | 854 | atgcaacccggctaaatggaact | 219296 | - | see also 16633 line | | |
| 37758 | FLJ12476 | NM_022784.1 | 1354 | gtctacatgcagccgtcaaatct | 219335 | - | see also 16634 line | | |
| 37759 | FLJ12476 | NM_022784.1 | 2064 | ggccttaacctcgcatatagtga | 219352 | - | see also 16634 line | | |
| 37760 | GKAP42 | NM_025211.2 | 956 | ttcggaagatcacattagtaaaa | 219373 | - | see also 16635 line | | |
| 37761 | GKAP42 | NM_025211.2 | 440 | ttctcgttttgccctgttacaag | 219362 | - | see also 16635 line | | |
| 37762 | HINT3 | NM_138571.3 | 384 | tgcgagaatgaggacctaatttg | 219388 | - | see also 16636 line | | |
| 37763 | HINT3 | NM_138571.3 | 476 | cagaactctaaggaaagatcaag | 219391 | - | see also 16636 line | | |
| 37764 | IKBKAP | NM_003640.2 | 1440 | gaccggagcgtgggagataattc | 219419 | - | see also 2458 line | | |
| 37765 | IKBKAP | NM_003640.2 | 1441 | accggagcgtgggagataattca | 219420 | - | see also 2458 line | | |
| 37766 | IKBKAP | NM_003640.2 | 2133 | cagaccgaattggccatgattgg | 219441 | - | see also 2458 line | | |
| 37767 | ITPK1 | NM_014216.2 | 1040 | ggcagcacgccgtcattgacatc | 219501 | - | see also 5540 line | | |
| 37768 | ITPK1 | NM_014216.2 | 722 | caccctgcgtggtccagaatttc | 219496 | - | see also 5540 line | | |
| 37769 | LOC147463 | NM_173505.1 | 993 | ttccggcagaactaaccaaaaat | 219514 | - | see also 16639 line | | |

Figure 46

| | | | | | | |
|---|---|---|---|---|---|---|
| 37770 | LOC147463 | NM_173505.1 | 465 | gagcatccactgaatttaggacc | 219504 | - | see also 16639 line |
| 37771 | LOC55971 | NM_018842.3 | 255 | gagcaacttaccggaatgttatgg | 219518 | - | see also 16640 line |
| 37772 | LOC55971 | NM_018842.3 | 1708 | cgcccgactgtgacgaatgatcg | 219556 | - | see also 16640 line |
| 37773 | MADD | NM_003682.2 | 1966 | atgcgtctatgacagcaattcc | 219595 | - | see also 2499 line |
| 37774 | MADD | NM_003682.2 | 4397 | gtcgcacattgggcttgtgtaca | 219640 | - | see also 2499 line |
| 37775 | MAP3K7IP2 | NM_015093.2 | 1688 | cacctcacggacacctacattagc | 219700 | - | see also 6230 line |
| 37776 | NYD-SP25 | NM_033516.2 | 450 | tccaagagctggcttgatgttca | 219724 | - | see also 16643 line |
| 37777 | NYD-SP25 | NM_033516.2 | 242 | gacctctgtcggcacatatgaat | 219719 | - | see also 16643 line |
| 37778 | P101-PI3K | NM_014308.1 | 839 | caggcgggaggagaaaaagctgg | 219726 | - | see also 16644 line |
| 37779 | P101-PI3K | NM_014308.1 | 1711 | cacacggttcttcaaacttcagt | 219729 | - | see also 16644 line |
| 37780 | P15RS | NM_018170.2 | 719 | aagcaactcactgaatgttagc | 219751 | - | see also 16645 line |
| 37781 | P15RS | NM_018170.2 | 416 | aagcggaacttatgaacagtaaa | 219741 | - | see also 16645 line |
| 37782 | SKP2 | NM_005983.2 | 741 | gacctatcgaactcagttataga | 219766 | - | see also 4182 line |
| 37783 | SKP2 | NM_005983.2 | 1206 | ctcagtcgtgctatgtatataat | 219784 | - | see also 4182 line |
| 37784 | SKP2 | NM_005983.2 | 426 | gtccgcaggcctaagctaaatcg | 219756 | - | see also 4182 line |
| 37785 | STAP2 | NM_017720.1 | 1278 | gagcccaaagtctttaatggtgg | 219815 | - | see also 16647 line |
| 37786 | STAP2 | NM_017720.1 | 661 | cgcacgtggtccggccattacaag | 219809 | - | see also 16647 line |
| 37787 | TSKS | NM_021733.1 | 1478 | cagctgtcagaggctacacacaga | 219821 | - | see also 16648 line |
| 37788 | TSKS | NM_021733.1 | 1643 | ggccactctggaccatcctacact | 219823 | - | see also 16648 line |
| 37789 | DHRS2 | NM_005794.2 | 1231 | tacgtcaacgggagaacaattgc | 219831 | - | see also 16649 line |
| 37790 | DHRS2 | NM_005794.2 | 978 | ggcgctgggtgtctacaatgtca | 219828 | - | see also 16649 line |
| 37791 | HSD17B1 | NM_000413.1 | 1161 | ctggaacgtaaggactcaaaatc | 219834 | - | estradiol 17 beta-dehydrogenase |
| 37792 | HSD17B1 | NM_000413.1 | 1162 | tggacgtaaaggactcaaaatcc | 219835 | - | see also 37791 line |
| 37793 | HSD17B1 | NM_000413.1 | 1802 | cgccatgcaccggaagtgttcg | 219836 | - | see also 37791 line |
| 37794 | HSD17B2 | NM_002153.1 | 488 | cacggtatttgccggagttttga | 219845 | - | see also 16650 line |
| 37795 | HSD17B2 | NM_002153.1 | 496 | ttgccggagtttgaatgaaaat | 219847 | - | see also 16650 line |
| 37796 | HSD17B3 | NM_000197.1 | 241 | tcgttcgagctagcaaaacgtgg | 219871 | - | see also 16651 line |
| 37797 | HSD17B3 | NM_000197.1 | 238 | tactcgttcgagctagcaaaacg | 219870 | - | see also 16651 line |
| 37798 | HSD17B7 | NM_016371.1 | 733 | cgctgttgatcgcggcaatattg | 219893 | - | see also 16652 line |
| 37799 | HSD17B7 | NM_016371.1 | 690 | ttgaccaattgacatatggaat | 219891 | - | see also 16652 line |
| 37800 | HSD17B8 | NM_014234.3 | 446 | aagccctggtgtccaatggttgt | 219897 | - | see also 5551 line |
| 37801 | H105E3 | NM_015922.1 | 231 | gtcgcacgactcatttgacaga | 219902 | - | see also 6572 line |
| 37802 | H105E3 | NM_015922.1 | 643 | ttgagggcgtcgtatcaagaat | 219914 | - | see also 6572 line |
| 37803 | HSD3B1 | NM_000862.1 | 680 | tgccttacgacccatgtatatct | 219919 | - | see also 16655 line |

Figure 46

EP 1 752 536 A1

| | | | | | | | | | | |
|---|---|---|---|---|---|---|---|---|---|---|
| 37804 | HSD3B1 | NM_000862.1 | 775 | ttggaaagttctccactgttaac | 219920 | - | see also 16655 line | | | |
| 37805 | HSD3B2 | NM_000198.1 | 692 | tgcgttaagacccacatatatct | 219922 | - | see also 16656 line | | | |
| 37806 | HSD3B2 | NM_000198.1 | 886 | gtgtccgaggtcaattctattac | 219923 | - | see also 16656 line | | | |
| 37807 | HSD3B7 | NM_025193.2 | 1151 | gccggacccgtaccattctctgg | 219928 | - | see also 16657 line | | | |
| 37808 | HSD3B7 | NM_025193.2 | 554 | tgcacaggcacccctatccttgc | 219926 | - | see also 16657 line | | | |
| 37809 | HSPC105 | NM_145168.1 | 338 | ggcgggagcaactcaatcgaaac | 219937 | - | see also 16658 line | | | |
| 37810 | HSPC105 | NM_145168.1 | 532 | cactactctcggacaaagtcaat | 219943 | - | see also 16658 line | | | |
| 37811 | HSD11B1 | NM_005525.2 | 932 | ctctactcaacgagctataatat | 219978 | - | see also 3860 line | | | |
| 37812 | HSD11B1 | NM_005525.2 | 930 | ttctctactcaacgagctataat | 219977 | - | see also 3860 line | | | |
| 37813 | HSD11B2 | NM_000196.2 | 446 | ggccaccgtattggagttgaaca | 219981 | - | - | 11-beta-hydroxysteroid dehydrogenase | - | |
| 37814 | HSD11B2 | NM_000196.2 | 612 | aacaacgcaggccacaatgaagt | 219982 | - | see also 37813 line | | | |
| 37815 | HSD11B2 | NM_000196.2 | 1062 | tccgacctcaccccagttgtaga | 219985 | - | see also 37813 line | | | |
| 37816 | HADH2 | NM_004493.1 | 782 | ggctggatggggccattcgtatg | 219997 | - | see also 16660 line | | | |
| 37817 | HADH2 | NM_004493.1 | 578 | gggatctggctcccataggtatc | 219996 | - | see also 16660 line | | | |
| 37818 | LDHA | NM_005566.1 | 808 | tgcttatgaggtgatcaaactca | 220003 | - | see also 16661 line | | | |
| 37819 | LDHA | NM_005566.1 | 252 | atgtcatcgaagacaaattgaag | 220001 | - | see also 16661 line | | | |
| 37820 | LDHB | NM_002300.3 | 917 | atcccgtgtcaacaatggtaaag | 220035 | - | see also 16662 line | | | |
| 37821 | LDHB | NM_002300.3 | 998 | ggggattaaccagcgttatcaac | 220037 | - | see also 16662 line | | | |
| 37822 | LDHC | NM_002301.2 | 497 | aacgtaatgtggctataatgaaa | 220047 | - | see also 16663 line | | | |
| 37823 | LDHC | NM_002301.2 | 718 | tgccatggttggattattggaga | 220052 | - | see also 16663 line | | | |
| 37824 | LDHC | NM_002301.2 | 527 | ttcctgccatagtccattatagt | 220050 | - | see also 16663 line | | | |
| 37825 | LDHL | NM_033195.1 | 1126 | tcctgggagagaacggtattacc | 220064 | - | see also 16664 line | | | |
| 37826 | LDHL | NM_033195.1 | 1007 | atctgtggccgatttaacagaaa | 220063 | - | see also 16664 line | | | |
| 37827 | MGC23940 | NM_144972.1 | 323 | cacaataagatctccattgtagg | 220069 | - | see also 16665 line | | | |
| 37828 | MGC23940 | NM_144972.1 | 271 | aactatcaagagtgaacttatta | 220066 | - | see also 16665 line | | | |
| 37829 | UEV3 | NM_018314.2 | 629 | ctcggtattgcctgcacattagc | 220087 | - | see also 16666 line | | | |
| 37830 | UEV3 | NM_018314.2 | 515 | ttgctagcctatattgcaaaaat | 220082 | - | see also 16666 line | | | |
| 37831 | EHHADH | NM_001966.1 | 63 | cgccggtcaacgcgatcagtacg | 220102 | - | see also 16667 line | | | |
| 37832 | EHHADH | NM_001966.1 | 71 | aacgcgatcagtacgactttact | 220103 | - | see also 16667 line | | | |
| 37833 | EHHADH | NM_001966.1 | 73 | cgcgatcagtacgactttactcc | 220104 | - | see also 16667 line | | | |
| 37834 | HADHSC | NM_005327.1 | 721 | aaggacactcctgggtttattgt | 220176 | - | see also 16668 line | | | |
| 37835 | HADHSC | NM_005327.1 | 143 | cggcctcggccaagaagataatc | 220169 | - | see also 16668 line | | | |
| 37836 | IDH3A | NM_005530.2 | 430 | accccttacaccgatgtaaatat | 220192 | - | see also 16669 line | | | |

Figure 46

| 37837 | IDH3A | NM_005530.2 | 513 | tggagtcgtgcagagtatcaagc | 220196 | - | see also 16669 line | | |
|---|---|---|---|---|---|---|---|---|---|
| 37838 | IDH3B | NM_006899.2 | 353 | ttcataccccgatggagtataag | 220212 | - | see also 4832 line | | |
| 37839 | IDH3B | NM_006899.2 | 880 | gtccctggtgagagctatagtgc | 220228 | - | see also 4832 line | | |
| 37840 | IDH1 | NM_005896.2 | 603 | atgggtaaaacctatcatcatag | 220252 | - | see also 4099 line | | |
| 37841 | IDH1 | NM_005896.2 | 595 | gtgagtggatgggtaaaacctat | 220250 | - | see also 4099 line | | |
| 37842 | IDH2 | NM_002168.2 | 193 | ggcgccactatgccgacaaaagg | 220273 | - | see also 1390 line | | |
| 37843 | IDH2 | NM_002168.2 | 253 | atgagatgacccgtattatctgg | 220276 | - | see also 1390 line | | |
| 37844 | RDH5 | NM_002905.1 | 680 | gaggcgggatgtagctcattttg | 220296 | - | see also 16672 line | | |
| 37845 | RDH5 | NM_002905.1 | 345 | accaccctgttggatatcactga | 220294 | - | see also 16672 line | | |
| 37846 | RDH8 | NM_015725.1 | 258 | tgcaccccggactgtgttgatct | 220301 | - | receptor_acitivity | - | - |
| 37847 | RDH8 | NM_015725.1 | 833 | agcttctggcgcaggtttctatg | 220308 | - | see also 37846 line | | |
| 37848 | RDH8 | NM_015725.1 | 260 | caccccggactgtgttgatctcc | 220302 | - | see also 37846 line | | |
| 37849 | RODH-4 | NM_003708.2 | 1202 | tgcccacggctcccaatgagttg | 220314 | - | see also 16673 line | | |
| 37850 | RODH-4 | NM_003708.2 | 925 | agccacctgagagataagtatgt | 220312 | - | see also 16673 line | | |
| 37851 | AKR1A1 | NM_006066.2 | 612 | tacggcaatgagcctgagattgg | 220324 | - | see also 16674 line | | |
| 37852 | AKR1A1 | NM_006066.2 | 848 | tgctgatgggactatatgctacg | 220327 | - | see also 16674 line | | |
| 37853 | AKR1A1 | NM_006066.2 | 954 | ttcaacagtcggcagattgatga | 220328 | - | see also 16674 line | | |
| 37854 | AKR1B1 | NM_001628.2 | 613 | ctgcagttaaccagattgagtgc | 220339 | - | see also 16675 line | | |
| 37855 | AKR1B1 | NM_001628.2 | 80 | aagccgtctcctgctcaacaacg | 220336 | - | see also 16675 line | | |
| 37856 | AKR7A2 | NM_003689.1 | 305 | ggccaacccttgggatggaaaat | 220347 | - | - | electron transporter | - |
| 37857 | AKR7A2 | NM_003689.1 | 306 | gccaaccettgggatggaaaatc | 220348 | - | see also 37856 line | | |
| 37858 | HIBADH | NM_152740.1 | 899 | tccctcggctaataactatcagg | 220378 | - | see also 16676 line | | |
| 37859 | HIBADH | NM_152740.1 | 408 | cccaccagtatcaatgcaataga | 220360 | - | see also 16676 line | | |
| 37860 | IMPDH1 | NM_000883.2 | 1404 | gggcgtcgacgtcatagtcttgg | 220401 | - | see also 574 line | | |
| 37861 | IMPDH1 | NM_000883.2 | 1083 | ctcccgagacatcgactttcttg | 220393 | - | see also 574 line | | |
| 37862 | IMPDH2 | NM_000884.1 | 418 | aggatcgcgtgcgggatgttttt | 220416 | - | see also 16678 line | | |
| 37863 | IMPDH2 | NM_000884.1 | 1477 | cccaagtccgagccatgatgtac | 220428 | - | see also 16678 line | | |
| 37864 | IMPDH2 | NM_000884.1 | 462 | ttctgcggtatcccaatcacaga | 220417 | - | see also 16678 line | | |
| 37865 | ME1 | NM_002395.2 | 1376 | aaccaagggacgtgcaattttg | 220469 | - | see also 16679 line | | |
| 37866 | ME1 | NM_002395.2 | 486 | atccacgatcgagggcatattgc | 220444 | - | see also 16679 line | | |
| 37867 | ME1 | NM_002395.2 | 489 | cacgatcgagggcatattgcttc | 220445 | - | see also 16679 line | | |
| 37868 | ME3 | NM_006680.1 | 168 | ctgaagaagcgcggatacgatgt | 220484 | - | see also 16680 line | | |
| 37869 | ME3 | NM_006680.1 | 301 | tcctccgaatcatgagatattac | 220487 | - | see also 16680 line | | |
| 37870 | GBA2 | NM_020944.2 | 2330 | atggctccgcgtcaatgcatatt | 220540 | - | see also 8043 line | | |
| 37871 | GBA2 | NM_020944.2 | 2332 | ggctccgcgtcaatgcatattta | 220542 | - | see also 8043 line | | |
| 37872 | HMGCR | NM_000859.1 | 727 | gccgcgagggtcgtccaatttgg | 220593 | - | see also 16682 line | | |
| 37873 | HMGCR | NM_000859.1 | 726 | agccgcgagggtcgtccaatttg | 220592 | - | see also 16682 line | | |

Figure 46

| 37874 | GPD1 | NM_005276.2 | 367 | aacgccactggcatatctcttat | 220651 | - | see also 16683 line |
|---|---|---|---|---|---|---|---|
| 37875 | GPD1 | NM_005276.2 | 368 | acgccactggcatatctcttatt | 220652 | - | see also 16683 line |
| 37876 | KIAA0089 | NM_015141.2 | 624 | tcgaattaccgtggttgatgatg | 220668 | - | see also 16684 line |
| 37877 | KIAA0089 | NM_015141.2 | 556 | accaccatcggcagcaaagtaat | 220664 | - | see also 16684 line |
| 37878 | G6PD | NM_000402.2 | 1846 | tccgtgaggcctggcgtattttc | 220679 | - | see also 16685 line |
| 37879 | G6PD | NM_000402.2 | 1739 | gacctacggcaacagatacaaga | 220678 | - | see also 16685 line |
| 37880 | G6PD | NM_000402.2 | 1849 | gtgaggcctggcgtattttcacc | 220680 | - | see also 16685 line |
| 37881 | CBR1 | NM_001757.1 | 517 | atgagcgtcagagcccttaaaag | 220684 | - | see also 16686 line |
| 37882 | CBR1 | NM_001757.1 | 521 | gcgtcagagcccttaaaagctgc | 220685 | - | see also 16686 line |
| 37883 | CBR3 | NM_001236.2 | 318 | atccaatgccctttgacattaaa | 220693 | - | see also 16687 line |
| 37884 | CBR3 | NM_001236.2 | 315 | atgatccaatgccctttgacatt | 220692 | - | see also 16687 line |
| 37885 | HPGD | NM_000860.2 | 513 | agcgttggctgctaatcttatga | 220715 | - | see also 16688 line |
| 37886 | HPGD | NM_000860.2 | 398 | gaggtgaaggcggcatcattatc | 220705 | - | see also 16688 line |
| 37887 | MDH1 | NM_005917.2 | 952 | ttgttgaaggtctccctattaat | 220752 | - | see also 4138 line |
| 37888 | MDH1 | NM_005917.2 | 204 | ctggacggtgtcctaatggaact | 220729 | - | see also 4138 line |
| 37889 | MDH2 | NM_005918.2 | 137 | cagcacctcggcccagaacaatg | 220757 | - | see also 16690 line |
| 37890 | MDH2 | NM_005918.2 | 138 | agcacctcggcccagaacaatgc | 220758 | - | see also 16690 line |
| 37891 | SPR | NM_003124.2 | 786 | cacgtggacttctatgacaaata | 220779 | - | see also 16691 line |
| 37892 | SPR | NM_003124.2 | 787 | acgtggacttctatgacaaataa | 220780 | - | see also 16691 line |
| 37893 | PHGDH | NM_006623.2 | 772 | cacgacaggcttgctgaatgaca | 220782 | - | see also 16692 line |
| 37894 | PHGDH | NM_006623.2 | 184 | cccttgctgccggaagatcttgc | 220781 | - | see also 16692 line |
| 37895 | UGDH | NM_003359.1 | 1023 | ttcccggatcatagatagtctgt | 220813 | - | see also 2267 line |
| 37896 | UGDH | NM_003359.1 | 1253 | cccggctcgtgaccatttccaag | 220821 | - | see also 2267 line |
| 37897 | BDH | NM_004051.2 | 325 | tggagcaagacgcccactattgc | 220839 | - | see also 2731 line |
| 37898 | BDH | NM_004051.2 | 767 | gtgcggatgacgaaatcctttct | 220847 | - | see also 2731 line |
| 37899 | ME2 | NM_002396.2 | 1527 | ctctgtaacacccggcatattag | 220894 | - | see also 1541 line |
| 37900 | ME2 | NM_002396.2 | 1626 | tacccaccgcttgctaatattca | 220896 | - | see also 1541 line |
| 37901 | AKR1C4 | NM_001818.2 | 409 | gagacgccactaccaaaagatga | 220904 | - | see also 16696 line |
| 37902 | AKR1C4 | NM_001818.2 | 405 | aggtgagacgccactaccaaaag | 220903 | - | see also 16696 line |
| 37903 | AKR1C3 | NM_003739.4 | 658 | ttcaaccggagtaaattgctaga | 220914 | - | see also 16697 line |
| 37904 | AKR1C3 | NM_003739.4 | 662 | accggagtaaattgctagatttc | 220915 | - | see also 16697 line |
| 37905 | GRHPR | NM_012203.1 | 773 | caggggcgacgtcgtaaaccagg | 220930 | - | see also 5197 line |
| 37906 | GRHPR | NM_012203.1 | 770 | cagcaggggcgacgtcgtaaacc | 220929 | - | see also 5197 line |
| 37907 | UBE1DC1 | NM_024818.2 | 453 | acctttgccgtagcaatagtagg | 220947 | - | see also 8764 line |
| 37908 | UBE1DC1 | NM_024818.2 | 1022 | tgggatcttagtacaaaacgtgt | 220972 | - | see also 8764 line |
| 37909 | HAO1 | NM_017545.2 | 1013 | aagatgtcctcgagatactaaag | 221006 | - | see also 7024 line |
| 37910 | HAO1 | NM_017545.2 | 527 | tggatgatgtgcgtaacagattc | 220994 | - | see also 7024 line |

Figure 46

| 37911 | HAO2 | NM_016527.1 | 1083 | cggtcgctgagatcaatcgaaac | 221023 | - | see also 16701 line |
|---|---|---|---|---|---|---|---|
| 37912 | HAO2 | NM_016527.1 | 565 | caggcgacatgacattcgaaacc | 221012 | - | see also 16701 line |
| 37913 | CYP11B2 | NM_000498.2 | 1203 | gacattggtacaggttttcctct | 221025 | - | see also 16702 line |
| 37914 | CYP39A1 | NM_016593.3 | 493 | gtgcaaaatatcgtttatcgtac | 221034 | - | see also 6936 line |
| 37915 | CYP39A1 | NM_016593.3 | 339 | tgggaaagcccctctagaattta | 221029 | - | see also 6936 line |
| 37916 | CYP7B1 | NM_004820.2 | 331 | cccggtgagcctccattgataaa | 221066 | - | see also 16704 line |
| 37917 | CYP7B1 | NM_004820.2 | 389 | tacgaaaagacccccttaaggttc | 221069 | - | see also 16704 line |
| 37918 | CYP7B1 | NM_004820.2 | 1152 | agcagtgcgtgacgaaattgacc | 221094 | - | see also 16704 line |
| 37919 | CYP21A2 | NM_000500.4 | 242 | cagcccgacctcccaatctatct | 221109 | - | see also 16705 line |
| 37920 | CYP21A2 | NM_000500.4 | 724 | gagccactggtccatccaaattg | 221113 | - | see also 16705 line |
| 37921 | CYP17A1 | NM_000102.2 | 571 | gtcttcgtggcggtaaccaatgt | 221124 | - | see also 16706 line |
| 37922 | CYP17A1 | NM_000102.2 | 257 | ttcgtatgggcaccaagactaca | 221120 | - | see also 16706 line |
| 37923 | CYP17A1 | NM_000102.2 | 1338 | ctcaccgtcagtaagctatttgc | 221142 | - | see also 16706 line |
| 37924 | CYP8B1 | NM_004391.1 | 834 | ttcgcttctgctattacatcttg | 221151 | - | see also 2952 line |
| 37925 | CYP11A1 | NM_000781.1 | 324 | ggcaacgtggagtcggtttatgt | 221168 | - | see also 16708 line |
| 37926 | CYP11A1 | NM_000781.1 | 176 | tcctcgccccttcaatgagatcc | 221166 | - | see also 16708 line |
| 37927 | CYP11A1 | NM_000781.1 | 171 | cgcagtcctcgccccttcaatga | 221163 | - | see also 16708 line |
| 37928 | CYP7A1 | NM_000780.2 | 307 | tcccttgtcataccataaggtgt | 221187 | - | see also 481 line |
| 37929 | CYP7A1 | NM_000780.2 | 326 | gtgttgtgccacggaaaatattt | 221189 | - | see also 481 line |
| 37930 | CH25H | NM_003956.2 | 764 | tgcaacttcgctccgtactttac | 221229 | - | see also 16710 line |
| 37931 | CH25H | NM_003956.2 | 767 | aacttcgctccgtactttacaca | 221230 | - | see also 16710 line |
| 37932 | CYP27A1 | NM_000784.2 | 1402 | cccacaaactcccggatcataga | 221240 | - | see also 16711 line |
| 37933 | CYP27A1 | NM_000784.2 | 1410 | ctcccggatcatagaaaaggaaa | 221241 | - | see also 16711 line |
| 37934 | CYP46A1 | NM_006668.1 | 267 | tcctgagtcggttaagaagttcc | 221248 | - | see also 16712 line |
| 37935 | CYP46A1 | NM_006668.1 | 266 | gtcctgagtcggttaagaagttc | 221247 | - | see also 16712 line |
| 37936 | CYP46A1 | NM_006668.1 | 444 | ctccttggttagcttaatggaaa | 221255 | - | see also 16712 line |
| 37937 | FMO1 | NM_002021.1 | 752 | atgggtgatcagccgaatctttg | 221274 | - | see also 16713 line |
| 37938 | FMO1 | NM_002021.1 | 1144 | aggcctcactgtacaagtatatc | 221286 | - | see also 16713 line |
| 37939 | FMO3 | NM_006894.3 | 476 | atggtttgttccggacatcatgt | 221303 | - | see also 4825 line |
| 37940 | FMO3 | NM_006894.3 | 525 | ttccaggactaaaccactttaaa | 221306 | - | see also 4825 line |
| 37941 | FMO4 | NM_002022.1 | 1319 | atcggccttatcggccttaaagg | 221370 | - | see also 16715 line |
| 37942 | FMO4 | NM_002022.1 | 1323 | gccttatcggccttaaaggatcc | 221372 | - | see also 16715 line |
| 37943 | FMO4 | NM_002022.1 | 437 | ttccacgaagattatcctaattt | 221341 | - | see also 16715 line |
| 37944 | FMO5 | NM_001461.1 | 943 | gacctgccaaatcgtatcatttc | 221414 | - | see also 1015 line |
| 37945 | FMO5 | NM_001461.1 | 596 | tccacagtcgagactataagaac | 221401 | - | see also 1015 line |
| 37946 | FMO5 | NM_001461.1 | 944 | acctgccaaatcgtatcatttct | 221415 | - | see also 1015 line |
| 37947 | CYP4F8 | NM_007253.2 | 323 | atcgtccgatctgtcatcaaatac | 221441 | - | see also 16717 line |

Figure 46

| | | | | | | | | | |
|---|---|---|---|---|---|---|---|---|---|
| 37948 | CYP4F8 | NM_007253.2 | 1502 | cgcaggacgccggagattgtttt | 221451 | - | see also 16717 line | | |
| 37949 | SQLE | NM_003129.2 | 1791 | ttgcaatctatgccgtgtatttt | 221488 | - | see also 16718 line | | |
| 37950 | SQLE | NM_003129.2 | 888 | atcacggaagattcatcatgagt | 221465 | - | see also 16718 line | | |
| 37951 | SQLE | NM_003129.2 | 401 | ttcgccctcttctcggatattct | 221453 | - | see also 16718 line | | |
| 37952 | TYR | NM_000372.2 | 1268 | caccccacaaatcctaacttact | 221507 | - | see also 16719 line | | |
| 37953 | TYR | NM_000372.2 | 1466 | ctgagtttgacccaatatgaatc | 221513 | - | see also 16719 line | | |
| 37954 | KMO | NM_003679.1 | 1108 | cgcgatttcagacctatccatgt | 221550 | - | see also 16720 line | | |
| 37955 | KMO | NM_003679.1 | 829 | tccggatgccatccctctaattg | 221545 | - | see also 16720 line | | |
| 37956 | CYP27B1 | NM_000785.2 | 1460 | tggctttggcccagatcctaaca | 221569 | - | see also 16721 line | | |
| 37957 | CYP27B1 | NM_000785.2 | 829 | tgggaccagatgtttgcatttgc | 221562 | - | see also 16721 line | | |
| 37958 | CYP26A1 | NM_000783.2 | 758 | gcgcgcatcgagcagaacattcg | 221575 | - | see also 484 line | | |
| 37959 | CYP26A1 | NM_000783.2 | 885 | tgcaggcactaaagcaatcttca | 221576 | - | see also 484 line | | |
| 37960 | CYP2J2 | NM_000775.2 | 344 | gacccctatgcgagaacatatct | 221598 | - | see also 16723 line | | |
| 37961 | CYP2J2 | NM_000775.2 | 346 | cccctatgcgagaacatatcttt | 221599 | - | see also 16723 line | | |
| 37962 | CYP2J2 | NM_000775.2 | 964 | tgcgatgggctctgctttatatg | 221612 | - | see also 16723 line | | |
| 37963 | COQ6 | NM_182476.1 | 911 | tgcagcagagctagttagcatgg | 221634 | - | see also 16724 line | | |
| 37964 | COQ6 | NM_182476.1 | 1257 | ctcagtacggcagccttcaatgg | 221637 | - | see also 16724 line | | |
| 37965 | COQ6 | NM_182476.1 | 498 | ggctatatcgtggagaatgatgt | 221625 | - | see also 16724 line | | |
| 37966 | CYP19A1 | NM_000103.2 | 161 | acccgatacattataacatcacc | 221649 | - | see also 69 line | | |
| 37967 | CYP19A1 | NM_000103.2 | 155 | tgctgaacccgatacattataac | 221648 | - | see also 69 line | | |
| 37968 | CYP1A1 | NM_000499.2 | 1330 | aggggcgttgtgtctttgtaaac | 221689 | - | see also 16726 line | | |
| 37969 | CYP1A1 | NM_000499.2 | 141 | atgtcggccacggagtttcttct | 221678 | - | see also 16726 line | | |
| 37970 | CYP1A2 | NM_000761.2 | 614 | gggcacttcgacccttacaatca | 221697 | - | see also 16727 line | | |
| 37971 | CYP1A2 | NM_000761.2 | 1366 | cgccgatggcactgccattaaca | 221700 | - | see also 16727 line | | |
| 37972 | CYP1B1 | NM_000104.2 | 1137 | caccgttttccgcgaattcgagc | 221703 | - | see also 16728 line | | |
| 37973 | CYP1B1 | NM_000104.2 | 609 | gcgctacggcgacgttttccaga | 221701 | - | see also 16728 line | | |
| 37974 | CYP20A1 | NM_020674.2 | 905 | cagatggtaatgggtagtacatt | 221729 | - | see also 16729 line | | |
| 37975 | CYP20A1 | NM_020674.2 | 908 | atggtaatgggtagtacatttga | 221730 | - | see also 16729 line | | |
| 37976 | CYP24A1 | NM_000782.2 | 1785 | tgcattggtcgccgattagcaga | 221783 | - | see also 483 line | | |
| 37977 | CYP24A1 | NM_000782.2 | 1260 | gacaaccggttagagaagtattc | 221769 | - | see also 483 line | | |
| 37978 | CYP2A13 | NM_000766.3 | 1400 | cccctcagtcgcctaaggatatc | 221789 | - | - | human cytochromes P450 family | - |
| 37979 | CYP2A13 | NM_000766.3 | 1399 | tcccctcagtcgcctaaggatat | 221788 | - | see also 37978 line | | |
| 37980 | CYP2A13 | NM_000766.3 | 1401 | ccctcagtcgcctaaggatatcg | 221790 | - | see also 37978 line | | |
| 37981 | CYP2B6 | NM_000767.4 | 608 | ttgttctaccagactttttcact | 221792 | - | see also 16731 line | | |
| 37982 | CYP2B6 | NM_000767.4 | 625 | ttcactcatcagctctgtattcg | 221793 | - | see also 16731 line | | |
| 37983 | CYP2C18 | NM_000772.1 | 1346 | aagggcacgaccataataacatc | 221803 | - | see also 16732 line | | |

Figure 46

| ID | Gene | Accession | No. | Sequence | Description | Marker | See also | — | ID2 |
|---|---|---|---|---|---|---|---|---|---|
| 37984 | CYP2C18 | NM_000772.1 | 1347 | agggcacgaccataataacatc | | | see also 16732 line | - | 221804 |
| 37985 | CYP2C19 | NM_000769.1 | 657 | tcccactaattgattattcc | mephenytoin 4-hydroxylase C19 defect | CYP2C19*8 | - | - | 221806 |
| 37986 | CYP2C8 | NM_000770.2 | 736 | ctcttaacagaagttacattagg | | | see also 16733 line | - | 221813 |
| 37987 | CYP2C8 | NM_000770.2 | 1129 | cagtgaccactgatactaagttc | | | see also 16733 line | - | 221818 |
| 37988 | CYP2C9 | NM_000771.2 | 824 | agcacaaccaacatctgaattt | | | see also 16734 line | - | 221826 |
| 37989 | CYP2C9 | NM_000771.2 | 826 | cacaaccaaccatctgaatttac | | | see also 16734 line | - | 221827 |
| 37990 | CYP2D6 | NM_000106.3 | 1222 | atcccgtgacatcgaagtacagg | debrisoquine sensitivity, debrisoquine/sparteine polymorphism, Alzheimer disease, Parkinson disease, head and neck cancer, schizophrenia, Lewy body dementia | CYP2D6-Tyr481 | - | - | 221829 |
| 37991 | CYP2E1 | NM_000773.2 | 1477 | tcccacacgttacaaactctgt | | | see also 16735 line | - | 221848 |
| 37992 | CYP2E1 | NM_000773.2 | 872 | tgcagagcgcttgtacacaatgg | | | see also 16735 line | - | 221839 |
| 37993 | CYP2F1 | NM_000774.3 | 571 | gtcgctcagtgtccaacattatc | | | see also 16736 line | - | 221850 |
| 37994 | CYP2F1 | NM_000774.3 | 645 | accattatccgccttatccaatga | | | see also 16736 line | - | 221855 |
| 37995 | CYP2S1 | NM_030622.5 | 650 | gtcagacctacgagatgttctcc | | | see also 16737 line | - | 221859 |
| 37996 | CYP2S1 | NM_030622.5 | 167 | ggctcatgcggctgagtaagaag | | | see also 16737 line | - | 221857 |
| 37997 | CYP3A4 | NM_017460.3 | 258 | tcctaccataagggctttgtat | prostate cancer, leukemia | human cytochromes P450 family | - | - | 221861 |
| 37998 | CYP3A4 | NM_017460.3 | 789 | ctcatcccaatcttgaagtatt | | | see also 37997 line | - | 221863 |
| 37999 | CYP3A4 | NM_017460.3 | 262 | accataagggctttgtatgttt | | | see also 37997 line | - | 221862 |
| 38000 | CYP3A43 | NM_022820.3 | 1014 | ttgctgcctatgacacaactagc | | | see also 16738 line | - | 221873 |
| 38001 | CYP3A43 | NM_022820.3 | 899 | aagcatcgagtagaatctcttca | | | see also 16738 line | - | 221871 |
| 38002 | CYP3A5 | NM_000777.2 | 391 | ttcacaaatcgaaggtcttagg | | | see also 16739 line | - | 221879 |
| 38003 | CYP3A5 | NM_000777.2 | 1516 | gacacgcaaggacttcttcaacc | | | see also 16739 line | - | 221881 |
| 38004 | CYP3A7 | NM_000765.2 | 309 | tagaaagtcggggtatttag | | human cytochromes P450 family | - | - | 221882 |
| 38005 | CYP4B1 | NM_000779.2 | 1018 | gagcaccagcatcgttgtagaga | | | see also 16740 line | - | 221894 |
| 38006 | CYP4B1 | NM_000779.2 | 483 | cactgagtctacacgtatcatgc | | | see also 16740 line | - | 221889 |
| 38007 | CYP4F11 | NM_021187.1 | 1605 | caggaaacccgagctgatattgc | | | see also 16741 line | - | 221904 |
| 38008 | CYP4F11 | NM_021187.1 | 453 | tgctgtcgcaccaaggatatga | | | see also 16741 line | - | 221899 |
| 38009 | CYP4F12 | NM_023944.1 | 381 | tgcaccaaggataatctcttca | | human cytochromes P450 family | - | - | 221905 |
| 38010 | CYP4F2 | NM_001082.3 | 975 | gacgggaagaagttatctgatga | | | see also 16742 line | - | 221907 |
| 38011 | CYP4F3 | NM_000896.1 | 257 | ctcggaggaaggtctcctataca | | | see also 16743 line | - | 221908 |
| 38012 | CYP4F3 | NM_000896.1 | 265 | aaggtctcctatacacacaaage | | | see also 16743 line | - | 221911 |
| 38013 | CYP4X1 | NM_178033.1 | 651 | tccagcatcgtcgcctactaact | | | see also 16744 line | - | 221916 |
| 38014 | CYP4X1 | NM_178033.1 | 995 | aagttaagccgagtgttgaatca | | | see also 16744 line | - | 221929 |

Figure 46

| | | | | | | | | |
|---|---|---|---|---|---|---|---|---|
| 38015 | CYP51A1 | NM_000786.2 | 1835 | atccgttacaaacgaagatcaaa | - | 221954 | - | see also 16745 line |
| 38016 | CYP51A1 | NM_000786.2 | 767 | gggaagggagttgcatacgatgt | - | 221951 | - | see also 16745 line |
| 38017 | FLJ20359 | NM_017781.1 | 1089 | acgcgaatgggcactttgtgaag | - | 221961 | - | see also 16746 line |
| 38018 | FLJ20359 | NM_017781.1 | 1090 | cgcgaatgggcactttgtgaagc | - | 221962 | - | see also 16746 line |
| 38019 | FLJ39501 | NM_173483.1 | 746 | agcggtctccttgatatgtttg | - | 221971 | - | see also 16747 line |
| 38020 | FLJ39501 | NM_173483.1 | 1634 | ggcactaacactgctacgttcc | - | 221976 | - | see also 16747 line |
| 38021 | LOC116123 | NM_138784.1 | 694 | ttccatagtcgggaatacaaaag | - | 221984 | - | see also 16748 line |
| 38022 | LOC116123 | NM_138784.1 | 787 | gagctcagtcgtgtagcaaaaca | - | 221988 | - | see also 16748 line |
| 38023 | LOC116123 | NM_138784.1 | 479 | gcctcctgaattacattcgtttt | - | 221980 | - | see also 16748 line |
| 38024 | MICAL2 | NM_014632.1 | 2320 | ctggacgagccttcaaactttc | - | 222018 | - | see also 5799 line |
| 38025 | MICAL2 | NM_014632.1 | 476 | aggcgtggttcccacaaagagtat | - | 221994 | - | see also 5799 line |
| 38026 | P450RAI-2 | NM_019885.2 | 219 | gtcgtcgggggaggagaagtatg | - | 222031 | - | see also 16750 line |
| 38027 | P450RAI-2 | NM_019885.2 | 245 | acgtgttcaagacgcatttgttg | - | 222033 | - | see also 16750 line |
| 38028 | PAMCI | NM_005447.2 | 433 | gagcagcccaatatgccatttgt | - | 222044 | - | see also 16751 line |
| 38029 | PAMCI | NM_005447.2 | 1349 | cggacactcgtatcagttctaac | - | 222061 | - | see also 16751 line |
| 38030 | TBXAS1 | NM_001061.2 | 480 | atcttggtcgtcggatgtttatt | - | 222065 | - | see also 651 line |
| 38031 | TBXAS1 | NM_001061.2 | 571 | ggcgtcgggtttggagttcaagt | - | 222071 | - | see also 651 line |
| 38032 | TBXAS1 | NM_001061.2 | 957 | cccggatttgcccaataagaac | - | 222084 | - | see also 651 line |
| 38033 | TYRP1 | NM_000550.1 | 1519 | gccaagtcgggagtttagtgtac | - | 222130 | - | see also 16753 line |
| 38034 | TYRP1 | NM_000550.1 | 1401 | atgcccctattgacacataataga | - | 222126 | - | see also 16753 line |
| 38035 | FLJ13491 | NM_024623.1 | 1353 | cccgccacggtggatgtatgtgc | - | 222136 | - | - |
| 38036 | FLJ13491 | NM_024623.1 | 712 | tccagcggctggagacagatatcg | - | 222135 | - | see also 38035 line |
| 38037 | PLOD | NM_000302.2 | 1744 | ctgcccgatgtctattggttcc | - | 222148 | - | see also 16754 line |
| 38038 | PLOD | NM_000302.2 | 1868 | tacgagaacgtgccgactattga | - | 222149 | - | see also 16754 line |
| 38039 | PLOD2 | NM_000935.1 | 519 | tccatatgtcaacgtatagttc | - | 222170 | - | see also 615 line |
| 38040 | PLOD2 | NM_000935.1 | 146 | gtgatggattccatcgatttatg | - | 222156 | - | see also 615 line |
| 38041 | PLOD3 | NM_001084.3 | 573 | gacagaaggtccggtcggttaaag | - | 222227 | - | see also 16756 line |
| 38042 | PLOD3 | NM_001084.3 | 977 | ctcaacggggctttagatgaagt | - | 222236 | - | see also 16756 line |
| 38043 | P4HA1 | NM_000917.1 | 1111 | atgggacaagcctcgtattattc | - | 222283 | - | see also 593 line |
| 38044 | P4HA1 | NM_000917.1 | 1695 | gaccttgtcgttgttcagaattg | - | 222304 | - | see also 593 line |
| 38045 | P4HA2 | NM_004199.1 | 1383 | atcgtcgatgcagcatatcaca | - | 222322 | - | see also 16758 line |
| 38046 | P4HA2 | NM_004199.1 | 1500 | agcgagtactttcaagcattta | - | 222325 | - | see also 16758 line |
| 38047 | P4HA2 | NM_004199.1 | 927 | tgcggtacttgagcagttattg | - | 222314 | - | see also 16758 line |
| 38048 | P4HB | NM_000918.2 | 1391 | tgccgacagacgtcattgatt | - | 222335 | - | see also 16759 line |

Figure 46

| 38049 | P4HB | NM_000918.2 | 1251 | ttggctcccatttgggataaact | 222333 | - | see also 16759 line | | |
|---|---|---|---|---|---|---|---|---|---|
| 38050 | LOC57168 | NM_020437.3 | 1240 | atccgcatccgatgccatttagg | 222348 | - | see also 16760 line | | |
| 38051 | LOC57168 | NM_020437.3 | 1243 | cgcatccgatgccatttaggtct | 222349 | - | see also 16760 line | | |
| 38052 | LOC57168 | NM_020437.3 | 1389 | tggcccacgggtggttttcatgg | 222350 | - | see also 16760 line | | |
| 38053 | BBOX1 | NM_003986.1 | 294 | cacatggcccgatgagcattaca | 222362 | - | see also 2723 line | | |
| 38054 | BBOX1 | NM_003986.1 | 944 | acgcaactagggacacaatattt | 222385 | - | see also 2723 line | | |
| 38055 | EGLN1 | NM_022051.1 | 4015 | ctgggcagctacaaaatcaatgg | 222390 | - | see also 16762 line | | |
| 38056 | EGLN2 | NM_017555.1 | 2519 | tacgtaaggcacgttgacaatcc | 222394 | - | signal_transduction | - | - |
| 38057 | EGLN3 | NM_022073.2 | 926 | ccctcttacgcaaccagatatgc | 222402 | - | see also 16763 line | | |
| 38058 | EGLN3 | NM_022073.2 | 646 | gagccggctgggcaaatactacg | 222397 | - | see also 16763 line | | |
| 38059 | FLJ10826 | NM_018233.1 | 1192 | accggtcactacactttaattca | 222418 | - | see also 16764 line | | |
| 38060 | FLJ10826 | NM_018233.1 | 1191 | gaccggtcactacactttaattc | 222417 | - | see also 16764 line | | |
| 38061 | FLJ22222 | NM_024648.1 | 865 | gcggagggcggttcatgttcatg | 222429 | - | see also 16765 line | | |
| 38062 | FLJ22222 | NM_024648.1 | 863 | cggcggagggcggttcatgttca | 222428 | - | see also 16765 line | | |
| 38063 | LEPRE1 | NM_022356.2 | 936 | ttcctcccatcgcattataatta | 222441 | - | see also 8344 line | | |
| 38064 | LEPRE1 | NM_022356.2 | 1704 | ctggatacgcccctctactttc | 222451 | - | see also 8344 line | | |
| 38065 | LEPREL2 | NM_014262.2 | 753 | ccgtcgggagccctacaactacc | 222465 | - | see also 16767 line | | |
| 38066 | LEPREL2 | NM_014262.2 | 752 | gccgtcgggagccctacaactac | 222464 | - | see also 16767 line | | |
| 38067 | MLAT4 | NM_018192.2 | 1776 | gagcgctcgtctgttttatgaca | 222504 | - | see also 7311 line | | |
| 38068 | MLAT4 | NM_018192.2 | 1996 | tacacatttcgagactatagtgc | 222510 | - | see also 7311 line | | |
| 38069 | MLAT4 | NM_018192.2 | 1377 | cactgaaccgaattattggatca | 222492 | - | see also 7311 line | | |
| 38070 | PTGS1 | NM_000962.2 | 565 | gtgagctattacactcgtattct | 222526 | - | see also 16769 line | | |
| 38071 | PTGS1 | NM_000962.2 | 838 | gacaatctggagcgtcagtatca | 222531 | - | see also 16769 line | | |
| 38072 | PTGS2 | NM_000963.1 | 1885 | cccgctccggactagatgatatc | 222592 | - | see also 16770 line | | |
| 38073 | PTGS2 | NM_000963.1 | 1883 | ttcccgctccggactagatgata | 222590 | - | see also 16770 line | | |
| 38074 | PTGS2 | NM_000963.1 | 1884 | tcccgctccggactagatgatat | 222591 | - | see also 16770 line | | |
| 38075 | HMOX1 | NM_002133.1 | 407 | agccatgcagcgctatgtgaagc | 222596 | - | see also 16771 line | | |
| 38076 | HMOX1 | NM_002133.1 | 692 | gactgcgttcctgctcaacatcc | 222599 | - | see also 16771 line | | |
| 38077 | HMOX2 | NM_002134.2 | 297 | cacggcactttacttcacatact | 222605 | - | see also 16772 line | | |
| 38078 | HMOX2 | NM_002134.2 | 290 | tggccaccacggcactttacttc | 222604 | - | see also 16772 line | | |
| 38079 | DAP13 | NM_018838.3 | 107 | ggcggtctccgaggctatctacg | 222609 | - | see also 16773 line | | |
| 38080 | DAP13 | NM_018838.3 | 151 | tgcgaaggttggtacattagtgg | 222612 | - | see also 16773 line | | |
| 38081 | DAP13 | NM_018838.3 | 230 | caccgatgggttgtatatactac | 222615 | - | see also 16773 line | | |
| 38082 | GRIM19 | NM_015965.3 | 324 | cgcggtcggatagttacactact | 222634 | - | see also 16774 line | | |
| 38083 | GRIM19 | NM_015965.3 | 628 | gcgcaggcgcctacaaatcgagg | 222638 | - | see also 16774 line | | |
| 38084 | NDUFA11 | NM_175614.1 | 64 | gcctacagcaccaccagtattgc | 222641 | - | see also 16775 line | | |
| 38085 | NDUFA11 | NM_175614.1 | 394 | ggctgggaggtgtttgcaaaacc | 222643 | - | see also 16775 line | | |

Figure 46

| 38086 | NDUFA2 | NM_002488.2 | 216 | atcccgacctacccatcctaatc | 222646 | - | see also 16776 line | | |
|---|---|---|---|---|---|---|---|---|---|
| 38087 | NDUFA2 | NM_002488.2 | 125 | gagattcgcatccacttatgtca | 222645 | - | see also 16776 line | | |
| 38088 | NDUFA3 | NM_004542.1 | 126 | ccccattgagcccctacttcaag | 222650 | - | - | NADH dehydrogenase (ubiquinone) | - |
| 38089 | NDUFA6 | NM_002490.2 | 14 | ggcgtccgccaagctacttctac | 222651 | - | see also 1592 line | | |
| 38090 | NDUFA6 | NM_002490.2 | 334 | agcgccaaggccaaaggatttcc | 222660 | - | see also 1592 line | | |
| 38091 | NDUFA7 | NM_005001.1 | 169 | agctctccaacaattactattgc | 222663 | - | see also 16778 line | | |
| 38092 | NDUFA7 | NM_005001.1 | 162 | agccacaagctctccaacaatta | 222661 | - | see also 16778 line | | |
| 38093 | NDUFA8 | NM_014222.2 | 552 | taccggagaatccctatcactca | 222668 | - | see also 16779 line | | |
| 38094 | NDUFA8 | NM_014222.2 | 630 | atggcagccgcttttatttctgg | 222669 | - | see also 16779 line | | |
| 38095 | NDUFA9 | NM_005002.3 | 352 | ttctatccgacgagtagtacaac | 222681 | - | see also 16780 line | | |
| 38096 | NDUFA9 | NM_005002.3 | 888 | tgccgcttttgcctatcgatgg | 222694 | - | see also 16780 line | | |
| 38097 | NDUFB1 | NM_004545.3 | 281 | tgccttccggaacaagagtatgt | 222704 | - | see also 16781 line | | |
| 38098 | NDUFB1 | NM_004545.3 | 222 | gtccctatgggatttgtcattgg | 222702 | - | see also 16781 line | | |
| 38099 | NDUFB10 | NM_004548.1 | 222 | accctcgtgagagaatttataga | 222708 | - | see also 16782 line | | |
| 38100 | NDUFB10 | NM_004548.1 | 221 | gaccctcgtgagagaatttatag | 222707 | - | see also 16782 line | | |
| 38101 | NDUFB3 | NM_002491.1 | 296 | aacttccagattatagacaatgg | 222712 | - | - | NADH dehydrogenase (ubiquinone) | - |
| 38102 | NDUFB5 | NM_002492.2 | 383 | aagatggattgcccgtaatttct | 222720 | - | see also 16783 line | | |
| 38103 | NDUFB5 | NM_002492.2 | 386 | atggattgcccgtaatttctatg | 222721 | - | see also 16783 line | | |
| 38104 | NDUFB6 | NM_002493.3 | 131 | gggtacactccggatgagaaact | 222737 | - | see also 16784 line | | |
| 38105 | NDUFB6 | NM_002493.3 | 302 | atggtccatggggtatacaaaaa | 222738 | - | see also 16784 line | | |
| 38106 | NDUFB8 | NM_005004.1 | 342 | ggcacctagacatgtacaacagg | 222742 | - | see also 16786 line | | |
| 38107 | NDUFB9 | NM_005005.1 | 344 | acctcctatgagagatacgattg | 222748 | - | see also 16787 line | | |
| 38108 | NDUFB9 | NM_005005.1 | 169 | gtgcgtccagagagacaaatacc | 222743 | - | see also 16787 line | | |
| 38109 | NDUFC1 | NM_002494.2 | 70 | gcccttcagtgcgatcaaagttc | 222752 | - | see also 16788 line | | |
| 38110 | NDUFC1 | NM_002494.2 | 74 | ttcagtgcgatcaaagttctacg | 222754 | - | see also 16788 line | | |
| 38111 | NDUFC2 | NM_004549.3 | 311 | tgcatcgccagcttctatatatt | 222762 | - | see also 16789 line | | |
| 38112 | NDUFC2 | NM_004549.3 | 314 | atcgccagcttctatatattacg | 222763 | - | see also 16789 line | | |
| 38113 | NDUFS1 | NM_005006.5 | 892 | tgcggttggaagtaatattgtgg | 222787 | - | see also 3499 line | | |
| 38114 | NDUFS1 | NM_005006.5 | 1004 | gcctatgatgggctaaaacgtca | 222789 | - | see also 3499 line | | |
| 38115 | NDUFS1 | NM_005006.5 | 1033 | taccgagccaatggtcagaaatg | 222793 | - | see also 3499 line | | |
| 38116 | NDUFS2 | NM_004550.3 | 851 | tactttgaccggctagactatgt | 222847 | - | see also 16791 line | | |
| 38117 | NDUFS2 | NM_004550.3 | 1702 | cccttatcgatgcaagatcaagg | 222869 | - | see also 16791 line | | |
| 38118 | NDUFS3 | NM_004551.1 | 374 | ctcggcaaaaccgttttgagatt | 222879 | - | see also 16792 line | | |

Figure 46

| | | | | | | | |
|---|---|---|---|---|---|---|---|
| 38119 | NDUFS3 | NM_004551.1 | 626 | ctggctatgttgagttacgttat | 222886 | - | see also 16792 line |
| 38120 | NDUFS4 | NM_002495.1 | 356 | aacggctgatccttatccaaca | 222895 | - | see also 1594 line |
| 38121 | NDUFS4 | NM_002495.1 | 371 | atccaacatggttctaaccttca | 222896 | - | see also 1594 line |
| 38122 | NDUFS6 | NM_004553.2 | 306 | ggccacccaaagttgtatataaa | 222905 | - | see also 16794 line |
| 38123 | NDUFS6 | NM_004553.2 | 144 | cacactggccaggtttatgatga | 222901 | - | see also 16794 line |
| 38124 | NDUFS6 | NM_004553.2 | 310 | acccaaaagtgtatataaacttg | 222907 | - | see also 16794 line |
| 38125 | NDUFS8 | NM_002496.1 | 408 | tccgggagagcgttgcattgc | 222913 | - | see also 16795 line |
| 38126 | NDUFS8 | NM_002496.1 | 194 | caactacaagtatgtgaacatgc | 222911 | - | see also 16795 line |
| 38127 | NDUFV1 | NM_007103.2 | 1172 | cccgcccattgagttcttataag | 222921 | - | see also 4951 line |
| 38128 | NDUFV1 | NM_007103.2 | 1321 | cagatagaaggccatacgatttg | 222923 | - | see also 4951 line |
| 38129 | NDUFV2 | NM_021074.1 | 310 | atgaacaagttgcagaagtttt | 222926 | - | see also 16797 line |
| 38130 | NDUFV2 | NM_021074.1 | 575 | ttcaaataaatgacaattactat | 222932 | - | see also 16797 line |
| 38131 | P17.3 | NM_019056.2 | 544 | acccgaggacgaaaacttgtatg | 222937 | - | - |
| 38132 | P17.3 | NM_019056.2 | 543 | aaaccgaggacgaaaaacttgtat | 222936 | - | see also 38131 line |
| 38133 | P17.3 | NM_019056.2 | 545 | cccgaggacgaaaacttgtatga | 222938 | - | see also 38131 line |
| 38134 | NQO1 | NM_000903.1 | 431 | cacttacgctgccatgatgaca | 222953 | - | see also 16798 line |
| 38135 | NQO1 | NM_000903.1 | 234 | gaccctgcgaactttcagtatcc | 222944 | - | see also 16798 line |
| 38136 | NQO2 | NM_000904.1 | 641 | tacacgaagaacaggagtcaatgg | 222972 | - | see also 16799 line |
| 38137 | NQO2 | NM_000904.1 | 453 | gggaggctgacctagtgatattt | 222968 | - | see also 16799 line |
| 38138 | CYB561 | NM_001915.2 | 454 | tggtgcgggatcctgtcttgt | 222974 | - | see also 16800 line |
| 38139 | CYB561 | NM_001915.2 | 725 | gggcggtgctctacatccttgacc | 222977 | - | see also 16800 line |
| 38140 | CYB5R2 | NM_016229.1 | 510 | aggggacgcttgttttaccatg | 222991 | - | see also 16801 line |
| 38141 | CYB5R2 | NM_016229.1 | 588 | gccgatcacctgggaatgattgc | 222995 | - | see also 16801 line |
| 38142 | CYB5R2 | NM_016229.1 | 559 | accagacgagtgagcctaaaaaa | 222992 | - | see also 16801 line |
| 38143 | DIA1 | NM_000398.3 | 295 | tacctctcggctcgaattgatg | 222999 | - | see also 16802 line |
| 38144 | DIA1 | NM_000398.3 | 292 | atctacctccggctcgaattga | 222998 | - | see also 16802 line |
| 38145 | DIA1 | NM_000398.3 | 300 | ctcggctcgaattgatgcgaaacc | 223000 | - | see also 16802 line |
| 38146 | NCB5OR | NM_016230.2 | 208 | tggattcgactgaccaaaagtgg | 223006 | - | see also 16803 line |
| 38147 | NCB5OR | NM_016230.2 | 584 | aagaaggtcctagttatccaagc | 223020 | - | see also 16803 line |
| 38148 | NNT | NM_012343.2 | 663 | atcaggaacgctgattagtttta | 223066 | - | see also 5302 line |
| 38149 | NNT | NM_012343.2 | 2445 | ggcctacctcggccacttacattg | 223113 | - | see also 5302 line |
| 38150 | DHDH | NM_014475.2 | 646 | gtcgtgggaagggcgtcatgaaac | 223155 | - | see also 16805 line |
| 38151 | DHDH | NM_014475.2 | 892 | aacgggcgcaggcatgagttatga | 223157 | - | see also 16805 line |
| 38152 | POR | NM_000941.1 | 1989 | cgcgcaggcggtggactacatca | 223169 | - | see also 16806 line |
| 38153 | POR | NM_000941.1 | 173 | aagtcccgcgagttcaccaaaatt | 223163 | - | see also 16806 line |
| 38154 | TXNRD2 | NM_006440.2 | 1589 | gccccaacgcaggcgaagttact | 223186 | - | see also 16807 line |
| 38155 | TXNRD2 | NM_006440.2 | 1591 | cccaacgcaggcgaagttactca | 223187 | - | see also 16807 line |

Figure 46

| | | | | | | |
|---|---|---|---|---|---|---|
| 38156 | PRDX3 | NM_006793.2 | 788 | gagtactttcagtaaggtaatca | 223196 | - | see also 16808 line |
| 38157 | PRDX3 | NM_006793.2 | 771 | gtccagctgcttccaaagagtac | 223194 | - | see also 16808 line |
| 38158 | GMPR | NM_006877.2 | 212 | aacgcaccttcacgtttcgaaat | 223199 | - | see also 16809 line |
| 38159 | GMPR | NM_006877.2 | 707 | agctgagtgccgtcattgagtgt | 223208 | - | see also 16809 line |
| 38160 | GMPR | NM_006877.2 | 217 | accttcacgtttcgaaattcaaa | 223200 | - | see also 16809 line |
| 38161 | GMPR2 | NM_016576.2 | 883 | gtgtgttactactcggaagaaaac | 223216 | - | see also 6920 line |
| 38162 | GMPR2 | NM_016576.2 | 1338 | cacccagcagtgaatccaatct | 223217 | - | see also 6920 line |
| 38163 | GMPR2 | NM_016576.2 | 1343 | agcaggtgaatccaatcttcagt | 223218 | - | see also 6920 line |
| 38164 | NOX1 | NM_007052.3 | 465 | agccgcacactgagaaagcaatt | 223222 | - | see also 4939 line |
| 38165 | NOX1 | NM_007052.3 | 530 | ctgcctacatacagctattcaca | 223226 | - | see also 4939 line |
| 38166 | AASS | NM_005763.2 | 953 | aacatccgagcgctacataagt | 223306 | - | see also 4023 line |
| 38167 | AASS | NM_005763.2 | 1450 | aacggtacattacctgataaata | 223330 | - | see also 4023 line |
| 38168 | AASS | NM_005763.2 | 1384 | gacggacacagcctcttgaaag | 223323 | - | see also 4023 line |
| 38169 | CAT | NM_001752.1 | 1024 | acggaatccagttaattacttt | 223400 | - | see also 16813 line |
| 38170 | CAT | NM_001752.1 | 1388 | tactcaggtgcgggcattctatg | 223415 | - | see also 16813 line |
| 38171 | CAT | NM_001752.1 | 1579 | agcctaagaatgcgattcacacc | 223416 | - | see also 16813 line |
| 38172 | EPX | NM_000502.1 | 1516 | agctggcggatcgtgatgaagg | 223431 | - | see also 16814 line |
| 38173 | EPX | NM_000502.1 | 319 | cagcggtccggaccttcaatgt | 223420 | - | see also 16814 line |
| 38174 | FLJ20473 | NM_017836.2 | 1272 | aggcctgatccaggtgacaatcc | 223460 | - | see also 16815 line |
| 38175 | FLJ20473 | NM_017836.2 | 964 | tacaaaggcatggcgatatttac | 223451 | - | see also 16815 line |
| 38176 | FLJ25471 | NM_144651.1 | 1419 | cacgtttgcagcggaaattcagg | 223487 | - | see also 16816 line |
| 38177 | FLJ25471 | NM_144651.1 | 1417 | agcacgtttgcagcggaaattca | 223486 | - | see also 16816 line |
| 38178 | GPR156 | NM_153002.1 | 1616 | cacgggttagttcagtaatcagg | 223519 | - | see also 16817 line |
| 38179 | GPR156 | NM_153002.1 | 1612 | cggtcacgggttagttcagtaat | 223517 | - | see also 16817 line |
| 38180 | GPX2 | NM_002083.2 | 631 | atcaagcgcctccttaaagttgc | 223532 | - | see also 16818 line |
| 38181 | GPX2 | NM_002083.2 | 94 | ttcattgccaagtccttcatga | 223527 | - | see also 16818 line |
| 38182 | GPX2 | NM_002083.2 | 490 | accgatcccaagctcatcatttg | 223530 | - | see also 16818 line |
| 38183 | GPX3 | NM_002084.2 | 568 | accattcggtctggtcattctgg | 223539 | - | see also 16819 line |
| 38184 | GPX3 | NM_002084.2 | 904 | cacggtcagcaacgtcaagatgg | 223541 | - | see also 16819 line |
| 38185 | GPX4 | NM_002085.1 | 298 | gaggcaagaccgaagtaaactac | 223544 | - | see also 16820 line |
| 38186 | GPX4 | NM_002085.1 | 443 | gggctacaacgtcaaattcgata | 223547 | - | see also 16820 line |
| 38187 | GPX5 | NM_001509.1 | 489 | ttgggcacattcaaatctatatc | 223558 | - | see also 16821 line |
| 38188 | GPX5 | NM_001509.1 | 638 | ggcgtacttgaagcaattcaaaa | 223561 | - | see also 16821 line |
| 38189 | GPX6 | NM_015696.2 | 369 | ctcattcccatgtttagccaaga | 223564 | - | see also 16822 line |
| 38190 | GPX6 | NM_015696.2 | 373 | ttcccatgtttagccaagattgc | 223565 | - | see also 16822 line |
| 38191 | HCCA3 | NM_020232.3 | 453 | ctcccttccggtacctacttaca | 223578 | - | see also 16823 line |
| 38192 | HCCA3 | NM_020232.3 | 455 | ccttccggtacctacttacacc | 223579 | - | see also 16823 line |

Figure 46

| | | | | | | | |
|---|---|---|---|---|---|---|---|
| 38193 | HCCA3 | NM_020232.3 | 691 | tgcattaggtcttgttgagtatc | 223585 | - | see also 16823 line |
| 38194 | MMS19L | NM_022362.2 | 2595 | tgccgaagtgcggatcatgttcc | 223627 | - | see also 16824 line |
| 38195 | MMS19L | NM_022362.2 | 2620 | cagcggttcttcacagataatgt | 223629 | - | see also 16824 line |
| 38196 | PI16 | NM_153370.1 | 1039 | aacggggattccggctttcttgg | 223637 | - | see also 9994 line |
| 38197 | PI16 | NM_153370.1 | 1112 | acccaggccccaacttccttagc | 223639 | - | see also 9994 line |
| 38198 | PRDX1 | NM_002574.2 | 588 | gtcatctagcatgggtcaataca | 223646 | - | see also 16825 line |
| 38199 | PRDX1 | NM_002574.2 | 635 | cccatgaacattcctttggtatc | 223650 | - | see also 16825 line |
| 38200 | PRDX4 | NM_006406.1 | 476 | gtggtagcatgctctgttgattc | 223679 | - | see also 4493 line |
| 38201 | PRDX4 | NM_006406.1 | 271 | aagcaaagcgaagatttccaagc | 223668 | - | see also 4493 line |
| 38202 | RTDR1 | NM_014433.2 | 788 | cgcccgtgcgctccttaatgtca | 223694 | - | see also 16828 line |
| 38203 | RTDR1 | NM_014433.2 | 790 | cccgtgcgctccttaatgtcagc | 223695 | - | see also 16828 line |
| 38204 | TPRA40 | NM_016372.1 | 607 | acctcgaacgctgcaactgttgc | 223701 | - | see also 16829 line |
| 38205 | TPRA40 | NM_016372.1 | 344 | cacccctggcaccaaacatcagt | 223700 | - | see also 16829 line |
| 38206 | ACADL | NM_001608.1 | 1094 | cactgcttgcatggcgaaatatt | 223738 | - | see also 16830 line |
| 38207 | ACADL | NM_001608.1 | 1063 | agctgcatgaagcgaaacgtttg | 223737 | - | see also 16830 line |
| 38208 | ACADVL | NM_000018.1 | 276 | aaccggccaaggcggaatctaag | 223751 | - | see also 16831 line |
| 38209 | ACADVL | NM_000018.1 | 277 | accggccaaggcggaatctaagt | 223752 | - | see also 16831 line |
| 38210 | GCDH | NM_000159.1 | 290 | tggccaatcgcaacgaagttttt | 223768 | - | see also 16832 line |
| 38211 | GCDH | NM_000159.1 | 681 | ctcgcctatggccgatctgtttg | 223772 | - | see also 16832 line |
| 38212 | GCDH | NM_000159.1 | 475 | ctcgtcatgcaccctatctatgc | 223771 | - | see also 16832 line |
| 38213 | ACADS | NM_000017.1 | 299 | cggtgctggcctcgattacctgg | 223780 | - | see also 16833 line |
| 38214 | ACADS | NM_000017.1 | 233 | agcggctcaggtgaagaagatgg | 223778 | - | see also 16833 line |
| 38215 | IVD | NM_002225.2 | 443 | cccgtggacgatgcaatcaatgg | 223786 | - | see also 16834 line |
| 38216 | IVD | NM_002225.2 | 1345 | ggcgtgtcggcagtatgtctaca | 223800 | - | see also 16834 line |
| 38217 | ACAD8 | NM_014384.1 | 341 | ggcttcggaggggtctacataca | 223807 | - | see also 5644 line |
| 38218 | ACAD8 | NM_014384.1 | 258 | cccgagagatggctccaaatatg | 223804 | - | see also 5644 line |
| 38219 | ACAD9 | NM_014049.3 | 1135 | ggcttacgtcatggagagtatga | 223842 | - | see also 16836 line |
| 38220 | ACAD9 | NM_014049.3 | 320 | gaggtggactcccgaaaaattga | 223827 | - | see also 16836 line |
| 38221 | ACADM | NM_000016.2 | 1229 | tggtcgtcgaaatacctattatg | 223880 | - | see also 16837 line |
| 38222 | ACADM | NM_000016.2 | 1226 | ttctggtcgtcgaaatacctatt | 223878 | - | see also 16837 line |
| 38223 | ACADSB | NM_001609.2 | 790 | acctgcccgttaacattcgaaaa | 223908 | - | see also 16838 line |
| 38224 | ACADSB | NM_001609.2 | 792 | ctgcccgttaacattcgaaaatg | 223909 | - | see also 16838 line |
| 38225 | SRD5A1 | NM_001047.1 | 570 | atccccgttttctaataggtttt | 223933 | - | see also 16839 line |
| 38226 | SRD5A1 | NM_001047.1 | 410 | tacgggcatcggtgcttaattta | 223928 | - | see also 16839 line |
| 38227 | SRD5A1 | NM_001047.1 | 499 | tacctgtaacggctatttgcaaa | 223930 | - | see also 16839 line |
| 38228 | SRD5A2 | NM_000348.2 | 494 | cacagacatacggtttagcttgg | 223954 | - | see also 290 line |
| 38229 | SRD5A2 | NM_000348.2 | 652 | tcctcggtgagatcattgaatgg | 223960 | - | see also 290 line |

Figure 46

| | | | | | | | | |
|---|---|---|---|---|---|---|---|---|
| 38230 | DHODH | NM_001361.1 | 311 | agccgtggacggactttataaga | 223971 | - | see also 924 line | |
| 38231 | DHODH | NM_001361.1 | 927 | ctccgggatttatcaactcaaac | 223981 | - | see also 924 line | |
| 38232 | DPYD | NM_000110.2 | 869 | ttgcggtaaaagcctttcagtga | 224010 | - | see also 93 line | |
| 38233 | DPYD | NM_000110.2 | 1276 | ccccacggaaggttatagtaaaa | 224024 | - | see also 93 line | |
| 38234 | SDHC | NM_003001.2 | 187 | cccacattactatctacagttgg | 224078 | - | see also 16843 line | |
| 38235 | SDHC | NM_003001.2 | 178 | ctctgtctccccacattactatc | 224075 | - | see also 16843 line | |
| 38236 | SDHC | NM_003001.2 | 182 | gtctccccacattactatctaca | 224076 | - | see also 16843 line | |
| 38237 | ACOX1 | NM_004035.4 | 1321 | gacctatcaccggattaacgaag | 224102 | - | see also 16844 line | |
| 38238 | ACOX1 | NM_004035.4 | 1322 | acctatcaccggattaacgaagg | 224103 | - | see also 16844 line | |
| 38239 | ACOX1 | NM_004035.4 | 881 | gcctttatcgtacctattcgtga | 224095 | - | see also 16844 line | |
| 38240 | ACOX2 | NM_003500.1 | 525 | atccagatcatcgcaacgtatgc | 224137 | - | see also 2359 line | |
| 38241 | ACOX2 | NM_003500.1 | 262 | tccacagttacccggagtttagc | 224133 | - | see also 2359 line | |
| 38242 | ACOX3 | NM_003501.1 | 797 | atggttggcgacataggaaaaaa | 224162 | - | see also 16846 line | |
| 38243 | ACOX3 | NM_003501.1 | 1129 | atggcgcttgcttccatatctgg | 224169 | - | see also 16846 line | |
| 38244 | CPO | NM_000097.3 | 1222 | aagaggacggtatgtagaattta | 224194 | - | see also 16847 line | |
| 38245 | CPO | NM_000097.3 | 1219 | cagaagaggacggtatgtagaat | 224193 | - | see also 16847 line | |
| 38246 | FLJ11164 | NM_018346.1 | 898 | gggcgctcagtacccacaattgg | 224206 | - | see also 16848 line | |
| 38247 | FLJ11164 | NM_018346.1 | 901 | cgctcagtacccacaattggact | 224207 | - | see also 16848 line | |
| 38248 | PPOX | NM_000309.1 | 1528 | ctgcattccccagtatacactag | 224226 | - | see also 16849 line | |
| 38249 | PPOX | NM_000309.1 | 1107 | tggaggctgaccacgttattagt | 224220 | - | see also 16849 line | |
| 38250 | BLVRA | NM_000712.3 | 658 | agcgaaaggaagatcagtatatg | 224234 | - | see also 16850 line | |
| 38251 | BLVRA | NM_000712.3 | 283 | aggtggaggtcgcctatatctgc | 224231 | - | see also 16850 line | |
| 38252 | BLVRB | NM_000713.1 | 254 | cacgtggtagtgggagatgttct | 224248 | - | see also 16851 line | |
| 38253 | DECR1 | NM_001359.1 | 661 | ttcgtgacactagaaattggaaa | 224268 | - | see also 923 line | |
| 38254 | DECR1 | NM_001359.1 | 289 | gcgatgctaccacctaatagttt | 224256 | - | see also 923 line | |
| 38255 | SDHA | NM_004168.1 | 609 | gactggccactcgctattgcaca | 224286 | - | - | succinate dehydrogenase (ubiquinone) | - |
| 38256 | SDHB | NM_003000.1 | 553 | ttcccgatttgagcaacttctat | 224298 | - | see also 16853 line | |
| 38257 | SDHB | NM_003000.1 | 554 | tcccgatttgagcaacttctatg | 224299 | - | see also 16853 line | |
| 38258 | FADS1 | NM_013402.3 | 1245 | cccacgatgcctcgacacaatta | 224309 | - | see also 16854 line | |
| 38259 | FADS1 | NM_013402.3 | 909 | ctctacttccagtggtatatttt | 224305 | - | see also 16854 line | |
| 38260 | SC5DL | NM_006918.2 | 130 | ttccgacaagctattagtcttct | 224318 | - | see also 16855 line | |
| 38261 | SC5DL | NM_006918.2 | 266 | tccgtcgagagattaagtttact | 224326 | - | see also 16855 line | |
| 38262 | TDO2 | NM_005651.1 | 530 | ttgcaattccgactattagaaaa | 224364 | - | see also 3920 line | |
| 38263 | TDO2 | NM_005651.1 | 1116 | tgcgatcaactgtgagtgatagg | 224380 | - | see also 3920 line | |
| 38264 | HPD | NM_002150.2 | 286 | gggcgatcacctggtgaaacacg | 224387 | - | see also 16857 line | |

Figure 46

| | | | | | | | |
|---|---|---|---|---|---|---|---|
| 38265 | HPD | NM_002150.2 | 914 | cctccagtactacaaacaact | 224397 | - | see also 16857 line |
| 38266 | INDO | NM_002164.3 | 686 | aagaacggacactttgctaaag | 224409 | - | see also 16858 line |
| 38267 | INDO | NM_002164.3 | 843 | atcagacggtctgtgtatgaag | 224414 | - | see also 16858 line |
| 38268 | HGD | NM_000187.1 | 889 | gtggttacacggtcattaataaa | 224447 | - | see also 16859 line |
| 38269 | HGD | NM_000187.1 | 319 | gtccacgagcaccaataagaga | 224430 | - | see also 16859 line |
| 38270 | CDO1 | NM_001801.1 | 665 | tggcttacatcgagtagagaaca | 224463 | - | see also 16860 line |
| 38271 | CDO1 | NM_001801.1 | 644 | tgcctacatcaatgattccattg | 224462 | - | see also 16860 line |
| 38272 | HAAO | NM_012205.1 | 266 | gtcattggcaggagagagatatt | 224467 | quinolinate synthetase A | - | - |
| 38273 | BCDO1 | NM_017429.1 | 1431 | gagcttccacggtcaattatgc | 224492 | - | see also 7003 line |
| 38274 | BCDO1 | NM_017429.1 | 1429 | tagagcttcaacggtcaattat | 224491 | - | see also 7003 line |
| 38275 | BCDO2 | NM_031938.1 | 211 | ggctctctacttcgaattggacc | 224512 | - | see also 9058 line |
| 38276 | BCDO2 | NM_031938.1 | 1149 | agggcttgatcaggtccataatt | 224551 | - | see also 9058 line |
| 38277 | FLJ32115 | NM_152321.1 | 335 | ctctttcgcctggtagacaatga | 224568 | - | see also 16863 line |
| 38278 | FLJ32115 | NM_152321.1 | 236 | ctccatagcatgtgcaaaaatt | 224566 | - | see also 16863 line |
| 38279 | TMLHE | NM_018196.1 | 344 | taccgtgatgcgctttgattacg | 224584 | - | see also 7312 line |
| 38280 | TMLHE | NM_018196.1 | 950 | gacactgctagtagatggattct | 224597 | - | see also 7312 line |
| 38281 | ALDRL6 | NM_017584.4 | 285 | gtcggaccggacgtagatttcc | 224605 | - | see also 16865 line |
| 38282 | ALDRL6 | NM_017584.4 | 544 | agcacagagctcgggatgtatca | 224607 | - | see also 16865 line |
| 38283 | ALDRL6 | NM_017584.4 | 546 | cacagagctcgggatgtatcagc | 224608 | - | see also 16865 line |
| 38284 | C12orf8 | NM_006817.2 | 730 | agctccagaagagcttaaacatc | 224613 | protein disulfide isomerase | - | - |
| 38285 | ERP70 | NM_004911.3 | 1921 | agctagagccgtgtacaacagc | 224643 | - | see also 16866 line |
| 38286 | ERP70 | NM_004911.3 | 320 | cccggacgaggattcttctaaca | 224615 | - | see also 16866 line |
| 38287 | ERP70 | NM_004911.3 | 324 | gacgaggattcttctaacagaga | 224617 | - | see also 16866 line |
| 38288 | P5 | NM_005742.2 | 823 | gcctccgatacgggattagagg | 224668 | - | see also 16867 line |
| 38289 | P5 | NM_005742.2 | 220 | atcgaattaactcatcgaattt | 224649 | - | see also 16867 line |
| 38290 | PDIP | NM_006849.1 | 992 | tgcgcttggtcaaccttgaaacc | 224679 | - | see also 16868 line |
| 38291 | PDIP | NM_006849.1 | 1139 | gggatcagcggcagttaagacc | 224681 | - | see also 16868 line |
| 38292 | PDIR | NM_006810.1 | 185 | cagaacccgaataatgtactgg | 224690 | - | see also 4785 line |
| 38293 | PDIR | NM_006810.1 | 569 | gccgctcctgatcatgttttatg | 224702 | - | see also 4785 line |
| 38294 | TXN | NM_003329.1 | 109 | gacgctgcaggtgataaaacttgt | 224718 | - | see also 16870 line |
| 38295 | TXN | NM_003329.1 | 188 | ttcattcctctctgaaaagtat | 224720 | - | see also 16870 line |
| 38296 | TXN2 | NM_012473.3 | 440 | cacagacctcgccattgagtatg | 224725 | - | see also 16871 line |
| 38297 | TXN2 | NM_012473.3 | 411 | gtgatggccaaggtggatattga | 224723 | - | see also 16871 line |
| 38298 | TXNL | NM_004786.1 | 889 | tggcattgttccacttcgttatg | 224747 | - | see also 3300 line |
| 38299 | AMID | NM_032797.4 | 1220 | gacggtgtgggccaaatcagtgg | 224763 | - | see also 9285 line |

Figure 46

| | | | | | | | |
|---|---|---|---|---|---|---|---|
| 38300 | AMID | NM_032797.4 | 869 | gagtatcgagagtacatcaaagt | 224762 | - | see also 9285 line |
| 38301 | FLJ22028 | NM_024854.1 | 483 | atcatagggaacggtggtattgc | 224778 | - | see also 16874 line |
| 38302 | FLJ22028 | NM_024854.1 | 260 | tcccaacattaaggttatagaat | 224770 | - | see also 16874 line |
| 38303 | FLJ23322 | NM_024955.4 | 2131 | ggctcagtccgtcgatagcaaca | 224814 | - | see also 16875 line |
| 38304 | FLJ23322 | NM_024955.4 | 1883 | accgatttgcgaagcttcatgc | 224812 | - | see also 16875 line |
| 38305 | FLJ30473 | NM_144704.1 | 509 | caegccctggccataagtgtcc | 224817 | - | see also 16876 line |
| 38306 | FLJ30473 | NM_144704.1 | 1030 | aacttaagaagtctgttcaagg | 224818 | - | see also 16876 line |
| 38307 | MTO1 | NM_012123.1 | 810 | ageatataccggacaatccatcc | 224838 | - | see also 16877 line |
| 38308 | MTO1 | NM_012123.1 | 1425 | gtgaaccatacgcatgtttacc | 224859 | - | see also 16877 line |
| 38309 | PDCD8 | NM_004208.2 | 1600 | gccgtactggcatcagtcaatgt | 224937 | - | see also 2816 line |
| 38310 | PDCD8 | NM_004208.2 | 1781 | gagtccgaggcctcagaaattac | 224940 | - | see also 2816 line |
| 38311 | SQRDL | NM_021199.1 | 484 | ctcctacgatatcttattattg | 224961 | - | see also 16879 line |
| 38312 | SQRDL | NM_021199.1 | 1223 | aceggctacaaccgtgtgattct | 224988 | - | see also 16879 line |
| 38313 | ALDH1A2 | NM_003888.2 | 1234 | gggccgaaaggggtttttcattg | 225025 | - | see also 16880 line |
| 38314 | ALDH1A2 | NM_003888.2 | 1233 | tgggccgaaagggtttcatt | 225024 | - | see also 16880 line |
| 38315 | ALDH2 | NM_000690.2 | 868 | cteaaatgtctccggrattatgc | 225039 | - | see also 16881 line |
| 38316 | ALDH2 | NM_000690.2 | 1613 | ctgctgaccgtgttacttcatc | 225050 | - | see also 16881 line |
| 38317 | ALDH3A2 | NM_000382.1 | 1276 | atgtattttgcataaccataag | 225081 | - | see also 16882 line |
| 38318 | ALDH3A2 | NM_000382.1 | 916 | ttgcaccgactatattctgt | 225073 | - | see also 16882 line |
| 38319 | ALDH4A1 | NM_003748.2 | 554 | atgaacttccgttcaatgc | 225095 | - | see also 16883 line |
| 38320 | ALDH4A1 | NM_003748.2 | 563 | ttccggttcaatgccaagtatgc | 225096 | - | see also 16883 line |
| 38321 | ALDH7A1 | NM_001182.1 | 963 | cactgcgaggcgactgtttatac | 225108 | - | see also 16884 line |
| 38322 | ALDH7A1 | NM_001182.1 | 966 | tgcgaggcgactgtttatacatg | 225109 | - | see also 16884 line |
| 38323 | ALDH9A1 | NM_000696.2 | 1786 | gccgtgtgacaatcgaatatat | 225193 | - | see also 16885 line |
| 38324 | ALDH9A1 | NM_000696.2 | 1782 | aacggccgtgtgacaatcgaata | 225189 | - | see also 16885 line |
| 38325 | ALDH1A3 | NM_000693.1 | 1289 | ttcgggccagtgcaaccaatact | 225217 | - | see also 16886 line |
| 38326 | ALDH1A3 | NM_000693.1 | 856 | tgcgtcccggacaatctgaagc | 225206 | - | see also 16886 line |
| 38327 | ALDH3A1 | NM_000691.3 | 804 | ctgatccagaaccaaattgtg | 225233 | - | see also 16887 line |
| 38328 | ALDH3A1 | NM_000691.3 | 187 | gccgcagaacctgcacaagaatga | 225226 | - | see also 16887 line |
| 38329 | ALDH3B1 | NM_000694.1 | 454 | tgctgaagccatcggagattagc | 225239 | - | aldehyde dehydrogenase (NAD(P)+) |
| 38330 | ALDH3B1 | NM_000694.1 | 375 | atcattgcgccctggaactatcc | 225238 | - | see also 38329 line |
| 38331 | ALDH3B2 | NM_000695.2 | 1329 | atgagggcttcacctacatatct | 225243 | - | see also 16888 line |
| 38332 | ALDH3B2 | NM_000695.2 | 567 | tggtgctgaagccgtcagaaatc | 225242 | - | see also 16888 line |
| 38333 | GAPDS | NM_014364.3 | 1215 | ttcatggtacgacaagaatatg | 225253 | - | see also 16889 line |
| 38334 | GAPDS | NM_014364.3 | 651 | ctcaccggatgcaccaatgttcg | 225248 | - | see also 16889 line |

Figure 46

| | | | | | | | |
|---|---|---|---|---|---|---|---|
| 38335 | ALDH5A1 | NM_001080.3 | 1359 | caccagttatcaagttcgataca | 225290 | - | see also 16890 line |
| 38336 | ALDH5A1 | NM_001080.3 | 1499 | gggtcaacgaaggattaattc | 225294 | - | see also 16890 line |
| 38337 | ALDH6A1 | NM_005589.2 | 415 | ctccgctatcaacaacttattaa | 225308 | - | see also 3880 line |
| 38338 | ALDH6A1 | NM_005589.2 | 413 | tgctccgctatcaacaacttatt | 225307 | - | see also 3880 line |
| 38339 | PDHA1 | NM_000284.1 | 175 | ctggtagcatccgtaatttgc | 225340 | - | see also 16892 line |
| 38340 | PDHA1 | NM_000284.1 | 1213 | tccagcgacccaccttgaagt | 225352 | - | see also 16892 line |
| 38341 | PDHA1 | NM_000284.1 | 179 | tagcatccgtaatttgcaaat | 225341 | - | see also 16892 line |
| 38342 | PDHA2 | NM_005390.3 | 903 | ctgcaaactaccgttatcatgg | 225376 | - | see also 16893 line |
| 38343 | PDHA2 | NM_005390.3 | 227 | gggcgagggcttaaatactaca | 225360 | - | see also 16893 line |
| 38344 | PDHB | NM_000925.1 | 947 | tggaaggtctgcgttcaattc | 225403 | - | see also 602 line |
| 38345 | PDHB | NM_000925.1 | 811 | gtgataaatatgcgtaccattag | 225401 | - | see also 602 line |
| 38346 | BCKDHA | NM_000709.2 | 911 | gtcaatccgcgtggatggtaatg | 225422 | - | see also 434 line |
| 38347 | BCKDHA | NM_000709.2 | 200 | ctcggcgagtttatagataagt | 225412 | - | see also 434 line |
| 38348 | BCKDHB | NM_000056.2 | 1098 | ttgaacctagaggctcctatatc | 225457 | - | see also 16896 line |
| 38349 | BCKDHB | NM_000056.2 | 372 | ggcttgcgagacaaatatggaaa | 225437 | - | see also 16896 line |
| 38350 | OGDH | NM_002541.1 | 2606 | tccggaagccgttaattactttc | 225493 | - | see also 1637 line |
| 38351 | OGDH | NM_002541.1 | 2766 | ctgcaccggcaaagtgtattatg | 225496 | - | see also 1637 line |
| 38352 | DHTKD1 | NM_018706.4 | 982 | ctcgccaagacgggcgattactct | 225516 | - | see also 16897 line |
| 38353 | DHTKD1 | NM_018706.4 | 1644 | ctcgacctctgcggttgttgg | 225529 | - | see also 16897 line |
| 38354 | DHTKD1 | NM_018706.4 | 984 | cgccaagacggcgattactctcc | 225517 | - | see also 16897 line |
| 38355 | FLJ10851 | NM_018245.1 | 2756 | cacgggaaaggtgtactatgacc | 225580 | - | see also 16898 line |
| 38356 | FLJ10851 | NM_018245.1 | 609 | ctgccgacaaccaccttcattgg | 225554 | - | see also 16898 line |
| 38357 | COX11 | NM_004375.2 | 68 | tggatggaggtgcgttccttct | 225584 | - | see also 16899 line |
| 38358 | COX15 | NM_004376.3 | 1172 | ctgctggctttggcgtattacaca | 225599 | - | see also 16900 line |
| 38359 | COX15 | NM_004376.3 | 629 | ggcatgaaaggacgtgtcttgc | 225591 | - | see also 16900 line |
| 38360 | COX4I1 | NM_001861.2 | 547 | ccctcccgcaaagctttgacaaa | 225604 | - | cbb3-type cytochrome c oxidase — |
| 38361 | COX4I1 | NM_001861.2 | 635 | ctccaagtgggactacgaaaaga | 225605 | - | see also 38360 line |
| 38362 | COX4I1 | NM_001861.2 | 244 | tgtgaagagcgaagactttcg | 225603 | - | see also 38360 line |
| 38363 | COX4I2 | NM_032609.2 | 438 | gttggcagccggtctacgtatttc | 225607 | - | see also 16901 line |
| 38364 | COX4I2 | NM_032609.2 | 439 | tggcagcgggtctacgtatttcc | 225608 | - | see also 16901 line |
| 38365 | COX5A | NM_004255.2 | 178 | ctgctcggtaacatacttcaac | 225614 | - | see also 16902 line |
| 38366 | COX5A | NM_004255.2 | 175 | atgctcgctcggtaacatacttc | 225612 | - | see also 16902 line |
| 38367 | COX5B | NM_001862.2 | 370 | cccgctgttggcgccattacaag | 225634 | - | see also 16903 line |
| 38368 | COX5B | NM_001862.2 | 276 | atctccaacaagagaatagttagg | 225633 | - | see also 16903 line |
| 38369 | COX5B | NM_001862.2 | 245 | caccagggaagaccctaatttag | 225631 | - | see also 16903 line |
| 38370 | COX6B | NM_001863.3 | 286 | aaggccaatgaccgctaaaggagg | 225635 | - | see also 16904 line |

Figure 46

| 38371 | COX6C | NM_004374.2 | 106 | gccaggcgtctgcgaaatcatat | 225637 | - | see also 16905 line | | |
|---|---|---|---|---|---|---|---|---|---|
| 38372 | COX6C | NM_004374.2 | 172 | aagtttcgtgtggctgatcaaag | 225639 | - | see also 16905 line | | |
| 38373 | COX7A1 | NM_001864.2 | 533 | accgagtgcgcgagaaacagaag | 225644 | - | - | electron transporter | - |
| 38374 | COX7A2L | NM_004718.2 | 194 | tccgattccacagtgtatgatta | 225649 | - | see also 16906 line | | |
| 38375 | COX7A2L | NM_004718.2 | 193 | ctccgattccacagtgtatgatt | 225648 | - | see also 16906 line | | |
| 38376 | COX7A3 | NM_183003.1 | 264 | agcaatcacttggttcacattca | 225656 | - | see also 16907 line | | |
| 38377 | COX7B | NM_001866.2 | 103 | gtcaaaagcgcactaaatcgtct | 225658 | - | see also 16908 line | | |
| 38378 | COX7B | NM_001866.2 | 100 | ttggtcaaaagcgcactaaatcg | 225657 | - | see also 16908 line | | |
| 38379 | COX7B | NM_001866.2 | 258 | agcaacacaagtcggaatagaat | 225661 | - | see also 16908 line | | |
| 38380 | COX7C | NM_001867.2 | 236 | tgctacacccttccttgtagtaa | 225667 | - | see also 16909 line | | |
| 38381 | COX7C | NM_001867.2 | 216 | ttgtactttggatctgcatttgc | 225665 | - | see also 16909 line | | |
| 38382 | CYB5 | NM_001914.1 | 89 | tgcaccacaaggtgtacgatttg | 225670 | - | see also 16910 line | | |
| 38383 | CYB5 | NM_001914.1 | 92 | accacaaggtgtacgatttgacc | 225671 | - | see also 16910 line | | |
| 38384 | SURF1 | NM_003172.2 | 750 | ggcgcagagcccatcttcattga | 225682 | - | see also 16911 line | | |
| 38385 | SURF1 | NM_003172.2 | 749 | aggcgcagagcccatcttcattg | 225681 | - | see also 16911 line | | |
| 38386 | FLJ13852 | NM_023078.1 | 599 | ggctgaaggagccgtcaagatgg | 225687 | - | see also 16912 line | | |
| 38387 | FLJ13852 | NM_023078.1 | 159 | gacaggaacctatgtcactttca | 225685 | - | see also 16912 line | | |
| 38388 | LOC122945 | NM_138791.1 | 435 | gccgtctcagctgcctaatatct | 225701 | - | see also 16913 line | | |
| 38389 | LOC122945 | NM_138791.1 | 683 | gtcacggctcaacatatttctgg | 225709 | - | see also 16913 line | | |
| 38390 | P5CR2 | NM_013328.2 | 967 | tggccgaccaagaaaagatctcc | 225716 | - | see also 5400 line | | |
| 38391 | P5CR2 | NM_013328.2 | 225 | atcctgtcggctcacaagataat | 225711 | - | see also 5400 line | | |
| 38392 | QDPR | NM_000320.1 | 224 | cagcgctacgatcattgttaaaa | 225722 | - | see also 267 line | | |
| 38393 | QDPR | NM_000320.1 | 225 | agcgctacgatcattgttaaaat | 225723 | - | see also 267 line | | |
| 38394 | QDPR | NM_000320.1 | 221 | ggccagcgctacgatcattgtta | 225720 | - | see also 267 line | | |
| 38395 | PRODH | NM_016335.2 | 1755 | tgctgaccaccgggtgtactttg | 225743 | - | see also 16916 line | | |
| 38396 | PRODH | NM_016335.2 | 526 | ctcatgaagatgaccttctatgg | 225741 | - | see also 16916 line | | |
| 38397 | PRODH2 | NM_021232.1 | 79 | aacgtgagaaccgtattttcaaa | 225745 | - | see also 16917 line | | |
| 38398 | PRODH2 | NM_021232.1 | 907 | ctgcatcgggtggcacagtatgc | 225751 | - | see also 16917 line | | |
| 38399 | ETFDH | NM_004453.1 | 1647 | acccgatggacagatcagttttg | 225793 | - | see also 2985 line | | |
| 38400 | ETFDH | NM_004453.1 | 239 | ctcgaattactacccattatact | 225755 | - | see also 2985 line | | |
| 38401 | PIPOX | NM_016518.1 | 708 | tgcggatcaacgtgtgttactgg | 225821 | - | see also 6899 line | | |
| 38402 | PIPOX | NM_016518.1 | 1034 | ctcgatcgccacccaaagtatga | 225822 | - | see also 6899 line | | |
| 38403 | MAOA | NM_000240.2 | 688 | tgctaggcggtttgcttatcttt | 225834 | - | see also 16920 line | | |
| 38404 | MAOA | NM_000240.2 | 1620 | acgttccagcggtagaaatcacc | 225853 | - | see also 16920 line | | |
| 38405 | MAOB | NM_000898.3 | 1107 | ttgcctacacgttggatgatacc | 225882 | - | see also 583 line | | |

Figure 46

| 38406 | SMOX | NM_019025.2 | 1780 | gccacccaccgcaagtactattc | 225916 | - | see also 7665 line | | | |
|---|---|---|---|---|---|---|---|---|---|---|
| 38407 | PNPO | NM_018129.1 | 638 | tccctgatcgggagtatctgaga | 225923 | - | see also 7270 line | | | |
| 38408 | PNPO | NM_018129.1 | 779 | gcctgcatgaccggatagtcttt | 225924 | - | see also 7270 line | | | |
| 38409 | PNPO | NM_018129.1 | 439 | aacttcgagagtcgaaaaggaaa | 225921 | - | see also 7270 line | | | |
| 38410 | DAO | NM_001917.3 | 1030 | ttcgcactggaccttcaaacaca | 225943 | - | see also 1221 line | | | |
| 38411 | DAO | NM_001917.3 | 579 | cagtggctgactgaaaggttaac | 225930 | - | see also 1221 line | | | |
| 38412 | GLDC | NM_000170.1 | 398 | ggctggcgagcattgatgaattg | 225946 | - | see also 142 line | | | |
| 38413 | MEGF11 | NM_032445.1 | 171 | ttcgatcagatctattacacacg | 226003 | - | see also 16925 line | | | |
| 38414 | MEGF11 | NM_032445.1 | 167 | ccccttcgatcagatctattaca | 226002 | - | see also 16925 line | | | |
| 38415 | MEGF11 | NM_032445.1 | 166 | accccttcgatcagatctattac | 226001 | - | see also 16925 line | | | |
| 38416 | QP-C | NM_014402.2 | 178 | cgcgtggtgccgcagtttgtagt | 226024 | - | see also 16926 line | | | |
| 38417 | QP-C | NM_014402.2 | 41 | gccgcgagtttgggaatctgacg | 226018 | - | see also 16926 line | | | |
| 38418 | QP-C | NM_014402.2 | 155 | gccgcattcgggagtctttcttt | 226021 | - | see also 16926 line | | | |
| 38419 | UQCR | NM_006830.2 | 148 | tggactgggtaccttacatcaat | 226030 | - | see also 16927 line | | | |
| 38420 | UQCR | NM_006830.2 | 150 | gactgggtaccttacatcaatgg | 226031 | - | see also 16927 line | | | |
| 38421 | UQCRB | NM_006294.2 | 321 | taccttgaaccgtatctgaaaga | 226033 | - | - | ubiquinol-cytochrome c reductase | - | |
| 38422 | UQCRFS1 | NM_006003.1 | 536 | tggcgaaaatcgaaatcaagtta | 226035 | - | see also 16928 line | | | |
| 38423 | UQCRFS1 | NM_006003.1 | 533 | ccctggcgaaaatcgaaatcaag | 226034 | - | see also 16928 line | | | |
| 38424 | AKR1B10 | NM_020299.3 | 400 | tgcctatgtctatcagaatgaac | 226042 | - | see also 16929 line | | | |
| 38425 | PCYOX1 | NM_016297.2 | 942 | tggccactccgttgaatcgaaaa | 226087 | - | see also 16930 line | | | |
| 38426 | PCYOX1 | NM_016297.2 | 944 | gccactccgttgaatcgaaaaat | 226089 | - | see also 16930 line | | | |
| 38427 | PCYOX1 | NM_016297.2 | 943 | ggccactccgttgaatcgaaaaa | 226088 | - | see also 16930 line | | | |
| 38428 | MSRA | NM_012331.2 | 350 | agcggccaaacatcatgtcaatg | 226109 | - | see also 16931 line | | | |
| 38429 | MSRA | NM_012331.2 | 893 | gtcctgcccagtgggtattaaaa | 226124 | - | see also 16931 line | | | |
| 38430 | RRM1 | NM_001033.2 | 1719 | aacgccatcgccccattggaatt | 226204 | - | see also 631 line | | | |
| 38431 | RRM1 | NM_001033.2 | 679 | gacgctagagcggtcttatttgt | 226151 | - | see also 631 line | | | |
| 38432 | RRM2 | NM_001034.1 | 555 | cccgaggagagatattttatatc | 226232 | - | see also 632 line | | | |
| 38433 | RRM2B | NM_015713.2 | 272 | gaggtcgacttatcaaaggatct | 226241 | - | see also 16934 line | | | |
| 38434 | RRM2B | NM_015713.2 | 588 | tgcgatggatagcagatagaaaa | 226247 | - | see also 16934 line | | | |
| 38435 | MDGA1 | NM_153487.2 | 1407 | aggtagcgcctcggacaagttcc | 226255 | - | see also 16935 line | | | |
| 38436 | MDGA1 | NM_153487.2 | 3730 | ctggtgcggtcccggaacaaagg | 226274 | - | see also 16935 line | | | |
| 38437 | FDXR | NM_004110.2 | 1390 | aggggtccggccagtctctttct | 226283 | - | see also 16936 line | | | |
| 38438 | FDXR | NM_004110.2 | 286 | ccccgaggtgaagaatgtcatca | 226279 | - | see also 16936 line | | | |
| 38439 | SC4MOL | NM_006745.2 | 885 | tacatggtgggatcgaattttg | 226308 | - | see also 16937 line | | | |
| 38440 | SC4MOL | NM_006745.2 | 243 | atggggatcccttatagttcatg | 226291 | - | see also 16937 line | | | |

Figure 46

| 38441 | AKR1D1 | NM_005989.1 | 91 | caccgcatacctctaagtgatgg | 226311 | - | see also 4183 line |
|---|---|---|---|---|---|---|---|
| 38442 | AKR1D1 | NM_005989.1 | 183 | aacatcggtgaaggttgctattg | 226312 | - | see also 4183 line |
| 38443 | ALDH1B1 | NM_000692.3 | 539 | atcaaggtgtatcggtactttgc | 226339 | - | see also 16939 line |
| 38444 | ALDH1B1 | NM_000692.3 | 1080 | cccggaccttcgtggaagaatcc | 226345 | - | see also 16939 line |
| 38445 | ALDH8A1 | NM_022568.2 | 1279 | ttgaaagagccaacaacgttaag | 226374 | - | see also 8421 line |
| 38446 | ALDH8A1 | NM_022568.2 | 1211 | ctgcatgacggaagagatatttg | 226371 | - | see also 8421 line |
| 38447 | AMACR | NM_014324.3 | 970 | gactccggttctgactttgagg | 226398 | - | see also 16941 line |
| 38448 | AMACR | NM_014324.3 | 563 | atgtgtgcactgggcattataat | 226387 | - | see also 16941 line |
| 38449 | C10orf33 | NM_032709.1 | 687 | gagcccagcttccccgatattat | 226406 | - | see also 16942 line |
| 38450 | C10orf33 | NM_032709.1 | 1162 | gtcgcctgtcaccaagatcaatg | 226410 | - | see also 16942 line |
| 38451 | C21orf59 | NM_017835.1 | 615 | cagcggaggtgcagtgtttaaaa | 226418 | - | - | - | - |
| 38452 | C21orf59 | NM_017835.1 | 616 | agcggaggtgcagtgtttaaaaa | 226419 | - | see also 38451 line |
| 38453 | C21orf59 | NM_017835.1 | 613 | cccagcggaggtgcagtgtttaa | 226417 | - | see also 38451 line |
| 38454 | CED-6 | NM_016315.1 | 1147 | ctccagtacctagtagatctact | 226445 | - | see also 6804 line |
| 38455 | CED-6 | NM_016315.1 | 921 | cagatcgcagggttacaaaaaag | 226440 | - | see also 6804 line |
| 38456 | CED-6 | NM_016315.1 | 1144 | cacctccagtacctagtagatct | 226444 | - | see also 6804 line |
| 38457 | CHDH | NM_018397.1 | 1595 | gtgaagctcaccagagaaatttt | 226459 | - | see also 7439 line |
| 38458 | CHDH | NM_018397.1 | 1012 | tgcagtgggcgtggagtatgtca | 226451 | - | see also 7439 line |
| 38459 | CRYL1 | NM_015974.1 | 545 | ctgtgaatccgccatactacatc | 226473 | - | see also 16945 line |
| 38460 | CRYL1 | NM_015974.1 | 838 | ctcaatgcagaaggtatgttaag | 226477 | - | see also 16945 line |
| 38461 | CYB5R1 | NM_016243.1 | 474 | agcgggttgctcacttacactgg | 226483 | - | see also 16946 line |
| 38462 | CYB5R1 | NM_016243.1 | 470 | gccaagcgggttgctcacttaca | 226482 | - | see also 16946 line |
| 38463 | CYB5R1 | NM_016243.1 | 276 | tacctctccacccgaattgatgg | 226480 | - | see also 16946 line |
| 38464 | CYBB | NM_000397.2 | 870 | gtgcggttttggcgatctcaaca | 226519 | - | see also 319 line |
| 38465 | CYBB | NM_000397.2 | 398 | tgcccgagtcaataattctgatc | 226497 | - | see also 319 line |
| 38466 | DCXR | NM_016286.1 | 422 | agcgggcagtaactaaccatagc | 226539 | - | see also 16948 line |
| 38467 | DCXR | NM_016286.1 | 417 | ctcccagcgggcagtaactaacc | 226538 | - | see also 16948 line |
| 38468 | DECR2 | NM_020664.3 | 425 | atcgacattctcattaactgtgc | 226542 | - | see also 16949 line |
| 38469 | DECR2 | NM_020664.3 | 687 | tggagtggggtccccaaaacatc | 226546 | - | see also 16949 line |
| 38470 | DECR2 | NM_020664.3 | 517 | taccagcggcaccttcaatgtgt | 226543 | - | see also 16949 line |
| 38471 | DHCR24 | NM_014762.1 | 401 | cactacgtgtcgggaagtacaag | 226551 | - | see also 5949 line |
| 38472 | DHCR24 | NM_014762.1 | 404 | tacgtgtcgggaagtacaagaag | 226552 | - | see also 5949 line |
| 38473 | DHCR24 | NM_014762.1 | 1425 | gccgcgtgtgaaacactttgaag | 226566 | - | see also 5949 line |
| 38474 | DHCR7 | NM_001360.1 | 308 | ttcactggcgagcgtcatcttcc | 226569 | - | see also 16951 line |
| 38475 | DHCR7 | NM_001360.1 | 1025 | ggccatctacgtgattgacttct | 226572 | - | see also 16951 line |
| 38476 | DHRS1 | NM_138452.1 | 356 | agcaggcgccacagtttacatca | 226574 | - | see also 16952 line |
| 38477 | DHRS1 | NM_138452.1 | 1078 | gccccgtccaagactatttgtct | 226587 | - | see also 16952 line |

Figure 46

| 38478 | DHRS1 | NM_138452.1 | 1165 | tccgtgtgcccaagtggattatt | 226591 | - | see also 16952 line |
|---|---|---|---|---|---|---|---|
| 38479 | DHRS10 | NM_016246.1 | 639 | gtgtccgagtcaactgtatctcc | 226598 | - | see also 16953 line |
| 38480 | DHRS10 | NM_016246.1 | 578 | gccaccaaggggggcagtaacagc | 226593 | - | see also 16953 line |
| 38481 | DHRS7 | NM_016029.1 | 570 | gggacggtgtccttgacaaaatg | 226609 | - | see also 16954 line |
| 38482 | DHRS7 | NM_016029.1 | 568 | tagggacggtgtccttgacaaaa | 226607 | - | see also 16954 line |
| 38483 | DHRS8 | NM_016245.1 | 832 | gtctgtgtcctaatttcgtaaac | 226638 | - | see also 16955 line |
| 38484 | DHRS8 | NM_016245.1 | 659 | tgcaatgacgaagaataaccatg | 226635 | - | see also 16955 line |
| 38485 | DHRS8 | NM_016245.1 | 655 | ttcctgcaatgacgaagaataac | 226634 | - | see also 16955 line |
| 38486 | DHRSX | NM_145177.1 | 431 | gagatggattcgaagaacatttc | 226673 | - | see also 16957 line |
| 38487 | DHRSX | NM_145177.1 | 794 | tcggctggttgcttttcaagacc | 226675 | - | see also 16957 line |
| 38488 | DKFZp566 O084 | NM_015510.3 | 775 | accgcggatggatctaggtatgg | 226684 | - | see also 16958 line |
| 38489 | DKFZp566 O084 | NM_015510.3 | 619 | aagatgagcattccttttcgatc | 226680 | - | see also 16958 line |
| 38490 | DKFZp566 O084 | NM_015510.3 | 694 | gccgagatggaacagtatgaaat | 226682 | - | see also 16958 line |
| 38491 | ERO1LB | NM_019891.2 | 508 | gagtaaaattccggttggaataa | 226691 | - | see also 7747 line |
| 38492 | ERO1LB | NM_019891.2 | 679 | ttcacgggatcacttttgtgaac | 226701 | - | see also 7747 line |
| 38493 | ERO1LB | NM_019891.2 | 724 | tgctcagtatgtagacctattgc | 226703 | - | see also 7747 line |
| 38494 | FADS2 | NM_004265.2 | 1349 | ccccgctggtgaagtctctatgt | 226742 | - | see also 2865 line |
| 38495 | FADS2 | NM_004265.2 | 1462 | ctggacgcctaccttcacaaatg | 226744 | - | see also 2865 line |
| 38496 | FADS3 | NM_021727.3 | 412 | ttcctacagcccctgttgattgg | 226746 | - | see also 16961 line |
| 38497 | FADS3 | NM_021727.3 | 1064 | atgcccgcttcttcttatcctac | 226748 | - | see also 16961 line |
| 38498 | FLJ11042 | NM_018308.1 | 964 | cacactcaccgagttaccttaga | 226763 | - | see also 16962 line |
| 38499 | FLJ11042 | NM_018308.1 | 865 | ttggtggccagaatttattataa | 226762 | - | see also 16962 line |
| 38500 | FLJ11577 | NM_025159.1 | 817 | atccagcggtactcatcctattg | 226777 | - | see also 16963 line |
| 38501 | FLJ11577 | NM_025159.1 | 1094 | gccgcctattgcaattgatgtca | 226787 | - | see also 16963 line |
| 38502 | FLJ12661 | NM_025138.2 | 2715 | gggacattgggccgtgcatatac | 226830 | - | see also 16964 line |
| 38503 | FLJ12661 | NM_025138.2 | 1259 | acctagccgcataaatggaattt | 226793 | - | see also 16964 line |
| 38504 | FLJ20643 | NM_017916.1 | 712 | atccggaatggcgcatgatgaag | 226858 | - | see also 16965 line |
| 38505 | FLJ20643 | NM_017916.1 | 713 | tccggaatggcgcatgatgaaga | 226859 | - | see also 16965 line |
| 38506 | FLJ20643 | NM_017916.1 | 416 | ctcggaagggaaggttttcatca | 226855 | - | see also 16965 line |
| 38507 | FVT1 | NM_002035.1 | 251 | tgctatcgagtgctataaacaag | 226865 | - | see also 16966 line |
| 38508 | FVT1 | NM_002035.1 | 800 | gcctttggagactcgacttattt | 226877 | - | see also 16966 line |
| 38509 | FZD7 | NM_003507.1 | 347 | cccgaactccgcttttttcttatg | 226890 | - | see also 16967 line |
| 38510 | FZD7 | NM_003507.1 | 343 | ttctcccgaactccgcttttct | 226887 | - | see also 16967 line |
| 38511 | FZD7 | NM_003507.1 | 345 | ctcccgaactccgcttttctta | 226888 | - | see also 16967 line |

Figure 46

| 38512 | GFOD1 | NM_018988.1 | 1730 | gacgggcgagtggcagaacattg | 226894 | - | - | - | - |
|---|---|---|---|---|---|---|---|---|---|
| 38513 | GFOD1 | NM_018988.1 | 813 | ttctacactagccgcattgatga | 226893 | - | see also 38512 line | | |
| 38514 | GMIP | NM_016573.1 | 623 | gccgccaaacggactgagattga | 226900 | - | see also 16968 line | | |
| 38515 | GMIP | NM_016573.1 | 625 | cgccaaacggactgagattgaga | 226901 | - | see also 16968 line | | |
| 38516 | H17 | NM_017547.2 | 1316 | cccccacccgctagttgtcaaca | 226929 | - | see also 7028 line | | |
| 38517 | H17 | NM_017547.2 | 1321 | acccgctagttgtcaacatgtac | 226930 | - | see also 7028 line | | |
| 38518 | H17 | NM_017547.2 | 1322 | cccgctagttgtcaacatgtact | 226931 | - | see also 7028 line | | |
| 38519 | HSD17B12 | NM_016142.1 | 898 | tgctcttatgggctcgataatct | 226958 | - | see also 16970 line | | |
| 38520 | HSD17B12 | NM_016142.1 | 900 | ctcttatgggctcgataatctca | 226959 | - | see also 16970 line | | |
| 38521 | HSD17B12 | NM_016142.1 | 968 | aagtctacacgggctcactatct | 226961 | - | see also 16970 line | | |
| 38522 | KIAA0769 | NM_014824.1 | 995 | tagccgacagttagaatcagaaa | 226999 | - | see also 6026 line | | |
| 38523 | KIAA0769 | NM_014824.1 | 1238 | agcccggttggacctgctaaagc | 227010 | - | see also 6026 line | | |
| 38524 | KREMEN2 | NM_024507.2 | 977 | agcgcctcagggcgtcatctact | 227038 | - | see also 16972 line | | |
| 38525 | LISCH7 | NM_015925.3 | 1687 | agcaggtctcccacgagtaatgg | 227040 | - | see also 6573 line | | |
| 38526 | LOC283985 | NM_178128.2 | 1066 | accgtacaacgaggactcatacc | 227051 | - | see also 16974 line | | |
| 38527 | LOC283985 | NM_178128.2 | 1110 | tccgtcgctatgaggaattcatg | 227052 | - | see also 16974 line | | |
| 38528 | LOC283985 | NM_178128.2 | 880 | gccccgtcggattcacatgatga | 227049 | - | see also 16974 line | | |
| 38529 | MAWBP | NM_022129.1 | 553 | tccgcctcagtgacgtttacaac | 227058 | - | see also 16975 line | | |
| 38530 | MAWBP | NM_022129.1 | 870 | gacggaagggttgacattagagg | 227064 | - | see also 16975 line | | |
| 38531 | MGC10204 | NM_153329.2 | 1507 | cccagacgggctgtatgagtatc | 227072 | - | see also 16976 line | | |
| 38532 | MGC10204 | NM_153329.2 | 191 | tgcttgggccactatgtgaatgg | 227067 | - | see also 16976 line | | |
| 38533 | MGC11335 | NM_030819.2 | 492 | tccgtgaaggctctaggtattgg | 227078 | - | see also 16977 line | | |
| 38534 | MGC11335 | NM_030819.2 | 491 | atccgtgaaggctctaggtattg | 227077 | - | see also 16977 line | | |
| 38535 | MGC15763 | NM_138381.1 | 601 | tccttcgacgggagattgtgtca | 227087 | - | see also 16978 line | | |
| 38536 | MGC15763 | NM_138381.1 | 1085 | taccagcgaactcctgtttaaga | 227101 | - | see also 16978 line | | |
| 38537 | MGC23280 | NM_144683.2 | 636 | ctgtcggggacgtcttgacttca | 227113 | - | see also 16979 line | | |
| 38538 | MGC23280 | NM_144683.2 | 1207 | caccgaattcaggctaaagttga | 227125 | - | see also 16979 line | | |
| 38539 | MGC4172 | NM_024308.2 | 706 | gacacaattcgccttcaaactcc | 227139 | - | see also 16980 line | | |

Figure 46

| 38540 | MGC4172 | NM_024308.2 | 522 | tggacgatgggcacatcattaac | 227132 | - | see also 16980 line |
|---|---|---|---|---|---|---|---|
| 38541 | MRPS30 | NM_016640.2 | 70 | tacgcggtccgaggctttcattg | 227140 | - | see also 6964 line |
| 38542 | MRPS30 | NM_016640.2 | 768 | tccaagcaactcgcagagtttgt | 227150 | - | see also 6964 line |
| 38543 | NOX3 | NM_015718.1 | 221 | ttccattacacacgagttatttt | 227172 | - | see also 6541 line |
| 38544 | NOX3 | NM_015718.1 | 891 | aggaaccctcggcttggaaatgg | 227185 | - | see also 6541 line |
| 38545 | NR1 | NM_014434.1 | 724 | aggacgttcggctgattgagttt | 227207 | - | see also 16983 line |
| 38546 | NR1 | NM_014434.1 | 726 | gacgttcggctgattgagtttga | 227208 | - | see also 16983 line |
| 38547 | PECR | NM_018441.2 | 280 | cacgagtcattcccatacaatgc | 227221 | - | see also 16984 line |
| 38548 | PECR | NM_018441.2 | 209 | atcccgtaagttggagagattga | 227217 | - | see also 16984 line |
| 38549 | PHYH | NM_006214.2 | 874 | agggattccggaaggcaatttcc | 227275 | - | see also 16985 line |
| 38550 | PHYH | NM_006214.2 | 849 | cacggatctggtcagaataaaac | 227274 | - | see also 16985 line |
| 38551 | RDH-E2 | NM_138969.2 | 958 | gagtagccgaccaggttaaaaaa | 227288 | - | see also 16986 line |
| 38552 | RDH-E2 | NM_138969.2 | 1005 | atcaacaatgccggaatcgtaac | 227294 | - | see also 16986 line |
| 38553 | RDH10 | NM_172037.1 | 871 | ctgccggagttgaggattactgt | 227311 | - | see also 10033 line |
| 38554 | RDH11 | NM_016026.2 | 710 | ggcgttacgacgtattctgtaca | 227337 | - | see also 6626 line |
| 38555 | RDH11 | NM_016026.2 | 753 | aactggttcggcactcatctttc | 227338 | - | see also 6626 line |
| 38556 | RDH12 | NM_152443.1 | 665 | tgccctgcacgggtggttaatg | 227349 | - | see also 16989 line |
| 38557 | RDH12 | NM_152443.1 | 666 | gccctgcacgggtggttaatgt | 227350 | - | see also 16989 line |
| 38558 | RDH12 | NM_152443.1 | 352 | gccgaggagcccgagtctatatt | 227345 | - | see also 16989 line |
| 38559 | RDH13 | NM_138412.2 | 608 | agcgagtggacattctaatcaac | 227355 | - | see also 16990 line |
| 38560 | RDH13 | NM_138412.2 | 753 | agcccttcgcggatcatcaacc | 227359 | - | see also 16990 line |
| 38561 | RDH14 | NM_020905.1 | 1051 | gtgatggttggcctgctaaaata | 227381 | - | see also 16991 line |
| 38562 | RDH14 | NM_020905.1 | 998 | tgcccaaagctatggatgaatct | 227379 | - | see also 16991 line |
| 38563 | RODH | NM_003725.2 | 934 | tgcatccgcgaactcgatattca | 227397 | - | see also 16992 line |
| 38564 | RODH | NM_003725.2 | 678 | ctgaggcgtgagattcaacattt | 227387 | - | see also 16992 line |
| 38565 | SCDR9 | NM_178135.2 | 870 | acgcgcctcagcgattttaaatc | 227433 | - | see also 16993 line |
| 38566 | SCDR9 | NM_178135.2 | 869 | aacgcgcctcagcgattttaaat | 227432 | - | see also 16993 line |
| 38567 | SDR-O | NM_148897.1 | 559 | aggcgtgagctctactactttgg | 227437 | - | see also 16994 line |
| 38568 | SDR-O | NM_148897.1 | 558 | aaggcgtgagctctactactttg | 227436 | - | see also 16994 line |
| 38569 | SELO | NM_031454.1 | 1405 | gaccggtgccgacttcacaaaca | 227456 | - | see also 9033 line |
| 38570 | SELO | NM_031454.1 | 734 | gtggtgcgcgacgtgttctatga | 227446 | - | see also 9033 line |
| 38571 | SEPW1 | NM_003009.1 | 216 | tagccgggaagttgattcactct | 227459 | - | see also 16996 line |
| 38572 | SEPW1 | NM_003009.1 | 190 | ggccaccgggttctttgaagtga | 227457 | - | see also 16996 line |
| 38573 | SHREW1 | NM_018836.2 | 1503 | ctcgacgacgggggagtacaaat | 227466 | - | see also 16997 line |
| 38574 | SHREW1 | NM_018836.2 | 1062 | ccccatgacgctgcagactaagg | 227465 | - | see also 16997 line |
| 38575 | SHREW1 | NM_018836.2 | 1551 | ctcttctgatcggcatcttattc | 227469 | - | see also 16997 line |

Figure 46

| | | | | | | | |
|---|---|---|---|---|---|---|---|
| 38576 | SLC35C2 | NM_015945.10 | 636 | caccgtctcgctgtacacaatga | 227472 | - | see also 16998 line |
| 38577 | SLC35C2 | NM_015945.10 | 1126 | ctctccattgccggcatttttaa | 227476 | - | see also 16998 line |
| 38578 | SLC35C2 | NM_015945.10 | 1223 | ccctctgcctctcgggaatatcc | 227478 | - | see also 16998 line |
| 38579 | TM7SF2 | NM_003273.1 | 937 | gtcgcctgcgctatcctattaac | 227483 | - | see also 16999 line |
| 38580 | TM7SF2 | NM_003273.1 | 935 | gagtcgcctgcgctatcctatta | 227482 | - | see also 16999 line |
| 38581 | TSTA3 | NM_003313.2 | 409 | gacgacctacccgatagatgaga | 227494 | - | see also 17000 line |
| 38582 | TSTA3 | NM_003313.2 | 61 | gggatccatgcggattctagtga | 227489 | - | see also 17000 line |
| 38583 | ANAPC2 | NM_013366.2 | 2304 | ctcactggatcgtatctacaaca | 227510 | - | see also 17001 line |
| 38584 | ANAPC2 | NM_013366.2 | 2306 | cactggatcgtatctacaacatg | 227511 | - | see also 17001 line |
| 38585 | ANAPC4 | NM_013367.1 | 85 | tcgcccaagcgggatctcattgc | 227514 | - | see also 5410 line |
| 38586 | ANAPC4 | NM_013367.1 | 84 | gtcgcccaagcgggatctcattg | 227513 | - | see also 5410 line |
| 38587 | ANAPC5 | NM_016237.3 | 992 | ctgccgcttcggtcactatcaac | 227600 | - | see also 6739 line |
| 38588 | APC10 | NM_014885.1 | 607 | gacacccatatgagacaaattaa | 227635 | - | see also 17004 line |
| 38589 | APC10 | NM_014885.1 | 504 | accaagtggctggattcatgttc | 227632 | - | see also 17004 line |
| 38590 | APC10 | NM_014885.1 | 525 | tcccttaactgacaatcataaga | 227633 | - | see also 17004 line |
| 38591 | C14orf130 | NM_018108.2 | 1091 | caggagcgatcccctaatggata | 227656 | - | see also 7256 line |
| 38592 | C14orf130 | NM_018108.2 | 662 | tggattggtgcggaacattgatg | 227640 | - | see also 7256 line |
| 38593 | C6orf133 | NM_015255.1 | 986 | atggccttcgccggattttatgt | 227696 | - | see also 17006 line |
| 38594 | C6orf133 | NM_015255.1 | 2372 | tggctcatagtgaattggtaaag | 227759 | - | see also 17006 line |
| 38595 | CDC34 | NM_004359.1 | 392 | cccatcgactacccatactctcc | 227835 | - | see also 17007 line |
| 38596 | CDC34 | NM_004359.1 | 300 | acgagggcgatctatacaactgg | 227834 | - | see also 17007 line |
| 38597 | CEB1 | NM_016323.1 | 366 | gcccggacgccgaaatgcattaa | 227836 | - | see also 17008 line |
| 38598 | CEB1 | NM_016323.1 | 367 | cccggacgccgaaatgcattaaa | 227837 | - | see also 17008 line |
| 38599 | DD5 | NM_015902.3 | 7479 | ctctagtcgaagtgtagtagata | 228114 | - | see also 17009 line |
| 38600 | DD5 | NM_015902.3 | 962 | aacgtgaatccgttttacgttta | 227935 | - | see also 17009 line |
| 38601 | DD5 | NM_015902.3 | 5726 | gacgacgtgccaccttgcttagc | 228076 | - | see also 17009 line |
| 38602 | DKFZP564G092 | NM_015601.2 | 2497 | atcctaaatacggcatgtttagg | 228217 | - | see also 6502 line |
| 38603 | DKFZP564G092 | NM_015601.2 | 506 | tacgttagcgctaaatgacaaag | 228155 | - | see also 6502 line |
| 38604 | DKFZP564G092 | NM_015601.2 | 3085 | gggcagaacatcctacgataaaa | 228240 | - | see also 6502 line |
| 38605 | E2-230K | NM_022066.2 | 1071 | gtgaagcgtctcggatgctttga | 228262 | - | see also 17011 line |
| 38606 | E2-230K | NM_022066.2 | 1243 | aggttgtgcggatcatgtcatgc | 228266 | - | see also 17011 line |
| 38607 | FBXL3A | NM_012158.1 | 1451 | gccgcctatctcaattatccatt | 228350 | - | see also 5154 line |

EP 1 752 536 A1

Figure 46

| | | | | | | | |
|---|---|---|---|---|---|---|---|
| 38608 | FBXL3A | NM_012158.1 | 1323 | tggattacggccactgatgaag | 228342 | - | see also 5154 line |
| 38609 | FBXL3B | NM_012159.1 | 1032 | tcctcgactgattgagttagtgg | 228377 | - | see also 17013 line |
| 38610 | FBXL3B | NM_012159.1 | 771 | ctcaagcgagactcatgttaacc | 228360 | - | see also 17013 line |
| 38611 | FBXL5 | NM_012161.2 | 885 | gtcccgcaactgaactgatact | 228399 | - | see also 5162 line |
| 38612 | FBXL5 | NM_012161.2 | 1002 | ctgcggaggaatcaattgctatc | 228403 | - | see also 5162 line |
| 38613 | FBXL6 | NM_012162.1 | 1115 | gcgagctcaacctgcaacttgt | 228427 | - | see also 17015 line |
| 38614 | FBXL6 | NM_012162.1 | 360 | gagaccgcattcccttgaaatc | 228424 | - | see also 17015 line |
| 38615 | FBXL7 | NM_012304.3 | 1706 | atcggcaaatgcccttggtatc | 228435 | - | see also 17016 line |
| 38616 | FBXL7 | NM_012304.3 | 545 | gacgtgagttcaagtacagatca | 228430 | - | see also 17016 line |
| 38617 | FBXO11 | NM_012167.1 | 1081 | cactgtatcatccgaagtacatg | 228467 | - | see also 17017 line |
| 38618 | FBXO11 | NM_012167.1 | 1425 | tacagtggttcgatgtgaaattc | 228474 | - | see also 17017 line |
| 38619 | FBXO2 | NM_012168.2 | 991 | tacgagctcaccgttaagctact | 228530 | - | see also 17018 line |
| 38620 | FBXO21 | NM_015002.1 | 332 | gggttagaagcgcggaagattgt | 228536 | - | see also 6184 line |
| 38621 | FBXO24 | NM_012172.2 | 245 | gagaaggccggtcccttgctaag | 228571 | - | see also 17020 line |
| 38622 | FBXO24 | NM_012172.2 | 1098 | tgcggctggcctgcatgacttcc | 228582 | - | see also 17020 line |
| 38623 | FBXO3 | NM_012175.2 | 440 | aagcttcctgaacgattatcgatg | 228602 | - | see also 5178 line |
| 38624 | FBXO3 | NM_012175.2 | 447 | ctgacgattatcgatgttcatac | 228604 | - | see also 5178 line |
| 38625 | FBXO4 | NM_012176.1 | 242 | gccgattgatgtacagctatata | 228631 | - | see also 17022 line |
| 38626 | FBXO4 | NM_012176.1 | 687 | cagaggcagattgatggttattgg | 228643 | - | see also 17022 line |
| 38627 | FBXO5 | NM_012177.2 | 170 | ctcgaccctccgatagttgtaaa | 228661 | - | see also 17023 line |
| 38628 | FBXO5 | NM_012177.2 | 410 | ctgaaagcaagcgcttgcataac | 228672 | - | see also 17023 line |
| 38629 | FBXO7 | NM_012179.2 | 695 | atgttaggcgctagtcaaaattt | 228720 | - | see also 5180 line |
| 38630 | FBXO7 | NM_012179.2 | 521 | gacattccagcgcctaatataac | 228714 | - | see also 5180 line |
| 38631 | FBXO9 | NM_012347.2 | 842 | ttcggtttgatggcgtgtatatc | 228770 | - | see also 17025 line |
| 38632 | FBXO9 | NM_012347.2 | 848 | ttgatggccgtgtatatcagtaaa | 228771 | - | see also 17025 line |
| 38633 | FBXW1B | NM_012300.1 | 1223 | caccgggctgccgtcaagtagt | 228815 | - | see also 5267 line |
| 38634 | FBXW1B | NM_012300.1 | 1002 | atgatgagcgtgtcattgtaact | 228812 | - | see also 5267 line |
| 38635 | FBXW2 | NM_012164.2 | 1298 | tgcggacagagagcctgattagt | 228852 | - | see also 5165 line |
| 38636 | FLJ11011 | NM_018299.1 | 306 | agcgctcagtccaatcagttt | 228858 | - | see also 17027 line |
| 38637 | FLJ11011 | NM_018299.1 | 183 | tagtagtcgatatccttttgact | 228854 | - | see also 17027 line |
| 38638 | FLJ25157 | NM_152653.1 | 486 | ttcaccagactatccgtttaaac | 228885 | - | see also 17029 line |
| 38639 | FLJ25157 | NM_152653.1 | 515 | aggttaccttccgaacaagaatc | 228888 | - | see also 17029 line |
| 38640 | FTS | NM_022476.1 | 857 | gaccccatgcaattagctttc | 228918 | - | see also 17030 line |
| 38641 | FTS | NM_022476.1 | 818 | gtgtgcactgctcgtttgtttga | 228914 | - | see also 17030 line |
| 38642 | HACE1 | NM_020771.2 | 417 | atgcattcggacgtgtgaaaaga | 228926 | - | see also 7947 line |
| 38643 | HACE1 | NM_020771.2 | 659 | gtgaattgggcggacagaaactact | 228933 | - | see also 7947 line |
| 38644 | HECTD1 | NM_015382.1 | 4747 | ctgggagtcctagtgcaatatcc | 229129 | - | see also 17032 line |

Figure 46

| 38645 | HECTD1 | NM_015382.1 | 5669 | tagacctggtcgtactaatgtcc | 229156 | - | see also 17032 line |
|---|---|---|---|---|---|---|---|
| 38646 | HERC1 | NM_003922.1 | 9748 | atccgaacgctagttcgattaat | 229480 | - | see also 17033 line |
| 38647 | HERC1 | NM_003922.1 | 9749 | tccgaacgctagttcgattaatg | 229481 | - | see also 17033 line |
| 38648 | HERC2 | NM_004667.2 | 9228 | gccggcacgcgacggctttaact | 229668 | - | see also 3188 line |
| 38649 | HERC2 | NM_004667.2 | 9565 | gaccgatgaaggtttggtatttt | 229677 | - | see also 3188 line |
| 38650 | HERC2 | NM_004667.2 | 13211 | ttcgacactctccgaggaattct | 229706 | - | see also 3188 line |
| 38651 | HERC3 | NM_014606.1 | 1227 | cccgagctgatcgctttaaatat | 229747 | - | see also 5769 line |
| 38652 | HERC3 | NM_014606.1 | 883 | acgcacgcaaaaagttgtctata | 229731 | - | see also 5769 line |
| 38653 | HERC3 | NM_014606.1 | 1810 | gtctcaggtatgcccgaaatatt | 229769 | - | see also 5769 line |
| 38654 | HIP2 | NM_005339.3 | 389 | gactctccgcacggtattattgt | 229825 | - | see also 3728 line |
| 38655 | HIP2 | NM_005339.3 | 391 | ctctccgcacggtattattgtca | 229826 | - | see also 3728 line |
| 38656 | HSPC150 | NM_014176.1 | 223 | gacctgcgagctcaaatattagg | 229841 | - | see also 5525 line |
| 38657 | HSPC150 | NM_014176.1 | 221 | atgacctgcgagctcaaatatta | 229840 | - | see also 5525 line |
| 38658 | ITCH | NM_031483.3 | 1062 | cacgggcgagtttactatgtaga | 229882 | - | see also 17038 line |
| 38659 | ITCH | NM_031483.3 | 1065 | gggcgagtttactatgtagatca | 229884 | - | see also 17038 line |
| 38660 | KIAA0010 | NM_014671.1 | 898 | ttgcactacatgattcacaatgg | 229949 | - | see also 5849 line |
| 38661 | KIAA0010 | NM_014671.1 | 1065 | aggtgcgaggcaacaagtttta | 229956 | - | see also 5849 line |
| 38662 | KIAA0317 | NM_014821.1 | 2237 | taggactgcgtatgcattacaag | 230072 | - | see also 6017 line |
| 38663 | KIAA0322 | NM_015052.1 | 390 | atcgatgccagctcgtactttgt | 230093 | - | see also 6210 line |
| 38664 | KIAA0322 | NM_015052.1 | 3120 | aacggtcgtcttcccaatcatct | 230120 | - | see also 6210 line |
| 38665 | KIAA0322 | NM_015052.1 | 4415 | caccgcggaaatcgacctaaatg | 230148 | - | see also 6210 line |
| 38666 | LOC51619 | NM_015983.2 | 370 | agcgttgactgtgtcaaaagttc | 230160 | - | see also 17042 line |
| 38667 | LOC51619 | NM_015983.2 | 503 | gagtggacacaaaaatatgctat | 230162 | - | see also 17042 line |
| 38668 | LOC92912 | NM_173469.1 | 1252 | agcaagagcccaacaatcctata | 230171 | - | see also 17043 line |
| 38669 | LOC92912 | NM_173469.1 | 809 | gggtcagtgcaagcttcagatag | 230165 | - | see also 17043 line |
| 38670 | MGC35130 | NM_152489.1 | 723 | tacctaaagacccacgtaaatgt | 230191 | - | see also 17044 line |
| 38671 | MGC35130 | NM_152489.1 | 299 | aacaattataagggtatcactgc | 230177 | - | see also 17044 line |
| 38672 | NCE2 | NM_080678.1 | 618 | tggatgactacatcaaacgttat | 230211 | - | see also 17045 line |
| 38673 | NCE2 | NM_080678.1 | 97 | aacgctagcaagtaaactgaagc | 230196 | - | see also 17045 line |
| 38674 | NEDD4 | NM_006154.1 | 689 | atccttggaaggacctattatgt | 230230 | - | see also 4260 line |
| 38675 | NEDD4 | NM_006154.1 | 2596 | tgctgaactatacggttcaaatg | 230276 | - | see also 4260 line |
| 38676 | NEDD4L | NM_015277.2 | 1732 | ttggatggccgaacgttttatat | 230389 | - | see also 6303 line |
| 38677 | NEDD4L | NM_015277.2 | 1737 | tggccgaacgttttatattgatc | 230391 | - | see also 6303 line |
| 38678 | RBAF600 | NM_020765.1 | 9619 | cgccgtcaagtccgcaaacttct | 230637 | - | see also 17047 line |
| 38679 | RBAF600 | NM_020765.1 | 13139 | ggccgctgatgagggatataaag | 230707 | - | see also 17047 line |

Figure 46

| | | | | | | | |
|---|---|---|---|---|---|---|---|
| 38680 | RBAF600 | NM_020765.1 | 13850 | ttcgcatcatccgtaccttcc | 230722 | - | see also 17047 line |
| 38681 | SMURF1 | NM_020429.1 | 1436 | gagatacgaaagagatctagtcc | 230785 | - | see also 7871 line |
| 38682 | SMURF1 | NM_020429.1 | 360 | aagatccgtctgacagtgttatg | 230759 | - | see also 7871 line |
| 38683 | SMURF2 | NM_022739.2 | 754 | cacgaccggcatccgaatattct | 230817 | - | see also 17049 line |
| 38684 | SMURF2 | NM_022739.2 | 2266 | caggcccgagactctttaccata | 230862 | - | see also 17049 line |
| 38685 | TRIP12 | NM_004238.1 | 1609 | tgcttgtcgagccttaacataca | 230906 | - | see also 2828 line |
| 38686 | TRIP12 | NM_004238.1 | 1608 | atgcttgtcgagccttaacatac | 230905 | - | see also 2828 line |
| 38687 | UBE2A | NM_003336.2 | 431 | tagtatatgtctggacatacttc | 231072 | - | see also 17051 line |
| 38688 | UBE2A | NM_003336.2 | 475 | atgatgttcttccattcttaaca | 231073 | - | see also 17051 line |
| 38689 | UBE2B | NM_003337.2 | 752 | tgctggatgaaccgaatcctaac | 231090 | - | see also 2257 line |
| 38690 | UBE2B | NM_003337.2 | 683 | tagatatccttcagaatcgatgg | 231086 | - | see also 2257 line |
| 38691 | UBE2C | NM_007019.2 | 532 | atgctgccgagctctggaaaaac | 231097 | - | cell_cycle |
| 38692 | UBE2D1 | NM_003338.3 | 381 | ctgtacatttccgacagattat | 231106 | - | see also 2258 line |
| 38693 | UBE2D1 | NM_003338.3 | 572 | gaccccttagtaccagatattgc | 231110 | - | see also 2258 line |
| 38694 | UBE2D2 | NM_003339.2 | 755 | tccctatcaggggtggagtattt | 231116 | - | see also 17054 line |
| 38695 | UBE2D2 | NM_003339.2 | 717 | ttccattggcaagctacaataat | 231115 | - | see also 17054 line |
| 38696 | UBE2E1 | NM_003341.3 | 384 | tcccaaaggcgataacatctatg | 231130 | - | see also 17055 line |
| 38697 | UBE2E1 | NM_003341.3 | 383 | gtcccaaaggggataacatctat | 231129 | - | see also 17055 line |
| 38698 | UBE2G1 | NM_003342.3 | 469 | accgatgggaagtccttattatt | 231143 | - | see also 2259 line |
| 38699 | UBE2G1 | NM_003342.3 | 468 | taccgatggaagtccttattat | 231142 | - | see also 2259 line |
| 38700 | UBE2G2 | NM_003343.4 | 267 | ccgttaagtcccccaaagatga | 231159 | - | see also 17057 line |
| 38701 | UBE2G2 | NM_003343.4 | 156 | cccatgaatgaaagaagaacttt | 231157 | - | see also 17057 line |
| 38702 | UBE2H | NM_003344.2 | 534 | aaggcggagtatggaaagttaga | 231166 | - | see also 17058 line |
| 38703 | UBE2H | NM_003344.2 | 553 | tagagtggacctacctgataaat | 231168 | - | see also 17058 line |
| 38704 | UBE2I | NM_003345.3 | 538 | agcagaggcctacacgatttact | 231192 | - | see also 17059 line |
| 38705 | UBE2I | NM_003345.3 | 537 | aagcagaggcctacacgatttac | 231191 | - | see also 17059 line |
| 38706 | UBE2J1 | NM_016021.2 | 565 | aacggctaatgtcgatttgaag | 231202 | - | see also 6622 line |
| 38707 | UBE2J1 | NM_016021.2 | 706 | agccataggttcttctagattaca | 231213 | - | see also 6622 line |
| 38708 | UBE2J2 | NM_058167.2 | 888 | ggcgaacttgttgtgatagttg | 231243 | - | see also 17061 line |
| 38709 | UBE2J2 | NM_058167.2 | 413 | ctcccagtatctatatgatcact | 231238 | - | see also 17061 line |
| 38710 | UBE2J2 | NM_058167.2 | 591 | gtcggacttcacgaaaagacaac | 231240 | - | see also 17061 line |
| 38711 | UBE2L6 | NM_004223.3 | 283 | ttcaagcctcccatgatcaaatt | 231248 | - | see also 17062 line |
| 38712 | UBE2Q | NM_017582.5 | 551 | tccgacctgtgtaaactcctataa | 231264 | - | see also 7037 line |
| 38713 | UBE2Q | NM_017582.5 | 550 | ctccgacctgtgtaaactctata | 231263 | - | see also 7037 line |
| 38714 | UBE2R2 | NM_017811.2 | 750 | ctggcggaatactgtcatcaaaac | 231303 | - | see also 7156 line |
| 38715 | UBE2R2 | NM_017811.2 | 549 | gtgaggactatcctattaagtgt | 231295 | - | see also 7156 line |
| 38716 | UBE2V2 | NM_003350.2 | 383 | agctaagacgtctaatgatgtcc | 231321 | - | see also 17064 line |

Figure 46

| | | | | | | | |
|---|---|---|---|---|---|---|---|
| 38717 | UBE2V2 | NM_003350.2 | 319 | gcccggagcataccagtgttagc | 231319 | - | see also 17064 line |
| 38718 | UBE3A | NM_000462.2 | 214 | gccctgagctttataagattaa | 231328 | - | see also 364 line |
| 38719 | UBE3A | NM_000462.2 | 829 | tactctcgagatcctaattatct | 231343 | - | see also 364 line |
| 38720 | UBE3B | NM_130466.2 | 712 | caggccatgtccggagtttct | 231401 | - | see also 9545 line |
| 38721 | UBE3B | NM_130466.2 | 1911 | gtcgcatcagtccggaatattc | 231434 | - | see also 9545 line |
| 38722 | UBR1 | NM_174916.1 | 3833 | ttggcgttgagtcttcgattaaa | 231582 | - | see also 10125 line |
| 38723 | UBR1 | NM_174916.1 | 3835 | ggcgttgagtcttcgattaaata | 231584 | - | see also 10125 line |
| 38724 | C6orf104 | NM_031949.2 | 610 | ttcgtcatgccaatgactatac | 231642 | - | see also 17068 line |
| 38725 | C6orf104 | NM_031949.2 | 675 | caccaagcatagctattggatt | 231647 | - | see also 17068 line |
| 38726 | FLJ35808 | NM_173623.1 | 1275 | gacggacagggttgtatccttta | 231699 | - | see also 17069 line |
| 38727 | FLJ35808 | NM_173623.1 | 1046 | ctctaccgatctgaggaatatc | 231690 | - | see also 17069 line |
| 38728 | FLJ36119 | NM_153254.1 | 324 | tgcagactcggatgaacactaacg | 231708 | - | see also 17070 line |
| 38729 | FLJ36119 | NM_153254.1 | 1116 | caccacacctacatgatcttct | 231710 | - | see also 17070 line |
| 38730 | KIAA0153 | NM_015140.1 | 627 | cgccacggcaaccttcttctaca | 231713 | - | see also 17071 line |
| 38731 | KIAA0153 | NM_015140.1 | 1529 | agcacttcacggtcatgaactat | 231716 | - | see also 17071 line |
| 38732 | KIAA0173 | NM_014640.1 | 1052 | gtgcccatatcgccttgtctacc | 231730 | - | see also 17072 line |
| 38733 | KIAA0173 | NM_014640.1 | 1117 | tggtataaccggaataacttagc | 231733 | - | see also 17072 line |
| 38734 | KIAA0998 | NM_015072.2 | 1707 | caggatcgctgctactaaacaag | 231818 | - | see also 17073 line |
| 38735 | KIAA0998 | NM_015072.2 | 1704 | aggcaggatcgtgctatctaaac | 231816 | - | see also 17073 line |
| 38736 | LOC158135 | NM_194252.1 | 951 | gacggactctctaggttttgtac | 231842 | - | see also 17074 line |
| 38737 | LOC158135 | NM_194252.1 | 895 | ttcgtctgtatgtcttactcaag | 231836 | - | see also 17074 line |
| 38738 | TTL | NM_153712.2 | 729 | cacgtgatccagaaatatcttga | 231875 | - | see also 10017 line |
| 38739 | TTL | NM_153712.2 | 726 | gttgcacgtgatccagaaatatct | 231874 | - | see also 10017 line |
| 38740 | TTLL1 | NM_012263.2 | 845 | accgtccactgcgcttacatg | 231896 | - | see also 17076 line |
| 38741 | TTLL1 | NM_012263.2 | 1290 | cgccgtccgaatggtgaaatcc | 231905 | - | see also 17076 line |
| 38742 | TTLL1 | NM_012263.2 | 729 | ttcgtttgtctcaatctaata | 231893 | - | see also 17076 line |
| 38743 | GCLC | NM_001498.1 | 1125 | gacatcgacttgacgatagataa | 231943 | - | see also 1027 line |
| 38744 | GCLC | NM_001498.1 | 1070 | atcccgatatgactcaatagaca | 231939 | - | see also 1027 line |
| 38745 | GCLM | NM_002061.1 | 996 | ttgcggtattcggtcattgtgaaa | 231979 | - | see also 17078 line |
| 38746 | GCLM | NM_002061.1 | 734 | gccataggtaccctcgatctaga | 231972 | - | see also 17078 line |
| 38747 | GCLM | NM_002061.1 | 785 | tgggcacaggtaaaaccaaatag | 231974 | - | see also 17078 line |
| 38748 | PAICS | NM_006452.2 | 757 | ttgtacactggttgatatgaaga | 231998 | - | see also 17079 line |
| 38749 | PAICS | NM_006452.2 | 211 | gacagctgaggtactgaacattg | 231983 | - | see also 17079 line |
| 38750 | MCCC2 | NM_022132.3 | 1740 | agccctcaacgcaccaatagaga | 232041 | - | see also 17080 line |
| 38751 | MCCC2 | NM_022132.3 | 672 | tgggcgtacattctataatcagg | 232014 | - | see also 17080 line |

Figure 46

| 38752 | PCCB | NM_000532.2 | 394 | cactggacgaggccgaatcaatg | 232045 | - | see also 17081 line |
|---|---|---|---|---|---|---|---|
| 38753 | PCCB | NM_000532.2 | 399 | gacgaggccgaatcaatggaaga | 232046 | - | see also 17081 line |
| 38754 | CTPS | NM_001905.1 | 143 | tgggcacaatactcaagtcatgt | 232071 | - | see also 1218 line |
| 38755 | CTPS | NM_001905.1 | 459 | ggcgttaatacctgtagatgaag | 232083 | - | see also 1218 line |
| 38756 | CTPS2 | NM_019857.3 | 669 | gagccccaaatatgcgttattga | 232116 | - | see also 7740 line |
| 38757 | CTPS2 | NM_019857.3 | 905 | aagttcaacgcccattgagatgg | 232121 | - | see also 7740 line |
| 38758 | HLCS | NM_000411.3 | 1620 | accttccctacgattatagcagc | 232153 | - | see also 325 line |
| 38759 | HLCS | NM_000411.3 | 2845 | cccgtttgtccagcatctgatgt | 232177 | - | see also 325 line |
| 38760 | ASNS | NM_001673.2 | 1729 | tactaccgtcaagtctttgaacg | 232194 | - | see also 17085 line |
| 38761 | ASNS | NM_001673.2 | 810 | acccctgcacgccctctatgaca | 232189 | - | see also 17085 line |
| 38762 | LIAS | NM_006859.2 | 141 | gcccagtcagaccgttaagctcc | 232195 | - | see also 17086 line |
| 38763 | LIAS | NM_006859.2 | 817 | tcgtgatcctcgggccaattttg | 232212 | - | see also 17086 line |
| 38764 | GLUL | NM_002065.3 | 735 | accctaacaagctggtgttatgt | 232234 | - | see also 17087 line |
| 38765 | GLUL | NM_002065.3 | 1307 | taccacatccgtgcctatgatcc | 232243 | - | see also 17087 line |
| 38766 | GLUL | NM_002065.3 | 1526 | atccgcacgtgtcttctcaatga | 232247 | - | see also 17087 line |
| 38767 | GLULD1 | NM_016571.1 | 1237 | aggcacccggatagaaaataaac | 232286 | - | see also 6919 line |
| 38768 | GLULD1 | NM_016571.1 | 1462 | aggagaaacctttattcgatatt | 232294 | - | see also 6919 line |
| 38769 | HAL | NM_002108.2 | 387 | ggccgtgaggcgctatatcaaga | 232297 | - | see also 1359 line |
| 38770 | HAL | NM_002108.2 | 386 | aggccgtgaggcgctatatcaag | 232296 | - | see also 1359 line |
| 38771 | BCL7C | NM_004765.2 | 666 | cccgaagcttaccctgtctttga | 232343 | - | see also 17090 line |
| 38772 | BCL7C | NM_004765.2 | 391 | ctctcatcctgctggatcttaat | 232341 | - | see also 17090 line |
| 38773 | RCL1 | NM_005772.2 | 239 | ccccgtcaaaatccgaaagattc | 232344 | - | see also 4030 line |
| 38774 | RCL1 | NM_005772.2 | 329 | aacgaatggttctcgaattgaaa | 232348 | - | see also 4030 line |
| 38775 | RCL1 | NM_005772.2 | 486 | cacccgttaaaaatagttctacg | 232356 | - | see also 4030 line |
| 38776 | SLC27A2 | NM_003645.2 | 666 | ctgctggtgtcgccagaactaca | 232374 | - | see also 17092 line |
| 38777 | SLC27A2 | NM_003645.2 | 590 | tgccatggcgtgcctcaattaca | 232371 | - | see also 17092 line |
| 38778 | SLC27A5 | NM_012254.1 | 1641 | ggcaatcgggcgacgtttactac | 232433 | - | see also 17093 line |
| 38779 | SLC27A5 | NM_012254.1 | 1645 | atcgggcgacgtttactacaaca | 232434 | - | see also 17093 line |
| 38780 | SLC27A6 | NM_014031.1 | 1238 | cagctaccgaatcaagcatatct | 232463 | - | see also 17094 line |
| 38781 | SLC27A6 | NM_014031.1 | 1808 | atgcttgtccacgatttttaaga | 232478 | - | see also 17094 line |
| 38782 | SUCLG2 | NM_003848.1 | 149 | gacaacggagtgagagttcaaag | 232484 | - | - | | |
| 38783 | SUCLA2 | NM_003850.1 | 590 | aaggtcctgtattaataggaagt | 232503 | - | see also 17095 line |
| 38784 | SUCLA2 | NM_003850.1 | 262 | gtctccgttcccaaaggatatgt | 232492 | - | see also 17095 line |
| 38785 | ACAS2L | NM_032501.2 | 1881 | gccgacatcggttggattacagg | 232533 | - | see also 9222 line |
| 38786 | ACAS2L | NM_032501.2 | 1241 | gtcccccgagagcgttgctttga | 232525 | - | see also 9222 line |
| 38787 | ACAS2L | NM_032501.2 | 1244 | ccccgagagcgttgctttgatct | 232526 | - | see also 9222 line |
| 38788 | AACS | NM_023928.2 | 1569 | gcggcgagctggtgtgtactaag | 232566 | - | see also 17097 line |

## Figure 46

| | | | | | | | |
|---|---|---|---|---|---|---|---|
| 38789 | AACS | NM_023928.2 | 1777 | cggcagctcggaaatctataaca | 232571 | - | see also 17097 line |
| 38790 | BIRC6 | NM_016252.1 | 12913 | cccgtcagtagtgcggtaaatgg | 232969 | - | see also 6755 line |
| 38791 | BIRC6 | NM_016252.1 | 4106 | gacgaagtatagcccataagtgt | 232698 | - | see also 6755 line |
| 38792 | BTRC | NM_003939.2 | 603 | ctggatgccaaatcactatgtgc | 233028 | - | see also 2696 line |
| 38793 | BTRC | NM_003939.2 | 961 | gccttcgagacaacacaatcaag | 233040 | - | see also 2696 line |
| 38794 | C20orf18 | NM_006462.2 | 1100 | ccgggtgcaccttcatcaacaag | 233061 | - | see also 17100 line |
| 38795 | C20orf18 | NM_006462.2 | 1611 | ttgcaagaccccagattgcaagg | 233064 | - | see also 17100 line |
| 38796 | CUL1 | NM_003592.2 | 1608 | gagttgctggctcgatactgtga | 233113 | - | see also 2402 line |
| 38797 | CUL1 | NM_003592.2 | 1839 | gggttcgagtacacctctaaact | 233117 | - | see also 2402 line |
| 38798 | GGCX | NM_000821.2 | 440 | taccggataagctgtgtgttatt | 233150 | - | see also 520 line |
| 38799 | GGCX | NM_000821.2 | 1296 | ctggcgaactgggctaccttaac | 233161 | - | see also 520 line |
| 38800 | LIPT1 | NM_015929.2 | 775 | ctgatgggacgttcttgtcttct | 233207 | - | see also 17102 line |
| 38801 | LIPT1 | NM_015929.2 | 828 | agcaatgccactgctagcatacc | 233210 | - | see also 17102 line |
| 38802 | MOCS3 | NM_014484.3 | 260 | gaccgcgtgcgtgctaatcgtgg | 233258 | - | see also 17103 line |
| 38803 | MOCS3 | NM_014484.3 | 519 | gacctggtccgccgatatgatgt | 233260 | - | see also 17103 line |
| 38804 | PARC | NM_015089.1 | 599 | gtcgggctcacgtccttctatca | 233282 | - | see also 17104 line |
| 38805 | PARC | NM_015089.1 | 1743 | ctcgtctacttcacgaaatcact | 233297 | - | see also 17104 line |
| 38806 | RNF25 | NM_022453.2 | 853 | ggctgagcgaaaccgatacttca | 233358 | - | see also 8361 line |
| 38807 | RNF25 | NM_022453.2 | 158 | gagtccatctatctagatgaact | 233348 | - | see also 8361 line |
| 38808 | UBE1 | NM_003334.2 | 2955 | accacgtcaccagtactataacc | 233406 | - | see also 17106 line |
| 38809 | UBE1 | NM_003334.2 | 1967 | tcctgacagagtcgtacagttcc | 233392 | - | see also 17106 line |
| 38810 | UBE1L | NM_003335.2 | 3036 | ggccggatggtcacctgaaaagc | 233430 | - | see also 17107 line |
| 38811 | UBE1L | NM_003335.2 | 2956 | gagtcgctgctggctcatcttca | 233429 | - | see also 17107 line |
| 38812 | C18B11 | NM_152260.1 | 829 | acctcagggggtgcttatgtttgc | 233435 | - | see also 17108 line |
| 38813 | C18B11 | NM_152260.1 | 1042 | ttccagaggctaagctacaatgg | 233440 | - | see also 17108 line |
| 38814 | CLONE249 22 | NM_015679.1 | 40 | tgggcttttcgcggtctctaaagc | 233446 | | see also 17109 line |
| 38815 | CLONE249 22 | NM_015679.1 | 39 | atgggcttttcgcggtctataag | 233445 | - | see also 17109 line |
| 38816 | FLJ14494 | NM_032795.1 | 1066 | tgcaccgcctgcgtttagagatg | 233471 | - | see also 17110 line |
| 38817 | FLJ14494 | NM_032795.1 | 285 | aaggcactgacccgaggaattct | 233464 | - | see also 17110 line |
| 38818 | PUS1 | NM_025215.3 | 584 | ctcgtccggtcaggctgtattcc | 233475 | - | see also 17111 line |
| 38819 | PUS1 | NM_025215.3 | 587 | gtccggtcaggctgtattcctga | 233476 | - | see also 17111 line |
| 38820 | RLUCL | NM_058192.1 | 837 | cgctgtgcgtggccctaaacaag | 233480 | - | - | - | - |
| 38821 | TRUB1 | NM_139169.3 | 904 | accatttacgctagaagaacatg | 233501 | - | see also 17112 line |
| 38822 | TRUB1 | NM_139169.3 | 86 | gtgtcttcgccgtctttgaaaac | 233482 | - | see also 17112 line |
| 38823 | ECH1 | NM_001398.1 | 207 | gacgtctgcgcagaaacatgttc | 233508 | - | see also 17113 line |

Figure 46

| 38824 | ECH1 | NM_001398.1 | 293 | tggtagagtgcttcaacaagatt | 233510 | - | see also 17113 line |
|---|---|---|---|---|---|---|---|
| 38825 | ECHS1 | NM_004092.2 | 152 | cgcctcgggtgctaactttgagt | 233514 | - | see also 2760 line |
| 38826 | UROS | NM_000375.1 | 831 | ctggtgacaatatcgatcaaatt | 233536 | - | see also 17115 line |
| 38827 | UROS | NM_000375.1 | 235 | ctgtggccaggatccgtatatca | 233526 | - | see also 17115 line |
| 38828 | ALAD | NM_000031.3 | 774 | tccgggatgcagctaagtcaagc | 233544 | - | see also 17116 line |
| 38829 | ALAD | NM_000031.3 | 366 | gcctacgctgtgtcttgatcttt | 233539 | - | see also 17116 line |
| 38830 | ALAD | NM_000031.3 | 544 | ctgcgggctcctgagtgaaaacg | 233540 | - | see also 17116 line |
| 38831 | FH | NM_000143.2 | 717 | atcaagattggacgtactcatac | 233566 | - | see also 128 line |
| 38832 | FH | NM_000143.2 | 213 | aaggtgccaaatgataagtatta | 233550 | - | see also 128 line |
| 38833 | CBS | NM_000071.1 | 812 | aagaacgaaatccccaattctca | 233581 | - | see also 45 line |
| 38834 | CBS | NM_000071.1 | 1776 | gggtggtcaccgccattgacttg | 233583 | - | see also 45 line |
| 38835 | TGDS | NM_014305.1 | 415 | ttcgtacgtgcctttgagtttac | 233594 | - | see also 17119 line |
| 38836 | TGDS | NM_014305.1 | 452 | atggcactcacgtttttggtaagt | 233598 | - | see also 17119 line |
| 38837 | GMDS | NM_001500.1 | 653 | tccgtgaggcgtataatctcttt | 233630 | - | see also 1030 line |
| 38838 | GMDS | NM_001500.1 | 236 | tacggcggtccagttcatttaat | 233616 | - | see also 1030 line |
| 38839 | FLJ31300 | NM_144639.1 | 1065 | tcctgccacaacccgttcaatgg | 233663 | - | see also 17121 line |
| 38840 | FLJ31300 | NM_144639.1 | 587 | ctcctcccggacggagtatgaga | 233656 | - | see also 17121 line |
| 38841 | PTS | NM_000317.1 | 260 | ccctgctacgggaatggttatga | 233681 | - | see also 17122 line |
| 38842 | PTS | NM_000317.1 | 128 | cagcgcgagccaccgattgtaca | 233670 | - | see also 17122 line |
| 38843 | ODC-p | NM_052998.2 | 1549 | agggcgtgtatgggatcttcaac | 233697 | - | see also 17123 line |
| 38844 | ODC-p | NM_052998.2 | 924 | aacccctgtaagcaaattgcaca | 233692 | - | see also 17123 line |
| 38845 | ODC1 | NM_002539.1 | 1540 | gccgacgatctactatgtgatgt | 233738 | - | see also 17124 line |
| 38846 | ODC1 | NM_002539.1 | 1539 | ggccgacgatctactatgtgatg | 233737 | - | see also 17124 line |
| 38847 | GAD1 | NM_000817.1 | 1095 | caggtcatcctcgatttttcaac | 233748 | - | see also 515 line |
| 38848 | GAD1 | NM_000817.1 | 1121 | ctctccactggattggatattat | 233750 | - | see also 515 line |
| 38849 | GAD2 | NM_000818.1 | 837 | gggctctggcgatgggatatttt | 233786 | - | see also 516 line |
| 38850 | GAD2 | NM_000818.1 | 1155 | caccgtgtacggagcatttgacc | 233798 | - | see also 516 line |
| 38851 | UROD | NM_000374.2 | 136 | ggccgttacttaccagagtttag | 233820 | - | see also 17127 line |
| 38852 | UROD | NM_000374.2 | 137 | gccgttacttaccagagtttagg | 233821 | - | see also 17127 line |
| 38853 | UROD | NM_000374.2 | 268 | ttctccgacatccttgttgtacc | 233822 | - | see also 17127 line |
| 38854 | HDC | NM_002112.1 | 1334 | ttcgtctaaagggtcctaattgt | 233842 | - | see also 1361 line |
| 38855 | HDC | NM_002112.1 | 1163 | gccgacggtttcgctctgttaaa | 233834 | - | see also 1361 line |
| 38856 | HDC | NM_002112.1 | 1414 | gccactatccaggacaagttaat | 233845 | - | see also 1361 line |
| 38857 | DDC | NM_000790.1 | 238 | atcaacgacgttgagaagataat | 233851 | - | see also 17129 line |
| 38858 | DDC | NM_000790.1 | 242 | acgacgttgagaagataatcatg | 233852 | - | see also 17129 line |
| 38859 | AMD1 | NM_001634.3 | 811 | tcccagagagtcgggtaatcagt | 233874 | - | see also 17130 line |
| 38860 | AMD1 | NM_001634.3 | 428 | aagatctgagtgggacatacttt | 233865 | - | see also 17130 line |

Figure 46

| 38861 | KIAA0251 | NM_015027.1 | 1501 | agctcgtagcctgcatagaaagc | 233882 | - | see also 17131 line |
|---|---|---|---|---|---|---|---|
| 38862 | KIAA0251 | NM_015027.1 | 2024 | ttctccggtccaggctttacaga | 233893 | - | see also 17131 line |
| 38863 | KIAA0251 | NM_015027.1 | 2007 | tccgtgctgaattggttttctcc | 233892 | - | see also 17131 line |
| 38864 | SGPL1 | NM_003901.2 | 453 | gcccattattggtcgtaagattc | 233910 | - | see also 2657 line |
| 38865 | SGPL1 | NM_003901.2 | 1333 | cagttcttcgtcgatacagattg | 233928 | - | see also 2657 line |
| 38866 | ALDOA | NM_000034.2 | 1976 | tgcgcaggaggagtatgtcaagc | 233945 | - | see also 17133 line |
| 38867 | ALDOA | NM_000034.2 | 1720 | atgcttgcactcagaagttttct | 233943 | - | see also 17133 line |
| 38868 | ALDOB | NM_000035.2 | 432 | atcgtggtgggaatcaagttaga | 233947 | - | see also 17134 line |
| 38869 | ALDOB | NM_000035.2 | 852 | aagtatactccagaacaagtagc | 233956 | - | see also 17134 line |
| 38870 | ALDOC | NM_005165.1 | 769 | atgcctgtccatcaagtatacc | 233969 | - | see also 17135 line |
| 38871 | ALDOC | NM_005165.1 | 935 | ccgaccctgggcgcttaccttct | 233971 | - | see also 17135 line |
| 38872 | CGI-26 | NM_015954.1 | 533 | atcgacgtggtaattaacagaag | 233982 | - | see also 6588 line |
| 38873 | CGI-26 | NM_015954.1 | 755 | gccaccttcccggtagctatagt | 233991 | - | see also 6588 line |
| 38874 | CGI-26 | NM_015954.1 | 526 | tacagaaatcgacgtggtaatta | 233980 | - | see also 6588 line |
| 38875 | CRY2 | NM_021117.1 | 1381 | agcgttcccctctcgatacatct | 234017 | - | see also 8091 line |
| 38876 | HMGCL | NM_000191.1 | 130 | gtccccgagatggactacaaaat | 234025 | - | see also 158 line |
| 38877 | HMGCL | NM_000191.1 | 434 | ttgttccatagaggagagttttc | 234036 | - | see also 158 line |
| 38878 | GUCY1A2 | NM_000855.1 | 1534 | ctgtctacccgtttgtgattag | 234062 | - | see also 17138 line |
| 38879 | GUCY1A2 | NM_000855.1 | 2272 | ggggtgcgaatgccacgttattg | 234072 | - | see also 17138 line |
| 38880 | GUCY1A3 | NM_000856.1 | 2265 | tggcgtcgttggagttaaaatgc | 234112 | - | see also 17139 line |
| 38881 | GUCY1A3 | NM_000856.1 | 712 | accagtcggagccgagtctatct | 234081 | - | see also 17139 line |
| 38882 | GUCY1B2 | NM_004129.1 | 1561 | cagtgtgcacgcagtctataaag | 234152 | - | see also 2786 line |
| 38883 | GUCY1B2 | NM_004129.1 | 1565 | gtgcacgcagtctataaagtaga | 234153 | - | see also 2786 line |
| 38884 | GUCY1B3 | NM_000857.1 | 1130 | gccacgcgcgatcttgttctttt | 234195 | - | see also 560 line |
| 38885 | GUCY1B3 | NM_000857.1 | 1129 | tgccacgcgcgatcttgttcttt | 234194 | - | see also 560 line |
| 38886 | SAC | NM_018417.2 | 3130 | ttcggctcaacgctttagacatg | 234296 | - | see also 17142 line |
| 38887 | SAC | NM_018417.2 | 4991 | agctaataccgtggacaatcatt | 234335 | - | see also 17142 line |
| 38888 | CGI-150 | NM_016080.2 | 409 | cccgggaggatataagttctatt | 234341 | - | see also 17143 line |
| 38889 | CGI-150 | NM_016080.2 | 818 | gtcggggatgaagcatttcgaga | 234353 | - | see also 17143 line |
| 38890 | GLO1 | NM_006708.1 | 450 | ccctcgaggattcggtcatattg | 234368 | - | see also 17144 line |
| 38891 | GLO1 | NM_006708.1 | 246 | tggaatgacgctaatccaaaaat | 234362 | - | see also 17144 line |
| 38892 | GLO1 | NM_006708.1 | 359 | gggcgctctccagaaaagctaca | 234365 | - | see also 17144 line |
| 38893 | HCCS | NM_005333.1 | 846 | accgttgcgggacagaagttaga | 234385 | - | see also 17145 line |
| 38894 | HCCS | NM_005333.1 | 366 | accaggaacgcgcctatgagtac | 234375 | - | see also 17145 line |
| 38895 | HCCS | NM_005333.1 | 777 | caccaagggcacgaattcgttcc | 234382 | - | see also 17145 line |
| 38896 | CTH | NM_001902.4 | 305 | agcactcgggttttgaatatagc | 234401 | - | see also 17146 line |
| 38897 | CTH | NM_001902.4 | 1185 | tgctgagcttccggcaatcatga | 234432 | - | see also 17146 line |

Figure 46

| 38898 | CTH | NM_001902.4 | 811 | ggcctggtgtctgttaattgtga | 234419 | - | see also 17146 line | | |
|---|---|---|---|---|---|---|---|---|---|
| 38899 | SDS | NM_006843.2 | 501 | aacccgggttgggtctacattcc | 234439 | - | see also 17147 line | | |
| 38900 | SDS | NM_006843.2 | 282 | aagcaaggctgtgcacattttgt | 234437 | - | see also 17147 line | | |
| 38901 | CRYM | NM_001888.1 | 402 | tgccattgccaccaagtttctga | 234444 | - | - | ornithine cyclodeaminase | - |
| 38902 | CRYM | NM_001888.1 | 529 | tggaaccgcaccaaagaaaatgc | 234445 | - | see also 38901 line | | |
| 38903 | ASL | NM_000048.2 | 769 | cactagtgagcgggactttgtgg | 234450 | - | see also 17148 line | | |
| 38904 | ASL | NM_000048.2 | 517 | ggcggaacgtgatgttctcttcc | 234447 | - | see also 17148 line | | |
| 38905 | ASL | NM_000048.2 | 719 | ctccgagcagaactcaactttgg | 234449 | - | see also 17148 line | | |
| 38906 | ADSL | NM_000026.1 | 1086 | ttggtcgtgtaccccaaagtaat | 234472 | - | see also 2 line | | |
| 38907 | ADSL | NM_000026.1 | 87 | gtgtttagcgacagagtataaatt | 234454 | - | see also 2 line | | |
| 38908 | C20orf3 | NM_020531.1 | 328 | accggagtccatagcacatattg | 234484 | - | see also 17150 line | | |
| 38909 | C20orf3 | NM_020531.1 | 530 | tacaagggactatttgaagtaaa | 234486 | - | see also 17150 line | | |
| 38910 | FLJ10916 | NM_018271.2 | 388 | atggcgtcacatatgcatttaag | 234492 | - | see also 17151 line | | |
| 38911 | FLJ10916 | NM_018271.2 | 661 | agctcgatgagccgatcaagact | 234496 | - | see also 17151 line | | |
| 38912 | LSS | NM_002340.2 | 1382 | tggactgcggctggatcgtttct | 234527 | - | see also 17152 line | | |
| 38913 | LSS | NM_002340.2 | 319 | cactggacgggtgattatggtgg | 234509 | - | see also 17152 line | | |
| 38914 | SDS-RS1 | NM_138432.2 | 935 | tgcaggtgtgcaagattcactct | 234537 | - | see also 17153 line | | |
| 38915 | SDS-RS1 | NM_138432.2 | 1043 | cagccttagcagccatctactca | 234538 | - | see also 17153 line | | |
| 38916 | SRR | NM_021947.1 | 426 | gcctacggagcgtcaattgtata | 234547 | - | see also 8210 line | | |
| 38917 | SRR | NM_021947.1 | 425 | agcctacggagcgtcaattgtat | 234546 | - | see also 8210 line | | |
| 38918 | FBXO18 | NM_032807.3 | 1664 | gacctcaacgctggtcaagtatg | 234588 | - | see also 9288 line | | |
| 38919 | FBXO18 | NM_032807.3 | 2123 | gactcatgacggctacttgaaac | 234599 | - | see also 9288 line | | |
| 38920 | FBXO18 | NM_032807.3 | 2272 | acccgcaccagcagatctatacc | 234603 | - | see also 9288 line | | |
| 38921 | LOC150678 | NM_138336.1 | 455 | cggaggcgctaatgtttaccaga | 234627 | - | see also 17156 line | | |
| 38922 | LOC150678 | NM_138336.1 | 631 | agcgaagacagcacaatatcagt | 234636 | - | see also 17156 line | | |
| 38923 | PEO1 | NM_021830.3 | 1744 | ggcacaagtccatcgtatctttc | 234660 | - | see also 8181 line | | |
| 38924 | PEO1 | NM_021830.3 | 1841 | tccagacctcaatcgtatcttga | 234662 | - | see also 8181 line | | |
| 38925 | FKBP11 | NM_016594.1 | 497 | cactaatccgagccaactactgg | 234675 | - | see also 17158 line | | |
| 38926 | FKBP11 | NM_016594.1 | 395 | agcgaagggcaatcattccttct | 234674 | - | see also 17158 line | | |
| 38927 | KIAA0073 | NM_015342.1 | 1741 | atggctaacgcgggatcaaatac | 234721 | - | see also 6383 line | | |
| 38928 | KIAA0073 | NM_015342.1 | 1159 | aaccggtgtgtgcggattttagg | 234712 | - | see also 6383 line | | |
| 38929 | PPIB | NM_000942.3 | 148 | gcctctccgaacgcaacatgaag | 234734 | - | see also 17161 line | | |
| 38930 | PPIB | NM_000942.3 | 264 | gtcaccgtcaaggtgtattttga | 234735 | - | see also 17161 line | | |
| 38931 | PPIB | NM_000942.3 | 285 | gacctacgaattggagatgaaga | 234737 | - | see also 17161 line | | |

EP 1 752 536 A1

Figure 46

| | | | | | | | |
|---|---|---|---|---|---|---|---|
| 38932 | PPIF | NM_005729.2 | 446 | tcctgacgagaacttacactga | 234741 | - | see also 17162 line |
| 38933 | PPIF | NM_005729.2 | 603 | atggacgtcgtgaagaaaataga | 234742 | - | see also 17162 line |
| 38934 | PPIF | NM_005729.2 | 609 | gtcgtgaagaaatagaatcttt | 234743 | - | see also 17162 line |
| 38935 | PPIL1 | NM_016059.3 | 347 | ggctcgtcgaggttactacaatg | 234746 | - | see also 17163 line |
| 38936 | PPIL1 | NM_016059.3 | 636 | gtgaatcgcgtgggaatggtaga | 234751 | - | see also 17163 line |
| 38937 | PPIL2 | NM_014337.2 | 687 | gacccgtcttattatctgaaaa | 234766 | - | see also 17164 line |
| 38938 | PPIL2 | NM_014337.2 | 685 | aggacccgtcttattatctgaaa | 234765 | - | see also 17164 line |
| 38939 | PPIL3 | NM_032472.3 | 676 | ccgacctttaatgatgtacaca | 234784 | - | see also 17165 line |
| 38940 | PPIL3 | NM_032472.3 | 590 | atgaaataccaccgtatttggaaa | 234781 | - | see also 17165 line |
| 38941 | SDCCAG10 | NM_005869.1 | 575 | aaggttacagcgggatacagtata | 234796 | - | see also 17166 line |
| 38942 | SDCCAG10 | NM_005869.1 | 530 | ctgggtcgagcagatgaacttaa | 234792 | - | see also 17166 line |
| 38943 | EBP | NM_006579.1 | 219 | ctctgtcacggggtcttagtcg | 234817 | - | see also 17167 line |
| 38944 | EBP | NM_006579.1 | 538 | cagcatccctccgcttcattct | 234820 | - | see also 17167 line |
| 38945 | DCI | NM_001919.1 | 275 | gagcttccgcgtgtcattctga | 234825 | - | see also 17168 line |
| 38946 | DCI | NM_001919.1 | 175 | gggtcgtgtgatgaaattcaag | 234823 | - | see also 17168 line |
| 38947 | JPH2 | NM_020433.3 | 1079 | ggcatgggctgggcatagagacc | 234827 | - | see also 17169 line |
| 38948 | GNPDA2 | NM_138335.1 | 179 | aactatgacttggctagtgaatg | 234835 | - | see also 9600 line |
| 38949 | GNPDA2 | NM_138335.1 | 698 | gtgccaactatggctctaactgt | 234850 | - | see also 9600 line |
| 38950 | RPIA | NM_144563.1 | 287 | aaccacgtgaggaataaccaagt | 234863 | - | see also 17170 line |
| 38951 | RPIA | NM_144563.1 | 858 | tggacacaggcctattcatcaac | 234872 | - | see also 17170 line |
| 38952 | PGDS | NM_014485.1 | 564 | ctgccgtgctaactggataaaa | 234892 | - | see also 17171 line |
| 38953 | PGDS | NM_014485.1 | 566 | gccgtgctaactggataaaacg | 234894 | - | see also 17171 line |
| 38954 | PGDS | NM_014485.1 | 393 | tgcaagacttggacacatattta | 234888 | - | see also 17171 line |
| 38955 | PTGIS | NM_000961.2 | 608 | cgctgccacgcacccatgaaagc | 234898 | - | see also 17172 line |
| 38956 | PTGIS | NM_000961.2 | 233 | agcacggtgacatctttactata | 234896 | - | see also 17172 line |
| 38957 | PMM1 | NM_002676.1 | 79 | gccggctgccagaaattgacc | 234907 | - | see also 17173 line |
| 38958 | PMM1 | NM_002676.1 | 744 | tgcagcggtgcgggagatttc | 234915 | - | see also 17173 line |
| 38959 | PMM2 | NM_000303.1 | 478 | ttctacgaactgataaaaaaga | 234935 | - | see also 17174 line |
| 38960 | PMM2 | NM_000303.1 | 475 | gagttctgcgaactcgataaaaa | 234934 | - | see also 17174 line |
| 38961 | PGM3 | NM_015599.1 | 933 | gagacaagatagcaacgttaatt | 234971 | - | see also 6499 line |
| 38962 | PGM3 | NM_015599.1 | 472 | gactgttctaggaggtcaattcc | 234950 | - | see also 6499 line |
| 38963 | FLJ32029 | NM_173582.2 | 601 | gacactcgcggtcaagtaactag | 235003 | - | see also 17176 line |
| 38964 | FLJ32029 | NM_173582.2 | 1840 | cgggacgttaccactggatatga | 235027 | - | see also 17176 line |
| 38965 | PGM2 | NM_018290.1 | 634 | ttgggacgattctttaattgata | 235048 | - | see also 17177 line |
| 38966 | PGM2 | NM_018290.1 | 1335 | gagtcagtcgccgctcataagt | 235066 | - | see also 17177 line |

Figure 46

| 38967 | PGM5 | NM_021965.2 | 1648 | ggcgaagacggatagttttgaat | 235096 | - | see also 17178 line |
|---|---|---|---|---|---|---|---|
| 38968 | PGM5 | NM_021965.2 | 1644 | gcgtggcgaagacggatagtttt | 235094 | - | see also 17178 line |
| 38969 | PGM5 | NM_021965.2 | 1646 | gtggcgaagacggatagttttga | 235095 | - | see also 17178 line |
| 38970 | MUT | NM_000255.1 | 265 | cccggaagggatctctataaaac | 235107 | - | see also 199 line |
| 38971 | MUT | NM_000255.1 | 1873 | tgctatcaagagggttcataaat | 235153 | - | see also 199 line |
| 38972 | GALE | NM_000403.2 | 781 | cacaggtgtccgggattacatcc | 235176 | - | see also 17180 line |
| 38973 | GALE | NM_000403.2 | 884 | ggcacgggcacaggctattcagt | 235177 | - | see also 17180 line |
| 38974 | MCEE | NM_032601.2 | 377 | atctgcatcgaggtggataatat | 235187 | - | see also 17181 line |
| 38975 | MCEE | NM_032601.2 | 323 | gacagtccaattgcaggttttct | 235185 | - | see also 17181 line |
| 38976 | MCEE | NM_032601.2 | 325 | cagtccaattgcaggttttctgc | 235186 | - | see also 17181 line |
| 38977 | ISYNA1 | NM_016368.3 | 107 | ggccatcgaggcgcaatacgagt | 235191 | - | see also 17182 line |
| 38978 | ISYNA1 | NM_016368.3 | 580 | ctgtttacatccccgaattcatc | 235192 | - | see also 17182 line |
| 38979 | EBRP | NM_032565.1 | 273 | atggcttgattgcttctttatgg | 235202 | - | see also 17183 line |
| 38980 | EBRP | NM_032565.1 | 225 | aaggccctttttgtctacttgtct | 235200 | - | see also 17183 line |
| 38981 | RENBP | NM_002910.4 | 1258 | tcccgagtacggggaatggtttg | 235209 | - | see also 17184 line |
| 38982 | RENBP | NM_002910.4 | 1005 | ttcgagcccacgtgattgacaag | 235207 | - | see also 17184 line |
| 38983 | RENBP | NM_002910.4 | 1316 | atcaagggaggtcctttcaaagg | 235210 | - | see also 17184 line |
| 38984 | TOPORS | NM_005802.2 | 350 | gccggcatcctccgagataatgg | 235212 | - | see also 4046 line |
| 38985 | TOPORS | NM_005802.2 | 1456 | aggtacacgttactatgtcttct | 235259 | - | see also 4046 line |
| 38986 | TXNDC5 | NM_022085.2 | 1292 | ggctaccccacgttattgctttt | 235328 | - | see also 17186 line |
| 38987 | TXNDC5 | NM_022085.2 | 717 | ttgcacaaggcgaccactttatc | 235320 | - | see also 17186 line |
| 38988 | 101F6 | NM_007022.3 | 662 | ccctggcgaagctcaagctatac | 235333 | - | see also 17187 line |
| 38989 | 101F6 | NM_007022.3 | 350 | ggcacccggtgcttatgtctttg | 235331 | - | see also 17187 line |
| 38990 | BRE | NM_004899.3 | 832 | ctgtacttgtcacctcgaattga | 235357 | - | see also 3403 line |
| 38991 | BRE | NM_004899.3 | 190 | gtggccttgaaccgaatatctcc | 235335 | - | see also 3403 line |
| 38992 | HSPC155 | NM_016406.1 | 423 | aggaactcggtggtttggaaaat | 235376 | - | see also 17189 line |
| 38993 | HSPC155 | NM_016406.1 | 422 | aaggaactcggtggtttggaaaa | 235375 | - | see also 17189 line |
| 38994 | HSPC155 | NM_016406.1 | 591 | gacggatcatttcaaacctttgt | 235381 | - | see also 17189 line |
| 38995 | MGC42638 | NM_178567.2 | 574 | cacccaagatcaaatttaccaac | 235382 | - | - | - | - |
| 38996 | NEDD8 | NM_006156.1 | 263 | cagcagctgattacaagatttta | 235386 | - | see also 17190 line |
| 38997 | NEDD8 | NM_006156.1 | 123 | gaccggaaaggagattgagattg | 235383 | - | see also 17190 line |
| 38998 | NEDD8 | NM_006156.1 | 264 | agcagctgattacaagattttag | 235387 | - | see also 17190 line |
| 38999 | UBE1C | NM_003968.3 | 1268 | gtcggtaacctctattgaagaac | 235434 | - | see also 2703 line |
| 39000 | UBE1C | NM_003968.3 | 1057 | atgatgtagatgggctgtataca | 235425 | - | see also 2703 line |
| 39001 | UBE4A | NM_004788.2 | 541 | ctcttcgctcgcttattacttca | 235458 | - | see also 3309 line |
| 39002 | UBE4A | NM_004788.2 | 2381 | accgccgtcccatgtatcctatc | 235514 | - | see also 3309 line |

Figure 46

| 39003 | UBE4A | NM_004788.2 | 544 | ttcgctcgcttattacttcaaga | 235459 | - | see also 3309 line |
|---|---|---|---|---|---|---|---|
| 39004 | SAE1 | NM_005500.1 | 750 | cacgacctccgactactttctcc | 235550 | - | see also 17193 line |
| 39005 | SAE1 | NM_005500.1 | 275 | gagctcagttcttgattcgtact | 235543 | - | see also 17193 line |
| 39006 | APG7L | NM_006395.1 | 1844 | agcagtgacgatcggatgaatga | 235608 | - | see also 17194 line |
| 39007 | APG7L | NM_006395.1 | 1782 | tggccgtggaattgatggtatct | 235605 | - | see also 17194 line |
| 39008 | UBA2 | NM_005499.1 | 275 | tacccgaaagctaatatcgttgc | 235626 | - | see also 3839 line |
| 39009 | UBA2 | NM_005499.1 | 159 | acctgattgatctggatactatt | 235622 | - | see also 3839 line |
| 39010 | UBA2 | NM_005499.1 | 267 | tgcagttttacccgaaagctaat | 235625 | - | see also 3839 line |
| 39011 | ABHD2 | NM_007011.4 | 1293 | cagcctgggtggtaacattgtgt | 235711 | - | see also 17196 line |
| 39012 | ABHD2 | NM_007011.4 | 1112 | tcgttgactacgcccagaaaaat | 235706 | - | see also 17196 line |
| 39013 | ABHD3 | NM_138340.3 | 941 | aagcaccgacatatgtttgtaaa | 235745 | - | see also 9602 line |
| 39014 | ACP33 | NM_016630.2 | 817 | ttcgggacatacctgtaactatt | 235774 | - | see also 17198 line |
| 39015 | ACP33 | NM_016630.2 | 818 | tcgggacatacctgtaactatta | 235775 | - | see also 17198 line |
| 39016 | APPBP1 | NM_003905.2 | 1241 | gtggtaagatgtcgatccttagc | 235812 | - | see also 2661 line |
| 39017 | BAT5 | NM_021160.1 | 156 | ctcctgggatacgtactatcagc | 235828 | - | see also 8113 line |
| 39018 | BG1 | NM_015162.3 | 2045 | ggggatccggagggtcaacatga | 235859 | - | see also 17201 line |
| 39019 | BG1 | NM_015162.3 | 365 | cactgtgcatcggatgttctacg | 235843 | - | see also 17201 line |
| 39020 | BGR | NM_030924.2 | 1857 | atcagtaacgccatgttagtagg | 235892 | - | see also 17202 line |
| 39021 | BGR | NM_030924.2 | 1526 | cacgatatccaaccagaataact | 235884 | - | see also 17202 line |
| 39022 | BGR | NM_030924.2 | 1347 | agctaccgcatggctaagactct | 235880 | - | see also 17202 line |
| 39023 | BUCS1 | NM_052956.1 | 663 | ttccgatcgctggttaaatcagc | 235916 | - | see also 17203 line |
| 39024 | BUCS1 | NM_052956.1 | 1011 | tgggggggtatcatctatatatcg | 235928 | - | see also 17203 line |
| 39025 | C12orf5 | NM_020375.1 | 657 | gtcacggtgcttacatgagaagt | 235947 | - | see also 17204 line |
| 39026 | C12orf5 | NM_020375.1 | 617 | agcggtattccaggattagcagc | 235946 | - | see also 17204 line |
| 39027 | C12orf5 | NM_020375.1 | 614 | gacagcggtattccaggattagc | 235945 | - | see also 17204 line |
| 39028 | C20orf135 | NM_080622.1 | 879 | caccgtgcgcgagcacttcaacc | 235955 | - | see also 17205 line |
| 39029 | C20orf135 | NM_080622.1 | 447 | cggcaacgagatcgacactatgt | 235953 | - | see also 17205 line |
| 39030 | C20orf22 | NM_015600.2 | 868 | cactggcgtggcgacaaatctgg | 235967 | - | see also 6501 line |
| 39031 | C20orf22 | NM_015600.2 | 866 | ggcactggcgtggcgacaaatct | 235966 | - | see also 6501 line |
| 39032 | C20orf22 | NM_015600.2 | 985 | ttcagtgatatatcgatacttcc | 235975 | - | see also 6501 line |
| 39033 | C21orf106 | NM_015151.1 | 2155 | gtcgatgaacggtctaagttatg | 236007 | - | see also 17207 line |
| 39034 | C21orf106 | NM_015151.1 | 3511 | ccccgatgtcctcgcatacttgg | 236018 | - | see also 17207 line |
| 39035 | C5orf4 | NM_016348.1 | 1268 | tcctgattgcacctaacaatttg | 236026 | - | see also 17208 line |
| 39036 | C5orf4 | NM_016348.1 | 1296 | ttcggccacacgccctaatgatg | 236027 | - | see also 17208 line |
| 39037 | CGI-111 | NM_016048.1 | 692 | ccgaggagtcgaggttcacattg | 236056 | - | see also 17209 line |
| 39038 | CGI-111 | NM_016048.1 | 619 | gagtcaggagtgttgtattattt | 236053 | - | see also 17209 line |
| 39039 | CGI-67 | NM_016014.1 | 637 | gactaggatcacggattaattgt | 236071 | - | see also 6615 line |

Figure 46

| | | | | | | | |
|---|---|---|---|---|---|---|---|
| 39040 | CGI-67 | NM_016014.1 | 554 | ttgttcaccaatgcgaaataca | 236067 | - | see also 6615 line |
| 39041 | CTMP | NM_053055.2 | 136 | cccgagctgaggtcatttcttc | 236083 | - | see also 17211 line |
| 39042 | CTMP | NM_053055.2 | 291 | acctactgaatggattcaagact | 236085 | - | see also 17211 line |
| 39043 | DKFZP434 P1750 | NM_015527.2 | 1802 | cccggcaggacgcttacttctga | 236096 | - | see also 6448 line |
| 39044 | DKFZP566J 2046 | NM_031208.1 | 588 | ggggagttggaccggttaaagaaa | 236099 | - | see also 17212 line |
| 39045 | DKFZP566J 2046 | NM_031208.1 | 590 | gagttggaccggttaaagaaaac | 236100 | - | see also 17212 line |
| 39046 | ECHDC1 | NM_018479.1 | 797 | caaccggctagttgaaataatcg | 236118 | - | see also 17213 line |
| 39047 | ECHDC1 | NM_018479.1 | 795 | accaccggctagttgaaataat | 236117 | - | see also 17213 line |
| 39048 | ECHDC1 | NM_018479.1 | 970 | aagggccaccggaagtaattaga | 236124 | - | see also 17213 line |
| 39049 | ECHDC3 | NM_024693.2 | 727 | agtcgggctctctgttctacc | 236135 | - | see also 17214 line |
| 39050 | ECHDC3 | NM_024693.2 | 455 | cagcaacgatctgaaagtcatta | 236132 | - | see also 17214 line |
| 39051 | FA2H | NM_024306.2 | 657 | tccgactcttcacgtcatttaca | 236139 | - | see also 17215 line |
| 39052 | FA2H | NM_024306.2 | 656 | gtccgactcttcacgtcatttac | 236138 | - | see also 17215 line |
| 39053 | FA2H | NM_024306.2 | 662 | ctcttcacgtcatttacaacaga | 236140 | - | see also 17215 line |
| 39054 | FHL5 | NM_020482.3 | 670 | ctgtgcacggagtgctattctaa | 236153 | - | see also 17216 line |
| 39055 | FHL5 | NM_020482.3 | 672 | gtgcacggagtgctattctaacg | 236154 | - | see also 17216 line |
| 39056 | FLJ10925 | NM_018275.3 | 192 | gtgcgactactcgatgtacttcc | 236165 | - | see also 7361 line |
| 39057 | FLJ10925 | NM_018275.3 | 642 | caacgtttcactggatagactgc | 236167 | - | see also 7361 line |
| 39058 | FLJ10948 | NM_018281.1 | 672 | gccgtgcggctgggccaaagtagc | 236178 | - | see also 17217 line |
| 39059 | FLJ10948 | NM_018281.1 | 810 | aagcggactcccaaatttgttgg | 236180 | - | see also 17217 line |
| 39060 | FLJ12377 | NM_024989.1 | 297 | tcccgcatagagttgtatcttt | 236189 | - | see also 17218 line |
| 39061 | FLJ12377 | NM_024989.1 | 298 | cccgcatatgagttgtatcttta | 236190 | - | see also 17218 line |
| 39062 | FLJ14906 | NM_032859.1 | 416 | ttctttccgatgcgttacttcc | 236264 | - | see also 17219 line |
| 39063 | FLJ14906 | NM_032859.1 | 421 | tccgatgcgttacttccttttat | 236265 | - | see also 17219 line |
| 39064 | FLJ20581 | NM_017888.2 | 1338 | acccttacgatgtgcagattgtgg | 236284 | - | see also 17220 line |
| 39065 | FLJ20581 | NM_017888.2 | 815 | accccgctgccatctactttacc | 236283 | - | see also 17220 line |
| 39066 | FLJ20581 | NM_017888.2 | 814 | gaccccgctggccatctacttac | 236282 | - | see also 17220 line |
| 39067 | FLJ20920 | NM_025149.2 | 1553 | gccgctctaaggatatgatcatc | 236297 | - | see also 17221 line |
| 39068 | FLJ20920 | NM_025149.2 | 1779 | tccgaagtacatcgtgtttgtca | 236302 | - | see also 17221 line |
| 39069 | FLJ21820 | NM_021925.1 | 631 | accgtgtcctgagacaatcaagt | 236326 | - | see also 17222 line |
| 39070 | FLJ21820 | NM_021925.1 | 850 | ttggtgtccaaaagagtactatg | 236334 | - | see also 17222 line |
| 39071 | FLJ21963 | NM_024560.2 | 437 | gccgttgatcgtcatattgaaaa | 236348 | - | see also 8643 line |
| 39072 | FLJ21963 | NM_024560.2 | 1749 | ctcgagtggatgatgtaataat | 236387 | - | see also 8643 line |
| 39073 | FLJ21963 | NM_024560.2 | 436 | tgccgttgatcgtcatattgaaa | 236347 | - | see also 8643 line |

Figure 46

| | | | | | | | | | |
|---|---|---|---|---|---|---|---|---|---|
| 39074 | FLJ23469 | NM_024710.1 | 480 | atcttggacccccgttcttatcc | 236398 | - | - | - | - |
| 39075 | FLJ23469 | NM_024710.1 | 698 | tgccgtccacccccagttcaagg | 236399 | - | see also 39074 line | | |
| 39076 | FLJ25449 | NM_144714.1 | 368 | accacattaccctgttccatatg | 236401 | - | see also 17224 line | | |
| 39077 | FLJ25449 | NM_144714.1 | 1123 | tgcagttacttgtatattggaag | 236423 | - | see also 17224 line | | |
| 39078 | FLJ32332 | NM_144641.1 | 708 | cagcagctggcctttgtctatcc | 236427 | - | see also 17225 line | | |
| 39079 | FLJ37964 | NM_182578.1 | 329 | tgctgagcctgtaccaagaattt | 236429 | - | see also 17226 line | | |
| 39080 | FLJ37964 | NM_182578.1 | 761 | aggaccagaagctttacatgtcc | 236433 | - | see also 17226 line | | |
| 39081 | FLJ40125 | NM_178494.2 | 555 | cgcgagcgttgggcgactttacc | 236435 | - | see also 17227 line | | |
| 39082 | FLJ40125 | NM_178494.2 | 559 | agcgttgggcgactttacctaca | 236437 | - | see also 17227 line | | |
| 39083 | FLJ40773 | NM_152666.1 | 842 | gagccgaggtgacgtacatgaac | 236450 | - | see also 17228 line | | |
| 39084 | FLJ40773 | NM_152666.1 | 1397 | ctgatccccttacgtttaacttt | 236473 | - | see also 17228 line | | |
| 39085 | GAF1 | NM_015470.1 | 1783 | gccaaggacccgactcagaaacc | 236493 | - | see also 17229 line | | |
| 39086 | GAF1 | NM_015470.1 | 1742 | gggaggaaaaggccaagagtagc | 236492 | - | see also 17229 line | | |
| 39087 | GENX-3414 | NM_003943.1 | 167 | ctggccggagcacttttcgtttg | 236496 | - | see also 17230 line | | |
| 39088 | GENX-3414 | NM_003943.1 | 904 | agggtctcagcaagttagtgtca | 236507 | - | see also 17230 line | | |
| 39089 | GPHN | NM_020806.3 | 2129 | accatcggtgtatactaacttgg | 236570 | - | see also 7978 line | | |
| 39090 | GPHN | NM_020806.3 | 1731 | cacgatcaacttgggtattgtag | 236555 | - | see also 7978 line | | |
| 39091 | GPHN | NM_020806.3 | 1328 | gacaagtcatgcgggttacaaca | 236539 | - | see also 7978 line | | |
| 39092 | GPR56 | NM_005682.3 | 234 | aggggccacagggaagactttcg | 236573 | - | see also 17232 line | | |
| 39093 | GPR56 | NM_005682.3 | 1193 | atgtgtgttctgggttgaagacc | 236576 | - | see also 17232 line | | |
| 39094 | HSRTSBETA | NM_017512.1 | 1168 | atcccggctactcaacagaaatg | 236596 | - | see also 17233 line | | |
| 39095 | HSRTSBETA | NM_017512.1 | 1169 | tcccggctactcaacagaaatga | 236597 | - | see also 17233 line | | |
| 39096 | KIAA1463 | NM_020849.1 | 1351 | ctgcccactgtccgaatgattgt | 236622 | - | see also 7993 line | | |
| 39097 | KIAA1463 | NM_020849.1 | 1285 | tgcctgtatgcgggctgtatacc | 236620 | - | see also 7993 line | | |
| 39098 | KIAA1959 | NM_032873.2 | 1057 | ctcgggatatccgatttgctaac | 236657 | - | see also 9329 line | | |
| 39099 | KIAA1959 | NM_032873.2 | 1608 | ttccgagattacgagaaagatgc | 236676 | - | see also 9329 line | | |
| 39100 | LIPH | NM_139248.1 | 535 | tacatgatcggagtaagtctagg | 236699 | - | see also 17236 line | | |
| 39101 | LIPH | NM_139248.1 | 936 | cagctgcggcacgtcacaaaaag | 236707 | - | see also 17236 line | | |
| 39102 | LOC122618 | NM_138790.1 | 1430 | ctccaactggtcggaggattact | 236732 | - | see also 17237 line | | |
| 39103 | LOC122618 | NM_138790.1 | 829 | agcttggcgctgtcatctataac | 236725 | - | see also 17237 line | | |
| 39104 | LOC197322 | NM_174917.1 | 565 | tccttcctatgcgctaacgatgc | 236734 | - | see also 17238 line | | |

EP 1 752 536 A1

Figure 46

| | | | | | | | |
|---|---|---|---|---|---|---|---|
| 39105 | LOC197322 | NM_174917.1 | 466 | cacacgtacagggagctttattc | 236733 | - | see also 17238 line |
| 39106 | LOC197322 | NM_174917.1 | 1200 | gccgcacgcccaggatttcttgc | 236738 | - | see also 17238 line |
| 39107 | LOC201164 | NM_178836.2 | 606 | gacgagtacgtgcggcttttct | 236745 | - | - |
| 39108 | LOC201164 | NM_178836.2 | 660 | aaccctacaaagtataccttttt | 236747 | - | see also 39107 line |
| 39109 | LOC201164 | NM_178836.2 | 662 | ccctacaaagtatacctttttcc | 236749 | - | see also 39107 line |
| 39110 | LOC93081 | NM_138779.2 | 764 | gacggtcgacaaatgatgttttc | 236764 | - | see also 17239 line |
| 39111 | LOC93081 | NM_138779.2 | 757 | gtgaaaggacggtcgacaaatga | 236762 | - | see also 17239 line |
| 39112 | LYPLAL1 | NM_138794.1 | 609 | gtgaccacgaagtttcatagttt | 236785 | - | see also 17240 line |
| 39113 | LYPLAL1 | NM_138794.1 | 227 | aggaatctccaatgtatggtttg | 236771 | - | see also 17240 line |
| 39114 | MCOLN1 | NM_020533.1 | 244 | accttcgccgtcgtctcaaatac | 236792 | - | see also 17241 line |
| 39115 | MCOLN1 | NM_020533.1 | 247 | ttcgccgtcgtctcaaatacttt | 236793 | - | see also 17241 line |
| 39116 | MCOLN1 | NM_020533.1 | 248 | tcgccgtcgtctcaaatactttt | 236794 | - | see also 17241 line |
| 39117 | MGC15429 | NM_032750.1 | 725 | gactccagctctgattgtatatg | 236816 | - | see also 17242 line |
| 39118 | MGC15429 | NM_032750.1 | 677 | ccccatctgcactgacaaaatca | 236814 | - | see also 17242 line |
| 39119 | MGC19531 | NM_005167.4 | 2001 | ttccctgaggtacgagtgtatga | 236824 | - | see also 3635 line |
| 39120 | MGC19531 | NM_005167.4 | 2003 | ccctgaggtacgagtgtatgacc | 236825 | - | see also 3635 line |
| 39121 | MGC19531 | NM_005167.4 | 2132 | gtcggcctatgagcctaatgacc | 236828 | - | see also 3635 line |
| 39122 | MGC5352 | NM_138575.1 | 757 | ctgttgtccgctggggattttgt | 236837 | - | see also 17244 line |
| 39123 | MGC5352 | NM_138575.1 | 599 | agccggaagctgtgcagtattac | 236836 | - | see also 17244 line |
| 39124 | MOCS2 | NM_004531.3 | 618 | tggtgcaatatccctatttgtag | 236841 | - | see also 3087 line |
| 39125 | MOCS2 | NM_004531.3 | 608 | ctccgctctgtggtgcaatatcc | 236840 | - | see also 3087 line |
| 39126 | NDRG1 | NM_006096.2 | 567 | gcctacatcctaactcgatttgc | 236862 | - | see also 17246 line |
| 39127 | NDRG1 | NM_006096.2 | 559 | gagcaggcgcctacatcctaact | 236861 | - | see also 17246 line |
| 39128 | NDRG3 | NM_022477.2 | 165 | gtgtggtccacgtcactataaga | 236868 | - | see also 8401 line |
| 39129 | NDRG3 | NM_022477.2 | 919 | gggactgccccaggtagttcagc | 236884 | - | see also 8401 line |
| 39130 | NLGN4 | NM_020742.2 | 506 | caccccggtctgcgtcatgttaa | 236887 | - | - | autism |
| 39131 | NLGN4 | NM_020742.2 | 508 | ccccggtctgcgtcatgttaaac | 236888 | - | see also 39130 line |
| 39132 | NRXN3 | NM_004796.2 | 3550 | tggacgagacgcggaactacatc | 236934 | - | see also 3329 line |

Figure 46

| | | | | | | | |
|---|---|---|---|---|---|---|---|
| 39133 | NRXN3 | NM_004796.2 | 3006 | ttcaacactcaggcgcaaatagc | 236922 | - | see also 3329 line |
| 39134 | OAZIN | NM_015878.3 | 1432 | ttcacgggaactgaatttcaatt | 236971 | - | see also 6548 line |
| 39135 | PGS1 | NM_024419.2 | 1426 | cgctgattggctcctcctaattt | 237000 | - | see also 17249 line |
| 39136 | PGS1 | NM_024419.2 | 1055 | gcccttcgagattcaaatcgatg | 236996 | - | see also 17249 line |
| 39137 | PIGB | NM_004855.3 | 330 | accgctcccacggcaagataaag | 237003 | - | see also 17250 line |
| 39138 | PIGB | NM_004855.3 | 1261 | gagataccggatactttggtga | 237034 | - | see also 17250 line |
| 39139 | PIGB | NM_004855.3 | 1832 | cacttgccagaggtcgaattgg | 237045 | - | see also 17250 line |
| 39140 | PLEKHE1 | NM_194449.1 | 564 | cagtggacctcgtgttgtagc | 237062 | - | see also 10309 line |
| 39141 | PLEKHE1 | NM_194449.1 | 1077 | tggatctaaggttgaacgtaatt | 237074 | - | see also 10309 line |
| 39142 | PME-1 | NM_016147.1 | 688 | ttcttacgggtgtcctaaaac | 237142 | - | see also 6695 line |
| 39143 | PME-1 | NM_016147.1 | 1004 | tccgaggcttatccaatctctt | 237152 | - | see also 6695 line |
| 39144 | PNPLA1 | NM_173676.1 | 512 | tccacgacttcgccatgttcaac | 237161 | - | see also 17253 line |
| 39145 | PNPLA1 | NM_173676.1 | 1336 | accgtcagcaagccttatgttaac | 237174 | - | see also 17253 line |
| 39146 | PNPLA1 | NM_173676.1 | 627 | agggtacgaggatgcagttttgt | 237165 | - | see also 17253 line |
| 39147 | PPM1L | NM_139245.1 | 581 | tgggggattatccgctgaaaaat | 237184 | - | see also 9682 line |
| 39148 | PPM1L | NM_139245.1 | 583 | ggggattatccgctgaaaaatct | 237186 | - | see also 9682 line |
| 39149 | SAH | NM_005622.1 | 185 | cagggcctagcaattttggttc | 237200 | - | see also 3901 line |
| 39150 | SAH | NM_005622.1 | 1418 | taccgcttaccccatataaact | 237238 | - | see also 3901 line |
| 39151 | SERAC1 | NM_032861.2 | 1005 | gaggctgacgacttcacagg | 237261 | - | see also 17256 line |
| 39152 | SERAC1 | NM_032861.2 | 2005 | ttgtaccaggctactttacaatt | 237289 | - | see also 17256 line |
| 39153 | SLC27A3 | NM_024330.1 | 1575 | ttctccttgattcgctatgatgt | 237302 | - | see also 17257 line |
| 39154 | SLC27A3 | NM_024330.1 | 1577 | ctccttgattcgctatgatgtca | 237303 | - | see also 17257 line |
| 39155 | SLC27A4 | NM_005094.2 | 2121 | gacccgctgttctatcctagatgc | 237326 | - | see also 17258 line |
| 39156 | SLC27A4 | NM_005094.2 | 1234 | ccggttctgggacgattgtatc | 237317 | - | see also 17258 line |
| 39157 | SLC27A4 | NM_005094.2 | 426 | accatcaggcgcgatatcttgg | 237310 | - | see also 17258 line |
| 39158 | TA-PP2C | NM_139283.1 | 522 | gcggacgtgtgaacgtttagtaa | 237331 | - | see also 9687 line |
| 39159 | TA-PP2C | NM_139283.1 | 549 | aggacgttcgtacctagtaatc | 237335 | - | see also 9687 line |
| 39160 | TA-WDRP | NM_139281.1 | 1548 | atctacaataggcgcttacttc | 237392 | - | see also 9686 line |
| 39161 | TA-WDRP | NM_139281.1 | 1119 | agcaattgccggactgacatttc | 237370 | - | see also 9686 line |
| 39162 | TA-WDRP | NM_139281.1 | 307 | cgcacgccagcgcgcgcttttgc | 237349 | - | see also 9686 line |
| 39163 | TTS-2.2 | NM_020376.2 | 565 | gtcagacggcgagaatgtcatta | 237434 | - | see also 17261 line |
| 39164 | TTS-2.2 | NM_020376.2 | 720 | tgccactctatgagcttaagaac | 237437 | - | see also 17261 line |
| 39165 | UBASH3A | NM_018961.1 | 1766 | gacacgggtgtcatcctaattgt | 237463 | - | see also 17262 line |
| 39166 | UBASH3A | NM_018961.1 | 1768 | cacgggtgtcatcctaattgtga | 237464 | - | see also 17262 line |
| 39167 | UBASH3A | NM_018961.1 | 1726 | cacggcgagcatggtgcaaatcg | 237461 | - | see also 17262 line |
| 39168 | UTP2 | NM_173355.2 | 814 | ccctcgttgacatacaatgtgt | 237488 | - | see also 10065 line |
| 39169 | UTP2 | NM_173355.2 | 812 | aaccctcgttgacatacaatgt | 237487 | - | see also 10065 line |

Figure 46

| 39170 | UPP2 | NM_173355.2 | 599 | gtgctgcgatgtcaccattatta | 237481 | - | see also 10065 line | | |
|---|---|---|---|---|---|---|---|---|---|
| 39171 | UXS1 | NM_025076.1 | 1151 | tacgtcagcgatctagtgaatgg | 237501 | - | see also 17264 line | | |
| 39172 | UXS1 | NM_025076.1 | 1431 | aactcgagtaccaggcaaataat | 237507 | - | see also 17264 line | | |
| 39173 | HTR1B | NM_000863.1 | 460 | gccgtggagtactcagctaaaag | 237516 | - | see also 17265 line | | |
| 39174 | HTR1B | NM_000863.1 | 792 | gtcctcggtcacctctattaact | 237523 | - | see also 17265 line | | |
| 39175 | HTR1B | NM_000863.1 | 702 | agcccgctcccggattttgaaac | 237520 | - | see also 17265 line | | |
| 39176 | HTR1D | NM_000864.3 | 1364 | ccctggaacgcaagaggatttct | 237530 | - | see also 17266 line | | |
| 39177 | HTR1D | NM_000864.3 | 686 | tactcaccaccatcttactcacc | 237528 | - | see also 17266 line | | |
| 39178 | HTR1E | NM_000865.1 | 1588 | ctctgctctatacgagtttttaat | 237551 | - | see also 17267 line | | |
| 39179 | HTR1E | NM_000865.1 | 1590 | ctgctctatacgagttttaatga | 237552 | - | see also 17267 line | | |
| 39180 | HTR1E | NM_000865.1 | 1362 | ccccccttcgacaatgatctaga | 237544 | - | see also 17267 line | | |
| 39181 | HTR1F | NM_000866.1 | 644 | gacaagcaagtaggattgcaaag | 237574 | - | see also 561 line | | |
| 39182 | HTR1F | NM_000866.1 | 723 | ttccacatcctatgtactagaaa | 237576 | - | see also 561 line | | |
| 39183 | HTR2A | NM_000621.1 | 1520 | gacaatagcgacggagtgaatga | 237630 | - | see also 17269 line | | |
| 39184 | HTR2A | NM_000621.1 | 184 | aactacgaactccctaatgcaat | 237591 | - | see also 17269 line | | |
| 39185 | HTR2C | NM_000868.1 | 2077 | tggttagcgaaaggattagcagt | 237683 | - | see also 563 line | | |
| 39186 | HTR2C | NM_000868.1 | 2074 | gtgtggttagcgaaaggattagc | 237682 | - | see also 563 line | | |
| 39187 | HTR4 | NM_000870.2 | 541 | cccctctgcgcatcgcattaatg | 237690 | - | see also 565 line | | |
| 39188 | HTR4 | NM_000870.2 | 542 | ccctctgcgcatcgcattaatgc | 237691 | - | see also 565 line | | |
| 39189 | HTR5A | NM_024012.1 | 108 | ctcggtcttcggagtgcttattc | 237709 | - | see also 17272 line | | |
| 39190 | HTR5A | NM_024012.1 | 1039 | agcgccttcaagaacttctttc | 237711 | - | see also 17272 line | | |
| 39191 | HTR7 | NM_000872.3 | 947 | aggactttggctatacgatttac | 237718 | - | see also 17273 line | | |
| 39192 | HTR7 | NM_000872.3 | 944 | gccaggactttggctatacgatt | 237716 | - | see also 17273 line | | |
| 39193 | ADRA2A | NM_000681.2 | 2090 | cacgcacgctcttcaaattcttc | 237730 | - | see also 17274 line | | |
| 39194 | ADRA2A | NM_000681.2 | 2088 | gccacgcacgctcttcaaattct | 237729 | - | see also 17274 line | | |
| 39195 | ADRA2B | NM_000682.3 | 1036 | ggcaggggcgtgggtgctatagg | 237733 | - | see also 17275 line | | |
| 39196 | ADRA2B | NM_000682.3 | 522 | ggcctccagcatcggatctttct | 237731 | - | see also 17275 line | | |
| 39197 | ADRA2C | NM_000683.2 | 1277 | cgctcgatgtgctgttttgcacc | 237735 | - | gpcr、receptor_acitivity、kinase_related、signal_transduction | alpha2-adrenergic receptor | - |
| 39198 | ADRA1A | NM_000680.1 | 1562 | cgcatccccgtgggatcaagaga | 237741 | - | see also 17276 line | | |
| 39199 | ADRA1A | NM_000680.1 | 490 | ggcaccggtgaacatttccaagg | 237737 | - | see also 17276 line | | |
| 39200 | ADRA1B | NM_000679.2 | 813 | ccctctggcggtcattctagtca | 237750 | - | see also 426 line | | |
| 39201 | ADRA1B | NM_000679.2 | 819 | ggcggtcattctagtcatgtact | 237751 | - | see also 426 line | | |
| 39202 | ADRA1D | NM_000678.2 | 1754 | acctacgggagaccgatatttaa | 237764 | - | see also 17278 line | | |
| 39203 | ADRA1D | NM_000678.2 | 1056 | ctccgtgcgcctgctcaagttct | 237759 | - | see also 17278 line | | |
| 39204 | ADRA1D | NM_000678.2 | 1191 | gccatcggagggcgtcttcaagg | 237761 | - | see also 17278 line | | |

Figure 46

| 39205 | ADRB2 | NM_000024.3 | 1400 | tgcctagcgataacattgattca | 237776 | - | see also 17279 line | | |
|---|---|---|---|---|---|---|---|---|---|
| 39206 | ADRB2 | NM_000024.3 | 1405 | agcgataacattgattcacaagg | 237777 | - | see also 17279 line | | |
| 39207 | ADRB2 | NM_000024.3 | 1121 | acctcatccgtaaggaagtttac | 237771 | - | see also 17279 line | | |
| 39208 | ADRB3 | NM_000025.1 | 1185 | gccctgaactggctaggttatgc | 237782 | - | gpcr、receptor_acitivity | beta3-adrenergic receptor | diabetes mellitus、diabetes、obesity |
| 39209 | HRH1 | NM_000861.2 | 409 | tgccgtcgtcatgcctatgaaca | 237784 | - | see also 17280 line | | |
| 39210 | HRH1 | NM_000861.2 | 846 | accgggagctcatcaataggtcc | 237790 | - | see also 17280 line | | |
| 39211 | HRH1 | NM_000861.2 | 1363 | ctcgcattcaagacagtatgtat | 237798 | - | see also 17280 line | | |
| 39212 | HRH2 | NM_022304.1 | 671 | tccgcaacctgaccaattgtttc | 237803 | - | see also 17281 line | | |
| 39213 | HRH2 | NM_022304.1 | 1450 | aaccgcaactcccacaaaacttc | 237812 | - | see also 17281 line | | |
| 39214 | HRH3 | NM_007232.1 | 1464 | tccctgactactggtacgaaacc | 237818 | - | see also 17282 line | | |
| 39215 | HRH3 | NM_007232.1 | 864 | atgccgagttcttctacaactgg | 237816 | - | see also 17282 line | | |
| 39216 | HRH4 | NM_021624.2 | 137 | agcactcgtgttactttagcatt | 237821 | - | see also 17283 line | | |
| 39217 | HRH4 | NM_021624.2 | 1178 | ttgtgtcacaagcgctttcaaaa | 237847 | - | see also 17283 line | | |
| 39218 | HRH4 | NM_021624.2 | 132 | cactaagcactcgtgttacttta | 237820 | - | see also 17283 line | | |
| 39219 | HTR6 | NM_000871.1 | 1064 | gccctcgggtgccatatgcttca | 237853 | - | see also 17284 line | | |
| 39220 | HTR6 | NM_000871.1 | 1063 | tgccctcgggtgccatatgcttc | 237852 | - | see also 17284 line | | |
| 39221 | DRD1 | NM_000794.2 | 1778 | tgccatagagacggtgagtatca | 237877 | - | see also 17285 line | | |
| 39222 | DRD1 | NM_000794.2 | 1787 | gacggtgagtatcaataacaatg | 237879 | - | see also 17285 line | | |
| 39223 | DRD2 | NM_000795.2 | 519 | cacggcgagcatcctgaacttgt | 237886 | - | see also 490 line | | |
| 39224 | DRD2 | NM_000795.2 | 521 | cggcgagcatcctgaacttgtgt | 237887 | - | see also 490 line | | |
| 39225 | DRD3 | NM_000796.2 | 790 | ttctgtttggctttaataccaca | 237903 | - | see also 491 line | | |
| 39226 | DRD3 | NM_000796.2 | 629 | tacagccagcatccttaatctct | 237901 | - | see also 491 line | | |
| 39227 | DRD5 | NM_000798.3 | 716 | atcgaacctacgccatctcttcc | 237918 | - | see also 17288 line | | |
| 39228 | DRD5 | NM_000798.3 | 300 | ggccgtgtcagacctttcgtgg | 237916 | - | see also 17288 line | | |
| 39229 | OR1A1 | NM_014565.1 | 775 | ttccgccctttgaccaattatag | 237927 | - | see also 17289 line | | |
| 39230 | OR1A1 | NM_014565.1 | 776 | tccgccctttgaccaattatagc | 237928 | - | see also 17289 line | | |
| 39231 | OR1A2 | NM_012352.1 | 756 | tacaacgatgggcatgtatttcc | 237941 | - | see also 17290 line | | |
| 39232 | OR1A2 | NM_012352.1 | 680 | ttccatctaccaagagtctattc | 237939 | - | see also 17290 line | | |
| 39233 | OR1A2 | NM_012352.1 | 602 | taggggtcggcgttttctctttg | 237937 | - | see also 17290 line | | |
| 39234 | OR1D2 | NM_002548.1 | 504 | ctgtgggtcacgaaaaatccact | 237950 | - | see also 17291 line | | |
| 39235 | OR1D2 | NM_002548.1 | 756 | tgggacactttgtatggtatacc | 237956 | - | see also 17291 line | | |
| 39236 | OR1D5 | NM_014566.1 | 645 | cacatcctatgtacgtattgtca | 331776 | - | see also 17292 line | | |
| 39237 | OR1D5 | NM_014566.1 | 642 | gaccacatcctatgtacgtattg | 331774 | - | see also 17292 line | | |
| 39238 | OR1D5 | NM_014566.1 | 643 | accacatcctatgtacgtattgt | 331775 | - | see also 17292 line | | |

Figure 46

| | | | | | | | | | |
|---|---|---|---|---|---|---|---|---|---|
| 39239 | OR1E1 | NM_003553.1 | 898 | gccctgagcagagtcattcatca | 237963 | - | receptor_acitivity、sig nal_transduction、gpcr | olfactory receptor | - |
| 39240 | OR1E1 | NM_003553.1 | 897 | agccctgagcagagtcattcatc | 237962 | - | see also 39239 line | | |
| 39241 | OR1E1 | NM_003553.1 | 234 | tcccaagttgttacagaacatgc | 237961 | - | see also 39239 line | | |
| 39242 | OR1G1 | NM_003555.1 | 282 | ctcagggtgtctactacagttgt | 237968 | - | see also 17293 line | | |
| 39243 | OR1G1 | NM_003555.1 | 277 | tcctactcagggtgtctactaca | 237967 | - | see also 17293 line | | |
| 39244 | OR2C1 | NM_012368.1 | 701 | ggcgaaaggcgttcaatacgtgc | 237984 | - | see also 17294 line | | |
| 39245 | OR2C1 | NM_012368.1 | 209 | accttgctttcgctactagttca | 237978 | - | see also 17294 line | | |
| 39246 | OR3A1 | NM_002550.1 | 633 | tacccccatggctctcattgtca | 237987 | - | see also 17295 line | | |
| 39247 | OR3A1 | NM_002550.1 | 791 | gactgggttcaaccaagctttca | 237988 | - | see also 17295 line | | |
| 39248 | OR51B4 | NM_033179.1 | 558 | tgctgatatcacgtttaatcaca | 238004 | - | see also 17296 line | | |
| 39249 | OR51B4 | NM_033179.1 | 840 | tcctccattcgtgaatcctatca | 238010 | - | see also 17296 line | | |
| 39250 | OR51B4 | NM_033179.1 | 839 | ttcctccattcgtgaatcctatc | 238009 | - | see also 17296 line | | |
| 39251 | OR51E2 | NM_030774.2 | 859 | gggcgtggacgtaatgttcatct | 238022 | - | see also 17297 line | | |
| 39252 | OR51E2 | NM_030774.2 | 684 | tggtccgcggatccctcttttt | 238018 | - | see also 17297 line | | |
| 39253 | OR7A5 | NM_017506.1 | 807 | atcacatggtgatatattttaca | 238029 | - | see also 17298 line | | |
| 39254 | OR7A5 | NM_017506.1 | 1147 | aacaatgaaagggcaattttca | 238030 | - | see also 17298 line | | |
| 39255 | OR7C1 | NM_198944.1 | 51 | ttcagcaacgtcagagattcagt | 238032 | - | see also 17299 line | | |
| 39256 | OR7C1 | NM_198944.1 | 498 | gtccttctgcaccgaaatggaaa | 238039 | - | see also 17299 line | | |
| 39257 | OR8B8 | NM_012378.1 | 187 | ctctataacttgtccttcataga | 238046 | - | see also 17300 line | | |
| 39258 | OR8B8 | NM_012378.1 | 16 | tcctccttcgtgacacagtttat | 238045 | - | see also 17300 line | | |
| 39259 | TAS2R1 | NM_019599.1 | 292 | tggctcggcgttttctattgtgc | 238061 | - | see also 17301 line | | |
| 39260 | TAS2R1 | NM_019599.1 | 607 | aggcacacccggcaaatgagaaa | 238071 | - | see also 17301 line | | |
| 39261 | TAS2R10 | NM_023921.1 | 225 | tgcctccggtaacctaattgaat | 238086 | - | see also 17302 line | | |
| 39262 | TAS2R10 | NM_023921.1 | 226 | gcctccggtaacctaattgaata | 238087 | - | see also 17302 line | | |
| 39263 | TAS2R10 | NM_023921.1 | 142 | accggcttagctatttcaagaat | 238080 | - | see also 17302 line | | |
| 39264 | TAS2R13 | NM_023920.1 | 532 | tcggtcaaattcactatgactat | 238132 | - | see also 17303 line | | |
| 39265 | TAS2R13 | NM_023920.1 | 66 | gagcaatggatttatagtactga | 238113 | - | see also 17303 line | | |
| 39266 | TAS2R14 | NM_023922.1 | 297 | agctacaggcctcggtactttt | 238142 | - | see also 17304 line | | |
| 39267 | TAS2R14 | NM_023922.1 | 434 | ttgcactgataaacatccatata | 238149 | - | see also 17304 line | | |
| 39268 | TAS2R16 | NM_016945.1 | 539 | ctcatacagttgcattggttatt | 238175 | - | see also 17305 line | | |
| 39269 | TAS2R16 | NM_016945.1 | 837 | cagccctacgttgaaaaggattc | 238181 | - | see also 17305 line | | |
| 39270 | TAS2R3 | NM_016943.1 | 378 | ttctagggtgatggtatggatgc | 238192 | - | see also 17306 line | | |
| 39271 | TAS2R3 | NM_016943.1 | 924 | tggatccaagggacccattttct | 238204 | - | see also 17306 line | | |
| 39272 | TAS2R4 | NM_016944.1 | 102 | ttgggtcaaaagccatagaatct | 238206 | - | see also 17307 line | | |
| 39273 | TAS2R4 | NM_016944.1 | 599 | tacactccttgaggagacatata | 238213 | - | see also 17307 line | | |

Figure 46

| | | | | | | | |
|---|---|---|---|---|---|---|---|
| 39274 | TAS2R5 | NM_018980.1 | 224 | gccgttggcttgctatcttagt | 238228 | - | see also 17308 line |
| 39275 | TAS2R5 | NM_018980.1 | 223 | agccgttggcttcgctatcttag | 238227 | - | see also 17308 line |
| 39276 | TAS2R7 | NM_023919.1 | 807 | tggttgagtccatagctctaatct | 238262 | - | see also 17309 line |
| 39277 | TAS2R7 | NM_023919.1 | 778 | atgccagagaacggaattagctgt | 238260 | - | see also 17309 line |
| 39278 | TAS2R8 | NM_023918.1 | 757 | ttgatgacctttagctatcttat | 238294 | - | see also 17310 line |
| 39279 | TAS2R8 | NM_023918.1 | 432 | cagccttatagcagcaatagtac | 238282 | - | see also 17310 line |
| 39280 | TAS2R9 | NM_023917.1 | 787 | gtgttgatgattggtgacatagt | 238315 | - | see also 17311 line |
| 39281 | TAS2R9 | NM_023917.1 | 793 | atgattggtgacatagtaacgt | 238317 | - | see also 17311 line |
| 39282 | ELOVL4 | NM_022726.2 | 247 | gccgggtagtgtcctaaacgtag | 238319 | - | see also 17312 line |
| 39283 | ELOVL4 | NM_022726.2 | 450 | tgcgtctagtgtcattatctat | 238328 | - | see also 17312 line |
| 39284 | OPN1SW | NM_001708.1 | 591 | accgcagcgagtcctatacgtgg | 238372 | - | see also 17313 line |
| 39285 | OPN1SW | NM_001708.1 | 471 | tggctactggaccattggtatt | 238370 | - | see also 17313 line |
| 39286 | OPN3 | NM_014322.1 | 909 | tggtcactcaacaatatctatt | 238389 | - | see also 17314 line |
| 39287 | OPN3 | NM_014322.1 | 327 | ccctcttcggggtcacctttacc | 238377 | - | see also 17314 line |
| 39288 | OPN4 | NM_033282.1 | 561 | gagacaggctgcgagttctatgc | 238401 | - | see also 17315 line |
| 39289 | OPN4 | NM_033282.1 | 527 | caccagtagcctctataagcagt | 238400 | - | see also 17315 line |
| 39290 | RDS | NM_000322.2 | 1057 | ctcttcgagtgaccattacaat | 238417 | - | see also 17316 line |
| 39291 | RDS | NM_000322.2 | 382 | agcgatgtgatgaataattctga | 238405 | - | see also 17316 line |
| 39292 | RHO | NM_000539.2 | 650 | ctgttgtggaatcgactactaca | 238424 | - | see also 406 line |
| 39293 | RHO | NM_000539.2 | 684 | gaggtcaacaacgagtctttgt | 238425 | - | see also 406 line |
| 39294 | ROM1 | NM_000327.2 | 640 | ggcgccacgggtacaaggattgg | 238431 | - | see also 17318 line |
| 39295 | ROM1 | NM_000327.2 | 645 | cacgggtacaaggattggtttgg | 238432 | - | see also 17318 line |
| 39296 | PTGFR2 | NM_000956.2 | 1018 | aacctcttcccgaaaggaaaat | 238449 | - | see also 17320 line |
| 39297 | PTGFR2 | NM_000956.2 | 986 | ttgcctttcaacgatttttgcata | 238445 | - | see also 17320 line |
| 39298 | PTGFR2 | NM_000956.2 | 987 | tgcctttcacgattttgcatat | 238446 | - | see also 17320 line |
| 39299 | PTGFR3 | NM_000957.2 | 896 | cgcataactgtggcgcaaccttc | 238455 | - | see also 17321 line |
| 39300 | PTGFR3 | NM_000957.2 | 651 | cgggctcctcctgtttcatcg | 238453 | - | see also 17321 line |
| 39301 | PTGFR4 | NM_000958.2 | 1450 | ccgctcgtgtgtgcgagtattcg | 238476 | - | see also 17322 line |
| 39302 | PTGFR4 | NM_000958.2 | 1004 | ctcacgctctttgcagtctatgc | 238474 | - | see also 17322 line |
| 39303 | PTGFR | NM_000959.2 | 654 | gccaattgagcggtgtattggagt | 238504 | - | see also 17323 line |
| 39304 | PTGFR | NM_000959.2 | 777 | atccttggacatcgagactataa | 238508 | - | see also 17323 line |
| 39305 | PTGDR | NM_000953.2 | 1018 | ctccgagccttgcgatttctatc | 238534 | - | see also 17324 line |
| 39306 | PTGDR | NM_000953.2 | 1019 | tccgagccttgcgatttctatct | 238535 | - | see also 17324 line |
| 39307 | TBXA2R | NM_001060.2 | 447 | gggcggtcgtcatgatcttcttcg | 238540 | - | see also 17325 line |
| 39308 | EDG1 | NM_001400.2 | 1295 | gccgcagcaaatcggacaattcc | 238556 | - | see also 17326 line |
| 39309 | EDG1 | NM_001400.2 | 695 | tccacaacgggagcaataacttc | 238549 | - | see also 17326 line |
| 39310 | EDG2 | NM_001401.2 | 871 | gccatcgttatgggtgctatacc | 238566 | - | see also 974 line |

Figure 46

| 39311 | EDG4 | NM_004720.3 | 962 | cccgcgagtctgtccactataca | 238580 | - | see also 17328 line | | |
|---|---|---|---|---|---|---|---|---|---|
| 39312 | EDG4 | NM_004720.3 | 964 | cgcgagtctgtccactatacatc | 238581 | - | see also 17328 line | | |
| 39313 | EDG8 | NM_030760.2 | 1171 | gcccggactctggtatcagaacc | 238585 | - | receptor_acitivity、sig nal_transduction、gpcr | - | - |
| 39314 | EDG8 | NM_030760.2 | 299 | ggccgctcacgctgaaactgtcc | 238584 | - | see also 39313 line | | |
| 39315 | EDG8 | NM_030760.2 | 150 | cgccttcatcgtgctagagaatc | 238582 | - | see also 39313 line | | |
| 39316 | TSHR | NM_000369.1 | 335 | tccagaatctacgtatctataga | 238593 | - | see also 17329 line | | |
| 39317 | TSHR | NM_000369.1 | 1528 | cactcactctgagtactacaacc | 238622 | - | see also 17329 line | | |
| 39318 | GNRHR | NM_000406.1 | 1858 | tccgagtgacggttactttcttc | 238640 | - | see also 17330 line | | |
| 39319 | GNRHR | NM_000406.1 | 2177 | cacgaggcccctagctttgaaaa | 238650 | - | see also 17330 line | | |
| 39320 | GNRHR | NM_000406.1 | 2360 | atggtggcatcaagcattttata | 238658 | - | see also 17330 line | | |
| 39321 | FSHR | NM_000145.2 | 1231 | tcctaactaccagccaatataaa | 238710 | - | see also 17331 line | | |
| 39322 | FSHR | NM_000145.2 | 1327 | ttgcatcagttgatatccatacc | 238713 | - | see also 17331 line | | |
| 39323 | GPR24 | NM_005297.2 | 667 | atcaacctctcggtagtagatct | 238724 | - | see also 17332 line | | |
| 39324 | GPR24 | NM_005297.2 | 1272 | cccgaccctcacctttgtctact | 238726 | - | see also 17332 line | | |
| 39325 | GPR24 | NM_005297.2 | 1271 | gcccgaccctcacctttgtctac | 238725 | - | see also 17332 line | | |
| 39326 | GPR105 | NM_014879.1 | 1114 | cagccgtttagggaaatcttatg | 238749 | - | see also 17333 line | | |
| 39327 | GPR105 | NM_014879.1 | 990 | gagtcagaccgaagctcattaca | 238743 | - | see also 17333 line | | |
| 39328 | P2RY4 | NM_002565.2 | 384 | tgcaacggccacctacatgttcc | 238758 | - | see also 17334 line | | |
| 39329 | P2RY4 | NM_002565.2 | 448 | accctcatctactattatgcagc | 238760 | - | see also 17334 line | | |
| 39330 | P2RY4 | NM_002565.2 | 1005 | agctgactgccgagtactgaaca | 238765 | - | see also 17334 line | | |
| 39331 | GPR86 | NM_023914.2 | 279 | accggcatcctgctgaatacttt | 238772 | - | see also 17335 line | | |
| 39332 | GPR86 | NM_023914.2 | 1040 | ttgtatggatcccttaatataca | 238789 | - | see also 17335 line | | |
| 39333 | GPR87 | NM_023915.2 | 1399 | atcggaagttcgcatatattatg | 238832 | - | see also 17336 line | | |
| 39334 | GPR87 | NM_023915.2 | 675 | gaccttggtacttcaagtttatt | 238808 | - | see also 17336 line | | |
| 39335 | P2RY12 | NM_022788.3 | 888 | aactgtaccggtcatacgtaaga | 238849 | - | see also 17337 line | | |
| 39336 | P2RY12 | NM_022788.3 | 910 | aacgagggtgtaggtaaagtcc | 238851 | - | see also 17337 line | | |
| 39337 | GPR135 | NM_022571.3 | 1210 | gagggctaccggactaggaatgt | 238868 | - | see also 17338 line | | |
| 39338 | GPR135 | NM_022571.3 | 1381 | gcccgcaaaaatccagttgtact | 238870 | - | see also 17338 line | | |
| 39339 | PTAFR | NM_000952.2 | 995 | tgcgcagtagccggaaatgctcc | 238890 | - | see also 17339 line | | |
| 39340 | PTAFR | NM_000952.2 | 918 | ctgtgtcttagaccctgttatct | 238887 | - | see also 17339 line | | |
| 39341 | CYSLTR1 | NM_006639.2 | 458 | gacttccgcaatcaagtgtattc | 238893 | - | see also 4687 line | | |
| 39342 | CYSLTR1 | NM_006639.2 | 1109 | gtcgtgaccgctgcctttttagt | 238916 | - | see also 4687 line | | |
| 39343 | CYSLTR2 | NM_020377.2 | 670 | ctgatcattcgggttctgttaaa | 238953 | - | see also 7856 line | | |
| 39344 | CYSLTR2 | NM_020377.2 | 547 | ggcagtgtcacatcatgcttaga | 238949 | - | see also 7856 line | | |

Figure 46

| | | | | | | | | | |
|---|---|---|---|---|---|---|---|---|---|
| 39345 | LTB4R2 | NM_019839.1 | 2272 | gaccgctttcgtgcttcctttcg | 238958 | - | receptor_acitivity、sig nal_transduction、gpcr | - | - |
| 39346 | LTB4R2 | NM_019839.1 | 1746 | cactgctgagctggaagacttcg | 238957 | - | see also 39345 line | | |
| 39347 | CNR1 | NM_016083.3 | 970 | agccacgccgtccgcatgattca | 238971 | - | see also 6654 line | | |
| 39348 | CNR1 | NM_016083.3 | 1279 | cacgctttccggagcatgtttcc | 238975 | - | see also 6654 line | | |
| 39349 | CNR2 | NM_001841.1 | 371 | tggccagtgtggtctttgcatgc | 238980 | - | see also 17343 line | | |
| 39350 | CNR2 | NM_001841.1 | 1173 | gccagattccagagatctagacc | 238982 | - | see also 17343 line | | |
| 39351 | MTNR1A | NM_005958.2 | 1054 | gacgtggccgatagggttaaatg | 238997 | - | see also 17344 line | | |
| 39352 | MTNR1A | NM_005958.2 | 405 | gagcgtcatcggctccatattca | 238986 | - | see also 17344 line | | |
| 39353 | MTNR1B | NM_005959.2 | 836 | agcgacttgcggagctttctaac | 239008 | - | see also 4166 line | | |
| 39354 | TRHR | NM_003301.1 | 966 | acccggtgatttacaatctcatg | 239045 | - | see also 2232 line | | |
| 39355 | TRHR | NM_003301.1 | 287 | tcctgggtctatggctatgttgg | 239019 | - | see also 2232 line | | |
| 39356 | TRHR | NM_003301.1 | 962 | atcaacccggtgatttacaatct | 239044 | - | see also 2232 line | | |
| 39357 | ADMR | NM_007264.2 | 877 | cgcctacgtggccgtctttgtca | 239053 | - | see also 17347 line | | |
| 39358 | ADMR | NM_007264.2 | 471 | gccgcttcactcactacttctac | 239049 | - | see also 17347 line | | |
| 39359 | ADMR | NM_007264.2 | 698 | tgcctcttcatggcaccttttga | 239052 | - | see also 17347 line | | |
| 39360 | ADORA1 | NM_000674.1 | 1068 | ggcgacccgcagaagtactatgg | 239058 | - | see also 17348 line | | |
| 39361 | ADORA1 | NM_000674.1 | 1295 | ccgcgtcaccttccttaagattt | 239059 | - | see also 17348 line | | |
| 39362 | ADORA2A | NM_000675.3 | 600 | cgcatcccgctccggtacaatgg | 239061 | - | see also 17349 line | | |
| 39363 | ADORA2A | NM_000675.3 | 1021 | tgcccctacacatcatcaactgc | 239064 | - | see also 17349 line | | |
| 39364 | ADORA2A | NM_000675.3 | 853 | tgctcatgctgggtgtctatttg | 239062 | - | see also 17349 line | | |
| 39365 | ADORA2B | NM_000676.2 | 1213 | accgagacttccgctacactttt | 239080 | - | see also 17350 line | | |
| 39366 | ADORA2B | NM_000676.2 | 658 | tcccgctcaggtataaaagtttg | 239069 | - | see also 17350 line | | |
| 39367 | ADORA2B | NM_000676.2 | 657 | gtcccgctcaggtataaaagttt | 239068 | - | see also 17350 line | | |
| 39368 | ADORA3 | NM_000677.2 | 1105 | agcttaccgtcagatacaagagg | 239085 | - | see also 17351 line | | |
| 39369 | ADORA3 | NM_000677.2 | 1341 | gtcatgtgcgccatctatcttga | 239090 | - | see also 17351 line | | |
| 39370 | AGTRL1 | NM_005161.2 | 749 | gccgtggccacggcagttctttg | 239105 | - | see also 17352 line | | |
| 39371 | AGTRL1 | NM_005161.2 | 492 | ggcgctcagctgatatcttcatt | 239103 | - | see also 17352 line | | |
| 39372 | BLR1 | NM_001716.2 | 146 | tggacagattggacaactataac | 239110 | - | see also 17353 line | | |
| 39373 | BLR1 | NM_001716.2 | 145 | ctggacagattggacaactataa | 239109 | - | see also 17353 line | | |
| 39374 | CMKOR1 | NM_020311.1 | 311 | atcttcgtcatcggcatgattgc | 239116 | - | see also 7831 line | | |
| 39375 | CXCR6 | NM_006564.1 | 395 | tactgggcatctacactattaac | 239127 | - | see also 17355 line | | |
| 39376 | CXCR6 | NM_006564.1 | 450 | cactgtggatcgtttcattgtag | 239130 | - | see also 17355 line | | |

EP 1 752 536 A1

Figure 46

| | | | | | | | |
|---|---|---|---|---|---|---|---|
| 39377 | EBI2 | NM_004951.2 | 345 | taggataactgcgctagtgttt | 239160 | - | see also 17356 line |
| 39378 | EBI2 | NM_004951.2 | 346 | aggataactgcgctagtgttta | 239161 | - | see also 17356 line |
| 39379 | EBI2 | NM_004951.2 | 277 | accgctttgctacacgaatagc | 239156 | - | see also 17356 line |
| 39380 | ET(B)R-LP-2 | NM_004767.2 | 985 | gcccgcatgtggtggtactttgg | 239208 | - | see also 17357 line |
| 39381 | ET(B)R-LP-2 | NM_004767.2 | 1404 | tgccaatgggtcggacaacaagc | 239209 | - | see also 17357 line |
| 39382 | ET(B)R-LP-2 | NM_047767.2 | 945 | gcccgagtccctgtattcactgg | 239207 | - | see also 17357 line |
| 39383 | FKSG79 | NM_032553.1 | 372 | cagtgtgcgacgatgttggtttc | 239221 | - | see also 17358 line |
| 39384 | FKSG79 | NM_032553.1 | 624 | tccgcttcgattgtcctatatt | 239230 | - | see also 17358 line |
| 39385 | FY | NM_002036.1 | 1078 | gactctgcaccctgatatacagc | 239261 | - | see also 17359 line |
| 39386 | FY | NM_002036.1 | 702 | accagtgtcctgggtatcctagc | 239259 | - | see also 17359 line |
| 39387 | GNRHR2 | NM_057163.1 | 229 | tcccgttcaggtgtaaggaaact | 239276 | - | see also 17361 line |
| 39388 | GNRHR2 | NM_057163.1 | 228 | atccgttcaggtgtaaggaaac | 239275 | - | see also 17361 line |
| 39389 | GPBAR1 | NM_170699.1 | 724 | taacctgtacctcgaagtctatgg | 239288 | - | receptor_acitivity、gpc r、signal_transduction see also 17362 line |
| 39390 | GPR | NM_007223.1 | 1212 | ttctgcaaggtcgtcaaattttt | 239296 | - | see also 17362 line |
| 39391 | GPR | NM_007223.1 | 1214 | ctgcaaggtcgtcaaatttttgc | 239297 | - | see also 17362 line |
| 39392 | GPR1 | NM_005279.2 | 188 | ttcctatgacctagactattact | 239313 | - | see also 17363 line |
| 39393 | GPR1 | NM_005279.2 | 949 | agctcaccattcaccaccaatagc | 239334 | - | see also 17363 line |
| 39394 | GPR100 | NM_181885.1 | 84 | tgctccgatgcctgtcaaattcc | 239342 | - | see also 17364 line |
| 39395 | GPR100 | NM_181885.1 | 179 | tgctgtgggtactgagtaactgt | 239344 | - | see also 17364 line |
| 39396 | GPR100 | NM_181885.1 | 87 | tccgatgcctgtcaaattcctag | 239343 | - | see also 17364 line |
| 39397 | GPR101 | NM_054021.1 | 191 | aggtgaccaaccgtttttatcttt | 239350 | - | see also 17365 line |
| 39398 | GPR101 | NM_054021.1 | 193 | gtgaccaaccgtttttatctttaa | 239351 | - | see also 17365 line |
| 39399 | GPR102 | NM_053278.1 | 100 | ctgtacacggcgtttagctttgg | 239368 | - | see also 17366 line |
| 39400 | GPR102 | NM_053278.1 | 553 | tgcgtaggtggctgtcaaattat | 239372 | - | see also 17366 line |
| 39401 | GPR119 | NM_178471.1 | 963 | ttcctgtcacatcgtcactatct | 239398 | - | see also 17367 line |
| 39402 | GPR119 | NM_178471.1 | 553 | tactgcgacatgctcaagattgc | 239393 | - | see also 17367 line |
| 39403 | GPR12 | NM_005288.1 | 449 | cggagaggacggtcacgtttacc | 239405 | - | see also 3704 line |
| 39404 | GPR12 | NM_005288.1 | 411 | ccgctacctcactgtactacg | 239404 | - | see also 3704 line |
| 39405 | GPR120 | NM_181745.1 | 995 | acccatcctcacaacatgaca | 239416 | - | see also 10252 line |
| 39406 | GPR120 | NM_181745.1 | 560 | accaggaaattcgatttgcaca | 239411 | - | see also 10252 line |
| 39407 | GPR132 | NM_013345.2 | 1864 | tccataaggcgtggaaagagtgg | 239422 | - | see also 17370 line |
| 39408 | GPR132 | NM_013345.2 | 1801 | gcgtggctgaccccattatctac | 239421 | - | see also 17370 line |

1364

Figure 46

| 39409 | GPR145 | NM_032503.1 | 685 | gacgatgtactctggtatacact | 239438 | - | see also 17371 line |
|---|---|---|---|---|---|---|---|
| 39410 | GPR145 | NM_032503.1 | 675 | gacatcccctgacgatgtactct | 239436 | - | see also 17371 line |
| 39411 | GPR146 | NM_138445.1 | 203 | gccggacgtgtactttgtcaaca | 239457 | - | see also 17372 line |
| 39412 | GPR146 | NM_138445.1 | 198 | accatgccggacgtgtactttgt | 239455 | - | see also 17372 line |
| 39413 | GPR15 | NM_005290.1 | 802 | gggttgcggcaagaacactattt | 239481 | - | see also 17373 line |
| 39414 | GPR15 | NM_005290.1 | 801 | tgggttgcggcaagaacactatt | 239480 | - | see also 17373 line |
| 39415 | GPR151 | NM_194251.1 | 245 | gagctacggcgtactccaaaagt | 239486 | - | see also 10305 line |
| 39416 | GPR151 | NM_194251.1 | 249 | tacggcgtactccaaaagtgttt | 239487 | - | see also 10305 line |
| 39417 | GPR151 | NM_194251.1 | 470 | tgccggaatggttctttagcacc | 239497 | - | see also 10305 line |
| 39418 | GPR160 | NM_014373.1 | 501 | ttcactagcattcgttgatcttt | 239512 | - | see also 17375 line |
| 39419 | GPR160 | NM_014373.1 | 1074 | ctggttaccatttgtactacttc | 239531 | - | see also 17375 line |
| 39420 | GPR161 | NM_007369.1 | 1075 | tgctttggggaccggtattatcg | 239550 | - | see also 17376 line |
| 39421 | GPR161 | NM_007369.1 | 555 | ttggtcatccgtggagtttgacg | 239544 | - | see also 17376 line |
| 39422 | GPR18 | NM_005292.2 | 1009 | aacctcactcgactgacattttt | 239582 | - | see also 17377 line |
| 39423 | GPR18 | NM_005292.2 | 822 | gccgaagtacgccaaagaactta | 239574 | - | see also 17377 line |
| 39424 | GPR19 | NM_006143.1 | 433 | gtgcaaggttgtgcgatattttc | 239604 | - | see also 17378 line |
| 39425 | GPR19 | NM_006143.1 | 1183 | ctcgatctatgactcatttgaca | 239630 | - | see also 17378 line |
| 39426 | GPR19 | NM_006143.1 | 819 | ttgtccctcggacaaaagtgaaa | 239616 | - | see also 17378 line |
| 39427 | GPR20 | NM_005293.1 | 372 | cccgcacgtcctcggttacttcc | 239634 | - | gpcr、receptor_acitivity、signal_transduction | rhodopsin-like receptor | - |
| 39428 | GPR20 | NM_005293.1 | 321 | cacgcgcttcgctgtgtactacg | 239633 | - | see also 39427 line |
| 39429 | GPR20 | NM_005293.1 | 732 | cacggtgctcatcatctttctcg | 239635 | - | see also 39427 line |
| 39430 | GPR22 | NM_005295.1 | 98 | cactatcatatccgttaagcttt | 239643 | - | see also 3705 line |
| 39431 | GPR22 | NM_005295.1 | 132 | caccggatttcttatgttagaaa | 239647 | - | see also 3705 line |
| 39432 | GPR23 | NM_005296.1 | 492 | tccttttcgatctcgtactatta | 239696 | - | see also 3710 line |
| 39433 | GPR23 | NM_005296.1 | 497 | ttcgatctcgtactattaggact | 239697 | - | see also 3710 line |
| 39434 | GPR26 | NM_153442.1 | 913 | cgcatccgacccctttgtgtact | 239722 | - | see also 17381 line |
| 39435 | GPR26 | NM_153442.1 | 945 | gacaccagtaccgcaaaagctgc | 239723 | - | see also 17381 line |
| 39436 | GPR27 | NM_018971.1 | 806 | gcctcctcgtgctggaagaattc | 239724 | - | see also 17382 line |
| 39437 | GPR3 | NM_005281.2 | 638 | gaccacatgtggcgtggtttatc | 239729 | - | see also 17383 line |
| 39438 | GPR3 | NM_005281.2 | 138 | ggctcaggcaacgtgaatgtaag | 239726 | - | see also 17383 line |
| 39439 | GPR3 | NM_005281.2 | 639 | accacatgtggcgtggtttatcc | 239730 | - | see also 17383 line |
| 39440 | GPR30 | NM_001505.1 | 969 | tccccgacctgtacttcatcaac | 239732 | - | see also 17384 line |
| 39441 | GPR30 | NM_001505.1 | 1698 | ggctgtacattgagcagaaaaca | 239737 | - | see also 17384 line |
| 39442 | GPR30 | NM_001505.1 | 1599 | ccctcacgggccacattgtcaac | 239736 | - | see also 17384 line |

Figure 46

| | | | | | | | | | |
|---|---|---|---|---|---|---|---|---|---|
| 39443 | GPR31 | NM_005299.1 | 938 | accccagagactcctattcctga | 239738 | - | gpcr、receptor_acitivity、signal_transduction | rhodopsin-like receptor | - |
| 39444 | GPR32 | NM_001506.1 | 952 | ttcgttggcagagatttccaaga | 239740 | - | see also 17385 line | | |
| 39445 | GPR34 | NM_005300.2 | 967 | agctcgtaactcctttattgtac | 239779 | - | see also 3711 line | | |
| 39446 | GPR34 | NM_005300.2 | 411 | ttctgggtattcaccgtaaaaga | 239754 | - | see also 3711 line | | |
| 39447 | GPR35 | NM_005301.2 | 731 | gacggagacccgcatctacatga | 239790 | - | see also 17387 line | | |
| 39448 | GPR35 | NM_005301.2 | 1064 | caggagcacccggcacaatttca | 239792 | - | see also 17387 line | | |
| 39449 | GPR37 | NM_005302.2 | 746 | ctgcgtccagaaacgaaacttgt | 239796 | - | see also 3712 line | | |
| 39450 | GPR37 | NM_005302.2 | 2226 | gacaatggacctccttaatatca | 239839 | - | see also 3712 line | | |
| 39451 | GPR38 | NM_001507.1 | 940 | ttccacgttggcagaatcattta | 239848 | - | see also 17389 line | | |
| 39452 | GPR38 | NM_001507.1 | 240 | ggccgtgtccgacctactcatcc | 239847 | - | see also 17389 line | | |
| 39453 | GPR39 | NM_001508.1 | 70 | gaggtggccacctggatcaaaat | 239855 | - | see also 17390 line | | |
| 39454 | GPR39 | NM_001508.1 | 24 | cagtgactgctcccaaatcattg | 239853 | - | see also 17390 line | | |
| 39455 | GPR4 | NM_005282.1 | 649 | ttccgccatccctctacatcttt | 239862 | - | gpcr、receptor_acitivity、signal_transduction | rhodopsin-like receptor | - |
| 39456 | GPR4 | NM_005282.1 | 1371 | ttcgaggagcgcgtcttttctgc | 239864 | - | see also 39455 line | | |
| 39457 | GPR4 | NM_005282.1 | 803 | gccgctgtgggtggactacttcc | 239863 | - | see also 39455 line | | |
| 39458 | GPR40 | NM_005303.1 | 816 | tccgctggtgaccggttacttgg | 239868 | - | gpcr、receptor_acitivity、signal_transduction | rhodopsin-like receptor | - |
| 39459 | GPR40 | NM_005303.1 | 739 | agcttcctgtaccccaatctagg | 239866 | - | see also 39458 line | | |
| 39460 | GPR40 | NM_005303.1 | 350 | tccggaggccgtgctattcctgg | 239865 | - | see also 39458 line | | |
| 39461 | GPR43 | NM_005306.1 | 751 | agcccctggtggcggtcaatagc | 239871 | - | see also 17391 line | | |
| 39462 | GPR43 | NM_005306.1 | 965 | tgccaagttcggacttcactaca | 239873 | - | see also 17391 line | | |
| 39463 | GPR44 | NM_004778.1 | 647 | cgcattatgtgctactacaatgt | 239875 | - | see also 17392 line | | |
| 39464 | GPR45 | NM_007227.3 | 134 | gtcccttgaggcttacacatacc | 239877 | - | see also 5041 line | | |
| 39465 | GPR45 | NM_007227.3 | 1198 | cagtctacgtgtgcaatgaaaac | 239883 | - | see also 5041 line | | |
| 39466 | GPR48 | NM_018490.1 | 3015 | ttgcgactgctgcgaatcgtttc | 239961 | - | see also 17393 line | | |
| 39467 | GPR48 | NM_018490.1 | 3016 | tgcgactgctgcgaatcgtttct | 239962 | - | see also 17393 line | | |
| 39468 | GPR49 | NM_003667.2 | 2698 | tccgtgccatcaccagcttatcc | 240033 | - | see also 2492 line | | |
| 39469 | GPR50 | NM_004224.1 | 1004 | tgcggcaccctatcatattcttc | 240052 | - | see also 17395 line | | |
| 39470 | GPR50 | NM_004224.1 | 180 | caccatcgttgtagacctaatcg | 240041 | - | see also 17395 line | | |
| 39471 | GPR50 | NM_004224.1 | 138 | cccaccggctctaatcatcttta | 240037 | - | see also 17395 line | | |
| 39472 | GPR52 | NM_005684.1 | 780 | ccgtcgctacgccatggttttgt | 240083 | - | see also 17396 line | | |
| 39473 | GPR52 | NM_005684.1 | 779 | accgtcgctacgccatggttttg | 240082 | - | see also 17396 line | | |

Figure 46

| 39474 | GPR52 | NM_005684.1 | 413 | atcgttatcttgcaataaccaag | 240073 | - | see also 17396 line | | |
|---|---|---|---|---|---|---|---|---|---|
| 39475 | GPR54 | NM_032551.2 | 218 | tgcggaccgtgaccaacttctac | 240091 | - | see also 17397 line | | |
| 39476 | GPR55 | NM_005683.2 | 643 | gaccggaagcatccctatctaca | 240097 | - | see also 17398 line | | |
| 39477 | GPR55 | NM_005683.2 | 344 | aacaggtggcccgattatgctgc | 240095 | - | see also 17398 line | | |
| 39478 | GPR58 | NM_014626.1 | 238 | atgcttagcataacatccatttt | 240114 | - | see also 17399 line | | |
| 39479 | GPR58 | NM_014626.1 | 232 | gacctgatgcttagcataacatc | 240112 | - | see also 17399 line | | |
| 39480 | GPR6 | NM_005284.2 | 517 | accgctacctgtccctgtataac | 240139 | - | gpcr、receptor_acitivity、signal_transduction | rhodopsin-like receptor | - |
| 39481 | GPR61 | NM_031936.2 | 1075 | ccggcaacgctccgaatctctca | 240151 | - | see also 17400 line | | |
| 39482 | GPR61 | NM_031936.2 | 526 | gacgcctgccctccgaaaatttg | 240140 | - | see also 17400 line | | |
| 39483 | GPR63 | NM_030784.1 | 379 | tccttgaccgtgaatagtacagc | 240162 | - | see also 8947 line | | |
| 39484 | GPR63 | NM_030784.1 | 961 | ttcatacccttcctggtaatact | 240191 | - | see also 8947 line | | |
| 39485 | GPR73 | NM_138964.1 | 109 | ttcagctacagcgactatgatat | 240203 | - | see also 17403 line | | |
| 39486 | GPR73 | NM_138964.1 | 225 | ctgcggcattggaaacttcatct | 240207 | - | see also 17403 line | | |
| 39487 | GPR73L1 | NM_144773.2 | 230 | ccctcacccgctataagaagttg | 240217 | - | see also 17404 line | | |
| 39488 | GPR73L1 | NM_144773.2 | 557 | tcccatcggcttactttgcaaca | 240221 | - | see also 17404 line | | |
| 39489 | GPR75 | NM_006794.1 | 1640 | ctcggattgaaccttactacagc | 240243 | - | see also 17405 line | | |
| 39490 | GPR75 | NM_006794.1 | 1058 | ttcagacccgtggatataccaag | 240234 | - | see also 17405 line | | |
| 39491 | GPR78 | NM_080819.2 | 1139 | caggaagattggcattgctattg | 240251 | - | see also 17406 line | | |
| 39492 | GPR78 | NM_080819.2 | 542 | aggcgtcctcctggtgaatctgt | 240250 | - | see also 17406 line | | |
| 39493 | GPR80 | NM_080818.2 | 24 | gagccactagactatttagcaaa | 240255 | - | see also 17407 line | | |
| 39494 | GPR80 | NM_080818.2 | 867 | tacatcgtttctagaccattagc | 240290 | - | see also 17407 line | | |
| 39495 | GPR82 | NM_080817.1 | 696 | aacctgtacgtccattatggaga | 240321 | - | see also 9525 line | | |
| 39496 | GPR83 | NM_016540.2 | 539 | accccggatctcaatcacaaagg | 240344 | - | see also 17409 line | | |
| 39497 | GPR83 | NM_016540.2 | 588 | accatggctacgttctttcact | 240345 | - | see also 17409 line | | |
| 39498 | GPR83 | NM_016540.2 | 780 | tacgctcgtgtggccaagaaact | 240347 | - | see also 17409 line | | |
| 39499 | GPR84 | NM_020370.1 | 1213 | cgccaattccgccaagcatatgg | 240372 | - | see also 17410 line | | |
| 39500 | GPR84 | NM_020370.1 | 549 | tcccctctggcctatttatatcc | 240358 | - | see also 17410 line | | |
| 39501 | GPR85 | NM_018970.3 | 730 | tgcatagagcaccttactacttc | 240377 | - | see also 7615 line | | |
| 39502 | GPR85 | NM_018970.3 | 1392 | ttggtcttagacgagttcaaaat | 240395 | - | see also 7615 line | | |
| 39503 | GPR88 | NM_022049.1 | 532 | tcccggtgtcactcctgtattcg | 240402 | - | see also 17412 line | | |
| 39504 | GPR88 | NM_022049.1 | 579 | gccaacggcatggtcatctatct | 240404 | - | see also 17412 line | | |
| 39505 | GPR88 | NM_022049.1 | 578 | ggccaacggcatggtcatctatc | 240403 | - | see also 17412 line | | |
| 39506 | GPR91 | NM_033050.1 | 170 | ttgttgtttacggctacatcttc | 240409 | - | see also 17413 line | | |
| 39507 | GPR91 | NM_033050.1 | 785 | atcacgtcatgcggaatgtgagg | 240422 | - | see also 17413 line | | |

Figure 46

| | | | | | | | |
|---|---|---|---|---|---|---|---|
| 39508 | GPR92 | NM_020400.3 | 1110 | accccgctggtgactacttagc | 240426 | - | receptor_acitivity, gpcr, signal_transduction / - |
| 39509 | GPR92 | NM_020400.3 | 394 | ctcggtgtgagcgtgtacatgt | 240424 | - | see also 39508 line |
| 39510 | GPR92 | NM_020400.3 | 1109 | gacccgctggtgtactactttag | 240425 | - | see also 39508 line |
| 39511 | GRCA | NM_014449.1 | 549 | agcaatgcgtggccatcatgtct | 240427 | - | see also 17414 line |
| 39512 | GRCA | NM_014449.1 | 610 | tgcagagggccgagtttgcaaag | 240428 | - | see also 17414 line |
| 39513 | GREAT | NM_130806.2 | 783 | agcgcttgttacgggattaaat | 240456 | - | see also 9550 line |
| 39514 | GREAT | NM_130806.2 | 1307 | cccttgacggacggcattcttc | 240466 | - | see also 9550 line |
| 39515 | H963 | NM_013308.2 | 918 | cacgggctacatcatatgctttg | 240523 | - | see also 17416 line |
| 39516 | H963 | NM_013308.2 | 913 | gtgaccaacgggctacatcatatg | 240522 | - | see also 17416 line |
| 39517 | HM74 | NM_006018.1 | 1136 | gcccaacctcaaataaccattcc | 240528 | - | purinergic nucleotide receptor, G-protein coupled / gpcr, receptor_acitivity, signal_transduction / - |
| 39518 | HM74a | NM_177551.2 | 566 | tccagaatggcggtgcaaatttg | 240529 | - | - |
| 39519 | JCG1 | NM_153445.1 | 642 | agccatatcctacatctatatc | 240541 | - | see also 17417 line |
| 39520 | JCG1 | NM_153445.1 | 107 | ttgtcaccttaatgggtaatatc | 240533 | - | see also 17417 line |
| 39521 | JCG3 | NM_153444.1 | 32 | ctctgacgggttcatcctattg | 240544 | - | see also 17418 line |
| 39522 | JCG3 | NM_153444.1 | 630 | agccgtctgacatctatatcc | 240557 | - | see also 17418 line |
| 39523 | JCG6 | NM_178168.1 | 811 | agcaagaagtgttatcattatc | 240561 | - | receptor_acitivity, signal_transduction, gpcr olfactory receptor / - |
| 39524 | LGR6 | NM_021636.1 | 710 | aactgggttccataacaacaac | 240571 | - | see also 17419 line |
| 39525 | LGR6 | NM_021636.1 | 111 | ttgtcaagrgtcaataatcatct | 240564 | - | see also 17419 line |
| 39526 | LGR7 | NM_021634.1 | 1870 | atgatccttgccaaacgttttt | 240638 | - | see also 17420 line |
| 39527 | LGR7 | NM_021634.1 | 594 | cagactagaatggctgataattg | 240600 | - | see also 17420 line |
| 39528 | LHCGR | NM_000233.1 | 756 | gccacgtcatcctattctctaaa | 240674 | - | see also 17421 line |
| 39529 | LHCGR | NM_000233.1 | 153 | gccggtctcactcgactatcact | 240655 | - | see also 17421 line |
| 39530 | LHCGR | NM_000233.1 | 1942 | aacgtcgggctgaactttataga | 240714 | - | see also 17421 line |
| 39531 | MAS1L | NM_052967.1 | 312 | cacgaatccctaacaggtataca | 240722 | - | see also 17422 line |
| 39532 | MAS1L | NM_052967.1 | 346 | gtcgctgactgatctatct | 240723 | - | see also 17422 line |
| 39533 | MAS1L | NM_052967.1 | 723 | gtcgagtcgactctactcatta | 240732 | - | see also 17422 line |
| 39534 | MBC2 | NM_015292.1 | 770 | tggcgtttgcgggtgatactgg | 240748 | - | see also 17423 line |
| 39535 | MBC2 | NM_015292.1 | 845 | ccgacgcccgaccctagacatca | 240750 | - | see also 17423 line |
| 39536 | MGC21621 | NM_145015.2 | 988 | ctgtgtcctccatctacttagg | 240796 | - | see also 17424 line |

Figure 46

| 39537 | MGC21621 | NM_145015.2 | 765 | cacctgcctgcacaactacttct | 240795 | - | see also 17424 line | | |
|---|---|---|---|---|---|---|---|---|---|
| 39538 | MGC24137 | NM_152430.1 | 655 | ttctgccgctccaatatcctttc | 240806 | - | see also 9862 line | | |
| 39539 | MGC24137 | NM_152430.1 | 565 | ttgcctcgtgtcaccaaaattgg | 240804 | - | see also 9862 line | | |
| 39540 | MGC26856 | NM_152779.1 | 388 | gccattacggcttggtataatga | 240813 | - | see also 17425 line | | |
| 39541 | MGC26856 | NM_152779.1 | 457 | ggccattatacacagttagtttg | 240816 | - | see also 17425 line | | |
| 39542 | MGC40047 | NM_175911.2 | 729 | atcctatccgcatgagtaaaatg | 240832 | - | see also 17426 line | | |
| 39543 | MGC40047 | NM_175911.2 | 838 | ctgcaggtctagggctattgacc | 240834 | - | see also 17426 line | | |
| 39544 | MRGX1 | NM_147199.1 | 727 | cacgtggacagggaagtcttatt | 240852 | - | see also 17427 line | | |
| 39545 | MRGX1 | NM_147199.1 | 268 | atccccataccatctctaaaat | 240846 | - | see also 17427 line | | |
| 39546 | MRGX1 | NM_147199.1 | 270 | ccccataccatctctaaaatcc | 240848 | - | see also 17427 line | | |
| 39547 | MRGX3 | NM_054031.1 | 522 | aacgtcagatttcattacaatcg | 240854 | - | gpcr、receptor_acitivity、signal_transduction | - | - |
| 39548 | MRGX3 | NM_054031.1 | 728 | acctggattggaaagtcttattt | 240856 | - | see also 39547 line | | |
| 39549 | MRGX3 | NM_054031.1 | 701 | ttcagtgggccctgttttccagg | 240855 | - | see also 39547 line | | |
| 39550 | MRGX4 | NM_054032.1 | 226 | ctcagcttccagattatacgttc | 240857 | - | gpcr、receptor_acitivity、signal_transduction | - | - |
| 39551 | MRGX4 | NM_054032.1 | 775 | tccctgtcctctctaaacagtag | 240859 | - | see also 39550 line | | |
| 39552 | MRGX4 | NM_054032.1 | 727 | cacctgaatttggaagtcttata | 240858 | - | see also 39550 line | | |
| 39553 | OPRL1 | NM_000913.3 | 272 | gccgttctgggaggttatctacg | 240861 | - | see also 17428 line | | |
| 39554 | OPRL1 | NM_000913.3 | 681 | atgagtgtggatcgctatgtagc | 240863 | - | see also 17428 line | | |
| 39555 | OR10H1 | NM_013940.1 | 514 | aaggagatccaccattttgcttg | 240864 | - | see also 17429 line | | |
| 39556 | OR10H1 | NM_013940.1 | 515 | aggagatccaccattttgcttgc | 240865 | - | see also 17429 line | | |
| 39557 | OR10H2 | NM_013939.1 | 917 | tcctcagcacactctattcctca | 240867 | - | - | olfactory receptor | - |
| 39558 | OR10H3 | NM_013938.1 | 389 | cactgcattacaacatgctaatg | 240870 | - | see also 17430 line | | |
| 39559 | OR10H3 | NM_013938.1 | 712 | aagactttctccacttgtgtatc | 240872 | - | see also 17430 line | | |
| 39560 | OR10J1 | NM_012351.1 | 447 | gaggctgcgtatccaacttgtcc | 240879 | - | see also 17431 line | | |
| 39561 | OR10J1 | NM_012351.1 | 144 | aaccttagcaggcaatatcatca | 240876 | - | see also 17431 line | | |
| 39562 | OR11A1 | NM_013937.2 | 850 | tggaacgctcatgatctttatg | 240897 | - | see also 17432 line | | |
| 39563 | OR11A1 | NM_013937.2 | 681 | aggtgacaactctcattctgtct | 240891 | - | see also 17432 line | | |

Figure 46

| | | | | | | | |
|---|---|---|---|---|---|---|---|
| 39564 | OR12D3 | NM_030959.2 | 257 | tgcagtcgcagggctatatcttt | 240905 | - | see also 17433 line |
| 39565 | OR12D3 | NM_030959.2 | 764 | gtgggcttcacatatattcgtcc | 240916 | - | see also 17433 line |
| 39566 | OR1E2 | NM_003554.1 | 213 | ctgcttttcctcagtcacaatgc | 240920 | - | receptor_acitivity, sig nal_transduction, gpcr olfactory receptor |
| 39567 | OR1F1 | NM_012360.1 | 580 | ctcaatgaggtcataatccttag | 240923 | - | see also 17434 line |
| 39568 | OR1F1 | NM_012360.1 | 575 | cacacctcaatgaggtcataatc | 240922 | - | see also 17434 line |
| 39569 | OR1F1 | NM_012360.1 | 895 | ggggctctgaaaaagtagttgg | 240925 | - | see also 17434 line |
| 39570 | OR2H1 | NM_030883.3 | 648 | ctctgctttaccacaagttgtgt | 240926 | - | receptor_acitivity, sig nal_transduction, gpcr olfactory receptor |
| 39571 | OR2H3 | NM_007160.2 | 503 | ccgatcggcaggtggatgatttt | 240927 | - | see also 17435 line |
| 39572 | OR2J2 | NM_030905.1 | 849 | accgagtcttaatcctctaatct | 240948 | - | see also 17436 line |
| 39573 | OR2J2 | NM_030905.1 | 285 | tgctggttgcatggttcaacttt | 240932 | - | see also 17436 line |
| 39574 | OR2W1 | NM_030903.1 | 857 | acccgctcatttacacccttaaga | 240967 | - | see also 17437 line |
| 39575 | OR2W1 | NM_030903.1 | 285 | gggttgtatcatccaactctatg | 240954 | - | see also 17437 line |
| 39576 | OR3A2 | NM_002551.2 | 1182 | cacctcaccgtgtttgtctttt | 240971 | - | see also 17438 line |
| 39577 | OR3A2 | NM_002551.2 | 1183 | acctcaccgtggtttgtctttc | 240972 | - | see also 17438 line |
| 39578 | OR52A1 | NM_012375.1 | 474 | agcccatgcctagtactgataa | 240990 | - | see also 17439 line |
| 39579 | OR52A1 | NM_012375.1 | 475 | gcccatgcctagtactgataaa | 240991 | - | see also 17439 line |
| 39580 | OR5I1 | NM_006637.1 | 436 | ttgattgtcttgtcataccttgg | 241023 | - | see also 17440 line |
| 39581 | OR5I1 | NM_006637.1 | 262 | ctctcggagaacaaatctatttc | 241017 | - | see also 17440 line |
| 39582 | OR5V1 | NM_030876.3 | 864 | tacggcccatctcaacttactca | 241049 | - | see also 17441 line |
| 39583 | OR5V1 | NM_030876.3 | 494 | agcaaggttcatgcaatcaatt | 241039 | - | see also 17441 line |
| 39584 | OR6A1 | NM_003696.2 | 263 | tgcgccactacaggttactattgt | 241053 | - | see also 17442 line |
| 39585 | OR6A1 | NM_003696.2 | 472 | aggatcatggacagctaatctcc | 241057 | - | see also 17442 line |
| 39586 | P2RY1 | NM_002563.2 | 1406 | aggtaccggggtccgcaaaaaca | 241076 | - | see also 1650 line |
| 39587 | P2RY1 | NM_002563.2 | 1458 | gacgagtaacctgcgaagttattt | 241079 | - | see also 1650 line |
| 39588 | P2RY10 | NM_014499.2 | 739 | ctgacgtgcatcagtcttcaaag | 241108 | - | see also 17444 line |
| 39589 | P2RY10 | NM_014499.2 | 629 | tacccctccggatttcactattac | 241105 | - | see also 17444 line |
| 39590 | P2RY11 | NM_002566.3 | 48 | ggcagctgccgacgacaaactca | 241120 | - | see also 17445 line |
| 39591 | P2RY11 | NM_002566.3 | 844 | tgcccgagctttgcagacatagc | 241123 | - | see also 17445 line |
| 39592 | P2RY2 | NM_002564.2 | 530 | atgcgtccaccacatatgttc | 241129 | - | see also 17446 line |
| 39593 | P2RY2 | NM_002564.2 | 591 | gccgctgtgtctattactacg | 241131 | - | see also 17446 line |
| 39594 | P2RY5 | NM_005767.3 | 1675 | tactttacatcggacacaattca | 241155 | - | see also 17447 line |
| 39595 | P2RY5 | NM_005767.3 | 1270 | accactctcaggtaacaatgc | 241144 | - | see also 17447 line |
| 39596 | P2RY5 | NM_005767.3 | 1212 | cactggccgtgtggttaactgtga | 241142 | - | see also 17447 line |

Figure 46

| 39597 | P2RY6 | NM_004154.3 | 483 | gccgtgtacaccctaaaccttgc | 241162 | - | see also 17448 line |
|---|---|---|---|---|---|---|---|
| 39598 | P2RY6 | NM_004154.3 | 482 | ggccgtgtacaccctaaacctg | 241161 | - | see also 17448 line |
| 39599 | P2RY8 | NM_178129.3 | 1113 | agctgcgcctgcgggaatattg | 241178 | - | see also 17449 line |
| 39600 | P2RY8 | NM_178129.3 | 1069 | ctgtctgacccgtttgtttatt | 241175 | - | see also 17449 line |
| 39601 | RGR | NM_002921.2 | 798 | cacgatcaatgccatcaactatg | 241181 | - | receptor_acitivity, gpc rhodopsin-like r, signal transduction receptor; see also 39601 line |
| 39602 | RGR | NM_002921.2 | 699 | ccctatgccatcctgtatctat | 241180 | - | see also 39601 line |
| 39603 | RGR | NM_002921.2 | 587 | cctcttcatcacgatcacttcc | 241179 | - | see also 39601 line |
| 39604 | RRH | NM_006583.1 | 565 | gtgctacgtgtaccataaaactgg | 241202 | - | see also 4613 line |
| 39605 | RRH | NM_006583.1 | 465 | aacacttacatcggcttgattct | 241196 | - | see also 4613 line |
| 39606 | SALPR | NM_016568.1 | 732 | gggcgctgacggactttcagtttg | 241228 | - | see also 17451 line |
| 39607 | SALPR | NM_016568.1 | 379 | gccacgatagccaccatgaataa | 241223 | - | see also 17451 line |
| 39608 | sdolf | NM_033519.1 | 559 | ctctgacaccgccacattgagc | 241238 | - | see also 17452 line |
| 39609 | sdolf | NM_033519.1 | 214 | ctccactgtggtacataggatgc | 241235 | - | see also 17452 line |
| 39610 | SREB3 | NM_018969.3 | 309 | accgtccgcatcagcttatgtga | 241240 | - | see also 7614 line |
| 39611 | SREB3 | NM_018969.3 | 1256 | ctgcgtcaaccaattgctgc | 241252 | - | see also 7614 line |
| 39612 | TA4 | NM_175067.1 | 91 | tccgggtgattctgtacatagt | 241254 | - | see also 17453 line |
| 39613 | TA4 | NM_175067.1 | 564 | aggttgtcagacgttgtaaatc | 241260 | - | see also 17453 line |
| 39614 | TAR1 | NM_138327.1 | 108 | cacactcgttggcaatctgatag | 241271 | - | see also 17454 line |
| 39615 | TAR1 | NM_138327.1 | 297 | cacaagcaccgacattagctga | 241276 | - | see also 17454 line |
| 39616 | TAR3 | NM_175057.1 | 994 | aggactgattcgtcaacaactaa | 241309 | - | see also 17455 line |
| 39617 | TAR3 | NM_175057.1 | 50 | acgtgaacgaatcctgcattaaa | 241288 | - | see also 17455 line |
| 39618 | TG1019 | NM_148962.3 | 517 | gcccctccgcgtggactactacc | 241315 | - | see also 17456 line |
| 39619 | TG1019 | NM_148962.3 | 412 | cagtttggccctcttcatcttct | 241314 | - | see also 17456 line |
| 39620 | VN1R1 | NM_020633.2 | 929 | ctgtgttggtcgcctcatgtttc | 241345 | - | see also 17457 line |
| 39621 | VN1R1 | NM_020633.2 | 242 | gacccacgacttgattcctcagc | 241329 | - | see also 17457 line |
| 39622 | VN1R2 | NM_173856.1 | 605 | aagtaaaagccccgaacatacatt | 241355 | - | see also 17458 line |
| 39623 | VN1R2 | NM_173856.1 | 1077 | tccaactattagcccttttgttc | 241368 | - | see also 17458 line |
| 39624 | VN1R3 | NM_174980.1 | 427 | aacacgctggtaaatattactgt | 241380 | - | see also 17459 line |
| 39625 | VN1R3 | NM_174980.1 | 165 | agccaactccttggttatactct | 241375 | - | see also 17459 line |
| 39626 | VN1R4 | NM_173857.1 | 531 | gacactgcgtgcaatgttgttat | 241404 | - | see also 17460 line |
| 39627 | VN1R4 | NM_173857.1 | 481 | aacgaggatttggatactgttc | 241401 | - | see also 17460 line |
| 39628 | VN1R4 | NM_173857.1 | 160 | ttcttagctccgctgtaaagg | 241393 | - | see also 17460 line |
| 39629 | CASR | NM_000388.2 | 1605 | cacgcatttacggatatcctaca | 241441 | - | see also 309 line |
| 39630 | CASR | NM_000388.2 | 1582 | gtcggacccctacatagatta | 241439 | - | see also 309 line |
| 39631 | GPRC5B | NM_016235.1 | 1205 | gacccagcgctccgttagaagc | 241476 | - | see also 17462 line |

Figure 46

| 39632 | GPRC5B | NM_016235.1 | 1114 | ttctccatgatgaacaacaatgc | 241475 | - | see also 17462 line |
|---|---|---|---|---|---|---|---|
| 39633 | GPRC6A | NM_148963.1 | 2121 | gcctctatagacegatccttatt | 241537 | - | see also 17463 line |
| 39634 | GPRC6A | NM_148963.1 | 684 | gacgattggctcttaacacttt | 241495 | - | see also 17463 line |
| 39635 | GRM1 | NM_000838.2 | 3361 | taccgcgatccggatttttcacg | 241603 | - | see also 550 line |
| 39636 | GRM2 | NM_000839.1 | 1591 | accgattgacgaattcaacttgc | 241617 | - | see also 17465 line |
| 39637 | GRM2 | NM_000839.1 | 2167 | accaccgcgatgcaagtatgttg | 241621 | - | see also 17465 line |
| 39638 | GRM2 | NM_000839.1 | 1590 | taccgattgacgaattcaacttg | 241616 | - | see also 17465 line |
| 39639 | GRM3 | NM_000840.1 | 1722 | atcgctagatgtcaactctatcc | 241654 | - | see also 552 line |
| 39640 | GRM3 | NM_000840.1 | 1643 | ggcgatacaacgtgttcaatttc | 241648 | - | see also 552 line |
| 39641 | GRM4 | NM_000841.1 | 953 | ggcaggcgagttcgacaagatca | 241687 | - | see also 17467 line |
| 39642 | GRM4 | NM_000841.1 | 2123 | ctgctcgctgccgccgaatcttcc | 241695 | - | see also 17467 line |
| 39643 | GRM5 | NM_000842.1 | 2058 | tgcgaagcccaaacagatttact | 241720 | - | see also 17468 line |
| 39644 | GRM5 | NM_000842.1 | 1506 | tacgatcctattcgatgaagaatg | 241710 | - | see also 17468 line |
| 39645 | GRM6 | NM_000843.2 | 2339 | cccacacagcgtgattgactatg | 241745 | - | see also 17469 line |
| 39646 | GRM6 | NM_000843.2 | 1178 | ggcctccatcgacggagtttgacc | 241738 | - | see also 17469 line |
| 39647 | GRM6 | NM_000843.2 | 2338 | ccccacacagcgtgattgactat | 241744 | - | see also 17469 line |
| 39648 | GRM7 | NM_000844.2 | 1194 | tactttacgtccgtacacttga | 241762 | - | see also 17470 line |
| 39649 | GRM7 | NM_000844.2 | 1377 | ttcgtgattgacgcagtctatgc | 241771 | - | see also 17470 line |
| 39650 | GRM8 | NM_000845.1 | 1293 | ggcttacgccctgcacaatatgc | 241835 | - | see also 559 line |
| 39651 | GRM8 | NM_000845.1 | 1901 | gggcttcaggacggcgaacttagt | 241849 | - | see also 559 line |
| 39652 | RAI3 | NM_003979.2 | 758 | tccgctcctcgtcgcaatgaaga | 241881 | - | see also 17472 line |
| 39653 | RAI3 | NM_003979.2 | 763 | tcctcgtcgcaatgaagactttg | 241883 | - | see also 17472 line |
| 39654 | TAS1R1 | NM_138697.2 | 2404 | agcggcttcggtcggtattttct | 241926 | - | see also 17473 line |
| 39655 | TAS1R1 | NM_138697.2 | 2403 | cagcggcttcggtcggtatttc | 241925 | - | see also 17473 line |
| 39656 | TAS1R2 | NM_152232.1 | 138 | agggcattgttcaccttaacttc | 241929 | - | see also 17474 line |
| 39657 | TAS1R2 | NM_152232.1 | 139 | gggcattgttcaccttaacttcc | 241930 | - | see also 17474 line |
| 39658 | PTHR1 | NM_000316.2 | 590 | gccctgtccggactacatttatg | 241948 | - | see also 17475 line |
| 39659 | PTHR1 | NM_000316.2 | 591 | ccctgtccggactacatttatga | 241949 | - | see also 17475 line |
| 39660 | PTHR2 | NM_005048.2 | 1334 | tgcattacatcgttcgtatgc | 241988 | - | see also 17476 line |
| 39661 | PTHR2 | NM_005048.2 | 638 | aacgcctcatgtaatgtatacc | 241972 | - | see also 17476 line |
| 39662 | CALCR | NM_001742.1 | 804 | agcaacgcttgcggtggtattat | 242019 | - | see also 1153 line |
| 39663 | RCP9 | NM_014478.3 | 92 | ctcagtaactacgaggtatttca | 242036 | - | see also 17478 line |
| 39664 | RCP9 | NM_014478.3 | 190 | cactatcacctcatgaaacgttaa | 242041 | - | see also 17478 line |
| 39665 | CRHR1 | NM_004382.2 | 440 | ttctatggttgtccgtcacaatac | 242045 | - | see also 2940 line |
| 39666 | CRHR1 | NM_004382.2 | 894 | ctgaccgctggcgcaatgggatg | 242049 | - | see also 2940 line |
| 39667 | CRHR2 | NM_001883.2 | 727 | gccatcggcaagcctcactatga | 242069 | - | see also 1207 line |
| 39668 | CRHR2 | NM_001883.2 | 261 | gacgtgggcctcaaagatcaact | 242059 | - | see also 1207 line |

Figure 46

| 39669 | VIPR1 | NM_004624.2 | 1160 | tggagtacactacatcatgttcg | 242089 | - | see also 17481 line |
|---|---|---|---|---|---|---|---|
| 39670 | VIPR1 | NM_004624.2 | 486 | gacaaggcagcgagtttggatga | 242077 | - | see also 17481 line |
| 39671 | GCGR | NM_000160.1 | 799 | tgccatccacgcgaatctgtttg | 242094 | - | see also 17482 line |
| 39672 | GCGR | NM_000160.1 | 800 | gccatccacgcgaatctgtttgc | 242095 | - | see also 17482 line |
| 39673 | GHRHR | NM_000823.1 | 922 | ctctcggtcggggtgaactttgg | 242110 | - | see also 17483 line |
| 39674 | GHRHR | NM_000823.1 | 323 | ctgtgaaacgggattgtactatc | 242102 | - | see also 17483 line |
| 39675 | SCTR | NM_002980.1 | 668 | ttcgtgccctgtccaacttcatc | 242117 | - | see also 17484 line |
| 39676 | SCTR | NM_002980.1 | 897 | gggttctccagccatttttgttg | 242118 | - | see also 17484 line |
| 39677 | FKSG83 | NM_032030.1 | 110 | ttgggctcatggggaacttattg | 242121 | - | see also 17485 line |
| 39678 | FKSG83 | NM_032030.1 | 520 | tccactaatgttggacttgtata | 242142 | - | see also 17485 line |
| 39679 | VN1R5 | NM_173858.1 | 936 | tgccgcaattagtcctttgatgc | 242176 | - | see also 17486 line |
| 39680 | VN1R5 | NM_173858.1 | 225 | caggtctaagtccattgacatga | 242160 | - | see also 17486 line |
| 39681 | ADCYAP1R1 | NM_001118.3 | 592 | gtcggaacccttccctcattact | 242181 | - | see also 17487 line |
| 39682 | ADCYAP1R1 | NM_001118.3 | 1075 | ggctacgctgagactctactttg | 242196 | - | see also 17487 line |
| 39683 | ADCYAP1R1 | NM_001118.3 | 351 | agctgatgggcttcaatgattcc | 242179 | - | see also 17487 line |
| 39684 | BAI2 | NM_001703.1 | 2305 | tacgtgccctcggctgatgatgt | 242217 | - | see also 1115 line |
| 39685 | BAI2 | NM_001703.1 | 601 | gagcaggtgtgcgcacactttgc | 242205 | - | see also 1115 line |
| 39686 | BAI3 | NM_001704.1 | 3610 | gacattgcctgtcgatcagttct | 242338 | - | see also 1116 line |
| 39687 | BAI3 | NM_001704.1 | 4385 | ttcgggatataccaaatacaagc | 242367 | - | see also 1116 line |
| 39688 | C17orf35 | NM_003876.1 | 544 | ccgttggcgctgcccttagatta | 242374 | - | see also 17490 line |
| 39689 | C17orf35 | NM_003876.1 | 416 | tggaagcccagtacaagtacatt | 242373 | - | see also 17490 line |
| 39690 | C7orf9 | NM_022150.2 | 508 | caccatgtgccaatgacttattt | 242387 | - | see also 17491 line |
| 39691 | C7orf9 | NM_022150.2 | 323 | tgggaggaacgttcaagaagaaa | 242383 | - | see also 17491 line |
| 39692 | CALCRL | NM_005795.2 | 1445 | cgcgttctcatcaccaagttaaa | 242428 | - | see also 17492 line |
| 39693 | CALCRL | NM_005795.2 | 948 | gacacggattgtctattgcatca | 242400 | - | see also 17492 line |
| 39694 | CALCRL | NM_005795.2 | 626 | atcatgacagctcaatatgaatg | 242392 | - | see also 17492 line |
| 39695 | FZD1 | NM_003505.1 | 459 | ggcgcgagctgggaactttgtgc | 242447 | - | see also 17493 line |
| 39696 | FZD1 | NM_003505.1 | 1544 | ccgagtggtgtgtaatgacaagt | 242452 | - | see also 17493 line |
| 39697 | FZD10 | NM_007197.2 | 2053 | caggtgtgcagccgtaggttaaa | 242459 | - | see also 17494 line |
| 39698 | FZD10 | NM_007197.2 | 2058 | gtgcagccgtaggttaaagaaga | 242461 | - | see also 17494 line |
| 39699 | FZD2 | NM_001466.2 | 818 | ggcccgatggttccatgttcttc | 242466 | - | see also 17495 line |
| 39700 | FZD2 | NM_001466.2 | 1686 | cacggtctacatgatcaaatacc | 242471 | - | see also 17495 line |
| 39701 | FZD3 | NM_017412.2 | 1305 | cagtgacacaaggatctcataat | 242505 | - | see also 6984 line |
| 39702 | FZD3 | NM_017412.2 | 1080 | ttgctcgctatttcataggattg | 242492 | - | see also 6984 line |

EP 1 752 536 A1

Figure 46

| | | | | | | | |
|---|---|---|---|---|---|---|---|
| 39703 | FZD4 | NM_012193.2 | 1690 | tgggcacttttcggtattctgc | 242559 | - | see also 5185 line |
| 39704 | FZD4 | NM_012193.2 | 1700 | ttcggtattctgcagatgattcc | 242560 | - | see also 5185 line |
| 39705 | FZD5 | NM_003468.2 | 816 | aggtcctctgcatggattacaac | 242562 | - | see also 17498 line |
| 39706 | FZD5 | NM_003468.2 | 1666 | ccgcatccgcagcgtcatccaagc | 242563 | - | see also 17498 line |
| 39707 | FZD6 | NM_003506.2 | 555 | accttgtctaaactttgtgaga | 242569 | - | see also 2363 line |
| 39708 | FZD6 | NM_003506.2 | 2087 | gagctagcacccccaggttaaga | 242614 | - | see also 2363 line |
| 39709 | FZD8 | NM_031866.1 | 242 | gtgctcgccgatctcaagttct | 242626 | - | see also 17500 line |
| 39710 | FZD8 | NM_031866.1 | 2039 | caeggcgagctccgtgtcttatc | 242630 | - | see also 17500 line |
| 39711 | FZD9 | NM_003508.1 | 1713 | gttggcacgcactgccactataag | 242643 | - | see also 17501 line |
| 39712 | FZD9 | NM_003508.1 | 1546 | ggcggtcttcatgctcaaaattt | 242642 | - | see also 17501 line |
| 39713 | GIPR | NM_000164.1 | 1475 | gccgggagttggaaagttactgc | 242657 | - | see also 17502 line |
| 39714 | GIPR | NM_000164.1 | 560 | tagccctgctcatcttgagtttg | 242649 | - | see also 17502 line |
| 39715 | GLP1R | NM_002062.2 | 228 | gccaggctcgttcgtgaatgtca | 242659 | - | see also 17503 line |
| 39716 | GLP1R | NM_002062.2 | 230 | caggctcgttcgtgaatgtcagc | 242660 | - | see also 17503 line |
| 39717 | GLP2R | NM_004246.1 | 437 | tagagaacgccacggatatttgg | 242675 | - | see also 2850 line |
| 39718 | GLP2R | NM_004246.1 | 695 | acgtcgtcttcacaactcttac | 242678 | - | see also 2850 line |
| 39719 | GPR110 | NM_153840.1 | 1081 | atcgtgagtgccaatgtactca | 242731 | - | see also 10021 line |
| 39720 | GPR110 | NM_153840.1 | 1268 | ctcacacacgtcgtatttgcatg | 242737 | - | see also 10021 line |
| 39721 | GPR111 | NM_153839.1 | 47 | tgccattcgcggctaacttgact | 242756 | - | see also 17506 line |
| 39722 | GPR111 | NM_153839.1 | 1448 | tagccaatgtgtgcattgttaac | 242787 | - | see also 17506 line |
| 39723 | GPR112 | NM_153834.1 | 8188 | agccggtcgagataaaagttgg | 243025 | - | see also 17507 line |
| 39724 | GPR112 | NM_153834.1 | 3047 | cccatgagaacttgattcgtacc | 242877 | - | see also 17507 line |
| 39725 | GPR113 | NM_153835.1 | 2994 | caeggtccctcattacatcttca | 243052 | - | see also 17508 line |
| 39726 | GPR113 | NM_153835.1 | 1927 | gccccattggtccgtaatggaac | 243041 | - | see also 17508 line |
| 39727 | GPR115 | NM_153838.1 | 1353 | atcgatgaccgacacaaagttctgg | 243086 | - | see also 17509 line |
| 39728 | GPR115 | NM_153838.1 | 2213 | cactaggcccaaccaatggatct | 243107 | - | see also 17509 line |
| 39729 | GPR115 | NM_153838.1 | 54 | tagcggtaggtttggtattgttc | 243057 | - | see also 17509 line |
| 39730 | GPR116 | NM_015234.1 | 2377 | caegtgctctcacggttaatgt | 243173 | - | see also 17510 line |
| 39731 | GPR116 | NM_015234.1 | 3120 | gaccaagaaccggacttcttata | 243185 | - | see also 17510 line |
| 39732 | GPR116 | NM_015234.1 | 2002 | tgccaggatccgtaataggtgt | 243165 | - | see also 17510 line |
| 39733 | GPR123 | NM_032422.1 | 2422 | atcgtgctcactattctacact | 243207 | - | see also 17511 line |
| 39734 | GPR123 | NM_032422.1 | 17 | accactcgcggctgaaatccaga | 243195 | - | see also 17511 line |
| 39735 | GPR124 | NM_032777.6 | 2658 | gtcctatgctccggttctatttg | 243223 | - | see also 17512 line |
| 39736 | GPR124 | NM_032777.6 | 1828 | gtcgtccttccacatccaagaaca | 243222 | - | see also 17512 line |
| 39737 | GPR128 | NM_032787.1 | 1485 | gccgctgcaaccatactactaat | 243268 | - | see also 17513 line |
| 39738 | GPR128 | NM_032787.1 | 715 | cacagcaccacttaataacattt | 243236 | - | see also 17513 line |
| 39739 | GPR128 | NM_032787.1 | 804 | cgcgagtggttggacagatattc | 243239 | - | see also 17513 line |

Figure 46

| | | | | | | | | |
|---|---|---|---|---|---|---|---|---|
| 39740 | GPR133 | NM_198827.1 | 565 | agcaagcaccgttactactatgg | 243323 | - | see also 17514 line | |
| 39741 | GPR133 | NM_198827.1 | 245 | aggtggcgctgtcgtctatcagc | 243320 | - | see also 17514 line | |
| 39742 | GPR143 | NM_000273.1 | 324 | gtccaccgtgtggttaggattcc | 243327 | - | see also 17515 line | |
| 39743 | GPR143 | NM_000273.1 | 779 | ccgtgatcaagatccgatttttc | 243334 | - | see also 17515 line | |
| 39744 | GPR157 | NM_024980.2 | 569 | ggccattgcgctctacttgtacc | 243344 | - | see also 17516 line | |
| 39745 | GPR64 | NM_005756.1 | 2726 | agcaatggaggcggtatctttgt | 243421 | - | see also 3997 line | |
| 39746 | GPR97 | NM_170776.3 | 606 | ggcagcggcgtgttgaacaatcg | 243446 | - | see also 17517 line | |
| 39747 | GPR97 | NM_170776.3 | 1367 | aaccatgtacgccctctatatca | 243452 | - | see also 17517 line | |
| 39748 | LANCL1 | NM_006055.1 | 887 | ccctccatgtataggtgataatc | 243485 | - | see also 4214 line | |
| 39749 | LANCL1 | NM_006055.1 | 564 | gggcgaataggctacatctatgc | 243475 | - | see also 4214 line | |
| 39750 | LANCL1 | NM_006055.1 | 122 | cccgaatccttatgctgattata | 243459 | - | see also 4214 line | |
| 39751 | TAS2R38 | NM_176817.1 | 275 | accaagccatcatcatgctatgg | 243498 | - | see also 17519 line | |
| 39752 | TAS2R38 | NM_176817.1 | 279 | agccatcatcatgctatggatga | 243499 | - | see also 17519 line | |
| 39753 | TAS2R38 | NM_176817.1 | 19 | atccgcactgtgtcctatgaagt | 243496 | - | see also 17519 line | |
| 39754 | TAS2R39 | NM_176881.1 | 921 | ctcaattctactgattcaagata | 243524 | - | see also 17520 line | |
| 39755 | TAS2R39 | NM_176881.1 | 102 | caccttaattttagcagtttac | 243506 | - | see also 17520 line | |
| 39756 | TAS2R40 | NM_176882.1 | 65 | tggtggtctccggaatagagtgc | 243526 | - | see also 17521 line | |
| 39757 | TAS2R40 | NM_176882.1 | 159 | ccccactggtgaccgcattatgt | 243528 | - | see also 17521 line | |
| 39758 | TAS2R41 | NM_176883.1 | 447 | ttgggtgaactaccctgtatatc | 243548 | - | see also 17522 line | |
| 39759 | TAS2R41 | NM_176883.1 | 451 | gtgaactaccctgtatatcaaga | 243549 | - | see also 17522 line | |
| 39760 | TAS2R45 | NM_176886.1 | 550 | aacttagtaccctttactgtaac | 243559 | - | receptor_acitivity、gpcr、signal_transduction | - | - |
| 39761 | TAS2R46 | NM_176887.1 | 522 | ttcaaatacaacggtaaccatcc | 243560 | - | - | - | - |
| 39762 | TAS2R48 | NM_176888.1 | 531 | gactgtaactactctagcaaacc | 243561 | - | receptor_acitivity、gpcr、signal_transduction | - | - |
| 39763 | TAS2R48 | NM_176888.1 | 725 | ttctgtgtataatcacatcaact | 243563 | - | see also 39762 line | |
| 39764 | TAS2R48 | NM_176888.1 | 559 | cccttttactctgagcctaatatg | 243562 | - | see also 39762 line | |
| 39765 | TAS2R49 | NM_176889.1 | 757 | aagatgcgaccaaaagaaattgt | 243577 | - | see also 17523 line | |
| 39766 | TAS2R49 | NM_176889.1 | 725 | ttctgtgtctaatcatatcgttt | 243572 | - | see also 17523 line | |
| 39767 | TAS2R49 | NM_176889.1 | 731 | gtctaatcatatcgtttggaat | 243574 | - | see also 17523 line | |
| 39768 | TAS2R50 | NM_176890.1 | 721 | ttctttctattcctaatcgtttc | 243580 | - | see also 17524 line | |
| 39769 | TAS2R50 | NM_176890.1 | 812 | atcttgcattcgactcattcatc | 243581 | - | see also 17524 line | |
| 39770 | TAS2R60 | NM_177437.1 | 215 | tggtaatgggtaagaccatttat | 243586 | - | see also 17525 line | |
| 39771 | TAS2R60 | NM_177437.1 | 221 | tgggtaagaccatttatgttttc | 243587 | - | see also 17525 line | |
| 39772 | VIPR2 | NM_003382.3 | 1023 | gccctggtgggtcatacgaatac | 243615 | - | see also 17526 line | |

1375

Figure 46

| | | | | | | | |
|---|---|---|---|---|---|---|---|
| 39773 | VIPR2 | NM_003382.3 | 1033 | gtcatacgaataccgattttaat | 243616 | - | see also 17526 line |
| 39774 | VIPR2 | NM_003382.3 | 1022 | tgccctggtgggtcatacgaata | 243614 | - | see also 17526 line |
| 39775 | XPR1 | NM_004736.2 | 2201 | aggctcgtgacactaaggtattg | 243683 | - | see also 3254 line |
| 39776 | XPR1 | NM_004736.2 | 2204 | ctcgtgacactaaggtattgata | 243685 | - | see also 3254 line |
| 39777 | LIFR | NM_002310.2 | 2112 | gacttgcgactacgtcattaagt | 243763 | - | see also 1475 line |
| 39778 | LIFR | NM_002310.2 | 1400 | tgggtcgatcacaatcaacaatt | 243738 | - | see also 1475 line |
| 39779 | GHR | NM_000163.1 | 1033 | tagctataaacccgaattccaca | 243865 | - | see also 17529 line |
| 39780 | GHR | NM_000163.1 | 659 | atgatggaccctatattgacaac | 243845 | - | see also 17529 line |
| 39781 | EPOR | NM_000121.2 | 1027 | caccacccacaagggtaacttcc | 243893 | - | see also 17530 line |
| 39782 | EPOR | NM_000121.2 | 997 | gagcccagagagcgagtttgaag | 243892 | - | see also 17530 line |
| 39783 | CNTFR | NM_001842.3 | 601 | ctgtgctgcatggctccaaaatt | 243897 | - | see also 1189 line |
| 39784 | GFRA2 | NM_001495.3 | 1097 | ctgccctgcggacaattaccagg | 243906 | - | see also 17531 line |
| 39785 | GFRA2 | NM_001495.3 | 641 | ctcccgcctctcggacatcttca | 243903 | - | see also 17531 line |
| 39786 | CRLF2 | NM_022148.2 | 1000 | cccacagaccgaggagaaagagg | 243913 | - | see also 17532 line |
| 39787 | CSF3R | NM_000760.2 | 1271 | agcggacggatccaaggttatgt | 243924 | - | see also 17533 line |
| 39788 | CSF3R | NM_000760.2 | 1584 | gcgcgagcaatagcaacaagacc | 243931 | - | see also 17533 line |
| 39789 | EBI3 | NM_005755.2 | 305 | ggctccctacgtgctcaatgtca | 243946 | - | - | - | - |
| 39790 | EBI3 | NM_005755.2 | 307 | ctccctacgtgctcaatgtcacc | 243947 | - | see also 39789 line |
| 39791 | EBI3 | NM_005755.2 | 357 | agcttcgtgcctttcataacaga | 243948 | - | see also 39789 line |
| 39792 | F3 | NM_001993.2 | 793 | gtgattccctcccgaacagttaa | 243965 | - | see also 17534 line |
| 39793 | F3 | NM_001993.2 | 214 | ggcgcttcaggcactacaaatac | 243949 | - | see also 17534 line |
| 39794 | FAD104 | NM_022763.2 | 3435 | tgcgtgtcgtcgctgtttagaca | 244061 | - | see also 8458 line |
| 39795 | FAD104 | NM_022763.2 | 1779 | tcctaccagaccgcttgtcaaag | 244018 | - | see also 8458 line |
| 39796 | IL11RA | NM_004512.3 | 629 | gagccagtaccggattaatgtga | 244077 | - | see also 17536 line |
| 39797 | IL11RA | NM_004512.3 | 631 | gccagtaccggattaatgtgact | 244078 | - | see also 17536 line |
| 39798 | IL13RA1 | NM_001560.2 | 497 | accagtcccgacactaactatac | 244096 | - | see also 17537 line |
| 39799 | IL13RA1 | NM_001560.2 | 502 | tcccgacactaactatactctct | 244097 | - | see also 17537 line |
| 39800 | IL13RA1 | NM_001560.2 | 280 | tccggaaactcgtcgttcaatag | 244088 | - | see also 17537 line |
| 39801 | IL20RA | NM_014432.2 | 1513 | agggtatgcttcgcatttgatgg | 244143 | - | see also 17538 line |
| 39802 | IL20RA | NM_014432.2 | 1371 | ccctcaatatctcggatgattct | 244139 | - | see also 17538 line |
| 39803 | IL22RA2 | NM_052962.2 | 408 | ttcagtcccgaaattttcacaac | 244156 | - | see also 17539 line |
| 39804 | IL22RA2 | NM_052962.2 | 389 | aagcctcagagggtacaatttca | 244153 | - | see also 17539 line |
| 39805 | MGC34923 | NM_144717.2 | 300 | gtggtttttctacgcattgattc | 244180 | - | see also 17540 line |
| 39806 | MGC34923 | NM_144717.2 | 418 | gtgatcgcgcctggagaaacagt | 244182 | - | see also 17540 line |
| 39807 | MPL | NM_005373.1 | 1655 | gggtcctaggccagtaccttagg | 244210 | - | see also 3743 line |

Figure 46

| | | | | | | | |
|---|---|---|---|---|---|---|---|
| 39808 | GRIA1 | NM_000827.2 | 185 | cgcggtagtaggtgccaatttcc | 244213 | - | see also 528 line |
| 39809 | GRIA1 | NM_000827.2 | 1235 | cacgctccacgtgattgaaatga | 244235 | - | see also 528 line |
| 39810 | GRIA2 | NM_000826.1 | 2063 | tcctcctacacggctaacttagc | 244299 | - | see also 524 line |
| 39811 | GRIA3 | NM_000828.2 | 383 | tccgctttgccgtgcagttatac | 244313 | - | see also 529 line |
| 39812 | GRIA3 | NM_000828.2 | 389 | ttgccgtgcagttatacacacac | 244315 | - | see also 529 line |
| 39813 | GRIK1 | NM_000830.2 | 1246 | cgggtttcgctgcttaacattg | 244389 | - | see also 540 line |
| 39814 | GRIK1 | NM_000830.2 | 2747 | tacggagtgggaacaccattgg | 244425 | - | see also 540 line |
| 39815 | GRIK2 | NM_021956.2 | 234 | gtcttcaggcgcaccgttaaact | 244440 | - | see also 8216 line |
| 39816 | GRIK2 | NM_021956.2 | 2808 | aagtgccagcgtcgttaaaaca | 244514 | - | see also 8216 line |
| 39817 | GRIK2 | NM_021956.2 | 711 | accgtcacgttgtgtatgatga | 244448 | - | see also 8216 line |
| 39818 | GRIK3 | NM_000831.2 | 2665 | gtgtacaagctccgcaaaacagc | 244550 | - | see also 17546 line |
| 39819 | GRIK3 | NM_000831.2 | 806 | gccgggaattccgcattatcttc | 244529 | - | see also 17546 line |
| 39820 | GRIK4 | NM_014619.2 | 530 | agctgctccggcaattccttatc | 244554 | - | see also 5794 line |
| 39821 | GRIK4 | NM_014619.2 | 2442 | tggcttaatcgtggccattttta | 244588 | - | see also 5794 line |
| 39822 | GRIK5 | NM_002088.3 | 2496 | gtggccggtcatggaattcatatg | 244606 | - | see also 1353 line |
| 39823 | GRIK5 | NM_002088.3 | 1083 | ctgcgcatggtagagtgatgatgg | 244599 | - | see also 1353 line |
| 39824 | GRIA4 | NM_000829.1 | 1093 | cactatggacgtagagtcaatta | 244642 | - | see also 536 line |
| 39825 | GRIA4 | NM_000829.1 | 167 | atgcgtcggaagctcctttaat | 244613 | - | see also 536 line |
| 39826 | GRIA4 | NM_000829.1 | 490 | tactgatacctccaagctattat | 244627 | - | see also 536 line |
| 39827 | GRID2 | NM_001510.1 | 57 | ctgggcgaatgcgggattcgatca | 244675 | - | see also 1034 line |
| 39828 | GRIN3A | NM_133445.1 | 3243 | ctccattgaccgccaacatatcc | 244814 | - | see also 17551 line |
| 39829 | GRIN3A | NM_133445.1 | 3627 | gtcagcttaccgtatggaatact | 244832 | - | see also 17551 line |
| 39830 | CD163 | NM_004244.2 | 963 | gacggctgggacagttacgatgc | 244850 | - | see also 17552 line |
| 39831 | CD163 | NM_004244.2 | 2234 | tggtcaacttcgcctggtaaatg | 244872 | - | see also 17552 line |
| 39832 | CD5 | NM_014207.2 | 1402 | gcctcccacgtggataacgaata | 244907 | - | see also 17553 line |
| 39833 | CD5 | NM_014207.2 | 285 | cccagtcaagcgtcaaaagtct | 244898 | - | see also 17553 line |
| 39834 | CD5 | NM_014207.2 | 1406 | cccacgtgataacgaatacagc | 244909 | - | see also 17553 line |
| 39835 | CD5L | NM_005894.1 | 757 | gacacgtggtcgaatgtgaaga | 244919 | - | see also 17554 line |
| 39836 | CD5L | NM_005894.1 | 271 | agcggaaccctagtgtgatttt | 244912 | - | see also 17554 line |
| 39837 | CD5L | NM_005894.1 | 943 | ttcagagaccggaaatgctatgg | 244921 | - | see also 17554 line |
| 39838 | CD6 | NM_006725.1 | 968 | ccggcaggatgtactactcatgc | 244924 | - | see also 17555 line |
| 39839 | CD6 | NM_006725.1 | 1114 | ccgaagtccctgcaagtgttca | 244928 | - | see also 17555 line |
| 39840 | DMBT1 | NM_004406.1 | 4720 | aacgccgaacctggtgatattca | 244976 | - | see also 17556 line |
| 39841 | DMBT1 | NM_004406.1 | 3619 | ttctggttatcgcataaaacctgg | 244942 | - | see also 17556 line |
| 39842 | DMBT1 | NM_004406.1 | 4220 | acccgtccaaacacagattattc | 244963 | - | see also 17556 line |
| 39843 | M160 | NM_174941.2 | 1050 | gacattccggaacgtcaatttt | 245011 | - | see also 17557 line |
| 39844 | M160 | NM_174941.2 | 1013 | tcctgctccgtaatgaatcttt | 245007 | - | see also 17557 line |

Figure 46

| | | | | | | | | MHC class I receptor |
|---|---|---|---|---|---|---|---|---|
| 39845 | M160 | NM_174941.2 | 331 | agccgtgactagacatggaaaa | 244992 | - | see also 17557 line | |
| 39846 | MARCO | NM_006770.2 | 212 | atggagcctttgaaatcaatgt | 245079 | - | see also 17558 line | |
| 39847 | MARCO | NM_006770.2 | 207 | ttgcaatggagcctttcgaaatc | 245078 | - | see also 17558 line | |
| 39848 | MSR1 | NM_002445.2 | 368 | ttgctcagttagttcaactaatg | 245097 | - | see also 17559 line | |
| 39849 | MSR1 | NM_002445.2 | 170 | ctgctccgaatctgtgaaatttg | 245092 | - | see also 17559 line | |
| 39850 | SCARA3 | NM_016240.2 | 297 | cacatcggtgccggattctttacc | 245122 | - | see also 6748 line | |
| 39851 | SCARA3 | NM_016240.2 | 402 | ctccttgacccagtctatttatg | 245124 | - | see also 6748 line | |
| 39852 | SCARA3 | NM_016240.2 | 467 | atccgaaagccctgaacaactgc | 245127 | - | see also 6748 line | |
| 39853 | SRCRB4D | NM_080744.1 | 879 | tacgacactgccgaatggaaaaa | 245142 | - | see also 17560 line | |
| 39854 | SRCRB4D | NM_080744.1 | 776 | gcgtccacaattgctttcactac | 245140 | - | see also 17560 line | |
| 39855 | B2M | NM_004048.2 | 213 | tccatccgacattgaagttgact | 245145 | - | see also 2730 line | |
| 39856 | B2M | NM_004048.2 | 121 | atccagcgtactccaaagattca | 245143 | - | see also 2730 line | |
| 39857 | B2M | NM_004048.2 | 217 | tccgacattgaagttgacttact | 245147 | - | see also 2730 line | |
| 39858 | HLA-E | NM_005516.3 | 264 | cagggacacgcacacagatttcc | 245174 | - | see also 17562 line | |
| 39859 | HLA-E | NM_005516.3 | 490 | tccgagcaaaagtcaaatgatgc | 245178 | - | see also 17562 line | |
| 39860 | HLA-E | NM_005516.3 | 487 | atcccgagcaaaagtcaaatga | 245177 | - | see also 17562 line | |
| 39861 | HLA-F | NM_018950.1 | 1065 | gtctctcacagctaataaagtgt | 245182 | - | see also 17563 line | |
| 39862 | HLA-F | NM_018950.1 | 1060 | ggggtgtctccacagctaataa | 245179 | - | see also 17563 line | |
| 39863 | HLA-G | NM_002127.3 | 744 | cccacaccctgtctttgactat | 245183 | - | receptor activity | MHC class I receptor |
| 39864 | MR1 | NM_001531.1 | 335 | gtctcacacttaccagagaatga | 245195 | - | see also 17564 line | |
| 39865 | MR1 | NM_001531.1 | 118 | tccatggggtccctgaatttatt | 245188 | - | see also 17564 line | |
| 39866 | ULBP1 | NM_025218.1 | 557 | cactgggggattgtaagatgtgg | 245211 | - | receptor activity | |
| 39867 | ULBP1 | NM_025218.1 | 571 | aagatgtggcttgaagaatttt | 245212 | - | see also 39866 line | |
| 39868 | ULBP3 | NM_024518.1 | 219 | atctatgggtcacctagaagagc | 245218 | - | see also 17565 line | |
| 39869 | ULBP3 | NM_024518.1 | 103 | tggtataacttcaccatcattca | 245214 | - | see also 17565 line | |
| 39870 | CD74 | NM_004355.1 | 501 | acctgagacaccttaagaacacc | 245225 | - | see also 17566 line | |
| 39871 | HLA-DMB | NM_002118.3 | 782 | cccctcttacgggacacttaca | 245235 | - | see also 17567 line | |
| 39872 | HLA-DMB | NM_002118.3 | 411 | aagatgccccttgcgaatttgg | 245229 | - | see also 17567 line | |
| 39873 | HLA-DOA | NM_002119.2 | 283 | gccggcatcgccgcaatcaaagc | 245239 | - | see also 17568 line | |
| 39874 | HLA-DOA | NM_002119.2 | 440 | tcccccctgatcaatatcacc | 245241 | - | see also 17568 line | |
| 39875 | HLA-DOA | NM_002119.2 | 436 | atcttccccctgtgatcaatat | 245240 | - | see also 17568 line | |
| 39876 | HLA-DOB | NM_002120.2 | 164 | ggcaaaggctgactgttacttca | 245246 | - | see also 17569 line | |
| 39877 | HLA-DOB | NM_002120.2 | 205 | tgcagtttggtgtcagattcatc | 245247 | - | see also 17569 line | |
| 39878 | HLA-DPA1 | NM_033554.2 | 145 | aacttatgccgcgtttgtacaga | 245261 | - | see also 17570 line | |

Figure 46

| 39879 | HLA-DPA1 | NM_033554.2 | 175 | accaacaggggagtttatgtttg | 245263 | - | see also 17570 line |
|---|---|---|---|---|---|---|---|
| 39880 | HLA-DPB1 | NM_002121.4 | 715 | ctgattctgcccggagtaagaca | 245273 | - | see also 17571 line |
| 39881 | HLA-DPB1 | NM_002121.4 | 719 | ttctgcccggagtaagacattga | 245274 | - | see also 17571 line |
| 39882 | HLA-DQA2 | NM_020056.1 | 206 | ggcagttgcctatgtttagcaaa | 245277 | - | see also 17572 line |
| 39883 | HLA-DQA2 | NM_020056.1 | 96 | ctcctatggtgtgaacttctacc | 245276 | - | see also 17572 line |
| 39884 | HLA-DQA2 | NM_020056.1 | 243 | cccgcagagtgcactgagaaata | 245279 | - | see also 17572 line |
| 39885 | HLA-DQB1 | NM_002123.2 | 785 | tcctcgggctgggccttatcatc | 245284 | - | see also 17573 line |
| 39886 | HLA-DQB1 | NM_002123.2 | 227 | ttgtgagcagaagcatctataac | 245283 | - | see also 17573 line |
| 39887 | HLA-DQB2 | NM_182549.1 | 273 | atcgaggactggaacaactataa | 245286 | - | see also 17574 line |
| 39888 | HLA-DQB2 | NM_182549.1 | 274 | tcgaggactggaacaactataag | 245287 | - | see also 17574 line |
| 39889 | HLA-DRA | NM_019111.2 | 189 | accaatcaggcgagtttatgttt | 245296 | - | see also 17575 line |
| 39890 | HLA-DRA | NM_019111.2 | 162 | tccaggccgagttctatctgaat | 245294 | - | see also 17575 line |
| 39891 | HLA-DRB3 | NM_022555.3 | 146 | ttcttggagctgcgtaagtctga | 245297 | - | receptor_acitivity、sig nal_transduction | | |
| 39892 | TNFRSF1A | NM_001065.2 | 1382 | cccgttgcgctggaaggaattcg | 245321 | - | see also 17576 line |
| 39893 | TNFRSF1A | NM_001065.2 | 1465 | gcctgcgcgaggcgcaatacagc | 245322 | - | see also 17576 line |
| 39894 | TNFRSF19 | NM_018647.2 | 1225 | aactcaggatgcactaactatga | 245346 | - | see also 17577 line |
| 39895 | TNFRSF19 | NM_018647.2 | 711 | ctgagctgtcgtgttttgacaga | 245334 | - | see also 17577 line |
| 39896 | TNFRSF1B | NM_001066.2 | 626 | caggccccaccagatctgtaacg | 245353 | - | see also 17578 line |
| 39897 | TNFRSF1B | NM_001066.2 | 206 | atgccggctcagagaatactatg | 245348 | - | see also 17578 line |
| 39898 | XEDAR | NM_021783.2 | 141 | tgccctcctcgcaggtacaaaag | 245362 | - | see also 17579 line |
| 39899 | XEDAR | NM_021783.2 | 142 | gccctcctcgcaggtacaaaagc | 245363 | - | see also 17579 line |
| 39900 | TNFRSF14 | NM_003820.2 | 1016 | ctccgtccagcggaaaagacagg | 245377 | - | see also 17580 line |
| 39901 | TNFRSF14 | NM_003820.2 | 462 | cccaagtgcagtccaggttatcg | 245372 | - | see also 17580 line |

Figure 46

| | | | | | | | molecular_function | |
|---|---|---|---|---|---|---|---|---|
| 39902 | TNFRSF25 | NM_003790.2 | 209 | aagaagattggtctgtttgttg | 245379 | - | receptor_acitivity, apoptosis, signal_transduction | - |
| 39903 | TNFRSF25 | NM_003790.2 | 193 | tgccggtgacttccacaagaaga | 245378 | - | see also 39902 line | - |
| 39904 | FLJ30296 | NM_173495.1 | 1490 | ttgtagcaaccgcgacacaaacc | 245424 | - | see also 17581 line | - |
| 39905 | FLJ30296 | NM_173495.1 | 1330 | tacgagagtcacctattggtatg | 245419 | - | see also 17581 line | - |
| 39906 | NPC1 | NM_000271.1 | 2940 | ctcgtcctggatcgacgattatt | 245549 | - | see also 17582 line | - |
| 39907 | NPC1 | NM_000271.1 | 3935 | agcgcgaacggcttctaaatttc | 245584 | - | see also 17582 line | - |
| 39908 | NPC1L1 | NM_013389.1 | 1474 | cccccctcaatccggacaataacc | 245599 | - | see also 17583 line | - |
| 39909 | NPC1L1 | NM_013389.1 | 1477 | ccctcaatcggacaataccagt | 245600 | - | see also 17583 line | - |
| 39910 | PTCH | NM_000264.2 | 1509 | cgcgtggccagcggctacttact | 245650 | - | see also 207 line | - |
| 39911 | PTCH | NM_000264.2 | 2615 | agcagactaccgaatatccagc | 245666 | - | see also 207 line | - |
| 39912 | PTCH2 | NM_003738.2 | 2565 | tccaccgagtcttctacatgg | 245696 | - | see also 2551 line | - |
| 39913 | PTCH2 | NM_003738.2 | 2970 | ctggacggctggcctcatagtgc | 245699 | - | see also 2551 line | - |
| 39914 | XPO7 | NM_015024.2 | 3329 | cacttatggcgtgaatagcaatg | 245795 | - | see also 6190 line | - |
| 39915 | XPO7 | NM_015024.2 | 2649 | ttcggagtcttcgtctctatgg | 245777 | - | see also 6190 line | - |
| 39916 | CD14 | NM_000591.1 | 484 | cgctcgaggacctaagataacc | 245799 | - | see also 17587 line | - |
| 39917 | CD14 | NM_000591.1 | 482 | gacgctcgaggacctaaagataaa | 245797 | - | see also 17587 line | - |
| 39918 | UNC5C | NM_003728.2 | 984 | gacgagggtatcagagaaaacgtaca | 245828 | - | see also 17588 line | - |
| 39919 | UNC5C | NM_003728.2 | 836 | caggcccgactctctgatactgc | 245822 | - | see also 17588 line | - |
| 39920 | ROBO1 | NM_002941.2 | 2791 | acctacgcgggtatcagaaaagt | 245941 | - | see also 1975 line | - |
| 39921 | ROBO1 | NM_002941.2 | 4153 | agcggacgctccagtgttagttc | 245973 | - | see also 1975 line | - |
| 39922 | ROBO1 | NM_002941.2 | 342 | cctcgctcacccgaatgttgc | 245859 | - | see also 1975 line | - |
| 39923 | RRBP1 | NM_004587.1 | 2731 | ctggcatgtcgttaacaagaag | 246006 | - | see also 17590 line | - |
| 39924 | RRBP1 | NM_004587.1 | 2407 | caggcccgagtgccagaaactacg | 246002 | - | see also 17590 line | - |
| 39925 | AGER | NM_001136.3 | 582 | cacactgcagtcggagctaatgg | 246013 | - | see also 17591 line | - |
| 39926 | AGER | NM_001136.3 | 1175 | agcgtgcagaactgaatcagtcg | 246016 | - | see also 17591 line | - |
| 39927 | CD3D | NM_000732.1 | 249 | atctaccgtgcaagttcattatc | 246030 | - | see also 17592 line | - |
| 39928 | CD3D | NM_000732.1 | 253 | accgtgcaagttcattatcgaat | 246031 | - | see also 17592 line | - |
| 39929 | CD3E | NM_000733.1 | 224 | tactatggcaacacaatgataaa | 246041 | - | see also 478 line | - |
| 39930 | CD3E | NM_000733.1 | 433 | gtgattgtcggttgccacaattgt | 246047 | - | see also 478 line | - |
| 39931 | CD3G | NM_000073.1 | 276 | accctcgaggatgttatcagtgt | 246059 | - | see also 17594 line | - |
| 39932 | CD3G | NM_000073.1 | 272 | aaggaccctcgagggatgtatca | 246058 | - | see also 17594 line | - |
| 39933 | CD79A | NM_001783.1 | 191 | tccaatgcccgcacaatgacagc | 246068 | - | receptor_acitivity, signal_transduction | - |
| 39934 | CD79B | NM_000626.1 | 559 | cacgctgaaggatgttatcatca | 246077 | - | receptor_acitivity, signal_transduction | chronic lymphocytic leukaemia |

Figure 46

| | | | | | | | |
|---|---|---|---|---|---|---|---|
| 39935 | CD79B | NM_000626.1 | 561 | cgctgaaggatggtatcatcatg | 246078 | - | see also 39934 line |
| 39936 | CLDN3 | NM_001306.2 | 748 | gtccccacgcgagaagaagtac | 246081 | - | see also 17595 line |
| 39937 | CLDN3 | NM_001306.2 | 749 | tccccacgcgagaagaagtaca | 246082 | - | see also 17595 line |
| 39938 | CLDN3 | NM_001306.2 | 751 | cccaacgcgagaagaagtacacg | 246083 | - | see also 17595 line |
| 39939 | CLDN4 | NM_001305.3 | 345 | ctccatgggctacagtaatgg | 246084 | - | see also 17596 line |
| 39940 | CLDN4 | NM_001305.3 | 911 | agcttactccgccaagtattct | 246088 | - | see also 17596 line |
| 39941 | CR2 | NM_001877.1 | 2543 | gtcgcgtattaggtgtcatact | 246182 | - | see also 17597 line |
| 39942 | CR2 | NM_001877.1 | 1162 | cagaaagatcgatatacctataa | 246133 | - | see also 17597 line |
| 39943 | DCC | NM_005215.1 | 2254 | aacatcgttggtgcgaggttatat | 246242 | - | see also 17598 line |
| 39944 | DCC | NM_005215.1 | 1773 | caccgaatatagtcttcgattct | 246230 | - | see also 17598 line |
| 39945 | DCC | NM_005215.1 | 2622 | gacgtctgaggtgcgactttaca | 246254 | - | see also 17598 line |
| 39946 | EDAR | NM_022336.1 | 503 | cccgagcggaatactcaaactgc | 246289 | - | see also 17599 line |
| 39947 | EDAR | NM_022336.1 | 500 | cagcccgagcggaatactcaaac | 246288 | - | see also 17599 line |
| 39948 | EVI2A | NM_014210.1 | 305 | cccgtctgtgggctaacagtact | 246308 | - | see also 17600 line |
| 39949 | EVI2A | NM_014210.1 | 304 | accgtctgtgggctaacagtac | 246307 | - | see also 17600 line |
| 39950 | EVI2A | NM_014210.1 | 449 | ctcacatcgtagctttaacttct | 246315 | - | see also 17600 line |
| 39951 | GPC1 | NM_002081.1 | 656 | ctcagagctgcgctgctactacc | 246326 | - | see also 17601 line |
| 39952 | GPC1 | NM_002081.1 | 1114 | tgctcatcaccgacaagttctgg | 246328 | - | see also 17601 line |
| 39953 | GPC4 | NM_001448.2 | 279 | gccgagtcaagtcgaaaagttg | 246331 | - | see also 1010 line |
| 39954 | GPC4 | NM_001448.2 | 814 | gagatgtccctcgcaaattgaag | 246345 | - | see also 1010 line |
| 39955 | GPC5 | NM_004466.3 | 1240 | cacatggcgagcttaatccaca | 246391 | - | see also 17602 line |
| 39956 | GPC5 | NM_004466.3 | 627 | tgcggctcgccaggatatgcagc | 246376 | - | see also 17602 line |
| 39957 | GPC6 | NM_005708.2 | 1552 | gacccgatagatgtcaagatttc | 246421 | - | see also 3961 line |
| 39958 | GPC6 | NM_005708.2 | 1515 | ggcgactgaggggccattcaaca | 246420 | - | see also 3961 line |
| 39959 | ICAM1 | NM_000201.1 | 1335 | gcccgagctcaagtgctaaagg | 246434 | - | see also 17604 line |
| 39960 | ICAM1 | NM_000201.1 | 1547 | gcctcagcacgtacctctataac | 246438 | - | see also 17604 line |
| 39961 | IGSF6 | NM_005849.1 | 633 | aagagtgctcggcgtattttca | 246450 | - | see also 17605 line |
| 39962 | IGSF6 | NM_005849.1 | 279 | tgcttggacgggtgcaaaagtga | 246444 | - | see also 17605 line |
| 39963 | IL17RD | NM_017563.1 | 1701 | gtcaggccgtcctataccgtcg | 246483 | - | see also 7033 line |
| 39964 | IL17RD | NM_017563.1 | 660 | gccggacaatctagcttgtaaac | 246461 | - | see also 7033 line |
| 39965 | KLRA1 | NM_006611.1 | 671 | aagatgaactcgtattttacatt | 246509 | - | see also 17607 line |
| 39966 | KLRA1 | NM_006611.1 | 463 | ctggattcacgccttatacaaaa | 246500 | - | see also 17607 line |
| 39967 | KLRA1 | NM_006611.1 | 720 | ctcaatgttggacctaagatatt | 246510 | - | see also 17607 line |
| 39968 | LRRC21 | NM_015613.1 | 1867 | caggagaatcaatgagtacttct | 246525 | - | see also 17608 line |
| 39969 | LRRC21 | NM_015613.1 | 1371 | caccccaggctaagaacacaact | 246520 | - | see also 17608 line |
| 39970 | LTBR | NM_002342.1 | 411 | cacatgtgccgagaattcctaca | 246529 | - | see also 17609 line |
| 39971 | LTBR | NM_002342.1 | 1222 | ggcgggtctatgactatcactgg | 246541 | - | see also 17609 line |

Figure 46

| | | | | | | | |
|---|---|---|---|---|---|---|---|
| 39972 | LTBR | NM_002342.1 | 1195 | cacggtaccaatggcattcatgt | 246539 | - | see also 17609 line |
| 39973 | LU | NM_005581.2 | 176 | aacccacgaccattatgctgg | 246551 | - | see also 17610 line |
| 39974 | LU | NM_005581.2 | 170 | tacgggaaccacgaccattata | 246547 | - | see also 17610 line |
| 39975 | M6PR | NM_002355.2 | 900 | tgccgttctaaacctcgaaatgt | 246571 | - | see also 17611 line |
| 39976 | M6PR | NM_002355.2 | 610 | gacacaccctagcggacaatttt | 246563 | - | see also 17611 line |
| 39977 | M6PR | NM_002355.2 | 612 | cacaccctagccggacaattttaa | 246564 | - | see also 17611 line |
| 39978 | MFRP | NM_031433.1 | 1150 | gccggacacagcatagaactaca | 246578 | - | see also 17612 line |
| 39979 | MFRP | NM_031433.1 | 1327 | gtgctgtttaggacagatcatgg | 246581 | - | see also 17612 line |
| 39980 | NCR2 | NM_004828.2 | 880 | gactaagataagcgatgatgatg | 246589 | - | see also 17613 line |
| 39981 | NCR2 | NM_004828.2 | 767 | aggagcctggatacccaaaaagc | 246587 | - | see also 17613 line |
| 39982 | NCR2 | NM_004828.2 | 400 | aggacattactggtgtagaatct | 246585 | - | see also 17613 line |
| 39983 | NGFR | NM_002507.1 | 903 | gtgggccttgtggcctacatagc | 246591 | - | see also 17614 line |
| 39984 | NGFR | NM_002507.1 | 1216 | accagcccgagcacatagactcc | 246593 | - | see also 17614 line |
| 39985 | PLXN3 | NM_017514.1 | 3193 | tgccattgaccgggctaacatct | 246609 | - | see also 17615 line |
| 39986 | PLXN3 | NM_017514.1 | 2420 | aacgcctcgtactcctatgaagg | 246607 | - | see also 17615 line |
| 39987 | PLXN3 | NM_017514.1 | 5659 | tgcgctcaacgagctgatttct | 246620 | - | see also 17615 line |
| 39988 | SARM1 | NM_015077.1 | 747 | tagccggtttggcgactaacaag | 246627 | - | see also 6225 line |
| 39989 | SARM1 | NM_015077.1 | 1761 | tgggtgcccgcaactttgttg | 246635 | - | see also 6225 line |
| 39990 | SFRP1 | NM_003012.2 | 952 | aagaaaatggcgacaagaagatt | 246640 | - | see also 17617 line |
| 39991 | SFRP4 | NM_003014.2 | 758 | gccccgatcggtgcaagtgtaaa | 246647 | - | see also 2018 line |
| 39992 | SFRP4 | NM_003014.2 | 760 | ccgatcggtgcaagtgtaaaaa | 246649 | - | see also 2018 line |
| 39993 | SFRP4 | NM_003014.2 | 610 | tgcgacgagctgcctgtctatga | 246644 | - | see also 2018 line |
| 39994 | SFRP5 | NM_003015.2 | 702 | cggcctccagtgaccaagatctgc | 246666 | - | see also 2020 line |
| 39995 | SORL1 | NM_003105.3 | 149 | gtcgcgactccgttcctattca | 246669 | - | see also 17620 line |
| 39996 | SORL1 | NM_003105.3 | 4434 | tgcgacgggtaccgagatttgtgc | 246758 | - | see also 17620 line |
| 39997 | SORL1 | NM_003105.3 | 4557 | caccaaccgaagaagtgtattcc | 246762 | - | see also 17620 line |
| 39998 | TIRAP | NM_052887.2 | 996 | acctgagctccgattcatgtact | 246818 | - | see also 17621 line |
| 39999 | TIRAP | NM_052887.2 | 1041 | tggttggctttcgtcaagtcaaag | 246820 | - | see also 17621 line |
| 40000 | TLR5 | NM_003268.3 | 1846 | ttccaagcatacccgatatcttc | 246862 | - | see also 17622 line |
| 40001 | TLR5 | NM_003268.3 | 723 | ggccgaatagcctttatcgttt | 246828 | - | see also 17622 line |
| 40002 | TLR5 | NM_003268.3 | 724 | gccgaatagcctttatcgtttc | 246829 | - | see also 17622 line |
| 40003 | TLR7 | NM_016562.2 | 944 | gtgcgccgtgtaaaataattct | 246910 | - | see also 6915 line |
| 40004 | TLR7 | NM_016562.2 | 2771 | aggggtatcagcgtctaatatca | 246974 | - | see also 6915 line |
| 40005 | TLR9 | NM_017442.2 | 2090 | accgtgcagccggagatgtttgc | 246992 | - | see also 17624 line |
| 40006 | TLR9 | NM_017442.2 | 2153 | tgcatctcgcaggcagtcaatgg | 246993 | - | see also 17624 line |
| 40007 | TNFRSF10C | NM_003841.2 | 934 | atcgtaggagatcattagttctaat | 247013 | - | see also 17625 line |

Figure 46

| | | | | | | | |
|---|---|---|---|---|---|---|---|
| 40008 | TNFRSF10C | NM_003841.2 | 931 | accatcgtaggggatcatagtct | 247012 | - | see also 17625 line |
| 40009 | TNFRSF5 | NM_001250.3 | 261 | tgcggtgaaagcgaattcctaga | 247016 | - | see also 17626 line |
| 40010 | TNFRSF5 | NM_001250.3 | 463 | ttggggtcaagcagattgctaca | 247017 | - | see also 17626 line |
| 40011 | TNFRSF6 | NM_000043.3 | 390 | ttgctagattatcgtccaaaagt | 247025 | - | see also 17627 line |
| 40012 | TNFRSF6 | NM_000043.3 | 1209 | aagaagcgtatgacacattgatt | 247042 | - | see also 17627 line |
| 40013 | TNFRSF7 | NM_001242.3 | 444 | agctgtcggcactgtaactctgg | 247048 | - | see also 17628 line |
| 40014 | TNFRSF7 | NM_001242.3 | 828 | ctccatcaaagaaggaaatatag | 247054 | - | see also 17628 line |
| 40015 | TNFRSF7 | NM_001242.3 | 829 | tccatcaacgaaggaaatataga | 247055 | - | see also 17628 line |
| 40016 | TNFRSF8 | NM_001243.2 | 1593 | cgcggaagagcgaggggttaatga | 247061 | - | see also 17629 line |
| 40017 | TNFRSF8 | NM_001243.2 | 1740 | cacggagcacaccaataacaaga | 247063 | - | see also 17629 line |
| 40018 | WSX1 | NM_004843.2 | 1579 | ggggtccctatcgaatcactgt | 247078 | - | see also 17630 line |
| 40019 | WSX1 | NM_004843.2 | 916 | cagttccactaccgaagatgtca | 247068 | - | see also 17630 line |
| 40020 | LOC51036 | NM_015854.1 | 388 | caggcggaacaccgttttccaag | 247089 | - | see also 17631 line |
| 40021 | LOC51036 | NM_015854.1 | 133 | agcaaacgagtgcagtcaattca | 247084 | - | see also 17631 line |
| 40022 | LY96 | NM_015364.2 | 164 | aactcatccgatgcaagtattc | 247090 | - | see also 17632 line |
| 40023 | LY96 | NM_015364.2 | 384 | atgacgattactcttttgcaga | 247098 | - | see also 17632 line |
| 40024 | LY96 | NM_015364.2 | 546 | tcctaccaacctaattcaaat | 247102 | - | see also 17632 line |
| 40025 | PVRL1 | NM_002855.2 | 1499 | ctcagaacgacgggtcttcatt | 247113 | - | see also 1887 line |
| 40026 | PVRL1 | NM_002855.2 | 544 | tgggaaactcgttaaaaggtga | 247108 | - | see also 1887 line |
| 40027 | PVRL2 | NM_002856.1 | 1164 | cgcgctgagcaggtcatcttgt | 247120 | - | see also 17634 line |
| 40028 | PVRL2 | NM_002856.1 | 1102 | cagtggacagtcgttcaatacc | 247117 | - | see also 17634 line |
| 40029 | ZP2 | NM_003460.1 | 2012 | gtgtcggctcatcgatctaaaa | 247171 | - | see also 2347 line |
| 40030 | ZP2 | NM_003460.1 | 1556 | tcctccgccaaccaatttacatg | 247160 | - | see also 2347 line |
| 40031 | ZP2 | NM_003460.1 | 2014 | gtcggctcatcgatctaaaagc | 247172 | - | see also 2347 line |
| 40032 | CR1 | NM_000573.2 | 4329 | tcctacgatcccaattaatgact | 247183 | - | see also 17636 line |
| 40033 | CR1 | NM_000573.2 | 3988 | aagggcagttcgttagtcattg | 247178 | - | see also 17636 line |
| 40034 | ITPR1 | NM_002222.1 | 308 | tacgcggaggggatcgacaaatgg | 247230 | - | see also 1411 line |
| 40035 | ITPR1 | NM_002222.1 | 4649 | accgaaatcgtcatgatattgt | 247301 | - | see also 1411 line |
| 40036 | ITPR1 | NM_002222.1 | 4648 | caccgaaatcgtcatgagtattg | 247300 | - | see also 1411 line |
| 40037 | ITPR2 | NM_002223.1 | 7592 | ggccgtctcattattctaactgc | 247558 | - | see also 17638 line |
| 40038 | ITPR2 | NM_002223.1 | 1123 | aggagggagcgttgttagattat | 247386 | - | see also 17638 line |
| 40039 | PLAUR | NM_002659.1 | 1170 | cggcactcacgaaccgaaaaacc | 247591 | - | see also 17639 line |
| 40040 | PLAUR | NM_002659.1 | 760 | agccgttacctcgaatgcatttc | 247585 | - | see also 17639 line |
| 40041 | PLAUR | NM_002659.1 | 761 | gccgttacctcgaatgcatttcc | 247586 | - | see also 17639 line |
| 40042 | AHSA2 | NM_152392.1 | 1683 | gttggcactgggtgtaaggattcc | 247598 | - | see also 17640 line |
| 40043 | AHSA2 | NM_152392.1 | 1888 | tacaattgaaggagttcctatct | 247606 | - | see also 17640 line |

Figure 46

| 40044 | AIPL1 | NM_014336.2 | 110 | accggatcccgagtgatctttca | 247614 | - | see also 17641 line |
|---|---|---|---|---|---|---|---|
| 40045 | AIPL1 | NM_014336.2 | 85 | cacgggcgagctcccaaacttca | 247611 | - | see also 17641 line |
| 40046 | ANTXR1 | NM_018153.2 | 983 | gtgtccagcgcctatcttaaaag | 247643 | - | see also 17642 line |
| 40047 | ANTXR1 | NM_018153.2 | 795 | aagtcctgcatcgaaattctagc | 247635 | - | see also 17642 line |
| 40048 | BTN1A1 | NM_001732.1 | 431 | gggagtacacgtgcttttttcagg | 247656 | - | see also 17643 line |
| 40049 | BTN1A1 | NM_001732.1 | 720 | gtcctgctacatccagaatctcc | 247660 | - | see also 17643 line |
| 40050 | BTN3A2 | NM_007047.2 | 1227 | gtcttcgtccgataccaataagt | 247670 | - | see also 17644 line |
| 40051 | BTN3A2 | NM_007047.2 | 1231 | tcgtccgataccaataagtcagc | 247671 | - | see also 17644 line |
| 40052 | BTNL3 | NM_006707.2 | 1059 | gtggacgtgggacaaaatgtagg | 247683 | - | see also 17645 line |
| 40053 | BTNL3 | NM_006707.2 | 474 | ttcagttcccagatttacgatga | 247676 | - | see also 17645 line |
| 40054 | C6orf33 | NM_133367.1 | 611 | tggcgtgagcgtttaccaatatg | 247697 | - | see also 17646 line |
| 40055 | C6orf33 | NM_133367.1 | 612 | ggcgtgagcgtttaccaatatgg | 247698 | - | see also 17646 line |
| 40056 | C6orf80 | NM_015439.2 | 396 | tggccgtggtgcaataaggaact | 247712 | - | see also 17647 line |
| 40057 | C6orf80 | NM_015439.2 | 557 | aacttcacagcgaccaaagttaa | 247714 | - | see also 17647 line |
| 40058 | CD160 | NM_007053.1 | 322 | aaggaacgcgactaaacttaatc | 247722 | - | see also 17648 line |
| 40059 | CD160 | NM_007053.1 | 327 | acgcgactaaacttaatctgtac | 247724 | - | see also 17648 line |
| 40060 | CD200R | NM_138806.2 | 309 | ctcctatcgcattaagaaatttg | 247746 | - | see also 17649 line |
| 40061 | CD200R | NM_138806.2 | 305 | tgccctcctatcgcattaagaaa | 247743 | - | see also 17649 line |
| 40062 | CD200R | NM_138806.2 | 306 | gccctcctatcgcattaagaaat | 247744 | - | see also 17649 line |
| 40063 | CD36 | NM_000072.1 | 803 | ttgccataatcgacacatataaa | 247797 | - | see also 48 line |
| 40064 | CD36 | NM_000072.1 | 389 | agcaaagaggtccttatacgtac | 247780 | - | see also 48 line |
| 40065 | CD7 | NM_006137.5 | 706 | tgctcgtggcgggataagaattc | 247827 | - | see also 17651 line |
| 40066 | CD7 | NM_006137.5 | 200 | tgggagcctccgtcaacatcacc | 247822 | - | see also 17651 line |
| 40067 | CD86 | NM_006889.2 | 950 | agcggcctcgcaactcttataaa | 247861 | - | see also 17652 line |
| 40068 | CD86 | NM_006889.2 | 799 | aagacgcggcttttatcttcacc | 247855 | - | see also 17652 line |
| 40069 | CNGB1 | NM_001297.1 | 3419 | ggggcaaaaggcggcaaacttgc | 247892 | - | see also 17653 line |
| 40070 | CNGB1 | NM_001297.1 | 2208 | gggacatcattacggacaaaaag | 247878 | - | see also 17653 line |
| 40071 | CNGB3 | NM_019098.2 | 1565 | cagttagccctcgccattgatgt | 247952 | - | see also 7704 line |
| 40072 | CNGB3 | NM_019098.2 | 956 | gtcgcatcaataataccatttga | 247932 | - | see also 7704 line |
| 40073 | CNTNAP1 | NM_003632.1 | 1155 | acctggcctatcggcataacttc | 247986 | - | see also 17655 line |
| 40074 | CNTNAP1 | NM_003632.1 | 730 | ggccgacatactctatttcgacg | 247982 | - | see also 17655 line |
| 40075 | CNTNAP1 | NM_003632.1 | 1288 | cccggtaccgcaccctatcaact | 247992 | - | see also 17655 line |
| 40076 | CRL3 | NM_139017.3 | 639 | ttcgctaagaaccgtaaggataa | 248052 | - | see also 17656 line |
| 40077 | CRL3 | NM_139017.3 | 1330 | aagttggcgagccatattccatc | 248073 | - | see also 17656 line |
| 40078 | CRLF1 | NM_004750.2 | 1152 | ggcatctatggctccaagaaagc | 248096 | - | receptor_acitivity |  |  |
| 40079 | CRLF3 | NM_015986.2 | 903 | ttcggaacgattctgaatcatcg | 248116 | - | see also 6596 line |
| 40080 | CRLF3 | NM_015986.2 | 1217 | cacttcaagcttcgagtaactat | 248124 | - | see also 6596 line |

Figure 46

| | | | | | | | |
|---|---|---|---|---|---|---|---|
| 40081 | CRLF3 | NM_015986.2 | 1128 | atcagttacccgcgagttacttct | 248120 | - | see also 6596 line |
| 40082 | CRSP3 | NM_004830.2 | 3222 | gagagaccgcgcatttctcaaac | 248226 | - | see also 17658 line |
| 40083 | CRSP3 | NM_004830.2 | 4151 | aacttcgtcttcgattcatcaca | 248265 | - | see also 17658 line |
| 40084 | CRSP6 | NM_004268.3 | 654 | ctcagacgttgcaattgatatct | 248279 | - | see also 2866 line |
| 40085 | CRSP6 | NM_004268.3 | 652 | tcctcagacgttgcaattgatat | 248278 | - | see also 2866 line |
| 40086 | DKFZP434N1817 | NM_032137.2 | 1961 | tggacgacaaggtgttataagatg | 248338 | | see also 17660 line |
| 40087 | DKFZP434N1817 | NM_032137.2 | 1032 | ctgccttaccagctgatctacc | 248321 | - | see also 17660 line |
| 40088 | DNAJ | NM_032364.4 | 783 | atcggctgggtccgaaaacgaagt | 248375 | - | see also 17661 line |
| 40089 | DNAJ | NM_032364.4 | 2060 | ttgagtcggatctttcaagtacc | 248403 | - | see also 17661 line |
| 40090 | EBAG9 | NM_004215.3 | 761 | atgggagcacacaggtttctctagt | 248416 | - | see also 2822 line |
| 40091 | EBAG9 | NM_004215.3 | 405 | caccagtttcggttatttaaat | 248404 | - | see also 2822 line |
| 40092 | EPS8 | NM_004447.3 | 1802 | atcgccatatagatagaaattat | 248463 | - | see also 2981 line |
| 40093 | EPS8 | NM_004447.3 | 1890 | cagtgagctccggttctaaagg | 248466 | - | see also 2981 line |
| 40094 | EPS8L1 | NM_017729.2 | 517 | gacgacgtagagagctttgtatc | 248502 | - | see also 17664 line |
| 40095 | EPS8L1 | NM_017729.2 | 963 | cccatacagacccgagttcttca | 248503 | - | see also 17664 line |
| 40096 | EPS8L2 | NM_022772.2 | 1948 | agcaggccggtcagaagtactgg | 248519 | - | see also 17665 line |
| 40097 | EPS8L2 | NM_022772.2 | 1627 | gccgacagtccataaagaaactcc | 248518 | - | see also 17665 line |
| 40098 | EPS8L3 | NM_024526.2 | 1072 | tgccctgagctgtacacatcc | 248524 | - | see also 17666 line |
| 40099 | EPS8L3 | NM_024526.2 | 984 | cccaggcacagtacattgactgc | 248523 | - | see also 17666 line |
| 40100 | FCRH1 | NM_052938.3 | 1207 | ttcactggcgctactataaccagc | 248552 | - | see also 17667 line |
| 40101 | FCRH1 | NM_052938.3 | 1280 | aaggtttcctttagcacatctattc | 248556 | - | see also 17667 line |
| 40102 | FEM1B | NM_015322.2 | 865 | ttcgacggtatgtcattgattgg | 248563 | - | see also 6354 line |
| 40103 | FEM1B | NM_015322.2 | 2344 | aagactccgctagacaaaagtac | 248599 | - | see also 6354 line |
| 40104 | FKSG87 | NM_032029.1 | 1124 | cacaagcaacgcagtttctaag | 248615 | - | see also 17669 line |
| 40105 | FKSG87 | NM_032029.1 | 1030 | ggggtcaggatagctcttgatgc | 248614 | - | see also 17669 line |
| 40106 | FLJ10587 | NM_018149.2 | 957 | gagtcgtgcttgactaatcaga | 248635 | - | see also 7284 line |
| 40107 | FLJ10587 | NM_018149.2 | 955 | aagagtcgtgcttgactaatca | 248634 | - | see also 7284 line |
| 40108 | FLVCR | NM_014053.1 | 242 | gacacccgctgcgaaaggatac | 248698 | - | see also 17671 line |
| 40109 | FLVCR | NM_014053.1 | 1142 | cccctgaagagtactcctataag | 248705 | - | see also 17671 line |
| 40110 | FLVCR | NM_014053.1 | 1249 | gtctcaagttattaaatcaaat | 248711 | - | see also 17671 line |
| 40111 | FRAG1 | NM_014489.1 | 912 | tactttcggcacaacatgtattg | 248729 | - | see also 17672 line |
| 40112 | FRAG1 | NM_014489.1 | 896 | ctcgggctggcgtgtctacttc | 248727 | - | see also 17672 line |
| 40113 | FREB | NM_032738.2 | 290 | aggtcaaggcctacactttcagt | 248733 | - | see also 17673 line |
| 40114 | FREB | NM_032738.2 | 591 | ggctatcacagtccaagaactgt | 248736 | - | see also 17673 line |
| 40115 | GAC1 | NM_006338.1 | 1737 | aaaccgaacccgagatctactgg | 248753 | - | see also 17675 line |

Figure 46

| | | | | | | | |
|---|---|---|---|---|---|---|---|
| 40116 | GAC1 | NM_006338.1 | 809 | gccagcctacaggaacctatct | 248746 | - | see also 17675 line |
| 40117 | GFRA1 | NM_005264.2 | 1762 | tccaatgtgtcgggcaatacaca | 248776 | - | see also 3701 line |
| 40118 | GFRA1 | NM_005264.2 | 1408 | cttgcctactcggggcttattgg | 248770 | - | see also 3701 line |
| 40119 | GFRA1 | NM_005264.2 | 943 | ttccgggtggtccattcatatc | 248761 | - | see also 3701 line |
| 40120 | GFRA4 | NM_022139.2 | 504 | caccgccgtcaccctaactacg | 248783 | - | see also 17677 line |
| 40121 | GOSR1 | NM_004871.1 | 151 | cggcacaggtatagttctgatac | 248788 | - | see also 17678 line |
| 40122 | GOSR1 | NM_004871.1 | 703 | ctcatcctaggggggtgttattgg | 248812 | - | see also 17678 line |
| 40123 | GPA33 | NM_005814.1 | 950 | cacagacacatcgggttactaca | 248824 | - | see also 17679 line |
| 40124 | GPA33 | NM_005814.1 | 492 | tccagtcgagaggagcttattca | 248815 | - | see also 17679 line |
| 40125 | GYPB | NM_002100.2 | 226 | taccagctctgtagtgataata | 248826 | - | see also 17680 line |
| 40126 | HAVCR1 | NM_012206.1 | 1003 | aaggaggttcaacaactaagtgt | 248848 | - | see also 17681 line |
| 40127 | HAVCR1 | NM_012206.1 | 794 | caccattgtactcttacacaaca | 248841 | - | see also 17681 line |
| 40128 | HAVCR2 | NM_032782.2 | 681 | ctctagattggccatgacttac | 248868 | - | see also 17682 line |
| 40129 | HAVCR2 | NM_032782.2 | 922 | accattgaagagaacgtatatga | 248871 | - | see also 17682 line |
| 40130 | IL23R | NM_144701.2 | 1474 | cccgcaaaactcgctattcgaca | 248918 | - | see also 17683 line |
| 40131 | IL23R | NM_144701.2 | 1481 | aactcgctattgacaatactac | 248919 | - | see also 17683 line |
| 40132 | IMPG1 | NM_001563.2 | 1797 | tacagtatatccaccactagttct | 248963 | - | see also 17684 line |
| 40133 | IMPG1 | NM_001563.2 | 1035 | tgcaacttacggccatcttaag | 248949 | - | see also 17684 line |
| 40134 | ITGA2B | NM_000419.2 | 1092 | gccgaagtgggcgtgtgtattt | 249011 | - | see also 17685 line |
| 40135 | ITGA2B | NM_000419.2 | 856 | tcgacggggatctcaacactaca | 249004 | - | see also 17685 line |
| 40136 | ITGA3 | NM_002204.1 | 2359 | ctgcttagcatggtaaatcacc | 249036 | - | see also 17686 line |
| 40137 | ITGA3 | NM_002204.1 | 756 | agcgcaaggagtgggacttatct | 249024 | - | see also 17686 line |
| 40138 | ITGA4 | NM_000885.2 | 3185 | aacgactcacatgtcaaactac | 249109 | - | see also 576 line |
| 40139 | ITGA4 | NM_000885.2 | 3486 | gaggttaagctgactgttcatgg | 249121 | - | see also 576 line |
| 40140 | ITGA5 | NM_002205.1 | 2836 | tccgagtctgggccaagactttc | 249173 | - | see also 17688 line |
| 40141 | ITGA5 | NM_002205.1 | 2276 | ccctcatcctccggacactaaga | 249167 | - | see also 17688 line |
| 40142 | ITGA6 | NM_000210.1 | 426 | tgcacgccgatcgagtttgataa | 249180 | - | see also 17689 line |
| 40143 | ITGA6 | NM_000210.1 | 432 | cggatcgagtttgataacgatgc | 249183 | - | see also 17689 line |
| 40144 | ITGA7 | NM_002206.1 | 2549 | tgcacgagccgrtgtcttcattg | 249294 | - | see also 1407 line |
| 40145 | ITGA7 | NM_002206.1 | 930 | ttggccctcaatagctacttagg | 249279 | - | see also 1407 line |
| 40146 | ITGA9 | NM_002207.1 | 1759 | atcagccgatcgtgtttgaagc | 249326 | - | see also 17691 line |
| 40147 | ITGA9 | NM_002207.1 | 868 | ggcatcggcaaggtttatatttt | 249311 | - | see also 17691 line |
| 40148 | ITGB2 | NM_000211.1 | 1853 | gccggtgccgtgcaacgtatgc | 249354 | - | see also 17692 line |
| 40149 | ITGB2 | NM_000211.1 | 447 | cccatcgacctgtactatctga | 249347 | - | see also 17692 line |
| 40150 | ITGB3 | NM_000212.1 | 2344 | cacgtctacttcaccaatatca | 249392 | - | see also 161 line |
| 40151 | ITGB3 | NM_000212.1 | 846 | atgccaagactcatatagcattg | 249377 | - | see also 161 line |
| 40152 | ITGB4 | NM_000213.2 | 3968 | aacgatgcaaccgaccctattgg | 249415 | - | see also 17694 line |

Figure 46

| | | | | | | | | |
|---|---|---|---|---|---|---|---|---|
| 40153 | ITGB4 | NM_000213.2 | 521 | cccggtgaggagcggcattttga | 249393 | - | see also 17694 line | |
| 40154 | ITGB4 | NM_000213.2 | 674 | ctcaccagcgactacactattgg | 249395 | - | see also 17694 line | |
| 40155 | ITGB5 | NM_002213.3 | 891 | gaccaatccgtgcattggttaca | 249434 | - | see also 17695 line | |
| 40156 | ITGB5 | NM_002213.3 | 1420 | tacaatagtatccggtctaaagt | 249451 | - | see also 17695 line | |
| 40157 | ITGB5 | NM_002213.3 | 359 | ggctcgcaggtctcaacatatgc | 249426 | - | see also 17695 line | |
| 40158 | ITGB6 | NM_000888.3 | 596 | gccaacccttgcagtagtattcc | 249478 | - | see also 578 line | |
| 40159 | ITGB6 | NM_000888.3 | 604 | ttgcagtagtattccatacttct | 249482 | - | see also 578 line | |
| 40160 | ITGB7 | NM_000889.1 | 714 | ctgtgcgcattggttttggttcc | 249523 | - | see also 17697 line | |
| 40161 | ITGB7 | NM_000889.1 | 1992 | gtgggcatggacgcctgcaaatgc | 249539 | - | see also 17697 line | |
| 40162 | ITGB8 | NM_002214.1 | 1243 | ttggctcatacgttgataaaaca | 249567 | - | see also 17698 line | |
| 40163 | ITGB8 | NM_002214.1 | 1182 | tccgttggaaacgatttatctag | 249562 | - | see also 17698 line | |
| 40164 | ITPR3 | NM_002224.1 | 6173 | cacgtgaagtgggccataacatc | 249656 | - | see also 17699 line | |
| 40165 | ITPR3 | NM_002224.1 | 6600 | ccgcagcatgccgctgatctact | 249663 | - | see also 17699 line | |
| 40166 | KDELR3 | NM_006855.2 | 392 | atgggaaattccgtaaaacttttt | 249675 | - | see also 17700 line | |
| 40167 | KDELR3 | NM_006855.2 | 698 | tcgtgtctggagtagtacaaacc | 249682 | - | see also 17700 line | |
| 40168 | KIAA1985 | NM_024577.2 | 180 | aactgtatcgagtgagtgtatag | 249686 | - | see also 17701 line | |
| 40169 | KIAA1985 | NM_024577.2 | 359 | aggaggtgcgcatgctgtttaag | 249691 | - | see also 17701 line | |
| 40170 | KIR2DL5 | NM_020535.1 | 205 | ctctcgtcttgggtttaccatct | 249733 | - | receptor_acitivity | - | - |
| 40171 | KIR2DL5 | NM_020535.1 | 351 | acccacgctcccccattgagtgg | 249734 | - | see also 40170 line | |
| 40172 | LILRB2 | NM_005874.1 | 464 | ggcgatatggctgtcagtattac | 249736 | - | receptor_acitivity | - | - |
| 40173 | LIMR | NM_018113.1 | 607 | ctccctgcctcggaactactaca | 249739 | - | see also 7259 line | |
| 40174 | LIMR | NM_018113.1 | 1083 | cccgcaggatctgtaatcctact | 249746 | - | see also 7259 line | |
| 40175 | LOC143458 | NM_174902.2 | 228 | gccccaaggctaagtcgaaatgt | 249752 | - | see also 17703 line | |
| 40176 | LOC143458 | NM_174902.2 | 230 | cccaaggctaagtcgaaatgtgg | 249753 | - | see also 17703 line | |
| 40177 | LOC143458 | NM_174902.2 | 728 | gtcacctacaacgtcaataatgg | 249758 | - | see also 17703 line | |
| 40178 | LOC158435 | NM_138497.1 | 610 | cacaaactcgtctcctaatgaag | 249762 | - | see also 17704 line | |
| 40179 | LOC158435 | NM_138497.1 | 616 | ctcgtctcctaatgaagctaacc | 249763 | - | see also 17704 line | |
| 40180 | LRP3 | NM_002333.1 | 458 | ggcgaccgtggtgacatgattac | 249766 | - | see also 17705 line | |
| 40181 | LRP3 | NM_002333.1 | 381 | agcggcgtggggtcatctacagc | 249764 | - | see also 17705 line | |
| 40182 | LRP5 | NM_002335.1 | 4338 | cccgcacgtgcccctcaatttca | 249800 | - | see also 17706 line | |
| 40183 | LRP5 | NM_002335.1 | 1191 | cccgctagagggctatgtctact | 249780 | - | see also 17706 line | |
| 40184 | LRP5 | NM_002335.1 | 2601 | gacgcagtacagcgattatatct | 249784 | - | see also 17706 line | |
| 40185 | MAML2 | NM_032427.1 | 2792 | ggcagcggccagtcgaaagtaat | 249825 | - | see also 17707 line | |

Figure 46

| 40186 | MAML2 | NM_032427.1 | 3617 | ctggctgacgcggagaaaattgc | 249829 | - | see also 17707 line |
|---|---|---|---|---|---|---|---|
| 40187 | MCC | NM_002387.1 | 2394 | acgccattcgtcgagaaaagaag | 249885 | - | see also 17708 line |
| 40188 | MCC | NM_002387.1 | 1889 | tgcgacaccgagttcactaaaga | 249882 | - | see also 17708 line |
| 40189 | MCP | NM_002389.3 | 940 | ctcgatggcagcgacacaattgt | 249925 | - | see also 17709 line |
| 40190 | MCP | NM_002389.3 | 607 | agcggtaagcccccaatatgtga | 249912 | - | see also 17709 line |
| 40191 | MCP | NM_002389.3 | 497 | atgggacttacgagtttggttat | 249906 | - | see also 17709 line |
| 40192 | MED6 | NM_005466.1 | 331 | cactagctgattactatatcatt | 249951 | - | see also 3809 line |
| 40193 | MED6 | NM_005466.1 | 57 | gcggtggatatccgagacaatct | 249940 | - | see also 3809 line |
| 40194 | MGC33974 | NM_153350.2 | 824 | cgcaggcctcgaggttatgcttg | 249959 | - | see also 17711 line |
| 40195 | MGC33974 | NM_153350.2 | 1583 | cacccccgagctcttcaagtatt | 249961 | - | see also 17711 line |
| 40196 | MGC4766 | NM_031451.3 | 1097 | aactcgatgctatcaaggaaaac | 249978 | - | see also 17712 line |
| 40197 | MGC4766 | NM_031451.3 | 727 | cagatccagccaatatgtttaac | 249971 | - | see also 17712 line |
| 40198 | MIP-T3 | NM_015650.2 | 1256 | acggcgatccaaaaattcagtgg | 249998 | - | see also 17713 line |
| 40199 | MIP-T3 | NM_015650.2 | 382 | acgccgagatgaagtctgataat | 249981 | - | see also 17713 line |
| 40200 | MMD | NM_012329.1 | 764 | accgaagtcctacggactttatg | 250037 | - | see also 5280 line |
| 40201 | MMD | NM_012329.1 | 95 | atcgattccagcggttcatgaac | 250014 | - | see also 5280 line |
| 40202 | MPRA | NM_178422.2 | 750 | tagtcctgtggtgcatcgtatct | 250048 | - | see also 17715 line |
| 40203 | MPRA | NM_178422.2 | 988 | gaggcccgacggcccatctatga | 250050 | - | see also 17715 line |
| 40204 | MS4A4A | NM_024021.2 | 523 | cagggatcttaatcaacacattt | 250061 | - | see also 17716 line |
| 40205 | MS4A4A | NM_024021.2 | 582 | aactactatggcaactcaaataa | 250064 | - | see also 17716 line |
| 40206 | MS4A5 | NM_023945.2 | 174 | gccacgacctttcaactcaaag | 250074 | - | see also 17717 line |
| 40207 | MS4A5 | NM_023945.2 | 524 | ttgtggttattctcaccaaaata | 250081 | - | see also 17717 line |
| 40208 | MS4A5 | NM_023945.2 | 432 | ttgagccgaataatgaattttct | 250079 | - | see also 17717 line |
| 40209 | MS4A6A | NM_022349.2 | 354 | aacatctacacgcagaaatcaaa | 250085 | - | see also 17718 line |
| 40210 | MS4A6A | NM_022349.2 | 524 | atcatctctggctctctatcaat | 250087 | - | see also 17718 line |
| 40211 | MS4A6E | NM_139249.2 | 266 | gtgtaagttggacgaaaaggata | 250094 | - | receptor_acitivity、 signal_transduction - - |
| 40212 | MS4A6E | NM_139249.2 | 270 | aagttggacgaaaaggatatacc | 250095 | - | see also 40211 line |
| 40213 | MS4A6E | NM_139249.2 | 231 | gtcaacccggctgcattaaatcc | 250093 | - | see also 40211 line |
| 40214 | MS4A7 | NM_021201.3 | 623 | gccttattcggagtactattatc | 250109 | - | see also 17719 line |
| 40215 | MS4A7 | NM_021201.3 | 629 | ttcggagtactattatccaatat | 250110 | - | see also 17719 line |
| 40216 | MS4A8B | NM_031457.1 | 702 | gcctaccccgactattatcctta | 250132 | - | see also 17720 line |
| 40217 | MS4A8B | NM_031457.1 | 295 | ctcacgtgcccctgtatccaaac | 250121 | - | see also 17720 line |
| 40218 | NCR1 | NM_004829.3 | 259 | ccccctgagcggattaacaaagt | 250140 | - | see also 17722 line |
| 40219 | NCR1 | NM_004829.3 | 258 | accccctgagcggattaacaaag | 250139 | - | see also 17722 line |

Figure 46

| | | | | | | | | |
|---|---|---|---|---|---|---|---|---|
| 40220 | NCR1 | NM_004829.3 | 726 | cacttgggcacctacctttaa | | 250147 | - | see also 17722 line |
| 40221 | NCR3 | NM_147130.1 | 732 | gggcagcaacgtcattaccagg | | 250152 | - | see also 17723 line |
| 40222 | NCR3 | NM_147130.1 | 275 | tgctgttgctcatcttgatcatg | | 250150 | - | see also 17723 line |
| 40223 | NECL1 | NM_021189.2 | 466 | agcccttgagataatcgaattc | | 250154 | - | see also 17724 line |
| 40224 | NECL1 | NM_021189.2 | 467 | gcccttcgagataatcgaattca | | 250155 | - | see also 17724 line |
| 40225 | NGRH1 | NM_178570.1 | 687 | caccatcctcacctgttcaaca | | 250167 | - | receptor_acitivity |
| 40226 | NGRH2 | NM_178568.1 | 921 | agcgtcccgcaccagatcaagt | | 250170 | - | see also 17725 line |
| 40227 | NGRH2 | NM_178568.1 | 115 | agctgccaggcgcacaacttgc | | 250168 | - | see also 17725 line |
| 40228 | NISCH | NM_007184.1 | 652 | tgccgttcgacctatcaatattc | | 250185 | - | see also 17726 line |
| 40229 | NISCH | NM_007184.1 | 3962 | tagtcggtcaagttacctacc | | 250213 | - | see also 17726 line |
| 40230 | NKIR | NM_139018.2 | 828 | tgccgaaggaggacatttcctat | | 250218 | - | receptor_acitivity |
| 40231 | NKIR | NM_139018.2 | 419 | tactggtgtggaattgagaaaac | | 250215 | - | see also 40230 line |
| 40232 | NKIR | NM_139018.2 | 829 | gccgaaggaggacatttcctatg | | 250219 | - | see also 40230 line |
| 40233 | NPTXR | NM_014293.2 | 1047 | tcccatcgtaacaactacatg | | 250221 | - | receptor_acitivity |
| 40234 | NPTXR | NM_014293.2 | 1597 | ggcctttgggggtgcaacaaagg | | 250223 | - | see also 40233 line |
| 40235 | NPTXR | NM_014293.2 | 1436 | accctgggtcgccggtttgatgc | | 250222 | - | see also 40233 line |
| 40236 | OGFRL1 | NM_024576.2 | 1020 | tgcggttcgccagaaaacactac | | 250244 | - | see also 17727 line |
| 40237 | OGFRL1 | NM_024576.2 | 1063 | ctgggacgccctcgaaaagaac | | 250248 | - | see also 17727 line |
| 40238 | OSCAR | NM_130771.1 | 479 | tccagccaagggggaatttacc | | 250262 | - | see also 17728 line |
| 40239 | OSCAR | NM_130771.1 | 478 | ctccagcccaaggggaatttac | | 250261 | - | see also 17728 line |
| 40240 | PILRA | NM_013439.2 | 398 | cacagctccgacgtgagaatat | | 250264 | - | see also 17729 line |
| 40241 | PILRA | NM_013439.2 | 935 | cacagaggccatatgagaata | | 250267 | - | see also 17729 line |
| 40242 | PILRB | NM_013440.3 | 3077 | tggctgtcgctgtctcaaaact | | 250270 | - | receptor_acitivity, kinase_related |
| 40243 | PKHD1 | NM_138694.2 | 685 | cccatcgttcaccaagtttatcc | | 250285 | - | see also 17730 line |
| 40244 | PKHD1 | NM_138694.2 | 9152 | ttcagcctgacgtatcatgtagg | | 250470 | - | see also 17730 line |
| 40245 | PLXDC1 | NM_020405.3 | 846 | gcctatcggatgcttcatgatt | | 250553 | - | see also 7865 line |
| 40246 | PLXDC2 | NM_032812.7 | 1551 | accgagtagagctacaaatgtca | | 250581 | - | see also 17732 line |
| 40247 | PLXDC2 | NM_032812.7 | 664 | ggccgctgcaggagttatgttac | | 250560 | - | see also 17732 line |
| 40248 | PLXNB3 | NM_005393.1 | 4872 | gaccctatcggacgaagacttga | | 250609 | - | see also 17733 line |
| 40249 | PLXNB3 | NM_005393.1 | 248 | atcgcttctccgcacctaatacc | | 250596 | - | see also 17733 line |
| 40250 | PLXND1 | NM_015103.1 | 3811 | cacgagtaccgggtcaagatagg | | 250632 | - | see also 17734 line |
| 40251 | PLXND1 | NM_015103.1 | 5718 | gccgctcagcgagcaagagatga | | 250641 | - | see also 17734 line |
| 40252 | PLXND1 | NM_015103.1 | 2645 | tcccgctcagcctccaactaaag | | 250625 | - | see also 17734 line |
| 40253 | PORIMIN | NM_052932.1 | 742 | ggcattcggtatcgaaccataga | | 250658 | - | see also 17735 line |
| 40254 | PORIMIN | NM_052932.1 | 748 | cggtatcgaaccatagatgaaca | | 250660 | - | see also 17735 line |
| 40255 | PPFIA1 | NM_003626.2 | 337 | agggaccgccttcttgatacact | | 250662 | - | see also 17736 line |

Figure 46

| 40256 | PPFIA1 | NM_003626.2 | 460 | caggagttcgcagcacttactaa | 250665 | - | see also 17736 line | | |
|---|---|---|---|---|---|---|---|---|---|
| 40257 | PROCR | NM_006404.3 | 215 | ctgtctggctgggccttttgtag | 250721 | - | see also 17737 line | | |
| 40258 | PROCR | NM_006404.3 | 746 | cagtatgtgcagaaacatatttc | 250722 | - | see also 17737 line | | |
| 40259 | PRV1 | NM_020406.1 | 670 | atcgggggaccaccattatgaca | 250726 | - | see also 17738 line | | |
| 40260 | PRV1 | NM_020406.1 | 154 | ggcaatggacccctaagaacacc | 250725 | - | see also 17738 line | | |
| 40261 | PSK-1 | NM_012410.1 | 1108 | cccctatccccgtgatctatga | 250736 | - | see also 17739 line | | |
| 40262 | PSK-1 | NM_012410.1 | 2493 | cagtggcgtttacatctactaca | 250750 | - | see also 17739 line | | |
| 40263 | PTDSR | NM_015167.1 | 824 | tcctcaatctcgacactactatc | 250780 | - | see also 6261 line | | |
| 40264 | PTDSR | NM_015167.1 | 683 | agcaagacgaagctattacctgg | 250772 | - | see also 6261 line | | |
| 40265 | PVR | NM_006505.2 | 435 | gtccaaacggctggaattcgtgg | 250781 | - | see also 17740 line | | |
| 40266 | PVR | NM_006505.2 | 989 | agcccacaggctataattggagc | 250785 | - | see also 17740 line | | |
| 40267 | RAMP1 | NM_005855.1 | 107 | tgcctgccaggaggctaactacg | 250792 | - | receptor_acitivity、sig nal_transduction、gpcr | receptor | - |
| 40268 | RAMP2 | NM_005854.1 | 315 | tggtgcgactgggccatgattag | 250799 | - | see also 17741 line | | |
| 40269 | RAMP2 | NM_005854.1 | 544 | tcctcatcactcttgtagtatgg | 250800 | - | see also 17741 line | | |
| 40270 | RAMP3 | NM_005856.1 | 207 | tccgagttcatcgtgtactatga | 250802 | - | see also 17742 line | | |
| 40271 | RAMP3 | NM_005856.1 | 206 | gtccgagttcatcgtgtactatg | 250801 | - | see also 17742 line | | |
| 40272 | RAMP3 | NM_005856.1 | 382 | ttctcatcccgctgatcgttata | 250805 | - | see also 17742 line | | |
| 40273 | RAP80 | NM_016290.2 | 2114 | ttcacacgtcgtgacttaaatga | 250839 | - | see also 17743 line | | |
| 40274 | RAP80 | NM_016290.2 | 1624 | cccgctatgtgaccaatgctttc | 250829 | - | see also 17743 line | | |
| 40275 | RAPSN | NM_005055.3 | 1201 | ctgtctgagcgagagcatttacc | 250848 | - | see also 17744 line | | |
| 40276 | RAPSN | NM_005055.3 | 665 | cgctatgcccacaacaatgatga | 250845 | - | see also 17744 line | | |
| 40277 | RHBDF1 | NM_022450.2 | 930 | tccacatacccggatgaagtttt | 250854 | - | see also 17745 line | | |
| 40278 | RHBDF1 | NM_022450.2 | 929 | gtccacatacccggatgaagttt | 250853 | - | see also 17745 line | | |
| 40279 | RIF1 | NM_018372.2 | 1274 | gtgggacgcgatccaaaaatatg | 250880 | - | see also 17746 line | | |
| 40280 | RIF1 | NM_018372.2 | 1275 | tgggacgcgatccaaaaatatgc | 250881 | - | see also 17746 line | | |
| 40281 | RILP | NM_031430.1 | 1268 | ccccggagtccaaaatacagagt | 250913 | - | see also 17747 line | | |
| 40282 | RNF139 | NM_007218.3 | 734 | atcgttctacagctaacatttgg | 250929 | - | see also 5026 line | | |
| 40283 | RNF139 | NM_007218.3 | 1724 | gtctactacgttcgttcaacagg | 250966 | - | see also 5026 line | | |
| 40284 | RTN4R | NM_023004.4 | 1191 | ccgcctcatgacactctatctgt | 250990 | - | see also 17749 line | | |
| 40285 | RTN4R | NM_023004.4 | 1637 | gcccacggcacatcaatgactca | 250996 | - | see also 17749 line | | |
| 40286 | RYR1 | NM_000540.1 | 11065 | cacgcaccgggcatgtaacatgt | 251083 | - | see also 17750 line | | |
| 40287 | RYR1 | NM_000540.1 | 280 | cactagcaacgcgcagaatgtgc | 250997 | - | see also 17750 line | | |
| 40288 | SCARB1 | NM_005505.3 | 676 | gacaagttcggattatttgctga | 251123 | - | see also 17751 line | | |
| 40289 | SCARB1 | NM_005505.3 | 566 | tcggcgaacgtgccttcatgaac | 251119 | - | see also 17751 line | | |
| 40290 | SCARB2 | NM_005506.2 | 905 | atgttttcaggcccgatatctct | 251148 | - | see also 3847 line | | |

Figure 46

| 40291 | SCARB2 | NM_005506.2 | 1607 | gtcgactgaagtctatgattaac | 251180 | - | see also 3847 line |
|---|---|---|---|---|---|---|---|
| 40292 | SCARF2 | NM_153334.3 | 1175 | accggtgcgagaccaagtgtagc | 251185 | - | see also 17753 line |
| 40293 | SCARF2 | NM_153334.3 | 2213 | aacggacgcccagcgacaaatcg | 251193 | - | see also 17753 line |
| 40294 | SDFR1 | NM_012428.1 | 784 | taccactgcgtatatcactttgt | 251207 | - | see also 17754 line |
| 40295 | SDFR1 | NM_012428.1 | 778 | ggcgaataccactgcgtatatca | 251206 | - | see also 17754 line |
| 40296 | SEMA4B | NM_020210.2 | 1528 | cccagaccgcgtgctgaacttcc | 251233 | - | see also 17755 line |
| 40297 | SEMA4B | NM_020210.2 | 666 | agcgcgactgtcaaaactacatc | 251223 | - | see also 17755 line |
| 40298 | SEMA4C | NM_017789.3 | 209 | aaccttgtgccgcgtaagacagt | 251244 | - | see also 17756 line |
| 40299 | SEMA4C | NM_017789.3 | 2613 | accccgaggagtcatcagtatga | 251263 | - | see also 17756 line |
| 40300 | SEMA4D | NM_006378.2 | 926 | cagcgccacttccctttacgtgt | 251264 | - | see also 17757 line |
| 40301 | SEMA4D | NM_006378.2 | 1778 | ctcggtaaccccaatagacaaca | 251278 | - | see also 17757 line |
| 40302 | SEMA4F | NM_004263.2 | 888 | ctcatacgagcgcattaaagtcc | 251311 | - | see also 2862 line |
| 40303 | SEMA4F | NM_004263.2 | 962 | agcagagatggacgacgtttttg | 251312 | - | see also 2862 line |
| 40304 | SEMA4G | NM_017893.2 | 1505 | ctggctcgtgtatcacagattca | 251341 | - | see also 17759 line |
| 40305 | SEMA4G | NM_017893.2 | 1639 | ctgctgctcaagcgcaacatacg | 251347 | - | see also 17759 line |
| 40306 | SEMA5A | NM_003966.1 | 3592 | caccctgctcgtctatacttact | 251397 | - | see also 17760 line |
| 40307 | SEMA5A | NM_003966.1 | 1879 | agcgctcgtccacatcatctatt | 251380 | - | see also 17760 line |
| 40308 | SEMA5B | NM_018987.1 | 2098 | gtcgagctcgatcctgtgattcc | 251411 | - | see also 7642 line |
| 40309 | SEMA5B | NM_018987.1 | 2317 | gccgggaggaacggttctgtaat | 251412 | - | see also 7642 line |
| 40310 | SH120 | NM_016334.1 | 782 | ttctggggaatgataaaaagtgt | 251422 | - | receptor_acitivity、kin ase_related \| - \| - |
| 40311 | SH120 | NM_016334.1 | 775 | atcaggtttctggggaatgataa | 251421 | - | see also 40310 line |
| 40312 | SLAMF6 | NM_052931.3 | 859 | tccgcaaggaacctagagtatgt | 251441 | - | see also 17761 line |
| 40313 | SLAMF6 | NM_052931.3 | 858 | gtccgcaaggaacctagagtatg | 251440 | - | see also 17761 line |
| 40314 | SLAMF6 | NM_052931.3 | 276 | tccagaaatccacgtgactaatc | 251428 | - | see also 17761 line |
| 40315 | SLAMF7 | NM_021181.3 | 818 | gtcgggaaactcctaacatatgc | 251465 | - | see also 17762 line |
| 40316 | SLAMF7 | NM_021181.3 | 908 | atccagcaaatacggtttactcc | 251470 | - | see also 17762 line |
| 40317 | SLC1A5 | NM_005628.1 | 1985 | gtcctgtaccgtcctcaatgtag | 251481 | - | see also 17763 line |
| 40318 | SLC1A5 | NM_005628.1 | 1986 | tcctgtaccgtcctcaatgtaga | 251482 | - | see also 17763 line |
| 40319 | SLC20A1 | NM_005415.3 | 1675 | aacccttaaggcgcaataatagc | 251514 | - | see also 3781 line |
| 40320 | SLC20A1 | NM_005415.3 | 1579 | atcacaccgtgcataaggattcc | 251511 | - | see also 3781 line |
| 40321 | SLC20A1 | NM_005415.3 | 1684 | ggcgcaataatagctatacttcc | 251516 | - | see also 3781 line |
| 40322 | SLC20A2 | NM_006749.3 | 535 | accaccggctccgtgttactagg | 251538 | - | see also 17765 line |
| 40323 | SLC20A2 | NM_006749.3 | 748 | ctggtcgcaatcggtaccaaagg | 251546 | - | see also 17765 line |
| 40324 | SPAP1 | NM_030764.2 | 1028 | cccccaatcttgtaccaatttta | 251587 | - | see also 17766 line |
| 40325 | SPAP1 | NM_030764.2 | 1027 | tcccccaatcttgtaccaatttt | 251586 | - | see also 17766 line |

Figure 46

| 40326 | TGFBRAP1 | NM_004257.3 | 848 | agcgctcgatgacgaattcatca | 251603 | - | see also 17767 line | | |
|---|---|---|---|---|---|---|---|---|---|
| 40327 | TGFBRAP1 | NM_004257.3 | 2438 | caggtccgaaaacttaatctaca | 251629 | - | see also 17767 line | | |
| 40328 | TIP47 | NM_005817.2 | 1324 | ccctttgcccctggaatcactga | 251633 | - | see also 17768 line | | |
| 40329 | TNFRSF10D | NM_003840.2 | 705 | ctccctatcactaccttatcatc | 251639 | - | see also 17769 line | | |
| 40330 | TNFRSF10D | NM_003840.2 | 1183 | gggctccgaaaagctcttttatg | 251646 | - | see also 17769 line | | |
| 40331 | TNFRSF11A | NM_003839.2 | 1416 | ctcgtgggttccccaaaacgtgg | 251666 | - | see also 17770 line | | |
| 40332 | TNFRSF11A | NM_003839.2 | 166 | tgggacggtgctgtaacaaatgt | 251650 | - | see also 17770 line | | |
| 40333 | TNFRSF12A | NM_016639.1 | 350 | gccgcaggagagagaagttcacc | 251669 | - | receptor_acitivity、apoptosis | receptor | - |
| 40334 | TNFRSF13B | NM_012452.2 | 409 | ctcgggaaggtaccaaggattgg | 251675 | - | see also 17771 line | | |
| 40335 | TNFRSF13B | NM_012452.2 | 301 | ccctaagcaatgtgcatacttct | 251672 | - | see also 17771 line | | |
| 40336 | TNFRSF17 | NM_001192.2 | 690 | accacgaaaacgaatgactattg | 251690 | - | see also 17772 line | | |
| 40337 | TNFRSF17 | NM_001192.2 | 319 | ctctaacatgtcagcgttattgt | 251677 | - | see also 17772 line | | |
| 40338 | TNFRSF19L | NM_032871.3 | 1418 | aagtggcggaagccgtacaaagt | 251696 | - | see also 17773 line | | |
| 40339 | TNFRSF19L | NM_032871.3 | 1420 | gtggcggaagccgtacaaagtgg | 251697 | - | see also 17773 line | | |
| 40340 | TNFRSF21 | NM_014452.3 | 2304 | aactagaccggctattcgaaatt | 251719 | - | see also 17774 line | | |
| 40341 | TNFRSF21 | NM_014452.3 | 2009 | cccaacgcgaaacttgagaattc | 251712 | - | see also 17774 line | | |
| 40342 | TNFRSF21 | NM_014452.3 | 1880 | cgccagcaccggagaaacgatgt | 251708 | - | see also 17774 line | | |
| 40343 | TNFRSF9 | NM_001561.4 | 494 | tgggacatttaacgatcagaaac | 251735 | - | see also 17775 line | | |
| 40344 | TNFRSF9 | NM_001561.4 | 798 | ttcaaacaaccatttatgagacc | 251739 | - | see also 17775 line | | |
| 40345 | TNPO3 | NM_012470.1 | 1403 | gcctcccggagaccgtacatacg | 251792 | - | see also 17776 line | | |
| 40346 | TNPO3 | NM_012470.1 | 845 | tggccgtggcacgtgaagattta | 251770 | - | see also 17776 line | | |
| 40347 | TOMM22 | NM_020243.3 | 41 | accccagtccccggacgaattgc | 251830 | - | receptor_acitivity | - | - |
| 40348 | TOMM22 | NM_020243.3 | 192 | gccggagccactttgatctttc | 251831 | - | see also 40347 line | | |
| 40349 | TRAP240 | NM_005121.1 | 4920 | agcacgatggatcgggataaagt | 251951 | - | see also 17777 line | | |
| 40350 | TRAP240 | NM_005121.1 | 2699 | tagtataggagcgcagttcaaaa | 251898 | - | see also 17777 line | | |
| 40351 | TREM1 | NM_018643.2 | 313 | gaggatcatactagaagactacc | 252015 | - | see also 17778 line | | |
| 40352 | TREM1 | NM_018643.2 | 661 | cagggttccggtgttcaacattg | 252022 | - | see also 17778 line | | |

Figure 46

| | | | | | | | |
|---|---|---|---|---|---|---|---|
| 40353 | TREM2 | NM_018965.1 | 374 | accattacgctgcggaatctaca | 252027 | - | see also 17779 line |
| 40354 | TREM2 | NM_018965.1 | 360 | tgggtggcactccaccattacg | 252026 | - | see also 17779 line |
| 40355 | TREM5 | NM_174892.1 | 212 | gacggttcaatgccactataagc | 252031 | - | see also 17780 line |
| 40356 | TREM5 | NM_174892.1 | 689 | ctccgaacctctgactaaagaca | 252044 | - | see also 17780 line |
| 40357 | TRIP3 | NM_004773.1 | 173 | aactcgtcctgttgagaaaaaa | 252045 | - | see also 17781 line |
| 40358 | TRIP3 | NM_004773.1 | 335 | aacattaagaagcttattgctca | 252048 | - | see also 17781 line |
| 40359 | TRIP3 | NM_004773.1 | 254 | ctctatagctgatttctcaata | 252046 | - | see also 17781 line |
| 40360 | TRPC4 | NM_016179.1 | 482 | agtcatcgaactactcttaagc | 252056 | - | see also 6708 line |
| 40361 | TRPC4 | NM_016179.1 | 2744 | ttgtgaccgatatcaaaaacttt | 252138 | - | see also 6708 line |
| 40362 | TRPC7 | NM_020389.1 | 1949 | acgttctctacggcgtttataac | 252186 | - | see also 7861 line |
| 40363 | TRPC7 | NM_020389.1 | 1948 | tacgttcttacgcgcgtttataa | 252185 | - | see also 7861 line |
| 40364 | TRPM8 | NM_024080.3 | 1689 | tccaacgacgtgtctctattact | 252239 | - | see also 8586 line |
| 40365 | TRPM8 | NM_024080.3 | 3272 | atgagccatcgatttagcacaact | 252266 | - | see also 8586 line |
| 40366 | TRPV2 | NM_016113.3 | 590 | gaccgagatcggcttcaatgc | 252270 | - | see also 17785 line |
| 40367 | TRPV2 | NM_016113.3 | 571 | gccggatccaaacgatttgacc | 252269 | - | see also 17785 line |
| 40368 | TRPV3 | NM_145068.2 | 1083 | accaaacagaaggcttcactc | 252296 | - | see also 9774 line |
| 40369 | TRPV3 | NM_145068.2 | 2551 | gacgcacgtctcttccttaacg | 252307 | - | see also 9774 line |
| 40370 | TRPV4 | NM_021625.3 | 269 | agggatgggcgaccaaatctgc | 252313 | - | see also 17787 line |
| 40371 | TRPV4 | NM_021625.3 | 773 | ctggcccttccgtgcatctact | 252321 | - | see also 17787 line |
| 40372 | TRPV5 | NM_019841.3 | 1862 | gcctccgttctatatcatttt | 252342 | - | see also 17788 line |
| 40373 | TRPV5 | NM_019841.3 | 554 | acctgtgacgttcgacaaagagg | 252330 | - | see also 17788 line |
| 40374 | UNC13B | NM_006377.2 | 230 | tgctctgcgtgcgcgttaaaagg | 252354 | - | see also 4469 line |
| 40375 | UNC13B | NM_006377.2 | 2437 | taccacgtgcagtatacatgtct | 252400 | - | see also 4469 line |
| 40376 | UNC5B | NM_170744.2 | 2628 | agccgaaaccgctaatgttcaag | 252446 | - | see also 17790 line |
| 40377 | UNC5B | NM_170744.2 | 2629 | gccgaaaccgtaatgttcaagg | 252447 | - | see also 17790 line |
| 40378 | WDR31 | NM_145241.1 | 1233 | tggtgacgccatctccttattgt | 252470 | - | see also 17791 line |
| 40379 | WDR31 | NM_145241.1 | 641 | cacaaccaaggcagcaattgtgt | 252462 | - | see also 17791 line |
| 40380 | ZYX | NM_003461.3 | 414 | cccccgatcgaggaatcatttcc | 252476 | - | see also 17792 line |
| 40381 | ZYX | NM_003461.3 | 413 | tcccccgatcgaggaatcatttc | 252475 | - | see also 17792 line |
| 40382 | ZYX | NM_003461.3 | 412 | ctccccgatcgaggaatcattt | 252474 | - | see also 17792 line |
| 40383 | APS | NM_020979.1 | 234 | tggactttgcgcacaagttctgc | 252488 | - | see also 17793 line |
| 40384 | APS | NM_020979.1 | 878 | atcccactgtcagccatcattga | 252489 | - | see also 17793 line |
| 40385 | ARHGAP4 | NM_001666.1 | 804 | cgctgctgcagtaactactacc | 252496 | - | see also 17794 line |
| 40386 | ARHGAP4 | NM_001666.1 | 803 | acgctgctgtcagtaactactac | 252495 | - | see also 17794 line |
| 40387 | ARHGAP6 | NM_001174.2 | 1834 | tgcgaatgacagggcctataaac | 252508 | - | see also 764 line |
| 40388 | ARHGAP6 | NM_001174.2 | 2084 | gacaatcagtctcgactactaga | 252513 | - | see also 764 line |
| 40389 | BLNK | NM_013314.2 | 511 | ttcgccagaggcgagtatataga | 252557 | - | see also 5395 line |

Figure 46

| | | | | | | | | | | |
|---|---|---|---|---|---|---|---|---|---|---|
| 40390 | BLNK | NM_013314.2 | 1342 | aagcgagtatataatattcctgt | 252577 | - | see also 5395 line | | | |
| 40391 | CRK | NM_005206.2 | 656 | tcccttacgtcgagaagtataga | 252588 | - | see also 3656 line | | | |
| 40392 | CRK | NM_005206.2 | 433 | gacactacaacgttgatagaacc | 252586 | - | see also 3656 line | | | |
| 40393 | CRK | NM_005206.2 | 355 | ccctccagactccgaataggaga | 252582 | - | see also 3656 line | | | |
| 40394 | GRAP | NM_006613.2 | 568 | ttccgccgtggcgacatcattga | 252596 | - | signal_transduction | SH3/SH2 adaptor protein | - | |
| 40395 | GRAP | NM_006613.2 | 453 | cgccaagaagcggcagatcttcc | 252595 | - | see also 40394 line | | | |
| 40396 | GRAP2 | NM_004810.2 | 998 | caccggcttgggcagtgaaatga | 252606 | - | see also 17798 line | | | |
| 40397 | GRAP2 | NM_004810.2 | 391 | aaggatatgtgcccaagaatttc | 252597 | - | see also 17798 line | | | |
| 40398 | GRB10 | NM_005311.2 | 1403 | caccacccgcacctaggattaga | 252618 | - | see also 17799 line | | | |
| 40399 | GRB10 | NM_005311.2 | 1939 | agcgttcagactcctcaagtatg | 252628 | - | see also 17799 line | | | |
| 40400 | GRB14 | NM_004490.1 | 1542 | gaccgcgattagattgcttaagt | 252668 | - | see also 17800 line | | | |
| 40401 | GRB14 | NM_004490.1 | 919 | gacataacggctcgagatgtttg | 252648 | - | see also 17800 line | | | |
| 40402 | GRB2 | NM_002086.2 | 362 | aagccatcgccaaatatgacttc | 252680 | - | see also 1348 line | | | |
| 40403 | GRB7 | NM_005310.1 | 588 | agctcgccacgtgtgtgaaatgc | 252694 | - | see also 17801 line | | | |
| 40404 | GRB7 | NM_005310.1 | 977 | tgcgccgatctggcctctattac | 252698 | - | see also 17801 line | | | |
| 40405 | LASP1 | NM_006148.1 | 384 | cacgcccgagctccagagaatca | 252711 | - | see also 17802 line | | | |
| 40406 | LASP1 | NM_006148.1 | 271 | gcggacacccggaaaaccttcg | 252710 | - | see also 17802 line | | | |
| 40407 | LASP1 | NM_006148.1 | 270 | ggcggacacccggaaaaccttc | 252709 | - | see also 17802 line | | | |
| 40408 | LAT | NM_014387.2 | 831 | ggctccagattacgagaatctgc | 252718 | - | see also 5645 line | | | |
| 40409 | LAT | NM_014387.2 | 724 | ggcagccgggagtatgtgaatgt | 252716 | - | see also 5645 line | | | |
| 40410 | RUSC1 | NM_014328.1 | 189 | gtcagcgtctccgttgataaaat | 252720 | - | see also 17803 line | | | |
| 40411 | RUSC1 | NM_014328.1 | 497 | tagccgcttccatgcctttatcc | 252723 | - | see also 17803 line | | | |
| 40412 | SCAP2 | NM_003930.3 | 551 | ttcattagcctcagaacgatatg | 252738 | - | see also 2689 line | | | |
| 40413 | SCAP2 | NM_003930.3 | 860 | ctctgctcctgataaacgtatat | 252746 | - | see also 2689 line | | | |
| 40414 | SCAP2 | NM_003930.3 | 723 | ctcagtaaaacggtattctatta | 252743 | - | see also 2689 line | | | |
| 40415 | SH2D1A | NM_002351.1 | 433 | tgctgtatcacggttacatttat | 252766 | - | see also 17805 line | | | |
| 40416 | SH2D1A | NM_002351.1 | 422 | ctgcctatgtgtgctgtatcacg | 252764 | - | see also 17805 line | | | |
| 40417 | SH2D3A | NM_005490.1 | 415 | accggctctggttcacagttata | 252774 | - | kinase_related、signal _transduction | - | | - |
| 40418 | SH2D3A | NM_005490.1 | 504 | ggcctctgcgacgcagctttagc | 252776 | - | see also 40417 line | | | |
| 40419 | SH2D3A | NM_005490.1 | 420 | ctctggttcacagttatatgaca | 252775 | - | see also 40417 line | | | |
| 40420 | SH2D3C | NM_005489.1 | 1387 | taggatactgggcgttaccaagg | 252784 | - | see also 17806 line | | | |
| 40421 | SH2D3C | NM_005489.1 | 158 | ctggactcatcgccagagaaact | 252782 | - | see also 17806 line | | | |
| 40422 | SH3BGR | NM_007341.1 | 193 | ttccaactgtcgaaatggttatc | 252785 | - | see also 17807 line | | | |
| 40423 | SH3BGR | NM_007341.1 | 239 | tgggtccatagcgattaggaaga | 252789 | - | see also 17807 line | | | |
| 40424 | SH3BGRL | NM_003022.1 | 111 | tggctctacagcgattaagaaga | 252797 | - | see also 2021 line | | | |

EP 1 752 536 A1

Figure 46

| | | | | | | | |
|---|---|---|---|---|---|---|---|
| 40425 | SH3BP2 | NM_003023.2 | 756 | ccgtacccacgacaatgaag | 252808 | - | see also 17809 line |
| 40426 | SH3BP2 | NM_003023.2 | 572 | agcaccgcacgtggttcttctg | 252805 | - | see also 17809 line |
| 40427 | SH3BP2 | NM_003023.2 | 1761 | gtgggacgaaacctctaacaaag | 252813 | - | see also 17809 line |
| 40428 | SH3BP5 | NM_004844.1 | 131 | atcagtccaccgatgatatcaac | 252820 | - | see also 17810 line |
| 40429 | SH3BP5 | NM_004844.1 | 632 | tgcagctcgagcaactgaaaaag | 252825 | - | see also 17810 line |
| 40430 | SHB | NM_003028.1 | 1426 | aagtgtccggtccagcataaag | 252836 | - | see also 17811 line |
| 40431 | SHB | NM_003028.1 | 1705 | aggggcttaagcctatcaaac | 252840 | - | see also 17811 line |
| 40432 | SLA | NM_006748.1 | 236 | cactggtcgagagagttacatcc | 252854 | - | see also 17812 line |
| 40433 | SLA | NM_006748.1 | 845 | ctcatcaccacttactttgagg | 252859 | - | see also 17812 line |
| 40434 | SNX9 | NM_016224.3 | 1293 | ctggcgggagtcatgatatttc | 252886 | - | see also 6729 line |
| 40435 | SNX9 | NM_016224.3 | 184 | aggctcggttatgtatgatttt | 252861 | - | see also 6729 line |
| 40436 | SNX9 | NM_016224.3 | 1294 | tggcgggagtcatgatatttcc | 252887 | - | see also 6729 line |
| 40437 | STAM | NM_003473.2 | 347 | aggattgttcggtcattatg | 252911 | - | see also 2349 line |
| 40438 | STAM | NM_003473.2 | 1583 | ctcaggccgcttacccaaataca | 252937 | - | see also 2349 line |
| 40439 | TP53BP2 | NM_005426.1 | 1285 | gtcattggataagcgtgttaatga | 252956 | - | see also 17815 line |
| 40440 | TP53BP2 | NM_005426.1 | 1169 | ctgagcttgatcgcctctataag | 252953 | - | see also 17815 line |
| 40441 | PAG | NM_018440.2 | 1347 | aacgactacgagcagcataagtga | 253026 | - | see also 17816 line |
| 40442 | PAG | NM_018440.2 | 1091 | atcgggcagtcgccttacagttc | 253021 | - | see also 17816 line |
| 40443 | SHC1 | NM_003029.3 | 601 | gccgagtatgtcgcctatgttgc | 253033 | - | see also 2031 line |
| 40444 | SHC1 | NM_003029.3 | 712 | ttcgagttgcgcttcaaacaata | 253036 | - | see also 2031 line |
| 40445 | SHC1 | NM_003029.3 | 194 | cccgccaacgggagctttgtcaat | 253027 | - | see also 2031 line |
| 40446 | TRIM | NM_016388.1 | 586 | ttctaagacaccttagtagaca | 253062 | - | see also 17818 line |
| 40447 | TRIM | NM_016388.1 | 573 | tagatgccagcgtttctagagacc | 253061 | - | see also 17818 line |
| 40448 | AKAP12 | NM_005100.2 | 4153 | ctgcagagtcagcgtaagtctcc | 253123 | - | see also 17819 line |
| 40449 | AKAP12 | NM_005100.2 | 3600 | cactgtcaagccgaaaccttag | 253113 | - | see also 17819 line |
| 40450 | AKAP3 | NM_006422.2 | 2424 | atgcctatcaagctatccataat | 253188 | - | see also 4498 line |
| 40451 | AKAP3 | NM_006422.2 | 395 | ttccaagatgttcgttcaaacc | 253140 | - | see also 4498 line |
| 40452 | AKAP4 | NM_003886.2 | 1026 | tctatatagcgaattatccaac | 253222 | - | see also 17821 line |
| 40453 | AKAP4 | NM_003886.2 | 2193 | agcaagcgaacgggcaatttata | 253245 | - | see also 17821 line |
| 40454 | AKAP4 | NM_003886.2 | 782 | tacgtcaacgactactctctct | 253217 | - | see also 17821 line |
| 40455 | AKAP6 | NM_004274.3 | 951 | tacggtagctgctgactctatct | 253273 | - | see also 17822 line |
| 40456 | AKAP6 | NM_004274.3 | 1395 | aagcaccccttcgctagatagatc | 253283 | - | see also 17822 line |
| 40457 | AKAP7 | NM_004842.2 | 274 | cccgatgacgctgaactagtaag | 253437 | - | see also 17823 line |
| 40458 | AKAP7 | NM_004842.2 | 273 | gcccgatgacgctgaactagtaa | 253436 | - | see also 17823 line |
| 40459 | PDCL | NM_005388.2 | 203 | aactgcagtactactatagcagc | 253463 | - | see also 3759 line |
| 40460 | CD19 | NM_001770.3 | 1409 | ctccaacgctgagtcttatgaga | 253489 | - | see also 17825 line |
| 40461 | CD19 | NM_001770.3 | 697 | accccaaggggcctaagtcattg | 253477 | - | see also 17825 line |

Figure 46

| 40462 | CD19 | NM_001770.3 | 864 | atcactgctcggccagtactatg | 253482 | - | see also 17825 line |
|---|---|---|---|---|---|---|---|
| 40463 | FLRT2 | NM_013231.2 | 1181 | ggccgtcagggaattaaatatga | 253523 | - | see also 17826 line |
| 40464 | FLRT2 | NM_013231.2 | 749 | gagcttggagcgtcttattgtgg | 253507 | - | see also 17826 line |
| 40465 | MADH1 | NM_005900.1 | 1703 | accaccgccaggatgttactagc | 253566 | - | see also 4110 line |
| 40466 | MADH1 | NM_005900.1 | 898 | ttccgtaacttaggacaaaatga | 253547 | - | see also 4110 line |
| 40467 | MADH5 | NM_005903.4 | 1313 | atcgtaattcgacaattgaaaac | 253604 | - | see also 4113 line |
| 40468 | MADH5 | NM_005903.4 | 396 | tccagcagtaaagcgattgttgg | 253571 | - | see also 4113 line |
| 40469 | MS4A1 | NM_021950.2 | 677 | cccatctacccaatactgttaca | 253623 | - | see also 17829 line |
| 40470 | MS4A1 | NM_021950.2 | 880 | aagtggttgggctaactgaaaca | 253629 | - | see also 17829 line |
| 40471 | NSMAF | NM_003580.2 | 1715 | cacgagtggattgatctaatatt | 253673 | - | see also 2376 line |
| 40472 | NSMAF | NM_003580.2 | 2308 | aagacgccaggacacgttaatgg | 253692 | - | see also 2376 line |
| 40473 | RGS14 | NM_006480.3 | 419 | cagcgcggaaaacgtgactttct | 253775 | - | see also 17831 line |
| 40474 | RGS14 | NM_006480.3 | 1020 | gggagcacggagggtgaaagtga | 253779 | - | see also 17831 line |
| 40475 | TBL3 | NM_006453.2 | 792 | gaggaccgtgcctgtgtttgaga | 253789 | - | see also 4524 line |
| 40476 | TBL3 | NM_006453.2 | 1301 | tccgtatctggagaatgaacaag | 253796 | - | see also 4524 line |
| 40477 | TIAM1 | NM_003253.1 | 5175 | agcgcaagcgaggaagtcatttg | 253871 | - | see also 17834 line |
| 40478 | TIAM1 | NM_003253.1 | 1506 | ctcctgtccaggcgatctaatgc | 253816 | - | see also 17834 line |
| 40479 | FST | NM_006350.2 | 383 | ccccggattgttccaacatcacc | 253875 | - | see also 17835 line |
| 40480 | FST | NM_006350.2 | 433 | gggaaaacctaccgcaatgaatg | 253876 | - | see also 17835 line |
| 40481 | ARHGEF1 | NM_004706.3 | 1030 | gccctctcgggaccggaatatcg | 253890 | - | see also 17836 line |
| 40482 | ARHGEF1 | NM_004706.3 | 454 | tcccaacgtcgcctttgaacttg | 253887 | - | see also 17836 line |
| 40483 | ARHGEF1 | NM_004706.3 | 455 | cccaacgtcgcctttgaacttga | 253888 | - | see also 17836 line |
| 40484 | ARHGEF11 | NM_014784.2 | 1623 | gacatacttccactatatggtga | 253902 | - | see also 17837 line |
| 40485 | ARHGEF11 | NM_014784.2 | 5108 | tacggggaactgcttttatgtca | 253943 | - | see also 17837 line |
| 40486 | ARHGEF12 | NM_015313.1 | 1380 | aggatctgcatcgccactatatc | 253981 | - | see also 6323 line |
| 40487 | ARHGEF12 | NM_015313.1 | 1036 | aacccgtatagcacctcatatta | 253971 | - | see also 6323 line |
| 40488 | AXIN1 | NM_003502.2 | 1072 | gagccagtcaacccctattatgt | 254091 | - | see also 17839 line |
| 40489 | AXIN1 | NM_003502.2 | 881 | ctggatacctgccgaccttaaat | 254086 | - | see also 17839 line |
| 40490 | AXIN2 | NM_004655.1 | 2564 | gacggtgctcccgatgtatgaag | 254124 | - | see also 17840 line |
| 40491 | AXIN2 | NM_004655.1 | 2562 | gagacggtgctcccgatgtatga | 254123 | - | see also 17840 line |
| 40492 | BCAR1 | NM_014567.1 | 964 | cccgttgctggaggtgtatgacg | 254130 | - | see also 17841 line |
| 40493 | BCAR1 | NM_014567.1 | 851 | gggccacaggacatctatgatgt | 254128 | - | see also 17841 line |
| 40494 | BSG | NM_001728.2 | 939 | ggccggtcagagctacacattga | 254132 | - | see also 17842 line |

Figure 46

| | | | | | | | |
|---|---|---|---|---|---|---|---|
| 40495 | BSG | NM_001728.2 | 941 | ccggtcagagctacacattgaga | 254133 | - | see also 17842 line |
| 40496 | CLTCL1 | NM_001835.1 | 3535 | gccgtgagtcctatatagagact | 254206 | - | see also 17843 line |
| 40497 | CLTCL1 | NM_001835.1 | 4242 | aacgtcgagctctgttacagagc | 254214 | - | see also 17843 line |
| 40498 | DIXDC1 | NM_033425.1 | 1699 | aaggaaatcaccggtatcacttc | 254243 | - | see also 17844 line |
| 40499 | DIXDC1 | NM_033425.1 | 472 | gtgcaaagagcgagtccattata | 254220 | - | see also 17844 line |
| 40500 | DKFZP434I092 | NM_015668.1 | 2479 | ctctctaacgtctctaaacgaac | 254323 | - | see also 17845 line |
| 40501 | DKFZP434I092 | NM_015668.1 | 43 | accgcggagccaccaactattac | 254246 | - | see also 17845 line |
| 40502 | DKFZP434I092 | NM_015668.1 | 1177 | agggcggactggtgtatttctca | 254281 | - | see also 17845 line |
| 40503 | DKKL1-pending | NM_014419.1 | 461 | gagcttcgagggtgatttgaagg | 254366 | - | see also 17846 line |
| 40504 | DRCTNNB1A | NM_032581.2 | 1762 | gacctcctagtattagcattact | 254420 | - | see also 9252 line |
| 40505 | DVL1 | NM_004421.2 | 1910 | cacgaccaaggcctatacagtgg | 254431 | - | see also 17848 line |
| 40506 | DVL1 | NM_004421.2 | 1908 | cccacgaccaaggcctatacagt | 254430 | - | see also 17848 line |
| 40507 | DVL2 | NM_004422.1 | 2148 | gacagccagccgccaaagcttcc | 254440 | - | see also 17849 line |
| 40508 | DVL2 | NM_004422.1 | 810 | cacgctaaacatggagaagtaca | 254434 | - | see also 17849 line |
| 40509 | GASZ | NM_130768.1 | 117 | ctcgaccggacgtctcagaaatt | 254441 | - | see also 17850 line |
| 40510 | GASZ | NM_130768.1 | 470 | gagacttatgaccccaatcatgt | 254461 | - | see also 17850 line |
| 40511 | IKBKG | NM_003639.2 | 1118 | gaggccgagcagcacaagattgt | 254493 | - | see also 2457 line |
| 40512 | IKBKG | NM_003639.2 | 1447 | ctgtcccaagtgccagtatcagg | 254496 | - | see also 2457 line |
| 40513 | KCNH7 | NM_033272.2 | 1028 | agcatcttcggtccatgatatag | 254522 | - | see also 9392 line |
| 40514 | KCNH7 | NM_033272.2 | 1027 | gagcatcttcggtccatgatata | 254521 | - | see also 9392 line |
| 40515 | LOC139135 | NM_173493.1 | 346 | accaagtgacgctacagttatta | 254616 | - | see also 17852 line |
| 40516 | LOC139135 | NM_173493.1 | 1487 | cacattacgccacgttgtcattc | 254639 | - | see also 17852 line |
| 40517 | LOC139135 | NM_173493.1 | 608 | tagttctgcttacgaaaacgtga | 254621 | - | see also 17852 line |
| 40518 | LY86 | NM_004271.1 | 155 | ttgcgatccattacaagattttg | 254659 | - | see also 17853 line |
| 40519 | LY86 | NM_004271.1 | 147 | taccagagttgcgatccattaca | 254656 | - | see also 17853 line |
| 40520 | NPAS3 | NM_022123.1 | 797 | aacgcggtgtgcacatcaaatca | 254676 | - | see also 8284 line |
| 40521 | NXF | NM_178864.2 | 866 | ctccgagagtgtcctaatctacc | 254709 | - | see also 10227 line |
| 40522 | NXF | NM_178864.2 | 666 | aacaaactcgtgcttattcgagg | 254708 | - | see also 10227 line |
| 40523 | NXF | NM_178864.2 | 323 | cactcgcaagggcgtcttcttcg | 254704 | - | see also 10227 line |
| 40524 | PDCD1 | NM_005018.1 | 245 | atcggagagcttcgtgctaaact | 254730 | - | see also 17856 line |

Figure 46

| | | | | | | | |
|---|---|---|---|---|---|---|---|
| 40525 | PDCD1 | NM_005018.1 | 651 | gccgcacgaggacaataggagc | 254731 | - | see also 17856 line |
| 40526 | PER1 | NM_002616.1 | 1214 | cgcatccattcgggttacgaagc | 254738 | - | see also 1691 line |
| 40527 | PER1 | NM_002616.1 | 1471 | tgcccgcaacgggagtatgtca | 254741 | - | see also 1691 line |
| 40528 | PER1 | NM_002616.1 | 1134 | tccgcctaacccgtatgtgacc | 254737 | - | see also 1691 line |
| 40529 | PER2 | NM_003894.3 | 1220 | cccggaacgaggagtacatcacg | 254771 | - | see also 17858 line |
| 40530 | PER2 | NM_003894.3 | 745 | ctgaccaggttgcatccatattt | 254762 | - | see also 17858 line |
| 40531 | PER3 | NM_016831.1 | 3368 | agcgacagcagtatataccttac | 254855 | - | see also 6970 line |
| 40532 | PER3 | NM_016831.1 | 1190 | cccattcgattttgtactccaaaa | 254820 | - | see also 6970 line |
| 40533 | PLEKHB1 | NM_021200.1 | 934 | cagcccgtaccaagactactacg | 254871 | - | see also 17860 line |
| 40534 | PLEKHB1 | NM_021200.1 | 619 | ccgtgtgtcatccacttcaatg | 254870 | - | see also 17860 line |
| 40535 | PPP1R12A | NM_002480.1 | 2300 | agcgttataggccagtatcaact | 254912 | - | see also 17861 line |
| 40536 | PPP1R12A | NM_002480.1 | 1631 | atgaaggatcaacgtatcataaa | 254897 | - | see also 17861 line |
| 40537 | PWP2H | NM_005049.1 | 35 | aagttcgcttaccggttttcaaa | 254932 | - | see also 17862 line |
| 40538 | PWP2H | NM_005049.1 | 119 | atcagtcccgtgggcaatagagt | 254938 | - | see also 17862 line |
| 40539 | RGS10 | NM_002925.2 | 213 | ggctagcatgtgaagattttaag | 254970 | - | signal_transduction [signal transducer] |
| 40540 | RGS10 | NM_002925.2 | 147 | cagaaggcgtgaaaagatttagg | 254967 | - | see also 40539 line |
| 40541 | RGS10 | NM_002925.2 | 212 | tggctagcatgtgaagattttaa | 254969 | - | see also 40539 line |
| 40542 | RGS13 | NM_002927.3 | 559 | cacagtcccctagaagattaac | 254982 | - | see also 1970 line |
| 40543 | RGS13 | NM_002927.3 | 590 | ttcgacaagagagactcatcca | 254983 | - | see also 1970 line |
| 40544 | RGS17 | NM_012419.3 | 612 | gagttagaggtgatcaataga | 254989 | - | see also 17864 line |
| 40545 | RGS17 | NM_012419.3 | 669 | atgcccaacttcagatatatact | 254990 | - | see also 17864 line |
| 40546 | RGS18 | NM_130782.1 | 344 | aagacacccgctccagtagatct | 254991 | - | see also 17865 line |
| 40547 | RGS18 | NM_130782.1 | 540 | aagcaaggggacctcaacaaattc | 254995 | - | see also 17865 line |
| 40548 | RGS3 | NM_017790.2 | 864 | acctctacctggacctattaac | 255004 | - | signal_transduction, g / pcr |
| 40549 | RGS3 | NM_017790.2 | 466 | gggcaaggcagacacaaaatgatga | 255003 | - | see also 40548 line |
| 40550 | RGS5 | NM_003617.1 | 599 | ctgagtttatcaggagttaatc | 255024 | - | see also 17866 line |
| 40551 | RGS5 | NM_003617.1 | 188 | ttgtcattcgtacaatgagaag | 255020 | - | see also 17866 line |
| 40552 | RGS8 | NM_033345.1 | 259 | aaccgcgctctcaagagattatc | 255029 | - | see also 17867 line |
| 40553 | RGS8 | NM_033345.1 | 217 | gactcgttagtgatttcacagc | 255028 | - | see also 17867 line |
| 40554 | RGSL1 | NM_181572.2 | 975 | ttgcgatcaatgatctatattc | 255065 | - | see also 17868 line |
| 40555 | RGSL1 | NM_181572.2 | 1047 | tgcaggtcaaccggcatataat | 255067 | - | see also 17868 line |
| 40556 | RGSL2 | NM_032267.1 | 211 | ttggaaaaatgccgattacctt | 255097 | - | see also 17869 line |
| 40557 | RGSL2 | NM_032267.1 | 20 | ctgagataattggttctacaaat | 255091 | - | see also 17869 line |
| 40558 | RTN1 | NM_021136.1 | 418 | atggggaaggatcggttacaca | 255121 | - | see also 8098 line |
| 40559 | RTN1 | NM_021136.1 | 591 | gagtctgtggcttatttagttc | 255127 | - | see also 8098 line |
| 40560 | RTN2 | NM_005619.2 | 892 | atcccgatcgcgagattcgaact | 255155 | - | see also 17871 line |

Figure 46

| | | | | | | | |
|---|---|---|---|---|---|---|---|
| 40561 | RTN2 | NM_005619.2 | 995 | ctgatagcacggaccaattaga | 255156 | - | see also 17871 line |
| 40562 | RTN2 | NM_005619.2 | 310 | agggaacgacgactctgatttc | 255152 | - | see also 17871 line |
| 40563 | SH3BP4 | NM_014521.1 | 1961 | ttccaatatgacgaattacgag | 255200 | - | see also 17872 line |
| 40564 | SH3BP4 | NM_014521.1 | 2725 | gggtggcgtccgtcctagaaaag | 255204 | - | see also 17872 line |
| 40565 | SHC3 | NM_016848.2 | 1253 | tcccgctccatgatcgaatgc | 255215 | - | see also 17873 line |
| 40566 | SHC3 | NM_016848.2 | 1210 | ttgagctcggtttaagcaatat | 255213 | - | see also 17873 line |
| 40567 | SNX13 | NM_015132.2 | 477 | aagattgacgggtgccaataaa | 255230 | - | see also 17874 line |
| 40568 | SNX13 | NM_015132.2 | 2619 | agctacatatggcgatactatta | 255299 | - | see also 17874 line |
| 40569 | SNX25 | NM_031953.2 | 2314 | ctcgttcagtcactttggaag | 255359 | - | see also 17875 line |
| 40570 | SNX25 | NM_031953.2 | 775 | ttgaagcaactaaggtatcaaat | 255322 | - | see also 17875 line |
| 40571 | STMN1 | NM_005563.2 | 164 | agccctcggtcaaaagaatctgt | 255371 | - | signal transducer |
| 40572 | STMN1 | NM_005563.2 | 453 | aacgtttgcgagagaaggataag | 255372 | - | see also 40571 line |
| 40573 | STMN1 | NM_005563.2 | 161 | ctcagccctcggtcaaaagaatc | 255370 | - | see also 40571 line |
| 40574 | TM4SF3 | NM_004616.2 | 274 | gggtacgaagcaatgactct | 255375 | - | see also 17876 line |
| 40575 | TM4SF3 | NM_004616.2 | 394 | tgggatgctcggtgctataaaa | 255377 | - | see also 17876 line |
| 40576 | TP53AP1 | NM_007233.1 | 332 | agcaccaggcgaacacttacacc | 255396 | - | - |
| 40577 | TP53AP1 | NM_007233.1 | 214 | acccaaccctcggatagatgc | 255392 | - | see also 40576 line |
| 40578 | TP53AP1 | NM_007233.1 | 242 | gcgatggttaagacccatttca | 255394 | - | see also 40576 line |
| 40579 | WNT1 | NM_005430.2 | 558 | ctgtcgagaaacggcgttatct | 255402 | - | see also 17877 line |
| 40580 | WNT1 | NM_005430.2 | 291 | tggccgatgtggggtattgtga | 255397 | - | see also 17877 line |
| 40581 | WNT10A | NM_025216.2 | 575 | aggtcagcaccaatgacattct | 255407 | - | see also 17878 line |
| 40582 | WNT10A | NM_025216.2 | 789 | gcctggagactcgcaacaagatc | 255408 | - | see also 17878 line |
| 40583 | WNT10B | NM_003394.2 | 412 | gtgcagtcgggctctaagcaatg | 255413 | - | see also 2293 line |
| 40584 | WNT11 | NM_004626.2 | 666 | aggatcccaagccaataaaactga | 255419 | - | see also 3143 line |
| 40585 | WNT16 | NM_016087.2 | 869 | ctgtctatgttaataagtctcc | 255431 | - | see also 17881 line |
| 40586 | WNT16 | NM_016087.2 | 753 | ttggccatttgttgaaggataaa | 255424 | - | see also 17881 line |
| 40587 | WNT2 | NM_003391.1 | 973 | ttcaggaaaacgggcgattatct | 255440 | - | see also 17882 line |
| 40588 | WNT2 | NM_003391.1 | 975 | caggaaaacgggcgattatctct | 255441 | - | see also 17882 line |
| 40589 | WNT2B | NM_004185.2 | 508 | ggggtagtccacgctattactcg | 255458 | - | see also 2806 line |
| 40590 | WNT2B | NM_004185.2 | 853 | tacctgcggcgacgctatgatgg | 255461 | - | see also 2806 line |
| 40591 | WNT3 | NM_030753.3 | 384 | tggaactgcaccaccatagatga | 255476 | - | see also 8927 line |
| 40592 | WNT3A | NM_033131.2 | 82 | gccccactcggatacttcttact | 255487 | - | see also 17885 line |
| 40593 | WNT3A | NM_033131.2 | 274 | gtggccgagggcatcaagattgg | 255488 | - | see also 17885 line |
| 40594 | WNT4 | NM_030761.2 | 812 | gccacgcaacgacagttcaagc | 255490 | - | signal transducer |
| 40595 | WNT5B | NM_030775.2 | 780 | tggcagacgtagcctgcaaatgc | 255501 | - | see also 17886 line |
| 40596 | WNT5B | NM_030775.2 | 757 | cggtcgcaggggtgtgtataaga | 255499 | - | see also 17886 line |
| 40597 | WNT6 | NM_006522.2 | 1219 | gagcgtgcagctcgaagagaact | 255504 | - | see also 17887 line |

Figure 46

| ID | Gene | Accession | Pos | Sequence | Seq ID | | See also | Note |
|---|---|---|---|---|---|---|---|---|
| 40598 | WNT7B | NM_058238.1 | 859 | aacagctgcgcagctatcagaag | 255509 | - | see also 9505 line | |
| 40599 | WNT8A | NM_031933.1 | 737 | gaccaggcgctgaaaattgaaat | 255524 | - | see also 17889 line | |
| 40600 | WNT8A | NM_031933.1 | 352 | gacttccttcatacatgctatca | 255514 | - | see also 17889 line | |
| 40601 | WNT8B | NM_003393.2 | 555 | ggcgatttccaagcagtttgtcg | 255533 | - | see also 2290 line | |
| 40602 | WNT8B | NM_003393.2 | 1109 | gccgaaggtcaccaagtacttc | 255535 | - | see also 2290 line | |
| 40603 | WNT9A | NM_003395.1 | 509 | gggctgcggagacaaccttaagt | 255538 | - | see also 17891 line | |
| 40604 | WNT9A | NM_003395.1 | 590 | ccgtgtggacttccacacacaacc | 255539 | - | see also 17891 line | |
| 40605 | WNT9B | NM_003396.1 | 747 | tgctgaaactgcgctatgactcg | 255543 | - | - | signal transducer |
| 40606 | WNT9B | NM_003396.1 | 600 | cacgggcagacgcccacaatacc | 255542 | - | see also 40605 line | |
| 40607 | C3orf1 | NM_016589.1 | 567 | cacacgaggcttcattcgttatg | 255548 | - | see also 17892 line | |
| 40608 | C3orf1 | NM_016589.1 | 708 | cacgggaagtcttttaggataa | 255551 | - | see also 17892 line | |
| 40609 | C3orf1 | NM_016589.1 | 862 | aaggcactccatgaagctaaaact | 255552 | - | see also 17892 line | |
| 40610 | SEC61A1 | NM_013336.2 | 561 | gccagtctatcgttatgttgatg | 255578 | - | see also 17893 line | |
| 40611 | SEC61A1 | NM_013336.2 | 1253 | gacccggtccatgcagtttgtata | 255592 | - | see also 17893 line | |
| 40612 | SEC61A2 | NM_018144.2 | 1298 | caccgagatacctctatggttca | 255625 | - | see also 7280 line | |
| 40613 | SEC61A2 | NM_018144.2 | 1428 | ttctgctagcagtcactattatt | 255627 | - | see also 7280 line | |
| 40614 | SEC61A2 | NM_018144.2 | 190 | gactgctataacgctcttcattt | 255600 | - | see also 7280 line | |
| 40615 | SEC61G | NM_014302.2 | 142 | tccattcggctggttaaaagatg | 255634 | - | see also 17894 line | |
| 40616 | SEC61G | NM_014302.2 | 162 | atgcactaaacctgatagaaaag | 255635 | - | see also 17894 line | |
| 40617 | TIMM17A | NM_006335.1 | 464 | cagttgccttcaactcagttacc | 255651 | - | see also 17895 line | |
| 40618 | TIMM17A | NM_006335.1 | 153 | gagggagtttgacagctattaaa | 255642 | - | see also 17895 line | |
| 40619 | TIMM17B | NM_005834.1 | 361 | gcctgttctccaccattgactgt | 255655 | - | see also 17896 line | |
| 40620 | TIMM17B | NM_005834.1 | 260 | ccgcaatgccctgttggaattc | 255654 | - | see also 17896 line | |
| 40621 | TIMM22 | NM_013337.1 | 321 | tacctaacgactgcgcaaaaga | 255660 | - | see also 17897 line | |
| 40622 | TIMM22 | NM_013337.1 | 275 | taccgctggcatgataccaacg | 255658 | - | see also 17897 line | |
| 40623 | TIMM22 | NM_013337.1 | 558 | ttctctgctgcgattgattatta | 255663 | - | see also 17897 line | |
| 40624 | TIMM23 | NM_006327.1 | 344 | gagctggccttctttacgattgg | 255672 | - | see also 17898 line | |
| 40625 | TIMM23 | NM_006327.1 | 309 | tacctaccggagctaataaaacc | 255670 | - | see also 17898 line | |
| 40626 | TIMM8A | NM_004085.2 | 232 | gcgtttgagcgcttcattgataca | 255681 | - | see also 17899 line | |
| 40627 | TIMM8A | NM_004085.2 | 230 | ctgcgttgagcgcttcattgata | 255680 | - | see also 17899 line | |
| 40628 | SLC6A4 | NM_001045.2 | 1972 | cccgccacaactacgactttcc | 255741 | - | see also 17901 line | |
| 40629 | SLC6A4 | NM_001045.2 | 679 | gggaatcccgctctttacatgg | 255721 | - | see also 17901 line | |
| 40630 | SLC6A2 | NM_001043.1 | 779 | gtcgtcatcgtctgtatttttag | 255755 | - | see also 17902 line | |
| 40631 | SLC6A2 | NM_001043.1 | 938 | atcgactctaccgcttgaaaga | 255757 | - | see also 17902 line | |
| 40632 | SLC6A8 | NM_005629.1 | 1406 | cacgggaaagatcgtgtacttca | 255775 | - | see also 17903 line | |
| 40633 | SLC6A8 | NM_005629.1 | 651 | aggccgagaaacggccatctatag | 255772 | - | see also 17903 line | |
| 40634 | FLJ31236 | NM_182632.1 | 1761 | ggcctggaacccaaaatacgagc | 255797 | - | see also 17905 line | |

Figure 46

| | | | | | | | |
|---|---|---|---|---|---|---|---|
| 40635 | FLJ31236 | NM_182632.1 | 1523 | ggctggagatttgacaattt | 255796 | - | see also 17905 line |
| 40636 | NTT5 | NM_014037.1 | 1765 | atcgtcgttgtcgtatttgaaac | 255838 | - | see also 17906 line |
| 40637 | NTT5 | NM_014037.1 | 1762 | atcatcgtcgttgtcgtatttga | 255837 | - | see also 17906 line |
| 40638 | NTT73 | NM_018057.2 | 744 | tactacttatgttaataggtatt | 255859 | - | see also 7220 line |
| 40639 | NTT73 | NM_018057.2 | 716 | ggcggtgcatatcttttaccata | 255855 | - | see also 7220 line |
| 40640 | SLC6A11 | NM_014229.1 | 1554 | ggccaccgtcgctcattaagtgg | 255929 | - | see also 5547 line |
| 40641 | SLC6A11 | NM_014229.1 | 1551 | accggccaccgtcgctcattaag | 255927 | - | see also 5547 line |
| 40642 | SLC6A12 | NM_003044.2 | 2017 | ggcggacgtttctatgacaaca | 255949 | - | see also 2046 line |
| 40643 | SLC6A12 | NM_003044.2 | 1046 | tggacgactgcaacaactttg | 255940 | - | see also 2046 line |
| 40644 | SLC6A13 | NM_016615.2 | 1598 | cactccgctgacctaccaacaaga | 255968 | - | see also 6952 line |
| 40645 | SLC6A13 | NM_016615.2 | 786 | ctggtgtctgttaattcgagg | 255960 | - | see also 6952 line |
| 40646 | SLC6A14 | NM_007231.1 | 1677 | ttgtaattacgcctatccttg | 256015 | - | see also 5043 line |
| 40647 | SLC6A14 | NM_007231.1 | 1862 | atctttcaacgcctataagttg | 256023 | - | see also 5043 line |
| 40648 | SLC6A3 | NM_001044.2 | 1421 | caacgggctcatcgatgagttcc | 256033 | - | see also 640 line |
| 40649 | SLC6A3 | NM_001044.2 | 732 | tcgggcctcaacgacacctttgg | 256030 | - | see also 640 line |
| 40650 | SLC6A3 | NM_001044.2 | 1765 | cccactaacgggacgctacatcttc | 256036 | - | see also 640 line |
| 40651 | SLC6A5 | NM_004211.1 | 2396 | tggctcttaccgctatcctaact | 256078 | - | see also 2820 line |
| 40652 | SLC6A5 | NM_004211.1 | 1334 | tcccaacgtgacaatggttaatt | 256049 | - | see also 2820 line |
| 40653 | SLC6A7 | NM_014228.2 | 1817 | ctgccgagacatccacacatgagc | 256093 | - | see also 17913 line |
| 40654 | SLC6A7 | NM_014228.2 | 1310 | cacgtttcaccagaacatctata | 256088 | - | see also 17913 line |
| 40655 | XT3 | NM_020208.1 | 1099 | gagatgttccgcaaatcaaaaa | 256106 | - | see also 17914 line |
| 40656 | XT3 | NM_020208.1 | 1097 | gcgagagtttccgcaaatcaaa | 256105 | - | see also 17914 line |
| 40657 | SLC10A1 | NM_003049.1 | 624 | cacaatacatgcgctatgtcatc | 256121 | - | see also 17915 line |
| 40658 | SLC10A1 | NM_003049.1 | 936 | ttccctcctctacatgatttc | 256123 | - | see also 17915 line |
| 40659 | SLC10A2 | NM_000452.1 | 1488 | agctcgcctttgcgcgcaatattc | 256150 | - | see also 17916 line |
| 40660 | SLC10A2 | NM_000452.1 | 1358 | aggtgccgaacggttgcttttga | 256143 | - | see also 17916 line |
| 40661 | SLC10A2 | NM_000452.1 | 1357 | caggtgccgaacggttgctttttg | 256142 | - | see also 17916 line |
| 40662 | SLC10A4 | NM_152679.2 | 805 | cacccctatcgtgcagttactac | 256160 | - | see also 17918 line |
| 40663 | SLC10A4 | NM_152679.2 | 1061 | ggctacgcttcaggttatggtt | 256174 | - | see also 17918 line |
| 40664 | SLC10A4 | NM_152679.2 | 806 | accccctatcgtgcagttactacc | 256161 | - | see also 17918 line |
| 40665 | SLC5A2 | NM_003041.1 | 978 | ctgaagctgacgccatgttct | 256193 | - | see also 2036 line |
| 40666 | SLC5A1 | NM_000343.1 | 1816 | caggagagcctatgaacctatttt | 256218 | - | see also 17919 line |
| 40667 | SLC5A1 | NM_000343.1 | 1282 | tgccggaaggttgtttatcctgg | 256208 | - | see also 17919 line |
| 40668 | SLC23A2 | NM_005116.4 | 2161 | gacggcatggagtcgtacaattt | 256254 | - | see also 3589 line |
| 40669 | SLC23A2 | NM_005116.4 | 539 | agctcatggcgatctacacactacg | 256226 | - | see also 3589 line |
| 40670 | SLC38A1 | NM_030674.2 | 1240 | cacgttacgccaaaatatgtta | 256295 | - | see also 17921 line |
| 40671 | SLC38A1 | NM_030674.2 | 1236 | ctgacacgtgtacgccaaaatat | 256293 | - | see also 17921 line |

Figure 46

| 40672 | SLC13A2 | NM_003984.1 | 298 | aggttgccgtcgagtatcttaag | 256320 | - | see also 17922 line |
|---|---|---|---|---|---|---|---|
| 40673 | SLC13A2 | NM_003984.1 | 1318 | tgccgtggaatatcgtgttattg | 256329 | - | see also 17922 line |
| 40674 | SLC28A1 | NM_004213.2 | 1286 | atcgatgccacctcgttgattgc | 256338 | - | see also 17923 line |
| 40675 | SLC28A1 | NM_004213.2 | 1606 | gcggcctgtagccttcttgatgg | 256340 | - | see also 17923 line |
| 40676 | SLC28A2 | NM_004212.1 | 918 | aaggtcgcctggtttttacaaat | 256364 | - | see also 17924 line |
| 40677 | SLC28A2 | NM_004212.1 | 1304 | tgccataggccttgctactaatg | 256368 | - | see also 17924 line |
| 40678 | SLC28A3 | NM_022127.1 | 1131 | ctggtccgaccatatttacctta | 256409 | - | see also 17925 line |
| 40679 | SLC28A3 | NM_022127.1 | 783 | tggggaatcgggctacagtttct | 256397 | - | see also 17925 line |
| 40680 | SLC15A1 | NM_005073.1 | 213 | accgccatctaccatacgtttgt | 256417 | - | see also 3545 line |
| 40681 | SLC15A1 | NM_005073.1 | 2081 | agcggagatcgaagctcaatttg | 256465 | - | see also 3545 line |
| 40682 | SLC15A2 | NM_021082.2 | 200 | tgcgagcgcttttcctattatgg | 256473 | - | see also 8081 line |
| 40683 | SLC15A2 | NM_021082.2 | 570 | aggaacggactagatacttctca | 256485 | - | see also 8081 line |
| 40684 | SLC15A2 | NM_021082.2 | 199 | ctgcgagcgcttttcctattatg | 256472 | - | see also 8081 line |
| 40685 | SLC17A5 | NM_012434.3 | 1042 | atcctaaggttcaatgttcaaga | 256561 | - | see also 17928 line |
| 40686 | SLC17A5 | NM_012434.3 | 197 | ctgctcgttacaacttagcaatt | 256532 | - | see also 17928 line |
| 40687 | SLC12A1 | NM_000338.1 | 719 | gccacgaacggagttgttagagg | 256595 | - | see also 284 line |
| 40688 | SLC12A1 | NM_000338.1 | 1554 | ttgtcagcgcacccaaagtgttc | 256612 | - | see also 284 line |
| 40689 | SLC12A2 | NM_001046.2 | 3393 | agcaagttccggatagactttc | 256773 | - | see also 641 line |
| 40690 | SLC12A2 | NM_001046.2 | 3708 | ctctacatggcatggttagaagc | 256789 | - | see also 641 line |
| 40691 | SLC12A5 | NM_020708.3 | 1905 | tggaggccacgctttcgatatta | 256799 | - | see also 7927 line |
| 40692 | SLC12A5 | NM_020708.3 | 1904 | ctggaggccacgctttcgatatt | 256798 | - | see also 7927 line |
| 40693 | SLC12A7 | NM_006598.1 | 1438 | aggcgtggtcttacgagataagt | 256828 | - | see also 4631 line |
| 40694 | SLC12A7 | NM_006598.1 | 2741 | atggttgaaaacgacatatctgc | 256834 | - | see also 4631 line |
| 40695 | SLC12A3 | NM_000339.1 | 187 | atcgatgtggtgcccacatatga | 256838 | - | see also 17933 line |
| 40696 | SLC12A3 | NM_000339.1 | 1713 | ggcgctgtttggggctatcatct | 256850 | - | see also 17933 line |
| 40697 | SLC12A3 | NM_000339.1 | 1067 | tcctggcaggggccaacatatct | 256844 | - | see also 17933 line |
| 40698 | SLC12A6 | NM_005135.1 | 2905 | cacatgcggctatccaaaacaga | 256947 | - | see also 3611 line |
| 40699 | SLC12A6 | NM_005135.1 | 903 | cggcgtacgctatgtgaacaagt | 256885 | - | see also 3611 line |
| 40700 | SLC13A4 | NM_012450.2 | 1596 | gccgcagaggtggtgaactttgg | 256962 | - | see also 17935 line |
| 40701 | SLC13A4 | NM_012450.2 | 1013 | tgctctgcgcatggtcttgatgg | 256958 | - | see also 17935 line |
| 40702 | SLC4A4 | NM_003759.1 | 3187 | tcctaagcgatagcaaaccttct | 257068 | - | see also 2569 line |
| 40703 | SLC4A5 | NM_021196.2 | 1137 | agcccgcaatcgggaagatctga | 257082 | - | see also 17937 line |
| 40704 | SLC4A5 | NM_021196.2 | 1095 | aaccctcatggtagatgatctct | 257081 | - | see also 17937 line |
| 40705 | SLC4A7 | NM_003615.2 | 510 | gaccgatggagtaaaccttatgt | 257126 | - | see also 17938 line |
| 40706 | SLC4A7 | NM_003615.2 | 2635 | tgcgatcgacaatcagtgatttt | 257170 | - | see also 17938 line |
| 40707 | SLC12A4 | NM_005072.3 | 975 | tcccgtgtttccggtatgcatgc | 257214 | - | see also 3544 line |
| 40708 | SLC12A4 | NM_005072.3 | 1449 | tacaacttccctcgtgtgacttca | 257219 | - | see also 3544 line |

Figure 46

| | | | | | | | |
|---|---|---|---|---|---|---|---|
| 40709 | SLC12A4 | NM_005072.3 | 1507 | gtggttctccgggacaagtatgg | 257220 | - | see also 3544 line |
| 40710 | SLC13A1 | NM_022444.3 | 732 | cacgtaaacttacgtgtttgtgc | 257250 | - | see also 8358 line |
| 40711 | SLC13A1 | NM_022444.3 | 723 | gccacgtgacacgtaaacttacg | 257249 | - | see also 8358 line |
| 40712 | SLC13A3 | NM_022829.3 | 365 | ccggcggaatcgccctcaagatcc | 257293 | - | see also 8485 line |
| 40713 | SLC13A3 | NM_022829.3 | 1765 | tcccggactgggctgatatgtac | 257314 | - | see also 8485 line |
| 40714 | SLC16A1 | NM_003051.2 | 1192 | tgcggcttcgttgttgcaaatg | 257341 | - | see also 17942 line |
| 40715 | SLC16A1 | NM_003051.2 | 1472 | ggcgtcgtcctaattatttcagg | 257357 | - | see also 17942 line |
| 40716 | SLC16A1 | NM_003051.2 | 1465 | ggcatgtgcgtcgtcctaatta | 257354 | - | see also 17942 line |
| 40717 | SLC16A2 | NM_006517.1 | 831 | ccctccgatgggtatgatcttc | 257364 | - | see also 17943 line |
| 40718 | SLC16A2 | NM_006517.1 | 829 | agccctcgcgatgggtatgatct | 257363 | - | see also 17943 line |
| 40719 | SLC16A2 | NM_006517.1 | 1623 | gccttatcgtcgtcgtctttc | 257375 | - | see also 17943 line |
| 40720 | SLC16A3 | NM_004207.1 | 1273 | tgggcaacttcttcgcattagg | 257380 | - | see also 17944 line |
| 40721 | SLC16A4 | NM_004696.1 | 724 | ttggagctatcgcattgaattg | 257397 | - | see also 3219 line |
| 40722 | SLC16A4 | NM_004696.1 | 728 | agctatcgcattgaatttggtgc | 257399 | - | see also 3219 line |
| 40723 | SLC16A5 | NM_004695.2 | 1672 | cacgtccagctgcgtcaataag | 257424 | - | see also 17946 line |
| 40724 | SLC16A5 | NM_004695.2 | 380 | cacgtatcggcatcttcttca | 257419 | - | see also 17946 line |
| 40725 | SLC16A6 | NM_004694.2 | 680 | tgctttcgcaacagcaatcatgg | 257439 | - | see also 17947 line |
| 40726 | SLC16A6 | NM_004694.2 | 1245 | agggagccattcgtaagattta | 257441 | - | see also 17947 line |
| 40727 | SLC16A7 | NM_004731.3 | 1276 | gccgtcggacttgtcacaattgt | 257484 | - | see also 3249 line |
| 40728 | SLC16A7 | NM_004731.3 | 539 | tgcaaccgccttaaccatcatt | 257454 | - | see also 3249 line |
| 40729 | SLC16A8 | NM_013356.1 | 1409 | tgcctgcgttgtgctaaagctgc | 257496 | - | [symporter] |
| 40730 | SLC16A8 | NM_013356.1 | 1602 | ggctggctgccgagtctgtataa | 257497 | - | see also 40729 line |
| 40731 | SLC16A8 | NM_013356.1 | 1057 | gccgcacgtccgtatctgttca | 257494 | - | see also 40729 line |
| 40732 | SLC17A1 | NM_005074.1 | 1283 | atccggaatccgcctggtttaaa | 257541 | - | see also 17949 line |
| 40733 | SLC17A1 | NM_005074.1 | 1284 | tcggaatccgcctggtttaaaa | 257542 | - | see also 17949 line |
| 40734 | SLC17A2 | NM_005835.1 | 1128 | atcacccgtgcataagrgttagg | 257565 | - | see also 17950 line |
| 40735 | SLC17A2 | NM_005835.1 | 1301 | tacctaccaacgtatatcagtac | 257570 | - | see also 17950 line |
| 40736 | SLC17A2 | NM_005835.1 | 1297 | aacataccaccaacgtatatca | 257569 | - | see also 17950 line |
| 40737 | SLC17A3 | NM_006632.1 | 1250 | ctcaattccggctatatcacagc | 257603 | - | see also 17951 line |
| 40738 | SLC17A3 | NM_006632.1 | 909 | aacaacagtcgggtcttctaag | 257595 | - | see also 17951 line |
| 40739 | SLC17A3 | NM_006632.1 | 467 | ttcacaacgatagcacacaaatgt | 257584 | - | see also 17951 line |
| 40740 | SLC1A1 | NM_004170.3 | 514 | tgcgcgctgtgtgttattc | 257620 | - | see also 17952 line |
| 40741 | SLC1A1 | NM_004170.3 | 515 | gcgcgctgtgtgttattct | 257621 | - | see also 17952 line |
| 40742 | SLC1A3 | NM_004172.3 | 606 | tgcgagctgtagtctattatg | 257658 | - | see also 2801 line |
| 40743 | SLC1A3 | NM_004172.3 | 444 | ccctccgaccattacagaatgagc | 257654 | - | see also 2801 line |
| 40744 | SLC1A4 | NM_003038.2 | 751 | tacgtatgcaaccgattataaag | 257682 | - | see also 17954 line |
| 40745 | SLC1A4 | NM_003038.2 | 1222 | agcgaaccttcctctatgatga | 257690 | - | see also 17954 line |

Figure 46

| | | | | | | | | |
|---|---|---|---|---|---|---|---|---|
| 40746 | SLC1A6 | NM_005071.1 | 394 | tgcgggcagctgtgtactacatg | 257698 | - | see also 3541 line | |
| 40747 | SLC1A6 | NM_005071.1 | 1530 | aacgtactgggggactcaattgg | 257705 | - | see also 3541 line | |
| 40748 | SLC1A7 | NM_006671.3 | 662 | tcctcggcggatcctcatctacg | 257710 | - | see also 17956 line | |
| 40749 | SLC1A7 | NM_006671.3 | 1492 | accgtttccgcaccatgattaac | 257716 | - | see also 17956 line | |
| 40750 | SLC23A1 | NM_005847.2 | 1432 | cgggctcacgctgcccaattacc | 257728 | - | see also 17957 line | |
| 40751 | SLC23A1 | NM_005847.2 | 1648 | cagcctcaagagctacgatttcc | 257733 | - | see also 17957 line | |
| 40752 | SLC24A1 | NM_004727.1 | 331 | tccggacaaagcggcttcattgg | 257736 | - | see also 17958 line | |
| 40753 | SLC24A1 | NM_004727.1 | 536 | ctccgaaatgggggggtaagatgc | 257741 | - | see also 17958 line | |
| 40754 | SLC24A2 | NM_020344.1 | 1522 | tacctgacgttcgcaaaccttca | 257804 | - | see also 7842 line | |
| 40755 | SLC24A2 | NM_020344.1 | 381 | ggcgagtctgagaatagtacaga | 257783 | - | see also 7842 line | |
| 40756 | SLC24A2 | NM_020344.1 | 666 | aacgttggcataggcacaattgt | 257790 | - | see also 7842 line | |
| 40757 | SLC24A3 | NM_020689.3 | 1238 | cagtcgcatgttgatcaatgaga | 257836 | - | see also 17960 line | |
| 40758 | SLC24A3 | NM_020689.3 | 1236 | gccagtcgcatgttgatcaatga | 257835 | - | see also 17960 line | |
| 40759 | SLC34A1 | NM_003052.2 | 741 | cacggtgcatgactgctttaact | 257843 | - | see also 17961 line | |
| 40760 | SLC34A1 | NM_003052.2 | 1519 | ttcaacatctcgggtatccttct | 257848 | - | see also 17961 line | |
| 40761 | SLC5A4 | NM_014227.1 | 164 | accaaccgaggtactataggagg | 257851 | - | see also 17962 line | |
| 40762 | SLC5A4 | NM_014227.1 | 297 | caggagtcgccaccgtaacattt | 257852 | - | see also 17962 line | |
| 40763 | SLC5A5 | NM_000453.1 | 1058 | cccgaggagccgctatacattct | 257903 | - | see also 17963 line | |
| 40764 | SLC5A5 | NM_000453.1 | 772 | tgctgtacaccggcatcgtaatc | 257898 | - | see also 17963 line | |
| 40765 | SLC5A5 | NM_000453.1 | 925 | tccaggtcgtggtgatgctaagt | 257900 | - | see also 17963 line | |
| 40766 | TSCOT | NM_033051.2 | 1436 | tggccatcattccaattagcatc | 257927 | - | see also 17964 line | |
| 40767 | TSCOT | NM_033051.2 | 1437 | ggccatcattccaattagcatcg | 257928 | - | see also 17964 line | |
| 40768 | FLJ14753 | NM_032558.1 | 643 | atcacgtttccggcaatcagtgc | 257943 | - | see also 9247 line | |
| 40769 | FLJ14753 | NM_032558.1 | 876 | tggggcatgtatagtccttatgt | 257950 | - | see also 9247 line | |
| 40770 | HIAT1 | NM_033055.2 | 1343 | ttccggaacataccaatttaagc | 257977 | - | see also 9355 line | |
| 40771 | HIAT1 | NM_033055.2 | 79 | ggcacgcctcaaggaataggttc | 257953 | - | see also 9355 line | |
| 40772 | MGC11332 | NM_032718.2 | 458 | tagagtcccggcaggtattttta | 257986 | - | see also 9277 line | |
| 40773 | MGC11332 | NM_032718.2 | 460 | gagtcccggcaggtattttaaa | 257987 | - | see also 9277 line | |
| 40774 | SLC22A1L | NM_002555.3 | 273 | ctcggtcatcttgcttacctacg | 258001 | - | - | hepatocellular carcinoma、 alveolar cell carcinoma、 rhabdomyosarcoma、 ataxia telangiectasia、 breast cancer、 lung cancer |
| 40775 | SLC22A1L | NM_002555.3 | 817 | agcaccaaaggggccaaaactga | 258002 | - | see also 40774 line | |
| 40776 | LOC133308 | NM_178833.2 | 1431 | caccgtaggcattgcagtattga | 258036 | - | see also 17968 line | |

Figure 46

| | | | | | | | |
|---|---|---|---|---|---|---|---|
| 40777 | LOC133308 | NM_178833.2 | 225 | cacgccctccatgcatcaagaag | 258003 | - | see also 17968 line |
| 40778 | MGC43026 | NM_178527.2 | 1803 | aagataaaacgaggatcgaatac | 258100 | - | see also 17969 line |
| 40779 | MGC43026 | NM_178527.2 | 1250 | aagatcgaacttgtaattactaa | 258085 | - | see also 17969 line |
| 40780 | SLC9A1 | NM_003047.2 | 2890 | cccaggaaccgacgatgtcttca | 258179 | - | see also 17970 line |
| 40781 | SLC9A1 | NM_003047.2 | 1524 | cagccttcacctcccgatttacc | 258172 | - | see also 17970 line |
| 40782 | SLC9A2 | NM_003048.3 | 2241 | ccgggaccaccgtgctcaatttg | 258227 | - | see also 17971 line |
| 40783 | SLC9A2 | NM_003048.3 | 1916 | aaggtcacgtccagtgaaactga | 258218 | - | see also 17971 line |
| 40784 | SLC9A3 | NM_004174.1 | 1495 | cggacagatcgggcacaattatc | 258236 | - | see also 17972 line |
| 40785 | SLC9A3 | NM_004174.1 | 844 | caccaagcatgtgcgtatcatcg | 258232 | - | see also 17972 line |
| 40786 | SLC9A5 | NM_004594.1 | 323 | tggtgctagggggaattgttttg | 258247 | - | see also 17973 line |
| 40787 | SLC9A5 | NM_004594.1 | 650 | aggaggtgcacgtcaatgagact | 258249 | - | see also 17973 line |
| 40788 | SLC9A6 | NM_006359.1 | 394 | ttcatgttccgagtgatgtaaat | 258269 | - | see also 4449 line |
| 40789 | SLC9A6 | NM_006359.1 | 1472 | cagcaccacgcttctgattgtgt | 258294 | - | see also 4449 line |
| 40790 | SLC9A7 | NM_032591.1 | 1039 | atgccaacgtgactaagtttacc | 258316 | - | see also 17975 line |
| 40791 | SLC9A7 | NM_032591.1 | 840 | gtggatctttacgcacttctttt | 258314 | - | see also 17975 line |
| 40792 | SLC9A9 | NM_173653.1 | 1688 | gagtgctcggctcttcagaatgt | 258357 | - | see also 10103 line |
| 40793 | SLC9A9 | NM_173653.1 | 861 | gtggccatagtccttacatattc | 258340 | - | see also 10103 line |
| 40794 | SLC4A1 | NM_000342.1 | 1554 | gaccaacggtctagagtacatcg | 258372 | - | see also 17977 line |
| 40795 | SLC4A1 | NM_000342.1 | 1753 | cacccactacagaagacttataa | 258374 | - | see also 17977 line |
| 40796 | SLC4A11 | NM_032034.1 | 1564 | gccgtcaagggcacggttaaaat | 258396 | - | see also 17978 line |
| 40797 | SLC4A11 | NM_032034.1 | 278 | ctggcgagagtatccgtttttt | 258382 | - | see also 17978 line |
| 40798 | SLC4A11 | NM_032034.1 | 279 | tggcgagagtatccgttttttg | 258383 | - | see also 17978 line |
| 40799 | SLC4A2 | NM_003040.2 | 2387 | taggggtgtcggagctgattatg | 258426 | - | see also 17979 line |
| 40800 | SLC4A2 | NM_003040.2 | 975 | ggccgacctagacctcatgaaga | 258416 | - | see also 17979 line |
| 40801 | SLC4A3 | NM_005070.1 | 845 | tggtgtgcggcgacacttagtga | 258446 | - | see also 3540 line |
| 40802 | SLC4A3 | NM_005070.1 | 2037 | ctgcggacgggctcggtatttgg | 258455 | - | see also 3540 line |
| 40803 | SLC4A8 | NM_004858.1 | 2309 | gggatgatcgcggatggattatt | 258496 | - | see also 3376 line |
| 40804 | SLC4A8 | NM_004858.1 | 2312 | atgatcgcggatggattattaat | 258497 | - | see also 3376 line |
| 40805 | SLC7A11 | NM_014331.2 | 485 | aacttgcgatcaagctcattaca | 258521 | - | see also 5626 line |
| 40806 | SLC7A11 | NM_014331.2 | 1327 | ctctattcggacccatttagtac | 258552 | - | see also 5626 line |
| 40807 | FLJ10847 | NM_018242.2 | 1678 | cacaggacggcgctaaattgtcc | 258595 | - | see also 7341 line |
| 40808 | FLJ31196 | NM_152908.2 | 1163 | tcgggcgtgctcagcatagttgg | 258610 | - | see also 17984 line |
| 40809 | FLJ31196 | NM_152908.2 | 1278 | gccaggtcttgccggtttatagt | 258611 | - | see also 17984 line |
| 40810 | CHDC1 | NM_015116.1 | 962 | gtgctcgtgattccaatttgttt | 258645 | - | see also 17985 line |

Figure 46

| 40811 | CHDC1 | NM_015116.1 | 1868 | ctgaagcctcgatcagtgtttct | 258674 | - | see also 17985 line |
| 40812 | FLJ10560 | NM_018138.1 | 1108 | atcgttgcaacgaatctttata | 258717 | - | see also 17986 line |
| 40813 | FLJ10560 | NM_018138.1 | 1416 | gtgcctaactattgggataatcc | 258731 | - | see also 17986 line |
| 40814 | FLJ37307 | NM_178515.2 | 588 | ttctccgtagctttaagagaaag | 258741 | - | see also 17987 line |
| 40815 | HT008 | NM_018469.3 | 3295 | cccgactgaccgagtatggtatg | 258809 | - | see also 17988 line |
| 40816 | HT008 | NM_018469.3 | 3112 | aggtcatcgaacaagtaagatta | 258803 | - | see also 17988 line |
| 40817 | KST1 | NM_052944.2 | 608 | accacgatgccagaatacctacg | 258816 | - | see also 17989 line |
| 40818 | KST1 | NM_052944.2 | 701 | aagatctcggtagacatgtatgc | 258820 | - | see also 17989 line |
| 40819 | MGC34837 | NM_152377.1 | 1703 | gccagcactgcggtacttaaagg | 258873 | - | see also 17990 line |
| 40820 | MGC34837 | NM_152377.1 | 1706 | agcactgcggtacttaaaggagc | 258874 | - | see also 17990 line |
| 40821 | NALP13 | NM_176810.1 | 647 | agcgcctactggatcctaatagg | 258889 | - | see also 17991 line |
| 40822 | NALP13 | NM_176810.1 | 1163 | gggacgacctacgggtatatttc | 258899 | - | see also 17991 line |
| 40823 | PTD015 | NM_024684.2 | 152 | ctctaatacaacctataaggact | 258947 | - | see also 17992 line |
| 40824 | PTD015 | NM_024684.2 | 142 | aagtaaaaggctctaatacaacc | 258944 | - | see also 17992 line |
| 40825 | SLC2A1 | NM_006516.1 | 1211 | ggcgggttgtgccatactcatga | 258956 | - | see also 17993 line |
| 40826 | SLC2A1 | NM_006516.1 | 422 | ctctgtgggccttttcgttaacc | 258953 | - | see also 17993 line |
| 40827 | SLC2A10 | NM_030777.3 | 1559 | ctcggcctgggcttcatctattt | 258970 | - | see also 17994 line |
| 40828 | SLC2A10 | NM_030777.3 | 1560 | tcggcctgggcttcatctattta | 258971 | - | see also 17994 line |
| 40829 | SLC2A11 | NM_030807.2 | 1172 | agctgctcacggccggttgttagt | 258980 | - | see also 17995 line |
| 40830 | SLC2A11 | NM_030807.2 | 1175 | tgctcacggcggttgttagttgt | 258982 | - | see also 17995 line |
| 40831 | SLC2A12 | NM_145176.1 | 1101 | ttggagtcgtcaaggtcattagc | 259018 | - | see also 9778 line |
| 40832 | SLC2A12 | NM_145176.1 | 1712 | ctgccatgggtgtgctttatata | 259037 | - | see also 9778 line |
| 40833 | SLC2A13 | NM_052885.1 | 1011 | cccaactcgccgagctttaattg | 259060 | - | see also 17997 line |
| 40834 | SLC2A13 | NM_052885.1 | 1014 | aactcgccgagctttaattgtgg | 259061 | - | see also 17997 line |
| 40835 | SLC2A14 | NM_153449.2 | 1119 | cgcgggtgtggttaatactatct | 259158 | - | see also 17998 line |
| 40836 | SLC2A14 | NM_153449.2 | 407 | ggggtatgatcggctccttttcc | 259154 | - | see also 17998 line |
| 40837 | SLC2A2 | NM_000340.1 | 895 | atcgtcacgggcattcttattag | 259117 | - | see also 287 line |
| 40838 | SLC2A2 | NM_000340.1 | 1343 | ttggcgctgtaaacatggttttc | 259135 | - | see also 287 line |
| 40839 | SLC2A3 | NM_006931.1 | 603 | atcctgggtcgcttggttattgg | 259156 | - | - | sugar porter | lung cancer |
| 40840 | SLC2A3 | NM_006931.1 | 581 | agctaagtcggttgaaatgctga | 259155 | - | see also 40839 line |
| 40841 | SLC2A4 | NM_001042.1 | 148 | gccgtcgggcttccaacagatag | 259164 | - | see also 18000 line |
| 40842 | SLC2A4 | NM_001042.1 | 1593 | tccaccggacaccctctcttta | 259169 | - | see also 18000 line |
| 40843 | SLC2A5 | NM_003039.1 | 945 | gtcgggcgtcaacgctatctact | 259185 | - | see also 18001 line |
| 40844 | SLC2A5 | NM_003039.1 | 948 | gggcgtcaacgctatctactact | 259187 | - | see also 18001 line |
| 40845 | SLC2A6 | NM_017585.2 | 166 | cgccgcagtgctcggcaatttca | 259192 | - | see also 18002 line |

Figure 46

| 40846 | SLC2A6 | NM_017585.2 | 639 | aggcgcctgtgctcatcatgatc | 259196 | - | see also 18002 line |
|---|---|---|---|---|---|---|---|
| 40847 | SLC2A8 | NM_014580.2 | 868 | gtcaacgccgtcatgttctatgc | 259201 | - | see also 18003 line |
| 40848 | SLC2A8 | NM_014580.2 | 866 | gggtcaacgccgtcatgttctat | 259200 | - | see also 18003 line |
| 40849 | SLC2A9 | NM_020041.1 | 1131 | tacagggggcatcgagactttgg | 259215 | - | see also 18004 line |
| 40850 | SLC2A9 | NM_020041.1 | 1281 | ctgggtccctacctgagtatcg | 259216 | - | see also 18004 line |
| 40851 | SLC35A2 | NM_005660.1 | 643 | cggcttcgcaggtgtctactttg | 259229 | - | see also 18005 line |
| 40852 | SLC35A2 | NM_005660.1 | 98 | cacaggcgcctgaagtacatatc | 259227 | - | see also 18005 line |
| 40853 | SLC35A3 | NM_012243.1 | 259 | ttctaacaatgcgttattccaga | 259235 | - | see also 5220 line |
| 40854 | SLC35A3 | NM_012243.1 | 918 | aaccgactgacctggatagtagt | 259261 | - | see also 5220 line |
| 40855 | SLC35C1 | NM_018389.3 | 698 | ctggcgcctgactttctacaaca | 259275 | - | see also 18007 line |
| 40856 | SLC35C1 | NM_018389.3 | 892 | gtccgctgacccacaatgtgtcg | 259277 | - | see also 18007 line |
| 40857 | SLC35D1 | NM_015139.1 | 855 | ctctgcacgcagtataattctgc | 259295 | - | see also 6258 line |
| 40858 | SLC35D1 | NM_015139.1 | 875 | tgctcttacaactacaatagttg | 259296 | - | see also 6258 line |
| 40859 | SLC37A1 | NM_018964.2 | 1447 | cggattctacgtggtaactcagg | 259320 | - | see also 7611 line |
| 40860 | SLC37A1 | NM_018964.2 | 1344 | gggagcgcctgccgattaggtat | 259313 | - | see also 7611 line |
| 40861 | SLC37A4 | NM_001467.2 | 735 | ttgggggctacagcctgtattac | 259334 | - | see also 18010 line |
| 40862 | SLC37A4 | NM_001467.2 | 736 | tgggggctacagcctgtattact | 259335 | - | see also 18010 line |
| 40863 | SLC7A7 | NM_003982.2 | 1150 | gggagcctctactcattttgaga | 259355 | - | see also 18011 line |
| 40864 | SLC7A7 | NM_003982.2 | 1146 | gccagggagcctctactcatttt | 259353 | - | see also 18011 line |
| 40865 | SLC7A7 | NM_003982.2 | 1077 | aagatattttcacctatgctaaa | 259351 | - | see also 18011 line |
| 40866 | SLC7A2 | NM_003046.1 | 474 | cccaagacggggtctgcatattt | 259367 | - | see also 18012 line |
| 40867 | SLC7A2 | NM_003046.1 | 904 | ctgaaaacggaacaagtatctat | 259380 | - | see also 18012 line |
| 40868 | SLC7A2 | NM_003046.1 | 1911 | tacttgatggtccagttaagtgc | 259396 | - | see also 18012 line |
| 40869 | SLC7A3 | NM_032803.3 | 899 | cagcgacctgtttctatgcattt | 259416 | - | see also 18013 line |
| 40870 | SLC7A3 | NM_032803.3 | 1383 | ctccctggtgtcgatttgtgttc | 259428 | - | see also 18013 line |
| 40871 | SLC7A5 | NM_003486.4 | 853 | tgcctatggaggatggaattact | 259449 | - | see also 18014 line |
| 40872 | SLC7A5 | NM_003486.4 | 1031 | gccgtggacttcgggaactatca | 259450 | - | see also 18014 line |
| 40873 | SLC7A8 | NM_012244.2 | 1238 | tcctcacatgggtcaactgttcc | 259457 | - | see also 5221 line |
| 40874 | SLC7A8 | NM_012244.2 | 1627 | gccgtcgctgtgactttggaga | 259466 | - | see also 5221 line |
| 40875 | SLC7A8 | NM_012244.2 | 1496 | agcttgttgatccctacaagaac | 259464 | - | see also 5221 line |
| 40876 | FLJ10815 | NM_018231.1 | 1038 | agccatcgttatcatcaagtaca | 259474 | - | see also 18016 line |
| 40877 | FLJ10815 | NM_018231.1 | 844 | ggcgaccagcaggacaagattat | 259472 | - | see also 18016 line |
| 40878 | FLJ39822 | NM_173512.1 | 1166 | tgcctcgggatagttctagaact | 259508 | - | see also 18017 line |
| 40879 | FLJ39822 | NM_173512.1 | 869 | atgtccatcgtgatttctgtatt | 259496 | - | see also 18017 line |
| 40880 | FLJ90709 | NM_173514.1 | 219 | ttgagccatcggatctaagatcc | 259529 | - | see also 10078 line |
| 40881 | FLJ90709 | NM_173514.1 | 1784 | ggcgtggctaacctgattgttca | 259575 | - | see also 10078 line |

Figure 46

| | | | | | | | |
|---|---|---|---|---|---|---|---|
| 40882 | MGC15523 | NM_138570.1 | 1294 | cccctctccggtttaaagcact | 259583 | - | see also 18019 line |
| 40883 | MGC15523 | NM_138570.1 | 1430 | cggcgctgatctacaagaaaatc | 259584 | - | see also 18019 line |
| 40884 | SLC12A8 | NM_024628.3 | 1253 | aacttcatgctgacatacgttgc | 259605 | - | see also 18020 line |
| 40885 | SLC12A8 | NM_024628.3 | 981 | tcctgatagcggaaaaggtatcc | 259603 | - | see also 18020 line |
| 40886 | SLC36A1 | NM_078483.1 | 309 | agcgctttggtcaaagcaatagc | 259624 | - | see also 9509 line |
| 40887 | SLC36A1 | NM_078483.1 | 978 | ttggcacagcgatttttttcattt | 259638 | - | see also 9509 line |
| 40888 | SLC36A4 | NM_152313.2 | 1303 | ttgtgccggagcaattcttattc | 259684 | - | see also 9840 line |
| 40889 | SLC38A3 | NM_006841.3 | 401 | ggccaatacgggcattatcctttt | 259697 | - | see also 18023 line |
| 40890 | SLC38A3 | NM_006841.3 | 402 | gccaatacgggcattatcctttt | 259698 | - | see also 18023 line |
| 40891 | SLC38A4 | NM_018018.2 | 1357 | gacggcctatgcaattcctatcc | 259750 | - | see also 7212 line |
| 40892 | SLC38A4 | NM_018018.2 | 1352 | tcccggacggcctatgcaattcc | 259748 | - | see also 7212 line |
| 40893 | SLC38A5 | NM_033518.1 | 2024 | tgccaaccatccgggatatcttt | 259786 | - | see also 18025 line |
| 40894 | SLC38A5 | NM_033518.1 | 954 | gccggtccagttcatggatttcg | 259775 | - | see also 18025 line |
| 40895 | SLC38A5 | NM_033518.1 | 2025 | gccaaccatccgggatatctttg | 259787 | - | see also 18025 line |
| 40896 | SLC7A10 | NM_019849.1 | 648 | cacgcgcatccaggacatgttca | 259792 | - | see also 18026 line |
| 40897 | SLC7A10 | NM_019849.1 | 1445 | gtggggtcggcgtcatcatcatc | 259799 | - | see also 18026 line |
| 40898 | SLC7A13 | NM_138817.1 | 324 | tgcagtggagctcaatactattt | 259809 | - | see also 18027 line |
| 40899 | SLC7A13 | NM_138817.1 | 1388 | cagcggattactattttacatac | 259847 | - | see also 18027 line |
| 40900 | SLC7A4 | NM_004173.1 | 1095 | ctccctgccacgcattgtctatg | 259854 | - | see also 18028 line |
| 40901 | SLC7A4 | NM_004173.1 | 1291 | ttcgtggccaccagtatcattgt | 259856 | - | see also 18028 line |
| 40902 | SLC7A6 | NM_003983.1 | 584 | cgggagccagctacgcttatatt | 259861 | - | see also 2721 line |
| 40903 | SLC7A6 | NM_003983.1 | 589 | gccagctacgcttatattctaga | 259863 | - | see also 2721 line |
| 40904 | SLC7A9 | NM_014270.1 | 1154 | agcgggcagactcatttacgtgg | 259907 | - | see also 18030 line |
| 40905 | SLC7A9 | NM_014270.1 | 1259 | tggtatcatagcaacgatttata | 259910 | - | see also 18030 line |
| 40906 | VIAAT | NM_080552.2 | 1018 | tgccttgcgccttccttaagaac | 259926 | - | see also 18031 line |
| 40907 | VIAAT | NM_080552.2 | 498 | gtcgagggagacatccattatca | 259921 | - | see also 18031 line |
| 40908 | SLC17A4 | NM_005495.1 | 111 | gtctaccggaccagatgtcaagg | 259934 | - | see also 18032 line |
| 40909 | SLC17A4 | NM_005495.1 | 628 | ggcatagcccaggttatggtatt | 259941 | - | see also 18032 line |
| 40910 | SLC17A4 | NM_005495.1 | 1373 | ctcggtacactggctttctcaaa | 259962 | - | see also 18032 line |
| 40911 | SLC17A7 | NM_020309.1 | 878 | acccctcacgaagtttagcact | 259970 | - | see also 18033 line |
| 40912 | SLC17A7 | NM_020309.1 | 1145 | acgtgcgcaagttgatgaactgc | 259971 | - | see also 18033 line |
| 40913 | SLC34A2 | NM_006424.1 | 1394 | ttggaatcggcgtgataaccatt | 260000 | - | see also 18034 line |
| 40914 | SLC34A2 | NM_006424.1 | 226 | tccacggctacactgatagatga | 259978 | - | see also 18034 line |
| 40915 | ANKH | NM_054027.3 | 1328 | cacgctctgtttcgtgatgtttt | 260038 | - | see also 9499 line |
| 40916 | ANKH | NM_054027.3 | 569 | gaccaaagccgtcctgtgtatgg | 260014 | - | see also 9499 line |

Figure 46

| 40917 | SLC26A1 | NM_022042.2 | 412 | agcccatctacagcctctatacg | 260043 | - | - | - | - |
|---|---|---|---|---|---|---|---|---|---|
| 40918 | SLC26A11 | NM_173626.1 | 1226 | agccttcgcatctcagaataatt | 260049 | - | see also 18036 line | | |
| 40919 | SLC26A11 | NM_173626.1 | 1225 | aagccttcgcatctcagaataat | 260048 | - | see also 18036 line | | |
| 40920 | SLC26A2 | NM_000112.1 | 850 | ctcaaccttcctcggactaatgg | 260071 | - | see also 95 line | | |
| 40921 | SLC26A2 | NM_000112.1 | 1964 | tgcatactatagtgattgactgc | 260111 | - | see also 95 line | | |
| 40922 | SLC26A4 | NM_000441.1 | 2251 | tgcgggtgattgtcaaagaattc | 260175 | - | see also 349 line | | |
| 40923 | SLC26A4 | NM_000441.1 | 486 | gtcatttcgggagttagtactgg | 260121 | - | see also 349 line | | |
| 40924 | SLC26A6 | NM_022911.2 | 738 | ctcttgtccgaggctataccaca | 260194 | - | see also 18039 line | | |
| 40925 | SLC26A6 | NM_022911.2 | 1656 | tgccagacacggatatttacaga | 260202 | - | see also 18039 line | | |
| 40926 | SLC26A7 | NM_052832.2 | 328 | ggcgactgcccattttggattgg | 260208 | - | see also 9433 line | | |
| 40927 | SLC26A7 | NM_052832.2 | 857 | atccggaccacttggattctttt | 260224 | - | see also 9433 line | | |
| 40928 | SLC26A8 | NM_052961.2 | 2304 | ttcacgggggttagtcgtattaa | 260315 | - | see also 18041 line | | |
| 40929 | SLC26A8 | NM_052961.2 | 2306 | cacggggttagtcgtattaaga | 260316 | - | see also 18041 line | | |
| 40930 | SLC26A9 | NM_052934.2 | 1191 | cggctacgacgtggattcgaacc | 260354 | - | see also 9454 line | | |
| 40931 | SLC26A9 | NM_052934.2 | 933 | tgctcgctacatgcacaagattc | 260350 | - | see also 9454 line | | |
| 40932 | SLC22A11 | NM_018484.2 | 943 | ccccaacattcgtcatctactgc | 260379 | - | see also 18042 line | | |
| 40933 | SLC22A11 | NM_018484.2 | 932 | caccatcttcgccccaacattcg | 260377 | - | see also 18042 line | | |
| 40934 | SLC22A11 | NM_018484.2 | 1855 | ctctcctctatggcgttatctcc | 260382 | - | see also 18042 line | | |
| 40935 | SLC22A7 | NM_006672.2 | 1074 | gtccgaagaccttcatacctaga | 260390 | - | see also 18043 line | | |
| 40936 | SLC22A7 | NM_006672.2 | 662 | ctccgtcagctatgtaatgtttg | 260388 | - | see also 18043 line | | |
| 40937 | SLCO1B1 | NM_006446.2 | 867 | atcaggataactcctactgattc | 260411 | - | see also 18044 line | | |
| 40938 | SLCO1B1 | NM_006446.2 | 1793 | ctcacatgtcatgctgattgtta | 260420 | - | see also 18044 line | | |
| 40939 | AFM | NM_001133.2 | 1211 | taccgttacgcggaagacaaatt | 260469 | - | see also 18045 line | | |
| 40940 | AFM | NM_001133.2 | 864 | tccgtgacacgagcaaggttatg | 260457 | - | see also 18045 line | | |
| 40941 | ECM1 | NM_004425.2 | 1521 | gtggtccccgacgtaacatctgg | 260509 | - | see also 2958 line | | |
| 40942 | ECM1 | NM_004425.2 | 1188 | gggaggatacccttgacaaatac | 260503 | - | see also 2958 line | | |
| 40943 | LOC90139 | NM_130783.1 | 634 | ctgggaaggagcctattcctaaa | 260517 | - | see also 18047 line | | |
| 40944 | LOC90139 | NM_130783.1 | 496 | gtcaacgggcctgaagactttaa | 260516 | - | see also 18047 line | | |
| 40945 | KCNJ3 | NM_002239.2 | 558 | gccttgcgtggccaatgtctata | 260526 | - | see also 1434 line | | |
| 40946 | KCNJ3 | NM_002239.2 | 1048 | cagcgaagcatgcaaactgaaca | 260534 | - | see also 1434 line | | |
| 40947 | KCNJ6 | NM_002240.2 | 1354 | ctggtgtcaccgctgatcattag | 260580 | - | see also 18049 line | | |
| 40948 | KCNJ6 | NM_002240.2 | 758 | atcgctacctgaccgatatcttc | 260560 | - | see also 18049 line | | |
| 40949 | KCNJ5 | NM_000890.3 | 1267 | cccggagctcctacatggataca | 260593 | - | see also 581 line | | |
| 40950 | KCNJ5 | NM_000890.3 | 635 | ttggtggctcattgcttatatcc | 260587 | - | see also 581 line | | |
| 40951 | KCNJ12 | NM_021012.2 | 1128 | tgcgaaggatctggtagagaaca | 260598 | - | see also 18051 line | | |
| 40952 | KCNJ12 | NM_021012.2 | 856 | atcaccatcttgcatgagattga | 260597 | - | see also 18051 line | | |
| 40953 | KCNJ13 | NM_002242.2 | 853 | ttccactaacgtactatcactcc | 260620 | - | see also 18052 line | | |

Figure 46

| 40954 | KCNJ13 | NM_002242.2 | 540 | atcgccttacttgccatacaaat | 260607 | - | see also 18052 line |
|---|---|---|---|---|---|---|---|
| 40955 | KCNJ14 | NM_013348.2 | 577 | cactgcaacgtgcgtttcgtaaa | 260630 | - | see also 18053 line |
| 40956 | KCNJ14 | NM_013348.2 | 579 | ctgcaacgtgcgtttcgtaaacc | 260631 | - | see also 18053 line |
| 40957 | KCNJ15 | NM_002243.3 | 758 | cacgaccttgattgagatcttca | 260643 | - | see also 18054 line |
| 40958 | KCNJ15 | NM_002243.3 | 820 | agcgggctgagaccatcaagttc | 260645 | - | see also 18054 line |
| 40959 | KCNJ15 | NM_002243.3 | 1183 | gccagagccgaacatcttatatc | 260646 | - | see also 18054 line |
| 40960 | KCNJ16 | NM_018658.1 | 776 | ttcatcatggcgatctattaaat | 260663 | - | see also 18055 line |
| 40961 | KCNJ16 | NM_018658.1 | 1138 | gcccaacttctccgctatacaga | 260673 | - | see also 18055 line |
| 40962 | KCNJ16 | NM_018658.1 | 1223 | tcctggtcacccgtaactatt | 260676 | - | see also 18055 line |
| 40963 | KCNJ2 | NM_000891.2 | 1645 | tacggcgagagtcggagatatga | 260720 | - | see also 18056 line |
| 40964 | KCNJ2 | NM_000891.2 | 1146 | gacagtggaatcgatcgtatatt | 260705 | - | see also 18056 line |
| 40965 | KCNJ2 | NM_000891.2 | 1151 | tggaatcgatcgtatatttctgg | 260707 | - | see also 18056 line |
| 40966 | KCNJ4 | NM_004981.1 | 893 | gacgaggacagcccgctttatgg | 260728 | - | see also 18057 line |
| 40967 | KCNJ4 | NM_004981.1 | 1085 | aaggtggactactcacgttttca | 260729 | - | see also 18057 line |
| 40968 | KCNJ8 | NM_004982.2 | 1303 | accgctttgtgtccattgtgact | 260744 | - | see also 18058 line |
| 40969 | KCNJ8 | NM_004982.2 | 589 | ggctgctcttcgctatcatgtgg | 260734 | - | see also 18058 line |
| 40970 | KCNJ9 | NM_004983.2 | 530 | gtggacgccgtgcgtcaacaacc | 260751 | - | see also 18059 line |
| 40971 | KCNJ9 | NM_004983.2 | 347 | gggcaacgtgcgcgagacatacc | 260749 | - | see also 18059 line |
| 40972 | KCNK1 | NM_002245.2 | 855 | accattggcctggggggattatgt | 260754 | - | see also 18060 line |
| 40973 | KCNK1 | NM_002245.2 | 903 | ttcagagagctctataagattgg | 260756 | - | see also 18060 line |
| 40974 | KCNK6 | NM_004823.1 | 427 | ccgtgggctatgggtacacaacg | 260760 | - | see also 18061 line |
| 40975 | KCNK6 | NM_004823.1 | 666 | ctggtgccggctgtgatctttgc | 260761 | - | see also 18061 line |
| 40976 | KCNQ5 | NM_019842.2 | 1700 | aacattacgtccatatgatgtaa | 260799 | - | see also 18062 line |
| 40977 | KCNQ5 | NM_019842.2 | 1868 | gaccacagacgatctcagtatgc | 260805 | - | see also 18062 line |
| 40978 | KCNQ5 | NM_019842.2 | 2082 | tcgggttccgcacaaaacagtgg | 260814 | - | see also 18062 line |
| 40979 | KCNE1 | NM_000219.1 | 334 | tgctatgtcgttgaaaaccatct | 260839 | - | see also 18063 line |
| 40980 | KCNE1 | NM_000219.1 | 267 | gtccgatgcctggcaagagaagg | 260836 | - | see also 18063 line |
| 40981 | KCNE1 | NM_000219.1 | 333 | gtgctatgtcgttgaaaaccatc | 260838 | - | see also 18063 line |
| 40982 | KCNQ1 | NM_000218.2 | 1484 | acggctatgacagttctgtaagg | 260845 | - | see also 18064 line |
| 40983 | KCNQ1 | NM_000218.2 | 1526 | aagtgagcatgccccatttcatg | 260846 | - | see also 18064 line |
| 40984 | KCNK2 | NM_014217.1 | 683 | ggctctgcctgcgatcatattca | 260885 | - | see also 18065 line |
| 40985 | KCNK2 | NM_014217.1 | 118 | gtgacacgaccattaatgttatg | 260861 | - | see also 18065 line |
| 40986 | KCNAB1 | NM_003471.2 | 808 | tgcaaatcgaccggacagtaaca | 260918 | - | see also 18066 line |
| 40987 | KCNAB1 | NM_003471.2 | 873 | accaaggcatggcgatgtactgg | 260923 | - | see also 18066 line |
| 40988 | KCNAB2 | NM_003636.2 | 1220 | atccacgagattgatagtatttt | 260954 | - | see also 18067 line |
| 40989 | KCNAB2 | NM_003636.2 | 581 | cacataatcgaaggtctgaaagc | 260947 | - | see also 18067 line |
| 40990 | KCNAB2 | NM_003636.2 | 1214 | tccattatccacgagattgatag | 260953 | - | see also 18067 line |

Figure 46

| | | | | | | | |
|---|---|---|---|---|---|---|---|
| 40991 | KCNAB3 | NM_004732.2 | 1095 | gtcgagtgcgagcagttgatag | 260969 | - | see also 18068 line |
| 40992 | KCNAB3 | NM_004732.2 | 1098 | gagtgcggagcagttgatagaac | 260971 | - | see also 18068 line |
| 40993 | KCNE2 | NM_172201.1 | 291 | ctgtacctcatggtgatgattgg | 260974 | - | see also 18069 line |
| 40994 | KCNE2 | NM_172201.1 | 343 | tggtgagcactgaaatccaag | 260975 | - | see also 18069 line |
| 40995 | KCNE3 | NM_005472.2 | 237 | gccgtgctgaaggctctaaatgc | 260980 | - | see also 3810 line |
| 40996 | KCNE4 | NM_080671.1 | 186 | ggcaacgagtacttcacattct | 260991 | | channel |
| 40997 | KCNE4 | NM_080671.1 | 575 | ctcggagaacatccatcagaatt | 260993 | - | see also 40996 line |
| 40998 | KCNE4 | NM_080671.1 | 218 | gtccttcacggcatttcttga | 260992 | - | see also 40996 line |
| 40999 | KCNG3 | NM_133329.4 | 1375 | tggcaatcacgccgtattacatc | 261002 | - | see also 9564 line |
| 41000 | KCNG3 | NM_133329.4 | 1376 | ggcaatcacgccgtattacatct | 261003 | - | see also 9564 line |
| 41001 | KCNH6 | NM_030779.2 | 1892 | ccgcctagccgtcaagttcaaga | 261035 | - | see also 18072 line |
| 41002 | KCNH6 | NM_030779.2 | 1894 | cgctagccgtcaagttcaagacc | 261036 | - | see also 18072 line |
| 41003 | KCNH6 | NM_030779.2 | 99 | cacgtcgctccccaaaacactta | 261022 | - | see also 18072 line |
| 41004 | KCNQ2 | NM_172106.1 | 944 | ggggaacgaccacttgacacc | 261044 | - | see also 18073 line |
| 41005 | KCNQ2 | NM_172106.1 | 912 | ggcctgttcctggtgtacttgg | 261042 | - | see also 18073 line |
| 41006 | KCNQ2 | NM_004518.2 | 2136 | caggcacggctgcattgtcaaga | 261048 | - | see also 18074 line |
| 41007 | KCNQ2 | NM_004518.2 | 2575 | gccaaagtcaggccctacattgc | 261050 | - | see also 18074 line |
| 41008 | KCNQ3 | NM_004519.2 | 1776 | cgccatcgagccgtcagaattc | 261074 | - | see also 3066 line |
| 41009 | KCNQ3 | NM_004519.2 | 1784 | gagccgtcagaattctacaattc | 261077 | - | see also 3066 line |
| 41010 | KCNQ4 | NM_004700.2 | 1822 | aactcgggtgaccaaattgtgg | 261108 | - | see also 18075 line |
| 41011 | KCNQ4 | NM_004700.2 | 1177 | ctggtactactatgacagtatcc | 261104 | - | see also 18075 line |
| 41012 | KCNV2 | NM_133497.2 | 645 | agcagacagacagaatacttcttc | 261112 | - | see also 18076 line |
| 41013 | KCNV2 | NM_133497.2 | 354 | cagagggcaactataactactac | 261111 | - | see also 18076 line |
| 41014 | KCNMB1 | NM_004137.2 | 721 | aggcagcgtggacaattaccaga | 261118 | - | see also 18077 line |
| 41015 | KCNMB1 | NM_004137.2 | 717 | tcccaggcagcgtggacaattac | 261117 | - | see also 18077 line |
| 41016 | KCNMB2 | NM_005832.3 | 563 | tgcgtccatcaacggaaacattta | 261126 | - | see also 18078 line |
| 41017 | KCNMB2 | NM_005832.3 | 425 | aggagaggaccgagctattctcc | 261121 | - | see also 18078 line |
| 41018 | KCNMB3 | NM_014407.3 | 732 | tcttgctcggaacaaccattct | 261147 | - | see also 18079 line |
| 41019 | KCNMB3 | NM_014407.3 | 736 | tgctcggaacaaccattctaaag | 261149 | - | see also 18079 line |
| 41020 | KCNMB4 | NM_014505.4 | 502 | agcatccgctcggcttgttct | 261164 | - | see also 18080 line |
| 41021 | KCNMB4 | NM_014505.4 | 507 | ccggctcggcttgtttctcatca | 261165 | - | see also 18080 line |
| 41022 | HCN1 | NM_021072.1 | 1452 | ttgctaatgcggatcctaatttt | 261206 | - | see also 8077 line |
| 41023 | HCN1 | NM_021072.1 | 1790 | gaccgactagatcgaataggaaa | 261219 | - | see also 8077 line |
| 41024 | HCN1 | NM_021072.1 | 822 | tgcgtttattacgactttcaagg | 261188 | - | see also 8077 line |
| 41025 | KCNK10 | NM_021161.3 | 2031 | gagccggagaacaactcattact | 261266 | - | see also 8114 line |
| 41026 | KCNK10 | NM_021161.3 | 1235 | gacggccttgagtccatttact | 261247 | - | see also 8114 line |
| 41027 | KCNK12 | NM_022055.1 | 370 | ggcaccgtggtgtcaaccatagg | 261269 | - | see also 18083 line |

Figure 46

| | | | | | | | | | |
|---|---|---|---|---|---|---|---|---|---|
| 41028 | KCNK12 | NM_022055.1 | 854 | tgctcggcgtgtgctgcatttac | 261270 | - | see also 18083 line | | |
| 41029 | KCNK13 | NM_022054.2 | 1082 | gccatgtacacccccattgaagg | 261273 | - | see also 8253 line | | |
| 41030 | KCNK13 | NM_022054.2 | 1398 | tccggaaccgctgcaacatctcc | 261277 | - | see also 8253 line | | |
| 41031 | KCNK15 | NM_022358.2 | 418 | cccgctgacgctggtcactttcc | 261282 | - | channel | - | - |
| 41032 | KCNK16 | NM_032115.2 | 595 | ttcagcgagggcttctactttgc | 261284 | - | channel | - | - |
| 41033 | KCNK16 | NM_032115.2 | 74 | tgctgctcggtgccactatcttc | 261283 | - | see also 41032 line | | |
| 41034 | KCNK17 | NM_031460.2 | 860 | tggcatggctggccttgatcatc | 261288 | - | see also 18085 line | | |
| 41035 | KCNK17 | NM_031460.2 | 1032 | gggacccatgggaatcatacagc | 261293 | - | see also 18085 line | | |
| 41036 | KCNK3 | NM_002246.1 | 479 | cccgctcacgctcgtcatgttcc | 261294 | - | channel | voltage-gated ion channel | - |
| 41037 | KCNK4 | NM_016611.2 | 702 | cacgttcgtgttctgctatatgg | 261307 | - | see also 18086 line | | |
| 41038 | KCNK4 | NM_016611.2 | 625 | gtgccaccggagctagtaagagt | 261304 | - | see also 18086 line | | |
| 41039 | KCNK5 | NM_003740.2 | 1647 | gtcctcgctagaggacaacttgg | 261321 | - | see also 2553 line | | |
| 41040 | KCNK5 | NM_003740.2 | 611 | ttgcagcgaccgtcattaccacc | 261314 | - | see also 2553 line | | |
| 41041 | KCNK7 | NM_005714.1 | 921 | ctgggccagctcgcacttcttgg | 261325 | - | see also 18088 line | | |
| 41042 | KCNK9 | NM_016601.2 | 1098 | gcctcttcccatcgcctattagc | 261335 | - | see also 18089 line | | |
| 41043 | KCNK9 | NM_016601.2 | 477 | acctgctgaagcgcattaagaag | 261329 | - | see also 18089 line | | |
| 41044 | KCNK9 | NM_016601.2 | 201 | tccggatcaaggggaagtacaac | 261328 | - | see also 18089 line | | |
| 41045 | CACNA2D1 | NM_000722.1 | 2083 | atcatgtaacgcggatttgatta | 261419 | - | see also 471 line | | |
| 41046 | CACNA2D1 | NM_000722.1 | 115 | cccttcggccgtcactatcaaat | 261337 | - | see also 471 line | | |
| 41047 | CACNA2D1 | NM_000722.1 | 671 | ggcctagctcgatattatccagc | 261366 | - | see also 471 line | | |
| 41048 | CACNG1 | NM_000727.2 | 481 | tgcgacccgcgtccatgttctat | 261465 | - | see also 18091 line | | |
| 41049 | CACNG1 | NM_000727.2 | 581 | caccgtctggatcgagtactatt | 261469 | - | see also 18091 line | | |
| 41050 | CACNB1 | NM_000723.3 | 951 | cactcgtgtgacggcagatattt | 261477 | - | see also 18092 line | | |
| 41051 | CACNB1 | NM_000723.3 | 1924 | tggggacgaggcgtctacattcg | 261483 | - | see also 18092 line | | |
| 41052 | CACNB2 | NM_000724.1 | 533 | gagtacgggtgtcctatggttcg | 261484 | - | see also 475 line | | |
| 41053 | CACNB2 | NM_000724.1 | 1766 | gccgtacattagccacttcaagt | 261524 | - | see also 475 line | | |
| 41054 | CACNB4 | NM_000726.1 | 867 | aagaggtctgtcctaaataatcc | 261547 | - | see also 18094 line | | |
| 41055 | CACNB4 | NM_000726.1 | 690 | gagcacattcctccttacgatgt | 261544 | - | see also 18094 line | | |
| 41056 | CACNG2 | NM_006078.2 | 866 | tgctggcggtgcacatgtttatc | 261567 | - | see also 4221 line | | |
| 41057 | CACNG2 | NM_006078.2 | 870 | ggcggtgcacatgtttatcgacc | 261568 | - | see also 4221 line | | |
| 41058 | CACNG3 | NM_006539.2 | 1285 | ggcacggactactggttatattc | 261574 | - | see also 4572 line | | |
| 41059 | CACNG4 | NM_014405.2 | 247 | gggcactgcttccggatcaatca | 261600 | - | see also 5663 line | | |
| 41060 | CACNG5 | NM_014404.1 | 259 | tccgagtccacggtcaatgttct | 261611 | - | see also 18098 line | | |

Figure 46

| 41061 | CACNG5 | NM_014404.1 | 267 | cacggtcaatgttctaaagatga | 261614 | - | see also 18098 line |
|---|---|---|---|---|---|---|---|
| 41062 | CACNG7 | NM_031896.3 | 248 | ttcttgaaccggagatcaatttg | 261628 | - | see also 9048 line |
| 41063 | CACNG8 | NM_031895.4 | 446 | cacgacagcgcggagtatctact | 261641 | - | see also 9047 line |
| 41064 | CACNG8 | NM_031895.4 | 401 | gtctgcgtgaagatcaatcattt | 261639 | - | see also 9047 line |
| 41065 | TRPC1 | NM_003304.3 | 2243 | ctgcctagtgcatcgttacttga | 261718 | - | see also 2233 line |
| 41066 | TRPC1 | NM_003304.3 | 2245 | gcctagtgcatcgttacttgact | 261719 | - | see also 2233 line |
| 41067 | TRPC3 | NM_003305.1 | 1052 | cactcacgctcgaggatcaatgc | 261727 | - | see also 2243 line |
| 41068 | TRPC3 | NM_003305.1 | 1336 | ttcattaagtcgtgtcaaacttg | 261730 | - | see also 2243 line |
| 41069 | TRPC3 | NM_003305.1 | 2740 | aaacacgttatcagcagataatga | 261764 | - | see also 2243 line |
| 41070 | TRPC5 | NM_012471.1 | 2811 | tgccgatcatgctgatatcgagt | 261804 | - | see also 5345 line |
| 41071 | TRPC5 | NM_012471.1 | 2698 | cacgaattcaccgagtttgtagg | 261802 | - | see also 5345 line |
| 41072 | TRPC5 | NM_012471.1 | 2115 | cccaactgtcgtggaatggatga | 261782 | - | see also 5345 line |
| 41073 | TRPC6 | NM_004621.3 | 696 | atcgctccacaagcctatctata | 261834 | - | see also 3141 line |
| 41074 | TRPC6 | NM_004621.3 | 787 | atgccactcactcaacgttaact | 261840 | - | see also 3141 line |
| 41075 | TRPM1 | NM_002420.3 | 4684 | ctcgcttgtccctaaccattact | 261979 | - | see also 18104 line |
| 41076 | TRPM1 | NM_002420.3 | 4398 | ttccccgatgaaacgatcaatgc | 261967 | - | see also 18104 line |
| 41077 | TRPM1 | NM_002420.3 | 3403 | tccagcgatatcagctgattatg | 261943 | - | see also 18104 line |
| 41078 | TRPM3 | NM_020952.1 | 5 | atgtgcgagtatcttttgataca | 261986 | - | see also 8046 line |
| 41079 | TRPM3 | NM_020952.1 | 427 | gacaacgggaccactggaaaata | 261995 | - | see also 8046 line |
| 41080 | SCN11A | NM_014139.1 | 4573 | tccgacgctcttcagaattgtcc | 262208 | - | see also 5511 line |
| 41081 | SCN11A | NM_014139.1 | 5287 | ctctgatggcctagatagtatga | 262224 | - | see also 5511 line |
| 41082 | SCN11A | NM_014139.1 | 1070 | atcagtaacccggaagcttatga | 262124 | - | see also 5511 line |
| 41083 | SCN1B | NM_001037.2 | 395 | accgcctgctcttcttcgaaaac | 262230 | - | see also 639 line |
| 41084 | SCN1B | NM_001037.2 | 398 | gcctgctcttcttcgaaaactac | 262231 | - | see also 639 line |
| 41085 | SCN2B | NM_004588.2 | 472 | aggatgaggggatttacaactgc | 262243 | - | see also 18108 line |
| 41086 | SCN2B | NM_004588.2 | 155 | cacggggctcagtctcttttct | 262239 | - | see also 18108 line |
| 41087 | SCN3B | NM_018400.2 | 998 | gcccgagggcggtaaagatttcc | 262248 | - | see also 7447 line |
| 41088 | SCN3B | NM_018400.2 | 1320 | ctgctcatcgagatgatatattg | 262253 | - | see also 7447 line |
| 41089 | SCN5A | NM_000335.3 | 4904 | cacaggcgagtgtattgtcaagc | 262294 | - | see also 18110 line |
| 41090 | SCN5A | NM_000335.3 | 2917 | aggtgtcggggcagtcattatgc | 262278 | - | see also 18110 line |
| 41091 | SCN7A | NM_002976.1 | 3832 | accagtacctcgcccattaaaca | 262390 | - | see also 18111 line |
| 41092 | SCN7A | NM_002976.1 | 1551 | gcccattatactggtataagttt | 262329 | - | see also 18111 line |
| 41093 | SCN7A | NM_002976.1 | 740 | ttcattccaacgcttcaaactgc | 262312 | - | see also 18111 line |
| 41094 | ACCN2 | NM_001095.2 | 1688 | agcctggacgacgtcaaaagaca | 262449 | - | see also 18112 line |
| 41095 | ACCN2 | NM_001095.2 | 412 | cacggagcgtgtgcagtactact | 262441 | - | see also 18112 line |
| 41096 | ACCN2 | NM_001095.2 | 1597 | cgcctacgaggtcattaagcaca | 262447 | - | see also 18112 line |
| 41097 | ACCN3 | NM_004769.1 | 209 | ctgagagggtgcgctactacagg | 262451 | - | see also 18113 line |

Figure 46

| 41098 | ACCN3 | NM_004769.1 | 882 | atctctgaaccccaactatgagc | 262454 | - | see also 18113 line |
|---|---|---|---|---|---|---|---|
| 41099 | APXL | NM_001649.2 | 524 | gccgacaccacgcgagttcttcc | 262463 | - | see also 1092 line |
| 41100 | APXL | NM_001649.2 | 4662 | tcccggtgatcggcaatcactgc | 262486 | - | see also 1092 line |
| 41101 | APXL | NM_001649.2 | 580 | aacctacagcgcaccttagatca | 262464 | - | see also 1092 line |
| 41102 | SCNN1B | NM_000336.1 | 1203 | gcccgtgcaccgtgaatggttct | 262499 | - | see also 18115 line |
| 41103 | SCNN1B | NM_000336.1 | 1222 | ttctgaggtccccgtccaaaact | 262500 | - | see also 18115 line |
| 41104 | SCNN1D | NM_002978.2 | 945 | gtcctctcctgcagttacgatgg | 262512 | - | see also 18116 line |
| 41105 | SCNN1D | NM_002978.2 | 624 | gagtttgccagggagaacattga | 262510 | - | see also 18116 line |
| 41106 | SCNN1G | NM_001039.1 | 1796 | acgatgacctacccactttcaac | 262535 | - | see also 18117 line |
| 41107 | SCNN1G | NM_001039.1 | 651 | caccttcagctcgggaatcaatg | 262520 | - | see also 18117 line |
| 41108 | ACCN4 | NM_018674.3 | 910 | agctcgccgacatgcttaagagc | 262536 | - | see also 7548 line |
| 41109 | ACCN4 | NM_018674.3 | 1346 | ctgccggctgcgctgtgaaaagg | 262542 | - | see also 7548 line |
| 41110 | ACCN5 | NM_017419.1 | 326 | aacgtccattgaggttcaatatg | 262557 | - | see also 18119 line |
| 41111 | ACCN5 | NM_017419.1 | 1313 | tagtgacctaaactataagataa | 262577 | - | see also 18119 line |
| 41112 | CATSPER1 | NM_053054.2 | 311 | ccctccggagttccaagacttcc | 262589 | - | see also 9488 line |
| 41113 | CATSPER1 | NM_053054.2 | 245 | cagagctctccaccattacgagt | 262588 | - | see also 9488 line |
| 41114 | CATSPER2 | NM_054020.2 | 400 | accagctagtgcgtttctctata | 262618 | - | see also 9497 line |
| 41115 | CATSPER2 | NM_054020.2 | 339 | cacactatcagggagttacttga | 262614 | - | see also 9497 line |
| 41116 | CATSPER3 | NM_178019.1 | 1100 | tagcttacccttcatcgatatct | 262656 | - | see also 18122 line |
| 41117 | CATSPER3 | NM_178019.1 | 88 | agcgccactcgagagtcatttct | 262635 | - | see also 18122 line |
| 41118 | MCOLN3 | NM_018298.9 | 452 | gtgacgtgtatgatcagttaatc | 262672 | - | see also 18123 line |
| 41119 | MCOLN3 | NM_018298.9 | 1591 | ctgatcactgatacatacgaaac | 262712 | - | see also 18123 line |
| 41120 | MGC15619 | NM_032369.1 | 1013 | tccagcccgacaagaataactat | 262719 | - | see also 18124 line |
| 41121 | MGC15619 | NM_032369.1 | 1307 | aacggcaactcttaaggttaaaa | 262728 | - | see also 18124 line |
| 41122 | NOLA1 | NM_018983.2 | 505 | ctccagaacgtgtagtcttatta | 262734 | - | see also 18125 line |
| 41123 | NOLA1 | NM_018983.2 | 503 | acctccagaacgtgtagtcttat | 262733 | - | see also 18125 line |
| 41124 | TPCN1 | NM_017901.3 | 2324 | gacgatcattgtcgcctttatcc | 262762 | - | see also 18126 line |
| 41125 | TPCN1 | NM_017901.3 | 889 | cccgagcactgcgctgcattttc | 262739 | - | see also 18126 line |
| 41126 | TPCN1 | NM_017901.3 | 1799 | ttccggatggaacttgtttgact | 262756 | - | see also 18126 line |
| 41127 | TPCN2 | NM_139075.1 | 325 | gacggtattactcgaacgtatgc | 262770 | - | see also 18127 line |

Figure 46

| 41128 | TPCN2 | NM_139075.1 | 314 | gtggctttaccgacggtattact | 262768 | - | see also 18127 line | | |
| 41129 | TRPM4 | NM_017636.2 | 3438 | aacgtgggaatcggtgcataagg | 262810 | - | see also 18128 line | | |
| 41130 | TRPM4 | NM_017636.2 | 1056 | aggcgaagcccgagatcgaatca | 262799 | - | see also 18128 line | | |
| 41131 | TRPM5 | NM_014555.2 | 1719 | cacgcgcgaggcgaaatacgagc | 262816 | - | see also 18129 line | | |
| 41132 | TRPM5 | NM_014555.2 | 1716 | agccacgcgcgaggcgaaatacg | 262815 | - | see also 18129 line | | |
| 41133 | TRPM5 | NM_014555.2 | 2546 | cgctgcggctgatccatatcttt | 262820 | - | see also 18129 line | | |
| 41134 | VGCNL1 | NM_052867.1 | 1522 | tccgagtagttcggctgattaag | 262883 | - | see also 18130 line | | |
| 41135 | VGCNL1 | NM_052867.1 | 1440 | ttcgaactactactcgtaattgg | 262878 | - | see also 18130 line | | |
| 41136 | CLCA1 | NM_001285.1 | 2891 | ttgataaggtcgatctgaaatca | 263041 | - | see also 18131 line | | |
| 41137 | CLCA1 | NM_001285.1 | 1454 | gggacacactcgccaaaagatta | 263013 | - | see also 18131 line | | |
| 41138 | CLCA2 | NM_006536.3 | 620 | ggggtgtgttcgatgagtataac | 263060 | - | see also 4569 line | | |
| 41139 | CLCA2 | NM_006536.3 | 118 | aggagcattgcaggtcctatttg | 263048 | - | see also 4569 line | | |
| 41140 | CLCA4 | NM_012128.2 | 562 | ttctaccgtgctaagtcaaaaaa | 263150 | - | see also 18133 line | | |
| 41141 | CLCA4 | NM_012128.2 | 2125 | ctgaatagagccgcgtacatacc | 263183 | - | see also 18133 line | | |
| 41142 | CLCN1 | NM_000083.1 | 1726 | ttcgaattaacgggtcagattgc | 263231 | - | see also 55 line | | |
| 41143 | CLCN1 | NM_000083.1 | 2746 | ttccggaacacgacttcaactcg | 263237 | - | see also 55 line | | |
| 41144 | CLCN1 | NM_000083.1 | 1346 | cccgcgaagccatcagtactttg | 263227 | - | see also 55 line | | |
| 41145 | CLCN2 | NM_004366.2 | 751 | tgcttcgcggcacctattggagg | 263250 | - | see also 2927 line | | |
| 41146 | CLCN2 | NM_004366.2 | 2476 | ctcattggaatcgttactctaaa | 263261 | - | see also 2927 line | | |
| 41147 | CLCN3 | NM_001829.1 | 1735 | gtcgacgcaagtccacgaaattt | 263293 | - | see also 1182 line | | |
| 41148 | CLCN3 | NM_001829.1 | 2176 | aggtcggggctgattgcattaca | 263305 | - | see also 1182 line | | |
| 41149 | CLCN4 | NM_001830.2 | 1203 | ggcgtgcttttcagtctagaaga | 263336 | - | see also 1188 line | | |
| 41150 | CLCN4 | NM_001830.2 | 2641 | accccgaatccatcatgtttaat | 263355 | - | see also 1188 line | | |
| 41151 | CLCN5 | NM_000084.1 | 1159 | ttcactctacgctccatcaatcc | 263384 | - | see also 56 line | | |
| 41152 | CLCN5 | NM_000084.1 | 498 | ttcgttagctggtttgatagaca | 263370 | - | see also 56 line | | |
| 41153 | CLCN5 | NM_000084.1 | 1192 | agccgcctggtactattttatgt | 263387 | - | see also 56 line | | |
| 41154 | CLCN6 | NM_001286.1 | 692 | acggaagatccagtttaacttcc | 263418 | - | see also 847 line | | |
| 41155 | CLCN6 | NM_021736.1 | 969 | ggcgagtttaagagtcttagaga | 263445 | - | see also 18138 line | | |
| 41156 | CLCN7 | NM_001287.3 | 646 | cagcggaatcccccagatcaagt | 263448 | - | see also 18139 line | | |
| 41157 | CLCN7 | NM_001287.3 | 1228 | ctggctgaccatgtttcgaatca | 263460 | - | see also 18139 line | | |
| 41158 | CLCNKA | NM_004070.2 | 1930 | accaggcacaaaacctctttaag | 263468 | - | see also 18140 line | | |
| 41159 | CLCNKA | NM_004070.2 | 1922 | caccttgcaccaggcacaaaacc | 263467 | - | see also 18140 line | | |
| 41160 | CLCNKB | NM_000085.1 | 1353 | ttggggagactctctcttttatc | 263470 | - | channel | voltage-gated chloride channel | Bartter syndrome |
| 41161 | CLCNKB | NM_000085.1 | 1310 | ttcatgcccatctttgtctatgg | 263469 | - | see also 41160 line | | |
| 41162 | CLCNKB | NM_000085.1 | 1356 | gggagactctctcttttatcttc | 263471 | - | see also 41160 line | | |
| 41163 | CLIC1 | NM_001288.3 | 600 | aacccagcactcaatgacaatct | 263477 | - | see also 848 line | | |

Figure 46

| | | | | | | | | | |
|---|---|---|---|---|---|---|---|---|---|
| 41164 | CLIC1 | NM_001288.3 | 596 | ttcaaacccagcactcaatgaca | 263476 | - | see also 848 line | | |
| 41165 | CLIC2 | NM_001289.3 | 754 | agctaacactggctgattgtagc | 263495 | - | see also 18141 line | | |
| 41166 | CLIC2 | NM_001289.3 | 428 | tcctccgttcctggtgtataaca | 263488 | - | see also 18141 line | | |
| 41167 | CLIC2 | NM_001289.3 | 848 | ctcaggagtctggcgttatctcc | 263498 | - | see also 18141 line | | |
| 41168 | CLIC3 | NM_004669.2 | 653 | cgcgatgcaggagaaagagttca | 263500 | - | channel、signal_transduction | voltage-gated chloride channel | - |
| 41169 | CLIC3 | NM_004669.2 | 98 | ctcctgccagcggctcttcatgg | 263499 | - | see also 41168 line | | |
| 41170 | CLIC4 | NM_013943.1 | 855 | tacctaactaatgcatacagtag | 263510 | - | see also 18142 line | | |
| 41171 | CLIC4 | NM_013943.1 | 408 | cccgggacccacccaccatttat | 263504 | - | see also 18142 line | | |
| 41172 | CLIC5 | NM_016929.1 | 635 | cagcgggcatcgacatctttcc | 263522 | - | see also 18143 line | | |
| 41173 | CLIC5 | NM_016929.1 | 536 | acgtgaagacagacgtcaataag | 263518 | - | see also 18143 line | | |
| 41174 | CLIC6 | NM_053277.1 | 1366 | ttcgtcaaggctggttatgatgg | 263528 | - | see also 9489 line | | |
| 41175 | CLIC6 | NM_053277.1 | 1435 | ctctggctgaaaggcgttatatt | 263532 | - | see also 9489 line | | |
| 41176 | LGICZ | NM_180990.2 | 257 | gtggacatcctgcgatacacaat | 263551 | - | see also 18145 line | | |
| 41177 | LGICZ | NM_180990.2 | 901 | ctgcaacccactgctcatttact | 263561 | - | see also 18145 line | | |
| 41178 | FXYD1 | NM_005031.3 | 279 | aagatgccggtgcaagttcaacc | 263566 | - | see also 18146 line | | |
| 41179 | FXYD1 | NM_005031.3 | 108 | ggcgtctcttggccacatcttgg | 263564 | - | see also 18146 line | | |
| 41180 | FLJ12770 | NM_032174.3 | 821 | tacactgggtagctacattgaat | 263578 | - | see also 18147 line | | |
| 41181 | FLJ12770 | NM_032174.3 | 307 | ctgcaccgtctatgcaaagatgt | 263569 | - | see also 18147 line | | |
| 41182 | VDAC1 | NM_003374.1 | 474 | gggctgcgacatggatttcgaca | 263590 | - | see also 18148 line | | |
| 41183 | VDAC1 | NM_003374.1 | 293 | ctgagtacggcctgacgtttaca | 263588 | - | see also 18148 line | | |
| 41184 | VDAC3 | NM_005662.3 | 823 | ctgattggactgggttatactca | 263606 | - | see also 18149 line | | |
| 41185 | VDAC3 | NM_005662.3 | 870 | gactttatcagctttaatcgatg | 263610 | - | see also 18149 line | | |
| 41186 | FXYD6 | NM_022003.1 | 213 | ctcggttgggatcctccttatcc | 263612 | - | see also 18151 line | | |
| 41187 | FXYD7 | NM_022006.1 | 268 | acctgcaaatcctgtaagtctga | 263617 | - | see also 18152 line | | |
| 41188 | FXYD7 | NM_022006.1 | 122 | aggaacctgacccattttactat | 263614 | - | see also 18152 line | | |
| 41189 | MLC1 | NM_015166.2 | 417 | tcccggctgagatggattacttg | 263618 | - | see also 6260 line | | |
| 41190 | MLC1 | NM_015166.2 | 549 | ctgtgaccactacgtgtttaatt | 263622 | - | see also 6260 line | | |
| 41191 | TM4SF11 | NM_015993.1 | 132 | gccgagttcccgtcgaaagttag | 263637 | - | see also 18154 line | | |
| 41192 | TM4SF11 | NM_015993.1 | 455 | cagcgccaccgttctctacatca | 263639 | - | see also 18154 line | | |
| 41193 | CX36 | NM_020660.1 | 311 | agcgagaacgccgctactctaca | 263641 | - | see also 18155 line | | |
| 41194 | CX36 | NM_020660.1 | 876 | gtcaatctatgagattcgtaaca | 263659 | - | see also 18155 line | | |
| 41195 | CX40.1 | NM_153368.1 | 331 | agccgggatgcgccaatgtttgc | 263663 | - | see also 18156 line | | |
| 41196 | CX40.1 | NM_153368.1 | 335 | gggatgcgccaatgtttgctacg | 263664 | - | see also 18156 line | | |
| 41197 | CX62 | NM_032602.1 | 865 | cagcaagtcattcgagttaatgt | 263691 | - | see also 18157 line | | |
| 41198 | CX62 | NM_032602.1 | 453 | atgtctgctgcgtacttatgtct | 263679 | - | see also 18157 line | | |
| 41199 | GJA1 | NM_000165.2 | 722 | tccagtggtacatctatggattc | 263718 | - | see also 18158 line | | |

EP 1 752 536 A1

Figure 46

| No. | Gene | Accession | Pos | Sequence | | ID | | Notes | Function |
|---|---|---|---|---|---|---|---|---|---|
| 41200 | GJA1 | NM_000165.2 | 659 | tgctgcgaacctactacatcagt | ` | 263715 | - | see also 18158 line | |
| 41201 | GJA4 | NM_002060.1 | 115 | ctgaccgtggtgggtaagatct | | 263736 | - | | connexon channel / atherosclerosis |
| 41202 | GJA5 | NM_005266.4 | 694 | caccccggtcaactgttacgtatc | | 263742 | - | see also 18160 line | |
| 41203 | GJA5 | NM_005266.4 | 695 | accccggtcaactgttaagtatcc | | 263743 | - | see also 18160 line | |
| 41204 | GJA5 | NM_005266.4 | 690 | tccccaccccggtcaactgttacg | | 263741 | - | see also 18160 line | |
| 41205 | GJA7 | NM_005497.1 | 903 | cacgaactgtccaatgctaaga | | 263772 | - | see also 3836 line | |
| 41206 | GJA7 | NM_005497.1 | 808 | gaggatccgggtgcttataatta | | 263766 | - | see also 3836 line | |
| 41207 | GJB1 | NM_000166.2 | 160 | ggcgtgaaccggcattcctactgc | | 263775 | - | see also 136 line | |
| 41208 | GJB1 | NM_000166.2 | 309 | cagcgtttgctatgaccaatct | | 263781 | - | see also 136 line | |
| 41209 | GJB2 | NM_004004.2 | 815 | atgtcactgaattgtgttatttg | | 263791 | - | see also 18162 line | |
| 41210 | GJB2 | NM_004004.2 | 522 | gggggagataaagagtgaattta | | 263790 | - | see also 18162 line | |
| 41211 | GJB4 | NM_153212.1 | 769 | cccgatacgtgcccaccatatgt | | 263800 | - | see also 18163 line | |
| 41212 | GJB4 | NM_153212.1 | 528 | ccgcctcacaaggattatgaca | | 263797 | - | see also 18163 line | |
| 41213 | GJB5 | NM_005268.2 | 278 | tgctccaacgtctgctttgatga | | 263805 | - | see also 18164 line | |
| 41214 | GJB5 | NM_005268.2 | 595 | cgcagatccagtccaatatag | | 263808 | - | see also 18164 line | |
| 41215 | GJE1 | NM_181538.1 | 515 | aaccttgccttgtagtataacc | | 263812 | - | see also 18165 line | |
| 41216 | GJE1 | NM_181538.1 | 594 | cagcggtttctgtctctgttta | | 263813 | - | see also 18165 line | |
| 41217 | AQP2 | NM_000486.3 | 662 | gtcgtcactggcaaattgatga | | 263816 | - | | water channel / diabetes insipidus |
| 41218 | AQP2 | NM_000486.3 | 252 | tacaggcctcggccacataagc | | 263815 | - | see also 41217 line | |
| 41219 | AQP4 | NM_001650.3 | 246 | accggtcgacatggttcatct | | 263818 | - | see also 1093 line | |
| 41220 | AQP4 | NM_001650.3 | 605 | ctggctcaatagctttagcaatt | | 263826 | - | see also 1093 line | |
| 41221 | AQP5 | NM_001651.1 | 1036 | accttgtcggaatctactttcact | | 263844 | - | see also 18167 line | |
| 41222 | AQP5 | NM_001651.1 | 1031 | gggccaccttgtcgggaatctact | | 263842 | - | see also 18167 line | |
| 41223 | AQP6 | NM_001652.2 | 1012 | tcctggcctcactgatctacaac | | 263849 | - | see also 18168 line | |
| 41224 | AQP6 | NM_001652.2 | 1015 | tggcctcactgatctacaacttc | | 263850 | - | see also 18168 line | |
| 41225 | AQP8 | NM_001169.1 | 800 | tgcttgttggactcgtcattagg | | 263858 | - | see also 18169 line | |
| 41226 | AQP8 | NM_001169.1 | 237 | cgggtgcctgtcgtcattgaga | | 263854 | - | see also 18169 line | |
| 41227 | AQP9 | NM_020980.2 | 561 | gtctctttggacggatgaaatgg | | 263867 | - | see also 18170 line | |
| 41228 | AQP9 | NM_020980.2 | 640 | aaccgtctttggcatttactatg | | 263869 | - | see also 18170 line | |
| 41229 | SLC14A1 | NM_015865.1 | 713 | tggcgttgtcaatgtacctttca | | 263885 | - | see also 18171 line | |
| 41230 | SLC14A1 | NM_015865.1 | 1114 | gccccgttcacggcctatcttgg | | 263897 | - | see also 18171 line | |
| 41231 | AQP1 | NM_000385.3 | 332 | tccgtgccctcatgtacatcatc | | 263903 | - | see also 18172 line | |
| 41232 | AQP1 | NM_000385.3 | 654 | cgcggtgatcacacacacaacttca | | 263906 | - | see also 18172 line | |
| 41233 | AQP10 | NM_080429.2 | 224 | ggctctctggccgttacgatagc | | 263910 | - | see also 18173 line | |
| 41234 | AQP10 | NM_080429.2 | 328 | ctgggtcaagctcccccatttaca | | 263911 | - | see also 18173 line | |
| 41235 | FLJ20130 | NM_017681.1 | 442 | tgcttgggacctacacattgattg | | 263929 | - | see also 18174 line | |
| 41236 | FLJ20130 | NM_017681.1 | 333 | tagcaactcctgtgatgacatat | | 263922 | - | see also 18174 line | |

Figure 46

| | | | | | | | |
|---|---|---|---|---|---|---|---|
| 41237 | LOC129401 | NM_138285.2 | 521 | agccacgaaagacgacattatct | 263945 | - | see also 18175 line |
| 41238 | LOC129401 | NM_138285.2 | 520 | cagccacgaaagacgacattatc | 263944 | - | see also 18175 line |
| 41239 | NUP214 | NM_005085.2 | 1093 | atccttgctcgacaaagtgatca | 263998 | - | see also 18176 line |
| 41240 | NUP214 | NM_005085.2 | 1199 | tgggagttgtcgtagactataca | 264003 | - | see also 18176 line |
| 41241 | NUP50 | NM_007172.2 | 955 | tgctccgtgcgggattggatagt | 264083 | - | see also 18177 line |
| 41242 | NUP50 | NM_007172.2 | 1257 | agcgacaagtgcctcatttaatt | 264091 | - | see also 18177 line |
| 41243 | NUP54 | NM_017426.2 | 1566 | atcattaaagacgatctagaaga | 264143 | - | see also 18178 line |
| 41244 | NUP54 | NM_017426.2 | 862 | ttgagcgttcgccaaatggtact | 264111 | - | see also 18178 line |
| 41245 | PCNT1 | NM_024844.2 | 1284 | gagtacgcctcgggactgtttgc | 264176 | - | see also 18179 line |
| 41246 | PCNT1 | NM_024844.2 | 231 | atctatatcatccgtaaggatgt | 264152 | - | see also 18179 line |
| 41247 | ROPN1 | NM_017578.2 | 918 | ggccctgatggtataatcacagt | 264197 | - | kinase_related、signal_transduction |
| 41248 | SYNPR | NM_144642.3 | 533 | aacggatcccaaggaagtattgc | 264206 | - | see also 18180 line |
| 41249 | SYNPR | NM_144642.3 | 179 | gagtgtggactgcgtcaacaaga | 264198 | - | see also 18180 line |
| 41250 | MAL | NM_002371.2 | 481 | tccatgcggtgttctctttaatc | 264221 | - | see also 18181 line |
| 41251 | STEAP | NM_012449.2 | 1053 | tggtatacacctccaactttat | 264231 | - | see also 18182 line |
| 41252 | STEAP | NM_012449.2 | 960 | cagagcaagctaggaattgtttc | 264224 | - | see also 18182 line |
| 41253 | ATP6V0A2 | NM_012463.1 | 1108 | ctcggatccgcaccaacaaattc | 264250 | - | see also 18183 line |
| 41254 | ATP6V0A2 | NM_012463.1 | 2056 | accggagtggctacacacttata | 264274 | - | see also 18183 line |
| 41255 | C10orf26 | NM_017787.2 | 395 | accgagaagcccacaattactca | 264289 | - | see also 18184 line |
| 41256 | C10orf26 | NM_017787.2 | 183 | ctgtgtgggtaccaacaatcaaa | 264286 | - | see also 18184 line |
| 41257 | C14orf4 | NM_024496.2 | 3231 | ggcgaaatcgcgactatcttagc | 264311 | - | see also 18185 line |
| 41258 | C14orf4 | NM_024496.2 | 3192 | cccctagtcgggtcgaatgtacc | 264308 | - | see also 18185 line |
| 41259 | C14orf52 | NM_145165.1 | 364 | gccgaagatactatcagtattct | 264326 | - | see also 18186 line |
| 41260 | C14orf52 | NM_145165.1 | 363 | agccgaagatactatcagtattc | 264325 | - | see also 18186 line |
| 41261 | C5orf12 | NM_178276.2 | 397 | tgccgtgtatagagtctgttttg | 264331 | - | see also 18187 line |
| 41262 | C5orf12 | NM_178276.2 | 398 | gccgtgtatagagtctgttttgg | 264332 | - | see also 18187 line |
| 41263 | CGI-07 | NM_015938.2 | 1358 | aacgtcagcgtcgtagaaactgg | 264394 | - | see also 6577 line |
| 41264 | CGI-07 | NM_015938.2 | 393 | gagtaaggtacggcttgtagatg | 264364 | - | see also 6577 line |
| 41265 | CYC1 | NM_001916.2 | 163 | ttcccgaggccggaaagtgatgc | 264398 | - | see also 18188 line |
| 41266 | CYC1 | NM_001916.2 | 518 | ttcgactatttcccaaaaccata | 264403 | - | see also 18188 line |
| 41267 | DEGS | NM_003676.2 | 521 | ctgatggcgtcgatgtagatatt | 264425 | - | see also 18189 line |
| 41268 | DEGS | NM_003676.2 | 527 | gcgtcgatgtagatattcctacc | 264427 | - | see also 18189 line |
| 41269 | DISP1 | NM_032890.2 | 2878 | gggccgaggtttgatatcaatga | 264520 | - | see also 9337 line |

Figure 46

| 41270 | DISP1 | NM_032890.2 | 1459 | accccaaagacggctgactatgc | 264478 | - | see also 9337 line | | |
| 41271 | DKFZp434F054 | NM_032259.1 | 1120 | agcgcacggtaaacaaagtctgc | 264568 | - | see also 18191 line | | |
| 41272 | DKFZp434F054 | NM_032259.1 | 1116 | cacaagcgcacggtaaacaaagt | 264567 | - | see also 18191 line | | |
| 41273 | DKFZP564D0478 | NM_032125.1 | 60 | gacggacatgaagcaatatcaag | 264578 | - | see also 9078 line | | |
| 41274 | DKFZP564D0478 | NM_032125.1 | 365 | agcaccgcatgcacaatctctgc | 264582 | - | see also 9078 line | | |
| 41275 | EHZF | NM_015461.1 | 737 | cacgtccaacaagccatataaat | 264600 | - | see also 6424 line | | |
| 41276 | EHZF | NM_015461.1 | 3666 | ttgaaaacgccccaagtatcacc | 264666 | - | see also 6424 line | | |
| 41277 | ERdj5 | NM_018981.1 | 1666 | gccgtctctagcagtatttaaag | 264716 | - | see also 7636 line | | |
| 41278 | ERdj5 | NM_018981.1 | 757 | ctggaactattatcgttatgatt | 264688 | - | see also 7636 line | | |
| 41279 | ETFA | NM_000126.1 | 37 | gcggcctcattgctacgatttca | 264761 | - | see also 99 line | | |
| 41280 | ETFA | NM_000126.1 | 39 | ggcctcattgctacgatttcaga | 264762 | - | see also 99 line | | |
| 41281 | ETFB | NM_001985.1 | 402 | ggccatcgatgatgactgtaacc | 264781 | - | - | electron transfer flavoprotein | glutaricaciduria |
| 41282 | ETFB | NM_001985.1 | 486 | gacgctggaggggacaagttga | 264782 | - | see also 41281 line | | |
| 41283 | FBLP-1 | NM_017556.1 | 950 | cccggcagaacctgttgagaaag | 264784 | - | see also 18196 line | | |
| 41284 | FBLP-1 | NM_017556.1 | 466 | gggtggcatcgtctgtctttatc | 264783 | - | see also 18196 line | | |
| 41285 | FLJ10900 | NM_018264.1 | 2050 | ctgatccccgaatatgaaattgc | 264792 | - | see also 18197 line | | |
| 41286 | FLJ10900 | NM_018264.1 | 2054 | tccccgaatatgaaattgcatgt | 264793 | - | see also 18197 line | | |
| 41287 | FLJ12519 | NM_032168.1 | 918 | cccgcgtttaggagctactattg | 264824 | - | see also 18198 line | | |
| 41288 | FLJ12519 | NM_032168.1 | 856 | ggcggtcgtgaatctgtacttgt | 264820 | - | see also 18198 line | | |
| 41289 | FLJ12519 | NM_032168.1 | 910 | gagtttctcccgcgtttaggagc | 264823 | - | see also 18198 line | | |
| 41290 | FLJ14627 | NM_032814.1 | 1058 | ggggatcgcagactttgttctga | 264870 | - | see also 18199 line | | |
| 41291 | FLJ14627 | NM_032814.1 | 1056 | gtggggatcgcagactttgttct | 264869 | - | see also 18199 line | | |
| 41292 | FLJ20095 | NM_017666.1 | 2219 | ttcttccggcttagatcgtatgg | 264936 | - | see also 18201 line | | |
| 41293 | FLJ20095 | NM_017666.1 | 1870 | agccaaggggacgtatagctaag | 264928 | - | see also 18201 line | | |
| 41294 | FLJ20511 | NM_017853.1 | 324 | aagtagaccaggcgataaaaagt | 264945 | - | see also 18202 line | | |
| 41295 | FLJ20511 | NM_017853.1 | 329 | gaccaggcgataaaaagtactgc | 264946 | - | see also 18202 line | | |
| 41296 | FLJ20511 | NM_017853.1 | 571 | atctccagatcacactaagtttg | 264950 | - | see also 18202 line | | |
| 41297 | FLJ22625 | NM_024715.2 | 1309 | aacggtagtgactgtactctagt | 264964 | - | see also 18203 line | | |
| 41298 | FLJ22625 | NM_024715.2 | 737 | tcctcggctggtggcaagtattg | 264957 | - | see also 18203 line | | |
| 41299 | FLJ23537 | NM_024889.2 | 840 | gaggagtccggccattaaaaaga | 264994 | - | see also 18204 line | | |
| 41300 | FLJ23537 | NM_024889.2 | 838 | aagaggagtccggccattaaaaa | 264993 | - | see also 18204 line | | |
| 41301 | FLJ23537 | NM_024889.2 | 435 | ctcactaactgttgtgcaattgt | 264986 | - | see also 18204 line | | |
| 41302 | FLJ25369 | NM_152670.1 | 615 | gtcttcattgccatatagtcaag | 265002 | - | see also 18205 line | | |

Figure 46

| | | | | | | | |
|---|---|---|---|---|---|---|---|
| 41303 | FLJ25369 | NM_152670.1 | 657 | ggggacccttgatgccatttac | 265004 | - | see also 18205 line |
| 41304 | FLJ25369 | NM_152670.1 | 341 | gggtaccctgctttcaaaagacc | 264999 | - | see also 18205 line |
| 41305 | FLJ31384 | NM_152472.1 | 1135 | gtgtggtaaggctcaccaatcact | 265007 | - | see also 18206 line |
| 41306 | FLJ31384 | NM_152472.1 | 1142 | aaggctcacaatcaacttgattga | 265010 | - | see also 18206 line |
| 41307 | FLJ40417 | NM_173650.1 | 438 | cagcacaccggctgtccaaaagt | 265011 | - | see also 18207 line |
| 41308 | FLJ40417 | NM_173650.1 | 563 | gagtatcaacctgggtaggaaact | 265014 | - | see also 18207 line |
| 41309 | GLRX | NM_002064.1 | 169 | aaccacactaacgagattcaaga | 265026 | - | see also 18208 line |
| 41310 | GLRX | NM_002064.1 | 219 | aacggtgcctcgagtctttattg | 265028 | - | see also 18208 line |
| 41311 | GLRX2 | NM_016066.3 | 665 | aacaatttcgataattgtgtgg | 265039 | - | see also 18209 line |
| 41312 | GLRX2 | NM_016066.3 | 716 | ctgtacaatgcaaaaaagcttt | 265041 | - | see also 18209 line |
| 41313 | HCA127 | NM_018684.1 | 675 | ttcaccgaatgcacctatatgc | 265047 | - | see also 18210 line |
| 41314 | HCA127 | NM_018684.1 | 658 | gtcattgtcaccagcaaaattcacc | 265046 | - | see also 18210 line |
| 41315 | HECA | NM_016217.1 | 1808 | tgcagtacttctccgaatatagc | 265066 | - | see also 18211 line |
| 41316 | HECA | NM_016217.1 | 340 | agccggcgacgagcgaggaaaatgg | 265048 | - | see also 18211 line |
| 41317 | IMAGE:4215339 | NM_052878.1 | 383 | ccccaacgagctgcgaaacatct | 265067 | - | - |
| 41318 | IMAGE:4215339 | NM_052878.1 | 427 | tccagaacgccatcatcgaaagg | 265068 | - | see also 41317 line |
| 41319 | KIAA0528 | NM_014802.1 | 948 | aacggccgtgtactctggataaat | 265092 | - | see also 5996 line |
| 41320 | KIAA0528 | NM_014802.1 | 2595 | cacggcagtcgcaatcactttg | 265151 | - | see also 5996 line |
| 41321 | KIAA0528 | NM_014802.1 | 1315 | cacgttgggggtgttagttagtgc | 265102 | - | see also 5996 line |
| 41322 | KIAA1441 | NM_020832.1 | 2450 | gacgtcgcactgcgaggtttcc | 265184 | - | see also 7985 line |
| 41323 | KIAA1441 | NM_020832.1 | 3160 | gagtttaccctgcaggtattgc | 265191 | - | see also 7985 line |
| 41324 | LOC152485 | NM_178835.2 | 644 | tccggattcagccatcctaaaac | 265268 | - | see also 18215 line |
| 41325 | LOC152485 | NM_178835.2 | 2895 | aagacccccagcggaatcaaatag | 265290 | - | see also 18215 line |
| 41326 | LOC204474 | NM_174924.1 | 826 | aacgtttggagtcataacgattg | 265302 | - | see also 18216 line |
| 41327 | LOC204474 | NM_174924.1 | 1069 | ctccaaaagtccgagtcatatg | 265312 | - | see also 18216 line |
| 41328 | LOC204474 | NM_174924.1 | 1079 | tccgagtcatatggtatcataat | 265314 | - | see also 18216 line |
| 41329 | LOC51061 | NM_015914.2 | 314 | cagtcgagcaaagatgtgataa | 265341 | - | see also 6568 line |
| 41330 | LOC51061 | NM_015914.2 | 311 | ctgcagtcgagcaaaagatgtga | 265340 | - | see also 6568 line |
| 41331 | LOC51066 | NM_015931.1 | 520 | taccgtctgaagacctttagtga | 265387 | - | see also 18218 line |
| 41332 | LOC51066 | NM_015931.1 | 1188 | cacagaagttcactattggtacc | 265395 | - | see also 18218 line |
| 41333 | LOC51257 | NM_016496.3 | 707 | cacgctgggtgccgtgcataaga | 265398 | - | see also 18219 line |

Figure 46

| 41334 | LOC51257 | NM_016496.3 | 709 | cgctgggtgccgtgcataagagc | 265399 | - | see also 18219 line |
|-------|----------|-------------|------|--------------------------|--------|---|---------------------|
| 41335 | LOC90637 | NM_182491.1 | 532 | tacataccgttgctcaaaagagg | 265407 | - | see also 18220 line |
| 41336 | LOC90637 | NM_182491.1 | 366 | cacataagtgtccgtttgcattc | 265404 | - | see also 18220 line |
| 41337 | MB | NM_005368.1 | 312 | ggcatcatgaggcagagattaag | 265414 | - | see also 18221 line |
| 41338 | MB | NM_005368.1 | 282 | ccgccctgggtggcatccttaag | 265411 | - | see also 18221 line |
| 41339 | MGC10986 | NM_030576.2 | 302 | tggtggccgacaagctcattttc | 265415 | - | - |
| 41340 | MGC10986 | NM_030576.2 | 306 | ggccgacaagctcattttccaca | 265416 | - | see also 41339 line |
| 41341 | MGC14353 | NM_032731.2 | 160 | cgcctactttacgggttctaagg | 265417 | - | see also 18222 line |
| 41342 | MGC14353 | NM_032731.2 | 348 | cagcagtgcctacactacttaag | 265422 | - | see also 18222 line |
| 41343 | MGC15482 | NM_032875.1 | 456 | ggccgagtagtggagaatatttc | 265435 | - | see also 18223 line |
| 41344 | MGC15482 | NM_032875.1 | 952 | gagggtgccataagttacaatcc | 265456 | - | see also 18223 line |
| 41345 | MGC33414 | NM_173574.1 | 1533 | accaggggggcactagatcttatg | 265476 | - | see also 18224 line |
| 41346 | MGC33414 | NM_173574.1 | 1077 | tcctgtacgagtgcaacatatgt | 265472 | - | see also 18224 line |
| 41347 | MGC33414 | NM_173574.1 | 1532 | caccaggggggcactagatcttat | 265475 | - | see also 18224 line |
| 41348 | MGC34648 | NM_152660.1 | 676 | gagtcaatcacaactaatggaga | 265483 | - | see also 9910 line |
| 41349 | MGC34648 | NM_152660.1 | 995 | cagagaagtcaggagctataacc | 265487 | - | see also 9910 line |
| 41350 | MGC48332 | NM_178450.2 | 862 | gacactagtgtcatttaggtacc | 265498 | - | see also 10189 line |
| 41351 | MGC48332 | NM_178450.2 | 779 | gaccacctgcactttagtagtcg | 265495 | - | see also 10189 line |
| 41352 | N33 | NM_006765.2 | 446 | caccacctcgaaactattccatg | 265505 | - | see also 4771 line |
| 41353 | N33 | NM_006765.2 | 447 | accacctcgaaactattccatga | 265506 | - | see also 4771 line |
| 41354 | NCF2 | NM_000433.1 | 609 | agctgtttcgaccaaatgagaga | 265541 | - | see also 18228 line |
| 41355 | NCF2 | NM_000433.1 | 605 | ggcaagctgtttcgaccaaatga | 265540 | - | see also 18228 line |
| 41356 | NCF4 | NM_000631.2 | 278 | tcgtcatcgaggtgaagacaaaa | 265553 | - | see also 18229 line |
| 41357 | NCF4 | NM_000631.2 | 749 | tcctcctcagtcggatcaacaaa | 265556 | - | see also 18229 line |
| 41358 | NSE1 | NM_145080.2 | 542 | cgcggtgaagatctgcaatatct | 265573 | - | see also 9775 line |
| 41359 | NSE1 | NM_145080.2 | 728 | gagggagtctggtgtcttgaaat | 265574 | - | see also 9775 line |

# EP 1 752 536 A1

Figure 46

| | | | | | | | |
|---|---|---|---|---|---|---|---|
| 41360 | OSR1 | NM_145260.1 | 953 | acctgcgagaccacagatatt | 265579 | - | see also 18231 line |
| 41361 | OSR1 | NM_145260.1 | 1067 | aggtgaaggagtcaaaacctcc | 265581 | - | see also 18231 line |
| 41362 | OSR2 | NM_053001.1 | 1026 | tgcgcagacactttaccaaatc | 265586 | - | see also 18232 line |
| 41363 | OSR2 | NM_053001.1 | 761 | gcctttaccaccccaccattcc | 265583 | - | see also 18232 line |
| 41364 | PDC | NM_002597.3 | 467 | aagatcaccacaattgttgttca | 265601 | - | see also 18233 line |
| 41365 | PDC | NM_002597.3 | 355 | tagacagtgtatgcaggatatgc | 265595 | - | see also 18233 line |
| 41366 | PGBD3 | NM_170753.1 | 908 | gaactgaccgtttgaaactata | 265645 | - | see also 18234 line |
| 41367 | PGBD3 | NM_170753.1 | 911 | gtgaccgtttgaaactatatt | 265647 | - | see also 18234 line |
| 41368 | PHTF2 | NM_020432.2 | 1199 | gcccctaaatcggtactagtg | 265710 | - | see also 7872 line |
| 41369 | PHTF2 | NM_020432.2 | 2093 | tggtgcatctcgttaacactttt | 265738 | - | see also 7872 line |
| 41370 | QSCN6 | NM_002826.2 | 1324 | caggcagctcggcaaaatgtaga | 265768 | - | see also 18236 line |
| 41371 | QSCN6 | NM_002826.2 | 463 | aacggctcgggagcagtatttcc | 265755 | - | see also 18236 line |
| 41372 | QSCN6 | NM_002826.2 | 954 | ggcagatcgctccaagatctaca | 265764 | - | see also 18236 line |
| 41373 | RNF34 | NM_025126.2 | 687 | ttcgtttcaggggagagcttatgg | 265799 | - | see also 8866 line |
| 41374 | RNF34 | NM_025126.2 | 220 | accggtccattcagatttacacc | 265781 | - | see also 8866 line |
| 41375 | SLAMF8 | NM_020125.1 | 463 | gcccaggccgtggtacaagtgt | 265814 | - | see also 18238 line |
| 41376 | SLAMF8 | NM_020125.1 | 548 | gcccccaacatcagccgaaataac | 265817 | - | see also 18238 line |
| 41377 | SYTL5 | NM_138780.1 | 1496 | atcgatttgacgtaatagcttc | 265863 | - | see also 9631 line |
| 41378 | SYTL5 | NM_138780.1 | 410 | atgttgtccgacagtccatttta | 265825 | - | see also 9631 line |
| 41379 | TLP19 | NM_015913.2 | 595 | agccctgacgggggttatattcc | 265880 | - | see also 6567 line |
| 41380 | TLP19 | NM_015913.2 | 703 | gtcagtgccgagcaagttgttca | 265882 | - | see also 6567 line |
| 41381 | TXNDC | NM_030755.3 | 342 | aggactgagtgacggtttatca | 265888 | - | see also 8932 line |
| 41382 | TXNDC | NM_030755.3 | 344 | gactgagtggacgtttatcata | 265889 | - | see also 8932 line |
| 41383 | TXNL2 | NM_006541.1 | 287 | cagaaaatgacgattagatgg | 265914 | - | see also 4587 line |
| 41384 | TXNL2 | NM_006541.1 | 870 | ttcggcaaggattaaaagcttac | 265936 | - | see also 4587 line |
| 41385 | ZNF330 | NM_014487.2 | 407 | gtcttcagactgtgtcataaagc | 265944 | - | see also 18243 line |
| 41386 | ZNF330 | NM_014487.2 | 916 | ctgggtatgatgcttattggaag | 265952 | - | see also 18243 line |
| 41387 | ZNF363 | NM_015436.1 | 447 | tcccgacgaattgtccaatatg | 265968 | - | see also 18244 line |
| 41388 | ZNF363 | NM_015436.1 | 178 | aaactagatcgctttaaagtgaag | 265959 | - | see also 18244 line |
| 41389 | ZNF451 | NM_015555.1 | 1748 | atgattcagcggtcattcgttta | 266018 | - | see also 6463 line |
| 41390 | ZNF451 | NM_015555.1 | 2968 | ggccgtctagatgaacaactacc | 266069 | - | see also 6463 line |
| 41391 | ZNF507 | NM_014910.1 | 961 | aacagtacggcatgtatcgatgc | 266098 | - | see also 18246 line |
| 41392 | ZNF507 | NM_014910.1 | 957 | tacgaacagtacggcatgtatcg | 266097 | - | see also 18246 line |
| 41393 | ATP5F1 | NM_001688.2 | 395 | ttcactgcctatcagtactagg | 266155 | - | see also 18247 line |
| 41394 | ATP5F1 | NM_001688.2 | 576 | ttcagaagcgccattaccttttt | 266157 | - | see also 18247 line |

1422

Figure 46

| | | | | | | | | | | |
|---|---|---|---|---|---|---|---|---|---|---|
| 41395 | ATP5H | NM_006356.1 | 51 | tgggcgaaaacttgctctaaaaa | 266169 | - | - | | hydrogen-transporting two-sector ATPase | - |
| 41396 | ATP5H | NM_006356.1 | 52 | gggcgaaaacttgctctaaaaac | 266170 | - | see also 41395 line | | | |
| 41397 | ATP5H | NM_006356.1 | 53 | ggcgaaaacttgctctaaaaacc | 266171 | - | see also 41395 line | | | |
| 41398 | ATP5I | NM_007100.1 | 144 | cacgcgctacaattacctaaaac | 266174 | - | see also 18248 line | | | |
| 41399 | ATP5I | NM_007100.1 | 142 | gccacgcgctacaattacctaaa | 266173 | - | see also 18248 line | | | |
| 41400 | ATP5I | NM_007100.1 | 141 | agccacgcgctacaattacctaa | 266172 | - | see also 18248 line | | | |
| 41401 | ATP5J | NM_001685.3 | 747 | gtccatttgcggaggaacattgg | 266177 | - | see also 1106 line | | | |
| 41402 | ATP5J | NM_001685.3 | 799 | ttgatcctatacagaaactcttt | 266179 | - | see also 1106 line | | | |
| 41403 | ATP5J2 | NM_004889.1 | 142 | ggcattttcggagcgtttcaaag | 266180 | - | see also 18249 line | | | |
| 41404 | ATP5J2 | NM_004889.1 | 165 | aggttactaccggtactacaaca | 266183 | - | see also 18249 line | | | |
| 41405 | ATP5S | NM_015684.1 | 1001 | atgggtaactaccctatcgtttt | 266199 | - | see also 18250 line | | | |
| 41406 | ATP5S | NM_015684.1 | 1003 | gggtaactaccctatcgttttgc | 266201 | - | see also 18250 line | | | |
| 41407 | ATP6V0A1 | NM_005177.2 | 389 | agctaacattccgattatggaca | 266209 | - | see also 3641 line | | | |
| 41408 | ATP6V0A1 | NM_005177.2 | 1494 | gtgttcagtggtcgatacattat | 266230 | - | see also 3641 line | | | |
| 41409 | TCIRG1 | NM_006019.2 | 1583 | tggcccagcacacgatgcttacc | 266264 | - | see also 18252 line | | | |
| 41410 | TCIRG1 | NM_006019.2 | 1560 | gtctggctggagtgatgcattcc | 266263 | - | see also 18252 line | | | |
| 41411 | TMEM1 | NM_003274.2 | 3105 | tagtaccgacctgcaactagtac | 266330 | - | see also 18253 line | | | |
| 41412 | TMEM1 | NM_003274.2 | 2766 | tgcgtaccacgtgatcgaatttg | 266320 | - | see also 18253 line | | | |
| 41413 | SLC11A2 | NM_000617.1 | 1705 | ctgtgggcatacggtaagcatct | 266383 | - | see also 415 line | | | |
| 41414 | SLC11A2 | NM_000617.1 | 1710 | ggcatacggtaagcatctctaaa | 266384 | - | see also 415 line | | | |
| 41415 | TTYH1 | NM_020659.1 | 346 | ggcattggcatcggtttctatgg | 266389 | - | see also 18255 line | | | |
| 41416 | TTYH1 | NM_020659.1 | 1367 | tgcagtggcagtcgtctatctga | 266392 | - | see also 18255 line | | | |
| 41417 | SLC31A2 | NM_001860.1 | 163 | ctgcttctggctgtactgtatga | 266394 | - | see also 18256 line | | | |
| 41418 | SLC31A2 | NM_001860.1 | 90 | tacagcggtgcttctgtttgatt | 266393 | - | see also 18256 line | | | |
| 41419 | SLC30A3 | NM_003459.3 | 1238 | ggctctactcccggtttggattc | 266399 | - | see also 18257 line | | | |
| 41420 | SLC30A3 | NM_003459.3 | 1147 | gcccttacgctcacttaccatgt | 266398 | - | see also 18257 line | | | |
| 41421 | SLC39A2 | NM_014579.1 | 771 | tgcggctagtgcatttaggtacc | 266445 | - | see also 18259 line | | | |
| 41422 | SLC39A2 | NM_014579.1 | 309 | gagcagggttcatgcatatgact | 266443 | - | see also 18259 line | | | |
| 41423 | RHAG | NM_000324.1 | 626 | atgaagagtccgcatactactca | 266466 | - | see also 18260 line | | | |
| 41424 | RHAG | NM_000324.1 | 989 | aactgaggatccatgatacatgt | 266475 | - | see also 18260 line | | | |
| 41425 | RHBG | NM_020407.1 | 781 | atcggacggccctcaacacatac | 266484 | - | see also 18261 line | | | |
| 41426 | RHBG | NM_020407.1 | 674 | gggctccgtctaccattcagacc | 266483 | - | see also 18261 line | | | |
| 41427 | RHCG | NM_016321.1 | 503 | ccctcttcgctgtgaatgagttc | 266497 | - | see also 18262 line | | | |

Figure 46

| ID | Gene | Accession | | Sequence | | | Note |
|---|---|---|---|---|---|---|---|
| 41428 | RHCG | NM_016321.1 | 930 | ggcttacggtgccctcatcatcg | 266504 | - | see also 18262 line |
| 41429 | RHCG | NM_016321.1 | 806 | tgcttaacctcggtggcaatatcc | 266502 | - | see also 18262 line |
| 41430 | SLC22A1 | NM_003057.2 | 1349 | cagcctgcctgtcatgatttt | 266526 | - | see also 18263 line |
| 41431 | SLC22A1 | NM_003057.2 | 1376 | cacctgacctgcactgttaaac | 266530 | - | see also 18263 line |
| 41432 | SLC22A13 | NM_004256.1 | 332 | atgagacgcagcctgtgatatg | 266543 | - | see also 18264 line |
| 41433 | SLC22A13 | NM_004256.1 | 66 | tcgcttccagatacagctattga | 266541 | - | see also 18264 line |
| 41434 | SLC22A14 | NM_004803.2 | 1257 | taccgtcagttacaacctattta | 266563 | - | see also 18265 line |
| 41435 | SLC22A14 | NM_004803.2 | 672 | cccgataggctctctcatcttca | 266558 | - | see also 18265 line |
| 41436 | SLC22A14 | NM_004803.2 | 293 | ggctagtagccctcaccttatc | 266554 | - | see also 18265 line |
| 41437 | SLC22A2 | NM_003058.2 | 891 | taccaagttgcctatacagttgg | 266574 | - | see also 18266 line |
| 41438 | SLC22A2 | NM_003058.2 | 1277 | gggccttgcaggtgacaatatct | 266579 | - | see also 18266 line |
| 41439 | SLC22A2 | NM_021977.2 | 1154 | ggggcaacctctatatagactt | 266618 | - | see also 8237 line |
| 41440 | BA108L7.2 | NM_030971.2 | 1016 | ttgaagtggtctactacaacaag | 266644 | - | see also 18268 line |
| 41441 | BA108L7.2 | NM_030971.2 | 1013 | gcgttgaagtggtctactacaac | 266643 | - | see also 18268 line |
| 41442 | DKFZP434 K0427 | NM_032148.2 | 1096 | tgctagttttggcgacctttataa | 266663 | - | see also 9084 line |
| 41443 | DKFZP434 K0427 | NM_032148.2 | 1212 | ctctaaccctatttgattata | 266670 | - | see also 9084 line |
| 41444 | DKFZp547 M236 | NM_018713.1 | 1214 | cacggacaaagtcttaacaaaac | 266700 | - | see also 18270 line |
| 41445 | DKFZp547 M236 | NM_018713.1 | 1234 | aactcaggaggaccaatgttatg | 266702 | - | see also 18270 line |
| 41446 | SFXN1 | NM_022754.4 | 1007 | tgcgacgcgtgtacttcaataag | 266730 | - | see also 18271 line |
| 41447 | SFXN1 | NM_022754.4 | 721 | tcggcgaacgtgcgaaacaagc | 266726 | - | see also 18271 line |
| 41448 | SFXN2 | NM_178858.3 | 721 | tgcggctaactgtcaatatcc | 266738 | - | see also 18272 line |
| 41449 | SFXN2 | NM_178858.3 | 1060 | accgaagctccaagacaactatca | 266742 | - | see also 18272 line |
| 41450 | SFXN4 | NM_178867.2 | 541 | gtctacatgtcccgaagtcttga | 266757 | - | see also 18273 line |
| 41451 | SFXN4 | NM_178867.2 | 295 | atcaagtccgtgatttttacctca | 266747 | - | see also 18273 line |
| 41452 | SFXN4 | NM_178867.2 | 648 | aacgctagcatccagaatagtgc | 266760 | - | see also 18273 line |
| 41453 | SFXN5 | NM_144579.1 | 277 | atgaccctcgcacactcttgt | 266765 | - | see also 9705 line |
| 41454 | SFXN5 | NM_144579.1 | 1127 | gccggacagtggtgtacaacaag | 266775 | - | see also 9705 line |
| 41455 | SFXN5 | NM_144579.1 | 474 | tcctttgggacgccaattgtag | 266770 | - | see also 9705 line |
| 41456 | SLC30A1 | NM_021194.1 | 594 | ctccaacgggctgaaattggacc | 266778 | - | see also 8132 line |
| 41457 | SLC30A1 | NM_021194.1 | 771 | gggttcagtgattgattagtagtaa | 266784 | - | see also 8132 line |
| 41458 | SLC30A2 | NM_032513.2 | 735 | tccatgtgatcgcggacttttatg | 266811 | - | see also 18275 line |
| 41459 | SLC30A2 | NM_032513.2 | 871 | gaccatcctgagagatgtgatcc | 266812 | - | see also 18275 line |
| 41460 | SLC30A5 | NM_022902.2 | 268 | gacgtaccacccagcctgcgattaac | 266813 | - | see also 8527 line |

Figure 46

| 41461 | SLC30A5 | NM_022902.2 | 334 | gtgggactttcgaatcatatga | 266822 | - | see also 8527 line |
|-------|---------|-------------|-----|------------------------|--------|---|--------------------|
| 41462 | SLC30A6 | NM_017964.2 | 1007 | gtgtctactctaactgttcaaat | 266921 | - | see also 18277 line |
| 41463 | SLC30A6 | NM_017964.2 | 557 | gagcatgttgcagatcttagtcg | 266908 | - | see also 18277 line |
| 41464 | SLC30A8 | NM_173851.1 | 382 | ccctctaagcggctgacatttgg | 266929 | - | see also 18278 line |
| 41465 | SLC30A8 | NM_173851.1 | 15 | aagaacgtatcttgtgaatgata | 266927 | - | see also 18278 line |
| 41466 | SLC41A1 | NM_173854.3 | 1484 | tccctgatggccacttcagtatt | 266952 | - | see also 18279 line |
| 41467 | SLC41A1 | NM_173854.3 | 1557 | ctccctggtactgggtatgatca | 266955 | - | see also 18279 line |
| 41468 | SLC41A1 | NM_173854.3 | 1924 | gtcttcacgcctgtgattaatgg | 266959 | - | see also 18279 line |
| 41469 | MRS2L | NM_020662.1 | 622 | ctcgttacatacccttaccttt | 266974 | - | see also 18280 line |
| 41470 | MRS2L | NM_020662.1 | 1082 | gccaccgaaacgtgatgatgagg | 266987 | - | see also 18280 line |
| 41471 | SLC39A11 | NM_139177.2 | 169 | ggcagctctcgtgttcgtattct | 266994 | - | see also 18281 line |
| 41472 | SLC39A11 | NM_139177.2 | 649 | gaggatcgcactgctcatcttgg | 266999 | - | see also 18281 line |
| 41473 | SLC39A12 | NM_152725.1 | 1674 | atgcctataggcagtatgacagc | 267034 | - | see also 18282 line |
| 41474 | SLC39A12 | NM_152725.1 | 1375 | cagcgctggtccttttccatagc | 267030 | - | see also 18282 line |
| 41475 | SLC39A13 | NM_152264.2 | 1111 | tgcgggcatcgtggtaatggtgc | 267051 | - | see also 18283 line |
| 41476 | SLC39A13 | NM_152264.2 | 726 | tgctggccaacaccatcgataac | 267047 | - | see also 18283 line |
| 41477 | SLC39A3 | NM_144564.3 | 293 | aggcccatcgctcgaaaaagatc | 267054 | - | see also 18284 line |
| 41478 | SLC39A3 | NM_144564.3 | 294 | ggcccatcgctcgaaaaagatcc | 267055 | - | see also 18284 line |
| 41479 | SLC39A5 | NM_173596.1 | 711 | tgcaccccaggggatctactat | 267059 | - | see also 18286 line |
| 41480 | SLC39A5 | NM_173596.1 | 808 | ctcctgggacctcgtctactacg | 267060 | - | see also 18286 line |
| 41481 | SLC39A6 | NM_012319.2 | 2066 | ggctggcatgaccgttaagcagg | 267114 | - | see also 18287 line |
| 41482 | SLC39A6 | NM_012319.2 | 2356 | aacataaaatcgtgtttcgtata | 267124 | - | see also 18287 line |
| 41483 | SLC39A7 | NM_006979.1 | 561 | cacgaccatggacattcacatga | 267127 | - | see also 4873 line |
| 41484 | SLC39A7 | NM_006979.1 | 1075 | atggacacgctcacagtcataca | 267132 | - | see also 4873 line |
| 41485 | SLC39A8 | NM_022154.4 | 1602 | ctgctatgttgggctagcttttg | 267162 | - | see also 18289 line |
| 41486 | SLC39A8 | NM_022154.4 | 1560 | cactcgacaagccttgctattca | 267160 | - | see also 18289 line |
| 41487 | SLC39A9 | NM_018375.2 | 748 | tggcttagagcggaatcgaatca | 267183 | - | see also 18290 line |
| 41488 | SLC39A9 | NM_018375.2 | 199 | atgttacgtggccggaatcattc | 267168 | - | see also 18290 line |
| 41489 | SLC22A6 | NM_004790.3 | 789 | gccggaaggtactcatcttgaac | 267189 | - | see also 3325 line |
| 41490 | SLC22A6 | NM_004790.3 | 620 | tgcaccgatggctggatctatga | 267188 | - | see also 3325 line |
| 41491 | SLC22A6 | NM_004790.3 | 1396 | tggagtcagcatctacctaatcc | 267198 | - | see also 3325 line |
| 41492 | SLC22A8 | NM_004254.2 | 1465 | aacaggtatgggcgtaagtaacc | 267215 | - | see also 18292 line |
| 41493 | SLC22A8 | NM_004254.2 | 1631 | cccttgccagagactatcgaaga | 267217 | - | see also 18292 line |
| 41494 | SLCO1A2 | NM_005075.2 | 688 | tacgtcctagtaggcaatattgt | 267229 | - | see also 18293 line |
| 41495 | SLCO1A2 | NM_005075.2 | 1844 | cccttggtgtgggattacataca | 267263 | - | see also 18293 line |
| 41496 | SLCO1B3 | NM_019844.1 | 1917 | tacttgggcttatctatagcttt | 267308 | - | see also 18294 line |
| 41497 | SLCO1B3 | NM_019844.1 | 1196 | tacggttgcaactggaatgtttt | 267294 | - | see also 18294 line |

Figure 46

| | | | | | | | |
|---|---|---|---|---|---|---|---|
| 41498 | DKFZp761H039 | NM_018711.1 | 1157 | ttctcttactacgggttagttct | 267332 | - | see also 18295 line |
| 41499 | DKFZp761H039 | NM_018711.1 | 1159 | ctcttactacgggttagttctac | 267333 | - | see also 18295 line |
| 41500 | DKFZp761H039 | NM_018711.1 | 580 | ctccacgtctgggaaatatct | 267323 | - | see also 18295 line |
| 41501 | PLP2 | NM_002668.1 | 225 | ctccctgtcggttgattgagatga | 267342 | - | see also 18296 line |
| 41502 | PLP2 | NM_002668.1 | 475 | ccgttcggcagccaagacacatac | 267347 | - | see also 18296 line |
| 41503 | PLP2 | NM_002668.1 | 393 | aggaaaccactccaaaatcgtcg | 267346 | - | see also 18296 line |
| 41504 | SLC22A5 | NM_003060.2 | 1304 | ttcgcttgatactcctaacttgc | 267367 | - | see also 18297 line |
| 41505 | SLC22A5 | NM_003060.2 | 1155 | atctttgacccgagtgagttaca | 267358 | - | see also 18297 line |
| 41506 | AP1B1 | NM_001127.2 | 1844 | ctcatcgagcccacactgttaga | 267387 | - | see also 715 line |
| 41507 | AP1B1 | NM_001127.2 | 1855 | cacactgttagacgagcttatct | 267388 | - | see also 715 line |
| 41508 | AP1B1 | NM_001127.2 | 2578 | cacggtgggctcggtcatgaaga | 267392 | - | see also 715 line |
| 41509 | AP1G1 | NM_001128.4 | 2698 | tgcgaatgcggatcaagcttaca | 267466 | - | see also 719 line |
| 41510 | AP1G2 | NM_003917.2 | 2041 | cacactcttccggaaatacgacc | 267482 | - | see also 18300 line |
| 41511 | AP1G2 | NM_003917.2 | 1255 | acgggttctagctgtcaacattc | 267474 | - | see also 18300 line |
| 41512 | AP1M1 | NM_032493.2 | 583 | ggcgcgtcgaaggcatcaagtat | 267492 | - | see also 9221 line |
| 41513 | AP1M1 | NM_032493.2 | 270 | gtccgtttcatgtggatcaaaca | 267489 | - | see also 9221 line |
| 41514 | AP1M2 | NM_005498.3 | 1099 | aacgtcgtgatttggagtattaa | 267524 | - | see also 18302 line |
| 41515 | AP1M2 | NM_005498.3 | 560 | ggcgcgtcgagggtatcaagtat | 267513 | - | see also 18302 line |
| 41516 | AP1S1 | NM_001283.2 | 34 | tgccgttcatgctattattcagc | 267533 | - | see also 18303 line |
| 41517 | AP1S1 | NM_001283.2 | 148 | tggctcgaaagcccaagatgtgc | 267534 | - | see also 18303 line |
| 41518 | AP1S3 | NM_178814.2 | 190 | acccgggaaattgttcagattat | 267541 | - | see also 18304 line |
| 41519 | AP1S3 | NM_178814.2 | 228 | caggacaagcagttttgttgact | 267543 | - | see also 18304 line |
| 41520 | AP2A1 | NM_014203.2 | 262 | ctccgacatccggaactgtaaga | 267546 | - | see also 5535 line |
| 41521 | AP2A1 | NM_014203.2 | 721 | gttgcctccttcgactgtacaagg | 267552 | - | see also 5535 line |
| 41522 | AP3B1 | NM_003664.3 | 2055 | ccgaccatcagttcgaaatgt | 267638 | - | see also 2480 line |
| 41523 | AP3B1 | NM_003664.3 | 1144 | tgcgtttacttctgagcaatagg | 267597 | - | see also 2480 line |
| 41524 | AP3B2 | NM_004644.3 | 418 | aacgtggcctgtaagaaacataga | 267685 | - | see also 18307 line |
| 41525 | AP3B2 | NM_004644.3 | 1401 | tactgtcctacgggaattccaga | 267692 | - | see also 18307 line |
| 41526 | AP3B2 | NM_004644.3 | 812 | agcgcatcgacctgattcacaa | 267688 | - | see also 18307 line |
| 41527 | AP3D1 | NM_003938.4 | 3383 | cagttgacgcctttgctaagttgc | 267741 | - | see also 2695 line |
| 41528 | AP3D1 | NM_003938.4 | 3215 | ctccaacgaagcccagtatgtgt | 267740 | - | see also 2695 line |
| 41529 | AP3M1 | NM_012095.2 | 240 | cagtatctaccgggataagctct | 267752 | - | see also 18309 line |
| 41530 | AP3M1 | NM_012095.2 | 434 | cactgctaccgaatctaacatt | 267762 | - | see also 18309 line |
| 41531 | AP3M2 | NM_006803.2 | 464 | taccgagtcgaacattcttaaag | 267810 | - | see also 4783 line |

Figure 46

| 41532 | AP3M2 | NM_006803.2 | 592 | ggcgacggactggggtgaaatat | 267814 | - | see also 4783 line |
|---|---|---|---|---|---|---|---|
| 41533 | AP3M2 | NM_006803.2 | 461 | ggctaccgagtcgaacattctta | 267809 | - | see also 4783 line |
| 41534 | AP3S1 | NM_001284.1 | 617 | aacattggtgacatcagtataaa | 267843 | - | see also 18311 line |
| 41535 | AP3S1 | NM_001284.1 | 564 | gtgctgtatcagctgtaaagaat | 267841 | - | see also 18311 line |
| 41536 | AP3S2 | NM_005829.3 | 143 | agcgggatgacaacatctgtaac | 267848 | - | see also 18312 line |
| 41537 | AP3S2 | NM_005829.3 | 224 | atgctaccctctactttgtattt | 267850 | - | see also 18312 line |
| 41538 | APBA1 | NM_001163.2 | 1686 | aagccgtaagcaggatcaagatg | 267866 | - | see also 742 line |
| 41539 | APBA1 | NM_001163.2 | 1687 | agccgtaagcaggatcaagatgg | 267867 | - | see also 742 line |
| 41540 | APBA1 | NM_001163.2 | 2573 | gtggggcaccggatcattgaaat | 267879 | - | see also 742 line |
| 41541 | ARCN1 | NM_001655.3 | 618 | aaggctgagatgcgtcgtaaagc | 267896 | - | see also 18314 line |
| 41542 | ARCN1 | NM_001655.3 | 1015 | gagacggaggattacagaatatg | 267903 | - | see also 18314 line |
| 41543 | BCAP29 | NM_018844.1 | 694 | ttcgaaagaatatgatcaactcc | 267926 | - | see also 7600 line |
| 41544 | BCAP31 | NM_005745.5 | 326 | atcgatgccgtgcgcgaaattcg | 267931 | - | see also 3995 line |
| 41545 | BCAP31 | NM_005745.5 | 338 | cgcgaaattcggaagtatgatga | 267934 | - | see also 3995 line |
| 41546 | BET1 | NM_005868.2 | 359 | aactatgggcaaactgaagattt | 267942 | - | - \| - \| - |
| 41547 | BET1 | NM_005868.2 | 363 | atgggcaaactgaagattttatc | 267943 | - | see also 41546 line |
| 41548 | BET1 | NM_005868.2 | 383 | atccagagggagccaaacaaagc | 267944 | - | see also 41546 line |
| 41549 | C14orf163 | NM_016106.2 | 974 | atgatttaactccggttgataaa | 267970 | - | see also 18317 line |
| 41550 | C14orf163 | NM_016106.2 | 253 | cactctgatcgagatcctattcc | 267952 | - | see also 18317 line |
| 41551 | C20orf161 | NM_033421.2 | 494 | gaccagcgctaacgttgtcaagg | 268005 | - | see also 18318 line |
| 41552 | CHS1 | NM_000081.1 | 6858 | cccacgccgacctgattacctaa | 268194 | - | see also 51 line |
| 41553 | CHS1 | NM_000081.1 | 6570 | tagtatcgacaggttacaaaata | 268187 | - | see also 51 line |
| 41554 | COG1 | NM_018714.1 | 2689 | cccggagcagtacgttcaactcc | 268402 | - | see also 7575 line |
| 41555 | COG1 | NM_018714.1 | 2625 | cgcctggtgcagcgaacttctgt | 268399 | - | see also 7575 line |
| 41556 | COG2 | NM_007357.1 | 755 | gacgtctgacgtcgatataatac | 268429 | - | see also 18321 line |
| 41557 | COG2 | NM_007357.1 | 791 | gacttacgccacgattgacaaga | 268431 | - | see also 18321 line |
| 41558 | COG3 | NM_031431.2 | 2164 | acgtagaccgtcatctgaaatcg | 268537 | - | see also 9018 line |
| 41559 | COG3 | NM_031431.2 | 1559 | ggcatatcccgataagttagtca | 268516 | - | see also 9018 line |
| 41560 | COG4 | NM_015386.1 | 232 | ctccaccgaatgggtcctaatct | 268557 | - | see also 6403 line |
| 41561 | COG4 | NM_015386.1 | 2052 | tgccgtcgagttggagaaagtgg | 268581 | - | see also 6403 line |
| 41562 | COG5 | NM_006348.2 | 1521 | atctcgactcttcgatcctatca | 268633 | - | see also 18324 line |
| 41563 | COG5 | NM_006348.2 | 1173 | tggacaaccggaaattttctaca | 268616 | - | see also 18324 line |
| 41564 | COG6 | NM_020751.1 | 155 | gtcgcgcaagctgcataagatcc | 268680 | - | see also 7941 line |
| 41565 | COG6 | NM_020751.1 | 1115 | gcctctaaaggttcgaattgagc | 268720 | - | see also 7941 line |
| 41566 | COG7 | NM_153603.1 | 582 | cacgttgagcgccgatattgagg | 268767 | - | see also 18325 line |
| 41567 | COG7 | NM_153603.1 | 580 | agcacgttgagcgccgatattga | 268766 | - | see also 18325 line |
| 41568 | COG8 | NM_032382.2 | 1659 | ctctccaagtacggtaacctagg | 268817 | - | see also 18326 line |

Figure 46

| | | | | | | | |
|---|---|---|---|---|---|---|---|
| 41569 | COG8 | NM_032382.2 | 549 | acctgtccggaacagttatta | - | 268799 | see also 18326 line |
| 41570 | COPB | NM_016451.3 | 3071 | accggccataagaattcgtgc | - | 268904 | see also 6862 line |
| 41571 | COPB | NM_016451.3 | 1993 | tgcattgcgctatgtagctttg | - | 268872 | see also 6862 line |
| 41572 | COPB | NM_016451.3 | 307 | ggcggctgagaacgtatgctaca | - | 268818 | see also 6862 line |
| 41573 | COPE | NM_007263.3 | 92 | acgagctgttcgacgtaaagaac | - | 268907 | see also 18328 line |
| 41574 | COPE | NM_007263.3 | 104 | acgtaaagaacggccttctacatc | - | 268908 | see also 18328 line |
| 41575 | COPG | NM_016128.3 | 1273 | tcgcaaacacgccgtccttatga | - | 268943 | see also 18329 line |
| 41576 | COPG | NM_016128.3 | 698 | ctgtaccatgtgcgtaagaatga | - | 268929 | see also 18329 line |
| 41577 | COPG2 | NM_012133.1 | 1775 | aaccgtttgacatgaaatcaatt | - | 268984 | see also 18330 line |
| 41578 | COPG2 | NM_012133.1 | 1776 | accgtttgacatgaaatcaattc | - | 268985 | see also 18330 line |
| 41579 | COPZ1 | NM_016057.1 | 53 | ttggaacttccctgtatactgt | - | 268989 | see also 18331 line |
| 41580 | COPZ1 | NM_016057.1 | 192 | cccatcggactgacagtgaaatt | - | 268993 | see also 18331 line |
| 41581 | COPZ2 | NM_016429.1 | 301 | gggtatgaccatgtctacaaga | - | 269002 | see also 18332 line |
| 41582 | COPZ2 | NM_016429.1 | 269 | agccggactgagagtgagattgc | - | 269001 | see also 18332 line |
| 41583 | EIF4ENIF1 | NM_019843.2 | 332 | ctgtgcgcaggatagtagatcc | - | 269012 | see also 7733 line |
| 41584 | EPIM | NM_001980.2 | 192 | accggcgttaggaagaagaatgatga | - | 269065 | see also 18334 line |
| 41585 | EPIM | NM_001980.2 | 699 | tccgacattatcagattcaca | - | 269082 | see also 18334 line |
| 41586 | EXO70 | NM_015219.1 | 1382 | agcggctgctaagcacctatatc | - | 269109 | see also 18335 line |
| 41587 | EXO70 | NM_015219.1 | 1623 | ctggttaaaggtgactgattaca | - | 269114 | see also 18335 line |
| 41588 | GGA1 | NM_013365.2 | 41 | ctctgagcgcggaatcaataga | - | 269120 | see also 18336 line |
| 41589 | GGA1 | NM_013365.2 | 498 | tccacggccgaagaatgtgatct | - | 269129 | see also 18336 line |
| 41590 | GOSR2 | NM_004287.2 | 456 | atggatgacctcattttagatgg | - | 269140 | see also 18337 line |
| 41591 | GOSR2 | NM_004287.2 | 386 | ctctgacacaccataccaatgg | - | 269137 | see also 18337 line |
| 41592 | IPO11 | NM_016338.3 | 819 | tgctgcgtaagttaactgttaat | - | 269158 | see also 6816 line |
| 41593 | IPO11 | NM_016338.3 | 1664 | ctcatcggtcagtggatttctgt | - | 269184 | see also 6816 line |
| 41594 | IPO11 | NM_016338.3 | 2667 | ttgatcgaatggacaacattacc | - | 269220 | see also 6816 line |
| 41595 | IPO13 | NM_014652.1 | 2495 | ctcgcttatctcaccctatatcc | - | 269255 | see also 5824 line |
| 41596 | IPO13 | NM_014652.1 | 2773 | cggtgtgcgctatctttgagaag | - | 269258 | see also 5824 line |
| 41597 | IPO9 | NM_018085.3 | 2712 | cccagaacgctggacaaacattc | - | 269312 | see also 18340 line |
| 41598 | IPO9 | NM_018085.3 | 1272 | agccactcgacatttacaagaag | - | 269285 | see also 18340 line |
| 41599 | KPNA4 | NM_002268.3 | 1598 | tactcgatggactaagtaatata | - | 269353 | see also 1456 line |
| 41600 | KPNA4 | NM_002268.3 | 1600 | ctcgatggactaagtaatatatt | - | 269354 | see also 1456 line |
| 41601 | KPNA5 | NM_002269.2 | 1079 | tgcattaagggcagttggtaata | - | 269388 | see also 18342 line |
| 41602 | KPNA5 | NM_002269.2 | 574 | ctgcatgggcattaacaaatata | - | 269369 | see also 18342 line |
| 41603 | KPNA6 | NM_012316.3 | 677 | gccgagattacgtcttgaactgt | - | 269416 | see also 5272 line |
| 41604 | KPNA6 | NM_012316.3 | 669 | ctctgtttgccgagattacgtct | - | 269414 | see also 5272 line |
| 41605 | MTX1 | NM_002455.2 | 630 | tgctgacctatgccagatttact | - | 269434 | see also 18344 line |

Figure 46

| | | | | | | | |
|---|---|---|---|---|---|---|---|
| 41606 | MTX1 | NM_002455.2 | 629 | gtgctgacctatgccagatttac | 269433 | - | see also 18344 line |
| 41607 | MTX2 | NM_006554.1 | 802 | aacacaaccgtatttcttcaata | 269464 | - | see also 4592 line |
| 41608 | MTX2 | NM_006554.1 | 488 | gccaagggccattcttcttagtga | 269456 | - | see also 4592 line |
| 41609 | NAPA | NM_003827.1 | 844 | caccgagtcggtgaaggaatacg | 269483 | - | see also 2608 line |
| 41610 | NAPA | NM_003827.1 | 780 | cccgggaatgcaagttgatgaaa | 269482 | - | see also 2608 line |
| 41611 | NAPG | NM_003826.1 | 588 | aggcggcactctcattcagaaa | 269501 | - | see also 18346 line |
| 41612 | NAPG | NM_003826.1 | 568 | acgaggacgtaggtttgatgagg | 269500 | - | see also 18346 line |
| 41613 | Nup37 | NM_024057.2 | 198 | gtcattggcacgtgacgtttca | 269514 | - | see also 8580 line |
| 41614 | NUP98 | NM_005387.3 | 2806 | agcatccgtcaaaactagtaca | 269620 | - | see also 3753 line |
| 41615 | NUP98 | NM_005387.3 | 2805 | gagcatccgtctaaaactagtac | 269619 | - | see also 3753 line |
| 41616 | NUP98 | NM_005387.3 | 2803 | aaggagcatccgtctaaaactagt | 269618 | - | see also 3753 line |
| 41617 | NUTF2 | NM_005796.1 | 432 | ttggtttgcaccaatgacatgt | 269692 | - | see also 18349 line |
| 41618 | NXT1 | NM_013248.2 | 582 | gccttccagcgagttccaaatca | 269694 | - | protein transporter |
| 41619 | NXT2 | NM_018698.3 | 518 | aagcaactcagtcccaaactaca | 269706 | - | see also 18350 line |
| 41620 | NXT2 | NM_018698.3 | 429 | agggctggatgccctaaataatt | 269703 | - | see also 18350 line |
| 41621 | PEX13 | NM_002618.1 | 622 | tacagcggatgttaggtttaaga | 269730 | - | see also 1696 line |
| 41622 | PEX13 | NM_002618.1 | 354 | aaccgcctccgtgtagatgatct | 269720 | - | see also 1696 line |
| 41623 | PIGR | NM_002644.2 | 2179 | aagaacgtcgaccgagtttcaat | 269784 | - | see also 18352 line |
| 41624 | PIGR | NM_002644.2 | 898 | cccgagccgagctgttttatga | 269773 | - | see also 18352 line |
| 41625 | SCAMP1 | NM_004866.3 | 170 | gccgacccgatctcaacaatcc | 269792 | - | see also 18353 line |
| 41626 | SCAMP1 | NM_004866.3 | 169 | tgccgacccggatctcaacaatc | 269791 | - | see also 18353 line |
| 41627 | SCAMP2 | NM_005697.3 | 825 | ctgtctcactgataaatcattc | 269849 | - | see also 18354 line |
| 41628 | SCAMP2 | NM_005697.3 | 754 | gtcaaataggatctacatcatc | 269845 | - | see also 18354 line |
| 41629 | SCAMP3 | NM_005698.2 | 482 | cccacagaacctaagaaactatgg | 269851 | - | see also 18355 line |
| 41630 | SCAMP3 | NM_005698.2 | 929 | cggagtgacagttcattcaatt | 269853 | - | see also 18355 line |
| 41631 | SEC10L1 | NM_006544.2 | 2247 | ctggtagttgcccagataatt | 269892 | - | see also 18356 line |
| 41632 | SEC10L1 | NM_006544.2 | 601 | accagttagagggggtaaaacaca | 269864 | - | see also 18356 line |
| 41633 | SEC13L1 | NM_030673.2 | 906 | tacgttggtcccctaaattgttgc | 269913 | - | see also 18357 line |
| 41634 | SEC13L1 | NM_030673.2 | 863 | atggtcgttgttcatttggacc | 269911 | - | see also 18357 line |
| 41635 | SEC15L1 | NM_019053.2 | 538 | tagtcaataccggttttgtcagc | 269933 | - | see also 7677 line |
| 41636 | SEC23A | NM_006364.2 | 1066 | cactggtgctcgtatcatgatgt | 270001 | - | see also 4461 line |
| 41637 | SEC23A | NM_006364.2 | 1262 | atctatgcgtgtgcattagatca | 270010 | - | see also 4461 line |
| 41638 | SEC23B | NM_006363.3 | 2210 | tcccgatgccacgttacatcaac | 270097 | - | see also 4458 line |
| 41639 | SEC23B | NM_006363.3 | 2211 | cccgatgccacgttacatcaaca | 270098 | - | see also 4458 line |
| 41640 | SEC24B | NM_006323.1 | 1968 | tgccgatcctgtcgaacgtatat | 270169 | - | see also 18361 line |
| 41641 | SEC24B | NM_006323.1 | 1600 | gccccagtaaccggtatattct | 270150 | - | see also 18361 line |
| 41642 | SEC24B | NM_006323.1 | 726 | gccgcgtatcctagtgtttcata | 270121 | - | see also 18361 line |

Figure 46

| 41643 | SEC24D | NM_014822.1 | 350 | caccgccactaggggaatgttgc | 270239 | - | see also 18362 line |
|---|---|---|---|---|---|---|---|
| 41644 | SEC24D | NM_014822.1 | 2445 | gtgctttacacgacaatcagtgg | 270277 | - | see also 18362 line |
| 41645 | SEC3 | NM_018261.2 | 315 | atccgataagggagatttctaca | 270304 | - | see also 18363 line |
| 41646 | SEC3 | NM_018261.2 | 502 | cagcgatatctccggaagaaaat | 270306 | - | see also 18363 line |
| 41647 | SEC5 | NM_018303.4 | 1782 | cacgccatccagactgtaagact | 270414 | - | see also 7374 line |
| 41648 | SEC5 | NM_018303.4 | 752 | aggcggtctcagtacattcttcg | 270389 | - | see also 7374 line |
| 41649 | SEC6 | NM_007277.3 | 1786 | aacgctgtagacattatctgtgt | 270466 | - | see also 18364 line |
| 41650 | SEC6 | NM_007277.3 | 759 | gcgcatacttgaccggaaaaagc | 270446 | - | see also 18364 line |
| 41651 | SEC6 | NM_007277.3 | 1100 | gcctcttgacgtgggtcttaaac | 270451 | - | see also 18364 line |
| 41652 | SEC63 | NM_007214.2 | 932 | gaccaacggataatattctaata | 270497 | - | see also 5021 line |
| 41653 | SEC63 | NM_007214.2 | 251 | atgccgagcaaattcgattaaag | 270473 | - | see also 5021 line |
| 41654 | SNAG1 | NM_052870.1 | 1804 | ctcctcgagtatcttgcattagg | 270514 | - | see also 18366 line |
| 41655 | SNAG1 | NM_052870.1 | 1840 | ttctcgaaggttcaaagagtacc | 270517 | - | see also 18366 line |
| 41656 | SNAG1 | NM_052870.1 | 1640 | agcaggtgatatggagtgtattg | 270513 | - | see also 18366 line |
| 41657 | SNAP23 | NM_003825.2 | 668 | accaacagagatcgtattgatat | 270529 | - | see also 18367 line |
| 41658 | SNAP23 | NM_003825.2 | 667 | caccaacagagatcgtattgata | 270528 | - | see also 18367 line |
| 41659 | SNAP29 | NM_004782.2 | 110 | gtcagcttaccctaaaagctaca | 270535 | - | see also 18368 line |
| 41660 | SNX1 | NM_003099.2 | 1383 | gtctcgggtgactcaatatgaaa | 270561 | - | see also 18369 line |
| 41661 | SNX1 | NM_003099.2 | 1084 | gaggacaacacggcattgtcacg | 270556 | - | see also 18369 line |
| 41662 | SNX10 | NM_013322.2 | 259 | ttcgagatcctaggattcagaag | 270566 | - | see also 18370 line |
| 41663 | SNX10 | NM_013322.2 | 296 | ttcttacattgactatgagatat | 270569 | - | see also 18370 line |
| 41664 | SNX11 | NM_013323.2 | 714 | tgccattcttcgatatgctatgt | 270577 | - | see also 18371 line |
| 41665 | SNX11 | NM_013323.2 | 719 | ttcttcgatatgctatgtcaaac | 270578 | - | see also 18371 line |
| 41666 | SNX12 | NM_013346.2 | 284 | ggcgctacagtgactttgagtgg | 270586 | - | see also 18372 line |
| 41667 | SNX12 | NM_013346.2 | 166 | gagatcgacatctttaatcctca | 270584 | - | see also 18372 line |
| 41668 | SNX15 | NM_013306.2 | 658 | ggctacaccgagtacaaagtaac | 270592 | - | see also 18373 line |
| 41669 | SNX15 | NM_013306.2 | 678 | aaccgcgcagttcatctcaaaga | 270594 | - | see also 18373 line |
| 41670 | SNX16 | NM_022133.2 | 986 | atccaccgggtccatttgatagc | 270616 | - | see also 8298 line |
| 41671 | SNX19 | NM_014758.1 | 2270 | gtcgctatcgggagttcttgaat | 270658 | - | see also 18375 line |
| 41672 | SNX19 | NM_014758.1 | 2411 | cccgtaagagcctcctagaatca | 270665 | - | see also 18375 line |
| 41673 | SNX19 | NM_014758.1 | 2272 | cgctatcgggagttcttgaatct | 270660 | - | see also 18375 line |
| 41674 | SNX2 | NM_003100.2 | 520 | tgcctatatggcatatagagtaa | 270687 | - | see also 2081 line |
| 41675 | SNX2 | NM_003100.2 | 408 | ctcccgtgatctttgatagatcc | 270686 | - | see also 2081 line |
| 41676 | SNX22 | NM_024798.1 | 350 | gcccaaggagttactggaattcc | 270711 | - | - protein transporter - |
| 41677 | SNX24 | NM_014035.1 | 105 | agcggggatacacggtgtttaag | 270713 | - | see also 5466 line |
| 41678 | SNX24 | NM_014035.1 | 73 | cccgtcctttcgctatgaagaga | 270712 | - | see also 5466 line |
| 41679 | SNX24 | NM_014035.1 | 115 | cacggtgtttaagatagaagtgc | 270715 | - | see also 5466 line |

Figure 46

| 41680 | SNX26 | NM_052948.2 | 410 | cacctccaccggtgcatatttga | 270730 | - | see also 18378 line |
| 41681 | SNX26 | NM_052948.2 | 374 | ctccggagttacgatgactttcg | 270728 | - | see also 18378 line |
| 41682 | SNX27 | NM_030918.4 | 455 | gacgactcgttgggacaatcatt | 270745 | - | see also 18379 line |
| 41683 | SNX27 | NM_030918.4 | 716 | gcccgacgtcggggattggaaga | 270756 | - | see also 18379 line |
| 41684 | SNX3 | NM_003795.3 | 469 | gccgcttcaccacttacgaaatc | 270786 | - | - | - | - |
| 41685 | SNX3 | NM_003795.3 | 630 | ttgcgtcagcttccttttagagg | 270791 | - | see also 41684 line |
| 41686 | SNX3 | NM_003795.3 | 794 | aagctatactccatctaaaataa | 270792 | - | see also 41684 line |
| 41687 | SNX4 | NM_003794.2 | 498 | gaggcgacggattggtttagaaa | 270810 | - | see also 18380 line |
| 41688 | SNX4 | NM_003794.2 | 496 | gagaggcgacggattggtttaga | 270809 | - | see also 18380 line |
| 41689 | SNX5 | NM_014426.2 | 171 | ccctcgcttcagattgacatacc | 270842 | - | see also 5673 line |
| 41690 | SNX5 | NM_014426.2 | 169 | atccctcgcttcagattgacata | 270840 | - | see also 5673 line |
| 41691 | SNX5 | NM_014426.2 | 170 | tccctcgcttcagattgacatac | 270841 | - | see also 5673 line |
| 41692 | SNX8 | NM_013321.1 | 717 | tccggaacatctacaatagcttt | 270861 | - | see also 18382 line |
| 41693 | SNX8 | NM_013321.1 | 782 | atcgacaatgcggcagatcttct | 270864 | - | see also 18382 line |
| 41694 | STX10 | NM_003765.1 | 324 | cccaacagccgtagcattttgg | 270873 | - | see also 18383 line |
| 41695 | STX10 | NM_003765.1 | 719 | tcctcgttctcatcttactattc | 270876 | - | see also 18383 line |
| 41696 | STX10 | NM_003765.1 | 323 | gcccaacagccgtagcattttg | 270872 | - | see also 18383 line |
| 41697 | STX11 | NM_003764.2 | 234 | cccagacggggacgatgagtttg | 270878 | - | see also 18384 line |
| 41698 | STX12 | NM_177424.1 | 766 | agcgagctgcttactatcagaaa | 270892 | - | see also 10162 line |
| 41699 | STX12 | NM_177424.1 | 791 | atctcgcaagaagatgtgtatcc | 270894 | - | see also 10162 line |
| 41700 | STX16 | NM_003763.2 | 912 | gacttaggggcgatgattgtaga | 270908 | - | see also 18386 line |
| 41701 | STX16 | NM_003763.2 | 951 | gacagaattgactataacgttga | 270912 | - | see also 18386 line |
| 41702 | STX18 | NM_016930.2 | 538 | cagagagccatccgagttaaaag | 270922 | - | see also 18387 line |
| 41703 | STX18 | NM_016930.2 | 886 | gagatattcacggaaaaggtttt | 270930 | - | see also 18387 line |
| 41704 | STX1A | NM_004603.1 | 228 | aagaactcatgtccgacataaag | 270935 | - | see also 18388 line |
| 41705 | STX1A | NM_004603.1 | 113 | gaggagattcgaggcttcattga | 270934 | - | see also 18388 line |
| 41706 | STX3A | NM_004177.3 | 635 | atccagcggcagctcgaaattac | 270946 | - | see also 2804 line |
| 41707 | STX3A | NM_004177.3 | 452 | gccaacaacgtccggaacaaact | 270944 | - | see also 2804 line |
| 41708 | STX4A | NM_004604.3 | 360 | cggacaattcggcagactattgt | 270961 | - | see also 18390 line |
| 41709 | STX4A | NM_004604.3 | 348 | ttccacaaggtccggacaattcg | 270959 | - | see also 18390 line |
| 41710 | STX5A | NM_003164.2 | 397 | tccgacaacgcagtgaattcacc | 270975 | - | see also 2138 line |
| 41711 | STX7 | NM_003569.1 | 749 | ctgtcaagggcagcagattatca | 270992 | - | see also 18392 line |
| 41712 | STX7 | NM_003569.1 | 319 | cagtgaacagcgtcaaaggaaaa | 270988 | - | see also 18392 line |
| 41713 | STXBP1 | NM_003165.1 | 742 | aagctcgatgcctataaagctga | 271005 | - | see also 18393 line |
| 41714 | STXBP1 | NM_003165.1 | 1545 | ctcacggtggactccgattatca | 271020 | - | see also 18393 line |
| 41715 | STXBP3 | NM_007269.1 | 1464 | ctctcggtggacacctttatca | 271074 | - | see also 5067 line |
| 41716 | STXBP3 | NM_007269.1 | 339 | aagtaaatcggagaacaagtata | 271044 | - | see also 5067 line |

Figure 46

| | | | | | | | |
|---|---|---|---|---|---|---|---|
| 41717 | SYBL1 | NM_005638.3 | 326 | aacgttcccgagcctttaatttt | 271103 | - | see also 3909 line |
| 41718 | SYBL1 | NM_005638.3 | 243 | aacgtactcacatggcaattatt | 271095 | - | see also 3909 line |
| 41719 | TLOC1 | NM_003262.2 | 731 | tgggtcaccgggttgattatttt | 271121 | - | see also 2210 line |
| 41720 | TLOC1 | NM_003262.2 | 732 | gggtcaccgggttgattatttta | 271122 | - | see also 2210 line |
| 41721 | TOM1 | NM_005488.1 | 207 | atgccctccgagcagtaaagaag | 271138 | - | see also 18397 line |
| 41722 | TOM1 | NM_005488.1 | 1369 | gggggtcaccagcgaagaatttg | 271151 | - | see also 18397 line |
| 41723 | TOM1L1 | NM_005486.1 | 1188 | cacctcaagccacgcatatgata | 271193 | - | see also 18398 line |
| 41724 | TOM1L1 | NM_005486.1 | 872 | ttggatatgagaggtttactaga | 271182 | - | see also 18398 line |
| 41725 | VDP | NM_003715.1 | 228 | cagaagccgagacgattcaaaag | 271201 | - | see also 2538 line |
| 41726 | VDP | NM_003715.1 | 1255 | accaagaccggcaattgtagtac | 271255 | - | see also 2538 line |
| 41727 | VPS18 | NM_080432.1 | 469 | ccccttccgagcgcattaccagt | 271305 | - | see also 9514 line |
| 41728 | VPS18 | NM_080432.1 | 1536 | ttcgatctggccaaagagtattg | 271315 | - | see also 9514 line |
| 41729 | VPS18 | NM_080432.1 | 394 | tcccccactcggggtatgtgaat | 271300 | - | see also 9514 line |
| 41730 | VPS33A | NM_022916.2 | 1462 | aaccccacggacatatcgtatgt | 271369 | - | see also 18401 line |
| 41731 | VPS33A | NM_022916.2 | 1467 | cacggacatatcgtatgtgtaca | 271370 | - | see also 18401 line |
| 41732 | VPS33B | NM_018668.2 | 646 | ggctggccgaactcgcaaataca | 271387 | - | see also 7540 line |
| 41733 | VPS33B | NM_018668.2 | 1571 | aaggattaccgatctctgaaaac | 271405 | - | see also 7540 line |
| 41734 | VPS33B | NM_018668.2 | 1826 | gacggcgagtatgatctgaaagt | 271411 | - | see also 7540 line |
| 41735 | VPS39 | NM_015289.1 | 283 | aggacgttggttgcaacagattt | 271420 | - | see also 18402 line |
| 41736 | VPS39 | NM_015289.1 | 2728 | gagtggtcgtccattacttctgt | 271472 | - | see also 18402 line |
| 41737 | VPS45A | NM_007259.2 | 752 | gccatggtccacgaactactagg | 271501 | - | see also 5062 line |
| 41738 | VPS45A | NM_007259.2 | 200 | tacctctttgaacgcattgattc | 271478 | - | see also 5062 line |
| 41739 | VPS45A | NM_007259.2 | 167 | tacacacaatcggagattctaca | 271476 | - | see also 5062 line |
| 41740 | VTI1A | NM_145206.1 | 119 | gtcgtccgacttcgaaggttacg | 271521 | - | see also 18404 line |
| 41741 | VTI1A | NM_145206.1 | 307 | cccaaagtcgagggatgtacagc | 271524 | - | see also 18404 line |
| 41742 | XPO1 | NM_003400.2 | 1533 | tgggcaataggctccattagtgg | 271583 | - | see also 2294 line |
| 41743 | XPO1 | NM_003400.2 | 2463 | aacaagttaggggggacatataac | 271621 | - | see also 2294 line |
| 41744 | XPO4 | NM_022459.3 | 413 | ctgcgctattgagtgaattttca | 271670 | - | see also 8382 line |
| 41745 | XPO4 | NM_022459.3 | 987 | aaccgtgttcccacgaaatgttt | 271688 | - | see also 8382 line |
| 41746 | CTNS | NM_004937.1 | 1163 | cagtcacgctggtcaagtatttt | 271791 | - | see also 3433 line |
| 41747 | CTNS | NM_004937.1 | 1068 | cgcgtggctcttcgcatttgtca | 271790 | - | see also 3433 line |
| 41748 | XK | NM_021083.2 | 750 | ttgtagtcctggtcctctttacc | 271803 | - | see also 8082 line |
| 41749 | XK | NM_021083.2 | 539 | accctacagctgtacataagtgt | 271798 | - | see also 8082 line |
| 41750 | PITPNC1 | NM_012417.2 | 381 | ctcaccgtagacgagtacaaaat | 271820 | - | see also 18409 line |
| 41751 | PITPNC1 | NM_012417.2 | 384 | accgtagacgagtacaaaattgg | 271822 | - | see also 18409 line |
| 41752 | APOA2 | NM_001643.1 | 295 | tggaacggaactggttaacttct | 271853 | - | see also 18410 line |
| 41753 | APOA2 | NM_001643.1 | 292 | ggctggaacggaactggttaact | 271851 | - | see also 18410 line |

Figure 46

| 41754 | APOC1 | NM_001645.2 | 241 | aagatgcgggagtggttttcaga | 271855 | - | - | lipid transporter | Alzheimer disease |
|--------|----------|-------------|------|----------------------------|--------|---|-------------------|-------------------|-------------------|
| 41755 | APOC3 | NM_000040.1 | 133 | cagcttcatgcagggttacatga | 271856 | - | see also 18411 line | | |
| 41756 | APOC4 | NM_001646.1 | 278 | ggcttcatgcagacctactatga | 271860 | - | see also 18412 line | | |
| 41757 | APOC4 | NM_001646.1 | 153 | ccccaccaaagctaaagatgagt | 271858 | - | see also 18412 line | | |
| 41758 | APOL1 | NM_003661.2 | 254 | agcgagggtgcaacaaaacgttc | 271864 | - | see also 18413 line | | |
| 41759 | APOL1 | NM_003661.2 | 814 | ccgctttgaccgggattaccagc | 271873 | - | see also 18413 line | | |
| 41760 | APOL3 | NM_014349.2 | 1135 | aaccagcattgaccgattgaagg | 271888 | - | see also 18414 line | | |
| 41761 | APOL3 | NM_014349.2 | 766 | agctattgaggacgaatatgtgc | 271882 | - | see also 18414 line | | |
| 41762 | APOL6 | NM_030641.2 | 973 | ctggcgatgaccaaaaatgctcg | 271903 | - | see also 18415 line | | |
| 41763 | APOL6 | NM_030641.2 | 481 | ctcgcagacgatattgacaaaac | 271895 | - | see also 18415 line | | |
| 41764 | APOL6 | NM_030641.2 | 844 | aacgtccgtgcattttggaaact | 271899 | - | see also 18415 line | | |
| 41765 | APOM | NM_019101.2 | 99 | tgctctacttctatggtattatc | 271907 | - | see also 7707 line | | |
| 41766 | APOM | NM_019101.2 | 616 | ggcctgtgagctgtccaataact | 271913 | - | see also 7707 line | | |
| 41767 | APOM | NM_019101.2 | 581 | tccaaagccttcttattgactcc | 271912 | - | see also 7707 line | | |
| 41768 | FHR-4 | NM_006684.1 | 225 | ctgcaggacaatcttattcctat | 271915 | - | see also 18416 line | | |
| 41769 | FHR-4 | NM_006684.1 | 614 | tacgatggatatgaaatcagtta | 271920 | - | see also 18416 line | | |
| 41770 | SAA4 | NM_006512.1 | 314 | gggtctatcttcagggattaata | 271932 | - | see also 18417 line | | |
| 41771 | SAA4 | NM_006512.1 | 183 | cagagcctattgggacataatga | 271922 | - | see also 18417 line | | |
| 41772 | SLCO2A1 | NM_005630.1 | 158 | ctcggtcttcggcaacattaagg | 271938 | - | see also 18418 line | | |
| 41773 | SLCO2A1 | NM_005630.1 | 1858 | gggcctgcgcctactatgacaac | 271962 | - | see also 18418 line | | |
| 41774 | SLC18A1 | NM_003053.1 | 676 | acccgggtcggggttctgtttgc | 271967 | - | see also 2047 line | | |
| 41775 | SLC18A1 | NM_003053.1 | 550 | agcgtacctagtggaatagcatg | 271963 | - | see also 2047 line | | |
| 41776 | SLC18A1 | NM_003053.1 | 675 | tacccgggtcggggttctgtttg | 271966 | - | see also 2047 line | | |
| 41777 | SLC18A2 | NM_003054.1 | 1260 | ttcgtcaatgatgcctatcatgg | 272000 | - | see also 2048 line | | |
| 41778 | CTL1 | NM_022109.2 | 739 | ctggtcatactcggttcacttgg | 272037 | - | see also 8271 line | | |
| 41779 | CTL1 | NM_022109.2 | 1365 | tccgcgaatgatccttatgtata | 272055 | - | see also 8271 line | | |
| 41780 | SLC5A7 | NM_021815.2 | 1795 | ttgccacctaaattagatgtatt | 272121 | - | see also 8176 line | | |
| 41781 | SLC5A7 | NM_021815.2 | 622 | gacccgtttcagcaaatctatgg | 272087 | - | see also 8176 line | | |
| 41782 | SLC35B1 | NM_005827.1 | 856 | atctttatgacggttgtgtattt | 272139 | - | see also 18423 line | | |
| 41783 | SLC35B1 | NM_005827.1 | 903 | cactacaactcgaaagttcttca | 272140 | - | see also 18423 line | | |
| 41784 | SLC35A5 | NM_017945.2 | 335 | ctcaagtcgcatcttactagtga | 272148 | - | see also 18424 line | | |
| 41785 | SLC35A5 | NM_017945.2 | 740 | tgccgggactaaaactttacagc | 272158 | - | see also 18424 line | | |
| 41786 | SLC35B4 | NM_032826.2 | 842 | aagagactctctacaaacgattt | 272184 | - | see also 9294 line | | |
| 41787 | SLC35B4 | NM_032826.2 | 1114 | gtcgtgaccctacgcaaatttgt | 272192 | - | see also 9294 line | | |
| 41788 | NUP107 | NM_020401.1 | 1317 | atccatatagacgcatttggaaa | 272227 | - | see also 18426 line | | |
| 41789 | NUP107 | NM_020401.1 | 305 | accccaacaagccgaagcttact | 272196 | - | see also 18426 line | | |
| 41790 | NUP155 | NM_004298.2 | 1826 | aacaccgtctcatggtatacagc | 272318 | - | see also 18427 line | | |

Figure 46

| 41791 | NUP155 | NM_004298.2 | 1670 | tactcgggctttctttaggtatg | 272316 | - | see also 18427 line | | |
|---|---|---|---|---|---|---|---|---|---|
| 41792 | NUP155 | NM_004298.2 | 462 | ttggcgacccctgtagacatagt | 272277 | - | see also 18427 line | | |
| 41793 | UPF3A | NM_023011.2 | 423 | atcgttttgatggatatatcttc | 272406 | - | see also 18428 line | | |
| 41794 | UPF3A | NM_023011.2 | 341 | gccgacctgagtctttatcctca | 272400 | - | see also 18428 line | | |
| 41795 | UPF3B | NM_023010.2 | 1006 | agcgttctgatagcgaacttaaa | 272436 | - | see also 18429 line | | |
| 41796 | UPF3B | NM_023010.2 | 522 | accaaagtcgggactatcgatga | 272430 | - | see also 18429 line | | |
| 41797 | CHAC | NM_015186.1 | 3180 | cccgacttgaaaagtacctataa | 272551 | - | see also 6271 line | | |
| 41798 | CHAC | NM_015186.1 | 1054 | atcgccgatctgattttgacttt | 272477 | - | see also 6271 line | | |
| 41799 | KIAA1012 | NM_014939.1 | 2749 | ggccctgatcgacgtttagatcc | 272837 | - | see also 6121 line | | |
| 41800 | KIAA1012 | NM_014939.1 | 142 | gagcggctcactcgtctcaatca | 272747 | - | see also 6121 line | | |
| 41801 | SEDL | NM_014563.2 | 465 | ttcgatcaagtgcatttgacaga | 272880 | - | transcription_regulator | intracellular transporter | osteoarthritis |
| 41802 | VPS41 | NM_014396.2 | 1068 | atcgtgagtccgagagatgttgt | 272905 | - | see also 5661 line | | |
| 41803 | VPS41 | NM_014396.2 | 487 | ttgaacggtcttggatgaacaga | 272894 | - | see also 5661 line | | |
| 41804 | DEFA4 | NM_001925.1 | 241 | gtctgctcttgcagattagtatt | 272939 | - | see also 18433 line | | |
| 41805 | DEFA4 | NM_001925.1 | 305 | gtggtgtgagtttcacatactgc | 272940 | - | see also 18433 line | | |
| 41806 | PLN | NM_002667.2 | 200 | ctcactcgctcagctataagaag | 272942 | - | see also 18434 line | | |
| 41807 | PLN | NM_002667.2 | 198 | acctcactcgctcagctataaga | 272941 | - | see also 18434 line | | |
| 41808 | PLN | NM_002667.2 | 250 | agcacgtcaaaagctacagaatc | 272943 | - | see also 18434 line | | |
| 41809 | CLNS1A | NM_001293.1 | 254 | ctggaataccccaccattagttt | 272947 | - | see also 18435 line | | |
| 41810 | CLNS1A | NM_001293.1 | 304 | ctgtctaggagagcatttgtatg | 272949 | - | see also 18435 line | | |
| 41811 | NNAT | NM_005386.2 | 148 | ctcggctgaactgctcatcatcg | 272954 | - | see also 3751 line | | |
| 41812 | CYGB | NM_134268.3 | 292 | tcctggtgaggttctttgtgaac | 272960 | - | see also 18436 line | | |
| 41813 | CYGB | NM_134268.3 | 511 | aggtggaaccggtgtacttcaag | 272961 | - | see also 18436 line | | |
| 41814 | HBB | NM_000518.4 | 397 | cccatcactttggcaaagaattc | 272966 | - | see also 18437 line | | |
| 41815 | HBB | NM_000518.4 | 396 | gcccatcactttggcaaagaatt | 272965 | - | see also 18437 line | | |
| 41816 | HBD | NM_000519.2 | 472 | ccctggctcacaagtaccattga | 272967 | - | - | translation regulator | - |
| 41817 | HBE1 | NM_005330.3 | 574 | gggtaacgtgatggtgattattc | 272970 | - | see also 18438 line | | |
| 41818 | HBE1 | NM_005330.3 | 463 | ttcctttggagatgctattaaaa | 272968 | - | see also 18438 line | | |
| 41819 | NGB | NM_021257.2 | 702 | gggtgagtctctgctctacatgc | 272974 | - | see also 18441 line | | |
| 41820 | SORT1 | NM_002959.3 | 1309 | agcgtgctctccgaagataattc | 273008 | - | see also 1996 line | | |
| 41821 | SORT1 | NM_002959.3 | 1310 | gcgtgctctccgaagataattct | 273009 | - | see also 1996 line | | |
| 41822 | SORT1 | NM_002959.3 | 1682 | gccgtcctatcaatgtgattaag | 273017 | - | see also 1996 line | | |
| 41823 | TETRAN | NM_001120.2 | 1407 | cacgtggtccgggctcttttgc | 273040 | - | see also 18443 line | | |
| 41824 | TETRAN | NM_001120.2 | 353 | cagtggagaagaggtacaacagt | 273038 | - | see also 18443 line | | |
| 41825 | SLC29A1 | NM_004955.1 | 787 | aagtgccttcggctactttatca | 273050 | - | see also 3445 line | | |
| 41826 | SLC29A1 | NM_004955.1 | 611 | atcaagatcgtgctcattaattc | 273047 | - | see also 3445 line | | |

Figure 46

| | | | | | | | |
|---|---|---|---|---|---|---|---|
| 41827 | SLC29A2 | NM_001532.2 | 1430 | ctgtcttgccgtttctaatgg | 273061 | - | see also 18445 line |
| 41828 | SLC29A2 | NM_001532.2 | 887 | cgctactacctggccaataaatc | 273058 | - | see also 18445 line |
| 41829 | SLC33A1 | NM_004733.2 | 974 | atggttgggcgttaactatgtta | 273079 | - | see also 3250 line |
| 41830 | SLC33A1 | NM_004733.2 | 764 | tggttgatgcggtcacgttaag | 273066 | - | see also 3250 line |
| 41831 | AP2B1 | NM_001282.1 | 714 | tgccgtagccggcattatctgaaa | 273115 | - | see also 18447 line |
| 41832 | AP2B1 | NM_001282.1 | 715 | gccgtagccggcattatctgaaat | 273116 | - | see also 18447 line |
| 41833 | AP2B1 | NM_001282.1 | 2369 | accgccaagggcacatctatatg | 273130 | - | see also 18447 line |
| 41834 | AP2M1 | NM_004068.2 | 304 | cacgttaagcggtccaacatttg | 273141 | - | see also 18448 line |
| 41835 | AP2M1 | NM_004068.2 | 305 | acgttaagcggtccaacatttgg | 273142 | - | see also 18448 line |
| 41836 | AP2S1 | NM_004069.2 | 124 | gtggtacatgcagtttgatgatg | 273160 | - | see also 18449 line |
| 41837 | AP2S1 | NM_004069.2 | 197 | gacgccaaacacaccaactttgt | 273163 | - | see also 18449 line |
| 41838 | AP4B1 | NM_006594.1 | 295 | atgtcatccagcgagtgattagg | 273168 | - | see also 4625 line |
| 41839 | AP4B1 | NM_006594.1 | 1738 | gggaccgaggtctcttctattat | 273216 | - | see also 4625 line |
| 41840 | AP4M1 | NM_004722.2 | 1492 | cacagccgacgcctatgtcattcg | 273252 | - | see also 18451 line |
| 41841 | AP4M1 | NM_004722.2 | 1085 | gccggctccagtttatcttaaag | 273250 | - | see also 18451 line |
| 41842 | AP4S1 | NM_007077.2 | 211 | gactcgactttctaagctactatg | 273256 | - | see also 18452 line |
| 41843 | AP4S1 | NM_007077.2 | 458 | agccgagtgagtgaattagatgt | 273268 | - | see also 18452 line |
| 41844 | AQP3 | NM_004925.3 | 450 | ttcgccgacaaccagcttttgt | 273273 | - | see also 18453 line |
| 41845 | AQP3 | NM_004925.3 | 517 | ctggaacttggatatgatcaat | 273278 | - | see also 18453 line |
| 41846 | C16orf7 | NM_004913.1 | 422 | gccgccaagcttgggaaaacacg | 273284 | - | see also 18454 line |
| 41847 | C16orf7 | NM_004913.1 | 191 | agggcacctggccggaataatgg | 273283 | - | see also 18454 line |
| 41848 | C20orf121 | NM_024331.2 | 378 | tccgagcccgcaagtttgattac | 273288 | - | see also 8611 line |
| 41849 | C20orf121 | NM_024331.2 | 450 | gggcccgaagtcttcaataactg | 273293 | - | see also 8611 line |
| 41850 | C20orf26 | NM_015585.2 | 1551 | aggacttagacgttacaacaag | 273340 | - | see also 18456 line |
| 41851 | C20orf26 | NM_015585.2 | 1930 | gcgaccacgacgacagattgtct | 273349 | - | see also 18456 line |
| 41852 | C20orf59 | NM_022082.2 | 1041 | ggcgattccggccagtctattca | 273403 | - | see also 18457 line |
| 41853 | C20orf59 | NM_022082.2 | 1063 | agcgcgtttctctgatcatct | 273405 | - | see also 18457 line |
| 41854 | CLCA3 | NM_004921.1 | 352 | tacggagtgatgatccctatacact | 273421 | - | see also 18458 line |
| 41855 | CLCA3 | NM_004921.1 | 56 | tgcttctccgcctgattgaaa | 273409 | - | see also 18458 line |
| 41856 | CPNE7 | NM_014427.3 | 1167 | agcagaagacgcagttataag | 273438 | - | see also 18459 line |
| 41857 | CPNE7 | NM_014427.3 | 1642 | caccgggaaagcctctcaatact | 273439 | - | see also 18459 line |
| 41858 | DAZAP2 | NM_014764.1 | 247 | accatgtcagccgcatttcctgg | 273440 | - | - |
| 41859 | EPB41L4B | NM_018424.1 | 1412 | atccaatagatccgcactttatca | 273461 | - | see also 7461 line |
| 41860 | EVER1 | NM_007267.5 | 1819 | atgttgctgacacgctttttgg | 273499 | - | see also 18460 line |
| 41861 | EVER1 | NM_007267.5 | 483 | gccctgctcgggaactttgtcc | 273496 | - | see also 18460 line |
| 41862 | FLJ14153 | NM_022736.1 | 657 | aaccctcgggatccacacttatga | 273525 | - | see also 8442 line |
| 41863 | FLJ14153 | NM_022736.1 | 340 | gaccgagtatttggaatacgatg | 273513 | - | see also 8442 line |

Figure 46

| 41864 | FLJ14153 | NM_022736.1 | 242 | aacgagatatgcaagtgaatacc | 273508 | - | see also 8442 line |
|---|---|---|---|---|---|---|---|
| 41865 | FLJ25217 | NM_152351.2 | 1023 | tccgtctctcgaacagatgatgt | 273539 | - | see also 18462 line |
| 41866 | FLJ25217 | NM_152351.2 | 746 | tacagacgccatgcacatgtttc | 273537 | - | see also 18462 line |
| 41867 | FLJ40121 | NM_175904.2 | 1058 | tccgcaaaggtcttagtatcatc | 273571 | - | see also 18463 line |
| 41868 | FLJ40121 | NM_175904.2 | 1457 | cagctgacaacaccggattttga | 273575 | - | see also 18463 line |
| 41869 | FLJ40121 | NM_175904.2 | 1055 | ccctccgcaaaggtcttagtatc | 273570 | - | see also 18463 line |
| 41870 | GASP | NM_014710.1 | 4273 | atccgtcctcccgagttagaaca | 273648 | - | see also 18464 line |
| 41871 | GASP | NM_014710.1 | 2038 | agcgtattggtgattccttattt | 273620 | - | see also 18464 line |
| 41872 | GOLGA3 | NM_005895.2 | 3370 | gtcgttgcagttcgataaggagc | 273675 | - | see also 4098 line |
| 41873 | GOLGA3 | NM_005895.2 | 3366 | tgcagtcgttgcagttcgataag | 273674 | - | see also 4098 line |
| 41874 | HIATL2 | NM_032318.1 | 449 | ttcctcatgaatggtctcattca | 273690 | - | see also 18466 line |
| 41875 | HIATL2 | NM_032318.1 | 460 | tggtctcattcaaggtgtaaagg | 273691 | - | see also 18466 line |
| 41876 | JUB | NM_032876.4 | 1733 | accgactaccacaaaaattatgc | 273699 | - | see also 9330 line |
| 41877 | LCN2 | NM_005564.2 | 497 | agcatgctatggtgttcttcaag | 273714 | - | see also 18468 line |
| 41878 | LCN2 | NM_005564.2 | 348 | ctccgtcctgtttaggaaaaaga | 273709 | - | see also 18468 line |
| 41879 | LGI3 | NM_139278.1 | 1051 | tgccgctgcgggagttcgattgc | 273722 | - | see also 18469 line |
| 41880 | LGI3 | NM_139278.1 | 1908 | gagggacgacagaagtttgtacg | 273733 | - | see also 18469 line |
| 41881 | LOC127262 | NM_182752.2 | 521 | tccccctaaatcgctcaacaagc | 273738 | - | see also 18470 line |
| 41882 | LOC127262 | NM_182752.2 | 494 | caccatttcctacggagaattcc | 273737 | - | see also 18470 line |
| 41883 | LOC127262 | NM_182752.2 | 627 | gtgccctatgccactttcacaga | 273741 | - | see also 18470 line |
| 41884 | LOC136306 | NM_174959.1 | 115 | cacgaaataccgaggctatatgt | 273747 | - | see also 18471 line |
| 41885 | LOC136306 | NM_174959.1 | 77 | gccgctggaaggttaatcataaa | 273744 | - | see also 18471 line |
| 41886 | LOC136306 | NM_174959.1 | 76 | ggccgctggaaggttaatcataa | 273743 | - | see also 18471 line |
| 41887 | LOC138307 | NM_178469.2 | 490 | gaccgtgaaggttgcatataaca | 273770 | - | see also 18472 line |
| 41888 | LOC138307 | NM_178469.2 | 628 | cacacagctaggccttattgttc | 273774 | - | see also 18472 line |
| 41889 | LOC143425 | NM_175733.2 | 1591 | atcatcggcgtgtgtcaagtagg | 273806 | - | see also 18473 line |
| 41890 | LOC143425 | NM_175733.2 | 370 | ctccgcgacccagatatctcagt | 273777 | - | see also 18473 line |
| 41891 | MGC11308 | NM_032889.2 | 634 | ttcgaggcctggtatatccatga | 273817 | - | see also 18474 line |

Figure 46

| | | | | | | | |
|---|---|---|---|---|---|---|---|
| 41892 | MGC11308 | NM_032889.2 | 872 | accggcagcgtgccttctcaagg | 273818 | - | see also 18474 line |
| 41893 | MGC33302 | NM_152778.1 | 807 | ctgatccttgccatactaagaga | 273856 | - | see also 9952 line |
| 41894 | MGC33302 | NM_152778.1 | 1130 | tggccgagcgtgctattctactgg | 273864 | - | see also 9952 line |
| 41895 | MGC34646 | NM_173519.1 | 1167 | ccccgactacagcgcgatgaaaatga | 273893 | - | see also 18475 line |
| 41896 | MGC34646 | NM_173519.1 | 791 | gtcattggaagtcctaatcgaag | 273883 | - | see also 18475 line |
| 41897 | MGC34646 | NM_173519.1 | 1210 | atgcaatgcacgtgaagcatacg | 273895 | - | see also 18475 line |
| 41898 | MGC34821 | NM_173586.1 | 1123 | ccggtcacgatctatgacaataa | 273911 | - | see also 18476 line |
| 41899 | MGC34821 | NM_173586.1 | 1121 | cccccggtcacgatctatgacaat | 273909 | - | see also 18476 line |
| 41900 | MGC52019 | NM_178498.2 | 942 | tggatctcgactacatatattg | 273937 | - | see also 18477 line |
| 41901 | MGC52019 | NM_178498.2 | 1043 | atggggtcaatcaatcaactatt | 273943 | - | see also 18477 line |
| 41902 | MGC9564 | NM_080669.2 | 829 | ttccgtcaccacccgatccattgt | 273958 | - | see also 9520 line |
| 41903 | MGC9564 | NM_080669.2 | 250 | agcgccgacctcggctacaatgg | 273953 | - | see also 9520 line |
| 41904 | MIP | NM_012064.1 | 560 | acctctttgggatgtattatact | 273969 | - | see also 5103 line |
| 41905 | MTAC2D1 | NM_152332.2 | 1059 | aggttccaacgctattgaattta | 274003 | - | see also 18480 line |
| 41906 | MTAC2D1 | NM_152332.2 | 565 | tcccagcaacgacctagctatga | 273985 | - | see also 18480 line |
| 41907 | NOD3 | NM_178844.1 | 1292 | ccctgtgcgagctctactcatgg | 274018 | - | see also 18481 line |
| 41908 | NOD3 | NM_178844.1 | 3142 | ttggagattctgacttaagagg | 274026 | - | see also 18481 line |
| 41909 | NUP88 | NM_002532.3 | 647 | accaactaacgtgataatacttt | 274054 | - | see also 1630 line |
| 41910 | NUP88 | NM_002532.3 | 1884 | gagcgtttagctgacaaatatga | 274089 | - | see also 1630 line |
| 41911 | PACSIN2 | NM_007229.1 | 1228 | gcccagcagcaccccttaatgtcc | 274100 | - | see also 18484 line |
| 41912 | PACSIN2 | NM_007229.1 | 1226 | aagcccagcagcaacccttaatgt | 274099 | - | see also 18484 line |
| 41913 | PDZK1 | NM_002614.3 | 201 | tcgggtggttgagaagtgtagc | 274105 | - | see also 1690 line |
| 41914 | PDZK1 | NM_002614.3 | 114 | accccgagaatgtaaactgtcc | 274103 | - | see also 1690 line |
| 41915 | PPP1R9B | NM_032595.1 | 1325 | ccccgagccggaagatccatttc | 274115 | - | see also 18486 line |
| 41916 | PPP1R9B | NM_032595.1 | 1374 | cacttactccaacgaggattacg | 274117 | - | see also 18486 line |
| 41917 | SEC22L2 | NM_012430.2 | 958 | gggcaaggctcccgattatgatg | 274162 | - | see also 5314 line |
| 41918 | SEC22L2 | NM_012430.2 | 956 | caggcaaggctccgattatga | 274161 | - | see also 5314 line |
| 41919 | SLC13A5 | NM_177550.2 | 1461 | cggcctcaatccgctgtacatca | 274174 | - | see also 18487 line |

Figure 46

| 41920 | SLC13A5 | NM_177550.2 | 1653 | gggacgggccatatttgacttgg | 274180 | - | see also 18487 line |
|---|---|---|---|---|---|---|---|
| 41921 | SLC15A3 | NM_016582.1 | 1881 | ggccgtcacggctctcctatttg | 274188 | - | see also 18488 line |
| 41922 | SLC15A3 | NM_016582.1 | 1882 | gccgtcacggctctcctatttgt | 274189 | - | see also 18488 line |
| 41923 | SLC17A6 | NM_020346.1 | 2119 | tacaagactcacatagctataag | 274234 | - | see also 18489 line |
| 41924 | SLC17A6 | NM_020346.1 | 2133 | agctataaggaccgagttgatta | 274235 | - | see also 18489 line |
| 41925 | SLC17A8 | NM_139319.1 | 1062 | aacgtggttagtctaagtaaatt | 274257 | - | see also 18490 line |
| 41926 | SLC17A8 | NM_139319.1 | 489 | aacaatagcaccgtatatgttga | 274240 | - | see also 18490 line |
| 41927 | SLC22A12 | NM_144585.2 | 2016 | cacgctggtgccccacgaaatgg | 274278 | - | see also 18491 line |
| 41928 | SLC22A15 | NM_018420.1 | 418 | atcgcctcggagtggtttttaat | 274282 | - | see also 18492 line |
| 41929 | SLC22A15 | NM_018420.1 | 866 | ctgaatcacctcgttggttatac | 274297 | - | see also 18492 line |
| 41930 | SLC22A16 | NM_033125.2 | 1143 | cacttaccgtttggctaatctgg | 274336 | - | see also 18493 line |
| 41931 | SLC22A16 | NM_033125.2 | 1652 | gggaaacggctagcaactacttg | 274349 | - | see also 18493 line |
| 41932 | SLC22A17 | NM_016609.2 | 266 | gtccgcacggtggctgatagtga | 274353 | - | see also 6950 line |
| 41933 | SLC22A9 | NM_080866.1 | 749 | atcctaggcggtcatttatcaga | 274362 | - | see also 18495 line |
| 41934 | SLC22A9 | NM_080866.1 | 1328 | ctgtcctttacgagatttgcaaa | 274369 | - | see also 18495 line |
| 41935 | SLC23A3 | NM_144712.2 | 1439 | gggagccatgccctgaatctagc | 274384 | - | see also 18496 line |
| 41936 | SLC23A3 | NM_144712.2 | 721 | ggcgagcttcaacgtcatcaact | 274377 | - | see also 18496 line |
| 41937 | SLC37A3 | NM_032295.1 | 1115 | tacgacgttggagggatcatagt | 274418 | - | see also 9147 line |
| 41938 | SLC37A3 | NM_032295.1 | 893 | gacgaatatgagccgaattattc | 274407 | - | see also 9147 line |
| 41939 | SLC5A8 | NM_145913.2 | 1599 | agcactcagcgtatttggtatgg | 274466 | - | see also 9804 line |
| 41940 | SLC5A8 | NM_145913.2 | 705 | cacctacgagtatttagaacttc | 274439 | - | see also 9804 line |
| 41941 | SLCO1C1 | NM_017435.2 | 2134 | ttgggtatctacacattagcaat | 274534 | - | see also 18499 line |
| 41942 | SLCO1C1 | NM_017435.2 | 1051 | gtgcagacggttgcaattatagg | 274499 | - | see also 18499 line |
| 41943 | SLCO2B1 | NM_007256.1 | 2176 | ctgtctgtcgctactacaataat | 274560 | - | see also 18500 line |
| 41944 | SLCO2B1 | NM_007256.1 | 600 | gtgcaccgaccccgaatgattgg | 274548 | - | see also 18500 line |
| 41945 | SLCO3A1 | NM_013272.2 | 818 | aaggactcctcgctctatatagg | 274572 | - | see also 5383 line |
| 41946 | SLCO3A1 | NM_013272.2 | 699 | accggacggctaccaacatgatg | 274567 | - | see also 5383 line |
| 41947 | SLCO4A1 | NM_016354.3 | 2188 | ggccatgagccgctacatactca | 274615 | - | see also 18502 line |
| 41948 | SLCO4A1 | NM_016354.3 | 1765 | ctcggacggcctcatgtacttct | 274604 | - | see also 18502 line |
| 41949 | SLCO4A1 | NM_016354.3 | 2054 | atccagtggattgtagttagaat | 274611 | - | see also 18502 line |
| 41950 | SLCO4C1 | NM_180991.4 | 629 | ggccgtcacgcaaggtattgtag | 274624 | - | see also 18503 line |
| 41951 | SLCO4C1 | NM_180991.4 | 627 | ttggccgtcacgcaaggtattgt | 274623 | - | see also 18503 line |
| 41952 | SLCO5A1 | NM_030958.1 | 2804 | aacgtgacgtcgtttcgttttgt | 274751 | - | see also 8975 line |
| 41953 | SLCO5A1 | NM_030958.1 | 2632 | aacactcaggtccgtagaagatg | 274747 | - | see also 8975 line |
| 41954 | SLCO5A1 | NM_030958.1 | 2541 | aacggacctgcaatactcttatc | 274739 | - | see also 8975 line |
| 41955 | SLCO6A1 | NM_173488.2 | 1110 | gtcgttgcatggtgtacattaat | 274790 | - | see also 18505 line |
| 41956 | SLCO6A1 | NM_173488.2 | 2102 | cacaggacgttgttggatatata | 274823 | - | see also 18505 line |

Figure 46

| 41957 | SMBP | NM_020123.2 | 1456 | tacaatacttggccgaaatctgt | 274859 | - | see also 7758 line |
|---|---|---|---|---|---|---|---|
| 41958 | SPINL | NM_032038.1 | 806 | gggtcggggaggccagttattcc | 274887 | - | see also 18507 line |
| 41959 | SPINL | NM_032038.1 | 1461 | tagcatcgtggccacttatattt | 274889 | - | see also 18507 line |
| 41960 | SV2A | NM_014849.1 | 1903 | agcgcgtagagcatgtaactttt | 274907 | - | see also 18508 line |
| 41961 | SV2A | NM_014849.1 | 2140 | gccgtctgataaacagtacattc | 274915 | - | see also 18508 line |
| 41962 | SV2B | NM_014848.1 | 801 | gggggtgctctaccgattgtttt | 274928 | - | see also 18509 line |
| 41963 | SV2B | NM_014848.1 | 802 | ggggtgctctaccgattgttttt | 274929 | - | see also 18509 line |
| 41964 | SV2B | NM_014848.1 | 803 | gggtgctctaccgattgtttttg | 274930 | - | see also 18509 line |
| 41965 | SYPL | NM_006754.2 | 399 | tcctcataggcgattactcttct | 274958 | - | see also 18510 line |
| 41966 | SYPL | NM_006754.2 | 398 | gtcctcataggcgattactcttc | 274957 | - | see also 18510 line |
| 41967 | SYPL | NM_006754.2 | 506 | ctgtatctggatagtcgtaaact | 274961 | - | see also 18510 line |
| 41968 | SYT11 | NM_152280.2 | 557 | ggcctgctaagccgagacaaaga | 274981 | - | see also 9827 line |
| 41969 | SYT12 | NM_177963.2 | 217 | ctctccgttccccaattacgact | 275001 | - | see also 10173 line |
| 41970 | SYT12 | NM_177963.2 | 214 | cccctctccgttccccaattacg | 275000 | - | see also 10173 line |
| 41971 | SYT13 | NM_020826.1 | 909 | gagctggagaggtcctactatcc | 275012 | - | see also 7984 line |
| 41972 | SYT13 | NM_020826.1 | 1087 | acgagctaagcacaagatcaacc | 275015 | - | see also 7984 line |
| 41973 | SYT14 | NM_153262.1 | 1057 | atggtgtacatcgcatgaaaaaa | 275045 | - | see also 9980 line |
| 41974 | SYT14 | NM_153262.1 | 296 | agcgctgggtaaatatcatgagg | 275029 | - | see also 9980 line |
| 41975 | SYT14 | NM_153262.1 | 410 | atcggcagagtatgatggataca | 275031 | - | see also 9980 line |
| 41976 | SYT14L | NM_031914.1 | 379 | gccagtgatgaccgcaagttacc | 275078 | - | see also 18515 line |
| 41977 | SYT14L | NM_031914.1 | 748 | gccagccaacggcgttattctga | 275087 | - | see also 18515 line |
| 41978 | SYT2 | NM_177402.3 | 722 | tcctccttcctgacaagaagaag | 275111 | - | see also 18516 line |
| 41979 | SYT2 | NM_177402.3 | 780 | ccctgccttcaatgaaaccttca | 275112 | - | see also 18516 line |
| 41980 | SYT3 | NM_032298.1 | 2110 | cacgctgaaccccacctataatg | 275121 | - | see also 9149 line |
| 41981 | SYT4 | NM_020783.2 | 1241 | ggggtcccgaaatgaggtaatcg | 275148 | - | see also 7967 line |
| 41982 | SYT4 | NM_020783.2 | 1027 | aagctcgacatctgcctaaatct | 275141 | - | see also 7967 line |
| 41983 | SYT7 | NM_004200.2 | 1007 | atgagtccttcgccttcgatatc | 275157 | - | see also 2811 line |
| 41984 | SYTL4 | NM_080737.1 | 1504 | agcgctctaacccatatgtgaag | 275182 | - | see also 9521 line |
| 41985 | SYTL4 | NM_080737.1 | 1375 | ctggtgatttcgggaacatcttt | 275181 | - | see also 9521 line |
| 41986 | TCOF1 | NM_000356.1 | 3602 | caggcatgttgtcccctaaaaca | 275209 | - | see also 18521 line |
| 41987 | TCOF1 | NM_000356.1 | 2974 | aaggagtccagtcggatatcaga | 275205 | - | see also 18521 line |
| 41988 | TM9SF1 | NM_006405.3 | 810 | ctggctcggtacaacttagatga | 275225 | - | see also 4487 line |
| 41989 | TM9SF1 | NM_006405.3 | 1487 | gggtcgggagcagtacactttgt | 275237 | - | see also 4487 line |
| 41990 | TM9SF2 | NM_004800.1 | 454 | ttcaccatataagtttacgttta | 275253 | - | see also 3332 line |
| 41991 | TM9SF2 | NM_004800.1 | 1665 | ttcctgaacagtcgttctacacg | 275304 | - | see also 3332 line |
| 41992 | TM9SF4 | NM_014742.1 | 1481 | ggccgtctgtaccgcacctttaaa | 275338 | - | see also 5939 line |
| 41993 | TM9SF4 | NM_014742.1 | 367 | gcctggggtcgcgcctatcaact | 275325 | - | see also 5939 line |

Figure 46

| 41994 | CNOT2 | NM_014515.1 | 1128 | tacctgatggtcgggttactaac | 275394 | - | see also 5729 line |
|---|---|---|---|---|---|---|---|
| 41995 | CNOT2 | NM_014515.1 | 1333 | accttgtcgacctcaagacatag | 275405 | - | see also 5729 line |
| 41996 | GTF2B | NM_001514.2 | 581 | tgccgtatcacgaatttctaaga | 275440 | - | see also 1042 line |
| 41997 | GTF2B | NM_001514.2 | 370 | atcgggcaatgatgaatgcattc | 275430 | - | see also 1042 line |
| 41998 | GTF2E1 | NM_005513.1 | 334 | atcaattatcgtactcttgttaa | 275465 | - | see also 18527 line |
| 41999 | GTF2E1 | NM_005513.1 | 309 | aaccactcgccataactactact | 275459 | - | see also 18527 line |
| 42000 | GTF2E2 | NM_002095.3 | 1047 | agcgacgctttaagactcataac | 275510 | - | see also 1358 line |
| 42001 | GTF2E2 | NM_002095.3 | 819 | ttgtaaatcgtcccgataagaag | 275507 | - | see also 1358 line |
| 42002 | TAF1C | NM_005679.2 | 630 | agcggggagccgcactaagaaga | 275519 | - | see also 18529 line |
| 42003 | TAF1C | NM_005679.2 | 1176 | tgccgtgtggaagtttggtaaac | 275526 | - | see also 18529 line |
| 42004 | BLZF1 | NM_003666.2 | 970 | aacgtctagcccgtgagaaaaat | 275549 | - | see also 2491 line |
| 42005 | BLZF1 | NM_003666.2 | 971 | acgtctagcccgtgagaaaaatc | 275550 | - | see also 2491 line |
| 42006 | CEP1 | NM_007018.3 | 6780 | cccaactccgacactgtatgtcc | 275727 | - | see also 4897 line |
| 42007 | EAP30 | NM_007241.2 | 645 | gtgagatcaaagccagtcttaaa | 275744 | - | see also 18532 line |
| 42008 | EAP30 | NM_007241.2 | 150 | accagctagcccagatgtcaaag | 275733 | - | see also 18532 line |
| 42009 | ELL2 | NM_012081.3 | 809 | atcaaacccggtggaccatatgt | 275758 | - | see also 18533 line |
| 42010 | ELL2 | NM_012081.3 | 535 | aggactccacgggcttgtcaaaa | 275748 | - | see also 18533 line |
| 42011 | ELL2 | NM_012081.3 | 730 | aacaaacgactcgtatcagatga | 275754 | - | see also 18533 line |
| 42012 | LITAF | NM_004862.1 | 855 | ggggaatacgtcgcaaaactaac | 275791 | - | see also 18534 line |
| 42013 | LITAF | NM_004862.1 | 854 | ggggaatacgtcgcaaaactaa | 275790 | - | see also 18534 line |
| 42014 | LITAF | NM_004862.1 | 435 | atccccaataacaatccaattac | 275785 | - | see also 18534 line |
| 42015 | SUPT3H | NM_003599.1 | 286 | tgcggggagcaagggtaatcact | 275800 | - | see also 18535 line |
| 42016 | SUPT3H | NM_003599.1 | 712 | atgttgtcgcaatggaaatctta | 275812 | - | see also 18535 line |
| 42017 | SURB7 | NM_004264.2 | 288 | ggctgctagcttgtataagctag | 275821 | - | see also 18536 line |
| 42018 | SURB7 | NM_004264.2 | 294 | tagcttgtataagctagaagaag | 275822 | - | see also 18536 line |
| 42019 | EPC1 | NM_025209.2 | 1264 | atccgaccgaaacggaaatatga | 275856 | - | see also 8877 line |
| 42020 | EPC1 | NM_025209.2 | 976 | aagctgcgacgagatctaagtcg | 275846 | - | see also 8877 line |
| 42021 | BTF | NM_014739.1 | 423 | atcgtcgcgattacagaaataat | 275913 | - | see also 5928 line |
| 42022 | BTF | NM_014739.1 | 2142 | agcggttcacttcgtatcagaaa | 275970 | - | see also 5928 line |
| 42023 | EED | NM_003797.2 | 1089 | aagatcatgctttacgattatgg | 276010 | - | see also 2593 line |
| 42024 | NAB1 | NM_005966.2 | 765 | ttcggaaatacagtgcaatatat | 276039 | - | see also 18538 line |
| 42025 | NAB1 | NM_005966.2 | 933 | ctcgagaagtcacctataaatat | 276043 | - | see also 18538 line |
| 42026 | REST | NM_005612.3 | 1005 | cgctgcggctacaatactaatcg | 276075 | - | see also 3900 line |
| 42027 | REST | NM_005612.3 | 1008 | tgcggctacaatactaatcgata | 276076 | - | see also 3900 line |
| 42028 | REST | NM_005612.3 | 1078 | atgagcgagtctacaagtgtatc | 276083 | - | see also 3900 line |
| 42029 | ELL | NM_006532.1 | 1521 | gtcggagacgcctgactacttgc | 276141 | - | see also 18540 line |
| 42030 | ELL | NM_006532.1 | 1719 | tactcgagggcagattttgcagg | 276144 | - | see also 18540 line |

Figure 46

| | | | | | | | |
|---|---|---|---|---|---|---|---|
| 42031 | GTF3C3 | NM_012086.1 | 584 | aacgtgaagaggcgatattgatg | 276155 | - | see also 18541 line |
| 42032 | GTF3C3 | NM_012086.1 | 2647 | cagttagacttacgaagagatat | 276241 | - | see also 18541 line |
| 42033 | GTF3C5 | NM_012087.1 | 1179 | ggcgcagcctatgattcgattt | 276256 | - | see also 18542 line |
| 42034 | GTF3C5 | NM_012087.1 | 1200 | ttgggtatgaccccgaaaaaac | 276259 | - | see also 18542 line |
| 42035 | A1BG | NM_130786.2 | 938 | cgccggtcgagctgattctgagc | 276277 | - | see also 18544 line |
| 42036 | A1BG | NM_130786.2 | 891 | ccgctaccggctgcatgacaacc | 276275 | - | see also 18544 line |
| 42037 | A2LP | NM_007245.2 | 687 | ccctcgtcgggaggacattgtgg | 276290 | - | see also 18545 line |
| 42038 | A2LP | NM_007245.2 | 509 | ttgaaggcgtctacaacaattcc | 276280 | - | see also 18545 line |
| 42039 | AAAS | NM_015665.3 | 642 | gactcagtccgtgtgtataatgc | 276331 | - | see also 6536 line |
| 42040 | AAAS | NM_015665.3 | 162 | cggggtcaagtcaccctatatga | 276320 | - | see also 6536 line |
| 42041 | ACRBP | NM_032489.2 | 160 | ctctctcctacgaatacgaacg | 276341 | - | see also 18547 line |
| 42042 | ACRBP | NM_032489.2 | 265 | ctcgtcagctgaccaatatga | 276342 | - | see also 18547 line |
| 42043 | ACTL6 | NM_016188.3 | 1183 | accgagcatgcgactgaaactca | 276368 | - | see also 6714 line |
| 42044 | AF1Q | NM_006818.2 | 435 | aaggcctggtctgtcagataca | 276373 | - | see also 18549 line |
| 42045 | AF1Q | NM_006818.2 | 371 | agccagtacagttcctttctttt | 276372 | - | see also 18549 line |
| 42046 | AMMECR1 | NM_015365.1 | 1006 | tacaaagctccgattactaatga | 276386 | - | see also 6397 line |
| 42047 | AMMECR1 | NM_015365.1 | 1048 | ctgaccaggtatgtagtgaaaa | 276390 | - | see also 6397 line |
| 42048 | ANGPT4 | NM_015985.2 | 1503 | accacgctccgacaacaagtac | 276401 | - | see also 18551 line |
| 42049 | ANGPT4 | NM_015985.2 | 1094 | ctcatccagtcgccgtgagaattgg | 276398 | - | see also 18551 line |
| 42050 | ANK3 | NM_001149.2 | 2374 | ttcgcatccttagcttacgtaa | 276454 | - | see also 18552 line |
| 42051 | ANK3 | NM_001149.2 | 1425 | tgcagtggttcccggattaagc | 276432 | - | see also 18552 line |
| 42052 | ANKRD2 | NM_020349.2 | 1073 | gaggctcaaccgctacacaaatca | 276775 | - | see also 18553 line |
| 42053 | ANKRD2 | NM_020349.2 | 926 | caccaatgtgagggataagctgc | 276774 | - | see also 18553 line |
| 42054 | ANUBL1 | NM_174890.1 | 515 | tgcgtgtcggacctataaatact | 276791 | - | see also 18554 line |
| 42055 | ANUBL1 | NM_174890.1 | 1002 | accatctgccaccgattgttaa | 276803 | - | see also 18554 line |
| 42056 | AP2A2 | NM_012305.1 | 2891 | ggctcacgctggcacaagtaag | 276868 | - | see also 18555 line |
| 42057 | AP2A2 | NM_012305.1 | 2787 | tcctaatcctgcgaatttcgtgg | 276866 | - | see also 18555 line |
| 42058 | APOA1BP | NM_144772.1 | 780 | taccggctgctaccattaactgg | 276884 | - | see also 18556 line |
| 42059 | APOA1BP | NM_144772.1 | 430 | ggctacgagccaaccatcctatta | 276878 | - | see also 18556 line |
| 42060 | APOAL | NM_024492.2 | 480 | tccaagcctagaggattcattca | 276885 | - | - |
| 42061 | ARFIP1 | NM_014447.1 | 197 | gactctcgtgaacatagctttaa | 276888 | - | see also 18557 line |
| 42062 | ARFIP1 | NM_014447.1 | 834 | atgatgcatatgcactgatttg | 276909 | - | see also 18557 line |
| 42063 | ARPP-21 | NM_016300.3 | 505 | aggcggcctggaggctcagaatca | 276918 | - | see also 6792 line |
| 42064 | ARPP-21 | NM_016300.3 | 451 | aacggcattgttaaatcagaaag | 276917 | - | see also 6792 line |
| 42065 | ARS2 | NM_015908.3 | 1635 | ttcgaccgcagtgttaacattaa | 276982 | - | see also 6566 line |

Figure 46

| | Gene | Accession | | Sequence | | | Note |
|---|---|---|---|---|---|---|---|
| 42066 | ARS2 | NM_015908.3 | 378 | cgccgtagtcggggtgaatatcg | 276971 | - | see also 6566 line |
| 42067 | ASB1 | NM_016114.3 | 128 | ctccgcaggtcgtaatctgaagg | 277003 | - | see also 18560 line |
| 42068 | ASB1 | NM_016114.3 | 1064 | ccccataaagaagtttctactcc | 277012 | - | see also 18560 line |
| 42069 | ASPSCR1 | NM_024083.2 | 400 | ctgaggttgcaggactctttct | 277016 | - | see also 18561 line |
| 42070 | ASPSCR1 | NM_024083.2 | 1370 | gggaacccgagctgtcatttta | 277022 | - | see also 18561 line |
| 42071 | AUTS2 | NM_015570.1 | 2149 | aagacatccaacctatcgatgt | 277042 | - | see also 18562 line |
| 42072 | AUTS2 | NM_015570.1 | 3552 | tccttaccgagaacttgacattc | 277055 | - | see also 18562 line |
| 42073 | B1 | NM_014451.2 | 1928 | tccgcgagttatccaatgtaaat | 277102 | - | see also 18563 line |
| 42074 | B1 | NM_014451.2 | 1599 | aacgttcaatctcgagaactaaa | 277086 | - | see also 18563 line |
| 42075 | B1 | NM_014451.2 | 1763 | ttctcaagcgaccgatgttgaag | 277095 | - | see also 18563 line |
| 42076 | B7 | NM_006992.1 | 630 | tggacccgagaagttgatcagc | 277146 | - | see also 18564 line |
| 42077 | B7 | NM_006992.1 | 309 | ggctggctcatgcttatgtcaag | 277140 | - | see also 18564 line |
| 42078 | B7H3 | NM_025240.1 | 586 | gccaacgagcagggctgtttga | 277147 | - | see also 18565 line |
| 42079 | BAL | NM_031458.1 | 2545 | cagggacatcgttaaatattgt | 277204 | - | see also 9034 line |
| 42080 | BAL | NM_031458.1 | 1814 | aggcccacgcatggatccaaaga | 277190 | - | see also 9034 line |
| 42081 | BBS2 | NM_031885.2 | 651 | gtctgactgcaggcgtattgaac | 277214 | - | see also 18567 line |
| 42082 | BBS2 | NM_031885.2 | 2506 | cactgcttgtcggatgcaattc | 277282 | - | see also 18567 line |
| 42083 | BC-2 | NM_014453.2 | 244 | gacccgcgagcgacagaaactaga | 277287 | - | see also 18568 line |
| 42084 | BC-2 | NM_014453.2 | 364 | cgggcctatgtgcgcaagtttgt | 277290 | - | see also 18568 line |
| 42085 | BCLP | NM_033504.2 | 247 | tacgttgggcaccctcaagatgc | 277297 | - | see also 18569 line |
| 42086 | BCLP | NM_033504.2 | 557 | accccacacgcatttatagctcc | 277299 | - | see also 18569 line |
| 42087 | BENE | NM_005434.3 | 592 | agcgctgccgtcctacaagtaca | 277303 | - | see also 18570 line |
| 42088 | BENE | NM_005434.3 | 414 | agcgcgcacccacatgtataacc | 277301 | - | see also 18570 line |
| 42089 | BICD1 | NM_001714.1 | 378 | atcgaaggaggcttactatctgg | 277309 | - | see also 1141 line |
| 42090 | BICD1 | NM_001714.1 | 2054 | agctagccccatgatttgataaa | 277337 | - | see also 1141 line |
| 42091 | BK65A6.2 | NM_019601.2 | 2441 | ggcggttgcgctgtctatgtgc | 277365 | - | see also 18572 line |
| 42092 | BK65A6.2 | NM_019601.2 | 790 | ttcggatcatcgacagcaaaaat | 277356 | - | see also 18572 line |
| 42093 | BK65A6.2 | NM_019601.2 | 2231 | cccacctcccaacggacaaaagg | 277363 | - | see also 18572 line |
| 42094 | BM-002 | NM_016617.1 | 78 | cccacggtgccgctaacaagtac | 277368 | - | see also 18573 line |
| 42095 | BM-002 | NM_016617.1 | 42 | gtcgaagtttcctttaagatca | 277366 | - | see also 18573 line |
| 42096 | BRD3 | NM_007371.2 | 322 | gcccgtggacgcaatcaaattga | 277378 | - | see also 18575 line |
| 42097 | BRD3 | NM_007371.2 | 506 | gtgctaatggcccaagctttaga | 277385 | - | see also 18575 line |
| 42098 | BRMS1 | NM_015399.2 | 511 | caggaataagtacgaatgtgagc | 277396 | - | see also 18576 line |
| 42099 | BRMS1 | NM_015399.2 | 791 | gaggactgacagccatcaaaaa | 277399 | - | see also 18576 line |
| 42100 | BRP44L | NM_016098.1 | 369 | tgccacgcaacaaatgaagtagc | 277405 | - | see also 18577 line |
| 42101 | BRP44L | NM_016098.1 | 273 | atcagtgggcggatgacatttgc | 277401 | - | see also 18577 line |
| 42102 | BTN2A1 | NM_007049.2 | 953 | tgcagttggcctgcctatcattg | 277408 | - | see also 18578 line |

Figure 46

| 42103 | BTN2A1 | NM_007049.2 | 665 | ttcaatgaggggccatgaagacg | 277407 | - | see also 18578 line | | |
|---|---|---|---|---|---|---|---|---|---|
| 42104 | C11orf1 | NM_022761.1 | 191 | caccactaatgagaataacctatt | 277416 | - | see also 8456 line | | |
| 42105 | C11orf1 | NM_022761.1 | 290 | ttcccagtttggacactactttg | 277419 | - | see also 8456 line | | |
| 42106 | C11orf2 | NM_013265.2 | 925 | tccggctcccgacgtgttagagt | 277425 | - | see also 18580 line | | |
| 42107 | C11orf2 | NM_013265.2 | 928 | ggctcccgacgtgttagagttca | 277426 | - | see also 18580 line | | |
| 42108 | C11orf2 | NM_013265.2 | 405 | tccggaagatgaagaacgatttc | 277424 | - | see also 18580 line | | |
| 42109 | C11ORF4 | NM_020155.2 | 1256 | ctctcggtcctacttctttgacc | 277434 | - | see also 18581 line | | |
| 42110 | C11ORF4 | NM_020155.2 | 1253 | tgcctctcggtcctacttctttg | 277432 | - | see also 18581 line | | |
| 42111 | C14orf1 | NM_007176.1 | 436 | ctggtcgggctccggtatctaga | 277446 | - | see also 4982 line | | |
| 42112 | C14orf1 | NM_007176.1 | 439 | gtcgggctccggtatctagaagt | 277447 | - | see also 4982 line | | |
| 42113 | C14orf122 | NM_016049.3 | 576 | tccgctgggtccctaaggataag | 277455 | - | see also 6641 line | | |
| 42114 | C14orf122 | NM_016049.3 | 575 | ctccgctgggtccctaaggataa | 277454 | - | see also 6641 line | | |
| 42115 | C14orf123 | NM_014169.2 | 1111 | ttgcctagtgtaccttctactca | 277466 | - | see also 18583 line | | |
| 42116 | C14orf123 | NM_014169.2 | 1059 | ggcccaggagttgttaaatgtgg | 277465 | - | see also 18583 line | | |
| 42117 | C15orf2 | NM_018958.1 | 3253 | gccgcctacattcctggtttaga | 277504 | - | see also 18584 line | | |
| 42118 | C15orf2 | NM_018958.1 | 2531 | aggaggagtacatccgattttat | 277495 | - | see also 18584 line | | |
| 42119 | C16orf35 | NM_012075.1 | 1395 | gccggtctccttgtcagaattta | 277528 | - | see also 18585 line | | |
| 42120 | C16orf35 | NM_012075.1 | 686 | cagacccgtcagtgataaactgt | 277517 | - | see also 18585 line | | |
| 42121 | C17orf1A | NM_031456.2 | 911 | tccggtgtatatccgaaatgaat | 277550 | - | see also 18587 line | | |
| 42122 | C17orf1A | NM_031456.2 | 1263 | gtcagcgcacggcttcattcaga | 277558 | - | see also 18587 line | | |
| 42123 | C18orf1 | NM_004338.1 | 692 | acccaaccgaaccatatttgaca | 277606 | - | see also 18588 line | | |
| 42124 | C18orf1 | NM_004338.1 | 341 | ctccacgcggtccttcatcaacc | 277604 | - | see also 18588 line | | |
| 42125 | C18orf2 | NM_031416.1 | 110 | cagacccaactgtcaacagaagc | 277610 | - | see also 18589 line | | |
| 42126 | C18orf2 | NM_031416.1 | 178 | agctaacctaaccactaatgacg | 277614 | - | see also 18589 line | | |
| 42127 | C1orf2 | NM_006589.2 | 1678 | tccgtggactacgttctctttcg | 277630 | - | see also 18590 line | | |
| 42128 | C1orf2 | NM_006589.2 | 1677 | ctccgtggactacgttctctttc | 277629 | - | see also 18590 line | | |
| 42129 | C1orf24 | NM_022083.1 | 2803 | atcaaggtagcccgtattcatga | 277666 | - | see also 18591 line | | |
| 42130 | C1orf24 | NM_022083.1 | 2678 | aagagggctgcaccttaggttct | 277665 | - | see also 18591 line | | |
| 42131 | C20orf10 | NM_014477.2 | 191 | accgtctgagaacggtgttaaaa | 277685 | - | see also 18592 line | | |
| 42132 | C20orf10 | NM_014477.2 | 564 | gacatcgagggatgatagcttga | 277693 | - | see also 18592 line | | |
| 42133 | C21orf33 | NM_004649.4 | 764 | aagctcacgtggaccagaaaaac | 277709 | - | - | molecular_function unknown | - |
| 42134 | C21orf33 | NM_004649.4 | 380 | aaggcgagagcaggaatgttttg | 277702 | - | see also 42133 line | | |
| 42135 | C21orf33 | NM_004649.4 | 414 | gaggatcgcccgtggcaaaatca | 277704 | - | see also 42133 line | | |
| 42136 | C21orf45 | NM_018944.1 | 479 | cagatgcacgcccaagaatcttg | 277714 | - | see also 18593 line | | |
| 42137 | C21orf45 | NM_018944.1 | 463 | atcttggctacgtgtacagatgc | 277713 | - | see also 18593 line | | |
| 42138 | C21orf5 | NM_005128.1 | 4837 | aggtctaacgaccattagtcatt | 277789 | - | see also 18594 line | | |

Figure 46

| 42139 | C21orf5 | NM_005128.1 | 980 | tggttactaggctcagacataaa | 277729 | - | see also 18594 line | | |
|---|---|---|---|---|---|---|---|---|---|
| 42140 | C21orf51 | NM_058182.2 | 228 | ctggggtgaaaatgtatcaaaga | 277833 | - | - | molecular_function unknown | - |
| 42141 | C21orf62 | NM_019596.1 | 341 | tgcggacatccgggattgtgact | 277835 | - | see also 18595 line | | |
| 42142 | C21orf62 | NM_019596.1 | 737 | ctcagccttaaatcatatagtat | 277847 | - | see also 18595 line | | |
| 42143 | C21orf62 | NM_019596.1 | 349 | tccgggattgtgactacagtttg | 277836 | - | see also 18595 line | | |
| 42144 | C22orf23 | NM_032561.2 | 839 | cagaaactccgggaaatggaaga | 277855 | - | see also 18596 line | | |
| 42145 | C22orf23 | NM_032561.2 | 715 | ccctgagctagaccgatttgaag | 277854 | - | see also 18596 line | | |
| 42146 | C2F | NM_006331.3 | 474 | tggcctcatggttcaacttttac | 277864 | - | see also 18597 line | | |
| 42147 | C2F | NM_006331.3 | 233 | cagtcaaggtagggaagacatat | 277858 | - | see also 18597 line | | |
| 42148 | C40 | NM_017546.3 | 1169 | aaccctttagtcgctatagaaat | 277892 | - | see also 7025 line | | |
| 42149 | C40 | NM_017546.3 | 971 | cacgcgatccagtgggataaatc | 277885 | - | see also 7025 line | | |
| 42150 | C6.1A | NM_024332.1 | 234 | atcatcttacgacgttctgataa | 277910 | - | - | molecular_function unknown | - |
| 42151 | C6.1A | NM_024332.1 | 773 | ctcagtaaccaagatccataatg | 277911 | - | see also 42150 line | | |
| 42152 | C6orf11 | NM_005452.4 | 872 | ccgagtaacacggcttgagttcc | 277930 | - | see also 18599 line | | |
| 42153 | C6orf11 | NM_005452.4 | 861 | cgccgctgtgaccgagtaacacg | 277929 | - | see also 18599 line | | |
| 42154 | C6orf15 | NM_014070.1 | 708 | gggaatctggggtatcaataatc | 277945 | - | see also 18600 line | | |
| 42155 | C6orf15 | NM_014070.1 | 527 | gacgactgccccgttctaattca | 277943 | - | see also 18600 line | | |
| 42156 | C6orf4 | NM_147200.1 | 782 | aaccatagacacgggatatgatt | 277959 | - | see also 9816 line | | |
| 42157 | C6orf4 | NM_147200.1 | 1557 | agggataagaccgtgatgataat | 277976 | - | see also 9816 line | | |
| 42158 | C6orf53 | NM_016462.2 | 368 | tgggttttcctagctacatctgg | 277987 | - | see also 18601 line | | |
| 42159 | C6orf54 | NM_014354.1 | 1017 | cgcggctgtacgcgtcatgatag | 277989 | - | see also 18602 line | | |
| 42160 | C6orf67 | NM_018247.1 | 371 | tccgcgagatcgagattgattat | 277994 | - | see also 7344 line | | |
| 42161 | C6orf67 | NM_018247.1 | 373 | cgcgagatcgagattgattatac | 277996 | - | see also 7344 line | | |
| 42162 | C7orf30 | NM_138446.1 | 419 | aacttctacccgacacttacatg | 278047 | - | see also 18604 line | | |
| 42163 | C7orf30 | NM_138446.1 | 276 | tcggacgcggcagatcatactgg | 278045 | - | see also 18604 line | | |
| 42164 | C8orf1 | NM_004337.1 | 934 | tacataacttccgtatcaagact | 278086 | - | see also 2901 line | | |
| 42165 | C8orf1 | NM_004337.1 | 1780 | ctgggtgttacacgctgtttagc | 278118 | - | see also 2901 line | | |
| 42166 | C8orf2 | NM_007175.4 | 389 | cccgaacgcagtgtatgatatag | 278132 | - | see also 18606 line | | |
| 42167 | C8orf2 | NM_007175.4 | 388 | tcccgaacgcagtgtatgatata | 278131 | - | see also 18606 line | | |
| 42168 | C9orf27 | NM_021208.1 | 347 | atgtaatgaacttgctacttatt | 278148 | - | see also 18608 line | | |
| 42169 | C9orf27 | NM_021208.1 | 214 | ctgtactcctcaaagaaagatga | 278146 | - | see also 18608 line | | |
| 42170 | C9orf5 | NM_032012.1 | 1607 | tgcggctaacaacgtgtatcagt | 278170 | - | see also 18609 line | | |
| 42171 | C9orf5 | NM_032012.1 | 2498 | agcaatcatcggtcctattcttc | 278204 | - | see also 18609 line | | |
| 42172 | CDCA1 | NM_031423.2 | 548 | agcatgccgtgaaacgtatatgg | 278227 | - | see also 9017 line | | |
| 42173 | CDCA1 | NM_031423.2 | 440 | gcctatctgccgggtgaatgact | 278220 | - | see also 9017 line | | |

Figure 46

| 42174 | CDCA3 | NM_031299.3 | 468 | tgcctctgggtacccagttatct | 278261 | - | see also 18611 line | | |
|---|---|---|---|---|---|---|---|---|---|
| 42175 | CDCA3 | NM_031299.3 | 315 | ttgcacggacacctatgaagacc | 278257 | - | see also 18611 line | | |
| 42176 | CDKAL1 | NM_017774.1 | 979 | atgacaaatccgccctatatttt | 278297 | - | see also 18612 line | | |
| 42177 | CDKAL1 | NM_017774.1 | 821 | tagatagagccaaacaatctttt | 278290 | - | see also 18612 line | | |
| 42178 | CENPH | NM_022909.3 | 673 | aagatcatacgacaaaacctaca | 278317 | - | see also 18613 line | | |
| 42179 | CENPH | NM_022909.3 | 481 | cacctattagagctaaataaatt | 278315 | - | see also 18613 line | | |
| 42180 | CENPH | NM_022909.3 | 479 | aacacctattagagctaaataaa | 278314 | - | see also 18613 line | | |
| 42181 | CENPJ | NM_018451.2 | 4167 | atccggtcggataagagttaagg | 278396 | - | see also 18614 line | | |
| 42182 | CENPJ | NM_018451.2 | 3423 | tgcgaacacatctgttcgatttc | 278379 | - | see also 18614 line | | |
| 42183 | CER1 | NM_005454.1 | 562 | gggaaatgcgggtctgttcattt | 278405 | - | see also 18615 line | | |
| 42184 | CER1 | NM_005454.1 | 340 | cagccgatagatggaatgaaaat | 278399 | - | see also 18615 line | | |
| 42185 | CFDP1 | NM_006324.1 | 164 | gtgccgtcgggtggagagtatag | 278407 | - | see also 18616 line | | |
| 42186 | CFDP1 | NM_006324.1 | 747 | ctgccgggtcagggttaaaaaga | 278421 | - | see also 18616 line | | |
| 42187 | CG005 | NM_014887.1 | 496 | tccgcagacgatgagatatatag | 278438 | - | see also 18617 line | | |
| 42188 | CG005 | NM_014887.1 | 1238 | atcgatatgtgagtaatggtttc | 278463 | - | see also 18617 line | | |
| 42189 | CGI-51 | NM_015380.3 | 478 | aggtgatgacgcacttccaaatg | 278482 | - | see also 6402 line | | |
| 42190 | CGI-51 | NM_015380.3 | 1468 | cgctcggttggaacttaattact | 278509 | - | see also 6402 line | | |
| 42191 | ch-TOG | NM_014756.1 | 2743 | agggtcgactcaatgattcaaat | 278587 | - | see also 18619 line | | |
| 42192 | ch-TOG | NM_014756.1 | 2742 | aagggtcgactcaatgattcaaa | 278586 | - | see also 18619 line | | |
| 42193 | CHIC2 | NM_012110.2 | 324 | ggcggatttcgacgaaatctatg | 278674 | - | see also 5137 line | | |
| 42194 | CHORDC1 | NM_012124.1 | 176 | cggtctttcacgatgcattaaag | 278688 | - | see also 5146 line | | |
| 42195 | CHORDC1 | NM_012124.1 | 165 | cccaggtgttccggtctttcacg | 278686 | - | see also 5146 line | | |
| 42196 | CHRD | NM_003741.2 | 683 | acccggagcatcgcagttatagc | 278693 | - | see also 18622 line | | |
| 42197 | CHRD | NM_003741.2 | 682 | gacccggagcatcgcagttatag | 278692 | - | see also 18622 line | | |
| 42198 | CISH | NM_013324.4 | 657 | caccaatgtacgcattgagtatg | 278704 | - | - | - | - |
| 42199 | CISH | NM_013324.4 | 885 | agccactgctgtacacctaaaac | 278706 | - | see also 42198 line | | |
| 42200 | CISH | NM_013324.4 | 658 | accaatgtacgcattgagtatgc | 278705 | - | see also 42198 line | | |
| 42201 | COL6A1 | NM_001848.1 | 423 | gccgcgacgcactcaaaagcagc | 278708 | - | see also 18623 line | | |
| 42202 | COL6A1 | NM_001848.1 | 481 | caccgactgcgctatcaagaagg | 278709 | - | see also 18623 line | | |
| 42203 | CP110 | NM_014711.3 | 2953 | agctgccttgtacggtattcatg | 278814 | - | see also 5904 line | | |
| 42204 | CP110 | NM_014711.3 | 1326 | aagtggacatacctatacgaact | 278752 | - | see also 5904 line | | |
| 42205 | CRF | NM_006688.3 | 718 | ggcaagtttacgtgcaacattcc | 278826 | - | see also 4722 line | | |
| 42206 | CRF | NM_006688.3 | 279 | cccggaaggccactatgagatgc | 278824 | - | see also 4722 line | | |
| 42207 | CRISP3 | NM_006061.1 | 543 | ttgggctaatagactatatgtcc | 278837 | - | see also 18625 line | | |
| 42208 | CRISP3 | NM_006061.1 | 278 | aggatcgaatgacaagtctaaaa | 278831 | - | see also 18625 line | | |
| 42209 | CRYPTIC | NM_032545.2 | 255 | tacggtcagtttggcattacaga | 278846 | - | see also 18626 line | | |
| 42210 | CRYPTIC | NM_032545.2 | 267 | ggcattacagatcatcaatttgg | 278847 | - | see also 18626 line | | |

Figure 46

| 42211 | CSDUFD1 | NM_031919.1 | 213 | tactctatactggatgaagtaaa | 278853 | - | see also 9052 line | | |
|---|---|---|---|---|---|---|---|---|---|
| 42212 | CSDUFD1 | NM_031919.1 | 289 | tacagagtcagattagtcaatgt | 278858 | - | see also 9052 line | | |
| 42213 | CSN3 | NM_005212.1 | 263 | aacgtagaccagctatagcaatt | 278871 | - | see also 18628 line | | |
| 42214 | CSN3 | NM_005212.1 | 259 | taccaacgtagaccagctatagc | 278870 | - | see also 18628 line | | |
| 42215 | CSRP2 | NM_001321.1 | 336 | ggcgagaggctgggcatcaaacc | 278882 | - | see also 18629 line | | |
| 42216 | CSRP2 | NM_001321.1 | 235 | ttcacgatgaagagatctactgc | 278881 | - | see also 18629 line | | |
| 42217 | CTAG2 | NM_020994.1 | 448 | gtccggcaacctactgtttatgt | 278884 | - | see also 18631 line | | |
| 42218 | CTAG2 | NM_020994.1 | 654 | gaggtgtcgcctttaatgtgatg | 278889 | - | see also 18631 line | | |
| 42219 | CTDSP2 | NM_005730.2 | 473 | cactgagctcgctgcgtataagg | 278900 | - | see also 18632 line | | |
| 42220 | CTDSP2 | NM_005730.2 | 400 | ctcgtggacgtaacatcttcaag | 278898 | - | see also 18632 line | | |
| 42221 | CTDSPL | NM_005808.1 | 636 | gacggtgcttgactatggaaaga | 278914 | - | see also 18633 line | | |
| 42222 | CTDSPL | NM_005808.1 | 699 | ttcgtttaagcctattagtaatg | 278915 | - | see also 18633 line | | |
| 42223 | CTNND1 | NM_001331.1 | 1392 | cagcgtagtatgggctatgatga | 278932 | - | see also 884 line | | |
| 42224 | CTNND1 | NM_001331.1 | 2603 | ctggacgtatggtcgatacatcc | 278966 | - | see also 884 line | | |
| 42225 | CUL4B | NM_003588.2 | 945 | caccgtctctagctttgctaaca | 278999 | - | see also 2381 line | | |
| 42226 | CUL4B | NM_003588.2 | 2454 | gggctattggccgacatatgtgc | 279055 | - | see also 2381 line | | |
| 42227 | CXorf1 | NM_004709.1 | 562 | ggccctgccaagaacatatctgt | 279078 | - | see also 18636 line | | |
| 42228 | CXorf1 | NM_004709.1 | 370 | agcatccccactgactatattct | 279070 | - | see also 18636 line | | |
| 42229 | CXorf12 | NM_003492.1 | 851 | ctcggctacctacttagctttgg | 279083 | - | - | molecular_function unknown | - |
| 42230 | CXorf12 | NM_003492.1 | 846 | accacctcggctacctacttagc | 279082 | - | see also 42229 line | | |
| 42231 | CXorf12 | NM_003492.1 | 347 | ggctgtgttcacgggcattttcg | 279080 | - | see also 42229 line | | |
| 42232 | CXorf2 | NM_001586.1 | 267 | aggcctagcccacaacttacagg | 279084 | - | see also 18637 line | | |
| 42233 | CXorf2 | NM_001586.1 | 923 | agggacagctgaatgagatttac | 279088 | - | see also 18637 line | | |
| 42234 | CXorf6 | NM_005491.1 | 2084 | gacgtgtcaccgtctaacattac | 279131 | - | see also 18638 line | | |
| 42235 | CXorf6 | NM_005491.1 | 2087 | gtgtcaccgtctaacattactca | 279133 | - | see also 18638 line | | |
| 42236 | CXX1 | NM_003928.1 | 396 | gggagggtccgctgtgttactgg | 279138 | - | see also 18639 line | | |
| 42237 | CYYR1 | NM_052954.1 | 251 | ctgcaattgcgggcattgttttt | 279140 | - | see also 18640 line | | |
| 42238 | CYYR1 | NM_052954.1 | 424 | gaccacgagatggaatactgtgc | 279142 | - | see also 18640 line | | |
| 42239 | CYYR1 | NM_052954.1 | 252 | tgcaattgcgggcattgtttttg | 279141 | - | see also 18640 line | | |
| 42240 | DGCR14 | NM_022719.1 | 977 | aacacacccttgagagttgaagg | 279145 | - | - | - | - |
| 42241 | DGCR14 | NM_022719.1 | 439 | gccgctgcccagcctagatgtct | 279143 | - | see also 42240 line | | |
| 42242 | DGCR14 | NM_022719.1 | 884 | tcccctcgagtgggtggatttgg | 279144 | - | see also 42240 line | | |
| 42243 | DIPA | NM_006848.1 | 450 | gcctggcgctgggcgaagaatgg | 279147 | - | see also 18641 line | | |
| 42244 | DISC1 | NM_018662.1 | 1666 | agccaccggaaaccataaggagc | 279160 | - | see also 18642 line | | |
| 42245 | DISC1 | NM_018662.1 | 116 | cgcaggcagccgggattgcttac | 279149 | - | see also 18642 line | | |
| 42246 | DISC1 | NM_018662.1 | 1175 | gacgttacaacaaagattagaag | 279158 | - | see also 18642 line | | |

EP 1 752 536 A1

Figure 46

<table>
<tr><td>42247</td><td>DKFZP434F091</td><td>NM_015453.1</td><td>1647</td><td>caggccgagctgtactacttact</td><td>279208</td><td>-</td><td>see also 18643 line</td></tr>
<tr><td>42248</td><td>DKFZP434F091</td><td>NM_015453.1</td><td>537</td><td>gagaccgtggcaagatatatttt</td><td>279181</td><td>-</td><td>see also 18643 line</td></tr>
<tr><td>42249</td><td>DKFZP434G1415</td><td>NM_031292.1</td><td>981</td><td>aagcgattggttttttagctatc</td><td>279231</td><td>-</td><td>see also 18644 line</td></tr>
<tr><td>42250</td><td>DKFZP434G1415</td><td>NM_031292.1</td><td>2026</td><td>aacgaaaggttcccacattgatg</td><td>279264</td><td>-</td><td>see also 18644 line</td></tr>
<tr><td>42251</td><td>DKFZP564B167</td><td>NM_015415.1</td><td>363</td><td>cagctcaatctgctgttttgatg</td><td>279271</td><td>-</td><td>see also 18645 line</td></tr>
<tr><td>42252</td><td>DKFZP564B167</td><td>NM_015415.1</td><td>477</td><td>gagcctctcagctttttcgtatt</td><td>279277</td><td>-</td><td>see also 18645 line</td></tr>
<tr><td>42253</td><td>DKFZP564C186</td><td>NM_015658.1</td><td>97</td><td>tccgaatccgagtccgaaaattc</td><td>279278</td><td>-</td><td>see also 18646 line</td></tr>
<tr><td>42254</td><td>DKFZP564C186</td><td>NM_015658.1</td><td>98</td><td>ccgaatccgagtccgaaaattct</td><td>279279</td><td>-</td><td>see also 18646 line</td></tr>
<tr><td>42255</td><td>DKFZP564C186</td><td>NM_015658.1</td><td>609</td><td>cacggacagtgctgcattcaatg</td><td>279281</td><td>-</td><td>see also 18646 line</td></tr>
<tr><td>42256</td><td>DKK4</td><td>NM_014420.1</td><td>743</td><td>atcggcagcatgctcgattaaga</td><td>279292</td><td>-</td><td>see also 18647 line</td></tr>
<tr><td>42257</td><td>DKK4</td><td>NM_014420.1</td><td>749</td><td>agcatgctcgattaagagtatgc</td><td>279295</td><td>-</td><td>see also 18647 line</td></tr>
<tr><td>42258</td><td>DLG7</td><td>NM_014750.3</td><td>1938</td><td>atcgcctagctgccataaaaaat</td><td>279341</td><td>-</td><td>see also 5943 line</td></tr>
<tr><td>42259</td><td>DLG7</td><td>NM_014750.3</td><td>1815</td><td>ctgggtggcaagtcaataataat</td><td>279338</td><td>-</td><td>see also 5943 line</td></tr>
<tr><td>42260</td><td>DLG7</td><td>NM_014750.3</td><td>577</td><td>gggtcgttatagacctgatatgc</td><td>279309</td><td>-</td><td>see also 5943 line</td></tr>
<tr><td>42261</td><td>DLGAP1</td><td>NM_004746.2</td><td>1711</td><td>aactaccacgaaacctttcattt</td><td>279384</td><td>-</td><td>see also 18649 line</td></tr>
<tr><td>42262</td><td>DLGAP1</td><td>NM_004746.2</td><td>1049</td><td>tacaccccacgaggtaaagatga</td><td>279372</td><td>-</td><td>see also 18649 line</td></tr>
<tr><td>42263</td><td>DOC1</td><td>NM_014890.1</td><td>1345</td><td>ctctagaacgaaggtatgctaat</td><td>279426</td><td>-</td><td>see also 18650 line</td></tr>
<tr><td>42264</td><td>DOC1</td><td>NM_014890.1</td><td>1014</td><td>aagtccaaaaccgatgtagaaga</td><td>279420</td><td>-</td><td>see also 18650 line</td></tr>
<tr><td>42265</td><td>DONSON</td><td>NM_017613.2</td><td>381</td><td>cggtcccgtttttagattctaat</td><td>279470</td><td>-</td><td>see also 7054 line</td></tr>
<tr><td>42266</td><td>DONSON</td><td>NM_017613.2</td><td>378</td><td>ccccggtcccgtttttagattct</td><td>279468</td><td>-</td><td>see also 7054 line</td></tr>
<tr><td>42267</td><td>DP1</td><td>NM_005669.3</td><td>321</td><td>ctgaattcttctctgatatcttc</td><td>279507</td><td>-</td><td>see also 18652 line</td></tr>
<tr><td>42268</td><td>DSCR10</td><td>NM_148676.1</td><td>548</td><td>ttctctgctgagtctggttatgg</td><td>279508</td><td>-</td><td>-    -    -</td></tr>
<tr><td>42269</td><td>DSCR3</td><td>NM_006052.1</td><td>560</td><td>gacgtatcatggcgtgtttgtca</td><td>279518</td><td>-</td><td>see also 4213 line</td></tr>
<tr><td>42270</td><td>DSCR3</td><td>NM_006052.1</td><td>699</td><td>cccgtggacttcacgattacacc</td><td>279522</td><td>-</td><td>see also 4213 line</td></tr>
<tr><td>42271</td><td>DSCR4</td><td>NM_005867.1</td><td>118</td><td>atcttgacgagagatgatgaacc</td><td>279531</td><td>-</td><td>see also 18654 line</td></tr>
<tr><td>42272</td><td>DSCR4</td><td>NM_005867.1</td><td>115</td><td>atcatcttgacgagagatgatga</td><td>279530</td><td>-</td><td>see also 18654 line</td></tr>
<tr><td>42273</td><td>DSCR6</td><td>NM_018962.1</td><td>460</td><td>cccatgtcaaagcgtcaagaata</td><td>279539</td><td>-</td><td>see also 18655 line</td></tr>
<tr><td>42274</td><td>DSCR6</td><td>NM_018962.1</td><td>465</td><td>gtcaaagcgtcaagaatacctgc</td><td>279540</td><td>-</td><td>see also 18655 line</td></tr>
<tr><td>42275</td><td>DSCR8</td><td>NM_032589.1</td><td>382</td><td>aagtgctggcccagtatgagtcc</td><td>279543</td><td>-</td><td>see also 18656 line</td></tr>
</table>

Figure 46

| | | | | | | | | |
|---|---|---|---|---|---|---|---|---|
| 42276 | DSCR9 | NM_148675.1 | 921 | gcccatgtcgcgatgtaactgc | - | 279546 | - | see also 18657 line |
| 42277 | DSCR9 | NM_148675.1 | 981 | gccgtcctccacgctgatgctagg | - | 279547 | - | see also 18657 line |
| 42278 | DXS9879E | NM_006014.2 | 696 | ttccgtcatcaactttcttgacc | - | 279550 | - | - |
| 42279 | DXS9879E | NM_006014.2 | 689 | tccgaatttccgtcatcaacttt | - | 279549 | - | see also 42278 line |
| 42280 | E46L | NM_013236.1 | 1003 | ttgcggcatgctgagttgattgc | - | 279585 | - | see also 5355 line |
| 42281 | E46L | NM_013236.1 | 280 | gcgctcacggcgctcttcaaaga | - | 279552 | - | see also 5355 line |
| 42282 | EAT2 | NM_053282.1 | 372 | gggatgtgttcaccttttaaa | - | 279605 | - | see also 9494 line |
| 42283 | EAT2 | NM_053282.1 | 326 | tccaagcctaaaggaactgatct | - | 279603 | - | see also 9494 line |
| 42284 | EAT2 | NM_053282.1 | 224 | ctgtgtcgtttaaaaatatg | - | 279600 | - | see also 9494 line |
| 42285 | EML4 | NM_019063.2 | 928 | tgcgcggtcggccaattaccatg | - | 279630 | - | see also 18660 line |
| 42286 | EML4 | NM_019063.2 | 1551 | agcggcaattcactaacaagaaa | - | 279659 | - | see also 18660 line |
| 42287 | EML4 | NM_019063.2 | 929 | gcgcggtcggccaattaccatgt | - | 279631 | - | see also 18660 line |
| 42288 | EPB41L4A | NM_022140.2 | 1963 | atccggattgtccgaagaacaat | - | 279750 | - | see also 8303 line |
| 42289 | EPB41L4A | NM_022140.2 | 1894 | ttcgagaagcccgatatccaag | - | 279747 | - | see also 8303 line |
| 42290 | ERH | NM_004450.1 | 211 | gtccctcatcacatatgacatc | - | 279754 | - | see also 18662 line |
| 42291 | ERH | NM_004450.1 | 204 | cccaacagtccctcatcacata | - | 279751 | - | see also 18662 line |
| 42292 | ERH | NM_004450.1 | 298 | cccagacataccagccttataac | - | 279756 | - | see also 18662 line |
| 42293 | ET | NM_024311.2 | 1152 | agcaagaacaatcgtttggtag | - | 279782 | - | see also 8609 line |
| 42294 | ET | NM_024311.2 | 1450 | cagccgtggcattttctacagc | - | 279795 | - | see also 8609 line |
| 42295 | FAF1 | NM_007051.2 | 905 | aagacagtacggtcctaaaatct | - | 279809 | - | see also 4936 line |
| 42296 | FAM3A | NM_021806.1 | 665 | gtcaaacgacctgttgaagtttat | - | 279845 | - | see also 18664 line |
| 42297 | FAM3A | NM_021806.1 | 429 | cgccacggccaggaagtacaag | - | 279842 | - | see also 18664 line |
| 42298 | FANCD2 | NM_033084.2 | 1752 | ctctagcaccgtattcaagtaca | - | 279899 | - | see also 18665 line |
| 42299 | FANCD2 | NM_033084.2 | 863 | tggataagttgtcgtcattaga | - | 279865 | - | see also 18665 line |
| 42300 | FANCE | NM_021922.1 | 1125 | cagcttcttcacgaatgtagtcc | - | 279968 | - | see also 18666 line |
| 42301 | FANCE | NM_021922.1 | 1320 | gcctcccgcctgcttacaactgc | - | 279970 | - | see also 18666 line |
| 42302 | FANCF | NM_022725.1 | 1074 | ttcgtagttggtgcatttaggaaa | - | 279989 | - | see also 18667 line |
| 42303 | FANCF | NM_022725.1 | 1071 | ctcttcgttagtggtgcatttagg | - | 279988 | - | see also 18667 line |
| 42304 | FETUB | NM_014375.1 | 614 | caccgagtctcttgcgaaataca | - | 280005 | - | see also 18668 line |
| 42305 | FETUB | NM_014375.1 | 303 | aacgacgcccaggaatacagacg | - | 279994 | - | see also 18668 line |
| 42306 | FFZ2 | NM_005102.1 | 503 | cacagtcagatcggctttcaatg | - | 280024 | - | see also 18669 line |
| 42307 | FFZ2 | NM_005102.1 | 891 | accgtctgttgaagatcttcaaa | - | 280033 | - | see also 18669 line |
| 42308 | FHL1 | NM_001449.3 | 984 | tggccaacaagcgctttgtttc | - | 280048 | - | see also 18670 line |
| 42309 | FHL1 | NM_001449.3 | 983 | ctggccaacaagcgctttgttt | - | 280047 | - | see also 18670 line |
| 42310 | FHL1 | NM_001449.3 | 1007 | caccaggagcaagtgttattgtcc | - | 280049 | - | see also 18670 line |
| 42311 | FKBPL | NM_022110.3 | 271 | atggagagcccaccagtcaatac | - | 280051 | - | see also 18671 line |
| 42312 | FKBPL | NM_022110.3 | 732 | aacttggggggagtcatagaga | - | 280059 | - | see also 18671 line |

Figure 46

| | | | | | | | | | | |
|---|---|---|---|---|---|---|---|---|---|---|
| 42313 | FKSG2 | NM_021631.1 | 190 | agcacgataatcactagtgttga | 280066 | - | see also 18672 line | | | |
| 42314 | FKSG2 | NM_021631.1 | 451 | taccgtaaggatggtcagtatga | 280070 | - | see also 18672 line | | | |
| 42315 | FLJ10525 | NM_018126.1 | 935 | acccatattgtcggaccttattt | 280098 | - | see also 7269 line | | | |
| 42316 | FLJ10525 | NM_018126.1 | 936 | cccatattgtcggaccttattta | 280099 | - | see also 7269 line | | | |
| 42317 | FLJ10525 | NM_018126.1 | 905 | ttcttacccttcgatattcgtct | 280096 | - | see also 7269 line | | | |
| 42318 | FLJ10769 | NM_018210.1 | 122 | aagagcgttttcgctacgtaaag | 280111 | - | see also 18674 line | | | |
| 42319 | FLJ10769 | NM_018210.1 | 136 | tacgtaaagcacattcgataaag | 280113 | - | see also 18674 line | | | |
| 42320 | FLJ10769 | NM_018210.1 | 126 | gcgttttcgctacgtaaagcaca | 280112 | - | see also 18674 line | | | |
| 42321 | FLJ11294 | NM_018383.2 | 3430 | cccgaggacccacgttttcgagg | 280193 | - | see also 7429 line | | | |
| 42322 | FLJ11294 | NM_018383.2 | 3235 | ggccaccggttacgtgaatttga | 280192 | - | see also 7429 line | | | |
| 42323 | FLJ11749 | NM_024591.2 | 288 | ggccatggttcagagtattgagt | 280202 | - | see also 18676 line | | | |
| 42324 | FLJ11749 | NM_024591.2 | 312 | cacccagatcgaaatgaaagtga | 280205 | - | see also 18676 line | | | |
| 42325 | FLJ13448 | NM_025147.2 | 447 | ctgtgttggagcgatatacatca | 280223 | - | see also 18677 line | | | |
| 42326 | FLJ13448 | NM_025147.2 | 143 | cccgtgcggaatggcagatattt | 280209 | - | see also 18677 line | | | |
| 42327 | FLJ14007 | NM_024699.1 | 467 | tgctgatggcgataagtcattac | 280247 | - | see also 18678 line | | | |
| 42328 | FLJ14007 | NM_024699.1 | 56 | ttgccggcagcgagattttcttc | 280237 | - | see also 18678 line | | | |
| 42329 | FLJ20485 | NM_019042.2 | 1915 | taccgaaagatcattattcgtcc | 280276 | - | see also 18679 line | | | |
| 42330 | FLJ20485 | NM_019042.2 | 1607 | atcgcttaatgtatattcatagc | 280256 | - | see also 18679 line | | | |
| 42331 | FLJ21128 | NM_025083.2 | 1567 | acgcaggccgtatctatttgtgc | 280313 | - | see also 18680 line | | | |
| 42332 | FLJ21128 | NM_025083.2 | 983 | tggcctggttgtcccaagtattt | 280303 | - | see also 18680 line | | | |
| 42333 | FLJ21827 | NM_020153.2 | 605 | ctcaacggggctttagtgaaaat | 280318 | - | see also 7771 line | | | |
| 42334 | FLJ21827 | NM_020153.2 | 603 | acctcaacggggctttagtgaaa | 280317 | - | see also 7771 line | | | |
| 42335 | FLJ21827 | NM_020153.2 | 850 | atcccagctgtcggggatattga | 280320 | - | see also 7771 line | | | |
| 42336 | FLJ32452 | NM_144576.2 | 432 | ggcttactcggagcgtagaatca | 280333 | - | see also 18682 line | | | |
| 42337 | FLJ32452 | NM_144576.2 | 447 | tagaatcatggggtactcaatgc | 280334 | - | see also 18682 line | | | |
| 42338 | FN5 | NM_020179.1 | 344 | ggccgtggtcgtgctcttgatcg | 280347 | - | - | - | - |
| 42339 | FSD1 | NM_024333.1 | 1224 | tggtgcctgcacgtcaacaattg | 280358 | - | see also 18683 line | | | |
| 42340 | FSD1 | NM_024333.1 | 438 | gccttccggctatcattgaaagc | 280351 | - | see also 18683 line | | | |
| 42341 | GAGEC1 | NM_007003.2 | 232 | acctccgatcgaagaacgtaaag | 280363 | - | see also 18684 line | | | |
| 42342 | GAGEC1 | NM_007003.2 | 235 | tccgatcgaagaacgtaaagtag | 280364 | - | see also 18684 line | | | |
| 42343 | GAS8 | NM_001481.1 | 720 | ctcgacttgcggagaaagactga | 280376 | - | see also 1020 line | | | |
| 42344 | GAS8 | NM_001481.1 | 718 | aactcgacttgcggagaaagact | 280375 | - | see also 1020 line | | | |
| 42345 | GCN5L1 | NM_001487.1 | 104 | gtcccgcctcctaaaagaacacc | 280383 | - | - | molecular_function unknown | - |
| 42346 | GCN5L1 | NM_001487.1 | 422 | tgccactgcactggaatatgtct | 280385 | - | see also 42345 line | | | |
| 42347 | GCN5L1 | NM_001487.1 | 242 | ggcctacatgaaccagagaaagc | 280384 | - | see also 42345 line | | | |
| 42348 | GKN1 | NM_019617.2 | 536 | tgctacacgaccagtgtactatg | 280403 | - | see also 18687 line | | | |

Figure 46

| | | | | | | | |
|---|---|---|---|---|---|---|---|
| 42349 | GKN1 | NM_019617.2 | 541 | cacgaccagtgtactatggatg | 280404 | - | see also 18687 line |
| 42350 | GLTSCR2 | NM_015710.2 | 165 | ggcgaggcccaagaaataagaag | 280407 | - | see also 18688 line |
| 42351 | GLTSCR2 | NM_015710.2 | 163 | gcggcgaggcccaagaaataaga | 280406 | - | see also 18688 line |
| 42352 | GPM6B | NM_005278.2 | 543 | agcggtgcccgtgtttatgttct | 280421 | - | see also 3703 line |
| 42353 | GPM6B | NM_005278.2 | 717 | ctgcaacacaaacgagttctaca | 280426 | - | see also 3703 line |
| 42354 | GPR155 | NM_152529.3 | 1301 | ctgctccatcatgtacgtttct | 280481 | - | see also 18690 line |
| 42355 | GPR155 | NM_152529.3 | 865 | ctcggactcctgcgtgtattaca | 280463 | - | see also 18690 line |
| 42356 | GREB1 | NM_014668.2 | 1184 | cgcgaccatcggctttaggtatc | 280544 | - | see also 5843 line |
| 42357 | GREB1 | NM_014668.2 | 344 | aagaggttgcacaattccatc | 280532 | - | see also 5843 line |
| 42358 | GTSE1 | NM_016426.3 | 1616 | accgatgaccccaaaacgatgc | 280627 | - | see also 18693 line |
| 42359 | GTSE1 | NM_016426.3 | 1179 | gccaagcgggtcgatgttctga | 280626 | - | see also 18693 line |
| 42360 | H-plk | NM_015852.1 | 2485 | tcctcaaaacttagtacagtaa | 280641 | - | see also 18694 line |
| 42361 | H-plk | NM_015852.1 | 2487 | ctcaaaacttagtacatgtaaga | 280642 | - | see also 18694 line |
| 42362 | H41 | NM_017548.2 | 615 | ctcagggttcaggcaatgcaaat | 280643 | - | see also 18695 line |
| 42363 | H41 | NM_017548.2 | 673 | gacaagatccaggtgataactgg | 280644 | - | see also 18695 line |
| 42364 | HBLD1 | NM_194279.1 | 287 | aacccgacgacagggtatttga | 280652 | - | see also 18696 line |
| 42365 | HBLD1 | NM_194279.1 | 240 | gtggatgctccgattccaatac | 280646 | - | see also 18696 line |
| 42366 | HCG9 | NM_005844.2 | 155 | ctcctggtcccgaaaaaaggagg | 280655 | - | see also 18697 line |
| 42367 | HERPUD1 | NM_014685.1 | 628 | cacgacagtactacatgcaatat | 280665 | - | see also 18698 line |
| 42368 | HERPUD1 | NM_014685.1 | 1061 | tctctctctgacgttgtaaatc | 280675 | - | see also 18698 line |
| 42369 | HHLA2 | NM_007072.2 | 579 | gggatccgaagtcgtaatacact | 280685 | - | see also 18699 line |
| 42370 | HHLA2 | NM_007072.2 | 576 | gagggatccgaagtcgtaatac | 280683 | - | see also 18699 line |
| 42371 | HRASLS3 | NM_007069.1 | 859 | gagtcatgttctcaagaaacaag | 280722 | - | see also 18700 line |
| 42372 | HRASLS3 | NM_007069.1 | 857 | tggagtcatgttctcaagaaaca | 280721 | - | see also 18700 line |
| 42373 | HSA272196 | NM_018405.2 | 524 | gcctccactgataccctattatt | 280728 | - | see also 18701 line |
| 42374 | HSA272196 | NM_018405.2 | 529 | cactgataccctattattccaag | 280729 | - | see also 18701 line |
| 42375 | HSA272196 | NM_018405.2 | 516 | caccatccgctccactgatacc | 280727 | - | see also 18701 line |
| 42376 | HSHIN1 | NM_017493.4 | 81 | agcgtttatagaaggatcatttg | 280730 | - | see also 18702 line |
| 42377 | HSHIN1 | NM_017493.4 | 245 | ttcctgaaaaggtgttactgtgt | 280731 | - | see also 18702 line |
| 42378 | HSPC117 | NM_014306.2 | 1410 | atctcgacgtaatttagatttcc | 280765 | - | see also 5612 line |
| 42379 | HSPC121 | NM_016395.1 | 191 | gcgcaccgcggagctatatctgc | 280774 | - | see also 18704 line |
| 42380 | HSPC121 | NM_016395.1 | 969 | ctcacatggcttcgttacactct | 280792 | - | see also 18704 line |
| 42381 | HSPC148 | NM_016403.1 | 698 | atgacaactgcgatctgaattt | 280815 | - | see also 18705 line |
| 42382 | HSPC148 | NM_016403.1 | 210 | tgcccctgaagaggttcgtaacc | 280805 | - | see also 18705 line |

Figure 46

| 42383 | HSPC177 | NM_016410.2 | 660 | aaggtgttcccactgatacaaaa | 280820 | - | see also 18706 line |
|---|---|---|---|---|---|---|---|
| 42384 | HSPC177 | NM_016410.2 | 452 | atcgaccagattgaggatttaca | 280819 | - | see also 18706 line |
| 42385 | HSRG1 | NM_014940.2 | 1892 | ttcgttacccacccaagtactcc | 280834 | - | see also 18707 line |
| 42386 | HSRG1 | NM_014940.2 | 728 | cggctaccatgggtgtaatgacc | 280824 | - | see also 18707 line |
| 42387 | IFI27 | NM_005532.1 | 351 | ctccggattgaccaagttcatcc | 280837 | - | see also 18708 line |
| 42388 | IFI27 | NM_005532.1 | 147 | caggattgctacagttgtgattg | 280836 | - | see also 18708 line |
| 42389 | IFIT1 | NM_001548.1 | 67 | gagtacaaatggtgatgatcatc | 280838 | - | see also 18709 line |
| 42390 | IFIT1 | NM_001548.1 | 786 | aagaagctctagccaacatgtcc | 280847 | - | see also 18709 line |
| 42391 | IFIT4 | NM_001549.2 | 1090 | ttcctggagacggaatgttatca | 280869 | - | see also 18710 line |
| 42392 | IFIT4 | NM_001549.2 | 238 | tacaacttgttggcctacataaa | 280854 | - | see also 18710 line |
| 42393 | IFIT5 | NM_012420.1 | 646 | aacatcggctatgctatcacagt | 280890 | - | see also 18711 line |
| 42394 | IFIT5 | NM_012420.1 | 1433 | gtgccctagggtttgtttacaag | 280911 | - | see also 18711 line |
| 42395 | IFRD2 | NM_006764.2 | 617 | cccgcagcaccgccagtgaatgc | 280919 | - | see also 18712 line |
| 42396 | IFRD2 | NM_006764.2 | 1330 | atcgctgccggtgaaaccattgc | 280920 | - | see also 18712 line |
| 42397 | IMPACT | NM_018439.1 | 281 | aacgtgcggatttatcaaatagc | 280931 | - | see also 7479 line |
| 42398 | IMPACT | NM_018439.1 | 278 | aagaacgtgcggatttatcaaat | 280930 | - | see also 7479 line |
| 42399 | JPH1 | NM_020647.1 | 1297 | aggccctgattacgtcaaacaga | 280956 | - | see also 7905 line |
| 42400 | JPH1 | NM_020647.1 | 289 | gggggagtggtcacatggtttca | 280945 | - | see also 7905 line |
| 42401 | KERA | NM_007035.2 | 1333 | gtggcctttttgagactaaatca | 280980 | - | see also 18715 line |
| 42402 | KERA | NM_007035.2 | 1564 | ttcagttatggacctcatcttcg | 280986 | - | see also 18715 line |
| 42403 | KERA | NM_007035.2 | 1588 | tacctccgtctggatggaaatga | 280987 | - | see also 18715 line |
| 42404 | KIAA0174 | NM_014761.1 | 850 | caggcctttcccaatattcatcc | 281005 | - | see also 18716 line |
| 42405 | KIAA0174 | NM_014761.1 | 629 | ctggggtagagacagatcttatt | 281000 | - | see also 18716 line |
| 42406 | KIAA0186 | NM_021067.1 | 540 | aagtccggtgtctaaaagactat | 281020 | - | see also 18717 line |
| 42407 | KIAA0186 | NM_021067.1 | 542 | gtccggtgtctaaaagactatgg | 281021 | - | see also 18717 line |
| 42408 | KIAA0186 | NM_021067.1 | 255 | gacgaagtgatttgataccaact | 281011 | - | see also 18717 line |
| 42409 | KIAA0285 | NM_014807.2 | 1624 | ccccgtatagacggcaaattaga | 281030 | - | see also 18718 line |
| 42410 | KIAA0285 | NM_014807.2 | 2062 | gagacaacccgttcggatatttc | 281033 | - | see also 18718 line |
| 42411 | KIAA0905 | NM_014933.1 | 2259 | gccatggacactagtactgtagg | 281100 | - | see also 18719 line |
| 42412 | KIAA0905 | NM_014933.1 | 959 | cacacagtggtgcttcgatattc | 281066 | - | see also 18719 line |
| 42413 | KIAA0905 | NM_014933.1 | 1170 | cagactgctcagcatagtatagt | 281077 | - | see also 18719 line |
| 42414 | KIAA1173 | NM_020707.2 | 1009 | ctcgctggcctagcctattcaaa | 281138 | - | see also 18720 line |
| 42415 | KIAA1173 | NM_020707.2 | 357 | ctcctttcgcaacgtcatcatct | 281133 | - | see also 18720 line |
| 42416 | KREMEN1 | NM_032045.3 | 906 | tggacttcgtcatcttgtatttc | 281168 | - | see also 9071 line |
| 42417 | KTN1 | NM_004986.1 | 1960 | aacgtgatcgtttaacaagtaaa | 281220 | - | see also 3481 line |
| 42418 | KTN1 | NM_004986.1 | 1432 | aactacgccaggattatgctagg | 281205 | - | see also 3481 line |
| 42419 | LENG1 | NM_024316.1 | 735 | ggcggtacaactcccaattcaac | 281276 | - | see also 18723 line |

Figure 46

| 42420 | LENG1 | NM_024316.1 | 138 | agcaagaggcccgtacagaattc | 281273 | - | see also 18723 line |
|---|---|---|---|---|---|---|---|
| 42421 | LENG4 | NM_024298.2 | 1459 | gtggctggcgcagtatatctaca | 281281 | - | see also 18724 line |
| 42422 | LENG4 | NM_024298.2 | 1464 | tggcgcagtatatctacaagagc | 281283 | - | see also 18724 line |
| 42423 | LGP1 | NM_032484.3 | 1615 | gtgcgaggggaagatattggtga | 281293 | - | see also 18725 line |
| 42424 | LGP1 | NM_032484.3 | 1749 | tccccactacgaggtgtttgtgg | 281294 | - | see also 18725 line |
| 42425 | Lin10 | NM_025187.3 | 344 | gactcaggacgggatcaaactaa | 281300 | - | see also 8875 line |
| 42426 | Lin10 | NM_025187.3 | 346 | ctcaggacgggatcaaactaatg | 281301 | - | see also 8875 line |
| 42427 | LLGL1 | NM_004140.2 | 65 | aggagcttttcgccttcaacaag | 281325 | - | see also 18727 line |
| 42428 | LLGL1 | NM_004140.2 | 310 | tgggagattgtccaccataatgg | 281329 | - | see also 18727 line |
| 42429 | LMCD1 | NM_014583.2 | 1036 | tgctccggctgcgatgagataat | 281357 | - | see also 5763 line |
| 42430 | LMCD1 | NM_014583.2 | 1039 | tccggctgcgatgagataatatt | 281359 | - | see also 5763 line |
| 42431 | LMCD1 | NM_014583.2 | 1038 | ctccggctgcgatgagataatat | 281358 | - | see also 5763 line |
| 42432 | LMNB2 | NM_032737.2 | 1767 | ctcggtgatgcgtgagaatgaga | 281367 | - | see also 18729 line |
| 42433 | LMNB2 | NM_032737.2 | 1556 | gggaggagatcgcctacaagttc | 281364 | - | see also 18729 line |
| 42434 | LMO6 | NM_006150.3 | 1088 | gccacgccgaggcctaatcttct | 281373 | - | see also 18730 line |
| 42435 | LMO6 | NM_006150.3 | 1087 | tgccacgccgaggcctaatcttc | 281372 | - | see also 18730 line |
| 42436 | LOC130617 | NM_138802.1 | 519 | aacgctgtagccgaaacttctgc | 281392 | - | see also 9640 line |
| 42437 | LOC130617 | NM_138802.1 | 438 | cacagcaaaaacgtaagatcttc | 281385 | - | see also 9640 line |
| 42438 | LOC55974 | NM_018845.1 | 291 | tgcgcttcagaccctgtatatct | 281396 | - | see also 18732 line |
| 42439 | LOC55974 | NM_018845.1 | 639 | aggaatcgtcaccagctttatcc | 281405 | - | see also 18732 line |
| 42440 | LOC91614 | NM_139160.1 | 631 | agcgcttatggactacaaagtat | 281413 | - | see also 18733 line |
| 42441 | LOC91614 | NM_139160.1 | 1204 | accagaccgaacagacttagtga | 281429 | - | see also 18733 line |
| 42442 | LPIN1 | NM_145693.1 | 2656 | ttcggataccttcagtaacttca | 281505 | - | see also 18734 line |
| 42443 | LPIN1 | NM_145693.1 | 992 | gccattcacagcgaatcttcaga | 281475 | - | see also 18734 line |
| 42444 | LPP | NM_005578.1 | 385 | ccggtagttgctccaaaacctaa | 281507 | - | see also 3879 line |
| 42445 | LPP | NM_005578.1 | 1714 | gccaccgggaaggcctatcatcc | 281523 | - | see also 3879 line |
| 42446 | LRBA | NM_006726.1 | 2169 | atggaccgcgacctaatcaaaaa | 281595 | - | see also 4735 line |
| 42447 | LRBA | NM_006726.1 | 2170 | tggaccgcgacctaatcaaaaag | 281596 | - | see also 4735 line |
| 42448 | LRG | NM_052972.1 | 635 | gaggggtccgctgcaattagaac | 281796 | - | see also 18737 line |
| 42449 | LRG | NM_052972.1 | 928 | acctgagcgacctctatcgttgg | 281800 | - | see also 18737 line |
| 42450 | LRPPRC | NM_133259.2 | 862 | cacatacctcgcattattgaatg | 281821 | - | see also 9557 line |
| 42451 | LRPPRC | NM_133259.2 | 3384 | gcctcatcataacgcaagttagg | 281899 | - | see also 9557 line |
| 42452 | LY6G5B | NM_021221.1 | 1254 | tacttgttcctcaatagttcagg | 281922 | - | see also 18739 line |
| 42453 | LY6G5B | NM_021221.1 | 888 | gtgatcaccatctattatgatgt | 281917 | - | see also 18739 line |
| 42454 | LY9 | NM_002348.1 | 918 | aacggcagatccactcattaaat | 281932 | - | see also 18740 line |

Figure 46

| 42455 | LY9 | NM_002348.1 | 1788 | tccatatgtcacggaagttgagt | 281941 | - | see also 18740 line |
|---|---|---|---|---|---|---|---|
| 42456 | MAC30 | NM_014573.1 | 490 | tccgatactctccacatttctgt | 281943 | - | see also 18741 line |
| 42457 | MAC30 | NM_014573.1 | 611 | atcccattcatactttaatttt | 281944 | - | see also 18741 line |
| 42458 | MAD1L1 | NM_003550.1 | 2024 | gcggctcaaggaggttttccaga | 281955 | - | see also 18742 line |
| 42459 | MAD1L1 | NM_003550.1 | 760 | tggacctgcaacacaaaaaatgc | 281952 | - | see also 18742 line |
| 42460 | MAGEA4 | NM_002362.3 | 833 | atcgtcctgggcacaattgcaat | 281958 | - | see also 18743 line |
| 42461 | MAGEA4 | NM_002362.3 | 550 | cagtaacaaggtggatgagttgg | 281956 | - | see also 18743 line |
| 42462 | MAGEA5 | NM_021049.2 | 436 | cactctatggaggcaatccatta | 281962 | - | see also 18744 line |
| 42463 | MAGEA5 | NM_021049.2 | 419 | cccactgccatcgatttcactct | 281960 | - | see also 18744 line |
| 42464 | MAGEL2 | NM_019066.2 | 797 | ggccacacgctagcctttcatga | 281968 | - | see also 18746 line |
| 42465 | MAGEL2 | NM_019066.2 | 1266 | atgagtgcttagatatcatcaac | 281971 | - | see also 18746 line |
| 42466 | MCSP | NM_030663.2 | 459 | cccatagcccacaaaatgagtcc | 281996 | - | see also 18748 line |
| 42467 | MCSP | NM_030663.2 | 455 | cagccccatagcccacaaaatga | 281995 | - | see also 18748 line |
| 42468 | MFAP4 | NM_002404.1 | 253 | cgggaagtggacggttttccaga | 281999 | - | see also 18749 line |
| 42469 | MGC13272 | NM_032355.2 | 1701 | ctcgcccactcaagaccatttac | 282009 | - | see also 9170 line |
| 42470 | MGC26979 | NM_153704.2 | 2277 | ttcgacagttcgttgatttatgc | 282084 | - | see also 10013 line |
| 42471 | MGC29816 | NM_152272.1 | 1255 | cccgcaataggcattttaccaac | 282121 | - | see also 9825 line |
| 42472 | MGC29816 | NM_152272.1 | 1353 | ttggtcccaagcagtaaatctcc | 282123 | - | see also 9825 line |
| 42473 | MLF2 | NM_005439.1 | 267 | agcatatgagccgtatgttgtca | 282127 | - | see also 18753 line |
| 42474 | MLF2 | NM_005439.1 | 253 | tgctattcaccgtcagcatatga | 282126 | - | see also 18753 line |
| 42475 | MLN51 | NM_007359.3 | 1591 | ctccggtggattctagtacaagt | 282156 | - | see also 5090 line |
| 42476 | MLN51 | NM_007359.3 | 2196 | caggccccatcacaggtatatgg | 282173 | - | see also 5090 line |
| 42477 | MN1 | NM_002430.1 | 3337 | agcgcacctcggccagtaaattg | 282191 | - | see also 18755 line |
| 42478 | MN1 | NM_002430.1 | 1634 | agccgtcgactcgctggaataca | 282181 | - | see also 18755 line |
| 42479 | MN1 | NM_002430.1 | 4267 | tgggcatcatgtctaactctacc | 282200 | - | see also 18755 line |
| 42480 | MORF4L2 | NM_012286.1 | 635 | aagtgaggaggcgtttaagaata | 282205 | - | see also 18756 line |
| 42481 | MORF4L2 | NM_012286.1 | 651 | aagaatagaatggaggttaaagt | 282209 | - | see also 18756 line |
| 42482 | MPV17 | NM_002437.3 | 83 | cgctcacccgtggaaagtacagg | 282219 | - | see also 18757 line |
| 42483 | MPV17 | NM_002437.3 | 330 | ggctttgccccgtgtttctagg | 282223 | - | see also 18757 line |
| 42484 | MRPL39 | NM_017446.2 | 64 | cacccggtggcgggatcaaatgg | 282229 | - | see also 7013 line |
| 42485 | MRPL39 | NM_017446.2 | 884 | gccaagtctcatacgaagattcc | 282267 | - | see also 7013 line |
| 42486 | MRPL40 | NM_003776.2 | 576 | cacaccaccgatccctaactacc | 282285 | - | see also 18759 line |
| 42487 | MRPL40 | NM_003776.2 | 131 | ctcaccagcgagcgtcattgttg | 282275 | - | see also 18759 line |

Figure 46

| 42488 | MRPL40 | NM_003776.2 | 137 | agcgagcgtcattgttgtctttc | 282277 | - | see also 18759 line | | |
|---|---|---|---|---|---|---|---|---|---|
| 42489 | MRPS10 | NM_018141.2 | 98 | ggcaatacagccaaaaatggtgg | 282289 | - | - | structural constituent of ribosome | - |
| 42490 | MRPS10 | NM_018141.2 | 146 | tgggtacagttttcaaacctaca | 282290 | - | see also 42489 line | | |
| 42491 | MRPS10 | NM_018141.2 | 157 | ttcaaacctacacgttgatgttc | 282291 | - | see also 42489 line | | |
| 42492 | MRPS22 | NM_020191.2 | 503 | accacaccgggagcgttttattg | 282311 | - | see also 7791 line | | |
| 42493 | MRPS22 | NM_020191.2 | 502 | taccacaccgggagcgttttatt | 282310 | - | see also 7791 line | | |
| 42494 | MRPS24 | NM_032014.1 | 254 | agcgaacggtggaggatgttttc | 282327 | - | see also 18760 line | | |
| 42495 | MRPS24 | NM_032014.1 | 120 | ggcccgagtacgcgtaagcaagg | 282326 | - | see also 18760 line | | |
| 42496 | MSLN | NM_005823.2 | 1322 | ccctgatcgaccgctttgtgaag | 282340 | - | see also 18761 line | | |
| 42497 | MSLN | NM_005823.2 | 1611 | ggcgctcagtcagcagaatgtga | 282342 | - | see also 18761 line | | |
| 42498 | MSLN | NM_005823.2 | 1127 | agctggacgtcctaaagcataaa | 282339 | - | see also 18761 line | | |
| 42499 | MSMB | NM_002443.2 | 69 | accttcgtgactttatgcaatgc | 282345 | - | see also 18762 line | | |
| 42500 | MSMB | NM_002443.2 | 353 | ttctgtcagtgaatggataatct | 282356 | - | see also 18762 line | | |
| 42501 | MUM-1 | NM_032853.2 | 1401 | taccacgaaccacgttcgtttga | 282374 | - | see also 18763 line | | |
| 42502 | MUM-1 | NM_032853.2 | 1728 | tacgcggctgatataagctatcc | 282381 | - | see also 18763 line | | |
| 42503 | MUM-1 | NM_032853.2 | 484 | ttctgagcgagggctcgatttgg | 282363 | - | see also 18763 line | | |
| 42504 | NAG | NM_015909.1 | 1995 | cgcccgattggccccatgtttca | 282437 | - | see also 18764 line | | |
| 42505 | NAG | NM_015909.1 | 1913 | atgctaaccggtgctttgaaaag | 282432 | - | see also 18764 line | | |
| 42506 | NAG | NM_015909.1 | 627 | gtgctctagtcgccttgaaaaca | 282397 | - | see also 18764 line | | |
| 42507 | NAP1 | NM_016481.2 | 172 | cagtcggctacttggaatcttgt | 282486 | - | see also 18765 line | | |
| 42508 | NAP1 | NM_016481.2 | 431 | ccctcatcgtcccaatgcaatag | 282496 | - | see also 18765 line | | |
| 42509 | NCSTN | NM_015331.1 | 352 | aggggttatccacgtagtagaga | 282516 | - | see also 6367 line | | |
| 42510 | NCSTN | NM_015331.1 | 1162 | ctcgaggatggtctacgatatgg | 282535 | - | see also 6367 line | | |
| 42511 | NDRG2 | NM_016250.1 | 510 | ggcgagatatgctcttaaccacc | 282569 | - | see also 6753 line | | |
| 42512 | NDRG2 | NM_016250.1 | 259 | cacgatgtgggactcaactataa | 282559 | - | see also 6753 line | | |
| 42513 | NDRG2 | NM_016250.1 | 442 | tgcgtcctgcagtacctaaattt | 282564 | - | see also 6753 line | | |
| 42514 | NEDF | NM_016079.1 | 241 | agcaagctgtatgcatccaaagc | 282580 | - | see also 18769 line | | |
| 42515 | NEDF | NM_016079.1 | 111 | caggcaaataagggatatccaaa | 282575 | - | see also 18769 line | | |
| 42516 | NEUGRIN | NM_016645.1 | 680 | gagcagatacggtatttacatga | 282582 | - | see also 18770 line | | |
| 42517 | NEUGRIN | NM_016645.1 | 764 | gtgatccgaagagttttaaaaag | 282585 | - | see also 18770 line | | |
| 42518 | NOC4 | NM_006067.3 | 742 | gacgctcgtggatttcgataacc | 282597 | - | see also 18771 line | | |
| 42519 | NOC4 | NM_006067.3 | 455 | agctacgtgattgctggttatta | 282588 | - | see also 18771 line | | |
| 42520 | NOLA3 | NM_018648.2 | 225 | gacaccgaatcaccatcaagaaa | 282603 | - | see also 7535 line | | |
| 42521 | NOLA3 | NM_018648.2 | 203 | tccccagatgacaaatactctcg | 282602 | - | see also 7535 line | | |
| 42522 | NPC2 | NM_006432.3 | 161 | ggccgaaccggtgcagttcaagg | 282605 | - | see also 18773 line | | |
| 42523 | NPC2 | NM_006432.3 | 536 | cccagtacagatcgtttctcatc | 282614 | - | see also 18773 line | | |

Figure 46

| | | | | | | | |
|---|---|---|---|---|---|---|---|
| 42524 | NRM | NM_007243.1 | 103 | acctcccttggccacttcttgg | 282616 | - | see also 18774 line |
| 42525 | NRM | NM_007243.1 | 220 | gggctcctgcttctatttgttgg | 282617 | - | see also 18774 line |
| 42526 | NTN2L | NM_006181.1 | 1558 | accgcatcagcctaaagaagttc | 282618 | - | see also 18775 line |
| 42527 | NTNG1 | NM_014917.1 | 930 | aactcgatcctccggatattacc | 282625 | - | see also 6088 line |
| 42528 | NTNG1 | NM_014917.1 | 1418 | gacaggttcgcgtttttgctgg | 282642 | - | see also 6088 line |
| 42529 | NTNG2 | NM_032536.1 | 1160 | tccaaccgctgcagctacattga | 282652 | - | see also 18777 line |
| 42530 | NTNG2 | NM_032536.1 | 294 | cccatgagaatcctacctatgc | 282650 | - | see also 18777 line |
| 42531 | NXPH3 | NM_007225.1 | 813 | gcccagattacaactaacatagt | 282660 | - | see also 18778 line |
| 42532 | NXPH3 | NM_007225.1 | 320 | ggggaggcttgggcattcttgg | 282654 | - | see also 18778 line |
| 42533 | NXPH4 | NM_007224.1 | 485 | ggctgggggggacttctacttg | 282661 | - | see also 18779 line |
| 42534 | NXPH4 | NM_007224.1 | 862 | ttgccacgtggatgatgagaaga | 282663 | - | see also 18779 line |
| 42535 | NYREN18 | NM_016118.3 | 707 | acegtacttagacatagctaacc | 282678 | - | see also 6672 line |
| 42536 | NYREN18 | NM_016118.3 | 706 | caccgtacttagacatagctaac | 282677 | - | see also 6672 line |
| 42537 | OFD1 | NM_003611.1 | 2103 | aacgttctagcacgtatggttgc | 282759 | - | see also 2425 line |
| 42538 | OFD1 | NM_003611.1 | 315 | atggcgcagtccaacatgtttac | 282702 | - | see also 2425 line |
| 42539 | OTOF | NM_004802.2 | 2224 | gggcaagaccgggggatgatgagg | 282798 | - | see also 18782 line |
| 42540 | OTOF | NM_004802.2 | 1069 | tccgcccatcattgtcattgaaa | 282788 | - | see also 18782 line |
| 42541 | OXR1 | NM_181354.2 | 1752 | ggcatcgattacataagttcttg | 282888 | - | see also 10230 line |
| 42542 | OXR1 | NM_181354.2 | 678 | ttcgacctgcacgagttgtatct | 282857 | - | see also 10230 line |
| 42543 | P53AIP1 | NM_022112.1 | 426 | tgccgacatcccaagtttcaga | 282917 | - | apoptosis |
| 42544 | P53AIP1 | NM_022112.1 | 423 | tggtgccgacatcccaagtttc | 282916 | - | see also 42543 line |
| 42545 | P53AIP1 | NM_022112.1 | 428 | ccgacatcccaagtttcagact | 282918 | - | see also 42543 line |
| 42546 | PART1 | NM_016590.2 | 144 | ttctcgtacgctgggctataatc | 282923 | - | see also 6935 line |
| 42547 | PDCD1LG2 | NM_025239.2 | 848 | ctcacgtgaggggaacttactttg | 282933 | - | see also 18785 line |
| 42548 | PDCD1LG2 | NM_025239.2 | 972 | cacagttgatagccctaagaaaac | 282935 | - | see also 18785 line |
| 42549 | PHEMX | NM_005705.3 | 443 | gacacctacgacctggtatatga | 282936 | - | see also 18786 line |
| 42550 | PHEMX | NM_005705.3 | 834 | cccagggtggaccaacacattgt | 282939 | - | see also 18786 line |
| 42551 | PKP2 | NM_005705.3 | 445 | caactacgacctggtatatgagc | 282937 | - | see also 18786 line |
| 42552 | PKP2 | NM_004572.1 | 236 | tccgttgggcaacggaaatcttca | 282947 | - | see also 18787 line |
| 42553 | PKP2 | NM_004572.1 | 1600 | tacgctgacggagaatcatca | 282964 | - | see also 18787 line |
| 42554 | PLAC1 | NM_021796.1 | 505 | cagttcacctaccgttactga | 282984 | - | see also 18788 line |
| 42555 | PLAC1 | NM_021796.1 | 594 | ttctaagggcacgccatctaagt | 282989 | - | see also 18788 line |
| 42556 | POLA2 | NM_002689.2 | 701 | ctcagaagcgagctatcctacc | 283008 | - | see also 1751 line |
| 42557 | POLA2 | NM_002689.2 | 1739 | gagtgatcttcgcttgacatcc | 283030 | - | see also 1751 line |
| 42558 | POPDC2 | NM_022135.2 | 270 | ggggtgtatggatgcttctatct | 283040 | - | see also 18790 line |

Figure 46

| 42559 | POPDC2 | NM_022135.2 | 861 | gagaagctctacactctcaatga | 283046 | - | see also 18790 line |
|---|---|---|---|---|---|---|---|
| 42560 | POPDC3 | NM_022361.3 | 487 | gccgaaggagccatttatcatct | 283051 | - | see also 8345 line |
| 42561 | POPDC3 | NM_022361.3 | 1076 | ttgctcagcatcgctacatctcc | 283067 | - | see also 8345 line |
| 42562 | PP3111 | NM_022156.3 | 763 | tgcagcgtcctgggagcatatca | 283082 | - | see also 18792 line |
| 42563 | PP3111 | NM_022156.3 | 518 | ttgggctgcccacagatgatagc | 283081 | - | see also 18792 line |
| 42564 | PP35 | NM_181581.1 | 427 | gccccaatggttcgatattcaaa | 283090 | - | see also 18793 line |
| 42565 | PP35 | NM_181581.1 | 599 | ctgctaacgatgcaagactttta | 283101 | - | see also 18793 line |
| 42566 | PRCC | NM_005973.4 | 1007 | tccgtcgccctctgctatcaagg | 283130 | - | see also 18794 line |
| 42567 | PRCC | NM_005973.4 | 916 | cggatggctcccctgatactaag | 283129 | - | see also 18794 line |
| 42568 | PRKAG2 | NM_016203.2 | 1246 | cccgttattgaccctatcagtgg | 283161 | - | see also 6719 line |
| 42569 | PRKAG2 | NM_016203.2 | 866 | gtgtttacatgcgattcatgagg | 283147 | - | see also 6719 line |
| 42570 | PRM3 | NM_021247.1 | 80 | gccacgaatcctccatgaaaaag | 283170 | - | see also 18796 line |
| 42571 | PRM3 | NM_021247.1 | 122 | aggataacttctccttgtcatca | 283173 | - | see also 18796 line |
| 42572 | PROK2 | NM_021935.2 | 222 | gactcgtaaagttccattttttg | 283175 | - | see also 18797 line |
| 42573 | PROK2 | NM_021935.2 | 285 | ggcctgtttacggacttcattta | 283177 | - | see also 18797 line |
| 42574 | PROL1 | NM_021225.1 | 232 | ctccctatgactcaagacttaat | 283184 | - | see also 18798 line |
| 42575 | PROL1 | NM_021225.1 | 516 | atcaccaccgcagatacaacaat | 283193 | - | see also 18798 line |
| 42576 | PROSC | NM_007198.2 | 625 | ggctagtggcggtcagcaaaacc | 283198 | - | see also 18799 line |
| 42577 | PROSC | NM_007198.2 | 1244 | tggagagcgggattactcaaaga | 283215 | - | see also 18799 line |
| 42578 | PSG9 | NM_002784.2 | 337 | atcgtatatagttgatggtaaaa | 283230 | - | see also 18801 line |
| 42579 | PSG9 | NM_002784.2 | 324 | accattacattatatcgtatata | 283229 | - | see also 18801 line |
| 42580 | PSMD9 | NM_002813.4 | 515 | cacgggccttcgccaaagtgaac | 283233 | - | - | - | - |
| 42581 | PSMD9 | NM_002813.4 | 241 | gacgtgctggaaagccaaaaagg | 283232 | - | see also 42580 line |
| 42582 | PTD008 | NM_016145.1 | 326 | cccgacgccggactacatgaacc | 283236 | - | see also 18802 line |
| 42583 | PTD008 | NM_016145.1 | 325 | acccgacgccggactacatgaac | 283235 | - | see also 18802 line |
| 42584 | PTD008 | NM_016145.1 | 257 | cccacggaggccgaacaaagtgc | 283234 | - | see also 18802 line |
| 42585 | PTTG1IP | NM_004339.2 | 380 | gtctttggtgcaacactaacaag | 283238 | - | see also 18803 line |
| 42586 | PTTG1IP | NM_004339.2 | 724 | aacccgtatgctagatttgaaaa | 283243 | - | see also 18803 line |
| 42587 | PTTG1IP | NM_004339.2 | 725 | acccgtatgctagatttgaaaac | 283244 | - | see also 18803 line |
| 42588 | RAI2 | NM_021785.2 | 1189 | ttcggcctgcacccctttaaagg | 283251 | - | see also 18804 line |
| 42589 | RAI2 | NM_021785.2 | 1709 | aagatgctgtgcctaccatattc | 283259 | - | see also 18804 line |
| 42590 | RCD-8 | NM_014329.2 | 2535 | gggatggagatcggcataatacc | 283284 | - | see also 5623 line |
| 42591 | RCD-8 | NM_014329.2 | 746 | tggcgcttggctctggttaatgg | 283268 | - | see also 5623 line |
| 42592 | RER1 | NM_007033.2 | 590 | tactttcttcgacgctttcaacg | 283303 | - | see also 18807 line |
| 42593 | RER1 | NM_007033.2 | 289 | ggggatctaccatctaaatcttt | 283299 | - | see also 18807 line |
| 42594 | RIOK2 | NM_018343.1 | 102 | gggtcttgaccgcggttgaaatg | 283306 | - | see also 7410 line |
| 42595 | RIOK3 | NM_003831.2 | 1326 | tgcggcagttatatcatgaatgt | 283376 | - | see also 2611 line |

Figure 46

| 42596 | RIOK3 | NM_003831.2 | 1325 | atgcggcagttatatcatgaatg | 283375 | - | see also 2611 line |
|---|---|---|---|---|---|---|---|
| 42597 | RP1 | NM_006269.1 | 4139 | gagacctacgttcctgtcaatgt | 283500 | - | see also 18809 line |
| 42598 | RP1 | NM_006269.1 | 5092 | tgggtctacccgcaaatctcagg | 283526 | - | see also 18809 line |
| 42599 | RP42 | NM_020640.2 | 84 | ttcgtcagtttatgatcttcaca | 283579 | - | see also 18810 line |
| 42600 | RP42 | NM_020640.2 | 647 | ttgttggaacatcataaacgatc | 283593 | - | see also 18810 line |
| 42601 | SACS | NM_014363.3 | 9994 | gccgctatgtaagcattggaaaa | 283927 | - | see also 5631 line |
| 42602 | SACS | NM_014363.3 | 1963 | aacgatttgcacgtcttatcaag | 283647 | - | see also 5631 line |
| 42603 | SBDS | NM_016038.2 | 827 | ggctgcttccgagaaattgatga | 284073 | - | - |
| 42604 | SBDS | NM_016038.2 | 880 | ttctttggaagtactcaatctga | 284075 | - | see also 42603 line |
| 42605 | SBDS | NM_016038.2 | 837 | gagaaattgatgagctaataaaa | 284074 | - | see also 42603 line |
| 42606 | SCA7 | NM_000333.1 | 1844 | accctcacggagtgattccttcc | 284083 | - | see also 283 line |
| 42607 | SCA7 | NM_000333.1 | 2417 | cagctcatggaaccacactaaat | 284092 | - | see also 283 line |
| 42608 | SCHIP1 | NM_014575.1 | 1345 | cagctacaagtgatagtcaatga | 284111 | - | see also 5762 line |
| 42609 | SEL1L | NM_005065.3 | 900 | agctcttgggggcaatctaatag | 284133 | - | see also 18814 line |
| 42610 | SEL1L | NM_005065.3 | 1642 | accggcgtgatgcgatcatgtca | 284157 | - | see also 18814 line |
| 42611 | SEPX1 | NM_016332.1 | 266 | agcgtccggagcacaatagatct | 284183 | - | see also 18815 line |
| 42612 | SEPX1 | NM_016332.1 | 265 | aagcgtccggagcacaatagatc | 284182 | - | see also 18815 line |
| 42613 | Shax3 | NM_152284.2 | 520 | agcgatgaaatctgttcatgaaa | 284191 | - | see also 18816 line |
| 42614 | Shax3 | NM_152284.2 | 297 | aagagtacctggaaaatcgaatc | 284188 | - | see also 18816 line |
| 42615 | SHFM1 | NM_006304.1 | 304 | aactagagaaacatggttataag | 284198 | - | see also 4396 line |
| 42616 | SHFM3 | NM_022039.2 | 1682 | tcctcctactacggtgttgtacg | 284200 | - | see also 18818 line |
| 42617 | SHOC2 | NM_007373.1 | 873 | ttcgctttaatcgtataactact | 284219 | - | see also 5097 line |
| 42618 | SHOC2 | NM_007373.1 | 869 | taccttcgctttaatcgtataac | 284217 | - | see also 5097 line |
| 42619 | SHOC2 | NM_007373.1 | 870 | accttcgctttaatcgtataact | 284218 | - | see also 5097 line |
| 42620 | SIPA1L1 | NM_015556.1 | 1192 | ctcaagcgacgttcaaaatctga | 284275 | - | see also 6465 line |
| 42621 | SIPA1L1 | NM_015556.1 | 1940 | ctccgtacaactaccgaataatt | 284294 | - | see also 6465 line |
| 42622 | SKI | NM_003036.1 | 1049 | ctgagcctccggcctccataaga | 284363 | - | see also 18820 line |
| 42623 | SKI | NM_003036.1 | 435 | gtgtctgccgcagattctcaact | 284360 | - | see also 18820 line |
| 42624 | SLC22A1LS | NM_007105.1 | 1020 | gtgctccgaggagaatgcaaagg | 284368 | - | see also 18821 line |
| 42625 | SLC35F2 | NM_017515.3 | 726 | gacaattcagggagtgatgtatt | 284387 | - | see also 18822 line |
| 42626 | SLC35F2 | NM_017515.3 | 543 | gcctaccagtacacaactctaac | 284383 | - | see also 18822 line |
| 42627 | SMARCD1 | NM_003076.3 | 1131 | ccctcacgagcgggagtttgtca | 284407 | - | see also 2067 line |
| 42628 | SNAP25 | NM_003081.2 | 794 | ggccaaccaacgtgcaacaaaga | 284429 | - | see also 18823 line |
| 42629 | SNAP25 | NM_003081.2 | 692 | ccgtcacatggccctggatatgg | 284426 | - | see also 18823 line |
| 42630 | SOST | NM_025237.1 | 122 | gtggcaggcgttcaagaatgatg | 284433 | - | see also 18824 line |
| 42631 | SOST | NM_025237.1 | 118 | aggggtggcaggcgttcaagaat | 284431 | - | see also 18824 line |

Figure 46

| | | | | | | | |
|---|---|---|---|---|---|---|---|
| 42632 | SPAG8 | NM_012436.2 | 154 | gtcgcgatctttagacatacagc | 284441 | - | see also 18825 line |
| 42633 | SPAG8 | NM_012436.2 | 780 | cagatcggtccctaactatacc | 284451 | - | see also 18825 line |
| 42634 | SPANXB1 | NM_032461.2 | 391 | aacgactgaaagacctaaaaagt | 284468 | - | - |
| 42635 | SPATA2 | NM_006038.2 | 1701 | tgcctcagcgcttaccattatga | 284483 | - | see also 4207 line |
| 42636 | SPATA2 | NM_006038.2 | 414 | aaggtggatccctttatcgatt | 284473 | - | see also 4207 line |
| 42637 | SPRY3 | NM_005840.1 | 514 | tactcatgctagcaatgaactacg | 284487 | - | see also 18827 line |
| 42638 | SPRY3 | NM_005840.1 | 681 | ctcagcatcgagccaatctagc | 284489 | - | see also 18827 line |
| 42639 | SPRY3 | NM_005840.1 | 985 | tgctgagcctctcgattatg | 284491 | - | see also 18827 line |
| 42640 | SQSTM1 | NM_003900.2 | 725 | tcggaggatccgagtgtgaattt | 284498 | - | see also 18828 line |
| 42641 | SQSTM1 | NM_003900.2 | 793 | gggcattgaagttgatatcgatg | 284501 | - | see also 18828 line |
| 42642 | ST7 | NM_013437.2 | 1679 | ctccctcgtatggacaattgatt | 284562 | - | see also 5439 line |
| 42643 | ST7 | NM_013437.2 | 291 | cccaggcgaaatcaattactataa | 284514 | - | see also 5439 line |
| 42644 | STAF65(gamma) | NM_014860.1 | 1053 | accgcatcaaggactatcacagt | 284590 | - | see also 18830 line |
| 42645 | STAF65(gamma) | NM_014860.1 | 1433 | gtcttcgaggagcctatgtcagg | 284594 | - | see also 18830 line |
| 42646 | SUMF2 | NM_015411.1 | 226 | aaccctttgccatcgacatatt | 284596 | - | see also 18831 line |
| 42647 | SUMF2 | NM_015411.1 | 870 | atgggcaacactccagattcagc | 284602 | - | see also 18831 line |
| 42648 | SURF2 | NM_017503.2 | 729 | aagcagttgggctcgttgaaaaa | 284605 | - | see also 18832 line |
| 42649 | SURF2 | NM_017503.2 | 737 | gggctcgttgaaaaagaagttca | 284606 | - | see also 18832 line |
| 42650 | SURF4 | NM_033161.2 | 764 | ctctttgccatcaacgtatatt | 284614 | - | see also 18833 line |
| 42651 | SURF4 | NM_033161.2 | 762 | ggctctttgccatcaacgtatat | 284613 | - | see also 18833 line |
| 42652 | SURF4 | NM_033161.2 | 654 | ttgacgccagcttctttctatt | 284611 | - | see also 18833 line |
| 42653 | SYMPK | NM_004819.1 | 1806 | agcccaggtcgcataaagatcc | 284635 | - | see also 18834 line |
| 42654 | SYMPK | NM_004819.1 | 1803 | ggcagcccagtccgcataaaga | 284633 | - | see also 18834 line |
| 42655 | SYMPK | NM_004819.1 | 2894 | agctcctgatcgcattacacaac | 284645 | - | see also 18834 line |
| 42656 | TBL2 | NM_012453.1 | 1212 | gcgagtgtatcgccaacttgtcc | 284659 | - | see also 18835 line |
| 42657 | TBL2 | NM_012453.1 | 1147 | cagtagtattcattcttacaata | 284657 | - | see also 18835 line |
| 42658 | TCTE1L | NM_006520.1 | 366 | aaccggaccatgaactgattgt | 284687 | - | see also 18836 line |
| 42659 | TCTE1L | NM_006520.1 | 199 | ctgcaagcatagtgaacaatcc | 284682 | - | see also 18836 line |
| 42660 | TEX27 | NM_021943.1 | 605 | atcgtgtcgctgcggttatgtgt | 284700 | - | see also 8205 line |
| 42661 | TEX27 | NM_021943.1 | 608 | gtgtcgctgcggttatgtgttct | 284701 | - | see also 8205 line |
| 42662 | TFG | NM_006070.3 | 368 | gaggatattcggcgaattcctat | 284704 | - | see also 4215 line |
| 42663 | TFG | NM_006070.3 | 369 | aggatattcggcgaattccatt | 284705 | - | see also 4215 line |
| 42664 | THSD3 | NM_182509.2 | 358 | tgccggattggccaacacaacc | 284738 | - | see also 18839 line |
| 42665 | THSD3 | NM_182509.2 | 853 | tgctacggacatgcatgatcaag | 284741 | - | see also 18839 line |
| 42666 | THSD3 | NM_182509.2 | 856 | tacggacatgcatgatcaagatg | 284742 | - | see also 18839 line |

Figure 46

| 42667 | THY1 | NM_006288.2 | 309 | aacatgaaggtcctctacttatc | 284746 | - | see also 4378 line |
|---|---|---|---|---|---|---|---|
| 42668 | THY1 | NM_006288.2 | 287 | aaccaacttcaccagcaaataca | 284745 | - | see also 4378 line |
| 42669 | TIMP3 | NM_000362.3 | 1713 | tggctaccagtccaaacactacg | 284753 | - | see also 296 line |
| 42670 | TIMP3 | NM_000362.3 | 1508 | gcgtctatgatggcaagatgtac | 284749 | - | see also 296 line |
| 42671 | TLE4 | NM_007005.3 | 1537 | aaggaaattgcagctcgttatga | 284764 | - | see also 4888 line |
| 42672 | TLE4 | NM_007005.3 | 3079 | tccgtggacgacaaatacattgt | 284781 | - | see also 4888 line |
| 42673 | TM4SF1 | NM_014220.1 | 159 | ctgtgcatcgcggctaatatttt | 284784 | - | see also 18843 line |
| 42674 | TM4SF1 | NM_014220.1 | 161 | gtgcatcgcggctaatattttgc | 284785 | - | see also 18843 line |
| 42675 | TM4SF1 | NM_014220.1 | 157 | tcctgtgcatcgcggctaatatt | 284783 | - | see also 18843 line |
| 42676 | TM6SF1 | NM_023003.1 | 793 | tgccgattatatacgcaatttca | 284827 | - | see also 8537 line |
| 42677 | TM6SF1 | NM_023003.1 | 203 | tggctcgtgtcctcgtcaaaaga | 284802 | - | see also 8537 line |
| 42678 | TMEFF2 | NM_016192.2 | 1182 | taccttgtccggaacattacaat | 284857 | - | see also 6717 line |
| 42679 | TMEFF2 | NM_016192.2 | 1497 | gagcgtccacgaggttaatctaa | 284871 | - | see also 6717 line |
| 42680 | TMEM14A | NM_014051.2 | 157 | tggacctgatcggttttggttat | 284873 | - | see also 18846 line |
| 42681 | TMEM14A | NM_014051.2 | 159 | gacctgatcggttttggttatgc | 284874 | - | see also 18846 line |
| 42682 | TMEM14A | NM_014051.2 | 156 | atggacctgatcggttttggtta | 284872 | - | see also 18846 line |
| 42683 | TMEM8 | NM_021259.1 | 951 | ctcgggacagtggctttcagtgc | 284886 | - | see also 18847 line |
| 42684 | TMEM8 | NM_021259.1 | 1035 | agccaaaaccagagcttcaatgc | 284887 | - | see also 18847 line |
| 42685 | TMEM9 | NM_016456.2 | 465 | atccgaaagccggatgcatatac | 284894 | - | see also 6866 line |
| 42686 | TMEM9 | NM_016456.2 | 466 | tccgaaagccggatgcatatact | 284895 | - | see also 6866 line |
| 42687 | TMEPAI | NM_020182.3 | 941 | tgcgcgcacccccaaacagaacc | 284897 | - | see also 18848 line |
| 42688 | TPARL | NM_018475.2 | 651 | aacgggtacaagcataacagtac | 284910 | - | see also 18849 line |
| 42689 | TPARL | NM_018475.2 | 837 | gtgcacgggattggcagtaattg | 284919 | - | see also 18849 line |
| 42690 | TPD52L1 | NM_003287.1 | 676 | ggcggtacgaaccctaatggagg | 284935 | - | see also 18850 line |
| 42691 | TPD52L1 | NM_003287.1 | 571 | tccattcgccattccataagtat | 284934 | - | see also 18850 line |
| 42692 | TPD52L1 | NM_003287.1 | 354 | agcgaaagaaaggcatctagttg | 284927 | - | see also 18850 line |
| 42693 | TSGA10 | NM_025244.1 | 900 | aacaacggcacatgctattctcc | 284948 | - | see also 18851 line |
| 42694 | TSGA10 | NM_025244.1 | 854 | cccgacttcgacgagaaatgatg | 284944 | - | see also 18851 line |
| 42695 | TU3A | NM_007177.1 | 1032 | agggaaaacctgcggagaattgc | 285009 | - | see also 18852 line |
| 42696 | TXNIP | NM_006472.1 | 1023 | aacatccttcgagttgaatattc | 285023 | - | see also 18853 line |
| 42697 | TXNIP | NM_006472.1 | 1340 | gccaccaccgacttatactgagg | 285034 | - | see also 18853 line |
| 42698 | TXNIP | NM_006472.1 | 1335 | ttcatgccaccaccgacttatac | 285032 | - | see also 18853 line |
| 42699 | UACA | NM_018003.1 | 663 | tgctaggttgcgaatatggttgc | 285053 | - | see also 18854 line |
| 42700 | UACA | NM_018003.1 | 221 | ggggtcaatccaggcaaactaga | 285039 | - | see also 18854 line |

Figure 46

| 42701 | USP6NL | NM_014688.1 | 2358 | gccgtctagtcgaatagaagtcc | 285184 | - | see also 18855 line | | |
|---|---|---|---|---|---|---|---|---|---|
| 42702 | USP6NL | NM_014688.1 | 2630 | gtcccgtgagatataaagcttca | 285191 | - | see also 18855 line | | |
| 42703 | UXT | NM_004182.2 | 239 | agctaagcactcggagttatata | 285197 | - | see also 18856 line | | |
| 42704 | UXT | NM_004182.2 | 165 | gaccatcgagacaaggtatatga | 285195 | - | see also 18856 line | | |
| 42705 | VCX-2r | NM_016378.1 | 399 | ctgtgtggatggccagcttttcc | 285201 | - | - | molecular_function unknown | - |
| 42706 | VMD2 | NM_004183.1 | 763 | tgcctacgactggattagtatcc | 285207 | - | see also 18857 line | | |
| 42707 | VMD2 | NM_004183.1 | 179 | ctgctatatggcgagttcttaat | 285202 | - | see also 18857 line | | |
| 42708 | VMD2L1 | NM_017682.1 | 432 | aggagcgcaagaagtttgaaaac | 285224 | - | see also 18858 line | | |
| 42709 | VMD2L1 | NM_017682.1 | 564 | tgctcgaggagctgaatgttttt | 285227 | - | see also 18858 line | | |
| 42710 | VMD2L2 | NM_153274.1 | 594 | cgggcgaatacgtgacgatatcg | 285235 | - | see also 18859 line | | |
| 42711 | VMD2L2 | NM_153274.1 | 1001 | tgctatccgtggacgaaatgtac | 285238 | - | see also 18859 line | | |
| 42712 | VMD2L3 | NM_152439.1 | 745 | atcggcattccatgataagaaga | 285250 | - | see also 18860 line | | |
| 42713 | VMD2L3 | NM_152439.1 | 427 | ttgggattccgctggtttacacc | 285243 | - | see also 18860 line | | |
| 42714 | WBSCR5 | NM_014146.2 | 569 | tgccaattcctacgagaatgtgc | 285255 | - | - | - | - |
| 42715 | WDR6 | NM_018031.2 | 2544 | ttcgtcccaccggctagatgagt | 285278 | - | see also 18861 line | | |
| 42716 | WDR6 | NM_018031.2 | 2549 | cccaccggctagatgagtattgg | 285281 | - | see also 18861 line | | |
| 42717 | WFDC1 | NM_021197.2 | 296 | ctgcgaggctggccgagaaatcc | 285287 | - | see also 18862 line | | |
| 42718 | WFDC1 | NM_021197.2 | 678 | aggtgacgtggccgaaggtatcc | 285288 | - | see also 18862 line | | |
| 42719 | WIT-1 | NM_015855.2 | 1022 | ggcctcgaaggtgcataattatt | 285296 | - | see also 18863 line | | |
| 42720 | WIT-1 | NM_015855.2 | 982 | accatgtggcgttgatacactgg | 285293 | - | see also 18863 line | | |
| 42721 | WIT-1 | NM_015855.2 | 1023 | gcctcgaaggtgcataattattg | 285297 | - | see also 18863 line | | |
| 42722 | WSB1 | NM_015626.7 | 1248 | accggtgggtacgatctgtatct | 285327 | - | see also 18864 line | | |
| 42723 | WSB1 | NM_015626.7 | 1252 | gtgggtacgatctgtatctttta | 285329 | - | see also 18864 line | | |
| 42724 | WSB1 | NM_015626.7 | 1247 | gaccggtgggtacgatctgtatc | 285326 | - | see also 18864 line | | |
| 42725 | XG | NM_175569.1 | 375 | tcccgacagcggtggaaatatct | 285344 | - | see also 18865 line | | |
| 42726 | XG | NM_175569.1 | 374 | atcccgacagcggtggaaatatc | 285343 | - | see also 18865 line | | |
| 42727 | YIF1 | NM_020470.1 | 826 | ctcatcggcgctcatgtacttca | 285353 | - | see also 18866 line | | |
| 42728 | YIF1 | NM_020470.1 | 729 | tggcctacagtggctacaaatac | 285351 | - | see also 18866 line | | |
| 42729 | ZNF200 | NM_003454.2 | 1973 | ttggtcggctgtcaaactgtacc | 285372 | - | see also 18867 line | | |
| 42730 | ZNF200 | NM_003454.2 | 1296 | cagtgtcggggaaatgatgttac | 285359 | - | see also 18867 line | | |
| 42731 | ZP4 | NM_021186.2 | 147 | aacgtctcctcctgtactaatag | 285374 | - | see also 18868 line | | |
| 42732 | ZP4 | NM_021186.2 | 497 | ggctaggctgttgttatagctct | 285382 | - | see also 18868 line | | |
| 42733 | AGS3 | NM_015597.2 | 1563 | ctcgccccagaccgaggaattct | 285409 | - | see also 18869 line | | |
| 42734 | AGS3 | NM_015597.2 | 572 | acctgggcaacacccactatttg | 285405 | - | see also 18869 line | | |
| 42735 | C15orf15 | NM_016304.2 | 185 | tcctggacacggcatgatgttcg | 285413 | - | - | - | - |
| 42736 | C15orf15 | NM_016304.2 | 184 | atcctggacacggcatgatgttc | 285412 | - | see also 42735 line | | |

Figure 46

| | | | | | | | |
|---|---|---|---|---|---|---|---|
| 42737 | C1orf33 | NM_016183.2 | 401 | taegcccgagctgtaaccaaagc | 285421 | - | see also 18870 line |
| 42738 | C1orf33 | NM_016183.2 | 610 | tgggtatgagatggctgaattca | 285422 | - | see also 18870 line |
| 42739 | DAP3 | NM_004632.2 | 451 | tgcttatccagctatacgatatc | 285437 | - | see also 3147 line |
| 42740 | DAP3 | NM_004632.2 | 460 | agctatacgatatctctgtatg | 285439 | - | see also 3147 line |
| 42741 | FLJ12331 | NM_024986.1 | 231 | ctctgggctagtacacccttatcc | 285458 | - | see also 18872 line |
| 42742 | FLJ12331 | NM_024986.1 | 536 | agcataaagctttcatacaagaat | 285459 | - | see also 18872 line |
| 42743 | GADD45B | NM_015675.1 | 222 | acgagtcggccaagttgatgaat | 285468 | - | see also 18874 line |
| 42744 | GADD45B | NM_015675.1 | 226 | gtcggccaagttgatgaatgtgg | 285470 | - | see also 18874 line |
| 42745 | GADD45G | NM_006705.2 | 323 | atcgcgctgcagatccattttac | 285474 | - | see also 18875 line |
| 42746 | GADD45G | NM_006705.2 | 324 | tcgcgctgcagatccattttacg | 285475 | - | see also 18875 line |
| 42747 | LACTB | NM_032857.2 | 487 | aaccgtgtaccatgtaaaccaga | 285479 | - | see also 9314 line |
| 42748 | LACTB | NM_032857.2 | 1129 | aacgagccagtgatttacaatag | 285496 | - | see also 9314 line |
| 42749 | MGC9651 | NM_152301.3 | 545 | agctcgtctggcgaatagagaga | 285509 | - | see also 18877 line |
| 42750 | MRP63 | NM_024026.3 | 342 | atctcaatgtcaccaagraaatgg | 285511 | - | - |
| 42751 | MRP63 | NM_024026.3 | 333 | agctcgaccatctcaatgtcacc | 285510 | - | see also 42750 line |
| 42752 | MRPL11 | NM_016050.2 | 276 | tgcctaccaagattttagtgaag | 285513 | - | see also 18878 line |
| 42753 | MRPL11 | NM_016050.2 | 277 | gcctaccaagattttagtgaagc | 285514 | - | see also 18878 line |
| 42754 | MRPL13 | NM_014078.4 | 766 | acctaaagtctagatgagtaca | 285533 | - | see also 18879 line |
| 42755 | MRPL13 | NM_014078.4 | 557 | ggctacccaggtggatttagaca | 285527 | - | see also 18879 line |
| 42756 | MRPL14 | NM_032111.1 | 480 | agcggaaggcgagtattccaag | 285540 | - | see also 18880 line |
| 42757 | MRPL14 | NM_032111.1 | 432 | accctgtggggacacgaattaag | 285537 | - | see also 18880 line |
| 42758 | MRPL15 | NM_014175.2 | 297 | cccaaaatacggctttaacgaag | 285545 | - | see also 18881 line |
| 42759 | MRPL15 | NM_014175.2 | 395 | atcctagtcaacctattgactta | 285548 | - | see also 18881 line |
| 42760 | MRPL15 | NM_014175.2 | 396 | tcctagtcaacctattgacttaa | 285549 | - | see also 18881 line |
| 42761 | MRPL16 | NM_017840.2 | 252 | tgcgggtgcccttgtcagattcc | 285569 | - | see also 18882 line |
| 42762 | MRPL16 | NM_017840.2 | 701 | atgggtgggcgttgtgaatttga | 285576 | - | see also 18882 line |
| 42763 | MRPL17 | NM_022061.2 | 328 | cccctcggtacaaagatcaaact | 285583 | - | see also 18883 line |
| 42764 | MRPL17 | NM_022061.2 | 326 | ggcccctcggtacaaagatcaaa | 285581 | - | see also 18883 line |
| 42765 | MRPL18 | NM_014161.2 | 588 | gactcgatgaaacgactacaaag | 285598 | - | see also 18884 line |
| 42766 | MRPL18 | NM_014161.2 | 440 | cactcgtgagtgggctattaaaa | 285593 | - | see also 18884 line |
| 42767 | MRPL19 | NM_014763.2 | 231 | aaccggaacgcaggttcttgagt | 285601 | - | see also 18885 line |
| 42768 | MRPL19 | NM_014763.2 | 567 | ggctggatagagcttgctatac | 285613 | - | see also 18885 line |
| 42769 | MRPL2 | NM_015950.2 | 334 | gccacaagcaacgttatcgaatg | 285637 | - | see also 18886 line |
| 42770 | MRPL2 | NM_015950.2 | 773 | ccgagtatccaacgttgatcata | 285642 | - | see also 18886 line |
| 42771 | MRPL22 | NM_014180.2 | 47 | cagttggtgcgttatggatca | 285649 | - | see also 18887 line |
| 42772 | MRPL22 | NM_014180.2 | 45 | gacagttgggtgcgttatggata | 285648 | - | see also 18887 line |
| 42773 | MRPL24 | NM_024540.2 | 580 | cacacgatcaggagagaattatcc | 285675 | - | see also 18888 line |

Figure 46

| 42774 | MRPL24 | NM_024540.2 | 767 | tacaagaaggtctattggtattg | 285678 | - | see also 18888 line |
|---|---|---|---|---|---|---|---|
| 42775 | MRPL27 | NM_016504.2 | 175 | cagacgccaaggcattaagaaaa | 285682 | - | see also 18889 line |
| 42776 | MRPL27 | NM_016504.2 | 133 | gtcgggtggtagctccaaaaacc | 285681 | - | see also 18889 line |
| 42777 | MRPL30 | NM_016503.2 | 168 | tgggattttgcgcttagtagttc | 285689 | - | see also 18890 line |
| 42778 | MRPL30 | NM_016503.2 | 169 | gggattttgcgcttagtagttca | 285690 | - | see also 18890 line |
| 42779 | MRPL32 | NM_031903.1 | 96 | ggggagtgcttcgaaactactgg | 285706 | - | see also 18891 line |
| 42780 | MRPL32 | NM_031903.1 | 250 | ttccagccttttggacagtatct | 285710 | - | see also 18891 line |
| 42781 | MRPL33 | NM_004891.2 | 215 | aagagtcctcttcgtggaaaaga | 285722 | - | - |
| 42782 | MRPL35 | NM_016622.2 | 301 | gccagtcagatctctaacatact | 285735 | - | see also 18892 line |
| 42783 | MRPL35 | NM_016622.2 | 157 | gtccactggacgttttagtcata | 285725 | - | see also 18892 line |
| 42784 | MRPL35 | NM_016622.2 | 459 | agcgattgagggaatttgtattc | 285738 | - | see also 18892 line |
| 42785 | MRPL36 | NM_032479.2 | 311 | tcggtggtacgtctactgtaaaa | 285750 | - | see also 18893 line |
| 42786 | MRPL36 | NM_032479.2 | 310 | gtcggtggtacgtctactgtaaa | 285749 | - | see also 18893 line |
| 42787 | MRPL36 | NM_032479.2 | 312 | cggtggtacgtctactgtaaaac | 285751 | - | see also 18893 line |
| 42788 | MRPL37 | NM_016491.2 | 357 | cacgagcatccgctgtacaaaga | 285752 | - | see also 18894 line |
| 42789 | MRPL37 | NM_016491.2 | 610 | cggtcatcgtggacaacctaata | 285761 | - | see also 18894 line |
| 42790 | MRPL4 | NM_015956.2 | 761 | ccccacaagttactactacatgc | 285781 | - | see also 18895 line |
| 42791 | MRPL4 | NM_015956.2 | 587 | tgctatgtggcagaagaacttca | 285780 | - | see also 18895 line |
| 42792 | MRPL42 | NM_014050.2 | 157 | tggctgtagctgcagtaaaatgg | 285785 | - | see also 18897 line |
| 42793 | MRPL42 | NM_014050.2 | 265 | ctcctctaccagatgactataat | 285789 | - | see also 18897 line |
| 42794 | MRPL43 | NM_032112.2 | 240 | acggaatccaggggtcgtaatat | 285793 | - | see also 18898 line |
| 42795 | MRPL43 | NM_032112.2 | 246 | tccaggggtcgtaatatatgtaa | 285795 | - | see also 18898 line |
| 42796 | MRPL46 | NM_022163.2 | 354 | ttgctggcgcaagatttggaaga | 285808 | - | see also 18899 line |
| 42797 | MRPL46 | NM_022163.2 | 358 | tggcgcaagatttggaagatatg | 285809 | - | see also 18899 line |
| 42798 | MRPL47 | NM_020409.2 | 403 | aagtccagagcggttagataagg | 285831 | - | see also 18900 line |
| 42799 | MRPL47 | NM_020409.2 | 406 | tccagagcggttagataaggtag | 285832 | - | see also 18900 line |
| 42800 | MRPL48 | NM_016055.3 | 446 | cacccacggcattggaaagtaca | 285852 | - | see also 6645 line |
| 42801 | MRPL48 | NM_016055.3 | 568 | ttcatctgactgcatatgatatg | 285857 | - | see also 6645 line |
| 42802 | MRPL50 | NM_019051.1 | 225 | gtcgtttggaatcttacgttaaa | 285869 | - | see also 18902 line |
| 42803 | MRPL50 | NM_019051.1 | 417 | tccctattcaagatagatctaaa | 285884 | - | see also 18902 line |
| 42804 | MRPL51 | NM_016497.2 | 520 | accgacatgggaagtttcgtag | 285888 | - | - |
| 42805 | MRPL51 | NM_016497.2 | 333 | atcgggatcctgggaaactttga | 285886 | - | see also 42804 line |
| 42806 | MRPL51 | NM_016497.2 | 425 | ttgtatccgaaagaggaaaatgg | 285887 | - | see also 42804 line |
| 42807 | MRPL9 | NM_031420.2 | 306 | accggcgacatcgcgtctataag | 285889 | - | see also 18903 line |
| 42808 | MRPL9 | NM_031420.2 | 310 | gcgacatcgcgtctataagctgg | 285891 | - | see also 18903 line |
| 42809 | MRPS11 | NM_022839.2 | 670 | agcggcgagagctaaacaaaagg | 285912 | - | see also 8491 line |
| 42810 | MRPS11 | NM_022839.2 | 394 | tgcggccaagcagaaagttgaac | 285907 | - | see also 8491 line |

Figure 46

| | | | | | | | | | |
|---|---|---|---|---|---|---|---|---|---|
| 42811 | MRPS12 | NM_021107.1 | 369 | ctcaacacgtccctaacttgtgg | 285916 | - | - | | - |
| 42812 | MRPS12 | NM_021107.1 | 367 | gcctcaacacgtccctaacttgt | 285915 | - | see also 42811 line | | |
| 42813 | MRPS14 | NM_022100.1 | 329 | gaggcttagtcgtatagtcttcc | 285929 | - | see also 18904 line | | |
| 42814 | MRPS14 | NM_022100.1 | 158 | cgcagatgagaggctacgtatta | 285920 | - | see also 18904 line | | |
| 42815 | MRPS15 | NM_031280.2 | 598 | aacctccgtaacaccaactatga | 285936 | - | see also 18905 line | | |
| 42816 | MRPS15 | NM_031280.2 | 333 | tggaattgagaaggttgatgatg | 285934 | - | see also 18905 line | | |
| 42817 | MRPS16 | NM_016065.2 | 293 | cccagggatggccgtttcgtaga | 285942 | - | - | structural constituent of ribosome | - |
| 42818 | MRPS16 | NM_016065.2 | 380 | gacaggatccgtcattggattgg | 285943 | - | see also 42817 line | | |
| 42819 | MRPS16 | NM_016065.2 | 513 | cacgtgaagtcctgttagcttct | 285948 | - | see also 42817 line | | |
| 42820 | MRPS17 | NM_015969.2 | 49 | tccgtccatgccagatggattgt | 285949 | - | see also 18906 line | | |
| 42821 | MRPS17 | NM_015969.2 | 243 | tccacgagcaaagcatgtgaaac | 285952 | - | see also 18906 line | | |
| 42822 | MRPS18A | NM_018135.2 | 380 | accgagcaggtctattaccaaat | 285959 | - | see also 18907 line | | |
| 42823 | MRPS18A | NM_018135.2 | 379 | caccgagcaggtctattaccaaa | 285958 | - | see also 18907 line | | |
| 42824 | MRPS18B | NM_014046.2 | 418 | ctgcgcccacacgggtatcatct | 285972 | - | see also 18908 line | | |
| 42825 | MRPS18B | NM_014046.2 | 313 | gacatgtattcgtcggaataaag | 285969 | - | see also 18908 line | | |
| 42826 | MRPS18C | NM_016067.1 | 207 | ctgcccatttcaatggaaaatcc | 285977 | - | - | | - |
| 42827 | MRPS2 | NM_016034.2 | 288 | tggctgtcggcacaggtttatgg | 285983 | - | see also 18909 line | | |
| 42828 | MRPS2 | NM_016034.2 | 169 | accgatttcaacgacaagatttt | 285979 | - | see also 18909 line | | |
| 42829 | MRPS21 | NM_018997.1 | 637 | agcatcggcggtattatgagaag | 285989 | - | see also 18910 line | | |
| 42830 | MRPS21 | NM_018997.1 | 724 | ctcgcaagatcaacttcttgatg | 285992 | - | see also 18910 line | | |
| 42831 | MRPS21 | NM_018997.1 | 608 | cactatggatgggctcattgagg | 285986 | - | see also 18910 line | | |
| 42832 | MRPS23 | NM_016070.2 | 221 | taccacgaggatcggattagagc | 285996 | - | see also 18911 line | | |
| 42833 | MRPS23 | NM_016070.2 | 238 | tagagcgaagtttattcagtgt | 285999 | - | see also 18911 line | | |
| 42834 | MRPS23 | NM_016070.2 | 113 | cccctgtggtttgacgtatatga | 285995 | - | see also 18911 line | | |
| 42835 | MRPS25 | NM_022497.3 | 144 | gccgcaccctgcaatatctgagc | 286003 | - | see also 18912 line | | |
| 42836 | MRPS25 | NM_022497.3 | 339 | tcctgcgattctacttagattct | 286009 | - | see also 18912 line | | |
| 42837 | MRPS27 | NM_015084.1 | 1000 | aacgatttaaggccttacattct | 286029 | - | see also 18914 line | | |
| 42838 | MRPS27 | NM_015084.1 | 223 | cacggcttatagacaacatttcc | 286019 | - | see also 18914 line | | |
| 42839 | MRPS31 | NM_005830.2 | 947 | aactatgggagttcccaattaac | 286047 | - | see also 18915 line | | |
| 42840 | MRPS31 | NM_005830.2 | 599 | atgcaaagcgacctaaaattagt | 286041 | - | see also 18915 line | | |
| 42841 | MRPS31 | NM_005830.2 | 893 | aacaacccttcagaatggattt | 286046 | - | see also 18915 line | | |
| 42842 | MRPS33 | NM_016071.2 | 274 | aaggagacttatgattggtatcc | 286058 | - | see also 18916 line | | |
| 42843 | MRPS33 | NM_016071.2 | 209 | ggcctactaattccaagtctatg | 286056 | - | see also 18916 line | | |
| 42844 | MRPS34 | NM_023936.1 | 620 | ctgggattaccctgcaaaacagg | 286061 | - | see also 18917 line | | |
| 42845 | MRPS35 | NM_021821.2 | 541 | aaccccagagcacgagtagtagt | 286071 | - | see also 18918 line | | |
| 42846 | MRPS35 | NM_021821.2 | 543 | ccccagagcacgagtagtagtct | 286072 | - | see also 18918 line | | |

EP 1 752 536 A1

Figure 46

| 42847 | MRPS36 | NM_033281.4 | 116 | gtcaaacacacactccattaat | 286090 | - | - | |
| 42848 | MRPS36 | NM_033281.4 | 163 | tcctaaaccaatgtatcagaag | 286093 | - | see also 42847 line | |
| 42849 | MRPS36 | NM_033281.4 | 277 | tccaccggacactgcagaaataa | 286095 | - | see also 42847 line | |
| 42850 | MRPS5 | NM_031902.3 | 619 | aagggcgttatggtttctatgg | 286108 | - | see also 18919 line | |
| 42851 | MRPS5 | NM_031902.3 | 1222 | aggtctctgggtccattaatatg | 286121 | - | see also 18919 line | |
| 42852 | MRPS6 | NM_032476.2 | 183 | ccgctacgagctggcttaatc | 286125 | - | see also 18920 line | |
| 42853 | MRPS7 | NM_015971.2 | 296 | gccgctatagtcctgaattcaag | 286129 | - | see also 6590 line | |
| 42854 | MRPS7 | NM_015971.2 | 297 | ccgctatagtcctgaattcaagg | 286130 | - | see also 6590 line | |
| 42855 | NOLA2 | NM_017838.2 | 213 | ctcacgcggaagcttacaaatg | 286138 | - | see also 18922 line | |
| 42856 | RPL10L | NM_080746.2 | 328 | gtggcagagatggctttcacatg | 286141 | - | see also 18923 line | |
| 42857 | RPL10L | NM_080746.2 | 306 | aacaaatacatggtgaaaagttg | 286140 | - | see also 18923 line | |
| 42858 | RPS27L | NM_015920.2 | 140 | gcctagtacaaagtccaaattct | 286147 | - | see also 18925 line | |
| 42859 | RPS27L | NM_015920.2 | 182 | gtccaggttgctacaagatcacc | 286148 | - | see also 18925 line | |
| 42860 | UBA52 | NM_003333.2 | 283 | tccgccagcttgcccagaaatac | 286156 | - | see also 18926 line | |
| 42861 | UBA52 | NM_003333.2 | 251 | gcgcctgcgaggtggcattattg | 286154 | - | see also 18926 line | |
| 42862 | 76P | NM_014444.2 | 377 | gccggctcggcacagactatatt | 286159 | - | see also 5676 line | |
| 42863 | 76P | NM_014444.2 | 376 | tgccggctcggccacagactatat | 286158 | - | see also 5676 line | |
| 42864 | ACTA1 | NM_001100.2 | 587 | accacacaagtgccattatga | 286206 | transcription co-repressor | - | nemaline myopathy |
| 42865 | ACTA2 | NM_001613.1 | 797 | caccatcggaaatgaacgtttcc | 286210 | - | see also 18928 line | |
| 42866 | ACTA2 | NM_001613.1 | 642 | ggctattccttgttactactgc | 286208 | - | see also 18928 line | |
| 42867 | ACTC | NM_005159.2 | 157 | gactcctacgtaggttgatgaagc | 286211 | - | see also 3626 line | |
| 42868 | ACTC | NM_005159.2 | 442 | ggccgtaccacaggccattgttct | 286212 | - | see also 3626 line | |
| 42869 | ACTG2 | NM_001615.2 | 945 | gacatccgtaaggacttatatgc | 286224 | - | see also 18930 line | |
| 42870 | ACTG2 | NM_001615.2 | 885 | ggcatggagtccgctggaattca | 286222 | - | see also 18930 line | |
| 42871 | ACTL7A | NM_006687.2 | 682 | cccatctacgagggttatccttt | 286234 | - | see also 4721 line | |
| 42872 | ACTL7A | NM_006687.2 | 227 | tgggcactggctactgtaaatgt | 286227 | - | see also 4721 line | |
| 42873 | ACTL7B | NM_006686.2 | 273 | cacccgcaagtggacttttagtgg | 286242 | - | see also 18932 line | |
| 42874 | ACTL7B | NM_006686.2 | 853 | gacggcaaactcatcactcattgg | 286246 | - | see also 18932 line | |
| 42875 | ACTL7B | NM_006686.2 | 271 | gacacccgcaagtgggactttagt | 286241 | - | see also 18932 line | |
| 42876 | ACTR10 | NM_018477.1 | 339 | cgccgagttgtgattatcgaatc | 286257 | - | see also 7492 line | |
| 42877 | ACTR10 | NM_018477.1 | 340 | gccgagttgtgattatcgaatcg | 286258 | - | see also 7492 line | |
| 42878 | ACTR1A | NM_005736.2 | 422 | aacgagctgcggaagttttcttc | 286302 | - | see also 3980 line | |
| 42879 | ACTR1A | NM_005736.2 | 929 | ggcgcacgcttttcctaacatt | 286310 | - | see also 3980 line | |
| 42880 | ACTR1B | NM_005735.2 | 368 | cccgctcaaccgagtaagaaac | 286321 | - | see also 18935 line | |
| 42881 | ACTR1B | NM_005735.2 | 369 | ccgctcaaaccgagtaagaacc | 286322 | - | see also 18935 line | |

Figure 46

| | | | | | | | | structural constituent of cytoskeleton |
|---|---|---|---|---|---|---|---|---|
| | | | | | | - | - | - |
| 42882 | ACTR2 | NM_005722.1 | 1066 | aggggtgatrggaaaaacttct | 286336 | - | - | |
| 42883 | ACTR3 | NM_005721.3 | 1121 | ttctatcgatgttggttatgaga | 286368 | - | see also 18936 line | |
| 42884 | ACTR3 | NM_005721.3 | 804 | acgttgaccgtacggtaataga | 286351 | - | see also 18936 line | |
| 42885 | ACTR3 | NM_005721.3 | 548 | gccaatccgccatggtatagttg | 286345 | - | see also 18936 line | |
| 42886 | ACTR6 | NM_022496.2 | 63 | gacgaccttagtgctggataatg | 286376 | - | see also 8419 line | |
| 42887 | ACTR6 | NM_022496.2 | 126 | gtcgttattcctaattgtcagt | 286383 | - | see also 8419 line | |
| 42888 | ACTR8 | NM_022899.3 | 692 | atgtctcatgcgatacaaaaat | 286435 | - | see also 8520 line | |
| 42889 | ACTR8 | NM_022899.3 | 978 | ctggcatacggaggatctgatgt | 286445 | - | see also 8520 line | |
| 42890 | ACTRT1 | NM_138289.1 | 967 | gtgccgaggttcttttgcacc | 286490 | - | see also 18939 line | |
| 42891 | ACTRT1 | NM_138289.1 | 822 | ggccgtggtaaataacatcaaag | 286488 | - | see also 18939 line | |
| 42892 | ANK2 | NM_001148.2 | 1140 | tggccactaccgtgtaaccaaac | 286569 | - | see also 730 line | |
| 42893 | ANK2 | NM_001148.2 | 4284 | accttgccgacgactatcattta | 286632 | - | see also 730 line | |
| 42894 | ARPC1B | NM_005720.2 | 272 | ccccgagagtaaccgtattgtga | 286812 | - | see also 18941 line | |
| 42895 | ARPC1B | NM_005720.2 | 579 | ttcaagtgtcgatcttttcagc | 286818 | - | see also 18941 line | |
| 42896 | ARPC2 | NM_005731.2 | 948 | atcaccgggaagacgtttcatc | 286843 | - | see also 18942 line | |
| 42897 | ARPC2 | NM_005731.2 | 186 | ttcgatggggtcctctatcatat | 286825 | - | see also 18942 line | |
| 42898 | ARPC4 | NM_005718.2 | 393 | gcctgtggaggggtatgatatca | 286850 | - | see also 3969 line | |
| 42899 | ARPC4 | NM_005718.2 | 392 | agcctgtgagggggtatgatatc | 286849 | - | see also 3969 line | |
| 42900 | ARPC5 | NM_005717.2 | 493 | atggtgtggatctcctaatgaag | 286853 | - | see also 18945 line | |
| 42901 | ARPC5 | NM_005717.2 | 496 | gtgtggatctcctaatgaagtat | 286855 | - | see also 18945 line | |
| 42902 | ARPM1 | NM_032487.2 | 1295 | gacaagcggttagttaaggatat | 286876 | - | see also 18946 line | |
| 42903 | ARPM1 | NM_032487.2 | 1055 | gccaagaaacccgattgtctaga | 286871 | - | see also 18946 line | |
| 42904 | ARPM2 | NM_080431.3 | 916 | tgcccgacgggaacatcatcagc | 286884 | - | - | |
| 42905 | ARPM2 | NM_080431.3 | 815 | gggtctcgtgggacgaatcaaaa | 286881 | - | see also 42904 line | |
| 42906 | ARPM2 | NM_080431.3 | 913 | agctgcccgacgggaaacatcatc | 286883 | - | see also 42904 line | |
| 42907 | BFSP1 | NM_001195.2 | 1439 | tggacccctagattctatgtctcc | 286902 | - | see also 18947 line | |
| 42908 | BFSP1 | NM_001195.2 | 361 | gacgagttccgaagcaagtatga | 286885 | - | see also 18947 line | |
| 42909 | BFSP2 | NM_003571.2 | 770 | aagtcattgatgaggctaatttg | 286921 | - | see also 18948 line | |
| 42910 | BFSP2 | NM_003571.2 | 805 | ctggagagtcaaatagaaagtct | 286922 | - | see also 18948 line | |
| 42911 | CD2AP | NM_012120.1 | 971 | ctggtggaggggcgaacttaatg | 286950 | - | see also 5143 line | |
| 42912 | CD2AP | NM_012120.1 | 972 | tggttggaggggcgaacttaatgg | 286951 | - | see also 5143 line | |
| 42913 | CTNNA2 | NM_004389.1 | 735 | tacgagagccaaccgagattatg | 286989 | - | see also 2951 line | |
| 42914 | CTNNA2 | NM_004389.1 | 740 | gagccaacgagattatgtgttc | 286991 | - | see also 2951 line | |
| 42915 | CYLC2 | NM_001340.1 | 169 | gacaacacgtttcctataattga | 287046 | - | see also 18951 line | |
| 42916 | CYLC2 | NM_001340.1 | 164 | tacgggacaacacgtttctcata | 287044 | - | see also 18951 line | |
| 42917 | D10S170 | NM_005436.1 | 1462 | atgcatgagctagttctgaattt | 287078 | - | see also 18952 line | |

Figure 46

| ID | Gene | Accession | Num | Sequence | ID2 | | see also | Function | Disease |
|---|---|---|---|---|---|---|---|---|---|
| 42918 | D10S170 | NM_005436.1 | 1709 | tggaaccttcgctgtttggtatt | 287088 | - | see also 18952 line | | |
| 42919 | DES | NM_001927.2 | 927 | atcgcggctaagaacatttctga | 287099 | - | see also 1229 line | | |
| 42920 | DES | NM_001927.2 | 924 | accatgccggctaagaacatttc | 287098 | - | see also 1229 line | | |
| 42921 | DSP | NM_004415.1 | 7366 | cacggtattgcttattagaagc | 287271 | - | see also 18953 line | | |
| 42922 | DSP | NM_004415.1 | 2013 | gacgatagccgacctrgagttac | 287134 | - | see also 18953 line | | |
| 42923 | FLJ12785 | NM_024855.2 | 811 | atggcggtgtctgattattatg | 287323 | - | see also 8793 line | | |
| 42924 | GFAP | NM_002055.2 | 777 | gagtgttaccgctccaagtttgc | 287339 | - | see also 18955 line | | |
| 42925 | GFAP | NM_002055.2 | 595 | aggaaggagatccggttcttgagg | 287338 | - | see also 18955 line | | |
| 42926 | INA | NM_032727.2 | 1251 | aaggcgaggagacacgttttagc | 287342 | - | see also 18956 line | | |
| 42927 | INA | NM_032727.2 | 1419 | aggtagcctcttaagaaaacctcc | 287350 | - | see also 18956 line | | |
| 42928 | KRT1 | NM_006121.2 | 697 | aggtagatacctccactagaacc | 287354 | - | see also 18957 line | | |
| 42929 | KRT1 | NM_006121.2 | 1265 | gagacttagatctgaaatcgaca | 287371 | - | see also 18957 line | | |
| 42930 | KRT13 | NM_002274.2 | 559 | atcctgaccgccaccattgaaaa | 287375 | - | see also 18958 line | | |
| 42931 | KRT13 | NM_002274.2 | 205 | ggcgtgagctgtgttttgctgg | 287374 | - | see also 18958 line | | |
| 42932 | KRT15 | NM_002275.2 | 1358 | agcagcaatttccacatcaatgt | 287381 | - | - | structural constituent of cytoskeleton | - |
| 42933 | KRT20 | NM_019010.1 | 383 | gtcgcgactacagtgcatattac | 287388 | - | see also 18959 line | | |
| 42934 | KRT20 | NM_019010.1 | 459 | tcggtgtgtcctgcaattgata | 287392 | - | see also 18959 line | | |
| 42935 | KRT2A | NM_000423.1 | 583 | gagcgtgagcagatcaaaactct | 287415 | - | see also 18960 line | | |
| 42936 | KRT2A | NM_000423.1 | 534 | gcctctcaactgaaagttgacc | 287414 | - | see also 18960 line | | |
| 42937 | KRT5 | NM_000424.2 | 1349 | ctgagagccgagattgacaatgt | 287427 | - | see also 18961 line | | |
| 42938 | KRT6A | NM_005554.2 | 528 | ggctgaggagctgaaacagatca | 287429 | - | - | structural constituent of cytoskeleton | breast cancer, pachyonychia congenita |
| 42939 | KRT9 | NM_000226.1 | 1332 | cactgacgtccggcaagagatcg | 287445 | - | see also 18962 line | | |
| 42940 | KRT9 | NM_000226.1 | 365 | ctggaggaggctatagtagttct | 287431 | - | see also 18962 line | | |
| 42941 | KRTHA1 | NM_002277.2 | 356 | cagttaccagtcctattttaaga | 287448 | - | - | structural constituent of cytoskeleton | - |
| 42942 | LOC81569 | NM_030812.1 | 1085 | ccctctatcccgggttcacaaag | 287457 | - | see also 18963 line | | |
| 42943 | LOC81569 | NM_030812.1 | 501 | gcctgccgaccgaaagaagatgc | 287452 | - | see also 18963 line | | |
| 42944 | LOR | NM_000427.1 | 282 | ctccggtgggacgtcaagtact | 287463 | - | see also 18964 line | | |
| 42945 | MSN | NM_002444.1 | 1806 | agcgcattgacgaattgagtct | 287504 | - | see also 1580 line | | |
| 42946 | MSN | NM_002444.1 | 478 | ctcgtatgctgtccagtctaagt | 287493 | - | see also 1580 line | | |
| 42947 | NEF3 | NM_005382.1 | 762 | cacggtggagcgcaaagactacc | 287507 | - | see also 18966 line | | |
| 42948 | NEF3 | NM_005382.1 | 1276 | cacgacccccaatcaccaatatc | 287514 | - | see also 18966 line | | |
| 42949 | NEFL | NM_006158.1 | 646 | agcttgatggacgaaatctcttt | 287525 | - | see also 18967 line | | |
| 42950 | NEFL | NM_006158.1 | 1096 | atggcacgatacctaaaagaata | 287532 | - | see also 18967 line | | |
| 42951 | PPL | NM_002705.2 | 3481 | agcgatctcaccgccaatatga | 287545 | - | see also 18968 line | | |

Figure 46

| | | | | | | | | | |
|---|---|---|---|---|---|---|---|---|---|
| 42952 | PPL | NM_002705.2 | 1750 | aacgagctactgcggattgaacc | 287536 | - | see also 18968 line | | |
| 42953 | PPL | NM_002705.2 | 3869 | gactgatcgaaaggtgtgattta | 287548 | - | see also 18968 line | | |
| 42954 | SCAM-1 | NM_005775.2 | 2119 | ttcggaacgttccctggaaatta | 287568 | - | see also 18969 line | | |
| 42955 | SCAM-1 | NM_005775.2 | 753 | acctagaagcaccttcaactaca | 287559 | - | see also 18969 line | | |
| 42956 | VIM | NM_003380.1 | 1447 | tcctgattaagacggttgaaact | 287581 | - | see also 18970 line | | |
| 42957 | VIM | NM_003380.1 | 1419 | ctggttgatacccactcaaaaag | 287579 | - | see also 18970 line | | |
| 42958 | ELN | NM_000501.1 | 241 | gccttccccgcagttacctttcc | 287588 | - | - | extracellular matrix structural constituent | atherosclerosis、hypercalcemia、supravalvular aortic stenosis、Williams-Beuren syndrome |
| 42959 | ELN | NM_000501.1 | 240 | cgccttccccgcagttacctttc | 287587 | - | see also 42958 line | | |
| 42960 | ELN | NM_000501.1 | 850 | ggcgtgcctggggcaattcctgg | 287589 | - | see also 42958 line | | |
| 42961 | COL11A2 | NM_080679.1 | 2328 | cccaaggggtccctaggatttcc | 287596 | - | see also 18971 line | | |
| 42962 | COL11A2 | NM_080679.1 | 4930 | cggagactagcccctatgtcaaa | 287600 | - | see also 18971 line | | |
| 42963 | BGN | NM_001711.3 | 525 | ccctcgtcctggtgaacaacaag | 287618 | - | see also 1139 line | | |
| 42964 | BGN | NM_001711.3 | 258 | tggacgatgggccattcatgatg | 287616 | - | see also 1139 line | | |
| 42965 | CHAD | NM_001267.1 | 953 | cacgctgaaacacgtccatttgg | 287624 | - | see also 18973 line | | |
| 42966 | CHAD | NM_001267.1 | 439 | gcctcaagcaacttatctacttg | 287623 | - | see also 18973 line | | |
| 42967 | COL10A1 | NM_000493.2 | 228 | tccctacaccataaagagtaaag | 287627 | - | see also 386 line | | |
| 42968 | COL11A1 | NM_001854.2 | 3241 | accggtgagactggtccaatagg | 287683 | - | see also 1198 line | | |
| 42969 | COL11A1 | NM_001854.2 | 1781 | ctcctggtactatgttgatgtta | 287676 | - | see also 1198 line | | |
| 42970 | COL12A1 | NM_004370.4 | 4758 | ctgcacgacctcactagtgaacc | 287813 | - | see also 2929 line | | |
| 42971 | COL12A1 | NM_004370.4 | 4339 | ctgacagtgtggatcgatataag | 287803 | - | see also 2929 line | | |
| 42972 | COL15A1 | NM_001855.2 | 4269 | ctgtgctaatcggctaattgtcc | 287960 | - | see also 18977 line | | |
| 42973 | COL15A1 | NM_001855.2 | 3910 | acctaccgagcattcttatcttc | 287945 | - | see also 18977 line | | |
| 42974 | COL16A1 | NM_001856.2 | 2503 | ggcgtgggccgacctggtaaacc | 287976 | - | see also 1199 line | | |
| 42975 | COL16A1 | NM_001856.2 | 298 | ttcggccatggggcaaatacagg | 287966 | - | see also 1199 line | | |
| 42976 | COL18A1 | NM_030582.2 | 3789 | cccacgagggacggacaatgaag | 288000 | - | see also 18979 line | | |
| 42977 | COL18A1 | NM_030582.2 | 1036 | gtcggccagtggacacacttagc | 287994 | - | see also 18979 line | | |
| 42978 | COL18A1 | NM_030582.2 | 3111 | tccagggactcctgtttacgaca | 287997 | - | see also 18979 line | | |
| 42979 | COL19A1 | NM_001858.2 | 1654 | ccccggaccccctggtttaatagg | 288029 | - | see also 1201 line | | |
| 42980 | COL19A1 | NM_001858.2 | 1051 | gagccgggtgaaaatggtttaca | 288024 | - | see also 1201 line | | |
| 42981 | COL1A1 | NM_000088.2 | 3759 | cgctactaccgggctgatgatgc | 288050 | - | see also 18981 line | | |
| 42982 | COL1A1 | NM_000088.2 | 227 | accaatcacctgcgtacagaacg | 288046 | - | see also 18981 line | | |
| 42983 | COL1A2 | NM_000089.2 | 2979 | cgcggttaccctggcaatattgg | 288064 | - | see also 67 line | | |
| 42984 | COL1A2 | NM_000089.2 | 3467 | tggttacgatggagacttctaca | 288066 | - | see also 67 line | | |
| 42985 | COL2A1 | NM_001844.3 | 1376 | tccggtaaccctggaacagatgg | 288114 | - | see also 18984 line | | |
| 42986 | COL2A1 | NM_001844.3 | 4419 | tccgggcagagggcaatagcagg | 288119 | - | see also 18984 line | | |
| 42987 | COL3A1 | NM_000090.2 | 3756 | tggcggttttgccccgtattatg | 288136 | - | see also 18985 line | | |

EP 1 752 536 A1

Figure 46

| | | | | | | | |
|---|---|---|---|---|---|---|---|
| 42988 | COL3A1 | NM_000090.2 | 3757 | ggcggtttgcccgtattatgg | 288137 | - | see also 18985 line |
| 42989 | COL3A1 | NM_000090.2 | 3879 | tcgtaaaaacccgctagaaact | 288141 | - | see also 18985 line |
| 42990 | COL4A1 | NM_001845.2 | 1048 | cccgggtacccaggactcattagg | 288153 | - | see also 18986 line |
| 42991 | COL4A1 | NM_001845.2 | 4858 | ctgttgatcggctactcttttgt | 288178 | - | see also 18986 line |
| 42992 | COL4A1 | NM_001845.2 | 4860 | gttggatcggctactcttttgtga | 288179 | - | see also 18986 line |
| 42993 | COL4A2 | NM_001846.1 | 3562 | gacatcggggacactataaattt | 288199 | - | see also 18987 line |
| 42994 | COL4A2 | NM_001846.1 | 5244 | cacaccattcatcgaatgcaatg | 288204 | - | see also 18987 line |
| 42995 | COL4A4 | NM_000092.2 | 719 | tccctgtcacttatactcattc | 288209 | - | see also 18988 line |
| 42996 | COL4A4 | NM_000092.2 | 5117 | gactgggtatagtctgttatacc | 288233 | - | see also 18988 line |
| 42997 | COL4A5 | NM_000495.3 | 224 | ctggctgccggcttgttcttact | 288244 | - | see also 388 line |
| 42998 | COL4A5 | NM_000495.3 | 226 | ggctgccggcttgttcttactgg | 288245 | - | see also 388 line |
| 42999 | COL4A6 | NM_001847.1 | 317 | tactggtttatcgggattgaaag | 288276 | - | see also 1195 line |
| 43000 | COL4A6 | NM_001847.1 | 3668 | tggcactccaggacctagtatca | 288307 | - | see also 1195 line |
| 43001 | COL5A2 | NM_000393.2 | 3997 | cagccccgatggctcgaaaaagc | 288341 | - | see also 315 line |
| 43002 | COL5A2 | NM_000393.2 | 3455 | ccgggttctgggggtcctatagg | 288336 | - | see also 315 line |
| 43003 | COL5A3 | NM_015719.2 | 3336 | cccggaacaaggggagtaaagg | 288363 | - | see also 18992 line |
| 43004 | COL5A3 | NM_015719.2 | 5192 | gacgaagaccctttcgaattca | 288375 | - | see also 18992 line |
| 43005 | COL6A2 | NM_001849.2 | 2284 | tcgggacgatgacctcaacttgc | 288386 | - | see also 18993 line |
| 43006 | COL6A2 | NM_001849.2 | 160 | cactaccgagagaaacaacaact | 288382 | - | see also 18993 line |
| 43007 | COL6A3 | NM_004369.1 | 7517 | tgcgagatgtgttcttgagtatt | 288513 | - | see also 18994 line |
| 43008 | COL6A3 | NM_004369.1 | 7364 | ggggcgactccatcgatcaatgt | 288510 | - | see also 18994 line |
| 43009 | COL7A1 | NM_000094.2 | 1420 | cccgtggctaccggttggaatgg | 288550 | - | see also 18995 line |
| 43010 | COL7A1 | NM_000094.2 | 195 | cgcctttacgccgctgacattgt | 288541 | - | see also 18995 line |
| 43011 | COL7A1 | NM_000094.2 | 2999 | ctcgatcgactccgtgactttgg | 288555 | - | see also 18995 line |
| 43012 | COL8A1 | NM_001850.3 | 2306 | cacgtacgacgagtaccaaaaagg | 288588 | - | see also 18996 line |
| 43013 | COL8A1 | NM_001850.3 | 2304 | tacacgtacgacgagtaccaaaaa | 288587 | - | see also 18996 line |
| 43014 | COL8A1 | NM_001850.3 | 496 | cacacctacccaatatatgaag | 288572 | - | see also 18996 line |
| 43015 | COL9A1 | NM_001851.2 | 2414 | accgacagatcagcacattaagc | 288619 | - | see also 1197 line |
| 43016 | COL9A1 | NM_001851.2 | 2875 | ctggtcagcgagcatttaacaaa | 288623 | - | see also 1197 line |
| 43017 | COL9A2 | NM_001852.3 | 128 | ctcgctctggcgcagattagagg | 288626 | - | see also 18998 line |
| 43018 | COL9A2 | NM_001852.3 | 126 | tgctcgctctggcgcagattaga | 288625 | - | see also 18998 line |
| 43019 | COL9A2 | NM_001853.2 | 308 | cccgggattgatggtttaactgg | 288628 | - | see also 18998 line |
| 43020 | COL9A3 | NM_001853.2 | 1621 | gggatgatcagcgaacaaattgc | 288634 | - | see also 18999 line |
| 43021 | COL9A3 | NM_001853.2 | 1610 | agctgtgtgggggatgatcagc | 288632 | - | see also 18999 line |
| 43022 | KAL1 | NM_000216.1 | 1071 | gagtgtgaccgtcactactagttt | 288651 | - | see also 19000 line |
| 43023 | KAL1 | NM_000216.1 | 1509 | cactgtcaacgatatcatgtgc | 288663 | - | see also 19000 line |
| 43024 | LAMA4 | NM_002290.2 | 1891 | acctgctctaacctttcagaac | 288725 | - | see also 19001 line |

Figure 46

| 43025 | LAMA4 | NM_002290.2 | 784 | aagatgtgctcccggttactatg | 288700 | - | see also 19001 line |
|---|---|---|---|---|---|---|---|
| 43026 | LUM | NM_002345.2 | 832 | ttgcagtatctgcgtttatctca | 288858 | - | see also 19002 line |
| 43027 | LUM | NM_002345.2 | 144 | gagtctaagtgcatttactctct | 288839 | - | see also 19002 line |
| 43028 | MAGP2 | NM_003480.1 | 543 | caccagtttacgacgtatgtaca | 288872 | - | see also 19003 line |
| 43029 | MAGP2 | NM_003480.1 | 660 | gagactccgtcgctccaattact | 288877 | - | see also 19003 line |
| 43030 | MATN1 | NM_002379.2 | 1688 | aagacctcggctttaagatgttt | 288891 | - | see also 1532 line |
| 43031 | MATN1 | NM_002379.2 | 1690 | gacctcggctttaagatgtttgc | 288892 | - | see also 1532 line |
| 43032 | MATN3 | NM_002381.2 | 600 | ggctcgagagccctcttctaaca | 288898 | - | see also 19005 line |
| 43033 | MATN3 | NM_002381.2 | 1429 | gacaaggtcagctcgtatcttca | 288915 | - | see also 19005 line |
| 43034 | MEPE | NM_020203.1 | 761 | ggcagcggttatacagatcttca | 288952 | - | see also 19006 line |
| 43035 | MEPE | NM_020203.1 | 817 | tggggacggccaaccttttaagg | 288955 | - | see also 19006 line |
| 43036 | MFAP1 | NM_005926.1 | 415 | gagattggctcgacatcgaaaaa | 288984 | - | see also 19007 line |
| 43037 | MFAP1 | NM_005926.1 | 421 | ggctcgacatcgaaaaatagtgg | 288986 | - | see also 19007 line |
| 43038 | MFAP1 | NM_005926.1 | 419 | ttggctcgacatcgaaaaatagt | 288985 | - | see also 19007 line |
| 43039 | MFAP2 | NM_002403.2 | 476 | tccgccgtgtgtacgtcattaac | 289011 | - | see also 19008 line |
| 43040 | MFAP2 | NM_002403.2 | 223 | atcgacaacccagactactatga | 289006 | - | see also 19008 line |
| 43041 | MFAP3 | NM_005927.3 | 849 | ttcgtcacctctccaattcgtgc | 289028 | - | see also 19009 line |
| 43042 | MFAP3 | NM_005927.3 | 880 | ctgtcaccctacgtgttatcttc | 289030 | - | see also 19009 line |
| 43043 | OPTC | NM_014359.2 | 524 | ctctgtgtattgcgatgacattg | 289059 | - | see also 19010 line |
| 43044 | OPTC | NM_014359.2 | 678 | ctcatttcctccatcgataatga | 289063 | - | see also 19010 line |
| 43045 | OPTC | NM_014359.2 | 291 | gtcattgacctgagcaactatga | 289055 | - | see also 19010 line |
| 43046 | PRELP | NM_002725.2 | 883 | gaccatccctaacggatacttca | 289072 | - | see also 19011 line |
| 43047 | PRELP | NM_002725.2 | 922 | tgccttcattcggcttaactaca | 289075 | - | see also 19011 line |
| 43048 | TECTA | NM_005422.1 | 4355 | tccgtcgcaacgtgattcagtgc | 289143 | - | see also 3787 line |
| 43049 | TECTA | NM_005422.1 | 678 | aaccccgagatcgtgaatatcc | 289100 | - | see also 3787 line |
| 43050 | TFPI2 | NM_006528.2 | 464 | gtcaccggaaccggattgagaac | 289210 | - | see also 19013 line |
| 43051 | TFPI2 | NM_006528.2 | 580 | gccaatgtgactcgctattattt | 289213 | - | see also 19013 line |
| 43052 | TFPI2 | NM_006528.2 | 579 | tgccaatgtgactcgctattatt | 289212 | - | see also 19013 line |
| 43053 | TNR | NM_003285.1 | 2758 | tactaccgagtatcatatcgacc | 289262 | - | see also 2220 line |
| 43054 | TNR | NM_003285.1 | 2222 | accactaccgaattacctttacc | 289245 | - | see also 2220 line |
| 43055 | MYBPH | NM_004997.1 | 1129 | tgccaaacctaaagggtttattg | 289292 | - | see also 3492 line |
| 43056 | MYBPH | NM_004997.1 | 116 | cccggagaagtggcagtatcaga | 289289 | - | see also 3492 line |
| 43057 | MYOM1 | NM_003803.1 | 1429 | ttgatcgaaggtcgttcctatat | 289329 | - | see also 2600 line |
| 43058 | MYOM1 | NM_003803.1 | 2329 | aactatcgcgaggtcattgatgg | 289344 | - | see also 2600 line |
| 43059 | MYOM2 | NM_003970.1 | 1749 | cccgagatatgccgtgtttgacc | 289418 | - | see also 19017 line |
| 43060 | MYOM2 | NM_003970.1 | 1939 | cagtgggaccgacctaagcatga | 289421 | - | see also 19017 line |
| 43061 | TTID | NM_006790.1 | 1641 | tacgggttcgaccaacattcagc | 289503 | - | see also 4781 line |

Figure 46

| 43062 | TTID | NM_006790.1 | 1454 | aacactgaccgaataagcttata | 289497 | - | see also 4781 line |
|---|---|---|---|---|---|---|---|
| 43063 | TTID | NM_006790.1 | 620 | tccgctatggattccaactatca | 289473 | - | see also 4781 line |
| 43064 | CRYAA | NM_000394.2 | 151 | ggcgagggccttttgagtatga | 289511 | - | see also 316 line |
| 43065 | CRYAA | NM_000394.2 | 149 | tcggcgagggcctttgagtat | 289509 | - | see also 316 line |
| 43066 | CRYAB | NM_001885.1 | 408 | tagaccctctcaccattacttca | 289517 | - | see also 1208 line |
| 43067 | CRYBA1 | NM_005208.3 | 616 | gacttcgcagatccaatcgattc | 289540 | - | see also 3659 line |
| 43068 | CRYBA1 | NM_005208.3 | 528 | cttgatatcgtgggtatcagtat | 289536 | - | see also 3659 line |
| 43069 | CRYBA2 | NM_005209.1 | 502 | accgaggctaccagtatgttg | 289544 | - | see also 19023 line |
| 43070 | CRYBA2 | NM_005209.1 | 187 | gcgtttgggtggcctttgagtac | 289542 | - | see also 19023 line |
| 43071 | CRYBA4 | NM_001886.1 | 498 | tccgggctaccgaggatttcagt | 289551 | - | see also 19024 line |
| 43072 | CRYBA4 | NM_001886.1 | 398 | agctgagcgatgactatccttcc | 289550 | - | see also 19024 line |
| 43073 | CRYBA4 | NM_001886.1 | 502 | ggctaccgaggatttcagtatgt | 289554 | - | see also 19024 line |
| 43074 | CRYBB1 | NM_001887.3 | 335 | gaccgtgtgcgcagcatcattgt | 289560 | - | see also 19025 line |
| 43075 | CRYBB1 | NM_001887.3 | 339 | gtgtgcgcagcatcattgtctcc | 289561 | - | see also 19025 line |
| 43076 | CRYBB3 | NM_004076.3 | 382 | ctccggcctcgaatattgatag | 289568 | - | see also 19026 line |
| 43077 | CRYBB3 | NM_004076.3 | 447 | tggccgcaagatggagatagtgg | 289570 | - | see also 19026 line |
| 43078 | CRYGA | NM_014617.2 | 334 | gtccaatctgccgtataattcc | 289577 | - | see also 19027 line |
| 43079 | CRYGA | NM_014617.2 | 611 | gacggtcaccgatttgtactaa | 289586 | - | see also 19027 line |
| 43080 | CRYGB | NM_005210.2 | 536 | ctcttagacgagtcatggatttg | 289594 | - | see also 19028 line |
| 43081 | CRYGB | NM_005210.2 | 542 | gacgagtcatggatttgtactga | 289596 | - | see also 19028 line |
| 43082 | CRYGD | NM_006891.2 | 357 | accgcttccgcttcaatgaaatc | 289599 | - | see also 19029 line |
| 43083 | CRYGD | NM_006891.2 | 356 | gaccgcttccgcttcaatgaaat | 289598 | - | see also 19029 line |
| 43084 | CRYGS | NM_017541.2 | 315 | tgcctagtggaggccagtataag | 289610 | - | see also 19030 line |
| 43085 | CRYGS | NM_017541.2 | 452 | gagggtgtctggattttctatga | 289613 | - | see also 19030 line |
| 43086 | LIM2 | NM_030657.2 | 604 | gacgttcttcgcagggattttct | 289615 | - | see also 19031 line |
| 43087 | AMBN | NM_016519.2 | 180 | aacaccggtatggctagtttga | 289617 | - | see also 19032 line |
| 43088 | AMBN | NM_016519.2 | 796 | atgttgtacgtgccttttggagc | 289626 | - | see also 19032 line |
| 43089 | AMBN | NM_016519.2 | 727 | ttccaaatagccccgttttgatttc | 289620 | - | see also 19032 line |
| 43090 | ENAM | NM_031889.1 | 604 | cagcacccaaacgtcataacaag | 289649 | - | see also 9042 line |
| 43091 | ENAM | NM_031889.1 | 2507 | gagcaggggctactacgttaata | 289705 | - | see also 9042 line |
| 43092 | TUFT1 | NM_020127.1 | 834 | gaggaacttcggagcaacaatgc | 289740 | - | see also 19034 line |
| 43093 | TUFT1 | NM_020127.1 | 960 | cggaaagtccggcaaatgataga | 289743 | - | see also 19034 line |
| 43094 | KRTHA6 | NM_003771.3 | 1230 | cacggcatgcaagcctgttatta | 289752 | - | see also 19035 line |
| 43095 | KRTHA6 | NM_003771.3 | 1231 | acggcatgcaagcctgttattag | 289753 | - | see also 19035 line |
| 43096 | KRTHB2 | NM_033033.3 | 501 | accgtttcgcatctttcatcaac | 289756 | - | see also 19036 line |
| 43097 | KRTHB2 | NM_033033.3 | 1155 | agcaagaaaaccgagaatgtcaaa | 289761 | - | see also 19036 line |
| 43098 | KRTHB4 | NM_033045.2 | 945 | aacgctttaactgaggagaaatcc | 289764 | - | see also 19037 line |

Figure 46

| | | | | | | | | |
|---|---|---|---|---|---|---|---|---|
| 43099 | KRTHB4 | NM_033045.2 | 182 | ggggccttggcagctttggtagt | 289762 | - | see also 19037 line | |
| 43100 | AF053356_CDS3 | NM_023948.3 | 656 | tggtgggacgcaaggacattacc | 289769 | - | see also 19038 line | |
| 43101 | AF053356_CDS3 | NM_023948.3 | 544 | ccccagtcttgcattgacattgt | 289768 | - | see also 19038 line | |
| 43102 | ARVCF | NM_001670.1 | 2906 | agccttgagggcgagaaaactgg | 289773 | - | see also 19039 line | |
| 43103 | ARVCF | NM_001670.1 | 2776 | gcgtggtaccttgcagaaagatg | 289772 | - | see also 19039 line | |
| 43104 | BFAR | NM_016561.1 | 826 | aagacgccctataccatagaaaa | 289784 | - | see also 6914 line | |
| 43105 | BFAR | NM_016561.1 | 823 | tccaagacgccctataccataga | 289783 | - | see also 6914 line | |
| 43106 | BFAR | NM_016561.1 | 828 | gacgccctataccatagaaaaca | 289785 | - | see also 6914 line | |
| 43107 | C21orf29 | NM_144991.2 | 1110 | agccacatccgccgtctacaagt | 289802 | - | see also 19041 line | |
| 43108 | C21orf29 | NM_144991.2 | 827 | aagataacgaggtgctaaaatat | 289797 | - | see also 19041 line | |
| 43109 | CAV1 | NM_001753.3 | 279 | atgtctgggggcaaatacgtaga | 289810 | - | see also 1158 line | |
| 43110 | CAV1 | NM_001753.3 | 548 | caccttcactgtgacgaaatact | 289816 | - | see also 1158 line | |
| 43111 | CLDN1 | NM_021101.3 | 630 | cagcatggtatggcaatagaatc | 289823 | - | see also 19043 line | |
| 43112 | CLDN1 | NM_021101.3 | 600 | ttcttgcaggtctggctatttta | 289822 | - | see also 19043 line | |
| 43113 | CLDN10 | NM_006984.3 | 472 | atcacaacggaattctttgatcc | 289838 | - | see also 19044 line | |
| 43114 | CLDN10 | NM_006984.3 | 651 | gtcttctcggacaaagtatcatg | 289847 | - | see also 19044 line | |
| 43115 | CLDN11 | NM_005602.4 | 522 | agcccggtgtggctaagtacagg | 289863 | - | - | structural molecule | - |
| 43116 | CLDN12 | NM_012129.2 | 391 | gcctgatgtacgacacttctggg | 289866 | - | see also 19045 line | |
| 43117 | CLDN12 | NM_012129.2 | 826 | cccatccacccagtatgcatact | 289873 | - | see also 19045 line | |
| 43118 | CLDN14 | NM_144492.1 | 1505 | caccagctgcctacaaagacaat | 289877 | - | see also 19046 line | |
| 43119 | CLDN15 | NM_014343.1 | 257 | gtcgatggctgtggaaacctttg | 289878 | - | see also 19047 line | |
| 43120 | CLDN17 | NM_012131.1 | 271 | tccttgatcgccctgcttattgg | 289892 | - | see also 19048 line | |
| 43121 | CLDN17 | NM_012131.1 | 179 | tccggttgcaatgcaagttctat | 289888 | - | see also 19048 line | |
| 43122 | CLDN18 | NM_016369.1 | 751 | gaggacgaggtacaatcttatcc | 289913 | - | see also 19049 line | |
| 43123 | CLDN18 | NM_016369.1 | 622 | aactacaaagccgtttcttatca | 289909 | - | see also 19049 line | |
| 43124 | CLDN19 | NM_148960.1 | 295 | acgcaggcgacgccatcatcact | 289916 | - | see also 19050 line | |
| 43125 | CLDN2 | NM_020384.2 | 780 | gtgcctgacagcatgaaatttga | 289919 | - | see also 19051 line | |
| 43126 | CLDN2 | NM_020384.2 | 778 | tggtgcctgacagcatgaaattt | 289918 | - | see also 19051 line | |
| 43127 | CLDN5 | NM_003277.2 | 1094 | caccggcgactacgacaagaaga | 289924 | - | see also 19052 line | |
| 43128 | CLDN5 | NM_003277.2 | 832 | gaggcgtgctctacctgttttgc | 289922 | - | see also 19052 line | |
| 43129 | CLDN7 | NM_001307.3 | 660 | ggccactcgagccctaatggtgg | 289928 | - | see also 19054 line | |
| 43130 | CLDN7 | NM_001307.3 | 883 | atccctaccaacattaagtatga | 289933 | - | see also 19054 line | |
| 43131 | CLDN7 | NM_001307.3 | 838 | tcctggtatggccatcagattgt | 289932 | - | see also 19054 line | |
| 43132 | CLDN8 | NM_012132.3 | 566 | gtgaatgttgcccaaaaacgtga | 289947 | - | see also 19055 line | |
| 43133 | CLDN8 | NM_012132.3 | 465 | tgctgacggctggaatcatcttc | 289944 | - | see also 19055 line | |

Figure 46

| | | | | | | | | structural molecule |
|---|---|---|---|---|---|---|---|---|
| 43134 | CLDN9 | NM_020982.1 | 3 | ggcttcgaccggcttagaactgc | 289956 | - | - | - |
| 43135 | CLDN9 | NM_020982.1 | 507 | ggcggctgcactgcttatgctgg | 289957 | - | see also 43134 line | |
| 43136 | CLTC | NM_004859.1 | 2877 | gtcgcgttgttggaaagtattgt | 290043 | - | see also 19056 line | |
| 43137 | CLTC | NM_004859.1 | 2113 | ccctgagtggttagtcaactact | 290014 | - | see also 19056 line | |
| 43138 | COL21A1 | NM_030820.2 | 288 | gtcgtactgtccgcacagattta | 290105 | - | see also 19057 line | |
| 43139 | COL21A1 | NM_030820.2 | 557 | cagtttgcgctcgattacctttt | 290124 | - | see also 19057 line | |
| 43140 | CTNNA1 | NM_001903.1 | 653 | aacgttccgatcctctatactgc | 290175 | - | see also 1216 line | |
| 43141 | CTNNA1 | NM_001903.1 | 697 | ccctgatgtcgcagcctataaag | 290176 | - | see also 1216 line | |
| 43142 | CTNNA1 | NM_001903.1 | 2513 | tcctacgtgcctctaccaaata | 290203 | - | see also 1216 line | |
| 43143 | CTNND2 | NM_001332.2 | 1733 | gccaggtcccgtccattgatag | 290217 | - | see also 19059 line | |
| 43144 | CTNND2 | NM_001332.2 | 2291 | accgggtgcctaaggaatgttag | 290228 | - | see also 19059 line | |
| 43145 | CTNND2 | NM_001332.2 | 649 | tccggccagctaccatagcaacc | 290213 | - | see also 19059 line | |
| 43146 | DCTN3 | NM_007234.3 | 295 | tcctapcagggagcagtttatc | 290256 | - | see also 19060 line | |
| 43147 | DCTN3 | NM_007234.3 | 495 | tacaacaagactacaatgcttct | 290258 | - | see also 19060 line | |
| 43148 | DJ473B4 | NM_019556.1 | 752 | ctccacttaagtgtgaatcaaaa | 290271 | - | see also 19061 line | |
| 43149 | DJ473B4 | NM_019556.1 | 749 | tacctccacttaagtgtgaatca | 290270 | - | see also 19061 line | |
| 43150 | DRP2 | NM_001939.1 | 2834 | ccgtatccttcggcaacataag | 290309 | - | see also 19062 line | |
| 43151 | DRP2 | NM_001939.1 | 1267 | atcaacgtccgatgaaacaact | 290291 | - | see also 19062 line | |
| 43152 | EVPL | NM_001988.1 | 1402 | agcggtataagcttgtagataac | 290313 | - | see also 19063 line | |
| 43153 | EVPL | NM_001988.1 | 6027 | agctacgaaggatttgacaga | 290322 | - | see also 19063 line | |
| 43154 | FLJ13646 | NM_024584.2 | 251 | gagctacaccgagaaaagttact | 290327 | - | see also 19064 line | |
| 43155 | FLJ13646 | NM_024584.2 | 184 | gacgatctgaacaagcatataa | 290323 | - | see also 19064 line | |
| 43156 | FLJ13646 | NM_024584.2 | 247 | cagggagctacaccgagaaaagt | 290325 | - | see also 19064 line | |
| 43157 | FLJ32214 | NM_152473.1 | 736 | cccatggagggttcacttatc | 290350 | - | see also 19065 line | |
| 43158 | FLJ32214 | NM_152473.1 | 966 | ggggcgatagagaggagatataaa | 290359 | - | see also 19065 line | |
| 43159 | HSPG2 | NM_005529.2 | 9926 | cacgtgagagccaccatatgc | 290405 | - | see also 19066 line | |
| 43160 | HSPG2 | NM_005529.2 | 10420 | gagctgccaaggacgtatatat | 290406 | - | see also 19066 line | |
| 43161 | HUMCYT2A | NM_015848.1 | 1260 | ggctacggctgagattgaaat | 290423 | - | see also 19067 line | |
| 43162 | HUMCYT2A | NM_015848.1 | 1588 | tggcagtagccgttggagttttg | 290426 | - | see also 19067 line | |
| 43163 | HUMCYT2A | NM_015848.1 | 1259 | aggctacgggctgagattgaaaa | 290422 | - | see also 19067 line | |
| 43164 | ITGBL1 | NM_004791.1 | 670 | tgctctaatgcaggttacatgtca | 290433 | - | see also 3327 line | |
| 43165 | ITGBL1 | NM_004791.1 | 1412 | gccaacatccgcggaagtgtaac | 290448 | - | see also 3327 line | |
| 43166 | IVL | NM_005547.1 | 1543 | cagcaggacggaccaactaaaaca | 290461 | - | see also 19069 line | |
| 43167 | IVL | NM_005547.1 | 1546 | caggaggaccgaccaactaaaacatct | 290462 | - | see also 19069 line | |

Figure 46

| | | | | | | | |
|---|---|---|---|---|---|---|---|
| 43168 | K6HF | NM_004693.1 | 978 | cggccgaggtcaaagcacaatacg | 290470 | - | see also 19070 line |
| 43169 | K6HF | NM_004693.1 | 848 | tgcccgaggagatcaacttcagc | 290469 | - | see also 19070 line |
| 43170 | KRT12 | NM_000223.1 | 1001 | agctccgtaaggagattagcacc | 290489 | - | see also 19071 line |
| 43171 | KRT12 | NM_000223.1 | 339 | aggaggtggtctctaggtattc | 290474 | - | see also 19071 line |
| 43172 | KRT4 | NM_002272.1 | 937 | gacggaagagcgcgaacagatca | 290496 | - | see also 19072 line |
| 43173 | KRT4 | NM_002272.1 | 1402 | caegtccgtggtcctttccatgg | 290500 | - | see also 19072 line |
| 43174 | KRT7 | NM_005556.2 | 1036 | tacccggaatgagatttcagaga | 290504 | - | see also 19073 line |
| 43175 | KRT7 | NM_005556.2 | 1473 | ctgggctcctgaaggcttattcc | 290507 | - | see also 19073 line |
| 43176 | KRTHA7 | NM_003770.3 | 402 | gtctttgtctcctatcgatgt | 290508 | - | see also 19074 line |
| 43177 | KRTHA7 | NM_003770.3 | 608 | ctgtggggctacggcaaaaaca | 290509 | - | see also 19074 line |
| 43178 | KRTHB5 | NM_002283.2 | 933 | ctcagacacctcggtcatagtca | 290512 | - | see also 19075 line |
| 43179 | KRTHB5 | NM_002283.2 | 883 | ttcctgaggcgcctcttatgaaga | 290511 | - | see also 19075 line |
| 43180 | LAD1 | NM_005558.3 | 1513 | gccttgcactgagttattcgtgg | 290520 | - | see also 19077 line |
| 43181 | LAD1 | NM_005558.3 | 1475 | gccaatacggagatcagaatctgt | 290519 | - | see also 19077 line |
| 43182 | LAMA1 | NM_005559.2 | 1859 | tacacggtgtctacgatattcc | 290556 | - | see also 3865 line |
| 43183 | LAMA1 | NM_005559.2 | 3236 | ctcgtggggtcgactcatcatcg | 290569 | - | see also 3865 line |
| 43184 | LAMA2 | NM_000426.2 | 1290 | gtcattgcgatccaattggttcc | 290716 | - | see also 327 line |
| 43185 | LAMA2 | NM_000426.2 | 6737 | taccgtatcgtagcatcaagaac | 290841 | - | see also 327 line |
| 43186 | LAMA2 | NM_000426.2 | 4855 | gcctggccatataaaatgctgt | 290792 | - | see also 327 line |
| 43187 | LAMA3 | NM_000227.2 | 2223 | ttggcgcaagattgaaagtatca | 290977 | - | see also 19080 line |
| 43188 | LAMA3 | NM_000227.2 | 3202 | aacggcagagaccattcgattca | 291009 | - | see also 19080 line |
| 43189 | LAMB1 | NM_002291.1 | 5638 | tccgttcactcctaaaggatata | 291267 | - | see also 1467 line |
| 43190 | LAMB1 | NM_002291.1 | 2658 | tgacccctcaaggttcgttaag | 291222 | - | see also 1467 line |
| 43191 | LAMB2 | NM_002292.2 | 822 | accgatatttctcctatgaactgt | 291272 | - | see also 1470 line |
| 43192 | LAMB2 | NM_002292.2 | 3732 | gtgactctcgtggaatagataca | 291293 | - | see also 1470 line |
| 43193 | LAMB3 | NM_000228.1 | 640 | gggaaggtccaacttaaccttat | 291316 | - | see also 170 line |
| 43194 | LAMB3 | NM_000228.1 | 1944 | ctcccgatcctagatgcaaaga | 291329 | - | see also 170 line |
| 43195 | LAMC1 | NM_002293.2 | 3601 | atcgcctacagagagtgaataac | 291401 | - | see also 1471 line |
| 43196 | LAMC1 | NM_002293.2 | 4362 | aagggacggatacettacaaga | 291418 | - | see also 1471 line |
| 43197 | LAMC3 | NM_006059.2 | 3992 | ggcgacgctacggcaaacagaac | 291448 | - | see also 19084 line |
| 43198 | LAMC3 | NM_006059.2 | 3236 | ctggggaccactagacattctgc | 291446 | - | see also 19084 line |
| 43199 | LCMR1 | NM_153450.1 | 504 | agcagtgtcgtctgatgcatatt | 291457 | - | see also 19085 line |
| 43200 | LCMR1 | NM_153450.1 | 397 | cagcctccgctctccattgaga | 291453 | - | see also 19085 line |
| 43201 | LMNB1 | NM_005573.2 | 1643 | ggcgagtagtgttagcatct | 291480 | - | see also 3873 line |
| 43202 | LMNB1 | NM_005573.2 | 1585 | gtcgtagtgtacgtacaactaga | 291477 | - | see also 3873 line |
| 43203 | LOC126147 | NM_145807.1 | 1262 | tgcgcgtgctggccgtttacaag | 291499 | - | see also 19087 line |

Figure 46

| | | | | | | | |
|---|---|---|---|---|---|---|---|
| 43204 | LOC126147 | NM_145807.1 | 749 | cccgacgctgccggttcaactct | 291494 | - | see also 19087 line |
| 43205 | LOC126147 | NM_145807.1 | 1118 | ctgacctcagtgtcaaaactac | 291497 | - | see also 19087 line |
| 43206 | MAP1A | NM_002373.3 | 7006 | aacccggcatgatgaatacctgg | 291571 | - | see also 19088 line |
| 43207 | MAP1A | NM_002373.3 | 6006 | ggggcacagccgagtatgacagt | 291564 | - | see also 19088 line |
| 43208 | MAP1B | NM_005909.2 | 6448 | gcccgtcaggatgtcgatttatg | 291725 | - | see also 4125 line |
| 43209 | MAP1B | NM_005909.2 | 585 | caccgaggtgcgcttaatgatca | 291592 | - | see also 4125 line |
| 43210 | MAP1B | NM_005909.2 | 6449 | ccgtcaggatgtcgatttatgc | 291726 | - | see also 4125 line |
| 43211 | MAP4 | NM_002375.2 | 1516 | cccggaaaccaacgtgatcttga | 291770 | - | see also 19090 line |
| 43212 | MAP4 | NM_002375.2 | 1629 | atgtggcttcgtccacagtaaaa | 291773 | - | see also 19090 line |
| 43213 | MAP7 | NM_003980.2 | 525 | ttgtacggcgcacaatggaaagg | 291799 | - | see also 19091 line |
| 43214 | MAP7 | NM_003980.2 | 669 | atgttgatccgtcattagcaag | 291801 | - | see also 19091 line |
| 43215 | MBP | NM_002385.1 | 443 | ggcagagcgtccgactataaatc | 291835 | - | see also 19092 line |
| 43216 | MBP | NM_002385.1 | 343 | ccccgtagtccacttcttcaaga | 291831 | - | see also 19092 line |
| 43217 | MEGF10 | NM_032446.1 | 2592 | ggctgtcgccagatatgtgattg | 291873 | - | see also 19093 line |
| 43218 | MEGF10 | NM_032446.1 | 1275 | gggctctacggcatcaaatgtga | 291858 | - | see also 19093 line |
| 43219 | MGC26706 | NM_152581.1 | 1267 | tccccatggggtttaacagtct | 291938 | - | see also 19094 line |
| 43220 | MGC26706 | NM_152581.1 | 1237 | cccgggtgcatcagtggatatag | 291934 | - | see also 19094 line |
| 43221 | MPZ | NM_000530.3 | 375 | atggctccattgtcatacacaac | 291954 | - | see also 19095 line |
| 43222 | MPZ | NM_000530.3 | 371 | aaggatggctccattgtcataca | 291953 | - | see also 19095 line |
| 43223 | MYOC | NM_000261.1 | 1118 | cacggacagttccgtattcttg | 291969 | - | see also 19096 line |
| 43224 | MYOC | NM_000261.1 | 748 | caccggatgtggaggaactagttt | 291962 | - | see also 19096 line |
| 43225 | NF2 | NM_000268.2 | 511 | gacgccgagatggagttcaattg | 291978 | - | see also 225 line |
| 43226 | NMP200 | NM_014502.3 | 1334 | gacttgaaggaacgtactaatgt | 292031 | - | see also 19098 line |
| 43227 | NMP200 | NM_014502.3 | 234 | tgccggacaccatgtgtatcc | 292014 | - | see also 19098 line |
| 43228 | NTN1 | NM_004822.1 | 260 | tgcatccggactttgtcaatgc | 292042 | - | see also 19099 line |
| 43229 | NTN1 | NM_004822.1 | 1639 | gtggaagttcacggtgaacatca | 292045 | - | see also 19099 line |
| 43230 | NTN4 | NM_021229.2 | 1884 | tccgtcccgtgcacaataagagc | 292072 | - | see also 19100 line |
| 43231 | NTN4 | NM_021229.2 | 1473 | gtcacttcgacgttaatgtgtgg | 292068 | - | see also 19100 line |
| 43232 | NUMA1 | NM_006185.1 | 670 | gaggacggctaaaccttaatga | 292085 | - | see also 4275 line |
| 43233 | NUMA1 | NM_006185.1 | 5031 | ggcagctgagcactaaagctgc | 292102 | - | see also 4275 line |
| 43234 | ODF1 | NM_024410.2 | 769 | aaccgtgcagcccatatgatcc | 292126 | - | see also 19102 line |
| 43235 | ODF1 | NM_024410.2 | 568 | tacgactgccttggatcgaaaaa | 292121 | - | see also 19102 line |
| 43236 | ODF2 | NM_002540.3 | 1912 | gacgggtaacagatcttgtaaa | 292148 | - | see also 1635 line |

Figure 46

| 43237 | ODF2 | NM_002540.3 | 1849 | agccgctacaaccaagttgtaaa | 292145 | - | see also 1635 line |
|---|---|---|---|---|---|---|---|
| 43238 | ODF2 | NM_002540.3 | 1913 | accgggtaacagatcttgtaaac | 292149 | - | see also 1635 line |
| 43239 | OSF-2 | NM_006475.1 | 2002 | tccccgtgactgtctatacaact | 292231 | - | see also 4545 line |
| 43240 | OSF-2 | NM_006475.1 | 2001 | atccccgtgactgtctatacaac | 292230 | - | see also 4545 line |
| 43241 | PKP3 | NM_007183.1 | 1004 | gagactcagcagcggttttgatg | 292244 | - | see also 4985 line |
| 43242 | PKP4 | NM_003628.2 | 2605 | tggcacccatcggtggtaaaacc | 292304 | - | see also 19106 line |
| 43243 | PKP4 | NM_003628.2 | 3311 | accctcgctctgaatacgatagg | 292334 | - | see also 19106 line |
| 43244 | PKP4 | NM_003628.2 | 3310 | gaccctcgctctgaatacgatag | 292333 | - | see also 19106 line |
| 43245 | PLP1 | NM_000533.2 | 525 | tggagcgggtgtgtcattgtttg | 292356 | - | see also 403 line |
| 43246 | PLP1 | NM_000533.2 | 404 | ggcgcagtcaggcagatctttgg | 292354 | - | see also 403 line |
| 43247 | PNN | NM_002687.2 | 87 | ggcggtcgccgtgagaactttgc | 292360 | - | see also 1749 line |
| 43248 | PNN | NM_002687.2 | 2137 | tccgacagaaagaggtctatatc | 292395 | - | see also 1749 line |
| 43249 | PRPH | NM_006262.2 | 62 | tcggtccaccgccctcactatcc | 292398 | - | see also 19108 line |
| 43250 | PRPH | NM_006262.2 | 1222 | tccgtgcccgtccattctttgc | 292403 | - | see also 19108 line |
| 43251 | SPON2 | NM_012445.1 | 1053 | agcagggacaatgagattgtaga | 292406 | - | see also 19109 line |
| 43252 | SPON2 | NM_012445.1 | 1243 | ctgagtgcgtccctgataactgc | 292408 | - | see also 19109 line |
| 43253 | TEKT1 | NM_053285.1 | 1096 | gtcgtgatgtcgcacaatatagg | 292421 | - | see also 9495 line |
| 43254 | TEKT1 | NM_053285.1 | 376 | atgatctactcatatataagatc | 292416 | - | see also 9495 line |
| 43255 | TEKT2 | NM_014466.2 | 646 | ctccgaccatcggggcaaaatgg | 292432 | - | see also 5697 line |
| 43256 | TEKT2 | NM_014466.2 | 168 | tgcccgactggcacactaacagc | 292425 | - | see also 5697 line |
| 43257 | TEKT3 | NM_031898.1 | 677 | aggtagcccgagaatgtctattt | 292460 | - | see also 19112 line |
| 43258 | TEKT3 | NM_031898.1 | 104 | aacgtgtaggttgtactttaacg | 292443 | - | see also 19112 line |
| 43259 | THOC1 | NM_005131.1 | 50 | agcgcggacgcggtttacgaagt | 292489 | - | see also 3606 line |
| 43260 | THOC1 | NM_005131.1 | 279 | accgcatctacacctttttgtatt | 292498 | - | see also 3606 line |
| 43261 | THOC1 | NM_005131.1 | 703 | ttgattacaacctgtatcgaaaa | 292510 | - | see also 3606 line |
| 43262 | UPK1B | NM_006952.2 | 575 | gccttccggactgagaataatga | 292539 | - | see also 19114 line |
| 43263 | UPK1B | NM_006952.2 | 226 | ggctgcctggatcggcatatttg | 292531 | - | see also 19114 line |
| 43264 | USH2A | NM_007123.3 | 737 | atcctaacgcccatagcaattct | 292556 | - | see also 4957 line |
| 43265 | USH2A | NM_007123.3 | 3685 | gaggatcaatacccatacagtat | 292655 | - | see also 4957 line |
| 43266 | VAPA | NM_003574.4 | 518 | ttgactatgatccgaatgaaaag | 292717 | - | see also 2371 line |
| 43267 | VAPA | NM_003574.4 | 357 | cacagatgtagtcactacaaatc | 292710 | - | see also 2371 line |
| 43268 | VAPB | NM_004738.3 | 372 | acccgacagaccgaaatgtgtgt | 292737 | - | see also 3265 line |
| 43269 | VAPB | NM_004738.3 | 452 | atcgatgcaggggcctcaattaa | 292739 | - | see also 3265 line |
| 43270 | VIL2 | NM_003379.3 | 670 | accgtgggatgctcaaagataat | 292764 | - | see also 19116 line |
| 43271 | VIL2 | NM_003379.3 | 1351 | aggcggtggatcagataaagagc | 292770 | - | see also 19116 line |
| 43272 | PI15 | NM_015886.1 | 1031 | cggataggatgcgcaattcatac | 292793 | - | see also 6561 line |
| 43273 | PI15 | NM_015886.1 | 1195 | gggatcttgtactgacaatctgt | 292804 | - | see also 6561 line |

Figure 46

| 43274 | CIDE-3 | NM_022094.2 | 437 | agccctggcaggggatacagtgt | 292808 | - | apoptosis | apoptosis regulator | - |
|---|---|---|---|---|---|---|---|---|---|
| 43275 | ECG2 | NM_032566.1 | 231 | cacctatgggaatgaatgtcact | 292811 | - | see also 19118 line | | |
| 43276 | ECG2 | NM_032566.1 | 253 | ttgtgtaccgagagcttgaaaag | 292812 | - | see also 19118 line | | |
| 43277 | ECG2 | NM_032566.1 | 187 | ccctgccccatcacatacctacc | 292810 | - | see also 19118 line | | |
| 43278 | FKSG28 | NM_030929.3 | 1169 | ccccagctgcgatcactaaacc | 292817 | - | see also 19119 line | | |
| 43279 | FKSG28 | NM_030929.3 | 1141 | cacctgaccagctgaactctaca | 292815 | - | see also 19119 line | | |
| 43280 | ITIH2 | NM_002216.1 | 1707 | ttcggctaacacgcagttagtct | 292885 | - | see also 1410 line | | |
| 43281 | ITIH2 | NM_002216.1 | 1589 | cagtcacggacgtcactcaaaac | 292876 | - | see also 1410 line | | |
| 43282 | ITIH3 | NM_002217.1 | 290 | gaccatcgacggtgttacctacc | 292913 | - | see also 19121 line | | |
| 43283 | ITIH3 | NM_002217.1 | 130 | atggcatcgaggtctacagtacc | 292907 | - | see also 19121 line | | |
| 43284 | ITIH4 | NM_002218.3 | 2156 | ctgtgtctcgtgtcatgaatatg | 292971 | - | see also 19122 line | | |
| 43285 | ITIH4 | NM_002218.3 | 1432 | accttcgagtacccaagcaatgc | 292955 | - | see also 19122 line | | |
| 43286 | OL-64 | NM_173850.2 | 1933 | gacaccactcgtcgtcaagatag | 292995 | - | see also 19124 line | | |
| 43287 | OL-64 | NM_173850.2 | 1936 | accactcgtcgtcaagatagaca | 292996 | - | see also 19124 line | | |
| 43288 | OL-64 | NM_173850.2 | 1938 | cactcgtcgtcaagatagacaaa | 292997 | - | see also 19124 line | | |
| 43289 | PZP | NM_002864.1 | 1446 | acggcacactatacactgaatag | 293039 | - | see also 19125 line | | |
| 43290 | PZP | NM_002864.1 | 1621 | cccccattgcacgaatgttcatc | 293042 | - | see also 19125 line | | |
| 43291 | RECK | NM_021111.1 | 2755 | ttcccgtcgatcactatccaaaa | 293161 | - | see also 8086 line | | |
| 43292 | RECK | NM_021111.1 | 2757 | cccgtcgatcactatccaaaagc | 293163 | - | see also 8086 line | | |
| 43293 | RECK | NM_021111.1 | 2751 | atcattcccgtcgatcactatcc | 293160 | - | see also 8086 line | | |
| 43294 | SERPINA1 | NM_000295.2 | 1098 | atgaactcacccacgatatcatc | 293167 | - | see also 19127 line | | |
| 43295 | SERPINA1 | NM_000295.2 | 1101 | aactcacccacgatatcatcacc | 293168 | - | see also 19127 line | | |
| 43296 | SERPINA10 | NM_016186.1 | 1710 | aagtggaccggccatttcatttc | 293190 | - | see also 19128 line | | |
| 43297 | SERPINA10 | NM_016186.1 | 1477 | tccgaagttcaagctagatcaga | 293183 | - | see also 19128 line | | |
| 43298 | SERPINA11 | NM_175739.2 | 225 | tacctttatggagtactctttgc | 293192 | - | see also 19129 line | | |
| 43299 | SERPINA11 | NM_175739.2 | 403 | ccccgagtcagaacatcttcttc | 293193 | - | see also 19129 line | | |
| 43300 | SERPINA3 | NM_001085.2 | 746 | gtgcccatgatgagtttgcatca | 293207 | - | see also 19130 line | | |
| 43301 | SERPINA3 | NM_001085.2 | 705 | atcagtcaaggttctacttgagc | 293206 | - | see also 19130 line | | |
| 43302 | SERPINB1 | NM_030666.2 | 488 | ggcttcgggcatggttgataaca | 293226 | - | see also 19131 line | | |
| 43303 | SERPINB1 | NM_030666.2 | 276 | aacaaacgtggagcgtcttatat | 293215 | - | see also 19131 line | | |
| 43304 | SERPINB11 | NM_080475.1 | 1133 | ctcataccaacacgatcctattc | 293283 | - | see also 19132 line | | |
| 43305 | SERPINB11 | NM_080475.1 | 707 | tgccctacgttaacaacaaatta | 293265 | - | see also 19132 line | | |

Figure 46

| | | | | | | | |
|---|---|---|---|---|---|---|---|
| 43306 | SERPINB13 | NM_012397.2 | 1146 | caccggcataggctttactgtca | 293301 | - | see also 19133 line |
| 43307 | SERPINB13 | NM_012397.2 | 1144 | gccaccggcataggctttactgt | 293300 | - | see also 19133 line |
| 43308 | SERPINB3 | NM_006919.1 | 383 | ttcggagaaaaaacgtatctatt | 293304 | - | see also 19134 line |
| 43309 | SERPINB3 | NM_006919.1 | 724 | tgcctcgctggaggatgtacagg | 293308 | - | see also 19134 line |
| 43310 | SERPINB4 | NM_002974.1 | 1036 | gccacggtctctcagtatctaaa | 293326 | - | - serpin - |
| 43311 | SERPINB4 | NM_002974.1 | 524 | ctgggtggaaagtcaaacgaatg | 293316 | - | see also 43310 line |
| 43312 | SERPINB5 | NM_002639.1 | 344 | agcggctctacgtagacaaatct | 293338 | - | see also 1713 line |
| 43313 | SERPINB5 | NM_002639.1 | 80 | atgccctgcaactagcaaattcg | 293332 | - | see also 1713 line |
| 43314 | SERPINB7 | NM_003784.1 | 488 | gtccgcttgggcgctcaagatga | 293359 | - | see also 19136 line |
| 43315 | SERPINB7 | NM_003784.1 | 490 | ccgcttgggcgctcaagatgact | 293360 | - | see also 19136 line |
| 43316 | SERPINB7 | NM_003784.1 | 1156 | aaggcgaatgacctctaagtatg | 293384 | - | see also 19136 line |
| 43317 | SERPINF1 | NM_002615.3 | 1040 | tccgagttcattcatgacataga | 293414 | - | see also 19137 line |
| 43318 | SERPINF1 | NM_002615.3 | 1039 | ctccgagttcattcatgacatag | 293413 | - | see also 19137 line |
| 43319 | SERPINF1 | NM_002615.3 | 1309 | cccgctggactatcaccttaacc | 293420 | - | see also 19137 line |
| 43320 | SERPINF2 | NM_000934.1 | 1426 | aagcttttcggccctgacttaaa | 293423 | - | see also 19138 line |
| 43321 | SERPINF2 | NM_000934.1 | 930 | acccacccactttgaatggaacg | 293421 | - | see also 19138 line |
| 43322 | SERPING1 | NM_000062.1 | 1248 | aacactaccccgcatcaaagtga | 293434 | - | see also 19139 line |
| 43323 | SERPING1 | NM_000062.1 | 477 | ggccgtgttgggggatgctttgg | 293428 | - | see also 19139 line |
| 43324 | SERPINI1 | NM_005025.1 | 654 | gtcgcagtttaggcctgaaaata | 293451 | - | see also 19140 line |
| 43325 | SERPINI1 | NM_005025.1 | 297 | ccgccactcaatgggatatgaca | 293441 | - | see also 19140 line |
| 43326 | SERPINI2 | NM_006217.2 | 949 | aacataaccgagatatttagtgg | 293477 | - | see also 19141 line |
| 43327 | SERPINI2 | NM_006217.2 | 1017 | ttcccaagtgacgcaaaaagttt | 293479 | - | see also 19141 line |
| 43328 | SLPI | NM_003064.2 | 395 | tcctgcgtttcccctgtgaaagc | 293493 | - | see also 19142 line |
| 43329 | SLPI | NM_003064.2 | 146 | cagtgccttagatacaagaaacc | 293487 | - | see also 19142 line |
| 43330 | SPINK1 | NM_003122.1 | 251 | gtcggaaacgccagacttctatc | 293501 | - | see also 19143 line |
| 43331 | SPINK1 | NM_003122.1 | 223 | cccaatgaatgcgtgttatgttt | 293500 | - | see also 19143 line |
| 43332 | SPINK2 | NM_021114.1 | 225 | ggcagtgacatgtccacttatgc | 293503 | - | see also 19144 line |
| 43333 | SPINK2 | NM_021114.1 | 123 | gcctctctgatccctcaatttgg | 293502 | - | see also 19144 line |
| 43334 | SPINK4 | NM_014471.1 | 183 | gggctcacatatacgaatgaatg | 293508 | - | see also 19145 line |
| 43335 | SPINK4 | NM_014471.1 | 184 | ggctcacatatacgaatgaatgc | 293509 | - | see also 19145 line |
| 43336 | SPINK5 | NM_006846.1 | 2583 | atctgtgtcgtgaatttcgaagc | 293549 | - | see also 19146 line |
| 43337 | SPINK5 | NM_006846.1 | 2239 | tggcaaatcgtacaacaatcagt | 293544 | - | see also 19146 line |
| 43338 | SPINLW1 | NM_020398.2 | 236 | atgtttagatctcaaacaagatg | 293568 | - | see also 19147 line |
| 43339 | SPINLW1 | NM_020398.2 | 301 | ttcttcattggtggtatgacaag | 293570 | - | see also 19147 line |
| 43340 | SPINT1 | NM_003710.2 | 1708 | cacggagcacctggtctataacc | 293583 | - | see also 2534 line |

Figure 46

| 43341 | SPINT1 | NM_003710.2 | 1031 | tgcaagagtttcgtttatggagg | 293579 | - | see also 2534 line | | |
|-------|--------|-------------|------|-------------------------|--------|---|--------------------|---|---|
| 43342 | SPINT2 | NM_021102.1 | 667 | tccagcgatatgttcaactatga | 293592 | - | see also 19149 line | | |
| 43343 | SPINT2 | NM_021102.1 | 659 | aagaccactccagcgatatgttc | 293589 | - | see also 19149 line | | |
| 43344 | UNC5D | NM_080872.1 | 1466 | aacccttgtcggacatcaaagt | 293621 | - | see also 9533 line | | |
| 43345 | UNC5D | NM_080872.1 | 371 | gtccgcgaagtgttcatcaatgt | 293604 | - | see also 9533 line | | |
| 43346 | WFDC10A | NM_080753.2 | 196 | agcagcctaaactatatctatgc | 293645 | - | see also 19151 line | | |
| 43347 | WFDC10A | NM_080753.2 | 195 | cagcagcctaaactatatctatg | 293644 | - | see also 19151 line | | |
| 43348 | WFDC12 | NM_080869.1 | 258 | aggaaacaaggatgaagatgtgt | 293649 | - | see also 19152 line | | |
| 43349 | WFDC3 | NM_080614.1 | 583 | ggcggtgattgtccaaaagttct | 293662 | - | see also 19153 line | | |
| 43350 | WFDC3 | NM_080614.1 | 747 | gtctgattccgaattagagatcc | 293665 | - | see also 19153 line | | |
| 43351 | WFDC5 | NM_145652.2 | 376 | ctcagactgctcgggcaaaaagc | 293666 | - | - | - | - |
| 43352 | WFDC8 | NM_130896.1 | 502 | cagaacggcctgcatgttaattg | 293675 | - | see also 19154 line | | |
| 43353 | WFDC8 | NM_130896.1 | 681 | gcccacgcaagcccttgctatgt | 293684 | - | see also 19154 line | | |
| 43354 | AHSG | NM_001622.1 | 424 | tccgtggtatacgcaaaatgtga | 293693 | - | see also 19155 line | | |
| 43355 | AHSG | NM_001622.1 | 414 | tggcaagttttccgtggtatacg | 293692 | - | see also 19155 line | | |
| 43356 | BIRC4 | NM_001167.2 | 398 | cagatttatcaacggcttttatc | 293707 | - | see also 19156 line | | |
| 43357 | BIRC4 | NM_001167.2 | 430 | gtgccacgcagtctacaaattct | 293708 | - | see also 19156 line | | |
| 43358 | BIRC5 | NM_001168.1 | 99 | accgcatctctacattcaagaac | 293735 | - | see also 19157 line | | |
| 43359 | BIRC5 | NM_001168.1 | 93 | aggaccaccgcatctctacattc | 293734 | - | see also 19157 line | | |
| 43360 | CAST | NM_001750.4 | 1035 | gacctccgctcaattaaggaagt | 293762 | - | see also 19158 line | | |
| 43361 | CAST | NM_001750.4 | 1030 | agctcgacctccgctcaattaag | 293761 | - | see also 19158 line | | |
| 43362 | CST1 | NM_001898.2 | 265 | gacgtccgctgcgggtactaaga | 293789 | - | - | cysteine protease inhibitor | - |
| 43363 | CST11 | NM_080830.2 | 68 | ctgttggccattctattgactct | 293792 | - | see also 19159 line | | |
| 43364 | CST11 | NM_080830.2 | 229 | caggatcttccgagtccttaaag | 293793 | - | see also 19159 line | | |
| 43365 | CST3 | NM_000099.2 | 241 | gccgtcggcgagtacaacaaagc | 293796 | - | - | structural molecule | atherosclerosis、coronary artery disease |
| 43366 | CST4 | NM_001899.2 | 144 | atcccaggtggcatctatgatgc | 293797 | - | - | cysteine protease inhibitor | - |
| 43367 | CST5 | NM_001900.2 | 244 | tggggtgaactactacttcaatg | 293800 | - | see also 19160 line | | |
| 43368 | CST5 | NM_001900.2 | 164 | gagtacaacaaggtcattaataa | 293798 | - | see also 19160 line | | |
| 43369 | CST6 | NM_001323.2 | 260 | tccgagacacgcacatcatcaag | 293804 | - | - | cysteine protease inhibitor | - |
| 43370 | CST6 | NM_001323.2 | 216 | ggccgtggccagctacaacatgg | 293803 | - | see also 43369 line | | |
| 43371 | CST7 | NM_003650.2 | 462 | ctgcacgaacgacatgttcttgt | 293808 | - | see also 19161 line | | |
| 43372 | CST7 | NM_003650.2 | 504 | aagggccctagttcagatagtga | 293811 | - | see also 19161 line | | |
| 43373 | CST8 | NM_005492.2 | 647 | agcctttaagcactaatgaaatc | 293818 | - | see also 19162 line | | |
| 43374 | CST8 | NM_005492.2 | 580 | cagcttcaggtcacaaatcttct | 293816 | - | see also 19162 line | | |

Figure 46

| | | | | | | | | |
|---|---|---|---|---|---|---|---|---|
| 43375 | CST9L | NM_080610.1 | 486 | gagtccaagactgtattctcaat | 293825 | - | see also 19163 line | |
| 43376 | CST9L | NM_080610.1 | 451 | tggggcacatcttgaattcctgg | 293824 | - | see also 19163 line | |
| 43377 | CSTA | NM_005213.2 | 227 | tacgagcagtgatataaatat | 293828 | - | see also 19164 line | |
| 43378 | CSTA | NM_005213.2 | 207 | aacaaattactacattaagtac | 293827 | - | see also 19164 line | |
| 43379 | CSTA | NM_005213.2 | 270 | aagtcttcccggacaaaatgagg | 293830 | - | see also 19164 line | |
| 43380 | CSTB | NM_000100.2 | 303 | tacaactgcgagtgttccaatct | 293832 | - | see also 19165 line | |
| 43381 | CSTB | NM_000100.2 | 342 | agccttgaccttatcttaactac | 293833 | - | see also 19165 line | |
| 43382 | ICEBERG | NM_021571.2 | 5 | ctgaccaactcttgcgtaaaaag | 293835 | - | apoptosis | cysteine protease inhibitor |
| 43383 | ICEBERG | NM_021571.2 | 70 | gccttgctgattgcctattaga | 293838 | - | see also 43382 line | |
| 43384 | ICEBERG | NM_021571.2 | 83 | gcctattagaggatgaagttatt | 293839 | - | see also 43382 line | |
| 43385 | KNG | NM_000893.2 | 915 | atcgatattcagctacgaattgc | 293863 | - | see also 19166 line | |
| 43386 | KNG | NM_000893.2 | 807 | tactcaattgtgcaaacgaattg | 293856 | - | see also 19166 line | |
| 43387 | TIMP4 | NM_003256.1 | 196 | ttgtgattcgggccaaaatcc | 293882 | - | see also 2200 line | |
| 43388 | TIMP4 | NM_003256.1 | 321 | aaggatgttcagtatatctatac | 293886 | - | see also 2200 line | |
| 43389 | C4A | NM_007293.1 | 4567 | gaccgttacgtggtgcactttga | 329804 | - | see also 19169 line | |
| 43390 | C4A | NM_007293.1 | 4701 | cgcaaccctgacgactactaca | 329805 | - | see also 19169 line | |
| 43391 | CD109 | NM_133493.1 | 3107 | gccgatccttacatagatattga | 293989 | - | see also 9588 line | |
| 43392 | CD109 | NM_133493.1 | 466 | tagtaccgcttatcatttgaga | 293894 | - | see also 9588 line | |
| 43393 | FLJ25179 | NM_144670.1 | 115 | gacggttgagatacaatattc | 294030 | - | see also 19171 line | |
| 43394 | FLJ25179 | NM_144670.1 | 113 | cagacggttgagatacaatat | 294029 | - | see also 19171 line | |
| 43395 | SPP2 | NM_006944.1 | 507 | ttcagacgagtccataagrtgaac | 294045 | - | see also 19172 line | |
| 43396 | SPP2 | NM_006944.1 | 176 | atctgtttcgggcattcagaagc | 294042 | - | see also 19172 line | |
| 43397 | SPP2 | NM_006944.1 | 539 | atcggtcaacttgggatcatgaga | 294048 | - | see also 19172 line | |
| 43398 | VIP | NM_015692.1 | 4837 | gacgccgagtgctttcttacttt | 294078 | - | see also 19173 line | |
| 43399 | VIP | NM_015692.1 | 3673 | tcgcacaggctcgcagctttatc | 294070 | - | see also 19173 line | |
| 43400 | DEFB126 | NM_030931.2 | 201 | agctgacagagtgctaattatc | 294093 | - | see also 19174 line | |
| 43401 | DEFB126 | NM_030931.2 | 205 | gacagacgtgctaattatcctgt | 294094 | - | see also 19174 line | |
| 43402 | CDKN1C | NM_000076.1 | 1067 | gcctctgatcctccgatttcttcg | 294097 | - | see also 19175 line | |
| 43403 | CDKN2C | NM_001262.2 | 1393 | aggtgctaatccgatttgaaag | 294108 | - | see also 822 line | |
| 43404 | PKIA | NM_006823.2 | 650 | agcaggtcttgatatcaacaaga | 294117 | - | see also 19177 line | |
| 43405 | PKIA | NM_006823.2 | 627 | agcaatgaattagccttgaaatt | 294116 | - | see also 19177 line | |
| 43406 | PKIB | NM_032471.4 | 432 | gagtctgsggtcgccaatttgc | 294119 | - | kinase_related | |
| 43407 | PKIB | NM_032471.4 | 512 | cacagacgaacctcagatttgc | 294121 | - | see also 43406 line | |
| 43408 | PKIB | NM_032471.4 | 441 | gtcgccaatttgtcatcttcagc | 294120 | - | see also 43406 line | |
| 43409 | DNAJC3 | NM_006260.2 | 1505 | caggcggaacatttagatttaaa | 294169 | - | see also 19178 line | |
| 43410 | DNAJC3 | NM_006260.2 | 251 | atcggaggctactgtctttta | 294127 | - | see also 19178 line | |

Figure 46

| 43411 | MBIP | NM_016586.1 | 134 | cccgagaggtgctctacgaaatc | 294173 | - | see also 6926 line |
|---|---|---|---|---|---|---|---|
| 43412 | MBIP | NM_016586.1 | 660 | taccccttacccgatttaaaa | 294187 | - | see also 6926 line |
| 43413 | MBIP | NM_016586.1 | 589 | aacgtcagggaatttgcaatgt | 294185 | - | see also 6926 line |
| 43414 | PPP1R1B | NM_032192.2 | 799 | ttcgggagctgggttatccaaga | 294198 | - | see also 19180 line |
| 43415 | PPP1R1B | NM_032192.2 | 736 | tgcagtctatcagcaatttgaat | 294197 | - | see also 19180 line |
| 43416 | SOCS1 | NM_003745.1 | 526 | gagcatccgcgtgcactttcagg | 294202 | - | see also 19181 line |
| 43417 | SOCS1 | NM_003745.1 | 471 | gcgcccagcggaactgcttttc | 294201 | - | see also 19181 line |
| 43418 | PPP1R1A | NM_006741.2 | 371 | ctccacgcaacggaagaagatg | 294203 | - | see also 4763 line |
| 43419 | C7orf16 | NM_066658.2 | 173 | ccctcgttcagccacttagatg | 294207 | - | see also 19183 line |
| 43420 | C7orf16 | NM_066658.2 | 183 | agccacttagatgatctttcaga | 294210 | - | see also 19183 line |
| 43421 | C7orf16 | NM_066658.2 | 200 | ttcagaccagttcattaaggact | 294211 | - | see also 19183 line |
| 43422 | CABIN1 | NM_012295.2 | 4193 | gaccacgattacgtcaaatgtaa | 294267 | - | see also 19184 line |
| 43423 | CABIN1 | NM_012295.2 | 6039 | ctgccgccacaactattatcacc | 294284 | - | see also 19184 line |
| 43424 | 1-4 | NM_025210.1 | 323 | accgcggcaacgtacagagattac | 294290 | - | see also 19185 line |
| 43425 | 1-4 | NM_025210.1 | 557 | aggcgcagttcgaaatgaagaaga | 294295 | - | see also 19185 line |
| 43426 | PPP1R11 | NM_021959.1 | 272 | aaccgagccttaccatcaaact | 294299 | - | phosphatase |
| 43427 | ANP32E | NM_030920.2 | 476 | atggctaatgtggaactaagttc | 294302 | - | see also 8963 line |
| 43428 | ANP32E | NM_030920.2 | 862 | agctggtccacggaaggatatg | 294314 | - | see also 8963 line |
| 43429 | SNCB | NM_003085.2 | 254 | tggcttcagtggctgaaaaaacc | 294318 | - | see also 19187 line |
| 43430 | GPS1 | NM_004127.3 | 1193 | gtcagccgacatgcataggatgg | 294330 | - | see also 19188 line |
| 43431 | GPS1 | NM_004127.3 | 1159 | accgtgccctcatccagtatttc | 294328 | - | see also 19188 line |
| 43432 | OAZ1 | NM_004152.2 | 368 | tccgatgatcggctgaatgtaac | 294400 | - | see also 19189 line |
| 43433 | OAZ1 | NM_004152.2 | 445 | cacagacgccaaaagcattaact | 294401 | - | see also 19189 line |
| 43434 | PSMF1 | NM_006814.2 | 817 | tggcttccaagagcacttattg | 294416 | - | see also 19190 line |
| 43435 | PSMF1 | NM_006814.2 | 296 | gaccagccgggtcccaatgataa | 294406 | - | see also 19190 line |
| 43436 | RNH | NM_002939.2 | 2700 | agctggtcctgtacgacatttac | 294417 | - | see also 19191 line |
| 43437 | ARRB1 | NM_004041.2 | 1099 | gagaacgagacgccagtagattac | 294430 | - | see also 19192 line |
| 43438 | ARRB1 | NM_004041.2 | 499 | gtcatccggaaggttcagtatgc | 294426 | - | see also 19192 line |
| 43439 | ATPIF1 | NM_016311.3 | 162 | ctcggatcagtccggaagaatgtcg | 294434 | - | see also 19193 line |
| 43440 | ATPIF1 | NM_016311.3 | 362 | agcgccataagcagaagatcaaa | 294438 | - | see also 19193 line |
| 43441 | NF1 | NM_000267.1 | 5709 | tgctcaatatcgcattacttaat | 294537 | - | see also 211 line |
| 43442 | NF1 | NM_000267.1 | 2821 | cagtgaacgtaagggttctatga | 294491 | - | see also 211 line |
| 43443 | ALS2 | NM_020919.2 | 4960 | ggcgtcactccacggaagacttct | 294774 | - | see also 8023 line |
| 43444 | ALS2 | NM_020919.2 | 1692 | aggctgcacgggtgaaaacgagg | 294670 | - | see also 8023 line |
| 43445 | ARHGEF3 | NM_019555.1 | 857 | tcccccttttagccgcaaactaga | 294798 | - | see also 19196 line |
| 43446 | ARHGEF3 | NM_019555.1 | 1513 | ctccgaggatcgttcctaaatc | 294815 | - | see also 19196 line |
| 43447 | ARHGEF3 | NM_019555.1 | 1514 | tccgagggatcgttcctaaatcc | 294816 | - | see also 19196 line |

Figure 46

| | | | | | | | |
|---|---|---|---|---|---|---|---|
| 43448 | ARHGEF4 | NM_015320.2 | 2130 | gccgcgacgtgttgtactacaag | 294825 | - | see also 19197 line |
| 43449 | ARHGEF4 | NM_015320.2 | 2030 | ggggagctgactcgagttacaca | 294822 | - | see also 19197 line |
| 43450 | ARHGEF6 | NM_004840.1 | 1528 | aacggtggtgactagattagatg | 294865 | - | see also 3355 line |
| 43451 | ARHGEF6 | NM_004840.1 | 47 | atcgtgacatggcttatatcttt | 294827 | - | see also 3355 line |
| 43452 | FARP1 | NM_005766.1 | 1692 | gtctaccaccgagcgaacatatc | 294923 | - | see also 19199 line |
| 43453 | FARP1 | NM_005766.1 | 703 | gcccgtcggctagagatgtatgg | 294905 | - | see also 19199 line |
| 43454 | FARP1 | NM_005766.1 | 702 | tgcccgtcggctagagatgtatg | 294904 | - | see also 19199 line |
| 43455 | FBXO8 | NM_012180.1 | 1139 | tacccgtcgcgctcaaaata | 294972 | - | see also 5182 line |
| 43456 | FBXO8 | NM_012180.1 | 1141 | cccgtcgcgctcaaaatatt | 294974 | - | see also 5182 line |
| 43457 | FBXO8 | NM_012180.1 | 380 | ttctaacaccaatcatcgtaaac | 294946 | - | see also 5182 line |
| 43458 | GBF1 | NM_004193.1 | 2914 | ggcttgttcgggagaactatgt | 295044 | - | see also 19201 line |
| 43459 | GBF1 | NM_004193.1 | 2306 | atccacgggaactaattgaaatt | 295032 | - | see also 19201 line |
| 43460 | PSCD1 | NM_004762.1 | 608 | tccagtccacggatacttgttac | 295084 | - | see also 19202 line |
| 43461 | PSCD1 | NM_004762.1 | 1220 | tgctcgcagcacggaaaaagaag | 295093 | - | see also 19202 line |
| 43462 | PSCD1 | NM_004762.1 | 607 | ttccagtccacggatactgtta | 295083 | - | see also 19202 line |
| 43463 | PSCD2 | NM_004228.3 | 1107 | cggaaaccgaactgctttgaact | 295096 | - | see also 19203 line |
| 43464 | PSCD2 | NM_004228.3 | 310 | aggccaatgagggcagagagacc | 295095 | - | see also 19203 line |
| 43465 | PSCD4 | NM_013385.2 | 1076 | aaggagccacggcggaattatacc | 295107 | - | see also 19204 line |
| 43466 | PSCD4 | NM_013385.2 | 224 | agcgggagacggaagagttaca | 295100 | - | see also 19204 line |
| 43467 | RAB2L | NM_004761.2 | 335 | accgtcacaagccgccaatatcg | 295114 | - | see also 19205 line |
| 43468 | RAB2L | NM_004761.2 | 338 | gtcacaagccgccaatatcgacc | 295115 | - | see also 19205 line |
| 43469 | RASGRF1 | NM_002891.3 | 3223 | aggacccctatattatcatgaaaac | 295171 | - | see also 1929 line |
| 43470 | RASGRF1 | NM_002891.3 | 3602 | aggagggggacgcccaattacacg | 295176 | - | see also 1929 line |
| 43471 | DELGEF | NM_012139.2 | 694 | gagtgacaggtctagagaattct | 295192 | - | see also 19207 line |
| 43472 | DELGEF | NM_012139.2 | 516 | caggccattgagctccataaaga | 295189 | - | see also 19207 line |
| 43473 | WBSCR16 | NM_030798.2 | 517 | aacgaggggtacgagtatgtgt | 295206 | - | see also 19208 line |
| 43474 | WBSCR16 | NM_030798.2 | 275 | gcgctgggcgtgcctccttgt | 295199 | - | see also 19208 line |
| 43475 | RIN2 | NM_018993.2 | 2245 | tacttgacaagcgcatatggagc | 295240 | - | see also 7644 line |
| 43476 | RGL | NM_015149.2 | 537 | gtggaagatcgttcgaatatg | 295253 | - | see also 19210 line |
| 43477 | RGL | NM_015149.2 | 979 | atgcaatcgcttccatactaagg | 295264 | - | see also 19210 line |
| 43478 | ABR | NM_001092.3 | 1831 | cggcgtcttcggttgtgaagatca | 295316 | - | see also 19211 line |
| 43479 | ABR | NM_001092.3 | 1728 | ggcacacggacgtgattgagatg | 295313 | - | see also 19211 line |
| 43480 | ARFGEF2 | NM_006420.1 | 2402 | atcggggtatcaatgatagttaaa | 295368 | - | see also 19212 line |
| 43481 | ARFGEF2 | NM_006420.1 | 4327 | ttggcgcgatcaggtacaaattg | 295408 | - | see also 19212 line |
| 43482 | ARHGEF7 | NM_003899.2 | 1590 | agcatgattgagcggatattagt | 295462 | - | see also 2656 line |
| 43483 | ARHGEF7 | NM_003899.2 | 921 | atgctgtacagtcttagaaga | 295448 | - | see also 2656 line |
| 43484 | BCAR3 | NM_003567.1 | 2193 | gccgcttgacgttaatggagc | 295514 | - | see also 19214 line |

Figure 46

| 43485 | BCAR3 | NM_003567.1 | 2484 | aactgccctctcgcgtaaattgg | 295521 | - | see also 19214 line |
|---|---|---|---|---|---|---|---|
| 43486 | BCAR3 | NM_003567.1 | 1929 | agcggccactctgagtaagatca | 295509 | - | see also 19214 line |
| 43487 | BIG1 | NM_006421.2 | 416 | gtcctcgcatagttagtacatct | 295526 | - | see also 19215 line |
| 43488 | BIG1 | NM_006421.2 | 5623 | ctgcgaatcggagtagtttttca | 295668 | - | see also 19215 line |
| 43489 | ECT2 | NM_018098.4 | 438 | gtcgcccgttgtattgtacaagt | 295686 | - | see also 7254 line |
| 43490 | ECT2 | NM_018098.4 | 1065 | atcgcaaacgacgtcgtttaaaa | 295712 | - | see also 7254 line |
| 43491 | ECT2 | NM_018098.4 | 1066 | tcgcaaacgacgtcgtttaaaag | 295713 | - | see also 7254 line |
| 43492 | FLJ10244 | NM_018037.1 | 421 | tgcgggtcagataacattaatgg | 295774 | - | see also 7215 line |
| 43493 | GPIG4 | NM_152545.1 | 1015 | gacgtagctcgggagtgttttaa | 295854 | - | see also 19218 line |
| 43494 | GPIG4 | NM_152545.1 | 1086 | gagcccagtctctcgactaaaaa | 295858 | - | see also 19218 line |
| 43495 | GPIG4 | NM_152545.1 | 344 | tactcagttctcggttatttatg | 295835 | - | see also 19218 line |
| 43496 | HAPIP | NM_003947.1 | 321 | tgcggaaactcgtgacgtatttg | 295877 | - | see also 19219 line |
| 43497 | HAPIP | NM_003947.1 | 2156 | cagcagaccgccactctagatgc | 295884 | - | see also 19219 line |
| 43498 | KIAA1010 | NM_015221.1 | 4457 | gtcaagacctcgtcaaaggatgt | 296036 | - | see also 19220 line |
| 43499 | KIAA1010 | NM_015221.1 | 2781 | gcgttacccgctgttgctaatgg | 296003 | - | see also 19220 line |
| 43500 | Link-GEFII | NM_016339.1 | 245 | atctatacctgctaattatgaag | 296046 | - | see also 19221 line |
| 43501 | Link-GEFII | NM_016339.1 | 1413 | ggcctatgtgcgccagtttcagg | 296056 | - | see also 19221 line |
| 43502 | LOC221002 | NM_145313.1 | 272 | accccgataggacgtacatcttc | 296058 | - | see also 19222 line |
| 43503 | LOC221002 | NM_145313.1 | 273 | ccccgataggacgtacatcttca | 296059 | - | see also 19222 line |
| 43504 | LOC221002 | NM_145313.1 | 1281 | gcccaacgggcacattaacttta | 296068 | - | see also 19222 line |
| 43505 | MCF2 | NM_005369.2 | 2413 | gacagatacccgtcatacagttt | 296140 | - | see also 3742 line |
| 43506 | MCF2 | NM_005369.2 | 2415 | cagatacccgtcatacagtttta | 296141 | - | see also 3742 line |
| 43507 | MCF2L | NM_024979.2 | 468 | accggcgacgggacaaatggacc | 296160 | - | see also 19224 line |
| 43508 | MCF2L | NM_024979.2 | 669 | tacacggttacatcgataagtcg | 296171 | - | see also 19224 line |
| 43509 | NET1 | NM_005863.2 | 916 | ctcgaagtcgcctagtcaaatac | 296216 | - | see also 19225 line |
| 43510 | NET1 | NM_005863.2 | 191 | gaccatacgagtcctagatgtca | 296197 | - | see also 19225 line |
| 43511 | NET1 | NM_005863.2 | 914 | ccctcgaagtcgcctagtcaaat | 296215 | - | see also 19225 line |
| 43512 | PDZGEF1 | NM_014247.1 | 2925 | cgggtacgtcgtagttcctttct | 296309 | - | see also 19226 line |
| 43513 | PDZGEF1 | NM_014247.1 | 3687 | tccgagggccgaggcttatatgc | 296326 | - | see also 19226 line |
| 43514 | PDZGEF2 | NM_016340.1 | 1757 | gtcatctccggttattgaatatt | 296404 | - | see also 6823 line |
| 43515 | PDZGEF2 | NM_016340.1 | 2577 | gaccggtgcatccgacacatatt | 296420 | - | see also 6823 line |
| 43516 | PL6 | NM_007024.4 | 1040 | ctctccagttgggtatatcttcg | 296451 | - | signal_transduction | - | - |
| 43517 | PL6 | NM_007024.4 | 1452 | ccccagaatccagtctaatcacc | 296453 | - | see also 43516 line |
| 43518 | PL6 | NM_007024.4 | 1450 | tgccccagaatccagtctaatca | 296452 | - | see also 43516 line |

EP 1 752 536 A1

Figure 46

| 43519 | RALGDS | NM_006266.1 | 1440 | aagaacttctcgtcactgtatgc | 296470 | - | see also 4357 line |
|---|---|---|---|---|---|---|---|
| 43520 | RALGPS1A | NM_014636.1 | 1054 | aagtccgtacgctacattgaaga | 296484 | - | see also 5810 line |
| 43521 | RALGPS1A | NM_014636.1 | 1051 | ctgaagtccgtacgctacattga | 296483 | - | see also 5810 line |
| 43522 | RASGRF2 | NM_006909.1 | 2424 | cccacgcgtggatctgtgtaaca | 296583 | - | see also 4847 line |
| 43523 | RASGRF2 | NM_006909.1 | 2426 | cacgcgtggatctgtgtaacaag | 296584 | - | see also 4847 line |
| 43524 | RASGRP4 | NM_052949.2 | 248 | caggaatgcaccggaaaaatagg | 296610 | - | signal_transduction、k inase_related、gpcr - - |
| 43525 | Rgr | NM_153615.1 | 1764 | tccctgtcgtgggtctgtaaaga | 296624 | - | see also 19231 line |
| 43526 | Rgr | NM_153615.1 | 1560 | tgggcaacacgcattaacaatgc | 296621 | - | see also 19231 line |
| 43527 | SOS2 | NM_006939.1 | 1632 | tcgtagtactctagatcgaatgt | 296675 | - | see also 19232 line |
| 43528 | SOS2 | NM_006939.1 | 2464 | atgattcgccataccacaaatct | 296697 | - | see also 19232 line |
| 43529 | CDC42EP2 | NM_006779.2 | 559 | ttccgccacaccattcatattgg | 296731 | - | - - - |
| 43530 | CDC42EP2 | NM_006779.2 | 456 | caccaaggtgcccatctatctga | 296730 | - | see also 43529 line |
| 43531 | GRAF | NM_015071.2 | 334 | aacggatacggatgattgagaat | 296738 | - | see also 19233 line |
| 43532 | GRAF | NM_015071.2 | 171 | ctcataagcgcgctcaagaattt | 296732 | - | see also 19233 line |
| 43533 | MIG-6 | NM_018948.2 | 1127 | tacttcgagcacctatagtgatg | 296805 | - | see also 19234 line |
| 43534 | MIG-6 | NM_018948.2 | 423 | tccaaatctgctccgatgaatgg | 296782 | - | see also 19234 line |
| 43535 | MIG-6 | NM_018948.2 | 1028 | ttctcctaactccgatgaagaca | 296801 | - | see also 19234 line |
| 43536 | OPHN1 | NM_002547.1 | 2394 | cacaaaccaatcacgatttcaaa | 296855 | - | see also 1641 line |
| 43537 | OPHN1 | NM_002547.1 | 948 | atggtacacaatgctagtgattt | 296831 | - | see also 1641 line |
| 43538 | PARG1 | NM_004815.2 | 2521 | ttcgattgtacaaggaatttata | 296944 | - | see also 3345 line |
| 43539 | PARG1 | NM_004815.2 | 336 | atggggctcaagtccttaagttc | 296864 | - | see also 3345 line |
| 43540 | IQGAP3 | NM_178229.3 | 3495 | tccgtatgggatgcgatatgtgg | 297022 | - | see also 19237 line |
| 43541 | IQGAP3 | NM_178229.3 | 2259 | agccaacgtcggctttgttatcc | 297002 | - | see also 19237 line |
| 43542 | RASA1 | NM_002890.1 | 961 | aaggcgtgtacgagctattctac | 297048 | - | see also 1924 line |
| 43543 | RASA1 | NM_002890.1 | 1505 | atccgtcgtaaaacaaaggatgc | 297070 | - | see also 1924 line |
| 43544 | RASA2 | NM_006506.2 | 2104 | agggtgagccgatgcaatcaaaa | 297205 | - | see also 4550 line |
| 43545 | RASA2 | NM_006506.2 | 916 | ctggggtctcttcgattaaatat | 297158 | - | see also 4550 line |
| 43546 | RASAL2 | NM_004841.2 | 1973 | gactaatcctacgccaatacaac | 297276 | - | see also 19240 line |
| 43547 | RASAL2 | NM_004841.2 | 157 | cccctcgtagacggagtatctca | 297217 | - | see also 19240 line |
| 43548 | ARHGDIB | NM_001175.1 | 718 | gggagtggaacctgtcgattaag | 297307 | - | see also 19241 line |
| 43549 | ARHGDIB | NM_001175.1 | 722 | gtggaacctgtcgattaagaagg | 297308 | - | see also 19241 line |
| 43550 | ARHGDIG | NM_001176.1 | 430 | aaggaaggtgttgattacagagt | 297309 | - | see also 19242 line |
| 43551 | GDI2 | NM_001494.2 | 329 | aggatcaccacccgagtcaatgg | 297314 | - | see also 19243 line |
| 43552 | GDI2 | NM_001494.2 | 204 | atcctgtcaggtataatgtcagt | 297312 | - | see also 19243 line |

Figure 46

| | | | | | | | |
|---|---|---|---|---|---|---|---|
| 43553 | GDI1 | NM_001493.1 | 113 | gaccggtctcacgaatgcatcc | 297350 | - | see also 19244 line |
| 43554 | GDI1 | NM_001493.1 | 588 | gacgtctaccggaagtttgatct | 297355 | - | see also 19244 line |
| 43555 | MAPKAP1 | NM_024117.2 | 1564 | atctaagcaatcacgactataaa | 297424 | - | see also 8590 line |
| 43556 | MAPKAP1 | NM_024117.2 | 1605 | gacgctgctaccgtcaatgaaat | 297425 | - | see also 8590 line |
| 43557 | RIN3 | NM_024832.3 | 2610 | ctggggagggttcctactatct | 297441 | - | see also 8771 line |
| 43558 | RIN3 | NM_024832.3 | 2169 | gaggagctgaagcaattgtaga | 297437 | - | see also 8771 line |
| 43559 | RANGAP1 | NM_002883.2 | 1487 | gccacgccctcacggaagattct | 297454 | - | see also 19246 line |
| 43560 | RANGAP1 | NM_002883.2 | 1489 | cacgccctcacggaagattctgg | 297455 | - | see also 19246 line |
| 43561 | SIPA1 | NM_006747.2 | 795 | tgctggctactaccgcaaatact | 297460 | - | see also 19247 line |
| 43562 | SIPA1 | NM_006747.2 | 2276 | cgcacgtggagccgcttcacattc | 297465 | - | see also 19247 line |
| 43563 | SIPA1 | NM_006747.2 | 489 | tcgatcgtttgcccactatgacg | 297458 | - | see also 19247 line |
| 43564 | BART1 | NM_012106.2 | 399 | aacattacagcaccataaggatg | 297467 | - | see also 19248 line |
| 43565 | BART1 | NM_012106.2 | 383 | tggcagccttcaccacaacatta | 297466 | - | see also 19248 line |
| 43566 | CCM1 | NM_004912.3 | 2029 | aacggatagacatataaacagacc | 297504 | - | see also 3413 line |
| 43567 | DKFZp761J1523 | NM_032293.3 | 1196 | acgtcggcgtaccctggatatgt | 297565 | - | see also 9145 line |
| 43568 | DKFZp761J1523 | NM_032293.3 | 2390 | ggcctccaggtcggatatatact | 297603 | - | see also 9145 line |
| 43569 | MINK | NM_015716.2 | 2929 | gagctcgttcacgatgtttgttgg | 297634 | - | see also 6540 line |
| 43570 | MINK | NM_015716.2 | 3608 | cagcggctcaaggtcatctatgg | 297637 | - | see also 6540 line |
| 43571 | RPIP8 | NM_006695.3 | 819 | gacccccgtgtcatcgattaca | 297645 | - | see also 19252 line |
| 43572 | RPIP8 | NM_006695.3 | 1270 | ccctcactgggaacgcttaatgg | 297647 | - | see also 19252 line |
| 43573 | WAS | NM_000377.1 | 878 | accgaccgcgagacctctaaact | 297652 | - | see also 19253 line |
| 43574 | WAS | NM_000377.1 | 122 | cacgagaaccagccgactctttga | 297648 | - | see also 19253 line |
| 43575 | WAS | NM_000377.1 | 1327 | gggagcgcgtttggatcaaatcc | 297656 | - | see also 19253 line |
| 43576 | BNIP2 | NM_004330.1 | 591 | gacgacgctggcgtatgttcagg | 297688 | - | see also 19254 line |
| 43577 | BNIP2 | NM_004330.1 | 902 | cagcaaattgatagaaggttacg | 297696 | - | see also 19254 line |
| 43578 | C5orf5 | NM_016603.1 | 3033 | aacgttgcgggaatttgaagaag | 297778 | - | see also 6940 line |
| 43579 | DOCK1 | NM_001380.1 | 160 | accgaggttacacgttacgaaaa | 297791 | - | see also 19255 line |
| 43580 | DOCK1 | NM_001380.1 | 2761 | ccgtgaaccgaaccgtcatttcc | 297862 | - | see also 19255 line |
| 43581 | LGN | NM_013296.3 | 392 | ccgcctggcgtgtcattctttg | 297929 | - | see also 5389 line |
| 43582 | LGN | NM_013296.3 | 625 | ttgacgaagccatagtttgtgt | 297941 | - | see also 5389 line |
| 43583 | RAB3-GAP150 | NM_012414.2 | 1342 | agggtaccgcgacgcacaaattg | 298037 | - | see also 19257 line |
| 43584 | RAB3-GAP150 | NM_012414.2 | 1444 | tccaagtcgagtagctcaattcc | 298043 | - | see also 19257 line |

Figure 46

| | | | | | | | |
|---|---|---|---|---|---|---|---|
| 43585 | RALBP1 | NM_006788.2 | 707 | agccgtttccgtgaatgtatag | 298102 | - | see also 19258 line |
| 43586 | RALBP1 | NM_006788.2 | 706 | cagccgtttccgtgaatgtata | 298101 | - | see also 19258 line |
| 43587 | RAP1GA1 | NM_002885.1 | 1107 | tcctttcgtcccgacatgatcg | 298144 | - | see also 19259 line |
| 43588 | RAP1GA1 | NM_002885.1 | 2127 | tgcgtgtcccgagatcaagatcc | 298155 | - | see also 19259 line |
| 43589 | RAP1GDS1 | NM_021159.3 | 1510 | tgcccttatacgacacagtaaat | 298195 | - | see also 8111 line |
| 43590 | RAP1GDS1 | NM_021159.3 | 1511 | gcccttatacgacacagtaaatc | 298196 | - | see also 8111 line |
| 43591 | RASA3 | NM_007368.2 | 1939 | ttcgcttgaccaaccatgaattt | 298228 | - | see also 5094 line |
| 43592 | RASA3 | NM_007368.2 | 979 | tgcggctgaacgttggtatacacg | 298215 | - | see also 5094 line |
| 43593 | SH3BP1 | NM_018957.2 | 536 | ctgacctgtaccactttgttacc | 298246 | - | see also 19262 line |
| 43594 | SH3BP1 | NM_018957.2 | 379 | ctcagtcaggcaaccaagaattc | 298245 | - | see also 19262 line |
| 43595 | SRGAP2 | NM_014850.1 | 2856 | gagcgctggcagcatcaactacc | 298278 | - | see also 6050 line |
| 43596 | SRGAP2 | NM_014850.1 | 1787 | accccttgtggacgatcaaaat | 298269 | - | see also 6050 line |
| 43597 | STARD13 | NM_052851.1 | 1767 | ggcgactccgatgaacagtttc | 298300 | - | see also 19264 line |
| 43598 | STARD13 | NM_052851.1 | 2583 | aggggctagcgcaatgatcatg | 298318 | - | see also 19264 line |
| 43599 | STARD8 | NM_014725.2 | 740 | agccaagaagcgccatcgtaacc | 298326 | - | see also 5915 line |
| 43600 | STARD8 | NM_014725.2 | 2073 | taccgggacagcacgtatttgg | 298339 | - | see also 5915 line |
| 43601 | STARD8 | NM_014725.2 | 1192 | gccatgcagcacgtatgacaac | 298331 | - | see also 5915 line |
| 43602 | TBC1D2 | NM_018421.1 | 2632 | tgcggacagtctcattagcaaca | 298349 | - | see also 19266 line |
| 43603 | TBC1D2 | NM_018421.1 | 2464 | gaccatcatgcccgctgattact | 298348 | - | see also 19266 line |
| 43604 | TSC2 | NM_000548.2 | 3241 | acegggacccggttcgttactagg | 298387 | - | see also 19267 line |
| 43605 | TSC2 | NM_000548.2 | 722 | gcctcccgctgttcatcgttacc | 298363 | - | see also 19267 line |
| 43606 | BCL2L13 | NM_015367.1 | 341 | ttcggcagagtacatcattcagc | 298400 | - | see also 19268 line |
| 43607 | BCL2L13 | NM_015367.1 | 371 | ctgggggcactgtttagtcttg | 298402 | - | see also 19268 line |
| 43608 | PSME1 | NM_006263.2 | 511 | acctcggattgaggatggtaaca | 298415 | - | see also 19269 line |
| 43609 | PSME1 | NM_006263.2 | 246 | aagccaacttggcaatctgaag | 298412 | - | see also 19269 line |
| 43610 | PSME3 | NM_005789.2 | 872 | agccttcggctcatcatatcaga | 298438 | - | see also 19270 line |
| 43611 | PSME3 | NM_005789.2 | 869 | atcagccttcggctcatcatatc | 298437 | - | see also 19270 line |
| 43612 | PSME3 | NM_005789.2 | 595 | acctgagatccggctgttgattg | 298431 | - | see also 19270 line |
| 43613 | PPP2R4 | NM_021131.3 | 332 | cgctgactacatcggattcatcc | 298444 | - | see also 19271 line |
| 43614 | PPP2R4 | NM_021131.3 | 984 | atgaagactggcccatttgcaga | 298459 | - | see also 19271 line |
| 43615 | PLAA | NM_004253.2 | 518 | ggcgagtgtcttgaagtatatta | 298479 | - | see also 2857 line |
| 43616 | PLAA | NM_004253.2 | 720 | gtgcgagtgatggcattattaga | 298486 | - | see also 2857 line |
| 43617 | GM2A | NM_000405.3 | 628 | ccgggaactaccgcatagagagc | 298553 | - | see also 322 line |
| 43618 | GM2A | NM_000405.3 | 262 | aggaccctgccggtgatcagaagc | 298548 | - | see also 322 line |
| 43619 | CLPS | NM_001832.2 | 244 | gacgctctatgggatttactaca | 298555 | - | see also 19274 line |

Figure 46

| 43620 | CLPS | NM_001832.2 | 246 | cgctctatgggatttactacaag | 298556 | - | see also 19274 line |
|---|---|---|---|---|---|---|---|
| 43621 | PPP1R12B | NM_002481.2 | 2961 | gcgagccttggagcgcaaaatgt | 298587 | - | see also 19275 line |
| 43622 | PPP1R12B | NM_002481.2 | 2065 | cagacacgaaggtctactcaagg | 298572 | - | see also 19275 line |
| 43623 | CABYR | NM_012189.2 | 509 | cacccagtttccatcagtttatg | 298597 | - | see also 5184 line |
| 43624 | CABYR | NM_012189.2 | 416 | aacatctgtagaatctaaagtac | 298596 | - | see also 5184 line |
| 43625 | SPA17 | NM_017425.2 | 429 | gacatcagtcaccatcttagact | 298652 | - | see also 7001 line |
| 43626 | SPA17 | NM_017425.2 | 200 | ggctgacacgcgagattctgaga | 298647 | - | see also 7001 line |
| 43627 | IGBP1 | NM_001551.1 | 288 | aaccccagcaagcgtctagatca | 298663 | - | see also 19278 line |
| 43628 | IGBP1 | NM_001551.1 | 682 | gagaggcatcaacttctaactca | 298679 | - | see also 19278 line |
| 43629 | PPP2R2B | NM_004576.2 | 1810 | gtggcggctacaaataacctata | 298712 | - | see also 3114 line |
| 43630 | PPP2R2B | NM_004576.2 | 657 | gggggggtcgggttgtaatatttc | 298685 | - | see also 3114 line |
| 43631 | PPP2R2C | NM_020416.2 | 900 | ctccgtgtccgacgtgaagttca | 298736 | - | see also 7868 line |
| 43632 | PPP2R2C | NM_020416.2 | 409 | aagattaccgaacgagataaaag | 298730 | - | see also 7868 line |
| 43633 | PPP2R2C | NM_020416.2 | 945 | cacccgggactaccttacagtca | 298739 | - | see also 7868 line |
| 43634 | PPP2R5A | NM_006243.2 | 1042 | gcctcacatacagttggtatatg | 298751 | - | see also 19281 line |
| 43635 | PPP2R5A | NM_006243.2 | 1101 | agcctagcattgcaaaacgatac | 298752 | - | see also 19281 line |
| 43636 | PPP2R5B | NM_006244.2 | 1301 | tcctccggttcatctatgaattc | 298782 | - | see also 4326 line |
| 43637 | PPP2R5B | NM_006244.2 | 1132 | ccctccgtggccaagagatatgt | 298779 | - | see also 4326 line |
| 43638 | PPP2R5B | NM_006244.2 | 1131 | gccctccgtggccaagagatatg | 298778 | - | see also 4326 line |
| 43639 | PPP2R5C | NM_002719.2 | 1224 | ttccttgtaccgcaactcaaaga | 298789 | - | phosphatase、signal_transduction ┤ - ├ - |
| 43640 | PPP2R5C | NM_002719.2 | 178 | cccgtggtccttctccatattcg | 298788 | - | see also 43639 line |
| 43641 | PPP2R5D | NM_006245.2 | 811 | ctctcctagacctatttgacagt | 298810 | - | see also 4327 line |
| 43642 | PPP2R5D | NM_006245.2 | 1405 | cccgagtcctccccatcatgttc | 298816 | - | see also 4327 line |
| 43643 | PPP1R7 | NM_002712.1 | 602 | accgcatccgggcaatcgaaaat | 298870 | - | see also 19284 line |
| 43644 | PPP1R7 | NM_002712.1 | 604 | cgcatccgggcaatcgaaaatat | 298872 | - | see also 19284 line |
| 43645 | PPP1R7 | NM_002712.1 | 601 | aaccgcatccgggcaatcgaaaa | 298869 | - | see also 19284 line |
| 43646 | PPP4R1 | NM_005134.1 | 1893 | acctcaggcgttgttagatcagt | 298918 | - | see also 19285 line |
| 43647 | PPP4R1 | NM_005134.1 | 1897 | caggcgttgttagatcagtattt | 298919 | - | see also 19285 line |
| 43648 | CAPON | NM_014697.1 | 554 | gccggatacggtatgagtttaaa | 298951 | - | see also 19286 line |
| 43649 | CAPON | NM_014697.1 | 553 | cgccggatacggtatgagtttaa | 298950 | - | see also 19286 line |
| 43650 | CAPON | NM_014697.1 | 1386 | tcgcgtgcatcagcttttgctgc | 298961 | - | see also 19286 line |
| 43651 | SLN | NM_003063.1 | 199 | aacttcactattgtcttgattac | 298968 | - | see also 19287 line |
| 43652 | SLN | NM_003063.1 | 188 | agctgtttctcaacttcactatt | 298967 | - | see also 19287 line |
| 43653 | DCPS | NM_014026.3 | 880 | ctgaagcggaccggattgtttc | 298974 | - | see also 19288 line |
| 43654 | DCPS | NM_014026.3 | 964 | agctcgatgacttgtacttgatc | 298977 | - | see also 19288 line |
| 43655 | DCPS | NM_014026.3 | 961 | agcagctcgatgacttgtacttg | 298976 | - | see also 19288 line |

Figure 46

| | | | | | | | | |
|---|---|---|---|---|---|---|---|---|
| 43656 | DNAJA1 | NM_001539.1 | 632 | ggggagcggatcagtcctaaaga | 298984 | - | see also 19289 line | |
| 43657 | DNAJA1 | NM_001539.1 | 1032 | agggtcgcctaatcatcgaatt | 298997 | - | see also 19289 line | |
| 43658 | DNAJB1 | NM_006145.1 | 877 | gacgataccgtcgtattcaaag | 299013 | - | see also 19290 line | |
| 43659 | DNAJB1 | NM_006145.1 | 910 | gcctggcatgcgggggaaaagttc | 299018 | - | see also 19290 line | |
| 43660 | DNAJB2 | NM_006736.4 | 670 | ttcgctctgtttcacatctac | 299025 | - | see also 19291 line | |
| 43661 | DNAJB2 | NM_006736.4 | 664 | gtgcttttgctctgtttctaca | 299024 | - | see also 19291 line | |
| 43662 | DNAJB4 | NM_007034.3 | 433 | ggcgatcctcatgctacatttgc | 299032 | - | see also 4923 line | |
| 43663 | DNAJB4 | NM_007034.3 | 1079 | gtggtgaccttcaatagaattt | 299050 | - | see also 4923 line | |
| 43664 | DNAJB5 | NM_012266.2 | 648 | ttcggccgtttggcttcaatgg | 299057 | - | see also 5244 line | |
| 43665 | DNAJB6 | NM_005494.2 | 194 | tgcctcaccgaggatattaaaa | 299062 | - | see also 19293 line | |
| 43666 | DNAJB6 | NM_005494.2 | 778 | ttgtcgagaacggtcaagaaaga | 299068 | - | see also 19293 line | |
| 43667 | FLJ32389 | NM_144617.1 | 265 | cacttctgccggaggaaattgc | 299075 | - | - | heat shock protein |
| 43668 | HSPB3 | NM_006308.1 | 486 | tgatgagcacggtttatctca | 299079 | - | see also 19294 line | |
| 43669 | HSPB3 | NM_006308.1 | 515 | ttcaccgacagtacaaactacc | 299082 | - | see also 19294 line | |
| 43670 | HspB9 | NM_033194.1 | 123 | agcggctcaggaggacaatgacc | 299085 | - | - | |
| 43671 | HSPE1 | NM_002157.1 | 187 | ttggatcggttctaaaggaaag | 299086 | - | see also 19295 line | |
| 43672 | TBCC | NM_003192.1 | 625 | tgcaaccgcggacgttctttg | 299092 | - | see also 2158 line | |
| 43673 | TBCC | NM_003192.1 | 660 | aactgcaggtcagactgtatgg | 299094 | - | see also 2158 line | |
| 43674 | TBCC | NM_003192.1 | 628 | accagccgcagttctttgacc | 299093 | - | see also 2158 line | |
| 43675 | TBCD | NM_005993.2 | 278 | ctggagcggttccgctaataat | 299107 | - | see also 19297 line | |
| 43676 | TBCD | NM_005993.2 | 288 | tccgcgtaataatggacaaatac | 299110 | - | see also 19297 line | |
| 43677 | TBCE | NM_003193.2 | 289 | ttcgtccgaacaaggtaaattt | 299161 | - | see also 19298 line | |
| 43678 | TBCE | NM_003193.2 | 412 | tggagactatcggttttgactct | 299166 | - | see also 19298 line | |
| 43679 | TBCE | NM_003193.2 | 660 | ccctccggttcagtattaactgg | 299176 | - | see also 19298 line | |
| 43680 | TBCA | NM_004607.1 | 58 | tcctgcgtgagacgagatcaaga | 299213 | - | - | tubulin-specific chaperone |
| 43681 | C19orf2 | NM_003796.2 | 561 | tgctcattaaacgccattccaaac | 299224 | - | see also 19299 line | |
| 43682 | C19orf2 | NM_003796.2 | 1060 | aagagggtccgaataaatactgg | 299239 | - | see also 19299 line | |
| 43683 | CABC1 | NM_020247.3 | 1645 | tggggcaacgcgggaatatgaca | 299256 | - | see also 19300 line | |
| 43684 | CABC1 | NM_020247.3 | 1084 | ccccaactggcgggacaagttgg | 299252 | - | see also 19300 line | |
| 43685 | CIA30 | NM_016013.1 | 959 | tacgattggtcccagttcaatac | 299272 | - | see also 6614 line | |
| 43686 | CIA30 | NM_016013.1 | 605 | ttcgataaagcaattagagatga | 299267 | - | see also 6614 line | |
| 43687 | CKAP1 | NM_001281.1 | 639 | tggattggtgtccgctatgatga | 299296 | - | see also 19302 line | |
| 43688 | CKAP1 | NM_001281.1 | 88 | cacccacggtgaccgttttcatc | 299289 | - | see also 19302 line | |
| 43689 | CLN3 | NM_000086.1 | 805 | tgctggccagctatttcttgttg | 299301 | - | see also 19303 line | |
| 43690 | CLN3 | NM_000086.1 | 454 | tccccactctgtcatcaaattg | 299300 | - | see also 19303 line | |
| 43691 | DNAJA2 | NM_005880.2 | 996 | aagggatgccgcagtatcgtaat | 299327 | - | see also 4088 line | |

Figure 46

| 43692 | DNAJA2 | NM_005880.2 | 997 | agggatgccgcagtatcgtaatc | 299328 | - | see also 4088 line | | |
|---|---|---|---|---|---|---|---|---|---|
| 43693 | DNAJA3 | NM_005147.3 | 169 | cccccgagcgctgctgacattga | 299339 | - | see also 19305 line | | |
| 43694 | DNAJA3 | NM_005147.3 | 1198 | cggctacggagaccactacatcc | 299351 | - | see also 19305 line | | |
| 43695 | DNAJA4 | NM_018602.2 | 1088 | ttggacaatcgaattcttgttat | 299370 | - | see also 7531 line | | |
| 43696 | DNAJA4 | NM_018602.2 | 371 | ggcgagaagtttaaactcatatc | 299357 | - | see also 7531 line | | |
| 43697 | DNAJB11 | NM_016306.3 | 404 | aacggaaacagtacgatacttat | 299382 | - | see also 6801 line | | |
| 43698 | DNAJB11 | NM_016306.3 | 739 | gacgaatgccctaatgtcaaact | 299394 | - | see also 6801 line | | |
| 43699 | DNAJB11 | NM_016306.3 | 222 | tgccggacgagatttctataaga | 299375 | - | see also 6801 line | | |
| 43700 | DNAJB12 | NM_017626.1 | 330 | ctgtgaaaagggtcaagcaatgt | 299409 | - | see also 19308 line | | |
| 43701 | DNAJB12 | NM_017626.1 | 896 | cacctgggtgtcgtctactatgt | 299412 | - | see also 19308 line | | |
| 43702 | DNAJB8 | NM_153330.1 | 775 | ctggcagctcggggttcaagtcg | 299416 | - | see also 19309 line | | |
| 43703 | DNAJB8 | NM_153330.1 | 656 | ctcggcaggctttggagaatttc | 299415 | - | see also 19309 line | | |
| 43704 | DNAJB9 | NM_012328.1 | 751 | ctctaccaatcagcatacagtac | 299429 | - | see also 19310 line | | |
| 43705 | DNAJB9 | NM_012328.1 | 595 | tggatccaagaagcgttttgaaa | 299428 | - | see also 19310 line | | |
| 43706 | DNAJB9 | NM_012328.1 | 438 | atgctaatagacgaaaagagtat | 299423 | - | see also 19310 line | | |
| 43707 | DNAJC8 | NM_014280.1 | 142 | ctcggttctaacttcgaaaaatc | 299434 | - | see also 19312 line | | |
| 43708 | DNAJC8 | NM_014280.1 | 284 | atcttggtgcatcctgacaaaaa | 299437 | - | see also 19312 line | | |
| 43709 | DNAJC8 | NM_014280.1 | 425 | tacgtggaacacactgtgaaaga | 299439 | - | see also 19312 line | | |
| 43710 | GRPEL1 | NM_025196.2 | 120 | ttgtgcacagccacgaaacaaaa | 299443 | - | - | chaperone | - |
| 43711 | GRPEL1 | NM_025196.2 | 325 | tggtggaggaggcaaaattatac | 299445 | - | see also 43710 line | | |
| 43712 | GRPEL1 | NM_025196.2 | 304 | tacggcagaggagccagaaattg | 299444 | - | see also 43710 line | | |
| 43713 | HKE2 | NM_014260.2 | 293 | ctgggtccggtgctagtcaaaca | 299452 | - | see also 19313 line | | |
| 43714 | HKE2 | NM_014260.2 | 269 | gggtccaacgtggtctttaaact | 299450 | - | see also 19313 line | | |
| 43715 | JDP1 | NM_021800.1 | 221 | aactatcttcggttgaacaaatc | 299458 | - | see also 19314 line | | |
| 43716 | JDP1 | NM_021800.1 | 622 | aagtcagtctccccgcaaaattc | 299461 | - | see also 19314 line | | |
| 43717 | JDP1 | NM_021800.1 | 631 | tccccgcaaaattcagattcttc | 299462 | - | see also 19314 line | | |
| 43718 | MGC26226 | NM_033105.3 | 481 | aacaacgcccacgcaatacttac | 299469 | - | see also 19315 line | | |
| 43719 | MGC26226 | NM_033105.3 | 491 | acgcaatacttaccgacatttca | 299470 | - | see also 19315 line | | |
| 43720 | PFDN2 | NM_012394.2 | 188 | agcacagcctagtgatcgataca | 299474 | - | see also 19316 line | | |
| 43721 | PFDN2 | NM_012394.2 | 262 | ctggtggagcgaactgtcaaaga | 299476 | - | see also 19316 line | | |
| 43722 | SCAP | NM_012235.1 | 1150 | ggccgacgctcttcagctattac | 299480 | - | see also 19317 line | | |
| 43723 | SCAP | NM_012235.1 | 1151 | gccgacgctcttcagctattaca | 299481 | - | see also 19317 line | | |
| 43724 | SCO1 | NM_004589.1 | 81 | tgcctcgcggactcgagttttgg | 299491 | - | see also 19318 line | | |
| 43725 | SCO1 | NM_004589.1 | 444 | ctcatactggggagcgtaaaact | 299496 | - | see also 19318 line | | |
| 43726 | SCO1 | NM_004589.1 | 448 | tactggggagcgtaaaactgaca | 299497 | - | see also 19318 line | | |

Figure 46

| ID | Gene | Accession | Pos | Sequence | Num | — | Note |
|---|---|---|---|---|---|---|---|
| 43727 | SIL1 | NM_022464.3 | 591 | tggtacggctgatcaacaagttc | 299513 | - | see also 19319 line |
| 43728 | SIL1 | NM_022464.3 | 648 | ctgcgctcttgatcttgaatat | 299519 | - | see also 19319 line |
| 43729 | WBSCR18 | NM_032317.2 | 495 | gcgccaacgcacgatgttcaac | 299535 | - | see also 19320 line |
| 43730 | WBSCR18 | NM_032317.2 | 341 | accctccgtcgcaagtatgatcg | 299534 | - | see also 19320 line |
| 43731 | APPBP2 | NM_006380.2 | 1921 | cggttgcgagatcggcaatattc | 299587 | - | see also 4473 line |
| 43732 | Dlc2 | NM_080677.1 | 428 | ttgtatcgtgggcgcgaaatttg | 299592 | - | see also 19322 line |
| 43733 | Dlc2 | NM_080677.1 | 272 | ccggaaggcagtgatcaagaaacg | 299590 | - | see also 19322 line |
| 43734 | DNCI2 | NM_001378.1 | 982 | gacgaacgttggtcaaagcatcg | 299600 | - | see also 947 line |
| 43735 | DNCI2 | NM_001378.1 | 1046 | tactcgtggcttcctataacaac | 299606 | - | see also 947 line |
| 43736 | FLJ32752 | NM_144666.1 | 2576 | cgctacaacgcgaccttgattgc | 299651 | - | see also 19325 line |
| 43737 | FLJ32752 | NM_144666.1 | 2791 | gcggacgcgagcagtgatgtacc | 299653 | - | see also 19325 line |
| 43738 | FLJ40427 | NM_178504.3 | 1170 | tggaaaagcgcctacgatattta | 299694 | - | see also 19326 line |
| 43739 | FLJ40427 | NM_178504.3 | 370 | taggcgttggcctttaatgattg | 299664 | - | see also 19326 line |
| 43740 | KLC2 | NM_022822.1 | 1393 | agcgggaggaaagcaaggataag | 299771 | - | see also 19327 line |
| 43741 | KLC2 | NM_022822.1 | 1811 | cagtgagatgctggtaaagaagc | 299773 | - | see also 19327 line |
| 43742 | KNS2 | NM_005552.3 | 1736 | tgctagggttctcgtaaacagg | 299804 | - | see also 19328 line |
| 43743 | KNS2 | NM_005552.3 | 1113 | ttggctattcgtgagaaaacttt | 299786 | - | see also 19328 line |
| 43744 | KNSL8 | NM_138343.1 | 270 | ttcgccgttctatggaaaacatt | 299806 | - | see also 19329 line |
| 43745 | KNSL8 | NM_138343.1 | 271 | tcgccgttctatggaaaacattg | 299807 | - | see also 19329 line |
| 43746 | TCTEL1 | NM_006519.1 | 279 | cactgtgcgatgggagaataaga | 299812 | - | microtubule motor |
| 43747 | DCTN1 | NM_004082.2 | 2881 | cccatgagcgctccttgatttc | 299824 | - | see also 19330 line |
| 43748 | DCTN1 | NM_004082.2 | 4232 | agctgagcgctgatcgtaaga | 299837 | - | see also 19330 line |
| 43749 | DCTN1 | NM_004082.2 | 1797 | gacgagctcactactgacttaga | 299816 | - | see also 19330 line |
| 43750 | DCTN2 | NM_006400.3 | 1272 | gacaaccatgcgtgaaaacctgg | 299855 | - | see also 19331 line |
| 43751 | DCTN2 | NM_006400.3 | 352 | ttctcagatcgtattggaaaaac | 299847 | - | see also 19331 line |
| 43752 | DNAI1 | NM_012144.2 | 552 | ggcagcattaccgcgatgaatta | 299861 | - | see also 19332 line |
| 43753 | DNAI1 | NM_012144.2 | 1912 | gacccgatgttcatctatgacc | 299873 | - | see also 19332 line |
| 43754 | DNAI2 | NM_023036.1 | 881 | tgggcatgagcagcgattcatac | 299884 | - | see also 19333 line |
| 43755 | DNAI2 | NM_023036.1 | 1009 | ggctgctacaatggacagatagc | 299889 | - | see also 19333 line |
| 43756 | DNALI1 | NM_003462.3 | 460 | ctcacagtgtttgattgagttga | 299908 | - | see also 19334 line |
| 43757 | DNALI1 | NM_003462.3 | 381 | tccaggagcagttagacttaaag | 299906 | - | see also 19334 line |
| 43758 | DNCI1 | NM_004411.1 | 890 | gaccggacaataccgggtaattga | 299923 | - | see also 2954 line |
| 43759 | DNCI1 | NM_004411.1 | 1531 | aacgggagacgtcaataacttcg | 299935 | - | see also 2954 line |
| 43760 | CIAO1 | NM_004804.2 | 827 | cgcctggcgtcttgtagtgatga | 299946 | - | see also 19335 line |
| 43761 | CIAO1 | NM_004804.2 | 412 | gtcccctgcgttaattacctgg | 299940 | - | see also 19335 line |
| 43762 | CIAO1 | NM_004804.2 | 822 | gccagcgcctggcgtcttgtagt | 299945 | - | see also 19335 line |
| 43763 | RBBP8 | NM_002894.1 | 1288 | tggagctacctcagtatcaaaa | 299975 | - | see also 19337 line |

Figure 46

| 43764 | RBBP8 | NM_002894.1 | 839 | aacccccatgtccgatacataga | 299961 | - | see also 19337 line |
|---|---|---|---|---|---|---|---|
| 43765 | RBBP8 | NM_002894.1 | 2058 | ctctacgtccacgtgaaagtttg | 299999 | - | see also 19337 line |
| 43766 | SMARCB1 | NM_003073.2 | 339 | tgcatcgtcacatggtaaaaaaa | 300024 | - | see also 2060 line |
| 43767 | SMARCB1 | NM_003073.2 | 450 | tggcaacgatgagaagtacaagg | 300026 | - | see also 2060 line |
| 43768 | THRSP | NM_003251.1 | 60 | tgcagccgaggtgcacaacatgg | 300030 | - | see also 19339 line |
| 43769 | THRSP | NM_003251.1 | 150 | ggctgaggcccctgatctctaca | 300031 | - | see also 19339 line |
| 43770 | RPC32 | NM_006467.1 | 183 | aggacgtgctgcttataccttta | 300034 | - | see also 19340 line |
| 43771 | RPC32 | NM_006467.1 | 182 | gaggacgtgctgcttataccttt | 300033 | - | see also 19340 line |
| 43772 | RPC62 | NM_006468.4 | 409 | gccgggtattgcgaatgcttaga | 300053 | - | see also 19341 line |
| 43773 | RPC62 | NM_006468.4 | 408 | agccgggtattgcgaatgcttag | 300052 | - | see also 19341 line |
| 43774 | RPC62 | NM_006468.4 | 433 | atccccggtacatctatactacc | 300055 | - | see also 19341 line |
| 43775 | AES | NM_001130.5 | 521 | tcccgagctgaactctatcatcc | 300102 | - | see also 19342 line |
| 43776 | AES | NM_001130.5 | 518 | cgctcccgagctgaactctatca | 300100 | - | see also 19342 line |
| 43777 | ASXL1 | NM_015338.3 | 3626 | atgtgcggtccgccaaaagatcc | 300161 | - | see also 19343 line |
| 43778 | ASXL1 | NM_015338.3 | 3111 | tggatacccatcccatcgaatga | 300155 | - | see also 19343 line |
| 43779 | BCL3 | NM_005178.2 | 340 | cacccctatacccatgatgtgc | 300182 | - | see also 19344 line |
| 43780 | BCL3 | NM_005178.2 | 502 | gccgggagctcgacatctacaac | 300184 | - | see also 19344 line |
| 43781 | BMI1 | NM_005180.5 | 820 | tgctgccaatggctctaatgaag | 300200 | - | see also 19345 line |
| 43782 | BMI1 | NM_005180.5 | 1430 | gccaatagacctcgaaaatcatc | 300214 | - | see also 19345 line |
| 43783 | BMI1 | NM_005180.5 | 1428 | ttgccaatagacctcgaaaatca | 300213 | - | see also 19345 line |
| 43784 | CCNH | NM_001239.2 | 830 | ttgacggatgcttaccttttata | 300251 | - | see also 19347 line |
| 43785 | CCNH | NM_001239.2 | 471 | tggggtacggcttgtatgtatttc | 300244 | - | see also 19347 line |
| 43786 | CCNT1 | NM_001240.2 | 2216 | aactcgtgtccctcattcgaaac | 300311 | - | see also 811 line |
| 43787 | CCNT1 | NM_001240.2 | 1366 | gtcatcggactagtgagaattta | 300288 | - | see also 811 line |
| 43788 | CCNT1 | NM_001240.2 | 472 | agcgtcttaacgtctcacaattg | 300269 | - | see also 811 line |
| 43789 | CCNT2 | NM_001241.2 | 1356 | aagcgtaaactagaaactcttga | 300364 | - | see also 19349 line |
| 43790 | CCNT2 | NM_001241.2 | 1797 | cacagtgccgacggaataccacc | 300372 | - | see also 19349 line |
| 43791 | DAT1 | NM_018640.2 | 499 | atcaaggaccggtatcttctaaa | 300379 | - | see also 19350 line |
| 43792 | DAT1 | NM_018640.2 | 825 | gacggactacgaggaaggtttaa | 300384 | - | see also 19350 line |
| 43793 | DAXX | NM_001350.2 | 1688 | cgcatagtgtcaccatcgttact | 300400 | - | see also 19351 line |
| 43794 | DAXX | NM_001350.2 | 812 | cgcctctttgggcgactatgtga | 300390 | - | see also 19351 line |
| 43795 | FLJ10201 | NM_018023.3 | 2543 | tgcgagctacgaacaatgctaat | 300468 | - | see also 19352 line |
| 43796 | FLJ10201 | NM_018023.3 | 3282 | cacagtatccggactgttaaaga | 300482 | - | see also 19352 line |
| 43797 | GTF2H2 | NM_001515.2 | 373 | cacctttatgtggtagtagatgg | 300499 | - | see also 19353 line |
| 43798 | GTF2H2 | NM_001515.2 | 451 | aagttgttggaatactttgtaga | 300502 | - | see also 19353 line |
| 43799 | JAZF1 | NM_175061.2 | 721 | ccccggtgtcggctgagattatc | 300513 | - | see also 19354 line |
| 43800 | JAZF1 | NM_175061.2 | 635 | tcgtgtccgcaaaccattcaagt | 300506 | - | see also 19354 line |

Figure 46

| 43801 | JJAZ1 | NM_015355.1 | 1738 | gtcgcaacggaccagttaagaga | 300542 | - | see also 6386 line |
|---|---|---|---|---|---|---|---|
| 43802 | JJAZ1 | NM_015355.1 | 1110 | aggcgcttacagctttagatgg | 300519 | - | see also 6386 line |
| 43803 | MADH9 | NM_005905.2 | 848 | gtgctcggtcgcctactatgaac | 300561 | - | see also 19356 line |
| 43804 | MADH9 | NM_005905.2 | 905 | ctcccgaagtgtgtcatagatg | 300565 | - | see also 19356 line |
| 43805 | MJD | NM_004993.2 | 362 | ctcaggatcgatcctataaatga | 300591 | - | see also 3484 line |
| 43806 | MJD | NM_004993.2 | 808 | cagggctattcagctaagttatgc | 300599 | - | see also 3484 line |
| 43807 | MLLT3 | NM_004529.1 | 1245 | aacgttaccgccatttgatgata | 300640 | - | see also 3082 line |
| 43808 | MLLT3 | NM_004529.1 | 1765 | cagcagatcgtgaaccttataga | 300655 | - | see also 3082 line |
| 43809 | NCOA5 | NM_020967.1 | 368 | ttcgggacgtgagggatcttaga | 300667 | - | see also 19359 line |
| 43810 | NCOA5 | NM_020967.1 | 508 | gaacgatacctacgaatggatga | 300673 | - | see also 19359 line |
| 43811 | NCOA5 | NM_020967.1 | 499 | ggctcttatgaccgatacctacg | 300672 | - | see also 19359 line |
| 43812 | PCQAP | NM_015889.2 | 278 | tccgtcagtgatcctatgaatgc | 300705 | - | see also 19360 line |
| 43813 | PCQAP | NM_015889.2 | 277 | ttccgtcagtgatcctatgaatg | 300704 | - | see also 19360 line |
| 43814 | PCQAP | NM_015889.2 | 193 | ggccaagacccgggacgaatacc | 300695 | - | see also 19360 line |
| 43815 | RBL1 | NM_002895.2 | 3076 | aagcgctcgctgtacaagttca | 300802 | - | see also 1930 line |
| 43816 | RBL1 | NM_002895.2 | 1261 | gagccggttacagatattgtgg | 300748 | - | see also 1930 line |
| 43817 | SEI1 | NM_013376.1 | 421 | cagtgtggctgaacaacttactgg | 300807 | - | see also 19362 line |
| 43818 | SEI1 | NM_013376.1 | 665 | acctctatgtatgacaatgaact | 300810 | - | see also 19362 line |
| 43819 | SIN3A | NM_015477.1 | 3561 | acgtggaggagatacatgaattca | 300887 | - | see also 6425 line |
| 43820 | SIN3A | NM_015477.1 | 2990 | tcccaagccgaacgcaaatga | 300873 | - | see also 6425 line |
| 43821 | SNAPC1 | NM_003082.2 | 506 | gtgatcgtgtgatgaaacttatc | 300906 | - | see also 2075 line |
| 43822 | SNAPC1 | NM_003082.2 | 703 | aagaatccatccttaaagtcaaa | 300909 | - | see also 2075 line |
| 43823 | TLE1 | NM_005077.3 | 1476 | agccatagacccctcgttaacc | 300922 | - | see also 19365 line |
| 43824 | TLE1 | NM_005077.3 | 2059 | cccgatggctgcactctcatagt | 300929 | - | see also 19365 line |
| 43825 | TLE2 | NM_003260.3 | 318 | gtcgtacgggctcaacattgaaa | 300935 | - | see also 19366 line |
| 43826 | TLE2 | NM_003260.3 | 375 | cggtatctgcgctcagattatcc | 300939 | - | see also 19366 line |
| 43827 | TLE3 | NM_005078.1 | 1033 | aggtcgatgccgggtaaacctcc | 300956 | - | see also 3546 line |
| 43828 | TRIP-Br2 | NM_014755.1 | 1045 | gacattgatacgtccatgtatga | 300973 | - | see also 5946 line |
| 43829 | TRIP-Br2 | NM_014755.1 | 480 | gaccgttttaattaacaacatgt | 300970 | - | see also 5946 line |
| 43830 | TRIP8 | NM_004241.1 | 6334 | aaggccggaatcaattagtcttga | 301153 | - | see also 19368 line |
| 43831 | TRIP8 | NM_004241.1 | 4178 | ctgccgatactaccagtgttcc | 301073 | - | see also 19368 line |
| 43832 | HSF2BP | NM_007031.1 | 363 | tgccggcacatgggaactaaga | 301178 | - | see also 19369 line |
| 43833 | HSF2BP | NM_007031.1 | 1311 | ctccgcactctggacgcataatgt | 301208 | - | see also 19369 line |
| 43834 | FLJ25348 | NM_144569.2 | 1505 | ctgccgcctgctctactcatacc | 301213 | - | see also 19370 line |
| 43835 | FLJ25348 | NM_144569.2 | 1109 | gagctgcggggacaatatcttcc | 301212 | - | see also 19370 line |
| 43836 | PWP1 | NM_007062.1 | 441 | tacggtcacggagtaatgatc | 301228 | - | see also 19371 line |
| 43837 | PWP1 | NM_007062.1 | 445 | grctacgggagtaatgatcaaga | 301229 | - | see also 19371 line |

Figure 46

| 43838 | NASP | NM_002482.2 | 1950 | cagaatggctgtactaaacgagc | 301276 | - | see also 1588 line |
|---|---|---|---|---|---|---|---|
| 43839 | NASP | NM_002482.2 | 2201 | cagacggtgcttcctcatcaaat | 301285 | - | see also 1588 line |
| 43840 | XRCC4 | NM_003401.2 | 874 | ctgctgtaagtaaagatgattcc | 301301 | - | see also 19373 line |
| 43841 | XRCC4 | NM_003401.2 | 163 | ctgaacccagtataactcatttt | 301291 | - | see also 19373 line |
| 43842 | ALKBH | NM_006020.1 | 511 | gaccgtaggctaccattataact | 301310 | - | see also 19374 line |
| 43843 | ALKBH | NM_006020.1 | 935 | ctcccgagagattcaatggtaga | 301316 | - | see also 19374 line |
| 43844 | FANCA | NM_000135.1 | 1825 | ctcccgtgtggcgtttatagagt | 301350 | - | see also 120 line |
| 43845 | FANCA | NM_000135.1 | 1823 | gactcccgtgtggcgtttataga | 301349 | - | see also 120 line |
| 43846 | FANCA | NM_000135.1 | 1827 | cccgtgtggcgtttatagagtct | 301351 | - | see also 120 line |
| 43847 | FANCC | NM_000136.1 | 796 | gcgtccctgtcacgagtttgtgt | 301403 | - | see also 121 line |
| 43848 | FANCC | NM_000136.1 | 1119 | tgccatattccgggttgttgatg | 301413 | - | see also 121 line |
| 43849 | FLJ10858 | NM_018248.1 | 948 | tgcaagctaccgactagaaatac | 301447 | - | see also 19377 line |
| 43850 | FLJ10858 | NM_018248.1 | 1561 | cagatggccctcgtaccttaaat | 301474 | - | see also 19377 line |
| 43851 | HUS1 | NM_004507.2 | 171 | accacttcacacgaatcagtaac | 301483 | - | see also 19378 line |
| 43852 | HUS1 | NM_004507.2 | 178 | cacacgaatcagtaacatgatag | 301484 | - | see also 19378 line |
| 43853 | NEIL1 | NM_024608.1 | 1300 | agggcgcaagtcccgcaaaaaga | 301510 | - | - | - | - |
| 43854 | NEIL1 | NM_024608.1 | 664 | cggcatgtccggctcttttcagc | 301509 | - | see also 43853 line |
| 43855 | NEIL1 | NM_024608.1 | 1309 | gtcccgcaaaaagaaatccaagg | 301511 | - | see also 43853 line |
| 43856 | NEIL2 | NM_145043.1 | 599 | agggccgttggtgaggaaatttc | 301513 | - | see also 19379 line |
| 43857 | NEIL2 | NM_145043.1 | 1270 | cagggctagggaacatcattaag | 301522 | - | see also 19379 line |
| 43858 | POLK | NM_016218.1 | 1367 | tccttgggtctaggttcaacaca | 301566 | - | see also 19380 line |
| 43859 | POLK | NM_016218.1 | 2459 | taccgccagccttacttatgtga | 301605 | - | see also 19380 line |
| 43860 | RAD9A | NM_004584.2 | 1015 | atcgccatggaaaccactatagg | 301625 | - | see also 19381 line |
| 43861 | RAD9A | NM_004584.2 | 1049 | cgcgggtgctgccctccatttcc | 301626 | - | see also 19381 line |
| 43862 | RBBP4 | NM_005610.1 | 1167 | cgggccaccagagttgttgttta | 301637 | - | see also 19382 line |
| 43863 | RBBP4 | NM_005610.1 | 1059 | gtcacctcacaatgagactattt | 301632 | - | see also 19382 line |
| 43864 | CDC45L | NM_003504.3 | 407 | aacgatacccagatcaaattact | 301648 | - | see also 2361 line |
| 43865 | GMNN | NM_015895.2 | 472 | atcggaatgaccacttaacatct | 301669 | - | see also 19384 line |
| 43866 | GMNN | NM_015895.2 | 920 | tacggatgcaaagccatgtatat | 301685 | - | see also 19384 line |
| 43867 | GMNN | NM_015895.2 | 592 | atccatcctctcagtattggaag | 301677 | - | see also 19384 line |
| 43868 | SPHAR | NM_006542.1 | 391 | ctcatttatgatagatttgcaag | 301690 | - | see also 19385 line |
| 43869 | SPHAR | NM_006542.1 | 383 | ttcaatgtctcatttatgataga | 301688 | - | see also 19385 line |
| 43870 | REC8L1 | NM_005132.1 | 651 | tcggtgtgatccgcgtctattct | 301691 | - | see also 19386 line |
| 43871 | REC8L1 | NM_005132.1 | 659 | atccgcgtctattctcaacaatg | 301693 | - | see also 19386 line |
| 43872 | CIDEA | NM_001279.2 | 562 | gacgggacagtttctcatctatc | 301712 | - | see also 19387 line |
| 43873 | CIDEA | NM_001279.2 | 558 | aggtgacgggacagtttctcatc | 301711 | - | see also 19387 line |
| 43874 | CDC40 | NM_015891.2 | 949 | atggactgtaaaattaagctatg | 301747 | - | see also 19388 line |

Figure 46

| 43875 | CDC40 | NM_015891.2 | 246 | tgccgtcaaagaagttcagtata | 301721 | - | see also 19388 line |
|---|---|---|---|---|---|---|---|
| 43876 | CRNKL1 | NM_016652.3 | 825 | tacgagcgtgctttagatgtaga | 301784 | - | see also 19389 line |
| 43877 | CRNKL1 | NM_016652.3 | 944 | aacgctgcctcgagttaatcagt | 301789 | - | see also 19389 line |
| 43878 | GEMIN4 | NM_015721.1 | 1830 | atggtgtcatgcctcaaagaaac | 301857 | - | see also 19390 line |
| 43879 | GEMIN4 | NM_015721.1 | 2292 | tcctggctccaccgcaagttaga | 301867 | - | see also 19390 line |
| 43880 | ARL6IP4 | NM_016638.1 | 608 | tggcgaggtcctagaggaaatcg | 301874 | - | see also 19391 line |
| 43881 | ARL6IP4 | NM_016638.1 | 613 | aggtcctagaggaaatcgtaacc | 301875 | - | see also 19391 line |
| 43882 | LUC7A | NM_016424.2 | 1217 | gccgggatcgaaagtcatataag | 301912 | - | see also 6850 line |
| 43883 | LUC7A | NM_016424.2 | 678 | aacgtcgacaattgaaagctttg | 301895 | - | see also 6850 line |
| 43884 | FLJ21172 | NM_024805.1 | 199 | agccgcgatgctgcgctatttcc | 301917 | - | see also 19393 line |
| 43885 | FLJ21172 | NM_024805.1 | 327 | gggttctcactcgacttacaaac | 301920 | - | see also 19393 line |
| 43886 | RNU3IP2 | NM_004704.2 | 1402 | atcaaagaggctcggaattctgt | 301944 | - | see also 19394 line |
| 43887 | RNU3IP2 | NM_004704.2 | 1301 | tccccctggtgggtttatcaac | 301941 | - | see also 19394 line |
| 43888 | HCA66 | NM_018428.1 | 1609 | tcctgcaatatggcgaacataag | 301987 | - | see also 19395 line |
| 43889 | HCA66 | NM_018428.1 | 1747 | atctactggcgagcgatgaaaat | 301992 | - | see also 19395 line |
| 43890 | HCA66 | NM_018428.1 | 411 | aactcgacttagcaaggtattct | 301957 | - | see also 19395 line |
| 43891 | PRPF39 | NM_017922.2 | 2423 | gtgcgtggtatcaatacaattat | 302044 | - | see also 7177 line |
| 43892 | PRPF39 | NM_017922.2 | 2421 | tagtgcgtggtatcaatacaatt | 302043 | - | see also 7177 line |
| 43893 | GLE1L | NM_001499.1 | 319 | gccctagatcaaccctcatttgt | 302052 | - | see also 19397 line |
| 43894 | GLE1L | NM_001499.1 | 450 | gtcctcacgggggatcaaagtgg | 302056 | - | see also 19397 line |
| 43895 | CD37 | NM_001774.1 | 419 | tgcgggacgtcgtagagaaaacc | 302081 | - | see also 19399 line |
| 43896 | CD37 | NM_001774.1 | 622 | accaacgactccacaatcctaga | 302082 | - | see also 19399 line |
| 43897 | CD37 | NM_001774.1 | 824 | tactcgagctcgggttcatgacg | 302084 | - | see also 19399 line |
| 43898 | GYPC | NM_002101.3 | 519 | cagcagaaaggagtactttattt | 302088 | - | see also 19400 line |
| 43899 | GYPC | NM_002101.3 | 520 | agcagaaaggagtactttatttg | 302089 | - | see also 19400 line |
| 43900 | TM4SF2 | NM_004615.2 | 433 | cacggacgctatgcagacttaca | 302097 | - | see also 19401 line |
| 43901 | TM4SF2 | NM_004615.2 | 437 | gacgctatgcagacttacaatgg | 302099 | - | see also 19401 line |
| 43902 | TM4SF4 | NM_004617.2 | 623 | tccacgacggggattatctcaat | 302120 | - | see also 19402 line |
| 43903 | TM4SF4 | NM_004617.2 | 590 | gcctcatggccaatagtacatgg | 302118 | - | see also 19402 line |
| 43904 | TM4SF4 | NM_004617.2 | 625 | cacgacggggattatctcaatga | 302121 | - | see also 19402 line |
| 43905 | TM4SF5 | NM_003963.2 | 587 | tgcggcgattgcaggaaaaaaca | 302128 | - | see also 19403 line |
| 43906 | TM4SF5 | NM_003963.2 | 589 | cggcgattgcaggaaaaaacagg | 302129 | - | see also 19403 line |
| 43907 | DAD1 | NM_001344.1 | 88 | gtcatttcgcggttcttagaaga | 302131 | - | see also 19404 line |
| 43908 | DAD1 | NM_001344.1 | 273 | agcggtttgcctgagaatacaga | 302137 | - | see also 19404 line |
| 43909 | SERP1 | NM_014445.2 | 385 | gtcgccaagacctcgagaaatgc | 302141 | - | see also 19405 line |
| 43910 | SERP1 | NM_014445.2 | 436 | tggttattggctctcttcatttt | 302143 | - | see also 19405 line |
| 43911 | UBQLN3 | NM_017481.2 | 257 | ccccgatcagcttgttctaatct | 302147 | - | see also 19406 line |

Figure 46

| 43912 | UBQLN3 | NM_017481.2 | 256 | accccgatcagcttgttctaatc | 302146 | - | see also 19406 line |
|---|---|---|---|---|---|---|---|
| 43913 | UBQLN3 | NM_017481.2 | 1274 | ccccgaggagtcagtagcaatca | 302164 | - | see also 19406 line |
| 43914 | APG12L | NM_004707.2 | 116 | agccgggaacaccaagtttcact | 302176 | - | see also 19407 line |
| 43915 | APG12L | NM_004707.2 | 487 | aaccatccaaggactcattgact | 302183 | - | see also 19407 line |
| 43916 | MGC47816 | NM_173642.1 | 1252 | cccgaacctggctacaacattaa | 302199 | - | see also 10102 line |
| 43917 | MGC47816 | NM_173642.1 | 1137 | tgcagagagcgtctataccatca | 302196 | - | see also 10102 line |
| 43918 | UBD | NM_006398.1 | 374 | tcgagactaagacgggtataatc | 302211 | - | see also 19409 line |
| 43919 | UBD | NM_006398.1 | 365 | aagcaatgatcgagactaagacg | 302210 | - | see also 19409 line |
| 43920 | CDC20 | NM_001255.1 | 1511 | gaggctatggcgctgttttgagt | 302217 | - | cell_cycle ⊢ ⊢ |
| 43921 | CDC20 | NM_001255.1 | 1236 | ggcaccagtgatcgacacattcg | 302216 | - | see also 43920 line |
| 43922 | CDC20 | NM_001255.1 | 1513 | ggctatggcgctgttttgagttg | 302218 | - | see also 43920 line |
| 43923 | CDC23 | NM_004661.2 | 1007 | gtgtagaaacgtgctgtgtaatt | 302251 | - | see also 3180 line |
| 43924 | CDC23 | NM_004661.2 | 231 | accgcctccgcctattacagagg | 302227 | - | see also 3180 line |
| 43925 | PSMD14 | NM_005805.1 | 1109 | ttggatactgtcgtatttaaata | 302304 | - | see also 4051 line |
| 43926 | PSMD8 | NM_002812.3 | 328 | aaccgtaaaagccccaatcttag | 302307 | - | see also 1830 line |
| 43927 | PSMD8 | NM_002812.3 | 329 | accgtaaaagccccaatcttagc | 302308 | - | see also 1830 line |
| 43928 | UK114 | NM_005836.1 | 486 | cactgacaacggcatcactataa | 302326 | - | see also 19413 line |
| 43929 | UK114 | NM_005836.1 | 173 | gtcgacaggaccatttacatttc | 302322 | - | see also 19413 line |
| 43930 | EEF1E1 | NM_004280.2 | 146 | gtctaacaggattgactactata | 302328 | - | see also 2868 line |
| 43931 | EEF1E1 | NM_004280.2 | 276 | tgggcactccagtaaaaatgaca | 302331 | - | see also 2868 line |
| 43932 | MRRF | NM_138777.2 | 744 | aggttcgcaccaactcaatgaac | 302339 | - | see also 19415 line |
| 43933 | PELO | NM_015946.4 | 2002 | tgcaggcaccgttaggatattct | 302358 | - | see also 19416 line |
| 43934 | PELO | NM_015946.4 | 1558 | ggctatccagcgccacatacact | 302344 | - | see also 19416 line |
| 43935 | TPR | NM_003292.1 | 2117 | tagttcgtcagcgtgatatgtac | 302402 | - | see also 2227 line |
| 43936 | TPR | NM_003292.1 | 5531 | gtggatccgttcgttctactagt | 302487 | - | see also 2227 line |
| 43937 | TRAM1 | NM_014294.3 | 454 | tgccgtaattcaagagtatatgt | 302537 | - | see also 5601 line |
| 43938 | TRAM1 | NM_014294.3 | 1075 | ggcatccatttgcgttactcagg | 302548 | - | see also 5601 line |
| 43939 | MGC14421 | NM_032907.2 | 565 | cgctaggggggctatagtatttca | 302561 | - | see also 19418 line |
| 43940 | MGC14421 | NM_032907.2 | 561 | tactcgctaggggggctatagtat | 302558 | - | see also 19418 line |
| 43941 | MGC14421 | NM_032907.2 | 563 | ctcgctaggggggctatagtattt | 302559 | - | see also 19418 line |
| 43942 | TRAM2 | NM_012288.1 | 1019 | tgcggcactggcggggaatactgg | 302601 | - | see also 5256 line |
| 43943 | TRAM2 | NM_012288.1 | 1155 | ctccccacggactaagaaactca | 302602 | - | see also 5256 line |
| 43944 | THEG | NM_016585.3 | 836 | cgcggatcctccagttgtcaaag | 302612 | - | see also 19420 line |

Figure 46

| | | | | | | | |
|---|---|---|---|---|---|---|---|
| 43945 | THEG | NM_016585.3 | 630 | gtctcggcccaagagattctacc | 302608 | - | see also 19420 line |
| 43946 | CD81 | NM_004356.2 | 240 | ctgtgggcgtgtcatgatgttc | 302614 | - | see also 19421 line |
| 43947 | CD81 | NM_004356.2 | 672 | tggtcgctgatcatgatcttc | 302617 | - | see also 19421 line |
| 43948 | LPXN | NM_004811.1 | 268 | cagetcgtgtatactaccaatat | 302624 | - | see also 3344 line |
| 43949 | LPXN | NM_004811.1 | 856 | aagccatattgccgaaaggattt | 302630 | - | see also 3344 line |
| 43950 | OCLN | NM_002538.2 | 1314 | atgtcgaggagtgtgttaaaaat | 302641 | - | see also 19423 line |
| 43951 | OCLN | NM_002538.2 | 1688 | agggaatatccacctatcactc | 302651 | - | see also 19423 line |
| 43952 | OXA1L | NM_005015.1 | 1325 | aacaacgcatgccggaatcagttg | 302689 | - | see also 19424 line |
| 43953 | OXA1L | NM_005015.1 | 955 | tccgatccatctacatattacc | 302679 | - | see also 19424 line |
| 43954 | PICALM | NM_007166.1 | 160 | agcctgacggaccgaatcactgc | 302693 | - | see also 4966 line |
| 43955 | PICALM | NM_007166.1 | 1740 | acctagcaagttagtatctgatg | 302733 | - | see also 4966 line |
| 43956 | PICALM | NM_007166.1 | 1959 | accaaacagcatgatgaggatatg | 302736 | - | see also 4966 line |
| 43957 | SLC9A3R1 | NM_004252.1 | 597 | cagggggctggcaacgaaaatga | 302744 | - | signal_transduction |
| 43958 | SLC9A3R1 | NM_004252.1 | 599 | gggggctggcaacgaaaatgagc | 302745 | - | see also 43957 line |
| 43959 | SLC9A3R1 | NM_004252.1 | 1160 | ctccgacccatcctagacttca | 302746 | - | see also 43957 line |
| 43960 | TM4SF7 | NM_003271.3 | 522 | ttcgctacacggacaagattga | 302748 | - | - |
| 43961 | TM4SF7 | NM_003271.3 | 524 | cgcctacacggacaagattgaca | 302749 | - | see also 43960 line |
| 43962 | TRAF1 | NM_005658.2 | 1060 | atcatgagagggagtatgatgc | 302753 | - | see also 19426 line |
| 43963 | TRAF1 | NM_005658.2 | 1044 | gtcgcttcatcgtgatcatga | 302752 | - | see also 19426 line |
| 43964 | VAMP3 | NM_004781.2 | 259 | tgggcaatcggattactgttct | 302763 | - | see also 3293 line |
| 43965 | VAMP3 | NM_004781.2 | 256 | atgtgggcaatcggattactgt | 302761 | - | see also 3293 line |
| 43966 | VAMP8 | NM_003761.2 | 285 | gtccttatctgcgtgattgttt | 302776 | - | see also 19429 line |
| 43967 | VAMP8 | NM_003761.2 | 209 | agccacatctgagcacttcaaga | 302775 | - | see also 19429 line |
| 43968 | DKFZp434O0515 | NM_178123.2 | 1075 | cccgctaccaggaagtttgtagg | 302802 | - | see also 19430 line |
| 43969 | DKFZp434O0515 | NM_178123.2 | 479 | tagaggatttaccgtgattgtgg | 302783 | - | see also 19430 line |
| 43970 | FKBP8 | NM_012181.2 | 982 | gaccgccttgaccggaaaatgc | 302834 | - | see also 19431 line |
| 43971 | FKBP8 | NM_012181.2 | 903 | agctggaaccttccaacaagacg | 302833 | - | see also 19431 line |
| 43972 | KIAA0372 | NM_014639.1 | 2003 | ggcgaggactatactatttgaaa | 302889 | - | see also 19432 line |
| 43973 | KIAA0372 | NM_014639.1 | 1410 | agcaattgctacgcttgatcaga | 302870 | - | see also 19432 line |
| 43974 | LOC57795 | NM_021165.1 | 2247 | acctacggagccgaatcaagtcc | 302997 | - | see also 19433 line |
| 43975 | LOC57795 | NM_021165.1 | 734 | aagtacggcactcatttcttact | 302987 | - | see also 19433 line |
| 43976 | PPIL6 | NM_173672.1 | 794 | agcaacggtcacaattctatat | 303025 | - | see also 19434 line |
| 43977 | PPIL6 | NM_173672.1 | 597 | aacgtggcataagactacattac | 303016 | - | see also 19434 line |
| 43978 | STUB1 | NM_005861.1 | 469 | ctcttcgaatcgcgaagaagaag | 303037 | - | see also 19435 line |
| 43979 | STUB1 | NM_005861.1 | 332 | gtctgtgaaggcgcacttcttcc | 303036 | - | see also 19435 line |

Figure 46

| 43980 | STUB1 | NM_005861.1 | 224 | cccgctggtggccgtgtattaca | 303034 | - | see also 19435 line | | |
|---|---|---|---|---|---|---|---|---|---|
| 43981 | LECT1 | NM_007015.1 | 224 | tccattacaccatgagtatcaat | 303043 | - | see also 19436 line | | |
| 43982 | LECT1 | NM_007015.1 | 830 | atcacgaaggaatctgttgtata | 303059 | - | see also 19436 line | | |
| 43983 | PTDSS2 | NM_030783.1 | 815 | ctggtggatgtgcatgatcatca | 303091 | - | see also 8946 line | | |
| 43984 | ELOVL1 | NM_022821.1 | 634 | ttccgtgcatgtcataatgtacc | 303103 | - | see also 19438 line | | |
| 43985 | ELOVL1 | NM_022821.1 | 631 | ctcttccgtgcatgtcataatgt | 303102 | - | see also 19438 line | | |
| 43986 | ELOVL3 | NM_152310.1 | 851 | cacaccacgatggaacacttatt | 303128 | - | see also 19439 line | | |
| 43987 | ELOVL3 | NM_152310.1 | 571 | gcgtaagcggccactcatcttta | 303119 | - | see also 19439 line | | |
| 43988 | PRKAB1 | NM_006253.4 | 519 | ctcggccaacggtgtttcgatgg | 303131 | - | see also 19440 line | | |
| 43989 | PRKAB1 | NM_006253.4 | 324 | agcggcatggtggccataagacg | 303129 | - | see also 19440 line | | |
| 43990 | PRKAB1 | NM_006253.4 | 695 | gacccttccgagcccatagtaac | 303135 | - | see also 19440 line | | |
| 43991 | C1orf17 | NM_015101.1 | 1977 | agccgagtacaaggagtattatg | 303162 | - | see also 6241 line | | |
| 43992 | C1orf17 | NM_015101.1 | 1978 | gccgagtacaaggagtattatga | 303163 | - | see also 6241 line | | |
| 43993 | CEECAM1 | NM_016174.3 | 833 | caccgttatgggtacatgaatgt | 303170 | - | see also 19442 line | | |
| 43994 | CEECAM1 | NM_016174.3 | 991 | agggtttgacgaggtctttgtca | 303172 | - | see also 19442 line | | |
| 43995 | FLJ22329 | NM_024656.2 | 1729 | acctcagtcgtatggaacaatga | 303187 | - | see also 8679 line | | |
| 43996 | FLJ22329 | NM_024656.2 | 1731 | ctcagtcgtatggaacaatgagc | 303188 | - | see also 8679 line | | |
| 43997 | COQ7 | NM_016138.3 | 370 | ttcagggtccggccaacagttct | 303198 | - | see also 19444 line | | |
| 43998 | COQ7 | NM_016138.3 | 118 | cggcggtccctctcagcttatgg | 303189 | - | see also 19444 line | | |
| 43999 | COQ4 | NM_016035.1 | 685 | ctccctcggtcgcgagtatctcc | 303209 | - | see also 19445 line | | |
| 44000 | COQ4 | NM_016035.1 | 521 | cacgacatggtcgcagttctagg | 303207 | - | see also 19445 line | | |
| 44001 | MATP | NM_016180.2 | 1625 | gtcgctctctttgttagatatgt | 303237 | - | see also 19446 line | | |
| 44002 | MATP | NM_016180.2 | 1306 | tgggctcttcccgaatgtctact | 303234 | - | see also 19446 line | | |
| 44003 | SILV | NM_006928.2 | 1429 | ctgtatcgatatggttccttttc | 303255 | - | see also 4859 line | | |
| 44004 | SILV | NM_006928.2 | 1373 | tggatggtacagccaccttaagg | 303252 | - | see also 4859 line | | |
| 44005 | GCHFR | NM_005258.2 | 142 | cactatggtgggcgatgaacagt | 303259 | - | - | - | - |
| 44006 | GCHFR | NM_005258.2 | 212 | ttgggaaacaacttttatgaata | 303260 | - | see also 44005 line | | |
| 44007 | FLJ31978 | NM_144669.1 | 466 | agccgctggggtacgattgattg | 303269 | - | see also 19448 line | | |
| 44008 | FLJ31978 | NM_144669.1 | 467 | gccgctggggtacgattgattgg | 303270 | - | see also 19448 line | | |
| 44009 | MGC35274 | NM_153374.1 | 831 | aactttgaacatcccagttatat | 303280 | | see also 19449 line | | |
| 44010 | MGC35274 | NM_153374.1 | 790 | gccaataaactgtttaccaatga | 303278 | - | see also 19449 line | | |
| 44011 | NCOA7 | NM_181782.1 | 1862 | ggcgggtgatcccataactgagg | 303329 | - | see also 10253 line | | |
| 44012 | NCOA7 | NM_181782.1 | 2625 | taccggaaatcggcatcactaga | 303348 | - | see also 10253 line | | |
| 44013 | OSBP2 | NM_030758.1 | 2343 | tccggctcgtgggatgaacaaat | 303374 | - | see also 19451 line | | |
| 44014 | OSBP2 | NM_030758.1 | 2342 | gtccggctcgtgggatgaacaaa | 303373 | - | see also 19451 line | | |

Figure 46

| | | | | | | | |
|---|---|---|---|---|---|---|---|
| 44015 | OSBPL10 | NM_017784.3 | 2631 | gggcgatggctgggtatacttca | 303420 | - | see also 7152 line |
| 44016 | OSBPL11 | NM_022776.3 | 2143 | atcgggttacagctgaagtaaag | 303468 | - | see also 8466 line |
| 44017 | OSBPL11 | NM_022776.3 | 1621 | gccgtaaggagccattgctaaa | 303453 | - | see also 8466 line |
| 44018 | OSBPL2 | NM_014835.2 | 1585 | ctccgactgcccagatatctact | 303502 | - | see also 6043 line |
| 44019 | OSBPL2 | NM_014835.2 | 1538 | gggaccccgactggttgtatgc | 303501 | - | see also 6043 line |
| 44020 | OSBPL3 | NM_015550.2 | 2735 | aaggctacgagcaatactatagc | 303556 | - | see also 19455 line |
| 44021 | OSBPL3 | NM_015550.2 | 1136 | acgtcctgcatcggacatactcg | 303518 | - | see also 19455 line |
| 44022 | OSBPL3 | NM_015550.2 | 2168 | ttgccatatacgcgtatgcatct | 303544 | - | see also 19455 line |
| 44023 | OSBPL6 | NM_032523.2 | 2930 | cccggttccggccagatcaaaga | 303640 | - | see also 9237 line |
| 44024 | OSBPL6 | NM_032523.2 | 3139 | aaggaacctgggtttagcaaagt | 303649 | - | see also 9237 line |
| 44025 | OSBPL7 | NM_017731.3 | 2437 | cccgaccatgagcgaaactcg | 303665 | - | see also 19457 line |
| 44026 | OSBPL7 | NM_017731.3 | 473 | gaggacggatccttcattatgc | 303654 | - | see also 19457 line |
| 44027 | OSBPL8 | NM_020841.3 | 2792 | aacatgtactccaatgcttagc | 303751 | - | see also 7988 line |
| 44028 | OSBPL8 | NM_020841.3 | 2545 | cgggttaactgcgagccataaatgc | 303742 | - | see also 7988 line |
| 44029 | OSBPL9 | NM_024586.3 | 1156 | gtcgcaggttagacttggaatgg | 303799 | - | see also 8649 line |
| 44030 | OSBPL9 | NM_024586.3 | 937 | atcccaaaggcgcttaatagatt | 303787 | - | see also 8649 line |
| 44031 | ASAH2 | NM_019893.1 | 1016 | ttggaccacgttgcatccaacaca | 303852 | - | see also 7751 line |
| 44032 | ASAH2 | NM_019893.1 | 1659 | atgcaacgtccatacacattaca | 303869 | - | see also 7751 line |
| 44033 | BTN3A1 | NM_007048.3 | 1325 | ttcagcctataatgaatggaaaa | 303880 | - | see also 19461 line |
| 44034 | CLU | NM_001831.1 | 195 | gtcaacgggtgaaacagataaa | 303891 | - | see also 19462 line |
| 44035 | CLU | NM_001831.1 | 193 | ctgtcaacgggtgaaacagata | 303890 | - | see also 19462 line |
| 44036 | CYB5-M | NM_030579.1 | 412 | atcataggcgctgttcttagg | 303909 | - | see also 8892 line |
| 44037 | CYB5-M | NM_030579.1 | 417 | aggcgtttctcttaggtttcc | 303910 | - | see also 8892 line |
| 44038 | CYBRD1 | NM_024843.2 | 195 | atgggagcgcactagagtttaac | 303911 | - | see also 19464 line |
| 44039 | CYBRD1 | NM_024843.2 | 322 | atccatccatgcagggttaaatg | 303913 | - | see also 19464 line |
| 44040 | CYP4Z1 | NM_178134.2 | 1192 | tccttacctgcaggaataactgt | 303927 | - | - |
| 44041 | FLJ20296 | NM_017750.2 | 409 | gggcagcattggccgttttatct | 303934 | - | see also 19465 line |
| 44042 | FLJ20296 | NM_017750.2 | 412 | cagcattggccgttttatcttgg | 303935 | - | see also 19465 line |
| 44043 | FLJ20296 | NM_017750.2 | 408 | aggcagcagcattggccgttttatc | 303933 | - | see also 19465 line |
| 44044 | FLJ23153 | NM_024636.1 | 767 | gacgtaatctaccccttatgttta | 303965 | - | see also 19466 line |
| 44045 | FLJ23153 | NM_024636.1 | 896 | tgctgccattctacaactgtacc | 303971 | - | see also 19466 line |
| 44046 | FLJ39035 | NM_182580.1 | 585 | ttctgggcatgtactcagtatgg | 303976 | - | see also 19467 line |
| 44047 | FLJ39035 | NM_182580.1 | 589 | gggcatgtactcagtatggttcc | 303977 | - | see also 19467 line |
| 44048 | KIAA0601 | NM_015013.1 | 948 | cagctcgacagttacaaagtttt | 303994 | - | see also 19468 line |
| 44049 | KIAA0601 | NM_015013.1 | 815 | gtcaccgagttcacagttattt | 303987 | - | see also 19468 line |
| 44050 | KIAA0601 | NM_015013.1 | 949 | agctcgacagttacaaagttttg | 303995 | - | see also 19468 line |
| 44051 | LDHD | NM_153486.2 | 929 | gcccgtagcccgccattgagttcc | 304041 | - | - |

Figure 46

| 44052 | LDHD | NM_153486.2 | 928 | tgcccgtagcccgcattgagttc | 304040 | - | see also 44051 line |
|---|---|---|---|---|---|---|---|
| 44053 | MGC20446 | NM_153611.2 | 979 | atccgtcatttcgggcattaatg | 304049 | - | see also 19469 line |
| 44054 | MGC20446 | NM_153611.2 | 980 | tccgtcatttcgggcattaatga | 304050 | - | see also 19469 line |
| 44055 | MGC25181 | NM_152783.2 | 1459 | tggtaacctgcacctcaatgtga | 304054 | - | see also 19470 line |
| 44056 | MGC25181 | NM_152783.2 | 775 | aggcctgcgtttcttcgatatg | 304052 | - | see also 19470 line |
| 44057 | PAOX | NM_152911.1 | 1506 | aagccacacatcgcacgtttac | 304069 | - | see also 19471 line |
| 44058 | PAOX | NM_152911.1 | 1507 | agccacacatcgcacgttttact | 304070 | - | see also 19471 line |
| 44059 | PAOX | NM_152911.1 | 942 | tcgtcacgtgcccttaggtttt | 304062 | - | see also 19471 line |
| 44060 | PCM1 | NM_006197.2 | 5157 | cagtaaggcgcatggttttgacc | 304242 | - | see also 4290 line |
| 44061 | PCM1 | NM_006197.2 | 3394 | cacgggtccttacagtgttatgc | 304175 | - | see also 4290 line |
| 44062 | SRPUL | NM_014467.1 | 579 | gagtccccgatggtgttataca | 304275 | - | see also 5698 line |
| 44063 | SRPUL | NM_014467.1 | 778 | gagatgccacgcactaccattca | 304281 | - | see also 5698 line |
| 44064 | STEAP2 | NM_152999.2 | 912 | agccgacaacaggttattgaaac | 304315 | - | see also 19474 line |
| 44065 | STEAP2 | NM_152999.2 | 1592 | atgtcgtctgctcataagtact | 304334 | - | see also 19474 line |
| 44066 | TSAP6 | NM_018234.1 | 1532 | gagggagagcaccatcaagttca | 304348 | - | see also 19475 line |
| 44067 | TSAP6 | NM_018234.1 | 1390 | tcgaggagagccgctacaagttc | 304346 | - | see also 19475 line |
| 44068 | FLJ10986 | NM_018291.2 | 1317 | tggtcaccggattgaaactgtct | 304365 | - | see also 19476 line |
| 44069 | FLJ10986 | NM_018291.2 | 582 | gggataaggcgggacatttcttt | 304353 | - | see also 19476 line |
| 44070 | KIAA1838 | NM_032448.1 | 788 | gcctccttggcaacgacataatc | 304386 | - | see also 19477 line |
| 44071 | KIAA1838 | NM_032448.1 | 291 | tggccagtggcgagaataacttt | 304379 | - | see also 19477 line |
| 44072 | MGC40579 | NM_152776.1 | 538 | ttgccgagtgcttcacttttca | 304436 | - | see also 19478 line |
| 44073 | MGC40579 | NM_152776.1 | 856 | gagggacacaagccacaatttg | 304448 | - | see also 19478 line |
| 44074 | KIAA0992 | NM_016081.2 | 2306 | cagccgagttaacatacgaaga | 304505 | - | see also 19479 line |
| 44075 | KIAA0992 | NM_016081.2 | 1017 | ctgcacctcgattcatccaaaag | 304471 | - | see also 19479 line |
| 44076 | WFS1 | NM_006005.2 | 2481 | gaccgctacaagtttgagattac | 304547 | - | see also 19480 line |
| 44077 | WFS1 | NM_006005.2 | 875 | cagcgagtccaagaactacacatcg | 304541 | - | see also 19480 line |
| 44078 | CDT6 | NM_021146.2 | 924 | aacctgcgctacgctgagtatag | 304557 | - | see also 19481 line |
| 44079 | CDT6 | NM_021146.2 | 655 | accgcatctctggagtgtataag | 304554 | - | see also 19481 line |
| 44080 | MTL5 | NM_004923.2 | 232 | tcgttcgcttcgaggaacatcg | 304562 | - | see also 19482 line |
| 44081 | MTL5 | NM_004923.2 | 991 | agccttaccatcggtagtcaacg | 304571 | - | see also 19482 line |
| 44082 | MTL5 | NM_004923.2 | 1022 | ttccctcgggatcaactcttcc | 304572 | - | see also 19482 line |
| 44083 | PDLIM1 | NM_020992.2 | 848 | gtggctgcgtcgattggaaatgc | 304592 | - | see also 19483 line |

Figure 46

| 44084 | PDLIM1 | NM_020992.2 | 218 | agcaaggcggctctagctaattt | 304581 | - | see also 19483 line |
|---|---|---|---|---|---|---|---|
| 44085 | CAMP | NM_004345.3 | 612 | aaggatttttgcggaatcttgt | 304605 | - | see also 19484 line |
| 44086 | CAMP | NM_004345.3 | 598 | ttgtccagagaatcaaggatttt | 304603 | - | see also 19484 line |
| 44087 | CAMP | NM_004345.3 | 504 | gacatcagttgtgataaggataa | 304600 | - | see also 19484 line |
| 44088 | DEFA5 | NM_021010.1 | 270 | ccctctccggggtgtgtgaaatc | 304608 | - | see also 19486 line |
| 44089 | DEFA5 | NM_021010.1 | 275 | tccggggtgtgtgaaatcagtgg | 304609 | - | see also 19486 line |
| 44090 | DEFA6 | NM_001926.2 | 305 | cactgtcatgggtattaaccaca | 304615 | - | see also 19487 line |
| 44091 | DEFA6 | NM_001926.2 | 122 | tccactgcaggcaaaagcttatg | 304611 | - | see also 19487 line |
| 44092 | DEFB1 | NM_005218.2 | 125 | ggcctcaggtggtaactttctca | 304616 | - | see also 19488 line |
| 44093 | DEFB103 | NM_018661.2 | 274 | caggtcatggaggaatcataaac | 304621 | - | see also 19489 line |
| 44094 | DEFB103 | NM_018661.2 | 272 | tccaggtcatggaggaatcataa | 304620 | - | see also 19489 line |
| 44095 | DEFB104 | NM_080389.1 | 117 | gcccgttgccggaagaaatgtcg | 304630 | - | see also 19490 line |
| 44096 | DEFB104 | NM_080389.1 | 206 | gagcttactgaatcgtacaaaac | 304635 | - | see also 19490 line |
| 44097 | DEFB106 | NM_152251.2 | 179 | gccatgtgggagcattatagatt | 304639 | - | see also 19491 line |
| 44098 | DEFB106 | NM_152251.2 | 141 | ctctgccagaagtctctgaaatg | 304637 | - | see also 19491 line |
| 44099 | DEFB118 | NM_054112.1 | 186 | tgctgcattccatccaatgaaga | 304641 | - | see also 19492 line |
| 44100 | DEFB118 | NM_054112.1 | 288 | aggttcacgacagactactttga | 304645 | - | see also 19492 line |
| 44101 | DEFB119 | NM_153289.2 | 222 | ttgtagggcctcttgcaaaaaga | 304648 | - | -  \|  -  \|  - |
| 44102 | DEFB119 | NM_153289.2 | 290 | tccagtcctacatgaggataagc | 304651 | - | see also 44101 line |
| 44103 | DEFB119 | NM_153289.2 | 248 | aacagccctacctctattgcaga | 304649 | - | see also 44101 line |
| 44104 | DEFB123 | NM_153324.2 | 144 | ctccaggtggcacccaaagatgc | 304654 | - | see also 19493 line |
| 44105 | DEFB123 | NM_153324.2 | 215 | gtctatgtttactgcataaataa | 304656 | - | see also 19493 line |
| 44106 | DEFB125 | NM_153325.1 | 128 | ttctttgtaggaacaagctatca | 304662 | - | see also 19494 line |
| 44107 | DEFB125 | NM_153325.1 | 261 | cccagtatctatgttgaatgatc | 304666 | - | see also 19494 line |
| 44108 | DEFB127 | NM_139074.2 | 42 | acccacagtaaccgaacaactta | 304670 | - | see also 19495 line |
| 44109 | DEFB127 | NM_139074.2 | 64 | aagaagtgctggaataactatgt | 304673 | - | see also 19495 line |
| 44110 | DEFB129 | NM_080831.2 | 264 | aagaattctagtgctgtgataca | 304680 | - | see also 19496 line |
| 44111 | DEFB129 | NM_080831.2 | 438 | accaagagtaacaccaaagaaag | 304684 | - | see also 19496 line |
| 44112 | GNLY | NM_006433.2 | 436 | ggcgcgacgtctgcagaaatttc | 304691 | - | see also 19497 line |
| 44113 | GNLY | NM_006433.2 | 525 | gaggacctcaggttgtgtatacc | 304692 | - | see also 19497 line |
| 44114 | HTN1 | NM_002159.2 | 204 | atggggactatggatcaaattat | 304696 | - | see also 19498 line |
| 44115 | HTN1 | NM_002159.2 | 181 | ttcacatcgagaatttccatttt | 304694 | - | see also 19498 line |
| 44116 | HTN3 | NM_000200.1 | 190 | atcgaggctatagatcaaattat | 304700 | - | see also 19499 line |
| 44117 | HTN3 | NM_000200.1 | 117 | atgactggagctgattcacatgc | 304699 | - | see also 19499 line |
| 44118 | LEAP-2 | NM_052971.1 | 1477 | cacggagaatgaccccattttgg | 304702 | - | see also 19500 line |
| 44119 | C7orf10 | NM_024728.1 | 869 | accaccttcgggtacacaataga | 304732 | - | see also 8717 line |
| 44120 | C7orf10 | NM_024728.1 | 871 | caccttcgggtacacaatagaaa | 304733 | - | see also 8717 line |

# Figure 46

| 44121 | C7orf10 | NM_024728.1 | 974 | tcccgtatggcccaatcaacaac | 304739 | - | see also 8717 line |
|-------|---------|-------------|-----|-------------------------|--------|---|--------------------|
| 44122 | INSIG1 | NM_005542.3 | 966 | ctgaaaagccccatagtgattga | 304767 | - | see also 19502 line |
| 44123 | INSIG1 | NM_005542.3 | 504 | aggtgatcgccaccatctttcc | 304754 | - | see also 19502 line |
| 44124 | PRNP | NM_000311.2 | 629 | cacgactgcgtcaatatcacaat | 304778 | - | see also 19503 line |
| 44125 | PRNP | NM_000311.2 | 694 | caccgagaccgacgttaagatga | 304781 | - | see also 19503 line |
| 44126 | ATP6V0A4 | NM_020632.1 | 2586 | gaggaatcgtcggggttttatt | 304818 | - | see also 19504 line |
| 44127 | ATP6V0A4 | NM_020632.1 | 1649 | tacacgggtttgatctacaatga | 304806 | - | see also 19504 line |
| 44128 | ATP6V0D2 | NM_152565.1 | 714 | ggccgacagacgtgcttttatca | 304844 | - | see also 9882 line |
| 44129 | ATP6V0D2 | NM_152565.1 | 870 | agcggatcattacggagtataca | 304848 | - | see also 9882 line |
| 44130 | BAT3 | NM_004639.2 | 2720 | cacggggctagaagagtatgtgc | 304889 | - | see also 3153 line |
| 44131 | BAT3 | NM_004639.2 | 2973 | ggccgaattcgtcgtatgtctcg | 304893 | - | see also 3153 line |
| 44132 | AP1GBP1 | NM_007247.3 | 1061 | gccttagctaatcgaactacacc | 304915 | - | see also 5048 line |
| 44133 | AP1GBP1 | NM_007247.3 | 1120 | agccatgatagcggtaacacaga | 304916 | - | see also 5048 line |
| 44134 | AP1GBP1 | NM_007247.3 | 2876 | tccaaagtaacgacctttgtaag | 304952 | - | see also 5048 line |
| 44135 | BRDG1 | NM_012108.1 | 543 | cagagtacgtccgtggaaaaaga | 304980 | - | see also 19507 line |
| 44136 | BRDG1 | NM_012108.1 | 545 | gagtacgtccgtggaaaaagaga | 304981 | - | see also 19507 line |
| 44137 | BRDG1 | NM_012108.1 | 199 | accgggagtatgagcattactgg | 304974 | - | see also 19507 line |
| 44138 | STAM2 | NM_005843.3 | 820 | atggtacgtcatcgaacaaaaac | 305003 | - | see also 19508 line |
| 44139 | STAM2 | NM_005843.3 | 1417 | aagctctggaactatataacaaa | 305022 | - | see also 19508 line |
| 44140 | STN2 | NM_033104.2 | 1541 | gaccgatcttcgtcaaactgaca | 305060 | - | see also 9358 line |
| 44141 | STN2 | NM_033104.2 | 2588 | accgactgccggacaaaaactca | 305074 | - | see also 9358 line |
| 44142 | SEC22L1 | NM_004892.3 | 501 | gacagtcgtgctcgaagaaatct | 305095 | - | see also 3394 line |
| 44143 | SEC22L1 | NM_004892.3 | 664 | aggatgcgaagtacttgaacatg | 305100 | - | see also 3394 line |
| 44144 | SEC22L3 | NM_004206.2 | 804 | ttcctttcgtagcctgcattttc | 305116 | - | see also 19511 line |
| 44145 | SEC22L3 | NM_004206.2 | 437 | tccaggccatacgcttttcttga | 305109 | - | see also 19511 line |
| 44146 | RAB9P40 | NM_005833.2 | 795 | acctccactgcattgatataagt | 305129 | - | see also 19512 line |
| 44147 | RAB9P40 | NM_005833.2 | 1020 | tggaccattccatgtgtatcatt | 305134 | - | see also 19512 line |
| 44148 | ELMO2 | NM_022086.6 | 907 | acctacgccattgcactgattaa | 305148 | - | see also 19513 line |
| 44149 | ELMO2 | NM_022086.6 | 1636 | gtcgtccgagagcaaatcactcg | 305157 | - | see also 19513 line |
| 44150 | ELMO3 | NM_024712.2 | 1106 | cgcgtacagccggtttgtgttgg | 305176 | - | see also 19514 line |
| 44151 | ELMO3 | NM_024712.2 | 1105 | gcgcgtacagccggtttgtgttg | 305175 | - | see also 19514 line |
| 44152 | AMPH | NM_001635.2 | 2134 | aacttcacccgacgcttagatta | 305217 | - | see also 19515 line |
| 44153 | AMPH | NM_001635.2 | 1221 | atggacgacaagcactgatttgg | 305203 | - | see also 19515 line |

Figure 46

| | | | | | | | |
|---|---|---|---|---|---|---|---|
| 44154 | AP4E1 | NM_007347.2 | 1718 | ctcaggcgcactcttctaataca | 305262 | - | see also 19516 line |
| 44155 | AP4E1 | NM_007347.2 | 2249 | atgtagatcaagctataactaaa | 305275 | - | see also 19516 line |
| 44156 | BIN1 | NM_004305.2 | 1027 | ctcaaccagaacctcaatgatgt | 305311 | - | see also 19517 line |
| 44157 | BIN1 | NM_004305.2 | 1478 | tgcccccaggtttcatgttcaag | 305313 | - | see also 19517 line |
| 44158 | BIN2 | NM_016187.1 | 309 | aaggccatcgtatggaataatga | 305320 | - | see also 19518 line |
| 44159 | BIN2 | NM_016187.1 | 610 | gtcgtattggctgctatgtgacc | 305324 | - | see also 19518 line |
| 44160 | BIN3 | NM_018688.3 | 346 | gactgtgatcgagcccttaaaaa | 305342 | - | see also 19519 line |
| 44161 | BIN3 | NM_018688.3 | 180 | acgcagacctggccatgtcaaaa | 305338 | - | see also 19519 line |
| 44162 | EPN1 | NM_013333.1 | 1237 | ggcgtgaacgtgcgtgagaaagc | 305345 | - | see also 19520 line |
| 44163 | EPN1 | NM_013333.1 | 2005 | cccgacgagttctctgactttga | 305346 | - | see also 19520 line |
| 44164 | HOOK2 | NM_013312.1 | 588 | agccagtcccgcaggtactattt | 305351 | - | see also 19521 line |
| 44165 | HOOK2 | NM_013312.1 | 2152 | agcagcggctggcaaccaattct | 305360 | - | see also 19521 line |
| 44166 | HOOK2 | NM_013312.1 | 589 | gccagtcccgcaggtactatttc | 305352 | - | see also 19521 line |
| 44167 | PACSIN3 | NM_016223.3 | 1086 | gacaatcagccggaaagagaagg | 305364 | - | - |
| 44168 | PACSIN3 | NM_016223.3 | 863 | ccgagcgccagcggcttcttttc | 305362 | - | see also 44167 line |
| 44169 | PACSIN3 | NM_016223.3 | 875 | ggcttcttttcttcaaggatatg | 305363 | - | see also 44167 line |
| 44170 | CPLX2 | NM_006650.2 | 422 | aggaccccgacgcgcagaaaaag | 305365 | - | see also 19522 line |
| 44171 | CPLX2 | NM_006650.2 | 426 | ccccgacgcgcagaaaaaggagg | 305366 | - | see also 19522 line |
| 44172 | YKT6 | NM_006555.3 | 359 | gtctacgtccggaatgatagtct | 305374 | - | see also 19523 line |
| 44173 | YKT6 | NM_006555.3 | 362 | tacgtccggaatgatagtcttgc | 305375 | - | see also 19523 line |
| 44174 | LRMP | NM_006152.1 | 847 | ttcatcgacagacggtactataa | 305388 | - | see also 19524 line |
| 44175 | LRMP | NM_006152.1 | 857 | gacggtactataacttcaagtga | 305390 | - | see also 19524 line |
| 44176 | CADPS | NM_003716.2 | 1589 | ggctccaaatcgcatcgtatatt | 305436 | - | see also 2539 line |
| 44177 | CADPS | NM_003716.2 | 3220 | ttctccgtactgactataatttg | 305470 | - | see also 2539 line |
| 44178 | CGI-141 | NM_016072.2 | 368 | gtcgttgttggctttattagaag | 305512 | - | see also 6649 line |
| 44179 | GOLGA4 | NM_002078.2 | 996 | tggcccgatgaatgttgatgtac | 305541 | - | see also 1344 line |
| 44180 | GOLGA4 | NM_002078.2 | 5605 | caggagcgcttaataaagctaga | 305650 | - | see also 1344 line |
| 44181 | GLTP | NM_016433.2 | 678 | ggcgaccatcgatgtcatctacg | 305709 | - | see also 6858 line |
| 44182 | GLTP | NM_016433.2 | 656 | gcctcttcctagtcaactacacg | 305708 | - | see also 6858 line |
| 44183 | FLJ23790 | NM_144963.1 | 825 | cagctttcttatagaactagaaa | 305716 | - | see also 19529 line |
| 44184 | FLJ23790 | NM_144963.1 | 323 | ttgaaactctactctataagata | 305712 | - | see also 19529 line |
| 44185 | LAPTM4A | NM_014713.2 | 174 | accgcagtgaccggttctacagc | 305719 | - | see also 19530 line |
| 44186 | LAPTM4A | NM_014713.2 | 773 | cagtacgttttgccaacctatga | 305739 | - | see also 19530 line |
| 44187 | NPTX1 | NM_002522.1 | 1409 | aggcctgtcgccagatcaactga | 305751 | - | see also 1619 line |
| 44188 | NPTX1 | NM_002522.1 | 826 | ttcccactgcggaccaactatat | 305744 | - | see also 1619 line |
| 44189 | NUP62 | NM_012346.3 | 754 | cccaaccctccggtttcaacatt | 305759 | - | see also 19532 line |
| 44190 | NUP62 | NM_012346.3 | 912 | cccagcctctttgcgtcaatagc | 305760 | - | see also 19532 line |

Figure 46

| 44191 | NUP62 | NM_012346.3 | 465 | cagacgacaggcttcactttgg | 305754 | - | see also 19532 line | | |
|---|---|---|---|---|---|---|---|---|---|
| 44192 | STX17 | NM_017919.1 | 270 | ttcagcaactccgatccaatatc | 305774 | - | see also 7174 line | | |
| 44193 | STX6 | NM_005819.3 | 447 | tacaagtactcggcaagttgtca | 305791 | - | see also 19534 line | | |
| 44194 | STX6 | NM_005819.3 | 836 | ttgcaaaagtatctcatatgacc | 305798 | - | see also 19534 line | | |
| 44195 | STX8 | NM_004853.1 | 169 | ttctccacatacgattctacttg | 305800 | - | see also 19535 line | | |
| 44196 | STX8 | NM_004853.1 | 172 | tccacatacgattctacttgtca | 305801 | - | see also 19535 line | | |
| 44197 | TRAPPC3 | NM_014408.3 | 592 | caggcggattgaggacaatcttc | 305833 | - | see also 19536 line | | |
| 44198 | TRAPPC3 | NM_014408.3 | 570 | cagaaatccggatgagattcatc | 305832 | - | see also 19536 line | | |
| 44199 | ASC | NM_013258.3 | 607 | atcgcgagggtcacaaacgttga | 305836 | - | apoptosis、signal_transduction、cell_cycle | - | - |
| 44200 | DLEU1 | NM_005887.1 | 347 | tacaggggttatcgcacttaagc | 305843 | - | see also 19537 line | | |
| 44201 | DLEU1 | NM_005887.1 | 361 | cacttaagcctcggaacaacttt | 305844 | - | see also 19537 line | | |
| 44202 | DLEU1 | NM_005887.1 | 303 | accgccctgccggcttgtagagc | 305840 | - | see also 19537 line | | |
| 44203 | DLEU2 | NM_006021.1 | 456 | ctcattgaatactatcaaaaagg | 305847 | - | cell_cycle | - | - |
| 44204 | PDAP2 | NM_007275.1 | 380 | agctgaggcgagtgcataagaat | 305848 | - | see also 19538 line | | |
| 44205 | PDAP2 | NM_007275.1 | 382 | ctgaggcgagtgcataagaatct | 305849 | - | see also 19538 line | | |
| 44206 | SASH1 | NM_015278.2 | 2396 | ctcctggatcggattaacctaaa | 305877 | - | see also 6310 line | | |
| 44207 | SASH1 | NM_015278.2 | 2397 | tcctggatcggattaacctaaaa | 305878 | - | see also 6310 line | | |
| 44208 | TSC1 | NM_000368.2 | 491 | ctcgttactgggtcatgtcataa | 305905 | - | see also 297 line | | |
| 44209 | TSC1 | NM_000368.2 | 288 | gacgacgtgacagctgtctttaa | 305894 | - | see also 297 line | | |
| 44210 | TSC1 | NM_000368.2 | 3496 | ctcgagagttatttcgtaataag | 305956 | - | see also 297 line | | |
| 44211 | BTG4 | NM_017589.2 | 573 | atcagttatgccgttagtagagc | 305974 | - | see also 19541 line | | |
| 44212 | BTG4 | NM_017589.2 | 669 | gtcagcaatccgaagagtattta | 305979 | - | see also 19541 line | | |
| 44213 | C10orf7 | NM_006023.1 | 508 | cacgcttacggcaccagaatttc | 305987 | - | see also 4202 line | | |
| 44214 | C10orf7 | NM_006023.1 | 612 | gggatgcgtattggatagcaatg | 305992 | - | see also 4202 line | | |
| 44215 | CUL2 | NM_003591.2 | 1621 | agcaagctacatcggatgtatac | 306061 | - | see also 2398 line | | |
| 44216 | CUL2 | NM_003591.2 | 2255 | ctgctatagttcgtatcatgaaa | 306090 | - | see also 2398 line | | |
| 44217 | CUL4A | NM_003589.1 | 808 | gccggacctcgcacagatgtacc | 306205 | - | see also 19545 line | | |
| 44218 | CUL4A | NM_003589.1 | 592 | tgccgaaggccaaaggttaatgc | 306200 | - | see also 19545 line | | |
| 44219 | GAR17 | NM_139285.1 | 1918 | ccctcgcagtggggtctacatcc | 306245 | - | see also 19546 line | | |
| 44220 | GAR17 | NM_139285.1 | 1277 | cccacatcttgggttcatgaaga | 306241 | - | see also 19546 line | | |
| 44221 | GAS2 | NM_005256.2 | 438 | atgaaacgtgtctatttgaatcg | 306264 | - | see also 3698 line | | |
| 44222 | GAS2 | NM_005256.2 | 182 | gccttgtggttaaccaatctatt | 306252 | - | see also 3698 line | | |
| 44223 | GAS2L1 | NM_006478.3 | 656 | cccacgcctcgtgcagtttgagc | 306277 | - | see also 4546 line | | |
| 44224 | GAS2L1 | NM_006478.3 | 542 | gccggaggtgctcatgtttgaga | 306276 | - | see also 4546 line | | |
| 44225 | LOC283431 | NM_174942.1 | 1321 | ctctatgtcagtccgttctaaat | 306326 | - | see also 19548 line | | |

Figure 46

| | | | | | | | | |
|---|---|---|---|---|---|---|---|---|
| 44226 | LOC283431 | NM_174942.1 | 1587 | atctgcccgagtccacacttttg | 306335 | - | see also 19548 line |
| 44227 | PPP1R15A | NM_014330.2 | 1759 | ttccgagtggccatctatgtacc | 306357 | - | see also 19549 line |
| 44228 | PPP1R15A | NM_014330.2 | 931 | gccacggaggataaaagaacaga | 306350 | - | see also 19549 line |
| 44229 | REPRIMO | NM_019845.1 | 382 | gacgagcgtagcctgtacataat | 306358 | - | see also 19550 line |
| 44230 | SESN1 | NM_014454.1 | 535 | atcgtcactacattggaataatg | 306378 | - | see also 19551 line |
| 44231 | SESN1 | NM_014454.1 | 1275 | ttggtaaatcgcctttatccaga | 306406 | - | see also 19551 line |
| 44232 | SESN2 | NM_031459.3 | 1606 | agcggaacctcaaggtctatatc | 306426 | - | see also 9035 line |
| 44233 | SESN2 | NM_031459.3 | 1655 | gaccacccgaagaatgtacaacc | 306427 | - | see also 9035 line |
| 44234 | SESN3 | NM_144665.2 | 888 | tggattcaggctaatatcagtca | 306448 | - | see also 19553 line |
| 44235 | SESN3 | NM_144665.2 | 426 | atccggtcgtctggacaacatca | 306433 | - | see also 19553 line |
| 44236 | BCCIP | NM_016567.2 | 218 | atgattatgacggaattaagaaa | 306480 | - | see also 19554 line |
| 44237 | BCCIP | NM_016567.2 | 461 | tacgcttctgtgagaagaactgt | 306487 | - | see also 19554 line |
| 44238 | CCNA1 | NM_003914.2 | 1069 | atcaccgatgatacatacacaaa | 306538 | - | see also 19555 line |
| 44239 | CCNA1 | NM_003914.2 | 1061 | ttgtctatatcaccgatgataca | 306537 | - | see also 19555 line |
| 44240 | CCNA2 | NM_001237.2 | 1223 | ggcctgaatcattaatacgaaag | 306589 | - | see also 809 line |
| 44241 | CCNA2 | NM_001237.2 | 150 | gggctcggcgctgctagcattgc | 306552 | - | see also 809 line |
| 44242 | CCNE2 | NM_004702.2 | 876 | tggatggtacttttgtcaatgt | 306607 | - | see also 19557 line |
| 44243 | CCNE2 | NM_004702.2 | 564 | atcttaaggatggaactcattat | 306597 | - | see also 19557 line |
| 44244 | CCNG1 | NM_004060.3 | 1046 | ggcgtactgcacggcaattgaag | 306647 | - | see also 2736 line |
| 44245 | CCNG1 | NM_004060.3 | 1066 | aagcatagctactacagaataac | 306651 | - | see also 2736 line |
| 44246 | RGC32 | NM_014059.1 | 318 | gagagtgcagattcactttatag | 306654 | - | see also 19559 line |
| 44247 | RGC32 | NM_014059.1 | 412 | ctgtcactcctcagaaagctaaa | 306656 | - | see also 19559 line |
| 44248 | BUB3 | NM_004725.1 | 619 | ctgcatacgagcgtttccaaaca | 306675 | - | see also 19560 line |
| 44249 | BUB3 | NM_004725.1 | 73 | gaccggttctaacgagttcaagc | 306657 | - | see also 19560 line |
| 44250 | MAD2L2 | NM_006341.2 | 281 | acccggagctgaatcagtatatc | 306690 | - | see also 19561 line |
| 44251 | MAD2L2 | NM_006341.2 | 123 | cacacgacaagacctcaactttg | 306688 | - | see also 19561 line |
| 44252 | CHFR | NM_018223.1 | 1345 | gacagcagtccaggattacgtgt | 306706 | - | see also 7328 line |
| 44253 | CHFR | NM_018223.1 | 1342 | gacgacagcagtccaggattacg | 306705 | - | see also 7328 line |
| 44254 | GML | NM_002066.1 | 151 | tgcgcgctcagtggacttacagt | 306722 | - | see also 19564 line |
| 44255 | GML | NM_002066.1 | 450 | gacatgttacccgatgaagtaac | 306743 | - | see also 19564 line |
| 44256 | GML | NM_002066.1 | 153 | cgcgctcagtggacttacagttt | 306723 | - | see also 19564 line |
| 44257 | CCNG2 | NM_004354.1 | 512 | tccggattagtcagtgtaaatgt | 306763 | - | see also 2913 line |
| 44258 | CDC16 | NM_003903.2 | 579 | ttgtcttctacgcgggaaaatct | 306788 | - | see also 2660 line |
| 44259 | CDC16 | NM_003903.2 | 677 | aagcgttcgatcttttaacatca | 306794 | - | see also 2660 line |
| 44260 | CENPF | NM_016343.2 | 1123 | gaccaggcgtcaaccaagtatac | 306863 | - | see also 19567 line |
| 44261 | CENPF | NM_016343.2 | 1124 | accaggcgtcaaccaagtatact | 306864 | - | see also 19567 line |

Figure 46

| 44262 | ANAPC7 | NM_016238.1 | 613 | tggatcaaagcgtatgcttttgt | 307035 | - | see also 6744 line | | |
|---|---|---|---|---|---|---|---|---|---|
| 44263 | ANAPC7 | NM_016238.1 | 1102 | ctcgcaccttgtcgcttagattg | 307045 | - | see also 6744 line | | |
| 44264 | BCL2 | NM_000633.1 | 74 | gtgatgaagtacatccattataa | 307068 | - | see also 19569 line | | |
| 44265 | BTG3 | NM_006806.3 | 542 | aagttaccagggcccttgataag | 307083 | - | see also 19570 line | | |
| 44266 | BTG3 | NM_006806.3 | 192 | aaggctagttcgaaaacatgata | 307072 | - | see also 19570 line | | |
| 44267 | CCNB1 | NM_031966.2 | 1392 | cacatcgaagcatgctaagatca | 307134 | - | see also 19571 line | | |
| 44268 | CCNB1 | NM_031966.2 | 1091 | tccttcggagagcatctaagatt | 307125 | - | see also 19571 line | | |
| 44269 | CCNB2 | NM_004701.2 | 230 | gtgactattaggcgaactgtttt | 307140 | - | see also 3221 line | | |
| 44270 | CCNB2 | NM_004701.2 | 165 | gcccgacggtgtccagtgatttg | 307137 | - | see also 3221 line | | |
| 44271 | CCNB3 | NM_033031.1 | 3784 | acctcgtgtggatgactttgtgt | 307235 | - | see also 19573 line | | |
| 44272 | CCNB3 | NM_033031.1 | 4201 | ttctcacccggtcttctttgaag | 307242 | - | see also 19573 line | | |
| 44273 | CCND1 | NM_053056.1 | 675 | accccgcacgatttcattgaaca | 307251 | - | see also 19574 line | | |
| 44274 | CCND1 | NM_053056.1 | 674 | gaccccgcacgatttcattgaac | 307250 | - | see also 19574 line | | |
| 44275 | CCND2 | NM_001759.2 | 1112 | tacagacgtgcgggatatcgacc | 307261 | - | see also 19575 line | | |
| 44276 | CCND2 | NM_001759.2 | 927 | gtgagctcgctcacttgtgatgc | 307259 | - | see also 19575 line | | |
| 44277 | CCND3 | NM_001760.2 | 514 | acgcccctgaccatcgaaaaact | 307262 | - | see also 19576 line | | |
| 44278 | CCND3 | NM_001760.2 | 683 | accgacaggccttggtcaaaaag | 307265 | - | see also 19576 line | | |
| 44279 | CCNE1 | NM_001238.1 | 453 | aacgtgcaagcctcggattattg | 307273 | - | see also 810 line | | |
| 44280 | CCNE1 | NM_001238.1 | 457 | tgcaagcctcggattattgcacc | 307275 | - | see also 810 line | | |
| 44281 | CCNE1 | NM_001238.1 | 1082 | ctgcttcggccttgtatcatttc | 307296 | - | see also 810 line | | |
| 44282 | CCNF | NM_001761.1 | 1183 | cacggacaacacttacaagtacg | 307319 | - | see also 19578 line | | |
| 44283 | CCNF | NM_001761.1 | 1129 | ctgcacccggtttatcagtaaag | 307312 | - | see also 19578 line | | |
| 44284 | HARC | NM_017913.1 | 647 | ctgaccatccataccttgtatgt | 307338 | - | see also 19579 line | | |
| 44285 | HARC | NM_017913.1 | 288 | ctctgtggcgtgcaaatggaatc | 307332 | - | see also 19579 line | | |
| 44286 | MPHOSPH6 | NM_005792.1 | 213 | gagcagagtttcttactatgtga | 307351 | - | see also 19580 line | | |
| 44287 | MPHOSPH6 | NM_005792.1 | 73 | tgcgcatgaagtttatgcaaagg | 307350 | - | see also 19580 line | | |
| 44288 | MPHOSPH9 | NM_022782.2 | 2807 | caccgataaccatgttaatcata | 307432 | - | see also 19581 line | | |
| 44289 | MPHOSPH9 | NM_022782.2 | 2803 | aagccaccgataaccatgttaat | 307429 | - | see also 19581 line | | |
| 44290 | MTCP1 | NM_014221.1 | 1604 | cacggaagtctgcatcaaagtaa | 307442 | - | cell_cycle | ubiquinol-cytochrome c reductase | leukemia、ataxia telangiectasia |
| 44291 | MTCP1 | NM_014221.1 | 1602 | aacacggaagtctgcatcaaagt | 307441 | - | see also 44290 line | | |
| 44292 | MTCP1 | NM_014221.1 | 1561 | gtcgtctgttcaggatttgaaaa | 307440 | - | see also 44290 line | | |
| 44293 | NEDD9 | NM_006403.2 | 528 | cacccaagaacaagaggtatatc | 307447 | - | see also 4486 line | | |

Figure 46

| | | | | | | | |
|---|---|---|---|---|---|---|---|
| 44294 | NEDD9 | NM_006403.2 | 529 | acccaagaacaagaggtatatca | 307448 | - | see also 4486 line |
| 44295 | NOL1 | NM_006170.1 | 930 | caccctccggaccaataccttga | 307501 | - | see also 19583 line |
| 44296 | NOL1 | NM_006170.1 | 2099 | aacgatcacctaaattcagtcc | 307519 | - | see also 19583 line |
| 44297 | NUDC | NM_006600.2 | 264 | atggcagagagcttatcacaca | 307526 | - | see also 4651 line |
| 44298 | SUGT1 | NM_006704.2 | 744 | atgtgcctacgccaaaacaattc | 307546 | - | see also 4730 line |
| 44299 | SUGT1 | NM_006704.2 | 476 | aagtatgactggtatcaaacaga | 307535 | - | see also 4730 line |
| 44300 | HEC | NM_006101.1 | 976 | atcgttagtagtcgttgagaaaa | 307588 | - | see also 4245 line |
| 44301 | HEC | NM_006101.1 | 1785 | cagcgggaataccaactagttgt | 307616 | - | see also 4245 line |
| 44302 | PRC1 | NM_003981.2 | 1170 | ttgtgcggttaaaaaactactat | 307643 | - | see also 19588 line |
| 44303 | PRC1 | NM_003981.2 | 567 | tgccccactatgatattgacagt | 307633 | - | see also 19588 line |
| 44304 | HCAP-G | NM_022346.2 | 763 | gacgggcagtgttatcatgtatt | 307672 | - | see also 19589 line |
| 44305 | HCAP-G | NM_022346.2 | 2126 | gacgttcggattgaaccattta | 307718 | - | see also 19589 line |
| 44306 | CDC27 | NM_001256.2 | 207 | ctcgcagaacgcctttatgcaga | 307758 | - | see also 812 line |
| 44307 | INCENP | NM_020238.1 | 2562 | ggctatcattcaccagtactacc | 307868 | - | see also 19591 line |
| 44308 | INCENP | NM_020238.1 | 125 | agcgcatgttcaccagagaattc | 307854 | - | see also 19591 line |
| 44309 | STAG1 | NM_005862.1 | 3267 | cacgtcgctttgcccttacattt | 307949 | - | see also 4074 line |
| 44310 | STAG1 | NM_005862.1 | 3952 | tgctgttcgggtctaattggagg | 307969 | - | see also 4074 line |
| 44311 | PSEN1 | NM_000021.2 | 1037 | tccgaaaggtccacttcgtatgc | 308003 | - | see also 19592 line |
| 44312 | PSEN1 | NM_000021.2 | 1479 | ttcgtagccatattaattggtt | 308012 | - | see also 19592 line |
| 44313 | PSEN2 | NM_000447.1 | 616 | cggagcgaagcacgcgatcatgc | 308022 | - | see also 19593 line |
| 44314 | PSEN2 | NM_000447.1 | 540 | aggaccctgaccgctatgtcgt | 308020 | - | see also 19593 line |
| 44315 | SC65 | NM_006455.2 | 1059 | ccggccatagcagatctcttgc | 308081 | - | see also 4525 line |
| 44316 | TSGA2 | NM_080860.1 | 360 | tgctcgatatatcggagaaatatg | 308085 | - | see also 19595 line |
| 44317 | TSGA2 | NM_080860.1 | 307 | agctacgaattcgtaaaagaca | 308083 | - | see also 19595 line |
| 44318 | C10orf9 | NM_145012.3 | 401 | atgtgtcgctcttgcaatatatt | 308119 | - | see also 9764 line |
| 44319 | C10orf9 | NM_145012.3 | 789 | gagctagagcgacagtttcttga | 308124 | - | see also 9764 line |
| 44320 | CCNK | NM_003858.2 | 287 | ggcgctcggttcatctttgatgt | 308132 | - | see also 2622 line |
| 44321 | CCNK | NM_003858.2 | 854 | gtcccggtcgacgtttttggaaga | 308148 | - | see also 2622 line |
| 44322 | CCNL2 | NM_030937.2 | 2062 | gggcggataatccgggaaaatac | 308157 | - | see also 19598 line |
| 44323 | CCNL2 | NM_030937.2 | 1337 | tgctcggaaaaggttgatctca | 308152 | - | see also 19598 line |
| 44324 | CEP2 | NM_007186.2 | 2130 | tgggcgctgcgaagggtaaatgt | 308170 | - | see also 19599 line |
| 44325 | CEP2 | NM_007186.2 | 1192 | acggtcgcctctcagtctatgg | 308162 | - | see also 19599 line |
| 44326 | FLJ13265 | NM_024877.1 | 51 | cccggctcgggcctatcgttagg | 308202 | - | see also 19600 line |
| 44327 | FLJ13265 | NM_024877.1 | 50 | tcccggctcgggcctatcgttag | 308201 | - | see also 19600 line |
| 44328 | FLJ13265 | NM_024877.1 | 396 | gcctgtttggcgtgcaaaatg | 308205 | - | see also 19600 line |
| 44329 | FLJ40137 | NM_173478.1 | 206 | gtcgccacagagacgattgaaga | 308207 | - | see also 19601 line |
| 44330 | FLJ40137 | NM_173478.1 | 596 | ctccaggctcaggctatctaca | 308218 | - | see also 19601 line |

Figure 46

| | | | | | | | |
|---|---|---|---|---|---|---|---|
| 44331 | FLJ40432 | NM_152523.1 | 443 | cagggcagcttactaaaaagtat | 308228 | - | see also 19602 line |
| 44332 | FLJ40432 | NM_152523.1 | 444 | agggcagcttactaaaaagtata | 308229 | - | see also 19602 line |
| 44333 | LOC91768 | NM_138375.1 | 515 | cccggagaaataccatagactcc | 308251 | - | see also 9604 line |
| 44334 | LOC91768 | NM_138375.1 | 360 | tacccaagtcggggacttgaagt | 308246 | - | see also 9604 line |
| 44335 | LOC91768 | NM_138375.1 | 385 | gacggaggaagacaatcaactgg | 308247 | - | see also 9604 line |
| 44336 | PARD3 | NM_019619.2 | 3515 | atcgtcggcggacctatagtttt | 308350 | - | see also 7724 line |
| 44337 | PARD3 | NM_019619.2 | 3516 | tcgtcggcggacctatagttttg | 308351 | - | see also 7724 line |
| 44338 | PARD6A | NM_016948.1 | 640 | agcgggttccaggaatcttcatc | 308356 | - | see also 6978 line |
| 44339 | SPIN | NM_006717.1 | 666 | cacgtgcacctgtcatgaacaca | 308361 | - | see also 19606 line |
| 44340 | SPIN | NM_006717.1 | 829 | cagcctggtaggcaaacaagtgg | 308364 | - | see also 19606 line |
| 44341 | APEG1 | NM_005876.3 | 2470 | cacctccgacgaggaatacctga | 308366 | - | see also 4087 line |
| 44342 | BTG1 | NM_001731.1 | 618 | ctctgggttgacccctatgaagt | 308378 | - | see also 19608 line |
| 44343 | BTG1 | NM_001731.1 | 445 | agctgctggcagaacattataaa | 308375 | - | see also 19608 line |
| 44344 | CD164 | NM_006016.3 | 539 | cacaacctgtgcgaaagtctacc | 308392 | - | see also 4200 line |
| 44345 | CD164 | NM_006016.3 | 428 | cagtgccaacagccaattctaca | 308390 | - | see also 4200 line |
| 44346 | CGRRF1 | NM_006568.1 | 539 | atccattggtagcgctattgacc | 308416 | - | see also 19610 line |
| 44347 | CGRRF1 | NM_006568.1 | 575 | atgaccgggaaatttatgatatt | 308417 | - | see also 19610 line |
| 44348 | 1.Dec | NM_017418.1 | 595 | gtgttacacatgatggttttac | 308427 | - | see also 19611 line |
| 44349 | 1.Dec | NM_017418.1 | 593 | ccgtgttacacatgatggtttt | 308426 | - | see also 19611 line |
| 44350 | DLEC1 | NM_005106.2 | 1602 | aaccgtcggctttgttgaacaac | 308464 | - | see also 19612 line |
| 44351 | DLEC1 | NM_005106.2 | 1662 | cccgggacatgctatattagtgg | 308466 | - | see also 19612 line |
| 44352 | DLEC1 | NM_005106.2 | 3842 | cccgaacccttcgcctgaataac | 308496 | - | see also 19612 line |
| 44353 | DRIM | NM_014503.1 | 3419 | gtcgtcagcacggtatcttaaac | 308612 | - | see also 19613 line |
| 44354 | DRIM | NM_014503.1 | 7009 | ctcgctcaactgaattacgaaca | 308710 | - | see also 19613 line |
| 44355 | DRIM | NM_014503.1 | 5596 | gtcgttcgagttccattagcttt | 308674 | - | see also 19613 line |
| 44356 | EMP3 | NM_001425.1 | 560 | accagcgtggcggtgtttactgg | 308748 | - | - | - | breast cancer |
| 44357 | EMP3 | NM_001425.1 | 391 | aacatgggcctgcagtaatgtca | 308747 | - | see also 44356 line |
| 44358 | EMP3 | NM_001425.1 | 374 | tggaacaacgacaccaaaacatg | 308746 | - | see also 44356 line |
| 44359 | GPNMB | NM_002510.1 | 356 | ctcgtgggctcaaatataacatt | 308757 | - | see also 1617 line |
| 44360 | GPNMB | NM_002510.1 | 151 | tgccgccaaacgatttcatgatg | 308749 | - | see also 1617 line |
| 44361 | LDOC1 | NM_012317.2 | 418 | tgccctacatcgagatggatagc | 308797 | - | see also 19615 line |
| 44362 | LDOC1 | NM_012317.2 | 258 | cccttccccgaaacgtttaatgg | 308794 | - | see also 19615 line |
| 44363 | MDM4 | NM_002393.1 | 1444 | gaccacgagacgggaacattatt | 308843 | - | see also 1538 line |
| 44364 | MDM4 | NM_002393.1 | 235 | caggtgcgcaaggtgaaatgttc | 308802 | - | see also 1538 line |
| 44365 | NCK2 | NM_003581.1 | 983 | gacttctccgtgtcccttaaagc | 308853 | - | see also 19617 line |
| 44366 | NCK2 | NM_003581.1 | 725 | gagtggtggaaatgcaaaaatgc | 308851 | - | see also 19617 line |
| 44367 | PMP22 | NM_000304.2 | 274 | cacgatcgtcagccaatggatcg | 308854 | - | see also 19618 line |

Figure 46

| | | | | | | | |
|---|---|---|---|---|---|---|---|
| 44368 | PMP22 | NM_000304.2 | 579 | ggcatctcaactcggattactcc | 308858 | - | see also 19618 line |
| 44369 | PMP22 | NM_000304.2 | 299 | ggcaatggacacgcaactgatct | 308855 | - | see also 19618 line |
| 44370 | RARRES1 | NM_002888.1 | 659 | cacaggtgtcacactactacttg | 308877 | - | see also 19619 line |
| 44371 | RARRES1 | NM_002888.1 | 445 | cccagaccaaccatcaatgtaac | 308871 | - | see also 19619 line |
| 44372 | RARRES1 | NM_002888.1 | 444 | acccagaccaaccatcaatgtaa | 308870 | - | see also 19619 line |
| 44373 | TP53I11 | NM_006034.2 | 346 | aggtgcatcgtccaagatcagc | 308878 | - | see also 19620 line |
| 44374 | TP53I11 | NM_006034.2 | 755 | cagcatttactactattaccaag | 308880 | - | see also 19620 line |
| 44375 | AMSH | NM_006463.2 | 297 | tccgctctggagttgagattatc | 308882 | - | see also 19621 line |
| 44376 | AMSH | NM_006463.2 | 296 | ttccgctctggagttgagattat | 308881 | - | see also 19621 line |
| 44377 | EDN1 | NM_001955.2 | 368 | ctcgtccctgatggataaagagt | 308899 | - | see also 19622 line |
| 44378 | EDN1 | NM_001955.2 | 366 | tgctcgtccctgatggataaaga | 308898 | - | see also 19622 line |
| 44379 | FGA | NM_000508.2 | 1399 | acgcgtcgttcatgctctaaaac | 308927 | - | see also 19623 line |
| 44380 | FGA | NM_000508.2 | 339 | ttcgttgaccactaatataatgg | 308908 | - | see also 19623 line |
| 44381 | FGB | NM_005141.1 | 791 | ttctgtcaaacgtatagagtat | 308957 | - | see also 19624 line |
| 44382 | FGB | NM_005141.1 | 1239 | gacaatggacgctggttaacatc | 308968 | - | see also 19624 line |
| 44383 | FGB | NM_005141.1 | 1330 | atccaaaggcagatactactgg | 308973 | - | see also 19624 line |
| 44384 | FGFR1OP | NM_007045.2 | 286 | taccaaagacggtcgtttagtgg | 308976 | - | see also 19625 line |
| 44385 | FGFR1OP | NM_007045.2 | 934 | ctcggatgacccccccttaaaaa | 308989 | - | see also 19625 line |
| 44386 | FGG | NM_000509.3 | 934 | gacaagtaccgcctaacatatgc | 309021 | - | see also 19626 line |
| 44387 | FGG | NM_000509.3 | 839 | ctgccatccatatgcattaaga | 309019 | - | see also 19626 line |
| 44388 | SAS | NM_005981.2 | 284 | cacatcatcggcggagtcattgc | 309032 | - | see also 19627 line |
| 44389 | SAS | NM_005981.2 | 563 | accctgtatcaacaagattatga | 309038 | - | see also 19627 line |
| 44390 | SAS | NM_005981.2 | 764 | gccaacccagtgcctttctatg | 309041 | - | see also 19627 line |
| 44391 | TBC1D8 | NM_007063.2 | 1644 | cagaacgaaacgggaattgctgc | 309050 | - | see also 4943 line |
| 44392 | TBC1D8 | NM_007063.2 | 1805 | gcggatgctgcccgattacttca | 309051 | - | see also 4943 line |
| 44393 | BST2 | NM_004335.2 | 55 | ggggataagcgctgtaagcttct | 309074 | - | see also 19629 line |
| 44394 | BST2 | NM_004335.2 | 120 | ggggtgccttgattatcttca | 309076 | - | see also 19629 line |
| 44395 | C6orf108 | NM_006443.2 | 470 | ggccctgctggatcgatacttcg | 309079 | - | - |
| 44396 | DAB2 | NM_001343.1 | 1122 | ctcgtctactccgctgagtaatg | 309094 | - | see also 887 line |
| 44397 | DAB2 | NM_001343.1 | 845 | tcctgttagttgatctaaactct | 309090 | - | see also 887 line |
| 44398 | EMP1 | NM_001423.1 | 249 | gtccacatcgctactgttattat | 309119 | - | see also 19631 line |
| 44399 | EMP1 | NM_001423.1 | 250 | tccacatcgctactgttattatg | 309120 | - | see also 19631 line |
| 44400 | EMP2 | NM_001424.2 | 452 | tgcggcctccatttatacagaca | 309134 | - | see also 19632 line |
| 44401 | EMP2 | NM_001424.2 | 490 | agcacaaaaacgcgaaattctat | 309136 | - | see also 19632 line |
| 44402 | EMP2 | NM_001424.2 | 254 | cacgaattgcacagtcatcaatg | 309132 | - | see also 19632 line |
| 44403 | HDGFRP3 | NM_016073.2 | 399 | aaacgaaggattgtgggaaataga | 309141 | - | - |
| 44404 | HDGFRP3 | NM_016073.2 | 275 | agcaaacaagtatcctatctct | 309138 | - | see also 44403 line |

Figure 46

| | | | | | | | |
|---|---|---|---|---|---|---|---|
| 44405 | HDGFRP3 | NM_016073.2 | 329 | tcccaagacctttccatata | 309140 | - | see also 44403 line |
| 44406 | LAMP3 | NM_014398.2 | 1391 | tccgcctaaggtgtcaatcatct | 309165 | - | see also 19633 line |
| 44407 | LAMP3 | NM_014398.2 | 1390 | atccgcctaaggtgtcaatcatc | 309164 | - | see also 19633 line |
| 44408 | LAMP3 | NM_014398.2 | 1308 | ctcgtctgactacacaattgtgc | 309163 | - | see also 19633 line |
| 44409 | LGI1 | NM_005097.1 | 1060 | caggcacatccactgtagtatgc | 309198 | - | see also 3574 line |
| 44410 | LGI1 | NM_005097.1 | 628 | tccgggactaaagtcattaatt | 309186 | - | see also 3574 line |
| 44411 | PDCD1LG1 | NM_014143.2 | 413 | gccgactacaagcgaattactgt | 309250 | - | see also 19635 line |
| 44412 | PDCD1LG1 | NM_014143.2 | 115 | caacggttcccaaggacctatatg | 309237 | - | see also 19635 line |
| 44413 | PRG4 | NM_005807.1 | 3676 | cagtactggcgtttaccaatga | 309309 | - | see also 4055 line |
| 44414 | PRG4 | NM_005807.1 | 321 | ctgtccgattatgagagttct | 309275 | - | see also 4055 line |
| 44415 | RBBP7 | NM_002893.2 | 1184 | atgggatctgcgtaacttaaaat | 309372 | - | see also 19637 line |
| 44416 | RBBP7 | NM_002893.2 | 1185 | tgggatctgcgtaacttaaaatt | 309373 | - | see also 19637 line |
| 44417 | RBBP7 | NM_002893.2 | 1163 | tgcggataagcaccgtagctttat | 309369 | - | see also 19637 line |
| 44418 | SIL | NM_003035.1 | 3622 | ctgtcactcgatcgaaccaaaat | 309477 | - | see also 2034 line |
| 44419 | SIL | NM_003035.1 | 2022 | aacgaagagtatcctataagacc | 309436 | - | see also 2034 line |
| 44420 | SPOCK | NM_004598.2 | 302 | ccgtttcgagacgatgatatt | 309503 | - | see also 19639 line |
| 44421 | SPOCK | NM_004598.2 | 303 | cgctttcgagacgatgatattt | 309504 | - | see also 19639 line |
| 44422 | TM4SF8 | NM_005724.4 | 953 | ctcatcactgcgcgaacctatgc | 309526 | - | see also 3971 line |
| 44423 | TM4SF8 | NM_005724.4 | 802 | gcgggtgtgaggctctagtagtga | 309525 | - | see also 3971 line |
| 44424 | TM4SF9 | NM_005723.2 | 671 | tgccggagttctagcatttgtt | 309532 | - | see also 3970 line |
| 44425 | TM4SF9 | NM_005723.2 | 668 | cactgccggagttctagcatttg | 309531 | - | see also 3970 line |
| 44426 | TPD52L2 | NM_003288.2 | 433 | gtctctagcgccctatgtgaaaac | 309541 | - | see also 19642 line |
| 44427 | TPD52L2 | NM_003288.2 | 491 | cccagtcagacctctacaagaag | 309542 | - | see also 19642 line |
| 44428 | TSPAN-1 | NM_005727.2 | 698 | aaggctcacgaccaaaaagtaga | 309551 | - | see also 19643 line |
| 44429 | TSPAN-1 | NM_005727.2 | 212 | ggcatctgggtgtcaatcgatgg | 309546 | - | see also 19643 line |
| 44430 | TSPAN-2 | NM_005725.2 | 663 | tgctgtcgatacgaaactcacg | 309570 | - | see also 3972 line |
| 44431 | TSPAN-2 | NM_005725.2 | 107 | tggatcggccgtcattgctttg | 309556 | - | see also 3972 line |
| 44432 | TSPAN-2 | NM_005725.2 | 658 | tctctgctgtcgatacgaaac | 309569 | - | see also 3972 line |
| 44433 | FLJ32871 | NM_144674.1 | 841 | agctcggccagrgtatcgatga | 309577 | - | see also 19645 line |
| 44434 | FLJ32871 | NM_144674.1 | 1293 | cagggacatcccgcagttgaagc | 309580 | - | see also 19645 line |
| 44435 | EML1 | NM_004434.1 | 1014 | ctccacgtcattgaataggttt | 309595 | - | see also 19646 line |
| 44436 | EML1 | NM_004434.1 | 910 | ctgatcggatcacgatagcaaca | 309590 | - | see also 19646 line |
| 44437 | SPRY2 | NM_005842.2 | 1247 | ctggttgccgtctgtaaaaactca | 309641 | - | see also 19647 line |
| 44438 | SPRY2 | NM_005842.2 | 665 | agcactcgcagtccattcttct | 309626 | - | see also 19647 line |
| 44439 | SPRY2 | NM_005842.2 | 1244 | ggcctggttgccgctgtaaaaac | 309639 | - | see also 19647 line |

Figure 46

| 44440 | TUBGCP2 | NM_006659.1 | 2576 | cccggctgagcatctatagcacc | 309670 | - | see also 4704 line |
|---|---|---|---|---|---|---|---|
| 44441 | TUBGCP2 | NM_006659.1 | 1464 | cacctgcccggtggctaaagaga | 309660 | - | see also 4704 line |
| 44442 | TUBGCP2 | NM_006659.1 | 2109 | gcggcagcgaatgctcaacttcg | 309666 | - | see also 4704 line |
| 44443 | TUBGCP3 | NM_006322.3 | 1141 | gactcgtcgggcagagcttttgt | 309697 | - | see also 4423 line |
| 44444 | TUBGCP3 | NM_006322.3 | 1949 | tccagctacgactttgtatcagc | 309730 | - | see also 4423 line |
| 44445 | TUBGCP3 | NM_006322.3 | 2194 | gggcgaagcggatggaatacatc | 309739 | - | see also 4423 line |
| 44446 | PLEK2 | NM_016445.1 | 1078 | agccgagtggattgaagctatca | 309774 | - | see also 6860 line |
| 44447 | PLEK2 | NM_016445.1 | 253 | ccgaccgctcctcattaagctga | 309760 | - | see also 6860 line |
| 44448 | CAP1 | NM_006367.2 | 531 | atgaatgatgccgccatgtttta | 309784 | - | see also 19651 line |
| 44449 | CAP1 | NM_006367.2 | 539 | tgccgccatgttttatacaaacc | 309785 | - | see also 19651 line |
| 44450 | CAP1 | NM_006367.2 | 111 | cgcctggaggcagtatctcatac | 309775 | - | see also 19651 line |
| 44451 | CAP2 | NM_006366.1 | 482 | ttcggccgtcagcgaaagcatcc | 309793 | - | see also 19652 line |
| 44452 | CAP2 | NM_006366.1 | 1382 | gagcgccaagtcatctgaaatga | 309820 | - | see also 19652 line |
| 44453 | KISS1 | NM_002256.2 | 203 | tggggagccattagaaaaggtgg | 309824 | - | see also 19653 line |
| 44454 | PEX11A | NM_003847.1 | 333 | ctggtacctcgcttatgcttaac | 309830 | - | see also 19654 line |
| 44455 | PEX11A | NM_003847.1 | 337 | tacctcgcttatgcttaacatta | 309832 | - | see also 19654 line |
| 44456 | PEX11B | NM_003846.1 | 379 | cagcgttcattcaggtactattt | 309854 | - | see also 2614 line |
| 44457 | PEX11B | NM_003846.1 | 640 | ctgctagacgtggtcagaaatgc | 309859 | - | see also 2614 line |
| 44458 | PEX16 | NM_004813.1 | 891 | aggagaccatcgcagagtttttg | 309861 | - | see also 19656 line |
| 44459 | PEX3 | NM_003630.1 | 215 | aagcacgacgacaatatcatttt | 309870 | - | see also 2437 line |
| 44460 | PEX3 | NM_003630.1 | 449 | tgcgggtccagttaaacataatt | 309879 | - | see also 2437 line |
| 44461 | PEX3 | NM_003630.1 | 211 | gcccaagcacgacgacaatatca | 309869 | - | see also 2437 line |
| 44462 | PXF | NM_002857.1 | 525 | tccccatcatgcagagtattatg | 309912 | - | see also 1890 line |
| 44463 | PXMP3 | NM_000318.1 | 1050 | ctcttactggtgcacctaatagt | 309934 | - | see also 19659 line |
| 44464 | PXMP3 | NM_000318.1 | 716 | tggttagaagaacgatgctatga | 309926 | - | see also 19659 line |
| 44465 | GOLGB1 | NM_004487.1 | 130 | ctgagccgattatcaggattagc | 309961 | - | see also 19660 line |
| 44466 | GOLGB1 | NM_004487.1 | 7580 | agcgacatctctctataatcttg | 310111 | - | see also 19660 line |
| 44467 | EBNA1BP2 | NM_006824.1 | 853 | cagtgctaaacgacggtataaaa | 310178 | - | see also 19661 line |
| 44468 | EBNA1BP2 | NM_006824.1 | 529 | tacgaagcgacccactgattatt | 310173 | - | see also 19661 line |
| 44469 | EBNA1BP2 | NM_006824.1 | 855 | gtgctaaacgacggtataaaaac | 310179 | - | see also 19661 line |
| 44470 | RRS1 | NM_015169.2 | 713 | ccctaccggacaccagagtaagg | 310187 | - | see also 19662 line |
| 44471 | RRS1 | NM_015169.2 | 535 | cccgggacgacaccaaagaatgg | 310186 | - | see also 19662 line |
| 44472 | TINF2 | NM_012461.1 | 355 | tccgcgagtactggagtttctgc | 310192 | - | see also 19663 line |
| 44473 | TINF2 | NM_012461.1 | 334 | cggacgctgcgtggaacattttc | 310191 | - | see also 19663 line |

## Figure 46

| 44474 | TINF2 | NM_012461.1 | 417 | ggcaccacgaacgcctttgtatg | 310193 | - | see also 19663 line |
|---|---|---|---|---|---|---|---|
| 44475 | PALM | NM_002579.1 | 832 | ctccgaggtggacgaactcatcc | 310204 | - | see also 19664 line |
| 44476 | PALM | NM_002579.1 | 1257 | cccaggacctcgacatgaagaag | 310205 | - | see also 19664 line |
| 44477 | PALM | NM_002579.1 | 646 | agccatgtactcggttgagatca | 310203 | - | see also 19664 line |
| 44478 | SOCS2 | NM_003877.3 | 866 | aactaatcttcgaatcgaatacc | 310215 | - | see also 2637 line |
| 44479 | SOCS2 | NM_003877.3 | 860 | tggaccaactaatcttcgaatcg | 310214 | - | see also 2637 line |
| 44480 | SOCS3 | NM_003955.2 | 702 | gccacctggactcctatgagaaa | 310221 | - | see also 19666 line |
| 44481 | SOCS3 | NM_003955.2 | 583 | tcccccagaagagcctattaca | 310219 | - | see also 19666 line |
| 44482 | SOCS4 | NM_004232.2 | 895 | aggatcacggagctattgtctgg | 310246 | - | see also 2824 line |
| 44483 | SOCS4 | NM_004232.2 | 350 | tgcgatatcaacggtgaagatga | 310225 | - | see also 2824 line |
| 44484 | SOCS7 | NM_080867.2 | 1649 | ggcatcgatgcccttccaattcc | 310306 | - | see also 9532 line |
| 44485 | SOCS7 | NM_080867.2 | 879 | aacgacacactgctcctataaat | 310279 | - | see also 9532 line |
| 44486 | SOCS7 | NM_080867.2 | 1412 | cgctatagtcgttctcttcatgc | 310295 | - | see also 9532 line |
| 44487 | NDRG4 | NM_020465.1 | 635 | tccggcctaactagcactttacc | 310315 | - | see also 19669 line |
| 44488 | NDRG4 | NM_020465.1 | 479 | ttcgggttcaagtatgtgattgg | 310313 | - | see also 19669 line |
| 44489 | PLEKHC1 | NM_006832.1 | 1610 | agcagatcacgactgatataact | 310356 | - | see also 19670 line |
| 44490 | PLEKHC1 | NM_006832.1 | 890 | tgctgctccgattcaagtattac | 310343 | - | see also 19670 line |
| 44491 | APG5L | NM_004849.1 | 377 | aacttgtttcacgctatatcagg | 310371 | - | see also 3359 line |
| 44492 | APG5L | NM_004849.1 | 1098 | ctgagctacccggataattttct | 310392 | - | see also 3359 line |
| 44493 | BCAS3 | NM_017679.2 | 1672 | accaaacgaaccggcaaagttaa | 310428 | - | see also 7109 line |
| 44494 | BCAS3 | NM_017679.2 | 1366 | cagccttgtgttcgtacacatat | 310422 | - | see also 7109 line |
| 44495 | BCAS4 | NM_017843.2 | 256 | cagtccttaaggccaaactgaca | 310457 | - | see also 19673 line |
| 44496 | BCAS4 | NM_017843.2 | 208 | ctgacctgatcaggagtgatact | 310455 | - | see also 19673 line |
| 44497 | BCL9 | NM_004326.1 | 3805 | cccgttataccatgatgctatca | 310511 | - | see also 19674 line |
| 44498 | BCL9 | NM_004326.1 | 4424 | accggaatacctgagtttgatct | 310518 | - | see also 19674 line |
| 44499 | CDR1 | NM_004065.1 | 528 | cccaaattatccggaagacttgg | 310528 | - | see also 19675 line |
| 44500 | CDR1 | NM_004065.1 | 674 | tggatttttccggaagatgtgg | 310532 | - | see also 19675 line |
| 44501 | CLN5 | NM_006493.1 | 2120 | ttcctacgactgttccaaatttg | 310548 | - | see also 19676 line |
| 44502 | CLN5 | NM_006493.1 | 1625 | ccgtccaaaacctgatccttatt | 310535 | - | see also 19676 line |
| 44503 | CLN5 | NM_006493.1 | 1640 | tccttattgtcaagctaagtata | 310536 | - | see also 19676 line |
| 44504 | DACH | NM_004392.2 | 1258 | tgcaccaacgcaagttctagacc | 310563 | - | see also 2953 line |
| 44505 | DACH | NM_004392.2 | 1286 | ggcctcctaagaggactcaaagt | 310564 | - | see also 2953 line |
| 44506 | DACH2 | NM_053281.2 | 1459 | ctgcgactggagctctatagaga | 310604 | - | see also 9490 line |
| 44507 | DACH2 | NM_053281.2 | 660 | ggctgaggcgatgaaacttcaga | 310590 | - | see also 9490 line |
| 44508 | FRAT1 | NM_005479.2 | 996 | aactggcgacggcgttcttgtgc | 310616 | - | see also 19679 line |
| 44509 | FRAT1 | NM_005479.2 | 833 | aggccgtgcgaaggcttcattcg | 310614 | - | see also 19679 line |
| 44510 | FSTL3 | NM_005860.1 | 188 | tgctgtgcctccggcaacattga | 310617 | - | see also 19680 line |

Figure 46

| 44511 | FSTL3 | NM_005860.1 | 192 | gtgcctccggcaacattgacacc | 310619 | - | see also 19680 line | | |
|---|---|---|---|---|---|---|---|---|---|
| 44512 | GPC3 | NM_004484.2 | 435 | aactaacagcacgattgaacatg | 310623 | - | see also 3040 line | | |
| 44513 | GPC3 | NM_004484.2 | 948 | gccgaatgctcaccagaatgtgg | 310632 | - | see also 3040 line | | |
| 44514 | GPC3 | NM_004484.2 | 1818 | ctccgaaggacaacgagataagc | 310663 | - | see also 3040 line | | |
| 44515 | HHCM | NM_006543.1 | 738 | atccatcgtgttgtcagtaaaac | 310679 | - | see also 19682 line | | |
| 44516 | HHCM | NM_006543.1 | 509 | ttgcctctaatcagataagctgt | 310672 | - | see also 19682 line | | |
| 44517 | IER3 | NM_003897.2 | 408 | cccgtcctcgagccctttaatct | 310690 | - | apoptosis | - | - |
| 44518 | IER3 | NM_003897.2 | 407 | ccccgtcctcgagccctttaatc | 310689 | - | see also 44517 line | | |
| 44519 | IER3 | NM_003897.2 | 412 | tcctcgagccctttaatctgact | 310691 | - | see also 44517 line | | |
| 44520 | LMO2 | NM_005574.2 | 1481 | tacgagtggactaagatcaatgg | 310697 | - | see also 3876 line | | |
| 44521 | LMO2 | NM_005574.2 | 1039 | gtcctcggccatcgaaaggaaga | 310692 | - | see also 3876 line | | |
| 44522 | OS-9 | NM_006812.1 | 985 | cccgaccaaggatgacagtaagg | 310711 | - | see also 19684 line | | |
| 44523 | OS-9 | NM_006812.1 | 1171 | tgcggatttgattcgattcatag | 310721 | - | see also 19684 line | | |
| 44524 | SEMA3C | NM_006379.2 | 2097 | accgctgccacatctatggtaca | 310781 | - | see also 4472 line | | |
| 44525 | SEMA3C | NM_006379.2 | 1340 | tccatgattgctcgaatatgtcc | 310750 | - | see also 4472 line | | |
| 44526 | SEMA3C | NM_006379.2 | 1507 | cccgaggacaacactagtgtatg | 310758 | - | see also 4472 line | | |
| 44527 | SS18 | NM_005637.1 | 486 | tggatccatgggaggttacaacc | 310805 | - | see also 19686 line | | |
| 44528 | SS18 | NM_005637.1 | 612 | gccaaaccaaggtccaatgatgc | 310811 | - | see also 19686 line | | |
| 44529 | TACC3 | NM_006342.1 | 1104 | gagcggacctgtaaaactagaat | 310833 | - | see also 19687 line | | |
| 44530 | TACC3 | NM_006342.1 | 1102 | aggagcggacctgtaaaactaga | 310832 | - | see also 19687 line | | |
| 44531 | TCL1A | NM_021966.1 | 160 | atcgagataaaggataggttaca | 310846 | - | see also 19688 line | | |
| 44532 | TCL1A | NM_021966.1 | 249 | cccaagcctgctgcctatcatgt | 310847 | - | see also 19688 line | | |
| 44533 | TCL1B | NM_004918.2 | 102 | gcctggcatctacgaagatgagg | 310849 | - | - | - | - |
| 44534 | TCL1B | NM_004918.2 | 189 | ctcccagggcagcagatatgaac | 310850 | - | see also 44533 line | | |
| 44535 | TCL1B | NM_004918.2 | 407 | atcagccggagaggaaagactga | 310851 | - | see also 44533 line | | |
| 44536 | CRADD | NM_003805.2 | 260 | aacaatgctcctgctggatatcc | 310856 | - | see also 19689 line | | |
| 44537 | CRADD | NM_003805.2 | 209 | gacggaaaaccatattcaagaaa | 310853 | - | see also 19689 line | | |
| 44538 | CRADD | NM_003805.2 | 245 | cacaggcctccggaaaacaatgc | 310855 | - | see also 19689 line | | |
| 44539 | DIABLO | NM_019887.2 | 47 | ggctgtcgcgcagcgtaacttca | 310860 | - | see also 19690 line | | |
| 44540 | DIABLO | NM_019887.2 | 51 | gtcgcgcagcgtaacttcattct | 310862 | - | see also 19690 line | | |
| 44541 | DIABLO | NM_019887.2 | 550 | ggcgcagatcaggcctctataac | 310881 | - | see also 19690 line | | |
| 44542 | FADD | NM_003824.2 | 641 | tggctcgtcagctcaaagtctca | 310888 | - | see also 19691 line | | |
| 44543 | FADD | NM_003824.2 | 719 | tgcgggagtcactgagaatctgg | 310889 | - | see also 19691 line | | |
| 44544 | DAP | NM_004394.1 | 219 | agctggtggaatgcgaattgtgc | 310891 | - | apoptosis | - | - |
| 44545 | CIDEB | NM_014430.1 | 1649 | tacgggctctactctatgagttg | 310896 | - | see also 19692 line | | |
| 44546 | CIDEB | NM_014430.1 | 1215 | tactcaggtcagtatctaatata | 310894 | - | see also 19692 line | | |
| 44547 | BAK1 | NM_001188.2 | 309 | aggttttccgcagctacgttttt | 310899 | - | see also 19693 line | | |

Figure 46

| 44548 | BCL2L11 | NM_006538.1 | 372 | acgactgttacgttacattgtcc | 310911 | - | see also 19694 line | | |
|---|---|---|---|---|---|---|---|---|---|
| 44549 | BCL2L11 | NM_006538.1 | 352 | cacccacgaatggttatcttacg | 310909 | - | see also 19694 line | | |
| 44550 | BIK | NM_001197.3 | 355 | gacatcagggatgttcttagaag | 310914 | - | see also 19695 line | | |
| 44551 | BIK | NM_001197.3 | 387 | cggtttcaccacacttaaggaga | 310916 | - | see also 19695 line | | |
| 44552 | BNIP3L | NM_004331.1 | 514 | gtccagtagacccgaaaacattc | 310921 | - | see also 19696 line | | |
| 44553 | BNIP3L | NM_004331.1 | 515 | tccagtagacccgaaaacattcc | 310922 | - | see also 19696 line | | |
| 44554 | CIAS1 | NM_004895.3 | 3459 | tcctcggtactcagcactaatca | 310967 | - | see also 19697 line | | |
| 44555 | CIAS1 | NM_004895.3 | 911 | cacgctaatgatcgacttcaatg | 310927 | - | see also 19697 line | | |
| 44556 | CIAS1 | NM_004895.3 | 1340 | ccccgtgagtcccattaagatgg | 310940 | - | see also 19697 line | | |
| 44557 | EI24 | NM_004879.2 | 812 | tgcaccagcggttgtctaacata | 310992 | - | see also 3388 line | | |
| 44558 | PDCD5 | NM_004708.2 | 306 | gacaactaagtgagaaggtatca | 310998 | - | apoptosis | - | - |
| 44559 | TRADD | NM_003789.2 | 464 | gacgaggagcgctgtttgagttg | 311000 | - | see also 19699 line | | |
| 44560 | TRADD | NM_003789.2 | 740 | gcgcgctctgtgggtctcaaatg | 311003 | - | see also 19699 line | | |
| 44561 | BCL2L1 | NM_001191.2 | 642 | ggcaggcgacgagtttgaactgc | 311007 | - | see also 772 line | | |
| 44562 | BCL2L1 | NM_001191.2 | 708 | caccccagggacagcatatcaga | 311009 | - | see also 772 line | | |
| 44563 | API5 | NM_006595.1 | 360 | atctattcgacgtcaagcaatta | 311021 | - | see also 19700 line | | |
| 44564 | API5 | NM_006595.1 | 419 | ctcgagtggcagatatactaacg | 311024 | - | see also 19700 line | | |
| 44565 | BCL2A1 | NM_004049.2 | 548 | ttgcggagttcataatgaataac | 311064 | - | see also 19701 line | | |
| 44566 | BCL2A1 | NM_004049.2 | 545 | ttgttgcggagttcataatgaat | 311063 | - | see also 19701 line | | |
| 44567 | BCL2L2 | NM_004050.2 | 413 | cacccaggtctccgatgaacttt | 311067 | - | see also 19702 line | | |
| 44568 | BCL2L2 | NM_004050.2 | 420 | gtctccgatgaactttttcaagg | 311071 | - | see also 19702 line | | |
| 44569 | BECN1 | NM_003766.2 | 1288 | aggcgagacacgttttttgtcttc | 311093 | - | see also 2577 line | | |
| 44570 | BECN1 | NM_003766.2 | 361 | tggtgtctctcgcagattcatcc | 311075 | - | see also 2577 line | | |
| 44571 | BIRC2 | NM_001166.2 | 1188 | tcccaggtccctcgtatcaaaac | 311101 | - | see also 743 line | | |
| 44572 | BIRC2 | NM_001166.2 | 1187 | ttcccaggtccctcgtatcaaaa | 311100 | - | see also 743 line | | |
| 44573 | BIRC2 | NM_001166.2 | 1192 | aggtccctcgtatcaaaacatta | 311103 | - | see also 743 line | | |
| 44574 | BIRC3 | NM_001165.3 | 4182 | gtctactaactgccggaattatt | 311174 | - | see also 19705 line | | |
| 44575 | BIRC3 | NM_001165.3 | 3767 | gagttcatccgtcaagttcaagc | 311162 | - | see also 19705 line | | |
| 44576 | BIRC8 | NM_033341.2 | 1913 | gtgcagcgcggttattgatttca | 311199 | - | see also 19706 line | | |
| 44577 | BIRC8 | NM_033341.2 | 1334 | tgctataggtcaagaggataaag | 311190 | - | see also 19706 line | | |
| 44578 | BNIP1 | NM_001205.1 | 90 | ttcaggatatccgtcgtgattgttca | 311206 | - | see also 19707 line | | |
| 44579 | BNIP1 | NM_001205.1 | 32 | cacgtccggatctgtaaccaaga | 311202 | - | see also 19707 line | | |
| 44580 | FAIM2 | NM_012306.2 | 498 | tggctgtcgtggctctctttact | 311230 | - | see also 19708 line | | |
| 44581 | FAIM2 | NM_012306.2 | 439 | tcgtcgagtctttgtcagaaagg | 311229 | - | see also 19708 line | | |
| 44582 | GG2-1 | NM_014350.1 | 504 | atgaccgtggtcagttccatca | 311233 | - | see also 19709 line | | |
| 44583 | GG2-1 | NM_014350.1 | 500 | tgctatgaccgtggtcagtttcc | 311232 | - | see also 19709 line | | |
| 44584 | SNCA | NM_000345.2 | 142 | gacaaaagagggtgttctctatg | 311241 | - | apoptosis | - | Parkinson disease、Alzheimer disease |

Figure 46

| | | | | | | | |
|---|---|---|---|---|---|---|---|
| 44585 | SNCA | NM_000345.2 | 156 | ttctctatgtaggctccaaaacc | 311242 | - | see also 44584 line |
| 44586 | TAX1BP1 | NM_006024.4 | 1183 | aactcggcaagaagttgtcttc | 311286 | - | see also 19710 line |
| 44587 | TAX1BP1 | NM_006024.4 | 257 | tggagtactgtcgtgattatta | 311255 | - | see also 19710 line |
| 44588 | TOSO | NM_005449.3 | 1062 | gcgaccgcgctcccaaaacaaca | 311329 | - | see also 19711 line |
| 44589 | TOSO | NM_005449.3 | 1061 | cgcgaccgcgctcccaaaacaac | 311328 | - | see also 19711 line |
| 44590 | TOSO | NM_005449.3 | 365 | gccgagttactctgaagcaatac | 311320 | - | see also 19711 line |
| 44591 | MCL1 | NM_021960.3 | 566 | ttggtcgggaatctggtaataa | 311335 | - | see also 19712 line |
| 44592 | MCL1 | NM_021960.3 | 1155 | gagctggtttggcatatctaata | 311353 | - | see also 19712 line |
| 44593 | AXUD1 | NM_033027.2 | 1382 | gcctggcacgcatcttgagttc | 311362 | - | see also 9349 line |
| 44594 | AXUD1 | NM_033027.2 | 1044 | cccatggcgcgtgtggaattta | 311358 | - | see also 9349 line |
| 44595 | BCL2L12 | NM_052842.2 | 779 | ccagcgttccgccctttctacg | 311372 | - | see also 19714 line |
| 44596 | BCL2L12 | NM_052842.2 | 749 | tgggagcgtcacatgcaaattga | 311371 | - | see also 19714 line |
| 44597 | BCLG | NM_030766.1 | 772 | cacgtgcctgagcttcaagttc | 311393 | - | see also 19715 line |
| 44598 | BCLG | NM_030766.1 | 773 | acgtgcctgagcttcaagttct | 311394 | - | see also 19715 line |
| 44599 | CARD6 | NM_032587.2 | 1126 | ctccttagatcgaggatgtaagt | 311430 | - | see also 19716 line |
| 44600 | CARD6 | NM_032587.2 | 2532 | gccgagtaaaagtttggtatt | 311460 | - | see also 19716 line |
| 44601 | CARD6 | NM_032587.2 | 1368 | tgcaaagccaagtcatgtcaaac | 311436 | - | see also 19716 line |
| 44602 | CARD8 | NM_014959.1 | 969 | ccacccgaagatattaagttc | 311485 | - | see also 19717 line |
| 44603 | CARD8 | NM_014959.1 | 1019 | ttgctaacaaaggcgatagatga | 311486 | - | see also 19717 line |
| 44604 | CARD9 | NM_022352.2 | 443 | ccgcgtcttctccatgatcatc | 311494 | - | see also 19718 line |
| 44605 | CTNNAL1 | NM_003798.1 | 1022 | gagcgtatggaggacttttactga | 311534 | - | see also 2597 line |
| 44606 | CTNNAL1 | NM_003798.1 | 517 | ggcagaccgagtagtcattaaac | 311512 | - | see also 2597 line |
| 44607 | MAEA | NM_005882.2 | 1004 | gtcgtgtgcccgagaaccaaaga | 311584 | - | see also 19721 line |
| 44608 | MAEA | NM_005882.2 | 956 | taagtctacggctacaattctct | 311581 | - | see also 19721 line |
| 44609 | MAEA | NM_005882.2 | 1183 | atccgtgagcaagataaatact | 311587 | - | see also 19721 line |
| 44610 | MGC13096 | NM_032346.1 | 955 | agcatgctcaagagtgctaattt | 311603 | - | see also 19722 line |
| 44611 | MGC13096 | NM_032346.1 | 440 | aggggccttcaccacagtttacc | 311592 | - | see also 19722 line |
| 44612 | NCKAP1 | NM_013436.2 | 2793 | aactactaggcccatatggtatg | 311687 | - | see also 5432 line |
| 44613 | NCKAP1 | NM_013436.2 | 2000 | atgagttgcacgcatgaactatg | 311658 | - | see also 5432 line |
| 44614 | PDCD6IP | NM_013374.2 | 2610 | atgcgtatggccagtataatag | 311747 | - | see also 19724 line |
| 44615 | PDCD6IP | NM_013374.2 | 998 | gtggcatctgctatgatgaata | 311731 | - | see also 19724 line |
| 44616 | PPP1R13B | NM_015316.1 | 3104 | ctcaaccataaggcgacattgaaa | 311805 | - | see also 19725 line |
| 44617 | PPP1R13B | NM_015316.1 | 162 | atgccgatgatattaactgttt | 311752 | - | see also 19725 line |
| 44618 | TAIP-2 | NM_024969.2 | 1672 | cccggagcaattcgttgactatg | 311833 | - | see also 8829 line |
| 44619 | TAIP-2 | NM_024969.2 | 1164 | ctgtcgctcactccgtagaatat | 311822 | - | see also 8829 line |

Figure 46

| | | | | | | | |
|---|---|---|---|---|---|---|---|
| 44620 | TAIP-2 | NM_024969.2 | 1166 | gtcgctcactccgtagaatattc | 311823 | - | see also 8829 line |
| 44621 | TEGT | NM_003217.1 | 117 | acctgaagaaggtctatgcaagt | 311846 | - | see also 19727 line |
| 44622 | TEGT | NM_003217.1 | 652 | ctggcactgcattgatctctct | 311851 | - | see also 19727 line |
| 44623 | TRIAD3 | NM_019011.3 | 1378 | acctctgcgagtttctattaat | 311868 | - | see also 19728 line |
| 44624 | TRIAD3 | NM_019011.3 | 1779 | cccaaacgtgcgggtcaactatga | 311874 | - | see also 19728 line |
| 44625 | C6 | NM_000065.1 | 1218 | agccgaatattcgatgacttgg | 311907 | - | see also 19729 line |
| 44626 | C6 | NM_000065.1 | 1207 | ctgctttgtacagccgaatattc | 311904 | - | see also 19729 line |
| 44627 | C7 | NM_000587.1 | 695 | cacgcagttatacttcacatacc | 311964 | - | see also 19730 line |
| 44628 | C7 | NM_000587.1 | 870 | tgcctaccgaagattaatcgacc | 311967 | - | see also 19730 line |
| 44629 | C7 | NM_000587.1 | 656 | gtcggaagcgtccttttttaga | 311960 | - | see also 19730 line |
| 44630 | C8A | NM_000562.1 | 714 | acctgtgaacgtctctactatgg | 312006 | - | see also 409 line |
| 44631 | C8A | NM_000562.1 | 290 | tccgtgccaggacacaaaagtacc | 311996 | - | see also 409 line |
| 44632 | C8B | NM_000066.1 | 842 | gtgatcgaggcaaacactatatt | 312064 | - | see also 19732 line |
| 44633 | C8B | NM_000066.1 | 954 | aagcctcatgctccattacgagt | 312072 | - | see also 19732 line |
| 44634 | C9 | NM_001737.2 | 1137 | gggtctctaggaggactctatga | 312108 | - | see also 19733 line |
| 44635 | C9 | NM_001737.2 | 1422 | ctccgaggaaccgtgattgatgt | 312122 | - | see also 19733 line |
| 44636 | PAQR10 | NM_198403.1 | 421 | ttcgaccggatggtcatctattt | 312138 | - | see also 10321 line |
| 44637 | PAQR10 | NM_198403.1 | 509 | tgcgctggctggtctggattatg | 312140 | - | see also 10321 line |
| 44638 | LIMS1 | NM_004987.2 | 1069 | aactagctgagaccttaggaagg | 312144 | - | - |
| 44639 | CLUL1 | NM_014410.4 | 1180 | ggcgatcaggttggtcaatgtat | 312165 | - | see also 19735 line |
| 44640 | CLUL1 | NM_014410.4 | 1490 | ggctacgtagtggcaaaagctct | 312172 | - | see also 19735 line |
| 44641 | OPTN | NM_021980.3 | 1835 | gacatagacacagttacagattca | 312197 | - | see also 8239 line |
| 44642 | OPTN | NM_021980.3 | 1785 | agcggaatattcgattcattcc | 312194 | - | see also 8239 line |
| 44643 | SMPX | NM_014332.1 | 389 | atctatcggaaatcagaatatt | 312206 | - | see also 19737 line |
| 44644 | SMPX | NM_014332.1 | 223 | atccaggcaaatatcaatattcc | 312202 | - | see also 19737 line |
| 44645 | TNNT2 | NM_000364.1 | 624 | gcccagacgagcgggaaaaagtgg | 312208 | - | see also 19738 line |
| 44646 | TNNT2 | NM_000364.1 | 402 | gaggagctgtttctctcaaaga | 312207 | - | see also 19738 line |
| 44647 | DYSF | NM_003494.2 | 5516 | accggacagaccgtgtaatgttt | 312265 | - | see also 2358 line |
| 44648 | DYSF | NM_003494.2 | 4051 | ctgtacgaccatgacaacttatgg | 312246 | - | see also 2358 line |
| 44649 | EMD | NM_000117.1 | 142 | atcaactctgtaggctttacgaga | 312283 | - | see also 19740 line |
| 44650 | EMD | NM_000117.1 | 119 | atcccgccacggcggcctgtagtagg | 312281 | - | see also 19740 line |
| 44651 | FER1L3 | NM_013451.2 | 4357 | accatgccgggacatcgttatcg | 312394 | - | see also 19741 line |
| 44652 | FER1L3 | NM_013451.2 | 4356 | caccatgccggacatcgttatc | 312393 | - | see also 19741 line |
| 44653 | SGCA | NM_000023.1 | 583 | gccgaaaagaagggtatacatt | 312449 | - | see also 19742 line |
| 44654 | SGCA | NM_000023.1 | 572 | tcccattgagggcgaaaagaag | 312447 | - | see also 19742 line |
| 44655 | SLMAP | NM_007159.1 | 477 | tgcagaggttacacatcgatact | 312463 | - | see also 4962 line |
| 44656 | SLMAP | NM_007159.1 | 551 | ttgcttagtgcccgagatgaaat | 312466 | - | see also 4962 line |

Figure 46

| | | | | | | | |
|---|---|---|---|---|---|---|---|
| 44657 | SSPN | NM_005086.3 | 418 | gacgaacggacatgtattcaatt | 312492 | - | see also 19744 line |
| 44658 | SSPN | NM_005086.3 | 374 | tggcctatcttggcttgtttatg | 312489 | - | see also 19744 line |
| 44659 | TRDN | NM_006073.1 | 1314 | aggagattggtgcgtttcaagt | 312509 | - | see also 4217 line |
| 44660 | FEZ1 | NM_005103.3 | 327 | atggaggacctcgtaaatgaatt | 312521 | - | see also 3578 line |
| 44661 | FEZ1 | NM_005103.3 | 279 | ctctccgagcttgagaattttc | 312519 | - | see also 3578 line |
| 44662 | SEMA3B | NM_004636.1 | 1552 | agccgctgacggacactatgacg | 312543 | - | see also 19746 line |
| 44663 | SEMA3B | NM_004636.1 | 942 | acccagacgacgacaaaatctac | 312542 | - | see also 19746 line |
| 44664 | F11R | NM_016946.3 | 466 | atccaagcctacagttaacatcc | 312549 | - | see also 19747 line |
| 44665 | F11R | NM_016946.3 | 588 | tgcctacgaatcccaaaagcacc | 312552 | - | see also 19747 line |
| 44666 | NEO1 | NM_002499.1 | 3365 | gaactaggatcgactacacaaacc | 312661 | - | see also 1595 line |
| 44667 | NEO1 | NM_002499.1 | 4493 | aaggaactaaacgctatcacaac | 312681 | - | see also 1595 line |
| 44668 | PECAM1 | NM_000442.2 | 1392 | tccagatagtcgtatgtgaaatg | 312709 | - | see also 19749 line |
| 44669 | PECAM1 | NM_000442.2 | 780 | aggaacaggacgcgcgtttatcc | 312695 | - | see also 19749 line |
| 44670 | PECAM1 | NM_000442.2 | 1327 | gtcggacagtgggacgtatatct | 312706 | - | see also 19749 line |
| 44671 | SEMA3E | NM_012431.1 | 2448 | ttgtccatacggtccgtaaaatc | 312799 | - | see also 5320 line |
| 44672 | SEMA3E | NM_012431.1 | 2451 | tccatacggtccgtaaaatcacc | 312800 | - | see also 5320 line |
| 44673 | SEMA3E | NM_012431.1 | 558 | cccggttacgcctgtcacataaa | 312733 | - | see also 5320 line |
| 44674 | TM4SF6 | NM_003270.2 | 800 | ctctctgtgccataacaaataa | 312834 | - | see also 2218 line |
| 44675 | TM4SF6 | NM_003270.2 | 298 | tgctactggtaccgtcattattc | 312813 | - | see also 2218 line |
| 44676 | TPBG | NM_006670.3 | 1188 | ccgcaacctgacacatcctagaaa | 312845 | - | see also 4714 line |
| 44677 | TPBG | NM_006670.3 | 1381 | gaccggctcacctgtgcatatcc | 312847 | - | see also 4714 line |
| 44678 | GAGEB1 | NM_003785.2 | 138 | atcgggtagaccaatgatgatcat | 312857 | - | see also 19753 line |
| 44679 | GAGEB1 | NM_003785.2 | 139 | tcggcgtagaccaatgatctatg | 312858 | - | see also 19753 line |
| 44680 | LY6H | NM_002347.1 | 409 | atctgatggggtttattaaactct | 312863 | - | see also 19754 line |
| 44681 | PTX3 | NM_002852.2 | 164 | ggccgagaactcggatgattatg | 312866 | - | see also 19755 line |
| 44682 | PTX3 | NM_002852.2 | 165 | gccgagaactcggatgattatga | 312867 | - | see also 19755 line |
| 44683 | CD1B | NM_001764.1 | 529 | tctgtgcactaatcatcaata | 312906 | - | see also 19756 line |
| 44684 | CD1B | NM_001764.1 | 139 | cagacctcgtctttaccaatag | 312895 | - | see also 19756 line |
| 44685 | CD1C | NM_001765.1 | 452 | tagctttcaacggattagattta | 312926 | - | see also 19757 line |
| 44686 | CD1C | NM_001765.1 | 961 | tggattgccttggttagttgatagt | 312938 | - | see also 19757 line |
| 44687 | CD226 | NM_006566.1 | 717 | ccccgtcagatcgacctcttaact | 312959 | - | see also 19758 line |
| 44688 | CD226 | NM_006566.1 | 209 | atggattatcctactttactttt | 312943 | - | see also 19758 line |
| 44689 | CD226 | NM_006566.1 | 288 | ttccctttgccgagaacatgtct | 312946 | - | see also 19758 line |
| 44690 | CD2BP2 | NM_006110.1 | 559 | ttgggccagacctcaatggagtgc | 312977 | - | see also 19759 line |
| 44691 | CD2BP2 | NM_006110.1 | 236 | gccgctttaaaggcaaacactct | 312973 | - | see also 19759 line |
| 44692 | CD53 | NM_000560.2 | 483 | accgttaccactcagacaatagc | 312992 | - | see also 19760 line |
| 44693 | CD53 | NM_000560.2 | 482 | caccgttaccactcagacaatag | 312991 | - | see also 19760 line |

Figure 46

| 44694 | C1QA | NM_015991.1 | 471 | cactgtacccggctactactact | 312998 | - | see also 19761 line |
|---|---|---|---|---|---|---|---|
| 44695 | C1QA | NM_015991.1 | 291 | ccgtggcatcccgggaattaaag | 312996 | - | see also 19761 line |
| 44696 | C1QB | NM_000491.2 | 116 | tcctgggcctaatcgatatctcc | 313004 | - | see also 19762 line |
| 44697 | C1QB | NM_000491.2 | 643 | gtggtcaccttctgtgactatgc | 313007 | - | see also 19762 line |
| 44698 | C1QG | NM_172369.1 | 550 | caccaacccgcagggagattatg | 313011 | - | see also 19763 line |
| 44699 | C1QG | NM_172369.1 | 551 | accaacccgcagggagattatga | 313012 | - | see also 19763 line |
| 44700 | C1QG | NM_172369.1 | 555 | acccgcagggagattatgacacg | 313013 | - | see also 19763 line |
| 44701 | C4BPA | NM_000715.2 | 370 | ctgccccgatggatattacgttg | 313020 | - | see also 19764 line |
| 44702 | C4BPA | NM_000715.2 | 371 | tgccccgatggatattacgttga | 313021 | - | see also 19764 line |
| 44703 | C4BPB | NM_000716.2 | 406 | ttctggggacttacgtttgtatc | 313061 | - | see also 19765 line |
| 44704 | C4BPB | NM_000716.2 | 744 | accttaggatccaccattagtta | 313064 | - | see also 19765 line |
| 44705 | DAF | NM_000574.2 | 515 | ttgaagagttctgcaatcgtagc | 313072 | - | see also 19766 line |
| 44706 | DAF | NM_000574.2 | 1021 | atgattggagagcactctattta | 313092 | - | see also 19766 line |
| 44707 | FHR5 | NM_030787.1 | 1520 | accgttgccagtccttctataaa | 313119 | - | see also 19767 line |
| 44708 | FHR5 | NM_030787.1 | 1720 | ttcccacataaagcgatgatatc | 313121 | - | see also 19767 line |
| 44709 | HF1 | NM_000186.1 | 3678 | aacggggatatcgtctttcatca | 313232 | - | see also 19768 line |
| 44710 | HF1 | NM_000186.1 | 1578 | aacccacgtgcattaaatcttgt | 313171 | - | see also 19768 line |
| 44711 | PFC | NM_002621.1 | 257 | agcgcaggcccctcgattgttgc | 313238 | - | see also 19769 line |
| 44712 | PFC | NM_002621.1 | 1420 | ccccctgtggacctaatcctacc | 313244 | - | see also 19769 line |
| 44713 | HFL1 | NM_002113.1 | 981 | cggggatatcgtctttcatcacg | 313233 | - | - \| - \| - |
| 44714 | CD24 | NM_013230.1 | 112 | tcctacccacgcagatttattcc | 313248 | - | see also 19770 line |
| 44715 | CD24 | NM_013230.1 | 111 | ctcctacccacgcagatttattc | 313247 | - | see also 19770 line |
| 44716 | CD83 | NM_004233.2 | 446 | cacggtctcctgggtcaagttat | 313251 | - | see also 19771 line |
| 44717 | CD83 | NM_004233.2 | 805 | ttgcacggctacagagtatcttc | 313260 | - | see also 19771 line |
| 44718 | HGRG8 | NM_016258.1 | 782 | ttggtagcgggtccattactagt | 313282 | - | see also 19772 line |
| 44719 | HGRG8 | NM_016258.1 | 786 | tagcgggtccattactagtaaca | 313283 | - | see also 19772 line |
| 44720 | AIM2 | NM_004833.1 | 912 | agcgcctcacgtgtgttagatgc | 313321 | - | see also 19773 line |
| 44721 | AIM2 | NM_004833.1 | 328 | ttctttcagacgagtttaatatt | 313308 | - | see also 19773 line |
| 44722 | AIM2 | NM_004833.1 | 955 | atgtcccgctgaacattatcaga | 313323 | - | see also 19773 line |
| 44723 | AZGP1 | NM_001185.2 | 602 | ccctgcgactctgcggaaatacc | 313349 | - | see also 19774 line |
| 44724 | AZGP1 | NM_001185.2 | 387 | cacgtattgcagggaaggtttgg | 313345 | - | see also 19774 line |
| 44725 | C1QBP | NM_001212.3 | 420 | tgggacagaagcgaaattagtgc | 313353 | - | see also 794 line |
| 44726 | C1QBP | NM_001212.3 | 574 | cccaatttcgtggttgaagttat | 313358 | - | see also 794 line |
| 44727 | CD1A | NM_001763.1 | 826 | ttcgtagatacgcccatgaattg | 313371 | - | see also 19776 line |
| 44728 | CD1A | NM_001763.1 | 623 | ctggatcgcatccttttacaacc | 313368 | - | see also 19776 line |
| 44729 | CD1A | NM_001763.1 | 1448 | ggcggtgatagtgcctttacttc | 313381 | - | see also 19776 line |
| 44730 | CD59 | NM_000611.3 | 277 | gagggaaaatgagctaacgtact | 313386 | - | see also 19777 line |

Figure 46

| 44731 | CD59 | NM_000611.3 | 207 | ctgggttacaagtgtataacaag | 313384 | - | see also 19777 line |
|---|---|---|---|---|---|---|---|
| 44732 | CD96 | NM_005816.3 | 941 | gagtcggtcctaacaaaatcttg | 313406 | - | see also 19778 line |
| 44733 | CD96 | NM_005816.3 | 1506 | gactacccgaggcttcaactatc | 313416 | - | see also 19778 line |
| 44734 | CEACAM8 | NM_001816.2 | 816 | accatgcaggggtaaatctcaac | 313435 | - | see also 19779 line |
| 44735 | CEACAM8 | NM_001816.2 | 726 | acccagcgagtgcaaacttcagt | 313434 | - | see also 19779 line |
| 44736 | CNIH | NM_005776.1 | 175 | atcctatagaccagtgtaatacc | 313440 | - | see also 19780 line |
| 44737 | CNIH | NM_005776.1 | 293 | gcccctcttggcatatcatattt | 313444 | - | see also 19780 line |
| 44738 | CTLA4 | NM_005214.2 | 307 | gaggcatcgccagctttgtgtgt | 313448 | - | see also 19781 line |
| 44739 | CTLA4 | NM_005214.2 | 467 | gggcacctccagtggaaatcaag | 313450 | - | see also 19781 line |
| 44740 | G1P3 | NM_002038.2 | 118 | aggcggtatcgcttttcttgtgc | 313456 | - | see also 19782 line |
| 44741 | G1P3 | NM_002038.2 | 417 | agcagcgtcgtcataggtaatat | 313458 | - | see also 19782 line |
| 44742 | ICOS | NM_012092.2 | 138 | aacggaggtgtacaaattttatg | 313465 | - | see also 19783 line |
| 44743 | ICOS | NM_012092.2 | 221 | ctgcgatctcactaagacaaaag | 313471 | - | see also 19783 line |
| 44744 | KAAG1 | NM_181337.2 | 944 | tcctgaaacgaacgagaaactga | 313486 | - | see also 19784 line |
| 44745 | NK4 | NM_004221.3 | 517 | gacagtggcggcttattatgagg | 313492 | - | see also 19785 line |
| 44746 | NK4 | NM_004221.3 | 248 | aagctgaaggcccgaatggtaat | 313487 | - | see also 19785 line |
| 44747 | RAET1E | NM_139165.1 | 212 | aagcgcaggtcttcttgaataaa | 313499 | - | see also 19786 line |
| 44748 | RAET1E | NM_139165.1 | 140 | tggttggtggtcactctctttgc | 313496 | - | see also 19786 line |
| 44749 | RAET1L | NM_130900.1 | 10 | gccgccatcccagctttgcttct | 313512 | - | - | - | - |
| 44750 | SEMA7A | NM_003612.1 | 1023 | gtgtattccctcggtgacattga | 313528 | - | see also 2426 line |
| 44751 | SEMA7A | NM_003612.1 | 335 | atctgtgcgcacggtgaatatcg | 313515 | - | see also 2426 line |
| 44752 | CAMLG | NM_001745.2 | 878 | atcgatcaatggatacctatagc | 313551 | - | see also 19788 line |
| 44753 | CAMLG | NM_001745.2 | 445 | ttggactcgttcattaaaccacc | 313539 | - | see also 19788 line |
| 44754 | CD84 | NM_003874.1 | 302 | tgccttaggtccgaactacaatc | 313564 | - | see also 2636 line |
| 44755 | CD84 | NM_003874.1 | 303 | gccttaggtccgaactacaatct | 313565 | - | see also 2636 line |
| 44756 | LY6E | NM_002346.1 | 364 | atcccagaaggcgtcaatgttgg | 313574 | - | see also 19790 line |
| 44757 | NOD27 | NM_032206.2 | 1107 | gccacgggtggaagaatatgtga | 313589 | - | see also 19791 line |
| 44758 | NOD27 | NM_032206.2 | 817 | ccgtccgagctcctttttgatct | 313584 | - | see also 19791 line |
| 44759 | NOD27 | NM_032206.2 | 816 | accgtccgagctcctttttgatc | 313583 | - | see also 19791 line |
| 44760 | TFF2 | NM_005423.2 | 389 | ctgtggaagactgccattactaa | 313622 | - | see also 19792 line |
| 44761 | TFF3 | NM_003226.1 | 171 | ctggagtgccttggtgtttcaag | 313623 | - | see also 19793 line |
| 44762 | PSTPIP1 | NM_003978.2 | 1207 | gccctaccagaactattacgatc | 313631 | - | see also 19794 line |
| 44763 | PSTPIP1 | NM_003978.2 | 1265 | cagccgtcctgcggcatgataaa | 313632 | - | see also 19794 line |
| 44764 | LECT2 | NM_002302.1 | 252 | ggcagggccatgggctaatatat | 313635 | - | see also 19795 line |
| 44765 | LECT2 | NM_002302.1 | 460 | atcaataatggtgttcgaatatc | 313642 | - | see also 19795 line |

Figure 46

| | | | | | | | |
|---|---|---|---|---|---|---|---|
| 44766 | LECT2 | NM_002302.1 | 511 | tacattaagccaattaagtataa | 313644 | - | see also 19795 line |
| 44767 | COCH | NM_004086.1 | 316 | ggggacctgtacgagtctatagc | 313657 | - | see also 2750 line |
| 44768 | DFNA5 | NM_004403.1 | 709 | gcctacggtgtcattgagttata | 313710 | - | see also 19796 line |
| 44769 | DFNA5 | NM_004403.1 | 707 | ttgcctacggtgtcattgagtta | 313708 | - | see also 19796 line |
| 44770 | DSPP | NM_014208.1 | 1004 | taggtcaaaattcggatagtaaa | 313741 | - | see also 19797 line |
| 44771 | DSPP | NM_014208.1 | 1178 | aacgcgtagaaaatagaatcacc | 313747 | - | see also 19797 line |
| 44772 | DSPP | NM_014208.1 | 886 | gacgaggttctggtgatgatga | 313739 | - | see also 19797 line |
| 44773 | ITM2B | NM_021999.2 | 674 | agcctattagatcttaacctgg | 313765 | - | see also 19798 line |
| 44774 | ITM2B | NM_021999.2 | 957 | ttcgcaattcggcatttgaaaa | 313775 | - | see also 19798 line |
| 44775 | ITM2B | NM_021999.2 | 457 | aaccagatgacgttgactactgt | 313763 | - | see also 19798 line |
| 44776 | KCNE1L | NM_012282.2 | 308 | aggggcgacgagcgctatcctac | 313778 | - | see also 19799 line |
| 44777 | NOG | NM_005450.2 | 653 | gccatgccgagcgagcaaagg | 313781 | - | see also 19800 line |
| 44778 | NOG | NM_005450.2 | 652 | ggccatgccgagcgagcaaag | 313780 | - | see also 19800 line |
| 44779 | NOG | NM_005450.2 | 753 | ggctgttgctgcgagacattctgc | 313784 | - | see also 19801 line |
| 44780 | OTOR | NM_020157.2 | 199 | cccggactgtagattcattaac | 313791 | - | see also 19801 line |
| 44781 | OTOR | NM_020157.2 | 401 | caccaeggatattgacttcttct | 313797 | - | see also 19801 line |
| 44782 | OTOR | NM_020157.2 | 398 | tcccaccacggatattgacttct | 313796 | - | see also 19801 line |
| 44783 | TBL1X | NM_005647.2 | 1491 | gccatgacgtccgagtaacaaa | 313801 | - | see also 19802 line |
| 44784 | TBL1X | NM_005647.2 | 654 | agtcgctggcgcctcttcatcg | 313798 | - | see also 19802 line |
| 44785 | TMC1 | NM_138691.2 | 1353 | caggttgccgctctctatttc | 313819 | - | see also 9628 line |
| 44786 | TMC1 | NM_138691.2 | 2235 | tcttcataccgaattcgaca | 313848 | - | see also 9628 line |
| 44787 | USH3A | NM_052995.2 | 454 | tccacgtcaatgtcattctc | 313861 | - | see also 9474 line |
| 44788 | USH3A | NM_052995.2 | 450 | agcatccacgtcaatgtcattct | 313860 | - | see also 9474 line |
| 44789 | GCPHLP | NM_152401.1 | 566 | cactaccatgacaattgtttacc | 313883 | - | see also 19805 line |
| 44790 | GCPHLP | NM_152401.1 | 700 | tggagcaatacagactgatttg | 313891 | - | see also 19805 line |
| 44791 | UNC119 | NM_005148.2 | 632 | caactgcgagcacatttacgact | 313897 | - | see also 19806 line |
| 44792 | UNC119 | NM_005148.2 | 739 | accggctggtgatgcacaataaa | 313898 | - | see also 19806 line |
| 44793 | UNC119 | NM_005148.2 | 567 | ttccgcaaccagctactcaaaag | 313896 | - | see also 19806 line |
| 44794 | ARR3 | NM_004312.1 | 258 | gacgttccgaaaagatctgtatg | 313903 | - | see also 19808 line |
| 44795 | ARR3 | NM_004312.1 | 186 | atgtcgaaagttgtttgtcatgt | 313902 | - | see also 19808 line |
| 44796 | OPA3 | NM_025136.1 | 117 | agccggcttgccaaccgtattaag | 313916 | - | see also 19809 line |
| 44797 | OPA3 | NM_025136.1 | 118 | gccgcttgccaaccgtattaagg | 313917 | - | see also 19809 line |
| 44798 | PROL4 | NM_007244.1 | 262 | tcctcccccacctcctttcaaa | 313922 | - | - |
| 44799 | PROL4 | NM_007244.1 | 70 | cacagataatgatgtgaactatg | 313921 | - | see also 44798 line |
| 44800 | PROM1 | NM_006017.1 | 1103 | ctggtccaacagggctatcaatc | 313954 | - | see also 19810 line |
| 44801 | PROM1 | NM_006017.1 | 1620 | tggatacacctacttactaaat | 313972 | - | see also 19810 line |
| 44802 | RS1 | NM_000330.1 | 264 | ctctaacccggagcagtatgtgg | 314016 | - | see also 19811 line |

Figure 46

| 44803 | RS1 | NM_000330.1 | 175 | gccacctccttggactgtatacc | 314013 | - | see also 19811 line | | |
|---|---|---|---|---|---|---|---|---|---|
| 44804 | TULP1 | NM_003322.2 | 1175 | cagcgtgggtacagcactaatgt | 314031 | - | see also 2254 line | | |
| 44805 | TULP1 | NM_003322.2 | 1064 | atctccatcgaccctaccaatct | 314029 | - | see also 2254 line | | |
| 44806 | TULP2 | NM_003323.1 | 1378 | tcgctactgagtcgttaccaacg | 314055 | - | see also 19813 line | | |
| 44807 | TULP2 | NM_003323.1 | 831 | tggcacggacagcgaccatatga | 314044 | - | see also 19813 line | | |
| 44808 | ARRB2 | NM_004313.3 | 645 | ggcctgcggcgtagactttgaga | 314066 | - | see also 2886 line | | |
| 44809 | ARRB2 | NM_004313.3 | 862 | ggggagcccctcaatgtaaatgt | 314074 | - | see also 2886 line | | |
| 44810 | CLONE24945 | NM_015683.1 | 662 | tgcccgatcctggtactgtaacc | 314083 | - | see also 19815 line | | |
| 44811 | CLONE24945 | NM_015683.1 | 518 | tagtgtccgctactgtatcaagg | 314081 | - | see also 19815 line | | |
| 44812 | KIAA1376 | NM_020801.1 | 408 | ggctccaatactgcctatacaca | 314089 | - | see also 7975 line | | |
| 44813 | KIAA1376 | NM_020801.1 | 1059 | atccgcgtggaatattcactaat | 314111 | - | see also 7975 line | | |
| 44814 | STIP1 | NM_006819.1 | 279 | aagggctattcacgaaaagcagc | 314128 | - | see also 4787 line | | |
| 44815 | STIP1 | NM_006819.1 | 1472 | ctgtatgatggcgcagtacaacc | 314143 | - | see also 4787 line | | |
| 44816 | SYN2 | NM_003178.2 | 851 | cccagctggtcgctatctataag | 314152 | - | see also 2156 line | | |
| 44817 | SYN2 | NM_003178.2 | 854 | agctggtcgctatctataagaca | 314153 | - | see also 2156 line | | |
| 44818 | CALCYON | NM_015722.2 | 470 | gccgctgaccctggagatgtact | 314166 | - | see also 19819 line | | |
| 44819 | CBLN1 | NM_004352.1 | 714 | aacgggtggccggtgatttcagc | 314173 | - | see also 19821 line | | |
| 44820 | CBLN1 | NM_004352.1 | 770 | cgccagcaacggagtcctaatcc | 314174 | - | see also 19821 line | | |
| 44821 | HAP1 | NM_003949.2 | 315 | cgccgtggacccgcttcgtattc | 314177 | - | see also 19822 line | | |
| 44822 | HAP1 | NM_003949.2 | 316 | gccgtggacccgcttcgtattcc | 314178 | - | see also 19822 line | | |
| 44823 | HCRT | NM_001524.1 | 195 | gcccgactgctgtcgtcaaaaga | 314191 | - | signal_transduction | - | narcolepsy、porphyria、diabetes |
| 44824 | HCRT | NM_001524.1 | 197 | ccgactgctgtcgtcaaaagact | 314192 | - | see also 44823 line | | |
| 44825 | HCRT | NM_001524.1 | 199 | gactgctgtcgtcaaaagacttg | 314193 | - | see also 44823 line | | |
| 44826 | MOG | NM_002433.1 | 734 | ctctcaggatccggaatgtaagg | 314197 | - | see also 1567 line | | |
| 44827 | MOG | NM_002433.1 | 1087 | aactggctacatcgaagactagc | 314206 | - | see also 1567 line | | |
| 44828 | CNTNAP2 | NM_014141.3 | 1762 | cgggaagtttcgcgaatgtcagc | 314250 | - | see also 19824 line | | |
| 44829 | CNTNAP2 | NM_014141.3 | 3419 | cacccgagagccatacaatattg | 314278 | - | see also 19824 line | | |
| 44830 | F13B | NM_001994.1 | 1260 | gacgggatattggcaagctatgc | 314318 | - | see also 19825 line | | |
| 44831 | F13B | NM_001994.1 | 1141 | ttctccatggatcgaatgagata | 314314 | - | see also 19825 line | | |
| 44832 | GP5 | NM_004488.1 | 579 | gaggctgtcgcgcaacaaaatca | 314344 | - | see also 19826 line | | |
| 44833 | GP5 | NM_004488.1 | 577 | ctgaggctgtcgcgcaacaaaat | 314343 | - | see also 19826 line | | |
| 44834 | GP5 | NM_004488.1 | 581 | ggctgtcgcgcaacaaaatcacg | 314345 | - | see also 19826 line | | |
| 44835 | MMRN | NM_007351.1 | 3388 | ctcatacgtatggaatgactata | 314494 | - | see also 19827 line | | |
| 44836 | MMRN | NM_007351.1 | 2678 | tacccagacgctcataccttatt | 314456 | - | see also 19827 line | | |
| 44837 | TRO | NM_016157.2 | 1418 | accgcgagatgtggccattttac | 314533 | - | see also 19828 line | | |

Figure 46

| | | | | | | | |
|---|---|---|---|---|---|---|---|
| 44838 | TRO | NM_016157.2 | 1419 | ccgcgagatgtggccatttaca | 314534 | - | see also 19828 line |
| 44839 | BYSL | NM_004053.2 | 635 | tccggaggtattatcctaagtac | 314550 | - | see also 19829 line |
| 44840 | BYSL | NM_004053.2 | 629 | gggggtccgggaggtattatct | 314548 | - | see also 19829 line |
| 44841 | PSG6 | NM_002782.3 | 143 | cccaccactgcccaagtaataat | 314561 | - | - |
| 44842 | CSN1S1 | NM_001890.1 | 337 | cagtaagtgtgcggaacagttt | 314568 | - | see also 19830 line |
| 44843 | CSN1S1 | NM_001890.1 | 324 | aggaaatgtctctcagtaagtgt | 314567 | - | see also 19830 line |
| 44844 | NPHS1 | NM_004646.1 | 1294 | tcgctcatcctgaacgtaaaata | 314585 | - | see also 19831 line |
| 44845 | NPHS1 | NM_004646.1 | 3322 | gaccgagtcaggaacgaatatga | 314607 | - | see also 19831 line |
| 44846 | NPHS2 | NM_014625.1 | 1178 | ttgagccactaaatcctaaaag | 314631 | - | see also 5796 line |
| 44847 | NPHS2 | NM_014625.1 | 1094 | tgccatttgacctactgaattgc | 314629 | - | see also 5796 line |
| 44848 | NPHS2 | NM_014625.1 | 604 | atcgtgaccaaagacatgtttat | 314622 | - | see also 5796 line |
| 44849 | TG737 | NM_006531.2 | 1827 | cacgcaatcctacgaaacagtgc | 314674 | - | see also 4565 line |
| 44850 | TG737 | NM_006531.2 | 833 | tgccgaagcacttaacacttatc | 314643 | - | see also 4565 line |
| 44851 | IBSP | NM_004967.2 | 261 | ggccacgatattatctttacaag | 314707 | - | see also 19834 line |
| 44852 | IBSP | NM_004967.2 | 257 | tacaggccacgatattatcttta | 314705 | - | see also 19834 line |
| 44853 | SFTPC | NM_003018.1 | 324 | tggcctcgtgtgtatgactacc | 314720 | - | see also 19835 line |
| 44854 | SFTPC | NM_003018.1 | 596 | gcgaggtgccgctctactacatc | 314722 | - | see also 19835 line |
| 44855 | FMR2 | NM_002025.1 | 2591 | gccaaagrctcgggaattcattg | 314761 | - | see also 1314 line |
| 44856 | FMR2 | NM_002025.1 | 953 | gtggtcaagtgatagtcataacc | 314737 | - | see also 1314 line |
| 44857 | GLIPR1 | NM_006851.1 | 238 | gcgttcgaatccataacaagttc | 314798 | - | see also 19837 line |
| 44858 | GLIPR1 | NM_006851.1 | 571 | aagttggctgcgcagttcaattt | 314815 | - | see also 19837 line |
| 44859 | PSORS1C1 | NM_014068.1 | 450 | gggacctccaagcaatgatatcc | 314834 | - | see also 19838 line |
| 44860 | PSORS1C1 | NM_014068.1 | 449 | gggacctccaagcaatgatatc | 314833 | - | see also 19839 line |
| 44861 | TTC3 | NM_003316.2 | 5061 | ggggagtatgtacgagttaaact | 314841 | - | see also 19839 line |
| 44862 | TTC3 | NM_003316.2 | 4349 | ctctgagccagcatcattgaagg | 314839 | - | see also 19839 line |
| 44863 | APBB1IP | NM_019043.2 | 1136 | ctcgagatctggcgtgttttata | 314862 | - | see also 7676 line |
| 44864 | APBB1IP | NM_019043.2 | 1137 | tcgagatctgcgtgttttac | 314863 | - | see also 7676 line |
| 44865 | APBB1IP | NM_019043.2 | 1212 | agcgcccactgactattgctttg | 314866 | - | see also 7676 line |
| 44866 | C12orf2 | NM_007211.2 | 1346 | aaggagttgcggcaagtcaatct | 314891 | - | see also 19841 line |
| 44867 | C12orf2 | NM_007211.2 | 407 | gccttagctcaagcaataggtcg | 314873 | - | see also 19841 line |
| 44868 | FLJ10324 | NM_018059.2 | 1083 | ggcgcacatctccgtcaacttct | 314899 | - | see also 19842 line |
| 44869 | FLJ10324 | NM_018059.2 | 1816 | ttccagcagtgcgtctactatgt | 314902 | - | see also 19842 line |
| 44870 | FLJ10324 | NM_018059.2 | 2178 | ggcggctggagagcacttcttcc | 314904 | - | see also 19842 line |
| 44871 | NMB | NM_021077.2 | 303 | cggaatcctcctgctcaaagaagg | 314908 | - | see also 19843 line |
| 44872 | PKD1L2 | NM_052892.2 | 6979 | atcggtcaaacttgatatttgg | 314964 | - | see also 19844 line |
| 44873 | PKD1L2 | NM_052892.2 | 1203 | gggctcgctaccgtacaatctga | 314918 | - | see also 19844 line |
| 44874 | RASSF1 | NM_007182.4 | 1100 | ctcctattgccgccagaagatcc | 314978 | - | see also 19845 line |

EP 1 752 536 A1

Figure 46

| | | | | | | | |
|---|---|---|---|---|---|---|---|
| 44875 | RASSF1 | NM_007182.4 | 594 | aacaaggacggttcttacacagg | 314973 | - | see also 19845 line |
| 44876 | RASSF1 | NM_007182.4 | 1087 | tcctgcagaagtactcctattgc | 314977 | - | see also 19845 line |
| 44877 | RASSF2 | NM_014737.1 | 1112 | accgtgtccggctaatgattcg | 314997 | - | see also 5925 line |
| 44878 | RASSF2 | NM_014737.1 | 1028 | ttcgagatgccggtacttaaaag | 314995 | - | see also 5925 line |
| 44879 | RASSF3 | NM_178169.2 | 214 | ccccgctccggccaacaagatgt | 315003 | - | see also 19847 line |
| 44880 | RASSF3 | NM_178169.2 | 436 | agcaatggctgtatgaatacact | 315013 | - | see also 19847 line |
| 44881 | RASSF3 | NM_178169.2 | 637 | cccagaacagacacacttagttt | 315016 | - | see also 19847 line |
| 44882 | RASSF4 | NM_032023.3 | 622 | ggcgacaccggttctctatcaac | 315019 | - | see also 9061 line |
| 44883 | RASSF4 | NM_032023.3 | 623 | gcgacaccggttcctcatccaacg | 315020 | - | see also 9061 line |
| 44884 | RASSF5 | NM_031437.1 | 759 | cacctacacggtttcatcaaag | 315031 | - | see also 9028 line |
| 44885 | RASSF6 | NM_177532.2 | 441 | gacgatctatcgtattagtga | 315055 | - | see also 19850 line |
| 44886 | RASSF6 | NM_177532.2 | 323 | gggagtaaaacgacctatacagc | 315048 | - | see also 19850 line |
| 44887 | HOMER2 | NM_004839.1 | 361 | atcgagaactcaagtaatcattc | 315081 | - | see also 19851 line |
| 44888 | HOMER2 | NM_004839.1 | 151 | gacggagccaaggtgatcataaa | 315076 | - | see also 19851 line |
| 44889 | CD47 | NM_001777.2 | 519 | aaacgatcatcgagctaaaatatc | 315102 | - | see also 19852 line |
| 44890 | CD47 | NM_001777.2 | 919 | ctctgtattgcgcgcgtgtatacc | 315119 | - | see also 19852 line |
| 44891 | CD47 | NM_001777.2 | 520 | acgatcatcgagctaaaatatcg | 315103 | - | see also 19852 line |
| 44892 | SIRPB1 | NM_006065.1 | 1098 | aggagcacggctcagatatcacc | 315131 | - | see also 19853 line |
| 44893 | SIRPB1 | NM_006065.1 | 931 | agcttcgaccctcatagagaaca | 315130 | - | see also 19853 line |
| 44894 | PARK7 | NM_007262.3 | 568 | ttcgagtttgccgttgcaattgt | 315139 | - | see also 19854 line |
| 44895 | PARK7 | NM_007262.3 | 162 | gaggcgagctggagttaaggtca | 315136 | - | see also 19854 line |
| 44896 | EDARADD | NM_080738.2 | 546 | tactagacgtgatcaggataaag | 315155 | - | see also 19855 line |
| 44897 | EDARADD | NM_080738.2 | 375 | gcccacgaattcagatatgaaa | 315151 | - | see also 19856 line |
| 44898 | AIP1 | NM_012301.2 | 580 | gtcactacctcaacttacgattt | 315163 | - | see also 19856 line |
| 44899 | AIP1 | NM_012301.2 | 671 | atgcaccacaaggcacattaagg | 315165 | - | see also 19856 line |
| 44900 | ALP | NM_014476.1 | 398 | caggacggaactacttggaaca | 315229 | - | see also 19857 line |
| 44901 | ALP | NM_014476.1 | 326 | cacttatggtctccacaagtatc | 315228 | - | see also 19857 line |
| 44902 | ARHGAP21 | NM_020824.1 | 1002 | agcggcaatgccgcaattatacc | 315248 | - | see also 19858 line |
| 44903 | ARHGAP21 | NM_020824.1 | 1001 | tagcggcaatgcccgcaatatac | 315247 | - | see also 19858 line |
| 44904 | ASB10 | NM_080871.2 | 576 | gagactctggtctctgacatacg | 315383 | - | see also 19859 line |
| 44905 | ASB11 | NM_080873.1 | 753 | agcttaacctgaagcgtagaaatg | 315397 | - | see also 19860 line |
| 44906 | ASB11 | NM_080873.1 | 936 | gccagagcactcgaacgattcc | 315401 | - | see also 19860 line |
| 44907 | ASB11 | NM_080873.1 | 226 | gggcgcttactgcccttaaaac | 315388 | - | see also 19860 line |

1521

Figure 46

| 44908 | ASB13 | NM_024701.2 | 721 | ttcgagtactacgaaaagacacc | 315409 | - | see also 19861 line |
|---|---|---|---|---|---|---|---|
| 44909 | ASB13 | NM_024701.2 | 711 | cgccaagtgcttcgagtactacg | 315408 | - | see also 19861 line |
| 44910 | ASB14 | NM_130387.4 | 653 | atcaagttcggatctgttcaaag | 315429 | - | see also 19862 line |
| 44911 | ASB14 | NM_130387.4 | 777 | atgcatgggacggttacatttgc | 315437 | - | see also 19862 line |
| 44912 | ASB15 | NM_080928.2 | 197 | aagtgctcaaaacagaaaacttg | 315448 | - | see also 9543 line |
| 44913 | ASB15 | NM_080928.2 | 552 | atggtgtcgactctgatcaaaca | 315453 | - | see also 9543 line |
| 44914 | ASB17 | NM_080868.1 | 699 | ctctacccttcgagagtaagagt | 315483 | - | see also 19864 line |
| 44915 | ASB17 | NM_080868.1 | 368 | atcaggataccgttttgaagtaa | 315466 | - | see also 19864 line |
| 44916 | ASB17 | NM_080868.1 | 697 | tactctacccttcgagagtaaga | 315482 | - | see also 19864 line |
| 44917 | ASB2 | NM_016150.3 | 1737 | cgcgaacatcgacgcctatatcg | 315507 | - | see also 19865 line |
| 44918 | ASB2 | NM_016150.3 | 657 | gcctgcggacccctttgataaagg | 315500 | - | see also 19865 line |
| 44919 | ASB3 | NM_016115.3 | 1571 | tacggtctgacagttatattagt | 315560 | - | see also 6671 line |
| 44920 | ASB3 | NM_016115.3 | 1195 | aagtacgagaagttttcgatatt | 315543 | - | see also 6671 line |
| 44921 | ASB4 | NM_016116.1 | 69 | ggcgctaaagtccaatgacttcg | 315569 | - | see also 19867 line |
| 44922 | ASB4 | NM_016116.1 | 738 | tgcccgagatgacgactttaaat | 315585 | - | see also 19867 line |
| 44923 | ASB5 | NM_080874.2 | 420 | atggcgtgactccgttattcaac | 315605 | - | see also 19868 line |
| 44924 | ASB5 | NM_080874.2 | 36 | atcggccgtttgctcaacaatta | 315597 | - | see also 19868 line |
| 44925 | ASB6 | NM_017873.2 | 1192 | gcccgaaaacttcgatatccacc | 315633 | - | see also 19869 line |
| 44926 | ASB6 | NM_017873.2 | 1188 | agctgcccgaaaacttcgatatc | 315632 | - | see also 19869 line |
| 44927 | ASB8 | NM_024095.2 | 110 | ctctctctccgagcgcttaatcc | 315638 | - | see also 19870 line |
| 44928 | ASB8 | NM_024095.2 | 123 | cgcttaatccgaacaattgctgc | 315640 | - | see also 19870 line |
| 44929 | ASB9 | NM_024087.1 | 490 | ctggcacactccactgtttaatg | 315659 | - | see also 19871 line |
| 44930 | ASB9 | NM_024087.1 | 524 | ggcagctgggattgtgtgaattt | 315660 | - | see also 19871 line |
| 44931 | C20orf163 | NM_080749.2 | 871 | cacggccgacatgcacatcatca | 315667 | - | see also 19872 line |
| 44932 | C20orf163 | NM_080749.2 | 688 | aaccctcctcgtggaaccatatc | 315665 | - | see also 19872 line |
| 44933 | CTEN | NM_032865.3 | 404 | accttgactcctacattgacttc | 315674 | - | see also 19873 line |
| 44934 | CTEN | NM_032865.3 | 557 | aagccttggacataaagtacatc | 315676 | - | see also 19873 line |
| 44935 | DCDC2 | NM_016356.1 | 679 | acgtgtcagctcgctttagaaaa | 315688 | - | see also 6840 line |
| 44936 | DCDC2 | NM_016356.1 | 1061 | tggaaatgatcgccactctaagt | 315701 | - | see also 6840 line |
| 44937 | EFS | NM_005864.2 | 1279 | aacgagcgtcagccttactcaat | 315708 | - | see also 19875 line |
| 44938 | EFS | NM_005864.2 | 1739 | ggccgaggagtatgactatgtcc | 315712 | - | see also 19875 line |
| 44939 | EFS | NM_005864.2 | 2048 | tgctcatcgcctggtgtttgttg | 315715 | - | see also 19875 line |
| 44940 | ENIGMA | NM_005451.2 | 617 | aggagcacctgaagaaatcaagc | 315717 | - | see also 19876 line |
| 44941 | ENIGMA | NM_005451.2 | 1347 | ctgtgcgatatgtcagatcaacc | 315720 | - | see also 19876 line |
| 44942 | ERBB2IP | NM_018695.1 | 3752 | gagtgctcgaccctctattaatg | 315826 | - | see also 19877 line |
| 44943 | ERBB2IP | NM_018695.1 | 1689 | cagcgggctcaagttgcatttga | 315767 | - | see also 19877 line |
| 44944 | FLJ12428 | NM_022783.1 | 462 | ttctaccgctttagaaaggatga | 315859 | - | see also 8477 line |

Figure 46

| | | | | | | | |
|---|---|---|---|---|---|---|---|
| 44945 | FLJ12428 | NM_022783.1 | 1276 | accggaccgtgagcaatctgatt | 315876 | - | see also 8477 line |
| 44946 | FLJ12987 | NM_025170.2 | 437 | aacggcggaaaggtttaaaatta | 315892 | - | see also 19879 line |
| 44947 | FLJ12987 | NM_025170.2 | 901 | ctgatagactggttaattgcaca | 315915 | - | see also 19879 line |
| 44948 | FLJ14249 | NM_022460.2 | 174 | cccgtctggctgcgttcaagtcg | 315950 | - | see also 19880 line |
| 44949 | FLJ14249 | NM_022460.2 | 462 | cagggctcaccagcagagattcc | 315956 | - | see also 19880 line |
| 44950 | FLJ20967 | NM_022071.2 | 744 | tccggataacagcagagactaaag | 315972 | - | see also 19881 line |
| 44951 | FLJ20967 | NM_022071.2 | 577 | atgggcgaacaccatctagataa | 315967 | - | see also 19881 line |
| 44952 | FLJ21687 | NM_024859.1 | 183 | accgttgttagagacatgtaac | 315992 | - | see also 19882 line |
| 44953 | FLJ21687 | NM_024859.1 | 177 | tccagcaccgttggttagagaca | 315991 | - | see also 19882 line |
| 44954 | FLJ21687 | NM_024859.1 | 673 | atcccggacgcgctcaagaaga | 315995 | - | see also 19882 line |
| 44955 | FLJ23209 | NM_024895.3 | 332 | aacgcgatacctgctaaggaagc | 315996 | - | see also 19883 line |
| 44956 | FLJ23209 | NM_024895.3 | 838 | ggcgcatcgtccacctatacaca | 315999 | - | see also 19883 line |
| 44957 | FLJ23209 | NM_024895.3 | 1693 | gcgggagcctggccaaaacttac | 316002 | - | see also 19883 line |
| 44958 | FLJ30934 | NM_152760.1 | 492 | tgcgatgcacgaagtctttctgc | 316007 | - | see also 19884 line |
| 44959 | FLJ30934 | NM_152760.1 | 539 | tgcgtcgagaccaacaacttcttt | 316008 | - | see also 19884 line |
| 44960 | FLJ30934 | NM_152760.1 | 414 | ctctgtcactcgggaagagtttg | 316006 | - | see also 19884 line |
| 44961 | FLJ32798 | NM_173496.2 | 1429 | accgtatcggcgacaaactaatg | 316035 | - | see also 19885 line |
| 44962 | FLJ32798 | NM_173496.2 | 1428 | caccgtatcggcgacaaactaat | 316034 | - | see also 19885 line |
| 44963 | FLJ33505 | NM_152317.1 | 487 | ggcaataaatcatcttacgattg | 316054 | - | see also 19886 line |
| 44964 | FLJ33505 | NM_152317.1 | 322 | aacacgtgccaagtagcaatga | 316051 | - | see also 19886 line |
| 44965 | FLJ38993 | NM_152495.1 | 973 | ggctgaatgtccctactttc | 316068 | - | - |
| 44966 | FLJ38993 | NM_152495.1 | 765 | ttcgccatctggcacataattgc | 316066 | - | see also 44965 line |
| 44967 | FLJ38993 | NM_152495.1 | 919 | tccatagcctcttctgcattag | 316067 | - | see also 44965 line |
| 44968 | GIPC2 | NM_017655.3 | 418 | ctcgccgtcggagatcttatatt | 316070 | - | see also 19887 line |
| 44969 | GIPC2 | NM_017655.3 | 420 | cgccgtcggagatcttatattgc | 316072 | - | see also 19887 line |
| 44970 | GIPC3 | NM_133261.1 | 753 | ggcatctcggaaggttgatgacc | 316098 | - | see also 19888 line |
| 44971 | GIPC3 | NM_133261.1 | 724 | gaggaagcgcccagtgagtttga | 316097 | - | see also 19888 line |
| 44972 | GOPC | NM_020399.1 | 918 | aaggtgttggtccaattagaaaa | 316117 | - | see also 7864 line |
| 44973 | GOPC | NM_020399.1 | 1283 | aaccctgtctgtagttgcaaaga | 316128 | - | see also 7864 line |
| 44974 | GORASP2 | NM_015530.3 | 583 | atcattcagccttatcgaaaca | 316145 | - | see also 19890 line |
| 44975 | GORASP2 | NM_015530.3 | 795 | atggctggtacacctattacacc | 316151 | - | see also 19890 line |
| 44976 | HSH2 | NM_032855.2 | 1202 | acctcgccactgtgaacttgtcg | 316166 | - | see also 19891 line |
| 44977 | HSH2 | NM_032855.2 | 1177 | agggtccggcagcagcctaaaaag | 316165 | - | see also 19891 line |
| 44978 | HSPC163 | NM_014184.1 | 88 | ctctcggtcacttcataattac | 316170 | - | see also 19892 line |
| 44979 | HSPC163 | NM_014184.1 | 85 | ttctctcggtcacttcataat | 316168 | - | see also 19892 line |
| 44980 | HSPC163 | NM_014184.1 | 86 | tctctcggtcacttcataatt | 316169 | - | see also 19892 line |
| 44981 | KIAA0645 | NM_014662.1 | 181 | ttgcacacccaacgatgaatac | 316187 | - | see also 5833 line |

## Figure 46

| 44982 | KIAA1365 | NM_020794.1 | 2960 | gccacgaacgaccggataagatg | 316373 | - | see also 19893 line |
|---|---|---|---|---|---|---|---|
| 44983 | KIAA1365 | NM_020794.1 | 2701 | ggcgtaccatgggagtatcatga | 316362 | - | see also 19893 line |
| 44984 | KIAA1444 | NM_032512.1 | 719 | ctgcgtgctcgtaaactgaaatc | 316420 | - | see also 19894 line |
| 44985 | KIAA1444 | NM_032512.1 | 717 | agctgcgtgctcgtaaactgaaa | 316419 | - | see also 19894 line |
| 44986 | LMO7 | NM_005358.3 | 2177 | ctgcgttacccttcaatcgtttt | 316457 | - | see also 19895 line |
| 44987 | LMO7 | NM_005358.3 | 2178 | tgcgttacccttcaatcgttttt | 316458 | - | see also 19895 line |
| 44988 | LNK | NM_005475.1 | 2006 | cccgggactcggactacgaaatg | 316527 | - | see also 19896 line |
| 44989 | LNK | NM_005475.1 | 1076 | tcgacccacccaagagttcaagg | 316519 | - | see also 19896 line |
| 44990 | LNX | NM_032622.1 | 1833 | taccacggtgcttgtataactgt | 316572 | - | see also 9261 line |
| 44991 | LNX | NM_032622.1 | 1690 | atcatcctcgatagtactcaaag | 316569 | - | see also 9261 line |
| 44992 | LNX | NM_032622.1 | 1836 | cacggtgcttgtataactgtaaa | 316573 | - | see also 9261 line |
| 44993 | LOC118987 | NM_173791.2 | 1618 | ctcatgccaatcgggttatgaag | 316615 | - | see also 19898 line |
| 44994 | LOC118987 | NM_173791.2 | 1047 | agcacaccctaccgaattacaag | 316593 | - | see also 19898 line |
| 44995 | LOC51248 | NM_016484.3 | 177 | atggacagccggattccttatga | 316684 | - | see also 6875 line |
| 44996 | LOC51248 | NM_016484.3 | 274 | acccggactacaacaatgagttg | 316689 | - | see also 6875 line |
| 44997 | MAGI1 | NM_173515.1 | 1237 | atgatgggttacacgtgttattact | 316710 | - | see also 19900 line |
| 44998 | MAGI1 | NM_173515.1 | 1947 | gagcggattcctccgatcattga | 316723 | - | see also 19900 line |
| 44999 | MGC2793 | NM_145064.1 | 437 | ctcctaagctggtcaacgataag | 316732 | - | see also 9773 line |
| 45000 | MGC32065 | NM_153271.1 | 1704 | gcgccgtgagctcgacttcaagc | 316752 | - | see also 19902 line |
| 45001 | MGC32065 | NM_153271.1 | 108 | aggccgagccctctatgactttc | 316746 | - | see also 19902 line |
| 45002 | MGC39715 | NM_152628.2 | 291 | agctaaggcgggtctttggaaat | 316754 | - | see also 19903 line |
| 45003 | MGC39715 | NM_152628.2 | 747 | ttggactccgaaagtggtatatg | 316768 | - | see also 19903 line |
| 45004 | MPP4 | NM_033066.1 | 1585 | gccacacactactcgtactaaaa | 316823 | - | see also 19905 line |
| 45005 | MPP4 | NM_033066.1 | 485 | gaggtagtggagttattacgtga | 316799 | - | see also 19905 line |
| 45006 | MPP5 | NM_022474.2 | 1298 | gtcatcattagccggatagtaaa | 316859 | - | see also 8392 line |
| 45007 | MPP5 | NM_022474.2 | 1301 | atcattagccggatagtaaaagg | 316860 | - | see also 8392 line |
| 45008 | NCK1 | NM_006153.3 | 647 | agcagtcgtcaataacctaaata | 316910 | - | see also 4255 line |
| 45009 | NOXO1 | NM_144603.2 | 776 | cacgcgggataggccttttcagg | 316934 | - | see also 19908 line |
| 45010 | NOXO1 | NM_144603.2 | 1027 | cgcgcgtgcgcgtgttggaaacg | 316935 | - | see also 19908 line |
| 45011 | NOXO1 | NM_144603.2 | 269 | ccccgatacccagtttcagtgc | 316931 | - | see also 19908 line |
| 45012 | PDLIM2 | NM_021630.4 | 714 | agggctccgtgaggacatacact | 316937 | - | see also 19909 line |
| 45013 | PDLIM2 | NM_021630.4 | 716 | ggctccgtgaggacatacactga | 316938 | - | see also 19909 line |

Figure 46

| 45014 | PDZK2 | NM_024791.2 | 40 | gcctcgttaactctgaagtttaa | 316943 | - | see also 19910 line |
|---|---|---|---|---|---|---|---|
| 45015 | PDZK2 | NM_024791.2 | 31 | caggacacagcctcgttaactct | 316941 | - | see also 19910 line |
| 45016 | PDZRN1 | NM_153371.2 | 1931 | gccacgatatagttttacgaaga | 317003 | - | see also 19911 line |
| 45017 | PDZRN1 | NM_153371.2 | 1930 | tgccacgatatagttttacgaag | 317002 | - | see also 19911 line |
| 45018 | PDZRN1 | NM_153371.2 | 1465 | cacacaccaccaccgtattatag | 316991 | - | see also 19911 line |
| 45019 | PSCDBP | NM_004288.2 | 492 | gtccggaaacctgctaacgatag | 317022 | - | see also 2873 line |
| 45020 | PSCDBP | NM_004288.2 | 493 | tccggaaacctgctaacgataga | 317023 | - | see also 2873 line |
| 45021 | RACGAP1 | NM_013277.2 | 2080 | taggacgacaaggcaactttttt | 317038 | - | see also 19913 line |
| 45022 | RACGAP1 | NM_013277.2 | 649 | gactatcaaccattgatgaatct | 317036 | - | see also 19913 line |
| 45023 | RHPN1 | NM_052924.1 | 639 | gggcagcgttctcttcaacatcg | 317042 | - | see also 19914 line |
| 45024 | RHPN1 | NM_052924.1 | 638 | agggcagcgttctcttcaacatc | 317041 | - | see also 19914 line |
| 45025 | RHPN2 | NM_033103.3 | 324 | ctcggtgggcgtctatcagaaca | 317048 | - | see also 19915 line |
| 45026 | RHPN2 | NM_033103.3 | 413 | cagtcgtcctcaaggatttttatc | 317051 | - | see also 19915 line |
| 45027 | RHPN2 | NM_033103.3 | 195 | gaggaccggagcggaaaaccttc | 317046 | - | see also 19915 line |
| 45028 | RIL | NM_003687.2 | 862 | accatcccgagtgcttcatgtgc | 317061 | - | - | - | - |
| 45029 | RIL | NM_003687.2 | 465 | cgctttccagtccctcacaatgg | 317060 | - | see also 45028 line |
| 45030 | RIMS2 | NM_014677.1 | 660 | cagtcacgctaccgaagtgatcc | 317068 | - | see also 5872 line |
| 45031 | RIMS2 | NM_014677.1 | 2723 | ccgcactatgaccggacattata | 317119 | - | see also 5872 line |
| 45032 | RP1L1 | NM_178857.4 | 2325 | ggctcagtgccacgatattctgg | 317161 | - | see also 19917 line |
| 45033 | RP1L1 | NM_178857.4 | 682 | cccggaggatactgctgattaag | 317151 | - | see also 19917 line |
| 45034 | SCRIB | NM_015356.1 | 2265 | gggggacgacgagggcatattca | 317186 | - | see also 19918 line |
| 45035 | SCRIB | NM_015356.1 | 808 | cagctatccatcctaaaggtaga | 317183 | - | see also 19918 line |
| 45036 | SDCBP2 | NM_015685.3 | 617 | aggtggacgggcagaatgttatc | 317191 | - | see also 19919 line |
| 45037 | SDCBP2 | NM_015685.3 | 521 | acgtcggcttcgtgatcaagaag | 317190 | - | see also 19919 line |
| 45038 | SDP35 | NM_017779.2 | 768 | acgtggagtagttatactacaaa | 317208 | - | see also 19920 line |
| 45039 | SDP35 | NM_017779.2 | 770 | gtggagtagttatactacaaaac | 317209 | - | see also 19920 line |
| 45040 | SH2B | NM_015503.1 | 3148 | aaggtccatccgagtatatcatg | 317229 | - | see also 6441 line |
| 45041 | SH2D2A | NM_003975.2 | 697 | aggaccgaagaatcaaactttgg | 317234 | - | see also 19922 line |
| 45042 | SH2D2A | NM_003975.2 | 741 | cccccagtacagcccaatcatca | 317235 | - | see also 19922 line |
| 45043 | SHANK1 | NM_016148.1 | 2629 | aagttacgagcgtccttctttcc | 317255 | - | see also 19923 line |
| 45044 | SHANK1 | NM_016148.1 | 4423 | cccgcacccggagtaagcaagc | 317260 | - | see also 19923 line |
| 45045 | SHD | NM_020209.1 | 418 | aaccccggagatgccaagtatgg | 317269 | - | see also 19924 line |
| 45046 | SHD | NM_020209.1 | 641 | gaggggtgcagctctatgacacc | 317272 | - | see also 19924 line |
| 45047 | ShrmL | NM_020859.1 | 1374 | gtggtcaggaggggttaaacttc | 317285 | - | see also 19925 line |
| 45048 | ShrmL | NM_020859.1 | 5561 | aggaaaccccggtgtatagcatg | 317304 | - | see also 19925 line |
| 45049 | SLA2 | NM_032214.2 | 953 | cccgctccctggcaaggatatac | 317324 | - | see also 19926 line |
| 45050 | SLA2 | NM_032214.2 | 952 | gcccgctccctggcaaggatata | 317323 | - | see also 19926 line |

Figure 46

| | | | | | | | |
|---|---|---|---|---|---|---|---|
| 45051 | SLA2 | NM_032214.2 | 873 | gccctgtggaccattactctga | 317322 | - | see also 19926 line |
| 45052 | SSB1 | NM_025106.2 | 468 | gaccgatcgctcaatgtctttgt | 317326 | - | see also 19927 line |
| 45053 | SSB1 | NM_025106.2 | 419 | gccccctgtcctatgatgtcc | 317325 | - | see also 19927 line |
| 45054 | SSB3 | NM_080861.3 | 174 | ctctagcaggtccactaaactgg | 317336 | - | see also 19928 line |
| 45055 | SSB3 | NM_080861.3 | 771 | ggcacggcacactcaccttttc | 317342 | - | see also 19928 line |
| 45056 | SSB4 | NM_080862.1 | 579 | cgcggcaccacgctgagttgg | 317345 | - | - |
| 45057 | SSB4 | NM_080862.1 | 1039 | tgcctcagtctctcaaaaactat | 317346 | - | see also 45056 line |
| 45058 | STAC | NM_003149.1 | 1219 | atcccctgatgtactagaaaa | 317372 | - | see also 19929 line |
| 45059 | STAC | NM_003149.1 | 772 | ggcgggtatgacctaaggaaacg | 317360 | - | see also 19929 line |
| 45060 | STMN4 | NM_030795.2 | 185 | tggccgatccctgaataagtcg | 317377 | - | see also 19930 line |
| 45061 | STMN4 | NM_030795.2 | 372 | ctcgggccaatcctttgaagtca | 317384 | - | see also 19930 line |
| 45062 | SYNJ2BP | NM_018373.1 | 322 | tgctgtagacctctttcgtaatg | 317390 | - | see also 19931 line |
| 45063 | SYNJ2BP | NM_018373.1 | 83 | atgaacggaagagtggattatt | 317386 | - | see also 19931 line |
| 45064 | TENC1 | NM_015319.2 | 1539 | ctcggtctctgtcgactacaaca | 317413 | - | see also 19932 line |
| 45065 | TENC1 | NM_015319.2 | 893 | cacagcacgtggtcgtactatac | 317400 | - | see also 19932 line |
| 45066 | TENC1 | NM_015319.2 | 896 | agcacgtggtcgtactactgc | 317401 | - | see also 19932 line |
| 45067 | TJP2 | NM_004817.1 | 3208 | tacctttgggcggtctatactga | 317480 | - | see also 3347 line |
| 45068 | TJP2 | NM_004817.1 | 1078 | ccgaacgggtctggcaactaaag | 317433 | - | see also 3347 line |
| 45069 | WSB2 | NM_018639.3 | 609 | tccgcgtcacggataagactct | 317490 | - | see also 19934 line |
| 45070 | WSB2 | NM_018639.3 | 863 | cacggcttcttacgataccaatg | 317496 | - | see also 19934 line |
| 45071 | XTP1 | NM_018369.1 | 505 | aagcgtcacagtatgtcaattgg | 317519 | - | see also 7423 line |
| 45072 | XTP1 | NM_018369.1 | 498 | gtggtacaagcgtcacagtattg | 317516 | - | see also 7423 line |
| 45073 | CHL1 | NM_006614.2 | 2762 | atccatggggtggacgttataaa | 317648 | - | see also 4676 line |
| 45074 | CHL1 | NM_006614.2 | 3444 | aacatatagttgcctaatgact | 317672 | - | see also 4676 line |
| 45075 | CHL1 | NM_006614.2 | 3766 | gtcccttaatagggatatgcagc | 317685 | - | see also 4676 line |
| 45076 | FLJ38991 | NM_173827.1 | 882 | aacggtaccctcatcaattgttc | 317718 | - | see also 19937 line |
| 45077 | FLJ38991 | NM_173827.1 | 883 | acggtaccctcatcaattgttct | 317719 | - | see also 19937 line |
| 45078 | HAN11 | NM_005828.1 | 532 | caggtgttagggcgagtgaatct | 317742 | - | see also 19938 line |
| 45079 | HAN11 | NM_005828.1 | 336 | ggcaacaagcggtgactatctcc | 317734 | - | see also 19938 line |
| 45080 | INPP4A | NM_001566.1 | 773 | gtgaccgttgtaggttaacatcacc | 317766 | - | see also 1065 line |
| 45081 | INPP4A | NM_001566.1 | 2463 | aggaaatcgcgacgggtttaacg | 317786 | - | see also 1065 line |
| 45082 | LRDD | NM_018494.2 | 968 | gacacgctacctcctgagattgg | 317807 | - | see also 19940 line |
| 45083 | LRDD | NM_018494.2 | 2303 | ttcgagggcgaagagttctttgc | 317812 | - | see also 19940 line |
| 45084 | RSU1 | NM_012425.3 | 823 | agcttggcgttgtccatgtttt | 317825 | - | see also 19941 line |
| 45085 | RSU1 | NM_012425.3 | 908 | accaccgaagaagaataatgaca | 317828 | - | see also 19941 line |
| 45086 | RSU1 | NM_012425.3 | 910 | caccgaagaagaataatgacaa | 317829 | - | see also 19941 line |
| 45087 | SH3GL2 | NM_003026.1 | 535 | gtcgacgcctggatttgattat | 317836 | - | see also 19942 line |

Figure 46

| | | | | | | | |
|---|---|---|---|---|---|---|---|
| 45088 | SH3GL2 | NM_003026.1 | 536 | tcgacgcctggatttgattata | 317837 | - | see also 19942 line |
| 45089 | SH3GL3 | NM_003027.2 | 1030 | aagaaacgagtaggtaagatacc | 317862 | - | see also 2029 line |
| 45090 | SH3GL3 | NM_003027.2 | 594 | aactaaactagacgatgaattc | 317850 | - | see also 2029 line |
| 45091 | UNC5CL | NM_173561.1 | 359 | gaccatgtccgccagttgatgc | 317873 | - | see also 19944 line |
| 45092 | UNC5CL | NM_173561.1 | 1207 | atggcttggagaccaagtatatg | 317882 | - | see also 19944 line |
| 45093 | NCAM2 | NM_004540.2 | 727 | gacaatcggttcgctatgttagc | 317907 | - | see also 3097 line |
| 45094 | NCAM2 | NM_004540.2 | 1294 | agcctgacgacggcgtatcgaagt | 317916 | - | see also 3097 line |
| 45095 | NCAM2 | NM_004540.2 | 474 | gtcacggttaaccatctacaatg | 317899 | - | see also 3097 line |
| 45096 | NINJ2 | NM_016533.4 | 595 | tacagccttcgggcacataaaa | 317972 | - | see also 19946 line |
| 45097 | NINJ2 | NM_016533.4 | 599 | gccttcgggcacataaaacagg | 317974 | - | see also 19946 line |
| 45098 | EVA1 | NM_005797.2 | 396 | accatgagtggcggtttaagg | 317977 | - | see also 19947 line |
| 45099 | EVA1 | NM_005797.2 | 259 | gggacagatgtccggttaaaatg | 317976 | - | see also 19947 line |
| 45100 | ICAM5 | NM_003259.2 | 810 | tgcactctgacgagctgtttcc | 317984 | - | see also 2209 line |
| 45101 | ICAM5 | NM_003259.2 | 2059 | caccggagatgatgaatctacc | 317990 | - | see also 2209 line |
| 45102 | ICAM5 | NM_003259.2 | 1262 | cgcagagcttcgtgtcctatacg | 317985 | - | see also 2209 line |
| 45103 | JAM2 | NM_021219.2 | 1263 | atcatagacagcctagtagttgt | 318007 | - | see also 19948 line |
| 45104 | JAM2 | NM_021219.2 | 1190 | agcccgcaattcgttggatatc | 318004 | - | see also 19948 line |
| 45105 | VCAM1 | NM_001078.2 | 512 | agccgatcacagtcaagtgttca | 318020 | - | see also 672 line |
| 45106 | ZAN | NM_003386.1 | 7344 | caccgtctacggctataaagtgc | 318137 | - | see also 19950 line |
| 45107 | ZAN | NM_003386.1 | 3299 | ctccaaatgccgctacgaatcc | 318094 | - | see also 19950 line |
| 45108 | ZAN | NM_003386.1 | 7342 | accacgtctacggctataaagt | 318136 | - | see also 19950 line |
| 45109 | CDA08 | NM_030790.2 | 1187 | aagagccgcgtcgaatgtttaaa | 318166 | - | see also 19951 line |
| 45110 | CDA08 | NM_030790.2 | 1191 | gggcgctcgaatgtttaaagtct | 318168 | - | see also 19951 line |
| 45111 | CDA08 | NM_030790.2 | 1600 | gaccatctcacgttggtattcc | 318192 | - | see also 19951 line |
| 45112 | COL17A1 | NM_000494.2 | 3555 | agctgagtagtcgcattcctcagc | 318238 | - | see also 19952 line |
| 45113 | COL17A1 | NM_000494.2 | 1370 | ggcctaaaagccgaagctaatgg | 318225 | - | see also 19952 line |
| 45114 | COL17A1 | NM_000494.2 | 3770 | ctctcgtcttcacttacactgc | 318243 | - | see also 19952 line |
| 45115 | DKFZP761 D0211 | NM_032039.1 | 425 | tgccgctgttatctatgactttc | 318256 | - | see also 19953 line |
| 45116 | DKFZP761 D0211 | NM_032039.1 | 422 | cagtgccgctgttatctatgact | 318254 | | |
| 45117 | MDS028 | NM_018463.2 | 670 | gccaggttgtcgtatgcaattc | 318275 | - | see also 19954 line |
| 45118 | MDS028 | NM_018463.2 | 1223 | gagcggatggagtctaccaatct | 318287 | - | see also 19954 line |
| 45119 | PPFIA2 | NM_003625.2 | 2538 | cacaatgatgctcgaagtagtt | 318335 | - | see also 2432 line |
| 45120 | PPFIA2 | NM_003625.2 | 950 | aaccgatgaaactagtcaaatag | 318304 | - | see also 2432 line |
| 45121 | SGCE | NM_003919.1 | 1002 | gactattacacggatttcctaat | 318388 | - | see also 2678 line |
| 45122 | HNT | NM_016522.2 | 1291 | gcccgtggtacggaggtaaagg | 318404 | - | see also 19957 line |

Figure 46

| 45123 | HNT | NM_016522.2 | 1290 | cgcccgtggtacggagagtaaag | 318403 | - | see also 19957 line |
|---|---|---|---|---|---|---|---|
| 45124 | HNT | NM_016522.2 | 825 | ccctcaggtgcactattgacaac | 318396 | - | see also 19957 line |
| 45125 | CASPR3 | NM_033655.1 | 1855 | ttgattcaggtgacacctattat | 318412 | - | see also 19958 line |
| 45126 | ADRM1 | NM_007002.2 | 233 | gacggacgactcgcttattcact | 318415 | - | see also 19959 line |
| 45127 | ADRM1 | NM_007002.2 | 1040 | gccgcagaccgcggatgagatcc | 318423 | - | see also 19959 line |
| 45128 | C1orf38 | NM_004848.1 | 392 | ctcccggcacgtccactttatca | 318425 | - | see also 19960 line |
| 45129 | C1orf38 | NM_004848.1 | 516 | tgggtgggctccttgcaaaaagg | 318429 | - | see also 19960 line |
| 45130 | CASPR4 | NM_033401.2 | 2052 | agcacaacggctctgacttaaca | 318481 | - | see also 9420 line |
| 45131 | CASPR4 | NM_033401.2 | 1890 | gaggagctacttgccataactct | 318475 | - | see also 9420 line |
| 45132 | caspr5 | NM_130773.2 | 2432 | acgccggatggaacaccatttac | 318598 | - | see also 19962 line |
| 45133 | caspr5 | NM_130773.2 | 1836 | gagggtgccccgacaatctcacc | 318580 | - | see also 19962 line |
| 45134 | CD151 | NM_004357.3 | 248 | ccctcaagagtgactacatcagc | 318646 | - | see also 2919 line |
| 45135 | CD151 | NM_004357.3 | 172 | tacctgctgtttacctacaattg | 318645 | - | see also 2919 line |
| 45136 | CD151 | NM_004357.3 | 133 | aacgagaagaagacaacatgtgg | 318644 | - | see also 2919 line |
| 45137 | CD34 | NM_001773.1 | 559 | cccactaaaccctatacatcatc | 318658 | - | see also 19963 line |
| 45138 | CD34 | NM_001773.1 | 174 | tgggttcatgagtcttgacaaca | 318650 | - | see also 19963 line |
| 45139 | CD99 | NM_002414.3 | 237 | ccccggatggtggtttcgatttat | 318664 | - | see also 19964 line |
| 45140 | CD99 | NM_002414.3 | 236 | ccccggatggtggtttcgattta | 318663 | - | see also 19964 line |
| 45141 | CD99 | NM_002414.3 | 430 | tcctccggtagcttttcagatgc | 318670 | - | see also 19964 line |
| 45142 | CDON | NM_016952.1 | 773 | cggcggactccggaaactattcc | 318695 | - | see also 19965 line |
| 45143 | CDON | NM_016952.1 | 1220 | tgccggttccggtcattcgttgg | 318708 | - | see also 19965 line |
| 45144 | CNTN2 | NM_005076.2 | 1814 | ctcaagtgccgacatcaacttgg | 318771 | - | see also 19966 line |
| 45145 | CNTN2 | NM_005076.2 | 1939 | gagggcactaccggagaactaat | 318772 | - | see also 19966 line |
| 45146 | DCBLD1 | NM_173674.1 | 604 | aacgagctagccattatttgaag | 318810 | - | see also 10108 line |
| 45147 | DCBLD1 | NM_173674.1 | 789 | cgcaaagggatcagtcgatatga | 318818 | - | see also 10108 line |
| 45148 | DCBLD1 | NM_173674.1 | 600 | ttggaacgagctagccattattt | 318808 | - | see also 10108 line |
| 45149 | DSCAM | NM_001389.2 | 5586 | ctcacacggtccattaccaatcg | 318968 | - | see also 955 line |
| 45150 | DSCAM | NM_001389.2 | 4448 | aacgggacacccagtctagtaac | 318941 | - | see also 955 line |
| 45151 | EFNB1 | NM_004429.3 | 937 | ttggtgatctatccgaaaattgg | 318983 | - | see also 19970 line |
| 45152 | EFNB1 | NM_004429.3 | 1076 | acgtgttggtcacctgcaatagg | 318984 | - | see also 19970 line |
| 45153 | ESDN | NM_080927.2 | 1811 | aaggtcgtgccccaaaatttacg | 319031 | - | see also 9542 line |
| 45154 | ESDN | NM_080927.2 | 1270 | atcgcggatcctcaaataacagc | 319015 | - | see also 9542 line |
| 45155 | IGSF1 | NM_001555.1 | 2453 | tggtgcccgagtgactttcaatt | 319107 | - | see also 1062 line |
| 45156 | IGSF1 | NM_001555.1 | 1538 | tcgcgtagagacacatcctaaca | 319092 | - | see also 1062 line |
| 45157 | L1CAM | NM_000425.2 | 982 | cccggcatgcgtactatgtcacc | 319150 | - | see also 19973 line |
| 45158 | L1CAM | NM_000425.2 | 3512 | ggcccgaccgatgaaagatgaga | 319170 | - | see also 19973 line |
| 45159 | LSAMP | NM_002338.1 | 755 | accgggatgacactaggataaat | 319195 | - | see also 1513 line |

Figure 46

| | | | | | | | |
|---|---|---|---|---|---|---|---|
| 45160 | MCAM | NM_006500.1 | 1425 | aacggcacggcaagtgaacaaga | 319210 | - | see also 19975 line |
| 45161 | MCAM | NM_006500.1 | 1852 | tagttgaagttaagtcagataag | 319216 | - | see also 19975 line |
| 45162 | MFGE8 | NM_005928.1 | 164 | gttggtttatgcgaggagatttcc | 319218 | - | see also 19976 line |
| 45163 | MFGE8 | NM_005928.1 | 555 | tggacacgaattcgatttcatcc | 319222 | - | see also 19976 line |
| 45164 | NINJ1 | NM_004148.2 | 284 | cccagcttcgccttctatgtgc | 319230 | - | see also 19977 line |
| 45165 | NRXN1 | NM_004801.2 | 3587 | gttggacaaatcacgtataagtgg | 319317 | - | see also 3336 line |
| 45166 | NRXN1 | NM_004801.2 | 3585 | tggttggacaaatcacgtataagt | 319316 | - | see also 3336 line |
| 45167 | NRXN2 | NM_015080.2 | 4079 | gacgagcccaacgccatagtaag | 319370 | - | see also 19979 line |
| 45168 | NRXN2 | NM_015080.2 | 4960 | caeggacgacgaggacttttaca | 319385 | - | see also 19979 line |
| 45169 | OMD | NM_005014.1 | 1131 | aaccaataagctcatacatctc | 319419 | - | see also 3510 line |
| 45170 | OMD | NM_005014.1 | 1198 | aagcactaatggtcaaacaatac | 319421 | - | see also 3510 line |
| 45171 | OMG | NM_002544.2 | 2340 | caccatgaccctaagtatcact | 319470 | - | see also 1639 line |
| 45172 | OMG | NM_002544.2 | 2342 | cccatgaccctaagtatcactag | 319471 | - | see also 1639 line |
| 45173 | PUNC | NM_004884.1 | 1598 | cccacggagctgagtatcagg | 319479 | - | see also 19982 line |
| 45174 | PUNC | NM_004884.1 | 2196 | agggtcctctgtgtaaagatgt | 319482 | - | see also 19982 line |
| 45175 | SRPX | NM_006307.2 | 916 | aggcgacggtgataattatggagc | 319510 | - | see also 19983 line |
| 45176 | SRPX | NM_006307.2 | 910 | tgctccagcgacggtgataatta | 319507 | - | see also 19983 line |
| 45177 | SRPX | NM_006307.2 | 912 | ctccagcgacggtgataattatg | 319508 | - | see also 19983 line |
| 45178 | TROAP | NM_005480.2 | 2248 | aggcctcaaatgtgtttaacc | 319534 | - | see also 19984 line |
| 45179 | TROAP | NM_005480.2 | 1151 | gcgtctcaccgttcaacctaaaa | 319524 | - | see also 19984 line |
| 45180 | DAP4 | NM_014902.2 | 2093 | gcctagtgcgctataagaagacc | 319544 | - | see also 19985 line |
| 45181 | DAP4 | NM_014902.2 | 2964 | gggcagtgcccagctactgatgt | 319550 | - | see also 19985 line |
| 45182 | EFNA5 | NM_001962.1 | 798 | tcgtgtttcgatgttaacgaca | 319570 | - | see also 1241 line |
| 45183 | EFNA5 | NM_001962.1 | 883 | ggcgagaacgcggcacaaacacc | 319573 | - | see also 1241 line |
| 45184 | EFNB2 | NM_004093.2 | 97 | ttccaaatcgatagtttagagc | 319582 | - | see also 2762 line |
| 45185 | IFRD1 | NM_001550.1 | 1293 | tggtcctgaacgcatgtatattg | 319625 | - | see also 1057 line |
| 45186 | IFRD1 | NM_001550.1 | 750 | ttgtgatgggtcagctagtatgc | 319614 | - | see also 1057 line |
| 45187 | SPAG6 | NM_012443.2 | 252 | tgcagaaacgcgggtgaatgtct | 319636 | - | see also 19989 line |
| 45188 | SPAG6 | NM_012443.2 | 702 | ctgcttcggccctcagtgatatt | 319650 | - | see also 19989 line |
| 45189 | MLF1 | NM_022443.2 | 588 | atgaccgagctcatgtcattaaa | 319686 | - | see also 19990 line |
| 45190 | MLF1 | NM_022443.2 | 162 | ttcttgcacaccgagaaaaatatg | 319676 | - | see also 19990 line |
| 45191 | CRISP1 | NM_001131.2 | 576 | cacctcgatatcctcacgtttgt | 319718 | - | see also 19991 line |
| 45192 | CRISP1 | NM_001131.2 | 585 | atctctacgtttgtcactattgt | 319720 | - | see also 19991 line |
| 45193 | CRISP1 | NM_001131.2 | 434 | tggtacagtgagtctacaagttt | 319712 | - | see also 19991 line |
| 45194 | NP25 | NM_013259.1 | 549 | ttcgccagggacagaacgtaata | 319736 | - | see also 19992 line |
| 45195 | NP25 | NM_013259.1 | 551 | cgccagggacagaacgtaatagg | 319737 | - | see also 19992 line |
| 45196 | PCP4 | NM_006198.1 | 98 | gacaagacatctggtgaaaatga | 319739 | - | - |

Figure 46

| | | | | | | | |
|---|---|---|---|---|---|---|---|
| 45197 | PCP4 | NM_006198.1 | 199 | gtctcagttcagaaattccaga | 319740 | - | see also 45196 line |
| 45198 | PNMA1 | NM_006029.3 | 1263 | ggcggttgatggagagtcttaga | 319757 | - | see also 19993 line |
| 45199 | PNMA1 | NM_006029.3 | 807 | accgaatgcttgggagaatgttc | 319742 | - | see also 19993 line |
| 45200 | RNF103 | NM_005667.2 | 1241 | aagcatccgaatcggtttctagt | 319769 | - | see also 3932 line |
| 45201 | RNF103 | NM_005667.2 | 1475 | tccgatccacatccattatgtct | 319776 | - | see also 3932 line |
| 45202 | C4orf6 | NM_005750.1 | 306 | gacactatggacggaaatccagg | 319841 | - | - |
| 45203 | C4orf6 | NM_005750.1 | 263 | aaggaaggaaacacgcaaaaattt | 319837 | - | see also 45202 line |
| 45204 | C4orf6 | NM_005750.1 | 264 | aggaaggaaacacgcaaaaatttt | 319838 | - | see also 45202 line |
| 45205 | CLN8 | NM_018941.2 | 948 | ggctctgcttacgttaatcatta | 319855 | - | see also 19994 line |
| 45206 | CLN8 | NM_018941.2 | 957 | tacgctaatcattaatccatatt | 319858 | - | see also 19994 line |
| 45207 | DAB1 | NM_021080.3 | 793 | tccggtacaaagccaaattgatc | 319862 | - | see also 8080 line |
| 45208 | DAB1 | NM_021080.3 | 810 | ttgatcgggattgatgaagttc | 319865 | - | see also 8080 line |
| 45209 | FCMD | NM_006731.1 | 952 | ctgcaactagcagcgaaaacatt | 319918 | - | see also 4750 line |
| 45210 | FCMD | NM_006731.1 | 618 | tgcgatccacttgagtcttc | 319904 | - | see also 4750 line |
| 45211 | FCMD | NM_006731.1 | 667 | cacggccacttgagcttaaaga | 319908 | - | see also 4750 line |
| 45212 | MAB21L2 | NM_006439.3 | 1370 | agcgctatgtggtgcaaatcact | 319943 | - | see also 4504 line |
| 45213 | MAB21L2 | NM_006439.3 | 1673 | ccggccagccgctcaacaactac | 319944 | - | see also 4504 line |
| 45214 | MOBP | NM_006501.1 | 130 | ateggaaatacagcatcgtaag | 319949 | - | see also 19998 line |
| 45215 | MOBP | NM_006501.1 | 91 | cgccgttcaccttcctcaattcc | 319948 | - | see also 19998 line |
| 45216 | NEURL | NM_004210.3 | 165 | accccagaacctcaaagactct | 319951 | - | see also 19999 line |
| 45217 | NUMBL | NM_004756.2 | 616 | tggtcgaccagaccatcgaaaag | 319956 | - | see also 20000 line |
| 45218 | NUMBL | NM_004756.2 | 583 | tccgagtgtggacgacaaaacc | 319954 | - | see also 20000 line |
| 45219 | SCRG1 | NM_007281.1 | 548 | agctgacctgacacagattgatg | 319980 | - | - |
| 45220 | SCRG1 | NM_007281.1 | 484 | atcgcctctgctacagaaag | 319979 | - | see also 45219 line |
| 45221 | SEMA3A | NM_006080.1 | 1463 | ttcgggaaccgactgctatttca | 320021 | - | see also 4222 line |
| 45222 | SEMA3A | NM_006080.1 | 1550 | tccctttacaccggtgtgatatt | 320026 | - | see also 4222 line |
| 45223 | SEMA6B | NM_020241.2 | 681 | accctgccaccgtgaaacatga | 320053 | - | see also 20002 line |
| 45224 | SEMA6B | NM_020241.2 | 1202 | cagcgccttgccggatgacatcc | 320059 | - | see also 20002 line |
| 45225 | SEMA6C | NM_030913.2 | 954 | cgcgtagcccgagtatgtaaacg | 320079 | - | see also 20003 line |
| 45226 | SEMA6C | NM_030913.2 | 952 | ccgcgtagcccgagtatgtaaaa | 320077 | - | see also 20003 line |
| 45227 | EVC | NM_014556.2 | 1100 | tactctgaacagctaatcgataa | 320087 | - | see also 20004 line |
| 45228 | EVC | NM_014556.2 | 1109 | cagctaatcgataatatggaagc | 320090 | - | see also 20004 line |
| 45229 | CSRP3 | NM_003476.1 | 195 | ggctcatgagtcggagatctact | 320111 | - | see also 20005 line |
| 45230 | CSRP3 | NM_003476.1 | 197 | ctcatgagtcggagatctactgc | 320112 | - | see also 20005 line |
| 45231 | VAMP5 | NM_006634.2 | 131 | gaccggaaattatgcgtaacaact | 320118 | - | see also 20006 line |
| 45232 | CAV3 | NM_001234.3 | 370 | ggcggttggtgcatgcattaaga | 320125 | - | see also 20007 line |
| 45233 | CAV3 | NM_001234.3 | 144 | accgagaccccaagaaacattaac | 320123 | - | see also 20007 line |

Figure 46

| 45234 | CAV3 | NM_001234.3 | 143 | aaccgagaccccaagaacattaa | 320122 | - | see also 20007 line | | | |
| 45235 | FHL3 | NM_004468.1 | 77 | ccctgtatggacgcaagtacatc | 320126 | - | - | | - | - |
| 45236 | FHL3 | NM_004468.1 | 373 | tcccggaagctggaatatggagg | 320128 | - | see also 45235 line | | | |
| 45237 | FHL3 | NM_004468.1 | 187 | gactcgagggagctgttctatga | 320127 | - | see also 45235 line | | | |
| 45238 | SGCB | NM_000232.2 | 300 | accaaatggctgtgatagtatgg | 320138 | - | see also 171 line | | | |
| 45239 | SGCB | NM_000232.2 | 341 | tgcttcgatttaagcaagtatct | 320141 | - | see also 171 line | | | |
| 45240 | SGCB | NM_000232.2 | 336 | tggcctgcttcgatttaagcaag | 320140 | - | see also 171 line | | | |
| 45241 | SGCD | NM_000337.3 | 612 | atccagtcccgaccaggtaatgc | 320165 | - | see also 20009 line | | | |
| 45242 | SGCD | NM_000337.3 | 716 | tccaaaagccgtagaagcttatg | 320169 | - | see also 20009 line | | | |
| 45243 | SGCD | NM_000337.3 | 723 | gccgtagaagcttatggtaaaaa | 320171 | - | see also 20009 line | | | |
| 45244 | SGCG | NM_000231.1 | 712 | ccccactcggagtctaagcatgg | 320207 | - | see also 20010 line | | | |
| 45245 | SGCG | NM_000231.1 | 684 | acccgtttcaagaccttagatta | 320203 | - | see also 20010 line | | | |
| 45246 | SGCG | NM_000231.1 | 260 | atcctcgttgtgaatttagctct | 320188 | - | see also 20010 line | | | |
| 45247 | KRTHA2 | NM_002278.2 | 792 | agcttggggaccgccttaacatc | 320217 | - | see also 20011 line | | | |
| 45248 | KRTHA2 | NM_002278.2 | 929 | cagatggaggagcttaaccaaca | 320219 | - | see also 20011 line | | | |
| 45249 | SCEL | NM_003843.2 | 2082 | ctgtgaaccttgctactctaaaa | 320263 | - | see also 20012 line | | | |
| 45250 | SCEL | NM_003843.2 | 2036 | atctacaagcgggtgatagtatt | 320262 | - | see also 20012 line | | | |
| 45251 | TNFAIP2 | NM_006291.2 | 1978 | tggccatcaaggggaacctatcc | 320271 | - | see also 20013 line | | | |
| 45252 | TNFAIP2 | NM_006291.2 | 1377 | acgaattacagggccaatgttat | 320268 | - | see also 20013 line | | | |
| 45253 | ANKRD7 | NM_019644.1 | 411 | cactattagttgccgttattaac | 320278 | - | see also 20014 line | | | |
| 45254 | ANKRD7 | NM_019644.1 | 285 | atctgagggatattcgttataat | 320274 | - | see also 20014 line | | | |
| 45255 | MEA | NM_014623.1 | 68 | gcccggatggcaacagtagttct | 320283 | - | see also 20015 line | | | |
| 45256 | MEA | NM_014623.1 | 71 | cggatggcaacagtagttctagg | 320285 | - | see also 20015 line | | | |
| 45257 | CCNI | NM_006835.2 | 1156 | ggccatggttagtctggaaatgg | 320306 | - | see also 20016 line | | | |
| 45258 | CCNI | NM_006835.2 | 699 | tggccaaactcaagtaccaattc | 320292 | - | see also 20016 line | | | |
| 45259 | FSHPRH1 | NM_006733.1 | 1912 | tgcgcttggagagcaacaatact | 320342 | - | see also 4753 line | | | |
| 45260 | FSHPRH1 | NM_006733.1 | 317 | ttcacaaggtagtagtagttttc | 320312 | - | see also 4753 line | | | |
| 45261 | SPAG9 | NM_003971.3 | 2331 | ttcggctacaaaagttcttatta | 320435 | - | see also 2714 line | | | |
| 45262 | SPAG9 | NM_003971.3 | 1058 | ttcaagcaatcatcgaatctact | 320396 | - | see also 2714 line | | | |
| 45263 | SPAG9 | NM_003971.3 | 2322 | cagcactcattcggctacaaaag | 320432 | - | see also 2714 line | | | |
| 45264 | D8S2298E | NM_005671.1 | 82 | cggcgtgggatccggatatagg | 320481 | - | see also 3942 line | | | |
| 45265 | D8S2298E | NM_005671.1 | 359 | aggagcgcttttatcaaatgacg | 320489 | - | see also 3942 line | | | |
| 45266 | SPAG1 | NM_003114.3 | 2584 | ttccgatagccaagcctaataat | 320550 | - | see also 20020 line | | | |
| 45267 | SPAG1 | NM_003114.3 | 1030 | tgcgtcgtgctactacatataaa | 320522 | - | see also 20020 line | | | |
| 45268 | VCY2 | NM_004678.1 | 425 | gggcatcatgacatattgcttga | 320559 | - | see also 20021 line | | | |
| 45269 | VCY2 | NM_004678.1 | 472 | ttctgcataggttgctaaaaaat | 320561 | - | see also 20021 line | | | |
| 45270 | XKRY | NM_004677.1 | 1096 | gacacgaggtgggacatacaatc | 320573 | - | see also 20022 line | | | |

Figure 46

| | | | | | | | | | |
|---|---|---|---|---|---|---|---|---|---|
| 45271 | XKRY | NM_004677.1 | 812 | ggcattcgttggtgattatctca | 320566 | - | see also 20022 line | | |
| 45272 | RQCD1 | NM_005444.1 | 384 | aacacgtccctttgagtatctcc | 320585 | - | see also 3792 line | | |
| 45273 | RQCD1 | NM_005444.1 | 784 | ttcgcccaggtgctaaaagatga | 320597 | - | see also 3792 line | | |
| 45274 | RQCD1 | NM_005444.1 | 492 | ccctttatgtttgcgaattatgg | 320590 | - | see also 3792 line | | |
| 45275 | FLJ10292 | NM_018048.2 | 138 | ggccggacggaaagcttagatat | 320599 | - | see also 20024 line | | |
| 45276 | FLJ10292 | NM_018048.2 | 139 | gccggacggaaagcttagatatg | 320600 | - | see also 20024 line | | |
| 45277 | SEMG1 | NM_003007.2 | 1384 | aacgaccgaaacccattatttac | 320613 | - | see also 20025 line | | |
| 45278 | SEMG1 | NM_003007.2 | 1387 | gaccgaaacccattatttacata | 320615 | - | see also 20025 line | | |
| 45279 | ACRV1 | NM_001612.4 | 1004 | cccatggaacgaggatgcaaatt | 320628 | - | see also 1069 line | | |
| 45280 | ACRV1 | NM_001612.4 | 349 | ggctccatagatcatcaaacttc | 320620 | - | see also 1069 line | | |
| 45281 | AMN | NM_030943.1 | 72 | cccaggcggtctccaaactctgg | 320637 | - | see also 20027 line | | |
| 45282 | AMN | NM_030943.1 | 1324 | cagcaccagccacagttacttcg | 320639 | - | see also 20027 line | | |
| 45283 | CLPTM1 | NM_001294.1 | 823 | gccgtgagcggtgactactatcc | 320648 | - | see also 20028 line | | |
| 45284 | CLPTM1 | NM_001294.1 | 822 | cgccgtgagcggtgactactatc | 320647 | - | see also 20028 line | | |
| 45285 | DKK2 | NM_014421.1 | 1069 | cccagtacccgctgcaataatgg | 320663 | - | see also 20029 line | | |
| 45286 | DKK2 | NM_014421.1 | 1163 | atcgaaaccacggtcattactca | 320664 | - | see also 20029 line | | |
| 45287 | DKK2 | NM_014421.1 | 1068 | ccccagtacccgctgcaataatg | 320662 | - | see also 20029 line | | |
| 45288 | DKK3 | NM_013253.3 | 1132 | gaggtccccgatgagtatgaagt | 320682 | - | see also 20030 line | | |
| 45289 | DKK3 | NM_013253.3 | 524 | cagacacgaaggttggaaataat | 320676 | - | see also 20030 line | | |
| 45290 | GTL3 | NM_013242.1 | 678 | ggcgagcatacggcaccaattac | 320690 | - | see also 5357 line | | |
| 45291 | GTL3 | NM_013242.1 | 558 | gtcgctttcgggcaagtaactac | 320687 | - | see also 5357 line | | |
| 45292 | GTL3 | NM_013242.1 | 556 | tcgtcgctttcgggcaagtaact | 320686 | - | see also 5357 line | | |
| 45293 | ID2 | NM_002166.4 | 275 | acccgatgagcctgctatacaac | 320700 | - | - | - | neuroblastoma、pancreatic cancer |
| 45294 | ID2 | NM_002166.4 | 276 | cccgatgagcctgctatacaaca | 320701 | - | see also 45293 line | | |
| 45295 | LOC56920 | NM_020163.1 | 1927 | cgcaggcttagccgtttcgatgc | 320715 | - | see also 20032 line | | |
| 45296 | LOC56920 | NM_020163.1 | 1343 | atgggacctacgatgtcattttc | 320710 | - | see also 20032 line | | |
| 45297 | NRLN1 | NM_145234.2 | 447 | cagcaagtcttgcgagtacaatg | 320720 | - | see also 20033 line | | |
| 45298 | NRLN1 | NM_145234.2 | 1240 | gtccacgtttggactattcgaaa | 320743 | - | see also 20033 line | | |
| 45299 | NUMB | NM_003744.3 | 361 | ggcgttcgcaccggaaaatgtag | 320749 | - | see also 2564 line | | |
| 45300 | NUMB | NM_003744.3 | 2113 | aagtccaagcagcgtactaatcc | 320779 | - | see also 2564 line | | |
| 45301 | SEMA3F | NM_004186.2 | 454 | aaccgcgagcccctcattataca | 320788 | - | see also 20035 line | | |
| 45302 | SEMA3F | NM_004186.2 | 921 | agccatgcgcacggatcagtaca | 320797 | - | see also 20035 line | | |
| 45303 | SEMA6D | NM_020858.1 | 1014 | tggccagcgatgccgttatttat | 320831 | - | see also 8000 line | | |
| 45304 | SEMA6D | NM_020858.1 | 1157 | atcgctgtcgaacataataattt | 320842 | - | see also 8000 line | | |
| 45305 | SPRY4 | NM_030964.2 | 824 | gcccagactctggtcaactatgg | 320914 | - | signal_transduction | selenium binding | - |
| 45306 | SPRY4 | NM_030964.2 | 210 | ggcccctagaagcctgtttctcc | 320911 | - | see also 45305 line | | |
| 45307 | SPRY4 | NM_030964.2 | 574 | ctctgaccaacggctcttagacc | 320912 | - | see also 45305 line | | |

Figure 46

| | | | | | | | |
|---|---|---|---|---|---|---|---|
| 45308 | TCP11 | NM_018679.2 | 730 | ctctaagtacgttctcaacatga | 320919 | - | see also 20037 line |
| 45309 | TCP11 | NM_018679.2 | 1530 | ttgatcagcggatccatttgttt | 320926 | - | see also 20037 line |
| 45310 | UPK2 | NM_006760.1 | 360 | aacctcgtgccaggaaccaaatt | 320933 | - | see also 20038 line |
| 45311 | UPK2 | NM_006760.1 | 361 | acctcgtgccaggaaccaaattc | 320934 | - | see also 20038 line |
| 45312 | WHSC2 | NM_005663.2 | 536 | ccccggtgaagcattttcagtta | 320941 | - | see also 20039 line |
| 45313 | WHSC2 | NM_005663.2 | 1608 | gacgcgcttcaagaagtacaagc | 320948 | - | see also 20039 line |
| 45314 | WHSC2 | NM_005663.2 | 1601 | gccagtggacgcgcttcaagaag | 320946 | - | see also 20039 line |
| 45315 | DOCK9 | NM_015296.1 | 318 | gacagggtcgatacatatgctca | 320958 | - | see also 20040 line |
| 45316 | DOCK9 | NM_015296.1 | 2723 | aacgtgactcgggtcattattca | 321030 | - | see also 20040 line |
| 45317 | HCP5 | NM_006674.1 | 630 | ctcctggggatcaggatctatta | 321125 | - | - \| - \| - |
| 45318 | HCP5 | NM_006674.1 | 440 | tacctggaaatgcgactgaaatc | 321123 | - | see also 45317 line |
| 45319 | HCP5 | NM_006674.1 | 441 | acctggaaatgcgactgaaatct | 321124 | - | see also 45317 line |
| 45320 | MGC10471 | NM_030818.2 | 354 | tccgattcgaaatgccatataac | 321128 | - | see also 20041 line |
| 45321 | MGC10471 | NM_030818.2 | 344 | atcctcatcatccgattcgaaat | 321127 | - | see also 20041 line |
| 45322 | NDP52 | NM_005831.3 | 185 | ggggacgtcacatgtcattatac | 321136 | - | see also 20042 line |
| 45323 | NDP52 | NM_005831.3 | 226 | ccctcgtcgaaaggattggattg | 321139 | - | see also 20042 line |
| 45324 | ALS2CR12 | NM_139163.1 | 715 | tacgtatcaggacagtatttatg | 321188 | - | see also 20043 line |
| 45325 | ALS2CR12 | NM_139163.1 | 712 | tcgtacgtatcaggacagtattt | 321187 | - | see also 20043 line |
| 45326 | ALS2CR12 | NM_139163.1 | 320 | cggaatcgggaacagatttctgt | 321180 | - | see also 20043 line |
| 45327 | ANGPTL3 | NM_014495.1 | 1089 | aaccaactatacgctacatctag | 321231 | - | see also 20044 line |
| 45328 | ANGPTL3 | NM_014495.1 | 1093 | aactatacgctacatctagttgc | 321233 | - | see also 20044 line |
| 45329 | ANGPTL3 | NM_014495.1 | 564 | gaccgtggaagaccaatataaac | 321215 | - | see also 20044 line |
| 45330 | ANGPTL4 | NM_016109.2 | 300 | tgcgtcctgggacgagatgaatg | 321240 | - | see also 20045 line |
| 45331 | ANGPTL4 | NM_016109.2 | 1196 | acctccgcagggacaagaactgc | 321245 | - | see also 20045 line |
| 45332 | BICD2 | NM_015250.1 | 2310 | caccaggtgtgacgagtacatta | 321256 | - | see also 6291 line |
| 45333 | BICD2 | NM_015250.1 | 2311 | accaggtgtgacgagtacattac | 321257 | - | see also 6291 line |
| 45334 | C14orf102 | NM_017970.2 | 1313 | cagtttagtaccttttcgtatatc | 321280 | - | see also 7185 line |
| 45335 | C14orf102 | NM_017970.2 | 1540 | acccgacagcgtgaaagatctgc | 321285 | - | see also 7185 line |
| 45336 | C14orf102 | NM_017970.2 | 3220 | tcctgagaccggcttaatgcatc | 321314 | - | see also 7185 line |
| 45337 | C14orf44 | NM_152445.1 | 1668 | accggaacaatgaccgtaaaaga | 321338 | - | see also 9868 line |
| 45338 | C14orf44 | NM_152445.1 | 144 | cggaggggagccgtcagatattt | 321319 | - | see also 9868 line |
| 45339 | C20orf11 | NM_017896.2 | 196 | cccgatgaaatcacgaaagatga | 321346 | - | see also 20049 line |
| 45340 | C20orf11 | NM_017896.2 | 706 | aaccaagctgtgctagattatga | 321352 | - | see also 20049 line |
| 45341 | C20orf11 | NM_017896.2 | 379 | atcaagatccgggagatgatact | 321347 | - | see also 20049 line |
| 45342 | C20orf96 | NM_153269.1 | 197 | ttccaggttccggattatgttcc | 321356 | - | see also 20050 line |

Figure 46

| | | | | | | | |
|---|---|---|---|---|---|---|---|
| 45343 | C20orf96 | NM_153269.1 | 1126 | accccgagaggtcatatttgagg | 321367 | - | see also 20050 line |
| 45344 | C8orf13 | NM_053279.1 | 1105 | tccacaccactcaagcttattgg | 321374 | - | see also 20051 line |
| 45345 | C8orf13 | NM_053279.1 | 1104 | ctccacaccactcaagcttattg | 321373 | - | see also 20051 line |
| 45346 | DACT1 | NM_016651.4 | 2102 | gtcctcggccgagatttcctacg | 321402 | - | see also 20052 line |
| 45347 | DACT1 | NM_016651.4 | 1261 | tgccctctggcgggataccttct | 321390 | - | see also 20052 line |
| 45348 | DACT1 | NM_016651.4 | 2051 | ggcggtggtggccaaacctaagc | 321401 | - | see also 20052 line |
| 45349 | FLJ20366 | NM_017786.1 | 793 | tacatgtcttgcggtgaaaatca | 321415 | - | see also 20053 line |
| 45350 | FLJ20366 | NM_017786.1 | 1941 | aacggatcctgtgtataacatcg | 321435 | - | see also 20053 line |
| 45351 | GCC1 | NM_024523.5 | 1838 | gagaaggctactgcactctatta | 321447 | - | see also 8625 line |
| 45352 | GCC2 | NM_014635.3 | 4317 | acgaggtccttcgaaatagcttc | 321554 | - | see also 20054 line |
| 45353 | GCC2 | NM_014635.3 | 1515 | gtcaagagttcgaatcaatgaag | 321488 | - | see also 20054 line |
| 45354 | GOLGA1 | NM_002077.1 | 2365 | ggcgccttccgtcacgaataaca | 321588 | - | see also 1341 line |
| 45355 | GOLGA1 | NM_002077.1 | 2459 | cgcgaatccgaggcttttcatct | 321593 | - | see also 1341 line |
| 45356 | GOLGA2 | NM_004486.3 | 306 | agcatggcggcatctcagaatca | 321597 | - | see also 20056 line |
| 45357 | GOLGA2 | NM_004486.3 | 2976 | taccgggctgacgagaatgatga | 321614 | - | see also 20056 line |
| 45358 | GOLGA2 | NM_004486.3 | 547 | aactcaatatcacgatagagaaa | 321606 | - | see also 20056 line |
| 45359 | GOLGA5 | NM_005113.2 | 2269 | tagcgcgagtttttgtaattata | 321692 | - | see also 3587 line |
| 45360 | GOLGA5 | NM_005113.2 | 2126 | aggcactcgtctgcgaaatgttc | 321681 | - | see also 3587 line |
| 45361 | GOLGIN-67 | NM_015003.3 | 1037 | gtccatacgggagcagatactgc | 321696 | - | see also 20058 line |
| 45362 | K6IRS2 | NM_080747.1 | 1368 | atgtctggcgaatatccaaattc | 321699 | - | see also 20059 line |
| 45363 | K6IRS2 | NM_080747.1 | 1534 | aggatccccttgccaaaacctcg | 321703 | - | see also 20059 line |
| 45364 | KIAA0373 | NM_014684.1 | 3695 | atcgttgatcgacatactagaga | 321791 | - | see also 20060 line |
| 45365 | KIAA0373 | NM_014684.1 | 4387 | tcctcgagattctgttgtagaag | 321810 | - | see also 20060 line |
| 45366 | KRT23 | NM_015515.3 | 1801 | gaccatcaacggaagattagttc | 321848 | - | see also 20061 line |
| 45367 | KRT23 | NM_015515.3 | 1609 | gacgcagctacgccatgaactgg | 321846 | - | see also 20061 line |
| 45368 | KRT23 | NM_015515.3 | 1802 | accatcaacggaagattagttct | 321849 | - | see also 20061 line |
| 45369 | KRT24 | NM_019016.1 | 502 | gaccgcttggccaattacctaga | 321853 | - | see also 20062 line |
| 45370 | KRT24 | NM_019016.1 | 946 | gaggtgaccgtagaaatgaatgc | 321857 | - | see also 20062 line |
| 45371 | KRT6IRS | NM_033448.1 | 768 | tgctgcttacgccaataaggtgg | 321871 | - | see also 20063 line |
| 45372 | KRT6IRS | NM_033448.1 | 1535 | ggggcagtgccaacgattacaaa | 321876 | - | see also 20063 line |
| 45373 | MCRS1 | NM_006337.3 | 1501 | gcctgcgattcgtcttccttatc | 321886 | - | see also 20065 line |
| 45374 | MCRS1 | NM_006337.3 | 1504 | tgcgattcgtcttccttatcaac | 321887 | - | see also 20065 line |
| 45375 | MGC33295 | NM_152505.2 | 725 | tgctatggctcatcgaatactct | 321896 | - | see also 20066 line |
| 45376 | MGC33295 | NM_152505.2 | 2031 | gggtatgagccctcatttggtaa | 321918 | - | see also 20066 line |

Figure 46

| | | | | | | | |
|---|---|---|---|---|---|---|---|
| 45377 | MLPH | NM_024101.4 | 1048 | tgcactcggtcgaatgtcatca | 321929 | - | see also 20067 line |
| 45378 | MLPH | NM_024101.4 | 1912 | cccaacgcgaggaaggaatgg | 321940 | - | see also 20067 line |
| 45379 | OLFM2 | NM_058164.1 | 1202 | aaggtctacttcgcctatttac | 321946 | - | see also 20068 line |
| 45380 | OLFM2 | NM_058164.1 | 1203 | aggtctacttcgcctatttacc | 321947 | - | see also 20068 line |
| 45381 | PCNT2 | NM_006031.3 | 5692 | gacgattgccgagagaaatttag | 321996 | - | see also 20069 line |
| 45382 | PCNT2 | NM_006031.3 | 8856 | agcgtcaggtgacttgcataag | 322016 | - | see also 20069 line |
| 45383 | PES1 | NM_014303.2 | 256 | cccgaaacgtttacettatcaaa | 322052 | - | see also 20070 line |
| 45384 | PES1 | NM_014303.2 | 251 | agcagcccgaacgttttacetta | 322050 | - | see also 20070 line |
| 45385 | PES1 | NM_014303.2 | 248 | tacagcagcccgaacgttttacc | 322049 | - | see also 20070 line |
| 45386 | PPP1R16A | NM_032902.4 | 1631 | ctcccagtgcggcatctatact | 322064 | - | see also 20071 line |
| 45387 | PPP1R16A | NM_032902.4 | 1633 | cccagtgcggcatctatactcc | 322065 | - | see also 20071 line |
| 45388 | ProSAPiP1 | NM_014731.2 | 2778 | ggcggatgtgtcgcagaagttga | 322071 | - | see also 20072 line |
| 45389 | ProSAPiP1 | NM_014731.2 | 3386 | ccgcctcgagcgcattgagtcc | 322072 | - | see also 20072 line |
| 45390 | PSTPIP2 | NM_024430.2 | 258 | gccgtgtgacagtctgaaatca | 322074 | - | see also 20073 line |
| 45391 | PSTPIP2 | NM_024430.2 | 452 | aacaaaagagcttacaattcaag | 322076 | - | see also 20073 line |
| 45392 | SAV1 | NM_021818.2 | 1073 | atccgaatttgaaccattatg | 322085 | - | see also 8177 line |
| 45393 | SAV1 | NM_021818.2 | 1066 | ttgagtcatccgaatttgaacc | 322084 | - | see also 8177 line |
| 45394 | SH3KBP1 | NM_031892.1 | 1246 | ccgataacttcgtgaagttact | 322112 | - | see also 9046 line |
| 45395 | SH3KBP1 | NM_031892.1 | 1003 | ctgccggtagaaaagactattgg | 322107 | - | see also 9046 line |
| 45396 | SPAG4 | NM_003116.1 | 878 | tgcggaagccgactatgctttg | 322135 | - | see also 20075 line |
| 45397 | SPAG4 | NM_003116.1 | 879 | gcggaagcccgactatgctttga | 322136 | - | see also 20075 line |
| 45398 | SPAG4L | NM_080675.3 | 1207 | cagaaccccctaccctaagagaga | 322152 | - | see also 20076 line |
| 45399 | SPAG4L | NM_080675.3 | 866 | gcggccaggtgaccatccaattg | 322148 | - | see also 20076 line |
| 45400 | SSNA1 | NM_003731.1 | 316 | gactttgctcagcgttctcaaga | 322154 | - | - |
| 45401 | TACC1 | NM_006283.1 | 1690 | aggcaaagtcgcgtttaataacg | 322178 | - | see also 4375 line |
| 45402 | TACC1 | NM_006283.1 | 524 | ccgatccgatcacctttcaagg | 322157 | - | see also 4375 line |
| 45403 | TACC2 | NM_006997.1 | 1953 | caggccttgaccttatgtttga | 322233 | - | see also 4883 line |
| 45404 | TCP10 | NM_004610.1 | 1064 | aaggaacggcgacaaaaccattg | 322248 | - | see also 20079 line |
| 45405 | TCP10 | NM_004610.1 | 1065 | aggaacggcgacaaaaccattgt | 322249 | - | see also 20079 line |
| 45406 | TCP10 | NM_004610.1 | 1036 | ttcctgatgggacaatcgtaagg | 322246 | - | see also 20079 line |
| 45407 | TPD52 | NM_005079.1 | 294 | agcggaaacttggaatcaattct | 322254 | - | see also 20081 line |
| 45408 | TPD52 | NM_005079.1 | 363 | cagcaacatcgtcttacaagaag | 322257 | - | see also 20081 line |
| 45409 | UVRAG | NM_003369.2 | 454 | cacgttggcgaagtctcgatttg | 322272 | - | see also 2273 line |
| 45410 | UVRAG | NM_003369.2 | 483 | tgccagaacgtcttgatacatct | 322276 | - | see also 2273 line |
| 45411 | UVRAG | NM_003369.2 | 1125 | ttcgttgcaggcagttactctct | 322292 | - | see also 2273 line |
| 45412 | VAMP1 | NM_014231.3 | 461 | tgccatcatcgtgggtagttattg | 322317 | - | see also 20083 line |
| 45413 | VAMP1 | NM_014231.3 | 462 | gccatcatcgtgggtagttattgt | 322318 | - | see also 20083 line |

Figure 46

| 45414 | VEZATIN | NM_017599.2 | 1072 | gacggttagccctattactttct | 322354 | - | see also 20085 line | | |
|---|---|---|---|---|---|---|---|---|---|
| 45415 | VEZATIN | NM_017599.2 | 301 | tccgactcgactcattacatacc | 322330 | - | see also 20085 line | | |
| 45416 | ZCWCC1 | NM_014941.1 | 2108 | gagcaaagctcggacattagaag | 322395 | - | see also 20086 line | | |
| 45417 | ZCWCC1 | NM_014941.1 | 2339 | tagccgactgatcaaaatgtatg | 322402 | - | see also 20086 line | | |
| 45418 | ZWINT | NM_007057.2 | 277 | agccgacagaaggcaattgcagc | 322429 | - | - | - | - |
| 45419 | ZWINT | NM_007057.2 | 250 | ctcgaccccttggcttctgaaga | 322428 | - | see also 45418 line | | |
| 45420 | ZWINT | NM_007057.2 | 830 | aacctgctggagatgtaaatttg | 322430 | - | see also 45418 line | | |
| 45421 | DCTN4 | NM_016221.2 | 151 | cacgaggtggactcccattattg | 322436 | - | see also 6725 line | | |
| 45422 | DCTN4 | NM_016221.2 | 37 | cagtcggaccgggttctctatct | 322432 | - | see also 6725 line | | |
| 45423 | EPLIN | NM_016357.1 | 507 | gccctcgttcagggtcgatatcc | 322487 | - | see also 6841 line | | |
| 45424 | EPLIN | NM_016357.1 | 893 | aacctctataaaggatcgaatgg | 322503 | - | see also 6841 line | | |
| 45425 | KRTAP11-1 | NM_175858.2 | 236 | acctgttaccggcgaacttcatg | 322536 | - | see also 20089 line | | |
| 45426 | KRTAP11-1 | NM_175858.2 | 288 | ctcgacaaactacctgtatttcc | 322537 | - | see also 20089 line | | |
| 45427 | KRTAP13-1 | NM_181599.1 | 330 | cagtccctgccagacaacttact | 322538 | - | see also 20090 line | | |
| 45428 | KRTAP7-1 | NM_181606.1 | 31 | ttcccaggataccctattctatg | 322540 | - | - | - | - |
| 45429 | KRTAP7-1 | NM_181606.1 | 32 | tcccaggataccctattctatgg | 322541 | - | see also 45428 line | | |
| 45430 | KRTAP8-1 | NM_175857.2 | 195 | aggagatactcgccatttgctct | 322543 | - | see also 20091 line | | |
| 45431 | MGC45562 | NM_152349.1 | 740 | accctcgattgaggaaaaagtct | 322554 | - | see also 20092 line | | |
| 45432 | MGC45562 | NM_152349.1 | 870 | atgcagtattccctctatcaaac | 322560 | - | see also 20092 line | | |
| 45433 | C14orf31 | NM_152330.2 | 1094 | ttctgcccggaagctcatatact | 322589 | - | see also 20093 line | | |
| 45434 | C14orf31 | NM_152330.2 | 1097 | tgcccggaagctcatatactaca | 322590 | - | see also 20093 line | | |
| 45435 | C20orf42 | NM_017671.2 | 407 | cgcgttgaccatcccaatgaaga | 322609 | - | see also 7090 line | | |
| 45436 | C20orf42 | NM_017671.2 | 1190 | ttcgacttgaatcctaaatatga | 322638 | - | see also 7090 line | | |
| 45437 | CDC42EP4 | NM_012121.3 | 547 | ctcggccctgtttgtcaagaatg | 322671 | - | see also 20095 line | | |
| 45438 | CDC42EP4 | NM_012121.3 | 548 | tcggccctgtttgtcaagaatgc | 322672 | - | see also 20095 line | | |
| 45439 | DOCK2 | NM_004946.1 | 3216 | ggggacatgagacggctaattgg | 322739 | - | see also 3444 line | | |
| 45440 | DOCK2 | NM_004946.1 | 780 | aacaagcaaacggtcataagtga | 322687 | - | see also 3444 line | | |
| 45441 | EMS1 | NM_005231.2 | 1419 | gccctcgagccccgtctatgagg | 322794 | - | see also 20097 line | | |
| 45442 | EMS1 | NM_005231.2 | 478 | cacgaatatcagtcgaaactttc | 322786 | - | see also 20097 line | | |
| 45443 | FARP2 | NM_014808.1 | 916 | tggtccaaggtccgtaaactaag | 322821 | - | see also 6000 line | | |
| 45444 | FARP2 | NM_014808.1 | 1997 | tgcgccagttaaaggagtttacc | 322836 | - | see also 6000 line | | |
| 45445 | FKSG43 | NM_032033.1 | 910 | tgccacaagctgcccttctatgg | 322855 | - | - | - | - |
| 45446 | FKSG44 | NM_031904.2 | 890 | gtgccacgagctgccgttttatg | 322856 | - | - | - | - |

Figure 46

| | | | | | | | |
|---|---|---|---|---|---|---|---|
| 45447 | FKSG44 | NM_031904.2 | 891 | tgccacgagctgccgttttatgg | 322857 | - | see also 45446 line |
| 45448 | FLJ10210 | NM_018027.2 | 2628 | ctcgagctcggagactactacc | 322905 | - | see also 7213 line |
| 45449 | FLJ10210 | NM_018027.2 | 634 | aagtcaggacccgtggttttata | 322864 | - | see also 7213 line |
| 45450 | FLJ10210 | NM_018027.2 | 3346 | agctcgacacgtcaagtgaaat | 322906 | - | see also 7213 line |
| 45451 | FRMD1 | NM_024919.2 | 533 | caggcacgcacctgtactact | 322912 | - | see also 20100 line |
| 45452 | FRMD1 | NM_024919.2 | 330 | atcagagacgcgcagttcttgg | 322909 | - | see also 20100 line |
| 45453 | NICAL | NM_022765.2 | 3100 | accatccaacggcgactaaatga | 322939 | - | see also 20101 line |
| 45454 | NICAL | NM_022765.2 | 1744 | cagttacgagacctgtgatgt | 322927 | - | see also 20101 line |
| 45455 | STOM | NM_004099.4 | 887 | ccctctgcccatagatatgctgc | 322951 | - | see also 2768 line |
| 45456 | STOM | NM_004099.4 | 911 | aggaatcatagggggcaaaacaca | 322952 | - | see also 2768 line |
| 45457 | STOM | NM_004099.4 | 160 | ttggacctgcggatggattttg | 322944 | - | see also 2768 line |
| 45458 | URP2 | NM_031471.4 | 1959 | gccgaattgtacagcgagtatatc | 322963 | - | see also 20102 line |
| 45459 | URP2 | NM_031471.4 | 347 | gccgacgcacgcctcttcttgg | 322954 | - | see also 20102 line |
| 45460 | C14orf2 | NM_004894.1 | 172 | atccgggctgctgataaaagaag | 322969 | - | see also 20103 line |
| 45461 | C14orf2 | NM_004894.1 | 101 | agccctactacaccaaagtttac | 322966 | - | see also 20103 line |
| 45462 | HAX1 | NM_006118.2 | 862 | gccggacagagactacagtaacc | 322979 | - | see also 20104 line |
| 45463 | HAX1 | NM_006118.2 | 861 | ggccggacagagagactacagtaac | 322978 | - | see also 20104 line |
| 45464 | TOMM70A | NM_014820.1 | 1535 | tacgcccaggcattaacagatca | 323008 | - | see also 6014 line |
| 45465 | TOMM70A | NM_014820.1 | 1530 | cacttacgcccaggcattaaca | 323007 | - | see also 6014 line |
| 45466 | MRVI1 | NM_006069.2 | 1958 | gtcgaaagcaacgaagtgatga | 323037 | - | see also 20106 line |
| 45467 | MRVI1 | NM_006069.2 | 1996 | atctaaagaggacgtatgagaag | 323039 | - | see also 20106 line |
| 45468 | PSMD1 | NM_002807.2 | 972 | tccactaattacgggtactgttcc | 323072 | - | see also 20107 line |
| 45469 | PSMD1 | NM_002807.2 | 1530 | aaegccagccaatgatatcgttag | 323095 | - | see also 20107 line |
| 45470 | PSMD10 | NM_002814.2 | 739 | gttgcctgggtttaatacttcaag | 323140 | - | see also 20108 line |
| 45471 | PSMD10 | NM_002814.2 | 104 | ggggtgttgtctaacctaatgg | 323133 | - | see also 20108 line |
| 45472 | PSMD12 | NM_002816.2 | 269 | atcgacatccgtatcttagttg | 323142 | - | see also 1833 line |
| 45473 | PSMD12 | NM_002816.2 | 512 | tgcgcggactgactaaaacattag | 323155 | - | see also 1833 line |
| 45474 | PSMD12 | NM_002816.2 | 511 | gttgcgcgactgactaacacatta | 323154 | - | see also 1833 line |
| 45475 | PSMD2 | NM_002808.3 | 645 | ttgcgacctgcttatggaaattg | 323201 | - | see also 1824 line |
| 45476 | ADFP | NM_001122.2 | 1092 | cacattgagtcacgtactcttgc | 323251 | - | see also 20110 line |
| 45477 | ADFP | NM_001122.2 | 390 | aagctagacgcgcaaattgcagt | 323241 | - | see also 20110 line |
| 45478 | JPH3 | NM_020655.2 | 2463 | atcggagtcgccattctgttat | 323263 | - | see also 20111 line |
| 45479 | JPH3 | NM_020655.2 | 2464 | tcggagtcgccattctgtttatt | 323264 | - | see also 20111 line |
| 45480 | PIGS | NM_033198.2 | 436 | ccctgactcgtgtacgtgatatct | 323269 | - | see also 20112 line |
| 45481 | PIGS | NM_033198.2 | 856 | gggtgaatcccgcgtttgactca | 323274 | - | see also 20112 line |

Figure 46

| 45482 | SART2 | NM_013352.1 | 1559 | ggctctgtacgggccaaagtaca | 323333 | - | see also 5405 line |
|---|---|---|---|---|---|---|---|
| 45483 | SART2 | NM_013352.1 | 1075 | tggctattgcggactcaaattac | 323316 | - | see also 5405 line |
| 45484 | SDF2L1 | NM_022044.1 | 178 | tgcactcgcacgacatcaaatac | 323366 | - | see also 20114 line |
| 45485 | SDF2L1 | NM_022044.1 | 177 | ctgcactcgcacgacatcaaata | 323365 | - | see also 20114 line |
| 45486 | VPREB3 | NM_013378.1 | 282 | tgctgacatccccgatcgattct | 323369 | - | see also 20115 line |
| 45487 | VPREB3 | NM_013378.1 | 362 | ctgaagacgacgcggattactac | 323370 | - | see also 20115 line |
| 45488 | PSMD11 | NM_002815.2 | 1037 | cagaatctgatccgagtcattga | 323382 | - | see also 1832 line |
| 45489 | PSMD11 | NM_002815.2 | 1114 | ggccgacgtggaaaggaaattat | 323391 | - | see also 1832 line |
| 45490 | PSMD13 | NM_002817.2 | 520 | atcggttcacagtcgtttctatg | 323408 | - | see also 1834 line |
| 45491 | PSMD3 | NM_002809.2 | 1354 | ggctgcggcacaacgtgattaag | 323436 | - | see also 1827 line |
| 45492 | PSMD3 | NM_002809.2 | 1032 | tactacacagggcgaatcaaagc | 323428 | - | see also 1827 line |
| 45493 | PSMD4 | NM_002810.1 | 201 | aacaacgtgggccttatcacact | 323446 | - | see also 20119 line |
| 45494 | PSMD4 | NM_002810.1 | 717 | gcccttcgtgtatctatggaaga | 323453 | - | see also 20119 line |
| 45495 | PSMD4 | NM_002810.1 | 358 | accgacaaggcaagaatcacaag | 323448 | - | see also 20119 line |
| 45496 | PSMD5 | NM_005047.2 | 881 | cagatctgtgagcgttatcctat | 323470 | - | see also 20120 line |
| 45497 | PSMD5 | NM_005047.2 | 1211 | ctcttccgtggcattagtagtca | 323479 | - | see also 20120 line |
| 45498 | SART1 | NM_005146.3 | 2167 | acccgacgttaagatcgaatacg | 323500 | - | see also 20121 line |
| 45499 | SART1 | NM_005146.3 | 2166 | aacccgacgttaagatcgaatac | 323499 | - | see also 20121 line |
| 45500 | TGOLN2 | NM_006464.1 | 833 | ttcccggaaagaccattccaagc | 323501 | - | see also 4536 line |
| 45501 | TGOLN2 | NM_006464.1 | 1205 | gagcagccacttctttgcatatc | 323506 | - | see also 4536 line |
| 45502 | GOLPH4 | NM_014498.2 | 1323 | aggattccaagccttcgaaaacc | 323523 | - | see also 5728 line |
| 45503 | GOLPH4 | NM_014498.2 | 1322 | taggattccaagccttcgaaaac | 323522 | - | see also 5728 line |
| 45504 | D4S234E | NM_014392.2 | 259 | gacgcccctcgatgtcaatcagc | 323544 | - | see also 5655 line |
| 45505 | GOLPH2 | NM_016548.2 | 1375 | cagcgtgaaaagcggaatcatac | 323559 | - | see also 6913 line |
| 45506 | GOLPH2 | NM_016548.2 | 1354 | gacaccataaatttacttgatca | 323558 | - | see also 6913 line |
| 45507 | HMP19 | NM_015980.3 | 335 | gtgccgaaaatcgctgaatttac | 323565 | - | see also 20125 line |
| 45508 | HMP19 | NM_015980.3 | 262 | tgctccagaaaaggtgattgtga | 323563 | - | see also 20125 line |
| 45509 | HMP19 | NM_015980.3 | 229 | cactcccttggaggttaatcact | 323562 | - | see also 20125 line |
| 45510 | MAPBPIP | NM_014017.1 | 211 | cgctgccatagccagtaacatct | 323567 | - | see also 5460 line |
| 45511 | ZDHHC3 | NM_016598.1 | 1027 | cacagatgagacgggaaatagaac | 323579 | - | see also 6937 line |
| 45512 | ZDHHC3 | NM_016598.1 | 931 | caccacagtgattctccttatcc | 323578 | - | see also 6937 line |
| 45513 | CD63 | NM_001780.3 | 525 | gggctgctaactacacagattgg | 323587 | - | see also 20127 line |
| 45514 | CD63 | NM_001780.3 | 315 | aggagaactattgtcttatgatc | 323582 | - | see also 20127 line |
| 45515 | LAMP2 | NM_002294.1 | 285 | ttcacagtacgctatgaaactac | 323599 | - | see also 20128 line |
| 45516 | LAMP2 | NM_002294.1 | 289 | cagtacgctatgaaactacaaat | 323600 | - | see also 20128 line |
| 45517 | CD68 | NM_001251.1 | 349 | gccaccactagtcatggaaatgc | 323642 | - | see also 20129 line |
| 45518 | CD68 | NM_001251.1 | 542 | ttcagattcgagtcatgtacaca | 323644 | - | see also 20129 line |

Figure 46

| | | | | | | | |
|---|---|---|---|---|---|---|---|
| 45519 | LAMP1 | NM_005561.2 | 1229 | aaggcgttttcagtcaatatatt | 323672 | - | see also 20130 line |
| 45520 | LAMP1 | NM_005561.2 | 449 | cccagtctcgtgattgcttttgg | 323656 | - | see also 20130 line |
| 45521 | LAPTM5 | NM_006762.1 | 704 | ggcggtgctacagattgatcaag | 323680 | - | see also 20131 line |
| 45522 | LAPTM5 | NM_006762.1 | 147 | ggccatctaccatgtgatcatga | 323675 | - | see also 20131 line |
| 45523 | PEX14 | NM_004565.1 | 93 | gccacggcagtgaagtttctaca | 323681 | - | see also 3105 line |
| 45524 | PEX14 | NM_004565.1 | 95 | cacggcagtgaagtttctacaga | 323682 | - | see also 3105 line |
| 45525 | PXMP4 | NM_007238.3 | 187 | ttccggaacggggctgtctatgg | 323687 | - | - | | - |
| 45526 | PXMP4 | NM_007238.3 | 162 | cgctgcgttggccgtgcttaagg | 323686 | - | see also 45525 line |
| 45527 | PXMP2 | NM_018663.1 | 290 | tgccgtttacgggttcttcttca | 323695 | - | see also 20132 line |
| 45528 | PXMP2 | NM_018663.1 | 539 | gtggacgccactacagttcatca | 323699 | - | see also 20132 line |
| 45529 | SRP72 | NM_006947.2 | 1746 | gccaatgcgagaacgttcttact | 323745 | - | see also 20133 line |
| 45530 | SRP72 | NM_006947.2 | 882 | tgcggaaggagtagagtttaagc | 323720 | - | see also 20133 line |
| 45531 | BRD7 | NM_013263.1 | 1588 | ttggcgttccagttgaagtttt | 323769 | - | see also 5381 line |
| 45532 | BRD7 | NM_013263.1 | 328 | acgcccctgtgagattagacttg | 323753 | - | see also 5381 line |
| 45533 | ICA1 | NM_004968.1 | 210 | atcgatatgctcaagataagtca | 323778 | - | see also 3450 line |
| 45534 | ICA1 | NM_004968.1 | 388 | atcgaaagcaattgtactctatc | 323787 | - | see also 3450 line |
| 45535 | ICA1 | NM_004968.1 | 265 | gacgaagcaggcctttattaaag | 323781 | - | see also 3450 line |
| 45536 | MVP | NM_005115.3 | 946 | gtcggaccggatggcaagaatca | 323822 | - | see also 20137 line |
| 45537 | MVP | NM_005115.3 | 1274 | aggtgcgcgctgtgattggaagc | 323826 | - | see also 20137 line |
| 45538 | NFKBIE | NM_004556.1 | 776 | agctggaagcactcacttacatc | 323835 | - | see also 20138 line |
| 45539 | NFKBIE | NM_004556.1 | 820 | gtccacctggcagtgattcatga | 323836 | - | see also 20138 line |
| 45540 | TUB | NM_003320.3 | 1635 | tgctagcacgctggcagaataag | 323851 | - | see also 20140 line |
| 45541 | TUB | NM_003320.3 | 1639 | agcacgctggcagaataagaaca | 323853 | - | see also 20140 line |
| 45542 | FLJ39369 | NM_152363.2 | 1517 | ccccaaggtgcgtcagatcttgg | 323863 | - | see also 20141 line |
| 45543 | FLJ39369 | NM_152363.2 | 1610 | tacacgggaggcgtgtattgtgg | 323864 | - | see also 20141 line |
| 45544 | LEMD2 | NM_181336.2 | 303 | ccctggggcctacggtgatatcc | 323865 | - | see also 20142 line |
| 45545 | LEMD2 | NM_181336.2 | 945 | cagctcctccgccaagtttgaag | 323872 | - | see also 20142 line |
| 45546 | ACRC | NM_052957.2 | 1740 | tacgcataggctggaataacaag | 323907 | - | see also 20143 line |
| 45547 | ACRC | NM_052957.2 | 250 | ggcgagttgcatccttaatgtcc | 323879 | - | see also 20143 line |
| 45548 | AKIP | NM_017900.1 | 436 | gtcgcggatgcgcctcaaattca | 323921 | - | see also 20144 line |
| 45549 | AKIP | NM_017900.1 | 459 | gtgcaaaaacgtgctgaagatcc | 323923 | - | see also 20144 line |
| 45550 | BARD1 | NM_000465.1 | 221 | tgctcgcgttgtactaacattct | 323929 | - | see also 373 line |
| 45551 | BARD1 | NM_000465.1 | 223 | ctcgcgttgtactaacattctga | 323930 | - | see also 373 line |
| 45552 | BOP1 | NM_015201.3 | 529 | tggatggcaggcgcatctacaag | 324004 | - | see also 6277 line |
| 45553 | BRD4 | NM_014299.1 | 1476 | tggtgctgacgtccgattgatgt | 324019 | - | see also 5607 line |
| 45554 | BRD4 | NM_014299.1 | 1478 | gtgctgacgtccgattgatgttc | 324020 | - | see also 5607 line |
| 45555 | BXDC1 | NM_032194.1 | 23 | gacactctggatcgagtagtaaa | 324035 | - | - | | - |

Figure 46

| | | | | | | | |
|---|---|---|---|---|---|---|---|
| 45556 | BXDC1 | NM_032194.1 | 900 | aagaccacgagaaaaagtcaaaa | 324037 | - | see also 45555 line |
| 45557 | BXDC1 | NM_032194.1 | 876 | agcgacctgcagaaaggataaca | 324036 | - | see also 45555 line |
| 45558 | C5orf6 | NM_016605.1 | 1075 | ttcgttggactttgacaagatga | 324041 | - | see also 20148 line |
| 45559 | C5orf6 | NM_016605.1 | 1071 | ggccttcgttggactttgacaag | 324040 | - | see also 20148 line |
| 45560 | COIL | NM_004645.1 | 1025 | gagtgtgctgcgggtttcttaaa | 324058 | - | see also 3164 line |
| 45561 | COIL | NM_004645.1 | 254 | aacgactgcctcagagttaaatt | 324049 | - | see also 3164 line |
| 45562 | CTNNBL1 | NM_030877.3 | 533 | ggcttgctcggacacgataatac | 324079 | - | see also 8958 line |
| 45563 | CTNNBL1 | NM_030877.3 | 539 | ctcggacacgataatacagatgt | 324081 | - | see also 8958 line |
| 45564 | D21S2056E | NM_003683.3 | 938 | tacgaggcagttgctaacagact | 324113 | - | see also 20151 line |
| 45565 | D21S2056E | NM_003683.3 | 373 | caggctgcgcctggataaattct | 324109 | - | see also 20151 line |
| 45566 | DKFZp761B128 | NM_152437.1 | 1843 | tgccccatgtgtagggaattttt | 324117 | - | see also 20152 line |
| 45567 | DKFZp761B128 | NM_152437.1 | 1872 | gagagcagatctttttatgcatc | 324120 | - | see also 20152 line |
| 45568 | DKFZp761B128 | NM_152437.1 | 1864 | ttctctgagagagcagatctttt | 324118 | - | see also 20152 line |
| 45569 | DNTTIP1 | NM_052951.2 | 170 | tacgaacccttggaacataatga | 324124 | - | see also 20153 line |
| 45570 | DNTTIP1 | NM_052951.2 | 174 | aacccttggaacataatgataaa | 324125 | - | see also 20153 line |
| 45571 | DXS9928E | NM_004699.1 | 407 | aggaagccaagcggaagatctcc | 324139 | - | see also 20154 line |
| 45572 | DXS9928E | NM_004699.1 | 179 | tcgcggaggagaacatcatgaaa | 324138 | - | see also 20154 line |
| 45573 | FAM9A | NM_174951.2 | 1017 | gccgctacttgagcaattactaa | 324145 | - | see also 20155 line |
| 45574 | FAM9A | NM_174951.2 | 1019 | cgctacttgagcaattactaaag | 324147 | - | see also 20155 line |
| 45575 | FAM9A | NM_174951.2 | 1045 | gccaaggacactaaagacaatta | 324150 | - | see also 20155 line |
| 45576 | FAM9C | NM_174901.2 | 573 | gccgaatgtccttgaagagttca | 324153 | - | - | - | - |
| 45577 | FBXL4 | NM_012160.3 | 698 | ttcaagaggacgccacctaattt | 324168 | - | see also 5158 line |
| 45578 | FBXL4 | NM_012160.3 | 701 | aagaggacgccacctaattttca | 324169 | - | see also 5158 line |
| 45579 | FBXL4 | NM_012160.3 | 703 | gaggacgccacctaattttcaga | 324170 | - | see also 5158 line |
| 45580 | FHL2 | NM_001450.2 | 1177 | ttcacagctcgcgatgactttgc | 324234 | - | see also 20157 line |
| 45581 | FHL2 | NM_001450.2 | 1395 | ccccgactgtgggaaagacatct | 324236 | - | see also 20157 line |
| 45582 | FHL2 | NM_001450.2 | 1394 | gccccgactgtgggaaagacatc | 324235 | - | see also 20157 line |
| 45583 | FJX1 | NM_014344.2 | 997 | gaccgacttaatccttttcgact | 324243 | - | see also 20158 line |
| 45584 | FJX1 | NM_014344.2 | 975 | agctggtggacctagtacaatgg | 324240 | - | see also 20158 line |
| 45585 | FLJ14011 | NM_022103.2 | 795 | aagaacaaccttattctacata | 324249 | - | see also 20160 line |
| 45586 | FLJ14011 | NM_022103.2 | 621 | tagtcgaagcttctctcttaaac | 324246 | - | see also 20160 line |
| 45587 | FLJ20079 | NM_017656.1 | 216 | aacttcagaatgggaaatacaac | 324256 | - | - | - | - |
| 45588 | FLJ21628 | NM_030613.1 | 598 | cagtcaaagcatgcatcttattg | 324264 | - | see also 20161 line |
| 45589 | FLJ21628 | NM_030613.1 | 184 | gagctcatcccttcttaagcacc | 324261 | - | see also 20161 line |

Figure 46

| 45590 | FLJ22059 | NM_022752.2 | 147 | aggtgcttatgcaccaaaactcc | 324268 | - | see also 8454 line |
|---|---|---|---|---|---|---|---|
| 45591 | FLJ22059 | NM_022752.2 | 1499 | ctgacccggcaccaacgttttgt | 324271 | - | see also 8454 line |
| 45592 | FLJ30726 | NM_153018.1 | 1332 | aggcggacctctcaccttattgt | 324293 | - | see also 20162 line |
| 45593 | FLJ30726 | NM_153018.1 | 282 | gagtccatggaagaggttataga | 324280 | - | see also 20162 line |
| 45594 | FLJ32053 | NM_152484.2 | 2237 | ctcagtgctcactgcttaattta | 324312 | - | see also 20163 line |
| 45595 | FLJ32053 | NM_152484.2 | 1102 | gtcattaccccctttaagtgtaa | 324306 | - | see also 20163 line |
| 45596 | FLJ36870 | NM_173658.1 | 764 | gtcggagttcgggccttatatca | 324323 | - | see also 20164 line |
| 45597 | FLJ36870 | NM_173658.1 | 766 | cggagttcgggccttatatcaca | 324324 | - | see also 20164 line |
| 45598 | FLJ38002 | NM_152412.1 | 1634 | cccgacgttggggattttcctca | 324353 | - | see also 20165 line |
| 45599 | FLJ38002 | NM_152412.1 | 907 | cagctatgtcctaatagaacatc | 324342 | - | see also 20165 line |
| 45600 | FLJ38451 | NM_175872.3 | 330 | aacgtacacaaccctatcagaac | 324362 | - | see also 20166 line |
| 45601 | FLJ38451 | NM_175872.3 | 1453 | aaccagacaggccttacgaatgc | 324375 | - | see also 20166 line |
| 45602 | FLJ38705 | NM_173832.2 | 548 | ttcgacgcttcatttaaagatga | 324384 | - | see also 20167 line |
| 45603 | FLJ38705 | NM_173832.2 | 366 | agcctgcaggcagaaaaaagaag | 324380 | - | see also 20167 line |
| 45604 | FLJ90764 | NM_173656.1 | 1782 | aacagggctatccgcagttaagc | 324399 | - | see also 20168 line |
| 45605 | FLJ90764 | NM_173656.1 | 1468 | caccgcagttcggtatttcttca | 324396 | - | see also 20168 line |
| 45606 | FLJ90764 | NM_173656.1 | 1465 | ttccaccgcagttcggtatttct | 324395 | - | see also 20168 line |
| 45607 | HIRIP3 | NM_003609.2 | 1769 | tggtgcccatcgaaactacaaga | 324410 | - | see also 20169 line |
| 45608 | HIRIP3 | NM_003609.2 | 716 | ttgtagcgacccggagagaaaaa | 324403 | - | see also 20169 line |
| 45609 | HSPC111 | NM_016391.2 | 903 | gagtaagatcaacgtctataaac | 324421 | - | see also 20170 line |
| 45610 | HSPC111 | NM_016391.2 | 793 | cgggacctcattgactatgtacg | 324420 | - | see also 20170 line |
| 45611 | KIAA0103 | NM_014673.2 | 810 | tagttgggcagctagtcaaataa | 324441 | - | see also 5854 line |
| 45612 | KIAA0924 | NM_014897.1 | 584 | gacgtgcatgctgttaaggaaga | 324448 | - | see also 20172 line |
| 45613 | KIAA0924 | NM_014897.1 | 1103 | aagcacatgaacgttactcatag | 324456 | - | see also 20172 line |
| 45614 | KIAA1190 | NM_145166.2 | 483 | gtccctccatgagcataacaaga | 324479 | - | see also 20173 line |
| 45615 | KIAA1190 | NM_145166.2 | 512 | acggctacgcagagaagaagttc | 324480 | - | see also 20173 line |
| 45616 | KIAA1615 | NM_020951.1 | 1948 | tgccaaccgaaaatttacactgg | 324501 | - | see also 20174 line |
| 45617 | KIAA1615 | NM_020951.1 | 1944 | tacttgccaaccgaaaatttaca | 324500 | - | see also 20174 line |
| 45618 | LOC148213 | NM_138286.1 | 1483 | ttctatcgatcctcaaaacttac | 324504 | | see also 20175 line |
| 45619 | LOC148213 | NM_138286.1 | 421 | aaggtaagacacaccagaaaaaa | 324502 | - | see also 20175 line |
| 45620 | LOC51058 | NM_015911.2 | 747 | ggcggaccttcagcgatatctcc | 324508 | - | - | - | - |
| 45621 | LOC51058 | NM_015911.2 | 523 | caccacgcaccagcaagatcacc | 324507 | - | see also 45620 line |
| 45622 | LOC51058 | NM_015911.2 | 841 | ctcccggagctcgaatctcatcc | 324509 | - | see also 45620 line |
| 45623 | LOC84661 | NM_032574.1 | 425 | gaccaccaaatcccattgaattt | 324514 | - | see also 20176 line |
| 45624 | LOC84661 | NM_032574.1 | 271 | gacaacgttgagagaatagtaga | 324510 | - | see also 20176 line |

Figure 46

| | | | | | | | |
|---|---|---|---|---|---|---|---|
| 45625 | LOC84661 | NM_032574.1 | 274 | aacgttgagagaatagtagaaaa | 324511 | - | see also 20176 line |
| 45626 | LOC90589 | NM_145233.2 | 1178 | tgcctcgagcgttaaaatccatg | 324519 | - | see also 20177 line |
| 45627 | LOC90589 | NM_145233.2 | 1175 | atgtgcctcgagcgttaaaatcc | 324518 | - | see also 20177 line |
| 45628 | LOC90589 | NM_145233.2 | 1179 | gcctcgagcgttaaaatccatga | 324520 | - | see also 20177 line |
| 45629 | LPIN2 | NM_014646.2 | 952 | cagcgtgtcctaagagtgattca | 324530 | - | see also 20178 line |
| 45630 | LPIN2 | NM_014646.2 | 788 | aggcgtgagctccgatgatgaca | 324526 | - | see also 20178 line |
| 45631 | MDM1 | NM_017440.2 | 168 | ttcctccgtggggcgaaagtacc | 324563 | - | see also 7012 line |
| 45632 | MDM1 | NM_017440.2 | 553 | accaaggttctttcagaaaatgt | 324567 | - | see also 7012 line |
| 45633 | MGC13071 | NM_032689.2 | 1133 | gtcatgccacaagcctcatatat | 324622 | - | see also 20180 line |
| 45634 | MGC13071 | NM_032689.2 | 628 | aggctcaattcatcccttaaaat | 324616 | - | see also 20180 line |
| 45635 | MGC27466 | NM_152373.2 | 491 | atctgactacccacttgttgatg | 324634 | - | see also 20181 line |
| 45636 | MGC27466 | NM_152373.2 | 669 | ttgccacctaatttagacttact | 324642 | - | see also 20181 line |
| 45637 | MGC45408 | NM_152289.1 | 1319 | cactacatccacaagtcttattc | 324654 | - | see also 20182 line |
| 45638 | MGC45408 | NM_152289.1 | 1322 | tacatccacaagtcttattcagc | 324655 | - | see also 20182 line |
| 45639 | MGC45408 | NM_152289.1 | 1317 | ttcactacatccacaagtcttat | 324653 | - | see also 20182 line |
| 45640 | NPAT | NM_002519.1 | 655 | tcccggtagacgcaaaagtgaat | 324670 | - | see also 20183 line |
| 45641 | NPAT | NM_002519.1 | 1367 | gagtccgtgcctaatttgaatga | 324693 | - | see also 20183 line |
| 45642 | NUCKS | NM_022731.1 | 286 | cactcagcagaggatagtgaaga | 324796 | - | see also 20184 line |
| 45643 | NUCKS | NM_022731.1 | 157 | ggccctcccactaagaaaattcg | 324791 | - | see also 20184 line |
| 45644 | NUSAP1 | NM_016359.1 | 1046 | aagaccatcacggggaattctgc | 324803 | - | see also 20185 line |
| 45645 | NUSAP1 | NM_016359.1 | 875 | ctcaagcgctctgctatctctgc | 324802 | - | see also 20185 line |
| 45646 | ORF1-FL49 | NM_032412.2 | 312 | ctcaggagcctcctaaaaccaca | 324821 | - | gpcr、receptor_acitivity、signal_transduction | - | - |
| 45647 | ORF1-FL49 | NM_032412.2 | 205 | accttatccaccacaaccaatgg | 324819 | - | see also 45646 line |
| 45648 | ORF1-FL49 | NM_032412.2 | 309 | gacctcaggagcctcctaaaacc | 324820 | - | see also 45646 line |
| 45649 | PCNP | NM_020357.1 | 243 | aggtagtcagacgacaaagaaag | 324824 | - | see also 20186 line |
| 45650 | PCNP | NM_020357.1 | 166 | gagaagcgatcagctgaagaaga | 324822 | - | see also 20186 line |
| 45651 | PELP1 | NM_014389.1 | 1285 | accaaggtgtatgcgatattaga | 324833 | - | see also 5649 line |

Figure 46

| | | | | | | | |
|---|---|---|---|---|---|---|---|
| 45652 | PELP1 | NM_014389.1 | 1528 | caccggaaagggatagcaatgc | 324834 | - | see also 5649 line |
| 45653 | PELP1 | NM_014389.1 | 1222 | tcctggagcatcggtagagattc | 324832 | - | see also 5649 line |
| 45654 | PHLDA1 | NM_007350.1 | 1267 | cacccgatccgcaccaaatacc | 324845 | - | see also 20188 line |
| 45655 | PHLDA1 | NM_007350.1 | 653 | agctctggaagaaaaagtgttgc | 324843 | - | see also 20188 line |
| 45656 | PLINP-1 | NM_052850.2 | 282 | ggccgaagaacgcgaatggtacc | 324848 | - | see also 20189 line |
| 45657 | PLINP-1 | NM_052850.2 | 230 | gggttggtcccgttcgttatgg | 324847 | - | see also 20189 line |
| 45658 | PRAC | NM_032391.2 | 135 | tgcgcccatttctcagatcaagg | 324850 | - | see also 20190 line |
| 45659 | PRAC | NM_032391.2 | 180 | aagagtgctttctctctaataa | 324851 | - | see also 20190 line |
| 45660 | RBBP5 | NM_005057.1 | 1029 | ctgttcgaccatcatagccatcc | 324874 | - | see also 3526 line |
| 45661 | RBBP5 | NM_005057.1 | 768 | cagatcgaataatcagagttat | 324872 | - | see also 3526 line |
| 45662 | RNF144 | NM_014746.2 | 1107 | tccttctgatacactacgataag | 324894 | - | see also 20192 line |
| 45663 | RNF144 | NM_014746.2 | 669 | tacaggagaacgagagattgagtgc | 324886 | - | see also 20192 line |
| 45664 | SH3BGRL2 | NM_031469.1 | 396 | gaccgatactgtggagattatga | 324899 | - | see also 20193 line |
| 45665 | SH3BGRL2 | NM_031469.1 | 209 | ctcgggcttgcgtggcgataaaga | 324896 | - | see also 20193 line |
| 45666 | SH3BGRL3 | NM_031286.1 | 104 | caccggctccgcgcgaaatcaagt | 324902 | - | see also 20194 line |
| 45667 | UBCE7IP5 | NM_014948.2 | 673 | gccacgtggcccacttaaggatc | 324908 | - | see also 6146 line |
| 45668 | UBCE7IP5 | NM_014948.2 | 1089 | ttcacggggtagcttttactcc | 324912 | - | see also 6146 line |
| 45669 | UTX | NM_021140.1 | 1464 | ctccgcgcaaatagaaataattt | 324933 | - | see also 8100 line |
| 45670 | UTX | NM_021140.1 | 1287 | gaggcgtggagcctaccaattcc | 324927 | - | see also 8100 line |
| 45671 | UTY | NM_007125.3 | 3446 | aagtagacctgcctttagctagc | 324991 | - | see also 20197 line |
| 45672 | UTY | NM_007125.3 | 1846 | ggccaatcgtgtattttcttgg | 324965 | - | see also 20197 line |
| 45673 | WDR13 | NM_017883.3 | 555 | cacagtttcggacgcagtatatc | 325004 | - | see also 20198 line |
| 45674 | WDR13 | NM_017883.3 | 959 | gcctttgcgggcatgtatcatgt | 325007 | - | see also 20198 line |
| 45675 | WDR13 | NM_017883.3 | 734 | cgcagtagccgcactactcttga | 325005 | - | see also 20198 line |
| 45676 | WDR3 | NM_006784.1 | 1104 | atccagccgggtgactaatataaa | 325040 | - | see also 20199 line |
| 45677 | WDR3 | NM_006784.1 | 528 | aagaacctgctagttactagtgg | 325029 | - | see also 20199 line |
| 45678 | WRB | NM_004627.2 | 429 | gtcgtgccgagtaaatggataac | 325103 | - | see also 20200 line |
| 45679 | WRB | NM_004627.2 | 424 | tggctgtcgtgccgagtaaatgg | 325101 | - | see also 20200 line |
| 45680 | YT521 | NM_133370.1 | 673 | atcggaaaattcgtctatcaagt | 325109 | - | see also 9566 line |
| 45681 | ZFP1 | NM_153688.1 | 373 | gacgagcaggcgaggcaattttt | 325157 | - | see also 20201 line |
| 45682 | ZFP1 | NM_153688.1 | 627 | aacccacagtgagtagaatatt | 325164 | - | see also 20201 line |
| 45683 | ZFP1 | NM_153688.1 | 377 | agcaggcgaggcaatttaatt | 325160 | - | see also 20201 line |
| 45684 | ZNF285 | NM_152354.2 | 723 | atcaacgtttgaaaaacgtaatg | 325181 | - | see also 20202 line |
| 45685 | ZNF285 | NM_152354.2 | 308 | aacaaaggatccggagatttaact | 325177 | - | see also 20202 line |

Figure 46

| | | | | | | | |
|---|---|---|---|---|---|---|---|
| 45686 | ZNF491 | NM_152356.2 | 1035 | ctgccttaagctttcgaaga | 325191 | - | see also 20203 line |
| 45687 | ZNF491 | NM_152356.2 | 619 | tcctgcaagcattcgaagatata | 325188 | - | see also 20203 line |
| 45688 | ZNF493 | NM_175910.4 | 1810 | acctcgctgggcacaagcaaat | 325196 | - | see also 20204 line |
| 45689 | ZNF493 | NM_175910.4 | 692 | tggcacatctttaccaattct | 325195 | - | see also 20204 line |
| 45690 | ZNF501 | NM_145044.1 | 577 | ttccgtaaacagtcaaatcttac | 325200 | - | see also 20205 line |
| 45691 | ZNF501 | NM_145044.1 | 656 | aagcctttcaaacaaaagcaatt | 325201 | - | see also 20205 line |
| 45692 | ZNF502 | NM_033210.3 | 1424 | cagagcacccactcactaaaca | 325221 | - | see also 20206 line |
| 45693 | ZNF502 | NM_033210.3 | 1426 | gagcacccactcactaaacatc | 325222 | - | see also 20206 line |
| 45694 | ATF7IP | NM_018179.3 | 2861 | aactaatcgagtctactacaga | 325272 | - | see also 7304 line |
| 45695 | ATF7IP | NM_018179.3 | 3856 | ttgtatctggtagcaaatactac | 325297 | - | see also 7304 line |
| 45696 | C14orf18 | NM_021178.2 | 969 | gagcgcaatcgtcagtatcaaaa | 325318 | - | see also 20208 line |
| 45697 | C14orf18 | NM_021178.2 | 970 | agcgcaatcgtcagtatcaaaag | 325319 | - | see also 20208 line |
| 45698 | C14orf18 | NM_021178.2 | 1012 | gccttaggctacgaaacatcact | 325322 | - | see also 20208 line |
| 45699 | LOC112840 | NM_080666.1 | 1261 | aacgagtacgagttcatagtaat | 325364 | - | see also 20209 line |
| 45700 | LOC112840 | NM_080666.1 | 1267 | tacgagttcatagtaatgattct | 325366 | - | see also 20209 line |
| 45701 | LRP10 | NM_014045.2 | 2064 | atcttacgccaggatatgactcc | 325380 | - | see also 20210 line |
| 45702 | LRP10 | NM_014045.2 | 519 | gcccatccagacccgattattt | 325368 | - | see also 20210 line |
| 45703 | LRP10 | NM_014045.2 | 518 | ggcccatccagaccggattatt | 325367 | - | see also 20210 line |
| 45704 | WDR21 | NM_015604.2 | 1265 | gccaccgcctgtttcatgattc | 325392 | - | - |
| 45705 | WDR21 | NM_015604.2 | 1268 | accccgccgtttcatgattcagc | 325393 | - | see also 45704 line |
| 45706 | WDR21 | NM_015604.2 | 366 | cagagctacctggttttacttt | 325381 | - | see also 45704 line |
| 45707 | WDR23 | NM_025230.3 | 1303 | atggccgtttccgtaaattcaag | 325400 | - | see also 20211 line |
| 45708 | WDR23 | NM_025230.3 | 950 | tcgtcttgggatcgatacaacc | 325395 | - | see also 20211 line |
| 45709 | FLJ12847 | NM_024596.1 | 1376 | aagcggttggtcgaaaagcaca | 325446 | - | see also 20212 line |
| 45710 | FLJ12847 | NM_024596.1 | 355 | ttgaacatttcacgtgatactt | 325429 | - | see also 20212 line |
| 45711 | MDC1 | NM_014641.1 | 2795 | tgcgatccagccgagttagaaga | 325500 | - | see also 20213 line |
| 45712 | MDC1 | NM_014641.1 | 5652 | gcctccgacgggaccaaacttaac | 325514 | - | see also 20213 line |
| 45713 | MDC1 | NM_014641.1 | 5651 | agcctccgacgggaccaaacttaa | 325513 | - | see also 20213 line |
| 45714 | PAXIP1L | NM_007349.2 | 2379 | gccaaatatacgggttatctatg | 325559 | - | see also 5082 line |
| 45715 | PAXIP1L | NM_007349.2 | 2919 | aagttcctgacggcgatttctgt | 325571 | - | see also 5082 line |
| 45716 | PAXIP1L | NM_007349.2 | 1099 | ctgaggtccgggtaatttaatg | 325535 | - | see also 5082 line |
| 45717 | CD9 | NM_001769.2 | 241 | atcttcgagcaagaaactaataa | 325594 | - | see also 20215 line |
| 45718 | CD9 | NM_001769.2 | 146 | acctgctgtccgatttaacttc | 325592 | - | see also 20215 line |
| 45719 | CDW52 | NM_001803.1 | 61 | ctcctcgtttattgttacagataca | 325600 | - | see also 20216 line |
| 45720 | CDW52 | NM_001803.1 | 84 | aactggactctcaggacaaaacg | 325602 | - | see also 20216 line |

Figure 46

| | | | | | | | |
|---|---|---|---|---|---|---|---|
| 45721 | CEACAM4 | NM_001817.1 | 252 | ttctactggcctgcaatatttca | 325606 | - | - |
| 45722 | CEACAM4 | NM_001817.1 | 455 | tacaccctacgaaccataaatgc | 325610 | - | see also 45721 line |
| 45723 | CEACAM4 | NM_001817.1 | 452 | tcctacaccctacgaaccataaa | 325609 | - | see also 45721 line |
| 45724 | DSCR2 | NM_003720.1 | 575 | atgttaccgattataaaacctca | 325625 | - | see also 20217 line |
| 45725 | DSCR2 | NM_003720.1 | 569 | gtcgacatgttaccgattataaa | 325624 | - | see also 20217 line |
| 45726 | GARP | NM_005512.1 | 880 | cgcctcccgagactcatctacc | 325636 | - | see also 20218 line |
| 45727 | GARP | NM_005512.1 | 1637 | tgcctcaagcggtcaatcttgc | 325640 | - | see also 20218 line |
| 45728 | GPM6A | NM_005277.2 | 862 | agcggtcaatgcatacacataa | 325666 | - | see also 3702 line |
| 45729 | GPM6A | NM_005277.2 | 729 | atggtcactacccttatggttct | 325659 | - | see also 3702 line |
| 45730 | GYPE | NM_002102.2 | 214 | ggggcgatggctcgtgttatttt | 325667 | - | see also 20220 line |
| 45731 | GYPE | NM_002102.2 | 215 | ggcgatggctcgtgttattttg | 325668 | - | see also 20220 line |
| 45732 | GYPE | NM_002102.2 | 216 | gcgatggctcgtgttattttga | 325669 | - | see also 20220 line |
| 45733 | HEM1 | NM_005337.2 | 261 | tccgaaacagcacgcaacattta | 325679 | - | see also 3724 line |
| 45734 | HEM1 | NM_005337.2 | 1208 | ctggttcgccacacagagaatgt | 325707 | - | see also 3724 line |
| 45735 | JTB | NM_006694.2 | 1081 | ttcgcttgttctttgtcatcattcg | 325749 | - | see also 20222 line |
| 45736 | JTB | NM_006694.2 | 1025 | tggaacaacgcttattttggaag | 325747 | - | see also 20222 line |
| 45737 | JTB | NM_006694.2 | 1021 | ttgatggaacaacgcttatttg | 325746 | - | see also 20222 line |
| 45738 | KAI1 | NM_002231.2 | 361 | cggcgtgggggcagtcactatgc | 325751 | - | see also 20223 line |
| 45739 | KAI1 | NM_002231.2 | 697 | ccctgttcctgcgaagtcaagg | 325754 | - | see also 20223 line |
| 45740 | M11S1 | NM_005898.2 | 1983 | acgtagcaatcagcctattaca | 325797 | - | see also 4104 line |
| 45741 | M11S1 | NM_005898.2 | 2099 | atggttaccgccttcattctct | 325800 | - | see also 4104 line |
| 45742 | MLANA | NM_005511.1 | 180 | atcggctgttggttattgtagaag | 325813 | - | see also 20225 line |
| 45743 | MLANA | NM_005511.1 | 175 | tgctcatcggctgttggttattgt | 325811 | - | see also 20225 line |
| 45744 | MLANA | NM_005511.1 | 177 | ctcatcggctgttggttattgtag | 325812 | - | see also 20225 line |
| 45745 | MOX2 | NM_005944.3 | 803 | ttctcgtcctaatctcaatctta | 325831 | - | see also 20226 line |
| 45746 | MOX2 | NM_005944.3 | 714 | ggggactgtgaccgacttaagc | 325828 | - | see also 20226 line |
| 45747 | NET-5 | NM_006675.2 | 687 | aacgggctgctatgaaaaggtga | 325836 | - | see also 20227 line |
| 45748 | NET-5 | NM_006675.2 | 815 | tccacggactggtaagaagtac | 325838 | - | see also 20227 line |
| 45749 | NET-7 | NM_012339.2 | 258 | agtttgagcggcagaaatataaa | 325841 | - | see also 20228 line |
| 45750 | NET-7 | NM_012339.2 | 257 | gagtttgagcggcagaaatataa | 325840 | - | see also 20228 line |
| 45751 | NKG7 | NM_005601.2 | 271 | gacgcagaccttcagcattatgg | 325850 | - | see also 20229 line |
| 45752 | PODXL | NM_005397.2 | 951 | ccctaccgtcatcgttatctcg | 325855 | - | see also 20230 line |
| 45753 | PODXL | NM_005397.2 | 1370 | cagaccgtggtcgtcaaagaaat | 325860 | - | see also 20230 line |
| 45754 | SYNGR1 | NM_004711.3 | 351 | agcgtcaaggaccgccagaaagc | 325866 | - | - |

Figure 46

| | | | | | | | |
|---|---|---|---|---|---|---|---|
| 45755 | T1A-2 | NM_006474.3 | 443 | agcgaagaccgctataagtctgg | 325872 | - | see also 20233 line |
| 45756 | T1A-2 | NM_006474.3 | 439 | aaccagcggaagaccgcctataagt | 325871 | - | see also 20233 line |
| 45757 | TM4SF13 | NM_014399.2 | 548 | tacggcaagtgctcgaaatgaca | 325886 | - | see also 20234 line |
| 45758 | TM4SF13 | NM_014399.2 | 476 | ttctgtatcttgcgcttgtttag | 325883 | - | see also 20234 line |
| 45759 | TM7SF1 | NM_003272.1 | 963 | acctaccacccttagtcgttatt | 325903 | - | see also 20235 line |
| 45760 | TM7SF1 | NM_003272.1 | 909 | gggagatgctggatacgtattat | 325900 | - | see also 20235 line |
| 45761 | TMEM4 | NM_014255.2 | 785 | atccgaatcgactcagatattag | 325916 | - | see also 20236 line |
| 45762 | TMEM4 | NM_014255.2 | 908 | tgcagtaagcgaacagatctttg | 325921 | - | see also 20236 line |
| 45763 | TMEM5 | NM_014254.1 | 1092 | tgcccggtcggagtaaaacacaga | 325946 | - | see also 20237 line |
| 45764 | TMEM5 | NM_014254.1 | 1109 | cacagaatgctatcgaatctatg | 325947 | - | see also 20237 line |
| 45765 | ADIPOR1 | NM_015999.2 | 766 | caccgtcattgtcattcagaga | 325961 | - | see also 6600 line |
| 45766 | ADIPOR1 | NM_015999.2 | 756 | ggcttcttcacacgtctattgt | 325959 | - | see also 6600 line |
| 45767 | ADIPOR2 | NM_024551.2 | 281 | tggtacacgaagagttgataatg | 325972 | - | see also 20239 line |
| 45768 | ADIPOR2 | NM_024551.2 | 1187 | ccggatcccgaaagcttttttcc | 325993 | - | see also 20239 line |
| 45769 | AMIGO2 | NM_181847.2 | 1683 | ttgcgtggccagtatcgttttgg | 326027 | - | see also 10258 line |
| 45770 | AMIGO2 | NM_181847.2 | 758 | ccctaattcttcgtcataacaac | 326003 | - | see also 10258 line |
| 45771 | AMIGO2 | NM_181847.2 | 1590 | aactgtggacgtcacaataaatg | 326023 | - | see also 10258 line |
| 45772 | ANTXR2 | NM_058172.1 | 268 | gacggtcggtgccatcatatgc | 326043 | - | see also 20241 line |
| 45773 | ANTXR2 | NM_058172.1 | 229 | acctccagtatcataattgctct | 326042 | - | see also 20241 line |
| 45774 | ARL6IP | NM_015161.1 | 322 | cccattctagcgcctagaatttt | 326065 | - | see also 20242 line |
| 45775 | ARL6IP | NM_015161.1 | 321 | tcccattctagccgctagaattt | 326064 | - | see also 20242 line |
| 45776 | BM88 | NM_016564.2 | 575 | ggcagccatagccctgattctcg | 326074 | - | see also 20243 line |
| 45777 | C11orf15 | NM_020644.1 | 238 | ttcgaggatgtcagatgtaaatg | 326075 | - | see also 7901 line |
| 45778 | C11orf15 | NM_020644.1 | 470 | ttctacttcgtacatggtatat | 326084 | - | see also 7901 line |
| 45779 | C19orf4 | NM_012109.1 | 564 | ctccacctggacatactactgc | 326090 | - | see also 20245 line |
| 45780 | C1orf8 | NM_004872.3 | 343 | gaccgcttcggctgaagcatttg | 326091 | - | see also 3385 line |
| 45781 | C20orf103 | NM_012261.2 | 842 | gcggtccacatccaacctttga | 326125 | - | see also 20247 line |
| 45782 | C20orf103 | NM_012261.2 | 768 | atgagtgtcaagtcaacaaacc | 326123 | - | see also 20247 line |
| 45783 | C20orf165 | NM_080608.3 | 546 | ggccgctcaagctttcatgatgg | 326131 | - | see also 20248 line |
| 45784 | C20orf165 | NM_080608.3 | 643 | atgccctagtgagatctaaaag | 326132 | - | see also 20248 line |
| 45785 | C20orf39 | NM_024893.1 | 986 | accggtcgccaccaaagacagc | 326134 | - | - |
| 45786 | C20orf39 | NM_024893.1 | 1017 | ccggatgggagttcattgacc | 326135 | - | see also 45785 line |
| 45787 | C20orf39 | NM_024893.1 | 1039 | ctctcagctgatgacataaaat | 326136 | - | see also 45785 line |
| 45788 | C20orf46 | NM_018354.1 | 1326 | gaactacggctcattaacctgc | 326142 | - | see also 20249 line |
| 45789 | C20orf46 | NM_018354.1 | 1102 | cccgtgaggctgagtcaatcc | 326140 | - | see also 20249 line |
| 45790 | C20orf52 | NM_080748.1 | 205 | ctgcttcgaccgtcaaaatgg | 326144 | - | see also 20250 line |
| 45791 | C20orf52 | NM_080748.1 | 203 | agctgcttcgaccgtgtcaaaat | 326143 | - | see also 20250 line |

Figure 46

| | | | | | | | |
|---|---|---|---|---|---|---|---|
| 45792 | C20orf52 | NM_080748.1 | 264 | gggcgctcttcggcaccttttcc | 326145 | - | see also 20250 line |
| 45793 | C20orf54 | NM_033409.2 | 741 | gtcaatgtcactgagatatcaga | 326150 | - | see also 20251 line |
| 45794 | C20orf54 | NM_033409.2 | 939 | tgctgcctcgtggcgttctttgt | 326152 | - | see also 20251 line |
| 45795 | C20orf54 | NM_033409.2 | 738 | tgcgtcaatgtcactgagatatc | 326149 | - | see also 20251 line |
| 45796 | C20orf75 | NM_152611.2 | 1011 | gtcgccacacacatctttcaaga | 326156 | - | see also 20252 line |
| 45797 | C20orf75 | NM_152611.2 | 1170 | ctcctcacggatgcaaagagaac | 326159 | - | see also 20252 line |
| 45798 | C21orf61 | NM_178817.2 | 204 | acgcctctgccccatactacagc | 326164 | - | see also 20253 line |
| 45799 | C21orf61 | NM_178817.2 | 276 | agctgaaagcccacaaacattcc | 326166 | - | see also 20253 line |
| 45800 | C22orf20 | NM_025225.2 | 1203 | aacttgctacccattaggataat | 326179 | - | see also 20254 line |
| 45801 | C22orf20 | NM_025225.2 | 583 | aagtcgtggatgccttggtatgt | 326171 | - | see also 20254 line |
| 45802 | C22orf5 | NM_012264.2 | 654 | gacttattccatcggatttctga | 326190 | - | see also 20255 line |
| 45803 | C22orf5 | NM_012264.2 | 586 | atgtcggagatcagaggaaaacc | 326185 | - | see also 20255 line |
| 45804 | C22orf5 | NM_012264.2 | 978 | gtgtggggccatccccaaaatcc | 326191 | - | see also 20255 line |
| 45805 | C3orf4 | NM_019895.1 | 1250 | aaccacaatagcgggattgatct | 326205 | - | see also 7752 line |
| 45806 | C3orf4 | NM_019895.1 | 968 | gacttctggtatgaatatcgaag | 326195 | - | see also 7752 line |
| 45807 | C6orf31 | NM_030651.2 | 1004 | cgcgcagcaccacgagaactact | 326220 | - | see also 20257 line |
| 45808 | C6orf31 | NM_030651.2 | 1006 | cgcagcaccacgagaactactgg | 326221 | - | see also 20257 line |
| 45809 | CAV2 | NM_001233.3 | 413 | ggcggacgtacagctcttcatgg | 326222 | - | see also 20258 line |
| 45810 | CDC91L1 | NM_080476.3 | 763 | cccgcagtctatggctttatact | 326249 | - | see also 20259 line |
| 45811 | CDC91L1 | NM_080476.3 | 762 | ccccgcagtctatggctttatac | 326248 | - | see also 20259 line |
| 45812 | CEACAM7 | NM_006890.1 | 702 | gtccgctatgagtcagtacaagc | 326274 | - | see also 20260 line |
| 45813 | CEACAM7 | NM_006890.1 | 220 | tgcatgccaactatcgaattata | 326264 | - | see also 20260 line |
| 45814 | CGI-119 | NM_016056.1 | 93 | ctcgatcgaggacgacttcaact | 326276 | - | see also 20261 line |
| 45815 | CGI-119 | NM_016056.1 | 292 | ctcggatctctgggtttgatttt | 326280 | - | see also 20261 line |
| 45816 | CGI-147 | NM_016077.1 | 311 | agcggggagtacaagatgattct | 326295 | - | see also 20262 line |
| 45817 | CGI-147 | NM_016077.1 | 333 | ttgtggttcgaaatgacttaaag | 326299 | - | see also 20262 line |
| 45818 | CKAP4 | NM_006825.2 | 1767 | tgcatactcggtcaaaatagaaa | 326319 | - | see also 20263 line |
| 45819 | CKAP4 | NM_006825.2 | 1763 | tggttgcatactcggtcaaaata | 326318 | - | see also 20263 line |
| 45820 | CLN6 | NM_017882.1 | 556 | ccccatcatcaagaatctcaagc | 326324 | - | see also 20264 line |
| 45821 | CLN6 | NM_017882.1 | 337 | gcccagtgttggggactacttcc | 326322 | - | see also 20264 line |
| 45822 | CUZD1 | NM_022034.3 | 2316 | atctccaacctacgacctaatca | 326369 | - | see also 8242 line |
| 45823 | CUZD1 | NM_022034.3 | 2319 | tccaacctacgacctaatcaaga | 326371 | - | see also 8242 line |
| 45824 | CUZD1 | NM_022034.3 | 1665 | caccttcgaatcgtcatcaaact | 326341 | - | see also 8242 line |
| 45825 | DC-TM4F2 | NM_030927.1 | 475 | tcctaccgggacgatatcgatct | 326385 | - | see also 8965 line |

Figure 46

| | | | | | | | |
|---|---|---|---|---|---|---|---|
| 45826 | DC-TM4F2 | NM_030927.1 | 566 | acctcaacgtctacttcaattgc | 326389 | - | see also 8965 line |
| 45827 | DC-TM4F2 | NM_030927.1 | 805 | atctcgctgttgcagatatttgg | 326390 | - | see also 8965 line |
| 45828 | DCAL1 | NM_172004.2 | 248 | tagtctacgctgacatcaaaact | 326397 | - | see also 20267 line |
| 45829 | DCAL1 | NM_172004.2 | 250 | gtctacgctgacatcaaaactgt | 326398 | - | see also 20267 line |
| 45830 | ELOVL5 | NM_021814.2 | 644 | caccgcaggagaatcagatatga | 326418 | - | see also 20268 line |
| 45831 | ELOVL5 | NM_021814.2 | 831 | tgcggccactcttattttggtgc | 326424 | - | see also 20268 line |
| 45832 | ELOVL5 | NM_021814.2 | 871 | tccacgtcctcatgtactcttac | 326425 | - | see also 20268 line |
| 45833 | ELOVL6 | NM_024090.1 | 417 | gtggtcggcacctaatgaataaa | 326430 | - | see also 8588 line |
| 45834 | F-LANa | NM_016041.2 | 389 | tacaataatgctcgtctatgtgt | 326459 | - | see also 6636 line |
| 45835 | F-LANa | NM_016041.2 | 208 | ttgggccagttggattcaatttt | 326455 | - | see also 6636 line |
| 45836 | F-LANa | NM_016041.2 | 375 | ttgggccaggcctttacaataat | 326458 | - | see also 6636 line |
| 45837 | FAM14A | NM_032036.1 | 383 | cccccactcaagtcagagaaaca | 326468 | - | see also 20270 line |
| 45838 | FAM14A | NM_032036.1 | 281 | ggggcctgcttggggaattcacc | 326465 | - | see also 20270 line |
| 45839 | FAM14B | NM_145249.1 | 448 | ctggactctctgtgacatctaaa | 326470 | - | see also 20271 line |
| 45840 | FAM14B | NM_145249.1 | 410 | tggcagtctggtggctattctgc | 326469 | - | see also 20271 line |
| 45841 | FLJ10134 | NM_018004.1 | 1016 | atcgttggaatgaattatgcttt | 326496 | - | see also 20272 line |
| 45842 | FLJ10134 | NM_018004.1 | 537 | cccatctgatgttatatgactat | 326481 | - | see also 20272 line |
| 45843 | FLJ10134 | NM_018004.1 | 931 | aggtcctgcatgggatctgatgg | 326492 | - | see also 20272 line |
| 45844 | FLJ22233 | NM_024959.2 | 1034 | aggaccgggtatcttctaatacc | 326509 | - | see also 20273 line |
| 45845 | FLJ22233 | NM_024959.2 | 1033 | gaggaccgggtatcttctaatac | 326508 | - | see also 20273 line |
| 45846 | FLJ22800 | NM_024795.1 | 700 | ctctaagcgaagaagtcaaattg | 326531 | - | see also 20274 line |
| 45847 | FLJ22800 | NM_024795.1 | 698 | gtctctaagcgaagaagtcaaat | 326530 | - | see also 20274 line |
| 45848 | FLJ30002 | NM_152341.2 | 796 | gggcaactcccaccagatcatgc | 326539 | - | see also 9841 line |
| 45849 | FLJ30002 | NM_152341.2 | 367 | tgcaggctccgtgctctatcacc | 326534 | - | see also 9841 line |
| 45850 | FLOT1 | NM_005803.2 | 625 | ttcacgatgaccaggactatttg | 326542 | - | see also 20276 line |
| 45851 | FLOT1 | NM_005803.2 | 803 | gagagattacgaactgaagaagg | 326544 | - | see also 20276 line |
| 45852 | GHITM | NM_014394.1 | 963 | ggcaatgtacggtggattagttc | 326573 | - | see also 20277 line |
| 45853 | GHITM | NM_014394.1 | 648 | tggaatgctggtacgatcaatac | 326561 | - | see also 20277 line |
| 45854 | GPSN2 | NM_004868.1 | 146 | tggcgctcgccatctttgtgatc | 326580 | - | see also 20278 line |
| 45855 | GPSN2 | NM_004868.1 | 78 | gtggatggcctattacatcaatc | 326579 | - | see also 20278 line |
| 45856 | GYPA | NM_002099.2 | 449 | gacacagacgtgcctttaagttc | 326599 | - | see also 20279 line |
| 45857 | GYPA | NM_002099.2 | 193 | tacgcacaaacgggacacatatg | 326590 | - | see also 20279 line |
| 45858 | HCST | NM_014266.3 | 189 | atcatcactccctgcctttacc | 326601 | - | see also 20280 line |
| 45859 | HCST | NM_014266.3 | 359 | atggcaaagtctacatcaacatg | 326604 | - | see also 20280 line |
| 45860 | HSPC039 | NM_016097.2 | 255 | gagagtgccattgataatagtaa | 326612 | - | see also 20281 line |

Figure 46

| | | | | | | | |
|---|---|---|---|---|---|---|---|
| 45861 | HSPC039 | NM_016097.2 | 226 | aaccttattcgatctgtaagaac | 326609 | - | see also 20281 line |
| 45862 | HTGN29 | NM_020199.1 | 372 | aaccgtactcaactcacatatt | 326617 | - | see also 20282 line |
| 45863 | HTGN29 | NM_020199.1 | 363 | accgagcccaacgtactcaact | 326616 | - | see also 20282 line |
| 45864 | HUCEP11 | NM_014858.1 | 256 | gacgcaaatgtggcagagtatct | 326633 | - | see also 20283 line |
| 45865 | HUCEP11 | NM_014858.1 | 612 | cagcctcatccgcaacaagtttg | 326634 | - | see also 20283 line |
| 45866 | IFRG28 | NM_022147.1 | 168 | ggctcaaggtggaagcaatacc | 326637 | - | see also 20284 line |
| 45867 | IFRG28 | NM_022147.1 | 685 | gggagtggagtatgagaaattagg | 326647 | - | see also 20284 line |
| 45868 | IGSF8 | NM_052868.1 | 1274 | ggctatgaaggccgacacattgc | 326649 | - | see also 20285 line |
| 45869 | IGSF8 | NM_052868.1 | 1846 | ccgctcaggccgttacagtct | 326651 | - | see also 20285 line |
| 45870 | ITM2A | NM_004867.2 | 116 | ccctaccgccgtgcaaaaggagg | 326655 | - | see also 20286 line |
| 45871 | ITM2A | NM_004867.2 | 113 | tacccctaccgccgtgcaaaagg | 326654 | - | see also 20286 line |
| 45872 | ITM2C | NM_030926.3 | 568 | gtctgactcgtaccatgatatc | 326679 | - | see also 8964 line |
| 45873 | ITM2C | NM_030926.3 | 818 | aacgcgaggcggatcaacaagc | 326681 | - | see also 8964 line |
| 45874 | KIAA0133 | NM_014777.1 | 4386 | acctgcctacggtcctaaagtgt | 326764 | - | see also 20288 line |
| 45875 | KIAA0133 | NM_014777.1 | 1980 | aggtggacctatgttcagtttg | 326712 | - | see also 20288 line |
| 45876 | KIAA0152 | NM_014730.1 | 614 | cacagctcacgatgaaattatac | 326784 | - | see also 20289 line |
| 45877 | KIAA0152 | NM_014730.1 | 456 | ggctacgaagtgcccatcaaaga | 326780 | - | see also 20289 line |
| 45878 | KIAA0247 | NM_014734.1 | 339 | tccttccgctagtgatcctttgc | 326793 | - | see also 20290 line |
| 45879 | KIAA0247 | NM_014734.1 | 503 | atgttgaagggcgattacaaata | 326796 | - | see also 20290 line |
| 45880 | KIAA0779 | NM_015008.1 | 1097 | atcgctacagtcctatgaacg | 326827 | - | see also 20291 line |
| 45881 | KIAA0779 | NM_015008.1 | 722 | cagtctagcccaaaatatggtag | 326816 | - | see also 20291 line |
| 45882 | KIAA0779 | NM_015008.1 | 571 | agcaggcgctgagtctcaaagc | 326809 | - | see also 20291 line |
| 45883 | KIAA1145 | NM_020698.1 | 901 | gtcaggctcggccgacagtaacg | 326849 | - | see also 7922 line |
| 45884 | LAPTM4B | NM_018407.3 | 509 | tgctcggcgtctggtatctgatc | 326858 | - | see also 20292 line |
| 45885 | LAPTM4B | NM_018407.3 | 570 | ggctgatccgatcagtataact | 326865 | - | see also 20292 line |
| 45886 | LAPTM4B | NM_018407.3 | 961 | aactgctaccgatacatcaatgg | 326876 | - | see also 20292 line |
| 45887 | LEPROTL1 | NM_015344.1 | 125 | gagcaatccgactgatgttttg | 326883 | - | see also 20293 line |
| 45888 | LEPROTL1 | NM_015344.1 | 123 | aggagcaatccgactgatgtttt | 326882 | - | see also 20293 line |
| 45889 | LOC116211 | NM_138461.1 | 551 | tgcatagtaggaattatctgtat | 326906 | - | see also 20294 line |
| 45890 | LOC116211 | NM_138461.1 | 547 | gaacctgcatagtaggaattatct | 326905 | - | see also 20294 line |
| 45891 | LOC116441 | NM_138786.1 | 209 | atcgtgaacatattattgtatt | 326913 | - | see also 20295 line |
| 45892 | LOC116441 | NM_138786.1 | 200 | tggagtataatcgtgaacatatt | 326912 | - | see also 20295 line |

Figure 46

| 45893 | LOC91663 | NM_138373.2 | 960 | ctcggcgctcgagagatgtaagc | 326934 | - | - | - | - |
|---|---|---|---|---|---|---|---|---|---|
| 45894 | LRRTM1 | NM_178839.3 | 733 | tgctcggtctctgtctatactgg | 326935 | - | see also 20296 line | | |
| 45895 | LRRTM1 | NM_178839.3 | 965 | cggccagttcacggggttaatgc | 326936 | - | see also 20296 line | | |
| 45896 | LRRTM2 | NM_015564.1 | 1661 | atcttcactcagcgggtaattac | 326983 | - | see also 6475 line | | |
| 45897 | LRRTM2 | NM_015564.1 | 1100 | ctgcacacgctcttcttacaatg | 326965 | - | see also 6475 line | | |
| 45898 | MAL2 | NM_052886.1 | 509 | aaccagtataacataaacgtagc | 327004 | - | see also 20298 line | | |
| 45899 | MAL2 | NM_052886.1 | 524 | aacgtagcagcctcaatttttgc | 327005 | - | see also 20298 line | | |
| 45900 | MAL2 | NM_052886.1 | 292 | gtccgtgacagcgtttttctttt | 326997 | - | see also 20298 line | | |
| 45901 | MPDU1 | NM_004870.1 | 168 | tggctggctcacttctagtaaag | 327012 | - | see also 20300 line | | |
| 45902 | MPDU1 | NM_004870.1 | 169 | ggctggctcacttctagtaaagc | 327013 | - | see also 20300 line | | |
| 45903 | MPRG | NM_017705.2 | 1338 | tcccaagccagaattacataaaa | 327029 | - | see also 20301 line | | |
| 45904 | MPRG | NM_017705.2 | 810 | gtgcaccactttccatgactact | 327020 | - | see also 20301 line | | |
| 45905 | NIFIE14 | NM_032635.2 | 335 | gacctgataggtctaaaccttgt | 327032 | - | see also 20302 line | | |
| 45906 | NIFIE14 | NM_032635.2 | 740 | ctgtatgtcgccgttgtcaatgt | 327038 | - | see also 20302 line | | |
| 45907 | NMA | NM_012342.1 | 965 | ttcacggacaccattccaaaaag | 327046 | - | see also 20303 line | | |
| 45908 | NMA | NM_012342.1 | 439 | ctcaccaaaggtgaaattcgatg | 327040 | - | see also 20303 line | | |
| 45909 | PANX1 | NM_015368.3 | 828 | aagtttacaaccgtgcaattaag | 327057 | - | see also 20305 line | | |
| 45910 | PANX1 | NM_015368.3 | 859 | gagtgcgcgtgaccttgacatga | 327058 | - | see also 20305 line | | |
| 45911 | PANX2 | NM_052839.2 | 443 | tgcaggagatcgacaactgttac | 327082 | - | see also 20306 line | | |
| 45912 | PANX2 | NM_052839.2 | 914 | tccgcaagagcaacttcatcttc | 327083 | - | see also 20306 line | | |
| 45913 | PANX3 | NM_052959.2 | 886 | cggtgggacaaacgactttatc | 327106 | - | see also 9472 line | | |
| 45914 | PANX3 | NM_052959.2 | 540 | ggctcggaaagaacgatactttg | 327093 | - | see also 9472 line | | |
| 45915 | PANX3 | NM_052959.2 | 660 | ctccgccacttacctataccttg | 327095 | - | see also 9472 line | | |
| 45916 | PAQR3 | NM_177453.2 | 563 | ttcctgccatcggtcagaaaaaa | 327121 | - | see also 10163 line | | |
| 45917 | PAQR6 | NM_024897.2 | 855 | caggagccttcgcctatccattc | 327136 | - | see also 20309 line | | |
| 45918 | PAQR6 | NM_024897.2 | 1595 | gaccgtgaggggggctcttgatgg | 327145 | - | see also 20309 line | | |
| 45919 | PKD1L1 | NM_138295.2 | 154 | tgggtcgaggtctatgctaatca | 327147 | - | see also 20310 line | | |
| 45920 | PKD1L1 | NM_138295.2 | 153 | gtgggtcgaggtctatgctaatc | 327146 | - | see also 20310 line | | |
| 45921 | RFT1 | NM_052859.2 | 1245 | gaggtcgacaggtacaattttgt | 327333 | - | see also 9439 line | | |
| 45922 | RFT1 | NM_052859.2 | 817 | cagaaggcgagcgatatgtgatg | 327319 | - | see also 9439 line | | |
| 45923 | SECTM1 | NM_003004.1 | 775 | gccgctggcactggtgttcaaac | 327344 | - | see also 20312 line | | |
| 45924 | SECTM1 | NM_003004.1 | 473 | ctcgtgggacaccagagaaataa | 327341 | - | see also 20312 line | | |
| 45925 | SELS | NM_018445.2 | 312 | tgcgcaagttgaaaagcataagg | 327350 | - | see also 7480 line | | |
| 45926 | SELS | NM_018445.2 | 311 | atgcgcaagttgaaaagcataag | 327349 | - | see also 7480 line | | |
| 45927 | SELS | NM_018445.2 | 158 | ttctctacgtggtctttcagaag | 327346 | - | see also 7480 line | | |
| 45928 | SEZ6L | NM_021115.2 | 1738 | tccgacccgacctataacattgg | 327361 | - | see also 20313 line | | |
| 45929 | SEZ6L | NM_021115.2 | 1737 | atccgacccgacctataacattg | 327360 | - | see also 20313 line | | |

Figure 46

| 45930 | SLC24A4 | NM_153646.1 | 582 | gtgccgagactccgtgtactaca | 327393 | - | see also 10003 line |
|---|---|---|---|---|---|---|---|
| 45931 | SLC24A4 | NM_153646.1 | 584 | gccgagactccgtgtactacacc | 327394 | - | see also 10003 line |
| 45932 | SMP1 | NM_014313.2 | 197 | gggggaaaagcgcaatactatt | 327422 | - | see also 20315 line |
| 45933 | SMP1 | NM_014313.2 | 355 | atgattaatgcagtatcgaatgg | 327429 | - | see also 20315 line |
| 45934 | SORCS1 | NM_052918.2 | 727 | agcgtaagataatgttactcaca | 327444 | - | see also 9443 line |
| 45935 | SORCS1 | NM_052918.2 | 1179 | tacgtgtcctaccgaaggaatgc | 327457 | - | see also 9443 line |
| 45936 | STARD3NL | NM_032016.2 | 562 | agcaaaagtgatcctttcgaagc | 327528 | - | see also 20316 line |
| 45937 | STARD3NL | NM_032016.2 | 326 | accttttgacctcttattcgtaac | 327517 | - | see also 20316 line |
| 45938 | SYNGR4 | NM_012451.2 | 858 | ggccccaacagcctgagttatgc | 327536 | - | see also 20317 line |
| 45939 | SYNGR4 | NM_012451.2 | 738 | ttccaggacctccgaaatgatgc | 327534 | - | see also 20317 line |
| 45940 | TACSTD1 | NM_002354.1 | 721 | cacgagtattttgtatgagaata | 327548 | - | see also 1522 line |
| 45941 | TACSTD1 | NM_002354.1 | 306 | atcgtcaatgccagtgtacttca | 327541 | - | see also 1522 line |
| 45942 | TDE1 | NM_006811.2 | 876 | tgcgttgtggcttctattatatc | 327584 | - | see also 4786 line |
| 45943 | TDE1 | NM_006811.2 | 239 | cacggtgactcgcctcatttatg | 327564 | - | see also 4786 line |
| 45944 | TDE2 | NM_020755.2 | 1067 | atccgtacttcaaacaatagtca | 327625 | - | see also 7945 line |
| 45945 | TDE2 | NM_020755.2 | 1283 | accaactggtacaggtatgaacc | 327636 | - | see also 7945 line |
| 45946 | TDE2 | NM_020755.2 | 1304 | ccctctcgtgagatgaaaagtca | 327637 | - | see also 7945 line |
| 45947 | TED | NM_015686.1 | 837 | ttcgacctcgtgctgcataaata | 327646 | - | see also 20321 line |
| 45948 | TED | NM_015686.1 | 1007 | gacccggtgcccctttatactcc | 327654 | - | see also 20321 line |
| 45949 | TM4-B | NM_012466.2 | 553 | aacgagccagacgactattctac | 327665 | - | see also 20322 line |
| 45950 | TM4-B | NM_012466.2 | 606 | gtgctgtggggtgaataactaca | 327668 | - | see also 20322 line |
| 45951 | TM4SF12 | NM_012338.2 | 427 | ttccagtacaatggtcagatatg | 327680 | - | see also 5298 line |
| 45952 | TM4SF12 | NM_012338.2 | 881 | atgtccctcagtagaactgttga | 327694 | - | see also 5298 line |
| 45953 | TM7SF3 | NM_016551.1 | 1675 | tggccgattaacccagattaaag | 327740 | - | see also 20324 line |
| 45954 | TM7SF3 | NM_016551.1 | 122 | agcgagggtcttattgaattttc | 327702 | - | see also 20324 line |
| 45955 | TMC2 | NM_080751.1 | 1037 | ttcgatcgatggccagcaatacc | 327757 | - | see also 9522 line |
| 45956 | TMC2 | NM_080751.1 | 2007 | accccatgaacgcgtgttcaaag | 327781 | - | see also 9522 line |
| 45957 | TMC2 | NM_080751.1 | 2008 | ccccatgaacgcgtgttcaaagc | 327782 | - | see also 9522 line |
| 45958 | TMEFF1 | NM_003692.2 | 529 | aggaccatgctactctgataatg | 327801 | - | see also 20326 line |
| 45959 | TMEFF1 | NM_003692.2 | 317 | agggagtctgacgtaagagtttg | 327791 | - | see also 20326 line |
| 45960 | TMEM10 | NM_033207.2 | 758 | gaccgttaccgtcctactataga | 327825 | - | see also 20327 line |
| 45961 | TMEM10 | NM_033207.2 | 705 | aagaggcccacatatatgtgaag | 327824 | - | see also 20327 line |
| 45962 | TMEM15 | NM_014908.1 | 533 | ttccgaatgtccgcaaacagtgg | 327829 | - | see also 20328 line |
| 45963 | TMEM15 | NM_014908.1 | 1750 | atctatatttgcgcagatcattt | 327847 | - | see also 20328 line |
| 45964 | TMEM15 | NM_014908.1 | 1313 | cccaccatcgcccgaaagtattt | 327840 | - | see also 20328 line |

Figure 46

| 45965 | TMEM2 | NM_013390.1 | 1781 | gggcgataccctgttcattttca | 327903 | - | see also 5419 line |
|---|---|---|---|---|---|---|---|
| 45966 | TMEM2 | NM_013390.1 | 584 | gacgccaccgtgcattctatagt | 327860 | - | see also 5419 line |
| 45967 | TMEM7 | NM_031440.1 | 665 | ttcctacgcatgccaaaaccaca | 327988 | - | see also 20330 line |
| 45968 | TMEM7 | NM_031440.1 | 499 | ctcttgaagggccacacaatagt | 327984 | - | see also 20330 line |
| 45969 | TNMD | NM_022144.1 | 1018 | cgcgtctgtgaacctttactagg | 328012 | - | see also 8313 line |
| 45970 | TNMD | NM_022144.1 | 1037 | taggctactacccatatccatac | 328013 | - | see also 8313 line |
| 45971 | UPK1A | NM_007000.2 | 572 | gtcgccggacgggaaacttcatc | 328021 | - | see also 20332 line |
| 45972 | UPK1A | NM_007000.2 | 445 | ctctgggaccgcgtcatgattga | 328018 | - | see also 20332 line |
| 45973 | UPK3A | NM_006953.1 | 509 | cacggagtacaggttcaagtatg | 328027 | - | see also 20333 line |
| 45974 | UPK3A | NM_006953.1 | 290 | ctcaacgttcctacaaacagagg | 328025 | - | see also 20333 line |
| 45975 | VANGL1 | NM_138959.1 | 1058 | aacatgaacggcgagtaaagaag | 328053 | - | see also 9642 line |
| 45976 | VANGL1 | NM_138959.1 | 1023 | ctcaagccacaacgagttgtatt | 328049 | - | see also 9642 line |
| 45977 | ZDHHC1 | NM_013304.1 | 1506 | aggaagaggcgcgtgtataaagt | 328074 | - | see also 20335 line |
| 45978 | ZDHHC1 | NM_013304.1 | 850 | cggaactaccggctctttctaca | 328068 | - | see also 20335 line |
| 45979 | ZDHHC11 | NM_024786.1 | 865 | tgcgtgggaagccggaattattg | 328084 | - | see also 20336 line |
| 45980 | ZDHHC11 | NM_024786.1 | 1071 | gaccctgatagtcgtgatcatcg | 328089 | - | see also 20336 line |
| 45981 | ZDHHC11 | NM_024786.1 | 713 | tgcccctcttcgacagatcaaaa | 328078 | - | see also 20336 line |
| 45982 | ZDHHC12 | NM_032799.3 | 297 | ctcttcggcgctgcagatactgc | 328090 | - | see also 20337 line |
| 45983 | ZDHHC2 | NM_016353.2 | 365 | tgcctgatggcctatcatctact | 328093 | - | see also 6833 line |
| 45984 | ZDHHC4 | NM_018106.2 | 663 | aacgtgaggtgctctacttgtga | 328131 | - | see also 20339 line |
| 45985 | ZDHHC4 | NM_018106.2 | 944 | cacggtctttcttattcagtacc | 328134 | - | see also 20339 line |
| 45986 | ZDHHC5 | NM_015457.1 | 425 | tcgccggaactaccgttattttt | 328142 | - | see also 20340 line |
| 45987 | ZDHHC5 | NM_015457.1 | 424 | gtcgccggaactaccgttatttt | 328141 | - | see also 20340 line |
| 45988 | ZDHHC5 | NM_015457.1 | 1732 | gccatgcccctcgtactagttcc | 328159 | - | see also 20340 line |
| 45989 | ZDHHC6 | NM_022494.1 | 1498 | agccacaagaggtttacgatact | 328200 | - | see also 20341 line |
| 45990 | ZDHHC6 | NM_022494.1 | 557 | tggtattggcccttacatacaac | 328174 | - | see also 20341 line |
| 45991 | ZDHHC7 | NM_017740.1 | 1104 | gagatcgagcgattgaaaagtga | 328220 | - | see also 20342 line |
| 45992 | ZDHHC7 | NM_017740.1 | 989 | acctccgataactgtaatcctgt | 328217 | - | see also 20342 line |
| 45993 | ZDHHC8 | NM_013373.1 | 525 | tgcatcgggcgtcgaaactatcg | 328227 | - | see also 20343 line |
| 45994 | ZDHHC8 | NM_013373.1 | 2341 | cacggcacacgctggttaagaag | 328232 | - | see also 20343 line |
| 45995 | ZDHHC9 | NM_016032.1 | 695 | caccgcctcgtatcaagaatttc | 328241 | - | see also 20344 line |
| 45996 | ZDHHC9 | NM_016032.1 | 696 | accgcctcgtatcaagaatttcc | 328242 | - | see also 20344 line |
| 45997 | SNAP91 | NM_014841.1 | 2930 | acccattagcggatcttaacatc | 328292 | - | see also 6046 line |
| 45998 | SNAP91 | NM_014841.1 | 607 | accttcataaggcgctatagtag | 328255 | - | see also 6046 line |
| 45999 | SNAP91 | NM_014841.1 | 2931 | cccattagcggatcttaacatca | 328293 | - | see also 6046 line |
| 46000 | MAGEA1 | NM_004988.3 | 480 | tccgagcagtaatcactaagaag | 328298 | - | see also 20346 line |
| 46001 | MAGEA1 | NM_004988.3 | 444 | aggggccaagcacctcttgtatc | 328295 | - | see also 20346 line |

Figure 46

| | | | | | | | |
|---|---|---|---|---|---|---|---|
| 46002 | SSFA2 | NM_006751.3 | 823 | aagccgtttcgtcaaattgaag | 328313 | - | see also 20347 line |
| 46003 | SSFA2 | NM_006751.3 | 963 | caccttaactcgaagtaacact | 328321 | - | see also 20347 line |
| 46004 | SSFA2 | NM_006751.3 | 1588 | gtcgaaagagatcatctgttac | 328336 | - | see also 20347 line |
| 46005 | CACNA2D2 | NM_006030.1 | 789 | ttcatacgcggctgtacagatcc | 328374 | - | see also 20348 line |
| 46006 | CACNA2D2 | NM_006030.1 | 424 | gacggcgtgatgcggatttttgg | 328370 | - | see also 20348 line |
| 46007 | CACNA2D3 | NM_018398.1 | 1942 | aagcggattaagctctacctaaaa | 328469 | - | see also 7440 line |
| 46008 | CACNA2D3 | NM_018398.1 | 595 | ggcagtatccggggattaaatgg | 328430 | - | see also 7440 line |
| 46009 | CACNA2D3 | NM_018398.1 | 1398 | accagatcgaagggcattcttct | 328451 | - | see also 7440 line |
| 46010 | CACNA2D4 | NM_172364.2 | 1274 | gtccaggcggaccgagacaatcg | 328512 | - | see also 20350 line |
| 46011 | CACNA2D4 | NM_172364.2 | 1119 | tggacgtgagcggcagtatgaag | 328510 | - | see also 20350 line |
| 46012 | CKLFSF2 | NM_144673.2 | 366 | gccgctaccgtgggaattaaaa | 328561 | - | see also 20351 line |
| 46013 | CKLFSF2 | NM_144673.2 | 365 | cgccgctaccggtgggaattaaa | 328560 | - | see also 20351 line |
| 46014 | CKLFSF2 | NM_144673.2 | 367 | ccgctaccggtgggaattaaaag | 328562 | - | see also 20351 line |
| 46015 | CKLFSF3 | NM_144601.2 | 691 | tactcggatggggcttccaaagc | 328575 | - | see also 20352 line |
| 46016 | CKLFSF3 | NM_144601.2 | 647 | cggccctcatcactttgctatc | 328573 | - | see also 20352 line |
| 46017 | CKLFSF4 | NM_178818.2 | 579 | ttctttattgcttcaatcgtact | 328583 | - | see also 10209 line |
| 46018 | CKLFSF5 | NM_138460.2 | 692 | agcgcttgaccgaattaactgg | 328595 | - | see also 20354 line |
| 46019 | CKLFSF5 | NM_138460.2 | 689 | accagcgcttcgaccgaattaac | 328594 | - | see also 20354 line |
| 46020 | CKLFSF5 | NM_138460.2 | 630 | ggcgctactggagttcttcatca | 328593 | - | see also 20354 line |
| 46021 | CKLFSF6 | NM_017801.2 | 609 | tcctacttgactttatcactatg | 328607 | - | see also 20355 line |
| 46022 | CKLFSF6 | NM_017801.2 | 608 | ttcctacttgactttatcactat | 328606 | - | see also 20355 line |
| 46023 | CKLFSF6 | NM_017801.2 | 413 | ctccttatactgattgtgtattg | 328599 | - | see also 20355 line |
| 46024 | CKLFSF7 | NM_138410.2 | 476 | caccatttgcgacttgataatga | 328613 | - | see also 9617 line |
| 46025 | CKLFSF7 | NM_138410.2 | 535 | accgcgtgctcacctgtatcagc | 328614 | - | see also 9617 line |
| 46026 | DOCK4 | NM_014705.2 | 421 | gacgtgaagcggccacattactgc | 328634 | - | see also 20357 line |
| 46027 | DOCK4 | NM_014705.2 | 2555 | caegtatccttagcaacgtattt | 328684 | - | see also 20357 line |
| 46028 | FLJ30532 | NM_144724.1 | 277 | agctatagcgccagatctcaaac | 328767 | - | see also 20358 line |
| 46029 | FLJ30532 | NM_144724.1 | 1182 | ctcggagacatagagaatatatg | 328802 | - | see also 20358 line |
| 46030 | FLJ33990 | NM_153226.1 | 351 | tagagattagtgcgtttcgatgt | 328806 | - | see also 9978 line |
| 46031 | FLJ33990 | NM_153226.1 | 1113 | atgttggagcggccattcgtaaa | 328842 | - | see also 9978 line |
| 46032 | GP2 | NM_001502.1 | 307 | aggggtggcgataaaaacatgagc | 328849 | - | see also 20360 line |

1553

Figure 46

| | | | | | | | |
|---|---|---|---|---|---|---|---|
| 46033 | GP2 | NM_001502.1 | 530 | cccaggcgggtaccacgtgtacc | 328850 | - | see also 20360 line |
| 46034 | KIAA0792 | NM_014698.1 | 762 | tggacaaagaccgtatagttt | 328870 | - | see also 20361 line |
| 46035 | KIAA0792 | NM_014698.1 | 1620 | cgctgaataaccgatcatcagc | 328880 | - | see also 20361 line |
| 46036 | MAMDC2 | NM_153267.3 | 2516 | aagccattcgaggagtatcaata | 328951 | - | see also 9985 line |
| 46037 | MAMDC2 | NM_153267.3 | 2238 | tggggtaggctactacatgtaca | 328943 | - | see also 9985 line |
| 46038 | MGC3295 | NM_025246.1 | 1315 | ctctctcaatgatgatatacaga | 328974 | - | see also 20363 line |
| 46039 | MGC3295 | NM_025246.1 | 1313 | tgctctctcaatgatgattataca | 328973 | - | see also 20363 line |
| 46040 | STOML3 | NM_145286.1 | 897 | accgagaagaattctacgattgt | 329007 | - | see also 20364 line |
| 46041 | STOML3 | NM_145286.1 | 444 | accagagactccgttaactactca | 328998 | - | see also 20364 line |
| 46042 | TDE2L | NM_178865.2 | 1210 | gaggagtgcccacctactgctaga | 329016 | - | see also 20365 line |
| 46043 | TDE2L | NM_178865.2 | 255 | caccgtgagccgctcatcttca | 329012 | - | see also 20365 line |
| 46044 | TERE1 | NM_013319.1 | 764 | ctgtgccgcttgcctctactacc | 329026 | - | see also 20366 line |
| 46045 | TERE1 | NM_013319.1 | 1089 | ggccccacgttctctacattct | 329028 | - | see also 20366 line |
| 46046 | CSPG5 | NM_006574.2 | 750 | gagcctgcttgacttatacgatg | 329041 | - | see also 20367 line |
| 46047 | CSPG5 | NM_006574.2 | 777 | cacccccttgatgaatctgatt | 329046 | - | see also 20367 line |
| 46048 | NESG1 | NM_012337.1 | 1580 | ccgtgagcgccatcgatgagatca | 329071 | - | see also 20368 line |
| 46049 | NESG1 | NM_012337.1 | 1061 | accagatgtgatggagtttacc | 329068 | - | see also 20368 line |
| 46050 | FGL1 | NM_004467.2 | 1012 | gccgttatgcacaatataagaat | 329079 | - | see also 20369 line |
| 46051 | FGL1 | NM_004467.2 | 1281 | ggcccctacacaggctaaaacaga | 329087 | - | see also 20369 line |
| 46052 | CHGB | NM_001819.1 | 1279 | cccagcttagagcttgataaga | 329094 | - | see also 20370 line |
| 46053 | CHGB | NM_001819.1 | 1386 | agccacgtgcctatttcatgtct | 329097 | - | see also 20370 line |
| 46054 | CRISP2 | NM_003296.1 | 243 | ggctttactaccggtgttgttc | 329109 | - | see also 20371 line |
| 46055 | CRISP2 | NM_003296.1 | 860 | atcaagatctcctaagtaactgt | 329129 | - | see also 20371 line |
| 46056 | HFL3 | NM_005666.1 | 489 | cccaaatgcagtccacctatttc | 329133 | - | see also 20373 line |
| 46057 | HFL3 | NM_005666.1 | 773 | aacaggtgacatagttgaatttg | 329138 | - | see also 20373 line |
| 46058 | HFL3 | NM_005666.1 | 767 | ttcaagaacaggtgacatagttg | 329136 | - | see also 20375 line |
| 46059 | PRH2 | NM_005042.1 | 126 | gacgttccttgttaatatcaga | 329142 | - | see also 20376 line |
| 46060 | SCGB1D1 | NM_006552.1 | 90 | tgctgctaccggcgcaaatgcagt | 329143 | - | see also 20377 line |
| 46061 | SCGB1D2 | NM_006551.2 | 180 | acctctgttcaagttaagtcttg | 329147 | - | see also 20377 line |
| 46062 | SCGB1D2 | NM_006551.2 | 152 | agctgttagacttcttcttcatt | 329145 | - | see also 20378 line |
| 46063 | SEMG2 | NM_003008.2 | 121 | agcggatcttcccaattccaaca | 329148 | - | see also 20378 line |
| 46064 | SEMG2 | NM_003008.2 | 222 | cacatatcatgtagacatcaatg | 329149 | - | see also 20379 line |
| 46065 | ASPN | NM_017680.2 | 468 | cagtgctattcacgagttgtaca | 329158 | - | see also 20379 line |
| 46066 | ASPN | NM_017680.2 | 471 | tgctattcacgagttgtacattg | 329160 | - | see also 20380 line |
| 46067 | CRTAP | NM_006371.2 | 846 | gagaacctcaccccagtttatagg | 329203 | - | see also 20380 line |
| 46068 | CRTAP | NM_006371.2 | 157 | ggcacgcgctggacaagtacagc | 329190 | - | see also 20381 line |
| 46069 | DCN | NM_001920.2 | 239 | tctgggctggaccgtttcaaca | 329207 | - | see also 20381 line |

Figure 46

| 46070 | DCN | NM_001920.2 | 262 | gagaggcttatttgactttatgc | 329209 | - | see also 20381 line |
|---|---|---|---|---|---|---|---|
| 46071 | GPC2 | NM_152742.1 | 603 | ctctcggctgcgagacttctatg | 329237 | - | see also 20382 line |
| 46072 | GPC2 | NM_152742.1 | 1830 | gggggcatctattggttttcaca | 329240 | - | see also 20382 line |
| 46073 | C9orf19 | NM_022343.2 | 140 | aaccgggaggctcaacagtattc | 329245 | - | see also 20383 line |
| 46074 | C9orf19 | NM_022343.2 | 141 | accgggaggctcaacagtattct | 329246 | - | see also 20383 line |
| 46075 | C9orf19 | NM_022343.2 | 339 | gacacttcacggccatggtatgg | 329250 | - | see also 20383 line |
| 46076 | DKFZP434B044 | NM_031476.1 | 1035 | aacctctgcggtcaactacatga | 329263 | - | see also 20384 line |
| 46077 | DKFZP434B044 | NM_031476.1 | 279 | gcccaacgtcactctcttagagg | 329254 | - | see also 20384 line |
| 46078 | FGL2 | NM_006682.1 | 1017 | tcgtttacacgttggtaactata | 329304 | - | see also 4716 line |
| 46079 | LOC57151 | NM_020426.1 | 153 | gacaaaggcgctactcatctatt | 329318 | - | see also 20386 line |
| 46080 | LOC57151 | NM_020426.1 | 159 | ggcgctactcatctatttggtca | 329319 | - | see also 20386 line |
| 46081 | LOC83690 | NM_031461.2 | 761 | ccccgacgtttcatgtacaatcg | 329342 | - | see also 20387 line |
| 46082 | LOC83690 | NM_031461.2 | 760 | cccccgacgtttcatgtacaatc | 329341 | - | see also 20387 line |
| 46083 | MGC26768 | NM_144634.2 | 537 | ttccgctttactgaatcctaatt | 329394 | - | see also 20388 line |
| 46084 | MGC26768 | NM_144634.2 | 293 | ttccaagtggtgcttacatcttg | 329390 | - | see also 20388 line |
| 46085 | MGC39497 | NM_152436.1 | 735 | aagcgattgaacactagtttttt | 329414 | - | see also 20389 line |
| 46086 | MGC39497 | NM_152436.1 | 366 | atgtgggtcggccctgaaaatga | 329406 | - | see also 20389 line |
| 46087 | MGC39497 | NM_152436.1 | 736 | agcgattgaacactagtttttta | 329415 | - | see also 20389 line |
| 46088 | R3HDML | NM_178491.1 | 384 | ccggtccgtagtggatctcatga | 329421 | - | see also 20390 line |
| 46089 | R3HDML | NM_178491.1 | 641 | ttggcgagtccccgtacaagatg | 329425 | - | see also 20390 line |
| 46090 | SPACA3 | NM_173847.2 | 560 | gccggatgtactgctcagatttg | 329434 | - | see also 20391 line |
| 46091 | SPACA3 | NM_173847.2 | 593 | atctcaaggataccgttatctgt | 329436 | - | see also 20391 line |
| 46092 | C6orf135 | NM_024839.1 | 104 | accaggcgctggcgaggttttac | 329439 | - | see also 20392 line |
| 46093 | 182-FIP | NM_020772.1 | 991 | gaggactcgtggaccctatttaa | 378696 | - | see also 20393 line |
| 46094 | 182-FIP | NM_020772.1 | 1777 | ttggaacccagtcatataggtga | 378726 | - | see also 20393 line |
| 46095 | 24432 | NM_022914.1 | 993 | cgcaaccaagacttagatgttca | 384855 | - | see also 20394 line |
| 46096 | 24432 | NM_022914.1 | 1349 | ggctctgcaggacctatctctga | 384856 | - | see also 20394 line |
| 46097 | 24b2/STAC2 | NM_198993.1 | 1107 | tacgttgcactctacaagtttct | 440389 | - | see also 10353 line |
| 46098 | 24b2/STAC2 | NM_198993.1 | 1103 | ctcctacgttgcactctacaagt | 440388 | - | see also 10353 line |

Figure 46

| | | | | | | | |
|---|---|---|---|---|---|---|---|
| 46099 | 384D8-2 | NM_014551.3 | 476 | ccggactaacgtggatctcaaga | 340832 | - | see also 20396 line |
| 46100 | 384D8-2 | NM_014551.3 | 479 | gactaacgtggatctcaagaatg | 340833 | - | see also 20396 line |
| 46101 | 7A5 | NM_182762.1 | 488 | tcctactacacgacttagagtgg | 435157 | - | see also 20397 line |
| 46102 | 7A5 | NM_182762.1 | 826 | atgttaggcaacctcaatacaat | 435170 | - | see also 20397 line |
| 46103 | 7h3 | NM_033025.3 | 1638 | tccagatgtcgcgccttacttgc | 402613 | - | see also 20398 line |
| 46104 | 7h3 | NM_033025.3 | 1176 | tgcagcggtctgcctatctgagg | 402609 | - | see also 20398 line |
| 46105 | 7h3 | NM_033025.3 | 1935 | cccgcacctgaatctcaaagact | 402615 | - | see also 20398 line |
| 46106 | AASDHPPT | NM_015423.2 | 440 | atcgaatccttacccgaatttca | 352602 | - | see also 6406 line |
| 46107 | AASDHPPT | NM_015423.2 | 332 | tggtcgtctgatgataaggaaat | 352600 | - | see also 6406 line |
| 46108 | AAT1 | NM_033364.2 | 1074 | agcgcacgcatgtatcaacaatc | 403728 | - | see also 9414 line |
| 46109 | AAT1 | NM_033364.2 | 1071 | ttcagcgcacgcatgtatcaaca | 403726 | - | see also 9414 line |
| 46110 | ABC1 | NM_022070.3 | 1655 | tgcaccttatgatcgtctaaaac | 382101 | - | see also 20401 line |
| 46111 | ABC1 | NM_022070.3 | 255 | tgccgactggtacctcttaatca | 382062 | - | see also 20401 line |
| 46112 | ABC1 | NM_022070.3 | 254 | ttgccgactggtacctcttaatc | 382061 | - | see also 20401 line |
| 46113 | ACMSD | NM_138326.1 | 335 | ctgggatccagaagttcgtatta | 407493 | - | see also 20402 line |
| 46114 | ACMSD | NM_138326.1 | 312 | ttcagagtggtgcgagagaattg | 407492 | - | see also 20402 line |
| 46115 | ACN9 | NM_020186.1 | 40 | ctcgagtccgggcattgtacaag | 375860 | - | see also 20403 line |
| 46116 | ACN9 | NM_020186.1 | 41 | tcgagtccgggcattgtacaagc | 375861 | - | see also 20403 line |
| 46117 | ACY-3 | NM_080658.1 | 1000 | acggtgtaccccgtgttcattaa | 406563 | - | see also 20404 line |
| 46118 | ACY-3 | NM_080658.1 | 417 | caccccggacgacccatatgagg | 406560 | - | see also 20404 line |
| 46119 | AD024 | NM_020675.3 | 218 | ggcgggactaagagatacctaca | 377910 | - | see also 20405 line |
| 46120 | AD024 | NM_020675.3 | 763 | ttgccaatgttcggaaagctttt | 377920 | - | see also 20405 line |
| 46121 | AD024 | NM_020675.3 | 764 | tgccaatgttcggaaagctttta | 377921 | - | see also 20405 line |
| 46122 | AD031 | NM_032021.2 | 269 | tcctggtcaggtcacgaaaaaga | 397608 | - | see also 20406 line |
| 46123 | AD031 | NM_032021.2 | 271 | ctggtcaggtcacgaaaaagaac | 397609 | - | see also 20406 line |
| 46124 | AD158 | NM_032270.2 | 2193 | atcgtacaatgacattcgattta | 399291 | - | see also 9130 line |
| 46125 | AD158 | NM_032270.2 | 2177 | tccgatacttggacttatcgtac | 399288 | - | see also 9130 line |
| 46126 | AD158 | NM_032270.2 | 238 | ggcgtgtttggatgtactttaca | 399250 | - | see also 9130 line |
| 46127 | AD24 | NM_022451.9 | 465 | agcatgaacgcattatagataaa | 382795 | - | see also 8360 line |
| 46128 | AD24 | NM_022451.9 | 1161 | ttcacaacaacatcatcgtattg | 382809 | - | see also 8360 line |
| 46129 | ADAMTS20 | NM_025003.2 | 2221 | cccgcaggagcaacaaacgttga | 392962 | - | see also 8832 line |
| 46130 | ADAMTS20 | NM_025003.2 | 4198 | aacaagccacctagcgtaataca | 393030 | - | see also 8832 line |
| 46131 | ADAMTSL 1 | NM_052866.2 | 480 | tagatggtacgcgttgctataca | 404540 | - | see also 20410 line |

Figure 46

| | ADAMTSL1 | | | | | | |
|---|---|---|---|---|---|---|---|
| 46132 | ADAMTSL1 | NM_052866.2 | 621 | ggctggtccgagggcagrataaa | 404545 | - | see also 20410 line |
| 46133 | ADCK2 | NM_052853.2 | 977 | ctgcagaccagtcgtttctaga | 404425 | - | see also 20411 line |
| 46134 | ADCK2 | NM_052853.2 | 1256 | cagattgacctgcgttacgaagc | 404428 | - | see also 20411 line |
| 46135 | ADCK4 | NM_024876.2 | 531 | agcggattgtgcagaccttatgt | 391626 | - | see also 20412 line |
| 46136 | ADCK4 | NM_024876.2 | 981 | aggccgccttgcccagaatttc | 391629 | - | see also 20412 line |
| 46137 | ADCK5 | NM_174922.2 | 226 | tcctcgagcccgctatgtcatg | 428682 | - | see also 20413 line |
| 46138 | ADCK5 | NM_174922.2 | 53 | accggtgcagtctgtcatttcc | 428680 | - | see also 20413 line |
| 46139 | ADCK5 | NM_174922.2 | 741 | gacctgcgggaccgctttgatgg | 428684 | - | see also 20413 line |
| 46140 | ADMP | NM_145035.2 | 191 | ccctccgggaagatattcgagc | 413015 | - | see also 20414 line |
| 46141 | ADMP | NM_145035.2 | 383 | cgggcacgatcctttcatctatt | 413019 | - | see also 20414 line |
| 46142 | AE2 | NM_032264.1 | 1072 | atcctctcatgatgaatggttgg | 399140 | - | - |
| 46143 | AE2 | NM_032264.1 | 491 | ttccttggccaaccggcaaaataa | 399139 | - | see also 46142 line |
| 46144 | AE2 | NM_032264.1 | 1307 | gtcggcttggcttttgacataag | 399141 | - | see also 46142 line |
| 46145 | AEBP2 | NM_153207.2 | 733 | gacatcgagccatatgctttaac | 422135 | - | see also 9975 line |
| 46146 | AEBP2 | NM_153207.2 | 666 | aacaaaagacgacgctcattaac | 422131 | - | see also 9975 line |
| 46147 | AF15Q14 | NM_020380.2 | 3621 | aaccgtattgttcacagataatt | 377250 | - | see also 20416 line |
| 46148 | AF15Q14 | NM_020380.2 | 215 | ctgttagaagacggcattcttca | 377146 | - | see also 20416 line |
| 46149 | AF311304 | NM_031214.1 | 77 | gggggagtgggcaaaatgttgatt | 395970 | - | - |
| 46150 | AF311304 | NM_031214.1 | 75 | tggggagtgggcaaaaatgttga | 395969 | - | see also 46149 line |
| 46151 | AF311304 | NM_031214.1 | 78 | gggagtgggcaaaatgttgatta | 395971 | - | see also 46149 line |
| 46152 | AFAP | NM_021638.3 | 1701 | agcgggacggcacttcattatga | 380971 | - | see also 20417 line |
| 46153 | AFAP | NM_021638.3 | 1635 | atgaacaggcgtgttatatctgc | 380968 | - | see also 20417 line |
| 46154 | AGR2 | NM_006408.2 | 298 | tgcccacagtcaagctttaaa | 335404 | - | see also 20418 line |
| 46155 | AGR2 | NM_006408.2 | 492 | ttcaaatcgtctatgcttacg | 335409 | - | see also 20418 line |
| 46156 | AGTRAP | NM_020350.3 | 206 | atggacgccataagcatgttct | 376820 | - | see also 20419 line |
| 46157 | AGTRAP | NM_020350.3 | 202 | ctccatcgacgccataagcatgt | 376819 | - | see also 20419 line |
| 46158 | AHI1 | NM_017651.3 | 3638 | aggagcgatccctccttaagc | 360245 | - | see also 7078 line |
| 46159 | AHI1 | NM_017651.3 | 2166 | cagccggatgatatccaatta | 360185 | - | see also 7078 line |
| 46160 | AHSA1 | NM_012111.1 | 1124 | tggctatggcgcacgcttatttt | 337519 | - | see also 5140 line |
| 46161 | AIG1 | NM_016108.2 | 475 | gaggacatgcaccatcagtatc | 356282 | - | see also 20422 line |
| 46162 | AIG1 | NM_016108.2 | 639 | ttgggtctacaacatcttaatg | 356284 | - | see also 20422 line |
| 46163 | AIM1L | NM_017977.1 | 645 | ctcccggacgctgaggaatacc | 365018 | - | see also 20423 line |
| 46164 | AIM1L | NM_017977.1 | 849 | ggccgtgaaagtgctaagtaacc | 365021 | - | see also 20423 line |
| 46165 | AK7 | NM_152327.1 | 1400 | aggtcaactagacgatcaatata | 415656 | - | see also 20424 line |
| 46166 | AK7 | NM_152327.1 | 132 | agcggaaacatcgggaagtttct | 415620 | - | see also 20424 line |
| 46167 | AKNA | NM_030767.2 | 2319 | cccctgccattgcacgtaaatg | 394901 | - | see also 20425 line |

Figure 46

| 46168 | AKNA | NM_030767.2 | 977 | gtccgcagcatgccacagataga | 394894 | - | see also 20425 line |
|---|---|---|---|---|---|---|---|
| 46169 | AKR1CL2 | NM_031436.2 | 946 | aacacagcacgatatggataaca | 396964 | - | see also 20426 line |
| 46170 | AKR1CL2 | NM_031436.2 | 357 | aggccttgaagctgaactatttg | 396961 | - | see also 20426 line |
| 46171 | ALEX1 | NM_016608.1 | 777 | aggacccttgcaccgagtttacc | 358390 | - | see also 20427 line |
| 46172 | ALEX1 | NM_016608.1 | 1107 | ttgggtggtgtcccaattattgc | 358398 | - | see also 20427 line |
| 46173 | ALEX2 | NM_014782.3 | 624 | ggctaagagccggattcacaatc | 343401 | - | see also 5963 line |
| 46174 | ALEX3 | NM_016607.2 | 1026 | ctcccaattgtcgcaaagattct | 358366 | - | see also 20429 line |
| 46175 | ALEX3 | NM_016607.2 | 1123 | agcgcaggcttaaagtatacatg | 358375 | - | see also 20429 line |
| 46176 | ALEX3 | NM_016607.2 | 1120 | atcagcgcaggcttaaagtatac | 358374 | - | see also 20429 line |
| 46177 | ALG10 | NM_032834.1 | 209 | gacagacttccacctattaaagg | 401870 | - | see also 20430 line |
| 46178 | ALG10 | NM_032834.1 | 856 | cacatccagactcatttgtgaac | 401883 | - | see also 20430 line |
| 46179 | ALLC | NM_018436.2 | 855 | ctggaccggccaccaatattaga | 371501 | - | see also 20431 line |
| 46180 | ALLC | NM_018436.2 | 574 | ctgcttccggccacaactatttt | 371490 | - | see also 20431 line |
| 46181 | ALMS1 | NM_015120.2 | 6958 | gcccgtttcagagatattagtga | 348340 | - | see also 20432 line |
| 46182 | ALMS1 | NM_015120.2 | 7384 | tgctcgtgggacagtaatttacc | 348359 | - | see also 20432 line |
| 46183 | ALS2CR17 | NM_198945.1 | 2028 | ctccggcgttacaatttaagaag | 440071 | - | see also 10350 line |
| 46184 | ALS2CR17 | NM_198945.1 | 2026 | atctccggcgttacaatttaaga | 440070 | - | see also 10350 line |
| 46185 | ALS2CR19 | NM_057177.3 | 1623 | tggtcgtctgcgaatgaatgacc | 406151 | - | see also 20434 line |
| 46186 | ALS2CR19 | NM_057177.3 | 3061 | cccgcagatcctgtagactatct | 406179 | - | see also 20434 line |
| 46187 | ALS2CR4 | NM_152388.1 | 383 | tccacttagtcgtcctaagaaaa | 416122 | - | see also 20435 line |
| 46188 | ALS2CR4 | NM_152388.1 | 1184 | atcagtagcaatccgaaattttt | 416146 | - | see also 20435 line |
| 46189 | AMACO | NM_198496.1 | 482 | ttctgttagatgggtctaacagc | 438598 | - | see also 20436 line |
| 46190 | AMACO | NM_198496.1 | 505 | gtcgggaaagggagctttgaaag | 438599 | - | see also 20436 line |
| 46191 | AMACO | NM_198496.1 | 1653 | gccacgtagagtggtggttttgc | 438603 | - | see also 20436 line |
| 46192 | AMICA | NM_153206.1 | 736 | ggctgaccagggaacctatatct | 422107 | - | see also 20437 line |
| 46193 | AMICA | NM_153206.1 | 609 | aggacgaatatgtgctatactat | 422104 | - | see also 20437 line |
| 46194 | AMOT | NM_133265.1 | 1269 | accaaagacgacacatcgaaatc | 407177 | - | see also 20438 line |
| 46195 | AMOT | NM_133265.1 | 2674 | tggaccacatcgtttgtctatac | 407188 | - | see also 20438 line |
| 46196 | AMOTL1 | NM_130847.1 | 777 | ggcggtgggccatggttactaca | 407067 | - | see also 20439 line |
| 46197 | AMOTL1 | NM_130847.1 | 1536 | tgccgacaagctccacaagtttg | 407076 | - | see also 20439 line |
| 46198 | AMOTL2 | NM_016201.2 | 1447 | aagacttcaaccgggatcttaga | 356550 | - | see also 20440 line |
| 46199 | AMOTL2 | NM_016201.2 | 1449 | gacttcaaccgggatcttagaga | 356551 | - | see also 20440 line |
| 46200 | AMSH-LP | NM_020799.1 | 984 | tgcgggaccagactattgtgaca | 379068 | - | see also 20441 line |
| 46201 | AMSH-LP | NM_020799.1 | 824 | aaggactgcgatgtgtagttttg | 379065 | - | see also 20441 line |
| 46202 | ANAPC1 | NM_022662.1 | 4897 | aacgggtggtgaaatgaactatg | 383663 | - | see also 8427 line |
| 46203 | ANAPC1 | NM_022662.1 | 4600 | gccgtgctcagaggatttgaatt | 383655 | - | see also 8427 line |

Figure 46

| ID | Gene | Accession | Position | Sequence | | | Note |
|---|---|---|---|---|---|---|---|
| 46204 | ANC_2H01 | NM_016331.1 | 480 | ctctacaccttctcgttattca | 357036 | - | see also 6814 line |
| 46205 | ANGPTL5 | NM_178127.2 | 692 | tacggactcttcagtagttaaca | 430492 | - | see also 20444 line |
| 46206 | ANGPTL5 | NM_178127.2 | 1605 | aacgagtgtggctcagcaaatct | 430521 | - | see also 20444 line |
| 46207 | ANGPTL6 | NM_031917.2 | 1028 | gccgtcacgtagtgtcagtatgg | 397296 | - | see also 20445 line |
| 46208 | ANGPTL6 | NM_031917.2 | 1393 | agggaccgagactcctattctgg | 397301 | - | see also 20445 line |
| 46209 | ANKMY1 | NM_016552.1 | 1540 | gaggacgtgttgggaaaacacaga | 358101 | - | see also 20446 line |
| 46210 | ANKMY1 | NM_016552.1 | 1101 | ctgccacaacgacatttgtcaacc | 358097 | - | see also 20446 line |
| 46211 | ANKRD10 | NM_017664.1 | 940 | tccgtatcgaatacattgacaaa | 360473 | - | see also 20447 line |
| 46212 | ANKRD10 | NM_017664.1 | 579 | ggctcacgtcgacctgagaaatg | 360463 | - | see also 20447 line |
| 46213 | ANKRD13 | NM_033121.1 | 715 | tgccaaactgcgcgtcgatatca | 403383 | - | see also 20448 line |
| 46214 | ANKRD13 | NM_033121.1 | 720 | aactgcgcgtgatatcacattg | 403384 | - | see also 20448 line |
| 46215 | ANKRD15 | NM_015158.1 | 3483 | gccatgtgtggggactacatagc | 348692 | - | see also 20449 line |
| 46216 | ANKRD15 | NM_015158.1 | 1485 | aagatctatcgctagaagtaca | 348665 | - | see also 20449 line |
| 46217 | ANKRD6 | NM_014942.1 | 708 | tgctgcgctataatcactgt | 346233 | - | see also 6130 line |
| 46218 | ANKRD6 | NM_014942.1 | 703 | cacgttgctgcgcgctataatca | 346232 | - | see also 6130 line |
| 46219 | ANKS1 | NM_015245.1 | 1869 | gccgctcgcaccctgaaacttg | 350260 | - | see also 6287 line |
| 46220 | ANP32B | NM_006401.1 | 280 | ttggacaattgcaaatcaaatga | 335385 | - | see also 20451 line |
| 46221 | ANP32C | NM_012403.1 | 450 | ggccccttactcagatattgagg | 338136 | - | see also 20452 line |
| 46222 | ANP32C | NM_012403.1 | 193 | ttgagaaagcttgaactaagagt | 338135 | - | see also 20452 line |
| 46223 | ANP32D | NM_012404.2 | 225 | agcctcagtgggcctagaagtat | 338137 | - | - |
| 46224 | APBB2 | NM_173075.1 | 2505 | gccgctgcatgttacgtatatca | 424496 | - | see also 20453 line |
| 46225 | APBB2 | NM_173075.1 | 2510 | ctgcatgttacgatatcagaagt | 424498 | - | see also 20453 line |
| 46226 | APCDD1 | NM_153000.3 | 454 | ctcatcctaggtccttagagaaa | 421767 | - | see also 20454 line |
| 46227 | APCDD1 | NM_153000.3 | 1612 | cggagtacgagatcttcaaaatg | 421781 | - | see also 20454 line |
| 46228 | APG10L | NM_031482.3 | 532 | ggtccgaagtgattaaatatga | 397186 | - | see also 9038 line |
| 46229 | APG10L | NM_031482.3 | 332 | cacaacagataggtgatagttgg | 397178 | - | see also 9038 line |
| 46230 | APG16L | NM_017974.2 | 1633 | ctcccgtgatgacttgctaaaag | 364959 | - | see also 7186 line |
| 46231 | APG16L | NM_017974.2 | 1634 | tcccgtgatgacttgctaaaagt | 364960 | - | see also 7186 line |
| 46232 | APG3 | NM_022488.3 | 467 | aacggaagccgttaaagagatca | 383438 | - | see also 8412 line |
| 46233 | APG3 | NM_022488.3 | 739 | accagactccacgattatggttg | 383445 | - | see also 8412 line |
| 46234 | APG4B | NM_013325.3 | 152 | cagaaaaggacgagagatcttgtct | 338706 | - | see also 5399 line |
| 46235 | APG4B | NM_013325.3 | 73 | ctccggtttgctgagtttgaaga | 338705 | - | see also 5399 line |
| 46236 | APH-1A | NM_016022.1 | 464 | tggtctccttcggtatcatca | 355908 | - | see also 20458 line |
| 46237 | APH-1A | NM_016022.1 | 797 | tggaggtgccctccgaagtattc | 355913 | - | see also 20458 line |
| 46238 | APOBEC3D | NM_152426.1 | 226 | atcacaggcaggagcggtgtatttc | 416624 | - | see also 20459 line |

Figure 46

| | | | | | | | |
|---|---|---|---|---|---|---|---|
| 46239 | APOBEC3F | NM_145298.3 | 948 | tgcttcaccatggaagttgtaaa | 414265 | - | see also 20460 line |
| 46240 | APOBEC3F | NM_145298.3 | 1275 | gtggagatcatgggctacaaaga | 414266 | - | see also 20460 line |
| 46241 | 3.Apr | NM_016085.2 | 826 | agcgccgaaaagccaagacttca | 356256 | - | see also 20461 line |
| 46242 | 3.Apr | NM_016085.2 | 344 | tgcccgttgctgcctgaatcaga | 356247 | - | see also 20461 line |
| 46243 | APTX | NM_017692.2 | 831 | accacgccattccgagtatgagc | 360903 | - | see also 20462 line |
| 46244 | APTX | NM_017692.2 | 841 | tccgagtatgagccatgtacatc | 360904 | - | see also 20462 line |
| 46245 | APTX | NM_017692.2 | 666 | acccaaaggcccgttaccattgg | 360900 | - | see also 20462 line |
| 46246 | APXL2 | NM_133456.1 | 1585 | ctgggactgcaaatgataacatc | 407223 | - | see also 20463 line |
| 46247 | APXL2 | NM_133456.1 | 1583 | cactgggactgcaaatgataaca | 407222 | - | see also 20463 line |
| 46248 | AQR | NM_014691.1 | 1669 | ctcttccgcttagaatcaactta | 342257 | - | see also 5891 line |
| 46249 | AQR | NM_014691.1 | 1481 | ctcgtcatgaacgtcgaatttct | 342250 | - | see also 5891 line |
| 46250 | ARCH | NM_178547.2 | 26 | ggcggaagtagagtgtctaatcc | 431159 | - | - | - | - |
| 46251 | ARCH | NM_178547.2 | 378 | cccgggaagtgaaagtacttagc | 431160 | - | see also 46250 line |
| 46252 | ARG99 | NM_031920.2 | 456 | aggttttagtcggcttgttgttc | 397316 | - | see also 20466 line |
| 46253 | ARG99 | NM_031920.2 | 1391 | tacaacactggccgatacgaaga | 397338 | - | see also 20466 line |
| 46254 | ARG99 | NM_031920.2 | 323 | tggcaggtcggcagtattcctct | 397312 | - | see also 20466 line |
| 46255 | ARH | NM_015627.1 | 300 | gtcgccacggggaattatcctga | 354624 | - | see also 20467 line |
| 46256 | ARH | NM_015627.1 | 395 | tgcacgacaaggtgtttgcatac | 354625 | - | see also 20467 line |
| 46257 | ARHGAP11A | NM_014783.2 | 1808 | cagttctacaccggtatcagttc | 343437 | - | see also 5965 line |
| 46258 | ARHGAP11A | NM_014783.2 | 1701 | aagcgtacattgccagtagattc | 343428 | - | see also 5965 line |
| 46259 | ARHGAP12 | NM_018287.4 | 1105 | agggcgttgctattactataaca | 369717 | - | see also 7363 line |
| 46260 | ARHGAP12 | NM_018287.4 | 1981 | tagtagtacaatcaataatcagg | 369751 | - | see also 7363 line |
| 46261 | ARHGAP15 | NM_018460.2 | 824 | aagcgacaaaaatcgagttaaaa | 371841 | - | see also 7485 line |
| 46262 | ARHGAP15 | NM_018460.2 | 816 | tccgatacaagcgacaaaaatcg | 371840 | - | see also 7485 line |
| 46263 | ARHGAP15 | NM_018460.2 | 815 | ttccgatacaagcgacaaaaatc | 371839 | - | see also 7485 line |
| 46264 | ARHGAP18 | NM_033515.2 | 1061 | tccattgacagcgctattagaac | 404078 | - | see also 20471 line |
| 46265 | ARHGAP18 | NM_033515.2 | 1107 | atgcgaatacccttgatctttca | 404082 | - | see also 20471 line |

Figure 46

| 46266 | ARHGAP19 | NM_032900.2 | 385 | tcctcctaatcgtaatttgctga | 402482 | - | see also 20472 line |
|---|---|---|---|---|---|---|---|
| 46267 | ARHGAP19 | NM_032900.2 | 380 | ctccctcctcctaatcgtaattt | 402479 | - | see also 20472 line |
| 46268 | ARHGAP9 | NM_032496.1 | 1934 | aaccgcgtgcgcaacaaactaaa | 400420 | - | see also 20473 line |
| 46269 | ARHGAP9 | NM_032496.1 | 1935 | accgcgtgcgcaacaaactaaag | 400421 | - | see also 20473 line |
| 46270 | ARHGEF15 | NM_173728.2 | 1368 | ggcgcatgcaggagagtcttttc | 427957 | - | see also 20474 line |
| 46271 | ARHGEF15 | NM_173728.2 | 1633 | ctcggtgtatgtggattatgtgc | 427959 | - | see also 20474 line |
| 46272 | ARHGEF15 | NM_173728.2 | 2135 | ctcatcactcagcctaagagtgg | 427965 | - | see also 20474 line |
| 46273 | ARHGEF16 | NM_014448.2 | 1093 | ccggccaacgtgctaccttttcc | 340664 | - | see also 20475 line |
| 46274 | ARHGEF16 | NM_014448.2 | 1039 | gcgcggagagctgttcttagtgg | 340660 | - | see also 20475 line |
| 46275 | ARHGEF17 | NM_014786.2 | 3493 | atctattctgcctatatcgataa | 343544 | - | see also 20476 line |
| 46276 | ARHGEF17 | NM_014786.2 | 1258 | gtgtaccgctcccctagctttgg | 343527 | - | see also 20476 line |
| 46277 | ARHGEF9 | NM_015185.1 | 1654 | aacgaacgcaagcgacgtttaga | 349458 | - | see also 20477 line |
| 46278 | ARHGEF9 | NM_015185.1 | 1660 | cgcaagcgacgtttagagaatat | 349461 | - | see also 20477 line |
| 46279 | ARHGEF9 | NM_015185.1 | 1658 | aacgcaagcgacgtttagagaat | 349459 | - | see also 20477 line |
| 46280 | ARLTS1 | NM_138450.3 | 721 | cacttccgctgcttaagatcaga | 408244 | - | see also 20478 line |
| 46281 | ARLTS1 | NM_138450.3 | 719 | tgcacttccgctgcttaagatca | 408243 | - | see also 20478 line |
| 46282 | ARMC2 | NM_032131.3 | 2258 | tgcgtgtttcggaaatctctcc | 398025 | - | see also 20479 line |
| 46283 | ARMC2 | NM_032131.3 | 1630 | aaggacgtctgtaccaatattgc | 398010 | - | see also 20479 line |
| 46284 | ARMET | NM_006010.1 | 368 | cgggcgactgcgaagtttgtatt | 334572 | - | see also 20480 line |
| 46285 | ARMET | NM_006010.1 | 499 | gagaatcggttgtgctactatat | 334580 | - | see also 20480 line |
| 46286 | ARP10 | NM_181773.2 | 587 | agcgacggcttgagaggataaag | 433123 | - | see also 20481 line |
| 46287 | ARP10 | NM_181773.2 | 189 | gcccacgagaggctactttgaaa | 433117 | - | see also 20481 line |
| 46288 | ARPC5L | NM_030978.1 | 132 | gacgaatttgacgagaacaaatt | 395916 | - | see also 8987 line |
| 46289 | ARV1 | NM_022786.1 | 584 | atctagctacggaaaactcttgc | 384241 | - | see also 20483 line |
| 46290 | ARV1 | NM_022786.1 | 175 | accacggtgtgctgaagataacc | 384226 | - | see also 20483 line |
| 46291 | ASAH3 | NM_133492.1 | 157 | cagaagcgctcccgctacattta | 407248 | - | see also 20484 line |
| 46292 | ASAH3 | NM_133492.1 | 163 | cgctcccgctacatttacgttgt | 407250 | - | see also 20484 line |
| 46293 | ASAM | NM_024769.1 | 881 | tcccaaatctaggattgactaca | 390338 | - | see also 20485 line |
| 46294 | ASAM | NM_024769.1 | 880 | ctcccaaatctaggattgactac | 390337 | - | see also 20485 line |

Figure 46

| | | | | | | | |
|---|---|---|---|---|---|---|---|
| 46295 | ASB12 | NM_130388.2 | 140 | gccaggagcgttacaaacgtttc | 406891 | - | see also 9544 line |
| 46296 | ASB12 | NM_130388.2 | 287 | aggcacagacgccactttcact | 406894 | - | see also 9544 line |
| 46297 | ASB7 | NM_024708.2 | 970 | cccgcattgcacgcttgatgtta | 389460 | - | see also 20487 line |
| 46298 | ASB7 | NM_024708.2 | 972 | cgcattgcacgcttgatgttaga | 389462 | - | see also 20487 line |
| 46299 | ASB7 | NM_024708.2 | 1145 | accgcttcagctcgccattatcc | 389468 | - | see also 20487 line |
| 46300 | ASC1p100 | NM_032204.3 | 454 | aagcgcctccatcgaagtgtttt | 398649 | - | see also 9095 line |
| 46301 | ASCL3 | NM_020646.1 | 471 | tgcgatcaagtacattaactacc | 377851 | - | see also 20488 line |
| 46302 | ASCL3 | NM_020646.1 | 468 | agctgcgatcaagtacattaact | 377849 | - | see also 20488 line |
| 46303 | ASF1B | NM_018154.1 | 595 | ccgggtgacccgcttccatatca | 367776 | - | see also 20489 line |
| 46304 | ASF1B | NM_018154.1 | 594 | cccgggtgacccgcttccatatc | 367775 | - | see also 20489 line |
| 46305 | ASH2L | NM_004674.1 | 1133 | ttgtatgaacgggttttgttagc | 332729 | - | see also 3208 line |
| 46306 | ASH2L | NM_004674.1 | 195 | aggcaaacttggtcgatgtaagc | 332704 | - | see also 3208 line |
| 46307 | ASMTL | NM_004192.1 | 563 | ccgtacacggggactttctgaac | 332460 | - | see also 20490 line |
| 46308 | ASMTL | NM_004192.1 | 109 | gtggtcccctccaagtttaaaga | 332457 | - | see also 20490 line |
| 46309 | ASP | NM_031916.2 | 779 | taccgctacttggccagattaga | 397287 | - | see also 20491 line |
| 46310 | ASP | NM_031916.2 | 872 | aacggcatgataggtctttcaga | 397293 | - | see also 20491 line |
| 46311 | ASPM | NM_018136.2 | 2270 | ctgctcggtgtaggttaaacaga | 367262 | - | see also 7271 line |
| 46312 | ASPM | NM_018136.2 | 2373 | taggcggttaattgttcgaaaag | 367266 | - | see also 7271 line |
| 46313 | ASTN2 | NM_014010.2 | 2691 | accaaggtctccgactatattct | 339039 | - | see also 5454 line |
| 46314 | ASTN2 | NM_014010.2 | 2428 | accgtgtggtgccactttatacc | 339037 | - | see also 5454 line |
| 46315 | ASTN2 | NM_014010.2 | 2885 | gcccgacggtctctacaagttca | 339041 | - | see also 5454 line |
| 46316 | ASXL2 | NM_018263.2 | 4265 | agggcacgccttcgaaatgttac | 369509 | - | see also 7348 line |
| 46317 | ASXL2 | NM_018263.2 | 2658 | agcaggtcctagtaagaatatac | 369460 | - | see also 7348 line |
| 46318 | ATCAY | NM_033064.1 | 390 | cacgctccggatggaaaacgtgg | 402757 | - | see also 20495 line |
| 46319 | ATCAY | NM_033064.1 | 1324 | gactgcgtcctgcaatacgaaga | 402767 | - | see also 20495 line |
| 46320 | ATF7IP2 | NM_024997.2 | 293 | atgttccaagcggtaatcagagt | 392828 | - | see also 20496 line |
| 46321 | ATF7IP2 | NM_024997.2 | 1136 | agcaggagatctatagcataaat | 392843 | - | see also 20496 line |
| 46322 | ATOH7 | NM_145178.2 | 624 | cccagtggggccaggataaaaag | 413757 | - | - | - | - |
| 46323 | ATOH7 | NM_145178.2 | 628 | gtggggccaggataaaaagctgt | 413758 | - | see also 46322 line |
| 46324 | ATP9B | NM_198531.2 | 590 | tactatgacacggggaagcaattg | 439242 | - | see also 10338 line |
| 46325 | ATP9B | NM_198531.2 | 631 | agcgtgacaaggaagtgaattca | 439244 | - | see also 10338 line |
| 46326 | ATP9B | NM_198531.2 | 349 | agctgaaagctcgcacagtatgg | 439238 | - | see also 10338 line |
| 46327 | ATPAF1 | NM_022745.1 | 905 | acgggttagtggagacctttaac | 383869 | - | see also 20498 line |
| 46328 | ATPAF1 | NM_022745.1 | 906 | cgggttagtggagacctttaacc | 383870 | - | see also 20498 line |
| 46329 | ATPAF2 | NM_145691.2 | 535 | atccgggcagccgtgaagtttct | 414692 | - | see also 20499 line |
| 46330 | ATPAF2 | NM_145691.2 | 732 | cagccacctggcatcttacaaca | 414697 | - | see also 20499 line |
| 46331 | AUTL1 | NM_032852.2 | 1117 | aacaccgactacttagaatttgt | 402059 | - | see also 20500 line |

Figure 46

| 46332 | AUTL1 | NM_032852.2 | 1113 | aaccaacaccgactacttagaat | 402057 | - | see also 20500 line |
|---|---|---|---|---|---|---|---|
| 46333 | AUTL2 | NM_052936.2 | 1002 | aacggttaatgaccagactttcc | 405437 | - | see also 20501 line |
| 46334 | AUTL2 | NM_052936.2 | 275 | ctcgtctatggtttacatacaga | 405417 | - | see also 20501 line |
| 46335 | AUTL2 | NM_052936.2 | 715 | gacaggcctcccgattctttaac | 405427 | - | see also 20501 line |
| 46336 | AUTL4 | NM_032885.4 | 920 | cccgcctggtggtgtacgtttct | 402435 | - | see also 20502 line |
| 46337 | AUTL4 | NM_032885.4 | 1131 | accgcgacactcactgtacttca | 402437 | - | see also 20502 line |
| 46338 | AUTL4 | NM_032885.4 | 349 | aagtacggttgggtggttaaaag | 402430 | - | see also 20502 line |
| 46339 | AXOT | NM_022826.1 | 1091 | ttcacagtcccgtagtaatgtac | 384302 | - | see also 8481 line |
| 46340 | AXOT | NM_022826.1 | 1921 | ctgtaaccacctgtgaactatgt | 384319 | - | see also 8481 line |
| 46341 | AZ2 | NM_022461.1 | 661 | tgcatatcgagaggtttgcattg | 382959 | - | see also 20504 line |
| 46342 | AZ2 | NM_022461.1 | 593 | aagctaatagctcgatttgaaga | 382953 | - | see also 20504 line |
| 46343 | B29 | NM_031939.2 | 944 | tgcagttcttctacgcaaataaa | 397415 | - | see also 9059 line |
| 46344 | B29 | NM_031939.2 | 672 | gagatactcggcctttgttttgt | 397410 | - | see also 9059 line |
| 46345 | B3GTL | NM_194318.1 | 1475 | aaggttttcgagaggagttataa | 436834 | - | see also 20506 line |
| 46346 | B3GTL | NM_194318.1 | 1364 | ctcatcaagttcccatatcgttc | 436831 | - | see also 20506 line |
| 46347 | B4GalNac-T3 | NM_173593.2 | 1864 | ttcgagtacgtacctgtgtttga | 426395 | - | see also 20507 line |
| 46348 | B4GalNac-T3 | NM_173593.2 | 2168 | tgcggctctcggagtatgtgtct | 426401 | - | see also 20507 line |
| 46349 | B7-H4 | NM_024626.1 | 485 | gggaatgctaaccttgagtataa | 388229 | - | see also 20508 line |
| 46350 | B7-H4 | NM_024626.1 | 442 | tgctggcacctacaaatgttata | 388226 | - | see also 20508 line |
| 46351 | BAALC | NM_024812.1 | 547 | gtcaccattaatgtaacagatag | 390960 | - | see also 20509 line |
| 46352 | BAALC | NM_024812.1 | 588 | aagtcgaagaatcacaaagaact | 390962 | - | see also 20509 line |
| 46353 | BAIAP3 | NM_003933.3 | 3219 | gacgctgcaccctgtatacgacg | 332295 | - | see also 20510 line |
| 46354 | BAIAP3 | NM_003933.3 | 3118 | ctggacgccaacggcttaagtga | 332293 | - | see also 20510 line |
| 46355 | BARHL2 | NM_020063.1 | 191 | ctccctgttcggagattgatacc | 375568 | - | see also 20512 line |
| 46356 | BARHL2 | NM_020063.1 | 975 | tccatcgccttatttctatcacc | 375573 | - | see also 20512 line |
| 46357 | BASE | NM_173859.1 | 557 | tcgccccagcgcaaaatttgttg | 428523 | - | see also 20513 line |
| 46358 | BASE | NM_173859.1 | 554 | aactcgccccagcgcaaaatttg | 428522 | - | see also 20513 line |
| 46359 | BASE | NM_173859.1 | 560 | ccccagcgcaaaatttgttgtgg | 428525 | - | see also 20513 line |
| 46360 | BAT2 | NM_004638.2 | 3089 | ggcttcggagggactattcgtat | 332684 | - | see also 3150 line |
| 46361 | BAT2 | NM_004638.2 | 4519 | agccgtcaagggagtgtaactgc | 332690 | - | see also 3150 line |
| 46362 | BAT2 | NM_004638.2 | 2412 | tccaccggtggatccaaagttgg | 332678 | - | see also 3150 line |
| 46363 | BBC3 | NM_014417.2 | 780 | ccctggagggtcctgtacaatct | 340558 | - | see also 20515 line |
| 46364 | BBC3 | NM_014417.2 | 785 | gagggtcctgtacaatctcatca | 340560 | - | see also 20515 line |
| 46365 | BBP | NM_032027.2 | 630 | gcccatatcttgccgaaatgtaa | 397649 | - | see also 20516 line |
| 46366 | BBP | NM_032027.2 | 425 | ggggcgaggagtcgcttaagtgc | 397640 | - | see also 20516 line |

EP 1 752 536 A1

Figure 46

| 46367 | BBS1 | NM_024649.4 | 891 | gggacttatccgggtacacaagg | 388507 | - | see also 20517 line | | |
|---|---|---|---|---|---|---|---|---|---|
| 46368 | BBS1 | NM_024649.4 | 1219 | aagcgtacagcagtgtttgtaga | 388512 | - | see also 20517 line | | |
| 46369 | BBS4 | NM_033028.2 | 50 | gtcgcgacgagaactcaatttcc | 402619 | - | see also 20518 line | | |
| 46370 | BBS4 | NM_033028.2 | 242 | atctatgtccaagcattgatatt | 402626 | - | see also 20518 line | | |
| 46371 | BBS7 | NM_018190.2 | 811 | gacggaaaacttgcgcttataca | 368258 | - | see also 7309 line | | |
| 46372 | BBS7 | NM_018190.2 | 802 | gggacatcagacggaaaacttgc | 368257 | - | see also 7309 line | | |
| 46373 | BBS7 | NM_018190.2 | 1473 | ttctgagtcaaacgacaacttcc | 368282 | - | see also 7309 line | | |
| 46374 | BC002942 | NM_033200.1 | 1528 | gacggagatcgagttcatgtaca | 403571 | - | see also 20520 line | | |
| 46375 | BC002942 | NM_033200.1 | 873 | tgctgcaggtcctgattatcatc | 403569 | - | see also 20520 line | | |
| 46376 | BC008967 | NM_033201.1 | 475 | ttggtcgccatcccagaaaaaga | 403573 | - | see also 9378 line | | |
| 46377 | BCAS1 | NM_003657.1 | 333 | tgcggatgccaacggaaagaatc | 331916 | - | see also 2477 line | | |
| 46378 | BCL11A | NM_018014.2 | 418 | atcgagcacaaacggaaacaatg | 365299 | - | see also 7199 line | | |
| 46379 | BCL11B | NM_022898.1 | 2202 | ggcgtcgcggcacttcatgaagg | 384706 | - | see also 8519 line | | |
| 46380 | BCL11B | NM_022898.1 | 2685 | cggcgagcacttgctgactaacg | 384707 | - | see also 8519 line | | |
| 46381 | BCL9L | NM_182557.1 | 1670 | tccctcgcagttcgtatatgtct | 434428 | - | see also 20525 line | | |
| 46382 | BCL9L | NM_182557.1 | 1673 | ctcgcagttcgtatatgtcttca | 434429 | - | see also 20525 line | | |
| 46383 | BCMP11 | NM_176813.3 | 507 | ctcgggatttacccctattgata | 429707 | - | see also 20526 line | | |
| 46384 | BCMP11 | NM_176813.3 | 251 | aagccattaatggttattcatca | 429694 | - | see also 20526 line | | |
| 46385 | BCNP1 | NM_173544.2 | 1199 | ggcggctgaggacggatatcagg | 425771 | - | see also 20527 line | | |
| 46386 | BCNP1 | NM_173544.2 | 2210 | ttcggcctaccccaagacaatga | 425779 | - | see also 20527 line | | |
| 46387 | BCNP1 | NM_173544.2 | 603 | gacttccagcgagaatatctcc | 425767 | - | see also 20527 line | | |
| 46388 | BET1L | NM_016526.3 | 236 | gcccacggtgtgcgtgtatcagt | 358047 | - | - | | - |
| 46389 | BET1L | NM_016526.3 | 313 | tgcgggtgggtggtcaaatcacc | 358048 | - | see also 46388 line | | |
| 46390 | BEX1 | NM_018476.2 | 279 | ccctttggatgctggtgaatact | 372008 | - | - | | - |
| 46391 | BGALT15 | NM_198540.1 | 1866 | agcattcggctctggaaacaact | 439314 | - | see also 20529 line | | |
| 46392 | BGALT15 | NM_198540.1 | 1424 | ctgccccaccgtgagttttgtct | 439310 | - | see also 20529 line | | |
| 46393 | BHD | NM_144606.3 | 1327 | tgctccgaccgaggataccttgg | 410253 | - | see also 9715 line | | |
| 46394 | BHD | NM_144606.3 | 766 | tgcagggcacccgggatatatca | 410246 | - | see also 9715 line | | |
| 46395 | BHLHB5 | NM_152414.2 | 966 | ggcgctgcggcttaacatcaatg | 416441 | - | see also 20531 line | | |
| 46396 | BHLHB5 | NM_152414.2 | 1322 | cggagaagtgcgccctgtttaac | 416442 | - | see also 20531 line | | |
| 46397 | BIA2 | NM_015431.2 | 1294 | gactatgaagccggtgaaatttc | 352755 | - | see also 20532 line | | |
| 46398 | BIA2 | NM_015431.2 | 1269 | tcctcgctgcattgggattttct | 352753 | - | see also 20532 line | | |
| 46399 | BICC1 | NM_025044.2 | 438 | tgctataccacaccttatgattc | 393207 | - | see also 20533 line | | |
| 46400 | BICC1 | NM_025044.2 | 1404 | tggcagccctaagcgtaaacaaa | 393233 | - | see also 20533 line | | |
| 46401 | BITE | NM_024491.1 | 759 | ttcctcataccgtcttggataga | 386382 | - | see also 20534 line | | |
| 46402 | BITE | NM_024491.1 | 471 | aagaacaacgagctaatgacttg | 386372 | - | see also 20534 line | | |
| 46403 | BIVM | NM_017693.2 | 909 | accaggcgacctcaatctataaa | 360914 | - | see also 7114 line | | |

Figure 46

| 46404 | BIVM | NM_017693.2 | 911 | caggcgacctcaatctataaaac | 360915 | - | see also 7114 line |
|---|---|---|---|---|---|---|---|
| 46405 | BIVM | NM_017693.2 | 1289 | tgcataagccgaccacagtataa | 360930 | - | see also 7114 line |
| 46406 | BLP1 | NM_031940.2 | 57 | gtggttgcccggttagttactta | 397418 | - | see also 20536 line |
| 46407 | BLP1 | NM_031940.2 | 61 | ttgcccggttagttacttacttc | 397420 | - | see also 20536 line |
| 46408 | BLP2 | NM_025141.2 | 503 | tacggctctggctctaagcatca | 393970 | - | see also 20537 line |
| 46409 | BLP2 | NM_025141.2 | 124 | tcccaccttatgtgatgaagtgt | 393957 | - | see also 20537 line |
| 46410 | BM-009 | NM_016623.2 | 1186 | aaggaccaacctcctaatagtgt | 358556 | - | see also 6955 line |
| 46411 | BM-009 | NM_016623.2 | 869 | tggcaaatcgaatgtctttgttt | 358545 | - | see also 6955 line |
| 46412 | BM039 | NM_018455.3 | 1180 | acctacctacgtggtgtactact | 371787 | - | see also 20538 line |
| 46413 | BM039 | NM_018455.3 | 906 | aggaaaagcgtgcaagtatcagt | 371782 | - | see also 20538 line |
| 46414 | BM039 | NM_018455.3 | 1295 | ctccgacaagaggagatcatttt | 371788 | - | see also 20538 line |
| 46415 | BMF | NM_033503.2 | 550 | caccagcagaaccaaaatcgtgt | 404037 | - | - | | - |
| 46416 | BMF | NM_033503.2 | 364 | gaggaaccccagcgactctttta | 404036 | - | see also 46415 line |
| 46417 | BNF1 | NM_015424.3 | 1333 | ctcggacttggtggagatctacc | 352624 | - | see also 20539 line |
| 46418 | BNIPL | NM_138278.1 | 853 | ggctgggccgaggttgtatgtgg | 407355 | - | see also 20540 line |
| 46419 | BNIPL | NM_138278.1 | 1184 | aagctggatacgtcagtgttacc | 407361 | - | see also 20540 line |
| 46420 | BOMB | NM_024949.3 | 1628 | accagccgttagtagattctata | 392585 | - | see also 20541 line |
| 46421 | BOMB | NM_024949.3 | 1627 | aaccagccgttagtagattctat | 392584 | - | see also 20541 line |
| 46422 | BRI3BP | NM_080626.5 | 641 | ttccatgtcctgcgtgtacatcc | 406510 | - | see also 20543 line |
| 46423 | BRI3BP | NM_080626.5 | 382 | ttgtgctgggagtggatatgttc | 406507 | - | see also 20543 line |
| 46424 | BRIX | NM_018321.2 | 281 | tggatcgtaaggataagctattt | 370307 | - | see also 7404 line |
| 46425 | BRMS1L | NM_032352.2 | 905 | tggtatatacgtggacaaacaat | 399909 | - | see also 20545 line |
| 46426 | BRMS1L | NM_032352.2 | 614 | gaggataggcacagcattgatat | 399900 | - | see also 20545 line |
| 46427 | BRRN1 | NM_015341.2 | 389 | cacgcagattacggaacattact | 351716 | - | see also 20546 line |
| 46428 | BRRN1 | NM_015341.2 | 587 | tgccgtccatgccgatgtataca | 351723 | - | see also 20546 line |
| 46429 | BSND | NM_057176.2 | 587 | gccacatccagatgaaagtcatg | 406118 | - | see also 20547 line |
| 46430 | BSND | NM_057176.2 | 494 | ggcttgctgccgagatgaagagc | 406117 | - | see also 20547 line |
| 46431 | BTLA | NM_181780.1 | 755 | aaccaggcattgtttatgcttcc | 433188 | - | see also 20548 line |
| 46432 | BTLA | NM_181780.1 | 707 | tgcaggaagggtctgaagtttgt | 433187 | - | see also 20548 line |
| 46433 | BTN2A2 | NM_006995.3 | 533 | tgggtctaagcccctcattgaaa | 336709 | - | see also 20549 line |
| 46434 | BTN2A2 | NM_006995.3 | 933 | atctgttgcatcaagaaacttca | 336711 | - | see also 20549 line |
| 46435 | BTN2A3 | NM_024018.1 | 743 | tcctgcctgttatcatgattatt | 385468 | - | see also 20550 line |
| 46436 | BTN2A3 | NM_024018.1 | 1199 | gggccctgacctcccttaagtgg | 385472 | - | see also 20550 line |
| 46437 | BTN3A3 | NM_006994.3 | 1913 | cacgcactgaagcactttactga | 336708 | - | see also 20551 line |
| 46438 | BTN3A3 | NM_006994.3 | 1792 | ccgggcttagctaatgaaagtgg | 336705 | - | see also 20551 line |
| 46439 | BTNL2 | NM_019602.1 | 1132 | cagccgatgtgctcttcagatgg | 375445 | - | see also 20552 line |
| 46440 | BTNL2 | NM_019602.1 | 402 | aacaagcttgctgctcaaagtag | 375441 | - | see also 20552 line |

Figure 46

| | | | | | | | |
|---|---|---|---|---|---|---|---|
| 46441 | BVES | NM_007073.2 | 313 | aggatgtaccctttatatcgtct | 336892 | - | see also 20553 line |
| 46442 | BVES | NM_007073.2 | 464 | ggcatgtaccggcgattgtttga | 336904 | - | see also 20553 line |
| 46443 | BXMAS2-10 | NM_145016.2 | 782 | atcctctttataccggaattacc | 412657 | | see also 20554 line |
| 46444 | BXMAS2-10 | NM_145016.2 | 785 | ctctttatacggaattaccaaa | 412658 | | see also 20554 line |
| 46445 | BXMAS2-10 | NM_145016.2 | 653 | gtcctgtcatactccaatgtaat | 412652 | - | see also 20554 line |
| 46446 | C10orf12 | NM_015652.2 | 2755 | cccggtctcagtcattgtgaag | 354907 | - | see also 20555 line |
| 46447 | C10orf12 | NM_015652.2 | 3088 | cagccagtacccgattcttaga | 354915 | - | see also 20555 line |
| 46448 | C10orf13 | NM_152429.2 | 899 | ggcactccctgcaacttcttt | 416677 | - | - |
| 46449 | C10orf13 | NM_152429.2 | 901 | cactccctgcaacttcttgt | 416678 | - | see also 46448 line |
| 46450 | C10orf23 | NM_152643.3 | 223 | ctgaccttcaccaagatctaca | 419450 | - | see also 20556 line |
| 46451 | C10orf23 | NM_152643.3 | 1004 | ctgattgcaaaatcttgctatga | 419454 | - | see also 20556 line |
| 46452 | C10orf24 | NM_152709.2 | 1152 | cagttgggtgaacccttaaga | 419967 | - | see also 20557 line |
| 46453 | C10orf24 | NM_152709.2 | 326 | ctgacaatcgggcataagtatcc | 419942 | - | see also 20557 line |
| 46454 | C10orf25 | NM_145022.1 | 44 | accgaaagcgttgttcgttct | 412799 | - | see also 20558 line |
| 46455 | C10orf25 | NM_145022.1 | 180 | agcctctcaacctagaaaactgc | 412800 | - | see also 20558 line |
| 46456 | C10orf27 | NM_152710.1 | 1307 | gtcaatcccgaagaaaacgaag | 420000 | - | see also 20559 line |
| 46457 | C10orf27 | NM_152710.1 | 519 | cccagggattggatttcgagc | 419996 | - | see also 20559 line |
| 46458 | C10orf27 | NM_152710.1 | 1312 | tccccgaagaaacgaagatatc | 420001 | - | see also 20559 line |
| 46459 | C10orf28 | NM_014472.2 | 1819 | ggcattgacctggtagtactg | 340783 | - | see also 20560 line |
| 46460 | C10orf28 | NM_014472.2 | 498 | accggaggctcgaagactaaata | 340743 | - | see also 20560 line |
| 46461 | C10orf29 | NM_152586.2 | 3267 | tcccatacgtgtgtaacctatcc | 418304 | - | see also 20561 line |
| 46462 | C10orf29 | NM_152586.2 | 3266 | ttcccatacgtgtgtaacctatc | 418303 | - | see also 20561 line |
| 46463 | C10orf29 | NM_152586.2 | 2305 | tccacaggcaaggtttacctaaa | 418278 | - | see also 20561 line |
| 46464 | C10orf3 | NM_018131.3 | 325 | ggggatcgaagcctagtaactcc | 367100 | - | see also 20562 line |
| 46465 | C10orf3 | NM_018131.3 | 1029 | gaggttgaacgacaaaccataac | 367129 | - | see also 20562 line |
| 46466 | C10orf30 | NM_152751.1 | 529 | cagtatggcaccgttctagaac | 420578 | - | see also 20563 line |
| 46467 | C10orf30 | NM_152751.1 | 1330 | atcgcggacagtgatgaaagact | 420592 | - | see also 20563 line |
| 46468 | C10orf32 | NM_144591.1 | 107 | caactggtactgacgtaatcg | 410088 | - | see also 20564 line |
| 46469 | C10orf32 | NM_144591.1 | 213 | gccatcttgcactcagaagatag | 410089 | - | see also 20564 line |
| 46470 | C10orf36 | NM_194298.1 | 1256 | ggcctagccttgtgtcaattcc | 436540 | - | see also 20565 line |
| 46471 | C10orf36 | NM_194298.1 | 1233 | atgttgctaccttaatcatcatg | 436539 | - | see also 20565 line |
| 46472 | C10orf39 | NM_194303.1 | 98 | ctggacgcagcgcagaagttaa | 436673 | - | see also 20566 line |
| 46473 | C10orf39 | NM_194303.1 | 926 | ctggctcagcgaagaattaaaga | 436676 | - | see also 20566 line |
| 46474 | C10orf4 | NM_145246.2 | 585 | gagacttgctaagaaatactatg | 413843 | - | see also 20567 line |

Figure 46

| | | | | | | | |
|---|---|---|---|---|---|---|---|
| 46475 | C10orf4 | NM_145246.2 | 390 | aaggtttcatctcattagctatgg | 413835 | - | see also 20567 line |
| 46476 | C10orf42 | NM_138357.1 | 539 | cagcaacgctgaatgatgtaaag | 407583 | - | see also 20568 line |
| 46477 | C10orf42 | NM_138357.1 | 496 | cacgtacgaccaccaaaagaga | 407580 | - | see also 20568 line |
| 46478 | C10orf45 | NM_031453.2 | 493 | ggcagtgcgaatcggaattcaag | 397064 | - | see also 20569 line |
| 46479 | C10orf45 | NM_031453.2 | 838 | tcccggaaccatcaagatcttca | 397079 | - | see also 20569 line |
| 46480 | C10orf45 | NM_031453.2 | 762 | ggccgagccagactacatagaag | 397072 | - | see also 20569 line |
| 46481 | C10orf46 | NM_153810.2 | 942 | gtgactatatccccatatcctat | 423908 | - | see also 20570 line |
| 46482 | C10orf46 | NM_153810.2 | 1005 | agcactcggaacagatgtatagt | 423913 | - | see also 20570 line |
| 46483 | C10orf47 | NM_153256.2 | 737 | ctcgttatggcgcagaagattc | 422674 | - | - |
| 46484 | C10orf47 | NM_153256.2 | 270 | ctgctccagaagcttcacttg | 422673 | - | see also 46483 line |
| 46485 | C10orf5 | NM_178816.2 | 142 | aacttcccaaggtatcattata | 431373 | - | see also 20571 line |
| 46486 | C10orf5 | NM_178816.2 | 183 | cacaccacactcaaatatatac | 431375 | - | see also 20571 line |
| 46487 | C10orf6 | NM_018121.2 | 2131 | aggccgattctaatgtatcttca | 366896 | - | see also 7265 line |
| 46488 | C10orf6 | NM_018121.2 | 2123 | gactaataaggccgattctaatg | 366894 | - | see also 7265 line |
| 46489 | C10orf8 | NM_012071.1 | 134 | gacgaggccgttgttagatcatcc | 337470 | - | see also 5109 line |
| 46490 | C10orf8 | NM_012071.1 | 130 | ggcgacgaggccgtgttagatc | 337468 | - | see also 5109 line |
| 46491 | C11orf10 | NM_014206.1 | 191 | tacactcgtgatatctataaga | 339971 | - | see also 20574 line |
| 46492 | C11orf16 | NM_020643.1 | 838 | atcactgggcgcatcactaatga | 377829 | - | see also 20575 line |
| 46493 | C11orf16 | NM_020643.1 | 840 | cactgggcgcatcactaatgage | 377830 | - | see also 20575 line |
| 46494 | C11orf16 | NM_020643.1 | 1389 | ctgaaagcaacccaccatgaagc | 377841 | - | see also 20575 line |
| 46495 | C11orf23 | NM_018312.2 | 1500 | gacacctaacgaggatagctaac | 370134 | - | see also 7389 line |
| 46496 | C11orf24 | NM_022338.1 | 1701 | cacccagtggactacttaatca | 382600 | - | see also 20577 line |
| 46497 | C11orf24 | NM_022338.1 | 1582 | caggccgtgtagacaaaactct | 382599 | - | see also 20577 line |
| 46498 | C11orf24 | NM_022338.1 | 1703 | ccaggttgactacttaatcaac | 382601 | - | see also 20577 line |
| 46499 | C11orf30 | NM_020193.2 | 322 | ccccaaacgcctttactgtaac | 375951 | - | see also 7794 line |
| 46500 | C11orf33 | NM_017678.1 | 728 | gagaggaaaactcatatacctaa | 360572 | - | see also 7108 line |
| 46501 | C11orf33 | NM_017678.1 | 730 | gaggaaaactcatatacctaatg | 360573 | - | see also 7108 line |
| 46502 | C11orf33 | NM_017678.1 | 850 | agcttgctgtttggatttggatagg | 360575 | - | see also 7108 line |
| 46503 | C11orf5 | NM_014205.1 | 1077 | ggcctgaccaacgaaaaatgagg | 339969 | - | - |
| 46504 | C11orf9 | NM_013279.1 | 1091 | ggcgaccctgtacgatgctaact | 338643 | - | see also 20580 line |
| 46505 | C11orf9 | NM_013279.1 | 1094 | gaccctgtacgatgctaactaca | 338644 | - | see also 20580 line |
| 46506 | C11orf9 | NM_013279.1 | 1837 | tgcggctggtgcactacagatac | 338655 | - | see also 20580 line |
| 46507 | C12orf10 | NM_021640.1 | 608 | tgcacgagttgctcgacttaatc | 381008 | - | see also 20581 line |
| 46508 | C12orf10 | NM_021640.1 | 317 | ccctcggagacaccgatatgacc | 381003 | - | see also 20581 line |
| 46509 | C12orf22 | NM_030809.1 | 598 | ctctgtacggagctatacactct | 395261 | - | see also 20582 line |
| 46510 | C12orf22 | NM_030809.1 | 641 | gaggtgaaccatcgagagattct | 395263 | - | see also 20582 line |
| 46511 | C12orf4 | NM_020374.2 | 1552 | aaggagagcggaacttgtgttca | 377138 | - | see also 7854 line |

Figure 46

| 46512 | C12orf4 | NM_020374.2 | 1703 | atcttccgagtatcatcaacact | 377145 | - | see also 7854 line |
|-------|---------|-------------|------|-------------------------|--------|---|--------------------|
| 46513 | C12orf6 | NM_020367.2 | 626 | atggatcgcaaccgaattaaaag | 376994 | - | see also 7850 line |
| 46514 | C12orf6 | NM_020367.2 | 1134 | atcctgagtacttgatagacttt | 377012 | - | see also 7850 line |
| 46515 | C13orf1 | NM_020456.1 | 501 | tacttatgaccatgtcgaattaa | 377668 | - | see also 20584 line |
| 46516 | C13orf1 | NM_020456.1 | 271 | gccagcgcacctttacatcaaaa | 377663 | - | see also 20584 line |
| 46517 | C13orf1 | NM_020456.1 | 273 | cagcgcacctttacatcaaaaca | 377664 | - | see also 20584 line |
| 46518 | C13orf12 | NM_015932.2 | 290 | gtctatttgctccgctaaaatta | 355502 | - | see also 20585 line |
| 46519 | C13orf12 | NM_015932.2 | 203 | tgcctagtcatccccttgaatta | 355499 | - | see also 20585 line |
| 46520 | C13orf12 | NM_015932.2 | 158 | atgatcttcttcggaaaggtttt | 355498 | - | see also 20585 line |
| 46521 | C13orf17 | NM_018185.1 | 87 | gcgagagaactgctatctactgc | 368148 | - | see also 20586 line |
| 46522 | C13orf17 | NM_018185.1 | 265 | tggagtcgatgggattcaacagt | 368152 | - | see also 20586 line |
| 46523 | C13orf18 | NM_025113.1 | 1583 | ctccggtactgcgaatacctagg | 393715 | - | see also 20587 line |
| 46524 | C13orf18 | NM_025113.1 | 591 | atggctcgagtgaaaagagtagc | 393683 | - | see also 20587 line |
| 46525 | C13orf3 | NM_145061.2 | 505 | tacatcgtatcccaagttctacc | 413369 | - | see also 9770 line |
| 46526 | C13orf3 | NM_145061.2 | 983 | aagatcgtacttcgttggtttta | 413390 | - | see also 9770 line |
| 46527 | C13orf3 | NM_145061.2 | 494 | gacttgagcggtacatcgtatcc | 413368 | - | see also 9770 line |
| 46528 | C13orf7 | NM_024546.2 | 317 | aagcatcttcggaaaactagact | 387013 | - | see also 8637 line |
| 46529 | C13orf7 | NM_024546.2 | 362 | gaggacgaaatagattgtttaca | 387020 | - | see also 8637 line |
| 46530 | C13orf9 | NM_016075.1 | 826 | accgagctcgaggaatggaattg | 356200 | - | see also 6652 line |
| 46531 | C13orf9 | NM_016075.1 | 289 | ctggcccattccagagtagtaag | 356185 | - | see also 6652 line |
| 46532 | C14orf100 | NM_016475.2 | 436 | ttcgttcatgtcgagtattgatg | 357673 | - | see also 6872 line |
| 46533 | C14orf100 | NM_016475.2 | 437 | tcgttcatgtcgagtattgatgc | 357674 | - | see also 6872 line |
| 46534 | C14orf101 | NM_017799.2 | 360 | tgcctaccgcgtcaatcttctct | 362609 | - | see also 20592 line |
| 46535 | C14orf101 | NM_017799.2 | 1022 | tagcaacaaagtggaacgattct | 362627 | - | see also 20592 line |
| 46536 | C14orf103 | NM_018036.4 | 1525 | ctccggctaaaagttatattagt | 365632 | - | see also 20593 line |
| 46537 | C14orf103 | NM_018036.4 | 2826 | gacacggctcagaccatttatga | 365668 | - | see also 20593 line |
| 46538 | C14orf103 | NM_018036.4 | 1518 | cccacttctccggctaaaagtta | 365629 | - | see also 20593 line |
| 46539 | C14orf104 | NM_018139.1 | 1173 | aacttaaacgagtcagtaataac | 367582 | - | see also 20594 line |
| 46540 | C14orf104 | NM_018139.1 | 1239 | aacaaaactacggttcacaatat | 367585 | - | see also 20594 line |
| 46541 | C14orf105 | NM_018168.1 | 459 | gacttgccacgagtaattacttc | 367887 | - | see also 20595 line |
| 46542 | C14orf105 | NM_018168.1 | 987 | gagagaatcccacttttcgatga | 367892 | - | see also 20595 line |
| 46543 | C14orf108 | NM_018229.2 | 755 | tcccctcgtccgccactaattag | 368724 | - | see also 20596 line |
| 46544 | C14orf108 | NM_018229.2 | 754 | gtcccctcgtccgccactaatta | 368723 | - | see also 20596 line |
| 46545 | C14orf11 | NM_018453.2 | 388 | aaggtactacgatgatatatatt | 371774 | - | see also 20597 line |
| 46546 | C14orf11 | NM_018453.2 | 232 | atgtcttaccggagaaagtgaat | 371771 | - | see also 20597 line |
| 46547 | C14orf111 | NM_015962.1 | 557 | ttgaacggatgccagatgattat | 355639 | - | see also 20598 line |
| 46548 | C14orf112 | NM_016468.3 | 111 | tgcgtgcttttcgcaagaacaag | 357615 | - | see also 20599 line |

Figure 46

| | | | | | | | |
|---|---|---|---|---|---|---|---|
| 46549 | C14orf112 | NM_016468.3 | 112 | gcgtgcttttcgcaagaacaaga | 357616 | - | see also 20599 line |
| 46550 | C14orf112 | NM_016468.3 | 274 | atctttagagtcggaatatgaga | 357620 | - | see also 20599 line |
| 46551 | C14orf115 | NM_018228.1 | 1005 | agcgctacagcgtcaccaaaagc | 368698 | - | see also 20600 line |
| 46552 | C14orf115 | NM_018228.1 | 1440 | cacggtccacttattataattgg | 368703 | - | see also 20600 line |
| 46553 | C14orf115 | NM_018228.1 | 2190 | cccgctccacatactacatgtgg | 368710 | - | see also 20600 line |
| 46554 | C14orf116 | NM_018589.2 | 428 | ctcgtcagctcggttgctttatc | 372275 | - | see also 20601 line |
| 46555 | C14orf116 | NM_018589.2 | 434 | agctcggttgctttatcatgaat | 372278 | - | see also 20601 line |
| 46556 | C14orf118 | NM_017926.2 | 321 | gtggctccggtgaccaattttag | 364239 | - | see also 20602 line |
| 46557 | C14orf118 | NM_017926.2 | 627 | ggccacggattgtgtgaatcagc | 364249 | - | see also 20602 line |
| 46558 | C14orf119 | NM_017924.2 | 196 | ccccagcgtgaacgtttcctaga | 364055 | - | see also 7178 line |
| 46559 | C14orf119 | NM_017924.2 | 96 | ctcagtaccacacaatactaacc | 364053 | - | see also 7178 line |
| 46560 | C14orf129 | NM_016472.2 | 314 | tgcggatgatgtggcctatatca | 357637 | - | see also 20604 line |
| 46561 | C14orf129 | NM_016472.2 | 546 | tggacagtcatgactacacttttt | 357641 | - | see also 20604 line |
| 46562 | C14orf132 | NM_020215.1 | 157 | ttcccctttcccatgaaatcaag | 376240 | - | see also 20605 line |
| 46563 | C14orf132 | NM_020215.1 | 440 | gcctgatccctgcaagacaaatg | 376241 | - | see also 20605 line |
| 46564 | C14orf133 | NM_022067.2 | 1558 | tccatcgggttgtcgaaattttg | 382010 | - | see also 8260 line |
| 46565 | C14orf133 | NM_022067.2 | 1557 | ttccatcgggttgtcgaaatttt | 382009 | - | see also 8260 line |
| 46566 | C14orf135 | NM_022495.2 | 1271 | cccctgggatatccgatttattc | 383542 | - | see also 20607 line |
| 46567 | C14orf135 | NM_022495.2 | 1270 | tcccctgggatatccgatttatt | 383541 | - | see also 20607 line |
| 46568 | C14orf138 | NM_024558.1 | 421 | tccacccgacttcatactgatgg | 387263 | - | see also 20608 line |
| 46569 | C14orf138 | NM_024558.1 | 420 | ctccacccgacttcatactgatg | 387262 | - | see also 20608 line |
| 46570 | C14orf139 | NM_024633.2 | 764 | cccgagcgcctgcaatatggtca | 388344 | - | see also 20609 line |
| 46571 | C14orf139 | NM_024633.2 | 761 | taccccgagcgcctgcaatatgg | 388343 | - | see also 20609 line |
| 46572 | C14orf140 | NM_024643.1 | 167 | ggcgttatgctcccacataatac | 388460 | - | see also 20610 line |
| 46573 | C14orf140 | NM_024643.1 | 168 | gcgttatgctcccacataataca | 388461 | - | see also 20610 line |
| 46574 | C14orf141 | NM_032035.1 | 828 | accttgagatattgtatgcatat | 397675 | - | see also 20611 line |
| 46575 | C14orf141 | NM_032035.1 | 823 | tgccaaccttgagatattgtatg | 397674 | - | see also 20611 line |
| 46576 | C14orf142 | NM_032490.2 | 510 | ctgtttctagcaaagatagtaaa | 400392 | - | see also 20612 line |
| 46577 | C14orf142 | NM_032490.2 | 409 | ttgtcacactaggacatttaagg | 400387 | - | see also 20612 line |
| 46578 | C14orf147 | NM_138288.1 | 131 | tgggagcggacggtgttcaattc | 407446 | - | see also 9592 line |
| 46579 | C14orf147 | NM_138288.1 | 132 | gggagcggacggtgttcaattcc | 407447 | - | see also 9592 line |
| 46580 | C14orf160 | NM_024884.1 | 85 | gccagcgctgcgttatttggttg | 391654 | - | see also 20614 line |
| 46581 | C14orf160 | NM_024884.1 | 607 | gccatattgtaggggtctaatgg | 391665 | - | see also 20614 line |
| 46582 | C14orf161 | NM_024764.1 | 1567 | atggacgtgtcccattgcaatct | 390269 | - | see also 8741 line |
| 46583 | C14orf161 | NM_024764.1 | 459 | ttgcaacatacgactcctaaagg | 390237 | - | see also 8741 line |
| 46584 | C14orf162 | NM_020181.1 | 231 | gccacgcccaaatgatccaaaaa | 375829 | - | see also 20616 line |
| 46585 | C14orf162 | NM_020181.1 | 188 | cgcggaggcctcccagagaaaca | 375826 | - | see also 20616 line |

Figure 46

| | | | | | | | |
|---|---|---|---|---|---|---|---|
| 46586 | C14orf162 | NM_020181.1 | 226 | gtcaagccacgcccaaatgatcc | 375828 | - | see also 20616 line |
| 46587 | C14orf166 | NM_016039.1 | 385 | atcgacaagaagctattgactgg | 356077 | - | see also 20617 line |
| 46588 | C14orf166 | NM_016039.1 | 621 | aaggcaattcggattttggttca | 356081 | - | see also 20617 line |
| 46589 | C14orf168 | NM_031427.1 | 333 | ctgtggatctcctacaattttat | 396944 | - | see also 20618 line |
| 46590 | C14orf168 | NM_031427.1 | 308 | ggcagtaggggacacattagaag | 396942 | - | see also 20618 line |
| 46591 | C14orf169 | NM_024644.1 | 1467 | tccgcgaagaaccgctttcatgg | 388488 | - | see also 8676 line |
| 46592 | C14orf169 | NM_024644.1 | 783 | taccgctgacctggattcgatgc | 388480 | - | see also 8676 line |
| 46593 | C14orf24 | NM_173607.2 | 330 | ctgttgatccgacaaaacttacc | 426483 | - | see also 20620 line |
| 46594 | C14orf24 | NM_173607.2 | 352 | ctggggtccctacttatggtttt | 426484 | - | see also 20620 line |
| 46595 | C14orf29 | NM_181533.3 | 635 | tgggttgcaagtatcaattatcc | 432397 | - | see also 20621 line |
| 46596 | C14orf29 | NM_181533.3 | 868 | gagggtcaagatggttatctttc | 432400 | - | see also 20621 line |
| 46597 | C14orf32 | NM_144578.2 | 601 | ggcagtatcctacccctaatatg | 409919 | - | see also 20622 line |
| 46598 | C14orf32 | NM_144578.2 | 774 | ctctatcctactcccagtaatcc | 409923 | - | see also 20622 line |
| 46599 | C14orf34 | NM_017949.1 | 901 | tggattacgacatcatcgaatgc | 364657 | - | see also 20623 line |
| 46600 | C14orf34 | NM_017949.1 | 1501 | gagatcgattgaaatacgaatcc | 364662 | - | see also 20623 line |
| 46601 | C14orf39 | NM_174978.1 | 1441 | ccccgttcgaaattaacagaaat | 428878 | - | see also 10136 line |
| 46602 | C14orf39 | NM_174978.1 | 488 | aacacccttttcacgtgaatatt | 428845 | - | see also 10136 line |
| 46603 | C14orf39 | NM_174978.1 | 1191 | tggtcggaaaaaggggataaaga | 428868 | - | see also 10136 line |
| 46604 | C14orf45 | NM_025057.1 | 690 | gtggtgctacccactattccaaa | 393358 | - | see also 20625 line |
| 46605 | C14orf45 | NM_025057.1 | 307 | tagcacaagctgctttcaattta | 393350 | - | see also 20625 line |
| 46606 | C14orf48 | NM_152777.2 | 751 | gaggaccacagggctctcatagc | 421068 | - | see also 20626 line |
| 46607 | C14orf48 | NM_152777.2 | 533 | aagtcgagaagcacaagctttt | 421065 | - | see also 20626 line |
| 46608 | C14orf49 | NM_152592.2 | 2385 | tcgacgcgcaaatcttctacagg | 418519 | - | see also 20627 line |
| 46609 | C14orf49 | NM_152592.2 | 2720 | gggagccgactggattccaaaag | 418523 | - | see also 20627 line |
| 46610 | C14orf50 | NM_172365.1 | 1031 | tgcgttctccagtcatgtctact | 424438 | - | see also 20628 line |
| 46611 | C14orf50 | NM_172365.1 | 890 | gactctttcgtaccaatatgttc | 424429 | - | see also 20628 line |
| 46612 | C14orf54 | NM_173526.1 | 820 | gtctctcaggaattacgaatagc | 425489 | - | see also 20629 line |
| 46613 | C14orf54 | NM_173526.1 | 462 | gtgaccgtaggcatctgtttttc | 425479 | - | see also 20629 line |
| 46614 | C14orf58 | NM_017791.1 | 1452 | gacaaccctggtagtctatatca | 362432 | - | see also 7154 line |
| 46615 | C14orf58 | NM_017791.1 | 1606 | gagctcacgtacccagaatcaga | 362438 | - | see also 7154 line |
| 46616 | C14orf59 | NM_174976.1 | 520 | ggcacgaccatcactgtttcttc | 428826 | - | see also 20631 line |
| 46617 | C14orf69 | NM_152333.1 | 649 | gcccagcttcggcgtctacttcc | 415694 | - | - | - | - |
| 46618 | C14orf8 | NM_173846.3 | 390 | accactggcgctactaaagcaac | 428477 | - | see also 20632 line |
| 46619 | C14orf8 | NM_173846.3 | 261 | gcccgaaccatcacgtttcaaca | 428472 | - | see also 20632 line |
| 46620 | C14orf9 | NM_144568.1 | 379 | gtgggagtgcccctatgatcacc | 409751 | - | see also 20633 line |
| 46621 | C14orf9 | NM_144568.1 | 441 | atgcatcagcatgtagtcaaatg | 409753 | - | see also 20633 line |
| 46622 | C14orf93 | NM_021944.1 | 1313 | caggcgcaagcgggatcttgtac | 381515 | - | see also 8207 line |

Figure 46

| | | | | | | | |
|---|---|---|---|---|---|---|---|
| 46623 | C14orf93 | NM_021944.1 | 1624 | ttgccaaccgatccagtatcatg | 381525 | - | see also 8207 line |
| 46624 | C14orf94 | NM_017815.1 | 416 | aggaagttcgactgcattaagaca | 362675 | - | see also 20635 line |
| 46625 | C14orf94 | NM_017815.1 | 1026 | cggtgctatgatccttaagctga | 362682 | - | see also 20635 line |
| 46626 | C15orf16 | NM_130901.1 | 561 | gagcgtgtacagcgaggatttca | 407136 | - | see also 20636 line |
| 46627 | C15orf16 | NM_130901.1 | 264 | agccgctctgagcgactatgagc | 407132 | - | see also 20636 line |
| 46628 | C15orf5 | NM_030944.1 | 602 | aacacctctccatacaaatga | 395655 | - | see also 20637 line |
| 46629 | C15orf5 | NM_030944.1 | 356 | tagggagctacttaaaattttg | 395650 | - | see also 20637 line |
| 46630 | C16orf33 | NM_024571.1 | 1073 | ctcccaaatagccctagaatacg | 387364 | - | - |
| 46631 | C16orf34 | NM_144570.1 | 96 | gaggagaatcgagcaatctttt | 409762 | - | see also 20638 line |
| 46632 | C16orf34 | NM_144570.1 | 142 | ttccagcaggcctaataggatgg | 409764 | - | see also 20638 line |
| 46633 | C16orf34 | NM_144570.1 | 97 | aggagaatcgagcaatcttttg | 409763 | - | see also 20638 line |
| 46634 | C17orf28 | NM_030630.1 | 2343 | tacatgtggggcgtcatctatct | 394730 | - | see also 20639 line |
| 46635 | C17orf28 | NM_030630.1 | 185 | caactcggtgcaggatgtgtttg | 394718 | - | see also 20639 line |
| 46636 | C17orf31 | NM_017575.3 | 382 | gacaagaggatcgtagtctaaaa | 359231 | - | see also 20640 line |
| 46637 | C17orf31 | NM_017575.3 | 451 | gacgacgtttgcagactgttagc | 359238 | - | see also 20640 line |
| 46638 | C17orf31 | NM_017575.3 | 2350 | agcttgacgctgtcattactat | 359283 | - | see also 20640 line |
| 46639 | C19orf10 | NM_019107.1 | 335 | cccaggggaagtcctatctgtac | 375236 | - | see also 20641 line |
| 46640 | C19orf10 | NM_019107.1 | 400 | taggccatggcctactctaaagc | 375237 | - | see also 20641 line |
| 46641 | C19orf6 | NM_033420.2 | 253 | tccacgcgctgttcttcaagatg | 403869 | - | see also 20642 line |
| 46642 | C19orf6 | NM_033420.2 | 1211 | ggccatcatgtcggagttcttca | 403874 | - | see also 20642 line |
| 46643 | C1orf14 | NM_030933.1 | 1172 | tggtcctatcgcacaacgttta | 395552 | - | see also 20643 line |
| 46644 | C1orf14 | NM_030933.1 | 1174 | gtcctatcgcacaacgttttaaa | 395553 | - | see also 20643 line |
| 46645 | C1orf16 | NM_014837.2 | 306 | tccgaatcggagtgaagttcagg | 344387 | - | see also 20644 line |
| 46646 | C1orf16 | NM_014837.2 | 1429 | cagcaacgcttgatcctatagg | 344420 | - | see also 20644 line |
| 46647 | C1orf19 | NM_052965.1 | 485 | gtctgatttctacaatagtctatt | 405571 | - | see also 20645 line |
| 46648 | C1orf19 | NM_052965.1 | 487 | ctgattctacaatagtctattat | 405572 | - | see also 20645 line |
| 46649 | C1orf21 | NM_030806.2 | 807 | ctgttcggaagaagaggatatca | 395223 | - | see also 8952 line |
| 46650 | C1orf21 | NM_030806.2 | 487 | aagcatgttgccactgttcaaaa | 395214 | - | see also 8952 line |
| 46651 | C1orf26 | NM_017673.4 | 2638 | atgttgagaccctctataacttc | 360559 | - | see also 7104 line |
| 46652 | C1orf26 | NM_017673.4 | 2847 | gacatgctcaactataggatata | 360570 | - | see also 7104 line |
| 46653 | C1orf27 | NM_017847.2 | 513 | ttgtcgaacttatgatatccatg | 363040 | - | see also 7161 line |
| 46654 | C1orf27 | NM_017847.2 | 388 | gccctgcgtagactaatgtttgc | 363038 | - | see also 7161 line |
| 46655 | C1orf28 | NM_024529.3 | 1240 | ctcgaacacccattatcataatt | 386755 | - | see also 8627 line |
| 46656 | C1orf28 | NM_024529.3 | 473 | aggtcaacatcggcaagtatag | 386723 | - | see also 8627 line |
| 46657 | C1orf29 | NM_006820.1 | 458 | ctggtgtcgtttatcgaaaac | 336381 | - | see also 20650 line |
| 46658 | C1orf29 | NM_006820.1 | 839 | accgagcggtataggatatttc | 336390 | - | see also 20650 line |
| 46659 | C1orf29 | NM_006820.1 | 838 | aaccgagcggtataggatatatt | 336389 | - | see also 20650 line |

Figure 46

| | | | | | | | |
|---|---|---|---|---|---|---|---|
| 46660 | C1orf35 | NM_024319.2 | 126 | gcgggcaggaccagttcaactgg | 386250 | - | see also 20651 line |
| 46661 | C1orf36 | NM_183059.1 | 1302 | gcgcagcaatgcggtcagaaagg | 435577 | - | see also 20652 line |
| 46662 | C1orf9 | NM_014283.2 | 3308 | tgcagcgttgtcgaaatacttct | 340108 | - | see also 5590 line |
| 46663 | C1orf9 | NM_014283.2 | 3310 | cagcgttgtcgaaatacttctca | 340109 | - | see also 5590 line |
| 46664 | C1QDC1 | NM_023925.2 | 3393 | gtggtccacgagcaaattcgaga | 385383 | - | see also 20655 line |
| 46665 | C1QDC1 | NM_023925.2 | 1095 | cccaagcgtatgagacctatatt | 385329 | - | see also 20655 line |
| 46666 | C1QTNF1 | NM_030968.2 | 601 | cgccgtgccccagatcaacatca | 395896 | - | see also 20656 line |
| 46667 | C1QTNF1 | NM_030968.2 | 606 | tgccccagatcaacatcactatc | 395897 | - | see also 20656 line |
| 46668 | C1QTNF1 | NM_030968.2 | 609 | cccagatcaacatcactatcttg | 395898 | - | see also 20656 line |
| 46669 | C1QTNF2 | NM_031908.3 | 686 | gcggcgtgcctgggatctactac | 397249 | - | see also 20657 line |
| 46670 | C1QTNF2 | NM_031908.3 | 923 | gcctctttacgggcttcctaatc | 397251 | - | see also 20657 line |
| 46671 | C1QTNF5 | NM_015645.1 | 678 | cgggccagcctgcagtttgatct | 354859 | - | see also 20658 line |
| 46672 | C1QTNF6 | NM_031910.3 | 689 | gacgtacgtgcacattatgcata | 397259 | - | see also 20659 line |
| 46673 | C1QTNF6 | NM_031910.3 | 685 | aggagacgtacgtgcacattatg | 397258 | - | see also 20659 line |
| 46674 | C1QTNF7 | NM_031911.3 | 1032 | ctccgggtttctcttatacgttg | 397276 | - | see also 9051 line |
| 46675 | C1QTNF7 | NM_031911.3 | 1033 | tccgggtttctcttatacgttga | 397277 | - | see also 9051 line |
| 46676 | C20orf102 | NM_080607.1 | 523 | gtgggcctcgaaccagctaaaag | 406401 | - | - |
| 46677 | C20orf102 | NM_080607.1 | 524 | tgggcctcgaaccagctaaaagc | 406402 | - | see also 46676 line |
| 46678 | C20orf102 | NM_080607.1 | 376 | cacagagacaccccatgacatga | 406400 | - | see also 46676 line |
| 46679 | C20orf108 | NM_080821.1 | 348 | tgcacattggaatctcattaatt | 406784 | - | see also 20661 line |
| 46680 | C20orf108 | NM_080821.1 | 347 | ttgcacattggaatctcattaat | 406783 | - | see also 20661 line |
| 46681 | C20orf110 | NM_021202.1 | 971 | gccgtgccagcgccagttcaact | 380752 | - | see also 8137 line |
| 46682 | C20orf111 | NM_016470.6 | 539 | ctctacttctctcgatgctaatc | 357627 | - | see also 6868 line |
| 46683 | C20orf111 | NM_016470.6 | 367 | agcgtcgtcgacgttctaagtct | 357626 | - | see also 6868 line |
| 46684 | C20orf111 | NM_016470.6 | 695 | atcagtttccaagctaaaccagg | 357632 | - | see also 6868 line |
| 46685 | C20orf112 | NM_080616.1 | 401 | ggctgcggagccgcgtcaaatac | 406447 | - | see also 20663 line |
| 46686 | C20orf112 | NM_080616.1 | 786 | gtcctgccgtcgcatgaagaaga | 406448 | - | see also 20663 line |
| 46687 | C20orf116 | NM_023935.1 | 790 | gactataacaggtgtgattgacg | 385439 | - | see also 20664 line |
| 46688 | C20orf116 | NM_023935.1 | 741 | tacgcactcaggacaccataaat | 385435 | - | see also 20664 line |
| 46689 | C20orf116 | NM_023935.1 | 55 | ggctctgctagtcggctttatcc | 385432 | - | see also 20664 line |
| 46690 | C20orf12 | NM_018152.2 | 2217 | aggaccaactcgctcaaactagg | 367770 | - | see also 20665 line |
| 46691 | C20orf12 | NM_018152.2 | 1727 | cagagtacctcagatactattga | 367763 | - | see also 20665 line |
| 46692 | C20orf126 | NM_030815.2 | 391 | tgcggaagcaacttaaagtgaag | 395291 | - | see also 20666 line |
| 46693 | C20orf126 | NM_030815.2 | 477 | aaccaggatgagcttaaagctct | 395297 | - | see also 20666 line |
| 46694 | C20orf126 | NM_030815.2 | 213 | gacctggacactaaaaggaatca | 395288 | - | see also 20666 line |
| 46695 | C20orf128 | NM_178468.1 | 1951 | cccaatggtctcccgatatcaag | 430826 | - | see also 20667 line |
| 46696 | C20orf128 | NM_178468.1 | 2219 | agctccgaagccgctttgagtcc | 430829 | - | see also 20667 line |

Figure 46

| | | | | | | | |
|---|---|---|---|---|---|---|---|
| 46697 | C20orf129 | NM_030919.1 | 83 | cgggactgcacgccggttttgt | 395439 | - | see also 20668 line |
| 46698 | C20orf129 | NM_030919.1 | 891 | tggcaacaggctcctacagtttt | 395453 | - | see also 20668 line |
| 46699 | C20orf132 | NM_152503.1 | 150 | ctctcgagcggcgactagtaacc | 417246 | - | see also 20669 line |
| 46700 | C20orf132 | NM_152503.1 | 1326 | tgccgctaaatgcttctacaact | 417268 | - | see also 20669 line |
| 46701 | C20orf133 | NM_080676.2 | 195 | ttcccctgaacagccattctatca | 406667 | - | see also 20670 line |
| 46702 | C20orf133 | NM_080676.2 | 230 | atgaagggcaagggccaaaatga | 406668 | - | see also 20670 line |
| 46703 | C20orf136 | NM_080621.2 | 296 | accactatggcctgtaccataca | 406498 | - | see also 20670 line |
| 46704 | C20orf136 | NM_080621.2 | 590 | aagcttcctcgggaaaatgtcc | 406499 | - | see also 46703 line |
| 46705 | C20orf140 | NM_144628.1 | 794 | caggcacgtcgtgcggttatatg | 410536 | - | see also 9720 line |
| 46706 | C20orf140 | NM_144628.1 | 799 | acgtcgtgcgttatatgacttc | 410538 | - | see also 9720 line |
| 46707 | C20orf144 | NM_080825.2 | 209 | cgctgggcaacggattgactacg | 406789 | - | see also 46707 line |
| 46708 | C20orf144 | NM_080825.2 | 158 | taccaggctcgtgctgattctcc | 406788 | - | see also 46707 line |
| 46709 | C20orf151 | NM_080833.1 | 685 | gggcctacgggagagaagaaaagc | 406816 | - | see also 46709 line |
| 46710 | C20orf151 | NM_080833.1 | 721 | gacatctccagtggccaaaatct | 406817 | - | see also 20673 line |
| 46711 | C20orf152 | NM_080834.1 | 569 | gtcgcaggcgttgatcatcaag | 406831 | - | see also 20673 line |
| 46712 | C20orf152 | NM_080834.1 | 845 | atgctcagtatcggtttgaattt | 406839 | - | see also 20674 line |
| 46713 | C20orf158 | NM_152302.1 | 2069 | gaccccttcgcgatttcacttcc | 415444 | - | see also 20674 line |
| 46714 | C20orf158 | NM_152302.1 | 1904 | aggccaaggcgcctaacttatgc | 415439 | - | see also 20675 line |
| 46715 | C20orf160 | NM_080625.2 | 363 | ctcacctggcgcgacaatgagaga | 406501 | - | see also 20675 line |
| 46716 | C20orf160 | NM_080625.2 | 991 | acggggaccagagtattgagtgt | 406504 | - | see also 20675 line |
| 46717 | C20orf166 | NM_178463.1 | 653 | aacagctgacgggggaagttcagc | 430801 | - | see also 20676 line |
| 46718 | C20orf172 | NM_024918.2 | 636 | gagctcagccggtctattcagtgt | 392140 | - | see also 20676 line |
| 46719 | C20orf172 | NM_024918.2 | 1305 | cagaacccacctgccatacatgg | 392159 | - | see also 20677 line |
| 46720 | C20orf173 | NM_080828.1 | 493 | ctctcggtgagccattttgattt | 406798 | - | see also 20677 line |
| 46721 | C20orf173 | NM_080828.1 | 490 | aggctctcggtgagccatttga | 406797 | - | see also 20678 line |
| 46722 | C20orf175 | NM_080829.1 | 1257 | gttggaacccgtttgatactgaga | 406810 | - | see also 20678 line |
| 46723 | C20orf175 | NM_080829.1 | 1253 | tgcagtggaaccgtttgatact | 406808 | - | see also 20679 line |
| 46724 | C20orf185 | NM_182658.1 | 87 | cacgctcgctcgattgaccaagg | 434856 | - | see also 20679 line |
| 46725 | C20orf185 | NM_182658.1 | 1258 | gacggatcgcgtttagaagaatg | 434866 | - | see also 20679 line |
| 46726 | C20orf185 | NM_182658.1 | 669 | ggctcttgggtccgtggaattct | 434859 | - | see also 20679 line |
| 46727 | C20orf186 | NM_182519.1 | 1661 | tctctcaaacatcgactttagcaat | 434085 | - | see also 20680 line |
| 46728 | C20orf186 | NM_182519.1 | 817 | cccaatcctcgttggacaatttagt | 434080 | - | see also 20680 line |
| 46729 | C20orf19 | NM_018474.2 | 474 | cagttgcacgagggggattaacttca | 371961 | - | see also 20681 line |
| 46730 | C20orf19 | NM_018474.2 | 890 | tccccgttacggggaaagattaag | 371976 | - | see also 20681 line |
| 46731 | C20orf20 | NM_018270.3 | 193 | accgacacttccacatgatttgt | 369602 | - | see also 20682 line |
| 46732 | C20orf20 | NM_018270.3 | 192 | aaccgacaacttccacatgatttg | 369601 | - | see also 20682 line |
| 46733 | C20orf20 | NM_018270.3 | 453 | gggggctgacgatgtttttttcatc | 369604 | - | see also 20682 line |

Figure 46

| | | | | | | | |
|---|---|---|---|---|---|---|---|
| 46734 | C20orf21 | NM_017798.2 | 1277 | gggtttgcccacgcaacagaaac | 362598 | - | see also 7155 line |
| 46735 | C20orf21 | NM_017798.2 | 570 | cagtaacggagaccatcattta | 362592 | - | see also 7155 line |
| 46736 | C20orf24 | NM_018840.1 | 360 | tggggagttttgccattacgagg | 372964 | - | see also 20684 line |
| 46737 | C20orf24 | NM_018840.1 | 438 | ttcagcaattacctacagattga | 372966 | - | see also 20684 line |
| 46738 | C20orf28 | NM_015417.2 | 544 | gggtaaggtggcctttccatt | 352365 | - | see also 20685 line |
| 46739 | C20orf28 | NM_015417.2 | 325 | cagcaactgggtcatctgaaca | 352363 | - | see also 20685 line |
| 46740 | C20orf29 | NM_018347.1 | 741 | gccacatggtcagctgctttcc | 370562 | - | see also 20686 line |
| 46741 | C20orf32 | NM_020356.1 | 910 | ggcggttacagcacattaccaaa | 376874 | - | see also 20687 line |
| 46742 | C20orf32 | NM_020356.1 | 911 | gcggttacagcacacattaccaaat | 376875 | - | see also 20687 line |
| 46743 | C20orf38 | NM_018327.1 | 559 | atctcatggcaactactattg | 370425 | - | see also 20688 line |
| 46744 | C20orf38 | NM_018327.1 | 477 | aggaactattcagggcaaatttc | 370421 | - | see also 20688 line |
| 46745 | C20orf4 | NM_015511.2 | 820 | gaccgcgtggggcagaatctacc | 353651 | - | see also 20689 line |
| 46746 | C20orf4 | NM_015511.2 | 524 | ctcgtatgggtttcttccttagc | 353649 | - | see also 20689 line |
| 46747 | C20orf40 | NM_144703.1 | 887 | agggcaaaacgtccaactaacg | 411497 | - | see also 9731 line |
| 46748 | C20orf40 | NM_144703.1 | 1007 | acccatactgctactgtgtttt | 411501 | - | see also 9731 line |
| 46749 | C20orf43 | NM_016407.1 | 161 | gtggcccaatggaactattgtac | 357395 | - | see also 20691 line |
| 46750 | C20orf43 | NM_016407.1 | 888 | aagaatcggaggcctacaagtcc | 357406 | - | see also 20691 line |
| 46751 | C20orf44 | NM_018244.2 | 687 | tacatgtcgtatcatagttca | 368963 | - | see also 20692 line |
| 46752 | C20orf44 | NM_018244.2 | 749 | gggggttaatccctatatcctga | 368967 | - | see also 20692 line |
| 46753 | C20orf45 | NM_016045.1 | 670 | atcgtgacagttggtagacaaca | 356097 | - | see also 20693 line |
| 46754 | C20orf45 | NM_016045.1 | 512 | gactgatgcaagtacgatatcc | 356095 | - | see also 20693 line |
| 46755 | C20orf45 | NM_016045.1 | 96 | gacttcggagcacgtctttgacc | 356087 | - | see also 20693 line |
| 46756 | C20orf51 | NM_022099.2 | 751 | cacgctgcgcgtcccttgtttc | 382285 | - | - |
| 46757 | C20orf51 | NM_022099.2 | 683 | ctgattgcggaatttactctca | 382284 | - | see also 46756 line |
| 46758 | C20orf51 | NM_022099.2 | 681 | ttctgattgcggaatttactct | 382283 | - | see also 46756 line |
| 46759 | C20orf55 | NM_031424.3 | 986 | cgcgtgatcaagtggttgtatgg | 396927 | - | see also 20694 line |
| 46760 | C20orf55 | NM_031424.3 | 762 | atctcgagcgctttttaactc | 396924 | - | see also 20694 line |
| 46761 | C20orf58 | NM_152864.1 | 558 | tcctcttaccatgtcaatgaaa | 421387 | - | - |
| 46762 | C20orf58 | NM_152864.1 | 491 | gccaggtggtcagcgtgtttacg | 421386 | - | see also 46761 line |
| 46763 | C20orf6 | NM_016649.3 | 1148 | cacgtcgattagcagtttgtaac | 358726 | - | see also 20695 line |
| 46764 | C20orf6 | NM_016649.3 | 1141 | gagattacacgtcgattagcagt | 358725 | - | see also 20695 line |
| 46765 | C20orf65 | NM_176791.2 | 450 | gacaccgagaaccctctgaaagt | 429610 | - | see also 20696 line |
| 46766 | C20orf65 | NM_176791.2 | 378 | cacgtggtcccatcacaaaatct | 429609 | - | see also 20696 line |
| 46767 | C20orf67 | NM_022104.2 | 966 | gcctccacggagtctttcaacc | 382311 | - | see also 20697 line |
| 46768 | C20orf67 | NM_022104.2 | 1700 | tccgacggtaccagatgatgttc | 382321 | - | see also 20697 line |
| 46769 | C20orf7 | NM_024120.2 | 206 | gcccgagccgaccaaatttgact | 386110 | - | see also 8603 line |
| 46770 | C20orf71 | NM_178466.1 | 564 | accgtccttgataacaacatcg | 430811 | - | see also 20699 line |

Figure 46

| | | | | | | | |
|---|---|---|---|---|---|---|---|
| 46771 | C20orf71 | NM_178466.1 | 563 | gaccgtccttcgataacaacatc | 430810 | - | see also 20699 line |
| 46772 | C20orf72 | NM_052865.2 | 322 | accegtgagcaagcataagctgc | 404517 | - | see also 20700 line |
| 46773 | C20orf72 | NM_052865.2 | 686 | aagtctggttaacattgaaagtgt | 404525 | - | see also 20700 line |
| 46774 | C20orf77 | NM_021215.2 | 878 | atgctactgtccgacagaaaatt | 380772 | - | see also 8140 line |
| 46775 | C20orf77 | NM_021215.2 | 1061 | ctcggatgttggtggagtatacc | 380778 | - | see also 8140 line |
| 46776 | C20orf77 | NM_021215.2 | 879 | tgctactgtccgacagaaaatg | 380773 | - | see also 8140 line |
| 46777 | C20orf81 | NM_022760.3 | 1050 | ccctcgggaacgtatcactgg | 383988 | - | see also 8455 line |
| 46778 | C20orf85 | NM_178456.1 | 377 | agtcgacgatgcgatcagaagc | 430789 | - | - |
| 46779 | C20orf85 | NM_178456.1 | 177 | cccttttgaggagttgatcaagt | 430787 | - | see also 46778 line |
| 46780 | C20orf85 | NM_178456.1 | 216 | cacccaagcccaaaatcgagc | 430788 | - | see also 46778 line |
| 46781 | C20orf86 | NM_080674.1 | 568 | ttctaccgaacgcaaattcaga | 406660 | - | see also 20702 line |
| 46782 | C20orf86 | NM_080674.1 | 915 | gtcgggataagggatctgaatg | 406664 | - | see also 20702 line |
| 46783 | C20orf9 | NM_016004.1 | 1142 | cacggctgctcagattaccaat | 355809 | - | see also 6603 line |
| 46784 | C20orf92 | NM_053041.1 | 452 | gtccaggcggatttcataactct | 405823 | - | see also 20704 line |
| 46785 | C20orf92 | NM_053041.1 | 447 | agcaggtccaggcggatttcata | 405821 | - | see also 20704 line |
| 46786 | C21orf100 | NM_145033.1 | 134 | tgcctcacaacgtgacattgttt | 412984 | - | see also 20705 line |
| 46787 | C21orf100 | NM_145033.1 | 135 | gcctcacaacgtgacattgtttg | 412985 | - | see also 20705 line |
| 46788 | C21orf121 | NM_198078.1 | 508 | gtgatactgacttgagatcttat | 436986 | - | see also 20706 line |
| 46789 | C21orf125 | NM_194309.1 | 96 | tcctcgggacacattaggaatga | 436679 | - | see also 20707 line |
| 46790 | C21orf125 | NM_194309.1 | 370 | cggctttgacaagaatcgatttg | 436685 | - | see also 20707 line |
| 46791 | C21orf128 | NM_152507.1 | 314 | ctctgacctagccaagaagagg | 417314 | - | see also 20708 line |
| 46792 | C21orf128 | NM_152507.1 | 75 | ggggctgccttgccatcagaaca | 417312 | - | see also 20708 line |
| 46793 | C21orf129 | NM_152506.1 | 511 | ctccctagacagggttaattatt | 417308 | - | see also 20709 line |
| 46794 | C21orf129 | NM_152506.1 | 512 | tccctagacaggttaattattc | 417309 | - | see also 20709 line |
| 46795 | C21orf18 | NM_017438.1 | 353 | taggggcatacattactaagtgg | 358832 | - | see also 20710 line |
| 46796 | C21orf18 | NM_017438.1 | 889 | tacggccctcacgataatcaacg | 358843 | - | see also 20710 line |
| 46797 | C21orf42 | NM_058184.1 | 505 | cagatcacatcaagagtctaaat | 406211 | - | see also 20711 line |
| 46798 | C21orf42 | NM_058184.1 | 508 | atcacatcaagagtctaaatatc | 406212 | - | see also 20711 line |
| 46799 | C21orf56 | NM_032261.3 | 367 | gagctactacctcaatgagatcc | 399104 | - | see also 20712 line |
| 46800 | C21orf7 | NM_020152.2 | 291 | ttgcacctttagaagttatgtgg | 375726 | - | see also 20714 line |
| 46801 | C21orf7 | NM_020152.2 | 830 | gagctcattgccaagttagatca | 375731 | - | - |
| 46802 | C21orf74 | NM_153203.1 | 225 | caactgaagcttgtttagtttt | 422093 | - | - |
| 46803 | C21orf77 | NM_018277.1 | 185 | ttcacgtcgctgccgccaaatga | 369636 | - | see also 20716 line |
| 46804 | C21orf77 | NM_018277.1 | 187 | cacgtcgctgccagccaaatgacc | 369637 | - | see also 20716 line |
| 46805 | C21orf80 | NM_015227.2 | 1104 | gacggaggcggttgcgattattga | 350121 | - | see also 20717 line |
| 46806 | C21orf80 | NM_015227.2 | 647 | agcccggtccgtgatgttagaca | 350115 | - | see also 20717 line |
| 46807 | C21orf80 | NM_015227.2 | 183 | aacctcgccagggatgtctatat | 350110 | - | see also 20717 line |

Figure 46

| | | | | | | | |
|---|---|---|---|---|---|---|---|
| 46808 | C21orf82 | NM_153751.1 | 248 | ccctgttgcaggctatgcttc | 423878 | - | - |
| 46809 | C21orf82 | NM_153751.1 | 278 | aacccaggccaagacactaaat | 423879 | - | see also 46808 line |
| 46810 | C21orf84 | NM_153752.1 | 95 | aactctgagcaagtcaaaacaac | 423880 | - | see also 20718 line |
| 46811 | C21orf86 | NM_153454.1 | 688 | cagagacggatgtttgtgaagc | 423284 | - | see also 20719 line |
| 46812 | C21orf86 | NM_153454.1 | 683 | ccccgcagagagcggatgtttgt | 423282 | - | see also 20719 line |
| 46813 | C21orf88 | NM_153754.1 | 269 | ttcgtctctcaaggccaaaatca | 423886 | - | see also 20720 line |
| 46814 | C21orf88 | NM_153754.1 | 367 | gtggggttgtaggaatgaagtcc | 423888 | - | see also 20720 line |
| 46815 | C21orf90 | NM_153204.1 | 741 | ccccccacagctcattcgttag | 422096 | - | see also 20721 line |
| 46816 | C21orf90 | NM_153204.1 | 747 | accagctcattcgttagatgatt | 422097 | - | see also 20721 line |
| 46817 | C21orf93 | NM_145179.1 | 968 | ccccgacacggcaccaagacatgg | 413760 | - | - |
| 46818 | C21orf93 | NM_145179.1 | 908 | accatcccgcacatggaaagg | 413759 | - | see also 46817 line |
| 46819 | C21orf94 | NM_145180.2 | 121 | agcctggaagactgtatggaagg | 413761 | - | - |
| 46820 | C21orf94 | NM_145180.2 | 219 | tacatttggtctgtgtatatact | 413762 | - | see also 46819 line |
| 46821 | C21orf97 | NM_021941.1 | 1023 | aaactgtcctaagtaaaactatg | 381388 | - | - |
| 46822 | C21orf97 | NM_021941.1 | 768 | ttcttcccatccaccagtatact | 381386 | - | see also 46821 line |
| 46823 | C21orf97 | NM_021941.1 | 1019 | gggaaacctgtcctaagtaaaac | 381387 | - | see also 46821 line |
| 46824 | C22orf18 | NM_024053.2 | 128 | ggcggactggatgctcaaagagg | 385652 | - | see also 20722 line |
| 46825 | C22orf18 | NM_024053.2 | 391 | agctgcccacacctatcaaagc | 385654 | - | see also 20722 line |
| 46826 | C22orf19 | NM_003678.2 | 1339 | gactttgaggcgactatgtacttg | 331991 | - | see also 20723 line |
| 46827 | C22orf19 | NM_003678.2 | 669 | accgagagtgctatctaacaag | 331979 | - | see also 20723 line |
| 46828 | C22orf2 | NM_015373.2 | 429 | cactgctgaatcccacttaatgg | 351874 | - | - |
| 46829 | C22orf2 | NM_015373.2 | 472 | aggatcagccggaagagagaaatg | 351876 | - | see also 46828 line |
| 46830 | C22orf2 | NM_015373.2 | 471 | gaggatcagccggaagagagaaat | 351875 | - | see also 46828 line |
| 46831 | C22orf4 | NM_014346.1 | 933 | ttctaggaacgaacgaagttcacc | 340249 | - | see also 20724 line |
| 46832 | C22orf4 | NM_014346.1 | 917 | ttgagcactattacgattctagg | 340248 | - | see also 20724 line |
| 46833 | C2orf11 | NM_144629.1 | 1568 | tggctacaaagcagcatcgtattc | 410581 | - | see also 20725 line |
| 46834 | C2orf11 | NM_144629.1 | 701 | tggtgctattgcgcttgaaatta | 410556 | - | see also 20725 line |
| 46835 | C2orf3 | NM_003203.2 | 494 | agcccggagaaaacgtgaattgg | 331248 | - | see also 20726 line |
| 46836 | C2orf3 | NM_003203.2 | 1746 | ccccgacttacagactttgttaga | 331282 | - | see also 20726 line |
| 46837 | C2orf3 | NM_003203.2 | 2095 | agctgctaaatcgttaccttatt | 331293 | - | see also 20726 line |
| 46838 | C2orf5 | NM_152516.1 | 174 | gagggggattccttaagtctattg | 417452 | - | see also 20727 line |
| 46839 | C2orf5 | NM_152516.1 | 435 | tgccattatagagctggaattag | 417455 | - | see also 20727 line |
| 46840 | C2orf6 | NM_018221.1 | 452 | gtccgagatatgaatatcactgg | 368571 | - | see also 20728 line |
| 46841 | C2orf6 | NM_018221.1 | 447 | tgcaggtccgagatatgaatatc | 368570 | - | see also 20728 line |
| 46842 | C2orf9 | NM_032309.2 | 281 | agcgggactgtcactaccttaag | 399641 | - | see also 20729 line |
| 46843 | C2orf9 | NM_032309.2 | 417 | gggcaactgtgcagagcatatgc | 399643 | - | see also 20729 line |
| 46844 | C3F | NM_005768.4 | 195 | tgccaccggcaactacgatatca | 334225 | - | see also 20730 line |

Figure 46

| | | | | | | | |
|---|---|---|---|---|---|---|---|
| 46845 | C3F | NM_005768.4 | 1183 | ttcctgagcctactattcatatt | 334237 | - | see also 20730 line |
| 46846 | C3orf6 | NM_174908.2 | 1417 | atgcggattacactcattttaca | 428622 | - | see also 20731 line |
| 46847 | C3orf6 | NM_174908.2 | 1198 | aagaaatcgctcgacttctaatg | 428614 | - | see also 20731 line |
| 46848 | C4.4A | NM_014400.1 | 680 | tgcactcgggatggagtaacagg | 340537 | - | see also 20732 line |
| 46849 | C4.4A | NM_014400.1 | 732 | gccaggggtcccgctgtaactct | 340538 | - | see also 20732 line |
| 46850 | C4.4A | NM_014400.1 | 614 | gtcaccttgacggcagctaatgt | 340535 | - | see also 20732 line |
| 46851 | C4orf7 | NM_152997.1 | 284 | ctccccttcctagcgaaaagtaa | 421766 | - | see also 20733 line |
| 46852 | C4orf7 | NM_152997.1 | 230 | ttccatggtttagacgtaatttt | 421763 | - | see also 20733 line |
| 46853 | C4orf7 | NM_152997.1 | 231 | tccatggtttagacgtaattttc | 421764 | - | see also 20733 line |
| 46854 | C4orf8 | NM_003704.1 | 423 | gccgcaagcgcaccagttcatct | 331996 | - | see also 20734 line |
| 46855 | C4orf8 | NM_003704.1 | 2087 | cagtctcggatagtaaagagaaa | 332019 | - | see also 20734 line |
| 46856 | C5orf7 | NM_016604.2 | 920 | gtcggtagatcccagactaatcc | 358256 | - | see also 6947 line |
| 46857 | C5orf7 | NM_016604.2 | 974 | aagcgagggggggtacgttaaaag | 358258 | - | see also 6947 line |
| 46858 | C5orf7 | NM_016604.2 | 975 | agcgagggggggtacgttaaaagc | 358259 | - | see also 6947 line |
| 46859 | C6orf10 | NM_006781.2 | 335 | tagaggatcccgacatgcatatt | 336322 | - | see also 20736 line |
| 46860 | C6orf10 | NM_006781.2 | 653 | tccaggacctattgtacaatatc | 336336 | - | see also 20736 line |
| 46861 | C6orf102 | NM_145027.2 | 278 | aggccctgggagaaagtataaat | 412863 | - | see also 20737 line |
| 46862 | C6orf102 | NM_145027.2 | 663 | atggtcccaactcctctctaaca | 412867 | - | see also 20737 line |
| 46863 | C6orf105 | NM_032744.1 | 414 | taccccaaggtcctagatactgt | 401313 | - | see also 20738 line |
| 46864 | C6orf105 | NM_032744.1 | 393 | ctctacaatcgagatctcattta | 401312 | - | see also 20738 line |
| 46865 | C6orf105 | NM_032744.1 | 511 | ctcactcctatccatcaaagaag | 401314 | - | see also 20738 line |
| 46866 | C6orf106 | NM_022758.3 | 671 | aaccacacttggcaagttaaatg | 383983 | - | - | - | - |
| 46867 | C6orf106 | NM_022758.3 | 672 | accacacttggcaagttaaatgt | 383984 | - | see also 46866 line |
| 46868 | C6orf106 | NM_022758.3 | 674 | cacacttggcaagttaaatgtcc | 383985 | - | see also 46866 line |
| 46869 | C6orf107 | NM_017754.2 | 2931 | agcactagcctcgtagattcaga | 361748 | - | see also 20739 line |
| 46870 | C6orf107 | NM_017754.2 | 3533 | gacggtatctcaacagtcatttg | 361761 | - | see also 20739 line |
| 46871 | C6orf107 | NM_017754.2 | 1312 | tccgcaagatggcgtttgactat | 361723 | - | see also 20739 line |
| 46872 | C6orf109 | NM_015388.1 | 600 | ttcactctggttgctatcctact | 352178 | - | see also 20740 line |
| 46873 | C6orf109 | NM_015388.1 | 814 | tcctgttcatcacctataatatc | 352183 | - | see also 20740 line |
| 46874 | C6orf111 | NM_032870.1 | 1937 | gagtcgagataggcgtaaaattg | 402362 | - | see also 20741 line |
| 46875 | C6orf111 | NM_032870.1 | 735 | tggattcgcgaaggtcttgaaaa | 402338 | - | see also 20741 line |
| 46876 | C6orf111 | NM_032870.1 | 1946 | taggcgtaaaattgatgatcaac | 402364 | - | see also 20741 line |
| 46877 | C6orf113 | NM_145062.1 | 1713 | aggtcacagtcgaactgttattg | 413457 | - | see also 9771 line |
| 46878 | C6orf113 | NM_145062.1 | 809 | gtgatcaaccactctatgattgt | 413426 | - | see also 9771 line |
| 46879 | C6orf117 | NM_138409.1 | 224 | tcccagcaatcggcatctaattc | 407972 | - | see also 20743 line |
| 46880 | C6orf117 | NM_138409.1 | 225 | cccagcaatcggcatctaattct | 407973 | - | see also 20743 line |
| 46881 | C6orf118 | NM_144980.1 | 236 | gcctccggagacgatcttacagc | 412271 | - | see also 20744 line |

Figure 46

| | | | | | | | |
|---|---|---|---|---|---|---|---|
| 46882 | C6orf118 | NM_144980.1 | 1041 | atgctcgtgacagccacaaaagc | 412277 | - | see also 20744 line |
| 46883 | C6orf125 | NM_032340.1 | 221 | agcgcgactccattcaaactact | 399820 | - | see also 20745 line |
| 46884 | C6orf125 | NM_032340.1 | 52 | gccggtaccggcgttttcttaag | 399816 | - | see also 20745 line |
| 46885 | C6orf128 | NM_145316.2 | 112 | ggccgtagacatgtatctcatct | 414413 | - | see also 20746 line |
| 46886 | C6orf128 | NM_145316.2 | 358 | tgcaaacgtcgtaatacaaatcc | 414422 | - | see also 20746 line |
| 46887 | C6orf130 | NM_145063.2 | 388 | gagagatgggcgatatatatatt | 413465 | - | see also 20747 line |
| 46888 | C6orf130 | NM_145063.2 | 394 | tgggcgatatatatattacttga | 413467 | - | see also 20747 line |
| 46889 | C6orf134 | NM_024909.1 | 715 | ctcctctagtgaccgagaatttc | 391972 | - | see also 8803 line |
| 46890 | C6orf134 | NM_024909.1 | 417 | ggcgagaactcttccagtatatg | 391967 | - | see also 8803 line |
| 46891 | C6orf134 | NM_024909.1 | 718 | ctctagtgaccgagaatttctga | 391974 | - | see also 8803 line |
| 46892 | C6orf136 | NM_145029.1 | 883 | atgagatcctcaacatacgtacc | 412880 | - | see also 20749 line |
| 46893 | C6orf136 | NM_145029.1 | 1074 | cggttctacaagcgtgacaaaga | 412885 | - | see also 20749 line |
| 46894 | C6orf141 | NM_153344.1 | 571 | agcagacccacccaaatacgtgc | 422841 | - | see also 20750 line |
| 46895 | C6orf141 | NM_153344.1 | 683 | ttcgtgaccgcgctcacttttcg | 422842 | - | see also 20750 line |
| 46896 | C6orf142 | NM_138569.1 | 1178 | ctcgctccatcctttatatcaga | 408480 | - | see also 20751 line |
| 46897 | C6orf142 | NM_138569.1 | 1174 | ctgtctcgctccatcctttatat | 408479 | - | see also 20751 line |
| 46898 | C6orf145 | NM_183373.1 | 664 | tacgtcaccaacctgtcatatta | 435684 | - | see also 10294 line |
| 46899 | C6orf146 | NM_173563.1 | 1201 | aactcgtttactggaactagaac | 426157 | - | see also 20752 line |
| 46900 | C6orf146 | NM_173563.1 | 1590 | aacctactattggccatacaaat | 426171 | - | see also 20752 line |
| 46901 | C6orf146 | NM_173563.1 | 1813 | gtcgccttcccttattgctaagg | 426181 | - | see also 20752 line |
| 46902 | C6orf148 | NM_030568.2 | 775 | ctgcccgaaaactttcattctcc | 394631 | - | see also 20753 line |
| 46903 | C6orf148 | NM_030568.2 | 688 | gagccaggactcctatcatcatg | 394630 | - | see also 20753 line |
| 46904 | C6orf150 | NM_138441.1 | 613 | agctcagccgcgatgatatctcc | 408193 | - | see also 20754 line |
| 46905 | C6orf150 | NM_138441.1 | 1493 | ctgcgtgacatactttcttcagt | 408209 | - | see also 20754 line |
| 46906 | C6orf151 | NM_152551.2 | 234 | tccatacgattccaatcatcaca | 418002 | - | see also 20755 line |
| 46907 | C6orf151 | NM_152551.2 | 775 | gaggtgattcgagatgtgataaa | 418021 | - | see also 20755 line |
| 46908 | C6orf152 | NM_181714.1 | 1442 | aggtacagcgactatatcacaaa | 432934 | - | see also 20756 line |
| 46909 | C6orf152 | NM_181714.1 | 816 | aagccctaagggtctaccaaaca | 432903 | - | see also 20756 line |
| 46910 | C6orf153 | NM_033112.1 | 318 | atccgagtaccatttttacgtca | 403361 | - | see also 20757 line |
| 46911 | C6orf153 | NM_033112.1 | 200 | gactaagacgtacaaacaattgg | 403356 | - | see also 20757 line |
| 46912 | C6orf153 | NM_033112.1 | 136 | acctattgaggggcacatctaac | 403350 | - | see also 20757 line |
| 46913 | C6orf155 | NM_024882.1 | 666 | cacctcgggctgcggatttcagg | 391647 | - | see also 20758 line |
| 46914 | C6orf155 | NM_024882.1 | 497 | aacttgcgaaccgggaaaagttt | 391645 | - | see also 20758 line |
| 46915 | C6orf162 | NM_020425.3 | 189 | ttcggattggtaactctttcact | 377419 | - | see also 20759 line |
| 46916 | C6orf162 | NM_020425.3 | 185 | ggctttcggattggtaactcttt | 377418 | - | see also 20759 line |
| 46917 | C6orf165 | NM_178823.2 | 285 | ctcggctattggatactaaaaat | 431672 | - | see also 20760 line |
| 46918 | C6orf165 | NM_178823.2 | 1013 | aagatagcggtcccaacatcaca | 431700 | - | see also 20760 line |

Figure 46

| | | | | | | | |
|---|---|---|---|---|---|---|---|
| 46919 | C6orf166 | NM_018064.2 | 687 | gccgcctcgccgcagaagtatct | 366029 | - | see also 20761 line |
| 46920 | C6orf166 | NM_018064.2 | 958 | tacggcaggttgggatgatctgt | 366034 | - | see also 20761 line |
| 46921 | C6orf166 | NM_018064.2 | 689 | cgcctcgccgcagaagtatctcc | 366030 | - | see also 20761 line |
| 46922 | C6orf167 | NM_198468.1 | 2366 | gtcttcattatcggctaaagagc | 438352 | - | see also 20762 line |
| 46923 | C6orf167 | NM_198468.1 | 2692 | ttcaagccttaaccgtaagatca | 438359 | - | see also 20762 line |
| 46924 | C6orf167 | NM_198468.1 | 2554 | ctcagccagttatcaattatt | 438356 | - | see also 20762 line |
| 46925 | C6orf168 | NM_032511.1 | 671 | gacttaccgtatcagaactattt | 400484 | - | see also 9229 line |
| 46926 | C6orf170 | NM_152730.2 | 622 | ttctgtgtcgtcacatatata | 420191 | - | see also 20764 line |
| 46927 | C6orf170 | NM_152730.2 | 1563 | ctccgaggttactcgaaaagagt | 420218 | - | see also 20764 line |
| 46928 | C6orf18 | NM_019052.2 | 1856 | ggcctgattgctcgaaagcttgc | 374774 | - | see also 20765 line |
| 46929 | C6orf18 | NM_019052.2 | 2545 | tccttccctactgataagaaga | 374775 | - | see also 20765 line |
| 46930 | C6orf182 | NM_173830.3 | 1512 | tccaatacgagttcataatcttc | 428446 | - | see also 20766 line |
| 46931 | C6orf182 | NM_173830.3 | 1422 | cagctaccctaaaggatcaaaga | 428445 | - | see also 20766 line |
| 46932 | C6orf188 | NM_153711.1 | 677 | ctggctgccgatctaaagttagc | 423836 | - | see also 10016 line |
| 46933 | C6orf188 | NM_153711.1 | 675 | tgctggctgccgatctaaagtta | 423835 | - | see also 10016 line |
| 46934 | C6orf192 | NM_052831.1 | 786 | ctgatcgcttacccaaagttgg | 404295 | - | see also 20768 line |
| 46935 | C6orf192 | NM_052831.1 | 711 | ttgctgatgtaccactcaatat | 404289 | - | see also 20768 line |
| 46936 | C6orf195 | NM_152554.1 | 874 | ccgttcagaggagattagcaat | 418071 | - | see also 20769 line |
| 46937 | C6orf195 | NM_152554.1 | 664 | ggctacagacatgcctcagattgc | 418070 | - | see also 20769 line |
| 46938 | C6orf203 | NM_016487.2 | 738 | gtgttaccggcggtggaaaagttt | 357829 | - | see also 20770 line |
| 46939 | C6orf203 | NM_016487.2 | 332 | aaggtctacaaaatctactaaaa | 357822 | - | see also 20770 line |
| 46940 | C6orf206 | NM_152732.2 | 181 | gcctcgtcgccgattactacac | 420249 | - | see also 20771 line |
| 46941 | C6orf206 | NM_152732.2 | 179 | tggcctcgtcgccgattactaca | 420248 | - | see also 20771 line |
| 46942 | C6orf208 | NM_025002.1 | 619 | cccgcactcgtccatttcataa | 392895 | - | - |
| 46943 | C6orf208 | NM_025002.1 | 618 | cccgcactcgtccatttcatta | 392894 | - | see also 46942 line |
| 46944 | C6orf210 | NM_020381.2 | 992 | cagatgatattggaatatcgact | 377334 | - | see also 20772 line |
| 46945 | C6orf210 | NM_020381.2 | 298 | ttgcacttgccacgttatcttgg | 377319 | - | see also 20772 line |
| 46946 | C6orf211 | NM_024573.1 | 652 | tccaccaatcgattactttgatg | 387389 | - | see also 20773 line |
| 46947 | C6orf211 | NM_024573.1 | 591 | caccgtggttggttggtagaatgt | 387381 | - | see also 20773 line |
| 46948 | C6orf25 | NM_025260.2 | 369 | gggggacaggaacctattgcaagg | 394622 | - | see also 20774 line |
| 46949 | C6orf25 | NM_025260.2 | 78 | cctgggggaccgggtgaatctct | 394619 | - | see also 20774 line |
| 46950 | C6orf27 | NM_025258.1 | 1387 | ctgcgtttgagccatacaaagc | 394616 | - | see also 20775 line |
| 46951 | C6orf27 | NM_025258.1 | 2187 | tagtggcccctcgggtttcttgg | 394617 | - | see also 20775 line |
| 46952 | C6orf29 | NM_025257.1 | 607 | cagggggatcagcgggtcttattga | 394584 | - | see also 20776 line |
| 46953 | C6orf29 | NM_025257.1 | 446 | tgccagggtaccctggaatatg | 394581 | - | see also 20776 line |
| 46954 | C6orf32 | NM_015864.2 | 466 | ggcccaatgggatcattagaagc | 355261 | - | see also 6546 line |
| 46955 | C6orf32 | NM_015864.2 | 820 | gacgcctggagtttcatataagt | 355267 | - | see also 6546 line |

Figure 46

| 46956 | C6orf37 | NM_017633.1 | 894 | ctgaccgatggatcttatatcc | 359781 | - | see also 20778 line |
|---|---|---|---|---|---|---|---|
| 46957 | C6orf37 | NM_017633.1 | 892 | ctctgaccgatggagtcttatat | 359780 | - | see also 20778 line |
| 46958 | C6orf48 | NM_016947.1 | 275 | ccctgctgactcaaaacaactaa | 358820 | - | - |
| 46959 | C6orf48 | NM_016947.1 | 280 | ctgatccaaacaactaatgaac | 358821 | - | see also 46958 line |
| 46960 | C6orf48 | NM_016947.1 | 386 | ctcatggagagaagctttgtatg | 358822 | - | see also 46958 line |
| 46961 | C6orf49 | NM_013397.3 | 1266 | gactcggaacctgaaggatttt | 338903 | - | - |
| 46962 | C6orf49 | NM_013397.3 | 444 | ctccctccgcaggacaattgatga | 338900 | - | see also 46961 line |
| 46963 | C6orf49 | NM_013397.3 | 794 | ggcttgtgaccagctgatcttct | 338902 | - | see also 46961 line |
| 46964 | C6orf55 | NM_016485.3 | 149 | ctggaatgaagatcgatagtaaa | 357777 | - | see also 6879 line |
| 46965 | C6orf55 | NM_016485.3 | 761 | ttgatccgcactttcaataca | 357802 | - | see also 6879 line |
| 46966 | C6orf56 | NM_014721.1 | 1756 | ctcgaaggatcctgcgatttaac | 342865 | - | see also 20780 line |
| 46967 | C6orf56 | NM_014721.1 | 1379 | ctcggacgggctatcttgtaca | 342855 | - | see also 20780 line |
| 46968 | C6orf60 | NM_024581.3 | 3608 | ctccagtccacgatattgagttc | 387553 | - | see also 20781 line |
| 46969 | C6orf60 | NM_024581.3 | 3609 | tccagtccacgatattgagttca | 387554 | - | see also 20781 line |
| 46970 | C6orf62 | NM_030939.2 | 813 | tccatgctctcgatatcagtct | 395635 | - | see also 8972 line |
| 46971 | C6orf62 | NM_030939.2 | 819 | ctcctcgatatcagtctcatgaga | 395637 | - | see also 8972 line |
| 46972 | C6orf64 | NM_018322.1 | 145 | aagcggttcctgtatggaaaacc | 370326 | - | see also 20783 line |
| 46973 | C6orf64 | NM_018322.1 | 356 | tggaatttggcctggcatattt | 370328 | - | see also 20783 line |
| 46974 | C6orf65 | NM_152731.1 | 474 | aagaattgtgcgccaaaataaaa | 420234 | - | see also 20784 line |
| 46975 | C6orf65 | NM_152731.1 | 702 | ttgcctcaacatgcagtaattct | 420239 | - | see also 20784 line |
| 46976 | C6orf69 | NM_173562.3 | 1400 | ggccggtcgaagtcatctataa | 426124 | - | see also 20785 line |
| 46977 | C6orf69 | NM_173562.3 | 1401 | gccggtctgaagtcatctataat | 426125 | - | see also 20785 line |
| 46978 | C6orf69 | NM_173562.3 | 1022 | ttcaacactatccgatgtcaaga | 426111 | - | see also 20785 line |
| 46979 | C6orf74 | NM_016063.1 | 195 | gagcgtttcagatcacatgtacc | 356171 | - | see also 20786 line |
| 46980 | C6orf74 | NM_016063.1 | 494 | ctgagatgtccagcttgtttct | 356176 | - | see also 20786 line |
| 46981 | C6orf75 | NM_021820.1 | 441 | agcgaatagatgcacttgaattt | 381057 | - | see also 20787 line |
| 46982 | C6orf75 | NM_021820.1 | 126 | tccgcctgcggaaataaagtct | 381042 | - | see also 20787 line |
| 46983 | C6orf76 | NM_024807.1 | 299 | tgctcctataagggctacaaaa | 390832 | - | see also 20788 line |
| 46984 | C6orf76 | NM_024807.1 | 240 | ctgctgacagtgtatacacaaaa | 390829 | - | see also 20788 line |
| 46985 | C6orf78 | NM_153036.1 | 515 | atgacttgcaaggtcactatagc | 422019 | - | see also 20789 line |
| 46986 | C6orf78 | NM_153036.1 | 395 | tgcatcgcataaagaagctattt | 422015 | - | see also 20789 line |
| 46987 | C6orf79 | NM_022102.1 | 586 | tggagcgcaaaaggagagagatttc | 382301 | - | - |
| 46988 | C6orf81 | NM_145028.3 | 423 | tgcttgtactacgcgatgacatcg | 412874 | - | see also 20790 line |
| 46989 | C6orf81 | NM_145028.3 | 550 | caggaacactccatcaaagtact | 412877 | - | see also 20790 line |
| 46990 | C6orf82 | NM_015921.1 | 274 | ctgtggcctccgcctttgttg | 355489 | - | see also 6571 line |
| 46991 | C6orf82 | NM_015921.1 | 481 | acctcatccctcagattacatcc | 355490 | - | see also 6571 line |
| 46992 | C6orf83 | NM_145169.1 | 205 | ggcggcataaagcttttgcagt | 413541 | - | see also 20792 line |

Figure 46

| 46993 | C6orf83 | NM_145169.1 | 151 | ttcgtatgtgcgtttcttc | 413540 | - | see also 20792 line |
|---|---|---|---|---|---|---|---|
| 46994 | C6orf83 | NM_145169.1 | 465 | tgcaagggatgcagttattaaat | 413548 | - | see also 20792 line |
| 46995 | C6orf84 | NM_014895.1 | 3160 | agccgaaatagacgttcttaaac | 345360 | - | see also 20793 line |
| 46996 | C6orf84 | NM_014895.1 | 3159 | aagccgaaatagacgttcttaaa | 345359 | - | see also 20793 line |
| 46997 | C6orf85 | NM_021945.2 | 2133 | taccctgtcagcctttcaataaa | 381531 | - | - |
| 46998 | C6orf85 | NM_021945.2 | 2134 | accctgtcagcctttcaataaaa | 381532 | - | see also 46997 line |
| 46999 | C6orf85 | NM_021945.2 | 1819 | agtgttgagcaacacctttatg | 381530 | - | see also 46997 line |
| 47000 | C6orf89 | NM_152734.1 | 1363 | tgcgatggaaccgctttctcaga | 420274 | - | see also 20794 line |
| 47001 | C6orf89 | NM_152734.1 | 1315 | cactggaaggtctacgttatagc | 420273 | - | see also 20794 line |
| 47002 | C6orf89 | NM_152734.1 | 1313 | cccactggaaggtctacgttata | 420272 | - | see also 20794 line |
| 47003 | C6orf93 | NM_032860.3 | 821 | tcgcttcacggagtattcgatga | 402146 | - | see also 9322 line |
| 47004 | C6orf93 | NM_032860.3 | 174 | cggagccaacgagatccttagc | 402135 | - | see also 9322 line |
| 47005 | C6orf96 | NM_017909.1 | 1196 | ctctaagccacccgtataaactg | 363870 | - | see also 7171 line |
| 47006 | C6orf96 | NM_017909.1 | 1193 | ttgcttcttaaggcacccgtataaac | 363868 | - | see also 7171 line |
| 47007 | C6orf97 | NM_025059.2 | 234 | tccgatccaagatgctttctaaa | 393364 | - | see also 20797 line |
| 47008 | C6orf97 | NM_025059.2 | 1549 | gagcctcctccggcagaaaatag | 393381 | - | see also 20797 line |
| 47009 | C7orf11 | NM_138701.1 | 501 | ggcctagaaccagtatcgtagt | 408605 | - | see also 20798 line |
| 47010 | C7orf11 | NM_138701.1 | 531 | agccaacaatacagcaatactca | 408607 | - | see also 20798 line |
| 47011 | C7orf13 | NM_032625.1 | 821 | tcgctgcttccttccttaagg | 401148 | - | see also 20799 line |
| 47012 | C7orf19 | NM_032831.1 | 155 | ccggccataagggcatggattac | 401805 | - | see also 20800 line |
| 47013 | C7orf2 | NM_022458.2 | 1401 | ttggcgacttggaagtttaat | 382944 | - | see also 20801 line |
| 47014 | C7orf2 | NM_022458.2 | 204 | aagtgcggagtccacgatatgt | 382905 | - | see also 20801 line |
| 47015 | C7orf20 | NM_015949.2 | 680 | ggcccaggccgtgctacagtttc | 355598 | - | see also 20802 line |
| 47016 | C7orf21 | NM_031434.2 | 294 | atgaggtgaccgtcctttctcg | 396954 | - | - |
| 47017 | C7orf22 | NM_031443.2 | 823 | agcgatgactcttctacacaaagt | 397037 | - | see also 20803 line |
| 47018 | C7orf22 | NM_031443.2 | 123 | cttgccatttaaacgagtattcc | 397027 | - | see also 20803 line |
| 47019 | C7orf23 | NM_024315.2 | 278 | gacgtccgccaaggatcgaatca | 386231 | - | see also 20804 line |
| 47020 | C7orf23 | NM_024315.2 | 281 | gtccgccaaggatcgaatcatca | 386232 | - | see also 20804 line |
| 47021 | C7orf24 | NM_024051.2 | 487 | aaactgtcgaagttatctgatga | 385631 | - | see also 20805 line |
| 47022 | C7orf24 | NM_024051.2 | 364 | gtggggagtagtatggaaaatga | 385628 | - | see also 20805 line |
| 47023 | C7orf24 | NM_024051.2 | 610 | agcaatagaaccacaatgactata | 385637 | - | see also 20805 line |
| 47024 | C7orf25 | NM_024054.1 | 1389 | caccccttaccaaaagactaca | 385682 | - | see also 20806 line |
| 47025 | C7orf25 | NM_024054.1 | 834 | cagagttgaccgagagaaaatatac | 385663 | - | see also 20806 line |
| 47026 | C7orf26 | NM_024067.2 | 413 | agcggacgcccgtggtttactgt | 385716 | - | see also 8581 line |
| 47027 | C7orf26 | NM_024067.2 | 549 | ctccgttaccgctctatgacc | 385720 | - | see also 8581 line |
| 47028 | C7orf27 | NM_152743.1 | 851 | ctgttctcccgttcagttct | 420312 | - | see also 20808 line |
| 47029 | C7orf27 | NM_152743.1 | 2138 | gccacgtggggctctttgacttc | 420315 | - | see also 20808 line |

Figure 46

| | | | | | | | |
|---|---|---|---|---|---|---|---|
| 47030 | C7orf28B | NM_198097.1 | 1370 | ggcgggagctctatgttatttg | 437027 | - | see also 20809 line |
| 47031 | C7orf31 | NM_138811.3 | 1556 | aaggacgaattgcccgattaatt | 409166 | - | see also 9641 line |
| 47032 | C7orf31 | NM_138811.3 | 2003 | ctgttagccttagtaaacctagt | 409184 | - | see also 9641 line |
| 47033 | C7orf33 | NM_145304.2 | 819 | acgtacgtgggcatgaagtaaga | 414285 | - | see also 20811 line |
| 47034 | C7orf33 | NM_145304.2 | 818 | aacgtacgtgggcatgaagtaag | 414284 | - | see also 20811 line |
| 47035 | C7orf34 | NM_178829.2 | 408 | tggccagtgtcatggtttctca | 431977 | - | see also 20812 line |
| 47036 | C7orf34 | NM_178829.2 | 384 | tccacctagcttctgctttagt | 431976 | - | see also 20812 line |
| 47037 | C7orf35 | NM_032936.2 | 499 | tgcctcgcactctgctaaact | 402565 | - | see also 9346 line |
| 47038 | C7orf35 | NM_032936.2 | 591 | aacagaactcggatataatgtct | 402568 | - | see also 9346 line |
| 47039 | C8orf12 | NM_054017.2 | 1070 | ctggtgctagcactttgcttct | 405968 | - | - |
| 47040 | C8orf14 | NM_054029.1 | 1567 | cccgagatcgttcactttgtgc | 406012 | - | see also 20814 line |
| 47041 | C8orf14 | NM_054029.1 | 1565 | agcccgagatcggttcacttgt | 406011 | - | see also 20814 line |
| 47042 | C8orf15 | NM_175076.1 | 1285 | aactgccttggtgttattc | 429008 | - | see also 20815 line |
| 47043 | C8orf15 | NM_175076.1 | 1282 | tggaactgccttggtgtttat | 429007 | - | see also 20815 line |
| 47044 | C8orf17 | NM_020237.1 | 1251 | cccgtcataagtacaagatgttg | 376405 | - | see also 20816 line |
| 47045 | C8orf17 | NM_020237.1 | 1249 | ctccgtcataagtacaagatgt | 376404 | - | see also 20816 line |
| 47046 | C8orf17 | NM_020237.1 | 1243 | ttcttctccgtcataagtaca | 376403 | - | see also 20816 line |
| 47047 | C8orf4 | NM_020130.2 | 193 | aacacagaccaagaatcactaga | 375625 | - | - |
| 47048 | C9orf10 | NM_014612.2 | 966 | cagatgacaaagttattcgattt | 341104 | - | see also 20817 line |
| 47049 | C9orf10 | NM_014612.2 | 1429 | cggagggcgacaaaccatatca | 341109 | - | see also 20817 line |
| 47050 | C9orf10OS | NM_198841.1 | 1546 | cccatactccggtgaaaaga | 439891 | - | - |
| 47051 | C9orf10OS | NM_198841.1 | 1620 | caccaacactaagatcattcttg | 439892 | - | see also 47050 line |
| 47052 | C9orf23 | NM_148178.1 | 163 | gggtccgagatggcagcaaatt | 415120 | - | see also 20818 line |
| 47053 | C9orf23 | NM_148178.1 | 281 | cagctgcgctgagattgtcaagc | 415122 | - | see also 20818 line |
| 47054 | C9orf24 | NM_032596.3 | 683 | tgcctacgagcgcgaagtgatgg | 400984 | - | see also 20819 line |
| 47055 | C9orf24 | NM_032596.3 | 692 | gcgcgaagtgatggtgaacatgc | 400985 | - | see also 20819 line |
| 47056 | C9orf25 | NM_147202.1 | 694 | cggctaccggttagatgagatcc | 415119 | - | see also 20820 line |
| 47057 | C9orf25 | NM_147202.1 | 517 | aaggctggtcgtcccaataaag | 415114 | - | see also 20820 line |
| 47058 | C9orf26 | NM_033439.1 | 514 | ctcaacaccctcaaatgaatca | 404017 | - | see also 20821 line |
| 47059 | C9orf26 | NM_033439.1 | 402 | tacaatgatcaatcattacttt | 404014 | - | see also 20821 line |
| 47060 | C9orf34 | NM_017948.3 | 333 | aggtaacgccaacttgttagaaa | 364570 | - | see also 20822 line |
| 47061 | C9orf34 | NM_017948.3 | 2016 | ttctaggccattagaaggtaaga | 364616 | - | see also 20822 line |
| 47062 | C9orf37 | NM_032937.2 | 1006 | cacgcgccagcgggtttctcagc | 402571 | - | see also 20823 line |
| 47063 | C9orf37 | NM_032937.2 | 1078 | caggagccaaggcacaaatttaa | 402572 | - | see also 20823 line |
| 47064 | C9orf41 | NM_152420.1 | 376 | tactacggcaccagtatgcatga | 416503 | - | see also 20824 line |
| 47065 | C9orf41 | NM_152420.1 | 397 | gagcgggtgaaccgaacagaaag | 416504 | - | see also 20824 line |
| 47066 | C9orf45 | NM_030814.3 | 586 | gggagatgctttgctactactta | 395279 | - | see also 20825 line |

Figure 46

| 47067 | C9orf45 | NM_030814.3 | 588 | gagatgctttgctactacttact | 395280 | - | see also 20825 line |
|---|---|---|---|---|---|---|---|
| 47068 | C9orf46 | NM_018465.1 | 450 | tcccgattgttccattaagcttt | 371893 | - | see also 7490 line |
| 47069 | C9orf46 | NM_018465.1 | 446 | ctggtcccgattgttccattaag | 371891 | - | see also 7490 line |
| 47070 | C9orf48 | NM_194313.1 | 2115 | accccacgaacaggaagtagtga | 436716 | - | see also 20827 line |
| 47071 | C9orf48 | NM_194313.1 | 2444 | gagccagctggcttctaatgatt | 436719 | - | see also 20827 line |
| 47072 | C9orf54 | NM_032809.2 | 1068 | ctccgccaccgagctctttgagt | 401599 | - | see also 20828 line |
| 47073 | C9orf54 | NM_032809.2 | 1089 | gtccctgcagactggagattacc | 401600 | - | see also 20828 line |
| 47074 | C9orf7 | NM_017586.1 | 239 | ggcgtgtggatgatcatgaatgc | 359388 | - | see also 20829 line |
| 47075 | C9orf7 | NM_017586.1 | 297 | gccagttcatcgagtttgcaaac | 359389 | - | see also 20829 line |
| 47076 | C9orf9 | NM_018956.3 | 572 | ccccctcatcctagacttaatga | 373893 | - | - | - | - |
| 47077 | C9orf9 | NM_018956.3 | 452 | aaccctcgttagccaaagcaacg | 373890 | - | see also 47076 line |
| 47078 | C9orf9 | NM_018956.3 | 570 | atccccctcatcctagacttaat | 373891 | - | see also 47076 line |
| 47079 | CAB2 | NM_033419.2 | 197 | gacctgtcgggacgactgtaagt | 403859 | - | see also 20830 line |
| 47080 | CAB2 | NM_033419.2 | 163 | cccgccagccaatctacatgagt | 403858 | - | see also 20830 line |
| 47081 | CAB56184 | NM_032520.2 | 240 | gtggagtccacgtacaagtatga | 400546 | - | see also 9232 line |
| 47082 | CAB56184 | NM_032520.2 | 430 | tggagctggcgtgtggaaaaagc | 400547 | - | see also 9232 line |
| 47083 | CACNB3 | NM_000725.2 | 1180 | cagtgcccaccggagtcatttga | 329865 | - | see also 20831 line |
| 47084 | CACNB3 | NM_000725.2 | 1183 | tgcccaccggagtcatttgatgt | 329866 | - | see also 20831 line |
| 47085 | CADPS2 | NM_017954.7 | 456 | aacgcagttcggagttattatga | 364760 | - | see also 7181 line |
| 47086 | CADPS2 | NM_017954.7 | 453 | tgcaacgcagttcggagttatta | 364759 | - | see also 7181 line |
| 47087 | CADPS2 | NM_017954.7 | 1168 | ctcccaatcgaattgtttactgt | 364789 | - | see also 7181 line |
| 47088 | CaMKIIalpha | NM_018584.4 | 552 | gccggagcaagcgggttgttatt | 372263 | - | see also 20832 line |
| 47089 | CaMKIIalpha | NM_018584.4 | 454 | cggcgacgtgggccagatcttct | 372261 | - | see also 20832 line |
| 47090 | CAMTA2 | NM_015099.2 | 2352 | cccgctcaacgtggatcatttct | 348024 | - | see also 6236 line |
| 47091 | CAMTA2 | NM_015099.2 | 2662 | gtcacgtcagcctattctagtgc | 348026 | - | see also 6236 line |
| 47092 | CANP | NM_198947.1 | 877 | ggccgttttcggtctgacatagg | 440166 | - | see also 20834 line |
| 47093 | CANP | NM_198947.1 | 1298 | atctgggtaggcggtatgctatt | 440175 | - | see also 20834 line |
| 47094 | CAP350 | NM_014810.2 | 8760 | agcgcgttaggagatgatcaaga | 344147 | - | see also 20835 line |
| 47095 | CAP350 | NM_014810.2 | 1950 | gaggtacgacagtacattgttag | 343945 | - | see also 20835 line |
| 47096 | CAPN12 | NM_144691.2 | 816 | ctcggaacagcggaatgagttct | 411331 | - | see also 20836 line |
| 47097 | CAPN12 | NM_144691.2 | 516 | ggccaaaggcgtgaaatggatga | 411327 | - | see also 20836 line |
| 47098 | CASKIN1 | NM_020764.1 | 958 | gggcgaccaaggattattgcaac | 378603 | - | see also 20837 line |
| 47099 | CASKIN1 | NM_020764.1 | 1252 | gggaccgaagcggcagcattagc | 378604 | - | see also 20837 line |
| 47100 | CASKIN2 | NM_020753.1 | 1294 | aggtccgagcgctcaaggatttc | 378545 | - | see also 20838 line |
| 47101 | CASKIN2 | NM_020753.1 | 459 | ctgatcctcgccgtcaagaatgg | 378538 | - | see also 20838 line |

Figure 46

| | | | | | | | |
|---|---|---|---|---|---|---|---|
| 47102 | CASP8AP2 | NM_012115.2 | 3075 | cagctgatgtcggaagtcaaag | 337596 | - | see also 20839 line |
| 47103 | CASP8AP2 | NM_012115.2 | 898 | tgggggagtagctcaaatgagg | 337539 | - | see also 20839 line |
| 47104 | CAST | NM_015576.1 | 1727 | gaggtagatgcgctgagattacg | 354210 | - | see also 6496 line |
| 47105 | CAST | NM_015576.1 | 2940 | accgactagtacatcaattaaag | 354231 | - | see also 6496 line |
| 47106 | CBLN2 | NM_182511.2 | 912 | ttcgaccaggtattagtaaatat | 434038 | - | see also 20842 line |
| 47107 | CBLN2 | NM_182511.2 | 1006 | acgtggtcaaagtgtataacaga | 434043 | - | see also 20842 line |
| 47108 | CBLNL1 | NM_080617.3 | 399 | aactatccaggttaacttgatgt | 406455 | - | see also 9516 line |
| 47109 | CBLNL1 | NM_080617.3 | 512 | aagaggataaggttacctaaaa | 406459 | - | see also 9516 line |
| 47110 | CBLNL1 | NM_080617.3 | 493 | gtcctgctcacctagataaaga | 406458 | - | see also 9516 line |
| 47111 | CCDC2 | NM_025103.1 | 1322 | tgcagtcgaaatataaatcgtat | 393651 | - | see also 8861 line |
| 47112 | CCDC2 | NM_025103.1 | 1324 | cagtcgaaatataaatcgtatag | 393652 | - | see also 8861 line |
| 47113 | CCNDBP1 | NM_012142.2 | 1059 | acctgaccgtgcgaatcaattct | 337705 | - | see also 20844 line |
| 47114 | CCNDBP1 | NM_012142.2 | 1058 | caactgaccgtgcgaatcaattc | 337704 | - | see also 20844 line |
| 47115 | CD1E | NM_030893.1 | 482 | tgctcaatcgctacctagatatt | 395363 | - | see also 20845 line |
| 47116 | CD1E | NM_030893.1 | 328 | cccttcgagatccagatattagc | 395360 | - | see also 20845 line |
| 47117 | CD1E | NM_030893.1 | 490 | cgctacctagatattaaggaaat | 395365 | - | see also 20845 line |
| 47118 | CD99L2 | NM_031462.1 | 518 | ggccgcaggaaaccaattgctgg | 397086 | - | - |
| 47119 | CD99L2 | NM_031462.1 | 487 | tgccctggatgatcgaaatgatc | 397085 | - | see also 47118 line |
| 47120 | CD99L2 | NM_031462.1 | 368 | cgcaggaaaccgggtataggagg | 397084 | - | see also 47118 line |
| 47121 | CDA017 | NM_032024.2 | 745 | aagtgtcgctacgtttactatgg | 397622 | - | see also 20846 line |
| 47122 | CDA017 | NM_032024.2 | 740 | tggggaagttgctacgtttac | 397620 | - | see also 20846 line |
| 47123 | CDA11 | NM_032026.1 | 30 | gtcgcttcaagtttatcgatatt | 397625 | - | see also 9062 line |
| 47124 | CDA11 | NM_032026.1 | 141 | ctgtcgagattgtttaaaaag | 397627 | - | see also 9062 line |
| 47125 | CDAN1 | NM_138477.1 | 1138 | gacagcgaccctgaactaagtcc | 408368 | - | see also 20848 line |
| 47126 | CDAN1 | NM_138477.1 | 1448 | gtgaccgagcctttcatacttt | 408373 | - | see also 20848 line |
| 47127 | CDCA4 | NM_017955.2 | 638 | cacaagtcacttgatcagatatt | 364860 | - | see also 20849 line |
| 47128 | CDCA4 | NM_017955.2 | 632 | agctttcacaagtcacttgatca | 364859 | - | see also 20849 line |
| 47129 | CDCA4 | NM_017955.2 | 606 | tggatggccctcgagaaaacaga | 364858 | - | see also 20849 line |
| 47130 | CDCA5 | NM_080668.2 | 300 | ctcgcaggagccctaggatttcc | 406616 | - | see also 20850 line |
| 47131 | CDCA5 | NM_080668.2 | 348 | ccccttggcaggagcttactaag | 406618 | - | see also 20850 line |
| 47132 | CDCA7 | NM_031942.3 | 1075 | tccgttgacccttccgcatataat | 397449 | - | see also 20851 line |
| 47133 | CDCA7 | NM_031942.3 | 1279 | tgccttcgaaacgttatggtga | 397458 | - | see also 20851 line |
| 47134 | CDCA8 | NM_018101.1 | 195 | ttcgaccgtgaagtggaaatacg | 366700 | - | see also 20852 line |
| 47135 | CDCA8 | NM_018101.1 | 214 | tacgaatcaagcaaattgagtca | 366703 | - | see also 20852 line |
| 47136 | CDCP1 | NM_022842.3 | 772 | aaccgctcatctataaaacgtct | 384651 | - | see also 20853 line |
| 47137 | CDCP1 | NM_022842.3 | 767 | ttgcaaaccgctcatctataaaa | 384650 | - | see also 20853 line |

Figure 46

| | | | | | | | |
|---|---|---|---|---|---|---|---|
| 47138 | CDK5RAP1 | NM_016082.3 | 1204 | gagttagttcaccattagaga | 356236 | - | see also 6653 line |
| 47139 | CDK5RAP1 | NM_016082.3 | 805 | cggcctattgcctccattctaga | 356223 | - | see also 6653 line |
| 47140 | CDK5RAP2 | NM_018249.3 | 3158 | gccaacgcccgacaaaacgttgc | 369052 | - | see also 20855 line |
| 47141 | CDK5RAP2 | NM_018249.3 | 3157 | ggccaacgcccgacaaaacgttg | 369051 | - | see also 20855 line |
| 47142 | CDK5RAP2 | NM_018249.3 | 1304 | tgcgctgcgctcacaaaacctca | 368998 | - | see also 20855 line |
| 47143 | CDT1 | NM_030928.2 | 1433 | tgctgcggagcgtctttgtgtcc | 395532 | - | see also 20856 line |
| 47144 | CDT1 | NM_030928.2 | 753 | gcgcaatgttggccagatcaaaa | 395530 | - | see also 20856 line |
| 47145 | CDT1 | NM_030928.2 | 602 | gactcgtgctgccctacaagtac | 395529 | - | see also 20856 line |
| 47146 | CDV-1 | NM_014055.2 | 1519 | aaggcaaactcactgtatagg | 339431 | - | see also 5472 line |
| 47147 | CDV-1 | NM_014055.2 | 774 | ttggtgattggaagtaaacctgt | 339423 | - | see also 5472 line |
| 47148 | CEAL1 | NM_020219.1 | 274 | tacggaggcacgagagctatttac | 376243 | - | see also 20858 line |
| 47149 | CEAL1 | NM_020219.1 | 279 | aaggcacgagagctatttacctaca | 376246 | - | see also 20858 line |
| 47150 | CECR5 | NM_017829.4 | 1107 | tccaacggcaccgagacttatgc | 362838 | - | see also 20859 line |
| 47151 | CECR5 | NM_017829.4 | 198 | gggtgcccgtgttttgttacca | 362828 | - | see also 20859 line |
| 47152 | CEI | NM_178569.2 | 429 | gccggtgcagtggggcattacagg | 431333 | - | see also 20861 line |
| 47153 | CEI | NM_178569.2 | 332 | aacactggggggatggattgttac | 431332 | - | see also 20861 line |
| 47154 | CG018 | NM_052818.1 | 631 | aacggtatgaaacacgatgttact | 404260 | - | see also 9432 line |
| 47155 | CG018 | NM_052818.1 | 774 | aaggcccacggtggatttacaaa | 404264 | - | see also 9432 line |
| 47156 | CGEF2 | NM_007023.1 | 753 | ctctcgagcacctcacatgataa | 336803 | - | see also 4903 line |
| 47157 | CGEF2 | NM_007023.1 | 2623 | aagcgtgttcagctattaaaaaa | 336856 | - | see also 4903 line |
| 47158 | CGI-115 | NM_016052.2 | 745 | gacgatcctacgtgatgattca | 356114 | - | see also 20864 line |
| 47159 | CGI-115 | NM_016052.2 | 157 | atcggaaaaggaccacacttatt | 356099 | - | see also 20864 line |
| 47160 | CGI-116 | NM_016053.1 | 263 | ggcagaccttcacttcgtatcc | 356123 | - | see also 6642 line |
| 47161 | CGI-116 | NM_016053.1 | 272 | ttcacttcgtatccaacaaattg | 356124 | - | see also 6642 line |
| 47162 | CGI-116 | NM_016053.1 | 329 | atctatcccaggcctagatgatg | 356126 | - | see also 6642 line |
| 47163 | CGI-12 | NM_015942.3 | 515 | ttcgagactatgtggatccattct | 355535 | - | see also 20865 line |
| 47164 | CGI-12 | NM_015942.3 | 973 | aaggtttatcgtcttgaacttgg | 355547 | - | see also 20865 line |
| 47165 | CGI-125 | NM_016060.1 | 348 | tgctcagtgtggcgaaatttattg | 356144 | - | see also 6646 line |
| 47166 | CGI-125 | NM_016060.1 | 161 | tagccaaccaaattaccttaat | 356137 | - | see also 6646 line |
| 47167 | CGI-127 | NM_016061.1 | 150 | atcggtgtaccccgtctgtttc | 356149 | - | see also 20867 line |
| 47168 | CGI-127 | NM_016061.1 | 234 | ggcggccactgcagagcatttct | 356155 | - | see also 20867 line |
| 47169 | CGI-127 | NM_016061.1 | 393 | gacacgccagcggatataaggaagg | 356161 | - | see also 20867 line |
| 47170 | CGI-128 | NM_016062.1 | 164 | acgcgagatcttgatctgattc | 356165 | - | see also 20868 line |

Figure 46

| | | | | | | | |
|---|---|---|---|---|---|---|---|
| 47171 | CGI-128 | NM_016062.1 | 312 | tgcagcatggccacccttattgg | 356167 | - | see also 20868 line |
| 47172 | CGI-32 | NM_015960.1 | 370 | ttgtattcagatcgtgaaattga | 355636 | - | see also 20869 line |
| 47173 | CGI-32 | NM_015960.1 | 159 | ggccggagcagcaaatggatttc | 355626 | - | see also 20869 line |
| 47174 | CGI-38 | NM_015964.1 | 413 | gtgggcgtcactaaagcaaaaac | 355651 | - | -  \| -  \| - |
| 47175 | CGI-38 | NM_015964.1 | 411 | atgtgggcgtcactaaagcaaaa | 355650 | - | see also 47174 line |
| 47176 | CGI-38 | NM_015964.1 | 407 | gccaatgtgggcgtcactaaagc | 355649 | - | see also 47174 line |
| 47177 | CGI-48 | NM_016001.1 | 1300 | atgaaggcagtttatatggatta | 355731 | - | see also 20871 line |
| 47178 | CGI-48 | NM_016001.1 | 1435 | aagctataatgaacttggttaca | 355736 | - | see also 20871 line |
| 47179 | CGI-49 | NM_016002.1 | 283 | ctcaattgcgtaggaccatatcg | 355742 | - | see also 20872 line |
| 47180 | CGI-49 | NM_016002.1 | 812 | ttcagtatgctgcgtatgtaact | 355765 | - | see also 20872 line |
| 47181 | CGI-57 | NM_015680.3 | 881 | atcggtggccgtaatacatcagt | 354989 | - | see also 20873 line |
| 47182 | CGI-57 | NM_015680.3 | 878 | ggcatcggtggccgtaatacatc | 354988 | - | see also 20873 line |
| 47183 | CGI-57 | NM_015680.3 | 597 | gtggccgtgcccactctcaatgc | 354986 | - | see also 20873 line |
| 47184 | CGI-62 | NM_016010.1 | 563 | agccacccgcacttaaaaagtca | 355862 | - | see also 6610 line |
| 47185 | CGI-62 | NM_016010.1 | 566 | cacccgcacttaaaaagtcaaat | 355863 | - | see also 6610 line |
| 47186 | CGI-83 | NM_016027.1 | 437 | agccactctaagagttctatata | 355969 | - | see also 6627 line |
| 47187 | CGI-83 | NM_016027.1 | 432 | gagggagccactctaagagttct | 355967 | - | see also 6627 line |
| 47188 | CGI-85 | NM_016028.3 | 686 | atgggcacggcactattttctca | 355993 | - | see also 6628 line |
| 47189 | CGI-85 | NM_016028.3 | 689 | ggcacggcactattttctcaaca | 355995 | - | see also 6628 line |
| 47190 | CGI-90 | NM_016033.1 | 380 | atcagttgctaacccaatacaag | 356041 | - | see also 6632 line |
| 47191 | CGI-94 | NM_016037.1 | 809 | ttcagagtcgtcgaaaacgttga | 356071 | - | see also 20878 line |
| 47192 | CGI-94 | NM_016037.1 | 731 | cacgcaaagatcttatggataaa | 356067 | - | see also 20878 line |
| 47193 | CGI-94 | NM_016037.1 | 363 | aactcaggacgtcaaatatatag | 356061 | - | see also 20878 line |
| 47194 | CHPF | NM_024536.4 | 1059 | accacgagggggtgcactatagc | 386906 | - | see also 20879 line |
| 47195 | CHPF | NM_024536.4 | 738 | agcacggcgacgactttgactgg | 386905 | - | see also 20879 line |
| 47196 | CHPPR | NM_014637.2 | 133 | ggctggattaagcgcctaattag | 341564 | - | see also 5813 line |
| 47197 | CHPPR | NM_014637.2 | 553 | aagatttgcgctctcgaaaatga | 341571 | - | see also 5813 line |
| 47198 | CHRM4 | NM_000741.1 | 1358 | ctctgtgcaacgccacctttaaa | 329873 | - | see also 20881 line |
| 47199 | CHRM4 | NM_000741.1 | 75 | tcgctatgagacggtggaaatgg | 329872 | - | see also 20881 line |
| 47200 | CHRNG | NM_005199.3 | 405 | gtcccctgacggctgtatctact | 333560 | - | see also 20882 line |
| 47201 | CHRNG | NM_005199.3 | 446 | tccgttccgcctgctctatctca | 333561 | - | see also 20882 line |
| 47202 | CHSY1 | NM_014918.3 | 2377 | gtcgacctcgtgtttactacaga | 345661 | - | see also 20883 line |
| 47203 | CHSY1 | NM_014918.3 | 2374 | gacgtcgacctcgtgtttactac | 345659 | - | see also 20883 line |
| 47204 | CHSY1 | NM_014918.3 | 2405 | ttcagcgatgtcgagcaaataca | 345663 | - | see also 20883 line |
| 47205 | cig5 | NM_080657.3 | 235 | accccaaccagcgtcaactatca | 406537 | - | see also 20884 line |
| 47206 | cig5 | NM_080657.3 | 261 | cactcgccagtgcaactacaaat | 406539 | - | see also 20884 line |
| 47207 | cig5 | NM_080657.3 | 234 | caccccaaccagcgtcaactatc | 406536 | - | see also 20884 line |

Figure 46

| 47208 | CIRH1A | NM_032830.1 | 739 | agccatctcatcgctaatgctga | 401773 | - | see also 20885 line |
|---|---|---|---|---|---|---|---|
| 47209 | CIRH1A | NM_032830.1 | 65 | tccatcgagtacgtttctttaat | 401748 | - | see also 20885 line |
| 47210 | CIRH1A | NM_032830.1 | 661 | tccgatggcactatcataagtgt | 401772 | - | see also 20885 line |
| 47211 | CKAP2 | NM_018204.2 | 147 | ggcgcagtccgcattcaaagagc | 368427 | - | see also 20887 line |
| 47212 | CKAP2 | NM_018204.2 | 1971 | acgttctcgacgtcttcaagaga | 368474 | - | see also 20887 line |
| 47213 | CKIP-1 | NM_016274.3 | 1363 | gagaggcatcatcgaattggagc | 356664 | - | see also 20888 line |
| 47214 | CKIP-1 | NM_016274.3 | 599 | cacggcacccaacctgatcttcc | 356661 | - | see also 20888 line |
| 47215 | CKLFSF8 | NM_178868.3 | 451 | ctgctggtatggacgcttattgc | 432047 | - | see also 20889 line |
| 47216 | CKLFSF8 | NM_178868.3 | 754 | atctgctacgctggaaatacata | 432052 | - | see also 20889 line |
| 47217 | CL25022 | NM_015702.1 | 897 | aacgctaccgacatttaggattc | 355201 | - | see also 20890 line |
| 47218 | CL25022 | NM_015702.1 | 294 | ctgcacctccagatatatgctct | 355187 | - | see also 20890 line |
| 47219 | CLASP1 | NM_015282.1 | 1025 | cacagtcccggttgaatgtaatt | 350922 | - | see also 20891 line |
| 47220 | CLASP1 | NM_015282.1 | 1027 | cagtcccggttgaatgtaatttt | 350923 | - | see also 20891 line |
| 47221 | CLIP-2 | NM_153221.1 | 2279 | cgcctacgccaacgacaagttca | 422266 | - | see also 20892 line |
| 47222 | CLIP-2 | NM_153221.1 | 1656 | gtccgggtcttgccttttgatcc | 422264 | - | see also 20892 line |
| 47223 | CLIP-2 | NM_153221.1 | 1561 | ccgcctaccagggcgactttacc | 422261 | - | see also 20892 line |
| 47224 | CLIPR-59 | NM_015526.1 | 876 | accctgcgctgaggaatcgaaaa | 353708 | - | see also 20893 line |
| 47225 | CLIPR-59 | NM_015526.1 | 1227 | ggggcgttcggtacttcatctgc | 353713 | - | see also 20893 line |
| 47226 | CLONE25003 | NM_015381.1 | 424 | gggggaaggctgcgacttgttaa | 351951 | - | see also 20894 line |
| 47227 | CLONE25003 | NM_015381.1 | 175 | aactttctgggcgttcatgatcc | 351947 | - | see also 20894 line |
| 47228 | CLST11240 | NM_016438.2 | 488 | cacaatgtacagcgattacgtca | 357480 | - | see also 20895 line |
| 47229 | CLST11240 | NM_016438.2 | 394 | ctcgtggttccaccaagatgtcc | 357475 | - | see also 20895 line |
| 47230 | CMIP | NM_030629.1 | 933 | tgcgaccggaagcccactttacc | 394713 | - | see also 20896 line |
| 47231 | CMIP | NM_030629.1 | 1301 | agctctcgcacatgagaagttca | 394715 | - | see also 20896 line |
| 47232 | CMIP | NM_030629.1 | 646 | tgcagcgaatcctcaagcataac | 394710 | - | see also 20896 line |
| 47233 | CML66 | NM_032869.2 | 1614 | gccttcgtcgagtattcatctat | 402319 | - | see also 9325 line |
| 47234 | CML66 | NM_032869.2 | 684 | tggagtgggtcactatcagtaag | 402296 | - | see also 9325 line |
| 47235 | CMRF-35H | NM_007261.1 | 890 | ctcggtggtgtttgattctaaca | 337363 | - | see also 20898 line |
| 47236 | CMRF-35H | NM_007261.1 | 888 | gcctcggtggtgtttgattctaa | 337362 | - | see also 20898 line |
| 47237 | CMT4B2 | NM_030962.1 | 1986 | tacaacattgccgcagcattact | 395769 | - | see also 8980 line |
| 47238 | CMT4B2 | NM_030962.1 | 3102 | ctgatgaagttccgttatcctca | 395799 | - | see also 8980 line |
| 47239 | CMYA1 | NM_194293.2 | 2652 | gggcaggggcacccttatatacg | 436467 | - | see also 20900 line |

Figure 46

| 47240 | CMYA1 | NM_194293.2 | 2655 | caggggcacccttatatacgaaa | 436468 | - | see also 20900 line |
|---|---|---|---|---|---|---|---|
| 47241 | CMYA3 | NM_152381.2 | 3731 | gagagctacttatgttcataaag | 416007 | - | see also 20901 line |
| 47242 | CMYA3 | NM_152381.2 | 3734 | agctacttatgttcataaagatg | 416008 | - | see also 20901 line |
| 47243 | CMYA4 | NM_173167.1 | 946 | gacaactcacgtaccatctatgt | 424788 | - | see also 20902 line |
| 47244 | CMYA4 | NM_173167.1 | 1506 | tgcggaagggtcgacagaaaaac | 424793 | - | see also 20902 line |
| 47245 | CMYA5 | NM_153610.2 | 1687 | aagttatgcggttccatttgaag | 423346 | - | see also 20903 line |
| 47246 | CMYA5 | NM_153610.2 | 1176 | cacgaacccagatatttcctacc | 423337 | - | see also 20903 line |
| 47247 | CNAP1 | NM_014865.1 | 4517 | ctccgtaagatgcttgacaattt | 345005 | - | see also 6064 line |
| 47248 | CNAP1 | NM_014865.1 | 1487 | gccttgacccgttataaccatat | 344975 | - | see also 6064 line |
| 47249 | CNNM1 | NM_020348.1 | 1842 | tagcgattttgaggaaaacgtgg | 376816 | - | see also 20905 line |
| 47250 | CNNM1 | NM_020348.1 | 660 | cacggttctggaggagtttaaga | 376803 | - | see also 20905 line |
| 47251 | CNNM1 | NM_020348.1 | 765 | cacgctggaggatatcatagagg | 376806 | - | see also 20905 line |
| 47252 | CNNM4 | NM_020184.1 | 1091 | tcgaagacgagcagtccaatatt | 375847 | - | see also 7789 line |
| 47253 | CNNM4 | NM_020184.1 | 1174 | aagactatcactcgcttctataa | 375853 | - | see also 7789 line |
| 47254 | CNOT10 | NM_015442.1 | 1293 | gtgtacgttactaaccaataaga | 352886 | - | see also 6418 line |
| 47255 | CNOT10 | NM_015442.1 | 2439 | cccgatccagcccatccaaatgc | 352922 | - | see also 6418 line |
| 47256 | CNOT3 | NM_014516.1 | 3596 | gtcgaccgagacactcttcttca | 340823 | - | see also 5745 line |
| 47257 | CNOT3 | NM_014516.1 | 1969 | ttggccagtggctcacgaatacc | 340817 | - | see also 5745 line |
| 47258 | CNOT6L | NM_144571.1 | 580 | tacgctacccggcagctatatgg | 409789 | - | see also 9693 line |
| 47259 | CNOT6L | NM_144571.1 | 232 | ttgacagcgctgcacctaaatga | 409777 | - | see also 9693 line |
| 47260 | CNTN4 | NM_175607.1 | 2933 | ccccggaaacatcatatggaatt | 429138 | - | see also 10145 line |
| 47261 | CNTN4 | NM_175607.1 | 2882 | ctctagtgcaacagtcaatgtga | 429137 | - | see also 10145 line |
| 47262 | CNTN5 | NM_014361.2 | 2628 | tcgaatgatccgcacaaatgaag | 340337 | - | see also 20912 line |
| 47263 | CNTN5 | NM_014361.2 | 1596 | cacgtatcgttggctgaagaatg | 340304 | - | see also 20912 line |
| 47264 | COBL | NM_015198.2 | 2271 | agcacggcctcacaacgtataaa | 349692 | - | see also 20913 line |
| 47265 | COBL | NM_015198.2 | 2273 | cacggcctcacaacgtataaaat | 349693 | - | see also 20913 line |
| 47266 | COBRA1 | NM_015456.2 | 196 | ttccaccagtcggtattcgatga | 353229 | - | see also 20914 line |
| 47267 | COBRA1 | NM_015456.2 | 1235 | ttcccgaaagcttcactaagttt | 353236 | - | see also 20914 line |
| 47268 | COBRA1 | NM_015456.2 | 1236 | tcccgaaagcttcactaagtttc | 353237 | - | see also 20914 line |
| 47269 | COH1 | NM_015243.2 | 218 | tactcagcaagctcgagttaaag | 350162 | - | see also 20915 line |
| 47270 | COH1 | NM_015243.2 | 536 | gtctgattagacgagttgtaaat | 350174 | - | see also 20915 line |
| 47271 | COH1 | NM_015243.2 | 538 | ctgattagacgagttgtaaataa | 350175 | - | see also 20915 line |
| 47272 | COL13A1 | NM_005203.3 | 781 | agcaaatggagacggctattttg | 333571 | - | see also 3655 line |
| 47273 | COL23A1 | NM_173465.2 | 1273 | cacagtggtgatcgactatgatg | 424843 | - | see also 20917 line |
| 47274 | COL23A1 | NM_173465.2 | 1366 | agccaagggcgagcttggattgc | 424844 | - | see also 20917 line |
| 47275 | COL24A1 | NM_152890.4 | 336 | ttggggcagccgtttacaatatt | 421431 | - | see also 20918 line |
| 47276 | COL24A1 | NM_152890.4 | 337 | tggggcagccgtttacaatatta | 421432 | - | see also 20918 line |

Figure 46

| 47277 | COL25A1 | NM_032518.2 | 1832 | acctccacgaagccttacagagg | 400521 | - | see also 20919 line |
|---|---|---|---|---|---|---|---|
| 47278 | COL25A1 | NM_032518.2 | 1838 | acgaagccttacagaggattacc | 400522 | - | see also 20919 line |
| 47279 | COL26A1 | NM_133457.1 | 266 | gcgccaacctcgtaagttacagg | 407231 | - | see also 20920 line |
| 47280 | COL26A1 | NM_133457.1 | 377 | gtgatgaggaatgcatgaactgc | 407232 | - | see also 20920 line |
| 47281 | COL8A2 | NM_005202.1 | 1939 | cacctatacctacgatgagtaca | 333570 | - | see also 20921 line |
| 47282 | COL8A2 | NM_005202.1 | 1768 | ctcgggcatgcccgtgaaatttg | 333567 | - | see also 20921 line |
| 47283 | COP1 | NM_022457.4 | 1233 | gtcatatgctagtgatctctata | 382885 | - | see also 8376 line |
| 47284 | COPS3 | NM_003653.2 | 943 | aaccttcactcgcgataacaaca | 331906 | - | see also 2472 line |
| 47285 | COPS3 | NM_003653.2 | 733 | ggcggtcagtcatatcatgttgg | 331898 | - | see also 2472 line |
| 47286 | COPS3 | NM_003653.2 | 727 | tgccatggcggtcagtcatatca | 331896 | - | see also 2472 line |
| 47287 | COPS4 | NM_016129.2 | 181 | ttcgagcggctctcataaagatc | 356372 | - | see also 6680 line |
| 47288 | COPS4 | NM_016129.2 | 828 | tgcactgtacgatcttagcatca | 356392 | - | see also 6680 line |
| 47289 | COPS4 | NM_016129.2 | 781 | gacaatagtccacgaaagtgaaa | 356391 | - | see also 6680 line |
| 47290 | COPS7A | NM_016319.1 | 358 | gggacatacgctgactacttagc | 356946 | - | see also 20925 line |
| 47291 | COPS7A | NM_016319.1 | 357 | tgggacatacgctgactacttag | 356945 | - | see also 20925 line |
| 47292 | COPS7B | NM_022730.1 | 263 | gagcttgcggaaggagctaatgc | 383736 | - | see also 8434 line |
| 47293 | COPS7B | NM_022730.1 | 319 | tgggacatacccagattacatag | 383739 | - | see also 8434 line |
| 47294 | CORTBP2 | NM_033427.1 | 4030 | ccgtctggcgtcagcttaactcc | 403970 | - | see also 20927 line |
| 47295 | CORTBP2 | NM_033427.1 | 1660 | cagttggtcggaccagtttaaag | 403919 | - | see also 20927 line |
| 47296 | COX7B2 | NM_130902.1 | 193 | tggcaagacatagccatgtaaaa | 407152 | - | see also 20928 line |
| 47297 | COX7B2 | NM_130902.1 | 199 | gacatagccatgtaaaacactca | 407155 | - | see also 20928 line |
| 47298 | COX8-3 | NM_182971.1 | 241 | ttcttaacaccagctgcatatgt | 435503 | - | - | - | - |
| 47299 | COX8-3 | NM_182971.1 | 226 | gtgttttttacgaccttcttaac | 435502 | - | see also 47298 line |
| 47300 | COX8-3 | NM_182971.1 | 215 | ttggacttgtggtgtttttacg | 435501 | - | see also 47298 line |
| 47301 | CPEB2 | NM_182485.1 | 607 | gtcatagcaccaccgaaatttac | 433841 | - | see also 10259 line |
| 47302 | CPEB2 | NM_182485.1 | 1579 | gccattagtgctcggtttgttca | 433872 | - | see also 10259 line |
| 47303 | CPLX4 | NM_181654.1 | 380 | ttctattcttgggcagatacaga | 432802 | - | see also 20930 line |
| 47304 | CPLX4 | NM_181654.1 | 323 | acctgaagatctccggaaaatgg | 432800 | - | see also 20930 line |
| 47305 | CPNE2 | NM_152727.4 | 1222 | ccctatgggcaccaacgaatatc | 420120 | - | see also 9933 line |
| 47306 | CPNE2 | NM_152727.4 | 1445 | cgcttctacggtcctaccaattt | 420125 | - | see also 9933 line |
| 47307 | CPNE2 | NM_152727.4 | 418 | gacagaaaccgcgatcaacaacc | 420106 | - | see also 9933 line |
| 47308 | CPNE4 | NM_130808.1 | 1106 | gccggctaaagtgcatagtatgg | 406953 | - | see also 20932 line |
| 47309 | CPNE4 | NM_130808.1 | 912 | tgcattcaatgcacggaaattgg | 406943 | - | see also 20932 line |
| 47310 | CPNE5 | NM_020939.1 | 356 | tacgacgttgactctaagagtcc | 380202 | - | see also 20933 line |
| 47311 | CPNE5 | NM_020939.1 | 332 | aagcagaacctccgttttgatct | 380200 | - | see also 20933 line |
| 47312 | CPNE5 | NM_020939.1 | 335 | cagaacctccgttttgatctata | 380201 | - | see also 20933 line |
| 47313 | CPNE8 | NM_153634.2 | 554 | gggatgccgttttgatgcaattt | 423419 | - | see also 10002 line |

Figure 46

| | | | | | | | |
|---|---|---|---|---|---|---|---|
| 47314 | CPNE8 | NM_153634.2 | 1744 | acccccacctacacatgtgttaca | 423453 | - | see also 10002 line |
| 47315 | CPR8 | NM_004748.2 | 2434 | atggacccaggtcggttacata | 332905 | - | see also 20935 line |
| 47316 | CPR8 | NM_004748.2 | 1374 | gagagagaacgactagtaactac | 332873 | - | see also 20935 line |
| 47317 | CPXCR1 | NM_033048.3 | 823 | gccttcgaggtgcactactacc | 402688 | - | see also 20936 line |
| 47318 | CPXCR1 | NM_033048.3 | 540 | agcccttaaatgatagatcaaga | 402681 | - | see also 20936 line |
| 47319 | CRA | NM_006697.1 | 484 | ctggtcaacattggacgattaga | 336043 | - | see also 20937 line |
| 47320 | CRA | NM_006697.1 | 688 | agcaatcaagcccaacagtattc | 336049 | - | see also 20937 line |
| 47321 | CREB3L2 | NM_194071.1 | 850 | ttcctcgtgccagaccattattc | 436044 | - | see also 20938 line |
| 47322 | CREB3L2 | NM_194071.1 | 451 | cacgcacttctcagaacttctgg | 436042 | - | see also 20938 line |
| 47323 | CREB3L2 | NM_194071.1 | 523 | ctcagagagagtgtgtcaatgg | 436043 | - | see also 20938 line |
| 47324 | CREG2 | NM_153836.1 | 953 | gaggcgcatccagtatttcaagg | 423954 | - | see also 20939 line |
| 47325 | CREG2 | NM_153836.1 | 885 | gcctcgtcaatatgaatggttct | 423950 | - | see also 20939 line |
| 47326 | CRG-L2 | NM_181789.1 | 1005 | gacggctcgagcattcttgtagc | 433494 | - | see also 20940 line |
| 47327 | CRG-L2 | NM_181789.1 | 1051 | cagtggtgcaacacgtcaatacc | 433495 | - | see also 20940 line |
| 47328 | CRIPT | NM_014171.2 | 324 | tgcgatgtgtggaaaaaaggttt | 339836 | - | see also 20941 line |
| 47329 | CRIPT | NM_014171.2 | 130 | tggaaagatggtgctaggaatac | 339830 | - | see also 20941 line |
| 47330 | CROC4 | NM_006365.1 | 122 | tcctgactgaggatctcataaca | 335348 | - | see also 20942 line |
| 47331 | CROC4 | NM_006365.1 | 527 | ggctaagttgggagaccaaaaag | 335353 | - | see also 20942 line |
| 47332 | CRR9 | NM_030782.2 | 1223 | aaccgaggagtacgatacttcagg | 394981 | - | see also 8945 line |
| 47333 | CRR9 | NM_030782.2 | 1282 | gtgtcgggggtgtctgtctattca | 394984 | - | see also 8945 line |
| 47334 | CRTAM | NM_019604.1 | 750 | ttctagtacatcggagattgaca | 375469 | - | see also 20944 line |
| 47335 | CRTAM | NM_019604.1 | 296 | tacattacagccgactctgtaagc | 375454 | - | see also 20944 line |
| 47336 | CRYGN | NM_144727.1 | 374 | aacatttccgcctagaaatcttc | 411864 | - | see also 20945 line |
| 47337 | CRYGN | NM_144727.1 | 101 | cgcagcgctcggggaagatcact | 411860 | - | see also 20945 line |
| 47338 | CSE-C | NM_018978.1 | 1697 | accctggcgatcgattcttgtca | 373985 | - | see also 20946 line |
| 47339 | CSE-C | NM_018978.1 | 1130 | taccagggggagtccaatataaa | 373967 | - | see also 20946 line |
| 47340 | CSMD2 | NM_052896.2 | 2592 | ctccgctatagactataacact | 404646 | - | see also 20947 line |
| 47341 | CSMD2 | NM_052896.2 | 1472 | atcggtcccgatctcattgtca | 404630 | - | see also 20947 line |
| 47342 | CSMD3 | NM_052900.2 | 3791 | tgcgcggacttgacacttagtagt | 404909 | - | see also 20948 line |
| 47343 | CSMD3 | NM_052900.2 | 7796 | taggaacgcgagttacctatttt | 405056 | - | see also 20948 line |
| 47344 | CSTL1 | NM_138283.1 | 415 | aacgacacttacttattatcgagt | 407374 | - | see also 20949 line |
| 47345 | CSTL1 | NM_138283.1 | 471 | gacgggagtggagtatatagtca | 407378 | - | see also 20949 line |
| 47346 | CTAG3 | NM_175745.2 | 743 | accaacttggtaacgagtatttt | 429228 | - | see also 20950 line |
| 47347 | CTAG3 | NM_175745.2 | 519 | ctcaacgaatgcactaattcagc | 429223 | - | see also 20950 line |
| 47348 | CTAGE-2 | NM_172241.1 | 119 | ccgattctcatccttatggttt | 424367 | - | see also 20951 line |
| 47349 | CTCFL | NM_080618.2 | 1073 | cacaggcgctataaacatactca | 406480 | - | see also 20952 line |
| 47350 | CTCFL | NM_080618.2 | 1067 | gtccgacacaggcgctataaaaca | 406477 | - | see also 20952 line |

Figure 46

| | | | | | | | |
|---|---|---|---|---|---|---|---|
| 47351 | CTHRC1 | NM_138455.2 | 495 | ttcaaatagtgctctaagagttt | 408277 | - | see also 20953 line |
| 47352 | CTHRC1 | NM_138455.2 | 560 | gtcagcgttgtatttcacattc | 408281 | - | see also 20953 line |
| 47353 | CTL2 | NM_020428.2 | 467 | cccgggactttgagtactataag | 377437 | - | see also 20954 line |
| 47354 | CTL2 | NM_020428.2 | 115 | cacggaacgccacagagtatga | 377425 | - | see also 20954 line |
| 47355 | CTXL | NM_014312.3 | 305 | ttcaccaatgccatcgtatcc | 340143 | - | see also 20955 line |
| 47356 | CTXL | NM_014312.3 | 288 | cccatccaatcctgacttcacc | 340142 | - | see also 20955 line |
| 47357 | CXorf15 | NM_018360.1 | 698 | agcgtcacaataagacgttaaag | 370751 | - | see also 20956 line |
| 47358 | CXorf15 | NM_018360.1 | 1302 | aacagtccgtgataaagagtaca | 370753 | - | see also 20956 line |
| 47359 | CYFIP1 | NM_014608.1 | 1016 | agcgcccactacgaggaaataaa | 341041 | - | see also 5772 line |
| 47360 | CYFIP1 | NM_014608.1 | 1670 | aagagcggcttcgacataaaagt | 341052 | - | see also 5772 line |
| 47361 | CYFIP1 | NM_014608.1 | 2217 | ttcttgataaacgttacgatca | 341067 | - | see also 5772 line |
| 47362 | CYFIP2 | NM_014376.1 | 2484 | caccgtccaatcgctatgaaaca | 340455 | - | see also 5638 line |
| 47363 | CYFIP2 | NM_014376.1 | 2426 | ctgttgataaacgtttcgagc | 340452 | - | see also 5638 line |
| 47364 | CYLN2 | NM_003388.3 | 2006 | gtgggtgctgcgggataaatac | 331651 | - | see also 20959 line |
| 47365 | CYLN2 | NM_003388.3 | 670 | gacccggtgggcaagaatgatgg | 331647 | - | see also 20959 line |
| 47366 | CYorf15B | NM_032576.1 | 671 | gggcaggtctgtgaaagtagc | 400827 | - | see also 20960 line |
| 47367 | CYorf15B | NM_032576.1 | 816 | atgaagtaaaatgctactcaaaa | 400828 | - | see also 20960 line |
| 47368 | CYP26C1 | NM_183374.1 | 797 | ccctcgacctaatcattcacagt | 435691 | - | see also 20961 line |
| 47369 | CYP26C1 | NM_183374.1 | 791 | gtgatgccctgacctaatcatt | 435689 | - | see also 20961 line |
| 47370 | D2LIC | NM_015522.2 | 406 | gacaccttacggacgtttctct | 353690 | - | see also 20962 line |
| 47371 | D2LIC | NM_015522.2 | 409 | accttacggacgttttctcttgt | 353692 | - | see also 20962 line |
| 47372 | D2LIC | NM_015522.2 | 408 | caccttacggacgttttcttg | 353691 | - | see also 20962 line |
| 47373 | D6S2654E | NM_012135.1 | 442 | gcgcgagcgcaagcgtaagatct | 337692 | - | see also 20963 line |
| 47374 | D6S2654E | NM_012135.1 | 444 | gcgagcgcaagcgtaagatctcc | 337693 | - | see also 20963 line |
| 47375 | DAB2IP | NM_032552.1 | 1791 | aacgatctttccggtctgatga | 400672 | - | see also 20964 line |
| 47376 | DAB2IP | NM_032552.1 | 2751 | atgaacgcgcagttgttagaaga | 400680 | - | see also 20964 line |
| 47377 | DARP | NM_144994.5 | 403 | acctgattgacaagtacttgaca | 412504 | - | see also 20965 line |
| 47378 | DATF1 | NM_022105.2 | 2068 | tccctcgcgtcactgttgtata | 382362 | - | see also 20966 line |
| 47379 | DATF1 | NM_022105.2 | 2071 | ctcgccgtcactgttgttataaat | 382364 | - | see also 20966 line |
| 47380 | DBC-1 | NM_021174.4 | 1320 | gacagtgacccgcttatagttc | 380568 | - | see also 20967 line |
| 47381 | DBC-1 | NM_021174.4 | 1627 | ggcgctttgccgagtttcagtac | 380570 | - | see also 20967 line |
| 47382 | DBC1 | NM_014618.1 | 1150 | gtcggccttgagctatatcatgt | 341351 | - | see also 5789 line |
| 47383 | DBC1 | NM_014618.1 | 1306 | ttgggccgaagcttataaggacc | 341354 | - | see also 5789 line |
| 47384 | DBCCR1L | NM_199051.1 | 1667 | gccgagtccacgatcactatat | 440692 | - | see also 10360 line |
| 47385 | DBCCR1L | NM_199051.1 | 1666 | cgccgagtccacgatcactacta | 440691 | - | see also 10360 line |
| 47386 | DC-UbP | NM_152277.1 | 541 | aggtagtcagcgggtgtttttt | 415304 | - | see also 9826 line |
| 47387 | DC-UbP | NM_152277.1 | 540 | caggtagtcagcgggtgttttt | 415303 | - | see also 9826 line |

Figure 46

| 47388 | DC12 | NM_020187.1 | 108 | gggcgaacatcctgtcacttacc | 375872 | - | see also 20971 line |
|---|---|---|---|---|---|---|---|
| 47389 | DC12 | NM_020187.1 | 443 | gggaagacgctgtgtcgttttag | 375877 | - | see also 20971 line |
| 47390 | DC13 | NM_020188.1 | 210 | aagaatgcaacgtcttgattaac | 375883 | - | see also 20972 line |
| 47391 | DC13 | NM_020188.1 | 291 | atgttgatcgggagttgagaaaa | 375887 | - | see also 20972 line |
| 47392 | DC6 | NM_020189.4 | 100 | gagcagcgattaaccaaaagttg | 375894 | - | see also 20973 line |
| 47393 | DC6 | NM_020189.4 | 98 | gagagcagcgattaaccaaaagt | 375893 | - | see also 20973 line |
| 47394 | DCLRE1A | NM_014881.1 | 1255 | aagatacgtccagtttatgatgg | 345137 | - | see also 6075 line |
| 47395 | DCLRE1A | NM_014881.1 | 2011 | ttcttaactcgggataagtatga | 345158 | - | see also 6075 line |
| 47396 | DCLRE1B | NM_022836.2 | 347 | atcgtcacctacaggtatctaag | 384492 | - | see also 20975 line |
| 47397 | DCLRE1B | NM_022836.2 | 912 | gacccaccctacgattgctatcc | 384506 | - | see also 20975 line |
| 47398 | DCLRE1C | NM_022487.1 | 650 | agcggcttatggctatgaatatc | 383397 | - | see also 20976 line |
| 47399 | DCLRE1C | NM_022487.1 | 614 | tcggagcccgtaccatgttgtgt | 383395 | - | see also 20976 line |
| 47400 | DCNP1 | NM_130848.1 | 844 | gcctggtcttcgcacttgatatc | 407096 | - | see also 20977 line |
| 47401 | DCNP1 | NM_130848.1 | 623 | cccggaaacatctggtttgtagt | 407093 | - | see also 20977 line |
| 47402 | DCOHM | NM_032151.2 | 288 | cacccagaatggttcaatgtata | 398390 | - | see also 20978 line |
| 47403 | DCOHM | NM_032151.2 | 170 | aggatggtcggaattaagtgaga | 398385 | - | see also 20978 line |
| 47404 | DCP1B | NM_152640.3 | 1490 | gccagccttggccgctaagtttc | 419445 | - | see also 20979 line |
| 47405 | DCP1B | NM_152640.3 | 1179 | aacaagtgtgaccctagtacacc | 419442 | - | see also 20979 line |
| 47406 | DCP2 | NM_152624.3 | 544 | caggggtacctagcaaaatcagg | 419115 | - | see also 20980 line |
| 47407 | DCP2 | NM_152624.3 | 851 | ttggtttggcacctaacaaattt | 419124 | - | see also 20980 line |
| 47408 | DDA3 | NM_032636.4 | 555 | ctcgactccggagtaatgatagg | 401158 | - | see also 20981 line |
| 47409 | DDA3 | NM_032636.4 | 551 | acgcctcgactccggagtaatga | 401157 | - | see also 20981 line |
| 47410 | DDX27 | NM_017895.6 | 1327 | atccggatccggcctaatcgtga | 363686 | - | see also 20982 line |
| 47411 | DDX27 | NM_017895.6 | 115 | ctcggcttaatcggaaccatagg | 363673 | - | see also 20982 line |
| 47412 | DEADC1 | NM_182503.1 | 754 | gtggagccgtgcattatgtgtgc | 433981 | - | see also 20983 line |
| 47413 | DEADC1 | NM_182503.1 | 777 | agctgctctccgcctgatgaaaa | 433982 | - | see also 20983 line |
| 47414 | DEPC-1 | NM_139178.1 | 606 | ctgagccacgagtgattgacaga | 409432 | - | see also 20984 line |
| 47415 | DEPC-1 | NM_139178.1 | 650 | ctgtcacccacaggtgtatctag | 409434 | - | see also 20984 line |
| 47416 | DERP6 | NM_015362.2 | 127 | aaggcgcttgtcaagaaatctgc | 351822 | - | see also 20985 line |
| 47417 | DERP6 | NM_015362.2 | 234 | tcggctggtttaccatgacttct | 351825 | - | see also 20985 line |
| 47418 | DEXI | NM_014015.3 | 621 | cccggctcgggcctttacgatgc | 339143 | - | see also 20986 line |
| 47419 | DEXI | NM_014015.3 | 647 | ctcggagctcgacgtctttgatg | 339144 | - | see also 20986 line |
| 47420 | DIBD1 | NM_024740.1 | 215 | cggagcaccggaccgagttatct | 389771 | - | see also 8718 line |
| 47421 | DIBD1 | NM_024740.1 | 863 | cgctgatggccctcatactattt | 389790 | - | see also 8718 line |
| 47422 | DIO3OS | NM_022345.2 | 661 | cccctccactggcctcttgaaag | 382624 | - | - | - | - |
| 47423 | DIP13B | NM_018171.2 | 1560 | acgccgattcaattcgatattgt | 367956 | - | see also 20988 line |
| 47424 | DIP13B | NM_018171.2 | 1559 | aacgccgattcaattcgatattg | 367955 | - | see also 20988 line |

Figure 46

| | | | | | | | | |
|---|---|---|---|---|---|---|---|---|
| 47425 | DIRC1 | NM_052952.1 | 520 | ctgtgaggctaaagtgtcttaac | 405503 | - | see also 20989 line |
| 47426 | DIRC1 | NM_052952.1 | 526 | ggctaaagtgtcttaactcattg | 405504 | - | see also 20989 line |
| 47427 | DIRC2 | NM_032839.1 | 788 | agcagcagggcgcatattaaaga | 401897 | - | see also 9304 line |
| 47428 | DIRC2 | NM_032839.1 | 810 | atcgcatagaggctgtgttatat | 401900 | - | see also 9304 line |
| 47429 | DIRC2 | NM_032839.1 | 939 | atcggagaagcgtttgtagatta | 401905 | - | see also 9304 line |
| 47430 | DISP2 | NM_033510.1 | 2914 | tccgccgtggttggttcactagc | 404048 | - | see also 20991 line |
| 47431 | DISP2 | NM_033510.1 | 4270 | ctcacacgtcaggctatagcagc | 404054 | - | see also 20991 line |
| 47432 | DJ1042K10.2 | NM_015705.3 | 581 | gtcctaccgcgagattgtgaaga | 355216 | - | see also 6539 line |
| 47433 | DJ1042K10.2 | NM_015705.3 | 268 | cagagcaaccagagttctactac | 355215 | - | see also 6539 line |
| 47434 | DJ122O8.2 | NM_020466.3 | 207 | tgccaccgaagaggatacaatcc | 377729 | - | see also 20993 line |
| 47435 | DJ122O8.2 | NM_020466.3 | 262 | aaggagttagaaaaaacacttgc | 377730 | - | see also 20993 line |
| 47436 | DJ167A19.1 | NM_018982.3 | 979 | caccgcaatactgtggattatcc | 374005 | - | see also 7641 line |
| 47437 | DJ167A19.1 | NM_018982.3 | 801 | tgcccgaattccgaaaagtgtcc | 374002 | - | see also 7641 line |
| 47438 | DJ465N24.2.1 | NM_020317.2 | 344 | gcgtctcgagccggttttcgtcc | 376480 | - | see also 20995 line |
| 47439 | DJ465N24.2.1 | NM_020317.2 | 425 | ggcgctactcgcggtcatactcg | 376482 | - | see also 20995 line |
| 47440 | DJ465N24.2.1 | NM_020317.2 | 833 | gccgtggaataggtgtatcaagt | 376492 | - | see also 20995 line |
| 47441 | DJ667H12.2 | NM_019605.2 | 555 | ctccgaagatctgtccttataaa | 375489 | - | see also 20996 line |
| 47442 | DJ667H12.2 | NM_019605.2 | 490 | aacgagcccacatcctttatatg | 375486 | - | see also 20996 line |
| 47443 | DJ79P11.1 | NM_032621.1 | 117 | aacgagcgttaaacaatctcatc | 401125 | - | see also 20997 line |
| 47444 | DJ79P11.1 | NM_032621.1 | 118 | acgagcgttaaacaatctcatcg | 401126 | - | see also 20997 line |
| 47445 | DKFZp313N0621 | NM_173826.1 | 420 | tccggatttcgagcagtcaaatt | 428404 | - | see also 20998 line |
| 47446 | DKFZp313N0621 | NM_173826.1 | 392 | ctccgatggccaggaacctttta | 428399 | - | see also 20998 line |
| 47447 | DKFZp313N0621 | NM_173826.1 | 1251 | ctcgggcattttaatattccaac | 428431 | - | see also 20998 line |
| 47448 | DKFZP434A1022 | NM_021185.3 | 1098 | atcgcgggtgagtatactctact | 380669 | - | see also 20999 line |
| 47449 | DKFZP434A1022 | NM_021185.3 | 1095 | tacatcgcgggtgagtatactct | 380668 | - | see also 20999 line |

EP 1 752 536 A1

Figure 46

| 47450 | DKFZP434 A1022 | NM_021185.3 | 2342 | ttcgattacgctcaaggataaaa | 380696 | - | see also 20999 line |
|---|---|---|---|---|---|---|---|
| 47451 | DKFZP434 A1315 | NM_032132.2 | 596 | ctggggcctttacctaatgatgt | 398069 | - | see also 21000 line |
| 47452 | DKFZP434 A1315 | NM_032132.2 | 893 | caggagcattatacaagtgatga | 398078 | - | see also 21000 line |
| 47453 | DKFZP434 A1319 | NM_032140.1 | 331 | gacgctctgtcctgacaactaca | 398321 | - | see also 21001 line |
| 47454 | DKFZP434 A1319 | NM_032140.1 | 1271 | gaggccatcaagatcttttctcg | 398325 | - | see also 21001 line |
| 47455 | DKFZp434 A1721 | NM_017609.2 | 114 | agcgtccagggcctattgtcact | 359415 | - | see also 21002 line |
| 47456 | DKFZp434 A1721 | NM_017609.2 | 1795 | tagtcgactcagacaacttcaag | 359432 | - | see also 21002 line |
| 47457 | DKFZP434 B0335 | NM_015395.1 | 2567 | cacacggcctgggtatacacagg | 352211 | - | see also 21003 line |
| 47458 | DKFZP434 B0335 | NM_015395.1 | 2818 | ttccgactggttcgtggatttca | 352215 | - | see also 21003 line |
| 47459 | DKFZp434 B0417 | NM_013377.2 | 2269 | gccgtcgccgtgagttcatgatg | 338898 | - | see also 21004 line |
| 47460 | DKFZp434 B0417 | NM_013377.2 | 1211 | gagcctcgtatctggtgaataca | 338878 | - | see also 21004 line |
| 47461 | DKFZp434 B1231 | NM_178275.3 | 881 | gacggcaggttggacatctatgg | 430685 | - | see also 21005 line |
| 47462 | DKFZp434 B1231 | NM_178275.3 | 1241 | gtccaggattccctaccattgc | 430687 | - | see also 21005 line |
| 47463 | DKFZP434 B168 | NM_015434.1 | 2625 | accaggactcttccgcaaaattc | 352835 | - | see also 21006 line |
| 47464 | DKFZP434 B168 | NM_015434.1 | 2605 | gtggttcagcacggatctaaacc | 352834 | - | see also 21006 line |
| 47465 | DKFZp434 B227 | NM_032263.2 | 1325 | gcccggactcatacagagattga | 399125 | - | see also 21007 line |
| 47466 | DKFZp434 B227 | NM_032263.2 | 966 | tgcaagattcggccacttacaac | 399117 | - | see also 21007 line |
| 47467 | DKFZp434 C0328 | NM_017577.1 | 2225 | cagaaaacgtttgatctactaaa | 359340 | - | see also 21008 line |
| 47468 | DKFZp434 C0328 | NM_017577.1 | 1516 | aagtcgggaagcacgattttatt | 359316 | - | see also 21008 line |
| 47469 | DKFZP434 C171 | NM_015621.1 | 759 | cactcccactgaggtctctttcc | 354617 | - | see also 21009 line |

EP 1 752 536 A1

Figure 46

| | | | | | | | |
|---|---|---|---|---|---|---|---|
| 47470 | DKFZp434 C1714 | NM_152834.2 | 313 | tgcgatgaactggagattatttt | 421378 | - | see also 21010 line |
| 47471 | DKFZp434 C1714 | NM_152834.2 | 361 | ggggatgttcatttctatagtat | 421384 | - | see also 21010 line |
| 47472 | DKFZP434 C245 | NM_015426.2 | 567 | atgtgtccactcgtatttgtgagc | 352632 | - | see also 21011 line |
| 47473 | DKFZP434 C245 | NM_015426.2 | 870 | ttcaagaacgggggagtatttg | 352636 | - | see also 21011 line |
| 47474 | DKFZP434 D1335 | NM_015578.1 | 929 | tcggcgagatgggccaatgaaat | 354309 | - | see also 21012 line |
| 47475 | DKFZP434 D1335 | NM_015578.1 | 367 | cactaggctcatcgacttcttca | 354299 | - | see also 21012 line |
| 47476 | DKFZP434 D146 | NM_015595.2 | 2009 | aagtttgttcgactatgcaatga | 354392 | - | see also 21013 line |
| 47477 | DKFZP434 D146 | NM_015595.2 | 291 | gccccaacgggttactaattacg | 354363 | - | see also 21013 line |
| 47478 | DKFZp434E 2220 | NM_017612.2 | 362 | ggactcgaccgagtggaatattgg | 359535 | - | see also 7049 line |
| 47479 | DKFZp434E 2220 | NM_017612.2 | 360 | ctgactcgaccgagtggaatatt | 359534 | - | see also 7049 line |
| 47480 | DKFZP434F 0318 | NM_030817.1 | 489 | tccatcgccctgtacaattctgt | 395349 | - | see also 21015 line |
| 47481 | DKFZP434F 0318 | NM_030817.1 | 323 | gttccgatctctgctgatcttct | 395344 | - | see also 21015 line |
| 47482 | DKFZP434F 0318 | NM_030817.1 | 485 | cgcctccatcgccctgtacaatt | 395347 | - | see also 21015 line |
| 47483 | DKFZP434F 1017 | NM_032135.2 | 346 | agctaccaccatcgtattggtaata | 398197 | - | see also 21016 line |
| 47484 | DKFZP434F 1017 | NM_032135.2 | 807 | atgccctgaaacataaatcgtcg | 398213 | - | see also 21016 line |
| 47485 | DKFZp434F 1719 | NM_032248.1 | 683 | tggcagtgagcctttgtttgttc | 399011 | - | see also 21017 line |
| 47486 | DKFZP434F 1719 | NM_032248.1 | 686 | cagtgagcctttgtttgttctga | 399012 | - | see also 21017 line |
| 47487 | DKFZP434F 2021 | NM_015412.1 | 1266 | aactagaatgcataaaaacgtct | 352326 | - | see also 21018 line |
| 47488 | DKFZP434F 2021 | NM_015412.1 | 1627 | ttcgttggacggtgatgcaataa | 352333 | - | see also 21018 line |
| 47489 | DKFZp434 G0522 | NM_017566.2 | 1014 | gcctgtcgggcgagttcttcaac | 359128 | - | see also 21019 line |

Figure 46

EP 1 752 536 A1

| | | | | | | | |
|---|---|---|---|---|---|---|---|
| 47490 | DKFZp434 G0522 | NM_017566.2 | 662 | aacgacgtgtatgcctttaatct | 359125 | - | see also 21019 line |
| 47491 | DKFZp434 G0625 | NM_181775.2 | 575 | ggccgtcaatcggatttacaagc | 433143 | - | see also 21020 line |
| 47492 | DKFZp434 G0625 | NM_181775.2 | 1623 | tccgacatggtgcgtggaattcc | 433155 | - | see also 21020 line |
| 47493 | DKFZP434 G072 | NM_032149.1 | 362 | gggcaaaggcagccatattatgg | 398354 | - | see also 21021 line |
| 47494 | DKFZP434 G072 | NM_032149.1 | 1215 | aagcctccactacttataagaag | 398380 | - | see also 21021 line |
| 47495 | DKFZp434 G118 | NM_032266.1 | 284 | ggccgatgttcaggacttaattg | 399165 | - | see also 21022 line |
| 47496 | DKFZp434 G118 | NM_032266.1 | 2417 | aggagccatcgtaggatttctga | 399213 | - | see also 21022 line |
| 47497 | DKFZP434 G156 | NM_022742.2 | 2636 | aactgtcgcaagacttatgatac | 383819 | - | see also 21023 line |
| 47498 | DKFZP434 G156 | NM_022742.2 | 988 | ctctgaaccatccggtagtttag | 383801 | - | see also 21023 line |
| 47499 | DKFZP434 H0115 | NM_031421.1 | 135 | ctctacctgtgcggggaattttc | 396891 | - | see also 21024 line |
| 47500 | DKFZP434 H0115 | NM_031421.1 | 1463 | gagagcaaagcttgtgcataaca | 396910 | - | see also 21024 line |
| 47501 | DKFZP434 H0820 | NM_022833.1 | 505 | tccttatgcgcgtcactactact | 384479 | - | see also 21025 line |
| 47502 | DKFZP434 H0820 | NM_022833.1 | 1198 | ggcgtaccacccctgaagatgc | 384485 | - | see also 21025 line |
| 47503 | DKFZP434 H2010 | NM_032129.1 | 1780 | acctctgggacgagactttgtct | 397954 | - | see also 21026 line |
| 47504 | DKFZP434 H2010 | NM_032129.1 | 422 | cggctcggaaggactcacatttc | 397952 | - | see also 21026 line |
| 47505 | DKFZp434 H2215 | NM_017559.1 | 157 | aaccggaccgtcactaccaaagg | 359077 | - | see also 21027 line |
| 47506 | DKFZp434 H2215 | NM_017559.1 | 703 | gagttgcccgaggcaaagaatcg | 359086 | - | see also 21027 line |
| 47507 | DKFZp434I 099 | NM_032269.3 | 1244 | gacgacagtgggataaacgatga | 399237 | - | see also 21028 line |
| 47508 | DKFZp434I 099 | NM_032269.3 | 1646 | atggaccgtgtcattgagtttta | 399242 | - | see also 21028 line |
| 47509 | DKFZp434I 099 | NM_032269.3 | 601 | ctcgaccactgtgctcaagtacc | 399229 | - | see also 21028 line |

Figure 46

| 47510 | DKFZp434I1916 | NM_032245.1 | 407 | aactgatccttcatgtttcaata | 399009 | - | see also 21030 line | | |
|---|---|---|---|---|---|---|---|---|---|
| 47511 | DKFZP434I2117 | NM_031478.3 | 276 | tcctctgcctacacgcaatttgc | 397175 | - | - | - | - |
| 47512 | DKFZP434I2117 | NM_031478.3 | 237 | acctcctgcaagcacatcattga | 397174 | - | see also 47511 line | | |
| 47513 | DKFZP434J0113 | NM_032130.1 | 2674 | ctcctccagttacgcaatagaaa | 397969 | - | see also 21031 line | | |
| 47514 | DKFZP434J0113 | NM_032130.1 | 2675 | tcctccagttacgcaatagaaaa | 397970 | - | see also 21031 line | | |
| 47515 | DKFZP434J0113 | NM_032130.1 | 175 | aactcgggctcaagaggatattt | 397955 | - | see also 21031 line | | |
| 47516 | DKFZP434J046 | NM_015671.2 | 1452 | agctcccgtgccaggatatcacg | 354963 | - | see also 21032 line | | |
| 47517 | DKFZP434J046 | NM_015671.2 | 885 | tgcgcaggtcccacagaagatga | 354960 | - | see also 21032 line | | |
| 47518 | DKFZP434J154 | NM_015610.1 | 1216 | ctgctcgctagccacaattcaga | 354612 | - | see also 6509 line | | |
| 47519 | DKFZP434J154 | NM_015610.1 | 848 | gacgcaagtggaactaaacttgc | 354603 | - | see also 6509 line | | |
| 47520 | DKFZP434K1172 | NM_031290.1 | 279 | gccattccggctgataaggaaga | 396520 | - | see also 21034 line | | |
| 47521 | DKFZP434K1172 | NM_031290.1 | 419 | ttcgcgaagagatgaaaattttt | 396526 | - | see also 21034 line | | |
| 47522 | DKFZP434K1172 | NM_031290.1 | 414 | ggcttttcgcgaagagatgaaaa | 396525 | - | see also 21034 line | | |
| 47523 | DKFZP434K1421 | NM_032141.1 | 1029 | accgtgattaccggaaagaaagg | 398343 | - | see also 21035 line | | |
| 47524 | DKFZP434K1421 | NM_032141.1 | 952 | aacgtcgagaggacatgagaaaa | 398338 | - | see also 21035 line | | |
| 47525 | DKFZP434K1421 | NM_032141.1 | 1028 | gaccgtgattaccggaaagaaag | 398342 | - | see also 21035 line | | |
| 47526 | DKFZp434K1815 | NM_152892.1 | 1410 | gcccaaccaggactacgaattcc | 421511 | - | see also 21036 line | | |
| 47527 | DKFZp434K1815 | NM_152892.1 | 1409 | tgcccaaccaggactacgaattc | 421510 | - | see also 21036 line | | |
| 47528 | DKFZp434K2435 | NM_032256.1 | 1161 | cactaaccctcggactaataaaa | 399057 | - | see also 21037 line | | |
| 47529 | DKFZp434K2435 | NM_032256.1 | 1159 | cacactaaccctcggactaataa | 399056 | - | see also 21037 line | | |

Figure 46

| | | | | | | | |
|---|---|---|---|---|---|---|---|
| 47530 | DKFZP434L0117 | NM_022778.2 | 1842 | tcctggcagaagcgatacgattc | 384158 | - | see also 8469 line |
| 47531 | DKFZP434L0117 | NM_022778.2 | 1857 | tacgattcgctccaaaagattgt | 384160 | - | see also 8469 line |
| 47532 | DKFZP434L0718 | NM_032139.1 | 1099 | aacggagatccctaattggatgg | 398287 | - | see also 21039 line |
| 47533 | DKFZP434L0718 | NM_032139.1 | 573 | gacactccgagcgatttgacagg | 398275 | - | see also 21039 line |
| 47534 | DKFZP434L0718 | NM_032139.1 | 642 | gcctccgtcaccacatagactca | 398279 | - | see also 21039 line |
| 47535 | DKFZp434L0850 | NM_017558.1 | 2168 | gtgctggcgctcttaattacagc | 359051 | - | see also 7032 line |
| 47536 | DKFZp434L0850 | NM_017558.1 | 2929 | acgtcggatccaacagttgttct | 359069 | - | see also 7032 line |
| 47537 | DKFZp434L142 | NM_016613.4 | 1411 | atcgcttagatacaaattgctgt | 358418 | - | see also 21041 line |
| 47538 | DKFZp434L142 | NM_016613.4 | 1807 | acggggtcaaagtattacctatg | 358433 | - | see also 21041 line |
| 47539 | DKFZP434L1717 | NM_032136.3 | 848 | aggtcggggtattccaaatattg | 398240 | - | see also 21042 line |
| 47540 | DKFZP434L1717 | NM_032136.3 | 847 | aaggtcggggtattccaaatatt | 398239 | - | see also 21042 line |
| 47541 | DKFZp434M202 | NM_182499.1 | 506 | taccacctgggggattatggaca | 433968 | - | see also 21043 line |
| 47542 | DKFZp434N035 | NM_032262.1 | 392 | aagcaatgaaagagcttatcttc | 399106 | - | see also 21044 line |
| 47543 | DKFZp434N035 | NM_032262.1 | 511 | ctctaggcataccacataatacc | 399108 | - | see also 21044 line |
| 47544 | DKFZp434N1415 | NM_032133.2 | 1616 | ttcgaacccatcctactctatta | 398111 | - | see also 21045 line |
| 47545 | DKFZp434N1415 | NM_032133.2 | 292 | cacattcctcgccttactgaaaa | 398092 | - | see also 21045 line |
| 47546 | DKFZP434O047 | NM_015594.1 | 2925 | gggagatgtggcaggataatagg | 354355 | - | see also 21046 line |
| 47547 | DKFZP434O047 | NM_015594.1 | 2924 | agggagatgtggcaggataatag | 354354 | - | see also 21046 line |
| 47548 | DKFZp434O0527 | NM_194302.1 | 1846 | acccccacggcagatttatgagc | 436653 | - | see also 21047 line |
| 47549 | DKFZp434O0527 | NM_194302.1 | 702 | tccagcccaacgagagacttacg | 436642 | - | see also 21047 line |

Figure 46

| | | | | | | | | |
|---|---|---|---|---|---|---|---|---|
| 47550 | DKFZP434P0316 | NM_032134.1 | 3160 | tccgtcgaccgagtcttatcaga | 398172 | - | see also 21048 line | |
| 47551 | DKFZP434P0316 | NM_032134.1 | 3087 | agcctcggaccgacatggaattc | 398168 | - | see also 21048 line | |
| 47552 | DKFZp434P055 | NM_173466.1 | 1330 | aaggcgaataatgctcagaatat | 424867 | - | see also 21049 line | |
| 47553 | DKFZp434P055 | NM_173466.1 | 1081 | ttgcatgctgaacgcatagaagc | 424862 | - | see also 21049 line | |
| 47554 | DKFZp451M2119 | NM_182585.1 | 3326 | gggaagttctgtaggcttataac | 434522 | - | see also 21050 line | |
| 47555 | DKFZp451M2119 | NM_182585.1 | 3264 | ctgtgtgcatggcccatttgtgc | 434521 | - | see also 21050 line | |
| 47556 | DKFZp547A023 | NM_018704.1 | 1756 | ggcacagactatcgaaatctagc | 372721 | - | see also 7571 line | |
| 47557 | DKFZp547A023 | NM_018704.1 | 1452 | ctcttcgccggtactcactaagc | 372716 | - | see also 7571 line | |
| 47558 | DKFZp547B1713 | NM_152379.1 | 625 | tgggcgctaagcctcctaaaaag | 416002 | - | see also 21052 line | |
| 47559 | DKFZp547B1713 | NM_152379.1 | 477 | aagcaggctaatcctagtgtttt | 415997 | - | see also 21052 line | |
| 47560 | DKFZp547B1713 | NM_152379.1 | 452 | gacgccagatcataacaagaaca | 415996 | - | see also 21052 line | |
| 47561 | DKFZp547C176 | NM_018712.2 | 367 | cccgacgctattgaaaaaactat | 372785 | - | see also 7574 line | |
| 47562 | DKFZp547C176 | NM_018712.2 | 512 | tgcgtagagaggcctatgattct | 372790 | - | see also 7574 line | |
| 47563 | DKFZp547D2210 | NM_153685.2 | 439 | ctctgccaatcccggatttctgg | 423472 | - | - | - | - |
| 47564 | DKFZp547D2210 | NM_153685.2 | 612 | agctctatgtcacaattaccatc | 423474 | - | see also 47563 line | |
| 47565 | DKFZp547D2210 | NM_153685.2 | 608 | ccccagctctatgtcacaattac | 423473 | - | see also 47563 line | |
| 47566 | DKFZp547E052 | NM_032276.2 | 802 | cccaggacggcaatactacttta | 399334 | - | see also 21054 line | |
| 47567 | DKFZp547E052 | NM_032276.2 | 643 | tgcttgttgggtcgaacttgtgg | 399329 | - | see also 21054 line | |
| 47568 | DKFZP547E1010 | NM_015607.2 | 365 | ctctaaatgagcgctttactaat | 354467 | - | see also 6506 line | |
| 47569 | DKFZp547G0215 | NM_173643.1 | 921 | aagttataccatactcaaaatga | 426935 | - | see also 21056 line | |

EP 1 752 536 A1

Figure 46

| | | | | | | | | | |
|---|---|---|---|---|---|---|---|---|---|
| 47570 | DKFZp547 G0215 | NM_173643.1 | 1048 | aacccagcaagtgtaatgatagt | 426936 | - | see also 21056 line | | |
| 47571 | DKFZp547 G0215 | NM_173643.1 | 911 | agccttgaagaagttataccata | 426934 | - | see also 21056 line | | |
| 47572 | DKFZP564 A022 | NM_030954.2 | 388 | tgccactcgacagcagttctaca | 395673 | - | see also 21057 line | | |
| 47573 | DKFZP564 A022 | NM_030954.2 | 654 | cagggcaacccagatctattatg | 395682 | - | see also 21057 line | | |
| 47574 | DKFZp564 A176 | NM_032242.1 | 488 | cactttagcagtatctgtttaca | 399007 | - | - | | - |
| 47575 | DKFZP564 B1162 | NM_031305.1 | 1206 | gtcctactcgggagttaacaaaa | 396634 | - | see also 21058 line | | |
| 47576 | DKFZP564 B1162 | NM_031305.1 | 1774 | ctgaaggtatctggtaccaaaat | 396645 | - | see also 21058 line | | |
| 47577 | DKFZP564 D116 | NM_015631.1 | 1672 | tccaacccgcaagctcatgtatc | 354735 | - | see also 6524 line | | |
| 47578 | DKFZP564 D116 | NM_015631.1 | 1695 | aggagttcgattcctataccagt | 354738 | - | see also 6524 line | | |
| 47579 | DKFZP564 D172 | NM_032042.3 | 923 | aacgagaagccgatgtaaaaaat | 397719 | - | see also 21060 line | | |
| 47580 | DKFZP564 D172 | NM_032042.3 | 643 | cacgtacagtcatcatctgatag | 397711 | - | see also 21060 line | | |
| 47581 | DKFZP564 G2022 | NM_015497.2 | 536 | aaccgcaatgcatgaaccattgc | 353592 | - | see also 6438 line | | |
| 47582 | DKFZP564 G2022 | NM_015497.2 | 167 | tccgataccgtcggggaaaaatt | 353577 | - | see also 6438 line | | |
| 47583 | DKFZp564I 1922 | NM_015419.1 | 6660 | gtgcgtagctcgaaataaggttg | 352501 | - | see also 21062 line | | |
| 47584 | DKFZp564I 1922 | NM_015419.1 | 2554 | gagcacgttttgggtaccatttc | 352409 | - | see also 21062 line | | |
| 47585 | DKFZP564J 047 | NM_032126.1 | 195 | gagccgaccaaaacatttgatta | 397901 | - | see also 21063 line | | |
| 47586 | DKFZP564J 047 | NM_032126.1 | 229 | aacaagaagggcggtttaccaaa | 397903 | - | see also 21063 line | | |
| 47587 | DKFZP564J 047 | NM_032126.1 | 591 | aagacgatggcaccacaaaaaac | 397910 | - | see also 21063 line | | |
| 47588 | DKFZP564 K0322 | NM_032040.1 | 2229 | gacgcttttcgtggttttgcaag | 397685 | - | see also 21064 line | | |
| 47589 | DKFZP564 K0322 | NM_032040.1 | 789 | tcggggaggacgtcgactatttg | 397678 | - | see also 21064 line | | |

Figure 46

| 47590 | DKFZP564K0822 | NM_030796.2 | 619 | cccgtacgaacaggtagtgaagg | 395099 | - | see also 21065 line |
|---|---|---|---|---|---|---|---|
| 47591 | DKFZP564K0822 | NM_030796.2 | 222 | gactctatccaacctattatata | 395097 | - | see also 21065 line |
| 47592 | DKFZP564K1964 | NM_015544.1 | 394 | aactggacgatgtcgttatcacc | 353844 | - | see also 21066 line |
| 47593 | DKFZP564K1964 | NM_015544.1 | 316 | tgctgcagcgctatgattctaag | 353843 | - | see also 21066 line |
| 47594 | DKFZP564K2062 | NM_015421.1 | 36 | ctccgagctgtgcgtaggtttcg | 352583 | - | see also 21067 line |
| 47595 | DKFZP564K2062 | NM_015421.1 | 280 | tgccaccaggctattacttgtac | 352589 | - | see also 21067 line |
| 47596 | DKFZP564M082 | NM_014042.1 | 494 | gacgagatgaatgactacaatga | 339374 | - | see also 21068 line |
| 47597 | DKFZP564M082 | NM_014042.1 | 394 | gccagcctcagagcactatgatg | 339373 | - | see also 21068 line |
| 47598 | DKFZP564M082 | NM_014042.1 | 262 | gactgagactctgtggtttaatc | 339371 | - | see also 21068 line |
| 47599 | DKFZP564O043 | NM_020319.1 | 397 | tgcgacatggagccgatgtaaat | 376505 | - | see also 7832 line |
| 47600 | DKFZP564O043 | NM_020319.1 | 375 | ctcgatatgtgcaaattactact | 376504 | - | see also 7832 line |
| 47601 | DKFZP564O043 | NM_020319.1 | 404 | tggagccgatgtaaattgtcatc | 376507 | - | see also 7832 line |
| 47602 | DKFZP564O0463 | NM_015420.4 | 925 | gtcaccgtgatggagtcaattgc | 352534 | - | see also 6405 line |
| 47603 | DKFZP564O0463 | NM_015420.4 | 1029 | cggaattgtatccgtacaataca | 352540 | - | see also 6405 line |
| 47604 | DKFZP564O0523 | NM_032120.1 | 87 | gacacacgggccaaatatcgaga | 397804 | - | see also 21071 line |
| 47605 | DKFZP564O0523 | NM_032120.1 | 999 | ttgaatacaacggcgaatttaat | 397832 | - | see also 21071 line |
| 47606 | DKFZP564O0823 | NM_015393.1 | 507 | agcggtggagtccacttaacaac | 352196 | - | see also 21072 line |
| 47607 | DKFZP564O0823 | NM_015393.1 | 186 | ctcttcgctctttgcatcttaac | 352189 | - | see also 21072 line |
| 47608 | DKFZP564O1664 | NM_030800.1 | 527 | tccccttcacgagagattataat | 395153 | - | see also 21073 line |
| 47609 | DKFZP564O1664 | NM_030800.1 | 1661 | aggccgctctagcctttggtttc | 395183 | - | see also 21073 line |

Figure 46

| | | | | | | | |
|---|---|---|---|---|---|---|---|
| 47610 | DKFZP564O1863 | NM_015633.1 | 522 | gccacggtccacgttagttatgg | 354746 | - | see also 21074 line |
| 47611 | DKFZP564O1863 | NM_015633.1 | 1038 | tgcgtcggaaagtacttctttgt | 354765 | - | see also 21074 line |
| 47612 | DKFZP566A1524 | NM_030797.1 | 399 | gagatccgagatgcaattcaaaa | 395102 | - | see also 21075 line |
| 47613 | DKFZP566A1524 | NM_030797.1 | 662 | cccggctattcagaatgacttca | 395108 | - | see also 21075 line |
| 47614 | DKFZP566B183 | NM_015509.2 | 316 | ttctagccgctactttgtaatcc | 353628 | - | see also 6445 line |
| 47615 | DKFZP566B183 | NM_015509.2 | 690 | caccacccattccgaaatctaac | 353640 | - | see also 6445 line |
| 47616 | DKFZP566B183 | NM_015509.2 | 691 | accacccattccgaaatctaacc | 353641 | - | see also 6445 line |
| 47617 | DKFZp566C0424 | NM_015609.1 | 365 | gacagcaggcagcccaataatgt | 354589 | - | see also 6508 line |
| 47618 | DKFZp566C0424 | NM_015609.1 | 103 | tcccaaagtgcccattgtgattc | 354588 | - | see also 6508 line |
| 47619 | DKFZP566D1346 | NM_030816.1 | 425 | ccctcggcggtacctttaccaga | 395299 | - | see also 21077 line |
| 47620 | DKFZP566D1346 | NM_030816.1 | 716 | ctctcatccgcacgcacaatatc | 395305 | - | see also 21077 line |
| 47621 | DKFZP566D193 | NM_015391.1 | 234 | gacagacttagccttacagtacc | 352188 | - | see also 21078 line |
| 47622 | DKFZP566D193 | NM_015391.1 | 196 | ggcaccacagaatctgtcaaaga | 352184 | - | see also 21078 line |
| 47623 | DKFZP566D193 | NM_015391.1 | 199 | accacagaatctgtcaaagaaca | 352185 | - | see also 21078 line |
| 47624 | DKFZP566F0546 | NM_015653.1 | 1258 | atcaacccacgggagactatttc | 354936 | - | see also 21079 line |
| 47625 | DKFZP566F0546 | NM_015653.1 | 687 | aacgcccgtgctgaacaaaaatg | 354932 | - | see also 21079 line |
| 47626 | DKFZP566F0546 | NM_015653.1 | 684 | aagaacgcccgtgctgaacaaaa | 354931 | - | see also 21079 line |
| 47627 | DKFZP566F084 | NM_015448.1 | 333 | ccctatcctaaggatgtctatag | 353071 | - | see also 21080 line |
| 47628 | DKFZP566F084 | NM_015448.1 | 327 | aacctcccctatcctaaggatgt | 353069 | - | see also 21080 line |
| 47629 | DKFZP566F2124 | NM_015630.2 | 672 | tgcaaactcgaaagaatcgtaag | 354649 | - | see also 6510 line |

Figure 46

| | | | | | | | |
|---|---|---|---|---|---|---|---|
| 47630 | DKFZP566F2124 | NM_015630.2 | 726 | aactgagacgagaatttagtaga | 354654 | - | see also 6510 line |
| 47631 | DKFZP566K1924 | NM_015463.1 | 476 | cacgctgcaggtcgagaatattt | 353312 | - | see also 21082 line |
| 47632 | DKFZP566K1924 | NM_015463.1 | 633 | accatgccgttcacagacattgg | 353315 | - | see also 21082 line |
| 47633 | DKFZP566M1046 | NM_032127.1 | 1449 | gacaccctcgttgctcgtattgg | 397934 | - | see also 9079 line |
| 47634 | DKFZP566M114 | NM_032128.1 | 236 | ggcgatcgctggagctaaactgg | 397942 | - | see also 21084 line |
| 47635 | DKFZP566M114 | NM_032128.1 | 333 | ttctttaacagtggactatcatt | 397945 | - | see also 21084 line |
| 47636 | DKFZP566N034 | NM_030923.2 | 909 | gacacgtcactacgagatgtttg | 395504 | - | see also 21085 line |
| 47637 | DKFZP566N034 | NM_030923.2 | 825 | cggcagcataggcgttctgatcg | 395501 | - | see also 21085 line |
| 47638 | DKFZP572C163 | NM_033160.2 | 478 | aactctgtggtggcagtgaatat | 403541 | - | - | - | - |
| 47639 | DKFZp586C1924 | NM_032273.2 | 257 | ttcgacgcatcttgaatgtgaca | 399312 | - | see also 21086 line |
| 47640 | DKFZp586C1924 | NM_032273.2 | 198 | tggatcggtttatgttggattaa | 399308 | - | see also 21086 line |
| 47641 | DKFZP586D0919 | NM_015433.1 | 665 | gccccatggcaccatctatctgg | 352771 | - | see also 21087 line |
| 47642 | DKFZP586D0919 | NM_015433.1 | 382 | gacatgctctatccatgtctacc | 352767 | - | see also 21087 line |
| 47643 | DKFZp586I1420 | NM_152747.1 | 2132 | gggactgtgtacctttattcatg | 420447 | - | see also 21088 line |
| 47644 | DKFZp586I1420 | NM_152747.1 | 1987 | tagactggataggcagatcaaga | 420444 | - | see also 21088 line |
| 47645 | DKFZP586L0724 | NM_015462.2 | 244 | ttgtacacgataataaggtttta | 353300 | - | see also 21089 line |
| 47646 | DKFZP586L0724 | NM_015462.2 | 629 | tgcatctgtagatcggaaattca | 353306 | - | see also 21089 line |
| 47647 | DKFZP586M1019 | NM_015603.1 | 1079 | accatcccaccgctatgatgacc | 354460 | - | see also 21090 line |
| 47648 | DKFZP586M1019 | NM_015603.1 | 212 | ggctcttcggcggaagaatgagg | 354458 | - | see also 21090 line |
| 47649 | DKFZP586M1120 | NM_031294.2 | 1387 | tggtaggactgtttatcgaaaat | 396579 | - | see also 21091 line |

Figure 46

| | | | | | | | |
|---|---|---|---|---|---|---|---|
| 47650 | DKFZP586M1120 | NM_031294.2 | 1551 | gtcgataaagatacgattgttaa | 396582 | - | see also 21091 line |
| 47651 | DKFZP586M1523 | NM_015476.1 | 744 | agcgttatactggcattttctca | 353425 | - | see also 21092 line |
| 47652 | DKFZP586M1523 | NM_015476.1 | 627 | ctctcgcagtgtgatatttgagc | 353423 | - | see also 21092 line |
| 47653 | DKFZP586N0721 | NM_015400.1 | 727 | atggcatgcacgtatgtaagtaa | 352294 | - | see also 21093 line |
| 47654 | DKFZP586N0721 | NM_015400.1 | 732 | atgcacgtatgtaagtaatctgg | 352297 | - | see also 21093 line |
| 47655 | DKFZP586O0120 | NM_014077.1 | 311 | cacactcacggagcattacgaca | 339521 | - | - |
| 47656 | DKFZP586O0120 | NM_014077.1 | 136 | aagcaatgggaacgagcaaaaag | 339518 | - | see also 47655 line |
| 47657 | DKFZP586O0120 | NM_014077.1 | 137 | agcaatgggaacgagcaaaaaga | 339519 | - | see also 47655 line |
| 47658 | DKFZp667B1218 | NM_177966.3 | 1155 | gaggttgaccgcgcagtgttttc | 430321 | - | see also 21094 line |
| 47659 | DKFZp667B1218 | NM_177966.3 | 1156 | aggttgaccgcgcagtgttttct | 430322 | - | see also 21094 line |
| 47660 | DKFZp686A17109 | NM_198465.1 | 3801 | atcttatacgctggattcgtaga | 438173 | - | see also 21095 line |
| 47661 | DKFZp686A17109 | NM_198465.1 | 3972 | ctgttgggaacccgatctaatct | 438177 | - | see also 21095 line |
| 47662 | DKFZp686B0797 | NM_198539.1 | 1306 | gtcatctgttacgctacatatga | 439306 | - | see also 21096 line |
| 47663 | DKFZp686B0797 | NM_198539.1 | 1311 | ctgttacgctacatatgagaagt | 439307 | - | see also 21096 line |
| 47664 | DKFZp686B2197 | NM_194319.1 | 1573 | ctcgacactgtagaatgtgtaat | 436841 | - | see also 21097 line |
| 47665 | DKFZp686B2197 | NM_194319.1 | 1479 | tccacactttgtcaacataatag | 436840 | - | see also 21097 line |
| 47666 | DKFZp686B2197 | NM_194319.1 | 966 | agccgtgtctcagctcttataga | 436836 | - | see also 21097 line |
| 47667 | DKFZp686G0786 | NM_198460.1 | 1829 | aacgtatgattgatactacaaaa | 437915 | - | see also 21098 line |
| 47668 | DKFZp686G0786 | NM_198460.1 | 625 | tggacagtacgggatttcactct | 437901 | - | see also 21098 line |
| 47669 | DKFZp686L1814 | NM_194282.1 | 2148 | atctgatcgacgtcatagcaaag | 436281 | - | see also 10306 line |

Figure 46

| | | | | | | | |
|---|---|---|---|---|---|---|---|
| 47670 | DKFZp686L1814 | NM_194282.1 | 1671 | gccacagcaacggcttatcatgc | 436269 | - | see also 10306 line |
| 47671 | DKFZp686L1814 | NM_194282.1 | 1182 | tgcaggtagggttctttcacagt | 436257 | - | see also 10306 line |
| 47672 | DKFZp686L20145 | NM_019045.2 | 728 | cccggactgttagccatagatca | 374673 | - | see also 21100 line |
| 47673 | DKFZp686L20145 | NM_019045.2 | 719 | agcaatatacccggactgttagc | 374672 | - | see also 21100 line |
| 47674 | DKFZp686N19164 | NM_194325.1 | 1469 | agcgaattcatgcagagataaag | 436868 | - | see also 21101 line |
| 47675 | DKFZp686N19164 | NM_194325.1 | 1866 | agcctcgtaccttgtacaacata | 436869 | - | see also 21101 line |
| 47676 | DKFZp686O1689 | NM_182608.2 | 824 | ggggctcacggcgttaatgaagg | 434634 | - | see also 21102 line |
| 47677 | DKFZp686O1689 | NM_182608.2 | 335 | ccgcgttgactgcctcatagata | 434629 | - | see also 21102 line |
| 47678 | DKFZP727C091 | NM_015443.1 | 1359 | gagccgtgcccgcagattacaaa | 352946 | - | see also 21103 line |
| 47679 | DKFZP727C091 | NM_015443.1 | 3099 | tcgcgtagagaaactgcaataca | 352984 | - | see also 21103 line |
| 47680 | DKFZp727G131 | NM_145111.1 | 426 | tggacatggtacggacaattttt | 413495 | - | see also 21104 line |
| 47681 | DKFZp727G131 | NM_145111.1 | 425 | atggacatggtacggacaatttt | 413494 | - | see also 21104 line |
| 47682 | DKFZP727M111 | NM_015540.2 | 1945 | agggcctacccctagtctataca | 353773 | - | see also 21105 line |
| 47683 | DKFZP727M111 | NM_015540.2 | 2063 | agccgcctgtgccgcatcatagc | 353777 | - | see also 21105 line |
| 47684 | DKFZp761A078 | NM_194292.1 | 716 | tagataagttacggaatgaatgg | 436420 | - | see also 21107 line |
| 47685 | DKFZp761A078 | NM_194292.1 | 1707 | cccaacctgtgggattggttatc | 436441 | - | see also 21107 line |
| 47686 | DKFZp761A132 | NM_032296.1 | 279 | tgctccgaggcgtggatagtttg | 399510 | - | - | | - |
| 47687 | DKFZp761B0514 | NM_032289.1 | 2323 | ccgttatgagacctatatccacc | 399402 | - | see also 9133 line |
| 47688 | DKFZp761B107 | NM_173463.2 | 472 | aaggctcggagacgaagcaatcg | 424834 | - | see also 21108 line |
| 47689 | DKFZp761B107 | NM_173463.2 | 770 | gaggtcaacctcttgaaatcaaa | 424839 | - | see also 21108 line |

Figure 46

| | | | | | | | |
|---|---|---|---|---|---|---|---|
| 47690 | DKFZp761 B1514 | NM_032288.3 | 507 | gagacgtcctaatggagttatca | 399364 | - | see also 21109 line |
| 47691 | DKFZp761 B1514 | NM_032288.3 | 299 | acctcgacaaaatagatatgtct | 399361 | - | see also 21109 line |
| 47692 | DKFZp761 C121 | NM_032290.1 | 985 | tacttagtaggatgttgctaaat | 399431 | - | see also 9136 line |
| 47693 | DKFZp761 C121 | NM_032290.1 | 1050 | tccaaagggttaactcatctaaa | 399432 | - | see also 9136 line |
| 47694 | DKFZp761 C169 | NM_022913.1 | 1975 | gacacgaatgcgcactgataaga | 384835 | - | see also 8531 line |
| 47695 | DKFZp761 C169 | NM_022913.1 | 1977 | cacgaatgcgcactgataagaag | 384836 | - | see also 8531 line |
| 47696 | DKFZp761 D221 | NM_032291.1 | 1171 | ggcctcctcgcaatgtactatcg | 399469 | - | see also 9139 line |
| 47697 | DKFZp761 D221 | NM_032291.1 | 1190 | atcgccgctcaatttagaagaag | 399471 | - | see also 9139 line |
| 47698 | DKFZP761 E1824 | NM_022486.1 | 1331 | ctgtaccctacgactgattatac | 383367 | - | see also 21113 line |
| 47699 | DKFZP761 E1824 | NM_022486.1 | 1329 | atctgtaccctacgactgattat | 383366 | - | see also 21113 line |
| 47700 | DKFZP761F 241 | NM_031455.2 | 528 | ctcttgcctcacggagtcaattt | 397082 | - | see also 21114 line |
| 47701 | DKFZP761F 241 | NM_031455.2 | 478 | tccaggactactcctatttcttc | 397081 | - | see also 21114 line |
| 47702 | DKFZp761 H0421 | NM_173079.1 | 165 | tggggcaaccgcgtttttagaag | 424569 | - | see also 21115 line |
| 47703 | DKFZp761 H0421 | NM_173079.1 | 166 | ggggcaaccgcgtttttagaaga | 424570 | - | see also 21115 line |
| 47704 | DKFZp761 H0421 | NM_173079.1 | 168 | ggcaaccgcgtttttagaagagg | 424571 | - | see also 21115 line |
| 47705 | DKFZp761 H079 | NM_144996.2 | 622 | agctgaacgagtgcgaaaattac | 412513 | - | see also 21116 line |
| 47706 | DKFZp761 H079 | NM_144996.2 | 621 | gagctgaacgagtgcgaaaatta | 412512 | - | see also 21116 line |
| 47707 | DKFZP761 H1710 | NM_031297.2 | 227 | acctgtgggcactcctacaatgt | 396589 | - | see also 21117 line |
| 47708 | DKFZp761 K1423 | NM_018422.1 | 1417 | ttcccatcggtcgccataaataa | 371344 | - | see also 21118 line |
| 47709 | DKFZp761 K1423 | NM_018422.1 | 1418 | tcccatcggtcgccataaataag | 371345 | - | see also 21118 line |

Figure 46

| 47710 | DKFZp761K1423 | NM_018422.1 | 1463 | tcccagcccaaggctcgattaag | 371349 | - | see also 21118 line | | |
|-------|---------------|-------------|------|-------------------------|--------|---|---------------------|---|---|
| 47711 | DKFZP761L0424 | NM_019590.1 | 1054 | gcctacagcacggcgacaatacc | 375348 | - | see also 7715 line | | |
| 47712 | DKFZP761L0424 | NM_019590.1 | 700 | atccggtcagcgagtgcttattg | 375333 | - | see also 7715 line | | |
| 47713 | DKFZp761L1417 | NM_152913.1 | 1189 | tcccatgtgctacacttatcact | 421685 | - | see also 21120 line | | |
| 47714 | DKFZp761L1417 | NM_152913.1 | 614 | cactaagaccgtcctgaaagtct | 421681 | - | see also 21120 line | | |
| 47715 | DKFZp761L1518 | NM_198075.1 | 1854 | cacccaaggccacgagattctgg | 436972 | - | see also 21121 line | | |
| 47716 | DKFZp761N1114 | NM_181644.2 | 1526 | ttcctacccaagacagatcaatt | 432649 | - | see also 21122 line | | |
| 47717 | DKFZp761N1114 | NM_181644.2 | 1527 | tcctacccaagacagatcaattg | 432650 | - | see also 21122 line | | |
| 47718 | DKFZp761N1114 | NM_181644.2 | 1201 | acgttcctggtgctgcttatttt | 432643 | - | see also 21122 line | | |
| 47719 | DKFZp761O0113 | NM_018409.2 | 999 | ttcctacgaggtcaactatattt | 371177 | - | see also 21123 line | | |
| 47720 | DKFZp761O0113 | NM_018409.2 | 1095 | cagctaggagtgatgtactatga | 371180 | - | see also 21123 line | | |
| 47721 | DKFZp761O17121 | NM_032287.1 | 650 | ttgtttggcatggtgatttgtgt | 399359 | - | see also 21124 line | | |
| 47722 | DKFZp761O17121 | NM_032287.1 | 258 | gaggtgtacagatcactttctct | 399357 | - | see also 21124 line | | |
| 47723 | DKFZp761P1121 | NM_152906.2 | 602 | gggagcctgatcctatcgttttg | 421678 | - | see also 21125 line | | |
| 47724 | DKFZp761P1121 | NM_152906.2 | 433 | gcccgagggcgaggtgaacttgt | 421675 | - | see also 21125 line | | |
| 47725 | DKFZp762A217 | NM_152588.1 | 514 | agccgtgctatcaagactaatca | 418347 | - | see also 9887 line | | |
| 47726 | DKFZp762A217 | NM_152588.1 | 1669 | ttctatgtcggctttgtaattgc | 418377 | - | see also 9887 line | | |
| 47727 | DKFZp762C2414 | NM_178542.2 | 1137 | cccgctacagacaacagaattgg | 431158 | - | see also 21126 line | | |
| 47728 | DKFZp762C2414 | NM_178542.2 | 1136 | ccccgctacagacaacagaattg | 431157 | - | see also 21126 line | | |
| 47729 | DKFZP762D096 | NM_031448.2 | 133 | agccggttcctcagatcctaatg | 397053 | - | - | | - |

Figure 46

| 47730 | DKFZP762 D096 | NM_031448.2 | 134 | gccggttcctcagatcctaatgg | 397054 | - | see also 47729 line | | | |
| 47731 | DKFZP762 D096 | NM_031448.2 | 323 | ggccgagatccagtatgatgact | 397055 | - | see also 47729 line | | | |
| 47732 | DKFZp762E 1312 | NM_018410.2 | 1710 | aggccacggaaggaatcgttacg | 371233 | - | see also 21127 line | | | |
| 47733 | DKFZp762E 1312 | NM_018410.2 | 1709 | caggccacggaaggaatcgttac | 371232 | - | see also 21127 line | | | |
| 47734 | DKFZp762 G199 | NM_197956.1 | 386 | gacccccatgcaacaacgtatct | 436914 | - | see also 21128 line | | | |
| 47735 | DKFZp762 G199 | NM_197956.1 | 877 | tccggcctatgatcaaagatttc | 436918 | - | see also 21128 line | | | |
| 47736 | DKFZp762 G199 | NM_197956.1 | 876 | ctccggcctatgatcaaagattt | 436917 | - | see also 21128 line | | | |
| 47737 | DKFZp762I 194 | NM_152384.1 | 349 | atgtaacagtactcgttttgaat | 416039 | - | see also 9843 line | | | |
| 47738 | DKFZp762I 194 | NM_152384.1 | 256 | ttctgtcggttacaattgcatat | 416031 | - | see also 9843 line | | | |
| 47739 | DKFZp762I 194 | NM_152384.1 | 98 | ctgtccgcgcagcaaatgaaaac | 416024 | - | see also 9843 line | | | |
| 47740 | DKFZp762 O076 | NM_018710.1 | 401 | gacacatctcggcgaataggatg | 372760 | - | see also 7572 line | | | |
| 47741 | DKFZp762 O076 | NM_018710.1 | 249 | gccgtgtgtgccaatcactaatc | 372751 | - | see also 7572 line | | | |
| 47742 | DKFZp762 O076 | NM_018710.1 | 431 | cccaactgtagacggataattaa | 372761 | - | see also 7572 line | | | |
| 47743 | DLEU7 | NM_198989.1 | 754 | ttcaactcctggctaatgagtga | 440307 | - | - | | - | - |
| 47744 | DLEU7 | NM_198989.1 | 317 | ggcctccatcagccaccaaatgg | 440306 | - | see also 47743 line | | | |
| 47745 | DLNB14 | NM_198489.1 | 427 | aggattatgcgcgattcaagaaa | 438536 | - | see also 21131 line | | | |
| 47746 | DLNB14 | NM_198489.1 | 434 | tgcgcgattcaagaaatccatgg | 438538 | - | see also 21131 line | | | |
| 47747 | DMRTC1 | NM_033053.1 | 571 | tccatctcgctgcctattggaca | 402739 | - | - | | - | - |
| 47748 | DMRTC1 | NM_033053.1 | 257 | ctcccatccgtgtcaggaatttg | 402738 | - | see also 47747 line | | | |
| 47749 | DNAJC9 | NM_015190.3 | 372 | ggcgtattggcggctactcttta | 349534 | - | see also 21132 line | | | |
| 47750 | DNAJC9 | NM_015190.3 | 585 | cggagaggtcccatcctataatg | 349541 | - | see also 21132 line | | | |
| 47751 | DNAJD1 | NM_013238.1 | 703 | aggcgagaagctggtcttatttt | 338366 | - | see also 21133 line | | | |
| 47752 | DNAJD1 | NM_013238.1 | 740 | ctgctggcaaggctaagattaga | 338367 | - | see also 21133 line | | | |
| 47753 | DNAPTP6 | NM_015535.1 | 865 | aactagatatcgcgtcatgatta | 353739 | - | see also 6460 line | | | |
| 47754 | DNAPTP6 | NM_015535.1 | 846 | aacgctgcaccgtttctctaact | 353736 | - | see also 6460 line | | | |
| 47755 | DOCK5 | NM_024940.3 | 1218 | ttgaacggaccacgtatacgact | 392337 | - | see also 21135 line | | | |

Figure 46

| | | | | | | | |
|---|---|---|---|---|---|---|---|
| 47756 | DOCK5 | NM_024940.3 | 1720 | aagccgcacgggtctattgtgc | 392351 | - | see also 21135 line |
| 47757 | DOLPPI | NM_020438.2 | 80 | accctcaccacgtcgaatatcc | 377464 | - | see also 7876 line |
| 47758 | DOLPPI | NM_020438.2 | 83 | ctcacccacgtcgaatatcctgc | 377465 | - | see also 7876 line |
| 47759 | DOM3Z | NM_005510.2 | 590 | cccgttccagggaacactatac | 334040 | - | see also 21137 line |
| 47760 | DOM3Z | NM_005510.2 | 668 | tcctccggagcttatgtacatg | 334041 | - | see also 21137 line |
| 47761 | DP1L1 | NM_138393.1 | 299 | cccgcatatgcctcaatcaaag | 407940 | - | see also 21138 line |
| 47762 | DP1L1 | NM_138393.1 | 298 | accccgcatatgcctccaatcaaa | 407939 | - | see also 21138 line |
| 47763 | DPCR1 | NM_080870.2 | 523 | tagaacgaagctgagttctatca | 406870 | - | see also 21139 line |
| 47764 | DPCR1 | NM_080870.2 | 568 | gagccatccatacctcaataaag | 406872 | - | see also 21139 line |
| 47765 | DPH2L2 | NM_001384.2 | 385 | gacgacaggtcaaagatgttca | 330192 | - | see also 21140 line |
| 47766 | DPH2L2 | NM_001384.2 | 1102 | tggcaagctagctatgttgttgg | 330199 | - | see also 21140 line |
| 47767 | DPPA4 | NM_018189.1 | 830 | cccgcaccttgaagacaatatg | 368236 | - | see also 21141 line |
| 47768 | DPPA4 | NM_018189.1 | 229 | aagataccaatcctccattacc | 368223 | - | see also 21141 line |
| 47769 | DREV1 | NM_016025.2 | 1029 | ctgtgtgaaggcgacatgtataa | 355955 | - | see also 6625 line |
| 47770 | DREV1 | NM_016025.2 | 1037 | agtcggacatgtataatgactact | 355957 | - | see also 6625 line |
| 47771 | DT1P1A10 | NM_058163.1 | 438 | atggaggtcacagctacgaatga | 406186 | - | see also 21143 line |
| 47772 | DT1P1A10 | NM_058163.1 | 505 | atccagaagctcagactattaag | 406188 | - | see also 21143 line |
| 47773 | DTNBP1 | NM_032122.3 | 486 | agcttaatcgcagacttagaat | 397880 | - | see also 21144 line |
| 47774 | DTNBP1 | NM_032122.3 | 301 | aagtactctgctggattagaatt | 397878 | - | see also 21144 line |
| 47775 | DUFD1 | NM_138419.2 | 977 | ctggcggtagaccattcataag | 408042 | - | see also 21145 line |
| 47776 | DUFD1 | NM_138419.2 | 628 | ttgagtgacgagcgcattagttt | 408026 | - | see also 21145 line |
| 47777 | DUSP15 | NM_080611.3 | 376 | tacctcggaaacttcattgatgc | 406407 | - | see also 21147 line |
| 47778 | DUSP15 | NM_080611.3 | 986 | cgcgcgtcaagcagacttctct | 406412 | - | see also 21147 line |
| 47779 | DUSP15 | NM_080611.3 | 413 | agctgggccgaaataagatcaca | 406410 | - | see also 21147 line |
| 47780 | DYX1C1 | NM_130810.1 | 373 | ctcttcaggttagcgattacagc | 407009 | - | see also 9554 line |
| 47781 | DYX1C1 | NM_130810.1 | 404 | gacgaagactgcggtcttctgt | 407010 | - | see also 9554 line |
| 47782 | DZIP1 | NM_014934.2 | 2258 | ggcattctctgtcgcacatactgg | 346071 | - | see also 21149 line |
| 47783 | DZIP1 | NM_014934.2 | 2988 | cccgcaggagtgtccgttaaaac | 346087 | - | see also 21149 line |
| 47784 | E2IG2 | NM_016565.1 | 328 | aggtgcaggcgttcaaggattgc | 358148 | - | see also 21150 line |
| 47785 | E2IG4 | NM_015516.1 | 796 | gtcgggcaaccccaagcttaact | 353654 | - | see also 21151 line |
| 47786 | E2IG4 | NM_015516.1 | 126 | gcctgactcggtggattgtagc | 353652 | - | see also 21151 line |
| 47787 | E2IG4 | NM_015516.1 | 408 | cctgagcgacgtgaaccttagc | 353653 | - | see also 21151 line |
| 47788 | E2IG5 | NM_014367.2 | 613 | gtggtatgaggatccatttcata | 340418 | - | see also 21152 line |
| 47789 | E2IG5 | NM_014367.2 | 374 | aagatcagctatctaatgattgc | 340416 | - | see also 21152 line |
| 47790 | EAF1 | NM_033083.5 | 514 | tccacactattcgttatgattt | 403199 | - | see also 21153 line |
| 47791 | EAF1 | NM_033083.5 | 1102 | accagcagccctacaacagtagg | 403208 | - | see also 21153 line |
| 47792 | EAF1 | NM_033083.5 | 661 | aacggccttaccagaaaagactgt | 403203 | - | see also 21153 line |

Figure 46

| | | | | | | | |
|---|---|---|---|---|---|---|---|
| 47793 | EAF2 | NM_018456.3 | 103 | cgcgagcgggttctcaagttagg | 371791 | - | see also 21154 line |
| 47794 | EAF2 | NM_018456.3 | 469 | gccaggactcccaatccttgtaaa | 371801 | - | see also 21154 line |
| 47795 | EB-1 | NM_152788.2 | 513 | cggacgcgttagagtgtaaaaa | 421301 | - | see also 21155 line |
| 47796 | EB-1 | NM_152788.2 | 3097 | accttgcgacctccgaatgaagc | 375652 | - | see also 21155 line |
| 47797 | EB-1 | NM_152788.2 | 3123 | agcctctacccggtacagtact | 375653 | - | see also 21155 line |
| 47798 | EB-1 | NM_020140.1 | 648 | cacttcgatggacctgttgaaaa | 375647 | - | see also 7764 line |
| 47799 | ECRG4 | NM_032411.1 | 531 | cagcgtcaactacgatgactact | 400206 | - | see also 21157 line |
| 47800 | ECRG4 | NM_032411.1 | 378 | gggctttgacgaagcgaaattg | 400197 | - | see also 21157 line |
| 47801 | EDRF1 | NM_015608.2 | 2081 | cagcactccaatccgttaaaat | 354533 | - | see also 21158 line |
| 47802 | EDRF1 | NM_015608.2 | 1553 | gccggtagccattcttcttgtaca | 354519 | - | see also 21158 line |
| 47803 | EDRF1 | NM_015608.2 | 613 | ttggcatggcatatgacttatt | 354495 | - | see also 21158 line |
| 47804 | EEG1 | NM_017611.2 | 580 | ggcacgcctcatagccatattt | 359517 | - | see also 21159 line |
| 47805 | EEG1 | NM_017611.2 | 1292 | ctgctcatggacggcttaaaca | 359522 | - | see also 21159 line |
| 47806 | EFA6R | NM_015310.1 | 585 | atggccacgtgaatattggaaag | 351410 | - | see also 6322 line |
| 47807 | EFA6R | NM_015310.1 | 797 | aagaccatcagtgtattggaag | 351415 | - | see also 6322 line |
| 47808 | EG1 | NM_025205.1 | 19 | ctccactagggggtatgtttct | 394412 | - | see also 21161 line |
| 47809 | EG1 | NM_025205.1 | 213 | accgatcaggaagaaattcgaac | 394417 | - | see also 21161 line |
| 47810 | EGFL11 | NM_198283.1 | 463 | aacatgtagacggcaattggtgg | 437589 | - | see also 21162 line |
| 47811 | EGFL11 | NM_198283.1 | 2040 | aggaatatcggtatctatgtttt | 437644 | - | see also 21162 line |
| 47812 | EIF2C3 | NM_024852.2 | 2481 | cacacccatagagttcgatttt | 391493 | - | see also 8784 line |
| 47813 | EIF2C3 | NM_024852.2 | 1798 | tggcctacagcttattatcgtca | 391461 | - | see also 8784 line |
| 47814 | EIF2C4 | NM_017629.2 | 2256 | ttcgaaagcatgattagcttg | 359769 | - | see also 7058 line |
| 47815 | ELAC1 | NM_018696.1 | 573 | aagcatttcgcctcttccacaga | 372654 | - | see also 7565 line |
| 47816 | ELAC1 | NM_018696.1 | 379 | cacggagctggtcttccattatg | 372650 | - | see also 7565 line |
| 47817 | ELAC2 | NM_018127.4 | 1534 | atcccgatgaagattcgaaatgt | 367000 | - | see also 21166 line |
| 47818 | ELAC2 | NM_018127.4 | 1536 | cccgatgaagattcgaaatgtca | 367001 | - | see also 21166 line |
| 47819 | ELAC2 | NM_018127.4 | 1195 | aaccttcgcagccacaagattca | 366994 | - | see also 21166 line |
| 47820 | ELKS | NM_015064.2 | 831 | cccttcgtcaggcgagagataac | 347534 | - | see also 6218 line |
| 47821 | ELKS | NM_015064.2 | 841 | ggcgagagataacacaatcatcg | 347536 | - | see also 6218 line |
| 47822 | ELKS | NM_015064.2 | 608 | acctctggccctatgttatctaag | 347530 | - | see also 6218 line |
| 47823 | Elis1 | NM_152793.1 | 200 | agctagcacaggattacatctcc | 421372 | - | see also 21168 line |
| 47824 | Elis1 | NM_152793.1 | 265 | ttcaagtcttcagaccttttact | 421373 | - | see also 21168 line |
| 47825 | ELP4 | NM_019040.2 | 1068 | ctcggcttaataacttgatctgt | 374617 | - | see also 7673 line |
| 47826 | ELSPBP1 | NM_022142.3 | 773 | gtgtgttgcaactttacaact | 382477 | - | see also 21170 line |
| 47827 | ELSPBP1 | NM_022142.3 | 698 | ttgtcactttcgttcaactata | 382473 | - | see also 21170 line |
| 47828 | EMCN | NM_016242.2 | 662 | gccggtcttattccagtattatt | 356595 | - | see also 21171 line |
| 47829 | EMCN | NM_016242.2 | 824 | agcttcttaccgttaagacaatt | 356603 | - | see also 21171 line |

Figure 46

| 47830 | EMILIN3 | NM_024756.1 | 1332 | gacgagactttcgatcagattag | 390098 | - | see also 21172 line |
|---|---|---|---|---|---|---|---|
| 47831 | EMILIN3 | NM_024756.1 | 2833 | agcgagtatggtttgagttaacc | 390106 | - | see also 21172 line |
| 47832 | EML5 | NM_183387.1 | 3948 | taccctaggcgtacagatatgt | 435972 | - | see also 10298 line |
| 47833 | EML5 | NM_183387.1 | 1223 | atcgttcggtcaggatatggagc | 435872 | - | see also 10298 line |
| 47834 | EMU1 | NM_133455.1 | 1135 | cgcgaggctttgaagattttagc | 407217 | - | see also 9579 line |
| 47835 | EMU1 | NM_133455.1 | 401 | gtctcaactgcagcaaagtgtca | 407214 | - | see also 9579 line |
| 47836 | ENAH | NM_018212.2 | 521 | accatcacgtcgtgataaaactgt | 368484 | - | see also 7322 line |
| 47837 | ENTH | NM_014666.2 | 1382 | cagccagccgtagaacttgttag | 341987 | - | see also 21176 line |
| 47838 | ENTH | NM_014666.2 | 441 | tgctcctagcttacctcataagg | 341968 | - | see also 21176 line |
| 47839 | EPM2AIP1 | NM_014805.2 | 1161 | ggcgttaggcgaactgaatttca | 343813 | - | see also 21177 line |
| 47840 | EPM2AIP1 | NM_014805.2 | 1978 | ttcggttgccacaactgaaatg | 343833 | - | see also 21177 line |
| 47841 | EPM2AIP1 | NM_014805.2 | 1160 | aggcgttaggcgacctgaatttc | 343812 | - | see also 21177 line |
| 47842 | EPN2 | NM_014964.2 | 1347 | agccgaagggaacacagttaaaat | 346408 | - | see also 21178 line |
| 47843 | EPN2 | NM_014964.2 | 1409 | tacgctgttggatttaatggatg | 346412 | - | see also 21178 line |
| 47844 | EPN3 | NM_017957.1 | 1801 | gccctgacctgggcatactagg | 364869 | - | see also 21179 line |
| 47845 | EPN3 | NM_017957.1 | 601 | cacgtgtacaaggctctaacatt | 364868 | - | see also 21179 line |
| 47846 | EPPB9 | NM_015681.2 | 525 | gtccagaatctacgtctaaact | 355001 | - | see also 21180 line |
| 47847 | EPPB9 | NM_015681.2 | 170 | gagtcctagcgtcttctactca | 354995 | - | see also 21180 line |
| 47848 | EPSTI1 | NM_033255.1 | 266 | caccttgatagcaccaaataaa | 403622 | - | see also 21181 line |
| 47849 | EPSTI1 | NM_033255.1 | 732 | tgggcttacagagattctctaaa | 403635 | - | see also 21181 line |
| 47850 | ERMAP | NM_018538.2 | 429 | aagatctgatgccggaatataag | 372165 | - | see also 21183 line |
| 47851 | ERMAP | NM_018538.2 | 810 | tccggagtgaactgaagttgaaa | 372168 | - | see also 21183 line |
| 47852 | ESAM | NM_138961.1 | 165 | gaccaacttgctgcggttttgt | 409268 | - | see also 21184 line |
| 47853 | ESAM | NM_138961.1 | 786 | ttccatggctggagtctatgtct | 409278 | - | see also 21184 line |
| 47854 | ESRRBL1 | NM_018010.2 | 318 | tgggcgaacagttcacatgttt | 365250 | - | see also 7196 line |
| 47855 | ESRRBL1 | NM_018010.2 | 100 | gtcacgacgtcgggtttggaaga | 365246 | - | see also 7196 line |
| 47856 | ESSPL | NM_183375.1 | 280 | tggctaggatcgattacagtagg | 435700 | - | see also 21187 line |
| 47857 | ESSPL | NM_183375.1 | 275 | ctgtgtggctaggatcgattaca | 435697 | - | see also 21187 line |
| 47858 | EST1B | NM_015327.1 | 684 | gggggatttgtcccgatatcaga | 351595 | - | see also 21188 line |
| 47859 | EST1B | NM_015327.1 | 332 | agctctgcgtcaagcttatgttc | 351589 | - | see also 21188 line |
| 47860 | ETAA16 | NM_019002.2 | 875 | ttcccgaaatagataatgctaca | 374296 | - | see also 7655 line |
| 47861 | ETAA16 | NM_019002.2 | 354 | aagtaacccgaggaaaaggtatg | 374281 | - | see also 7655 line |
| 47862 | ETAA16 | NM_019002.2 | 145 | atgagtcggcgaaggaaacatga | 374278 | - | see also 7655 line |
| 47863 | ETEA | NM_014613.1 | 192 | aagagggcgtacctagtgtttc | 341141 | - | see also 5781 line |
| 47864 | ETEA | NM_014613.1 | 96 | atctcactgcatcgaatctatg | 341138 | - | see also 5781 line |
| 47865 | ETHE1 | NM_014297.2 | 306 | tgccacgcggaccacattacagg | 340134 | - | - |
| 47866 | ETHE1 | NM_014297.2 | 596 | ctcggtccatgaaaagatcttca | 340137 | - | see also 47865 line |

Figure 46

| | | | | | | | |
|---|---|---|---|---|---|---|---|
| 47867 | ETHE1 | NM_014297.2 | 642 | taccctgctcacgattaccatgg | 340140 | - | see also 47865 line |
| 47868 | EVC2 | NM_147127.2 | 1564 | tcccttaacgaccaaatgataga | 414974 | - | see also 21192 line |
| 47869 | EVC2 | NM_147127.2 | 4085 | tgcgaggagatctgataagcaga | 415001 | - | see also 21192 line |
| 47870 | EVC2 | NM_147127.2 | 2514 | aagacgagagttgctacaaaagc | 414989 | - | see also 21192 line |
| 47871 | EVER2 | NM_152468.3 | 1544 | ccctgggtcagaccatactgtgc | 416989 | - | see also 21193 line |
| 47872 | EVER2 | NM_152468.3 | 862 | ttgaggttccacaatcaactttg | 416986 | - | see also 21193 line |
| 47873 | EVI2B | NM_006495.2 | 1303 | caccgcttgactcacttaacttg | 335685 | - | see also 21194 line |
| 47874 | EVI2B | NM_006495.2 | 1304 | accgcttgactcacttaacttgc | 335686 | - | see also 21194 line |
| 47875 | EVI2B | NM_006495.2 | 293 | aacacaattcagcgacactttt | 335661 | - | see also 21194 line |
| 47876 | F25965 | NM_019104.1 | 378 | gccgcagcgatccaacacatatg | 375231 | - | see also 21195 line |
| 47877 | F25965 | NM_019104.1 | 377 | ggccgcagcgatccaacacatat | 375230 | - | see also 21195 line |
| 47878 | FAIM | NM_018147.1 | 174 | ggcaaacgagtagtatatgtaga | 367679 | - | see also 21197 line |
| 47879 | FAIM | NM_018147.1 | 133 | acggagtccacaagatcgaattt | 367675 | - | see also 21197 line |
| 47880 | FAM11A | NM_032508.1 | 1243 | cagttccggagccccctaaaatc | 400470 | - | see also 21198 line |
| 47881 | FAM11A | NM_032508.1 | 894 | ttgttcttgcgctctatggatgt | 400459 | - | see also 21198 line |
| 47882 | FAM13A1 | NM_014883.1 | 287 | ttctatttgagtgctcatgtacc | 345250 | - | see also 6077 line |
| 47883 | FAM13A1 | NM_014883.1 | 1244 | gcccagctcacacgaaggattca | 345274 | - | see also 6077 line |
| 47884 | FAM13C1 | NM_198215.1 | 658 | tgcttgccgatgggacagattct | 437259 | - | see also 10314 line |
| 47885 | FAM13C1 | NM_198215.1 | 981 | aagcggaaaattcggaaatttga | 437262 | - | see also 10314 line |
| 47886 | FAM18B | NM_016078.1 | 755 | gtggttggcggtggttatcatgg | 356216 | - | see also 21201 line |
| 47887 | FAM20A | NM_017565.1 | 321 | ggcacgatcgtgcacaactttc | 359091 | - | see also 21202 line |
| 47888 | FAM20A | NM_017565.1 | 320 | cggcacgatcgtgcacaacttt | 359090 | - | see also 21202 line |
| 47889 | FAM20B | NM_014864.1 | 350 | agcgagtcgtgctgttagcaatt | 344939 | - | see also 21203 line |
| 47890 | FAM20B | NM_014864.1 | 450 | gagggcctttcaccgaatgatga | 344940 | - | see also 21203 line |
| 47891 | FAM20C | NM_020223.1 | 1178 | acggcgagtgttcctactactgc | 376251 | - | see also 21204 line |
| 47892 | FAM20C | NM_020223.1 | 922 | tacgggcaagcgctgttcaaacc | 376250 | - | see also 21204 line |
| 47893 | FAM8A1 | NM_016255.1 | 238 | cgccgcagccgacaaattggagc | 356630 | - | see also 21205 line |
| 47894 | FAM8A1 | NM_016255.1 | 1102 | tgcaccaagtcgggttttagtga | 356648 | - | see also 21205 line |
| 47895 | FAM8A1 | NM_016255.1 | 1105 | accaagtcgggttttagtgattc | 356650 | - | see also 21205 line |
| 47896 | FANCL | NM_018062.1 | 707 | cacgcagaattgcattaggtaat | 366003 | - | see also 21206 line |
| 47897 | FANCL | NM_018062.1 | 90 | ccggtcgaaaaccgtgtatgagg | 365978 | - | see also 21206 line |
| 47898 | FANCL | NM_018062.1 | 80 | tgccccagaaccggtcgaaaacc | 365977 | - | see also 21206 line |
| 47899 | FANK1 | NM_145235.2 | 857 | ctctgcggtgtcgggaaatcaga | 413793 | - | see also 21207 line |
| 47900 | FANK1 | NM_145235.2 | 1076 | gagtgtagtctccttattagaag | 413804 | - | see also 21207 line |
| 47901 | FAPP2 | NM_032639.2 | 153 | ttctctgtgggggaatattgtcc | 401160 | - | see also 21208 line |
| 47902 | FATE | NM_033085.1 | 272 | cagccaaacgagtttggaatatg | 403211 | - | see also 21209 line |
| 47903 | FATE | NM_033085.1 | 397 | ttccaaggcatacgtttccatta | 403214 | - | see also 21209 line |

Figure 46

| | | | | | | | | molecular_function unknown |
|---|---|---|---|---|---|---|---|---|
| 47904 | FAU | NM_001997.2 | 310 | ggccgcatgcttggaggtaagt | 330570 | - | - | - |
| 47905 | FAU | NM_001997.2 | 340 | tccctggcccgtgctggaaaagt | 330571 | - | see also 47904 line | |
| 47906 | FBS1 | NM_022452.1 | 1081 | aaggccaccccgcttctatgagg | 382835 | - | see also 21210 line | |
| 47907 | FBS1 | NM_022452.1 | 843 | ggcctcggccaaccaaggaatct | 382834 | - | see also 21210 line | |
| 47908 | FBX30 | NM_033182.4 | 730 | gaggagtctcccacacattct | 403564 | - | see also 21211 line | |
| 47909 | FBXL12 | NM_017703.1 | 393 | ctctacacgatgcgacctaaagt | 361080 | - | see also 21212 line | |
| 47910 | FBXL12 | NM_017703.1 | 269 | ctggtcctgctcgagatcttct | 361078 | - | see also 21212 line | |
| 47911 | FBXL12 | NM_017703.1 | 398 | cacgatgcgacctaaagtcatgt | 361081 | - | see also 21212 line | |
| 47912 | FBXL8 | NM_018378.2 | 1109 | tgcgcgaggtgcattgtttctgc | 370928 | - | see also 21213 line | |
| 47913 | FBXL8 | NM_018378.2 | 1106 | ccctgcgcgaggtgcattgtttc | 370927 | - | see also 21213 line | |
| 47914 | FBXL8 | NM_018378.2 | 334 | cacaaacctacggctggaatttga | 370925 | - | see also 21213 line | |
| 47915 | FBXL9 | NM_012163.1 | 827 | cacgtgccctgcatcaacatgg | 337713 | - | - | - |
| 47916 | FBXO15 | NM_152676.1 | 1327 | ttccatgatggacgttaactcttt | 419619 | - | see also 21214 line | |
| 47917 | FBXO15 | NM_152676.1 | 1033 | cacattaggctcggctactatcc | 419603 | - | see also 21214 line | |
| 47918 | FBXO15 | NM_152676.1 | 804 | cagtttttaccgactggtataaa | 419599 | - | see also 21214 line | |
| 47919 | FBXO16 | NM_173266.2 | 630 | atcgctgacgttcaactagttac | 424463 | - | see also 21215 line | |
| 47920 | FBXO16 | NM_173266.2 | 631 | tcgctgacgttcaactagttaca | 424464 | - | see also 21215 line | |
| 47921 | FBXO22 | NM_012170.2 | 694 | aagaatttaacattagaaagaca | 337730 | - | see also 5175 line | |
| 47922 | FBXO22 | NM_012170.2 | 451 | gagtgtcgtggccataagagagc | 337722 | - | see also 5175 line | |
| 47923 | FBXO25 | NM_012173.3 | 704 | accacaatcctgcttaatcaaa | 337739 | - | see also 21217 line | |
| 47924 | FBXO25 | NM_012173.3 | 706 | cacaatcctgcttaatcaaaga | 337740 | - | see also 21217 line | |
| 47925 | FBXO26 | NM_024907.5 | 896 | ggcatccgctacgtatctttga | 391894 | - | - | - |
| 47926 | FBXO26 | NM_024907.5 | 895 | gggcatccgctacgtatctttg | 391893 | - | see also 47925 line | |
| 47927 | FBXO26 | NM_024907.5 | 614 | tgcttcgtgacctcttcgaatg | 391891 | - | see also 47925 line | |
| 47928 | FBXO27 | NM_178820.3 | 535 | gacgtgcttggtgacttcattca | 431377 | - | see also 21218 line | |
| 47929 | FBXO27 | NM_178820.3 | 666 | gacacgacagcggctgtagttac | 431379 | - | see also 21218 line | |
| 47930 | FBXO27 | NM_178820.3 | 710 | gacgccaaccagacgttctaga | 431381 | - | see also 21218 line | |
| 47931 | FBXO32 | NM_058229.2 | 1019 | agcggcagatccgcaaacgatta | 406257 | - | see also 21219 line | |
| 47932 | FBXO32 | NM_058229.2 | 1024 | cagatccgcaaacgattaattct | 406261 | - | see also 21219 line | |
| 47933 | FBXO34 | NM_017943.2 | 848 | ctgctagctagcgttcaaaaaa | 364490 | - | see also 21220 line | |
| 47934 | FBXO34 | NM_017943.2 | 1650 | gtcagcacttgtcagactattcc | 364508 | - | see also 21220 line | |
| 47935 | FBXO34 | NM_017943.2 | 2264 | agctttaatcggcaatccataa | 364514 | - | see also 21220 line | |
| 47936 | FBXO6 | NM_018438.2 | 669 | ctgcacctaccaactcaaagtgc | 371549 | - | see also 7478 line | |
| 47937 | FBXO6 | NM_018438.2 | 992 | accgagctgttgtccagattttc | 371551 | - | see also 7478 line | |
| 47938 | FBXW5 | NM_018998.2 | 1462 | accaatcgcgaggagattgacc | 374102 | - | see also 7651 line | |
| 47939 | FBXW5 | NM_018998.2 | 768 | tgcaccgcatcggagatatcacc | 374098 | - | see also 7651 line | |

Figure 46

| 47940 | FBXW7 | NM_018315.2 | 1461 | cacgttgcaggggcatactaata | 370196 | - | see also 7394 line |
|---|---|---|---|---|---|---|---|
| 47941 | FBXW7 | NM_018315.2 | 515 | aacaacgacgccgaattacatct | 370168 | - | see also 7394 line |
| 47942 | FBXW8 | NM_012174.1 | 1063 | ctcggccaaccaagttgcttttg | 337766 | - | see also 21224 line |
| 47943 | FBXW8 | NM_012174.1 | 241 | gccttacgaattggcaatcaata | 337753 | - | see also 21224 line |
| 47944 | FBXW8 | NM_012174.1 | 551 | gtcattgcgggatatacatcagg | 337758 | - | see also 21224 line |
| 47945 | FCGBP | NM_003890.1 | 15547 | gccgatacttgccggtaaactcc | 332266 | - | see also 21225 line |
| 47946 | FCGBP | NM_003890.1 | 418 | aggccctaggcaccgagtatttt | 332241 | - | see also 21225 line |
| 47947 | FCGBP | NM_003890.1 | 419 | ggccctaggcaccgagtattttg | 332242 | - | see also 21225 line |
| 47948 | FEM1A | NM_018708.1 | 1297 | cgccgactcgggcaatttcgagc | 372742 | - | see also 21226 line |
| 47949 | FEM1A | NM_018708.1 | 938 | ctgaacggggaatcttacgaaag | 372731 | - | see also 21226 line |
| 47950 | FEM1C | NM_020177.2 | 772 | tgggggctccgtcaattttgatg | 375757 | - | see also 7781 line |
| 47951 | FEM1C | NM_020177.2 | 1005 | atgggcatacgtgcttgatgatt | 375766 | - | see also 7781 line |
| 47952 | FEZL | NM_018008.2 | 618 | ggccagcggctcgctctactact | 365225 | - | see also 7195 line |
| 47953 | FEZL | NM_018008.2 | 367 | tacgaggtgccgtcaaagacact | 365223 | - | see also 7195 line |
| 47954 | FEZL | NM_018008.2 | 1202 | agcccttcgtctgcgaattttgc | 365239 | - | see also 7195 line |
| 47955 | FGIF | NM_017704.1 | 305 | gtggtatcgattgcaagaaaaaa | 361089 | - | see also 21228 line |
| 47956 | FGIF | NM_017704.1 | 680 | cagcaaggataccctagaactcc | 361095 | - | see also 21228 line |
| 47957 | FILIP1 | NM_015687.1 | 3276 | tactgtccgaccagtaaacgtga | 355081 | - | see also 21229 line |
| 47958 | FILIP1 | NM_015687.1 | 3475 | cccacccgcattcctatgtcaaa | 355082 | - | see also 21229 line |
| 47959 | FIS | NM_175616.2 | 536 | tgctgctccatcgcatgaattcc | 429154 | - | see also 21230 line |
| 47960 | FIS | NM_175616.2 | 604 | cccagggaggtcggttgatgagc | 429156 | - | see also 21230 line |
| 47961 | FKRP | NM_024301.2 | 1659 | ggcgcaggcgcctaacaactacc | 386215 | - | see also 21231 line |
| 47962 | FKRP | NM_024301.2 | 1473 | ggcggtcgagggcgactttttcc | 386213 | - | see also 21231 line |
| 47963 | FKSG14 | NM_022145.2 | 275 | aggatctagatccggatagtact | 382502 | - | see also 21232 line |
| 47964 | FKSG14 | NM_022145.2 | 278 | atctagatccggatagtactaca | 382503 | - | see also 21232 line |
| 47965 | FKSG32 | NM_031307.2 | 378 | aaccaagactcgactagtagaaa | 396663 | - | see also 9012 line |
| 47966 | FKSG32 | NM_031307.2 | 1325 | ccctcatggaccgtcctaaatgc | 396699 | - | see also 9012 line |
| 47967 | FKSG42 | NM_032032.1 | 1905 | aggaaggcaaggcaacctttttg | 397667 | - | see also 21234 line |
| 47968 | FKSG42 | NM_032032.1 | 373 | gagatgttcctgtgtgacatatc | 397664 | - | see also 21234 line |
| 47969 | FLJ00005 | NM_020447.2 | 141 | tccgggcagatcggtaatagagc | 377479 | - | see also 21235 line |
| 47970 | FLJ00005 | NM_020447.2 | 273 | aaggccgttctgcggagaaaagg | 377480 | - | see also 21235 line |
| 47971 | FLJ00007 | NM_033449.1 | 708 | tgcccacctcgaccattactacc | 404031 | - | see also 9425 line |
| 47972 | FLJ00332 | NM_198517.1 | 191 | tccgccaacgggagatgaagtgg | 438949 | - | - |
| 47973 | FLJ00332 | NM_198517.1 | 116 | gccaggccgaccgctatggattc | 438947 | - | see also 47972 line |
| 47974 | FLJ00332 | NM_198517.1 | 121 | gccgaccgctatggattcattgg | 438948 | - | see also 47972 line |
| 47975 | FLJ10006 | NM_017969.1 | 1897 | accgcgatggtggcacctttatt | 364923 | - | see also 21237 line |
| 47976 | FLJ10006 | NM_017969.1 | 2233 | gacgagcagtgatgtatctctat | 364928 | - | see also 21237 line |

Figure 46

| | | | | | | | |
|---|---|---|---|---|---|---|---|
| 47977 | FLJ10036 | NM_017975.2 | 1248 | ttccgtcaagcgtctttcaaag | 364987 | - | see also 7188 line |
| 47978 | FLJ10036 | NM_017975.2 | 912 | aactgcgatcgctttggatatt | 364980 | - | see also 7188 line |
| 47979 | FLJ10036 | NM_017975.2 | 1245 | tcgttccgtcaagcgtctttca | 364986 | - | see also 7188 line |
| 47980 | FLJ10055 | NM_017983.3 | 143 | aactaaagccggtataagctgt | 365035 | - | see also 21239 line |
| 47981 | FLJ10055 | NM_017983.3 | 1227 | tccacgtgccaggttattctga | 365057 | - | see also 21239 line |
| 47982 | FLJ10081 | NM_017991.3 | 462 | cacgcagcgtgatgaatgagtgt | 365109 | - | see also 21240 line |
| 47983 | FLJ10081 | NM_017991.3 | 1341 | tagatgatcccctcttggatatg | 365125 | - | see also 21240 line |
| 47984 | FLJ10099 | NM_017994.3 | 479 | cacaactccgaaagtacaatga | 365142 | - | see also 21241 line |
| 47985 | FLJ10099 | NM_017994.3 | 477 | accacaactccggaaagtacaat | 365141 | - | see also 21241 line |
| 47986 | FLJ10099 | NM_017994.3 | 406 | ttctgctacggttcaatgatttg | 365139 | - | see also 21241 line |
| 47987 | FLJ10103 | NM_017996.2 | 391 | caactgatggacgctactttatt | 365161 | - | see also 21242 line |
| 47988 | FLJ10103 | NM_017996.2 | 1461 | cgccggttcaaagacactattat | 365187 | - | see also 21242 line |
| 47989 | FLJ10103 | NM_017996.2 | 477 | gaactactgcagggatacgaagg | 365164 | - | see also 21242 line |
| 47990 | FLJ10116 | NM_018000.1 | 688 | aagctgagagatgttgttaaaa | 365214 | - | see also 21243 line |
| 47991 | FLJ10116 | NM_018000.1 | 349 | agcccctgcagtgataacaat | 365203 | - | see also 21243 line |
| 47992 | FLJ10154 | NM_018011.1 | 573 | ttcgagccgacgcgaaaaattcg | 365282 | - | see also 7198 line |
| 47993 | FLJ10154 | NM_018011.1 | 576 | gagcggcagcgaaaaattcgaca | 365283 | - | see also 7198 line |
| 47994 | FLJ10154 | NM_018011.1 | 792 | gagcttgccgcacaaaaagctag | 365285 | - | see also 7198 line |
| 47995 | FLJ10156 | NM_019013.1 | 52 | ggcttctcggtggcagaacatgg | 374468 | - | see also 21245 line |
| 47996 | FLJ10159 | NM_018013.1 | 586 | aagtcagaccgaaccttaataa | 365287 | - | see also 21246 line |
| 47997 | FLJ10159 | NM_018013.1 | 777 | gtgcctccgaattagaaatcaga | 365292 | - | see also 21246 line |
| 47998 | FLJ10178 | NM_018015.2 | 1109 | aaccgtcgagcaaatataaatgc | 365372 | - | see also 7211 line |
| 47999 | FLJ10178 | NM_018015.2 | 1106 | ttcaaccgtcgagcaaatataaa | 365371 | - | see also 7211 line |
| 48000 | FLJ10188 | NM_018017.2 | 2807 | agcatgggaagtcgttctagttc | 365437 | - | see also 21248 line |
| 48001 | FLJ10188 | NM_018017.2 | 1182 | aacatcgagtaccaaatggaatg | 365400 | - | see also 21248 line |
| 48002 | FLJ10193 | NM_018019.2 | 82 | ggcgacagcgcggaggatgtattg | 365452 | - | see also 21249 line |
| 48003 | FLJ10199 | NM_018022.1 | 787 | ggctgcctcaaggtactatgttc | 365456 | - | see also 21250 line |
| 48004 | FLJ10199 | NM_018022.1 | 1120 | tccaccgcagtatgatgaagtcc | 365465 | - | see also 21250 line |
| 48005 | FLJ10199 | NM_018022.1 | 1117 | tcctccaccgcagtatgatgaag | 365464 | - | see also 21250 line |
| 48006 | FLJ10204 | NM_018024.1 | 698 | cacactatccgaatttacacatc | 365478 | - | see also 21251 line |
| 48007 | FLJ10204 | NM_018024.1 | 696 | tacacactatccgaatttacaca | 365477 | - | see also 21251 line |
| 48008 | FLJ10213 | NM_018029.3 | 909 | tccttatgctagcagattcaaag | 365493 | - | see also 21252 line |
| 48009 | FLJ10213 | NM_018029.3 | 908 | ctccttatgctagcagattcaaa | 365492 | - | see also 21252 line |
| 48010 | FLJ10213 | NM_018029.3 | 1217 | tacacaagctacgcaagtcaagt | 365498 | - | see also 21252 line |
| 48011 | FLJ10233 | NM_018034.2 | 1061 | cccactacgtgcacatatagtag | 365542 | - | see also 21254 line |
| 48012 | FLJ10233 | NM_018034.2 | 1841 | gacaagacgatgacagtaatcc | 365579 | - | see also 21254 line |
| 48013 | FLJ10241 | NM_018035.1 | 286 | caggagaggcgcagtcaagtttcg | 365586 | - | see also 21255 line |

Figure 46

| 48014 | FLJ10241 | NM_018035.1 | 202 | aaggtgcatgagaaaaatcgatc | 365582 | - | see also 21255 line |
|---|---|---|---|---|---|---|---|
| 48015 | FLJ10246 | NM_018038.1 | 107 | ttcactttagcgggcagttaacc | 365674 | - | see also 21256 line |
| 48016 | FLJ10246 | NM_018038.1 | 294 | tggtaaacttgagattgtataca | 365679 | - | see also 21256 line |
| 48017 | FLJ10251 | NM_018039.2 | 1208 | tggaagaaccgcatctataattc | 365692 | - | see also 21257 line |
| 48018 | FLJ10251 | NM_018039.2 | 1207 | ctggaagaaccgcatctataatt | 365691 | - | see also 21257 line |
| 48019 | FLJ10260 | NM_018042.2 | 2040 | taccagataatcggtatagattg | 365727 | - | see also 21258 line |
| 48020 | FLJ10260 | NM_018042.2 | 875 | gccccgtgttgatatacaagaag | 365711 | - | see also 21258 line |
| 48021 | FLJ10287 | NM_019083.1 | 25 | tcgccgcacgcgcctggttttcc | 375030 | - | see also 21259 line |
| 48022 | FLJ10287 | NM_019083.1 | 502 | cagcaggcttctattttaggtaa | 375047 | - | see also 21259 line |
| 48023 | FLJ10298 | NM_018050.2 | 1493 | tccgcaggaaacgttactcaaga | 365876 | - | see also 21260 line |
| 48024 | FLJ10298 | NM_018050.2 | 409 | ggcaatgagcccgtatatacttc | 365850 | - | see also 21260 line |
| 48025 | FLJ10300 | NM_018051.2 | 2402 | cacgttttccaccgatggaatcc | 365890 | - | see also 21261 line |
| 48026 | FLJ10300 | NM_018051.2 | 2661 | agggtcaagctggtacatagtgc | 365897 | - | see also 21261 line |
| 48027 | FLJ10300 | NM_018051.2 | 2619 | atcgcaggttcaataagtgattt | 365895 | - | see also 21261 line |
| 48028 | FLJ10305 | NM_018052.3 | 1601 | tgcagacgttatcggatgaatcg | 365927 | - | see also 7217 line |
| 48029 | FLJ10307 | NM_018053.2 | 1188 | aacaggcgcatgacccatttagc | 365933 | - | see also 21263 line |
| 48030 | FLJ10315 | NM_018056.1 | 1517 | tgctccgggaccgcttggtattg | 365942 | - | see also 21264 line |
| 48031 | FLJ10315 | NM_018056.1 | 1229 | agcacccgtggactgaagaatgc | 365937 | - | see also 21264 line |
| 48032 | FLJ10330 | NM_018061.1 | 1019 | cggcgaagatcccgaagtattga | 365963 | - | see also 7226 line |
| 48033 | FLJ10330 | NM_018061.1 | 1018 | acggcgaagatcccgaagtattg | 365962 | - | see also 7226 line |
| 48034 | FLJ10350 | NM_018067.2 | 902 | aggcctcgccggtgcagaaaaag | 366044 | - | see also 21265 line |
| 48035 | FLJ10350 | NM_018067.2 | 1749 | gcgtctggagcgggaaaagcact | 366046 | - | see also 21265 line |
| 48036 | FLJ10350 | NM_018067.2 | 1808 | agcgtctggaggagatcatgaag | 366047 | - | see also 21265 line |
| 48037 | FLJ10359 | NM_018072.3 | 427 | tgcgcctagatgatacttacagt | 366125 | - | see also 21266 line |
| 48038 | FLJ10359 | NM_018072.3 | 2648 | ctcctcgcctaaggttcgatttg | 366178 | - | see also 21266 line |
| 48039 | FLJ10359 | NM_018072.3 | 1080 | accgttctcggctatatcagtgc | 366150 | - | see also 21266 line |
| 48040 | FLJ10374 | NM_018074.3 | 210 | gacgtgcggagaatacatctaca | 366188 | - | see also 21267 line |
| 48041 | FLJ10374 | NM_018074.3 | 79 | atgtcggagcgaaaagtattaaa | 366182 | - | see also 21267 line |
| 48042 | FLJ10375 | NM_018075.2 | 1347 | gccacggcatatccagatgaaac | 366217 | - | see also 7232 line |
| 48043 | FLJ10375 | NM_018075.2 | 646 | aagtatcagcccatagagaatca | 366200 | - | see also 7232 line |
| 48044 | FLJ10378 | NM_018078.2 | 625 | atcctcgattgaactttgattat | 366318 | - | see also 7235 line |
| 48045 | FLJ10385 | NM_018081.1 | 252 | cccacgcttccccgatgaataaa | 366367 | - | see also 21269 line |
| 48046 | FLJ10385 | NM_018081.1 | 708 | ctgacggttcctgcatcttgacc | 366372 | - | see also 21269 line |
| 48047 | FLJ10404 | NM_019057.2 | 4029 | ctcctccttggacgatgtgttcc | 374799 | - | see also 21270 line |
| 48048 | FLJ10404 | NM_019057.2 | 3847 | tgggctggcagtcccaaaactga | 374798 | - | see also 21270 line |
| 48049 | FLJ10404 | NM_019057.2 | 3166 | ctgcaggagatcaaaaacactgt | 374797 | - | see also 21270 line |
| 48050 | FLJ10407 | NM_018087.3 | 2045 | gtcgacaagtactttaagcttcc | 366553 | - | see also 7249 line |

Figure 46

| | | | | | | | |
|---|---|---|---|---|---|---|---|
| 48051 | FLJI10407 | NM_018087.3 | 759 | gagcatttatgggctatagctat | 366514 | - | see also 7249 line |
| 48052 | FLJI10415 | NM_018089.1 | 171 | cgcggatgctccggtcttcagg | 366558 | - | see also 21272 line |
| 48053 | FLJI10415 | NM_018089.1 | 1007 | cccgctctggccggtcttgttc | 366574 | - | see also 21272 line |
| 48054 | FLJI10420 | NM_018090.3 | 323 | ggcgacgggcgtttattggaatt | 366579 | - | see also 21273 line |
| 48055 | FLJI10420 | NM_018090.3 | 324 | gcgacgggcgtttattggaatg | 366580 | - | see also 21273 line |
| 48056 | FLJI10439 | NM_018093.1 | 378 | cagcaccgagatggcatattcc | 366630 | - | see also 7253 line |
| 48057 | FLJI10439 | NM_018093.1 | 778 | taccaccagtccgtgtttatga | 366633 | - | see also 7253 line |
| 48058 | FLJI10460 | NM_018097.1 | 580 | ctcaagtggcgtaaacaacaaaa | 366654 | - | see also 21274 line |
| 48059 | FLJI10460 | NM_018097.1 | 438 | gactttcattgagagattagaaa | 366652 | - | see also 21274 line |
| 48060 | FLJI10460 | NM_018097.1 | 121 | aacatgtctaagaaaaacagtttc | 366644 | - | see also 21274 line |
| 48061 | FLJI10462 | NM_018099.3 | 1088 | gacgtaattcagttgatacagt | 366674 | - | see also 21275 line |
| 48062 | FLJI10462 | NM_018099.3 | 814 | gtggttagacgatgctattattg | 366669 | - | see also 21275 line |
| 48063 | FLJI10493 | NM_018112.1 | 578 | gtggtaccattataacgaatttt | 366831 | - | see also 21277 line |
| 48064 | FLJI10493 | NM_018112.1 | 575 | caggtggtaccattataacgaat | 366829 | - | see also 21277 line |
| 48065 | FLJI10498 | NM_018115.1 | 1875 | ggcactacgagatgcactttga | 366852 | - | see also 21278 line |
| 48066 | FLJI10498 | NM_018115.1 | 1492 | agcactacgccgagtttaactca | 366848 | - | see also 21278 line |
| 48067 | FLJI10498 | NM_018115.1 | 1494 | cactagccgagtttaactcagg | 366849 | - | see also 21278 line |
| 48068 | FLJI10520 | NM_018124.2 | 1075 | accgtgtccagacttataatgc | 366954 | - | see also 21279 line |
| 48069 | FLJI10520 | NM_018124.2 | 1074 | taccgtgtccagacttataatg | 366953 | - | see also 21279 line |
| 48070 | FLJI10521 | NM_018125.2 | 866 | agcacaagtacgattgtaagatg | 366975 | - | see also 7268 line |
| 48071 | FLJI10521 | NM_018125.2 | 503 | gtcgcattgaccggttcacttc | 366972 | - | see also 7268 line |
| 48072 | FLJI10521 | NM_018125.2 | 1368 | gtccatggtctagatgtgtaca | 366976 | - | see also 7268 line |
| 48073 | FLJI10534 | NM_018128.4 | 1594 | aagcgctttccgcatgacaaact | 367020 | - | see also 21281 line |
| 48074 | FLJI10534 | NM_018128.4 | 2437 | gtggctgctcgaattcgatttca | 367029 | - | see also 21281 line |
| 48075 | FLJI10539 | NM_018130.1 | 534 | tccgcagtgccactatggatttg | 367061 | - | see also 21282 line |
| 48076 | FLJI10539 | NM_018130.1 | 1782 | tgctgtaaaccgcagcaatattc | 367097 | - | see also 21282 line |
| 48077 | FLJI10546 | NM_018133.2 | 772 | gagccgcctagtggtcaactacg | 367159 | - | see also 21283 line |
| 48078 | FLJI10546 | NM_018133.2 | 2169 | aacgctgccctgctactctaac | 367193 | - | see also 21283 line |
| 48079 | FLJI10547 | NM_018134.1 | 860 | accgtgtctataccatcaaaca | 367212 | - | see also 21284 line |
| 48080 | FLJI10547 | NM_018134.1 | 1040 | tgcactctatgaggactcaaata | 367214 | - | see also 21284 line |
| 48081 | FLJI10547 | NM_018134.1 | 856 | gaccaccgtgtctataccatca | 367211 | - | see also 21284 line |
| 48082 | FLJI10565 | NM_018140.1 | 1648 | gacactgcagccacgttaaattt | 367601 | - | see also 21285 line |
| 48083 | FLJI10565 | NM_018140.1 | 1653 | tgcagccacgttaaatttgcaga | 367603 | - | see also 21285 line |
| 48084 | FLJI10569 | NM_018142.1 | 614 | ttcgattaactgccaatttattg | 367612 | - | see also 7273 line |
| 48085 | FLJI10569 | NM_018142.1 | 1563 | tgcgctagggagtaacagaatga | 367649 | - | see also 7273 line |
| 48086 | FLJI10579 | NM_018145.1 | 339 | gactatacgcagacttcagatcc | 367659 | - | see also 21287 line |
| 48087 | FLJI10579 | NM_018145.1 | 720 | gccaatgcggagtcgacaatga | 367660 | - | see also 21287 line |

Figure 46

| | | | | | | | |
|---|---|---|---|---|---|---|---|
| 48088 | FLJ10597 | NM_016501.2 | 396 | ctggtgttcagtggtgtatatt | 357897 | - | see also 21288 line |
| 48089 | FLJ10597 | NM_016501.2 | 455 | atgatcctcctttagtcttagc | 357898 | - | see also 21288 line |
| 48090 | FLJ10637 | NM_018164.1 | 1114 | cagtcgagaaggctcgtttaaag | 367843 | - | see also 7291 line |
| 48091 | FLJ10652 | NM_018169.1 | 396 | agcggcgtgcagacctctaaaga | 367905 | - | see also 21289 line |
| 48092 | FLJ10652 | NM_018169.1 | 194 | cacgaaagcgagttcatctaaat | 367898 | - | see also 21289 line |
| 48093 | FLJ10661 | NM_152563.1 | 261 | agcgggctcctcaatttcacatg | 367980 | - | see also 21290 line |
| 48094 | FLJ10665 | NM_018173.1 | 1910 | ctctggctacggcactttgatcc | 367988 | - | see also 21291 line |
| 48095 | FLJ10665 | NM_018173.1 | 2087 | ccccgaactgccggaaggaatcc | 367989 | - | see also 21291 line |
| 48096 | FLJ10665 | NM_018173.1 | 198 | gcctcccgcattgagacttatgg | 367983 | - | see also 21291 line |
| 48097 | FLJ10697 | NM_018181.3 | 1073 | agcgttatcgtcaagaatgttcg | 368067 | - | see also 21292 line |
| 48098 | FLJ10697 | NM_018181.3 | 4170 | gacgtaccttaccaaacgtttg | 368127 | - | see also 21292 line |
| 48099 | FLJ10700 | NM_018182.1 | 909 | gaccgtgtcacctcaactatcc | 368141 | - | see also 7307 line |
| 48100 | FLJ10700 | NM_018182.1 | 311 | ccgaattggatgcgtatgctaag | 368138 | - | see also 7307 line |
| 48101 | FLJ10724 | NM_018194.1 | 209 | tgggaactggcactttacctact | 368302 | - | see also 21294 line |
| 48102 | FLJ10724 | NM_018194.1 | 1565 | tggaataggatcttcattaacaagg | 368320 | - | see also 21294 line |
| 48103 | FLJ10737 | NM_018198.1 | 964 | gacgatcagactgtgtgaaagg | 368352 | - | see also 21295 line |
| 48104 | FLJ10737 | NM_018198.1 | 173 | cacaggcggaagcgactgtttaac | 368334 | - | see also 21295 line |
| 48105 | FLJ10743 | NM_018201.2 | 546 | accggagtgggtcacaaatatg | 368382 | - | see also 21296 line |
| 48106 | FLJ10743 | NM_018201.2 | 345 | gccggtggaacacgtacttcaag | 368376 | - | see also 21296 line |
| 48107 | FLJ10743 | NM_018201.2 | 820 | ggccgagatccgggacaacttta | 368385 | - | see also 21296 line |
| 48108 | FLJ10747 | NM_018202.2 | 612 | ttggcttcaaagttacgtaagc | 368395 | - | see also 21297 line |
| 48109 | FLJ10747 | NM_018202.2 | 1499 | caggaagcccgaagttttgtaga | 368409 | - | see also 21297 line |
| 48110 | FLJ10748 | NM_018203.1 | 1080 | tcctccgaggtccaagatctac | 368420 | - | see also 21298 line |
| 48111 | FLJ10748 | NM_018203.1 | 928 | ttctgtcaaggccaaagattacc | 368419 | - | see also 21298 line |
| 48112 | FLJ10781 | NM_018215.2 | 1549 | ctgggcgtgccaccaatgaatcc | 368532 | - | see also 21299 line |
| 48113 | FLJ10781 | NM_018215.2 | 420 | taccgcgtgctcaacaagatttt | 368517 | - | see also 21299 line |
| 48114 | FLJ10781 | NM_018215.2 | 416 | cccgtaccgcgtgctcaacaaga | 368516 | - | see also 21299 line |
| 48115 | FLJ10786 | NM_018219.1 | 1381 | gccgtaccggtctcgtgatagaaa | 368543 | - | see also 21300 line |
| 48116 | FLJ10786 | NM_018219.1 | 749 | gccgtccaccagagtttctcaat | 368538 | - | see also 21300 line |
| 48117 | FLJ10803 | NM_018224.1 | 531 | caccttgacgaggtcttttaga | 368582 | - | - |
| 48118 | FLJ10803 | NM_018224.1 | 304 | ttcattccaggcgtttattattac | 368579 | - | see also 48117 line |
| 48119 | FLJ10803 | NM_018224.1 | 406 | agtccatcgacagggaaaacttc | 368581 | - | see also 48117 line |
| 48120 | FLJ10817 | NM_018076.2 | 1759 | gccgagactatcgcgaatgttgc | 366256 | - | see also 21301 line |
| 48121 | FLJ10817 | NM_018076.2 | 1691 | accttggggcttaccaattatg | 366252 | - | see also 21301 line |
| 48122 | FLJ10817 | NM_018076.2 | 2988 | ggctccaactagtgcgttatctga | 366291 | - | see also 21301 line |
| 48123 | FLJ10846 | NM_018241.1 | 557 | accttagttagtcggtcatctacc | 368896 | - | see also 21302 line |
| 48124 | FLJ10846 | NM_018241.1 | 907 | atctaactaaccggtatccaaat | 368914 | - | see also 21302 line |

Figure 46

| | | | | | | | |
|---|---|---|---|---|---|---|---|
| 48125 | FLJ10853 | NM_018246.1 | 340 | gccgtggtctaacctgaagaaaa | 368977 | - | see also 7342 line |
| 48126 | FLJ10871 | NM_018250.1 | 288 | gagtgctcgggtcatgtatttgt | 369102 | - | see also 21303 line |
| 48127 | FLJ10871 | NM_018250.1 | 1003 | tcctggagtgcctatatcagtac | 369116 | - | see also 21303 line |
| 48128 | FLJ10874 | NM_018252.1 | 921 | gacaagtgtggttaactacacattg | 369143 | - | see also 21304 line |
| 48129 | FLJ10874 | NM_018252.1 | 516 | cagctgtaagcaccattacgagg | 369138 | - | see also 21304 line |
| 48130 | FLJ10884 | NM_019079.2 | 754 | atcacgaagttacgaagtcatgg | 374997 | - | see also 21305 line |
| 48131 | FLJ10884 | NM_019079.2 | 704 | aacactaacgaatacaatagtaa | 374995 | - | see also 21305 line |
| 48132 | FLJ10890 | NM_018259.2 | 3227 | tggaatgacgccgtcatagtagc | 369348 | - | see also 21306 line |
| 48133 | FLJ10890 | NM_018259.2 | 1799 | tcccgtattaataactatactat | 369318 | - | see also 21306 line |
| 48134 | FLJ10901 | NM_018265.1 | 505 | aggatcggagcggcttacaaact | 369515 | - | see also 21307 line |
| 48135 | FLJ10901 | NM_018265.1 | 10 | atgccgaagttaaatgaaatacc | 369513 | - | see also 21307 line |
| 48136 | FLJ10902 | NM_018266.1 | 881 | ccctcgtcaatctttcgaagc | 369531 | - | see also 7356 line |
| 48137 | FLJ10902 | NM_018266.1 | 1607 | atcgaccattaaggctgttaaat | 369554 | - | see also 7356 line |
| 48138 | FLJ10902 | NM_018266.1 | 746 | tgcttgcgagagaggcttgtatgg | 369523 | - | see also 7356 line |
| 48139 | FLJ10904 | NM_018268.1 | 1033 | aggcctatgaacgcaacttctgg | 369585 | - | see also 21309 line |
| 48140 | FLJ10904 | NM_018268.1 | 311 | ggccgagaaatctcctttacaga | 369562 | - | see also 21309 line |
| 48141 | FLJ10922 | NM_018273.2 | 1040 | atgcagtacgtccaacaactcg | 369631 | - | see also 7360 line |
| 48142 | FLJ10922 | NM_018273.2 | 413 | ccctcgatcagccatcactaacg | 369623 | - | see also 7360 line |
| 48143 | FLJ10936 | NM_018279.2 | 825 | gaggctagtcgttgatttattc | 369667 | - | see also 21311 line |
| 48144 | FLJ10936 | NM_018279.2 | 831 | tagtcgttgatttattcctaacc | 369669 | - | see also 21311 line |
| 48145 | FLJ10970 | NM_018286.1 | 594 | aacagctctcgtggcaaatcaga | 369699 | - | see also 21312 line |
| 48146 | FLJ10979 | NM_018289.2 | 1255 | atctctacgtgtatatcgaatcc | 369797 | - | see also 21313 line |
| 48147 | FLJ10979 | NM_018289.2 | 967 | tgcgtaccagacgcagaaggaaatt | 369790 | - | see also 21313 line |
| 48148 | FLJ10996 | NM_019044.2 | 1610 | gccgtgccgagctaataacagtat | 374654 | - | see also 21314 line |
| 48149 | FLJ10996 | NM_019044.2 | 1614 | tgccgagctaattacagtatcaga | 374656 | - | see also 21314 line |
| 48150 | FLJ10996 | NM_019044.2 | 1605 | cctagccgtgccgagctaatac | 374652 | - | see also 21314 line |
| 48151 | FLJ10997 | NM_018293.1 | 650 | accgtacatccaaccgatttaaa | 369819 | - | see also 21315 line |
| 48152 | FLJ10997 | NM_018293.1 | 651 | ccgtacatccaaccgatttaaat | 369820 | - | see also 21315 line |
| 48153 | FLJ10998 | NM_018294.3 | 1197 | gccactctgagacggttctttaa | 369853 | - | see also 21316 line |
| 48154 | FLJ10998 | NM_018294.3 | 634 | atgagaggcttccatatcgaaac | 369843 | - | see also 21316 line |
| 48155 | FLJ11000 | NM_018295.1 | 354 | acctgagaatcggcttctataac | 369863 | - | see also 21317 line |
| 48156 | FLJ11000 | NM_018295.1 | 353 | aacctgagaatcggcttctataa | 369862 | - | see also 21317 line |
| 48157 | FLJ11004 | NM_018296.2 | 1806 | tgccgagctgccctaatgaca | 369891 | - | see also 21318 line |
| 48158 | FLJ11004 | NM_018296.2 | 2228 | atgagactggtcagtatctaata | 369905 | - | see also 21318 line |
| 48159 | FLJ11017 | NM_018302.1 | 760 | agctggagacaacgttgatcata | 369978 | - | see also 21319 line |
| 48160 | FLJ11017 | NM_018302.1 | 1061 | gcggaggcccttgcagctttaga | 369984 | - | see also 21319 line |
| 48161 | FLJ11021 | NM_023012.3 | 1106 | agccgctgttaatccaatgaaat | 384976 | - | see also 21320 line |

Figure 46

| | | | | | | | |
|---|---|---|---|---|---|---|---|
| 48162 | FLJ11021 | NM_023012.3 | 1082 | agctgttcctagctactataacc | 384973 | - | see also 21320 line |
| 48163 | FLJ11029 | NM_018304.1 | 891 | gtgttatcgactcgagttacaaa | 370008 | - | see also 21321 line |
| 48164 | FLJ11029 | NM_018304.1 | 901 | ctcgagttacaaacgtcttaatc | 370009 | - | see also 21321 line |
| 48165 | FLJ11036 | NM_018306.2 | 439 | tggggagcctgacgtttgaagg | 370021 | - | see also 21322 line |
| 48166 | FLJ11036 | NM_018306.2 | 555 | aagtggaggcctcagttaaga | 370023 | - | see also 21322 line |
| 48167 | FLJ11046 | NM_018309.1 | 1964 | atcttggcaatcgaaagtattt | 370091 | - | see also 7384 line |
| 48168 | FLJ11046 | NM_018309.1 | 423 | aactgccacgcagcgatttatac | 370043 | - | see also 7384 line |
| 48169 | FLJ11046 | NM_018309.1 | 427 | gccacgcagcgatttatacaact | 370045 | - | see also 7384 line |
| 48170 | FLJ11082 | NM_018317.1 | 741 | ctgggtatagatgatctacaca | 370225 | - | see also 7399 line |
| 48171 | FLJ11082 | NM_018317.1 | 1426 | aaccacttcgcatatcatttaag | 370247 | - | see also 7399 line |
| 48172 | FLJ11088 | NM_018318.2 | 250 | tagcattgtgagtcaaactattc | 370257 | - | see also 21325 line |
| 48173 | FLJ11088 | NM_018318.2 | 168 | tggaccgtgaccactcttcttcc | 370255 | - | see also 21325 line |
| 48174 | FLJ11127 | NM_019018.1 | 256 | ttccgaggctacatttatattc | 374471 | - | see also 21326 line |
| 48175 | FLJ11127 | NM_019018.1 | 959 | gactagagcagattgatatgttt | 374486 | - | see also 21326 line |
| 48176 | FLJ11171 | NM_018348.4 | 2092 | ttcgactctcgtaatatcaaaa | 370623 | - | see also 21327 line |
| 48177 | FLJ11171 | NM_018348.4 | 1796 | tccaatcttgagtgtcacttatg | 370616 | - | see also 21327 line |
| 48178 | FLJ11184 | NM_018352.1 | 156 | aagtcatccatcatatagtaga | 370641 | - | see also 21328 line |
| 48179 | FLJ11184 | NM_018352.1 | 158 | gtcatccatccatatagtagaaa | 370642 | - | see also 21328 line |
| 48180 | FLJ11193 | NM_018356.1 | 305 | cagcagacaccgtgtttgataag | 370657 | - | see also 21329 line |
| 48181 | FLJ11193 | NM_018356.1 | 1175 | ttcaccaggctgtttaacctgc | 370681 | - | see also 21329 line |
| 48182 | FLJ11200 | NM_018359.1 | 1128 | accaattgatcgtataacgtca | 370726 | - | see also 7413 line |
| 48183 | FLJ11200 | NM_018359.1 | 1133 | ttgatcggttataacgtcaaaaat | 370727 | - | see also 7413 line |
| 48184 | FLJ11200 | NM_018359.1 | 1432 | tcctcagcgaccaaatatgatt | 370741 | - | see also 7413 line |
| 48185 | FLJ11218 | NM_018363.1 | 323 | gcctctaagctcgcctattgaag | 370775 | - | see also 21331 line |
| 48186 | FLJ11218 | NM_018363.1 | 724 | tacgcttcgtctccattgataat | 370791 | - | see also 21331 line |
| 48187 | FLJ11220 | NM_018364.2 | 1790 | atgtcggtcagggtttgactgg | 370833 | - | see also 7417 line |
| 48188 | FLJ11220 | NM_018364.2 | 1686 | aagacgacgatcaatgaatgaaa | 370829 | - | see also 7417 line |
| 48189 | FLJ11235 | NM_019033.1 | 16 | atgaagcgctcggcttttctttt | 374580 | - | see also 21333 line |
| 48190 | FLJ11235 | NM_019033.1 | 252 | gccgtgttgagccaagttcaagc | 374583 | - | see also 21333 line |
| 48191 | FLJ11259 | NM_018370.1 | 90 | gggcattgtgccaattttcagg | 370890 | - | see also 7424 line |
| 48192 | FLJ11259 | NM_018370.1 | 88 | atgggcattgtgccaatttca | 370889 | - | see also 7424 line |
| 48193 | FLJ11267 | NM_019607.1 | 366 | ctgtcgggagcacttcgaaaacc | 375505 | - | see also 21335 line |
| 48194 | FLJ11267 | NM_019607.1 | 656 | gaccagatgtacgagtttgaaa | 375510 | - | see also 21335 line |
| 48195 | FLJ11280 | NM_018379.2 | 990 | gtcagaggaacttcagcttaatt | 370932 | - | see also 21336 line |
| 48196 | FLJ11280 | NM_018379.2 | 1367 | agggtgaacttagcgtctttttc | 370938 | - | see also 21336 line |
| 48197 | FLJ11280 | NM_018379.2 | 1377 | tagcgtcttttccgaaacaacc | 370939 | - | see also 21336 line |
| 48198 | FLJ11286 | NM_018381.1 | 231 | atcgttacggggtaaagcaaaa | 370951 | - | see also 21337 line |

Figure 46

| | | | | | | | |
|---|---|---|---|---|---|---|---|
| 48199 | FLJ11286 | NM_018381.1 | 362 | agccaacctacgcatgtttcaac | 370959 | - | see also 21337 line |
| 48200 | FLJ11292 | NM_018382.1 | 152 | tgggactctggcagcaaatttca | 370964 | - | see also 21338 line |
| 48201 | FLJ11292 | NM_018382.1 | 160 | tggcagcaaatttcaaactgtat | 370965 | - | see also 21338 line |
| 48202 | FLJ11301 | NM_018385.1 | 1386 | agcggaatcctcccaattgatca | 370995 | - | see also 21339 line |
| 48203 | FLJ11301 | NM_018385.1 | 1385 | cagcggaatcctcccaattgatc | 370994 | - | see also 21339 line |
| 48204 | FLJ11305 | NM_018386.1 | 657 | agcgacacccgtgctggtataga | 371015 | - | see also 21340 line |
| 48205 | FLJ11305 | NM_018386.1 | 288 | accgtgatagtccaatatccttt | 371011 | - | see also 21340 line |
| 48206 | FLJ11331 | NM_018392.3 | 1276 | tagaccagtcagtcggtaataat | 371055 | - | see also 21341 line |
| 48207 | FLJ11331 | NM_018392.3 | 2148 | cccccgaataaatctaaaggtat | 371078 | - | see also 21341 line |
| 48208 | FLJ11336 | NM_018393.2 | 1668 | tgctcgcctggtcaactataaca | 371137 | - | see also 7437 line |
| 48209 | FLJ11362 | NM_021946.2 | 2931 | tagcggtggggacattcgaatga | 381553 | - | see also 8209 line |
| 48210 | FLJ11362 | NM_021946.2 | 1610 | tccgttcttacccgtatgcattt | 381544 | - | see also 8209 line |
| 48211 | FLJ11383 | NM_014801.2 | 107 | acgaccaagtcagtaatacctgt | 343677 | - | see also 21344 line |
| 48212 | FLJ11383 | NM_014801.2 | 1305 | gtgggcttctccgagataactgt | 343705 | - | see also 21344 line |
| 48213 | FLJ11457 | NM_024753.2 | 3922 | gacaaatccggcagtaggataca | 390073 | - | see also 21345 line |
| 48214 | FLJ11457 | NM_024753.2 | 3795 | aacggtgcctgcgtcataataga | 390063 | - | see also 21345 line |
| 48215 | FLJ11467 | NM_024963.2 | 1811 | ccgcgctctcagacatgttgaag | 392755 | - | see also 21346 line |
| 48216 | FLJ11467 | NM_024963.2 | 1878 | cagcgccaacgcccagttcttcc | 392757 | - | see also 21346 line |
| 48217 | FLJ11506 | NM_024666.2 | 680 | atcggggtggtgcatctaacaca | 388798 | - | see also 21347 line |
| 48218 | FLJ11506 | NM_024666.2 | 327 | atggttacctgaggtgatgatct | 388794 | - | see also 21347 line |
| 48219 | FLJ11526 | NM_024632.1 | 1000 | accctacgacgttataaacgaca | 388331 | - | see also 21348 line |
| 48220 | FLJ11526 | NM_024632.1 | 997 | aacaccctacgacgttataaacg | 388330 | - | see also 21348 line |
| 48221 | FLJ11535 | NM_024888.1 | 333 | tggccgagggcatgttgtactgt | 391714 | - | see also 21349 line |
| 48222 | FLJ11535 | NM_024888.1 | 157 | ttccatcgtatccttgtacttcc | 391713 | - | see also 21349 line |
| 48223 | FLJ11560 | NM_025182.2 | 1586 | caccgtccaagtgaccttattta | 394293 | - | see also 8874 line |
| 48224 | FLJ11560 | NM_025182.2 | 1887 | accgaggccctcataatccacg | 394298 | - | see also 8874 line |
| 48225 | FLJ11588 | NM_024603.1 | 823 | cagcggtcccgccattgatctgg | 387743 | - | see also 8659 line |
| 48226 | FLJ11712 | NM_024570.1 | 550 | cacacctgtggatcctctatttc | 387340 | - | see also 21351 line |
| 48227 | FLJ11712 | NM_024570.1 | 639 | tggataacgtgtttccaaattgc | 387344 | - | see also 21351 line |
| 48228 | FLJ11730 | NM_022756.3 | 127 | gagcgacagatctatgcttttga | 383970 | - | see also 21352 line |
| 48229 | FLJ11730 | NM_022756.3 | 550 | agcccgtctggcatgtttgatta | 383980 | - | see also 21352 line |
| 48230 | FLJ11730 | NM_022756.3 | 245 | atcgaaggaaccggaagtttaag | 383972 | - | see also 21352 line |
| 48231 | FLJ11752 | NM_152281.1 | 998 | gagaccagtggttcgtttagaga | 415324 | - | see also 9829 line |
| 48232 | FLJ11752 | NM_152281.1 | 810 | cagcgcaagactgagataaaaga | 415320 | - | see also 9829 line |
| 48233 | FLJ11753 | NM_024659.2 | 552 | ttcacgagaaccagttgatatat | 388667 | - | see also 8689 line |
| 48234 | FLJ11753 | NM_024659.2 | 1345 | ttgggcagccctacatggtaaat | 388694 | - | see also 8689 line |
| 48235 | FLJ11773 | NM_021934.3 | 526 | cacaggcaagttccactacaaga | 381380 | - | see also 21355 line |

Figure 46

| | | | | | | | |
|---|---|---|---|---|---|---|---|
| 48236 | FLJ11773 | NM_021934.3 | 934 | cacacagtcgagggtggataacg | 381384 | - | see also 21355 line |
| 48237 | FLJ11785 | NM_021930.2 | 2227 | ttgatatgactcggaatctttc | 381361 | - | see also 8200 line |
| 48238 | FLJ11785 | NM_021930.2 | 1076 | agggaaccggcagactaatgtgt | 381316 | - | see also 8200 line |
| 48239 | FLJ11795 | NM_024669.1 | 660 | ctgccagatgttactagataacc | 388818 | - | see also 21357 line |
| 48240 | FLJ11795 | NM_024669.1 | 298 | cccagcaggaccgatacagaga | 388811 | - | see also 21357 line |
| 48241 | FLJ11800 | NM_024974.1 | 101 | tgcctccgaaacacatcacttga | 392758 | - | see also 21358 line |
| 48242 | FLJ11800 | NM_024974.1 | 304 | cacagagctttggctaattact | 392761 | - | see also 21358 line |
| 48243 | FLJ11848 | NM_025155.1 | 1166 | tgctgtcgagacggtcttgtacg | 394136 | - | see also 21360 line |
| 48244 | FLJ11848 | NM_025155.1 | 964 | tccaagggtccggtacaagtca | 394133 | - | see also 21360 line |
| 48245 | FLJ11856 | NM_024531.3 | 644 | ggcaggacagttgcattctgtgg | 386783 | - | see also 21361 line |
| 48246 | FLJ11856 | NM_024531.3 | 751 | ctcgcttcacggtcattcttc | 386784 | - | see also 21361 line |
| 48247 | FLJ12056 | NM_024933.1 | 117 | tccatgcagcaggcgaacaaagt | 392318 | - | - |
| 48248 | FLJ12057 | NM_024768.1 | 851 | ggctccggacctgaacatatcg | 390318 | - | see also 21362 line |
| 48249 | FLJ12057 | NM_024768.1 | 855 | ccgggacctgaacatatgaaaa | 390319 | - | see also 21362 line |
| 48250 | FLJ12085 | NM_022771.3 | 746 | cccttatacggcaactatgatag | 384059 | - | see also 8462 line |
| 48251 | FLJ12085 | NM_022771.3 | 938 | gggggaacgcccctgttgttcaaa | 384067 | - | see also 8462 line |
| 48252 | FLJ12150 | NM_024736.4 | 998 | cccaagcaacctgcttttattt | 389759 | - | see also 21364 line |
| 48253 | FLJ12150 | NM_024736.4 | 694 | tggaaacccgttataagrtgt | 389755 | - | see also 21364 line |
| 48254 | FLJ12168 | NM_024682.1 | 1362 | ctcctcacctactgcatgtatca | 389125 | - | see also 21365 line |
| 48255 | FLJ12168 | NM_024682.1 | 1161 | aaggcccacatacgcaagaaaac | 389122 | - | see also 21365 line |
| 48256 | FLJ12168 | NM_024682.1 | 1427 | ccgatcctacgtcattcaga | 389126 | - | see also 21365 line |
| 48257 | FLJ12355 | NM_024988.1 | 374 | cggcgtggagtcgccgattttt | 392770 | - | see also 21366 line |
| 48258 | FLJ12355 | NM_024988.1 | 375 | ggcgtggagtcgccgatttttt | 392771 | - | see also 21366 line |
| 48259 | FLJ12355 | NM_024988.1 | 376 | gcgtggagtcgccgatttttt | 392772 | - | see also 21366 line |
| 48260 | FLJ12363 | NM_032167.1 | 280 | cagcgggctttgccagccaaaacc | 398408 | - | see also 21367 line |
| 48261 | FLJ12363 | NM_032167.1 | 191 | ctgtctgtgccagtttgaag | 398407 | - | see also 21367 line |
| 48262 | FLJ12436 | NM_024661.2 | 996 | atggcctagaaaagacttgtagc | 388717 | - | see also 21368 line |
| 48263 | FLJ12436 | NM_024661.2 | 953 | aggcaagtcattcattgtctaga | 388713 | - | see also 21368 line |
| 48264 | FLJ12438 | NM_021933.1 | 1144 | ctccgaagtctgagaaaagctca | 381379 | - | - |
| 48265 | FLJ12438 | NM_021933.1 | 794 | aaccaacaaggaggagrtgtatct | 381378 | - | see also 48264 line |
| 48266 | FLJ12439 | NM_023077.1 | 708 | aaggtgtccaaccctaacattt | 385255 | - | see also 21369 line |
| 48267 | FLJ12439 | NM_023077.1 | 659 | gccgaggtgctaaaaaatcgagc | 385254 | - | see also 21369 line |
| 48268 | FLJ12442 | NM_022908.1 | 1379 | accggatcacccgcttggaaaag | 384789 | - | see also 21370 line |
| 48269 | FLJ12442 | NM_022908.1 | 1477 | ttcggggaccacctctatagtga | 384792 | - | see also 21370 line |
| 48270 | FLJ12442 | NM_022908.1 | 461 | gcctgtcgacgttgctagaaaag | 384773 | - | see also 21370 line |
| 48271 | FLJ12448 | NM_022895.1 | 43 | ctgtgagcgattcggaaagtagt | 384690 | - | see also 21371 line |
| 48272 | FLJ12448 | NM_022895.1 | 49 | gcgattcggaaagtagtaacagc | 384692 | - | see also 21371 line |

Figure 46

| | | | | | | | |
|---|---|---|---|---|---|---|---|
| 48273 | FLJ12505 | NM_024749.2 | 668 | accctacactacttaaccaatgg | 389908 | - | see also 21372 line |
| 48274 | FLJ12505 | NM_024749.2 | 667 | gaccctacactacttaaccaatg | 389907 | - | see also 21372 line |
| 48275 | FLJ12517 | NM_023007.1 | 609 | ctcgtccgactggctgaatgagt | 384930 | - | see also 21373 line |
| 48276 | FLJ12517 | NM_023007.1 | 1120 | tcctgctcgggcatcaactttga | 384935 | - | see also 21373 line |
| 48277 | FLJ12517 | NM_023007.1 | 572 | ttccggtggaggacgttttcacc | 384929 | - | see also 21373 line |
| 48278 | FLJ12525 | NM_031206.2 | 1376 | gacggaatgctcgccgattttct | 395944 | - | see also 8988 line |
| 48279 | FLJ12525 | NM_031206.2 | 971 | acccgtccccacgtgtagaatgt | 395941 | - | see also 8988 line |
| 48280 | FLJ12525 | NM_031206.2 | 881 | cccgagaactgctggtatcatac | 395934 | - | see also 8988 line |
| 48281 | FLJ12541 | NM_022369.2 | 1846 | cacgtaccgaaacttcttgaaga | 382735 | - | see also 21375 line |
| 48282 | FLJ12541 | NM_022369.2 | 1060 | gacagggacggccatttaccagg | 382732 | - | see also 21375 line |
| 48283 | FLJ12571 | NM_024926.1 | 1382 | agctcggtgctatattatgaata | 392302 | - | see also 8808 line |
| 48284 | FLJ12571 | NM_024926.1 | 1078 | tgggttcaagggatcatatgaaa | 392291 | - | see also 8808 line |
| 48285 | FLJ12572 | NM_022905.2 | 993 | aagcacggatcagattatcattt | 384763 | - | see also 21377 line |
| 48286 | FLJ12572 | NM_022905.2 | 650 | tgcgtagcactgacaagaatttg | 384757 | - | see also 21377 line |
| 48287 | FLJ12577 | NM_030925.1 | 493 | tacgtggagttgtcaacatttga | 395518 | - | see also 21378 line |
| 48288 | FLJ12577 | NM_030925.1 | 707 | ttgccatcatgacaaagtatatc | 395525 | - | see also 21378 line |
| 48289 | FLJ12584 | NM_025139.2 | 1570 | ggcaggcctcgtgctcaaagtcc | 393944 | - | see also 8868 line |
| 48290 | FLJ12584 | NM_025139.2 | 973 | ctccttggcacccgtgaaattga | 393937 | - | see also 8868 line |
| 48291 | FLJ12598 | NM_024754.2 | 1078 | atctcacacgttgctattaaaca | 390087 | - | see also 21380 line |
| 48292 | FLJ12598 | NM_024754.2 | 732 | tagctctgaatcagaatgagatg | 390081 | - | see also 21380 line |
| 48293 | FLJ12604 | NM_024621.1 | 1265 | ggccaacatggagcattcgtttc | 388090 | - | see also 8669 line |
| 48294 | FLJ12604 | NM_024621.1 | 1266 | gccaacatggagcattcgtttca | 388091 | - | see also 8669 line |
| 48295 | FLJ12604 | NM_024621.1 | 2591 | ccctgacgactgcccaatagaac | 388122 | - | see also 8669 line |
| 48296 | FLJ12610 | NM_024782.1 | 598 | tacgctgattcgagatcgattga | 390651 | - | see also 21382 line |
| 48297 | FLJ12610 | NM_024782.1 | 823 | aggaatcgatagccaatgtgtaa | 390652 | - | see also 21382 line |
| 48298 | FLJ12650 | NM_024522.1 | 486 | ccccagaagacgtcgcatttaca | 386633 | - | see also 21383 line |
| 48299 | FLJ12650 | NM_024522.1 | 131 | tggctggaatgcatttatcatct | 386631 | - | see also 21383 line |
| 48300 | FLJ12666 | NM_024595.1 | 536 | ctccgacaagttggcataatatg | 387714 | - | see also 21384 line |
| 48301 | FLJ12666 | NM_024595.1 | 560 | gagcgcctcttaaaagactatga | 387718 | - | see also 21384 line |
| 48302 | FLJ12666 | NM_024595.1 | 558 | gtgagcgcctcttaaaagactat | 387717 | - | see also 21384 line |
| 48303 | FLJ12681 | NM_022773.1 | 176 | ggccctagccttcgtgtacttcg | 384093 | - | see also 21385 line |
| 48304 | FLJ12681 | NM_022773.1 | 1543 | gagagcactacaggtacaagttc | 384103 | - | see also 21385 line |
| 48305 | FLJ12684 | NM_024534.2 | 836 | tccggacactatctatcttgact | 386841 | - | see also 21386 line |
| 48306 | FLJ12684 | NM_024534.2 | 830 | cagttttccggacactatctatc | 386840 | - | see also 21386 line |
| 48307 | FLJ12684 | NM_024534.2 | 1465 | cagctatttcgcaacctattttg | 386865 | - | see also 21386 line |
| 48308 | FLJ12700 | NM_024910.1 | 470 | agggggagtcccctaagactacg | 391976 | - | see also 21387 line |
| 48309 | FLJ12716 | NM_021942.4 | 918 | aggaccgcctataatcttgtaca | 381414 | - | see also 21388 line |

Figure 46

| 48310 | FLJ12716 | NM_021942.4 | 3294 | accgacttagttcaagatgtaga | 381498 | - | see also 21388 line | | |
|---|---|---|---|---|---|---|---|---|---|
| 48311 | FLJ12748 | NM_024871.1 | 420 | cacgtcgtacagacaggaattcc | 391584 | - | - | - | - |
| 48312 | FLJ12748 | NM_024871.1 | 603 | ctcagctccccggattctcaacg | 391587 | - | see also 48311 line | | |
| 48313 | FLJ12748 | NM_024871.1 | 463 | ccctcaagatccacaaagacaaa | 391585 | - | see also 48311 line | | |
| 48314 | FLJ12750 | NM_024667.1 | 797 | gccgtgccgtacccaggattaca | 388808 | - | see also 21389 line | | |
| 48315 | FLJ12750 | NM_024667.1 | 755 | cgcttagccaccccgtttactgc | 388807 | - | see also 21389 line | | |
| 48316 | FLJ12787 | NM_032175.1 | 495 | tacggtgtccatcataaaggagt | 398503 | - | see also 9086 line | | |
| 48317 | FLJ12788 | NM_022492.1 | 87 | tgctgcacccaaactttgcaagg | 383484 | - | see also 21391 line | | |
| 48318 | FLJ12788 | NM_022492.1 | 92 | cacccaaactttgcaaggaattc | 383485 | - | see also 21391 line | | |
| 48319 | FLJ12806 | NM_022831.1 | 248 | gcgatagacgagtatcagatatt | 384467 | - | see also 21392 line | | |
| 48320 | FLJ12806 | NM_022831.1 | 247 | ggcgatagacgagtatcagatat | 384466 | - | see also 21392 line | | |
| 48321 | FLJ12875 | NM_024544.1 | 353 | agctgtgcggtctgttaaagaaa | 386944 | - | see also 21393 line | | |
| 48322 | FLJ12875 | NM_024544.1 | 327 | tgcgtgccttatgctgttataga | 386942 | - | see also 21393 line | | |
| 48323 | FLJ12886 | NM_019108.1 | 460 | gtcgtgggtcaggccaaactact | 375243 | - | see also 21394 line | | |
| 48324 | FLJ12886 | NM_019108.1 | 658 | gaggaccagaggacttatgtttt | 375247 | - | see also 21394 line | | |
| 48325 | FLJ12903 | NM_022753.1 | 507 | aactaagaccaggactaatgttc | 383966 | - | see also 21395 line | | |
| 48326 | FLJ12903 | NM_022753.1 | 254 | ttcaacagactgtctctgataaa | 383955 | - | see also 21395 line | | |
| 48327 | FLJ12949 | NM_023008.2 | 464 | aggagacatcgtcgcaaagttat | 384947 | - | see also 21396 line | | |
| 48328 | FLJ12949 | NM_023008.2 | 982 | tcctccgtgcgccgtaaggatga | 384952 | - | see also 21396 line | | |
| 48329 | FLJ12953 | NM_032118.2 | 140 | cggctcgcaacctcacgtatttt | 397779 | - | see also 9076 line | | |
| 48330 | FLJ12953 | NM_032118.2 | 327 | ctcacatcgaggaatacagatgt | 397785 | - | see also 9076 line | | |
| 48331 | FLJ12973 | NM_024908.1 | 723 | tccaacaccgccgacattagtag | 391917 | - | see also 21397 line | | |
| 48332 | FLJ12973 | NM_024908.1 | 669 | aaggtcaatgcgattactaaaag | 391913 | - | see also 21397 line | | |
| 48333 | FLJ12975 | NM_024809.2 | 1667 | tccgattacgctgatcttagtga | 390916 | - | see also 21398 line | | |
| 48334 | FLJ12975 | NM_024809.2 | 1119 | ttgccttaggaggcatagttaca | 390898 | - | see also 21398 line | | |
| 48335 | FLJ12994 | NM_022841.2 | 3449 | ttcatcgccaacgtaatcttagt | 384629 | - | see also 21399 line | | |
| 48336 | FLJ12994 | NM_022841.2 | 2927 | gccgacatacacccattggtact | 384609 | - | see also 21399 line | | |
| 48337 | FLJ13031 | NM_024688.1 | 1052 | aaggcgtaatactcgtattgtag | 389259 | - | see also 21400 line | | |
| 48338 | FLJ13031 | NM_024688.1 | 2001 | atgtccgtacaacgtcaagaagg | 389286 | - | see also 21400 line | | |
| 48339 | FLJ13081 | NM_024834.1 | 1376 | cacagaacacttgtatcgaatta | 391265 | - | see also 21401 line | | |
| 48340 | FLJ13081 | NM_024834.1 | 576 | ggggaatctacgtgggtaaaaga | 391237 | - | see also 21401 line | | |
| 48341 | FLJ13089 | NM_024953.2 | 295 | tgcaccgaccggagttagttaca | 392606 | - | see also 8822 line | | |
| 48342 | FLJ13089 | NM_024953.2 | 1005 | gagctgattaggcgtttacgaag | 392638 | - | see also 8822 line | | |
| 48343 | FLJ13096 | NM_025000.1 | 759 | cactacatcgtcacacctaataa | 392875 | - | see also 21403 line | | |
| 48344 | FLJ13096 | NM_025000.1 | 1061 | ttctggacgggtggtaaaaaaaa | 392888 | - | see also 21403 line | | |
| 48345 | FLJ13110 | NM_022912.1 | 189 | caccctttaccctgcgtattatt | 384796 | - | see also 8530 line | | |
| 48346 | FLJ13110 | NM_022912.1 | 190 | accctttaccctgcgtattattc | 384797 | - | see also 8530 line | | |

Figure 46

| | | | | | | | |
|---|---|---|---|---|---|---|---|
| 48347 | FLJ13110 | NM_022912.1 | 686 | aacacggccagcctaagatgtcc | 384801 | - | see also 8530 line |
| 48348 | FLJ13114 | NM_024541.1 | 44 | ttccctgccgtcctttagtatgt | 386911 | - | see also 8635 line |
| 48349 | FLJ13114 | NM_024541.1 | 185 | gtcgggtacgcctgcattacacc | 386920 | - | see also 8635 line |
| 48350 | FLJ13150 | NM_024813.1 | 1534 | aacgcatcgtacttgaaaagttg | 390997 | - | see also 21405 line |
| 48351 | FLJ13150 | NM_024813.1 | 294 | gtcgtggatgaacgttctattgt | 390970 | - | see also 21405 line |
| 48352 | FLJ13150 | NM_024813.1 | 998 | aactctagtaggcataagtaaga | 390985 | - | see also 21405 line |
| 48353 | FLJ13154 | NM_024598.1 | 329 | cacccacgtctatgtaccatatg | 387722 | - | - | - | - |
| 48354 | FLJ13154 | NM_024598.1 | 330 | acccacgtctatgtaccatatga | 387723 | - | see also 48353 line |
| 48355 | FLJ13154 | NM_024598.1 | 333 | cacgtctatgtaccatatgaagc | 387724 | - | see also 48353 line |
| 48356 | FLJ13188 | NM_022063.1 | 660 | tagatttgacccgcctgttaaaa | 381783 | - | see also 8259 line |
| 48357 | FLJ13188 | NM_022063.1 | 599 | gtgaaccgtcctcaaatgaaacc | 381781 | - | see also 8259 line |
| 48358 | FLJ13197 | NM_024614.1 | 148 | gtgagggagacgggacttacttt | 387969 | - | see also 21407 line |
| 48359 | FLJ13197 | NM_024614.1 | 150 | gagggagacgggacttactttgc | 387970 | - | see also 21407 line |
| 48360 | FLJ13215 | NM_025004.1 | 803 | caccggctagcatcctttaaaac | 393088 | - | see also 21408 line |
| 48361 | FLJ13215 | NM_025004.1 | 524 | taccgagtaaatcaacaaattag | 393077 | - | see also 21408 line |
| 48362 | FLJ13231 | NM_023073.2 | 2670 | atgataaggtggtctaatagaag | 385138 | - | see also 21409 line |
| 48363 | FLJ13231 | NM_023073.2 | 1539 | gaggacgtgagggttcctttaag | 385106 | - | see also 21409 line |
| 48364 | FLJ13236 | NM_024902.2 | 895 | ggcatcgccgctacaaagctttg | 391883 | - | see also 21410 line |
| 48365 | FLJ13236 | NM_024902.2 | 1251 | ctctcagaaggggcaacaaatga | 391887 | - | see also 21410 line |
| 48366 | FLJ13263 | NM_025125.2 | 348 | agcgtctctcaccatcttgattg | 393822 | - | see also 21411 line |
| 48367 | FLJ13263 | NM_025125.2 | 373 | tacaaacggaagcgccaaaaaca | 393825 | - | see also 21411 line |
| 48368 | FLJ13273 | NM_024751.1 | 1593 | ttctgcagcggtggggtatttta | 389941 | - | see also 21412 line |
| 48369 | FLJ13273 | NM_024751.1 | 1595 | ctgcagcggtggggtattttatc | 389942 | - | see also 21412 line |
| 48370 | FLJ13291 | NM_032178.1 | 418 | ctcgggctttcagttgttagacc | 398530 | - | see also 21413 line |
| 48371 | FLJ13291 | NM_032178.1 | 82 | ggctcttgtgctcgcttgtaaac | 398529 | - | see also 21413 line |
| 48372 | FLJ13310 | NM_025118.1 | 701 | tgccaatagctgggcagtttatc | 393806 | - | see also 21414 line |
| 48373 | FLJ13310 | NM_025118.1 | 939 | gtgctttggtcgaagaatagacc | 393814 | - | see also 21414 line |
| 48374 | FLJ13330 | NM_025091.2 | 494 | ggccacccgcatcctaaatcagg | 393567 | - | see also 21415 line |
| 48375 | FLJ13330 | NM_025091.2 | 408 | ctgaaaaagtcacctggtttaaa | 393565 | - | see also 21415 line |
| 48376 | FLJ13352 | NM_024592.1 | 286 | gccgcctgccgagcctttgatgt | 387670 | - | see also 21416 line |
| 48377 | FLJ13352 | NM_024592.1 | 288 | cgcctgccgagcctttgatgtcc | 387671 | - | see also 21416 line |
| 48378 | FLJ13386 | NM_025180.2 | 2172 | gaggacaagcgtcggatagtata | 394237 | - | see also 8873 line |
| 48379 | FLJ13386 | NM_025180.2 | 2295 | tgccttcagcgctagatacaaat | 394241 | - | see also 8873 line |
| 48380 | FLJ13391 | NM_032181.1 | 500 | tagcggcctattcctttgtctca | 398540 | - | see also 21418 line |
| 48381 | FLJ13391 | NM_032181.1 | 584 | ccctggctgctctggtgataagg | 398542 | - | see also 21418 line |
| 48382 | FLJ13441 | NM_023924.1 | 653 | gccagataccgtgtactacaagt | 385316 | - | see also 21419 line |
| 48383 | FLJ13441 | NM_023924.1 | 652 | ggccagataccgtgtactacaag | 385315 | - | see also 21419 line |

Figure 46

| 48384 | FLJ13441 | NM_023924.1 | 691 | ttcacgcaggctttaagatgatg | 385319 | - | see also 21419 line |
|---|---|---|---|---|---|---|---|
| 48385 | FLJ13544 | NM_025008.1 | 522 | ctcgggatgggattatgtgtact | 393110 | - | see also 21420 line |
| 48386 | FLJ13544 | NM_025008.1 | 517 | cccagctcgggatgggattatgt | 393108 | - | see also 21420 line |
| 48387 | FLJ13576 | NM_022484.2 | 1839 | gacccagacatgatacgtatatt | 383322 | - | see also 21421 line |
| 48388 | FLJ13576 | NM_022484.2 | 1988 | atcgtattagacagcgaaaattc | 383331 | - | see also 21421 line |
| 48389 | FLJ13576 | NM_022484.2 | 807 | gtcatcggcccactttactaacc | 383289 | - | see also 21421 line |
| 48390 | FLJ13611 | NM_024941.1 | 260 | cagttatatcagcgttcataatg | 392359 | - | see also 8813 line |
| 48391 | FLJ13611 | NM_024941.1 | 655 | tggagccatctattatgtacaat | 392373 | - | see also 8813 line |
| 48392 | FLJ13614 | NM_139076.1 | 1034 | ttcaagagatctcggttgttaga | 409351 | - | see also 21423 line |
| 48393 | FLJ13614 | NM_139076.1 | 405 | atcgactggaacattccttatat | 409328 | - | see also 21423 line |
| 48394 | FLJ13614 | NM_139076.1 | 807 | gtcaggcattacggaccttttt | 409339 | - | see also 21423 line |
| 48395 | FLJ13615 | NM_025114.1 | 1700 | atcgggagtatatggtttagaag | 393766 | - | see also 21424 line |
| 48396 | FLJ13615 | NM_025114.1 | 1570 | ttcagtcaacgttagacatttta | 393763 | - | see also 21424 line |
| 48397 | FLJ13621 | NM_025009.1 | 343 | ttcagcgacttagttcatacaac | 393115 | - | see also 21425 line |
| 48398 | FLJ13621 | NM_025009.1 | 346 | agcgacttagttcatacaactga | 393116 | - | see also 21425 line |
| 48399 | FLJ13639 | NM_024705.1 | 226 | atcgccaagcgagaacatttttc | 389442 | - | see also 21426 line |
| 48400 | FLJ13639 | NM_024705.1 | 522 | atgatctccagtccgaaagaaca | 389446 | - | see also 21426 line |
| 48401 | FLJ13657 | NM_024828.1 | 246 | ctgctgtgggaacgccaatttta | 391151 | - | see also 21427 line |
| 48402 | FLJ13657 | NM_024828.1 | 478 | gtgtcattgaaaccagttagttt | 391154 | - | see also 21427 line |
| 48403 | FLJ13710 | NM_024817.1 | 109 | agcgtccaaatgagatttcgagc | 391054 | - | see also 21428 line |
| 48404 | FLJ13710 | NM_024817.1 | 559 | aaggatcgcagtaccctatttc | 391062 | - | see also 21428 line |
| 48405 | FLJ13710 | NM_024817.1 | 449 | tccccatcgaccggacaacttgg | 391057 | - | see also 21428 line |
| 48406 | FLJ13725 | NM_024519.2 | 2536 | gaggtgacccgcctagaaagtct | 386603 | - | see also 21429 line |
| 48407 | FLJ13725 | NM_024519.2 | 739 | ctgtgtgtaggcgatcagtatga | 386578 | - | see also 21429 line |
| 48408 | FLJ13744 | NM_025011.1 | 653 | atggatgcgtctatgtgtaatga | 393147 | - | see also 21430 line |
| 48409 | FLJ13744 | NM_025011.1 | 529 | gagctgaactccggtattcattg | 393140 | - | see also 21430 line |
| 48410 | FLJ13798 | NM_024773.1 | 246 | ctcctgccgcacagtaaagaaga | 390443 | - | see also 21431 line |
| 48411 | FLJ13798 | NM_024773.1 | 678 | agggcggaccatggtttgattcc | 390451 | - | see also 21431 line |
| 48412 | FLJ13840 | NM_024746.2 | 1364 | ggccgaggccggatattctgtgg | 389880 | - | see also 21432 line |
| 48413 | FLJ13840 | NM_024746.2 | 991 | ttctcgggctgatcctaacaaag | 389875 | - | see also 21432 line |
| 48414 | FLJ13840 | NM_024746.2 | 1064 | aaccataatggcggacaacttct | 389877 | - | see also 21432 line |
| 48415 | FLJ13841 | NM_024702.1 | 1403 | gtcgagttcgaaagtccaattcc | 389434 | - | see also 21433 line |
| 48416 | FLJ13841 | NM_024702.1 | 1174 | tccagccacatacgatcactaca | 389426 | - | see also 21433 line |
| 48417 | FLJ13868 | NM_022744.1 | 1234 | gtgcgggcaggtcctaagaaaga | 383859 | - | see also 8452 line |
| 48418 | FLJ13868 | NM_022744.1 | 379 | aacgcaaaagccactgtttcagc | 383850 | - | see also 8452 line |
| 48419 | FLJ13910 | NM_022780.2 | 1079 | gtgctggtatcactctatatttg | 384185 | - | see also 8471 line |
| 48420 | FLJ13910 | NM_022780.2 | 971 | ggcgctgccagctttaattaaca | 384181 | - | see also 8471 line |

Figure 46

| 48421 | FLJ13912 | NM_022770.2 | 501 | cagactttatcggacgtttcg | 384027 | - | see also 21437 line |
|---|---|---|---|---|---|---|---|
| 48422 | FLJ13912 | NM_022770.2 | 680 | ttccaacctcgttcagaattaca | 384029 | - | see also 21437 line |
| 48423 | FLJ13912 | NM_022770.2 | 203 | gcctcgcctggcgcttcttcc | 384024 | - | see also 21437 line |
| 48424 | FLJ13941 | NM_024848.1 | 660 | agctacgaggatcgtgatcttg | 391423 | - | see also 21438 line |
| 48425 | FLJ13941 | NM_024848.1 | 95 | acggttatggtgtcacgtatac | 391416 | - | see also 21438 line |
| 48426 | FLJ13946 | NM_152275.2 | 755 | ctctacaaggaggacagtatga | 415288 | - | see also 21439 line |
| 48427 | FLJ13946 | NM_152275.2 | 2205 | cagatgagtccagacaattgaaa | 415293 | - | see also 21439 line |
| 48428 | FLJ13955 | NM_024759.1 | 790 | cggatggcagttcctgatctatg | 390149 | - | see also 8739 line |
| 48429 | FLJ13955 | NM_024759.1 | 1088 | gtggtgccagttaatcatattt | 390156 | - | see also 8739 line |
| 48430 | FLJ13962 | NM_024862.1 | 584 | gggaaatttcgtaatcgtaatgg | 391552 | - | see also 8794 line |
| 48431 | FLJ13984 | NM_024770.1 | 611 | aactagtgctacaaatcgtttct | 390349 | - | see also 21441 line |
| 48432 | FLJ13984 | NM_024770.1 | 504 | aagttttcaaggatcgtaattg | 390346 | - | see also 21441 line |
| 48433 | FLJ14001 | NM_024677.3 | 481 | cgggcgaactggagtttcgaac | 389013 | - | see also 21442 line |
| 48434 | FLJ14001 | NM_024677.3 | 593 | tccggactcgtttatgtcatgg | 389017 | - | see also 21442 line |
| 48435 | FLJ14001 | NM_024677.3 | 482 | gggcgaactggagtttcgaacg | 389014 | - | see also 21442 line |
| 48436 | FLJ14009 | NM_024760.1 | 565 | gagcaactcggccggatttgc | 390161 | - | see also 21443 line |
| 48437 | FLJ14009 | NM_024760.1 | 806 | ctcggtttcacagtccatacc | 390163 | - | see also 21443 line |
| 48438 | FLJ14009 | NM_024760.1 | 1343 | atcctgatggagtcaagagtatc | 390166 | - | see also 21443 line |
| 48439 | FLJ14054 | NM_024563.2 | 641 | tacagaccctactataattcttc | 387318 | - | see also 21445 line |
| 48440 | FLJ14054 | NM_024563.2 | 778 | tccctattcacaggcttctaaaa | 387319 | - | see also 21445 line |
| 48441 | FLJ14075 | NM_024894.1 | 335 | ctggggttcgatgttatgacacc | 391726 | - | see also 21446 line |
| 48442 | FLJ14075 | NM_024894.1 | 333 | acctcggttcgatgttatgaca | 391725 | - | see also 21446 line |
| 48443 | FLJ14075 | NM_024894.1 | 1397 | ttctcaccgatgatcgatttaaa | 391750 | - | see also 21446 line |
| 48444 | FLJ14084 | NM_021637.1 | 135 | atccccagaaccgtaactattg | 380943 | - | see also 21447 line |
| 48445 | FLJ14084 | NM_021637.1 | 448 | ctggtcggtgatcctctcaaacg | 380949 | - | see also 21447 line |
| 48446 | FLJ14103 | NM_024689.1 | 231 | gactttcctcggttctgataaat | 389306 | - | see also 8696 line |
| 48447 | FLJ14103 | NM_024689.1 | 345 | tcccgattccttgctttcttatc | 389310 | - | see also 8696 line |
| 48448 | FLJ14117 | NM_022777.1 | 510 | tccggatcgaattcataaagtat | 384122 | - | - |
| 48449 | FLJ14129 | NM_030895.1 | 638 | gccggtcacttctgagaaaact | 395375 | - | - |
| 48450 | FLJ14146 | NM_024709.2 | 396 | gtcggaaggtcatcatcaaagg | 389488 | - | see also 21450 line |
| 48451 | FLJ14166 | NM_024565.4 | 1296 | cccgtgcaggacctatgcttgg | 387328 | - | see also 21451 line |
| 48452 | FLJ14166 | NM_024565.4 | 1108 | cacctcagcacgtgtattgaaat | 387326 | - | see also 21451 line |
| 48453 | FLJ14166 | NM_024565.4 | 1487 | gcctcgccaccacctatggaagc | 387330 | - | see also 21451 line |
| 48454 | FLJ14297 | NM_024903.1 | 339 | cacgaagtagaggaactcatacc | 391889 | - | - |
| 48455 | FLJ14299 | NM_025069.1 | 1890 | cccacagccggacctactattc | 393469 | - | see also 21452 line |
| 48456 | FLJ14299 | NM_025069.1 | 1656 | ctgccgggagccgagaaacttct | 393467 | - | see also 21452 line |

Figure 46

| | | | | | | | | | |
|---|---|---|---|---|---|---|---|---|---|
| 48457 | FLJ14345 | NM_024733.2 | 655 | ctcacggctaacaagtcataaga | 389741 | - | transcription_regulator | - | - |
| 48458 | FLJ14345 | NM_024733.2 | 289 | agcgttctacacggtgaagttgg | 389737 | - | see also 48457 line | | |
| 48459 | FLJ14345 | NM_024733.2 | 648 | gtcaaaactcacggctaacaagt | 389739 | - | see also 48457 line | | |
| 48460 | FLJ14351 | NM_024732.1 | 336 | atgatgagactccctttttatgc | 389728 | - | see also 21453 line | | |
| 48461 | FLJ14351 | NM_024732.1 | 600 | ggcccactggctgcaacaagtag | 389736 | - | see also 21453 line | | |
| 48462 | FLJ14351 | NM_024732.1 | 389 | ttcctggtggccagacttctagc | 389730 | - | see also 21453 line | | |
| 48463 | FLJ14397 | NM_032779.2 | 507 | gtcggctgtgcgactcatgaaga | 401447 | - | see also 21454 line | | |
| 48464 | FLJ14397 | NM_032779.2 | 2144 | aggaggtccagaccacgaaattg | 401454 | - | see also 21454 line | | |
| 48465 | FLJ14399 | NM_032780.2 | 865 | cccggcacccatctctgatatca | 401463 | - | see also 21455 line | | |
| 48466 | FLJ14399 | NM_032780.2 | 1094 | cccagtgctggggctatatctatc | 401467 | - | see also 21455 line | | |
| 48467 | FLJ14399 | NM_032780.2 | 887 | aagtgactccaacaacctaaaac | 401465 | - | see also 21455 line | | |
| 48468 | FLJ14464 | NM_032789.1 | 1010 | tgcagggtagagggattatgaca | 401515 | - | see also 21456 line | | |
| 48469 | FLJ14464 | NM_032789.1 | 1496 | cacttgaaggaccggatatgact | 401518 | - | see also 21456 line | | |
| 48470 | FLJ14466 | NM_032790.2 | 574 | acctgtttgcgctcatgatcagc | 401519 | - | - | - | - |
| 48471 | FLJ14466 | NM_032790.2 | 904 | tgcccttcggcctgatctttatc | 401520 | - | see also 48470 line | | |
| 48472 | FLJ14503 | NM_152780.2 | 673 | ttcacccactcgaaacattgaga | 421081 | - | see also 21457 line | | |
| 48473 | FLJ14503 | NM_152780.2 | 2138 | agcctggacgactgtaacaaaaa | 421098 | - | see also 21457 line | | |
| 48474 | FLJ14525 | NM_032800.1 | 96 | agcgaaagcgcttcacttacttc | 401545 | - | see also 21458 line | | |
| 48475 | FLJ14525 | NM_032800.1 | 341 | ttcggggacgaggatctaacttg | 401546 | - | see also 21458 line | | |
| 48476 | FLJ14564 | NM_032550.1 | 1153 | cccggatgcccagcgctttaact | 400653 | - | see also 21459 line | | |
| 48477 | FLJ14564 | NM_032550.1 | 1706 | gtgagtgcggccaaaaactctct | 400660 | - | see also 21459 line | | |
| 48478 | FLJ14566 | NM_032806.4 | 1143 | ttgcacggttcatgacagaaaag | 401582 | - | see also 21460 line | | |
| 48479 | FLJ14566 | NM_032806.4 | 1190 | cccctaggcgaggagtacattct | 401584 | - | see also 21460 line | | |
| 48480 | FLJ14566 | NM_032806.4 | 1137 | ggcagtttgcacggttcatgaca | 401581 | - | see also 21460 line | | |
| 48481 | FLJ14594 | NM_032808.2 | 895 | ggccgtccgccacctagtctatc | 401593 | - | see also 21461 line | | |
| 48482 | FLJ14594 | NM_032808.2 | 1201 | ccgctggcggctcaacttcaacc | 401594 | - | see also 21461 line | | |
| 48483 | FLJ14624 | NM_032813.1 | 390 | agcgactggagggtaattgcact | 401608 | - | see also 21462 line | | |
| 48484 | FLJ14624 | NM_032813.1 | 271 | tgctggtgagggccgtaaactac | 401602 | - | see also 21462 line | | |
| 48485 | FLJ14639 | NM_032815.2 | 1122 | ctcgagattccctctaaagacc | 401654 | - | see also 21463 line | | |
| 48486 | FLJ14639 | NM_032815.2 | 1120 | gtctcgagattccctctaaaga | 401653 | - | see also 21463 line | | |
| 48487 | FLJ14640 | NM_032816.2 | 272 | gactgggcggacggttgctattc | 401658 | - | see also 21464 line | | |
| 48488 | FLJ14640 | NM_032816.2 | 250 | cagccattctggcgacaacattg | 401657 | - | see also 21464 line | | |
| 48489 | FLJ14641 | NM_032817.1 | 763 | ggcctgtctgctgtgctatttat | 401677 | - | see also 21465 line | | |
| 48490 | FLJ14641 | NM_032817.1 | 766 | ctgtctgctgtgctatttatttg | 401678 | - | see also 21465 line | | |
| 48491 | FLJ14642 | NM_032818.1 | 148 | ggctggtggccacgtacttttg | 401680 | - | see also 21466 line | | |
| 48492 | FLJ14668 | NM_032822.1 | 450 | aggctctcttatcaattggaaaa | 401685 | - | see also 21467 line | | |

Figure 46

| | | | | | | | |
|---|---|---|---|---|---|---|---|
| 48493 | FLJ14668 | NM_032822.1 | 451 | ggctctcttatcaattggaaat | 401686 | - | see also 21467 line |
| 48494 | FLJ14681 | NM_032824.1 | 1639 | cgctgggttgacgatgcatttg | 401720 | - | see also 21468 line |
| 48495 | FLJ14681 | NM_032824.1 | 1638 | acgctgggttgacgatgcattt | 401719 | - | see also 21468 line |
| 48496 | FLJ14681 | NM_032824.1 | 1188 | gtcaacccaaggcttattgatat | 401706 | - | see also 21468 line |
| 48497 | FLJ14721 | NM_032829.1 | 1503 | tccgcctaccgtctacagataa | 401747 | - | see also 21469 line |
| 48498 | FLJ14721 | NM_032829.1 | 1181 | ttcaccgtaggccagtactttgc | 401744 | - | see also 21469 line |
| 48499 | FLJ14721 | NM_032829.1 | 1383 | tgcagtcactggagtatctcatc | 401745 | - | see also 21469 line |
| 48500 | FLJ14803 | NM_032842.1 | 742 | accgacctacgaactttggatac | 401947 | - | see also 9305 line |
| 48501 | FLJ14803 | NM_032842.1 | 1218 | cacaatcgttcagtatctagacc | 401965 | - | see also 9305 line |
| 48502 | FLJ14816 | NM_032845.1 | 85 | gacgattccggtcttccttagc | 401979 | - | see also 21472 line |
| 48503 | FLJ14816 | NM_032845.1 | 131 | tccccatgggtctcttgatgagt | 401980 | - | see also 21472 line |
| 48504 | FLJ14816 | NM_032845.1 | 412 | cacgagttggctgtcagttttat | 401983 | - | see also 21472 line |
| 48505 | FLJ14825 | NM_032847.1 | 220 | ggcctacagacgacaagagtatc | 401987 | - | see also 21473 line |
| 48506 | FLJ14825 | NM_032847.1 | 221 | gcctacagacgacaagagtatca | 401988 | - | see also 21473 line |
| 48507 | FLJ14825 | NM_032847.1 | 870 | ctcagcaaacatcgtttgcttg | 402000 | - | see also 21473 line |
| 48508 | FLJ14827 | NM_032848.1 | 778 | agccacacccgtcttactgtga | 402002 | - | see also 21474 line |
| 48509 | FLJ14827 | NM_032848.1 | 998 | cggcagactccagaagttatct | 402006 | - | see also 21474 line |
| 48510 | FLJ14834 | NM_032849.2 | 716 | aggacgtacgcgtttcttgtaaa | 402013 | - | see also 9311 line |
| 48511 | FLJ14834 | NM_032849.2 | 737 | aacacgaggcaccccaagataag | 402015 | - | see also 9311 line |
| 48512 | FLJ14871 | NM_032854.1 | 479 | gtcctattgccctttacgatcc | 402079 | - | see also 21476 line |
| 48513 | FLJ14871 | NM_032854.1 | 475 | cgggtcctattgcccttttacg | 402078 | - | see also 21476 line |
| 48514 | FLJ14888 | NM_032856.1 | 84 | cactcgagtccttgaatggattg | 402082 | - | see also 21477 line |
| 48515 | FLJ14888 | NM_032856.1 | 41 | tgcgcttgtaccaggatttctat | 402081 | - | see also 21477 line |
| 48516 | FLJ14904 | NM_032858.1 | 87 | gccagccggaatgcttactattt | 402096 | - | see also 9318 line |
| 48517 | FLJ14904 | NM_032858.1 | 89 | cagccggaatgcttactatttct | 402097 | - | see also 9318 line |
| 48518 | FLJ14936 | NM_032284.2 | 673 | gtctccgaaaggtctaagaaga | 399347 | - | - |
| 48519 | FLJ14936 | NM_032284.2 | 675 | ctcccgaaaggtctaagaagagc | 399348 | - | see also 48518 line |
| 48520 | FLJ14957 | NM_032866.2 | 1574 | gccaccgctacgctgatgttaca | 402217 | - | see also 21478 line |
| 48521 | FLJ14957 | NM_032866.2 | 1046 | gacaacgtcaatgtcatgaaaa | 402200 | - | see also 21478 line |
| 48522 | FLJ14966 | NM_032867.1 | 1599 | gacaaactcgtcgattccttaga | 402266 | - | see also 21479 line |
| 48523 | FLJ14966 | NM_032867.1 | 1568 | caccgagctgatgcaagtgattg | 402265 | - | see also 21479 line |
| 48524 | FLJ14981 | NM_032868.2 | 1332 | cacctacctgacaagcttaaga | 402274 | - | see also 21480 line |
| 48525 | FLJ14981 | NM_032868.2 | 1337 | acctcgacaagcttaagatctcc | 402275 | - | see also 21480 line |
| 48526 | FLJ16030 | NM_198494.1 | 786 | ttcacaggagcgcttatatcatg | 438578 | - | see also 21481 line |
| 48527 | FLJ16030 | NM_198494.1 | 1034 | cacctacaaagcggaacacatac | 438582 | - | see also 21481 line |
| 48528 | FLJ20003 | NM_017615.1 | 338 | tactgaatgccggtgacaaatta | 359589 | - | see also 21482 line |
| 48529 | FLJ20003 | NM_017615.1 | 345 | tgccggtgacaaattaacagagg | 359591 | - | see also 21482 line |

Figure 46

| | | | | | | | |
|---|---|---|---|---|---|---|---|
| 48530 | FLJ20006 | NM_017618.1 | 1051 | cagcgactgaagggttaattaat | 359619 | - | see also 21483 line |
| 48531 | FLJ20006 | NM_017618.1 | 997 | ggcagaacgcacccataacttgg | 359615 | - | see also 21483 line |
| 48532 | FLJ20010 | NM_019021.1 | 497 | tgcaacagcgtttgattgcatta | 374505 | - | see also 21484 line |
| 48533 | FLJ20010 | NM_019021.1 | 313 | aggcctgaagcgattcatctagg | 374504 | - | see also 21484 line |
| 48534 | FLJ20032 | NM_017628.2 | 2788 | agcgagttcgagactcataatgt | 359709 | - | see also 21486 line |
| 48535 | FLJ20032 | NM_017628.2 | 2843 | atcgtagaaattccccttatagt | 359710 | - | see also 21486 line |
| 48536 | FLJ20045 | NM_017638.1 | 702 | ctctcctatctggaattaag | 359870 | - | see also 7062 line |
| 48537 | FLJ20045 | NM_017638.1 | 318 | caccgccttcgtgtttgtgtga | 359864 | - | see also 7062 line |
| 48538 | FLJ20045 | NM_017638.1 | 270 | tacctgttgcagggaagtgtttt | 359862 | - | see also 7062 line |
| 48539 | FLJ20051 | NM_019087.1 | 253 | cagtgaaagccccgataacgtcg | 375119 | - | see also 7698 line |
| 48540 | FLJ20054 | NM_019049.1 | 200 | ctgcttacggacagcatataaa | 374740 | - | see also 21489 line |
| 48541 | FLJ20054 | NM_019049.1 | 1104 | tggactgtccatgaagtagtgt | 374767 | - | see also 21489 line |
| 48542 | FLJ20071 | NM_017653.2 | 383 | cagaatcggcgatcttcctaaaa | 360250 | - | see also 7082 line |
| 48543 | FLJ20071 | NM_017653.2 | 387 | atcgggcgatcttcctaaaaatga | 360251 | - | see also 7082 line |
| 48544 | FLJ20071 | NM_017653.2 | 1718 | gactaggacaagagacaagtacc | 360293 | - | see also 7082 line |
| 48545 | FLJ20073 | NM_017654.2 | 3364 | cacaagacacgccgatgaacatg | 360390 | - | see also 21491 line |
| 48546 | FLJ20073 | NM_017654.2 | 3806 | aagtcaaaaggcggtatgatac | 360400 | - | see also 21491 line |
| 48547 | FLJ20084 | NM_017659.1 | 556 | cccggatcgacccccttttgtagg | 360451 | - | see also 7086 line |
| 48548 | FLJ20084 | NM_017659.1 | 167 | cggcgttctacaccatttggagc | 360447 | - | see also 7086 line |
| 48549 | FLJ20097 | NM_024553.2 | 674 | aacagatgtacgttaagtgaaa | 387190 | - | see also 8641 line |
| 48550 | FLJ20097 | NM_024553.2 | 676 | cagatgtacggttaagtgaaatg | 387191 | - | see also 8641 line |
| 48551 | FLJ20136 | NM_017684.2 | 2684 | ttccgatacgtgatacactacc | 360666 | - | see also 21494 line |
| 48552 | FLJ20136 | NM_017684.2 | 2026 | acctggacgcgattgtatataatg | 360639 | - | see also 21494 line |
| 48553 | FLJ20136 | NM_017684.2 | 3083 | ttcaaccttcacgaatagttac | 360682 | - | see also 21494 line |
| 48554 | FLJ20151 | NM_017689.1 | 385 | tcttcggatccgcactcattac | 360830 | - | see also 21495 line |
| 48555 | FLJ20151 | NM_017689.1 | 33 | ttggccttggtccaaaattgtca | 360822 | - | see also 21495 line |
| 48556 | FLJ20151 | NM_017689.1 | 39 | ttggtccaaaattgtcaatcaga | 360823 | - | see also 21495 line |
| 48557 | FLJ20152 | NM_019000.2 | 675 | cacgttgctgccaaagagttat | 374112 | - | see also 7652 line |
| 48558 | FLJ20152 | NM_019000.2 | 719 | cagaggtatcctggactgataat | 374116 | - | see also 7652 line |
| 48559 | FLJ20156 | NM_017691.1 | 1705 | agcccaactccgtcgtattgaac | 360883 | - | see also 7113 line |
| 48560 | FLJ20156 | NM_017691.1 | 380 | tcgtcattcccgtagactttt | 360836 | - | see also 7113 line |
| 48561 | FLJ20156 | NM_017691.1 | 642 | atctacagaagttaatatcgttg | 360852 | - | see also 7113 line |
| 48562 | FLJ20160 | NM_017694.2 | 1612 | tgcaatacggtcgtctatattta | 360998 | - | see also 7116 line |
| 48563 | FLJ20174 | NM_017699.1 | 846 | ctcagtacaaactgctagttacc | 361020 | - | see also 21499 line |
| 48564 | FLJ20174 | NM_017699.1 | 511 | cgccgatttcgatcatgtctaca | 361012 | - | see also 21499 line |
| 48565 | FLJ20174 | NM_017699.1 | 2401 | tagccgacctctatatatggata | 361059 | - | see also 21499 line |
| 48566 | FLJ20184 | NM_017700.1 | 631 | tggaaccggccatgcaagtaatt | 361073 | - | see also 21500 line |

Figure 46

| | | | | | | | |
|---|---|---|---|---|---|---|---|
| 48567 | FLJ20184 | NM_017700.1 | 541 | tggatgtggatagcttgtttagc | 361069 | - | see also 21500 line |
| 48568 | FLJ20186 | NM_017702.1 | 534 | tccttgagcaccgcttttacaag | 361076 | - | see also 21501 line |
| 48569 | FLJ20195 | NM_017706.3 | 1076 | gactaccgtcgtcgcaaaaaaa | 361100 | - | see also 21502 line |
| 48570 | FLJ20195 | NM_017706.3 | 1132 | ttggagcaccgatgacttcttcg | 361101 | - | see also 21502 line |
| 48571 | FLJ20200 | NM_017708.1 | 888 | ggcaccgtgcgggagaagtttgt | 361103 | - | see also 21503 line |
| 48572 | FLJ20200 | NM_017708.1 | 887 | cggcaccgtgcgggagaagtttg | 361102 | - | see also 21503 line |
| 48573 | FLJ20202 | NM_017709.2 | 710 | gactccattggcgtcagtttga | 361118 | - | see also 21504 line |
| 48574 | FLJ20202 | NM_017709.2 | 1212 | tgcgtgtcggcggaacaaaac | 361122 | - | see also 21504 line |
| 48575 | FLJ20202 | NM_017709.2 | 427 | tggccacgttttggtcaaagaca | 361113 | - | see also 21504 line |
| 48576 | FLJ20211 | NM_017713.1 | 569 | ggcggtggctgtgctagacttca | 361129 | - | see also 21505 line |
| 48577 | FLJ20211 | NM_017713.1 | 608 | caccgtgtctgggaactaaagg | 361131 | - | see also 21505 line |
| 48578 | FLJ20232 | NM_019008.3 | 1322 | gtgcagtatgagcgtgacaaaca | 374457 | - | see also 21506 line |
| 48579 | FLJ20232 | NM_019008.3 | 1115 | ttcctggtcgtcgtgagaatcc | 374448 | - | see also 21506 line |
| 48580 | FLJ20241 | NM_017721.2 | 1214 | cgcgtggctaagagctttgatgc | 361150 | - | see also 21507 line |
| 48581 | FLJ20241 | NM_017721.2 | 1540 | agccgtggattgctgcgtgaattgc | 361153 | - | see also 21507 line |
| 48582 | FLJ20259 | NM_017730.2 | 1457 | taccagaatacgctcaaactgt | 361187 | - | see also 7124 line |
| 48583 | FLJ20259 | NM_017730.2 | 2236 | cacgagtatccggtacttgaagg | 361205 | - | see also 7124 line |
| 48584 | FLJ20265 | NM_017733.2 | 1540 | ttcggccgttcacgtcattgtgt | 361238 | - | see also 21509 line |
| 48585 | FLJ20265 | NM_017733.2 | 1398 | cccagtagacatctattcgatg | 361237 | - | see also 21509 line |
| 48586 | FLJ20272 | NM_017735.2 | 1830 | ttcgaacgtcggttaagattaa | 361358 | - | see also 7125 line |
| 48587 | FLJ20272 | NM_017735.2 | 2559 | ctcaatttacgggggcttgttatc | 361378 | - | see also 7125 line |
| 48588 | FLJ20272 | NM_017735.2 | 1991 | ttcctatatccgattaaaaacaaa | 361368 | - | see also 7125 line |
| 48589 | FLJ20274 | NM_017736.1 | 944 | ctggaatctcggacaaatcaga | 361406 | - | see also 21511 line |
| 48590 | FLJ20274 | NM_017736.1 | 740 | gtgtgcaccctcaattcagaaaa | 361397 | - | see also 21511 line |
| 48591 | FLJ20275 | NM_017737.1 | 991 | taagttccacgtcatacataga | 361429 | - | see also 7126 line |
| 48592 | FLJ20275 | NM_017737.1 | 55 | atggacgaacgaaggactattaa | 361411 | - | see also 7126 line |
| 48593 | FLJ20280 | NM_017741.1 | 635 | agctaagccgaacattgagtaga | 361435 | - | see also 21513 line |
| 48594 | FLJ20280 | NM_017741.1 | 752 | ctcgtactgaacccatcaacact | 361446 | - | see also 21513 line |
| 48595 | FLJ20280 | NM_017741.1 | 662 | ctccataacctgaataccctaaaa | 361437 | - | see also 21513 line |
| 48596 | FLJ20287 | NM_017746.1 | 1838 | ctctacacagcttatcgatatca | 361630 | - | see also 7139 line |
| 48597 | FLJ20287 | NM_017746.1 | 661 | gccgtagcagcatattgcttaag | 361596 | - | see also 7139 line |
| 48598 | FLJ20291 | NM_017748.3 | 724 | cacagaagctcgagtagttgatcg | 361665 | - | see also 21515 line |
| 48599 | FLJ20291 | NM_017748.3 | 1361 | ggggtgaagcggaatatctactct | 361668 | - | see also 21515 line |
| 48600 | FLJ20297 | NM_017751.1 | 630 | tccctccacgtccgtacttcaga | 361670 | - | see also 21516 line |
| 48601 | FLJ20297 | NM_017751.1 | 927 | cactattccttggagatgatca | 361674 | - | see also 21516 line |
| 48602 | FLJ20297 | NM_017751.1 | 2130 | agcttggtccgcacactctttag | 361675 | - | see also 21516 line |
| 48603 | FLJ20300 | NM_017753.1 | 284 | cactcaacgaagttattccatca | 361676 | - | see also 7140 line |

Figure 46

| | | | | | | | |
|---|---|---|---|---|---|---|---|
| 48604 | FLJ20303 | NM_017755.4 | 586 | tgcggcctcatcataagatctta | 361786 | - | see also 7144 line |
| 48605 | FLJ20303 | NM_017755.4 | 654 | atgctacatgccgacatgaatgt | 361792 | - | see also 7144 line |
| 48606 | FLJ20308 | NM_017758.2 | 320 | gacgtcccggacaactataagg | 361890 | - | see also 21519 line |
| 48607 | FLJ20308 | NM_017758.2 | 1129 | tagatgcacccggttggaaaca | 361896 | - | see also 21519 line |
| 48608 | FLJ20309 | NM_017759.2 | 2950 | aacatgtactctggtgataata | 361949 | - | see also 21520 line |
| 48609 | FLJ20309 | NM_017759.2 | 1592 | aagaaatgccggcatacgtttag | 361918 | - | see also 21520 line |
| 48610 | FLJ20311 | NM_017760.4 | 2108 | atcgctgcaagatcccctattc | 361993 | - | see also 7147 line |
| 48611 | FLJ20311 | NM_017760.4 | 672 | ttggcgtatccatcaagctttat | 361957 | - | see also 7147 line |
| 48612 | FLJ20313 | NM_017762.1 | 1211 | tactcgatggttagaatatgtaa | 362054 | - | see also 21522 line |
| 48613 | FLJ20313 | NM_017762.1 | 832 | acgagggttacatgatatccact | 362042 | - | see also 21522 line |
| 48614 | FLJ20315 | NM_017763.1 | 1665 | ctccctaagcagtgactttgacc | 362075 | - | see also 21523 line |
| 48615 | FLJ20315 | NM_017763.1 | 312 | cagagtgatcccctgaaaatgg | 362072 | - | see also 21523 line |
| 48616 | FLJ20320 | NM_017765.1 | 612 | gacgctacttttactacaagt | 362084 | - | see also 7148 line |
| 48617 | FLJ20320 | NM_017765.1 | 604 | gtgatgctgacgctgacttta | 362082 | - | see also 7148 line |
| 48618 | FLJ20323 | NM_019005.1 | 867 | gtgtgacgtagaccccatattc | 374387 | - | see also 7656 line |
| 48619 | FLJ20343 | NM_017775.2 | 683 | agcgagccaagttgagcattatg | 362186 | - | see also 21525 line |
| 48620 | FLJ20343 | NM_017775.2 | 752 | gtctgcctatcagactgataac | 362193 | - | see also 21525 line |
| 48621 | FLJ20343 | NM_017775.2 | 746 | ctcttcgtctgcctatcagact | 362192 | - | see also 21525 line |
| 48622 | FLJ20345 | NM_017777.1 | 318 | caggaacaaccacgtcattaaca | 362216 | - | see also 21526 line |
| 48623 | FLJ20345 | NM_017777.1 | 186 | agccagcgaggtgccttcattct | 362212 | - | see also 21526 line |
| 48624 | FLJ20356 | NM_018986.2 | 483 | accggatcgtggtgacgtttaag | 374070 | - | see also 21527 line |
| 48625 | FLJ20356 | NM_018986.2 | 429 | ccgcgctgggggaattatca | 374068 | - | see also 21527 line |
| 48626 | FLJ20356 | NM_018986.2 | 1167 | gccccgtgtccgagttggaaagt | 374078 | - | see also 21527 line |
| 48627 | FLJ20360 | NM_017782.2 | 3673 | tacgccaacttctctataacaa | 362324 | - | see also 21528 line |
| 48628 | FLJ20360 | NM_017782.2 | 4398 | caccaatttgcacgtcaaactaa | 362347 | - | see also 21528 line |
| 48629 | FLJ20364 | NM_017785.2 | 579 | cagaggaactgcgcgtaatgtct | 362380 | - | see also 21529 line |
| 48630 | FLJ20364 | NM_017785.2 | 1342 | gagccagcgggctctagatattg | 362399 | - | see also 21529 line |
| 48631 | FLJ20373 | NM_017792.1 | 337 | cactcaagctccgtaaggatat | 362443 | - | see also 21530 line |
| 48632 | FLJ20373 | NM_017792.1 | 420 | gtcgagttctgagrtggaaataac | 362445 | - | see also 21530 line |
| 48633 | FLJ20397 | NM_017802.2 | 1294 | agctcgtcgggacgtttgtcagc | 362630 | - | see also 21531 line |
| 48634 | FLJ20397 | NM_017802.2 | 750 | gacgacgtgctttcccatttgc | 362629 | - | see also 21531 line |
| 48635 | FLJ20420 | NM_017812.1 | 210 | ggcatccggcctttcggaaaatgt | 362656 | - | see also 21532 line |
| 48636 | FLJ20420 | NM_017812.1 | 267 | tcgaagttcagcggtattctgg | 362658 | - | see also 21532 line |
| 48637 | FLJ20422 | NM_017814.1 | 132 | cggcagttgttcgatacaagc | 362667 | - | see also 21533 line |
| 48638 | FLJ20422 | NM_017814.1 | 488 | cagtgacacgctgctgctacttcagc | 362670 | - | see also 21533 line |
| 48639 | FLJ20432 | NM_017819.1 | 1267 | tgcaattcgttccaagagaaaa | 362747 | - | see also 21534 line |
| 48640 | FLJ20432 | NM_017819.1 | 1253 | ttggcaagaggctctgcaattcg | 362746 | - | see also 21534 line |

Figure 46

| | | | | | | | |
|---|---|---|---|---|---|---|---|
| 48641 | FLJ20436 | NM_017822.1 | 583 | aagcgccgatacttacataatcg | 362754 | - | see also 21535 line |
| 48642 | FLJ20436 | NM_017822.1 | 580 | aagaagcgccgatacttacataa | 362753 | - | see also 21535 line |
| 48643 | FLJ20445 | NM_017824.2 | 796 | gtggcgcaaatactcgaataaac | 362768 | - | see also 21536 line |
| 48644 | FLJ20445 | NM_017824.2 | 606 | tggtcggctctatctattggaca | 362763 | - | see also 21536 line |
| 48645 | FLJ20449 | NM_017826.1 | 1238 | ggccagacgtgcactacacatcc | 362816 | - | see also 21537 line |
| 48646 | FLJ20449 | NM_017826.1 | 1025 | tccgcctcagagaatgctattgg | 362808 | - | see also 21537 line |
| 48647 | FLJ20449 | NM_017826.1 | 788 | gccgttatggcccagattacaga | 362805 | - | see also 21538 line |
| 48648 | FLJ20452 | NM_017828.2 | 381 | gtctgcagcctacgcatgaatag | 362823 | - | see also 21538 line |
| 48649 | FLJ20452 | NM_017828.2 | 536 | gtccctccagcagacaagttcc | 362825 | - | see also 21538 line |
| 48650 | FLJ20457 | NM_017832.2 | 342 | gccttcgctcgtggatcgatact | 362887 | - | see also 21539 line |
| 48651 | FLJ20457 | NM_017832.2 | 347 | cgctcgtgatcgatacttcact | 362889 | - | see also 21539 line |
| 48652 | FLJ20457 | NM_017832.2 | 422 | tacagcactcaacgaatatgt | 362898 | - | see also 21539 line |
| 48653 | FLJ20464 | NM_017834.1 | 504 | tgctaccacgctagaagttgtaca | 362939 | - | see also 21540 line |
| 48654 | FLJ20464 | NM_017834.1 | 641 | cagcgaagtatgtgttccaaact | 362942 | - | see also 21540 line |
| 48655 | FLJ20477 | NM_017837.2 | 639 | gacccttacgggggttactgagt | 362949 | - | see also 21541 line |
| 48656 | FLJ20477 | NM_017837.2 | 682 | ttcggtagcatcactcaatttct | 362951 | - | see also 21541 line |
| 48657 | FLJ20487 | NM_017841.1 | 330 | acctctatgacggcctgattaac | 363007 | - | see also 7160 line |
| 48658 | FLJ20487 | NM_017841.1 | 344 | ctgattaacgagccctagtaatga | 363008 | - | see also 7160 line |
| 48659 | FLJ20489 | NM_017842.1 | 768 | gaggggcagcttagacctttcaa | 363012 | - | see also 21543 line |
| 48660 | FLJ20489 | NM_017842.1 | 770 | gggcagcttagacctttcaa | 363013 | - | see also 21543 line |
| 48661 | FLJ20502 | NM_017845.2 | 403 | gacaagattgctgcattacgaat | 363033 | - | see also 21544 line |
| 48662 | FLJ20502 | NM_017845.2 | 522 | gtccttggaagcagcggaataagg | 363035 | - | see also 21544 line |
| 48663 | FLJ20507 | NM_017849.1 | 680 | caccgtcattggctttctatt | 363124 | - | see also 21545 line |
| 48664 | FLJ20507 | NM_017849.1 | 484 | acccggacctgctgaaagattc | 363120 | - | see also 21545 line |
| 48665 | FLJ20507 | NM_017849.1 | 485 | cccggacctgctgaaagatttct | 363121 | - | see also 21545 line |
| 48666 | FLJ20508 | NM_017850.1 | 378 | ttcgggccttcccagatttaaaa | 363129 | - | see also 21546 line |
| 48667 | FLJ20508 | NM_017850.1 | 500 | agccatgtggagcgagtgtttca | 363131 | - | see also 21546 line |
| 48668 | FLJ20509 | NM_017851.1 | 785 | gacgagcgagaatcaaacatagt | 363147 | - | see also 21547 line |
| 48669 | FLJ20509 | NM_017851.1 | 779 | atcgcagacgagcgagaatcaaa | 363146 | - | see also 21547 line |
| 48670 | FLJ20513 | NM_017855.2 | 187 | aactagcagaacctgctatatca | 363158 | - | see also 21549 line |
| 48671 | FLJ20513 | NM_017855.2 | 364 | aaccagcacaaccaatgttttc | 363162 | - | see also 21549 line |
| 48672 | FLJ20514 | NM_017856.1 | 468 | ttcaatcttccatggtacttacc | 363166 | - | - |
| 48673 | FLJ20516 | NM_017858.1 | 452 | ttcgacttgatctcctatttta | 363178 | - | see also 7162 line |
| 48674 | FLJ20516 | NM_017858.1 | 436 | acctgtttaaaacgaattcgact | 363177 | - | see also 7162 line |
| 48675 | FLJ20516 | NM_017858.1 | 257 | ttccagcttaaggcatgtattt | 363171 | - | see also 7162 line |
| 48676 | FLJ20519 | NM_017860.3 | 347 | tagccggtcgtccgccattaacg | 363199 | - | see also 21551 line |
| 48677 | FLJ20519 | NM_017860.3 | 399 | gtgattaatgccggaaaggatag | 363202 | - | see also 21551 line |

Figure 46

| 48678 | FLJ20522 | NM_017861.1 | 572 | agcctcgattgtggtcaataacc | 363218 | - | see also 21552 line |
|---|---|---|---|---|---|---|---|
| 48679 | FLJ20522 | NM_017861.1 | 616 | accaagagttcccgattttgaaa | 363219 | - | see also 21552 line |
| 48680 | FLJ20530 | NM_017864.1 | 602 | tccgaccttataagtagagtacg | 363255 | - | see also 7164 line |
| 48681 | FLJ20530 | NM_017864.1 | 1577 | atcggccagacagagttgaatgc | 363283 | - | see also 7164 line |
| 48682 | FLJ20531 | NM_017865.2 | 1423 | gtgagatatgcgggtttacctgc | 363296 | - | see also 7167 line |
| 48683 | FLJ20531 | NM_017865.2 | 1184 | ctccaaccggcagtatttgaatc | 363294 | - | see also 7167 line |
| 48684 | FLJ20533 | NM_017866.3 | 275 | tcgagcgcagatccctgtttatt | 363300 | - | see also 21554 line |
| 48685 | FLJ20533 | NM_017866.3 | 592 | aggccacaacagacacttataaa | 363313 | - | see also 21554 line |
| 48686 | FLJ20534 | NM_017867.1 | 696 | ggcttggttgttccagtagataa | 363319 | - | see also 21555 line |
| 48687 | FLJ20542 | NM_017871.3 | 582 | atgaccccagaccgacacttagg | 363333 | - | see also 21556 line |
| 48688 | FLJ20542 | NM_017871.3 | 107 | ctccattgcgggcaagaatgtca | 363328 | - | see also 21556 line |
| 48689 | FLJ20546 | NM_017872.2 | 10 | ggcgcctgtaaagttaaggttca | 363341 | - | see also 21557 line |
| 48690 | FLJ20546 | NM_017872.2 | 338 | agcggaaaaccaattggtttaaa | 363344 | - | see also 21557 line |
| 48691 | FLJ20555 | NM_017877.2 | 141 | cggcaaaatgggcggacaatttc | 363358 | - | see also 21558 line |
| 48692 | FLJ20555 | NM_017877.2 | 432 | ggggtgcagtgatcatattcact | 363362 | - | see also 21558 line |
| 48693 | FLJ20580 | NM_017887.1 | 159 | ttcggacaagtggcagtacatcc | 363373 | - | see also 21559 line |
| 48694 | FLJ20580 | NM_017887.1 | 448 | gactggactgactatgatgaaaa | 363378 | - | see also 21559 line |
| 48695 | FLJ20605 | NM_017898.3 | 723 | agcgtatagattggttcaatttg | 363704 | - | see also 21560 line |
| 48696 | FLJ20605 | NM_017898.3 | 997 | atggcatgccccaggtgtatttt | 363714 | - | see also 21560 line |
| 48697 | FLJ20618 | NM_017903.2 | 813 | tggatttttgcccgagatgattc | 363752 | - | see also 21561 line |
| 48698 | FLJ20618 | NM_017903.2 | 703 | atgctccatccaaagtgaattat | 363750 | - | see also 21561 line |
| 48699 | FLJ20619 | NM_017904.1 | 50 | ggctgtggccgacgatctagacg | 363754 | - | - | - | - |
| 48700 | FLJ20619 | NM_017904.1 | 931 | atcgcctggcaaaaatcttctac | 363756 | - | see also 48699 line |
| 48701 | FLJ20619 | NM_017904.1 | 613 | aggagaaaaggggctggtatttc | 363755 | - | see also 48699 line |
| 48702 | FLJ20625 | NM_017907.1 | 446 | gtctccaggatagctgcttatgc | 363835 | - | see also 21562 line |
| 48703 | FLJ20625 | NM_017907.1 | 134 | ccccctaccaaagctctcaatgg | 363830 | - | see also 21562 line |
| 48704 | FLJ20635 | NM_017911.1 | 1098 | tgcacggaatcaaagttgtatcc | 363888 | - | see also 21563 line |
| 48705 | FLJ20635 | NM_017911.1 | 793 | ggcaagagggcacatgaagtacg | 363880 | - | see also 21563 line |
| 48706 | FLJ20641 | NM_017915.1 | 705 | gtggccacgtcttttattagaac | 363904 | - | see also 21564 line |
| 48707 | FLJ20641 | NM_017915.1 | 1742 | tggccaggcaaagttaactcagt | 363931 | - | see also 21564 line |
| 48708 | FLJ20647 | NM_017918.3 | 884 | cactcgacaggattatacttact | 363944 | - | see also 21565 line |
| 48709 | FLJ20647 | NM_017918.3 | 882 | gtcactcgacaggattatactta | 363943 | - | see also 21565 line |
| 48710 | FLJ20668 | NM_017923.1 | 604 | tacgtacagtgtaaagtctatgt | 364046 | - | see also 21566 line |
| 48711 | FLJ20668 | NM_017923.1 | 69 | aaccatgtattaccttaaccaag | 364033 | - | see also 21566 line |
| 48712 | FLJ20694 | NM_017928.1 | 392 | gcctcaccctgtgcaaatcatgc | 364266 | - | see also 21567 line |
| 48713 | FLJ20694 | NM_017928.1 | 480 | tggcaagaacacactgttgttgc | 364267 | - | see also 21567 line |
| 48714 | FLJ20699 | NM_017931.1 | 403 | ttccgaaagcctgtgaactatgg | 364281 | - | see also 21568 line |

Figure 46

| | | | | | | | |
|---|---|---|---|---|---|---|---|
| 48715 | FLJ20699 | NM_017931.1 | 772 | ctgatatgttggcttgtcataac | 364289 | - | see also 21568 line |
| 48716 | FLJ20701 | NM_017933.3 | 598 | cagtggctgcaaggttacctacc | 364296 | - | see also 21569 line |
| 48717 | FLJ20701 | NM_017933.3 | 372 | tagaagagtcgaggaacatgttct | 364292 | - | see also 21569 line |
| 48718 | FLJ20716 | NM_017938.2 | 494 | tacgctaaccggtgccattatgt | 364358 | - | see also 7179 line |
| 48719 | FLJ20716 | NM_017938.2 | 334 | tggatcgttccttggaatcattg | 364352 | - | see also 7179 line |
| 48720 | FLJ20718 | NM_017939.1 | 1818 | gccgaaaagacctcgattcaaat | 364414 | - | see also 21571 line |
| 48721 | FLJ20718 | NM_017939.1 | 1959 | ttgcgaagatttattgcttatca | 364421 | - | see also 21571 line |
| 48722 | FLJ20729 | NM_017953.2 | 832 | cacgttgatcgatattcctgc | 364736 | - | see also 21572 line |
| 48723 | FLJ20729 | NM_017953.2 | 952 | atctcctaagtgattatcgatt | 364737 | - | see also 21572 line |
| 48724 | FLJ20758 | NM_017952.4 | 1539 | ggccaatcggctagaagtgattc | 364720 | - | see also 21573 line |
| 48725 | FLJ20758 | NM_017952.4 | 434 | aacctcatatacgtgtttaatg | 364677 | - | see also 21573 line |
| 48726 | FLJ20758 | NM_017952.4 | 1544 | atcggctagaagtgattcctaaa | 364721 | - | see also 21573 line |
| 48727 | FLJ20772 | NM_017956.1 | 1396 | tccccatgtggatcacatagtcc | 364867 | - | see also 21574 line |
| 48728 | FLJ20772 | NM_017956.1 | 196 | taccagcggatacagagaatatc | 364863 | - | see also 21574 line |
| 48729 | FLJ20811 | NM_019007.2 | 989 | agcaggccggattaaatttgtta | 374437 | - | see also 21575 line |
| 48730 | FLJ20811 | NM_019007.2 | 986 | tgcagcaggccggattaaatttg | 374436 | - | see also 21575 line |
| 48731 | FLJ20859 | NM_022734.1 | 850 | accgcactgaggtagttcaaacc | 383771 | - | see also 21576 line |
| 48732 | FLJ20859 | NM_022734.1 | 849 | gaccgcactgaggtagttcaaac | 383770 | - | see also 21576 line |
| 48733 | FLJ20859 | NM_022734.1 | 423 | gtgctacacgcactacgtaaaac | 383761 | - | see also 21576 line |
| 48734 | FLJ20896 | NM_024605.2 | 544 | ctgtgttaatgcgatccattc | 387806 | - | see also 8665 line |
| 48735 | FLJ20898 | NM_024600.1 | 881 | gtcccgaggcaccgtcaaactgc | 387738 | - | see also 21578 line |
| 48736 | FLJ20898 | NM_024600.1 | 1077 | ctcgtgactttctacagaattgg | 387740 | - | see also 21578 line |
| 48737 | FLJ20972 | NM_025030.1 | 445 | tccatgttagagcctgaattatc | 393164 | - | see also 21579 line |
| 48738 | FLJ20972 | NM_025030.1 | 70 | aagttaggggtggctcatgaaga | 393160 | - | see also 21579 line |
| 48739 | FLJ20989 | NM_023080.1 | 477 | tcggaaccctgcgcgcaaaaga | 385259 | - | see also 21580 line |
| 48740 | FLJ20989 | NM_023080.1 | 662 | gtctgcaggcctcgctctatatg | 385261 | - | see also 21580 line |
| 48741 | FLJ20989 | NM_023080.1 | 241 | aggcggcacagcgtcgaaaaaac | 385257 | - | see also 21580 line |
| 48742 | FLJ21047 | NM_024569.2 | 545 | tggcaaaacaggtgtcttaaaa | 387332 | - | signal transduction, kinase_related |
| 48743 | FLJ21047 | NM_024569.2 | 478 | ttcctggttcagataacgaact | 387331 | - | see also 48742 line |
| 48744 | FLJ21047 | NM_024569.2 | 546 | ggcaaaacaggtgtcttaaaat | 387333 | - | see also 48742 line |
| 48745 | FLJ21062 | NM_024788.2 | 2715 | agcactcctgccgattagtagg | 390724 | - | see also 21581 line |
| 48746 | FLJ21062 | NM_024788.2 | 2147 | cccatctattcggttattggatg | 390708 | - | see also 21581 line |
| 48747 | FLJ21069 | NM_024692.3 | 1085 | ggggatcgtgttgttattgcagg | 389343 | - | see also 8697 line |
| 48748 | FLJ21069 | NM_024692.3 | 2297 | gtgacctatcggggaattaatgg | 389378 | - | see also 8697 line |
| 48749 | FLJ21103 | NM_024556.1 | 421 | ctctcctgtaccagtaatgttc | 387229 | - | see also 21584 line |
| 48750 | FLJ21103 | NM_024556.1 | 878 | gaggctgtcaagcataaaatctga | 387239 | - | see also 21584 line |

Figure 46

| | | | | | | | |
|---|---|---|---|---|---|---|---|
| 48751 | FLJ21106 | NM_025097.1 | 1135 | aagtcgcaaccctcagtcatacc | 393595 | - | see also 8860 line |
| 48752 | FLJ21106 | NM_025097.1 | 935 | gacaagctaactaaccttaatct | 393589 | - | see also 8860 line |
| 48753 | FLJ21125 | NM_024627.4 | 688 | ggcacttgtggcgtaaacctgc | 388235 | - | see also 21586 line |
| 48754 | FLJ21125 | NM_024627.4 | 966 | gcccctgcctgacgattacgagc | 388239 | - | see also 21586 line |
| 48755 | FLJ21127 | NM_024549.3 | 1281 | gggtcggacccccagtattatt | 387169 | - | see also 21587 line |
| 48756 | FLJ21127 | NM_024549.3 | 1279 | gggggtccggaccccagtattat | 387167 | - | see also 21587 line |
| 48757 | FLJ21144 | NM_022774.1 | 372 | aaccataagcttacaaaactg | 384107 | - | see also 21588 line |
| 48758 | FLJ21144 | NM_022774.1 | 1057 | cccagttattgatatcttgaaga | 384118 | - | see also 21588 line |
| 48759 | FLJ21148 | NM_024860.1 | 712 | aacgccaatctagaatactctgc | 391517 | - | see also 21589 line |
| 48760 | FLJ21148 | NM_024860.1 | 703 | gccaatcacaacgccaatctaga | 391515 | - | see also 21589 line |
| 48761 | FLJ21159 | NM_024826.1 | 909 | aacgccgaatggcatacaattag | 391135 | - | see also 21590 line |
| 48762 | FLJ21159 | NM_024826.1 | 552 | atcaaacggtaacataaccaaag | 391130 | - | see also 21590 line |
| 48763 | FLJ21168 | NM_025073.1 | 579 | tagcccaattagagcttgaaaat | 393488 | - | see also 21591 line |
| 48764 | FLJ21168 | NM_025073.1 | 580 | agcccaattagagcttgaaaata | 393489 | - | see also 21591 line |
| 48765 | FLJ21174 | NM_024863.3 | 625 | ccgcaggtcgttgaaagacagg | 391576 | - | see also 21592 line |
| 48766 | FLJ21174 | NM_024863.3 | 623 | tccccgcaggtcgttgaaagaca | 391575 | - | see also 21592 line |
| 48767 | FLJ21195 | NM_022469.2 | 631 | cccaccttccgactcaagaaaa | 383037 | - | - |
| 48768 | FLJ21195 | NM_022469.2 | 633 | caccccttccgactcaagaaaatc | 383038 | - | see also 48767 line |
| 48769 | FLJ21308 | NM_024615.2 | 1338 | aagcaggtcttacctcagtaatc | 388006 | - | see also 8668 line |
| 48770 | FLJ21308 | NM_024615.2 | 1255 | aagtcgatagactattgactcg | 387999 | - | see also 8668 line |
| 48771 | FLJ21415 | NM_024738.1 | 507 | ctgctgcagcgcctatgagtact | 389766 | - | see also 21594 line |
| 48772 | FLJ21415 | NM_024738.1 | 618 | ggcagtcgaagatcacttgagt | 389767 | - | see also 21594 line |
| 48773 | FLJ21458 | NM_024850.1 | 573 | tccatcacgggatatgttgatag | 391429 | - | - |
| 48774 | FLJ21458 | NM_024850.1 | 578 | cacgggatatgttgatagagaca | 391430 | - | see also 48773 line |
| 48775 | FLJ21458 | NM_024850.1 | 483 | ctctatgggtgcaggattagttc | 391428 | - | see also 48773 line |
| 48776 | FLJ21477 | NM_025153.1 | 87 | agcgagtagtgtctctaacttg | 394097 | - | see also 21595 line |
| 48777 | FLJ21477 | NM_025153.1 | 363 | aagacctccgoggttacaaatcg | 394103 | - | see also 21595 line |
| 48778 | FLJ21511 | NM_025087.1 | 257 | gggaccgatgatctattacttc | 393517 | - | see also 8859 line |
| 48779 | FLJ21511 | NM_025087.1 | 253 | acctggaccgatgatcctattac | 393515 | - | see also 8859 line |
| 48780 | FLJ21603 | NM_024762.1 | 699 | tgcagtgagtccagtctcattaa | 390180 | - | see also 21597 line |
| 48781 | FLJ21603 | NM_024762.1 | 367 | gtgaggttgtcagaaattttt | 390178 | - | see also 21597 line |
| 48782 | FLJ21610 | NM_022751.1 | 2507 | gtcactacggttcattgtttgt | 383949 | - | see also 21598 line |
| 48783 | FLJ21610 | NM_022751.1 | 297 | gagattccggtacattatgcagg | 383904 | - | see also 21598 line |
| 48784 | FLJ21657 | NM_022483.2 | 959 | ttcaaaacatggagttcgtattg | 383272 | - | see also 21599 line |
| 48785 | FLJ21657 | NM_022483.2 | 907 | ctcacatcgttcattcgttatg | 383270 | - | see also 21599 line |
| 48786 | FLJ21742 | NM_032207.1 | 77 | gccgtcccatgccgtgatgtttt | 398668 | - | see also 9098 line |
| 48787 | FLJ21742 | NM_032207.1 | 76 | agccgtcccatgccgtgatgtttt | 398667 | - | see also 9098 line |

Figure 46

| | | | | | | | |
|---|---|---|---|---|---|---|---|
| 48788 | FLJ21749 | NM_025124.1 | 431 | ctggacccaacacccttgatcc | 393817 | - | see also 21601 line |
| 48789 | FLJ21749 | NM_025124.1 | 604 | gagtctatcacgtgatcttcatc | 393819 | - | see also 21601 line |
| 48790 | FLJ21777 | NM_032209.1 | 1061 | cacgccctgcaccctacgtaaat | 398702 | - | see also 21602 line |
| 48791 | FLJ21777 | NM_032209.1 | 806 | ctccgcattccgacattacatgc | 398701 | - | see also 21602 line |
| 48792 | FLJ21816 | NM_024675.2 | 968 | tactactcacgacctaaaaaaca | 388945 | - | see also 21603 line |
| 48793 | FLJ21816 | NM_024675.2 | 3641 | gtcggtacagactctcatttgc | 389003 | - | see also 21603 line |
| 48794 | FLJ21816 | NM_024675.2 | 2276 | tacggttgcgcctgatgataatg | 388976 | - | see also 21603 line |
| 48795 | FLJ21868 | NM_022769.1 | 456 | ccettagcaaccatcctcttcc | 384015 | - | see also 21604 line |
| 48796 | FLJ21868 | NM_022769.1 | 290 | atgaccacctgggtataagaag | 384011 | - | see also 21604 line |
| 48797 | FLJ21901 | NM_024622.2 | 2016 | ttcgtccattcagcgtattgaac | 388183 | - | see also 21606 line |
| 48798 | FLJ21901 | NM_024622.2 | 2071 | aacttgcgtgcaacatcttaatt | 388187 | - | see also 21606 line |
| 48799 | FLJ21901 | NM_024622.2 | 1595 | tcccgaattgaagccgttttacc | 388173 | - | see also 21606 line |
| 48800 | FLJ21908 | NM_024604.1 | 1314 | caccgcatctgatcaactaaa | 387779 | - | see also 8664 line |
| 48801 | FLJ21908 | NM_024604.1 | 1315 | accgcatcctgatcaactaaac | 387780 | - | see also 8664 line |
| 48802 | FLJ21919 | NM_023015.3 | 1298 | cgggatctcgtaagactacttca | 384996 | - | see also 8538 line |
| 48803 | FLJ21919 | NM_023015.3 | 1895 | cacgtgccgcaggtgtctttc | 385015 | - | see also 8538 line |
| 48804 | FLJ21940 | NM_022828.2 | 3052 | gacattcgggacgttaacacaaa | 384425 | - | see also 21609 line |
| 48805 | FLJ21940 | NM_022828.2 | 3048 | tggtgacattcgggacgttaaca | 384423 | - | see also 21609 line |
| 48806 | FLJ21941 | NM_025040.2 | 851 | aacgtacgcatactaaaactaga | 393177 | - | see also 21610 line |
| 48807 | FLJ21941 | NM_025040.2 | 1567 | gtcaaacgcactctcgttataca | 393191 | - | see also 21610 line |
| 48808 | FLJ21941 | NM_025040.2 | 845 | agcatgaacgtacgcatactaaa | 393175 | - | see also 21610 line |
| 48809 | FLJ21945 | NM_025203.1 | 137 | gcctggaccgatgggaatcaagt | 394359 | - | see also 21611 line |
| 48810 | FLJ21945 | NM_025203.1 | 2029 | gggcttatccgttaaccttca | 394404 | - | see also 21611 line |
| 48811 | FLJ21986 | NM_024913.3 | 686 | atggccgaagggccatacctctac | 392011 | - | see also 21612 line |
| 48812 | FLJ21986 | NM_024913.3 | 422 | tcccttgtcgtcggcgatttgc | 392009 | - | see also 21612 line |
| 48813 | FLJ22021 | NM_024535.1 | 1319 | gacgcaagcgagggtttctcttc | 386894 | - | see also 21613 line |
| 48814 | FLJ22021 | NM_024535.1 | 345 | ctgacaggacggtaaaactctgg | 386891 | - | see also 21613 line |
| 48815 | FLJ22054 | NM_018527.2 | 356 | ctgtcgaaccaaaatttgctga | 372117 | - | see also 7506 line |
| 48816 | FLJ22054 | NM_018527.2 | 509 | ctcttgcagcgttctgataaaaa | 372121 | - | see also 7506 line |
| 48817 | FLJ22104 | NM_022918.2 | 975 | ctgcgctggatcagaaacttaga | 384916 | - | see also 8536 line |
| 48818 | FLJ22104 | NM_022918.2 | 336 | gccgctctgccagcatcctatgt | 384898 | - | see also 8536 line |
| 48819 | FLJ22160 | NM_024585.2 | 356 | ggcgctggtcacggaattccagg | 387601 | - | see also 21616 line |
| 48820 | FLJ22169 | NM_024085.2 | 530 | ctggatccaccggcttatcaagt | 385789 | - | see also 21617 line |
| 48821 | FLJ22169 | NM_024085.2 | 977 | gtggattggcatcgctaacttcc | 385797 | - | see also 21617 line |
| 48822 | FLJ22170 | NM_025099.1 | 1697 | gtcgaagatcgtcccattagaac | 393635 | - | see also 21618 line |
| 48823 | FLJ22170 | NM_025099.1 | 419 | ggccctcatgaagcgtaatttt | 393609 | - | see also 21618 line |
| 48824 | FLJ22170 | NM_025099.1 | 418 | aggccctcatgaagcgtaatttt | 393608 | - | see also 21618 line |

Figure 46

| | | | | | | | |
|---|---|---|---|---|---|---|---|
| 48825 | FLJ22202 | NM_024883.1 | 224 | tcggagtatgcgtggattgttcg | 391651 | - | see also 21620 line |
| 48826 | FLJ22202 | NM_024883.1 | 284 | ctcctttctctgaaatttgaagg | 391652 | - | see also 21620 line |
| 48827 | FLJ22246 | NM_025232.2 | 605 | caggatcgttacagacatttt | 394489 | - | see also 21621 line |
| 48828 | FLJ22246 | NM_025232.2 | 607 | gagatcgttacagacatttat | 394490 | - | see also 21621 line |
| 48829 | FLJ22269 | NM_032219.2 | 1212 | ggccgagggaatactcatcatgc | 398709 | - | see also 21622 line |
| 48830 | FLJ22269 | NM_032219.2 | 621 | gggtgaggacattcgttaatgc | 398705 | - | see also 21622 line |
| 48831 | FLJ22301 | NM_024836.1 | 814 | gccgcttccgttgcctagaatgc | 391349 | - | see also 21623 line |
| 48832 | FLJ22313 | NM_022373.3 | 730 | aacgcatcatctaacgttacc | 382755 | - | see also 8352 line |
| 48833 | FLJ22313 | NM_022373.3 | 1402 | gggaggtccagtactaaatgaag | 382776 | - | see also 8352 line |
| 48834 | FLJ22318 | NM_022762.3 | 1132 | gacgagttaccgattgagattga | 384000 | - | see also 8457 line |
| 48835 | FLJ22318 | NM_022762.3 | 1130 | aggacgagttaccgattgagatt | 383999 | - | see also 8457 line |
| 48836 | FLJ22341 | NM_024599.2 | 1255 | ctccccgatgggggtgcaaatcc | 387730 | - | see also 21625 line |
| 48837 | FLJ22341 | NM_024599.2 | 2801 | agccgcttctgcgagaagtatga | 387736 | - | see also 21625 line |
| 48838 | FLJ22344 | NM_024717.2 | 1331 | aacgatagactgctaacacatac | 389525 | - | see also 21626 line |
| 48839 | FLJ22344 | NM_024717.2 | 2038 | ctgcattccgctgagatacattg | 389546 | - | see also 21626 line |
| 48840 | FLJ22349 | NM_024821.1 | 470 | ccgaggattgtgcactattact | 391110 | - | - |
| 48841 | FLJ22349 | NM_024821.1 | 654 | atgaccgcacagagttcattcc | 391112 | - | see also 48840 line |
| 48842 | FLJ22349 | NM_024821.1 | 523 | tccgctgcggtatcagtttctgc | 391111 | - | see also 48840 line |
| 48843 | FLJ22353 | NM_024587.1 | 672 | ctcgaggcgtgacgaagtagtcc | 387608 | - | see also 21627 line |
| 48844 | FLJ22353 | NM_024587.1 | 791 | tccgtgactacctacttactac | 387610 | - | see also 21627 line |
| 48845 | FLJ22374 | NM_032222.1 | 1055 | ctctacttgcctggtgtaattc | 398722 | - | see also 21628 line |
| 48846 | FLJ22374 | NM_032222.1 | 1130 | cccgcaccgtcaggaaaaatca | 398724 | - | see also 21628 line |
| 48847 | FLJ22390 | NM_022746.2 | 670 | gggtgtcggcggatctcaact | 383880 | - | see also 21631 line |
| 48848 | FLJ22390 | NM_022746.2 | 620 | aaggaccagattgcttactcaga | 383877 | - | see also 21631 line |
| 48849 | FLJ22457 | NM_024901.3 | 1453 | accgccgatttgtaagaagttt | 391874 | - | see also 21632 line |
| 48850 | FLJ22457 | NM_024901.3 | 1448 | gaccaaccgcgatttgtgaaga | 391873 | - | see also 21632 line |
| 48851 | FLJ22471 | NM_025140.1 | 783 | cccttgcaccgaatttgaag | 393950 | - | see also 21633 line |
| 48852 | FLJ22471 | NM_025140.1 | 781 | gcccccttgcacccgaatttga | 393949 | - | see also 21633 line |
| 48853 | FLJ22471 | NM_025140.1 | 784 | ccttgcaccgaatttgaaga | 393951 | - | see also 21633 line |
| 48854 | FLJ22490 | NM_024790.2 | 734 | tgsggcttacggtgagacatatc | 390743 | - | see also 21634 line |
| 48855 | FLJ22490 | NM_024790.2 | 1030 | tagggggatgacggatcaaactct | 390750 | - | see also 21634 line |
| 48856 | FLJ22531 | NM_024650.2 | 1095 | gggagcagccgatgactacttc | 388528 | - | see also 21635 line |
| 48857 | FLJ22531 | NM_024650.2 | 1716 | ctgaggacgcacctgataacagc | 388540 | - | see also 21635 line |
| 48858 | FLJ22555 | NM_024520.1 | 680 | aactggttaggactcgaattaa | 386610 | - | see also 21636 line |
| 48859 | FLJ22555 | NM_024520.1 | 1167 | cacggattgaacactcaaaatta | 386628 | - | see also 21636 line |
| 48860 | FLJ22557 | NM_024713.1 | 806 | ctggcttcaagcagtcattaaa | 389501 | - | see also 21637 line |
| 48861 | FLJ22557 | NM_024713.1 | 834 | tggtcagaactatcatccaaaac | 389503 | - | see also 21637 line |

Figure 46

| | | | | | | | |
|---|---|---|---|---|---|---|---|
| 48862 | FLJ22573 | NM_024660.1 | 311 | cccgttccgaaagtgttctct | 388703 | - | see also 21638 line |
| 48863 | FLJ22573 | NM_024660.1 | 268 | cgggaaaactgcggactcaatga | 388701 | - | see also 21638 line |
| 48864 | FLJ22609 | NM_022072.2 | 763 | atgatcgaagctgttgttttct | 382151 | - | see also 8265 line |
| 48865 | FLJ22609 | NM_022072.2 | 800 | ggcatcctgtaggataagtcaaa | 382152 | - | see also 8265 line |
| 48866 | FLJ22624 | NM_024808.2 | 1269 | cacctacgcagtttagtaatga | 390865 | - | see also 8748 line |
| 48867 | FLJ22624 | NM_024808.2 | 1267 | tacaactacggcagtttagtaat | 390864 | - | see also 8748 line |
| 48868 | FLJ22635 | NM_025092.1 | 728 | gggctgtggccgagtttggtgc | 393569 | - | see also 21641 line |
| 48869 | FLJ22635 | NM_025092.1 | 1007 | gggaggacttttcagatgttag | 393570 | - | see also 21641 line |
| 48870 | FLJ22639 | NM_024796.1 | 509 | acctgccgatcctgtaacaacc | 390765 | - | - |
| 48871 | FLJ22639 | NM_024796.1 | 508 | gaactgccgatcctgtaacaac | 390764 | - | see also 48870 line |
| 48872 | FLJ22649 | NM_021928.1 | 242 | ctcgcggatcatgctaaaaaatg | 381273 | - | see also 21642 line |
| 48873 | FLJ22649 | NM_021928.1 | 551 | gtcttggaacgtcgtaccaaatg | 381276 | - | see also 21642 line |
| 48874 | FLJ22662 | NM_024829.1 | 601 | gacattgctccgctctatcaag | 391186 | - | see also 21643 line |
| 48875 | FLJ22662 | NM_024829.1 | 117 | gacgcctatggcttttacaataa | 391169 | - | see also 21643 line |
| 48876 | FLJ22679 | NM_032227.1 | 721 | atgatgcatctccctttgaatt | 398734 | - | see also 9124 line |
| 48877 | FLJ22684 | NM_025048.1 | 269 | atctagcccagtcgaagaatat | 393241 | - | see also 21644 line |
| 48878 | FLJ22684 | NM_025048.1 | 530 | gagcactccaagctgtgaatgt | 393248 | - | see also 21644 line |
| 48879 | FLJ22688 | NM_025129.3 | 482 | aggacttgaggcgcagttattgc | 393826 | - | see also 21645 line |
| 48880 | FLJ22709 | NM_024578.1 | 440 | gcccgactatgagcttaagtacc | 387439 | - | see also 21647 line |
| 48881 | FLJ22709 | NM_024578.1 | 439 | tgcccgactatgagcttaagtac | 387438 | - | see also 21647 line |
| 48882 | FLJ22729 | NM_024683.1 | 904 | ttctcttcgattctatactgaag | 389143 | - | see also 21648 line |
| 48883 | FLJ22729 | NM_024683.1 | 813 | gtgctgagcatgaatcgaaatgc | 389140 | - | see also 21648 line |
| 48884 | FLJ22746 | NM_024785.2 | 646 | gacgctgactgcagtttgata | 390657 | - | see also 21649 line |
| 48885 | FLJ22746 | NM_024785.2 | 647 | acgctgtactgcagttttgataa | 390658 | - | see also 21649 line |
| 48886 | FLJ22746 | NM_024785.2 | 929 | gactacgatggcaacaagattct | 390662 | - | see also 21649 line |
| 48887 | FLJ22757 | NM_024898.1 | 1825 | gaccgagccaagtttagactgc | 391780 | - | see also 21650 line |
| 48888 | FLJ22757 | NM_024898.1 | 1898 | atggcaacagacgataagaaac | 391782 | - | see also 21650 line |
| 48889 | FLJ22789 | NM_032230.1 | 349 | ctggctgcgaaatactattctgt | 398863 | - | see also 21651 line |
| 48890 | FLJ22789 | NM_032230.1 | 126 | tgcacataccgtggatttctaca | 398858 | - | see also 21651 line |
| 48891 | FLJ22794 | NM_022074.2 | 846 | gggcgcaaactgtgtttatgc | 382183 | - | see also 21652 line |
| 48892 | FLJ22794 | NM_022074.2 | 1250 | ttcgattaaagtagtgaaacttc | 382189 | - | see also 21652 line |
| 48893 | FLJ22833 | NM_022837.1 | 486 | agggtcaacgacccacttatttt | 384527 | - | see also 8490 line |
| 48894 | FLJ22833 | NM_022837.1 | 568 | taggacgcgtgaccaaaaccaaa | 384533 | - | see also 8490 line |
| 48895 | FLJ22833 | NM_022837.1 | 801 | gcccgcaatgcgagatccataca | 384538 | - | see also 8490 line |
| 48896 | FLJ22875 | NM_032231.3 | 577 | cccaacttacgggaaattgtgg | 398923 | - | see also 9127 line |
| 48897 | FLJ22875 | NM_032231.3 | 383 | atcaggttcacgccaacagtacc | 398915 | - | see also 9127 line |
| 48898 | FLJ22875 | NM_032231.3 | 453 | ttcagcgatgtttaccatttaaa | 398919 | - | see also 9127 line |

Figure 46

| | | | | | | | |
|---|---|---|---|---|---|---|---|
| 48899 | FLJ22938 | NM_024676.2 | 1549 | ctcgctggtcccgcaaaactaca | 389011 | - | see also 21655 line |
| 48900 | FLJ22938 | NM_024676.2 | 1551 | cgctggtcccgcaaaactacacg | 389012 | - | see also 21655 line |
| 48901 | FLJ22938 | NM_024676.2 | 1209 | cccacgccaggctgagttgaagt | 389010 | - | see also 21655 line |
| 48902 | FLJ22944 | NM_025145.3 | 1118 | aagttacatgatcgaggattttc | 394007 | - | see also 21656 line |
| 48903 | FLJ22944 | NM_025145.3 | 873 | ttgcttcacccgactatgcattg | 393995 | - | see also 21656 line |
| 48904 | FLJ22944 | NM_025145.3 | 759 | ccccagtcgttgccgaaagatct | 393991 | - | see also 21656 line |
| 48905 | FLJ22965 | NM_022101.2 | 120 | agcccctccatgtttactactgt | 382287 | - | see also 21657 line |
| 48906 | FLJ22965 | NM_022101.2 | 85 | cactcgggaccgggaggaatatg | 382286 | - | see also 21657 line |
| 48907 | FLJ23018 | NM_024810.1 | 831 | tgcagcggctaatcaagctatta | 390941 | - | see also 21658 line |
| 48908 | FLJ23018 | NM_024810.1 | 835 | gcggctaatcaagctattagtac | 390943 | - | see also 21658 line |
| 48909 | FLJ23047 | NM_024548.2 | 315 | agggctaaaggaactagtacatc | 387083 | - | see also 8640 line |
| 48910 | FLJ23047 | NM_024548.2 | 1768 | gccaaagatgtgcgttacgaaat | 387122 | - | see also 8640 line |
| 48911 | FLJ23056 | NM_024582.1 | 431 | tccagtcggactttctattgaag | 387568 | - | see also 21660 line |
| 48912 | FLJ23056 | NM_024582.1 | 601 | ctgatagtgactcccatgaatct | 387570 | - | see also 21660 line |
| 48913 | FLJ23058 | NM_024696.1 | 399 | tccaacctatatctgatttctgt | 389383 | - | see also 21661 line |
| 48914 | FLJ23058 | NM_024696.1 | 564 | gtcctaactttcctgaaagtagt | 389384 | - | see also 21661 line |
| 48915 | FLJ23059 | NM_032234.2 | 1356 | gacgtgaaggcatctaatctact | 399002 | - | see also 21662 line |
| 48916 | FLJ23059 | NM_032234.2 | 1319 | cacggactgtagtaataagcttg | 398999 | - | see also 21662 line |
| 48917 | FLJ23091 | NM_024911.3 | 1307 | cacgaatccttctacagtatct | 392003 | - | see also 8804 line |
| 48918 | FLJ23091 | NM_024911.3 | 380 | tgcccgtaagaaccatcacaaga | 391980 | - | see also 8804 line |
| 48919 | FLJ23091 | NM_024911.3 | 786 | gggtctgtggcccataagtttta | 391993 | - | see also 8804 line |
| 48920 | FLJ23129 | NM_024763.2 | 620 | aacgggaaagattgactagtttc | 390197 | - | see also 21664 line |
| 48921 | FLJ23129 | NM_024763.2 | 840 | cagattaggcaatgacctatatg | 390203 | - | see also 21664 line |
| 48922 | FLJ23151 | NM_024772.2 | 2613 | gtccatgatcgtacattactatc | 390431 | - | see also 21665 line |
| 48923 | FLJ23151 | NM_024772.2 | 2699 | tacaagtttcgccgatgaattga | 390434 | - | see also 21665 line |
| 48924 | FLJ23151 | NM_024772.2 | 2609 | gacagtccatgatcgtacattac | 390430 | - | see also 21665 line |
| 48925 | FLJ23186 | NM_024616.1 | 600 | ttccgtgatcagaatatacctgg | 388056 | - | see also 21666 line |
| 48926 | FLJ23186 | NM_024616.1 | 594 | caggatttccgtgatcagaatat | 388055 | - | see also 21666 line |
| 48927 | FLJ23191 | NM_024574.2 | 1387 | aacatgagcaccgcttatgtagg | 387416 | - | see also 21667 line |
| 48928 | FLJ23191 | NM_024574.2 | 1925 | gtctaggaccagatataaggaag | 387430 | - | see also 21667 line |
| 48929 | FLJ23191 | NM_024574.2 | 1337 | aacccgacacgcagtactacttt | 387413 | - | see also 21667 line |
| 48930 | FLJ23221 | NM_024579.1 | 348 | tccagtttgcgaagtcctattcc | 387454 | - | see also 21668 line |
| 48931 | FLJ23221 | NM_024579.1 | 117 | caggtggtctattattatacagt | 387442 | - | see also 21668 line |
| 48932 | FLJ23231 | NM_025079.1 | 1522 | cgggctacagtccctatggatct | 393509 | - | see also 21669 line |
| 48933 | FLJ23231 | NM_025079.1 | 280 | tccggaagctgggctattcatcc | 393507 | - | see also 21669 line |
| 48934 | FLJ23235 | NM_024943.1 | 670 | atggaatacccgaatgattgtat | 392404 | - | see also 21670 line |
| 48935 | FLJ23235 | NM_024943.1 | 667 | atcatggaatacccgaatgattg | 392403 | - | see also 21670 line |

Figure 46

| 48936 | FLJ23259 | NM_024727.1 | 1401 | agctaatcgagctggatattagc | 389718 | - | see also 21671 line |
|-------|----------|-------------|------|-------------------------|--------|---|---------------------|
| 48937 | FLJ23259 | NM_024727.1 | 869 | cagttggtcatgctaaagcatct | 389705 | - | see also 21671 line |
| 48938 | FLJ23263 | NM_025115.1 | 861 | ttggcaggacccgtttatatttt | 393777 | - | see also 8865 line |
| 48939 | FLJ23263 | NM_025115.1 | 858 | cacttggcaggacccgtttatat | 393776 | - | see also 8865 line |
| 48940 | FLJ23311 | NM_024680.2 | 981 | acgtcgacgcatttacgatatcg | 389062 | - | see also 21674 line |
| 48941 | FLJ23311 | NM_024680.2 | 984 | tcgacgcatttacgatatcgtga | 389063 | - | see also 21674 line |
| 48942 | FLJ23342 | NM_024631.1 | 1267 | tagaacccacccgaattatctat | 388314 | - | see also 8673 line |
| 48943 | FLJ23342 | NM_024631.1 | 51 | cccgccaactcgccgctaaaaat | 388292 | - | see also 8673 line |
| 48944 | FLJ23375 | NM_024956.2 | 947 | gtcggagcaaccatacaatttgg | 392710 | - | see also 8825 line |
| 48945 | FLJ23375 | NM_024956.2 | 1562 | tccgtactgatcactacatcatg | 392731 | - | see also 8825 line |
| 48946 | FLJ23403 | NM_022068.1 | 827 | gacaccggtgctatgcaatttct | 382034 | - | see also 8262 line |
| 48947 | FLJ23403 | NM_022068.1 | 134 | gccgtgactgacgtgtatgtact | 382022 | - | see also 8262 line |
| 48948 | FLJ23451 | NM_024766.1 | 829 | cacgccgagggaatactttaaac | 390308 | - | see also 21677 line |
| 48949 | FLJ23451 | NM_024766.1 | 666 | tgcgttttacgatgggataatga | 390300 | - | see also 21677 line |
| 48950 | FLJ23467 | NM_024575.1 | 526 | cgcatccgccacgtgtttgatca | 387436 | - | see also 21678 line |
| 48951 | FLJ23471 | NM_024723.1 | 518 | tccgccctcgtgtgaccaatagc | 389560 | - | see also 21679 line |
| 48952 | FLJ23471 | NM_024723.1 | 194 | agggctaccgcgacgtgaatatc | 389558 | - | see also 21679 line |
| 48953 | FLJ23476 | NM_024640.2 | 780 | agccatcctccaacagaagtacg | 388429 | - | see also 21680 line |
| 48954 | FLJ23476 | NM_024640.2 | 635 | ggcctcagttgtccttggttatt | 388427 | - | see also 21680 line |
| 48955 | FLJ23514 | NM_021827.1 | 702 | ctcagtcctgcgcttaatgctct | 381195 | - | see also 21681 line |
| 48956 | FLJ23514 | NM_021827.1 | 1082 | agccatcctgcatcaactagtgg | 381204 | - | see also 21681 line |
| 48957 | FLJ23518 | NM_024725.2 | 793 | cagcgatatcctagttagaaaag | 389595 | - | see also 21682 line |
| 48958 | FLJ23518 | NM_024725.2 | 511 | tggcaacggttcaacatatgaag | 389588 | - | see also 21682 line |
| 48959 | FLJ23550 | NM_025063.1 | 1360 | cccagctcttgacgactcttatg | 393432 | - | see also 21683 line |
| 48960 | FLJ23550 | NM_025063.1 | 729 | aagcctacaaacggtaaaagtca | 393415 | - | see also 21683 line |
| 48961 | FLJ23560 | NM_024685.2 | 1211 | atgttcatctaggcttgataagc | 389178 | - | see also 21684 line |
| 48962 | FLJ23560 | NM_024685.2 | 829 | gacatgcgaatggtgatagtaac | 389159 | - | see also 21684 line |
| 48963 | FLJ23584 | NM_024588.2 | 462 | acccctgccagggatcatagagt | 387613 | - | see also 21685 line |
| 48964 | FLJ23584 | NM_024588.2 | 662 | cccggaagctgccatcattctag | 387614 | - | see also 21685 line |
| 48965 | FLJ23594 | NM_024781.1 | 748 | aacaaccgagtggatcaaaatga | 390635 | - | see also 21686 line |
| 48966 | FLJ23594 | NM_024781.1 | 920 | ctgaactcaggcacttgcaaaac | 390636 | - | see also 21686 line |
| 48967 | FLJ23594 | NM_024781.1 | 691 | gcctactataaactaaacagaca | 390633 | - | see also 21686 line |
| 48968 | FLJ23654 | NM_152550.1 | 975 | caccgcaagacacctttagaga | 417989 | - | see also 9880 line |
| 48969 | FLJ23654 | NM_152550.1 | 643 | cccggtgacctaaggtttaatag | 417981 | - | see also 9880 line |
| 48970 | FLJ23654 | NM_152550.1 | 642 | tcccggtgacctaaggtttaata | 417980 | - | see also 9880 line |
| 48971 | FLJ23657 | NM_178497.2 | 355 | cacaccgttaccttacttatagg | 430893 | - | see also 21688 line |
| 48972 | FLJ23657 | NM_178497.2 | 353 | gccacaccgttaccttacttata | 430892 | - | see also 21688 line |

Figure 46

| | | | | | | | |
|---|---|---|---|---|---|---|---|
| 48973 | FLJ23657 | NM_178497.2 | 358 | acggttaccttacttattaggtat | 430894 | - | see also 21688 line |
| 48974 | FLJ23703 | NM_182534.1 | 387 | cccacaaattaccgtaatgcaaa | 434213 | - | see also 21689 line |
| 48975 | FLJ23703 | NM_182534.1 | 112 | ccgccattctggcttcttaact | 434211 | - | see also 21689 line |
| 48976 | FLJ23825 | NM_173620.1 | 531 | tcgggtgtgatgaggtctattac | 426586 | - | see also 21690 line |
| 48977 | FLJ23825 | NM_173620.1 | 532 | cgggtgtgatgaggtctattacc | 426587 | - | see also 21690 line |
| 48978 | FLJ23865 | NM_152669.1 | 408 | gccgctgcctgatgattgtgaga | 419553 | - | - |
| 48979 | FLJ23865 | NM_152669.1 | 414 | gcctgatgattgtgagattctgt | 419554 | - | see also 48978 line |
| 48980 | FLJ23878 | NM_144990.1 | 248 | ccctcggcgcacactctctatgt | 412463 | - | see also 21691 line |
| 48981 | FLJ25045 | NM_178537.3 | 3028 | ctccgactgcggaattctcatca | 431145 | - | see also 21692 line |
| 48982 | FLJ25045 | NM_178537.3 | 716 | cccggaggtactacttgagttg | 431132 | - | see also 21692 line |
| 48983 | FLJ25056 | NM_182530.1 | 235 | gacgatagttgccctctatatac | 434180 | - | see also 21693 line |
| 48984 | FLJ25056 | NM_182530.1 | 245 | gccctctatatacttagcttaaa | 434183 | - | see also 21693 line |
| 48985 | FLJ25067 | NM_152504.2 | 502 | aagtatgccctccgcagttttca | 417300 | - | see also 21694 line |
| 48986 | FLJ25067 | NM_152504.2 | 153 | agcgtgtgacataagagattacg | 417284 | - | see also 21694 line |
| 48987 | FLJ25102 | NM_182626.1 | 386 | gaccagccttacgtcttgaaagg | 434764 | - | see also 21695 line |
| 48988 | FLJ25102 | NM_182626.1 | 420 | ttctggcttcaggtgttactcg | 434765 | - | see also 21695 line |
| 48989 | FLJ25124 | NM_144698.2 | 601 | cgcccgagttaatgttacagaca | 411469 | - | see also 21696 line |
| 48990 | FLJ25124 | NM_144698.2 | 2981 | tccacctacaggaatcatctact | 411477 | - | see also 21696 line |
| 48991 | FLJ25143 | NM_182500.1 | 558 | gagtttcctgaaagactatgacc | 433970 | - | see also 21697 line |
| 48992 | FLJ25161 | NM_182522.2 | 810 | ttgtaaagtgctgccagattact | 434153 | - | see also 10267 line |
| 48993 | FLJ25161 | NM_182522.2 | 727 | cgggctcaaccttcttgtgttga | 434152 | - | see also 10267 line |
| 48994 | FLJ25168 | NM_152466.1 | 479 | atcagggtcacatacttctaag | 416982 | - | see also 21699 line |
| 48995 | FLJ25168 | NM_152466.1 | 502 | tacaccctactgccttggaatct | 416983 | - | see also 21699 line |
| 48996 | FLJ25224 | NM_182495.2 | 20 | tccataggatactcactttgttt | 433910 | - | see also 21700 line |
| 48997 | FLJ25224 | NM_182495.2 | 760 | tactgtgtgaggcctcaacatat | 433917 | - | see also 21700 line |
| 48998 | FLJ25286 | NM_152546.1 | 913 | agcggtgacgaacttcttcattgg | 417897 | - | see also 9879 line |
| 48999 | FLJ25286 | NM_152546.1 | 176 | ttggccgactgaagtcaaaaaag | 417883 | - | see also 9879 line |
| 49000 | FLJ25323 | NM_198521.1 | 855 | cacaccccgacgacatccaaagc | 439100 | - | see also 21702 line |
| 49001 | FLJ25323 | NM_198521.1 | 376 | ggcgtgtaaaagactacttcata | 439090 | - | see also 21702 line |
| 49002 | FLJ25328 | NM_152483.1 | 505 | agcccagagcgcccaaagaactg | 417052 | - | see also 21703 line |
| 49003 | FLJ25328 | NM_152483.1 | 302 | atggctctttgatagcaatttt | 417047 | - | see also 21703 line |
| 49004 | FLJ25333 | NM_152548.1 | 401 | aacaacaggcgcgctaccatagc | 417907 | - | see also 21704 line |
| 49005 | FLJ25333 | NM_152548.1 | 901 | gggggattatcgccacgaaatga | 417923 | - | see also 21704 line |
| 49006 | FLJ25351 | NM_152525.3 | 1297 | aaccaatcggaccaagataattc | 417647 | - | see also 21705 line |
| 49007 | FLJ25351 | NM_152525.3 | 1298 | accaatcgaccaagataattca | 417648 | - | see also 21705 line |
| 49008 | FLJ25351 | NM_152525.3 | 156 | cccggatatcatgcacattaaag | 417614 | - | see also 21705 line |
| 49009 | FLJ25359 | NM_144587.1 | 1580 | agcggcacgcctggtgaagtatg | 410065 | - | see also 21706 line |

Figure 46

| | | | | | | | |
|---|---|---|---|---|---|---|---|
| 49010 | FLJ25359 | NM_144587.1 | 1581 | gcggcacgcctggtgaagtatga | 410066 | - | see also 21706 line |
| 49011 | FLJ25371 | NM_152543.1 | 603 | agctgccgaagctaccaaaaagg | 417858 | - | see also 21707 line |
| 49012 | FLJ25371 | NM_152543.1 | 320 | tggagaataccacagtataattt | 417847 | - | see also 21707 line |
| 49013 | FLJ25371 | NM_152543.1 | 86 | atggcatctgtatcataccaaaa | 417837 | - | see also 21707 line |
| 49014 | FLJ25378 | NM_152768.1 | 257 | agcaagaggtgatgttccaaagc | 420890 | - | see also 21708 line |
| 49015 | FLJ25378 | NM_152768.1 | 277 | agcagaggaaggctaagtttata | 420891 | - | see also 21708 line |
| 49016 | FLJ25393 | NM_181645.1 | 103 | ttggagacacgattagatcttcg | 432651 | - | see also 21709 line |
| 49017 | FLJ25393 | NM_181645.1 | 1039 | gtggaagagtaccataactctga | 432674 | - | see also 21709 line |
| 49018 | FLJ25409 | NM_144652.1 | 1211 | tacagccgccaccagttacatca | 410862 | - | see also 21710 line |
| 49019 | FLJ25409 | NM_144652.1 | 1214 | agccgccaccagttacatcatca | 410864 | - | see also 21710 line |
| 49020 | FLJ25414 | NM_152343.1 | 1036 | gacaagaacttcgctcaatctta | 415726 | - | see also 21711 line |
| 49021 | FLJ25414 | NM_152343.1 | 160 | atgcgagatgacttaagtcaaca | 415723 | - | see also 21711 line |
| 49022 | FLJ25416 | NM_145018.2 | 798 | gactctcaggcacctaacaatca | 412691 | - | see also 21712 line |
| 49023 | FLJ25416 | NM_145018.2 | 1649 | aagatcatcacaagtaatagtca | 412713 | - | see also 21712 line |
| 49024 | FLJ25421 | NM_152512.3 | 1282 | gccccgacttaagggtatcaaag | 417373 | - | see also 21713 line |
| 49025 | FLJ25421 | NM_152512.3 | 696 | cagcgtacctcccactctatatt | 417357 | - | see also 21713 line |
| 49026 | FLJ25422 | NM_145000.2 | 698 | agcgtgtacggtcttcatctttt | 412549 | - | see also 21714 line |
| 49027 | FLJ25422 | NM_145000.2 | 1598 | tacgaataacagctactgattta | 412571 | - | see also 21714 line |
| 49028 | FLJ25429 | NM_152376.2 | 258 | gtgtgcgctcatcatataccatc | 415966 | - | see also 21715 line |
| 49029 | FLJ25429 | NM_152376.2 | 255 | gaggtgtgcgctcatcatatacc | 415965 | - | see also 21715 line |
| 49030 | FLJ25436 | NM_144581.1 | 599 | atggcggtgcattttatgcattt | 409943 | - | see also 21716 line |
| 49031 | FLJ25436 | NM_144581.1 | 873 | agcccgaattgccttacagtatc | 409949 | - | see also 21716 line |
| 49032 | FLJ25444 | NM_152761.1 | 1034 | ctcaaaccgtctacgaaatgaaa | 420801 | - | see also 21717 line |
| 49033 | FLJ25444 | NM_152761.1 | 1032 | atctcaaaccgtctacgaaatga | 420800 | - | see also 21717 line |
| 49034 | FLJ25444 | NM_152761.1 | 1078 | cagtaacgcaagagatccataag | 420804 | - | see also 21717 line |
| 49035 | FLJ25476 | NM_152493.2 | 207 | aggatggccgagcctcgatttaa | 417099 | - | see also 21718 line |
| 49036 | FLJ25476 | NM_152493.2 | 1404 | ggcatcccggtgcgaatctctct | 417106 | - | see also 21718 line |
| 49037 | FLJ25477 | NM_152704.2 | 273 | cgccgtcggggaagattaataaa | 419899 | - | see also 21719 line |
| 49038 | FLJ25477 | NM_152704.2 | 1834 | gtcctccctgtcccggttaaagc | 419908 | - | see also 21719 line |
| 49039 | FLJ25477 | NM_152704.2 | 1751 | agcggggacgcgctctatgatct | 419906 | - | see also 21719 line |
| 49040 | FLJ25530 | NM_152722.3 | 334 | accgtatccgactctttgaaaat | 420082 | - | see also 9931 line |
| 49041 | FLJ25530 | NM_152722.3 | 333 | gaccgtatccgactctttgaaaa | 420081 | - | see also 9931 line |
| 49042 | FLJ25534 | NM_153234.3 | 875 | gacaagaactacggttttacaaa | 422441 | - | see also 21721 line |
| 49043 | FLJ25534 | NM_153234.3 | 689 | aagcccttcgtgaaacaaagtgt | 422432 | - | see also 21721 line |
| 49044 | FLJ25555 | NM_152345.3 | 604 | ccctagtgacacctacaaagtgt | 415733 | - | see also 21722 line |
| 49045 | FLJ25555 | NM_152345.3 | 1013 | acccgcacacggacagaacatct | 415737 | - | see also 21722 line |
| 49046 | FLJ25590 | NM_175895.2 | 459 | aggaggcgaaaacggaacaaacg | 429516 | - | - | - | - |

EP 1 752 536 A1

Figure 46

| | | | | | | | |
|---|---|---|---|---|---|---|---|
| 49047 | FLJ25621 | NM_198514.1 | 1511 | cagtagagctcaggtatgtatt | 438912 | - | see also 10334 line |
| 49048 | FLJ25621 | NM_198514.1 | 1220 | tccatcaggatcaaagctaact | 438907 | - | see also 10334 line |
| 49049 | FLJ25660 | NM_152481.1 | 737 | ggctcgactgtccttaggatcc | 417044 | - | see also 21724 line |
| 49050 | FLJ25660 | NM_152481.1 | 529 | cgcctgggtaccgctacattga | 417037 | - | see also 21724 line |
| 49051 | FLJ25694 | NM_173604.1 | 819 | gtcggacccgtccgatagtaaa | 426478 | - | see also 21725 line |
| 49052 | FLJ25694 | NM_173604.1 | 906 | tcctccagtcaatcccaattaag | 426481 | - | see also 21725 line |
| 49053 | FLJ25715 | NM_182570.1 | 403 | caacgaactgcagcttacactct | 434481 | - | - |
| 49054 | FLJ25735 | NM_152577.1 | 723 | gtgggaaaccgttagtatttta | 418196 | - | see also 21726 line |
| 49055 | FLJ25735 | NM_152577.1 | 489 | cagcgcttcgcttgaaaaagtc | 418179 | - | see also 21726 line |
| 49056 | FLJ25736 | NM_152578.1 | 242 | atggcgggaatctctaaagatgc | 418218 | - | see also 21727 line |
| 49057 | FLJ25736 | NM_152578.1 | 141 | caacttagagctggcaacttatga | 418216 | - | see also 21727 line |
| 49058 | FLJ25770 | NM_178555.2 | 1235 | cccggacagagcgtgatcaatcc | 431250 | - | see also 10196 line |
| 49059 | FLJ25770 | NM_178555.2 | 1234 | gcccggacagagcgtgatcaatc | 431249 | - | see also 10196 line |
| 49060 | FLJ25773 | NM_182560.1 | 484 | agggaacaaagccgatgatcaca | 434449 | - | see also 21729 line |
| 49061 | FLJ25773 | NM_182560.1 | 555 | cacctgcagagcctaagacataa | 434450 | - | see also 21729 line |
| 49062 | FLJ25791 | NM_173559.1 | 683 | agggaaccgatacttcttactag | 426078 | - | see also 21730 line |
| 49063 | FLJ25791 | NM_173559.1 | 461 | aggagtcgattccctaagrtgtgc | 426073 | - | see also 21730 line |
| 49064 | FLJ25801 | NM_173553.1 | 828 | caggtgcaagtggccataracc | 426000 | - | see also 21731 line |
| 49065 | FLJ25801 | NM_173553.1 | 219 | gagactccggttgttgaatgaag | 425991 | - | see also 21731 line |
| 49066 | FLJ25851 | NM_174927.1 | 386 | cacgaatccagttcattaagatgg | 428716 | - | see also 21732 line |
| 49067 | FLJ25851 | NM_174927.1 | 486 | aagcatatccgtcttacagatg | 428721 | - | see also 21732 line |
| 49068 | FLJ25952 | NM_153251.1 | 22 | atgcctaatttgcaaaatcact | 422595 | - | - |
| 49069 | FLJ25954 | NM_173572.1 | 673 | cacggcagctccgttcattaagt | 426272 | - | see also 21733 line |
| 49070 | FLJ25954 | NM_173572.1 | 438 | gagaaccgaggtactacttttg | 426266 | - | see also 21733 line |
| 49071 | FLJ25955 | NM_178821.1 | 782 | atcacgggtctttgatcatac | 431408 | - | see also 21734 line |
| 49072 | FLJ25955 | NM_178821.1 | 386 | tcgggctcatgcttatcacagg | 431402 | - | see also 21734 line |
| 49073 | FLJ25972 | NM_178822.3 | 5776 | gagcgaagagtagtaatgcttac | 431577 | - | see also 21735 line |
| 49074 | FLJ25972 | NM_178822.3 | 2152 | aggcacaactatcgggaattaac | 431480 | - | see also 21735 line |
| 49075 | FLJ25976 | NM_174943.2 | 662 | cggagaacatgatcttaaccata | 428770 | - | see also 21736 line |
| 49076 | FLJ25989 | NM_198512.1 | 745 | ggcataccttgtcccttcatatt | 438875 | - | see also 21737 line |
| 49077 | FLJ25989 | NM_198512.1 | 804 | tccctgagggcacgtggttaagg | 438878 | - | see also 21737 line |
| 49078 | FLJ26016 | NM_198472.1 | 355 | aagatagagaggtttgagtttta | 438405 | - | see also 21738 line |
| 49079 | FLJ30046 | NM_144595.2 | 655 | ctccggtcacgtgcgaaatagt | 410118 | - | see also 21739 line |
| 49080 | FLJ30046 | NM_144595.2 | 794 | gtctatccgacagcctctaaag | 410120 | - | see also 21739 line |
| 49081 | FLJ30213 | NM_145008.1 | 512 | cccgctgtcacgcacttacagc | 412587 | - | see also 21740 line |
| 49082 | FLJ30213 | NM_145008.1 | 603 | tggccgagcctacctgtttaact | 412591 | - | see also 21740 line |
| 49083 | FLJ30277 | NM_153008.3 | 965 | ccctgttgcataggcatttattt | 421813 | - | see also 21741 line |

Figure 46

| | | | | | | | |
|---|---|---|---|---|---|---|---|
| 49084 | FLJ30277 | NM_153008.3 | 964 | tccctgttgcataggcatttatt | 421812 | - | see also 21741 line |
| 49085 | FLJ30294 | NM_144632.2 | 151 | gactaaatatcgctatcttcttt | 410594 | - | see also 21742 line |
| 49086 | FLJ30294 | NM_144632.2 | 780 | ttgctagctggattactatttct | 410611 | - | see also 21742 line |
| 49087 | FLJ30313 | NM_152757.1 | 667 | gccgggaaggaccatctgattct | 420697 | - | - | - | - |
| 49088 | FLJ30317 | NM_172136.1 | 182 | aaggaacagggattttgtctct | 424268 | - | - | - | - |
| 49089 | FLJ30373 | NM_152479.1 | 220 | gcgctgctatcgagagaagaagt | 417016 | - | see also 21743 line |
| 49090 | FLJ30373 | NM_152479.1 | 394 | gagcacggaggtggagtgttacg | 417018 | - | see also 21743 line |
| 49091 | FLJ30430 | NM_153009.1 | 372 | tgcaacccaagcacctatcttcc | 421816 | - | see also 21744 line |
| 49092 | FLJ30430 | NM_153009.1 | 443 | ggctgtcacggccgtttatcaca | 421819 | - | see also 21744 line |
| 49093 | FLJ30469 | NM_182573.1 | 332 | cgcggctgcacaactgacaaatg | 434482 | - | see also 21745 line |
| 49094 | FLJ30469 | NM_182573.1 | 539 | atgacagttggcaatttctcagt | 434484 | - | see also 21745 line |
| 49095 | FLJ30507 | NM_153010.3 | 549 | tacacgtgcatagaccaatttgc | 421823 | - | see also 21746 line |
| 49096 | FLJ30507 | NM_153010.3 | 625 | cacgttcacccaagaagtattta | 421825 | - | see also 21746 line |
| 49097 | FLJ30525 | NM_144584.1 | 521 | cagcggtaccagttcgttaaaaa | 409980 | - | see also 21747 line |
| 49098 | FLJ30525 | NM_144584.1 | 519 | gacagcggtaccagttcgttaaa | 409979 | - | see also 21747 line |
| 49099 | FLJ30525 | NM_144584.1 | 1478 | aaccgcttatgtgctaatgaaga | 410014 | - | see also 21747 line |
| 49100 | FLJ30634 | NM_153014.1 | 757 | ctcttctccctgttatccaattt | 421854 | - | see also 21748 line |
| 49101 | FLJ30634 | NM_153014.1 | 795 | ttgggtaaggatgaagtgttaaa | 421855 | - | see also 21748 line |
| 49102 | FLJ30655 | NM_144643.1 | 1337 | agcagttacagtctagtataaaa | 410657 | - | see also 21749 line |
| 49103 | FLJ30655 | NM_144643.1 | 1317 | agcatcagataggcgtttacagc | 410653 | - | see also 21749 line |
| 49104 | FLJ30655 | NM_144643.1 | 1108 | aacatggaagtgactaaccaaca | 410648 | - | see also 21749 line |
| 49105 | FLJ30668 | NM_153015.1 | 878 | agctctatcgtcgaaacagattt | 421879 | - | see also 21750 line |
| 49106 | FLJ30668 | NM_153015.1 | 387 | agcggaacggaaagtctgtaact | 421864 | - | see also 21750 line |
| 49107 | FLJ30672 | NM_153016.2 | 366 | ctctttgcagccttgaaacatgg | 421882 | - | see also 21751 line |
| 49108 | FLJ30707 | NM_145019.2 | 768 | tactgtcgctacgacaactatgc | 412759 | - | - | - | - |
| 49109 | FLJ30707 | NM_145019.2 | 1075 | cacaaagggtgcataagaagttt | 412760 | - | see also 49108 line |
| 49110 | FLJ30707 | NM_145019.2 | 218 | gtcctccacgagcaatggattct | 412758 | - | see also 49108 line |
| 49111 | FLJ30794 | NM_152527.1 | 836 | gccgacttgccttgcattagtgt | 417677 | - | see also 21752 line |
| 49112 | FLJ30794 | NM_152527.1 | 834 | tagccgacttgccttgcattagt | 417676 | - | see also 21752 line |
| 49113 | FLJ30834 | NM_152399.1 | 549 | aacggggcatgccagttcaaatc | 416271 | - | see also 21753 line |
| 49114 | FLJ30834 | NM_152399.1 | 361 | gccatctggtgcgattctacaga | 416268 | - | see also 21753 line |
| 49115 | FLJ30851 | NM_198553.1 | 323 | gtgtgagactggcaaaatatatt | 439467 | - | see also 10339 line |
| 49116 | FLJ30851 | NM_198553.1 | 428 | tccaaacgagtggctgaaactgc | 439470 | - | see also 10339 line |
| 49117 | FLJ30973 | NM_152451.1 | 1313 | agcaggttggactatttaacaga | 416932 | - | see also 21755 line |
| 49118 | FLJ30973 | NM_152451.1 | 1073 | atgtctggggagttaactgattc | 416928 | - | see also 21755 line |
| 49119 | FLJ30990 | NM_152517.1 | 1877 | gagtctaggcagttaaaagcttt | 417462 | - | see also 21756 line |
| 49120 | FLJ30990 | NM_152517.1 | 1100 | ctccggaaacttaccatacaagt | 417458 | - | see also 21756 line |

Figure 46

| 49121 | FLJ31031 | NM_182533.1 | 262 | ctgtcggacccaagaccttttcc | 434206 | - | see also 21757 line |
|---|---|---|---|---|---|---|---|
| 49122 | FLJ31031 | NM_182533.1 | 553 | aagacgtgacgtgtctttcatgg | 434208 | - | see also 21757 line |
| 49123 | FLJ31051 | NM_153687.1 | 657 | ctcactgaacggctaaaagattt | 423510 | - | see also 21758 line |
| 49124 | FLJ31051 | NM_153687.1 | 679 | tggaagatagcacactaagaaat | 423513 | - | see also 21758 line |
| 49125 | FLJ31052 | NM_152378.1 | 918 | gccgctgcagttcgcgttttaca | 415988 | - | see also 21759 line |
| 49126 | FLJ31052 | NM_152378.1 | 510 | ggcgcagcgtctcctattctagg | 415986 | - | see also 21759 line |
| 49127 | FLJ31164 | NM_145003.1 | 197 | ccctagagtgcgctagatgttgg | 412578 | - | see also 21761 line |
| 49128 | FLJ31164 | NM_145003.1 | 196 | cccctagagtgcgctagatgttg | 412577 | - | see also 21761 line |
| 49129 | FLJ31166 | NM_153022.1 | 1013 | gagaaggagtcgactcgaatagt | 421887 | - | see also 21762 line |
| 49130 | FLJ31166 | NM_153022.1 | 750 | gtgaagtgaccgtcattgctttc | 421885 | - | see also 21762 line |
| 49131 | FLJ31338 | NM_152469.1 | 1110 | atgcgtgcgcaccgttattttct | 416993 | - | see also 21764 line |
| 49132 | FLJ31338 | NM_152469.1 | 1114 | gtgcgcaccgttattttctaatg | 416995 | - | see also 21764 line |
| 49133 | FLJ31338 | NM_152469.1 | 1115 | tgcgcaccgttattttctaatgg | 416996 | - | see also 21764 line |
| 49134 | FLJ31340 | NM_152748.2 | 616 | gtgtaaagacgactctcaatttt | 420453 | - | see also 9942 line |
| 49135 | FLJ31340 | NM_152748.2 | 2529 | tagtaatgacgactaactcaaaa | 420509 | - | see also 9942 line |
| 49136 | FLJ31434 | NM_152496.1 | 806 | ggcccgagatcgtttccattacc | 417137 | - | see also 21766 line |
| 49137 | FLJ31434 | NM_152496.1 | 374 | gtgcattttaccgctataagaac | 417129 | - | see also 21766 line |
| 49138 | FLJ31438 | NM_152385.1 | 2067 | aggatgacgcagtcaacctaatg | 416121 | - | see also 21767 line |
| 49139 | FLJ31438 | NM_152385.1 | 1569 | atggggagcaggatacttataac | 416106 | - | see also 21767 line |
| 49140 | FLJ31461 | NM_152454.1 | 204 | atgaattgtgatgctttgctaca | 416954 | - | see also 21768 line |
| 49141 | FLJ31461 | NM_152454.1 | 559 | atcccacatgtgtcttcacattc | 416957 | - | see also 21768 line |
| 49142 | FLJ31528 | NM_144679.1 | 86 | aaggggacgtccgatgatgatgt | 411282 | - | see also 21769 line |
| 49143 | FLJ31528 | NM_144679.1 | 88 | ggggacgtccgatgatgatgtcc | 411283 | - | see also 21769 line |
| 49144 | FLJ31564 | NM_144720.1 | 1530 | cacgcgggaaaacgtggaaatga | 411779 | - | see also 9739 line |
| 49145 | FLJ31564 | NM_144720.1 | 1275 | gggcgtccaggatcaacatatgg | 411774 | - | see also 9739 line |
| 49146 | FLJ31564 | NM_144720.1 | 1582 | aagcggcatacctccttgaatga | 411780 | - | see also 9739 line |
| 49147 | FLJ31568 | NM_152509.1 | 491 | tcgcagcctgtcctacaataatg | 417316 | - | see also 21771 line |
| 49148 | FLJ31568 | NM_152509.1 | 931 | accctgtcctgcagacatcattg | 417318 | - | see also 21771 line |
| 49149 | FLJ31606 | NM_153025.1 | 381 | cagtacagcagacgcctataatt | 421894 | - | see also 21772 line |
| 49150 | FLJ31606 | NM_153025.1 | 49 | aagggatccacctattcagttcc | 421891 | - | see also 21772 line |
| 49151 | FLJ31659 | NM_153027.1 | 387 | ggctgtcacgccaccgaaattgc | 421971 | - | - | | |
| 49152 | FLJ31659 | NM_153027.1 | 304 | tcctgggagaagcgaatcgtttt | 421970 | - | see also 49151 line |
| 49153 | FLJ31737 | NM_144984.1 | 381 | ctccgatcaagggcattacgtct | 412436 | - | see also 21773 line |
| 49154 | FLJ31737 | NM_144984.1 | 550 | ttgcccttcaccgcagacttaac | 412439 | - | see also 21773 line |
| 49155 | FLJ31751 | NM_152652.1 | 166 | agccgaatgagtttgaacatacc | 419510 | - | see also 21774 line |
| 49156 | FLJ31751 | NM_152652.1 | 161 | ttctcagccgaatgagtttgaac | 419509 | - | see also 21774 line |
| 49157 | FLJ31795 | NM_144609.1 | 335 | agcaggcacaaatcgtagtaaag | 410289 | - | see also 21775 line |

Figure 46

| | | | | | | | |
|---|---|---|---|---|---|---|---|
| 49158 | FLJ31795 | NM_144609.1 | 462 | aggtgctaccacaatgaacattg | 410291 | - | see also 21775 line |
| 49159 | FLJ31810 | NM_152570.1 | 2153 | aggattcgttcagatcgtttc | 418149 | - | see also 9884 line |
| 49160 | FLJ31810 | NM_152570.1 | 2180 | tgcgaacaggacccctatgtaca | 418152 | - | see also 9884 line |
| 49161 | FLJ31818 | NM_152556.1 | 399 | agggcgaaggtcgtattgaatgg | 418076 | - | see also 9881 line |
| 49162 | FLJ31818 | NM_152556.1 | 708 | ctgtagagagtgtcataaatgt | 418086 | - | see also 9881 line |
| 49163 | FLJ31842 | NM_152487.1 | 714 | agctatcgttatcaatggaatac | 417075 | - | see also 9878 line |
| 49164 | FLJ31842 | NM_152487.1 | 570 | tgcgtccctgatgcatactacc | 417070 | - | see also 9878 line |
| 49165 | FLJ31846 | NM_144974.1 | 788 | aggaaggaaccgaataacacaag | 412164 | - | see also 21779 line |
| 49166 | FLJ31846 | NM_144974.1 | 714 | ctccatgaaaagcgagatttgt | 412162 | - | see also 21779 line |
| 49167 | FLJ31882 | NM_152460.1 | 709 | gactaagttacagtgtatgttgc | 416966 | - | - |
| 49168 | FLJ31882 | NM_152460.1 | 653 | ctgtagctacttccttttaca | 416964 | - | see also 49167 line |
| 49169 | FLJ31882 | NM_152460.1 | 732 | aaggtgtgcccaaacttttgtgc | 416967 | - | see also 49167 line |
| 49170 | FLJ31951 | NM_144726.1 | 1010 | aggtttatcgagcttcatgaat | 411836 | - | see also 21780 line |
| 49171 | FLJ31951 | NM_144726.1 | 1989 | cagggacaataatgagrtacattg | 411858 | - | see also 21780 line |
| 49172 | FLJ31952 | NM_144682.2 | 1190 | ttcaaaacctcgggtagagtaca | 411291 | - | see also 21781 line |
| 49173 | FLJ31952 | NM_144682.2 | 1206 | gagtacagcaccaaaatcgtaga | 411292 | - | see also 21781 line |
| 49174 | FLJ31952 | NM_144682.2 | 2540 | agccgagtacatacaacaagaaa | 411299 | - | see also 21781 line |
| 49175 | FLJ32000 | NM_152498.1 | 403 | tgccgaagcgcaaagttcttaa | 417163 | - | see also 21782 line |
| 49176 | FLJ32000 | NM_152498.1 | 1971 | aaccattcgtgccatgaagtacc | 417210 | - | see also 21782 line |
| 49177 | FLJ32000 | NM_152498.1 | 404 | gccggaagcgcaaagttcttaat | 417164 | - | see also 21782 line |
| 49178 | FLJ32001 | NM_152609.1 | 218 | gacagcgatactcctacatatta | 418763 | - | see also 9899 line |
| 49179 | FLJ32001 | NM_152609.1 | 945 | cagttacggctctaaggaattca | 418780 | - | see also 9899 line |
| 49180 | FLJ32011 | NM_182516.1 | 534 | ttgcctgttcaatctactgatt | 434051 | - | see also 21784 line |
| 49181 | FLJ32011 | NM_182516.1 | 531 | ttgttgcctgttcaatctactga | 434050 | - | see also 21784 line |
| 49182 | FLJ32028 | NM_152680.1 | 584 | gccgacttgaatgtttacctac | 419696 | - | see also 21785 line |
| 49183 | FLJ32028 | NM_152680.1 | 342 | tggtgttaatccattgattta | 419689 | - | see also 21785 line |
| 49184 | FLJ32063 | NM_153031.1 | 964 | accccgtcacgttaagcaacc | 421980 | - | see also 21786 line |
| 49185 | FLJ32063 | NM_153031.1 | 777 | ccaggtaatgcgcttcattatt | 421978 | - | see also 21786 line |
| 49186 | FLJ32063 | NM_153031.1 | 1117 | aacttagtccttgatttcgaagg | 421984 | - | see also 21786 line |
| 49187 | FLJ32065 | NM_153032.1 | 443 | ctcccgcagcttcctagattag | 421986 | - | - |
| 49188 | FLJ32065 | NM_153032.1 | 348 | tccggttggcccgagatgttct | 421985 | - | see also 49187 line |
| 49189 | FLJ32096 | NM_173795.2 | 548 | gtcggggtcaccgtccaaaagc | 427981 | - | - |
| 49190 | FLJ32096 | NM_173795.2 | 328 | ggctgtgcagagcaatacgaag | 427979 | - | see also 49189 line |
| 49191 | FLJ32096 | NM_173795.2 | 527 | ccctcccagtaggaaagatgt | 427980 | - | see also 49189 line |
| 49192 | FLJ32110 | NM_181646.2 | 2142 | aaccaaagacgatgatagctaat | 432714 | - | see also 21787 line |
| 49193 | FLJ32110 | NM_181646.2 | 2143 | accaaagacgatgatagctaatc | 432715 | - | see also 21787 line |
| 49194 | FLJ32112 | NM_153035.1 | 273 | tacaagcaggccacgattgaatc | 421993 | - | see also 21788 line |

Figure 46

| 49195 | FLJ32112 | NM_153035.1 | 585 | gagtcgttgaggaaaaatgctca | 422008 | - | see also 21788 line |
|---|---|---|---|---|---|---|---|
| 49196 | FLJ32206 | NM_152497.1 | 1125 | tggctccctggtgattgtatta | 417149 | - | see also 21789 line |
| 49197 | FLJ32206 | NM_152497.1 | 942 | tggaatacaaccacttacagact | 417147 | - | see also 21789 line |
| 49198 | FLJ32252 | NM_182510.1 | 1580 | ccggatcagcgtggtccaaaagg | 434034 | - | see also 21790 line |
| 49199 | FLJ32252 | NM_182510.1 | 839 | aggtgggacccccgatctagaga | 434031 | - | see also 21790 line |
| 49200 | FLJ32312 | NM_144709.1 | 1448 | tgcgagctcgcgtcattcacttc | 411548 | - | see also 21791 line |
| 49201 | FLJ32312 | NM_144709.1 | 761 | ctgcgatatgcccagattgtttt | 411536 | - | see also 21791 line |
| 49202 | FLJ32334 | NM_144565.1 | 923 | caccccaaagatcctgattgtgc | 409682 | - | see also 21792 line |
| 49203 | FLJ32334 | NM_144565.1 | 219 | ttcgggtggtgaccagcttattc | 409678 | - | see also 21792 line |
| 49204 | FLJ32343 | NM_152434.1 | 1781 | tacccaacgtacggtctcttact | 416797 | - | see also 21793 line |
| 49205 | FLJ32343 | NM_152434.1 | 439 | gacattgtagccgaaagatatgg | 416771 | - | see also 21793 line |
| 49206 | FLJ32363 | NM_198566.1 | 522 | cagcgagccctagattttcgaaa | 439614 | - | see also 10343 line |
| 49207 | FLJ32363 | NM_198566.1 | 1600 | gccataactatcgtatatgctgc | 439646 | - | see also 10343 line |
| 49208 | FLJ32363 | NM_198566.1 | 523 | agcgagccctagattttcgaaac | 439615 | - | see also 10343 line |
| 49209 | FLJ32384 | NM_144608.1 | 255 | aaccagaccgcctgtaatgcaga | 410278 | - | see also 21795 line |
| 49210 | FLJ32384 | NM_144608.1 | 1010 | gaggctccggaccgaaaaccagc | 410282 | - | see also 21795 line |
| 49211 | FLJ32416 | NM_144616.2 | 551 | atcgtcgcccctgcctaagttcg | 410343 | - | see also 21796 line |
| 49212 | FLJ32416 | NM_144616.2 | 392 | gggagacctgtcgctcaacaagt | 410341 | - | see also 21796 line |
| 49213 | FLJ32421 | NM_144695.1 | 928 | gactcatagaagcatacgttatt | 411359 | - | see also 21797 line |
| 49214 | FLJ32421 | NM_144695.1 | 923 | gtcacgactcatagaagcatacg | 411358 | - | see also 21797 line |
| 49215 | FLJ32440 | NM_173685.1 | 439 | tggatcggcaactaaaccattat | 427403 | - | see also 10110 line |
| 49216 | FLJ32440 | NM_173685.1 | 801 | gacgccattgttcgcatgattga | 427412 | - | see also 10110 line |
| 49217 | FLJ32447 | NM_153038.1 | 487 | acccgcgttcctcgagttttaaa | 422022 | - | see also 21799 line |
| 49218 | FLJ32447 | NM_153038.1 | 488 | cccgcgttcctcgagttttaaaa | 422023 | - | see also 21799 line |
| 49219 | FLJ32447 | NM_153038.1 | 597 | ggcgcgatctggccagttcttgg | 422028 | - | see also 21799 line |
| 49220 | FLJ32535 | NM_152547.1 | 442 | ttccggagtcagaccttcaatgt | 417902 | - | - | - | - |
| 49221 | FLJ32535 | NM_152547.1 | 592 | ccctctgacaagggcacatatgg | 417903 | - | see also 49220 line |
| 49222 | FLJ32535 | NM_152547.1 | 839 | acctgttcagtctggaaacatct | 417904 | - | see also 49220 line |
| 49223 | FLJ32549 | NM_152440.1 | 214 | cggccgagaaggtctatcacagc | 416812 | - | see also 9866 line |
| 49224 | FLJ32549 | NM_152440.1 | 1217 | cccggaacctcactttaccattg | 416833 | - | see also 9866 line |
| 49225 | FLJ32642 | NM_152415.1 | 912 | taccgacaagccaaaatggtttt | 416467 | - | see also 21801 line |
| 49226 | FLJ32642 | NM_152415.1 | 394 | ctccagtatagccgaaatacaga | 416444 | - | see also 21801 line |
| 49227 | FLJ32642 | NM_152415.1 | 523 | tccaccaatacgacatcacttaa | 416452 | - | see also 21801 line |
| 49228 | FLJ32670 | NM_144612.3 | 1135 | ctcccgcttcatcgtagagttag | 410310 | - | see also 21802 line |
| 49229 | FLJ32670 | NM_144612.3 | 398 | aacgagatcacgtactactttcc | 410298 | - | see also 21802 line |
| 49230 | FLJ32675 | NM_173811.2 | 304 | accggacattggaccgttttaag | 428217 | - | see also 21803 line |
| 49231 | FLJ32675 | NM_173811.2 | 308 | gacattggaccgttttaagctgg | 428219 | - | see also 21803 line |

Figure 46

| | | | | | | | |
|---|---|---|---|---|---|---|---|
| 49232 | FLJ32675 | NM_173811.2 | 531 | atcagtcaggcggtctatgagtcg | 428227 | - | see also 21803 line |
| 49233 | FLJ32682 | NM_182542.1 | 988 | tacgacagaactgccgtaaaatc | 434290 | - | see also 21804 line |
| 49234 | FLJ32682 | NM_182542.1 | 2164 | ttcatagaggccgtcagtatatc | 434312 | - | see also 21804 line |
| 49235 | FLJ32702 | NM_152337.1 | 435 | tgcatctggccgaggaagataac | 415705 | - | see also 21805 line |
| 49236 | FLJ32702 | NM_152337.1 | 322 | ttctcgatgtcagtgacattacg | 415701 | - | see also 21805 line |
| 49237 | FLJ32734 | NM_144681.1 | 306 | ctccagaaactccccaatgttga | 411286 | - | see also 21806 line |
| 49238 | FLJ32734 | NM_144681.1 | 913 | accgtgcccgcagcaatgtcatc | 411289 | - | see also 21806 line |
| 49239 | FLJ32742 | NM_152580.1 | 485 | tccccagtatggtccatttaaaa | 418252 | - | see also 21807 line |
| 49240 | FLJ32742 | NM_152580.1 | 586 | ctgcaagaccctacatggtttc | 418253 | - | see also 21807 line |
| 49241 | FLJ32743 | NM_145020.2 | 856 | ggcacgccttgtggaaagtaaca | 412771 | - | see also 21808 line |
| 49242 | FLJ32743 | NM_145020.2 | 858 | cacgccttgtggaaagtaacaac | 412772 | - | see also 21808 line |
| 49243 | FLJ32745 | NM_144978.1 | 485 | gaactcggactgagtgttgtagt | 412227 | - | see also 21809 line |
| 49244 | FLJ32745 | NM_144978.1 | 141 | ctgccccgacgagtatgatttt | 412212 | - | see also 21809 line |
| 49245 | FLJ32745 | NM_144978.1 | 402 | tagagttagtacctcgaaaataa | 412224 | - | see also 21809 line |
| 49246 | FLJ32762 | NM_145023.3 | 1256 | aagcccactcaactgatgaattt | 412824 | - | see also 21810 line |
| 49247 | FLJ32762 | NM_145023.3 | 578 | aaggactacataatttaccatta | 412809 | - | see also 21810 line |
| 49248 | FLJ32770 | NM_182525.1 | 460 | aagaactgcacagctttttgagg | 434165 | - | - |
| 49249 | FLJ32770 | NM_182525.1 | 251 | accttgtcctcgcactcaatga | 434164 | - | see also 49248 line |
| 49250 | FLJ32771 | NM_145017.1 | 227 | agcctacggtcgggagagatttca | 412668 | - | see also 21811 line |
| 49251 | FLJ32771 | NM_145017.1 | 645 | ttcatgacgtcggagtacaattc | 412670 | - | see also 21811 line |
| 49252 | FLJ32779 | NM_173521.1 | 784 | atgagccgtcaagtttcttatt | 425405 | - | see also 21812 line |
| 49253 | FLJ32779 | NM_173521.1 | 1471 | tgcctacgaaagtgcttcaatta | 425427 | - | see also 21812 line |
| 49254 | FLJ32783 | NM_144968.1 | 520 | aagaacgggcgtgaatttagtct | 412001 | - | see also 21813 line |
| 49255 | FLJ32783 | NM_144968.1 | 569 | ggcttccaactatcttagttac | 412006 | - | see also 21813 line |
| 49256 | FLJ32784 | NM_144623.1 | 952 | ctgcaacattagcaaataca | 410443 | - | see also 21814 line |
| 49257 | FLJ32784 | NM_144623.1 | 1254 | atcaaagttctctcaacttct | 410445 | - | see also 21814 line |
| 49258 | FLJ32785 | NM_152494.1 | 1763 | taccgattgcctgttagtgtgt | 417126 | - | see also 21815 line |
| 49259 | FLJ32785 | NM_152494.1 | 495 | agccaatctgcgacacaatctca | 417110 | - | see also 21815 line |
| 49260 | FLJ32785 | NM_152494.1 | 596 | ccccttacgggccatgttcaag | 417111 | - | see also 21815 line |
| 49261 | FLJ32786 | NM_144648.1 | 90 | ctggagccgatcgttacagttt | 410727 | - | see also 21816 line |
| 49262 | FLJ32786 | NM_144648.1 | 2353 | gacccggaaagcactacctatac | 410804 | - | see also 21816 line |
| 49263 | FLJ32796 | NM_153606.1 | 680 | gccaccggctgcgaagagtatgc | 423296 | - | see also 21817 line |
| 49264 | FLJ32796 | NM_153606.1 | 1862 | cacagggactgccataaaggtgt | 423312 | - | see also 21817 line |
| 49265 | FLJ32800 | NM_152647.1 | 950 | aaccaagattgcttattcgatag | 419473 | - | see also 21818 line |
| 49266 | FLJ32800 | NM_152647.1 | 1300 | aactagtatcgacttcaatatta | 419490 | - | see also 21818 line |
| 49267 | FLJ32825 | NM_152492.1 | 429 | cacggcttcattgtccaaatgg | 417084 | - | see also 21819 line |
| 49268 | FLJ32825 | NM_152492.1 | 1947 | gagaaggcgactactataatcagc | 417098 | - | see also 21819 line |

Figure 46

| | | | | | | | |
|---|---|---|---|---|---|---|---|
| 49269 | FLJ32827 | NM_173081.1 | 623 | gtgtcgagctaaacttcaagaac | 424624 | - | see also 21820 line |
| 49270 | FLJ32827 | NM_173081.1 | 1867 | acctacgggctgtactcttaatc | 424676 | - | see also 21820 line |
| 49271 | FLJ32830 | NM_152781.1 | 1767 | tgctcgaaaccacgaaaacatag | 421124 | - | see also 21821 line |
| 49272 | FLJ32830 | NM_152781.1 | 1766 | ctgctcgaaaccacgaaaacata | 421123 | - | see also 21821 line |
| 49273 | FLJ32830 | NM_152781.1 | 1764 | tgctgctcgaaaccacgaaaaca | 421122 | - | see also 21821 line |
| 49274 | FLJ32831 | NM_153040.1 | 737 | aagctcacgagtggatggtttga | 422038 | - | see also 21822 line |
| 49275 | FLJ32831 | NM_153040.1 | 541 | aaggggtcccacctaataggaag | 422034 | - | see also 21822 line |
| 49276 | FLJ32844 | NM_173543.1 | 995 | agctacgggccaagctaaagtgg | 425754 | - | see also 21823 line |
| 49277 | FLJ32844 | NM_173543.1 | 2435 | gaggggtcagtctgttttttatg | 425763 | - | see also 21823 line |
| 49278 | FLJ32844 | NM_173543.1 | 1207 | gccaagcagaactctacactaga | 425755 | - | see also 21823 line |
| 49279 | FLJ32855 | NM_182791.1 | 846 | ctctggtacttcagacaagttca | 435293 | - | see also 21824 line |
| 49280 | FLJ32855 | NM_182791.1 | 305 | gagggacagggtgcagcatttgc | 435290 | - | see also 21824 line |
| 49281 | FLJ32859 | NM_152539.1 | 364 | aacaccgtctgaacagactaaag | 417770 | - | see also 21825 line |
| 49282 | FLJ32859 | NM_152539.1 | 1089 | cagacagacaagctaatcataaa | 417787 | - | see also 21825 line |
| 49283 | FLJ32880 | NM_152762.1 | 1101 | gcctatgcctcgggatacgatga | 420821 | - | see also 21826 line |
| 49284 | FLJ32880 | NM_152762.1 | 37 | ggcaggacaccgatatgctaaat | 420815 | - | see also 21826 line |
| 49285 | FLJ32884 | NM_144702.1 | 1607 | cccctatcagctgtcatactaga | 411491 | - | see also 21827 line |
| 49286 | FLJ32884 | NM_144702.1 | 676 | gtctctgcggaacaataacatcg | 411479 | - | see also 21827 line |
| 49287 | FLJ32884 | NM_144702.1 | 1383 | agcaccgagatgggaaagttttc | 411486 | - | see also 21827 line |
| 49288 | FLJ32894 | NM_144667.1 | 251 | tccggagcctccactttgactgg | 411150 | - | - | - | - |
| 49289 | FLJ32915 | NM_145014.1 | 1167 | gaccgggtagcccggtattttga | 412637 | - | see also 21828 line |
| 49290 | FLJ32915 | NM_145014.1 | 662 | agcccaatctatccaatatgatc | 412623 | - | see also 21828 line |
| 49291 | FLJ32926 | NM_144577.1 | 758 | gccccctgacactctagaagacc | 409906 | - | see also 21829 line |
| 49292 | FLJ32926 | NM_144577.1 | 255 | gagatcgaggagcgcaactttgc | 409905 | - | see also 21829 line |
| 49293 | FLJ32934 | NM_144622.1 | 1461 | tacgctgggaatatttatcgtga | 410427 | - | see also 21830 line |
| 49294 | FLJ32934 | NM_144622.1 | 702 | aagtgtgcacgggtttttcgatga | 410415 | - | see also 21830 line |
| 49295 | FLJ32934 | NM_144622.1 | 1938 | cccggctgccaaggatttatatg | 410438 | - | see also 21830 line |
| 49296 | FLJ32940 | NM_144696.2 | 1797 | ttcggtactccaagcgtatatat | 411428 | - | see also 21831 line |
| 49297 | FLJ32940 | NM_144696.2 | 742 | gtgatggaccagcgcattttaga | 411392 | - | see also 21831 line |
| 49298 | FLJ32940 | NM_144696.2 | 1403 | ttctactgtcaaaatacgatact | 411415 | - | see also 21831 line |
| 49299 | FLJ32942 | NM_144594.1 | 95 | ttgcaatgcccctatgacaaaaa | 410096 | - | see also 21832 line |
| 49300 | FLJ32942 | NM_144594.1 | 141 | ttccttatcatcttatcaagtgc | 410100 | - | see also 21832 line |
| 49301 | FLJ32949 | NM_173812.3 | 1844 | ttcgcagagttcgttttgagaag | 428245 | - | see also 21833 line |
| 49302 | FLJ32949 | NM_173812.3 | 1850 | gagttcgttttgagaaggttatc | 428246 | - | see also 21833 line |
| 49303 | FLJ32949 | NM_173812.3 | 677 | atctttatccagaggtaatcata | 428244 | - | see also 21833 line |
| 49304 | FLJ32954 | NM_144713.1 | 501 | atcccgtaaactaagtatagttt | 411631 | - | see also 21834 line |
| 49305 | FLJ32954 | NM_144713.1 | 496 | ttctcatcccgtaaactaagtat | 411629 | - | see also 21834 line |

Figure 46

EP 1 752 536 A1

| | | | | | | | |
|---|---|---|---|---|---|---|---|
| 49306 | FLJ32955 | NM_153041.1 | 237 | ctgtcctcttatccaagcttttc | 422045 | - | see also 21835 line |
| 49307 | FLJ32955 | NM_153041.1 | 211 | ttctcagtcagatgtttagctca | 422044 | - | see also 21835 line |
| 49308 | FLJ32965 | NM_182506.1 | 935 | ggggttatatgacggaattgagc | 434009 | - | see also 21836 line |
| 49309 | FLJ32965 | NM_182506.1 | 1241 | cggtgcacgttccaaggttaagt | 434011 | - | see also 21836 line |
| 49310 | FLJ33008 | NM_152449.2 | 593 | gagaacaagacttatatgctttg | 416881 | - | see also 21837 line |
| 49311 | FLJ33008 | NM_152449.2 | 552 | tggcaaagttgcagatatcaaga | 416879 | - | see also 21837 line |
| 49312 | FLJ33069 | NM_144649.1 | 425 | gaccagcgttatgtataaggaga | 410820 | - | see also 9725 line |
| 49313 | FLJ33069 | NM_144649.1 | 301 | ccctgacaggctcccactatacc | 410816 | - | see also 9725 line |
| 49314 | FLJ33084 | NM_152500.1 | 964 | ggcgcttggaggcagaaatcttg | 417222 | - | see also 21839 line |
| 49315 | FLJ33084 | NM_152500.1 | 1212 | ctgggccctgcaccctatgatcc | 417224 | - | see also 21839 line |
| 49316 | FLJ33167 | NM_152683.1 | 1085 | ttcggctatataaatcatcaaaa | 419777 | - | see also 21840 line |
| 49317 | FLJ33167 | NM_152683.1 | 1055 | atctcggagtttatacaagaaat | 419773 | - | see also 21840 line |
| 49318 | FLJ33167 | NM_152683.1 | 257 | aacgagcatctcattatgagagg | 419748 | - | see also 21840 line |
| 49319 | FLJ33207 | NM_178554.2 | 1190 | cccgacgctgttcatgttcatgc | 431221 | - | see also 21841 line |
| 49320 | FLJ33207 | NM_178554.2 | 1189 | ccccgacgctgttcatgttcatg | 431220 | - | see also 21841 line |
| 49321 | FLJ33207 | NM_178554.2 | 384 | ttctccttggctaaacgtttaca | 431207 | - | see also 21841 line |
| 49322 | FLJ33215 | NM_148894.1 | 1592 | aacgacgacagtccattggtatt | 415155 | - | see also 21842 line |
| 49323 | FLJ33215 | NM_148894.1 | 1521 | tgcgtatgtccacaaaccatatc | 415151 | - | see also 21842 line |
| 49324 | FLJ33318 | NM_153229.1 | 384 | cccctacagtgaggtgattctga | 422386 | - | - | - | - |
| 49325 | FLJ33318 | NM_153229.1 | 126 | tgcctgccccaaaggattcaaat | 422385 | - | see also 49324 line |
| 49326 | FLJ33387 | NM_182526.1 | 324 | gggagttcgtggtgaacttgaac | 434166 | - | see also 21844 line |
| 49327 | FLJ33496 | NM_182587.1 | 948 | caccgaggaaactcctttgatgg | 434542 | - | see also 21845 line |
| 49328 | FLJ33496 | NM_182587.1 | 781 | agcccatcaggaacatcattaca | 434537 | - | see also 21845 line |
| 49329 | FLJ33496 | NM_182587.1 | 184 | ggcccaaactggggaagcaatat | 434532 | - | see also 21845 line |
| 49330 | FLJ33516 | NM_152423.2 | 949 | cccacaatggtcgatactattcc | 416553 | - | see also 21846 line |
| 49331 | FLJ33516 | NM_152423.2 | 947 | cacccacaatggtcgatactatt | 416551 | - | see also 21846 line |
| 49332 | FLJ33534 | NM_182586.1 | 429 | agcaggtctcgtggataatattt | 434528 | - | see also 21847 line |
| 49333 | FLJ33534 | NM_182586.1 | 431 | caggtctcgtggataatatttct | 434529 | - | see also 21847 line |
| 49334 | FLJ33590 | NM_173821.1 | 1454 | ttcgcagtcttcgatgtcataaa | 428335 | - | see also 21848 line |
| 49335 | FLJ33590 | NM_173821.1 | 512 | tggagcgccaacgccacaaaagg | 428332 | - | see also 21848 line |
| 49336 | FLJ33600 | NM_198478.1 | 573 | ggctggtgtcgcgtgaaaagttc | 438451 | - | see also 21849 line |
| 49337 | FLJ33600 | NM_198478.1 | 531 | tgggcaagcacctgttcgtaagc | 438448 | - | see also 21849 line |
| 49338 | FLJ33610 | NM_173697.1 | 288 | gcctaagagcctggagatttata | 427698 | - | - | - | - |
| 49339 | FLJ33610 | NM_173697.1 | 287 | tgcctaagagcctggagatttat | 427697 | - | see also 49338 line |
| 49340 | FLJ33620 | NM_178824.3 | 1724 | ctcatgaggaccgaataagttcc | 431760 | - | see also 21850 line |
| 49341 | FLJ33620 | NM_178824.3 | 1372 | accacgggagtccgcaactttga | 431744 | - | see also 21850 line |
| 49342 | FLJ33641 | NM_152687.1 | 714 | aaccgataaggaactatatgaaa | 419810 | - | see also 21851 line |

Figure 46

| 49343 | FLJ33641 | NM_152687.1 | 747 | gtctaatttcgaggagcatatct | 419812 | - | see also 21851 line |
|---|---|---|---|---|---|---|---|
| 49344 | FLJ33641 | NM_152687.1 | 500 | accgtgttgccacacgatttacc | 419804 | - | see also 21851 line |
| 49345 | FLJ33706 | NM_182584.1 | 707 | cagtttctctccaattcatcacg | 434519 | - | see also 21853 line |
| 49346 | FLJ33708 | NM_173675.1 | 338 | tcctcttgcaagtgacttgaaag | 427314 | - | see also 21854 line |
| 49347 | FLJ33710 | NM_198480.1 | 41 | cactgtctcatggtacatcaacg | 438462 | - | see also 21855 line |
| 49348 | FLJ33710 | NM_198480.1 | 176 | tgcaccgaatgtcgaaaaggttt | 438463 | - | see also 21855 line |
| 49349 | FLJ33761 | NM_153211.1 | 1432 | ctctgtgtgttggcgtctattga | 422176 | - | see also 9976 line |
| 49350 | FLJ33761 | NM_153211.1 | 1672 | cagccgtatgcctgttatgaact | 422191 | - | see also 9976 line |
| 49351 | FLJ33768 | NM_173610.1 | 217 | ttggcaactcttccgtctatttt | 426493 | - | see also 21858 line |
| 49352 | FLJ33768 | NM_173610.1 | 218 | tggcaactcttccgtctattttg | 426494 | - | see also 21858 line |
| 49353 | FLJ33790 | NM_173583.1 | 464 | ccggctacactcgctcagaattc | 426298 | - | see also 21859 line |
| 49354 | FLJ33810 | NM_182558.1 | 486 | ctgggaaatccactaagactaga | 434447 | - | see also 21860 line |
| 49355 | FLJ33810 | NM_182558.1 | 346 | ctgacccagatcagttgttctct | 434445 | - | see also 21860 line |
| 49356 | FLJ33814 | NM_173510.1 | 491 | ttccatacctgggatattagatg | 425233 | - | see also 10077 line |
| 49357 | FLJ33814 | NM_173510.1 | 496 | tacctgggatattagatgttatt | 425235 | - | see also 10077 line |
| 49358 | FLJ33814 | NM_173510.1 | 492 | tccatacctgggatattagatgt | 425234 | - | see also 10077 line |
| 49359 | FLJ33817 | NM_152348.1 | 814 | tccgcctgctgacgtcttgttat | 415757 | - | see also 21862 line |
| 49360 | FLJ33817 | NM_152348.1 | 815 | ccgcctgctgacgtcttgttatg | 415758 | - | see also 21862 line |
| 49361 | FLJ33860 | NM_173644.1 | 602 | tgcaaagttctgcaccaaataaa | 426943 | - | see also 21863 line |
| 49362 | FLJ33860 | NM_173644.1 | 646 | atgccatgtttccaaaacctact | 426944 | - | see also 21863 line |
| 49363 | FLJ33915 | NM_182613.1 | 1566 | agcgacctaacaaatacatatgg | 434683 | - | see also 21864 line |
| 49364 | FLJ33915 | NM_182613.1 | 1563 | tccagcgacctaacaaatacata | 434681 | - | see also 21864 line |
| 49365 | FLJ33962 | NM_153213.2 | 523 | gccacggctcggagaagaagtct | 422198 | - | see also 21865 line |
| 49366 | FLJ33962 | NM_153213.2 | 2287 | gcccccaggttcagtgtgttagg | 422204 | - | see also 21865 line |
| 49367 | FLJ33977 | NM_173666.1 | 669 | ttcggatgcagccgactaataga | 427233 | - | see also 21866 line |
| 49368 | FLJ33977 | NM_173666.1 | 356 | ttgcgtacagttcctctactagc | 427215 | - | see also 21866 line |
| 49369 | FLJ34047 | NM_173669.1 | 123 | ctcccgacgtcgactacgattcc | 427262 | - | see also 21867 line |
| 49370 | FLJ34064 | NM_152633.1 | 2543 | gtcgtcgcggctgctttatcaga | 419406 | - | see also 21868 line |
| 49371 | FLJ34064 | NM_152633.1 | 1976 | aactgtcctaaagatcgttatgt | 419384 | - | see also 21868 line |
| 49372 | FLJ34154 | NM_173813.2 | 1577 | aactggttggccgaaagacttgg | 428294 | - | see also 21869 line |
| 49373 | FLJ34154 | NM_173813.2 | 1845 | gagctattgcggtccaaactttt | 428297 | - | see also 21869 line |
| 49374 | FLJ34278 | NM_173602.1 | 433 | gccagggatgaacgatatcgatc | 426458 | - | see also 21870 line |
| 49375 | FLJ34278 | NM_173602.1 | 483 | tgcactggcaaagcataaagaac | 426461 | - | see also 21870 line |
| 49376 | FLJ34283 | NM_182612.1 | 202 | tgcacgctgggtgcaagacttcc | 434676 | - | - | | |
| 49377 | FLJ34443 | NM_175918.2 | 1713 | gacgctgttaccgtacattctac | 429567 | - | see also 21871 line |
| 49378 | FLJ34443 | NM_175918.2 | 1786 | ctctgcacctcgagataacgtag | 429572 | - | see also 21871 line |
| 49379 | FLJ34503 | NM_173673.1 | 694 | caccctcactagcacttaatatt | 427310 | - | see also 21872 line |

Figure 46

| | | | | | | | |
|---|---|---|---|---|---|---|---|
| 49380 | FLJ34503 | NM_173673.1 | 692 | tacaccctcactagcacttaata | 427309 | - | see also 21872 line |
| 49381 | FLJ34503 | NM_173673.1 | 576 | ttggacgttgttacagattttct | 427307 | - | see also 21872 line |
| 49382 | FLJ34512 | NM_173476.2 | 456 | caccagatctagcgctcaataag | 424942 | - | see also 21873 line |
| 49383 | FLJ34512 | NM_173476.2 | 457 | accagatctagcgctcaataagg | 424943 | - | see also 21873 line |
| 49384 | FLJ34512 | NM_173476.2 | 233 | tgcaccctgggcctaatttcagc | 424941 | - | see also 21873 line |
| 49385 | FLJ34583 | NM_198276.1 | 261 | gccgtgttcagtgattccaatcg | 437422 | - | see also 21874 line |
| 49386 | FLJ34583 | NM_198276.1 | 501 | ctgggctacgtgggtaacctaca | 437427 | - | see also 21874 line |
| 49387 | FLJ34633 | NM_152365.1 | 1032 | acctcggagggtcgttcaactcg | 415823 | - | see also 21875 line |
| 49388 | FLJ34633 | NM_152365.1 | 984 | cgcggcatcattcgcattagtac | 415821 | - | see also 21875 line |
| 49389 | FLJ34633 | NM_152365.1 | 975 | cgcctggtacgcggcatcattcg | 415820 | - | see also 21875 line |
| 49390 | FLJ34690 | NM_182567.1 | 447 | ggccaaagtacatgactatctcc | 434479 | - | see also 21876 line |
| 49391 | FLJ34690 | NM_182567.1 | 338 | caccatgaagggttgtcatttgc | 434476 | - | see also 21876 line |
| 49392 | FLJ34766 | NM_178502.2 | 491 | ggcgagacttctgacatctatgt | 430917 | - | see also 21877 line |
| 49393 | FLJ34766 | NM_178502.2 | 1197 | accagcgtctcaccttcactatc | 430921 | - | see also 21877 line |
| 49394 | FLJ34780 | NM_173651.1 | 86 | aacgcctattcctaatggataga | 427066 | - | see also 21878 line |
| 49395 | FLJ34780 | NM_173651.1 | 338 | ctggctttaacggagaagatata | 427068 | - | see also 21878 line |
| 49396 | FLJ34790 | NM_173621.1 | 942 | cacgcgtcaatctatagttgtgg | 426604 | - | see also 21879 line |
| 49397 | FLJ34790 | NM_173621.1 | 940 | gacacgcgtcaatctatagttgt | 426603 | - | see also 21879 line |
| 49398 | FLJ34836 | NM_173668.1 | 462 | gtccgtaggcacttgctttaacc | 427253 | - | see also 21880 line |
| 49399 | FLJ34836 | NM_173668.1 | 475 | tgctttaaccgtgaagaattacc | 427255 | - | see also 21880 line |
| 49400 | FLJ34922 | NM_152270.1 | 2595 | ttcagggaaccttacgaattaag | 415252 | - | see also 21881 line |
| 49401 | FLJ34922 | NM_152270.1 | 1141 | accccaacgcccgataaccttca | 415237 | - | see also 21881 line |
| 49402 | FLJ34969 | NM_152678.1 | 525 | ttccgacaagttcgagataaaac | 419639 | - | see also 9911 line |
| 49403 | FLJ35093 | NM_198549.1 | 238 | ctccggtggctaaaaagttgttt | 439374 | - | see also 21883 line |
| 49404 | FLJ35093 | NM_198549.1 | 1810 | ttcgccgcactgagcttttaatg | 439413 | - | see also 21883 line |
| 49405 | FLJ35093 | NM_198549.1 | 1362 | accgatcactggggtagtactga | 439402 | - | see also 21883 line |
| 49406 | FLJ35119 | NM_175871.2 | 502 | ggcactgctccagcggtattttc | 429377 | - | see also 21884 line |
| 49407 | FLJ35119 | NM_175871.2 | 506 | ctgctccagcggtattttcctgc | 429378 | - | see also 21884 line |
| 49408 | FLJ35220 | NM_173627.2 | 855 | gagagtcctcagcactttgttga | 426639 | - | see also 21885 line |
| 49409 | FLJ35283 | NM_152702.1 | 531 | cacaggactgacgcatcacatct | 419896 | - | see also 21886 line |
| 49410 | FLJ35283 | NM_152702.1 | 593 | gtgggtctcaaggggaattcagc | 419898 | - | see also 21886 line |
| 49411 | FLJ35424 | NM_173661.1 | 660 | agcgacataggctcacaagtaga | 427147 | - | see also 21887 line |
| 49412 | FLJ35424 | NM_173661.1 | 440 | gagaaaacgttccctgaacatgc | 427144 | - | see also 21887 line |
| 49413 | FLJ35453 | NM_152600.1 | 1529 | cccgctgcccctcgcaaacatta | 418729 | - | see also 21888 line |
| 49414 | FLJ35453 | NM_152600.1 | 1535 | gcccctcgcaaacattaaggaag | 418732 | - | see also 21888 line |
| 49415 | FLJ35487 | NM_173820.1 | 173 | aggacccgaccatttagatcacc | 428326 | - | see also 21889 line |
| 49416 | FLJ35487 | NM_173820.1 | 405 | atggagggcttcagtttatttct | 428330 | - | see also 21889 line |

Figure 46

| | | | | | | | |
|---|---|---|---|---|---|---|---|
| 49417 | FLJ35700 | NM_178533.2 | 964 | cagtgtcaggcggtctttgtac | 431114 | - | - |
| 49418 | FLJ35700 | NM_178533.2 | 845 | acctccaaggactatttaagagc | 431113 | - | see also 49417 line |
| 49419 | FLJ35709 | NM_173589.1 | 848 | gcctcttacgcaagtccttacc | 426331 | - | see also 21890 line |
| 49420 | FLJ35709 | NM_173589.1 | 1175 | tacaggagcgagtacaaaactgt | 426338 | - | see also 21890 line |
| 49421 | FLJ35709 | NM_173589.1 | 1177 | caggagcgagtacaaaactgtga | 426339 | - | see also 21890 line |
| 49422 | FLJ35721 | NM_173684.1 | 546 | agcacgagtgaaattactgaaca | 427392 | - | see also 21891 line |
| 49423 | FLJ35721 | NM_173684.1 | 450 | tgggggactttgtataaaaaata | 427390 | - | see also 21891 line |
| 49424 | FLJ35725 | NM_152544.1 | 1126 | agcaaccgaagcgaaacagaga | 417875 | - | see also 21892 line |
| 49425 | FLJ35725 | NM_152544.1 | 706 | acgccaggtgcgggtgtgtaacc | 417869 | - | see also 21892 line |
| 49426 | FLJ35728 | NM_152610.1 | 693 | agcgaccaggttgagtcattagc | 418810 | - | see also 21893 line |
| 49427 | FLJ35728 | NM_152610.1 | 629 | ttctgtgccctcgcaaaagttca | 418807 | - | see also 21893 line |
| 49428 | FLJ35740 | NM_147195.1 | 448 | tcccgtctactttctttgtaga | 415093 | - | - |
| 49429 | FLJ35740 | NM_147195.1 | 447 | ctcccgtctactttctttgtag | 415092 | - | see also 49428 line |
| 49430 | FLJ35757 | NM_152598.1 | 445 | ttgagagatcccggttttctagc | 418662 | - | see also 21894 line |
| 49431 | FLJ35757 | NM_152598.1 | 2491 | cacaaaacgagctgatgaattca | 418699 | - | see also 21894 line |
| 49432 | FLJ35773 | NM_152599.1 | 469 | tggtcccaccgtagacaaaaat | 418702 | - | see also 21895 line |
| 49433 | FLJ35773 | NM_152599.1 | 1816 | tgcccgagacgcgaaaatcaag | 418726 | - | see also 21895 line |
| 49434 | FLJ35775 | NM_152418.1 | 680 | cacgcgatcactcttcagtac | 416501 | - | see also 21896 line |
| 49435 | FLJ35775 | NM_152418.1 | 678 | tccacgcgtatcactcttcagt | 416500 | - | see also 21896 line |
| 49436 | FLJ35779 | NM_152408.1 | 827 | ctggcgttccgtagtgcaaaagc | 416399 | - | see also 9859 line |
| 49437 | FLJ35779 | NM_152408.1 | 830 | gcgttccgtagtgcaaaagcagt | 416400 | - | see also 9859 line |
| 49438 | FLJ35779 | NM_152408.1 | 1032 | atgagggtgtatgtgcattaaa | 416403 | - | see also 9859 line |
| 49439 | FLJ35782 | NM_152631.1 | 1618 | gcggcaacgcattcttatagtgc | 419223 | - | see also 21898 line |
| 49440 | FLJ35782 | NM_152631.1 | 1305 | gaccggagcgtccatcctaaaag | 419213 | - | see also 21898 line |
| 49441 | FLJ35794 | NM_152617.2 | 665 | accgtcgaaaaggcgagtttatg | 418899 | - | see also 9903 line |
| 49442 | FLJ35794 | NM_152617.2 | 1444 | tgcctgtggtgccgaatggtacc | 418916 | - | see also 9903 line |
| 49443 | FLJ35801 | NM_153044.1 | 775 | caggcctcctcctagagatgtagt | 422054 | - | see also 21900 line |
| 49444 | FLJ35801 | NM_153044.1 | 777 | ggcctctctagagatgtagtct | 422055 | - | see also 21900 line |
| 49445 | FLJ35802 | NM_173538.1 | 1290 | cacgtgcacaatcattaccaaaa | 425741 | - | see also 21901 line |
| 49446 | FLJ35802 | NM_173538.1 | 209 | gccggagtatgagcaatatctta | 425706 | - | see also 21901 line |
| 49447 | FLJ35821 | NM_152589.1 | 898 | tccctaactaccccgactagtacc | 418438 | - | see also 21902 line |
| 49448 | FLJ35821 | NM_152589.1 | 1151 | atgcggagaatgcatcctatatc | 418450 | - | see also 21902 line |
| 49449 | FLJ35827 | NM_153265.1 | 2680 | ttccgatcgcgcattttcctgg | 422758 | - | see also 9983 line |
| 49450 | FLJ35827 | NM_153265.1 | 328 | gcctagccgggaccccaatctgaag | 422734 | - | see also 9983 line |
| 49451 | FLJ35834 | NM_178827.3 | 1945 | atccacctcacgccgcatatacc | 431932 | - | see also 21904 line |
| 49452 | FLJ35834 | NM_178827.3 | 1965 | accggtgtcgtaactgcattaac | 431933 | - | see also 21904 line |
| 49453 | FLJ35834 | NM_178827.3 | 1969 | gtgtcgtaactgcattaaccttc | 431935 | - | see also 21904 line |

Figure 46

| | | | | | | |
|---|---|---|---|---|---|---|
| 49454 | FLJ35838 | NM_173532.1 | 509 | gtccgtggtgccgaactgtatga | - | see also 21905 line |
| 49455 | FLJ35838 | NM_173532.1 | 1001 | atcaacgtgagctggactattgg | - | see also 21905 line |
| 49456 | FLJ35843 | NM_152591.1 | 736 | gagattgaagacctacgatttga | - | see also 21906 line |
| 49457 | FLJ35843 | NM_152591.1 | 1516 | atcaaccttcgcagtatttgc | - | see also 21906 line |
| 49458 | FLJ35866 | NM_178828.3 | 1192 | ccgaccaccgcacatgacatca | - | see also 21907 line |
| 49459 | FLJ35866 | NM_178828.3 | 3941 | tggcccaccaaggcagttatgg | - | see also 21907 line |
| 49460 | FLJ35880 | NM_153264.2 | 656 | ctctacacgtaaggatgacatgg | - | see also 21908 line |
| 49461 | FLJ35880 | NM_153264.2 | 1498 | ttgtaggaaccccaatctgagg | - | see also 21908 line |
| 49462 | FLJ35894 | NM_173616.1 | 911 | ctgagataattgccctatttgc | - | see also 10089 line |
| 49463 | FLJ35894 | NM_173616.1 | 713 | cacggttattcaggacagttatt | - | see also 10089 line |
| 49464 | FLJ35961 | NM_152372.2 | 2364 | cagctgcgaaccgcaaaatca | - | see also 21910 line |
| 49465 | FLJ35961 | NM_152372.2 | 2369 | cgccgaaccgcaaaatcaattt | - | see also 21910 line |
| 49466 | FLJ35961 | NM_152372.2 | 2322 | agccgctgagctacatctaatct | - | see also 21910 line |
| 49467 | FLJ35989 | NM_152597.3 | 1542 | ttccaggtccatcatctctaaat | - | see also 9898 line |
| 49468 | FLJ35989 | NM_152597.3 | 1641 | agcttctaaaggctactatctca | - | see also 9898 line |
| 49469 | FLJ36004 | NM_152590.1 | 276 | tggaatgccttgttactatc | - | see also 21913 line |
| 49470 | FLJ36004 | NM_152590.1 | 1187 | ctctgattgaaccaccacaataca | - | see also 21913 line |
| 49471 | FLJ36046 | NM_152612.2 | 1436 | tccgcaagtacgagttcgaaaag | - | see also 21914 line |
| 49472 | FLJ36046 | NM_152612.2 | 892 | ttccgcaagcgagagttcaataa | - | see also 21914 line |
| 49473 | FLJ36046 | NM_152612.2 | 893 | tccgcaagcgagagttcaataag | - | see also 21914 line |
| 49474 | FLJ36090 | NM_153223.1 | 1689 | cacgaggatcgaataataagtga | - | see also 21915 line |
| 49475 | FLJ36090 | NM_153223.1 | 1437 | cagatgagactacgttaccttgc | - | see also 21915 line |
| 49476 | FLJ36102 | NM_173590.1 | 703 | gtgaaacttgagcatctcatt | - | see also 21916 line |
| 49477 | FLJ36102 | NM_173590.1 | 859 | gtgcgcaaggtgaagagttttga | - | see also 21916 line |
| 49478 | FLJ36144 | NM_182561.1 | 364 | atcatacaatacgaattctcacg | - | see also 21917 line |
| 49479 | FLJ36155 | NM_147193.1 | 855 | ctggtcacctgtgtaaatggact | - | see also 21918 line |
| 49480 | FLJ36155 | NM_147193.1 | 1934 | cggcctcctcaccaatagtca | - | see also 21918 line |
| 49481 | FLJ36166 | NM_182634.1 | 796 | tcgcgtgtttagctacaaacg | - | - |
| 49482 | FLJ36166 | NM_182634.1 | 29 | ttcatatattgaaacatacctaag | - | see also 49481 line |
| 49483 | FLJ36175 | NM_152528.1 | 1413 | atcgcatcagatggctattcata | - | see also 21919 line |
| 49484 | FLJ36175 | NM_152528.1 | 258 | tactcgttacgtgactttactga | - | see also 21919 line |
| 49485 | FLJ36180 | NM_178556.3 | 827 | agctcggctttcgaatctcttga | - | see also 21920 line |
| 49486 | FLJ36180 | NM_178556.3 | 822 | gtcaaagctcggctttcgaatct | - | see also 21920 line |
| 49487 | FLJ36198 | NM_173801.2 | 220 | tgccctgcaaatcggatacatac | - | see also 21921 line |
| 49488 | FLJ36198 | NM_173801.2 | 320 | ttcaaacgagctgactttacc | - | see also 21921 line |
| 49489 | FLJ36335 | NM_173568.1 | 1049 | tgccggttatgtggtccttattg | - | see also 21922 line |
| 49490 | FLJ36335 | NM_173568.1 | 1050 | gccggttatgtggtccttattgt | - | see also 21922 line |

Figure 46

| | | | | | | | |
|---|---|---|---|---|---|---|---|
| 49491 | FLJ36335 | NM_173568.1 | 916 | gagccacgtgcaaatcaattgc | 426228 | - | see also 21922 line |
| 49492 | FLJ36445 | NM_153233.1 | 340 | acccagctggggcaaacactgt | 422422 | - | see also 21923 line |
| 49493 | FLJ36445 | NM_153233.1 | 99 | ctgcctctgggccctagacttgg | 422421 | - | see also 21923 line |
| 49494 | FLJ36525 | NM_147129.2 | 1875 | gcccttccgggactttgtgt | 415029 | - | see also 21924 line |
| 49495 | FLJ36525 | NM_147129.2 | 1873 | cagcccttccgggactttgtgt | 415028 | - | see also 21924 line |
| 49496 | FLJ36561 | NM_182520.1 | 737 | gaggcagagtagggaccattagg | 434086 | - | see also 21925 line |
| 49497 | FLJ36561 | NM_182520.1 | 1031 | atgagcaaagcccccacttcaagg | 434089 | - | see also 21925 line |
| 49498 | FLJ36576 | NM_173522.1 | 366 | ctgcgccgtggaccatcacttc | 425431 | - | see also 21926 line |
| 49499 | FLJ36576 | NM_173522.1 | 405 | gcggcctattgcggcaactactca | 425433 | - | see also 21926 line |
| 49500 | FLJ36600 | NM_182497.1 | 561 | cccggattatcagcgttacttca | 433958 | - | see also 21927 line |
| 49501 | FLJ36600 | NM_182497.1 | 337 | ctcctgccatgctacttactgg | 433957 | - | see also 21927 line |
| 49502 | FLJ36600 | NM_182497.1 | 708 | gtcccttcgccagctgttagaagg | 433960 | - | see also 21927 line |
| 49503 | FLJ36601 | NM_173695.1 | 1060 | gtgccatactgcagcaataatat | 427667 | - | see also 21928 line |
| 49504 | FLJ36601 | NM_173695.1 | 1197 | tactgacatgtgatatggaaaa | 427675 | - | see also 21928 line |
| 49505 | FLJ36664 | NM_173690.1 | 1433 | gtctaatagtgttgcgtataaga | 427495 | - | see also 10111 line |
| 49506 | FLJ36664 | NM_173690.1 | 1304 | tagtaacgggatattattaatg | 427491 | - | see also 10111 line |
| 49507 | FLJ36664 | NM_173690.1 | 649 | tggtagttaagaagttacgatat | 427459 | - | see also 10111 line |
| 49508 | FLJ36674 | NM_173622.1 | 303 | cgcttgccgaatccacgttatca | 426611 | - | see also 21930 line |
| 49509 | FLJ36674 | NM_173622.1 | 309 | cagaatccacgttatcaatgtgg | 426614 | - | see also 21930 line |
| 49510 | FLJ36760 | NM_198550.1 | 926 | tacgacggcagaaatattagtga | 439442 | - | see also 21931 line |
| 49511 | FLJ36760 | NM_198550.1 | 439 | atccaaatcgataactatcaga | 439424 | - | see also 21931 line |
| 49512 | FLJ36779 | NM_152571.1 | 740 | tgccgctcgcgagttcaaagaac | 418164 | - | see also 21932 line |
| 49513 | FLJ36779 | NM_152571.1 | 741 | gccgctcgcggagttcaaagaacc | 418165 | - | see also 21932 line |
| 49514 | FLJ36779 | NM_152571.1 | 653 | tccgcggaagaggtgtcattgt | 418163 | - | see also 21932 line |
| 49515 | FLJ36812 | NM_153260.1 | 628 | tagagacgttaagcctaaacaac | 422680 | - | see also 9979 line |
| 49516 | FLJ36812 | NM_153260.1 | 789 | aaccagattcgaagtatacctga | 422685 | - | see also 9979 line |
| 49517 | FLJ36874 | NM_152716.1 | 1809 | ttcatggcaacacgagaacttct | 420066 | - | see also 21934 line |
| 49518 | FLJ36874 | NM_152716.1 | 816 | caccggagcagtcatcaagatca | 420050 | - | see also 21934 line |
| 49519 | FLJ36878 | NM_178518.2 | 1612 | gccgggacgttgcctcactattt | 431059 | - | see also 21935 line |
| 49520 | FLJ36878 | NM_178518.2 | 774 | cccaggagcacgtgattcgatcc | 431053 | - | see also 21935 line |
| 49521 | FLJ36878 | NM_178518.2 | 1613 | ccgggacgttgcctcactatttt | 431060 | - | see also 21935 line |
| 49522 | FLJ36888 | NM_178830.2 | 414 | agcccccttgcaggcgaaattcg | 431981 | - | see also 21936 line |
| 49523 | FLJ36888 | NM_178830.2 | 866 | ggcttgggacggcgacaatgaca | 431983 | - | see also 21936 line |
| 49524 | FLJ36980 | NM_182598.1 | 166 | ggcgaacggagcagctgacaatgg | 434594 | - | see also 21937 line |
| 49525 | FLJ36980 | NM_182598.1 | 410 | tacgttacacattgagaaagtta | 434599 | - | see also 21937 line |
| 49526 | FLJ37045 | NM_175889.2 | 773 | ccggctcgtttaatatcactga | 429499 | - | - |
| 49527 | FLJ37099 | NM_198459.1 | 1047 | atctggttacgccgtataaag | 437892 | - | see also 21938 line |

Figure 46

| | | | | | | | |
|---|---|---|---|---|---|---|---|
| 49528 | FLJ37099 | NM_198459.1 | 927 | tagttcatacggataaccaaga | 437889 | - | see also 21938 line |
| 49529 | FLJ37118 | NM_173507.1 | 1820 | cagcctggtggagcttgaataac | 425205 | - | see also 21939 line |
| 49530 | FLJ37118 | NM_173507.1 | 1076 | gcctgaacgacctgaatcactc | 425202 | - | see also 21939 line |
| 49531 | FLJ37131 | NM_173687.1 | 524 | gtccacaacggcacttaacctgc | 427422 | - | - |
| 49532 | FLJ37131 | NM_173687.1 | 575 | tgccaaagacgctcactttctgg | 427423 | - | see also 49531 line |
| 49533 | FLJ37306 | NM_173497.1 | 397 | gacaaaaacagcgtacatctatg | 425158 | - | see also 21940 line |
| 49534 | FLJ37306 | NM_173497.1 | 287 | agctgttagcccgaagaaagaag | 425154 | - | see also 21940 line |
| 49535 | FLJ37357 | NM_173645.1 | 707 | cacatcttaacgttaaatgctgt | 426950 | - | see also 21941 line |
| 49536 | FLJ37357 | NM_173645.1 | 1010 | ctcgtgcctgattcgtatttga | 426957 | - | see also 21941 line |
| 49537 | FLJ37396 | NM_173671.1 | 1195 | tccgccaccggctgatagaaatg | 427290 | - | see also 21942 line |
| 49538 | FLJ37396 | NM_173671.1 | 1194 | ctccgccacggctgatagaaat | 427289 | - | see also 21942 line |
| 49539 | FLJ37538 | NM_173564.2 | 1939 | gggaccgatggggtgctttgc | 426186 | - | see also 21943 line |
| 49540 | FLJ37538 | NM_173564.2 | 1105 | accaagacactccttgtgaaat | 426184 | - | see also 21943 line |
| 49541 | FLJ37543 | NM_173667.1 | 101 | gtctgcgtaacttgttcagaaaa | 427242 | - | see also 21944 line |
| 49542 | FLJ37543 | NM_173667.1 | 96 | atgctgtcgctaacttgttca | 427241 | - | see also 21944 line |
| 49543 | FLJ37549 | NM_152605.1 | 1048 | cagagagccatcttagtcaaca | 418741 | - | see also 21945 line |
| 49544 | FLJ37549 | NM_152605.1 | 1212 | ttgattgttcaactcttaacacc | 418744 | - | see also 21945 line |
| 49545 | FLJ37587 | NM_173597.1 | 372 | tccgaaagtgcttcagctaatta | 426417 | - | see also 21946 line |
| 49546 | FLJ37587 | NM_173597.1 | 340 | gtcaacagtcggatagatgaagg | 426416 | - | see also 21946 line |
| 49547 | FLJ37659 | NM_173698.1 | 743 | agtctacgcatgaatcagaatc | 427708 | - | see also 21947 line |
| 49548 | FLJ37659 | NM_173698.1 | 716 | aagaaccgttcatacaaatcatc | 427705 | - | see also 21947 line |
| 49549 | FLJ37673 | NM_182591.1 | 278 | cccccaagaggtagtcttactct | 434561 | - | see also 21948 line |
| 49550 | FLJ37673 | NM_182591.1 | 420 | atgtctcaagacacagacataaaa | 434566 | - | see also 21948 line |
| 49551 | FLJ37794 | NM_173588.1 | 1336 | gacgcacacgcttaagattaagg | 426316 | - | see also 21949 line |
| 49552 | FLJ37794 | NM_173588.1 | 1332 | gtctgacgcacacgcttaagatt | 426314 | - | see also 21949 line |
| 49553 | FLJ37818 | NM_175916.2 | 684 | tacacccagccgggtgataaagc | 429564 | - | see also 21950 line |
| 49554 | FLJ37818 | NM_175916.2 | 383 | ctggtcctaagccagttagatt | 429558 | - | see also 21950 line |
| 49555 | FLJ37927 | NM_152623.1 | 295 | cgccaacgtactgattcagtta | 419075 | - | see also 21951 line |
| 49556 | FLJ37927 | NM_152623.1 | 296 | gccaacgtactcgattcagttaa | 419076 | - | see also 21951 line |
| 49557 | FLJ37927 | NM_152623.1 | 299 | aacgtactcgattcagttaatgc | 419077 | - | see also 21951 line |
| 49558 | FLJ37940 | NM_178534.2 | 431 | tccagagagccaggaacagttg | 431115 | - | - |
| 49559 | FLJ37953 | NM_152382.1 | 417 | gtcctcgagatgcccagtattta | 416010 | - | see also 21952 line |
| 49560 | FLJ37953 | NM_152382.1 | 420 | ctcgagatgcccagtatttatat | 416012 | - | see also 21952 line |
| 49561 | FLJ38101 | NM_153261.3 | 390 | aacggtattagcagaatacaata | 422700 | - | see also 21953 line |
| 49562 | FLJ38101 | NM_153261.3 | 446 | aacctaggcctcatgttcaatga | 422704 | - | see also 21953 line |
| 49563 | FLJ38101 | NM_153261.3 | 315 | cactctaataggcttgttctttg | 422699 | - | see also 21953 line |
| 49564 | FLJ38159 | NM_152723.1 | 325 | aggagcgagaaggctatgcttcg | 420094 | - | see also 21954 line |

Figure 46

| | | | | | | | |
|---|---|---|---|---|---|---|---|
| 49565 | FLJ38159 | NM_152723.1 | 554 | tgctccgcttcaaggatgaatac | 420098 | - | see also 21954 line |
| 49566 | FLJ38190 | NM_152335.1 | 1029 | aacagctacatcagtcagtatta | 415697 | - | see also 21955 line |
| 49567 | FLJ38190 | NM_152335.1 | 916 | cgcgcagccgcttcaaagtgttg | 415696 | - | see also 21955 line |
| 49568 | FLJ38288 | NM_173632.2 | 545 | gtccacaataccgtttagcaaca | 426901 | - | see also 21956 line |
| 49569 | FLJ38288 | NM_173632.2 | 553 | taccgtttagcaacaaacactca | 426902 | - | see also 21956 line |
| 49570 | FLJ38335 | NM_153228.1 | 921 | agcgtcaatgcagctgtagtaac | 422338 | - | see also 21957 line |
| 49571 | FLJ38335 | NM_153228.1 | 1069 | ttcaagtctcggcttacaatatg | 422341 | - | see also 21957 line |
| 49572 | FLJ38377 | NM_152698.1 | 1512 | tggggacgccactgtccatatgc | 419888 | - | see also 21958 line |
| 49573 | FLJ38377 | NM_152698.1 | 628 | gtgctttcggaacctattccaca | 419884 | - | see also 21958 line |
| 49574 | FLJ38426 | NM_173611.1 | 669 | ccgccgacgaatgttaatgaaac | 426515 | - | see also 21960 line |
| 49575 | FLJ38426 | NM_173611.1 | 883 | aagaccgcatgtgccattaataa | 426523 | - | see also 21960 line |
| 49576 | FLJ38464 | NM_173689.1 | 614 | acctggaggtcctgaatggtttc | 427438 | - | - | | - | | - |
| 49577 | FLJ38482 | NM_152681.1 | 280 | cactttagaccccgattccatcc | 419698 | - | see also 21961 line |
| 49578 | FLJ38482 | NM_152681.1 | 539 | accgaggctataacttgatctac | 419709 | - | see also 21961 line |
| 49579 | FLJ38482 | NM_152681.1 | 580 | aagagacttgcgttgatgataca | 419712 | - | see also 21961 line |
| 49580 | FLJ38564 | NM_152579.1 | 667 | ttgtcatagggattcgatttttc | 418241 | - | see also 21962 line |
| 49581 | FLJ38564 | NM_152579.1 | 673 | tagggattcgatttttcttatat | 418242 | - | see also 21962 line |
| 49582 | FLJ38568 | NM_153252.1 | 1758 | tgggaggtacgctatattgaaca | 422657 | - | see also 21963 line |
| 49583 | FLJ38568 | NM_153252.1 | 505 | agcgcaaacgtcagcatacttac | 422609 | - | see also 21963 line |
| 49584 | FLJ38607 | NM_152654.1 | 392 | gccatacgcctccgaaatcatct | 419517 | - | see also 21964 line |
| 49585 | FLJ38607 | NM_152654.1 | 549 | cccgtcgacgggtgaagatatcc | 419520 | - | see also 21964 line |
| 49586 | FLJ38607 | NM_152654.1 | 552 | gtcgacgggtgaagatatccacc | 419521 | - | see also 21964 line |
| 49587 | FLJ38608 | NM_153215.1 | 175 | tccatcagcgacctacatagtgg | 422205 | - | - | | - | | - |
| 49588 | FLJ38615 | NM_173528.1 | 773 | ccccgtcccggcaaatgctaaaa | 425515 | - | see also 21965 line |
| 49589 | FLJ38615 | NM_173528.1 | 588 | cactggtcaagtccttagatatg | 425509 | - | see also 21965 line |
| 49590 | FLJ38615 | NM_173528.1 | 633 | aacaggaggatttgacaacaaaa | 425510 | - | see also 21965 line |
| 49591 | FLJ38628 | NM_152267.2 | 511 | gagaatagaggggggatttcaagg | 415229 | - | see also 9823 line |
| 49592 | FLJ38716 | NM_152367.1 | 391 | cagcaaccaagacattagatttc | 415832 | - | see also 21967 line |
| 49593 | FLJ38716 | NM_152367.1 | 476 | ctcgtcacagtcccaataaaagg | 415836 | - | see also 21967 line |
| 49594 | FLJ38723 | NM_173805.2 | 149 | cactgagctcacgcttcaacagg | 428053 | - | see also 21968 line |
| 49595 | FLJ38723 | NM_173805.2 | 79 | ttcaagttgtactgattgacaag | 428052 | - | see also 21968 line |
| 49596 | FLJ38725 | NM_153218.1 | 1166 | cagactgtataccgatagttttt | 422245 | - | see also 21969 line |
| 49597 | FLJ38725 | NM_153218.1 | 1556 | atgtccgagatggccttaatttt | 422257 | - | see also 21969 line |
| 49598 | FLJ38736 | NM_182758.1 | 2725 | ttgcgagagtcagatactatagt | 435094 | - | see also 21970 line |
| 49599 | FLJ38736 | NM_182758.1 | 3058 | aagatacccgtcaatagtcaacc | 435109 | - | see also 21970 line |
| 49600 | FLJ38753 | NM_152375.1 | 1629 | ggcggtcaacggctttctctacc | 415958 | - | see also 21971 line |
| 49601 | FLJ38753 | NM_152375.1 | 1497 | cacggtgcgtgccaaggaaatct | 415955 | - | see also 21971 line |

Figure 46

| | | | | | | | |
|---|---|---|---|---|---|---|---|
| 49602 | FLJ38771 | NM_173511.1 | 1156 | tccttcctacccggatcttaca | 425262 | - | see also 21972 line |
| 49603 | FLJ38771 | NM_173511.1 | 1160 | tcctacccggatcttacactca | 425263 | - | see also 21972 line |
| 49604 | FLJ38773 | NM_178528.2 | 653 | agctcggagatgaattggttagg | 431072 | - | see also 21973 line |
| 49605 | FLJ38773 | NM_178528.2 | 652 | aagctcggagatgaattggttag | 431071 | - | see also 21973 line |
| 49606 | FLJ38792 | NM_178520.2 | 624 | atgtgatcgggcagctgatttat | 431066 | - | see also 21974 line |
| 49607 | FLJ38792 | NM_178520.2 | 766 | ctgcctctgtgagatgaagataa | 431068 | - | see also 21974 line |
| 49608 | FLJ38819 | NM_145865.1 | 99 | aggctaccaagcgagatctaaat | 414936 | - | see also 21975 line |
| 49609 | FLJ38819 | NM_145865.1 | 104 | accaagcgagatctaaatcttc | 414939 | - | see also 21975 line |
| 49610 | FLJ38944 | NM_152361.1 | 362 | tccatgattccggtcagattact | 415807 | - | see also 21976 line |
| 49611 | FLJ38944 | NM_152361.1 | 361 | ctccatgattccggtcagattac | 415806 | - | see also 21976 line |
| 49612 | FLJ38968 | NM_152316.1 | 189 | gtccagcttcgtactacaaatga | 415552 | - | see also 21977 line |
| 49613 | FLJ38968 | NM_152316.1 | 213 | tgccataaaacctactatactcg | 415554 | - | see also 21977 line |
| 49614 | FLJ38973 | NM_153689.3 | 557 | accggttccccaatagttatatt | 423526 | - | see also 21978 line |
| 49615 | FLJ38973 | NM_153689.3 | 556 | caccggttccccaatagttatat | 423525 | - | see also 21978 line |
| 49616 | FLJ38984 | NM_152374.1 | 535 | gggacccacctcctggattatgt | 415949 | - | see also 21979 line |
| 49617 | FLJ38984 | NM_152374.1 | 935 | ggctgtgcagcacttacaagtcc | 415950 | - | see also 21979 line |
| 49618 | FLJ39106 | NM_173629.1 | 360 | gccgcaatgactggtctatgtgg | 426684 | - | see also 21981 line |
| 49619 | FLJ39106 | NM_173629.1 | 468 | cggccaattcctccattgttatc | 426690 | - | see also 21981 line |
| 49620 | FLJ39237 | NM_198571.1 | 1115 | cacttggcgctatctcaacatcg | 439709 | - | see also 21982 line |
| 49621 | FLJ39237 | NM_198571.1 | 1302 | ctggtgaagggttatactgaaca | 439711 | - | see also 21982 line |
| 49622 | FLJ39267 | NM_173550.2 | 1063 | gaccttttacggcgacaaacaag | 425870 | - | see also 21983 line |
| 49623 | FLJ39267 | NM_173550.2 | 1689 | gaggcgtaccttgacagattacc | 425885 | - | see also 21983 line |
| 49624 | FLJ39370 | NM_152400.1 | 341 | acgaatagtggaaccagtaatag | 416281 | - | see also 9851 line |
| 49625 | FLJ39378 | NM_178314.2 | 1131 | gggcatcaagcgactgatcttta | 430699 | - | see also 21985 line |
| 49626 | FLJ39378 | NM_178314.2 | 1176 | agcgggactgataatagatgacc | 430702 | - | see also 21985 line |
| 49627 | FLJ39415 | NM_173681.1 | 771 | tccttcgatgcgtggattacaat | 427336 | - | see also 21986 line |
| 49628 | FLJ39415 | NM_173681.1 | 834 | ggccgttccacagcaaagtgacc | 427338 | - | see also 21986 line |
| 49629 | FLJ39415 | NM_173681.1 | 994 | ctgggacatccaggtgttttaca | 427339 | - | see also 21986 line |
| 49630 | FLJ39421 | NM_178519.2 | 1152 | tccctgcgcacgctttaatcaag | 431064 | - | - | - | - |
| 49631 | FLJ39421 | NM_178519.2 | 1149 | ttctccctgcgcacgctttaatc | 431063 | - | see also 49630 line |
| 49632 | FLJ39421 | NM_178519.2 | 1153 | ccctgcgcacgctttaatcaagg | 431065 | - | see also 49630 line |
| 49633 | FLJ39426 | NM_173609.1 | 889 | gagataaaaccaaggttaagtag | 426492 | - | see also 21987 line |
| 49634 | FLJ39426 | NM_173609.1 | 724 | ctctggctacaaaaaagaataga | 426491 | - | see also 21987 line |
| 49635 | FLJ39441 | NM_194285.1 | 399 | tggttacaatgggattcctattg | 436321 | - | see also 10307 line |
| 49636 | FLJ39441 | NM_194285.1 | 1274 | ttgctcgacccacagttagttct | 436335 | - | see also 10307 line |
| 49637 | FLJ39485 | NM_175920.2 | 833 | ctcctgtaggagcgttaagttgt | 429573 | - | - | - | - |
| 49638 | FLJ39485 | NM_175920.2 | 858 | cagctgccgagcccaacttttgt | 429574 | - | see also 49637 line |

Figure 46

| 49639 | FLJ39553 | NM_173549.1 | 563 | gaggccgagtctctaaaaggaaa | 425846 | - | see also 21989 line |
| 49640 | FLJ39553 | NM_173549.1 | 319 | aagccgtcctcccttacaaaagc | 425842 | - | see also 21989 line |
| 49641 | FLJ39553 | NM_173549.1 | 559 | gacagaggccgagtctctaaaag | 425845 | - | see also 21989 line |
| 49642 | FLJ39575 | NM_182597.1 | 462 | agccggatcgcagcatctgttcc | 434585 | - | see also 21990 line |
| 49643 | FLJ39575 | NM_182597.1 | 595 | ttcgtgatatttctaatagttca | 434589 | - | see also 21990 line |
| 49644 | FLJ39576 | NM_182592.1 | 266 | atcgggttacgcaggacaatttt | 434571 | - | see also 21991 line |
| 49645 | FLJ39576 | NM_182592.1 | 267 | tcgggttacgcaggacaattttt | 434572 | - | see also 21991 line |
| 49646 | FLJ39576 | NM_182592.1 | 269 | gggttacgcaggacaatttttc | 434574 | - | see also 21991 line |
| 49647 | FLJ39582 | NM_178315.2 | 389 | gacagtatcgcaatacagaaatt | 430708 | - | see also 21992 line |
| 49648 | FLJ39582 | NM_178315.2 | 277 | cacgaaagaacccgacaaaaacc | 430705 | - | see also 21992 line |
| 49649 | FLJ39599 | NM_173803.2 | 502 | tacgctggagtctgtggttttct | 428048 | - | - | - | - |
| 49650 | FLJ39599 | NM_173803.2 | 526 | tgggccaccttcatctgtttttc | 428049 | - | see also 49649 line |
| 49651 | FLJ39616 | NM_016534.2 | 152 | gtcgctctcctccgacatactca | 358049 | - | - | - | - |
| 49652 | FLJ39630 | NM_173688.1 | 403 | aaggacaccgatctaatgacatt | 427429 | - | see also 21993 line |
| 49653 | FLJ39630 | NM_173688.1 | 298 | cagtacagacctcgatacataat | 427426 | - | see also 21993 line |
| 49654 | FLJ39647 | NM_173625.2 | 229 | ttcaaaaccggactatagctacc | 426623 | - | see also 21994 line |
| 49655 | FLJ39647 | NM_173625.2 | 590 | atggagtattgtggtcaaaattc | 426631 | - | see also 21994 line |
| 49656 | FLJ39653 | NM_152684.1 | 383 | ctgcctgcccgctattttggttc | 419791 | - | see also 21995 line |
| 49657 | FLJ39653 | NM_152684.1 | 379 | ggccctgcctgcccgctattttg | 419789 | - | see also 21995 line |
| 49658 | FLJ39660 | NM_173646.1 | 440 | atcgaatgtgccgtcttgaaagc | 426984 | - | see also 21996 line |
| 49659 | FLJ39660 | NM_173646.1 | 269 | cactgagggatgacctaataatg | 426981 | - | see also 21996 line |
| 49660 | FLJ39743 | NM_182562.1 | 399 | gagcggctgttgggttttacaca | 434456 | - | see also 21997 line |
| 49661 | FLJ39743 | NM_182562.1 | 652 | tgcgaggacttctcagaatgaca | 434459 | - | see also 21997 line |
| 49662 | FLJ39821 | NM_173700.1 | 807 | ctcactcaagctgcataccattg | 427735 | - | see also 21998 line |
| 49663 | FLJ39821 | NM_173700.1 | 756 | ctccaccgaagaataaggtttta | 427733 | - | see also 21998 line |
| 49664 | FLJ39827 | NM_152424.1 | 2161 | gcccgagaggttcgttgtagaga | 416611 | - | see also 21999 line |
| 49665 | FLJ39827 | NM_152424.1 | 323 | ctgggagtacccgtgaacaaaca | 416595 | - | see also 21999 line |
| 49666 | FLJ39873 | NM_173799.2 | 507 | tcgtggtggtcgcgttgactaga | 427986 | - | see also 22000 line |
| 49667 | FLJ39873 | NM_173799.2 | 336 | acgatacaggggagtacttctgc | 427983 | - | see also 22000 line |
| 49668 | FLJ40089 | NM_182496.1 | 596 | tgctcgagtatgctttgtcaacc | 433936 | - | see also 22001 line |
| 49669 | FLJ40089 | NM_182496.1 | 1585 | gacggcctcaacccaattcaaaa | 433949 | - | see also 22001 line |
| 49670 | FLJ40126 | NM_173599.1 | 366 | cacaaggcatggactcatatagt | 426432 | - | see also 22002 line |
| 49671 | FLJ40126 | NM_173599.1 | 972 | gtgtagtcactagttctgataaa | 426444 | - | see also 22002 line |
| 49672 | FLJ40160 | NM_173484.2 | 457 | gcccgtaggagagcccaatattc | 424994 | - | see also 22003 line |
| 49673 | FLJ40160 | NM_173484.2 | 458 | cccgtaggagagcccaatattcc | 424995 | - | see also 22003 line |
| 49674 | FLJ40160 | NM_173484.2 | 492 | ggcccttcggtgggaatctaagg | 424998 | - | see also 22003 line |
| 49675 | FLJ40172 | NM_173649.1 | 239 | cagtgatagcaacctacaatttt | 427058 | - | see also 22004 line |

Figure 46

| | | | | | | | |
|---|---|---|---|---|---|---|---|
| 49676 | FLJ40172 | NM_173649.1 | 226 | tccctattaaatccagtgatagc | 427057 | - | see also 22004 line |
| 49677 | FLJ40182 | NM_173696.1 | 779 | tgctacgtgaccaattcgtaaag | 427694 | - | see also 22005 line |
| 49678 | FLJ40182 | NM_173696.1 | 543 | tggcgtctctagtgaacaacata | 427686 | - | see also 22005 line |
| 49679 | FLJ40201 | NM_152607.1 | 215 | ccccatactccacgcgttattct | 418746 | - | see also 22006 line |
| 49680 | FLJ40201 | NM_152607.1 | 1156 | gacaggcggcagcgatatcgatc | 418752 | - | see also 22006 line |
| 49681 | FLJ40235 | NM_173635.1 | 739 | atctatgcgggaattgtaattgc | 426919 | - | see also 22008 line |
| 49682 | FLJ40235 | NM_173635.1 | 913 | cccgtagtcgccacattttctga | 426926 | - | see also 22008 line |
| 49683 | FLJ40243 | NM_173489.2 | 3601 | aggcacaatctaccatcacatgc | 425098 | - | see also 22009 line |
| 49684 | FLJ40243 | NM_173489.2 | 4584 | accgagacgtgagcttctacttc | 425115 | - | see also 22009 line |
| 49685 | FLJ40249 | NM_152425.1 | 1953 | gccgcgacagttccatcaagagt | 416623 | - | - | - | - |
| 49686 | FLJ40288 | NM_173682.1 | 799 | gccgaggacctccgtctatcact | 427349 | - | see also 22010 line |
| 49687 | FLJ40288 | NM_173682.1 | 723 | ccccacggtgctttatcttcatt | 427347 | - | see also 22010 line |
| 49688 | FLJ40288 | NM_173682.1 | 1013 | cagagtcaagtgccgtaaaatgg | 427359 | - | see also 22010 line |
| 49689 | FLJ40296 | NM_198441.1 | 815 | ctcagcggttttacaagtcattc | 437772 | - | see also 22011 line |
| 49690 | FLJ40296 | NM_198441.1 | 809 | gggtgactcagcggttttacaag | 437771 | - | see also 22011 line |
| 49691 | FLJ40296 | NM_198441.1 | 264 | cgctccatggcccctaatcaagc | 437769 | - | see also 22011 line |
| 49692 | FLJ40319 | NM_182564.1 | 337 | gtcctaaagcaatgcactaatcc | 434467 | - | - | - | - |
| 49693 | FLJ40319 | NM_182564.1 | 389 | aggcaaaggagtgtgaaaaatcc | 434469 | - | see also 49692 line |
| 49694 | FLJ40319 | NM_182564.1 | 356 | atccaaaattaaacgaaaacaaa | 434468 | - | see also 49692 line |
| 49695 | FLJ40321 | NM_198479.1 | 848 | ctcaggcccaggcccaattatag | 438455 | - | see also 22012 line |
| 49696 | FLJ40321 | NM_198479.1 | 1251 | gagggtggctctgtgaatgaaaa | 438459 | - | see also 22012 line |
| 49697 | FLJ40321 | NM_198479.1 | 1252 | agggtggctctgtgaatgaaaat | 438460 | - | see also 22012 line |
| 49698 | FLJ40343 | NM_182579.1 | 496 | agcggcatcccaccttgagatga | 434507 | - | - | - | - |
| 49699 | FLJ40343 | NM_182579.1 | 447 | cgcagttgtccagggaactatcc | 434506 | - | see also 49698 line |
| 49700 | FLJ40343 | NM_182579.1 | 249 | cccctacagtctccatacacaga | 434504 | - | see also 49698 line |
| 49701 | FLJ40365 | NM_173482.1 | 959 | tgcgccttggcgctaaacgaagc | 424985 | - | see also 22013 line |
| 49702 | FLJ40365 | NM_173482.1 | 1164 | tcctccggtgtacgaaatataac | 424988 | - | see also 22013 line |
| 49703 | FLJ40411 | NM_198852.1 | 505 | acctgatacacccgttcaattca | 439949 | - | see also 22014 line |
| 49704 | FLJ40411 | NM_198852.1 | 320 | gggaatagaattagttcgaataa | 439941 | - | see also 22014 line |
| 49705 | FLJ40457 | NM_173628.1 | 661 | ctcgaattttgaccgtgattatg | 426650 | - | see also 22015 line |
| 49706 | FLJ40457 | NM_173628.1 | 578 | ctggtgacccgtatctatgatga | 426649 | - | see also 22015 line |
| 49707 | FLJ40504 | NM_173624.1 | 506 | ttcgggtcttagccagttagatt | 426618 | - | see also 22016 line |
| 49708 | FLJ40504 | NM_173624.1 | 508 | cgggtcttagccagttagattta | 426619 | - | see also 22016 line |
| 49709 | FLJ40542 | NM_178503.2 | 354 | ctccgccttccggctcagaattg | 430926 | - | see also 22017 line |
| 49710 | FLJ40542 | NM_178503.2 | 335 | gccgtgcccacgcatttatctcc | 430925 | - | see also 22017 line |
| 49711 | FLJ40597 | NM_152615.1 | 1176 | tacgttgtgcgagtacttactgg | 418888 | - | see also 22018 line |
| 49712 | FLJ40597 | NM_152615.1 | 225 | atgaaaatcggtgcaattacttt | 418858 | - | see also 22018 line |

Figure 46

| 49713 | FLJ40629 | NM_152515.2 | 573 | ttgctcgatatcttaacagaacc | 417405 | - | see also 22019 line |
|---|---|---|---|---|---|---|---|
| 49714 | FLJ40629 | NM_152515.2 | 570 | aacttgctcgatatcttaacaga | 417404 | - | see also 22019 line |
| 49715 | FLJ40852 | NM_173677.1 | 480 | aagcttcactattgggaaaaatc | 427327 | - | see also 22020 line |
| 49716 | FLJ40852 | NM_173677.1 | 432 | caggttggatcatgttctgatga | 427325 | - | see also 22020 line |
| 49717 | FLJ40869 | NM_182625.1 | 344 | agccaattcgaattgttaagact | 434727 | - | see also 22021 line |
| 49718 | FLJ40869 | NM_182625.1 | 341 | tacagccaattcgaattgttaag | 434726 | - | see also 22021 line |
| 49719 | FLJ40873 | NM_152665.1 | 500 | cacggtataaactaattgtgatt | 419548 | - | see also 22022 line |
| 49720 | FLJ40873 | NM_152665.1 | 359 | ctgtggtcaccgtcaatcatatt | 419541 | - | see also 22022 line |
| 49721 | FLJ40906 | NM_173640.1 | 442 | aggcctgcttcagccataacttc | 426931 | - | see also 22023 line |
| 49722 | FLJ40906 | NM_173640.1 | 299 | gtgctcacccaagctgttcatcc | 426930 | - | see also 22023 line |
| 49723 | FLJ40919 | NM_182508.1 | 177 | ggcaacagcgctactttacagc | 434017 | - | see also 10266 line |
| 49724 | FLJ40919 | NM_182508.1 | 183 | agcgctacttttacagcattatg | 434018 | - | see also 10266 line |
| 49725 | FLJ40919 | NM_182508.1 | 438 | ttctacctagggcccaaagtaaa | 434028 | - | see also 10266 line |
| 49726 | FLJ41131 | NM_198476.1 | 515 | cacggcccagggagagatgttct | 438437 | - | see also 22025 line |
| 49727 | FLJ41238 | NM_198485.1 | 971 | ttgcccgtgggagtattggtttt | 438525 | - | see also 22026 line |
| 49728 | FLJ41238 | NM_198485.1 | 972 | tgcccgtgggagtattggtttt | 438526 | - | see also 22026 line |
| 49729 | FLJ41238 | NM_198485.1 | 271 | gacccgatgccaagacagatttc | 438509 | - | see also 22026 line |
| 49730 | FLJ41410 | NM_198581.1 | 116 | gacgtggcttgccgaagaaaatc | 439742 | - | see also 10347 line |
| 49731 | FLJ41410 | NM_198581.1 | 1240 | aacccagctgagttttacgataa | 439771 | - | see also 10347 line |
| 49732 | FLJ41410 | NM_198581.1 | 162 | aaccaataaagggcctaatgtgt | 439749 | - | see also 10347 line |
| 49733 | FLJ42117 | NM_198463.1 | 633 | accacgaagctgtcaactaatga | 438022 | - | see also 22028 line |
| 49734 | FLJ42117 | NM_198463.1 | 635 | cacgaagctgtcaactaatgaca | 438023 | - | see also 22028 line |
| 49735 | FLJ42461 | NM_198501.1 | 205 | ccctgagattgcccaaaacttct | 438737 | - | see also 22029 line |
| 49736 | FLJ42461 | NM_198501.1 | 423 | gtcctagcgagaagaattcctct | 438740 | - | see also 22029 line |
| 49737 | FLJ42654 | NM_198447.1 | 258 | tggaacactcctgtactttgatt | 437822 | - | see also 22030 line |
| 49738 | FLJ42654 | NM_198447.1 | 196 | atcaccgaatggcagaagattgg | 437821 | - | see also 22030 line |
| 49739 | FLJ42925 | NM_198511.1 | 1008 | cacggcttgtcgtctattcttca | 438842 | - | see also 22031 line |
| 49740 | FLJ42925 | NM_198511.1 | 323 | cgccaatcgcttcgatgactacc | 438836 | - | see also 22031 line |
| 49741 | FLJ42925 | NM_198511.1 | 955 | aaccattcccctgatgtattct | 438841 | - | see also 22031 line |
| 49742 | FLJ43070 | NM_152286.2 | 3893 | ttcacggaccttgccgaaattgt | 415360 | - | see also 22032 line |
| 49743 | FLJ43070 | NM_152286.2 | 3897 | cggaccttgccgaaattgtgtct | 415362 | - | see also 22032 line |
| 49744 | FLJ43654 | NM_198562.1 | 160 | aagacatggtcgcttttaggaga | 439518 | - | see also 22034 line |
| 49745 | FLJ43654 | NM_198562.1 | 922 | atgacgtcttttaaataccaaat | 439528 | - | see also 22034 line |
| 49746 | FLJ43654 | NM_198562.1 | 624 | ctcaaacatgccactcaacaatt | 439524 | - | see also 22034 line |
| 49747 | FLJ43842 | NM_198440.1 | 754 | tgcggtgggccatatctactact | 437766 | - | see also 22035 line |
| 49748 | FLJ43842 | NM_198440.1 | 562 | gtcgctgtctccagcttagtatg | 437760 | - | see also 22035 line |
| 49749 | FLJ44112 | NM_198520.1 | 2146 | agcatgctaaagtcgatgttact | 439039 | - | see also 22036 line |

Figure 46

| | | | | | | | |
|---|---|---|---|---|---|---|---|
| 49750 | FLJ44112 | NM_198520.1 | 2662 | ttgaacgcaagctgtaattcttgg | 439051 | - | see also 22036 line |
| 49751 | FLJ44186 | NM_198508.1 | 973 | ccgaggagcacgttactacttc | 438795 | - | see also 22037 line |
| 49752 | FLJ44216 | NM_198567.1 | 532 | atcgcagtggacgtatgtgatt | 439669 | - | see also 10346 line |
| 49753 | FLJ44216 | NM_198567.1 | 2706 | cagacttgggacgcaattggttga | 439682 | - | see also 10346 line |
| 49754 | FLJ44299 | NM_198491.1 | 558 | gtcaccctcggatcagcaaatga | 438548 | - | see also 22039 line |
| 49755 | FLJ44299 | NM_198491.1 | 560 | caccctcgatcagcaaatgatc | 438549 | - | see also 22039 line |
| 49756 | FLJ44691 | NM_198506.1 | 864 | aaggtcgttgaccctgctatagt | 438751 | - | see also 22040 line |
| 49757 | FLJ44691 | NM_198506.1 | 1089 | agctcgccagttaattattacagt | 438760 | - | see also 22040 line |
| 49758 | FLJ44968 | NM_198537.1 | 1882 | cagtggctggatgcacgttttc | 439296 | - | - see also 22036 line |
| 49759 | FLJ44968 | NM_198537.1 | 1840 | aactcaggcggagaataaacaa | 439295 | - | see also 49758 line |
| 49760 | FLJ45235 | NM_198493.1 | 662 | ctcagtgcatgccgttgcaaaaa | 438570 | - | see also 22041 line |
| 49761 | FLJ45235 | NM_198493.1 | 389 | gggtacacactcttacattgtgc | 438564 | - | see also 22041 line |
| 49762 | FLJ45246 | NM_198484.1 | 702 | tccgatataactcaaagcttatt | 438501 | - | see also 22042 line |
| 49763 | FLJ45246 | NM_198484.1 | 701 | ttccgatataactcaaagcttat | 438500 | - | see also 22042 line |
| 49764 | FLJ45246 | NM_198484.1 | 1042 | ttgtaggtcactttattgtcc | 438505 | - | see also 22042 line |
| 49765 | FLJ45273 | NM_198461.1 | 1548 | atcacgcgtaagatgaatagtcg | 437959 | - | see also 22043 line |
| 49766 | FLJ45273 | NM_198461.1 | 1017 | cccgctatcggcttatgataag | 437946 | - | see also 22043 line |
| 49767 | FLJ45273 | NM_198461.1 | 1523 | tgccatccgacggatattagtca | 437955 | - | see also 22043 line |
| 49768 | FLJ45459 | NM_198446.1 | 390 | cgccagaggcggctttccaagg | 437820 | - | - see also 22044 line |
| 49769 | FLJ45645 | NM_198557.1 | 1085 | aagttcgagataccttgaagtcc | 439499 | - | see also 22044 line |
| 49770 | FLJ45645 | NM_198557.1 | 1105 | tcctgattaacctttataggaca | 439501 | - | see also 22044 line |
| 49771 | FLJ45651 | NM_198442.1 | 505 | agctacatcaccggtctatcagg | 437773 | - | see also 22045 line |
| 49772 | FLJ45651 | NM_198442.1 | 1064 | cacacacctcggaggagtttttc | 437776 | - | see also 22045 line |
| 49773 | FLJ45778 | NM_198532.1 | 637 | gagcggggacgccctgattacc | 439257 | - | see also 22046 line |
| 49774 | FLJ45778 | NM_198532.1 | 1441 | ctggctgttgcgaataacctgg | 439258 | - | see also 22046 line |
| 49775 | FLJ45909 | NM_198445.1 | 1464 | agctggactagagtttcttatg | 437818 | - | see also 22047 line |
| 49776 | FLJ45909 | NM_198445.1 | 891 | gggttgcgcatcgcctcagattct | 437814 | - | see also 22047 line |
| 49777 | FLJ46072 | NM_198488.1 | 1856 | tccaactacaggatttatcgagc | 438533 | - | see also 22048 line |
| 49778 | FLJ46072 | NM_198488.1 | 922 | ggcgtttgcctcctacttgagc | 438529 | - | see also 22048 line |
| 49779 | FLJ46072 | NM_198488.1 | 2688 | aagatcctgggcacgttcaaaag | 438534 | - | see also 22048 line |
| 49780 | FLJ46154 | NM_198462.1 | 3228 | atccgatagtttgatggttatag | 438008 | - | see also 22049 line |
| 49781 | FLJ46154 | NM_198462.1 | 2163 | ttgtcatcagcagttcgaataca | 437985 | - | see also 22049 line |
| 49782 | FLJ46156 | NM_198499.1 | 335 | atcctcgtatagttgattatca | 438615 | - | see also 22050 line |
| 49783 | FLJ46156 | NM_198499.1 | 2271 | gactagcgtttacagtcatattg | 438671 | - | see also 22050 line |
| 49784 | FLJ46156 | NM_198499.1 | 325 | ttcttagggtatcctgtatagt | 438614 | - | see also 22050 line |
| 49785 | FLJ46247 | NM_198529.1 | 2029 | tccctagagcgcggtgtatagtga | 439189 | - | see also 22051 line |
| 49786 | FLJ46247 | NM_198529.1 | 2317 | aaccagtccgtaataagcatga | 439195 | - | see also 22051 line |

Figure 46

| | | | | | | | | | |
|---|---|---|---|---|---|---|---|---|---|
| 49787 | FLJ46361 | NM_198577.1 | 2085 | cacatagaactcataattccaag | 439722 | - | - | - | - |
| 49788 | FLJ46361 | NM_198577.1 | 2102 | tccaagtttgaaggttatacagt | 439724 | - | see also 49787 line | | |
| 49789 | FLJ46361 | NM_198577.1 | 2092 | aactcataattccaagtttgaag | 439723 | - | see also 49787 line | | |
| 49790 | FLJ46603 | NM_198530.1 | 1856 | cactttggcccatcgtttgtaca | 439234 | - | - | - | - |
| 49791 | FLJ46909 | NM_198469.1 | 409 | tccccaagggctattacgattgt | 438401 | - | see also 22052 line | | |
| 49792 | FLJ46909 | NM_198469.1 | 408 | atccccaagggctattacgattg | 438400 | - | see also 22052 line | | |
| 49793 | FLJ46909 | NM_198469.1 | 206 | ccccagcggaagccaatacgacg | 438396 | - | see also 22052 line | | |
| 49794 | FLJ90005 | NM_153341.1 | 213 | tacgagttcggaccaaacacact | 422808 | - | see also 22053 line | | |
| 49795 | FLJ90005 | NM_153341.1 | 291 | cacgatgcagtgcatacattatc | 422812 | - | see also 22053 line | | |
| 49796 | FLJ90013 | NM_153365.1 | 253 | gagggtacttccttgaacataat | 423076 | - | see also 9993 line | | |
| 49797 | FLJ90013 | NM_153365.1 | 1618 | aggacataacgcaggacaattct | 423108 | - | see also 9993 line | | |
| 49798 | FLJ90022 | NM_153690.3 | 723 | aacgcgctggcggaatttaaacg | 423543 | - | see also 22055 line | | |
| 49799 | FLJ90022 | NM_153690.3 | 332 | cactagcgaggacccaacttaca | 423540 | - | see also 22055 line | | |
| 49800 | FLJ90024 | NM_153342.1 | 456 | gtcggcactcttgggttaacacc | 422838 | - | see also 22056 line | | |
| 49801 | FLJ90119 | NM_153347.1 | 660 | atcgccctcaacaaattctgttt | 422931 | - | see also 22057 line | | |
| 49802 | FLJ90119 | NM_153347.1 | 625 | gtggtgcactcttctttatcatc | 422928 | - | see also 22057 line | | |
| 49803 | FLJ90166 | NM_153360.1 | 1412 | tggagtacgagcttttcaagatg | 423054 | - | see also 22058 line | | |
| 49804 | FLJ90166 | NM_153360.1 | 1409 | atgtggagtacgagcttttcaag | 423053 | - | see also 22058 line | | |
| 49805 | FLJ90166 | NM_153360.1 | 334 | atcctgcctccacgccttaatgg | 423050 | - | see also 22058 line | | |
| 49806 | FLJ90406 | NM_173670.1 | 995 | gacggctttgactctgagttttg | 427264 | - | see also 22059 line | | |
| 49807 | FLJ90406 | NM_173670.1 | 1115 | ctcatgagccagaggaattgttc | 427267 | - | see also 22059 line | | |
| 49808 | FLJ90492 | NM_181783.1 | 1020 | ctcctacaaggcaactaactttt | 433251 | - | see also 22060 line | | |
| 49809 | FLJ90492 | NM_181783.1 | 402 | accgtcccttaacagtattgaca | 433228 | - | see also 22060 line | | |
| 49810 | FLJ90575 | NM_153376.1 | 724 | ctcccagcgctacaacctatacc | 423159 | - | see also 22061 line | | |
| 49811 | FLJ90575 | NM_153376.1 | 1367 | gagaacgcgtgcaaaaagacaca | 423165 | - | see also 22061 line | | |
| 49812 | FLJ90575 | NM_153376.1 | 1593 | accacgctagcctcactttgtcc | 423167 | - | see also 22061 line | | |
| 49813 | FLJ90579 | NM_173591.1 | 1145 | atgcccatcagctaccatatata | 426377 | - | see also 22062 line | | |
| 49814 | FLJ90579 | NM_173591.1 | 466 | gcccaacatggcactgtgaatgt | 426358 | - | see also 22062 line | | |
| 49815 | FLJ90583 | NM_173828.3 | 1714 | aggttccgggtgacacacattga | 428440 | - | see also 22063 line | | |
| 49816 | FLJ90583 | NM_173828.3 | 2065 | agcaaaccagacacttctgatca | 428441 | - | see also 22063 line | | |
| 49817 | FLJ90586 | NM_153345.1 | 727 | aggcaatgcacgggacaatgaag | 422850 | - | see also 22064 line | | |
| 49818 | FLJ90586 | NM_153345.1 | 593 | gacatcacccccgttgcttattt | 422846 | - | see also 22064 line | | |
| 49819 | FLJ90650 | NM_173800.2 | 448 | gtctactggcgtcatgaaacagg | 427997 | - | see also 22065 line | | |
| 49820 | FLJ90650 | NM_173800.2 | 1399 | tggccggtatgtcgcaaaagact | 428022 | - | see also 22065 line | | |
| 49821 | FLJ90652 | NM_173618.1 | 118 | acggcgaagtggactacaaaaaa | 426578 | - | see also 10091 line | | |
| 49822 | FLJ90652 | NM_173618.1 | 117 | gacggcgaagtggactacaaaaa | 426577 | - | see also 10091 line | | |
| 49823 | FLJ90805 | NM_173633.1 | 1366 | atgggaacgtgacgtttatcagc | 426915 | - | see also 10101 line | | |

Figure 46

| | | | | | | | |
|---|---|---|---|---|---|---|---|
| 49824 | FLJ90805 | NM_173633.1 | 708 | tgcatctactgggtcaatatgc | 426911 | - | see also 10101 line |
| 49825 | FLJ90806 | NM_182513.1 | 428 | aactttaccaccaagttagtaaa | 434047 | - | see also 22068 line |
| 49826 | FLJ90806 | NM_182513.1 | 412 | gccgtgtacgtggtcaactta | 434045 | - | see also 22068 line |
| 49827 | FLJ90811 | NM_153339.1 | 495 | cccggcagactgcctggatatgg | 422793 | - | see also 22069 line |
| 49828 | FLJ90811 | NM_153339.1 | 843 | agccccagccacggcttattcc | 422795 | - | see also 22069 line |
| 49829 | FLJ90834 | NM_173578.1 | 958 | accctacaacgtccctataaac | 426294 | - | - |
| 49830 | FLJ90834 | NM_173578.1 | 962 | tacaaacgtccctataaacttta | 426295 | - | see also 49829 line |
| 49831 | FLJ90834 | NM_173578.1 | 428 | atcctgtgtcactctgtatatca | 426293 | - | see also 49829 line |
| 49832 | FLN29 | NM_006700.1 | 487 | gaggttgccatacctcctaatgc | 336122 | - | see also 22070 line |
| 49833 | FLN29 | NM_006700.1 | 1405 | cccagccgaccattaacaatat | 336142 | - | see also 22070 line |
| 49834 | FLNC | NM_001458.1 | 4586 | tacaacggtagccgtcaagtatgc | 330267 | - | see also 22071 line |
| 49835 | FLNC | NM_001458.1 | 2275 | ctcctgggagggcgtaaacgtgc | 330256 | - | see also 22071 line |
| 49836 | FNBP1 | NM_015033.1 | 1131 | aagttatgccgttcatcaaaaa | 347011 | - | see also 22072 line |
| 49837 | FNBP1 | NM_015033.1 | 1871 | aggaacgatttcgtagttgaag | 347027 | - | see also 22072 line |
| 49838 | FNBP4 | NM_015308.1 | 1352 | atggaatgcgtaggcttatgtct | 351364 | - | see also 6321 line |
| 49839 | FNBP4 | NM_015308.1 | 1642 | ttcgagtttctaggcattaatag | 351370 | - | see also 6321 line |
| 49840 | FRBZ1 | NM_194314.1 | 1809 | aagttcgtatagacttcacttac | 436780 | - | see also 10308 line |
| 49841 | FRBZ1 | NM_194314.1 | 1886 | tccgtcatgatcaacttacaaag | 436784 | - | see also 10308 line |
| 49842 | FRCP1 | NM_022823.1 | 919 | ggggtcgttcgccgtcagtatga | 384251 | - | see also 22075 line |
| 49843 | FRCP1 | NM_022823.1 | 587 | tcgtcattgctactccatttcc | 384247 | - | see also 22075 line |
| 49844 | FRCP2 | NM_153756.1 | 616 | gggcttctccgcagcaagatatg | 423893 | - | see also 22076 line |
| 49845 | FRCP2 | NM_153756.1 | 617 | ggcttctccgcagcaagatatga | 423894 | - | see also 22076 line |
| 49846 | FS | NM_021996.3 | 1180 | acctgaacgtcacttcatctca | 381677 | - | see also 22077 line |
| 49847 | FS | NM_021996.3 | 1286 | ttctacactggacaaggatatca | 381679 | - | see also 22077 line |
| 49848 | FTSJ3 | NM_017647.2 | 96 | aagagccgacgagacaagtttta | 360059 | - | see also 7077 line |
| 49849 | FTSJ3 | NM_017647.2 | 103 | gacgagacaagttttatcacttg | 360063 | - | see also 7077 line |
| 49850 | FTSJ3 | NM_017647.2 | 2132 | ctccttcaaccgtacacattta | 360092 | - | see also 7077 line |
| 49851 | FZR1 | NM_016263.2 | 196 | agccaactgagcgtgaacttcc | 356657 | - | see also 22080 line |
| 49852 | FZR1 | NM_016263.2 | 388 | gccctccacgcctgagaagaag | 356659 | - | see also 22080 line |
| 49853 | G30 | NM_172368.1 | 405 | ggccaggtcttgaaaggtaac | 424480 | - | see also 22081 line |
| 49854 | G30 | NM_172368.1 | 492 | gcccacattcaagggaacattca | 424481 | - | see also 22081 line |
| 49855 | G6PC3 | NM_138387.1 | 686 | ttggcttgtcgcgaatcttcatc | 407874 | - | see also 22082 line |
| 49856 | G6PC3 | NM_138387.1 | 793 | gagcgggagctaagcttctatgg | 407878 | - | see also 22082 line |
| 49857 | GA17 | NM_006360.2 | 105 | ttcgtgcttatctgaaatctaaa | 335314 | - | see also 22083 line |
| 49858 | GA17 | NM_006360.2 | 247 | cagtgtggtatccctactcttga | 335316 | - | see also 22083 line |
| 49859 | GAB2 | NM_012296.2 | 1870 | caccggcagcgttgattatctgg | 337976 | - | see also 22084 line |
| 49860 | GAB2 | NM_012296.2 | 805 | gccgagccggcacaatacagaat | 337962 | - | see also 22084 line |

1665

Figure 46

| 49861 | GAB2 | NM_012296.2 | 1368 | gccgatcggacagcaccaattct | 337969 | - | see also 22084 line |
|---|---|---|---|---|---|---|---|
| 49862 | GAB3 | NM_080612.1 | 1126 | accgagacaagcggcttagtttg | 406427 | - | see also 22085 line |
| 49863 | GAB3 | NM_080612.1 | 1131 | gacaagcggcttagtttgaattt | 406430 | - | see also 22085 line |
| 49864 | GAGED2 | NM_020411.1 | 446 | tccggcgtcaaggtgaagataat | 377376 | - | see also 22086 line |
| 49865 | GAGED2 | NM_020411.1 | 421 | accggggataaatctggatttgg | 377375 | - | see also 22086 line |
| 49866 | GAJ | NM_032117.2 | 278 | gaggatcggaacttctaattatt | 397764 | - | see also 22087 line |
| 49867 | GAJ | NM_032117.2 | 282 | atcggaacttctaattattattg | 397766 | - | see also 22087 line |
| 49868 | GALNT10 | NM_017540.3 | 977 | gtgaccataggatcttcatgaac | 358994 | - | see also 7020 line |
| 49869 | GALNT10 | NM_017540.3 | 524 | gggatgtcgcagtccagaaaaag | 358986 | - | see also 7020 line |
| 49870 | GALNT11 | NM_022087.1 | 1221 | ttccgaaaaaggcgaccatatgg | 382244 | - | see also 22089 line |
| 49871 | GALNT11 | NM_022087.1 | 949 | tcgtccgcggagggttcaactgg | 382233 | - | see also 22089 line |
| 49872 | GALNT15 | NM_145292.2 | 1158 | ttgctatacgtcggcattatttt | 414231 | - | see also 22090 line |
| 49873 | GALNT15 | NM_145292.2 | 1452 | ttcgcgagcgtgttgagttaagg | 414248 | - | see also 22090 line |
| 49874 | GALNT15 | NM_145292.2 | 1159 | tgctatacgtcggcattatttta | 414232 | - | see also 22090 line |
| 49875 | GALNT7 | NM_054110.2 | 1739 | ctctcgggtaggacacatctacc | 406052 | - | see also 22091 line |
| 49876 | GALNT7 | NM_054110.2 | 1640 | tggagcgtatgactctcttatgt | 406048 | - | see also 22091 line |
| 49877 | GBAS | NM_001483.1 | 544 | tgccaagatccggacctaatata | 330295 | - | see also 22092 line |
| 49878 | GBAS | NM_001483.1 | 254 | accggaatgcctagaagcataca | 330290 | - | see also 22092 line |
| 49879 | GBDR1 | NM_016172.1 | 1269 | accaacccgaaaacattgctagc | 356529 | - | see also 22093 line |
| 49880 | GBDR1 | NM_016172.1 | 365 | aacccatcataaattaatccacg | 356519 | - | see also 22093 line |
| 49881 | GBL | NM_022372.2 | 310 | cagctacgacggcgtcaacaaga | 382749 | - | see also 22094 line |
| 49882 | GBL | NM_022372.2 | 257 | taccagcacatccgcatgtatga | 382747 | - | see also 22094 line |
| 49883 | GBP | NM_017870.2 | 2780 | ctgcgctatcagcgcaaagaacc | 363324 | - | see also 22095 line |
| 49884 | GBP | NM_017870.2 | 2578 | aggtgtgaggggcaagtttgagc | 363323 | - | see also 22095 line |
| 49885 | GBX2 | NM_001485.1 | 945 | gccctcccggaaccctaagatcg | 330304 | - | transcription_regulator | transcription factor | - |
| 49886 | GBX2 | NM_001485.1 | 855 | cagcgaggtgcaggtgaaaatct | 330303 | - | see also 49885 line |
| 49887 | GBX2 | NM_001485.1 | 988 | gtcagcaggttcgctatcagaag | 330305 | - | see also 49885 line |
| 49888 | GCET2 | NM_152785.2 | 375 | atcatcgggttctctgtacaagg | 421215 | - | see also 22096 line |
| 49889 | GCET2 | NM_152785.2 | 528 | cccgatcccagaagatgaatat | 421217 | - | see also 22096 line |
| 49890 | GCP16 | NM_016099.1 | 263 | cagcatataccatcttcctatgc | 356273 | - | see also 22097 line |
| 49891 | GCP16 | NM_016099.1 | 401 | tgcgagttattgaaattaccatt | 356281 | - | see also 22097 line |
| 49892 | GDAP1 | NM_018972.1 | 64 | ggccgacgcggaggttaagctca | 373895 | - | see also 7621 line |
| 49893 | GDAP1 | NM_018972.1 | 461 | atcccggcttatgcaactacaag | 373905 | - | see also 7621 line |
| 49894 | GDAP1 | NM_018972.1 | 766 | cacattgcatcgactgaagttcc | 373911 | - | see also 7621 line |
| 49895 | GDAP1L1 | NM_024034.3 | 602 | gccgagatccgcagacatttagc | 385535 | - | see also 8566 line |
| 49896 | GDAP1L1 | NM_024034.3 | 69 | tggcgaccccaacaatctgacc | 385533 | - | see also 8566 line |

Figure 46

| | | | | | | | |
|---|---|---|---|---|---|---|---|
| 49897 | GDAP2 | NM_017686.1 | 952 | acctgaacgacagattagaataa | 360794 | - | see also 22099 line |
| 49898 | GDAP2 | NM_017686.1 | 951 | tacctgaacgacagattagaata | 360793 | - | see also 22099 line |
| 49899 | GDDR | NM_182536.1 | 323 | tccaatggtacatctatgagaaa | 434221 | - | see also 22100 line |
| 49900 | GDDR | NM_182536.1 | 89 | atggatacgaggtttttaacatc | 434215 | - | see also 22100 line |
| 49901 | GDF7 | NM_182828.1 | 1498 | gcgtcgccaagggcagtcattgg | 435297 | - | see also 22101 line |
| 49902 | GDF7 | NM_182828.1 | 1391 | cccgcacgcagaggaaagagagc | 435296 | - | see also 22101 line |
| 49903 | GEFT | NM_133483.1 | 862 | ctgaacgacctcctcatcaaacc | 407241 | - | see also 22102 line |
| 49904 | GEFT | NM_133483.1 | 1620 | agccctcttgccactggataaac | 407246 | - | see also 22102 line |
| 49905 | GEMIN5 | NM_015465.1 | 320 | tccatcagcatacgatatcaaca | 353333 | - | see also 22103 line |
| 49906 | GEMIN5 | NM_015465.1 | 3184 | aagggggatgcggcatcacttag | 353393 | - | see also 22103 line |
| 49907 | GEMIN6 | NM_024775.8 | 444 | ggctggggtcctgactatagacc | 390576 | - | see also 22104 line |
| 49908 | GEMIN6 | NM_024775.8 | 80 | gcccttagaatggcaagattac | 390570 | - | see also 22104 line |
| 49909 | GEMIN7 | NM_024707.1 | 574 | tgctccgatgtagtgacattatt | 389454 | - | see also 22105 line |
| 49910 | GEMIN7 | NM_024707.1 | 567 | gaggcgctgctccgatgtagtga | 389452 | - | see also 22105 line |
| 49911 | GGN | NM_152657.3 | 1886 | tgcccttccaggtgcttaactcc | 419530 | - | - |
| 49912 | GJA3 | NM_021954.2 | 796 | atcgggttcccaccctactatgc | 381578 | - | see also 8215 line |
| 49913 | GJA3 | NM_021954.2 | 437 | tgcggacctacgtcttcaacatc | 381576 | - | see also 8215 line |
| 49914 | GJA8 | NM_005267.2 | 1303 | cccctgagcaggctaagcaaagc | 333583 | - | see also 22106 line |
| 49915 | GJA8 | NM_005267.2 | 934 | ctgcctgccaagcctttcaatca | 333579 | - | see also 22106 line |
| 49916 | GJB7 | NM_198568.1 | 701 | ggcctatggtacgcttatcttat | 439696 | - | see also 22107 line |
| 49917 | GJB7 | NM_198568.1 | 702 | gcctatggtacgcttatcttatc | 439697 | - | see also 22107 line |
| 49918 | GK001 | NM_020198.1 | 340 | atctcctcaacgggtcataatca | 376095 | - | see also 22108 line |
| 49919 | GK001 | NM_020198.1 | 945 | aagacatggatacctacgtattt | 376118 | - | see also 22108 line |
| 49920 | GK003 | NM_020192.1 | 384 | tccaccgtcccatgttgtagatt | 375930 | - | see also 22109 line |
| 49921 | GK003 | NM_020192.1 | 239 | gacgactccgaggaacattgagt | 375926 | - | see also 22109 line |
| 49922 | GK003 | NM_020192.1 | 128 | gacgagtcaaagaaggttataga | 375921 | - | see also 22109 line |
| 49923 | GL004 | NM_020194.4 | 721 | gagagcggtctatgagtgaaaat | 376049 | - | see also 7800 line |
| 49924 | GL004 | NM_020194.4 | 697 | tccgagcagttggcagactaaaa | 376046 | - | see also 7800 line |
| 49925 | GL009 | NM_032492.2 | 354 | gtgggaatacccgtatttgctga | 400396 | - | see also 9220 line |
| 49926 | GL009 | NM_032492.2 | 410 | ttccccgcaacaacattagctac | 400398 | - | see also 9220 line |
| 49927 | GLIS2 | NM_032575.1 | 616 | gaccatgtcaacgattaccatgt | 400821 | - | see also 22112 line |
| 49928 | GLIS2 | NM_032575.1 | 885 | ctccagtgaccgctttaagcaca | 400823 | - | see also 22112 line |
| 49929 | GLTDC1 | NM_182974.1 | 376 | aacgccctgatgttataacgaaa | 435531 | - | see also 22113 line |
| 49930 | GLTDC1 | NM_182974.1 | 1127 | tcctccacagatccaatacgtca | 435549 | - | see also 22113 line |
| 49931 | GLTDC1 | NM_182974.1 | 1143 | tacgtcaaggtcgcacatgattc | 435550 | - | see also 22113 line |
| 49932 | GLTSCR1 | NM_015711.1 | 3652 | acccaggccatgctcaataaata | 355230 | - | see also 22114 line |
| 49933 | GLTSCR1 | NM_015711.1 | 2220 | ggcgtctagcccggagaagatcg | 355227 | - | see also 22114 line |

Figure 46

| | | | | | | | | | |
|---|---|---|---|---|---|---|---|---|---|
| 49934 | GLYCTK | NM_145262.2 | 1064 | cagccatgcaaggtgatgtaaaa | 413972 | - | kinase_related、transcription_regulator | - | - |
| 49935 | GLYCTK | NM_145262.2 | 1065 | agccatgcaaggtgatgtaaaaa | 413973 | - | see also 49934 line | | |
| 49936 | GLYCTK | NM_145262.2 | 1069 | atgcaaggtgatgtaaaaagtat | 413974 | - | see also 49934 line | | |
| 49937 | gm117 | NM_198077.1 | 223 | ctctacaatccgctcaacaaaca | 436977 | - | see also 22115 line | | |
| 49938 | gm117 | NM_198077.1 | 368 | cagagcttgacatggcaataaaa | 436980 | - | see also 22115 line | | |
| 49939 | GMH1 | NM_014044.4 | 1264 | agcgaaacgctacaaatatctga | 339390 | - | see also 5471 line | | |
| 49940 | GMH1 | NM_014044.4 | 1526 | tgggttgtactcatagattgtgt | 339396 | - | see also 5471 line | | |
| 49941 | GNPNAT1 | NM_198066.1 | 586 | tacatgtgtcggaggtttctaaa | 436955 | - | see also 22117 line | | |
| 49942 | GNPNAT1 | NM_198066.1 | 497 | aactgaactgttacaagattacc | 436953 | - | see also 22117 line | | |
| 49943 | GNRPX | NM_018049.1 | 413 | tgcggagaagcctcatcttctac | 365841 | - | - | - | - |
| 49944 | GnT-IX | NM_144677.2 | 479 | caccatccgcacagaagtgatgg | 411273 | - | see also 22118 line | | |
| 49945 | GnT-IX | NM_144677.2 | 2419 | gcctggaccacgggctaatctgt | 411279 | - | see also 22118 line | | |
| 49946 | GOLPH3 | NM_022130.3 | 503 | accgcgagggttacacatcattt | 382399 | - | see also 8292 line | | |
| 49947 | GORASP1 | NM_031899.2 | 528 | caggagtccgaggacttctttac | 397233 | - | see also 9049 line | | |
| 49948 | GORASP1 | NM_031899.2 | 989 | tccagtgcagcgagttatggacc | 397240 | - | see also 9049 line | | |
| 49949 | GPP34R | NM_018178.3 | 303 | aggatatccgccttactcttatg | 368035 | - | see also 7302 line | | |
| 49950 | GPR103 | NM_198179.1 | 183 | tggcaatgctctggtgttctacg | 437162 | - | see also 22121 line | | |
| 49951 | GPR103 | NM_198179.1 | 331 | tggctgggggggtgctttcatttg | 437163 | - | see also 22121 line | | |
| 49952 | GPR107 | NM_020960.1 | 429 | tacccagttaccaaagatcatct | 380231 | - | see also 22122 line | | |
| 49953 | GPR107 | NM_020960.1 | 864 | tcgaaaacgacggaatgatgtat | 380241 | - | see also 22122 line | | |
| 49954 | GPR141 | NM_181791.1 | 629 | agctacgccactctttactatcc | 433519 | - | see also 22123 line | | |
| 49955 | GPR141 | NM_181791.1 | 27 | ttcctcttgcgatcctatagtga | 433507 | - | see also 22123 line | | |
| 49956 | GPR142 | NM_181790.1 | 112 | atggtgtcccatgcacagaaaag | 433503 | - | receptor_acitivity、gpcr、signal_transduction | - | - |
| 49957 | GPR142 | NM_181790.1 | 329 | agctgcggggaccctcagaaaaa | 433504 | - | see also 49956 line | | |
| 49958 | GPR142 | NM_181790.1 | 73 | gtcctgggtacagaagcatatac | 433501 | - | see also 49956 line | | |
| 49959 | GPR144 | NM_182611.1 | 1372 | ctgagccaaggcgttgtatctgt | 434665 | - | see also 22124 line | | |
| 49960 | GPR144 | NM_182611.1 | 1370 | agctgagccaaggcgttgtatct | 434664 | - | see also 22124 line | | |
| 49961 | GPR8 | NM_005286.2 | 189 | cacggccgtcatccttgtaatcc | 333585 | - | see also 22125 line | | |
| 49962 | GPR8 | NM_005286.2 | 363 | ggccgtcgaccactacaacatct | 333588 | - | see also 22125 line | | |
| 49963 | GPT | NM_005309.1 | 1113 | gggttcgcagttccactcattca | 333627 | - | see also 22126 line | | |
| 49964 | GPT | NM_005309.1 | 566 | tccctgacgatgccaagaaaagg | 333625 | - | see also 22126 line | | |
| 49965 | GPX6 | NM_182701.1 | 197 | cggcgaggagtacatccaattca | 434874 | - | see also 22127 line | | |
| 49966 | GPX6 | NM_182701.1 | 230 | aggcaagcacgtcctgtttgtca | 434875 | - | see also 22127 line | | |
| 49967 | GR6 | NM_007354.1 | 1179 | cccacccactaccatttgacatg | 337467 | - | - | - | - |

Figure 46

| 49968 | GRASP | NM_181711.1 | 549 | ggcatccggcatcgagagattgt | 432876 | - | see also 22128 line |
|-------|-------|-------------|-----|-------------------------|--------|---|---------------------|
| 49969 | GRASP | NM_181711.1 | 453 | acctttgtctgccgagttcatga | 432874 | - | see also 22128 line |
| 49970 | GRCC10 | NM_138425.2 | 304 | cggggaaggtgtccttaagtttg | 408103 | - | see also 22129 line |
| 49971 | GRCC10 | NM_138425.2 | 305 | ggggaaggtgtccttaagtttgc | 408104 | - | see also 22129 line |
| 49972 | GRLF1 | NM_004491.2 | 611 | aaggtgttgagcggtacattaga | 332504 | - | see also 22130 line |
| 49973 | GRLF1 | NM_004491.2 | 1743 | gccgtctgaccggaatcagaaaa | 332541 | - | see also 22130 line |
| 49974 | GRPEL2 | NM_152407.2 | 592 | accgtggcattagtaagacaaga | 416376 | - | see also 9857 line |
| 49975 | GRPEL2 | NM_152407.2 | 589 | ggcaccgtggcattagtaagaca | 416374 | - | see also 9857 line |
| 49976 | GRWD1 | NM_031485.2 | 1205 | gggaccttcggcagttcaagtct | 397206 | - | see also 22132 line |
| 49977 | GRWD1 | NM_031485.2 | 443 | cagagcttcctcttacactttac | 397205 | - | see also 22132 line |
| 49978 | GSDM | NM_178171.2 | 1108 | ggcgccatcctctatttcgttgg | 430565 | - | - | - | - |
| 49979 | GSDM | NM_178171.2 | 1012 | gaccttgagctcgcacttgaagg | 430564 | - | see also 49978 line |
| 49980 | GSDM | NM_178171.2 | 779 | ttccacatatctgcaatgataac | 430560 | - | see also 49978 line |
| 49981 | GSDML | NM_018530.1 | 732 | ctgaaaagcgaccggcaatataa | 372153 | - | see also 22133 line |
| 49982 | GSDML | NM_018530.1 | 737 | aagcgaccggcaatataaatttt | 372154 | - | see also 22133 line |
| 49983 | GSDML | NM_018530.1 | 738 | agcgaccggcaatataaattttg | 372155 | - | see also 22133 line |
| 49984 | GSG1 | NM_031289.1 | 479 | tgccgaagtttcattgaacttac | 396510 | - | see also 9003 line |
| 49985 | GSG1 | NM_031289.1 | 672 | atcacctacatcggacttcaatt | 396516 | - | see also 9003 line |
| 49986 | GW112 | NM_006418.3 | 630 | tgccattcgccgagaaatcgtgg | 335504 | - | see also 22136 line |
| 49987 | GW112 | NM_006418.3 | 1474 | gtctataacgatggttaccttct | 335535 | - | see also 22136 line |
| 49988 | H326 | NM_015726.2 | 1167 | gtggggctgtatacgatctatgt | 355246 | - | see also 6542 line |
| 49989 | H63 | NM_138423.2 | 631 | aactacagaacaacatatcgtat | 408078 | - | see also 9618 line |
| 49990 | H63 | NM_138423.2 | 1225 | accccggtacttcaaaacagaat | 408089 | - | see also 9618 line |
| 49991 | HABP4 | NM_014282.1 | 1112 | tccggaaacccgccaatgacatc | 340022 | - | see also 22140 line |
| 49992 | HABP4 | NM_014282.1 | 793 | acctggggatcgggtaaagatac | 340017 | - | see also 22140 line |
| 49993 | HAGHL | NM_032304.1 | 262 | tgctcgaggacaactacatgtac | 399602 | - | see also 22141 line |
| 49994 | HAGHL | NM_032304.1 | 257 | ccccgtgctcgaggacaactaca | 399601 | - | see also 22141 line |
| 49995 | HATH6 | NM_032827.3 | 402 | gcgcgaacggctataaaactttc | 401729 | - | see also 9297 line |
| 49996 | HATH6 | NM_032827.3 | 911 | tcctccgcgtcgcctaggaaacg | 401736 | - | see also 9297 line |
| 49997 | HATH6 | NM_032827.3 | 401 | cgcgcgaacggctataaaacttt | 401728 | - | see also 9297 line |
| 49998 | HBXIP | NM_006402.2 | 165 | gacgttgcaccgtgatcaatttt | 335387 | - | see also 22144 line |
| 49999 | HBXIP | NM_006402.2 | 497 | gtgtgtctagaatcagataatgg | 335391 | - | see also 22144 line |
| 50000 | HCA112 | NM_018487.2 | 496 | ttccgatatggctactcttatta | 372040 | - | - | - | - |
| 50001 | HCA112 | NM_018487.2 | 84 | aacagccgacagtgatgagatgg | 372035 | - | see also 50000 line |
| 50002 | HCA112 | NM_018487.2 | 378 | agctgctgccttcatttacgaga | 372038 | - | see also 50000 line |
| 50003 | HCA3 | NM_138703.2 | 669 | ttccagatcaccgataggatagc | 408635 | - | see also 22145 line |
| 50004 | HCA3 | NM_138703.2 | 1137 | ttggtccagttggctattagtgt | 408644 | - | see also 22145 line |

Figure 46

| | | | | | | | |
|---|---|---|---|---|---|---|---|
| 50005 | HCCA2 | NM_053005.2 | 211 | cagcaacaccacgacgttttcc | 405762 | - | see also 9480 line |
| 50006 | HCCA2 | NM_053005.2 | 358 | ggccccacagtacgttgacttcg | 405764 | - | see also 9480 line |
| 50007 | HCCR1 | NM_015416.2 | 931 | atctccgtggcctgaattctacg | 352360 | - | see also 22147 line |
| 50008 | HCCR1 | NM_015416.2 | 826 | gacatcgtttgaagactcataca | 352358 | - | see also 22147 line |
| 50009 | HCDI | NM_020195.1 | 281 | cccaactccacgggaatataaac | 376061 | - | see also 22148 line |
| 50010 | HCDI | NM_020195.1 | 280 | ccccaactccacgggaatataaa | 376060 | - | see also 22148 line |
| 50011 | HCDI | NM_020195.1 | 284 | aactccacgggaatataaacaat | 376062 | - | see also 22148 line |
| 50012 | HCFC1R1 | NM_017885.1 | 396 | cccagctggggacataatggagc | 363372 | - | - | - | - |
| 50013 | HCG4 | NM_018985.1 | 1224 | cccgtccctctctcttcactttg | 374066 | - | see also 22149 line |
| 50014 | HCG4 | NM_018985.1 | 1229 | ccctctctcttcactttgcatca | 374067 | - | see also 22149 line |
| 50015 | HCG4 | NM_018985.1 | 406 | aggttcactcggtaaaactgtgc | 374063 | - | see also 22149 line |
| 50016 | HCMOGT-1 | NM_152904.1 | 2075 | accgggctgtcaagttacacaat | 421606 | - | see also 22150 line |
| 50017 | HCMOGT-1 | NM_152904.1 | 1133 | ccccaaacagcgtaagtgaattg | 421588 | - | see also 22150 line |
| 50018 | HCNGP | NM_013260.3 | 662 | aggagcgaacaaaaattgagttt | 338494 | - | see also 22151 line |
| 50019 | HCNGP | NM_013260.3 | 381 | accccctggcagatgttcaaatc | 338488 | - | see also 22151 line |
| 50020 | HDCMA18P | NM_016648.1 | 593 | aagagtatttagcgctacaaaaa | 358688 | - | see also 22152 line |
| 50021 | HDCMA18P | NM_016648.1 | 1139 | aagcgagtaaacatataagattt | 358708 | - | see also 22152 line |
| 50022 | HE9 | NM_172000.1 | 469 | tgctgatggtgtcacatgttaaa | 424171 | - | see also 22153 line |
| 50023 | HE9 | NM_172000.1 | 502 | gggtggagctgcacgtttattct | 424175 | - | see also 22153 line |
| 50024 | HEBP2 | NM_014320.1 | 459 | atccagacgggctttacgaaact | 340174 | - | see also 22154 line |
| 50025 | HEBP2 | NM_014320.1 | 422 | cagcacgtccgtggagtctatgg | 340173 | - | see also 22154 line |
| 50026 | HEIL1 | NM_032362.1 | 367 | tcgctcaatcctgcaatgtgtca | 400001 | - | see also 22155 line |
| 50027 | HEIL1 | NM_032362.1 | 40 | gacaacttgtacagagaatataa | 399997 | - | see also 22155 line |
| 50028 | HELB | NM_033647.2 | 201 | ccgggtgcctccgcgtttctatt | 404156 | - | see also 22156 line |
| 50029 | HELB | NM_033647.2 | 1474 | cacaatcgttagccgtcttttta | 404193 | - | see also 22156 line |
| 50030 | HEMGN | NM_018437.3 | 795 | taccaagatatggctaaacgaga | 371527 | - | see also 22157 line |
| 50031 | HEMGN | NM_018437.3 | 902 | aggatgctctcttcaagcatatc | 371531 | - | see also 22157 line |
| 50032 | HFL-EDDG1 | NM_006553.2 | 521 | tgctatcgagtggataattgaac | 335810 | - | see also 22158 line |
| 50033 | HFL-EDDG1 | NM_006553.2 | 483 | gccaatacgatatcatccaatgc | 335807 | - | see also 22158 line |
| 50034 | HHIP | NM_022475.1 | 2180 | tacgtgtttggagatcgtaatgg | 383239 | - | see also 8397 line |
| 50035 | HHIP | NM_022475.1 | 2726 | tacttgctggatctaacaagtta | 383251 | - | see also 8397 line |
| 50036 | HHL | NM_014857.2 | 1982 | tggtcaagaatcgctctataaga | 344896 | - | see also 6054 line |

Figure 46

| 50037 | HHLA1 | NM_005712.1 | 1679 | aaggcaacagcccccagatatcc | 334206 | - | see also 22161 line | | |
|---|---|---|---|---|---|---|---|---|---|
| 50038 | HHLA1 | NM_005712.1 | 1548 | ccgaggaagccatgaacactaca | 334202 | - | see also 22161 line | | |
| 50039 | HHLA3 | NM_007071.1 | 269 | tacttcgtgccgtcttgtcaacg | 336883 | - | - | - | - |
| 50040 | HHLA3 | NM_007071.1 | 127 | ttcggtgcatgttataaacaacc | 336882 | - | see also 50039 line | | |
| 50041 | hIAN2 | NM_024711.2 | 337 | cagggacgtcttcgagtctaaac | 389490 | - | see also 22162 line | | |
| 50042 | hIAN2 | NM_024711.2 | 732 | ggcgccattgcggcttcaacaac | 389493 | - | see also 22162 line | | |
| 50043 | hIAN6 | NM_175571.1 | 1836 | tacgaaatacgcgattatgctgt | 429052 | - | see also 22163 line | | |
| 50044 | hIAN6 | NM_175571.1 | 1922 | aagcccttcggcgcattttaaa | 429053 | - | see also 22163 line | | |
| 50045 | hIAN7 | NM_153236.2 | 694 | gtgcaacgaaggggcttactttt | 422451 | - | see also 22164 line | | |
| 50046 | hIAN7 | NM_153236.2 | 555 | atgcggatgtgggcctaaaaagc | 422450 | - | see also 22164 line | | |
| 50047 | hIAN7 | NM_153236.2 | 698 | aacgaaggggcttacttttctga | 422453 | - | see also 22164 line | | |
| 50048 | HIC1 | NM_006497.1 | 384 | ctcgtggcgctgtgcaagaaacg | 335695 | - | see also 22165 line | | |
| 50049 | HIF1AN | NM_017902.1 | 852 | tggcatcacatagagtcattact | 363737 | - | see also 22166 line | | |
| 50050 | HIF1AN | NM_017902.1 | 207 | gtgctgaccgacacaaatcttgt | 363721 | - | see also 22166 line | | |
| 50051 | HILS1 | NM_194072.1 | 550 | ggcccgaaatgcctatcacttca | 436064 | - | see also 22167 line | | |
| 50052 | HILS1 | NM_194072.1 | 479 | gacaaggggacctgcaagtatgt | 436062 | - | see also 22167 line | | |
| 50053 | HIMAP2 | NM_015660.1 | 1080 | aacgcaagactcctaggttatag | 354958 | - | see also 22168 line | | |
| 50054 | HIMAP2 | NM_015660.1 | 496 | tgccatgggacacacaattgtcc | 354944 | - | see also 22168 line | | |
| 50055 | HIMAP4 | NM_018326.2 | 1050 | tacgtctgttcgcggaagattaa | 370409 | - | see also 22169 line | | |
| 50056 | HIMAP4 | NM_018326.2 | 634 | ccgctactgtgcgttaaacaaca | 370400 | - | see also 22169 line | | |
| 50057 | HINT2 | NM_032593.1 | 448 | ctgggtgcacaatctgtgtatca | 400966 | - | see also 22170 line | | |
| 50058 | HINT2 | NM_032593.1 | 450 | gggtgcacaatctgtgtatcatc | 400967 | - | see also 22170 line | | |
| 50059 | HIP14 | NM_015336.1 | 1990 | aaccggtgaggtccaaacattgt | 351677 | - | see also 6369 line | | |
| 50060 | HIP14L | NM_019028.1 | 267 | aagctactcaatacggaattttt | 374522 | - | see also 7667 line | | |
| 50061 | HIP14L | NM_019028.1 | 231 | ctcttatagaggactctagtaac | 374519 | - | see also 7667 line | | |
| 50062 | HIRIP5 | NM_015700.1 | 793 | ggcgtagaacaggttatggatga | 355153 | - | see also 22173 line | | |
| 50063 | HIRIP5 | NM_015700.1 | 647 | atggaggggatgtaatctacaaa | 355148 | - | see also 22173 line | | |
| 50064 | HIS1 | NM_006460.1 | 1333 | aggaggagccggatctcaaaacc | 335592 | - | see also 22174 line | | |
| 50065 | HIS1 | NM_006460.1 | 1457 | aggggacggcagcgagtttctgc | 335593 | - | see also 22174 line | | |
| 50066 | HIST1H4B | NM_003544.2 | 134 | agcgaatttccggtttgatttat | 331766 | - | see also 22175 line | | |
| 50067 | HIST1H4H | NM_003543.3 | 7 | ggccgtggtaaaggtggaaaagg | 331764 | - | - | - | - |
| 50068 | HIST1H4L | NM_003546.2 | 163 | gacacgcggagttcttaaagtgt | 331769 | - | see also 22176 line | | |
| 50069 | HIVEP2 | NM_006734.2 | 5315 | ctggaaccggcaagcttacatca | 336252 | - | see also 4756 line | | |
| 50070 | HIVEP2 | NM_006734.2 | 5629 | atccgtacccatactgatgttcg | 336264 | - | see also 4756 line | | |
| 50071 | HIWI2 | NM_152431.1 | 2433 | cacgtaacgaatggtatgacttt | 416754 | - | see also 22179 line | | |
| 50072 | HIWI2 | NM_152431.1 | 2428 | agcaacacgtaacgaatggtatg | 416753 | - | see also 22179 line | | |
| 50073 | HKR2 | NM_181846.1 | 815 | ctggtgcctcgaggaacagttct | 433809 | - | see also 22180 line | | |

Figure 46

| | | | | | | | |
|---|---|---|---|---|---|---|---|
| 50074 | HKR2 | NM_181846.1 | 653 | ccctggaccgcctttgtcaag | 433807 | - | see also 22180 line |
| 50075 | HLC-8 | NM_017941.3 | 1670 | aaccccctgccggacttacaacc | 364470 | - | see also 22181 line |
| 50076 | HLC-8 | NM_017941.3 | 1327 | gagagtccgaggacagttatc | 364459 | - | see also 22181 line |
| 50077 | HMGA1L4 | NM_031288.1 | 295 | tcccctatggtgtggataatg | 396500 | - | see also 22182 line |
| 50078 | HMGA1L4 | NM_031288.1 | 296 | cccctatggtgtggataatga | 396501 | - | see also 22182 line |
| 50079 | HN1 | NM_016185.1 | 145 | caggaatagctccgagttttgc | 356545 | - | - |
| 50080 | HN1 | NM_016185.1 | 177 | gttggtggatccaattttcatta | 356547 | - | see also 50079 line |
| 50081 | HN1 | NM_016185.1 | 171 | ctccaggttggtgatccaatttt | 356546 | - | see also 50079 line |
| 50082 | HNLF | NM_182547.2 | 341 | cccggtgaccatcaaatctgtct | 434327 | - | see also 22183 line |
| 50083 | HNLF | NM_182547.2 | 539 | gagcaggattaccaaaggtatcg | 434331 | - | see also 22183 line |
| 50084 | HNOEL-iso | NM_020190.1 | 1133 | aacgggcagcactccctatttt | 375917 | - | see also 22184 line |
| 50085 | HNOEL-iso | NM_020190.1 | 1047 | gaccctctatgtcgtctataaca | 375916 | - | see also 22184 line |
| 50086 | HOM-TES-85 | NM_016383.1 | 99 | ctctagacgacattataatctat | 357258 | - | see also 22185 line |
| 50087 | HOM-TES-85 | NM_016383.1 | 63 | atcccagtcggaaaaagttaac | 357254 | - | see also 22185 line |
| 50088 | HOM-TES-85 | NM_016383.1 | 64 | tcccagtcggaaaaagttaact | 357255 | - | see also 22185 line |
| 50089 | HOOK1 | NM_015888.3 | 1506 | ttgagcagcgtgatctttgaaa | 355339 | - | see also 6562 line |
| 50090 | HOOK1 | NM_015888.3 | 1790 | ctgagcaaagagcgtattagaga | 355349 | - | see also 6562 line |
| 50091 | HOXA13 | NM_000522.2 | 1013 | aacgggaatacgccacgaataaa | 329695 | - | see also 400 line |
| 50092 | HOXA13 | NM_000522.2 | 1021 | tacgccacgaataaattcattac | 329697 | - | see also 400 line |
| 50093 | HOXD11 | NM_021192.2 | 114 | gtcgtcctgccagatgactttcc | 380742 | - | see also 8130 line |
| 50094 | HOXD12 | NM_021193.2 | 719 | aacgccaaggaattgtccaatagg | 380749 | - | see also 22188 line |
| 50095 | HOXD12 | NM_021193.2 | 279 | acccgaagagcaggctaagttct | 380746 | - | see also 22188 line |
| 50096 | HPS3 | NM_032383.3 | 2664 | cagtgtaatatcatctgattc | 400167 | - | see also 22189 line |
| 50097 | HPS3 | NM_032383.3 | 224 | ggggcgttgacgcgctttcgtgg | 400097 | - | see also 22189 line |
| 50098 | HPS5 | NM_007216.3 | 1685 | ctccgttcgcattccattac | 337154 | - | see also 22190 line |
| 50099 | HPS5 | NM_007216.3 | 2674 | tgctacccgttgtatgaaaagt | 337190 | - | see also 22190 line |
| 50100 | HPS6 | NM_024747.4 | 1463 | cgggattaccgaggcttagaaca | 389899 | - | see also 22191 line |
| 50101 | HPS6 | NM_024747.4 | 783 | ttggtctcctacagtaagagt | 389893 | - | see also 22191 line |
| 50102 | HRASLS | NM_020386.2 | 808 | caggccaaccgagcgataagtac | 377364 | - | see also 22192 line |
| 50103 | HRASLS | NM_020386.2 | 819 | agcgataagtaccggttgagtttg | 377366 | - | see also 22192 line |
| 50104 | HRASLS2 | NM_017878.1 | 210 | ctgtctgccctgaccaaccaaagc | 363369 | - | see also 22193 line |
| 50105 | HRASLS2 | NM_017878.1 | 314 | accactgccttccaacaaaatcg | 363370 | - | see also 22193 line |
| 50106 | HRD1 | NM_032431.1 | 558 | tcgtcagccacgcctatcacagc | 400224 | - | see also 9187 line |

Figure 46

| | | | | | | | | | | |
|---|---|---|---|---|---|---|---|---|---|---|
| 50107 | HRLP5 | NM_054108.1 | 298 | tgccccactcaggtctaaatagc | 406027 | - | see also 22195 line | | | |
| 50108 | HRLP5 | NM_054108.1 | 958 | cagctgtagtggatagcataaag | 406035 | - | see also 22195 line | | | |
| 50109 | HS322B1A | NM_015371.1 | 73 | agcatgacatccagttcaacttt | 351873 | - | - | - | - | |
| 50110 | HS747E2A | NM_015370.1 | 721 | gtcggaggacacgctaaccatcc | 351871 | - | - | - | - | |
| 50111 | HS747E2A | NM_015370.1 | 176 | ctccgacagtccatcttattaaa | 351862 | - | see also 50110 line | | | |
| 50112 | HS747E2A | NM_015370.1 | 349 | ctccctggttaagcttgtactca | 351866 | - | see also 50110 line | | | |
| 50113 | HSA275986 | NM_018403.1 | 247 | gggaccttattcgtatatcgaag | 371143 | - | see also 7448 line | | | |
| 50114 | HSA275986 | NM_018403.1 | 1255 | tccctcccaagcgttgatcttct | 371162 | - | see also 7448 line | | | |
| 50115 | HSA277841 | NM_018553.1 | 1112 | gccggttagataacttaccatct | 372210 | - | see also 7508 line | | | |
| 50116 | HSCARG | NM_020677.2 | 603 | caccttcatcgtgaccaattact | 377923 | - | see also 22197 line | | | |
| 50117 | HSCARG | NM_020677.2 | 829 | gtgcggctgccctgctattttga | 377924 | - | see also 22197 line | | | |
| 50118 | HSPBAP1 | NM_024610.3 | 285 | agcacgacactggaatgctaaat | 387839 | - | see also 8667 line | | | |
| 50119 | HSPBAP1 | NM_024610.3 | 888 | gtggcattacgtagaatccattg | 387851 | - | see also 8667 line | | | |
| 50120 | HSPC009 | NM_014019.1 | 112 | acccgagcaactgcattccatgc | 339205 | - | - | - | - | |
| 50121 | HSPC023 | NM_014047.1 | 96 | aagaaaaggcggtcgtgttatcg | 339416 | - | - | - | - | |
| 50122 | HSPC023 | NM_014047.1 | 38 | cgcacaaacccgcaaagagtaag | 339415 | - | see also 50121 line | | | |
| 50123 | HSPC043 | NM_021218.1 | 311 | cactctcgagaccctctcttaat | 380784 | - | see also 22199 line | | | |
| 50124 | HSPC043 | NM_021218.1 | 438 | ctctgcatttgggaaccttattc | 380786 | - | see also 22199 line | | | |
| 50125 | HSPC047 | NM_014147.1 | 215 | gggagagccaagtttgcattttc | 339626 | - | - | - | - | |
| 50126 | HSPC063 | NM_014155.1 | 660 | aagcgcaaaagctacattgttat | 339697 | - | see also 5523 line | | | |
| 50127 | HSPC063 | NM_014155.1 | 697 | ctgtaaagtgtggcacacaaaca | 339699 | - | see also 5523 line | | | |
| 50128 | HSPC065 | NM_014157.2 | 1120 | atcgtaaggcttggaatcgaatg | 339732 | - | see also 22202 line | | | |
| 50129 | HSPC065 | NM_014157.2 | 1121 | tcgtaaggcttggaatcgaatga | 339733 | - | see also 22202 line | | | |
| 50130 | HSPC072 | NM_014162.1 | 444 | aaggaacacaactggttcaaata | 339768 | - | see also 22203 line | | | |
| 50131 | HSPC072 | NM_014162.1 | 474 | gcctccaaatatccagatctaaa | 339770 | - | see also 22203 line | | | |
| 50132 | HSPC109 | NM_016390.2 | 306 | acctacttggccggtcagattgc | 357275 | - | see also 22204 line | | | |
| 50133 | HSPC109 | NM_016390.2 | 62 | gggtgaacacggccaaaggattg | 357274 | - | see also 22204 line | | | |
| 50134 | HSPC128 | NM_014167.1 | 91 | gtcgggtacaggaataagaatgt | 339792 | - | see also 22205 line | | | |
| 50135 | HSPC128 | NM_014167.1 | 278 | atcgatacccagataatctgaaa | 339798 | - | see also 22205 line | | | |
| 50136 | HSPC129 | NM_016396.1 | 393 | aacgtgatatagataacaatttg | 357285 | - | see also 22206 line | | | |
| 50137 | HSPC129 | NM_016396.1 | 1284 | accggcttttccgtgaacattgt | 357317 | - | see also 22206 line | | | |
| 50138 | HSPC132 | NM_016399.2 | 99 | atcgctggttcgccgagaaattt | 357372 | - | see also 22207 line | | | |
| 50139 | HSPC132 | NM_016399.2 | 101 | cgctggttcgccgagaaatttct | 357374 | - | see also 22207 line | | | |
| 50140 | HSPC135 | NM_014170.1 | 418 | cggctcgcggtgtgtttataggg | 339815 | - | see also 22208 line | | | |

Figure 46

| | | | | | | | |
|---|---|---|---|---|---|---|---|
| 50141 | HSPC135 | NM_014170.1 | 416 | tccggctcgcggtgtgttttata | 339813 | - | see also 22208 line |
| 50142 | HSPC138 | NM_016401.2 | 351 | atgggaggatctgtctactttc | 357378 | - | see also 22209 line |
| 50143 | HSPC138 | NM_016401.2 | 352 | tgggaggatctgtctactttct | 357379 | - | see also 22209 line |
| 50144 | HSPC142 | NM_014173.1 | 511 | gagatgttcgtgcggacaaaca | 339839 | - | see also 22210 line |
| 50145 | HSPC142 | NM_014173.1 | 1110 | gtctgaaccatccctgtacatct | 339844 | - | see also 22210 line |
| 50146 | HSPC154 | NM_014177.1 | 213 | ttctacgagccgtacagtatacg | 339864 | - | see also 22211 line |
| 50147 | HSPC154 | NM_014177.1 | 349 | aacgctgcgacctagatgtattc | 339869 | - | see also 22211 line |
| 50148 | HSPC157 | NM_014179.1 | 658 | acccatgggaaatctgaacttcc | 339903 | - | see also 22212 line |
| 50149 | HSPC157 | NM_014179.1 | 782 | gtcctcttgttaggacaatcagc | 339906 | - | see also 22212 line |
| 50150 | HSPC159 | NM_014181.1 | 467 | accatcgcattcaaacgttatct | 339936 | - | see also 5527 line |
| 50151 | HSPC159 | NM_014181.1 | 118 | tacttcccacgactgatagttcc | 339923 | - | see also 5527 line |
| 50152 | HSPC166 | NM_014186.1 | 246 | gtcctctgccgaggcaattctgg | 339963 | - | see also 22213 line |
| 50153 | HSPC166 | NM_014186.1 | 28 | ctgacagcggagcattttgcagc | 339960 | - | see also 22213 line |
| 50154 | HSPC166 | NM_014186.1 | 323 | tcctagaacatgtgtctacttgg | 339964 | - | see also 22213 line |
| 50155 | HSPC189 | NM_016535.2 | 322 | cccaaccactacctgcttattga | 358050 | - | - | - | - |
| 50156 | HSPC189 | NM_016535.2 | 326 | accactacctgcttattgacact | 358051 | - | see also 50155 line |
| 50157 | HSPC196 | NM_016464.2 | 547 | gactagcagcagtgttgtactgc | 357589 | - | see also 22215 line |
| 50158 | HSPC196 | NM_016464.2 | 178 | tgctccagaccagtaactacagc | 357584 | - | see also 22215 line |
| 50159 | HSPC242 | NM_016498.2 | 376 | ctggcggatgccattgacaaagg | 357885 | - | see also 22216 line |
| 50160 | HSPC268 | NM_197964.1 | 155 | ggcctatcggtaccttgtgaagg | 436919 | - | see also 22217 line |
| 50161 | HSPC268 | NM_197964.1 | 187 | cacatcgggtcaccagtgaaaag | 436920 | - | see also 22217 line |
| 50162 | HSU15552 | NM_014597.1 | 1880 | ttccaccatatagtgaatcaaag | 340958 | - | see also 22218 line |
| 50163 | HSU15552 | NM_014597.1 | 1875 | ctgtgttccaccatatagtgaat | 340957 | - | see also 22218 line |
| 50164 | HSU79266 | NM_013299.1 | 969 | agccctgcatgaggttctacagc | 338682 | - | - | - | - |
| 50165 | HSU79274 | NM_013300.1 | 882 | agctcccgttactcacagaaaca | 338692 | - | see also 22219 line |
| 50166 | HSU79274 | NM_013300.1 | 876 | cgccttagctcccgttactcaca | 338691 | - | see also 22219 line |
| 50167 | HSU79274 | NM_013300.1 | 917 | cactacatggcgacatcaactgg | 338694 | - | see also 22219 line |
| 50168 | hSyn | NM_018157.1 | 307 | tgctgcgactagccaagctaaat | 367782 | - | see also 7288 line |
| 50169 | hSyn | NM_018157.1 | 639 | aagtggaccgatgagtatgaatc | 367795 | - | see also 7288 line |
| 50170 | HT007 | NM_018480.2 | 441 | cagacgctgcttcaaggttaaac | 372021 | - | see also 22221 line |
| 50171 | HT007 | NM_018480.2 | 753 | ggcgattcctctagtctttcaga | 372032 | - | see also 22221 line |
| 50172 | HT009 | NM_018470.1 | 485 | gccgagtaacaggcatgaagtga | 371925 | - | see also 22222 line |
| 50173 | HT009 | NM_018470.1 | 640 | atcggaaatgcctacacaaatcg | 371929 | - | see also 22222 line |
| 50174 | HT011 | NM_018472.1 | 256 | ttggagtcgggctgatcgaaaag | 371930 | - | see also 22223 line |
| 50175 | HT011 | NM_018472.1 | 257 | tggagtcgggctgatcgaaaagg | 371931 | - | see also 22223 line |
| 50176 | HT011 | NM_018472.1 | 768 | gtggtgtatcaccccaagataga | 371949 | - | see also 22223 line |
| 50177 | HT014 | NM_020362.2 | 611 | cagatacgacaaaggtcttttat | 376902 | - | see also 7847 line |

Figure 46

| 50178 | HT017 | NM_020678.2 | 566 | ttgggagaacctaactatacttg | 377938 | - | see also 22225 line |
|---|---|---|---|---|---|---|---|
| 50179 | HT017 | NM_020678.2 | 287 | gactcgaacgctgcatttacaag | 377930 | - | see also 22225 line |
| 50180 | HT021 | NM_020685.3 | 141 | ctcaagaaattcgcctttctaaa | 377977 | - | see also 22226 line |
| 50181 | HT021 | NM_020685.3 | 432 | taggaagagcaccatatcctttt | 377982 | - | see also 22226 line |
| 50182 | HT036 | NM_031207.2 | 407 | accccagggagctgatcgaatag | 395959 | - | see also 22227 line |
| 50183 | HT036 | NM_031207.2 | 602 | aagacccaacctccaattacaaa | 395964 | - | see also 22227 line |
| 50184 | hT2R55 | NM_181429.1 | 379 | aacggaatgattgttatgcttct | 432103 | - | see also 22228 line |
| 50185 | hT2R55 | NM_181429.1 | 899 | ggcatcttaggaactatacaaaa | 432113 | - | see also 22228 line |
| 50186 | HTPAP | NM_032483.2 | 598 | gtcccgcacatgtgactacaagc | 400369 | - | see also 22229 line |
| 50187 | HTPAP | NM_032483.2 | 166 | caccaagccgatgtttgttattg | 400360 | - | see also 22229 line |
| 50188 | HTR1A | NM_000524.1 | 545 | accccgacgcatgcaccattagc | 329705 | - | see also 22230 line |
| 50189 | HTR1A | NM_000524.1 | 1185 | ccccgtcatttacgcatacttca | 329713 | - | see also 22230 line |
| 50190 | HTR3D | NM_182537.1 | 391 | gcccagcccctacgtggtaaact | 434229 | - | see also 22231 line |
| 50191 | HTR3D | NM_182537.1 | 397 | cccctacgtggtaaactttctgg | 434231 | - | see also 22231 line |
| 50192 | HTR3E | NM_182589.1 | 654 | ggcctcacagcatatgtaagtaa | 434551 | - | see also 22232 line |
| 50193 | HTR3E | NM_182589.1 | 563 | gggcatcacgaagatgagtatgg | 434549 | - | see also 22232 line |
| 50194 | HUMAGCGB | NM_013286.2 | 744 | tggtcgcaaccccattaagatag | 338667 | - | see also 22233 line |
| 50195 | HUMAGCGB | NM_013286.2 | 864 | gagcattcggaccattgatcacg | 338670 | - | see also 22233 line |
| 50196 | HUMPPA | NM_014603.1 | 1723 | ggcgctcttcaaagagatcttct | 340991 | - | see also 22234 line |
| 50197 | HYPB | NM_012271.1 | 3695 | tggagaggtactcgatcataaag | 337879 | - | see also 22235 line |
| 50198 | HYPB | NM_012271.1 | 574 | gacgagggtcatcatattctaag | 337798 | - | see also 22235 line |
| 50199 | HYPC | NM_012272.1 | 1799 | tgggatgagtagtgtcaacttcc | 337931 | - | see also 5247 line |
| 50200 | HYPC | NM_012272.1 | 2144 | ctccacccctctggacttattca | 337932 | - | see also 5247 line |
| 50201 | IAN4L1 | NM_018384.2 | 493 | aggacgtgccaggtgaaaacagg | 370977 | - | see also 22237 line |
| 50202 | IAN4L1 | NM_018384.2 | 374 | caccaccggcattgaggattatc | 370974 | - | see also 22237 line |
| 50203 | IBRDC1 | NM_152553.2 | 999 | gccatagcggttgtaatcgttga | 418067 | - | see also 22238 line |
| 50204 | IBRDC1 | NM_152553.2 | 998 | ggccatagcggttgtaatcgttg | 418066 | - | see also 22238 line |
| 50205 | IDN3 | NM_015384.2 | 4198 | gacgattgatggataattcgact | 352063 | - | see also 22239 line |
| 50206 | IDN3 | NM_015384.2 | 6015 | gacgtgactatgctcttgtatat | 352115 | - | see also 22239 line |
| 50207 | IER5 | NM_016545.2 | 560 | tggagtcggggacgtttttcagg | 358078 | - | see also 22240 line |
| 50208 | IER5 | NM_016545.2 | 557 | gactggagtcggggacgtttttc | 358077 | - | see also 22240 line |
| 50209 | IFI30 | NM_006332.3 | 579 | ggctgtcgccagacacactatg | 335218 | - | see also 22241 line |
| 50210 | IFI30 | NM_006332.3 | 402 | gtggcaggtgggagttcaagtgc | 335212 | - | see also 22241 line |
| 50211 | IFI44 | NM_006417.2 | 903 | aacggtaacattcgtgatagata | 335477 | - | see also 22242 line |
| 50212 | IFI44 | NM_006417.2 | 913 | ttcgtgatagataccagtttaat | 335480 | - | see also 22242 line |

Figure 46

| | | | | | | | |
|---|---|---|---|---|---|---|---|
| 50213 | IFIT2 | NM_001547.3 | 505 | gggtggacacggttaaagtgtgg | 330352 | - | see also 22243 line |
| 50214 | IFIT2 | NM_001547.3 | 238 | tgcaacctactggcctactaaa | 330349 | - | see also 22243 line |
| 50215 | IFIX | NM_152501.2 | 883 | atcgccatggtactaaatgcaac | 417235 | - | see also 22244 line |
| 50216 | IFIX | NM_152501.2 | 1626 | atcgcctgcaaacttagaatca | 417244 | - | see also 22244 line |
| 50217 | IFRG15 | NM_022347.1 | 115 | ttcttagaccgtattaacagagc | 382634 | - | see also 8337 line |
| 50218 | IFRG15 | NM_022347.1 | 73 | ttgtaccaaactgaacttgttga | 382632 | - | see also 8337 line |
| 50219 | IGJ | NM_144646.2 | 79 | tgggagtcctgccggtttttat | 410714 | - | see also 22246 line |
| 50220 | IGJ | NM_144646.2 | 456 | agctgtggtccccactcgtatatg | 410725 | - | see also 22246 line |
| 50221 | IGRP | NM_021176.1 | 936 | cccgactcacgaagagcatttat | 380604 | - | see also 8116 line |
| 50222 | IGRP | NM_021176.1 | 843 | ctgccgaggggaaataactaca | 380602 | - | see also 8116 line |
| 50223 | IGSF11 | NM_152538.1 | 1264 | gtcccgggtcaacataagactct | 417764 | - | see also 22248 line |
| 50224 | IGSF11 | NM_152538.1 | 1393 | cacgcaacactggacgaattgg | 417767 | - | see also 22248 line |
| 50225 | IGSF11 | NM_152538.1 | 434 | tccaacggtagggtaggatttaca | 417736 | - | see also 22248 line |
| 50226 | IGSF3 | NM_001542.1 | 3528 | ctccaacgacgcactcttctact | 330325 | - | see also 22249 line |
| 50227 | IGSF3 | NM_001542.1 | 3371 | tgcaggcaagccccaagataca | 330319 | - | see also 22249 line |
| 50228 | IGSF4 | NM_014333.2 | 434 | cacgtaatctgatcgatatatc | 340192 | - | see also 5628 line |
| 50229 | IGSF9 | NM_020789.2 | 420 | tccccgaattgaccctgattacg | 379025 | - | see also 7968 line |
| 50230 | IGSF9 | NM_020789.2 | 3387 | cacatctgtggacgagaactatg | 379040 | - | see also 7968 line |
| 50231 | IL17D | NM_138284.1 | 381 | gtcgccctgggcctacagaatct | 407383 | - | - |
| 50232 | IL17RC | NM_032732.2 | 938 | tggaccgcagatcattacccttga | 401302 | - | see also 22252 line |
| 50233 | IL17RC | NM_032732.2 | 831 | aacgtgcatccggttctgaatgt | 401300 | - | see also 22252 line |
| 50234 | IL17RE | NM_144640.2 | 668 | gggctattccggtgaccatatct | 410622 | - | see also 22253 line |
| 50235 | IL17RE | NM_144640.2 | 548 | agggacttcgtctaaaaggacc | 410620 | - | see also 22253 line |
| 50236 | IL23A | NM_016584.2 | 471 | agctgctaggatcggatatttc | 358231 | - | see also 22254 line |
| 50237 | IL23A | NM_016584.2 | 474 | tgctaggatggatattttcaca | 358232 | - | see also 22254 line |
| 50238 | IL23A | NM_016584.2 | 440 | aggatccaccagggtctgatttt | 358228 | - | see also 22254 line |
| 50239 | IL29 | NM_172140.1 | 626 | ctcacgcgagacctcaaatatgt | 424271 | - | - |
| 50240 | IL29 | NM_172140.1 | 197 | tgccacattggcaggttcaaatc | 424269 | - | see also 50239 line |
| 50241 | IL29 | NM_172140.1 | 200 | cacattggcaggttcaaatctct | 424270 | - | see also 50239 line |
| 50242 | ILT10 | NM_024317.2 | 1116 | acgggagcagttggacactttcc | 386249 | - | see also 22255 line |
| 50243 | ILT7 | NM_012276.2 | 742 | gtcggctacatcagatacactct | 337952 | - | see also 22256 line |
| 50244 | ILT7 | NM_012276.2 | 577 | ttcagatgctacggctatgaaaa | 337949 | - | see also 22256 line |
| 50245 | IMAGE3451454 | NM_053052.2 | 1332 | ctcaccttacgaaattagcatcc | 405899 | - | see also 22257 line |
| 50246 | IMAGE3451454 | NM_053052.2 | 1348 | agcatccgccagccgcgttattgg | 405901 | - | see also 22257 line |

Figure 46

| | | | | | | | |
|---|---|---|---|---|---|---|---|
| 50247 | IMAGE3455200 | NM_024006.2 | 304 | cactacagctattgttaggttgc | 385461 | - | see also 22258 line |
| 50248 | IMAGE3455200 | NM_024006.2 | 257 | cagcatcctcaatcaatccaaca | 385460 | - | see also 22258 line |
| 50249 | IMAP1 | NM_130759.2 | 635 | gccgggtctgtgcctttgattaac | 406924 | - | see also 22259 line |
| 50250 | IMAP1 | NM_130759.2 | 230 | tgggccagacggttcttctcc | 406919 | - | see also 22259 line |
| 50251 | IMMT | NM_006839.1 | 403 | ttcagtcggtccactaaaaatc | 336426 | - | see also 4794 line |
| 50252 | IMMT | NM_006839.1 | 1313 | tgcacatgtcgtattgatcagc | 336445 | - | see also 4794 line |
| 50253 | IMP-2 | NM_006548.3 | 425 | gccgttgtcaacgtcacatatgc | 335753 | - | see also 4590 line |
| 50254 | IMP-2 | NM_006548.3 | 738 | cccgggtagatatccatagaaaa | 335760 | - | see also 4590 line |
| 50255 | IMP4 | NM_033416.1 | 703 | tccggcaccatgttataagaag | 403834 | - | see also 22262 line |
| 50256 | IMP4 | NM_033416.1 | 262 | atcccaagttatgatcactacc | 403830 | - | see also 22262 line |
| 50257 | INM01 | NM_175075.2 | 786 | aggaactaacttacgattaaaag | 429002 | - | see also 22263 line |
| 50258 | INM01 | NM_175075.2 | 785 | aaggaactaacttacgattaaaa | 429001 | - | see also 22263 line |
| 50259 | INM01 | NM_175075.2 | 897 | aggttgggagcctccaaaactg | 429005 | - | see also 22263 line |
| 50260 | INSIG2 | NM_016133.2 | 531 | atgcggtgtgtagcagtctttgt | 356421 | - | see also 22264 line |
| 50261 | INSIG2 | NM_016133.2 | 829 | gacaactggcaatgtacgaatgt | 356432 | - | see also 22264 line |
| 50262 | INSM2 | NM_032594.2 | 1471 | caggggatccgagattttcgtgt | 400974 | - | see also 22265 line |
| 50263 | INSM2 | NM_032594.2 | 604 | gcccgtctccgccgagtctttcc | 400970 | - | see also 22265 line |
| 50264 | INSM2 | NM_032594.2 | 982 | cacgccactgggggagttcatct | 400971 | - | see also 22265 line |
| 50265 | INVS | NM_014425.2 | 948 | tgcaatataacgtcttatgattaa | 340597 | - | see also 5671 line |
| 50266 | INVS | NM_014425.2 | 1811 | tgttgagccatgaagttgatcc | 340627 | - | see also 5671 line |
| 50267 | IRA1 | NM_024665.2 | 1509 | atgtagaccgagggatatgcatc | 388780 | - | see also 8693 line |
| 50268 | IRA1 | NM_024665.2 | 481 | tgccgtaatgcctgatgtagtac | 388742 | - | see also 8693 line |
| 50269 | IREM2 | NM_181449.1 | 513 | ggggaggttgacccaaaattc | 432137 | - | see also 22268 line |
| 50270 | IREM2 | NM_181449.1 | 341 | atcttactggtgcaaaattcaga | 432136 | - | see also 22268 line |
| 50271 | IRF2BP1 | NM_015649.1 | 1268 | ggcttcttcgggttcaagtacc | 354863 | - | see also 22269 line |
| 50272 | IRF2BP1 | NM_015649.1 | 1277 | gggcttcaagtacctcgaatatg | 354864 | - | see also 22269 line |
| 50273 | IRF2BP2 | NM_182972.1 | 1597 | gtccgtcccttgcacaagttct | 435508 | - | see also 22270 line |
| 50274 | IRF2BP2 | NM_182972.1 | 1313 | ctgatcttagtagcagacaatgc | 435505 | - | see also 22270 line |
| 50275 | IRTA1 | NM_031282.1 | 1189 | gagccatgcaggggatactact | 396481 | - | see also 22271 line |
| 50276 | IRTA1 | NM_031282.1 | 1480 | gtcgttgtatgttgatgtacacc | 396485 | - | see also 22271 line |
| 50277 | IRTA2 | NM_031281.1 | 2878 | agctgcaaccagtgtacactaat | 396465 | - | see also 22272 line |
| 50278 | IRTA2 | NM_031281.1 | 2671 | cagggctgaccgcgaacagaagt | 396461 | - | see also 22272 line |
| 50279 | IRX2 | NM_033267.1 | 1355 | ggctacgagcccaagaaagatgc | 403648 | - | see also 22273 line |
| 50280 | IRX2 | NM_033267.1 | 770 | tcggatgcaaggacaagtatga | 403646 | - | see also 22273 line |
| 50281 | ITGA1 | NM_181501.1 | 117 | ttctacgctgctggtatcattc | 432183 | - | see also 22274 line |

Figure 46

| | | | | | | | |
|---|---|---|---|---|---|---|---|
| 50282 | ITGA1 | NM_181501.1 | 2043 | ccccgagatgtggccgtagttaaa | 432258 | - | see also 22274 line |
| 50283 | ITGA1 | NM_181501.1 | 2216 | taccgtgtcacctagattcact | 432263 | - | see also 22274 line |
| 50284 | ITIH5 | NM_030569.2 | 1565 | aacggctcggagatcatcattgc | 394661 | - | see also 8889 line |
| 50285 | ITIH5 | NM_030569.2 | 1492 | ctctgacatccgcatcgattac | 394660 | - | see also 8889 line |
| 50286 | ITR | NM_180989.3 | 620 | ggcggacccatgccatgcatgatttt | 432066 | - | see also 22276 line |
| 50287 | ITR | NM_180989.3 | 676 | tgttcagctttagctaattaca | 432069 | - | see also 22276 line |
| 50288 | JAM3 | NM_032801.3 | 423 | ggccggtcgtgcagaaatactgg | 401562 | - | see also 22277 line |
| 50289 | JAM3 | NM_032801.3 | 875 | acctgaacattggccggaattatt | 401571 | - | see also 22277 line |
| 50290 | JM1 | NM_014008.1 | 591 | ctggtcgtgccagaattgagttc | 339010 | - | see also 5453 line |
| 50291 | JM1 | NM_014008.1 | 1739 | tgctgttcggaaggcctataagt | 339015 | - | see also 5453 line |
| 50292 | JM11 | NM_033626.2 | 2046 | tcccacactcgaggagtttcacg | 404145 | - | see also 22279 line |
| 50293 | JM11 | NM_033626.2 | 829 | cagccaagaggacgtagttctgc | 404142 | - | see also 22279 line |
| 50294 | JM4 | NM_007213.1 | 435 | tgcgcctgcaacttaagaac | 337118 | - | see also 22280 line |
| 50295 | JM4 | NM_007213.1 | 112 | atgggccacgcgtcatcaaca | 337116 | - | see also 22280 line |
| 50296 | JM5 | NM_007075.1 | 1057 | ggccccttggtcgccaatacttcc | 336943 | - | see also 4945 line |
| 50297 | JM5 | NM_007075.1 | 1056 | tgcgccttcggtcgcaatacttc | 336942 | - | see also 4945 line |
| 50298 | JMJD1 | NM_018433.2 | 3747 | gaccgattaagaaaacgtct | 371472 | - | see also 22282 line |
| 50299 | JMJD1 | NM_018433.2 | 3737 | ctggtatttagaccgatcattaa | 371470 | - | see also 22282 line |
| 50300 | JMY | NM_152405.1 | 510 | agcacgattggatcagttagaag | 416332 | - | see also 9855 line |
| 50301 | JMY | NM_152405.1 | 1791 | cacacttctacccgatacagacc | 416360 | - | see also 9855 line |
| 50302 | JPHL1 | NM_032452.1 | 2224 | gaggacagccctgggtatatga | 400350 | - | see also 22284 line |
| 50303 | JWA | NM_006407.2 | 352 | acgtccttcgccggatgaagaag | 335395 | - | see also 22285 line |
| 50304 | JWA | NM_006407.2 | 400 | tggtcatgttggcgagctattc | 335396 | - | see also 22285 line |
| 50305 | K5B | NM_173352.1 | 1257 | agctgacgagcacgaagctttcc | 424815 | - | see also 22286 line |
| 50306 | K5B | NM_173352.1 | 1345 | gtgaccagccaggtcactatct | 424816 | - | see also 22286 line |
| 50307 | K6IRS3 | NM_175068.2 | 234 | tgggcaggaggctatggatttgg | 428980 | - | see also 22287 line |
| 50308 | K6IRS3 | NM_175068.2 | 233 | gtgggcaggaggctatggatttg | 428979 | - | see also 22287 line |
| 50309 | K6IRS4 | NM_175053.2 | 210 | agggaatcgcgtatttctttca | 428896 | - | see also 22288 line |
| 50310 | K6IRS4 | NM_175053.2 | 981 | cgctgaggtccgcatgcattatg | 428900 | - | see also 22288 line |
| 50311 | KAB | NM_014812.1 | 563 | gccccgtggtactccattatatg | 344217 | - | see also 22289 line |
| 50312 | KAB | NM_014812.1 | 561 | atgccccgttggtactccattata | 344215 | - | see also 22289 line |
| 50313 | KCMF1 | NM_020122.2 | 73 | ttcgaggtcgcagatataagtgt | 375576 | - | see also 7755 line |
| 50314 | KCMF1 | NM_020122.2 | 512 | tcctcgttcgctagatcaaaca | 375592 | - | see also 7755 line |
| 50315 | KCMF1 | NM_020122.2 | 511 | gtcctcgtcgctagatcaaac | 375591 | - | see also 7755 line |
| 50316 | KCNJ11 | NM_000525.2 | 1460 | cagcgctttgtgccattgtagc | 329720 | - | see also 22290 line |
| 50317 | KCNJ11 | NM_000525.2 | 1134 | acctccgcaagagagcatgatcatc | 329717 | - | see also 22290 line |
| 50318 | KCP2 | NM_173852.2 | 400 | tggtctgtactatcaacaaga | 428502 | - | see also 10122 line |

Figure 46

| 50319 | KCP2 | NM_173852.2 | 270 | atcttgtctttggcaaaggattc | 428500 | - | see also 10122 line | | |
|---|---|---|---|---|---|---|---|---|---|
| 50320 | KCP3 | NM_173853.2 | 712 | tccgggcatcacagagaagttgg | 428507 | - | - | voltage-gated potassium channel | - |
| 50321 | KCP3 | NM_173853.2 | 481 | accccacaagaatctatgataca | 428504 | - | see also 50320 line | | |
| 50322 | KCP3 | NM_173853.2 | 480 | gaccccacaagaatctatgatac | 428503 | - | see also 50320 line | | |
| 50323 | KCTD4 | NM_198404.1 | 595 | cacgatcgttcacaaggattaag | 437745 | - | see also 10322 line | | |
| 50324 | KCTD4 | NM_198404.1 | 596 | acgatcgttcacaaggattaaga | 437746 | - | see also 10322 line | | |
| 50325 | KCTD8 | NM_198353.1 | 325 | tgcccattagcgagatggtttcc | 437680 | - | see also 10316 line | | |
| 50326 | KCTD8 | NM_198353.1 | 1449 | ttggatcgccctctaaaaaagc | 437691 | - | see also 10316 line | | |
| 50327 | KCTD9 | NM_017634.2 | 848 | ttctcgtttggaccttcgataca | 359807 | - | see also 22292 line | | |
| 50328 | KCTD9 | NM_017634.2 | 885 | gccaatttaagccgctgtaatct | 359810 | - | see also 22292 line | | |
| 50329 | KDELC1 | NM_024089.1 | 1136 | atgcctacgtacgatttgactga | 385859 | - | see also 22293 line | | |
| 50330 | KDELC1 | NM_024089.1 | 1455 | cagcttatcgcctgccatatttg | 385868 | - | see also 22293 line | | |
| 50331 | KENAE | NM_176816.2 | 835 | ctcgccatgcttgatatcaaaca | 429792 | - | see also 10160 line | | |
| 50332 | KEO4 | NM_006459.2 | 846 | cacaagttgaccccggaatatct | 335575 | - | see also 4531 line | | |
| 50333 | KEO4 | NM_006459.2 | 744 | aagcgcatttctgaaatcgaaga | 335574 | - | see also 4531 line | | |
| 50334 | KIAA0014 | NM_014665.1 | 570 | gccgtagctcgcacatgcattgc | 341957 | - | - | - | - |
| 50335 | KIAA0014 | NM_014665.1 | 148 | tgcacacgcttgtgttcttgagc | 341956 | - | see also 50334 line | | |
| 50336 | KIAA0036 | NM_014642.1 | 1895 | aggatgagcttagtatagaatta | 341614 | - | see also 22296 line | | |
| 50337 | KIAA0036 | NM_014642.1 | 1890 | tccaaaggatgagcttagtatag | 341612 | - | see also 22296 line | | |
| 50338 | KIAA0040 | NM_014656.1 | 1264 | tgcagggaagtacattatctaca | 341805 | - | see also 22297 line | | |
| 50339 | KIAA0040 | NM_014656.1 | 1035 | ttggcagctatgtgtccaatagc | 341797 | - | see also 22297 line | | |
| 50340 | KIAA0053 | NM_014882.1 | 1666 | gacttccacctacgataacgtcc | 345241 | - | see also 6076 line | | |
| 50341 | KIAA0053 | NM_014882.1 | 1662 | aacggacttccacctacgataac | 345239 | - | see also 6076 line | | |
| 50342 | KIAA0063 | NM_014876.3 | 504 | tggtgacacctcacaagaagagc | 345096 | - | see also 22299 line | | |
| 50343 | KIAA0063 | NM_014876.3 | 322 | aggagacaaggccaaatctgaat | 345094 | - | see also 22299 line | | |
| 50344 | KIAA0076 | NM_014780.2 | 3190 | cgcgcattcggggggttagagatc | 343248 | - | see also 22300 line | | |
| 50345 | KIAA0076 | NM_014780.2 | 849 | tggagtgcaggccggatgataca | 343234 | - | see also 22300 line | | |
| 50346 | KIAA0087 | NM_014769.1 | 623 | gccagtctgagacattcaatatt | 343192 | - | see also 22301 line | | |
| 50347 | KIAA0087 | NM_014769.1 | 620 | gaggccagtctgagacattcaat | 343190 | - | see also 22301 line | | |
| 50348 | KIAA0090 | NM_015047.1 | 2333 | ggcgtcactgggcgtatcattca | 347507 | - | see also 6205 line | | |
| 50349 | KIAA0090 | NM_015047.1 | 2791 | ttctcgaatgcgaggtatctaca | 347519 | - | see also 6205 line | | |
| 50350 | KIAA0092 | NM_014679.1 | 173 | ttcggataagcctttccttaata | 342121 | - | see also 5881 line | | |
| 50351 | KIAA0092 | NM_014679.1 | 943 | atgatcgagtcatcaacagtatt | 342136 | - | see also 5881 line | | |
| 50352 | KIAA0095 | NM_014669.1 | 1794 | aagccgagagttcgatatgattc | 342020 | - | see also 5846 line | | |
| 50353 | KIAA0095 | NM_014669.1 | 1014 | tcctgtgtgggcgctaatttact | 342009 | - | see also 5846 line | | |
| 50354 | KIAA0101 | NM_014736.3 | 267 | aggttgtccctaaagattctga | 342888 | - | see also 22305 line | | |

Figure 46

| | | | | | | | |
|---|---|---|---|---|---|---|---|
| 50355 | KIAA0101 | NM_014736.3 | 270 | ttgtccctaaagattctgaaaa | 342889 | - | see also 22305 line |
| 50356 | KIAA0140 | NM_014661.1 | 1022 | gtgtgtccttaacgacaagaagg | 341887 | - | see also 22306 line |
| 50357 | KIAA0140 | NM_014661.1 | 1035 | gacaagaaggtcggtgttaaaag | 341888 | - | see also 22306 line |
| 50358 | KIAA0141 | NM_014773.1 | 972 | agcaaggcggtcctttattatca | 343219 | - | see also 22307 line |
| 50359 | KIAA0141 | NM_014773.1 | 977 | ggcggtcctttattcagttgg | 343221 | - | see also 22307 line |
| 50360 | KIAA0157 | NM_032182.2 | 1039 | caccagtgccggactgaagtatc | 398567 | - | see also 9091 line |
| 50361 | KIAA0157 | NM_032182.2 | 1040 | accagtgccggactgaagtatcc | 398568 | - | see also 9091 line |
| 50362 | KIAA0196 | NM_014846.1 | 1845 | tgccgatactcgaaagttcttc | 344694 | - | see also 6049 line |
| 50363 | KIAA0196 | NM_014846.1 | 1846 | gccgatactcgaaagttcttca | 344695 | - | see also 6049 line |
| 50364 | KIAA0233 | NM_014745.1 | 4142 | gaggcgcatcagtctacgtttta | 342994 | - | see also 22309 line |
| 50365 | KIAA0233 | NM_014745.1 | 4143 | aggcgcatcagtctacgtttag | 342995 | - | see also 22309 line |
| 50366 | KIAA0233 | NM_014745.1 | 4144 | gggcatcagtctacgttttaga | 342996 | - | see also 22309 line |
| 50367 | KIAA0252 | NM_015138.2 | 1263 | aacgcgtgttccgtttagagttt | 348580 | - | see also 22310 line |
| 50368 | KIAA0252 | NM_015138.2 | 1037 | ttgaatcgggttcgattatcacg | 348574 | - | see also 22310 line |
| 50369 | KIAA0258 | NM_014785.1 | 959 | tcctgctacatacaactagaac | 343518 | - | see also 5968 line |
| 50370 | KIAA0258 | NM_014785.1 | 364 | tactccaccgaaaattctattct | 343503 | - | see also 5968 line |
| 50371 | KIAA0266 | NM_021645.1 | 2099 | tccgaattgaggccactatctca | 381016 | - | see also 22312 line |
| 50372 | KIAA0266 | NM_021645.1 | 2450 | ttggcagttcccacaataataga | 381018 | - | see also 22312 line |
| 50373 | KIAA0274 | NM_014845.4 | 541 | atctataaggtcgaagatacaaa | 344577 | - | see also 6047 line |
| 50374 | KIAA0274 | NM_014845.4 | 2727 | ctcggcttctcgcaagataaca | 344652 | - | see also 6047 line |
| 50375 | KIAA0276 | NM_015115.1 | 711 | ttgactttgcacggcaatcaaaa | 348203 | - | see also 6254 line |
| 50376 | KIAA0276 | NM_015115.1 | 514 | accagaaaacgtagttatgcttg | 348201 | - | see also 6254 line |
| 50377 | KIAA0319 | NM_014809.1 | 1968 | ctccgtctaagcttcgtctaac | 343858 | - | see also 22315 line |
| 50378 | KIAA0319 | NM_014809.1 | 1522 | atcggctgagataaacctaatta | 343841 | - | see also 22315 line |
| 50379 | KIAA0319 | NM_014809.1 | 1705 | gtccgtcggactttatgtcttca | 343850 | - | see also 22315 line |
| 50380 | KIAA0329 | NM_014844.1 | 3521 | gtcgctaaggagagtctcatagg | 344555 | - | see also 22316 line |
| 50381 | KIAA0329 | NM_014844.1 | 1762 | gggcagtagcgtggatcagttaa | 344533 | - | see also 22316 line |
| 50382 | KIAA0355 | NM_014686.1 | 2167 | ctctagaatcgtggttcaagtcc | 342175 | - | see also 22317 line |
| 50383 | KIAA0355 | NM_014686.1 | 1543 | ggcgacatctagactaagagaaa | 342162 | - | see also 22317 line |
| 50384 | KIAA0390 | NM_014717.1 | 2858 | ttgtgagcaacggtgtgaatttc | 342796 | - | see also 5911 line |
| 50385 | KIAA0390 | NM_014717.1 | 3300 | gccctcgttacaatcaaacaaag | 342809 | - | see also 5911 line |
| 50386 | KIAA0391 | NM_014672.1 | 382 | ttcgaagctttcgaagctttgg | 342046 | - | see also 22318 line |
| 50387 | KIAA0391 | NM_014672.1 | 721 | cagaggagttggataaaacttaag | 342057 | - | see also 22318 line |
| 50388 | KIAA0399 | NM_015113.2 | 6900 | accgcgtcgtttatatggataa | 348166 | - | see also 22319 line |
| 50389 | KIAA0399 | NM_015113.2 | 4158 | gtgtatcgcactccagatttata | 348109 | - | see also 22319 line |
| 50390 | KIAA0406 | NM_014657.1 | 1055 | gactggcgacaagttgactatcc | 341829 | - | see also 5826 line |
| 50391 | KIAA0406 | NM_014657.1 | 1210 | aggccttagtggactagtaaat | 341835 | - | see also 5826 line |

Figure 46

| | | | | | | | |
|---|---|---|---|---|---|---|---|
| 50392 | KIAA0408 | NM_014702.1 | 1283 | ttcgaaagcggctaaccataga | 342378 | - | see also 22321 line |
| 50393 | KIAA0408 | NM_014702.1 | 1716 | aacttcgaaagatggtatcatct | 342398 | - | see also 22321 line |
| 50394 | KIAA0408 | NM_014702.1 | 2315 | gtcctcgcagttatcgaaatatg | 342425 | - | see also 22321 line |
| 50395 | KIAA0409 | NM_015324.2 | 640 | gttggcggaaccggcaaaagaata | 351576 | - | see also 22322 line |
| 50396 | KIAA0409 | NM_015324.2 | 606 | cccctaaacccctcatacatta | 351574 | - | see also 22322 line |
| 50397 | KIAA0431 | NM_015251.1 | 1894 | aaggtaacttcatctatagctgc | 350298 | - | see also 22323 line |
| 50398 | KIAA0431 | NM_015251.1 | 1546 | ctgatcctcgcctagattcaga | 350288 | - | see also 22323 line |
| 50399 | KIAA0476 | NM_014856.1 | 937 | ggcgaggcactcctcatactta | 344817 | - | see also 22324 line |
| 50400 | KIAA0476 | NM_014856.1 | 4166 | cggctacgcctgcccagtattct | 344843 | - | see also 22324 line |
| 50401 | KIAA0483 | NM_015176.1 | 552 | atcgtgttgagatatgtcaat | 348929 | - | see also 6268 line |
| 50402 | KIAA0483 | NM_015176.1 | 275 | cagctccgcctggtttgtaaaag | 348918 | - | see also 6268 line |
| 50403 | KIAA0483 | NM_015176.1 | 427 | agtcgtcatgcagacattcttg | 348927 | - | see also 6268 line |
| 50404 | KIAA0513 | NM_014732.1 | 649 | cccgtggctcgctaatcgact | 342880 | - | see also 22326 line |
| 50405 | KIAA0513 | NM_014732.1 | 1686 | tccggttcaacgagaacatcacc | 342884 | - | see also 22326 line |
| 50406 | KIAA0555 | NM_014790.1 | 1529 | gacccgtttattggctatgatga | 343590 | - | see also 22327 line |
| 50407 | KIAA0555 | NM_014790.1 | 1004 | aacatgagcagcccaaaacgaga | 343576 | - | see also 22327 line |
| 50408 | KIAA0555 | NM_014790.1 | 1955 | gagagacgatccctccatttaa | 343600 | - | see also 22327 line |
| 50409 | KIAA0562 | NM_014704.1 | 2194 | gacggcggttcgaattattttgg | 342621 | - | see also 5897 line |
| 50410 | KIAA0562 | NM_014704.1 | 2189 | cgcgagacgcggttcgaattat | 342618 | - | see also 5897 line |
| 50411 | KIAA0582 | NM_015147.1 | 1370 | tgcgaaggaccggctgactatag | 348613 | - | see also 22329 line |
| 50412 | KIAA0582 | NM_015147.1 | 1864 | atcgcctgatgactctactctg | 348626 | - | see also 22329 line |
| 50413 | KIAA0586 | NM_014749.1 | 4668 | gtgatatggatcggacacaaatt | 343121 | - | see also 22330 line |
| 50414 | KIAA0586 | NM_014749.1 | 793 | aaccgcagtccgttgataaagg | 343012 | - | see also 22330 line |
| 50415 | KIAA0590 | NM_014714.1 | 3137 | cgggcaggcggtgcacttcctaca | 342675 | - | see also 22331 line |
| 50416 | KIAA0590 | NM_014714.1 | 2327 | gagaccctgcgagactttgtgg | 342670 | - | see also 22331 line |
| 50417 | KIAA0605 | NM_014694.1 | 1121 | cggctgacgggtgctttct | 342363 | - | see also 22332 line |
| 50418 | KIAA0605 | NM_014694.1 | 2322 | gccatgtgtccgctatgatgg | 342373 | - | see also 22332 line |
| 50419 | KIAA0605 | NM_014694.1 | 1272 | gcccgagacatccagattgtaga | 342365 | - | see also 22332 line |
| 50420 | KIAA0625 | NM_015046.4 | 3210 | ctcccgtggacaggttattatta | 347307 | - | see also 6202 line |
| 50421 | KIAA0625 | NM_015046.4 | 686 | ttcggcgttggctatcttgact | 347231 | - | see also 6202 line |
| 50422 | KIAA0625 | NM_015046.4 | 1898 | ctctaggttggcagttgactagt | 347273 | - | see also 6202 line |
| 50423 | KIAA0632 | NM_015545.2 | 2087 | cggcttccgagcctattacaagc | 353865 | - | see also 22334 line |
| 50424 | KIAA0632 | NM_015545.2 | 398 | tgggaccctctgacaaatact | 353848 | - | see also 22334 line |
| 50425 | KIAA0635 | NM_014645.1 | 1382 | agcgcacgtgaaaaaagttcaat | 341632 | - | see also 5819 line |
| 50426 | KIAA0635 | NM_014645.1 | 1473 | ttcagcgtatgctagaaagattt | 341637 | - | see also 5819 line |
| 50427 | KIAA0638 | NM_015157.1 | 3605 | ccctccatgcaccactctatcc | 348646 | - | see also 22336 line |
| 50428 | KIAA0638 | NM_015157.1 | 3609 | ctcatgcaacactctatcctaca | 348647 | - | see also 22336 line |

Figure 46

| | | | | | | | |
|---|---|---|---|---|---|---|---|
| 50429 | KIAA0643 | NM_024793.1 | 756 | caccaaccaagcccaatcgaagg | 390762 | - | see also 22337 line |
| 50430 | KIAA0643 | NM_024793.1 | 497 | ctcaagagtggaagtaacgatga | 390758 | - | see also 22337 line |
| 50431 | KIAA0643 | NM_024793.1 | 716 | gacgaggatgacgatttggaaga | 390760 | - | see also 22337 line |
| 50432 | KIAA0644 | NM_014817.1 | 1974 | gtccgaccatcgaacttcaaca | 344309 | - | see also 22338 line |
| 50433 | KIAA0644 | NM_014817.1 | 1428 | ggccctgcgaggcaaatacctgg | 344306 | - | see also 22338 line |
| 50434 | KIAA0648 | NM_015200.1 | 2254 | cagatacgatcgacttaattcc | 349771 | - | see also 22339 line |
| 50435 | KIAA0648 | NM_015200.1 | 1655 | atgtacgcgaactattggatttg | 349750 | - | see also 22339 line |
| 50436 | KIAA0649 | NM_014811.1 | 1424 | ctccccggttagcgaagatgaac | 344181 | - | see also 22340 line |
| 50437 | KIAA0649 | NM_014811.1 | 1425 | tccccggttagcgaagatgaacg | 344182 | - | see also 22340 line |
| 50438 | KIAA0652 | NM_014741.1 | 1463 | gaccctgacgagtttggatatac | 342913 | - | see also 22341 line |
| 50439 | KIAA0652 | NM_014741.1 | 440 | tccaacgggttcagattggttca | 342893 | - | see also 22341 line |
| 50440 | KIAA0672 | NM_014859.1 | 2692 | atgcggcgacactcagtaactga | 344937 | - | see also 6061 line |
| 50441 | KIAA0672 | NM_014859.1 | 1677 | tccagtacacgtgaaccatatg | 344929 | - | see also 6061 line |
| 50442 | KIAA0683 | NM_016111.1 | 1988 | ccccggtggcgactatctgacc | 356294 | - | see also 22343 line |
| 50443 | KIAA0683 | NM_016111.1 | 253 | gccgtccggaagccattcatgc | 356291 | - | see also 22343 line |
| 50444 | KIAA0683 | NM_016111.1 | 1788 | gaccacgrctgaggacatagagc | 356293 | - | see also 22343 line |
| 50445 | KIAA0685 | NM_014678.2 | 772 | aaccgaacaggtgattacgtttt | 342103 | - | see also 22344 line |
| 50446 | KIAA0685 | NM_014678.2 | 773 | accgaacaggtgattacgtttt | 342104 | - | see also 22344 line |
| 50447 | KIAA0690 | NM_015179.2 | 1509 | ggcctgacgtacaaattccatgc | 348964 | - | see also 22345 line |
| 50448 | KIAA0690 | NM_015179.2 | 103 | ctggtgtctagctaagttgaag | 348951 | - | see also 22345 line |
| 50449 | KIAA0720 | NM_020631.2 | 892 | cagtcgttcagcggcttttca | 377815 | - | see also 22346 line |
| 50450 | KIAA0720 | NM_020631.2 | 1106 | agctggcgggagctcattgatgg | 377816 | - | see also 22346 line |
| 50451 | KIAA0720 | NM_020631.2 | 2043 | tgctcgtggacaagatrgrtgc | 377819 | - | see also 22346 line |
| 50452 | KIAA0738 | NM_014719.1 | 737 | atcccagtgttggttagttgtga | 342828 | - | see also 22347 line |
| 50453 | KIAA0738 | NM_014719.1 | 693 | aaggggacacgagtttctttaaa | 342826 | - | see also 22347 line |
| 50454 | KIAA0746 | NM_015187.1 | 2371 | aaaccagatgcgtcataacaatct | 349521 | - | see also 6272 line |
| 50455 | KIAA0746 | NM_015187.1 | 1055 | ttcgactggatatctctttaac | 349490 | - | see also 6272 line |
| 50456 | KIAA0748 | NM_014796.1 | 1039 | tgccatccatcaggattctgtcc | 343663 | - | see also 5991 line |
| 50457 | KIAA0753 | NM_014804.1 | 240 | aacttggcgatccgatattcttg | 343720 | - | see also 5998 line |
| 50458 | KIAA0753 | NM_014804.1 | 2726 | ctccctagccgaagattctcaac | 343779 | - | see also 5998 line |
| 50459 | KIAA0753 | NM_014804.1 | 1551 | gtgttagccgttggcaaaaacaaa | 343752 | - | see also 5998 line |
| 50460 | KIAA0759 | NM_015305.2 | 1345 | atctttgtcgsgggacctaaattc | 351330 | - | see also 6320 line |
| 50461 | KIAA0759 | NM_015305.2 | 1772 | gcctccacctgacgtcagtatat | 351338 | - | see also 6320 line |
| 50462 | KIAA0763 | NM_014869.1 | 2231 | gtccccggagcagatatcaaagt | 345061 | - | see also 22352 line |
| 50463 | KIAA0763 | NM_014869.1 | 467 | ttctctaggcaagtgaaatcact | 345053 | - | see also 22352 line |
| 50464 | KIAA0776 | NM_015323.2 | 596 | cacgtatccgtggactattcagt | 351520 | - | see also 6359 line |
| 50465 | KIAA0776 | NM_015323.2 | 1573 | ctcgaggtgtacgttcagtatt | 351540 | - | see also 6359 line |

Figure 46

| 50466 | KIAA0776 | NM_015323.2 | 2325 | tgccagtactactcgtaaagagc | 351563 | - | see also 6359 line |
|---|---|---|---|---|---|---|---|
| 50467 | KIAA0831 | NM_014924.2 | 559 | atcttcgacgatcccatatatta | 345770 | - | see also 22353 line |
| 50468 | KIAA0831 | NM_014924.2 | 1098 | ctgcataccctcaggaatctaat | 345785 | - | see also 22353 line |
| 50469 | KIAA0853 | NM_015070.2 | 3578 | gacgcctcacgtgactatagaag | 347803 | - | see also 22354 line |
| 50470 | KIAA0853 | NM_015070.2 | 2865 | atgcgtgcacaggacattatagg | 347794 | - | see also 22354 line |
| 50471 | KIAA0861 | NM_015078.2 | 605 | ttcacggctacatcgacaaaagc | 347885 | - | see also 22355 line |
| 50472 | KIAA0861 | NM_015078.2 | 2574 | gactgaatgtccggacgatattg | 347924 | - | see also 22355 line |
| 50473 | KIAA0874 | NM_015208.2 | 6374 | aacgacgactttgaattgactcc | 349995 | - | see also 6278 line |
| 50474 | KIAA0874 | NM_015208.2 | 4025 | gagcggattaaaccaccatatgc | 349918 | - | see also 6278 line |
| 50475 | KIAA0889 | NM_015377.1 | 535 | gacgggcatccgagtctactata | 351936 | - | see also 22357 line |
| 50476 | KIAA0889 | NM_015377.1 | 536 | acgggcatccgagtctactatag | 351937 | - | see also 22357 line |
| 50477 | KIAA0893 | NM_014969.3 | 2210 | agccggtggtacgttttaaaagg | 346528 | - | see also 6158 line |
| 50478 | KIAA0893 | NM_014969.3 | 2209 | cagccggtggtacgttttaaaag | 346527 | - | see also 6158 line |
| 50479 | KIAA0903 | NM_015252.1 | 2644 | tactcatatagtagagatctaga | 350358 | - | see also 6292 line |
| 50480 | KIAA0903 | NM_015252.1 | 3487 | aaggcagcgataactgaaactca | 350387 | - | see also 6292 line |
| 50481 | KIAA0907 | NM_014949.1 | 1155 | ccctacggagtaccaagcatagt | 346277 | - | see also 6147 line |
| 50482 | KIAA0907 | NM_014949.1 | 518 | gggccagacacgggaattagtgg | 346264 | - | see also 6147 line |
| 50483 | KIAA0922 | NM_015196.2 | 4729 | cccgtttcgcgcctatatgaacc | 349649 | - | see also 6276 line |
| 50484 | KIAA0922 | NM_015196.2 | 2504 | accgcagcggacctagaatttcg | 349600 | - | see also 6276 line |
| 50485 | KIAA0922 | NM_015196.2 | 4728 | acccgtttcgcgcctatatgaac | 349648 | - | see also 6276 line |
| 50486 | KIAA0963 | NM_014963.1 | 3251 | tcccggtatcgaggagatctacg | 346398 | - | see also 22362 line |
| 50487 | KIAA0963 | NM_014963.1 | 2019 | ggcgtgttcctgtcgctaattca | 346395 | - | see also 22362 line |
| 50488 | KIAA0970 | NM_014923.2 | 2407 | cagactacgtgcagctaacaaaa | 345730 | - | see also 22363 line |
| 50489 | KIAA0970 | NM_014923.2 | 1344 | tagccaatcggaccaaaaattca | 345696 | - | see also 22363 line |
| 50490 | KIAA0971 | NM_014929.1 | 827 | gtgacccaggaacgtatcaatga | 345915 | - | see also 22364 line |
| 50491 | KIAA0971 | NM_014929.1 | 831 | cccaggaacgtatcaatgagtgt | 345917 | - | see also 22364 line |
| 50492 | KIAA0977 | NM_014900.1 | 2507 | tacccaaaattggcatgactact | 345430 | - | see also 22365 line |
| 50493 | KIAA0977 | NM_014900.1 | 986 | cccctgcaacccctctagtaaat | 345388 | - | see also 22365 line |
| 50494 | KIAA0982 | NM_014023.1 | 168 | ttcgactgctcgccaaacaaaac | 339281 | - | see also 5462 line |
| 50495 | KIAA0982 | NM_014023.1 | 1429 | aaccgacaagtgagactgtttga | 339312 | - | see also 5462 line |
| 50496 | KIAA1007 | NM_016284.2 | 2046 | tgcttcacgtcgtgaatacctca | 356712 | - | see also 6780 line |
| 50497 | KIAA1007 | NM_016284.2 | 1875 | tccctcaattcgccaacttatca | 356705 | - | see also 6780 line |
| 50498 | KIAA1007 | NM_016284.2 | 5888 | gccgcgacagtaccaaagctttc | 356836 | - | see also 6780 line |
| 50499 | KIAA1018 | NM_014967.1 | 389 | atggtgcctagatatgacttaaa | 346426 | - | see also 6156 line |
| 50500 | KIAA1018 | NM_014967.1 | 1345 | aacaaccggtcatccttactacc | 346454 | - | see also 6156 line |
| 50501 | KIAA1023 | NM_152558.1 | 1916 | ctccgatgattccgacgatattg | 418107 | - | see also 22369 line |
| 50502 | KIAA1023 | NM_152558.1 | 331 | atgacgagattattgagttaaag | 418094 | - | see also 22369 line |

Figure 46

| | | | | | | | |
|---|---|---|---|---|---|---|---|
| 50503 | KIAA1033 | NM_015275.1 | 133 | gacgacggctcgcgagaaattca | 350721 | - | see also 22370 line |
| 50504 | KIAA1033 | NM_015275.1 | 262 | ttcaatcttgatcctatagcatt | 350729 | - | see also 22370 line |
| 50505 | KIAA1043 | NM_015281.1 | 3698 | gacgctccgacatagacacaaact | 350888 | - | see also 6311 line |
| 50506 | KIAA1043 | NM_015281.1 | 2438 | accggagcaagctaatcgacg | 350878 | - | see also 6311 line |
| 50507 | KIAA1049 | NM_014972.1 | 275 | accgcttgagctgataaacatt | 346556 | - | see also 22372 line |
| 50508 | KIAA1049 | NM_014972.1 | 973 | agcgatgcctgccgctttcaaga | 346563 | - | see also 22372 line |
| 50509 | KIAA1049 | NM_014972.1 | 900 | gtctatggaccgaacaacatcg | 346562 | - | see also 22372 line |
| 50510 | KIAA1052 | NM_014956.1 | 3347 | cagctccgagaaattcttgatga | 346337 | - | see also 6151 line |
| 50511 | KIAA1068 | NM_015332.2 | 620 | ctggtcacaggactaactgacc | 351626 | - | see also 22374 line |
| 50512 | KIAA1068 | NM_015332.2 | 530 | ccctaggagaaccaccaattcttc | 351625 | - | see also 22374 line |
| 50513 | KIAA1074 | NM_014915.1 | 3871 | ctggagttacgtcacgttatcg | 345610 | - | see also 22375 line |
| 50514 | KIAA1074 | NM_014915.1 | 3875 | aggttacgtcacgttatcgtatt | 345612 | - | see also 22375 line |
| 50515 | KIAA1115 | NM_014931.1 | 2200 | aacgccaacctacttgagatatg | 345968 | - | see also 22376 line |
| 50516 | KIAA1115 | NM_014931.1 | 1647 | ccctgagacgcccatccaaaacc | 345959 | - | see also 22376 line |
| 50517 | KIAA1117 | NM_015018.2 | 1040 | ggccactcgaccggatatgatca | 346803 | - | see also 22377 line |
| 50518 | KIAA1117 | NM_015018.2 | 6230 | atggctgcgacgaaatcttgaag | 346945 | - | see also 22377 line |
| 50519 | KIAA1143 | NM_020696.1 | 143 | gacccaccgtagagactaagaga | 378155 | - | see also 22378 line |
| 50520 | KIAA1143 | NM_020696.1 | 148 | accgtagaagactaagagaattca | 378158 | - | see also 22378 line |
| 50521 | KIAA1160 | NM_020701.1 | 754 | cagagccgagttagttggaaagt | 378179 | - | see also 22379 line |
| 50522 | KIAA1160 | NM_020701.1 | 231 | aggccatgacggcttagcaaga | 378165 | - | see also 22379 line |
| 50523 | KIAA1181 | NM_020462.1 | 327 | gagttggttggcttgacattca | 377675 | - | see also 22380 line |
| 50524 | KIAA1181 | NM_020462.1 | 443 | cagcatcaacaaggtatggaagc | 377677 | - | see also 22380 line |
| 50525 | KIAA1185 | NM_020710.1 | 1204 | gcccccacaggacctcaagattg | 378262 | - | see also 22381 line |
| 50526 | KIAA1185 | NM_020710.1 | 1463 | ttgtttttggaagtaacaagtgc | 378264 | - | see also 22381 line |
| 50527 | KIAA1191 | NM_020444.2 | 390 | gtccctgcccatcgggatatacc | 377474 | - | see also 22382 line |
| 50528 | KIAA1191 | NM_020444.2 | 624 | ggctaaagctaccatgtcatca | 377476 | - | see also 22382 line |
| 50529 | KIAA1191 | NM_020444.2 | 1011 | accgagatcccttcagaagtacg | 377477 | - | see also 22382 line |
| 50530 | KIAA1194 | NM_015455.2 | 740 | aacaacctgttacgagttctacc | 353193 | - | see also 22383 line |
| 50531 | KIAA1194 | NM_015455.2 | 454 | ccctcggaggatgtatacaatta | 353185 | - | see also 22383 line |
| 50532 | KIAA1199 | NM_018689.1 | 519 | gaccacgacgagccgattgtttt | 372550 | - | see also 7562 line |
| 50533 | KIAA1199 | NM_018689.1 | 1235 | gacggagttggttcgatcatgata | 372578 | - | see also 7562 line |
| 50534 | KIAA1199 | NM_018689.1 | 520 | accacgacgagccgattgttttg | 372551 | - | see also 7562 line |
| 50535 | KIAA1219 | NM_020336.1 | 1202 | gagcggaatgcgcgcaagaattga | 376558 | - | see also 22385 line |
| 50536 | KIAA1219 | NM_020336.1 | 2662 | accggaagcgagccatcagttct | 376606 | - | see also 22385 line |
| 50537 | KIAA1221 | NM_032186.2 | 3687 | gaggatagtccgttgatgtatca | 398631 | - | see also 22386 line |
| 50538 | KIAA1221 | NM_032186.2 | 956 | tcgttataccgaggattgcttta | 398601 | - | see also 22386 line |
| 50539 | KIAA1244 | NM_020340.2 | 761 | ctgtggctcggactatctattac | 376691 | - | see also 22387 line |

Figure 46

| | | | | | | | |
|---|---|---|---|---|---|---|---|
| 50540 | KIAA1244 | NM_020340.2 | 759 | acctgtggctcggactatctatt | 376690 | - | see also 22387 line |
| 50541 | KIAA1279 | NM_015634.1 | 744 | tcctatagagtgggctatcaatg | 354784 | - | see also 22388 line |
| 50542 | KIAA1279 | NM_015634.1 | 66 | gtcgcgggtggaactgcataaaa | 354771 | - | see also 22388 line |
| 50543 | KIAA1280 | NM_015691.2 | 2068 | tccgcatgtctgtcggattattc | 355115 | - | see also 22389 line |
| 50544 | KIAA1280 | NM_015691.2 | 2962 | cagccctcgcctcttaaggttga | 355124 | - | see also 22389 line |
| 50545 | KIAA1287 | NM_020748.1 | 2298 | ggcgaatgctcctgactaataat | 378425 | - | see also 7930 line |
| 50546 | KIAA1287 | NM_020748.1 | 1884 | tacttactcctgcgtcgaaatct | 378410 | - | see also 7930 line |
| 50547 | KIAA1295 | NM_017963.2 | 368 | aggccacgccatagaatcaattt | 364898 | - | see also 22391 line |
| 50548 | KIAA1295 | NM_017963.2 | 369 | ggccacgccatagaatcaatttc | 364899 | - | see also 22391 line |
| 50549 | KIAA1318 | NM_020769.1 | 1132 | accccgctcatgtcagatctaga | 378629 | - | see also 22392 line |
| 50550 | KIAA1318 | NM_020769.1 | 3227 | gtgttgcgaactctagatctatg | 378651 | - | see also 22392 line |
| 50551 | KIAA1322 | NM_020773.1 | 1945 | ctgtcgcgatggggaagagttcc | 378775 | - | see also 7950 line |
| 50552 | KIAA1322 | NM_020773.1 | 1362 | tagccattggcaacgagttaaat | 378756 | - | see also 7950 line |
| 50553 | KIAA1324 | NM_020775.2 | 697 | caccgttaacttcgaatactact | 378869 | - | see also 22394 line |
| 50554 | KIAA1324 | NM_020775.2 | 2512 | accggacgtgatcttctttata | 378912 | - | see also 22394 line |
| 50555 | KIAA1336 | NM_020779.2 | 596 | atggtctgcggacagtaaagtct | 378941 | - | see also 7964 line |
| 50556 | KIAA1336 | NM_020779.2 | 2563 | aacgcttagctgaatgttactat | 378997 | - | see also 7964 line |
| 50557 | KIAA1383 | NM_019090.1 | 1544 | tacactaagactacgtttaaagc | 375179 | - | see also 22396 line |
| 50558 | KIAA1383 | NM_019090.1 | 518 | gggacgtttcccctgcataatc | 375157 | - | see also 22396 line |
| 50559 | KIAA1387 | NM_020463.1 | 464 | gtccgacggatcactactcttgg | 377679 | - | see also 7879 line |
| 50560 | KIAA1387 | NM_020463.1 | 1315 | aacggcgtgaattggttaatttt | 377698 | - | see also 7879 line |
| 50561 | KIAA1387 | NM_020463.1 | 2021 | gtctcttactgcccatatagttg | 377718 | - | see also 7879 line |
| 50562 | KIAA1404 | NM_021035.1 | 1748 | tacgactttacccccttaataga | 380405 | - | see also 22397 line |
| 50563 | KIAA1404 | NM_021035.1 | 2217 | tccgaagggcctacatgagtatc | 380417 | - | see also 22397 line |
| 50564 | KIAA1407 | NM_020817.1 | 200 | atcgctggaaacggttcacaagg | 379147 | - | see also 22398 line |
| 50565 | KIAA1407 | NM_020817.1 | 2886 | tggaaggtaccacgagctatatc | 379185 | - | see also 22398 line |
| 50566 | KIAA1446 | NM_020836.2 | 246 | caggattcagagcaactacatgg | 379382 | - | see also 22399 line |
| 50567 | KIAA1449 | NM_020839.2 | 151 | agccggtcgagactctatcataa | 379392 | - | see also 7986 line |
| 50568 | KIAA1449 | NM_020839.2 | 152 | gccggtcgagactctatcataag | 379393 | - | see also 7986 line |
| 50569 | KIAA1465 | NM_020851.1 | 236 | ttcgcggactgcgcttacaaaga | 379610 | - | see also 22401 line |
| 50570 | KIAA1465 | NM_020851.1 | 964 | ccccggtggcaccgtagtcttag | 379620 | - | see also 22401 line |
| 50571 | KIAA1465 | NM_020851.1 | 238 | cgcggactgcgcttacaaagagt | 379611 | - | see also 22401 line |
| 50572 | KIAA1468 | NM_020854.2 | 811 | atgtcagcagatagtcgtttagg | 379648 | - | see also 7995 line |
| 50573 | KIAA1468 | NM_020854.2 | 1903 | tggcgcctttgccggacatttgg | 379680 | - | see also 7995 line |
| 50574 | KIAA1508 | NM_020880.2 | 1882 | atcttttacccgcaaaaatcacc | 379872 | - | see also 22403 line |
| 50575 | KIAA1508 | NM_020880.2 | 837 | ctggacgaaagcctctcaaatac | 379863 | - | see also 22403 line |
| 50576 | KIAA1518 | NM_015493.3 | 1969 | cccggcaggcgtgcgatctatca | 353567 | - | see also 22404 line |

Figure 46

| | | | | | | | |
|---|---|---|---|---|---|---|---|
| 50577 | KIAA1518 | NM_015493.3 | 2720 | ttccggcttggcaacatcaatgc | 353572 | - | see also 22404 line |
| 50578 | KIAA1524 | NM_020890.1 | 1202 | ctgttcctcggctgatcgtttg | 380022 | - | see also 8016 line |
| 50579 | KIAA1524 | NM_020890.1 | 500 | tacgttcctgatagatcacattc | 379988 | - | see also 8016 line |
| 50580 | KIAA1533 | NM_020895.2 | 399 | cacttataagcagcgtaatgagg | 380073 | - | see also 22406 line |
| 50581 | KIAA1533 | NM_020895.2 | 968 | gccatgtcacgccaacctttcc | 380076 | - | see also 22406 line |
| 50582 | KIAA1536 | NM_020898.1 | 1343 | gagtgcagagatacttcgattgg | 380094 | - | see also 22407 line |
| 50583 | KIAA1536 | NM_020898.1 | 95 | tggagtcaactttctcaattgtag | 380085 | - | see also 22407 line |
| 50584 | KIAA1587 | NM_020932.1 | 2839 | tacttgagtcagcgctatataga | 380193 | - | see also 8037 line |
| 50585 | KIAA1587 | NM_020932.1 | 1475 | aceggaaccctccaagrgttcc | 380158 | - | see also 8037 line |
| 50586 | KIAA1598 | NM_018330.2 | 490 | cacaagttccaaggttacgtttc | 370436 | - | see also 22409 line |
| 50587 | KIAA1598 | NM_018330.2 | 564 | aagccgaacagttgtagttta | 370437 | - | see also 22409 line |
| 50588 | KIAA1608 | NM_024820.1 | 1330 | gtcgattagatcttcaattcc | 391100 | - | see also 8765 line |
| 50589 | KIAA1608 | NM_024820.1 | 1075 | tcccaacgacgtgatctcttcc | 391094 | - | see also 8765 line |
| 50590 | KIAA1609 | NM_020947.2 | 222 | gcccgaatgtctcatccaaatcc | 380213 | - | see also 22411 line |
| 50591 | KIAA1609 | NM_020947.2 | 721 | ccccatgtggccatattcctga | 380217 | - | see also 22411 line |
| 50592 | KIAA1609 | NM_020947.2 | 221 | agcccgaatgtctcatccaaatc | 380212 | - | see also 22411 line |
| 50593 | KIAA1622 | NM_020958.2 | 367 | aacaagtgattgcaaatctcc | 380219 | - | - |
| 50594 | KIAA1622 | NM_020958.2 | 379 | tgcaaatctccattttgatgc | 380220 | - | see also 50593 line |
| 50595 | KIAA1627 | NM_020961.2 | 1192 | cagttggaccaagcttacaaat | 380276 | - | see also 22412 line |
| 50596 | KIAA1627 | NM_020961.2 | 621 | tacttacaagccgatatagaagc | 380263 | - | see also 22412 line |
| 50597 | KIAA1701 | NM_030639.1 | 1511 | ttgttagcttacttaagtcaact | 394795 | - | see also 8914 line |
| 50598 | KIAA1712 | NM_030633.1 | 351 | gtgctccgcttgctaaattatcc | 394735 | - | see also 22414 line |
| 50599 | KIAA1712 | NM_030633.1 | 822 | gtggtgattcgtcacttgtataa | 394748 | - | see also 22414 line |
| 50600 | KIAA1715 | NM_030650.1 | 583 | gacggctaaattaattcttgaaa | 394836 | - | see also 8922 line |
| 50601 | KIAA1715 | NM_030650.1 | 948 | aacagaggtgctttggatagaatt | 394843 | - | see also 8922 line |
| 50602 | KIAA1720 | NM_030645.1 | 1020 | gggcgtcatggctgacaagaacc | 394823 | - | see also 22416 line |
| 50603 | KIAA1720 | NM_030645.1 | 830 | aggagtcggcgaggaaaactgaat | 394820 | - | see also 22416 line |
| 50604 | KIAA1737 | NM_033426.2 | 1030 | cactgtccgctaattatagctca | 403889 | - | see also 9424 line |
| 50605 | KIAA1737 | NM_033426.2 | 1137 | accctagtagtcctacataaatc | 403893 | - | see also 9424 line |
| 50606 | KIAA1737 | NM_033426.2 | 1136 | tacccctagtagtcctacataaat | 403892 | - | see also 9424 line |
| 50607 | KIAA1754 | NM_033397.1 | 1706 | tagcgagtattccctcacatgtcc | 403778 | - | see also 22418 line |
| 50608 | KIAA1754 | NM_033397.1 | 890 | gaccaagctcgggggaagaacatgc | 403776 | - | see also 22418 line |
| 50609 | KIAA1754L | NM_178495.3 | 805 | agcatgaaccggccttgattcc | 430836 | - | see also 22419 line |
| 50610 | KIAA1754L | NM_178495.3 | 1926 | atccctaagacatttaggaatgc | 430847 | - | see also 22419 line |

Figure 46

| 50611 | KIAA1754L | NM_178495.3 | 1401 | gcccttgtggcccacaagtatga | 430842 | - | see also 22419 line |
|---|---|---|---|---|---|---|---|
| 50612 | KIAA1796 | NM_032439.1 | 364 | gaccgggacgggaacaaatcaca | 400284 | - | see also 9189 line |
| 50613 | KIAA1797 | NM_017794.2 | 5069 | atcggatcgcccaggttactaag | 362575 | - | see also 22420 line |
| 50614 | KIAA1797 | NM_017794.2 | 5259 | tgccaatacgggcgttttgaaga | 362581 | - | see also 22420 line |
| 50615 | KIAA1797 | NM_017794.2 | 5258 | atgccaatacgggcgttttgaag | 362580 | - | see also 22420 line |
| 50616 | KIAA1827 | NM_032423.1 | 2347 | cagcctcataacccatcagttaa | 400212 | - | see also 22421 line |
| 50617 | KIAA1827 | NM_032423.1 | 2841 | atcaagcatcataccttacaaga | 400218 | - | see also 22421 line |
| 50618 | KIAA1892 | NM_015397.1 | 637 | cacgctatttgtcgtagatgtcc | 352271 | - | see also 6404 line |
| 50619 | KIAA1892 | NM_015397.1 | 743 | atcgagctgaatccttctagaac | 352273 | - | see also 6404 line |
| 50620 | KIAA1892L | NM_178470.2 | 1150 | ctgccctacttccgggataatgt | 430831 | - | see also 22423 line |
| 50621 | KIAA1892L | NM_178470.2 | 1152 | gccctacttccgggataatgtgt | 430832 | - | see also 22423 line |
| 50622 | KIAA1893 | NM_052899.1 | 2538 | cgcggactcgcgacaacttcacc | 404786 | - | see also 22424 line |
| 50623 | KIAA1893 | NM_052899.1 | 657 | ccgattccacttccataggaaag | 404772 | - | see also 22424 line |
| 50624 | KIAA1913 | NM_052913.2 | 3437 | gtcgttgtcgagtcagtacaagt | 405309 | - | see also 22425 line |
| 50625 | KIAA1913 | NM_052913.2 | 3547 | tgctctcgtcacactcaaagtcc | 405311 | - | see also 22425 line |
| 50626 | KIAA1919 | NM_153369.1 | 889 | tgctgttatcggtacttacatgt | 423136 | - | see also 22426 line |
| 50627 | KIAA1919 | NM_153369.1 | 447 | tgggtcgtgccttgggatatttg | 423115 | - | see also 22426 line |
| 50628 | KIAA1924 | NM_153239.2 | 643 | gacgggacgatacctgtatgtgc | 422467 | - | see also 22427 line |
| 50629 | KIAA1924 | NM_153239.2 | 729 | aagtcatccgtgtgtctttctcc | 422468 | - | see also 22427 line |
| 50630 | KIAA2010 | NM_017936.2 | 1727 | tccggagagtgctagttcttatg | 364347 | - | see also 22428 line |
| 50631 | KIAA2010 | NM_017936.2 | 292 | agcgacggttctctacttttaga | 364301 | - | see also 22428 line |
| 50632 | KIAA2024 | NM_172070.2 | 2186 | tgcatcggtaattatcatcattc | 424255 | - | see also 22429 line |
| 50633 | KIAA2024 | NM_172070.2 | 1213 | tgcggctttatagcattgactct | 424234 | - | see also 22429 line |
| 50634 | KIBRA | NM_015238.1 | 2885 | gccggctgaatcggagtgatagt | 350157 | - | see also 22430 line |
| 50635 | KIBRA | NM_015238.1 | 2036 | tgggtgcgacccgaattcagatt | 350149 | - | see also 22430 line |
| 50636 | KIF24 | NM_018278.1 | 442 | tgcaatactggggggattgtgata | 369650 | - | see also 22431 line |
| 50637 | KIF24 | NM_018278.1 | 149 | agcagcctgcgcatcaaatctca | 369641 | - | see also 22431 line |
| 50638 | KIRREL | NM_018240.3 | 1810 | atctactcgtcgtttaaggatga | 368892 | - | see also 22432 line |
| 50639 | KIRREL | NM_018240.3 | 1815 | ctcgtcgtttaaggatgatgtgg | 368893 | - | see also 22432 line |
| 50640 | KIRREL2 | NM_032123.4 | 1992 | cccaaccaacggttactacaagg | 397896 | - | see also 22433 line |
| 50641 | KIRREL2 | NM_032123.4 | 1991 | acccaaccaacggttactacaag | 397895 | - | see also 22433 line |
| 50642 | KIRREL3 | NM_032531.1 | 205 | tgcgccatccccgaatacgatgg | 400554 | - | see also 9238 line |
| 50643 | KIRREL3 | NM_032531.1 | 717 | gacgtcggtcaccattgacatcc | 400558 | - | see also 9238 line |
| 50644 | KLF8 | NM_007250.2 | 442 | gtcgatatggataaactcataaa | 337318 | - | see also 22435 line |

Figure 46

| | | | | | | | |
|---|---|---|---|---|---|---|---|
| 50645 | KLF8 | NM_007250.2 | 653 | aagaacttggctagtgattc | 337327 | - | see also 22435 line |
| 50646 | KLHDC1 | NM_172193.1 | 768 | atcggtcatgcatactttaaca | 424295 | - | see also 10039 line |
| 50647 | KLHDC1 | NM_172193.1 | 765 | aacatcggtcatgcatactta | 424294 | - | see also 10039 line |
| 50648 | KLHDC2 | NM_014315.1 | 830 | ttgggaactttgaattcgatga | 340164 | - | see also 5618 line |
| 50649 | KLHDC2 | NM_014315.1 | 649 | aagaggcaataccaataagttct | 340159 | - | see also 5618 line |
| 50650 | KLHDC2 | NM_014315.1 | 477 | aagtcagagattatatgacttt | 340151 | - | see also 5618 line |
| 50651 | KLHDC3 | NM_057161.2 | 1202 | gccgtgattcagtataacctaga | 406116 | - | see also 22438 line |
| 50652 | KLHDC3 | NM_057161.2 | 284 | gagacactgcgtcagatagatgt | 406100 | - | see also 22438 line |
| 50653 | KLIP1 | NM_024629.2 | 1195 | accaaacgtaaaggaaacgtatg | 388278 | - | see also 22439 line |
| 50654 | KLIP1 | NM_024629.2 | 544 | tacacaacgtcatgaggttattc | 388255 | - | see also 22439 line |
| 50655 | KR-ZNF1 | NM_198457.1 | 1464 | cacacctgttaagaacatactaga | 437871 | - | - |
| 50656 | KR-ZNF1 | NM_198457.1 | 1294 | ttctcagctggcaagaacataaa | 437870 | - | see also 50655 line |
| 50657 | KRT25A | NM_181534.2 | 1325 | aggtagaccaacgcagcaaaata | 432413 | - | see also 22440 line |
| 50658 | KRT25A | NM_181534.2 | 1230 | tgggggttacaagtctaaagatt | 432409 | - | see also 22440 line |
| 50659 | KRT25B | NM_181539.2 | 424 | ttccgggaacacagatcatgact | 432571 | - | see also 22441 line |
| 50660 | KRT25B | NM_181539.2 | 1424 | gagcaacgagtaccttctaaagc | 432587 | - | see also 22441 line |
| 50661 | KRT25C | NM_181537.2 | 1225 | aagatgctcctgttctaaatca | 432566 | - | see also 22442 line |
| 50662 | KRT25C | NM_181537.2 | 1296 | accattgtcaaaacagttgttga | 432569 | - | see also 22442 line |
| 50663 | KRT25D | NM_181535.2 | 797 | tagacctgcggtttgttgaac | 432423 | - | see also 22443 line |
| 50664 | KRT25D | NM_181535.2 | 453 | cagtatatcaacctaacaattgagg | 432418 | - | see also 22443 line |
| 50665 | KRT3 | NM_057088.1 | 1220 | atgggatgacctaagaaataacc | 406095 | - | see also 22444 line |
| 50666 | KRT3 | NM_057088.1 | 932 | tggacagtgcctatatgaacaag | 406094 | - | see also 22444 line |
| 50667 | KRT6L | NM_175834.2 | 42 | ctccgtctccggcaaacatact | 429272 | - | see also 22445 line |
| 50668 | KRT6L | NM_175834.2 | 207 | cagccgaagcctcctaaacttg | 429275 | - | see also 22445 line |
| 50669 | KRTAP12-3 | NM_198697.1 | 223 | agctgcaggccatcatcatatgt | 439880 | - | see also 22446 line |
| 50670 | KRTAP13-2 | NM_181621.2 | 269 | gtccttgcaagacgacttactct | 432613 | - | - |
| 50671 | KRTAP13-3 | NM_181622.1 | 296 | ctcggggttttgatccaatagc | 432617 | - | see also 22447 line |
| 50672 | KRTAP13-3 | NM_181622.1 | 290 | ttgggtctcggggttttggatcc | 432616 | - | see also 22447 line |
| 50673 | KRTAP13-4 | NM_181600.1 | 452 | gttggatctcgcttctatcaattc | 432591 | - | see also 22448 line |
| 50674 | KRTAP13-4 | NM_181600.1 | 448 | atctgtggatctcgcttctatca | 432589 | - | see also 22448 line |
| 50675 | KRTAP15-1 | NM_181623.1 | 257 | cccgccagactaactacataaga | 432626 | - | see also 22449 line |

Figure 46

| | | | | | | | | | |
|---|---|---|---|---|---|---|---|---|---|
| 50676 | KRTAP15-1 | NM_181623.1 | 260 | gccagactaactacataagatcc | 432628 | - | see also 22449 line | | |
| 50677 | KRTAP15-1 | NM_181623.1 | 259 | cgccagactaactacataagatc | 432627 | - | see also 22449 line | | |
| 50678 | KRTAP17-1 | NM_031964.1 | 339 | cacacctatatgcgacacaaaat | 397549 | - | - | | - |
| 50679 | KRTAP17-1 | NM_031964.1 | 341 | cacctatatgcgacacaaatga | 397550 | - | see also 50678 line | | |
| 50680 | KRTAP17-1 | NM_031964.1 | 328 | tgctgccagcccacacctatatg | 397548 | - | see also 50678 line | | |
| 50681 | KRTAP18-8 | NM_198695.1 | 38 | tgcaccaggacgtatgtgattgc | 439878 | - | see also 22450 line | | |
| 50682 | KRTAP19-2 | NM_181608.1 | 127 | tacagaagatatagattcactgg | 432595 | - | - | | - |
| 50683 | KRTAP19-4 | NM_181610.1 | 196 | taccctgaggacacgatatcaga | 432598 | - | see also 22452 line | | |
| 50684 | KRTAP19-4 | NM_181610.1 | 198 | ccctgaggacacgatatcagagg | 432599 | - | see also 22452 line | | |
| 50685 | KRTAP19-4 | NM_181610.1 | 220 | gtcataagaagatcattcaattt | 432600 | - | see also 22452 line | | |
| 50686 | KRTAP19-6 | NM_181612.1 | 135 | cccatcatgccgtgaaggatatg | 432602 | - | see also 22453 line | | |
| 50687 | KRTAP19-7 | NM_181614.1 | 169 | tactggtcttctggattctattg | 432604 | - | - | | - |
| 50688 | KRTAP22-1 | NM_181620.1 | 103 | tgcccatggtgttatgaaagatc | 432611 | - | - | | - |
| 50689 | KRTAP22-1 | NM_181620.1 | 104 | gcccatggtgttatgaaagatct | 432612 | - | see also 50688 line | | |
| 50690 | KRTAP22-1 | NM_181620.1 | 100 | tactgcccatggtgttatgaaag | 432610 | - | see also 50688 line | | |
| 50691 | KRTAP3-1 | NM_031958.1 | 203 | tgctgcaagcctgatacctatgt | 397547 | - | see also 22456 line | | |
| 50692 | KRTAP3-1 | NM_031958.1 | 91 | caccttctgctcatttgataaaa | 397544 | - | see also 22456 line | | |
| 50693 | KRTAP6-1 | NM_181602.1 | 192 | ctctggctctggctactattatt | 432593 | - | - | | - |
| 50694 | KRTHA4 | NM_021013.3 | 29 | ccccacccacaattaatggtata | 380357 | - | - | structural molecule | - |
| 50695 | KRTHA4 | NM_021013.3 | 28 | ccccacccacaattaatggtat | 380356 | - | see also 50694 line | | |
| 50696 | KRTHA4 | NM_021013.3 | 33 | acccacaattaatggtataaaag | 380360 | - | see also 50694 line | | |
| 50697 | KRTHA8 | NM_006771.3 | 698 | gtggggcctatggtgaaaacacc | 336319 | - | - | structural molecule | - |
| 50698 | KRTHA8 | NM_006771.3 | 670 | tgccacattcctggcaacattgg | 336318 | - | see also 50697 line | | |
| 50699 | KSP37 | NM_031950.2 | 709 | ggccctgtgcgcctttctcatca | 397469 | - | see also 22458 line | | |

Figure 46

| | | | | | | | |
|---|---|---|---|---|---|---|---|
| 50700 | KSP37 | NM_031950.2 | 436 | tggaccccaggcccatatgcagc | 397467 | - | see also 22458 line |
| 50701 | LACE1 | NM_145315.2 | 1382 | atctcaaggtgcgtataatttgc | 414406 | - | see also 9789 line |
| 50702 | LACE1 | NM_145315.2 | 1379 | atgatctcaaggtgcgtataatt | 414405 | - | see also 9789 line |
| 50703 | LACRT | NM_033277.1 | 144 | gggacctctaagcctaatgaaga | 403650 | - | see also 22460 line |
| 50704 | LACRT | NM_033277.1 | 362 | aggcgtgccaggtggaaaacaat | 403653 | - | see also 22460 line |
| 50705 | LAIR1 | NM_002287.2 | 613 | ctccatcgccagaatcagataaa | 330747 | - | see also 22461 line |
| 50706 | LAIR1 | NM_002287.2 | 614 | tccatcgccagaatcagataaag | 330748 | - | see also 22461 line |
| 50707 | LALP1 | NM_020354.1 | 311 | accacgagataggcaatacgaaa | 376825 | - | see also 7844 line |
| 50708 | LALP1 | NM_020354.1 | 1493 | ggcttggtcggtactaactcaga | 376854 | - | see also 7844 line |
| 50709 | LANCL2 | NM_018697.2 | 565 | ctccgatccctggattacgtaaa | 372668 | - | see also 7568 line |
| 50710 | LANCL2 | NM_018697.2 | 1354 | ctctaccgagcttgcaagtttgc | 372695 | - | see also 7568 line |
| 50711 | LANCL2 | NM_018697.2 | 566 | tccgatccctggattacgtaaaa | 372669 | - | see also 7568 line |
| 50712 | LAP1B | NM_015602.2 | 796 | tacgtcgacccctctaagatac | 354423 | - | see also 22464 line |
| 50713 | LAP1B | NM_015602.2 | 337 | gcgatgcgcctgcgtacagaact | 354416 | - | see also 22464 line |
| 50714 | LARP | NM_015315.2 | 914 | ctcgtacgcccaagtacatgaac | 351437 | - | see also 22465 line |
| 50715 | LARP | NM_015315.2 | 640 | gccaatcgcggagagatcaaagg | 351434 | - | see also 22465 line |
| 50716 | LAS1 | NM_018272.1 | 628 | tggatttatgccagttcacaact | 369614 | - | see also 22466 line |
| 50717 | LAS1 | NM_018272.1 | 343 | agcttgtcaaagatgatgttaag | 369610 | - | see also 22466 line |
| 50718 | LAX | NM_017773.1 | 539 | ttcgcatgattatgtcaatgtcc | 362177 | - | see also 22467 line |
| 50719 | LAX | NM_017773.1 | 297 | aggagtatgcgcattttcagtac | 362174 | - | see also 22467 line |
| 50720 | LBH | NM_030915.1 | 247 | atctgagatcggccaagatgact | 395380 | - | see also 8962 line |
| 50721 | LBH | NM_030915.1 | 345 | ttcccagacccgtcagattttga | 395382 | - | see also 8962 line |
| 50722 | LCMT2 | NM_014793.3 | 474 | tggcttcgactcgctctattttc | 343620 | - | see also 22468 line |
| 50723 | LCMT2 | NM_014793.3 | 483 | ctcgctctattttcgcttaaaaa | 343622 | - | see also 22468 line |
| 50724 | LCN6 | NM_198946.1 | 377 | gccaccaacttcagagactatgc | 440144 | - | see also 22469 line |
| 50725 | LCN6 | NM_198946.1 | 323 | tccggatgggtgtttgagaatcc | 440143 | - | see also 22469 line |
| 50726 | LENG8 | NM_052925.1 | 2124 | ggcaatgtgggcgagtttactgc | 405329 | - | see also 9453 line |
| 50727 | LENG8 | NM_052925.1 | 2245 | cccacgccttggcattaaggaca | 405333 | - | see also 9453 line |
| 50728 | LENG8 | NM_052925.1 | 1134 | gacggctcggcctataccattga | 405322 | - | see also 9453 line |
| 50729 | LGI2 | NM_018176.2 | 687 | gtcggtttcagtggatacgttca | 368008 | - | see also 7299 line |
| 50730 | LGI2 | NM_018176.2 | 1125 | tgcggagtttgttgatatcgatg | 368023 | - | see also 7299 line |
| 50731 | LGI2 | NM_018176.2 | 1000 | atcgacgacgagacgttctttgt | 368017 | - | see also 7299 line |
| 50732 | LGI4 | NM_139284.1 | 1728 | ccccgaggccgaggatgtctatg | 409616 | - | see also 22472 line |
| 50733 | LGI4 | NM_139284.1 | 1729 | cccgaggccgaggatgtctatgc | 409617 | - | see also 22472 line |
| 50734 | LHFPL1 | NM_178175.2 | 312 | taccagttacttcctaccttact | 430614 | - | see also 22473 line |
| 50735 | LHFPL1 | NM_178175.2 | 881 | ttgaccattgcctcaaaccttga | 430623 | - | see also 22473 line |
| 50736 | LHFPL2 | NM_005779.1 | 511 | ttccttatcctcggtttgatact | 334248 | - | see also 22474 line |

EP 1 752 536 A1

Figure 46

| | | | | | | | |
|---|---|---|---|---|---|---|---|
| 50737 | LHFPL2 | NM_005779.1 | 699 | aacctctagtgacaaagtacagg | 334251 | - | see also 22474 line |
| 50738 | LHFPL3 | NM_199000.1 | 523 | cacggccactgtgtacaagatat | 440581 | - | see also 22475 line |
| 50739 | LHFPL3 | NM_199000.1 | 191 | aagctgtaccacaccaactatgt | 440579 | - | see also 22475 line |
| 50740 | LIMS2 | NM_017980.2 | 989 | tcggggacgtctgctacaactgc | 365032 | - | see also 22476 line |
| 50741 | LIMS2 | NM_017980.2 | 462 | atcctgcggtgagttcatcattg | 365028 | - | see also 22476 line |
| 50742 | LIP8 | NM_053051.1 | 1524 | ctgccgaccgcaagaaagatacc | 405866 | - | see also 22477 line |
| 50743 | LIP8 | NM_053051.1 | 1062 | acggcttcgcccaagaattgagt | 405857 | - | see also 22477 line |
| 50744 | LIPE | NM_005357.2 | 1102 | tgggacgtcgccacatgagaaaa | 333636 | - | see also 22478 line |
| 50745 | LIPE | NM_005357.2 | 2995 | ctccgatgtcaacttcttattac | 333655 | - | see also 22478 line |
| 50746 | LIR9 | NM_021250.2 | 456 | gagataccgctgttactactaca | 380903 | - | see also 22479 line |
| 50747 | LIR9 | NM_021250.2 | 765 | tggctctcgcaggcatatcctgc | 380905 | - | see also 22479 line |
| 50748 | LL5beta | NM_145753.1 | 3489 | atcggaacaagcgaacattctct | 414845 | - | see also 9799 line |
| 50749 | LL5beta | NM_145753.1 | 764 | ctggcgcttcaacccaagttaac | 414784 | - | see also 9799 line |
| 50750 | LLGL2 | NM_004524.1 | 3085 | agcggcaactggcgttcacatcg | 332619 | - | see also 22481 line |
| 50751 | LLGL2 | NM_004524.1 | 963 | ttccttgcaaagcgattaccaga | 332614 | - | see also 22481 line |
| 50752 | LLGL2 | NM_004524.1 | 180 | agcgggacctgttccagtttaac | 332612 | - | see also 22481 line |
| 50753 | LMOD3 | NM_198271.1 | 375 | atcgaaattagatcctaagaagt | 437382 | - | see also 22482 line |
| 50754 | LMOD3 | NM_198271.1 | 646 | aacatgttgcgtgaaaatagaag | 437387 | - | see also 22482 line |
| 50755 | LNIR | NM_030916.1 | 505 | ctccaaatacgggcttcatgtga | 395385 | - | see also 22483 line |
| 50756 | LNIR | NM_030916.1 | 361 | gacctcagacgtggtaactgtgg | 395383 | - | see also 22483 line |
| 50757 | LOC112476 | NM_145239.1 | 1045 | atcgtggccttcgcttatgctgt | 413808 | - | see also 22484 line |
| 50758 | LOC112476 | NM_145239.1 | 1121 | gccgggtagccaagctcttaagc | 413809 | - | see also 22484 line |
| 50759 | LOC112476 | NM_145239.1 | 1180 | gcctcctgcgtcatcaacttagg | 413811 | - | see also 22484 line |
| 50760 | LOC112817 | NM_138413.2 | 1005 | ctggatcggctggctttctgatg | 407985 | - | see also 22485 line |
| 50761 | LOC112817 | NM_138413.2 | 1006 | tggatcggctggctttctgatgg | 407986 | - | see also 22485 line |
| 50762 | LOC112869 | NM_138414.1 | 260 | ttcgcggagcgaacacaacttag | 407990 | - | see also 22486 line |
| 50763 | LOC112869 | NM_138414.1 | 263 | gcggagcgaacacaacttagtga | 407991 | - | see also 22486 line |
| 50764 | LOC113174 | NM_138421.1 | 1095 | agcctcattcgggtcttacaaaa | 408073 | - | see also 22487 line |
| 50765 | LOC113174 | NM_138421.1 | 352 | gtcctcgattaagagaaatctgt | 408055 | - | see also 22487 line |

Figure 46

| 50766 | LOC113174 | NM_138421.1 | 1094 | cagcctcattcgggtcttacaaa | 408072 | - | see also 22487 line |
|---|---|---|---|---|---|---|---|
| 50767 | LOC113444 | NM_138428.2 | 715 | gggatacctgcatgtctagttcc | 408110 | - | see also 22488 line |
| 50768 | LOC113444 | NM_138428.2 | 546 | aagaggaaggccgcatctgttgt | 408105 | - | see also 22488 line |
| 50769 | LOC113612 | NM_183075.1 | 1143 | gtcgctgaaccccgatgtacaag | 435613 | - | see also 22489 line |
| 50770 | LOC113612 | NM_183075.1 | 1290 | gccgcttgccattcctcatatga | 435617 | - | see also 22489 line |
| 50771 | LOC113612 | NM_183075.1 | 755 | agcgctttgattacactaatagt | 435603 | - | see also 22489 line |
| 50772 | LOC113655 | NM_138431.1 | 1353 | ctctgcctttcccgttctgtacc | 408115 | - | see also 22490 line |
| 50773 | LOC113828 | NM_138435.1 | 687 | ctgatggtggacggtgacaaagt | 408121 | - | see also 22492 line |
| 50774 | LOC113828 | NM_138435.1 | 470 | tggtgatatctttcaagacattg | 408119 | - | see also 22492 line |
| 50775 | LOC114659 | NM_052888.1 | 37 | tacgtggcaactattgtctttac | 404590 | - | see also 22493 line |
| 50776 | LOC114659 | NM_052888.1 | 451 | ctcaagtgggttcaaactacaga | 404600 | - | see also 22493 line |
| 50777 | LOC114926 | NM_138436.2 | 307 | cacgataagcaagattcgtttaa | 408136 | - | see also 22494 line |
| 50778 | LOC114926 | NM_138436.2 | 313 | aagcaagattcgtttaagacaac | 408137 | - | see also 22494 line |
| 50779 | LOC114928 | NM_138437.1 | 2376 | gccaatgcgagaacgaagtttca | 408178 | - | see also 22495 line |
| 50780 | LOC114928 | NM_138437.1 | 506 | tggcgctcgttctaaaactgatg | 408143 | - | see also 22495 line |
| 50781 | LOC114990 | NM_138440.1 | 284 | ggggctgtacgtctttgagaacg | 408190 | - | see also 22496 line |
| 50782 | LOC114990 | NM_138440.1 | 922 | agctggatgtgagcaacctaagc | 408192 | - | see also 22496 line |
| 50783 | LOC115098 | NM_138442.2 | 680 | gcggctgtcgcagctgaaacagc | 408212 | - | see also 22497 line |
| 50784 | LOC115106 | NM_138443.2 | 380 | tacctcgctagctagttttatcc | 408220 | - | see also 22498 line |
| 50785 | LOC115106 | NM_138443.2 | 794 | tccgtctcttgctcaagtgaaaa | 408236 | - | see also 22498 line |

Figure 46

| 50786 | LOC115265 | NM_145244.2 | 587 | tagcgtcgtacctacttttgagc | 413829 | - | see also 22499 line |
|---|---|---|---|---|---|---|---|
| 50787 | LOC115265 | NM_145244.2 | 504 | tgcggcttgcgaggttgtgttat | 413822 | - | see also 22499 line |
| 50788 | LOC115265 | NM_145244.2 | 584 | ttctagcgtcgtacctacttttg | 413828 | - | see also 22499 line |
| 50789 | LOC115811 | NM_138451.1 | 188 | ctcgccatggcccctttgtatca | 408248 | - | see also 22500 line |
| 50790 | LOC115811 | NM_138451.1 | 336 | tcctggatgaggcgatctacaag | 408254 | - | see also 22500 line |
| 50791 | LOC115811 | NM_138451.1 | 285 | ttctctctaggaccaaactcacc | 408252 | - | see also 22500 line |
| 50792 | LOC116236 | NM_198147.1 | 1320 | tccgaacggaggagaggattaaa | 437113 | - | see also 22501 line |
| 50793 | LOC116236 | NM_198147.1 | 997 | ccgttccttcgagagtttgagg | 437111 | - | see also 22501 line |
| 50794 | LOC116238 | NM_138463.2 | 749 | tgctggacgtgatgatcataatc | 408307 | - | see also 22502 line |
| 50795 | LOC116238 | NM_138463.2 | 340 | gagacggcgtggtcactttctgg | 408300 | - | see also 22502 line |
| 50796 | LOC118430 | NM_058173.1 | 241 | ttctaccactgctcgtaaagaca | 406198 | - | see also 22503 line |
| 50797 | LOC118812 | NM_178832.2 | 526 | tgctgcgacgtgagaagtgttct | 431999 | - | see also 10222 line |
| 50798 | LOC118812 | NM_178832.2 | 492 | ccccgcaatgaaggtctctttga | 431997 | - | see also 10222 line |
| 50799 | LOC118812 | NM_178832.2 | 413 | gacctttgagggggaatttaaaa | 431995 | - | see also 10222 line |
| 50800 | LOC119395 | NM_182494.1 | 248 | agctcggagtcggtgatgaatgg | 433908 | - | see also 22504 line |
| 50801 | LOC119395 | NM_182494.1 | 332 | ccctgcctggtgcactacaatgc | 433909 | - | see also 22504 line |
| 50802 | LOC119504 | NM_173473.1 | 280 | ctgcgaatctgtttttagctatc | 424920 | - | see also 22505 line |
| 50803 | LOC119504 | NM_173473.1 | 313 | cacgcttaaacaggtgaaacatg | 424921 | - | see also 22505 line |
| 50804 | LOC119710 | NM_138787.1 | 356 | gacaaacagacggaagagatact | 408757 | - | see also 22506 line |
| 50805 | LOC119710 | NM_138787.1 | 446 | aagtttagtcctgcagagataga | 408759 | - | see also 22506 line |

Figure 46

| 50806 | LOC120224 | NM_138788.2 | 209 | agccgcaattaggactttgtttt | 408766 | - | see also 22507 line |
|---|---|---|---|---|---|---|---|
| 50807 | LOC120224 | NM_138788.2 | 753 | gccgtcaactattctcttgttta | 408777 | - | see also 22507 line |
| 50808 | LOC120379 | NM_138789.2 | 947 | atccacgctaatcatcacaatgc | 408807 | - | see also 22508 line |
| 50809 | LOC120379 | NM_138789.2 | 592 | gacagatacgaagcagtactatg | 408793 | - | see also 22508 line |
| 50810 | LOC120526 | NM_181706.2 | 462 | agcctgatttcttgtgatacatg | 432847 | - | see also 22509 line |
| 50811 | LOC120526 | NM_181706.2 | 475 | gtgatacatgttcactaattata | 432848 | - | see also 22509 line |
| 50812 | LOC122258 | NM_145248.2 | 265 | accgttacatgctggtattgatg | 413882 | - | see also 22510 line |
| 50813 | LOC122258 | NM_145248.2 | 406 | tggcgatccttctgagaattatc | 413887 | - | see also 22510 line |
| 50814 | LOC123169 | NM_138792.2 | 1651 | ttccatacgtagggaatctcagc | 408844 | - | see also 22511 line |
| 50815 | LOC123169 | NM_138792.2 | 886 | tgcgattgcatctgattcagaag | 408826 | - | see also 22511 line |
| 50816 | LOC123169 | NM_138792.2 | 876 | aacgcaagaatgcgattgcatct | 408824 | - | see also 22511 line |
| 50817 | LOC124220 | NM_145252.1 | 424 | caccagcaaggaccgctatttct | 413901 | - | see also 22512 line |
| 50818 | LOC124220 | NM_145252.1 | 361 | aggcgaatacatcacaaaagtct | 413899 | - | see also 22512 line |
| 50819 | LOC124220 | NM_145252.1 | 405 | tccggggtatggtcatgtacacc | 413900 | - | see also 22512 line |
| 50820 | LOC124446 | NM_194280.1 | 689 | gggtgaggaatgtgttagtgtct | 436237 | - | see also 22514 line |
| 50821 | LOC124491 | NM_145254.1 | 499 | tacggattttagctactctatag | 413919 | - | see also 9784 line |
| 50822 | LOC124773 | NM_181707.1 | 458 | agcttcgccgactttacaagtac | 432852 | - | see also 22515 line |
| 50823 | LOC124773 | NM_181707.1 | 461 | ttcgccgactttacaagtacacc | 432853 | - | see also 22515 line |
| 50824 | LOC124773 | NM_181707.1 | 455 | agcagcttcgccgactttacaag | 432851 | - | see also 22515 line |
| 50825 | LOC125476 | NM_194281.1 | 163 | ggggctatggcgccagtaagaag | 436238 | - | see also 22516 line |

Figure 46

| | | | | | | | |
|---|---|---|---|---|---|---|---|
| 50826 | LOC126248 | NM_173479.1 | 480 | ttctgtggttcgcgattttgagc | 424953 | - | see also 22517 line |
| 50827 | LOC126248 | NM_173479.1 | 556 | gtcattgccgcatcctatgataa | 424955 | - | see also 22517 line |
| 50828 | LOC126353 | NM_173481.2 | 1131 | tacgtgcagcgggacatagtaca | 424970 | - | see also 22518 line |
| 50829 | LOC126353 | NM_173481.2 | 1376 | cgggacccccagctagaattct | 424971 | - | see also 22518 line |
| 50830 | LOC126731 | NM_145257.1 | 680 | gtgcacgagattcatgaatcagc | 413935 | - | see also 22519 line |
| 50831 | LOC126731 | NM_145257.1 | 67 | cggggtgaagagcgagtacatga | 413928 | - | see also 22519 line |
| 50832 | LOC128153 | NM_138796.2 | 163 | gtcaagttcgggcatatatcagg | 408861 | - | see also 22520 line |
| 50833 | LOC128153 | NM_138796.2 | 161 | atgtcaagttcgggcatatatca | 408860 | - | see also 22520 line |
| 50834 | LOC128344 | NM_181643.1 | 166 | ggcttggcatacgacttatctag | 432634 | - | see also 22521 line |
| 50835 | LOC128344 | NM_181643.1 | 76 | ttccatcccccgaatttgattgg | 432631 | - | see also 22521 line |
| 50836 | LOC129138 | NM_138797.1 | 404 | gactcggccaatgccaatgatgt | 408884 | - | - |
| 50837 | LOC129138 | NM_138797.1 | 406 | ctcggccaatgccaatgatgtgg | 408885 | - | see also 50836 line |
| 50838 | LOC129138 | NM_138797.1 | 703 | ggccaagtcaaagctgaatatcc | 408886 | - | see also 50836 line |
| 50839 | LOC129285 | NM_152994.2 | 1423 | atggactccttcggacaaactac | 421725 | - | see also 9970 line |
| 50840 | LOC129285 | NM_152994.2 | 982 | ttgtgacggctcttctaaacttt | 421714 | - | see also 9970 line |
| 50841 | LOC129531 | NM_138798.1 | 362 | cactttttcgcgaataccttaat | 408893 | - | see also 22524 line |
| 50842 | LOC129531 | NM_138798.1 | 368 | ttcgcgaataccttaatgagaca | 408894 | - | see also 22524 line |
| 50843 | LOC129531 | NM_138798.1 | 360 | gtcactttttcgcgaatacctta | 408892 | - | see also 22524 line |
| 50844 | LOC129642 | NM_138799.2 | 258 | ttcgaacttatctacattcaagc | 408914 | - | see also 9636 line |
| 50845 | LOC129642 | NM_138799.2 | 471 | gccaagttactcgagtctatatc | 408924 | - | see also 9636 line |

Figure 46

| 50846 | LOC129642 | NM_138799.2 | 480 | ctcgagtctatatctttgactat | 408926 | - | see also 9636 line |
|---|---|---|---|---|---|---|---|
| 50847 | LOC130026 | NM_138468.3 | 953 | cacgccaagcaagattagtgaag | 408339 | - | see also 9624 line |
| 50848 | LOC130026 | NM_138468.3 | 907 | ttggctttcatccgtatgatttt | 408335 | - | see also 9624 line |
| 50849 | LOC130589 | NM_138801.1 | 479 | aggctaccccggagagttaaaag | 408985 | - | see also 22527 line |
| 50850 | LOC130589 | NM_138801.1 | 769 | aggacttccatctcaatggtttt | 408991 | - | see also 22527 line |
| 50851 | LOC130888 | NM_174899.3 | 67 | gccgcctagcaaagactattacc | 428533 | - | see also 22528 line |
| 50852 | LOC130888 | NM_174899.3 | 145 | gagtgagtatcgatcaacaaaac | 428540 | - | see also 22528 line |
| 50853 | LOC130888 | NM_174899.3 | 149 | gagtatcgatcaacaaaacctgg | 428541 | - | see also 22528 line |
| 50854 | LOC130940 | NM_138803.2 | 1188 | gaccaatatcgctttgctataga | 409030 | - | see also 22529 line |
| 50855 | LOC130940 | NM_138803.2 | 2008 | aggacatggagtctactgtattt | 409048 | - | see also 22529 line |
| 50856 | LOC130951 | NM_138804.2 | 1017 | gtcatcggcctctcattacaagc | 409068 | - | see also 22530 line |
| 50857 | LOC130951 | NM_138804.2 | 121 | atgcatcctgggcgaactactgg | 409049 | - | see also 22530 line |
| 50858 | LOC131118 | NM_145261.1 | 366 | ctcatcgacgaattatgctttta | 413970 | - | see also 22531 line |
| 50859 | LOC131118 | NM_145261.1 | 364 | tgctcatcgacgaattatgcttt | 413969 | - | see also 22531 line |
| 50860 | LOC131368 | NM_175056.1 | 759 | tccccagtataggattacctttg | 428937 | - | see also 10141 line |
| 50861 | LOC131368 | NM_175056.1 | 926 | cagaccaccgtcattgagaatgg | 428948 | - | see also 10141 line |
| 50862 | LOC132200 | NM_138808.1 | 520 | tgcgactatacaacttgatgttg | 409126 | - | see also 22533 line |
| 50863 | LOC132200 | NM_138808.1 | 511 | ctcagagaatgcgactatacaac | 409124 | - | see also 22533 line |
| 50864 | LOC132200 | NM_138808.1 | 509 | ctctcagagaatgcgactataca | 409123 | - | see also 22533 line |
| 50865 | LOC132321 | NM_173487.1 | 339 | agcatgagcccgtatttatcagg | 425002 | - | see also 22534 line |

Figure 46

| | | | | | | | |
|---|---|---|---|---|---|---|---|
| 50866 | LOC132321 | NM_173487.1 | 535 | tccccacggacagcatttagtgc | 425005 | - | see also 22534 line |
| 50867 | LOC132671 | NM_145263.1 | 1358 | atcgttgacaccatcttatgtgg | 414002 | - | see also 22535 line |
| 50868 | LOC132671 | NM_145263.1 | 626 | tcctcgggatcgggatatgcaac | 413985 | - | see also 22535 line |
| 50869 | LOC132671 | NM_145263.1 | 1818 | ccccgtagccaaattggtttaaa | 414021 | - | see also 22535 line |
| 50870 | LOC133619 | NM_130809.2 | 1398 | gtcccgtcggcagatgatctaca | 407008 | - | see also 22536 line |
| 50871 | LOC133619 | NM_130809.2 | 293 | tcccaccactcgcatactctact | 406970 | - | see also 22536 line |
| 50872 | LOC133957 | NM_145265.2 | 718 | agccggtctcaccgacatatttc | 414034 | - | see also 22537 line |
| 50873 | LOC133957 | NM_145265.2 | 719 | gccggtctcaccgacatatttca | 414035 | - | see also 22537 line |
| 50874 | LOC134218 | NM_194283.1 | 92 | tacggtagtccatcctttctacg | 436293 | - | see also 22538 line |
| 50875 | LOC134218 | NM_194283.1 | 1338 | aacagcgcaacaagtagtcaaag | 436313 | - | see also 22538 line |
| 50876 | LOC134218 | NM_194283.1 | 306 | gtgcaggcgcatcgaaaacttgt | 436298 | - | see also 22538 line |
| 50877 | LOC134285 | NM_173490.3 | 465 | ggcgcaggaaccgctaaacgaga | 425127 | - | see also 22539 line |
| 50878 | LOC134285 | NM_173490.3 | 172 | gggttccaggcatgccaatataa | 425125 | - | see also 22539 line |
| 50879 | LOC134548 | NM_175873.2 | 773 | tggctccacgtaaaaagacaaag | 429386 | - | see also 22540 line |
| 50880 | LOC134548 | NM_175873.2 | 774 | ggctccacgtaaaaagacaaaga | 429387 | - | see also 22540 line |
| 50881 | LOC134548 | NM_175873.2 | 766 | tcgcccatggctccacgtaaaaa | 429382 | - | see also 22540 line |
| 50882 | LOC136263 | NM_145268.2 | 638 | aagccttgagaaccaacgaatgg | 414068 | - | see also 22541 line |
| 50883 | LOC136263 | NM_145268.2 | 673 | tcccgacagaagccttcagtaac | 414069 | - | see also 22541 line |
| 50884 | LOC142678 | NM_080875.1 | 1177 | gacgggcaccgtgcatcgtatca | 406882 | - | see also 22542 line |
| 50885 | LOC142678 | NM_080875.1 | 2137 | cgcgctagctgtgagaaagattc | 406886 | - | see also 22542 line |

Figure 46

| | | | | | | | |
|---|---|---|---|---|---|---|---|
| 50886 | LOC143241 | NM_138812.1 | 253 | atccgatagaatatttagcattg | 409203 | - | see also 22543 line |
| 50887 | LOC143241 | NM_138812.1 | 599 | agctgaaatcagcgatcgttatg | 409214 | - | see also 22543 line |
| 50888 | LOC144097 | NM_138471.1 | 935 | cagacctatgaccaaaaacctgg | 408350 | - | - |
| 50889 | LOC144100 | NM_175058.2 | 813 | accgtcagcgctcccagtttaca | 428961 | - | see also 22544 line |
| 50890 | LOC144100 | NM_175058.2 | 956 | gagcgacactgacgtcaaactga | 428963 | - | see also 22544 line |
| 50891 | LOC144233 | NM_181708.1 | 90 | gccccgttcggaaattttcctca | 432856 | - | see also 10249 line |
| 50892 | LOC144233 | NM_181708.1 | 354 | aagcgagccgaaaaagaatgtcc | 432860 | - | see also 10249 line |
| 50893 | LOC144501 | NM_182507.1 | 238 | ttcgacctcgggcatctctatga | 434013 | - | see also 22546 line |
| 50894 | LOC144501 | NM_182507.1 | 480 | gccctctttccctccagatatcc | 434014 | - | see also 22546 line |
| 50895 | LOC144747 | NM_194286.1 | 664 | tgctgctcgtcgtgaggatttat | 436360 | - | see also 22547 line |
| 50896 | LOC144747 | NM_194286.1 | 399 | gcctgcttcggggaaatctctgc | 436357 | - | see also 22547 line |
| 50897 | LOC145497 | NM_194287.1 | 969 | gccaagccctgtcgaccaattac | 436369 | - | see also 22548 line |
| 50898 | LOC145497 | NM_194287.1 | 1016 | cacaaccaattctctgatgtagg | 436373 | - | see also 22548 line |
| 50899 | LOC145497 | NM_194287.1 | 979 | gtcgaccaattaccaaattaaaa | 436370 | - | see also 22548 line |
| 50900 | LOC146174 | NM_173501.1 | 321 | ctcgtgcgacagtgtcaaacaat | 425172 | - | see also 22549 line |
| 50901 | LOC146174 | NM_173501.1 | 319 | gcctcgtgcgacagtgtcaaaca | 425171 | - | see also 22549 line |
| 50902 | LOC146325 | NM_145270.1 | 378 | accctacaactacaaatgcttcc | 414073 | - | see also 22550 line |
| 50903 | LOC146562 | NM_139170.1 | 1228 | agcgtgggatttggatgacatcc | 409416 | - | see also 22551 line |
| 50904 | LOC146562 | NM_139170.1 | 590 | ttcatcttccagcgaaaccaaac | 409413 | - | see also 22551 line |
| 50905 | LOC146845 | NM_145054.2 | 1993 | atccgcataagtccaggaaatca | 413299 | - | see also 22552 line |

Figure 46

| 50906 | LOC146845 | NM_145054.2 | 1388 | cagcatggaacgacggtaaaatc | 413285 | - | see also 22552 line | | |
|---|---|---|---|---|---|---|---|---|---|
| 50907 | LOC146845 | NM_145054.2 | 2051 | tgcgatggaagtacccatatacc | 413303 | - | see also 22552 line | | |
| 50908 | LOC146853 | NM_145272.2 | 442 | agctcctcttctgcaagaaatgc | 414074 | - | see also 22553 line | | |
| 50909 | LOC147111 | NM_178493.3 | 1437 | gcctctcccatgagatcatcatc | 430835 | - | see also 22554 line | | |
| 50910 | LOC147965 | NM_174905.2 | 964 | gtgttggggtcgcaagaagaaga | 428575 | - | see also 22555 line | | |
| 50911 | LOC148066 | NM_181710.2 | 851 | ctggtcgtatcagccttctttgt | 432871 | - | see also 22556 line | | |
| 50912 | LOC148137 | NM_144692.1 | 329 | ctccgtggtggccaagtatataa | 411338 | - | see also 22557 line | | |
| 50913 | LOC148137 | NM_144692.1 | 327 | gactccgtggtggccaagtatat | 411337 | - | see also 22557 line | | |
| 50914 | LOC148523 | NM_144697.2 | 924 | gacaggatccagcgtattgtagg | 411452 | - | see also 22558 line | | |
| 50915 | LOC148523 | NM_144697.2 | 1310 | aaccggcattggcgtcattcttt | 411459 | - | see also 22558 line | | |
| 50916 | LOC148738 | NM_145277.2 | 1043 | cgcaatcgtcggggagctataac | 414100 | - | see also 22559 line | | |
| 50917 | LOC148738 | NM_145277.2 | 1047 | atcgtcggggagctataaccatt | 414101 | - | see also 22559 line | | |
| 50918 | LOC148823 | NM_145278.1 | 382 | ttcttaggacttctgttagtagg | 414110 | - | see also 22560 line | | |
| 50919 | LOC148823 | NM_145278.1 | 203 | cacttctaatcaggaaaacgaga | 414105 | - | see also 22560 line | | |
| 50920 | LOC148872 | NM_198543.1 | 286 | caccgcttccctgctcagattcg | 439316 | - | - | - | - |
| 50921 | LOC149345 | NM_198149.1 | 208 | ctcctggcatccggggtttaact | 437115 | - | - | - | - |
| 50922 | LOC149345 | NM_198149.1 | 634 | tgcagctcctcctccctatatgc | 437116 | - | see also 50921 line | | |
| 50923 | LOC149469 | NM_182518.1 | 279 | tggcagtatcggtttgtcattga | 434062 | - | see also 22561 line | | |
| 50924 | LOC149469 | NM_182518.1 | 441 | cagcctgagatctatgtaactat | 434065 | - | see also 22561 line | | |
| 50925 | LOC149830 | NM_177549.2 | 1051 | tgctaattcccatgtctaacaca | 430278 | - | - | - | - |

Figure 46

| | | | | | | | |
|---|---|---|---|---|---|---|---|
| 50926 | LOC149830 | NM_177549.2 | 1179 | gactgccaggcatgcaatagaaa | 430281 | - | see also 50925 line |
| 50927 | LOC149830 | NM_177549.2 | 1081 | cccatactgcatggccattatcc | 430280 | - | see also 50925 line |
| 50928 | LOC150159 | NM_139173.1 | 195 | ctcattgatcctaataatactgc | 409420 | - | see also 22562 line |
| 50929 | LOC150159 | NM_139173.1 | 160 | atgaaaacttccaaacatctaca | 409419 | - | see also 22562 line |
| 50930 | LOC150379 | NM_138814.1 | 368 | ggcctgacggacgcaacttcttg | 409216 | - | see also 22563 line |
| 50931 | LOC150379 | NM_138814.1 | 369 | gcctgacggacgcaacttcttgg | 409217 | - | see also 22563 line |
| 50932 | LOC150677 | NM_148961.2 | 348 | gacatggcccgaaccttcttgc | 415176 | - | |
| 50933 | LOC150759 | NM_175853.2 | 68 | aagtctgctagcagactaataca | 429277 | - | see also 22564 line |
| 50934 | LOC150759 | NM_175853.2 | 72 | ctgctagcagactaatacagagg | 429278 | - | see also 22564 line |
| 50935 | LOC151194 | NM_145280.3 | 695 | ttcgctatgaacgggataacaac | 414124 | - | see also 9786 line |
| 50936 | LOC151194 | NM_145280.3 | 441 | gactatcacggatcgaaaagtag | 414115 | - | see also 9786 line |
| 50937 | LOC151194 | NM_145280.3 | 439 | gtgactatcacggatcgaaaagt | 414114 | - | see also 9786 line |
| 50938 | LOC151534 | NM_138482.1 | 882 | ccgcaagtctgagtttctta | 408400 | - | see also 22566 line |
| 50939 | LOC151534 | NM_138482.1 | 961 | cacccgacctcatcttgatgg | 408401 | - | see also 22566 line |
| 50940 | LOC151636 | NM_138287.2 | 874 | cagtcgtgagtctttgctagt | 407405 | - | see also 22567 line |
| 50941 | LOC151636 | NM_138287.2 | 1509 | aaggcgaagcagtatgttctaaa | 407427 | - | see also 22567 line |
| 50942 | LOC151648 | NM_138484.1 | 500 | tggaatggacccaatagtgatg | 408412 | - | see also 22568 line |
| 50943 | LOC151648 | NM_138484.1 | 770 | agccagcgtgaactataaggagc | 408422 | - | see also 22568 line |
| 50944 | LOC151835 | NM_153635.1 | 1645 | ttcgtccattcgagactatgt | 423468 | - | see also 22569 line |
| 50945 | LOC151835 | NM_153635.1 | 382 | ttcgtcctgactatttctttga | 423457 | - | see also 22569 line |

Figure 46

| 50946 | LOC151963 | NM_178496.2 | 434 | aactcatccaagaatttacgaag | 430855 | - | see also 10193 line | | |
| 50947 | LOC151963 | NM_178496.2 | 1005 | tagtcgcatgttgtatgatattg | 430877 | - | see also 10193 line | | |
| 50948 | LOC151963 | NM_178496.2 | 1002 | gagtagtcgcatgttgtatgata | 430876 | - | see also 10193 line | | |
| 50949 | LOC152185 | NM_144718.2 | 2420 | gtctctaacaccaggtagtatgg | 411735 | - | see also 9734 line | | |
| 50950 | LOC152195 | NM_194289.1 | 444 | gcccacttctatgccaaatctct | 436382 | - | - | | - |
| 50951 | LOC152687 | NM_182524.1 | 1461 | atcctcaactcttatattacaca | 434162 | - | see also 22572 line | | |
| 50952 | LOC152687 | NM_182524.1 | 1457 | accaatcctcaactcttatatta | 434161 | - | see also 22572 line | | |
| 50953 | LOC152687 | NM_182524.1 | 1469 | ctcttatattacacaagagaatc | 434163 | - | see also 22572 line | | |
| 50954 | LOC153684 | NM_194290.1 | 553 | gactagcagttgacacaaacaga | 436384 | - | see also 22573 line | | |
| 50955 | LOC154807 | NM_173517.1 | 565 | atcaactacaaacgactagttta | 425321 | - | see also 22574 line | | |
| 50956 | LOC154807 | NM_173517.1 | 483 | ggcctacattctgtactttgtgc | 425320 | - | see also 22574 line | | |
| 50957 | LOC157378 | NM_194291.1 | 1058 | tgcaggctacgttgaagcattgg | 436393 | - | see also 22575 line | | |
| 50958 | LOC157378 | NM_194291.1 | 1083 | tccaggttaggcctgtcaattcc | 436395 | - | see also 22575 line | | |
| 50959 | LOC157567 | NM_198401.1 | 338 | agctcgagggaatgtagacatct | 437700 | - | see also 10317 line | | |
| 50960 | LOC157567 | NM_198401.1 | 435 | ggccatgtggatactatccaatt | 437702 | - | see also 10317 line | | |
| 50961 | LOC157657 | NM_177965.2 | 409 | tgcgttgtatagcctgtgatttc | 430305 | - | see also 22576 line | | |
| 50962 | LOC157657 | NM_177965.2 | 52 | aagtcgagtccaagttttgcaca | 430298 | - | see also 22576 line | | |
| 50963 | LOC158427 | NM_139246.3 | 1583 | cccgacggccacgcatacctagg | 409604 | - | see also 9683 line | | |
| 50964 | LOC159090 | NM_145284.3 | 719 | gacaagccggagaaattatattc | 414138 | - | see also 9788 line | | |
| 50965 | LOC159090 | NM_145284.3 | 564 | ctcggaggaatagtacaacaatt | 414134 | - | see also 9788 line | | |

EP 1 752 536 A1

Figure 46

| | | | | | | | |
|---|---|---|---|---|---|---|---|
| 50966 | LOC159091 | NM_138819.1 | 622 | tccatcagccgccttcatcaaat | 409259 | - | see also 22578 line |
| 50967 | LOC159091 | NM_138819.1 | 779 | ctctaaagtgcattgatttaact | 409267 | - | see also 22578 line |
| 50968 | LOC159091 | NM_138819.1 | 456 | taccgcagacggcaacattctga | 409255 | - | see also 22578 line |
| 50969 | LOC161577 | NM_198524.1 | 885 | gccacagaggttcgcttgaatag | 439123 | - | see also 22579 line |
| 50970 | LOC161577 | NM_198524.1 | 205 | tacgatctaggcctgtttcaaca | 439109 | - | see also 22579 line |
| 50971 | LOC162427 | NM_178126.2 | 408 | gccaagacccgacgcattagaca | 430476 | - | see also 10179 line |
| 50972 | LOC162427 | NM_178126.2 | 414 | acccgacgcattagacaatgaga | 430478 | - | see also 10179 line |
| 50973 | LOC165257 | NM_182528.1 | 1426 | ggctcacggaggcaataataaca | 434174 | - | see also 22581 line |
| 50974 | LOC165257 | NM_182528.1 | 1387 | ttcaggggacgaagtgtatgtga | 434171 | - | see also 22581 line |
| 50975 | LOC165324 | NM_181713.3 | 374 | accgtcaacgacgatttcagaag | 432886 | - | see also 22582 line |
| 50976 | LOC165324 | NM_181713.3 | 380 | aacgacgatttcagaagttattc | 432888 | - | see also 22582 line |
| 50977 | LOC168850 | NM_176814.3 | 2520 | ctgacgtcggtattgaagtaaaa | 429770 | - | see also 22583 line |
| 50978 | LOC168850 | NM_176814.3 | 2522 | gacgtcggtattgaagtaaaagt | 429771 | - | see also 22583 line |
| 50979 | LOC168850 | NM_176814.3 | 1443 | gtgttcgccgtcacattcgaaaa | 429736 | - | see also 22583 line |
| 50980 | LOC169355 | NM_194294.1 | 682 | tgctgacgaactggaccaaaaaa | 436498 | - | see also 22584 line |
| 50981 | LOC169355 | NM_194294.1 | 287 | gccccacagaccgaatgtgaaga | 436493 | - | see also 22584 line |
| 50982 | LOC169355 | NM_194294.1 | 684 | ctgacgaactggaccaaaaaaga | 436499 | - | see also 22584 line |
| 50983 | LOC169611 | NM_182487.1 | 806 | gagacgacaggatctatgtcacc | 433879 | - | see also 22585 line |
| 50984 | LOC169611 | NM_182487.1 | 804 | tcgagacgacaggatctatgtca | 433878 | - | see also 22585 line |
| 50985 | LOC170393 | NM_173541.1 | 248 | agcacgtcgcccatttctcatca | 425743 | - | see also 22586 line |

1702

Figure 46

| | | | | | | | |
|---|---|---|---|---|---|---|---|
| 50986 | LOC170393 | NM_173541.1 | 283 | gaccacgagagcgctttctctgc | 425745 | - | see also 22586 line |
| 50987 | LOC196264 | NM_198275.1 | 114 | gcccatgtccgaggttatgttgg | 437407 | - | see also 22587 line |
| 50988 | LOC196264 | NM_198275.1 | 347 | aagcaaccctaccataaaggaca | 437415 | - | see also 22587 line |
| 50989 | LOC196463 | NM_173542.2 | 840 | tgcgtgtcatcaagaagtactgg | 425746 | - | see also 22588 line |
| 50990 | LOC196463 | NM_173542.2 | 1604 | cgctcccatgggggtatcgatgt | 425752 | - | see also 22588 line |
| 50991 | LOC199675 | NM_174918.1 | 928 | gacgacattagcaggcataaaaa | 428661 | - | see also 22589 line |
| 50992 | LOC199675 | NM_174918.1 | 558 | acccagactatgagaatatcacc | 428657 | - | see also 22589 line |
| 50993 | LOC199964 | NM_182532.1 | 313 | ctccaccctccgctattgcatga | 434204 | - | - |
| 50994 | LOC199964 | NM_182532.1 | 789 | ggcctccacccagctatgagagc | 434205 | - | see also 50993 line |
| 50995 | LOC200186 | NM_181715.1 | 115 | ggcttccaatccgcgcaaattta | 432965 | - | see also 22591 line |
| 50996 | LOC200186 | NM_181715.1 | 218 | acccggttacaggcccaaaaact | 432968 | - | see also 22591 line |
| 50997 | LOC201158 | NM_145301.1 | 770 | tccaggcaactgccaacagatgg | 414269 | - | - |
| 50998 | LOC201191 | NM_174920.2 | 836 | ccccaccatcggcctcgataaga | 428665 | - | see also 22592 line |
| 50999 | LOC201191 | NM_174920.2 | 839 | caccatcggcctcgataagaaga | 428666 | - | see also 22592 line |
| 51000 | LOC201243 | NM_175734.2 | 1027 | ccctcccccgaccatgtttgtcc | 429170 | - | see also 22593 line |
| 51001 | LOC201292 | NM_173547.2 | 1051 | agcgccaaccgtcacttctatct | 425837 | - | see also 10082 line |
| 51002 | LOC201292 | NM_173547.2 | 498 | ggccgaaggccagctactagagc | 425830 | - | see also 10082 line |
| 51003 | LOC201292 | NM_173547.2 | 512 | tactagagctgcgcaagcaaagc | 425831 | - | see also 10082 line |
| 51004 | LOC201895 | NM_174921.1 | 284 | accgggaccctctggtgataatg | 428672 | - | see also 22595 line |
| 51005 | LOC201895 | NM_174921.1 | 209 | tgcaatgagaagactgatcaatc | 428671 | - | see also 22595 line |

Figure 46

| | | | | | | | |
|---|---|---|---|---|---|---|---|
| 51006 | LOC203522 | NM_182540.1 | 1281 | tagattgtgagccaatggtaata | 434267 | - | see also 10268 line |
| 51007 | LOC205251 | NM_174925.1 | 1066 | ggcgagacagatgggactattag | 428697 | - | see also 22597 line |
| 51008 | LOC205251 | NM_174925.1 | 1036 | gtccaaagcctgggaaagtcttc | 428696 | - | see also 22597 line |
| 51009 | LOC220032 | NM_182833.1 | 1396 | agcatgtgggccgtttagtatcc | 435423 | - | see also 22598 line |
| 51010 | LOC220032 | NM_182833.1 | 1473 | ttgttccctaatggttaagaga | 435429 | - | see also 22598 line |
| 51011 | LOC220070 | NM_145308.2 | 305 | gcccacctgctccatgcaaatc | 414338 | - | see also 22599 line |
| 51012 | LOC220070 | NM_145308.2 | 306 | cccacctgctccatgcaaatcc | 414339 | - | see also 22599 line |
| 51013 | LOC220074 | NM_145309.1 | 174 | aacgggactatatgaacacttcg | 414341 | - | see also 22600 line |
| 51014 | LOC220074 | NM_145309.1 | 280 | acgcactgaagcgttcaaagt | 414345 | - | see also 22600 line |
| 51015 | LOC220929 | NM_182755.1 | 1918 | accaaacagacgcccttacagttg | 434962 | - | see also 22601 line |
| 51016 | LOC220929 | NM_182755.1 | 1891 | caccttcgagaccacatgaatac | 434961 | - | see also 22601 line |
| 51017 | LOC221044 | NM_145314.1 | 446 | gacggcctgcaccatcctatct | 414360 | - | see also 22602 line |
| 51018 | LOC221044 | NM_145314.1 | 541 | cccgtgttccagcaatctttcc | 414361 | - | see also 22602 line |
| 51019 | LOC221711 | NM_194299.1 | 1110 | gagacggacagtagacgtttct | 436582 | - | see also 22603 line |
| 51020 | LOC221711 | NM_194299.1 | 1116 | gacagtagacgttttcaatca | 436585 | - | see also 22603 line |
| 51021 | LOC221711 | NM_194299.1 | 1112 | gacggacagtagacgttttctca | 436583 | - | see also 22603 line |
| 51022 | LOC222171 | NM_175887.2 | 925 | ccccaaaccagacaagttatacg | 429498 | - | - |
| 51023 | LOC222171 | NM_175887.2 | 923 | cccccaaaccagacaagttata | 429497 | - | see also 51022 line |
| 51024 | LOC222967 | NM_173565.1 | 592 | ttgtacctacgttggtatgtttt | 426189 | - | see also 22604 line |
| 51025 | LOC222967 | NM_173565.1 | 722 | ccggacggcagcatgtatgaagg | 426196 | - | see also 22604 line |

Figure 46

| 51026 | LOC223075 | NM_194300.1 | 3267 | ggccgagcaactgcaaacgttac | 436625 | - | see also 22605 line | | |
|-------|-----------|-------------|------|-------------------------|--------|---|---------------------|---|---|
| 51027 | LOC223075 | NM_194300.1 | 1983 | aagtagggcgagcatgtcttttt | 436607 | - | see also 22605 line | | |
| 51028 | LOC223075 | NM_194300.1 | 3268 | gccgagcaactgcaaacgttacg | 436626 | - | see also 22605 line | | |
| 51029 | LOC223082 | NM_147128.3 | 1591 | aacaaagccacgaataacctata | 415009 | - | see also 9806 line | | |
| 51030 | LOC223082 | NM_147128.3 | 1606 | aacctataatgaggatgtactga | 415012 | - | see also 9806 line | | |
| 51031 | LOC253012 | NM_198151.1 | 550 | ggcactcggctagcttaccaatg | 437127 | - | see also 10311 line | | |
| 51032 | LOC253012 | NM_198151.1 | 1072 | ttgtcacctttagcaagtataac | 437139 | - | see also 10311 line | | |
| 51033 | LOC253018 | NM_181717.1 | 529 | ctcacacatcacccccttatttgg | 432999 | - | - | - | - |
| 51034 | LOC253018 | NM_181717.1 | 376 | gtgtgactgggtgaagtgttttc | 432998 | - | see also 51033 line | | |
| 51035 | LOC253559 | NM_153184.1 | 835 | ttcgcgcttcctggcatgaattg | 422065 | - | see also 22608 line | | |
| 51036 | LOC253559 | NM_153184.1 | 836 | tcgcgcttcctggcatgaattga | 422066 | - | see also 22608 line | | |
| 51037 | LOC253827 | NM_198080.1 | 382 | ttcattccacgatgtgatcaatt | 436989 | - | - | - | - |
| 51038 | LOC253827 | NM_198080.1 | 155 | tccgggtcgtgtagggataaaaa | 436987 | - | see also 51037 line | | |
| 51039 | LOC253827 | NM_198080.1 | 510 | ctcgtccaactgggaaaagatac | 436991 | - | see also 51037 line | | |
| 51040 | LOC253982 | NM_181718.3 | 973 | gggggcttcgaagctttatgagt | 433004 | - | see also 22609 line | | |
| 51041 | LOC253982 | NM_181718.3 | 971 | gagggggcttcgaagctttatga | 433003 | - | see also 22609 line | | |
| 51042 | LOC254531 | NM_153613.1 | 248 | cccttcgtgcatgagttacatct | 423389 | - | see also 22610 line | | |
| 51043 | LOC254531 | NM_153613.1 | 247 | ccccttcgtgcatgagttacatc | 423388 | - | see also 22610 line | | |
| 51044 | LOC255104 | NM_181719.1 | 847 | ctggcctgacaggatacaagatg | 433014 | - | see also 10250 line | | |
| 51045 | LOC255104 | NM_181719.1 | 802 | cagccggcatagccatcatgacc | 433012 | - | see also 10250 line | | |

Figure 46

| | | | | | | | | | |
|---|---|---|---|---|---|---|---|---|---|
| 51046 | LOC255313 | NM_173571.1 | 499 | cgccgaggaggactcagatatcg | 426260 | - | - | - | - |
| 51047 | LOC255743 | NM_198278.1 | 1253 | accaccggactgacaactatagc | 437487 | - | see also 10315 line | | |
| 51048 | LOC255743 | NM_198278.1 | 798 | aaggcttcgatctcatgtatatt | 437469 | - | see also 10315 line | | |
| 51049 | LOC256643 | NM_198279.1 | 1466 | aaccttacatgaattatactacg | 437520 | - | see also 22613 line | | |
| 51050 | LOC256643 | NM_198279.1 | 1273 | gacctacgacatgacattgaaag | 437514 | - | see also 22613 line | | |
| 51051 | LOC256643 | NM_198279.1 | 1274 | acctacgacatgacattgaaaga | 437515 | - | see also 22613 line | | |
| 51052 | LOC257106 | NM_181720.1 | 2592 | atcccggagtcgtcttagtctgc | 433031 | - | see also 22614 line | | |
| 51053 | LOC257106 | NM_181720.1 | 360 | cacttaccggctctatgacaagt | 433022 | - | see also 22614 line | | |
| 51054 | LOC282966 | NM_182554.1 | 365 | ttgtattggcccagattggatca | 434416 | - | see also 22615 line | | |
| 51055 | LOC282966 | NM_182554.1 | 133 | gaggtcatcctagagaagataga | 434415 | - | see also 22615 line | | |
| 51056 | LOC283031 | NM_194304.1 | 448 | accaccatggatggcctattatt | 436677 | - | - | - | - |
| 51057 | LOC283232 | NM_174940.1 | 242 | aacgtactacgccctgtatttcc | 428766 | - | - | - | - |
| 51058 | LOC283232 | NM_174940.1 | 276 | ctgatgatcacgtataaaagtca | 428769 | - | see also 51057 line | | |
| 51059 | LOC283232 | NM_174940.1 | 274 | tcctgatgatcacgtataaaagt | 428768 | - | see also 51057 line | | |
| 51060 | LOC283241 | NM_175893.2 | 376 | tacgttttaacacctcttttata | 429513 | - | see also 22616 line | | |
| 51061 | LOC283241 | NM_175893.2 | 386 | cacctcttttatacttggtaaaa | 429514 | - | see also 22616 line | | |
| 51062 | LOC283385 | NM_173855.2 | 537 | tgcgcctgagtcaaaatcctaga | 428516 | - | see also 22617 line | | |
| 51063 | LOC283385 | NM_173855.2 | 404 | ttcggacccaaggagtattatga | 428512 | - | see also 22617 line | | |
| 51064 | LOC283385 | NM_173855.2 | 533 | atgctgcgcctgagtcaaaatcc | 428514 | - | see also 22617 line | | |
| 51065 | LOC283476 | NM_175896.2 | 241 | tccatccaactcaaaacctattc | 429522 | - | see also 22618 line | | |

Figure 46

| | | | | | | | |
|---|---|---|---|---|---|---|---|
| 51066 | LOC283476 | NM_175896.2 | 385 | caggctcctggtactttgtcagc | 429530 | - | see also 22618 line |
| 51067 | LOC283487 | NM_178514.2 | 628 | gagaggtaccacgccattagtgc | 430967 | - | see also 22619 line |
| 51068 | LOC283487 | NM_178514.2 | 1028 | gtgtgcccatctcatgtaagaca | 430976 | - | see also 22619 line |
| 51069 | LOC283514 | NM_198849.1 | 367 | cccacctgcggcagatccatagg | 439926 | - | - |
| 51070 | LOC283514 | NM_198849.1 | 449 | gccggctgattggatcatcatgc | 439927 | - | see also 51069 line |
| 51071 | LOC283537 | NM_181785.1 | 637 | gtgatcactacggacgaaaattc | 433331 | - | see also 22620 line |
| 51072 | LOC283537 | NM_181785.1 | 848 | aacaattcgaatagctatcattg | 433338 | - | see also 22620 line |
| 51073 | LOC283537 | NM_181785.1 | 1072 | acctattttaccgaacttacatg | 433350 | - | see also 22620 line |
| 51074 | LOC283687 | NM_175898.2 | 19 | tccgaaagctggtctcctataca | 429533 | - | - |
| 51075 | LOC283849 | NM_178516.2 | 1451 | acccgtctggctcctattactca | 430979 | - | see also 22621 line |
| 51076 | LOC283849 | NM_178516.2 | 1448 | cagacccgtctggctcctattac | 430978 | - | see also 22621 line |
| 51077 | LOC283932 | NM_175901.2 | 604 | gggagaggactacgaaaaagatg | 429538 | - | see also 22622 line |
| 51078 | LOC283932 | NM_175901.2 | 461 | cccggctcccgtctatcatctca | 429536 | - | see also 22622 line |
| 51079 | LOC283932 | NM_175901.2 | 549 | gactgcgacatgggatgtgtagt | 429537 | - | see also 22622 line |
| 51080 | LOC284001 | NM_198082.1 | 2071 | tgccgccactcctgattatgttc | 437008 | - | see also 22623 line |
| 51081 | LOC284001 | NM_198082.1 | 958 | gtcccgggagcttaaggttaaac | 437001 | - | see also 22623 line |
| 51082 | LOC284001 | NM_198082.1 | 2112 | tacgaactctaaagcataagttt | 437009 | - | see also 22623 line |
| 51083 | LOC284018 | NM_181655.1 | 413 | agctatgtgaaaaggtttaatcg | 432804 | - | - |
| 51084 | LOC284033 | NM_175903.2 | 531 | gtgcacttgacccaaggtaaaat | 429542 | - | see also 22624 line |
| 51085 | LOC284033 | NM_175903.2 | 710 | ttcagggttccaatgttattttt | 429546 | - | see also 22624 line |

Figure 46

| | | | | | | | |
|---|---|---|---|---|---|---|---|
| 51086 | LOC284033 | NM_175903.2 | 683 | agcgcattgtgtttaatgaagt | 429545 | - | see also 22624 line |
| 51087 | LOC284296 | NM_175908.3 | 196 | gacgccttgttccatgacattcc | 429547 | - | see also 22625 line |
| 51088 | LOC284296 | NM_175908.3 | 293 | atcgccatgggaagatattttc | 429549 | - | see also 22625 line |
| 51089 | LOC284345 | NM_198850.1 | 288 | ggctacgcctccgatagctatgg | 439929 | - | see also 22626 line |
| 51090 | LOC284467 | NM_182759.1 | 254 | agcgctcccagacggtgaaatgc | 435115 | - | - |
| 51091 | LOC284467 | NM_182759.1 | 429 | cagcagtggacacaaagtcaaaa | 435116 | - | see also 51090 line |
| 51092 | LOC284467 | NM_182759.1 | 430 | agcagtggacacaaagtcaaaac | 435117 | - | see also 51090 line |
| 51093 | LOC284612 | NM_198280.1 | 128 | aggatgatgggcccatttttc | 437531 | - | see also 22628 line |
| 51094 | LOC284612 | NM_198280.1 | 127 | caggatgatgggcccatttttt | 437530 | - | see also 22628 line |
| 51095 | LOC284680 | NM_182581.1 | 829 | ttggcggtgaagcctacttatgt | 434512 | - | see also 22629 line |
| 51096 | LOC284680 | NM_182581.1 | 831 | ggcggtgaagcctacttatgtct | 434513 | - | see also 22629 line |
| 51097 | LOC284697 | NM_183242.1 | 966 | ctccaagagtacgttactcttc | 435654 | - | see also 22630 line |
| 51098 | LOC284697 | NM_183242.1 | 967 | tcccaagagtacgttactcttca | 435655 | - | see also 22630 line |
| 51099 | LOC284837 | NM_194310.1 | 388 | accgggcaaatctgaaacatgg | 436689 | - | see also 22631 line |
| 51100 | LOC284837 | NM_194310.1 | 383 | aacacaccgggcaaatctgaaac | 436688 | - | see also 22631 line |
| 51101 | LOC284948 | NM_198482.1 | 239 | cccacccagctcaataatctgc | 438484 | - | - |
| 51102 | LOC284948 | NM_198482.1 | 391 | gtgcctgtccacttacaaaag | 438485 | - | see also 51101 line |
| 51103 | LOC284948 | NM_198482.1 | 237 | accccaccccagctcaataatct | 438483 | - | see also 51101 line |
| 51104 | LOC285498 | NM_194439.1 | 235 | aagctccttgtcgtacagttttg | 436882 | - | see also 22632 line |
| 51105 | LOC285498 | NM_194439.1 | 236 | agctccttgtcgtacagttttgc | 436883 | - | see also 22632 line |

Figure 46

| 51106 | LOC285513 | NM_198281.1 | 2488 | gccagacccggagatccatttcc | 437569 | - | see also 22633 line | | |
|---|---|---|---|---|---|---|---|---|---|
| 51107 | LOC285513 | NM_198281.1 | 1589 | tgcccagcatacgtgtaaagaag | 437550 | - | see also 22633 line | | |
| 51108 | LOC285533 | NM_173662.1 | 1031 | aaggagcttatcggacaatatct | 427169 | - | see also 22634 line | | |
| 51109 | LOC285533 | NM_173662.1 | 833 | gggttacttggcgatcatgttta | 427166 | - | see also 22634 line | | |
| 51110 | LOC285636 | NM_175921.2 | 895 | cactggttggtacaattatgagg | 429579 | - | see also 22635 line | | |
| 51111 | LOC285636 | NM_175921.2 | 893 | ctcactggttggtacaattatga | 429578 | - | see also 22635 line | | |
| 51112 | LOC285671 | NM_178532.2 | 385 | cccgtttagggggctttaatttt | 431081 | - | see also 22636 line | | |
| 51113 | LOC285671 | NM_178532.2 | 663 | gaggtgcgaccaacatattttga | 431089 | - | see also 22636 line | | |
| 51114 | LOC285852 | NM_198153.1 | 445 | acgcttccgaaaacatcatcact | 437158 | - | - | - | - |
| 51115 | LOC285852 | NM_198153.1 | 539 | cccaacaagcacagttctgatca | 437160 | - | see also 51114 line | | |
| 51116 | LOC285852 | NM_198153.1 | 247 | ggccctatcagcccaaatcctgg | 437157 | - | see also 51114 line | | |
| 51117 | LOC286046 | NM_173683.2 | 639 | cggactcgatatcgaatgtttgc | 427368 | - | see also 10109 line | | |
| 51118 | LOC286046 | NM_173683.2 | 638 | gcggactcgatatcgaatgtttg | 427367 | - | see also 10109 line | | |
| 51119 | LOC286148 | NM_181787.1 | 1670 | gccgtgcctactataataggtct | 433442 | - | see also 22639 line | | |
| 51120 | LOC286148 | NM_181787.1 | 1664 | agcatggccgtgcctactataat | 433440 | - | see also 22639 line | | |
| 51121 | LOC286257 | NM_183241.1 | 566 | tccaggagagtcgctcatcaacc | 435641 | - | - | - | - |
| 51122 | LOC286257 | NM_183241.1 | 588 | cccgggttcaagagtaagaaacc | 435642 | - | see also 51121 line | | |
| 51123 | LOC338799 | NM_178538.2 | 597 | accagcttaaagaatctttcttg | 431146 | - | - | - | - |
| 51124 | LOC339168 | NM_198154.1 | 178 | ttctcggcctttgccttagatga | 437161 | - | - | - | - |
| 51125 | LOC339451 | NM_198317.1 | 1328 | gtcctacgacccgtgactaaca | 437658 | - | see also 22641 line | | |

Figure 46

| 51126 | LOC339451 | NM_198317.1 | 1740 | aactcggtagagagatacagtcc | 437659 | - | see also 22641 line |
|---|---|---|---|---|---|---|---|
| 51127 | LOC339768 | NM_194312.1 | 2242 | tgcgcaaggagcgcatcatcatg | 436693 | - | see also 22642 line |
| 51128 | LOC339768 | NM_194312.1 | 697 | tcgtctggctggtcacattcacc | 436691 | - | see also 22642 line |
| 51129 | LOC339834 | NM_178173.2 | 1020 | agctagacaaggagagtttatag | 430587 | - | see also 22643 line |
| 51130 | LOC339834 | NM_178173.2 | 1019 | aagctagacaaggagagtttata | 430586 | - | see also 22643 line |
| 51131 | LOC340061 | NM_198282.1 | 780 | gcccggattcgaacttacaatca | 437575 | - | see also 22644 line |
| 51132 | LOC340061 | NM_198282.1 | 1366 | tccctctccgcacggatttctct | 437586 | - | see also 22644 line |
| 51133 | LOC340069 | NM_182761.1 | 381 | gtcgtcctattcatcctataaga | 435137 | - | see also 22645 line |
| 51134 | LOC340069 | NM_182761.1 | 385 | tcctattcatcctataagacttg | 435138 | - | see also 22645 line |
| 51135 | LOC341019 | NM_181807.1 | 832 | ggccgcaagacgagtgttcttgg | 433656 | - | see also 22646 line |
| 51136 | LOC341019 | NM_181807.1 | 869 | gccctcgaacctgaagatatacc | 433657 | - | see also 22646 line |
| 51137 | LOC341567 | NM_181788.1 | 427 | cagctgccagcggaaaaaactgt | 433470 | - | see also 22647 line |
| 51138 | LOC344901 | NM_198184.1 | 332 | atccaaaaaggcgatttggtatc | 437190 | - | see also 22648 line |
| 51139 | LOC344901 | NM_198184.1 | 368 | ttggtagaaaccggctttcaaat | 437192 | - | see also 22648 line |
| 51140 | LOC345667 | NM_197941.1 | 492 | aacacaccggcctgtcgaaaaag | 436908 | - | see also 22649 line |
| 51141 | LOC345667 | NM_197941.1 | 802 | tacaccggacaggcatctagtga | 436910 | - | see also 22649 line |
| 51142 | LOC345667 | NM_197941.1 | 310 | gacagtaagccacctgaaaatca | 436904 | - | see also 22649 line |
| 51143 | LOC346673 | NM_182489.1 | 720 | cacgcccgaggagatccttttg | 433900 | - | see also 22650 line |
| 51144 | LOC346673 | NM_182489.1 | 438 | ctccccttggtatctcaactttt | 433894 | - | see also 22650 line |
| 51145 | LOC348094 | NM_182703.2 | 109 | ctgcactgcgcagcccaaaaagg | 434902 | - | see also 22651 line |

EP 1 752 536 A1

Figure 46

| | | | | | | | |
|---|---|---|---|---|---|---|---|
| 51146 | LOC348094 | NM_182703.2 | 860 | aagctgatcgtttctacagatgg | 434911 | - | see also 22651 line |
| 51147 | LOC348645 | NM_198851.1 | 937 | gtccacctggggcgtgacaatgc | 439932 | - | see also 22652 line |
| 51148 | LOC348645 | NM_198851.1 | 947 | ggcgtgacaatgcatttgattca | 439936 | - | see also 22652 line |
| 51149 | LOC348840 | NM_182631.1 | 534 | aggaagagacgtgttccattatt | 434807 | - | see also 22653 line |
| 51150 | LOC348840 | NM_182631.1 | 604 | cactgctcttcatgatggtaaac | 434808 | - | see also 22653 line |
| 51151 | LOC349136 | NM_198285.1 | 1031 | tcgcaggcgtctggtttttcttt | 437648 | - | - | - | - |
| 51152 | LOC349136 | NM_198285.1 | 701 | gccacacattcatcatcaactgc | 437647 | - | see also 51151 line |
| 51153 | LOC352909 | NM_178837.2 | 415 | ggcctcgcaggaggttcaacttc | 432003 | - | see also 22655 line |
| 51154 | LOC352909 | NM_178837.2 | 416 | gcctcgcaggaggttcaacttct | 432004 | - | see also 22655 line |
| 51155 | LOC352909 | NM_178837.2 | 394 | ccctgtcccgagcgaagttctgg | 432002 | - | see also 22655 line |
| 51156 | LOC360200 | NM_182973.1 | 1939 | aaggcgtccgtgggcatcataga | 435522 | - | see also 22656 line |
| 51157 | LOC360200 | NM_182973.1 | 1398 | cagcacccccaccaaatcgatgc | 435518 | - | see also 22656 line |
| 51158 | LOC374654 | NM_198525.1 | 552 | accttacacctgcgcagaaatag | 439129 | - | see also 22657 line |
| 51159 | LOC374654 | NM_198525.1 | 2237 | gggccgttacatgtggataaacc | 439134 | - | see also 22657 line |
| 51160 | LOC374946 | NM_198545.2 | 832 | ttcgactggaccgattatgaaga | 439328 | - | see also 22658 line |
| 51161 | LOC374946 | NM_198545.2 | 838 | tggaccgattatgaagacttaaa | 439330 | - | see also 22658 line |
| 51162 | LOC375108 | NM_198996.1 | 305 | tggcttattcacggatacagacc | 440468 | - | see also 22659 line |
| 51163 | LOC375108 | NM_198996.1 | 652 | accatatagcagattagattaca | 440479 | - | see also 22659 line |
| 51164 | LOC375323 | NM_198560.1 | 646 | cacggctacggtctacaagatct | 439515 | - | see also 22660 line |
| 51165 | LOC375323 | NM_198560.1 | 765 | gggccaagacggggaagtactcc | 439517 | - | see also 22660 line |

Figure 46

| 51166 | LOC375449 | NM_198828.1 | 632 | acgaggagcttgaccacatatta | 439887 | - | see also 22661 line |
|---|---|---|---|---|---|---|---|
| 51167 | LOC375449 | NM_198828.1 | 397 | tcctcctcgggctcagaaactct | 439886 | - | see also 22661 line |
| 51168 | LOC51035 | NM_015853.3 | 291 | gactccccttggacatatcctgg | 355257 | - | see also 22662 line |
| 51169 | LOC51035 | NM_015853.3 | 599 | aggagttagcagccagacaaaga | 355259 | - | see also 22662 line |
| 51170 | LOC51054 | NM_015899.1 | 1591 | gccgcgttaaccgtaaaggaagg | 355382 | - | see also 22663 line |
| 51171 | LOC51054 | NM_015899.1 | 1483 | gccctgaataacgcatatggtaa | 355377 | - | see also 22663 line |
| 51172 | LOC51057 | NM_015910.2 | 1437 | ttggtgctcgtgacctctttatg | 355434 | - | see also 22664 line |
| 51173 | LOC51057 | NM_015910.2 | 1213 | cagcatgagcgccattgtaaacc | 355430 | - | see also 22664 line |
| 51174 | LOC51059 | NM_015912.2 | 1287 | acctccggctggtaaagactttc | 355468 | - | see also 22665 line |
| 51175 | LOC51059 | NM_015912.2 | 850 | gccttcccggaggctaaacataa | 355462 | - | see also 22665 line |
| 51176 | LOC51059 | NM_015912.2 | 1290 | tccggctggtaaagactttcata | 355469 | - | see also 22665 line |
| 51177 | LOC51063 | NM_015916.3 | 892 | tgcgtggtgaggcttatgtctgt | 355473 | - | see also 22666 line |
| 51178 | LOC51063 | NM_015916.3 | 1361 | tgccatcaccggcgtctacttgt | 355477 | - | see also 22666 line |
| 51179 | LOC51063 | NM_015916.3 | 1286 | ggcgctcaacaaggatgatgagg | 355476 | - | see also 22666 line |
| 51180 | LOC51122 | NM_016094.1 | 152 | cggcgcaaacccaaaaatctacg | 356260 | - | see also 6659 line |
| 51181 | LOC51122 | NM_016094.1 | 122 | tgggcggattgctgtggaattcc | 356259 | - | see also 6659 line |
| 51182 | LOC51136 | NM_016125.2 | 550 | ttctgagcgtcaaacttgttatg | 356333 | - | see also 6677 line |
| 51183 | LOC51149 | NM_016175.1 | 644 | cacgactatgtgacctctttata | 356534 | - | - | - | - |
| 51184 | LOC51149 | NM_016175.1 | 361 | gtcctgccacctagaaaaagttc | 356533 | - | see also 51183 line |
| 51185 | LOC51159 | NM_016206.1 | 301 | ttctatcactcttgtaatcgaga | 356557 | - | see also 22669 line |
| 51186 | LOC51159 | NM_016206.1 | 625 | atcttgcgatgtcctaagtctcc | 356561 | - | see also 22669 line |
| 51187 | LOC51159 | NM_016206.1 | 966 | tgcacttgtacaagagactattt | 356566 | - | see also 22669 line |
| 51188 | LOC51161 | NM_016210.2 | 849 | cacggaggaacaagatgagttgg | 356572 | - | see also 22670 line |
| 51189 | LOC51161 | NM_016210.2 | 821 | agctcatgcccatgtacaacttc | 356571 | - | see also 22670 line |
| 51190 | LOC51186 | NM_016303.1 | 531 | tgcgttggaaggttaatcgaaac | 356927 | - | see also 6800 line |
| 51191 | LOC51186 | NM_016303.1 | 536 | tggaaggttaatcgaaaccatcc | 356929 | - | see also 6800 line |
| 51192 | LOC51198 | NM_016349.1 | 176 | tccggtttggcagcagtcattcg | 357206 | - | see also 22672 line |
| 51193 | LOC51204 | NM_016360.2 | 734 | agctcgtcactcttttgacaaaa | 357225 | - | see also 22673 line |
| 51194 | LOC51204 | NM_016360.2 | 648 | atcgaggcattatctaacagtag | 357218 | - | see also 22673 line |
| 51195 | LOC51204 | NM_016360.2 | 733 | gagctcgtcactcttttgacaaa | 357224 | - | see also 22673 line |
| 51196 | LOC51212 | NM_016380.1 | 777 | aagtggctagacggacactattt | 357249 | - | see also 22674 line |
| 51197 | LOC51212 | NM_016380.1 | 606 | ctggtgagaccactaggtattta | 357242 | - | see also 22674 line |
| 51198 | LOC51233 | NM_016449.1 | 330 | gggaaggacgcaccctgttatga | 357562 | - | see also 22675 line |
| 51199 | LOC51233 | NM_016449.1 | 289 | tactgacctgttgtatcaactcc | 357561 | - | see also 22675 line |
| 51200 | LOC51234 | NM_016454.2 | 545 | tacacatgcatcggattggttag | 357575 | - | see also 6865 line |

Figure 46

| | | | | | | | |
|---|---|---|---|---|---|---|---|
| 51201 | LOC51234 | NM_016454.2 | 497 | gggtttggcattggctgtttaca | 357572 | - | see also 6865 line |
| 51202 | LOC51236 | NM_016458.2 | 781 | tggacctgaggtggacattctcc | 357577 | - | see also 22677 line |
| 51203 | LOC51240 | NM_016467.3 | 285 | ggggatgtacgtattttgcatg | 357606 | - | see also 22678 line |
| 51204 | LOC51240 | NM_016467.3 | 542 | gtgttcggatctttggaattaat | 357614 | - | see also 22678 line |
| 51205 | LOC51244 | NM_016474.3 | 768 | gcccgtacattatgaagacattc | 357648 | - | see also 6870 line |
| 51206 | LOC51244 | NM_016474.3 | 1162 | gagtgggaccgcggaaaagaatt | 357651 | - | see also 6870 line |
| 51207 | LOC51249 | NM_016486.2 | 688 | ttcattggtttgccttccttattt | 357806 | - | - |
| 51208 | LOC51249 | NM_016486.2 | 882 | ttccagaaaaagacattaagaga | 357807 | - | see also 51207 line |
| 51209 | LOC51252 | NM_016490.3 | 220 | atcatctcaggcaatacctgg | 357846 | - | - |
| 51210 | LOC51255 | NM_016494.2 | 33 | ggcgtcctatttcgatgaacacg | 357847 | - | - |
| 51211 | LOC51255 | NM_016494.2 | 104 | tggagctcgcaaggtcactttc | 357848 | - | see also 51210 line |
| 51212 | LOC51255 | NM_016494.2 | 255 | gtgcccgtgtgtctttggaat | 357852 | - | see also 51210 line |
| 51213 | LOC51308 | NM_016606.1 | 132 | gccgagacgctcacggatatagt | 358360 | - | see also 6949 line |
| 51214 | LOC51315 | NM_016618.1 | 1082 | gaggaacgttaagcgtacaaaaaa | 358460 | - | see also 22680 line |
| 51215 | LOC51315 | NM_016618.1 | 1057 | aagccgagatgtagtctctaaga | 358459 | - | see also 22680 line |
| 51216 | LOC51320 | NM_016626.2 | 866 | gccatgctacaggaaactatat | 358603 | - | see also 22681 line |
| 51217 | LOC51320 | NM_016626.2 | 691 | ccttatcgtgtggtaggattag | 358596 | - | see also 22681 line |
| 51218 | LOC51333 | NM_016643.1 | 707 | cgcctaagctcgcacttcattcg | 358678 | - | see also 22682 line |
| 51219 | LOC51334 | NM_016644.1 | 891 | tcgagaacgagttcggtttaatg | 358685 | - | see also 22683 line |
| 51220 | LOC51334 | NM_016644.1 | 896 | aacgagttcgtttaatgaaaaa | 358686 | - | see also 22683 line |
| 51221 | LOC51668 | NM_016126.1 | 288 | acgtctaaagagccagttgatt | 356338 | - | see also 22684 line |
| 51222 | LOC51668 | NM_016126.1 | 237 | aaggcttgtaatccaaagttact | 356336 | - | see also 22684 line |
| 51223 | LOC51693 | NM_016209.1 | 324 | cagcccttcgagacaacgaaatt | 356569 | - | see also 22685 line |
| 51224 | LOC51759 | NM_016482.2 | 595 | ctgggcatcgatgctaaaataaa | 357749 | - | see also 22686 line |
| 51225 | LOC51759 | NM_016482.2 | 596 | tgggcatgcatgctaaataaaaa | 357750 | - | see also 22686 line |
| 51226 | LOC51759 | NM_016482.2 | 349 | ctgcacctgggacatcgttttc | 357739 | - | see also 22686 line |
| 51227 | LOC54499 | NM_019026.1 | 155 | cggacactctcctcatcgtttt | 374509 | - | see also 22687 line |
| 51228 | LOC54499 | NM_019026.1 | 436 | gccctaatgggaatgttcaattc | 374515 | - | see also 22687 line |
| 51229 | LOC55565 | NM_017530.1 | 768 | cggaaggaaccagcgatcctaactgt | 358975 | - | see also 22688 line |
| 51230 | LOC55565 | NM_017530.1 | 1331 | atgattgccgagctcagtttgg | 358983 | - | see also 22688 line |
| 51231 | LOC55580 | NM_017571.1 | 1642 | ctcgtcgttctgataaatta | 359198 | - | see also 22689 line |
| 51232 | LOC55580 | NM_017571.1 | 995 | agcggttgccgcacatattcaag | 359182 | - | see also 22689 line |
| 51233 | LOC55831 | NM_018447.1 | 330 | gacagtcaagtcctaattcggaag | 371553 | - | see also 7484 line |
| 51234 | LOC55831 | NM_018447.1 | 645 | atcgagctactcacattagatgc | 371568 | - | see also 7484 line |
| 51235 | LOC55908 | NM_018687.3 | 807 | accgagaatttgaggtcttaaag | 372547 | - | see also 22691 line |
| 51236 | LOC55908 | NM_018687.3 | 575 | ctctatgccgcacaatagaact | 372545 | - | see also 22691 line |
| 51237 | LOC55924 | NM_019099.3 | 624 | gtgttaggggacaacgttttgc | 375226 | - | see also 22692 line |

Figure 46

| | | | | | | | |
|---|---|---|---|---|---|---|---|
| 51238 | LOC55924 | NM_019099.3 | 770 | cgccctgacagccaaacatcttta | 375227 | - | see also 22692 line |
| 51239 | LOC56181 | NM_019557.3 | 369 | ctgtctcgaagtgtagtaagaaga | 375309 | - | see also 7713 line |
| 51240 | LOC56181 | NM_019557.3 | 990 | aggatgactgcgtctctttgtcc | 375319 | - | see also 7713 line |
| 51241 | LOC56270 | NM_019613.2 | 1105 | cagacggcagctactacaaattc | 375532 | - | see also 7723 line |
| 51242 | LOC56270 | NM_019613.2 | 601 | gtgtcctttgtcccaatagtaac | 375520 | - | see also 7723 line |
| 51243 | LOC56901 | NM_020142.3 | 356 | tgggcagcgctgcgctttacttg | 375674 | - | see also 22695 line |
| 51244 | LOC56901 | NM_020142.3 | 479 | cagtttccactgactataagaag | 375676 | - | see also 22695 line |
| 51245 | LOC56965 | NM_020213.1 | 1063 | ctcgtgaactatgcgttttctcc | 376222 | - | see also 22696 line |
| 51246 | LOC56965 | NM_020213.1 | 1297 | ctcttgatagttgtatgtctatt | 376226 | - | see also 22696 line |
| 51247 | LOC57146 | NM_020422.3 | 512 | ctctgctggggtcataatattg | 377400 | - | see also 22697 line |
| 51248 | LOC57146 | NM_020422.3 | 606 | cgccatgtcgggatgatgatgatag | 377403 | - | see also 22697 line |
| 51249 | LOC57149 | NM_020424.2 | 406 | aacggcaacacgacacaagaagtcc | 377407 | - | see also 22698 line |
| 51250 | LOC57149 | NM_020424.2 | 637 | tccttacccaaggccaattcatc | 377413 | - | see also 22698 line |
| 51251 | LOC57149 | NM_020424.2 | 535 | aacgctgttccggaaaaaacaaaa | 377408 | - | see also 22698 line |
| 51252 | LOC57228 | NM_020467.2 | 443 | ggcagcgagaaagaggaatattt | 377732 | - | see also 22699 line |
| 51253 | LOC57228 | NM_020467.2 | 478 | cccaggcccaaggagcttattc | 377734 | - | see also 22699 line |
| 51254 | LOC57399 | NM_020669.1 | 269 | cacaagctacggtattatgttaa | 377903 | - | - |
| 51255 | LOC57399 | NM_020669.1 | 273 | agctacggtattatgttaacacc | 377904 | - | see also 51254 line |
| 51256 | LOC57399 | NM_020669.1 | 154 | tagcaaaaggcgtgatagtaaac | 377901 | - | see also 51254 line |
| 51257 | LOC57821 | NM_021179.1 | 1084 | gtcaactcagatcgaagtaaagg | 380633 | - | see also 22700 line |
| 51258 | LOC57821 | NM_021179.1 | 1049 | caccactcaaccgcaagacttgt | 380632 | - | see also 22700 line |
| 51259 | LOC63920 | NM_022090.2 | 811 | atgtatcaatttgcgtatttat | 382265 | - | see also 22701 line |
| 51260 | LOC63920 | NM_022090.2 | 2257 | tcctcgtttctcggacatcatg | 382280 | - | see also 22701 line |
| 51261 | LOC63920 | NM_022090.2 | 2264 | ttctcggaacatcattgaacaaaa | 382282 | - | see also 22701 line |
| 51262 | LOC65122 | NM_023014.1 | 1240 | ggcagcaattgcatgtctattga | 384984 | - | see also 22702 line |
| 51263 | LOC65122 | NM_023014.1 | 308 | atctggagccattgaaagcattg | 384983 | - | see also 22702 line |
| 51264 | LOC80298 | NM_025198.2 | 828 | atgtggtcattagcagacttttg | 394330 | - | see also 22703 line |
| 51265 | LOC80298 | NM_025198.2 | 1377 | atggacatctagcaaatctaaat | 394343 | - | see also 22703 line |
| 51266 | LOC81558 | NM_030802.2 | 1062 | ccctcgacctaatcatagctaca | 395205 | - | see also 22704 line |
| 51267 | LOC81558 | NM_030802.2 | 332 | ttcagtccgcgtacattctcc | 395204 | - | see also 22704 line |
| 51268 | LOC81558 | NM_030802.2 | 1067 | gacctaatcatagctacatcttc | 395206 | - | see also 22704 line |
| 51269 | LOC84664 | NM_032577.1 | 595 | tgcacacggtgcactctacaagt | 400829 | - | - |
| 51270 | LOC85865 | NM_033107.1 | 667 | agctgatcttccgggtttaatag | 403273 | - | see also 22705 line |
| 51271 | LOC85865 | NM_033107.1 | 882 | ctgcactcttggcagttaataaa | 403284 | - | see also 22705 line |
| 51272 | LOC88523 | NM_033111.2 | 2152 | gagtcgtatcggtgtaaagaaa | 403330 | - | see also 22706 line |
| 51273 | LOC88523 | NM_033111.2 | 2150 | cagagtcgtatcggtgtaaaaga | 403329 | - | see also 22706 line |
| 51274 | LOC88523 | NM_033111.2 | 2047 | acctcaaccggatataccaaaga | 403327 | - | see also 22706 line |

Figure 46

| | | | | | | | |
|---|---|---|---|---|---|---|---|
| 51275 | LOC89894 | NM_138341.1 | 978 | atccgagcccagacattgtataa | 407538 | - | see also 22707 line |
| 51276 | LOC89894 | NM_138341.1 | 979 | tccgagcccagacattgtataag | 407539 | - | see also 22707 line |
| 51277 | LOC90050 | NM_138344.2 | 499 | ggcatccgacagtgatgtgaaga | 407552 | - | see also 22708 line |
| 51278 | LOC90167 | NM_194277.1 | 1959 | cagacatgaaaactattcgttt | 436225 | - | see also 22709 line |
| 51279 | LOC90167 | NM_194277.1 | 1724 | aggacaagtccacatagctatgt | 436218 | - | see also 22709 line |
| 51280 | LOC90167 | NM_194277.1 | 606 | cacaaactcggtacttaccaaac | 436192 | - | see also 22709 line |
| 51281 | LOC90268 | NM_138348.3 | 726 | ctgaggacgatcggcactataac | 407558 | - | see also 22710 line |
| 51282 | LOC90268 | NM_138348.3 | 508 | tgctcgggacacatcaaatgacc | 407557 | - | see also 22710 line |
| 51283 | LOC90313 | NM_138349.2 | 753 | atccctgagggagagtaatgc | 407561 | - | see also 22711 line |
| 51284 | LOC90313 | NM_138349.2 | 618 | cccaggcagccctcttctagtgg | 407560 | - | see also 22711 line |
| 51285 | LOC90355 | NM_033211.2 | 571 | ccgttcctcgtttgtataaa | 403583 | - | see also 9380 line |
| 51286 | LOC90355 | NM_033211.2 | 570 | gccgttcctcgtttgtataa | 403582 | - | see also 9380 line |
| 51287 | LOC90355 | NM_033211.2 | 572 | ccgttcctctcgtttgtataaaa | 403584 | - | see also 9380 line |
| 51288 | LOC90378 | NM_138352.1 | 1394 | tggatgtcgtcgaatatttact | 407565 | - | see also 22713 line |
| 51289 | LOC90378 | NM_138352.1 | 1393 | atggatgtcgtcgaatattttac | 407564 | - | see also 22713 line |
| 51290 | LOC90410 | NM_174887.2 | 274 | cagaaaatagttggtggtttaat | 428526 | - | - |
| 51291 | LOC90522 | NM_033557.1 | 194 | gacccccaccagctttcgatga | 404131 | - | see also 22714 line |
| 51292 | LOC90522 | NM_033557.1 | 256 | ggcacctggtggcctgagttatc | 404132 | - | see also 22714 line |
| 51293 | LOC90522 | NM_033557.1 | 991 | ggcgcgagcctatgctcatgtact | 404140 | - | see also 22714 line |
| 51294 | LOC90529 | NM_178122.2 | 457 | tcgagccgggtttatgtcaaaaa | 430448 | - | see also 22715 line |
| 51295 | LOC90529 | NM_178122.2 | 456 | ctcgagccggtttatgtcaaaa | 430447 | - | see also 22715 line |
| 51296 | LOC90580 | NM_138358.2 | 86 | cggctctgaggagatttggtcc | 407603 | - | see also 22716 line |
| 51297 | LOC90624 | NM_181705.1 | 40 | gggacgggcagtcaaggtttac | 432838 | - | see also 22717 line |
| 51298 | LOC90624 | NM_181705.1 | 39 | tgggacgggcagtcaaggttta | 432837 | - | see also 22717 line |
| 51299 | LOC90736 | NM_138362.1 | 237 | tactgaaccgatgcaaacttc | 407611 | - | see also 22718 line |
| 51300 | LOC90736 | NM_138362.1 | 134 | aagatacagagtatttcatgg | 407610 | - | see also 22718 line |
| 51301 | LOC90768 | NM_178838.2 | 780 | tcgcgccaccctccactttcagc | 432008 | - | see also 22719 line |
| 51302 | LOC90799 | NM_138363.1 | 2045 | atcgttcgtgctcgaaaatatta | 407660 | - | see also 22720 line |
| 51303 | LOC90799 | NM_138363.1 | 291 | aggtcagcttgtctcacataaca | 407621 | - | see also 22720 line |
| 51304 | LOC90806 | NM_144567.2 | 1095 | atccaaggcgaggtgatattaag | 409720 | - | see also 22721 line |
| 51305 | LOC90806 | NM_144567.2 | 1099 | aaggcgaggtgatattaagctga | 409722 | - | see also 22721 line |
| 51306 | LOC90850 | NM_178167.2 | 1896 | gccagttgccgtgcgttaatg | 430558 | - | see also 22722 line |
| 51307 | LOC90850 | NM_178167.2 | 2385 | ctccgcagcccagtattacaaga | 430559 | - | see also 22722 line |
| 51308 | LOC90925 | NM_175870.3 | 138 | ctctgaggatctctgtagact | 429375 | - | - |
| 51309 | LOC91056 | NM_138368.2 | 2054 | taccagctactgtgtagccatcc | 407758 | - | see also 22723 line |
| 51310 | LOC91056 | NM_138368.2 | 1238 | gagtgctacctcaactttctac | 407755 | - | see also 22723 line |
| 51311 | LOC91272 | NM_138369.1 | 520 | atggaatcctacgatgaacaaaa | 407759 | - | - |

Figure 46

| | | | | | | | |
|---|---|---|---|---|---|---|---|
| 51312 | LOC91272 | NM_138369.1 | 521 | tggaatcctacgatgaacaaaaa | 407760 | - | see also 51311 line |
| 51313 | LOC91526 | NM_153697.1 | 1979 | cccggaggccgtcgatgtgaaag | 423640 | - | see also 10007 line |
| 51314 | LOC91526 | NM_153697.1 | 1280 | cagccggagcagattccataaaa | 423615 | - | see also 10007 line |
| 51315 | LOC91689 | NM_033318.3 | 86 | agccggctcgctggctagtattgg | 403699 | - | see also 22725 line |
| 51316 | LOC91689 | NM_033318.3 | 138 | gggcctagcttgaggaaagatgg | 403700 | - | see also 22725 line |
| 51317 | LOC91752 | NM_194250.1 | 2896 | ttctatcgaaaacgtagacaaca | 436124 | - | see also 10304 line |
| 51318 | LOC91752 | NM_194250.1 | 3122 | atcctcggatagttaataagt | 436136 | - | see also 10304 line |
| 51319 | LOC91752 | NM_194250.1 | 3560 | ctgagtgctgcgttataattca | 436149 | - | see also 10304 line |
| 51320 | LOC91937 | NM_138379.1 | 1166 | aagacgaagaacggcctttacc | 407820 | - | see also 22727 line |
| 51321 | LOC91937 | NM_138379.1 | 408 | tggctggttcaacgatgtaaaga | 407812 | - | see also 22727 line |
| 51322 | LOC91947 | NM_183376.1 | 452 | ggccggtgaaggcatcatttat | 435723 | - | see also 22728 line |
| 51323 | LOC91947 | NM_183376.1 | 229 | agcgcaagggctgctattccagc | 435722 | - | see also 22728 line |
| 51324 | LOC91966 | NM_178124.3 | 771 | aagtttggtcgaggagtgatagc | 430466 | - | see also 22729 line |
| 51325 | LOC91966 | NM_178124.3 | 820 | tgcaatgccccgaagacttaact | 430469 | - | see also 22729 line |
| 51326 | LOC92170 | NM_138384.1 | 774 | tagtcttgcgccaaacctttgg | 407827 | - | see also 22730 line |
| 51327 | LOC92170 | NM_138384.1 | 426 | accgctaccaccgaaaagagaac | 407825 | - | see also 22730 line |
| 51328 | LOC92305 | NM_138385.1 | 749 | atgtgccttgccgtcagaaaa | 407829 | - | - |
| 51329 | LOC92305 | NM_138385.1 | 1006 | accaggtgccgtrgattgtga | 407832 | - | see also 51328 line |
| 51330 | LOC92305 | NM_138385.1 | 751 | gtgccttgcgcttcagaaaagc | 407830 | - | see also 51328 line |
| 51331 | LOC92345 | NM_138386.1 | 1281 | gagcatgcaaaaggatatcgtaa | 407855 | - | see also 22731 line |
| 51332 | LOC92345 | NM_138386.1 | 845 | gactaacctacctccagttaatg | 407840 | - | see also 22731 line |
| 51333 | LOC92345 | NM_138386.1 | 1122 | gaggccttagattttagtgatga | 407850 | - | see also 22731 line |
| 51334 | LOC92346 | NM_139240.2 | 643 | agctgtcttccacggattactga | 409460 | - | see also 22732 line |
| 51335 | LOC92346 | NM_139240.2 | 664 | gacagaggcctacaaaactctaa | 409461 | - | see also 22732 line |
| 51336 | LOC92689 | NM_138389.1 | 1506 | tacatgtcattgaaagtct | 407907 | - | see also 9605 line |
| 51337 | LOC92689 | NM_138389.1 | 429 | gacgtgactgaggatacacttgc | 407886 | - | see also 9605 line |
| 51338 | LOC92691 | NM_138390.2 | 478 | agccgctacgtcaccttaactgg | 407928 | - | see also 22734 line |
| 51339 | LOC92691 | NM_138390.2 | 346 | taccgctcagacaatgagaaaac | 407922 | - | see also 22734 line |
| 51340 | LOC92715 | NM_138778.1 | 836 | gggtaccgcaaattctcttc | 408748 | - | see also 22735 line |
| 51341 | LOC92715 | NM_138778.1 | 832 | accaggtaccccgccaaattct | 408746 | - | see also 22735 line |
| 51342 | LOC92979 | NM_138396.3 | 1344 | agcgctgcggttatacaatcttg | 407952 | - | see also 22736 line |
| 51343 | LOC92979 | NM_138396.3 | 1341 | ctcagcgctgcggttatacaatc | 407951 | - | see also 22736 line |
| 51344 | LOC93109 | NM_138399.2 | 398 | gttggatccaaattcaagtcaat | 407959 | - | see also 22737 line |
| 51345 | LOC93109 | NM_138399.2 | 319 | ctcacaatccaggtttcactgg | 407956 | - | see also 22737 line |
| 51346 | LOC93343 | NM_138401.1 | 172 | aagagcttcgcgcagaaatctgg | 407964 | - | see also 22738 line |
| 51347 | LOC93343 | NM_138401.1 | 427 | aagacagtgcctggataccttcg | 407966 | - | see also 22738 line |
| 51348 | LOC93380 | NM_173470.1 | 466 | acctgttacggtatagttcatat | 424895 | - | see also 10066 line |

Figure 46

| | | | | | | | |
|---|---|---|---|---|---|---|---|
| 51349 | LOC93380 | NM_173470.1 | 472 | tacggtatagttcatattgcagg | 424897 | - | see also 10066 line |
| 51350 | LOC93380 | NM_173470.1 | 465 | tacctgttacggtatagttcata | 424894 | - | see also 10066 line |
| 51351 | LOC93426 | NM_130784.1 | 180 | cccgagttgaggtcctgattaac | 406926 | - | see also 22740 line |
| 51352 | LOC93426 | NM_130784.1 | 295 | ctcgctgcatggaggaagaaagtac | 406927 | - | see also 22740 line |
| 51353 | LOH11CR2A | NM_014622.3 | 612 | agcatcgctgccaccatagattc | 341387 | - | see also 22741 line |
| 51354 | LOH11CR2A | NM_014622.3 | 1268 | cacgtttagtgtaattaaagaag | 341403 | - | see also 22741 line |
| 51355 | LOH12CR1 | NM_058169.2 | 607 | ctgcctccgatatcaagatcacc | 406194 | - | see also 22742 line |
| 51356 | LOH12CR1 | NM_058169.2 | 605 | ctctgcctccgatatcaagatca | 406193 | - | see also 22742 line |
| 51357 | LOH3CR2A | NM_013343.1 | 601 | ggcacaataacgtgctacatacc | 338779 | - | see also 22743 line |
| 51358 | LOH3CR2A | NM_013343.1 | 740 | agcgaaccaagccaataagcttt | 338784 | - | see also 22743 line |
| 51359 | LOST1 | NM_172367.1 | 782 | tggctcggctgccagcattacc | 424477 | - | see also 22744 line |
| 51360 | LOST1 | NM_172367.1 | 836 | tggccgtgaccgtcaacttcaca | 424478 | - | see also 22744 line |
| 51361 | LPPR2 | NM_022737.1 | 1266 | tggatagcccctcgaaaagaac | 383792 | - | see also 22745 line |
| 51362 | LPPR2 | NM_022737.1 | 1180 | caactgcgttgcataacttc | 383788 | - | see also 22745 line |
| 51363 | LR8 | NM_014020.2 | 291 | gacgcaaaacacggtgattgtga | 339206 | - | see also 22746 line |
| 51364 | LR8 | NM_014020.2 | 965 | gagtaggtcttgaaacttgtgt | 339214 | - | see also 22746 line |
| 51365 | LRIG1 | NM_015541.1 | 1612 | aacgagatttcgggcaccaataga | 353809 | - | see also 22747 line |
| 51366 | LRIG1 | NM_015541.1 | 996 | tccacacggaccgcctataaagg | 353804 | - | see also 22747 line |
| 51367 | LRIG1 | NM_015541.1 | 2471 | tgccggatgacgacgtgtttttc | 353820 | - | see also 22748 line |
| 51368 | LRP11 | NM_032832.3 | 1740 | gactacgactggtgaaacagaaa | 401814 | - | see also 22748 line |
| 51369 | LRP11 | NM_032832.3 | 1447 | gggtgactccttggtggaaaagt | 401809 | - | see also 22748 line |
| 51370 | LRP15 | NM_052953.2 | 1063 | gagcatggcgtccaatcatgaga | 405523 | - | see also 22749 line |
| 51371 | LRP15 | NM_052953.2 | 1086 | cagcccacaacgtgatcgtaaa | 405524 | - | see also 22749 line |
| 51372 | LRP16 | NM_014067.2 | 596 | ggcgttgacggctgcattcatcg | 339516 | - | see also 22750 line |
| 51373 | LRP16 | NM_014067.2 | 378 | agcggagggaacattacttctgc | 339515 | - | see also 22750 line |
| 51374 | Lrp2bp | NM_181726.1 | 671 | gagtctcggaagtgtagaaata | 433044 | - | see also 22751 line |
| 51375 | Lrp2bp | NM_181726.1 | 669 | cggagtctcggaagtgtagaaaa | 433043 | - | see also 22751 line |
| 51376 | Lrp2bp | NM_181726.1 | 673 | gtctcggaagtgtagaaaatacc | 433045 | - | see also 22751 line |
| 51377 | LRRC1 | NM_025168.1 | 807 | ttccagctagtcaaattacgaaa | 368508 | - | see also 22752 line |
| 51378 | LRRC1 | NM_025168.1 | 808 | tccagctagtcaaattacgaaag | 368509 | - | see also 22752 line |
| 51379 | LRRC16 | NM_017640.2 | 1571 | aagcgagattctcggaaaagtag | 359922 | - | see also 7063 line |
| 51380 | LRRC16 | NM_017640.2 | 1059 | cagtttaaacgttccatcatcaca | 359911 | - | see also 7063 line |

Figure 46

| | | | | | | | |
|---|---|---|---|---|---|---|---|
| 51381 | LRRC17 | NM_005824.1 | 436 | tcccgtgacgtgtaccacatat | 334277 | - | see also 22754 line |
| 51382 | LRRC17 | NM_005824.1 | 843 | aactacgccaagtgtgaaagtcc | 334295 | - | see also 22754 line |
| 51383 | LRRC17 | NM_005824.1 | 440 | gtgtgacgtgtacacatatctcc | 334280 | - | see also 22754 line |
| 51384 | LRRC19 | NM_022901.1 | 931 | ctgaacgacttcacttcattt | 384746 | - | see also 22755 line |
| 51385 | LRRC19 | NM_022901.1 | 967 | tgcccaatatggtacaatatct | 384749 | - | see also 22755 line |
| 51386 | LRRC2 | NM_024512.2 | 914 | gaccgacctgccgcaagatatag | 386549 | - | see also 22756 line |
| 51387 | LRRC2 | NM_024512.2 | 1165 | gtgaacggatcgccaacatttt | 386557 | - | see also 22756 line |
| 51388 | LRRC3 | NM_030891.1 | 665 | atcgcctacgtcgtgtactatgt | 395353 | - | - |
| 51389 | LRRC3 | NM_030891.1 | 667 | cgcctacgtcgtgtactatgtgc | 395354 | - | see also 51388 line |
| 51390 | LRRC3 | NM_030891.1 | 410 | ggcaaactcagcgccaagatacg | 395352 | - | see also 51388 line |
| 51391 | LRRC4 | NM_022143.3 | 1537 | aggacacaacgcgaaagtacaag | 382491 | - | see also 8308 line |
| 51392 | LRRC5 | NM_018103.3 | 1980 | ttcttcgcatcacttgagatgc | 366760 | - | see also 22757 line |
| 51393 | LRRC5 | NM_018103.3 | 2131 | ccgagagttgcggcaccttaaga | 366768 | - | see also 22757 line |
| 51394 | LRRC8 | NM_019594.1 | 933 | tgcggctcaagagagcaacctaagc | 375436 | - | see also 22758 line |
| 51395 | LRRC8 | NM_019594.1 | 719 | cacacagcggccaagattgaagc | 375434 | - | see also 22758 line |
| 51396 | LRRN1 | NM_020873.3 | 724 | accgagatgactgattacgtct | 379808 | - | see also 8011 line |
| 51397 | LRRN1 | NM_020873.3 | 1417 | ctgcgtgagatcagtatccatag | 379816 | - | see also 8011 line |
| 51398 | LRRN3 | NM_018334.3 | 1625 | ctgatttccatcgatagtcttgc | 370479 | - | see also 7407 line |
| 51399 | LRRN3 | NM_018334.3 | 1560 | tacgaaggggtgattttagcaat | 370477 | - | see also 7407 line |
| 51400 | LRRN4 | NM_002319.2 | 1181 | agcagcgaccacccgaattagc | 330869 | - | see also 22761 line |
| 51401 | LRRN4 | NM_002319.2 | 1883 | ttctagaagcctgtcgaaaatg | 330873 | - | see also 22761 line |
| 51402 | LSR7 | NM_018559.2 | 748 | cacatctgagatcgatcaatct | 372225 | - | see also 22762 line |
| 51403 | LSR7 | NM_018559.2 | 1098 | cacacggcaaagcaatatgttt | 372239 | - | see also 22762 line |
| 51404 | LSR7 | NM_018559.2 | 1096 | ttcacacggcaaaggcaatatgt | 372238 | - | see also 22762 line |
| 51405 | LUC7L | NM_018032.2 | 965 | tcccggtcccgagatagacatcg | 365520 | - | see also 7214 line |
| 51406 | LUC7L | NM_018032.2 | 939 | ccgagacgacggaaattgtcc | 365517 | - | see also 7214 line |
| 51407 | LUC7L | NM_018032.2 | 1044 | accgaagtgcgaaatacaagtaa | 365523 | - | see also 7214 line |
| 51408 | LUC7L2 | NM_016019.1 | 52 | tcccgggacggagatacaactcg | 355880 | - | see also 6617 line |
| 51409 | LUC7L2 | NM_016019.1 | 956 | gtcggcacagttctagagatagg | 355895 | - | see also 6617 line |
| 51410 | LXN | NM_020169.2 | 821 | tccacaatacggccactaaagtaa | 375749 | - | see also 22763 line |
| 51411 | LXN | NM_020169.2 | 828 | tacggcactaaagtaaaacataa | 375751 | - | see also 22763 line |
| 51412 | LY6G6C | NM_025261.1 | 192 | atgcctgacaacacatgccatacc | 394623 | - | see also 22764 line |
| 51413 | LY6G6C | NM_025261.1 | 196 | ctgacaacacatgcatccttgg | 394624 | - | see also 22764 line |
| 51414 | LY6G6D | NM_021246.1 | 809 | accgaatgcggtgctacaactgt | 380900 | - | see also 22765 line |
| 51415 | LY6G6D | NM_021246.1 | 964 | gtcgcagcccatcattgcaatca | 380902 | - | see also 22765 line |
| 51416 | LY6G6D | NM_021246.1 | 377 | tgctcaaaggatcccagttatct | 380896 | - | see also 22765 line |
| 51417 | LYAR | NM_017816.1 | 285 | tgcattgactgcggtaaagattt | 362687 | - | see also 22766 line |

Figure 46

| | | | | | | | |
|---|---|---|---|---|---|---|---|
| 51418 | LYAR | NM_017816.1 | 284 | ttgcattgactgcggtaaagatt | 362686 | - | see also 22766 line |
| 51419 | LYRIC | NM_178812.2 | 1821 | cagccgaagtactcgtcaaaaat | 431364 | - | see also 10201 line |
| 51420 | LYRIC | NM_178812.2 | 1435 | ccgtaatcaaccctatatcgatg | 431359 | - | see also 10201 line |
| 51421 | LZIC | NM_032368.3 | 355 | atgactttggtagatgaactaag | 400009 | - | see also 22768 line |
| 51422 | LZIC | NM_032368.3 | 627 | tgctatactcagccagtttgaga | 400014 | - | see also 22768 line |
| 51423 | LZK1 | NM_024835.1 | 717 | ctccgagcatacaatatccttat | 391307 | - | see also 8772 line |
| 51424 | LZK1 | NM_024835.1 | 1042 | ttgatgctttacgcaagagtttt | 391315 | - | see also 8772 line |
| 51425 | LZTFL1 | NM_020347.1 | 177 | tgcgttttgctcgttcaaagaga | 376777 | - | see also 22770 line |
| 51426 | LZTFL1 | NM_020347.1 | 847 | gacagccaagcacgatctactca | 376793 | - | see also 22770 line |
| 51427 | LZTR1 | NM_006767.1 | 1186 | ctcggacgccatgtacatcttcg | 336309 | - | see also 4772 line |
| 51428 | LZTR1 | NM_006767.1 | 1059 | agcgggttggcttcaagaagtcc | 336307 | - | see also 4772 line |
| 51429 | M-RIP | NM_015134.1 | 2996 | cacggtgtccggatatgatataa | 348564 | - | see also 22772 line |
| 51430 | M-RIP | NM_015134.1 | 2994 | gccacggtgtccggatatgatat | 348563 | - | see also 22772 line |
| 51431 | M-RIP | NM_015134.1 | 1353 | gacttgtccgcatgttacgatgt | 348552 | - | see also 22772 line |
| 51432 | M17S2 | NM_005899.2 | 169 | ctgatatcgaagctatggtaaaa | 334440 | - | see also 4109 line |
| 51433 | M17S2 | NM_005899.2 | 1499 | ctggtgcagtatcatagtagatc | 334469 | - | see also 4109 line |
| 51434 | MAB21L1 | NM_005584.2 | 1474 | tactttctaccgaacttagatct | 334109 | - | see also 22774 line |
| 51435 | MAB21L1 | NM_005584.2 | 1349 | tggtttcctacgagtgtgaaaag | 334105 | - | see also 22774 line |
| 51436 | MAD2L1BP | NM_014628.1 | 286 | aagcacttttaccgaaaaccttc | 341433 | - | see also 22775 line |
| 51437 | MAD2L1BP | NM_014628.1 | 82 | cacgcctcccagatagaactact | 341427 | - | see also 22775 line |
| 51438 | MAD2L1BP | NM_014628.1 | 36 | agccgcagtccctgatttggagt | 341426 | - | see also 22775 line |
| 51439 | MAF1 | NM_032272.2 | 983 | ctcaagcgaatcgtcttctttag | 399306 | - | see also 9132 line |
| 51440 | MAF1 | NM_032272.2 | 987 | agcgaatcgtcttctttagctgc | 399307 | - | see also 9132 line |
| 51441 | MAGEA10 | NM_021048.3 | 996 | ctcatacttatcctaagcataat | 380495 | - | see also 22777 line |
| 51442 | MAGEA10 | NM_021048.3 | 501 | tcctgctatcctctaataccaag | 380489 | - | see also 22777 line |
| 51443 | MAGEA10 | NM_021048.3 | 1187 | tcctgcacggtatgagtttctgt | 380496 | - | see also 22777 line |
| 51444 | MAGEA2 | NM_005361.2 | 1336 | tcctgcaccatacactaaagatc | 333658 | - | - | - | melanoma |
| 51445 | MAGEA8 | NM_005364.3 | 834 | atcgtcctgggcatgatcttaat | 333663 | - | see also 22778 line |
| 51446 | MAGEA8 | NM_005364.3 | 276 | gaggcaccagggcttatggatgt | 333659 | - | see also 22778 line |
| 51447 | MAGEB1 | NM_002363.3 | 1264 | cccaagtccgatccagtgttaga | 330880 | - | see also 22779 line |
| 51448 | MAGEB1 | NM_002363.3 | 845 | caccctcgtcagtaagctaaacc | 330877 | - | see also 22779 line |
| 51449 | MAGEB1 | NM_002363.3 | 669 | cacttcattctacgtaagtataa | 330875 | - | see also 22779 line |
| 51450 | MAGEB2 | NM_002364.3 | 1001 | tgccttcccaacccattacgaag | 330885 | - | see also 22780 line |
| 51451 | MAGEB2 | NM_002364.3 | 613 | accccaacggccacacttacacc | 330884 | - | see also 22780 line |

EP 1 752 536 A1

Figure 46

| 51452 | MAGEB3 | NM_002365.2 | 840 | accgacaagtgcccaacagtaat | 330896 | - | see also 22781 line |
|---|---|---|---|---|---|---|---|
| 51453 | MAGEB3 | NM_002365.2 | 271 | caccaactacatctgagatgtt | 330888 | - | see also 22781 line |
| 51454 | MAGEB3 | NM_002365.2 | 807 | cccaagatttggtgaagcttaaa | 330894 | - | see also 22781 line |
| 51455 | MAGEB4 | NM_002367.2 | 307 | atgtcatgcactggatctgataa | 330898 | - | see also 22782 line |
| 51456 | MAGEB4 | NM_002367.2 | 330 | aggcgacgagagccaagatgaag | 330899 | - | see also 22782 line |
| 51457 | MAGEB6 | NM_173523.2 | 863 | cagaacctcccaacatttggtgg | 425439 | - | see also 22783 line |
| 51458 | MAGEB6 | NM_173523.2 | 953 | aggcctcccagtgaaggaattc | 425440 | - | see also 22783 line |
| 51459 | MAGEC1 | NM_005462.2 | 3453 | accgggaggtgcccaactctct | 333851 | - | see also 22784 line |
| 51460 | MAGEC1 | NM_005462.2 | 3567 | ccgtccctattaccttccatcc | 333854 | - | see also 22784 line |
| 51461 | MAGEC3 | NM_138702.1 | 1445 | tgctgacgactgtcatcaagaag | 408619 | - | see also 22785 line |
| 51462 | MAGEC3 | NM_138702.1 | 1462 | aagaagtataaggactattttcc | 408620 | - | see also 22785 line |
| 51463 | MAGEC3 | NM_138702.1 | 1551 | cccgacaaccactcctatttct | 408623 | - | see also 22785 line |
| 51464 | MAGED1 | NM_006986.2 | 854 | ctggagtcccggacaataattcg | 336684 | - | see also 22786 line |
| 51465 | MAGED1 | NM_006986.2 | 886 | gacccgcaagattaataacttga | 336687 | - | see also 22786 line |
| 51466 | MAGED2 | NM_014599.4 | 1530 | ggcgatggaagcggatttgaagg | 340986 | - | see also 5768 line |
| 51467 | MAGED2 | NM_014599.4 | 1297 | ctgcgccctggatacatcattc | 340982 | - | see also 5768 line |
| 51468 | MAGED4 | NM_030801.2 | 367 | ggccttcgcatcttatcgaatga | 395191 | - | see also 22788 line |
| 51469 | MAGED4 | NM_030801.2 | 366 | aggccttcgcatcttatcgaatg | 395190 | - | see also 22788 line |
| 51470 | MAGEE1 | NM_016249.2 | 369 | tccattccgaacgttgacaacg | 356608 | - | see also 22789 line |
| 51471 | MAGEE1 | NM_016249.2 | 396 | cccgacctcagttgagttagaag | 356610 | - | see also 22789 line |
| 51472 | MAGEE1 | NM_016249.2 | 354 | tcccgttccaggcgttccattcc | 356607 | - | see also 22789 line |
| 51473 | MAGEF1 | NM_022149.3 | 671 | ggccccagattgggtctgttaat | 382534 | - | see also 22790 line |
| 51474 | MAGEF1 | NM_022149.3 | 699 | tcctgggccttatctatatgaga | 382538 | - | see also 22790 line |
| 51475 | MAGEH1 | NM_014061.3 | 692 | tgcgcagaggtaacctgatttat | 339495 | - | see also 22791 line |
| 51476 | MAGEH1 | NM_014061.3 | 549 | gtctatattagcgctcatcttca | 339493 | - | see also 22791 line |
| 51477 | MAIL | NM_031419.1 | 383 | aaggtgttcgggtaaagaactca | 396859 | - | see also 22792 line |
| 51478 | MAIL | NM_031419.1 | 2024 | tgcagtatcggttgacacaatta | 396889 | - | see also 22792 line |
| 51479 | MAMDC1 | NM_182830.2 | 734 | agcatcaacgtcaattaaactct | 435299 | - | see also 22793 line |
| 51480 | MAMDC1 | NM_182830.2 | 1765 | tacggacgggtcaatttgactct | 435342 | - | see also 22793 line |
| 51481 | MAML1 | NM_014757.2 | 571 | cggccacgcatcttcatgataca | 343126 | - | see also 22794 line |
| 51482 | MAML1 | NM_014757.2 | 1744 | caccgagtaacttgaatcagaac | 343148 | - | see also 22794 line |
| 51483 | MAML3 | NM_018717.2 | 838 | ccgggatcggtgtgaataataact | 372838 | - | see also 22795 line |
| 51484 | MAML3 | NM_018717.2 | 778 | ctgccgcgaattggcagtagtatt | 372833 | - | see also 22795 line |
| 51485 | MAN1A1 | NM_005907.2 | 2066 | tacatcttacggccagaagttat | 334524 | - | see also 4122 line |
| 51486 | MAN1A1 | NM_005907.2 | 1795 | gtctagcagcggactaacttata | 334520 | - | see also 4122 line |
| 51487 | MANEA | NM_024641.1 | 800 | aacactcggaacgaatcaatgg | 388450 | - | see also 22797 line |
| 51488 | MANEA | NM_024641.1 | 810 | accgaatcaattgggaagtattat | 388452 | - | see also 22797 line |

Figure 46

| | | | | | | | |
|---|---|---|---|---|---|---|---|
| 51489 | MAP1LC3A | NM_032514.2 | 430 | cacgcccatcgcggacatctacg | 400511 | - | see also 22798 line |
| 51490 | MAP4K4 | NM_004834.2 | 2554 | cacccccggagattcgtaaataca | 333077 | - | see also 3349 line |
| 51491 | MAP4K4 | NM_004834.2 | 2723 | gacgtacttgagggcttgaatgt | 333085 | - | see also 3349 line |
| 51492 | MBD3L1 | NM_145208.1 | 542 | tgcggatggactcgctaatgagg | 413771 | - | see also 22800 line |
| 51493 | MBD3L1 | NM_145208.1 | 49 | aacgtgactgtgtaaaccaatgc | 413766 | - | see also 22800 line |
| 51494 | MBD6 | NM_052897.1 | 1361 | gaccccctactgtatttcgattg | 404756 | - | see also 22801 line |
| 51495 | MBD6 | NM_052897.1 | 1362 | accccctactgtatttcgattgc | 404757 | - | see also 22801 line |
| 51496 | MBD6 | NM_052897.1 | 3131 | accctacccggaacagcaatagc | 404770 | - | see also 22801 line |
| 51497 | MBTD1 | NM_017643.1 | 1209 | agccacagtaactcgaattattc | 359975 | - | see also 7066 line |
| 51498 | MBTD1 | NM_017643.1 | 1186 | atggagccacgtttaatatgtgt | 359972 | - | see also 7066 line |
| 51499 | MCC2 | NM_031941.2 | 1414 | agcgccgttctctaatgaagatt | 397429 | - | see also 22803 line |
| 51500 | MCC2 | NM_031941.2 | 1029 | aagctcaaatgctttaatcgtct | 397427 | - | see also 22803 line |
| 51501 | MCLC | NM_015127.2 | 815 | tactagtcaaccctatttggttg | 348515 | - | see also 6257 line |
| 51502 | MCLC | NM_015127.2 | 1186 | ggcccttatgccaaaacgtatga | 348526 | - | see also 6257 line |
| 51503 | MCM10 | NM_018518.3 | 246 | gacggcgacggtgaatcttatac | 372061 | - | see also 22805 line |
| 51504 | MCM10 | NM_018518.3 | 249 | ggcgacggtgaatcttatacaga | 372063 | - | see also 22805 line |
| 51505 | MCM3APAS | NM_018118.1 | 790 | gacacatatgctcacaatgatgg | 366859 | - | see also 22806 line |
| 51506 | MCM3APAS | NM_018118.1 | 699 | tagggataataagtaacagtacc | 366855 | - | see also 22806 line |
| 51507 | MDAC1 | NM_139172.1 | 69 | ctcggcagacggaaatggaatcc | 409418 | - | - | - | - |
| 51508 | MDS006 | NM_020233.3 | 91 | ggcgtcatcgcagatgttcaatt | 376344 | - | see also 22807 line |
| 51509 | MDS006 | NM_020233.3 | 154 | cggcgatactacagacatagtct | 376347 | - | see also 22807 line |
| 51510 | MDS009 | NM_020234.3 | 581 | tacacgtatccgtgtattccaga | 376381 | - | see also 22808 line |
| 51511 | MDS009 | NM_020234.3 | 993 | tagactaccatactgatatatta | 376396 | - | see also 22808 line |
| 51512 | MDS010 | NM_020231.3 | 455 | atcaatgtacgagattatcctca | 376320 | - | see also 7808 line |
| 51513 | MDS010 | NM_020231.3 | 452 | gtgatcaatgtacgagattatcc | 376319 | - | see also 7808 line |
| 51514 | MDS018 | NM_021823.2 | 207 | ctcttctggtgtcaaagcttttg | 381068 | - | - | - | - |
| 51515 | MDS025 | NM_021825.3 | 406 | gaggagatcgttgggtttcaagg | 381102 | - | see also 8178 line |
| 51516 | MDS025 | NM_021825.3 | 454 | tgcggagagagttcttcactacc | 381104 | - | see also 8178 line |
| 51517 | MDS027 | NM_018462.3 | 126 | ctcaactcgttcgatatgtcttg | 371871 | - | see also 7487 line |
| 51518 | MDS027 | NM_018462.3 | 73 | accgggagtacattgagataatc | 371868 | - | see also 7487 line |
| 51519 | MDS029 | NM_018464.1 | 326 | gccgttgttggaggtccaaaaag | 371884 | - | see also 22812 line |
| 51520 | MDS029 | NM_018464.1 | 147 | atggatcgcagcagttaccattg | 371880 | - | see also 22812 line |
| 51521 | MDS031 | NM_018466.2 | 185 | ttcagtactgagtcgtttactct | 371900 | - | see also 22813 line |
| 51522 | MDS031 | NM_018466.2 | 314 | aagccactcgtagtggttataaa | 371904 | - | see also 22813 line |

Figure 46

| 51523 | MDS032 | NM_018467.1 | 37 | ggcgtcgaggctggagctaaacc | 371915 | - | see also 22814 line |
|---|---|---|---|---|---|---|---|
| 51524 | MDS032 | NM_018467.1 | 786 | ttcgaatcatgcctaaactcaaa | 371921 | - | see also 22814 line |
| 51525 | MDS2 | NM_148895.1 | 643 | gagggttaattggagttttatca | 415160 | - | see also 22815 line |
| 51526 | MDS2 | NM_148895.1 | 645 | gggttaattggagttttatcaag | 415161 | - | see also 22815 line |
| 51527 | MDS2 | NM_148895.1 | 589 | tgctccaggctgcggattttatt | 415159 | - | see also 22815 line |
| 51528 | MECT1 | NM_015321.1 | 29 | ttcgaacaatccgcggaaattca | 351496 | - | see also 22816 line |
| 51529 | MECT1 | NM_015321.1 | 183 | cccagccgaggccagtactatgg | 351498 | - | see also 22816 line |
| 51530 | MED8 | NM_052877.1 | 489 | ggccgaacaagcagacctttaac | 404583 | - | receptor_acitivity、tra nscription_regulator |- |- |
| 51531 | MED8 | NM_052877.1 | 270 | aaggacgggtgcctgttttcagc | 404579 | - | see also 51530 line |
| 51532 | MED8 | NM_052877.1 | 490 | gccgaacaagcagacctttaacc | 404584 | - | see also 51530 line |
| 51533 | MEP50 | NM_024102.2 | 597 | cagcgaggacaatagaattttac | 386037 | - | see also 22817 line |
| 51534 | MEP50 | NM_024102.2 | 948 | gacttggtccccgctcaatcact | 386040 | - | see also 22817 line |
| 51535 | MEP50 | NM_024102.2 | 506 | agcaggtggtactgagttcatac | 386036 | - | see also 22817 line |
| 51536 | MESDC1 | NM_022566.1 | 1698 | taccgccagtgaattccaattct | 383580 | - | see also 22818 line |
| 51537 | MESDC1 | NM_022566.1 | 1645 | aggctgcaccctgctatctcagg | 383577 | - | see also 22818 line |
| 51538 | MFN2 | NM_014874.1 | 1509 | agcggcaagaccgactgaaattt | 345077 | - | see also 6074 line |
| 51539 | MGC10084 | NM_153702.1 | 117 | ttctatgggcactttttttcgatt | 423677 | - | see also 10010 line |
| 51540 | MGC10084 | NM_153702.1 | 270 | cagaaggcgacacatgttgttca | 423683 | - | see also 10010 line |
| 51541 | MGC10120 | NM_173809.1 | 287 | agcttacaagttggatgcatatt | 428202 | - | see also 22821 line |
| 51542 | MGC10120 | NM_173809.1 | 95 | ggccaccagtgaagactataagc | 428197 | - | see also 22821 line |
| 51543 | MGC10198 | NM_152682.1 | 481 | aaccgctatgacctatacattgt | 419737 | - | see also 22822 line |
| 51544 | MGC10198 | NM_152682.1 | 238 | agcattacgctctatttatgaag | 419729 | - | see also 22822 line |
| 51545 | MGC10200 | NM_145060.1 | 633 | tcccgcttaacctataatcaaat | 413350 | - | see also 22823 line |
| 51546 | MGC10200 | NM_145060.1 | 632 | atcccgcttaacctataatcaaa | 413349 | - | see also 22823 line |
| 51547 | MGC10233 | NM_152715.1 | 727 | ctcgtcctggccaacaatcattt | 420022 | - | see also 22824 line |
| 51548 | MGC10233 | NM_152715.1 | 971 | aacttaatggcagcgttgttact | 420034 | - | see also 22824 line |
| 51549 | MGC10233 | NM_152715.1 | 689 | tacgaaagttaggagttatgttt | 420020 | - | see also 22824 line |

Figure 46

| 51550 | MGC10540 | NM_032353.2 | 256 | gtcgatccagattgtattagagg | 399919 | - | see also 22825 line | | |
| 51551 | MGC10540 | NM_032353.2 | 297 | acctcgagtggttggataagagc | 399920 | - | see also 22825 line | | |
| 51552 | MGC10540 | NM_032353.2 | 251 | gtggagtcgatccagattgtatt | 399917 | - | see also 22825 line | | |
| 51553 | MGC10744 | NM_032354.2 | 450 | tgcactacgtattggtacatttt | 399928 | - | see also 22826 line | | |
| 51554 | MGC10744 | NM_032354.2 | 169 | tggtggtcgtcatcaccttattc | 399926 | - | see also 22826 line | | |
| 51555 | MGC10765 | NM_024345.1 | 441 | gccccgatggacgaatgatttct | 386354 | - | see also 8613 line | | |
| 51556 | MGC10765 | NM_024345.1 | 440 | agccccgatggacgaatgatttc | 386353 | - | see also 8613 line | | |
| 51557 | MGC10812 | NM_031425.1 | 204 | cccgggtggacgctgaacatacc | 396929 | - | - | - | - |
| 51558 | MGC10812 | NM_031425.1 | 203 | tcccgggtggacgctgaacatac | 396928 | - | see also 51557 line | | |
| 51559 | MGC10870 | NM_032301.1 | 1307 | tgcacccgaaggcatgacaatgg | 399558 | - | see also 22828 line | | |
| 51560 | MGC10870 | NM_032301.1 | 1317 | ggcatgacaatgggctcaatagg | 399559 | - | see also 22828 line | | |
| 51561 | MGC10911 | NM_032302.1 | 408 | aagcctgtgctcaccacaaaagt | 399561 | - | see also 22829 line | | |
| 51562 | MGC10911 | NM_032302.1 | 451 | ctctcatccatgtctttgcaaag | 399562 | - | see also 22829 line | | |
| 51563 | MGC10946 | NM_030572.1 | 294 | cacagggtcgccgcttcatctcc | 394686 | - | - | - | - |
| 51564 | MGC10946 | NM_030572.1 | 368 | agcccaaatccccaactactaac | 394687 | - | see also 51563 line | | |
| 51565 | MGC10946 | NM_030572.1 | 369 | gcccaaatccccaactactaact | 394688 | - | see also 51563 line | | |
| 51566 | MGC10955 | NM_032676.2 | 229 | gcgagccagctgctgaaaacagg | 401211 | - | see also 22830 line | | |
| 51567 | MGC10992 | NM_033212.2 | 1033 | ctggcgttgagcaggaacattga | 403595 | - | see also 22831 line | | |
| 51568 | MGC10992 | NM_033212.2 | 1035 | ggcgttgagcaggaacattgaga | 403596 | - | see also 22831 line | | |
| 51569 | MGC10993 | NM_030577.1 | 475 | aaccatcccgtggtcatacatgg | 394693 | - | see also 22832 line | | |

Figure 46

| 51570 | MGC10993 | NM_030577.1 | 455 | gagacctagtgatcaacactaac | 394692 | - | see also 22832 line | | |
|---|---|---|---|---|---|---|---|---|---|
| 51571 | MGC11061 | NM_032312.2 | 570 | tccatgatatcattatatggaca | 399656 | - | see also 9153 line | | |
| 51572 | MGC11061 | NM_032312.2 | 691 | aagtccttggagttataggatat | 399667 | - | see also 9153 line | | |
| 51573 | MGC11082 | NM_032691.1 | 895 | cactccctatggctttgcaaaat | 401231 | - | see also 22834 line | | |
| 51574 | MGC11082 | NM_032691.1 | 852 | aacacctattgttcaaaatcttc | 401230 | - | see also 22834 line | | |
| 51575 | MGC11115 | NM_032310.2 | 699 | agcatggggcatcttatacgtcc | 399648 | - | see also 22835 line | | |
| 51576 | MGC11115 | NM_032310.2 | 351 | gcgggactgccaggagttctacc | 399644 | - | see also 22835 line | | |
| 51577 | MGC11242 | NM_024320.2 | 252 | aggccccaacagcgactttaaca | 386253 | - | see also 22836 line | | |
| 51578 | MGC11242 | NM_024320.2 | 384 | ccctaacctctttgatgatcacg | 386256 | - | see also 22836 line | | |
| 51579 | MGC11257 | NM_032350.3 | 1218 | ctgcagatgggcccaaaagcaca | 399870 | - | - | - | - |
| 51580 | MGC11266 | NM_024322.1 | 876 | gacgtcaccgtgacatgtcaagg | 386274 | - | see also 22837 line | | |
| 51581 | MGC11266 | NM_024322.1 | 959 | tacgaagcccttgcatcaagtgt | 386278 | - | see also 22837 line | | |
| 51582 | MGC1203 | NM_024296.2 | 346 | gaccgaggtgactgatgtctatg | 386202 | - | see also 22838 line | | |
| 51583 | MGC1203 | NM_024296.2 | 347 | accgaggtgactgatgtctatga | 386203 | - | see also 22838 line | | |
| 51584 | MGC1203 | NM_024296.2 | 712 | agccctccaggagtattcacaca | 386208 | - | see also 22838 line | | |
| 51585 | MGC12197 | NM_016625.2 | 501 | tagtcggtctcgggatagagaac | 358577 | - | see also 6961 line | | |
| 51586 | MGC12197 | NM_016625.2 | 112 | atgggacgtcggtcatcagatac | 358561 | - | see also 6961 line | | |
| 51587 | MGC12909 | NM_145055.1 | 180 | aagatggtgtagcggattctaca | 413306 | - | see also 9769 line | | |
| 51588 | MGC12965 | NM_198519.1 | 1195 | ttgatcgtgtattgaaagagtgg | 438954 | - | see also 22842 line | | |
| 51589 | MGC12965 | NM_198519.1 | 1617 | ttgtccaatggtgaagtaacaaa | 438960 | - | see also 22842 line | | |
| 51590 | MGC12966 | NM_032706.1 | 376 | gtccgcctgcaggttcatattga | 401245 | - | see also 22843 line | | |

Figure 46

| | | | | | | | |
|---|---|---|---|---|---|---|---|
| 51591 | MGC12972 | NM_032683.1 | 423 | tgctgcgcagttcgtgaacttcc | 401218 | - | see also 22844 line |
| 51592 | MGC12972 | NM_032683.1 | 110 | gcgcgctgctcgtcactaacacg | 401213 | - | see also 22844 line |
| 51593 | MGC12981 | NM_032357.2 | 587 | ccccctgaactggtttggaatcc | 399933 | - | see also 22845 line |
| 51594 | MGC12981 | NM_032357.2 | 586 | accccctgaactggtttggaatc | 399932 | - | see also 22845 line |
| 51595 | MGC12992 | NM_032342.1 | 1465 | gccgaacctcgtgaaacacatcg | 399839 | - | see also 22846 line |
| 51596 | MGC12992 | NM_032342.1 | 1464 | agccgaacctcgtgaaacacatc | 399838 | - | see also 22846 line |
| 51597 | MGC12992 | NM_032342.1 | 453 | accgacttctacactcttacttc | 399825 | - | see also 22846 line |
| 51598 | MGC13010 | NM_032687.2 | 703 | gtcttcacggccaacacttatgg | 401222 | - | see also 22847 line |
| 51599 | MGC13010 | NM_032687.2 | 527 | ttccccttctggccttcgatttc | 401219 | - | see also 22847 line |
| 51600 | MGC13016 | NM_032343.1 | 484 | gacgccgtgacaccttctacaag | 399845 | - | see also 22848 line |
| 51601 | MGC13016 | NM_032343.1 | 320 | tgctgctatccaggataagctct | 399844 | - | see also 22848 line |
| 51602 | MGC13024 | NM_152288.1 | 954 | acccgtggggctcgtgtttgtgg | 415378 | - | see also 22849 line |
| 51603 | MGC13024 | NM_152288.1 | 802 | tcccttagtccagcttccaatct | 415377 | - | see also 22849 line |
| 51604 | MGC13034 | NM_153217.1 | 679 | cacaggcccaatcctgatgttga | 422221 | - | see also 22850 line |
| 51605 | MGC13034 | NM_153217.1 | 750 | tcctccccttatgaagaaatat | 422224 | - | see also 22850 line |
| 51606 | MGC13034 | NM_153217.1 | 749 | ctcctcccccttatgaagaaata | 422223 | - | see also 22850 line |
| 51607 | MGC13040 | NM_032930.1 | 407 | atcggaaggatgatttcgttatg | 402527 | - | see also 22851 line |
| 51608 | MGC13040 | NM_032930.1 | 402 | gtcctatcggaaggatgatttcg | 402526 | - | see also 22851 line |
| 51609 | MGC13045 | NM_032344.1 | 685 | ctggtggtacatgaactcttttc | 399847 | - | see also 22852 line |
| 51610 | MGC13045 | NM_032344.1 | 686 | tggtggtacatgaactcttttcc | 399848 | - | see also 22852 line |

Figure 46

| 51611 | MGC13057 | NM_032321.1 | 529 | gcctgtctcaggatatcttgtgt | 399707 | - | see also 22854 line |
|-------|----------|-------------|-----|-------------------------|--------|---|---------------------|
| 51612 | MGC13057 | NM_032321.1 | 568 | gggcatgcaataacatcaagtac | 399712 | - | see also 22854 line |
| 51613 | MGC13102 | NM_032323.1 | 1121 | tgccgtctcccggctgatctact | 399738 | - | see also 22855 line |
| 51614 | MGC13102 | NM_032323.1 | 581 | gccccaagaagaccttatcgtgc | 399727 | - | see also 22855 line |
| 51615 | MGC13125 | NM_032725.2 | 114 | cggtcgcaagcgtcgcaaaaagc | 401260 | - | see also 22856 line |
| 51616 | MGC13125 | NM_032725.2 | 385 | cacgatacccggattcatctcc | 401265 | - | see also 22856 line |
| 51617 | MGC13159 | NM_032927.2 | 583 | atgccgccatttgggtacaaaac | 402510 | - | see also 22857 line |
| 51618 | MGC13159 | NM_032927.2 | 850 | gtcaagtatccagccttgatacc | 402520 | - | see also 22857 line |
| 51619 | MGC13159 | NM_032927.2 | 685 | tccattgttgtgacctattatgt | 402514 | - | see also 22857 line |
| 51620 | MGC13183 | NM_032358.2 | 1252 | atggggaagcgtttacagataat | 399955 | - | see also 22858 line |
| 51621 | MGC13183 | NM_032358.2 | 1108 | aagtccctaaaagataagttagt | 399951 | - | see also 22858 line |
| 51622 | MGC13198 | NM_032690.1 | 477 | gtgttcaggcccagcgtttttcc | 401223 | - | - |
| 51623 | MGC13198 | NM_032690.1 | 721 | gtcggcagccctctcacattttg | 401224 | - | see also 51622 line |
| 51624 | MGC13198 | NM_032690.1 | 724 | ggcagccctctcacattttgagg | 401225 | - | see also 51622 line |
| 51625 | MGC13204 | NM_031465.1 | 741 | cccgaggacaagtatggaataaa | 397099 | - | see also 22859 line |
| 51626 | MGC13204 | NM_031465.1 | 373 | gacactcaagtcgaaaacctacc | 397087 | - | see also 22859 line |
| 51627 | MGC13251 | NM_032714.1 | 713 | caccctgcttagcgtgatcaacg | 401249 | - | see also 22860 line |
| 51628 | MGC13251 | NM_032714.1 | 772 | cccagctgcggaacgagtttatc | 401250 | - | see also 22860 line |
| 51629 | MGC14126 | NM_032898.2 | 465 | atgccagttcgaaactttcaaa | 402462 | - | see also 22861 line |
| 51630 | MGC14126 | NM_032898.2 | 467 | gccagttcgaaacttttcaaagt | 402463 | - | see also 22861 line |

Figure 46

| | | | | | | | |
|---|---|---|---|---|---|---|---|
| 51631 | MGC14128 | NM_032899.3 | 952 | gacagtcacctcaagaacatttc | 402473 | - | see also 9341 line |
| 51632 | MGC14128 | NM_032899.3 | 694 | aagccctacctgaaggaaaaatc | 402469 | - | see also 9341 line |
| 51633 | MGC14128 | NM_032899.3 | 1051 | atcatctcggactggagatttgt | 402475 | - | see also 9341 line |
| 51634 | MGC14156 | NM_032906.2 | 263 | aagaagccgtcagatatgaagc | 402495 | - | see also 9344 line |
| 51635 | MGC14161 | NM_032892.1 | 1510 | gccggagcgtccctccataacc | 402450 | - | see also 9938 line |
| 51636 | MGC14276 | NM_153248.2 | 318 | ggctctggttgatgagttcttca | 422594 | - | - |
| 51637 | MGC14288 | NM_032901.2 | 222 | ttggctataagaccttctctacc | 402491 | - | see also 22865 line |
| 51638 | MGC14288 | NM_032901.2 | 289 | ccgagtctaccactatttccagt | 402493 | - | see also 22865 line |
| 51639 | MGC14289 | NM_080660.2 | 783 | accttcgactgagcattcacaag | 406580 | - | see also 22866 line |
| 51640 | MGC14289 | NM_080660.2 | 487 | cagaatgacccgtcttttacc | 406574 | - | see also 22866 line |
| 51641 | MGC14289 | NM_080660.2 | 441 | tgggactttttggtctcaatgaaa | 406573 | - | see also 22866 line |
| 51642 | MGC14327 | NM_053045.1 | 469 | tccgcctttctgggtacttacg | 405848 | - | - |
| 51643 | MGC14327 | NM_053045.1 | 488 | tacggtcctgagtctcaagttcg | 405849 | - | see also 51642 line |
| 51644 | MGC14327 | NM_053045.1 | 556 | agctctgttcggcctcattacg | 405850 | - | see also 51642 line |
| 51645 | MGC14407 | NM_032908.1 | 431 | ggcatgcacacgatgttatgaga | 402501 | - | see also 22867 line |
| 51646 | MGC14407 | NM_032908.1 | 471 | agccatcagactggtcaatcacg | 402505 | - | see also 22867 line |
| 51647 | MGC14595 | NM_032334.1 | 645 | cagggtttagttgaaaaacactga | 399783 | - | see also 22868 line |
| 51648 | MGC14595 | NM_032334.1 | 499 | ctggagttcctccatgtttatt | 399781 | - | see also 22868 line |
| 51649 | MGC14697 | NM_032747.1 | 264 | atggcaggtccagaaagttgatgc | 401316 | - | see also 22869 line |
| 51650 | MGC14697 | NM_032747.1 | 331 | ctctcacaggtagaatgaactgt | 401317 | - | see also 22869 line |

Figure 46

| 51651 | MGC14799 | NM_032336.1 | 628 | aagcacatgccccctaacttaca | 399796 | - | see also 9166 line |
|---|---|---|---|---|---|---|---|
| 51652 | MGC14801 | NM_032705.2 | 234 | caccgatcccgacatagatcagt | 401242 | - | see also 22871 line |
| 51653 | MGC14801 | NM_032705.2 | 266 | ttcttcagatgatcctatgtttc | 401243 | - | see also 22871 line |
| 51654 | MGC14816 | NM_144620.1 | 741 | ttgatctgagtatgaacgatttt | 410392 | - | see also 22872 line |
| 51655 | MGC14816 | NM_144620.1 | 874 | tacgttatggctgcaacgaaatg | 410398 | - | see also 22872 line |
| 51656 | MGC14817 | NM_032338.2 | 157 | aactagacggtgatgttttaatg | 399804 | - | see also 22873 line |
| 51657 | MGC14817 | NM_032338.2 | 160 | tagacggtgatgttttaatgaaa | 399805 | - | see also 22873 line |
| 51658 | MGC14839 | NM_080659.1 | 213 | tacgtatgaacgggtgttacagc | 406569 | - | - | - | - |
| 51659 | MGC14839 | NM_080659.1 | 270 | gtcggaagacagcaacttacatt | 406570 | - | see also 51658 line |
| 51660 | MGC14839 | NM_080659.1 | 184 | ctcccaaagggccatgaaacaac | 406567 | - | see also 51658 line |
| 51661 | MGC15396 | NM_052855.2 | 420 | aggtggtctcttacctgttaaaa | 404441 | - | see also 22874 line |
| 51662 | MGC15396 | NM_052855.2 | 371 | gtcaacggctggacttgtttaca | 404438 | - | see also 22874 line |
| 51663 | MGC15396 | NM_052855.2 | 395 | tgggcatgtaaacgaaaccatgg | 404440 | - | see also 22874 line |
| 51664 | MGC15397 | NM_080652.2 | 624 | atcggtttgatcccatataattt | 406522 | - | see also 22875 line |
| 51665 | MGC15397 | NM_080652.2 | 626 | cggtttgatcccatataatttca | 406524 | - | see also 22875 line |
| 51666 | MGC15407 | NM_080667.2 | 478 | ctgcaagccattcgaataattca | 406599 | - | see also 22876 line |
| 51667 | MGC15407 | NM_080667.2 | 371 | ctcttgcaaagacccatacatca | 406595 | - | see also 22876 line |
| 51668 | MGC15416 | NM_032371.2 | 143 | ggctcggcgagctcctgaaatgc | 400018 | - | - | - | - |
| 51669 | MGC15419 | NM_024735.2 | 564 | cacgtcgatgaccctatgagatt | 389745 | - | see also 22877 line |
| 51670 | MGC15419 | NM_024735.2 | 1095 | gagaaccagcgcaacttcaatga | 389751 | - | see also 22877 line |

Figure 46

| 51671 | MGC15476 | NM_145056.1 | 1583 | agcttcccacgcgctcaagaaaa | 413336 | - | - | | - | - |
|---|---|---|---|---|---|---|---|---|---|---|
| 51672 | MGC15476 | NM_145056.1 | 1289 | tccccagcgtcgtctgctttacg | 413335 | - | see also 51671 line | | | |
| 51673 | MGC15476 | NM_145056.1 | 1590 | cacgcgctcaagaaaaagatact | 413338 | - | see also 51671 line | | | |
| 51674 | MGC15606 | NM_145037.1 | 1719 | cagatttagtggagtttaacttg | 413093 | - | see also 22878 line | | | |
| 51675 | MGC15606 | NM_145037.1 | 2131 | ctcaggggttggagtatatctcg | 413105 | - | see also 22878 line | | | |
| 51676 | MGC15634 | NM_032755.1 | 330 | tccatgtctcaaaacattagtcc | 401319 | - | - | | - | - |
| 51677 | MGC15634 | NM_032755.1 | 362 | ccctatgaggtttatcttctttt | 401320 | - | see also 51676 line | | | |
| 51678 | MGC15668 | NM_032756.1 | 1022 | gggctgtatacgcctaacattgt | 401327 | - | see also 22879 line | | | |
| 51679 | MGC15668 | NM_032756.1 | 388 | cagcggcgacgcggtctttttgg | 401321 | - | see also 22879 line | | | |
| 51680 | MGC15677 | NM_032878.2 | 202 | ttgcttcgacaggttttttaatgc | 402393 | - | see also 22880 line | | | |
| 51681 | MGC15677 | NM_032878.2 | 147 | accacctgtaatctactatgtcc | 402392 | - | see also 22880 line | | | |
| 51682 | MGC15705 | NM_032757.1 | 340 | tgcttaatcctcgtaaaaggaaa | 401335 | - | - | | - | - |
| 51683 | MGC15705 | NM_032757.1 | 336 | tgcctgcttaatcctcgtaaaag | 401333 | - | see also 51682 line | | | |
| 51684 | MGC15705 | NM_032757.1 | 326 | gccagcaggatgcctgcttaatc | 401332 | - | see also 51682 line | | | |
| 51685 | MGC15716 | NM_032370.1 | 660 | cacgcaccgctcgctcttcaagc | 400017 | - | see also 22881 line | | | |
| 51686 | MGC15716 | NM_032370.1 | 614 | aacccaatcgctacttcaagtgt | 400016 | - | see also 22881 line | | | |
| 51687 | MGC15730 | NM_032880.2 | 496 | ctggagacgccgtgactttgaag | 402396 | - | see also 9331 line | | | |
| 51688 | MGC15730 | NM_032880.2 | 497 | tggagacgccgtgactttgaagt | 402397 | - | see also 9331 line | | | |
| 51689 | MGC15737 | NM_032926.2 | 831 | tacacgatatcccatacctttaa | 402509 | - | - | | - | - |
| 51690 | MGC15827 | NM_032882.2 | 458 | ctcgaccgggaagtcaactatgc | 402415 | - | see also 22883 line | | | |

Figure 46

| | | | | | | | |
|---|---|---|---|---|---|---|---|
| 51691 | MGC15827 | NM_032882.2 | 514 | cccgtggaacgtggtcttgtgc | 402416 | - | see also 22883 line |
| 51692 | MGC15882 | NM_032884.1 | 961 | gccgagacttcgaaacaaagtgg | 402422 | - | see also 22884 line |
| 51693 | MGC15882 | NM_032884.1 | 1233 | acccgcagctgggatgttactcc | 402425 | - | see also 22884 line |
| 51694 | MGC15887 | NM_198552.1 | 343 | gtccttgctcgccaactgtaca | 439462 | - | |
| 51695 | MGC15912 | NM_032886.1 | 541 | tgcaaaagcattcgatcttatga | 402447 | - | see also 22885 line |
| 51696 | MGC15912 | NM_032886.1 | 442 | aggtcaggtgggttatcttctgc | 402444 | - | see also 22885 line |
| 51697 | MGC15937 | NM_080661.2 | 356 | cacaaacgtatatcgtatgttct | 406585 | - | see also 22886 line |
| 51698 | MGC15937 | NM_080661.2 | 878 | acgagtgatggtgcgatacatga | 406586 | - | see also 22886 line |
| 51699 | MGC16028 | NM_052873.1 | 250 | atctgaacgcatgctatcacaaa | 404567 | - | see also 22887 line |
| 51700 | MGC16028 | NM_052873.1 | 467 | tggggagatcgactgaaactcc | 404568 | - | see also 22887 line |
| 51701 | MGC16044 | NM_138371.1 | 1081 | caccgaggcacgtaaaacataact | 407772 | - | see also 22888 line |
| 51702 | MGC16044 | NM_138371.1 | 763 | ccgcgttgctactccgattacctcc | 407765 | - | see also 22888 line |
| 51703 | MGC16121 | NM_032762.2 | 484 | cccgagatgcatcgaaattgagc | 401341 | - | see also 22890 line |
| 51704 | MGC16121 | NM_032762.2 | 480 | gtctcccgagatgcatcgaatt | 401339 | - | see also 22890 line |
| 51705 | MGC16121 | NM_032762.2 | 482 | ctcccgagatgcatcgaaattga | 401340 | - | see also 22890 line |
| 51706 | MGC16142 | NM_032763.1 | 517 | ttgtggtgtcaagcagcaaaaca | 401346 | - | |
| 51707 | MGC16142 | NM_032763.1 | 471 | atcccaccaacataattcatca | 401343 | - | see also 51706 line |
| 51708 | MGC16142 | NM_032763.1 | 476 | caccaacataattcatcatgagg | 401345 | - | see also 51706 line |
| 51709 | MGC16202 | NM_032373.2 | 878 | ggcgactaagaggcgaaaacttt | 400040 | - | see also 9175 line |
| 51710 | MGC16202 | NM_032373.2 | 879 | gcgactaagaggcgaaaactttc | 400041 | - | see also 9175 line |

Figure 46

| | | | | | | | |
|---|---|---|---|---|---|---|---|
| 51711 | MGC16291 | NM_032770.2 | 404 | gactttggacgcggcctcatatag | 401347 | - | - |
| 51712 | MGC16309 | NM_033413.2 | 410 | gaccgggagggattactactat | 403817 | - | see also 22892 line |
| 51713 | MGC16309 | NM_033413.2 | 609 | ctgagaacctgatagaaacattg | 403821 | - | see also 22892 line |
| 51714 | MGC16353 | NM_138774.2 | 662 | gccgtgacacagcaatgctaga | 408733 | - | see also 22893 line |
| 51715 | MGC16353 | NM_138774.2 | 390 | tggaggtctggaacgatttcatg | 408731 | - | see also 22893 line |
| 51716 | MGC16372 | NM_145038.1 | 198 | cgcatcgctgcgcgcttagaagc | 413106 | - | see also 22894 line |
| 51717 | MGC16372 | NM_145038.1 | 1099 | atcgcctgcatgatatacttaac | 413126 | - | see also 22894 line |
| 51718 | MGC16372 | NM_145038.1 | 1732 | atgagagctcaccgtttatcttcc | 413142 | - | see also 22894 line |
| 51719 | MGC16491 | NM_052943.2 | 786 | cggtgagacgccagtttgaattc | 405462 | - | see also 22895 line |
| 51720 | MGC16491 | NM_052943.2 | 1362 | tgccagccactgtcaattactac | 405464 | - | see also 22895 line |
| 51721 | MGC16664 | NM_173509.2 | 868 | accaatccaagggctattagtac | 425230 | - | see also 10076 line |
| 51722 | MGC16664 | NM_173509.2 | 857 | gtgaggccttcaccaatccaagg | 425229 | - | see also 10076 line |
| 51723 | MGC16733 | NM_033547.2 | 404 | tacgtctcatggtgtaagaaata | 404125 | - | see also 22896 line |
| 51724 | MGC16733 | NM_033547.2 | 432 | ctgcagttacttggcaatcttgg | 404126 | - | see also 22896 line |
| 51725 | MGC16824 | NM_020314.3 | 755 | atacccgacatacttgatacatt | 376438 | - | see also 22897 line |
| 51726 | MGC16824 | NM_020314.3 | 1373 | atcgccacaaggtctatggattt | 376452 | - | see also 22897 line |
| 51727 | MGC16824 | NM_020314.3 | 446 | accgaaaagctgtctattaatct | 376431 | - | see also 22897 line |
| 51728 | MGC17330 | NM_052880.2 | 742 | cagcatgatcagaaagtatgtga | 404588 | - | see also 22898 line |
| 51729 | MGC17330 | NM_052880.2 | 461 | tgccagccttcacgcacagaaatc | 404587 | - | see also 22898 line |
| 51730 | MGC17791 | NM_152362.1 | 142 | ggctctgcaggcgcagaagaagc | 415809 | - | - |

Figure 46

| | | | | | | | | |
|---|---|---|---|---|---|---|---|---|
| 51731 | MGC17839 | NM_174926.1 | 506 | acccatggagtcctctctatagg | 428704 | - | see also 22899 line | |
| 51732 | MGC17839 | NM_174926.1 | 737 | cacaacggacatctcaaaataca | 428707 | - | see also 22899 line | |
| 51733 | MGC17943 | NM_152261.1 | 239 | aagaaatcccatcataccttaat | 415203 | - | see also 22900 line | |
| 51734 | MGC17943 | NM_152261.1 | 244 | atcccatcataccttaatgatga | 415204 | - | see also 22900 line | |
| 51735 | MGC18079 | NM_144675.1 | 551 | agcgaggaggactttcacttaga | 411271 | - | see also 22901 line | |
| 51736 | MGC18079 | NM_144675.1 | 424 | caccaagacggtcattgagttcc | 411268 | - | see also 22901 line | |
| 51737 | MGC18216 | NM_152452.1 | 2325 | cacagccaatggttttcatgatg | 416938 | - | see also 22902 line | |
| 51738 | MGC18216 | NM_152452.1 | 2269 | tggtctggctgccgtcattgtca | 416937 | - | see also 22902 line | |
| 51739 | MGC19595 | NM_033415.2 | 1312 | gacgacgtgaaagatgctattgt | 403826 | - | see also 22903 line | |
| 51740 | MGC19595 | NM_033415.2 | 850 | cacgcttgcctgaaacatgaaca | 403823 | - | see also 22903 line | |
| 51741 | MGC19604 | NM_080665.2 | 484 | agctggaaggagcggaattcacc | 406592 | - | - | - | - |
| 51742 | MGC19780 | NM_144988.1 | 561 | atctgccgtgtagaaacgttatc | 412458 | - | see also 22904 line | |
| 51743 | MGC19780 | NM_144988.1 | 566 | ccgtgtagaaacgttatccatgt | 412459 | - | see also 22904 line | |
| 51744 | MGC20255 | NM_052848.1 | 378 | gacttgacagagcatgagaaagt | 404408 | - | see also 22905 line | |
| 51745 | MGC20262 | NM_152421.2 | 490 | gcgggagctggtactgtttgaca | 416540 | - | see also 22906 line | |
| 51746 | MGC20262 | NM_152421.2 | 271 | ctgccaggtggtcatttgtgacc | 416539 | - | see also 22906 line | |
| 51747 | MGC20398 | NM_052857.1 | 921 | gtgttaccgacgggtggaaaagc | 404470 | - | see also 9437 line | |
| 51748 | MGC20398 | NM_052857.1 | 79 | accagtcggaaacggatagaatc | 404454 | - | see also 9437 line | |
| 51749 | MGC20398 | NM_052857.1 | 713 | aagcgttaccggaaggtttttt | 404465 | - | see also 9437 line | |
| 51750 | MGC20460 | NM_053043.1 | 3402 | agcgccgtcacacgtgatagaaa | 405832 | - | see also 22908 line | |

EP 1 752 536 A1

Figure 46

| | | | | | | | |
|---|---|---|---|---|---|---|---|
| 51751 | MGC20460 | NM_053043.1 | 3302 | ggctccggcataatacaacttct | 405830 | - | see also 22908 line |
| 51752 | MGC20470 | NM_145053.2 | 1064 | atctagacacgcaacaattgatg | 413239 | - | see also 22909 line |
| 51753 | MGC20470 | NM_145053.2 | 133 | gccaacgatcgtggtcatctact | 413221 | - | see also 22909 line |
| 51754 | MGC20481 | NM_052849.2 | 322 | aggatctcgggctgaaatttgt | 404411 | - | see also 9435 line |
| 51755 | MGC20486 | NM_052844.2 | 1128 | gcggcttccgctcaagtgttcc | 404406 | - | see also 22911 line |
| 51756 | MGC20486 | NM_052844.2 | 440 | tggcagagccacgcggtttgatgg | 404404 | - | see also 22911 line |
| 51757 | MGC20579 | NM_182614.2 | 337 | atcgtggacggcgtatttgcagc | 434691 | - | see also 22912 line |
| 51758 | MGC20579 | NM_182614.2 | 334 | gccatcgtggacggcgtatttgc | 434690 | - | see also 22912 line |
| 51759 | MGC20700 | NM_174983.2 | 1441 | ggcctgcgtgagctttaccact | 428891 | - | see also 22913 line |
| 51760 | MGC20781 | NM_052935.2 | 472 | accactattaccccattgagatc | 405403 | - | see also 22914 line |
| 51761 | MGC20781 | NM_052935.2 | 765 | gtgtctaactacatggattttaa | 405408 | - | see also 22914 line |
| 51762 | MGC20781 | NM_052935.2 | 412 | ttctggataatagcaagatcatc | 405401 | - | see also 22914 line |
| 51763 | MGC20806 | NM_144999.2 | 772 | gacctgcgctggaataaacgttgg | 412541 | - | see also 22915 line |
| 51764 | MGC20806 | NM_144999.2 | 1290 | agctgcagagcgggagtctaaac | 412544 | - | see also 22915 line |
| 51765 | MGC20806 | NM_144999.2 | 500 | tgctgcgctttctggacttaaag | 412539 | - | see also 22915 line |
| 51766 | MGC21416 | NM_173834.2 | 154 | ctcgcatccggagtttgacagc | 428457 | - | see also 22916 line |
| 51767 | MGC21416 | NM_173834.2 | 85 | caggcctttcagatatatccatc | 428454 | - | see also 22916 line |
| 51768 | MGC21518 | NM_145274.1 | 762 | ctcatgccggttggccaattgtca | 414086 | - | see also 22917 line |
| 51769 | MGC21518 | NM_145274.1 | 598 | ctcgttggttgctcttcaacact | 414085 | - | see also 22917 line |
| 51770 | MGC21518 | NM_145274.1 | 370 | tgcagctcattctgttagagttc | 414080 | - | see also 22917 line |

Figure 46

| | | | | | | | |
|---|---|---|---|---|---|---|---|
| 51771 | MGC21636 | NM_145032.2 | 1584 | caccgcatatctccgattgtact | 412931 | - | see also 22918 line |
| 51772 | MGC21636 | NM_145032.2 | 1932 | agcgctgccctaatttaaactac | 412953 | - | see also 22918 line |
| 51773 | MGC21644 | NM_138492.3 | 583 | tgccttacgtggacacagtatgc | 408428 | - | see also 22919 line |
| 51774 | MGC21644 | NM_138492.3 | 621 | gtctgtcttccgggaaagtatgg | 408429 | - | see also 22919 line |
| 51775 | MGC21644 | NM_138492.3 | 542 | ggctcaatcctcgggaaagaaac | 408427 | - | see also 22919 line |
| 51776 | MGC21654 | NM_145647.1 | 157 | cacgcgggacggaattatagtga | 414484 | - | see also 9790 line |
| 51777 | MGC21654 | NM_145647.1 | 1426 | tgcgtttagtaccctcatagata | 414538 | - | see also 9790 line |
| 51778 | MGC21654 | NM_145647.1 | 155 | gccacgcgggacggaattatagt | 414483 | - | see also 9790 line |
| 51779 | MGC21658 | NM_198451.1 | 1031 | ctggaagagcaccattcattaca | 437867 | - | see also 22921 line |
| 51780 | MGC21658 | NM_198451.1 | 541 | ctccctgtgaacccaatctgtgg | 437863 | - | see also 22921 line |
| 51781 | MGC21675 | NM_052861.1 | 651 | cgcgtgggacaacctagtattgg | 404479 | - | see also 22922 line |
| 51782 | MGC21675 | NM_052861.1 | 662 | acctagtattgggggaaaactca | 404480 | - | see also 22922 line |
| 51783 | MGC21688 | NM_144635.3 | 2173 | cccaggactcactctacaactcg | 410613 | - | - | | - |
| 51784 | MGC21688 | NM_144635.3 | 1848 | tccactgatgactcctatgatga | 410612 | - | see also 51783 line |
| 51785 | MGC21830 | NM_182563.2 | 775 | cggagacagcggctgatttatct | 434462 | - | see also 22923 line |
| 51786 | MGC21830 | NM_182563.2 | 779 | gacagcggctgatttatctctgc | 434464 | - | see also 22923 line |
| 51787 | MGC21854 | NM_052862.2 | 594 | agcttcaggccaatttaaccttt | 404485 | - | see also 22924 line |
| 51788 | MGC21854 | NM_052862.2 | 807 | tgcggacgaggggctcaataaaa | 404488 | - | see also 22924 line |
| 51789 | MGC22001 | NM_153238.1 | 305 | ctcgctgcgtcctaaccaaatgc | 422465 | - | - | | - |
| 51790 | MGC22001 | NM_153238.1 | 303 | tcctcgctgcgtcctaaccaaat | 422464 | - | see also 51789 line |

EP 1 752 536 A1

Figure 46

| 51791 | MGC2217 | NM_024300.2 | 323 | gccggagattctggaattctatc | 386211 | - | see also 22925 line |
| 51792 | MGC22773 | NM_145258.1 | 677 | agcatgtaaccatcgactttct | 413961 | - | see also 22926 line |
| 51793 | MGC22773 | NM_145258.1 | 463 | ttcatgacaatgggtttgcaaag | 413955 | - | see also 22926 line |
| 51794 | MGC22773 | NM_145258.1 | 680 | atgtaaccatcgactttctta | 413962 | - | see also 22926 line |
| 51795 | MGC22776 | NM_144962.1 | 334 | gccgtggacggcgcaacctatat | 411944 | - | see also 22927 line |
| 51796 | MGC22776 | NM_144962.1 | 594 | aactcgaggctcttggaaaatgg | 411947 | - | see also 22927 line |
| 51797 | MGC22793 | NM_145030.1 | 775 | gcccctctccgacttcaactcc | 412895 | - | - | - |
| 51798 | MGC22793 | NM_145030.1 | 604 | gccggaggaacaggttctgaatg | 412892 | - | see also 51797 line |
| 51799 | MGC22793 | NM_145030.1 | 727 | cccagaaacgctattttatgaat | 412894 | - | see also 51797 line |
| 51800 | MGC22805 | NM_144590.1 | 557 | tggctgtaccgcattacattatg | 410081 | - | see also 22928 line |
| 51801 | MGC22805 | NM_144590.1 | 669 | ctggatattgcacggagattaaa | 410083 | - | see also 22928 line |
| 51802 | MGC22960 | NM_199044.2 | 174 | cgcggagacatcgatataagaag | 440591 | - | see also 22929 line |
| 51803 | MGC22960 | NM_199044.2 | 231 | ttcgactggctttgcagaatttt | 440592 | - | see also 22929 line |
| 51804 | MGC23244 | NM_144615.1 | 199 | ctgggaacggctccgtgttaagt | 410337 | - | see also 22930 line |
| 51805 | MGC23244 | NM_144615.1 | 240 | tcctgtgtcaaccgtacatcacc | 410339 | - | see also 22930 line |
| 51806 | MGC23401 | NM_144982.3 | 3109 | gacggttacaaaagctagaatat | 412336 | - | see also 22931 line |
| 51807 | MGC23401 | NM_144982.3 | 1725 | gacctgaggcgatccttagataa | 412303 | - | see also 22931 line |
| 51808 | MGC23918 | NM_144716.1 | 168 | gcgagaagcacagggaacttagg | 411679 | - | see also 22932 line |
| 51809 | MGC23937 | NM_145052.1 | 723 | tgctgtcgatccatacgaattgg | 413210 | - | see also 22933 line |
| 51810 | MGC23937 | NM_145052.1 | 727 | gtcgatccatacgaattggaaag | 413211 | - | see also 22933 line |

Figure 46

| | | | | | | | |
|---|---|---|---|---|---|---|---|
| 51811 | MGC24039 | NM_144973.2 | 2669 | gacgagctcgggcgtggataaga | 412130 | - | see also 9754 line |
| 51812 | MGC24039 | NM_144973.2 | 1888 | cccaccttgcggacatctatata | 412107 | - | see also 9754 line |
| 51813 | MGC24047 | NM_178840.2 | 182 | ccccacggctcacctgacttttg | 432010 | - | - |
| 51814 | MGC24047 | NM_178840.2 | 185 | cacggctcacctgacttttgtta | 432012 | - | see also 51813 line |
| 51815 | MGC24047 | NM_178840.2 | 183 | cccacggctcacctgacttttgt | 432011 | - | see also 51813 line |
| 51816 | MGC24103 | NM_152576.1 | 471 | ttgcaaactaagagttgatgtgt | 418166 | - | see also 22935 line |
| 51817 | MGC24133 | NM_174896.2 | 492 | ccccattgtctatgctcaaatta | 428530 | - | see also 22936 line |
| 51818 | MGC24133 | NM_174896.2 | 399 | cccaggggaatcacttacctatg | 428528 | - | see also 22936 line |
| 51819 | MGC24180 | NM_152352.1 | 985 | tgctcggtataccgtgactttgg | 415782 | - | see also 22937 line |
| 51820 | MGC24180 | NM_152352.1 | 1063 | gccacccgtcaaggagtatctgc | 415785 | - | see also 22937 line |
| 51821 | MGC2452 | NM_032644.1 | 2531 | cacagctcaccacctatggatgc | 401163 | - | - |
| 51822 | MGC2452 | NM_032644.1 | 2482 | ttcaacagtgtggcctttcaaaa | 401162 | - | see also 51821 line |
| 51823 | MGC24665 | NM_152308.1 | 379 | aagatgacagacctttctgataa | 415447 | - | see also 22938 line |
| 51824 | MGC24665 | NM_152308.1 | 422 | gggaactggaggtagaagattta | 415448 | - | see also 22938 line |
| 51825 | MGC2477 | NM_024099.1 | 2812 | ggctcccgtgtttgtagaagtgg | 386025 | - | see also 22939 line |
| 51826 | MGC2477 | NM_024099.1 | 2977 | cacgcggcaccacctcaagaagc | 386026 | - | see also 22939 line |
| 51827 | MGC2491 | NM_024040.1 | 235 | ttcgatatggaggctttcactga | 385602 | - | see also 22940 line |
| 51828 | MGC2491 | NM_024040.1 | 429 | gcccgaaatgctcaaagaagaga | 385603 | - | see also 22940 line |
| 51829 | MGC2494 | NM_023933.1 | 691 | gaccgagtatgggcttatgatgt | 385431 | - | - |
| 51830 | MGC24975 | NM_153359.1 | 1394 | caccgtgtccaacgttgactact | 423049 | - | see also 22941 line |
| 51831 | MGC24975 | NM_153359.1 | 620 | gggccagtcagccctgtataagc | 423047 | - | see also 22941 line |
| 51832 | MGC24976 | NM_144598.2 | 648 | gaccgaaatcgtctatggtatgt | 410185 | - | see also 9711 line |
| 51833 | MGC24976 | NM_144598.2 | 639 | ctcactgtggaccgaaatcgtct | 410184 | - | see also 9711 line |

Figure 46

| 51834 | MGC25062 | NM_019020.2 | 2219 | cagcctgcacgatctgtgtaagc | 374501 | - | see also 22943 line | | |
|---|---|---|---|---|---|---|---|---|---|
| 51835 | MGC25062 | NM_019020.2 | 1099 | gagagagccagtacaaggttttc | 374492 | - | see also 22943 line | | |
| 51836 | MGC2508 | NM_024327.1 | 218 | cccgaaccctgaagagaacatga | 386295 | - | see also 22944 line | | |
| 51837 | MGC2562 | NM_032374.2 | 171 | tggataggaccccccagataaata | 400049 | - | see also 9176 line | | |
| 51838 | MGC2562 | NM_032374.2 | 202 | ttcgacctgttcactttacata | 400052 | - | see also 9176 line | | |
| 51839 | MGC2574 | NM_024098.1 | 798 | accgctccaagaaaagattctcc | 386021 | - | see also 22946 line | | |
| 51840 | MGC2574 | NM_024098.1 | 689 | aagaagcgaaaaggttcttcatc | 386016 | - | see also 22946 line | | |
| 51841 | MGC2574 | NM_024098.1 | 460 | ctcggagttggcccagaataagg | 386015 | - | see also 22946 line | | |
| 51842 | MGC2594 | NM_024050.2 | 141 | aagcaattttagtcgatttcacg | 385624 | - | - | - | - |
| 51843 | MGC2594 | NM_024050.2 | 278 | tgcatcagcaatgggacaaaaag | 385625 | - | see also 51842 line | | |
| 51844 | MGC2601 | NM_024042.2 | 669 | caccagcgacttcgtaattcacg | 385621 | - | see also 22947 line | | |
| 51845 | MGC2601 | NM_024042.2 | 667 | tgcaccagcgacttcgtaattca | 385620 | - | see also 22947 line | | |
| 51846 | MGC2603 | NM_024037.1 | 324 | aggcttagcacacgattgcatgg | 385555 | - | see also 22948 line | | |
| 51847 | MGC2603 | NM_024037.1 | 792 | aagccgtagtccagtttctgtgt | 385565 | - | see also 22948 line | | |
| 51848 | MGC2641 | NM_032631.2 | 265 | ctgttcccctacgacaaatgtaa | 401149 | - | see also 22949 line | | |
| 51849 | MGC2641 | NM_032631.2 | 268 | ttcccctacgacaaatgtaaaga | 401150 | - | see also 22949 line | | |
| 51850 | MGC2647 | NM_178841.2 | 685 | tgcttcaccgacacaagttctcc | 432015 | - | - | - | - |
| 51851 | MGC2650 | NM_024108.1 | 12 | ggcggatactgtgttgtttgagt | 386048 | - | see also 22950 line | | |
| 51852 | MGC2650 | NM_024108.1 | 324 | gacctacgtcctgcaagacaaca | 386050 | - | see also 22950 line | | |
| 51853 | MGC2654 | NM_024109.1 | 746 | gcccgcacgatatcatcagaata | 386058 | - | see also 22951 line | | |
| 51854 | MGC2654 | NM_024109.1 | 751 | cacgatatcatcagaataggtgt | 386061 | - | see also 22951 line | | |
| 51855 | MGC2655 | NM_024339.2 | 847 | gacgatcgaggtctataagcacg | 386325 | - | see also 22952 line | | |
| 51856 | MGC2655 | NM_024339.2 | 465 | ggcccgtctatagcatggtttcc | 386321 | - | see also 22952 line | | |
| 51857 | MGC2655 | NM_024339.2 | 289 | ggccttgcagcggctccatatga | 386316 | - | see also 22952 line | | |
| 51858 | MGC2656 | NM_024509.1 | 2099 | ctcggtactggtcttcatcttcg | 386476 | - | see also 22953 line | | |
| 51859 | MGC2656 | NM_024509.1 | 1453 | gccggctgctaggcaactcaagc | 386474 | - | see also 22953 line | | |
| 51860 | MGC26568 | NM_152402.1 | 391 | aagtccctctattattatggtgt | 416290 | - | see also 22954 line | | |
| 51861 | MGC26568 | NM_152402.1 | 1111 | tacgtaacatggaacttaattac | 416306 | - | see also 22954 line | | |
| 51862 | MGC26598 | NM_152638.2 | 946 | ggccggtcaagctgtatgagtgg | 419414 | - | see also 22955 line | | |
| 51863 | MGC26598 | NM_152638.2 | 561 | ccgggtgctccagcgtacaatag | 419410 | - | see also 22955 line | | |
| 51864 | MGC26647 | NM_152706.2 | 185 | gtccttttcggtccataaccaga | 419921 | - | see also 22956 line | | |

Figure 46

| 51865 | MGC26647 | NM_152706.2 | 250 | tccaagtcccacgtagcaattac | 419923 | - | see also 22956 line |
|---|---|---|---|---|---|---|---|
| 51866 | MGC26690 | NM_152450.1 | 1168 | tgggtttacagccgtctatgaaa | 416913 | - | see also 22957 line |
| 51867 | MGC26690 | NM_152450.1 | 447 | ctgccatagtgaagcaacttaat | 416897 | - | see also 22957 line |
| 51868 | MGC26710 | NM_152510.1 | 610 | tactatgaaactccactactata | 417336 | - | see also 22958 line |
| 51869 | MGC26710 | NM_152510.1 | 739 | ctccactggctttcatagcatga | 417342 | - | see also 22958 line |
| 51870 | MGC26710 | NM_152510.1 | 653 | taccaacccctcggttttaaaga | 417340 | - | see also 22958 line |
| 51871 | MGC26733 | NM_144992.2 | 2092 | ccctcggagtgcacttattaaga | 412500 | - | see also 22959 line |
| 51872 | MGC26733 | NM_144992.2 | 2089 | ttgccctcggagtgcacttatta | 412497 | - | see also 22959 line |
| 51873 | MGC26744 | NM_144645.1 | 337 | gggcagttgttataatgtacagg | 410712 | - | see also 22960 line |
| 51874 | MGC26744 | NM_144645.1 | 463 | cacaaaatgtaccaccattaaag | 410713 | - | see also 22960 line |
| 51875 | MGC26778 | NM_145010.2 | 797 | tccagtccctctcggtctttata | 412609 | - | see also 22961 line |
| 51876 | MGC26778 | NM_145010.2 | 807 | ctcggtctttatagattctatac | 412614 | - | see also 22961 line |
| 51877 | MGC26816 | NM_152613.1 | 271 | gacgaacctcactgttgaacaac | 418834 | - | see also 22962 line |
| 51878 | MGC26816 | NM_152613.1 | 440 | gcccgaggatttccacttagaac | 418837 | - | see also 22962 line |
| 51879 | MGC26816 | NM_152613.1 | 197 | taccgggtgattttcataacttc | 418831 | - | see also 22962 line |
| 51880 | MGC26818 | NM_152371.1 | 357 | atgagagcaagcagctttacaag | 415906 | - | see also 22963 line |
| 51881 | MGC26818 | NM_152371.1 | 384 | taggcttcaagcggtacaacagc | 415907 | - | see also 22963 line |
| 51882 | MGC26847 | NM_145006.2 | 526 | ggcctcaagcacttcaacaaacc | 412586 | - | see also 22964 line |
| 51883 | MGC26885 | NM_152339.2 | 1087 | taccagcctcagcctataggtct | 415719 | - | see also 22965 line |
| 51884 | MGC26963 | NM_152621.3 | 1440 | cacgaacactacactatcgatgt | 419029 | - | see also 22966 line |

Figure 46

| 51885 | MGC26963 | NM_152621.3 | 798 | tacccggactatatccaaattgc | 419009 | - | see also 22966 line |
|-------|----------|-------------|-----|-------------------------|--------|---|---------------------|
| 51886 | MGC26988 | NM_130899.1 | 1510 | cggtccgcatctggccataaaaa | 407125 | - | see also 22967 line |
| 51887 | MGC26988 | NM_130899.1 | 237 | tgcaccaatgttcgagagtaatt | 407103 | - | see also 22967 line |
| 51888 | MGC26989 | NM_152763.2 | 566 | gacgcttctaagtcaagcatttc | 420834 | - | see also 22968 line |
| 51889 | MGC26989 | NM_152763.2 | 277 | ttcagaacacacgacttataagc | 420824 | - | see also 22968 line |
| 51890 | MGC26999 | NM_152630.2 | 612 | atcatgaaagacgcttacgtaca | 419183 | - | see also 22969 line |
| 51891 | MGC26999 | NM_152630.2 | 623 | cgcttacgtacagaaattggtca | 419185 | - | see also 22969 line |
| 51892 | MGC27034 | NM_152292.2 | 951 | atcacaagggactcacatataaa | 415423 | - | see also 22970 line |
| 51893 | MGC27034 | NM_152292.2 | 876 | cagattcacctaatatactgaag | 415419 | - | see also 22970 line |
| 51894 | MGC27044 | NM_144977.1 | 823 | atgaaaggcgcatcgtgattatc | 412184 | - | see also 9761 line |
| 51895 | MGC27044 | NM_144977.1 | 837 | gtgattatctcgagcaaattaag | 412186 | - | see also 9761 line |
| 51896 | MGC2705 | NM_032701.2 | 575 | gtgtccactcgtgcttggaaaaa | 401234 | - | see also 22972 line |
| 51897 | MGC2705 | NM_032701.2 | 758 | tgcaaacccaactgcaagtttgt | 401235 | - | see also 22972 line |
| 51898 | MGC27085 | NM_153353.2 | 648 | tccatgtaggccgcatgttgaaa | 422953 | - | see also 22973 line |
| 51899 | MGC27085 | NM_153353.2 | 573 | ttgctgcaatgctgcaaattaat | 422950 | - | see also 22973 line |
| 51900 | MGC2714 | NM_032299.2 | 651 | tgcgctcacagttgaatgatatt | 399522 | - | see also 9150 line |
| 51901 | MGC2714 | NM_032299.2 | 371 | ccccctgctagactaataagtgg | 399515 | - | see also 9150 line |
| 51902 | MGC27169 | NM_176782.1 | 1173 | acgtccagggcagcggtaaaaca | 429602 | - | see also 22975 line |
| 51903 | MGC27169 | NM_176782.1 | 986 | ctctacgtccgggataacactgc | 429597 | - | see also 22975 line |
| 51904 | MGC27169 | NM_176782.1 | 1175 | gtccagggcagcggtaaaacagc | 429603 | - | see also 22975 line |
| 51905 | MGC2731 | NM_024068.2 | 345 | accaaagggtacgcttcagtttt | 385733 | - | see also 22976 line |
| 51906 | MGC2731 | NM_024068.2 | 350 | agggtacgcttcagttttcaaag | 385735 | - | see also 22976 line |
| 51907 | MGC27434 | NM_145050.1 | 413 | aggcattgcgtgaaaaactaaac | 413200 | - | see also 22977 line |

EP 1 752 536 A1

Figure 46

| | | | | | | | |
|---|---|---|---|---|---|---|---|
| 51908 | MGC27434 | NM_145050.1 | 412 | gaggcattgcgtgaaaaactaaa | 413199 | - | see also 22977 line |
| 51909 | MGC2747 | NM_024104.2 | 98 | ttcctccattcctgatgacttca | 386041 | - | see also 22978 line |
| 51910 | MGC2747 | NM_024104.2 | 190 | tcgccgaggtgtgcatggtttcc | 386043 | - | see also 22978 line |
| 51911 | MGC2749 | NM_024069.2 | 340 | cacgaggaccctagtagagatga | 385740 | - | see also 22979 line |
| 51912 | MGC2749 | NM_024069.2 | 344 | aggaccctagtagagatgaaacg | 385742 | - | see also 22979 line |
| 51913 | MGC2803 | NM_024038.2 | 551 | tgcggtgacgatgataaaactcg | 385586 | - | see also 8567 line |
| 51914 | MGC2803 | NM_024038.2 | 548 | cagtgcggtgacgatgataaaac | 385585 | - | see also 8567 line |
| 51915 | MGC2848 | NM_032917.1 | 141 | acccgaacaaactctgaagatga | 402506 | - | - | - | - |
| 51916 | MGC2865 | NM_032375.2 | 1117 | gaccccgagagtacagatgatgg | 400061 | - | see also 22981 line |
| 51917 | MGC2865 | NM_032375.2 | 1031 | agcagctgggcattagtgataat | 400059 | - | see also 22981 line |
| 51918 | MGC29649 | NM_173810.3 | 637 | tacgaacgagtgagagaatatag | 428210 | - | see also 22982 line |
| 51919 | MGC29649 | NM_173810.3 | 772 | gtgaataggcagcctaaagatgc | 428215 | - | see also 22982 line |
| 51920 | MGC29671 | NM_182538.2 | 504 | ggccttgatcctgcttatcctgc | 434233 | - | - | - | - |
| 51921 | MGC29671 | NM_182538.2 | 772 | aaccccgacagcctgatttttgg | 434234 | - | see also 51920 line |
| 51922 | MGC29671 | NM_182538.2 | 1065 | gacggcagaggcacttcagatca | 434235 | - | see also 51920 line |
| 51923 | MGC29761 | NM_144654.1 | 404 | ttccggaacaatactctcaatgt | 410873 | - | see also 22983 line |
| 51924 | MGC29761 | NM_144654.1 | 407 | cggaacaatactctcaatgttta | 410875 | - | see also 22983 line |
| 51925 | MGC29762 | NM_144706.2 | 608 | cagcgactcagttatttcaaaac | 411515 | - | see also 22984 line |
| 51926 | MGC29762 | NM_144706.2 | 601 | ttggaaccagcgactcagttatt | 411512 | - | see also 22984 line |
| 51927 | MGC29784 | NM_173659.1 | 437 | agctagtcgcctccagaagtact | 427135 | - | see also 22985 line |
| 51928 | MGC29784 | NM_173659.1 | 1021 | gcctggggctccgcttacaatag | 427139 | - | see also 22985 line |
| 51929 | MGC29784 | NM_173659.1 | 251 | acctctagtgacgttgaacaagc | 427133 | - | see also 22985 line |
| 51930 | MGC29875 | NM_014388.2 | 1605 | tgcggatgtggagcctcaataat | 340507 | - | see also 5646 line |
| 51931 | MGC29875 | NM_014388.2 | 1925 | gacttcgtgcgtcttcgaaatta | 340514 | - | see also 5646 line |

EP 1 752 536 A1

Figure 46

| | | | | | | | |
|---|---|---|---|---|---|---|---|
| 51932 | MGC29875 | NM_014388.2 | 2128 | gccgacatatccacactttaca | 340526 | - | see also 5646 line |
| 51933 | MGC29891 | NM_144618.1 | 679 | tcgagatgtcgtagagttactta | 410347 | - | see also 9716 line |
| 51934 | MGC29891 | NM_144618.1 | 686 | gtcgtagagttacttatcaaata | 410350 | - | see also 9716 line |
| 51935 | MGC29898 | NM_145048.2 | 741 | tacaagcgggagccttagcatca | 413189 | - | see also 22989 line |
| 51936 | MGC29898 | NM_145048.2 | 697 | gtcgttgttggtccttctctaaa | 413187 | - | see also 22989 line |
| 51937 | MGC29898 | NM_145048.2 | 711 | ttctctaaacgaccatctgaagc | 413188 | - | see also 22989 line |
| 51938 | MGC29937 | NM_144597.1 | 368 | tggtaaatctcgtgaaaaggtgg | 410168 | - | see also 22990 line |
| 51939 | MGC29937 | NM_144597.1 | 163 | tggcagttgatcctaaaggatgc | 410164 | - | see also 22990 line |
| 51940 | MGC29956 | NM_144638.1 | 233 | ttgtgatggcgagcaccaattct | 410617 | - | see also 22991 line |
| 51941 | MGC29956 | NM_144638.1 | 113 | gccgcttttggggcgtattcaac | 410614 | - | see also 22991 line |
| 51942 | MGC3020 | NM_024048.2 | 737 | acccatgtcaccacatcacttct | 385623 | - | - | | |
| 51943 | MGC3035 | NM_024293.2 | 738 | gggattatgatttcctacattgt | 386149 | - | see also 22992 line |
| 51944 | MGC3035 | NM_024293.2 | 782 | gcccctggtggtttatcatgagc | 386152 | - | see also 22992 line |
| 51945 | MGC3036 | NM_023942.1 | 675 | atcgacggtgagcgtgtgattgg | 385458 | - | - | | |
| 51946 | MGC3047 | NM_032348.2 | 1239 | aggacatccagctagattacaaa | 399864 | - | see also 9169 line |
| 51947 | MGC3048 | NM_024052.3 | 881 | tggtatcagtccctcaatctaac | 385650 | - | see also 8579 line |
| 51948 | MGC3048 | NM_024052.3 | 627 | aagatgttgatcggaaacactgg | 385642 | - | see also 8579 line |
| 51949 | MGC3067 | NM_024295.3 | 215 | ggcgatcacgcgctattggttcg | 386175 | - | see also 8605 line |
| 51950 | MGC3067 | NM_024295.3 | 675 | atcggaggctcggtaatcaatga | 386195 | - | see also 8605 line |
| 51951 | MGC3103 | NM_024036.3 | 1237 | cccaacgggaccttagagattgg | 385547 | - | see also 22996 line |
| 51952 | MGC3103 | NM_024036.3 | 866 | tggctccggacccgcttttctct | 385544 | - | see also 22996 line |
| 51953 | MGC3103 | NM_024036.3 | 1236 | ccccaacgggaccttagagattg | 385546 | - | see also 22996 line |
| 51954 | MGC3113 | NM_024035.1 | 274 | cggtgtcgtcctgcttccaaaac | 385539 | - | - | | |
| 51955 | MGC3121 | NM_024031.2 | 1652 | ctcgaccaatcaggttgaacaag | 385502 | - | see also 22997 line |
| 51956 | MGC3121 | NM_024031.2 | 1615 | gcccgtcctcagctaaaccttac | 385501 | - | see also 22997 line |
| 51957 | MGC3121 | NM_024031.2 | 1193 | ggcgaactcaggaccacaatacc | 385497 | - | see also 22997 line |
| 51958 | MGC3123 | NM_024107.1 | 297 | agctcctgggcgctattgtgtca | 386044 | - | - | | |
| 51959 | MGC3123 | NM_024107.1 | 377 | ggcaaccccgagccaactgaact | 386045 | - | see also 51958 line |

EP 1 752 536 A1

Figure 46

| 51960 | MGC3130 | NM_024032.2 | 835 | tccacaaagcgggtatcatgtcc | 385516 | - | see also 22998 line |
|---|---|---|---|---|---|---|---|
| 51961 | MGC3130 | NM_024032.2 | 1170 | aggactagctctggattatttcc | 385523 | - | see also 22998 line |
| 51962 | MGC3162 | NM_024078.1 | 1458 | ctcacggcctacgagatctttga | 385760 | - | see also 22999 line |
| 51963 | MGC3162 | NM_024078.1 | 962 | ggccttgaacgggctgttcatct | 385756 | - | see also 22999 line |
| 51964 | MGC3162 | NM_024078.1 | 1049 | ctctgtctttcacgtcaagtacc | 385759 | - | see also 22999 line |
| 51965 | MGC3169 | NM_024074.1 | 157 | ccccgtcttcgacctcagttact | 385743 | - | see also 23000 line |
| 51966 | MGC3169 | NM_024074.1 | 518 | cacgggtggttcgtcatgattgc | 385749 | - | see also 23000 line |
| 51967 | MGC31963 | NM_144580.1 | 773 | cactccatcgacgatgaatatgc | 409933 | - | see also 23001 line |
| 51968 | MGC31963 | NM_144580.1 | 771 | agcactccatcgacgatgaatat | 409932 | - | see also 23001 line |
| 51969 | MGC31963 | NM_144580.1 | 776 | tccatcgacgatgaatatgcacc | 409934 | - | see also 23001 line |
| 51970 | MGC31967 | NM_174923.1 | 294 | cgcggcttttgtgctgtacaagt | 428691 | - | see also 23002 line |
| 51971 | MGC31967 | NM_174923.1 | 296 | cggcttttgtgctgtacaagtgt | 428692 | - | see also 23002 line |
| 51972 | MGC3200 | NM_032305.1 | 367 | tggagcgttattcagacaaatat | 399606 | - | see also 9151 line |
| 51973 | MGC3200 | NM_032305.1 | 366 | gtggagcgttattcagacaaata | 399605 | - | see also 9151 line |
| 51974 | MGC32020 | NM_152266.1 | 314 | aggcttgttcgggttagaaattc | 415213 | - | see also 23004 line |
| 51975 | MGC32020 | NM_152266.1 | 321 | ttcgggttagaaattccaataat | 415216 | - | see also 23004 line |
| 51976 | MGC32124 | NM_144611.2 | 1258 | gcccaccgctagccatcacatgc | 410297 | - | see also 23005 line |
| 51977 | MGC32124 | NM_144611.2 | 854 | ttggctttcattctatgagaaga | 410296 | - | see also 23005 line |
| 51978 | MGC3222 | NM_024334.1 | 801 | atcattcgccgtggagacttttt | 386310 | - | see also 8612 line |
| 51979 | MGC3222 | NM_024334.1 | 176 | tacccggagagaacatgtcaaag | 386298 | - | see also 8612 line |
| 51980 | MGC3232 | NM_032313.2 | 1695 | tgctataggccgcatagatttcc | 399686 | - | see also 23007 line |
| 51981 | MGC3232 | NM_032313.2 | 219 | gggacgcgagcttccttatgacc | 399670 | - | see also 23007 line |
| 51982 | MGC3248 | NM_032486.2 | 148 | agccagaacatcgttctcaatgg | 400374 | - | see also 9218 line |
| 51983 | MGC3262 | NM_024029.3 | 161 | ttcgaggaggccactaatcttct | 385488 | - | see also 23009 line |
| 51984 | MGC3262 | NM_024029.3 | 404 | caggtcctggaccggatcaaagg | 385490 | - | see also 23009 line |
| 51985 | MGC3265 | NM_024028.2 | 623 | cacgcagcgctttattgatgatg | 385481 | - | see also 23010 line |
| 51986 | MGC3265 | NM_024028.2 | 621 | gtcacgcagcgctttattgatga | 385480 | - | see also 23010 line |
| 51987 | MGC3265 | NM_024028.2 | 618 | ggcgtcacgcagcgctttattga | 385479 | - | see also 23010 line |

Figure 46

| 51988 | MGC32871 | NM_152311.1 | 314 | atcacttacggactttttcgtgg | 415496 | - | see also 23011 line |
|---|---|---|---|---|---|---|---|
| 51989 | MGC32871 | NM_152311.1 | 564 | ggctcggtgcatccttcgttttt | 415504 | - | see also 23011 line |
| 51990 | MGC32871 | NM_152311.1 | 424 | aactctgcattcggtgactatcc | 415499 | - | see also 23011 line |
| 51991 | MGC33190 | NM_152749.2 | 300 | agcacatgacgacttctatctcg | 420521 | - | see also 9946 line |
| 51992 | MGC33212 | NM_152773.2 | 513 | ttctgcgttgtagcagcatttgg | 420988 | - | see also 23013 line |
| 51993 | MGC33212 | NM_152773.2 | 393 | atggtggtgcaagtagtgattgg | 420985 | - | see also 23013 line |
| 51994 | MGC33214 | NM_153354.2 | 464 | tgctacagttgtgtatctagtaa | 422982 | - | see also 9990 line |
| 51995 | MGC33214 | NM_153354.2 | 1063 | ctctgcgactctggttaataatc | 423004 | - | see also 9990 line |
| 51996 | MGC33214 | NM_153354.2 | 1061 | tactctgcgactctggttaataa | 423003 | - | see also 9990 line |
| 51997 | MGC3329 | NM_024086.2 | 742 | ctccgcctagttctgttaataca | 385824 | - | see also 8587 line |
| 51998 | MGC3329 | NM_024086.2 | 1150 | ttgtcacgacatggattgaaaaa | 385839 | - | see also 8587 line |
| 51999 | MGC33329 | NM_152782.2 | 1092 | ggggacacccgaagtatacttgt | 421150 | - | see also 23016 line |
| 52000 | MGC33329 | NM_152782.2 | 376 | cagacttcgtatgcctaaagagc | 421128 | - | see also 23016 line |
| 52001 | MGC33338 | NM_152366.2 | 325 | cagcagcgatacggtggttttcg | 415825 | - | see also 23017 line |
| 52002 | MGC33338 | NM_152366.2 | 328 | cagcgatacggtggttttcgacc | 415826 | - | see also 23017 line |
| 52003 | MGC33365 | NM_173552.2 | 1320 | gtgccactgtggaccacaattac | 425982 | - | see also 23018 line |
| 52004 | MGC33365 | NM_173552.2 | 1524 | acctagcacaattaagtaacaac | 425986 | - | see also 23018 line |
| 52005 | MGC33367 | NM_144602.2 | 595 | ggcttacgaacgcaagctaaaga | 410240 | - | see also 23019 line |
| 52006 | MGC33367 | NM_144602.2 | 487 | cccattccgtcgacaaagcattg | 410232 | - | see also 23019 line |
| 52007 | MGC33370 | NM_173807.2 | 353 | gtggtactatagagttagacatt | 428087 | - | see also 23020 line |
| 52008 | MGC33370 | NM_173807.2 | 920 | atcgcgggatggaatcgtttttc | 428099 | - | see also 23020 line |

Figure 46

| 52009 | MGC33371 | NM_144664.2 | 345 | gtgtacccagcgttatcctttcg | 411135 | - | see also 23021 line |
| 52010 | MGC33371 | NM_144664.2 | 705 | tactttatcgtacaaaagagttt | 411142 | - | see also 23021 line |
| 52011 | MGC33382 | NM_173529.3 | 215 | ttctgatggctcgtttcactata | 425524 | - | see also 23022 line |
| 52012 | MGC33382 | NM_173529.3 | 186 | tgcaccctgagtggtagtaattc | 425522 | - | see also 23022 line |
| 52013 | MGC33382 | NM_173529.3 | 668 | tcctgttatacgctcaaatataa | 425542 | - | see also 23022 line |
| 52014 | MGC33424 | NM_153705.2 | 611 | tacacgattctcaataaccatgt | 423731 | - | see also 23023 line |
| 52015 | MGC33424 | NM_153705.2 | 881 | atgcggggtgttacaaatgatct | 423744 | - | see also 23023 line |
| 52016 | MGC33510 | NM_152765.2 | 141 | cagtgacgagaacatagaagttt | 420878 | - | see also 9951 line |
| 52017 | MGC33510 | NM_152765.2 | 136 | agctgcagtgacgagaacataga | 420875 | - | see also 9951 line |
| 52018 | MGC33530 | NM_182546.1 | 977 | gtcgtatagagtagacagattta | 434323 | - | see also 23024 line |
| 52019 | MGC33530 | NM_182546.1 | 964 | cacagaaaggtcagtcgtataga | 434321 | - | see also 23024 line |
| 52020 | MGC33530 | NM_182546.1 | 392 | ggcgaccgaggggcagaatgtgg | 434314 | - | see also 23024 line |
| 52021 | MGC33547 | NM_144661.2 | 190 | aacacagcgctataaagaacagc | 411111 | - | see also 23025 line |
| 52022 | MGC33547 | NM_144661.2 | 324 | tcctggaatgcaaatatgtaaag | 411114 | - | see also 23025 line |
| 52023 | MGC33600 | NM_182539.1 | 671 | gccgctctgccggaattacgtgc | 434238 | - | see also 23026 line |
| 52024 | MGC33600 | NM_182539.1 | 1011 | aaggcacgcatcataattcgaag | 434242 | - | see also 23026 line |
| 52025 | MGC33602 | NM_152391.2 | 415 | tccttacatcgctgtattggtgt | 416197 | - | see also 23027 line |
| 52026 | MGC33602 | NM_152391.2 | 288 | ttctgcggtaccagtgttactat | 416194 | - | see also 23027 line |
| 52027 | MGC33607 | NM_152775.1 | 360 | gtgtttggcacgcgcattgaaaa | 421009 | - | see also 23028 line |
| 52028 | MGC33607 | NM_152775.1 | 368 | cacgcgcattgaaaaggatttgc | 421011 | - | see also 23028 line |

1744

Figure 46

| | | | | | | | |
|---|---|---|---|---|---|---|---|
| 52029 | MGC33630 | NM_144668.2 | 2367 | ttcgctctcatcgcaaaagcatt | 411205 | - | see also 23029 line |
| 52030 | MGC33630 | NM_144668.2 | 1669 | cacggtacttaccacaattgata | 411187 | - | see also 23029 line |
| 52031 | MGC33648 | NM_153706.2 | 815 | ggcgcagtcgtatctatgtatcc | 423765 | - | see also 23030 line |
| 52032 | MGC33648 | NM_153706.2 | 814 | aggcgcagtcgtatctatgtatc | 423764 | - | see also 23030 line |
| 52033 | MGC33653 | NM_153346.2 | 2198 | gtcggctagataccttattcaga | 422917 | - | see also 23031 line |
| 52034 | MGC33653 | NM_153346.2 | 2194 | gcctgtcggctagataccttatt | 422915 | - | see also 23031 line |
| 52035 | MGC33835 | NM_182548.2 | 738 | tgcaacacggccacagtctataa | 434334 | - | see also 23032 line |
| 52036 | MGC33835 | NM_182548.2 | 643 | tcccctctagagccttcaagact | 434333 | - | see also 23032 line |
| 52037 | MGC33864 | NM_152522.2 | 627 | gggctatagcatggcgattttga | 417559 | - | - |
| 52038 | MGC33864 | NM_152522.2 | 652 | ggcatcgtagctgctcttactgt | 417560 | - | see also 52037 line |
| 52039 | MGC33887 | NM_145036.1 | 1058 | caggagaagacgcacctattaca | 413046 | - | see also 9767 line |
| 52040 | MGC33887 | NM_145036.1 | 1344 | aacatgagattgtcgtcaataaa | 413052 | - | see also 9767 line |
| 52041 | MGC33889 | NM_173699.1 | 697 | caggcactactatgccttttca | 427725 | - | see also 23034 line |
| 52042 | MGC33889 | NM_173699.1 | 605 | aaggataagtgtcacttcaatga | 427722 | - | see also 23034 line |
| 52043 | MGC33894 | NM_152914.1 | 240 | gtccggctcaacggatgtcatga | 421687 | - | see also 23035 line |
| 52044 | MGC33894 | NM_152914.1 | 266 | ggccgtcctgctctatgagtacg | 421689 | - | see also 23035 line |
| 52045 | MGC33926 | NM_152390.1 | 848 | tgcatcgcttatccgtttattag | 416191 | - | see also 23036 line |
| 52046 | MGC33926 | NM_152390.1 | 435 | agcgatgcacggccatcaagtac | 416170 | - | see also 23036 line |
| 52047 | MGC33947 | NM_152358.1 | 581 | atcgtggtctcggcttgtacata | 415796 | - | see also 23037 line |
| 52048 | MGC33947 | NM_152358.1 | 585 | tggtctcggcttgtacatacaga | 415798 | - | see also 23037 line |

Figure 46

| 52049 | MGC33951 | NM_152448.1 | 148 | acccagacacgctgagaatatat | 416861 | - | see also 23038 line |
|---|---|---|---|---|---|---|---|
| 52050 | MGC33951 | NM_152448.1 | 149 | cccagacacgctgagaatatatc | 416862 | - | see also 23038 line |
| 52051 | MGC33951 | NM_152448.1 | 536 | aacaggttatatatcaattgatg | 416874 | - | see also 23038 line |
| 52052 | MGC33993 | NM_152737.1 | 1080 | cccttcgagcctcgttattctta | 420294 | - | see also 9937 line |
| 52053 | MGC33993 | NM_152737.1 | 1078 | gtccttcgagcctcgttattct | 420293 | - | see also 9937 line |
| 52054 | MGC34032 | NM_152697.2 | 1557 | tggtgcattcgctacttattact | 419861 | - | see also 23040 line |
| 52055 | MGC34032 | NM_152697.2 | 1308 | atcggggtacctgtatacaaag | 419850 | - | see also 23040 line |
| 52056 | MGC34032 | NM_152697.2 | 1554 | tgctggtgcattcgctacttatt | 419859 | - | see also 23040 line |
| 52057 | MGC34034 | NM_153224.2 | 642 | gccaagcactcacgcaatctttg | 422320 | - | see also 23041 line |
| 52058 | MGC34034 | NM_153224.2 | 450 | aagggcatgcacgctcactttaa | 422316 | - | see also 23041 line |
| 52059 | MGC34132 | NM_173654.1 | 1535 | gaggcgttcactacatcacttgg | 427129 | - | see also 10104 line |
| 52060 | MGC34132 | NM_173654.1 | 968 | aacttgacatccgtacattgacg | 427097 | - | see also 10104 line |
| 52061 | MGC34290 | NM_152315.1 | 1058 | cactacgtcagtggatctactac | 415536 | - | see also 23043 line |
| 52062 | MGC34290 | NM_152315.1 | 1347 | cagggccatcggtgttcaaaagg | 415545 | - | see also 23043 line |
| 52063 | MGC34290 | NM_152315.1 | 770 | cccggaatagagaggtatcttat | 415525 | - | see also 23043 line |
| 52064 | MGC34647 | NM_152456.1 | 527 | gagccgacttcagtacatgaaac | 416960 | - | see also 23044 line |
| 52065 | MGC34647 | NM_152456.1 | 461 | gtggcccttgacgcagaatgagg | 416958 | - | see also 23044 line |
| 52066 | MGC34680 | NM_152346.1 | 1460 | cacaatcgtgcgaggattcatcc | 415754 | - | see also 23045 line |
| 52067 | MGC34680 | NM_152346.1 | 609 | gaccttcggtccacgtttattgc | 415752 | - | see also 23045 line |
| 52068 | MGC34713 | NM_173665.1 | 731 | atcttcgacgcttcttagtgagc | 427197 | - | see also 23046 line |

Figure 46

| 52069 | MGC34713 | NM_173665.1 | 446 | ttgaatggctaactaactataat | 427187 | - | see also 23046 line |
|---|---|---|---|---|---|---|---|
| 52070 | MGC34728 | NM_152533.1 | 546 | ctcggggctccagattttacatc | 417728 | - | - |
| 52071 | MGC34728 | NM_152533.1 | 544 | ggctccgggctccagattttaca | 417727 | - | see also 52070 line |
| 52072 | MGC34741 | NM_153356.1 | 1023 | gccgcctggtggtttatgctga | 423040 | - | see also 23047 line |
| 52073 | MGC34741 | NM_153356.1 | 210 | gccaaatcacgggacttcatttg | 423034 | - | see also 23047 line |
| 52074 | MGC34741 | NM_153356.1 | 209 | ggccaaatcacgggacttcattt | 423033 | - | see also 23047 line |
| 52075 | MGC34799 | NM_182575.1 | 601 | ctgccgaggggtgttatatgt | 434487 | - | see also 23048 line |
| 52076 | MGC34799 | NM_182575.1 | 1443 | tgcggctccctagcactgataac | 434498 | - | see also 23048 line |
| 52077 | MGC34805 | NM_173525.1 | 329 | cccactgcactcgggaatactca | 425469 | - | see also 23049 line |
| 52078 | MGC34805 | NM_173525.1 | 601 | ctccggctacttcggtcattacc | 425473 | - | see also 23049 line |
| 52079 | MGC34824 | NM_173652.1 | 755 | ttcaagcgactaactaggaaaca | 427077 | - | see also 23050 line |
| 52080 | MGC34824 | NM_173652.1 | 798 | cactgtgcaacagattacatacc | 427079 | - | see also 23050 line |
| 52081 | MGC34830 | NM_152314.1 | 369 | ggcccagttagcacaatgtcttg | 415517 | - | see also 23051 line |
| 52082 | MGC34830 | NM_152314.1 | 370 | gcccagttagcacaatgtcttgc | 415518 | - | see also 23051 line |
| 52083 | MGC34831 | NM_152632.1 | 188 | gtgtaccgcctcccgattactgt | 419238 | - | see also 23052 line |
| 52084 | MGC34831 | NM_152632.1 | 1544 | ttccatcacgtatattagcttt | 419301 | - | see also 23052 line |
| 52085 | MGC34919 | NM_182582.1 | 1412 | cacgatattgtgtatgtacatat | 434516 | - | see also 23053 line |
| 52086 | MGC34919 | NM_182582.1 | 1448 | cacacacgattatattgttgtatg | 434517 | - | see also 23053 line |
| 52087 | MGC35010 | NM_178505.2 | 585 | ggcgggatcactcgagatcaact | 430936 | - | see also 23054 line |
| 52088 | MGC35010 | NM_178505.2 | 630 | gtgggacagcgcgctgacatact | 430937 | - | see also 23054 line |

Figure 46

| | | | | | | | | | |
|---|---|---|---|---|---|---|---|---|---|
| 52089 | MGC35033 | NM_152319.2 | 308 | ggcctccagattccttgatgacc | 415589 | - | - | - | - |
| 52090 | MGC35033 | NM_152319.2 | 193 | gtgctgacagtttttaaggatat | 415588 | - | see also 52089 line | | |
| 52091 | MGC35043 | NM_152770.1 | 284 | cagcaaaagacgctaacaagtgc | 420899 | - | see also 23055 line | | |
| 52092 | MGC35043 | NM_152770.1 | 451 | caggctaaagacggaagattttg | 420902 | - | see also 23055 line | | |
| 52093 | MGC35048 | NM_153208.1 | 838 | accgagccgtcaagcaagaatct | 422141 | - | see also 23056 line | | |
| 52094 | MGC35048 | NM_153208.1 | 842 | agccgtcaagcaagaatctgtgg | 422142 | - | see also 23056 line | | |
| 52095 | MGC35048 | NM_153208.1 | 1205 | ttctgactgagtggttatacaac | 422147 | - | see also 23056 line | | |
| 52096 | MGC35062 | NM_198515.1 | 312 | cgccgtttgatgcgagaagtaag | 438913 | - | see also 23057 line | | |
| 52097 | MGC35062 | NM_198515.1 | 313 | gccgtttgatgcgagaagtaagg | 438914 | - | see also 23057 line | | |
| 52098 | MGC35097 | NM_173546.1 | 976 | gcctgcgcatgagggataagagg | 425829 | - | see also 23058 line | | |
| 52099 | MGC35097 | NM_173546.1 | 897 | ttctactctcgcccacactttgt | 425826 | - | see also 23058 line | | |
| 52100 | MGC35118 | NM_152453.2 | 383 | acgttggtccacagtataacaga | 416944 | - | see also 23059 line | | |
| 52101 | MGC35118 | NM_152453.2 | 392 | cacagtataacagaacttcaaca | 416945 | - | see also 23059 line | | |
| 52102 | MGC35138 | NM_173573.1 | 648 | aagcgacctctctgaaaacattc | 426288 | - | see also 23060 line | | |
| 52103 | MGC35138 | NM_173573.1 | 815 | cagagtcctctggaaagcattcc | 426290 | - | see also 23060 line | | |
| 52104 | MGC35154 | NM_152614.1 | 180 | cactgactggcaggatatagatc | 418846 | - | see also 23061 line | | |
| 52105 | MGC35154 | NM_152614.1 | 177 | atccactgactggcaggatatag | 418845 | - | see also 23061 line | | |
| 52106 | MGC35154 | NM_152614.1 | 223 | acccccagggccatatcaacatc | 418848 | - | see also 23061 line | | |
| 52107 | MGC35169 | NM_152324.1 | 526 | gacgaggataaggatgatgtaac | 415592 | - | see also 23062 line | | |
| 52108 | MGC35169 | NM_152324.1 | 194 | atgttcctctgtatgacatttgt | 415590 | - | see also 23062 line | | |

1748

Figure 46

| | | | | | | | |
|---|---|---|---|---|---|---|---|
| 52109 | MGC35179 | NM_153230.1 | 1382 | atcgaatcagagcttagctattt | 422417 | - | see also 23063 line |
| 52110 | MGC35179 | NM_153230.1 | 1295 | ttcaaggcgagaatttatacaaa | 422413 | - | see also 23063 line |
| 52111 | MGC35182 | NM_153707.1 | 643 | ctcgggacagtaaatatccatgt | 423796 | - | see also 23064 line |
| 52112 | MGC35182 | NM_153707.1 | 574 | tggatttgctcacgacgtataag | 423792 | - | see also 23064 line |
| 52113 | MGC35182 | NM_153707.1 | 584 | cacgacgtataagaaagattaca | 423795 | - | see also 23064 line |
| 52114 | MGC35194 | NM_152290.1 | 627 | tacaaactatggactctatgagc | 415386 | - | see also 23065 line |
| 52115 | MGC35194 | NM_152290.1 | 504 | cacctatgacgaccattacaacc | 415384 | - | see also 23065 line |
| 52116 | MGC35206 | NM_178552.2 | 598 | aagtcttcgagggccagaaaagg | 431190 | - | see also 23066 line |
| 52117 | MGC35206 | NM_178552.2 | 566 | ctccgaggagcaggctcaaaagg | 431189 | - | see also 23066 line |
| 52118 | MGC35206 | NM_178552.2 | 839 | gtggcaccagaaaaatgctatga | 431196 | - | see also 23066 line |
| 52119 | MGC35212 | NM_152764.1 | 932 | ctgcacgttcgttctatcacaca | 420872 | - | see also 23067 line |
| 52120 | MGC35212 | NM_152764.1 | 736 | aagtagttcgaaatagatgttcc | 420866 | - | see also 23067 line |
| 52121 | MGC35261 | NM_173494.1 | 652 | ctcttgaaatcactatgactatg | 425153 | - | see also 23068 line |
| 52122 | MGC35261 | NM_173494.1 | 162 | gccctctctaagctacttaatcc | 425141 | - | see also 23068 line |
| 52123 | MGC35295 | NM_152717.1 | 312 | atctggccgtgcttactatcttc | 420070 | - | see also 23069 line |
| 52124 | MGC35392 | NM_153336.1 | 588 | atcggtgttgtcgcgtatgatga | 422769 | - | see also 23070 line |
| 52125 | MGC35392 | NM_153336.1 | 931 | accagctggctcggaaatattca | 422780 | - | see also 23070 line |
| 52126 | MGC35468 | NM_153244.1 | 372 | atcttgagcccgaaaagagaaag | 422577 | - | see also 23071 line |
| 52127 | MGC35468 | NM_153244.1 | 505 | aagtcggggacaagtgatgaagc | 422580 | - | see also 23071 line |
| 52128 | MGC35521 | NM_145065.1 | 1472 | atggctgcgtccgcctcattttc | 413485 | - | see also 23072 line |

EP 1 752 536 A1

Figure 46

| | | | | | | | |
|---|---|---|---|---|---|---|---|
| 52129 | MGC35521 | NM_145065.1 | 1473 | tggctcgctccgcctcattttcc | 413486 | - | see also 23072 line |
| 52130 | MGC35555 | NM_178565.3 | 392 | acgcagtaagcgagctagttatg | 431295 | - | see also 23073 line |
| 52131 | MGC35555 | NM_178565.3 | 393 | cgcagtaagcgagctagttatgt | 431296 | - | see also 23073 line |
| 52132 | MGC35555 | NM_178565.3 | 399 | aagcggagctagttatgtatcaaa | 431298 | - | see also 23073 line |
| 52133 | MGC35558 | NM_145013.1 | 393 | caggatgtgcatgccatagtaac | 412616 | - | see also 23074 line |
| 52134 | MGC35558 | NM_145013.1 | 549 | atctgcaaggccatgatagtag | 412618 | - | see also 23074 line |
| 52135 | MGC3731 | NM_024313.1 | 78 | gccgagctcgttcttagcttcg | 386225 | - | see also 23075 line |
| 52136 | MGC3731 | NM_024313.1 | 540 | tgcacacgccgcaaaaaggtca | 386230 | - | see also 23075 line |
| 52137 | MGC3794 | NM_152902.2 | 758 | tacgtcacgagaaaagcaaaattt | 421557 | - | see also 9960 line |
| 52138 | MGC3794 | NM_152902.2 | 433 | aaccatatgattggacctataca | 421544 | - | see also 9960 line |
| 52139 | MGC39325 | NM_147189.1 | 1155 | tccctccagcggtctaagtcaga | 415085 | - | see also 23077 line |
| 52140 | MGC39325 | NM_147189.1 | 1401 | cgcgtgcgtatggcatttctgc | 415088 | - | see also 23077 line |
| 52141 | MGC39338 | NM_152480.1 | 358 | gccacgcctacccaagatttacg | 417024 | - | see also 23078 line |
| 52142 | MGC39338 | NM_152480.1 | 356 | ggggcacgcctacccaagattta | 417023 | - | see also 23078 line |
| 52143 | MGC39350 | NM_144970.1 | 600 | gtggcagtatactccagaataga | 412044 | - | see also 9751 line |
| 52144 | MGC39518 | NM_173822.1 | 1632 | aacaatgctaatcgatactccaac | 428386 | - | see also 10117 line |
| 52145 | MGC39518 | NM_173822.1 | 862 | aactctgtggtcgaatagtattg | 428357 | - | see also 10117 line |
| 52146 | MGC39520 | NM_153364.1 | 613 | accgtgccgaatggtaacaatg | 423060 | - | see also 23080 line |
| 52147 | MGC39520 | NM_153364.1 | 519 | tacaaaggcgagtatactatatt | 423058 | - | see also 23080 line |
| 52148 | MGC39571 | NM_152728.1 | 448 | gagattctacatgctctcaatga | 420136 | - | see also 23081 line |
| 52149 | MGC39571 | NM_152728.1 | 384 | ctggtttatcagccacacttaca | 420134 | - | see also 23081 line |
| 52150 | MGC39581 | NM_152784.2 | 1839 | atcgacgccgagtatgttgttact | 421202 | - | see also 23082 line |

Figure 46

| | | | | | | | |
|---|---|---|---|---|---|---|---|
| 52151 | MGC39581 | NM_152784.2 | 2213 | tggagtcgtcatcctactgatca | 421211 | - | see also 23082 line |
| 52152 | MGC39633 | NM_152549.1 | 1414 | gccagcgccagtttaagttaaaa | 417969 | - | see also 23083 line |
| 52153 | MGC39633 | NM_152549.1 | 1411 | aacgccagcgccagtttaagtta | 417966 | - | see also 23083 line |
| 52154 | MGC39655 | NM_182541.1 | 359 | cacgtaggacacctacaacatcc | 434277 | - | see also 23084 line |
| 52155 | MGC39655 | NM_182541.1 | 390 | aacgatcaaccttcttgaagtcc | 434279 | - | see also 23084 line |
| 52156 | MGC39662 | NM_152394.2 | 1864 | atccgtcgcctgtttcataaact | 416248 | - | see also 23085 line |
| 52157 | MGC39662 | NM_152394.2 | 1805 | ttccaatcctccgatacgtaagt | 416245 | - | see also 23085 line |
| 52158 | MGC39662 | NM_152394.2 | 1861 | aagatccgtcgcctgtttcataa | 416247 | - | see also 23085 line |
| 52159 | MGC39681 | NM_174939.2 | 212 | ttcggccttgcaataattggtat | 428761 | - | see also 23086 line |
| 52160 | MGC39681 | NM_174939.2 | 344 | ctgcagctgcttgttagatctac | 428763 | - | see also 23086 line |
| 52161 | MGC39724 | NM_174906.1 | 506 | tccctggaacccccaactcatct | 428577 | - | see also 23088 line |
| 52162 | MGC39725 | NM_152397.1 | 87 | gacgaaggaaccctcaaaagaag | 416256 | - | see also 23089 line |
| 52163 | MGC39725 | NM_152397.1 | 360 | gtgctggtggcggctgatactgt | 416258 | - | see also 23089 line |
| 52164 | MGC39827 | NM_178499.2 | 610 | ctcaaaaaggaccttatacgaag | 430896 | - | see also 23090 line |
| 52165 | MGC39827 | NM_178499.2 | 1558 | aagtctaaaaaccgcactaattg | 430908 | - | see also 23090 line |
| 52166 | MGC39827 | NM_178499.2 | 847 | gtcacacgtcgcccattcactcc | 430901 | - | see also 23090 line |
| 52167 | MGC39830 | NM_152756.1 | 451 | gacacaagcacttcgattagtca | 420679 | - | see also 23091 line |
| 52168 | MGC39830 | NM_152756.1 | 333 | tacgagcgcttcgatatctcatc | 420672 | - | see also 23091 line |
| 52169 | MGC39830 | NM_152756.1 | 334 | acgagcgcttcgatatctcatcc | 420673 | - | see also 23091 line |
| 52170 | MGC40053 | NM_152583.1 | 441 | gcccgagtgcgctctaccatacc | 418256 | - | see also 23092 line |

Figure 46

| 52171 | MGC40053 | NM_152583.1 | 1164 | cacgaacaagactggaattgtgg | 418270 | - | see also 23092 line | | |
|---|---|---|---|---|---|---|---|---|---|
| 52172 | MGC40069 | NM_182615.1 | 405 | ctcacgctataatgtattagagg | 434704 | - | see also 23093 line | | |
| 52173 | MGC40069 | NM_182615.1 | 403 | ggctcacgctataatgtattaga | 434703 | - | see also 23093 line | | |
| 52174 | MGC40084 | NM_152769.1 | 1255 | ctcggcaggtatttttcagtaga | 420893 | - | - | | - |
| 52175 | MGC40157 | NM_152350.1 | 424 | ttgcaaattcgtgaagaatcagc | 415766 | - | see also 23094 line | | |
| 52176 | MGC40157 | NM_152350.1 | 132 | gcggagggcagctgttgagatgg | 415764 | - | see also 23094 line | | |
| 52177 | MGC40168 | NM_153709.1 | 729 | ggcttctgctctcctcaatgtat | 423822 | - | - | | - |
| 52178 | MGC40168 | NM_153709.1 | 464 | tgggaattgaaaagagatttaat | 423821 | - | see also 52177 line | | |
| 52179 | MGC40178 | NM_152325.1 | 162 | ttcgccaaaacggtatcagaaga | 415593 | - | see also 23095 line | | |
| 52180 | MGC40178 | NM_152325.1 | 170 | aacggtatcagaagattaggata | 415594 | - | see also 23095 line | | |
| 52181 | MGC40178 | NM_152325.1 | 201 | cacttagtgatcctattctcaat | 415598 | - | see also 23095 line | | |
| 52182 | MGC40179 | NM_173472.1 | 348 | cacaccgtccgaccttgaagtgc | 424908 | - | see also 23096 line | | |
| 52183 | MGC40179 | NM_173472.1 | 460 | ttgcgcacgatgaacaacaaagg | 424910 | - | see also 23096 line | | |
| 52184 | MGC40195 | NM_152441.1 | 942 | ctcggggctggatgtttcgttct | 416839 | - | see also 23097 line | | |
| 52185 | MGC40195 | NM_152441.1 | 1291 | aaggaggcacgaggggatttttc | 416841 | - | see also 23097 line | | |
| 52186 | MGC40214 | NM_152416.1 | 216 | acgggattatgaaggttatttat | 416486 | - | see also 23098 line | | |
| 52187 | MGC40214 | NM_152416.1 | 355 | tgggatctgaacactaaacattg | 416492 | - | see also 23098 line | | |
| 52188 | MGC40222 | NM_152738.1 | 279 | atggattcgagtgatgaaacagg | 420297 | - | see also 23099 line | | |
| 52189 | MGC40222 | NM_152738.1 | 424 | atggaaccttagtgaaaatctgg | 420300 | - | see also 23099 line | | |
| 52190 | MGC40368 | NM_152772.1 | 747 | tcccgtgcgagataatgatatca | 420938 | - | see also 23100 line | | |

EP 1 752 536 A1

Figure 46

| | | | | | | | |
|---|---|---|---|---|---|---|---|
| 52191 | MGC40368 | NM_152772.1 | 744 | tgctccgtgcgagataargata | 420936 | - | see also 23100 line |
| 52192 | MGC40397 | NM_152318.1 | 293 | tgcaccaactggtcgttcaata | 415578 | - | see also 23101 line |
| 52193 | MGC40397 | NM_152318.1 | 299 | acctggtcgtttcaatattgaaa | 415581 | - | see also 23101 line |
| 52194 | MGC40397 | NM_152318.1 | 295 | caccaactggtcgtttcaatatt | 415579 | - | see also 23101 line |
| 52195 | MGC40499 | NM_152755.1 | 535 | gcccagcgtggaggtcacatacc | 420654 | - | see also 23102 line |
| 52196 | MGC40499 | NM_152755.1 | 195 | cagaacgcttgcccagccaaatgc | 420650 | - | see also 23102 line |
| 52197 | MGC4093 | NM_030578.2 | 448 | agcttgccaggctatggatttgc | 394699 | - | see also 23103 line |
| 52198 | MGC4126 | NM_032773.2 | 733 | tacagacgatcaccctagataac | 401377 | - | see also 23104 line |
| 52199 | MGC4126 | NM_032773.2 | 272 | gtcgcgaaatgccctttcaggaaa | 401357 | - | see also 23104 line |
| 52200 | MGC41816 | NM_016466.4 | 360 | cacgcggctcctgctatcacatg | 357595 | - | see also 23105 line |
| 52201 | MGC41816 | NM_016466.4 | 361 | acgcggctcctgctatcacatgg | 357596 | - | see also 23105 line |
| 52202 | MGC4189 | NM_032308.1 | 120 | gccgggacgaggctcctaaacagg | 399635 | - | see also 23106 line |
| 52203 | MGC4189 | NM_032308.1 | 175 | ttctcagaaacagctttctagttc | 399636 | - | see also 23106 line |
| 52204 | MGC41906 | NM_152474.2 | 460 | atctcctatatgatatatcgact | 417009 | - | see also 23107 line |
| 52205 | MGC41906 | NM_152474.2 | 529 | ctcaggataccgttattaggaga | 417013 | - | see also 23107 line |
| 52206 | MGC42090 | NM_152774.1 | 263 | tcgactagccagttatgaacaga | 420994 | - | see also 23108 line |
| 52207 | MGC42090 | NM_152774.1 | 153 | ctccgggcagtcacaaagaaaac | 420992 | - | see also 23108 line |
| 52208 | MGC42157 | NM_153241.1 | 330 | gccatgttgtacagaactacttc | 422569 | - | see also 23109 line |
| 52209 | MGC42157 | NM_153241.1 | 87 | ctggcccctcaagggtaaagtca | 422568 | - | see also 23109 line |
| 52210 | MGC4238 | NM_032332.2 | 101 | cagtgcgagccgtacgatttaat | 399765 | - | see also 23110 line |
| 52211 | MGC4238 | NM_032332.2 | 105 | gcgagccgtacgatttaatgtgg | 399767 | - | see also 23110 line |
| 52212 | MGC4248 | NM_032333.2 | 569 | gtctgggagtgtgttacaacttc | 399776 | - | see also 23111 line |
| 52213 | MGC4248 | NM_032333.2 | 459 | gactgaagtgaaggatttccagc | 399775 | - | see also 23111 line |
| 52214 | MGC42493 | NM_178549.2 | 592 | tgcaatagacttactcaatgttc | 431172 | - | see also 23112 line |

Figure 46

| | | | | | | | |
|---|---|---|---|---|---|---|---|
| 52215 | MGC42493 | NM_178549.2 | 494 | aagtgacactgacaagacataga | 431166 | - | see also 23112 line |
| 52216 | MGC4251 | NM_032376.2 | 516 | aggaccggctggcgtatctgaac | 400066 | - | see also 23113 line |
| 52217 | MGC4251 | NM_032376.2 | 336 | ggctgaaggtccgtatgtactcg | 400065 | - | see also 23113 line |
| 52218 | MGC42530 | NM_173570.1 | 454 | gtgatcgatggattacatgtaaa | 426243 | - | see also 23114 line |
| 52219 | MGC42530 | NM_173570.1 | 984 | atgtcgggcagtctcaacaatcg | 426251 | - | see also 23114 line |
| 52220 | MGC42630 | NM_175923.2 | 105 | accgcgtcggcgaaaaggattga | 429589 | - | - |
| 52221 | MGC42630 | NM_175923.2 | 104 | aaccgcgtcggcgaaaaggattg | 429588 | - | see also 52220 line |
| 52222 | MGC42630 | NM_175923.2 | 47 | tgctccgggaccgaagaatctcc | 429587 | - | see also 52220 line |
| 52223 | MGC4268 | NM_031445.1 | 491 | aggaagcggtctagaagtgtacg | 397040 | - | see also 23115 line |
| 52224 | MGC4268 | NM_031445.1 | 517 | acggtcgcctcatgaaagtgtgt | 397042 | - | see also 23115 line |
| 52225 | MGC43033 | NM_152711.1 | 165 | cacgtgttcaacggaaattcaca | 420006 | - | see also 23116 line |
| 52226 | MGC43033 | NM_152711.1 | 163 | tccacgtgttcaacggaaattca | 420005 | - | see also 23116 line |
| 52227 | MGC43033 | NM_152711.1 | 559 | ggccgagggacttgggttttaca | 420012 | - | see also 23116 line |
| 52228 | MGC4308 | NM_032359.2 | 560 | ttgtcctaagtgggtaaaactta | 399967 | - | see also 23117 line |
| 52229 | MGC4308 | NM_032359.2 | 680 | aggagacggcaaagttataaaat | 399970 | - | see also 23117 line |
| 52230 | MGC43122 | NM_173513.2 | 341 | aaggacaatggcagtagagatga | 425266 | - | see also 23118 line |
| 52231 | MGC43122 | NM_173513.2 | 398 | accactgtcccgggaaagaaagg | 425267 | - | see also 23118 line |
| 52232 | MGC43690 | NM_182552.2 | 2300 | ctcacggcctgtccatcaaatct | 434397 | - | see also 23119 line |
| 52233 | MGC43690 | NM_182552.2 | 2156 | cacgacgggtgcagtagacatga | 434392 | - | see also 23119 line |
| 52234 | MGC43690 | NM_182552.2 | 1442 | accgacctctccactgtatctgg | 434376 | - | see also 23119 line |
| 52235 | MGC44287 | NM_182607.2 | 394 | tccaacgatccaggtaatgcatc | 434609 | - | see also 23120 line |
| 52236 | MGC44287 | NM_182607.2 | 1000 | gacgctacccaactagaagtaac | 434625 | - | see also 23120 line |
| 52237 | MGC44294 | NM_173499.1 | 282 | acctcgtcgcgacatttctcaga | 425167 | - | see also 23121 line |

1754

Figure 46

| | | | | | | | |
|---|---|---|---|---|---|---|---|
| 52238 | MGC44294 | NM_173499.1 | 320 | ctgcggctggaggcctttcaagg | 425168 | - | see also 23121 line |
| 52239 | MGC44505 | NM_178553.2 | 1257 | ccccagtcgtggagtatcctata | 431199 | - | see also 23122 line |
| 52240 | MGC44505 | NM_178553.2 | 589 | ttggggcttctcacaacttgtgg | 431197 | - | see also 23122 line |
| 52241 | MGC44593 | NM_173554.1 | 908 | ggcctttaatgcacgaatagaaa | 426024 | - | see also 23123 line |
| 52242 | MGC44593 | NM_173554.1 | 862 | tcggcattcagaccgagataaac | 426019 | - | see also 23123 line |
| 52243 | MGC4473 | NM_080719.1 | 565 | gaccaagtgaccgttcttgttcc | 406669 | - | see also 23124 line |
| 52244 | MGC4473 | NM_080719.1 | 590 | gtgtgttgtgggctaagaagaga | 406670 | - | see also 23124 line |
| 52245 | MGC4504 | NM_024111.2 | 438 | cagtgcttggtggctacgatacc | 386065 | - | see also 23125 line |
| 52246 | MGC4504 | NM_024111.2 | 604 | ctccggccacaaccttgaatact | 386067 | - | see also 23125 line |
| 52247 | MGC45386 | NM_198527.1 | 165 | gaggcgggaatcactgacattgt | 439137 | - | see also 23126 line |
| 52248 | MGC45386 | NM_198527.1 | 391 | cagacaagctgtacaatctgagg | 439139 | - | see also 23126 line |
| 52249 | MGC45400 | NM_153333.1 | 436 | cacacccgttaggcatttggacc | 422768 | - | - | - | - |
| 52250 | MGC45400 | NM_153333.1 | 245 | atgcaaaagtcttgtgaagaaaa | 422766 | - | see also 52249 line |
| 52251 | MGC45400 | NM_153333.1 | 408 | atcagcctggccagggatttaaa | 422767 | - | see also 52249 line |
| 52252 | MGC45416 | NM_152398.1 | 407 | gactagtctaccaaggttatttg | 416263 | - | see also 23127 line |
| 52253 | MGC45416 | NM_152398.1 | 218 | ctgctcgtggaaaccaagataaa | 416261 | - | see also 23127 line |
| 52254 | MGC45441 | NM_152499.1 | 482 | cccaagcttgggtccagtatagc | 417214 | - | - | - | - |
| 52255 | MGC45441 | NM_152499.1 | 387 | accaccgcctctcctaaaggacc | 417213 | - | see also 52254 line |
| 52256 | MGC45441 | NM_152499.1 | 361 | atctctgacccctcttctcttct | 417212 | - | see also 52254 line |
| 52257 | MGC45474 | NM_152369.2 | 1100 | tacatgtggtcgtaccatttaat | 415871 | - | see also 23128 line |
| 52258 | MGC45474 | NM_152369.2 | 1153 | tgcgtgccagcaaatgactatag | 415876 | - | see also 23128 line |
| 52259 | MGC4549 | NM_032377.2 | 188 | ttccagacgcccataacgtatct | 400070 | - | - | - | - |
| 52260 | MGC4549 | NM_032377.2 | 23 | gggcgcagaaagtcaaaacgaaa | 400068 | - | see also 52259 line |

Figure 46

| 52261 | MGC4549 | NM_032377.2 | 24 | ggcgcagaaagtcaaaacgaaag | 400069 | - | see also 52259 line | | | |
|---|---|---|---|---|---|---|---|---|---|---|
| 52262 | MGC45491 | NM_153246.1 | 984 | cacggctaatgcgctctaattac | 422592 | - | see also 23129 line | | | |
| 52263 | MGC45491 | NM_153246.1 | 949 | cccgagcgcgagaagaaattaca | 422591 | - | see also 23129 line | | | |
| 52264 | MGC45714 | NM_152464.1 | 574 | gggtctggccgagctgtatgtca | 416980 | - | see also 23130 line | | | |
| 52265 | MGC45714 | NM_152464.1 | 286 | tccacggaacccagaactagttg | 416970 | - | see also 23130 line | | | |
| 52266 | MGC45780 | NM_173833.2 | 659 | ggccctgactcgcaatgtgaacc | 428450 | - | see also 23131 line | | | |
| 52267 | MGC45780 | NM_173833.2 | 673 | atgtgaaccggctgaatgagagc | 428451 | - | see also 23131 line | | | |
| 52268 | MGC45866 | NM_152259.2 | 1092 | tgccaggcttcggcacaactaga | 415186 | - | see also 23132 line | | | |
| 52269 | MGC45866 | NM_152259.2 | 1196 | tggccttcccaaacttcgaatta | 415188 | - | see also 23132 line | | | |
| 52270 | MGC45871 | NM_182705.1 | 99 | agctcgtggacgcgaagaagaag | 434922 | - | see also 23133 line | | | |
| 52271 | MGC45871 | NM_182705.1 | 683 | agcgacgacctctctgatgaatg | 434923 | - | see also 23133 line | | | |
| 52272 | MGC45873 | NM_152486.1 | 1402 | gcccttgccggtcaaacttcacc | 417056 | - | see also 23134 line | | | |
| 52273 | MGC45873 | NM_152486.1 | 1251 | caggcgcctgggccgagttttct | 417054 | - | see also 23134 line | | | |
| 52274 | MGC45962 | NM_152644.1 | 286 | gccttgctcctgctgatagttgt | 419459 | - | - | | - | - |
| 52275 | MGC45962 | NM_152644.1 | 285 | ggccttgctcctgctgatagttg | 419458 | - | see also 52274 line | | | |
| 52276 | MGC4606 | NM_024516.2 | 758 | tccctcggcagcggaaatactga | 386572 | - | - | | - | - |
| 52277 | MGC4606 | NM_024516.2 | 502 | cacatgcccacggaatttgattt | 386570 | - | see also 52276 line | | | |
| 52278 | MGC4606 | NM_024516.2 | 510 | cacggaatttgattttgatgatg | 386571 | - | see also 52276 line | | | |
| 52279 | MGC4614 | NM_024294.1 | 422 | gtcaatacctccggatactcagt | 386157 | - | see also 23135 line | | | |
| 52280 | MGC4614 | NM_024294.1 | 981 | agcagtcacgcaaacaacttatc | 386172 | - | see also 23135 line | | | |
| 52281 | MGC4614 | NM_024294.1 | 428 | acctccggatactcagtttgtaa | 386158 | - | see also 23135 line | | | |
| 52282 | MGC4618 | NM_032326.2 | 467 | gccttacacgttttcgttaatgg | 399743 | - | see also 23136 line | | | |
| 52283 | MGC4618 | NM_032326.2 | 465 | ctgccttacacgttttcgttaat | 399741 | - | see also 23136 line | | | |
| 52284 | MGC4645 | NM_024515.2 | 533 | ctcagctgaccgcaccattattg | 386567 | - | see also 23137 line | | | |
| 52285 | MGC4645 | NM_024515.2 | 316 | gtggtcgaagtgactttagaatc | 386562 | - | see also 23137 line | | | |

Figure 46

Figure 46

| | | | | | | | | | |
|---|---|---|---|---|---|---|---|---|---|
| 52286 | MGC46496 | NM_174952.1 | 444 | tacacttggtccagcatactaca | 428788 | - | see also 23138 line | | |
| 52287 | MGC46496 | NM_174952.1 | 651 | ttcatttatcccacgactatatg | 428793 | - | see also 23138 line | | |
| 52288 | MGC46534 | NM_153340.2 | 1062 | ctcctccgagagtgaattggatg | 422802 | - | see also 9989 line | | |
| 52289 | MGC46534 | NM_153340.2 | 565 | cccatggctcccccttctaaaga | 422798 | - | see also 9989 line | | |
| 52290 | MGC4659 | NM_025268.2 | 889 | ggccatcttcgtcggcaaaaacg | 394628 | - | - | - | - |
| 52291 | MGC4659 | NM_025268.2 | 381 | tcctgtggttcctttacatcttc | 394627 | - | see also 52290 line | | |
| 52292 | MGC46719 | NM_153713.1 | 353 | gggctatggccgagtgaatgtgg | 423843 | - | see also 23140 line | | |
| 52293 | MGC46719 | NM_153713.1 | 614 | aagaatcactgatgagttcatcg | 423853 | - | see also 23140 line | | |
| 52294 | MGC46732 | NM_153714.1 | 265 | gggtccacgagacttaacatttc | 423867 | - | see also 23141 line | | |
| 52295 | MGC46732 | NM_153714.1 | 369 | gagttcatctacgcaaaagttag | 423873 | - | see also 23141 line | | |
| 52296 | MGC4701 | NM_024511.3 | 1674 | aaggcatcatttgaacttctaca | 386503 | - | see also 23142 line | | |
| 52297 | MGC4701 | NM_024511.3 | 1045 | atgaagttacacaattgatgatg | 386482 | - | see also 23142 line | | |
| 52298 | MGC4707 | NM_024113.2 | 330 | gggcaaaaatggcgatttgctga | 386074 | - | see also 23143 line | | |
| 52299 | MGC4707 | NM_024113.2 | 789 | ctcaaagcaccgtggaatcaacc | 386078 | - | see also 23143 line | | |
| 52300 | MGC4707 | NM_024113.2 | 277 | ccccacaatagggtatcattttt | 386072 | - | see also 23143 line | | |
| 52301 | MGC4734 | NM_145051.2 | 184 | cacgttccataccccaaaatgc | 413202 | - | see also 23145 line | | |
| 52302 | MGC4734 | NM_145051.2 | 587 | aaccctcagttccgcatctttgc | 413203 | - | see also 23145 line | | |
| 52303 | MGC47799 | NM_173545.1 | 737 | cagtggaggtaatgtaatccagg | 425797 | - | see also 10081 line | | |
| 52304 | MGC47799 | NM_173545.1 | 1245 | aagaggacatcctgcatgtatgg | 425809 | - | see also 10081 line | | |
| 52305 | MGC47869 | NM_175874.2 | 316 | agccgcagtatgaatactcaaat | 429392 | - | see also 23147 line | | |
| 52306 | MGC47869 | NM_175874.2 | 317 | gccgcagtatgaatactcaaatc | 429393 | - | see also 23147 line | | |
| 52307 | MGC4825 | NM_024122.2 | 722 | ctggggtttacgaggatatatag | 386143 | - | see also 23148 line | | |
| 52308 | MGC4825 | NM_024122.2 | 504 | gggttagacagctatgactatct | 386138 | - | see also 23148 line | | |
| 52309 | MGC48935 | NM_178536.2 | 196 | aacgctttcaccgcaacttttga | 431116 | - | see also 23149 line | | |
| 52310 | MGC48935 | NM_178536.2 | 207 | cgcaacttttgagctaagtgatg | 431118 | - | see also 23149 line | | |

Figure 46

| | | | | | | | |
|---|---|---|---|---|---|---|---|
| 52311 | MGC48972 | NM_173475.1 | 731 | cacggttccctagcctcttaaca | 424931 | - | see also 10069 line |
| 52312 | MGC48986 | NM_175881.2 | 375 | cacgggctacatagatcatgaca | 429490 | - | see also 23151 line |
| 52313 | MGC48986 | NM_175881.2 | 373 | tgcacgggctacatagatcatga | 429489 | - | see also 23151 line |
| 52314 | MGC48998 | NM_178550.2 | 134 | atggtcaggggacaattgtataa | 431176 | - | see also 23152 line |
| 52315 | MGC48998 | NM_178550.2 | 139 | caggggacaattgtataagcatt | 431178 | - | see also 23152 line |
| 52316 | MGC50104 | NM_198498.1 | 159 | gccggtcacgtcaggttactacg | 438605 | - | see also 23153 line |
| 52317 | MGC50104 | NM_198498.1 | 175 | tactacggtgtcagaagatcttt | 438606 | - | see also 23153 line |
| 52318 | MGC50104 | NM_198498.1 | 702 | ccctttcatgacggtgtcaaatg | 438610 | - | see also 23153 line |
| 52319 | MGC50372 | NM_173566.1 | 996 | gccttcctcgacccaaaaggtta | 426209 | - | see also 23154 line |
| 52320 | MGC50372 | NM_173566.1 | 1227 | gccatccgagaagctatccatat | 426213 | - | see also 23154 line |
| 52321 | MGC50372 | NM_173566.1 | 1230 | atccgagaagctatccatattgg | 426214 | - | see also 23154 line |
| 52322 | MGC50721 | NM_173806.2 | 380 | aggatatactcttcgagaatttt | 428056 | - | see also 23155 line |
| 52323 | MGC50721 | NM_173806.2 | 900 | gcccaactggtatcaaaggttgg | 428068 | - | see also 23155 line |
| 52324 | MGC50811 | NM_198559.1 | 262 | aactcggcatgctgacatactgc | 439505 | - | see also 23156 line |
| 52325 | MGC50811 | NM_198559.1 | 642 | ctctgctatttgacctatcaaaa | 439508 | - | see also 23156 line |
| 52326 | MGC50844 | NM_178562.2 | 481 | tgggtccctccgcgagaacatct | 431288 | - | see also 10198 line |
| 52327 | MGC50896 | NM_182553.1 | 376 | gcgggcacgcgagcgtttaaaaa | 434408 | - | see also 23158 line |
| 52328 | MGC50896 | NM_182553.1 | 377 | cgggcacgcgagcgtttaaaaaa | 434409 | - | see also 23158 line |
| 52329 | MGC50896 | NM_182553.1 | 378 | gggcacgcgagcgtttaaaaaac | 434410 | - | see also 23158 line |
| 52330 | MGC51025 | NM_178571.2 | 897 | ggcactccctgtggtattctaca | 431338 | - | see also 23159 line |

Figure 46

| | | | | | | | |
|---|---|---|---|---|---|---|---|
| 52331 | MGC51025 | NM_178571.2 | 899 | cactcccctgtggtattctacagc | 431339 | - | see also 23159 line |
| 52332 | MGC51029 | NM_173794.2 | 253 | cacagttcgggacctatggtaga | 427971 | - | see also 10114 line |
| 52333 | MGC51029 | NM_173794.2 | 392 | ttcttcagattgctagtcatagt | 427976 | - | see also 10114 line |
| 52334 | MGC51082 | NM_182498.2 | 506 | cccgagcattcctctgattcaga | 433961 | - | |
| 52335 | MGC51082 | NM_182498.2 | 592 | cactgacgacgatcctgaatatgatc | 433966 | - | see also 52334 line |
| 52336 | MGC51082 | NM_182498.2 | 589 | gaccactgacgatcctgaatatg | 433964 | - | see also 52334 line |
| 52337 | MGC52010 | NM_194326.1 | 223 | ctcagagtaaactgaagtttct | 436873 | - | |
| 52338 | MGC52022 | NM_198563.1 | 222 | gggccttcagcacgttaatgctca | 439530 | - | see also 10340 line |
| 52339 | MGC52022 | NM_198563.1 | 487 | tccctgcgttcgggcgaatatgg | 439537 | - | see also 10340 line |
| 52340 | MGC52057 | NM_194317.1 | 732 | cacgacgtcacctataaatcaga | 436797 | - | see also 23162 line |
| 52341 | MGC52057 | NM_194317.1 | 760 | gggcacccacgctgtatgtcagt | 436799 | - | see also 23162 line |
| 52342 | MGC5242 | NM_024033.1 | 631 | agtccgggagatcttttcagc | 385532 | - | see also 23163 line |
| 52343 | MGC52423 | NM_182517.1 | 316 | gcctgtgctggtaggaatttgtgc | 434055 | - | see also 23164 line |
| 52344 | MGC5306 | NM_024116.1 | 379 | gtgttcacgcaagtgattcatca | 386084 | - | see also 23166 line |
| 52345 | MGC5306 | NM_024116.1 | 382 | ttcacgcaagtgattcatcaagt | 386085 | - | see also 23166 line |
| 52346 | MGC5309 | NM_032286.1 | 93 | tcctcccagctcacaataggtgc | 399349 | - | see also 23167 line |
| 52347 | MGC5309 | NM_032286.1 | 329 | tgctggtcaagatttaccttta | 399354 | - | see also 23167 line |
| 52348 | MGC5309 | NM_032286.1 | 173 | accctttcgttcgttctttc | 399351 | - | see also 23167 line |
| 52349 | MGC5356 | NM_024059.2 | 910 | tgctgcccaaccgatcctataag | 385708 | - | see also 23168 line |
| 52350 | MGC5356 | NM_024059.2 | 914 | gcccaaccgatcctataagttca | 385710 | - | see also 23168 line |
| 52351 | MGC5384 | NM_030972.2 | 1549 | cacagctggctcgacatagaaga | 395901 | - | see also 23169 line |
| 52352 | MGC5384 | NM_030972.2 | 1554 | ctggctcgacatagaagaattca | 395904 | - | see also 23169 line |
| 52353 | MGC54289 | NM_178454.1 | 291 | ccccgaggacaaaggttatgtgc | 430777 | - | see also 10190 line |
| 52354 | MGC54289 | NM_178454.1 | 308 | atgtgcttcacatgatcactact | 430778 | - | see also 10190 line |
| 52355 | MGC5508 | NM_024092.1 | 169 | gcccagtcccgtcgagactttgc | 385953 | - | see also 23171 line |

Figure 46

| | | | | | | | |
|---|---|---|---|---|---|---|---|
| 52356 | MGC5508 | NM_024092.1 | 402 | gggactagctggtgattacctcg | 385956 | - | see also 23171 line |
| 52357 | MGC5509 | NM_024093.1 | 216 | cagagggatttgccgaagaatag | 385959 | - | see also 8589 line |
| 52358 | MGC5509 | NM_024093.1 | 220 | gggatttgccgaagaatagatgg | 385960 | - | see also 8589 line |
| 52359 | MGC5528 | NM_024094.1 | 927 | agcagcacgaatgctacttcaga | 385992 | - | see also 23173 line |
| 52360 | MGC5528 | NM_024094.1 | 1108 | gaggataatcaggaacgtttaa | 385997 | - | see also 23173 line |
| 52361 | MGC5576 | NM_024056.2 | 267 | gccctcagcgaactaagcaatat | 385685 | - | see also 23175 line |
| 52362 | MGC5576 | NM_024056.2 | 609 | gaggaccgttttcctatgtgtac | 385693 | - | see also 23175 line |
| 52363 | MGC5590 | NM_024058.1 | 245 | cacgtggccatgccattagcatc | 385700 | - | see also 23176 line |
| 52364 | MGC5590 | NM_024058.1 | 313 | ctcgcttggatcagcaagataaa | 385703 | - | see also 23176 line |
| 52365 | MGC57211 | NM_175878.2 | 783 | agctaccgccgatagaattcttc | 429476 | - | see also 23177 line |
| 52366 | MGC57211 | NM_175878.2 | 886 | gagcctacccgttttgttaatca | 429481 | - | see also 23177 line |
| 52367 | MGC57211 | NM_175878.2 | 614 | ctcggttctgttccacaattaat | 429472 | - | see also 23177 line |
| 52368 | MGC57858 | NM_178508.2 | 907 | ggctggtagacacctttgctttc | 430947 | - | - | | - |
| 52369 | MGC59868 | NM_198584.1 | 76 | cgcgagcacaacggtcctattca | 439814 | - | see also 23178 line |
| 52370 | MGC59868 | NM_198584.1 | 185 | tccgaccacttagtatcaagtat | 439822 | - | see also 23178 line |
| 52371 | MGC59868 | NM_198584.1 | 182 | ccctccgaccacttagtatcaag | 439821 | - | see also 23178 line |
| 52372 | MGC5987 | NM_138476.2 | 233 | gcctgaggtcctaaaacgattgc | 408357 | - | see also 23179 line |
| 52373 | MGC5987 | NM_138476.2 | 154 | tagaccctccgttccataagagc | 408356 | - | see also 23179 line |
| 52374 | MGC61550 | NM_182616.1 | 262 | agcgctactacgtgctgtatatc | 434707 | - | see also 23180 line |
| 52375 | MGC61550 | NM_182616.1 | 312 | ttcgacgccaagggaaatgaaat | 434709 | - | see also 23180 line |
| 52376 | MGC61571 | NM_182523.1 | 295 | ctggagttcttatggtagtaaaa | 434159 | - | see also 23181 line |
| 52377 | MGC61571 | NM_182523.1 | 193 | gacatgtcgaaaaagatgttttg | 434156 | - | see also 23181 line |
| 52378 | MGC61599 | NM_182505.3 | 538 | gacattgttattccgttgaataa | 433998 | - | see also 23182 line |
| 52379 | MGC61599 | NM_182505.3 | 503 | aacttgaagtttgcgcaaaatgt | 433997 | - | see also 23182 line |
| 52380 | MGC61633 | NM_198572.1 | 1167 | cccccttcccgaatgcataattc | 439714 | - | - | | - |

Figure 46

| | | | | | | | |
|---|---|---|---|---|---|---|---|
| 52381 | MGC61633 | NM_198572.1 | 1166 | tcccccttcccgaatgcataatt | 439713 | - | see also 52380 line |
| 52382 | MGC61633 | NM_198572.1 | 1165 | atccccttcccgaatgcataat | 439712 | - | see also 52380 line |
| 52383 | MGC61716 | NM_182501.1 | 818 | agcgcctgggacggtaccaaacc | 433975 | - | see also 23183 line |
| 52384 | MGC61716 | NM_182501.1 | 498 | ctccagttacctgcaaaagcttg | 433971 | - | see also 23183 line |
| 52385 | MGC64882 | NM_182627.1 | 1085 | tggcttcgtgtggtaatattttt | 434785 | - | see also 23184 line |
| 52386 | MGC64882 | NM_182627.1 | 1082 | ctgtggcttcgtgtggtaatatt | 434784 | - | see also 23184 line |
| 52387 | MGC7036 | NM_145058.1 | 841 | gtggttactagtgccaaaaatgc | 413340 | - | see also 23185 line |
| 52388 | MGC71745 | NM_198449.1 | 381 | ttccttggagagtcataacatat | 437825 | - | - |
| 52389 | MGC71745 | NM_198449.1 | 404 | cactgactgaacattctagtatg | 437828 | - | see also 52388 line |
| 52390 | MGC71745 | NM_198449.1 | 382 | tccttggagagtcataacatatc | 437826 | - | see also 52388 line |
| 52391 | MGC71806 | NM_198516.1 | 1915 | gacgcctcagaacgtgtactaca | 438945 | - | see also 10337 line |
| 52392 | MGC71806 | NM_198516.1 | 838 | tgggtgccgtaacctctcatttc | 438931 | - | see also 10337 line |
| 52393 | MGC8721 | NM_016127.3 | 310 | gacggacttagatattgcataca | 356351 | - | see also 6679 line |
| 52394 | MGC8721 | NM_016127.3 | 106 | ctggaacgaccctgacagaatgt | 356340 | - | see also 6679 line |
| 52395 | MGC874 | NM_016500.3 | 443 | gacccttaggatagatgtgttgg | 357890 | - | see also 23188 line |
| 52396 | MGC874 | NM_016500.3 | 647 | tcggaaccgggaaggctataaca | 357895 | - | see also 23188 line |
| 52397 | MGC8974 | NM_052940.3 | 669 | aacaggtatctcgtgatttcaga | 405441 | - | see also 9463 line |
| 52398 | MGC8974 | NM_052940.3 | 866 | agcaccagttcgagtgatgaaaa | 405449 | - | see also 9463 line |
| 52399 | MGC9084 | NM_033418.1 | 1101 | aagcttgtactaagtagtgaaaa | 403849 | - | see also 23190 line |
| 52400 | MGC9084 | NM_033418.1 | 651 | cagcatgttaagttatcagtagt | 403841 | - | see also 23190 line |
| 52401 | MGC955 | NM_024097.1 | 281 | gtcatagctgagcaaatccaaca | 386006 | - | see also 23191 line |
| 52402 | MGC955 | NM_024097.1 | 285 | tagctgagcaaatccaacatttg | 386007 | - | see also 23191 line |
| 52403 | MGC9712 | NM_152689.2 | 2037 | ggctgtgggaatcgctcttattt | 419818 | - | see also 23192 line |
| 52404 | MGC9850 | NM_152705.1 | 408 | ctcgaggttccgccagttactcc | 419919 | - | see also 23193 line |
| 52405 | MGC9850 | NM_152705.1 | 229 | tcctcttgctagcaccaataaaa | 419915 | - | see also 23193 line |
| 52406 | MGC9850 | NM_152705.1 | 235 | tgctagcaccaataaaagatttc | 419917 | - | see also 23193 line |
| 52407 | MGC:13379 | NM_016499.2 | 340 | tgccatgatggcctcctattacc | 357888 | - | see also 23194 line |

Figure 46

| | | | | | | | |
|---|---|---|---|---|---|---|---|
| 52408 | MIA2 | NM_054024.2 | 164 | gccgatacctgaacttcactaag | 405976 | - | see also 23195 line |
| 52409 | MIA2 | NM_054024.2 | 1342 | gacgaatctgatactataccata | 406000 | - | see also 23195 line |
| 52410 | MIB | NM_020774.1 | 365 | atcgctttaccgaattactaca | 378781 | - | see also 7952 line |
| 52411 | MINA53 | NM_032778.3 | 1115 | ctgttgctacagacgattaagt | 401436 | - | see also 9281 line |
| 52412 | MINA53 | NM_032778.3 | 867 | caggcccgtgcatgagtttatgc | 401428 | - | see also 9281 line |
| 52413 | MIR | NM_145021.2 | 355 | tgggacattcatgagtcattca | 412789 | - | see also 23198 line |
| 52414 | MIR | NM_145021.2 | 752 | gtccttgtatgtgctcattgaac | 412791 | - | see also 23198 line |
| 52415 | MIS12 | NM_024039.1 | 664 | ggccgttgaacaggttattctga | 385591 | - | see also 23199 line |
| 52416 | MIS12 | NM_024039.1 | 1013 | cagacgttgactttctttgatga | 385597 | - | see also 23199 line |
| 52417 | MIST1 | NM_177455.2 | 272 | ggcagcggatgcacaagctaaat | 429882 | - | see also 23200 line |
| 52418 | MIZF | NM_015517.3 | 1179 | gtccattaccgcaaagtacatg | 353671 | - | see also 23201 line |
| 52419 | MIZF | NM_015517.3 | 932 | tgcgcaaccatgtgaatcactat | 353664 | - | see also 23201 line |
| 52420 | MKLN1 | NM_013255.2 | 1031 | aacggaggcaaatctacacattg | 338446 | - | see also 5368 line |
| 52421 | MKLN1 | NM_013255.2 | 205 | aagctgaaaggcctgctatagt | 338412 | - | see also 5368 line |
| 52422 | MLR1 | NM_153686.4 | 866 | tgccttaatcgtcccattgaatg | 423499 | - | see also 10004 line |
| 52423 | MLR1 | NM_153686.4 | 867 | gccttaatcgtcccattgaatgg | 423500 | - | see also 10004 line |
| 52424 | MLZE | NM_031415.1 | 1615 | tacgaaatttccgaaatggtagg | 396739 | - | see also 23204 line |
| 52425 | MLZE | NM_031415.1 | 1038 | ttgggaacaatctgactatgttc | 396722 | - | see also 23204 line |
| 52426 | MMAA | NM_172250.1 | 648 | acccgaatgactgagttatcaag | 424380 | - | see also 23205 line |
| 52427 | MMAA | NM_172250.1 | 760 | aaggagcgggatatgacataatt | 424388 | - | see also 23205 line |
| 52428 | MMRP19 | NM_015957.1 | 521 | ttccgaggtattatagatatg | 355615 | - | see also 6589 line |
| 52429 | MMRP19 | NM_015957.1 | 335 | ttcgccatcgaagaagctaaaaa | 355606 | - | see also 6589 line |
| 52430 | MNS1 | NM_018365.1 | 311 | accgtgttcagcgcaagcaatt | 370861 | - | see also 23207 line |
| 52431 | MNS1 | NM_018365.1 | 1405 | gagcgtcgccaacaattccttgc | 370881 | - | see also 23207 line |
| 52432 | MO25 | NM_016289.2 | 1021 | ctcggtgaactactacagatag | 356911 | - | see also 6789 line |
| 52433 | MO25 | NM_016289.2 | 1012 | ctgaagcttctcggtgaactact | 356910 | - | see also 6789 line |
| 52434 | MOAP1 | NM_022151.3 | 411 | ccctccgaaagcgctattgattg | 382547 | - | see also 23209 line |
| 52435 | MOAP1 | NM_022151.3 | 767 | agggcatgatccggaaatgtgg | 382554 | - | see also 23209 line |
| 52436 | MOB | NM_147156.2 | 1825 | ggcgactctggtgtatccactgg | 415053 | - | see also 9807 line |
| 52437 | MOB | NM_147156.2 | 1773 | cacacgtcatgctaacacttac | 415050 | - | see also 9807 line |
| 52438 | MOB3B | NM_024761.2 | 994 | aaggagctagagcctttgaaaga | 390177 | - | see also 23210 line |
| 52439 | MOB3B | NM_024761.2 | 759 | ttggattgaggttcagatcaaca | 390174 | - | see also 23210 line |
| 52440 | MOB4A | NM_173468.1 | 610 | cgcctcttaggtttatgctca | 424882 | - | see also 23211 line |
| 52441 | MOB4A | NM_173468.1 | 563 | tcccgttcccaaagaatttcatg | 424881 | - | see also 23211 line |
| 52442 | moblak | NM_130807.2 | 741 | cactccgtttcccaagaacttcc | 406934 | - | see also 23212 line |
| 52443 | moblak | NM_130807.2 | 615 | ccgctggcaggatgagcataagt | 406932 | - | see also 23212 line |
| 52444 | MOCOS | NM_017947.1 | 997 | atcgcgctaaaacatggatttga | 364531 | - | see also 23213 line |

Figure 46

| | | | | | | | |
|---|---|---|---|---|---|---|---|
| 52445 | MOCOS | NM_017947.1 | 2339 | tgcgtttcgtgccaatatatt | 364556 | - | see also 23213 line |
| 52446 | MOCOS | NM_017947.1 | 2338 | ttgcgtttcgtgccaatattat | 364555 | - | see also 23213 line |
| 52447 | MOCS1 | NM_005942.1 | 1348 | ctccatgttcagggtctaagacc | 334543 | - | see also 23214 line |
| 52448 | MOCS1 | NM_005942.1 | 1361 | gtctaagacccagaatgagtttc | 334545 | - | see also 23214 line |
| 52449 | MONDOA | NM_014938.2 | 1338 | ctccccgtgttaccattagttc | 346221 | - | see also 23215 line |
| 52450 | MONDOA | NM_014938.2 | 1339 | tccccgtgttaccattagttcc | 346222 | - | see also 23215 line |
| 52451 | MONDOA | NM_014938.2 | 1748 | aaccacaagcgtgatcatgacg | 346225 | - | see also 23215 line |
| 52452 | MOPT | NM_194270.1 | 401 | ttcccaaatggggcaaagtatac | 436162 | - | see also 23216 line |
| 52453 | MOPT | NM_194270.1 | 343 | agcagtatatgaaggacaattta | 436160 | - | see also 23216 line |
| 52454 | MPN2 | NM_183062.2 | 466 | ctgaagacccgcattrgtgtttc | 435584 | - | see also 23217 line |
| 52455 | MPN2 | NM_183062.2 | 403 | gtgatctgcaccccacatatga | 435582 | - | see also 23217 line |
| 52456 | MR-1 | NM_015488.3 | 357 | accctaaaggccactcgaaaacc | 353507 | - | see also 6437 line |
| 52457 | MRGD | NM_198923.1 | 107 | ccgtggagtcagccctaaactat | 439994 | - | see also 23219 line |
| 52458 | MRGD | NM_198923.1 | 1014 | cacgtgggcaccaatgagatgg | 439998 | - | see also 23219 line |
| 52459 | MRPL10 | NM_145255.2 | 511 | aggagatggtacggatcttaagg | 413924 | - | see also 23220 line |
| 52460 | MRPL10 | NM_145255.2 | 432 | gaggattccagtaccaaaatct | 413923 | - | see also 23220 line |
| 52461 | MRPL28 | NM_006428.3 | 459 | aggcttatgggctcgactttac | 335545 | - | see also 23222 line |
| 52462 | MRPL28 | NM_006428.3 | 159 | cgcccactccgtgcactaagg | 335541 | - | see also 23222 line |
| 52463 | MRPL28 | NM_006428.3 | 460 | ggcttatgggctcgactttaca | 335546 | - | see also 23222 line |
| 52464 | MRPL38 | NM_032478.2 | 730 | ggccgacctaccgagagtattc | 400352 | - | see also 9212 line |
| 52465 | MRPL38 | NM_032478.2 | 731 | gccggacctaccgagagtatttcg | 400353 | - | see also 9212 line |
| 52466 | MRPL45 | NM_032351.3 | 831 | cggtttggccgttgatgtatgg | 399882 | - | see also 23224 line |
| 52467 | MRPL45 | NM_032351.3 | 830 | ccggtttggccgttgatgtatg | 399881 | - | see also 23224 line |
| 52468 | MRPL52 | NM_178336.1 | 375 | aaagcccacttccaagtcaata | 430713 | - | see also 23225 line |
| 52469 | MRPL53 | NM_053050.2 | 200 | ctgtcccttcgagaaaaacgtgg | 405852 | - | see also 23226 line |
| 52470 | MRPL53 | NM_053050.2 | 164 | tggtctgcggcctgtcaaacagg | 405851 | - | see also 23226 line |
| 52471 | MRPL54 | NM_172251.1 | 240 | tgccatgggctcaacatctaca | 424404 | - | see also 23227 line |
| 52472 | MRPL55 | NM_181441.1 | 340 | gtgcaccgcaggcttatgcacg | 432133 | - | see also 23228 line |
| 52473 | MRPS9 | NM_182640.1 | 710 | ttcggctgtagaaaagttattg | 434842 | - | see also 23229 line |
| 52474 | MRPS9 | NM_182640.1 | 223 | aacgttcctacctcaaaacgtga | 434824 | - | see also 23229 line |
| 52475 | MS4A12 | NM_017716.1 | 739 | aacaccacaaccaatatgtctgt | 361143 | - | see also 23230 line |
| 52476 | MS4A12 | NM_017716.1 | 595 | ctggtggatatgtgcatcaatgg | 361141 | - | see also 23230 line |
| 52477 | MS4A3 | NM_006138.3 | 572 | gtcaacggacctatgcaattaca | 334869 | - | see also 23231 line |
| 52478 | MS4A3 | NM_006138.3 | 574 | caccggacctatgcaattacatg | 334870 | - | see also 23231 line |
| 52479 | MSL3L1 | NM_006800.2 | 977 | cccagtcgcctttgttgaatcc | 336369 | - | transcription_regulator |
| 52480 | MSL3L1 | NM_006800.2 | 976 | tcccagtcgcctttgttgaatc | 336368 | - | see also 52479 line |

**Figure 46**

| | Gene | Accession | | Sequence | | | Note |
|---|---|---|---|---|---|---|---|
| 52481 | MSL3L1 | NM_006800.2 | 899 | aaggtgacttcgtctaaatttt | 336365 | - | see also 52479 line |
| 52482 | MTBP | NM_022045.2 | 1823 | gtcctcgggactcaatcacattg | 381734 | - | see also 8246 line |
| 52483 | MTBP | NM_022045.2 | 2240 | cccgattgaaacggagatctaaa | 381752 | - | see also 8246 line |
| 52484 | MTMR9 | NM_015458.3 | 566 | cgccatcgacaagcgatttgtag | 353239 | - | see also 6422 line |
| 52485 | MTMR9 | NM_015458.3 | 567 | gccatcgacaagcgatttgtagg | 353240 | - | see also 6422 line |
| 52486 | MTMR9 | NM_015458.3 | 1463 | caggaacattcgtggttttgagg | 353273 | - | see also 6422 line |
| 52487 | MUC13 | NM_033049.1 | 993 | gaaccttcggtgtgattattatg | 402725 | - | see also 23234 line |
| 52488 | MUC13 | NM_033049.1 | 567 | tccctgtgcagataattcgttat | 402714 | - | see also 23234 line |
| 52489 | MUC15 | NM_145650.2 | 1180 | tgcaacggaaccttatgatgtga | 414621 | - | see also 9792 line |
| 52490 | MUC15 | NM_145650.2 | 1126 | ttccatcggcgactttatgacg | 414617 | - | see also 9792 line |
| 52491 | MUC15 | NM_145650.2 | 1175 | gacaatgcaccggaaccttatga | 414620 | - | see also 9792 line |
| 52492 | MUC20 | NM_152673.1 | 983 | caccaagttacgtcaaagtctca | 419568 | - | see also 23236 line |
| 52493 | MUC20 | NM_152673.1 | 184 | cacgccgtctcggaaactcaaac | 419564 | - | see also 23236 line |
| 52494 | MUC7 | NM_152291.1 | 248 | caccagaagccgttcattagaaa | 415389 | - | see also 23237 line |
| 52495 | MUC7 | NM_152291.1 | 1004 | gcccaattaccaccacctaattc | 415397 | - | see also 23237 line |
| 52496 | MUF1 | NM_006369.3 | 1111 | gagatgaaggtccctcttattg | 335360 | - | see also 23238 line |
| 52497 | MUF1 | NM_006369.3 | 2689 | aggccttgcagattatgttagc | 335377 | - | see also 23238 line |
| 52498 | MXD3 | NM_031300.2 | 222 | ggggtcagtgcaacatgaact | 396591 | - | see also 23239 line |
| 52499 | my048 | NM_080390.2 | 604 | ctcagtacctcaagcaatataag | 406373 | - | see also 23240 line |
| 52500 | my048 | NM_080390.2 | 200 | ttcgaatcaaggaaagatagaca | 406370 | - | see also 23240 line |
| 52501 | my048 | NM_080390.2 | 684 | atggctagggtgcaggataaaag | 406374 | - | see also 23240 line |
| 52502 | MYCT1 | NM_025107.1 | 635 | gtcttcgagtgggcctttcaaca | 393675 | - | see also 23241 line |
| 52503 | MYCT1 | NM_025107.1 | 137 | tggatattatggctaataacaca | 393668 | - | see also 23241 line |
| 52504 | MYH14 | NM_024729.2 | 1290 | ttgctcaccctcgcatcaaagt | 389721 | - | see also 23242 line |
| 52505 | MYH14 | NM_024729.2 | 418 | acctccggagcggtactactcc | 389719 | - | see also 23242 line |
| 52506 | MYOZ1 | NM_021245.2 | 665 | agctggtcaggagttctcataca | 380886 | - | see also 23243 line |
| 52507 | MYOZ1 | NM_021245.2 | 1152 | tccaacaggccttctcaatcg | 380890 | - | see also 23243 line |
| 52508 | MYOZ3 | NM_133371.2 | 322 | agctgtcactacgcaacaacaga | 407192 | - | see also 23244 line |
| 52509 | MYOZ3 | NM_133371.2 | 763 | gccccaacgactaccgaaattc | 407194 | - | see also 23244 line |
| 52510 | N4BP1 | NM_014664.1 | 2232 | aacaaggcgtgatcctaatgtca | 341934 | - | see also 23245 line |
| 52511 | N4BP1 | NM_014664.1 | 2520 | aagaagttggacctcgattagagg | 341947 | - | see also 23245 line |
| 52512 | NAG73 | NM_032570.1 | 792 | cacctcacttaacgtctaacttc | 400765 | - | see also 23246 line |
| 52513 | NAG73 | NM_032570.1 | 555 | gtgtcgtaactggaattaagttg | 400760 | - | see also 23246 line |
| 52514 | NALP10 | NM_176821.2 | 594 | cccaggccgtttgattatgtct | 429822 | - | see also 23247 line |
| 52515 | NALP10 | NM_176821.2 | 596 | caggccgtttgattatgtcttt | 429823 | - | see also 23247 line |
| 52516 | NALP4 | NM_134444.1 | 2485 | aacaagagcgttgcgctatctaga | 407339 | - | see also 23248 line |
| 52517 | NALP4 | NM_134444.1 | 2740 | acggattgccgcttagagattct | 407345 | - | see also 23248 line |

Figure 46

| | | | | | | | | |
|---|---|---|---|---|---|---|---|---|
| 52518 | NALP8 | NM_176811.2 | 2506 | ttcaaggtaacgggcatctaaag | 429670 | - | see also 23249 line |
| 52519 | NALP8 | NM_176811.2 | 1885 | gggtcccgcagttattctactgt | 429657 | - | see also 23249 line |
| 52520 | NAPE-PLD | NM_198990.1 | 1102 | tacggacttaacgctgaagattt | 440339 | - | see also 10351 line |
| 52521 | NAPE-PLD | NM_198990.1 | 497 | gtcgttccccgtgcacaataagt | 440322 | - | see also 10351 line |
| 52522 | NAPE-PLD | NM_198990.1 | 528 | tccaatagatgcggtccttatca | 440326 | - | see also 10351 line |
| 52523 | NARFL | NM_022493.1 | 201 | ggccaaggtctcgctaaacgact | 383493 | - | see also 23251 line |
| 52524 | NARFL | NM_022493.1 | 249 | ctccgcagagaccgtgcttatca | 383494 | - | see also 23251 line |
| 52525 | NARG2 | NM_024611.2 | 2091 | aacctatcaaacgagtatataaa | 387937 | - | see also 23252 line |
| 52526 | NARG2 | NM_024611.2 | 923 | gacgatatagctaccattgaaac | 387892 | - | see also 23252 line |
| 52527 | NCDN | NM_014284.1 | 1832 | ccctcgcttctgtgcaagtattt | 340128 | - | see also 23253 line |
| 52528 | NCDN | NM_014284.1 | 1126 | aaccgtgccccctgaatgctacc | 340123 | - | see also 23253 line |
| 52529 | NCDN | NM_014284.1 | 1483 | ggccataggggctgttatccact | 340126 | - | see also 23253 line |
| 52530 | NDE1 | NM_017668.1 | 244 | gacgcagctgcaacaaattgaaa | 360484 | - | see also 23254 line |
| 52531 | NDE1 | NM_017668.1 | 747 | accgaggacccagctcaagttta | 360488 | - | see also 23254 line |
| 52532 | NDEL1 | NM_030808.2 | 1037 | agcatcacgaaaatcctatattt | 395252 | - | see also 8953 line |
| 52533 | NDEL1 | NM_030808.2 | 1029 | aaggaccaagcatcacgaaaatc | 395249 | - | see also 8953 line |
| 52534 | NDFIP1 | NM_030571.2 | 360 | gagcgcagcatattttgactaca | 394677 | - | see also 23256 line |
| 52535 | NDFIP1 | NM_030571.2 | 704 | ttgtcaggttttccacctatttc | 394682 | - | see also 23256 line |
| 52536 | NDNL2 | NM_138704.2 | 834 | ccccgtcgactacgaattccagt | 408669 | - | see also 23257 line |
| 52537 | NDNL2 | NM_138704.2 | 630 | cactacgggcctcctgatgatcg | 408666 | - | see also 23257 line |
| 52538 | NDUFS7 | NM_024407.3 | 490 | gaggctactaccactattcctac | 386364 | - | see also 23258 line |
| 52539 | NDUFS7 | NM_024407.3 | 538 | tcgtgcccgtggacatctacatc | 386365 | - | see also 23258 line |
| 52540 | NDUFV3 | NM_021075.2 | 1627 | ggcaatcgtggaagatcagatac | 380512 | - | see also 23259 line |
| 52541 | NDUFV3 | NM_021075.2 | 1800 | tgcctgctgagccgtttgacaac | 380516 | - | see also 23259 line |
| 52542 | NECAB2 | NM_019065.2 | 225 | cgccgtgcggacaaaaatgatga | 374882 | - | see also 23260 line |
| 52543 | NECAB2 | NM_019065.2 | 319 | atctctttcacacgattgactct | 374887 | - | see also 23260 line |
| 52544 | NECAB2 | NM_019065.2 | 224 | ccgccgtgcggacaaaaatgatg | 374881 | - | see also 23260 line |
| 52545 | NEDD1 | NM_152905.2 | 750 | cagagtccataccataactttga | 421635 | - | see also 23261 line |
| 52546 | NEDD1 | NM_152905.2 | 658 | ctgttcggcacttgaagtactcc | 421629 | - | see also 23261 line |
| 52547 | NEFH | NM_021076.2 | 1170 | cgcccagctgcgagaataccagg | 380522 | - | see also 23262 line |
| 52548 | NEFH | NM_021076.2 | 2570 | aagcccctgccacaccaaaaaca | 380525 | - | see also 23262 line |
| 52549 | NEGR1 | NM_173808.1 | 219 | ggctgaaccggtcaagtattatt | 428167 | - | see also 10116 line |
| 52550 | NEGR1 | NM_173808.1 | 170 | acggcggtgcttaggtgttattt | 428162 | - | see also 10116 line |
| 52551 | NELF | NM_015537.2 | 237 | gccggaaacgggagaatgattcc | 353757 | - | see also 23264 line |

Figure 46

| 52552 | NELF | NM_015537.2 | 570 | ctgagtacatccccactatcatc | 353760 | - | see also 23264 line | | |
|-------|------|-------------|-----|-------------------------|--------|---|---------------------|---|---|
| 52553 | NES | NM_006617.1 | 888 | agcggctgcgggctactgaaaag | 335944 | - | see also 23265 line | | |
| 52554 | NES | NM_006617.1 | 2573 | agcgtagaggcagtaaaatcttt | 335972 | - | see also 23265 line | | |
| 52555 | NES | NM_006617.1 | 891 | ggctgcgggctactgaaaagttc | 335946 | - | see also 23265 line | | |
| 52556 | NESH | NM_016428.2 | 589 | gactactgcgaggacaactatgt | 357447 | - | - | - | - |
| 52557 | NETO1 | NM_138966.2 | 557 | accgtcttgggagtgcaaatttg | 409290 | - | see also 9647 line | | |
| 52558 | NETO1 | NM_138966.2 | 479 | ccgggaatgcatctacatcatag | 409287 | - | see also 9647 line | | |
| 52559 | NETO2 | NM_018092.3 | 1224 | gtcggcttagcaggtttcgaatg | 366607 | - | see also 23267 line | | |
| 52560 | NETO2 | NM_018092.3 | 1225 | tcggcttagcaggtttcgaatgc | 366608 | - | see also 23267 line | | |
| 52561 | NEUROD2 | NM_006160.2 | 1209 | cacgggccacgggctagtcttcg | 334905 | - | see also 4263 line | | |
| 52562 | NEUROD2 | NM_006160.2 | 1146 | gcccgaccacgagaaaagctacc | 334904 | - | see also 4263 line | | |
| 52563 | NEUROG2 | NM_024019.2 | 1058 | ccgggtcagacatggactattgg | 385477 | - | see also 23269 line | | |
| 52564 | NEUROG2 | NM_024019.2 | 1022 | cccctacagctgcactttatcg | 385476 | - | see also 23269 line | | |
| 52565 | NFAM1 | NM_145912.3 | 792 | accgcatagattcgaagatgatg | 414963 | - | see also 23270 line | | |
| 52566 | NFAM1 | NM_145912.3 | 225 | ctgcaggatcacctatccataca | 414955 | - | see also 23270 line | | |
| 52567 | NFKBIB | NM_002503.2 | 44 | gtcgcgtgcttgggaaaagctgc | 330985 | - | see also 23271 line | | |
| 52568 | NFKBIB | NM_002503.2 | 991 | cggagacgagggcgatgaatacg | 330990 | - | see also 23271 line | | |
| 52569 | NFKBIL1 | NM_005007.2 | 974 | ggcagcctctggcgatttggtga | 333365 | - | see also 23272 line | | |
| 52570 | NFKBIL1 | NM_005007.2 | 471 | gtccctccgccatgggaataaag | 333364 | - | see also 23272 line | | |
| 52571 | NFKBIL2 | NM_013432.3 | 708 | agcgagtgctgcgtggttattgc | 338911 | - | see also 23273 line | | |
| 52572 | NFKBIL2 | NM_013432.3 | 2778 | cccatccgggttcgagttcaagt | 338918 | - | see also 23273 line | | |
| 52573 | NFRKB | NM_006165.2 | 2084 | ctggatcggctacattacgaaaa | 334946 | - | see also 4268 line | | |
| 52574 | NFRKB | NM_006165.2 | 1064 | atcatgactcgagtaaatgctgg | 334931 | - | see also 4268 line | | |
| 52575 | NGEF | NM_019850.1 | 1086 | ccgaggcgtcctactacaagagt | 375545 | - | see also 23275 line | | |
| 52576 | NGEF | NM_019850.1 | 1618 | gagccgcacggaacagatgatca | 375548 | - | see also 23275 line | | |
| 52577 | NGLY1 | NM_018297.2 | 967 | gtggcgagtgggccaattgtttt | 369923 | - | see also 7372 line | | |
| 52578 | NGLY1 | NM_018297.2 | 968 | tggcgagtgggccaattgtttta | 369924 | - | see also 7372 line | | |
| 52579 | NGX6 | NM_016446.2 | 1726 | cagatgcgctcacctatggattc | 357485 | - | see also 23277 line | | |
| 52580 | NGX6 | NM_016446.2 | 1337 | ctccgtgcgccaggaaaacgtga | 357481 | - | see also 23277 line | | |
| 52581 | NHLRC1 | NM_198586.2 | 595 | gcgatcgctccatcaaagtgttt | 439854 | - | see also 23278 line | | |
| 52582 | NHLRC1 | NM_198586.2 | 458 | gaggcgtgtcaagattttttgact | 439849 | - | see also 23278 line | | |
| 52583 | NHS | NM_198270.2 | 5010 | tgctccccacgagttaatgcaga | 437374 | - | see also 23279 line | | |
| 52584 | NHS | NM_198270.2 | 5125 | tccgctgccggctgtacaatacg | 437376 | - | see also 23279 line | | |
| 52585 | NICE-3 | NM_015449.1 | 470 | gccggcatccccgttccttaatg | 353083 | - | see also 23280 line | | |
| 52586 | NICE-3 | NM_015449.1 | 393 | aagttgctacaactatctgtata | 353080 | - | see also 23280 line | | |
| 52587 | NICN1 | NM_032316.2 | 340 | atcgctgttcaagcatcagatgc | 399700 | - | see also 23281 line | | |
| 52588 | NICN1 | NM_032316.2 | 382 | atcggagctacgcctgattctgc | 399701 | - | see also 23281 line | | |

Figure 46

| | | | | | | | | | |
|---|---|---|---|---|---|---|---|---|---|
| 52589 | NIF3L1 | NM_021824.1 | 840 | ttcccggaacaaacaactttatc | 381084 | - | see also 23282 line | | |
| 52590 | NIF3L1 | NM_021824.1 | 665 | accgagtagaattcaacgttaac | 381074 | - | see also 23282 line | | |
| 52591 | NIF3L1 | NM_021824.1 | 841 | tcccggaacaaacaactttatca | 381085 | - | see also 23282 line | | |
| 52592 | NIF3L1BP1 | NM_025075.1 | 529 | gagacggaaacagtttcatgttc | 393503 | - | see also 8849 line | | |
| 52593 | NIPA | NM_016478.2 | 591 | tagtgaattcctagatcgttttc | 357715 | - | see also 23283 line | | |
| 52594 | NIPA | NM_016478.2 | 653 | ggccggaggacttgaaaactatg | 357717 | - | see also 23283 line | | |
| 52595 | NIPA | NM_016478.2 | 162 | cccgcttgaggctctctatgaat | 357702 | - | see also 23283 line | | |
| 52596 | NIPA2 | NM_030922.2 | 429 | aaggtggccatgcatatcttaag | 395458 | - | see also 23284 line | | |
| 52597 | NIPA2 | NM_030922.2 | 430 | aggtggccatgcatatcttaagg | 395459 | - | see also 23284 line | | |
| 52598 | NIPSNAP1 | NM_003634.1 | 471 | aagatccagtttcacaatgtaaa | 331851 | - | see also 2452 line | | |
| 52599 | NIPSNAP1 | NM_003634.1 | 381 | tggttccgctccctctttgttca | 331849 | - | see also 2452 line | | |
| 52600 | NIPSNAP3A | NM_015469.1 | 606 | ggcagttcatgctcatgtcaatc | 353414 | - | - | - | - |
| 52601 | NIPSNAP3A | NM_015469.1 | 538 | gtctatgaactggccacttttca | 353413 | - | see also 52600 line | | |
| 52602 | NIPSNAP3B | NM_018376.2 | 221 | tgcgttcatggaaaatcttaaga | 370918 | - | see also 23286 line | | |
| 52603 | NIPSNAP3B | NM_018376.2 | 220 | atgcgttcatggaaaatcttaag | 370917 | - | see also 23286 line | | |
| 52604 | NJMU-R1 | NM_022344.1 | 303 | ttcattgctaagcgtctttcaag | 382607 | - | see also 23287 line | | |
| 52605 | NJMU-R1 | NM_022344.1 | 443 | aacgatagactctgaacgtaacc | 382611 | - | see also 23287 line | | |
| 52606 | NKAP | NM_024528.1 | 717 | aagaagtctagccgttcaaaaga | 386707 | - | see also 23288 line | | |
| 52607 | NKAP | NM_024528.1 | 1258 | ctgtgcgactgcgaaaagagaac | 386712 | - | see also 23288 line | | |
| 52608 | NKAP | NM_024528.1 | 1256 | ggctgtgcgactgcgaaaagaga | 386711 | - | see also 23288 line | | |
| 52609 | NKD2 | NM_033120.2 | 383 | gacgttcacgctctatgactttg | 403371 | - | see also 23289 line | | |
| 52610 | NKD2 | NM_033120.2 | 384 | acgttcacgctctatgactttga | 403372 | - | see also 23289 line | | |
| 52611 | NLGN1 | NM_014932.2 | 2090 | tccctcaagacacgaaattcatt | 346023 | - | see also 6115 line | | |
| 52612 | NLGN1 | NM_014932.2 | 1859 | ttggttcacctacgtacttctat | 346015 | - | see also 6115 line | | |
| 52613 | NMES1 | NM_032413.2 | 350 | gtgatccttgatcgaaaaaaaaa | 400207 | - | - | - | - |
| 52614 | NOB1P | NM_014062.1 | 203 | gtgcggctggtgactgagttttc | 339504 | - | see also 23292 line | | |
| 52615 | NOB1P | NM_014062.1 | 310 | tggggtgtctcacctaaaacaag | 339507 | - | see also 23292 line | | |
| 52616 | NOB1P | NM_014062.1 | 311 | ggggtgtctcacctaaaacaaga | 339508 | - | see also 23292 line | | |
| 52617 | NOD9 | NM_024618.2 | 394 | accacggaagctcggtagatagc | 388057 | - | see also 23293 line | | |
| 52618 | NOD9 | NM_024618.2 | 2618 | ggcaggtgttgccgtgctaatgg | 388066 | - | see also 23293 line | | |
| 52619 | NOL6 | NM_022917.4 | 611 | ctgcatccgaccagacatcaatg | 384865 | - | see also 23294 line | | |
| 52620 | NOL6 | NM_022917.4 | 1923 | atgtcccagaagcgccttattcc | 384876 | - | see also 23294 line | | |
| 52621 | NOPE | NM_020962.1 | 2179 | cagatctctggctacaaactata | 380302 | - | see also 23295 line | | |

Figure 46

| | | | | | | | |
|---|---|---|---|---|---|---|---|
| 52622 | NOPE | NM_020962.1 | 3365 | aaggccggagtcactccaaaga | 380313 | - | see also 23295 line |
| 52623 | NOR1 | NM_145047.2 | 1000 | cggattccggttgaatctctta | 413166 | - | see also 23296 line |
| 52624 | NOR1 | NM_145047.2 | 593 | atccgagtatccatgtttctaaa | 413156 | - | see also 23296 line |
| 52625 | NOSIP | NM_015953.3 | 231 | ggctatgggaccccagaacattcg | 355599 | - | see also 23297 line |
| 52626 | NOSTRIN | NM_052946.2 | 252 | agcaataaaacgacttatcaag | 405468 | - | see also 9464 line |
| 52627 | NOSTRIN | NM_052946.2 | 1141 | aaggagcatactcatagctatgt | 405492 | - | see also 9464 line |
| 52628 | 1.Nov | NM_144663.1 | 595 | aactgtatggatcgtgttctact | 411133 | - | see also 23299 line |
| 52629 | 1.Nov | NM_144663.1 | 523 | gaccgcactgattacaaaacact | 411129 | - | see also 23299 line |
| 52630 | NPD007 | NM_020684.2 | 805 | gaggatatccgttcaagtacg | 377974 | - | see also 23300 line |
| 52631 | NPD007 | NM_020684.2 | 318 | caggctggtaacctacatcttg | 377965 | - | see also 23300 line |
| 52632 | NPHP3 | NM_153240.3 | 2883 | gaggacaacatgagttgcttagc | 422535 | - | see also 23301 line |
| 52633 | NPHP3 | NM_153240.3 | 1605 | taccaacgccttaatgatctagt | 422495 | - | see also 23301 line |
| 52634 | NPL | NM_030769.1 | 585 | gacttcggcaatgtgttgatca | 394926 | - | see also 23302 line |
| 52635 | NPL | NM_030769.1 | 483 | gccttgacagggtaaagattcg | 394924 | - | see also 23302 line |
| 52636 | NPL | NM_030769.1 | 90 | atcacgccaatgactgagaatgg | 394914 | - | see also 23302 line |
| 52637 | NPL4 | NM_017921.1 | 567 | gccattcgatgaggactatctaa | 363999 | - | see also 23303 line |
| 52638 | NPL4 | NM_017921.1 | 1102 | accgtccgctacagtcgaaataa | 364011 | - | see also 23303 line |
| 52639 | NPL4 | NM_017921.1 | 556 | gtccctcagagccattcgatga | 363998 | - | see also 23303 line |
| 52640 | NPR2L | NM_006545.3 | 159 | ggctgccgcatcgaatgcatatt | 335733 | - | see also 23304 line |
| 52641 | NPR2L | NM_006545.3 | 163 | gccgcatcgaatgcatattcttc | 335735 | - | see also 23304 line |
| 52642 | NRAP | NM_006175.2 | 1141 | cacgaactcgctagtgacataaa | 334995 | - | see also 23305 line |
| 52643 | NRAP | NM_006175.2 | 3132 | cagtgagacgcgttataaggaat | 335056 | - | see also 23305 line |
| 52644 | NRCAM | NM_005010.1 | 1999 | atgttaaacagcccgaatatgc | 333426 | - | see also 3503 line |
| 52645 | NRCAM | NM_005010.1 | 1050 | ccgcggaagactatatctgttatg | 333393 | - | see also 3503 line |
| 52646 | NRPS998 | NM_181806.1 | 1361 | tcccgatgagcttgtattgatc | 433579 | - | see also 10254 line |
| 52647 | NRPS998 | NM_181806.1 | 435 | tacagggataccgaagattgtca | 433537 | - | see also 10254 line |
| 52648 | NS | NM_014366.3 | 781 | gccattcggttggagtaattgg | 340378 | - | see also 23308 line |
| 52649 | NS | NM_014366.3 | 60 | gacctgccataagcggtataaaa | 340361 | - | see also 23308 line |
| 52650 | NS | NM_014366.3 | 61 | aacctgccataagcggtataaaat | 340362 | - | see also 23308 line |
| 52651 | NS3TP2 | NM_023927.1 | 702 | ctcggtaaccctaataagagaaa | 385408 | - | see also 8553 line |
| 52652 | NS5ATP13TP2 | NM_178507.2 | 593 | gccaagtccggcgaaaaatcc | 430945 | - | - |
| 52653 | NS5ATP13TP2 | NM_178507.2 | 915 | acccctgccatgtcaagtactgc | 430946 | - | see also 52652 line |
| 52654 | NSE1 | NM_145175.1 | 298 | gggaccgtcgggattgaaaag | 413752 | - | see also 9776 line |
| 52655 | NSE1 | NM_145175.1 | 297 | ggggacccgtcggggattgaaaa | 413751 | - | see also 9776 line |
| 52656 | NSFL1C | NM_016143.3 | 1968 | gactactttccgaacaaagagc | 356455 | - | see also 6693 line |

Figure 46

| | | | | | | | |
|---|---|---|---|---|---|---|---|
| 52657 | NSPC1 | NM_032673.1 | 469 | gcccactactatcgctatgatga | 401207 | - | see also 23311 line |
| 52658 | NSPC1 | NM_032673.1 | 612 | gtgtcaccgctgatgctaaaac | 401209 | - | see also 23311 line |
| 52659 | NT5C2L1 | NM_152729.2 | 1053 | ttcccagctcgtcactatagtaa | 420160 | - | see also 23312 line |
| 52660 | NT5C2L1 | NM_152729.2 | 1054 | tcccagctcgtcactatagtaat | 420161 | - | see also 23312 line |
| 52661 | NUDT12 | NM_031438.2 | 414 | ttggttcctaacgaatgaagtgg | 396973 | - | see also 23313 line |
| 52662 | NUDT12 | NM_031438.2 | 921 | ctggcacagtcgatacaagtttt | 396993 | - | see also 23313 line |
| 52663 | NUDT13 | NM_015901.3 | 761 | ttctggctgcccctaagttagc | 355404 | - | see also 23314 line |
| 52664 | NUDT13 | NM_015901.3 | 261 | cccggcacagcctgttagagttg | 355389 | - | see also 23314 line |
| 52665 | NUDT14 | NM_177533.2 | 201 | gcgtgacgttcttattcaac | 430275 | - | see also 23315 line |
| 52666 | NUDT14 | NM_177533.2 | 254 | cagttccggccagctgtgtatgc | 430276 | - | see also 23315 line |
| 52667 | NUDT15 | NM_018283.1 | 583 | ttctgggggactgcgttgtttaaa | 369693 | - | see also 23316 line |
| 52668 | NUDT15 | NM_018283.1 | 585 | ctggggactgc-gttgtttaaaag | 369694 | - | see also 23316 line |
| 52669 | NUDT8 | NM_181843.1 | 320 | ggcctgctgcggcctgtgtatga | 433805 | - | see also 23317 line |
| 52670 | NUP133 | NM_018230.2 | 2409 | ggcatccgaacggtaataatacg | 368842 | - | see also 23318 line |
| 52671 | NUP133 | NM_018230.2 | 2223 | ttgagggatgcacctatggattc | 368833 | - | see also 23318 line |
| 52672 | NUP210 | NM_024923.2 | 4673 | gggatccgtgacgtttactatg | 392258 | - | see also 8807 line |
| 52673 | NUP210 | NM_024923.2 | 4672 | tgggatccgtgacgtttactat | 392257 | - | see also 8807 line |
| 52674 | NUPL1 | NM_014089.2 | 477 | caccatttgctctacctattacc | 339538 | - | see also 5488 line |
| 52675 | NUPL1 | NM_014089.2 | 1000 | gtcgttggctgccaatggaatac | 339552 | - | see also 5488 line |
| 52676 | NURIT | NM_152719.1 | 257 | cccgaggctccacaacttgtaca | 420076 | - | see also 23321 line |
| 52677 | NURIT | NM_152719.1 | 118 | aacttctgcataggacctataccc | 420072 | - | see also 23321 line |
| 52678 | NURIT | NM_152719.1 | 256 | tcccgaggctccacaacttgtac | 420075 | - | see also 23321 line |
| 52679 | NX17 | NM_020665.2 | 487 | atgtaaccagagggtatcattc | 377885 | - | see also 23322 line |
| 52680 | NX17 | NM_020665.2 | 870 | aagggaggggcatattaatgatgc | 377899 | - | see also 23322 line |
| 52681 | NXN | NM_022463.3 | 891 | aaccggctgacggaatccaagg | 382993 | - | see also 23323 line |
| 52682 | NXN | NM_022463.3 | 1226 | gactgactccctgcgagattaca | 382997 | - | see also 23323 line |
| 52683 | NXPH1 | NM_152745.1 | 1237 | ttgttaaaacgggcaagtttaag | 420423 | - | see also 23324 line |
| 52684 | NXPH1 | NM_152745.1 | 1421 | tacaaaaatcgtggaatttgact | 420432 | - | see also 23324 line |
| 52685 | NXPH1 | NM_152745.1 | 1445 | ggcacaacaaacgtgattgatg | 420433 | - | see also 23324 line |
| 52686 | NY-REN-41 | NM_080654.1 | 683 | tcctattacttcattacctag | 406529 | - | - |
| 52687 | NY-REN-41 | NM_080654.1 | 186 | tgccgctgagctgcagcaattcc | 406526 | - | see also 52686 line |
| 52688 | NY-REN-41 | NM_080654.1 | 461 | gtgtggttattggcaaagaaaa | 406528 | - | see also 52686 line |
| 52689 | NY-REN-58 | NM_016122.1 | 920 | cagcaaaacgagtgaacaacttg | 356299 | - | see also 6676 line |

Figure 46

| | | | | | | | |
|---|---|---|---|---|---|---|---|
| 52690 | NYD-SP12 | NM_031955.2 | 1092 | ctgaatcttctcggttatgtac | 397509 | - | see also 9060 line |
| 52691 | NYD-SP12 | NM_031955.2 | 163 | tgcagtgaataggatctatcatg | 397493 | - | see also 9060 line |
| 52692 | NYD-SP14 | NM_031956.1 | 1017 | tacttgggcttagcacacttagc | 397535 | - | see also 23327 line |
| 52693 | NYD-SP14 | NM_031956.1 | 396 | atgctgcgagatggttatcataa | 397526 | - | see also 23327 line |
| 52694 | NYD-SP17 | NM_032600.2 | 692 | tgctccacgatacattgtctaga | 401107 | - | see also 23328 line |
| 52695 | NYD-SP17 | NM_032600.2 | 697 | cacgatacattgtctagagattc | 401108 | - | see also 23328 line |
| 52696 | NYD-SP18 | NM_032599.1 | 620 | ctggatccgactggttcaaattc | 401083 | - | see also 23329 line |
| 52697 | NYD-SP18 | NM_032599.1 | 228 | ctctgccgggttgttcattctcc | 401080 | - | see also 23329 line |
| 52698 | NYD-SP20 | NM_032598.2 | 986 | atgaagtattggccgtgaacatgc | 401065 | - | see also 23330 line |
| 52699 | NYD-SP20 | NM_032598.2 | 1063 | acctggcccatattattcgaaga | 401068 | - | see also 23330 line |
| 52700 | NYD-SP21 | NM_032597.2 | 102 | gggcaactcacgtcatcactata | 400991 | - | see also 23331 line |
| 52701 | NYD-SP21 | NM_032597.2 | 110 | cacgtcatcactataaaaccaa | 400993 | - | see also 23331 line |
| 52702 | NYD-SP26 | NM_033122.2 | 1015 | ttgttgtccctgcatcaatagc | 403455 | - | see also 23332 line |
| 52703 | NYD-SP26 | NM_033122.2 | 360 | ctccataacaagagactctatta | 403439 | - | see also 23332 line |
| 52704 | NYD-SP28 | NM_033124.2 | 1061 | accggtatatccgtaatgacaag | 403482 | - | see also 23333 line |
| 52705 | NYD-SP28 | NM_033124.2 | 1062 | ccggtatatccgtaatgacaagg | 403483 | - | see also 23333 line |
| 52706 | NYD-SP29 | NM_145172.1 | 1402 | tccgagtgatcctaatatcattg | 413678 | - | see also 23334 line |
| 52707 | NYD-SP29 | NM_145172.1 | 1397 | ttctgtccgagtgatcctaatat | 413676 | - | see also 23334 line |
| 52708 | NYD-SP29 | NM_145172.1 | 1252 | agccgtgcgacttcttttgaag | 413669 | - | see also 23334 line |
| 52709 | NYD-TSP1 | NM_032567.2 | 847 | gtccttaacgaaactcaacaaag | 400743 | - | see also 23335 line |
| 52710 | NYD-TSP1 | NM_032567.2 | 793 | aacaccgcacaagttttgcaag | 400741 | - | see also 23335 line |
| 52711 | NYD-TSP1 | NM_032567.2 | 1118 | aactgagccatgacacctattca | 400750 | - | see also 23335 line |
| 52712 | OAZ | NM_015069.1 | 1878 | gaccacgtgacccactatgtgt | 347735 | - | see also 6220 line |
| 52713 | OAZ | NM_015069.1 | 2910 | ttccacgcttgagctcaagatcc | 347740 | - | see also 6220 line |
| 52714 | OCIA | NM_017830.1 | 330 | atgttgattactcaaggattaat | 362849 | - | see also 7158 line |
| 52715 | OCIA | NM_017830.1 | 541 | ctgggcactattatccaaaagtca | 362857 | - | see also 7158 line |
| 52716 | OCSP | NM_031945.2 | 301 | ctgcgtcaagtatctgatcttcc | 397465 | - | see also 23338 line |
| 52717 | OCSP | NM_031945.2 | 293 | gggagcagctgcgtcaagtatct | 397463 | - | see also 23338 line |
| 52718 | ODF3 | NM_053280.3 | 392 | caccaacaacaggcttcatgaag | 405964 | - | see also 23339 line |
| 52719 | ODF3 | NM_053280.3 | 502 | tacaatgtaaaccccaagatact | 405966 | - | see also 23339 line |
| 52720 | ODF4 | NM_153007.3 | 764 | caactgcgcgatcctttgctact | 421806 | - | see also 23340 line |
| 52721 | ODF4 | NM_153007.3 | 452 | tgcctttatcctactattggtca | 421801 | - | see also 23340 line |
| 52722 | ODF4 | NM_153007.3 | 770 | cgcgatcctttgctacttcaacc | 421807 | - | see also 23340 line |
| 52723 | OFCC1 | NM_153003.1 | 433 | atgacgatagaattttctataat | 421787 | - | see also 23341 line |
| 52724 | OFCC1 | NM_153003.1 | 440 | tagaatttctataatcgattaa | 421789 | - | see also 23341 line |
| 52725 | OKL38 | NM_013370.2 | 1169 | cagggactacgtggtcaagaagg | 338850 | - | see also 23342 line |
| 52726 | OKL38 | NM_013370.2 | 1192 | grctggggcataactttgtgtcc | 338852 | - | see also 23342 line |

Figure 46

| | | | | | | | |
|---|---|---|---|---|---|---|---|
| 52727 | OLIG3 | NM_175747.2 | 854 | gcctcccactcgctactcaagg | 429271 | - | see also 23343 line |
| 52728 | OLIG3 | NM_175747.2 | 659 | gaggctggttggcgagatctatg | 429269 | - | see also 23343 line |
| 52729 | OR12D2 | NM_013936.2 | 670 | ttcttcaagaccccgttcttgtag | 338981 | - | see also 23344 line |
| 52730 | OR12D2 | NM_013936.2 | 833 | ctgtggtcactcctgtactaaac | 338984 | - | see also 23344 line |
| 52731 | OR2B2 | NM_033057.1 | 99 | ttcctatatcttgacaatctttg | 402741 | - | see also 23345 line |
| 52732 | OR2B2 | NM_033057.1 | 450 | tagtggctttagcaattcagtat | 402745 | - | see also 23345 line |
| 52733 | OR2C3 | NM_198074.1 | 899 | gcgccctccggcacatggtatta | 436969 | - | see also 23346 line |
| 52734 | OR2C3 | NM_198074.1 | 289 | gggtgtggtccagttctatat | 436961 | - | see also 23346 line |
| 52735 | OR2F1 | NM_012369.1 | 756 | tggtgtggccatttcacttaca | 338102 | - | see also 23347 line |
| 52736 | OR2F1 | NM_012369.1 | 755 | atgtgtggccatttcacttac | 338101 | - | see also 23347 line |
| 52737 | OR2F1 | NM_012369.1 | 758 | gtgtggccatttcacttacatc | 338103 | - | see also 23347 line |
| 52738 | OR2S2 | NM_019897.1 | 594 | aggagtccggttctgttcatct | 375554 | - | see also 23348 line |
| 52739 | OR2S2 | NM_019897.1 | 596 | gagtccggttcgttcatctct | 375555 | - | see also 23348 line |
| 52740 | OR3A3 | NM_012373.1 | 842 | aggggttggggtttcatgact | 338107 | - | see also 23349 line |
| 52741 | OR3A3 | NM_012373.1 | 809 | ggctggttcagttggaatcttca | 338106 | - | see also 23349 line |
| 52742 | OR51B2 | NM_033180.1 | 219 | gaccacgatgctactgtaatgg | 403544 | - | see also 23350 line |
| 52743 | OR51B2 | NM_033180.1 | 370 | atccgcaatcctttgagatatgc | 403547 | - | see also 23350 line |
| 52744 | OR7C2 | NM_012377.1 | 667 | tcctccgtcctaagagtatctgc | 338112 | - | see also 23351 line |
| 52745 | OR7C2 | NM_012377.1 | 664 | ttctcctccgtcctaagagtatc | 338111 | - | see also 23351 line |
| 52746 | ORAOV1 | NM_153451.1 | 497 | cagaaggttccggactttcattt | 423270 | - | see also 23352 line |
| 52747 | ORAOV1 | NM_153451.1 | 500 | aaggttccggactttcattttga | 423271 | - | see also 23352 line |
| 52748 | ORAOV2 | NM_018043.4 | 800 | acccggagcacgattgtctatga | 365741 | - | see also 23353 line |
| 52749 | ORAOV2 | NM_018043.4 | 516 | tccacgagtcgggtttgtgaaa | 365735 | - | see also 23353 line |
| 52750 | ORAOV2 | NM_018043.4 | 495 | agcggacgaggaacactaaaatc | 365733 | - | see also 23353 line |
| 52751 | ORMDL2 | NM_014182.3 | 165 | atccataacctggctacgtatgt | 339942 | - | see also 5530 line |
| 52752 | ORMDL2 | NM_014182.3 | 36 | cccaaacaccgagtgatgaatag | 339939 | - | see also 5530 line |
| 52753 | ORMDL3 | NM_139280.1 | 567 | cacggagtccgatttttggaat | 409612 | - | see also 23355 line |
| 52754 | ORMDL3 | NM_139280.1 | 425 | cacggcctctcggaagttcttga | 409609 | - | see also 23355 line |
| 52755 | ORMDL3 | NM_139280.1 | 564 | ctccacggagtccggattttgg | 409611 | - | see also 23355 line |
| 52756 | OSAP | NM_032623.2 | 824 | gccataggtggtctgataagtgc | 401146 | - | see also 9262 line |
| 52757 | OSAP | NM_032623.2 | 158 | cgccggatgtcatcttaacagatt | 401130 | - | see also 9262 line |
| 52758 | OSRF | NM_012382.1 | 370 | tgctacctatacgagatgaaat | 338119 | - | see also 23357 line |
| 52759 | OSRF | NM_012382.1 | 212 | tggcttcatgccattaaacgtag | 338113 | - | see also 23357 line |
| 52760 | OSTalpha | NM_152672.2 | 983 | gaccacaaggttgggtatgaaac | 419562 | - | - |
| 52761 | OSTalpha | NM_152672.2 | 949 | gactgtgctgacacgaatgtact | 419561 | - | see also 52760 line |
| 52762 | OSTalpha | NM_152672.2 | 623 | ctcgtccccgacggcatctatga | 419559 | - | see also 52760 line |
| 52763 | OSTbeta | NM_178859.2 | 159 | ggcagctgtgtggtcattataa | 432018 | - | see also 23358 line |

Figure 46

| | | | | | | | |
|---|---|---|---|---|---|---|---|
| 52764 | OSTbeta | NM_178859.2 | 129 | atctccctggaatcattccatcc | 432016 | - | see also 23358 line |
| 52765 | OSTM1 | NM_014028.2 | 870 | aactatggagtcgaactttcaac | 339332 | - | see also 23359 line |
| 52766 | OSTM1 | NM_014028.2 | 740 | tgccgtgaagcatacaaaactct | 339329 | - | see also 23359 line |
| 52767 | OSTM1 | NM_014028.2 | 657 | ccctgacctgctttgaacataac | 339325 | - | see also 23359 line |
| 52768 | OTOA | NM_144672.2 | 1213 | gtcggatgcagttgtaggtttga | 411252 | - | see also 9728 line |
| 52769 | OTOA | NM_144672.2 | 1839 | caccctatcaggttaattgtttg | 411261 | - | see also 9728 line |
| 52770 | OTOA | NM_144672.2 | 1751 | ggcgtgacctgctcacacattga | 411260 | - | see also 9728 line |
| 52771 | OTOP1 | NM_177998.1 | 425 | ggctgcgcggtagtatcacattg | 430351 | - | see also 10176 line |
| 52772 | OTOP1 | NM_177998.1 | 428 | tgcgcggtagtatcacattgttt | 430352 | - | see also 10176 line |
| 52773 | OTOP2 | NM_178160.1 | 801 | caggggtacttctacctatatcc | 430533 | - | see also 23362 line |
| 52774 | OTOP2 | NM_178160.1 | 1219 | tgggtcagtacgccatctcttac | 430537 | - | see also 23362 line |
| 52775 | OTOP3 | NM_178233.1 | 1641 | tggcatacacccggagtttgaga | 430637 | - | see also 23363 line |
| 52776 | OTOP3 | NM_178233.1 | 837 | tgccaccgcgtgtgaagctttcc | 430630 | - | see also 23363 line |
| 52777 | OTP | NM_032109.2 | 175 | cgccaggctaggtatgaaagatg | 397755 | - | see also 9074 line |
| 52778 | OTP | NM_032109.2 | 176 | gccaggctaggtatgaaagatgc | 397756 | - | see also 9074 line |
| 52779 | OTP | NM_032109.2 | 173 | gacgccaggctaggtatgaaaga | 397754 | - | see also 9074 line |
| 52780 | OVCA2 | NM_080822.1 | 241 | gacgttttctccgcattggaaga | 406786 | - | see also 23364 line |
| 52781 | OVCH1 | NM_183378.1 | 2837 | tacgaggtgcatttggtataagc | 435811 | - | see also 23365 line |
| 52782 | OVCH1 | NM_183378.1 | 2370 | gccatggaagccgggtgtatttg | 435801 | - | see also 23365 line |
| 52783 | OVTN | NM_198185.1 | 728 | cactcatgtgccggaataagaaa | 437207 | - | see also 23366 line |
| 52784 | OVTN | NM_198185.1 | 983 | cagaaagcctccacctatattat | 437213 | - | see also 23366 line |
| 52785 | P114-RHO-GEF | NM_015318.1 | 259 | ctccaaacaccgagtccattttt | 351469 | - | see also 23367 line |
| 52786 | P114-RHO-GEF | NM_015318.1 | 1157 | atcagggcgcttgaaagatatcc | 351487 | - | see also 23367 line |
| 52787 | P114-RHO-GEF | NM_015318.1 | 714 | aagtacggtgtgttttgtagtgg | 351480 | - | see also 23367 line |
| 52788 | p25 | NM_007030.1 | 400 | ctcgccaagaagcgattcaaaga | 336880 | - | see also 23368 line |
| 52789 | p25 | NM_007030.1 | 325 | atcgtcttcagcaagatcaaagg | 336879 | - | see also 23368 line |
| 52790 | p30 | NM_181716.1 | 650 | agggagctgagcacataacgact | 432990 | - | see also 23369 line |
| 52791 | p30 | NM_181716.1 | 721 | ggcgttcagagcttctatactcc | 432995 | - | see also 23369 line |
| 52792 | P38IP | NM_017569.2 | 1439 | tcgtccacagatgatcattcaaa | 359161 | - | see also 7036 line |
| 52793 | P38IP | NM_017569.2 | 1491 | ctgagagggtagtcaatcagtac | 359164 | - | see also 7036 line |
| 52794 | P518 | NM_198180.1 | 8 | ggccttaccccctgatctacttc | 437179 | - | see also 23371 line |
| 52795 | P518 | NM_198180.1 | 9 | gccttaccccctgatctacttcc | 437180 | - | see also 23371 line |
| 52796 | P5326 | NM_031450.2 | 755 | cacctacaagcctgatgtctaca | 397058 | - | see also 23372 line |
| 52797 | P5326 | NM_031450.2 | 655 | ggcgccaggtcatctgtgtttac | 397057 | - | see also 23372 line |

Figure 46

| | | | | | | | |
|---|---|---|---|---|---|---|---|
| 52798 | PACAP | NM_016459.2 | 871 | ttccctctggactcaaataaaa | 357581 | - | see also 23373 line |
| 52799 | PACAP | NM_016459.2 | 872 | tccctctggactcaaataaaac | 357582 | - | see also 23373 line |
| 52800 | PACRG | NM_152410.1 | 959 | ctccggagacggcattgactaca | 416436 | - | see also 23374 line |
| 52801 | PACRG | NM_152410.1 | 666 | cagctcattatccgcataaaaaa | 416434 | - | see also 23374 line |
| 52802 | PACRG | NM_152410.1 | 1038 | gagcgctacggaggagaaatgc | 416437 | - | see also 23374 line |
| 52803 | PAF53 | NM_022490.1 | 912 | accgccaggccgatgcatatgg | 383471 | - | see also 23375 line |
| 52804 | PAF53 | NM_022490.1 | 911 | gaccgccaggcccgatgcatatg | 383470 | - | see also 23375 line |
| 52805 | PAIP2 | NM_016480.2 | 508 | ctgaggtgaagtacggaaatatt | 357735 | - | see also 6873 line |
| 52806 | PAIP2 | NM_016480.2 | 510 | gagggtgaagtacggaaatatttg | 357737 | - | see also 6873 line |
| 52807 | PAK1IP1 | NM_017906.1 | 492 | cctgtcggttggtacagataaa | 363811 | - | see also 23377 line |
| 52808 | PAK1IP1 | NM_017906.1 | 1098 | aagaaaagcggtcaaaacctaac | 363827 | - | see also 23377 line |
| 52809 | PALM2 | NM_053016.1 | 697 | caggaggcacgtagtagaaaat | 405785 | - | see also 9481 line |
| 52810 | PALM2 | NM_053016.1 | 1118 | gacggacgtgtccactattgacg | 405796 | - | see also 9481 line |
| 52811 | PALMD | NM_017734.2 | 1269 | tccccgatcagtgattcaacaag | 361289 | - | see also 23379 line |
| 52812 | PALMD | NM_017734.2 | 1514 | cagaaccggtgacaatgattttc | 361300 | - | see also 23379 line |
| 52813 | PALMD | NM_017734.2 | 1300 | aagcttcacaccccgcaaaaaag | 361291 | - | see also 23379 line |
| 52814 | PAN3 | NM_175854.4 | 1647 | tggtgctcgattttatactcaat | 429348 | - | see also 10154 line |
| 52815 | PAN3 | NM_175854.4 | 596 | gtgggacgacttacttctataca | 429306 | - | see also 10154 line |
| 52816 | PAQR9 | NM_198504.1 | 899 | agccgggtctttgacacattatc | 438746 | - | see also 10333 line |
| 52817 | PBF | NM_018660.1 | 1129 | gacgaaccagctccacgaaaaag | 372385 | - | see also 23382 line |
| 52818 | PBF | NM_018660.1 | 1704 | agcacctgcgatgaaatctcatc | 372389 | - | see also 23382 line |
| 52819 | PBOV1 | NM_021635.1 | 235 | taccattctgctacaaggtattt | 380933 | - | see also 23383 line |
| 52820 | PBOV1 | NM_021635.1 | 243 | tgctacaaggtatttgaaaaa | 380935 | - | see also 23383 line |
| 52821 | PCNX | NM_014982.1 | 2065 | gggatgttcgacctaaatcttct | 346599 | - | see also 6164 line |
| 52822 | PCNX | NM_014982.1 | 2168 | aagccggcataggactatgttt | 346603 | - | see also 6164 line |
| 52823 | PD2 | NM_019088.1 | 620 | tgcgaaagacgagtacatctcc | 375141 | - | see also 7702 line |
| 52824 | PD2 | NM_019088.1 | 1209 | aaccaggtccgccttagttaagc | 375152 | - | see also 7702 line |
| 52825 | PDCD10 | NM_007217.3 | 920 | cagtgatactctgaaaacgtatt | 337226 | - | see also 23386 line |
| 52826 | PDCD10 | NM_007217.3 | 641 | tcgtatggcagctgatgatgtag | 337213 | - | see also 23386 line |
| 52827 | PDCD4 | NM_014456.3 | 490 | gacgccccttagaagtggattaac | 340701 | - | see also 5683 line |
| 52828 | PDCD4 | NM_014456.3 | 1134 | ggctaccgtgcttctgagtatgt | 340718 | - | see also 5683 line |
| 52829 | PDCD7 | NM_005707.1 | 944 | gccgctgatggcgtactatctga | 334182 | - | see also 3960 line |
| 52830 | PDCD7 | NM_005707.1 | 1330 | gccttccgacagtattatctcc | 334194 | - | see also 3960 line |
| 52831 | PDIP38 | NM_015584.2 | 259 | accgaccagagggcaaagtgttg | 354327 | - | see also 6497 line |
| 52832 | PDIP38 | NM_015584.2 | 1089 | ggcacgttccgctttgaaagacc | 354342 | - | see also 6497 line |
| 52833 | PEGASUS | NM_022466.2 | 556 | aagtgtcggtactgcaactatgc | 383005 | - | see also 23390 line |
| 52834 | PEGASUS | NM_022466.2 | 932 | aaccagactaccttaacgatttt | 383017 | - | see also 23390 line |

Figure 46

| | | | | | | | |
|---|---|---|---|---|---|---|---|
| 52835 | PELI1 | NM_020651.2 | 4949 | aaccatgggtatatctaaactgc | 377876 | - | see also 7907 line |
| 52836 | PELI2 | NM_021255.1 | 249 | ctcgggtacaatggtgctttacc | 380907 | - | see also 23392 line |
| 52837 | PELI2 | NM_021255.1 | 988 | aagccctccggcaggagattaac | 380920 | - | see also 23392 line |
| 52838 | PEN2 | NM_172341.1 | 222 | gtccaatgaggagaaattgaacc | 424405 | - | - | - | - |
| 52839 | PEPP3 | NM_014935.2 | 1391 | ccccgatgattatcagtactacc | 346100 | - | see also 6118 line |
| 52840 | PEPP3 | NM_014935.2 | 1772 | acctcgcagtgaggacatctatg | 346106 | - | see also 6118 line |
| 52841 | PERQ1 | NM_022574.2 | 2203 | gggtcggcccattcgcaaaaaga | 383587 | - | see also 23394 line |
| 52842 | PERQ1 | NM_022574.2 | 2205 | gtcggcccattcgcaaaaagacg | 383588 | - | see also 23394 line |
| 52843 | PERQ1 | NM_022574.2 | 2380 | cccctatgatgtccacgattata | 383589 | - | see also 23394 line |
| 52844 | PEX11G | NM_080662.2 | 190 | accatcttgcgactctttgatga | 406590 | - | see also 23395 line |
| 52845 | PEX11G | NM_080662.2 | 189 | gaccatcttgcgactctttgatg | 406589 | - | see also 23395 line |
| 52846 | PEX26 | NM_017929.2 | 931 | ctgcggtgagccacttcttttct | 364274 | - | see also 23396 line |
| 52847 | PEX26 | NM_017929.2 | 1074 | atccggaaggctgcattttctcg | 364278 | - | see also 23396 line |
| 52848 | PEX26 | NM_017929.2 | 1071 | tggatccggaaggctgcattttc | 364277 | - | see also 23396 line |
| 52849 | PEX5R | NM_016559.1 | 416 | aggccagtaacatccaatacagc | 358115 | - | see also 23397 line |
| 52850 | PEX5R | NM_016559.1 | 878 | cgctggggaagcgcattactttc | 358127 | - | see also 23397 line |
| 52851 | PF20 | NM_024532.2 | 714 | accgactataagggtgttacatg | 386799 | - | see also 23398 line |
| 52852 | PF20 | NM_024532.2 | 1875 | ttctcacgacggggagattctct | 386834 | - | see also 23398 line |
| 52853 | PF20 | NM_024532.2 | 1258 | ggcgacaaattggctacttcaag | 386811 | - | see also 23398 line |
| 52854 | Pfs2 | NM_016095.1 | 564 | agcgctcaaccacatgtacaaac | 356269 | - | see also 23399 line |
| 52855 | Pfs2 | NM_016095.1 | 363 | tacgaagctcctgttaaatcatg | 356266 | - | see also 23399 line |
| 52856 | Pfs2 | NM_016095.1 | 416 | tccggaccctggtcaaggatatg | 356267 | - | see also 23399 line |
| 52857 | PGA5 | NM_014224.1 | 717 | tggtggcattgactcttcttact | 339975 | - | see also 23400 line |
| 52858 | PGA5 | NM_014224.1 | 696 | gagtggcagcgtggtgatctttg | 339973 | - | see also 23400 line |
| 52859 | PGBD2 | NM_170725.1 | 1772 | gactgagagccgcttcgatatga | 424151 | - | see also 23401 line |
| 52860 | PGBD2 | NM_170725.1 | 1776 | gagagccgcttcgatatgattgg | 424153 | - | see also 23401 line |
| 52861 | PGBD4 | NM_152595.2 | 1250 | cagtacctcccgacaaaacgagt | 418598 | - | see also 23402 line |
| 52862 | PGBD4 | NM_152595.2 | 877 | aggattttcgcgaatggataaat | 418585 | - | see also 23402 line |
| 52863 | PGBD4 | NM_152595.2 | 1258 | cccgacaaaacgagtacgatttg | 418599 | - | see also 23402 line |
| 52864 | PGBD5 | NM_024554.2 | 1085 | acgcggcaggcaagaactacatc | 387212 | - | see also 23403 line |
| 52865 | PGBD5 | NM_024554.2 | 1086 | cgcggcaggcaagaactacatca | 387213 | - | see also 23403 line |
| 52866 | PGSF1 | NM_174947.2 | 416 | atcaagggaaacgatccttattg | 428774 | - | see also 23404 line |
| 52867 | PGSF1 | NM_174947.2 | 118 | caggttggcgcatggacattttc | 428772 | - | see also 23404 line |
| 52868 | PHAX | NM_032177.1 | 492 | atccgagacctacaattatttgc | 398518 | - | see also 23405 line |
| 52869 | PHAX | NM_032177.1 | 488 | gacaatccgagacctacaattat | 398517 | - | see also 23405 line |
| 52870 | PHC3 | NM_024947.2 | 849 | ctgtcacccggacatcaagtatt | 392503 | - | see also 8814 line |
| 52871 | PHF7 | NM_016483.4 | 1424 | gtgtaacaatcgaaaagagtttc | 357764 | - | see also 23407 line |

EP 1 752 536 A1

1774

Figure 46

| | | | | | | | |
|---|---|---|---|---|---|---|---|
| 52872 | PHF7 | NM_016483.4 | 984 | ttctgtcttatctatctagtaa | 357756 | - | see also 23407 line |
| 52873 | PHGDHL1 | NM_177967.2 | 1249 | cagcggacaaaacgtaaactatca | 430345 | - | see also 23408 line |
| 52874 | PHGDHL1 | NM_177967.2 | 1247 | gtcagcgacaaaacgtaaactat | 430344 | - | see also 23408 line |
| 52875 | PHLDA3 | NM_012396.1 | 613 | caccctaggcctggtcaagttca | 338132 | - | - |
| 52876 | PHLDA3 | NM_012396.1 | 615 | cctaggcctggtcaagttcaag | 338133 | - | see also 52875 line |
| 52877 | PHOSPHO1 | NM_178500.2 | 413 | ctggcctccgctgcctactagg | 430915 | - | see also 23409 line |
| 52878 | PHYHIP | NM_014759.1 | 418 | cacgccccacagcattgagatca | 343158 | - | see also 23410 line |
| 52879 | PHYHIP | NM_014759.1 | 1164 | tgcccctcctggacattgcttgc | 343163 | - | see also 23410 line |
| 52880 | PIB5PA | NM_014422.1 | 1079 | gtgggtaccaacaacatacgatac | 340567 | - | see also 23411 line |
| 52881 | PIB5PA | NM_014422.1 | 561 | atgcgctagggcccttcaacttc | 340562 | - | see also 23411 line |
| 52882 | PIBF1 | NM_006346.1 | 1301 | aacacgatcagctcttagacagg | 335274 | - | see also 4443 line |
| 52883 | PIBF1 | NM_006346.1 | 1675 | tcctacggctatggtgctaatgt | 335282 | - | see also 4443 line |
| 52884 | PIGN | NM_012327.3 | 829 | tggatacgtgggttttgataat | 338040 | - | see also 5279 line |
| 52885 | PIGN | NM_012327.3 | 752 | tgctagtggagaccacgtttata | 338033 | - | see also 5279 line |
| 52886 | PIGT | NM_015937.2 | 913 | accccgaccaactactatcagg | 355520 | - | see also 23414 line |
| 52887 | PIGT | NM_015937.2 | 1513 | ctctgatggctctaactacttg | 355525 | - | see also 23414 line |
| 52888 | PIGT | NM_015937.2 | 1385 | tacaacgccagatcctaaccatgg | 355523 | - | see also 23414 line |
| 52889 | PIGW | NM_178517.2 | 695 | atcggacgattagccattataaa | 431005 | - | see also 10195 line |
| 52890 | PIGW | NM_178517.2 | 932 | ggcacacgggttggtcattaaa | 431019 | - | see also 10195 line |
| 52891 | PIGW | NM_178517.2 | 686 | ttcctaggaatcggacgattagc | 431004 | - | see also 10195 line |
| 52892 | PIK3AP1 | NM_152309.1 | 1564 | aacgatcggagtccttcgttc | 415490 | - | see also 23416 line |
| 52893 | PIK3AP1 | NM_152309.1 | 423 | acgttatcagctattactga | 415455 | - | see also 23416 line |
| 52894 | PIK3AP1 | NM_152309.1 | 1565 | acgatcggagtcctttcgtttcc | 415491 | - | see also 23416 line |
| 52895 | PIP3AP | NM_019061.2 | 198 | tacgtacgccctgaggaaattca | 374803 | - | see also 7685 line |
| 52896 | PIP3AP | NM_019061.2 | 652 | ctcctaaactgcttaaacgatta | 374812 | - | see also 7685 line |
| 52897 | PISD | NM_014338.3 | 538 | gtccagaaccgctctgaaactgc | 340215 | - | see also 23418 line |
| 52898 | PISD | NM_014338.3 | 536 | atgtccagaccgctctgaaact | 340214 | - | see also 23418 line |
| 52899 | PIWIL1 | NM_004764.2 | 1560 | ttgatctatacgcgaagaaatta | 332966 | - | see also 3275 line |
| 52900 | PIWIL1 | NM_004764.2 | 1281 | gacttagccgttcatacaagact | 332956 | - | see also 3275 line |
| 52901 | PIWIL2 | NM_018068.2 | 1374 | aacaatcgtacctatcgtattga | 366075 | - | see also 23420 line |
| 52902 | PIWIL2 | NM_018068.2 | 923 | tgcgtttgatgatctattctct | 366063 | - | see also 23420 line |
| 52903 | PIWIL2 | NM_018068.2 | 2020 | caccggctgggttgaactaaag | 366091 | - | see also 23420 line |
| 52904 | PJA1 | NM_022368.2 | 851 | ctctgcgtcggaaatatcgaagc | 382720 | - | see also 23421 line |
| 52905 | PJA1 | NM_022368.2 | 600 | gaggataccgccaatgacaatg | 382713 | - | see also 23421 line |
| 52906 | PKD1L3 | NM_181536.1 | 1472 | tactaagcgctaatcgtaaattg | 432471 | - | see also 10237 line |
| 52907 | PKD1L3 | NM_181536.1 | 1476 | aagcgctaatcgtaaattgctcc | 432472 | - | see also 10237 line |

Figure 46

| 52908 | PKHD1L1 | NM_177531.2 | 7510 | tccggttggggcgatatccaata | 430120 | - | see also 10166 line |
|---|---|---|---|---|---|---|---|
| 52909 | PKHD1L1 | NM_177531.2 | 7509 | ttccggttggggcgatatccaat | 430119 | - | see also 10166 line |
| 52910 | PLAC4 | NM_182832.1 | 175 | tcgccgctagggtgtcttttaag | 435375 | - | see also 23424 line |
| 52911 | PLAC4 | NM_182832.1 | 294 | ctggcctaaacatggttctatat | 435378 | - | see also 23424 line |
| 52912 | PLAC8 | NM_016619.1 | 339 | atccctggatctatttgtgatga | 358463 | - | see also 23425 line |
| 52913 | PLAC8 | NM_016619.1 | 256 | gtcaagttgcagctgatatgaat | 358462 | - | see also 23425 line |
| 52914 | PLEKHB2 | NM_017958.1 | 152 | gtcggatggtcacctgatctatt | 364871 | - | see also 23426 line |
| 52915 | PLEKHB2 | NM_017958.1 | 527 | cccgccaggaactcaagttgtct | 364882 | - | see also 23426 line |
| 52916 | PLVAP | NM_031310.1 | 244 | gccgagccgagggcctatacagt | 396707 | - | see also 23427 line |
| 52917 | PLVAP | NM_031310.1 | 101 | ctctcggggctgctggtattacc | 396706 | - | see also 23427 line |
| 52918 | PLVAP | NM_031310.1 | 637 | agctggttgaatgcgtgaaaacc | 396711 | - | see also 23427 line |
| 52919 | PMAIP1 | NM_021127.1 | 285 | ttccggcagaaacttctgaatct | 380529 | - | see also 23428 line |
| 52920 | PMFBP1 | NM_031293.1 | 2770 | taccgagggaatgatcagattat | 396557 | - | see also 23429 line |
| 52921 | PMFBP1 | NM_031293.1 | 697 | ctgagcaacatcgagttactaga | 396534 | - | see also 23429 line |
| 52922 | PNAS-131 | NM_031446.2 | 597 | atgttacttagtcagaatgaatc | 397052 | - | see also 23430 line |
| 52923 | PNAS-131 | NM_031446.2 | 304 | aagactccaaaagtgtattgttg | 397043 | - | see also 23430 line |
| 52924 | PNAS-4 | NM_016076.2 | 636 | gcctacttcagctcctgtatacc | 356214 | - | see also 23431 line |
| 52925 | PNAS-4 | NM_016076.2 | 645 | agctcctgtataccctttctaca | 356215 | - | see also 23431 line |
| 52926 | PNLDC1 | NM_173516.1 | 986 | tcccagaaagctacgatcaattt | 425294 | - | see also 23432 line |
| 52927 | PNLDC1 | NM_173516.1 | 1517 | atgtcaggcgactcacaagaagc | 425315 | - | see also 23432 line |
| 52928 | PNMA2 | NM_007257.3 | 662 | accggaaactgcgagtattctca | 337352 | - | see also 23433 line |
| 52929 | PNMA2 | NM_007257.3 | 664 | cggaaactgcgagtattctcagg | 337353 | - | see also 23433 line |
| 52930 | PNMA5 | NM_052926.1 | 1355 | gcgagggtgtcctcccaattttc | 405357 | - | see also 23434 line |
| 52931 | PNMA5 | NM_052926.1 | 1600 | ggcggctgttaggcagtgaaagc | 405361 | - | see also 23434 line |
| 52932 | PODN | NM_153703.2 | 1473 | acctgggctgcctcgaaatgtcc | 423711 | - | see also 23435 line |
| 52933 | PODN | NM_153703.2 | 1781 | aggggatctttctcaggtttaac | 423713 | - | see also 23435 line |
| 52934 | POF1B | NM_024921.2 | 940 | gccgtaagaatacggatctatat | 392173 | - | see also 23436 line |
| 52935 | POF1B | NM_024921.2 | 1362 | ctctctcttcgacatacactatc | 392183 | - | see also 23436 line |
| 52936 | POLN | NM_181808.1 | 538 | gacgccgaaggctacctaaattc | 433679 | - | see also 23437 line |
| 52937 | POLN | NM_181808.1 | 903 | tgcccggaacgtgctatttcaaa | 433690 | - | see also 23437 line |
| 52938 | POMT1 | NM_007171.2 | 993 | atcccggtcgtcctgtacttact | 336990 | - | see also 4973 line |
| 52939 | POMT1 | NM_007171.2 | 1020 | ttctacgtccacttgattctagt | 336994 | - | see also 4973 line |
| 52940 | POP5 | NM_015918.3 | 356 | aacatgtcagaagttcctaattc | 355486 | - | see also 23439 line |
| 52941 | POP5 | NM_015918.3 | 143 | cgccagggtgcacggaactttcg | 355478 | - | see also 23439 line |
| 52942 | POT1 | NM_015450.1 | 458 | accgtcttggacattactaaaat | 353116 | - | see also 23440 line |
| 52943 | POT1 | NM_015450.1 | 1102 | accgcatccgagcaaaattgagg | 353151 | - | see also 23440 line |
| 52944 | PP1057 | NM_031285.1 | 941 | ctgcgcttggccgttttgttatt | 396495 | - | see also 23441 line |

Figure 46

| 52945 | PP1057 | NM_031285.1 | 673 | gggcctcgtgcgtttttgctgt | 396490 | - | see also 23441 line |
|---|---|---|---|---|---|---|---|
| 52946 | PP1201 | NM_022152.3 | 655 | atcactgcggtggtatccatttc | 382586 | - | see also 23442 line |
| 52947 | PP1201 | NM_022152.3 | 603 | gggcaccatttccagtatgtacc | 382583 | - | see also 23442 line |
| 52948 | PP2447 | NM_025204.2 | 875 | cggagcgcgacgtctacctaacc | 394410 | - | see also 23443 line |
| 52949 | PP2447 | NM_025204.2 | 363 | cagcaagagggacgttgtgaaga | 394409 | - | see also 23443 line |
| 52950 | pp9099 | NM_025201.3 | 1390 | cccctgttagtgccgaaacattg | 394357 | - | see also 23444 line |
| 52951 | pp9099 | NM_025201.3 | 1391 | ccctgttagtgccgaaacattgc | 394358 | - | see also 23444 line |
| 52952 | pp9099 | NM_025201.3 | 320 | gcgaaacaccgctttatcctgc | 394349 | - | see also 23444 line |
| 52953 | PPAN | NM_020230.2 | 291 | tgcaggttcgtaagaagaactcg | 376292 | - | see also 23445 line |
| 52954 | PPAN | NM_020230.2 | 341 | cccctcggggtcacacactttct | 376293 | - | see also 23445 line |
| 52955 | PPFIA3 | NM_003660.1 | 3889 | gcgtttcggtccggacctattcc | 331962 | - | see also 23446 line |
| 52956 | PPFIA3 | NM_003660.1 | 3264 | ctggtggacgctcgaatgttaga | 331952 | - | see also 23446 line |
| 52957 | PPIL5 | NM_152329.2 | 589 | ttggataccgcaaaaatttgtgt | 415687 | - | see also 23447 line |
| 52958 | PPIL5 | NM_152329.2 | 494 | agccactgttcggttaaaggagc | 415679 | - | see also 23447 line |
| 52959 | PPL13 | NM_020129.1 | 226 | gtcatcactacccgtaccataca | 375610 | - | see also 23448 line |
| 52960 | PPL13 | NM_020129.1 | 537 | atggccaacgcatttacaactttt | 375619 | - | see also 23448 line |
| 52961 | PPN | NM_022825.1 | 281 | cccatcgaggcgtcttcctatcc | 384255 | - | see also 8480 line |
| 52962 | PPN | NM_022825.1 | 1288 | ggctacggcatggcatacactgt | 384268 | - | see also 8480 line |
| 52963 | PPP1R12C | NM_017607.1 | 1019 | cgccctctagcagcaaacacaga | 359412 | - | see also 23450 line |
| 52964 | PPP1R12C | NM_017607.1 | 1412 | agctccgtcttgccagaattacc | 359414 | - | see also 23450 line |
| 52965 | PPP1R14A | NM_033256.1 | 406 | gcccgatgagatcaacattgatg | 403645 | - | - |
| 52966 | PPP1R14A | NM_033256.1 | 397 | ggcagacatgcccgatgagatca | 403644 | - | see also 52965 line |
| 52967 | PPP1R14C | NM_030949.1 | 312 | gacagtgaaatacgatcgtaagg | 395666 | - | see also 23451 line |
| 52968 | PPP1R14C | NM_030949.1 | 509 | aaccaacagaggaatttatcaaa | 395668 | - | see also 23451 line |
| 52969 | PPP1R14C | NM_030949.1 | 572 | ctccgcagaagaagagtgtatga | 395670 | - | see also 23451 line |
| 52970 | PPP1R14D | NM_017726.6 | 484 | ctgctcagtcaactcaagaaact | 361163 | - | see also 23452 line |
| 52971 | PPP1R14D | NM_017726.6 | 220 | cacccggactcctccaagatacc | 361161 | - | see also 23452 line |
| 52972 | PPP1R15B | NM_032833.1 | 1600 | gggccgaataagtgtagttgatt | 401837 | - | see also 9300 line |
| 52973 | PPP1R15B | NM_032833.1 | 1594 | tggagagggccgaataagtgtag | 401835 | - | see also 9300 line |
| 52974 | PPP1R3B | NM_024607.1 | 403 | cccgctagatatgccattcaaca | 387820 | - | see also 23454 line |
| 52975 | PPP1R3B | NM_024607.1 | 849 | tccgggctgagttaaaatctacc | 387832 | - | see also 23454 line |
| 52976 | PR1 | NM_183240.1 | 487 | atgtttggtgcgaattcactgc | 435640 | - | see also 23455 line |
| 52977 | PR1 | NM_183240.1 | 472 | atcggcttcaccctaatgttttg | 435638 | - | see also 23455 line |
| 52978 | PR1 | NM_183240.1 | 121 | tcctcggtctccatttgtgatgg | 435637 | - | see also 23455 line |
| 52979 | PRAM-1 | NM_032152.3 | 1797 | atcgaccccaacgctaagacacg | 398404 | - | see also 23456 line |
| 52980 | PRAM-1 | NM_032152.3 | 631 | ccccgcagcctgagttgagtacc | 398399 | - | see also 23456 line |
| 52981 | PRAM-1 | NM_032152.3 | 127 | ctccgaagcctgagtttggtaaa | 398397 | - | see also 23456 line |

Figure 46

| 52982 | PRAME | NM_006115.2 | 1308 | gggtcatgctgaccgatgtaagt | 334835 | - | see also 23457 line |
|---|---|---|---|---|---|---|---|
| 52983 | PRAME | NM_006115.2 | 1273 | cagcgtcagtcagctaagtgtcc | 334834 | - | see also 23457 line |
| 52984 | PRDM11 | NM_020229.1 | 832 | aaccgctataagtccatagatgg | 376277 | - | see also 23458 line |
| 52985 | PRDM11 | NM_020229.1 | 708 | gagtgacgtgcgatgtgtaaacg | 376276 | - | see also 23458 line |
| 52986 | PRDM8 | NM_020226.1 | 1428 | gagaacagtaccgtatatctttc | 376258 | - | see also 7806 line |
| 52987 | PRDM8 | NM_020226.1 | 1598 | aagacgaggagttactagtttgg | 376262 | - | see also 7806 line |
| 52988 | PREI3 | NM_015387.1 | 484 | atcagtatgccgtaggatttaca | 352166 | - | see also 23460 line |
| 52989 | PREI3 | NM_015387.1 | 471 | tagcgaaactaggatcagtatgc | 352165 | - | see also 23460 line |
| 52990 | PRES | NM_198999.1 | 2290 | atgacctcactcggaatagattt | 440576 | - | see also 10354 line |
| 52991 | PRES | NM_198999.1 | 671 | tggtgtagctgttcgattagtac | 440521 | - | see also 10354 line |
| 52992 | PRG1 | NM_002727.1 | 354 | tggcttcctaacggaaatggaac | 331031 | - | see also 23462 line |
| 52993 | PRG1 | NM_002727.1 | 355 | ggcttcctaacggaaatggaaca | 331032 | - | see also 23462 line |
| 52994 | PRG1 | NM_014839.2 | 561 | acctgcaattacgattatggtag | 344464 | - | see also 6044 line |
| 52995 | PRG1 | NM_014839.2 | 1042 | tgcgggctaacacggataactca | 344475 | - | see also 6044 line |
| 52996 | PRIC285 | NM_033405.2 | 5273 | cccttactcgtcggaaatcaagg | 403807 | - | see also 23464 line |
| 52997 | PRIC285 | NM_033405.2 | 1993 | ggcacgagctggcgtttgtgtgc | 403786 | - | see also 23464 line |
| 52998 | PRICKLE1 | NM_153026.1 | 833 | ctcagcatgtgacgagataattt | 421909 | - | see also 23465 line |
| 52999 | PRICKLE1 | NM_153026.1 | 881 | gggtcgccattggcacatgaaac | 421912 | - | see also 23465 line |
| 53000 | PRICKLE2 | NM_198859.1 | 2278 | aggagcacctatcccgattttcc | 439983 | - | see also 23466 line |
| 53001 | PRICKLE2 | NM_198859.1 | 727 | aacaggtacaccagtactatagc | 439959 | - | see also 23466 line |
| 53002 | PRKRIP1 | NM_024653.1 | 219 | acctcgacctcccccagaatttg | 388543 | - | see also 23468 line |
| 53003 | PRKRIP1 | NM_024653.1 | 81 | accaccgaggcccaagaaagagc | 388541 | - | see also 23468 line |
| 53004 | PRND | NM_012409.2 | 493 | ccctcaagcattgcgagtttttgg | 338141 | - | see also 23469 line |
| 53005 | PRND | NM_012409.2 | 326 | ccccgatggcatccactacaacg | 338139 | - | see also 23469 line |
| 53006 | PRND | NM_012409.2 | 484 | agctctgctccctcaagcattgc | 338140 | - | see also 23469 line |
| 53007 | PRO0149 | NM_014117.2 | 773 | aactattcgtcgagaagatttga | 339600 | - | see also 23470 line |
| 53008 | PRO0149 | NM_014117.2 | 864 | tcccctaaccgagctacttcaac | 339602 | - | see also 23470 line |
| 53009 | PRO0628 | NM_014134.1 | 588 | aacacggcatttggtactaaatt | 339622 | - | see also 23471 line |
| 53010 | PRO0628 | NM_014134.1 | 564 | tgcctatactcacgcataactgt | 339620 | - | see also 23471 line |
| 53011 | PRO0971 | NM_018569.2 | 966 | ttctacgggcagcacttaagtat | 372254 | - | see also 23472 line |
| 53012 | PRO0971 | NM_018569.2 | 969 | tacgggcagcacttaagtatagc | 372255 | - | see also 23472 line |
| 53013 | PRO1580 | NM_018502.2 | 967 | tgccccgtgcttcgatgtctaag | 372044 | - | see also 23473 line |
| 53014 | PRO1580 | NM_018502.2 | 968 | gccccgtgcttcgatgtctaagc | 372045 | - | see also 23473 line |
| 53015 | PRO1853 | NM_018607.4 | 538 | gtccggctgaaggttcttttaga | 372314 | - | see also 23474 line |
| 53016 | PRO1853 | NM_018607.4 | 697 | aggaatgcacgtcagtcaaaacc | 372318 | - | see also 23474 line |
| 53017 | PRO1853 | NM_018607.4 | 484 | gcctctctgggcccaataaaaca | 372313 | - | see also 23474 line |
| 53018 | PRO1914 | NM_014106.1 | 1317 | tccccctgtcaaaagaaagttc | 339586 | - | see also 23475 line |

Figure 46

| | | | | | | | | | |
|---|---|---|---|---|---|---|---|---|---|
| 53019 | PRO1914 | NM_014106.1 | 1234 | ctgtaaattaaacccaagttact | 339584 | - | see also 23475 line | | |
| 53020 | PRO2268 | NM_018520.2 | 376 | tccctccccgctaaaatactagg | 372113 | - | see also 23476 line | | |
| 53021 | PRO2268 | NM_018520.2 | 215 | agccaaacaggattagactttat | 372111 | - | see also 23476 line | | |
| 53022 | PRO2730 | NM_025222.2 | 1355 | ctgcccaccacaaattgtgtaca | 394448 | - | see also 23477 line | | |
| 53023 | PRO2730 | NM_025222.2 | 1642 | tgcatccctgctaatagtgtttg | 394459 | - | see also 23477 line | | |
| 53024 | PRO2964 | NM_018547.1 | 695 | tacagaataaaatataacgatag | 372188 | - | see also 23478 line | | |
| 53025 | PRO2964 | NM_018547.1 | 643 | gacttggagaatactgttaattc | 372183 | - | see also 23478 line | | |
| 53026 | ProSAPiP2 | NM_014726.1 | 1690 | atggcgtgggtgaaaagagttgg | 342877 | - | - | - | - |
| 53027 | ProSAPiP2 | NM_014726.1 | 2550 | gtcctggccgtccatcaacttgc | 342879 | - | see also 53026 line | | |
| 53028 | ProSAPiP2 | NM_014726.1 | 1687 | gacatggcgtgggtgaaaagagt | 342876 | - | see also 53026 line | | |
| 53029 | Prostein | NM_033102.1 | 1328 | ttcggcactcgagcagtctattt | 403251 | - | see also 9357 line | | |
| 53030 | Prostein | NM_033102.1 | 1330 | cggcactcgagcagtctatttgg | 403253 | - | see also 9357 line | | |
| 53031 | PRY | NM_004676.2 | 396 | gactcatttctctggagatatgg | 332743 | - | - | - | - |
| 53032 | PSARL | NM_018622.3 | 142 | tcctcggacgcaggtttaacttc | 372321 | - | see also 23480 line | | |
| 53033 | PSARL | NM_018622.3 | 526 | agcggactgtgacaggtattata | 372338 | - | see also 23480 line | | |
| 53034 | PSD | NM_002779.2 | 1398 | aagaggctgtaccgactagatgg | 331068 | - | see also 23481 line | | |
| 53035 | PSD | NM_002779.2 | 2305 | atgtagtagccgctatgttctct | 331072 | - | see also 23481 line | | |
| 53036 | PSFL | NM_031301.1 | 98 | caccgagccgttgcgtatcatct | 396597 | - | see also 23482 line | | |
| 53037 | PSFL | NM_031301.1 | 357 | atgcgactgctggcctatgtttc | 396609 | - | see also 23482 line | | |
| 53038 | PSG11 | NM_002785.1 | 471 | tcccttcagtcacctcttactat | 331075 | - | - | - | - |
| 53039 | PSG11 | NM_002785.1 | 674 | tccacatccttgacaatcagagt | 331076 | - | see also 53038 line | | |
| 53040 | PSIP2 | NM_021144.2 | 696 | gagagtgacatcattactgaaga | 380538 | - | see also 23483 line | | |
| 53041 | PSIP2 | NM_021144.2 | 659 | aggcagacccaaaatggtaaaac | 380537 | - | see also 23483 line | | |
| 53042 | PTD002 | NM_016144.2 | 125 | cactcggattcttcaaaaacttc | 356461 | - | see also 6694 line | | |
| 53043 | PTD002 | NM_016144.2 | 120 | ttgctcactcggattcttcaaaa | 356459 | - | see also 6694 line | | |
| 53044 | PTD012 | NM_014039.2 | 306 | ttgattgccctgatttgactaag | 339348 | - | see also 5469 line | | |
| 53045 | PTD012 | NM_014039.2 | 834 | tgcgactggagcacactcatttt | 339364 | - | see also 5469 line | | |
| 53046 | PTE2B | NM_152331.1 | 274 | tggaacccgagaagccttttgg | 415691 | - | - | - | - |
| 53047 | PTE2B | NM_152331.1 | 809 | cacagccatcaactataagcaca | 415692 | - | see also 53046 line | | |
| 53048 | PTE2B | NM_152331.1 | 819 | aactataagcacagtagcattcc | 415693 | - | see also 53046 line | | |
| 53049 | PTF1A | NM_178161.1 | 958 | aacgaaccaccatttgagtttgt | 430548 | - | see also 23486 line | | |
| 53050 | PTF1A | NM_178161.1 | 792 | ctcccccagcgaccctgattatg | 430547 | - | see also 23486 line | | |
| 53051 | PTPLB | NM_198402.1 | 636 | gacaagctggcctatattccatc | 437720 | - | see also 10318 line | | |
| 53052 | PTPNS1 | NM_080792.1 | 918 | aacggcctcaaccgttacagaga | 406772 | - | see also 23487 line | | |
| 53053 | PTPNS1 | NM_080792.1 | 1198 | ctctacctcgtccgaatcagaca | 406773 | - | see also 23487 line | | |
| 53054 | PTPNS1L2 | NM_178460.1 | 167 | atcaactggggagtcaatcatct | 430791 | - | see also 23488 line | | |
| 53055 | PTPNS1L2 | NM_178460.1 | 371 | tgctgatgctggcacctattact | 430798 | - | see also 23488 line | | |

Figure 46

| | | | | | | | |
|---|---|---|---|---|---|---|---|
| 53056 | PTRF | NM_012232.1 | 422 | tggaggaccccacgctctatatt | 337792 | - | see also 23489 line |
| 53057 | PTRF | NM_012232.1 | 585 | gagcctcctggacaaaatcatcg | 337794 | - | see also 23489 line |
| 53058 | PTTG3 | NM_021000.1 | 148 | ttcgatgctcccacatccttacc | 380355 | - | - |
| 53059 | PTTG3 | NM_021000.1 | 116 | ctcaagtttcaatatcatgtttt | 380354 | - | see also 53058 line |
| 53060 | PTTG3 | NM_021000.1 | 46 | atccttgtggctacaaaggatgg | 380353 | - | see also 53058 line |
| 53061 | PTX1 | NM_016570.1 | 355 | tggagcggatgtattggatttag | 358163 | - | see also 6917 line |
| 53062 | PTX1 | NM_016570.1 | 91 | gaggcgactgaatcggaaaaaaa | 358149 | - | see also 6917 line |
| 53063 | PURB | NM_033224.2 | 364 | cgcgctcaagagcgaattcttgg | 403615 | - | see also 9382 line |
| 53064 | PURB | NM_033224.2 | 268 | ctcgctgggcgacttcatagaac | 403612 | - | see also 9382 line |
| 53065 | PVRL3 | NM_015480.1 | 596 | aacccgttgcacatattgactgg | 353447 | - | see also 6429 line |
| 53066 | PVRL3 | NM_015480.1 | 713 | ttgctagaggaaggcgaattact | 353453 | - | see also 6429 line |
| 53067 | PWDMP | NM_025132.2 | 2513 | ttcgcgggtgattatgtaaatgc | 393890 | - | see also 8867 line |
| 53068 | PWDMP | NM_025132.2 | 1572 | atgataagtgccgtatcttatgc | 393866 | - | see also 8867 line |
| 53069 | PWDMP | NM_025132.2 | 880 | tggccgcatcatgattggttttt | 393848 | - | see also 8867 line |
| 53070 | PWWP1 | NM_138574.1 | 117 | accgctaccaggtgtttttcttc | 408492 | - | - |
| 53071 | PYC1 | NM_152901.1 | 180 | cgcgctcgggcagctagatatcg | 421533 | - | see also 23494 line |
| 53072 | PYM | NM_032345.1 | 179 | tacggagacaggcaagtatatcg | 399852 | - | see also 23495 line |
| 53073 | PYM | NM_032345.1 | 270 | gaggaggtcccagtatatgaaaa | 399855 | - | see also 23495 line |
| 53074 | R29124_1 | NM_033543.1 | 230 | agctaatcgcagcatatgtaata | 404117 | - | see also 23496 line |
| 53075 | R29124_1 | NM_033543.1 | 402 | atctcaccatctccgtgtatacg | 404120 | - | see also 23496 line |
| 53076 | R30953_1 | NM_019612.1 | 360 | cccggctgacaagtacctaaagc | 375512 | - | - |
| 53077 | R30953_1 | NM_019612.1 | 473 | gccagggcaagaagttctacttt | 375513 | - | see also 53076 line |
| 53078 | Rab11-FIP2 | NM_014904.1 | 1019 | gcccgatgccaatagtgaatttt | 345465 | - | see also 23497 line |
| 53079 | Rab11-FIP2 | NM_014904.1 | 881 | gaccgcaagtatgtttgacttat | 345459 | - | see also 23497 line |
| 53080 | Rab11-FIP2 | NM_014904.1 | 1018 | tgcccgatgccaatagtgaattt | 345464 | - | see also 23497 line |
| 53081 | RAB15 | NM_198686.1 | 217 | gaccatcacaaagcagtactatc | 439869 | - | see also 10348 line |
| 53082 | RAB3IP | NM_022456.2 | 383 | ttcctacacaacccgtgtattca | 382840 | - | see also 23500 line |
| 53083 | RAB3IP | NM_022456.2 | 402 | ttcatcccccagacgtttaaatt | 382841 | - | see also 23500 line |
| 53084 | RABAC1 | NM_006423.1 | 345 | cgcctgttacattctctatctgc | 335540 | - | see also 23501 line |
| 53085 | RAD9B | NM_152442.1 | 1095 | aaggtcagatctgattgaaaaaa | 416858 | - | see also 23502 line |
| 53086 | RAD9B | NM_152442.1 | 1172 | caccaagaaggctttatcctaag | 416860 | - | see also 23502 line |
| 53087 | RAD9B | NM_152442.1 | 1027 | gccacattagctgatgaacaaag | 416855 | - | see also 23502 line |
| 53088 | RAI14 | NM_015577.1 | 1242 | ggcggaagcagacctaagctttg | 354260 | - | see also 23503 line |
| 53089 | RAI14 | NM_015577.1 | 1098 | tactacaggtgctgatagcttat | 354254 | - | see also 23503 line |

## Figure 46

| | | | | | | | |
|---|---|---|---|---|---|---|---|
| 53090 | RAI14 | NM_015577.1 | 2462 | tacctcggtcttcctatgaaaaa | 354279 | - | see also 23503 line |
| 53091 | RAI16 | NM_022749.4 | 1730 | cacatacgtccacgatgcttatg | 383897 | - | see also 23504 line |
| 53092 | RAI16 | NM_022749.4 | 2048 | cggggacttgatgcagagaatcc | 383899 | - | see also 23504 line |
| 53093 | RAM2 | NM_018719.2 | 1025 | gacgacgtcaccgtatatcttct | 372948 | - | see also 23505 line |
| 53094 | RAM2 | NM_018719.2 | 585 | ttctagcgcacgcttacagaatg | 372939 | - | see also 23505 line |
| 53095 | RAM2 | NM_018719.2 | 1092 | tgccataactgttcgagataaaa | 372952 | - | see also 23505 line |
| 53096 | RAMP | NM_016448.1 | 1970 | ctcctatcagtccgtatgcttca | 357552 | - | see also 6861 line |
| 53097 | RAMP | NM_016448.1 | 857 | atcgacaagaacccatagcatcc | 357521 | - | see also 6861 line |
| 53098 | RANBP9 | NM_005493.2 | 1483 | ctgttagtcctcgacctttagt | 333887 | - | see also 3817 line |
| 53099 | RANBP9 | NM_005493.2 | 1111 | cacagatagatcgatttcctatc | 333872 | - | see also 3817 line |
| 53100 | RANGNRF | NM_014185.1 | 295 | ggctgcgcggtaccactttgagg | 339954 | - | see also 23508 line |
| 53101 | RANGNRF | NM_014185.1 | 203 | cccggacaatcaagaagttttct | 339951 | - | see also 23508 line |
| 53102 | RANGNRF | NM_014185.1 | 197 | accggtcccggacaatcaagaag | 339948 | - | see also 23508 line |
| 53103 | RAP140 | NM_015224.1 | 1194 | gtctcgagtcctacatgctttgt | 350007 | - | see also 23509 line |
| 53104 | RAP140 | NM_015224.1 | 2912 | caccagtcagtcctaacgattct | 350056 | - | see also 23509 line |
| 53105 | raptor | NM_020761.1 | 3392 | tacgagattggacgaccaaatat | 378588 | - | see also 7946 line |
| 53106 | raptor | NM_020761.1 | 1955 | ccggaccatgacggctttcattc | 378574 | - | see also 7946 line |
| 53107 | RARG-1 | NM_016167.3 | 404 | agcttcacagactaacatcaaga | 356508 | - | see also 23511 line |
| 53108 | RARG-1 | NM_016167.3 | 767 | aagacggtggatggtcagaaaga | 356517 | - | see also 23511 line |
| 53109 | RARRES3 | NM_004585.2 | 125 | gagcactgggccctgtatatagg | 332624 | - | see also 23512 line |
| 53110 | RARRES3 | NM_004585.2 | 324 | ccgtggaggtgatcatcagttct | 332627 | - | see also 23512 line |
| 53111 | RB1CC1 | NM_014781.1 | 3140 | aggtaaacttgacggactaataa | 343346 | - | see also 5953 line |
| 53112 | RBPMS2 | NM_194272.1 | 514 | ctgaacggtattcgctttgatcc | 436174 | - | see also 23514 line |
| 53113 | RBPMS2 | NM_194272.1 | 526 | cgctttgatcccgaaaatccaca | 436175 | - | see also 23514 line |
| 53114 | RBT1 | NM_013368.2 | 391 | ctccgcatctccctagacaaagt | 338845 | - | see also 23515 line |
| 53115 | RBT1 | NM_013368.2 | 752 | ttgacacatctgcggtagaaaag | 338848 | - | see also 23515 line |
| 53116 | RC3 | NM_015263.1 | 7556 | ttcctcccgaacttagtatgtgg | 350648 | - | see also 23516 line |
| 53117 | RC3 | NM_015263.1 | 5134 | aacacgcttcgaagatgcattga | 350557 | - | see also 23516 line |
| 53118 | RCP | NM_025151.2 | 857 | aacagcgagtacggatcttaagc | 394076 | - | see also 23517 line |
| 53119 | RCP | NM_025151.2 | 860 | agcgagtacggatcttaagcaac | 394077 | - | see also 23517 line |
| 53120 | RCP | NM_025151.2 | 936 | ggccttcggtctaagaatgatgt | 394082 | - | see also 23517 line |
| 53121 | REC14 | NM_025234.1 | 79 | gaccaaccagtacggtattctct | 394492 | - | see also 23518 line |
| 53122 | REC14 | NM_025234.1 | 823 | cagttcgtctgacaaaagtgtaa | 394507 | - | see also 23518 line |
| 53123 | REC14 | NM_025234.1 | 821 | tccagttcgtctgacaaaagtgt | 394506 | - | see also 23518 line |

Figure 46

| | | | | | | | |
|---|---|---|---|---|---|---|---|
| 53124 | RFPL1 | NM_021026.1 | 1069 | gtccacctaatggtgataagagt | 380367 | - | see also 23519 line |
| 53125 | RGMA | NM_020211.1 | 833 | gccgctcatcgacaataattacc | 376159 | - | see also 23520 line |
| 53126 | RGMA | NM_020211.1 | 695 | ccccgagatctgccattacgaga | 376155 | - | see also 23520 line |
| 53127 | RGPR | NM_033127.1 | 507 | aaggcaacggagtccttatatct | 403505 | - | see also 9373 line |
| 53128 | RGPR | NM_033127.1 | 509 | ggcaacggagtccttatatctgg | 403506 | - | see also 9373 line |
| 53129 | RhoGAP2 | NM_021226.2 | 1213 | cagaatctggcaaccgtttttgg | 380853 | - | see also 23522 line |
| 53130 | RhoGAP2 | NM_021226.2 | 1225 | accgtttttggacctaacattct | 380855 | - | see also 23522 line |
| 53131 | RIC-8 | NM_021932.4 | 886 | aggagtgcgcctgctaactgaca | 381369 | - | see also 8203 line |
| 53132 | RIC-8 | NM_021932.4 | 1328 | ctgagcgtgctgactgaatgtgc | 381373 | - | see also 8203 line |
| 53133 | RIC3 | NM_024557.2 | 177 | tccacctatgatgcatcatcacc | 387251 | - | see also 23523 line |
| 53134 | RIC3 | NM_024557.2 | 147 | gccgacacctgaaggaaaattgg | 387250 | - | see also 23523 line |
| 53135 | RIC3 | NM_024557.2 | 752 | agcgtaggcaagaaacaatcttg | 387257 | - | see also 23523 line |
| 53136 | RICS | NM_014715.2 | 4187 | gccgggtccactacaggtatagc | 342762 | - | see also 5906 line |
| 53137 | RICS | NM_014715.2 | 4600 | gacgacttgggtgggatctatgt | 342774 | - | see also 5906 line |
| 53138 | RIMS4 | NM_182970.2 | 222 | tggctcagagggcaaccttaact | 435497 | - | see also 23524 line |
| 53139 | RIMS4 | NM_182970.2 | 463 | aaggcctacctgctagagaatgg | 435498 | - | see also 23524 line |
| 53140 | RIPX | NM_014961.1 | 1839 | atcagatggctgctaccattaaa | 346374 | - | see also 6152 line |
| 53141 | RIPX | NM_014961.1 | 1268 | gacgggaacagcagtaaaggtac | 346360 | - | see also 6152 line |
| 53142 | RNF10 | NM_014868.3 | 2696 | agcgacggggagagtgataattc | 345047 | - | see also 23527 line |
| 53143 | RNF10 | NM_014868.3 | 2699 | gacggggagagtgataattcaga | 345048 | - | see also 23527 line |
| 53144 | RNF111 | NM_017610.6 | 1507 | cacggaaccgcagtaggatttct | 359468 | - | see also 23528 line |
| 53145 | RNF111 | NM_017610.6 | 1640 | tactagcgcttccattaacaatt | 359474 | - | see also 23528 line |
| 53146 | RNF121 | NM_018320.3 | 969 | gtgtagtccaaggcatcaactac | 370299 | - | see also 7403 line |
| 53147 | RNF121 | NM_018320.3 | 893 | gagaggcctcacgtcatgtatgg | 370298 | - | see also 7403 line |
| 53148 | RNF122 | NM_024787.2 | 501 | tccgctcaacatctatatggtca | 390668 | - | see also 8747 line |
| 53149 | RNF122 | NM_024787.2 | 497 | accttccgctcaacatctatatg | 390667 | - | see also 8747 line |
| 53150 | RNF123 | NM_022064.2 | 3630 | cagctacgctcaatatgctatct | 381822 | - | see also 23531 line |
| 53151 | RNF123 | NM_022064.2 | 3018 | ggcttcgggtaccgctatacacg | 381816 | - | see also 23531 line |
| 53152 | RNF125 | NM_017831.2 | 529 | gacccgctgtggccacgtattct | 362868 | - | see also 23532 line |
| 53153 | RNF125 | NM_017831.2 | 1035 | tagaccggtcacttcttgaatat | 362885 | - | see also 23532 line |
| 53154 | RNF126 | NM_017876.3 | 123 | cgcatcccggacggtacttctgc | 363353 | - | - | | - |
| 53155 | RNF126 | NM_017876.3 | 196 | aagatgcgagtctggtttttatcg | 363355 | - | see also 53154 line |
| 53156 | RNF126 | NM_017876.3 | 199 | atgcgagtctggtttttatcgagg | 363356 | - | see also 53154 line |
| 53157 | RNF135 | NM_032322.3 | 1083 | aagtggacactaggaattgcagc | 399719 | - | see also 23533 line |
| 53158 | RNF135 | NM_032322.3 | 599 | cagaggcccctatcagaatctgg | 399715 | - | see also 23533 line |
| 53159 | RNF141 | NM_016422.3 | 435 | atcacggatcatgaatttatacc | 357427 | - | see also 6848 line |
| 53160 | RNF146 | NM_030963.2 | 745 | tacgatgagcgcactagtagaga | 395880 | - | see also 8983 line |

Figure 46

| 53161 | RNF146 | NM_030963.2 | 991 | gacggagcggacagtgtatcagc | 395889 | - | see also 8983 line |
|---|---|---|---|---|---|---|---|
| 53162 | RNF148 | NM_198085.1 | 236 | acccccttcgacgcatagttctgt | 437012 | - | see also 23536 line |
| 53163 | RNF148 | NM_198085.1 | 1023 | atgtagtacgcattttaacttgc | 437025 | - | see also 23536 line |
| 53164 | RNF152 | NM_173557.1 | 996 | agcgcttcactgtgatatcctgt | 426067 | - | see also 23537 line |
| 53165 | RNF152 | NM_173557.1 | 974 | cacaacatgtcttgcatttctaa | 426066 | - | see also 23537 line |
| 53166 | RNF17 | NM_031277.1 | 480 | caccatagtttcgaacagttaag | 396387 | - | see also 23538 line |
| 53167 | RNF17 | NM_031277.1 | 745 | tacaattatcgccttctctaaga | 396393 | - | see also 23538 line |
| 53168 | RNF17 | NM_031277.1 | 307 | aactgcagcctaagacgataaag | 396383 | - | see also 23538 line |
| 53169 | RNF2 | NM_007212.2 | 985 | tggtaacgccactgttgatcact | 337105 | - | see also 5019 line |
| 53170 | RNF2 | NM_007212.2 | 989 | aacgccactgttgatcacttatc | 337106 | - | see also 5019 line |
| 53171 | RNF20 | NM_019592.5 | 400 | atccgtatcatccttaaacgtta | 375379 | - | see also 7717 line |
| 53172 | RNF20 | NM_019592.5 | 1247 | cacagttctccgtcttgtataat | 375405 | - | see also 7717 line |
| 53173 | RNF24 | NM_007219.2 | 575 | ggctggaggttcgtaaagtgtgt | 337244 | - | see also 5032 line |
| 53174 | RNF24 | NM_007219.2 | 385 | tacttgattaggctaagacatca | 337238 | - | see also 5032 line |
| 53175 | RNF3 | NM_006315.3 | 907 | ggcgaccgtcttgcatctgaaga | 335207 | - | see also 4420 line |
| 53176 | RNF3 | NM_006315.3 | 753 | aagcagacgacagttcaaacaaa | 335205 | - | see also 4420 line |
| 53177 | RNF31 | NM_017999.3 | 484 | ctggcgtggtgtcaagtttaata | 365192 | - | see also 23543 line |
| 53178 | RNF31 | NM_017999.3 | 771 | cagaatttgaccccctattgaga | 365197 | - | see also 23543 line |
| 53179 | RNF32 | NM_030936.2 | 621 | gtgttccacaaagcatgtcttca | 395589 | - | see also 23544 line |
| 53180 | RNF32 | NM_030936.2 | 939 | cagtgcttggccataaatcgaag | 395594 | - | see also 23544 line |
| 53181 | RNF36 | NM_080745.2 | 734 | agcgtctggcatggtgacattaa | 406733 | - | see also 23545 line |
| 53182 | RNF36 | NM_080745.2 | 675 | cactaactctggaccctaaaaca | 406732 | - | see also 23545 line |
| 53183 | RNF36 | NM_080745.2 | 615 | gtcctatccagtacatggtatgg | 406731 | - | see also 23545 line |
| 53184 | RNF38 | NM_022781.3 | 1532 | ttcttatcggttcaatcctaaca | 384218 | - | see also 8473 line |
| 53185 | RNF38 | NM_022781.3 | 672 | ttccaccctccgaatgtatctcc | 384200 | - | see also 8473 line |
| 53186 | RNF40 | NM_014771.2 | 3096 | tgcccacgacttccatcgtatct | 343208 | - | see also 23546 line |
| 53187 | RNF40 | NM_014771.2 | 1788 | cagtaccccgacaacagaaagg | 343203 | - | see also 23546 line |
| 53188 | RNF41 | NM_005785.2 | 401 | atgttgacgaagatcttatctgc | 334253 | - | see also 4044 line |
| 53189 | RNF41 | NM_005785.2 | 413 | atcttatctgccctatttgcagt | 334255 | - | see also 4044 line |
| 53190 | RNF44 | NM_014901.3 | 1272 | tcctttggtgtgccatattctca | 345446 | - | see also 6084 line |
| 53191 | RNF8 | NM_003958.2 | 429 | acctttaagggtctattccattc | 332306 | - | see also 23549 line |
| 53192 | RNF8 | NM_003958.2 | 647 | ctgaaggcccctcaaatttgaaa | 332308 | - | see also 23549 line |
| 53193 | ROBO4 | NM_019055.4 | 890 | ctgcgcctgcccaatcttacacg | 374784 | - | see also 7684 line |
| 53194 | ROBO4 | NM_019055.4 | 1019 | aagactacgagttcaaagtgaga | 374787 | - | see also 7684 line |
| 53195 | RP4-622L5 | NM_019118.2 | 373 | tgctcaccgtgataggaatttca | 375304 | - | see also 23551 line |
| 53196 | RP4-622L5 | NM_019118.2 | 377 | caccgtgataggaatttcactct | 375306 | - | see also 23551 line |
| 53197 | RPC2 | NM_018082.2 | 2976 | gacctcgttcgccatggttataa | 366482 | - | see also 23552 line |

Figure 46

| | | | | | | | |
|---|---|---|---|---|---|---|---|
| 53198 | RPC2 | NM_018082.2 | 2983 | ttcgccatggttataactacttg | 366485 | - | see also 23552 line |
| 53199 | RPESP | NM_153225.1 | 759 | gactgcgggcacacctatgttcc | 422326 | - | - |
| 53200 | RPF1 | NM_025065.5 | 189 | agcggcgacacttaatgttcacg | 393441 | - | see also 8840 line |
| 53201 | RPF1 | NM_025065.5 | 186 | aacagcggcgacacttaatgttc | 393440 | - | see also 8840 line |
| 53202 | RPF1 | NM_025065.5 | 849 | tccacaatcaacgggattacata | 393458 | - | see also 8840 line |
| 53203 | RPGRIP1 | NM_020366.2 | 1789 | acccgaccgttgtcgttatgttt | 376936 | - | see also 7849 line |
| 53204 | RPGRIP1 | NM_020366.2 | 1790 | cccgaccgttgtcgttatgtttg | 376937 | - | see also 7849 line |
| 53205 | RPIB9 | NM_138290.1 | 729 | ctcgtagcttctgggactatatc | 407460 | - | see also 9593 line |
| 53206 | RPIB9 | NM_138290.1 | 1778 | taccttgcaagtcctacaacaga | 407491 | - | see also 9593 line |
| 53207 | Rpp25 | NM_017793.1 | 1218 | ccgctagtctcagcgtacttaag | 362453 | - | see also 23554 line |
| 53208 | Rpp25 | NM_017793.1 | 1219 | cgctagtctcagcgtacttaaga | 362454 | - | see also 23554 line |
| 53209 | RSHL2 | NM_031924.3 | 495 | tcctcgcgtcgcggaatccttgc | 397354 | - | see also 23555 line |
| 53210 | RSHL2 | NM_031924.3 | 982 | tgctgatcgcataatagaagttg | 397367 | - | see also 23555 line |
| 53211 | RTBDN | NM_031429.1 | 359 | gacgcttctcggctacagaatcc | 396946 | - | see also 23556 line |
| 53212 | RTBDN | NM_031429.1 | 781 | ttcgccaactgcgaagatgatat | 396949 | - | see also 23556 line |
| 53213 | RTKN2 | NM_145307.2 | 469 | atcacgacgctatgccattttt | 414297 | - | see also 23557 line |
| 53214 | RTKN2 | NM_145307.2 | 285 | ctcgactaatggcctatacatcg | 414291 | - | see also 23557 line |
| 53215 | RTP801 | NM_019058.1 | 570 | agccaggtgggcaaagaactact | 374802 | - | see also 23558 line |
| 53216 | RTTN | NM_173630.2 | 2377 | atcggtccgttcgctagcattaa | 426764 | - | see also 10092 line |
| 53217 | RTTN | NM_173630.2 | 2379 | cggtccgttcgctagcattaaag | 426766 | - | see also 10092 line |
| 53218 | RWDD3 | NM_015485.2 | 448 | cacgaccatggatgatggattgt | 353498 | - | see also 23560 line |
| 53219 | RWDD3 | NM_015485.2 | 246 | tacctggtatctcgattaactct | 353493 | - | see also 23560 line |
| 53220 | RWDD3 | NM_015485.2 | 256 | ctcgattaactctgaacagttga | 353494 | - | see also 23560 line |
| 53221 | RYD5 | NM_145651.1 | 87 | ggggaggacaacgatgagttttt | 414628 | - | see also 23561 line |
| 53222 | RYD5 | NM_145651.1 | 111 | atggacttcctgcaaacactact | 414629 | - | see also 23561 line |
| 53223 | SACM1L | NM_014016.1 | 147 | agcagatgacgtacttaccattg | 339147 | - | see also 5456 line |
| 53224 | SACM1L | NM_014016.1 | 421 | ttcctagcgatgctaaaccatgt | 339156 | - | see also 5456 line |
| 53225 | SAGE | NM_018666.1 | 1546 | ttctatccgggcttattaatatg | 372422 | - | see also 23563 line |
| 53226 | SAGE | NM_018666.1 | 1094 | accgcaacctaataacgtattgt | 372414 | - | see also 23563 line |
| 53227 | SAMD3 | NM_152552.1 | 441 | cagtggacgaagtcctatgtttt | 418040 | - | see also 23564 line |
| 53228 | SAMD3 | NM_152552.1 | 114 | gccgctcttcttgcacttaatga | 418033 | - | see also 23564 line |
| 53229 | SAMD3 | NM_152552.1 | 113 | ggccgctcttcttgcacttaatg | 418032 | - | see also 23564 line |
| 53230 | SAMD4 | NM_015589.2 | 1058 | gaccgaacctgacatcaacaaca | 354351 | - | see also 23565 line |
| 53231 | SAMD4 | NM_015589.2 | 382 | cacgcgtcatggggaaagtgtgc | 354343 | - | see also 23565 line |
| 53232 | SAMD6 | NM_173551.1 | 917 | aagggacgcgagagacaaatttt | 425965 | - | see also 23566 line |
| 53233 | SAMD6 | NM_173551.1 | 534 | ctcctgttggcaagcagaaattg | 425959 | - | see also 23566 line |
| 53234 | SAMD7 | NM_182610.1 | 1369 | ttcgaggcactatgggattaaag | 434650 | - | see also 23567 line |

Figure 46

| | | | | | | | |
|---|---|---|---|---|---|---|---|
| 53235 | SAMD7 | NM_182610.1 | 811 | gtcgtcgactcaggaaaaataca | 434641 | - | see also 23567 line |
| 53236 | SAMD8 | NM_144660.1 | 1021 | gtcaccgaatgtaagtatctttt | 411109 | - | see also 9727 line |
| 53237 | SAMD8 | NM_144660.1 | 439 | gactgtgacggaccccataactga | 411088 | - | see also 9727 line |
| 53238 | SAMD8 | NM_144660.1 | 986 | gccacacagtcgtcctaactatg | 411107 | - | see also 9727 line |
| 53239 | SAMSN1 | NM_022136.2 | 458 | ttcgatcgtttcggaataattc | 382432 | - | see also 8300 line |
| 53240 | SAMSN1 | NM_022136.2 | 459 | tcgatcgtttcggaataattct | 382433 | - | see also 8300 line |
| 53241 | SAMSN1 | NM_022136.2 | 462 | atcgttttcggaataattcttta | 382434 | - | see also 8300 line |
| 53242 | SAP130 | NM_024545.2 | 1738 | agcgtctaccgtccacctaaacc | 386976 | - | see also 8636 line |
| 53243 | SAP130 | NM_024545.2 | 1783 | tgcatcgcatgctcgacatattc | 386977 | - | see also 8636 line |
| 53244 | SAS10 | NM_020368.1 | 761 | gacacgggtcgtgaaggatttgg | 377016 | - | see also 23571 line |
| 53245 | SAS10 | NM_020368.1 | 1067 | tacctaccgaaatttgatcaaca | 377025 | - | see also 23571 line |
| 53246 | SBBI54 | NM_138334.1 | 423 | aggtggacggtgtctactacaac | 407529 | - | see also 9599 line |
| 53247 | SBBI54 | NM_138334.1 | 224 | accggcaactatgatgtcaatgt | 407528 | - | see also 9599 line |
| 53248 | SCAMP-4 | NM_079834.1 | 374 | gagccgacagctcctttaatttc | 406365 | - | see also 9510 line |
| 53249 | SCAMP5 | NM_138967.1 | 445 | tccttcttcggaacgaacattgg | 409323 | - | see also 23573 line |
| 53250 | SCAMP5 | NM_138967.1 | 294 | ctggtttcggcccatttacaagg | 409321 | - | see also 23573 line |
| 53251 | SCAPIN1 | NM_080672.2 | 1190 | agcgaactgccgctaaggaatct | 406632 | - | see also 23574 line |
| 53252 | SCAPIN1 | NM_080672.2 | 1284 | ggcgctgaacgactccattattt | 406636 | - | see also 23574 line |
| 53253 | SCDR10 | NM_198533.1 | 1078 | aagcaaaaccgagaaaaacgacg | 439265 | - | see also 23575 line |
| 53254 | SCDR10 | NM_198533.1 | 1176 | ctcacctgtttggccatgattga | 439266 | - | see also 23575 line |
| 53255 | SCDR10 | NM_198533.1 | 1050 | agcccaagatgaagtcatcaaga | 439264 | - | see also 23575 line |
| 53256 | SCG3 | NM_013243.2 | 418 | ctcgggaccggcacttggattct | 338372 | - | see also 5359 line |
| 53257 | SCG3 | NM_013243.2 | 1784 | aggccatcaagcgcatttatagc | 338405 | - | see also 5359 line |
| 53258 | SCGB3A2 | NM_054023.2 | 191 | gacaagttggcacctttacctct | 405972 | - | see also 23577 line |
| 53259 | SCGB3A2 | NM_054023.2 | 291 | aggggctaaggaagtgtgtaaat | 405974 | - | see also 23577 line |
| 53260 | SCML4 | NM_198081.1 | 887 | cccgcactagcccatgtcttct | 436996 | - | see also 23578 line |
| 53261 | SCML4 | NM_198081.1 | 705 | gacagtcaccaccgaagagtacc | 436995 | - | see also 23578 line |
| 53262 | SCML4 | NM_198081.1 | 300 | cacagtctgcctctacatcaaca | 436993 | - | see also 23578 line |
| 53263 | SCN4B | NM_174934.1 | 595 | ttcctccaagtcgttgatagact | 428731 | - | see also 23579 line |
| 53264 | SCN4B | NM_174934.1 | 373 | gacgcattcaagattctcataga | 428727 | - | see also 23579 line |
| 53265 | SCN4B | NM_174934.1 | 371 | gtgacgcattcaagattctcata | 428726 | - | see also 23579 line |
| 53266 | SCNM1 | NM_024041.1 | 97 | gacgattggagtcaactcaatgt | 385604 | - | see also 23580 line |
| 53267 | SCNM1 | NM_024041.1 | 185 | ttcgggatggacgctttgcttgt | 385607 | - | see also 23580 line |
| 53268 | SCOC | NM_032547.1 | 459 | atgtcagcttctagtgtttttca | 400645 | - | see also 23581 line |
| 53269 | SCOC | NM_032547.1 | 252 | ttgttacccaagatgatgaatgc | 400643 | - | see also 23581 line |
| 53270 | SDBCAG84 | NM_015966.2 | 512 | ctgaggcagaagatatcaagtgc | 355659 | - | see also 23582 line |

Figure 46

| | | | | | | | |
|---|---|---|---|---|---|---|---|
| 53271 | SDBCAG84 | NM_015966.2 | 674 | atggcttcttggaagtcaataag | 355663 | - | see also 23582 line |
| 53272 | SDCCAG1 | NM_004713.2 | 1155 | caggtagttcgaagtgctttagc | 332775 | - | see also 3231 line |
| 53273 | SDCCAG1 | NM_004713.2 | 1878 | tgggaatgcacgagttatcactag | 332799 | - | see also 3231 line |
| 53274 | SDCCAG16 | NM_006649.2 | 1715 | aacctcctaaccacacaatctcc | 336039 | - | see also 23584 line |
| 53275 | SDCCAG16 | NM_006649.2 | 1227 | aggcccatggagtttctgaaagt | 336037 | - | see also 23584 line |
| 53276 | SDCCAG8 | NM_006642.1 | 339 | tactatgcacgaccttgttcata | 335985 | - | see also 23585 line |
| 53277 | SDCCAG8 | NM_006642.1 | 346 | cacgaccttgttcatactattaa | 335988 | - | see also 23585 line |
| 53278 | SDCCAG8 | NM_006642.1 | 344 | tgcacgaccttgttcatactatt | 335987 | - | see also 23585 line |
| 53279 | SDK1 | NM_152744.2 | 2340 | ctccggctcgtacctatcaattc | 420358 | - | see also 9941 line |
| 53280 | SDK1 | NM_152744.2 | 2475 | ggcggactaatcagtccattatg | 420363 | - | see also 9941 line |
| 53281 | SDK1 | NM_152744.2 | 2341 | tccggctcgtacctatcaattcc | 420359 | - | see also 9941 line |
| 53282 | SDK2 | NM_019064.2 | 2820 | caccggcttgggcaaatacgtgc | 374864 | - | see also 23587 line |
| 53283 | SDK2 | NM_019064.2 | 3687 | cacgcttcgaggcatcaacaacc | 374868 | - | see also 23587 line |
| 53284 | SDK2 | NM_019064.2 | 4734 | cgcgtcaacgactatggtttcg | 374874 | - | see also 23587 line |
| 53285 | SDOS | NM_032349.2 | 539 | ccctcaaggtgctcaacatgatg | 399869 | - | - |
| 53286 | SDOS | NM_032349.2 | 537 | tgccctcaaggtgctcaacatga | 399868 | - | see also 53285 line |
| 53287 | SE57-1 | NM_025214.1 | 245 | gtctacgtccgctcacattattg | 394425 | - | see also 23588 line |
| 53288 | SE57-1 | NM_025214.1 | 249 | acgtccgctcacattattgaaga | 394427 | - | see also 23588 line |
| 53289 | SEC13L | NM_031216.2 | 918 | tggtcagttcgataatcataat | 395999 | - | see also 8992 line |
| 53290 | SEC13L | NM_031216.2 | 199 | caggatcagagcgttaagtct | 395973 | - | see also 8992 line |
| 53291 | SEC31L2 | NM_015490.3 | 374 | gcggggacaatggcatgctatt | 353513 | - | see also 23590 line |
| 53292 | SEC31L2 | NM_015490.3 | 2425 | cagctaagagatcggctttca | 353552 | - | see also 23590 line |
| 53293 | SELH | NM_170746.1 | 542 | gccccacgcaaactcaaattcc | 424155 | - | see also 23591 line |
| 53294 | SELK | NM_021237.2 | 145 | ttctgggaatagctgagtttgt | 380878 | - | - |
| 53295 | SELM | NM_080430.1 | 269 | cacgcaggacattccatttctatc | 406376 | - | see also 23592 line |
| 53296 | SELM | NM_080430.1 | 270 | acgcaggacattccattctatca | 406377 | - | see also 23592 line |
| 53297 | SELV | NM_182704.1 | 970 | tccgaatcacctactctttgagg | 434918 | - | see also 23593 line |
| 53298 | SELV | NM_182704.1 | 930 | ttcggtacattctactgaaaaag | 434915 | - | see also 23593 line |
| 53299 | SEMA3D | NM_152754.1 | 1061 | aacggaatatgggctactgattc | 420638 | - | see also 9948 line |
| 53300 | SEMA3D | NM_152754.1 | 430 | caggaggaccaacgttcaagaga | 420609 | - | see also 9948 line |
| 53301 | SES1 | NM_014766.2 | 768 | gagagggcgagttcaattttcc | 343177 | - | see also 23595 line |
| 53302 | SES1 | NM_014766.2 | 979 | aagtctccgtgcattcactact | 343179 | - | see also 23595 line |
| 53303 | Ses2 | NM_138355.1 | 217 | ctccagtgcacctacattgaagt | 407568 | - | see also 23596 line |
| 53304 | Ses2 | NM_138355.1 | 944 | agccctgcgtgcacttcttacc | 407571 | - | see also 23596 line |

Figure 46

| | | | | | | | |
|---|---|---|---|---|---|---|---|
| 53305 | SES3 | NM_024583.2 | 133 | aacagtcgataacaggattattt | 387575 | - | see also 23597 line |
| 53306 | SES3 | NM_024583.2 | 812 | gagggctacaagcttcttaaataa | 387595 | - | see also 23597 line |
| 53307 | SETMAR | NM_006515.1 | 892 | ctctgtactgccccgtagaaaag | 335725 | - | see also 4558 line |
| 53308 | SETMAR | NM_006515.1 | 74 | cgcgtgcggccaggaaaacttgc | 335697 | - | see also 4558 line |
| 53309 | SEZ6 | NM_178860.2 | 1360 | ccccgtcgtccagcttatggaga | 432032 | - | see also 10223 line |
| 53310 | SFMBT1 | NM_016329.1 | 1441 | gaggtgcttcctccgttaattt | 356988 | - | see also 6811 line |
| 53311 | SFMBT1 | NM_016329.1 | 1681 | tcctcgccgagcacgagtatata | 356993 | - | see also 6811 line |
| 53312 | SGTB | NM_019072.1 | 818 | atggctagcttgataaataatcc | 374919 | - | see also 7693 line |
| 53313 | SGTB | NM_019072.1 | 210 | ttcgtttctacggaacaagt | 374894 | - | see also 7693 line |
| 53314 | SH2BP1 | NM_014633.1 | 880 | atcctagcaacctatggtattg | 341483 | - | see also 5801 line |
| 53315 | SH2BP1 | NM_014633.1 | 1572 | tccgttaccacgtcatataatct | 341507 | - | see also 5801 line |
| 53316 | SH3GLB1 | NM_016009.2 | 689 | atcgtcaagcagaagattaccaga | 355836 | - | see also 6608 line |
| 53317 | SH3GLB1 | NM_016009.2 | 749 | accttcgctctgaatgacttt | 355837 | - | see also 6608 line |
| 53318 | SH3GLB2 | NM_020145.2 | 1163 | agctcgggtgctctatgactacg | 375689 | - | see also 7766 line |
| 53319 | SH3MD1 | NM_014631.1 | 1297 | cccgccatacatacgataagc | 341443 | - | see also 23603 line |
| 53320 | SH3MD1 | NM_014631.1 | 2325 | ggccagccacctcgtacatgaca | 341446 | - | see also 23603 line |
| 53321 | SH3MD2 | NM_020870.1 | 291 | atgtaggttctcgaaatgaact | 379722 | - | see also 23604 line |
| 53322 | SH3MD2 | NM_020870.1 | 263 | tacgtttgcaaggcgatgtttgc | 379720 | - | see also 23604 line |
| 53323 | SH3YL1 | NM_015677.1 | 1140 | aagtgacagcgctgtattcattt | 354977 | - | see also 23605 line |
| 53324 | SH3YL1 | NM_015677.1 | 868 | gcctgctcaagccgaagatcttt | 354969 | - | see also 23605 line |
| 53325 | SIP | NM_014412.1 | 197 | atgccctacagctgaaaaatcc | 340555 | - | see also 5666 line |
| 53326 | SIPL | NM_018269.1 | 449 | cacggtggacgagaagaactaca | 369599 | - | see also 23607 line |
| 53327 | SIPL | NM_018269.1 | 208 | tcgaagagagaggaactactcc | 369597 | - | see also 23607 line |
| 53328 | SITPEC | NM_016581.2 | 960 | ctccgcaacaagtgtgtatta | 358221 | - | see also 23608 line |
| 53329 | SITPEC | NM_016581.2 | 952 | cctgtggctccgcaacaagtgt | 358220 | - | see also 23608 line |
| 53330 | SIX5 | NM_175875.3 | 1610 | gccccacctcccacctattgaac | 429409 | - | transcription_regulator - |
| 53331 | SIX5 | NM_175875.3 | 2160 | cccaccagagggcgtgatgtgt | 429411 | - | see also 53330 line |
| 53332 | SIX5 | NM_175875.3 | 1611 | cccacctcccactattgaacc | 429410 | - | see also 53330 line |
| 53333 | SLAC2-B | NM_015065.1 | 3787 | gacgactagtacgtttctgttt | 347666 | - | see also 6219 line |
| 53334 | SLAC2-B | NM_015065.1 | 3785 | aagacgactagtacgtttctgt | 347665 | - | see also 6219 line |
| 53335 | SLAMF9 | NM_033438.1 | 599 | gggggggatgcacttatacattc | 403998 | - | see also 23610 line |
| 53336 | SLAMF9 | NM_033438.1 | 739 | ttctatgcagatcctaactatgc | 404001 | - | see also 23610 line |
| 53337 | SLC16A10 | NM_018593.2 | 1581 | tcccgtgatccatagtaagaag | 372309 | - | see also 23611 line |
| 53338 | SLC16A10 | NM_018593.2 | 1387 | tcccatagccttgagttagttg | 372299 | - | see also 23611 line |
| 53339 | SLC16A11 | NM_153357.1 | 464 | ggccgcagccttgcgataaaacg | 423041 | - | see also 23612 line |
| 53340 | SLC16A11 | NM_153357.1 | 713 | cagcgatctgctgcatcctacc | 423042 | - | see also 23612 line |

Figure 46

| 53341 | SLC27A1 | NM_198580.1 | 764 | gggcatggacgatcgtcttttct | 439728 | - | see also 23613 line |
|---|---|---|---|---|---|---|---|
| 53342 | SLC27A1 | NM_198580.1 | 573 | ggcgctaaggccctgatctttgg | 439726 | - | see also 23613 line |
| 53343 | SLC35A4 | NM_080670.2 | 1009 | tgctctatggcgctaacaacaac | 406623 | - | see also 23614 line |
| 53344 | SLC35A4 | NM_080670.2 | 1026 | aacaacctggtgatctatcttca | 406624 | - | see also 23614 line |
| 53345 | SLC35B3 | NM_015948.2 | 1236 | tccagttcggacctatggttatg | 355585 | - | see also 6587 line |
| 53346 | SLC35B3 | NM_015948.2 | 1235 | atccagttcggacctatggttat | 355584 | - | see also 6587 line |
| 53347 | SLC35E1 | NM_024881.3 | 1214 | acccaaactcgtacagtttgaac | 391642 | - | see also 23616 line |
| 53348 | SLC35E1 | NM_024881.3 | 1142 | cccagcacggggactatcagtac | 391641 | - | see also 23616 line |
| 53349 | SLC35E1 | NM_024881.3 | 1225 | tacagtttgaaccgctatgatgt | 391644 | - | see also 23616 line |
| 53350 | SLC35E3 | NM_018656.1 | 618 | atcctaaattcttattacgatgt | 372367 | - | see also 23617 line |
| 53351 | SLC35E3 | NM_018656.1 | 626 | ttcttattacgatgtgaagttta | 372368 | - | see also 23617 line |
| 53352 | SLC35F3 | NM_173508.1 | 885 | tacggatgtctccgtgttgttct | 425211 | - | see also 10075 line |
| 53353 | SLC35F3 | NM_173508.1 | 1399 | accagtcagatcgtcttcaatgg | 425221 | - | see also 10075 line |
| 53354 | SLC35F5 | NM_025181.2 | 1348 | tggaggcgttgtactggtaaacc | 394276 | - | see also 23619 line |
| 53355 | SLC35F5 | NM_025181.2 | 1067 | ttgtctcgcatgtcatatcctgt | 394261 | - | see also 23619 line |
| 53356 | SLC36A2 | NM_181776.1 | 544 | tcgtgagcttcttccttattaac | 433170 | - | see also 23620 line |
| 53357 | SLC36A2 | NM_181776.1 | 140 | tgccatcaaattggaccttatgt | 433165 | - | see also 23620 line |
| 53358 | SLC36A3 | NM_181774.1 | 1550 | tgggccttttagggtgtatattt | 433140 | - | see also 23621 line |
| 53359 | SLC36A3 | NM_181774.1 | 803 | ttcgtttctacatgctgataatc | 433133 | - | see also 23621 line |
| 53360 | SLC37A2 | NM_198277.1 | 1455 | ttgctcctttgccggttagtata | 437451 | - | see also 23622 line |
| 53361 | SLC37A2 | NM_198277.1 | 1465 | gccggttagtatacaaagagatc | 437455 | - | see also 23622 line |
| 53362 | SLC43A1 | NM_003627.3 | 613 | cacgttaatggccctcatgattg | 331799 | - | see also 23623 line |
| 53363 | SLC43A1 | NM_003627.3 | 957 | atgttcggggcacctcagaaaac | 331803 | - | see also 23623 line |
| 53364 | SLC4A9 | NM_031467.1 | 1161 | ctcggccgtactctacatttacc | 397154 | - | see also 23624 line |
| 53365 | SLC4A9 | NM_031467.1 | 1159 | ttctcggccgtactctacattta | 397153 | - | see also 23624 line |
| 53366 | SLIC1 | NM_153337.1 | 329 | ccgctggaagcacgtcaaactgc | 422785 | - | see also 23625 line |
| 53367 | SLIC1 | NM_153337.1 | 368 | agctcgcatcgaggagagaaaag | 422786 | - | see also 23625 line |
| 53368 | SMC4L1 | NM_005496.2 | 469 | tcctcggcttatgataactcata | 333919 | - | see also 3826 line |
| 53369 | SMC4L1 | NM_005496.2 | 3706 | cgccgaactcgagcttgtagaca | 334024 | - | see also 3826 line |
| 53370 | SMC4L1 | NM_005496.2 | 3760 | cagtgttcgaccacctaagaaaa | 334025 | - | see also 3826 line |
| 53371 | SMCR5 | NM_144774.1 | 553 | tactttctgcggttcagaatact | 411905 | - | see also 23627 line |
| 53372 | SMCR5 | NM_144774.1 | 454 | tgcgaaggtgtttaagagtaaaa | 411901 | - | see also 23627 line |
| 53373 | SMCR7 | NM_139162.2 | 325 | ggccgtgaagcggttcattgaca | 409360 | - | see also 23628 line |
| 53374 | SMCR7 | NM_139162.2 | 327 | ccgtgaagcggttcattgacagg | 409361 | - | see also 23628 line |
| 53375 | SMCR8 | NM_144775.1 | 896 | caggagctttcagccgaattttc | 411911 | - | see also 23629 line |
| 53376 | SMCR8 | NM_144775.1 | 2362 | ggctagccgggacatcagtaaga | 411935 | - | see also 23629 line |
| 53377 | SMT3H1 | NM_006936.1 | 384 | gcctggcagggcacagtttctag | 336619 | - | see also 23630 line |

Figure 46

| 53378 | SMU-1 | NM_018225.1 | 685 | atgtggagtgtgctcgattttct | 368599 | - | see also 23631 line | | |
|-------|-------|-------------|-----|-------------------------|--------|---|---------------------|---|---|
| 53379 | SMU-1 | NM_018225.1 | 368 | gccagagcgatatattcatctgg | 368595 | - | see also 23631 line | | |
| 53380 | SMU-1 | NM_018225.1 | 687 | gtggagtgtgctcgattttctcc | 368600 | - | see also 23631 line | | |
| 53381 | SMYD2 | NM_020197.1 | 936 | tccggagggccaagcactataaa | 376090 | - | see also 23632 line | | |
| 53382 | SMYD2 | NM_020197.1 | 752 | ctgtacccaacggaagatagaaa | 376082 | - | see also 23632 line | | |
| 53383 | SMYD3 | NM_022743.1 | 576 | accccaactgttcgattgtgttc | 383837 | - | see also 8451 line | | |
| 53384 | SMYD3 | NM_022743.1 | 144 | gccgcgtcgccaaatactgtagt | 383831 | - | see also 8451 line | | |
| 53385 | SNIP1 | NM_024700.2 | 975 | agcagcatgcggtctttcaatat | 389406 | - | see also 23634 line | | |
| 53386 | SNIP1 | NM_024700.2 | 720 | aagaggtgcccgctaaagaaaaa | 389390 | - | see also 23634 line | | |
| 53387 | SOAT | NM_197965.1 | 643 | ctggcgaaaggatcttggaattc | 436932 | - | see also 23635 line | | |
| 53388 | SOAT | NM_197965.1 | 536 | gtgtctatgtgaattacagatgg | 436930 | - | see also 23635 line | | |
| 53389 | SOC | NM_145345.1 | 408 | gagcggttcctcagtgactatgg | 414434 | - | see also 23636 line | | |
| 53390 | SOC | NM_145345.1 | 751 | agcgctgcctccgagacatattg | 414436 | - | see also 23636 line | | |
| 53391 | SOM | NM_021180.2 | 1107 | aggtgttcatcggcgtaaactgt | 380653 | - | see also 23637 line | | |
| 53392 | SOM | NM_021180.2 | 1744 | atgcaagcgaggaatcttagtca | 380662 | - | see also 23637 line | | |
| 53393 | SOSTDC1 | NM_015464.1 | 698 | aacgagtccagtcacaactttga | 353328 | - | see also 23638 line | | |
| 53394 | SOSTDC1 | NM_015464.1 | 214 | tccccttgcatgcatcctaatga | 353318 | - | see also 23638 line | | |
| 53395 | SOSTDC1 | NM_015464.1 | 571 | gtggcggtgtgtcaatgacaaaa | 353325 | - | see also 23638 line | | |
| 53396 | SP192 | NM_021639.3 | 1696 | tccgtggttccaagtgtctataa | 380987 | - | see also 8159 line | | |
| 53397 | SP192 | NM_021639.3 | 1811 | gccgggagtctgcttttaccagt | 380990 | - | see also 8159 line | | |
| 53398 | SPACA1 | NM_030960.2 | 535 | cacacatctcccttaaatcgttt | 395705 | - | see also 23640 line | | |
| 53399 | SPACA1 | NM_030960.2 | 542 | ctcccttaaatcgtttcaaatat | 395706 | - | see also 23640 line | | |
| 53400 | SPAG5 | NM_006461.1 | 2917 | acccgagtagcatcaatggtttc | 335639 | - | see also 23641 line | | |
| 53401 | SPAG5 | NM_006461.1 | 1674 | gtctcggcgtgcagaaacattgg | 335626 | - | see also 23641 line | | |
| 53402 | SPAG5 | NM_006461.1 | 1350 | tgccttgtctcgacatgacttgg | 335617 | - | see also 23641 line | | |
| 53403 | SPANXE | NM_145665.1 | 200 | aacctgctccgaaaaaactaaaa | 400208 | - | - | - | - |
| 53404 | SPANXE | NM_145665.1 | 201 | acctgctccgaaaaaactaaaaa | 400209 | - | see also 53403 line | | |
| 53405 | SPATA1 | NM_022354.1 | 296 | aggatcatggaactataagctaa | 382645 | - | see also 23642 line | | |
| 53406 | SPATA1 | NM_022354.1 | 862 | atgattgctttggcactaaaaaa | 382662 | - | see also 23642 line | | |
| 53407 | SPATA11 | NM_032306.2 | 889 | taggggctcagcccgttatgact | 399612 | - | see also 23643 line | | |
| 53408 | SPATA11 | NM_032306.2 | 891 | ggggctcagcccgttatgacttc | 399613 | - | see also 23643 line | | |
| 53409 | SPATA12 | NM_181727.1 | 1197 | caggtccctctctacagtatact | 433061 | - | see also 23644 line | | |
| 53410 | SPATA12 | NM_181727.1 | 952 | gacatcgctgtatcccaaataaa | 433050 | - | see also 23644 line | | |
| 53411 | SPATA6 | NM_019073.1 | 1053 | ctgccgacccaaggattataagg | 374955 | - | see also 7694 line | | |
| 53412 | SPATA7 | NM_018418.1 | 528 | ccctcaggcgaaccgcaaattga | 371300 | - | see also 23646 line | | |
| 53413 | SPATA7 | NM_018418.1 | 202 | atggtccaccgtgcctatttaaa | 371285 | - | see also 23646 line | | |
| 53414 | SPATA9 | NM_031952.1 | 455 | cgccgaggcaacccctatataac | 397479 | - | see also 23647 line | | |

Figure 46

| 53415 | SPATA9 | NM_031952.1 | 310 | aagattaatcgagcaacattaat | 397475 | - | see also 23647 line |
|---|---|---|---|---|---|---|---|
| 53416 | SPATS1 | NM_145026.2 | 955 | ctccaggcgacaatccatatatg | 412852 | - | see also 23648 line |
| 53417 | SPATS1 | NM_145026.2 | 953 | aactccaggcgacaatccatata | 412851 | - | see also 23648 line |
| 53418 | SPATS2 | NM_023071.1 | 1144 | tgcacggtatcgagttgtagtta | 385058 | - | see also 23649 line |
| 53419 | SPATS2 | NM_023071.1 | 1146 | cacggtatcgagttgtagttaaa | 385059 | - | see also 23649 line |
| 53420 | SPG20 | NM_015087.3 | 1885 | tggcgtaactgcctacaatatta | 348003 | - | see also 6226 line |
| 53421 | SPG20 | NM_015087.3 | 862 | ttctctcgatacggttctaaacc | 347969 | - | see also 6226 line |
| 53422 | SPG6 | NM_144599.2 | 1401 | gagtaccgttcgggtccatttta | 410206 | - | see also 23651 line |
| 53423 | SPG6 | NM_144599.2 | 1492 | ctccgtcgtgctgattatccact | 410211 | - | see also 23651 line |
| 53424 | SPON1 | NM_006108.1 | 439 | aaccagctaccgcgtaacacttt | 334784 | - | see also 23652 line |
| 53425 | SPON1 | NM_006108.1 | 971 | agcgaaggcgtcaaacaagttgc | 334799 | - | see also 23652 line |
| 53426 | SPRED2 | NM_181784.1 | 470 | aaggtcgataataggaagtttgg | 433307 | - | see also 23654 line |
| 53427 | SPRED2 | NM_181784.1 | 462 | atcactggaaggtcgataatagg | 433305 | - | see also 23654 line |
| 53428 | SPRR3 | NM_005416.1 | 501 | ctgaccaaggcttcatcaagttt | 333754 | - | see also 23655 line |
| 53429 | SPRR4 | NM_173080.1 | 147 | gccacccctgttaaatgtcaag | 424600 | - | see also 23656 line |
| 53430 | SPRR4 | NM_173080.1 | 143 | gtcagccacccctgttaaatgt | 424598 | - | see also 23656 line |
| 53431 | SPUF | NM_013349.3 | 435 | tgcccggagaattctcaatgagg | 338812 | - | see also 23657 line |
| 53432 | SPUF | NM_013349.3 | 235 | tccggaaaggagttttatggacg | 338810 | - | see also 23657 line |
| 53433 | SPUF | NM_013349.3 | 432 | cactgcccggagaattctcaatg | 338811 | - | see also 23657 line |
| 53434 | SPY1 | NM_182756.1 | 751 | ttgcaccaacccattatatctgg | 434998 | - | see also 23658 line |
| 53435 | SPY1 | NM_182756.1 | 700 | ttgactatagggctattgtaagc | 434995 | - | see also 23658 line |
| 53436 | SR-A1 | NM_021228.1 | 677 | cccccgattcgatatctatgacc | 380865 | - | see also 23659 line |
| 53437 | SR-A1 | NM_021228.1 | 2587 | tgggctccattggcgtcaaattc | 380870 | - | see also 23659 line |
| 53438 | SRRM2 | NM_016333.2 | 6410 | ctcggggtaaacggtccttaaca | 357193 | - | see also 6815 line |
| 53439 | SRRM2 | NM_016333.2 | 498 | ggcgacctttcgactcatgttgc | 357081 | - | see also 6815 line |
| 53440 | SS18L2 | NM_016305.1 | 183 | gaccagctgatccgctgtattgt | 356934 | - | see also 23661 line |
| 53441 | SS18L2 | NM_016305.1 | 194 | ccgctgtattgtggagtatcaga | 356935 | - | see also 23661 line |
| 53442 | SSTK-IP | NM_144627.1 | 449 | ctcccccagctatattaatcttc | 410515 | - | see also 23662 line |
| 53443 | SSTK-IP | NM_144627.1 | 450 | tcccccagctatattaatcttca | 410516 | - | see also 23662 line |
| 53444 | SSTR4 | NM_001052.1 | 750 | caggcgctcggagaagaaaatca | 330139 | - | see also 23663 line |
| 53445 | SSTR4 | NM_001052.1 | 371 | gcgtcgacggcctcaacatgttc | 330138 | - | see also 23663 line |
| 53446 | SSX2IP | NM_014021.1 | 1483 | cagctcgctactgcatatgatga | 339254 | - | see also 23664 line |
| 53447 | SSX2IP | NM_014021.1 | 1515 | ttcactattacgagactgttatt | 339257 | - | see also 23664 line |
| 53448 | ST5 | NM_005418.2 | 3058 | gacgacgaagacacgttgttacc | 333779 | - | see also 3786 line |
| 53449 | ST5 | NM_005418.2 | 3154 | gactccgacgatgaatgtaatac | 333783 | - | see also 3786 line |
| 53450 | ST5 | NM_005418.2 | 3151 | agcgactccgacgatgaatgtaa | 333782 | - | see also 3786 line |
| 53451 | ST7 | NM_018412.2 | 366 | cacattaacaccgaagttctacg | 371243 | - | see also 7450 line |

Figure 46

| | | | | | | | |
|---|---|---|---|---|---|---|---|
| 53452 | ST7 | NM_018412.2 | 1727 | cacatcagttcccggaacttatg | 371282 | - | see also 7450 line |
| 53453 | ST7 | NM_018412.2 | 452 | ttcgcaaatacggaacttcattc | 371246 | - | see also 7450 line |
| 53454 | ST7L | NM_017744.4 | 1596 | ctgaaacggggtgatagtgaagc | 361472 | - | see also 7131 line |
| 53455 | ST7L | NM_017744.4 | 974 | agcagcctatcaagctttagaat | 361459 | - | see also 7131 line |
| 53456 | STAF42 | NM_053053.2 | 206 | ttctcacgcgttgtcagatttg | 405912 | - | see also 9486 line |
| 53457 | STAF42 | NM_053053.2 | 268 | gggggttccgcagcaaaacctgg | 405913 | - | see also 9486 line |
| 53458 | STAG2 | NM_006603.3 | 2420 | gtgtactcactatgtaatccttt | 335902 | - | see also 4654 line |
| 53459 | STAG2 | NM_006603.3 | 3067 | ttgctcgactttgctttaact | 335917 | - | see also 4654 line |
| 53460 | STARD10 | NM_006645.1 | 812 | acgctgacgtgggctattactcc | 336029 | - | see also 4698 line |
| 53461 | STARD10 | NM_006645.1 | 762 | atgggacacaacgtcattgaga | 336026 | - | see also 4698 line |
| 53462 | STARD7 | NM_020151.2 | 1284 | ttcctcgctactgttagttgg | 375718 | - | see also 7769 line |
| 53463 | STARD7 | NM_020151.2 | 1058 | atgtactcacggattatgttta | 375711 | - | see also 7769 line |
| 53464 | STARS | NM_139166.2 | 389 | aacggtggtcagcaagacttacg | 409366 | - | see also 23671 line |
| 53465 | STARS | NM_139166.2 | 96 | aagagcgccctcggaagatacg | 409362 | - | see also 23671 line |
| 53466 | STATIP1 | NM_018255.1 | 840 | cagtgctagccggtcatgaaaac | 369177 | - | see also 23672 line |
| 53467 | STATIP1 | NM_018255.1 | 343 | tgcggtgcatgctgtttaccaga | 369161 | - | see also 23672 line |
| 53468 | STEPP | NM_152990.2 | 96 | tggcagagatgcactagatcatt | 421692 | - | see also 23673 line |
| 53469 | STEPP | NM_152990.2 | 97 | ggcagagatgcactagatcattt | 421693 | - | see also 23673 line |
| 53470 | ST2 | NM_145755.1 | 3785 | aagttcgacctcgcttagaact | 414918 | - | see also 23674 line |
| 53471 | ST2 | NM_145755.1 | 2860 | ggcggtacagtcttataaggatg | 414908 | - | see also 23674 line |
| 53472 | STK11IP | NM_052902.1 | 275 | ggcgactccctgttattcttca | 405141 | - | see also 23675 line |
| 53473 | STK11IP | NM_052902.1 | 271 | tcctgccgactccctgttattc | 405140 | - | see also 23675 line |
| 53474 | STK22B | NM_053006.3 | 183 | gggttcctacgcaaaagtcaaat | 405769 | - | see also 23676 line |
| 53475 | STK22B | NM_053006.3 | 157 | tacatcgtaggcatcaatcttgg | 405767 | - | see also 23676 line |
| 53476 | STK22B | NM_053006.3 | 366 | ctctgacggacggatctacatca | 405773 | - | see also 23676 line |
| 53477 | STRAIT114 99 | NM_021242.3 | 884 | accagaagcaactcgctctttaac | 380879 | | see also 8143 line |
| 53478 | STRC | NM_153700.2 | 3200 | tccttcctaggcacgatctatcc | 423672 | - | see also 10008 line |
| 53479 | STX1B2 | NM_052874.1 | 681 | tccgcgagctgcacgatatgttt | 404572 | - | see also 23679 line |
| 53480 | STX1B2 | NM_052874.1 | 438 | tggaggtaatgaccggaatataac | 404571 | - | see also 23679 line |
| 53481 | STXBP4 | NM_178509.3 | 321 | gccaagttgagtactaggttaga | 430949 | - | see also 23680 line |
| 53482 | STXBP4 | NM_178509.3 | 316 | ccggagccaagttgagtactagg | 430948 | - | see also 23680 line |
| 53483 | STXBP5 | NM_139244.2 | 2897 | gtgtctccaagtggtactatatt | 409554 | - | see also 23681 line |
| 53484 | STXBP5 | NM_139244.2 | 2516 | ccccgatcctctgtaaatctcg | 409542 | - | see also 23681 line |
| 53485 | STXBP6 | NM_014178.5 | 455 | ctggagtcaaggcgaatattta | 339889 | - | see also 23682 line |
| 53486 | STXBP6 | NM_014178.5 | 464 | aaggcgaatatttaacttatatc | 339892 | - | see also 23682 line |
| 53487 | SUHW1 | NM_080740.1 | 1515 | ggcttccgttattgttagcaaca | 406724 | - | see also 23683 line |

Figure 46

| | | | | | | | |
|---|---|---|---|---|---|---|---|
| 53488 | SUHW1 | NM_080740.1 | 813 | tcccatcgtgttacttagcaact | 406716 | - | see also 23683 line |
| 53489 | SULT1C2 | NM_006588.2 | 610 | ggcaccgactcatcaacgattc | 335823 | - | see also 23684 line |
| 53490 | SULT1C2 | NM_006588.2 | 944 | gacaaacaccgtattctctatct | 335830 | - | see also 23684 line |
| 53491 | SULT1C2 | NM_006588.2 | 431 | aactacgtgaagggaattcttca | 335820 | - | see also 23684 line |
| 53492 | SUMF1 | NM_182760.2 | 727 | ctgtcgaggaggcctgcataata | 435126 | - | see also 23685 line |
| 53493 | SUMF1 | NM_182760.2 | 366 | caactgcgagagttactatt | 435120 | - | see also 23685 line |
| 53494 | SVH | NM_031905.2 | 623 | gacctcgcagcctgaagacttaa | 397244 | - | see also 23686 line |
| 53495 | SYAP1 | NM_032796.2 | 555 | tccggctggcgtgcaatttaatt | 401528 | - | see also 23687 line |
| 53496 | SYAP1 | NM_032796.2 | 554 | ctccggctggccgtgcaatttaat | 401527 | - | see also 23687 line |
| 53497 | SYCP3 | NM_153694.2 | 122 | gccgtcttggaagatcagttta | 423550 | - | see also 23688 line |
| 53498 | SYCP3 | NM_153694.2 | 220 | ctgcagtcattgagaaacgtagg | 423555 | - | see also 23688 line |
| 53499 | SYN1 | NM_006950.2 | 1265 | gacggaaggggatcaccatcattga | 336636 | - | see also 4865 line |
| 53500 | SYN1 | NM_006950.2 | 149 | cgcctgcggacagcaacttat | 336620 | - | see also 4865 line |
| 53501 | SYN1 | NM_006950.2 | 495 | cgcacacgactgggcaaaatac | 336622 | - | see also 4865 line |
| 53502 | SYNCOILIN | NM_030786.1 | 361 | atcgcacttgtgaggcaaaaacg | 394994 | | see also 23690 line |
| 53503 | SYNCOILIN | NM_030786.1 | 321 | ccggcagctcgcctgcaaaaca | 394993 | - | see also 23690 line |
| 53504 | SYNE1 | NM_015293.1 | 9421 | gtccctattgattctaaccttga | 351301 | - | see also 23691 line |
| 53505 | SYNE1 | NM_015293.1 | 7705 | cttgatgactattcacgttttga | 351282 | - | see also 23691 line |
| 53506 | SYNE2 | NM_015180.3 | 20267 | ggctcatgtcacgatccaaagg | 349442 | - | see also 6270 line |
| 53507 | SYNE2 | NM_015180.3 | 19764 | ggggactggccggtatcacaga | 349434 | - | see also 6270 line |
| 53508 | SYNPO | NM_007286.3 | 2771 | ggcgcttctgtgcggaaacatca | 337407 | - | see also 23693 line |
| 53509 | SYNPO | NM_007286.3 | 580 | taccactgtgtttatctaaag | 337388 | - | see also 23693 line |
| 53510 | SYNPO2L | NM_024875.1 | 1771 | ccgcctccattgcttacaacc | 391619 | - | see also 8800 line |
| 53511 | SYNPO2L | NM_024875.1 | 1875 | agggcatcaaggctctagattt | 391621 | - | see also 8800 line |
| 53512 | SYNPO2L | NM_024875.1 | 378 | cccgcagctcaccaatcaatct | 391612 | - | see also 8800 line |
| 53513 | SYT10 | NM_198992.1 | 1647 | ttgcggtcatggattacgatagg | 440378 | - | see also 10352 line |
| 53514 | SYT10 | NM_198992.1 | 1661 | tacgatagggtaggacacaatga | 440379 | - | see also 10352 line |
| 53515 | SYT5 | NM_003180.1 | 1417 | cccctattacaacggaagcttca | 331244 | - | see also 23696 line |
| 53516 | SYT5 | NM_003180.1 | 1408 | caactgaaccctattacaacg | 331243 | - | see also 23696 line |
| 53517 | SYTL2 | NM_032379.2 | 1752 | tccccaatacttgattgaagc | 400096 | - | see also 9177 line |
| 53518 | SYTL2 | NM_032379.2 | 1181 | tacatttaagcgcaatagtttcc | 400083 | - | see also 9177 line |
| 53519 | T1 | NM_031466.3 | 2266 | gactggtgaacgattactgtga | 397125 | - | see also 23698 line |
| 53520 | T1 | NM_031466.3 | 2262 | cgcagacgactggaacgattact | 397124 | - | see also 23698 line |
| 53521 | T2BP | NM_052864.2 | 472 | cagtttccgagttcagtttc | 404500 | - | see also 23699 line |
| 53522 | T2BP | NM_052864.2 | 668 | aagatgcgagtcattggaatt | 404507 | - | see also 23699 line |

Figure 46

| | | | | | | | |
|---|---|---|---|---|---|---|---|
| 53523 | T3JAM | NM_025228.1 | 388 | gcgcgtgccgtccaatgtgacc | 394464 | - | see also 8885 line |
| 53524 | T3JAM | NM_025228.1 | 1314 | caggccctgcgattttggaaaa | 394474 | - | see also 8885 line |
| 53525 | TA-LRRP | NM_015350.1 | 882 | tgccgtcgttacgagaaacagc | 351765 | - | see also 23701 line |
| 53526 | TA-LRRP | NM_015350.1 | 2932 | ctccctgaacagaacgtttaca | 351816 | - | see also 23701 line |
| 53527 | TA-LRRP | NM_015350.1 | 2213 | cccggatcccacagttgttaca | 351797 | - | see also 23701 line |
| 53528 | TA-NFKBH | NM_032721.2 | 953 | ggccatcctggcccttaacgttg | 401255 | - | see also 23702 line |
| 53529 | TA-NFKBH | NM_032721.2 | 52 | gccaagccttctggcatggaaacg | 401251 | - | see also 23702 line |
| 53530 | TAB3 | NM_152787.2 | 1103 | cacatacctggtatagtacaaa | 421240 | - | see also 23703 line |
| 53531 | TAB3 | NM_152787.2 | 1101 | tgcacatacctggtatagtaca | 421239 | - | see also 23703 line |
| 53532 | TAF1L | NM_153809.1 | 867 | gtgttacgttcctacatctttt | 423897 | - | see also 23704 line |
| 53533 | TAF1L | NM_153809.1 | 4441 | tccgtgaaaatgtgcgtaaatgc | 423899 | - | see also 23704 line |
| 53534 | TAF7L | NM_024885.1 | 664 | tggcattacgccaccacttaaga | 391703 | - | see also 23705 line |
| 53535 | TAF7L | NM_024885.1 | 594 | ctgcctcactgatcctaatata | 391700 | - | see also 23705 line |
| 53536 | TAFA2 | NM_178539.2 | 1260 | ttcttccgatcggaaaggatgg | 431154 | - | see also 23706 line |
| 53537 | TAFA2 | NM_178539.2 | 1183 | atgtgtggatgcttcaatagtgg | 431153 | - | see also 23706 line |
| 53538 | TAGAP | NM_054114.3 | 351 | cacaatgcttcaaaaacactaaa | 406064 | - | see also 23707 line |
| 53539 | TAPBP-R | NM_018009.3 | 991 | caggacgaggggacctacatttg | 365243 | - | see also 23708 line |
| 53540 | TAPBP-R | NM_018009.3 | 391 | gaggacttcaccgatttccaagg | 365240 | - | see also 23708 line |
| 53541 | TARSH | NM_015429.2 | 1109 | cactacgtctagtgcattagatg | 352673 | - | see also 6413 line |
| 53542 | TARSH | NM_015429.2 | 1107 | cccactacgtctagtgcattaga | 352672 | - | see also 6413 line |
| 53543 | TARSH | NM_015429.2 | 1583 | accggctccggtaaaaacacaat | 352690 | - | see also 6413 line |
| 53544 | TBC1D4 | NM_014832.1 | 3786 | aacacgctacctgatatgaatac | 344373 | - | see also 6041 line |
| 53545 | TBC1D4 | NM_014832.1 | 1714 | gcccgatgcactctttgcataag | 344330 | - | see also 6041 line |
| 53546 | TBC1D5 | NM_014744.1 | 2849 | cccattcatcggggatgtacact | 342954 | - | see also 5940 line |
| 53547 | TBC1D5 | NM_014744.1 | 3614 | aggttcctgcgtttaaccaga | 342973 | - | see also 5940 line |
| 53548 | TBC1D7 | NM_016495.2 | 803 | ttgtagctgcgaaattttatta | 357882 | - | see also 23712 line |
| 53549 | TBC1D7 | NM_016495.2 | 367 | ctgaaagtcgttcgctttgttag | 357867 | - | see also 23712 line |
| 53550 | TBL1Y | NM_033284.1 | 1820 | tgcatgcgtccacgatcttcagg | 403664 | - | see also 23713 line |
| 53551 | TBL1Y | NM_033284.1 | 1333 | ggcacgacgtcccaagtaataaa | 403659 | - | see also 23713 line |
| 53552 | TBN | NM_138572.1 | 281 | gcccacactgtccgatatcgtgg | 408487 | - | see also 23714 line |
| 53553 | TBN | NM_138572.1 | 90 | aaccctgccgataactatcatct | 408485 | - | see also 23714 line |
| 53554 | TCBA1 | NM_153355.2 | 717 | tacaactacagcctatgtacatg | 423031 | - | see also 23715 line |
| 53555 | TCBA1 | NM_153355.2 | 335 | tgggttacgtggaatgtgtttgt | 423025 | - | see also 23715 line |
| 53556 | TCEB3L | NM_016427.1 | 1858 | gacgaattacggaggaatcattg | 357442 | - | see also 23716 line |
| 53557 | TCEB3L | NM_016427.1 | 2112 | ccccgaaaatggggagatcaagc | 357446 | - | see also 23716 line |

Figure 46

| 53558 | TCERG1L | NM_174937.1 | 1138 | gacgggcgatgaccgagttttct | 428744 | - | see also 23717 line | | |
|--------|---------|-------------|------|-------------------------|--------|---|----------------------|---|---|
| 53559 | TCERG1L | NM_174937.1 | 1140 | cgggcgatgaccgagttttcttc | 428745 | - | see also 23717 line | | |
| 53560 | TCERG1L | NM_174937.1 | 1141 | gggcgatgaccgagttttcttct | 428746 | - | see also 23717 line | | |
| 53561 | TCF7L2 | NM_030756.1 | 1218 | atgtcggctcactccatagttca | 394883 | - | see also 8933 line | | |
| 53562 | TCF7L2 | NM_030756.1 | 946 | tccagatatatcccccgtattacc | 394878 | - | see also 8933 line | | |
| 53563 | TCTA | NM_022171.1 | 499 | cagcaaacgaacctctcaaaacc | 382591 | - | - | - | small cell lung cancer、leukemia |
| 53564 | TCTE3 | NM_174910.1 | 604 | gacagctgggtcgcagctaaaca | 428645 | - | see also 23718 line | | |
| 53565 | TCTE3 | NM_174910.1 | 508 | taccaccgttataagttcattat | 428641 | - | see also 23718 line | | |
| 53566 | TD-60 | NM_018715.1 | 506 | accaaggagcgcgtcaaacttga | 372813 | - | see also 23719 line | | |
| 53567 | TD-60 | NM_018715.1 | 621 | accgcaatctcggtcagaatttg | 372815 | - | see also 23719 line | | |
| 53568 | TD-60 | NM_018715.1 | 505 | caccaaggagcgcgtcaaacttg | 372812 | - | see also 23719 line | | |
| 53569 | TEAD1 | NM_021961.2 | 1492 | gccgatttgtataccgaataaac | 381662 | - | see also 8217 line | | |
| 53570 | TEAD1 | NM_021961.2 | 561 | cccgacatcgagcaaagctttca | 381643 | - | see also 8217 line | | |
| 53571 | TEB4 | NM_005885.2 | 2044 | cagccttaccgccgacctttaaa | 334405 | - | see also 4093 line | | |
| 53572 | TEB4 | NM_005885.2 | 2046 | gccttaccgccgacctttaaatt | 334407 | - | see also 4093 line | | |
| 53573 | TECTB | NM_058222.1 | 122 | tccccgagtgtccctatggatgg | 406219 | - | see also 9502 line | | |
| 53574 | TECTB | NM_058222.1 | 688 | accgtcctcgtgcatgagaatgg | 406235 | - | see also 9502 line | | |
| 53575 | TES | NM_015641.2 | 1296 | tgcagcgggtgacctataacaat | 354845 | - | see also 23723 line | | |
| 53576 | TES | NM_015641.2 | 1299 | agcgggtgacctataacaatttc | 354847 | - | see also 23723 line | | |
| 53577 | TESSP1 | NM_183379.1 | 662 | agccctctagccgtagtatgatc | 435829 | - | see also 23724 line | | |
| 53578 | TESSP1 | NM_183379.1 | 422 | ttggggttttacgcaatgacatt | 435826 | - | see also 23724 line | | |
| 53579 | TESSP1 | NM_183379.1 | 663 | gccctctagccgtagtatgatct | 435830 | - | see also 23724 line | | |
| 53580 | TESSP2 | NM_182702.1 | 173 | ccccactcaatctgtttacgtcc | 434880 | - | see also 23725 line | | |
| 53581 | TESSP2 | NM_182702.1 | 396 | gggagatcggagtgtctataatg | 434884 | - | see also 23725 line | | |
| 53582 | TEX11 | NM_031276.1 | 748 | tacggctattagccacgaattat | 396306 | - | see also 23726 line | | |
| 53583 | TEX11 | NM_031276.1 | 400 | tggagcaattatacgtcaaatta | 396296 | - | see also 23726 line | | |
| 53584 | TEX12 | NM_031275.2 | 363 | ttcctgcgacagaggtttacagt | 396275 | - | see also 9000 line | | |
| 53585 | TEX13A | NM_031274.2 | 920 | gtcgaaacagccacatcttattt | 396265 | - | see also 23727 line | | |
| 53586 | TEX13A | NM_031274.2 | 921 | tcgaaacagccacatcttatttc | 396266 | - | see also 23727 line | | |
| 53587 | TEX13B | NM_031273.1 | 188 | ccgagttctacttcgagaatata | 396255 | - | see also 23728 line | | |
| 53588 | TEX13B | NM_031273.1 | 816 | aagatctcaggtcagtacaaaca | 396261 | - | see also 23728 line | | |
| 53589 | TEX15 | NM_031271.2 | 5614 | aacgaggcagtacgtgttaaaat | 396176 | - | see also 8996 line | | |
| 53590 | TEX15 | NM_031271.2 | 4826 | atcatacggcccttatagctaat | 396148 | - | see also 8996 line | | |
| 53591 | TEX15 | NM_031271.2 | 3782 | aggcgaaaactgacgtacttata | 396115 | - | see also 8996 line | | |
| 53592 | TEX261 | NM_144582.1 | 17 | ttctccaccgctgtactgattgg | 409961 | - | see also 23730 line | | |
| 53593 | TEX261 | NM_144582.1 | 138 | tgctgacctcgcctaacttcatc | 409963 | - | see also 23730 line | | |
| 53594 | TEX261 | NM_144582.1 | 279 | ttgcgttttttgtgtcactttcg | 409970 | - | see also 23730 line | | |

Figure 46

| 53595 | TFB2M | NM_022366.1 | 211 | agcggcgacgcgaaagcatttgc | 382669 | - | see also 23731 line |
|---|---|---|---|---|---|---|---|
| 53596 | TFB2M | NM_022366.1 | 330 | tagactttaagcgttacgtaacc | 382671 | - | see also 23731 line |
| 53597 | TFPT | NM_013342.1 | 981 | ttctcggggtccagacaaactgc | 338778 | - | see also 23732 line |
| 53598 | TFPT | NM_013342.1 | 735 | cccaggcaatgcggagaatgagc | 338777 | - | see also 23732 line |
| 53599 | TGM6 | NM_198994.1 | 1287 | gccgggagcgtgtatactcaaac | 440397 | - | see also 23733 line |
| 53600 | TGM6 | NM_198994.1 | 1286 | agccgggagcgtgtatactcaaa | 440396 | - | see also 23733 line |
| 53601 | TGM6 | NM_198994.1 | 936 | cacgggtcgtgtccaacttcaac | 440393 | - | see also 23733 line |
| 53602 | TGS | NM_173083.2 | 728 | tacgagtggttctattcaaatat | 424726 | - | see also 10061 line |
| 53603 | TGS | NM_173083.2 | 673 | tggttttcgattacgtaatctgc | 424723 | - | see also 10061 line |
| 53604 | TH1L | NM_016397.2 | 547 | ttcaccgttaagcttatttctga | 357341 | - | see also 6847 line |
| 53605 | TH1L | NM_016397.2 | 1608 | ttcactcattcgctattttgtca | 357364 | - | see also 6847 line |
| 53606 | THAP10 | NM_020147.2 | 603 | aaccccatgctgataatccatct | 375696 | - | see also 23736 line |
| 53607 | THAP10 | NM_020147.2 | 707 | caccgtagtgtgggtattcaagc | 375701 | - | see also 23736 line |
| 53608 | THAP4 | NM_015963.3 | 720 | atctggggcgtgcaaatttatcg | 355643 | - | see also 23737 line |
| 53609 | THAP4 | NM_015963.3 | 716 | caggatctggggcgtgcaaattt | 355641 | - | see also 23737 line |
| 53610 | THAP9 | NM_024672.2 | 414 | gaggaccataactatagtttaaa | 388842 | - | see also 23738 line |
| 53611 | THAP9 | NM_024672.2 | 1374 | cagcacctcgtggagttagtagc | 388877 | - | see also 23738 line |
| 53612 | THOC3 | NM_032361.1 | 912 | ggcgtcagcatcggaagatcatt | 399990 | - | see also 9174 line |
| 53613 | THOC3 | NM_032361.1 | 859 | aggctggattggcctgtaagaac | 399989 | - | see also 9174 line |
| 53614 | THSD1 | NM_018676.2 | 904 | ggcgagactcagtcattacctcc | 372506 | - | see also 23740 line |
| 53615 | THSD1 | NM_018676.2 | 1940 | cagcgctcccttagatttggaaa | 372520 | - | see also 23740 line |
| 53616 | THSD2 | NM_032784.2 | 801 | aacacgggtccgagaaataatac | 401500 | - | see also 9282 line |
| 53617 | THSD2 | NM_032784.2 | 804 | acgggtccgagaaataatacagc | 401501 | - | see also 9282 line |
| 53618 | THY28 | NM_014174.2 | 974 | cagtttgttcggatgatgaaacg | 339856 | - | see also 5524 line |
| 53619 | THY28 | NM_014174.2 | 563 | aactcaggtgaggcattagctaa | 339847 | - | see also 5524 line |
| 53620 | TIC | NM_012455.1 | 1501 | ttgcccaagtggacactagatgc | 338225 | - | see also 23743 line |
| 53621 | TIC | NM_012455.1 | 1152 | accgtgtgtggacgaagcattga | 338224 | - | see also 23743 line |
| 53622 | TIGA1 | NM_053000.1 | 558 | aacggggagcttcgtcgatttgt | 405591 | - | see also 23744 line |
| 53623 | TIGA1 | NM_053000.1 | 539 | ctgcgttcccacgtttggaaacg | 405590 | - | see also 23744 line |
| 53624 | TIGD2 | NM_145715.1 | 252 | tccgtggtgtacggaattggtga | 414718 | - | see also 23745 line |
| 53625 | TIGD2 | NM_145715.1 | 442 | ttccagtgtccggaacgatttgt | 414727 | - | see also 23745 line |
| 53626 | TIGD3 | NM_145719.1 | 288 | atctcgcgcatctgcaagaataa | 414760 | - | see also 23746 line |
| 53627 | TIGD3 | NM_145719.1 | 1502 | cacttctcctgagctattcgaaa | 414772 | - | see also 23746 line |
| 53628 | TIP120A | NM_018448.2 | 3789 | agccattacgtgcaacatgtaca | 371693 | - | see also 23747 line |
| 53629 | TIP120A | NM_018448.2 | 1853 | tacgtaatcctctgtaaccattc | 371625 | - | see also 23747 line |
| 53630 | TJP1 | NM_003257.2 | 2608 | tgcagttgatcgtcttaactatg | 331372 | - | see also 2201 line |
| 53631 | TJP1 | NM_003257.2 | 5379 | cagcccgaggcatatttaacagc | 331450 | - | see also 2201 line |

Figure 46

| 53632 | TJP3 | NM_014428.1 | 268 | tccgcgtttgccattcagatact | 340648 | - | see also 23749 line |
|---|---|---|---|---|---|---|---|
| 53633 | TJP3 | NM_014428.1 | 267 | ctccgcgtttgccattcagatac | 340647 | - | see also 23749 line |
| 53634 | TJP4 | NM_080604.1 | 431 | atcgggagctgcgtttggaaatt | 406396 | - | see also 23750 line |
| 53635 | TJP4 | NM_080604.1 | 641 | tggacaaatttaaggataagttc | 406398 | - | see also 23750 line |
| 53636 | TLK2 | NM_006852.1 | 498 | cccttaccgcgacgagtagaaca | 336476 | - | see also 4796 line |
| 53637 | TLX3 | NM_021025.1 | 731 | aacacgacgccttccaaaagagc | 380366 | - | see also 8069 line |
| 53638 | TM4SF10 | NM_031442.2 | 597 | gacctgttgcggtcatgcttttt | 397014 | - | see also 9030 line |
| 53639 | TMC3 | NM_181841.1 | 2893 | cccaggaacgtgcgacaatatgc | 433792 | - | see also 23753 line |
| 53640 | TMC3 | NM_181841.1 | 2661 | aacgcacatcgaagatgtacact | 433785 | - | see also 23753 line |
| 53641 | TMC4 | NM_144686.1 | 960 | agcgcatcatcttgtacgaatta | 411311 | - | see also 23754 line |
| 53642 | TMC4 | NM_144686.1 | 1284 | ctcggagtcgccagatcgttttt | 411313 | - | see also 23754 line |
| 53643 | TMC4 | NM_144686.1 | 50 | aacccgaccttggaatcagaagc | 411307 | - | see also 23754 line |
| 53644 | TMC5 | NM_024780.3 | 2491 | ttgacttgcgatctagaagatca | 390627 | - | see also 8744 line |
| 53645 | TMC5 | NM_024780.3 | 2488 | gtcttgacttgcgatctagaaga | 390626 | - | see also 8744 line |
| 53646 | TMC7 | NM_024847.2 | 2170 | gtcgtgacaagtgctacctaatc | 391413 | - | see also 8783 line |
| 53647 | TMC7 | NM_024847.2 | 1344 | atccgctatgaggattattctcc | 391395 | - | see also 8783 line |
| 53648 | TMEM13 | NM_181744.1 | 202 | gacccgctgaaataatgactatc | 433068 | - | see also 10251 line |
| 53649 | TMEM13 | NM_181744.1 | 387 | agcctggatcgatatttgaaaat | 433075 | - | see also 10251 line |
| 53650 | TMEM16C | NM_031418.1 | 3263 | cccgagcaactgacataggtatc | 396844 | - | see also 23758 line |
| 53651 | TMEM16C | NM_031418.1 | 1457 | gccccgatatcatgtttattaaa | 396779 | - | see also 23758 line |
| 53652 | TMEM16D | NM_178826.2 | 1596 | gacggcgagcagtaattgcttat | 431814 | - | see also 10214 line |
| 53653 | TMEM16D | NM_178826.2 | 2646 | ctcgcttggtgtatgcttataag | 431859 | - | see also 10214 line |
| 53654 | TMIE | NM_147196.1 | 555 | gacacagtggccatcaaagtaga | 415097 | - | - | | - |
| 53655 | TMIE | NM_147196.1 | 325 | ggcacgtggtgggcatctttttcg | 415094 | - | see also 53654 line |
| 53656 | TMIE | NM_147196.1 | 557 | cacagtggccatcaaagtagagg | 415098 | - | see also 53654 line |
| 53657 | TMPIT | NM_031925.1 | 700 | acggtctcatgtaccagaaattc | 397389 | - | see also 23760 line |
| 53658 | TMPIT | NM_031925.1 | 426 | ctgagcaagcaggctaagtttgc | 397387 | - | see also 23760 line |
| 53659 | TNIP2 | NM_024309.2 | 1134 | cgccgacgcattagagcttatgg | 386224 | - | see also 23761 line |
| 53660 | TNIP2 | NM_024309.2 | 992 | atcgggaacgggctcaaagtagg | 386220 | - | see also 23761 line |
| 53661 | TNIP3 | NM_024873.2 | 849 | atcggatcgagagagacttaatc | 391602 | - | see also 23762 line |
| 53662 | TNIP3 | NM_024873.2 | 220 | cagggcacatctagaatgattgc | 391590 | - | see also 23762 line |
| 53663 | TNRC15 | NM_015575.1 | 4002 | atcatcggagcgactcaacatgg | 354185 | - | see also 6488 line |
| 53664 | TNRC15 | NM_015575.1 | 1190 | aagaacccgataagacaaataag | 354128 | - | see also 6488 line |

Figure 46

| | | | | | | | |
|---|---|---|---|---|---|---|---|
| 53665 | TNRC5 | NM_006586.2 | 512 | gggttcgcctgcccagcaaatgc | 335811 | - | see also 23763 line |
| 53666 | TNRC5 | NM_006586.2 | 531 | atgcgaagtgtgtaaatatgttg | 335812 | - | see also 23763 line |
| 53667 | TNT | NM_182831.1 | 205 | ttcaaagggcagccttatatacg | 435370 | - | see also 23764 line |
| 53668 | TNT | NM_182831.1 | 438 | ctcaggagcccctgaaagtgtcc | 435372 | - | see also 23764 line |
| 53669 | TOR2A | NM_130459.1 | 297 | accggcaccggcaaatcctatgt | 406913 | - | see also 23765 line |
| 53670 | TOR2A | NM_130459.1 | 296 | gaccggcaccggcaaatcctatg | 406912 | - | see also 23765 line |
| 53671 | TOR3A | NM_022371.2 | 864 | ctcaggggcgatataatcaatga | 382741 | - | see also 23766 line |
| 53672 | TOR3A | NM_022371.2 | 917 | gtcccgggaagaaattacgatgg | 382744 | - | see also 23766 line |
| 53673 | TP53INP1 | NM_033285.2 | 979 | gccttcgtcgccaaaatcttacc | 403686 | - | see also 9396 line |
| 53674 | TP53TG3 | NM_016212.1 | 377 | gccgcagggttgtcaagtttttc | 356574 | - | see also 23768 line |
| 53675 | TP53TG3 | NM_016212.1 | 127 | aactcccggaacggttgaagacg | 351857 | - | see also 23768 line |
| 53676 | TP53TG3 | NM_015369.1 | 256 | ggctagtttcacctggtaattgt | 351859 | - | - |
| 53677 | TRAG3 | NM_004909.1 | 226 | taccacaagactaacataaaaat | 333244 | - | see also 23769 line |
| 53678 | TRALPUSH | NM_053002.2 | 2700 | aggcgctatcacagttgtctaat | 405679 | - | see also 9475 line |
| 53679 | TRALPUSH | NM_053002.2 | 3227 | gtgggggtttaatgatgtacttt | 405695 | - | see also 9475 line |
| 53680 | TRAP25 | NM_080651.1 | 417 | gtcgagcaacttattccatatgt | 406517 | - | see also 23771 line |
| 53681 | TRAP25 | NM_080651.1 | 472 | ctggcccacctcgttttgctagt | 406519 | - | see also 23771 line |
| 53682 | TRAPPC6B | NM_177452.2 | 221 | tggcggatgaggcgttgtttttg | 429866 | - | see also 23772 line |
| 53683 | TRAPPC6B | NM_177452.2 | 513 | cagaggtggcttatcaaacttgg | 429880 | - | see also 23772 line |
| 53684 | TREM4 | NM_145273.2 | 380 | ccccgatgagtctttactgatct | 414078 | - | see also 23773 line |
| 53685 | TREM4 | NM_145273.2 | 379 | gccccgatgagtctttactgatc | 414077 | - | see also 23773 line |
| 53686 | TREM4 | NM_145273.2 | 892 | cacagaggaacgagaagttctgc | 414079 | - | see also 23773 line |
| 53687 | TREML1 | NM_178174.2 | 301 | tgctgcaggtggaaatggttacc | 430606 | - | see also 23774 line |
| 53688 | TREML1 | NM_178174.2 | 877 | ctgtgacatatgccacagtaatc | 430610 | - | see also 23774 line |
| 53689 | TRF3 | NM_199047.1 | 1001 | aaccacgaattgtgttgcttatc | 440628 | - | see also 23775 line |
| 53690 | TRF3 | NM_199047.1 | 223 | tccaatccggatactgcatttaa | 440614 | - | see also 23775 line |
| 53691 | TRF3 | NM_199047.1 | 1002 | accacgaattgtgttgcttatct | 440629 | - | see also 23775 line |
| 53692 | TRIF | NM_014261.1 | 577 | accagacgccactccaactttct | 339991 | - | see also 23776 line |
| 53693 | TRIF | NM_014261.1 | 1528 | cagggctgcaacccctcattatc | 339997 | - | see also 23776 line |
| 53694 | TRIK | NM_181840.1 | 463 | ctggcaaccatcttatctacatc | 433726 | - | see also 23777 line |
| 53695 | TRIK | NM_181840.1 | 464 | tggcaaccatcttatctacatct | 433727 | - | see also 23777 line |
| 53696 | TRIP | NM_005879.2 | 964 | gactgtcgaccgcctggttttag | 334333 | - | see also 23778 line |
| 53697 | TRIP | NM_005879.2 | 1366 | cggtggccggacaaaattcatcc | 334344 | - | see also 23778 line |

Figure 46

| 53698 | TRIP | NM_005879.2 | 1362 | ggctcggtggccggacaaaattc | 334342 | - | see also 23778 line |
|---|---|---|---|---|---|---|---|
| 53699 | TRIPIN | NM_152524.1 | 382 | ttcttcgcctaaagctaaataac | 417566 | - | see also 23779 line |
| 53700 | TRIPIN | NM_152524.1 | 1779 | aacctagacgtcacaaatgaatt | 417603 | - | see also 23779 line |
| 53701 | TSARG1 | NM_139073.2 | 164 | cccagcatgctagctccaattcc | 409326 | - | see also 23780 line |
| 53702 | TSARG2 | NM_144644.2 | 859 | ctctgggcgcagatataatttaa | 410705 | - | see also 23781 line |
| 53703 | TSARG2 | NM_144644.2 | 861 | ctgggcgcagatataatttaaaa | 410706 | - | see also 23781 line |
| 53704 | TSARG2 | NM_144644.2 | 857 | gcctctgggcgcagatataattt | 410704 | - | see also 23781 line |
| 53705 | TSARG6 | NM_153614.2 | 776 | ctcgggatcactcgcaattcaga | 423400 | - | see also 23782 line |
| 53706 | TSARG6 | NM_153614.2 | 823 | ccgcagactcgcccttaagcacc | 423402 | - | see also 23782 line |
| 53707 | TSGA13 | NM_052933.2 | 732 | aggataagacgttactgattatg | 405374 | - | see also 23783 line |
| 53708 | TSGA13 | NM_052933.2 | 572 | gtcgggcaatcaaaatttgttct | 405365 | - | see also 23783 line |
| 53709 | TSGA14 | NM_018718.1 | 886 | ctgggtctgcccggaaacgatcc | 372924 | - | see also 23784 line |
| 53710 | TSGA14 | NM_018718.1 | 797 | ttccggaggtctaaaagtcttag | 372921 | - | see also 23784 line |
| 53711 | TSLL2 | NM_145296.1 | 949 | gagggcgctctacgtacttgtgg | 414263 | - | see also 23785 line |
| 53712 | TSLL2 | NM_145296.1 | 394 | tgccacgctcacggtactagtgg | 414258 | - | see also 23785 line |
| 53713 | TSLP | NM_033035.2 | 467 | tgcgcgtcgctcgccaaagaaat | 402664 | - | see also 23786 line |
| 53714 | TSLP | NM_033035.2 | 469 | cgcgtcgctcgccaaagaaatgt | 402666 | - | see also 23786 line |
| 53715 | TSLRP | NM_012472.3 | 1088 | aacttacgtgcgagtaatgatca | 338265 | - | see also 5348 line |
| 53716 | TSLRP | NM_012472.3 | 1085 | accaacttacgtgcgagtaatga | 338264 | - | see also 5348 line |
| 53717 | TSLRP | NM_012472.3 | 1092 | tacgtgcgagtaatgatcaaagg | 338266 | - | see also 5348 line |
| 53718 | TSNAXIP1 | NM_018430.1 | 1577 | gtcagaacgaggggctactaacc | 371359 | - | see also 23788 line |
| 53719 | TSNAXIP1 | NM_018430.1 | 1714 | ctcaactaccgctcactgtttat | 371360 | - | see also 23788 line |
| 53720 | TSNAXIP1 | NM_018430.1 | 2027 | agcggcttcaggtgattgacatc | 371361 | - | see also 23788 line |
| 53721 | TSP-NY | NM_032573.2 | 1688 | ctgccacccgagtaacttcataa | 400803 | - | see also 23789 line |
| 53722 | TSP-NY | NM_032573.2 | 1689 | tgccacccgagtaacttcataat | 400804 | - | see also 23789 line |
| 53723 | TSRC1 | NM_019032.2 | 1135 | gggagacgccctgatcctttcc | 374563 | - | see also 23790 line |
| 53724 | TSRC1 | NM_019032.2 | 1901 | gcgggaccgtctttcgatataac | 374569 | - | see also 23790 line |
| 53725 | TSSC1 | NM_003310.1 | 479 | cacgagtccctgatgattcatc | 331501 | - | see also 23791 line |
| 53726 | TSSC1 | NM_003310.1 | 476 | agccacgagtccctgatgattc | 331499 | - | see also 23791 line |
| 53727 | TSSC4 | NM_005706.2 | 228 | aacacgggacggagtatgacacg | 334178 | - | see also 23792 line |
| 53728 | TSSC4 | NM_005706.2 | 404 | gccacggtgcagccattccatct | 334179 | - | see also 23792 line |
| 53729 | TTC11 | NM_016068.1 | 171 | gtccaagagcacgcagtttgagt | 356179 | - | see also 23793 line |
| 53730 | TTC13 | NM_024525.2 | 2525 | aacgttaagatcgatgattgagg | 386702 | - | see also 23794 line |
| 53731 | TTC13 | NM_024525.2 | 1445 | agggttgcaaccccacataaaag | 386676 | - | see also 23794 line |
| 53732 | TTC13 | NM_024525.2 | 2071 | atggattcacaatcacgattaca | 386693 | - | see also 23794 line |
| 53733 | TTC15 | NM_016030.5 | 1904 | ggcgtatcattcggttatcaagt | 356030 | - | see also 23795 line |
| 53734 | TTC15 | NM_016030.5 | 1894 | tggccgtggaggcgtatcattcg | 356028 | - | see also 23795 line |

Figure 46

| | | | | | | | |
|---|---|---|---|---|---|---|---|
| 53735 | TTC4 | NM_004623.2 | 164 | gacgagcgcgccatcagaaattg | 332628 | - | see also 23796 line |
| 53736 | TTC4 | NM_004623.2 | 253 | aagaacaggccaagacctataaa | 332631 | - | see also 23796 line |
| 53737 | TTC5 | NM_138376.1 | 725 | aacagggcgacgttgcataaata | 407802 | - | see also 23797 line |
| 53738 | TTC5 | NM_138376.1 | 727 | cagggcgacgttgcataaatatg | 407803 | - | see also 23797 line |
| 53739 | TTC8 | NM_144596.1 | 1080 | aagccatcgcatgcattggaagc | 410154 | - | see also 9709 line |
| 53740 | TTF1 | NM_007344.2 | 2299 | gacttaatgtcggcgtatctact | 337455 | - | see also 5081 line |
| 53741 | TTF1 | NM_007344.2 | 1802 | aggagatactgtttagattaca | 337436 | - | see also 5081 line |
| 53742 | TTYH2 | NM_032646.4 | 891 | ctcgctacactgtattgcagc | 401171 | - | see also 23801 line |
| 53743 | TTYH2 | NM_032646.4 | 316 | tgcggtgggcgttggttctatg | 401165 | - | see also 23801 line |
| 53744 | TU12B1-TY | NM_016575.1 | 312 | tacagcgggcagtattaatgaag | 358194 | - | see also 23802 line |
| 53745 | TU12B1-TY | NM_016575.1 | 1036 | ggccggatttgaccctaaagcatg | 358208 | - | see also 23802 line |
| 53746 | TU12B1-TY | NM_016575.1 | 1330 | gtcggcgttcgctgacatctaca | 358215 | - | see also 23802 line |
| 53747 | TUWD12 | NM_172240.1 | 125 | acccgttctggagcgttatttc | 424304 | - | see also 23803 line |
| 53748 | TUWD12 | NM_172240.1 | 564 | accgatggaagactaattgtgt | 424322 | - | see also 23803 line |
| 53749 | TUWD12 | NM_172240.1 | 126 | cccgttctggagcgttatttca | 424305 | - | see also 23803 line |
| 53750 | UBAP1 | NM_016525.3 | 328 | agctaaagttggtctacctattg | 358014 | - | see also 6900 line |
| 53751 | UBAP1 | NM_016525.3 | 1543 | gggcttcgaccctctttagtgg | 358045 | - | see also 6900 line |
| 53752 | UBAP2 | NM_018449.2 | 2671 | gagatgggaggcctagctaataat | 371759 | - | see also 23805 line |
| 53753 | UBAP2 | NM_018449.2 | 328 | tagtggccctacatgattgtaat | 371714 | - | see also 23805 line |
| 53754 | UBE4B | NM_006048.2 | 2874 | gaccccgcatatcccgatataac | 334741 | - | see also 4208 line |
| 53755 | UBE4B | NM_006048.2 | 2875 | accccgcatatcccgatataaca | 334742 | - | see also 4208 line |
| 53756 | UBE4B | NM_006048.2 | 2609 | gcctagttgccgtcgcctatatcc | 334735 | - | see also 4208 line |
| 53757 | UBN1 | NM_016936.2 | 1977 | ctcaagcgtgtcggaaacttca | 358786 | - | see also 23807 line |
| 53758 | UBN1 | NM_016936.2 | 2504 | gtcgtctacgaagcctgataaaa | 358792 | - | see also 23807 line |
| 53759 | UBN1 | NM_016936.2 | 2505 | tcgtctacgaagcctgataaaaa | 358793 | - | see also 23807 line |
| 53760 | UBPH | NM_019116.1 | 1327 | tccggcatgtacaataaatctgg | 375288 | - | see also 23808 line |
| 53761 | UBPH | NM_019116.1 | 1066 | ctcgtcccgaggataaaaacatt | 375275 | - | see also 23808 line |
| 53762 | UBQLN1 | NM_013438.2 | 364 | ctccgcggagcccaaaatcatga | 338923 | - | see also 5443 line |
| 53763 | UBQLN1 | NM_013438.2 | 1345 | tactactggccaaattggtgc | 338936 | - | see also 5443 line |
| 53764 | UBQLN1 | NM_013438.2 | 797 | atgcagcgacaactttgtctaa | 338931 | - | see also 5443 line |
| 53765 | UBQLN2 | NM_013444.2 | 2000 | ctcaacgcaatggggttcttaaa | 338968 | - | see also 5446 line |
| 53766 | UBQLN2 | NM_013444.2 | 1291 | cacgttgctgccgctaattatg | 338959 | - | see also 5446 line |
| 53767 | UBXD1 | NM_025241.1 | 1085 | ggctgcgaagtacaactacacg | 394517 | - | see also 23811 line |
| 53768 | UBXD1 | NM_025241.1 | 561 | cgcctccatcgaagaatctaca | 394515 | - | see also 23811 line |

Figure 46

| 53769 | UBXD2 | NM_014607.2 | 929 | ggcagctcgagaacgtataaaac | 341004 | - | see also 23812 line | | |
|---|---|---|---|---|---|---|---|---|---|
| 53770 | UBXD2 | NM_014607.2 | 928 | gggcagctcgagaacgtataaaa | 341003 | - | see also 23812 line | | |
| 53771 | UBXD2 | NM_014607.2 | 932 | agctcgagaacgtataaaacagc | 341005 | - | see also 23812 line | | |
| 53772 | UNC93A | NM_018974.2 | 1160 | tgccgtctggcagacacaaaaca | 373926 | - | see also 23813 line | | |
| 53773 | UNC93A | NM_018974.2 | 813 | ctgtcgactttcaagctatatag | 373918 | - | see also 23813 line | | |
| 53774 | UNC93B1 | NM_030930.1 | 599 | ggcgcagaagtaccatgagtact | 395541 | - | see also 8966 line | | |
| 53775 | UNC93B1 | NM_030930.1 | 317 | gacctaccgcgaggtgaagtatg | 395535 | - | see also 8966 line | | |
| 53776 | UNQ1912 | NM_198507.1 | 259 | gaccctagaccacggacattacc | 438790 | - | see also 23815 line | | |
| 53777 | UNQ1912 | NM_198507.1 | 179 | tgctgttgctgcctgaactaagc | 438789 | - | see also 23815 line | | |
| 53778 | UNQ2446 | NM_198443.1 | 93 | ttgctgccctcgtcctttacc | 437791 | - | see also 23816 line | | |
| 53779 | UNQ2446 | NM_198443.1 | 398 | cggcacaggctccgaaaccaacc | 437793 | - | see also 23816 line | | |
| 53780 | UNQ2492 | NM_198585.1 | 636 | gccggtctcccgtggacttttgg | 439841 | - | see also 23817 line | | |
| 53781 | UNQ2492 | NM_198585.1 | 854 | aagagcacccaggccgattttcg | 439844 | - | see also 23817 line | | |
| 53782 | UNQ2492 | NM_198585.1 | 1143 | tccgggaacttttcaacttctcc | 439846 | - | see also 23817 line | | |
| 53783 | UNQ3030 | NM_198565.1 | 1360 | ctggcctcttcgccaatgctagg | 439597 | - | see also 23818 line | | |
| 53784 | UNQ3030 | NM_198565.1 | 1789 | ccctggatctccgtagaaactcg | 439607 | - | see also 23818 line | | |
| 53785 | UNQ3033 | NM_198481.1 | 729 | gaggctgccgaggcagatttatc | 438475 | - | see also 23819 line | | |
| 53786 | UNQ3033 | NM_198481.1 | 399 | cagagctcggcagaaaacgaagc | 438473 | - | see also 23819 line | | |
| 53787 | UNQ473 | NM_198477.1 | 369 | aagcagtgcccctgtgatcattt | 438443 | - | see also 23821 line | | |
| 53788 | UNQ473 | NM_198477.1 | 462 | cagcaatttctcaaacaatgtca | 438446 | - | see also 23821 line | | |
| 53789 | UNQ564 | NM_198474.1 | 262 | agcaacgagggcatacattcaag | 438409 | - | see also 23822 line | | |
| 53790 | UNQ564 | NM_198474.1 | 872 | ggggtaggccgagcattggttta | 438425 | - | see also 23822 line | | |
| 53791 | UNQ6369 | NM_198510.1 | 804 | tggagacgtgcagatttacgatg | 438804 | - | see also 23823 line | | |
| 53792 | UNQ6369 | NM_198510.1 | 811 | gtgcagatttacgatgactattt | 438805 | - | see also 23823 line | | |
| 53793 | UNQ698 | NM_198538.1 | 142 | cagcgatgaccccattgagaagg | 439297 | - | see also 23824 line | | |
| 53794 | UNQ698 | NM_198538.1 | 617 | gagctgcagaatgctcataatgg | 439298 | - | see also 23824 line | | |
| 53795 | UNQ739 | NM_198570.1 | 1523 | gccatgtgcacgagacatgaatg | 439704 | - | - | - | - |
| 53796 | UNQ739 | NM_198570.1 | 1471 | caccatatgccactgtacttatg | 439703 | - | see also 53795 line | | |
| 53797 | UNQ8193 | NM_198450.1 | 472 | acccagttcagtccgtaataatt | 437848 | - | see also 10332 line | | |
| 53798 | UNQ8193 | NM_198450.1 | 471 | tacccagttcagtccgtaataat | 437847 | - | see also 10332 line | | |
| 53799 | UNQ9366 | NM_198444.1 | 1505 | aacacgacgggatctacaactgc | 437809 | - | see also 23826 line | | |
| 53800 | UNQ9366 | NM_198444.1 | 1910 | gccgtagaggcaattctttcagc | 437812 | - | see also 23826 line | | |
| 53801 | UNQ9366 | NM_198444.1 | 1015 | aaggtccgagtacttgaaacact | 437803 | - | see also 23826 line | | |
| 53802 | UNQ9391 | NM_198464.1 | 386 | tggggaccaacgacttaactagc | 438071 | - | see also 23827 line | | |
| 53803 | UNQ9391 | NM_198464.1 | 870 | cacctcgttggtgaactacaacc | 438082 | - | see also 23827 line | | |
| 53804 | UNRIP | NM_007178.2 | 335 | cacgcgacccgtggttgatttgg | 337012 | - | see also 23828 line | | |
| 53805 | UNRIP | NM_007178.2 | 848 | gtctgttagtagtatggaatata | 337014 | - | see also 23828 line | | |

Figure 46

| 53806 | UPK3B | NM_030570.2 | 482 | tgccctacacaccacagataaca | 394674 | - | see also 23829 line |
|---|---|---|---|---|---|---|---|
| 53807 | URB | NM_178274.2 | 942 | tccgtaggatcgagaagatcagg | 430651 | - | see also 10183 line |
| 53808 | URB | NM_178274.2 | 2255 | gagtacggaatgacctacaatga | 430665 | - | see also 10183 line |
| 53809 | URG4 | NM_017920.2 | 1407 | accggatggagaggattaccagg | 363970 | - | see also 23831 line |
| 53810 | URG4 | NM_017920.2 | 1155 | accgtgggaagcgcaaacacaaac | 363967 | - | see also 23831 line |
| 53811 | URG4 | NM_017920.2 | 1046 | atctcctcgctgtgtttatatt | 363966 | - | see also 23831 line |
| 53812 | URP | NM_198152.1 | 81 | ttggactcctaactttgttatcc | 437152 | - | see also 23832 line |
| 53813 | URP | NM_198152.1 | 247 | cactgacctagccttacctaaca | 437153 | - | see also 23832 line |
| 53814 | USH1G | NM_173477.2 | 316 | ctcgagtcgtcgcgtcttcattgt | 424945 | - | see also 23833 line |
| 53815 | USH1G | NM_173477.2 | 1300 | ccctgggatgagctcgatttagg | 424949 | - | see also 23833 line |
| 53816 | USH1G | NM_173477.2 | 1299 | gccctgggatgagctcgatttag | 424948 | - | see also 23833 line |
| 53817 | VCIP135 | NM_025054.3 | 418 | gtgtcaggcgc-gtctattttcc | 393256 | - | see also 8835 line |
| 53818 | VCIP135 | NM_025054.3 | 416 | aagtgtcaggcgccgtctattttt | 393255 | - | see also 8835 line |
| 53819 | VCIP135 | NM_025054.3 | 529 | cccggacgtagtgctacacaacc | 393257 | - | see also 8835 line |
| 53820 | VCY2IP1 | NM_018174.3 | 148 | gggctcgcaaccttgatggaaca | 367994 | - | - |
| 53821 | VCY2IP1 | NM_018174.3 | 1406 | ggcgagccgagcgaagagagc | 367995 | - | see also 53820 line |
| 53822 | VEST1 | NM_052958.1 | 2106 | ccctagctgctgaaatgctaca | 405543 | - | see also 23835 line |
| 53823 | VEST1 | NM_052958.1 | 2006 | ttcagatgaactcgataaggaaa | 405542 | - | see also 23835 line |
| 53824 | VGLL2 | NM_153453.1 | 375 | agccaaacccccagccagtataaa | 423277 | - | see also 23836 line |
| 53825 | VGLL2 | NM_153453.1 | 376 | gccaaacccccagccagtataaaa | 423278 | - | see also 23836 line |
| 53826 | VGLL2 | NM_153453.1 | 283 | accagaaactagcctattattcc | 423275 | - | see also 23836 line |
| 53827 | VIT | NM_053276.2 | 573 | tccaacggtgtccaatcgttatc | 405943 | - | see also 23837 line |
| 53828 | VIT | NM_053276.2 | 723 | ggctatcagaggccacctattcc | 405948 | - | see also 23837 line |
| 53829 | VMP | NM_080723.2 | 804 | cacacccagccgtcggttacaaagc | 406675 | - | - |
| 53830 | VMP | NM_080723.2 | 802 | cccacaccccagccgttacaaaa | 406674 | - | see also 53829 line |
| 53831 | VMP | NM_080723.2 | 428 | agcctcaatttgggagtatgagg | 406672 | - | see also 53829 line |
| 53832 | VMP1 | NM_030938.2 | 592 | caccctatcctgatcagattatt | 395605 | - | see also 8971 line |
| 53833 | VMP1 | NM_030938.2 | 925 | ttgatctggctggaataacgtgt | 395618 | - | see also 8971 line |
| 53834 | VprBP | NM_014703.1 | 3296 | ggcggcaaagcgccaataaacttt | 342521 | - | see also 23839 line |
| 53835 | VprBP | NM_014703.1 | 3302 | aagcgccaataaacttacgtca | 342525 | - | see also 23839 line |
| 53836 | VPS52 | NM_022553.3 | 1542 | agcgcctagggggggttggatact | 383570 | - | see also 23840 line |
| 53837 | VPS52 | NM_022553.3 | 1208 | cactacgccctcctagacaattc | 383565 | - | see also 23840 line |
| 53838 | VPS54 | NM_016516.1 | 252 | aacgatcctagattagcaaaaag | 357911 | - | see also 6888 line |
| 53839 | VPS54 | NM_016516.1 | 1677 | tccgagccagaatgtactactga | 357942 | - | see also 6888 line |
| 53840 | VR22 | NM_013266.1 | 565 | tgcggtgacgagactccttatcc | 338503 | - | see also 23841 line |
| 53841 | VR22 | NM_013266.1 | 1623 | aggtacatggagaatcatata | 338527 | - | see also 23841 line |
| 53842 | VR22 | NM_013266.1 | 2421 | ctcggcagattgctaatcagtgc | 338548 | - | see also 23841 line |

Figure 46

| 53843 | WAC | NM_016628.2 | 1832 | tacgcgaagaagcgcataacatg | 358667 | - | see also 23842 line |
|---|---|---|---|---|---|---|---|
| 53844 | WAC | NM_016628.2 | 1720 | gcccgatccacgtgttcattaac | 358665 | - | see also 23842 line |
| 53845 | WBP2 | NM_012478.2 | 288 | gcccgtatttggtgcaaactaca | 338288 | - | see also 5354 line |
| 53846 | WBP2 | NM_012478.2 | 504 | cccgccagtcgccaatggaatgt | 338290 | - | see also 5354 line |
| 53847 | WBSCR20A | NM_018044.2 | 81 | gagccgccagggctctatcaagg | 365765 | - | see also 23844 line |
| 53848 | WBSCR27 | NM_152559.2 | 550 | ctcgtccaaccttcaatacaagg | 418110 | - | - | | - |
| 53849 | WBSCR27 | NM_152559.2 | 487 | tgcgatacctgagctacatgtca | 418109 | - | see also 53848 line |
| 53850 | WBSCR28 | NM_182504.2 | 119 | accgagatcacctctataatttc | 433990 | - | see also 23845 line |
| 53851 | WBSCR28 | NM_182504.2 | 118 | aaccgagatcacctctataattt | 433989 | - | see also 23845 line |
| 53852 | WDR10 | NM_018262.2 | 2188 | acctcgcgcttgaaatgtacacc | 369384 | - | see also 7347 line |
| 53853 | WDR10 | NM_018262.2 | 293 | ggctgccggaagcagattactgg | 369351 | - | see also 7347 line |
| 53854 | WDR12 | NM_018256.2 | 1164 | gtgggatccccgaactaaagatg | 369273 | - | see also 23847 line |
| 53855 | WDR12 | NM_018256.2 | 1199 | cgctgtccctaacgtcacatact | 369276 | - | see also 23847 line |
| 53856 | WDR17 | NM_170710.2 | 347 | atcagttggatcaccgttataat | 423969 | - | see also 10024 line |
| 53857 | WDR17 | NM_170710.2 | 382 | cacgcaattatgtctgaacataa | 423973 | - | see also 10024 line |
| 53858 | WDR18 | NM_024100.2 | 587 | tggaccagacggtgaagctatgg | 386029 | - | see also 23849 line |
| 53859 | WDR18 | NM_024100.2 | 1007 | tgccgctgccccacttcaacaag | 386030 | - | see also 23849 line |
| 53860 | WDR20 | NM_144574.2 | 498 | cacgagttacctgtgtcaaatgg | 409865 | - | see also 9702 line |
| 53861 | WDR20 | NM_144574.2 | 552 | cccactcgagtgggaacatgtac | 409867 | - | see also 9702 line |
| 53862 | WDR20 | NM_144574.2 | 806 | gagctgcacggtacgatgaaaag | 409876 | - | see also 9702 line |
| 53863 | WDR7 | NM_015285.1 | 2484 | accccactgcaccgtatcgtttg | 351091 | - | see also 23851 line |
| 53864 | WDR7 | NM_015285.1 | 2485 | ccccactgcaccgtatcgtttgg | 351092 | - | see also 23851 line |
| 53865 | WDR8 | NM_017818.2 | 242 | gtcggcagactcgctcttcatcc | 362717 | - | see also 23852 line |
| 53866 | WDR8 | NM_017818.2 | 437 | gtgcacaaaatccgtgtcttaca | 362719 | - | see also 23852 line |
| 53867 | WFDC10B | NM_172131.2 | 186 | agcgacccagcatagatctatgc | 424207 | - | see also 23853 line |
| 53868 | WFDC10B | NM_172131.2 | 182 | gagaagcgacccagcatagatct | 424205 | - | see also 23853 line |
| 53869 | WFDC11 | NM_147197.2 | 341 | aagaatgtaccaataagtgttct | 415099 | - | see also 23854 line |
| 53870 | WFDC11 | NM_147197.2 | 360 | ttctaaagcctttagatgtaaag | 415100 | - | see also 23854 line |
| 53871 | WFDC13 | NM_172005.1 | 177 | tcccaagcagcgtgttctgaagt | 424200 | - | see also 23855 line |
| 53872 | WFDC13 | NM_172005.1 | 182 | agcagcgtgttctgaagtatatc | 424202 | - | see also 23855 line |
| 53873 | WFDC6 | NM_080827.1 | 114 | tcctggtaccattcatccttttg | 406791 | - | see also 23856 line |
| 53874 | WFDC6 | NM_080827.1 | 109 | gccaatcctggtaccattcatcc | 406790 | - | see also 23856 line |
| 53875 | WFDC6 | NM_080827.1 | 311 | gtcagccttactttataccataa | 406795 | - | see also 23856 line |
| 53876 | WFDC9 | NM_147198.2 | 392 | gacttgtgtacgtccaaatcata | 415107 | - | see also 23857 line |
| 53877 | WFDC9 | NM_147198.2 | 453 | aacgaagagcccottaaatcaat | 415109 | - | see also 23857 line |
| 53878 | WFDC9 | NM_147198.2 | 460 | agcccottaaatcaatgctaaac | 415112 | - | see also 23857 line |

Figure 46

| | | | | | | | |
|---|---|---|---|---|---|---|---|
| 53879 | WINS1 | NM_018148.3 | 405 | cccggatattgtctgtcaaaacc | 367688 | - | see also 23858 line |
| 53880 | WINS1 | NM_018148.3 | 404 | acccggatattgtctgtcaaaac | 367687 | - | see also 23858 line |
| 53881 | WINS1 | NM_018148.3 | 1225 | cactagtccagatcatgtgatcc | 367708 | - | see also 23858 line |
| 53882 | WIRE | NM_133264.2 | 76 | cagcccaagctgagtagaagatga | 407159 | - | see also 9562 line |
| 53883 | WIRE | NM_133264.2 | 470 | cggaccctctcgcctaataacc | 407163 | - | see also 9562 line |
| 53884 | WTAP | NM_004906.3 | 244 | cccaagaaggttcgattgagtga | 333212 | - | see also 23860 line |
| 53885 | WTAP | NM_004906.3 | 283 | atggccaagatgatgagttaattct | 333214 | - | see also 23860 line |
| 53886 | WWP2 | NM_007014.3 | 1931 | gggctgtaccatggaaagttca | 336776 | - | see also 4892 line |
| 53887 | WWP2 | NM_007014.3 | 1455 | agcgaggggtgcgatactttgt | 336764 | - | see also 4892 line |
| 53888 | XPO5 | NM_020750.1 | 2213 | accgtgcacgaatgagctttgt | 378513 | - | see also 7939 line |
| 53889 | XPO5 | NM_020750.1 | 1405 | aggttggcatgtgtttggatcc | 378495 | - | see also 7939 line |
| 53890 | XTP2 | NM_015172.2 | 6857 | gcctaacaccgtggctactaata | 348862 | - | see also 23864 line |
| 53891 | XTP2 | NM_015172.2 | 5569 | ctccacttccggcaaccttaact | 348840 | - | see also 23864 line |
| 53892 | XTP3TPA | NM_024096.1 | 478 | agctcttccgcaagtatacaga | 386003 | - | - |
| 53893 | XTP3TPA | NM_024096.1 | 483 | ttccgcaagtatacagaattgc | 386004 | - | see also 53892 line |
| 53894 | XTP3TPB | NM_015701.2 | 1050 | atccacaagtcgattgctattgg | 355174 | - | see also 6537 line |
| 53895 | XTP3TPB | NM_015701.2 | 483 | gagtcttattggacttacgaagt | 355162 | - | see also 6537 line |
| 53896 | YPEL1 | NM_013313.3 | 584 | cacgctcggcttggaaatacgagc | 338703 | - | see also 23867 line |
| 53897 | YPEL1 | NM_013313.3 | 581 | gaccacgctcggcttggaaatacg | 338702 | - | see also 23867 line |
| 53898 | Z391G | NM_007268.1 | 573 | ttggtataagcaacagactaata | 337370 | - | see also 23868 line |
| 53899 | Z391G | NM_007268.1 | 416 | aagtcgtgagagataagattact | 337367 | - | see also 23868 line |
| 53900 | ZA20D1 | NM_020205.1 | 693 | aacaggccagggcgtttgaactgg | 376142 | - | see also 23869 line |
| 53901 | ZA20D1 | NM_020205.1 | 2505 | accgagtggctgattcctatagc | 376153 | - | see also 23869 line |
| 53902 | ZAR1 | NM_175619.1 | 1101 | cccttacgagtgagggatatca | 429165 | - | see also 23870 line |
| 53903 | ZAR1 | NM_175619.1 | 1035 | gtgtgtacagggaactaacaagg | 429161 | - | see also 23870 line |
| 53904 | ZBTB1 | NM_014950.1 | 1921 | gtctagctgcttagatcaagata | 346320 | - | see also 23871 line |
| 53905 | ZBTB1 | NM_014950.1 | 1458 | gaggtggtttagagataatgtgagg | 346305 | - | see also 23871 line |
| 53906 | ZCWCC2 | NM_024657.2 | 2230 | atcgggtatgactcagaacaagt | 388660 | - | see also 8681 line |
| 53907 | ZCWCC2 | NM_024657.2 | 1515 | aggtagcacaccataaacaaag | 388648 | - | see also 8681 line |
| 53908 | ZCWPW1 | NM_017984.2 | 1671 | cccattcgtaagcgagtcaaaat | 365092 | - | see also 23873 line |
| 53909 | ZCWPW1 | NM_017984.2 | 1528 | gccgattcaacgatctaacagt | 365087 | - | see also 23873 line |
| 53910 | ZD52F10 | NM_033317.2 | 1224 | gacgtcctcgggatgtttaact | 403691 | - | see also 23874 line |
| 53911 | ZD52F10 | NM_033317.2 | 359 | gggcagctggctctaaagtcagt | 403688 | - | see also 23874 line |
| 53912 | ZDHHC14 | NM_024630.2 | 1053 | aactgcgtagaacgtttgatca | 388286 | - | see also 8672 line |
| 53913 | ZDHHC14 | NM_024630.2 | 671 | ccggcagcacgggcgtcttcacc | 388281 | - | see also 8672 line |
| 53914 | ZDHHC15 | NM_144969.1 | 107 | gtcgtgctcggtcctactatgc | 412013 | - | see also 9748 line |
| 53915 | ZDHHC15 | NM_144969.1 | 854 | ttctggttaatacctattggttc | 412028 | - | see also 9748 line |

Figure 46

| | | | | | | | |
|---|---|---|---|---|---|---|---|
| 53916 | ZDHHC16 | NM_032327.2 | 352 | ggcagtgacaccgctgttgatgc | 399746 | - | see also 9163 line |
| 53917 | ZDHHC16 | NM_032327.2 | 1126 | aagggcagagtatttaggaatcc | 399764 | - | see also 9163 line |
| 53918 | ZDHHC21 | NM_178566.2 | 665 | gtgagggcctccataactgatcc | 431319 | - | see also 10200 line |
| 53919 | ZFP106 | NM_022473.1 | 2660 | gccaaccctacggcagattctaa | 383108 | - | see also 23878 line |
| 53920 | ZFP106 | NM_022473.1 | 5743 | aggccgtgaggcttaatctgatg | 383203 | - | see also 23878 line |
| 53921 | ZFP276 | NM_152287.2 | 757 | gccacgactacaccatggatacc | 415365 | - | see also 9830 line |
| 53922 | ZFP42 | NM_174900.1 | 372 | agcgcggtgtgggccttatgtga | 428554 | - | see also 23880 line |
| 53923 | ZFP42 | NM_174900.1 | 1129 | tcctaacgcatgcaaatacgaac | 428573 | - | see also 23880 line |
| 53924 | ZFP42 | NM_174900.1 | 465 | tgcgtcataaggggtgagttttc | 428559 | - | see also 23880 line |
| 53925 | ZFP91 | NM_053023.2 | 433 | gagtccgcgactcctatgcatag | 405798 | - | see also 23881 line |
| 53926 | ZFP91 | NM_053023.2 | 1236 | gtgctcgggccttcaagagttcc | 405808 | - | see also 23881 line |
| 53927 | ZFPM1 | NM_153813.1 | 456 | ctcccagcgcagatgttaactca | 423923 | - | see also 23882 line |
| 53928 | ZFPM1 | NM_153813.1 | 458 | cccagcgcagatgttaactcacc | 423924 | - | see also 23882 line |
| 53929 | ZG16 | NM_152338.1 | 76 | tcctcctatagtggagagtatgg | 415712 | - | see also 23883 line |
| 53930 | ZG16 | NM_152338.1 | 164 | gggtccgagtcaacacatactac | 415715 | - | see also 23883 line |
| 53931 | ZIC1 | NM_003412.2 | 970 | gccacgtcggctcctattccagc | 331655 | - | see also 2306 line |
| 53932 | ZIC1 | NM_003412.2 | 2024 | cagtgcgctctcttccaatttta | 331665 | - | see also 2306 line |
| 53933 | ZMYND10 | NM_015896.2 | 998 | tgccaagggacggctactcaagc | 355371 | - | see also 23885 line |
| 53934 | ZMYND10 | NM_015896.2 | 756 | agcaccttgagccgtatgcttag | 355367 | - | see also 23885 line |
| 53935 | ZMYND12 | NM_032257.3 | 695 | atcgttcaggctgaagaatatct | 399080 | - | see also 23886 line |
| 53936 | ZMYND12 | NM_032257.3 | 908 | tacttccacctggctaatatatt | 399088 | - | see also 23886 line |
| 53937 | ZMYND12 | NM_032257.3 | 1130 | atcttgaacattcgagaatctac | 399095 | - | see also 23886 line |
| 53938 | ZMYND15 | NM_032265.1 | 94 | ttcggctggttccgaaagtttgt | 399145 | - | see also 23887 line |
| 53939 | ZMYND15 | NM_032265.1 | 51 | gtctggataccgggatgagttcc | 399142 | - | see also 23887 line |
| 53940 | ZMYND17 | NM_178451.2 | 760 | ttcgtgactataagattcctaca | 430738 | - | see also 23888 line |
| 53941 | ZMYND17 | NM_178451.2 | 310 | ccccgtatcaggctttggatttc | 430729 | - | see also 23888 line |
| 53942 | ZMYND17 | NM_178451.2 | 889 | aacctgaacaggtctattccagt | 430741 | - | see also 23888 line |
| 53943 | ZMYND19 | NM_138462.2 | 589 | agcttgtattggcttgcaattca | 408293 | - | see also 23889 line |
| 53944 | ZMYND19 | NM_138462.2 | 616 | ctgcctacagaccctatagaaga | 408294 | - | see also 23889 line |
| 53945 | ZNF169 | NM_194320.1 | 946 | ggcgacacttcaggtatacatcc | 436852 | - | see also 23890 line |
| 53946 | ZNF169 | NM_194320.1 | 943 | gtgggcgacacttcaggtataca | 436850 | - | see also 23890 line |
| 53947 | ZNF233 | NM_181756.1 | 869 | tcccagcatagcataatccaatc | 433104 | - | see also 23891 line |
| 53948 | ZNF233 | NM_181756.1 | 476 | accagtaatcaagacctaataat | 433095 | - | see also 23891 line |
| 53949 | ZNF233 | NM_181756.1 | 475 | aaccagtaatcaagacctaataa | 433094 | - | see also 23891 line |
| 53950 | ZNF237 | NM_014242.1 | 241 | gacataggggattcatttggtca | 339980 | - | see also 23892 line |
| 53951 | ZNF237 | NM_014242.1 | 553 | aagtcttcctgttttatcgaatg | 339986 | - | see also 23892 line |
| 53952 | ZNF237 | NM_014242.1 | 595 | aacaaaaccaacgatttggattt | 339987 | - | see also 23892 line |

Figure 46

| 53953 | ZNF291 | NM_020843.1 | 2355 | ggcgacatgcaaatactgattat | 379537 | - | see also 7989 line | | | |
|---|---|---|---|---|---|---|---|---|---|---|
| 53954 | ZNF291 | NM_020843.1 | 256 | atcgtctacgactggagataaac | 379469 | - | see also 7989 line | | | |
| 53955 | ZNF294 | NM_015565.1 | 1628 | gtccgttttgggtgtatctaacc | 353948 | - | see also 23894 line | | | |
| 53956 | ZNF294 | NM_015565.1 | 2596 | cagcgaaaggatgcttgctaatg | 353977 | - | see also 23894 line | | | |
| 53957 | ZNF313 | NM_018683.3 | 629 | ggcaccggttttcttatgacact | 372540 | - | see also 7554 line | | | |
| 53958 | ZNF313 | NM_018683.3 | 524 | tccatagcacggataccaaatct | 372538 | - | see also 7554 line | | | |
| 53959 | ZNF313 | NM_018683.3 | 161 | tgccctgcggacacgtcttttgc | 372532 | - | see also 7554 line | | | |
| 53960 | ZNF364 | NM_014455.1 | 378 | ggcggcagtcggatagacaatac | 340680 | - | see also 5679 line | | | |
| 53961 | ZNF364 | NM_014455.1 | 282 | ggcgaggtcagccccaaactacc | 340673 | - | see also 5679 line | | | |
| 53962 | ZNF367 | NM_153695.1 | 492 | cagcagaatccgttgtaacatct | 423573 | - | see also 23897 line | | | |
| 53963 | ZNF367 | NM_153695.1 | 996 | tggagccctcgcgctcatagagc | 423580 | - | see also 23897 line | | | |
| 53964 | ZNF407 | NM_017757.1 | 2035 | gtcgttctataccgaaagcaacc | 361869 | - | see also 23898 line | | | |
| 53965 | ZNF407 | NM_017757.1 | 2570 | tgccacttatgtgatagaagttt | 361881 | - | see also 23898 line | | | |
| 53966 | ZNF410 | NM_021188.1 | 871 | ttgtatggccagctcactttaaa | 380724 | - | see also 8120 line | | | |
| 53967 | ZNF410 | NM_021188.1 | 1522 | tgcagtcagggaggcaatcatat | 380735 | - | see also 8120 line | | | |
| 53968 | ZNF445 | NM_181489.3 | 830 | tgcgatctgcacagatatggtcc | 432140 | - | see also 10232 line | | | |
| 53969 | ZNF445 | NM_181489.3 | 3413 | ctcggcatatgaaaaaccatatt | 432178 | - | see also 10232 line | | | |
| 53970 | ZNF488 | NM_153034.2 | 1020 | aacgtccgacctggtctttcaca | 421991 | - | - | | - | - |
| 53971 | ZNF488 | NM_153034.2 | 1023 | gtccgacctggtctttcacatgc | 421992 | - | see also 53970 line | | | |
| 53972 | ZNF488 | NM_153034.2 | 635 | agcaaagaagcgcctttagcaaa | 421990 | - | see also 53970 line | | | |
| 53973 | ZNF498 | NM_145115.1 | 1298 | ccccctgcctgacgaagtcaaaa | 413535 | - | - | | - | - |
| 53974 | ZNF498 | NM_145115.1 | 1300 | ccctgcctgacgaagtcaaaacc | 413537 | - | see also 53973 line | | | |
| 53975 | ZNF498 | NM_145115.1 | 1299 | ccccTgcctgacgaagtcaaaac | 413536 | - | see also 53973 line | | | |
| 53976 | ZNF503 | NM_032772.3 | 589 | cgctgttggcgcaaacatgttcg | 401353 | - | see also 23900 line | | | |
| 53977 | ZNF503 | NM_032772.3 | 588 | gcgctgttggcgcaaacatgttc | 401352 | - | see also 23900 line | | | |
| 53978 | ZNF509 | NM_145291.2 | 1515 | acgtccagcgtcacattattatt | 414183 | - | see also 23901 line | | | |
| 53979 | ZNF509 | NM_145291.2 | 1575 | gtcgagggtttagtaacttcagt | 414188 | - | see also 23901 line | | | |
| 53980 | ZNF511 | NM_145806.2 | 687 | ggcggatctacagacatagaata | 414927 | - | see also 23902 line | | | |
| 53981 | ZNF511 | NM_145806.2 | 732 | agggtgccgctcgaggatttaaa | 414930 | - | see also 23902 line | | | |
| 53982 | ZNF513 | NM_144631.2 | 1268 | ctctgctcatctggataacctga | 410590 | - | see also 9722 line | | | |
| 53983 | ZNF513 | NM_144631.2 | 514 | ccccacggctactgtactcatgc | 410587 | - | see also 9722 line | | | |
| 53984 | ZNF515 | NM_152629.2 | 1343 | ggccggttgccctcgaagataca | 419154 | - | see also 23903 line | | | |
| 53985 | ZNF515 | NM_152629.2 | 2366 | gactcactcgggcattacagtgt | 419166 | - | see also 23903 line | | | |
| 53986 | ZNHIT1 | NM_006349.2 | 722 | aacttcgcttccgaaaaaacttt | 335309 | - | see also 23904 line | | | |
| 53987 | ZNHIT1 | NM_006349.2 | 697 | aagaaaacccgaggtgatcattt | 335305 | - | see also 23904 line | | | |
| 53988 | ZNHIT1 | NM_006349.2 | 726 | tcgcttccgaaaaaactttcagg | 335310 | - | see also 23904 line | | | |
| 53989 | ZNRF1 | NM_032268.3 | 1283 | tgcatagactcgtggtttgaagt | 399226 | - | see also 9129 line | | | |

Figure 46

| 53990 | ZPBP | NM_007009.1 | 773 | agcgatgtacagaccataactgt | 336735 | - | see also 23906 line | | |
|-------|------|-------------|-----|-------------------------|--------|---|---------------------|---|---|
| 53991 | ZPBP | NM_007009.1 | 677 | ctgaatgccatcgcgttaaaatg | 336730 | - | see also 23906 line | | |
| 53992 | ZPBP | NM_007009.1 | 522 | tgcttatcgtgagcctcattatt | 336726 | - | see also 23906 line | | |
| 53993 | ZRANB1 | NM_017580.1 | 1970 | tagacgaccaattatagtttatg | 359377 | - | see also 23907 line | | |
| 53994 | ZRANB1 | NM_017580.1 | 886 | gtgatcatcccagacctaataac | 359351 | - | see also 23907 line | | |
| 53995 | ZSIG11 | NM_015926.3 | 531 | cgctccatcgctgtctactatga | 355493 | - | - | - | - |
| 53996 | ZSIG11 | NM_015926.3 | 826 | accaggaagaccagatccatttc | 355494 | - | see also 53995 line | | |
| 53997 | ZSWIM2 | NM_182521.1 | 371 | gggggatacatcgagttcaaact | 434095 | - | see also 23908 line | | |
| 53998 | ZSWIM2 | NM_182521.1 | 361 | gacttgcttcggggatacatcg | 434093 | - | see also 23908 line | | |

# INTERNATIONAL SEARCH REPORT

| International application No. |
|---|
| PCT/IB2005/001647 |

**A. CLASSIFICATION OF SUBJECT MATTER**
Int.Cl$^7$ C12N15/09, A61K31/713, 48/00, A61P35/00//G06F17/30, G06F17/40, G06F17/50

According to International Patent Classification (IPC) or to both national classification and IPC

**B. FIELDS SEARCHED**

Minimum documentation searched (classification system followed by classification symbols)
Int.Cl$^7$ C12N15/09, A61K31/713, 48/00, A61P35/00//G06F17/30, G06F17/40, G06F17/50

Documentation searched other than minimum documentation to the extent that such documents are included in the fields searched
| Jitsuyo Shinan Koho | 1922-1996 | Jitsuyo Shinan Toroku Koho | 1996-2005 |
| Kokai Jitsuyo Shinan Koho | 1971-2005 | Toroku Jitsuyo Shinan Koho | 1994-2005 |

Electronic data base consulted during the international search (name of data base and, where practicable, search terms used)
BIOSIS/MEDLINE/WPIDS(STN), CAplus(STN), JMEDPlus(JOIS), JSTPlus(JOIS)

**C. DOCUMENTS CONSIDERED TO BE RELEVANT**

| Category* | Citation of document, with indication, where appropriate, of the relevant passages | Relevant to claim No. |
|---|---|---|
| X | UI-TEI, K. et al., Guidelines for the selection of highly effective siRNA sequences for mammalian and chick RNA interference, Nucleic Acids Res., 09 February, 2004 (09.02.04), Vol.32, No.3, pages 936 to 948 | 1-31 |
| E,X | WO 2004/048566 A1 (SAIGO, Kaoru et al.), 10 June, 2004 (10.06.04) & EP 1571209 A1 | 1-31 |
| P,X | Kumiko TEI et al., "RNAi Koka no Takai siRNA Hairetsu Sekkei Guideline", Dai 27 Kai The Molecular Biology Society of Japan Nenkai Program Koen Yoshishu, 25 November, 2004 (25.11.04), page 487, left column, 1PA-422 | 1-31 |

| ☒ Further documents are listed in the continuation of Box C. | ☐ See patent family annex. |
|---|---|

| * | Special categories of cited documents: | "T" | later document published after the international filing date or priority date and not in conflict with the application but cited to understand the principle or theory underlying the invention |
|---|---|---|---|
| "A" | document defining the general state of the art which is not considered to be of particular relevance | | |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
|---|---|
| 13 September, 2005 (13.09.05) | 27 September, 2005 (27.09.05) |

| Name and mailing address of the ISA/ | Authorized officer |
|---|---|
| Japanese Patent Office | |
| Facsimile No. | Telephone No. |

Form PCT/ISA/210 (second sheet) (January 2004)

| | INTERNATIONAL SEARCH REPORT | International application No. |
|---|---|---|
| | | PCT/IB2005/001647 |

C (Continuation).   DOCUMENTS CONSIDERED TO BE RELEVANT

| Category* | Citation of document, with indication, where appropriate, of the relevant passages | Relevant to claim No. |
|---|---|---|
| P,X | NAITO, Y. et al., siDirect: highly effective, target-specific siRNA design software for mammalian RNA interference, Nucleic Acids Res., 01 July, 2004 (01.07.04), Vol.32, Web Server issue, pages W124 to W129 | 1-31 |
| T | NAITO, Y. et al., dsCheck: highly sensitive off-target search software for double-stranded RNA-mediated RNA interference, Nucleic Acids Res., 01 July, 2005 (01.07.05), Vol.33, Web Server issue, pages W589 to W591 | 1-31 |
| A | DOI, Z. et al., Short-interfering-RNA-mediated gene silencing in mammalian cells requires Dicer and eIF2C translation initiation factors, Curr.Biol., 2003, Vol.13, No.1, pages 41 to 46 | 1-31 |

Form PCT/ISA/210 (continuation of second sheet) (January 2004)

## INTERNATIONAL SEARCH REPORT

| International application No. |
|---|
| PCT/IB2005/001647 |

**Box No. II    Observations where certain claims were found unsearchable (Continuation of item 2 of first sheet)**

This international search report has not been established in respect of certain claims under Article 17(2)(a) for the following reasons:

1. ☒ Claims Nos.: 3 2
   because they relate to subject matter not required to be searched by this Authority, namely:
   Claim 32 is relevant to methods for treatment of the human body by therapy and thus relates to a subject matter which this International Searching Authority is not required, under the provisions of Article 17(2)(a)(i) of the PCT and Rule 39.1(iv) of the Regulations under the PCT, to search.

2. ☐ Claims Nos.:
   because they relate to parts of the international application that do not comply with the prescribed requirements to such an extent that no meaningful international search can be carried out, specifically:

3. ☐ Claims Nos.:
   because they are dependent claims and are not drafted in accordance with the second and third sentences of Rule 6.4(a).

**Box No. III    Observations where unity of invention is lacking (Continuation of item 3 of first sheet)**

This International Searching Authority found multiple inventions in this international application, as follows:

1. ☐ As all required additional search fees were timely paid by the applicant, this international search report covers all searchable claims.

2. ☐ As all searchable claims could be searched without effort justifying an additional fee, this Authority did not invite payment of any additional fee.

3. ☐ As only some of the required additional search fees were timely paid by the applicant, this international search report covers only those claims for which fees were paid, specifically claims Nos.:

4. ☐ No required additional search fees were timely paid by the applicant.   Consequently, this international search report is restricted to the invention first mentioned in the claims; it is covered by claims Nos.:

**Remark on Protest**    ☐    The additional search fees were accompanied by the applicant's protest.

☐    No protest accompanied the payment of additional search fees.

Form PCT/ISA/210 (continuation of first sheet (2)) (January 2004)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 0149844 A
- WO 0136646 A

**Non-patent literature cited in the description**

- **GRIBSKOV ; BURGESS.** *Nucl. Acids Res.,* 1986
- Atlas of Polypeptide Sequence and Structure. National Biomedical Research Foundation, 1979, 353-358
- **WOOTTON ; FEDERHEN.** Analysis of compositionally biased regions in sequence databases. *Methods Enzymol.,* 1996, vol. 266, 554-71
- **SAMBROOK et al.** Molecular Cloning: A Laboratory Manual. Cold Spring Harbor Laboratory Press, 2001
- *BASIC METHODS IN MOLECULAR BIOLOGY,* 1986
- **SAMBROOK et al.** MOLECULAR CLONING; A LABORATORY MANUAL. Cold Spring Harbor Laboratory Press, 1989
- Saibo-Kogaku Handbook. Yodosha, 1992
- Shin-Idenshi-Kogaku Handbook. Yodosha, 1999
- Gene Ontology Consortium, ''Gene Ontology Consortium home page. *Gene Ontology Consortium,* 1999, http://www.geneontology.org
- **SCHNEIDER, I. et al.** *J. Embryol. Exp. Morph.,* 1972, vol. 27, 353-365